(19) Europäisches Patentamt
European Patent Office
Office européen des brevets

(11) EP 3 356 415 B1

(12) **EUROPEAN PATENT SPECIFICATION**

(45) Date of publication and mention of the grant of the patent:
**01.05.2024 Bulletin 2024/18**

(21) Application number: **16781931.7**

(22) Date of filing: **28.09.2016**

(51) International Patent Classification (IPC):
***C07K 16/28*** *(2006.01)* ***A61K 39/395*** *(2006.01)*
***A61P 9/00*** *(2006.01)* ***A61P 3/06*** *(2006.01)*
***C12N 15/113*** *(2010.01)*

(52) Cooperative Patent Classification (CPC):
**C07K 16/28; A61K 39/3955; A61P 9/04; A61P 9/10; C07H 21/02; C12N 15/1138;**
C07K 2317/30; C07K 2317/76; C07K 2317/77; C07K 2317/92; C07K 2317/94; C12N 2310/14

(86) International application number:
**PCT/US2016/054222**

(87) International publication number:
**WO 2017/058944 (06.04.2017 Gazette 2017/14)**

(54) **ASGR INHIBITORS FOR REDUZING CHOLESTEROL LEVELS**

ASGR-INHIBITOREN

INHIBITEURS DE L'ASGR POUR RÉDUIRE LES TAUX DE CHOLESTÉROL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.09.2015 US 201562234546 P**
**24.11.2015 US 201562259553 P**
**07.04.2016 US 201662319740 P**

(43) Date of publication of application:
**08.08.2018 Bulletin 2018/32**

(73) Proprietor: **Amgen Inc.**
**Thousand Oaks, California 91320 (US)**

(72) Inventors:
- **NIOI, Paul**
  **Wellesley, MA 02482 (US)**
- **COWARD, Peter**
  **San Francisco, CA 94118 (US)**
- **MURAWSKY, Christopher**
  **Roberts Creek, British Columbia V0N 2W0 (CA)**
- **PIPER, Derek, E.**
  **Santa Clara, CA 95051 (US)**
- **GARCES, Fernando**
  **San Mateo, CA 94401 (US)**
- **ZHANG, Jun**
  **Foster City, CA 94404 (US)**
- **LI, Yang**
  **Mountain View, CA 94040 (US)**
- **CHAN, Brian, Mingtung**
  **Port Coquitlam, British Columbia V3C 5N8 (CA)**

(74) Representative: **Dörries, Hans Ulrich**
**df-mp**
**Fünf Höfe**
**Theatinerstrasse 16**
**80333 München (DE)**

(56) References cited:
**WO-A1-2011/068798 WO-A1-2011/068798**
**WO-A1-2014/023709 WO-A1-2018/039647**
**WO-A1-2018/039647**

- **ANDERS WITTRUP ET AL: "Knocking down disease: a progress report on siRNA therapeutics", NATURE REVIEWS GENETICS, vol. 16, no. 9, 1 September 2015 (2015-09-01), pages 543-552, XP055681998, GB ISSN: 1471-0056, DOI: 10.1038/nrg3978**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- PAUL NIOI ET AL: "Variant ASGR1 Associated with a Reduced Risk of Coronary Artery Disease", THE NEW ENGLAND JOURNAL OF MEDICINE, - NEJM -, MASSACHUSETTS MEDICAL SOCIETY, US, vol. 374, 1 January 2016 (2016-01-01), pages 2131-2141, XP009192659, ISSN: 0028-4793, DOI: 10.1056/NEJMOA1508419
- Abin754108: "Asialoglycoprotein Receptor 1 antibody (ASGR1) (AA 250-290)" In: "Asialoglycoprotein Receptor 1 antibody (ASGR1) (AA 250-290)", 1 January 2014 (2014-01-01), XP055325933, the whole document
- RALUCA STEFANESCU ET AL: "Epitope Structure of the Carbohydrate Recognition Domain of Asialoglycoprotein Receptor to a Monoclonal Antibody Revealed by High-Resolution Proteolytic Excision Mass Spectrometry", JOURNAL OF THE AMERICAN SOCIETY FOR MASS SPECTROMETRY., vol. 22, no. 1, 1 January 2011 (2011-01-01), pages 148-157, XP055325761, US ISSN: 1044-0305, DOI: 10.1007/s13361-010-0010-y -& RALUCA STEFANESCU: "Epitope Structure of the Carbohydrate Recognition Domain of Asialoglycoprotein Receptor to a Monoclonal Antibody Revealed by High-Resolution Proteolytic Excision Mass Spectrometry", JOURNAL OF THE AMERICAN SOCIETY FOR MASS SPECTROMETRY JANUARY 2011, VOLUME 22, ISSUE 1, PP 148-157, vol. 22, no. 1, 1 January 2011 (2011-01-01), pages 1-6, XP055326603,
- Rita Born: "Benefit and Application of Antibodies Against the H1 Carbohydrate Recognition Domain of the Human Hepatic Asialoglycoprotein Receptor High Yield Recombinant Production of the H1 Carbohydrate Recognition Domain And Production and Characterization of Murine Monoclonal and Chicken Polyclonal Antibodies", Thesis, 1 September 2005 (2005-09-01), XP055326628, Retrieved from the Internet: URL:http://edoc.unibas.ch/536/1/DissB_7770 .pdf [retrieved on 2016-12-06]
- FU-YUE ZENG ET AL: "A Specific Antibody to the Carbohydrate Recognition Domain of the Asialoglycoprotein Receptor RHL1 Subunit Does Not React with RHL2/3 but Blocks Ligand Binding", BIOCHEMICAL AND BIOPHYSICAL RESEARCH COMMUNICATIONS, vol. 249, no. 1, 1 August 1998 (1998-08-01), pages 236-240, XP055326018, AMSTERDAM, NL ISSN: 0006-291X, DOI: 10.1006/bbrc.1998.9120
- JUNG-HYUN PARK ET AL: "Detection of surface asialoglycoprotein receptor expression in hepatic and extra-hepatic cells using a novel monoclonal antibody", BIOTECHNOLOGY LETTERS, vol. 28, no. 14, 24 June 2006 (2006-06-24) , pages 1061-1069, XP055326059, NL ISSN: 0141-5492, DOI: 10.1007/s10529-006-9064-0
- LIMIN CAO ET AL: "Characterization of a single-chain variable fragment (scFv) antibody directed against the human asialoglycoprotein receptor", BIOTECHNOLOGY AND APPLIED BIOCHEMISTRY, ACADEMIC PRESS, US, vol. 44, no. 2, 1 May 2006 (2006-05-01), pages 65-72, XP002643344, ISSN: 0885-4513, DOI: 10.1042/BA20050081 [retrieved on 2010-12-23]
- ALLA TRAHTENHERTS ET AL: "An Internalizing Antibody Specific for the Human Asialoglycoprotein Receptor", HYBRIDOMA, vol. 28, no. 4, 1 August 2009 (2009-08-01) , pages 225-233, XP055084704, ISSN: 1554-0014, DOI: 10.1089/hyb.2009.0019
- Wei-Yu Wang ET AL: "Screening of specific single-chain variable-fragment (scFv) antibody against human asialoglycoprotein receptor by capture phage enzyme-linked immunosorbent assay", African Journal of Biotechnology, 7 March 2011 (2011-03-07), pages 1919-1925, XP055323145, Victoria Island DOI: 10.5897/AJB10.1716 Retrieved from the Internet: URL:http://www.academicjournals.org/journal/AJB/article-full-text-pdf/6C33AB930362
- YUTAKA KOHGO ET AL: "Production and Characterization of Specific Asialoglycoprotein Receptor Antibodies", HYBRIDOMA, vol. 12, no. 5, 1 October 1993 (1993-10-01), pages 591-598, XP055326047, US ISSN: 0272-457X, DOI: 10.1089/hyb.1993.12.591
- M SHIMADA: "A monoclonal antibody to rat asialoglycoprotein receptor that recognizes an epitope specific to its major subunit", HEPATOLOGY RESEARCH, vol. 26, no. 1, 1 May 2003 (2003-05-01), pages 55-60, XP055325823, NL ISSN: 1386-6346, DOI: 10.1016/S1386-6346(02)00329-7
- ILARIA MASSARELLI ET AL: "Three-Dimensional Models of the Oligomeric Human Asialoglycoprotein Receptor (ASGP-R)", INTERNATIONAL JOURNAL OF MOLECULAR SCIENCES, vol. 11, no. 10, 11 October 2010 (2010-10-11), pages 3867-3884, XP055326753, DOI: 10.3390/ijms11103867
- ANDERS WITTRUP ET AL: "Knocking down disease: a progress report on siRNA therapeutics", NATURE REVIEWS GENETICS, vol. 16, no. 9, 1 September 2015 (2015-09-01), pages 543-552, XP055681998, GB ISSN: 1471-0056, DOI: 10.1038/nrg3978


- PAUL NIOI ET AL: "Variant ASGR1 Associated with a Reduced Risk of Coronary Artery Disease", THE NEW ENGLAND JOURNAL OF MEDICINE, - NEJM -, MASSACHUSETTS MEDICAL SOCIETY, US, vol. 374, 1 January 2016 (2016-01-01), pages 2131-2141, XP009192659, ISSN: 0028-4793, DOI: 10.1056/NEJMOA1508419


Remarks:

The complete document including Reference Table(s) and the Sequence Listing(s) can be downloaded from the EPO website

## Description

CROSS REFERENCE TO RELATED APPLICATIONS

This application claims priority to U.S. Provisional Patent Application No. 62/319,740, filed April 7, 2016, U.S. Provisional Patent Application No. 62/259,553, filed November 24, 2015, and U.S. Provisional Patent Application No. 62/234,546, filed September 29, 2015.

REFERENCE TO THE SEQUENCE LISTING AND TABLES IN ELECTRONIC FORMAT

This application contains a Sequence Listing which has been submitted electronically in ASCII format. Said ASCII copy, created on September 28, 2016, is named APMOL017WOSEQUENCE.txt and is 14,772,812 bytes in size.

FIELD

The field of this invention relates to the use of interfering RNA of asialoglycoprotein receptor 1 (ASGR-1) and/or asialoglycoprotein receptor 2 (ASGR-2) for treating or preventing a cardiovascular disease in a patient.

BACKGROUND OF VARIOUS EMBODIMENTS

Cardiovascular disease involving the heart or blood vessels remains a leading cause of global mortality. Cardiovascular disease includes coronary artery disease (CAD) which can lead to angina and myocardial infarction (MI), stroke, hypertensive heart disease, rheumatic heart disease, and other disorders of the cardiovascular system. Medicines for treating cardiovascular disease, and in particular coronary artery disease, have been introduced over the years (e.g., the small molecule class of drugs called statins and the recently approved Repatha®, an antibody targeting PCSK9).

SUMMARY OF VARIOUS EMBODIMENTS

The invention is set out in the appended set of claims. The embodiments and/or examples of the following description which are not covered by the appended claims are considered as not being part of the present invention. Any references in the description to methods of treatment refer to the compounds, pharmaceutical compositions and medicaments of the present invention for use in a method for treatment of the human or animal body by therapy or for diagnosis. The present invention provides interfering RNA of asialoglycoprotein receptor 1 (ASGR-1), and/or asialoglycoprotein receptor 2 (ASGR-2) for use for treating or preventing a cardiovascular disease in a patient. In some embodiments, the interfering RNA is siRNA or shRNA.
In some embodiments, the interfering RNA comprises a double-stranded RNA molecule which comprises two antiparallel strands of contiguous nucleotides that are sufficiently complementary to each other to hybridize to form a duplex region. In other embodiments, the interfering RNA is a double-stranded RNA molecule that comprises a sense strand and an antisense strand, wherein the sense strand comprises a sequence that is sufficiently complementary to the sequence of the antisense strand such that the two strands hybridize under physiological conditions to form a duplex region of about 15 to about 30 base pairs, or about 19 to about 21 base pairs in length. In further embodiments, the interfering RNA is an siRNA formed from two separate strands of a sense strand and an antisense strand that hybridize to form a duplex region but are otherwise unconnected.
In some embodiments, the cardiovascular disease is coronary artery disease or myocardial infarction. In other embodiments, the interfering RNA reduces blood LDL cholesterol level of the patient by at least 15% as compared to a predose level of blood LDL cholesterol in the patient. In further embodiments, the interfering RNA reduces non-HDL cholesterol level of the patient by at least 5% as compared to a predose level of non-HDL cholesterol in the patient. In other embodiments, the interfering RNA increases ALP level of the patient by at least 30% as compared to a predose ALP level in the patient.
The interfering RNA may be a double-stranded RNA that comprises one or more modified nucleotides. Additionally or alternatively, the interfering RNA may be coupled to a targeting moiety. In some preferred embodiments, the interfering RNA is specifically targeted to liver cells using the surface expressed ASGR-1 and/or ASGR-2, and expression of ASGR-1 and/or ASGR-2 is reduced in the liver cells due to the effect of the targeted interfering RNA.
In some embodiments, the interfering RNA is administered in combination with one or more other therapeutic agents useful for treating or preventing cardiovascular disease, wherein the interfering RNA is administered simultaneously, prior to, concurrent with, or subsequent to administering the one or more other therapeutic agents. In some preferred embodiments, the one or more other therapeutic agents is a statin, an anti-PCSK9 antibody or a combination thereof.
In some embodiments, the interfering RNA is an siRNA targeting ASGR-1, wherein the siRNA comprises a sense strand

and an antisense strand, wherein the antisense strand comprises a region having a sequence that is substantially or fully complementary to an ASGR-1 mRNA sequence, and wherein the sense strand comprises a sequence that is sufficiently complementary to the sequence of the antisense strand such that the two strands hybridize under physiological conditions to form a duplex region that is about 15 to about 30 base pairs in length. In some of these embodiments, the cardiovascular disease is coronary artery disease or myocardial infarction.
In some embodiments, the interfering RNA is a double-stranded RNA molecule comprising a sense strand and an antisense strand that is 15 to 30 nucleotides in length, and comprises a region having a sequence that is fully complementary to an ASGR-1 mRNA sequence and/or an ASGR-2 mRNA sequence.
In some embodiments, the interfering RNA is siRNA formed from two separate strands of a sense strand and an antisense strand that hybridize to form a duplex region but are otherwise unconnected, wherein the siRNA comprises a duplex region of about 19 to about 25 base pairs in length: or the siRNA comprises a sense strand of about 15-30 nucleotides and an antisense strand of about 15-30 nucleotides: or the siRNA comprises a sense strand of about 19-25 nucleotides and an antisense strand of about 19-25 nucleotides.
In some embodiments, the interfering RNA is an inhibitor of ASGR-1. In other embodiments, the interfering RNA is an inhibitor of ASGR-2. In some embodiments, the interfering RNA is a double- stranded RNA molecule that comprises a sense strand and an antisense strand, wherein the antisense strand comprises a region having a sequence that is substantially or fully complementary to an ASGR- 1 mRNA sequence encoding a human ASGR-1 protein. In other embodiments, the interfering RNA is a double-stranded RNA molecule that comprises a sense strand and an antisense strand, wherein the antisense strand comprises a region having a sequence that is substantially or fully complementary to an ASGR-2 mRNA sequence encoding a human ASGR-2 protein.

BRIEF DESCRIPTION OF THE FIGURES

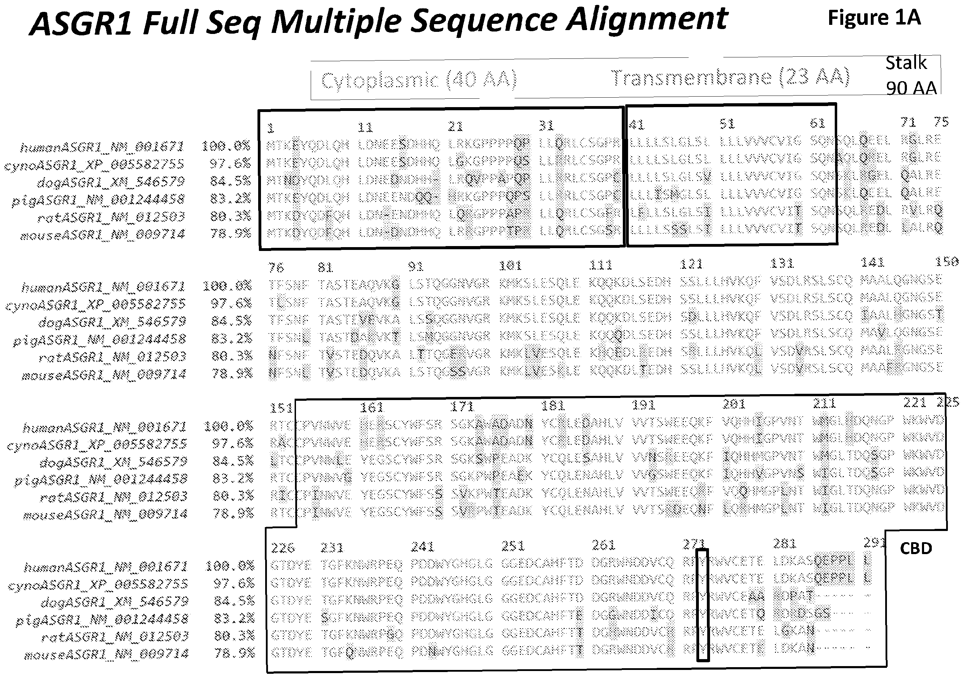

**Figure 1A.** ASGR-1 sequence alignments of human (SEQ ID NO: 32699), cynomolgus monkey (cyno) (SEQ ID NO: 32700), dog (SEQ ID NO: 32701), pig (SEQ ID NO: 32702), rat (SEQ ID NO: 32703) and mouse ASGR-1 (SEQ ID NO: 32704). The boxed areas denoting different regions of ASGR-1 (i.e., cytoplasmic, transmembrane, and the carbohydrate binding domain (CBD; also called the carbohydrate recognition domain, or CRD) are representative of the approximate amino acid locations of these regions; the human Y273 amino acid is boxed.

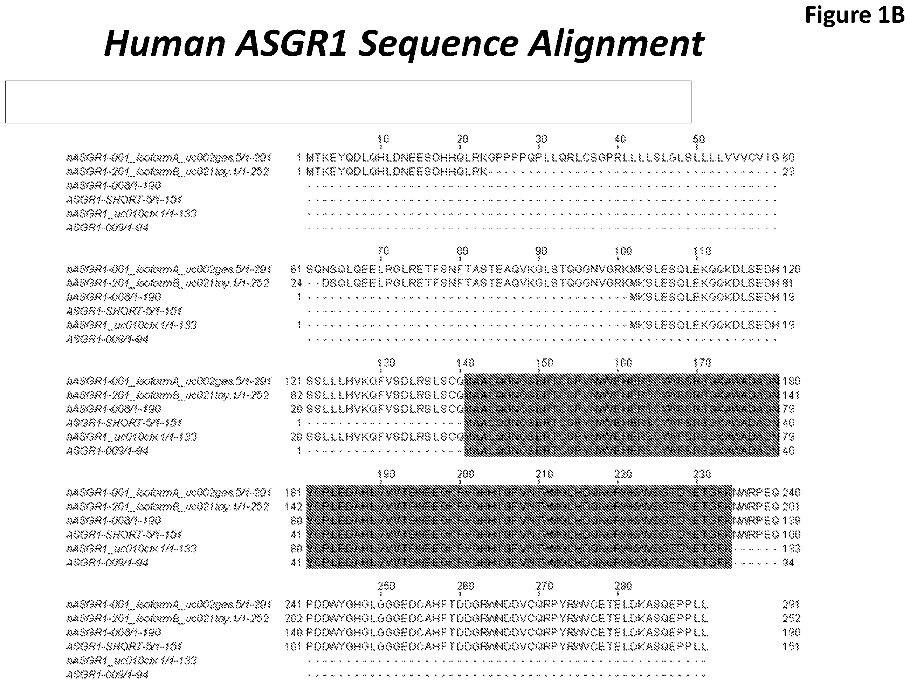

**Figure 1B.** Human ASGR-1 sequence alignments (SEQ ID NOS 32705-32710, respectively, in order of appearance).

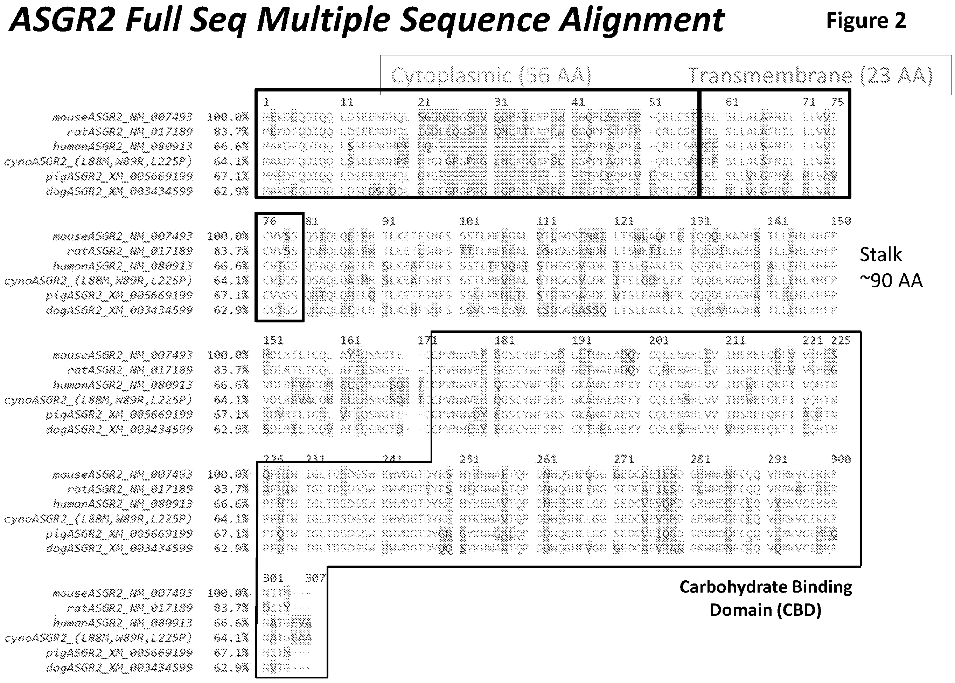

**Figure 2.** ASGR-2 sequence alignments of human (SEQ ID NO: 32713), cyno (SEQ ID NO: 32714), dog (SEQ ID NO: 32716), pig (SEQ ID NO: 32715), rat (SEQ ID NO: 32712) and mouse ASGR-2 (SEQ ID NO: 32711). The boxed areas denoting different regions of ASGR-2 (i.e., cytoplasmic, transmembrane, and the carbohydrate binding domain (CBD; also called the carbohydrate recognition domain, or CRD) are representative of the approximate amino acid locations of these regions.

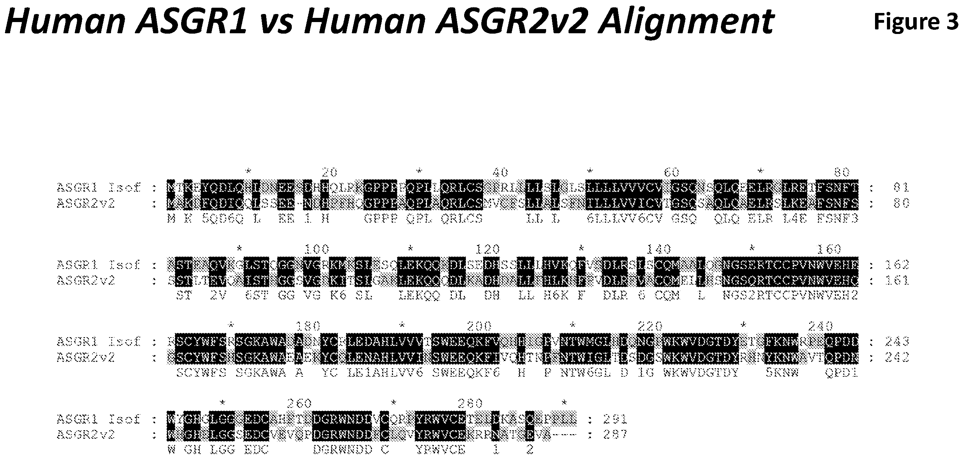

**Figure 3.** Human ASGR-1 (SEQ ID NO: 32717) vs. human ASGR-2v2 (SEQ ID NO: 32718) alignments are provided.

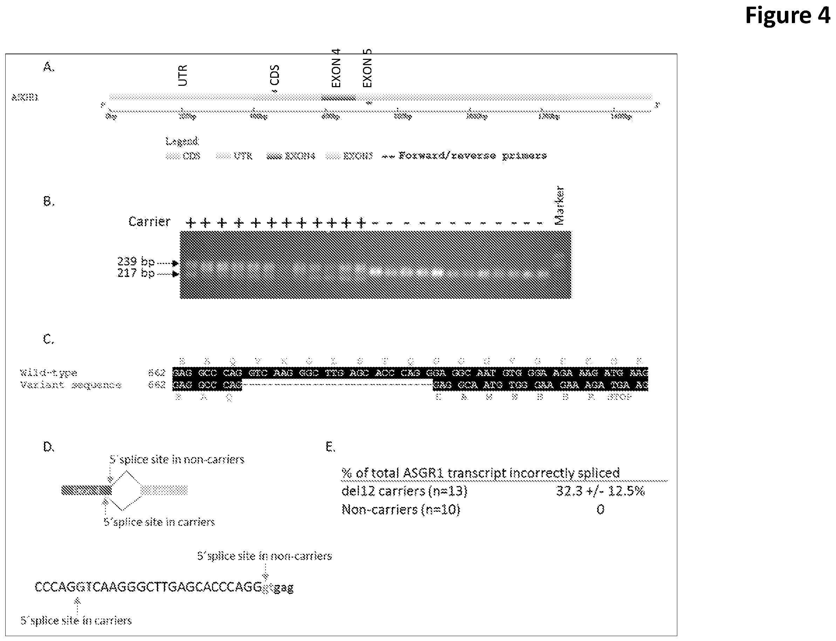

**Figure 4.** The del12 variant is associated with a splicing error and frameshift in ASGR-1. (A) Overview of the structure of the ASGR-1 mRNA. Exons 4 and 5 are highlighted (the del12 variant lies within intron 4 between exons 4 and 5 in the unspliced RNA) along with the positions of the PCR primers (red arrows) used to amplify the cDNA. (B) Agarose gel showing the PCR products generated by amplifying cDNA generated from RNA isolated from the blood of del12 carriers and non-carriers. Arrows indicate both the size of the expected PCR product (239 bp) along with the size of the truncated band (217 bp) observed only in del12 heterozygote carriers. (C) Shown is the sequence difference between the full-length (239 bp) and variant (217 bp) cDNA fragments based on Sanger sequencing. The variant sequence in del12 carriers lacks 22 bp at the end of exon 4 compared to the wild-type sequence that results in frame-shift and introduction of a stop codon. (D) Diagrammatic representation of the splicing defect observed in del12 carriers. The sequence around the exon 4-intron 4 boundary (exon 4 sequence in capital letters and intron 4 sequence in small letters) is shown along with the 5'splice site in non-carriers and the cryptic 5' splice site activated in del12 carriers. (E) Quantification of the full-length (239 bp) and variant (217 bp) cDNA fragments from heterozygote del12 carriers and non-carriers by direct digital counting of sequencing reads generated following sequencing of the amplified cDNA product from carriers and non-carriers of del12 using the Illumina TruSeq method. The percentage of incorrectly spliced ASGR-1 transcript is shown. Note that the incorrectly spliced form was completely undetectable in non-carriers.

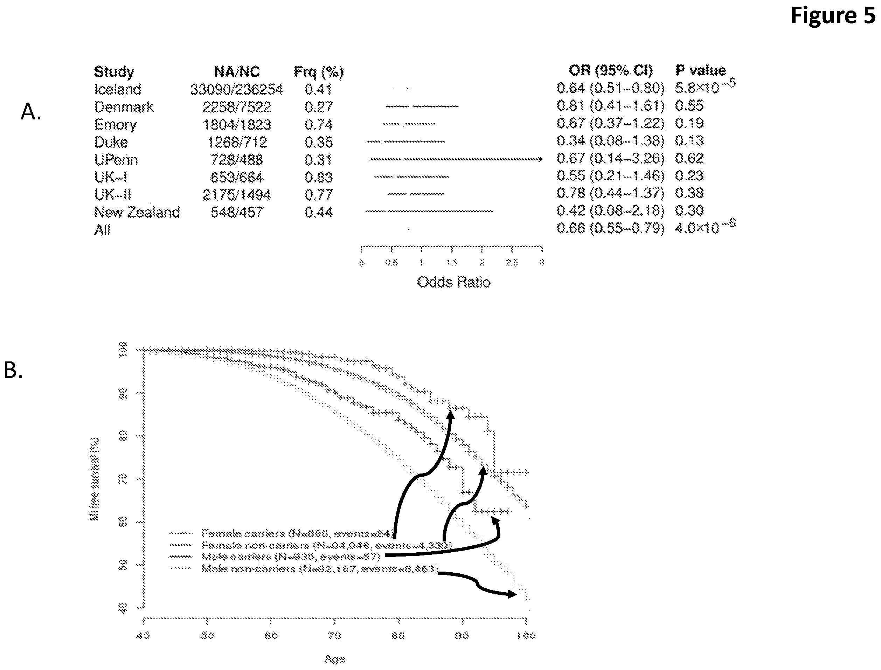

**Figure 5.**(A) The del12 variant was typed in the indicated populations a total of 41,648 CAD cases and 247,374 controls. For each cohort, the square (diamond in the case of the combined estimate) indicates the estimated odds ratio and the line shows the 95% confidence interval. There was no evidence of heterogeneity across the eight study populations (Phet = 0.96). (B) Kaplan- Meier curves for survival to first myocardial infarction in carriers and non-carriers of del12 in ASGR- 1 stratified by sex. The proportion of individuals that have not had a myocardial infarction is shown on the y-axis and plotted against age on the x-axis. Males and females are represented separately and a distinction is made between del12 carriers and non-carriers in each case.

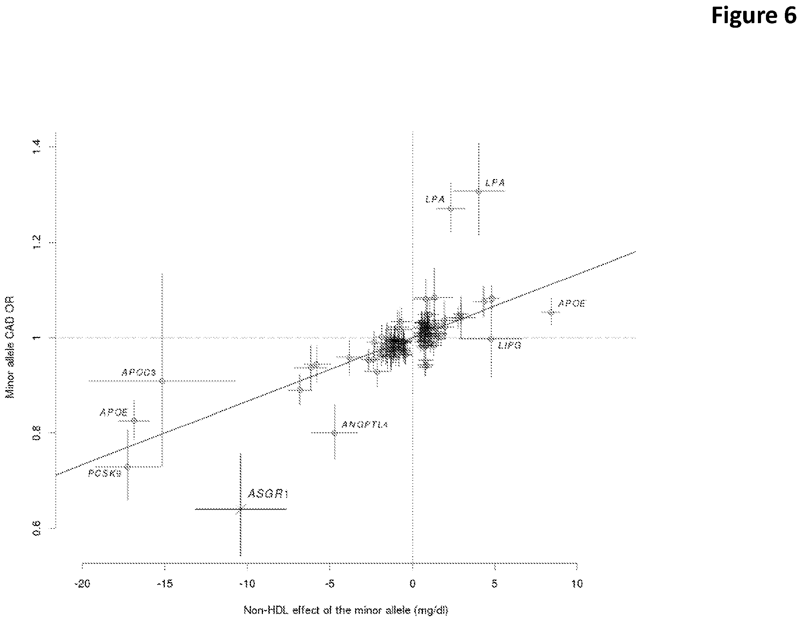

**Figure 6.** Comparison of relationship between CAD and non-HDL cholesterol levels between previously identified sequence variants and del12 in ASGR-1. Based on the Icelandic population, the estimated odds ratio (OR) of the minor allele for coronary artery disease (CAD, 41,648 cases and 247,374 controls) as a function of the estimated effect of the minor allele on non-HDL cholesterol levels (N=119,146). A full list of the sequence variants included is provided in Table 1.7. The error bars represent 95% confidence intervals. The del12 variant in ASGR-1 is shown. The line indicates the best linear regression fit through the origin.

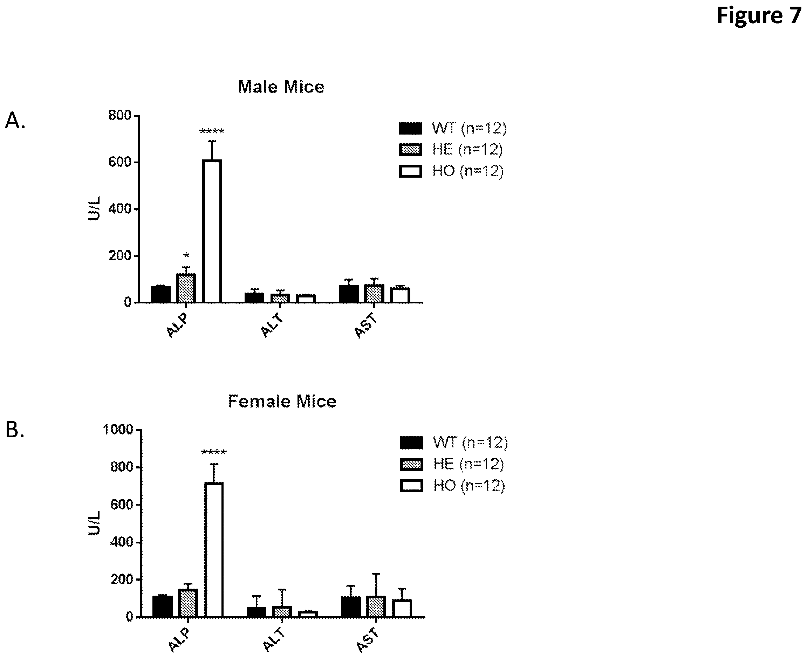

**Figure 7.** Analysis of serum ALP, ALT, and AST from ASGR-1 knockout mice is provided. Panel A is data from the male mice studied and Panel B is data from the female mice.

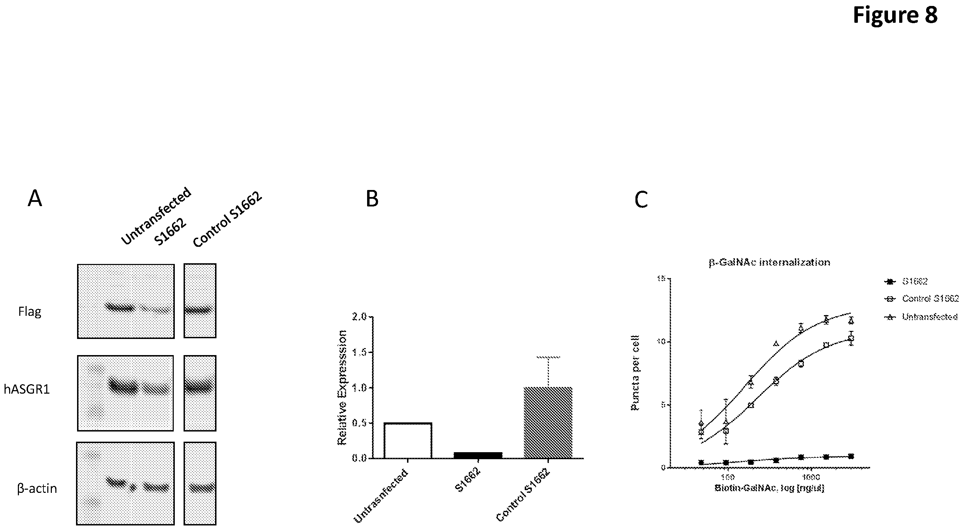

**Figure 8.** RNAi *in vitro* data in CHO cells transfected with hASGR-1 using construct S1662. Panel A is a western blot demonstrating reduction of expression of human ASGR- 1. Panel B is a graphical representation of the relative reduction in expression of human ASGR-1. Panel C demonstrates that CHO cells receiving construct S1662 displays a dramatic reduction in internalization of ligand (β-GalNAc).

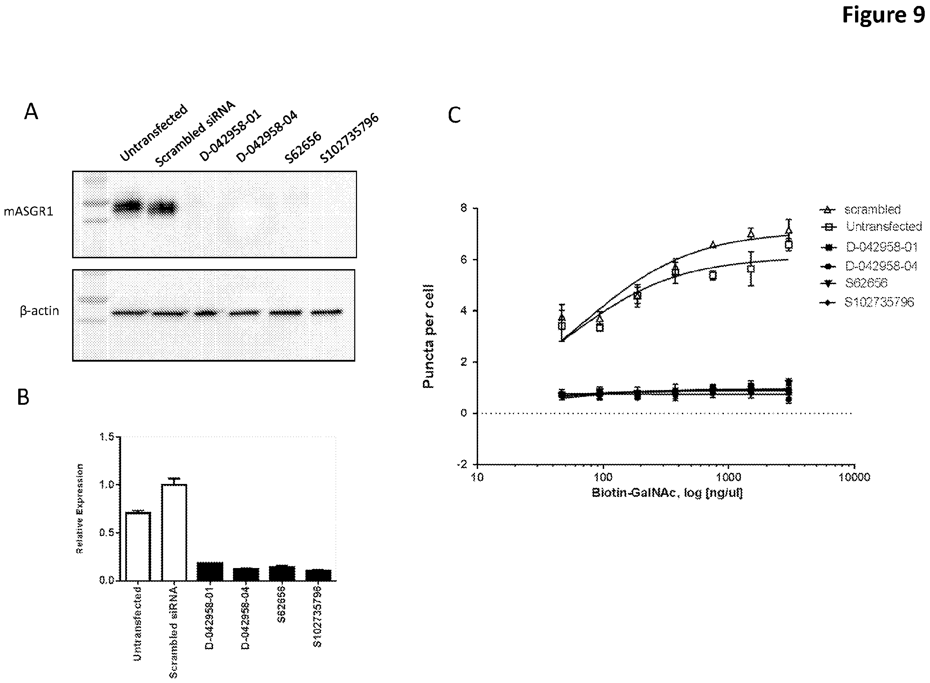

**Figure 9.** RNAi *in vitro* data in CHO cells transfected with mASGR-1 using various constructs. Panel A is a western blot demonstrating reduction of expression of mouse ASGR- 1. Panel B is a graphical representation of the relative reduction in expression of mouse ASGR-1. Panel C demonstrates that CHO cells receiving the various constructs display a dramatic reduction in internalization of ligand (β-GalNAc).

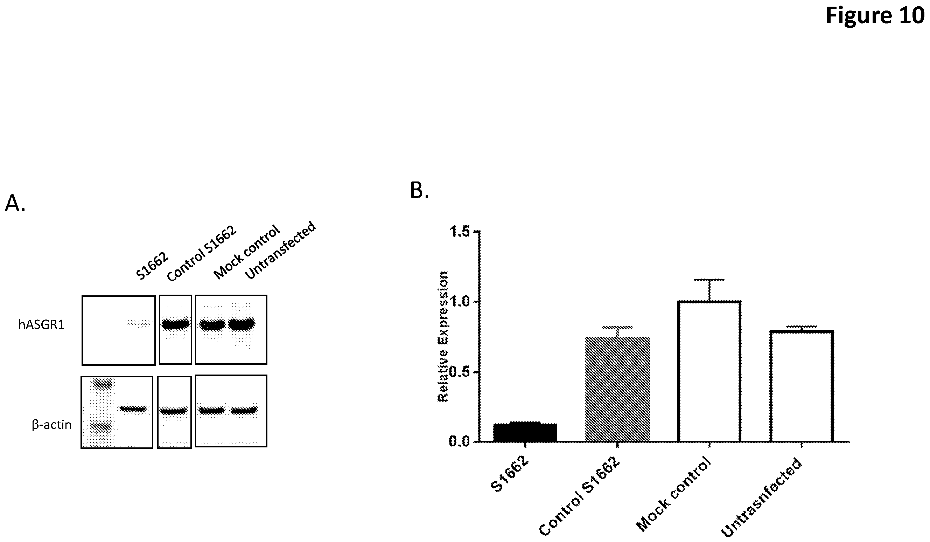

**Figure 10.** RNAi *in vitro* data in HepG2 cells using construct S1662. Panel A is a western blot demonstrating reduction of expression of human ASGR-1. Panel B is a graphical representation of the relative reduction in expression of human ASGR-1.

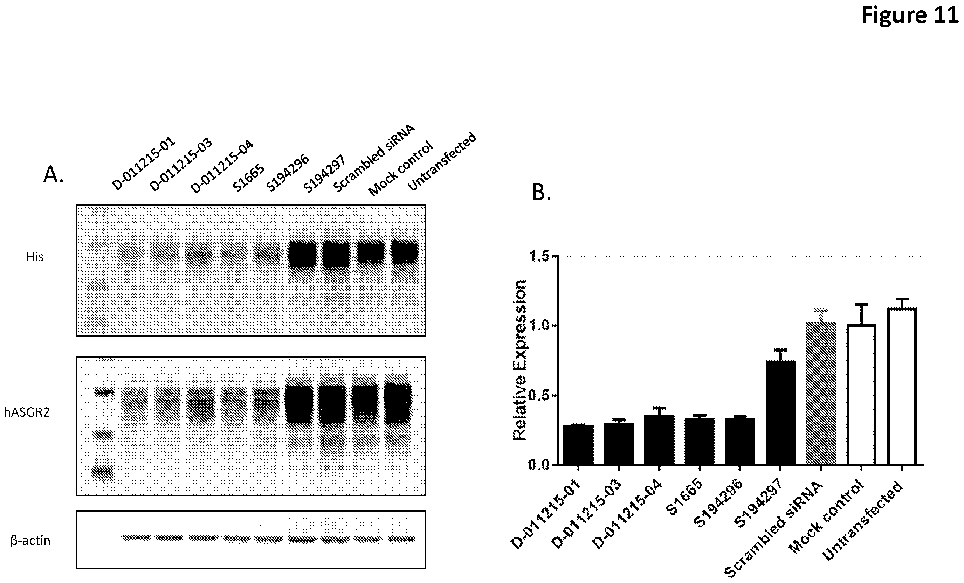

**Figure 11.** RNAi *in vitro* data in CHO cells transfected with hASGR-2 using various constructs. Panel A is a western blot demonstrating reduction of expression of human ASGR- 2. Panel B is a graphical representation of the relative reduction in expression of human ASGR-2 by the various constructs.

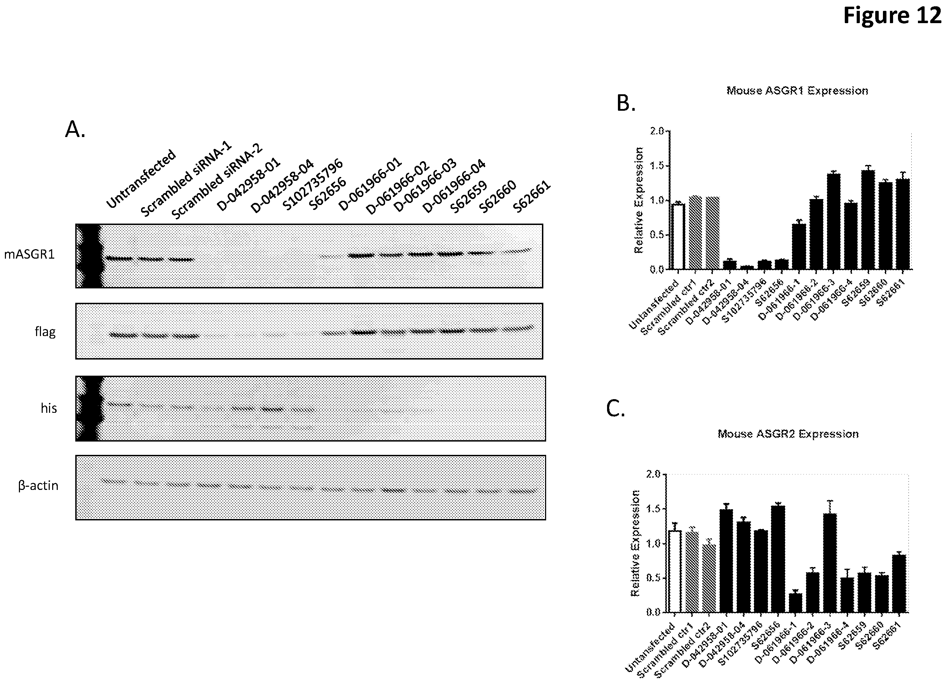

**Figure 12.** RNAi *in vitro* data in CHO cells transfected with mASGR-1 and mASGR-2 using various other constructs. Panel A is a western blot demonstrating reduction of expression of mouse ASGR-1 (anti-mouse ASGR-1 or anti-flag) or mouse ASGR-2 (anti-his). Panel B is a graphical representation of the relative reduction in expression of mouse ASGR-1 by the various constructs. Panel C is a graphical representation of the relative reduction in expression of mouse ASGR-2 by the various constructs.

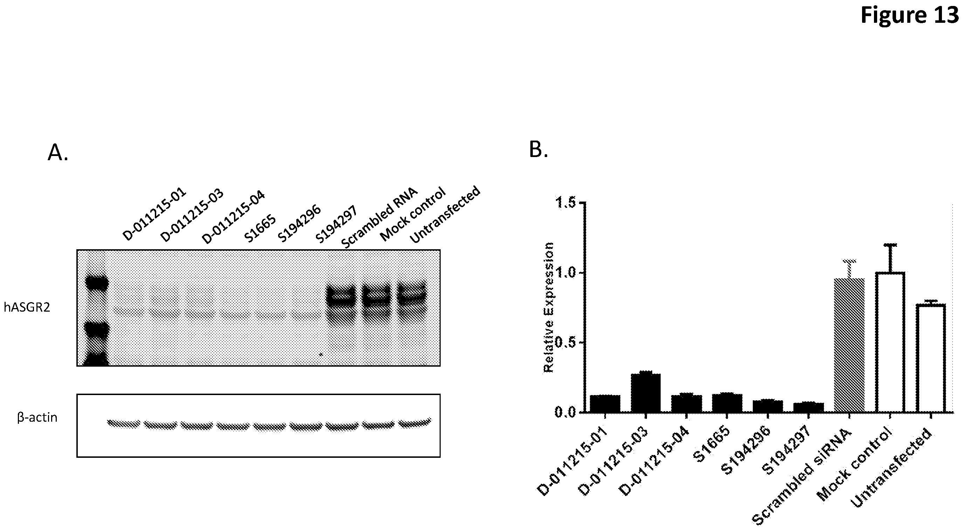

**Figure 13.** RNAi *in vitro* data in HepG2 cells using various constructs. Panel A is a western blot demonstrating reduction of expression of human ASGR-2. Panel B is a graphical representation of the relative reduction in expression of human ASGR-2 by the various constructs.

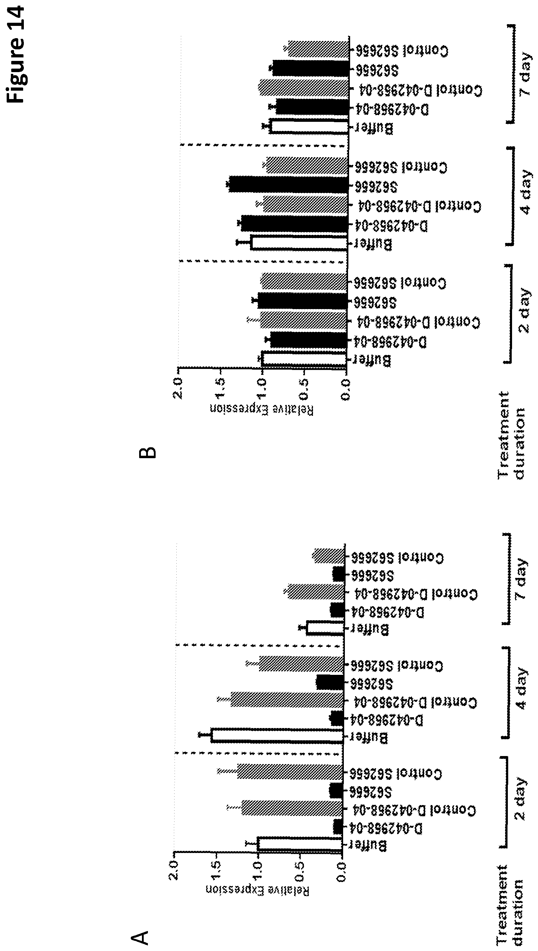

**Figure 14.** RNAi *in vivo* data in in C57BL/6J mice using various constructs over the course of 7 days with three injections total, one injection at day 0, one injection at day 2 and one injection at day 4. Panel A is a graphical representation of quantitative per data showing the relative reduction in expression of mASGR-1 RNA in the liver. Panel B is a graphical representation of the relative reduction in expression of mASGR-2 RNA in the liver.

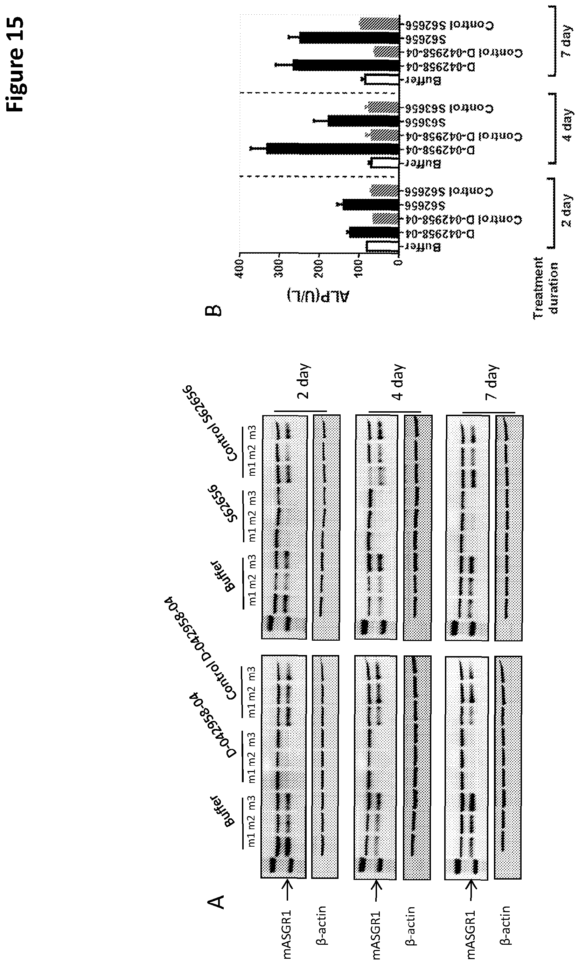

**Figure 15.** RNAi *in vivo* data in in C57BL/6J mice using various constructs over the course of 7 days with three injections total, one injection at day 0, one injection at day 2, and one injection at day 4. Panel A is a western blot demonstrating reduction of expression of mouse ASGR- 1 protein. Panel B is a graphical representation of the relative increase of serum ALP activity.

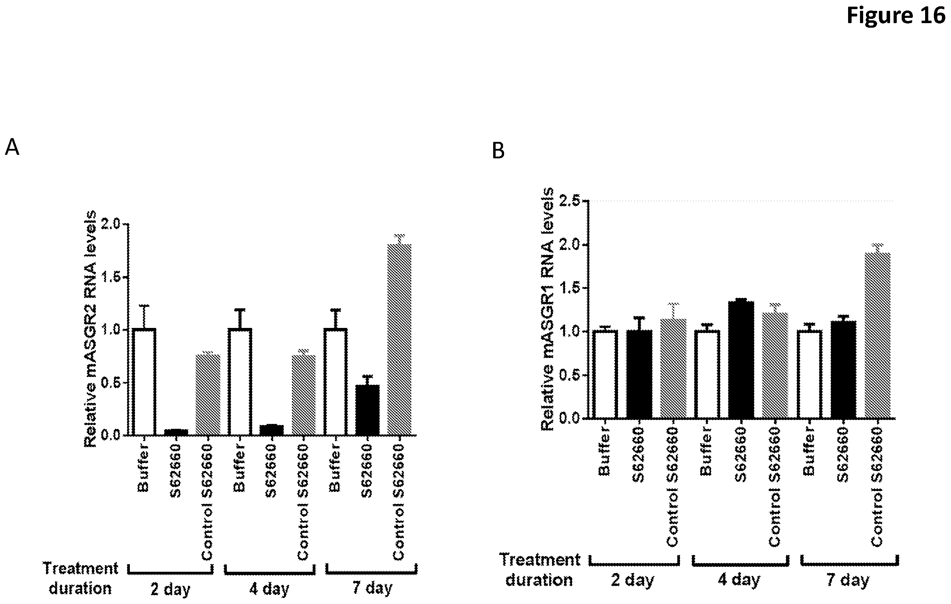

**Figure 16.** RNAi *in vivo* data in C57BL/6J mice using various constructs over the course of 7 days with one injection at day 0. Panel A is a graphical representation of the relative reduction in expression of mASGR-2 in the liver. Panel B is a graphical representation of the relative reduction in expression of mASGR-1 in the liver.

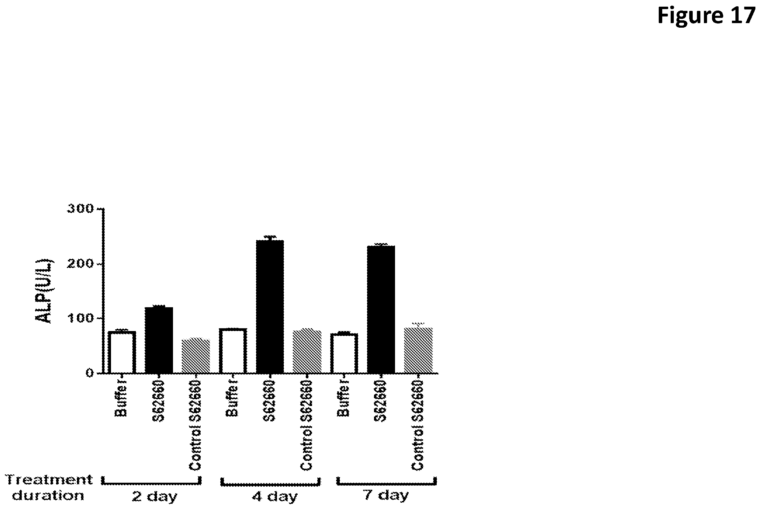

**Figure 17.** RNAi *in vivo* data in C57BL/6J mice using various ASGR-2 constructs over the course of 7 days with one injection at day 0. The figure is a graphical representation of the relative increase in serum ALP activity.

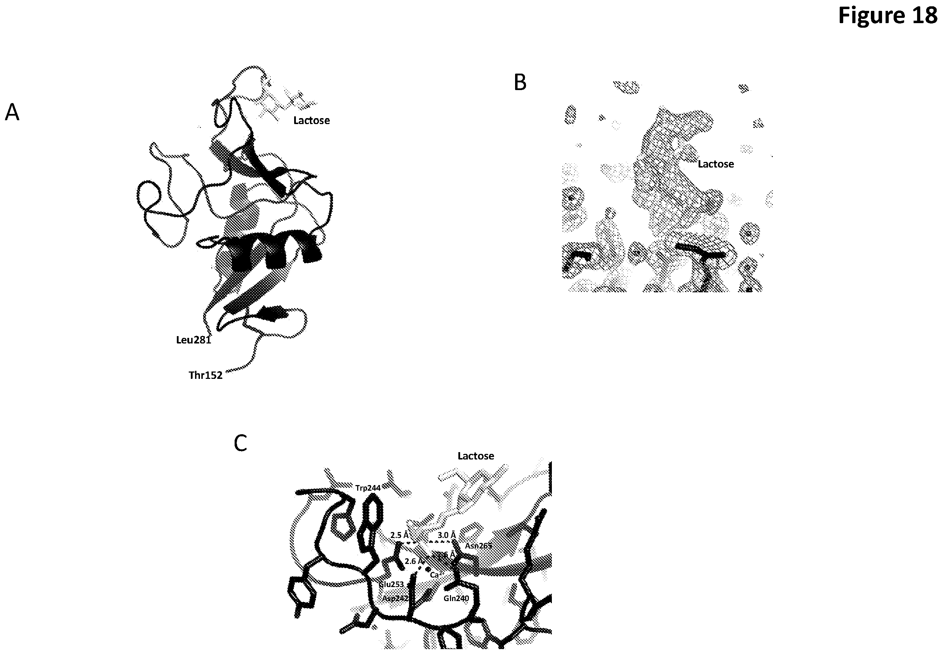

**Figure 18.** Panel A shows a computer representation of the crystal structure of the ASGR-1/lactose complex. Panel B is a computer representation of the observed electron density. Panel C is an enlarged view of the carbohydrate binding domain.

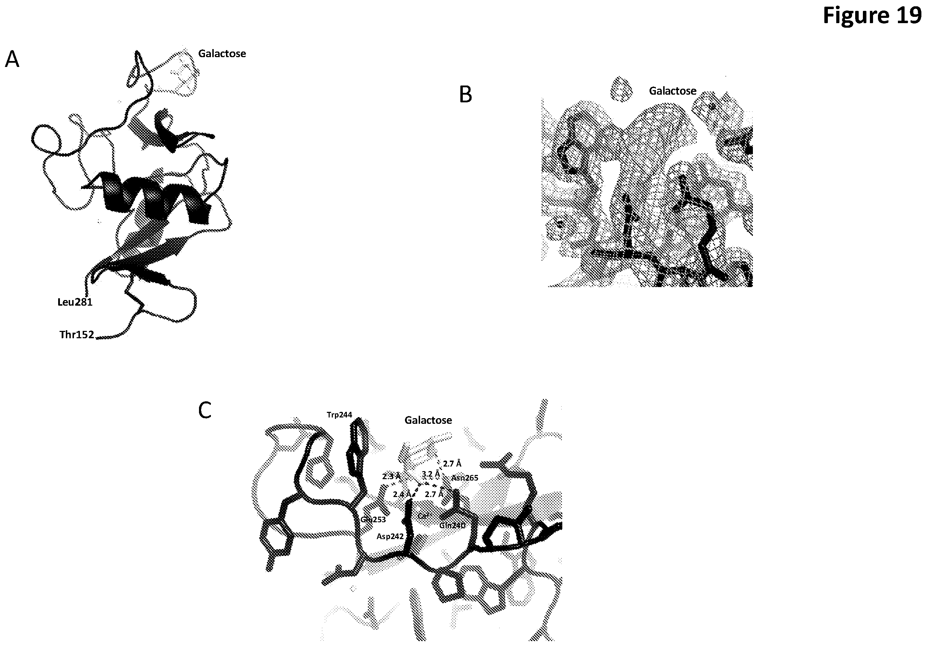

**Figure 19.** Panel A shows a computer representation of the crystal structure of the ASGR-1/galactose complex. Panel B is a computer representation of the observed electron density. Panel C is an enlarged view of the carbohydrate binding domain.

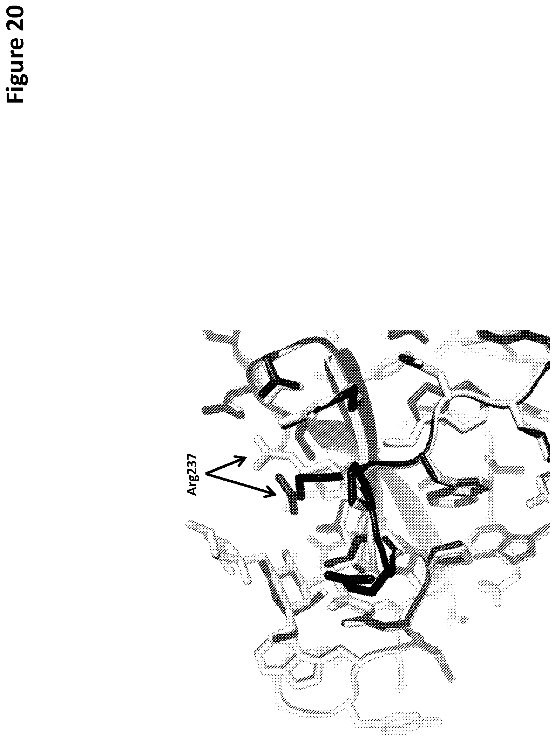

**Figure 20.** A computer representation of the crystal structure of an enlarged view of the conformational difference of R237 between the ASGR-1/lactose (white) complex and ASGR- 1/galactose (black) complex.

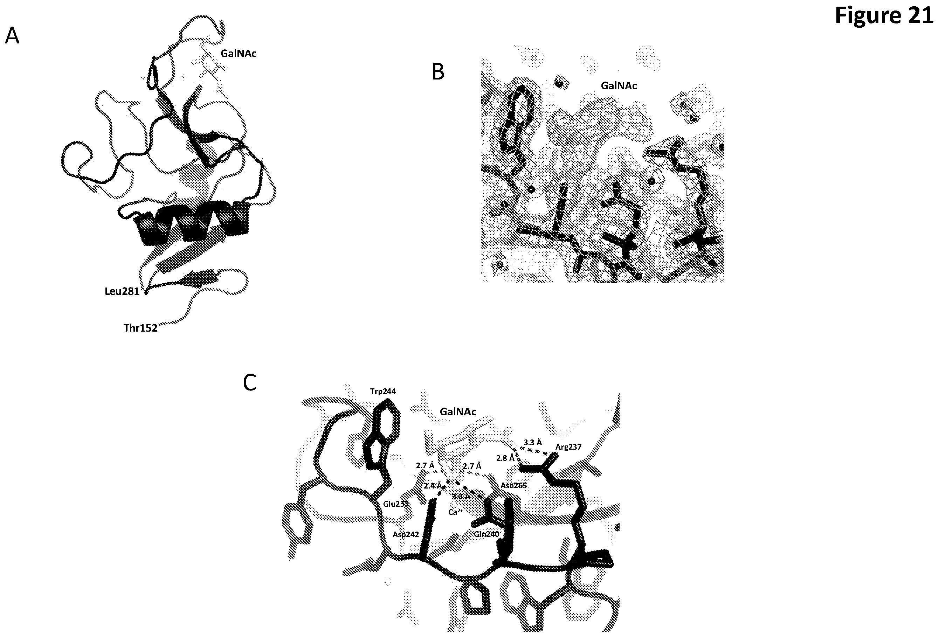

**Figure 21.** Panel A shows a computer representation of the crystal structure of the ASGR-1/GalNAc complex. Panel B is a computer representation of the observed electron density. Panel C is an enlarged view of the carbohydrate binding domain.

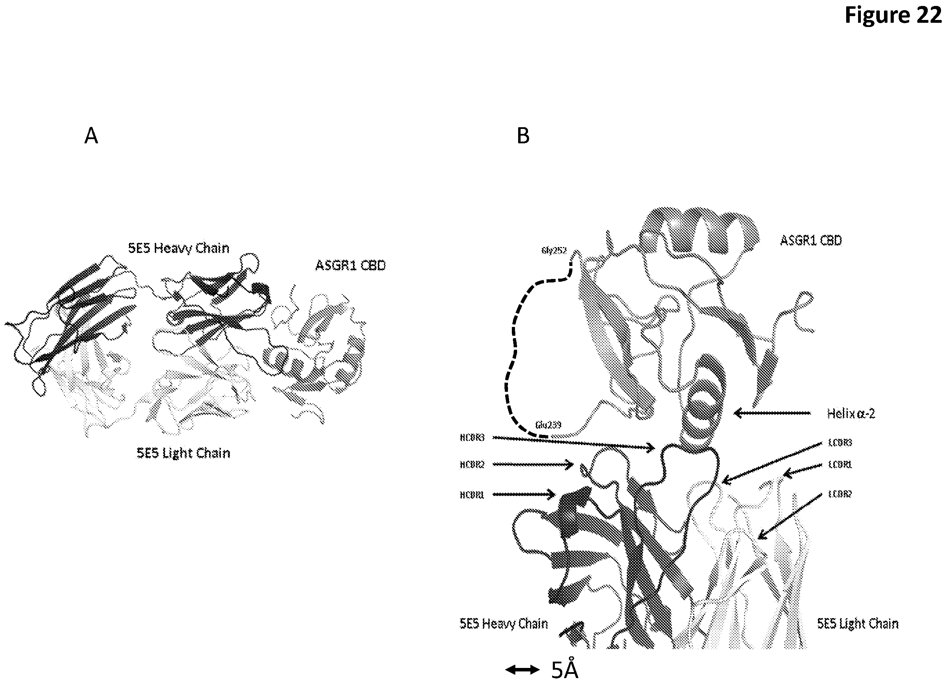

**Figure 22.** Panel A shows a depiction of the structure of the ASGR-1 CBD and the 5E5 Fab. Panel B is an enlarged view of the ASGR-1 CBD and 5E5 Fab that represents a disordered carbohydrate binding loop with a dashed line and highlights the indirect inhibition of ASGR-1 CBD and the ligand (GalNAc) binding. Panel B also incorporates a double-headed arrow which represents a 5 angstrom distance from tip to tip.

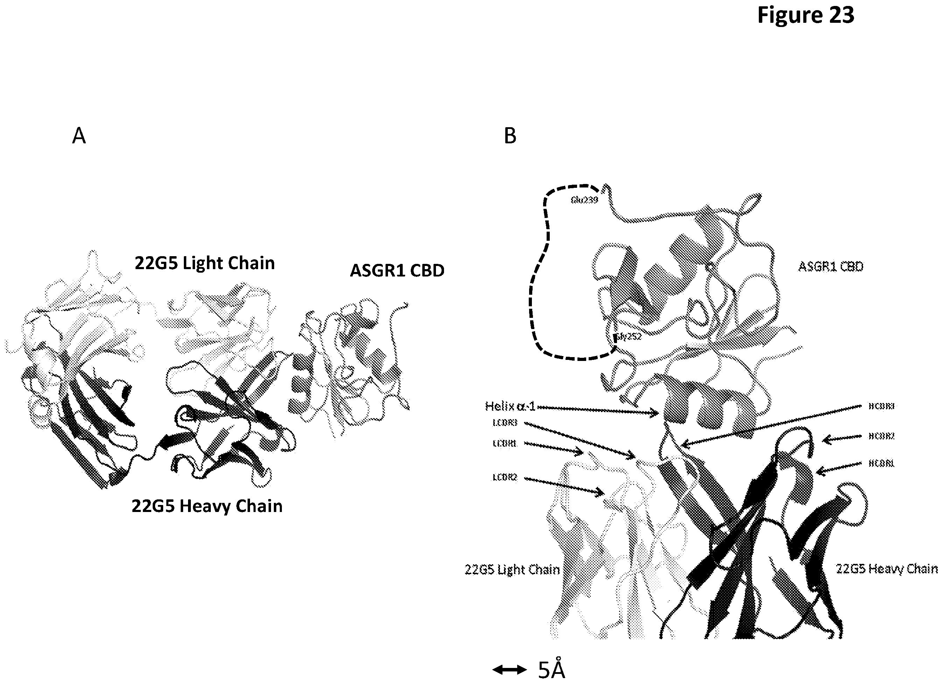

**Figure 23.** Panel A shows a depiction of the structure of the ASGR-1 CB and the 22G5 Fab. Panel B is an enlarged view of the ASGR-1 CBD and 22G5 Fab that represents a disordered carbohydrate binding loop with a dashed line and highlights the indirect inhibition of ASGR-1 CBD and the ligand (GalNAc) binding. Panel B also incorporates a double-headed arrow which represents a 5 angstrom distance from tip to tip.

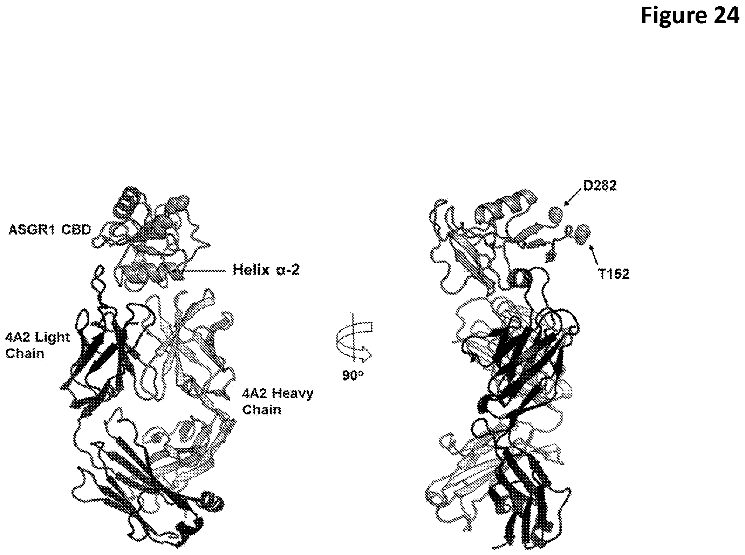

**Figure 24.** A depiction of the structure of the ASGR-1 CBD and the 4A2 Fab.

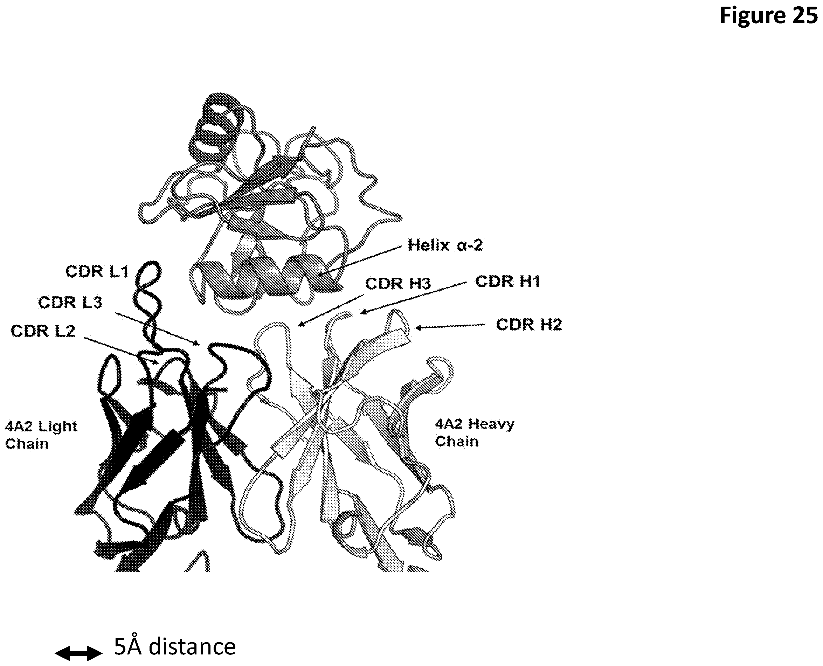

**Figure 25.** An enlarged view of the structure of the ASGR-1 CBD and the 4A2 Fab that shows the CDRs of the 4A2 Fab that interact with ASGR-1 CBD Helix alpha-2 and highlights the indirect inhibition of ASGR-1 CBD and the ligand (GalNAc) binding. The figure incorporates a double-headed arrow which represents a 5 angstrom distance from tip to tip.

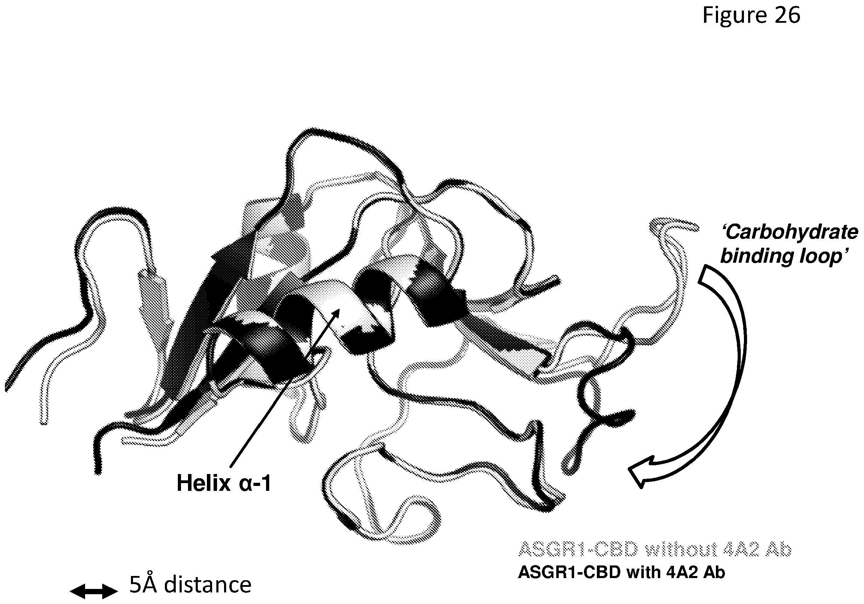

**Figure 26.** An enlarged view of the structure of the ASGR-1 CBD and the carbohydrate binding loop with and without and the 4A2 Fab that includes a double-headed arrow which represents a 5 angstrom distance from tip to tip.

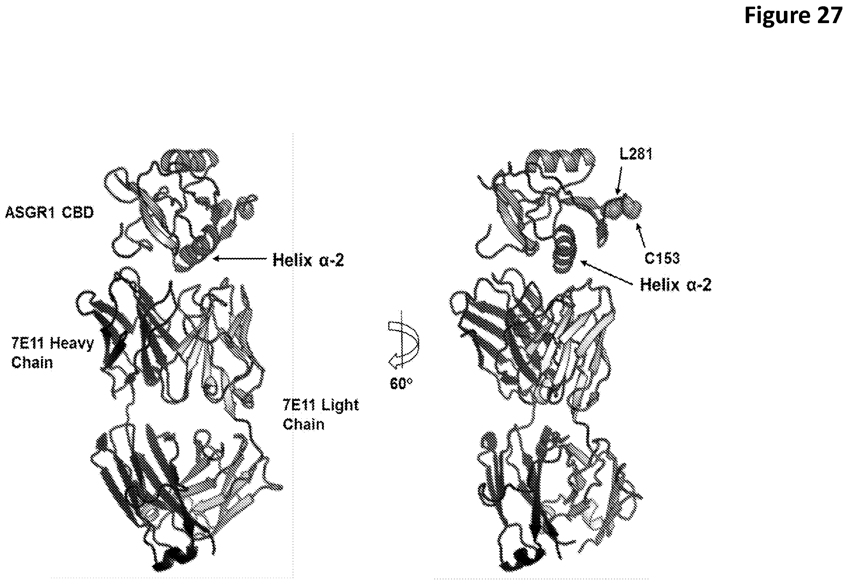

**Figure 27.** A depiction of the structure of ASGR-1 CBD and the 7E11 Fab.

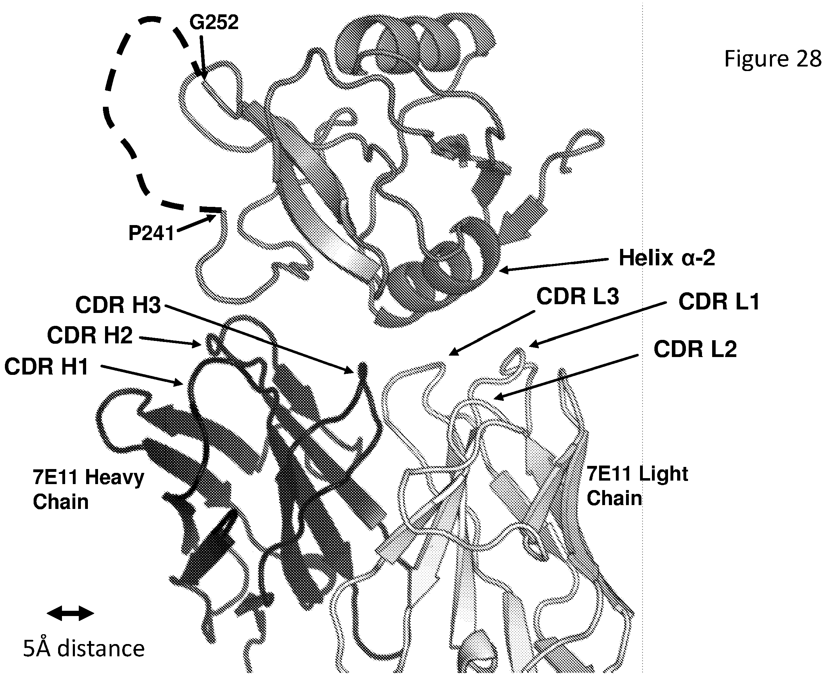

**Figure 28.** An enlarged view of the structure of the ASGR-1 CBD and the 7E11 Fab. The figure represents a disordered carbohydrate binding loop with a dashed line and highlights the indirect inhibition of ASGR-1 CBD and the ligand (GalNAc) binding. The figure incorporates a double-headed arrow which represents a 5 angstrom distance from tip to tip.

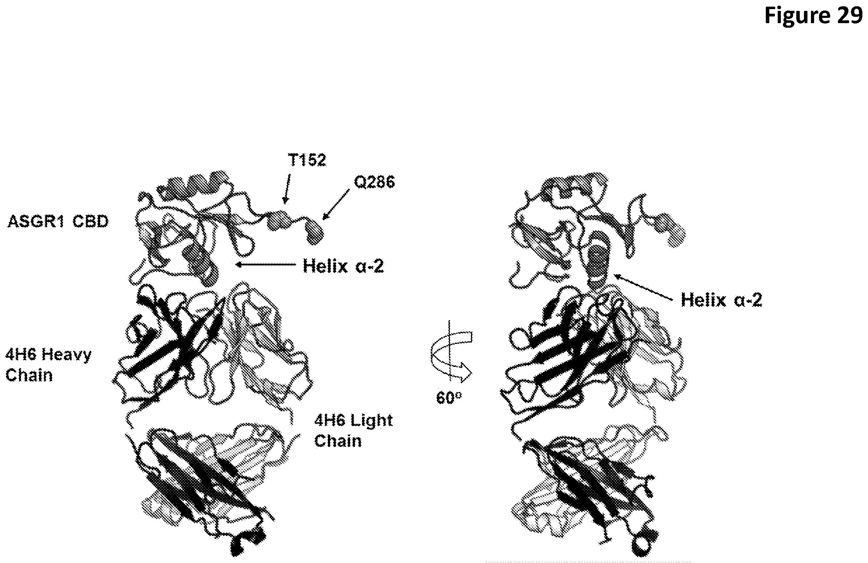

**Figure 29.** A depiction of the structure of the ASGR-1 CBD and the 4H6 Fab.

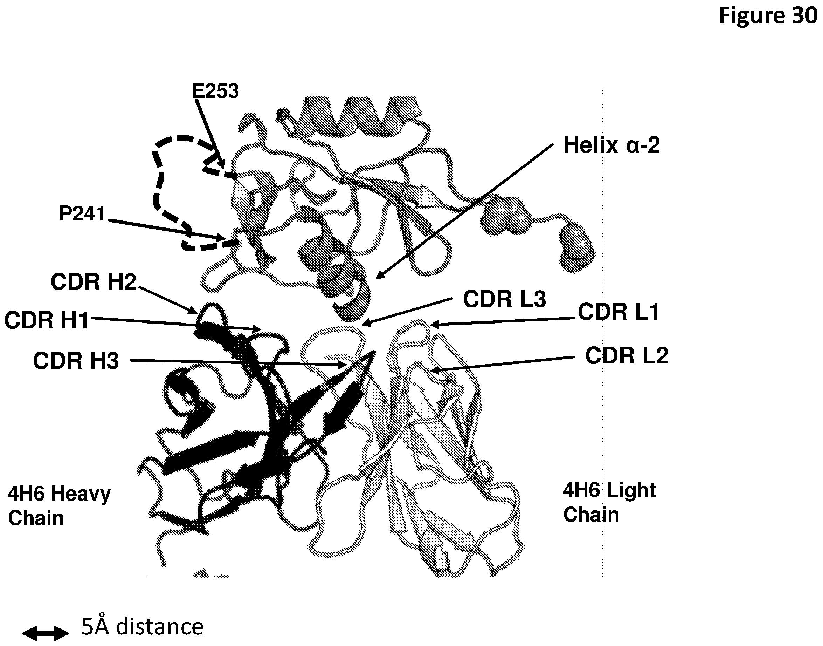

**Figure 30.** An enlarged view of structure of the ASGR-1 CBD and the 4H6 Fab. The figure represents a disordered carbohydrate binding loop with a dashed line and highlights the indirect inhibition of ASGR-1 CBD and the ligand (GalNAc) binding. The figure incorporates a double-headed arrow which represents a 5 angstrom distance from tip to tip.

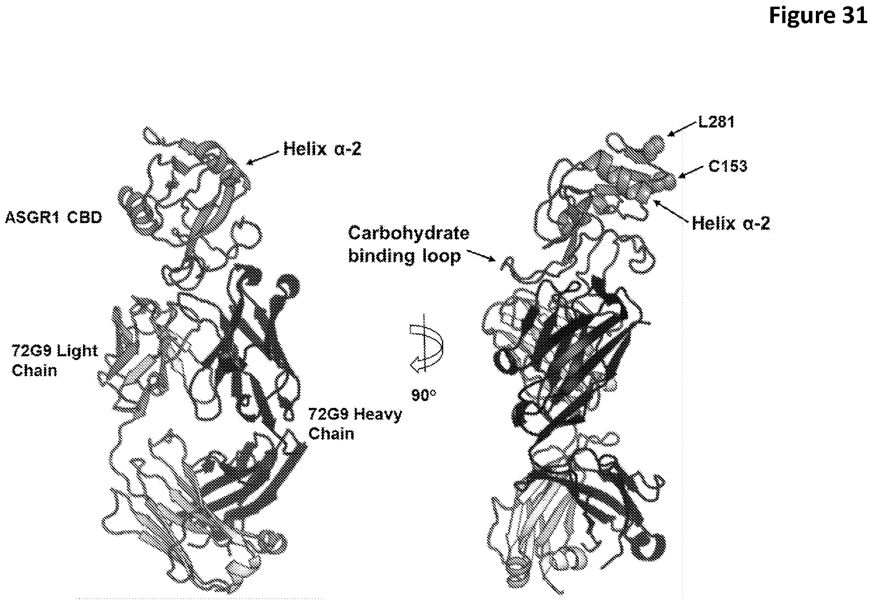

**Figure 31.** A depiction of the structure of the ASGR-1 CBD and the 72G9 Fab.

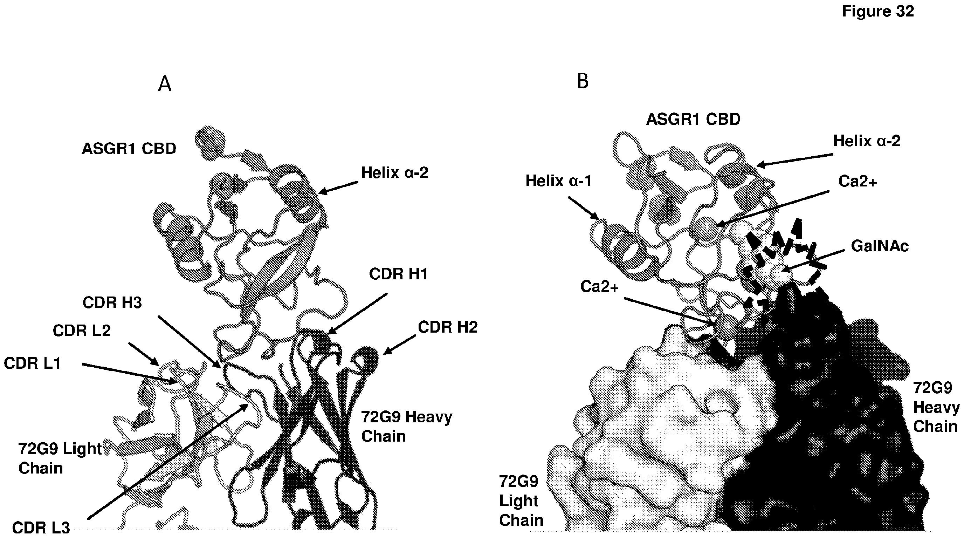

**Figure 32.** Panel A is an enlarged view of the structure of ASGR-1 CBD and the 72G9 Fab; and Panel B is a depiction of the structure of ASGR-1 CBD and the 72G9 Fab that also overlays the structure of ASGR-1 CBD and the ligand and highlights the direct inhibition of ASGR-1 CBD and the ligand (GalNAc) binding.

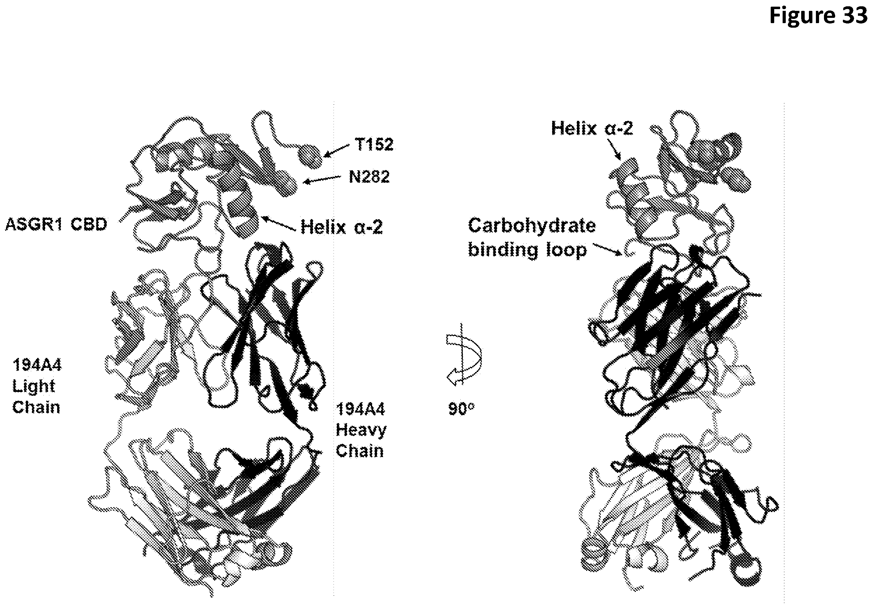

**Figure 33.** A depiction of the structure of the ASGR-1 CBD and the 194A4 Fab.

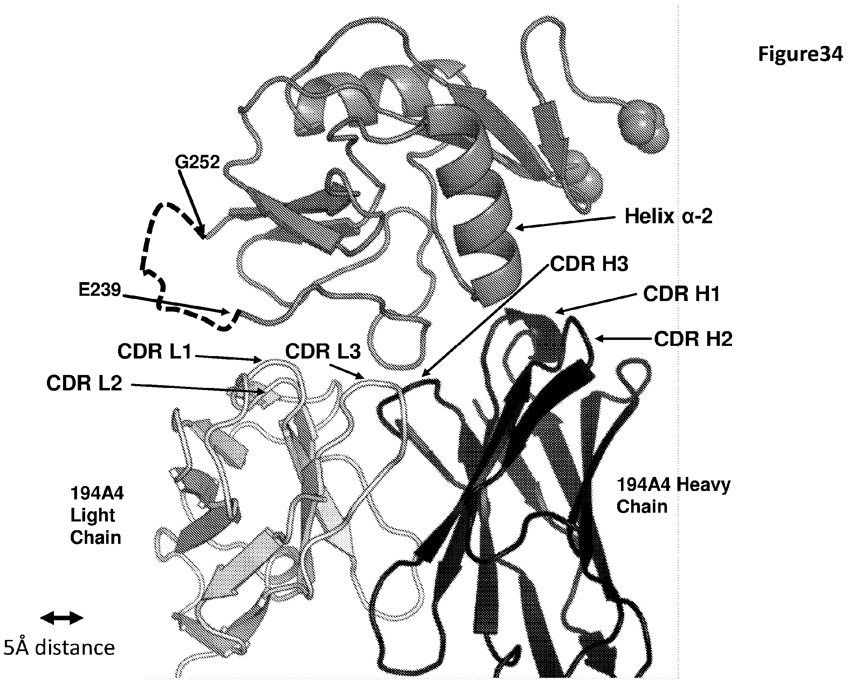

**Figure 34.** An enlarged view of the structure of the ASGR-1 CBD and the 194A4 Fab. The figure represents a disordered carbohydrate binding loop with a dashed line and highlights the indirect inhibition of ASGR-1 CBD and the ligand (GalNAc) binding. The figure incorporates a double-headed arrow which represents a 5 angstrom distance from tip to tip.

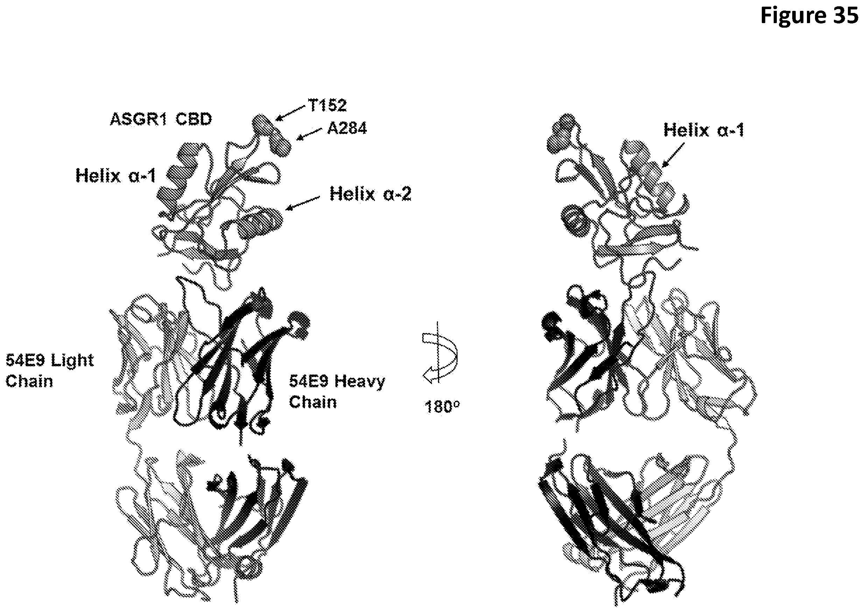

**Figure 35.** A depiction of the structure of the ASGR-1 CBD and the 54E9 Fab.

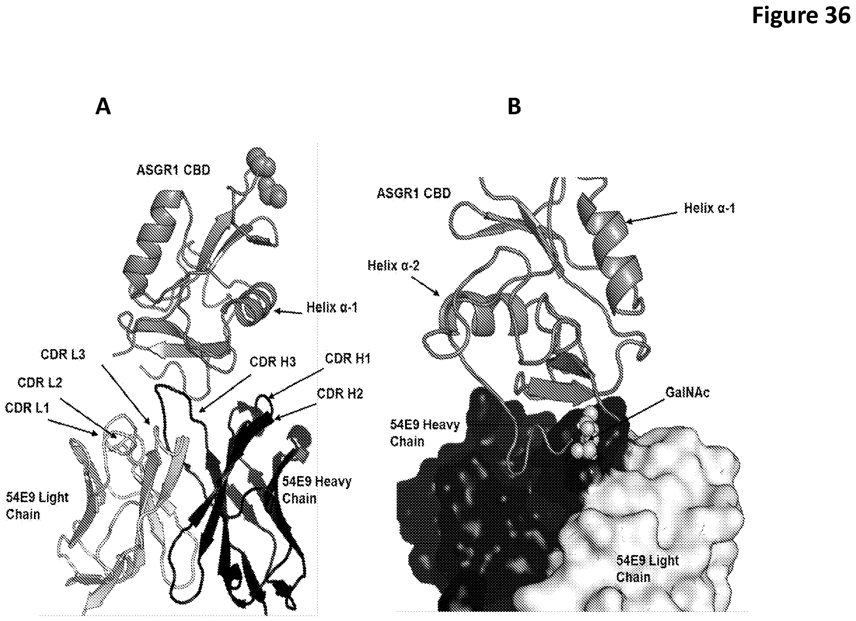

**Figure 36.** Panel A is an enlarged view of the structure of the ASGR-1 CBD and the 54E9 Fab; and Panel B is a depiction of the structure of the ASGR-1 CBD and the 54E9 Fab that also overlays the structure of ASGR-1 CBD and the ligand and highlights the direct inhibition of ASGR-1 CBD and the ligand (GalNAc) binding.

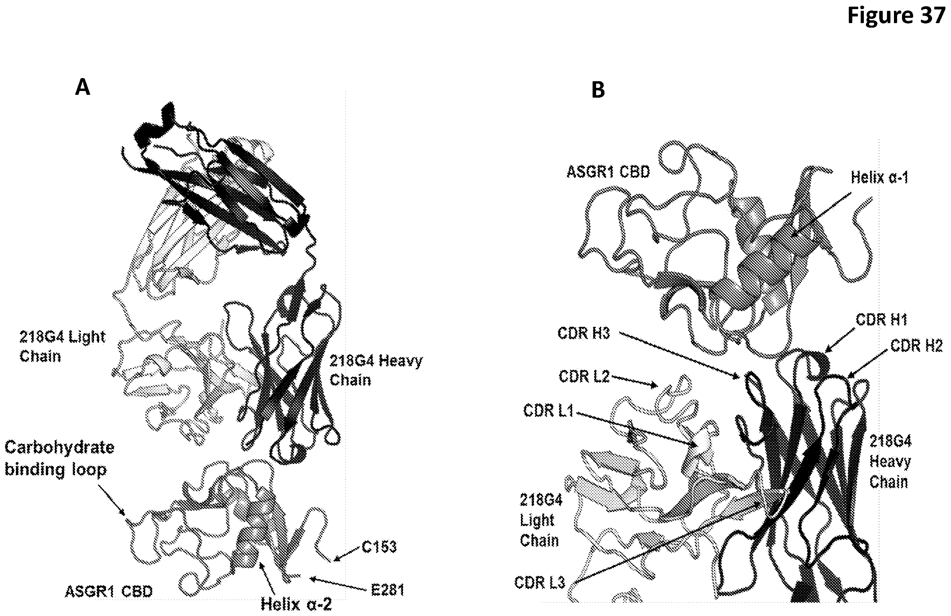

**Figure 37.** Panel A is a depiction of the structure of the ASGR-1 CBD and the 218G4 Fab; and Panel B is an enlarged view of the structure of the ASGR-1 CBD and the 218G4 Fab.

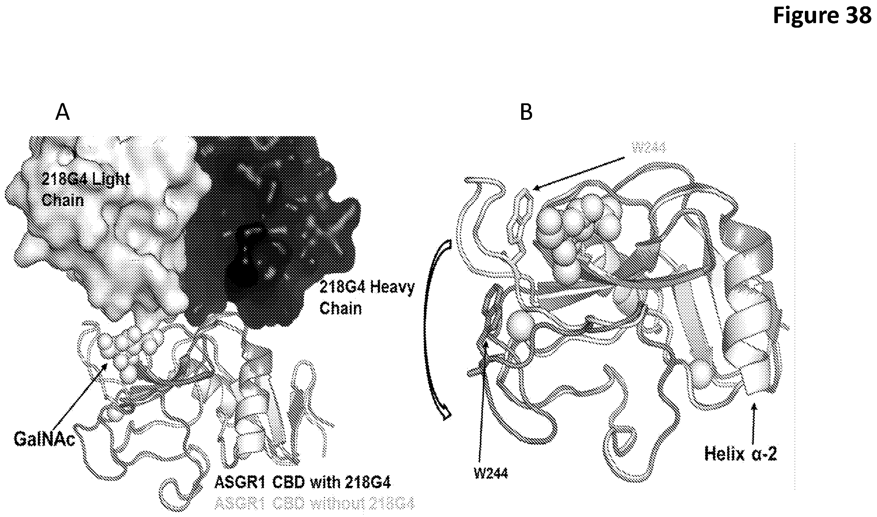

**Figure 38.** Panels A and B are enlarged views of the structure of ASGR-1 CBD and the 218G4 Fab that also overlays the structure of ASGR-1 CBD and the ligand. These figures highlight the direct inhibition of ASGR-1 CBD and the ligand (GalNAc) binding when the 218G4 Fab is present.

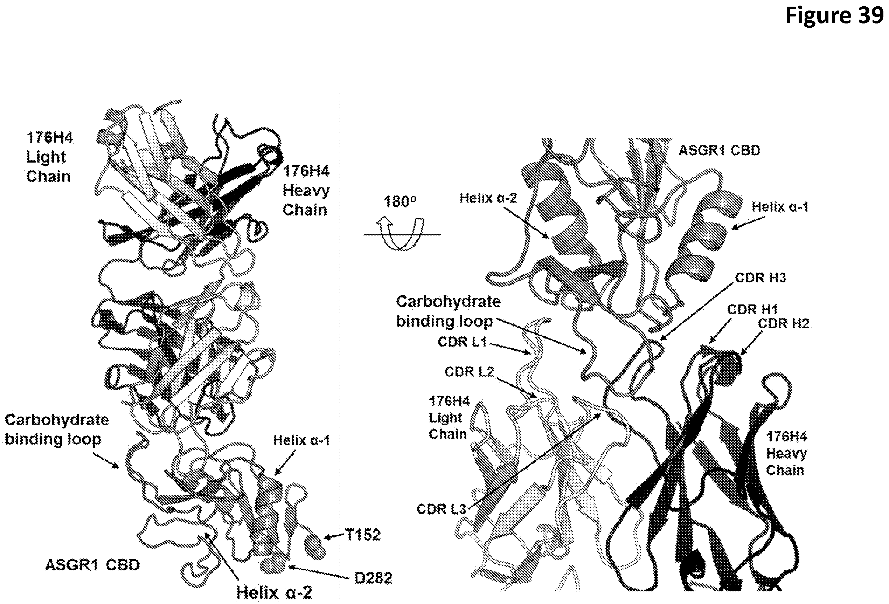

**Figure 39.** A depiction of the structure of the ASGR-1 CBD and the 176H4 Fab.

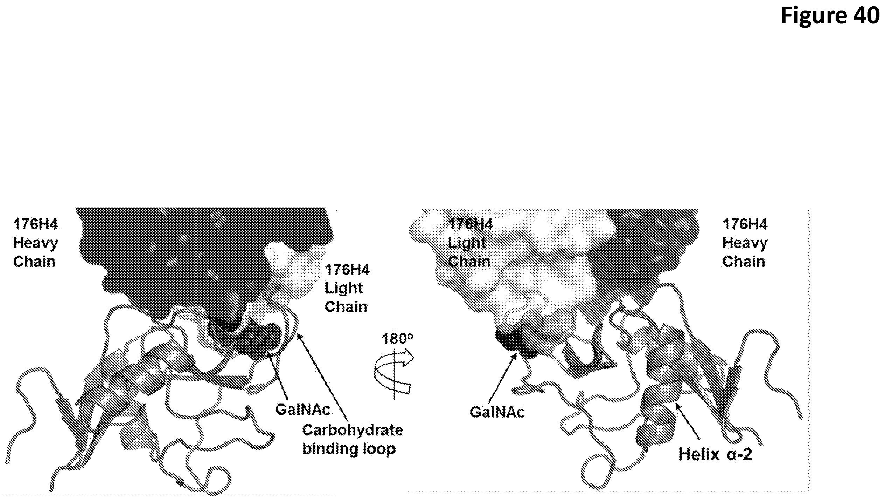

**Figure 40.** An enlarged view of the structure of the ASGR-1 CBD and the 176H4 Fab that also overlays the structure of ASGR-1 CBD and the ligand. This figure highlight the direct inhibition of ASGR-1 CBD and the ligand (GalNAc) binding when the 176H4 Fab is present.

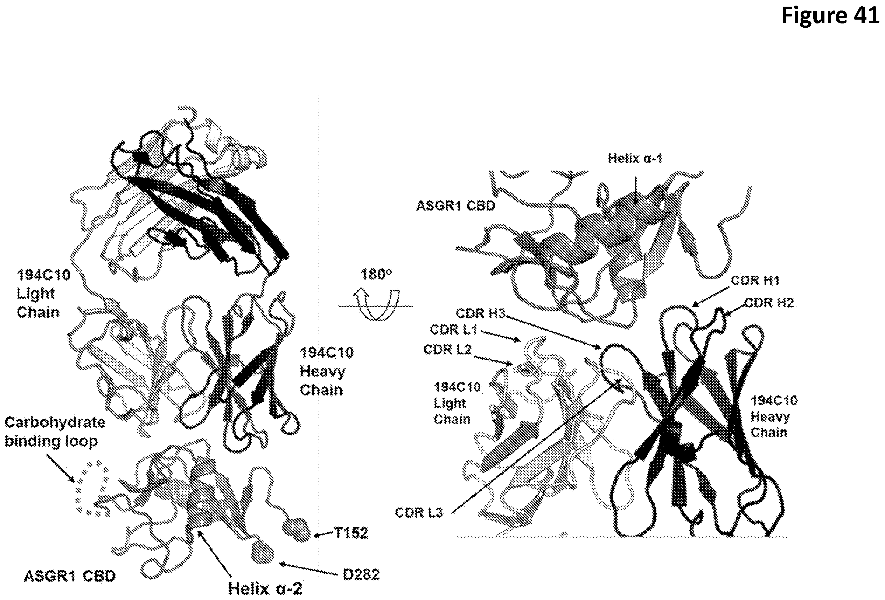

**Figure 41.** A depiction of the structure of the ASGR-1 CBD and the 194C10 Fab. This figure depicts represents a disordered carbohydrate binding loop with a dashed line and highlights possible indirect inhibition of ASGR-1 CBD and the ligand (GalNAc) binding.

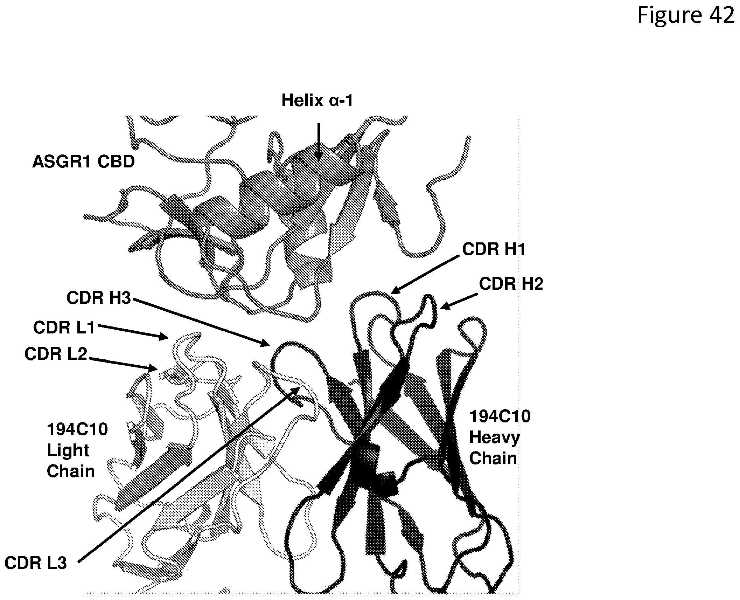

**Figure 42.** An enlarged view of the structure of the ASGR-1 CBD and the 194C10 Fab. This figure shows the CDRs of the 194C10 that interact with the ASGR-1 CBD and highlights that there may be direct inhibition of the ASGR-1 CBD and the ligand (GalNAc) binding.

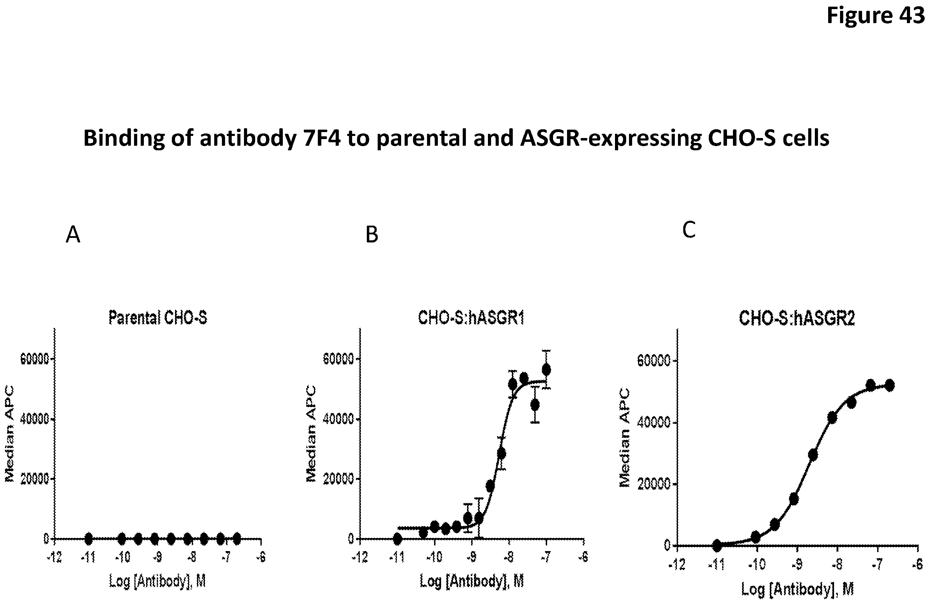

**Figure 43.** Panels A-C are graphical representations showing antibody binding results from human ASGR-1 and human ASGR-2 expressing cells.

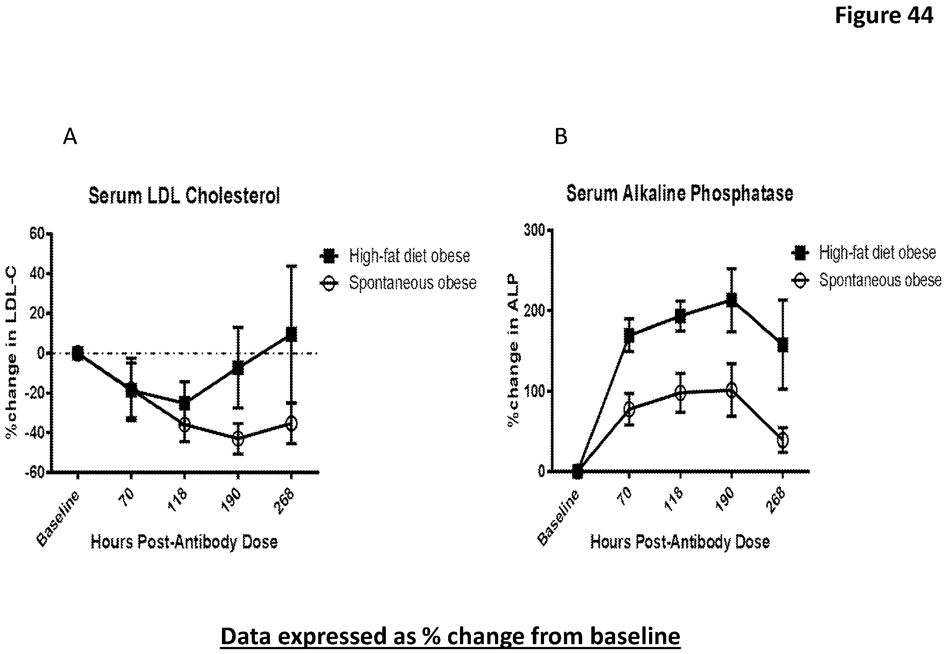

**Figure 44.** Panel A is a graphical representation of the effect of ASGR-1 antibody, 4A2, on serum LDL cholesterol levels in obese cynomologous monkeys. Panel B is a graphical representation of the effect of ASGR-1 antibody, 4A2, on serum alkaline phosphatase levels in obese cynomologous monkeys. Data is expressed in the % change from baseline.

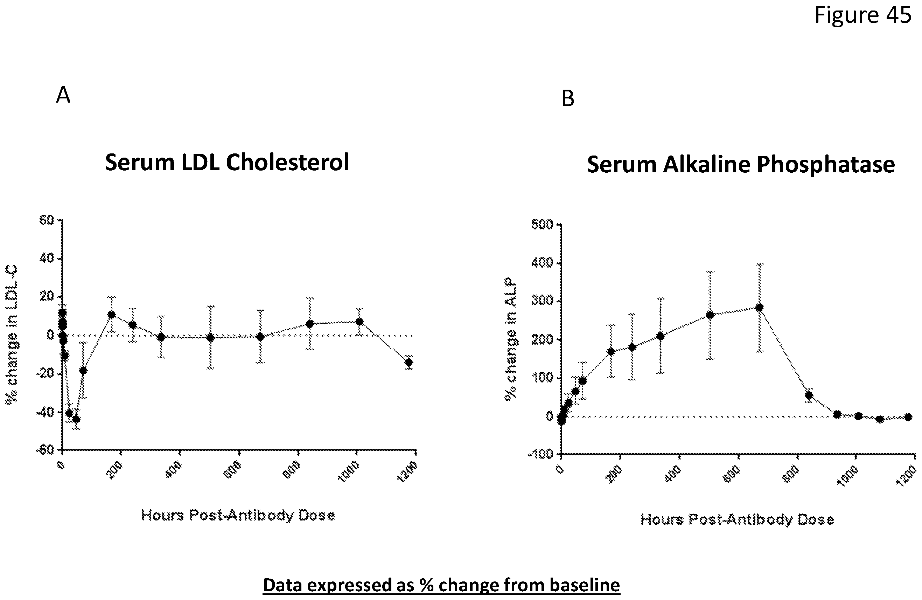

**Figure 45.** Panel A is a graphical representation of the effect of ASGR-1 antibody, 4A2, on serum LDL cholesterol levels in normal cynomologous monkeys. Panel B is a graphical representation of the effect of ASGR-1 antibody, 4A2, on serum alkaline phosphatase levels in normal cynomologous monkeys. Data is expressed in the percent change from baseline.

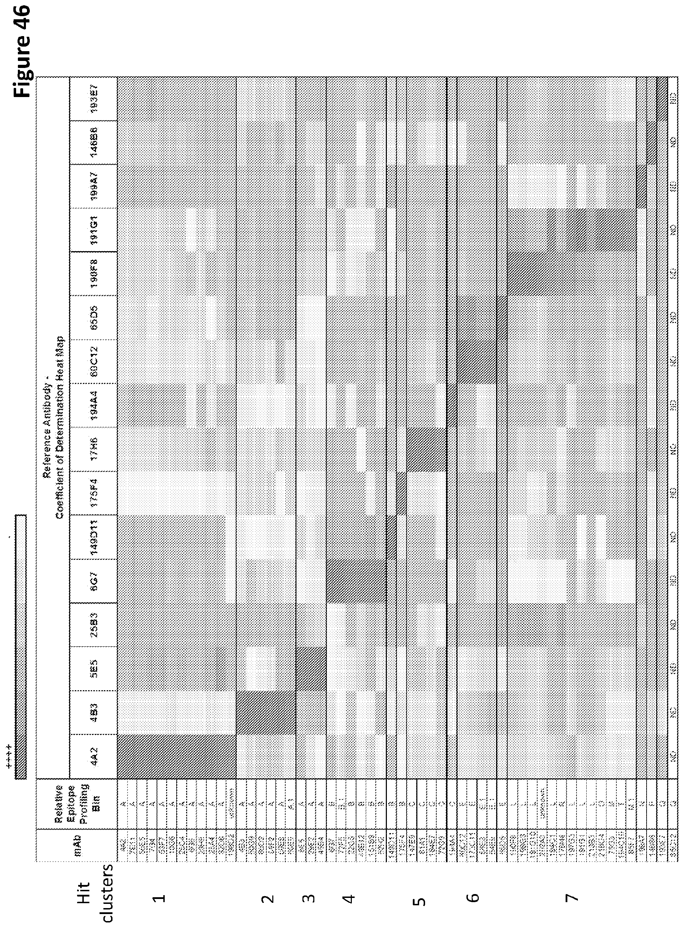

**Figure 46.** A coefficient of determination heat map representing the coefficient of determination profiles of test ASGR-1 ligand blocking antibody-reference antibody combinations from an Arginine/Glutamic Acid scanning mutagenesis (Example 7E). Dark shading represents highly similar data, while light shading represents highly dissimilar data. The relative epitope profiling (antibody competition/binding) bin assignments are also indicated.

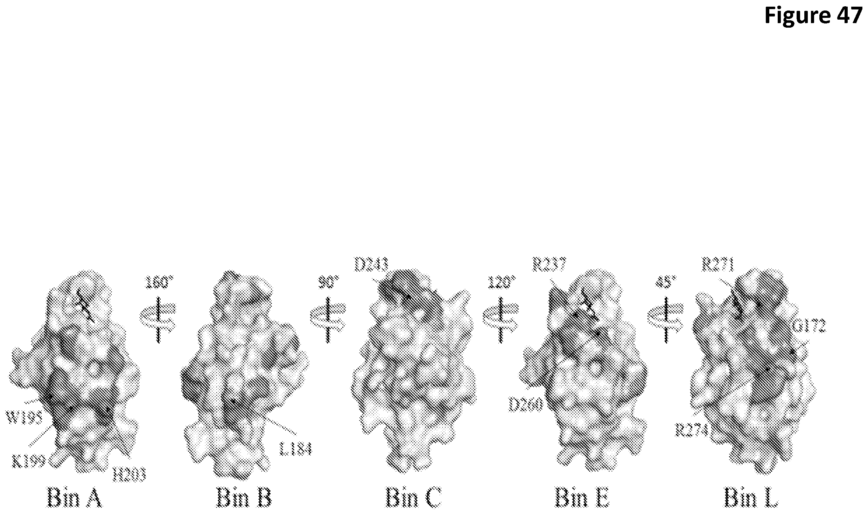

**Figure 47.** A computer representation showing alternative views of the ASGR-1 CBD protein and the surface locations of amino acid residues identified as being important for antibody binding via Arginine/Glutamic Acid scanning mutagenesis (Example 7E). The relative epitope profiling (antibody competition/binding) bin assignments are also indicated. Ligand (GalNAc) is shown as a stick representation (black). The ASGR-1 CBD is shown as a surface representation (light grey). The positions of amino acids identified by Arg/Glu mutational scanning are indicated (dark grey surface). The relative positions of key amino acids in each bin are shown for reference only.

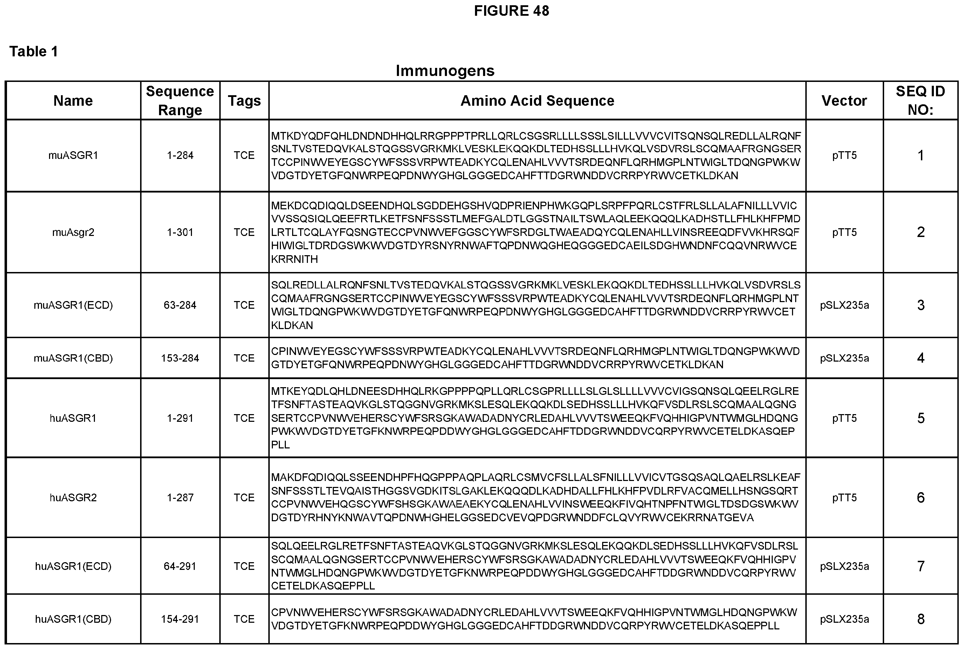

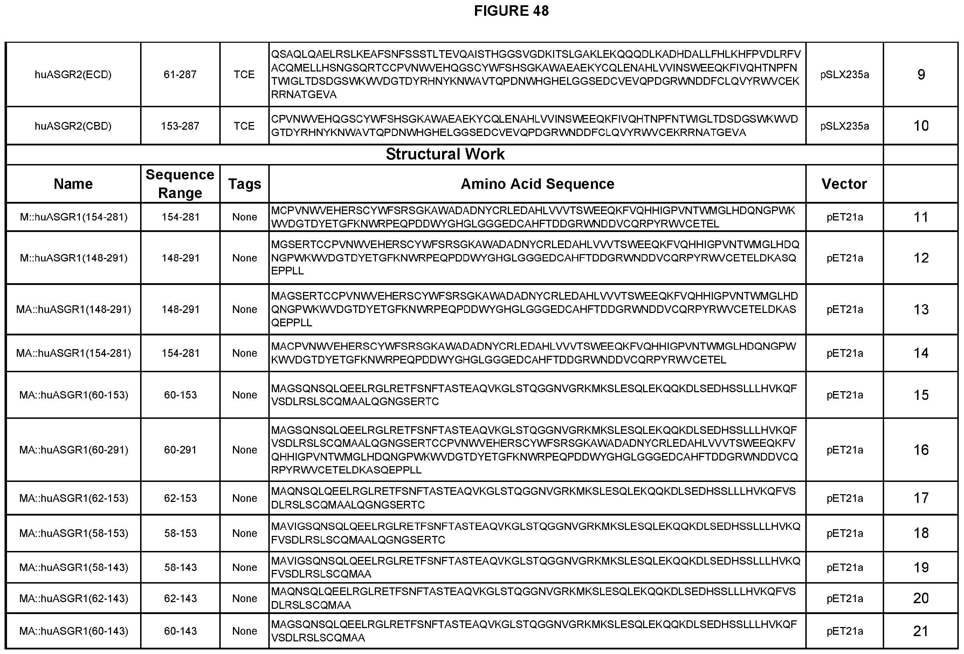

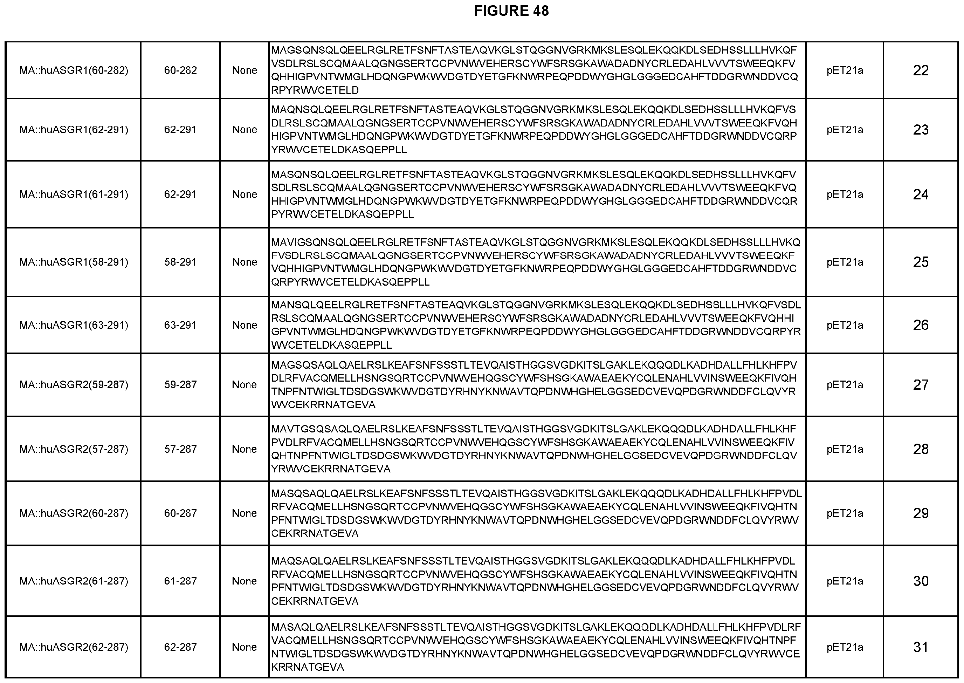

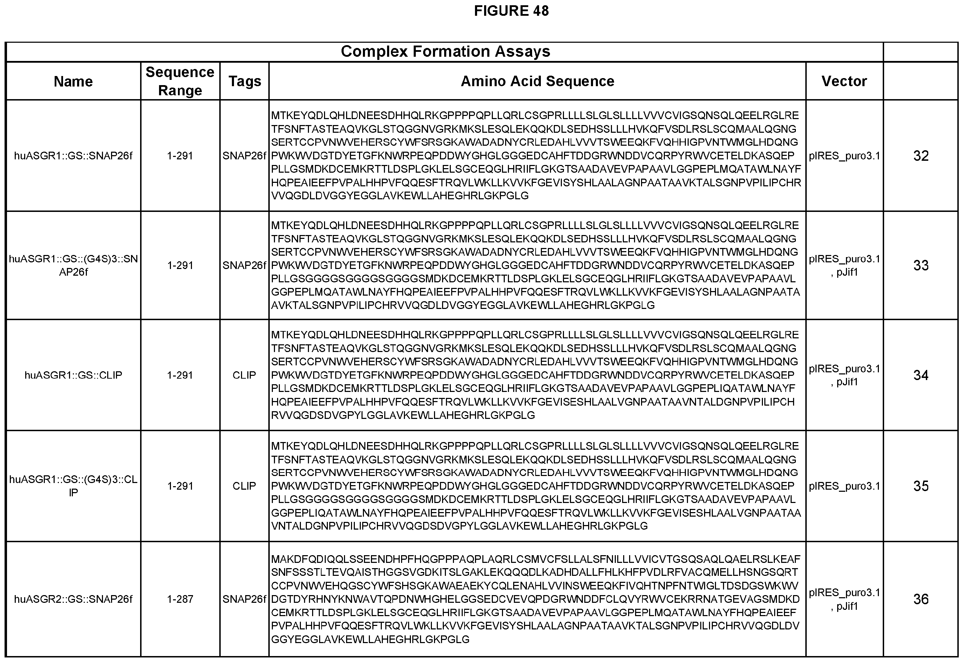

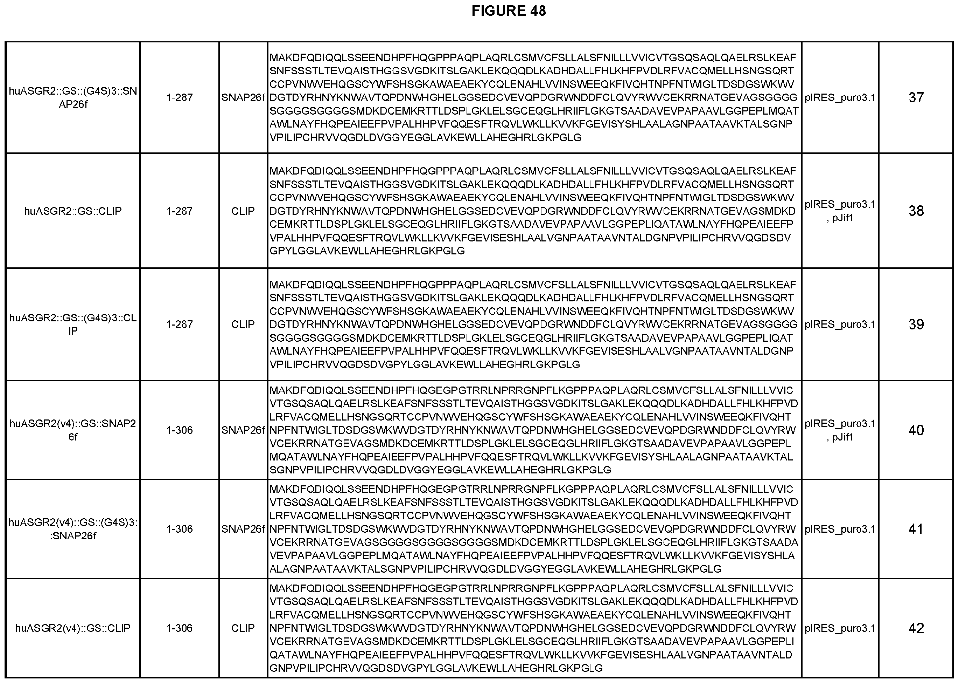

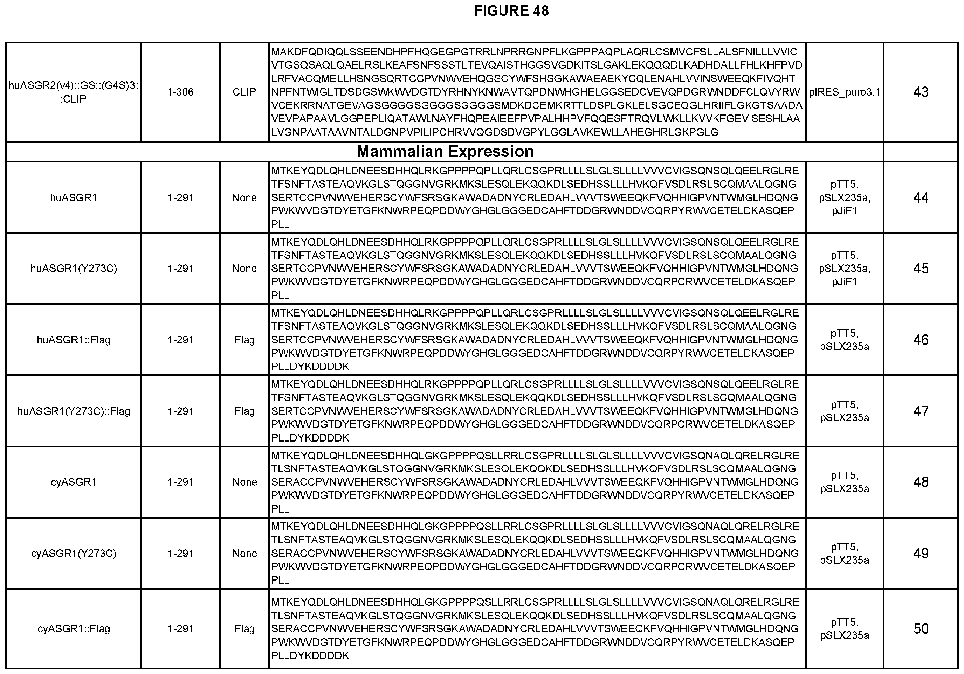

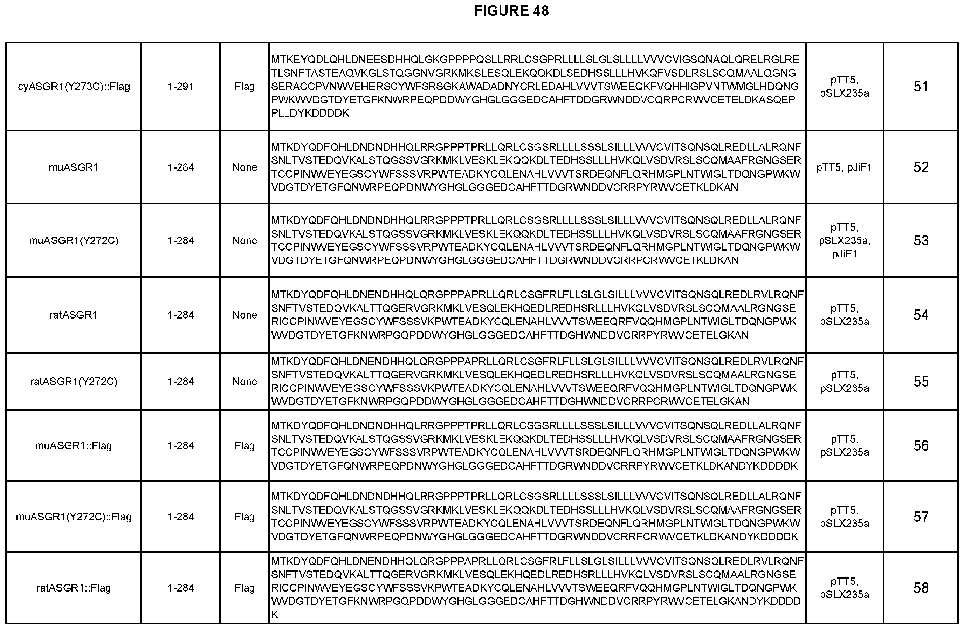

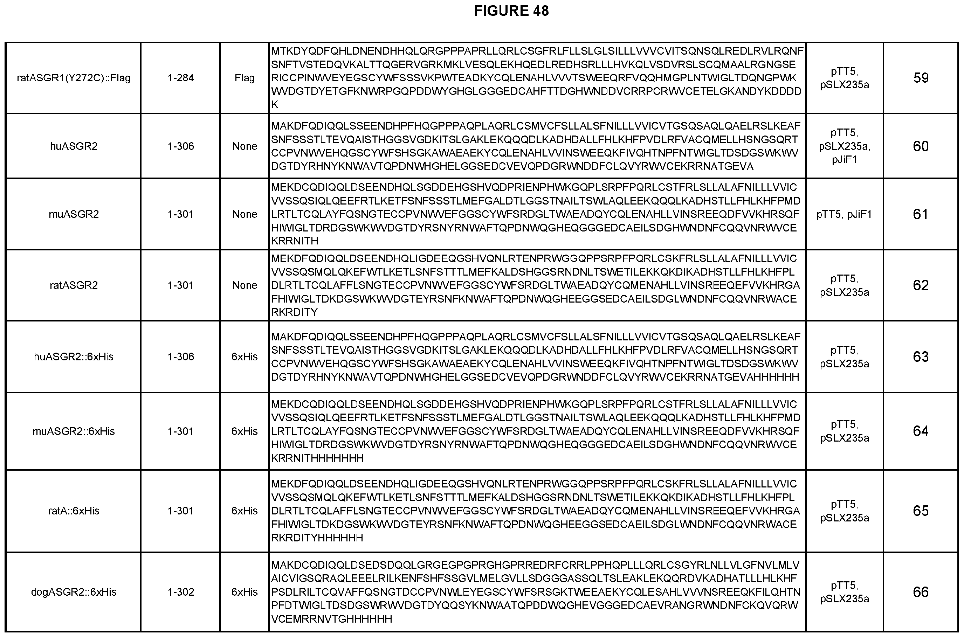

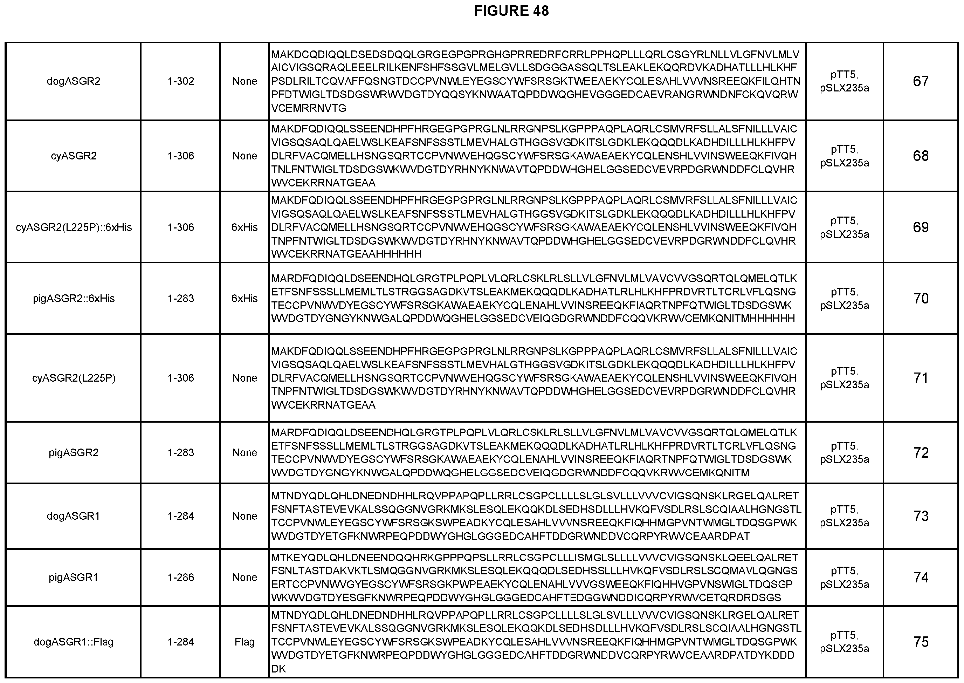

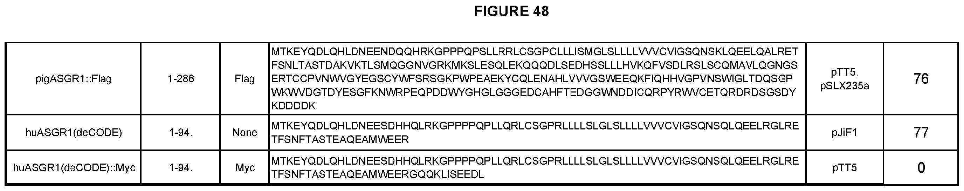

**Figure 48.** A table presenting various protein sequences for human, mouse, rat, pig, dog and cynomolgus monkey ASGR, ASGR-1 and ASGR-2 (Table 1).

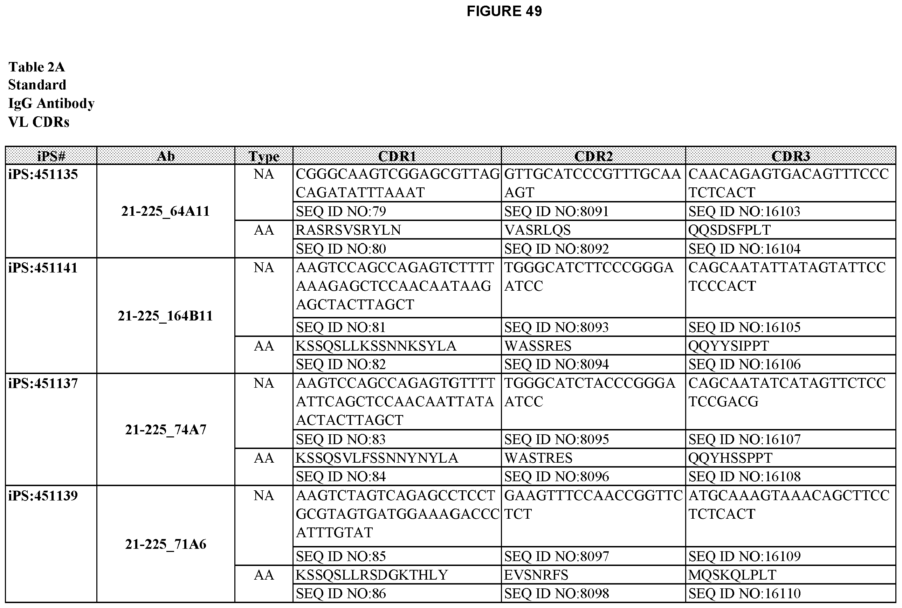

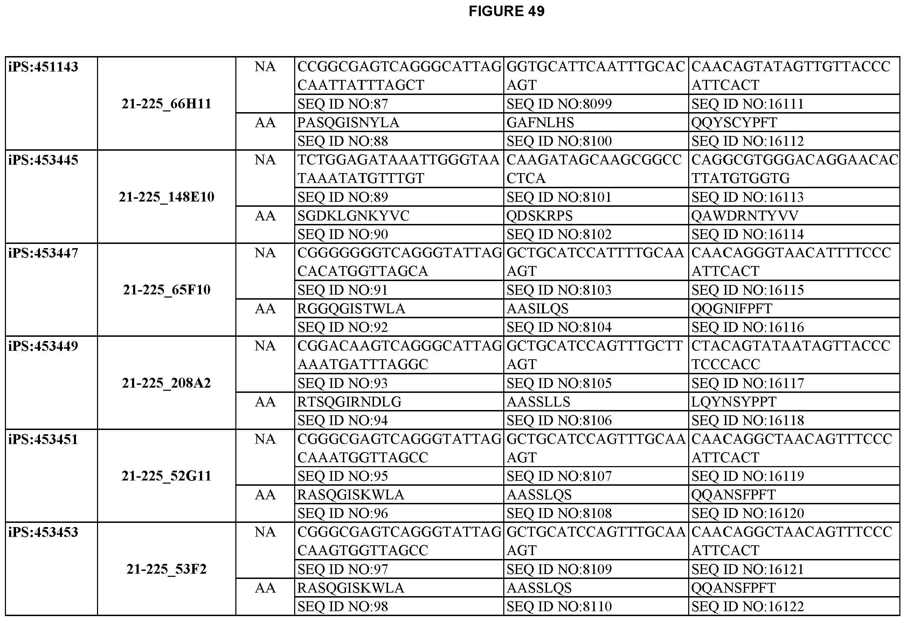

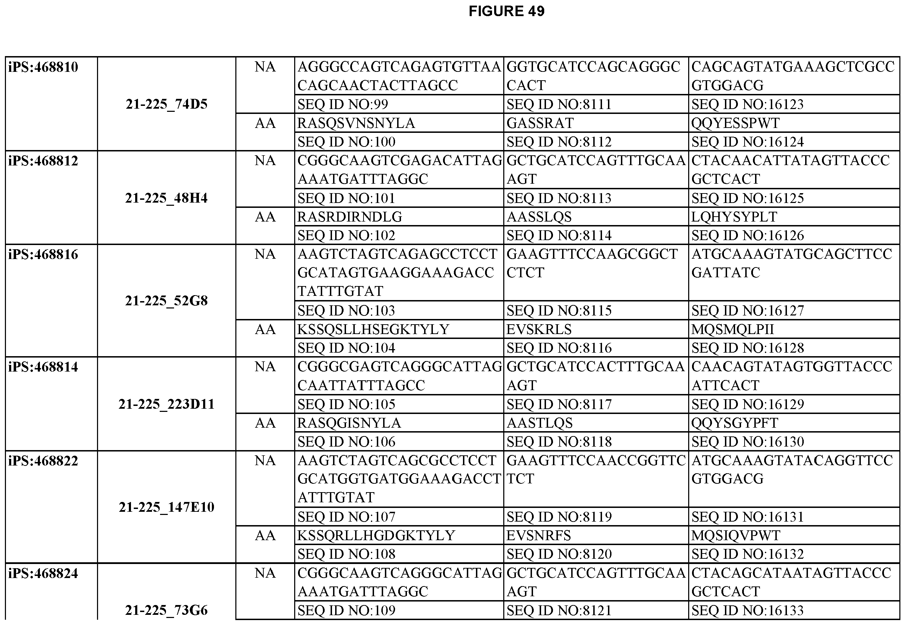

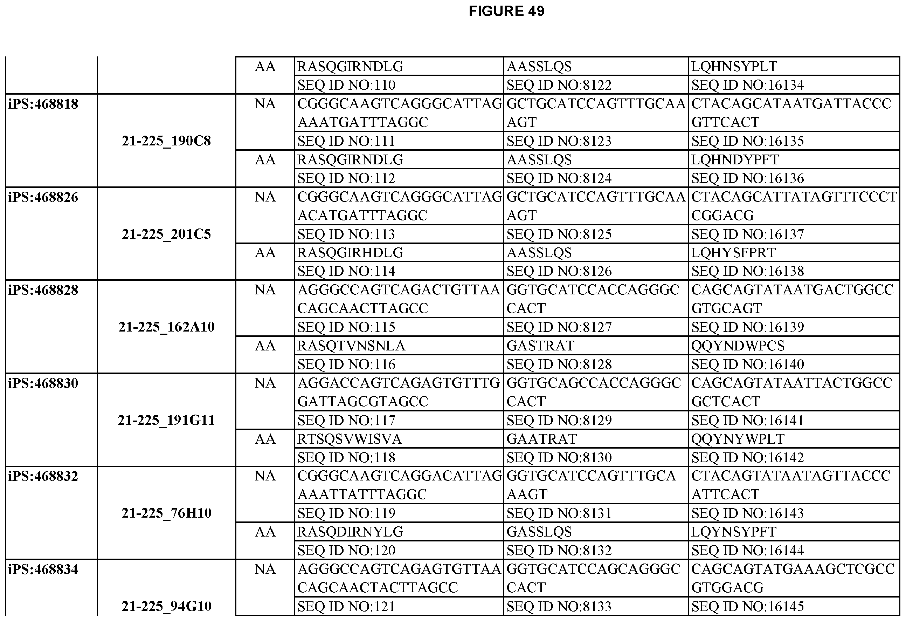

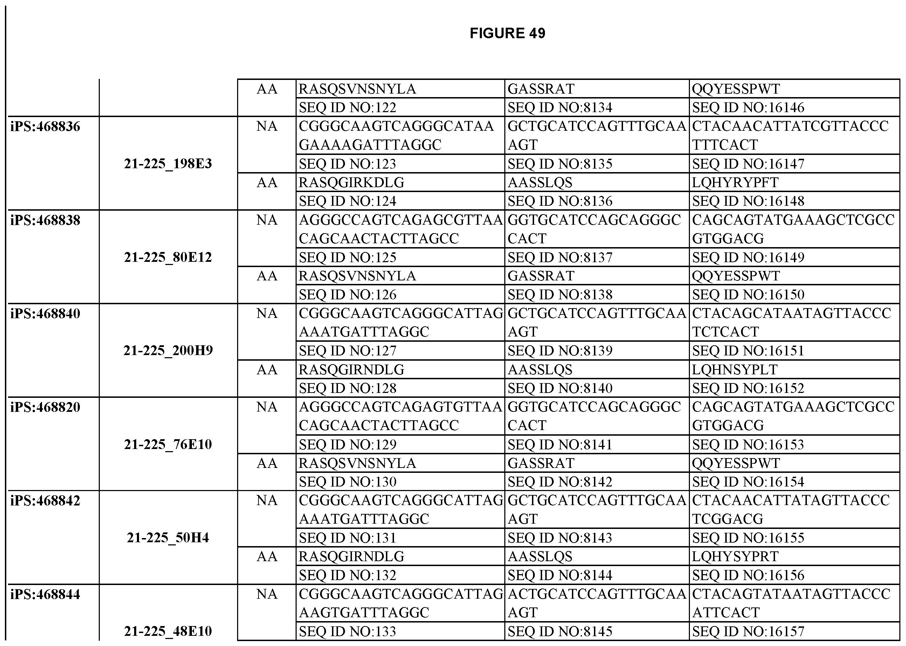

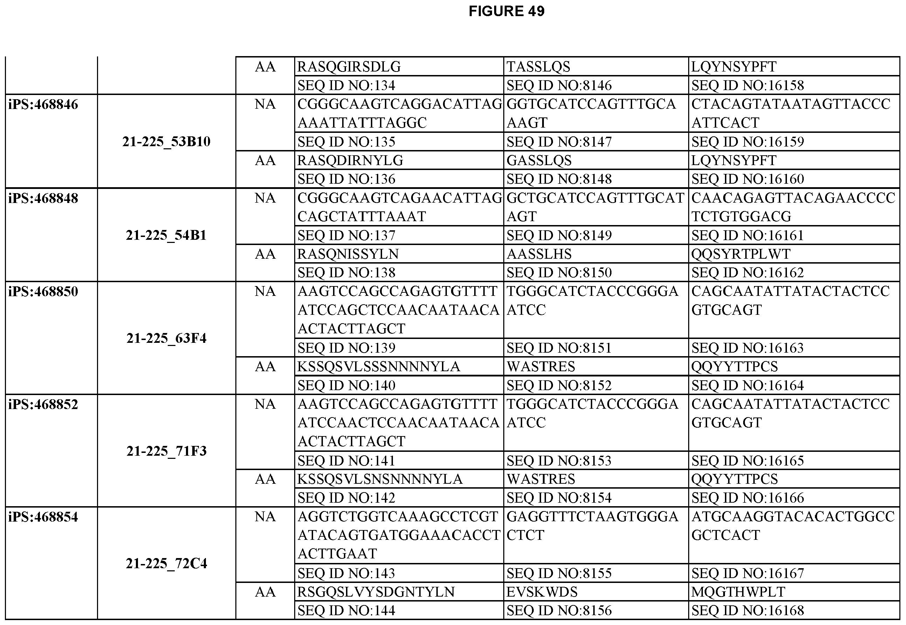

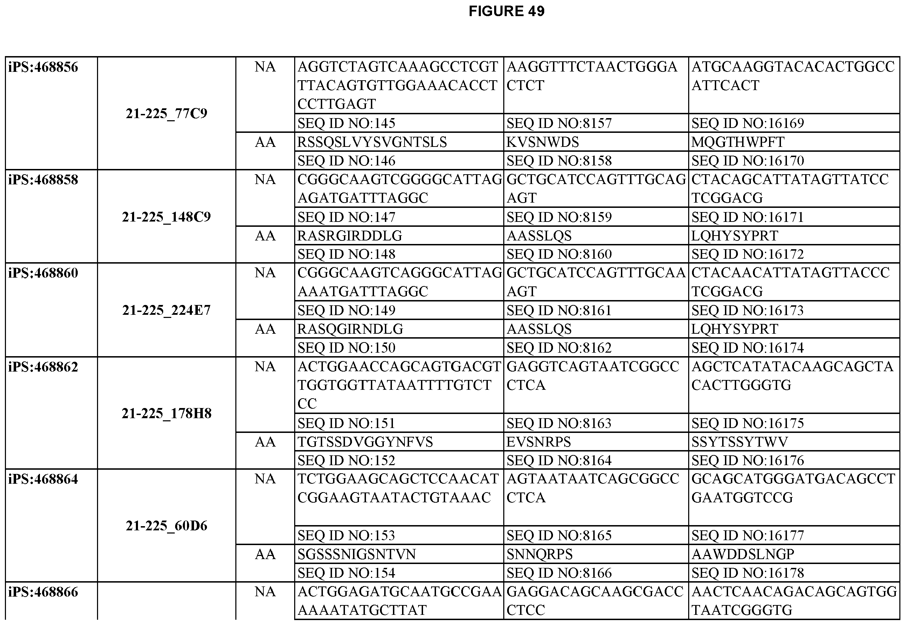

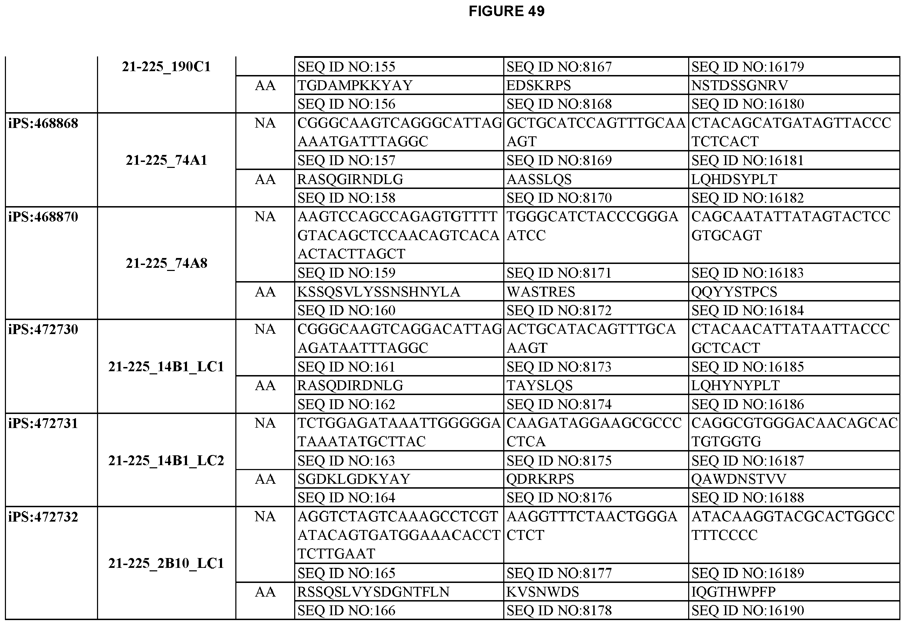

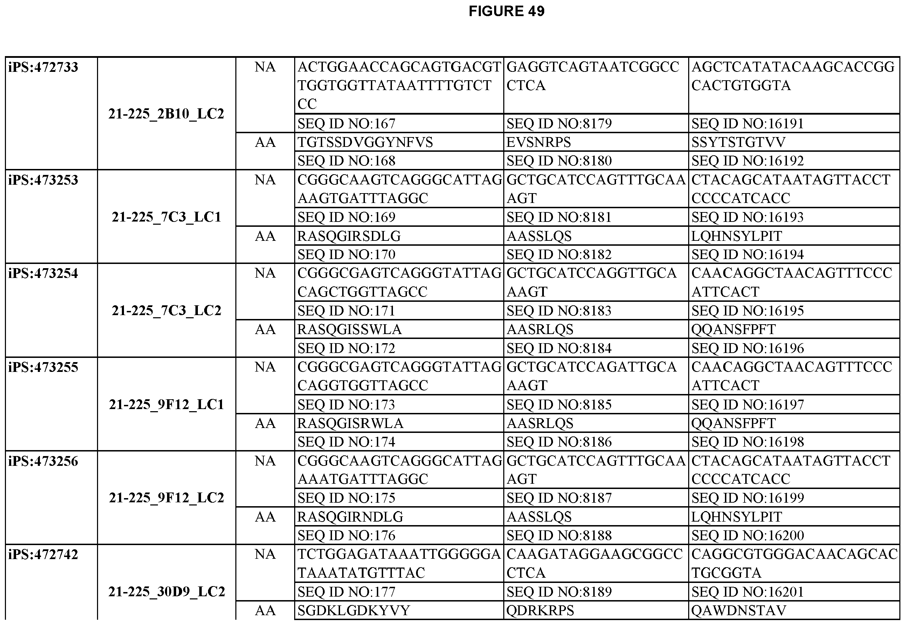

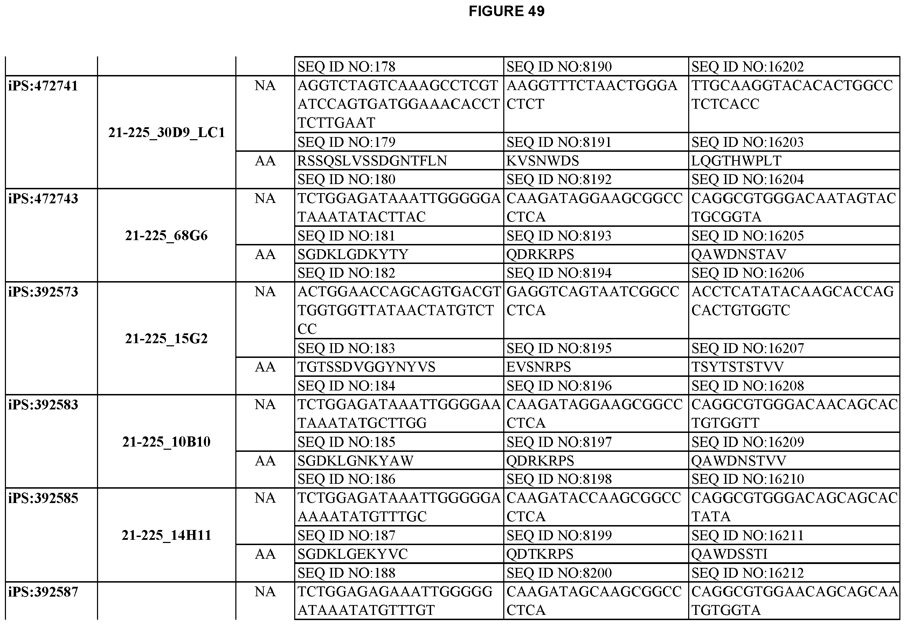

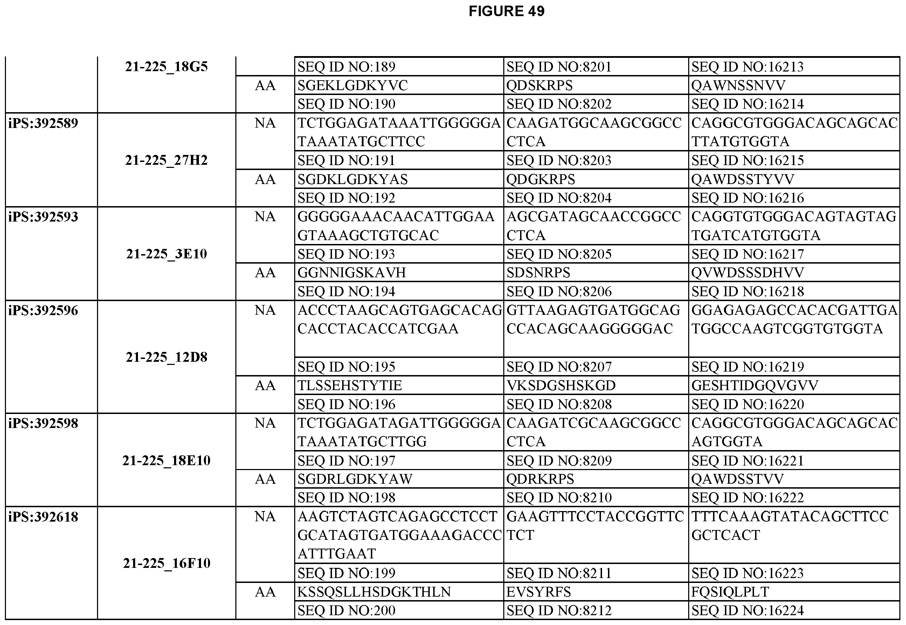

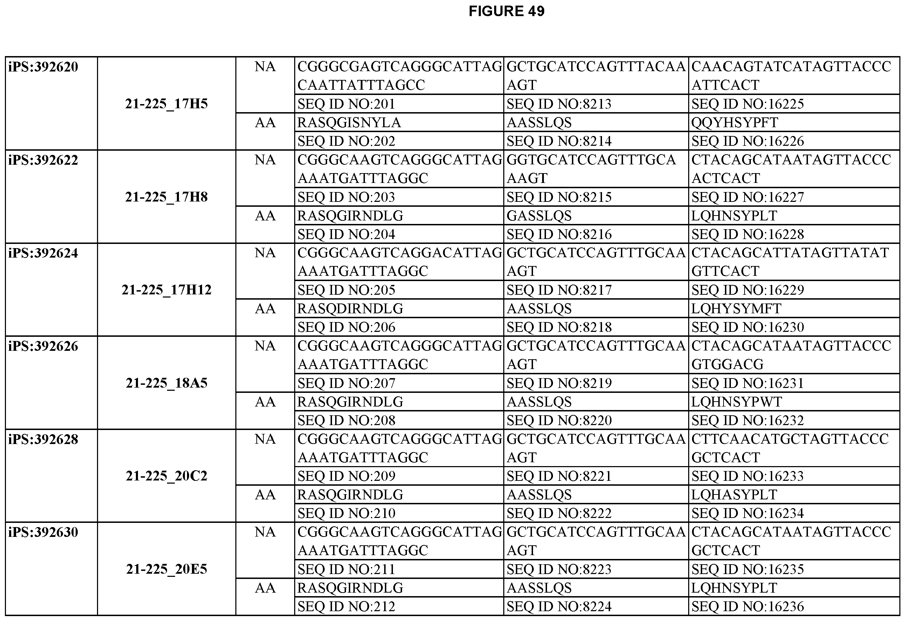

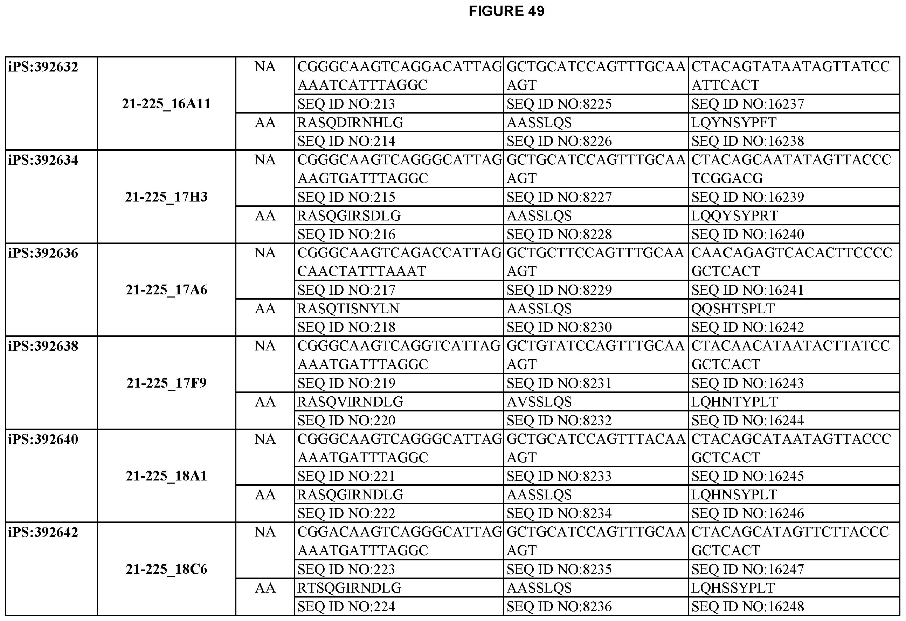

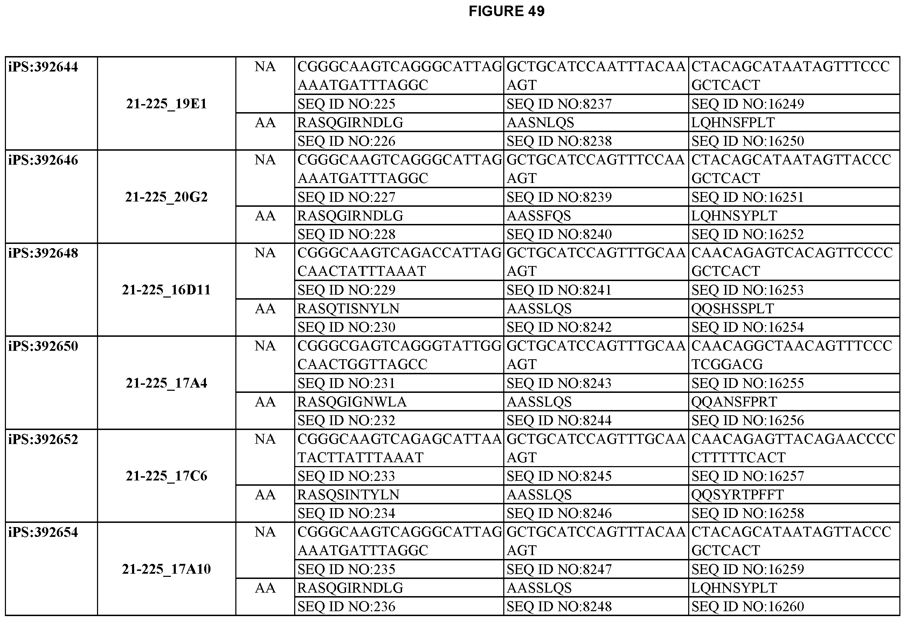

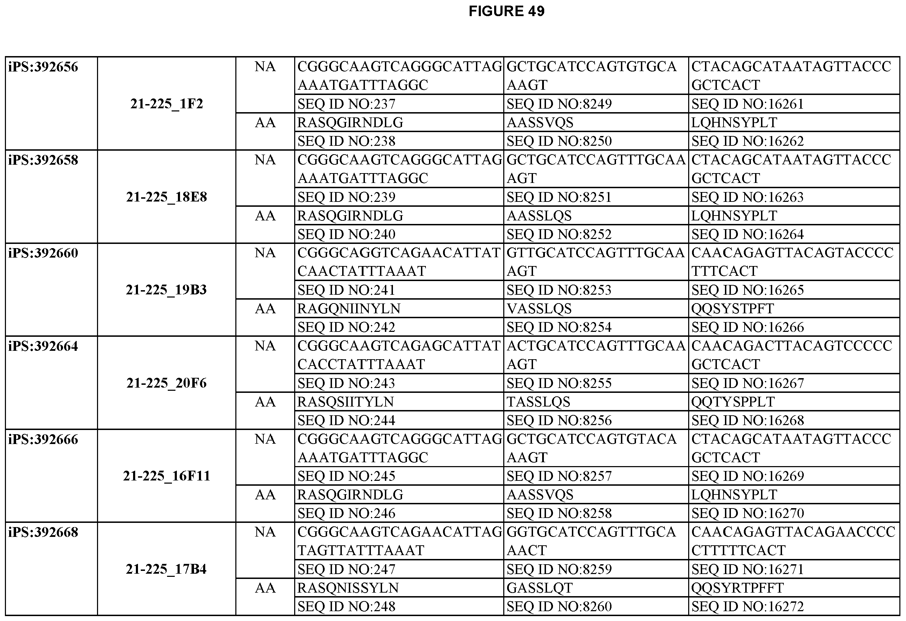

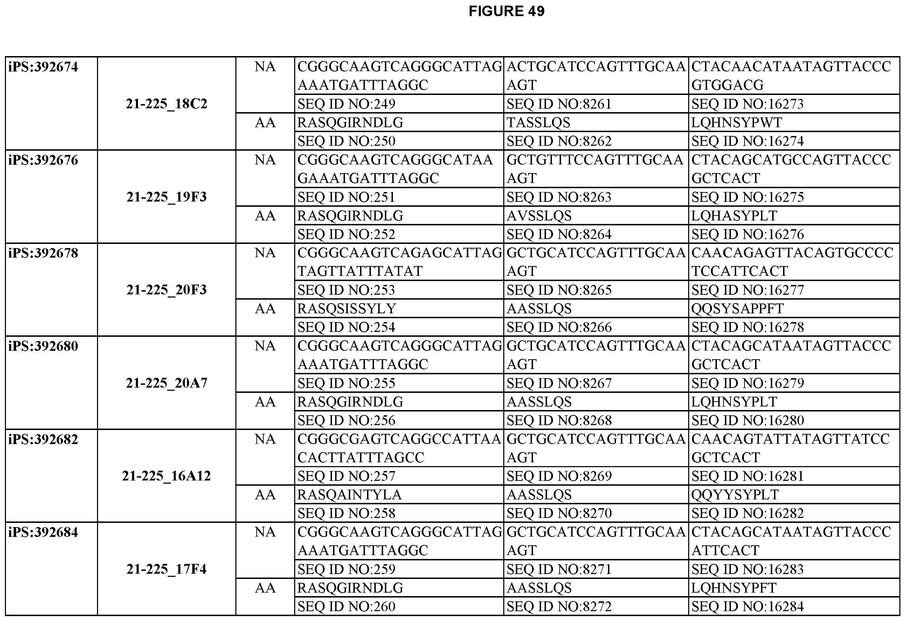

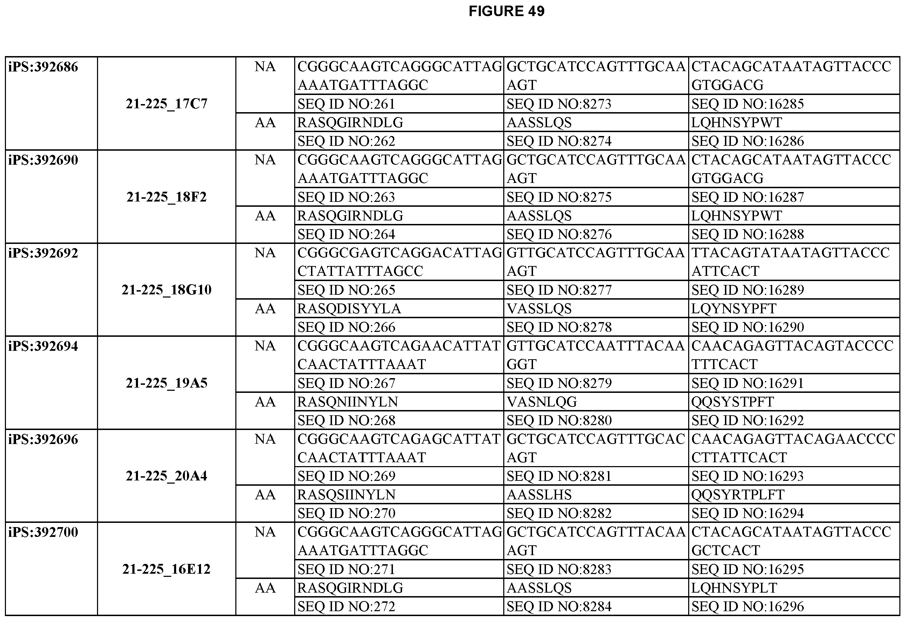

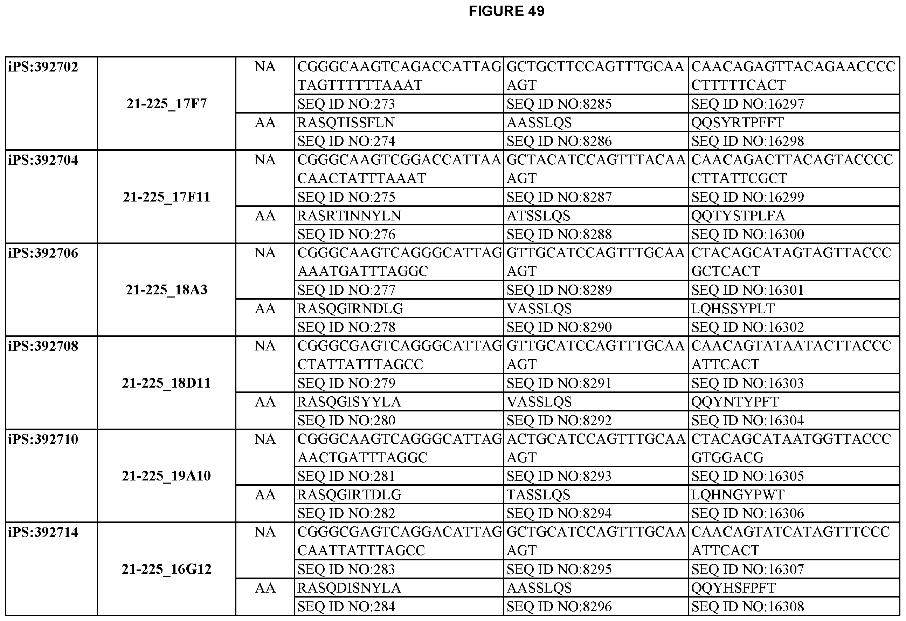

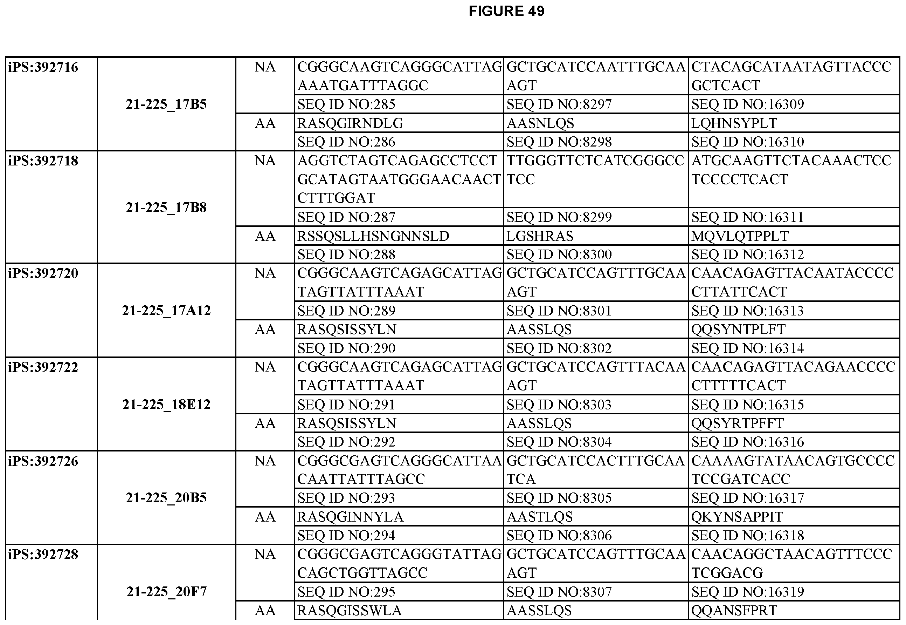

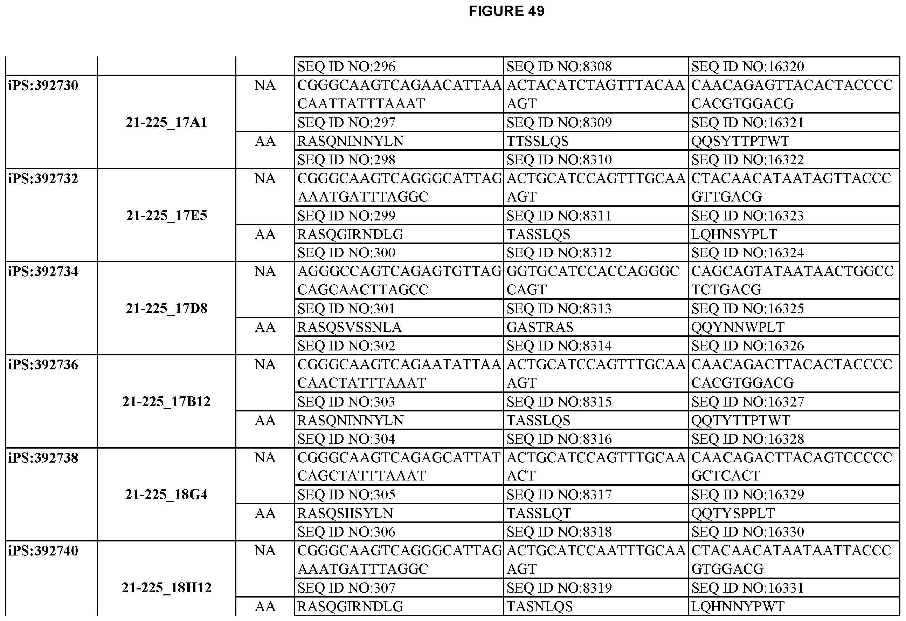

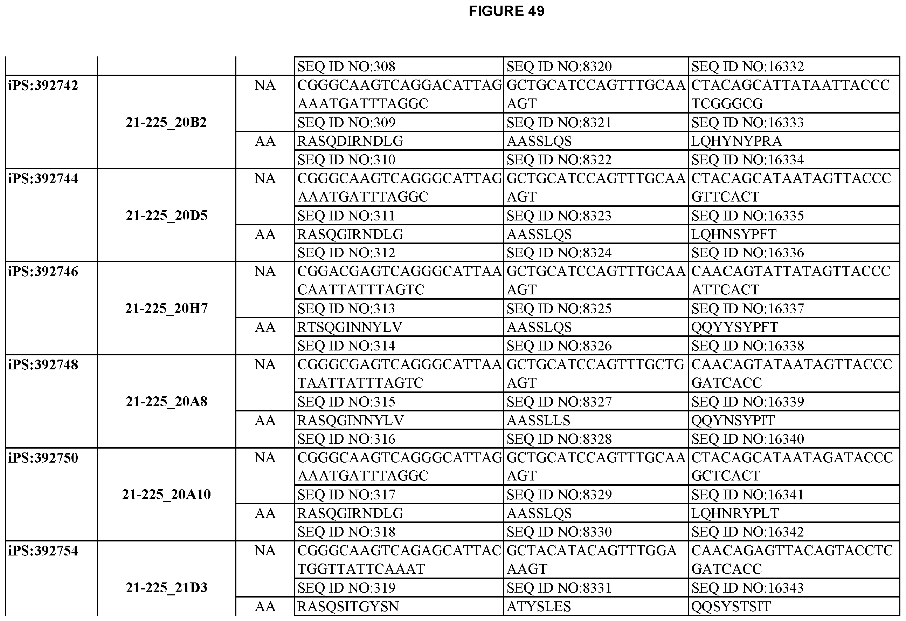

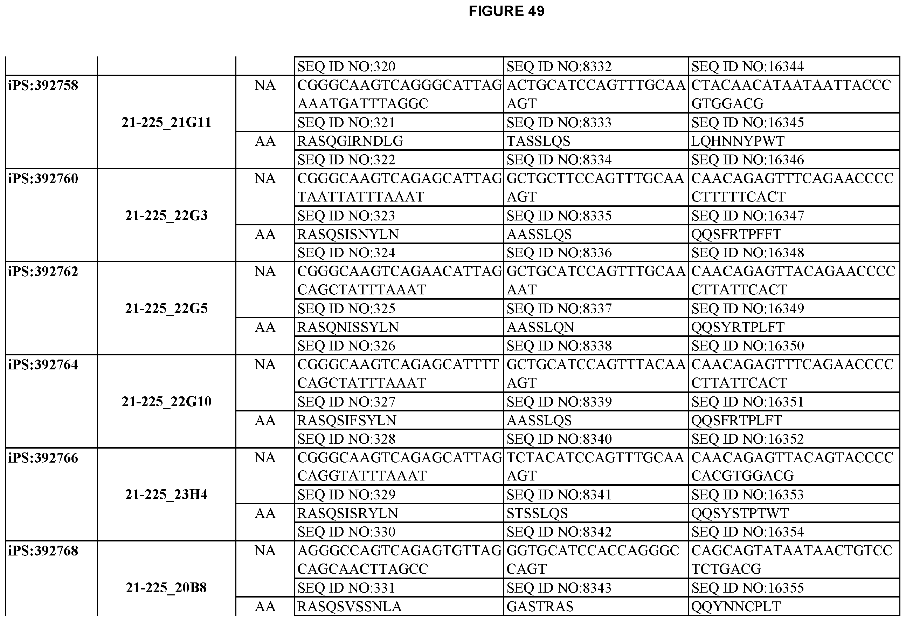

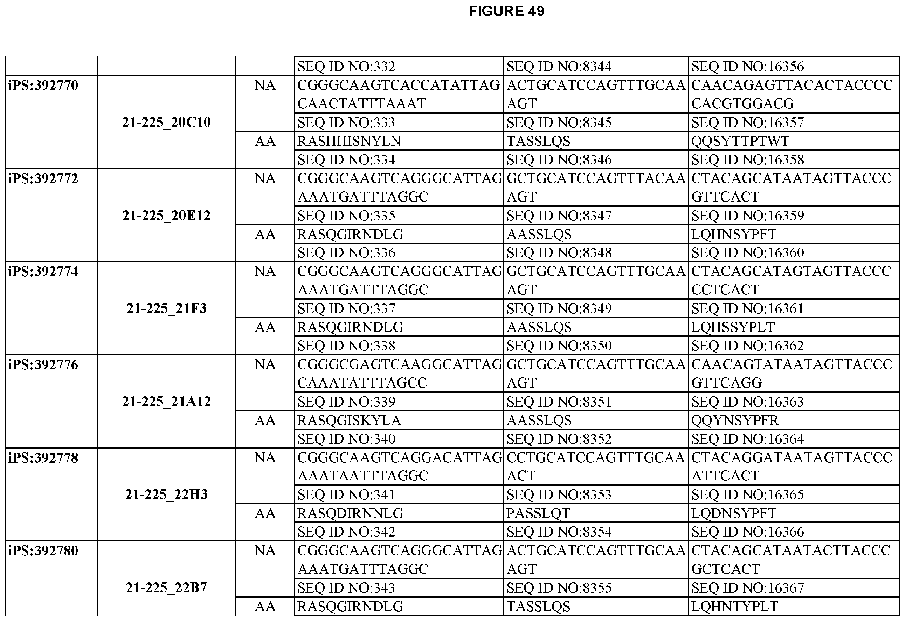

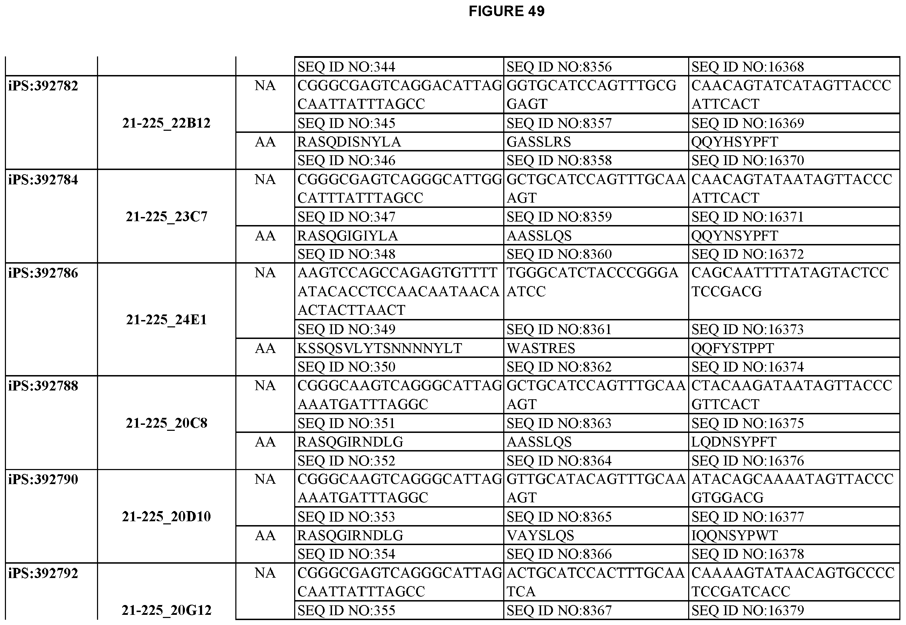

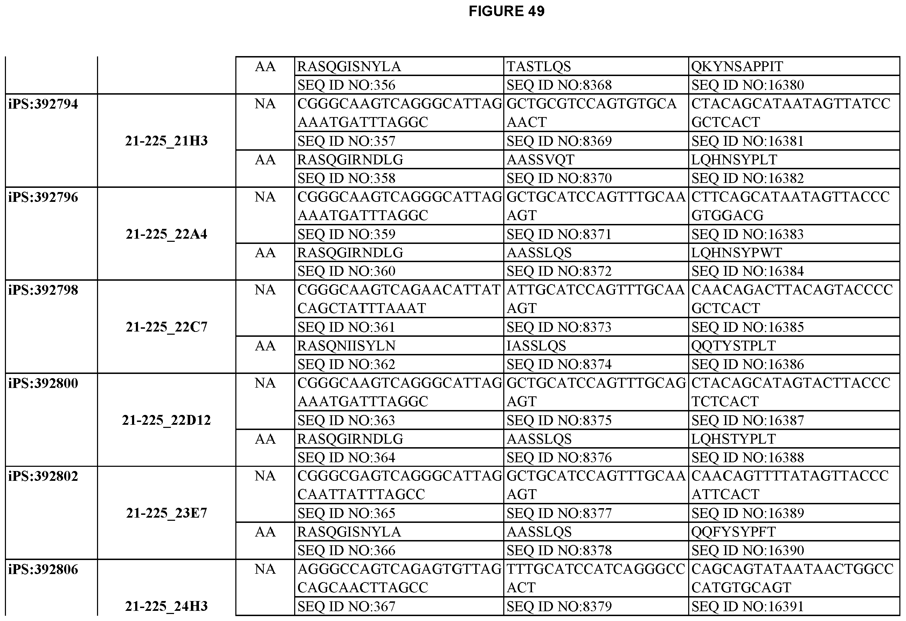

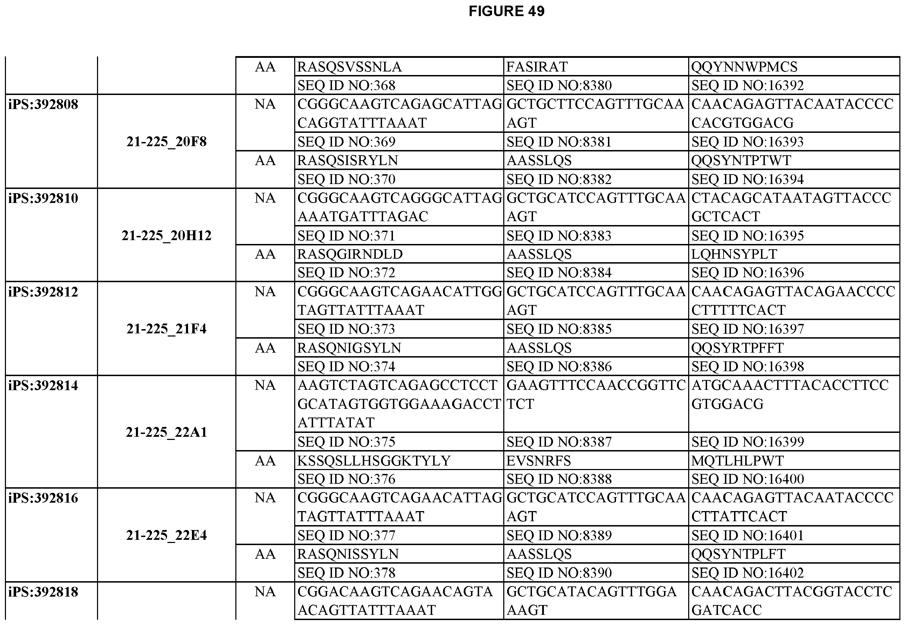

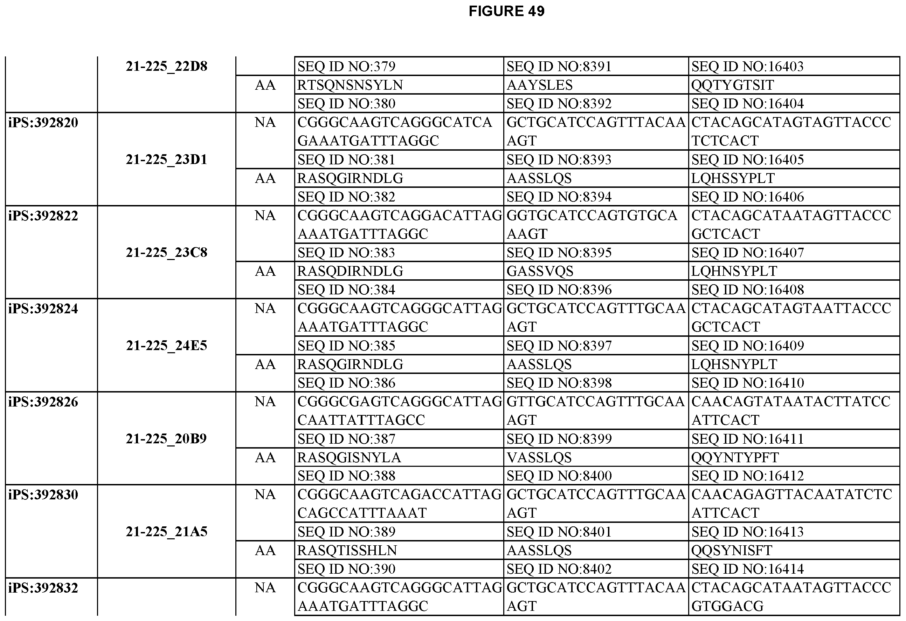

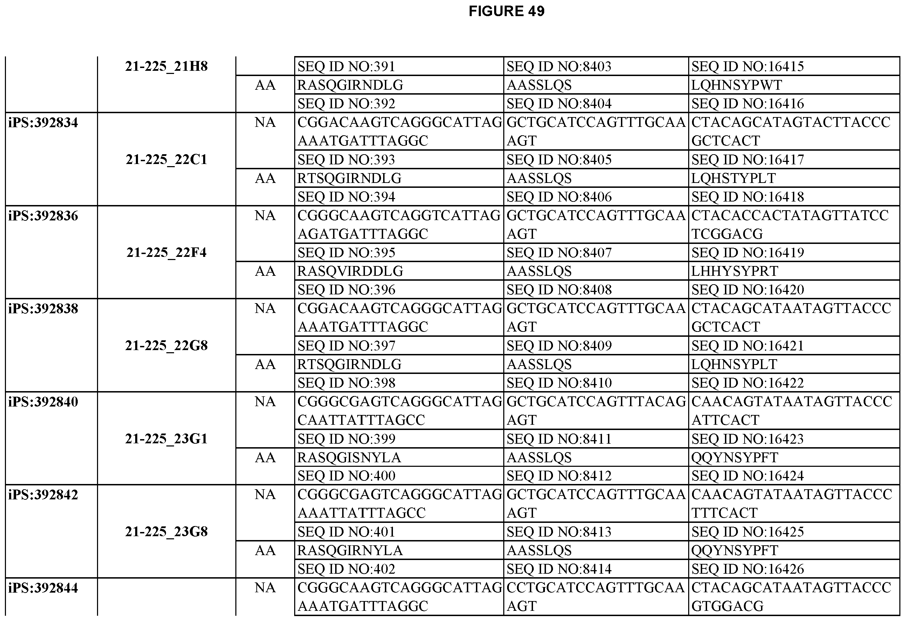

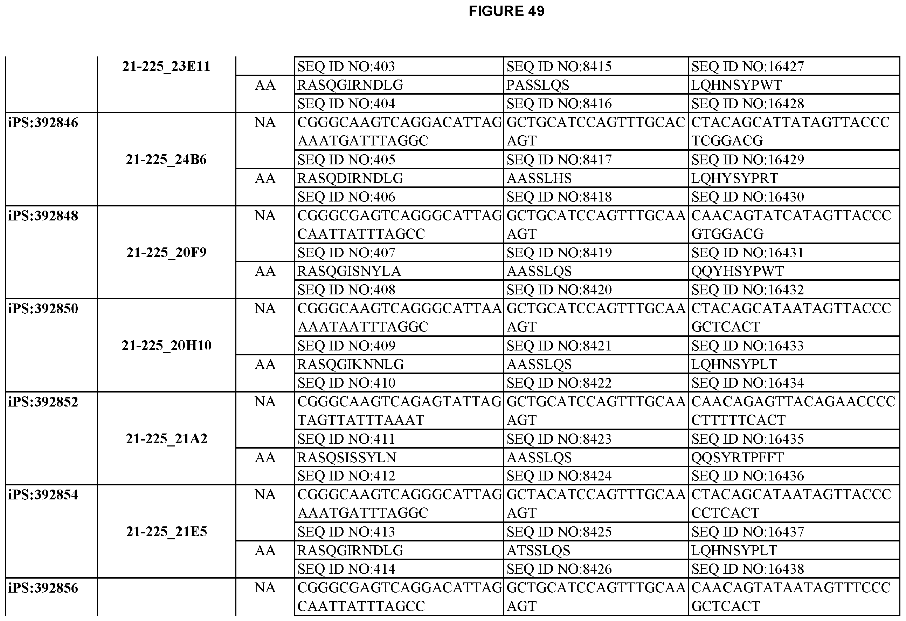

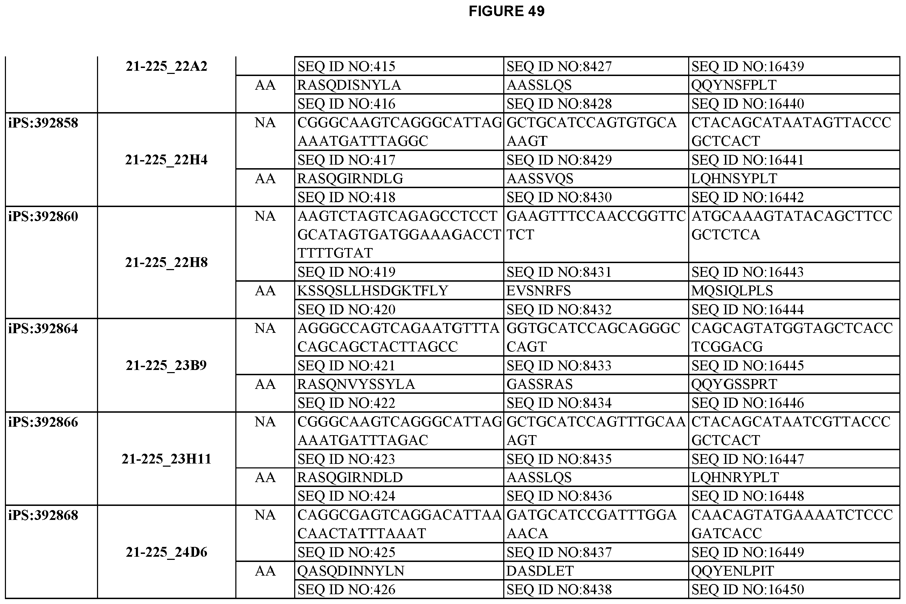

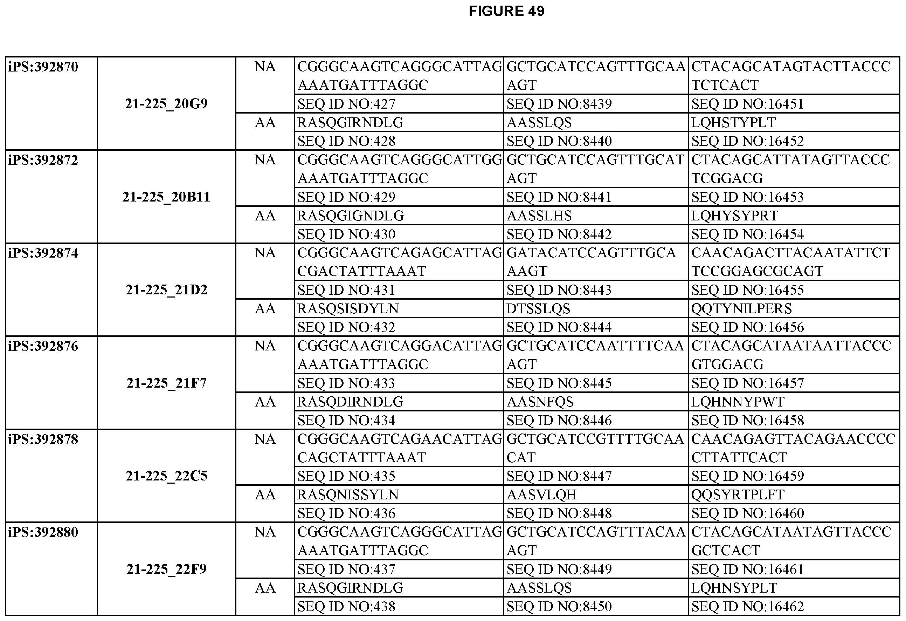

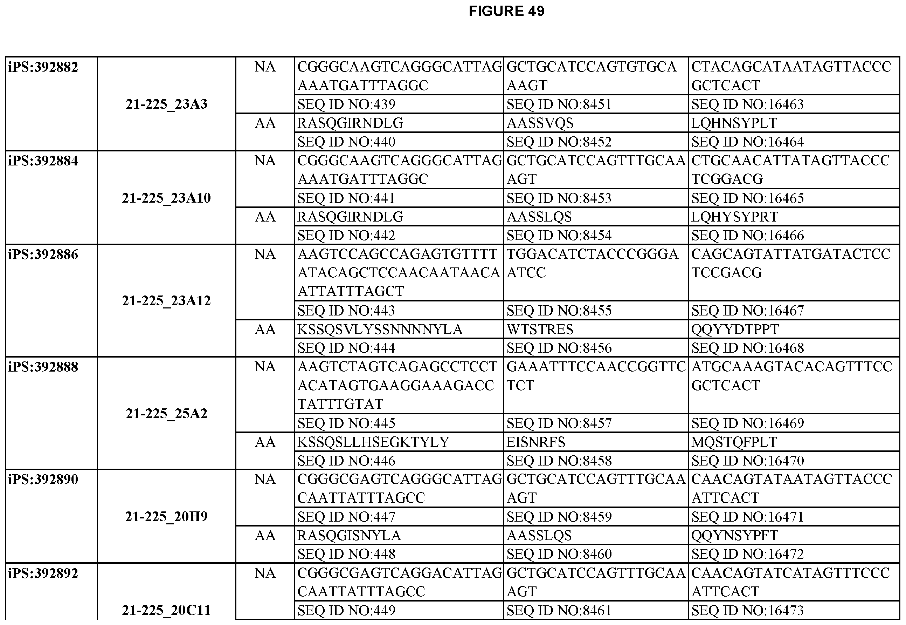

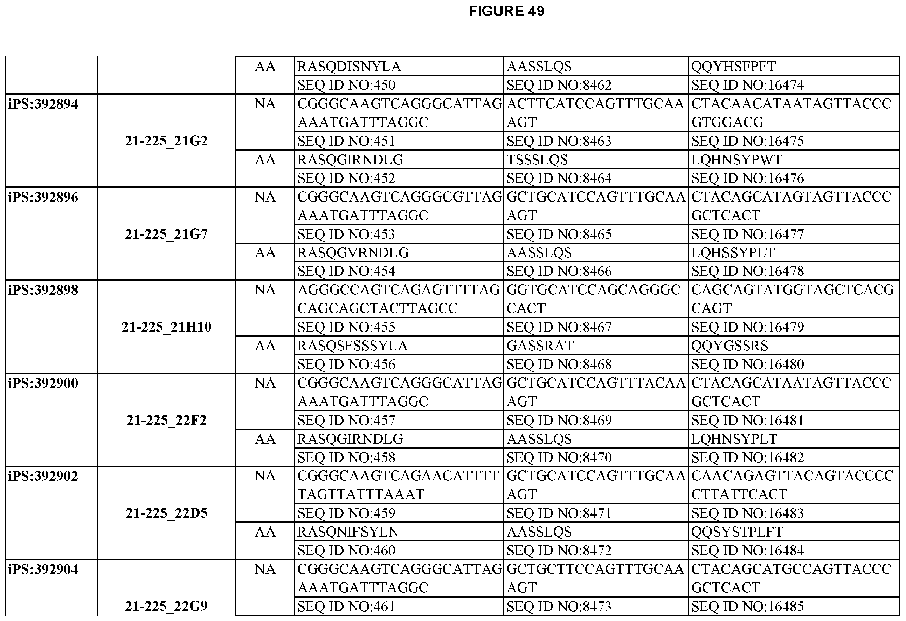

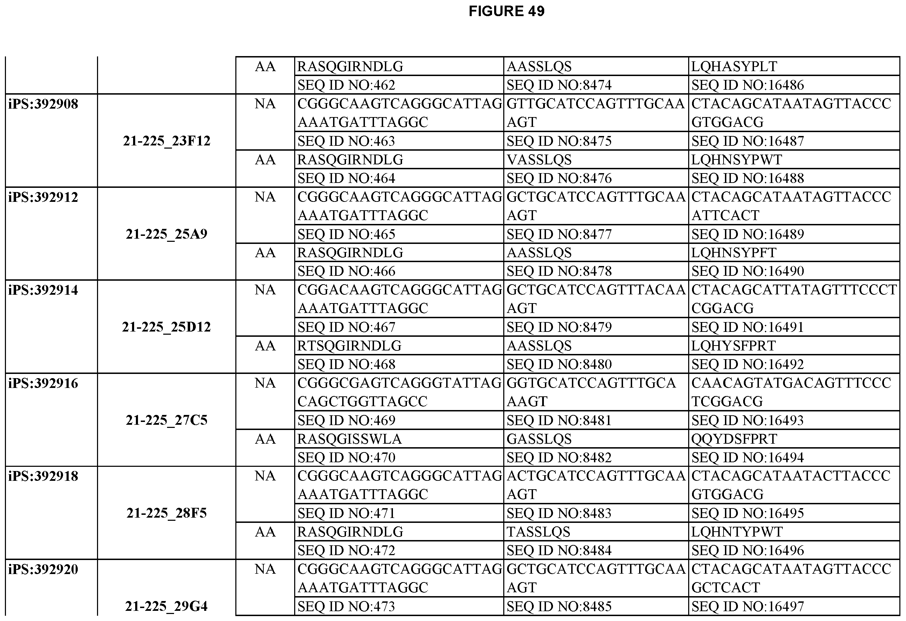

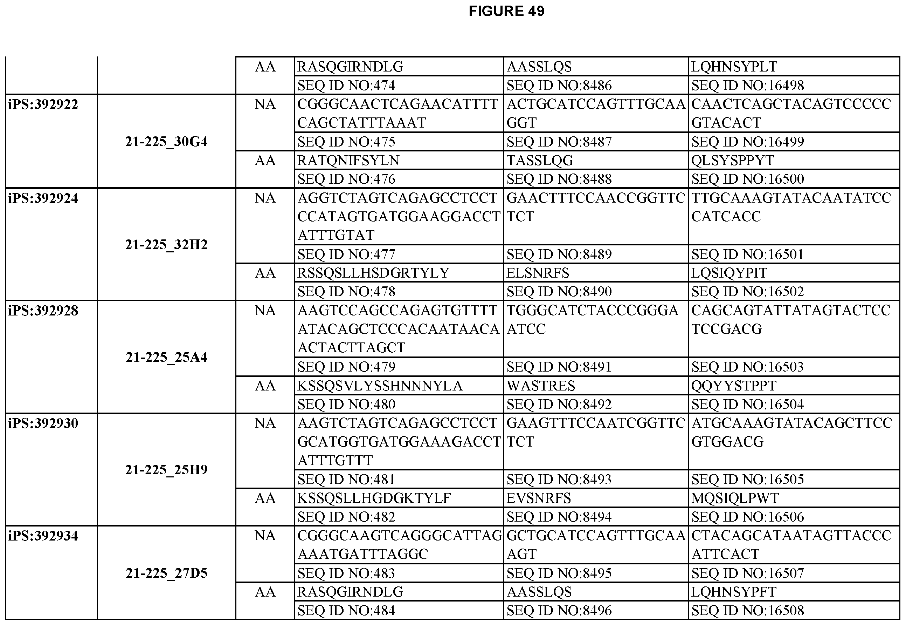

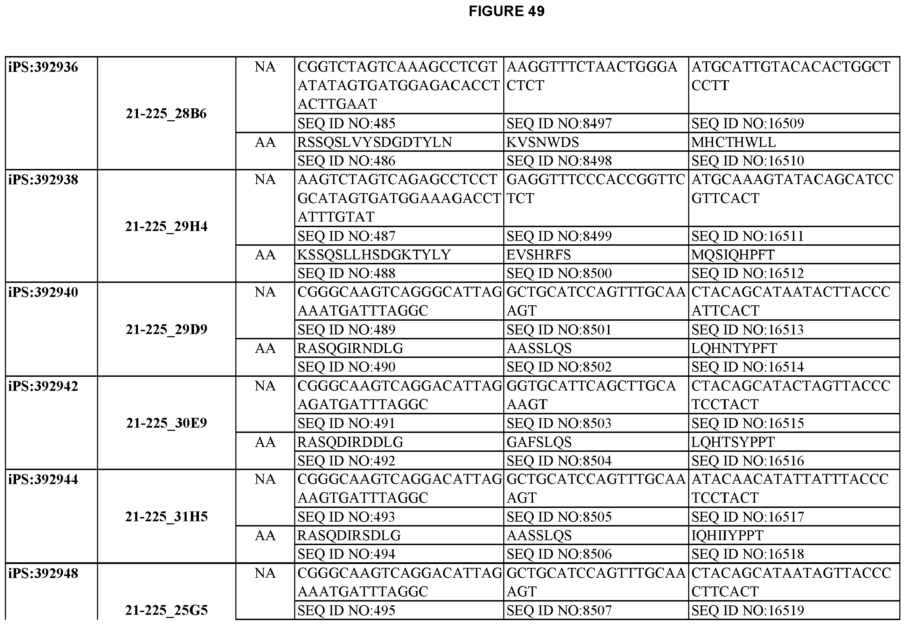

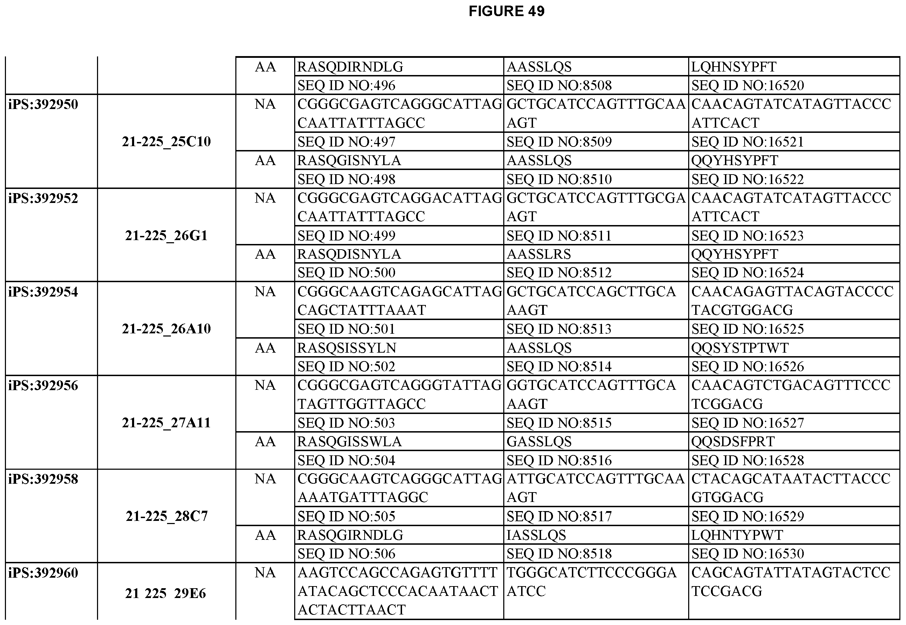

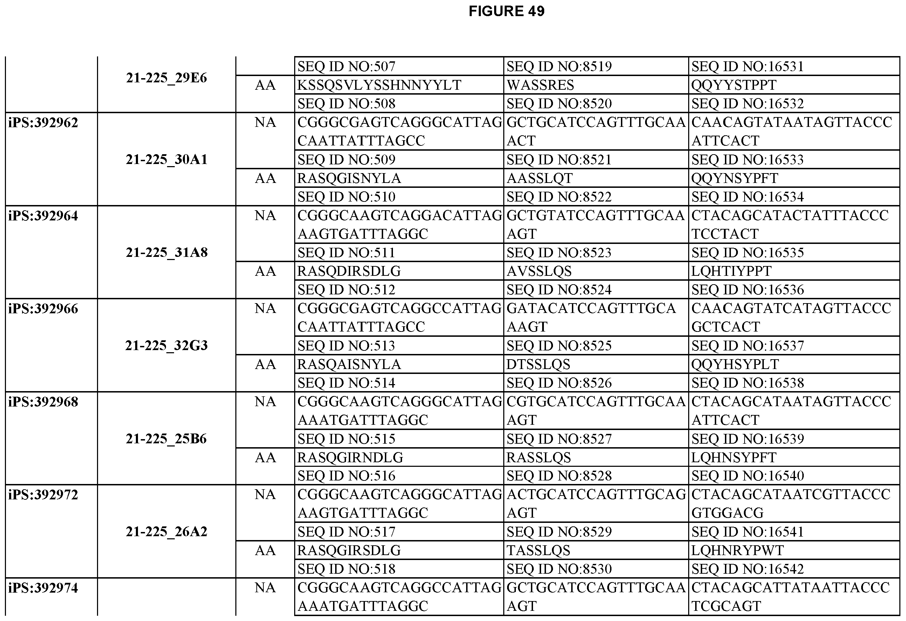

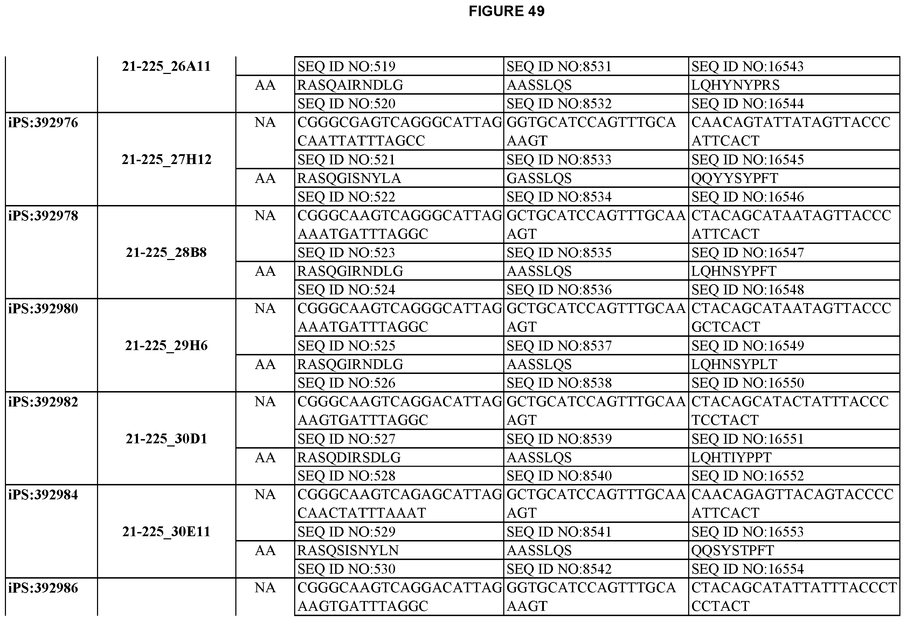

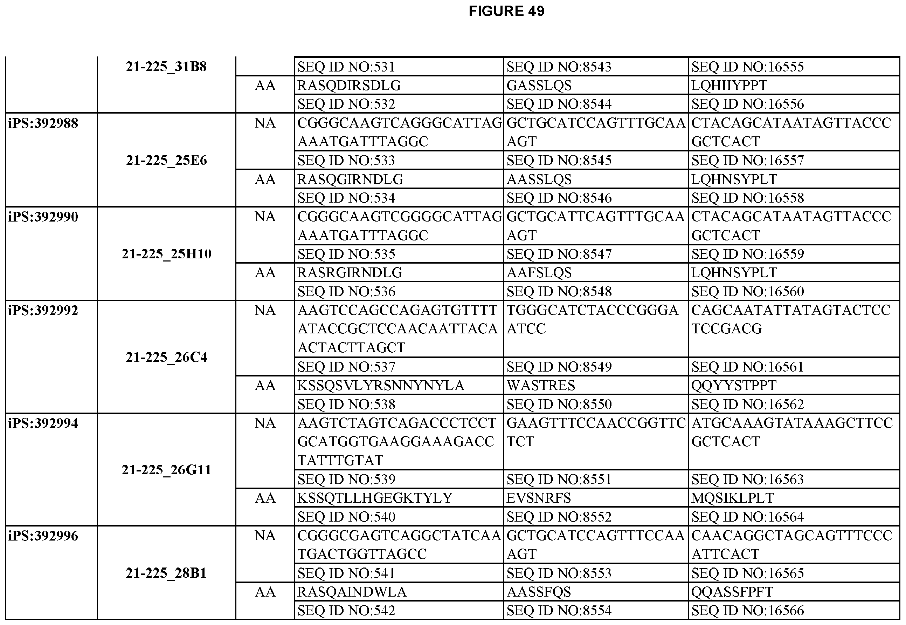

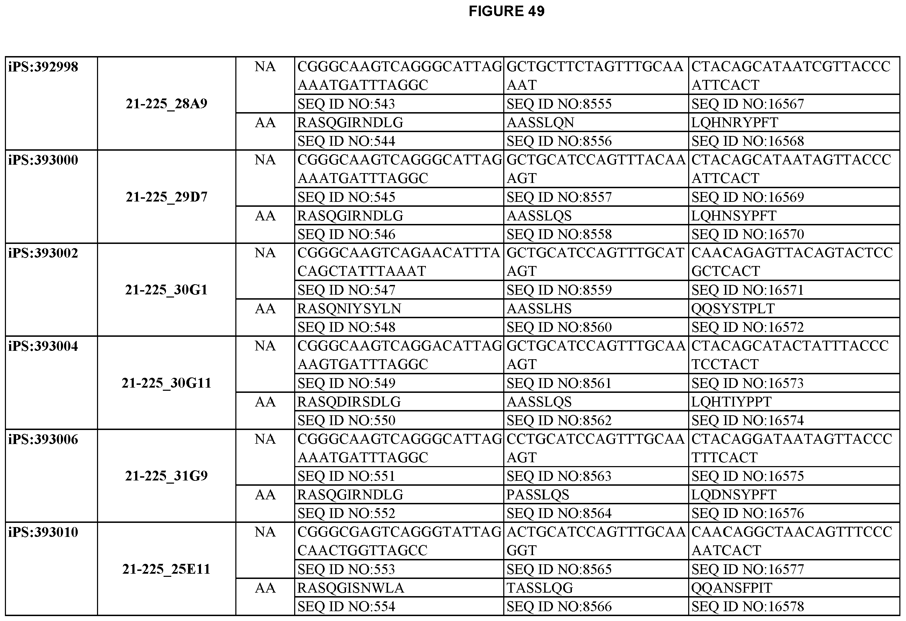

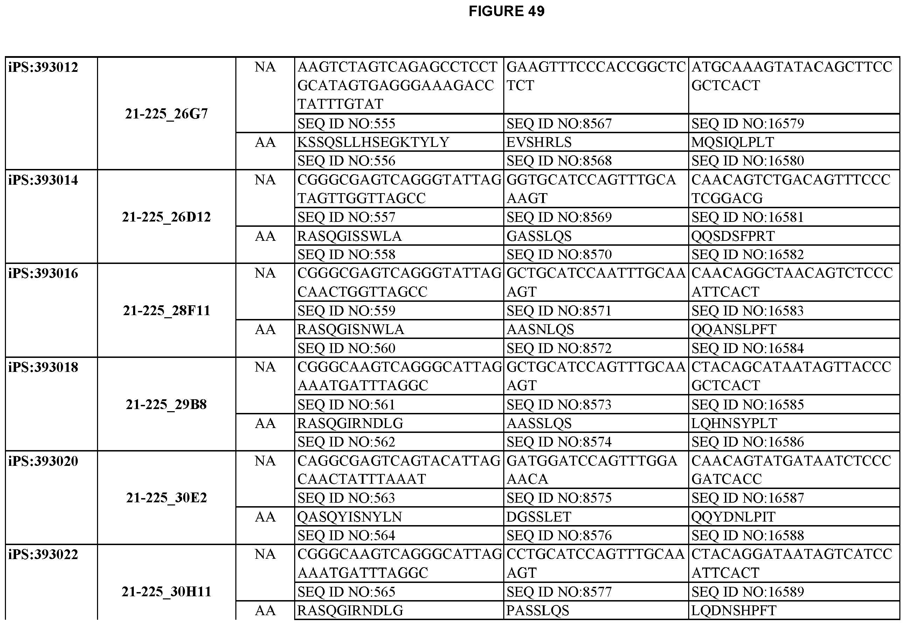

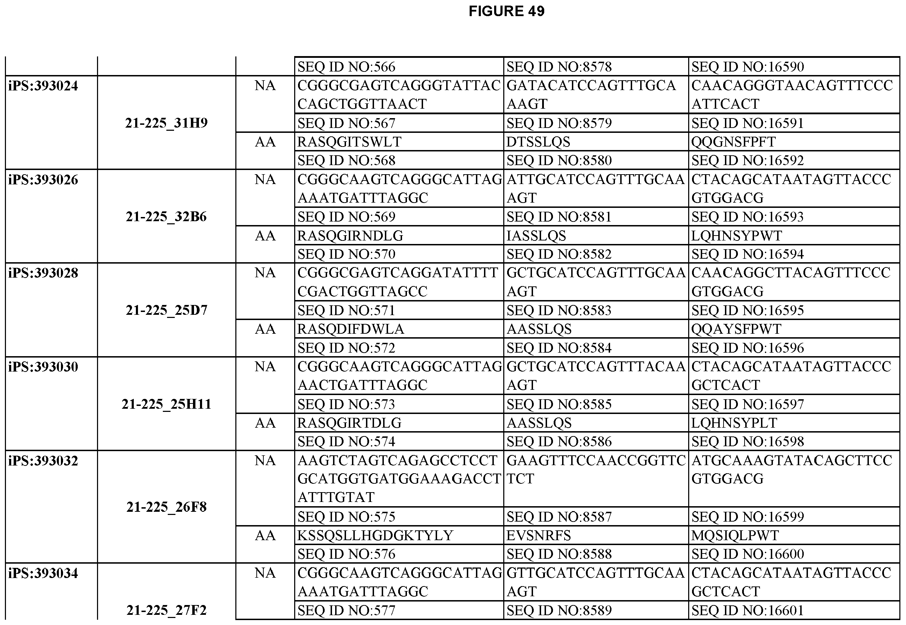

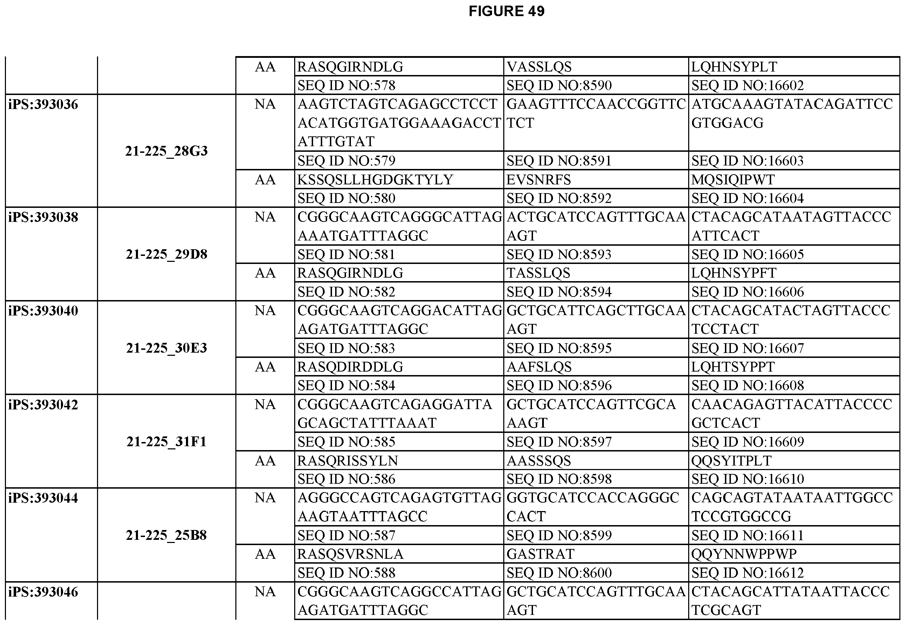

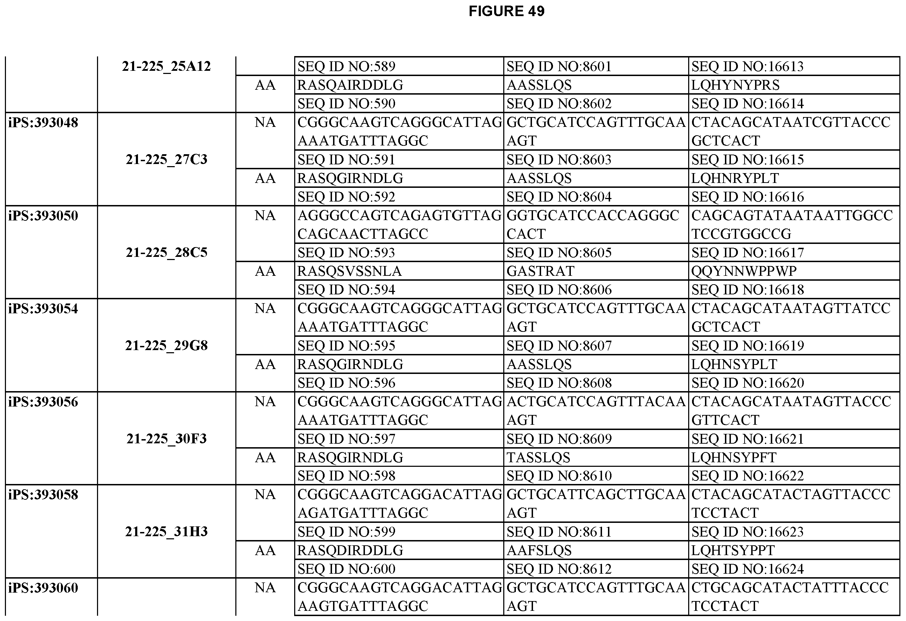

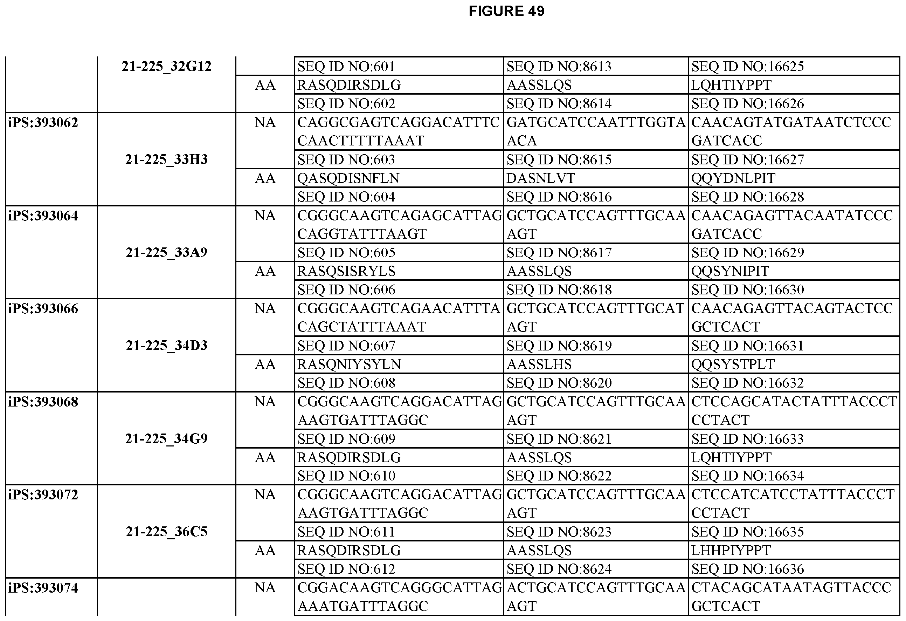

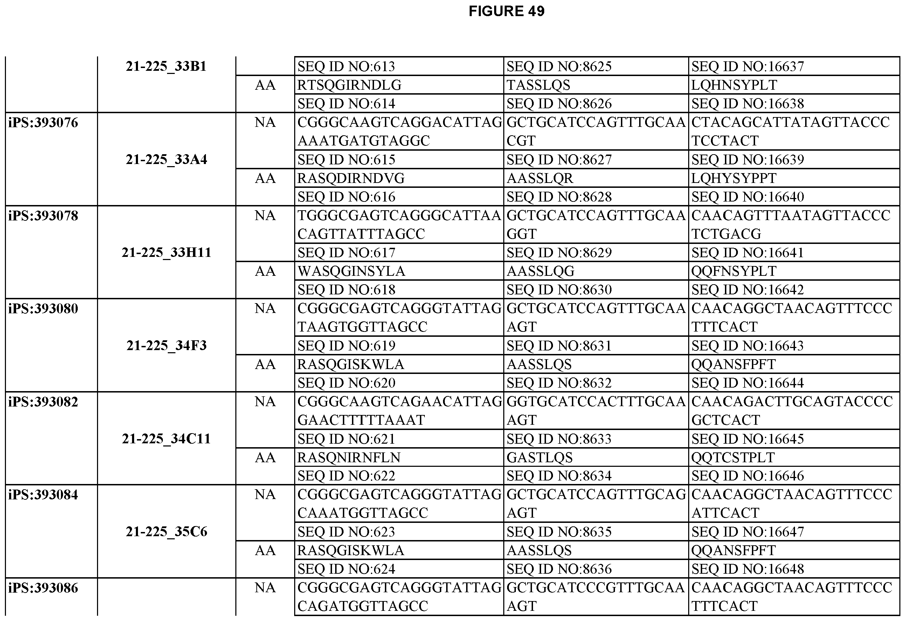

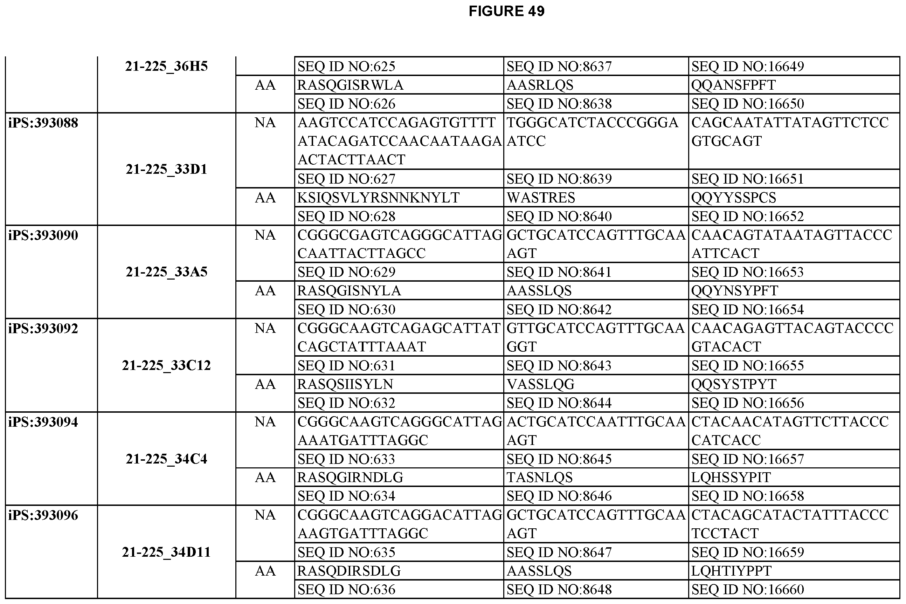

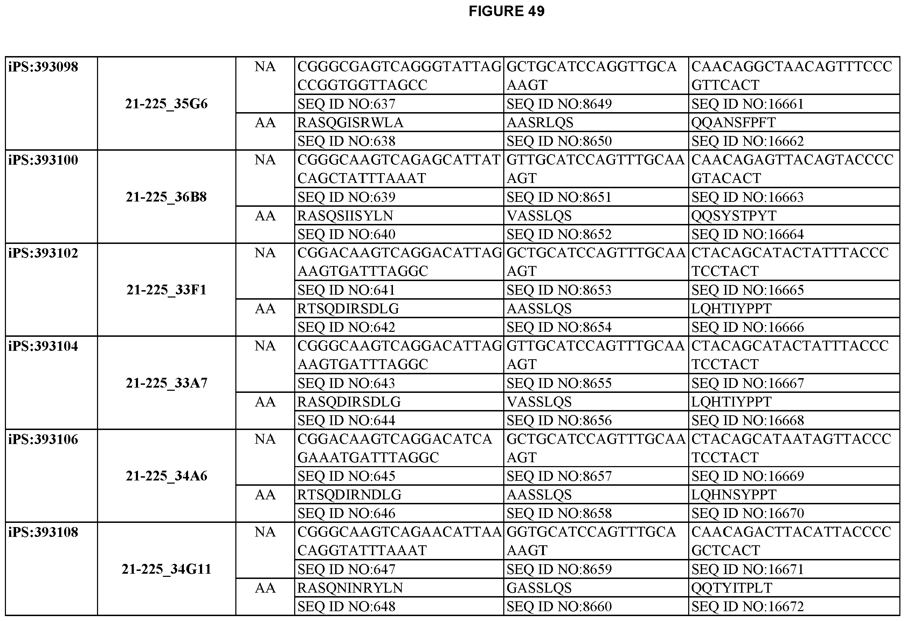

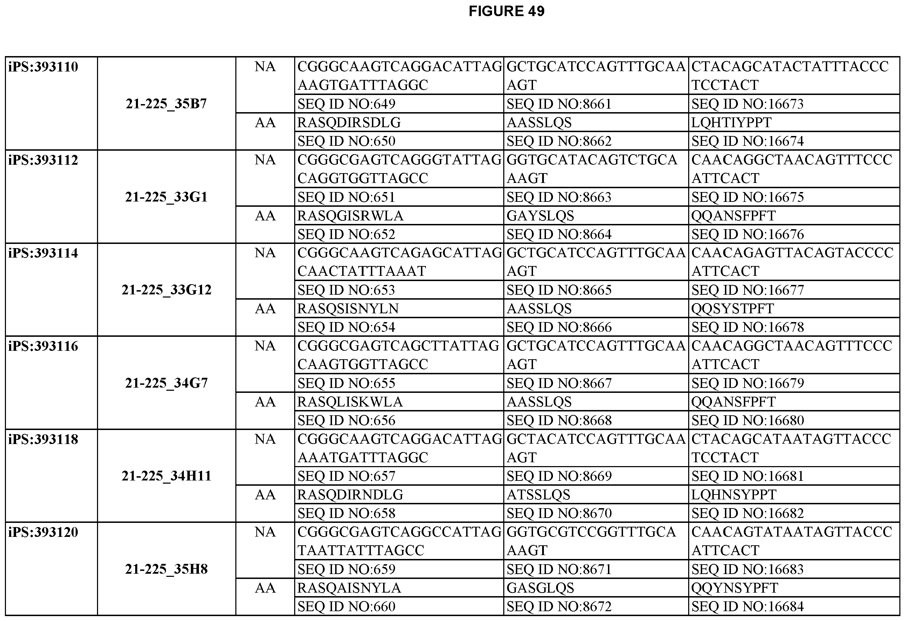

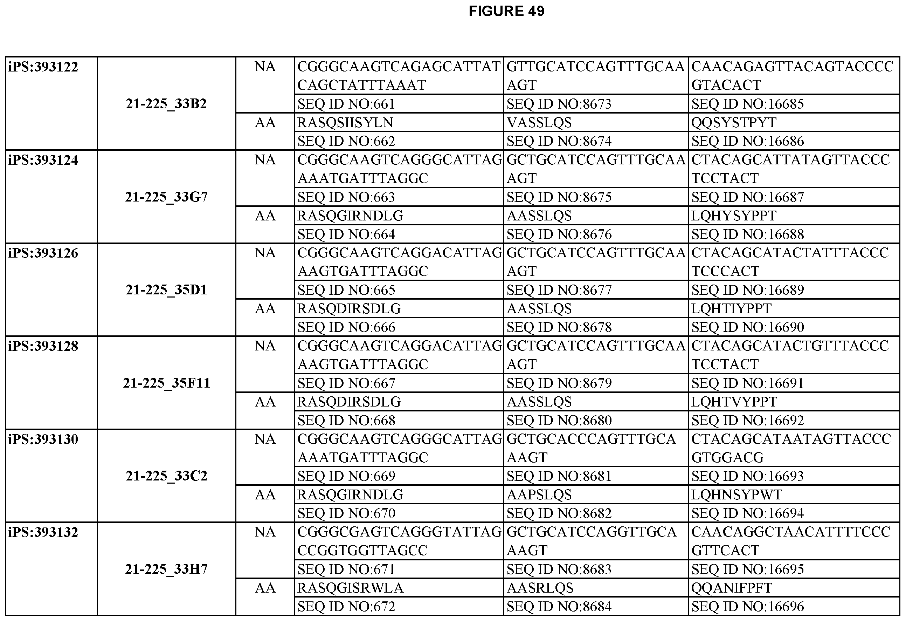

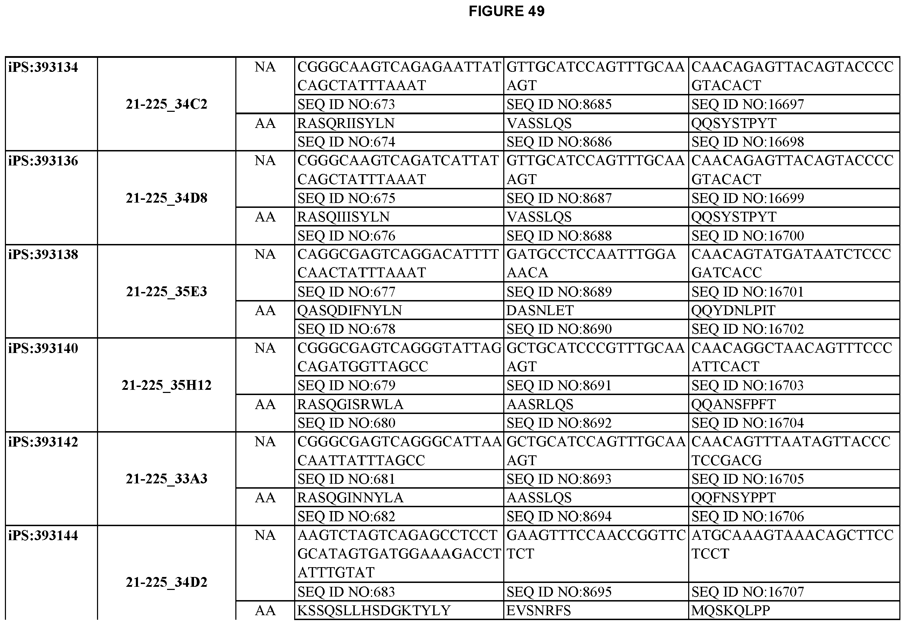

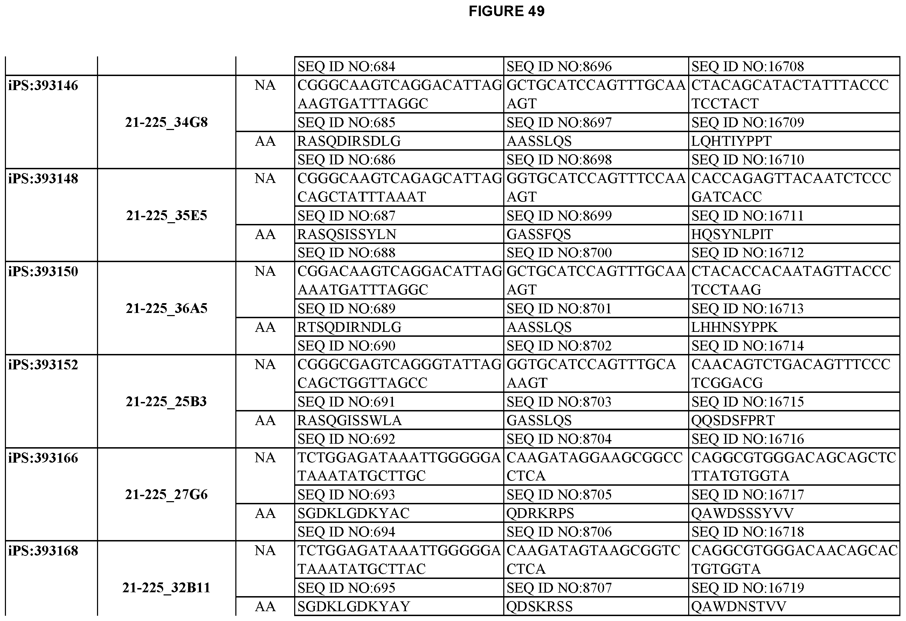

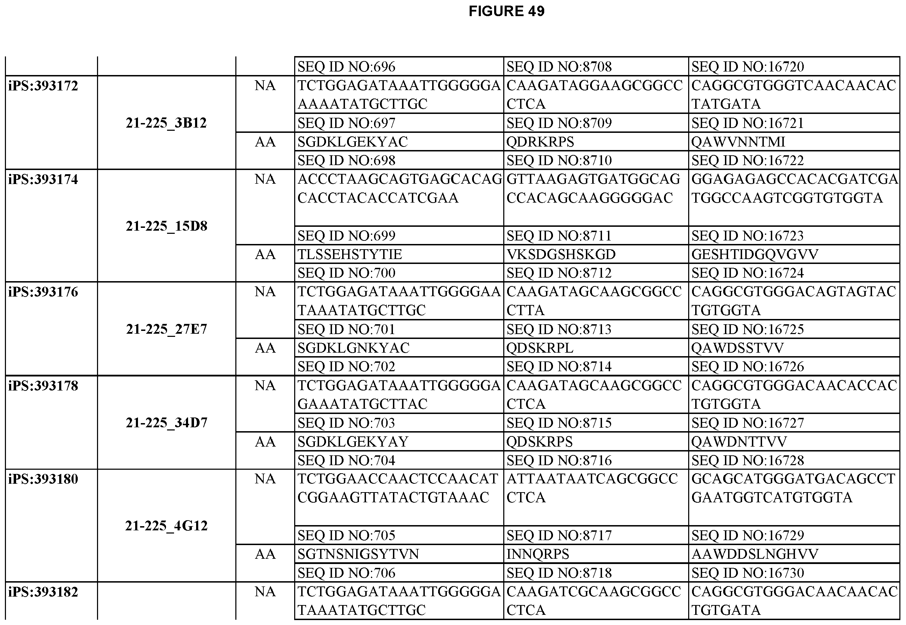

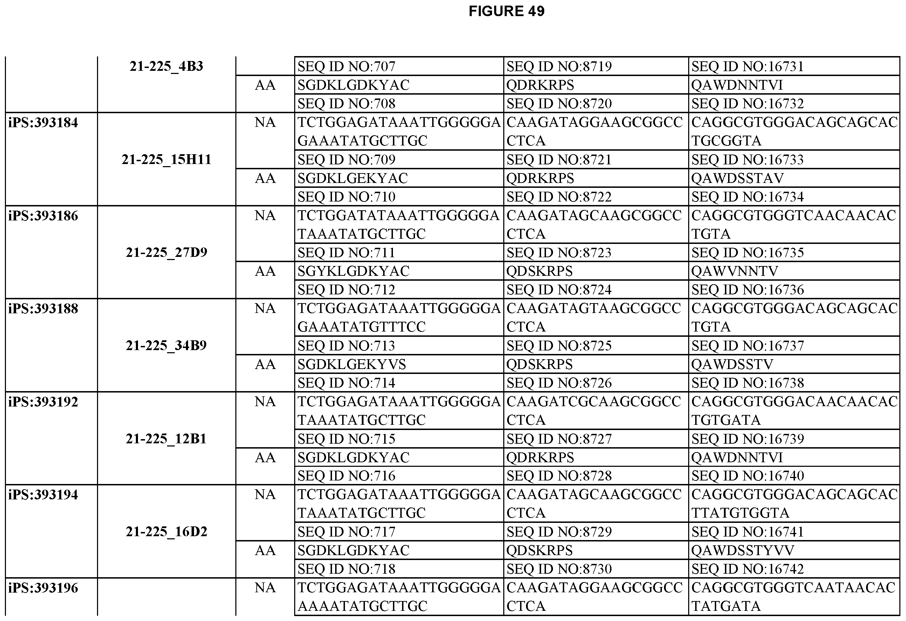

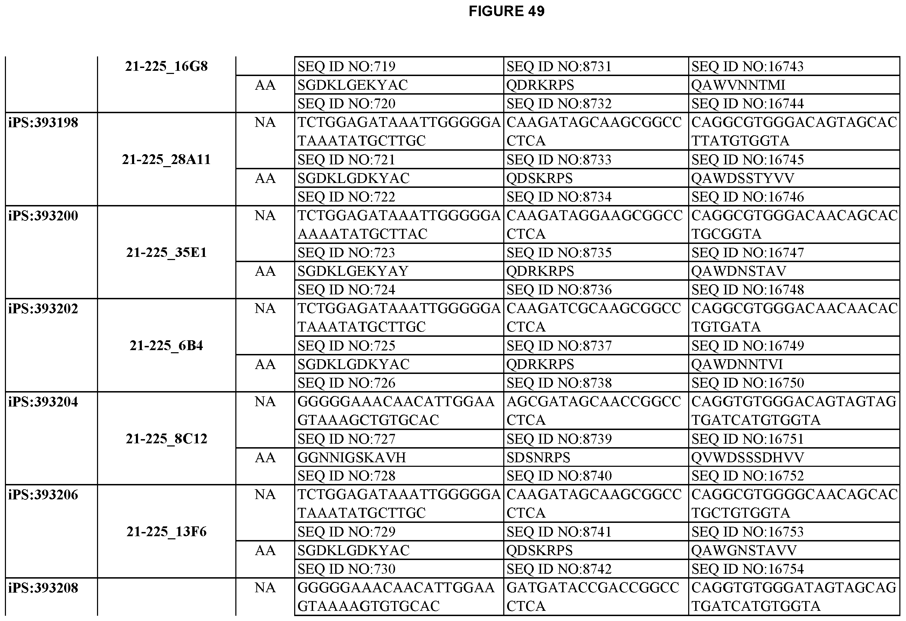

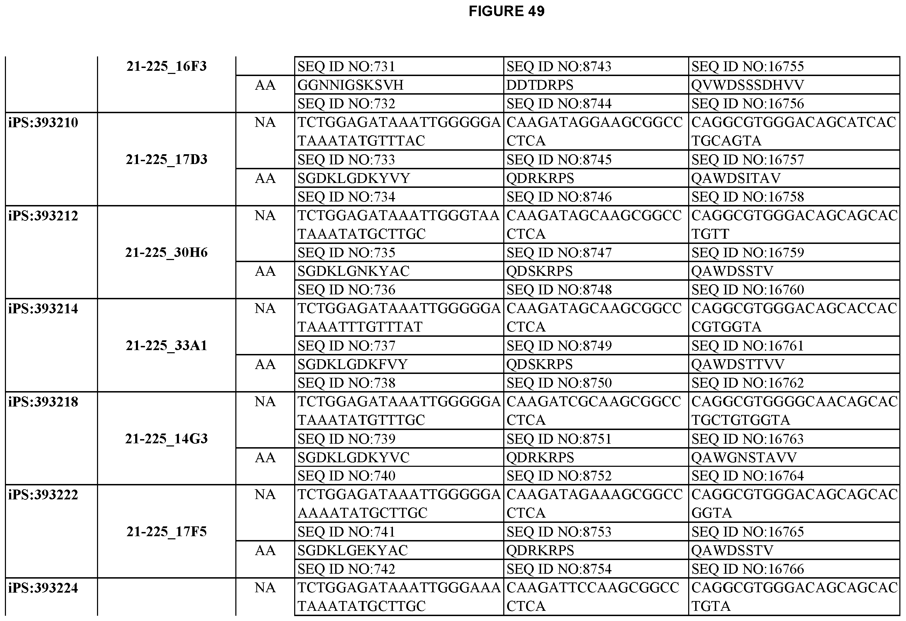

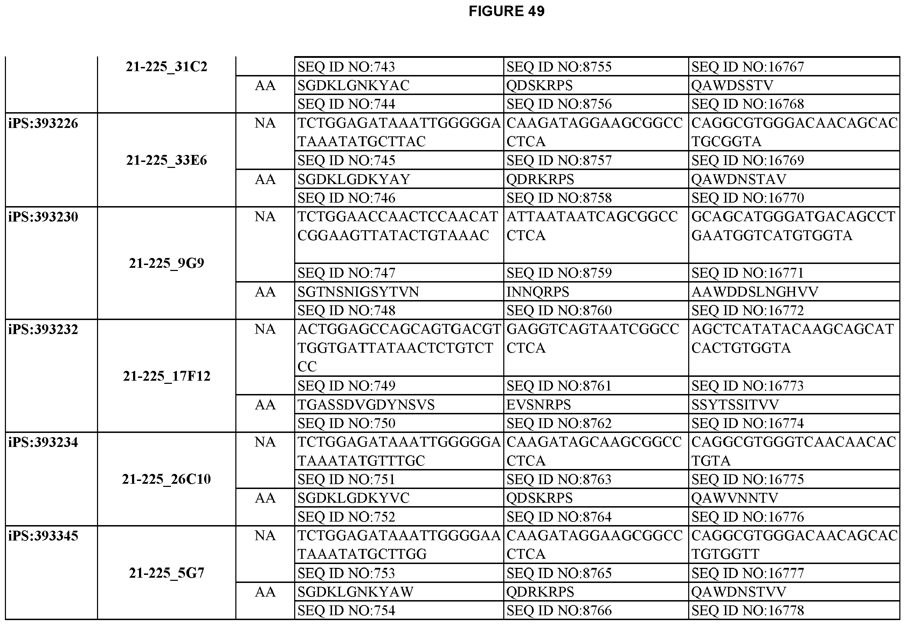

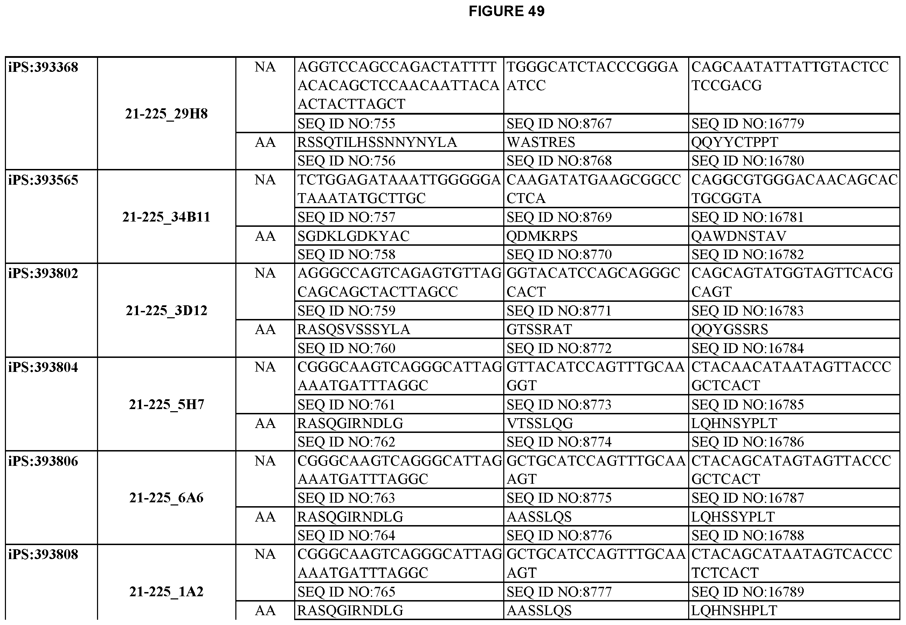

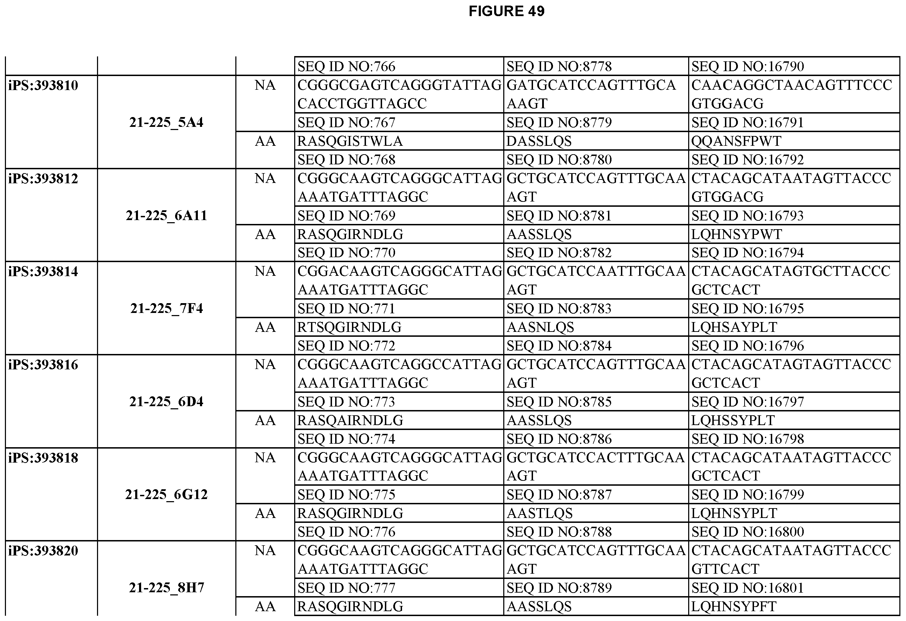

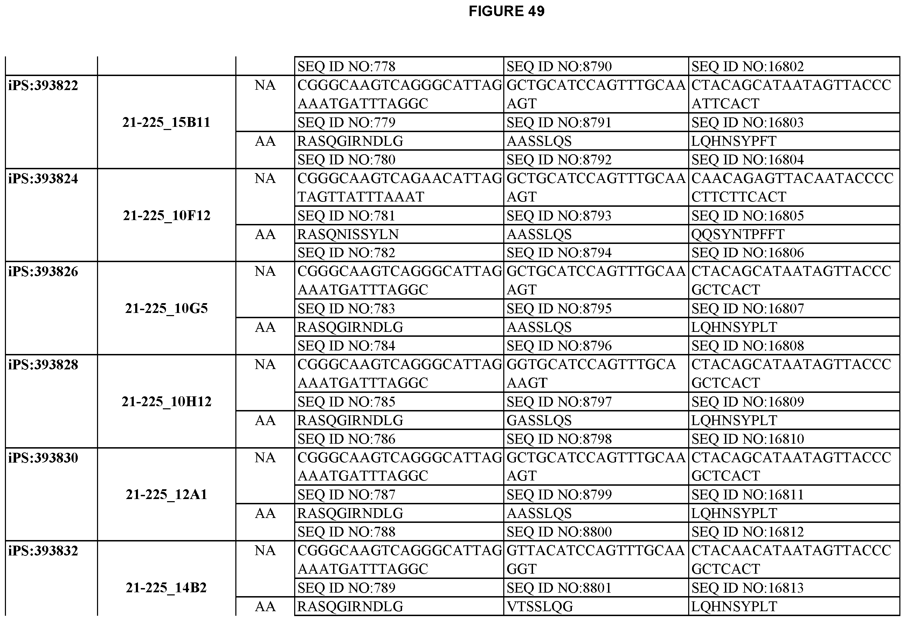

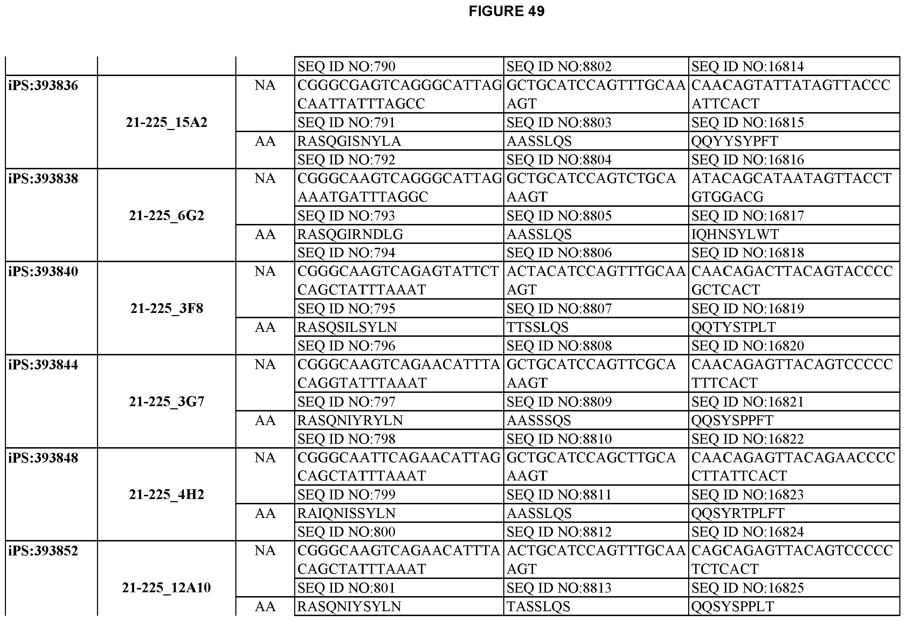

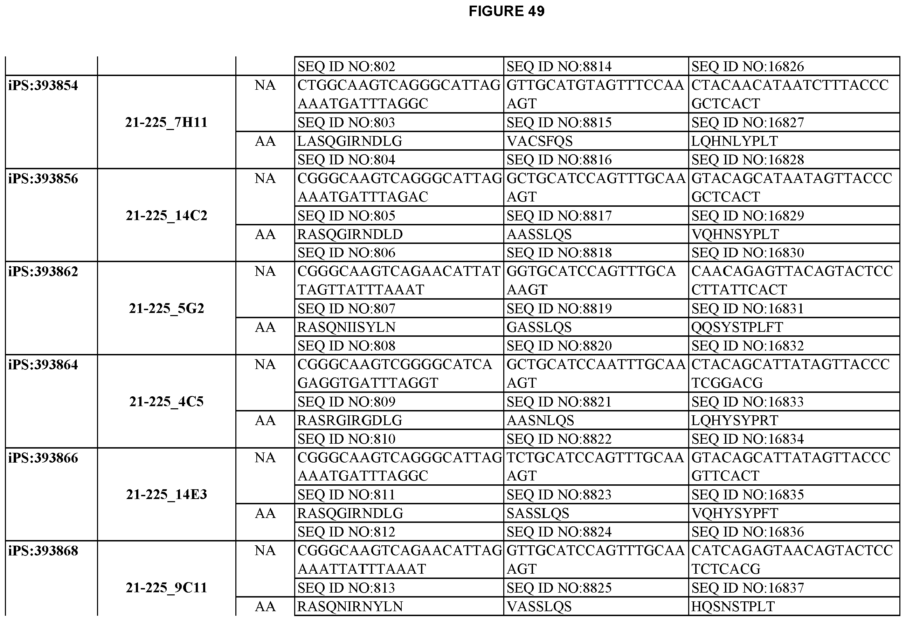

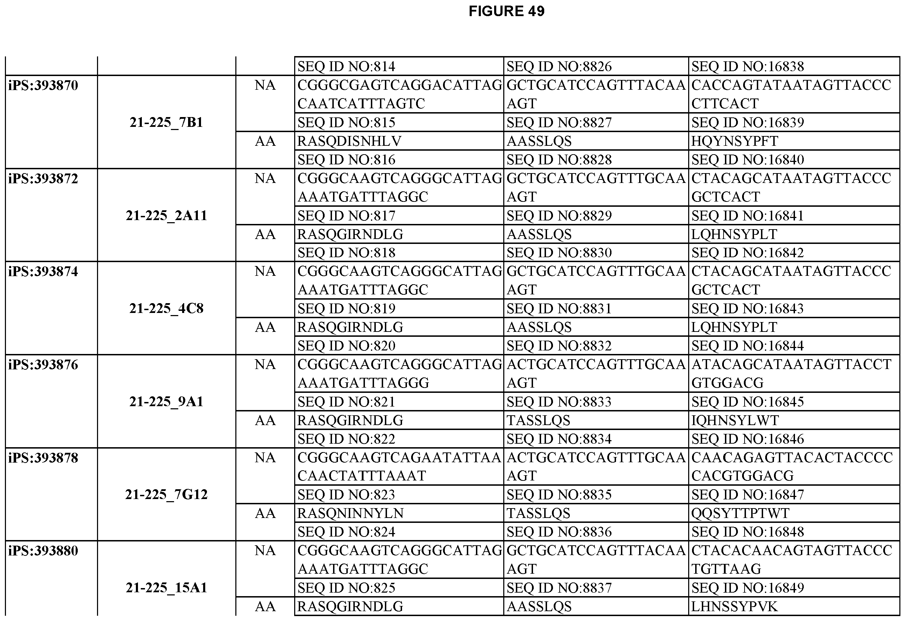

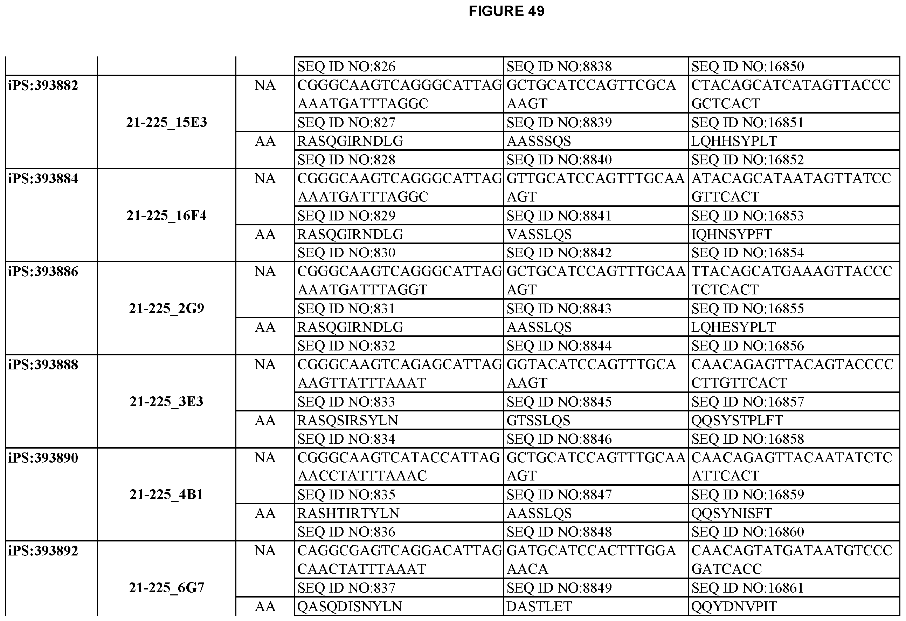

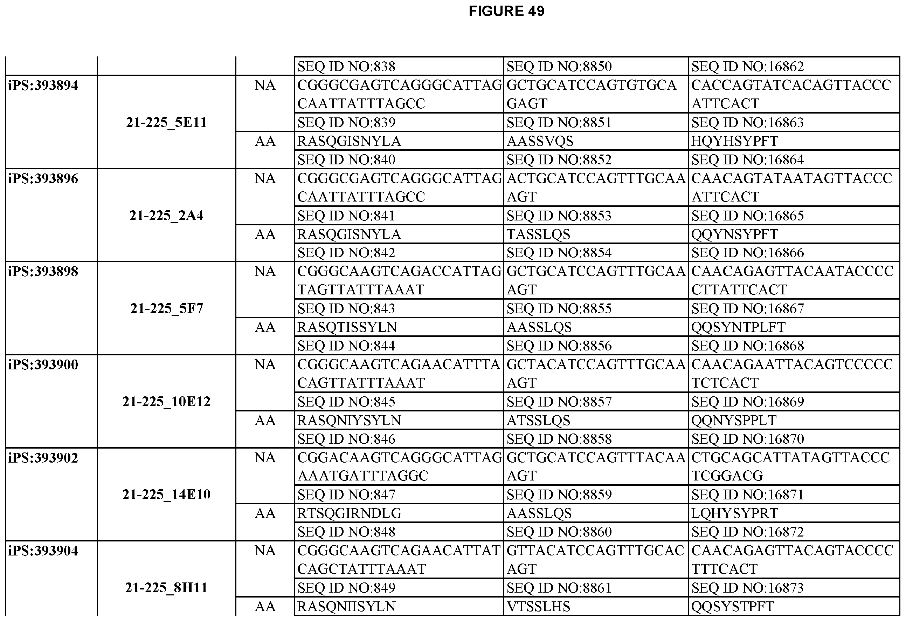

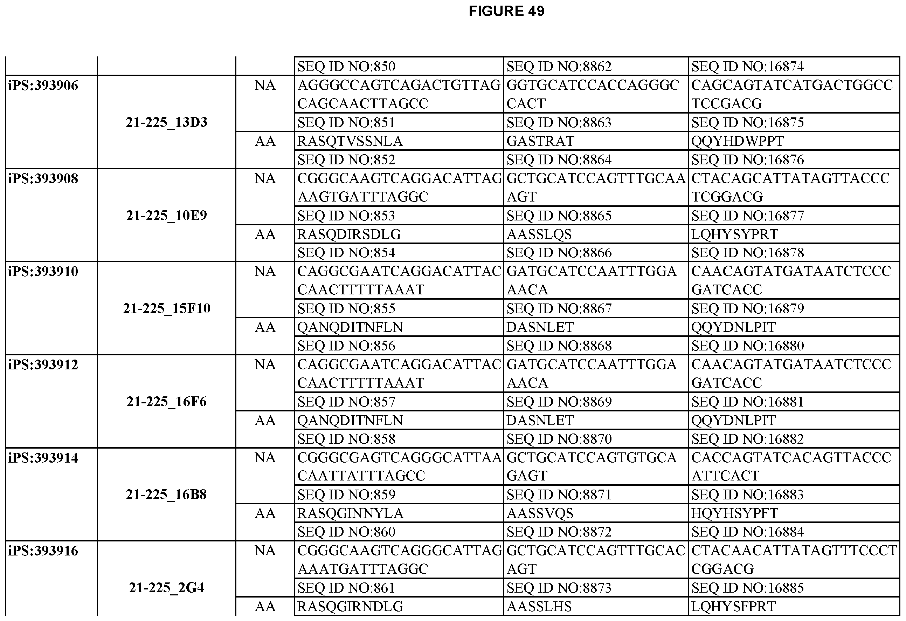

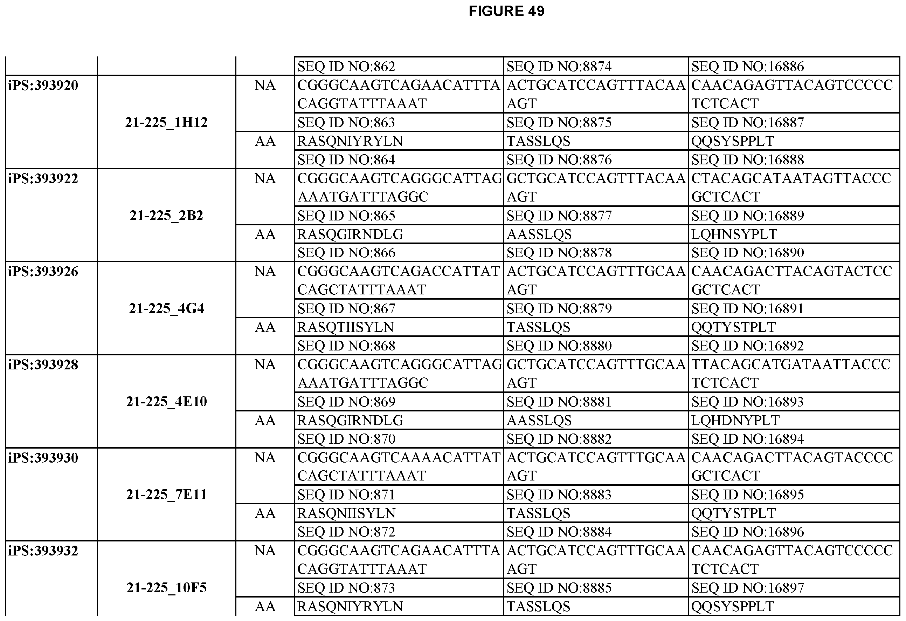

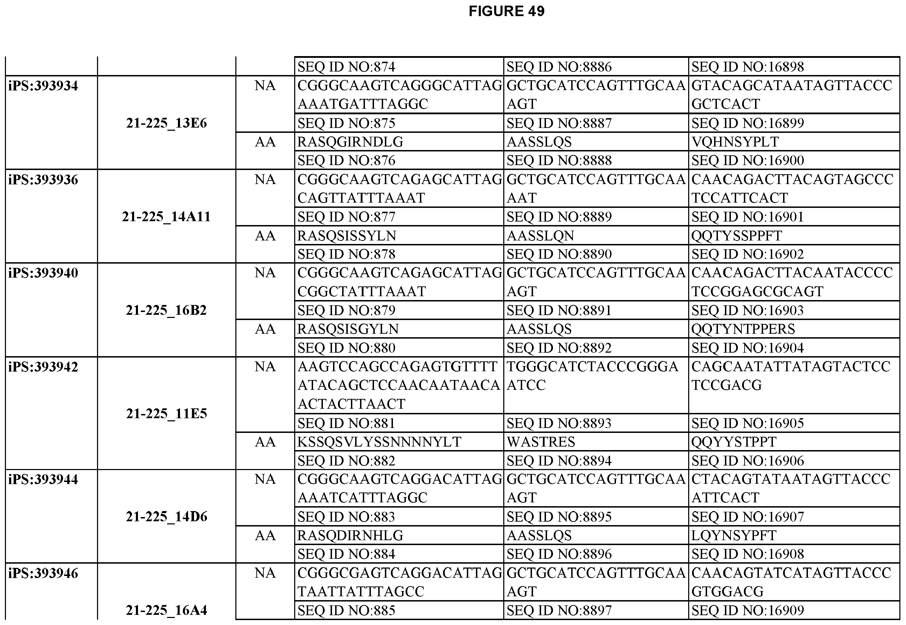

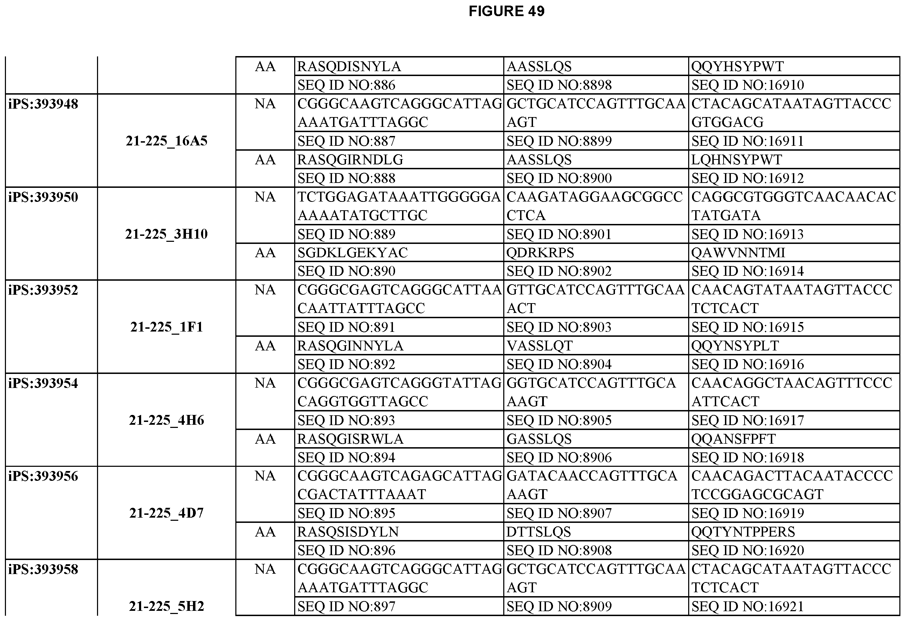

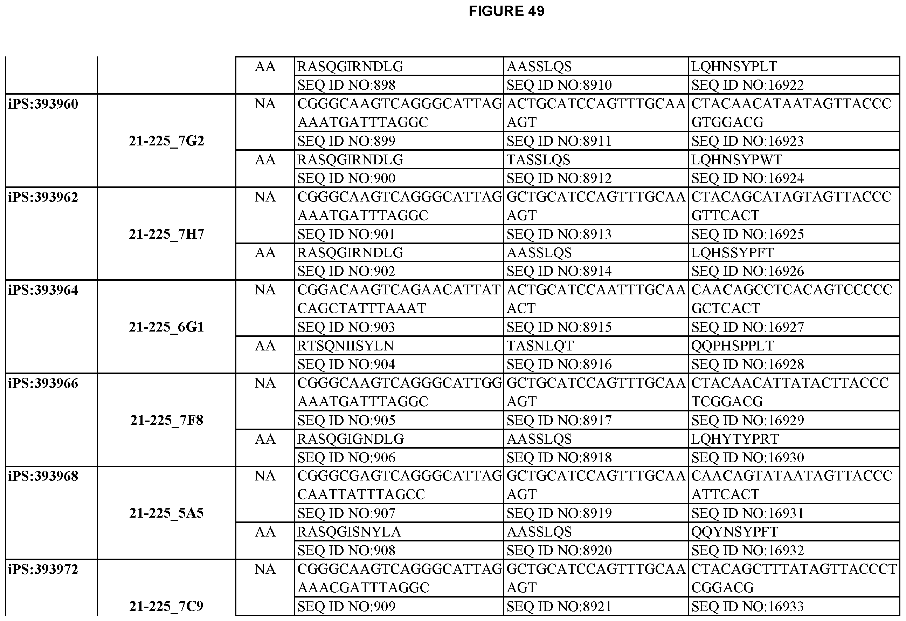

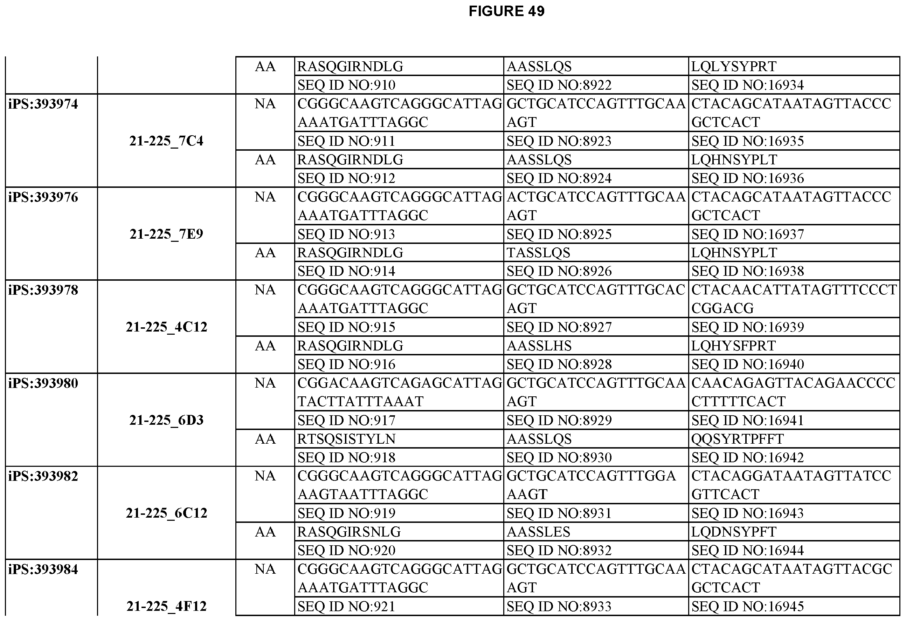

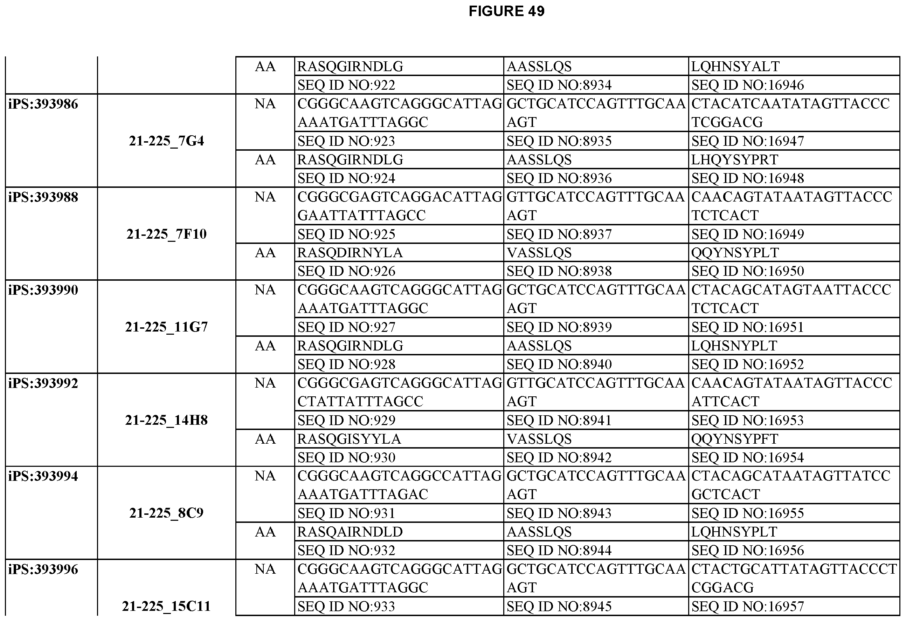

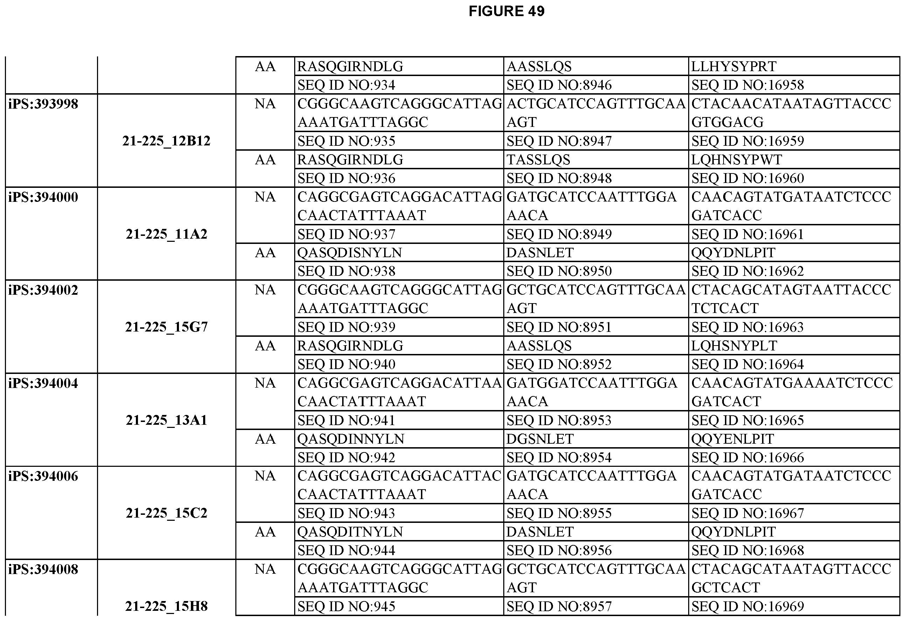

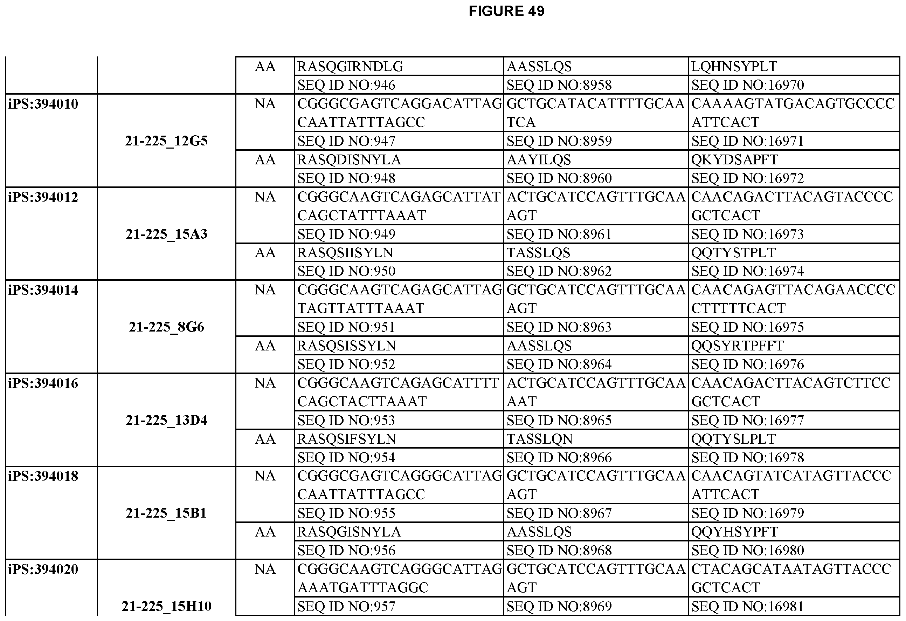

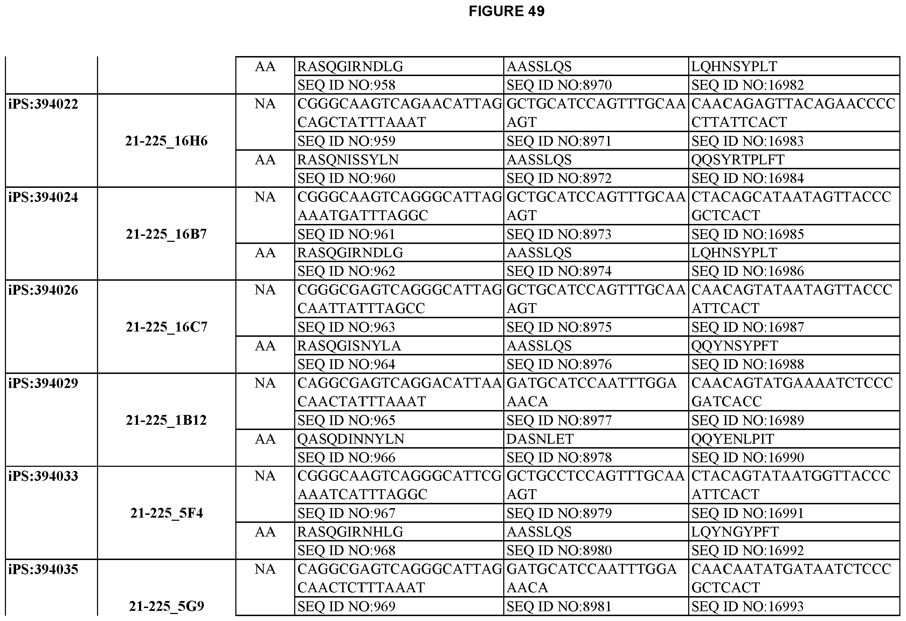

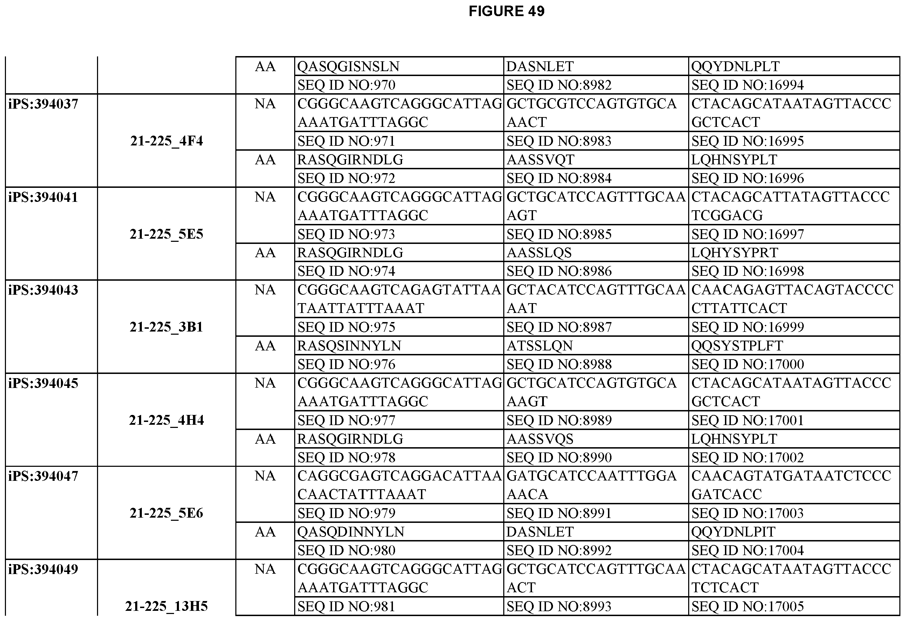

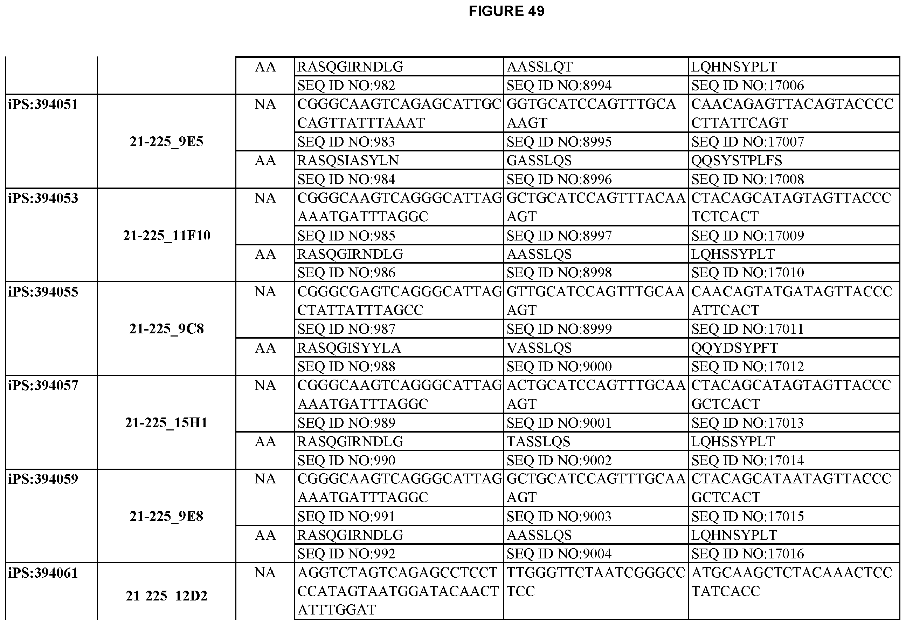

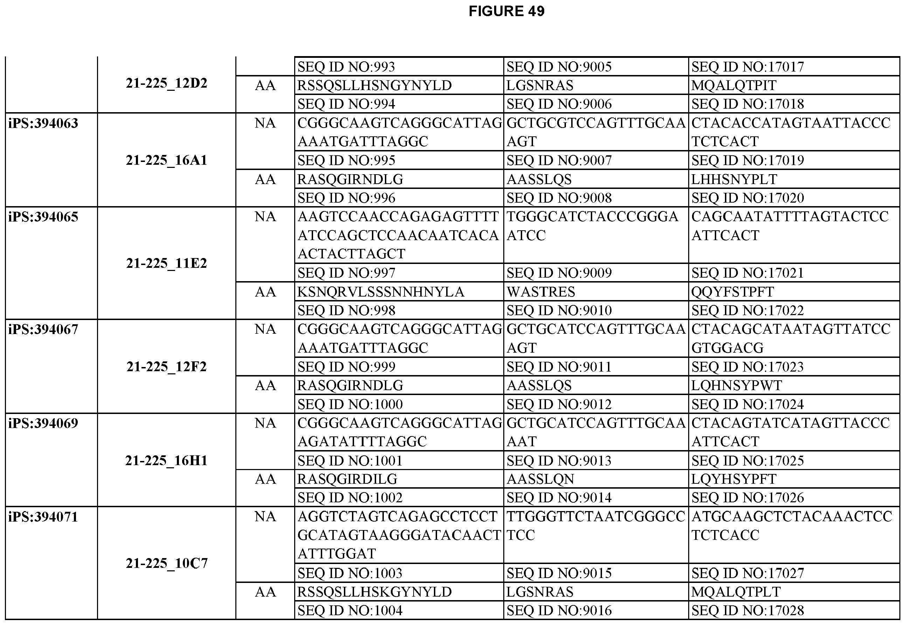

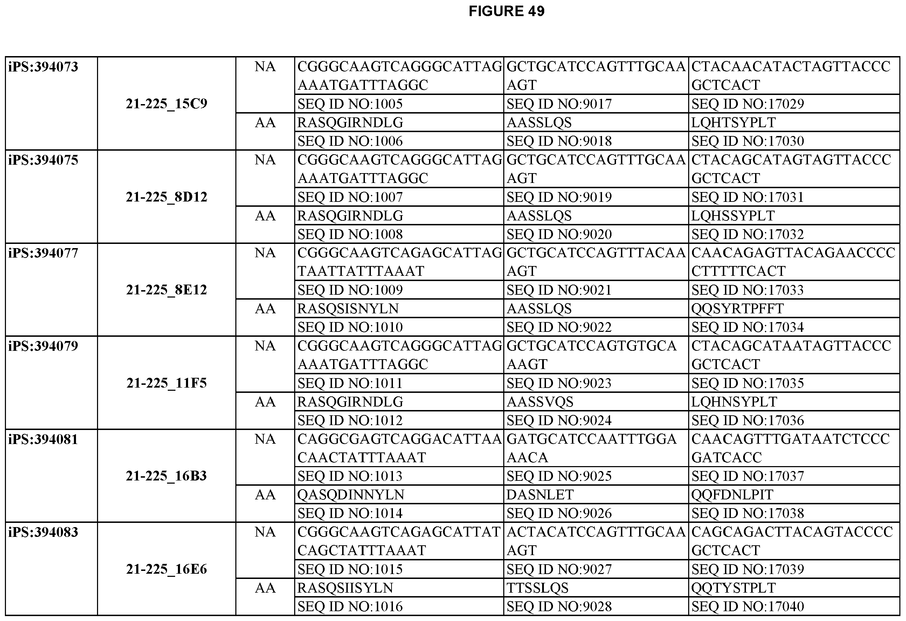

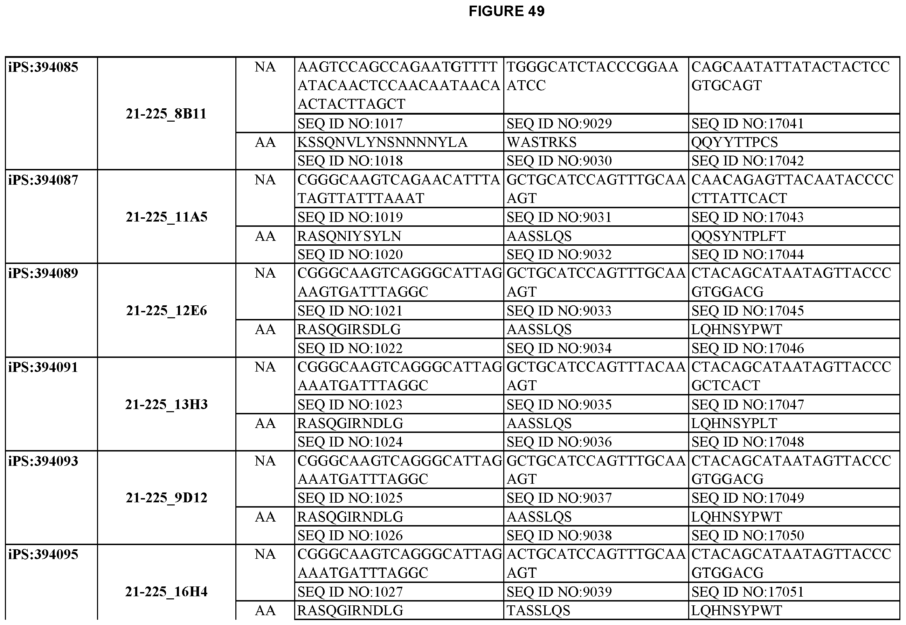

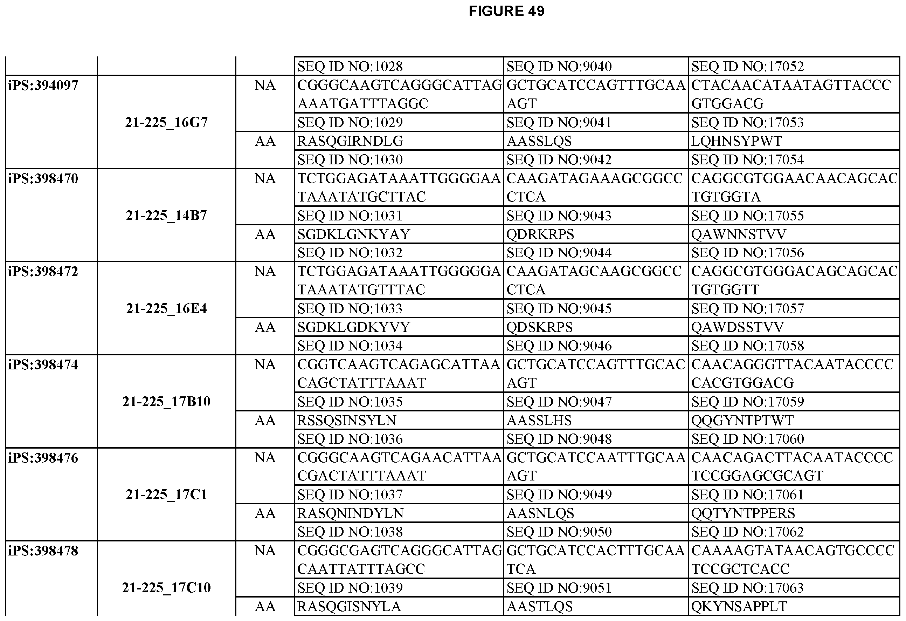

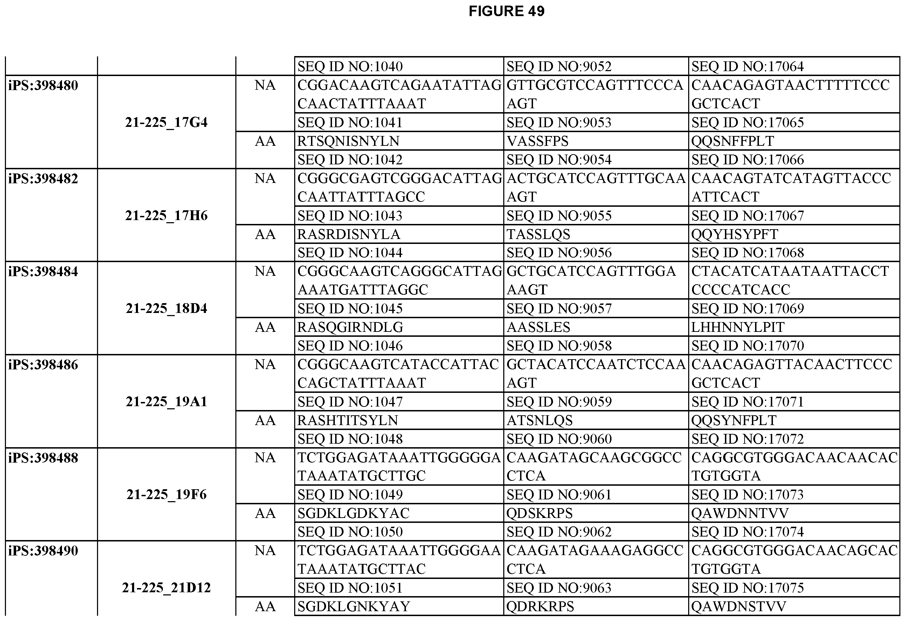

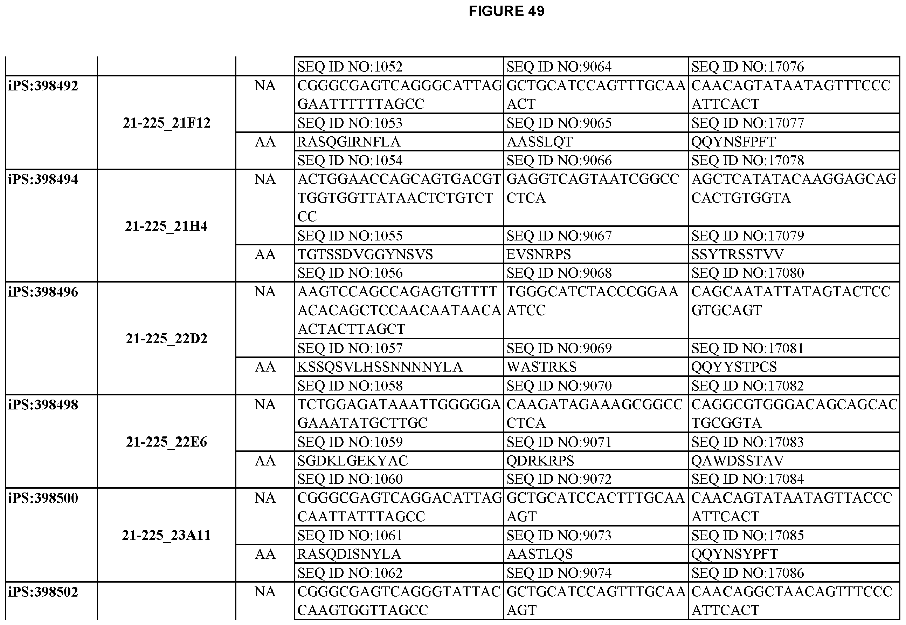

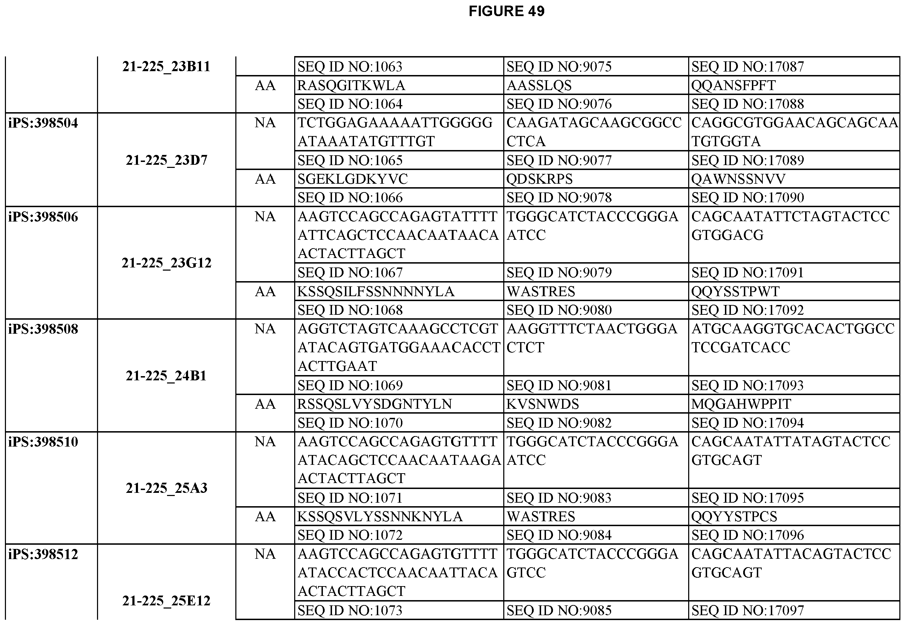

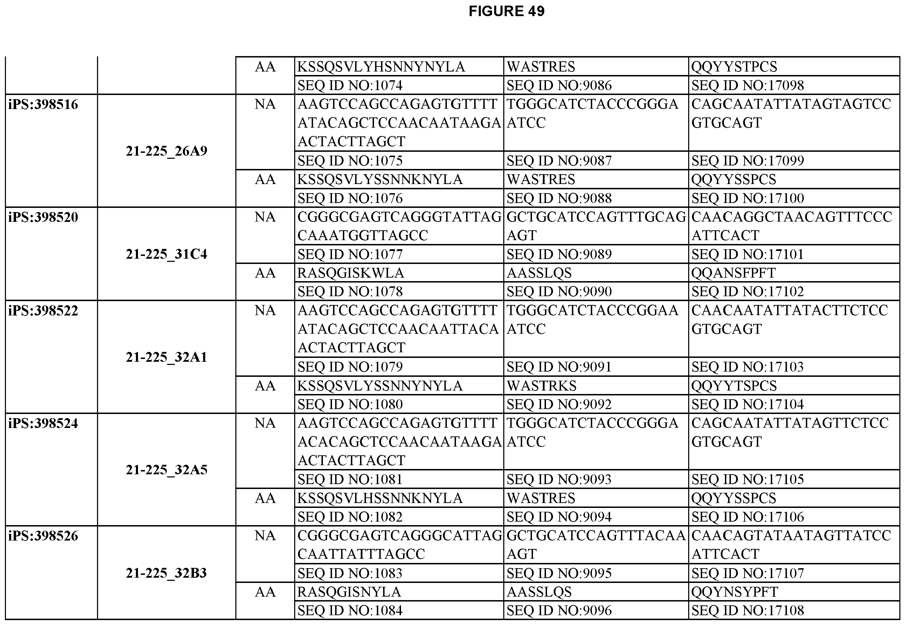

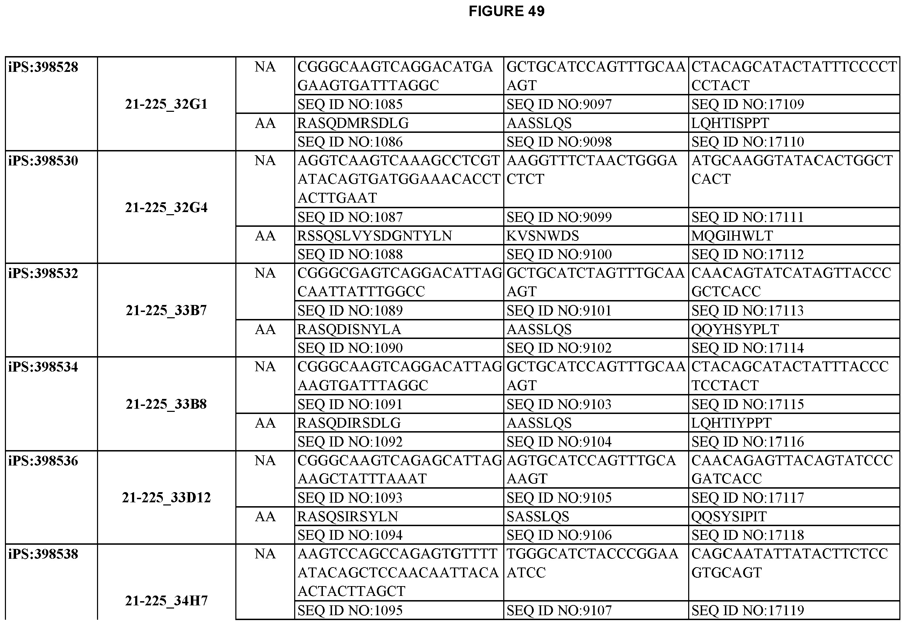

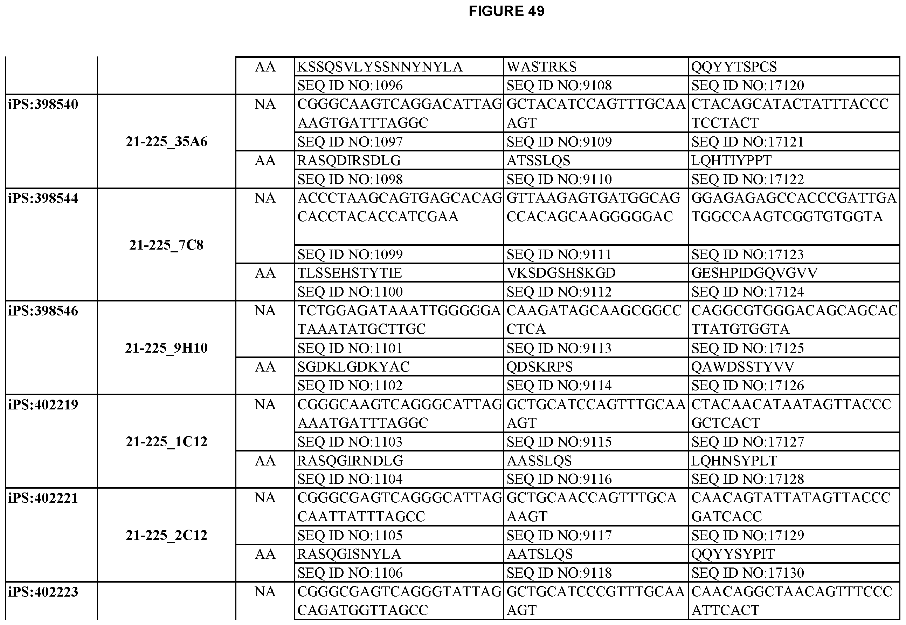

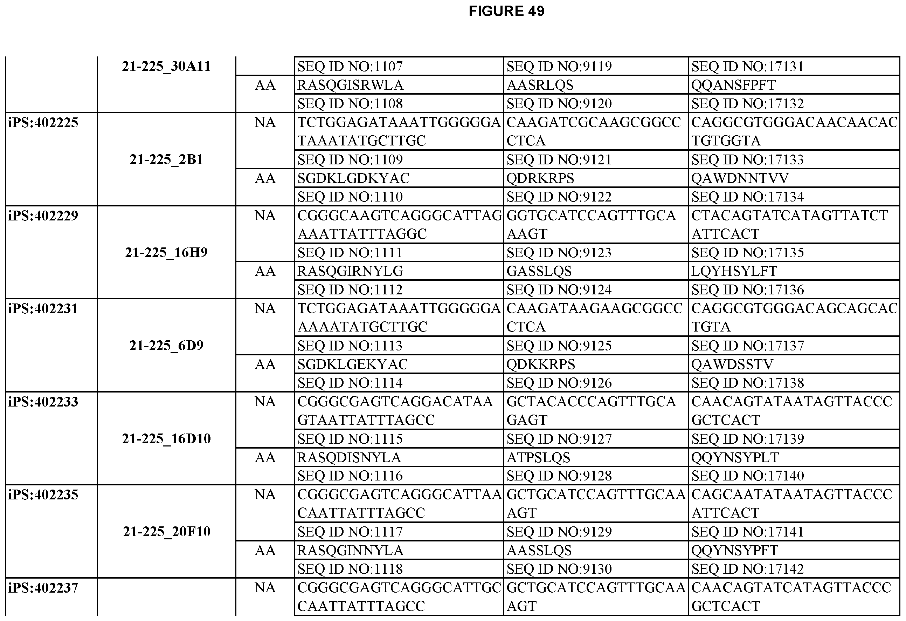

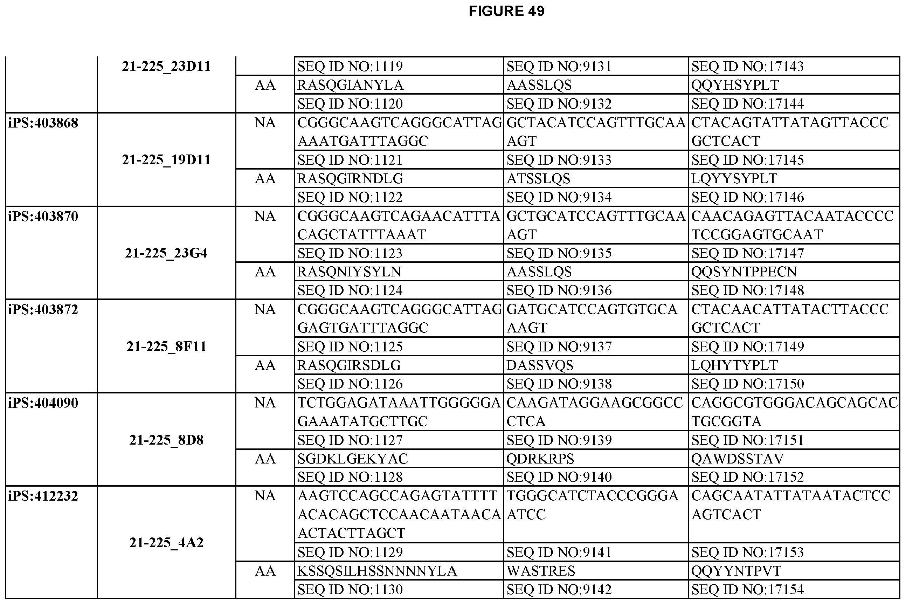

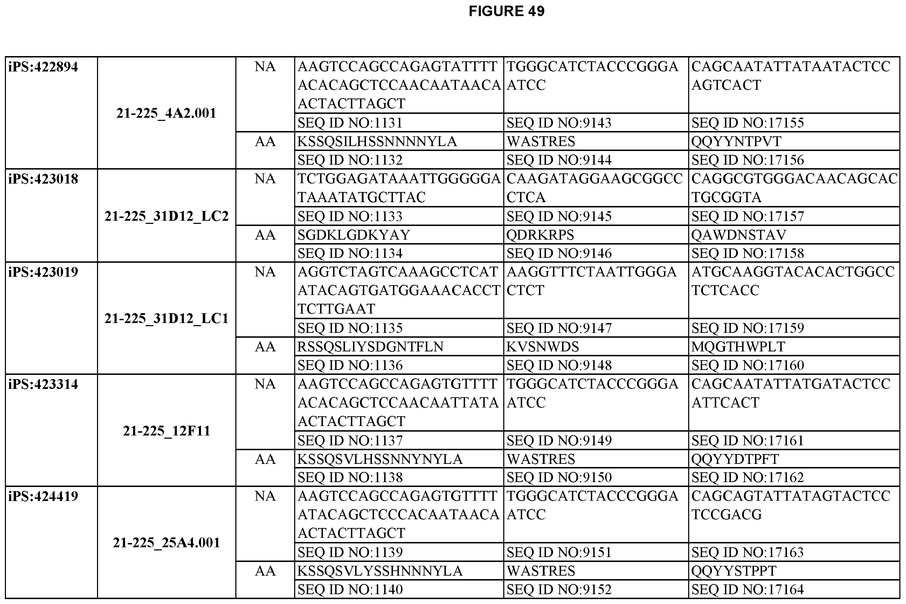

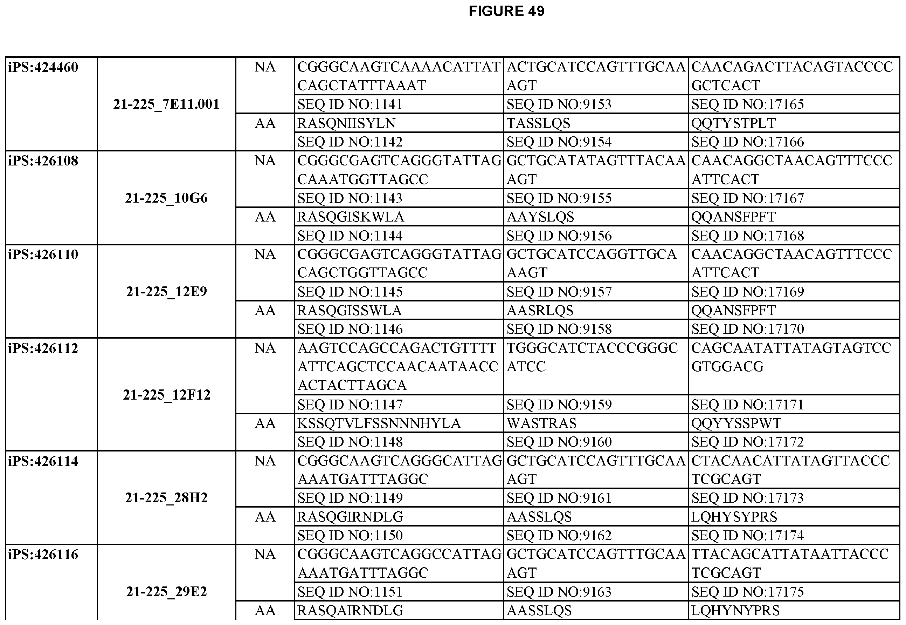

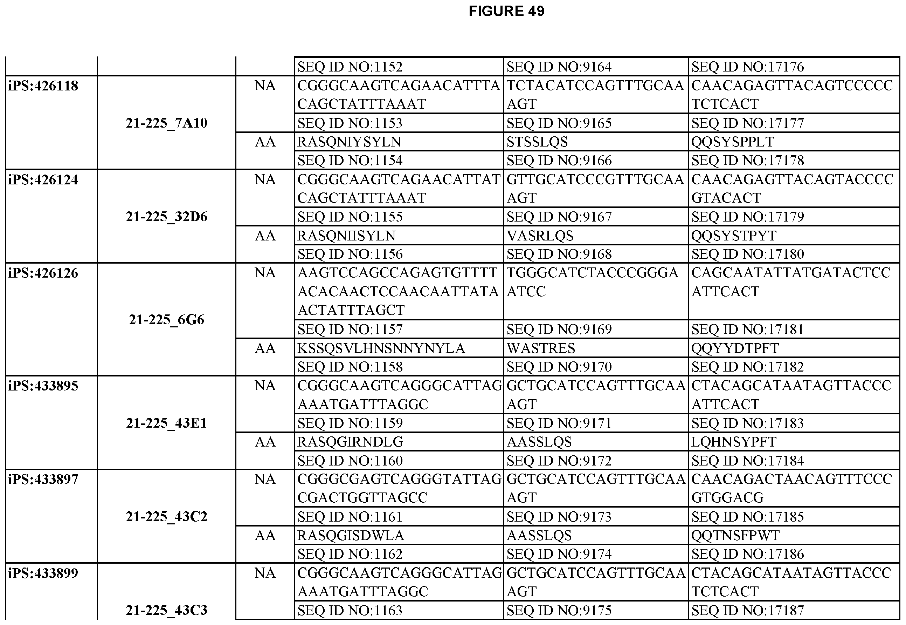

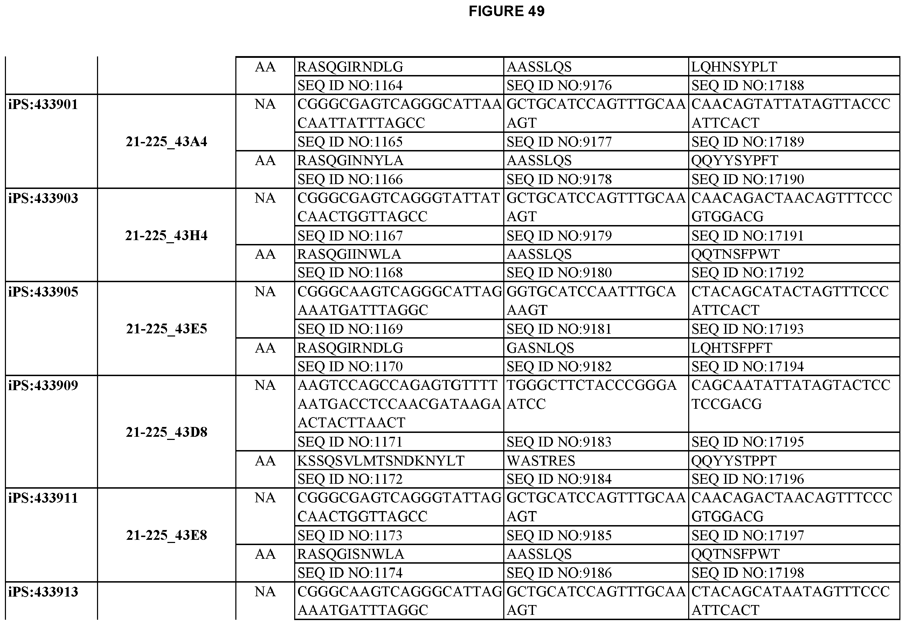

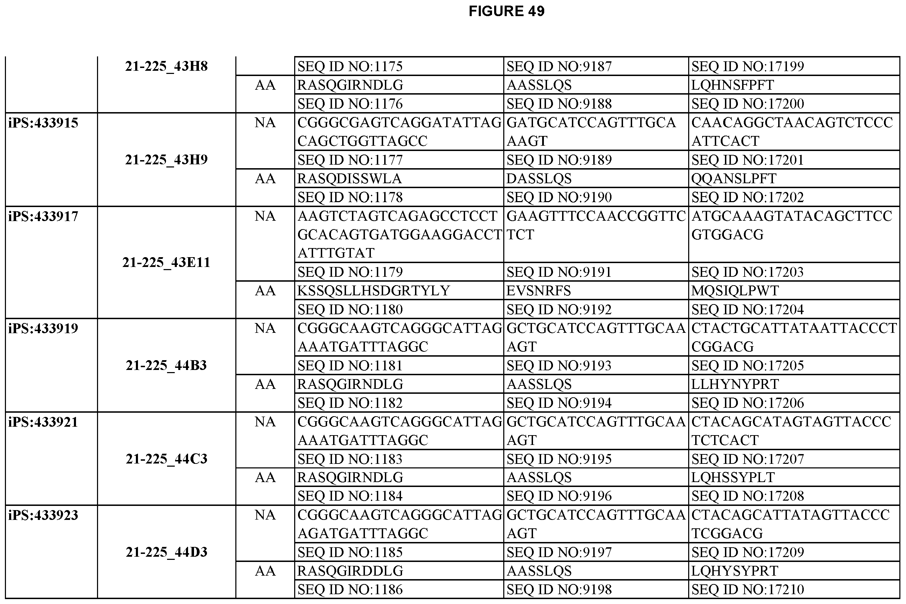

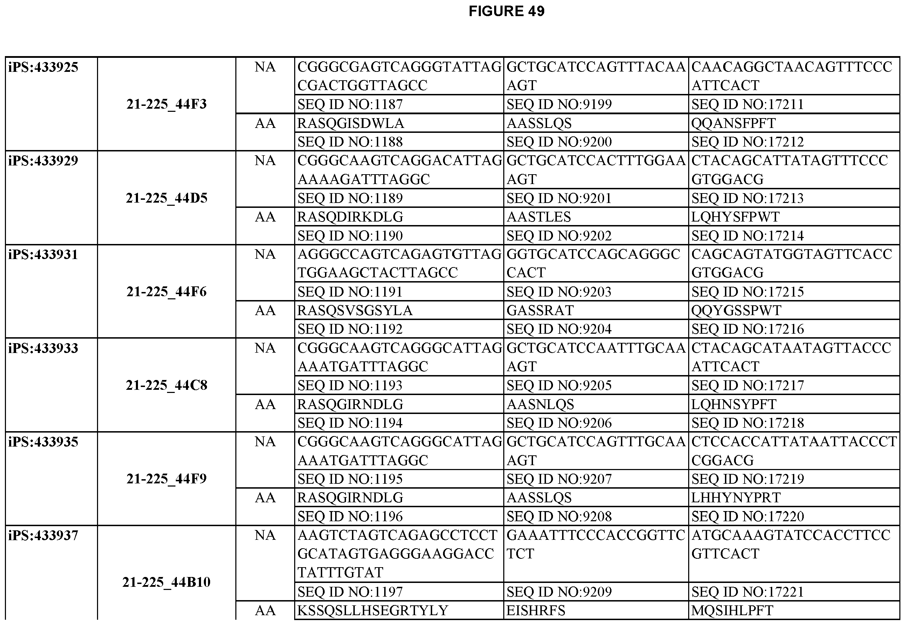

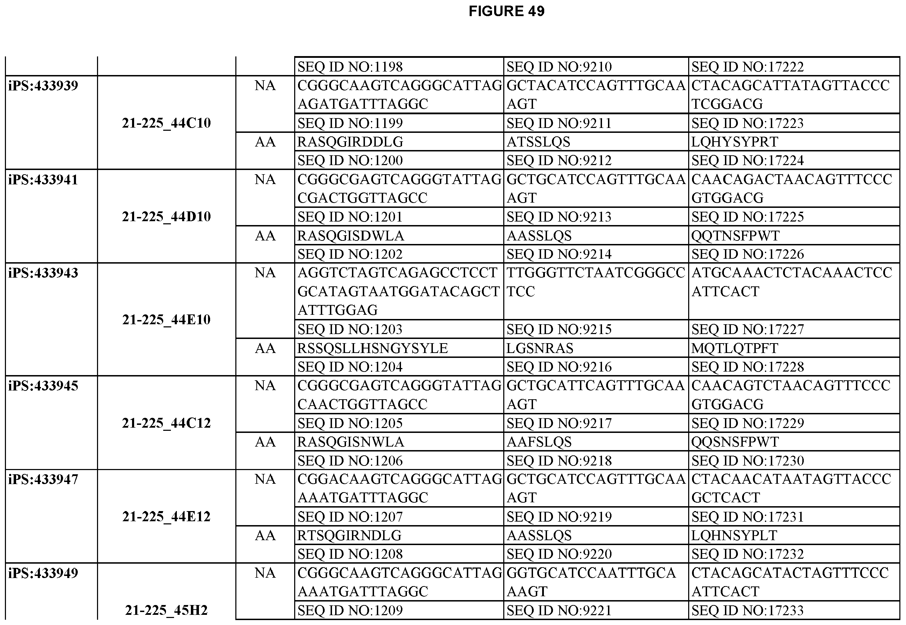

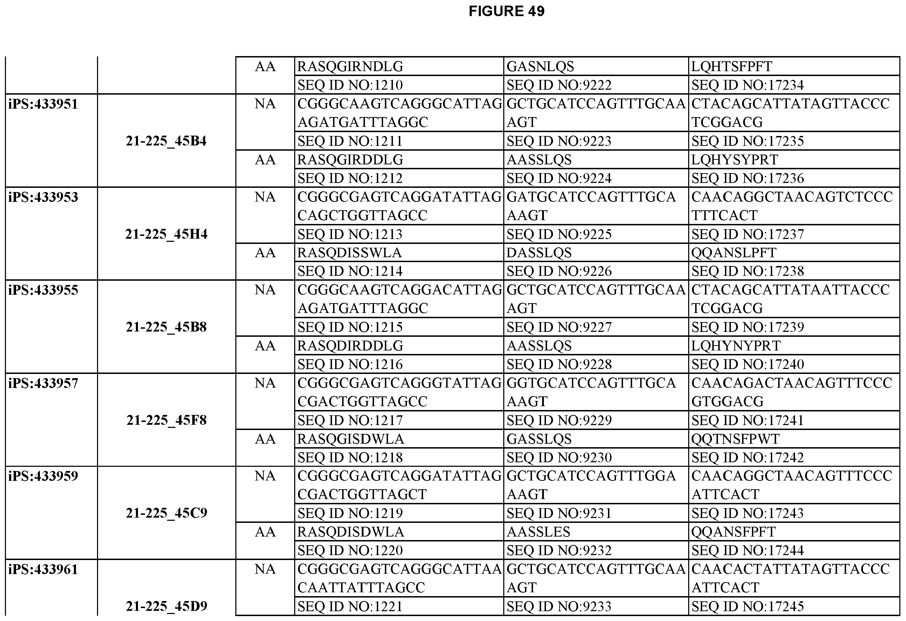

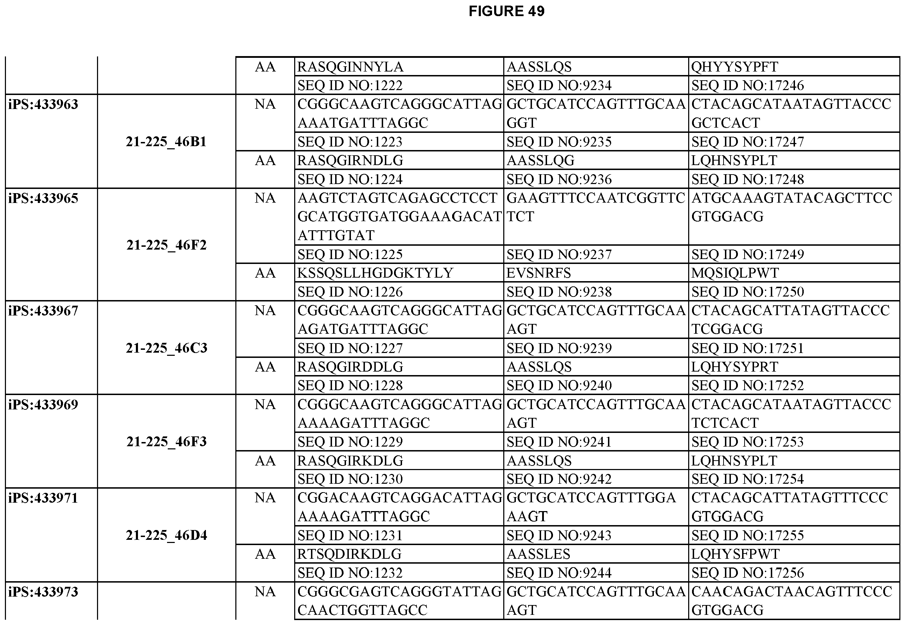

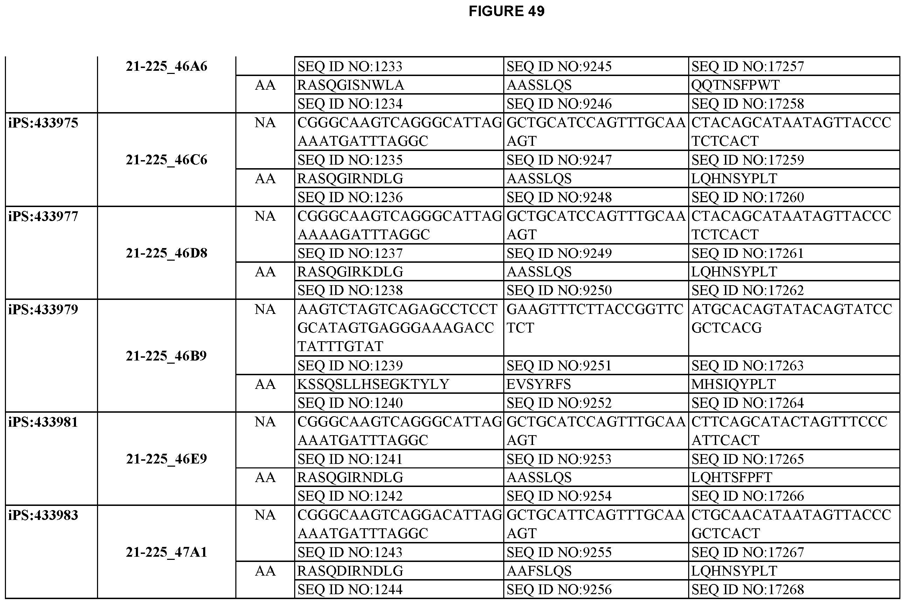

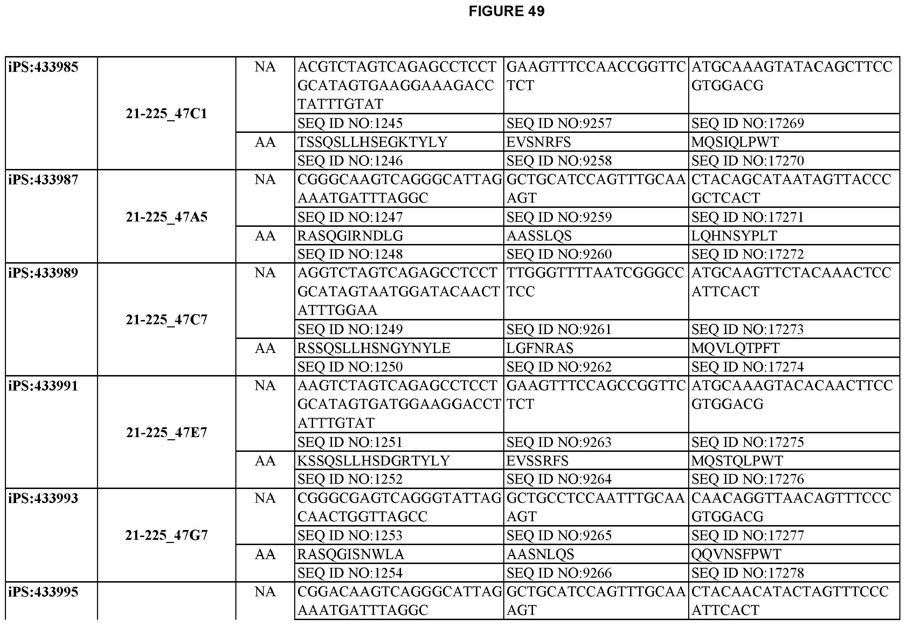

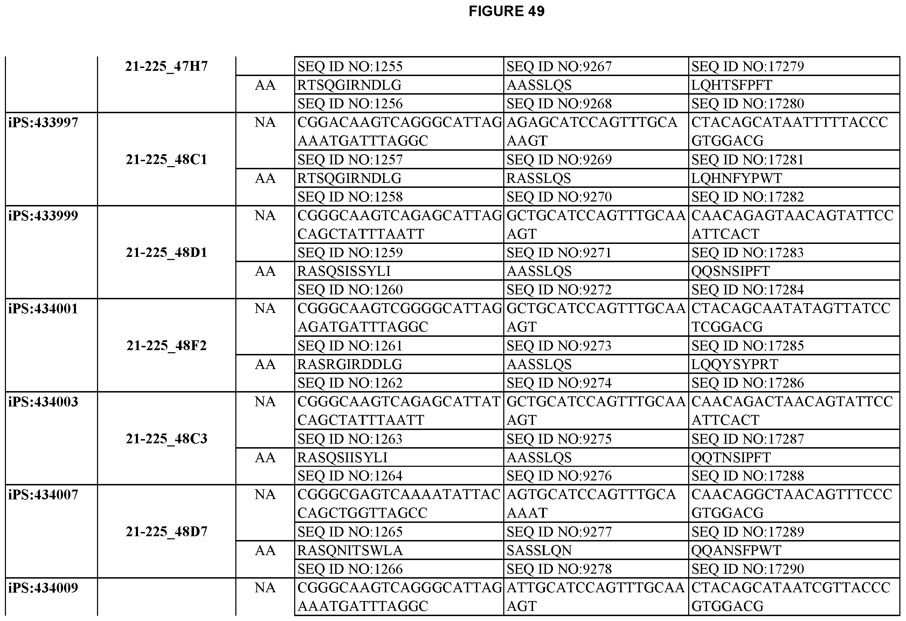

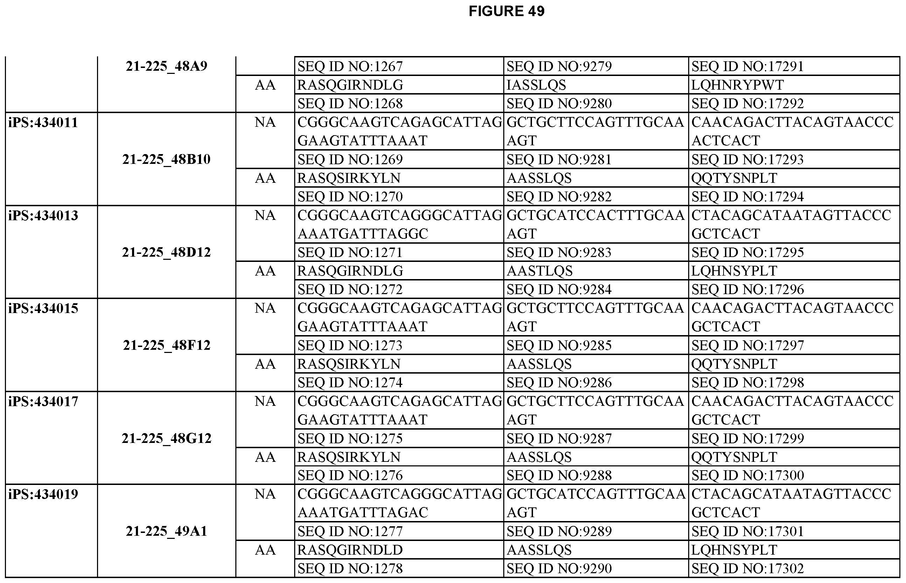

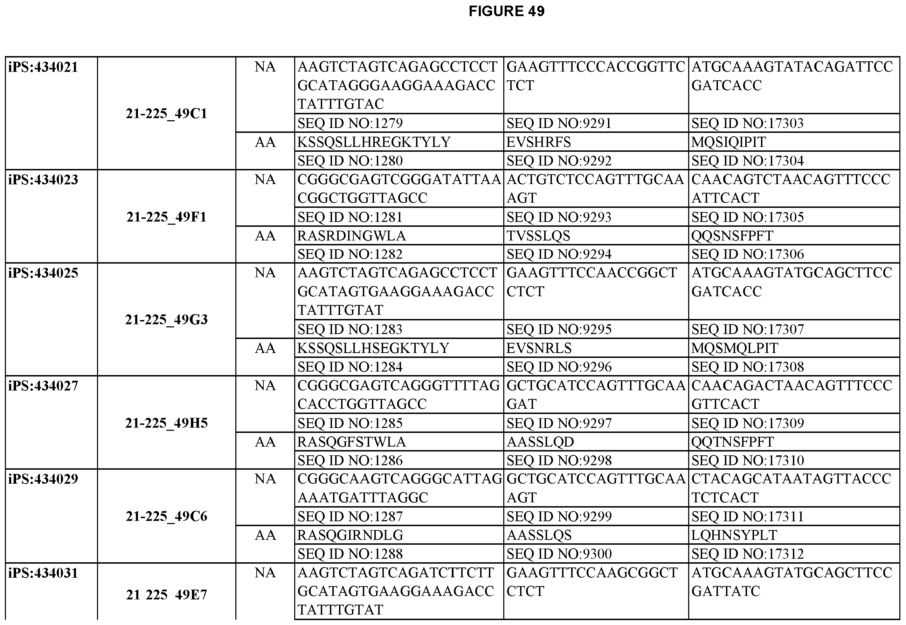

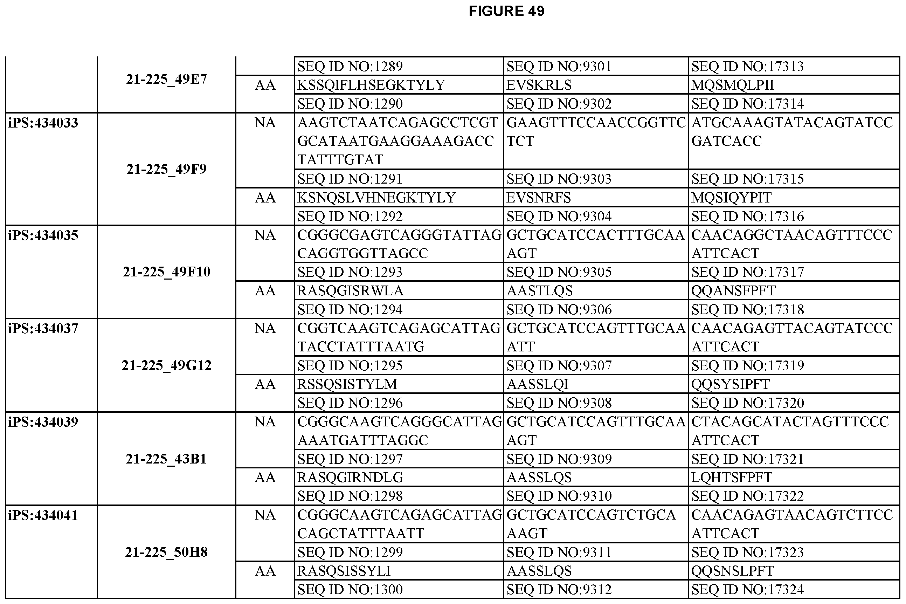

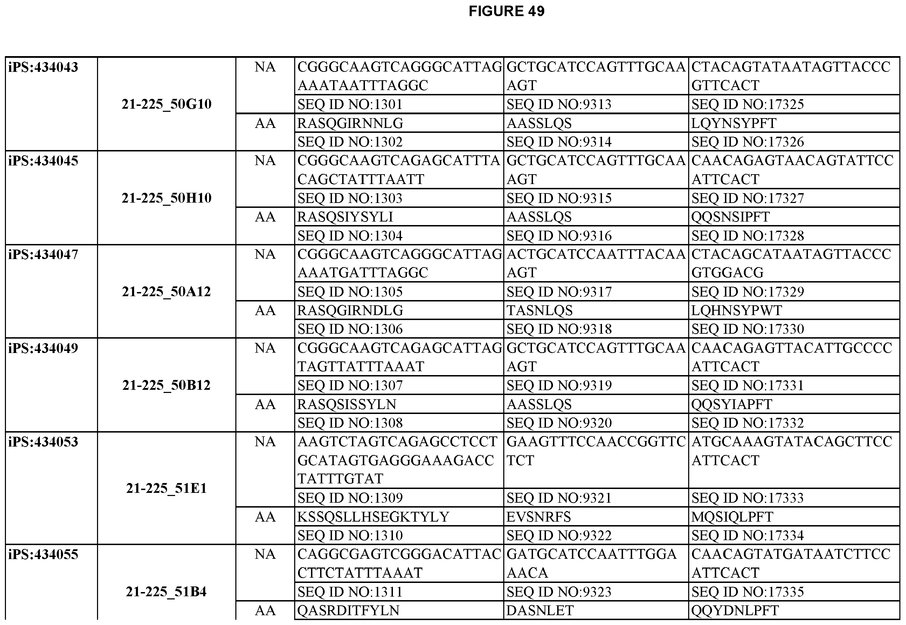

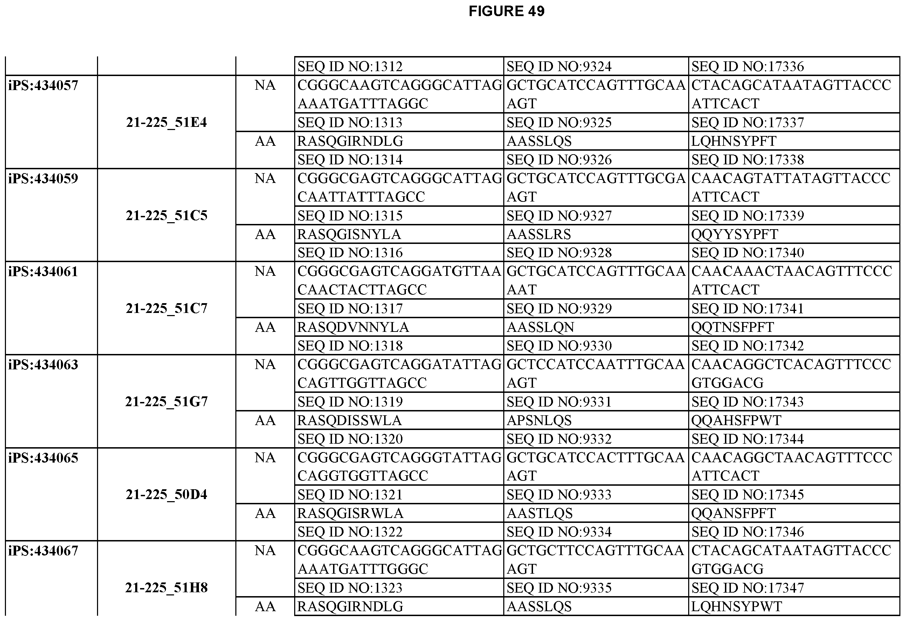

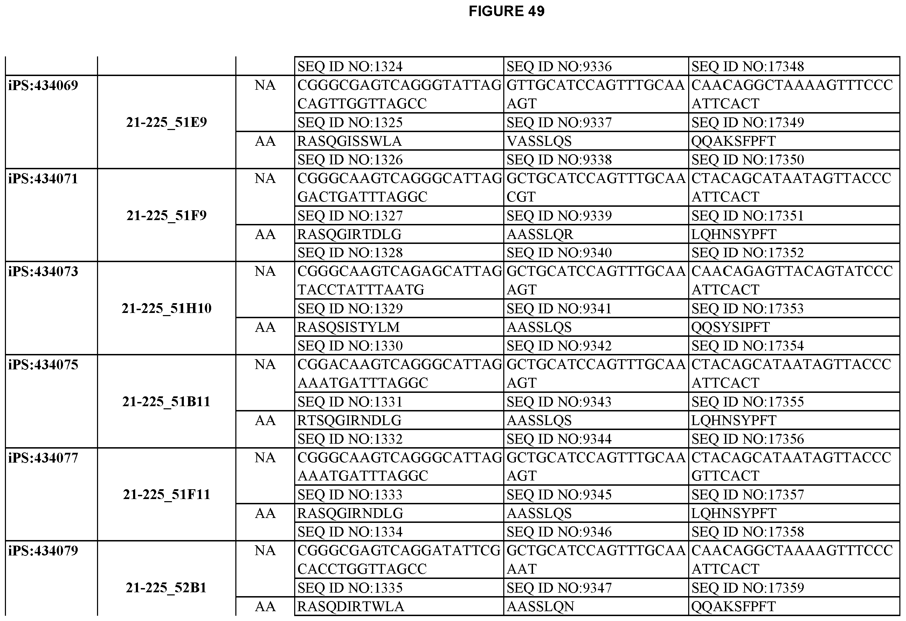

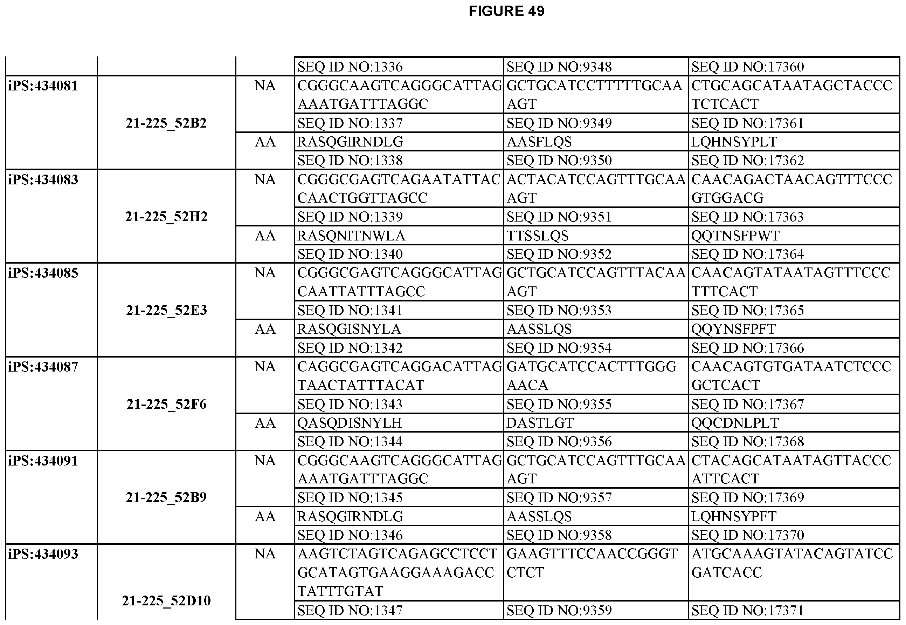

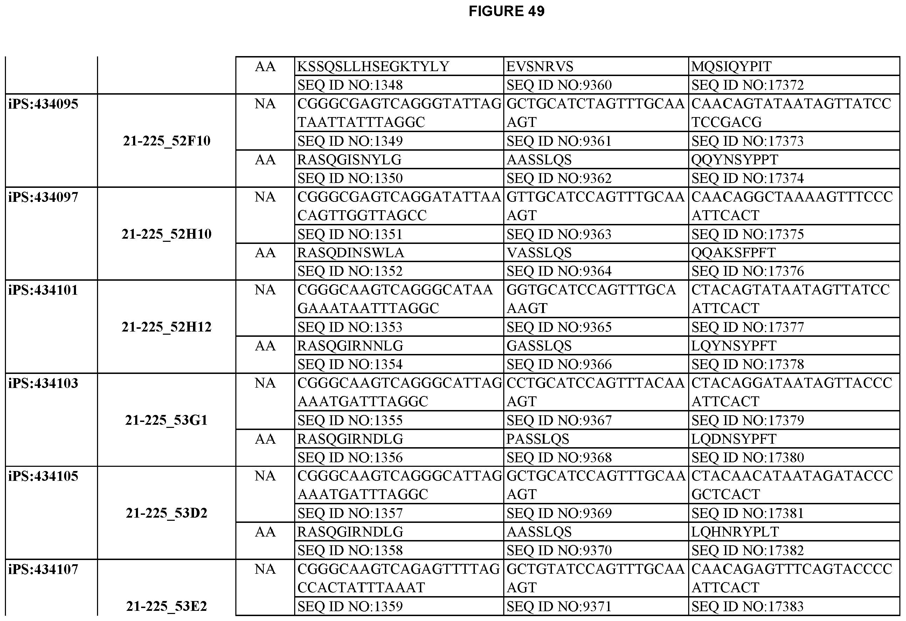

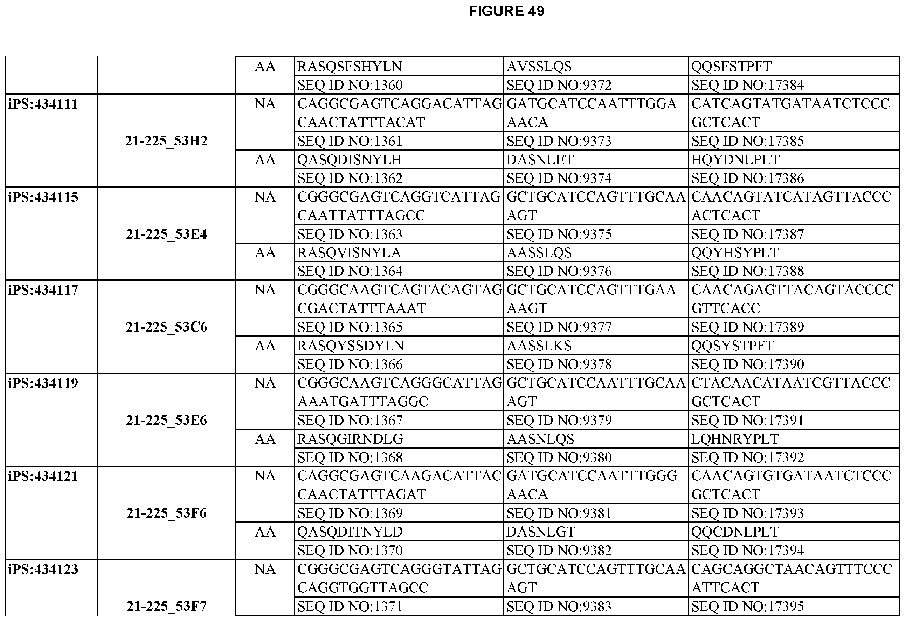

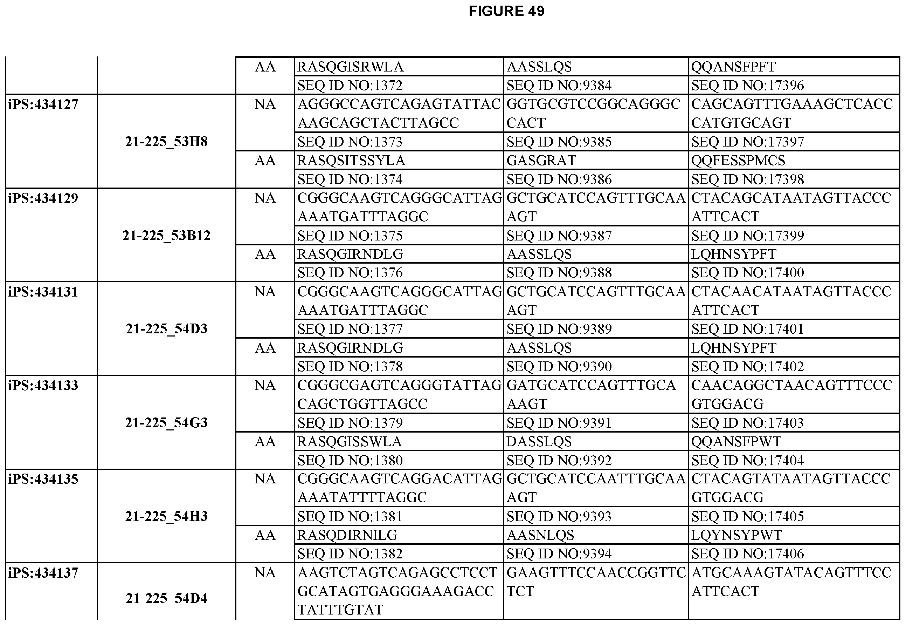

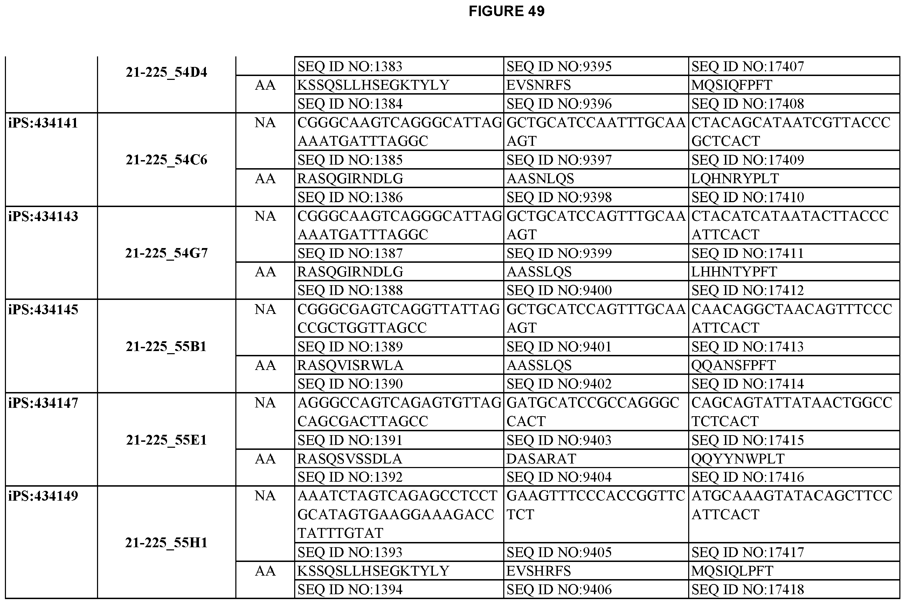

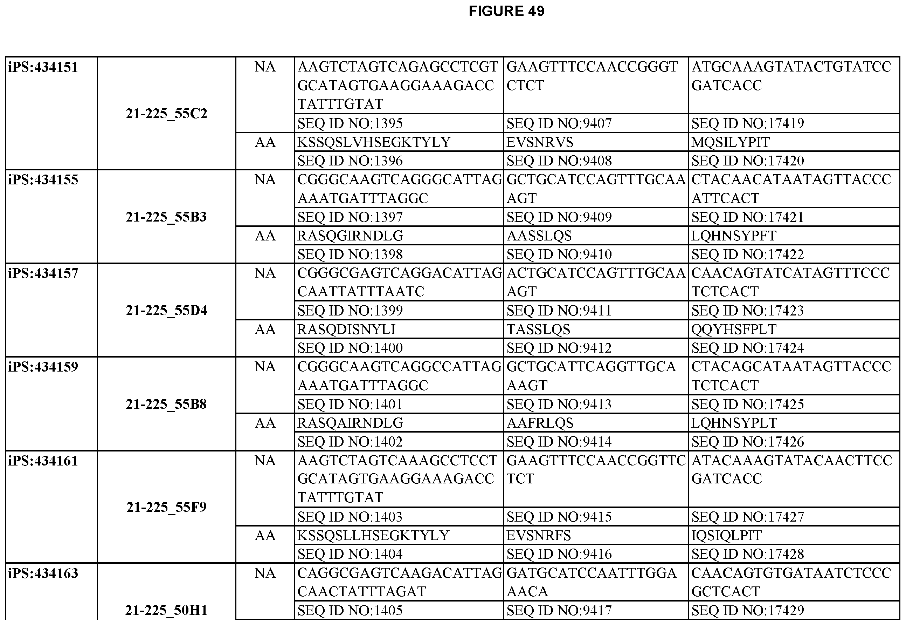

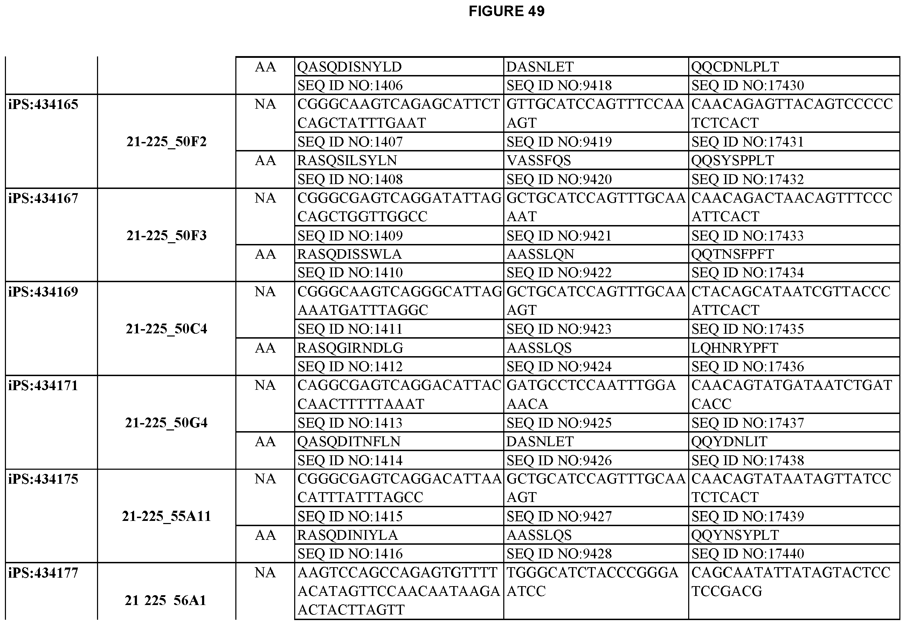

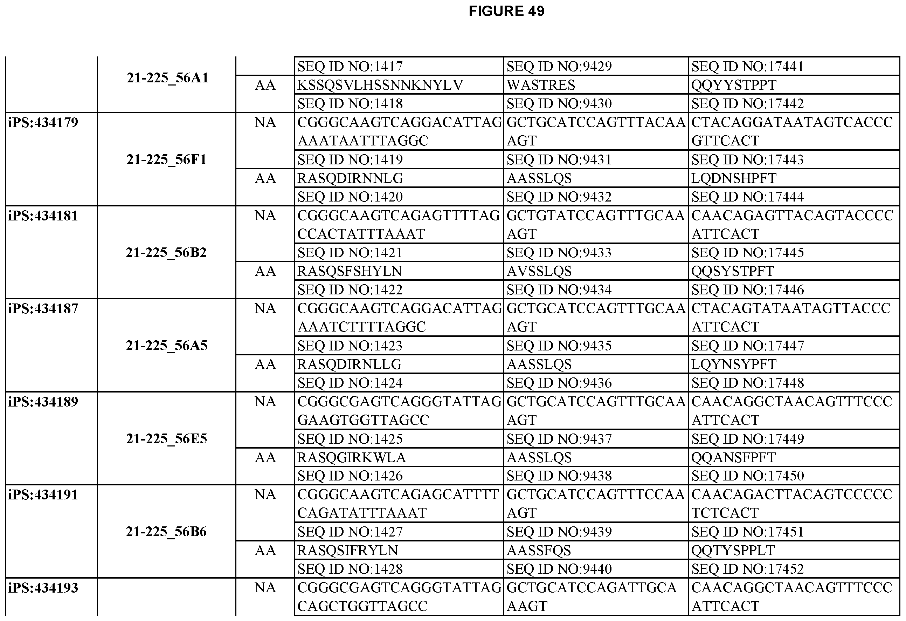

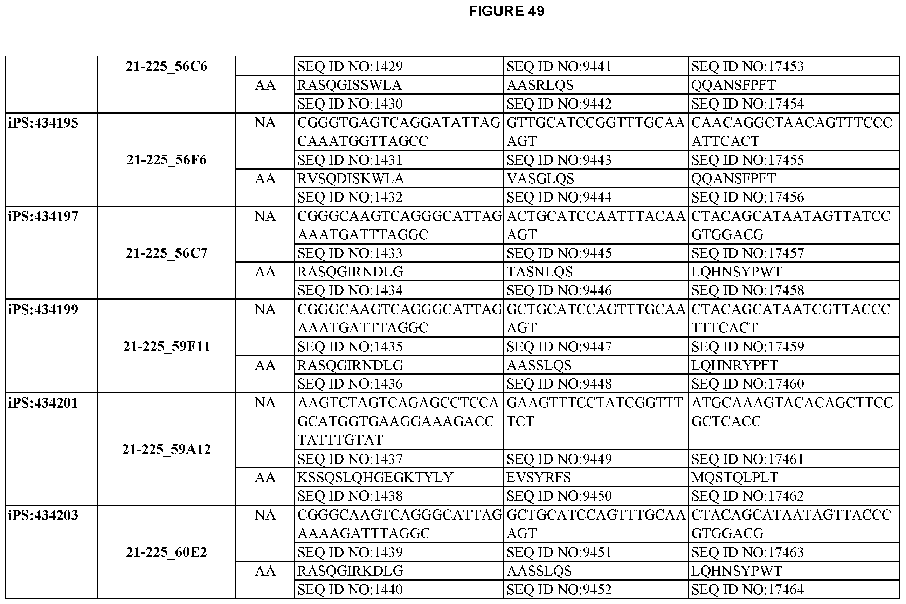

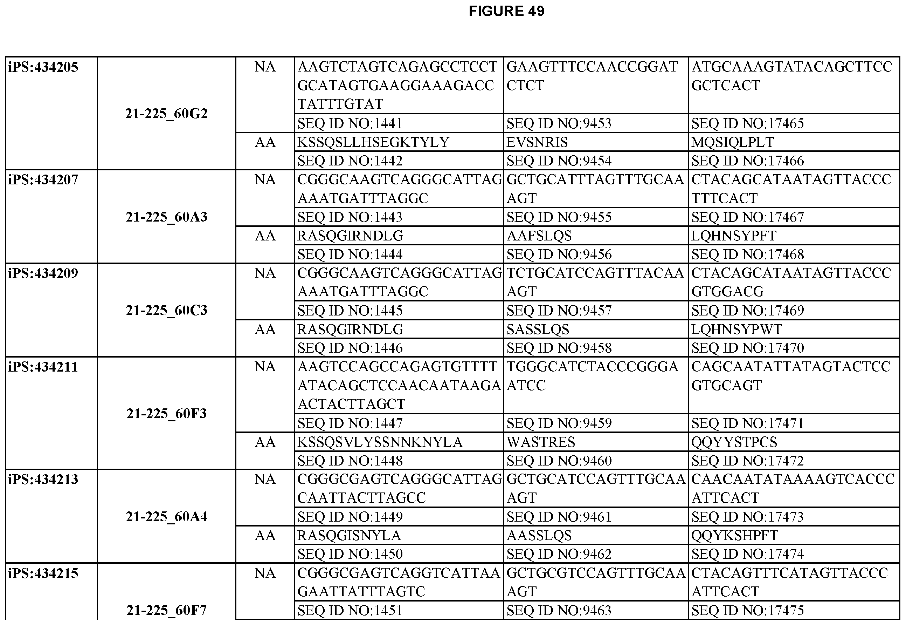

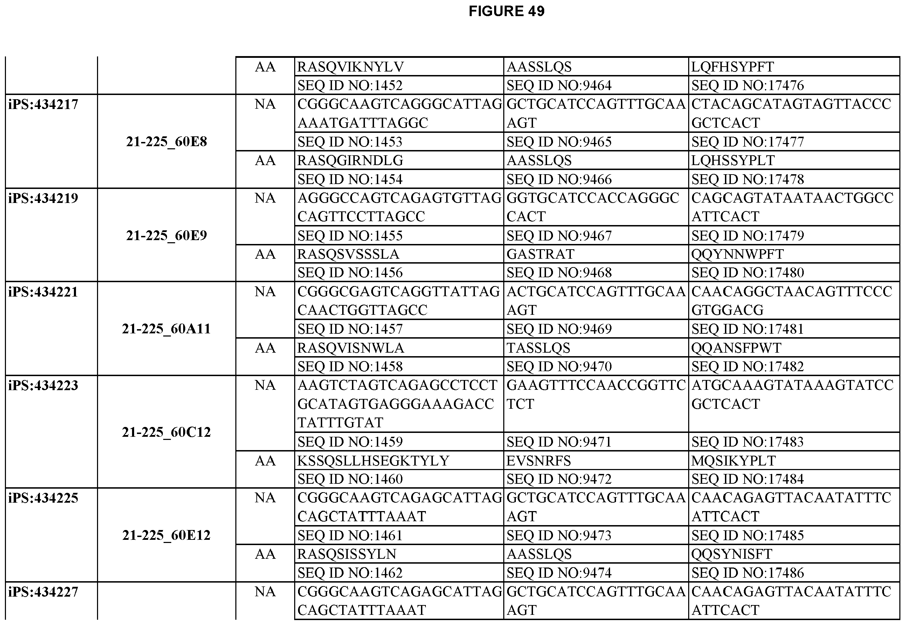

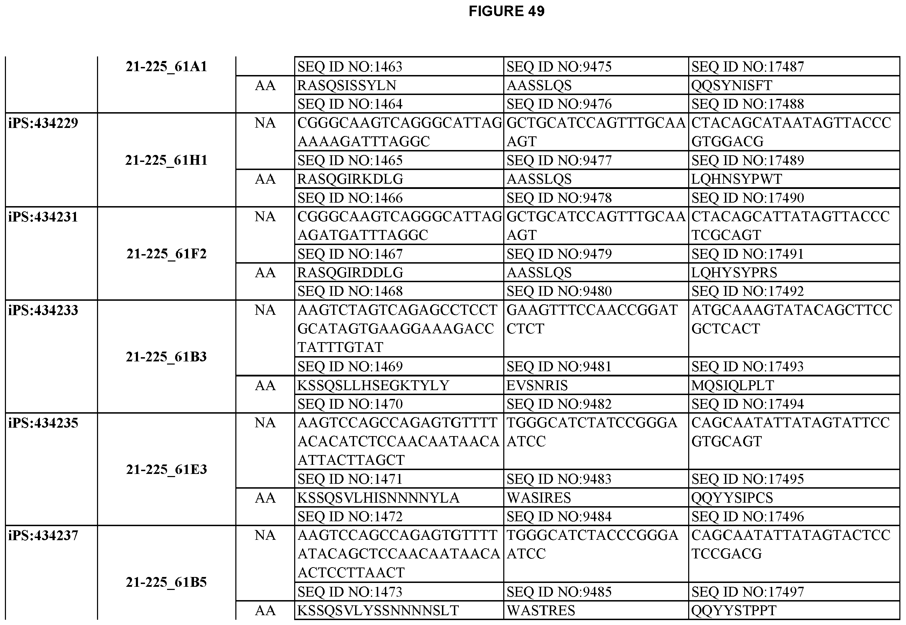

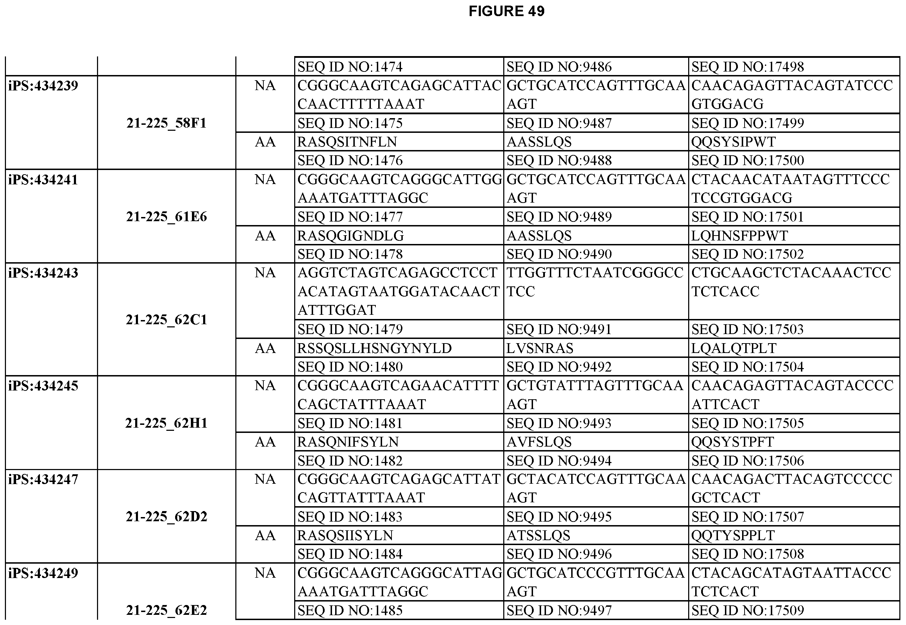

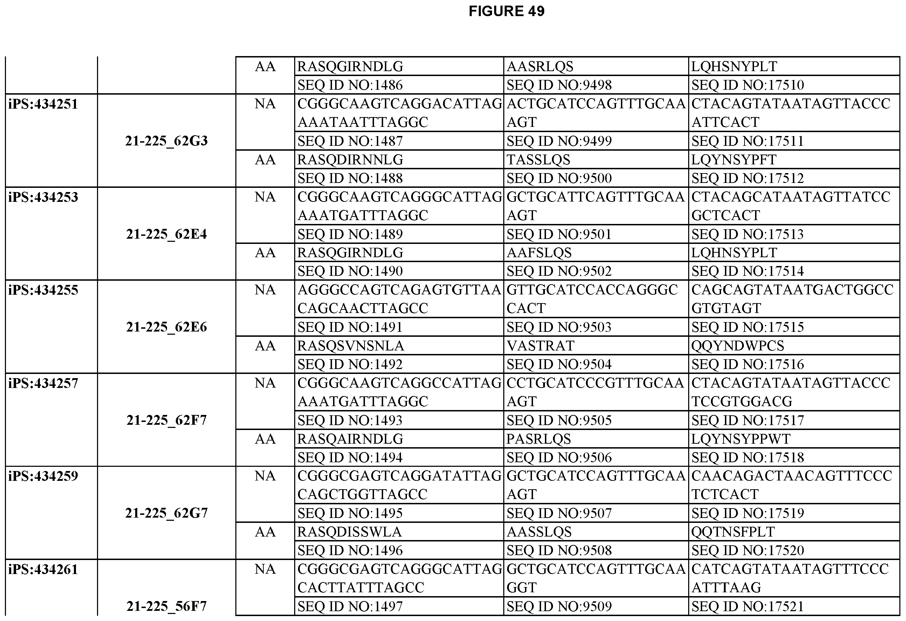

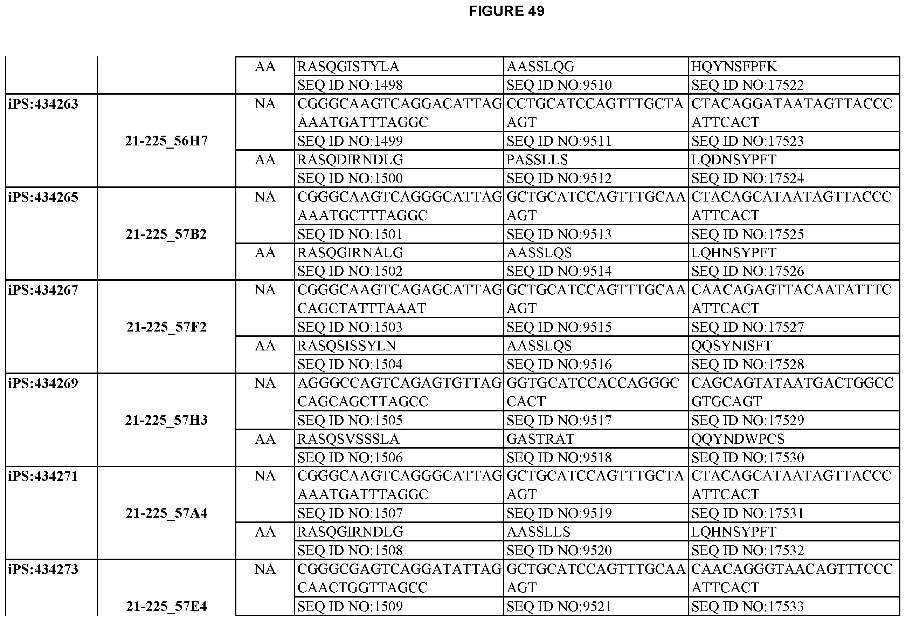

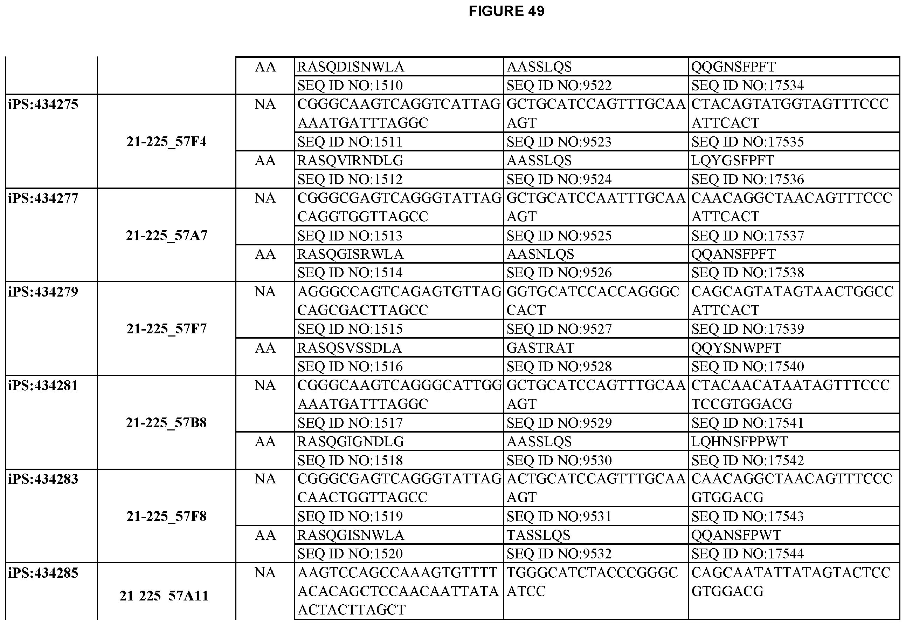

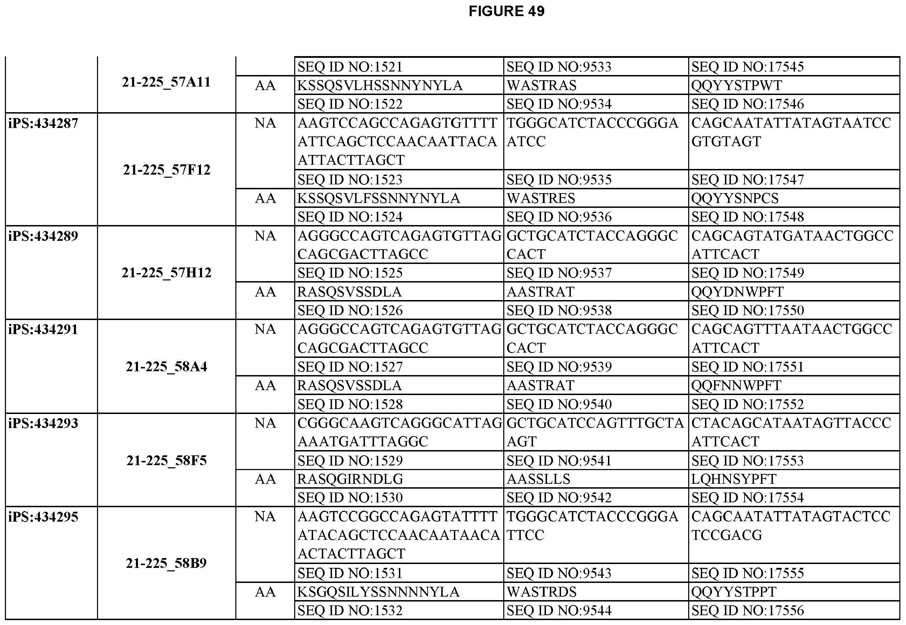

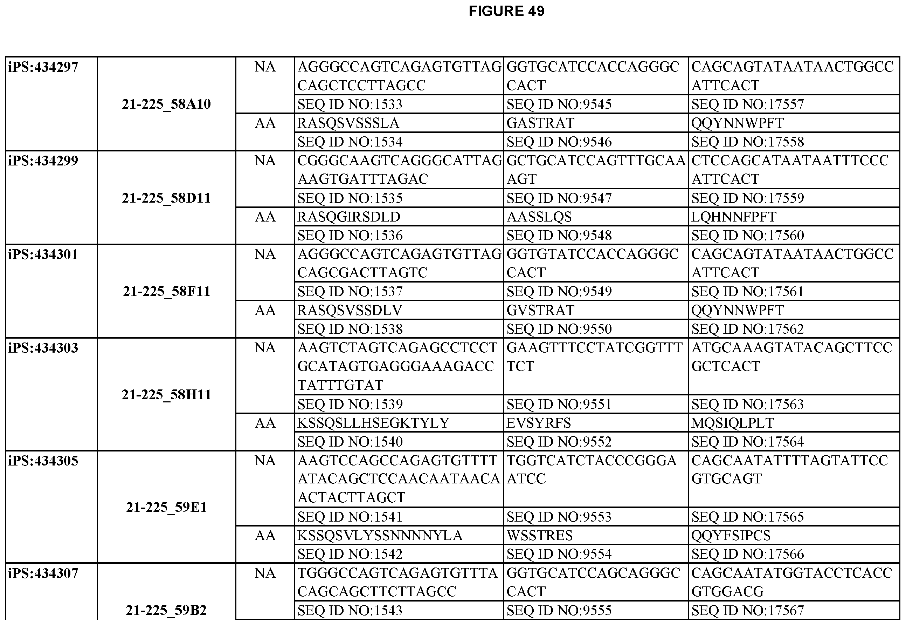

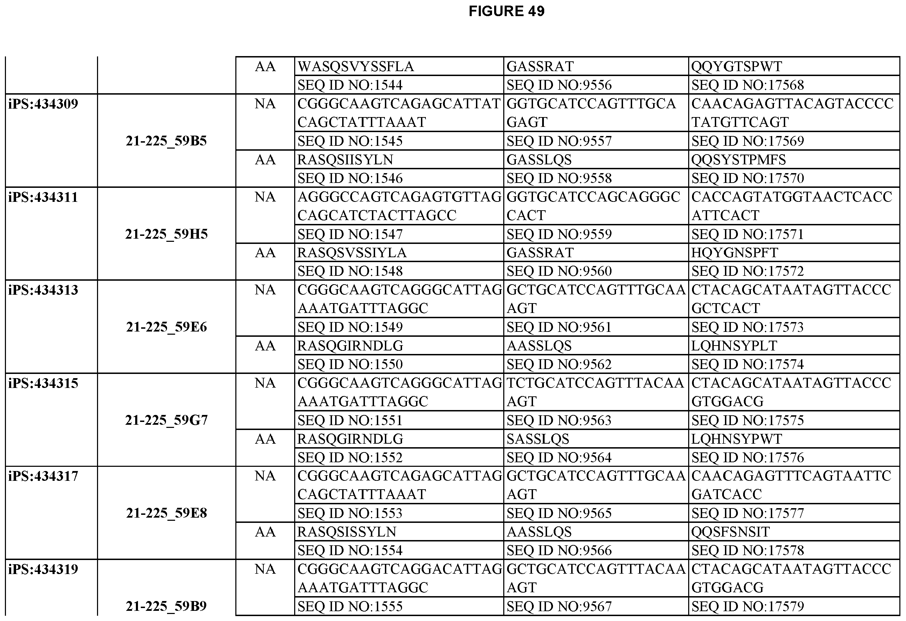

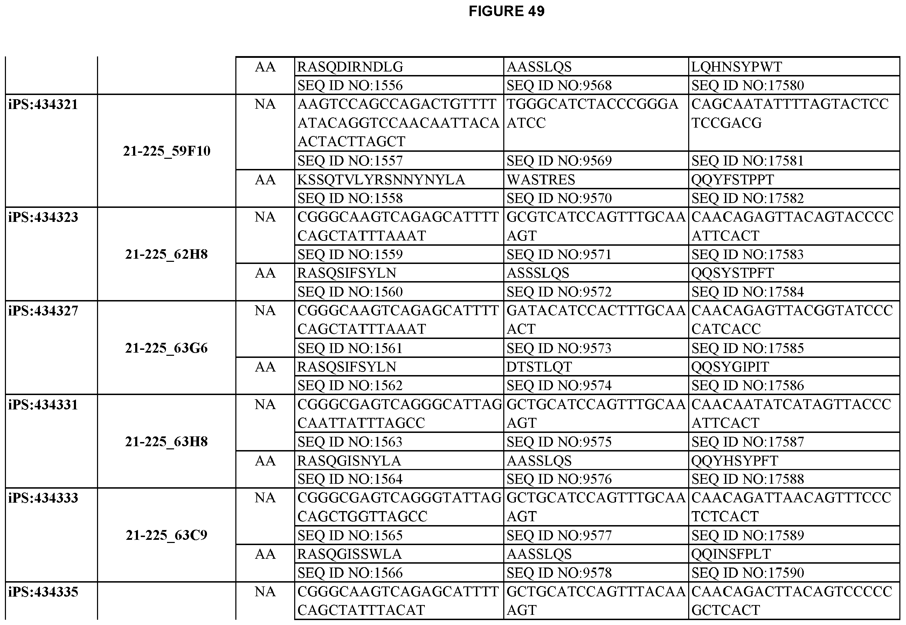

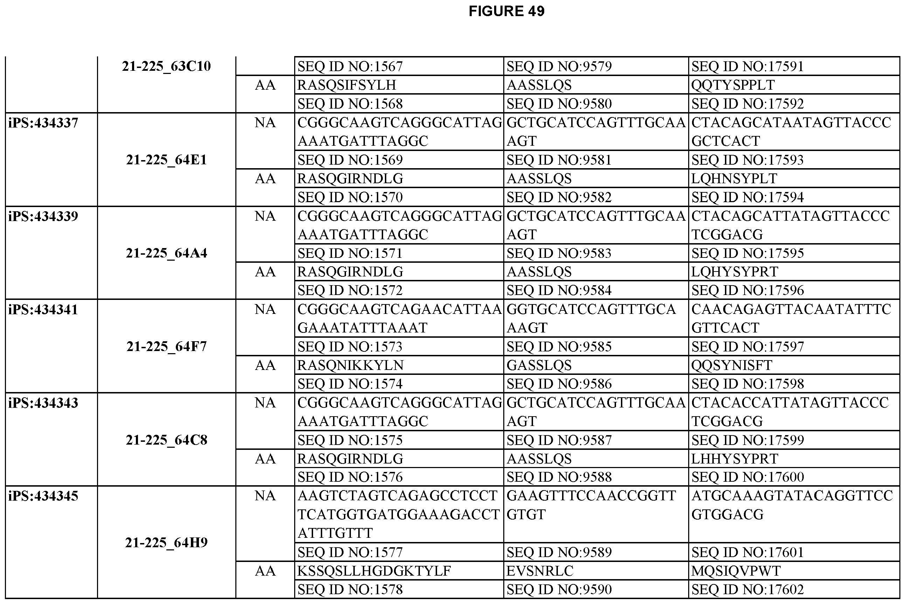

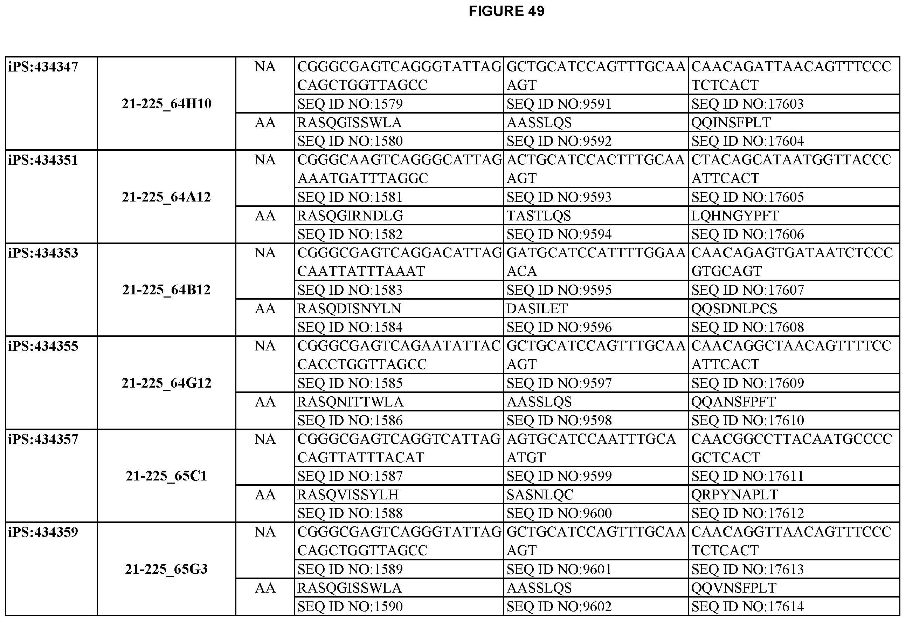

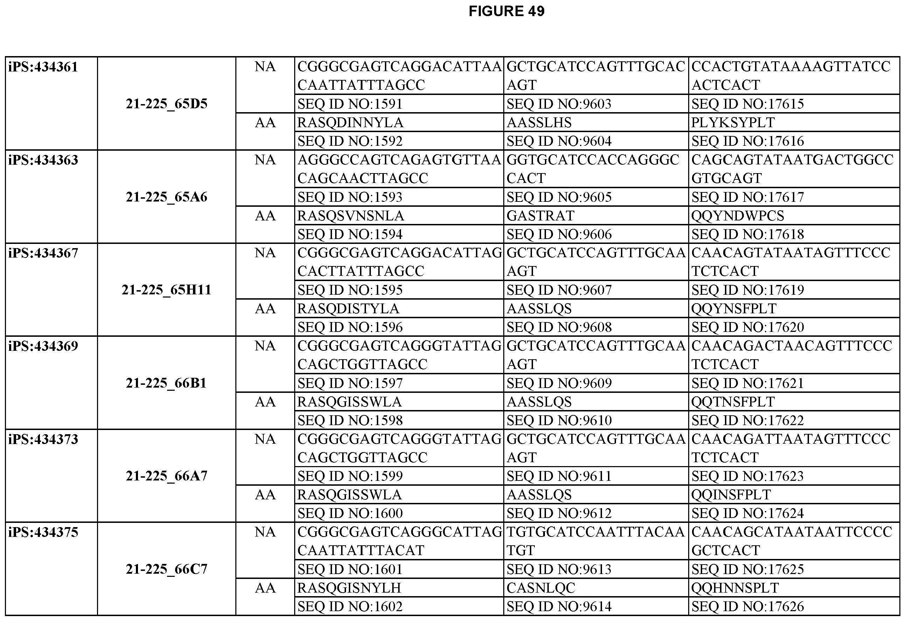

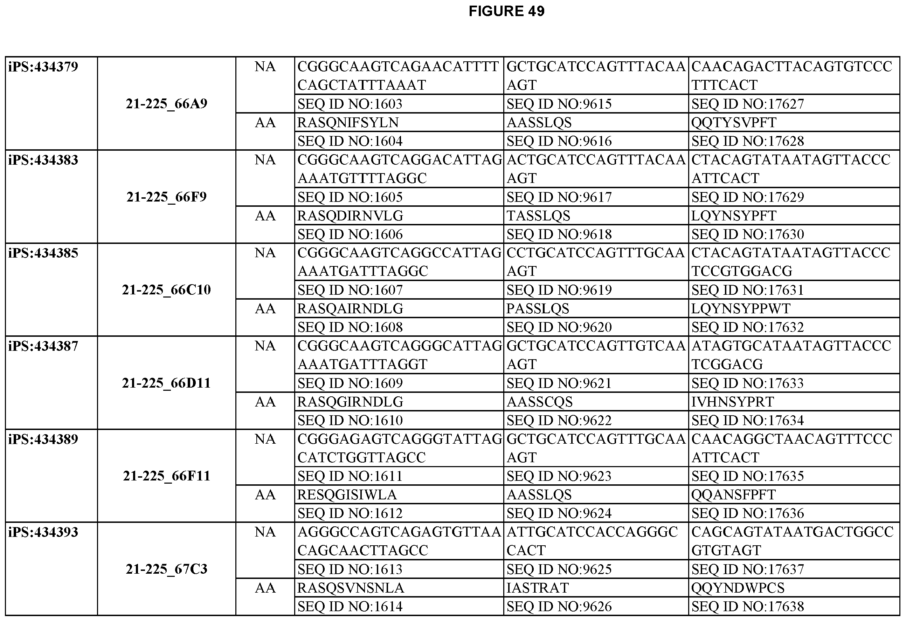

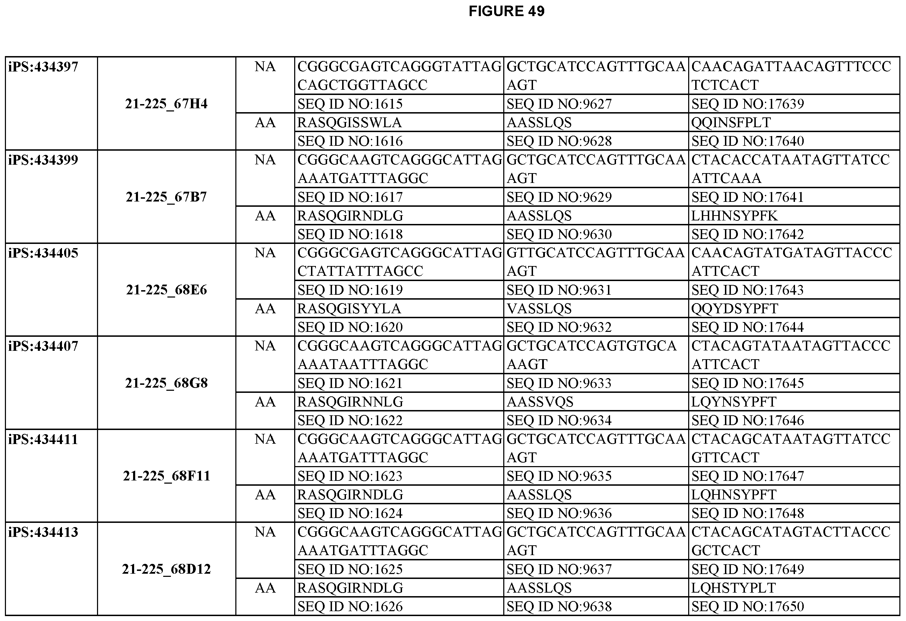

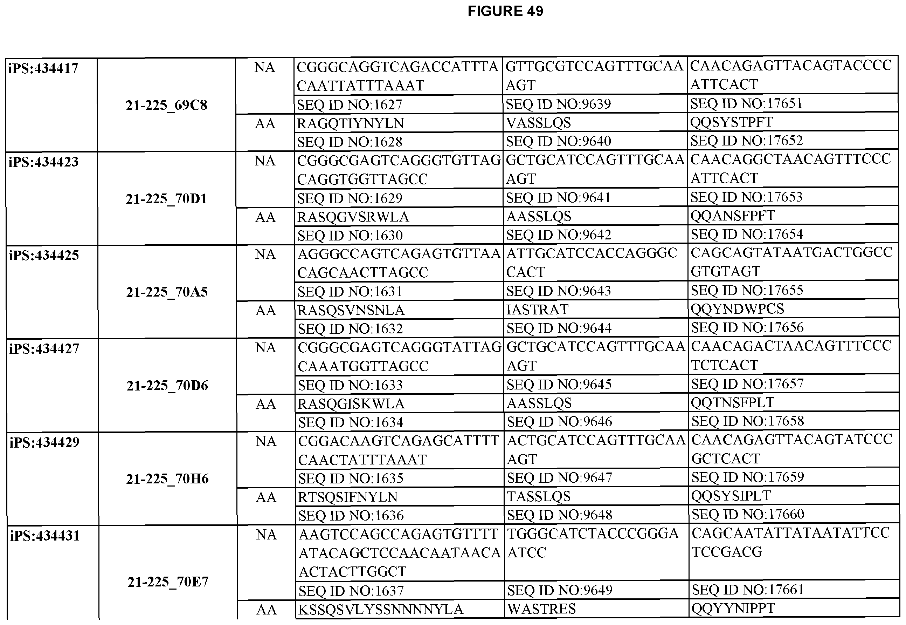

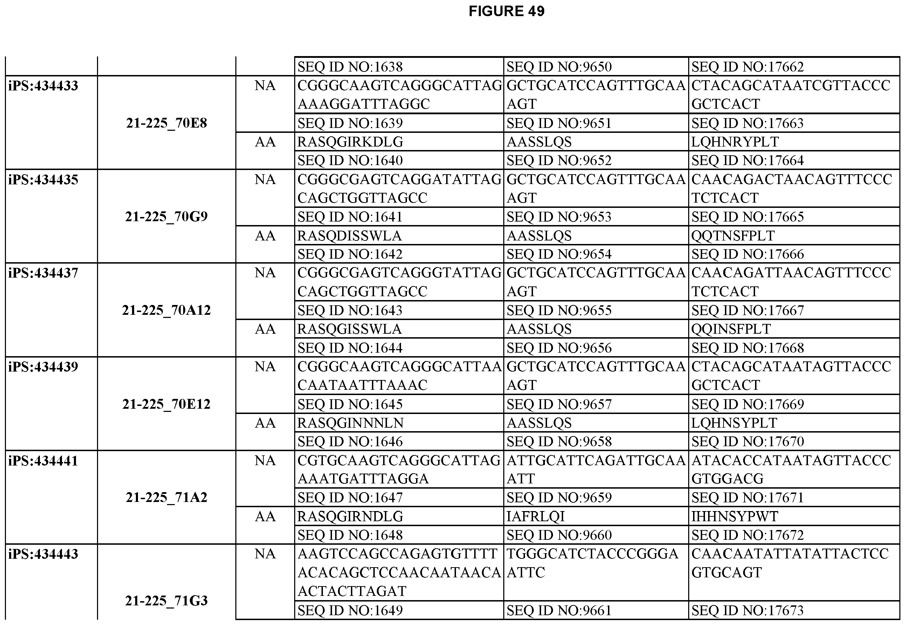

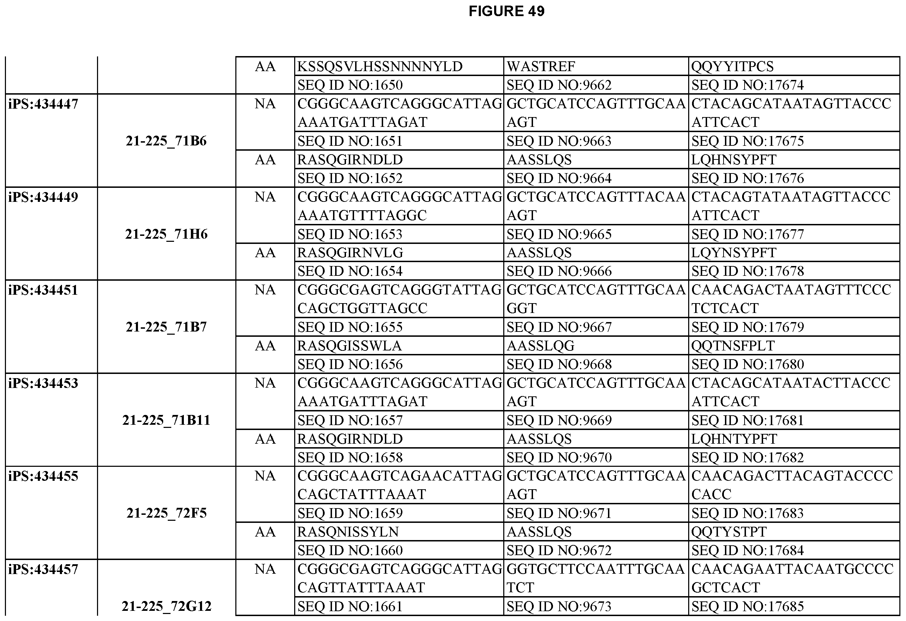

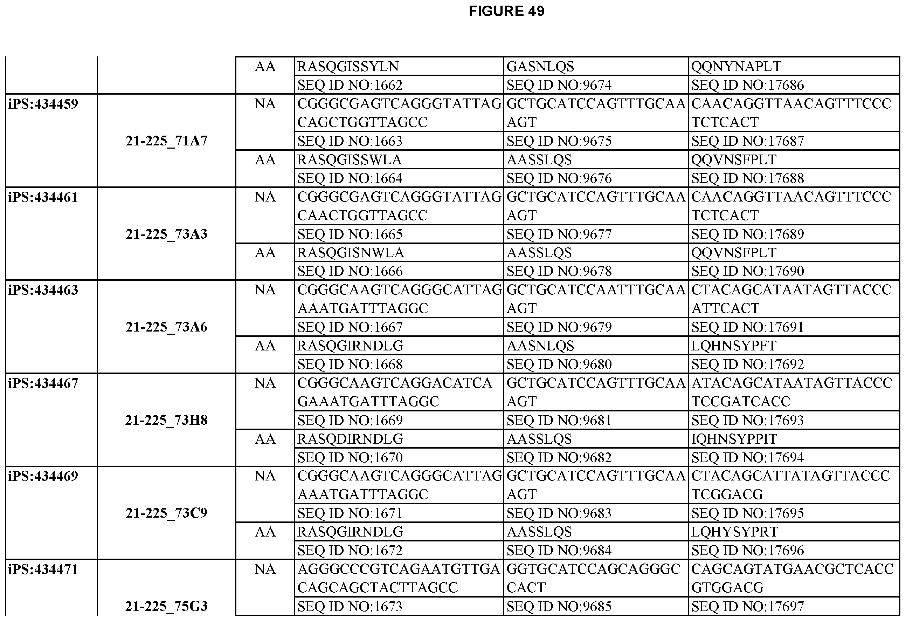

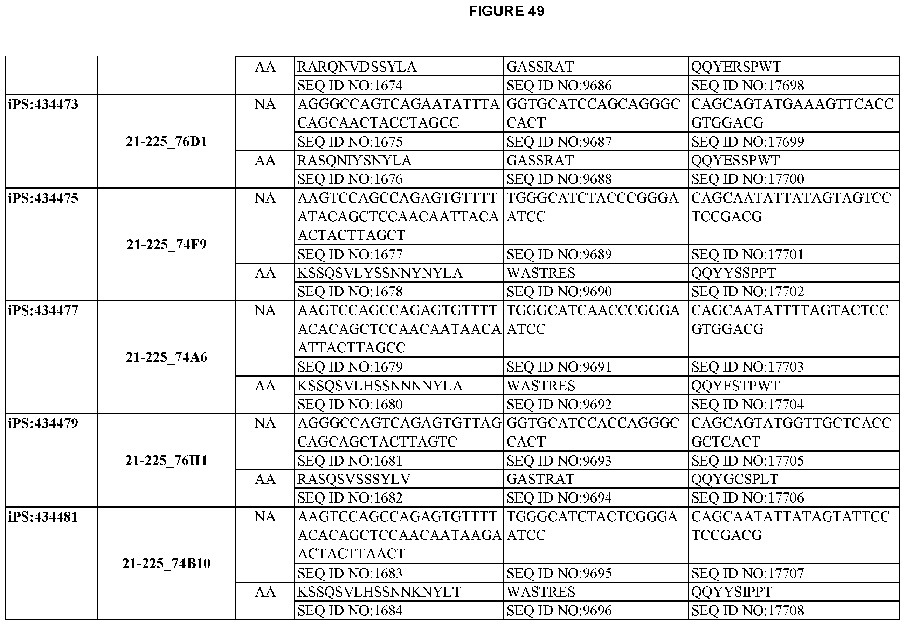

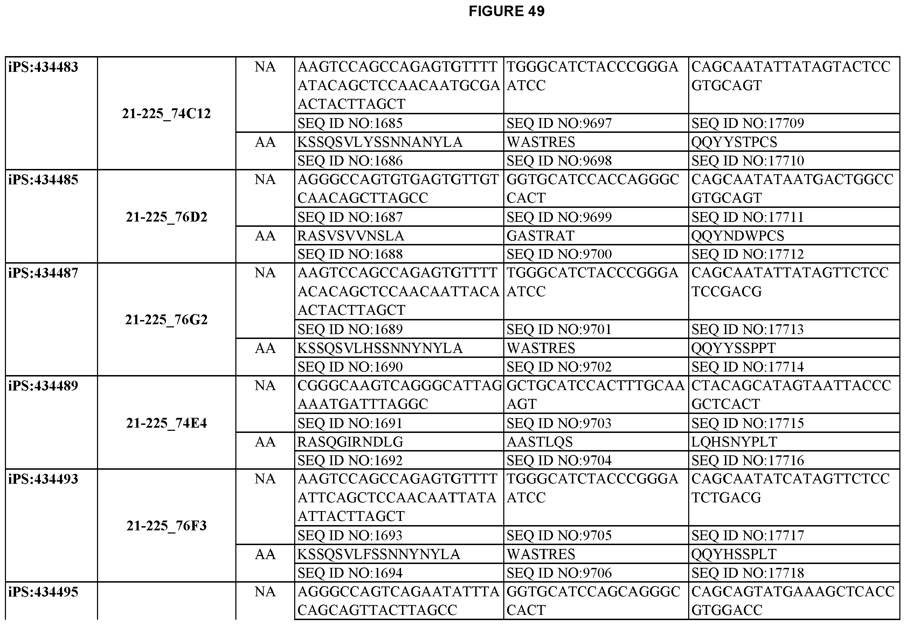

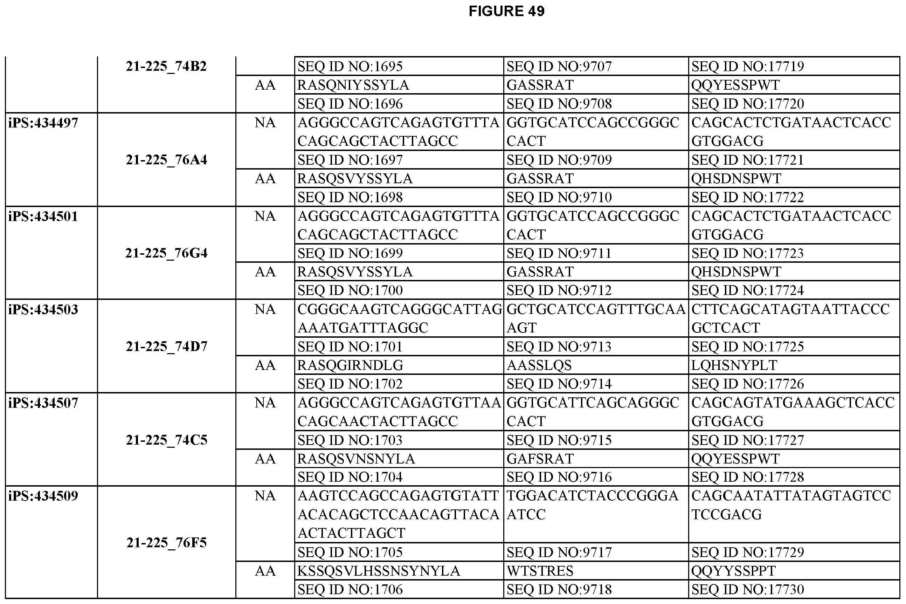

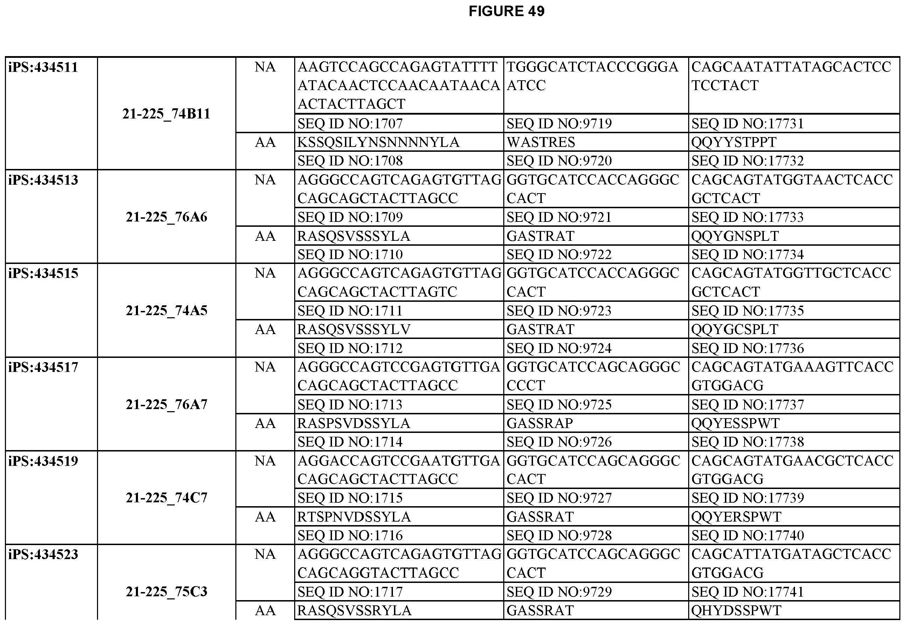

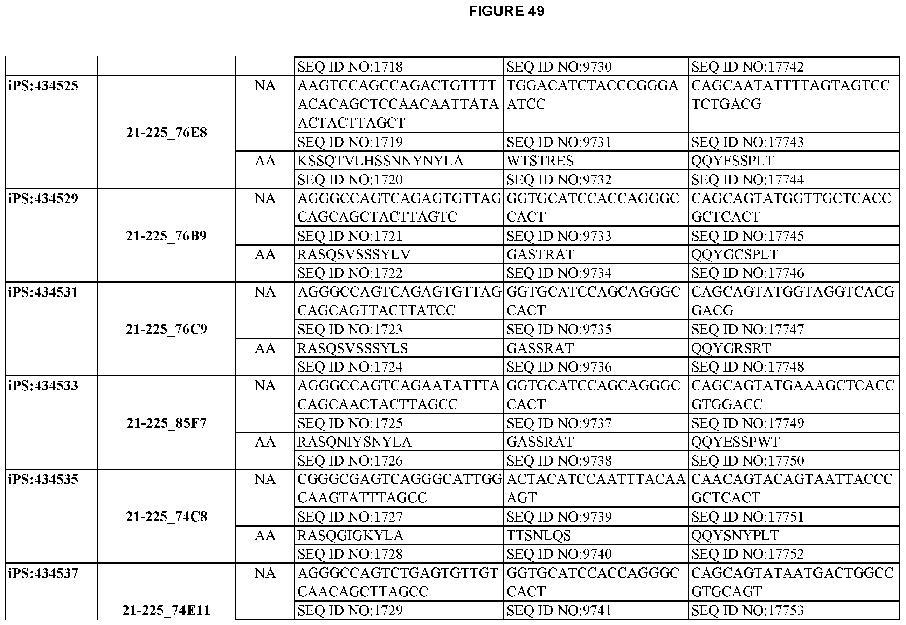

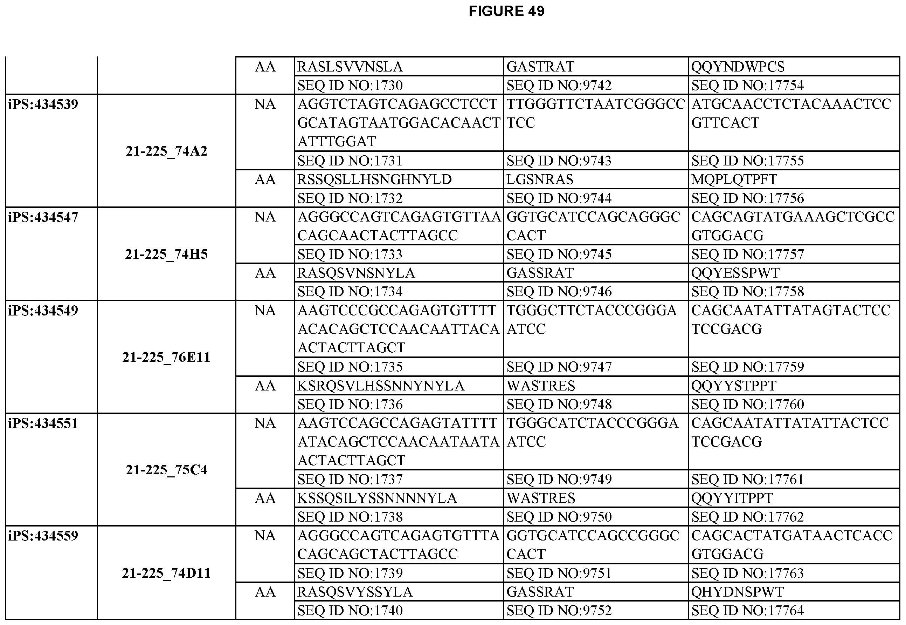

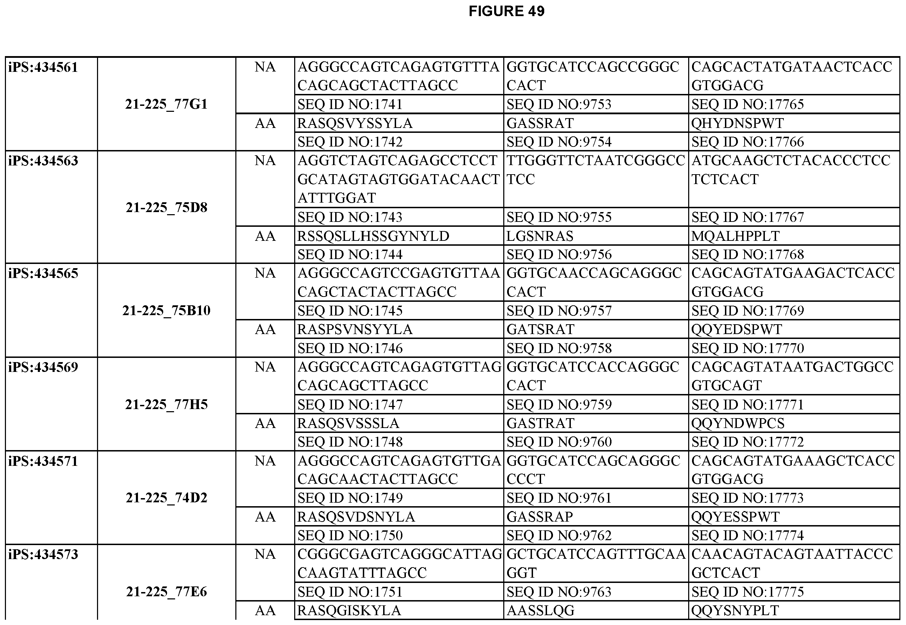

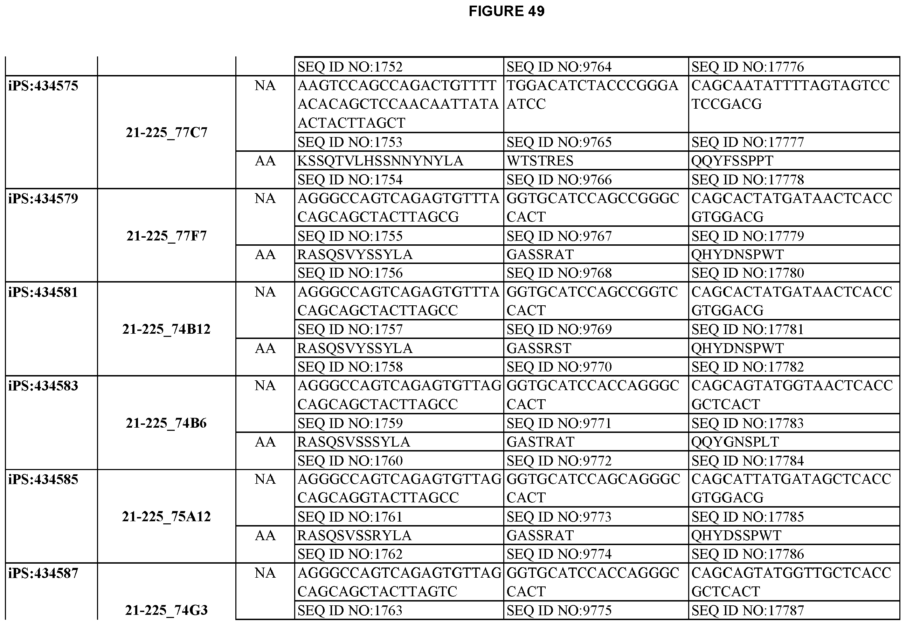

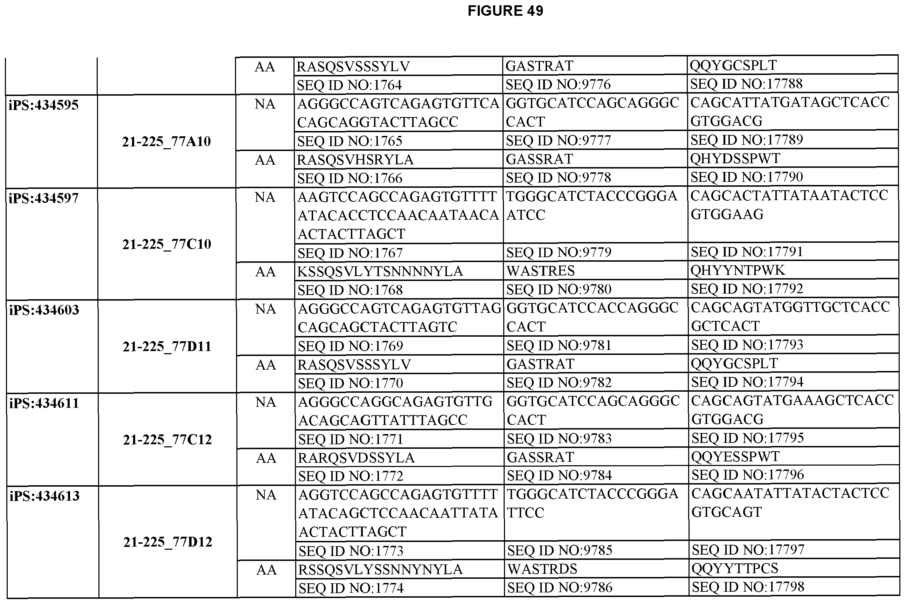

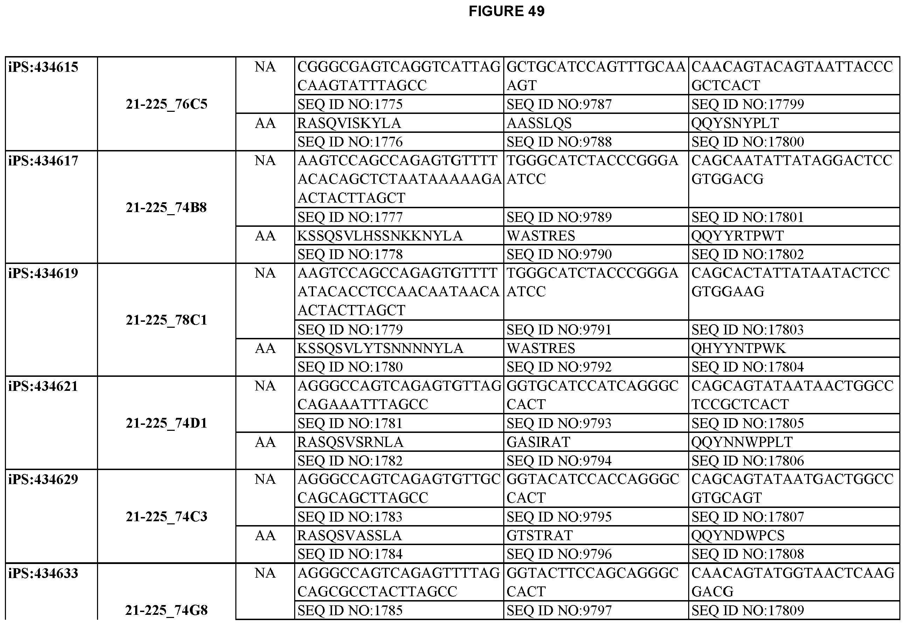

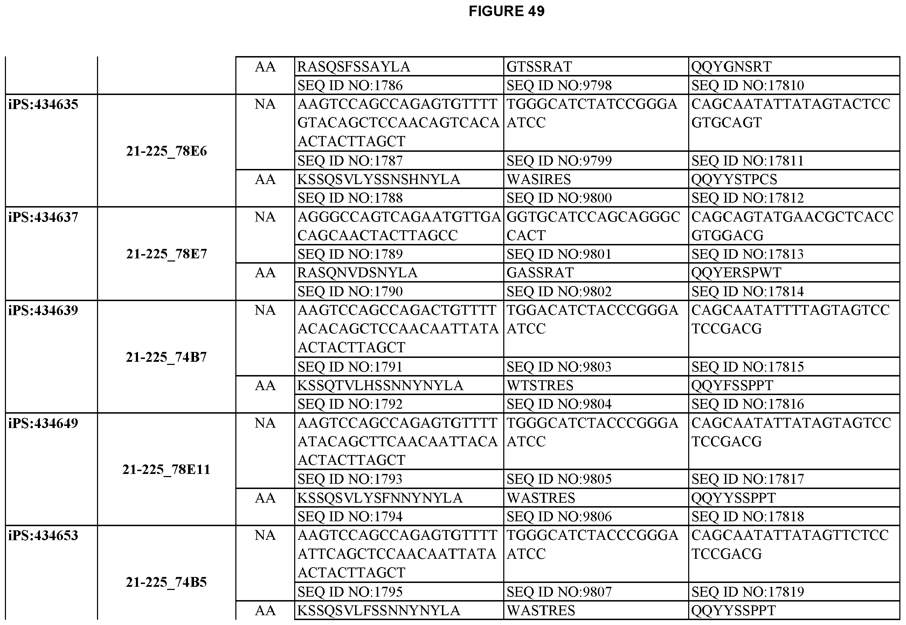

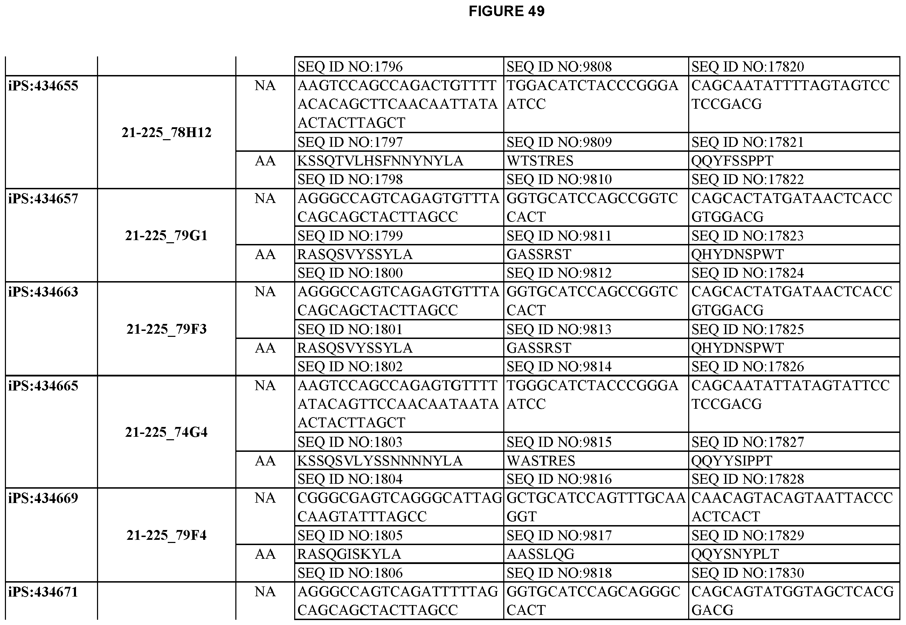

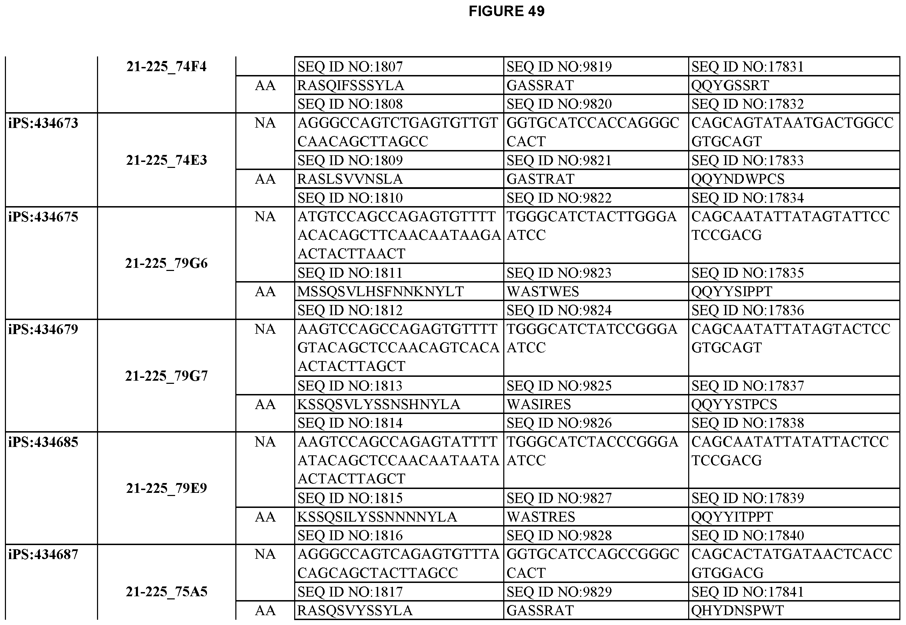

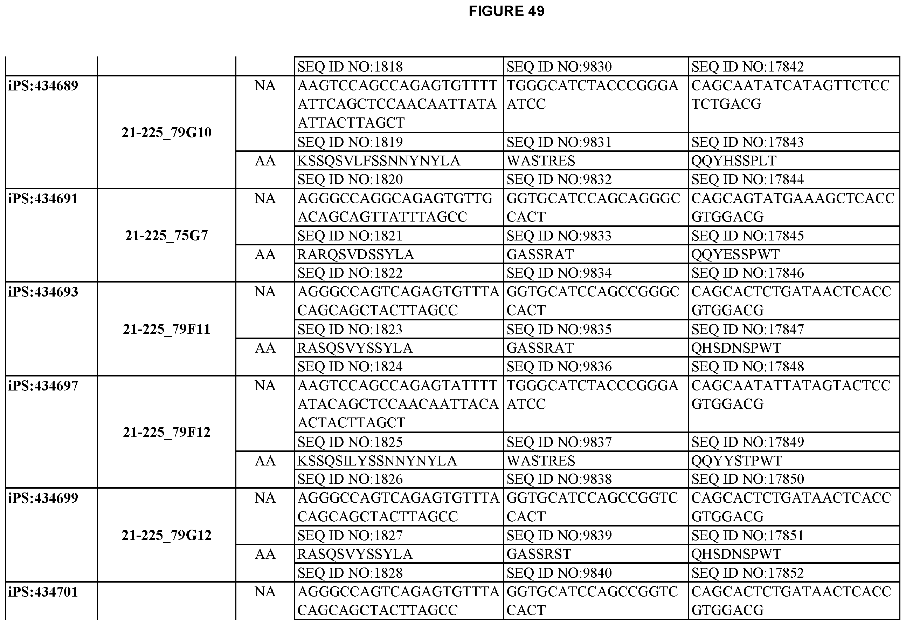

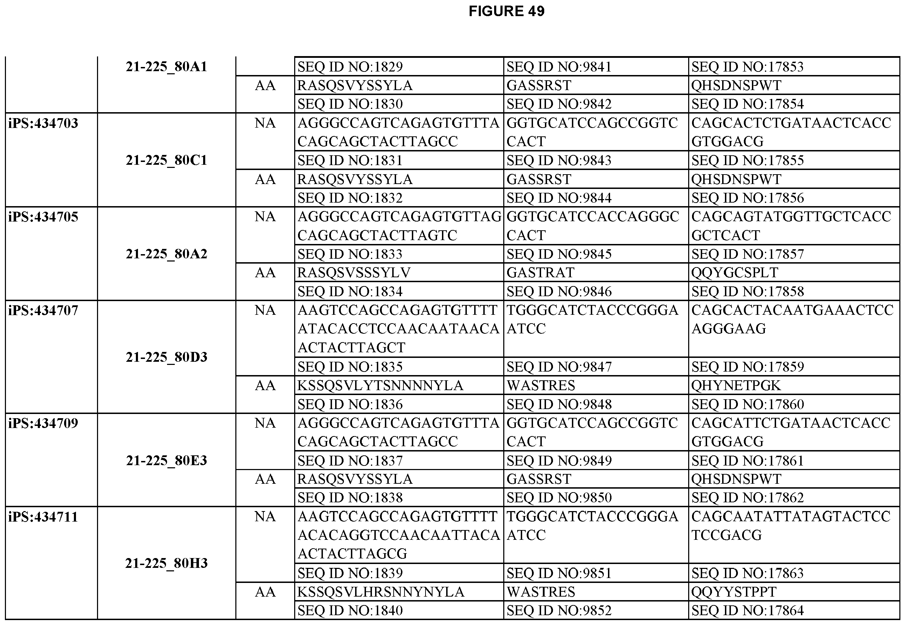

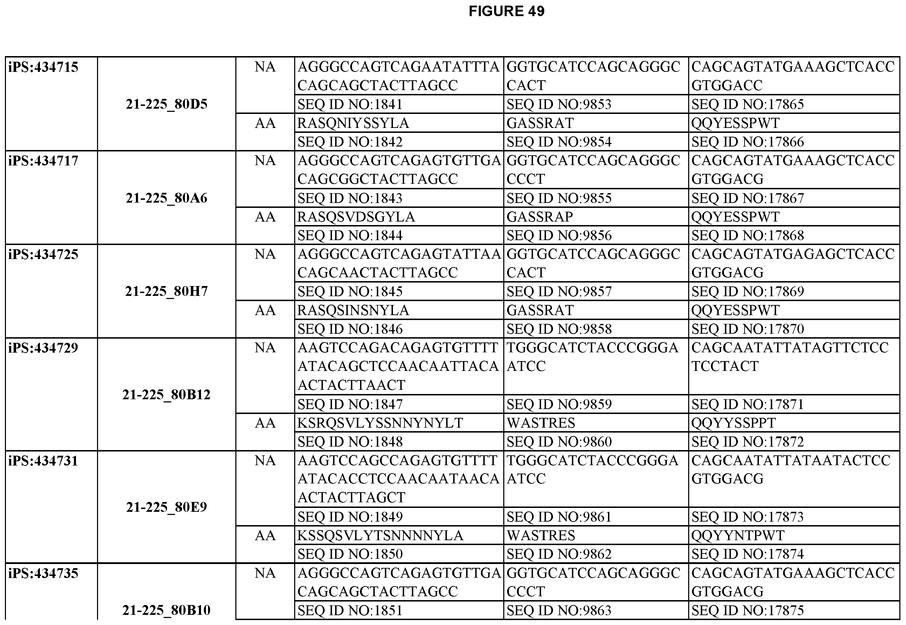

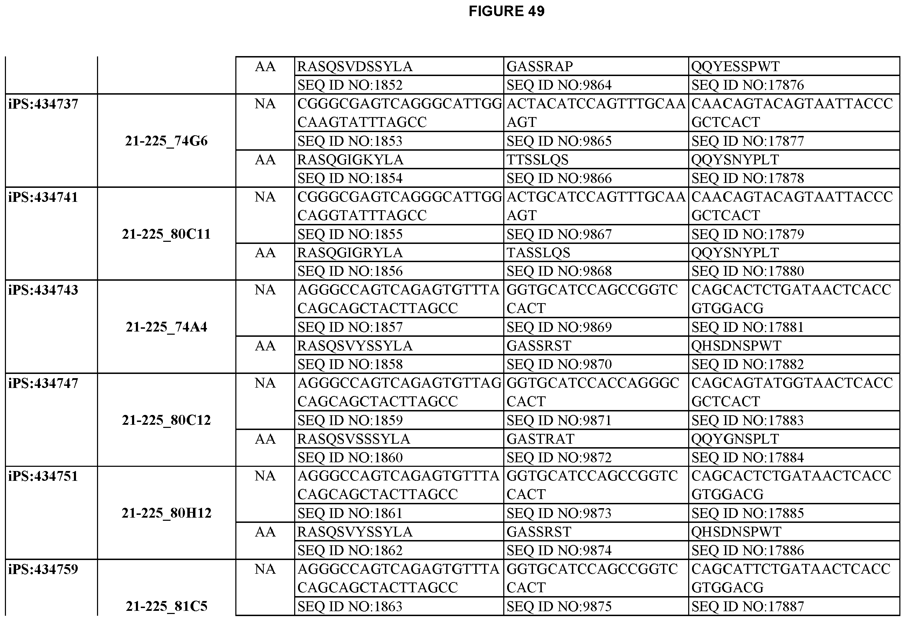

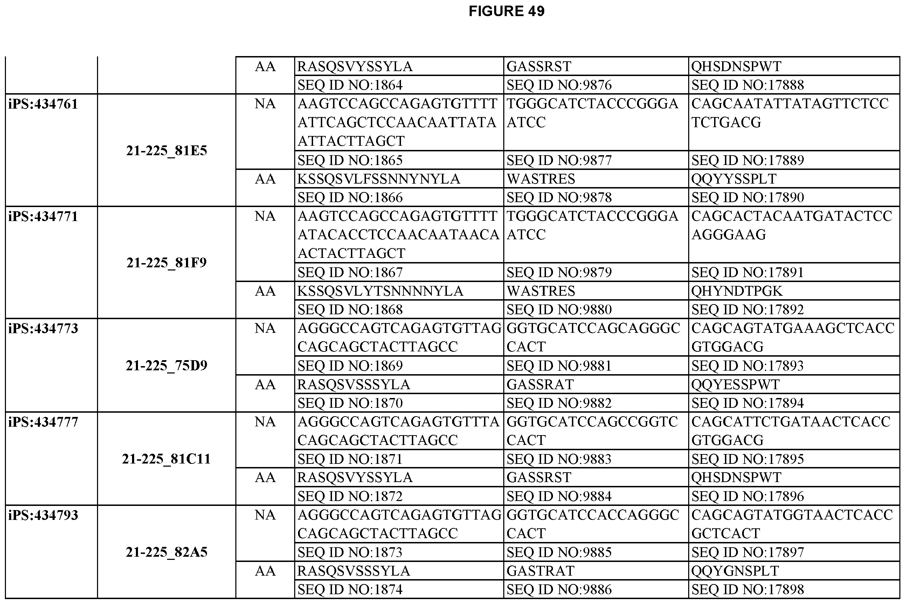

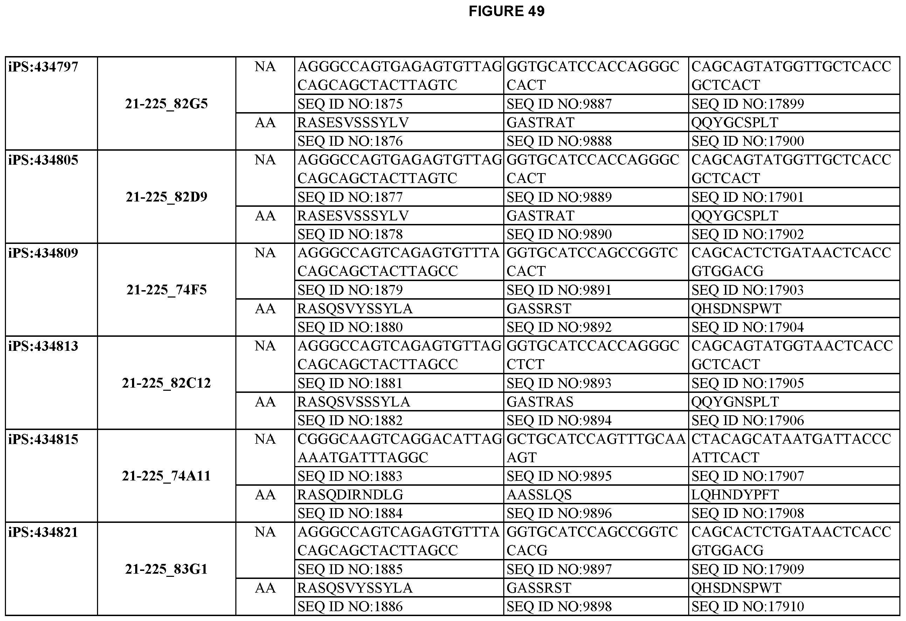

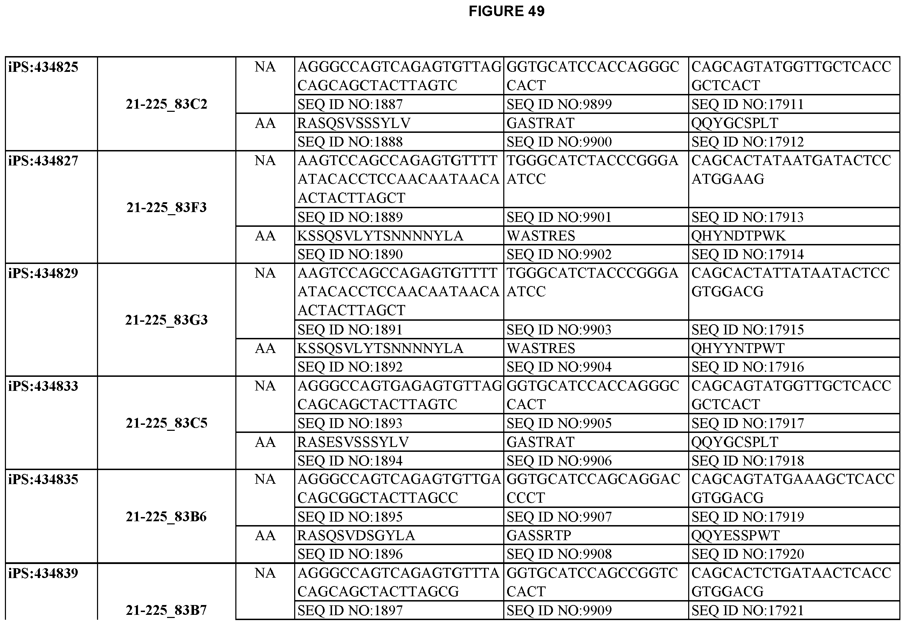

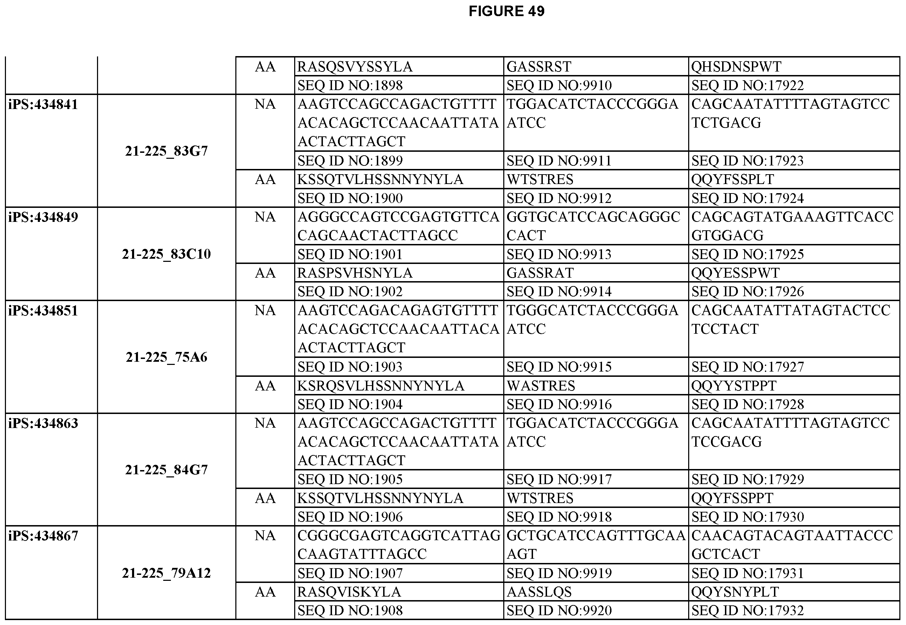

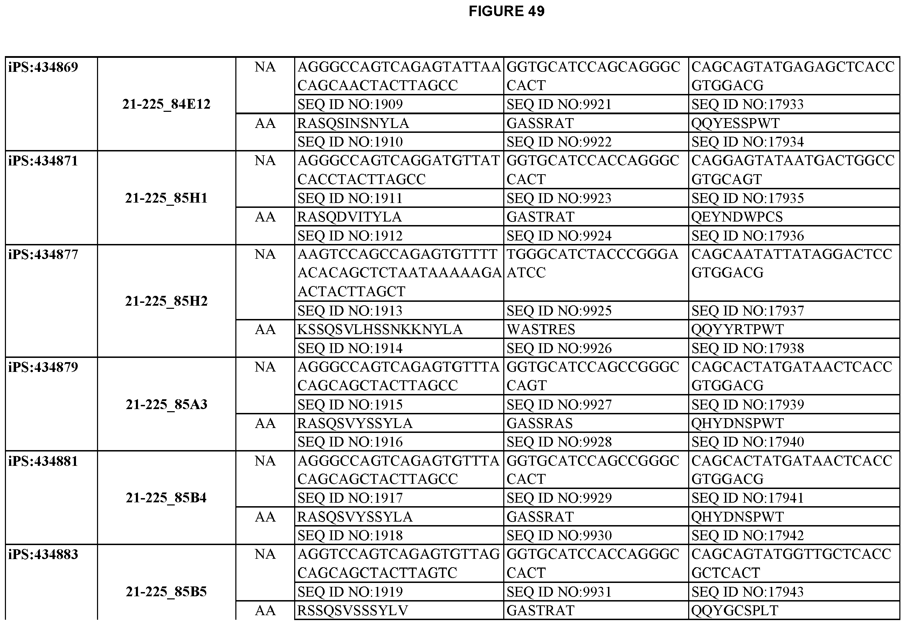

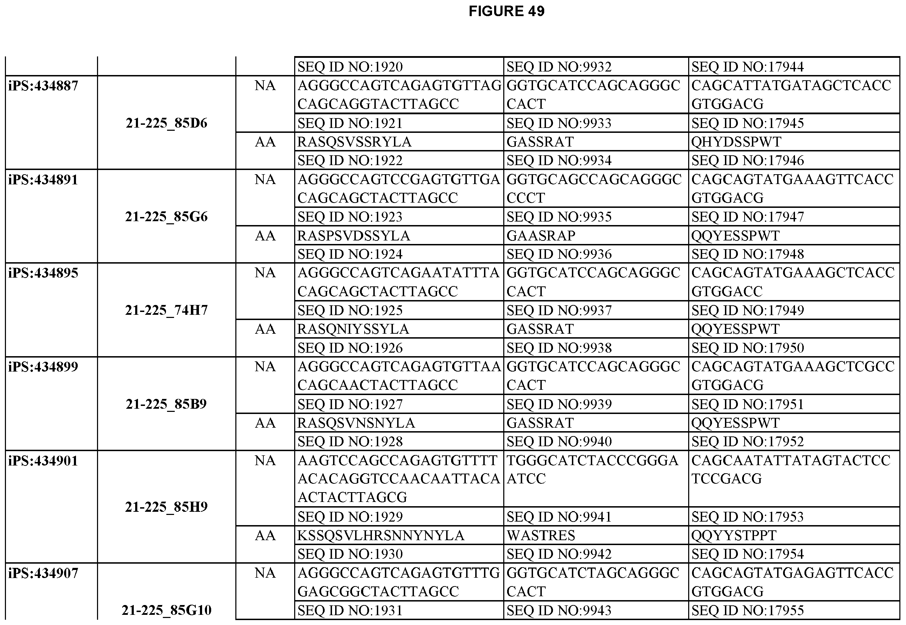

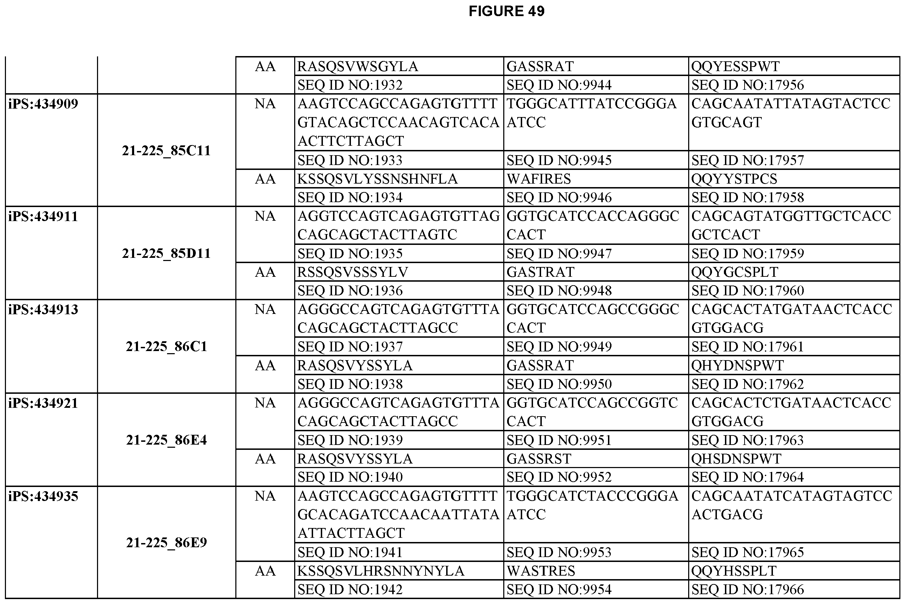

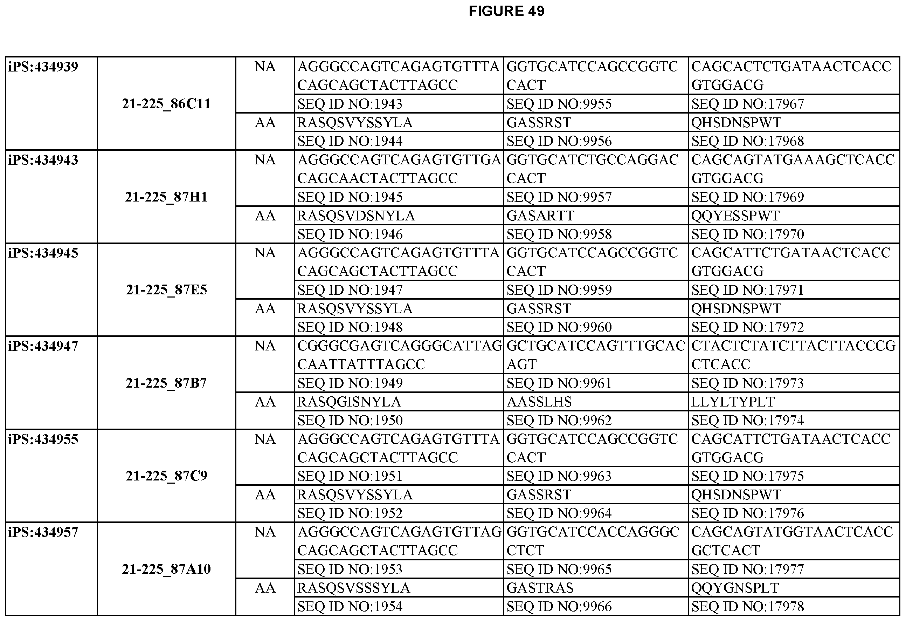

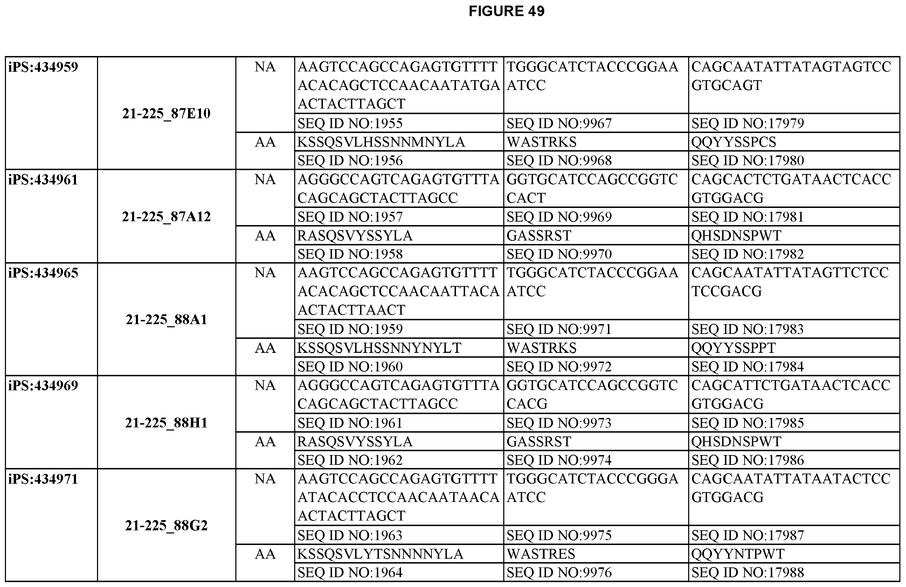

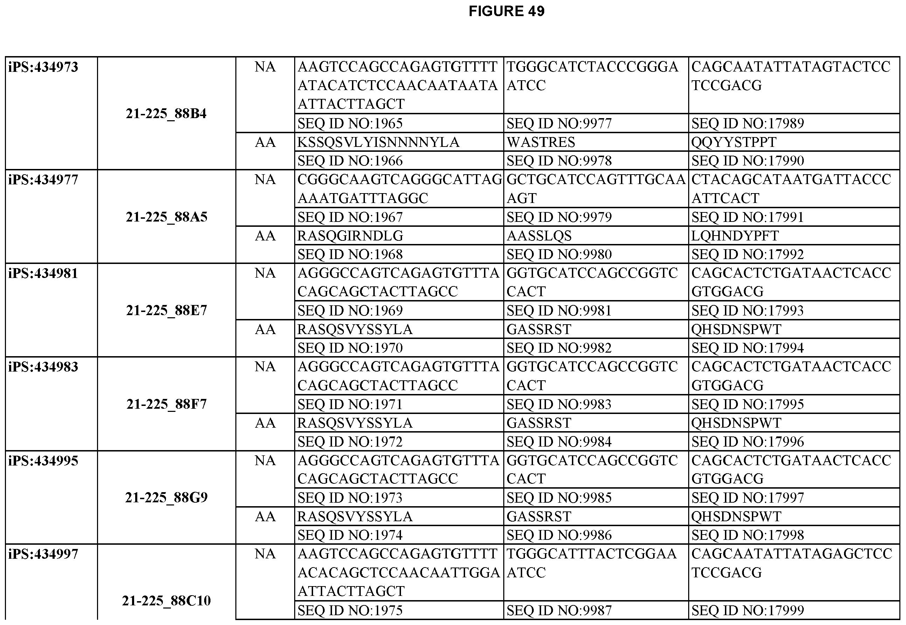

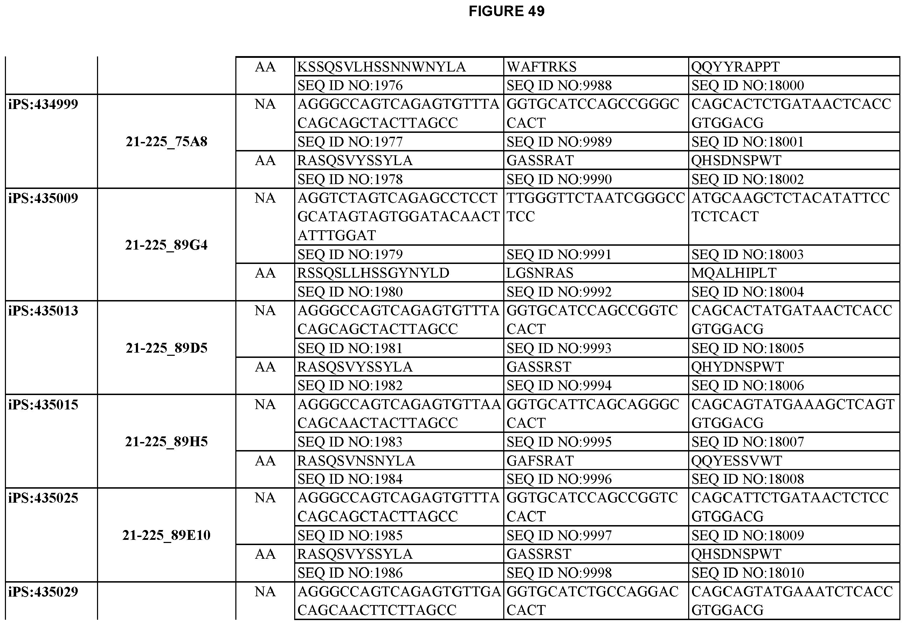

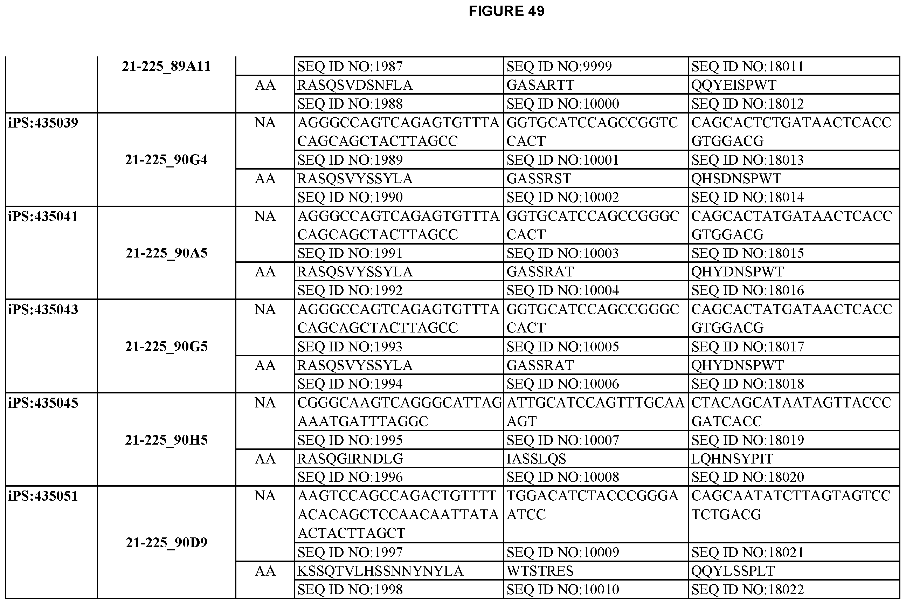

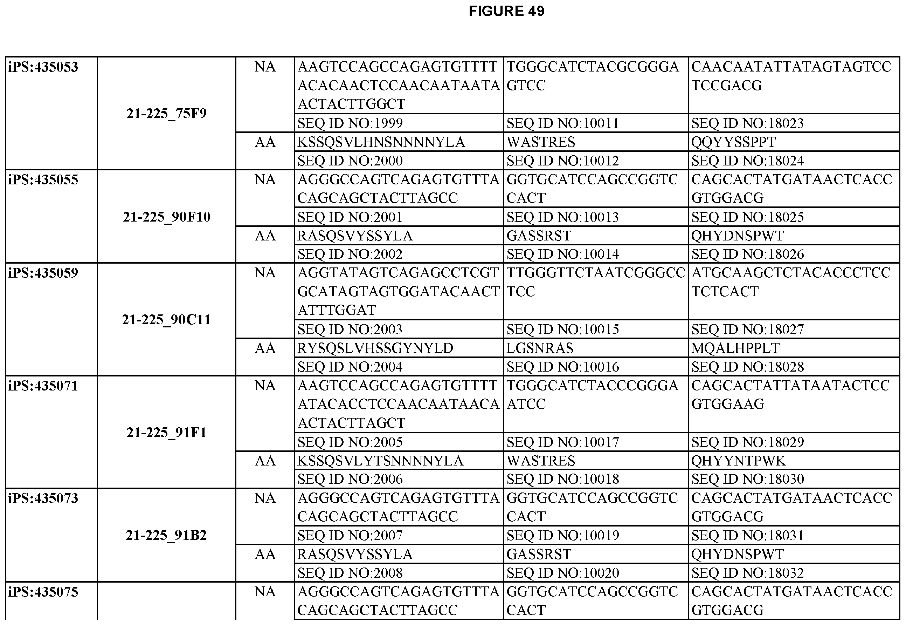

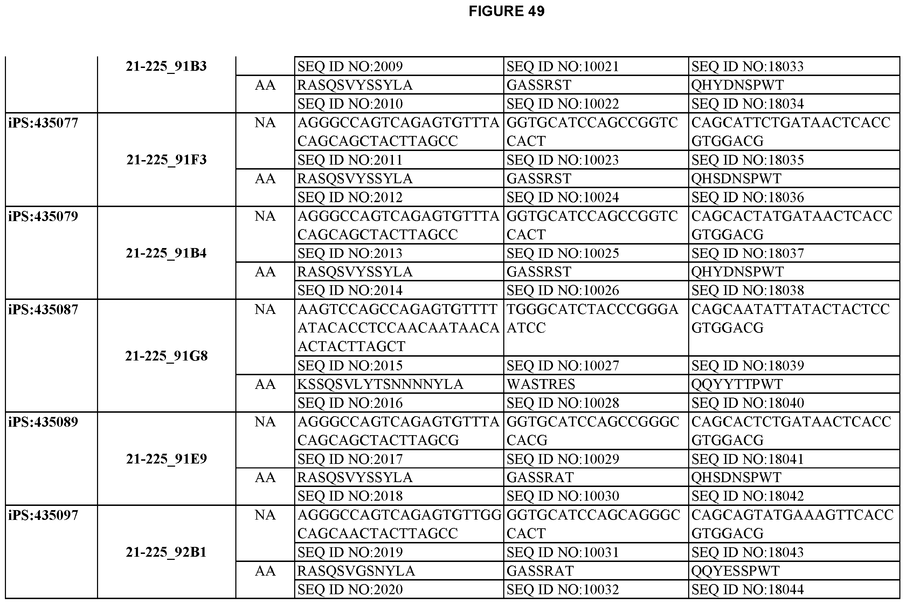

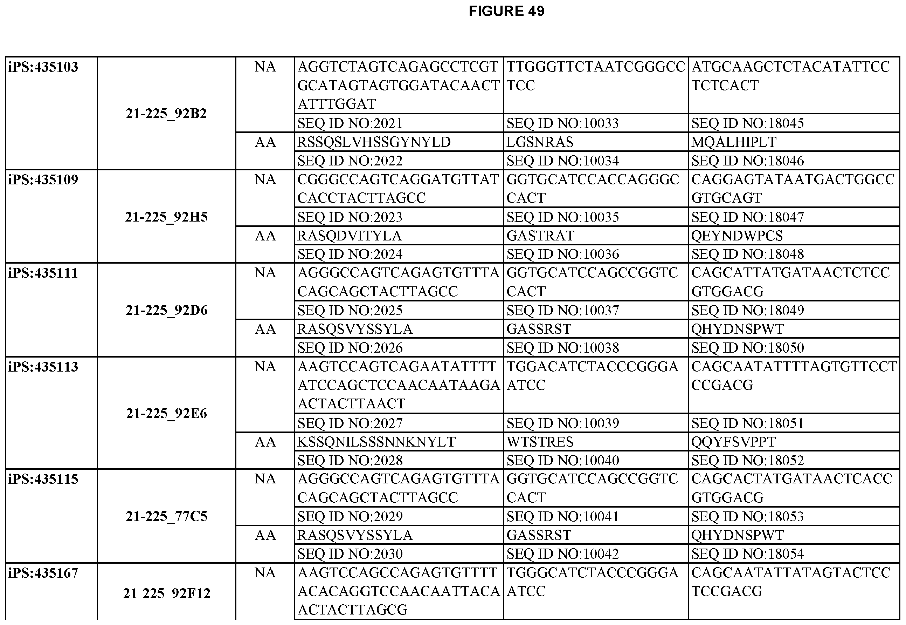

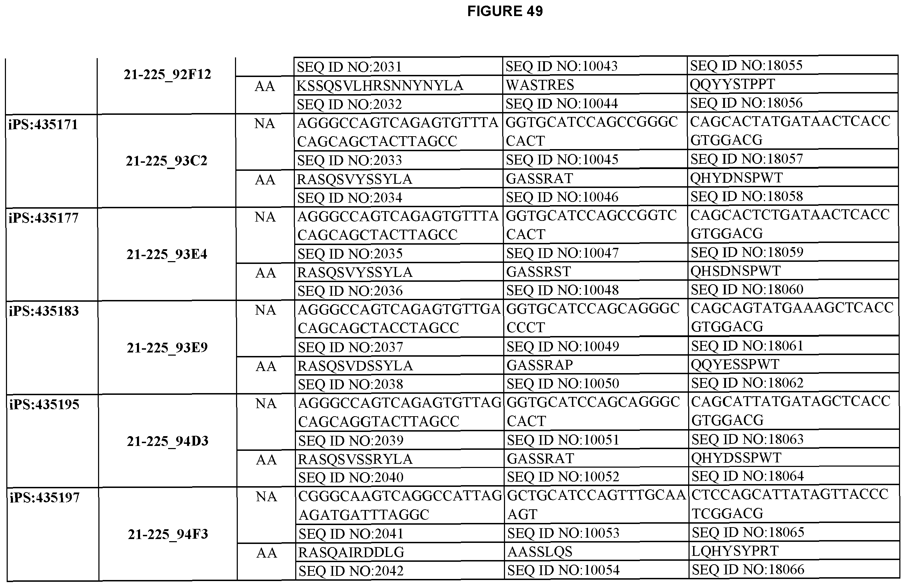

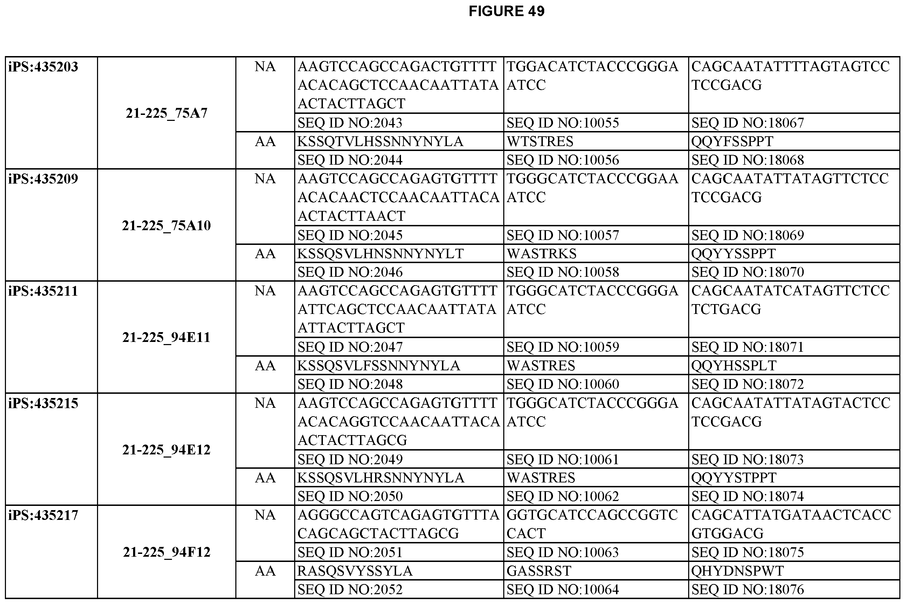

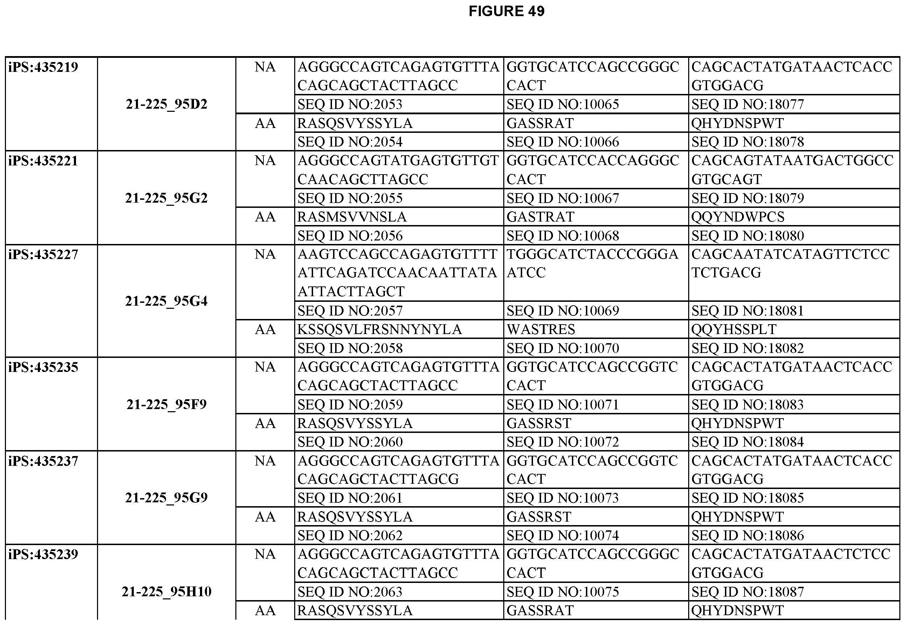

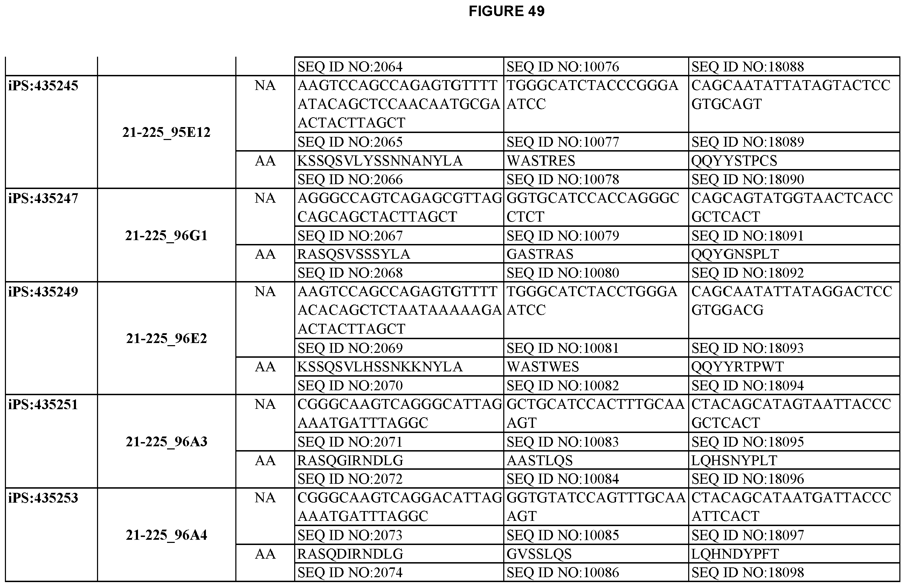

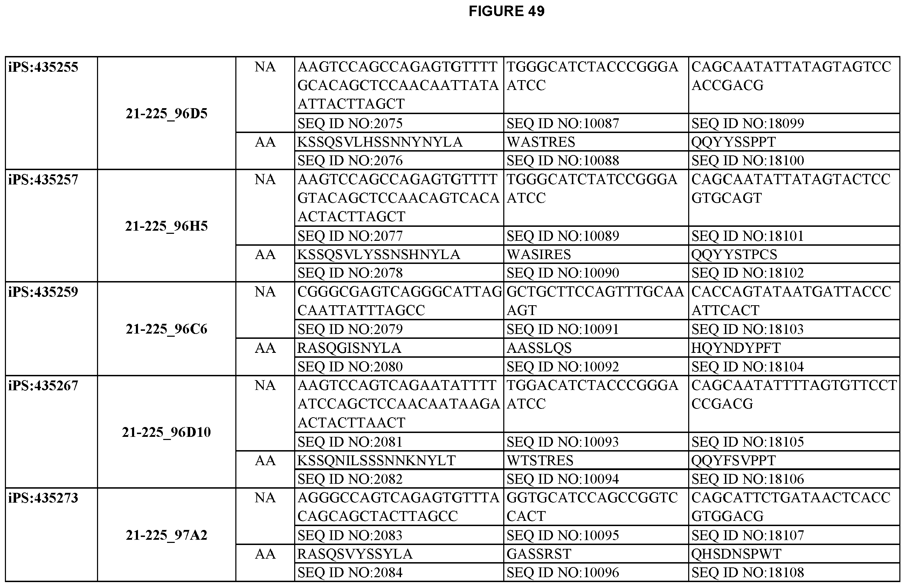

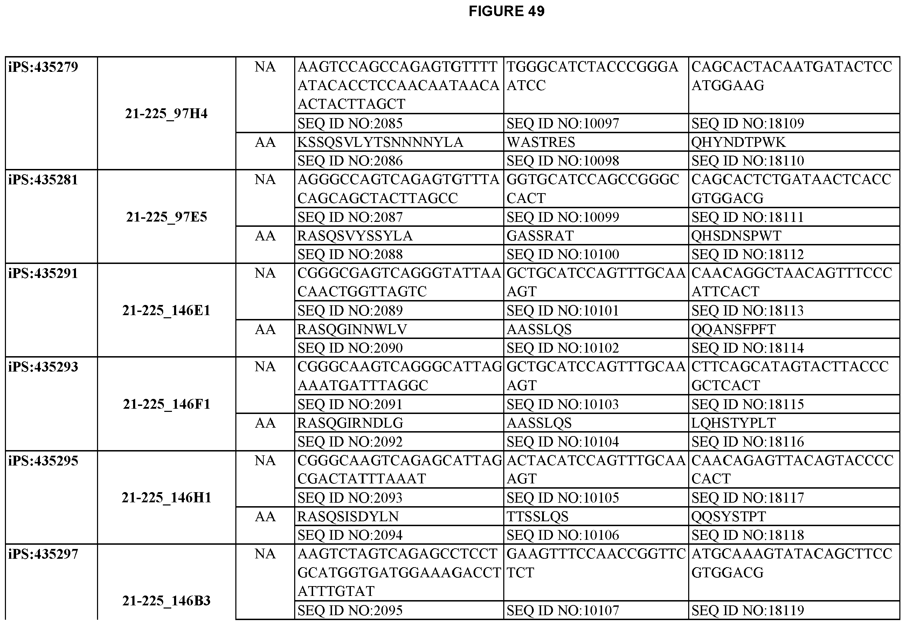

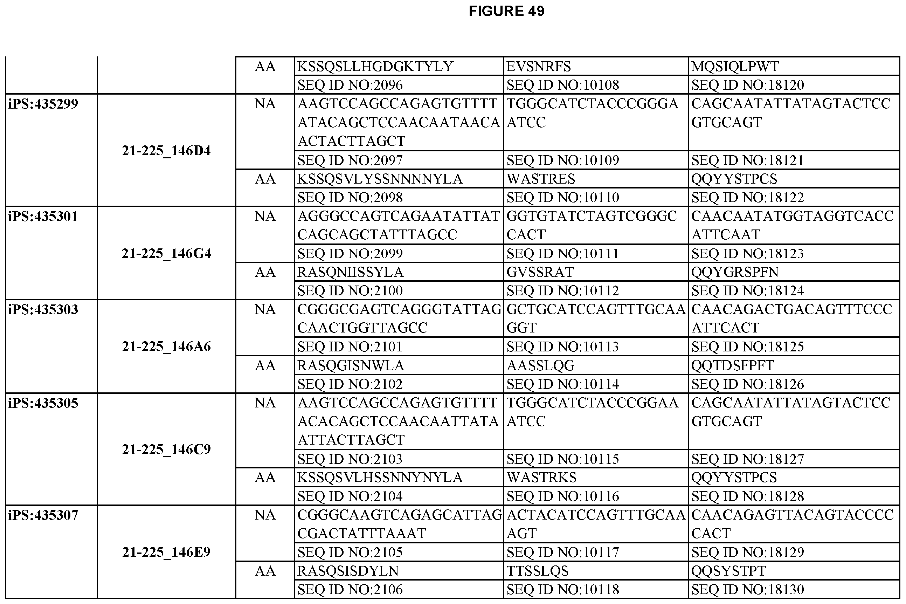

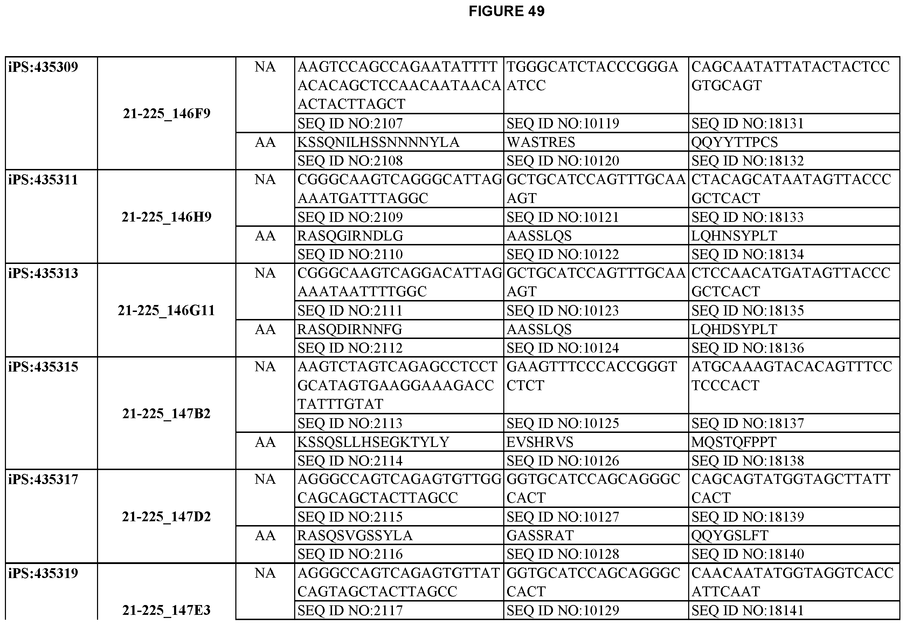

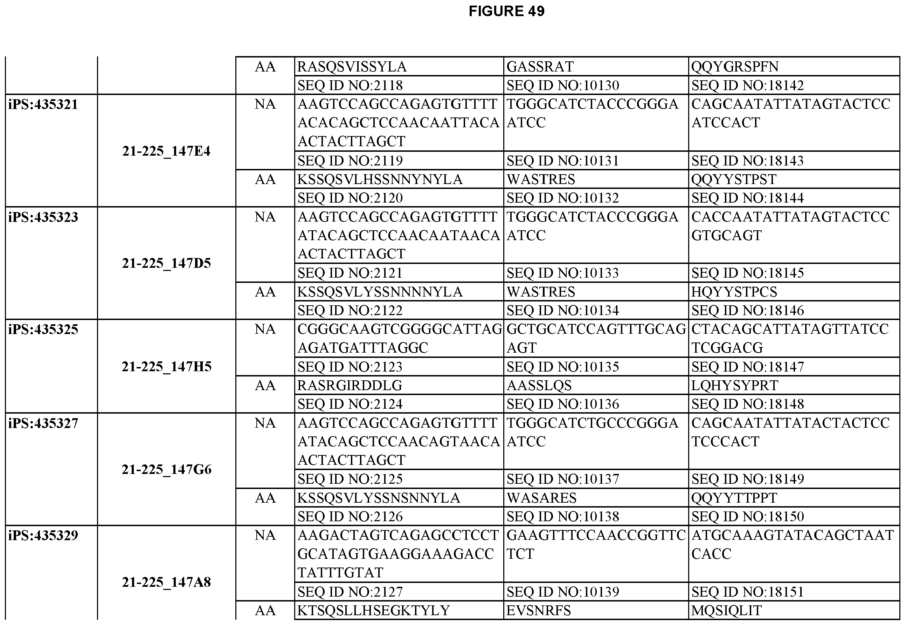

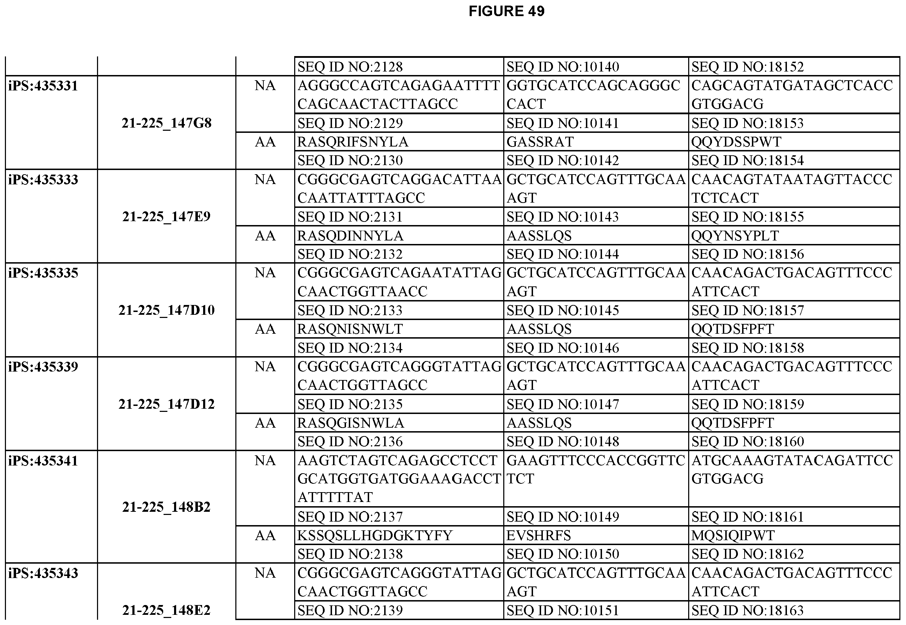

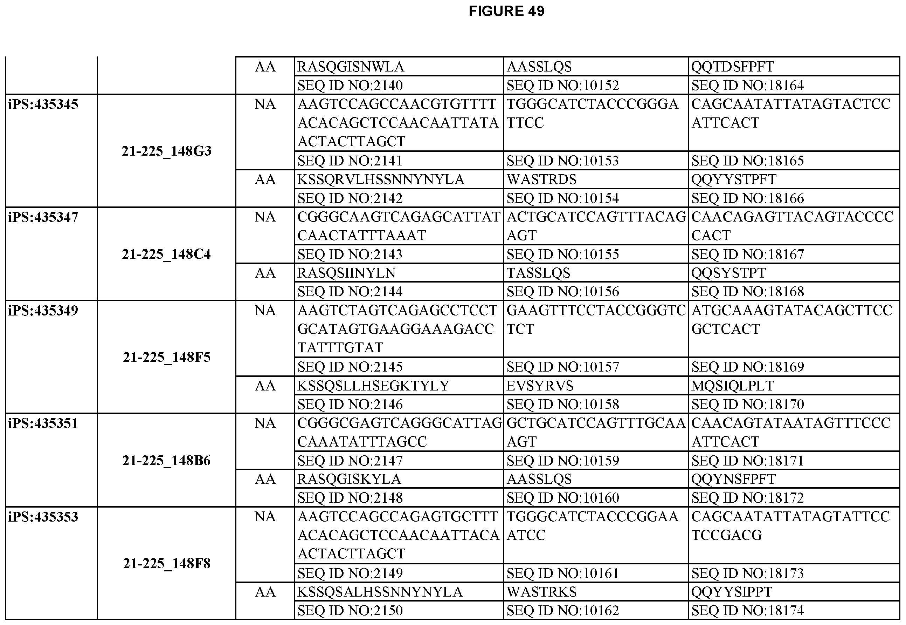

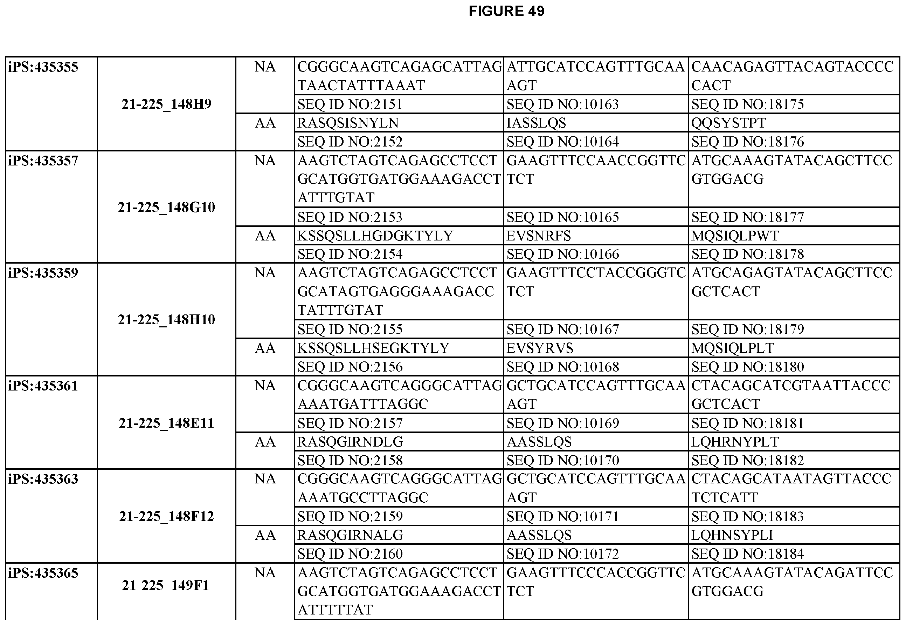

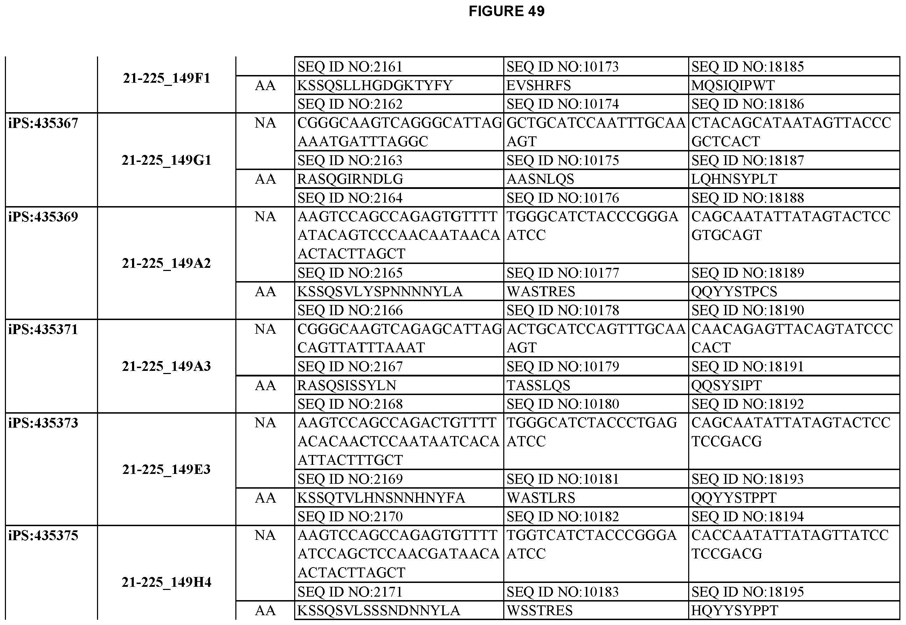

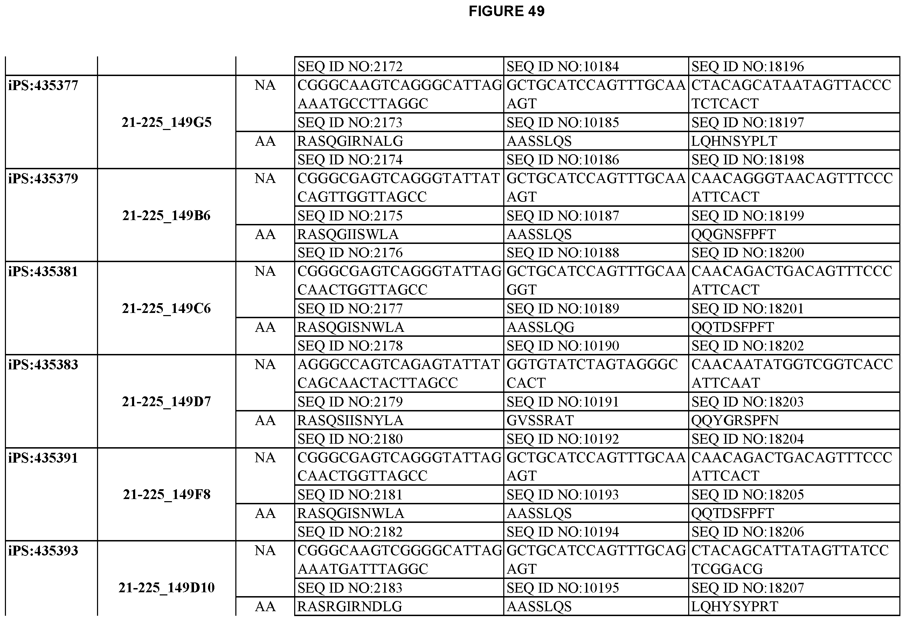

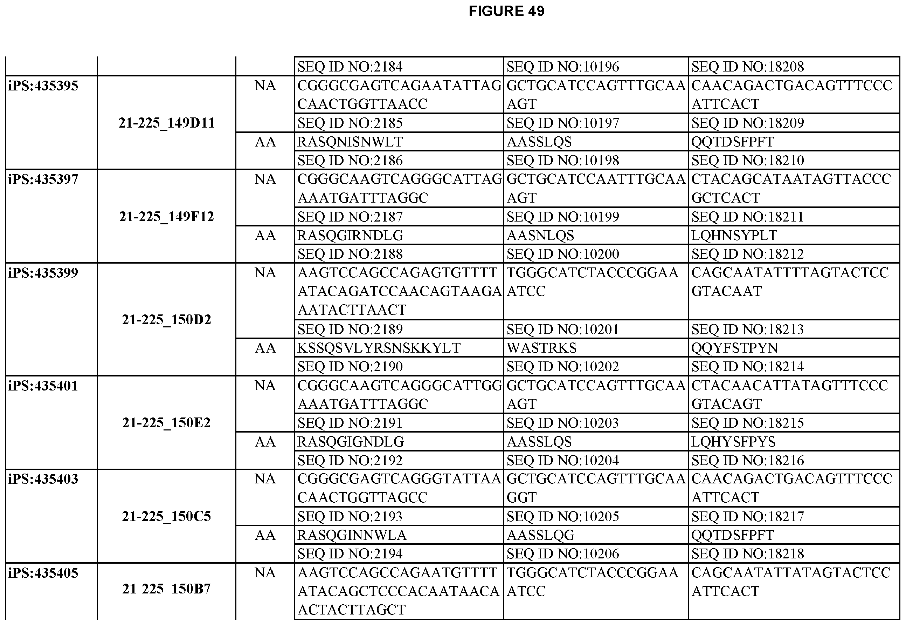

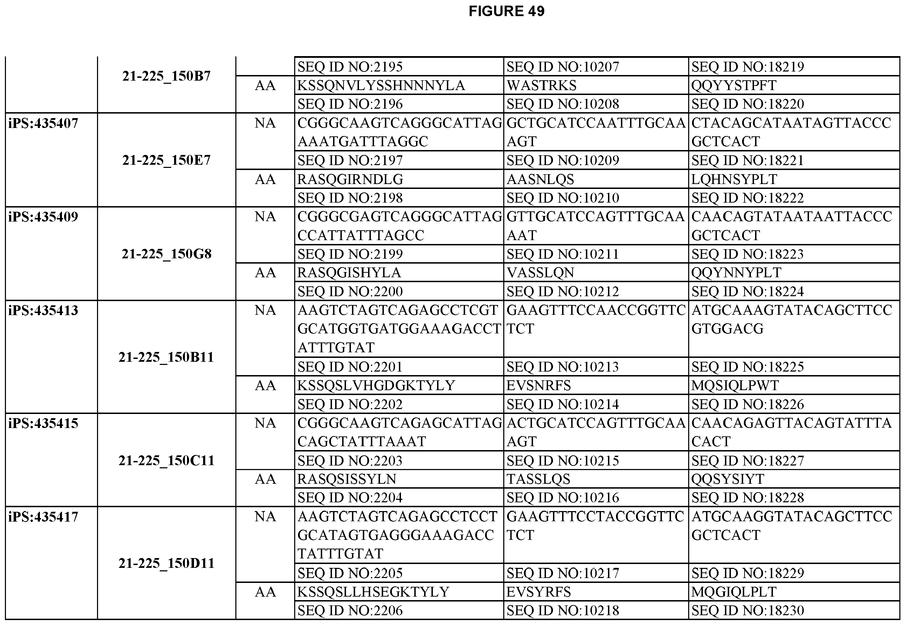

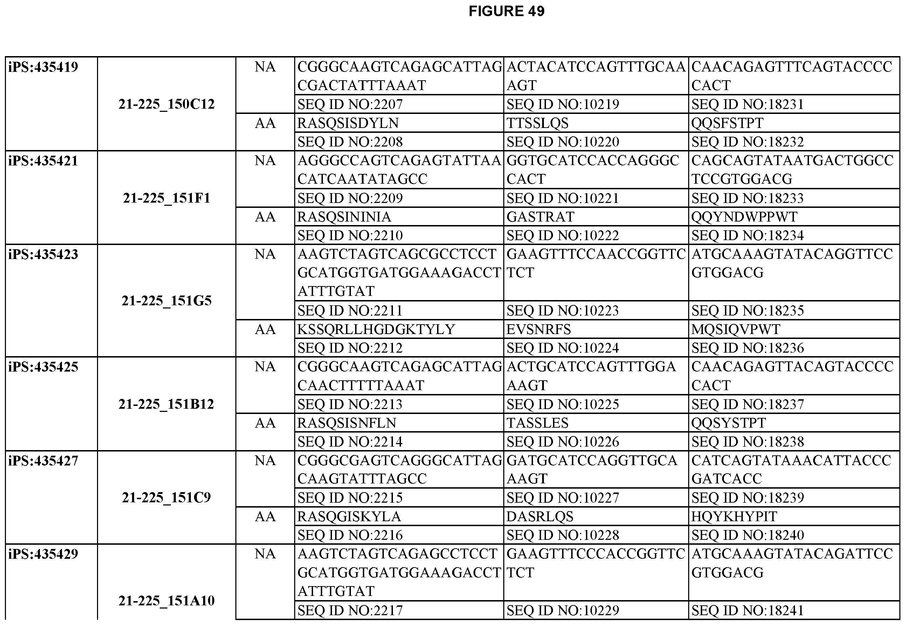

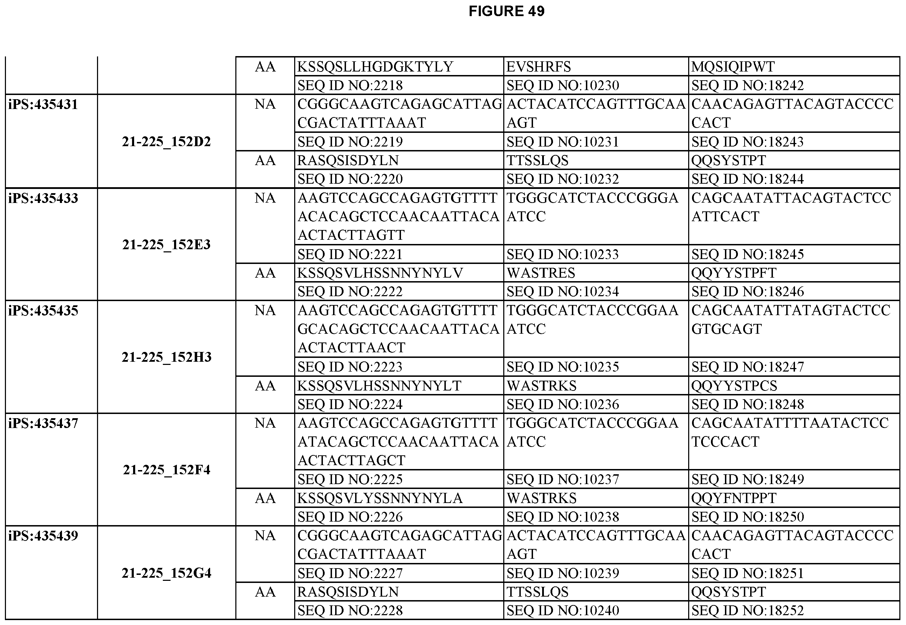

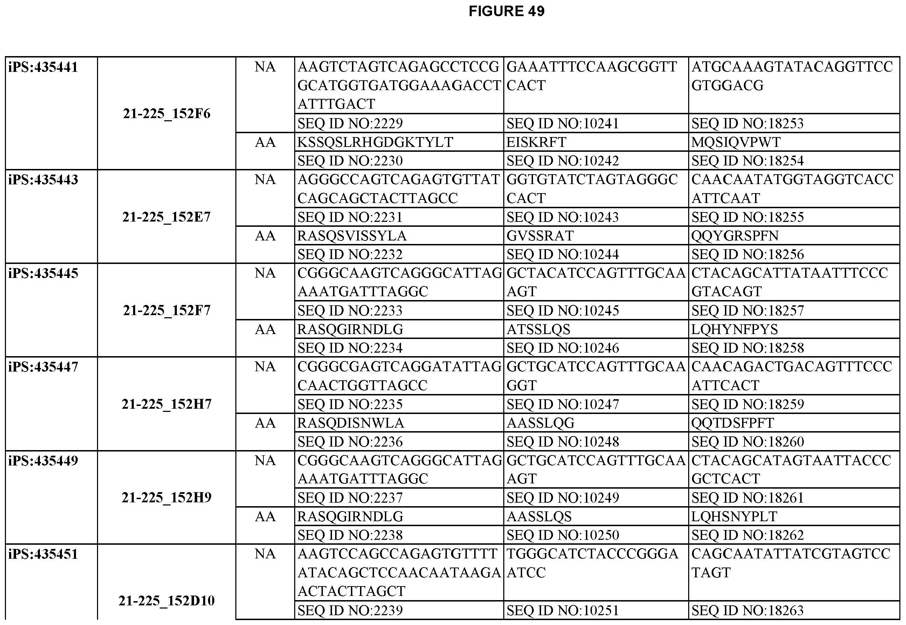

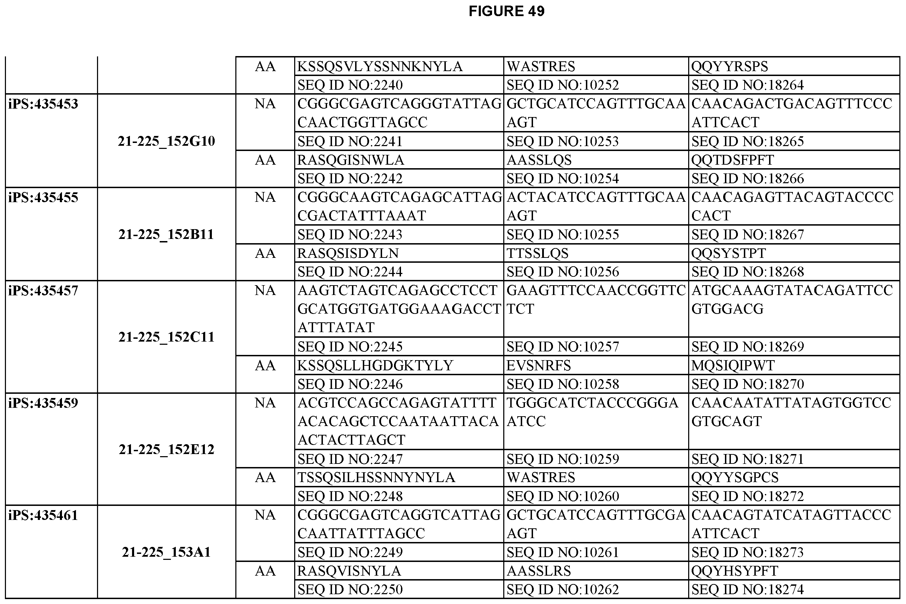

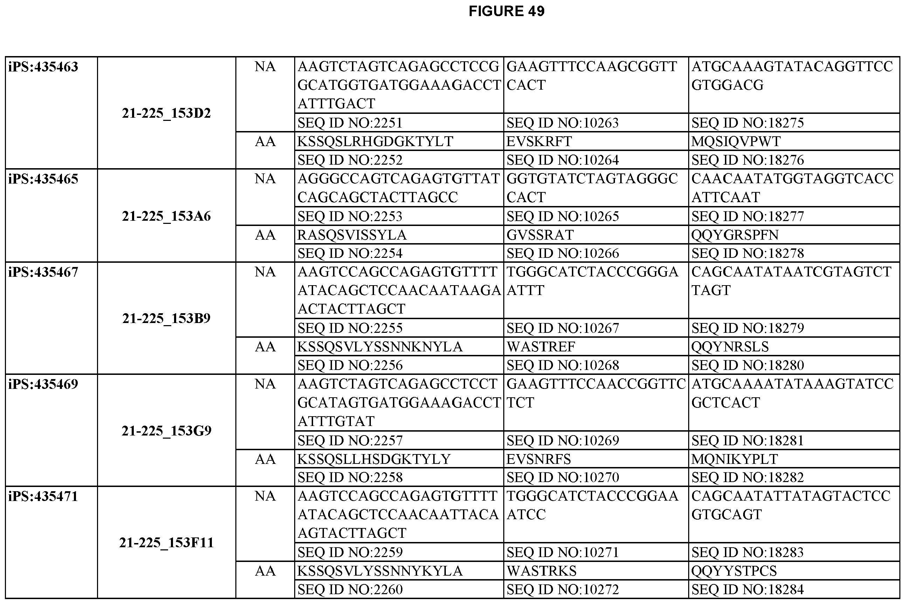

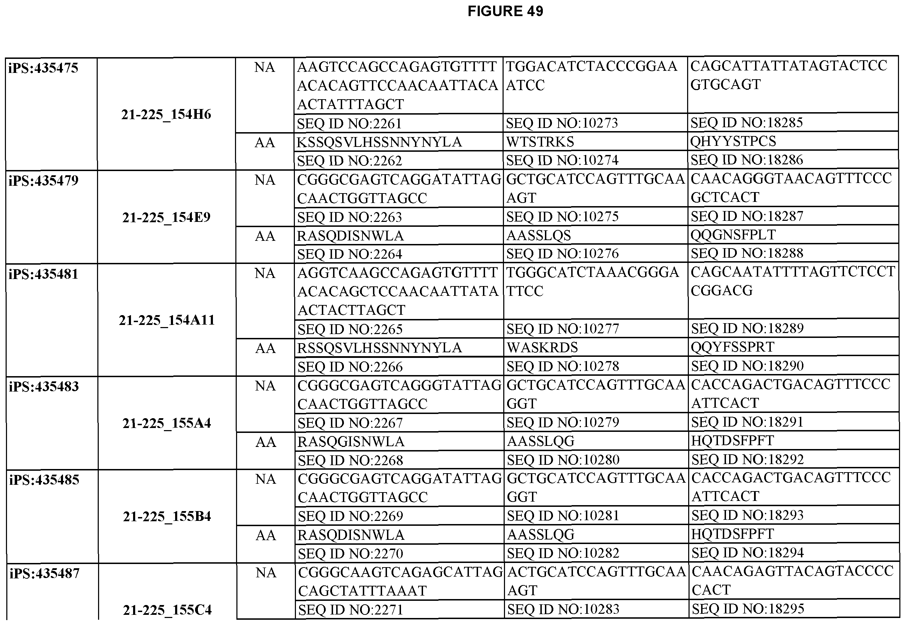

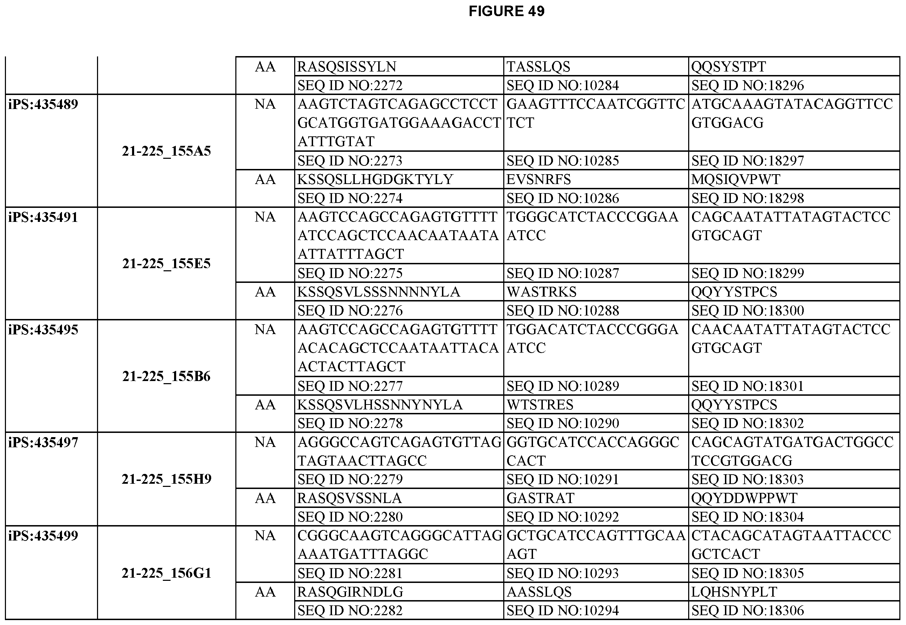

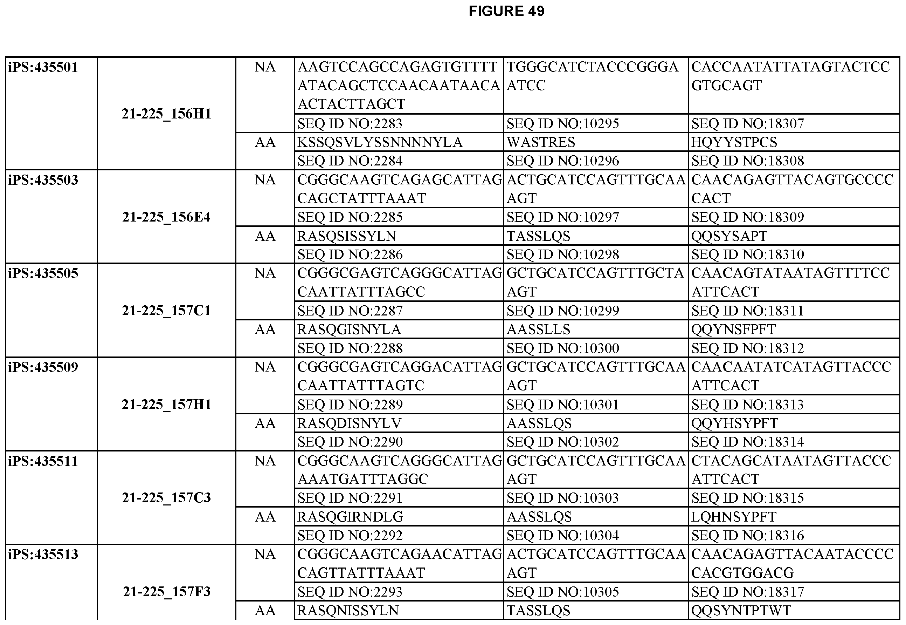

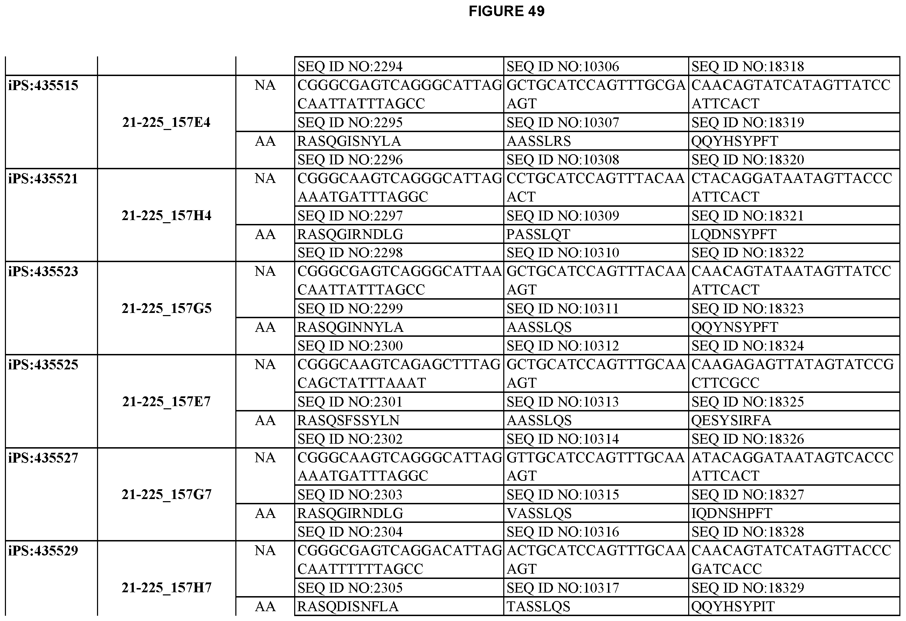

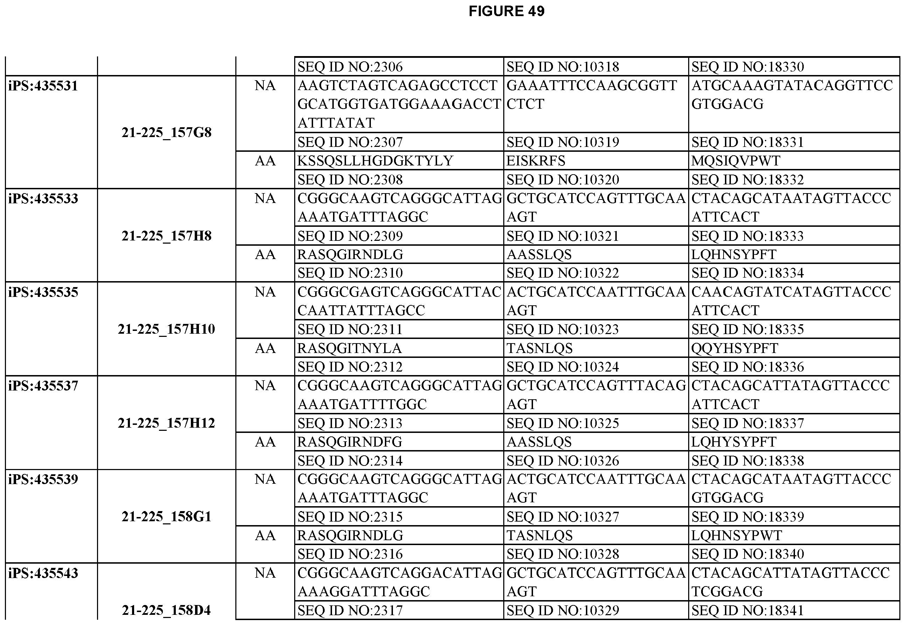

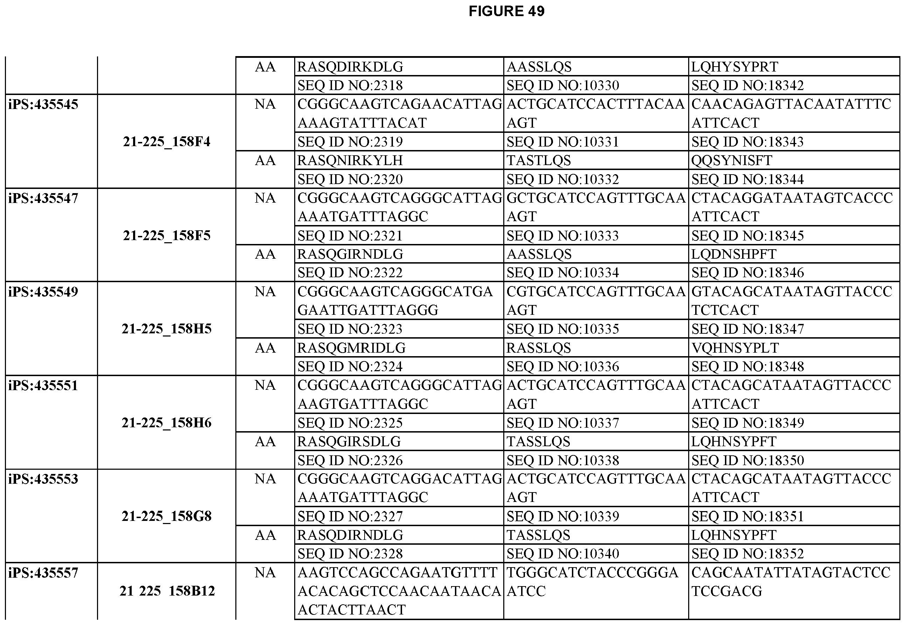

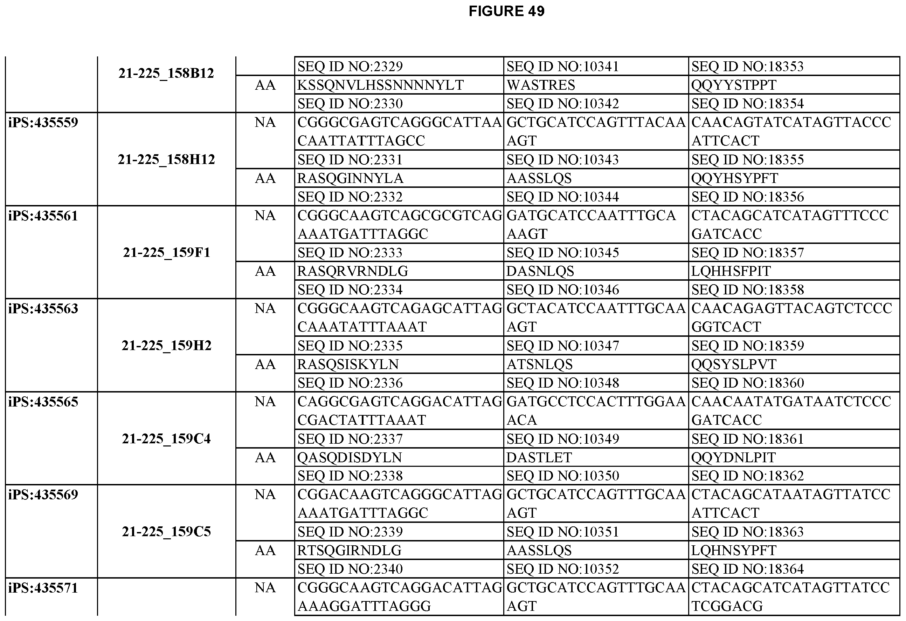

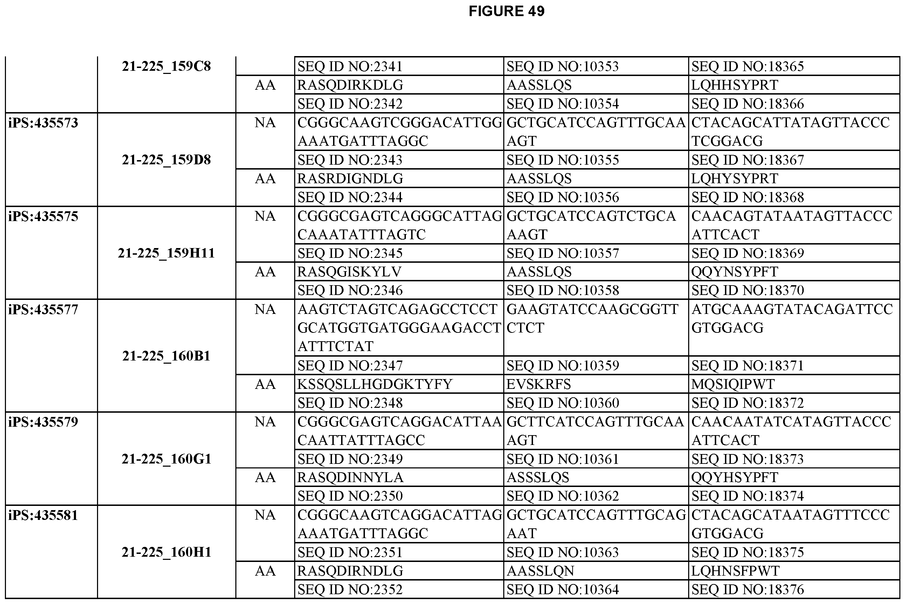

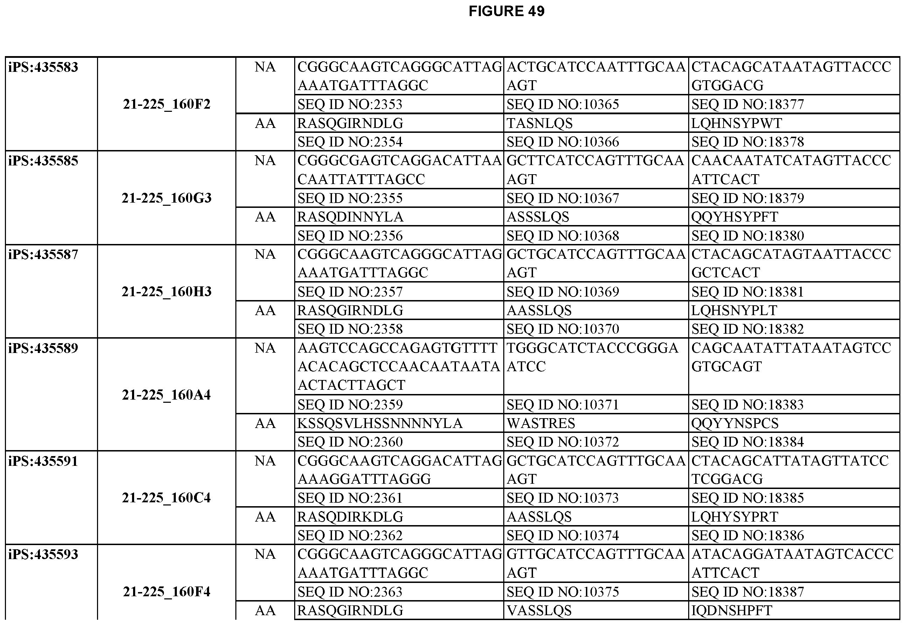

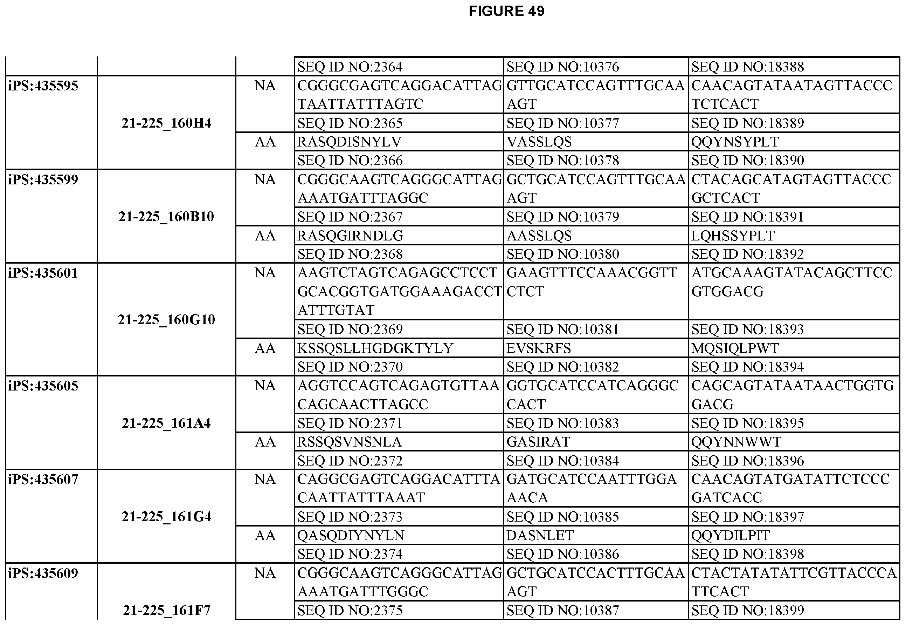

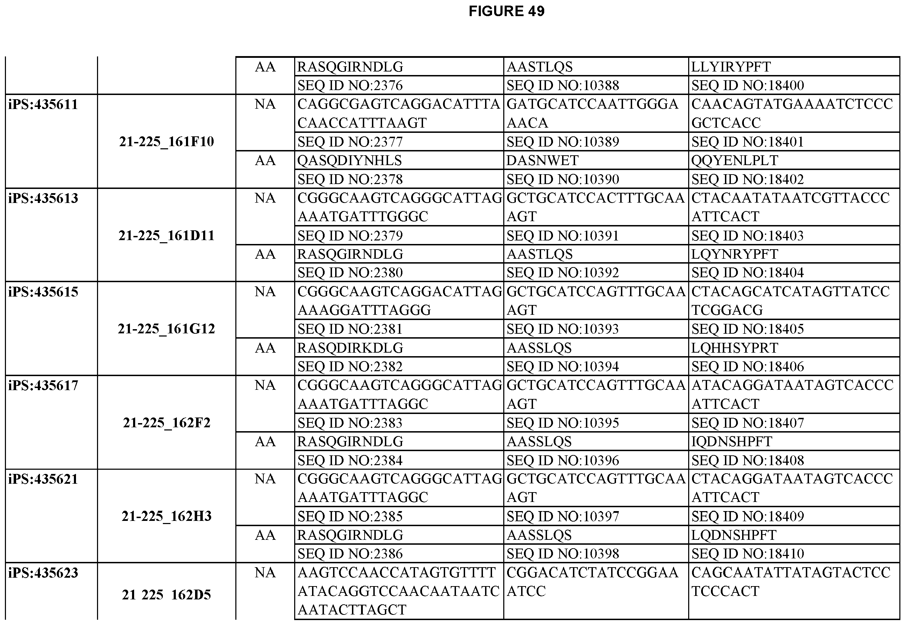

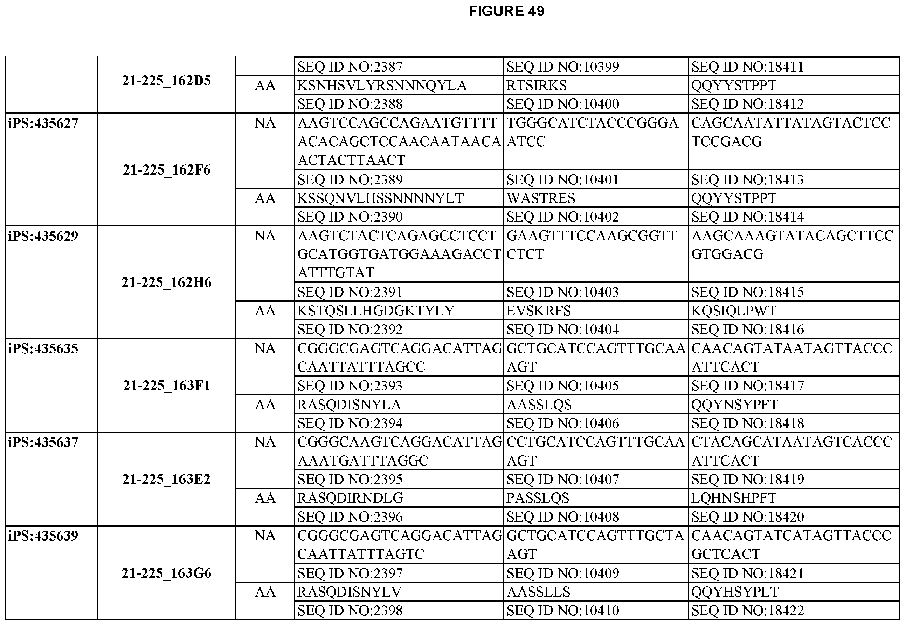

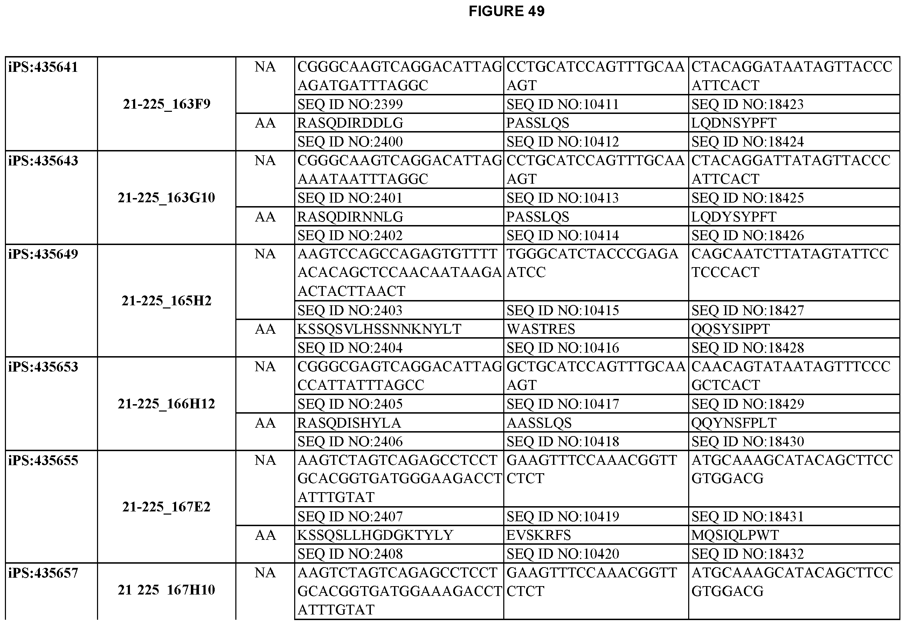

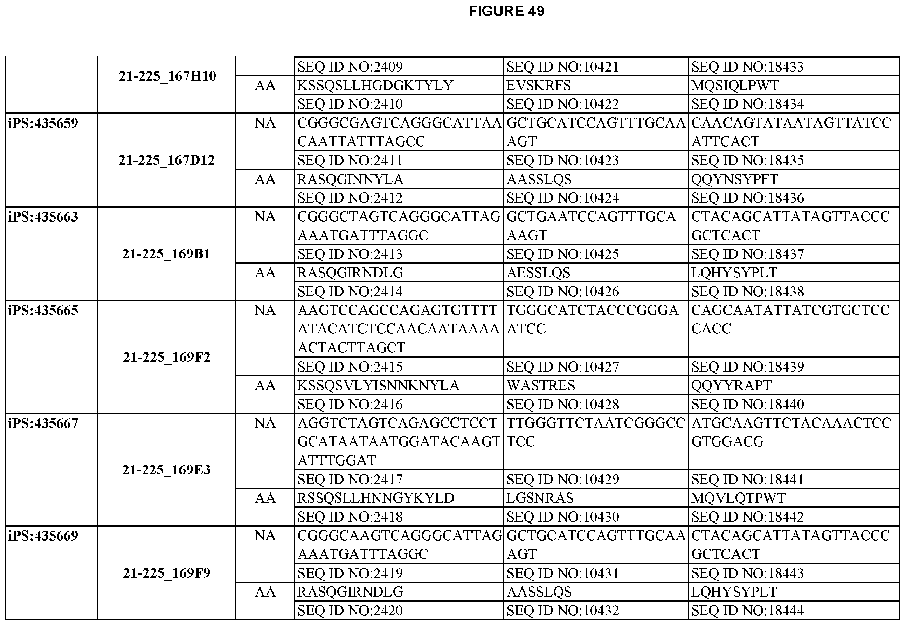

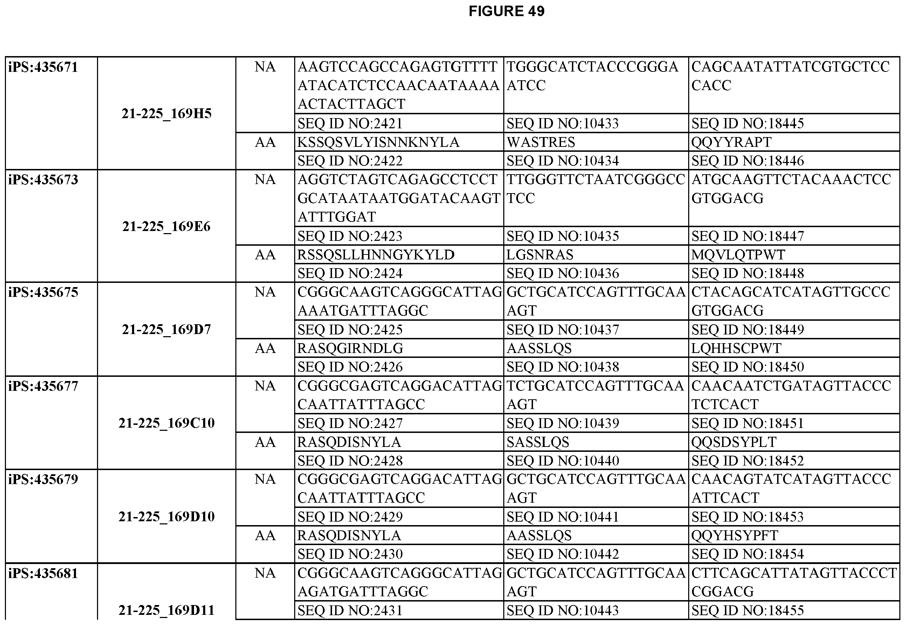

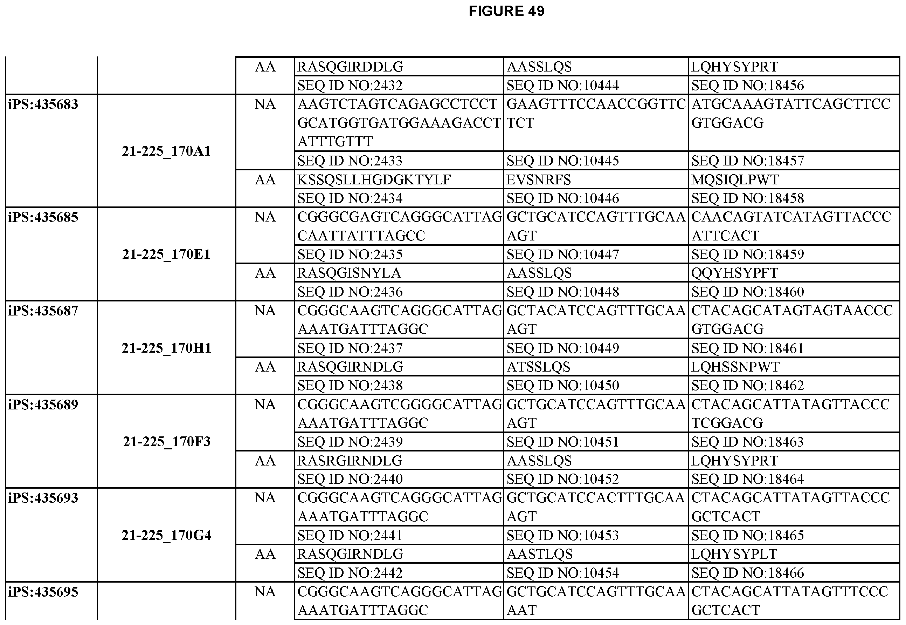

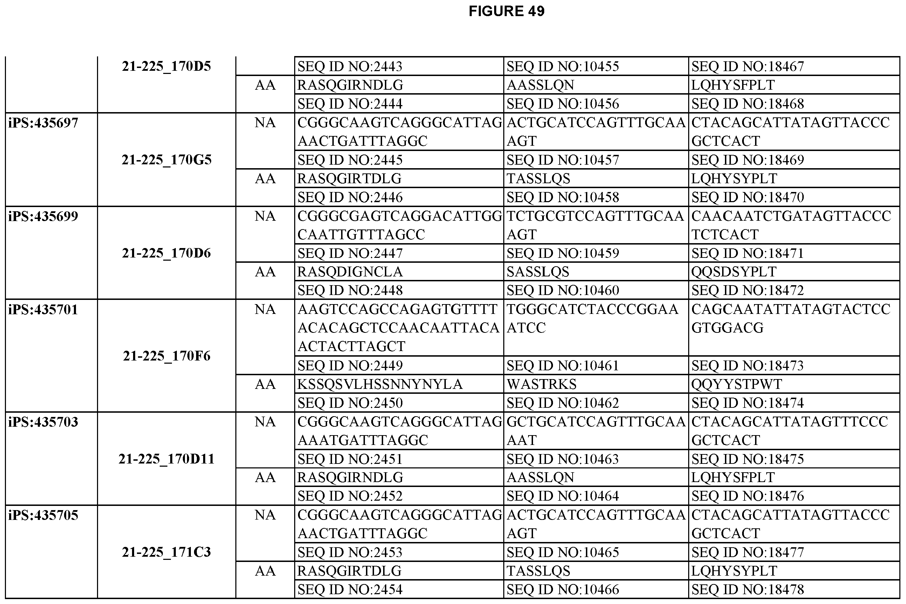

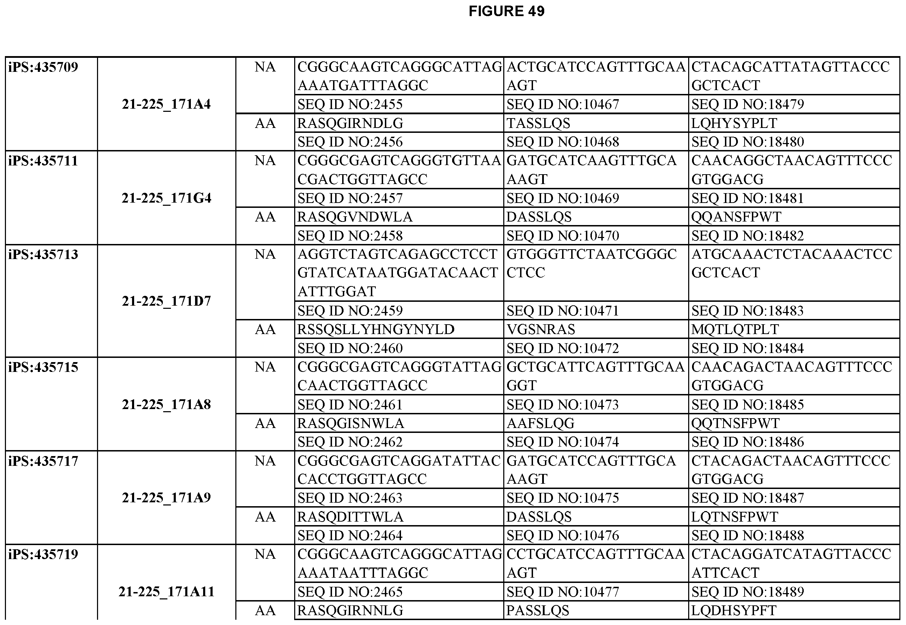

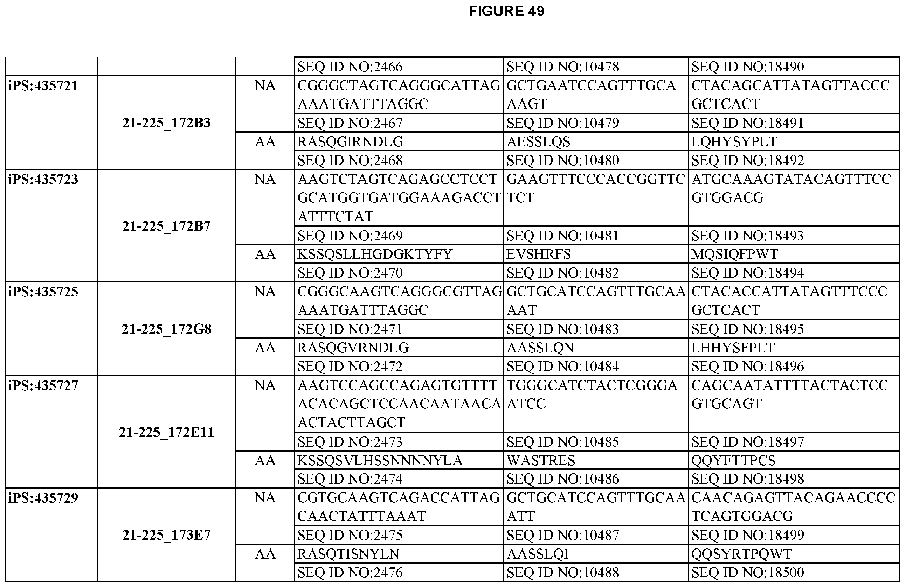

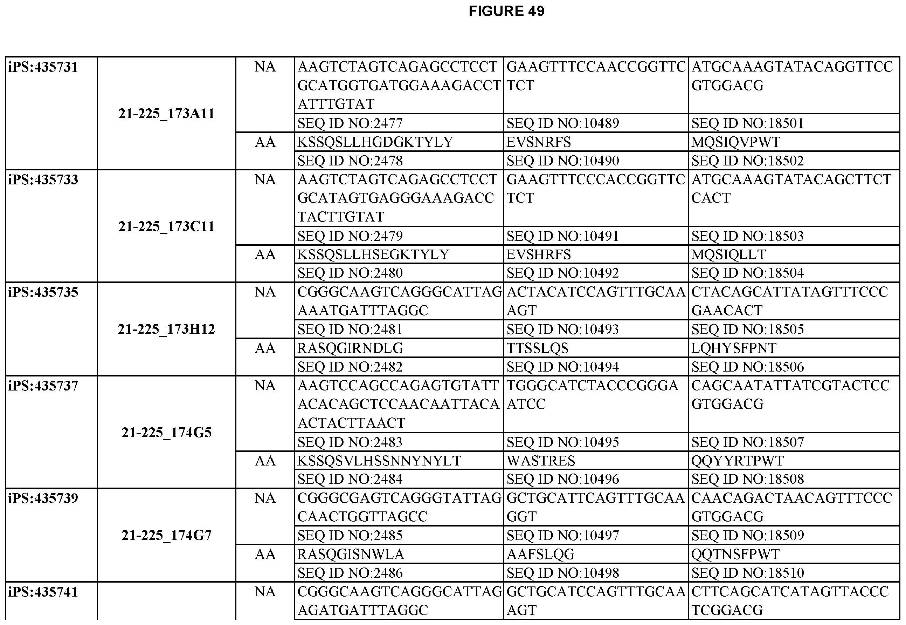

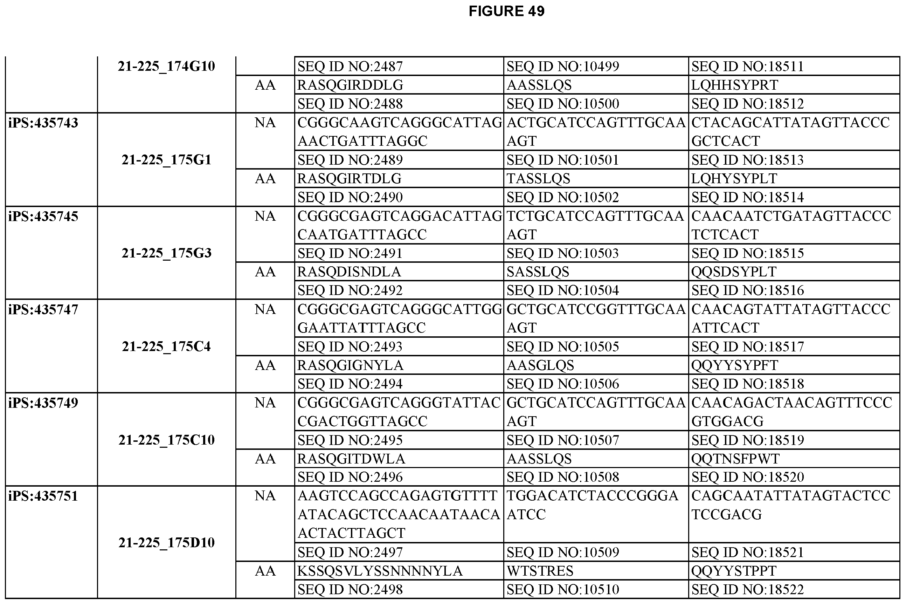

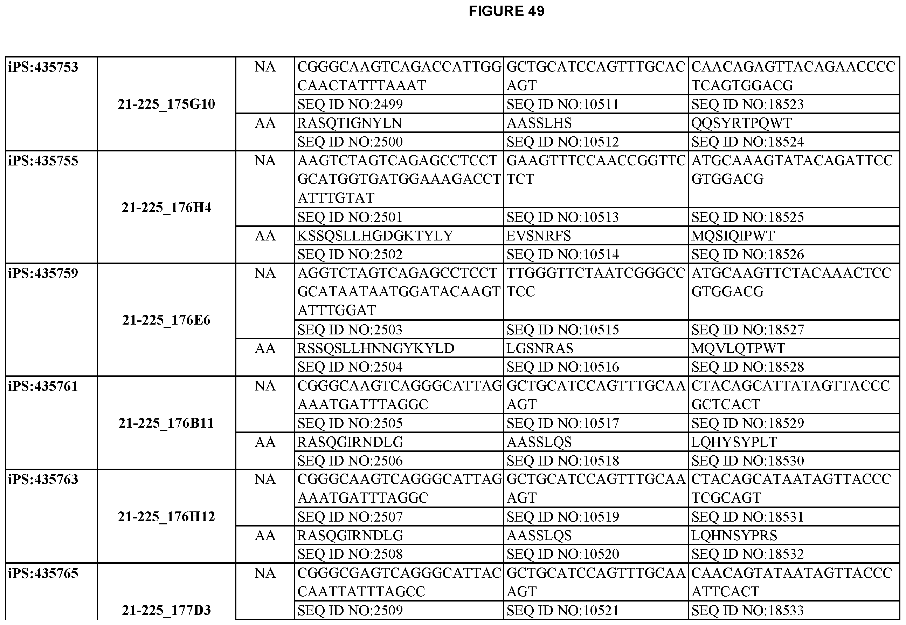

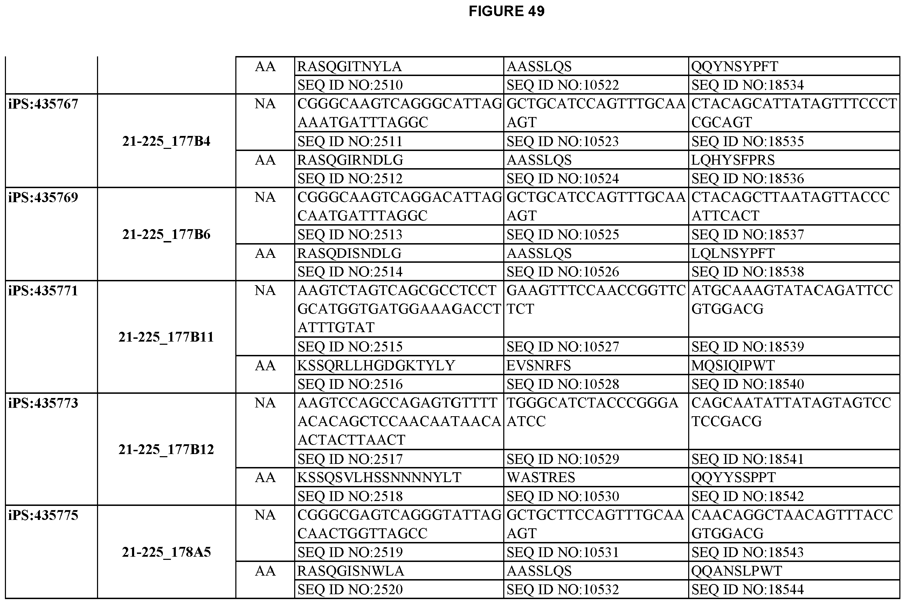

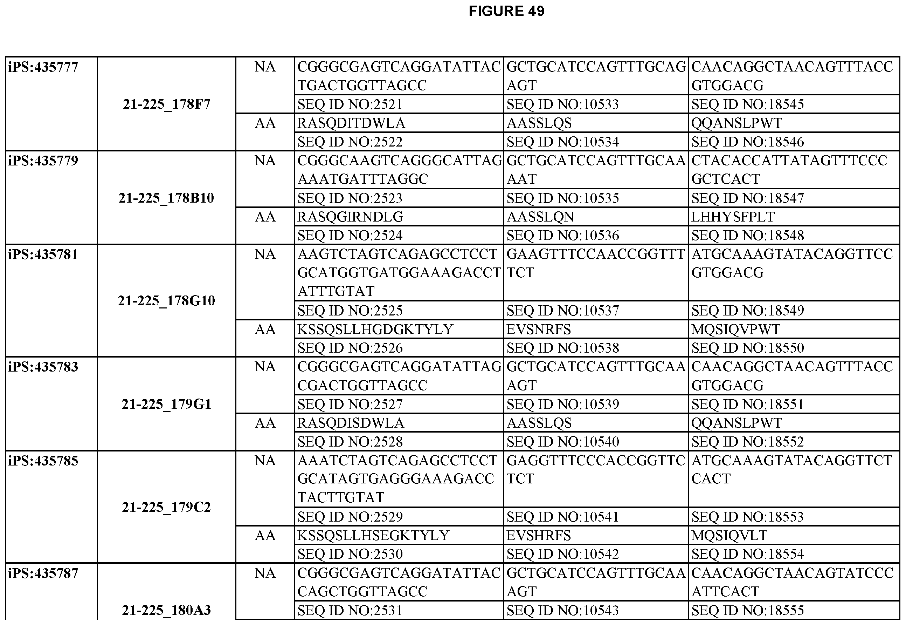

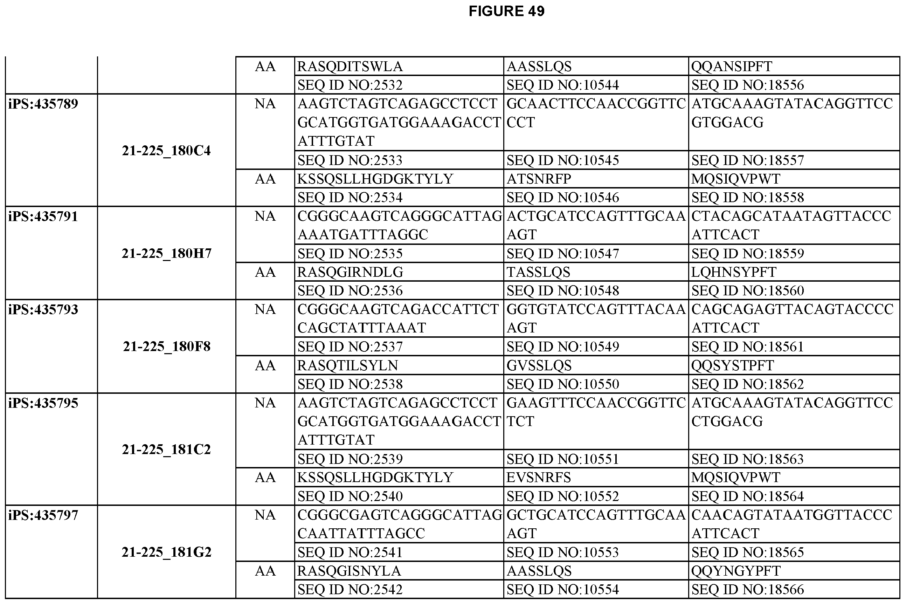

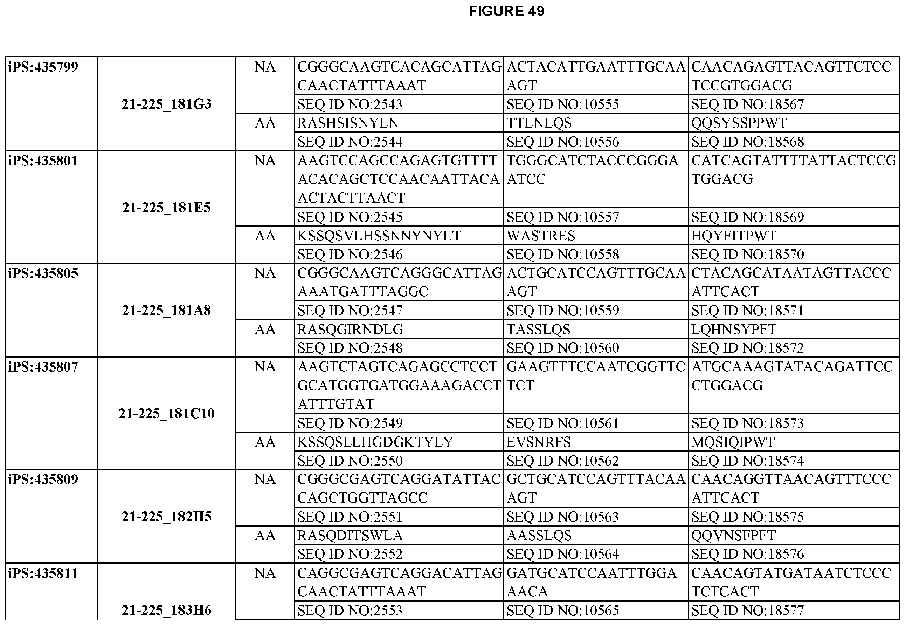

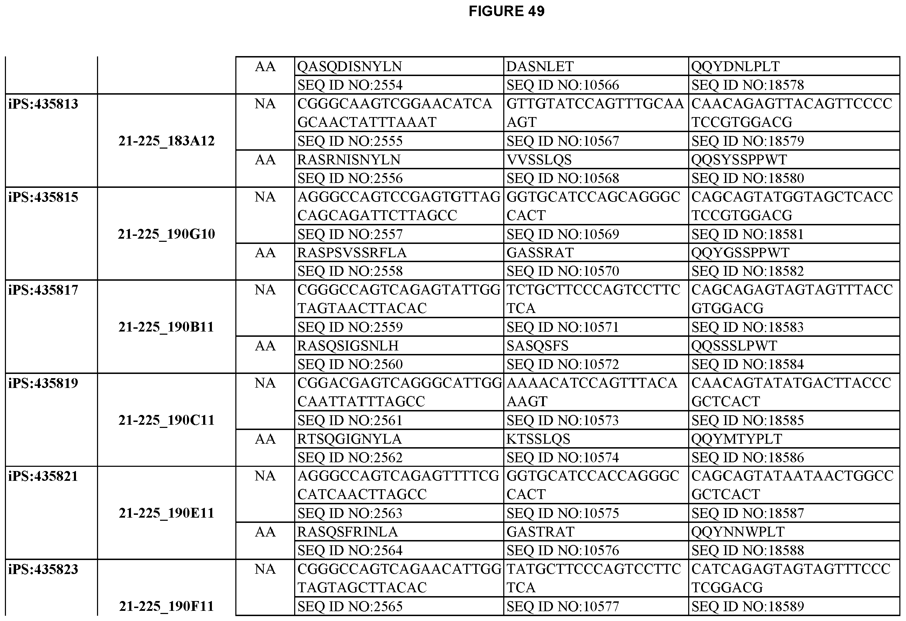

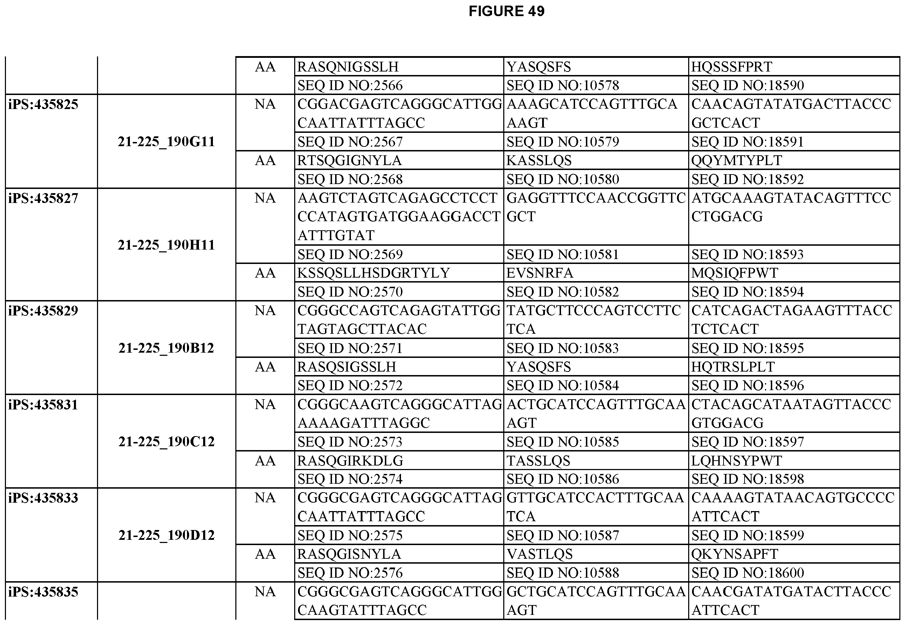

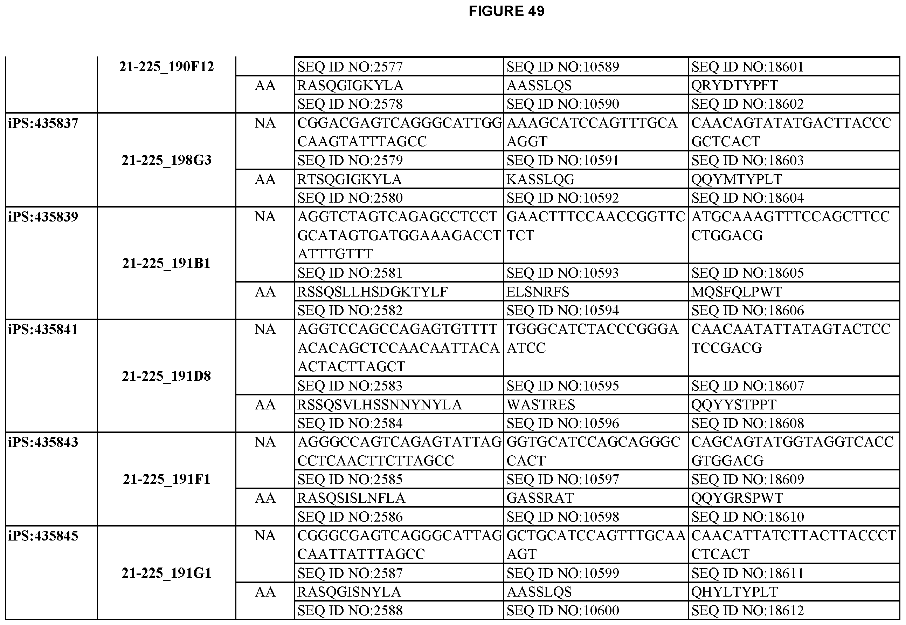

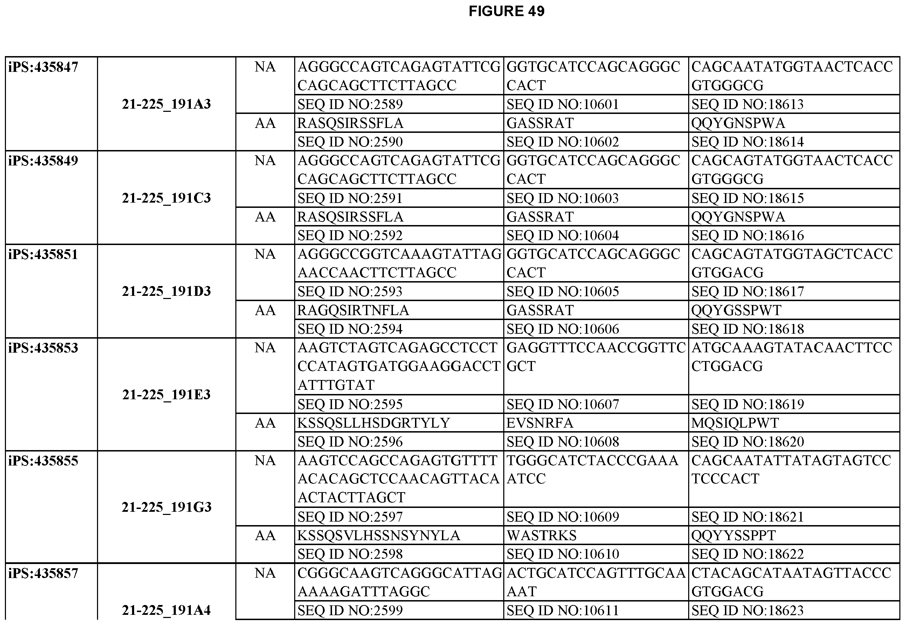

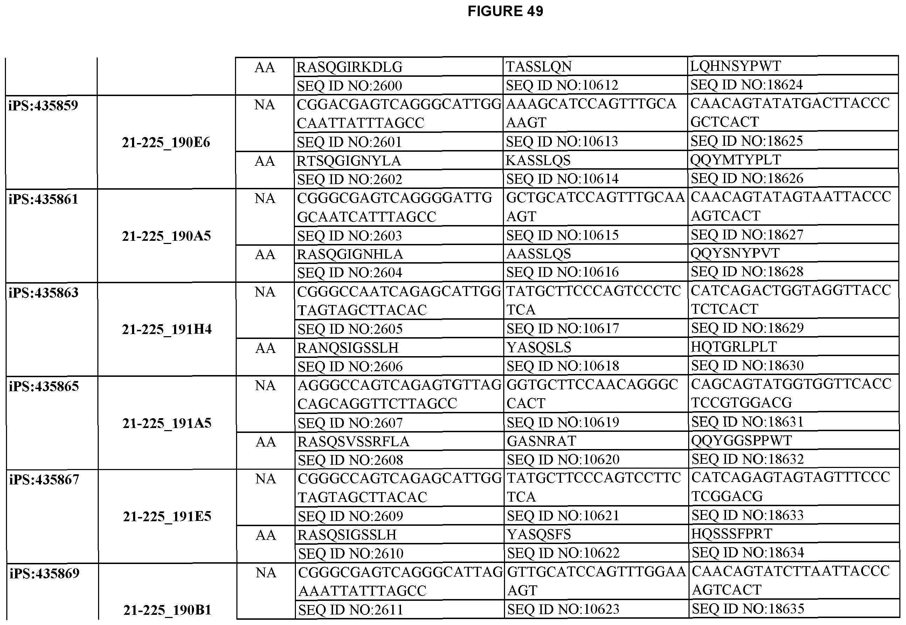

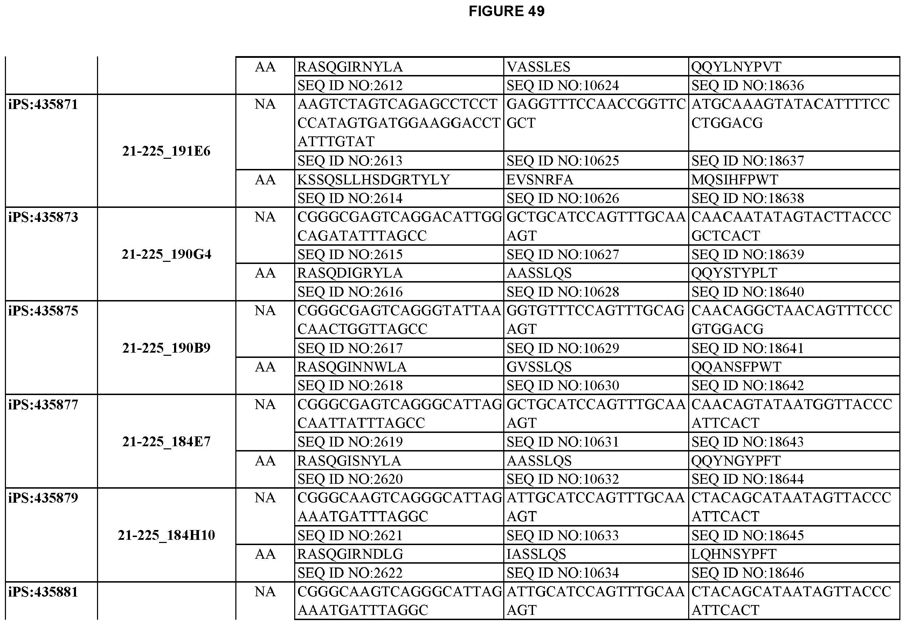

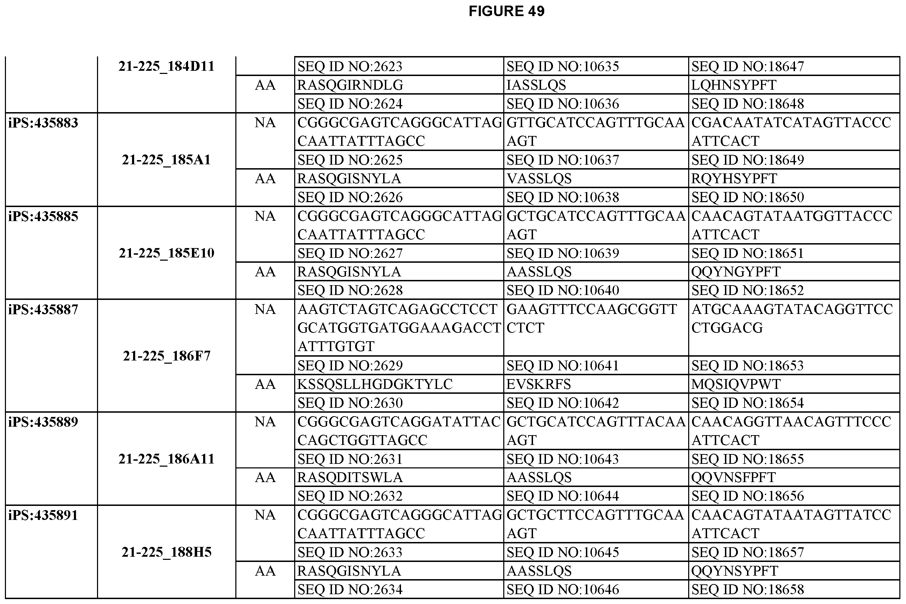

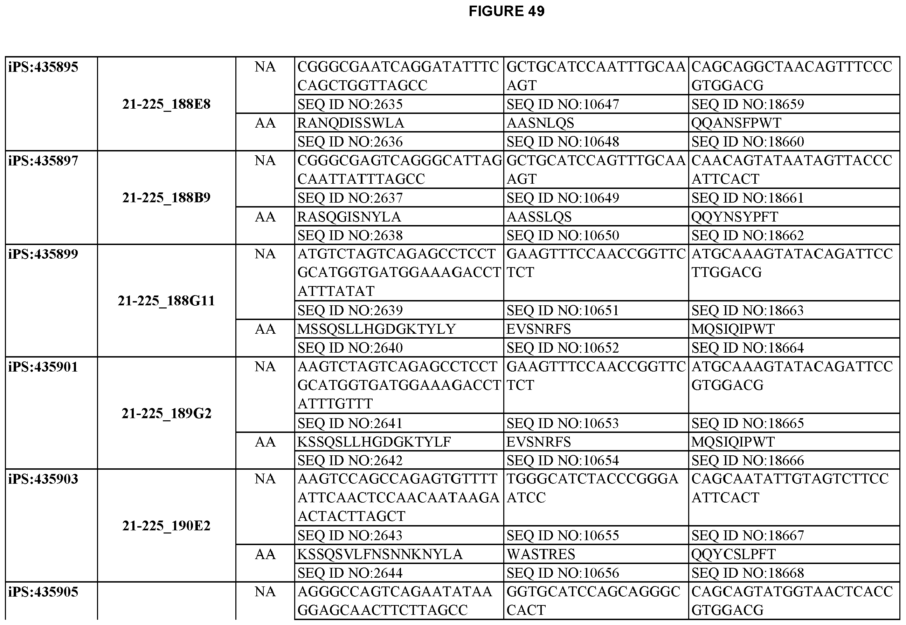

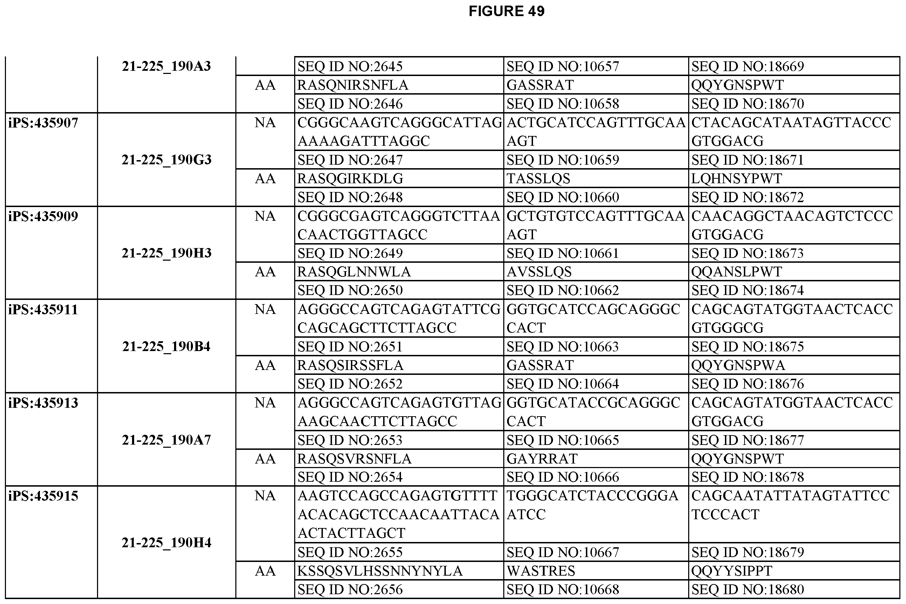

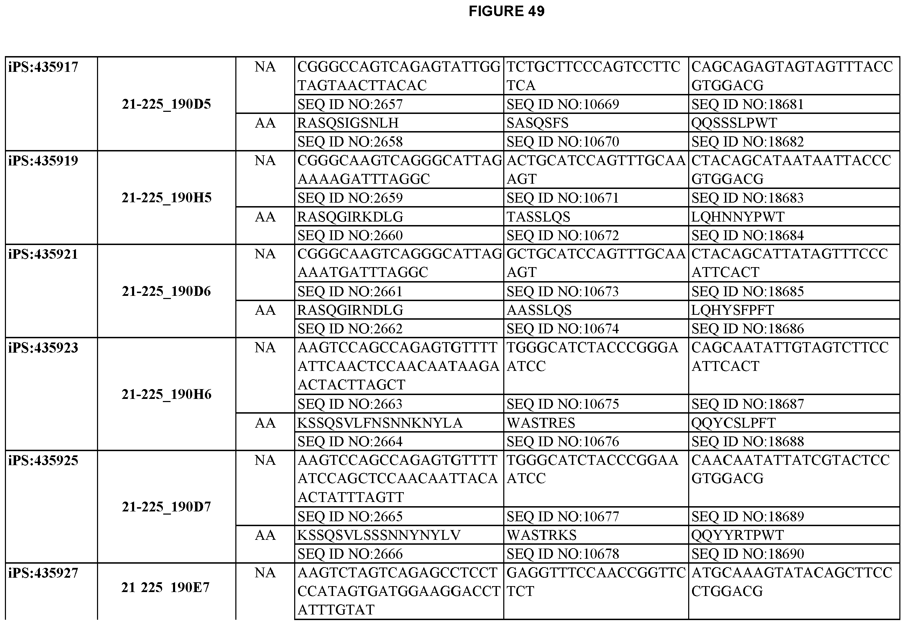

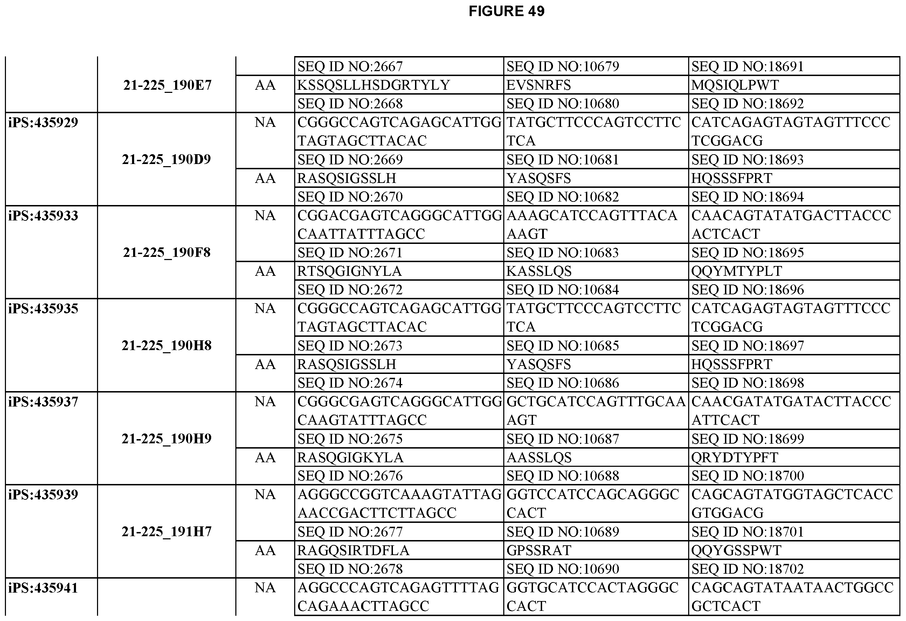

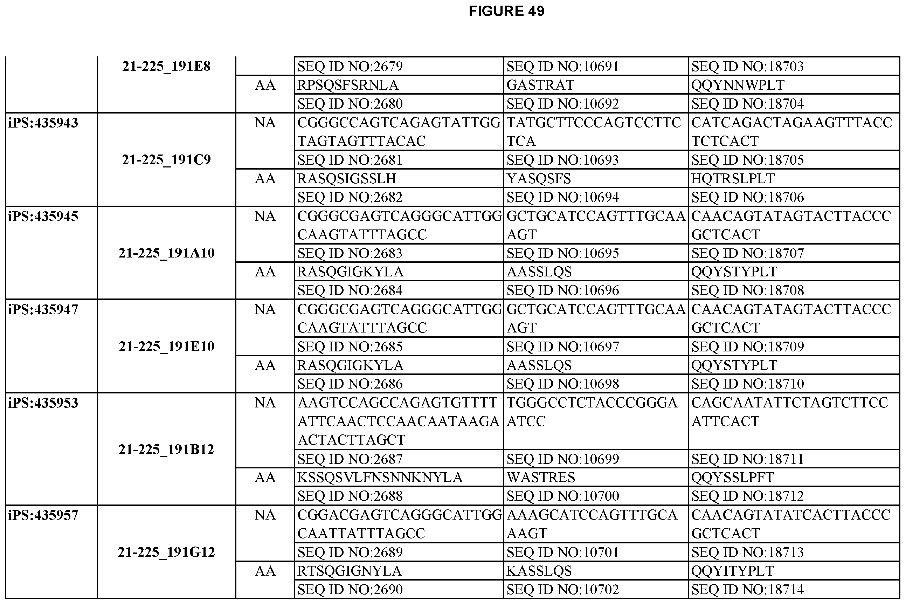

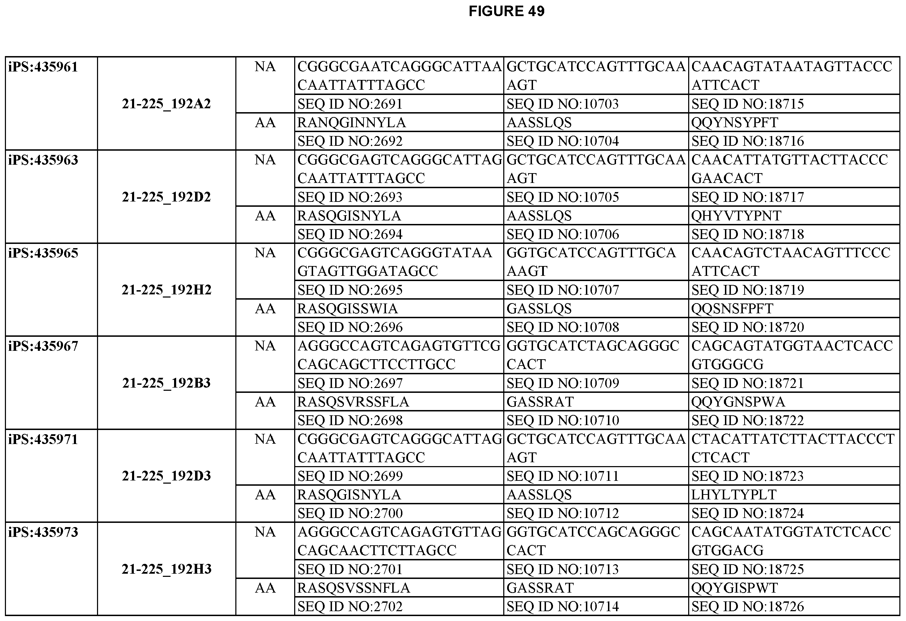

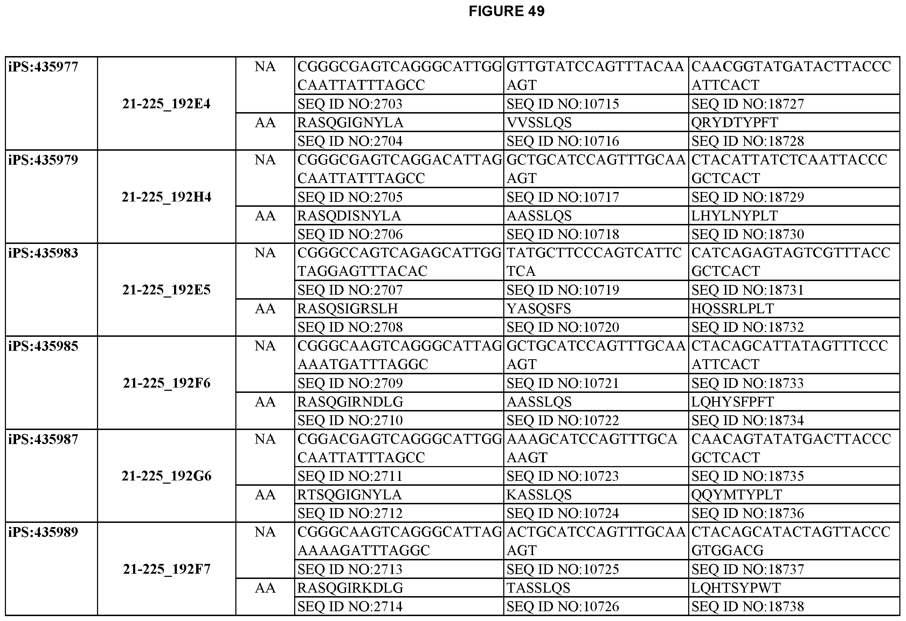

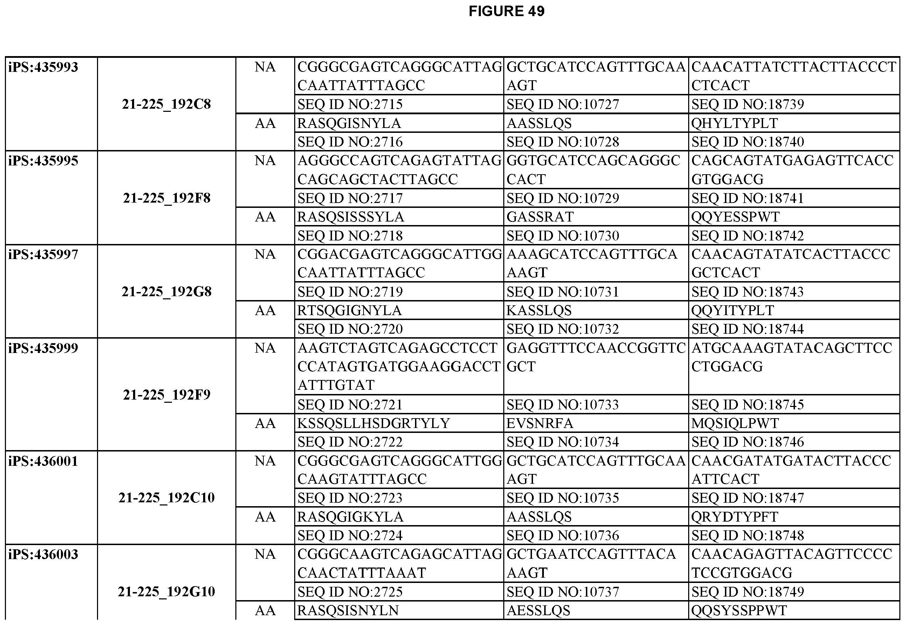

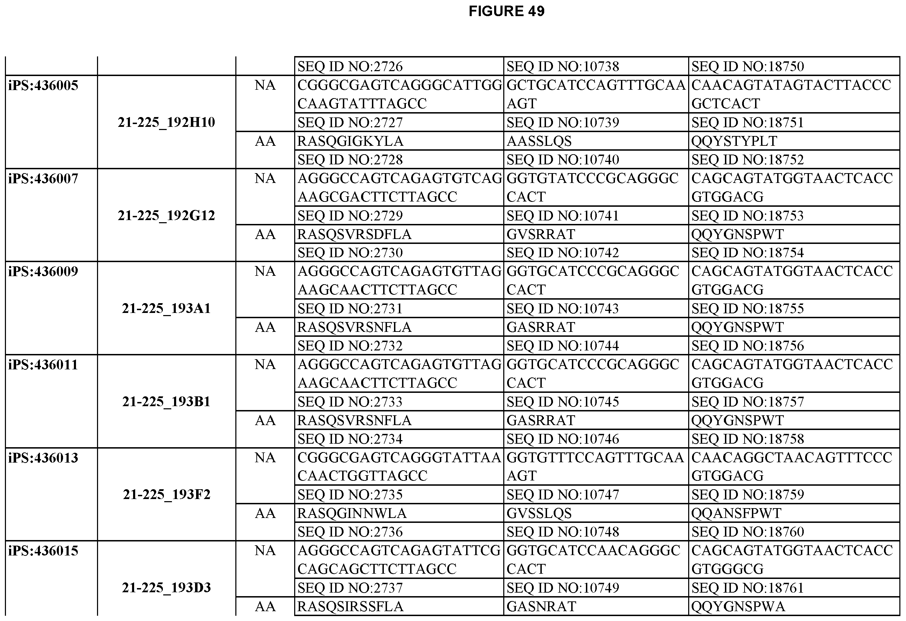

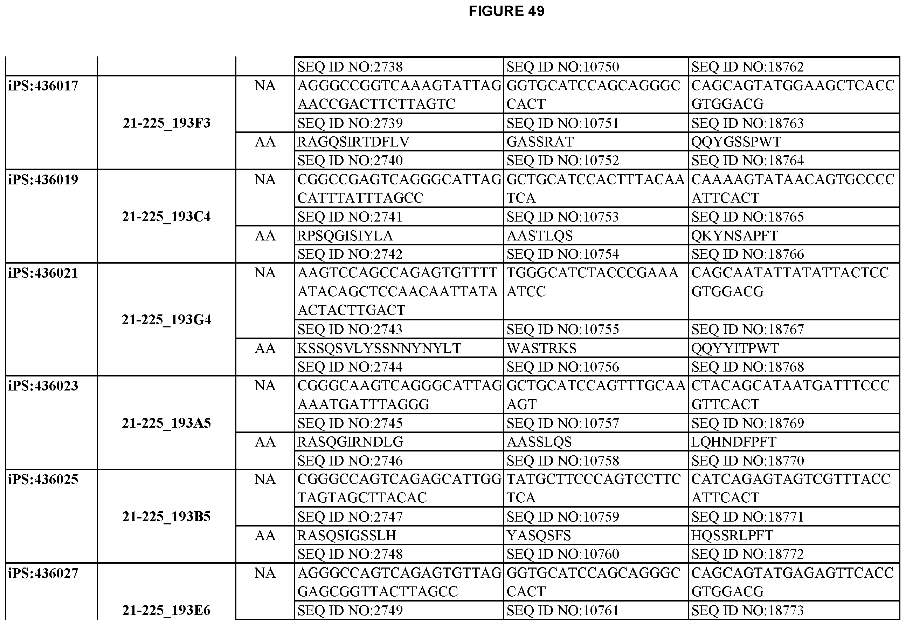

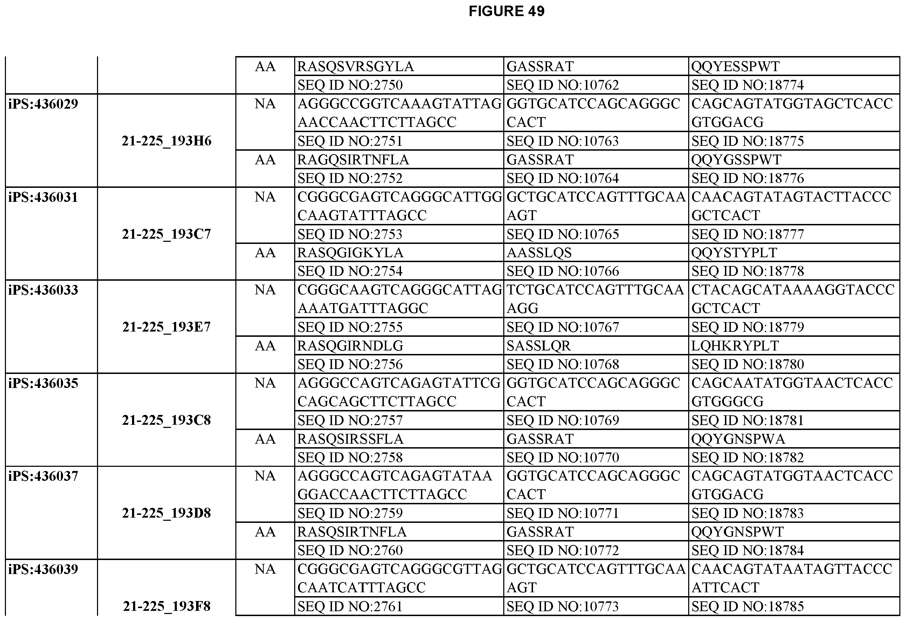

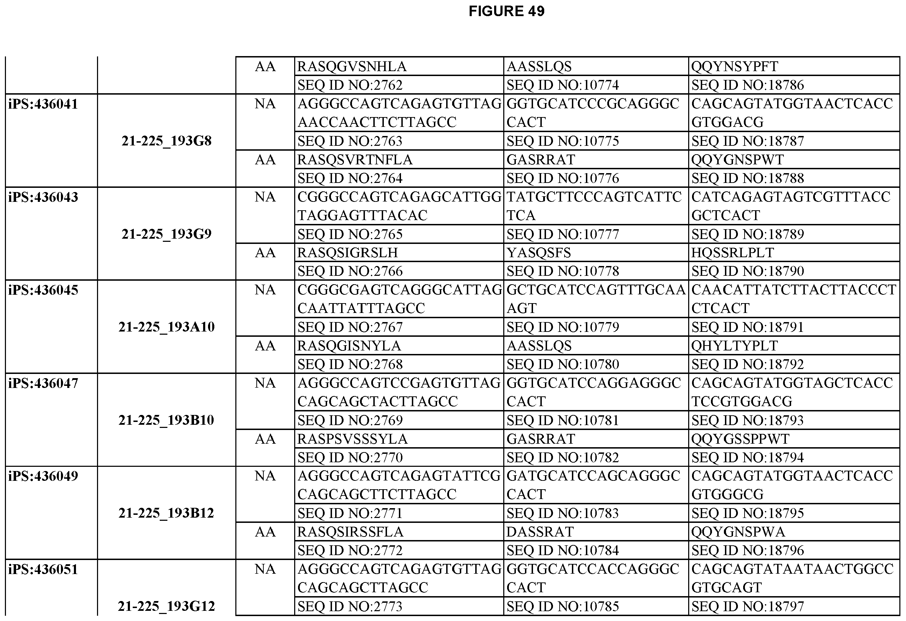

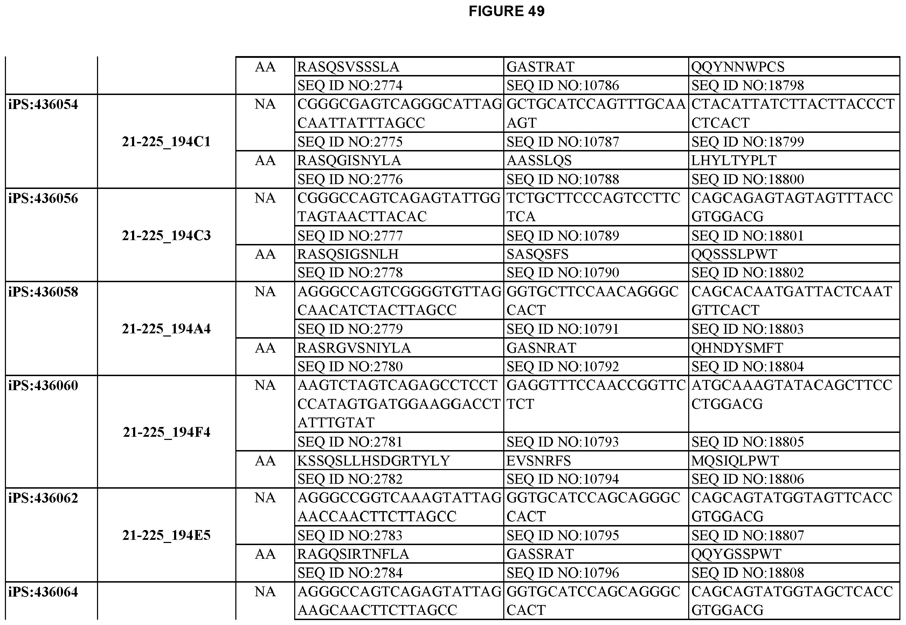

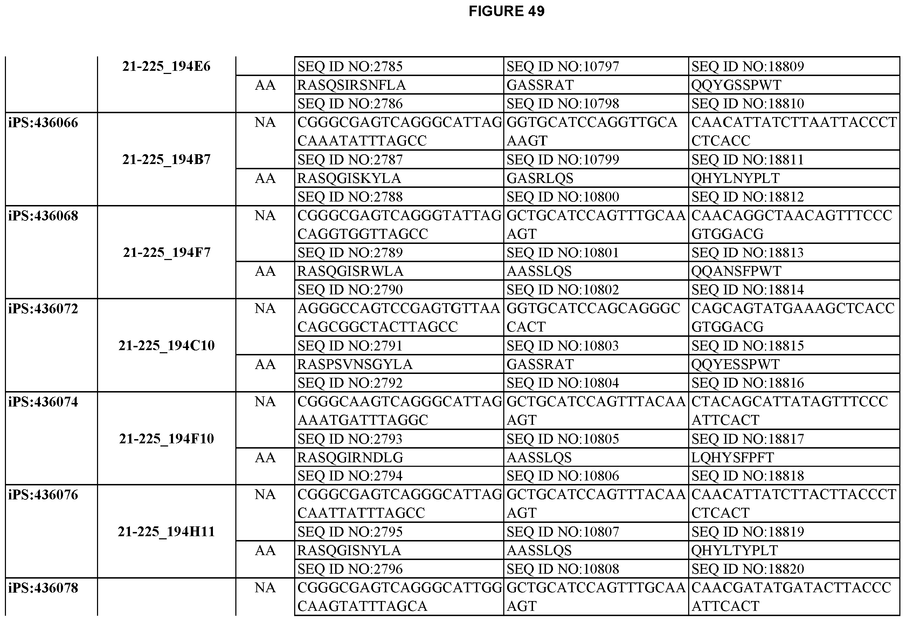

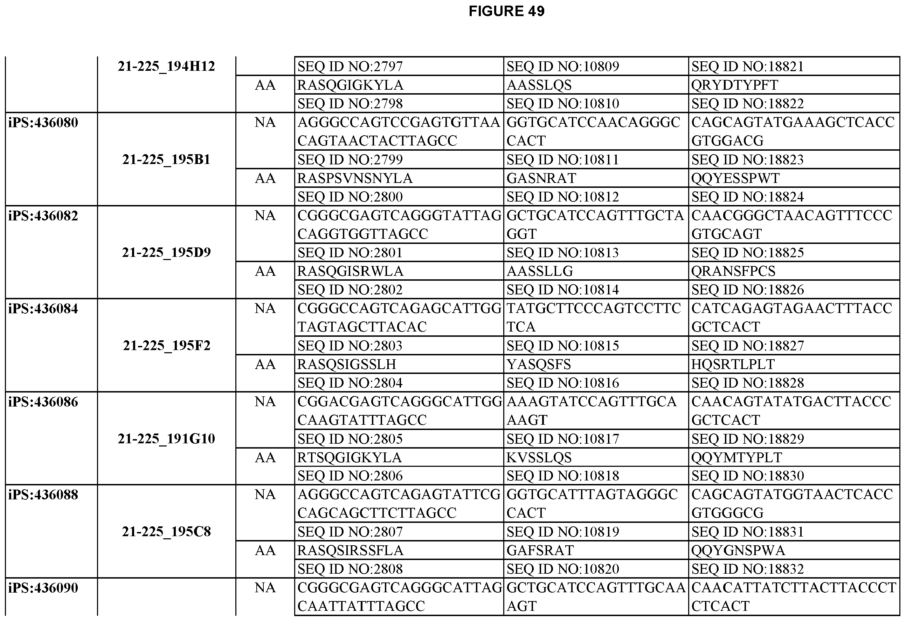

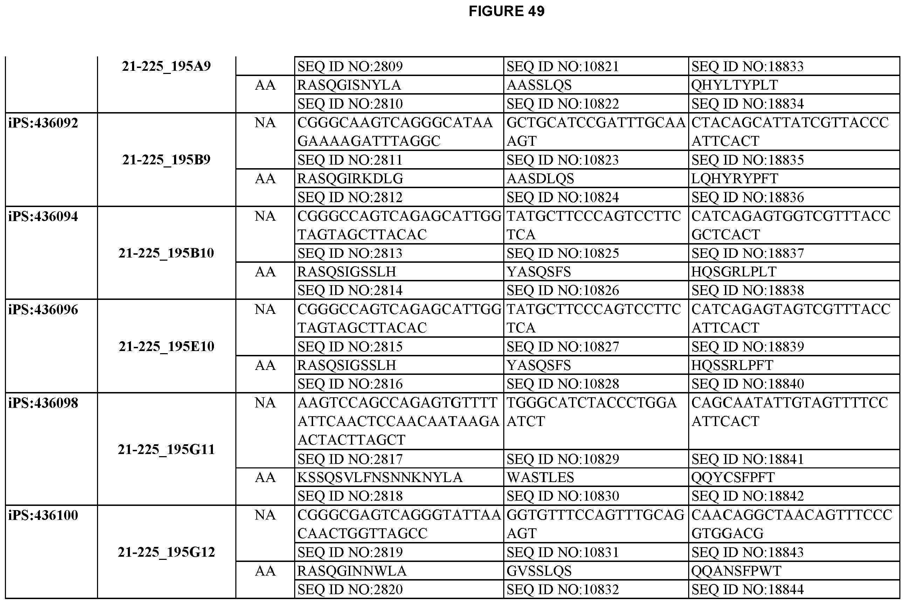

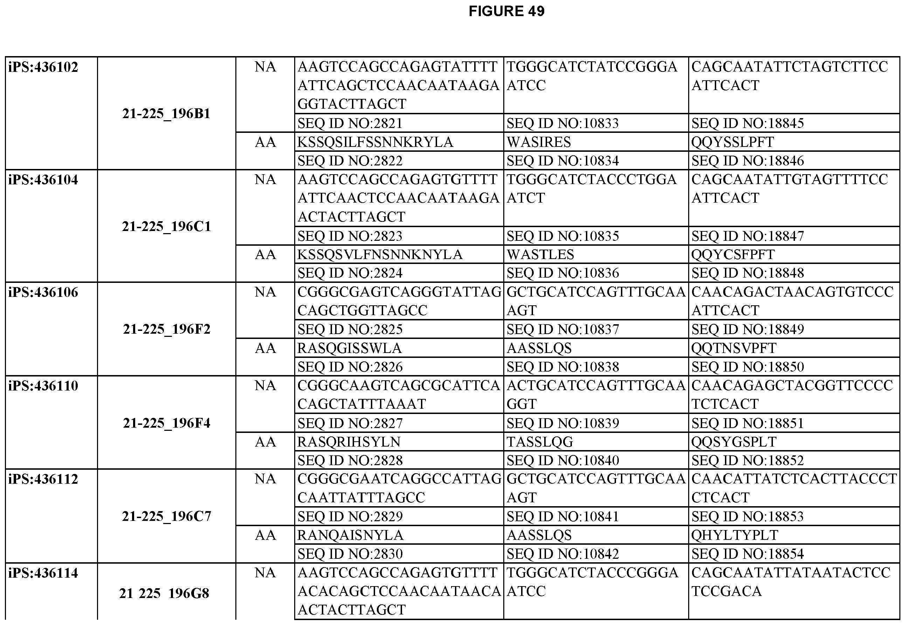

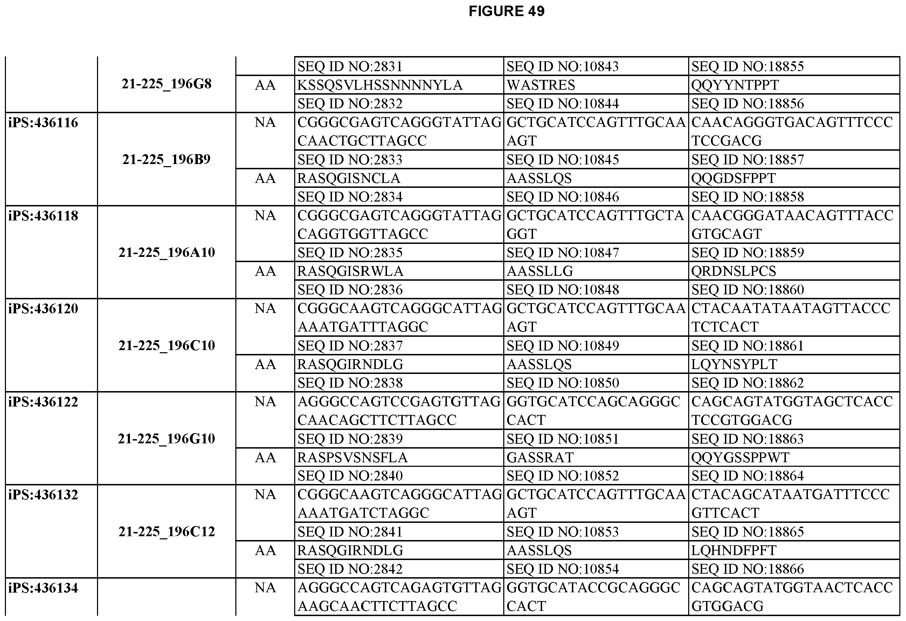

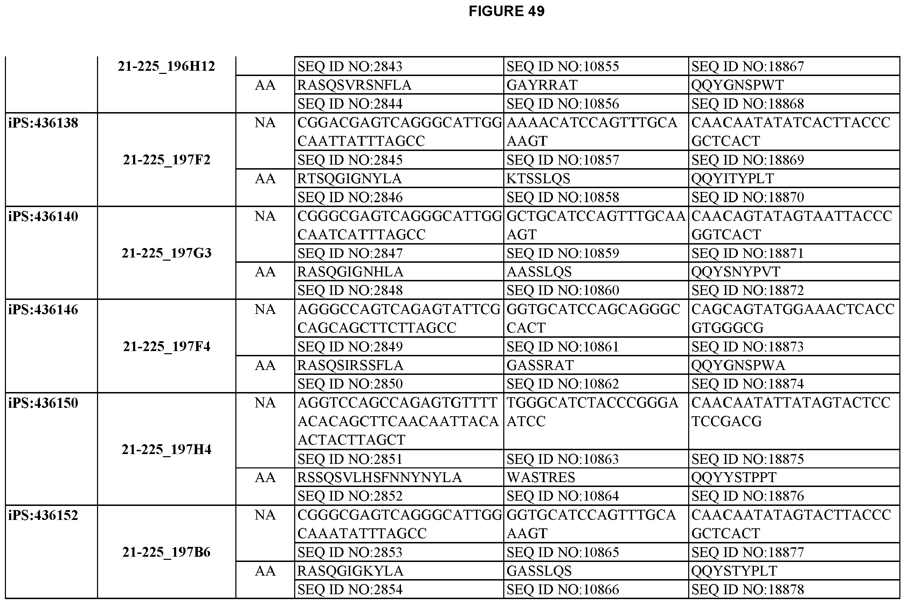

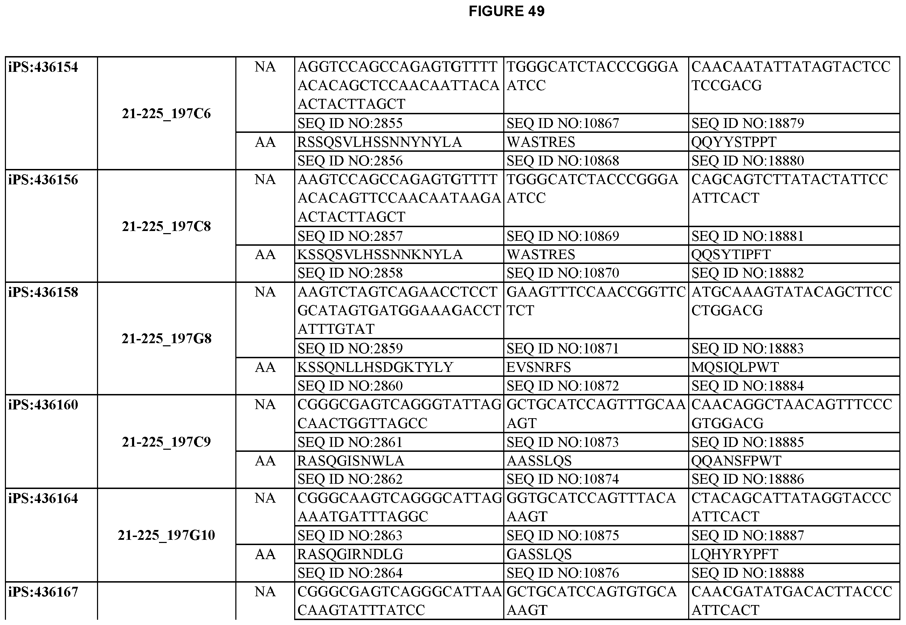

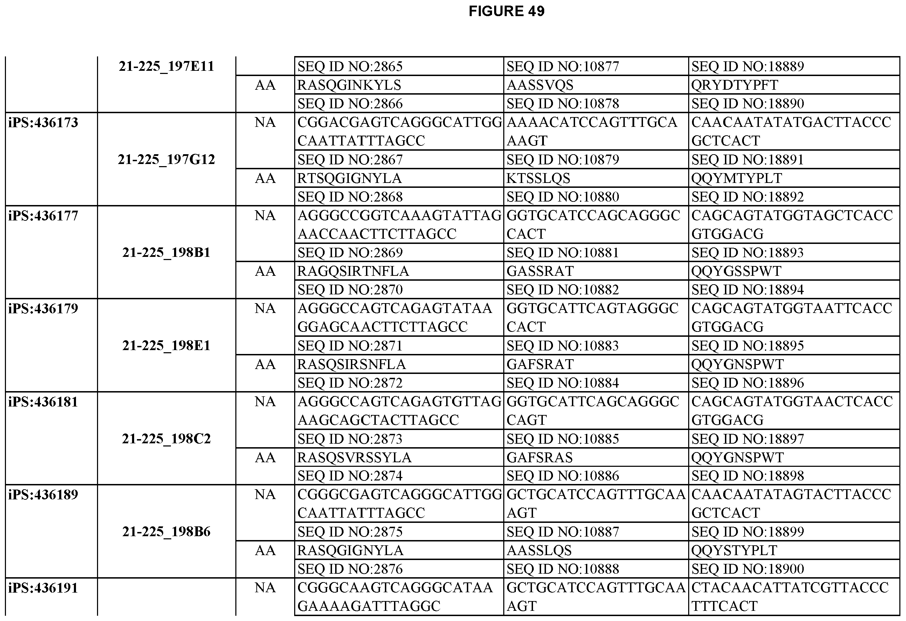

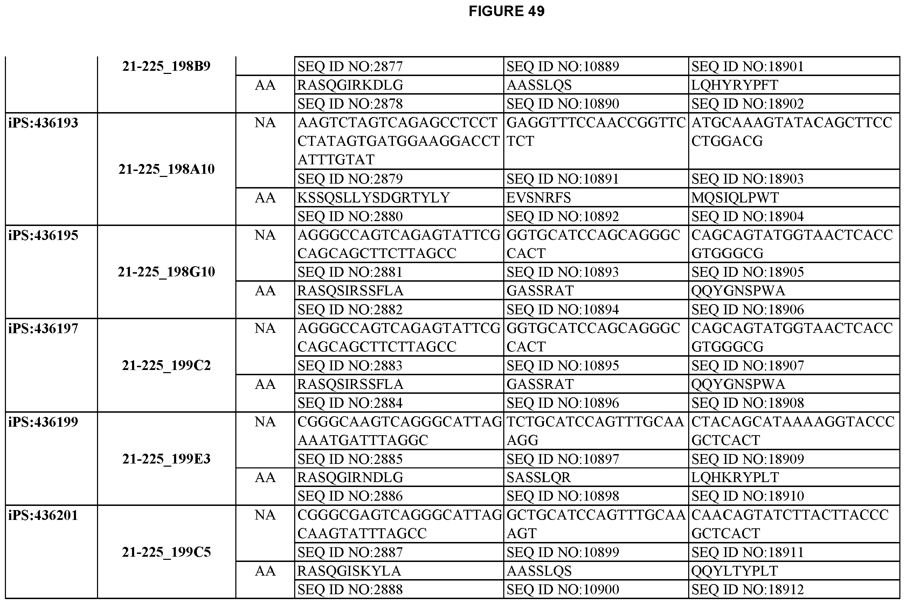

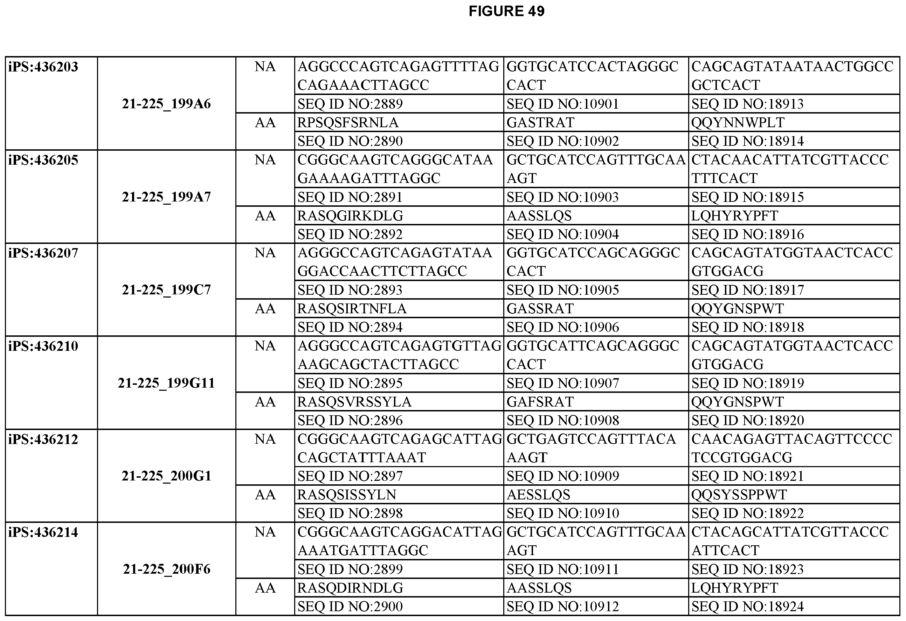

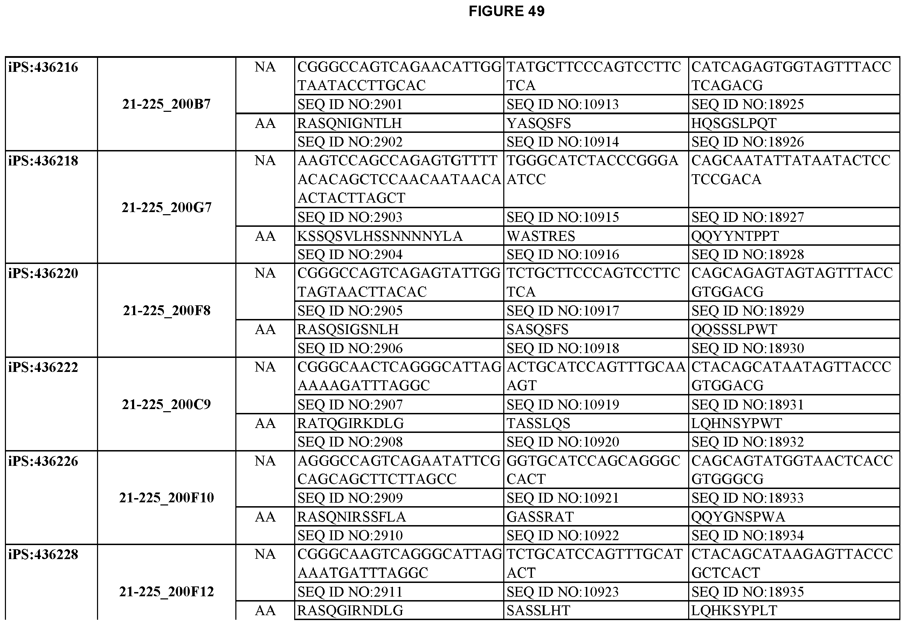

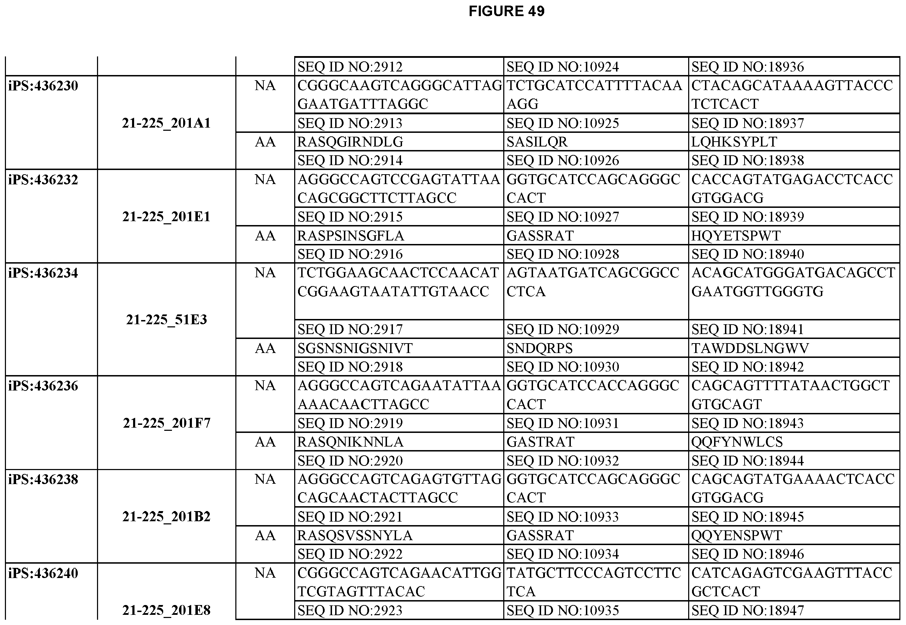

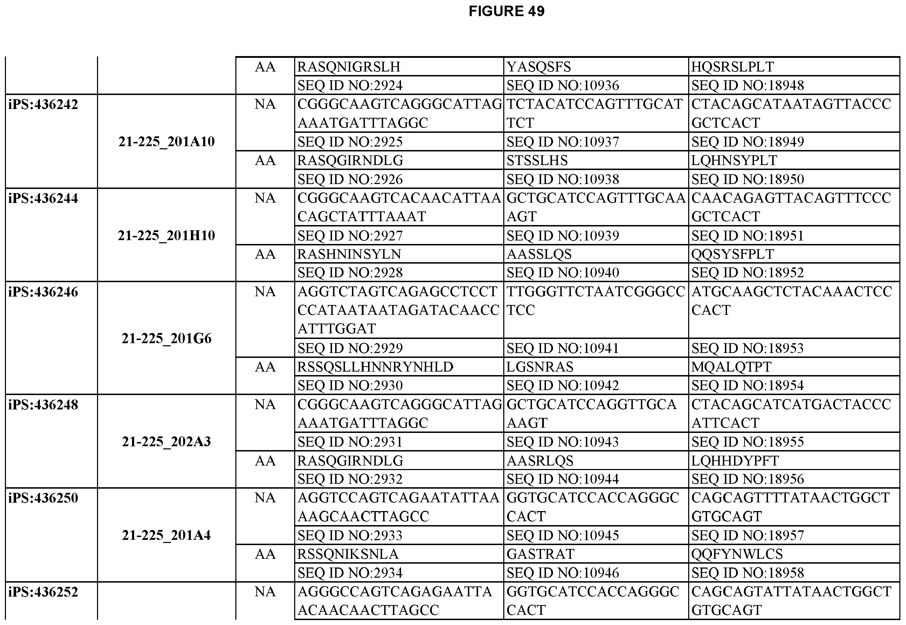

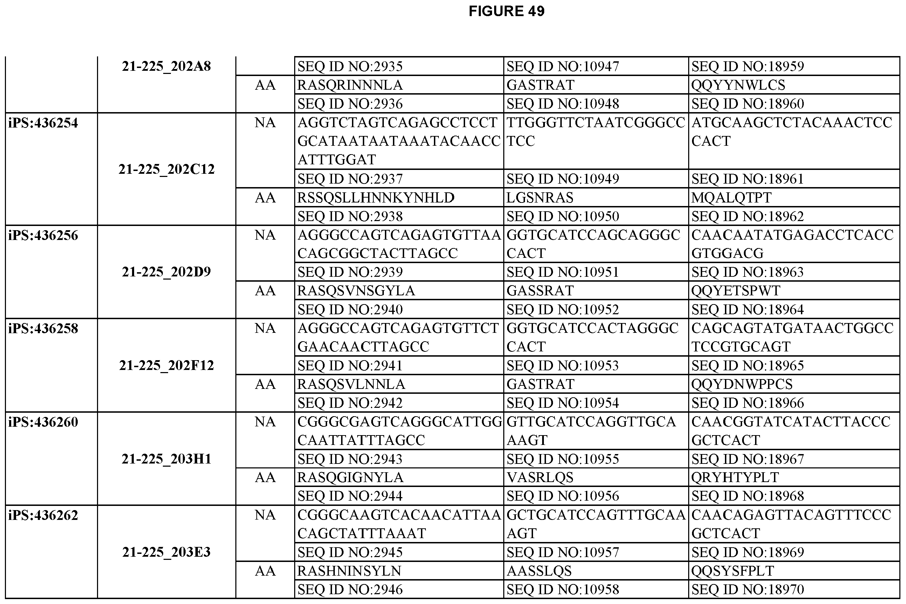

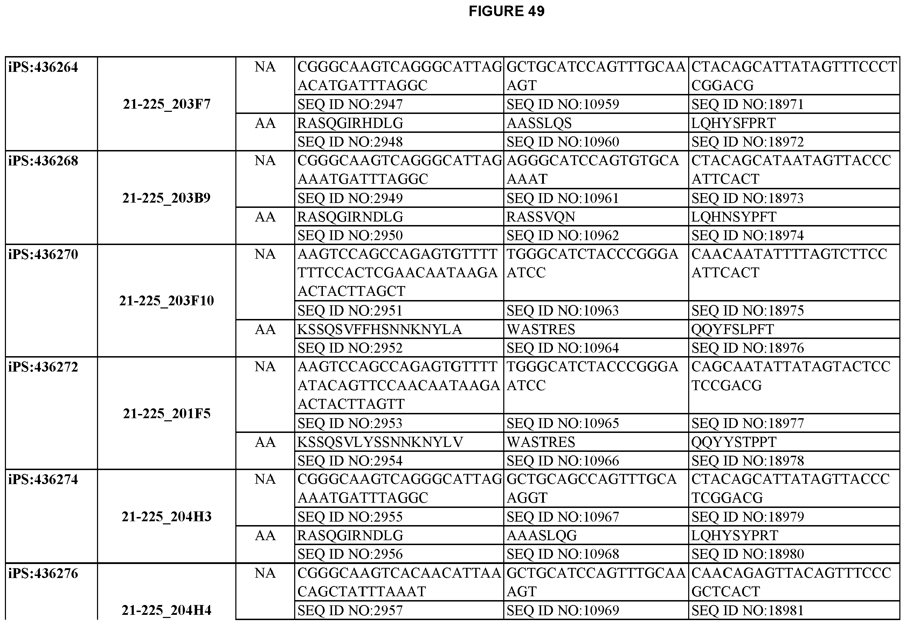

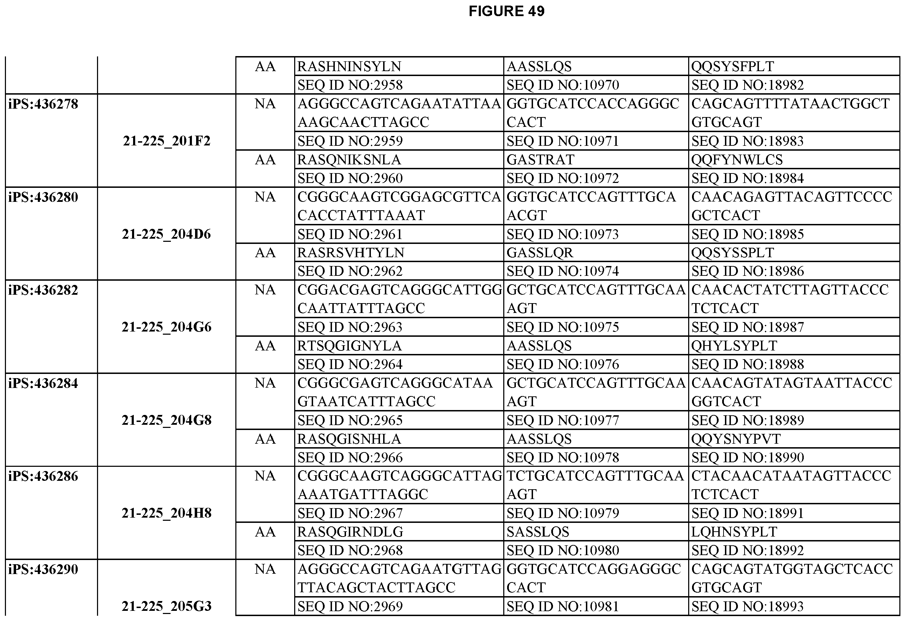

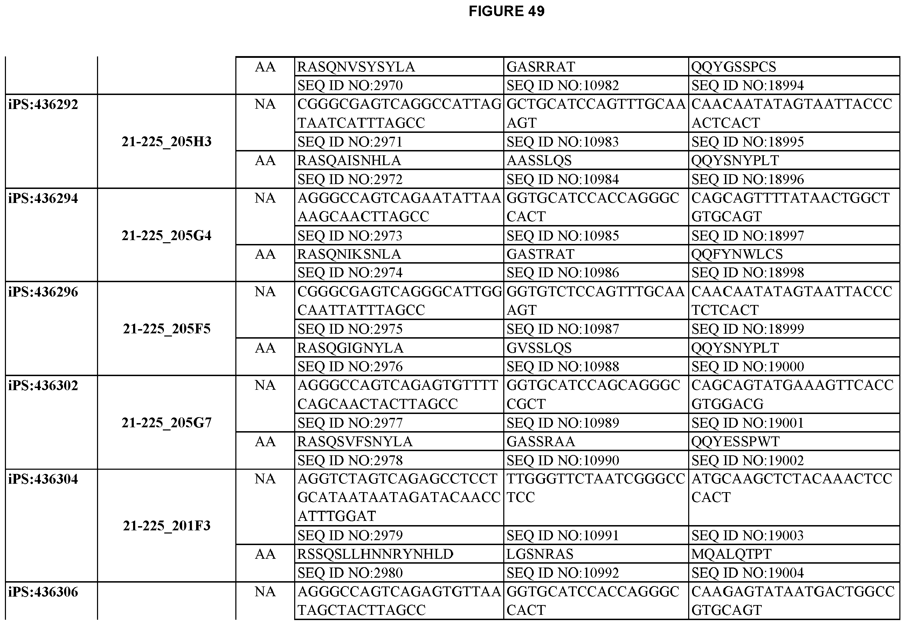

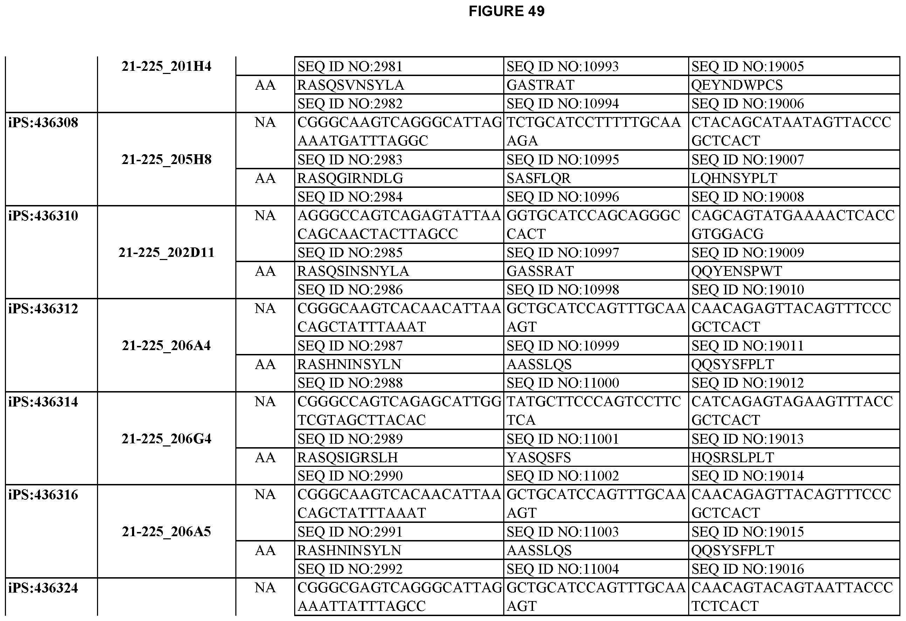

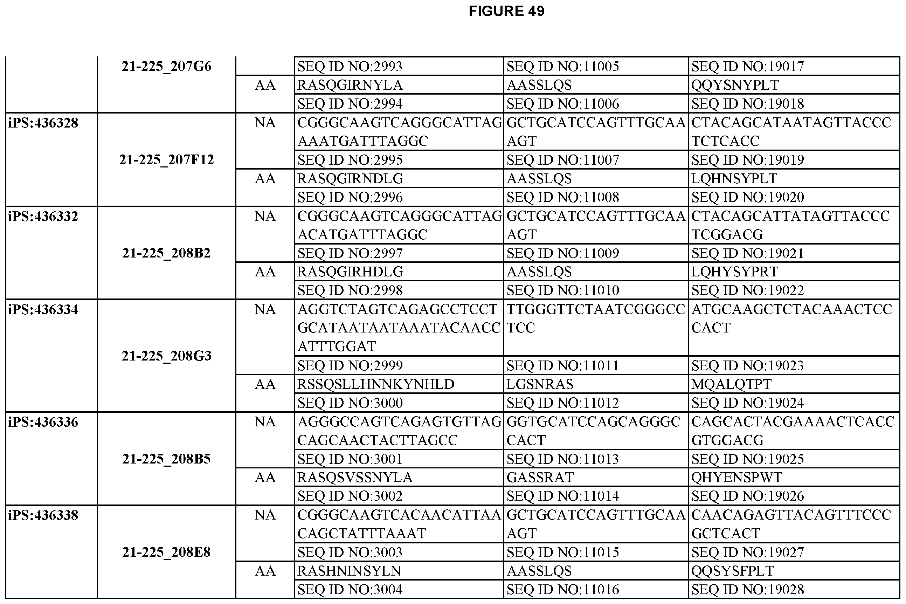

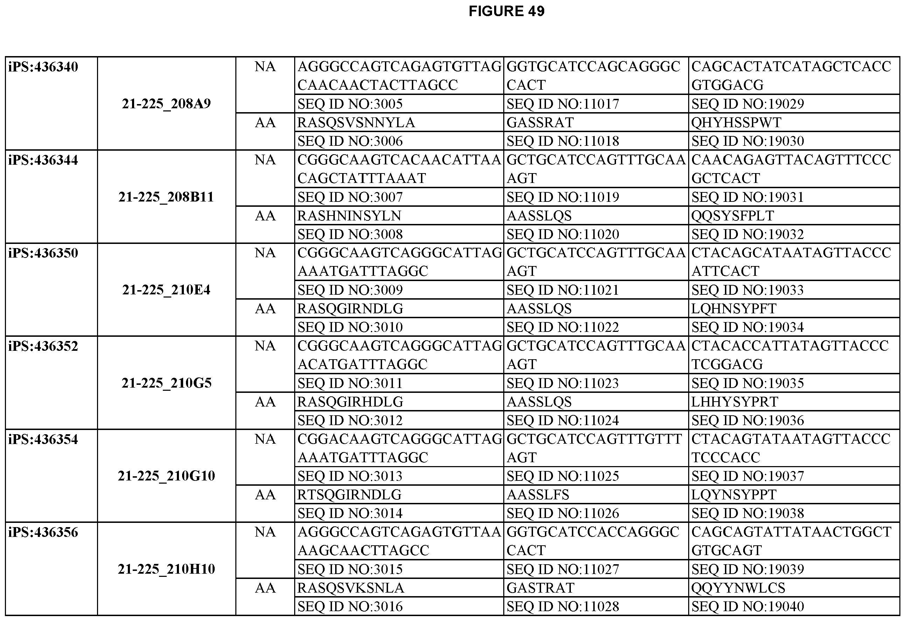

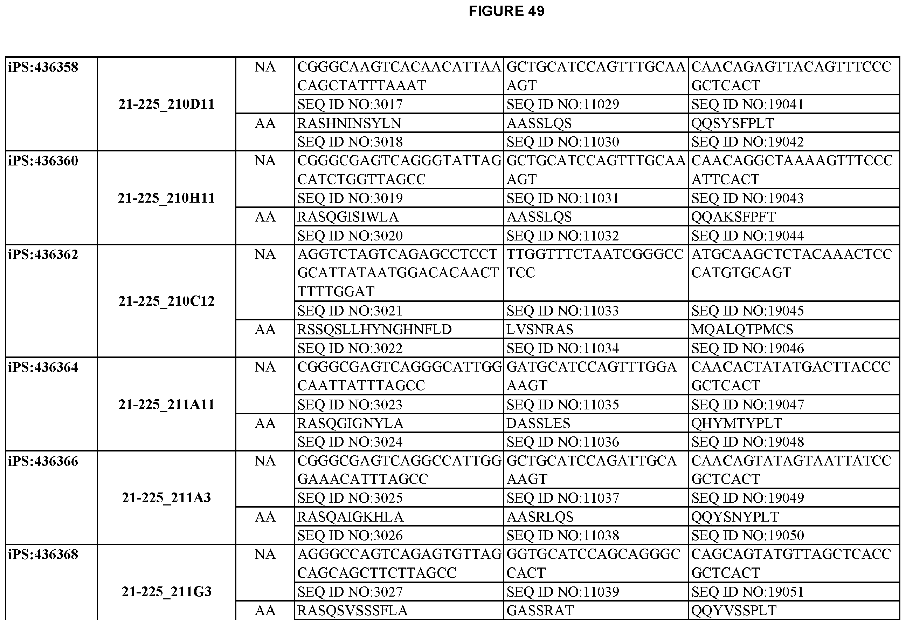

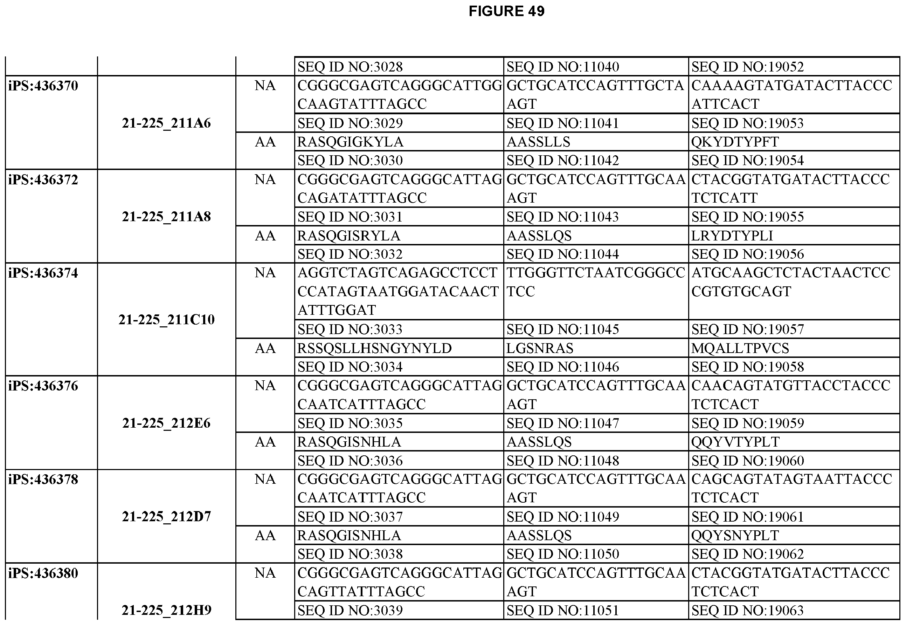

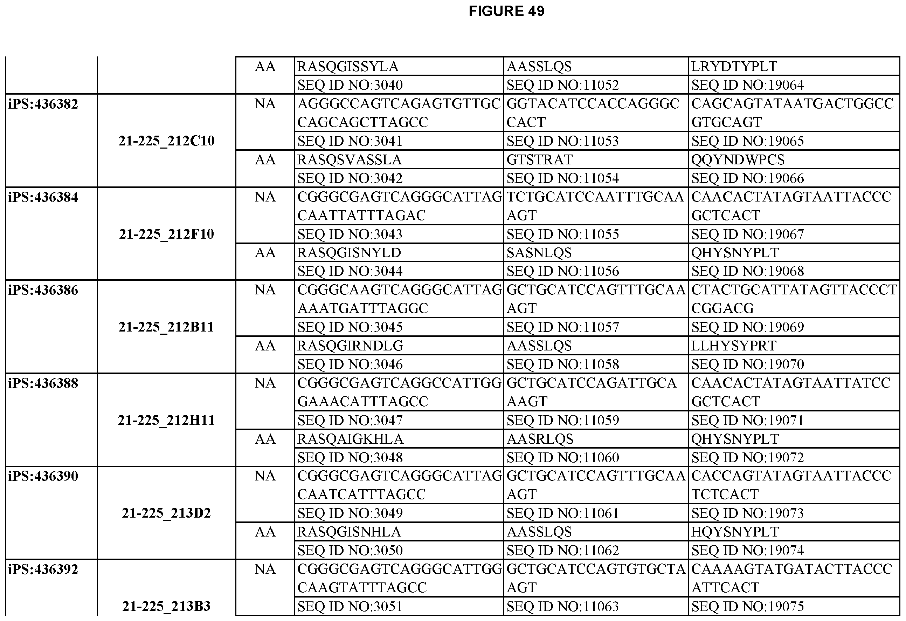

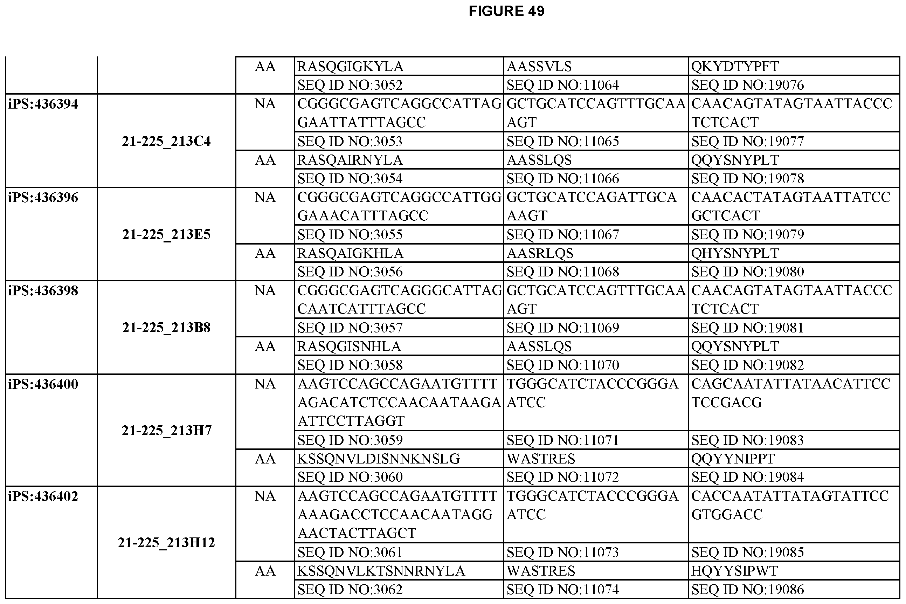

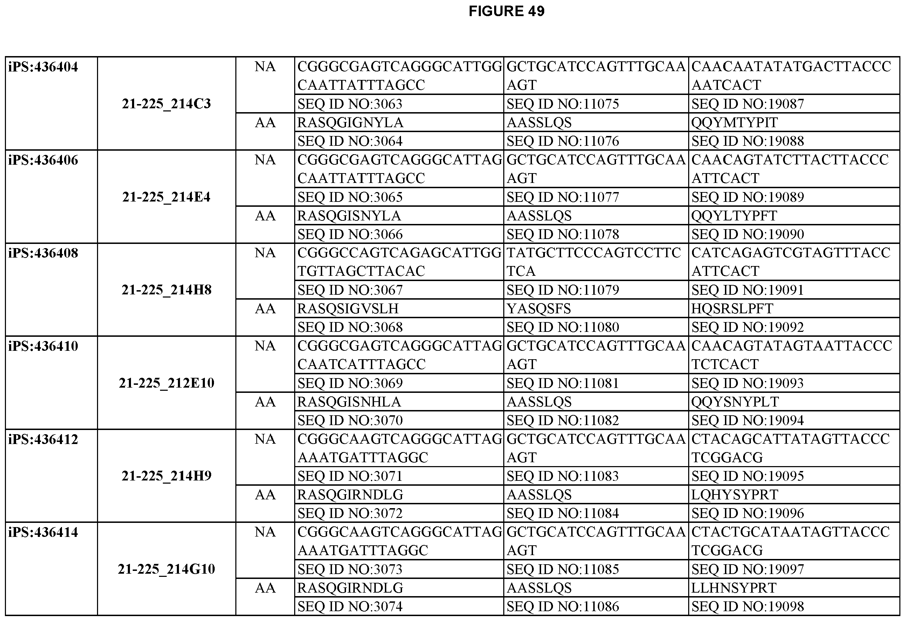

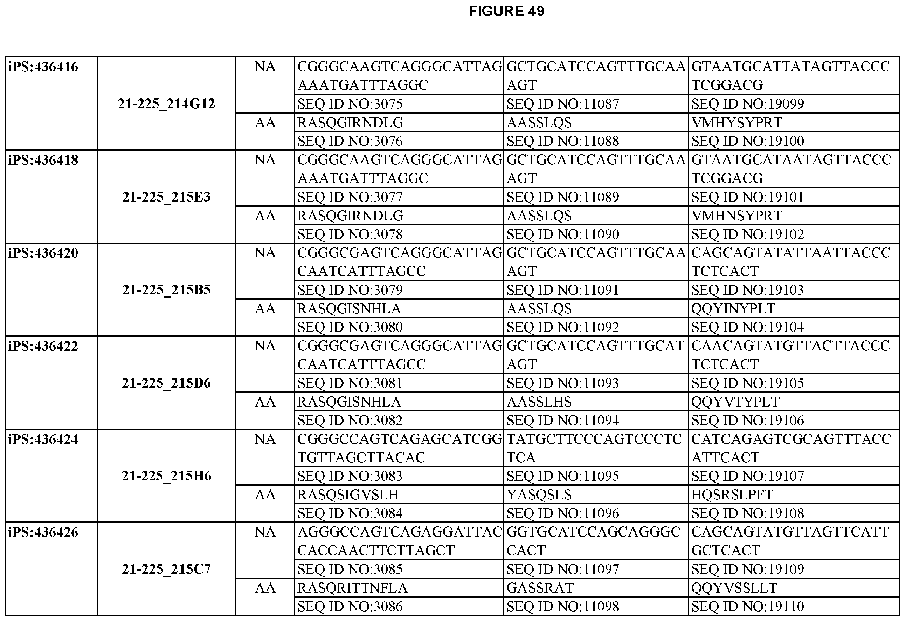

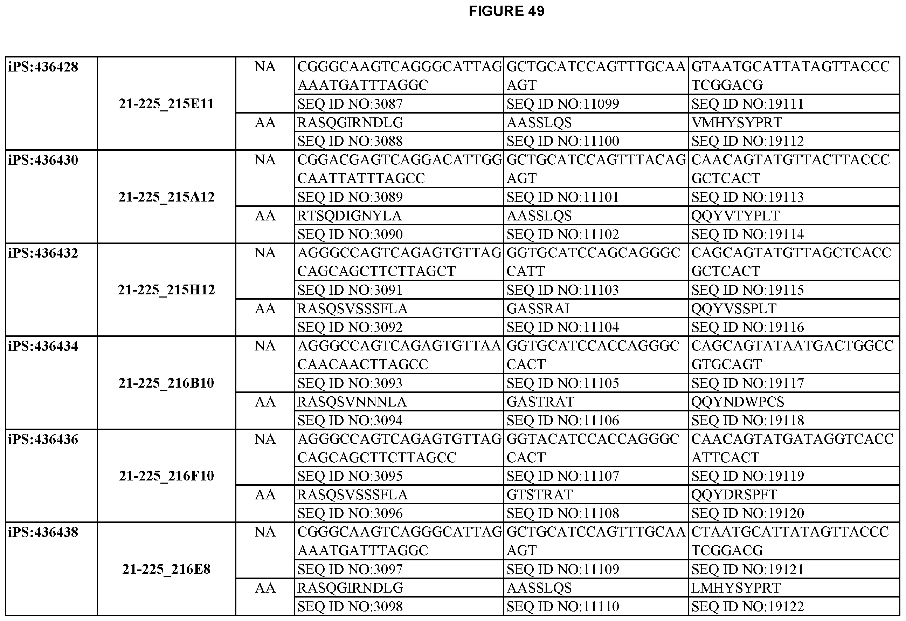

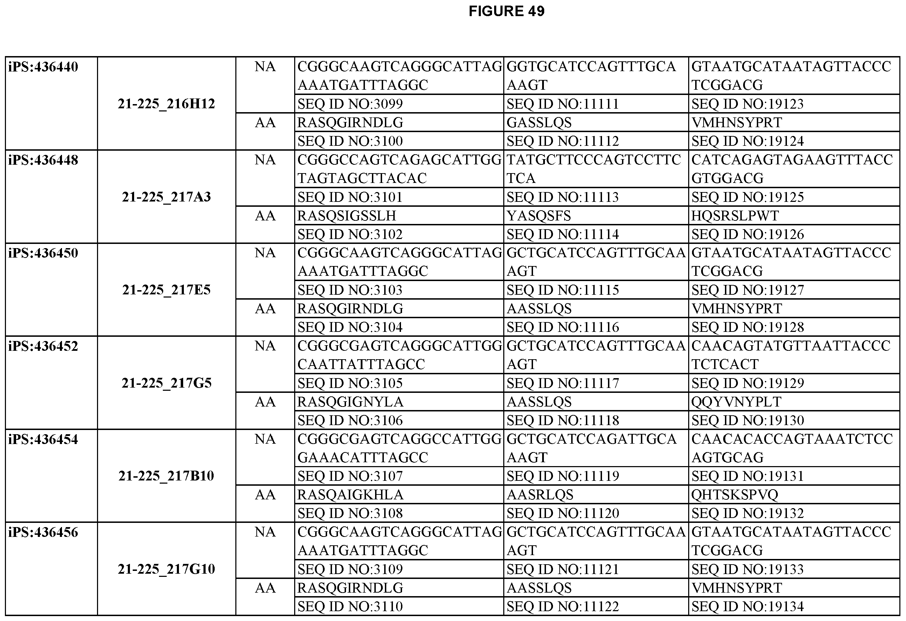

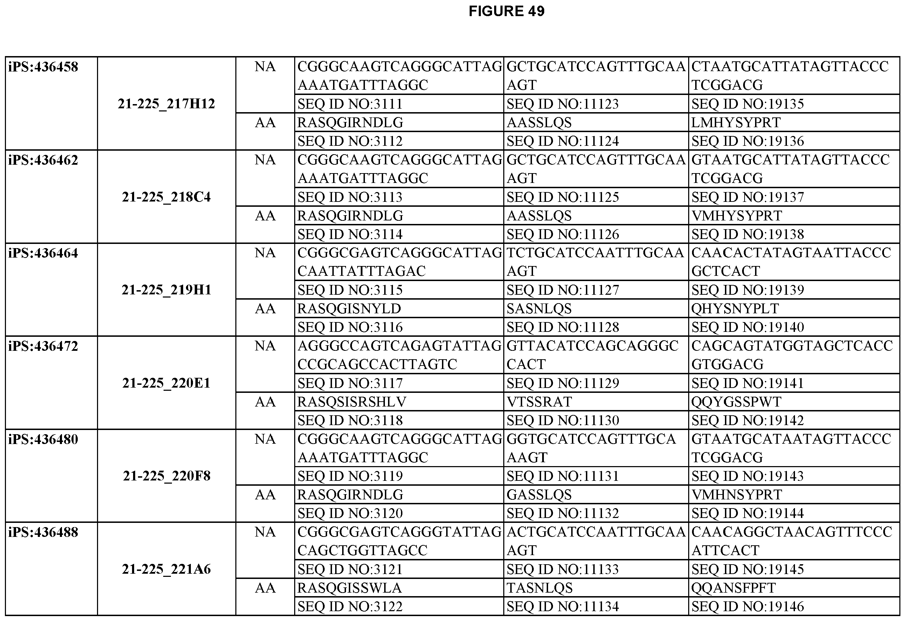

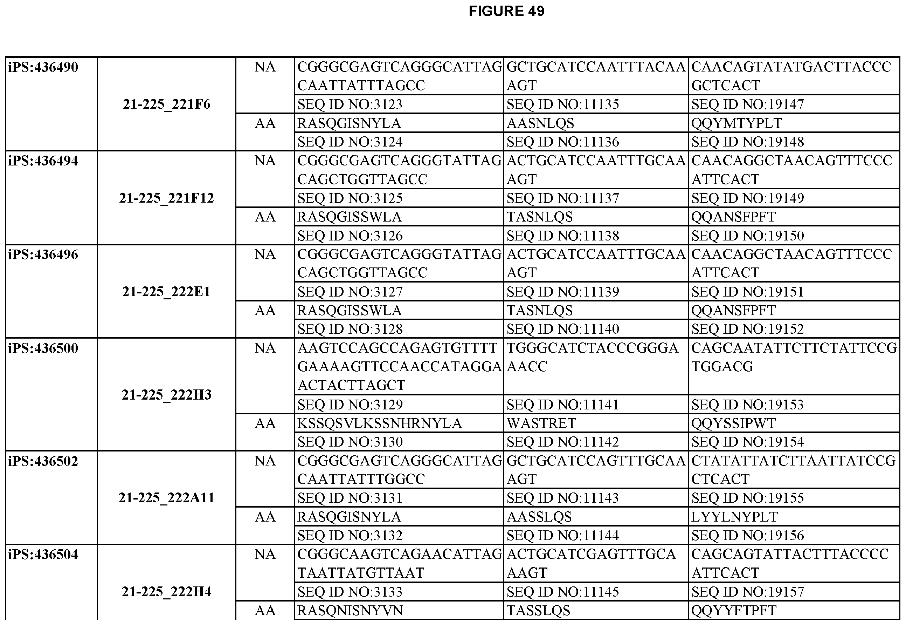

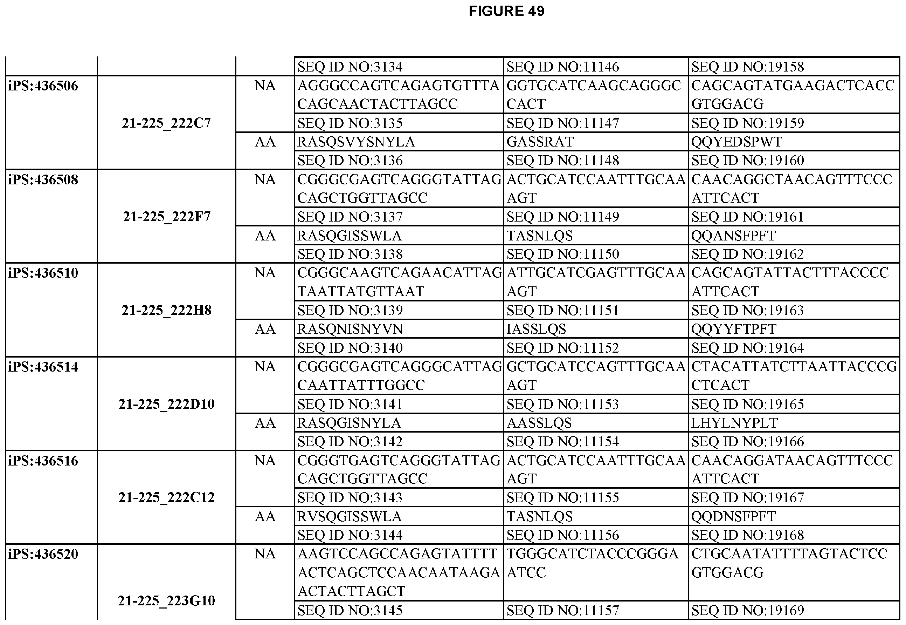

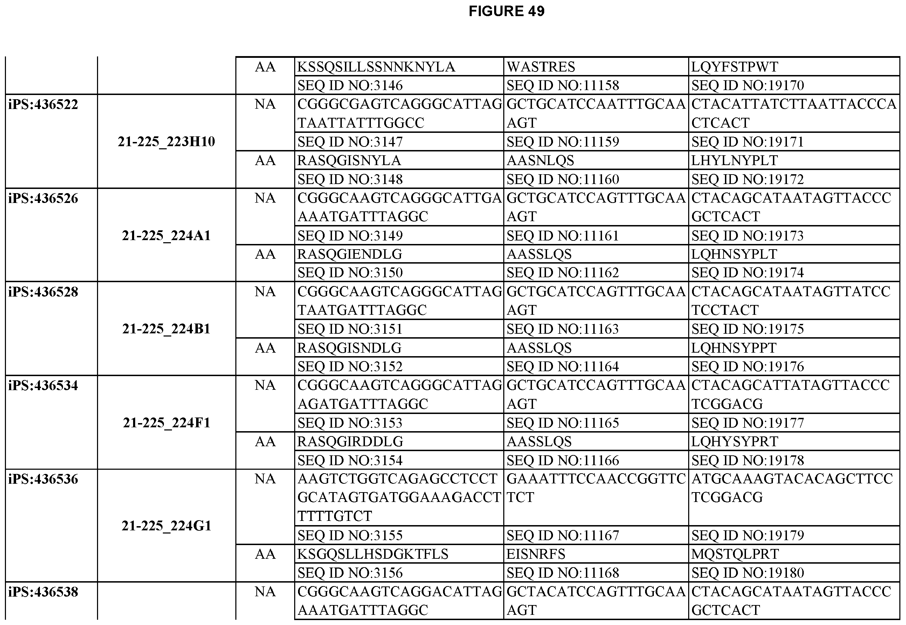

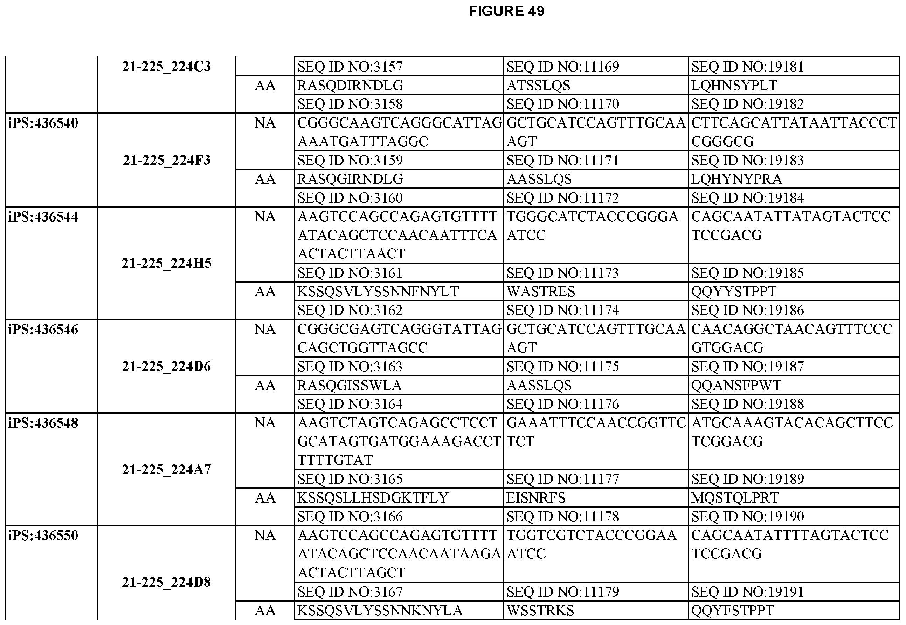

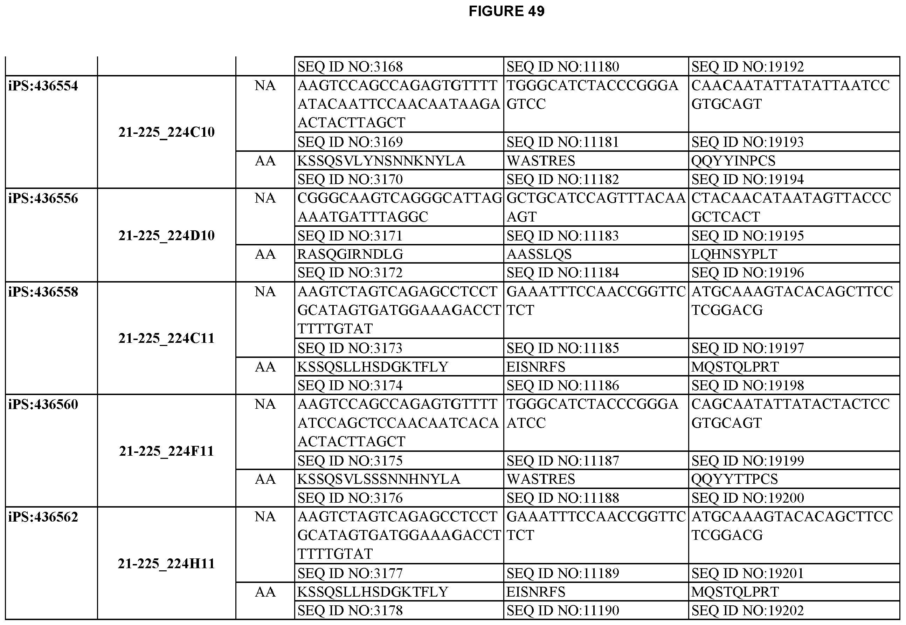

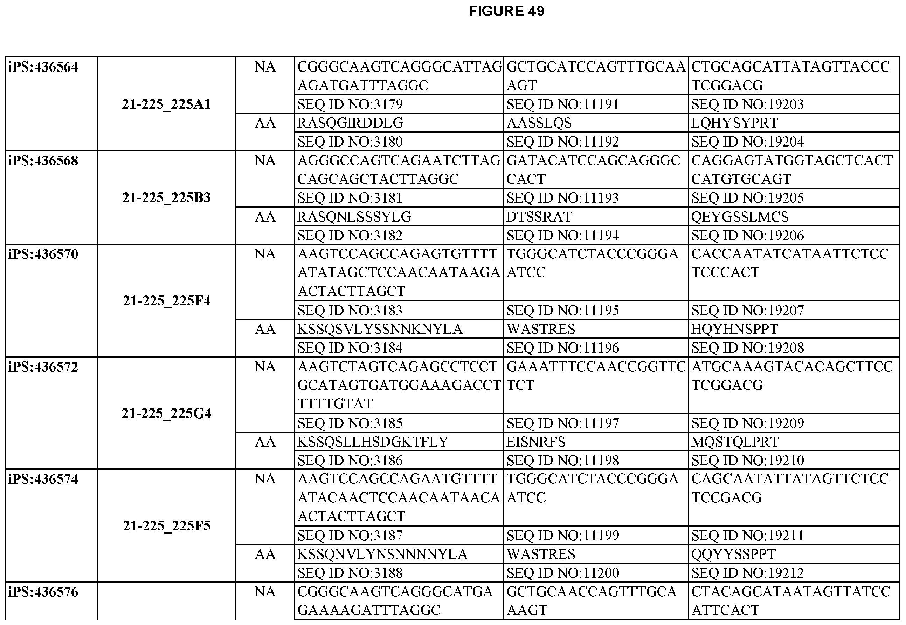

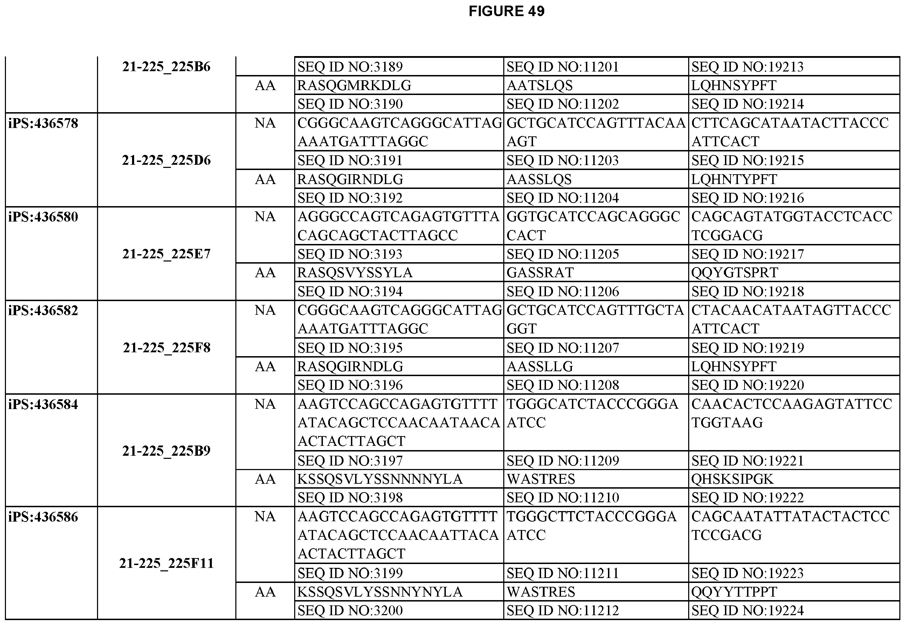

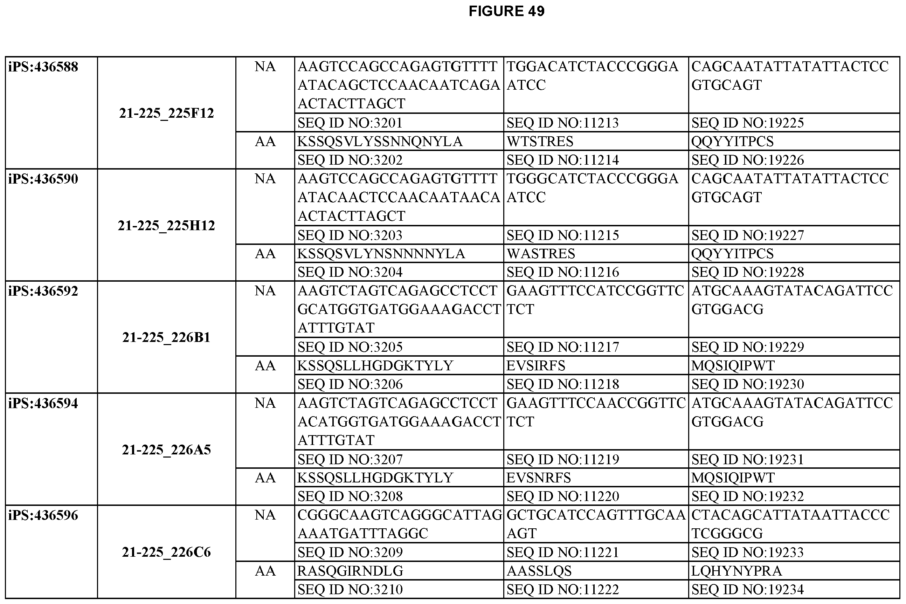

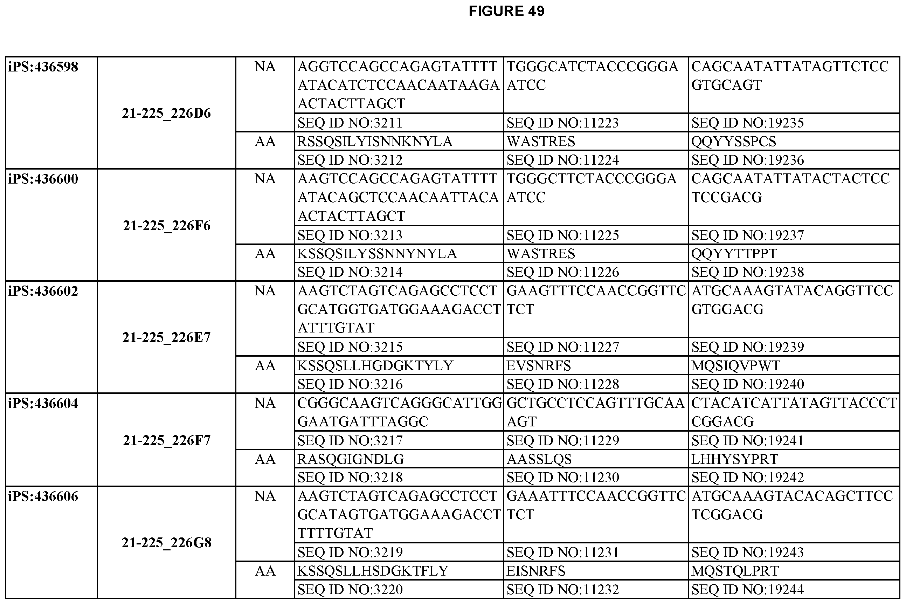

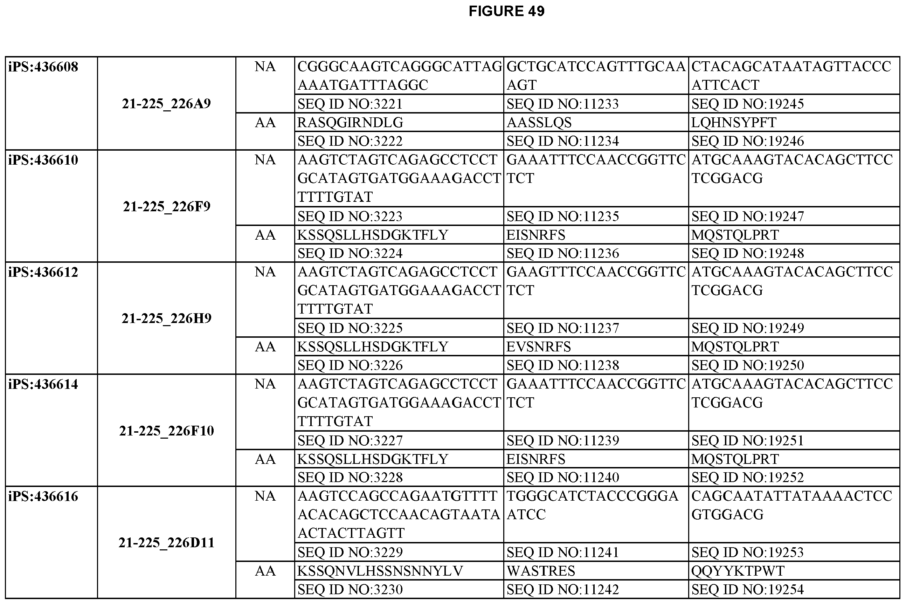

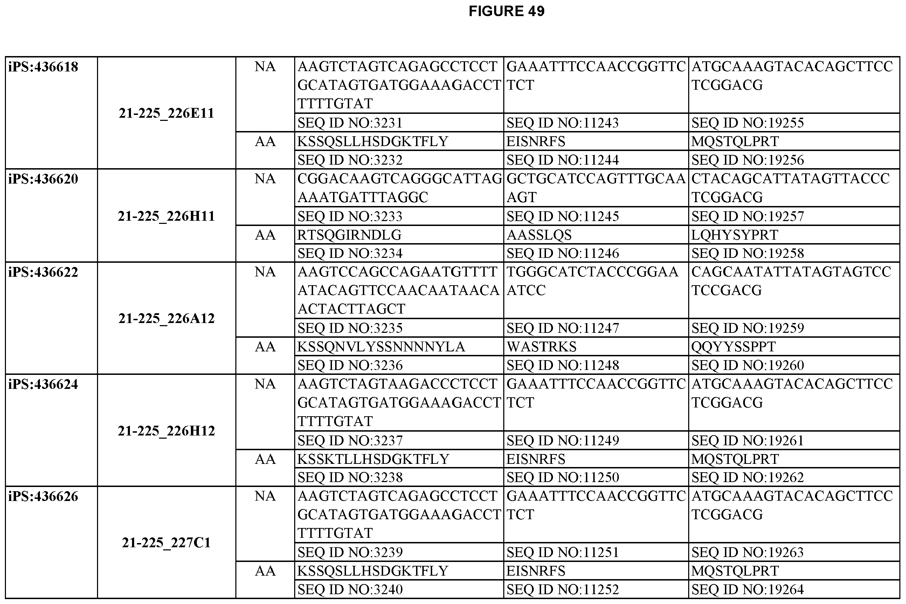

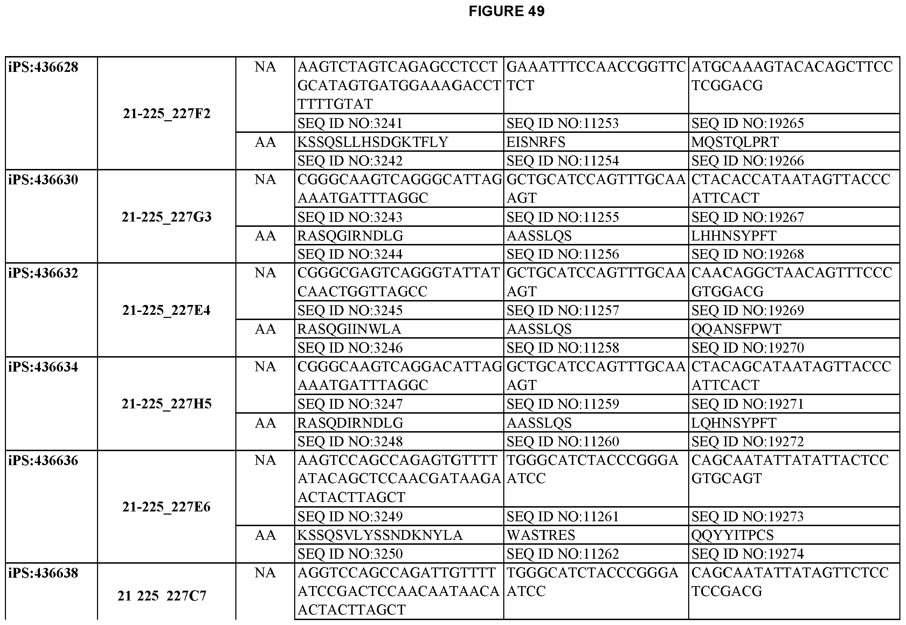

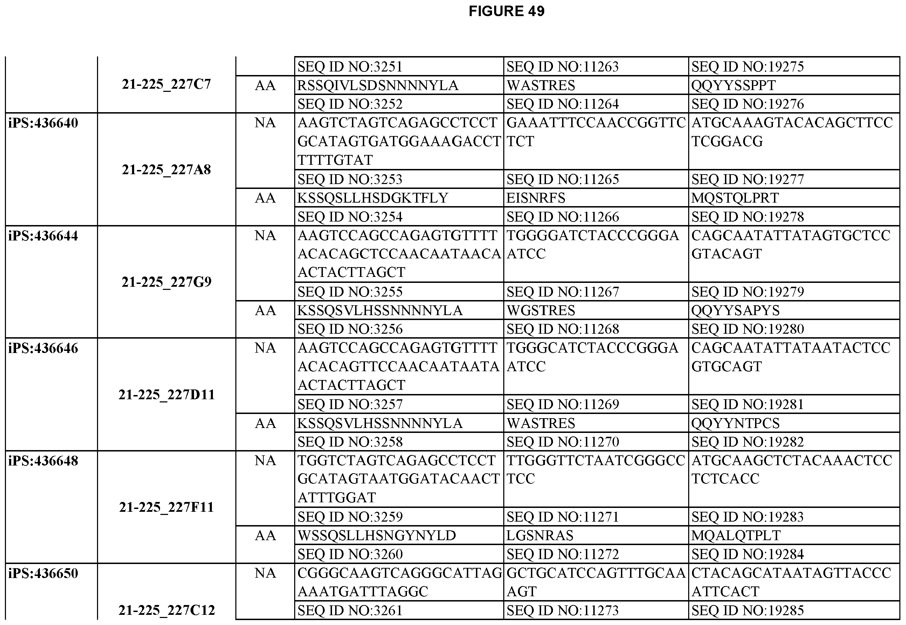

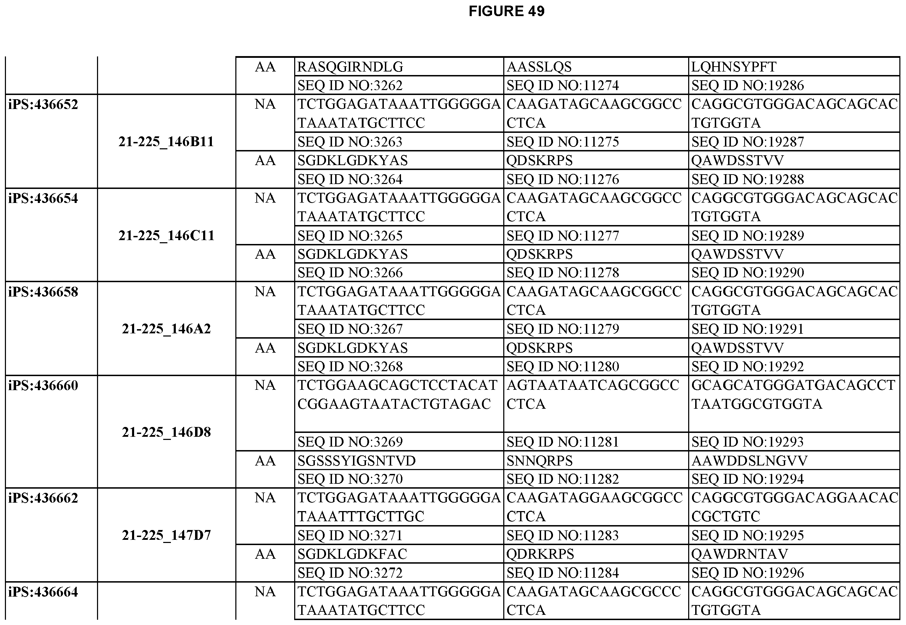

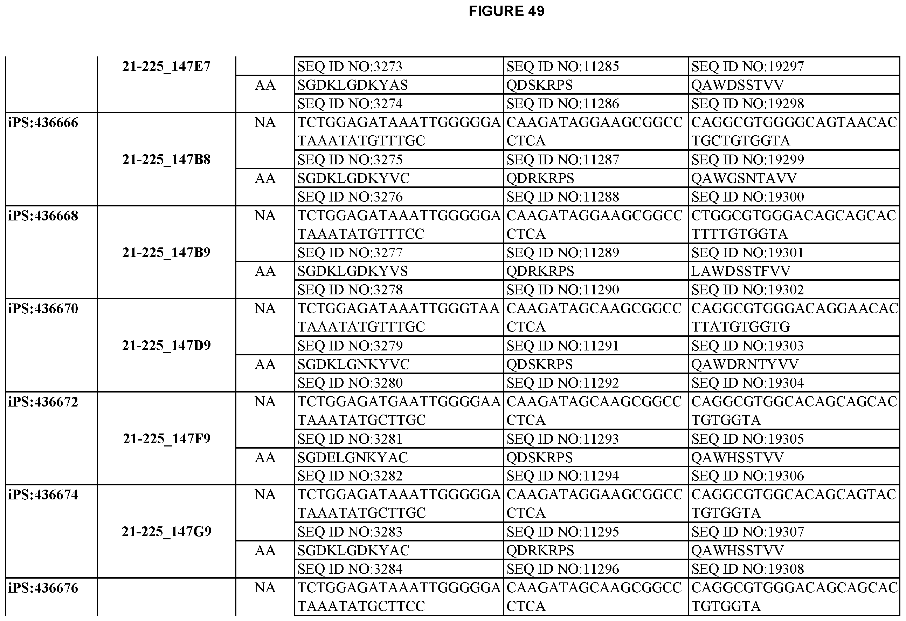

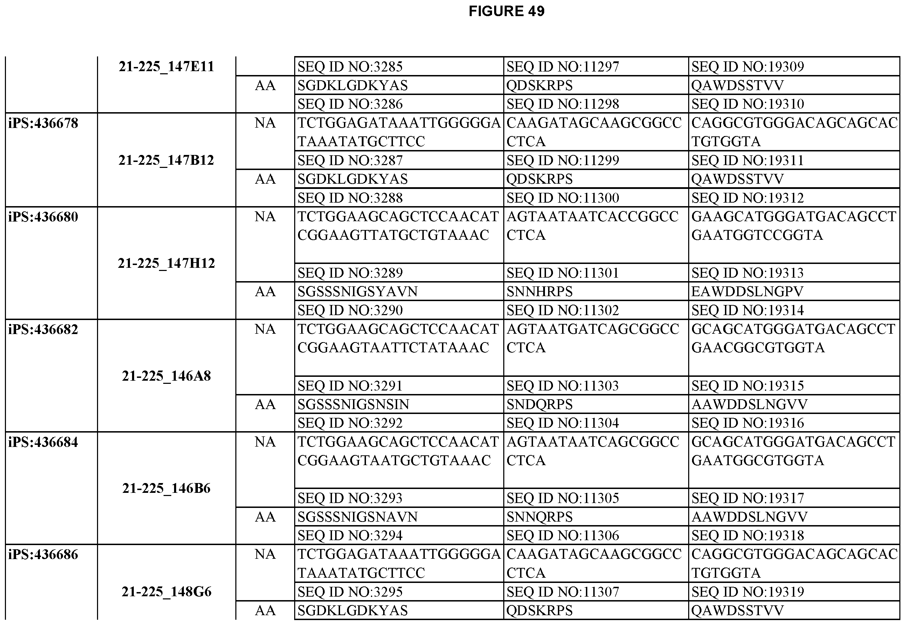

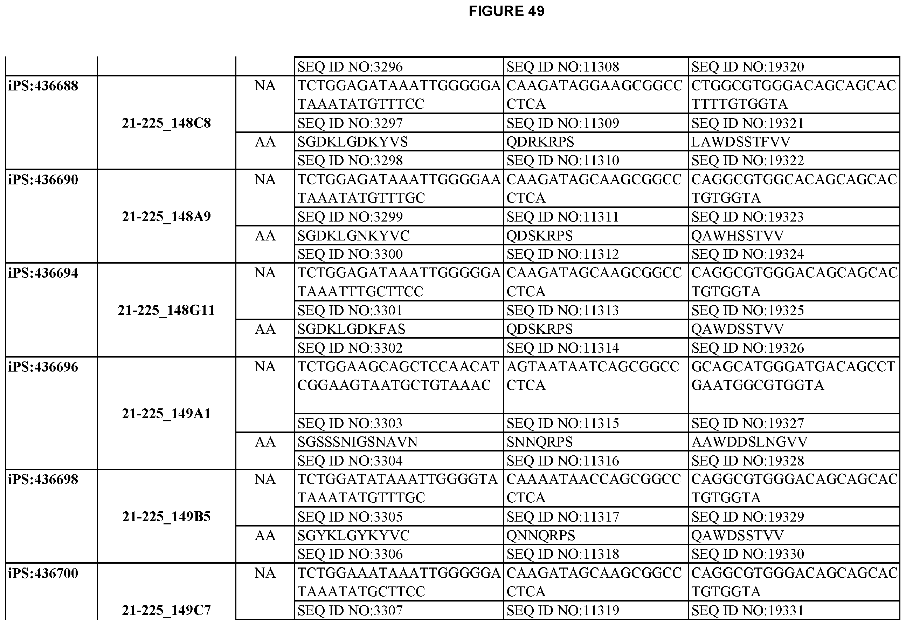

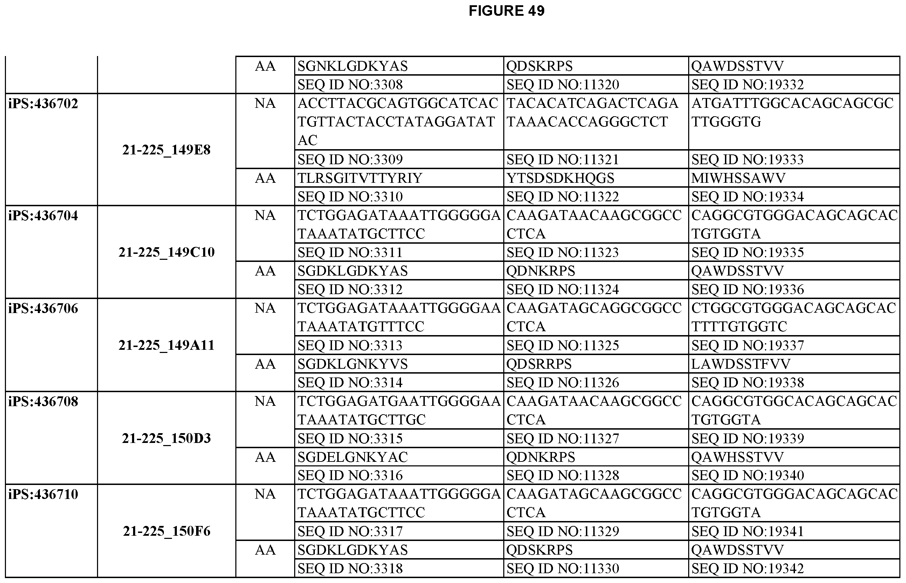

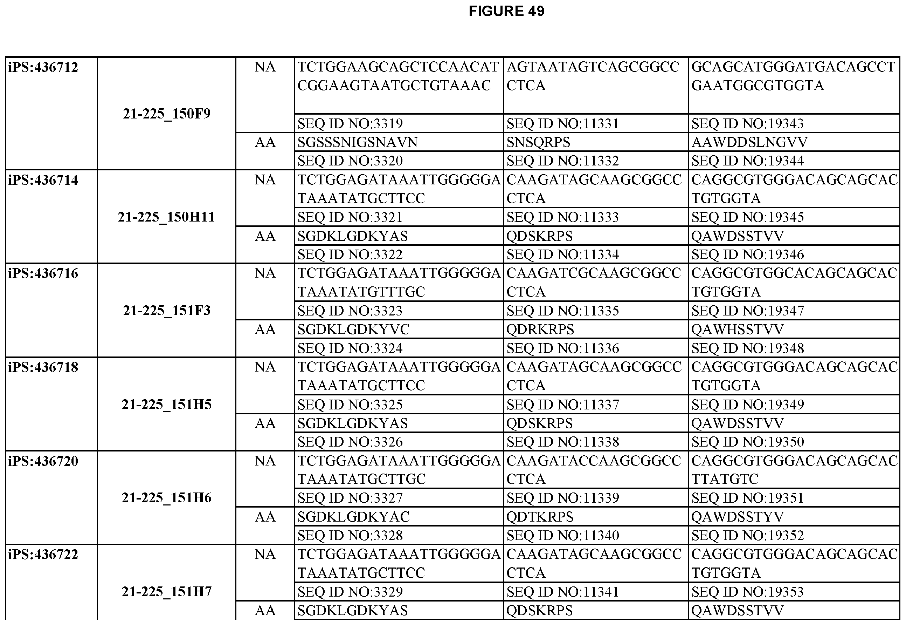

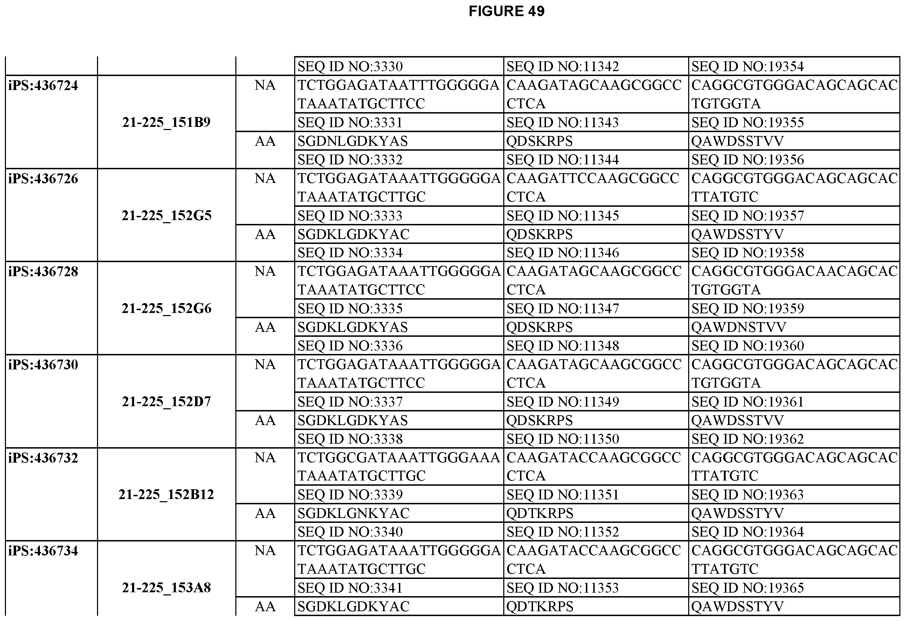

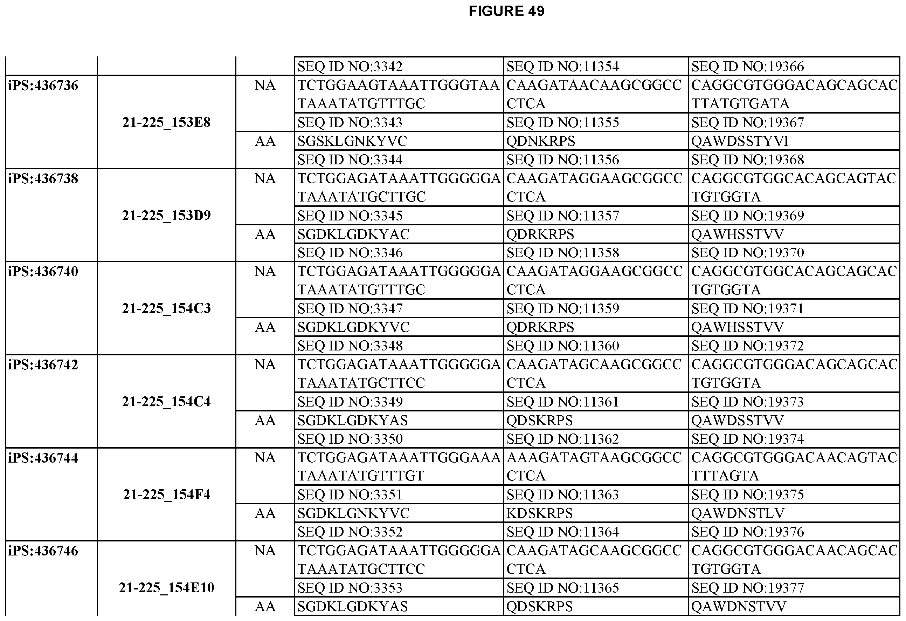

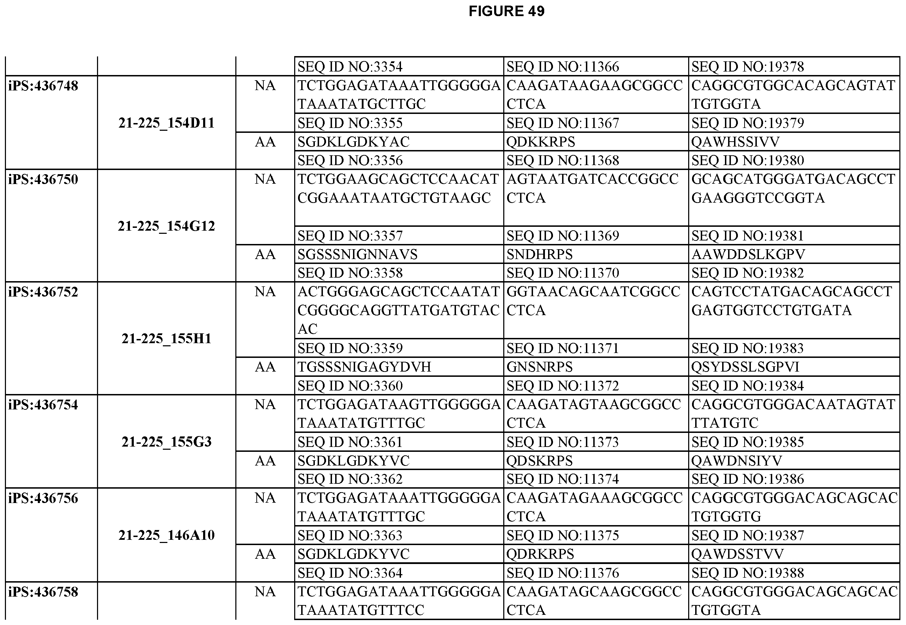

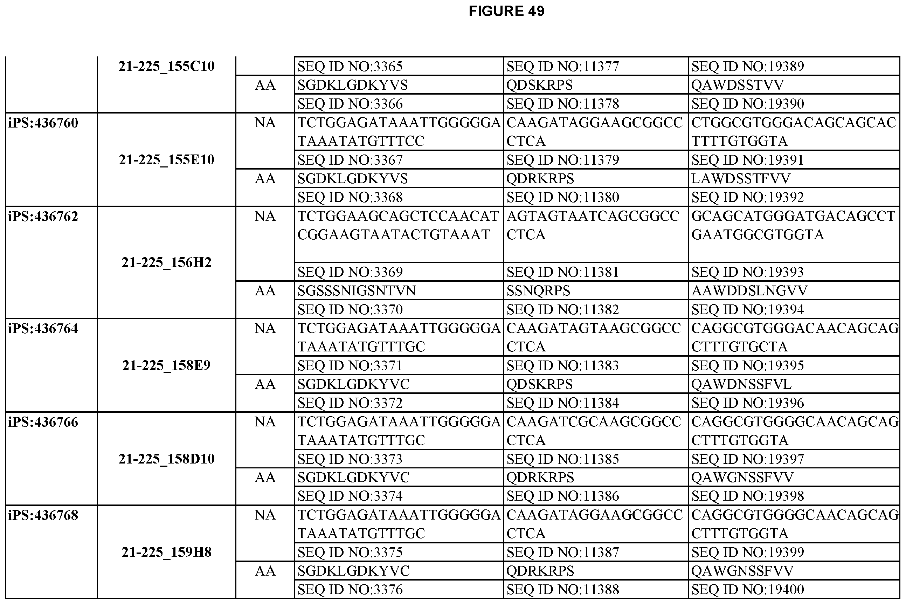

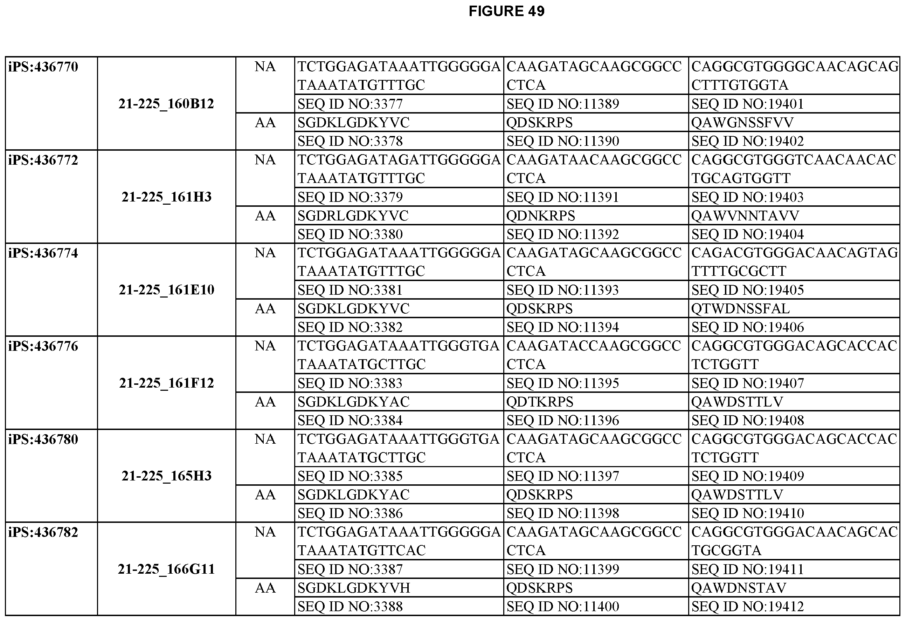

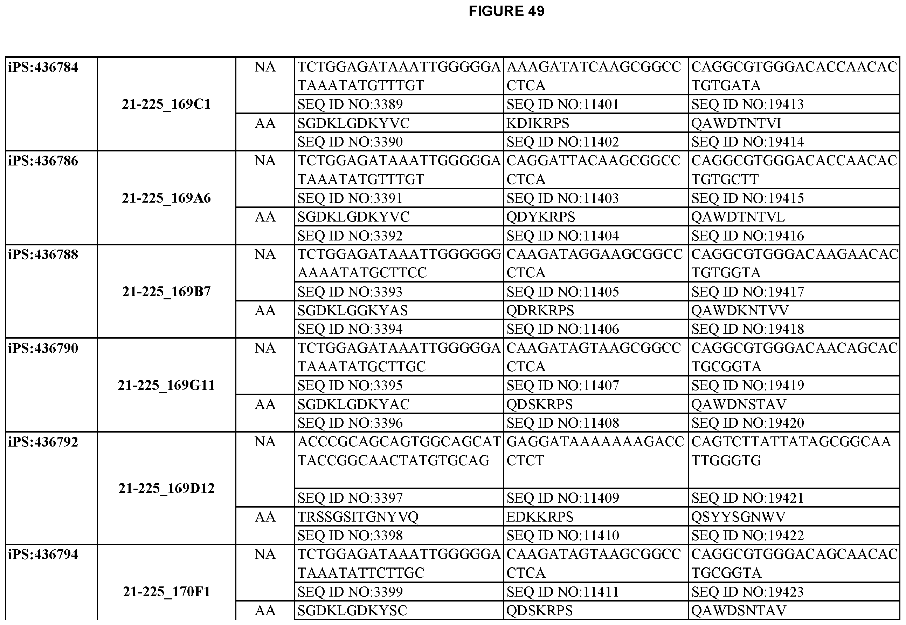

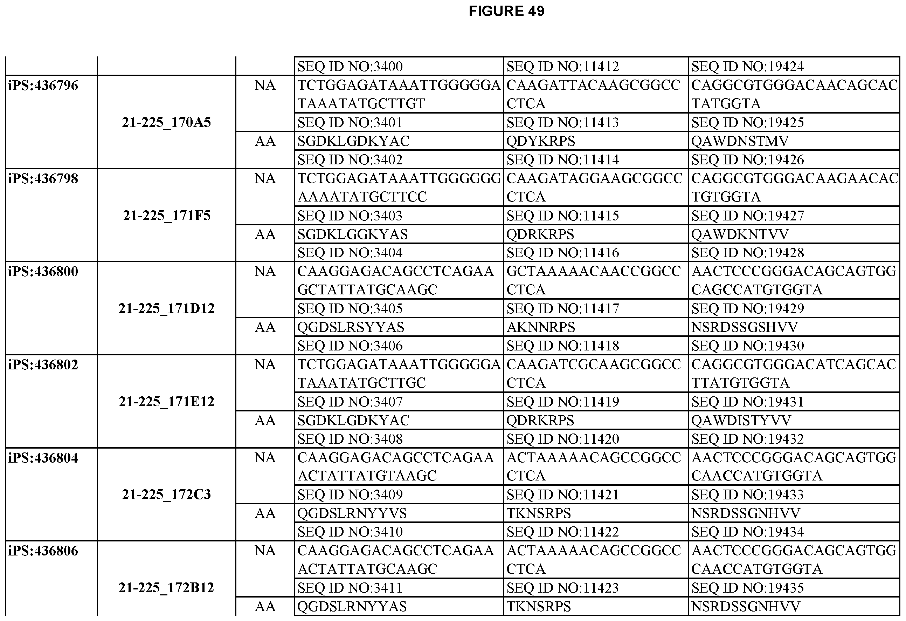

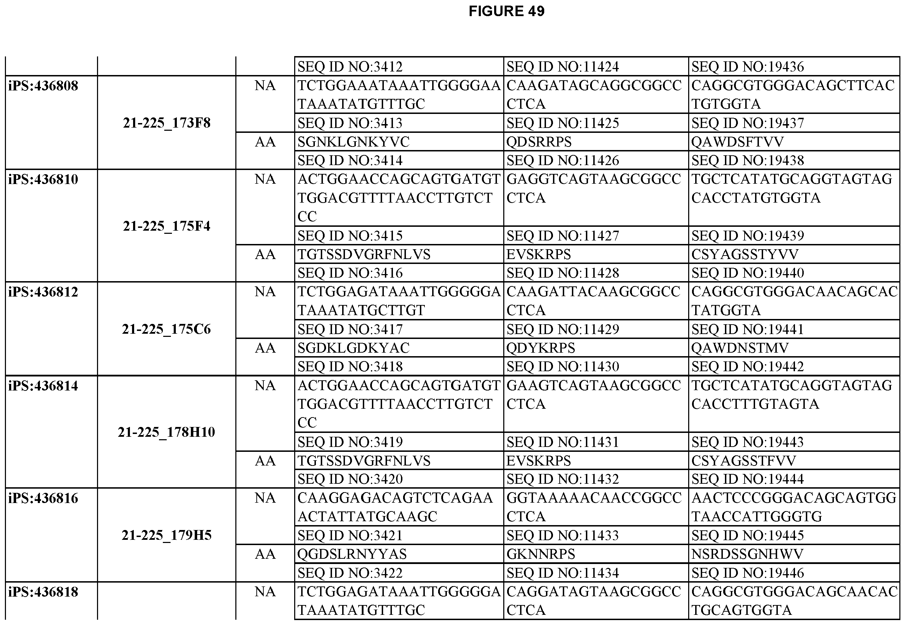

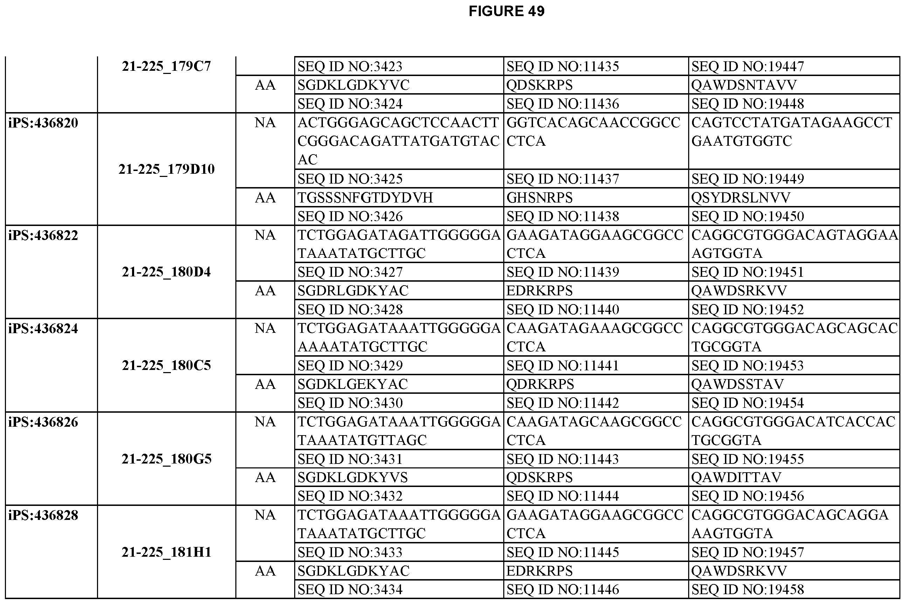

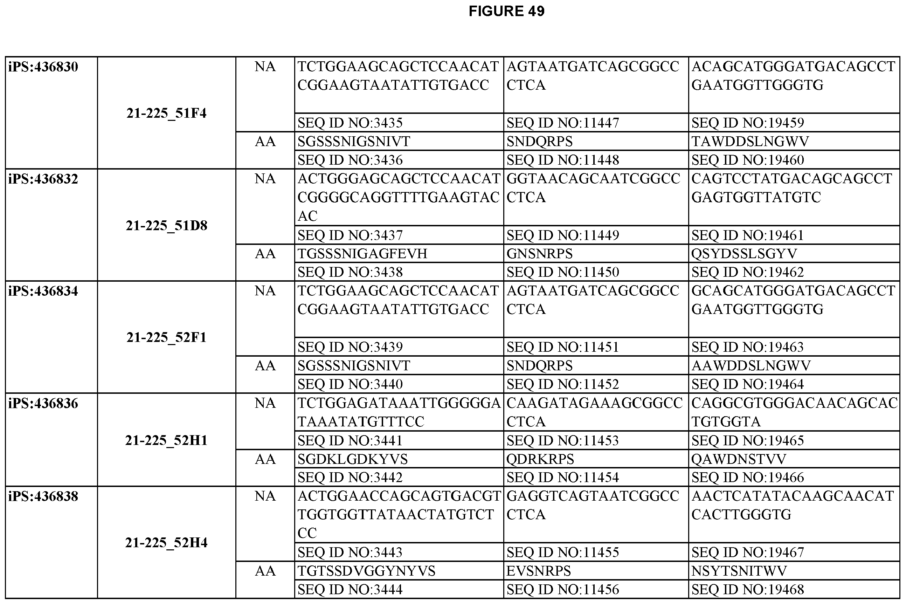

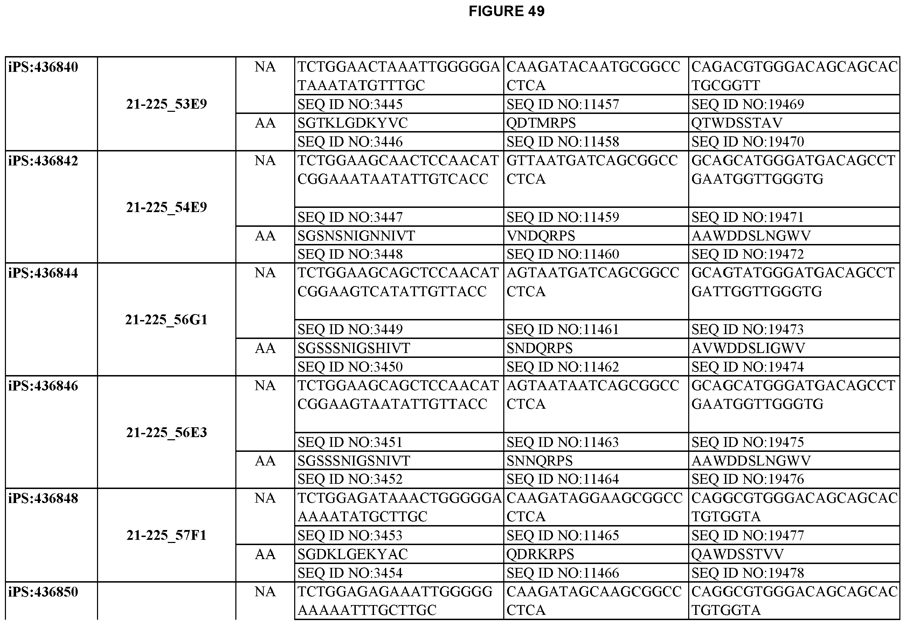

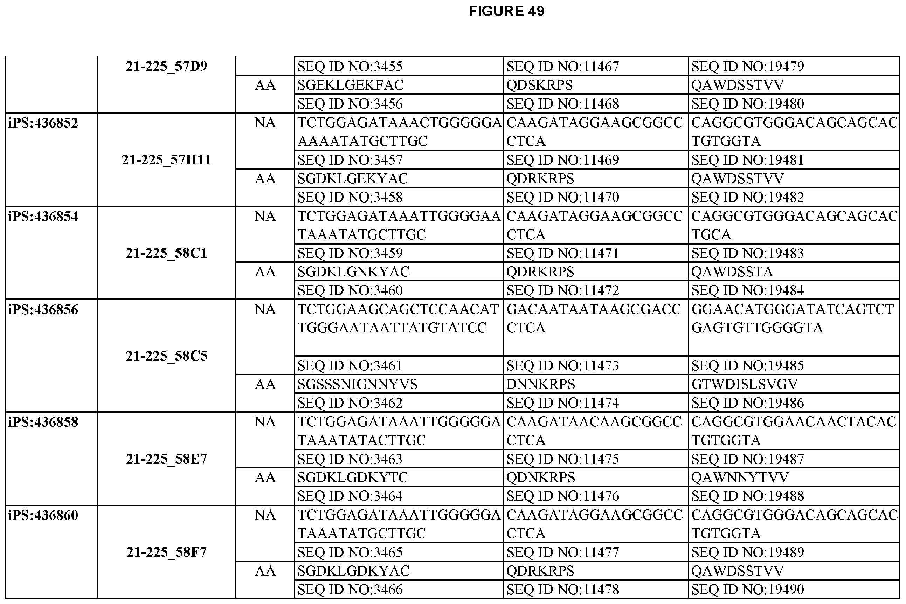

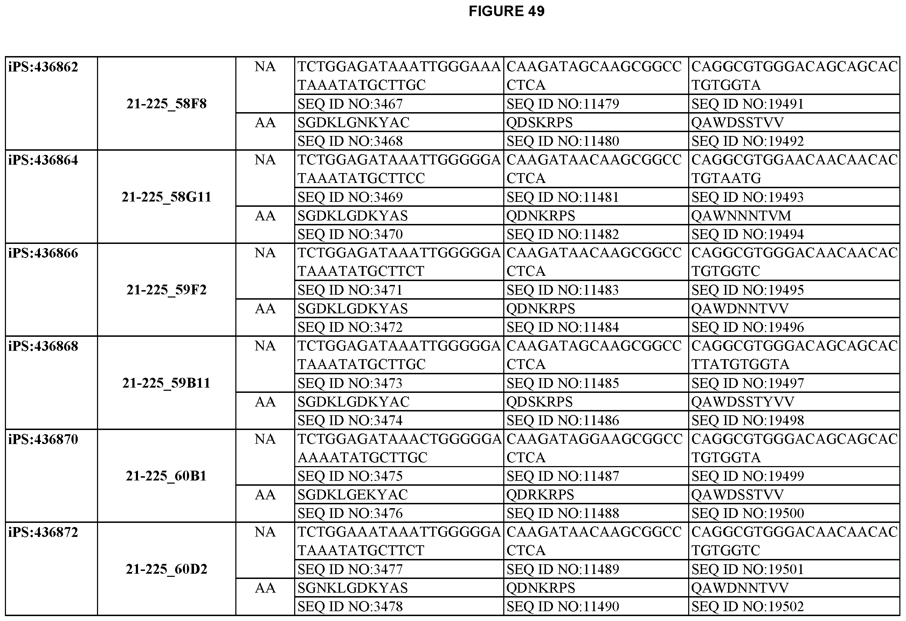

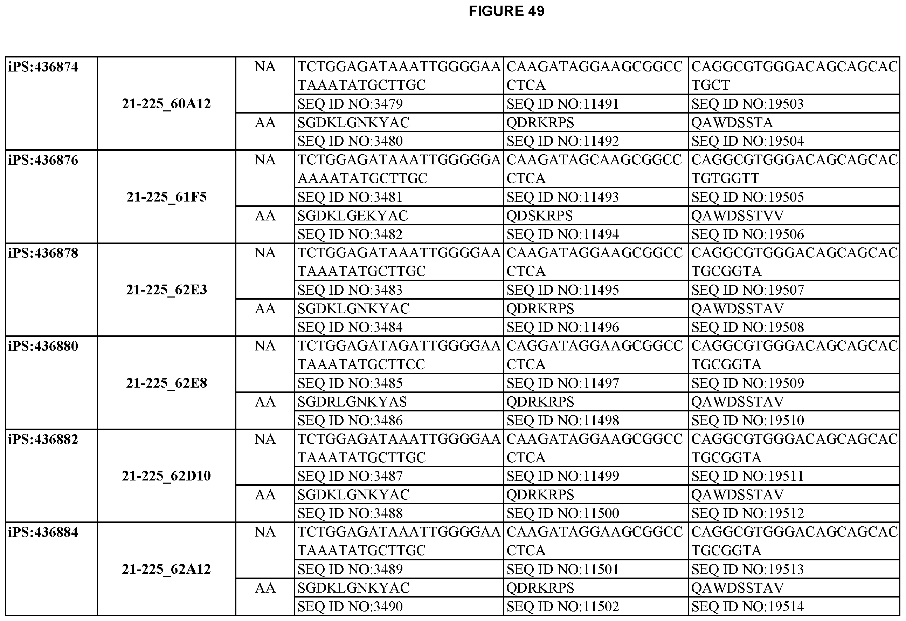

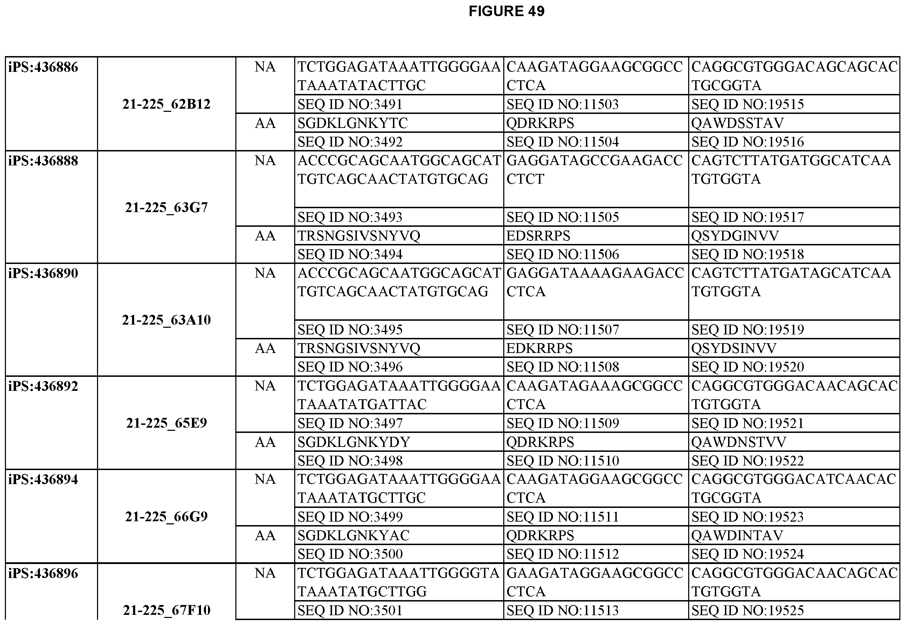

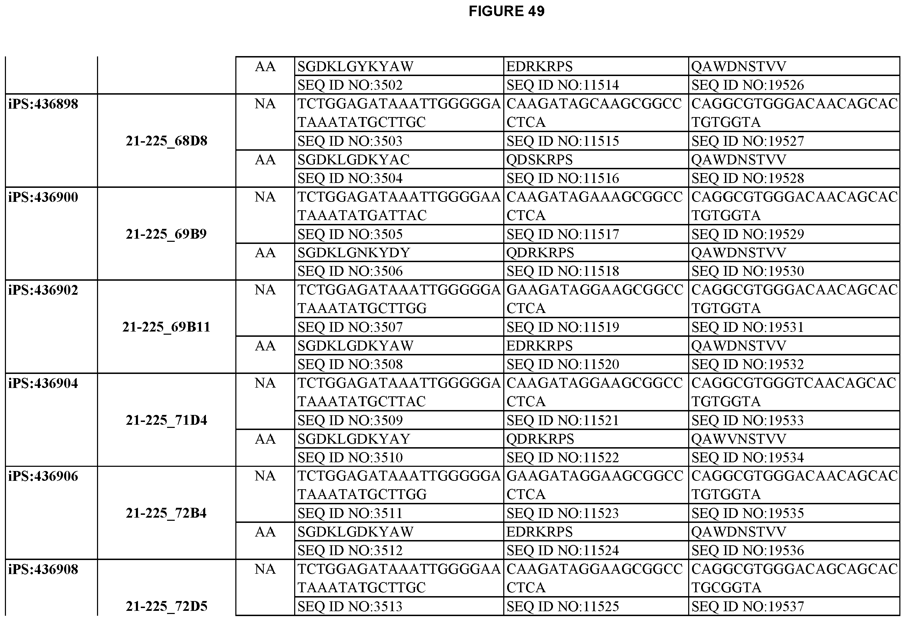

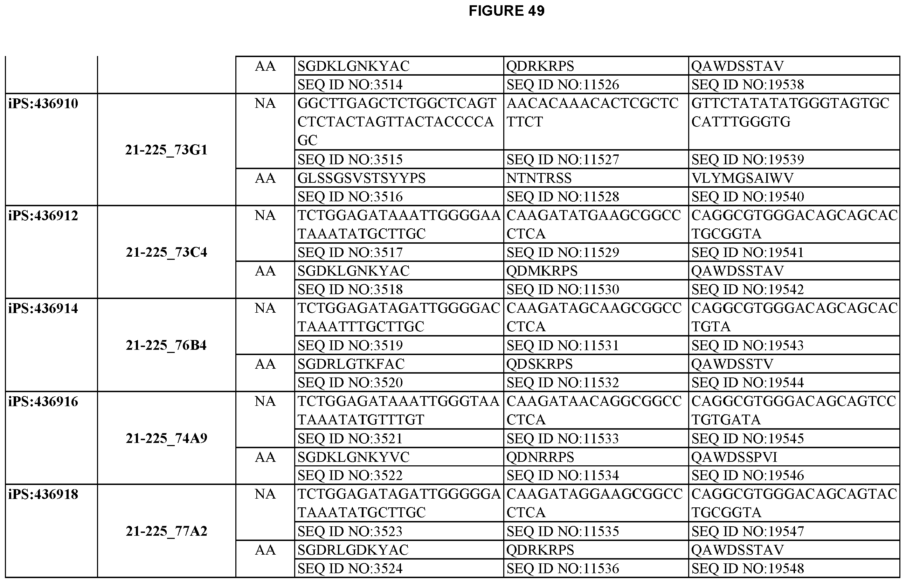

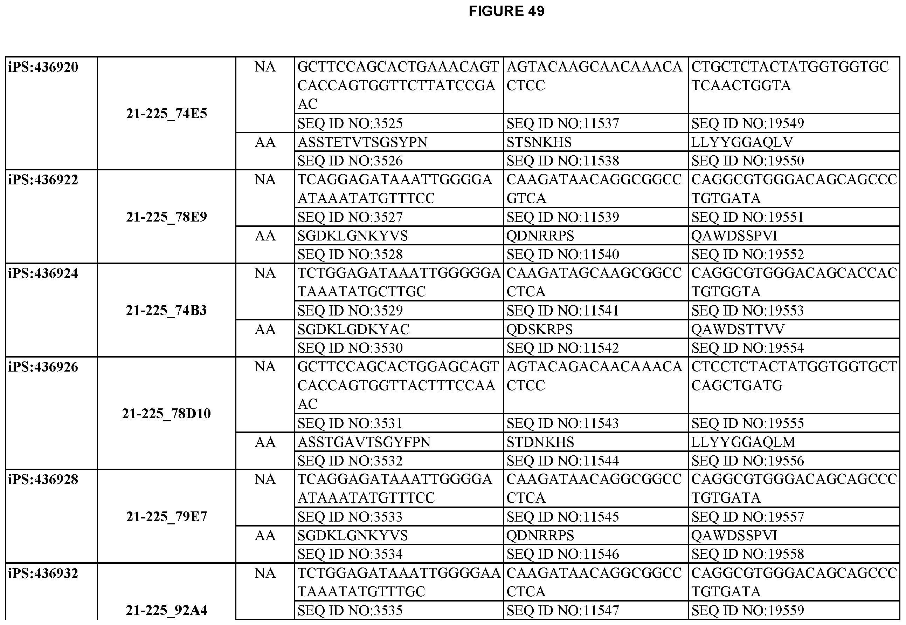

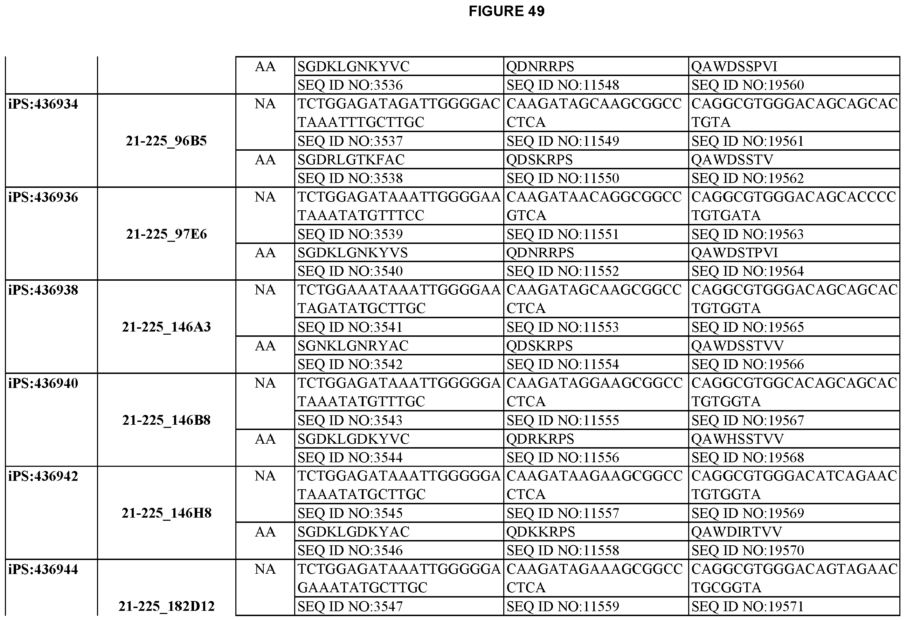

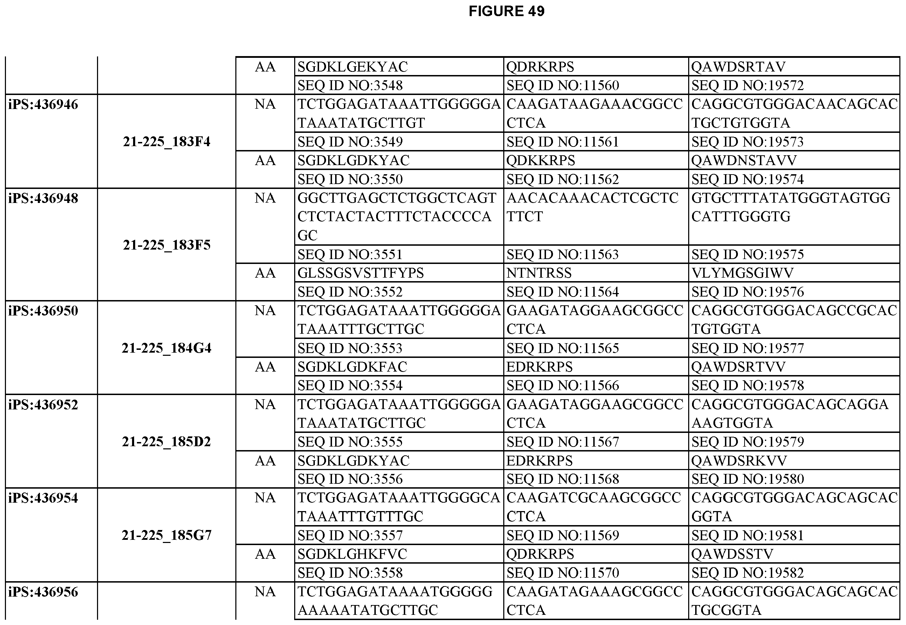

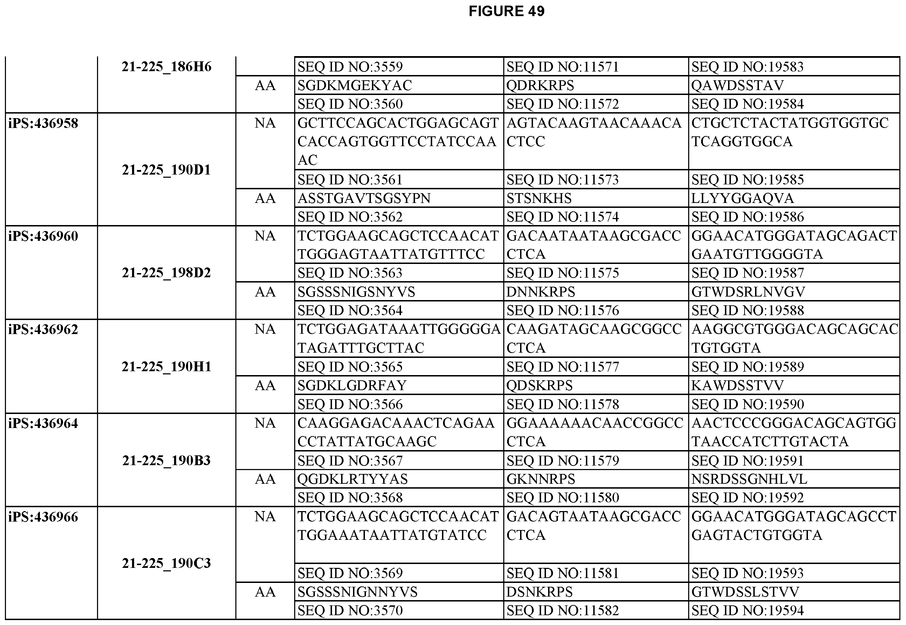

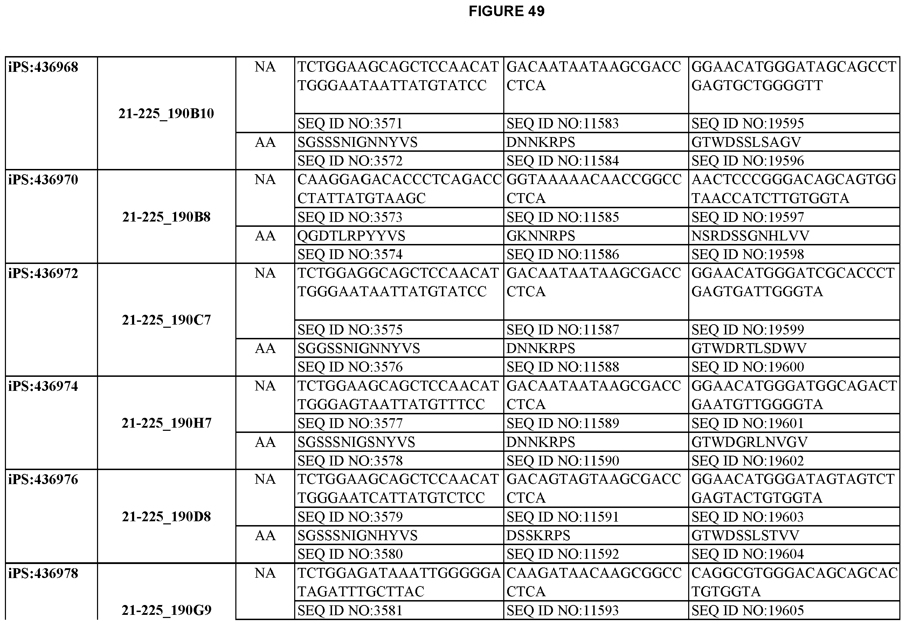

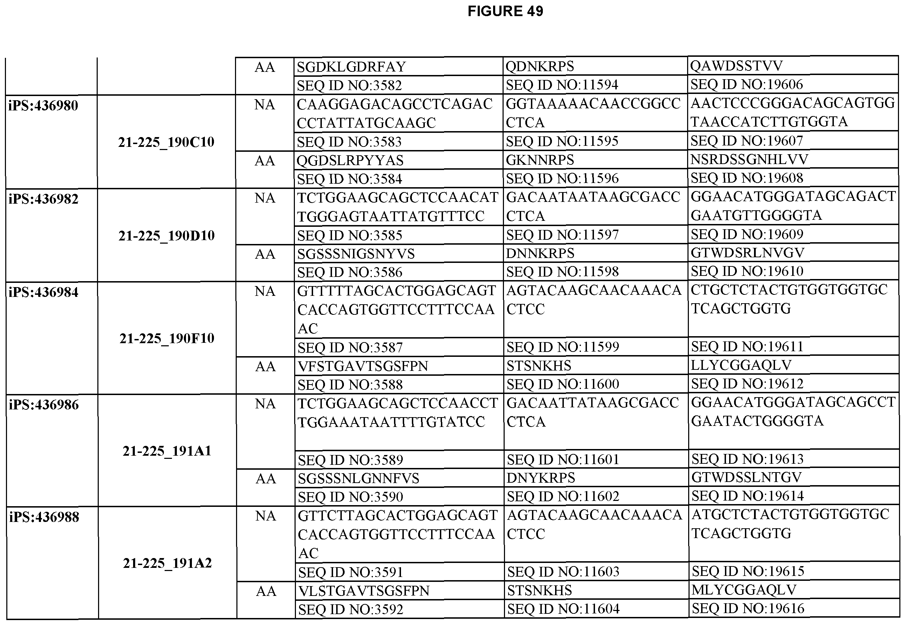

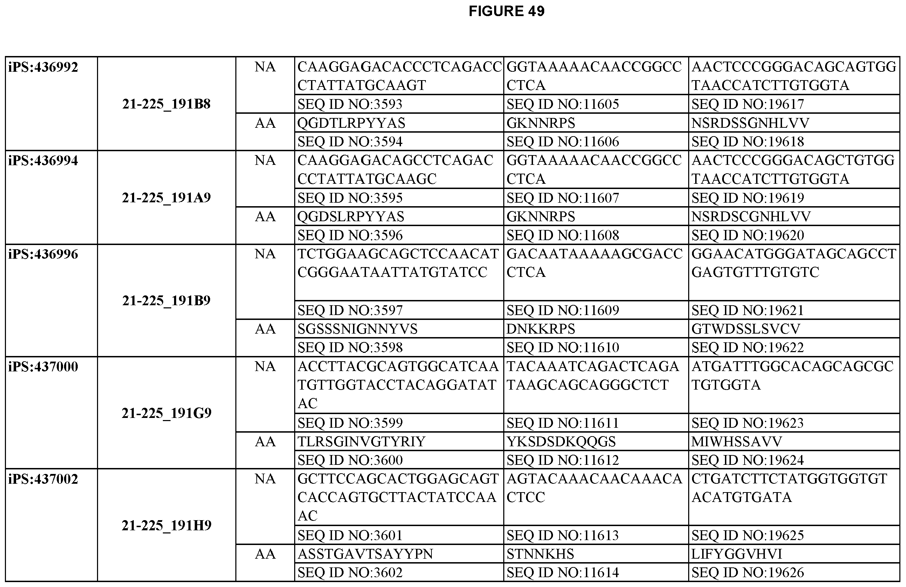

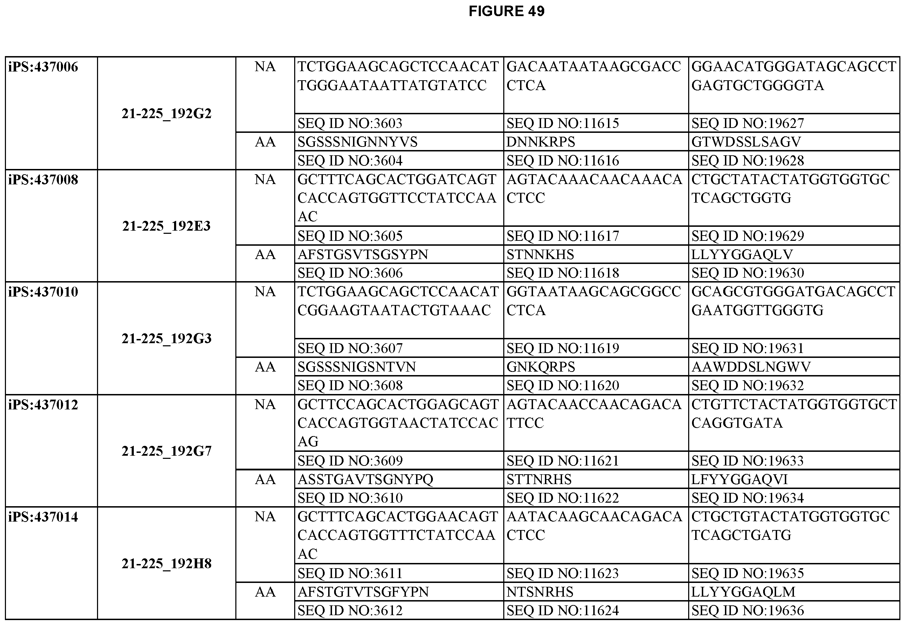

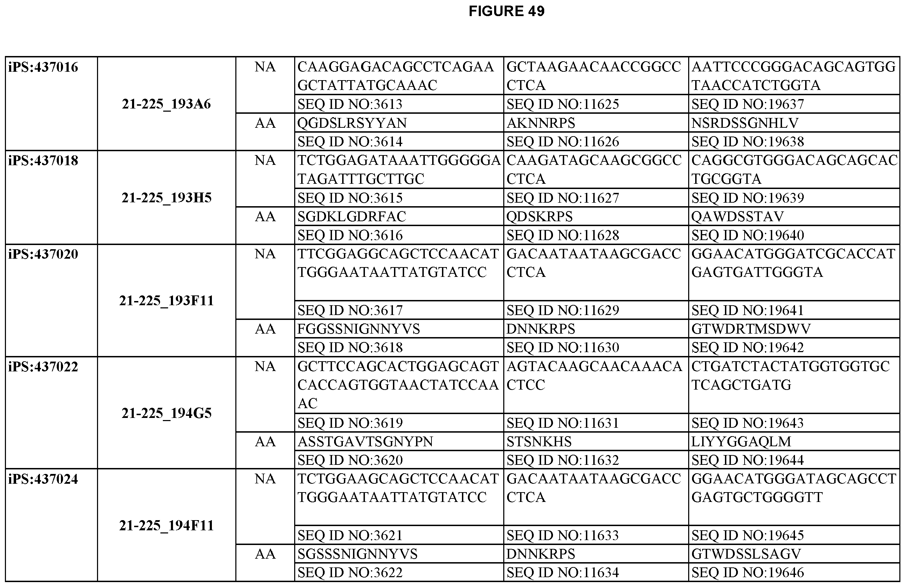

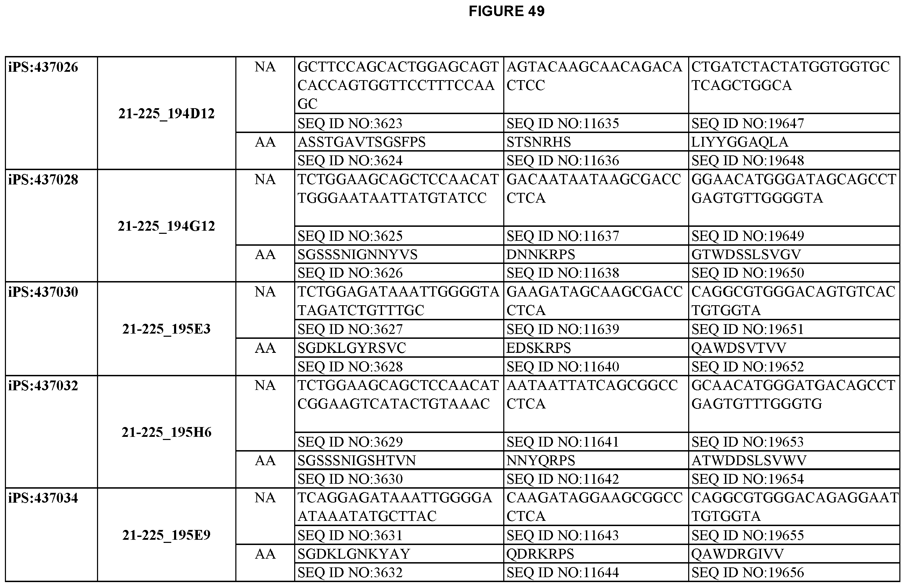

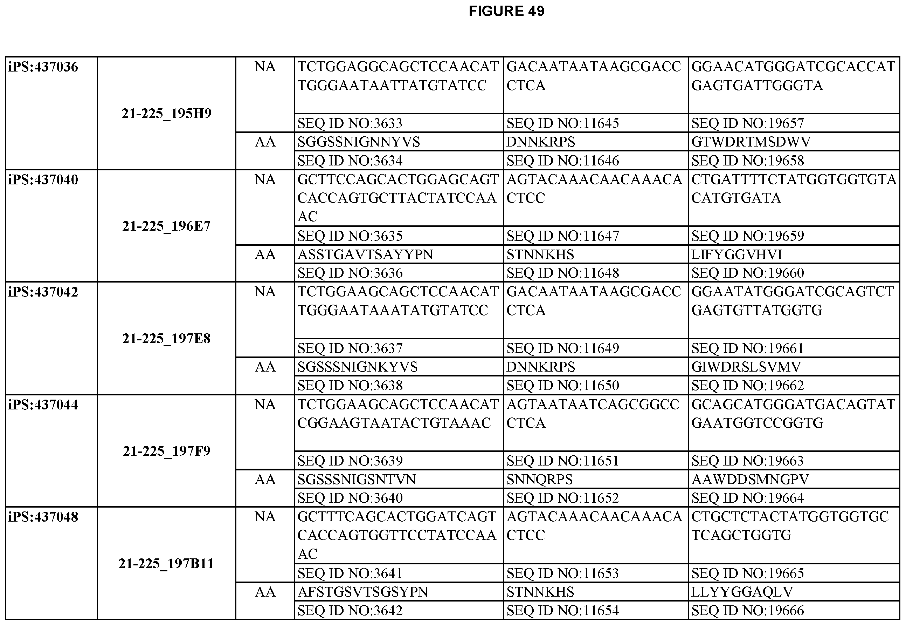

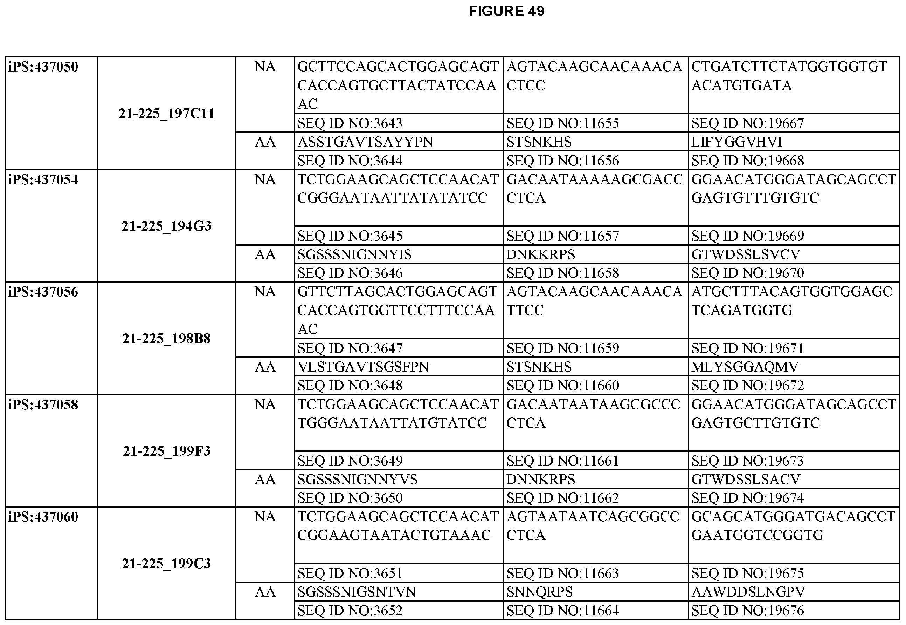

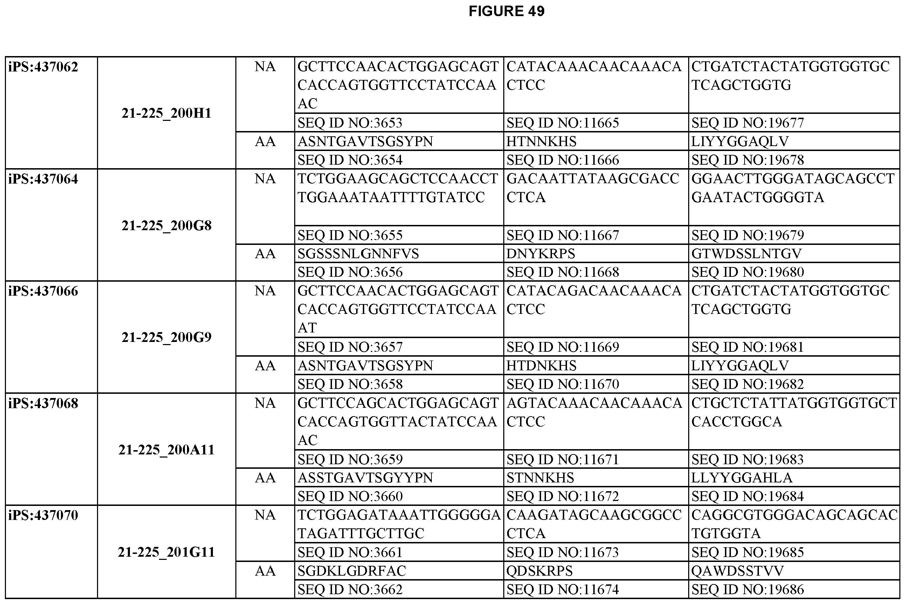

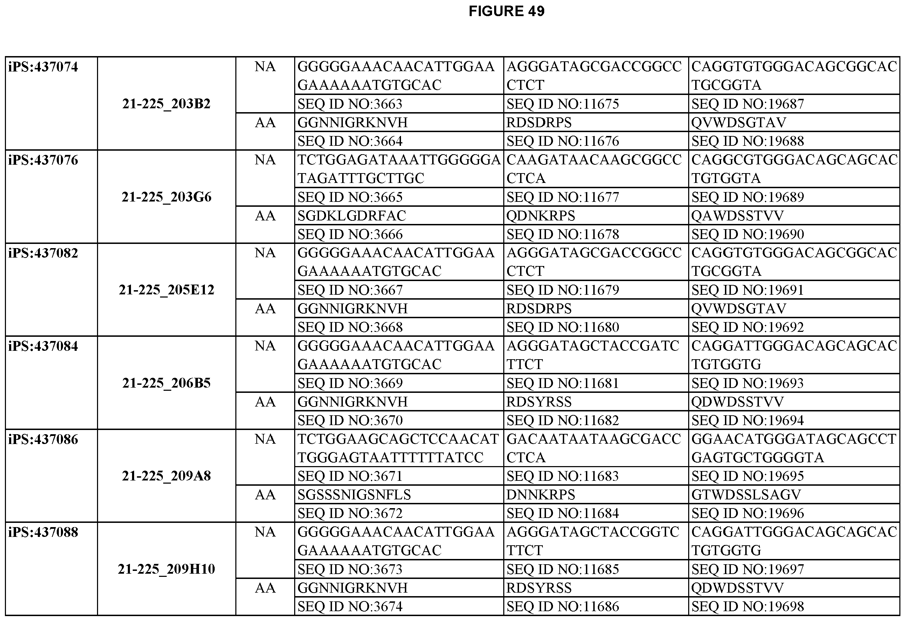

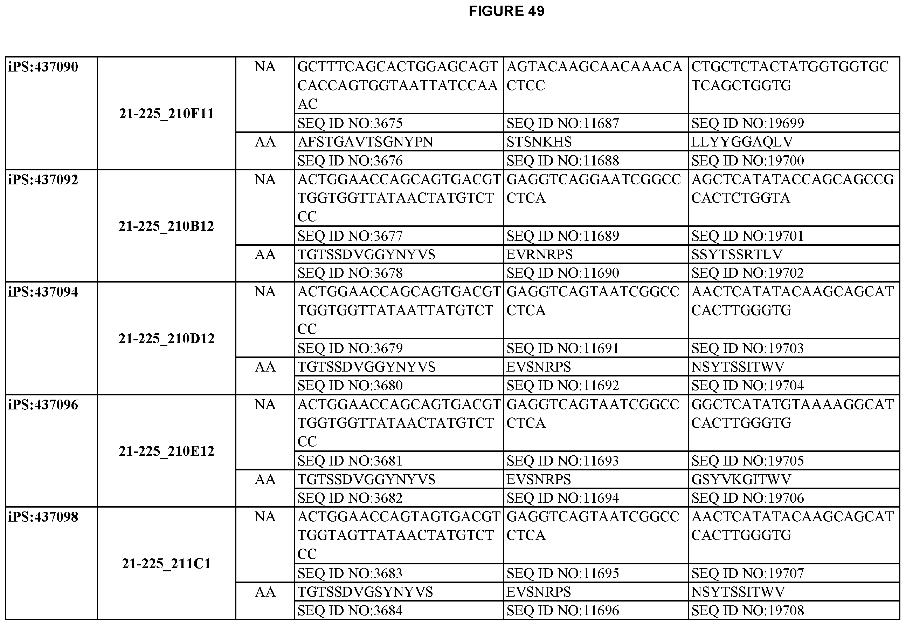

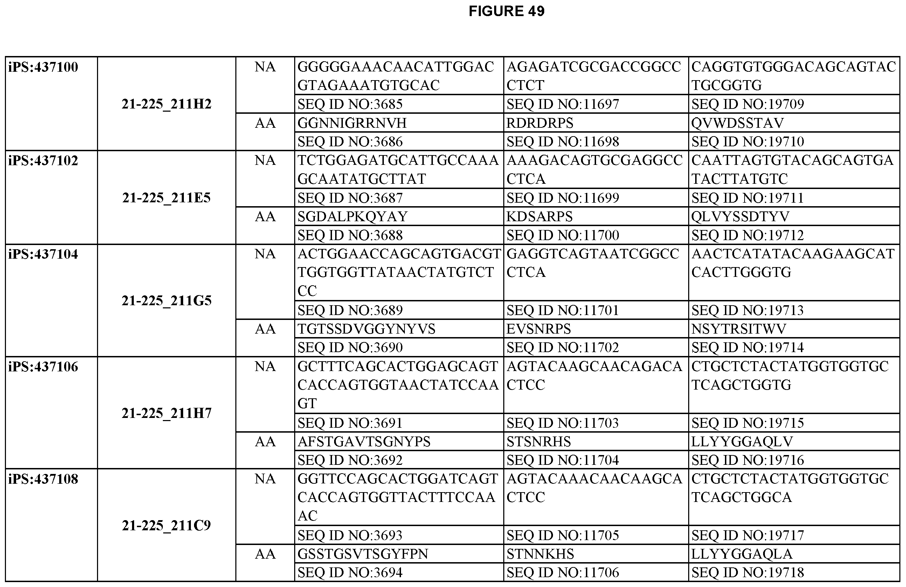

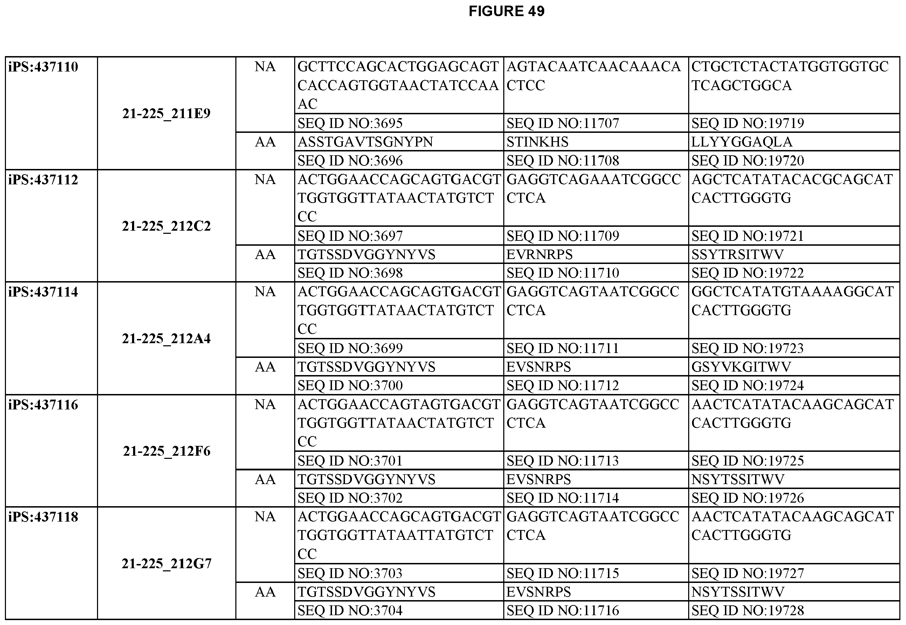

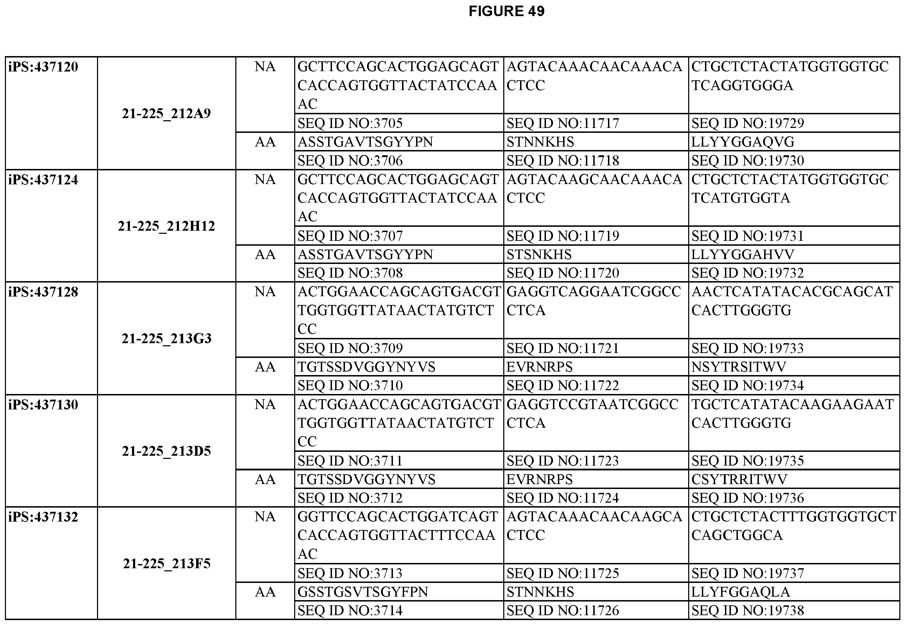

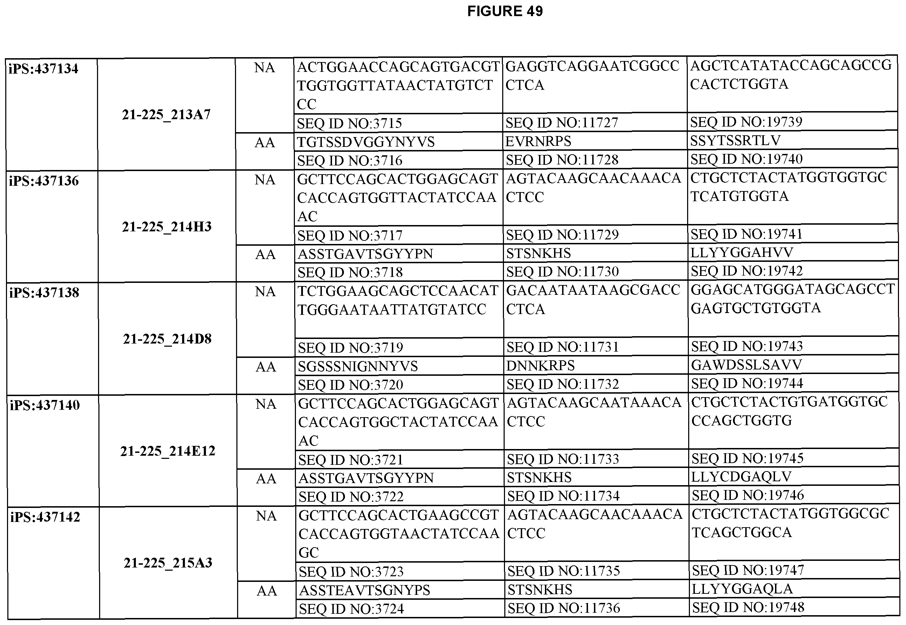

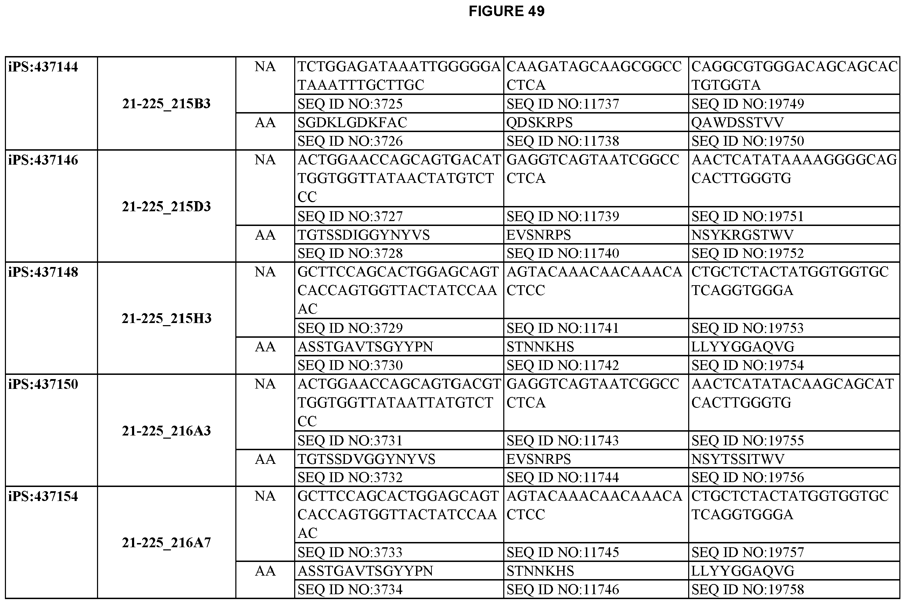

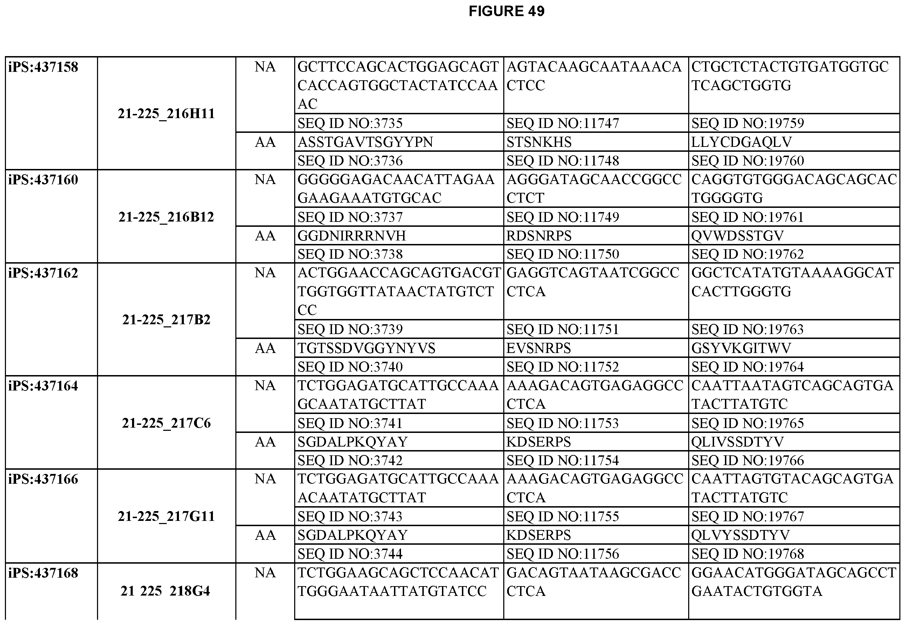

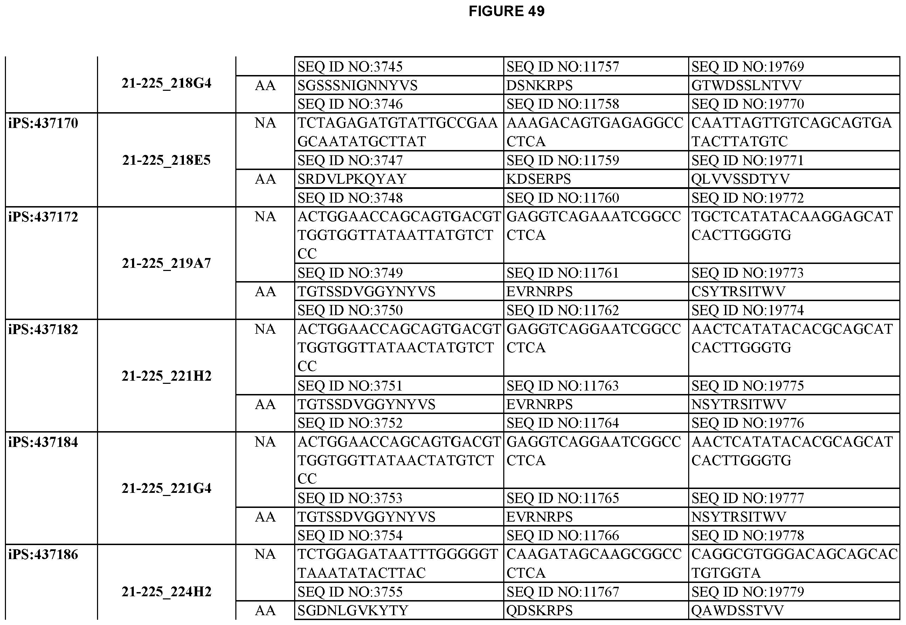

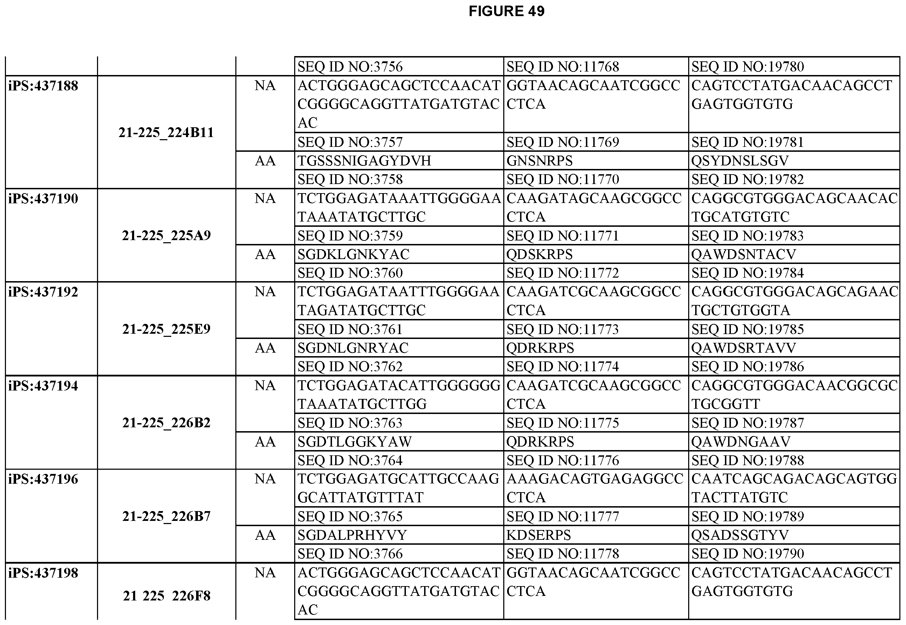

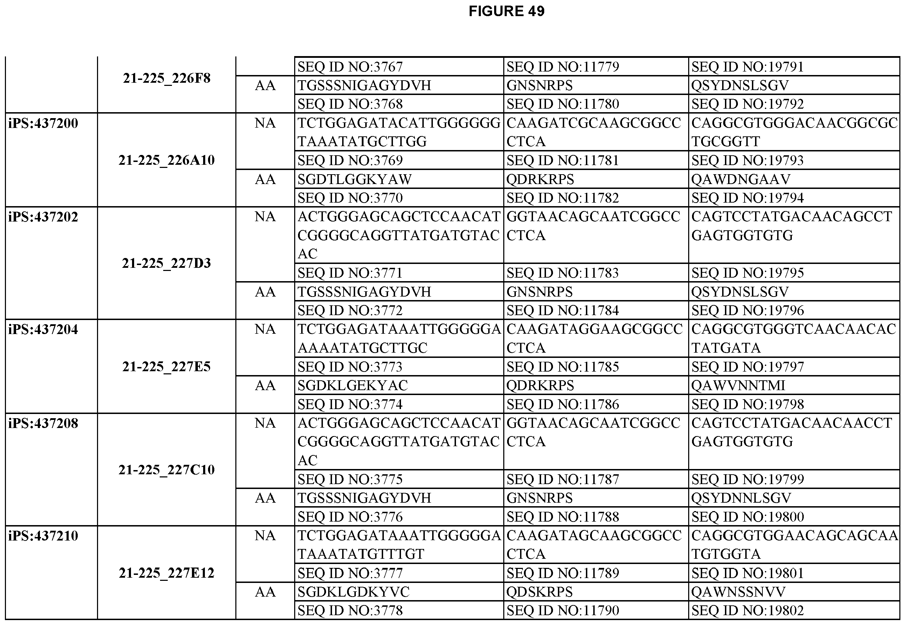

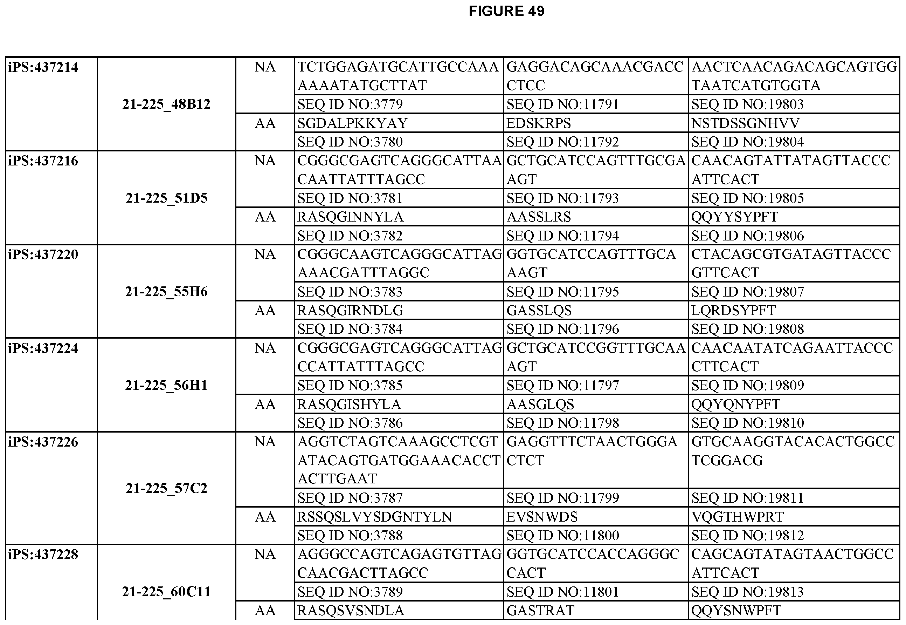

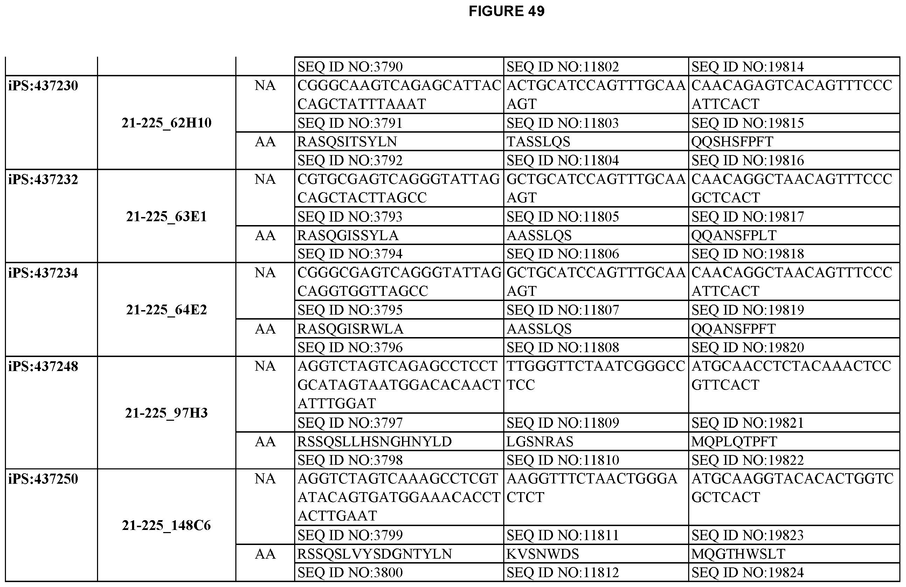

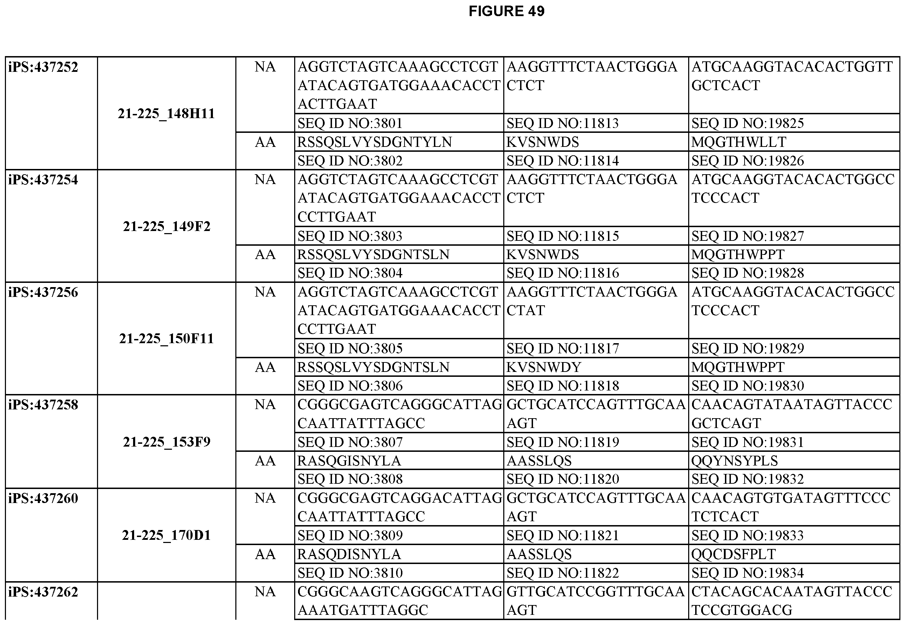

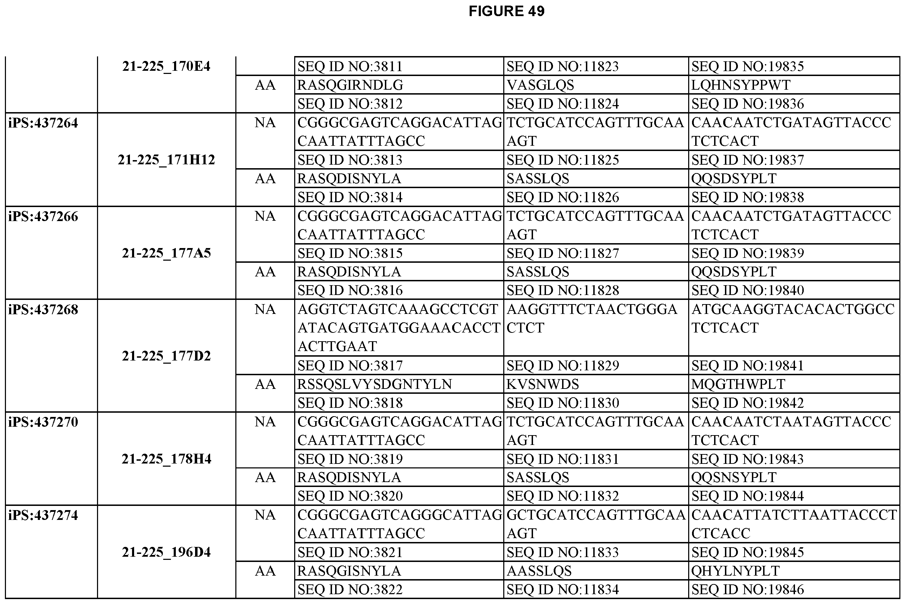

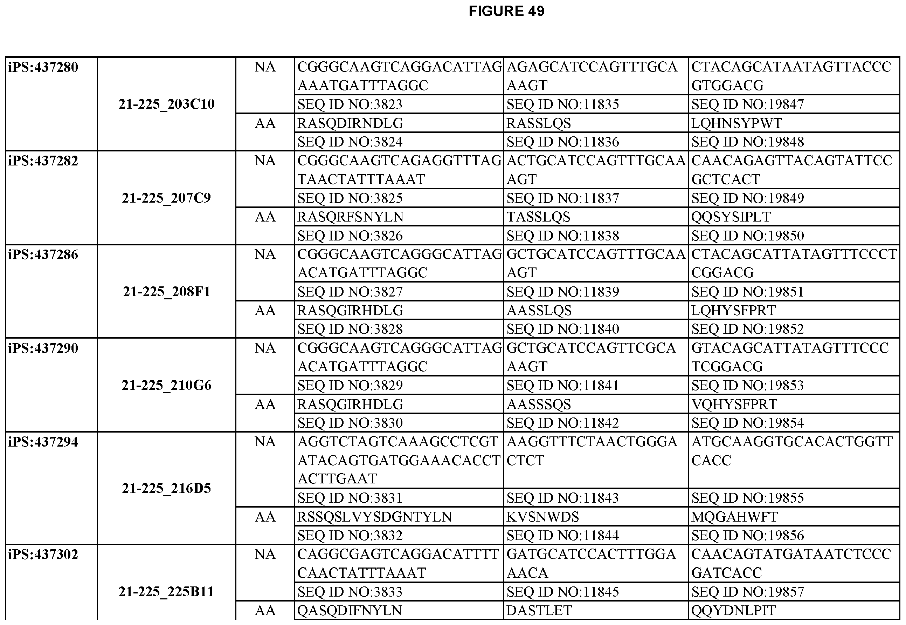

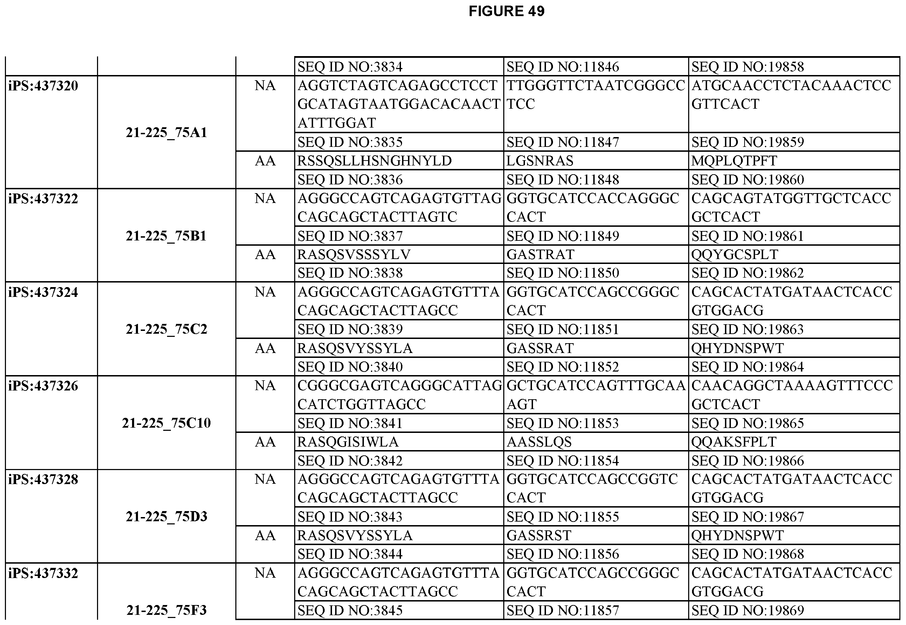

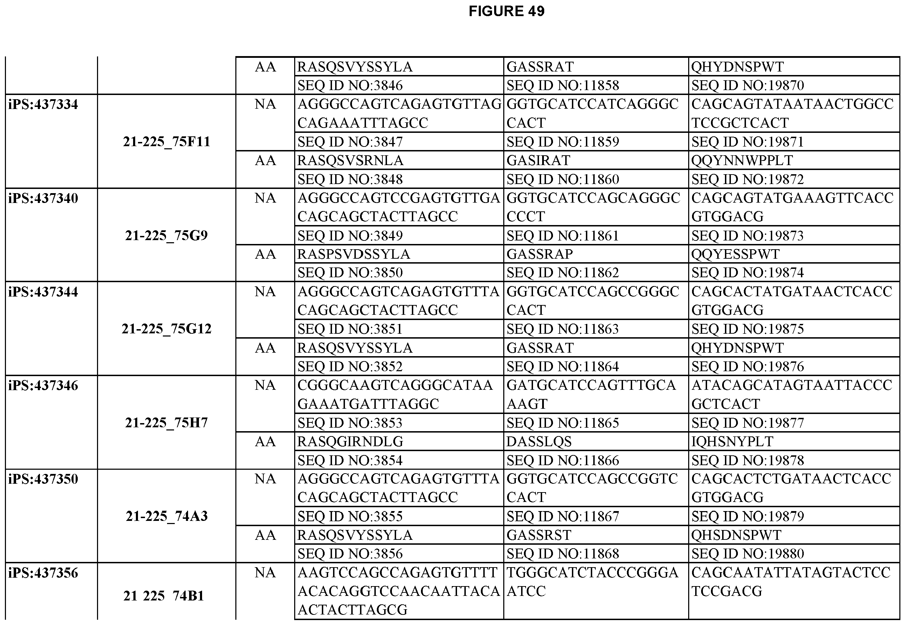

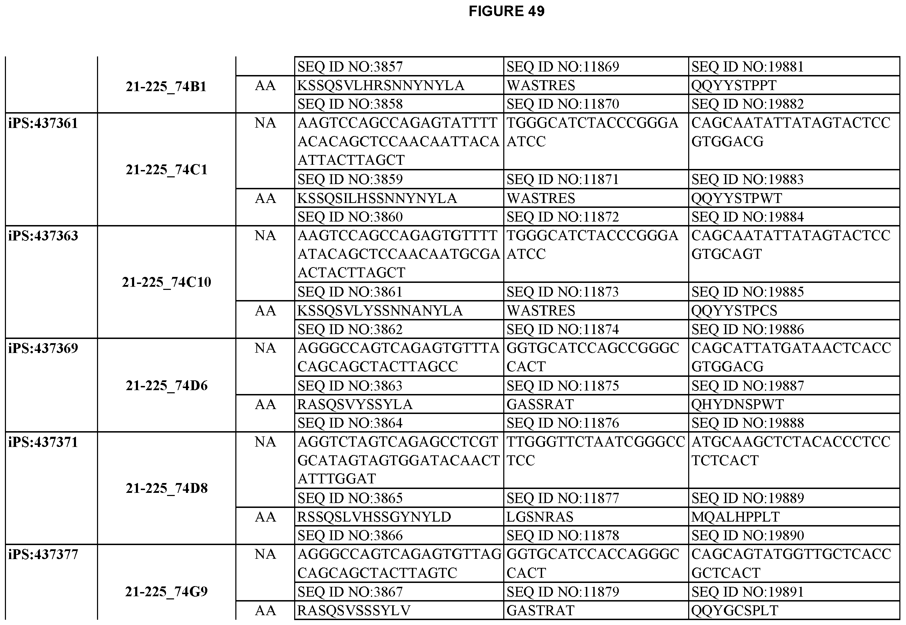

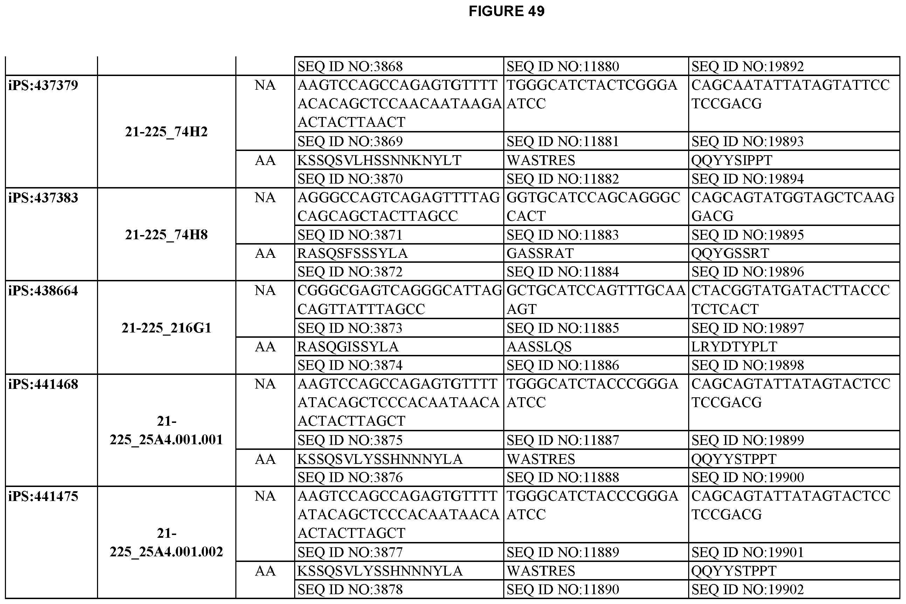

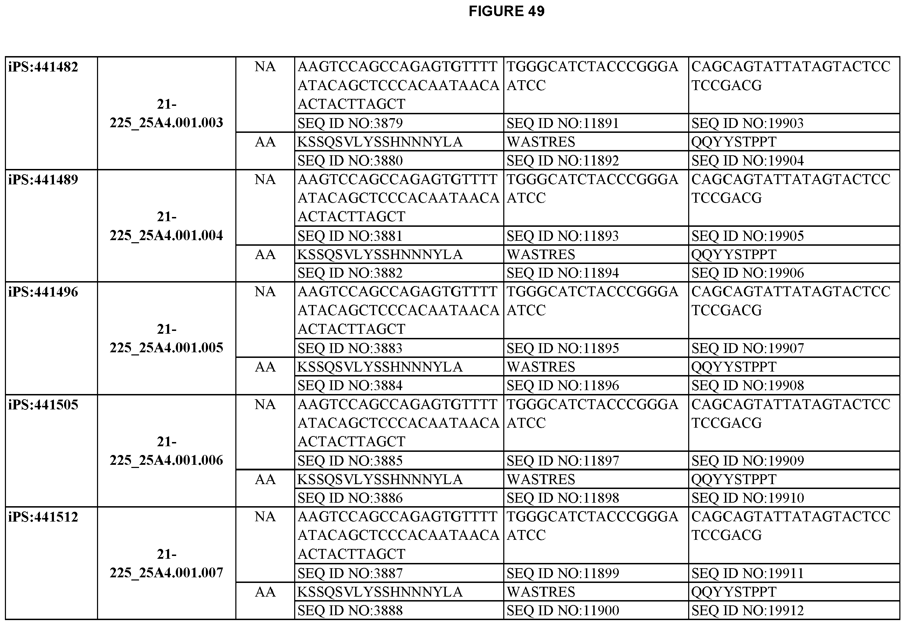

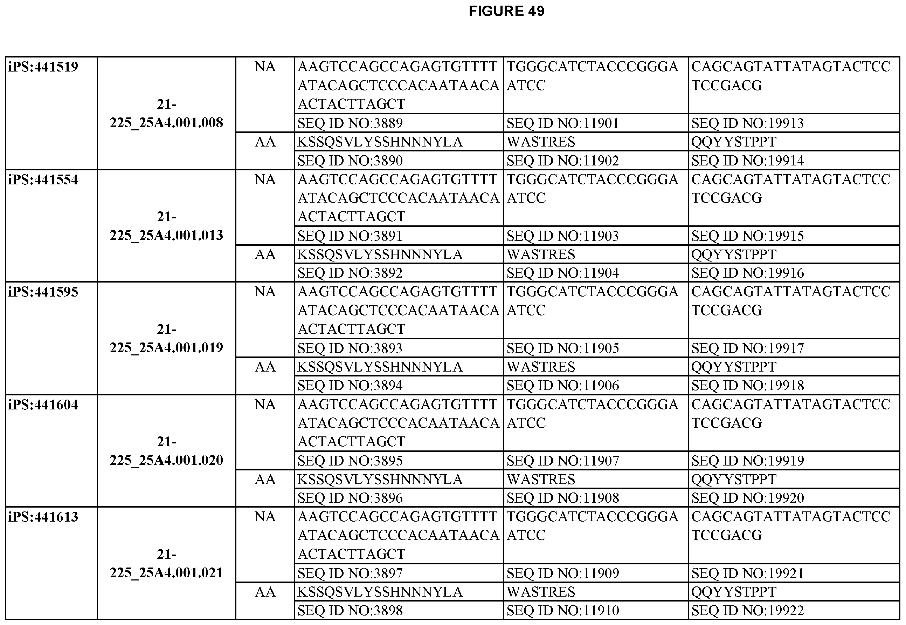

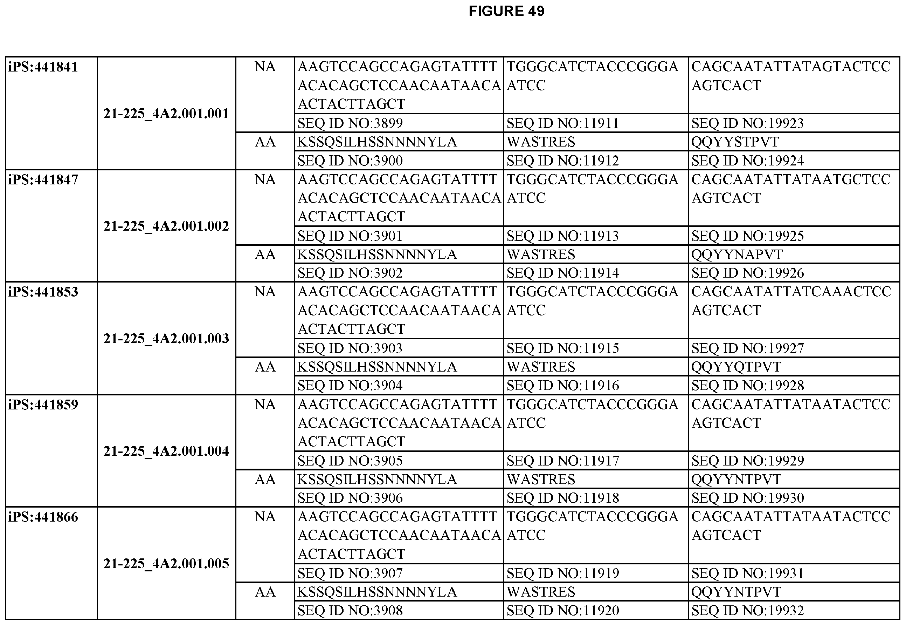

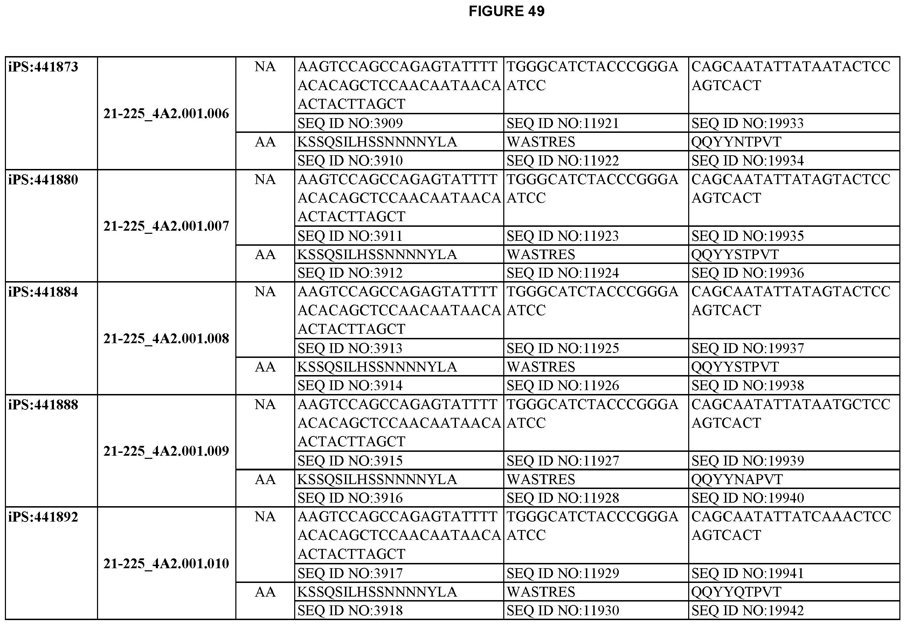

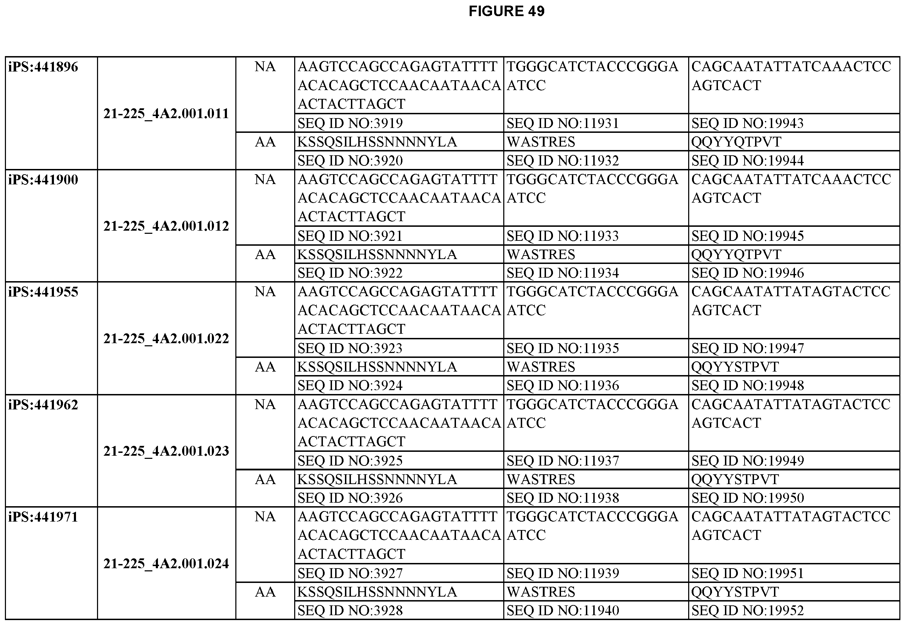

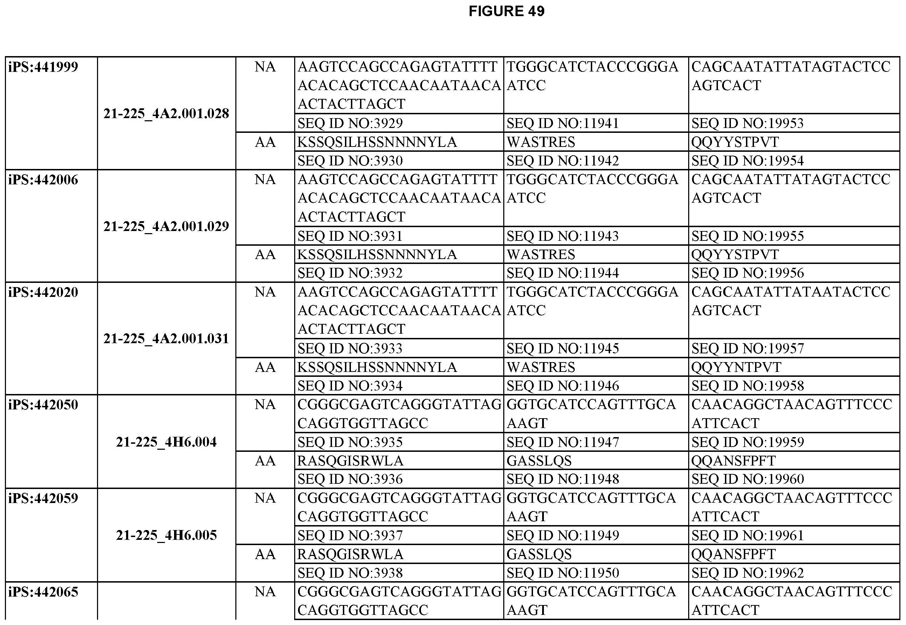

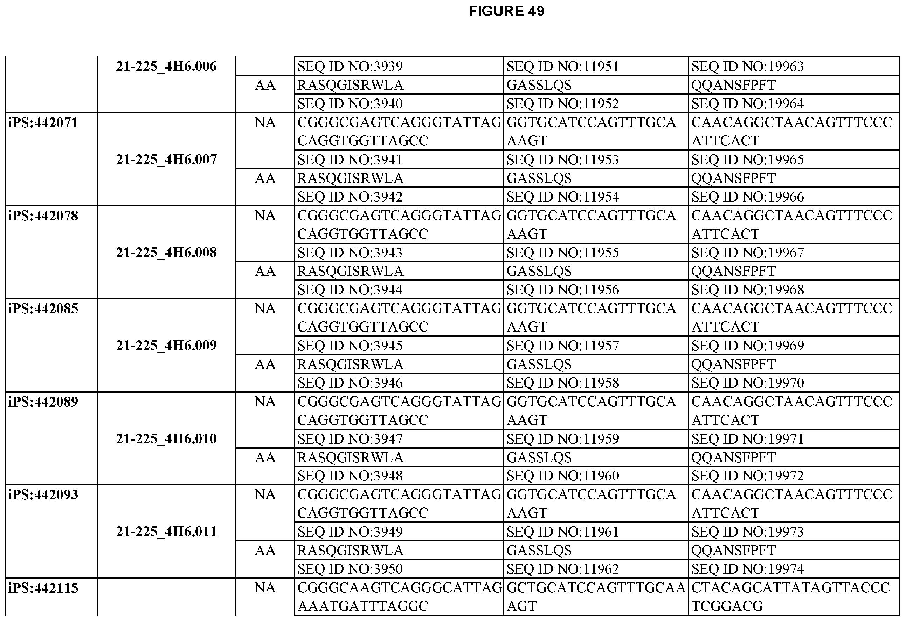

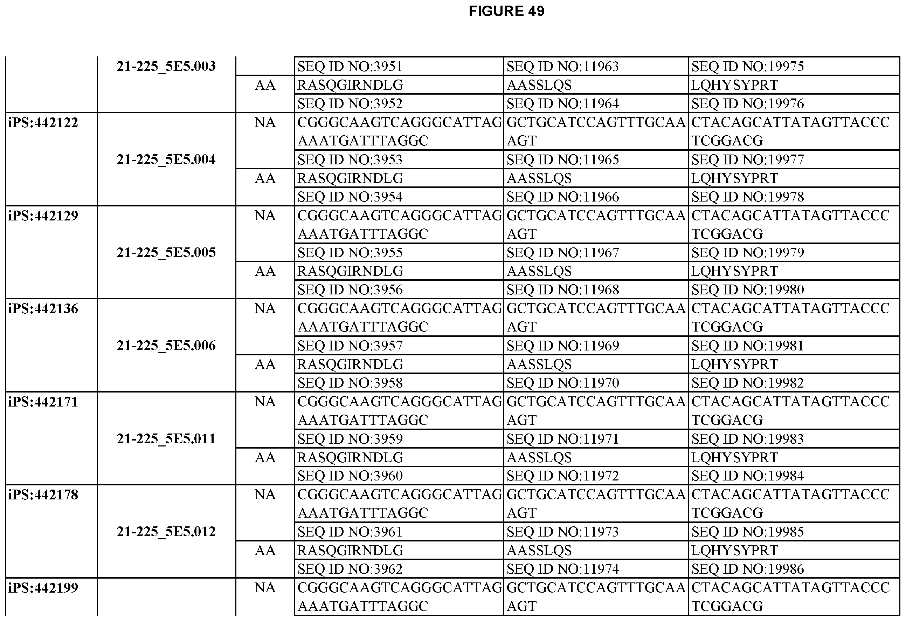

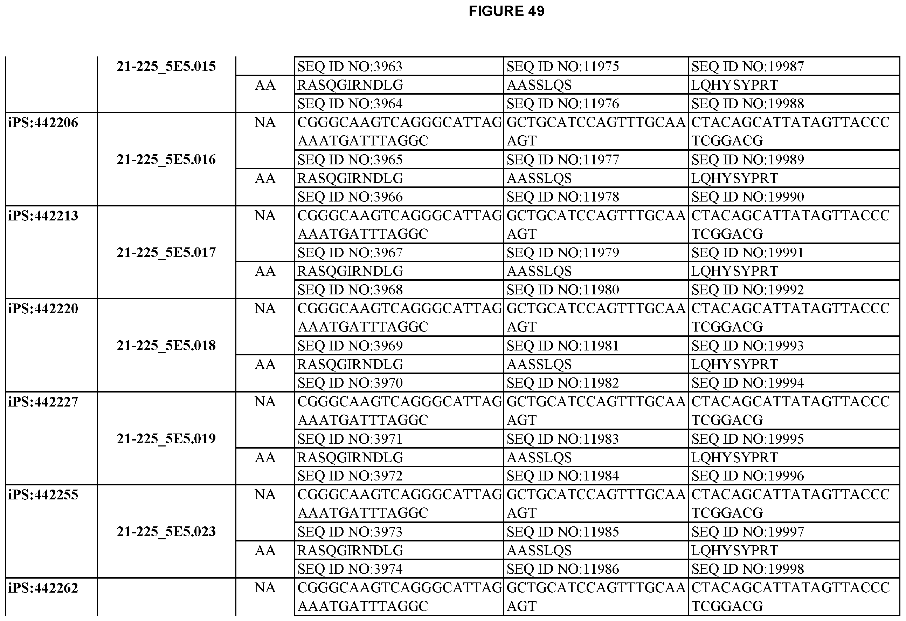

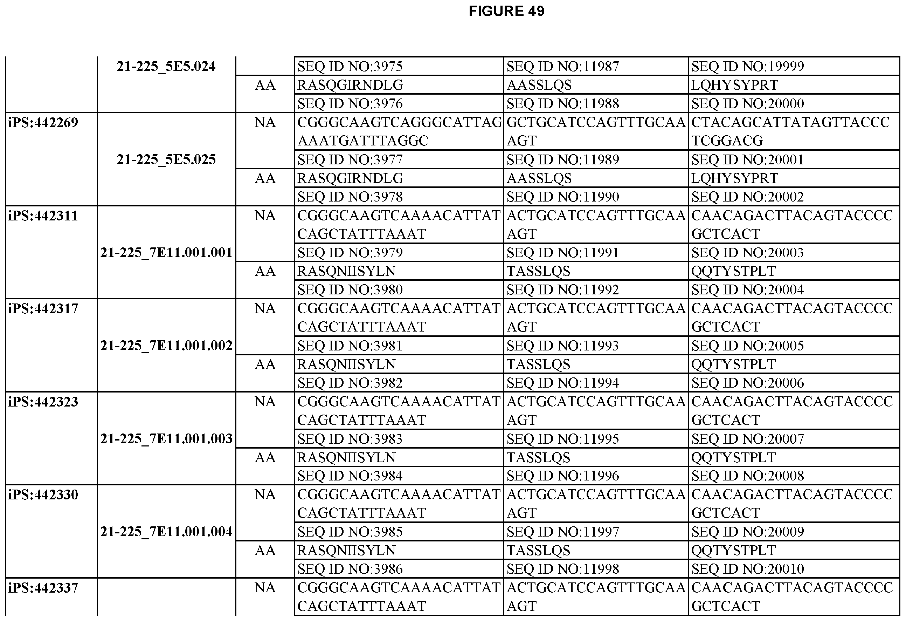

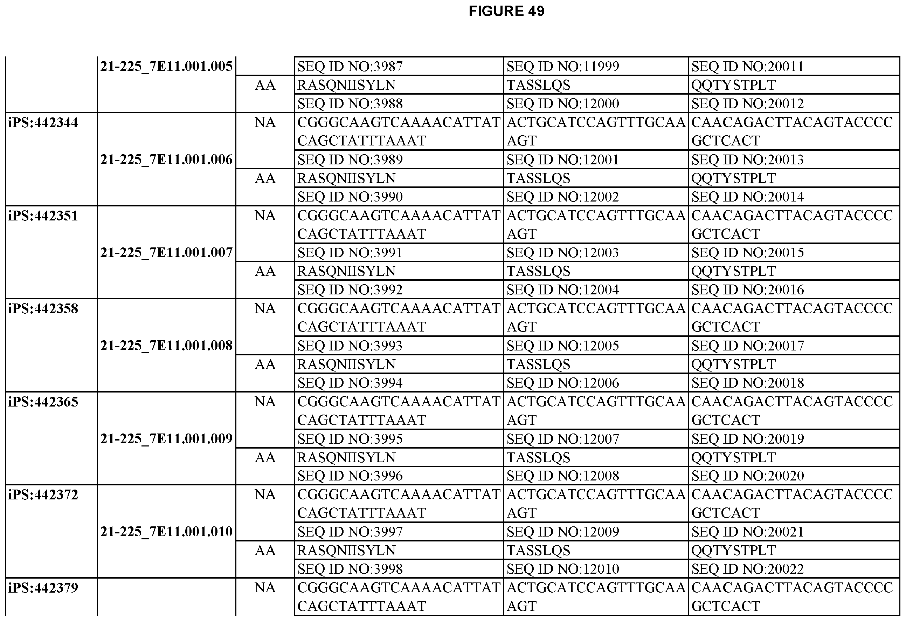

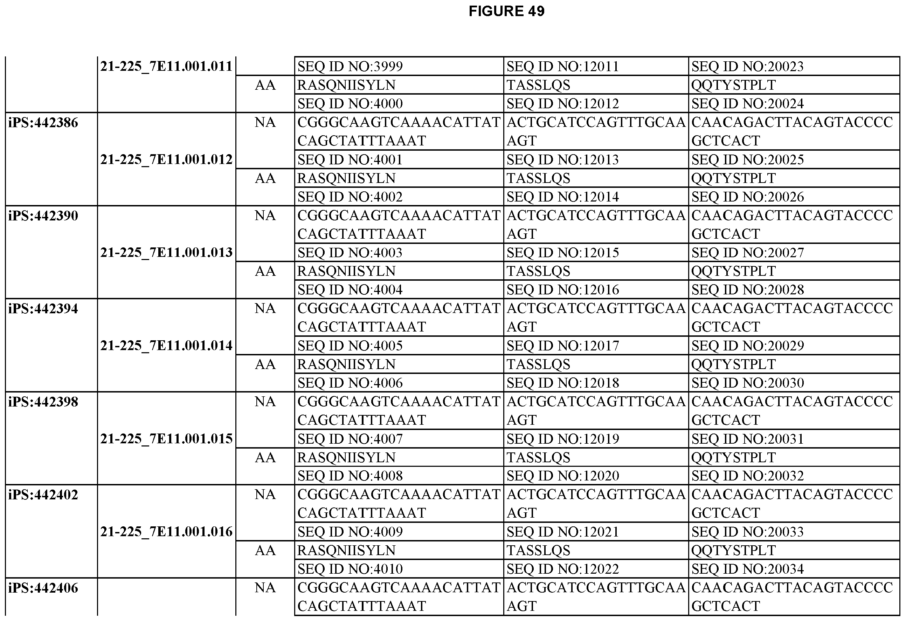

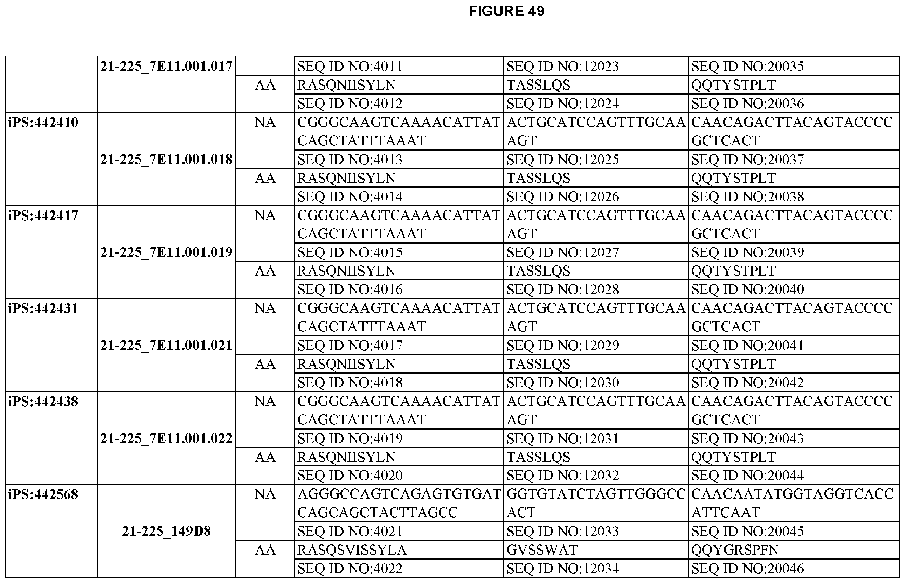

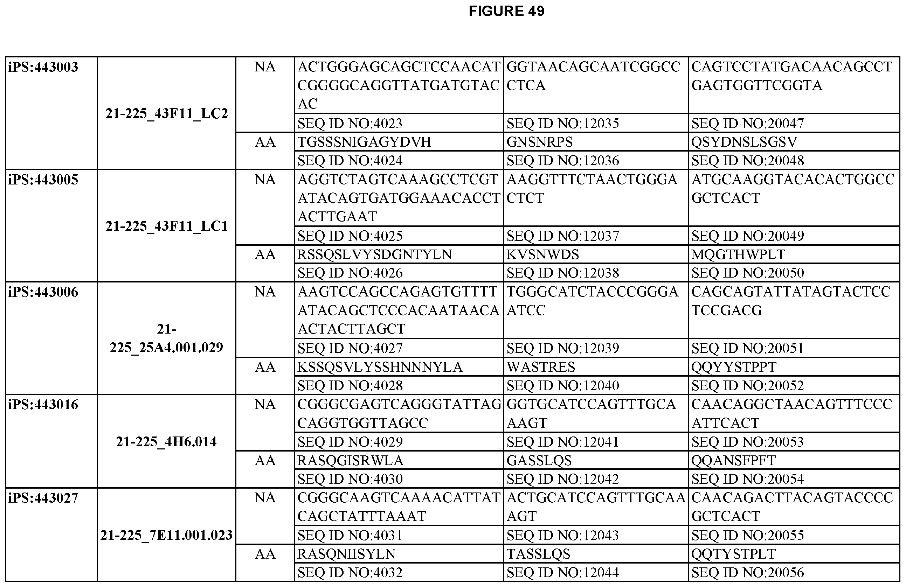

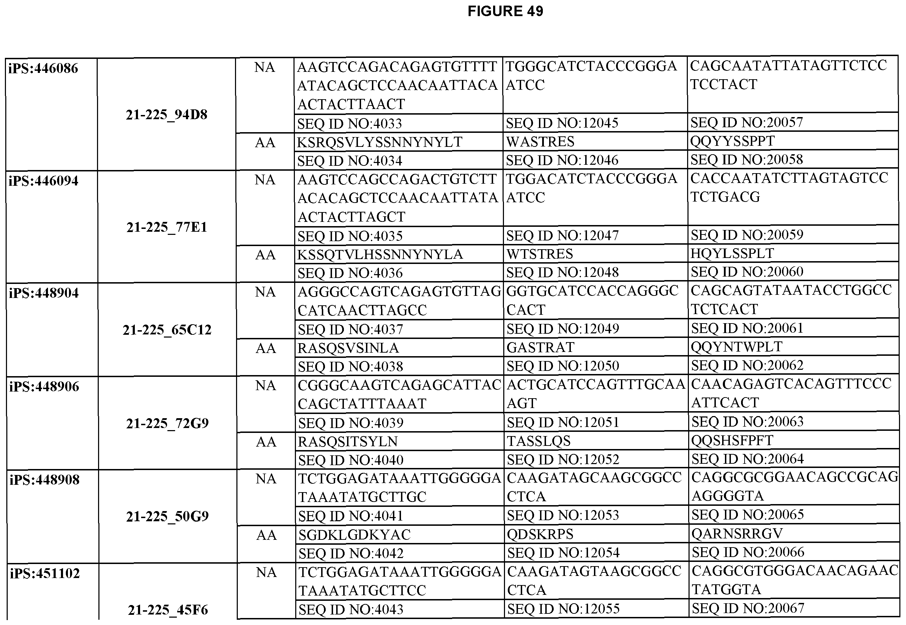

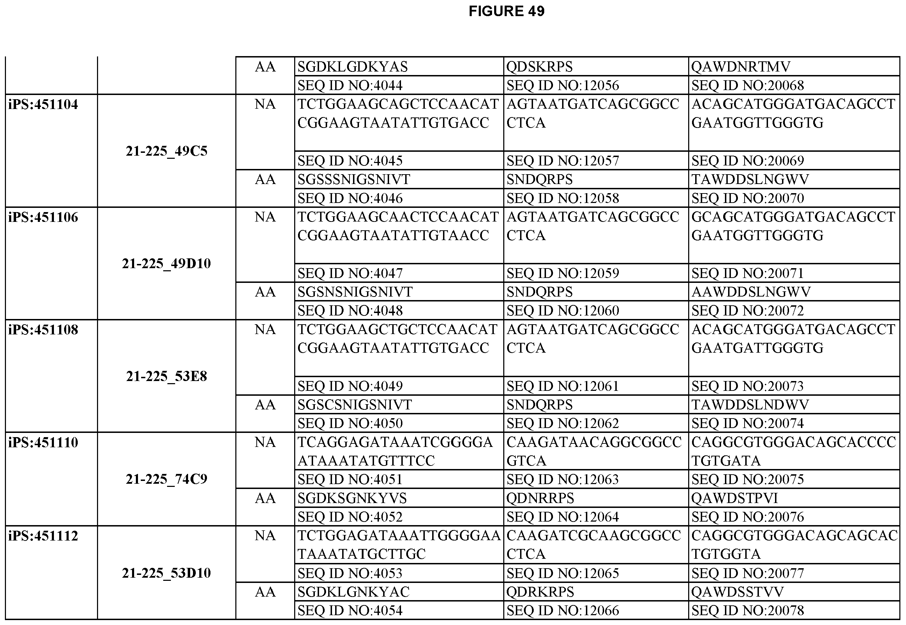

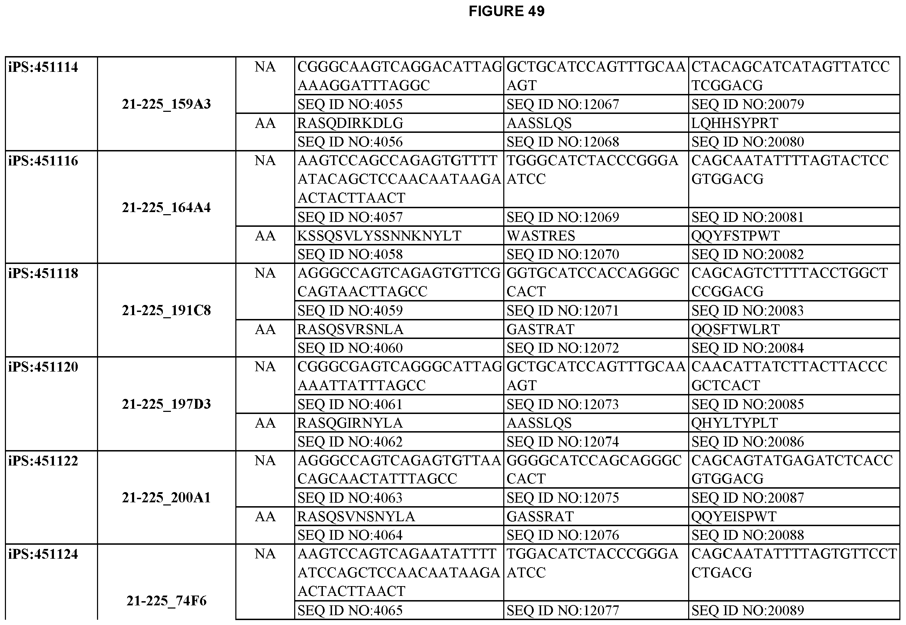

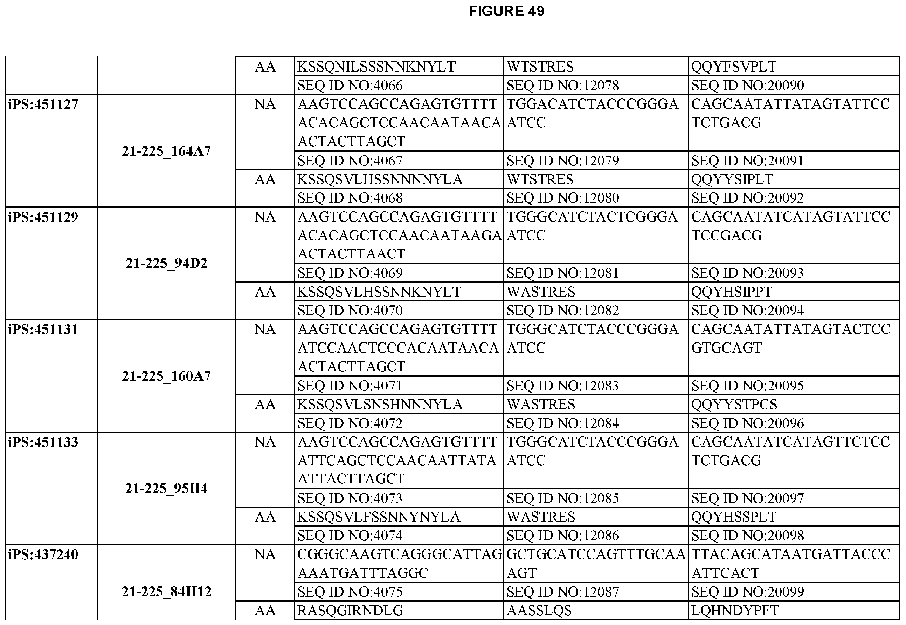

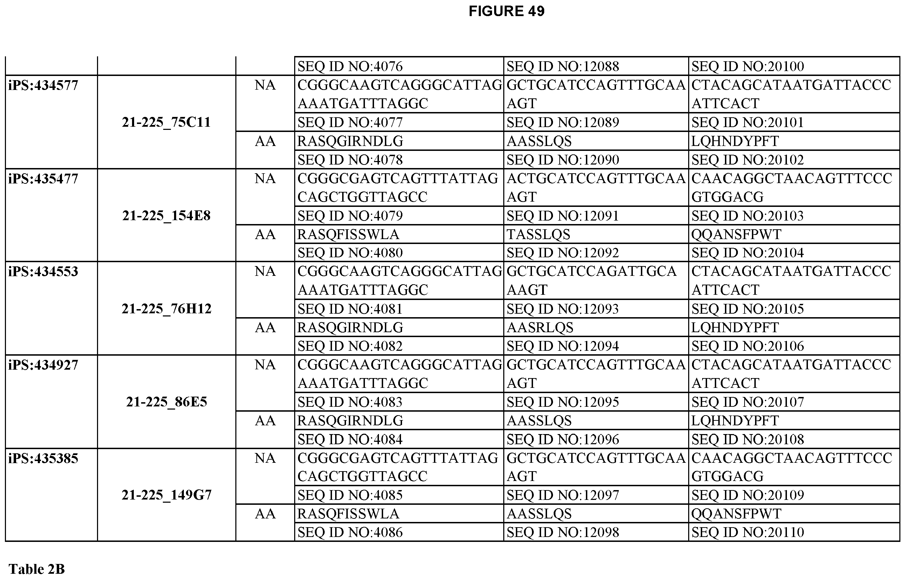

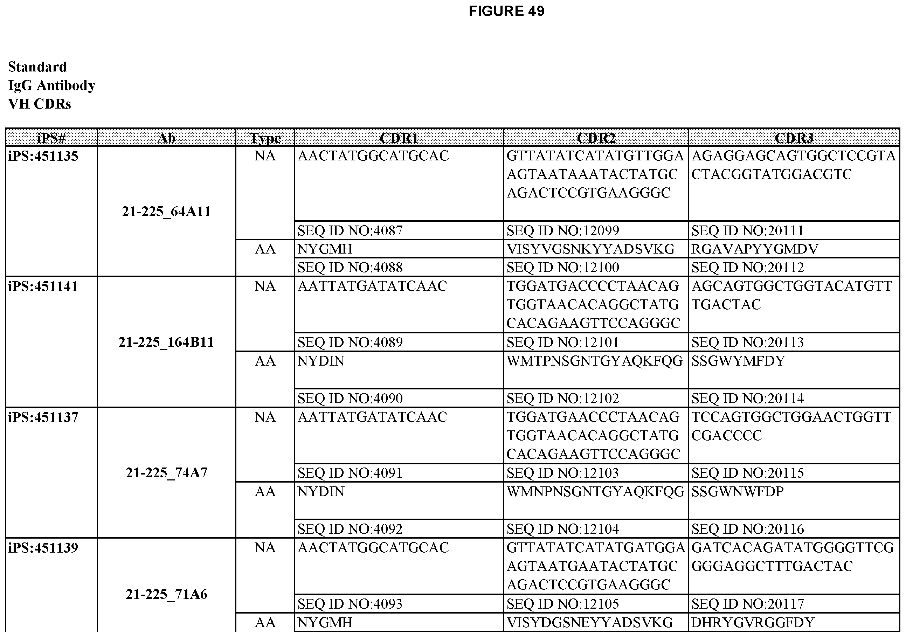

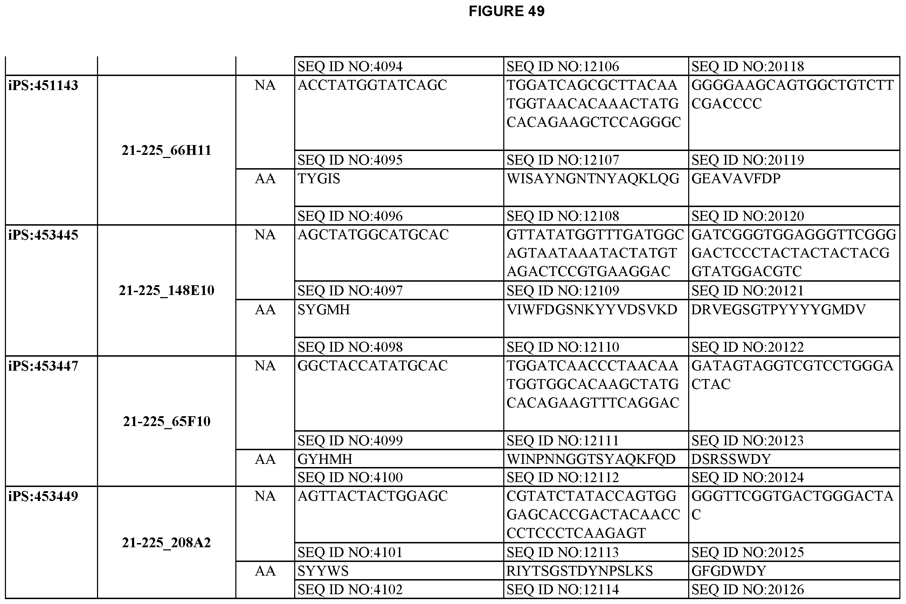

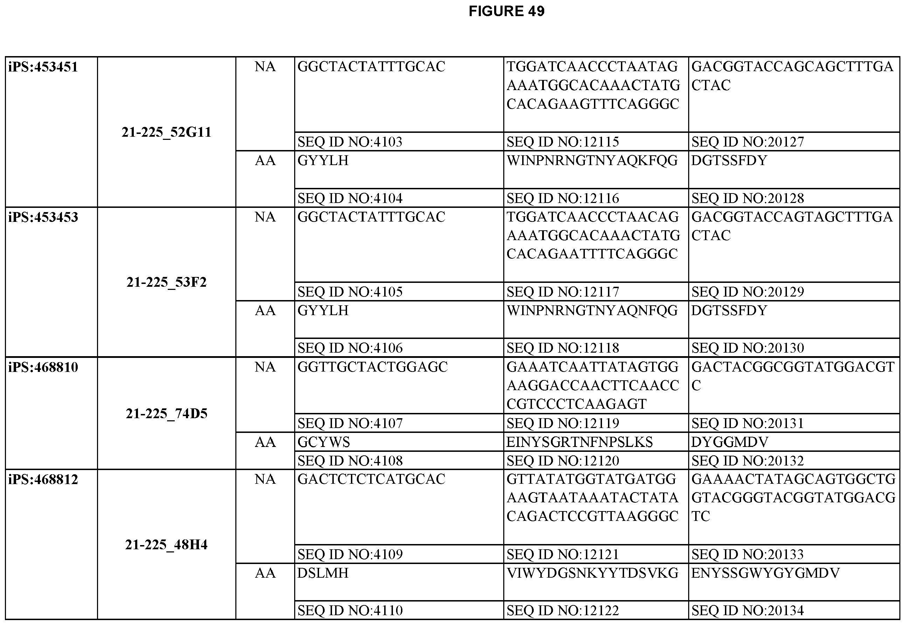

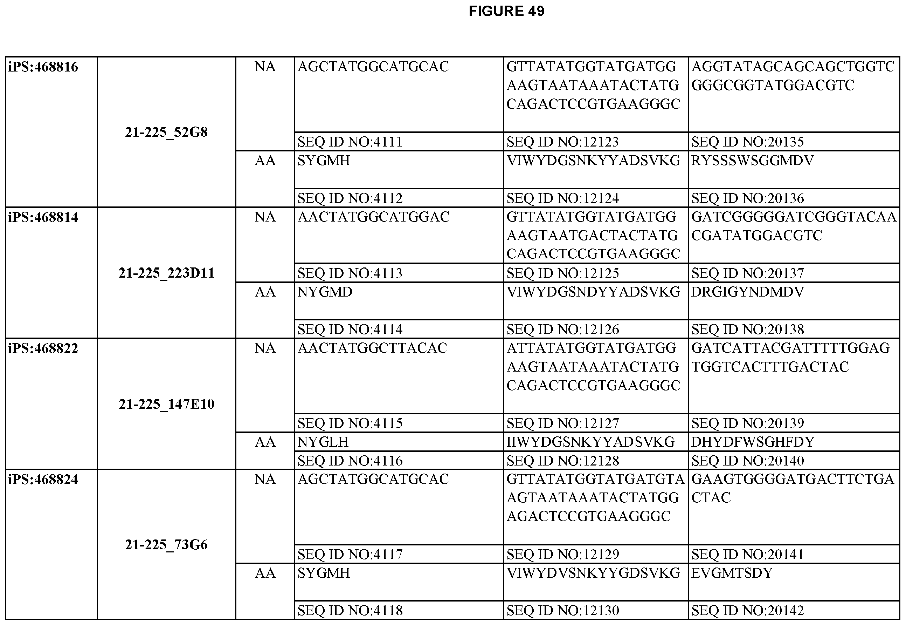

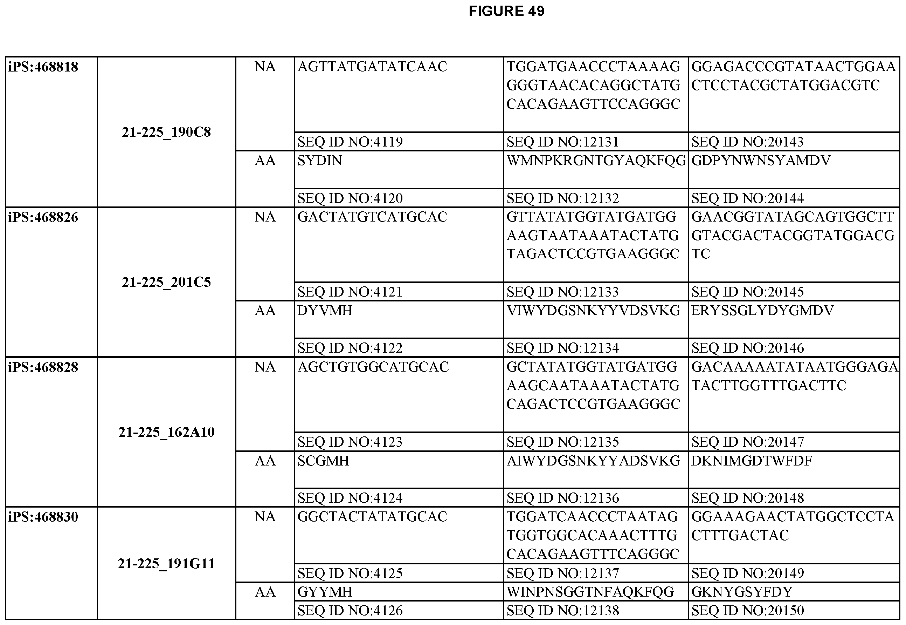

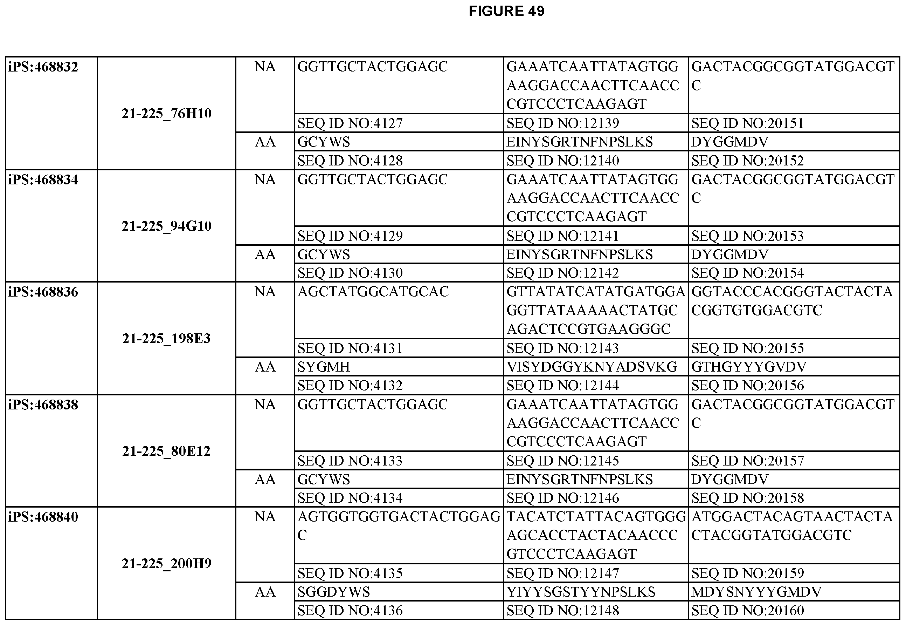

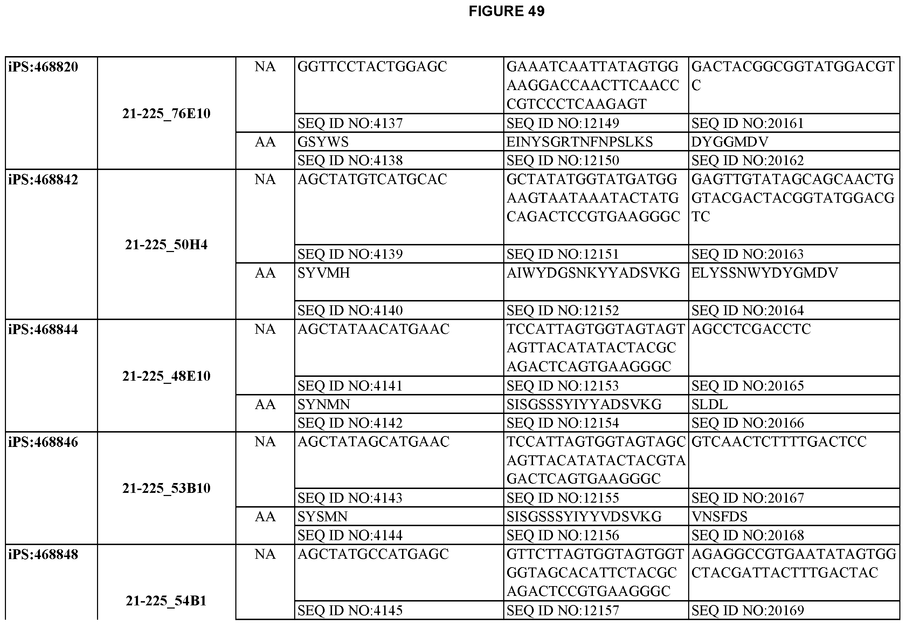

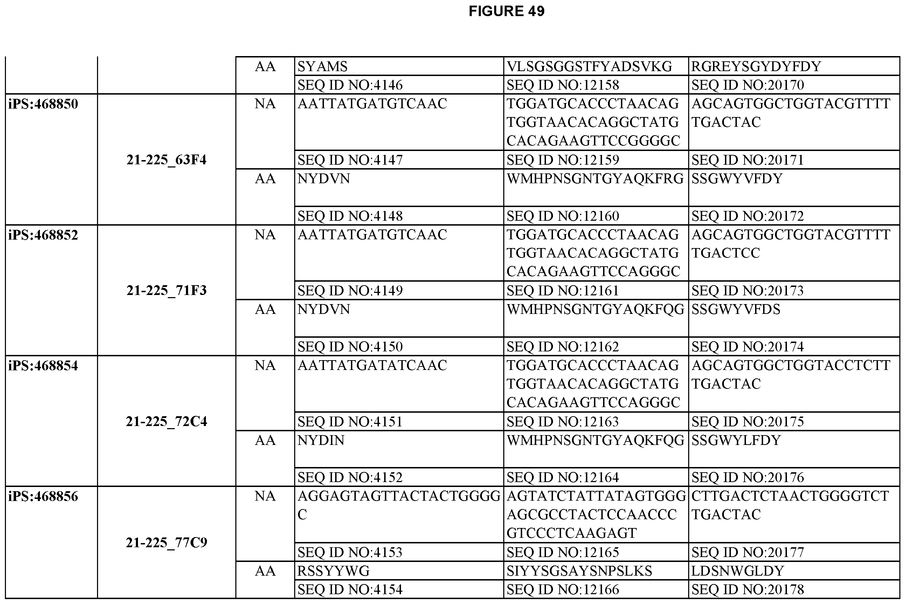

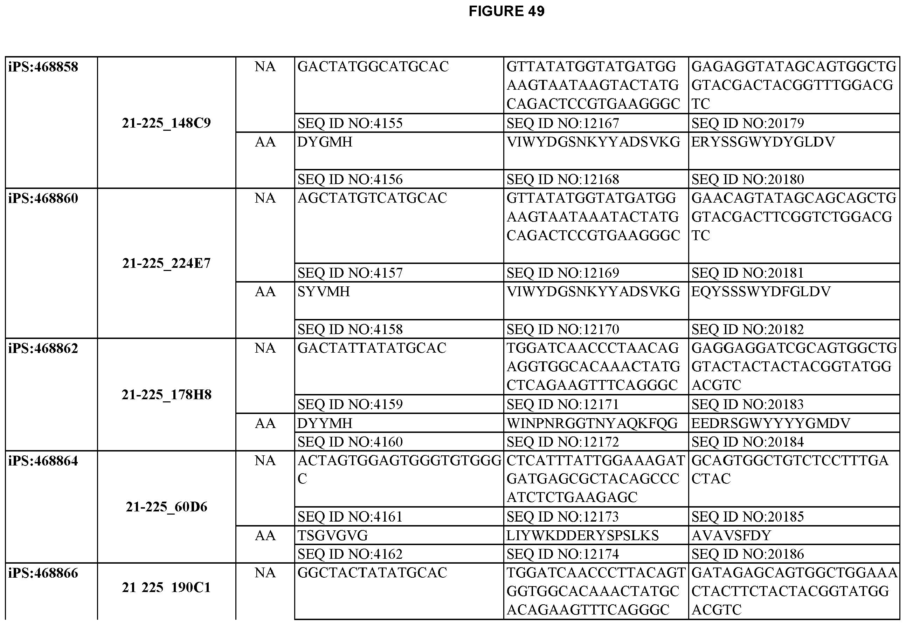

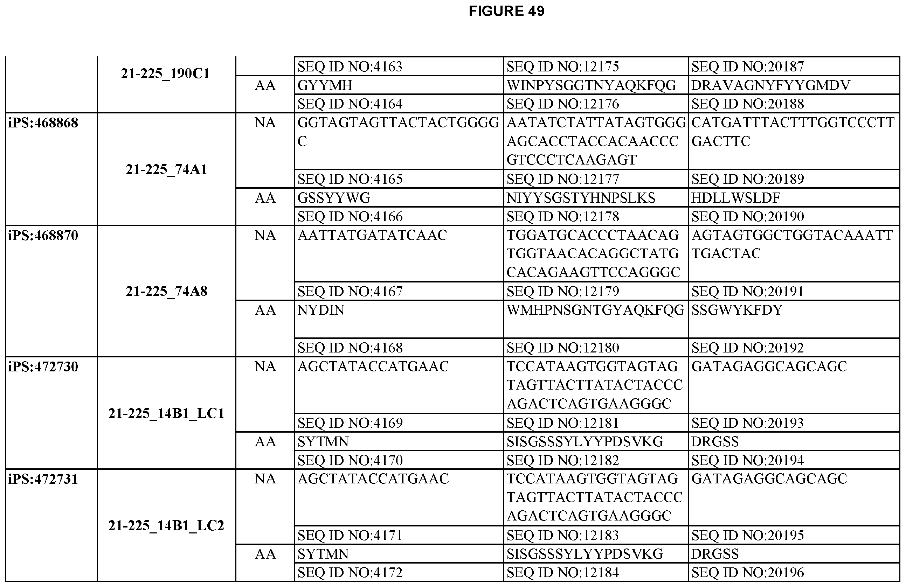

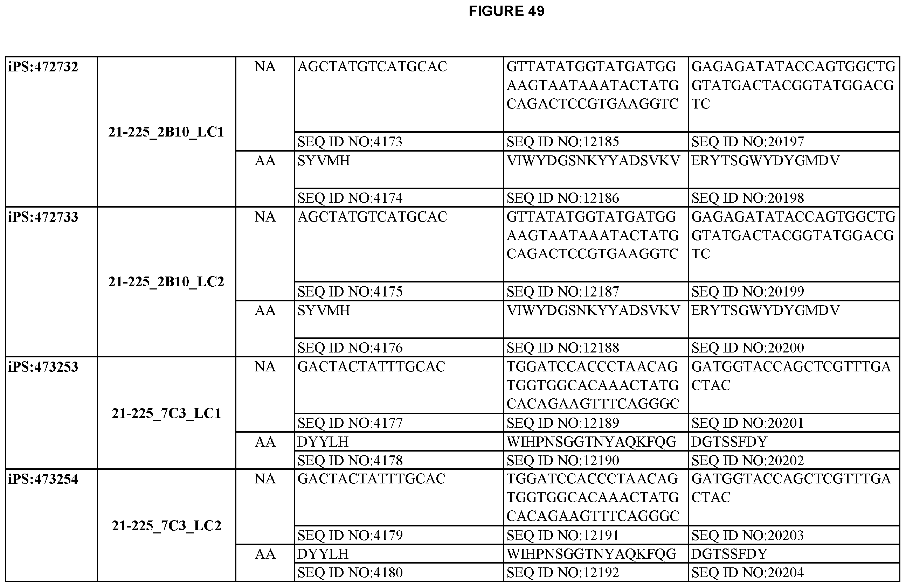

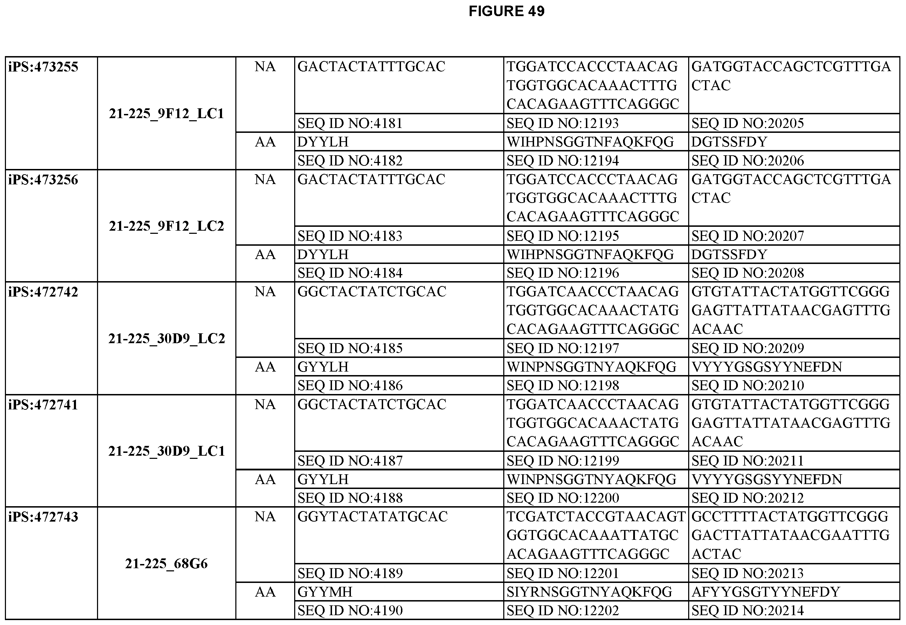

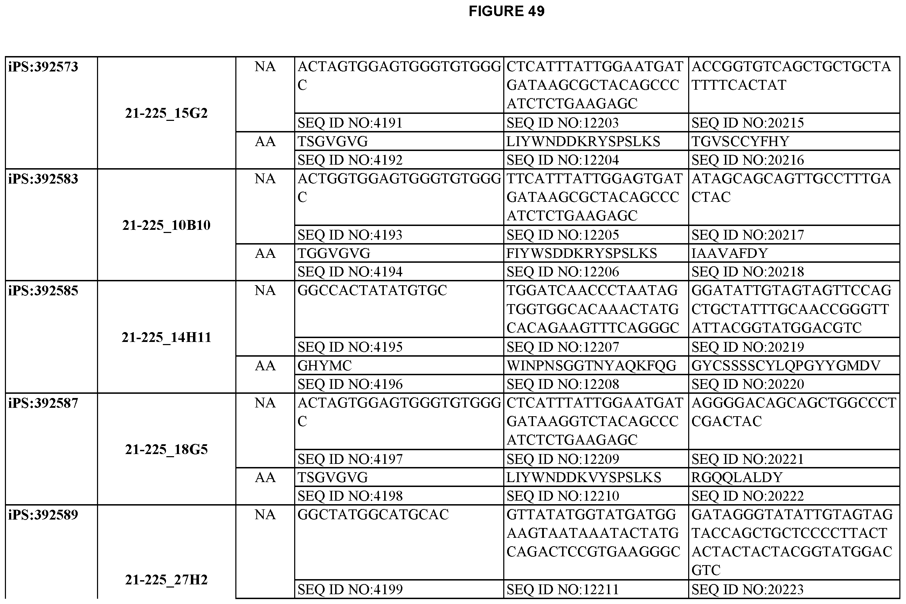

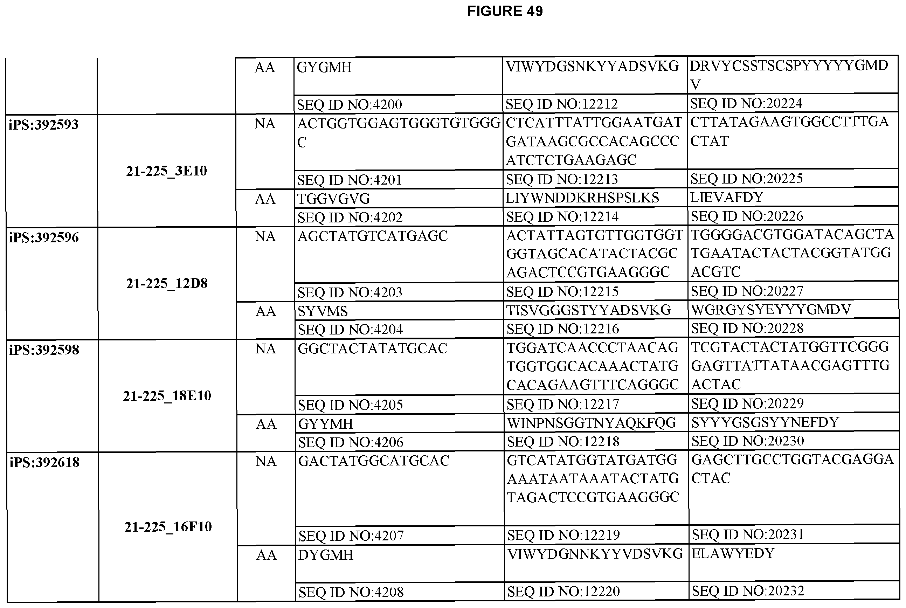

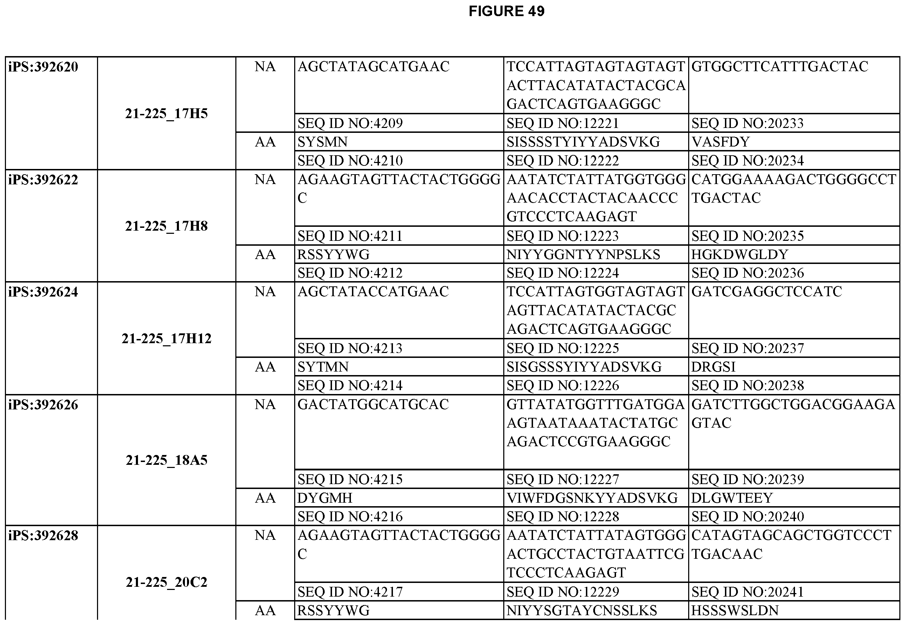

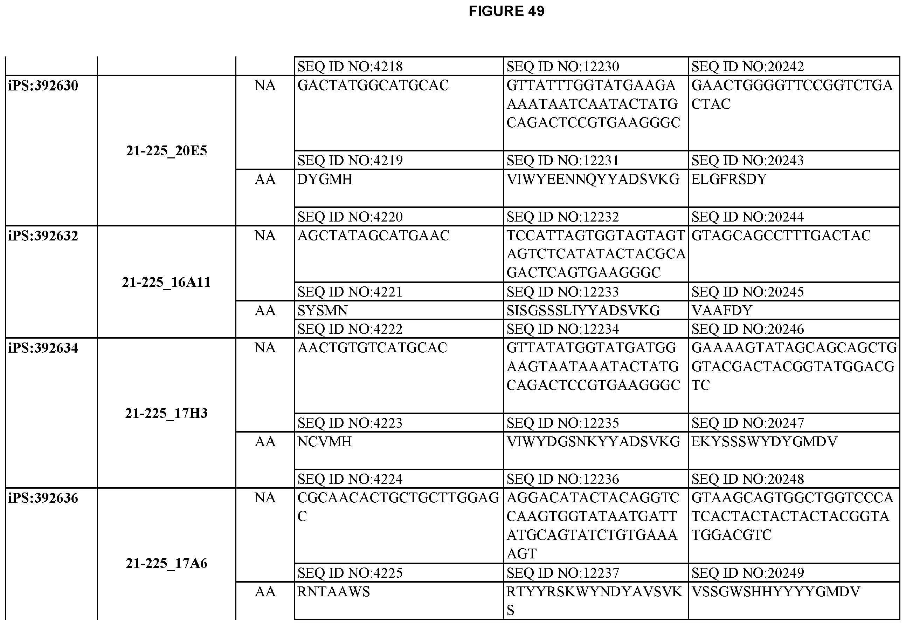

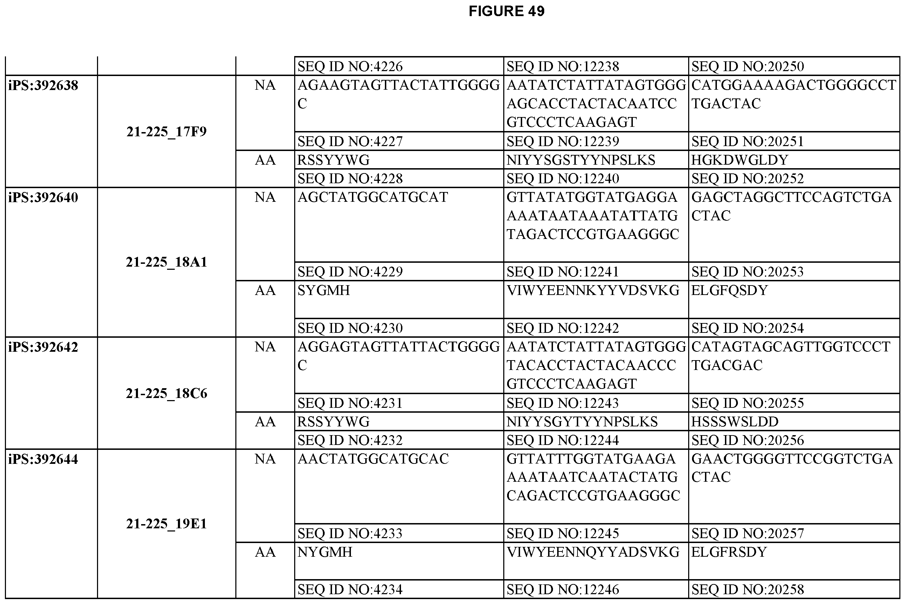

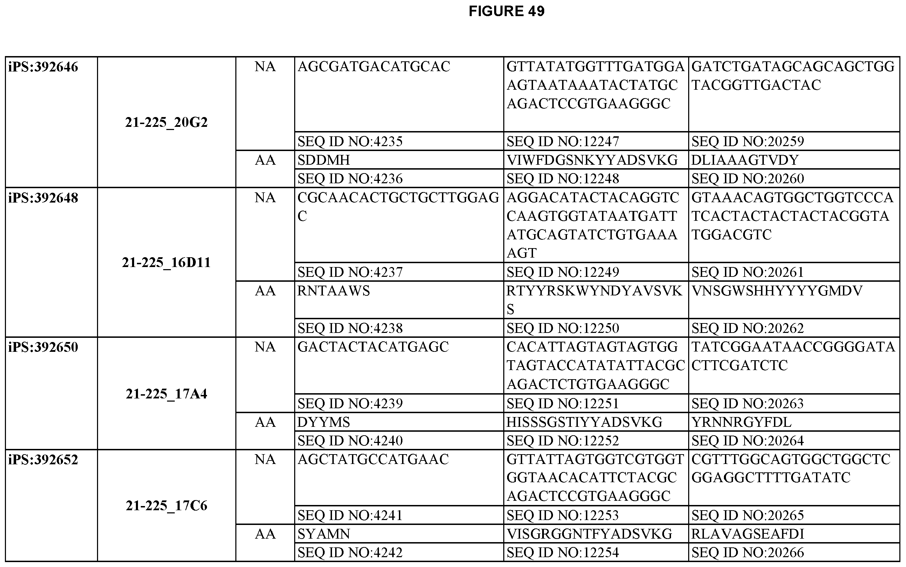

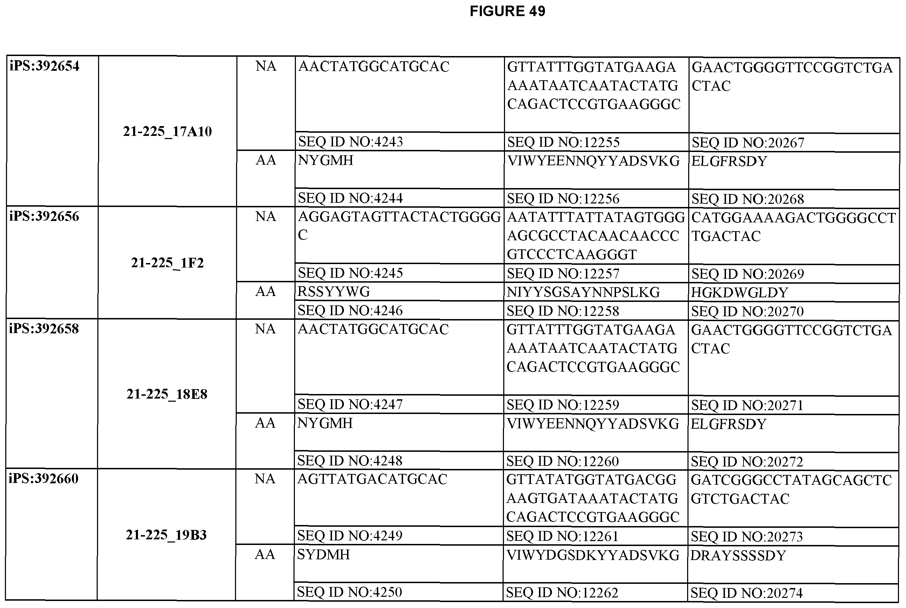

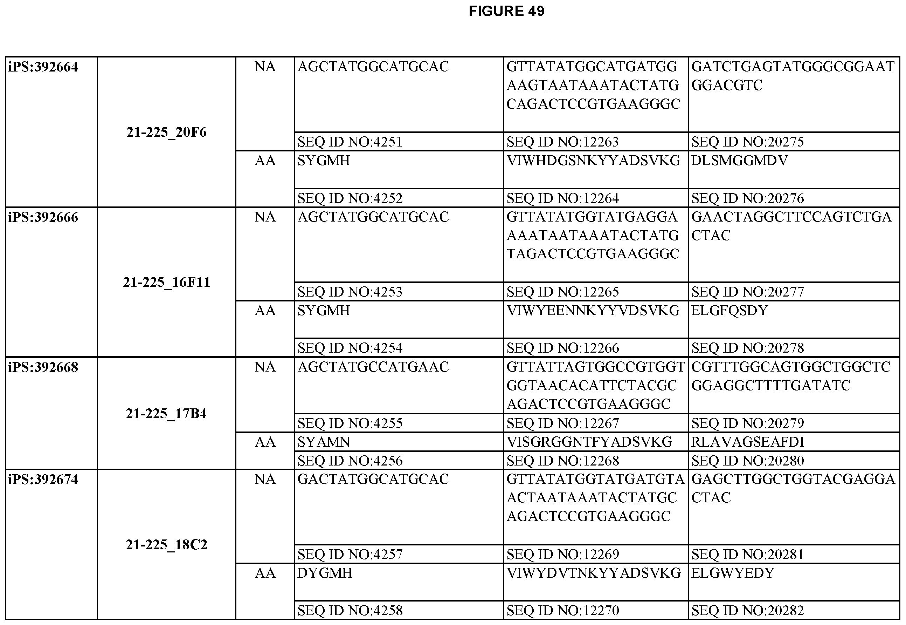

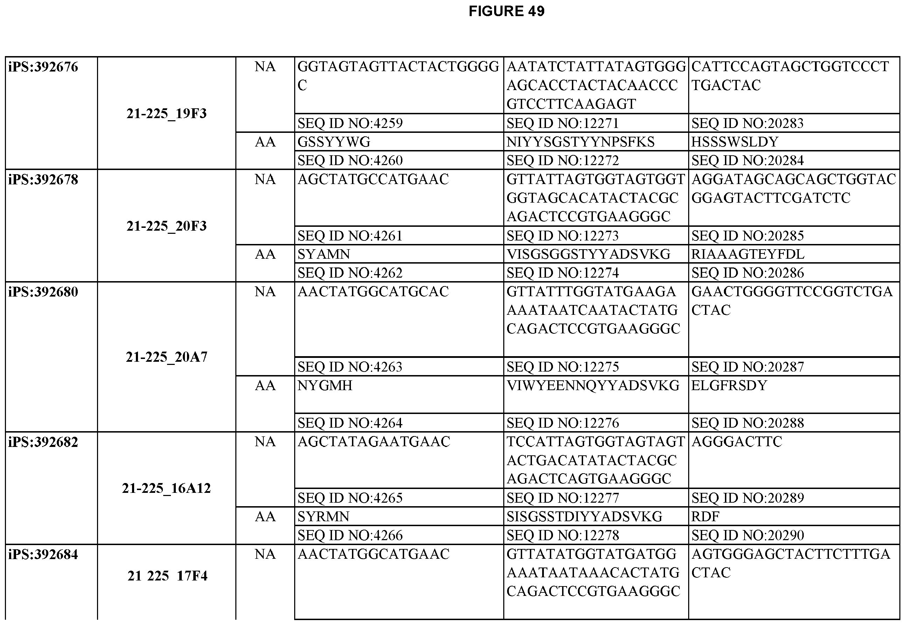

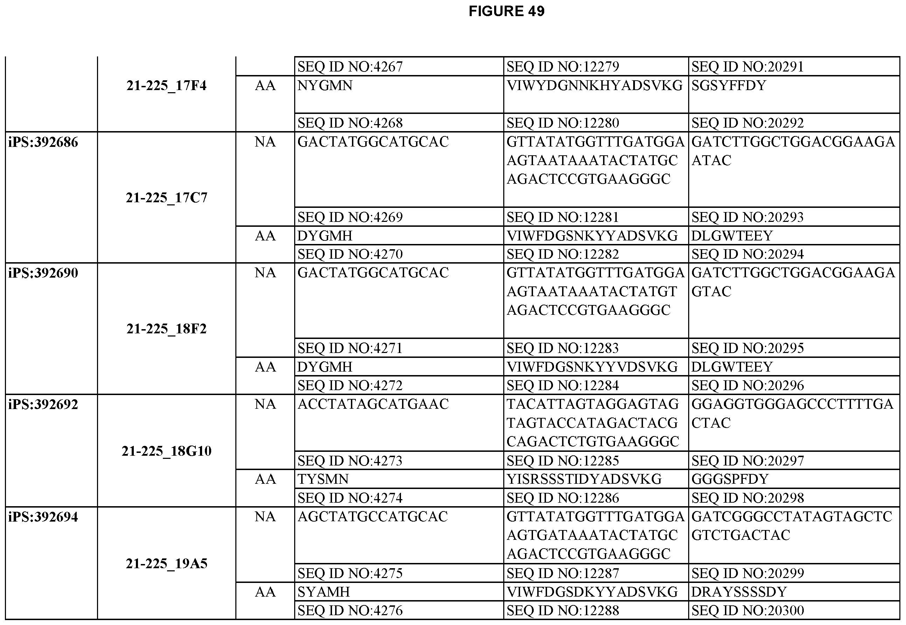

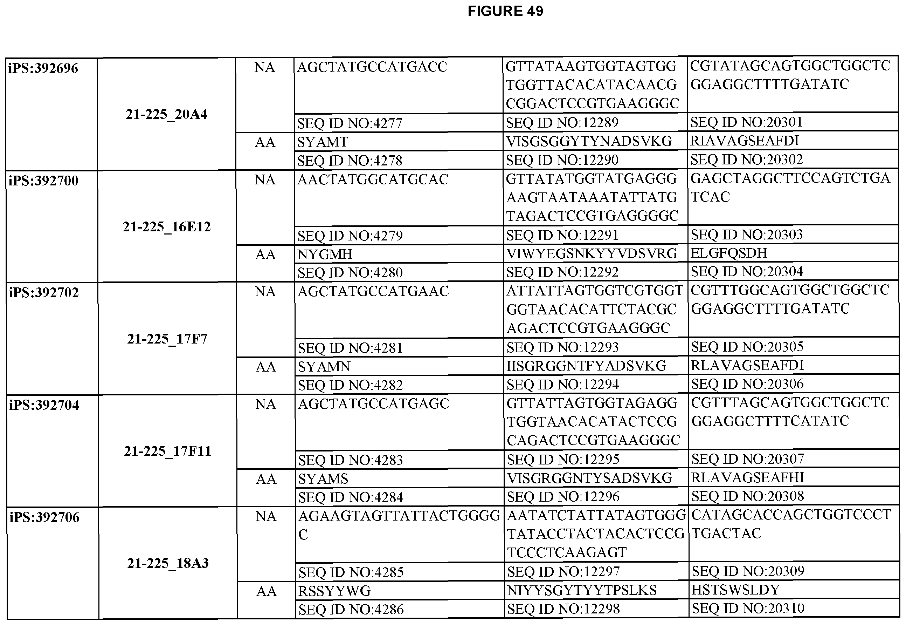

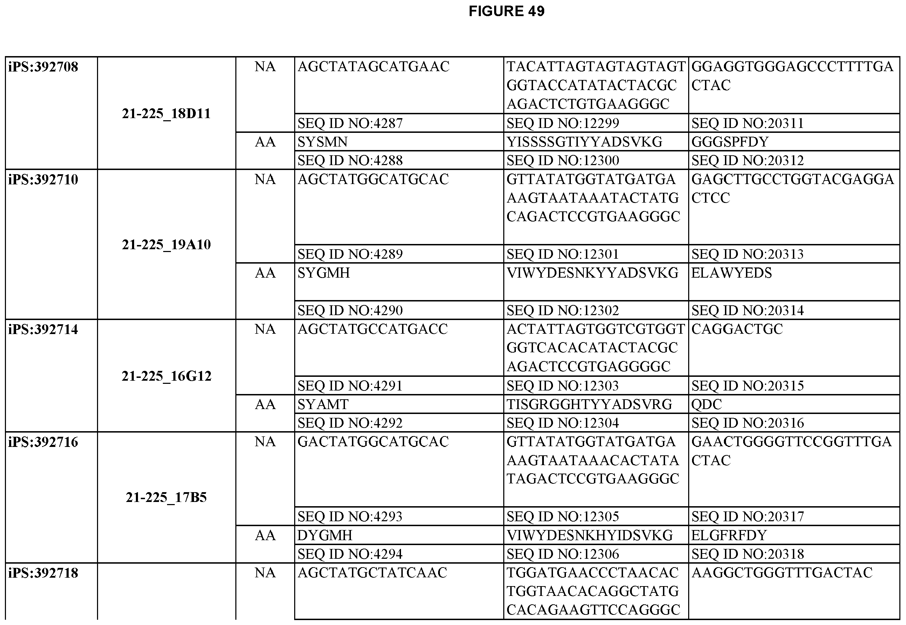

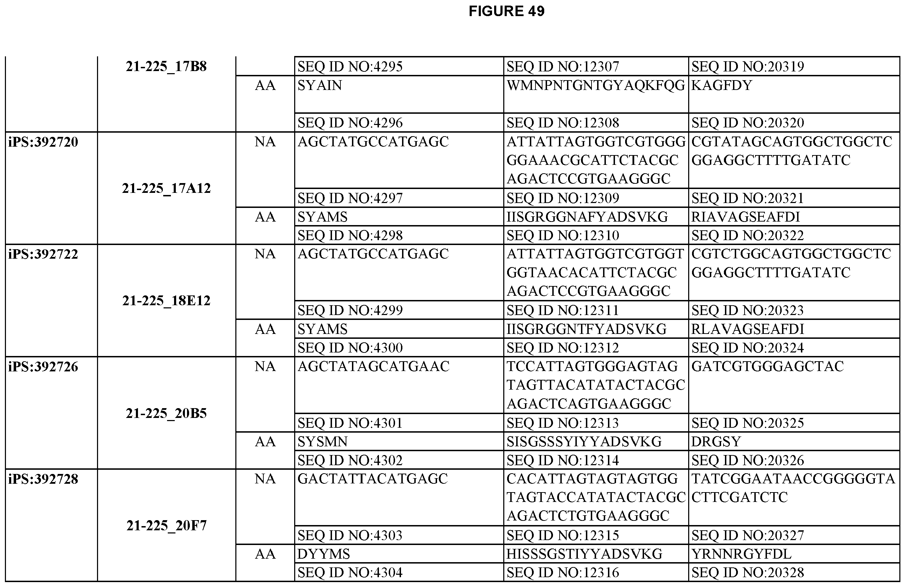

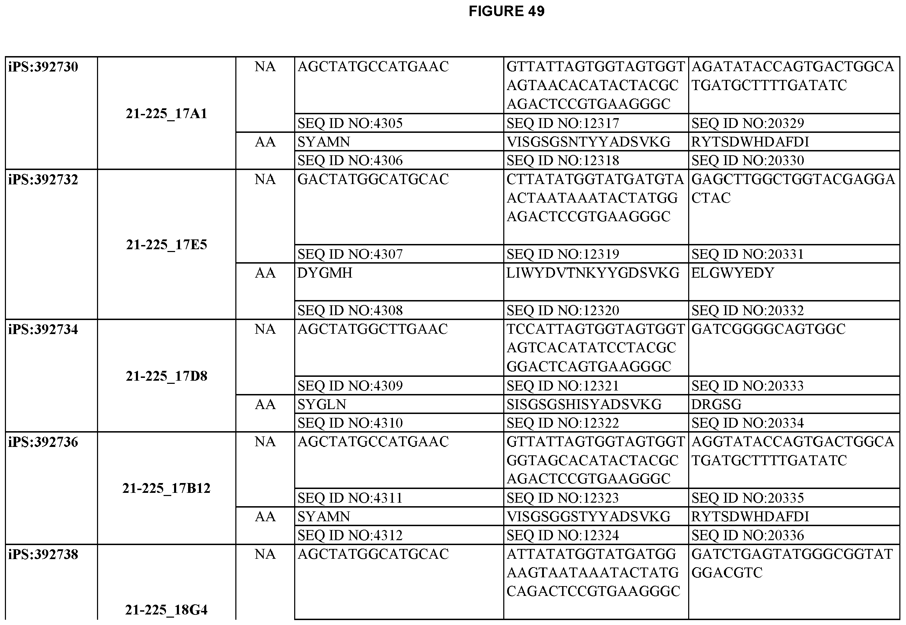

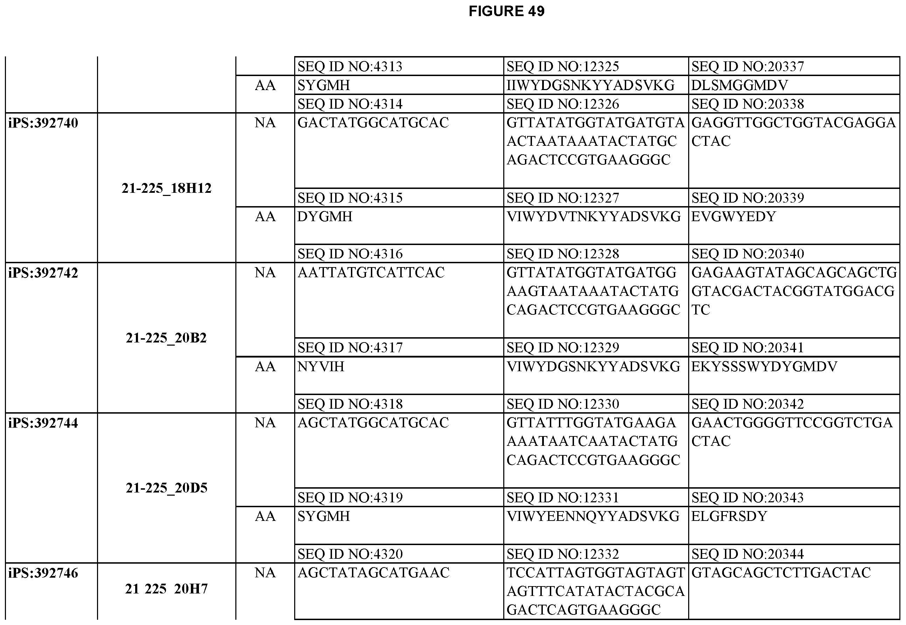

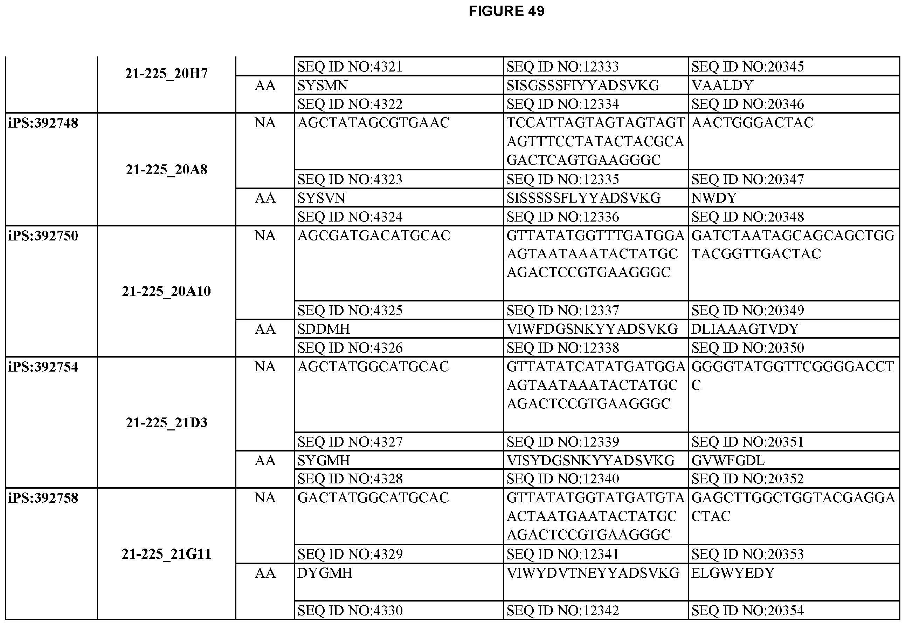

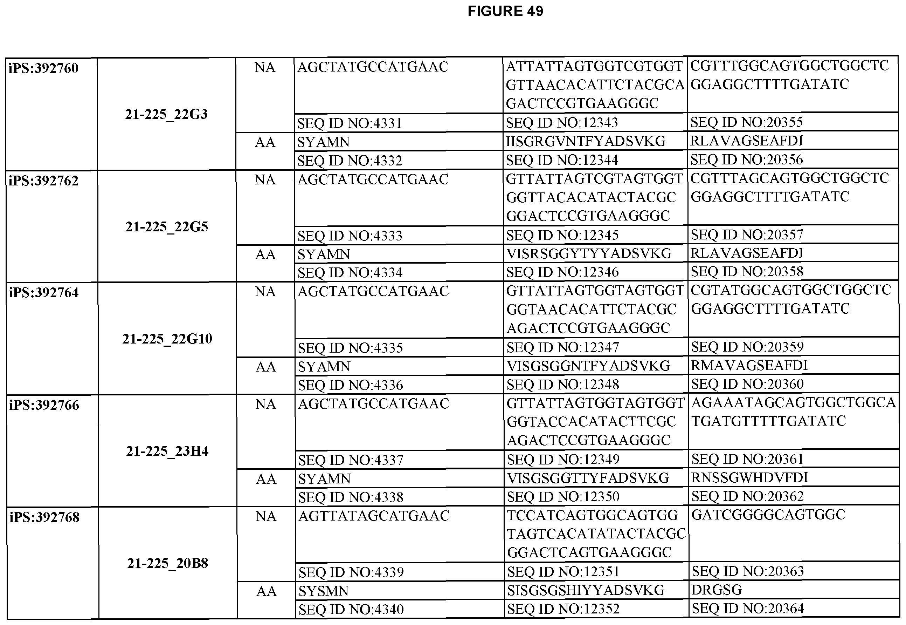

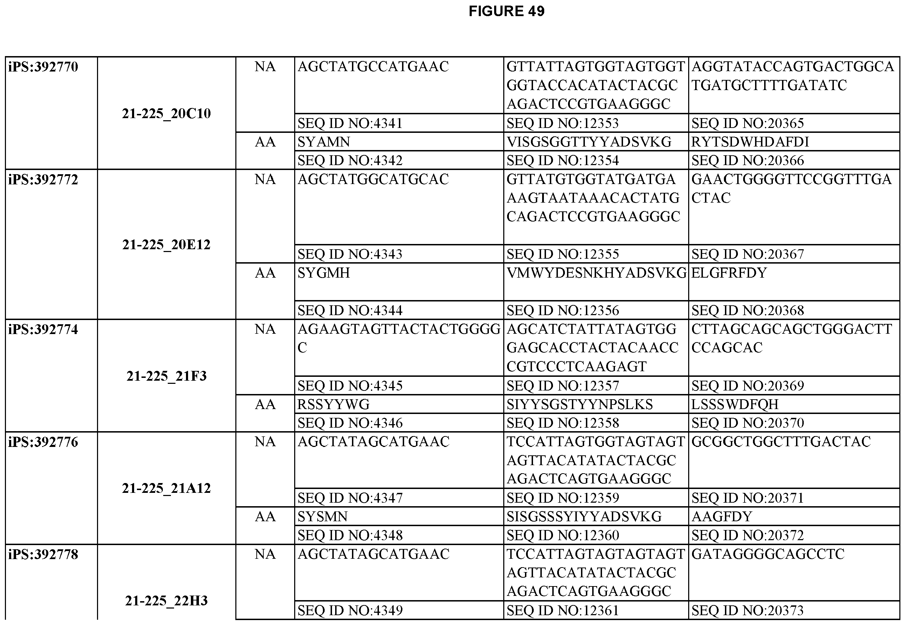

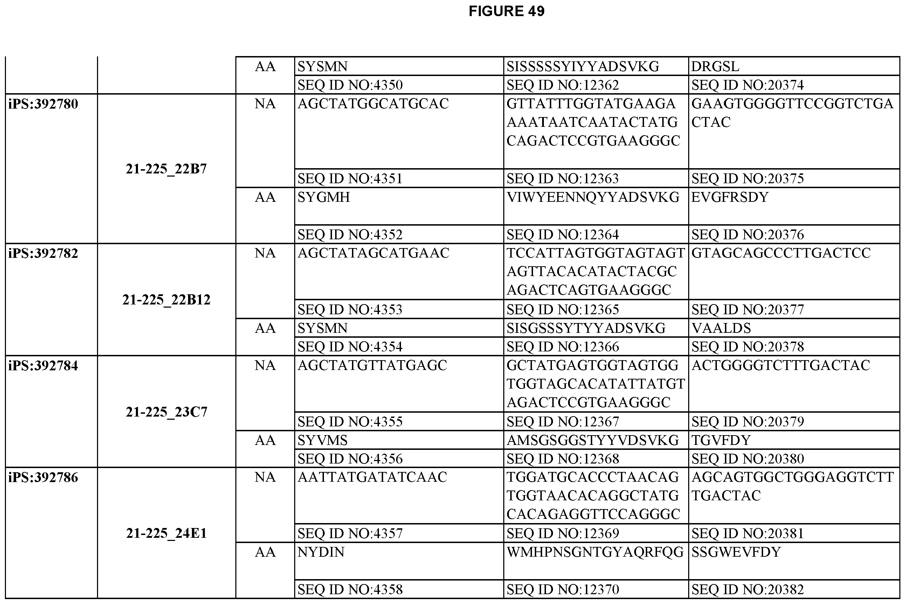

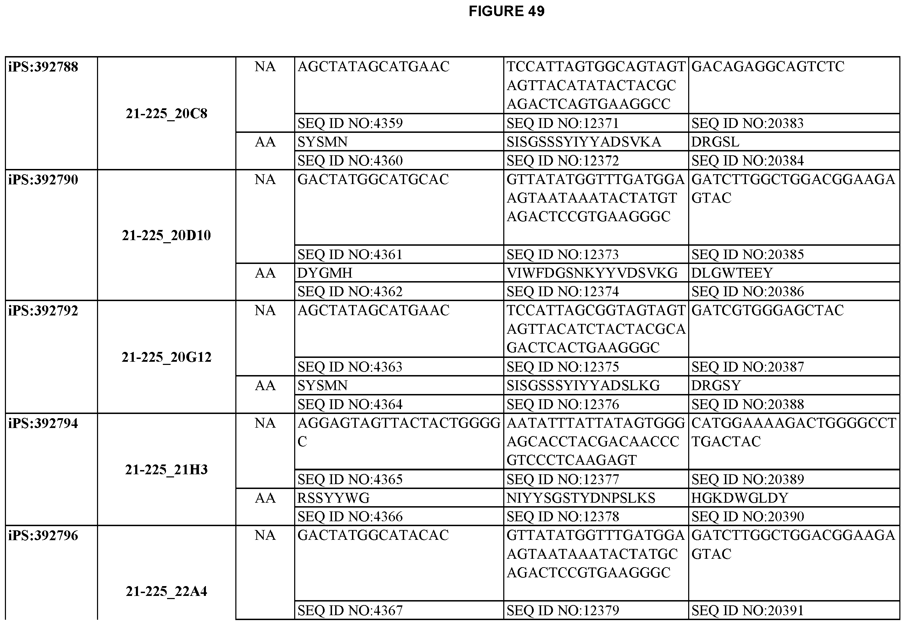

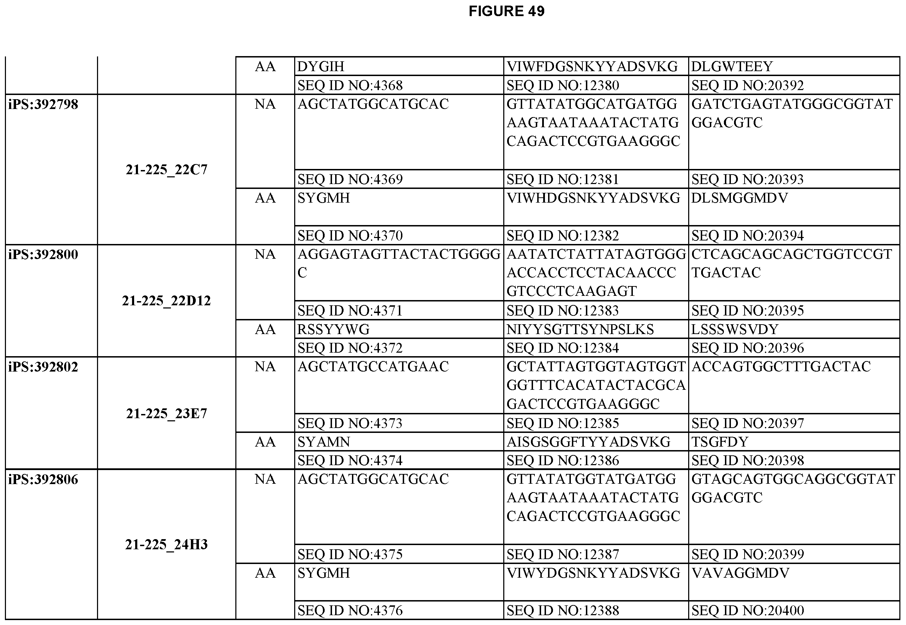

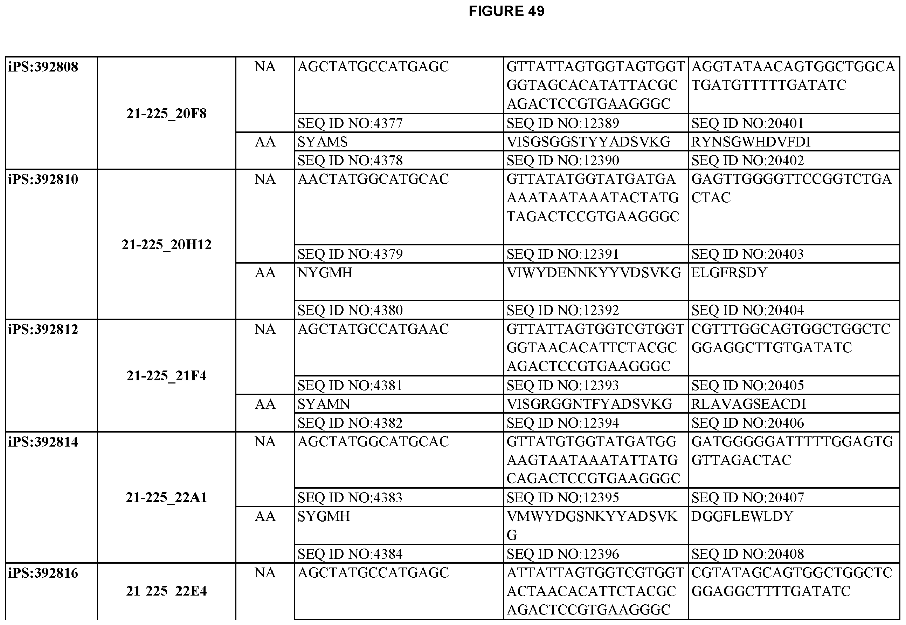

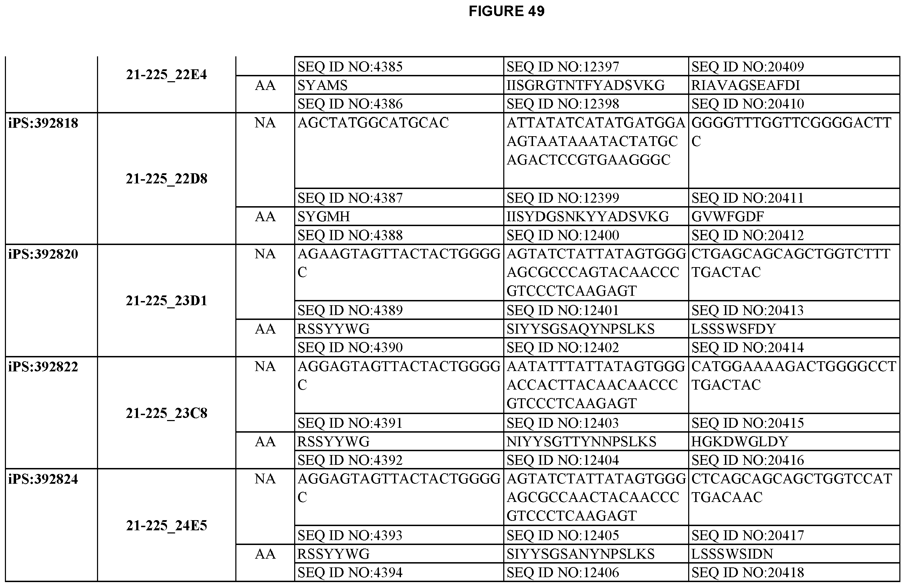

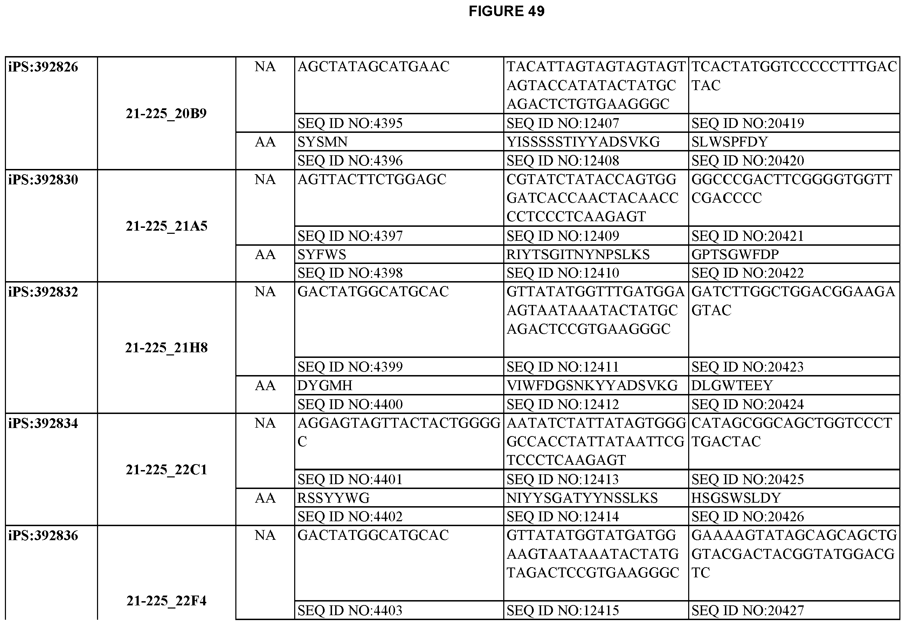

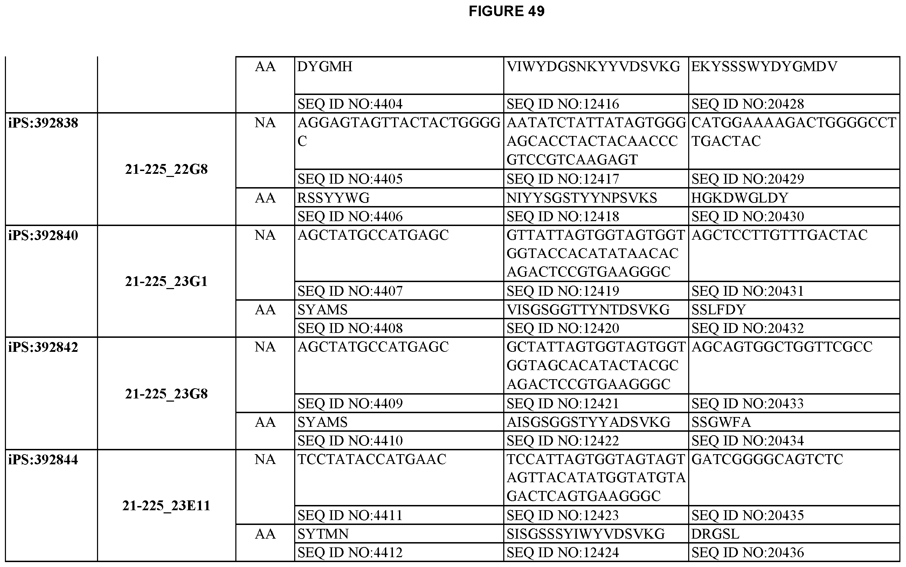

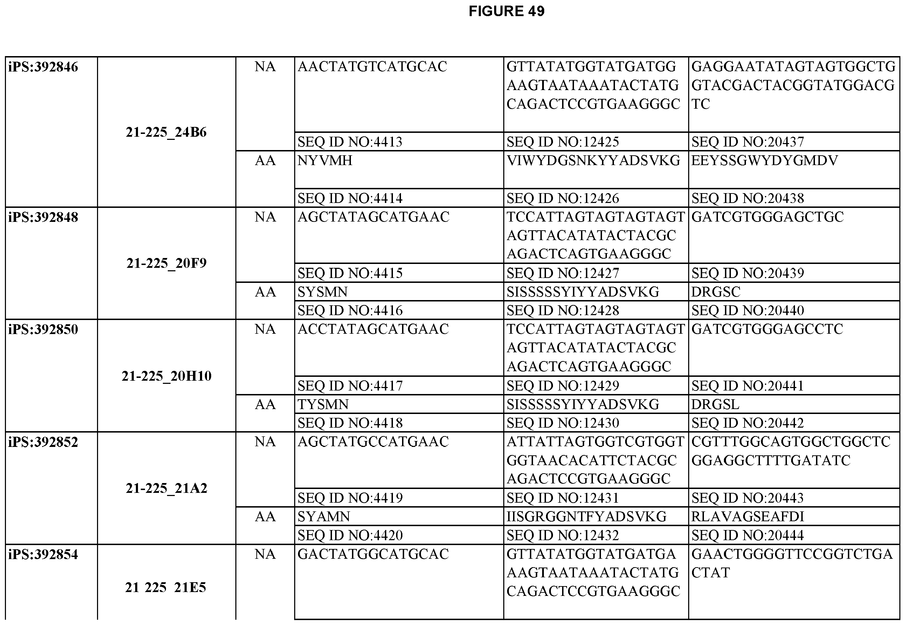

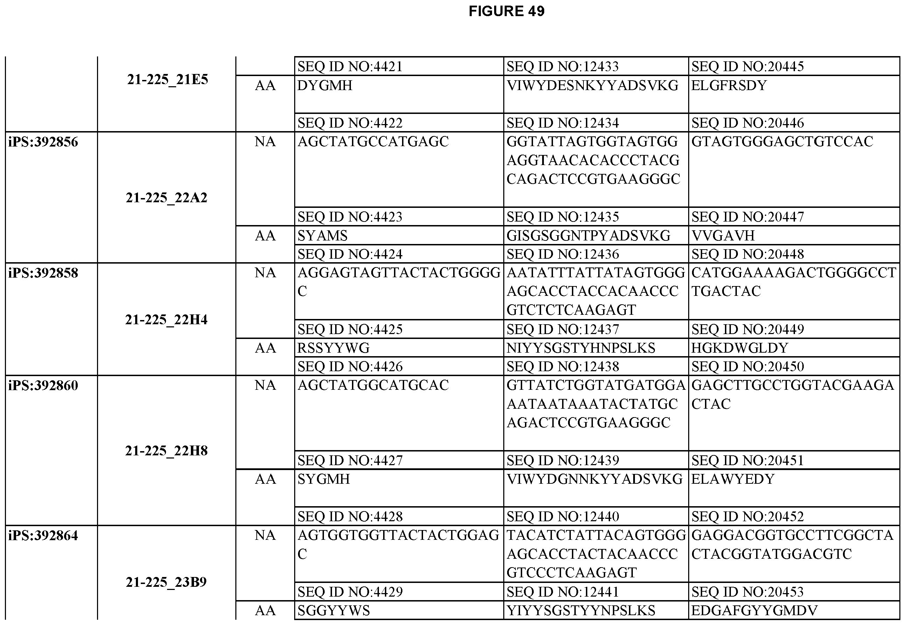

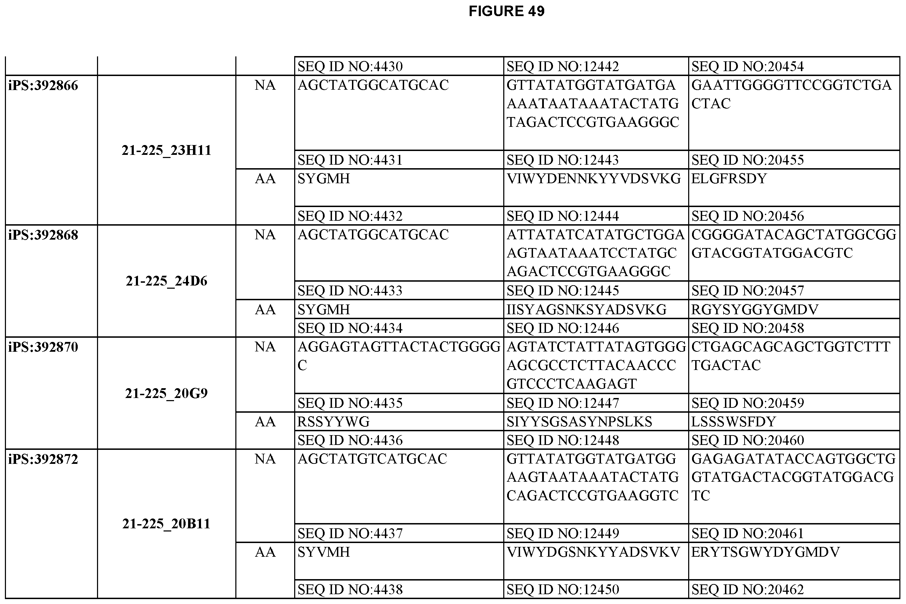

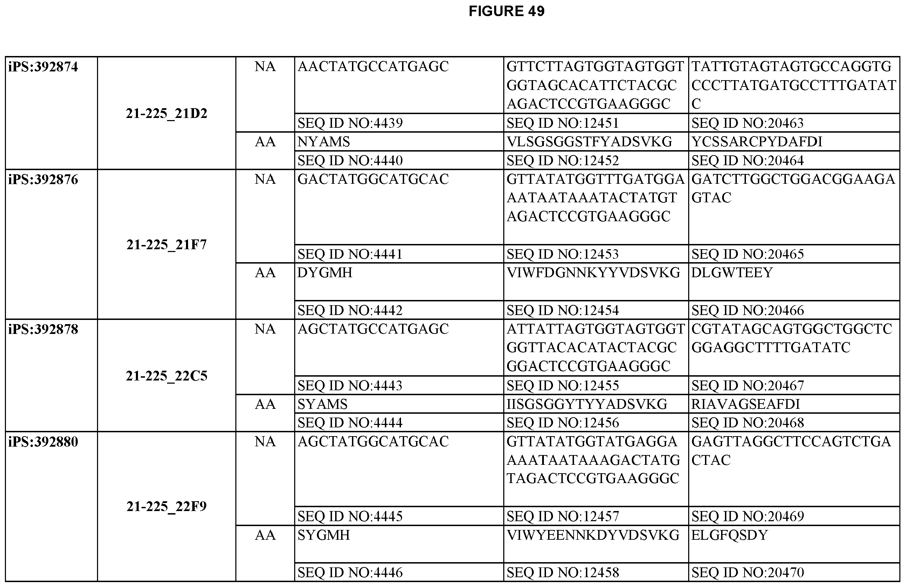

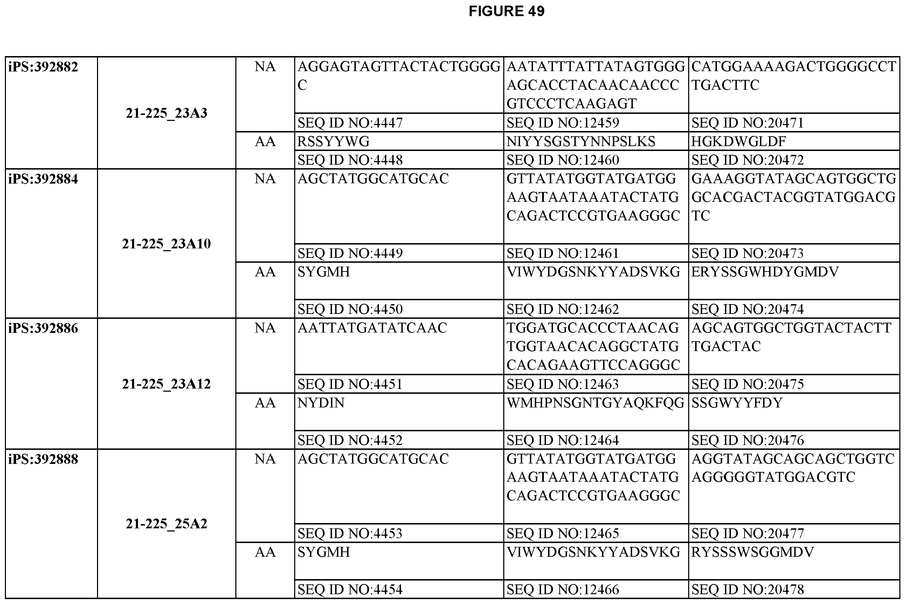

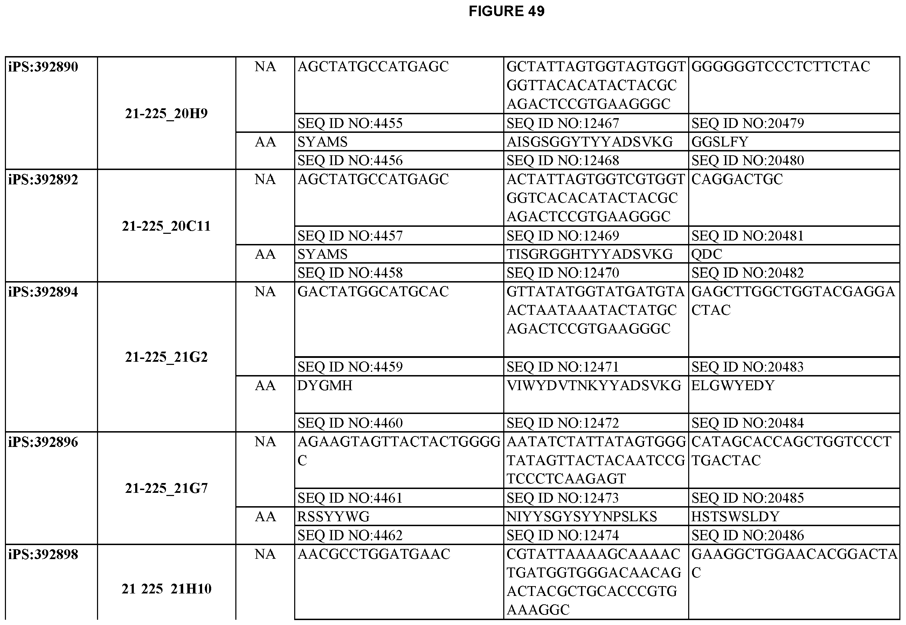

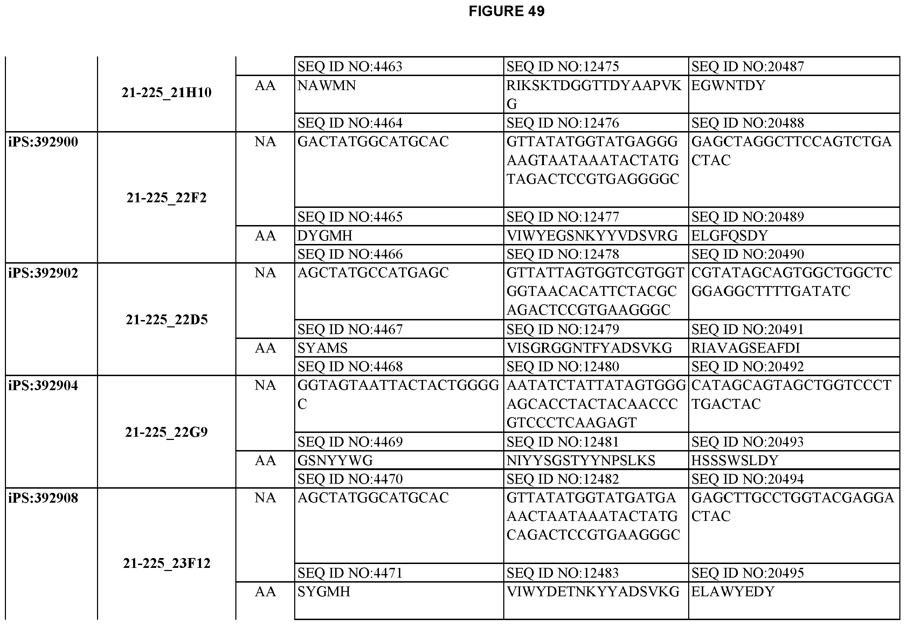

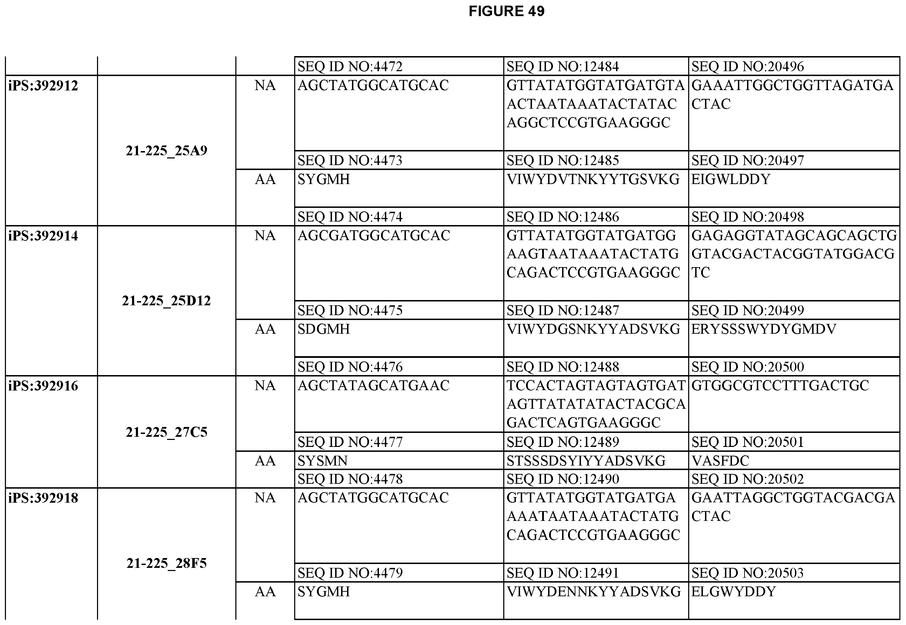

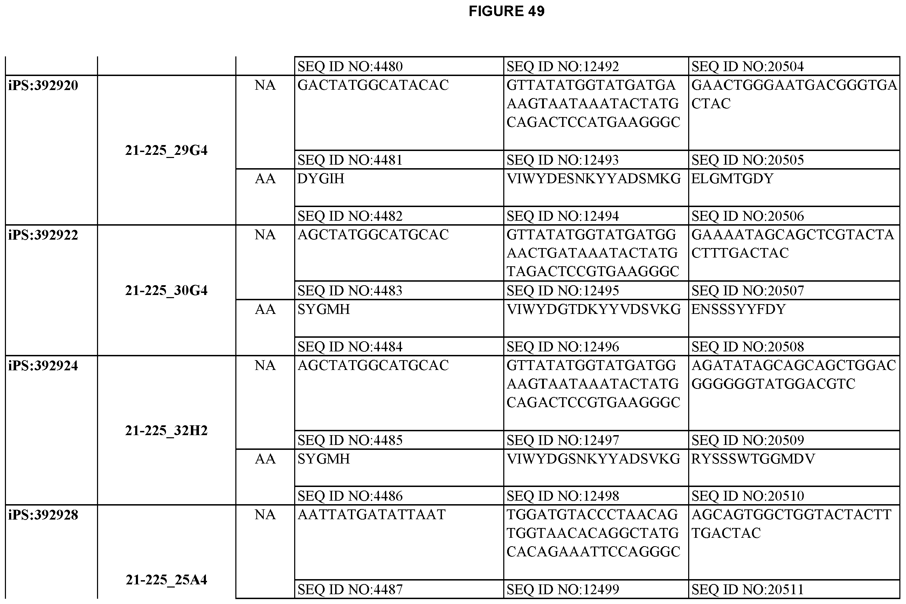

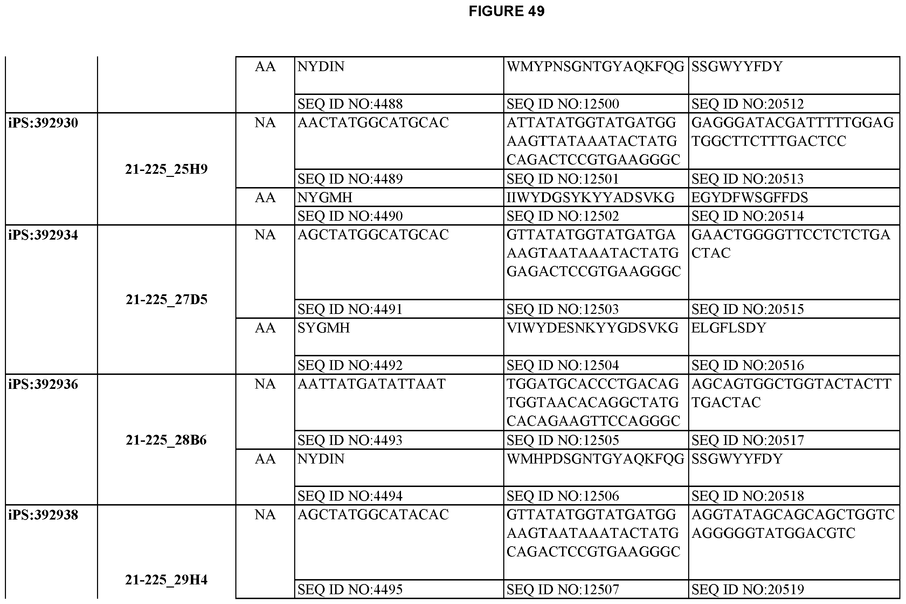

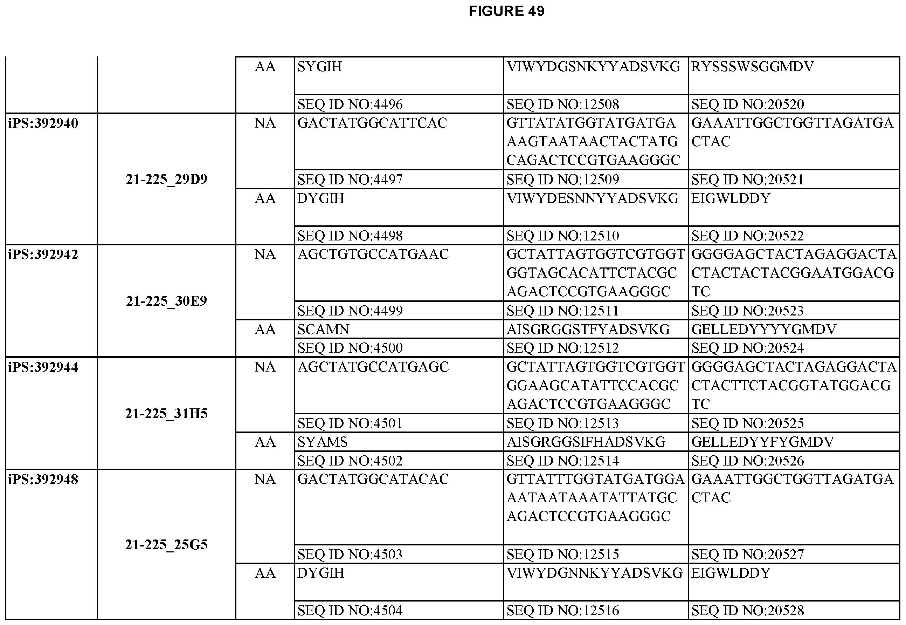

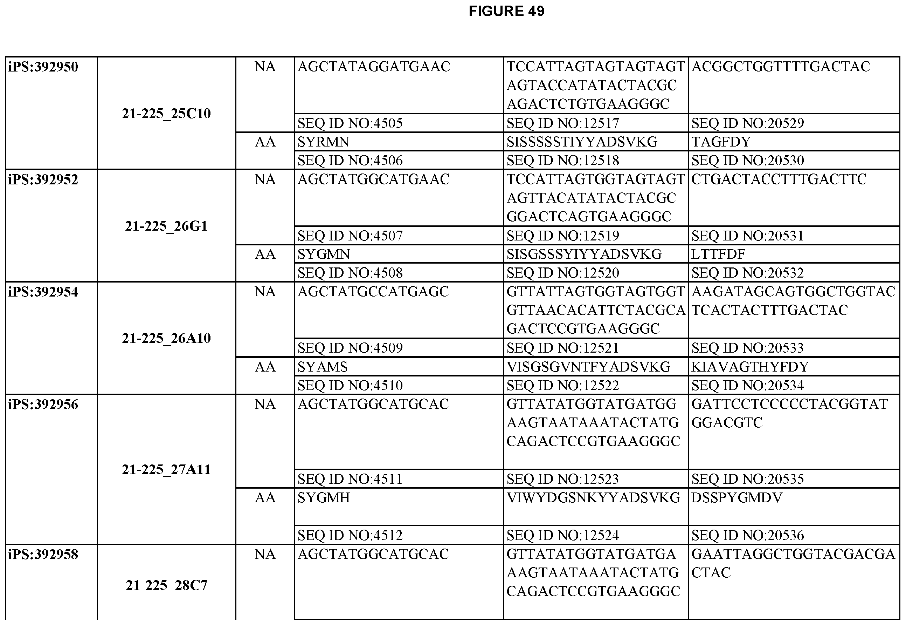

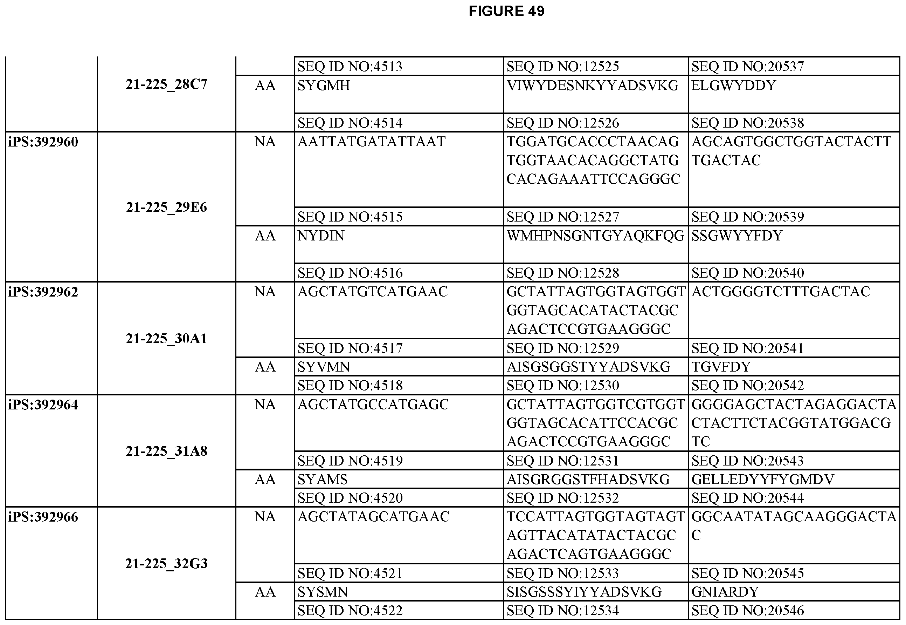

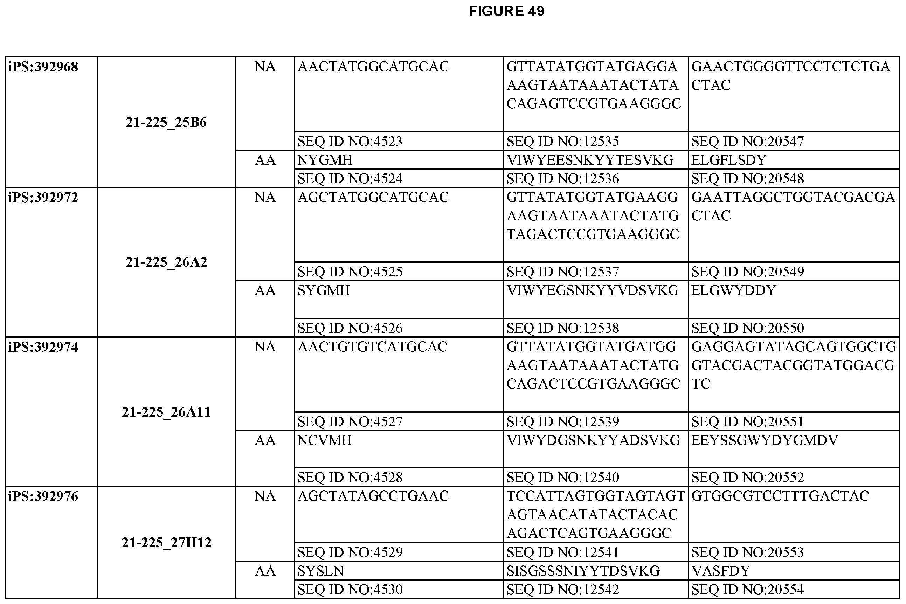

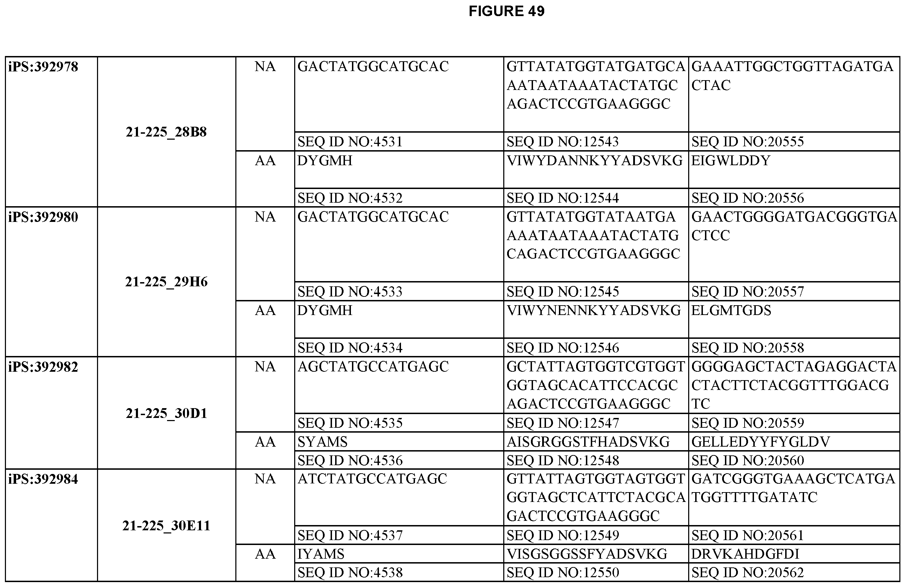

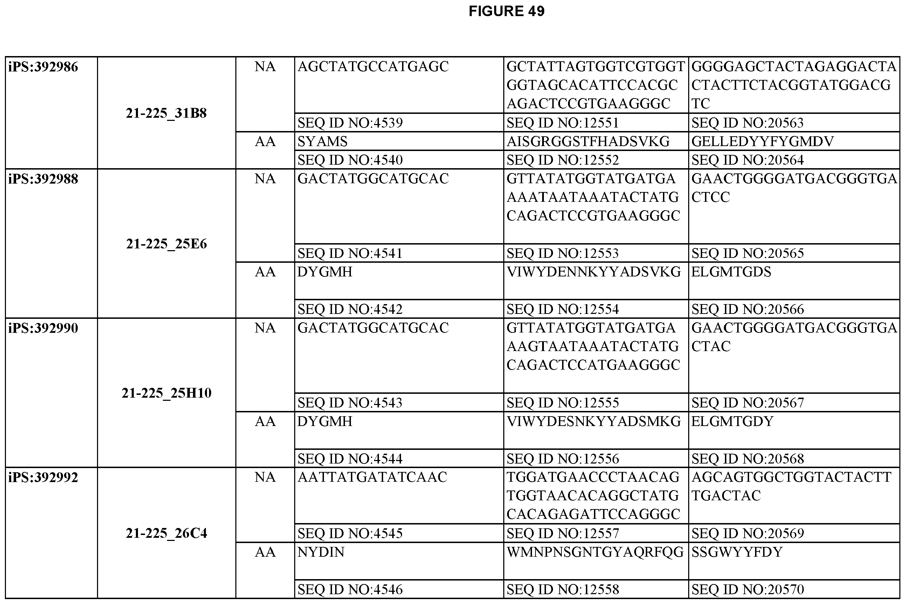

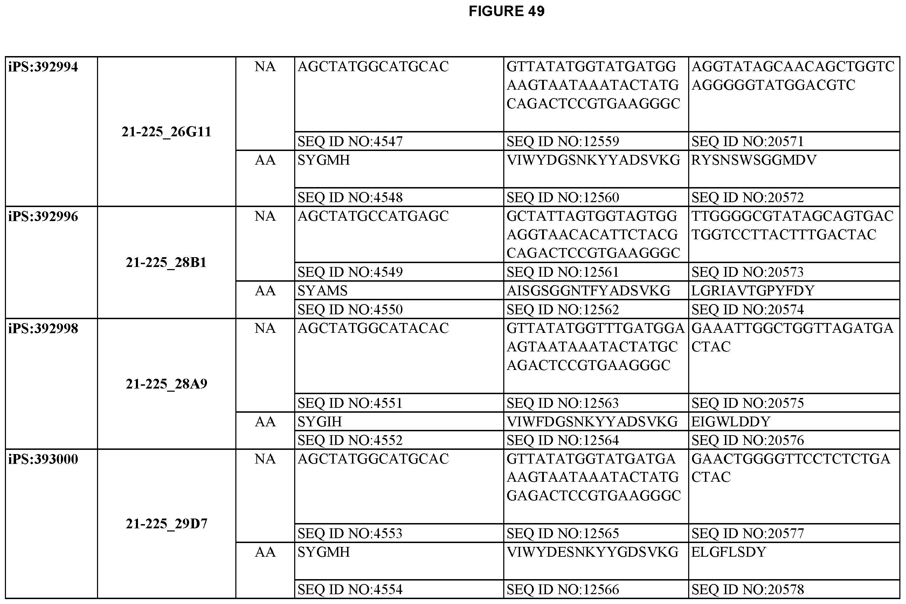

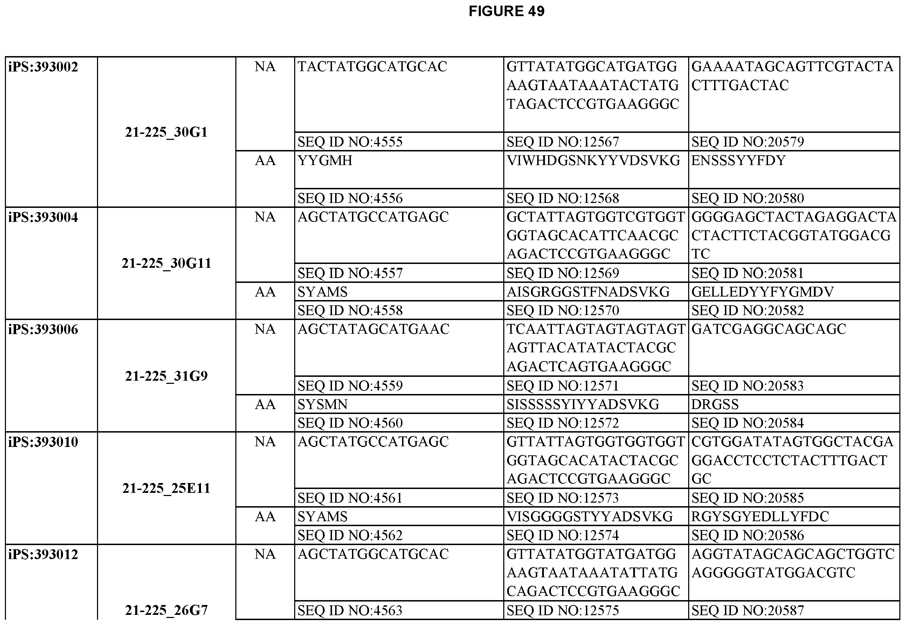

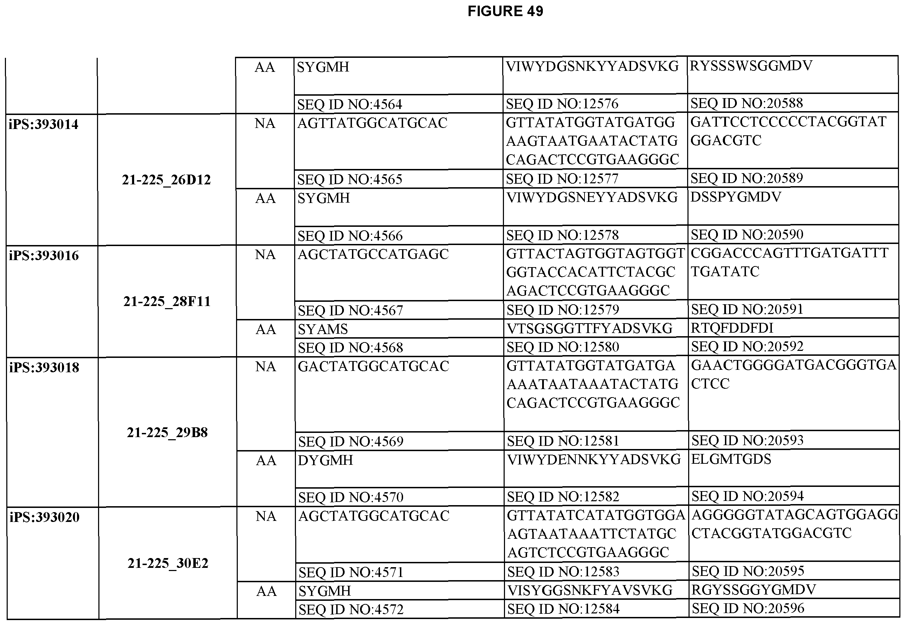

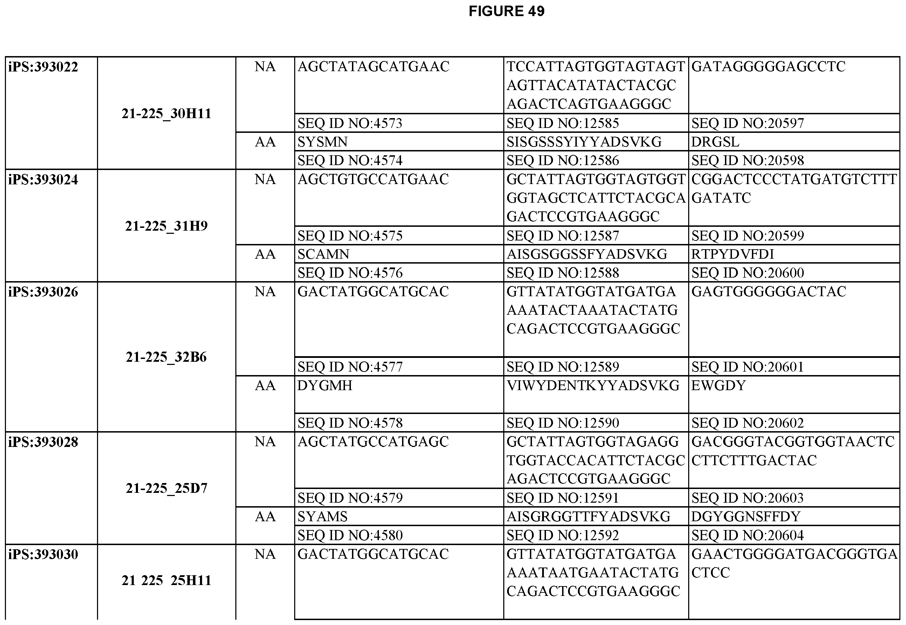

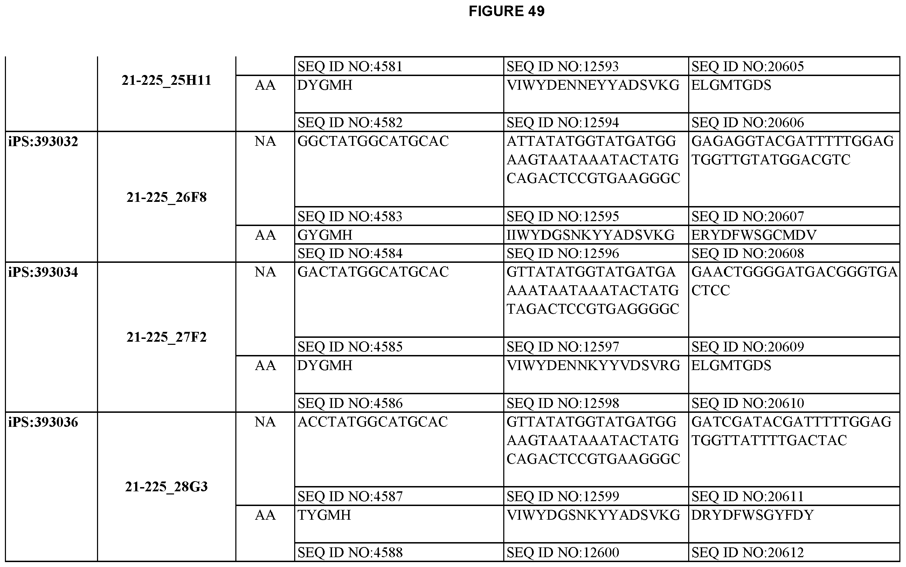

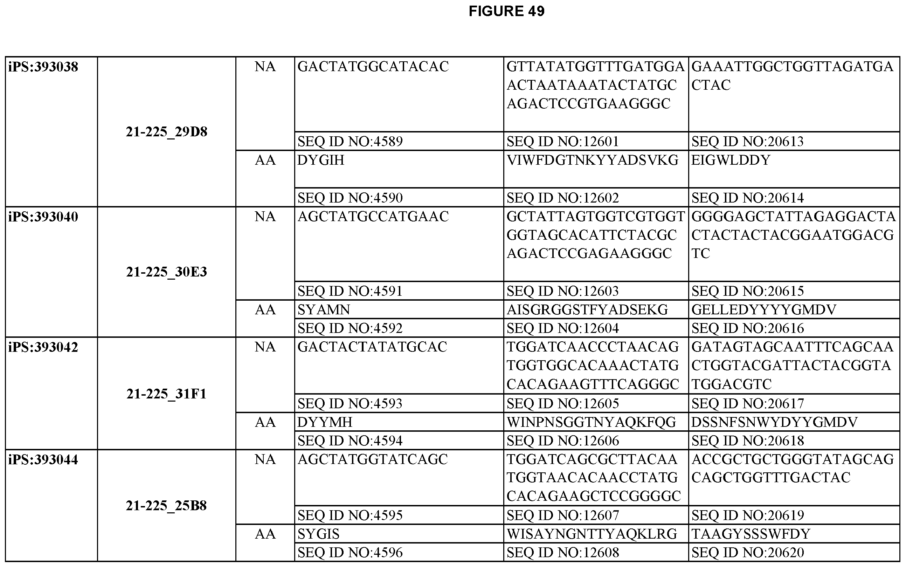

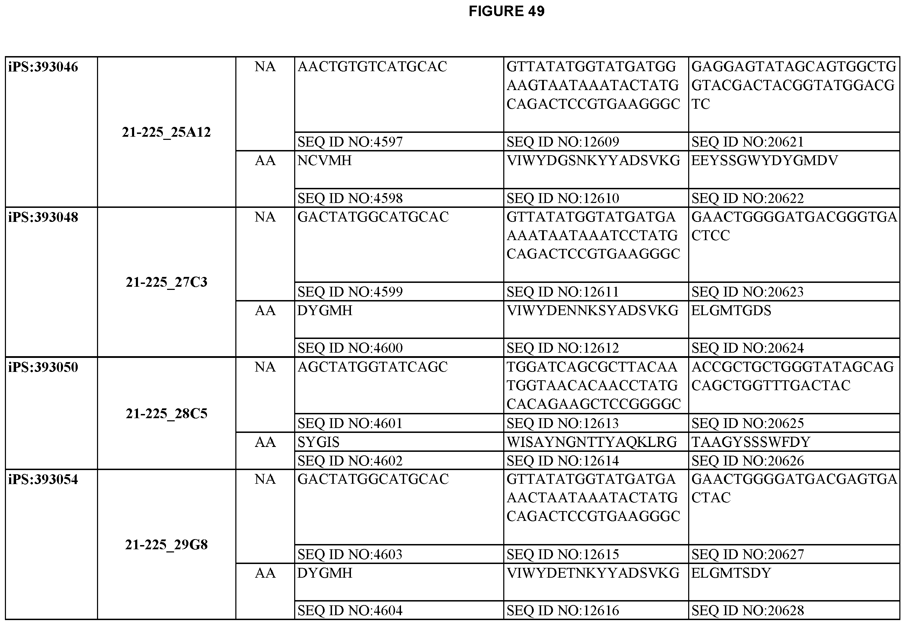

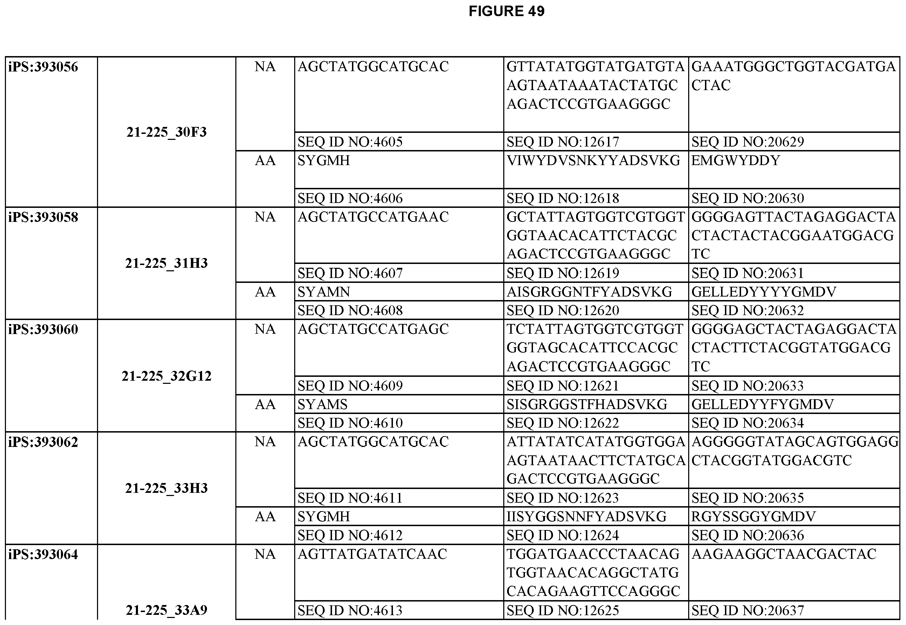

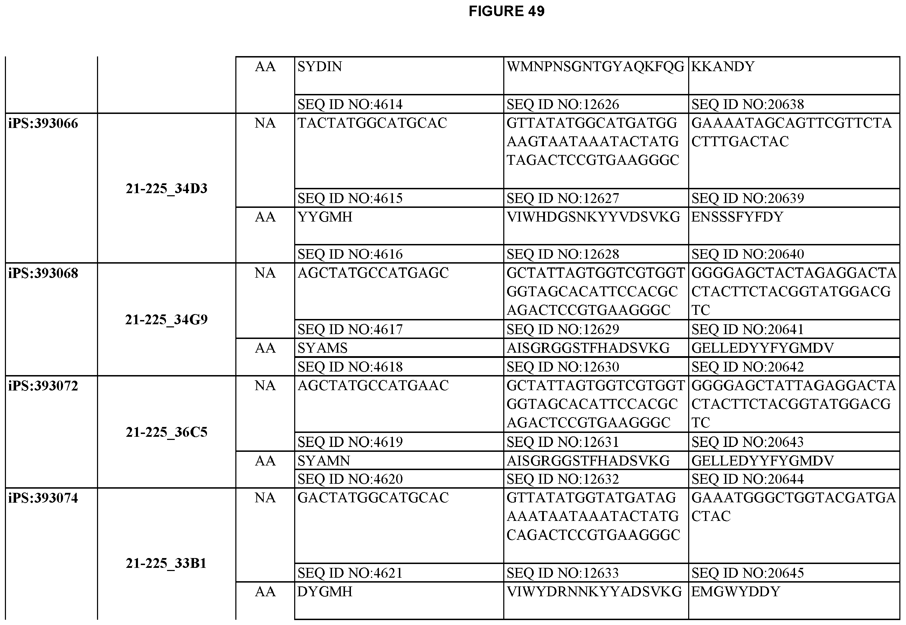

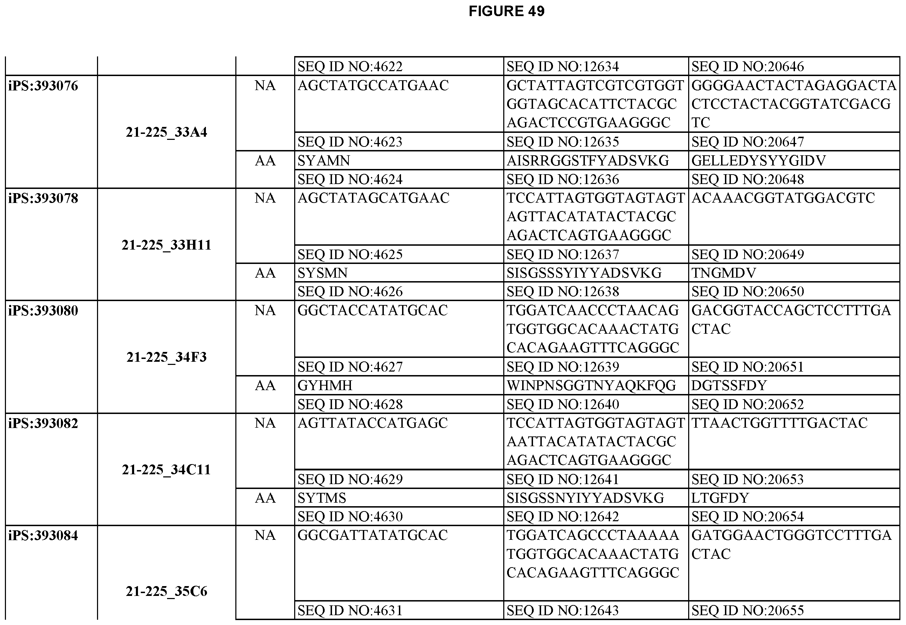

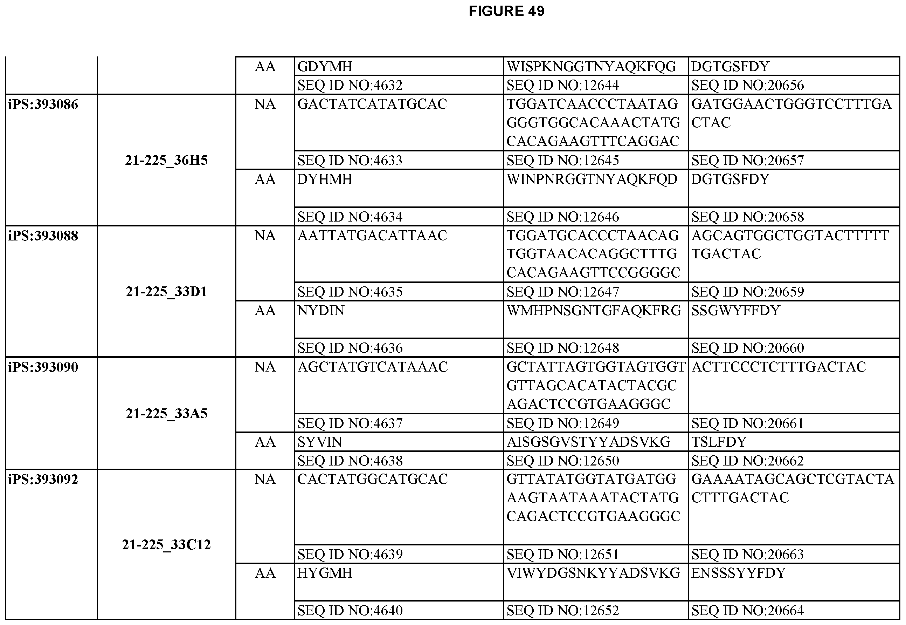

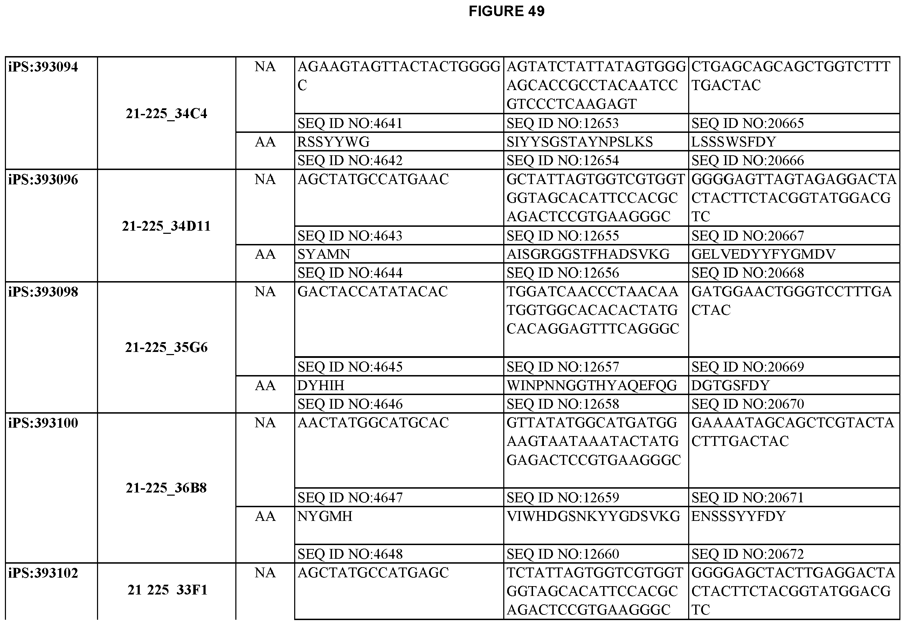

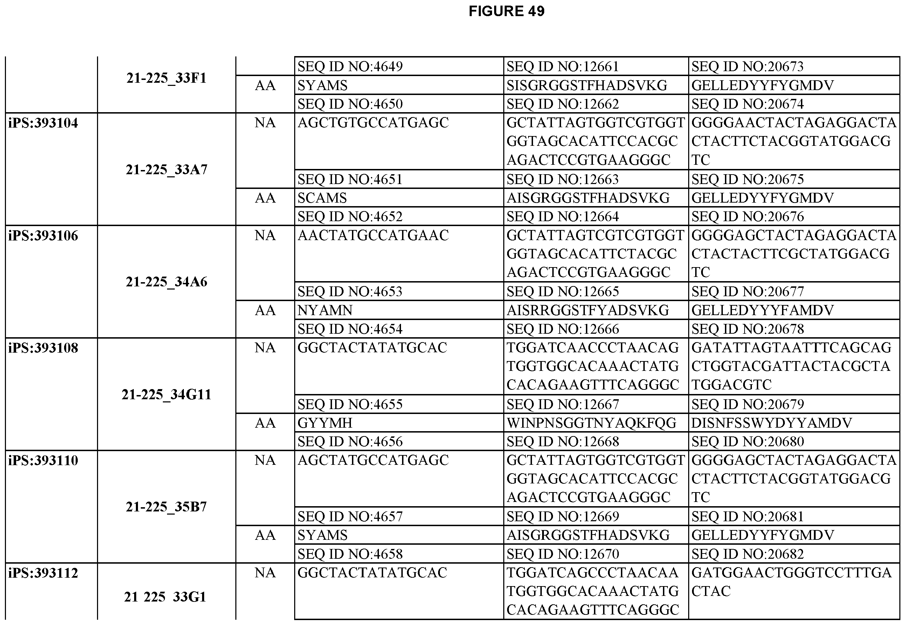

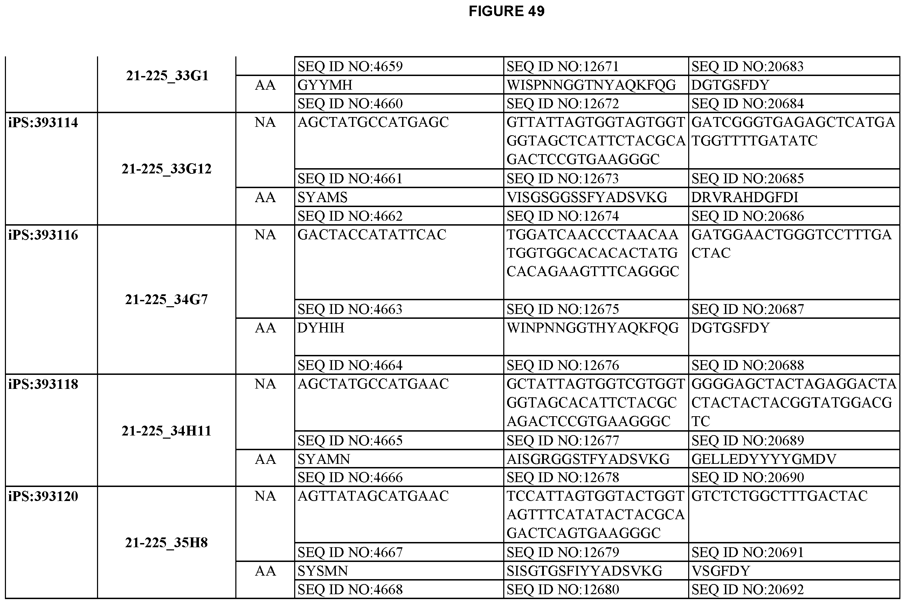

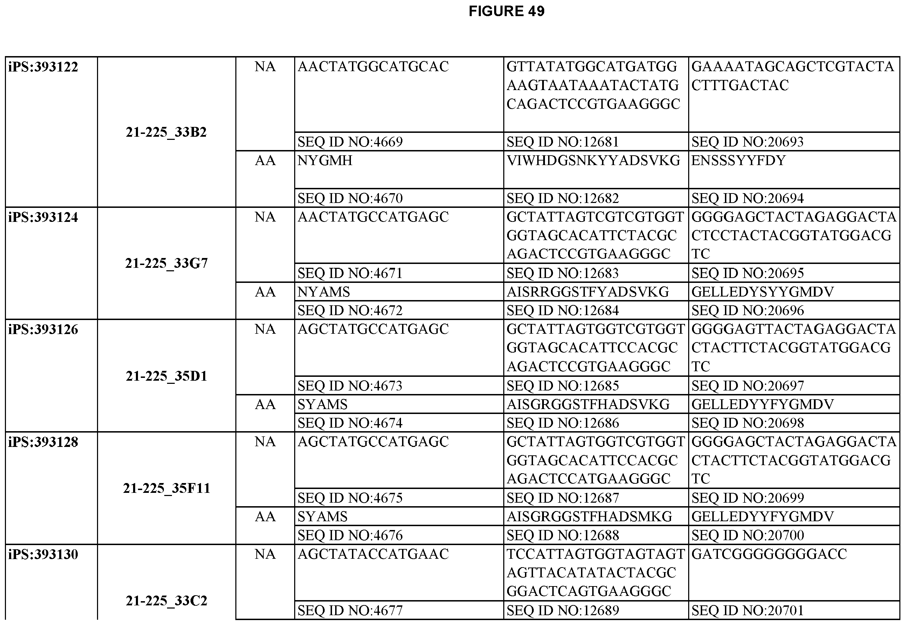

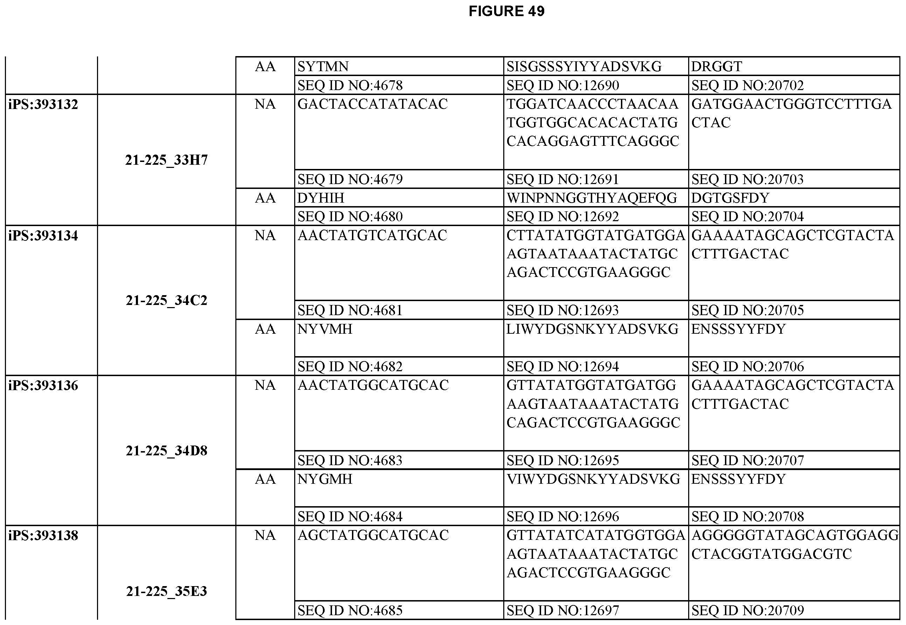

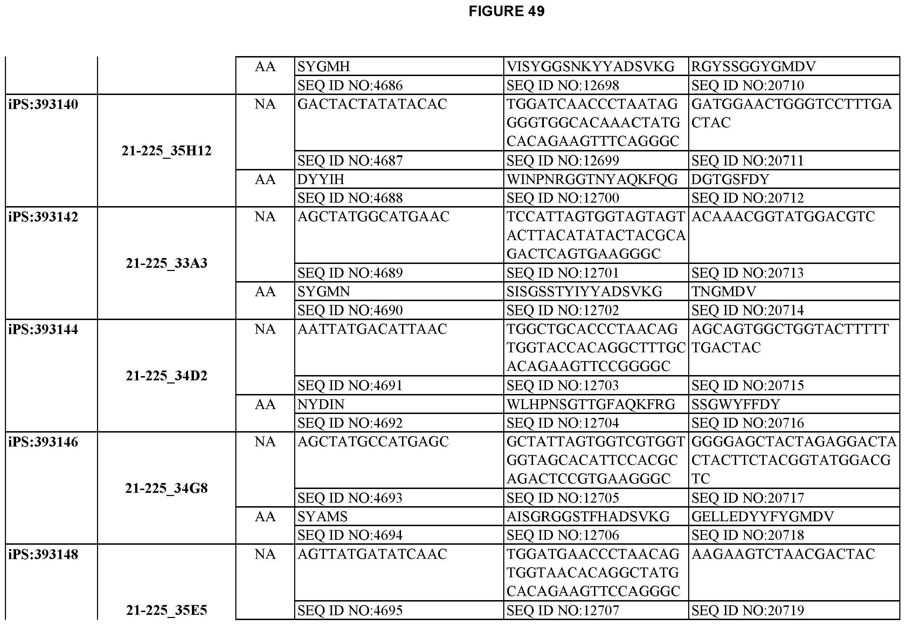

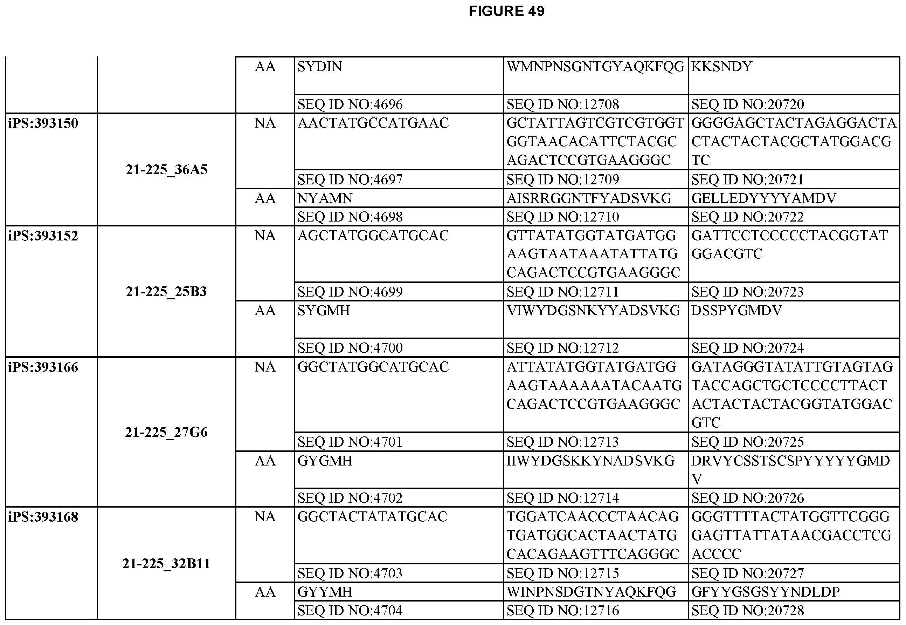

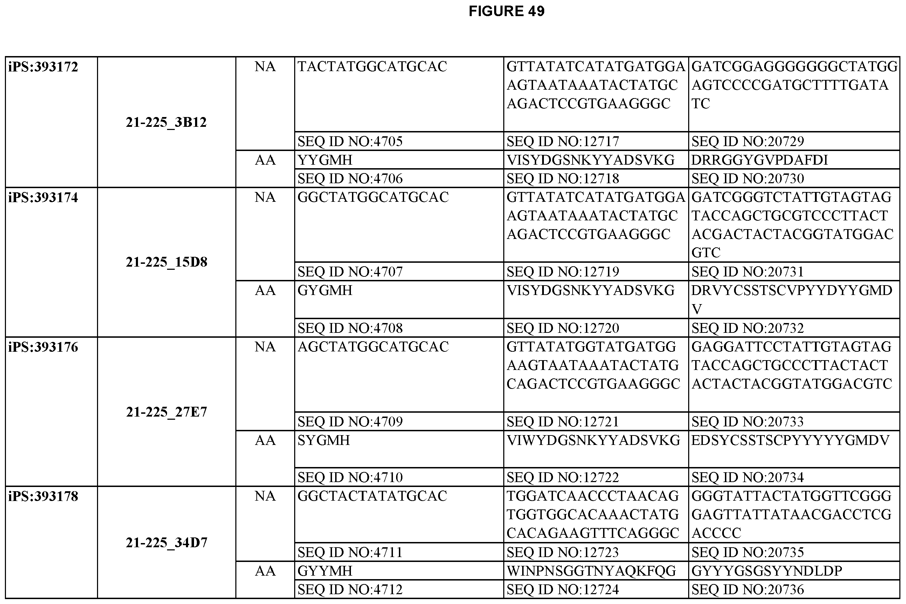

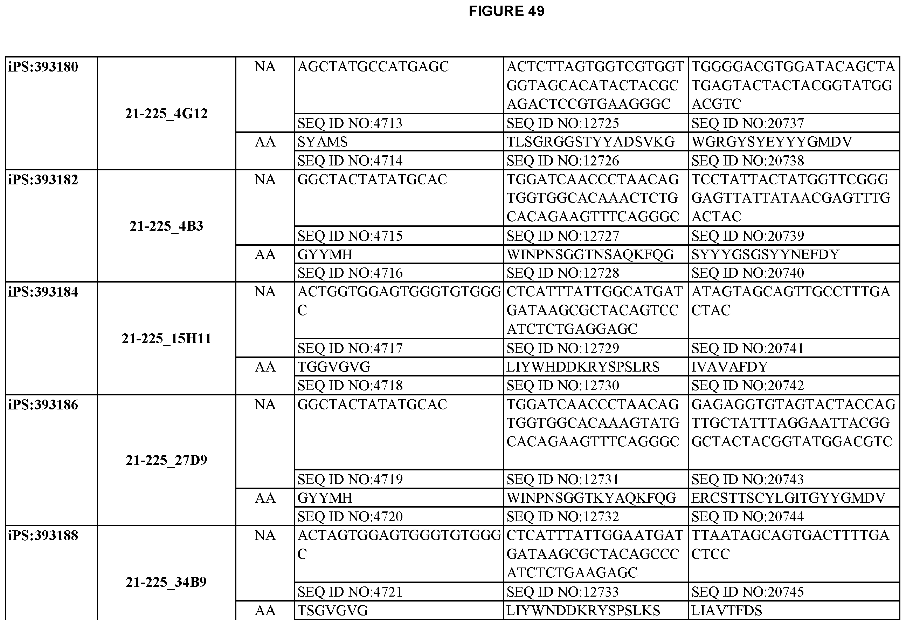

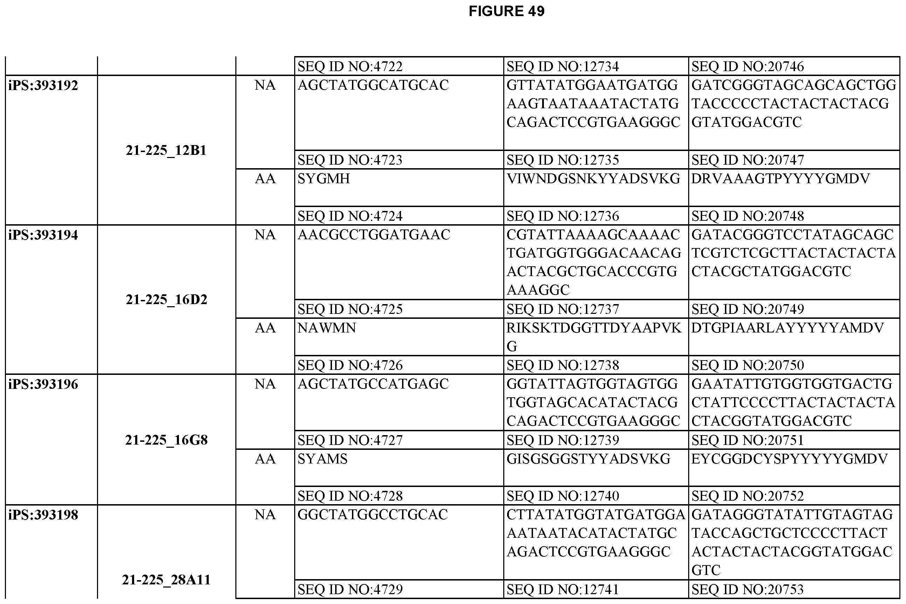

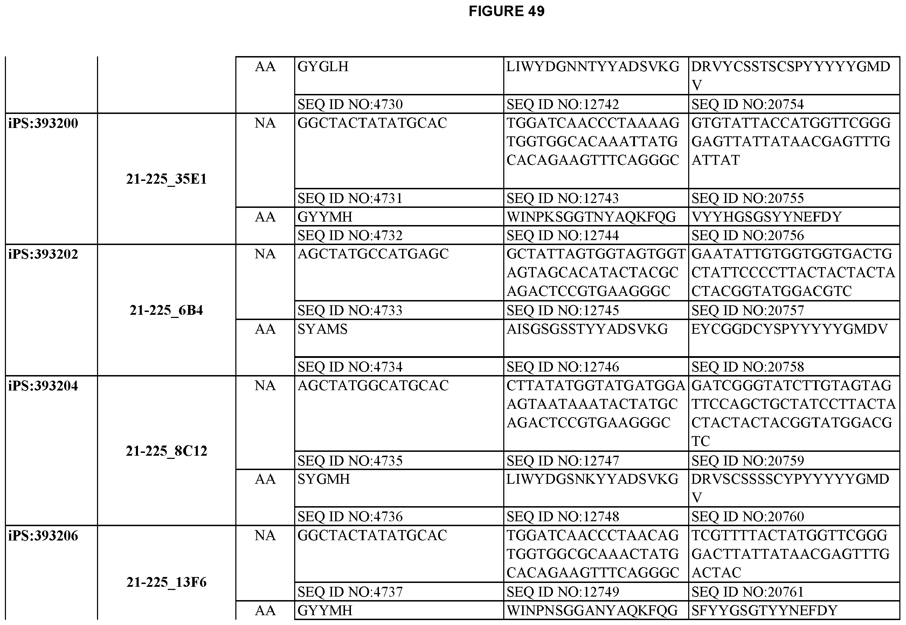

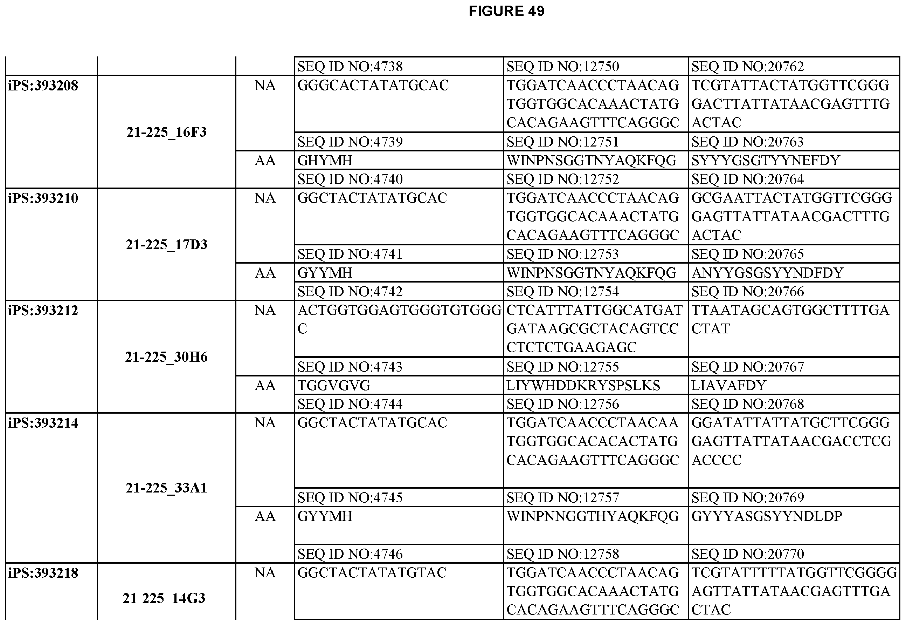

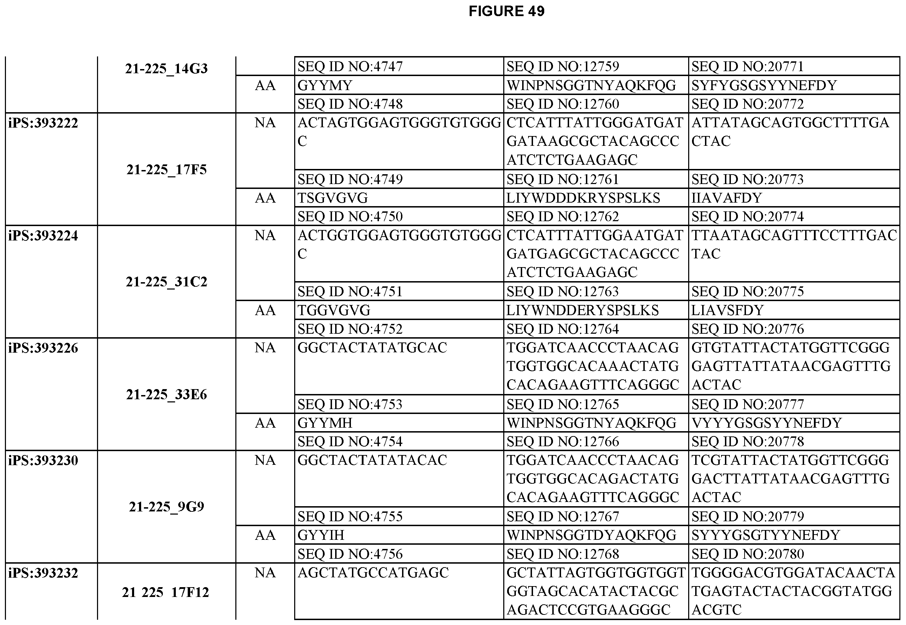

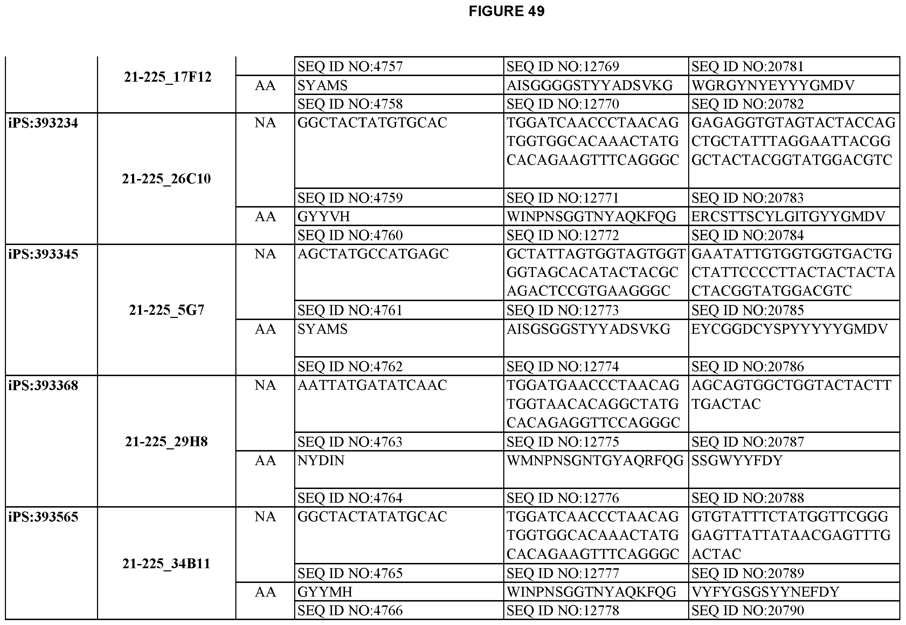

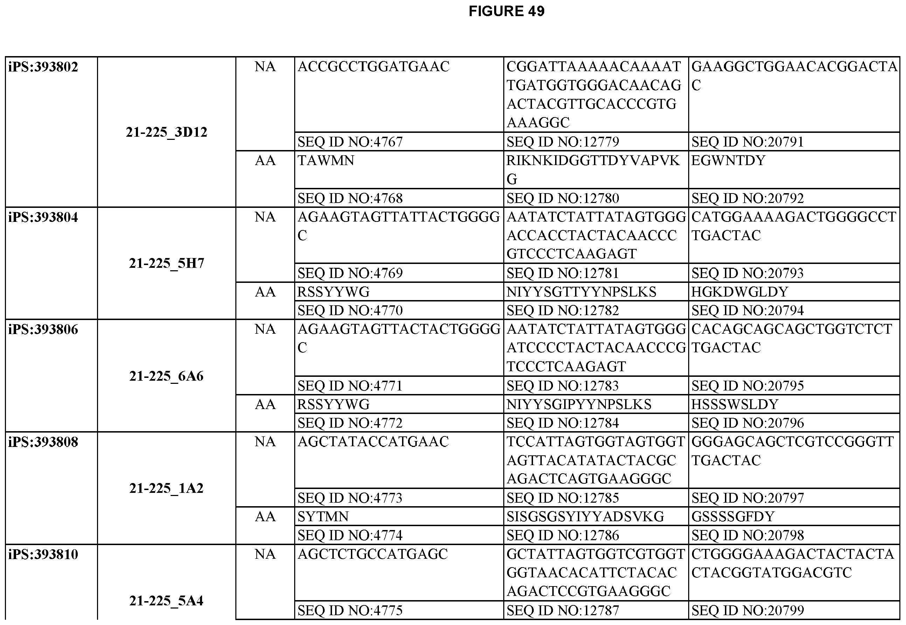

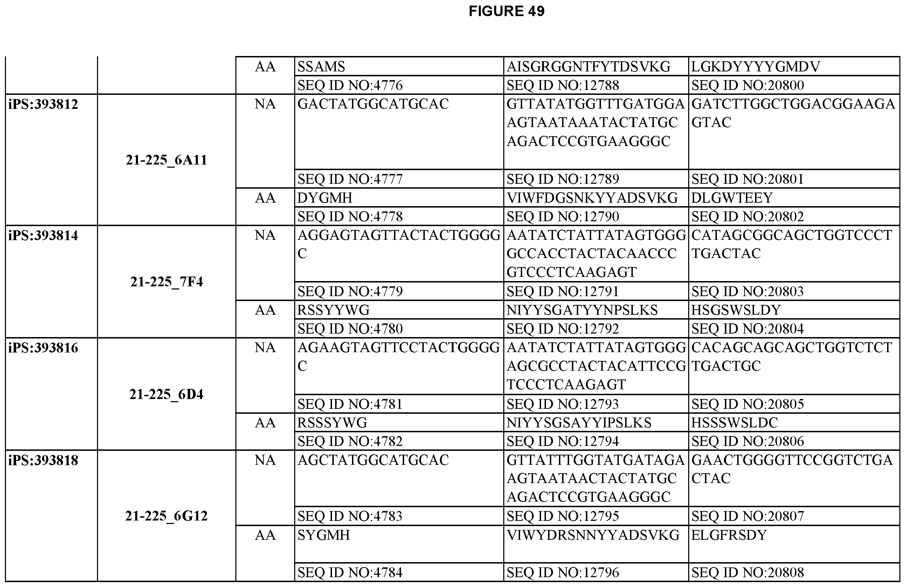

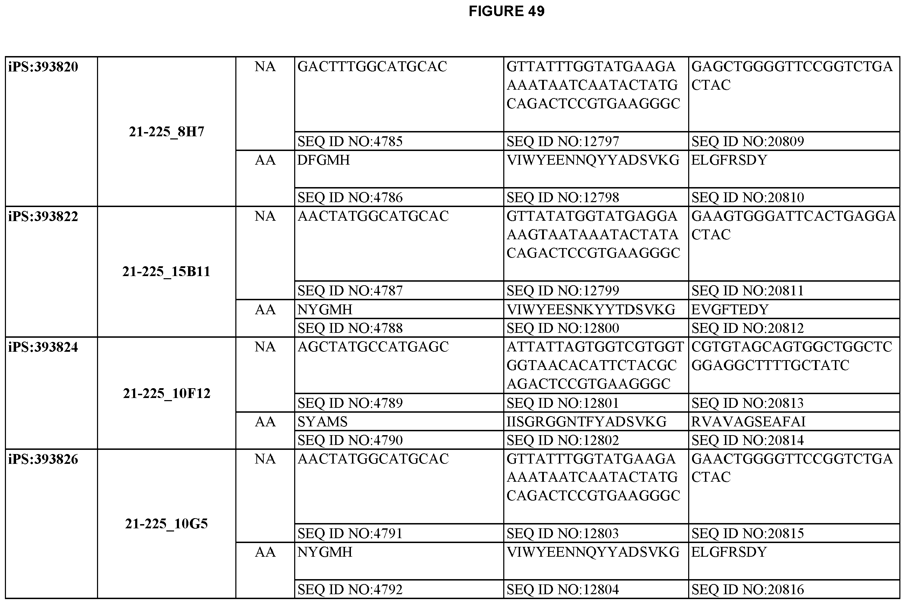

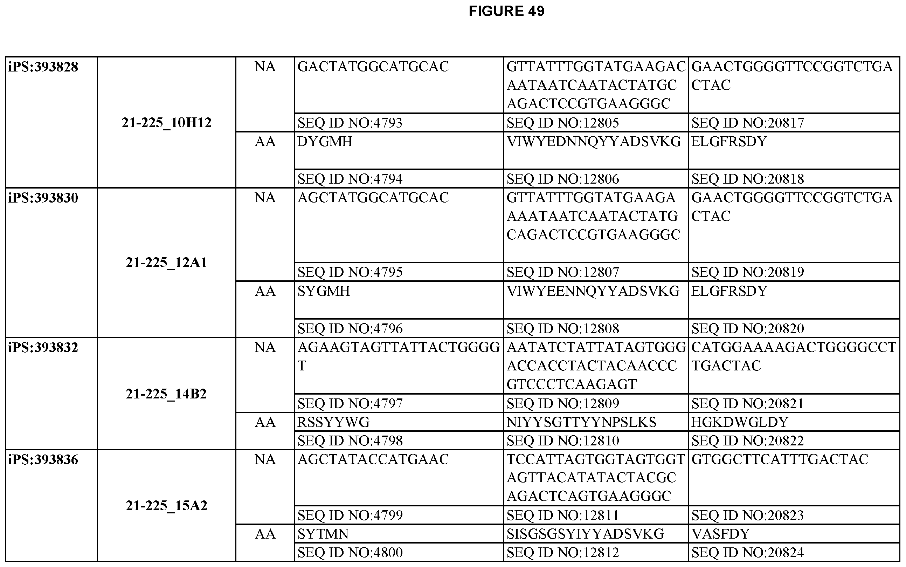

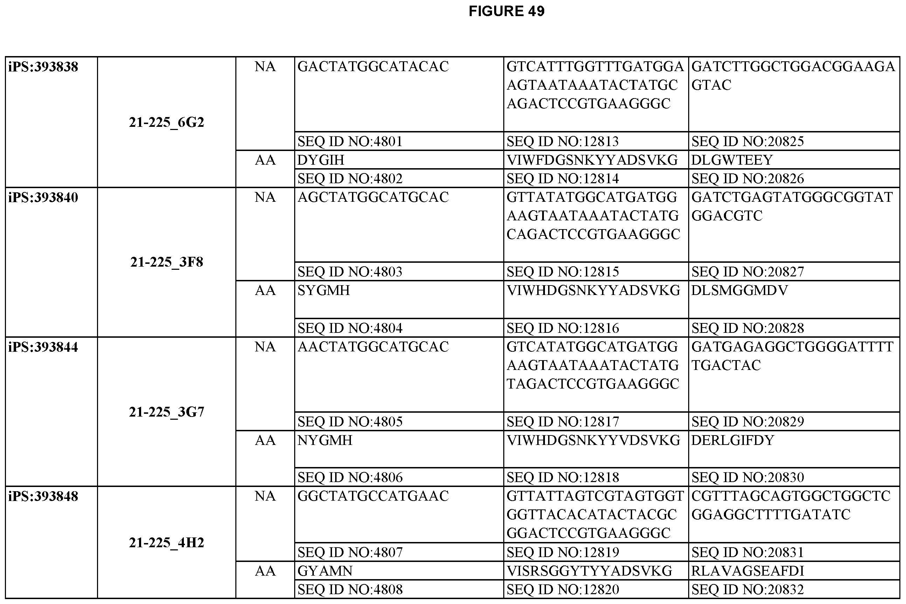

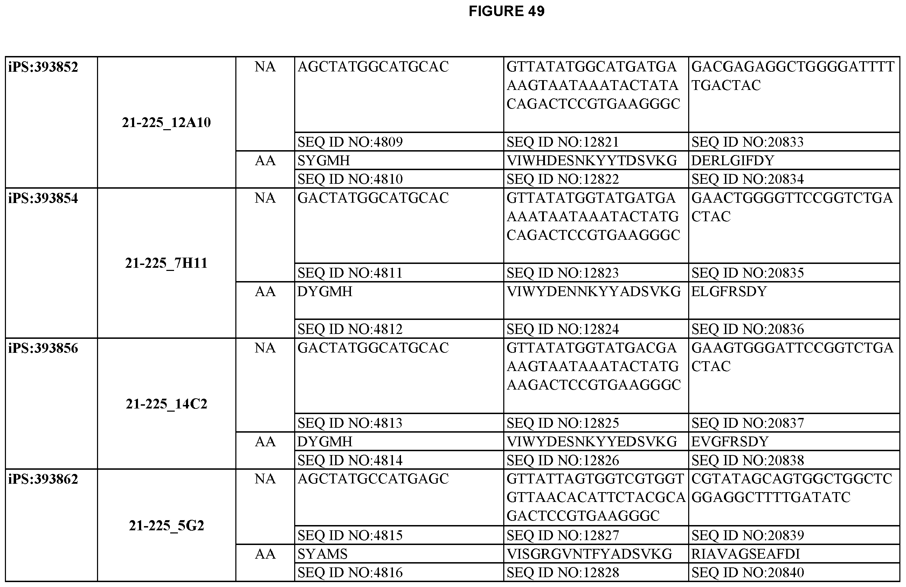

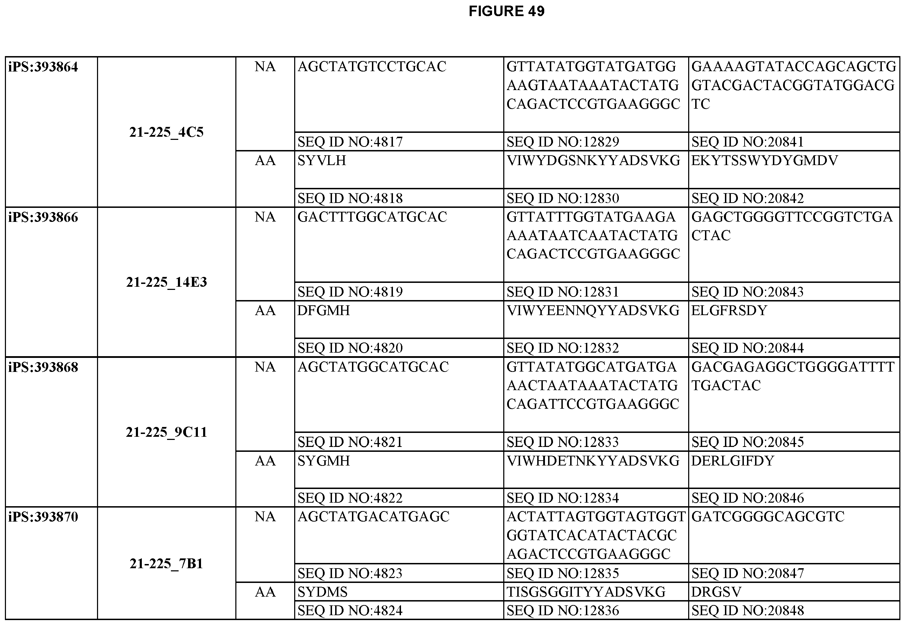

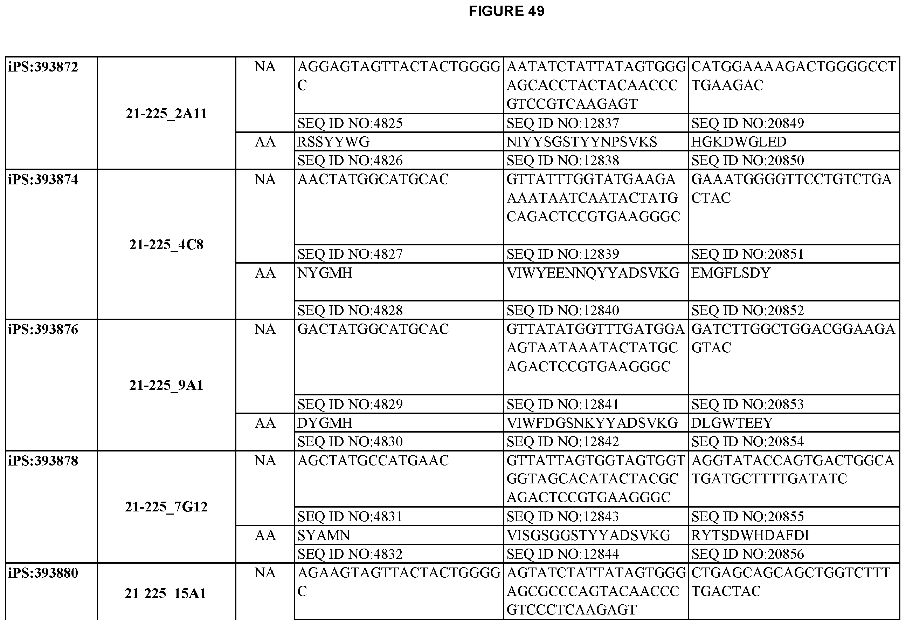

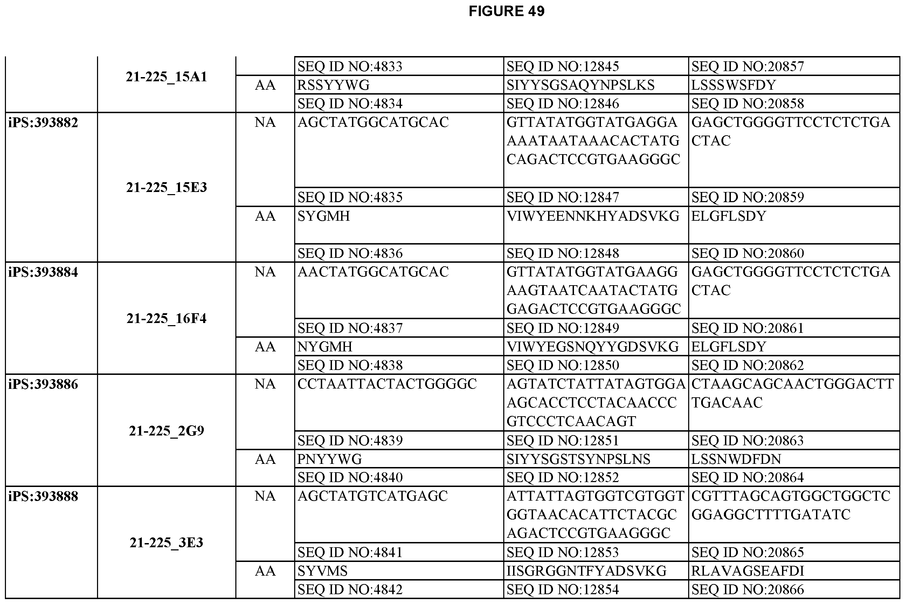

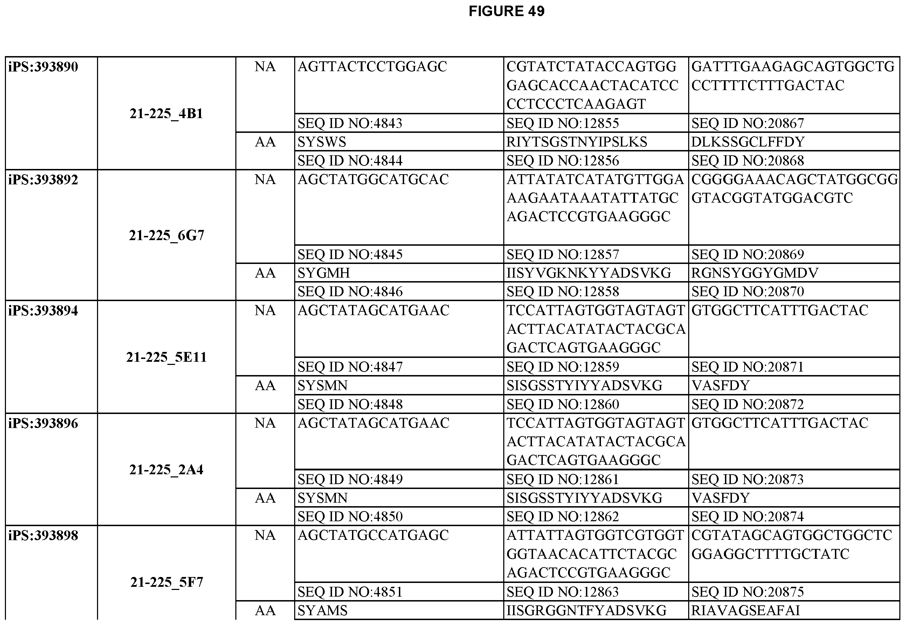

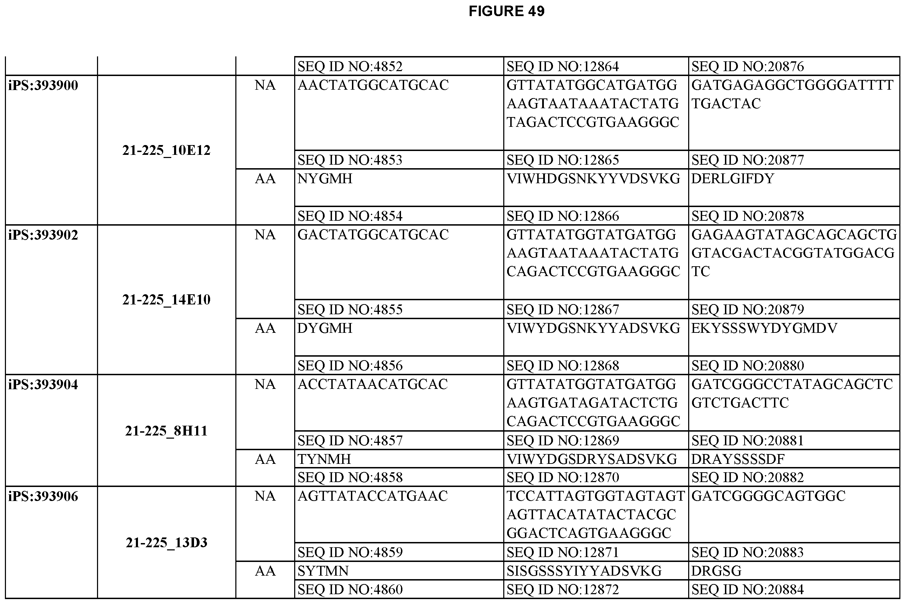

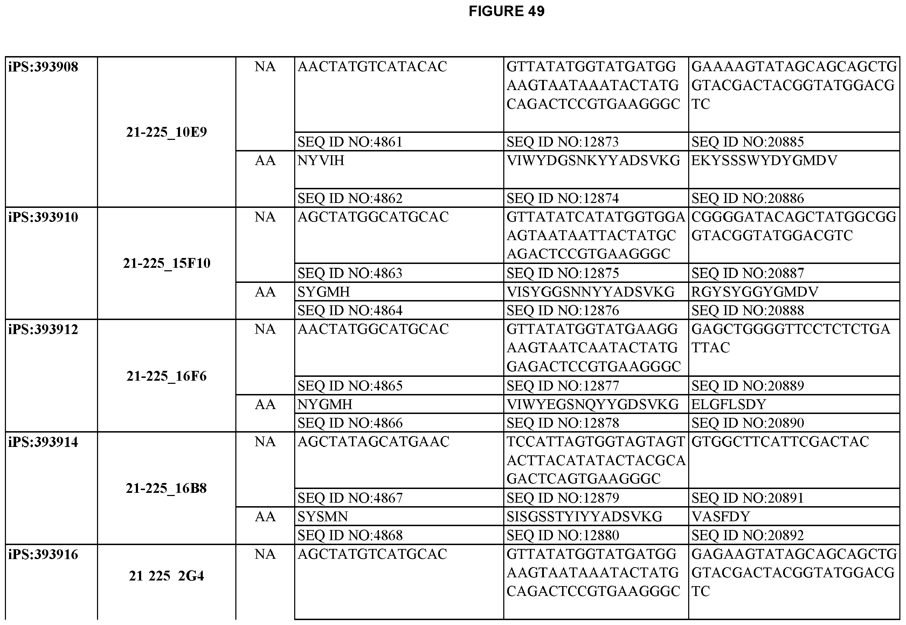

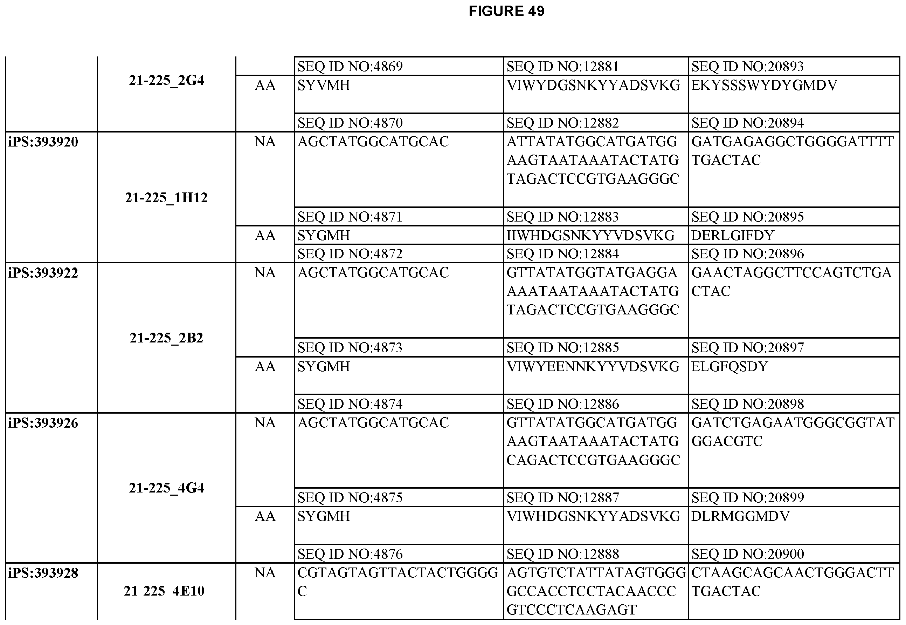

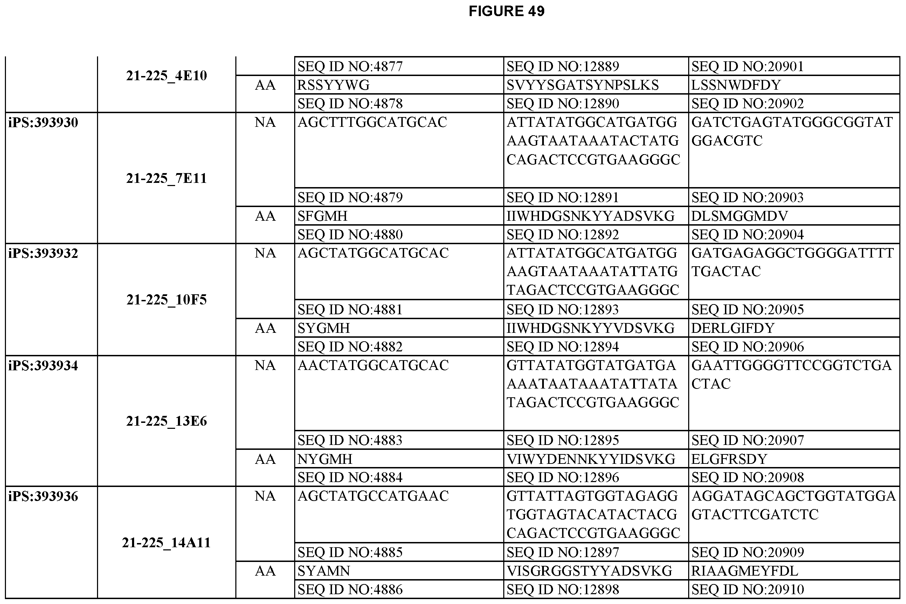

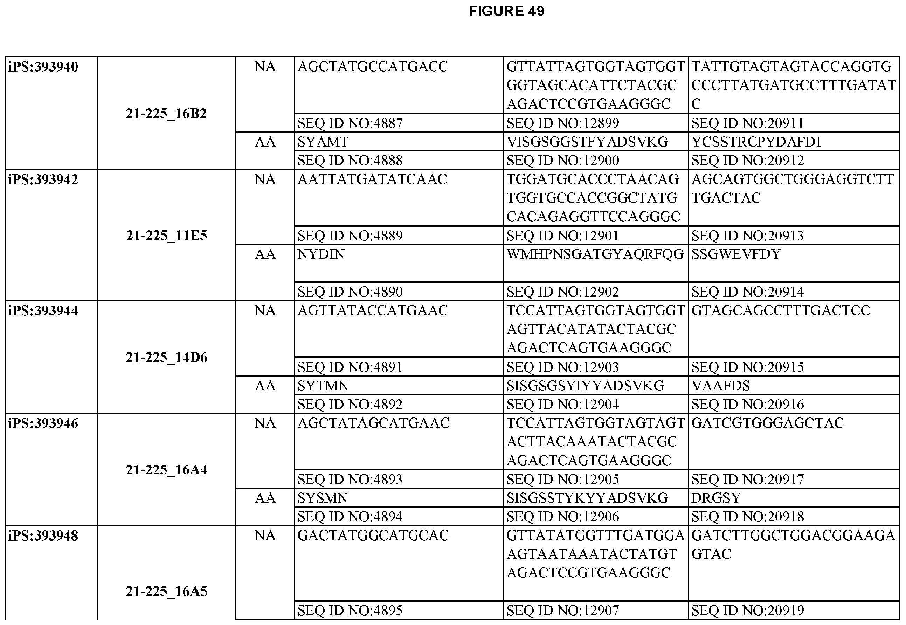

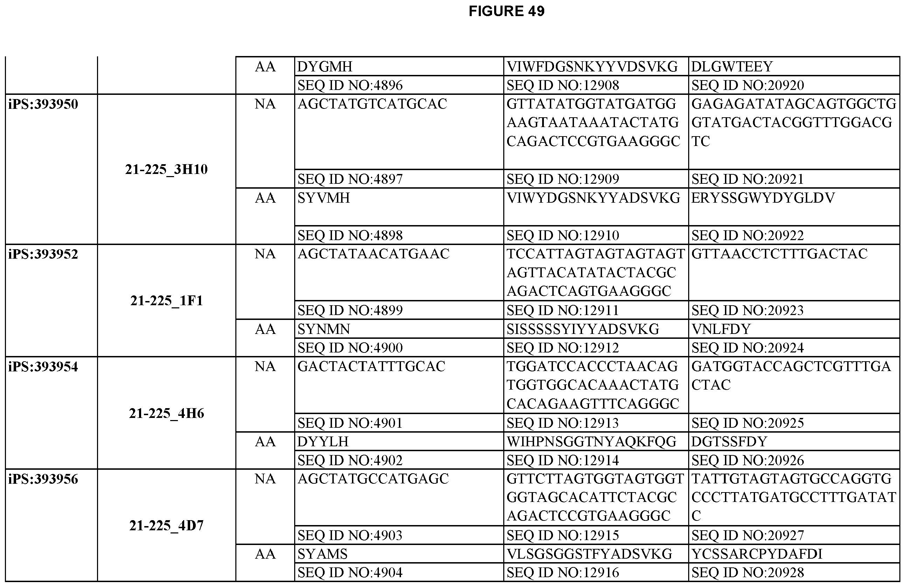

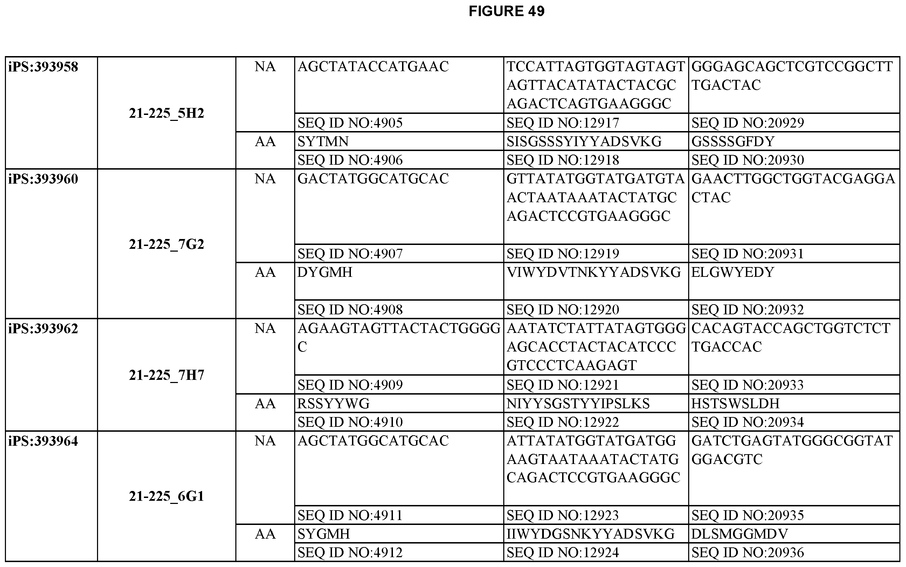

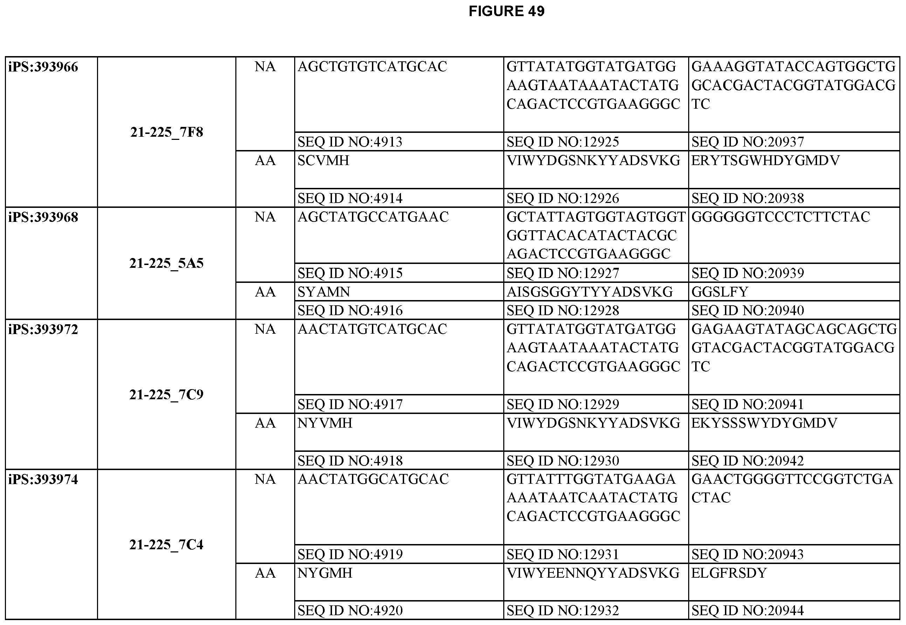

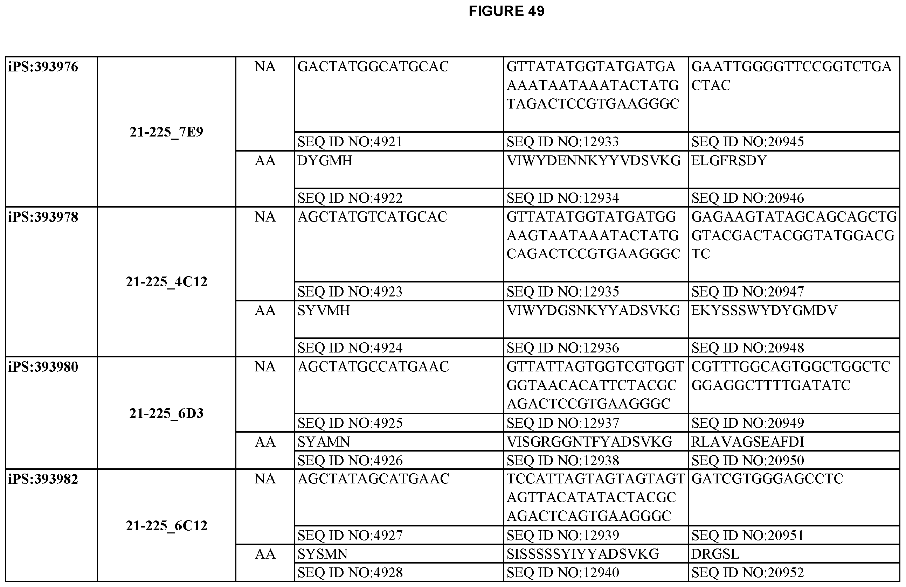

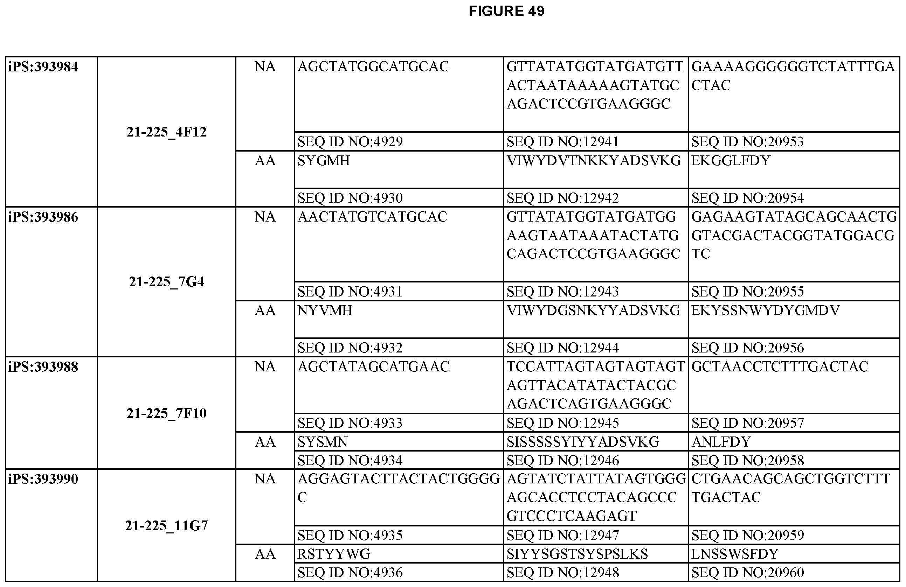

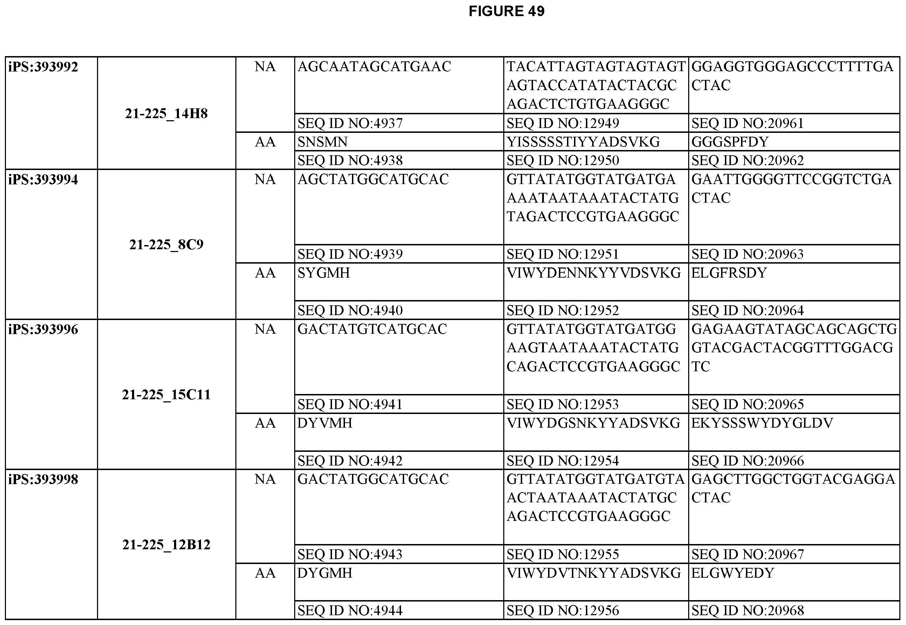

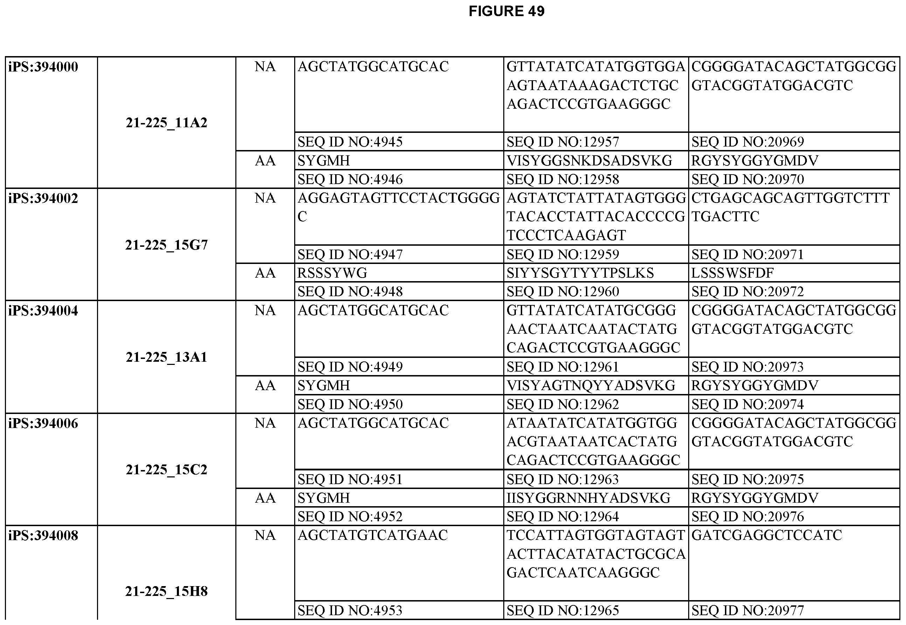

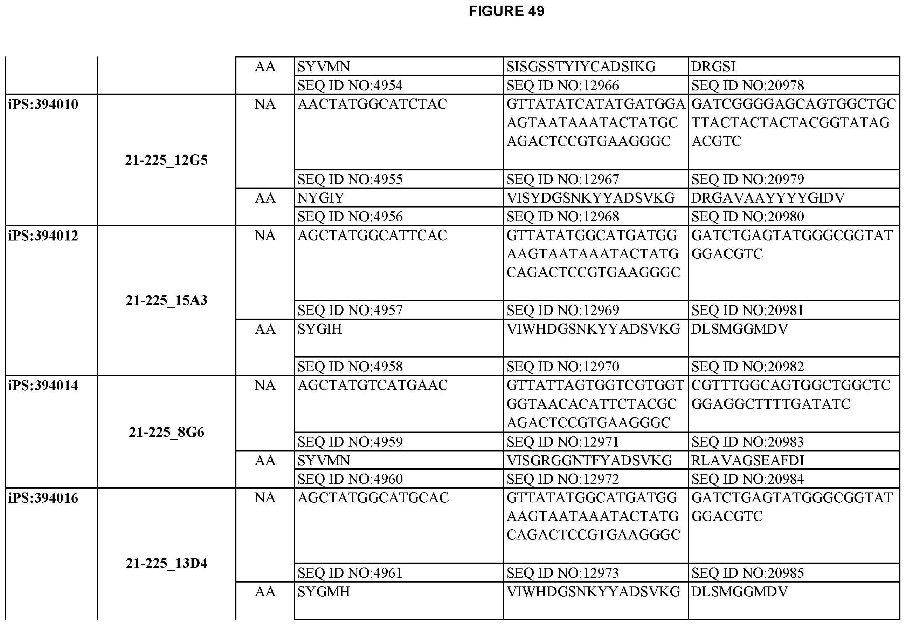

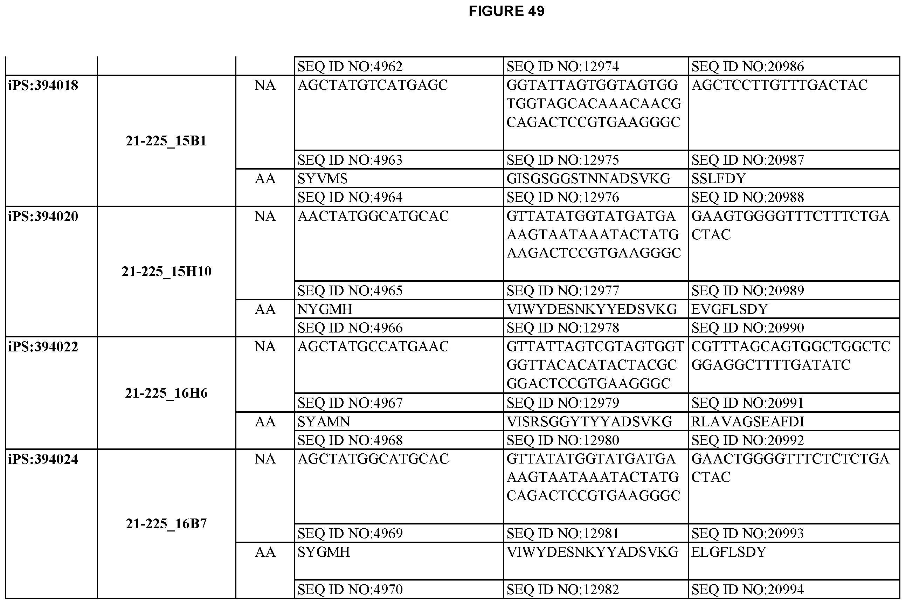

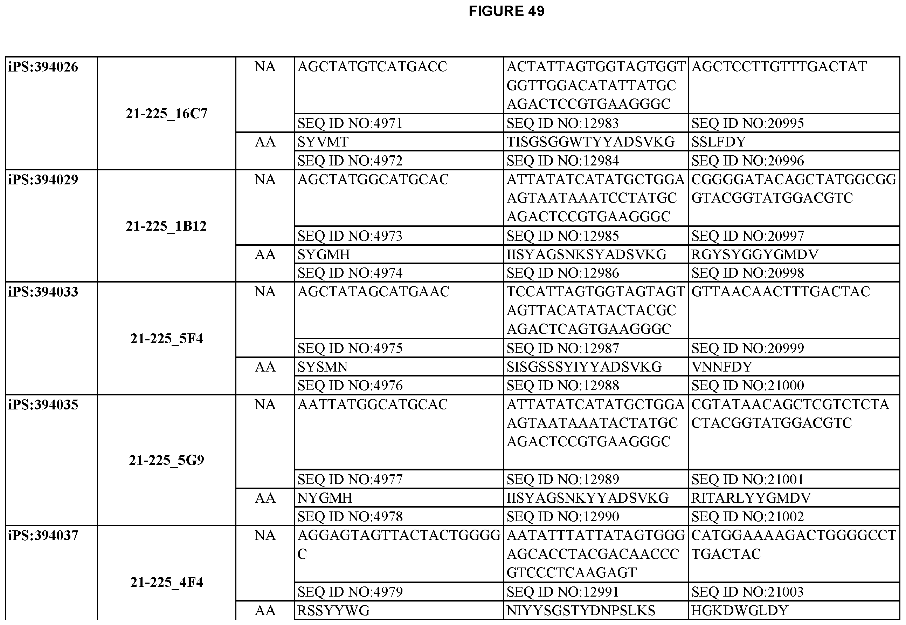

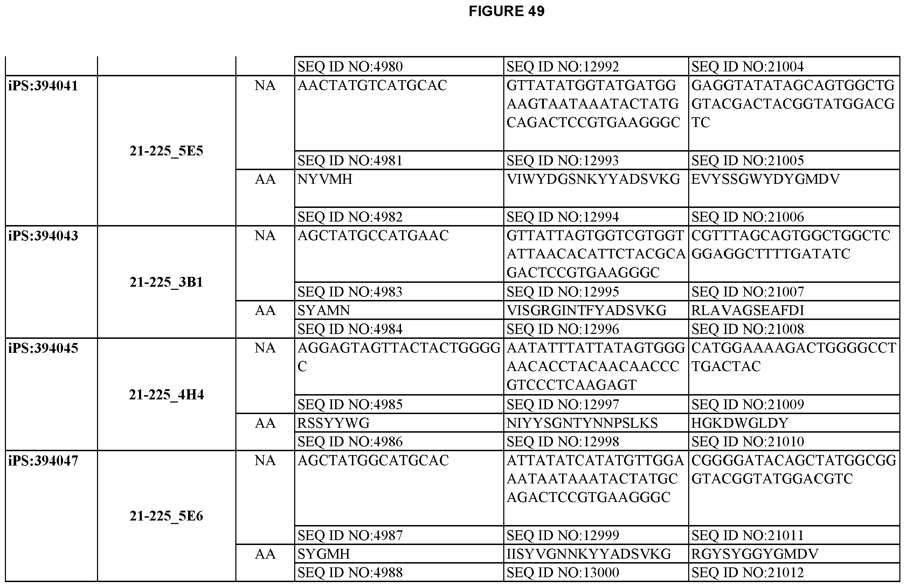

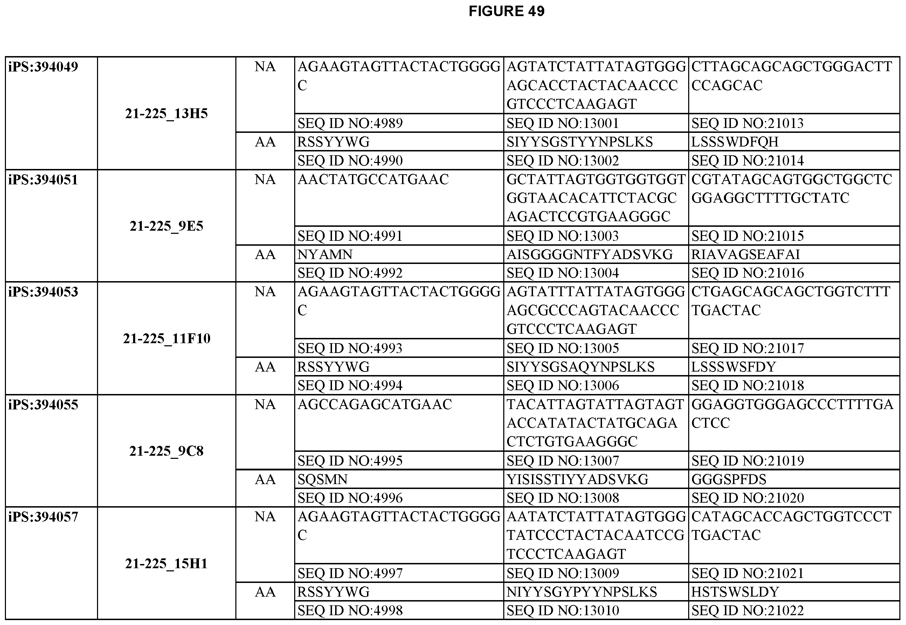

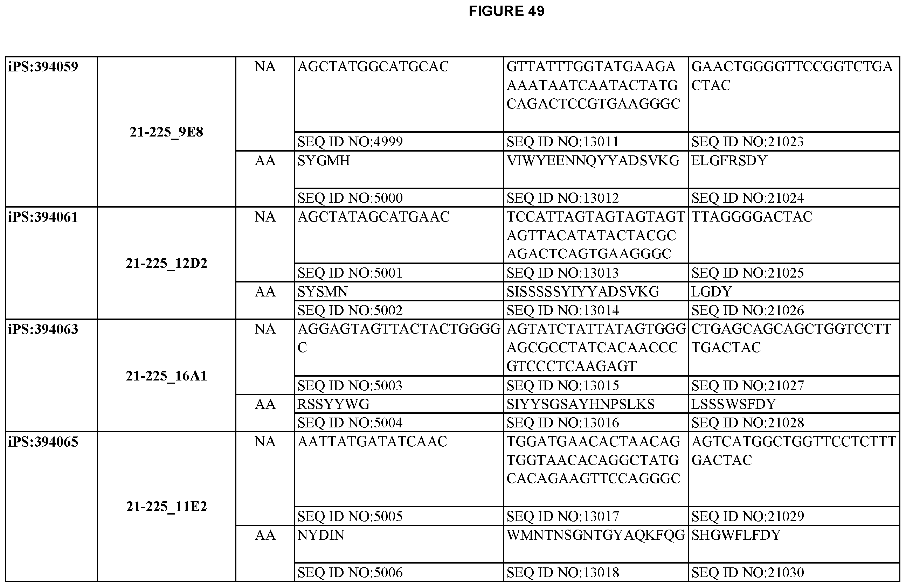

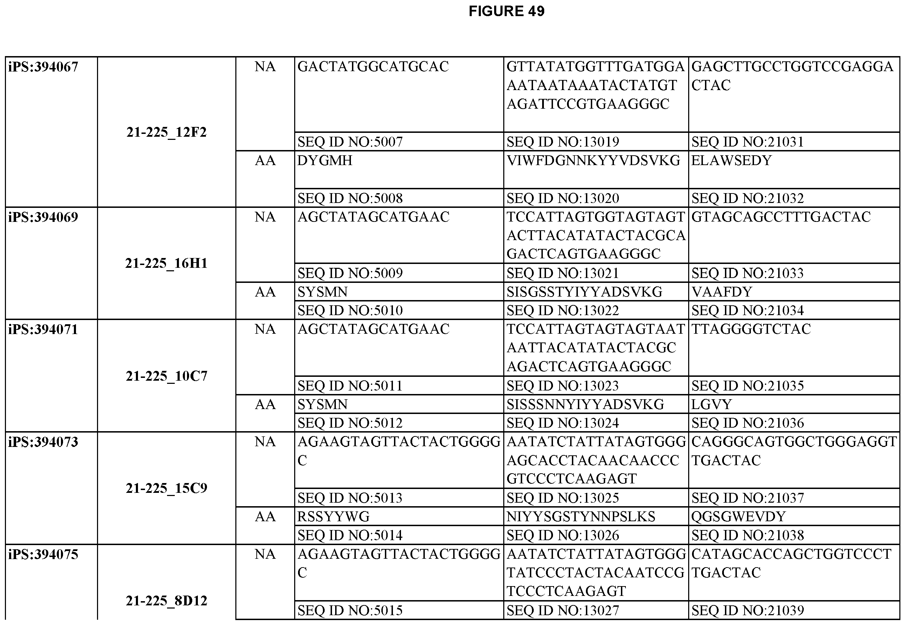

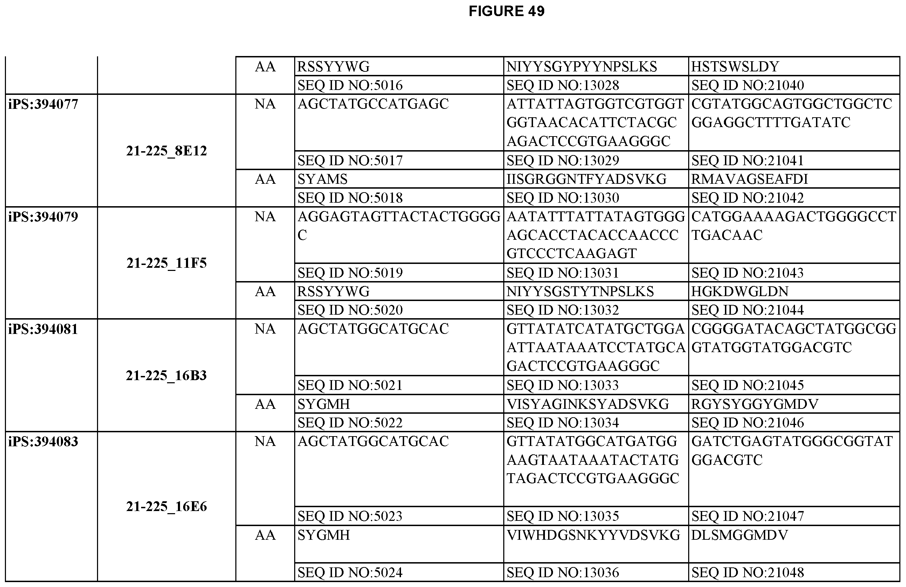

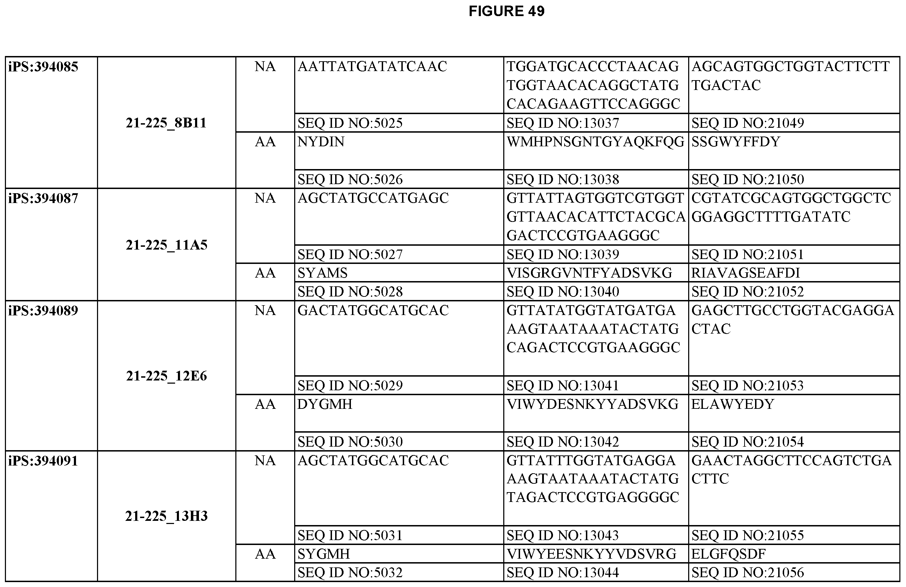

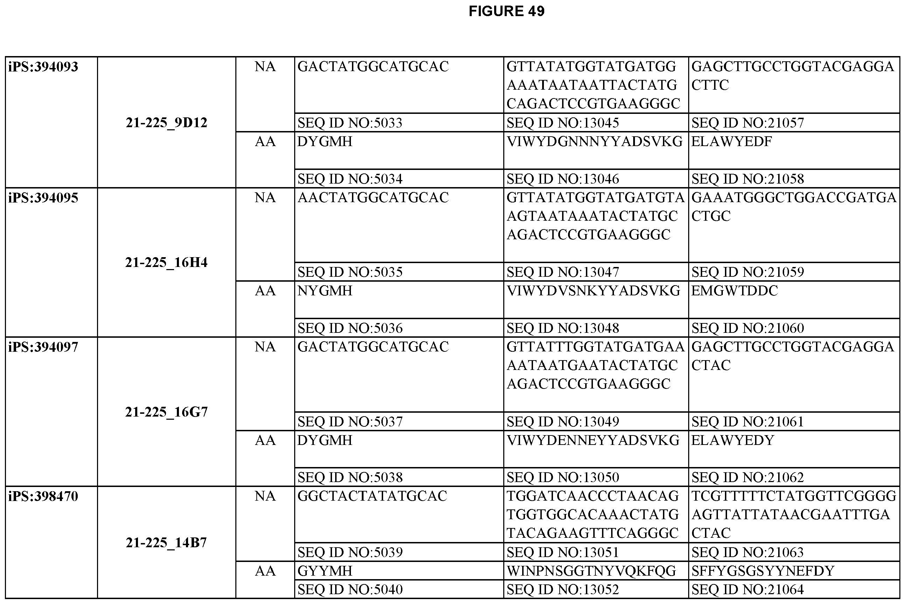

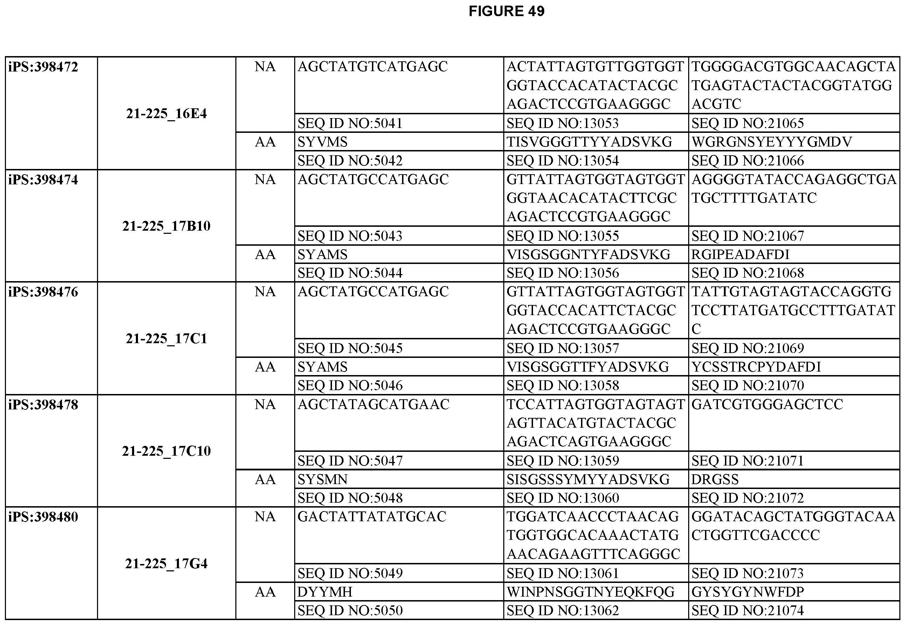

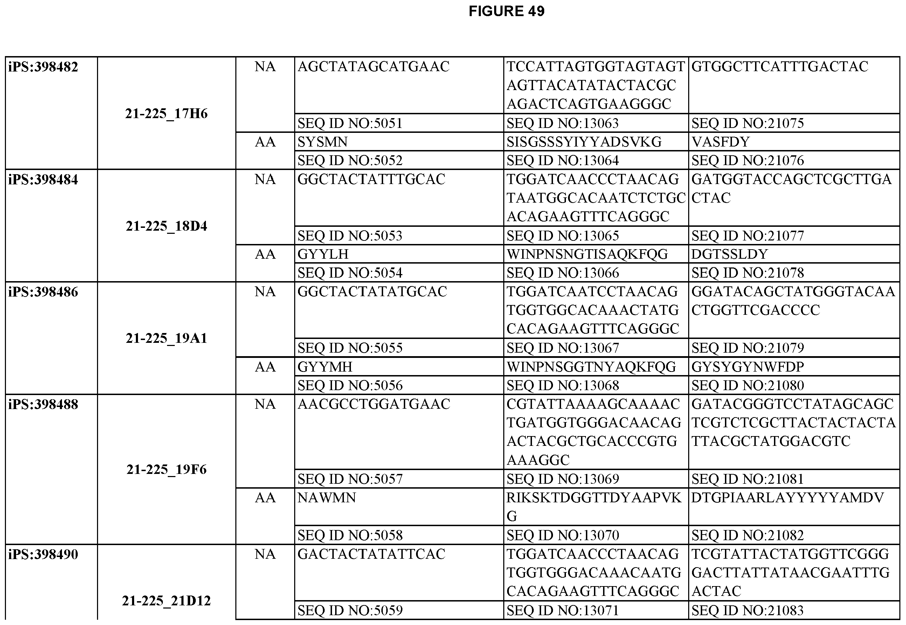

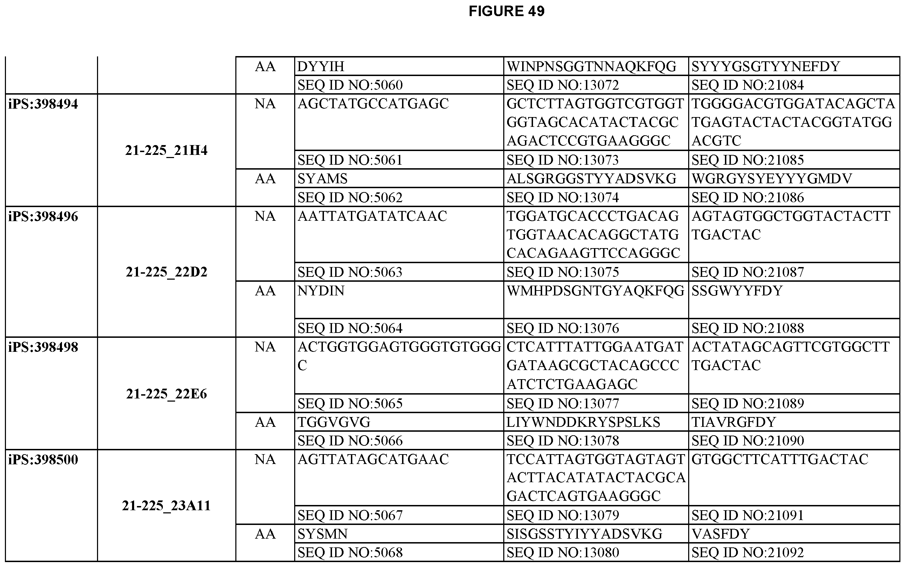

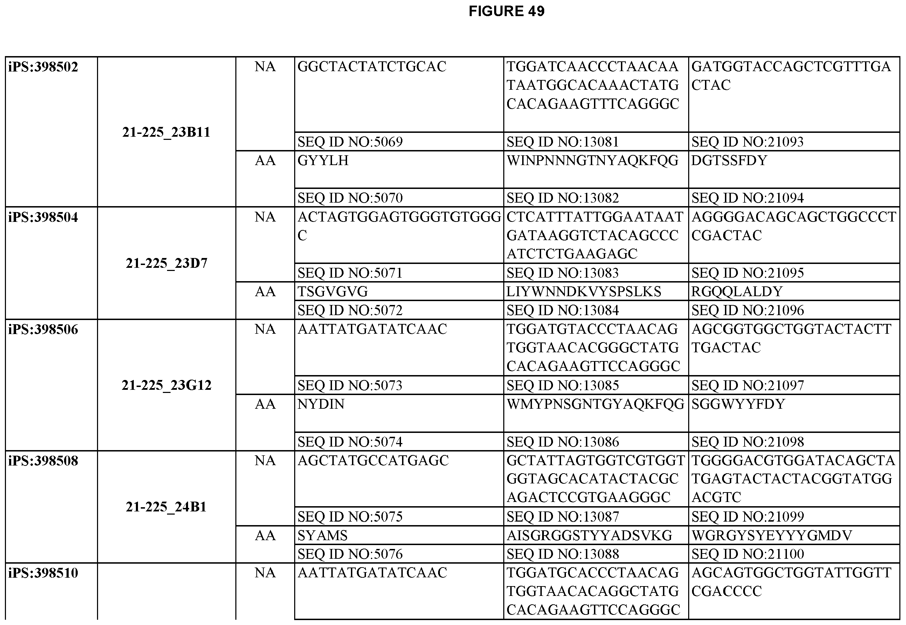

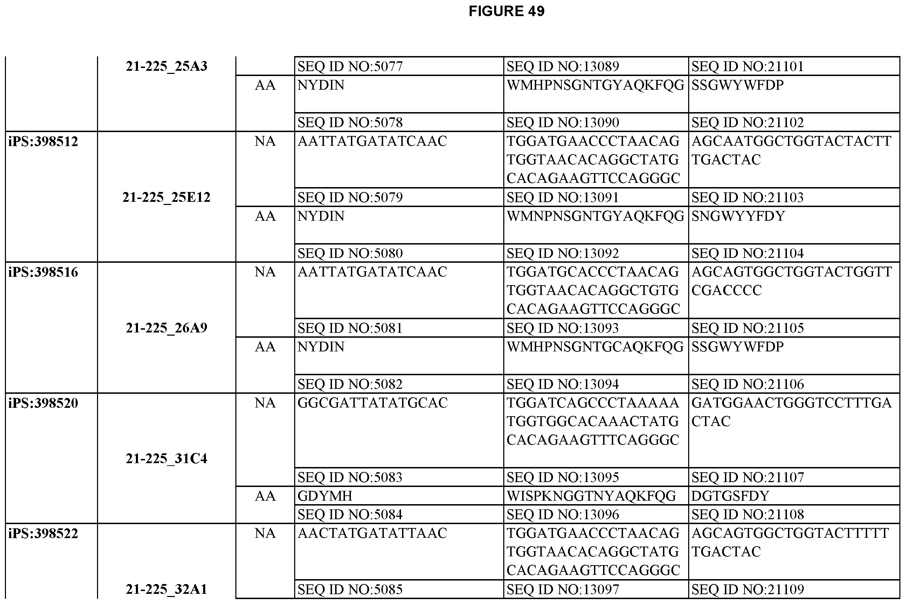

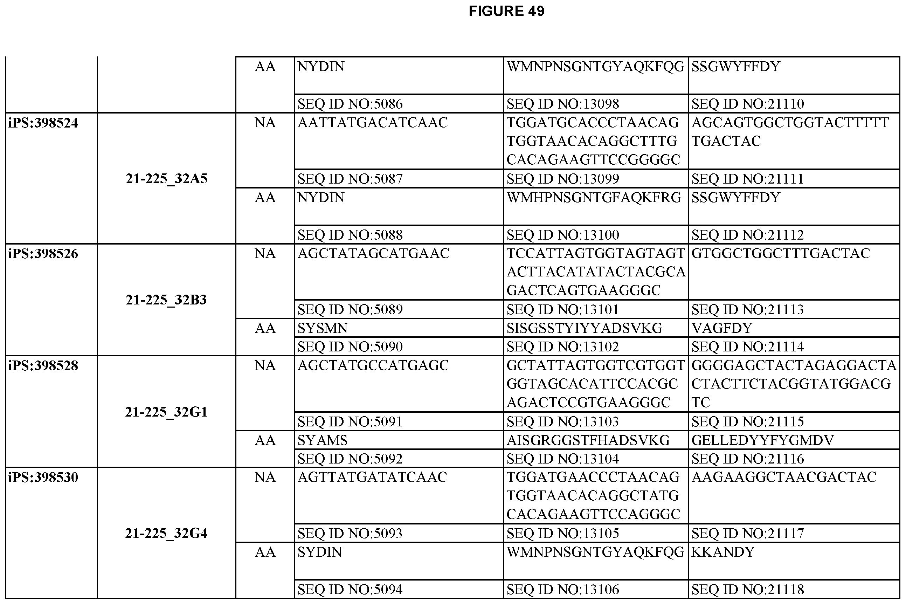

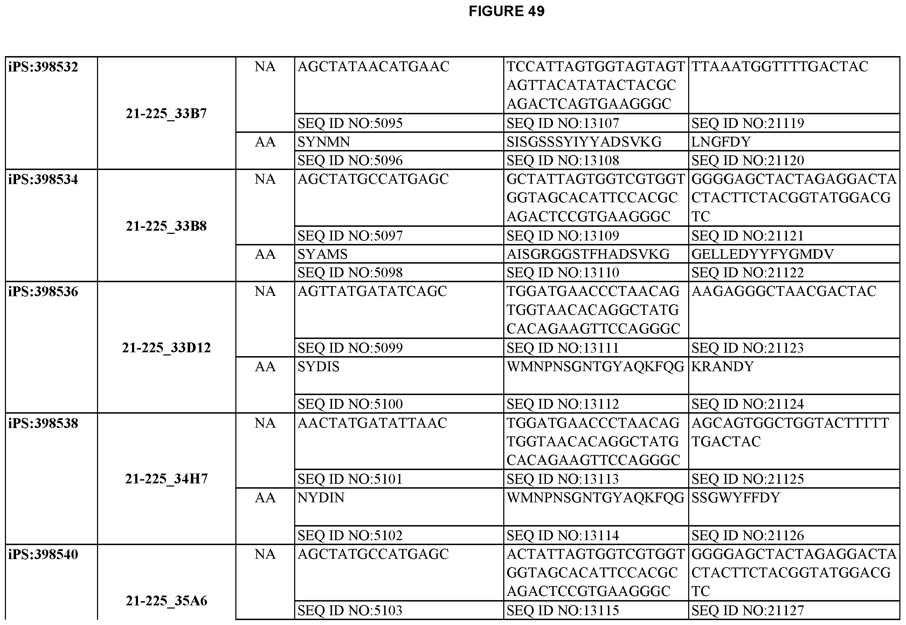

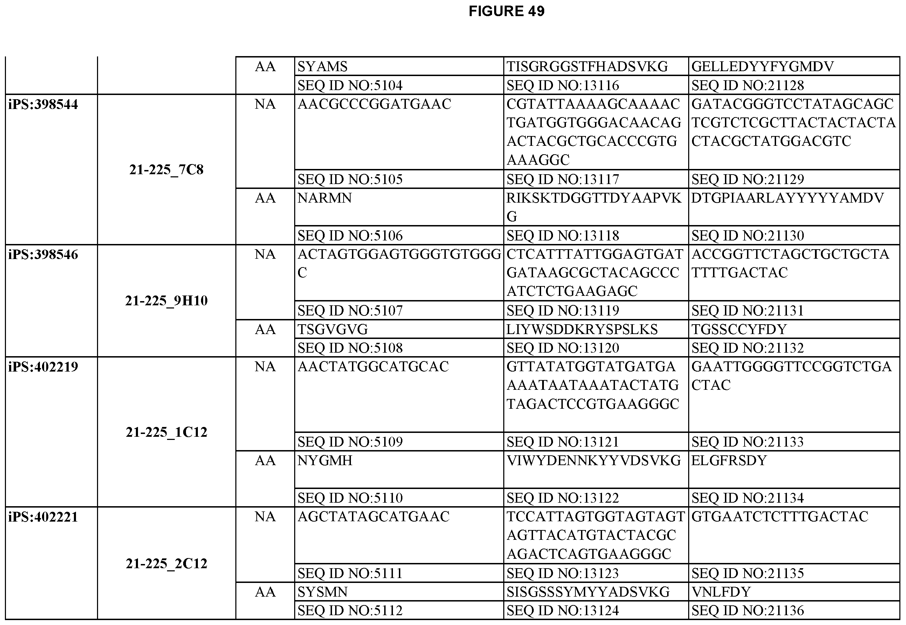

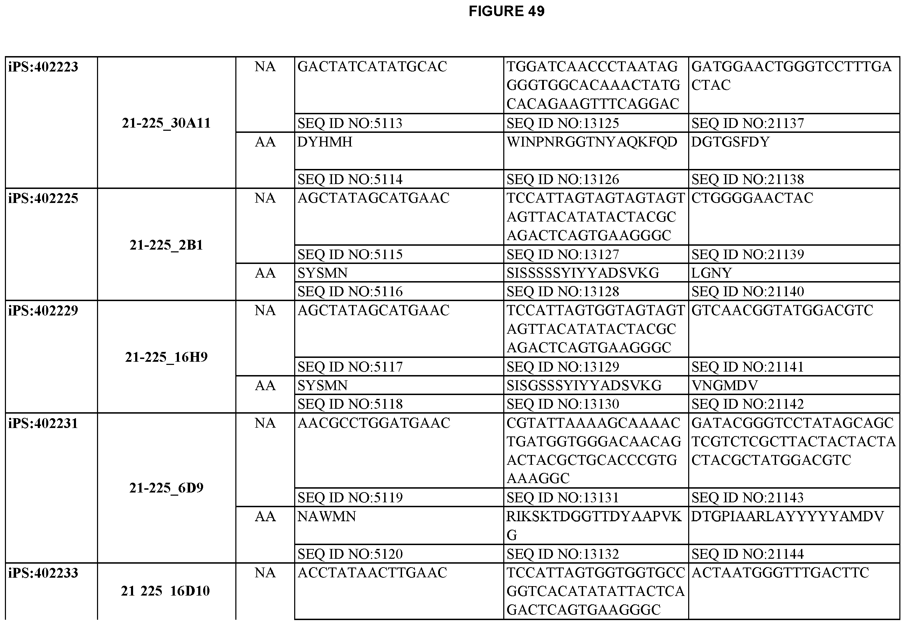

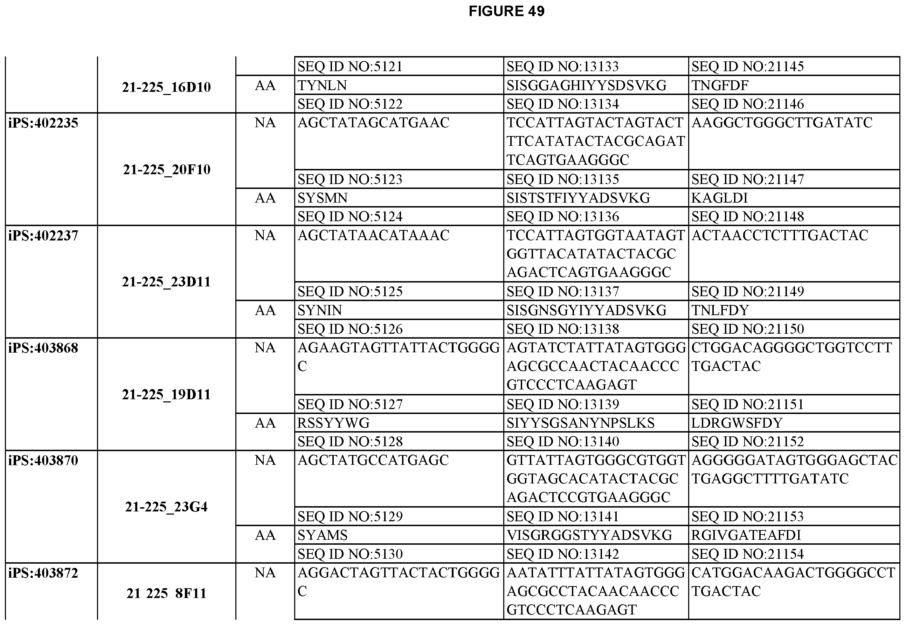

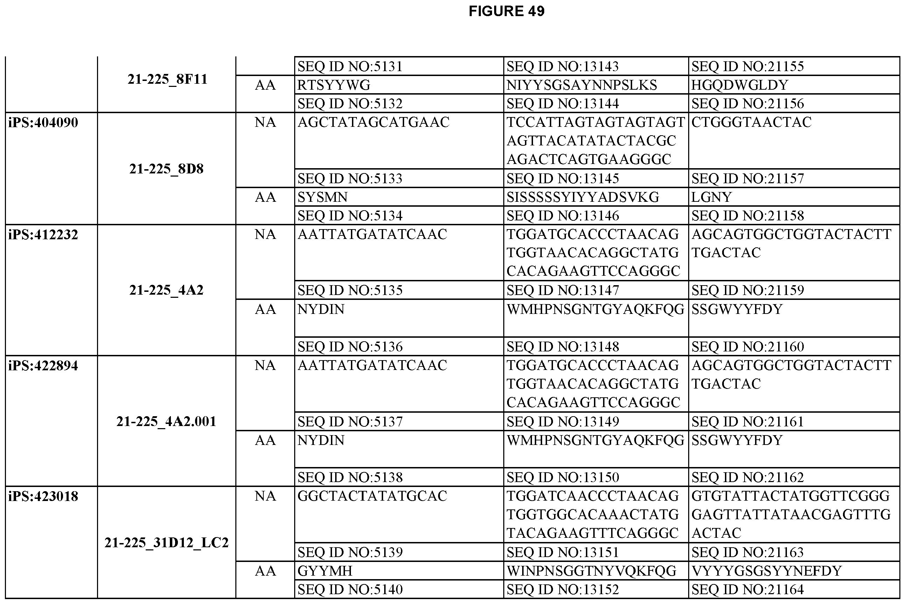

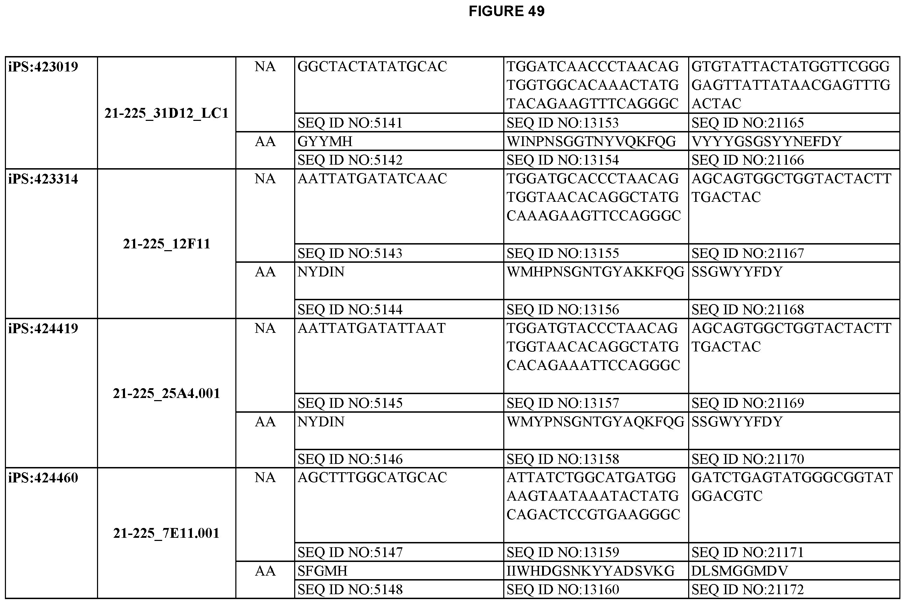

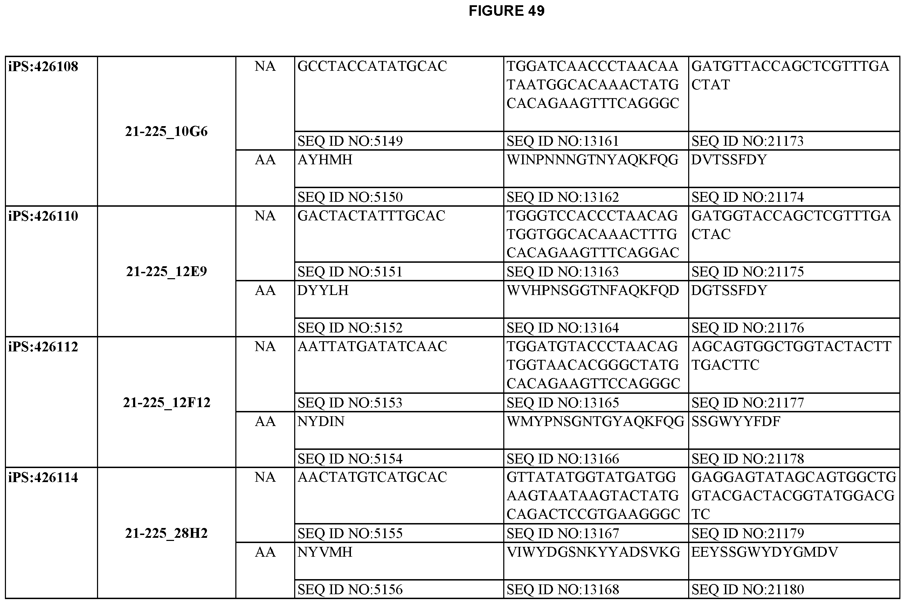

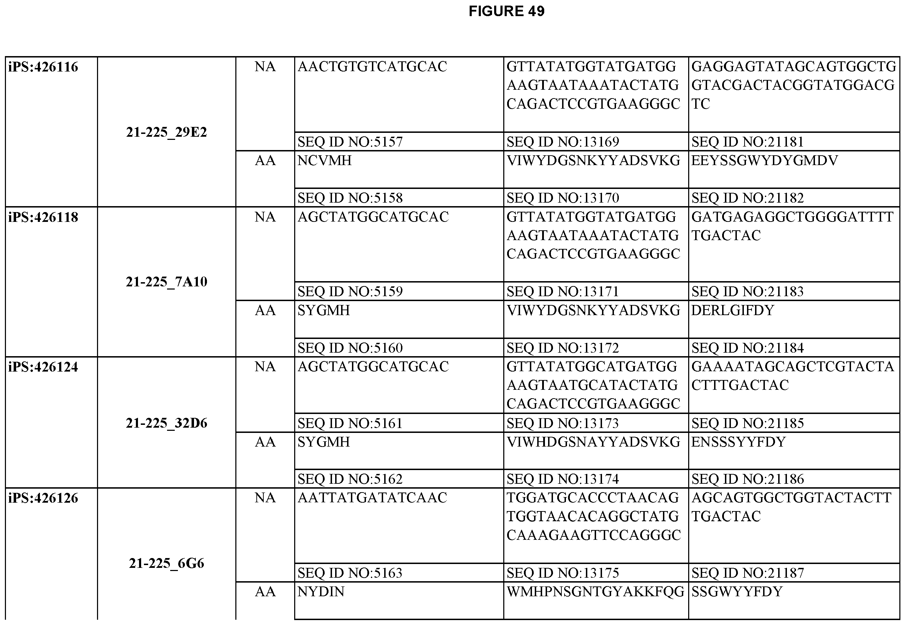

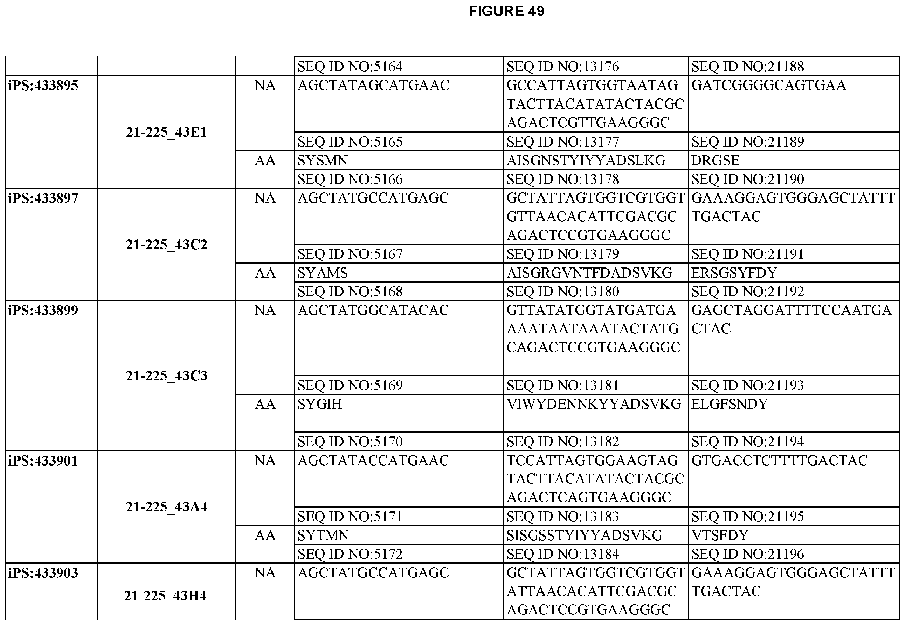

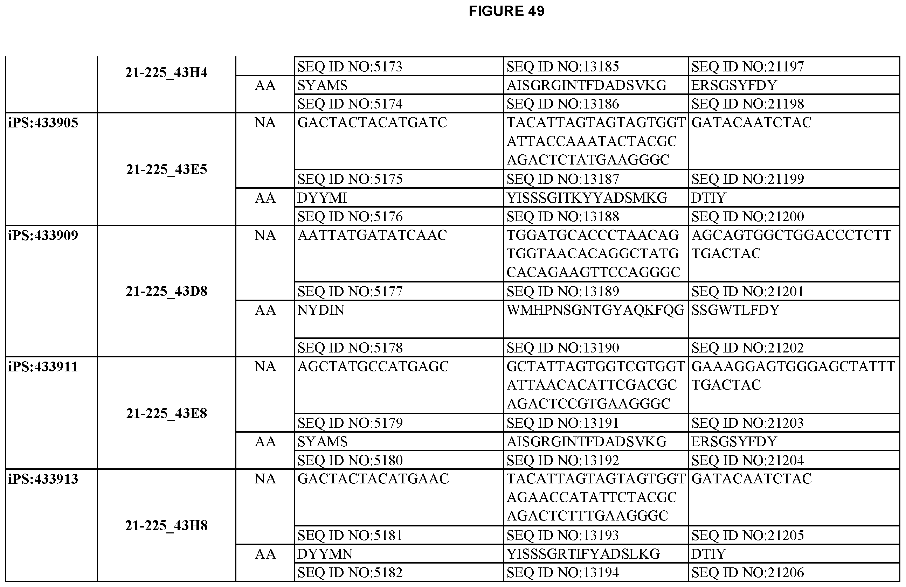

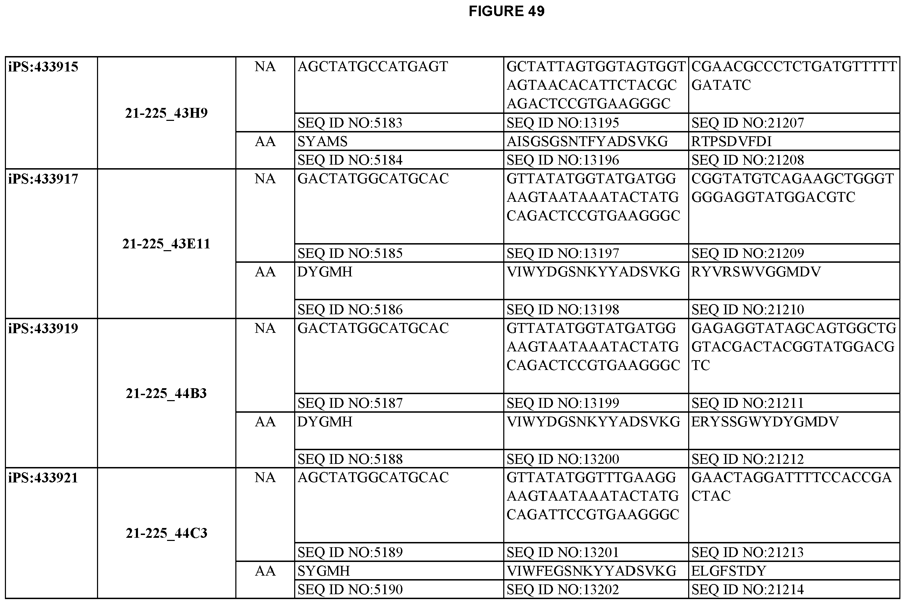

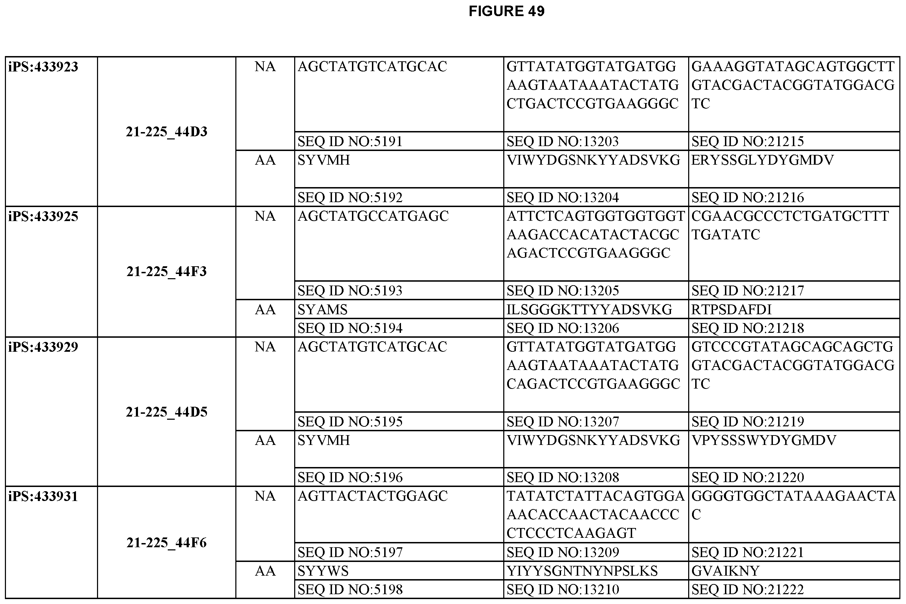

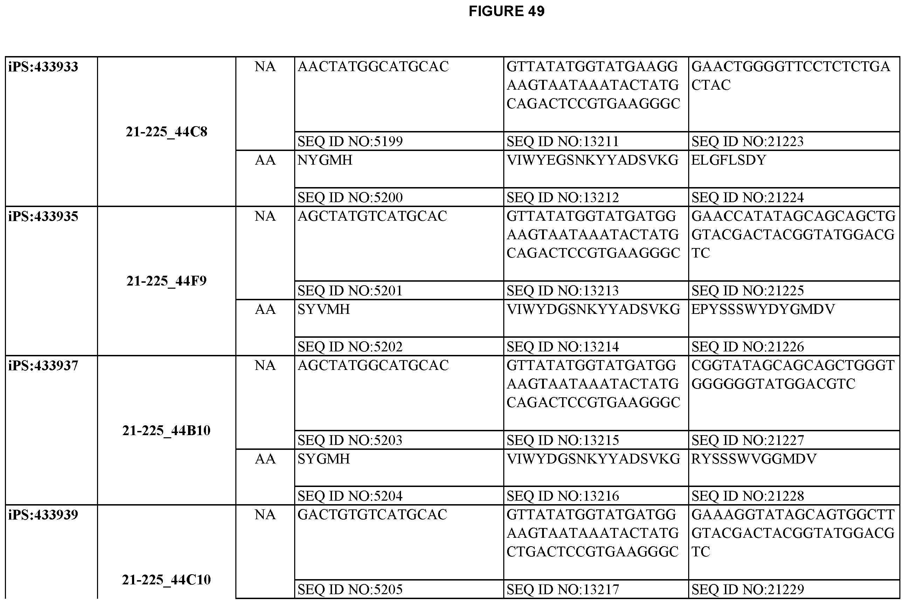

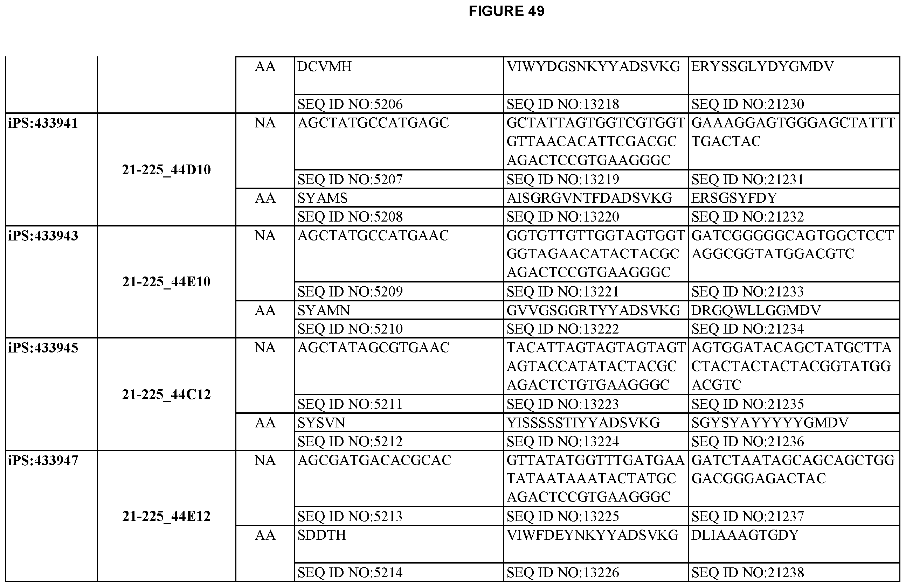

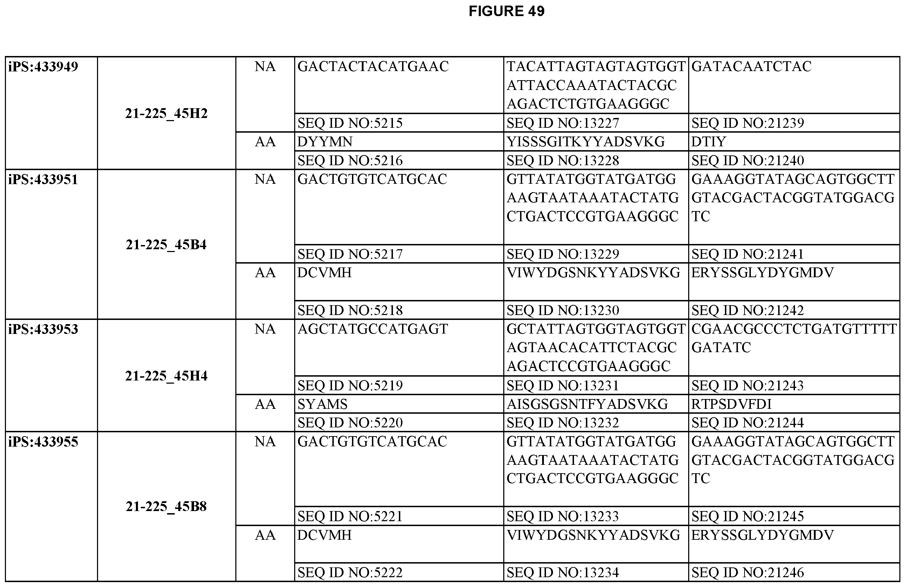

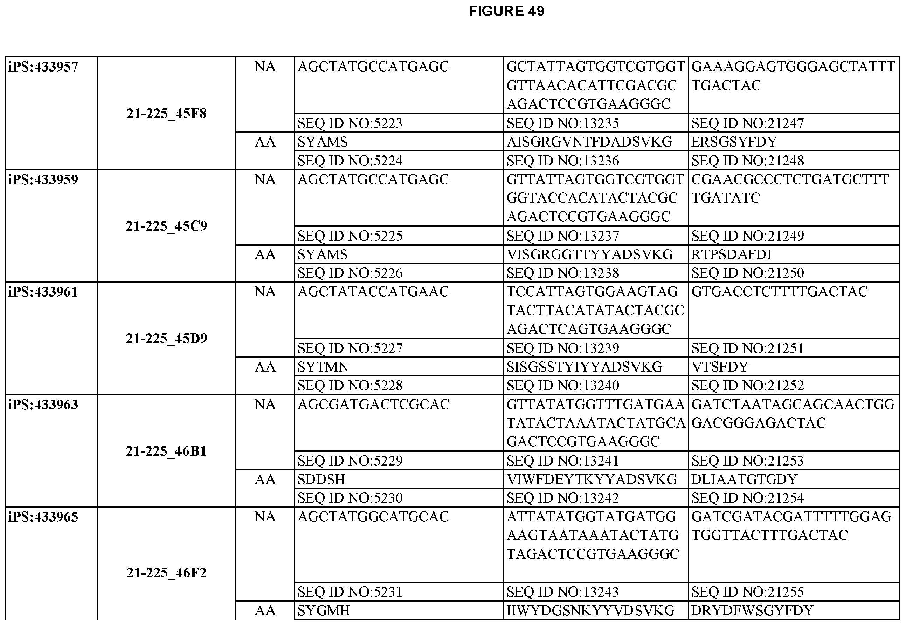

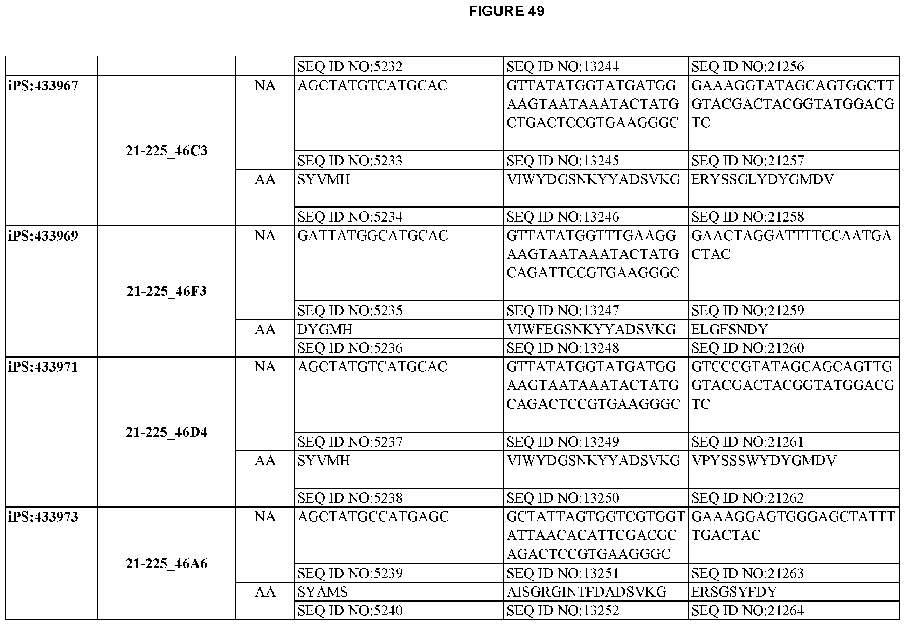

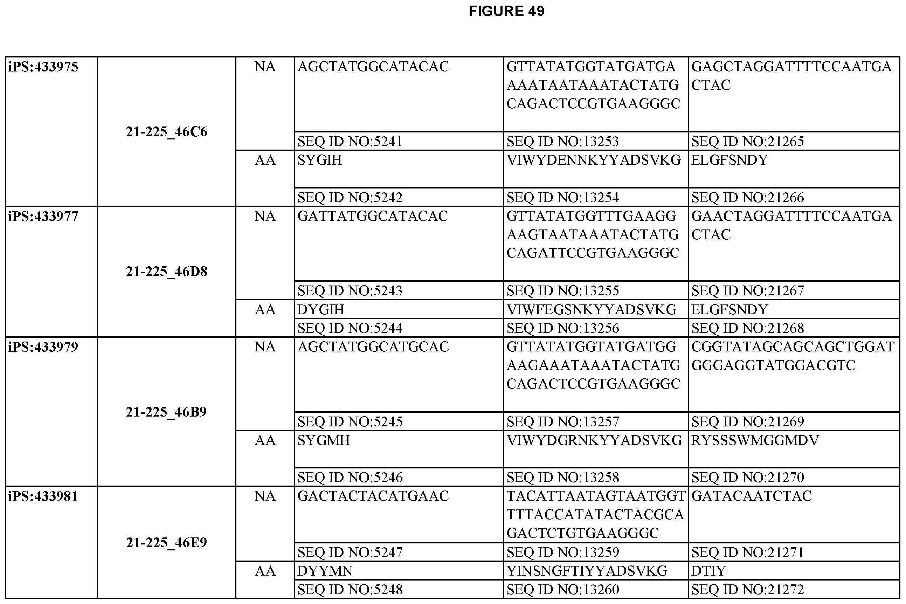

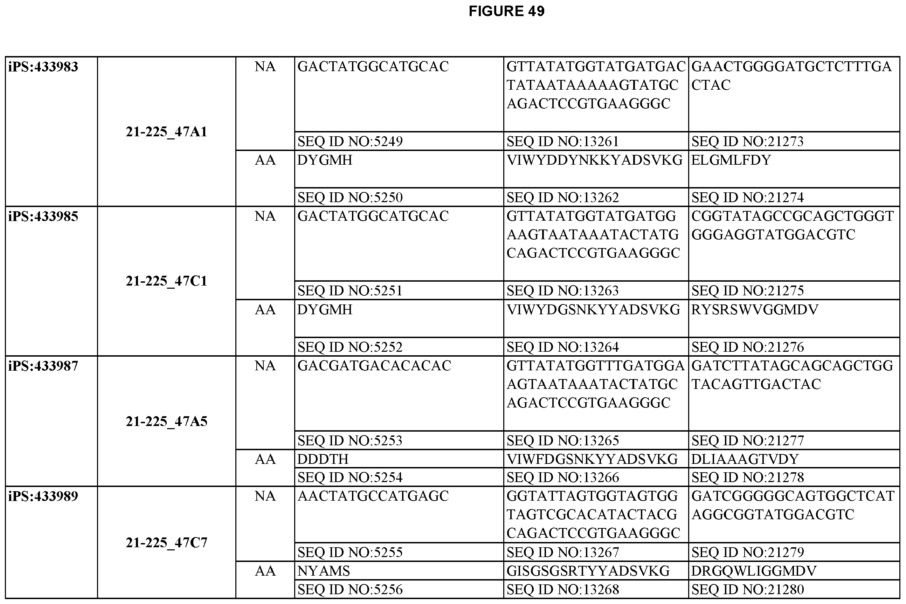

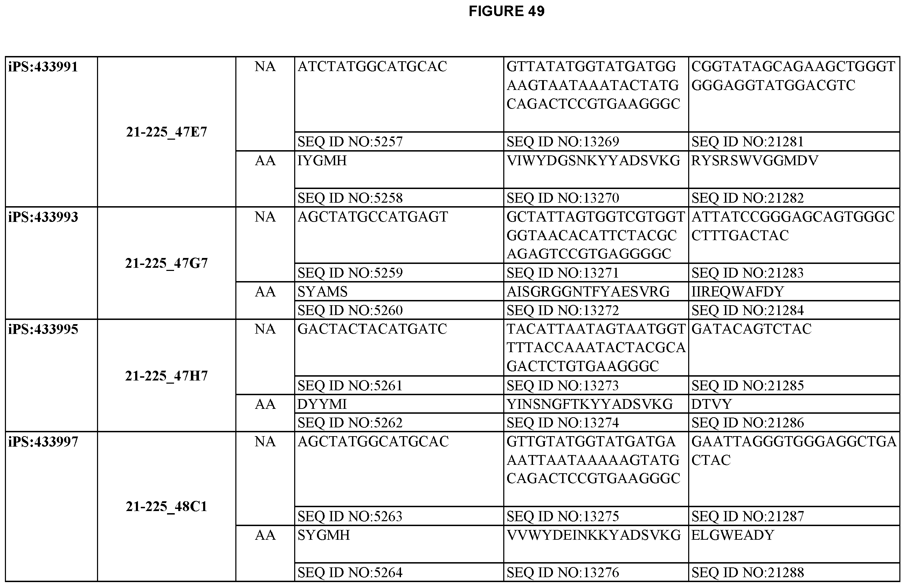

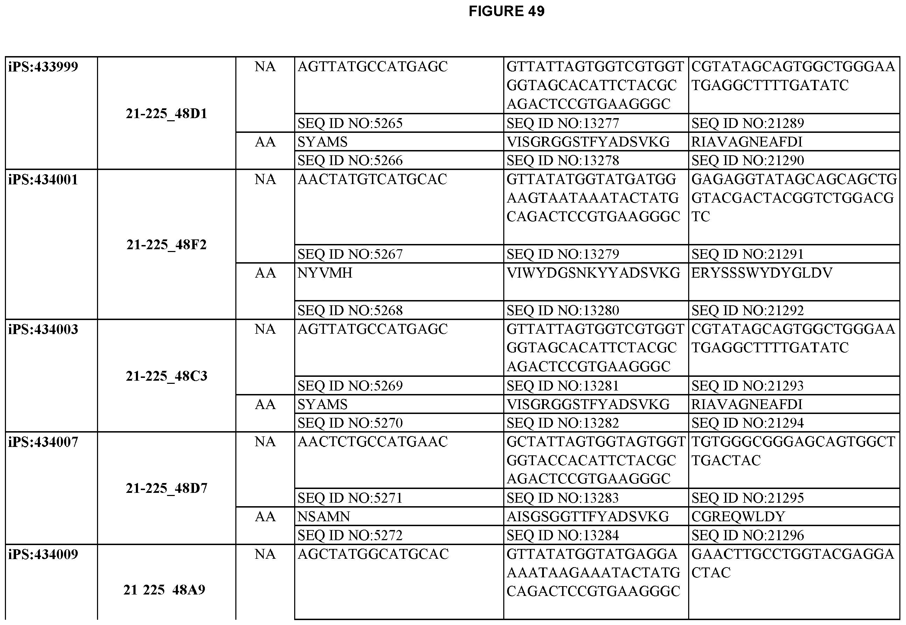

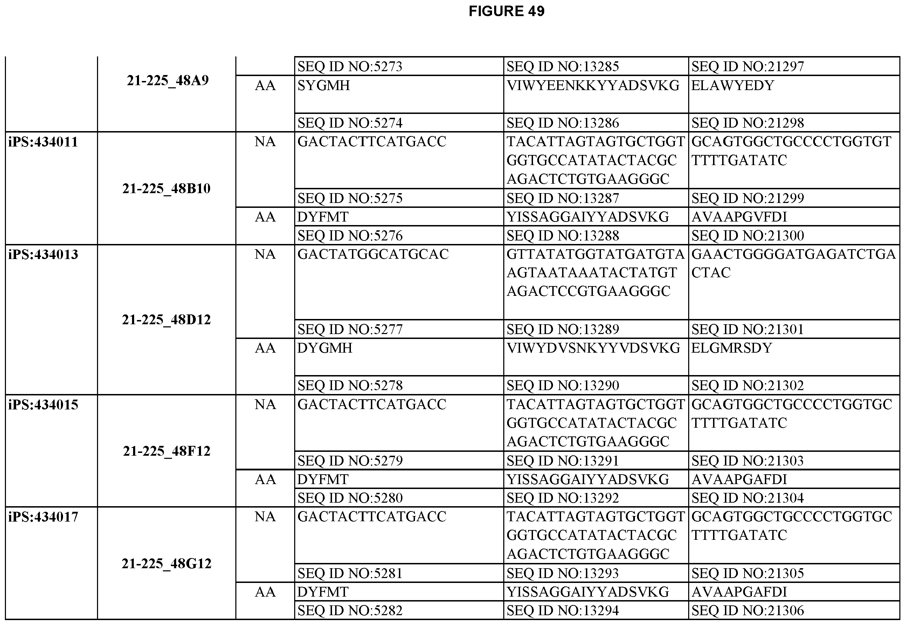

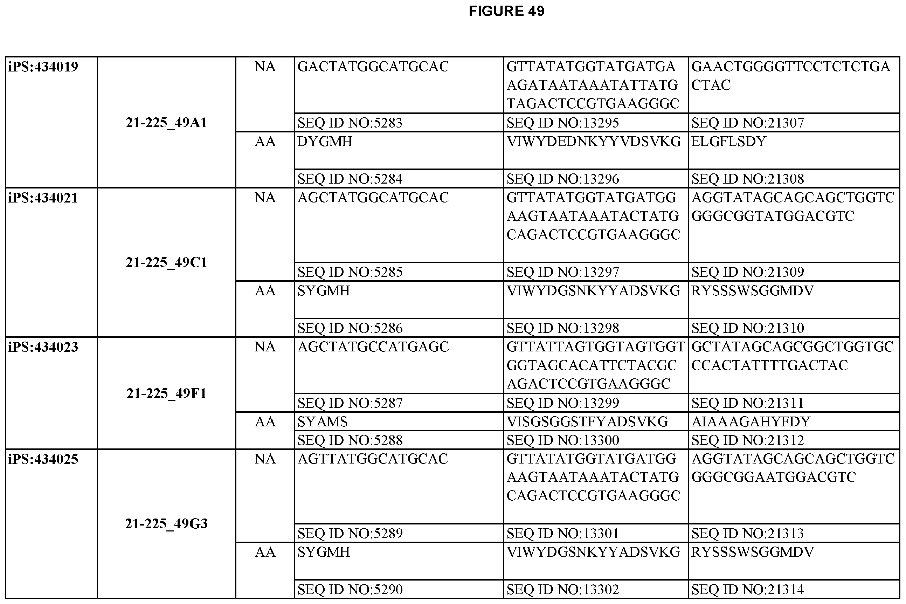

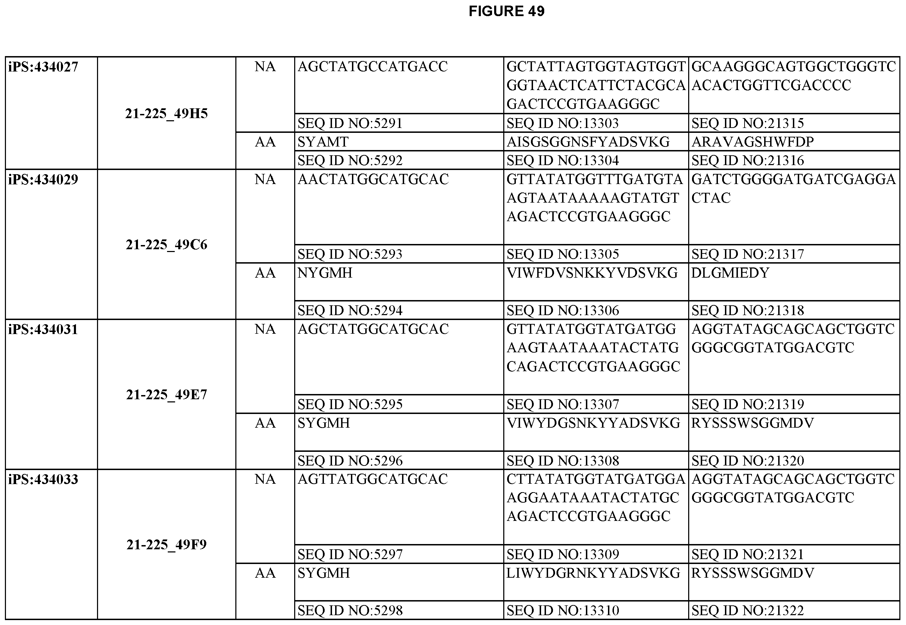

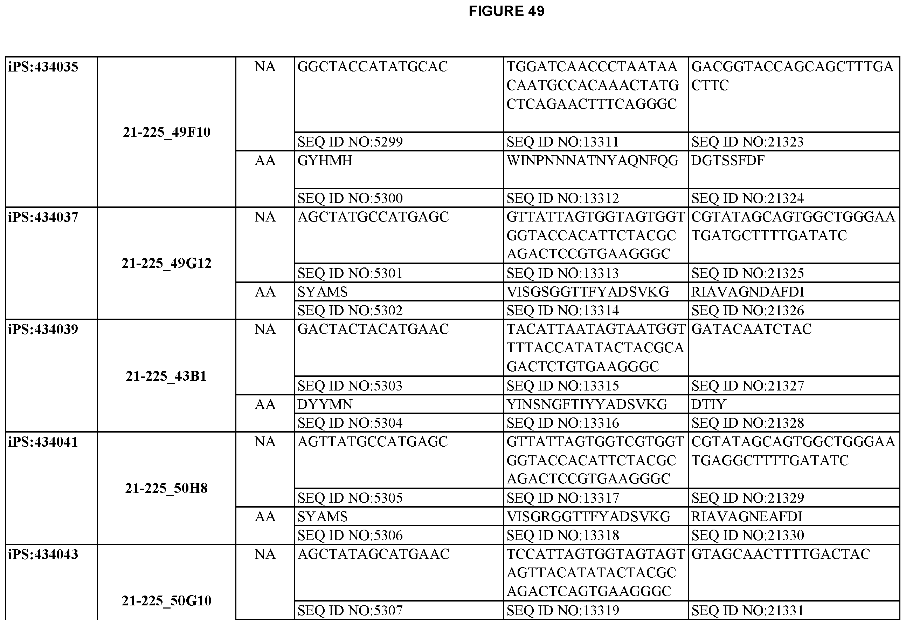

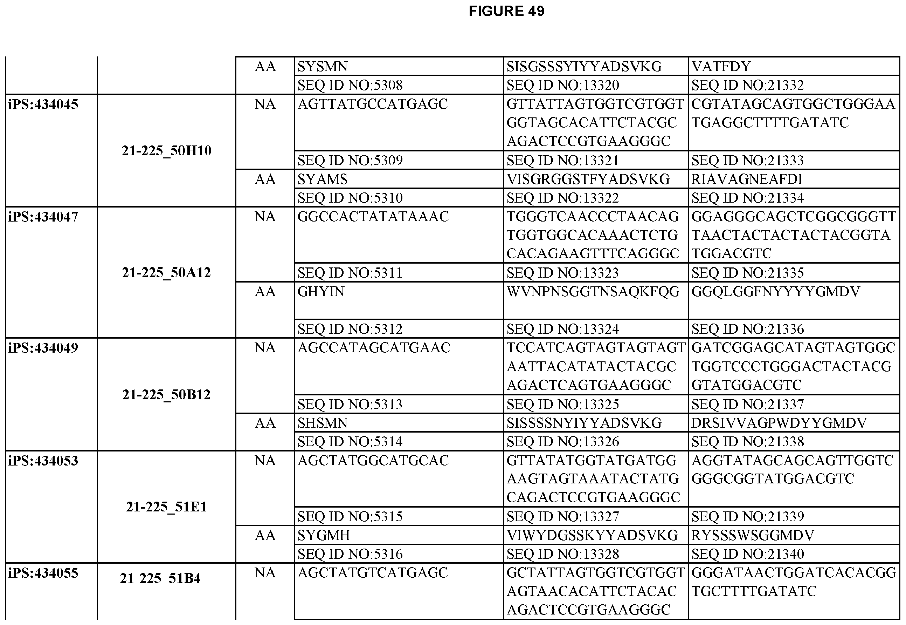

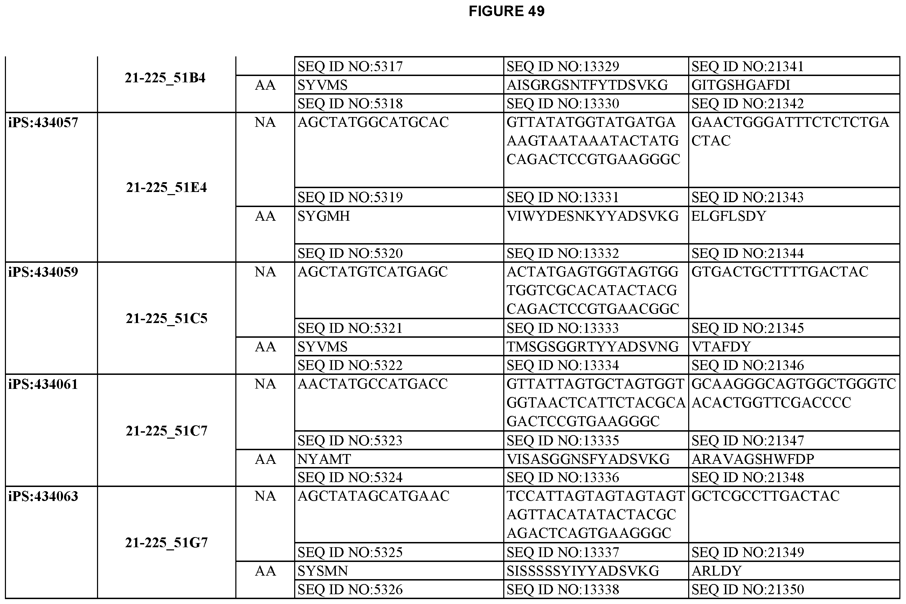

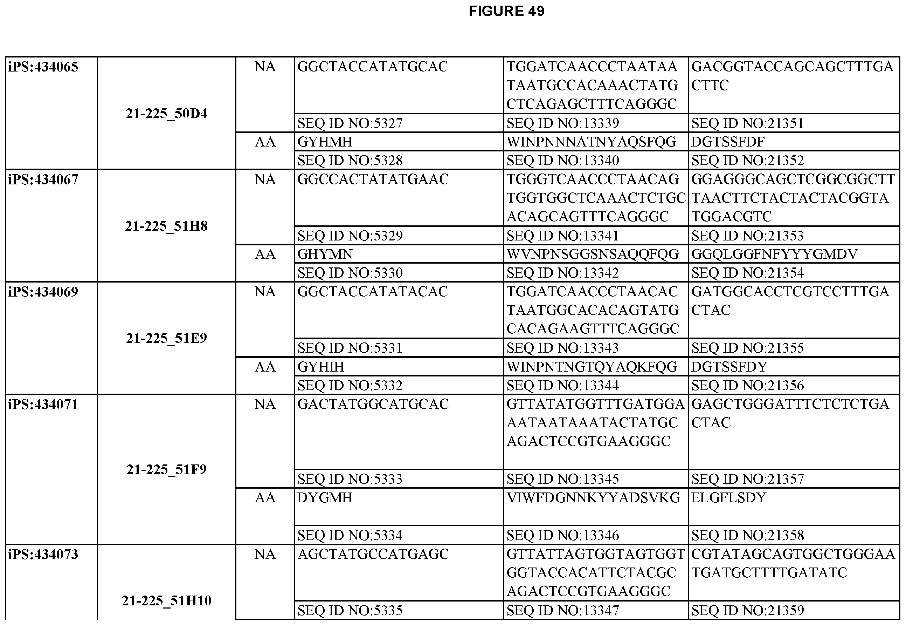

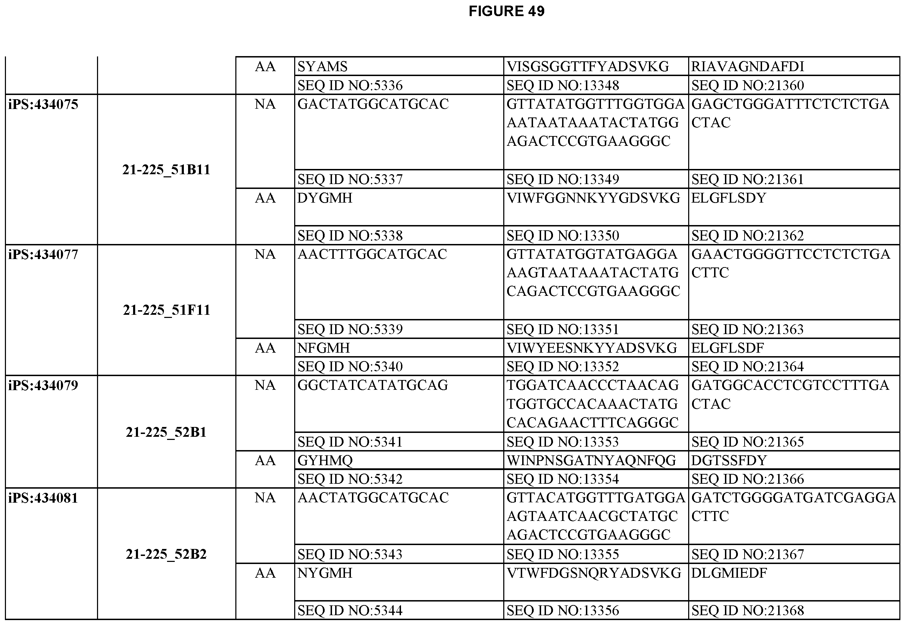

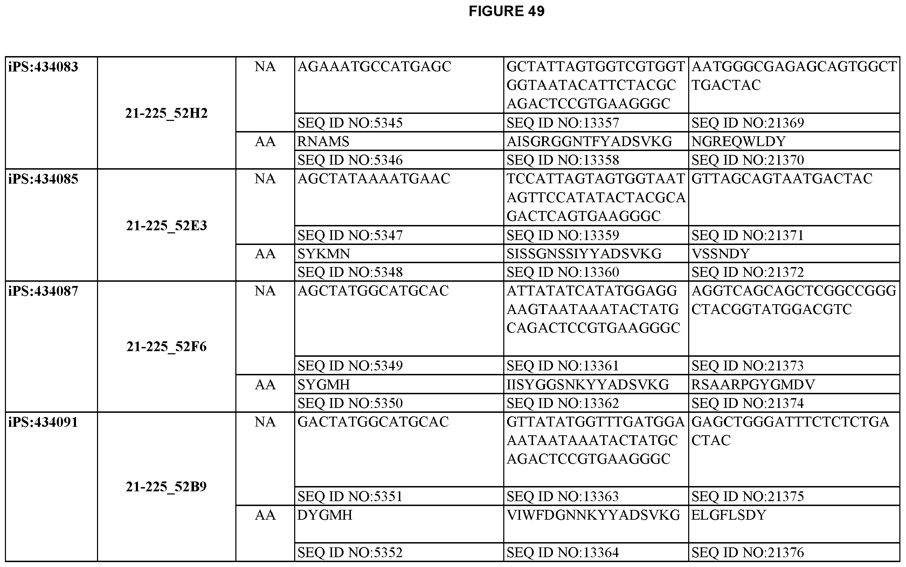

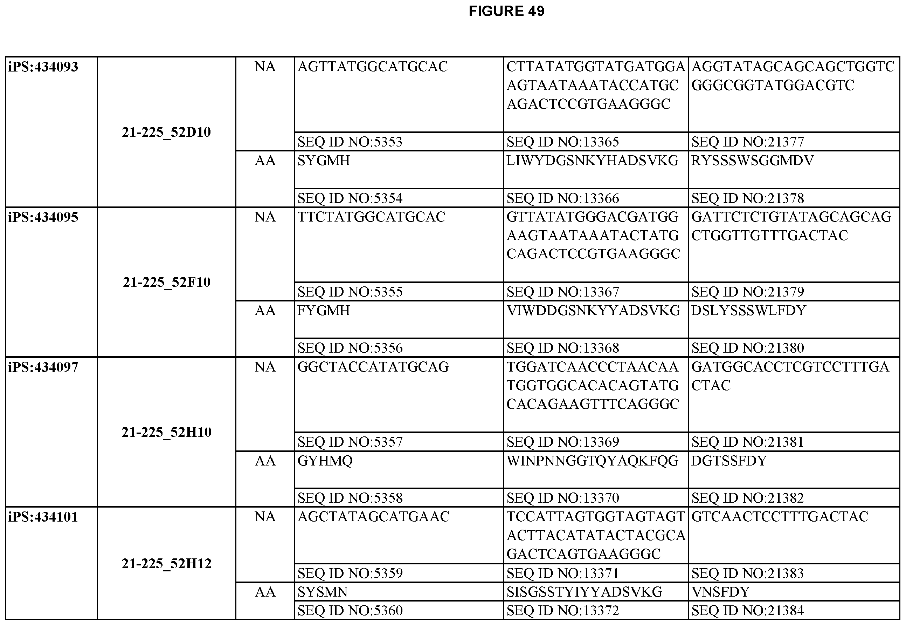

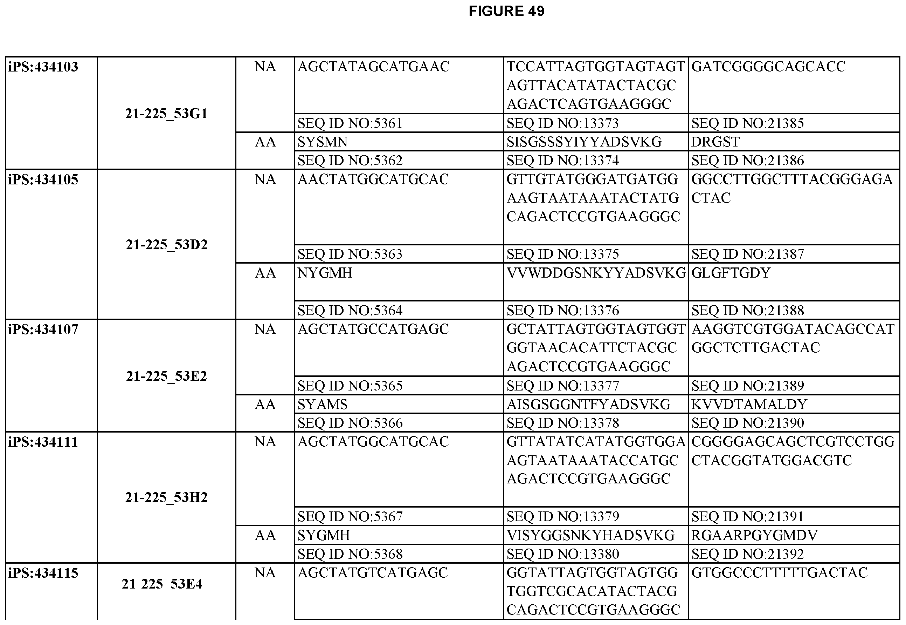

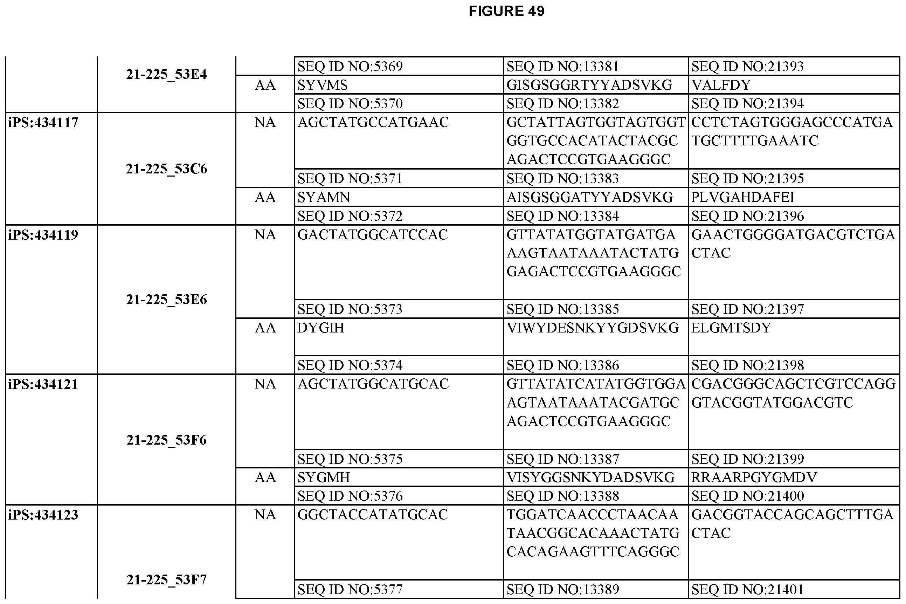

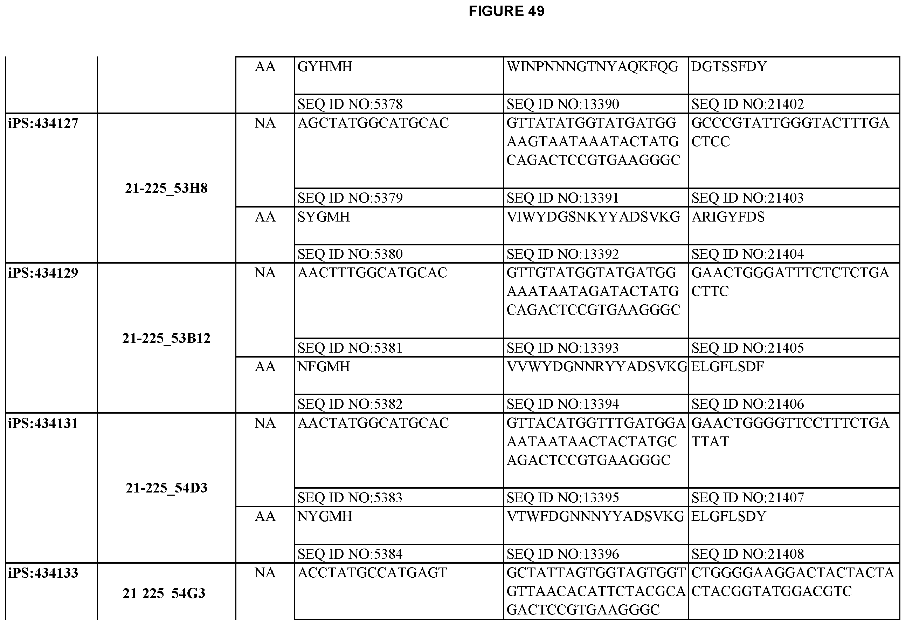

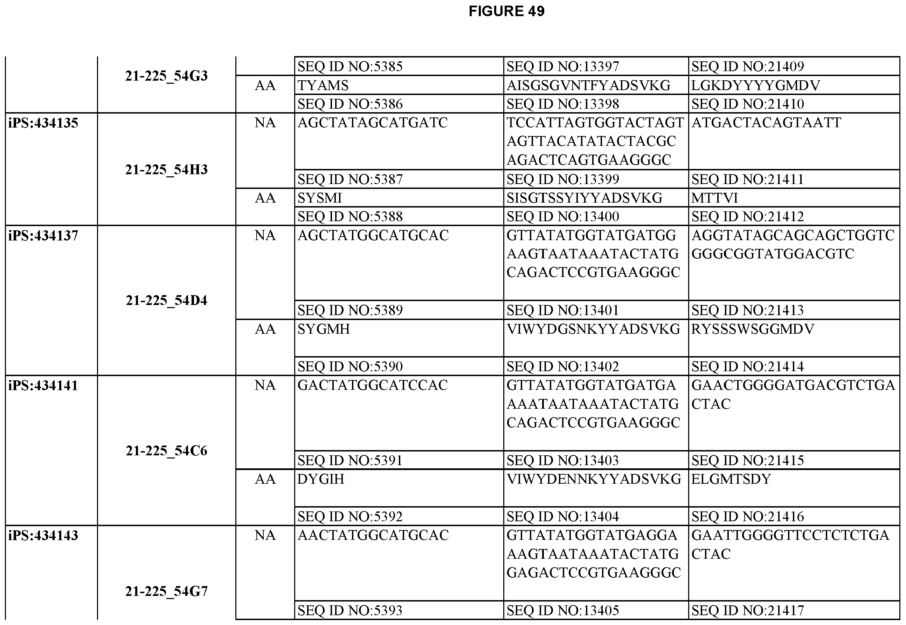

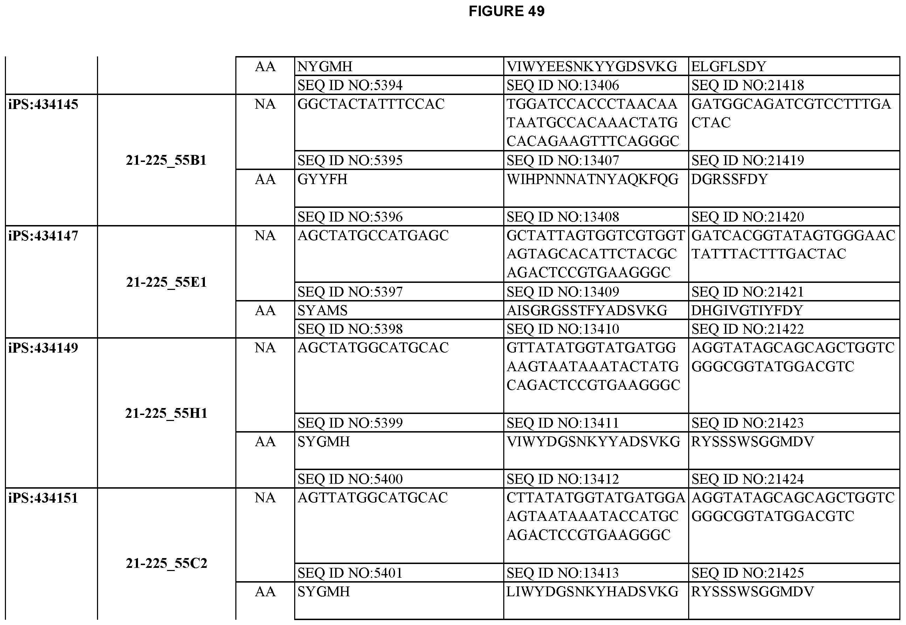

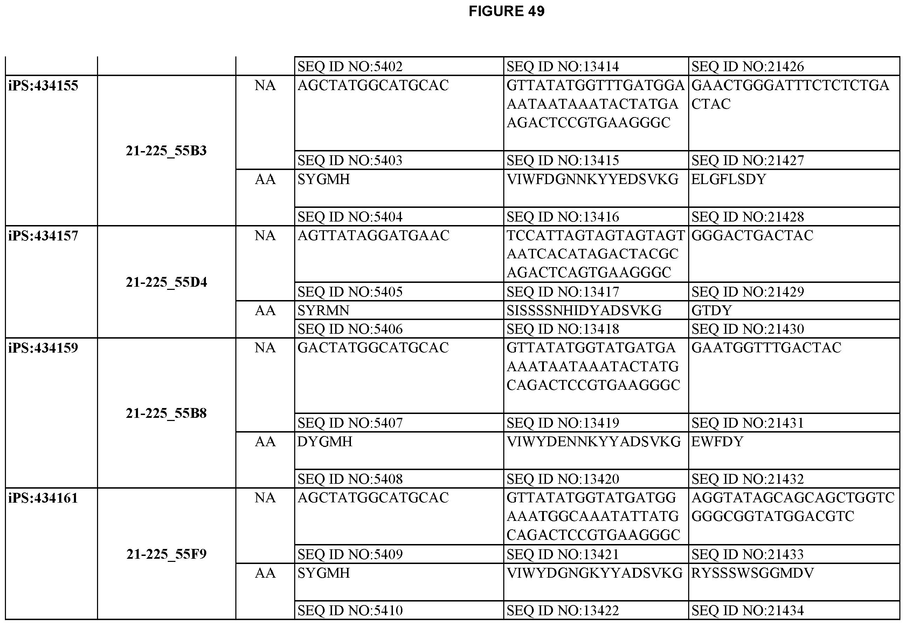

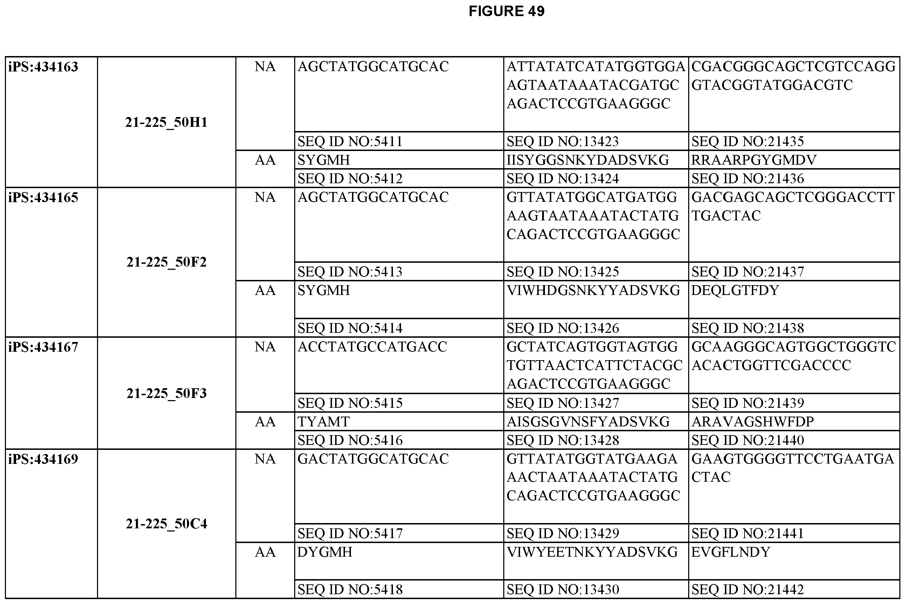

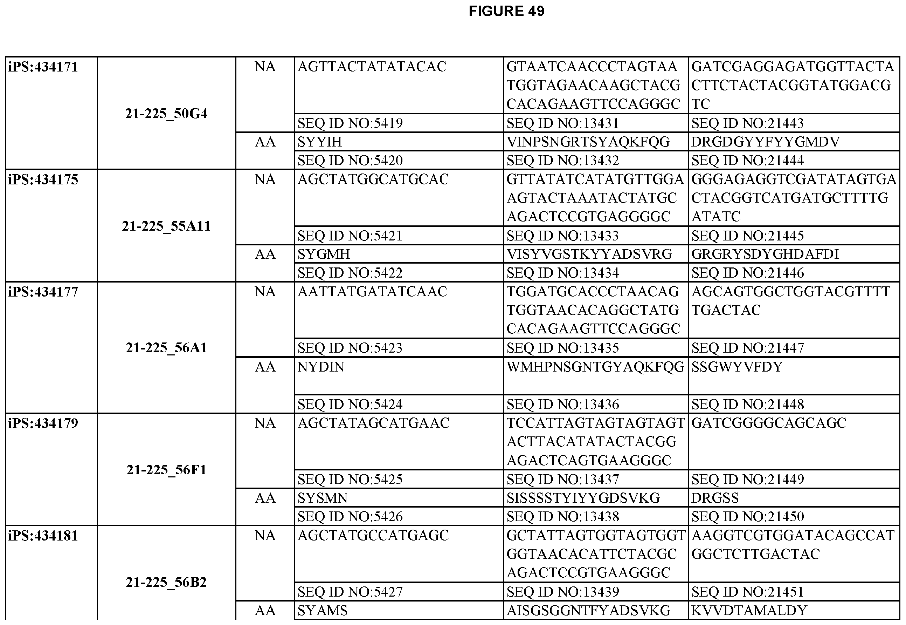

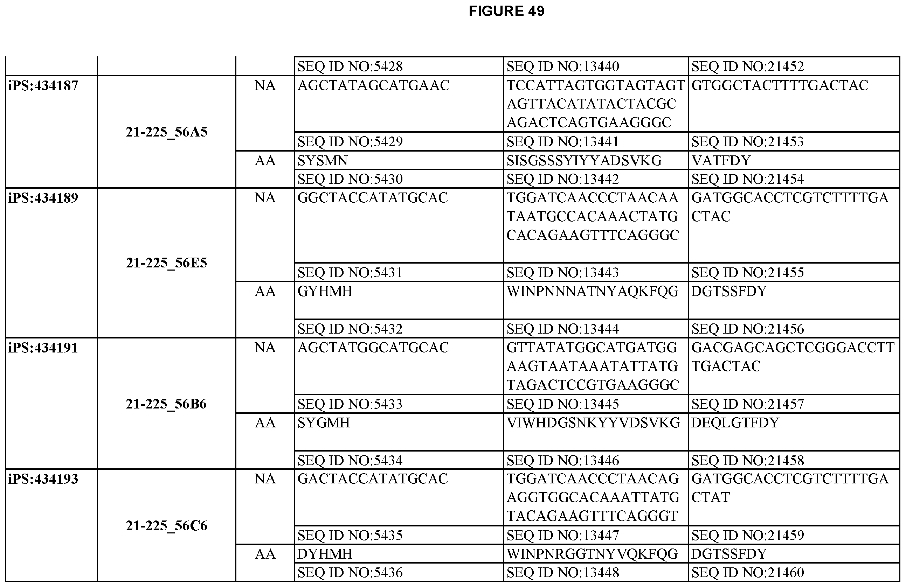

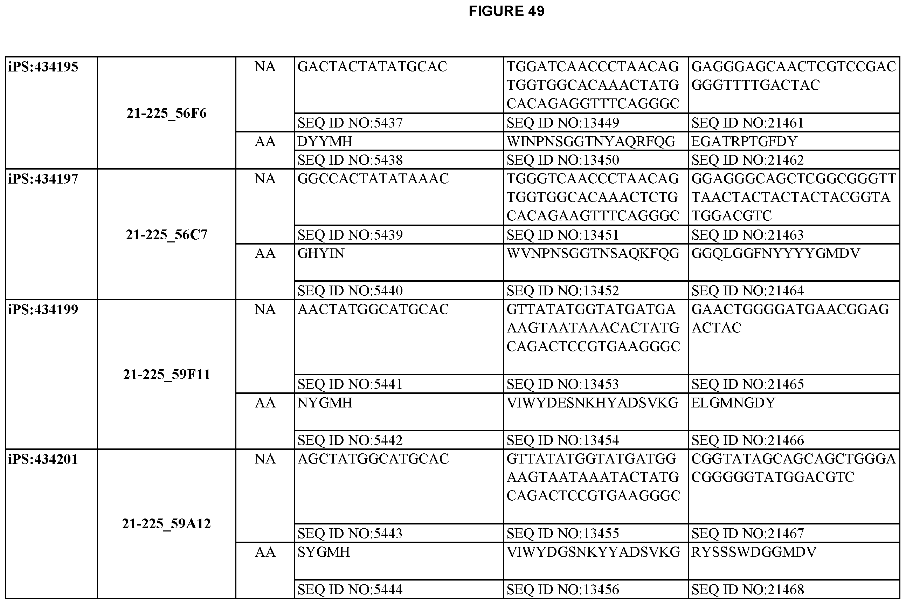

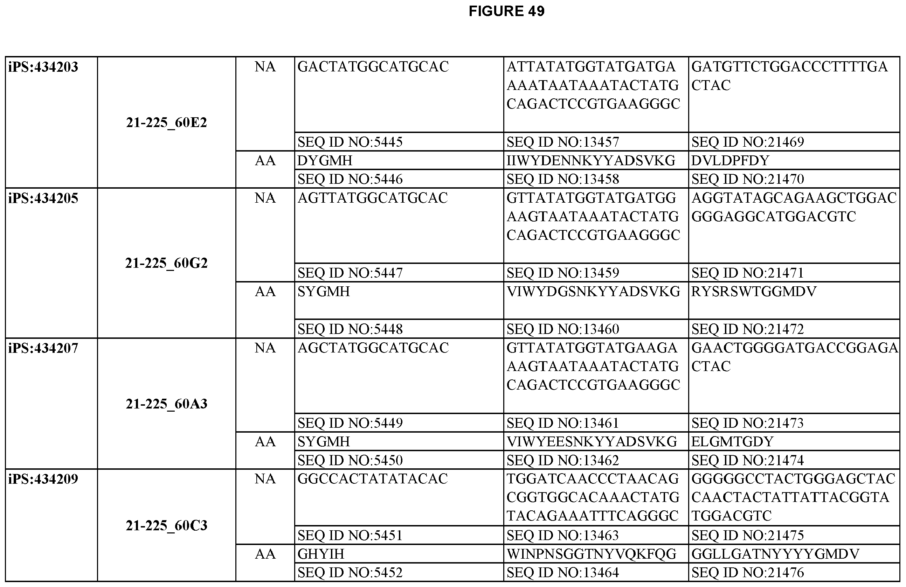

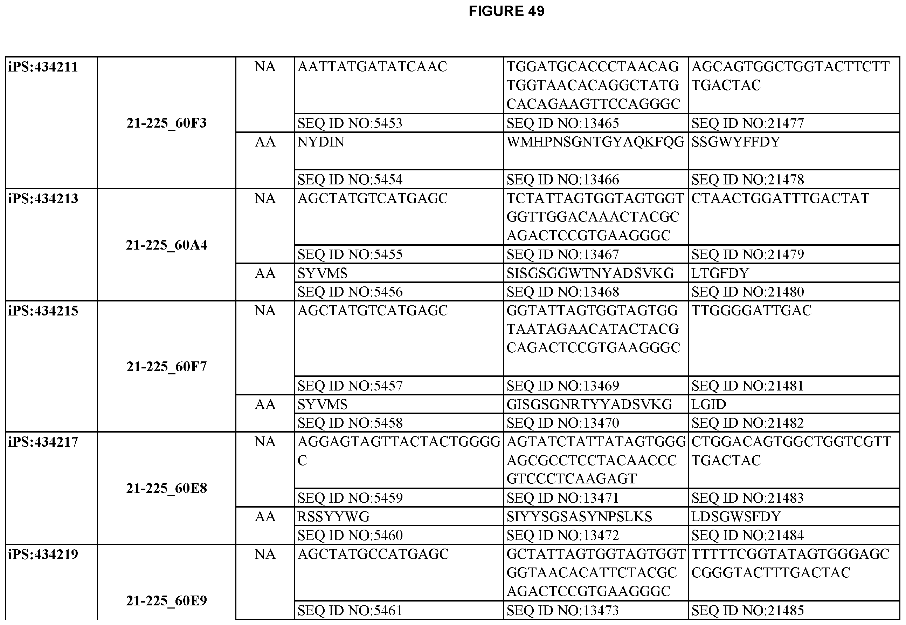

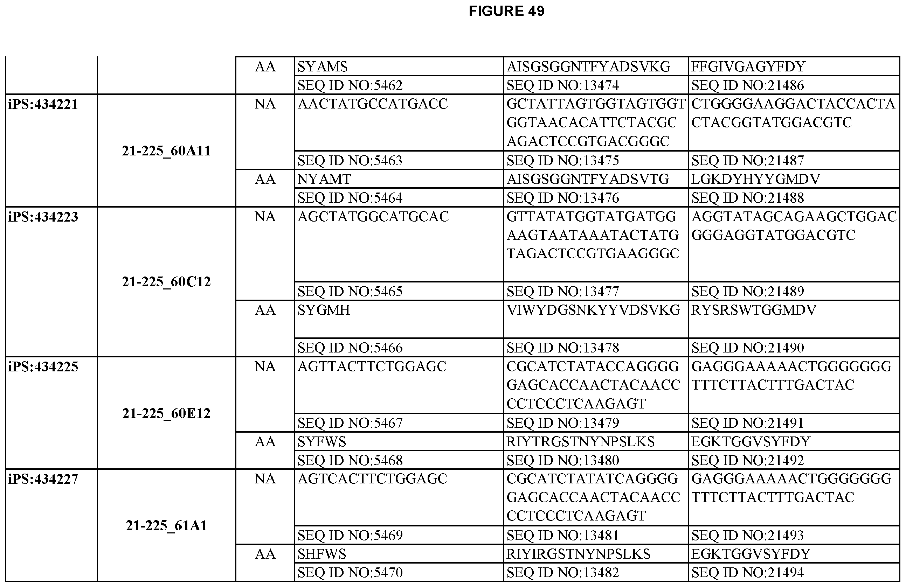

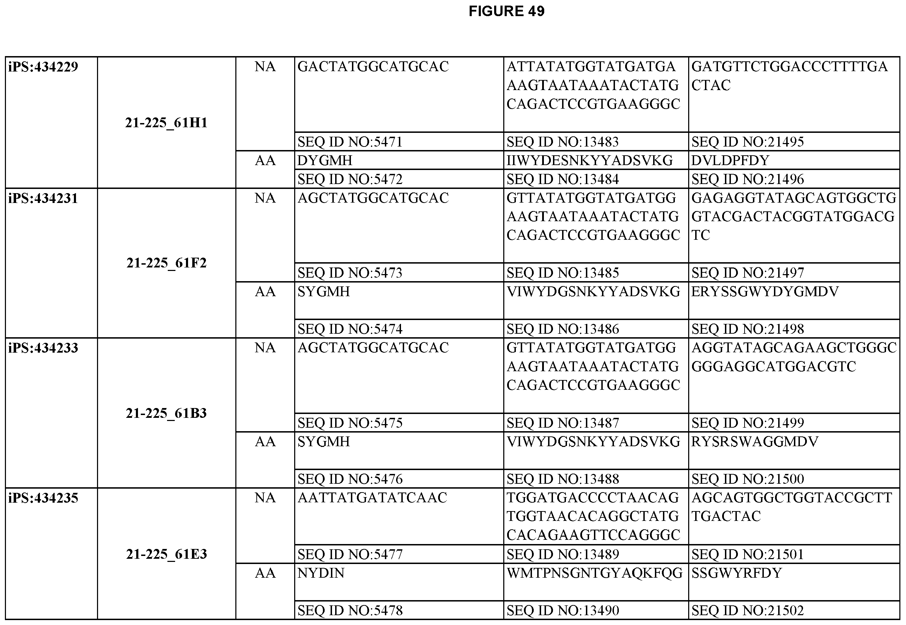

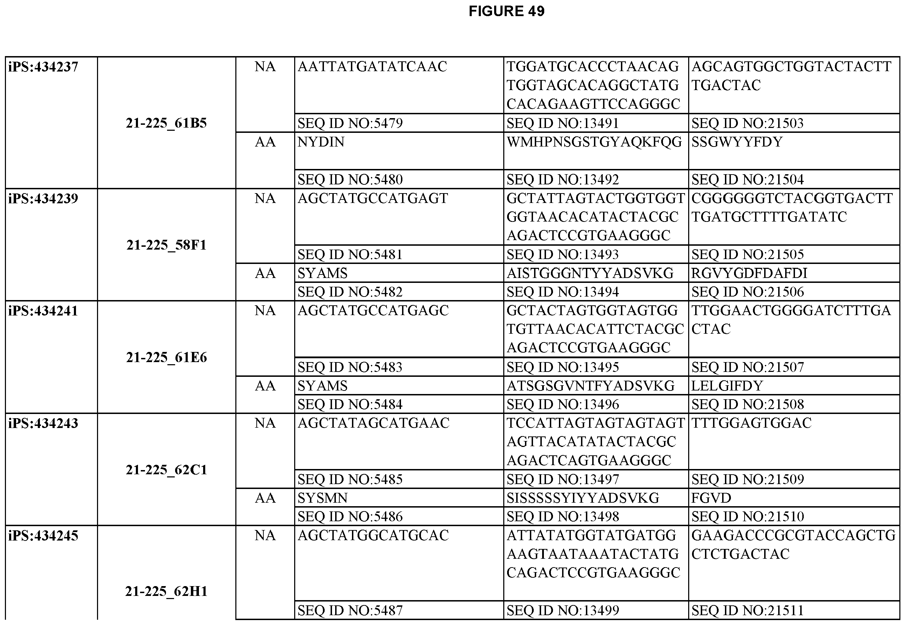

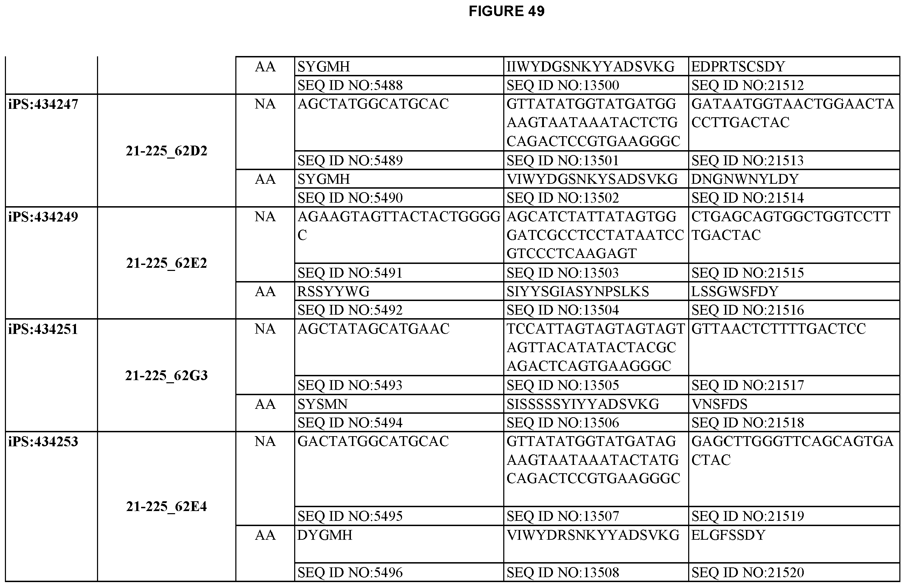

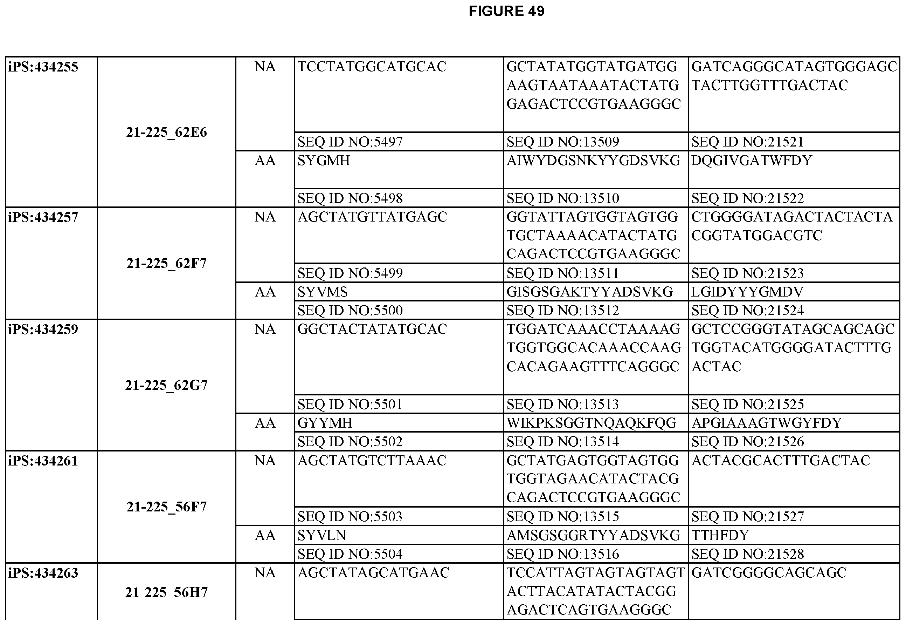

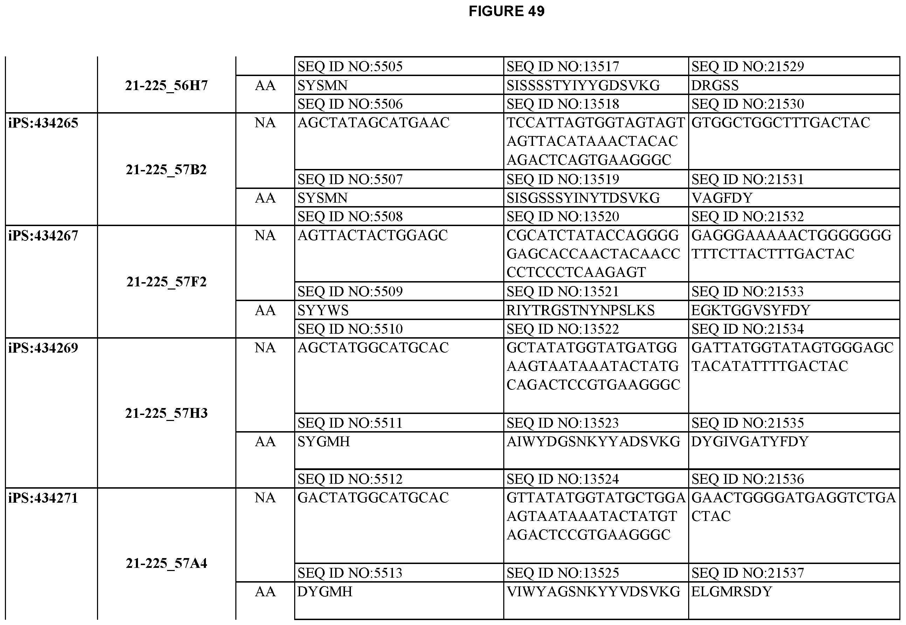

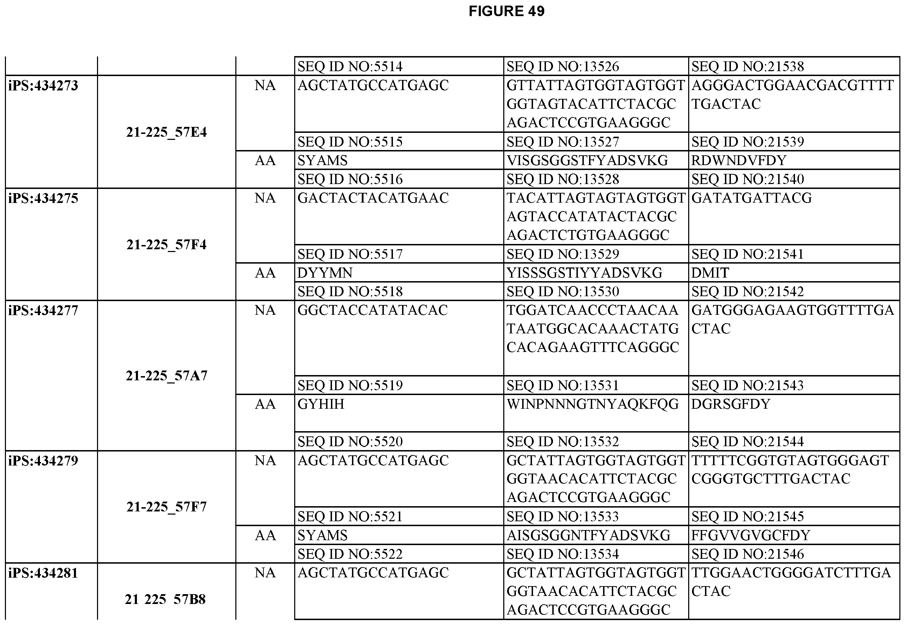

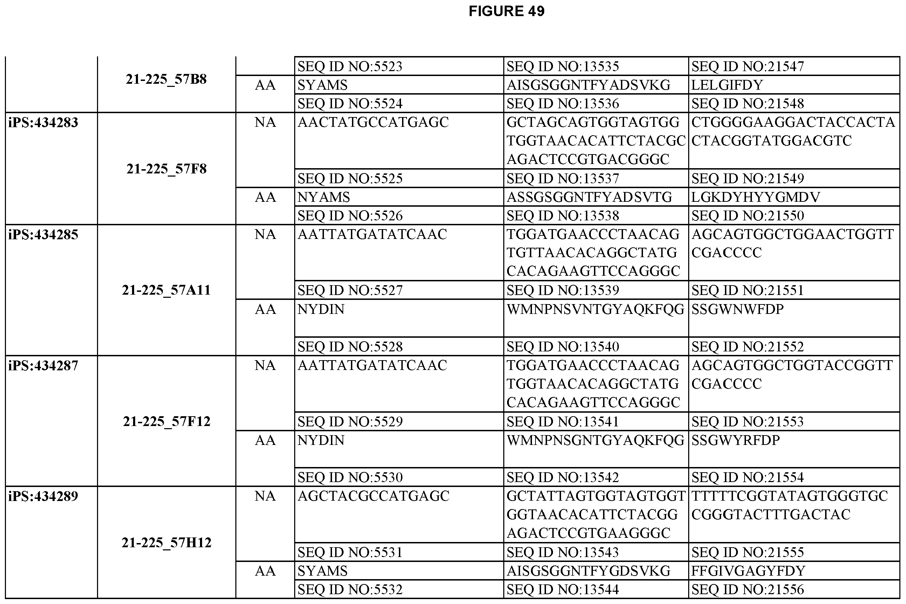

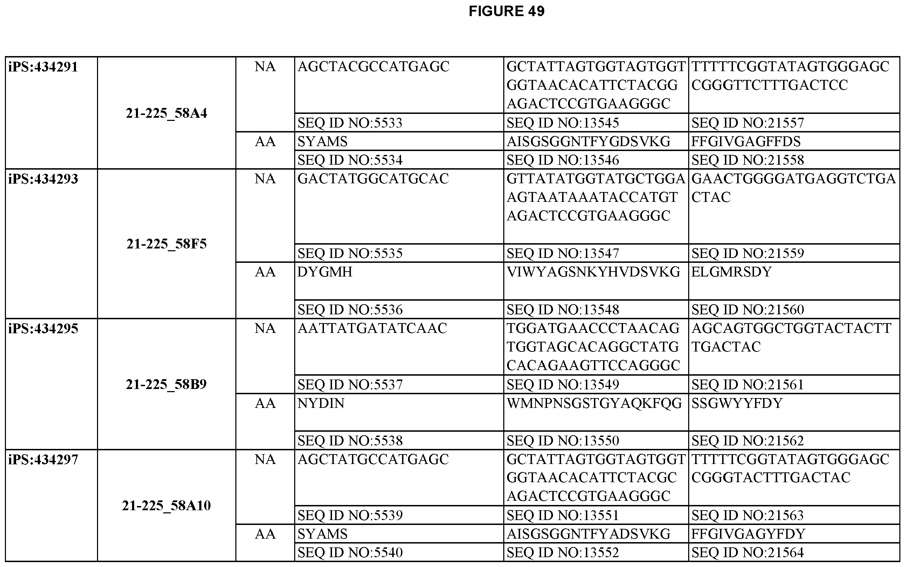

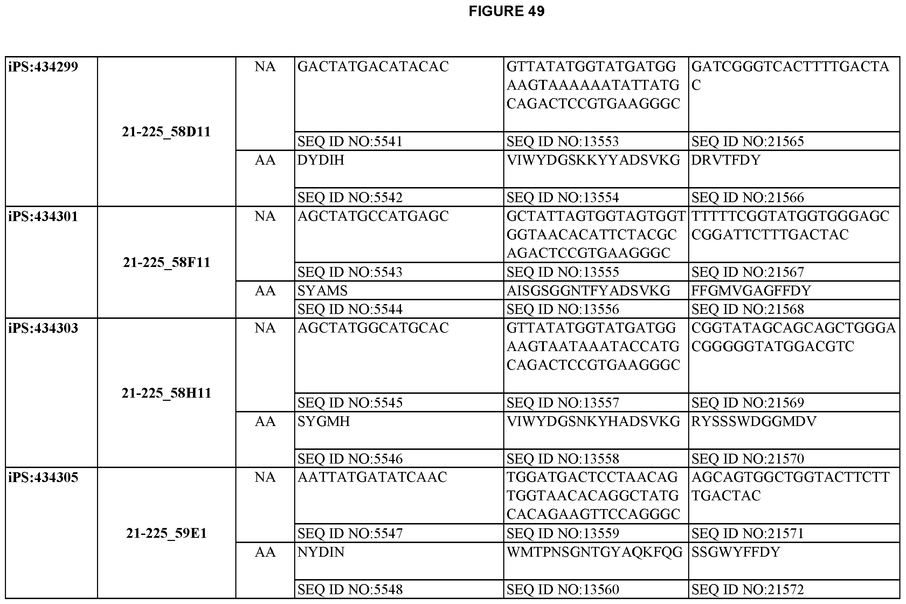

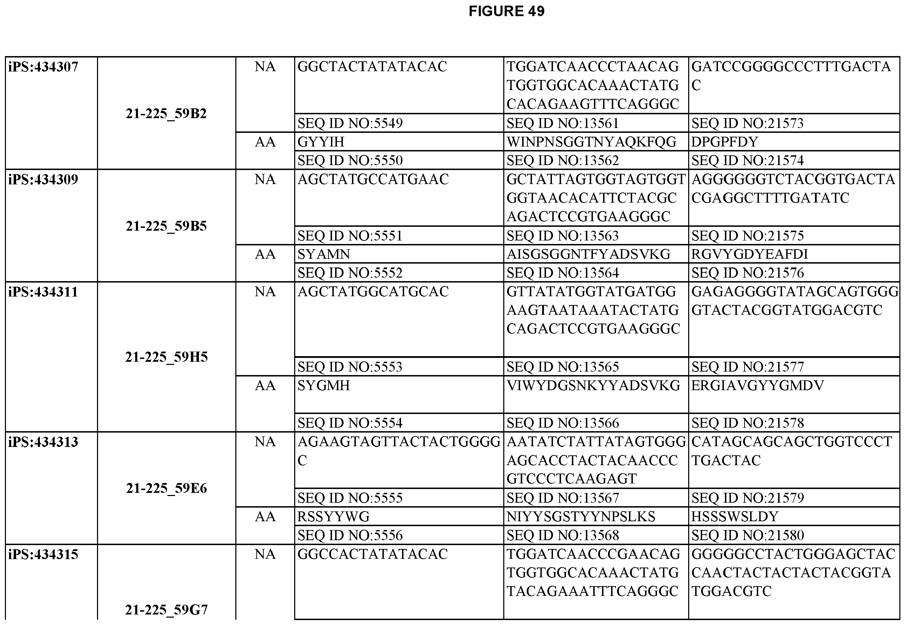

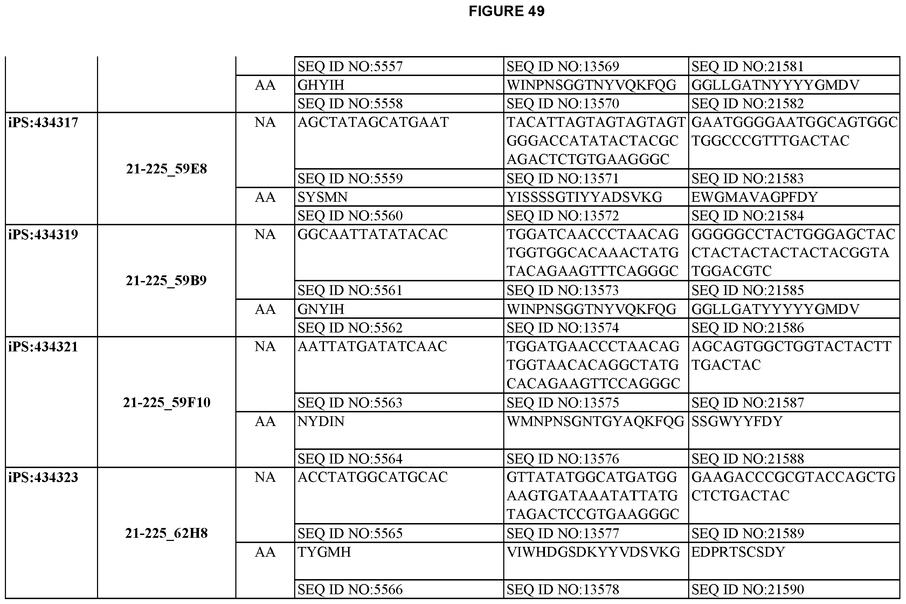

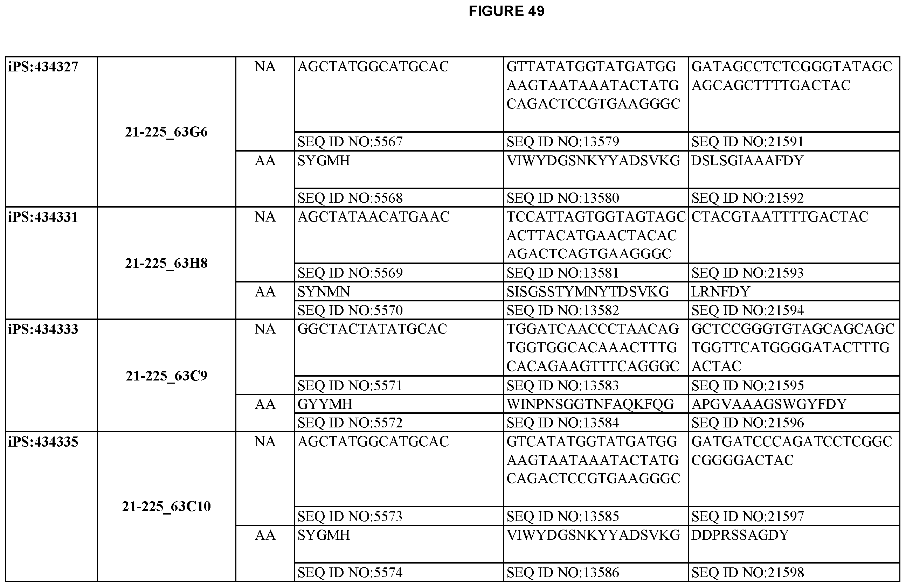

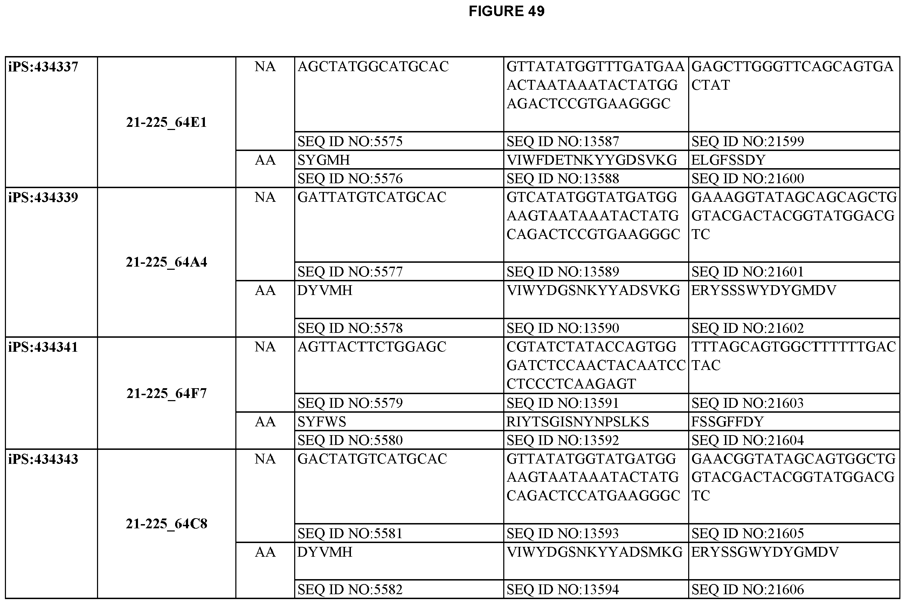

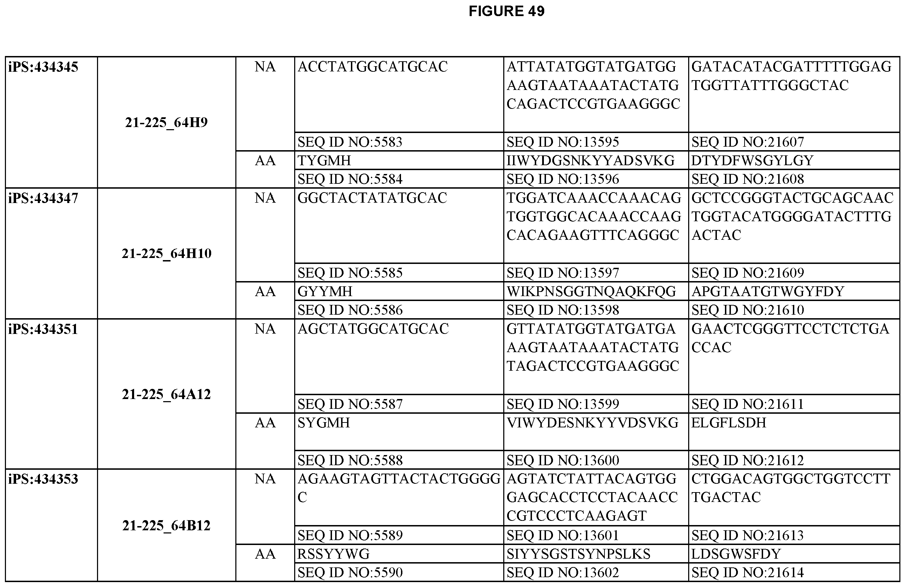

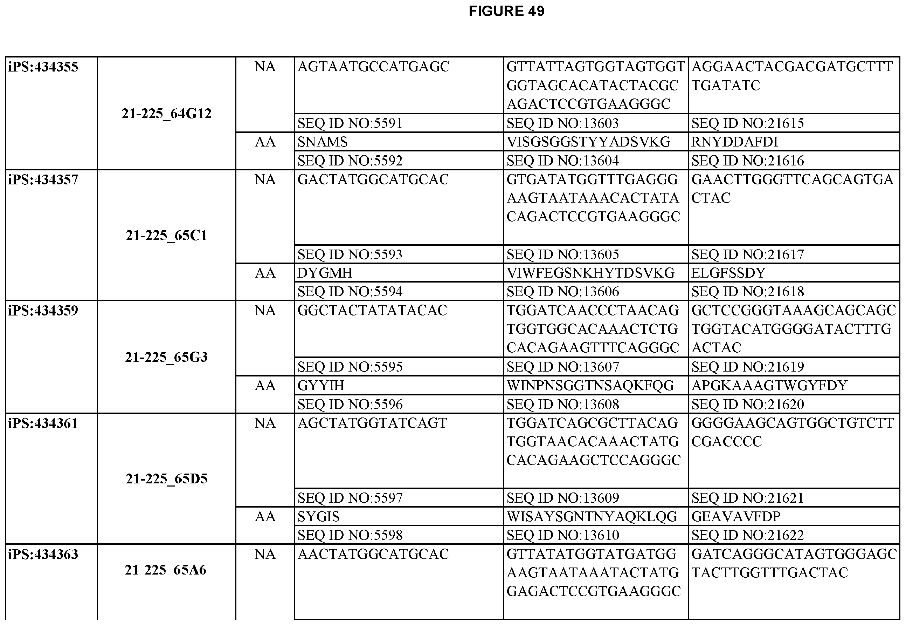

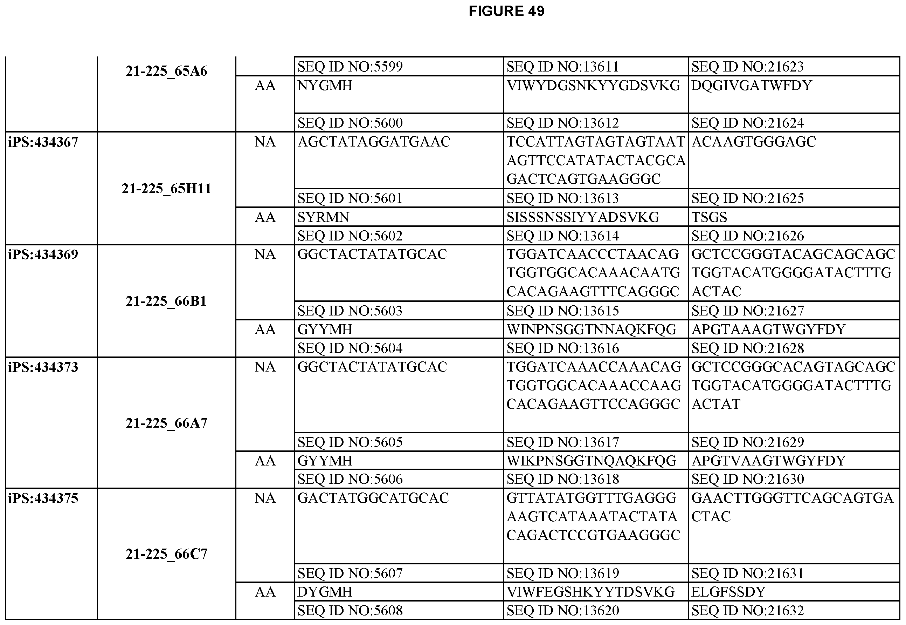

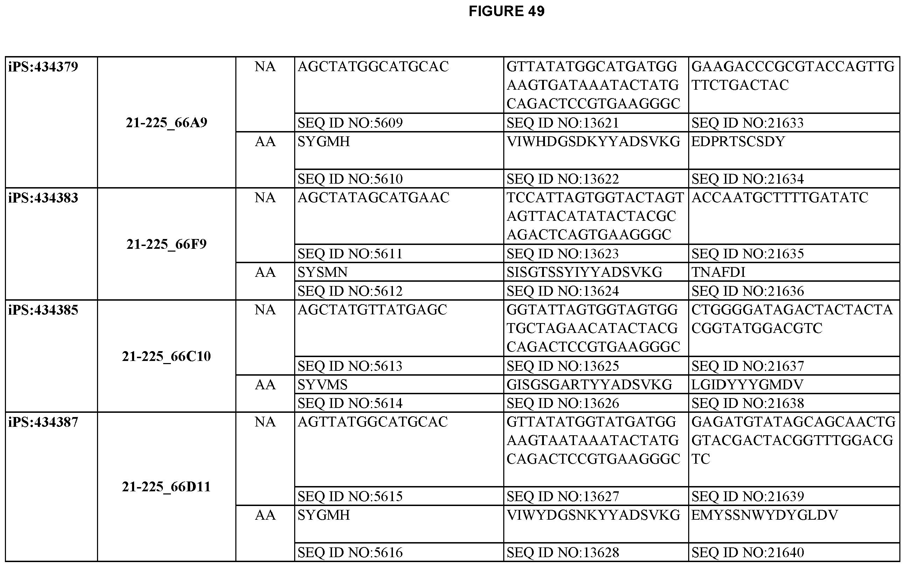

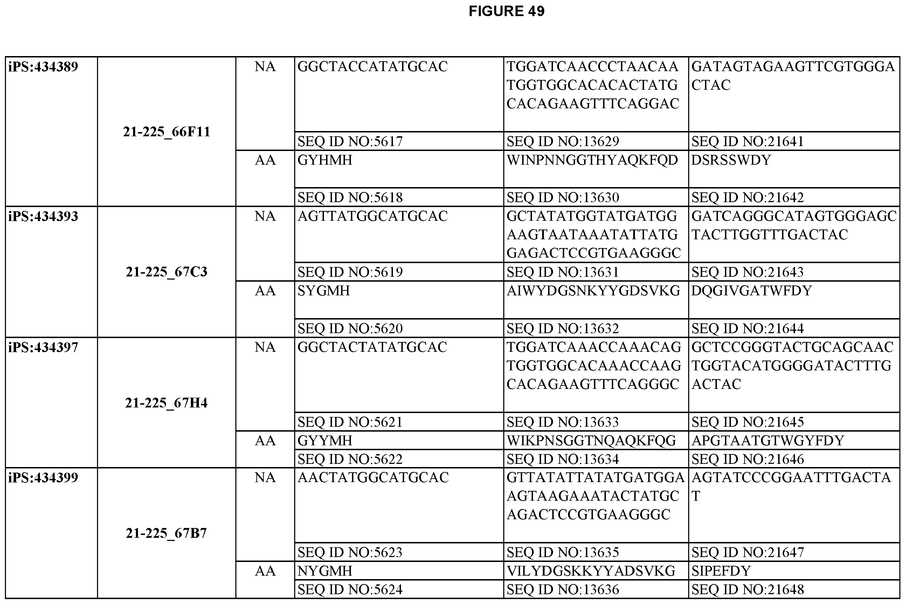

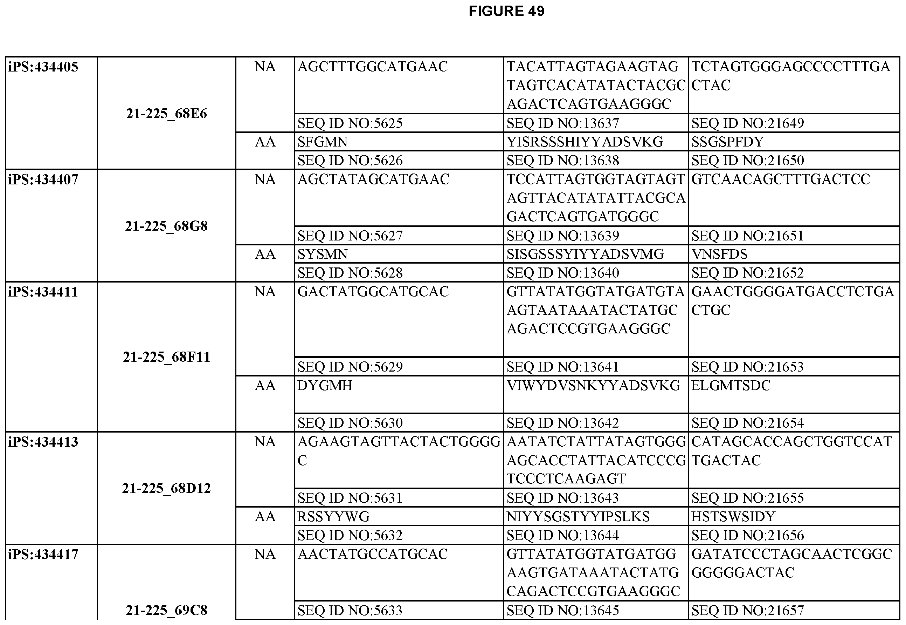

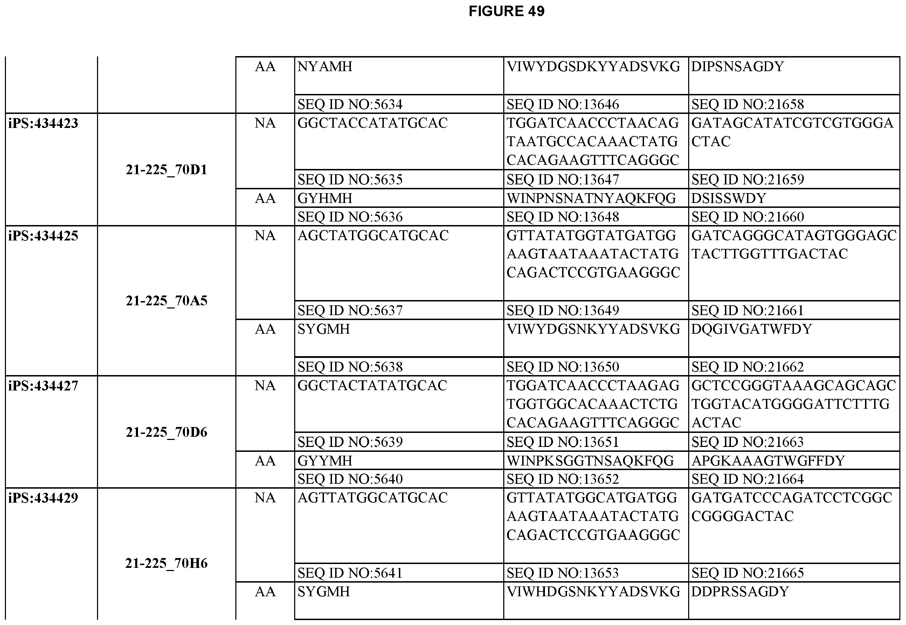

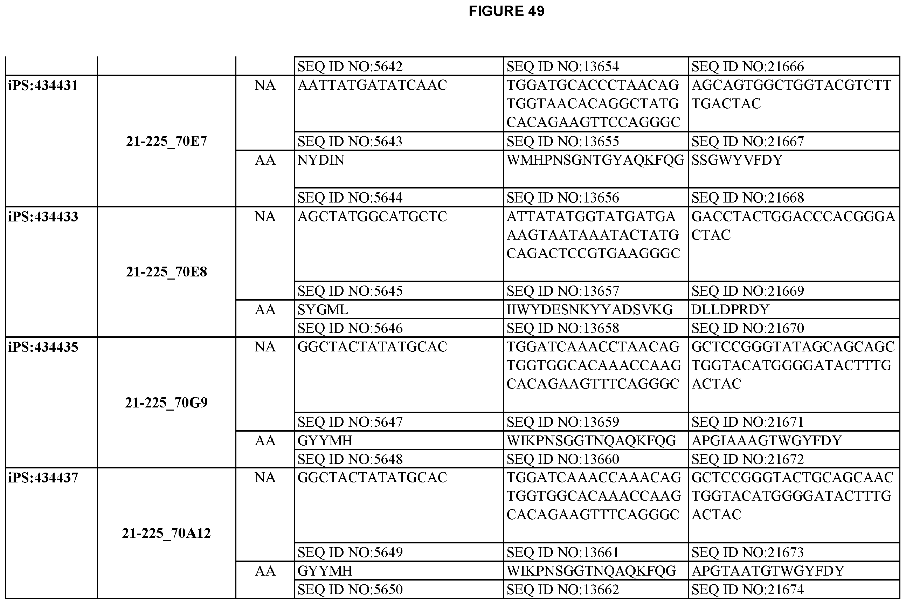

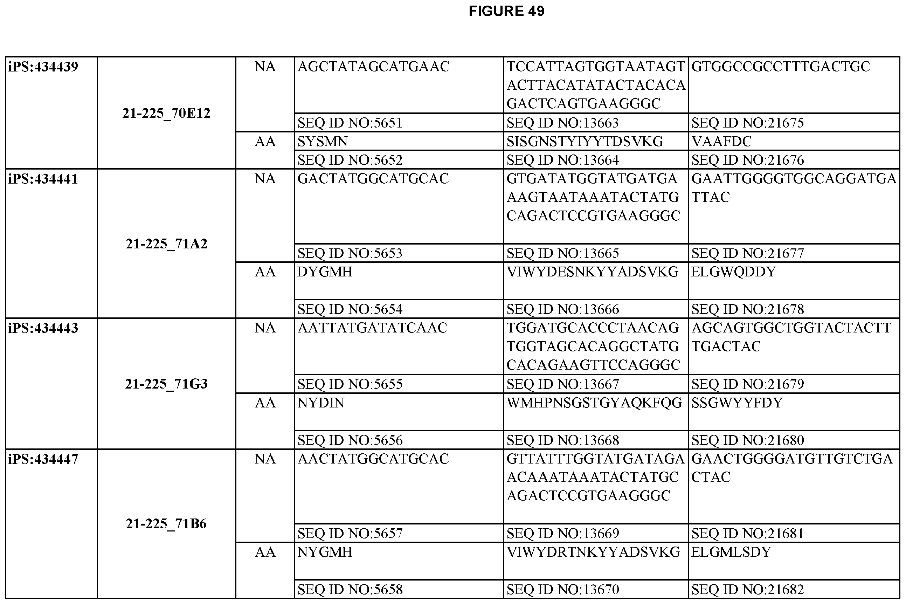

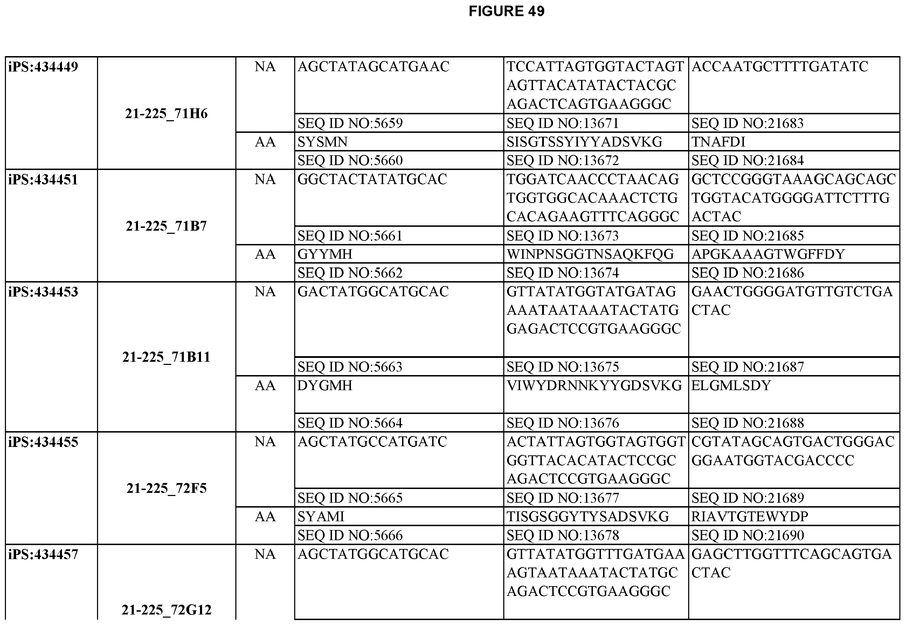

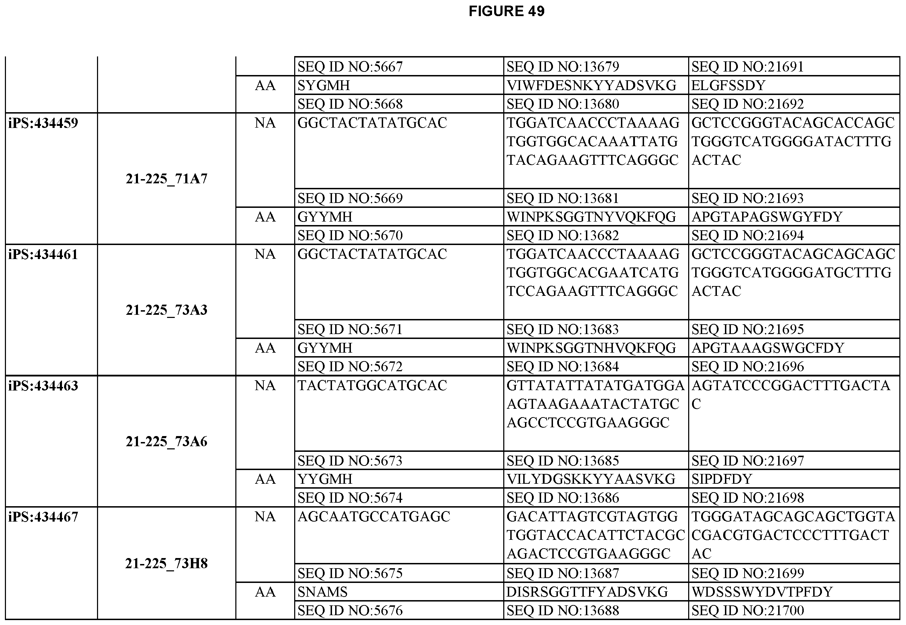

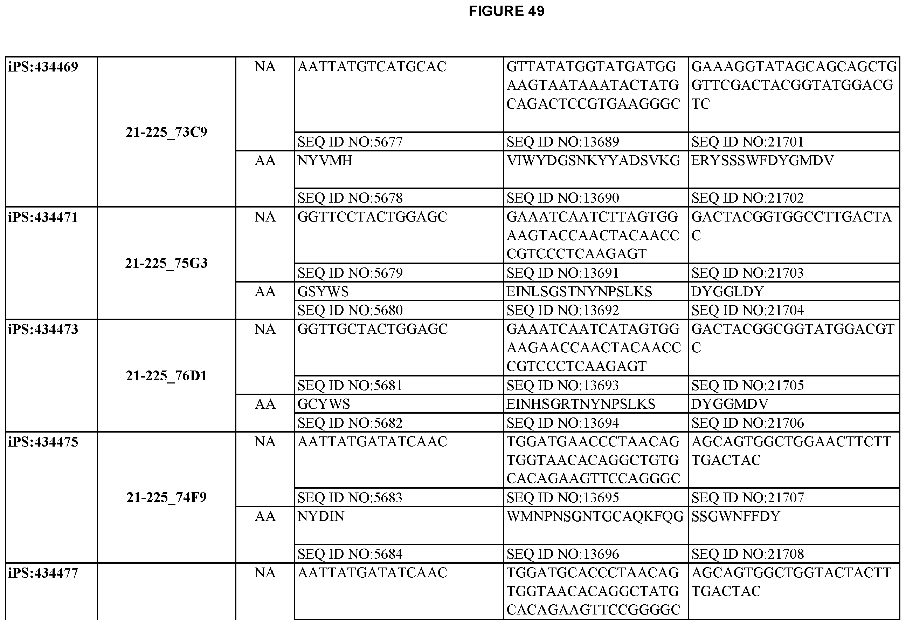

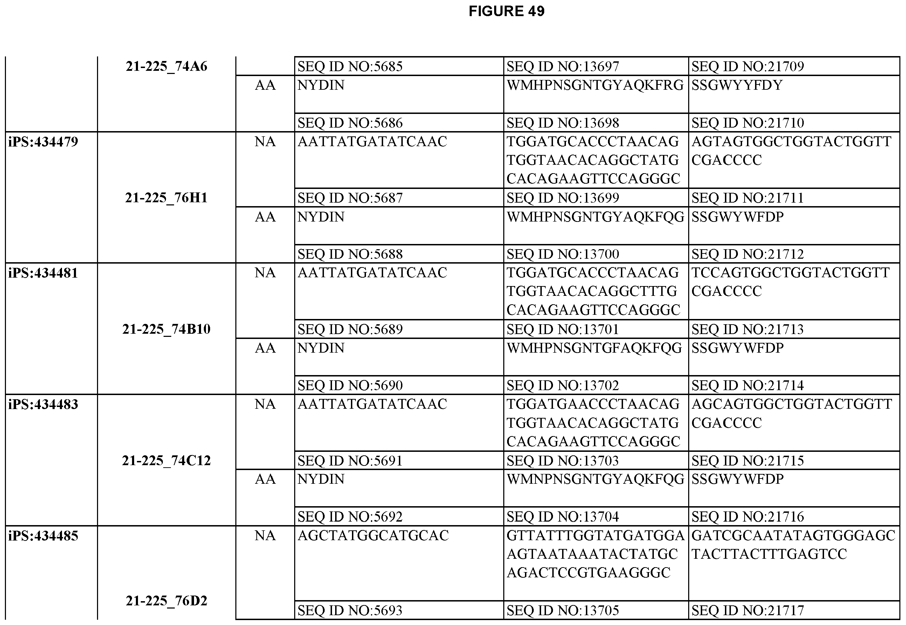

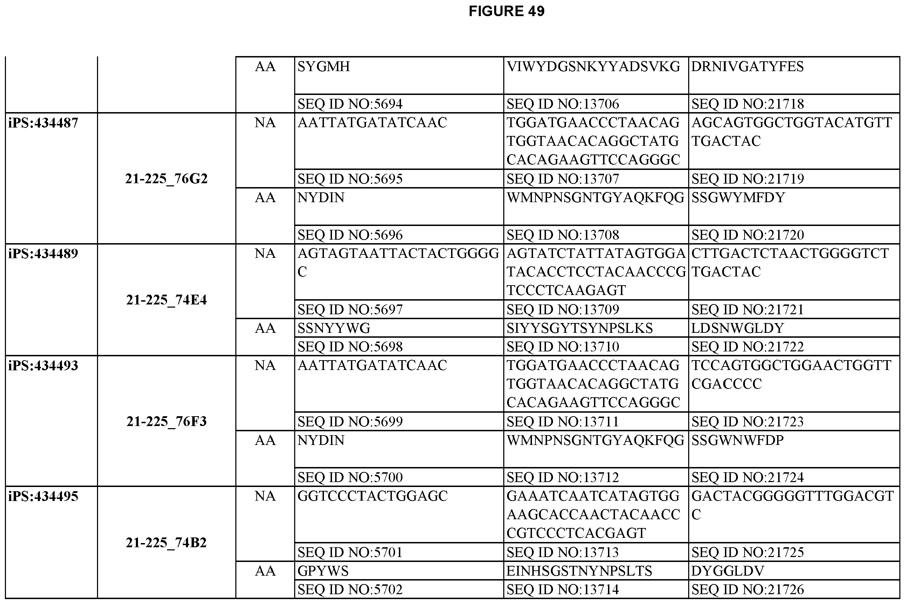

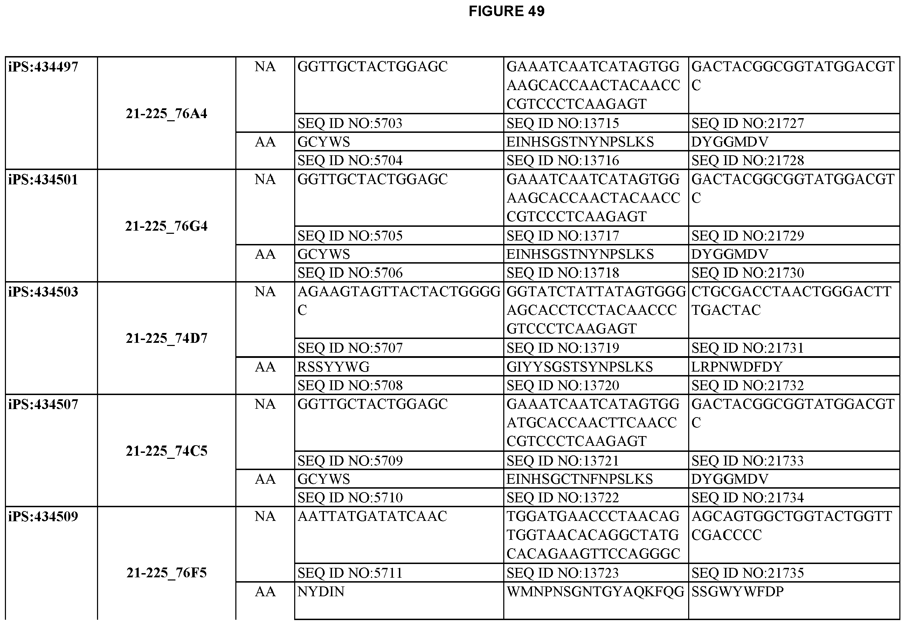

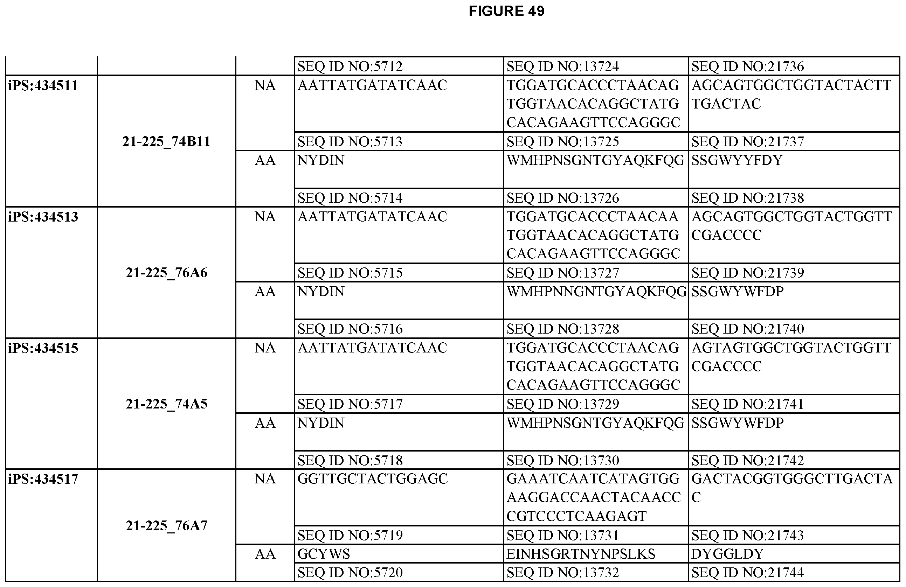

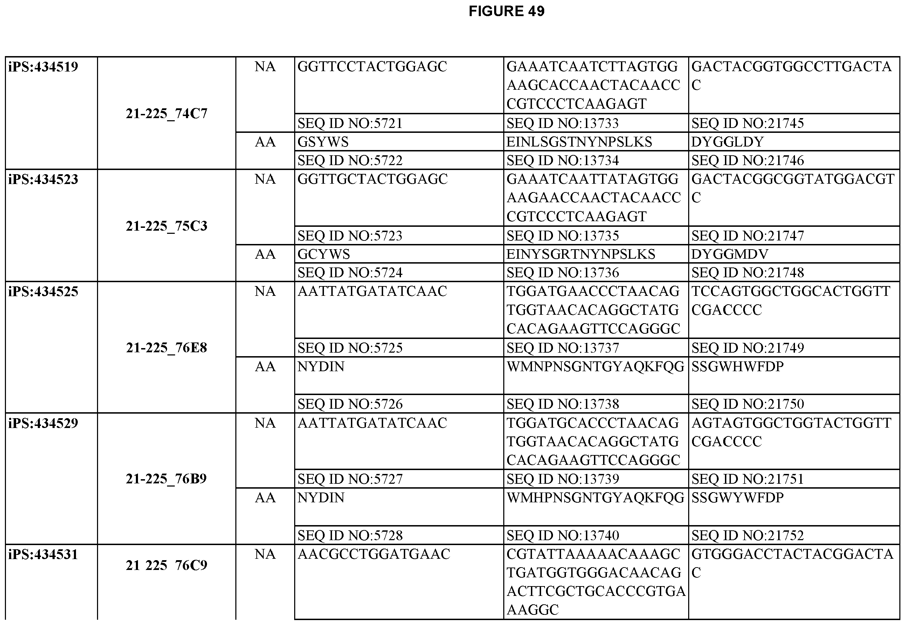

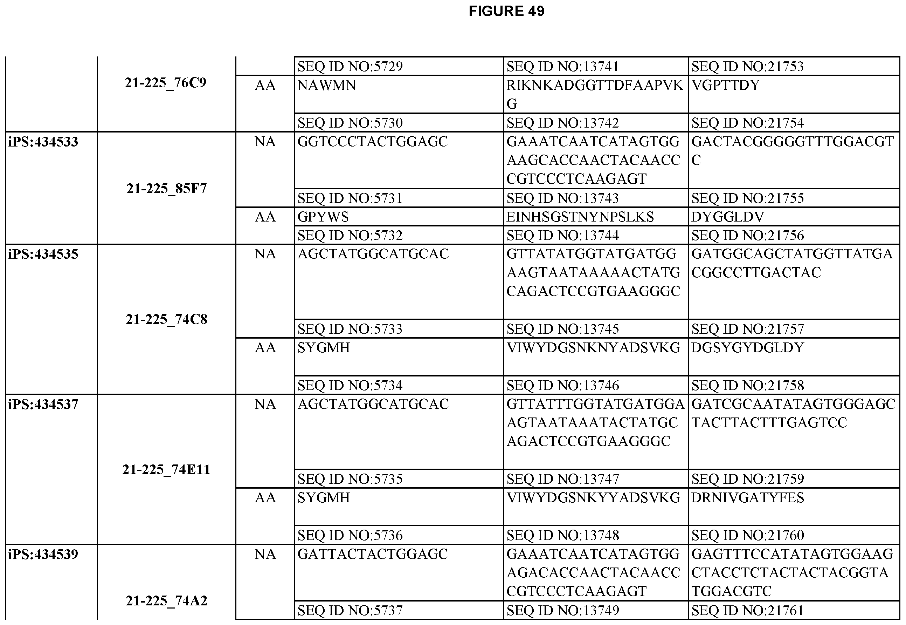

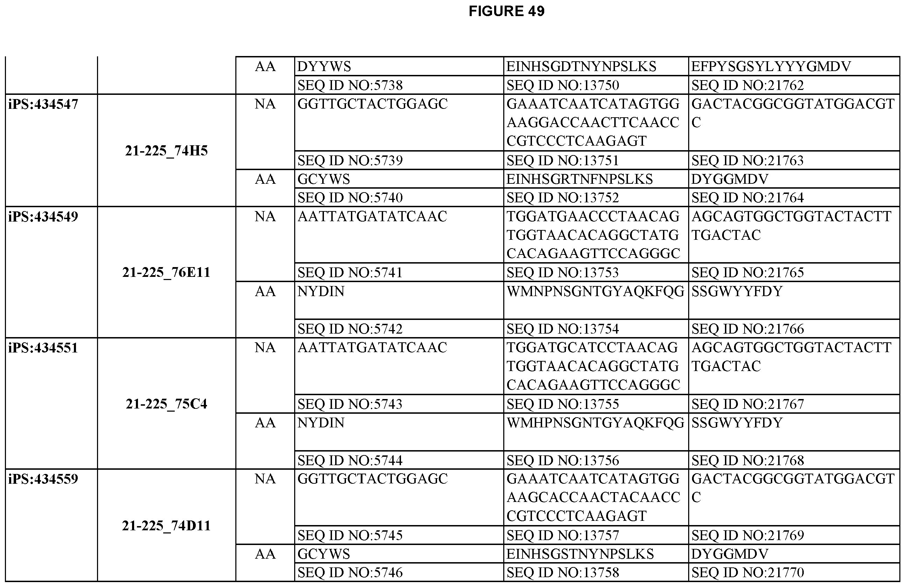

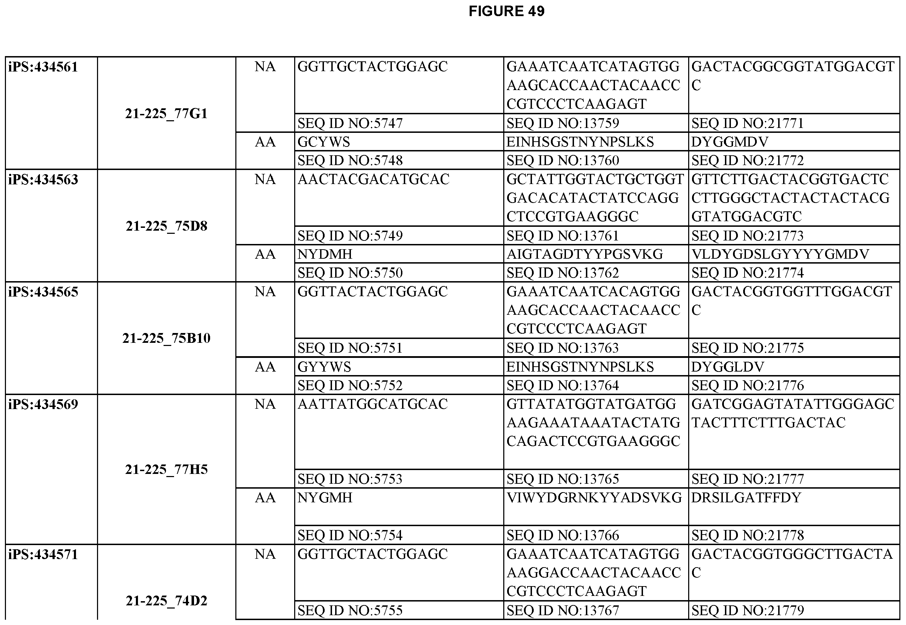

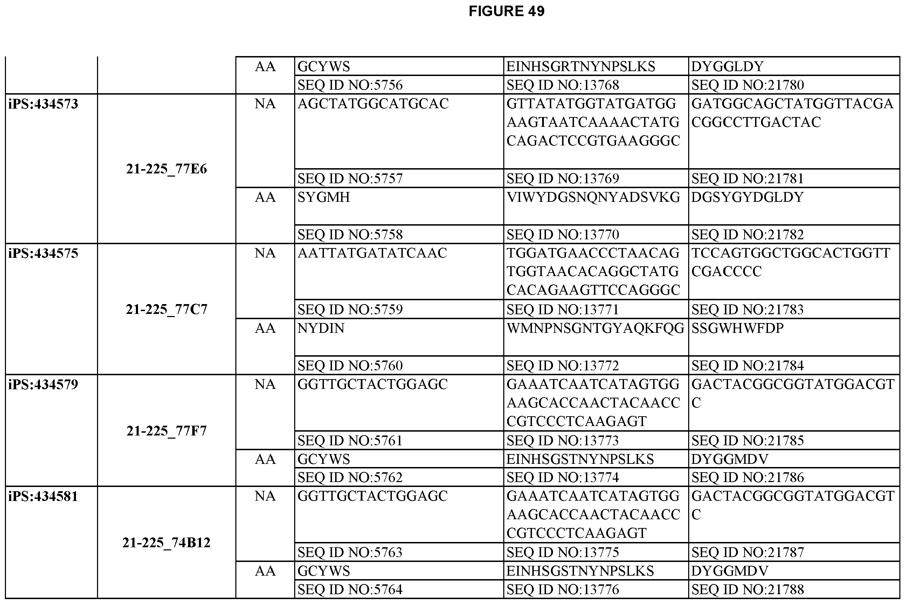

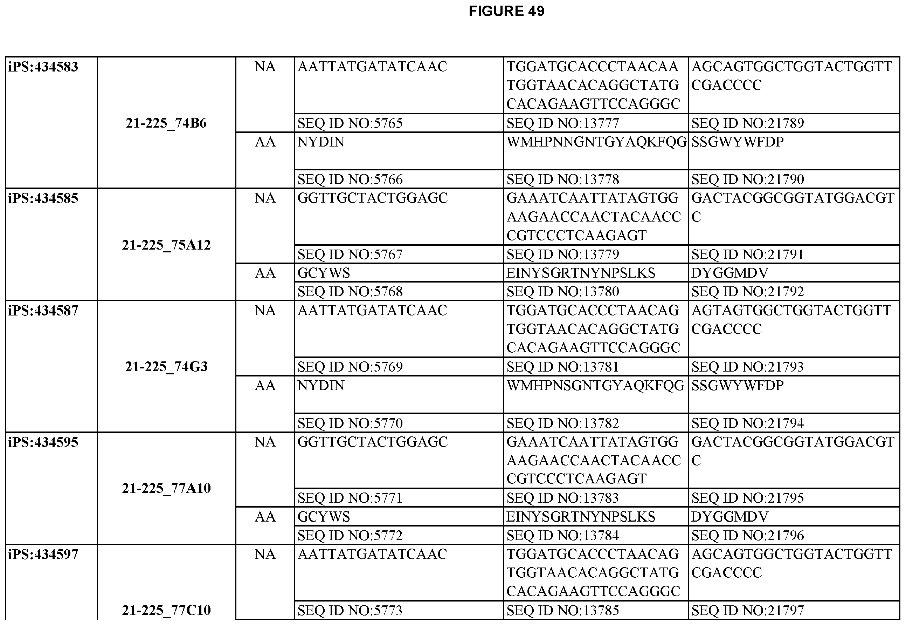

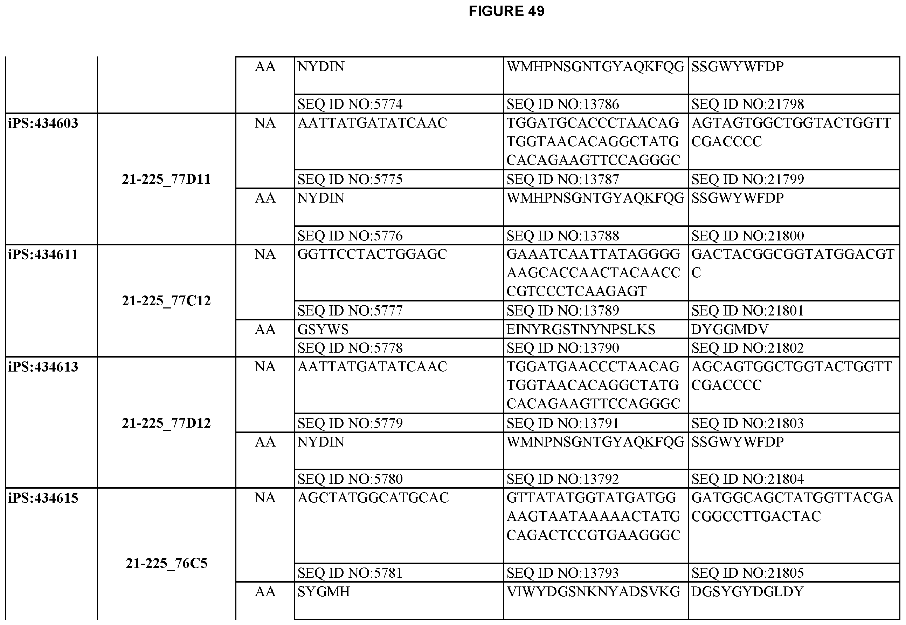

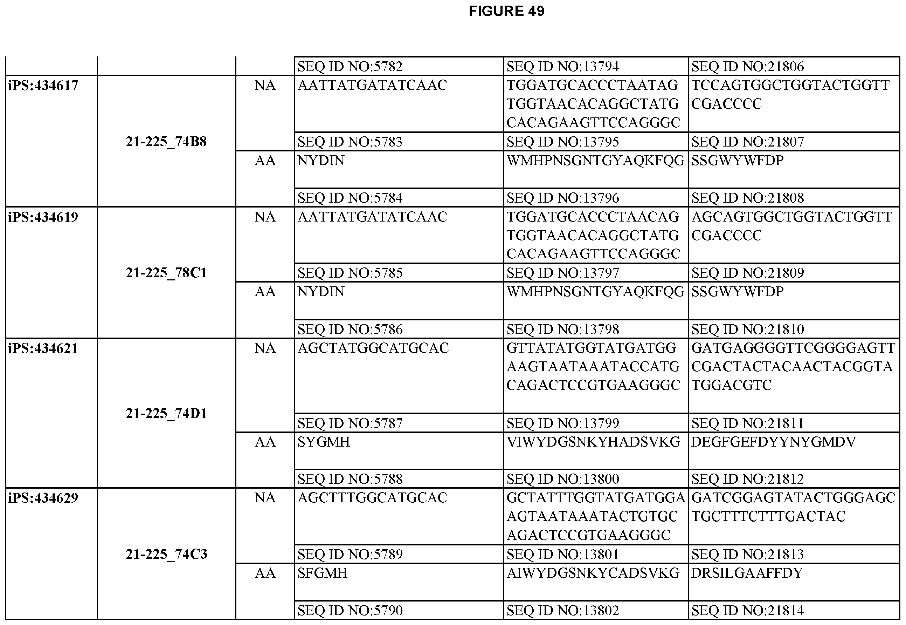

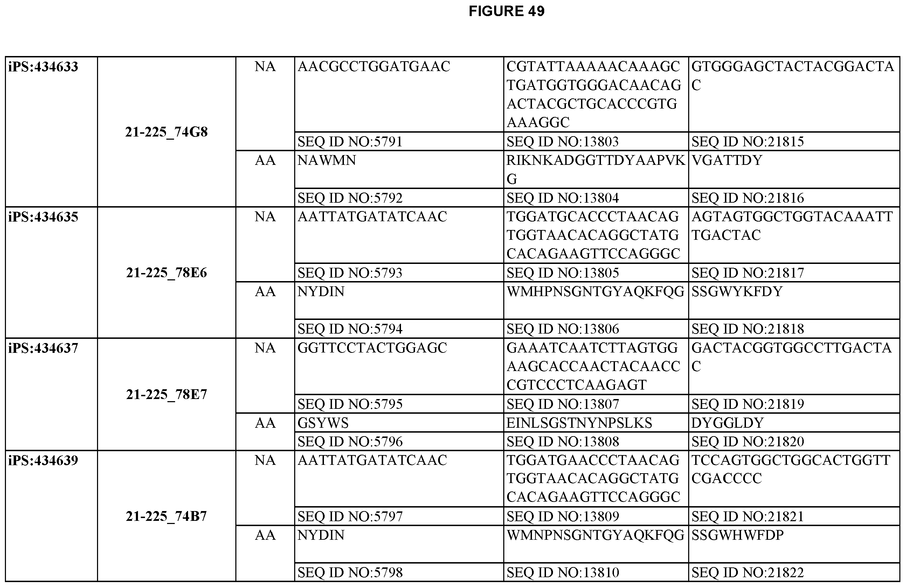

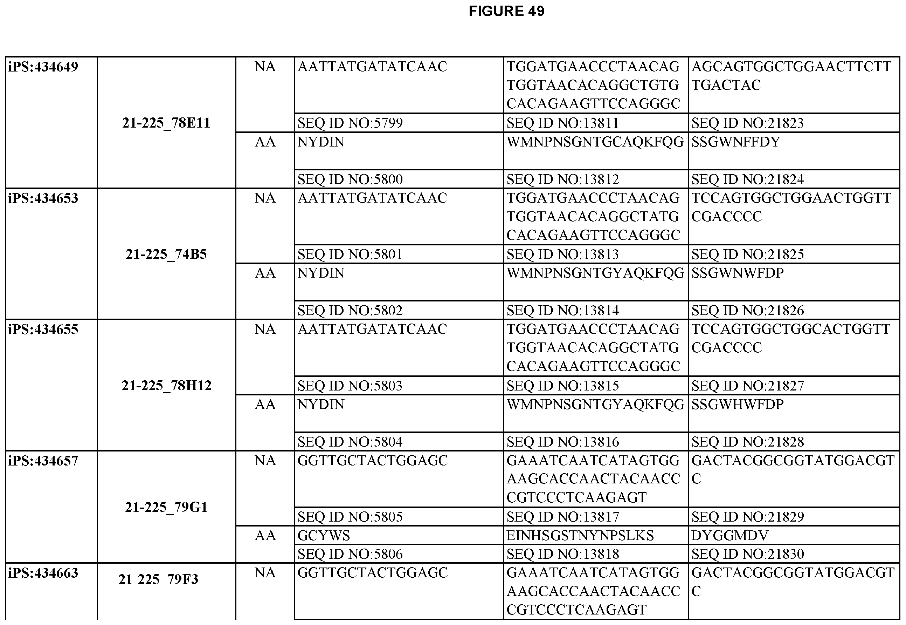

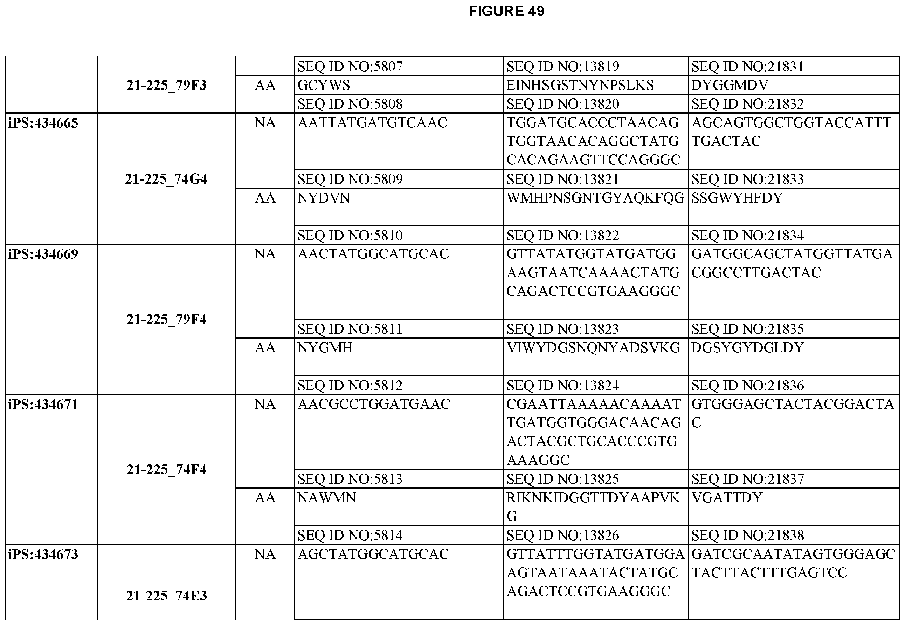

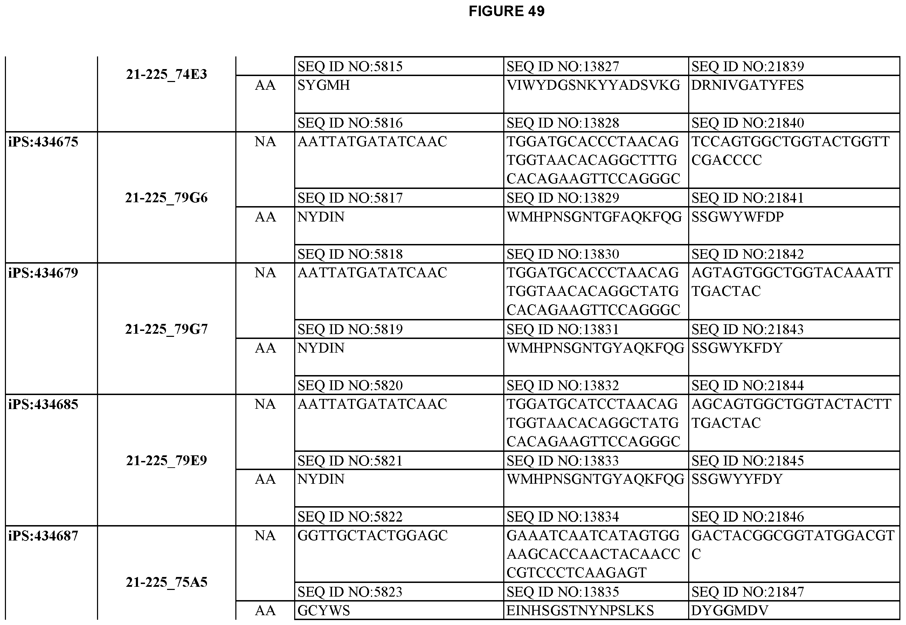

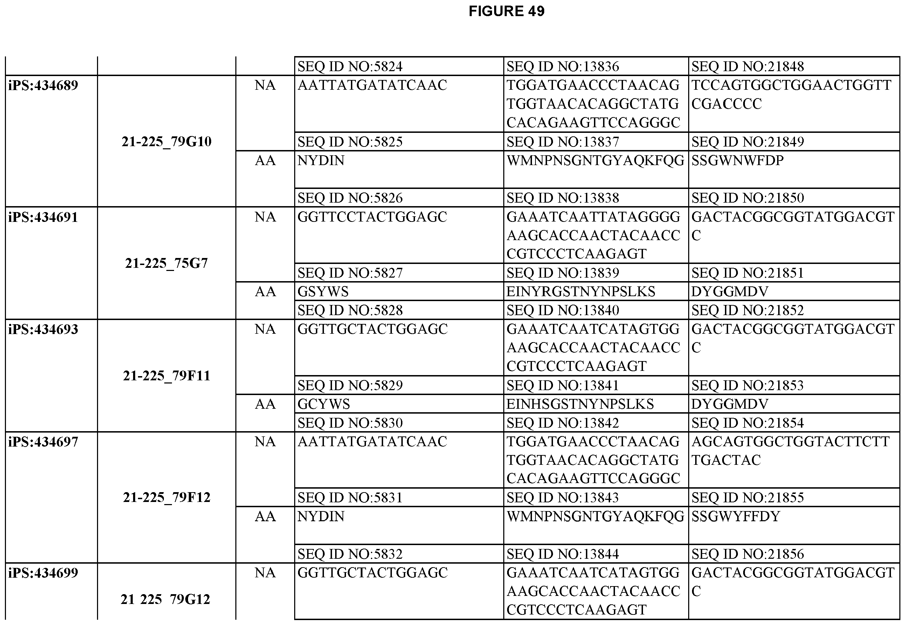

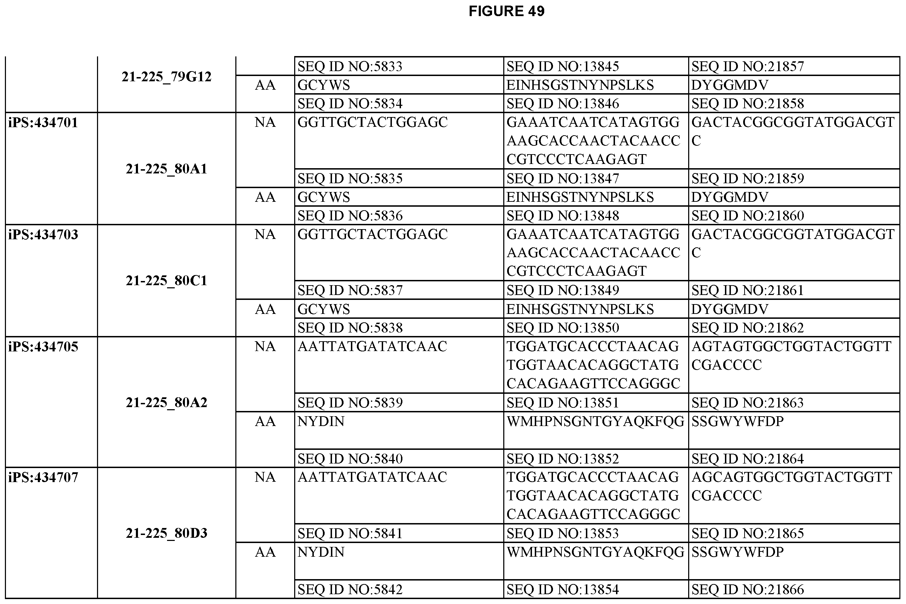

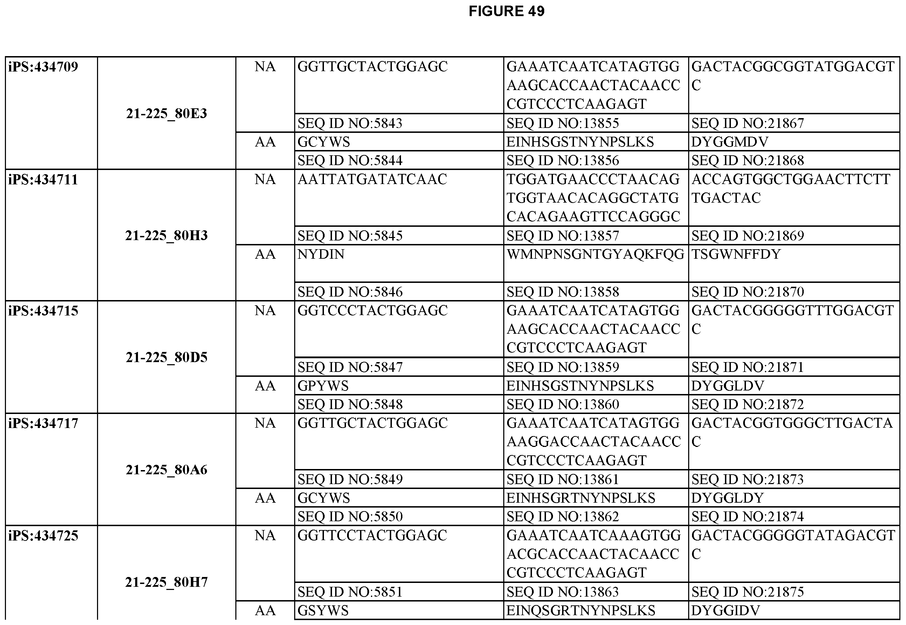

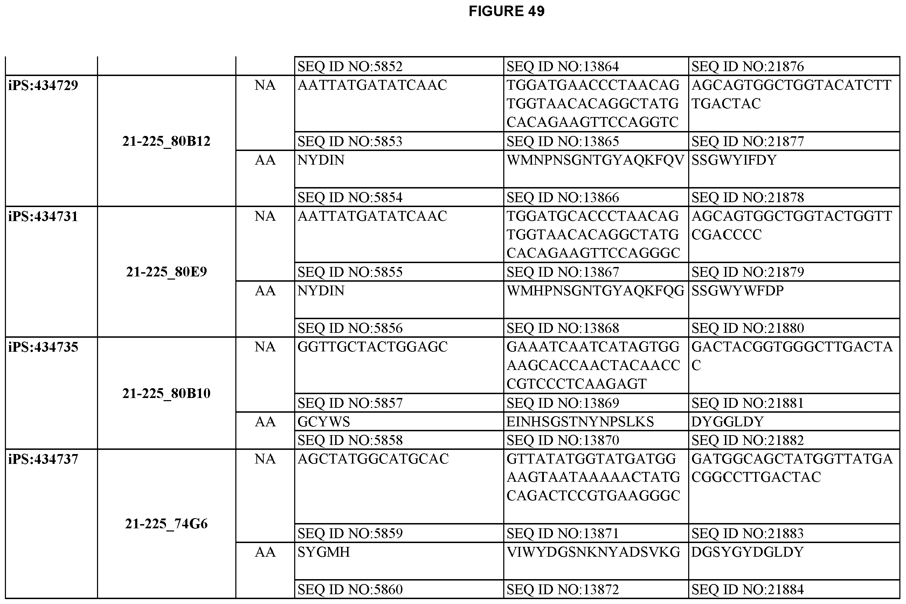

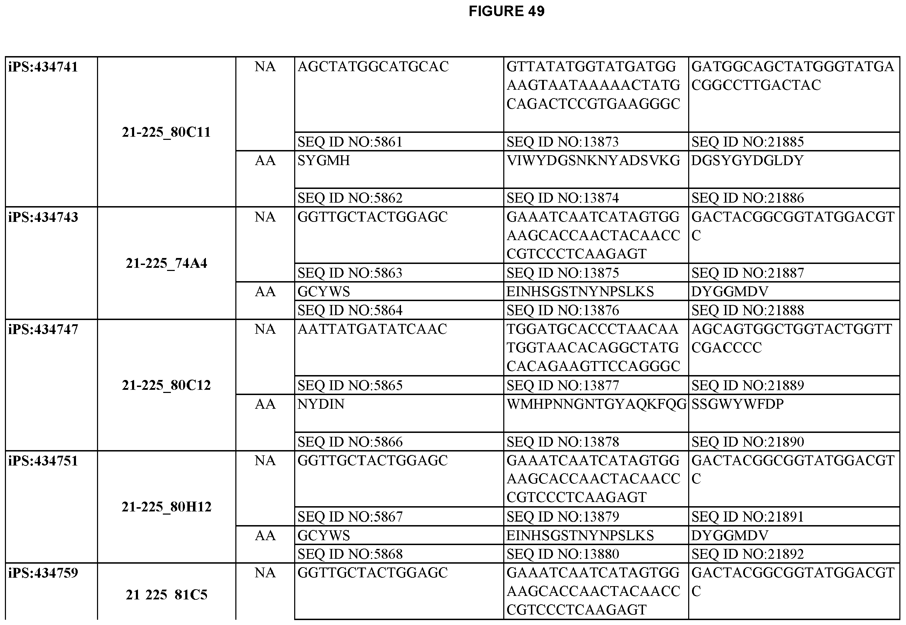

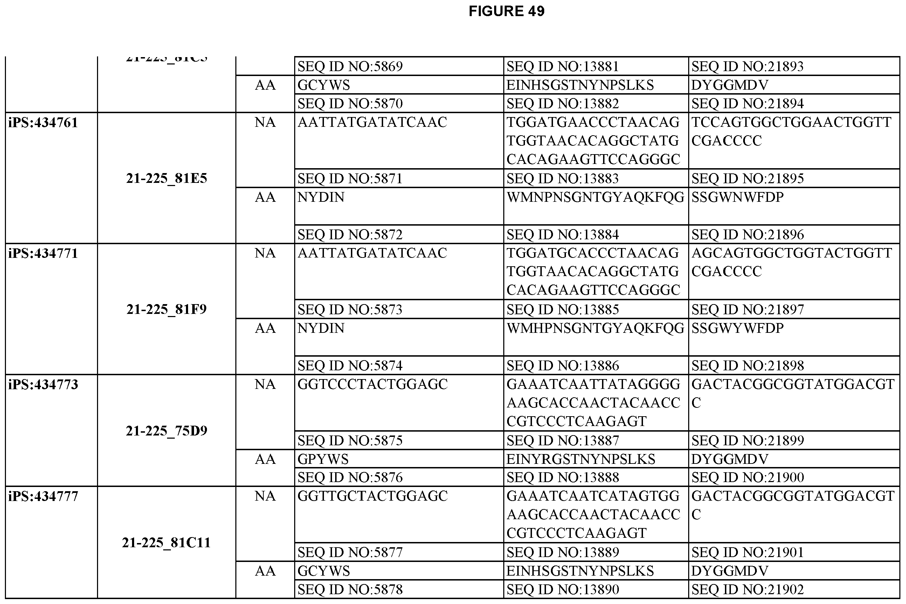

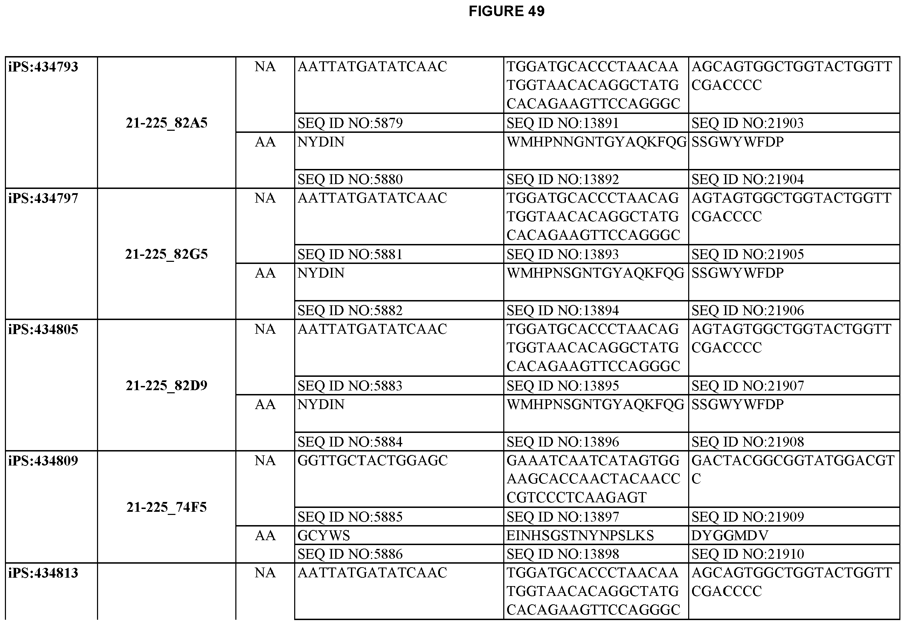

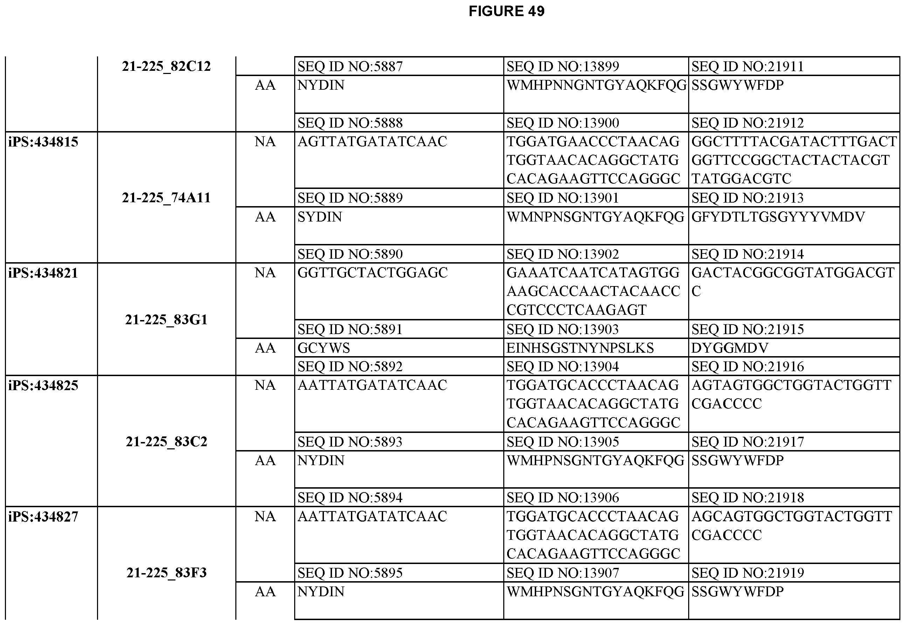

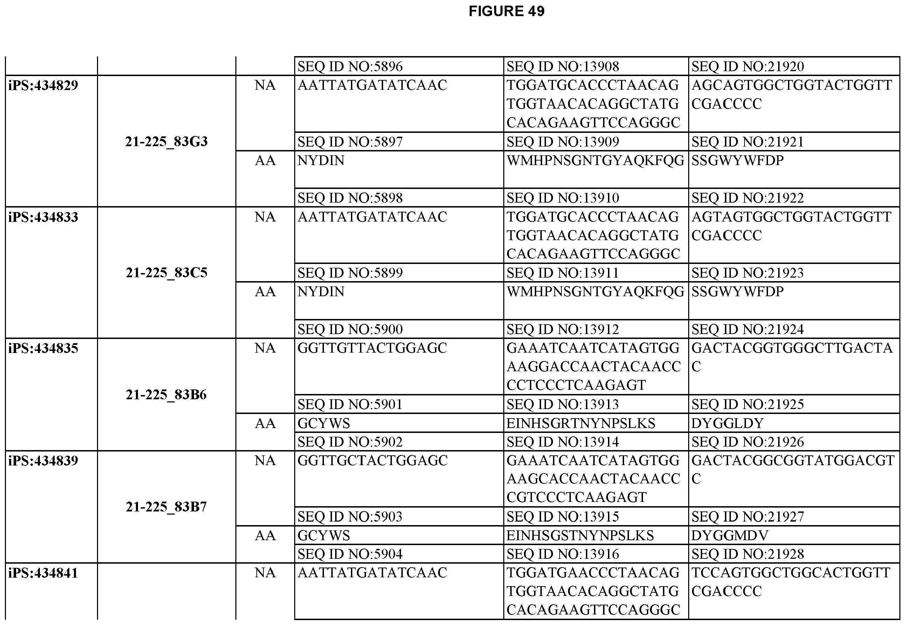

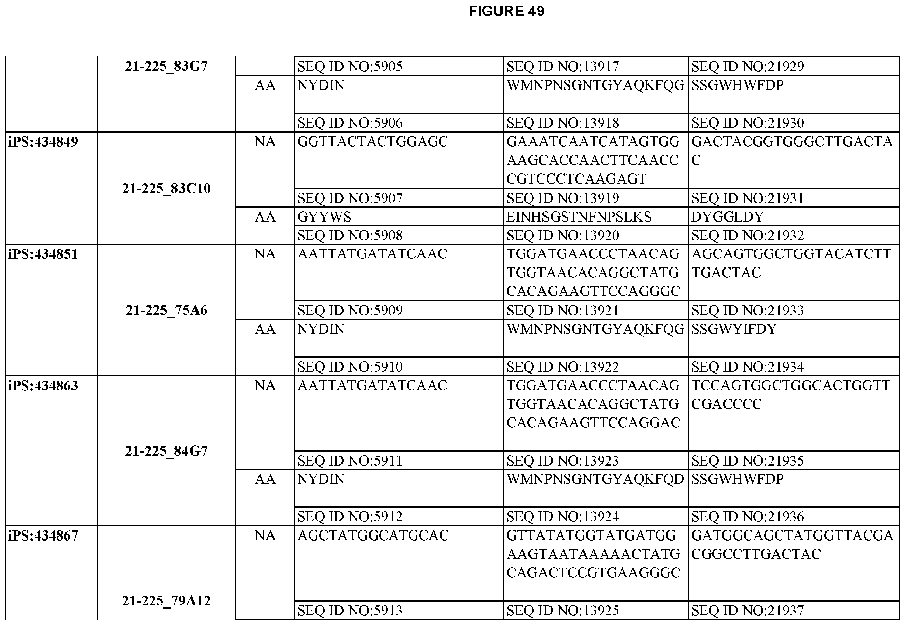

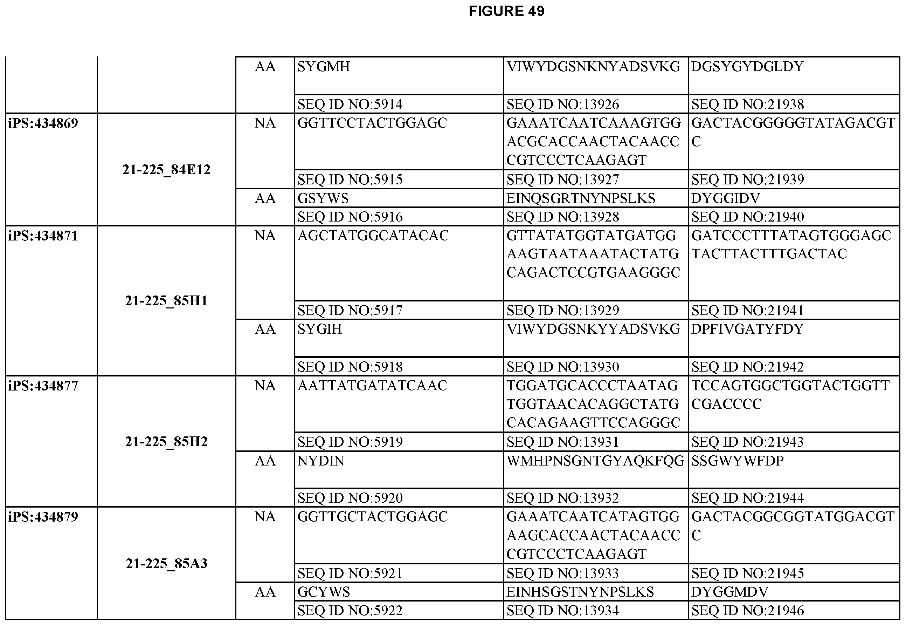

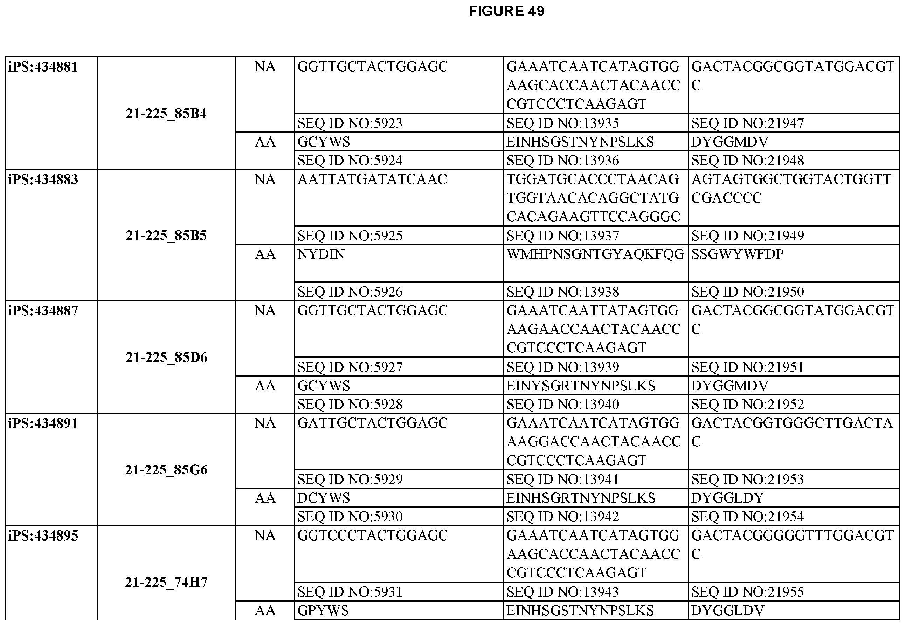

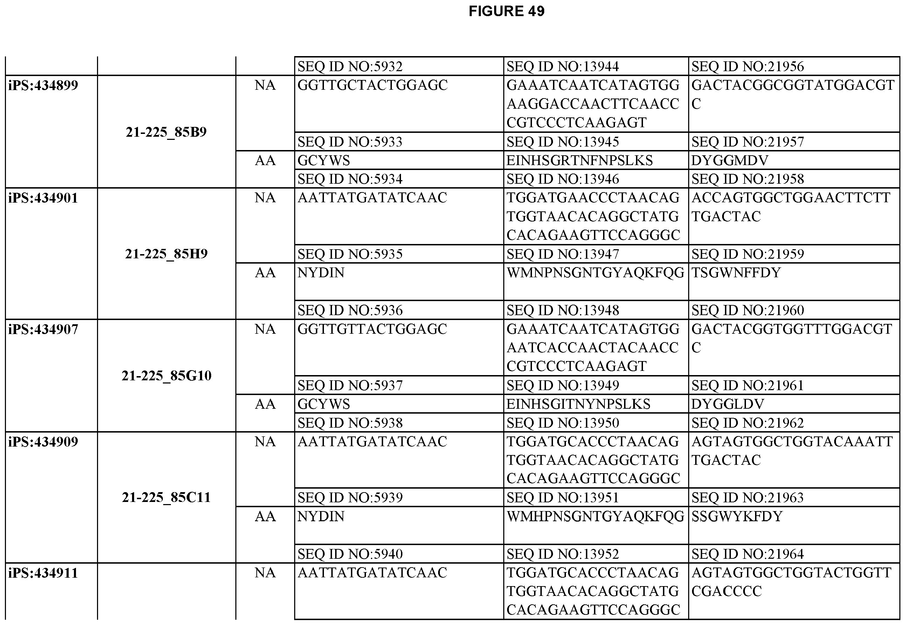

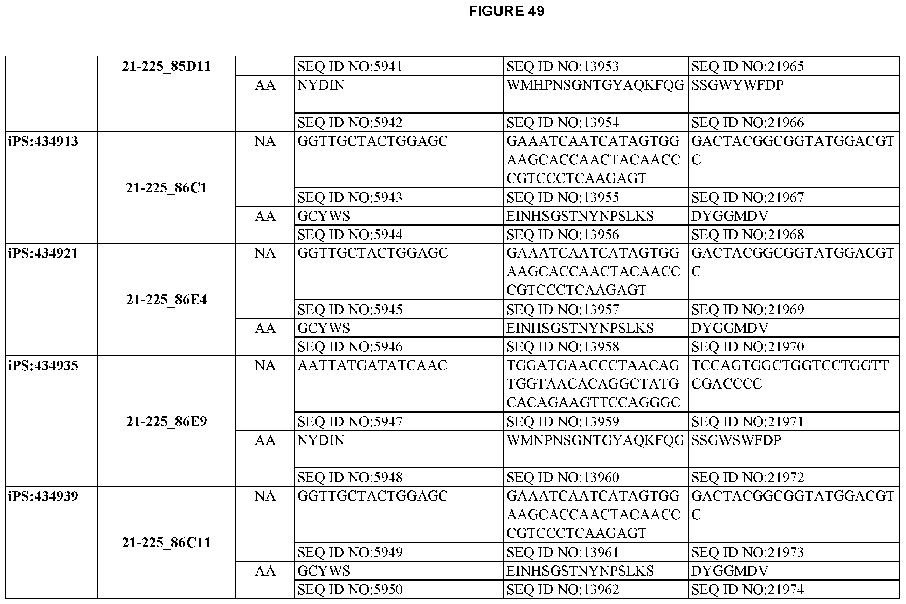

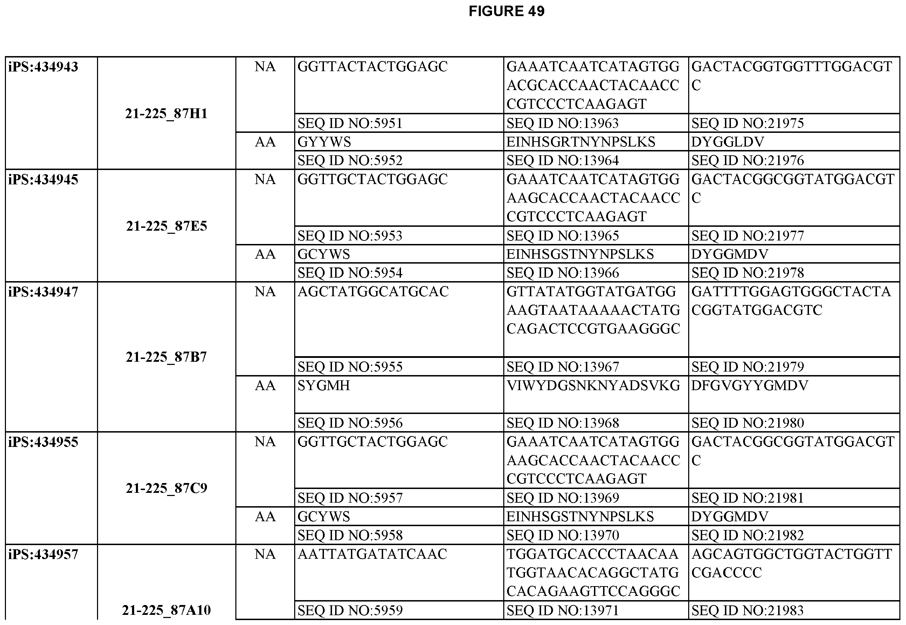

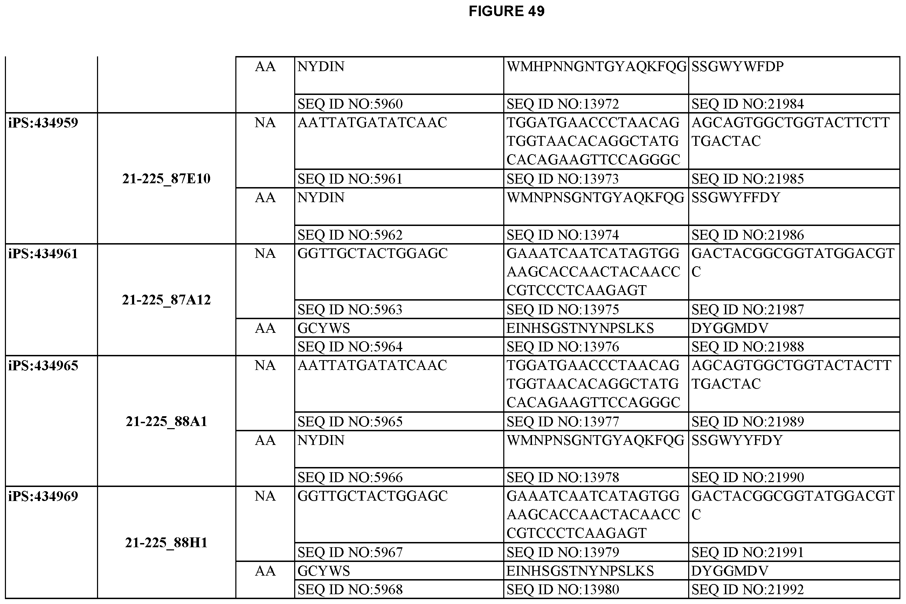

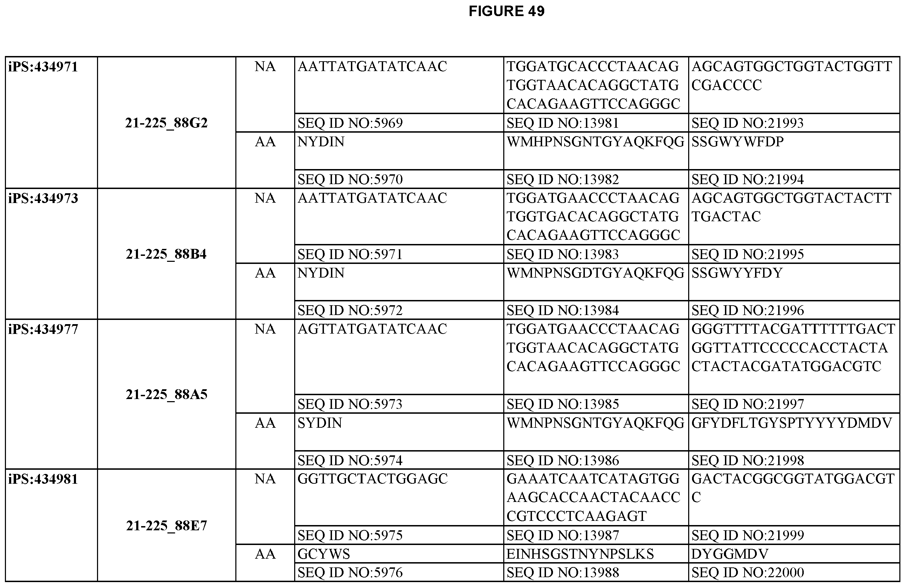

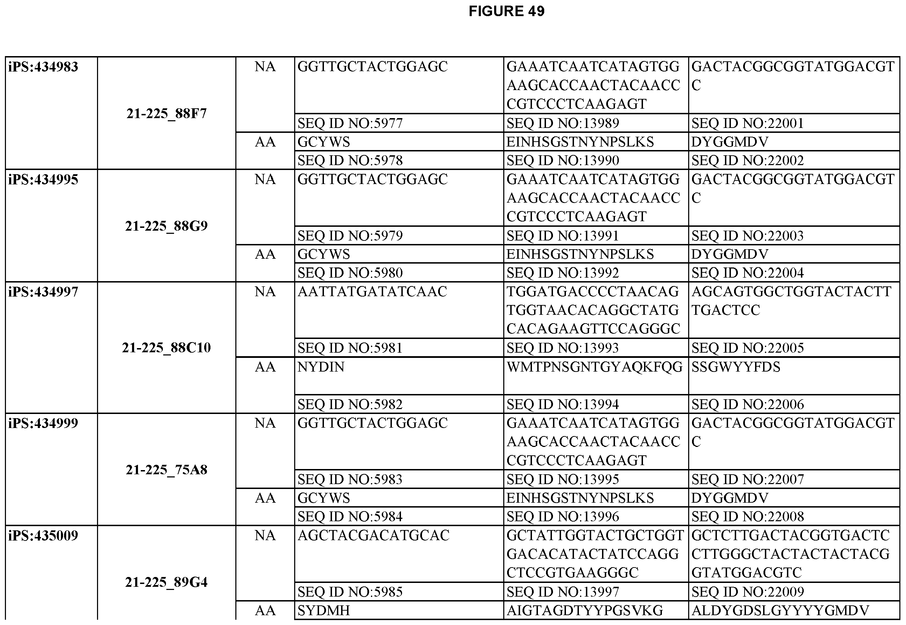

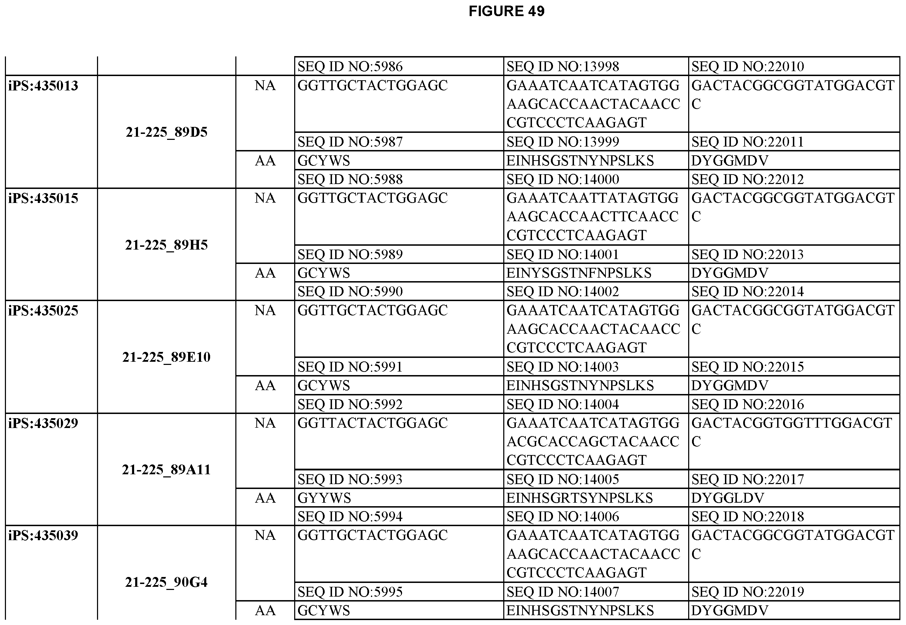

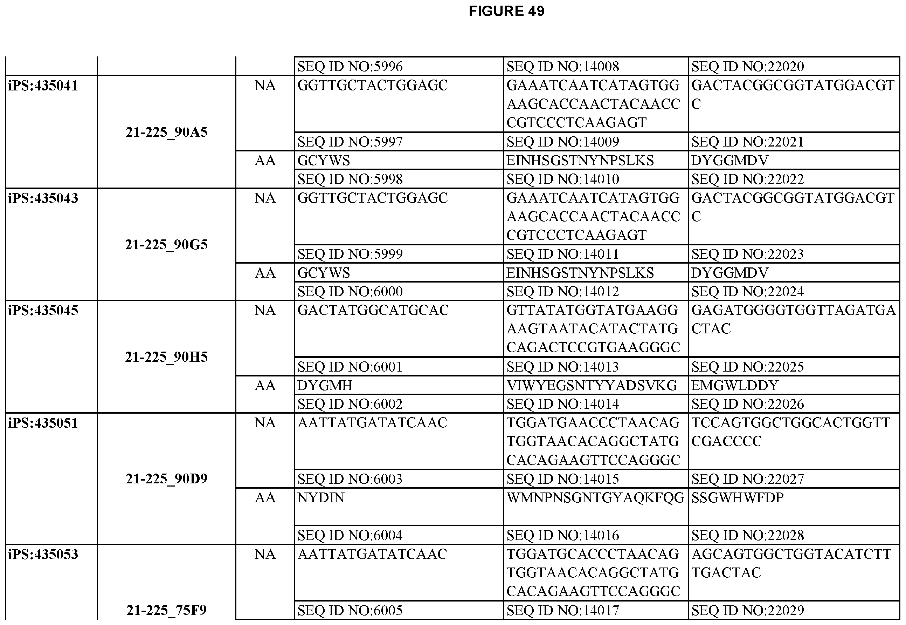

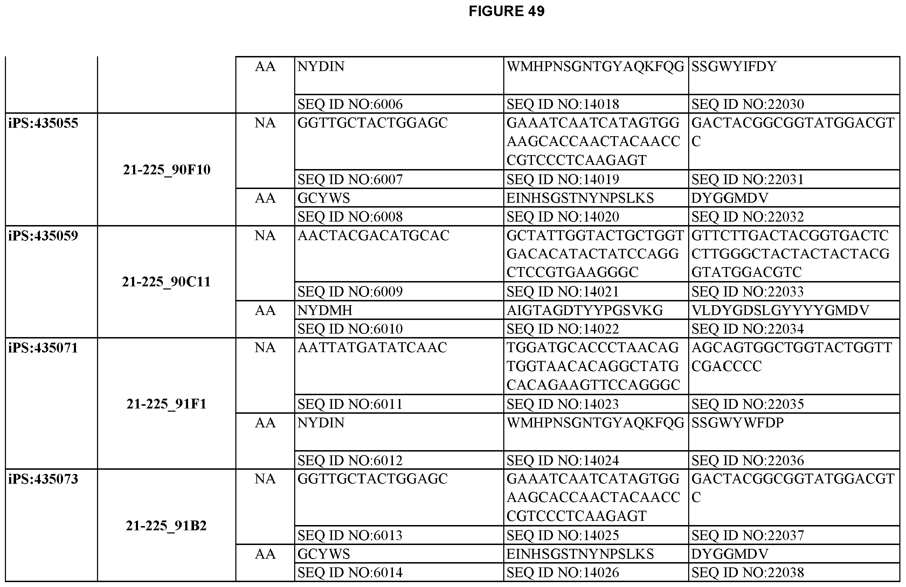

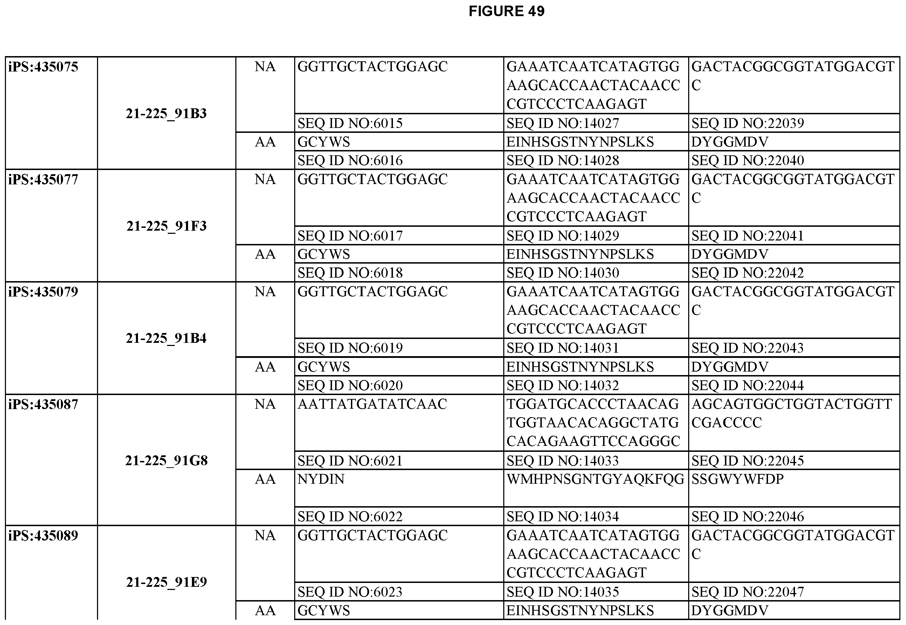

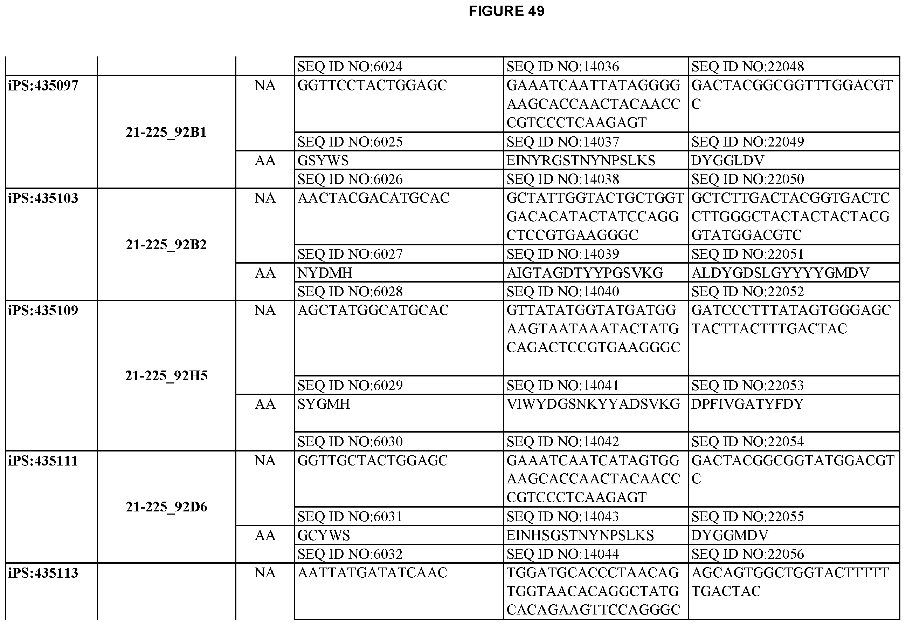

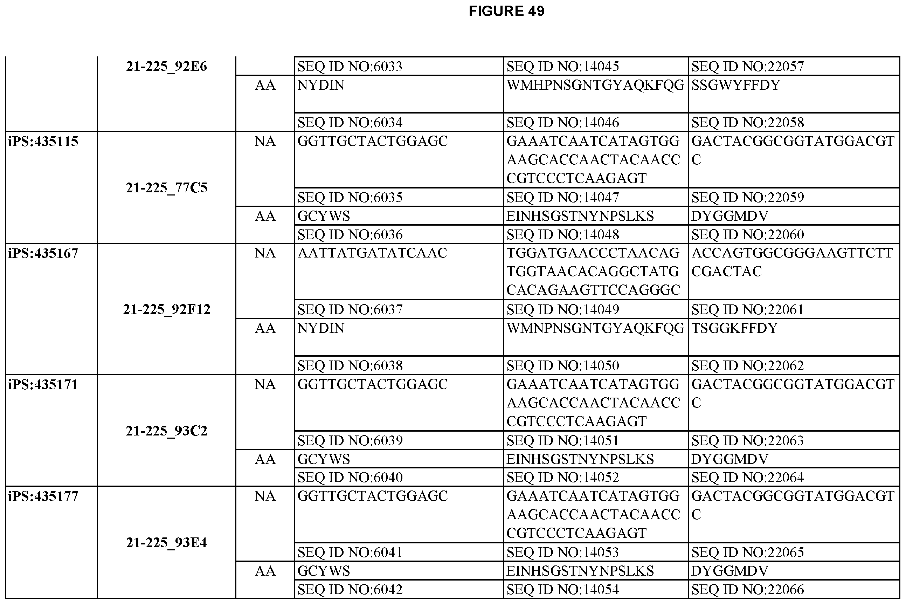

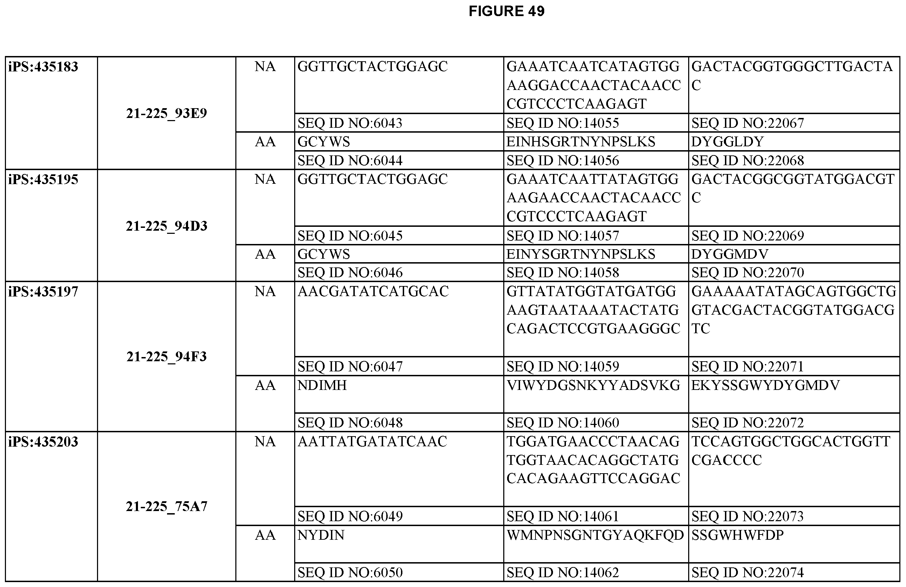

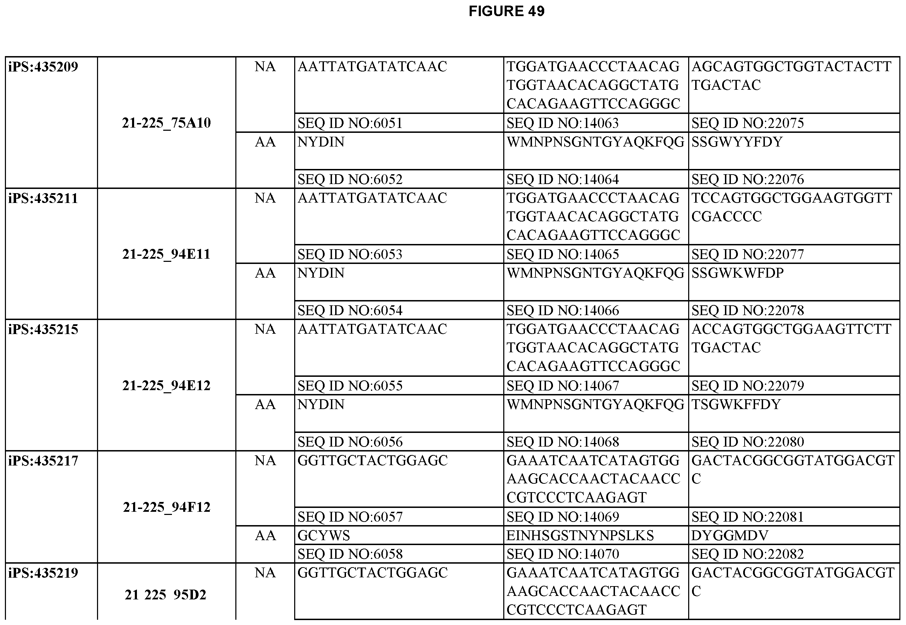

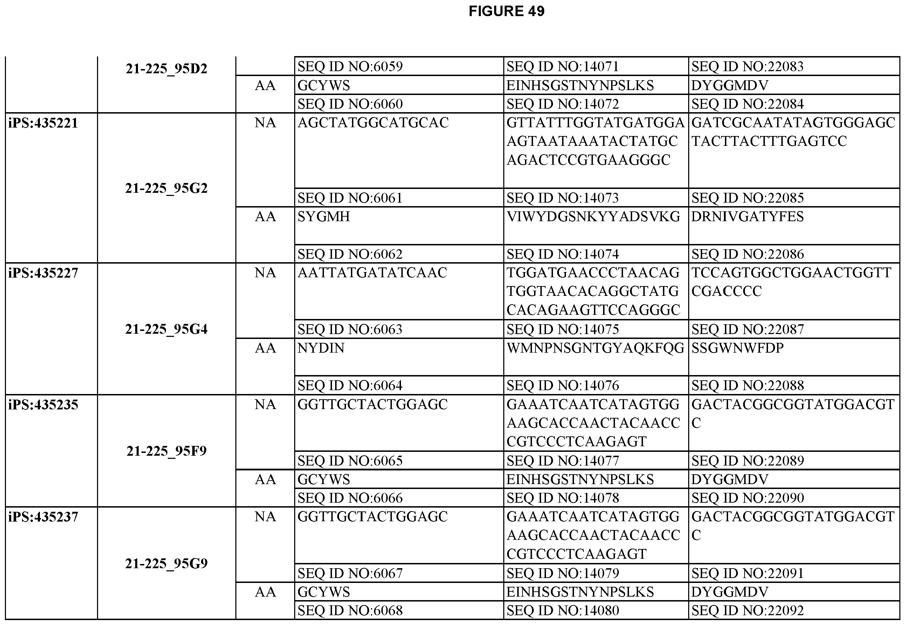

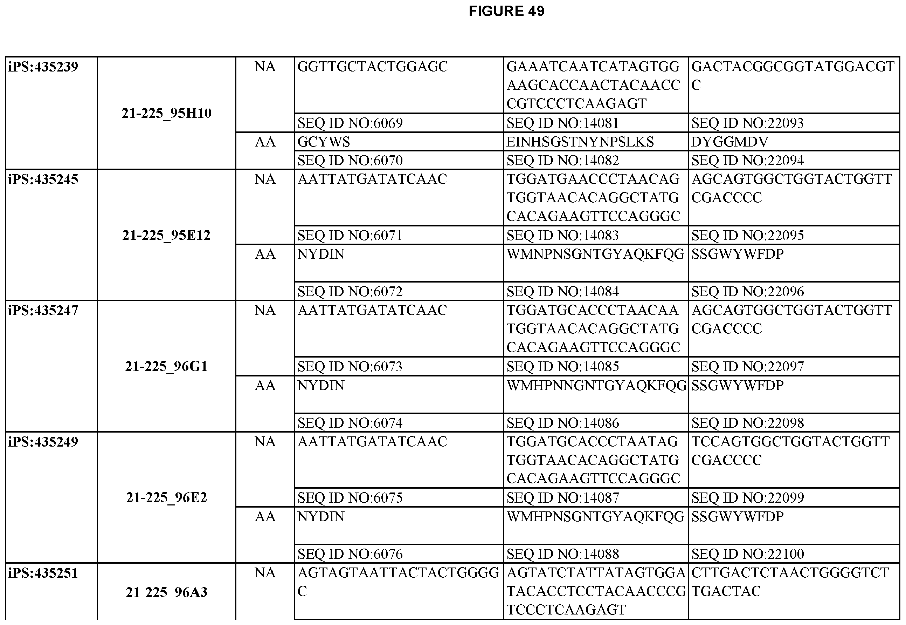

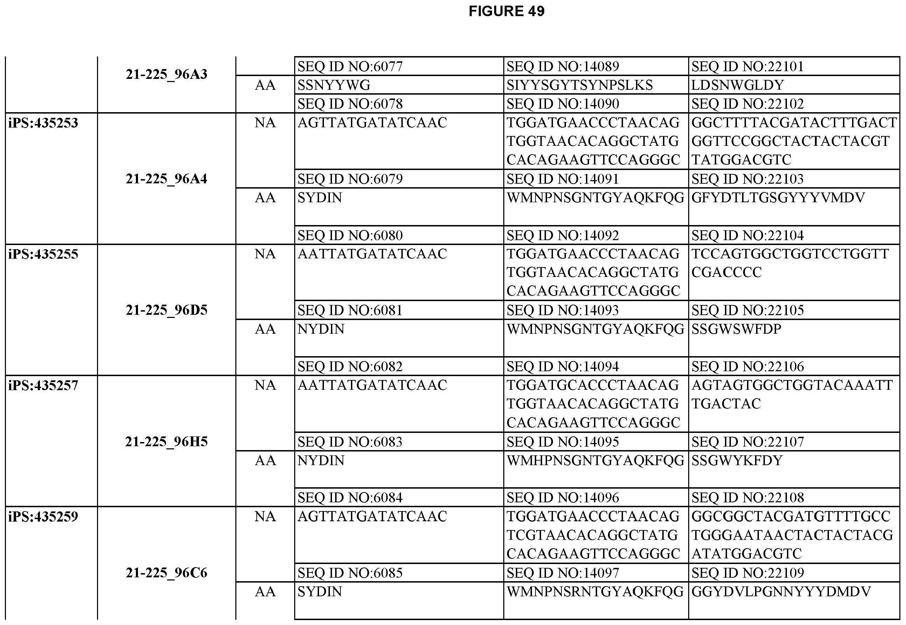

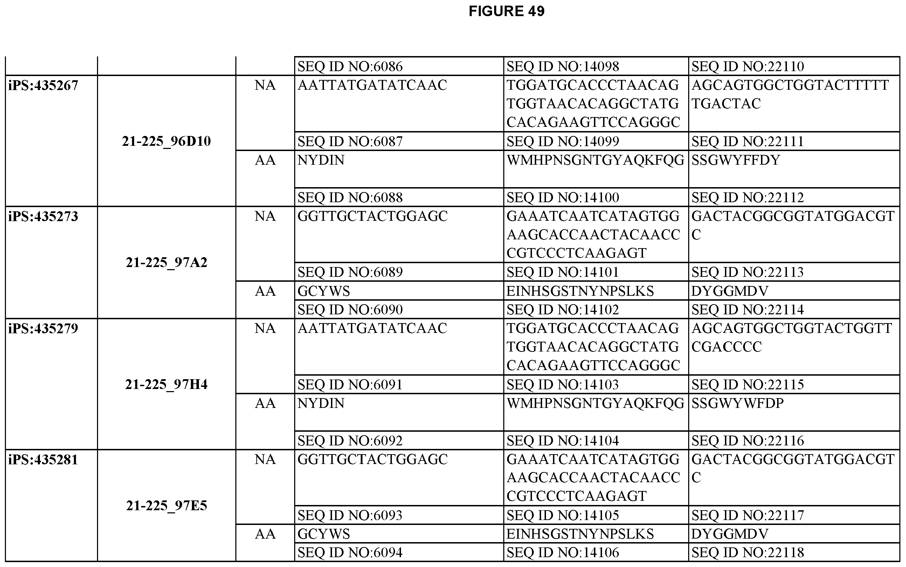

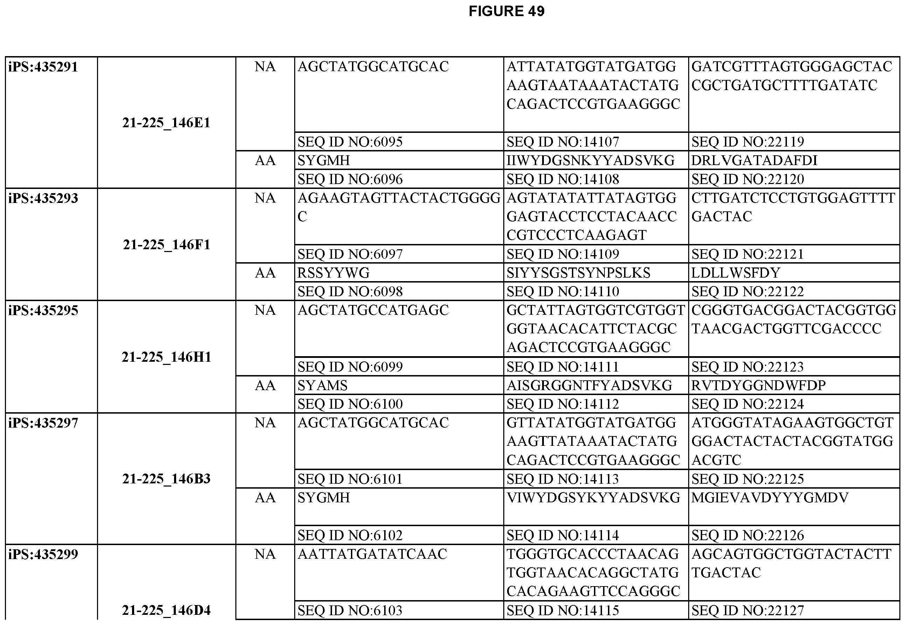

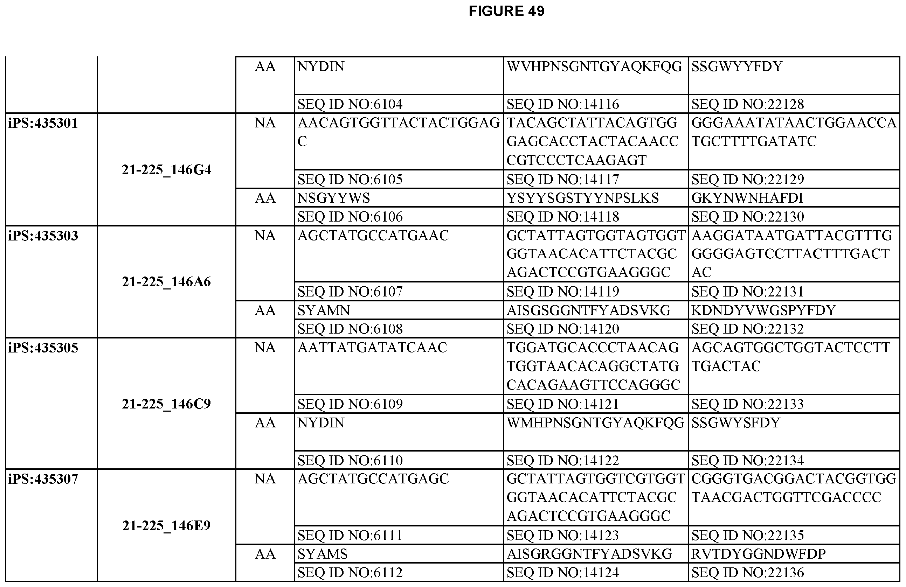

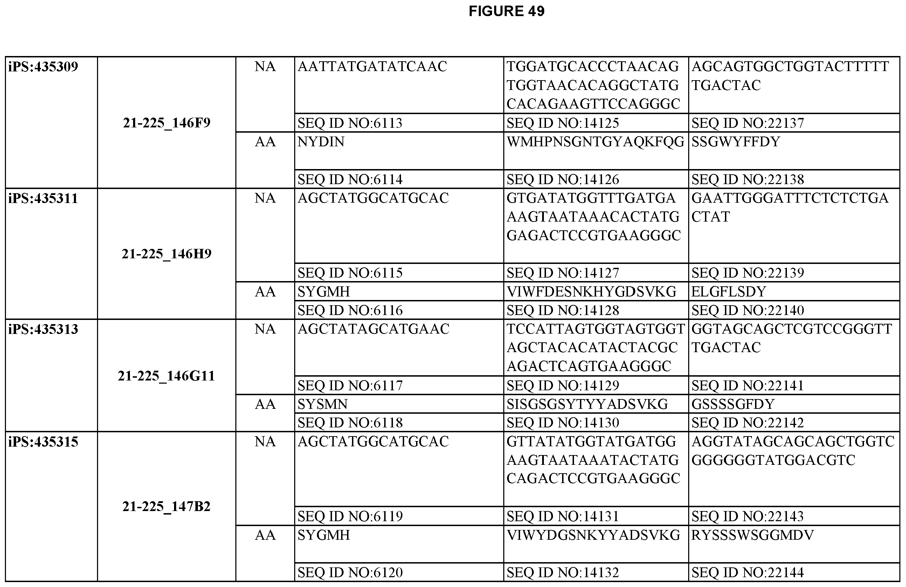

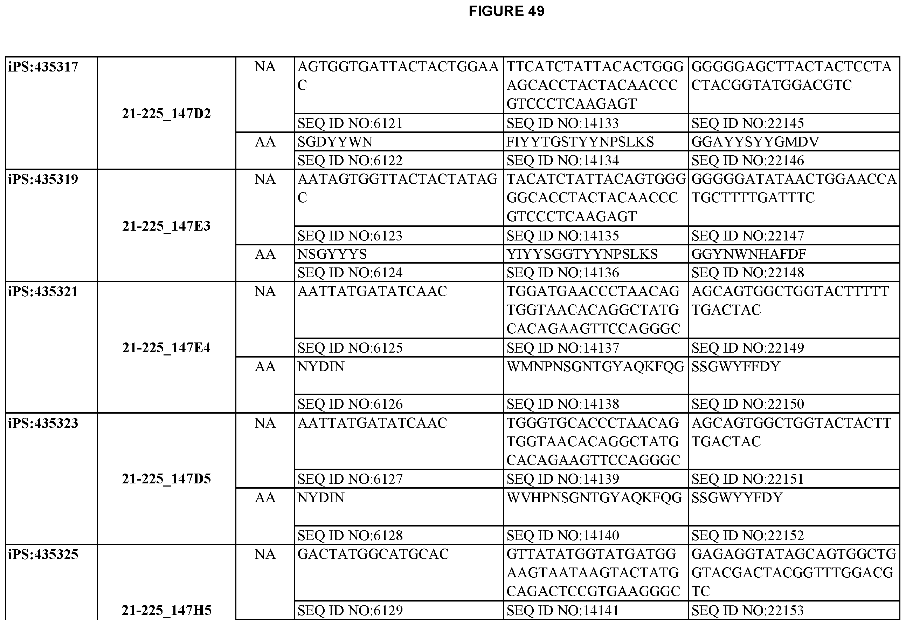

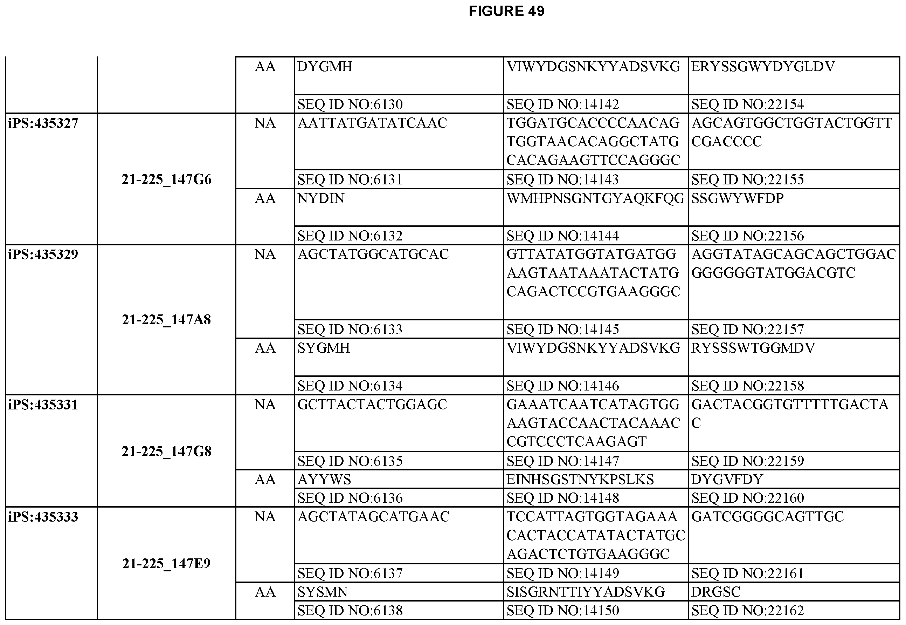

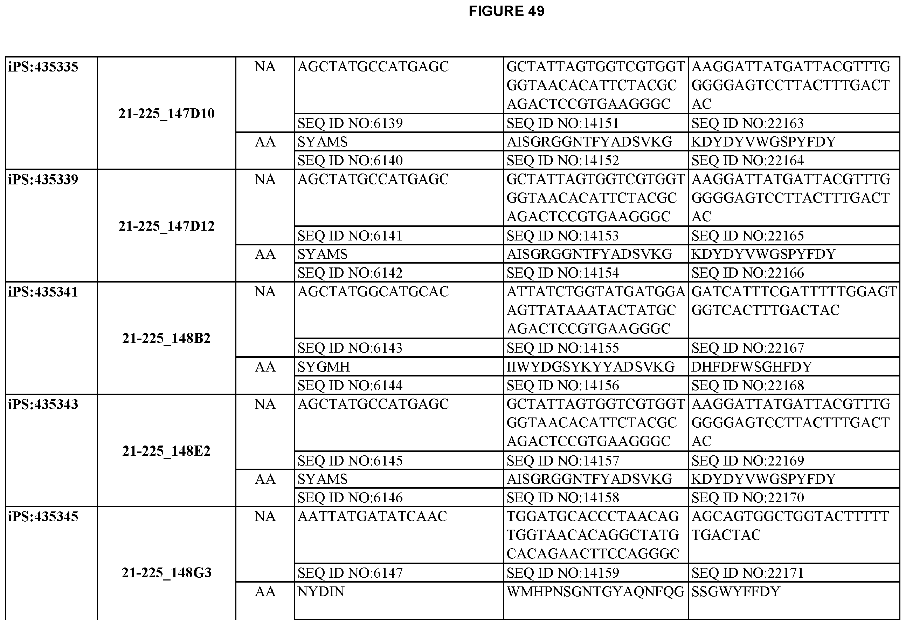

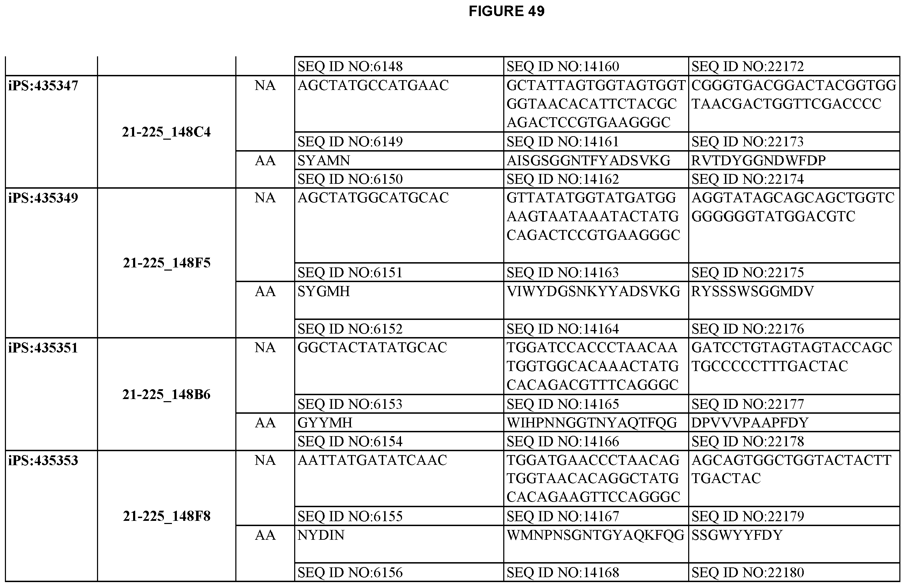

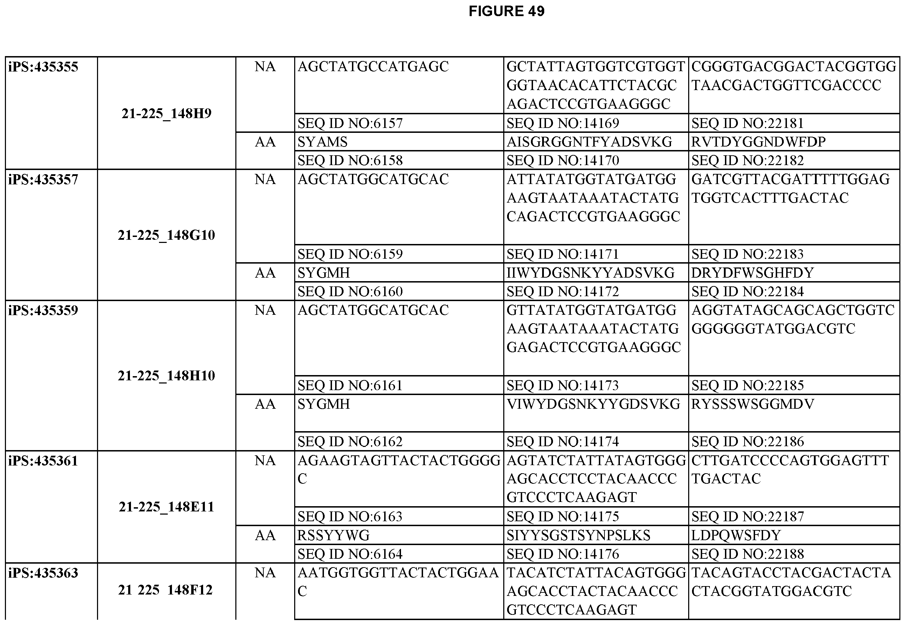

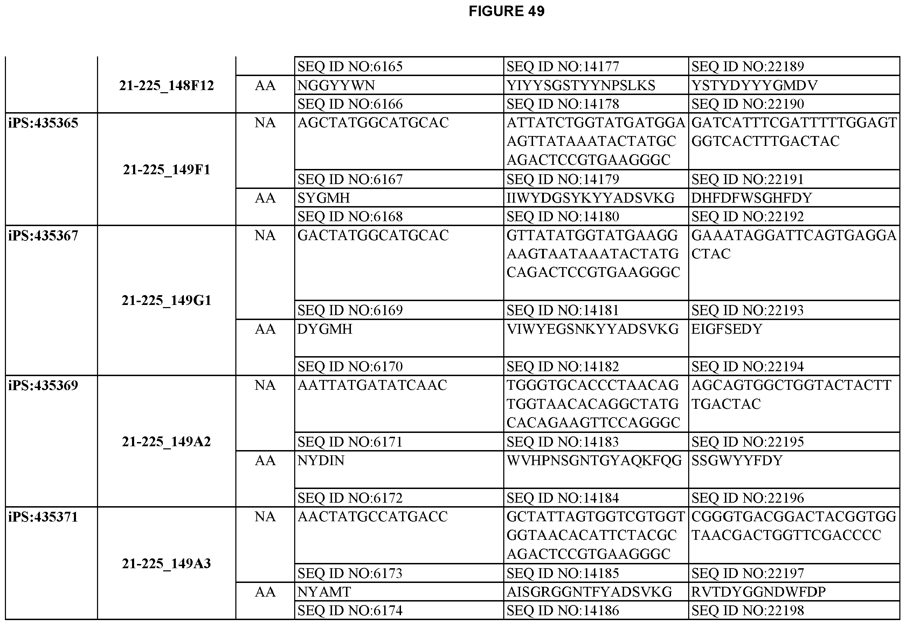

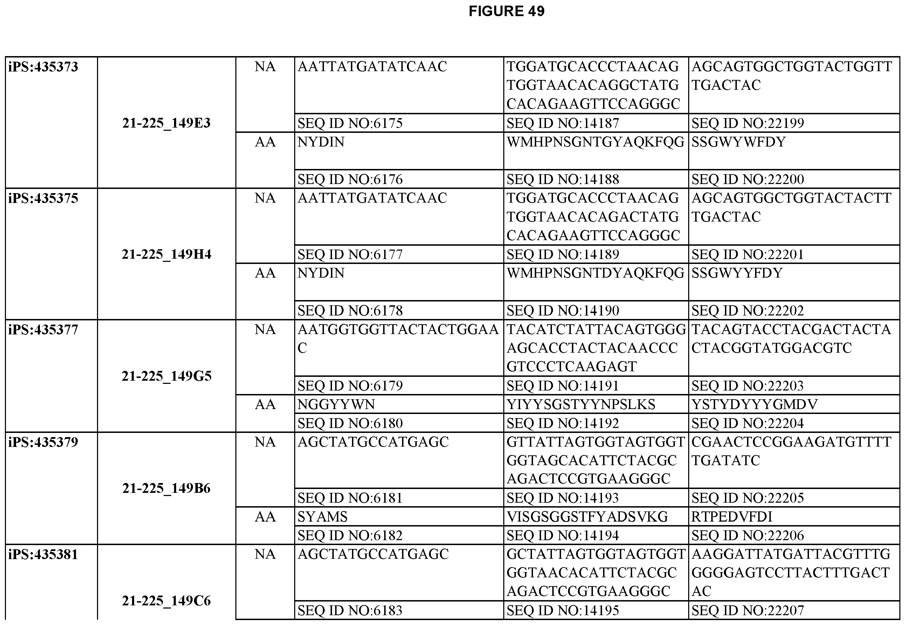

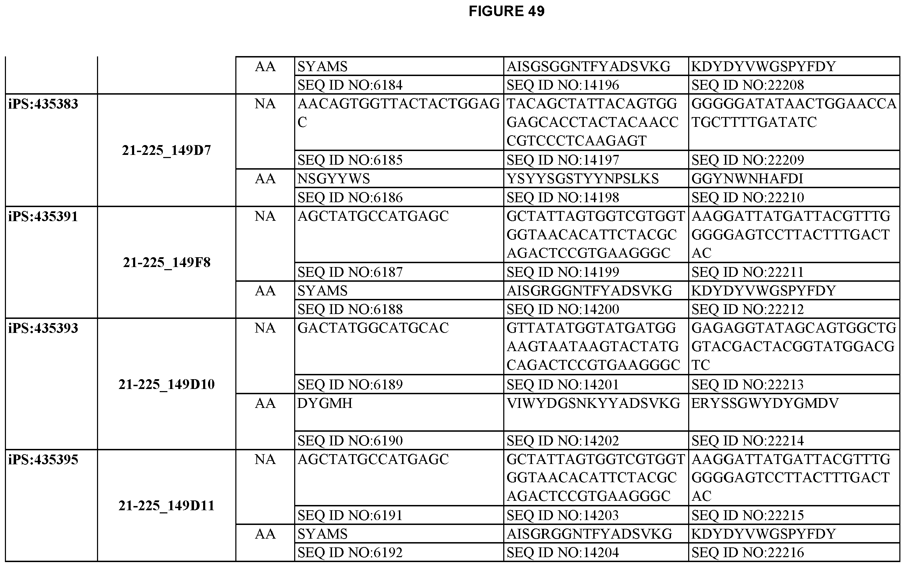

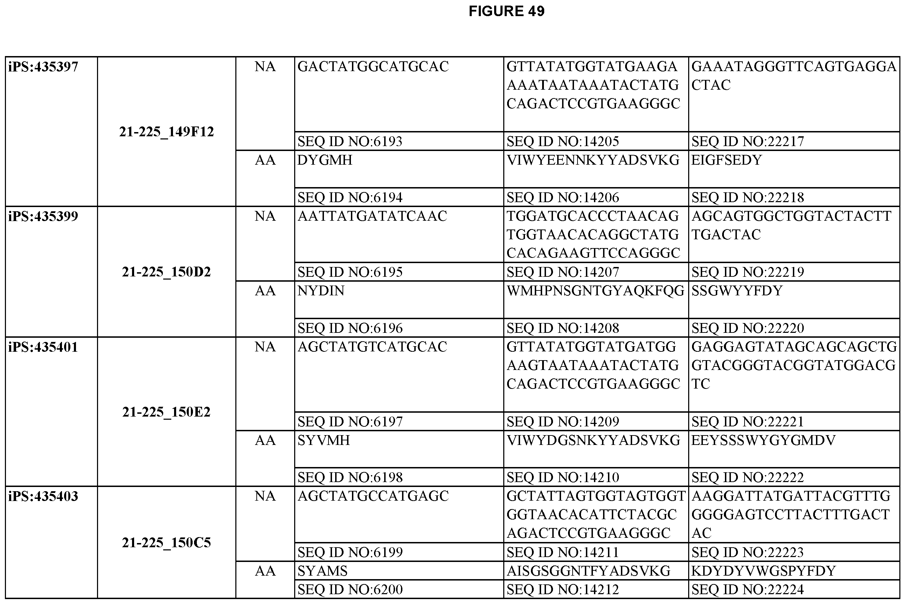

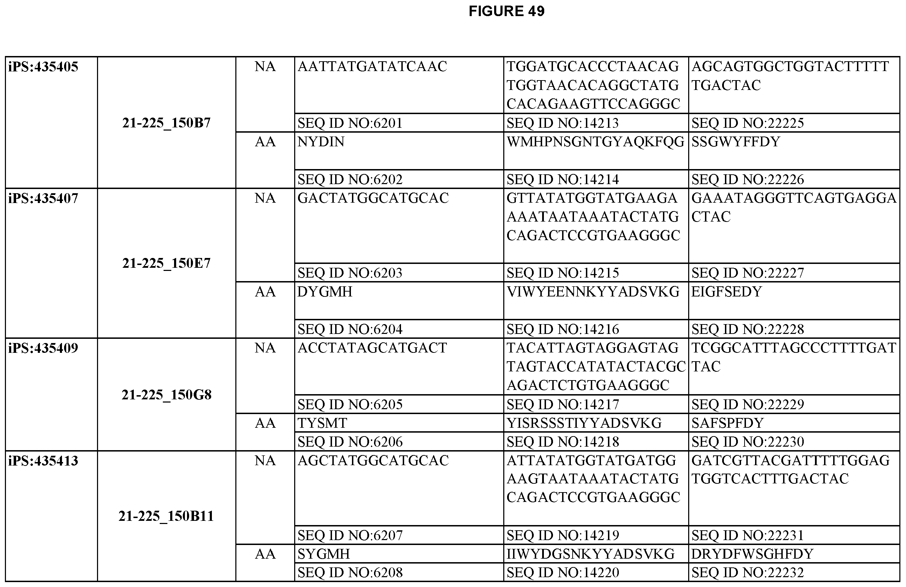

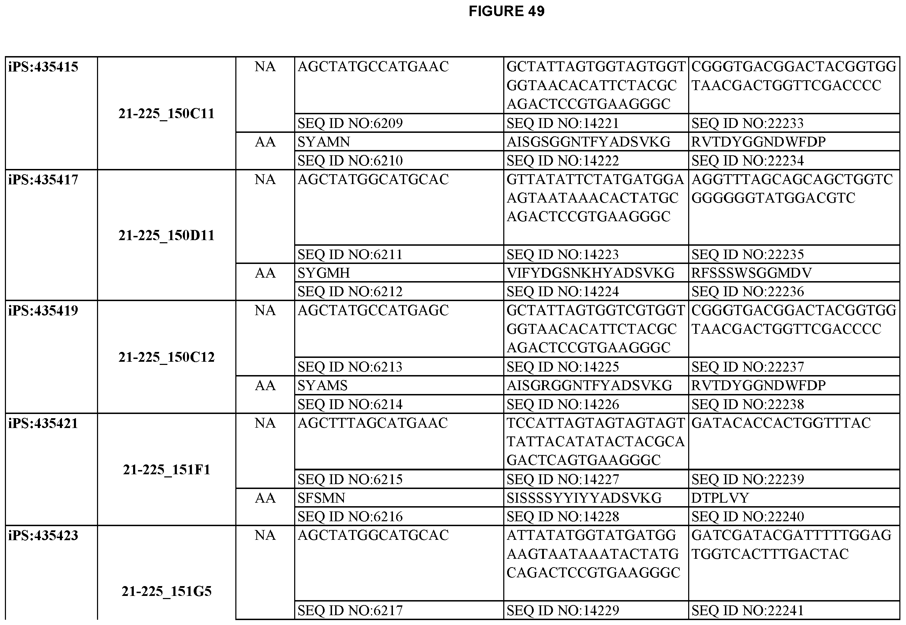

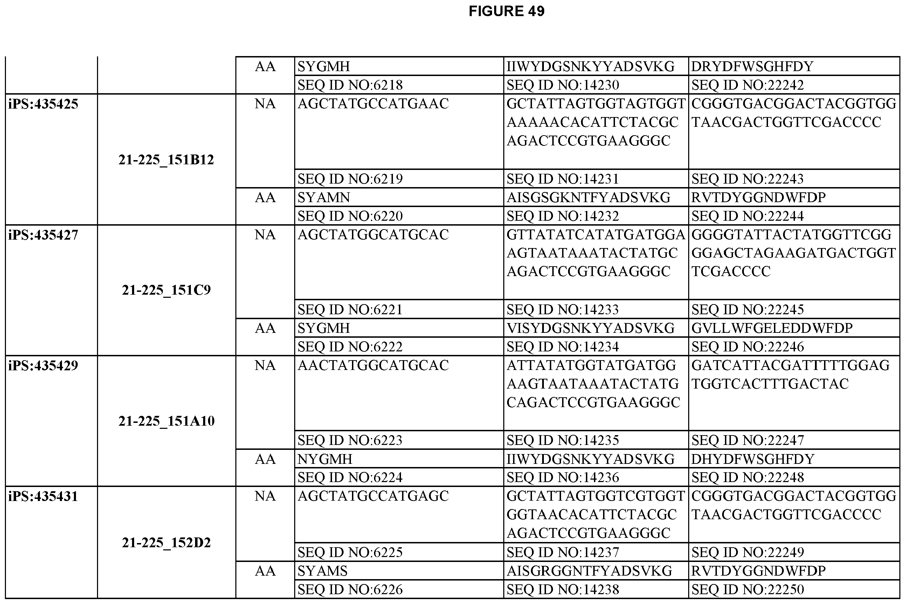

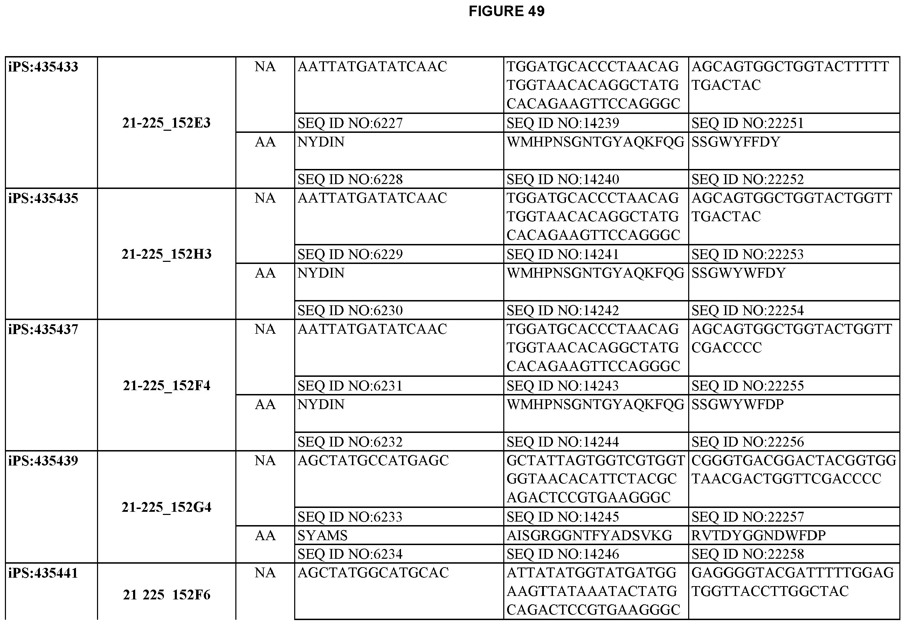

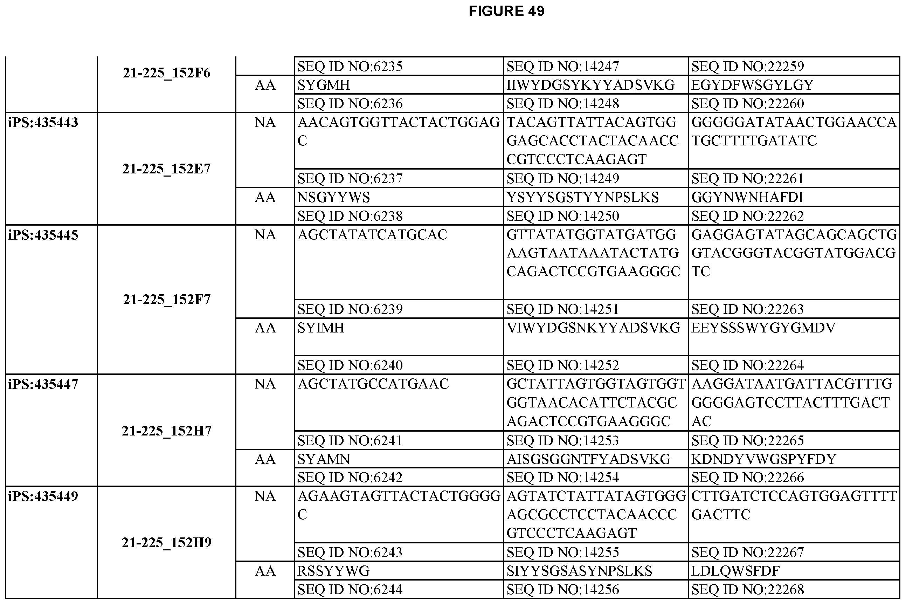

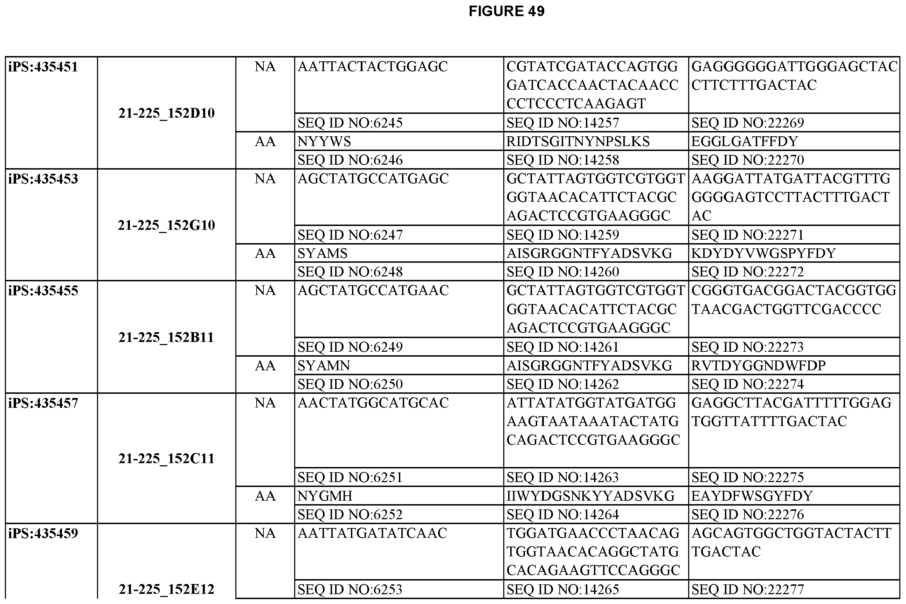

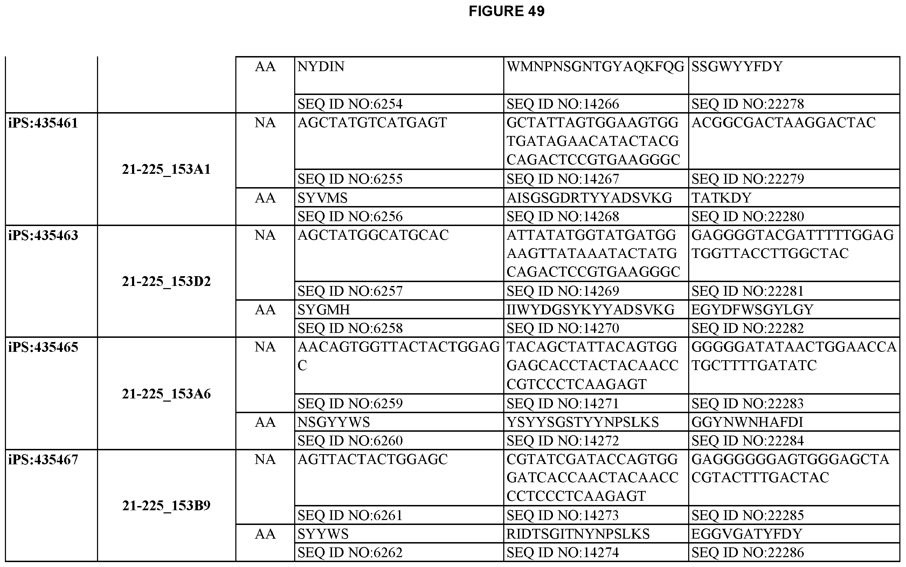

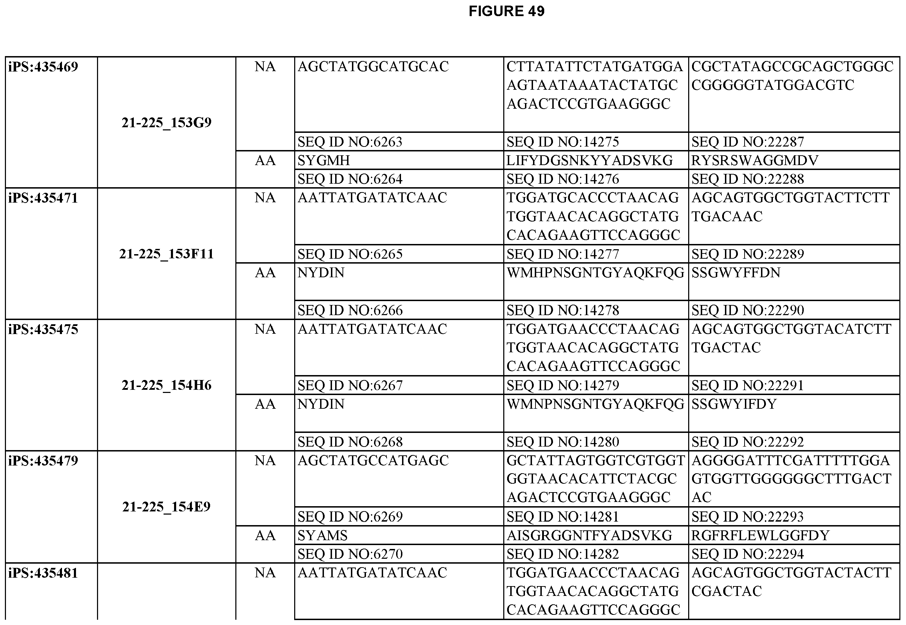

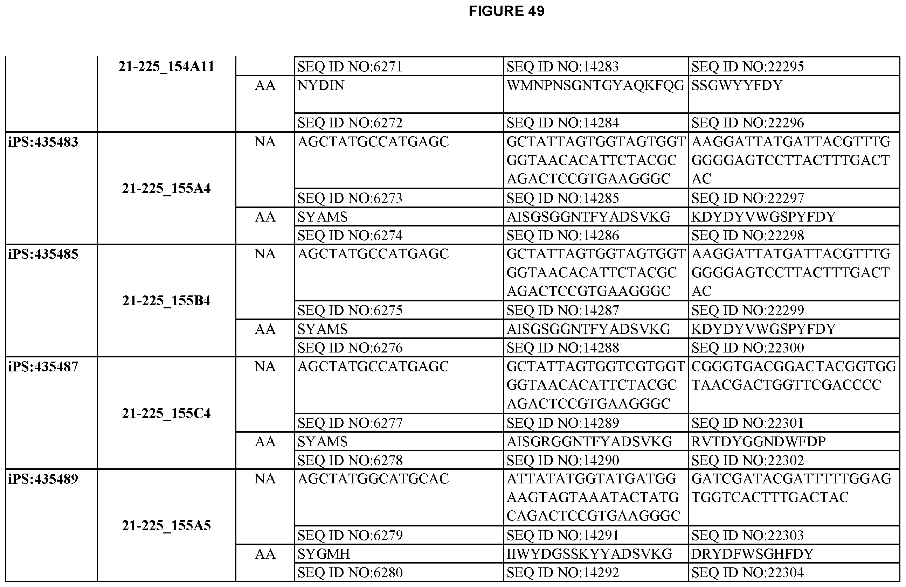

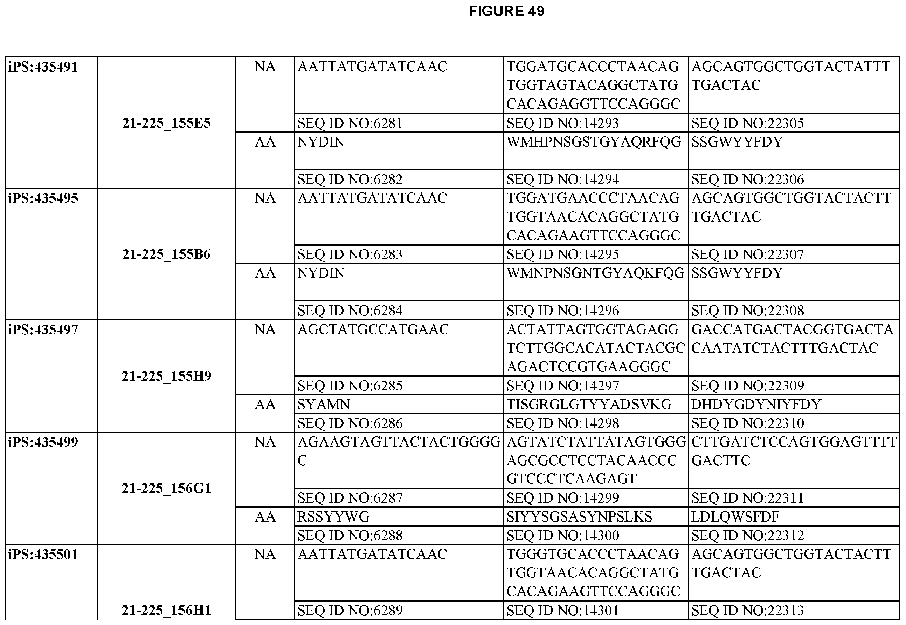

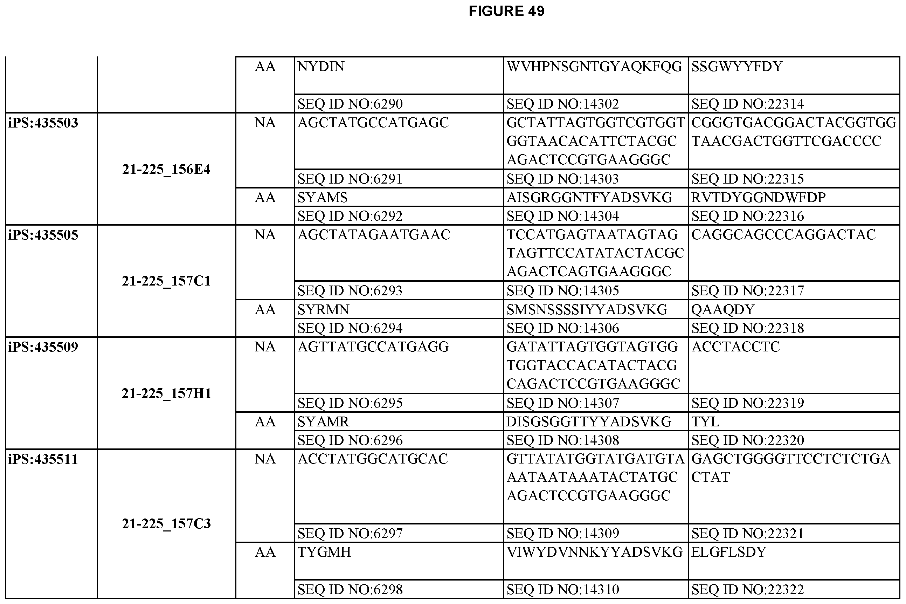

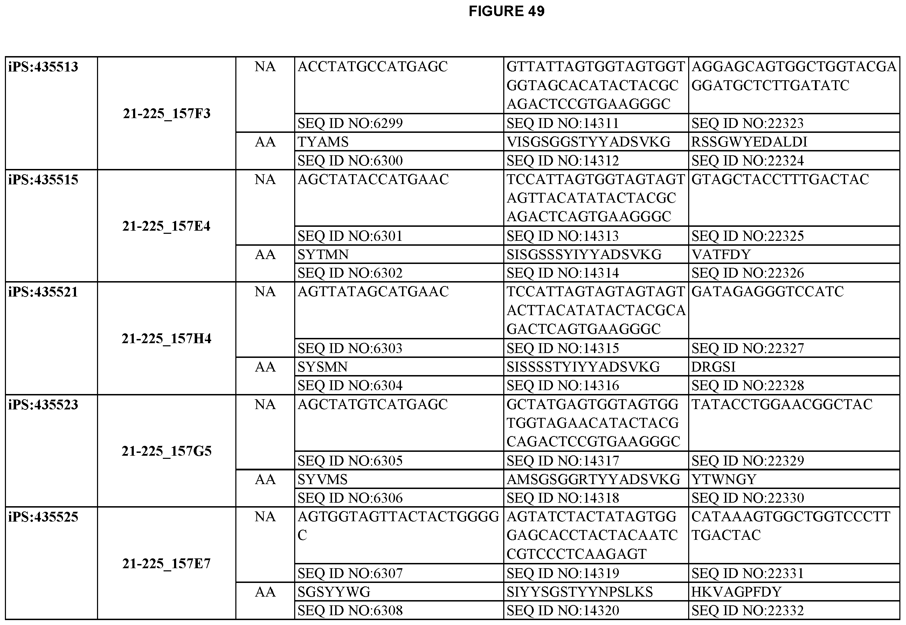

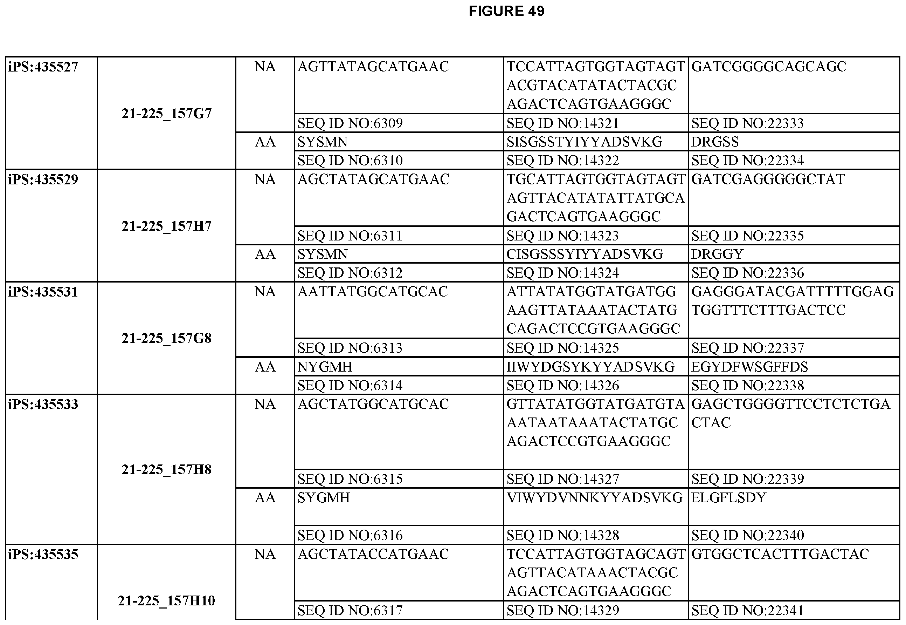

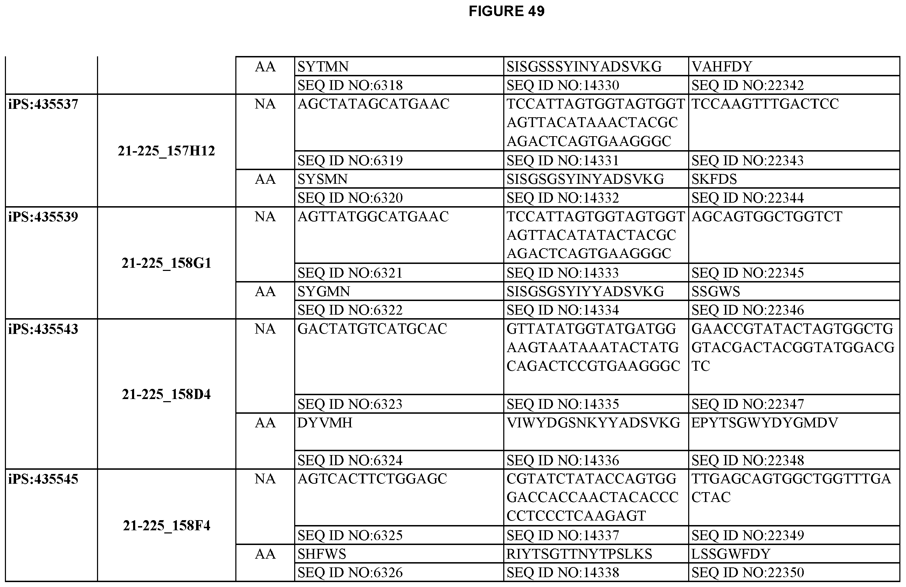

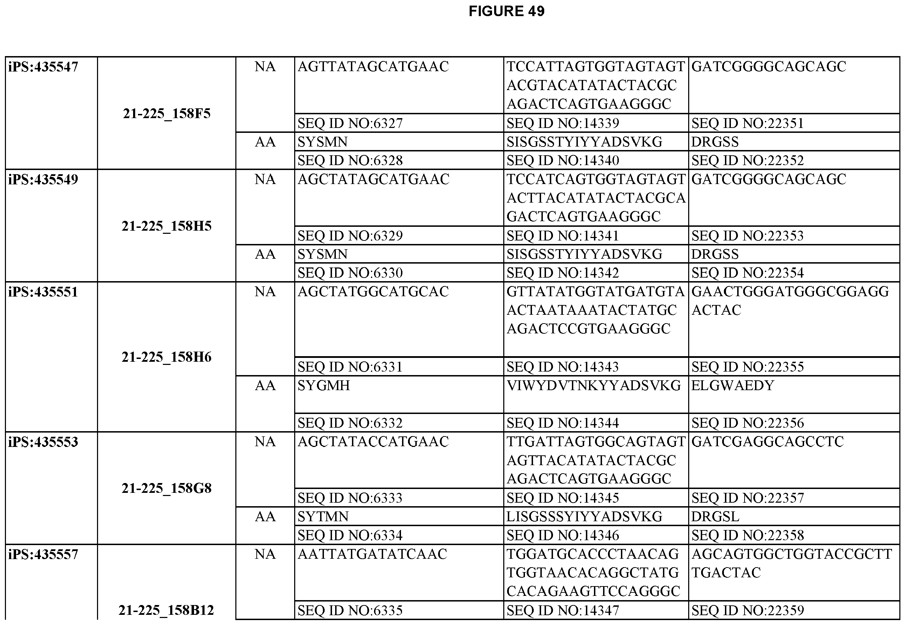

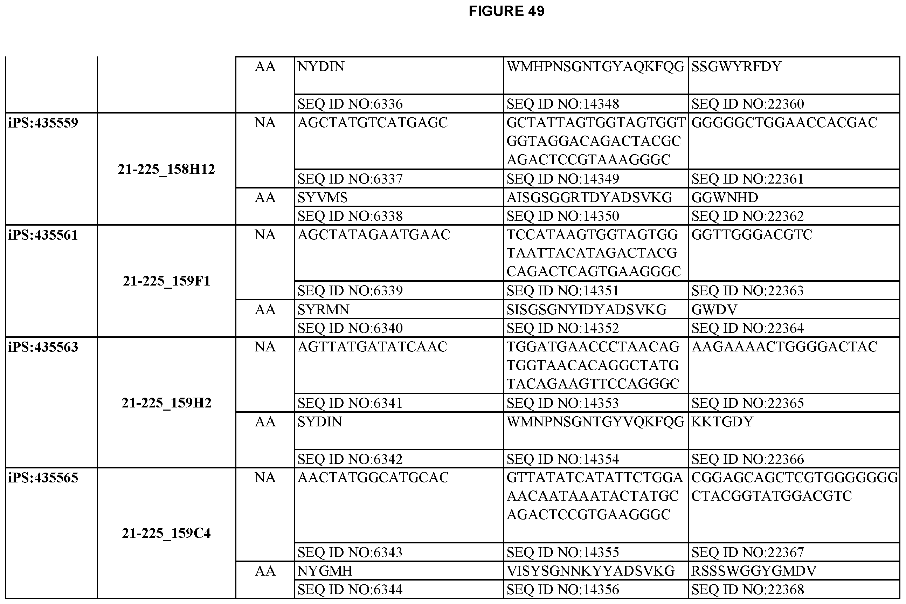

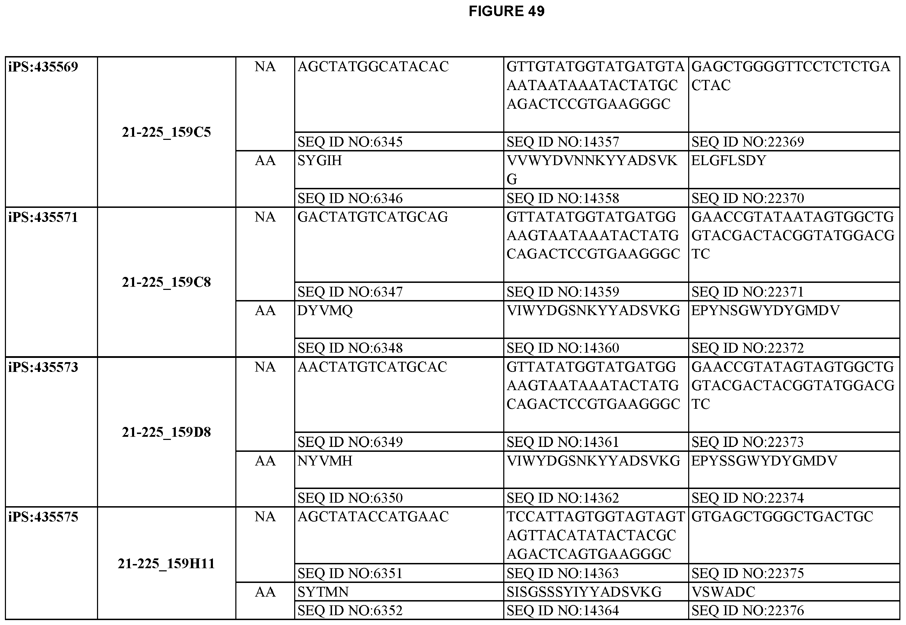

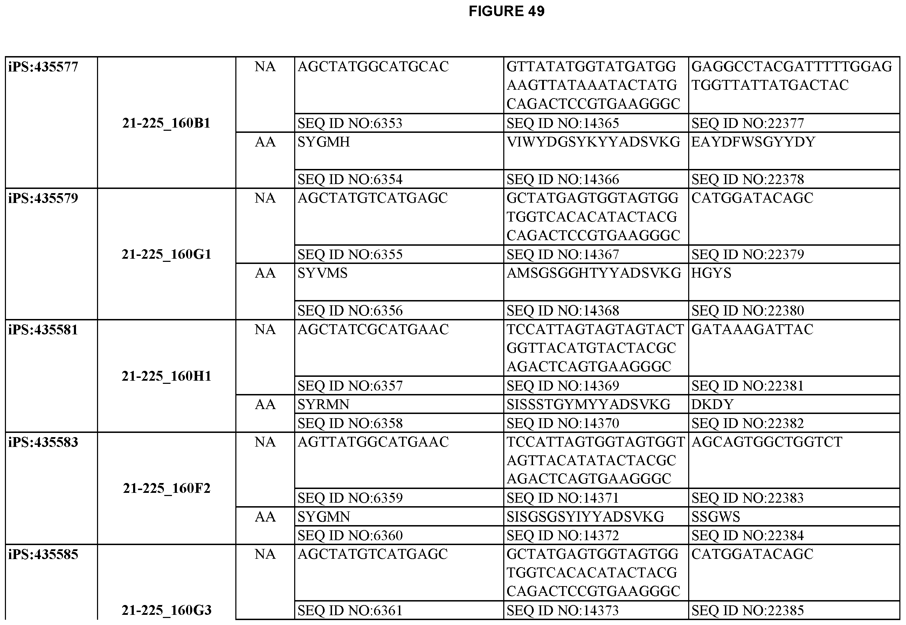

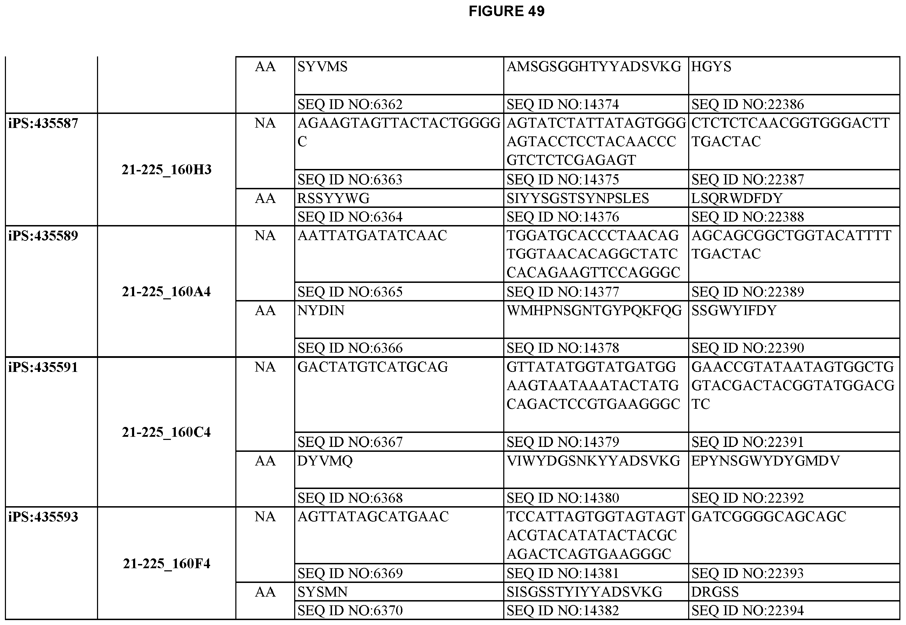

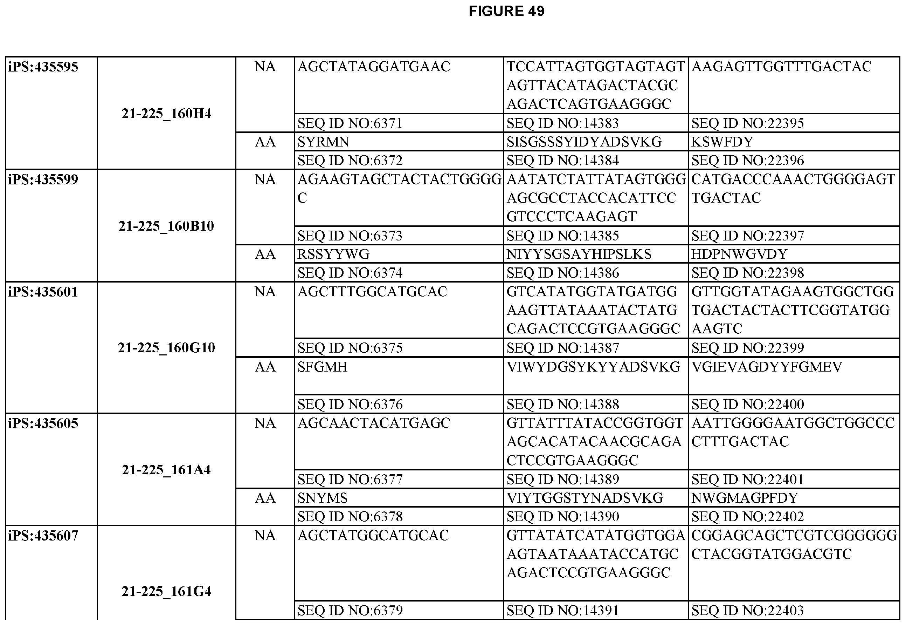

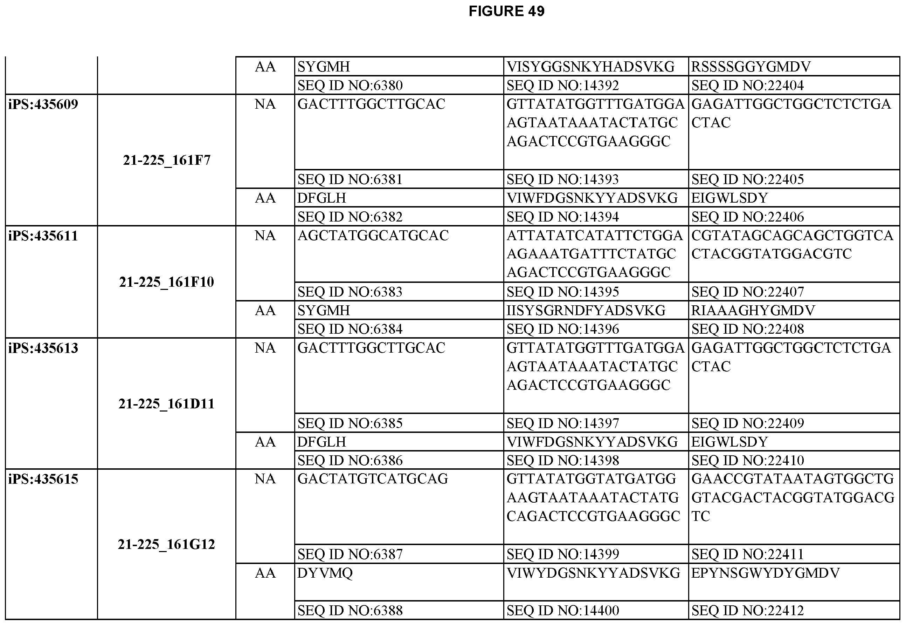

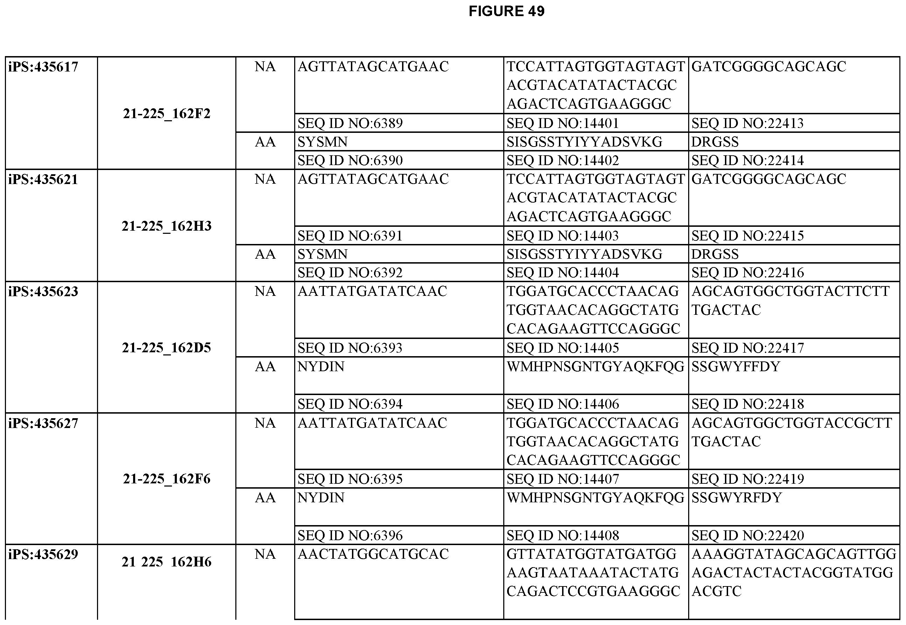

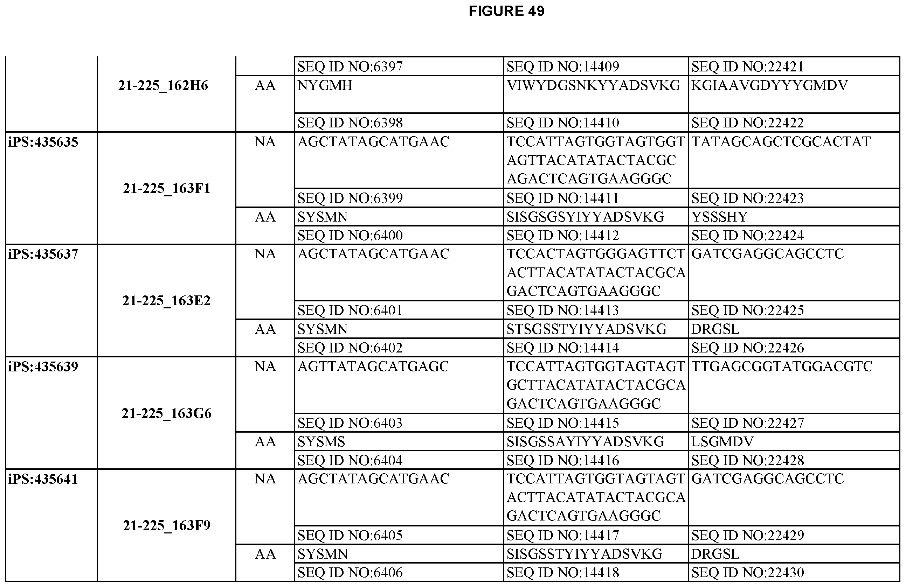

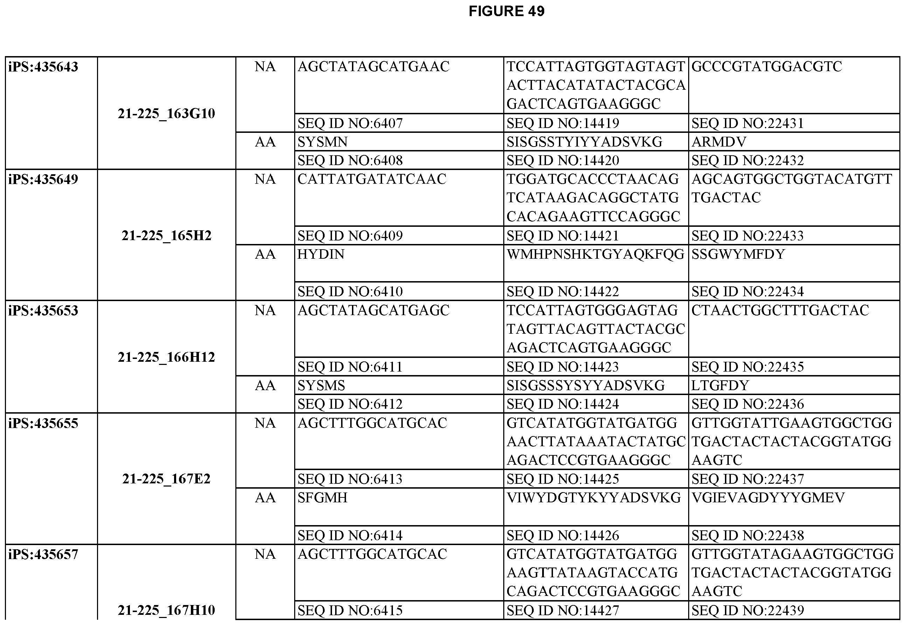

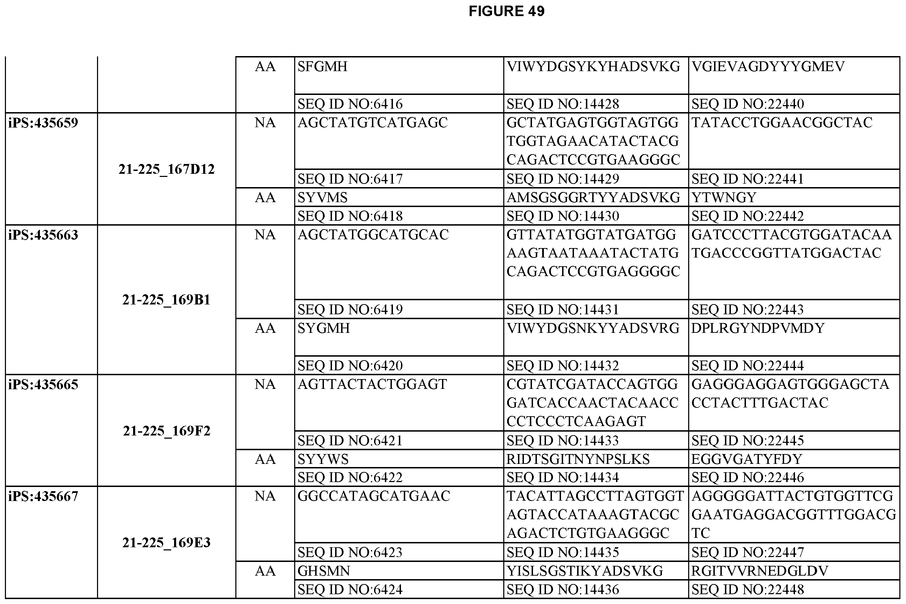

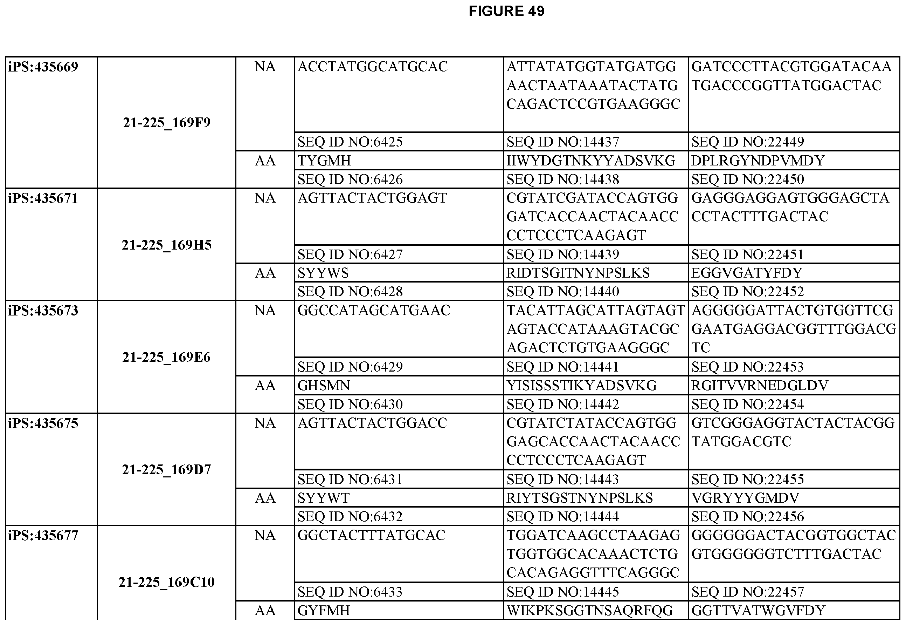

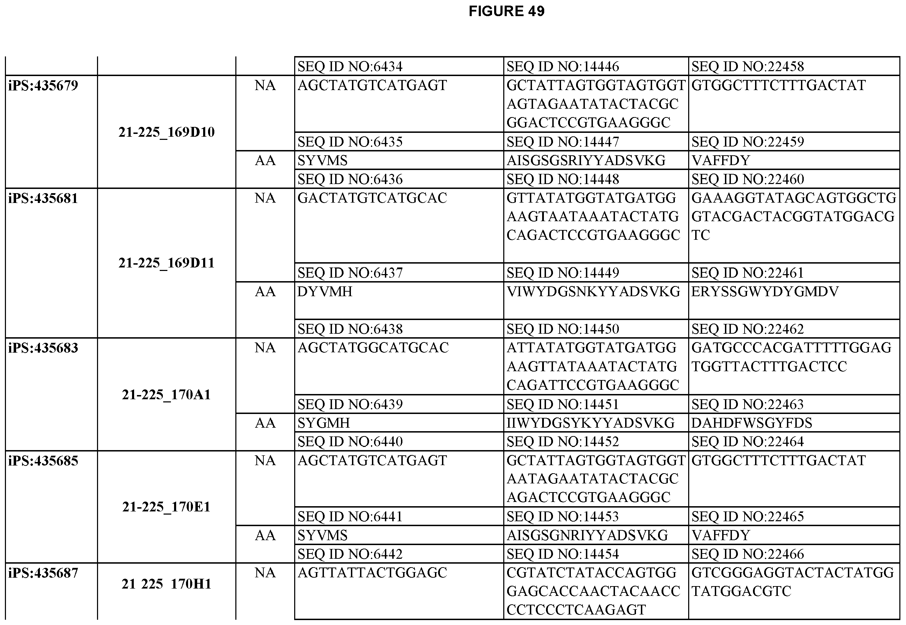

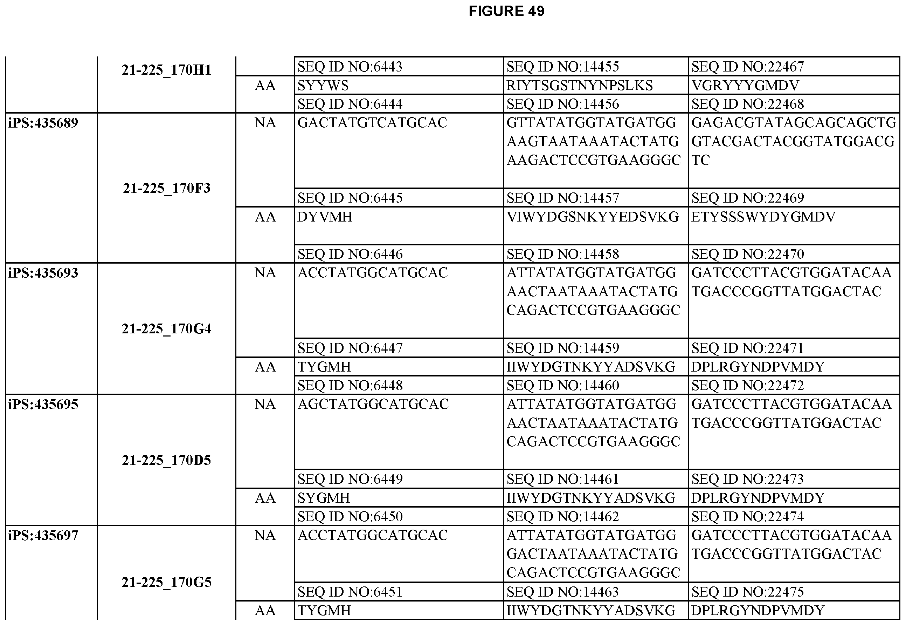

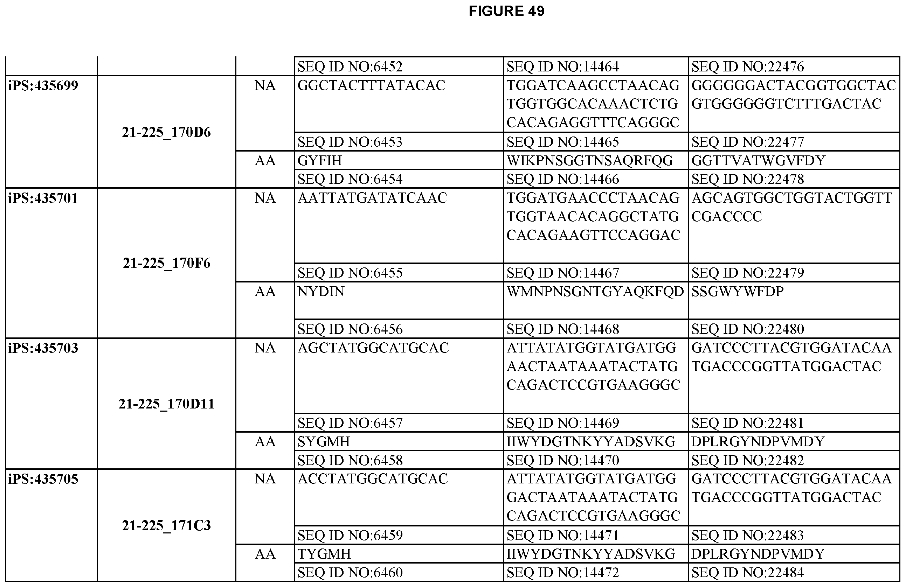

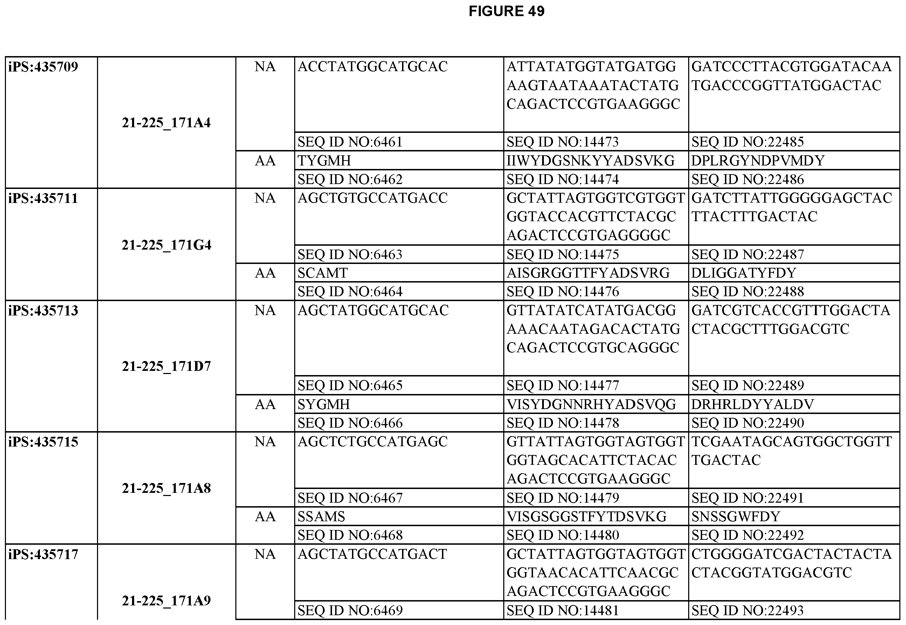

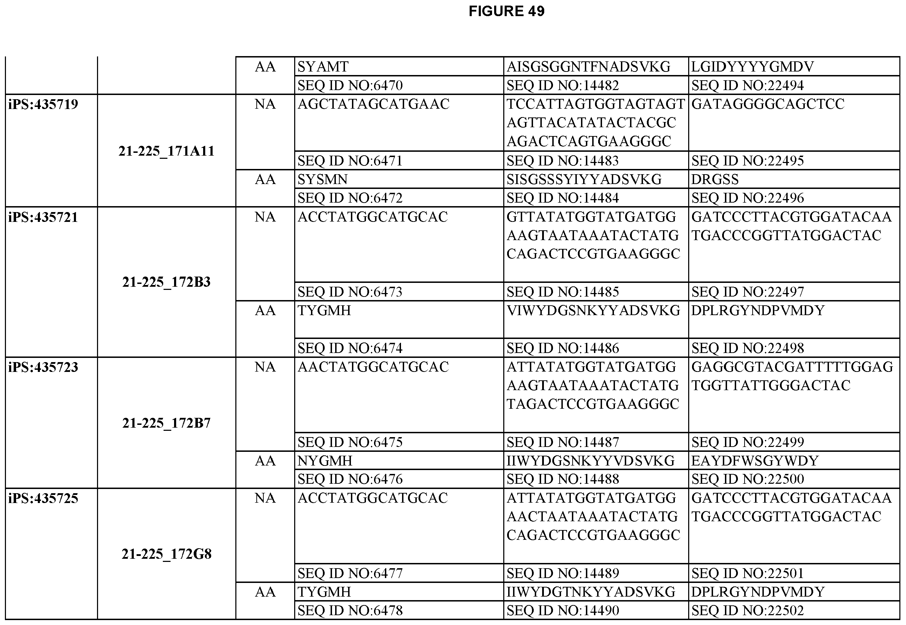

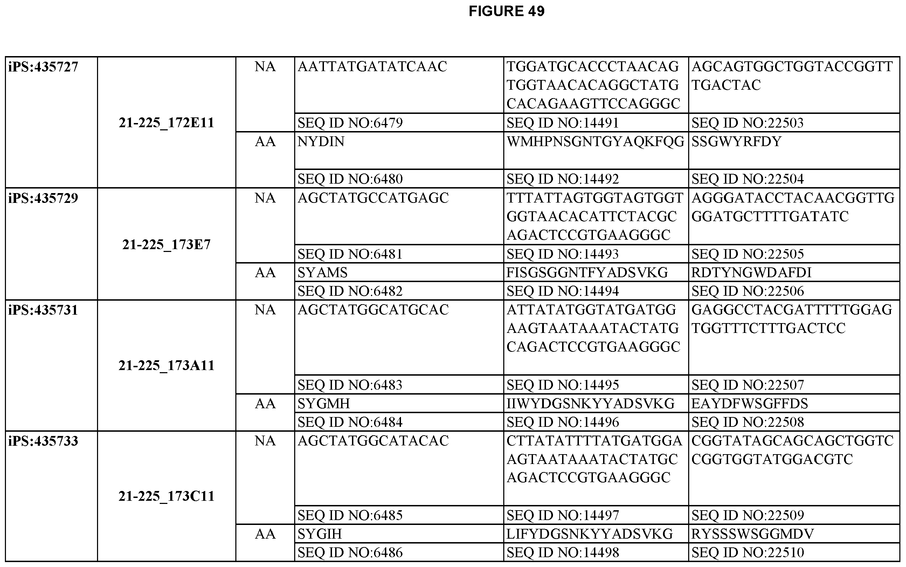

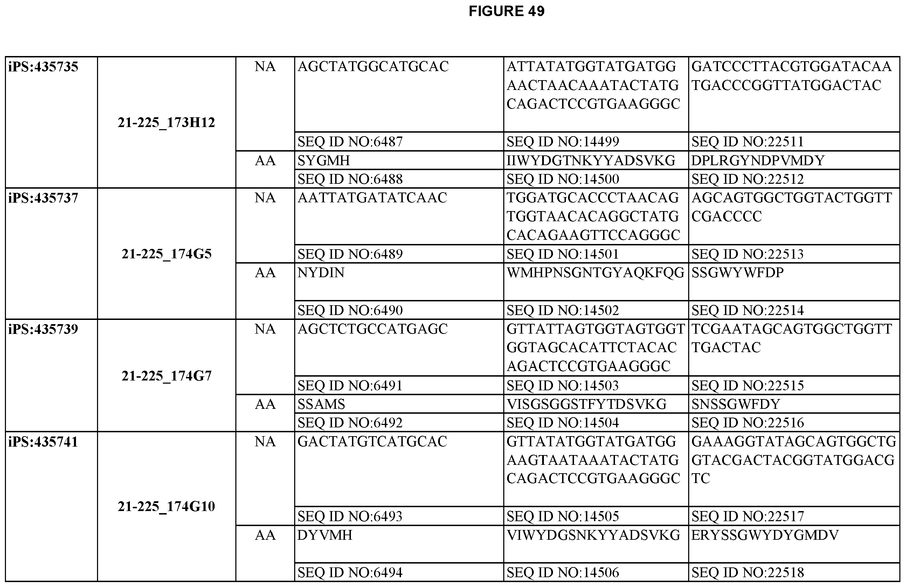

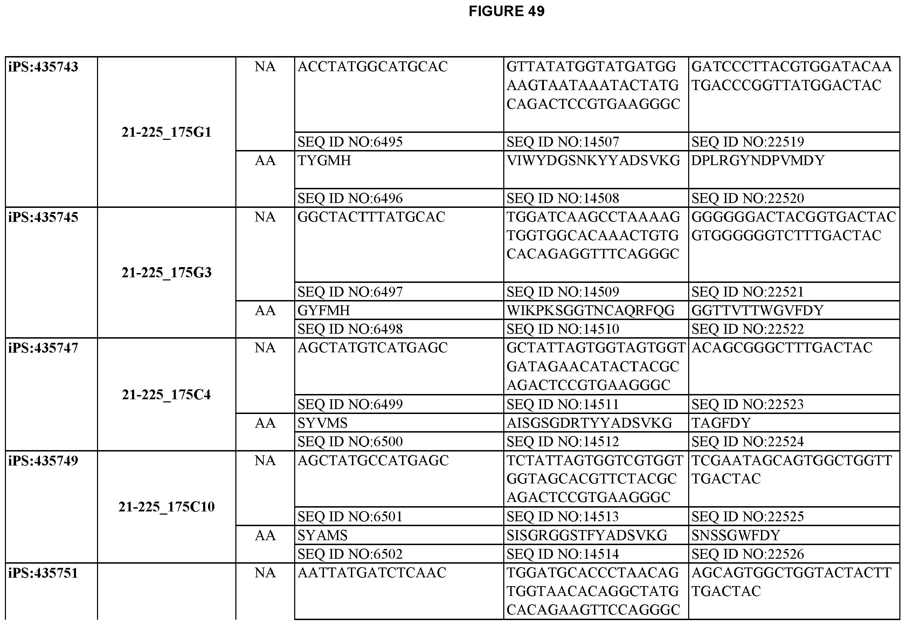

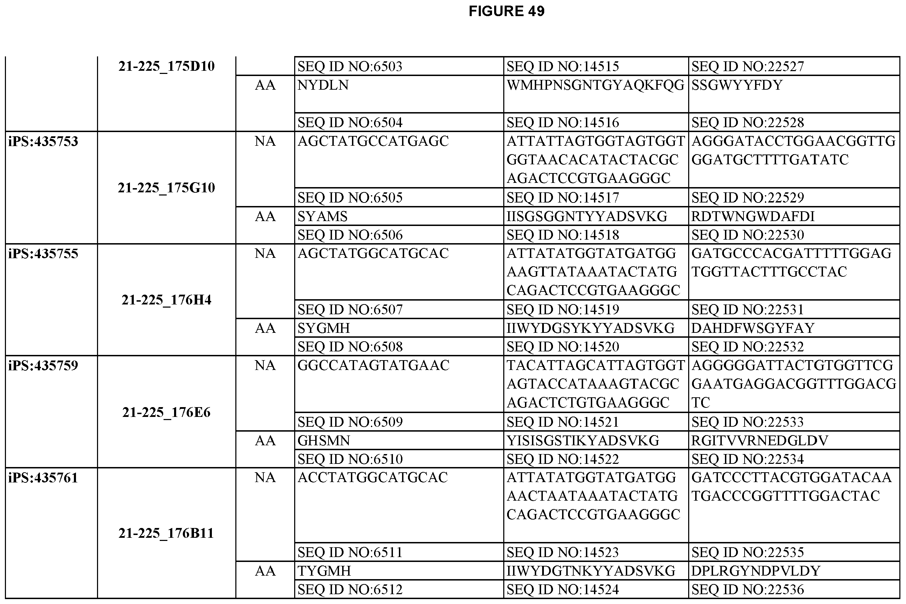

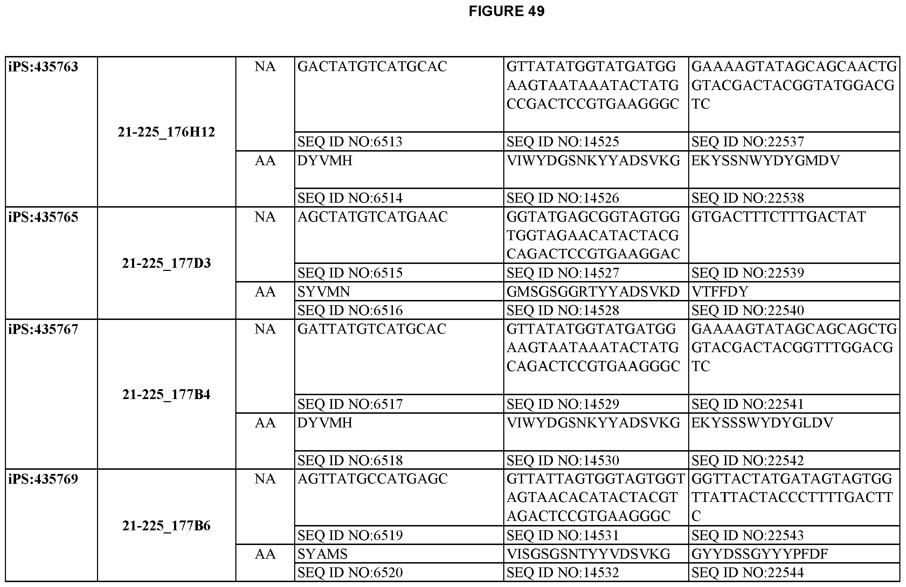

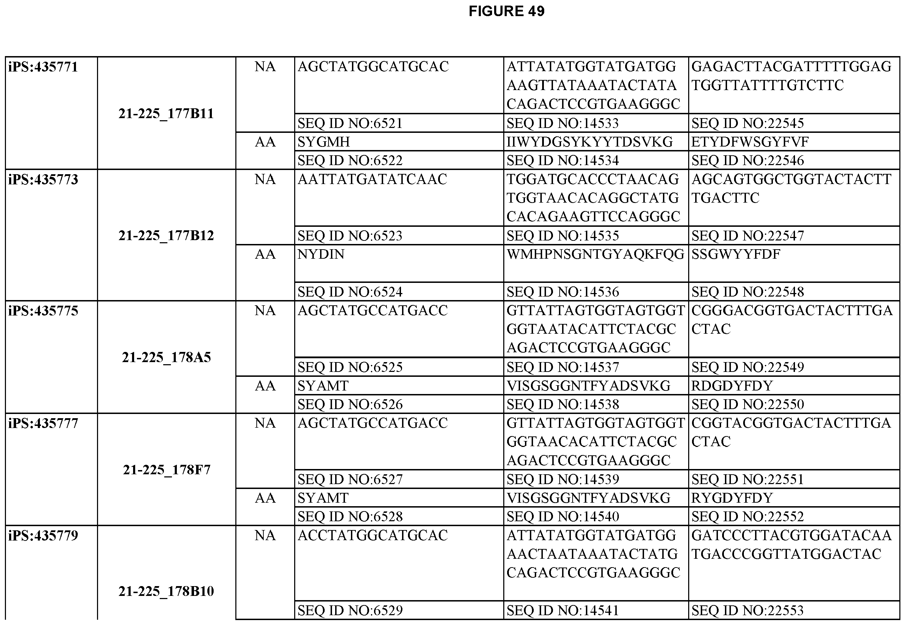

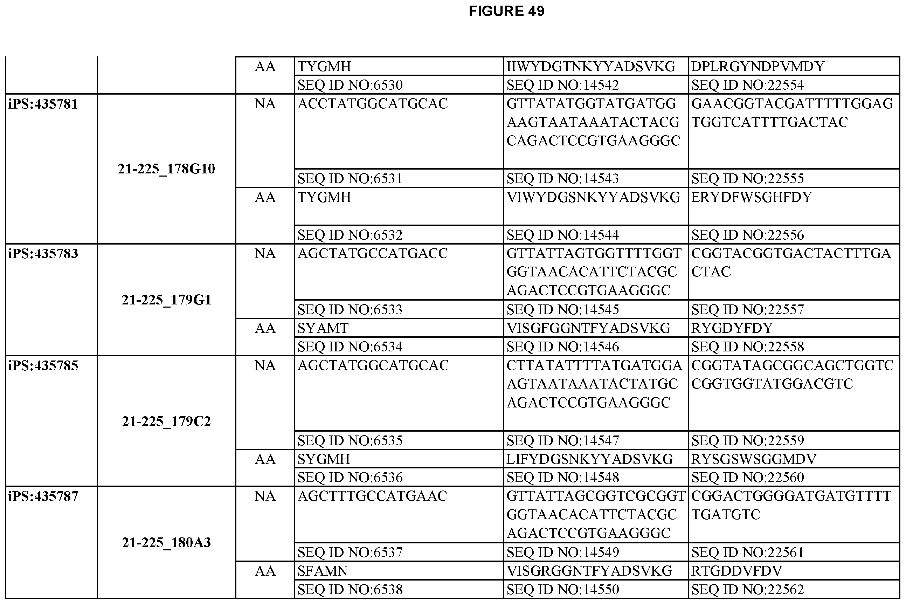

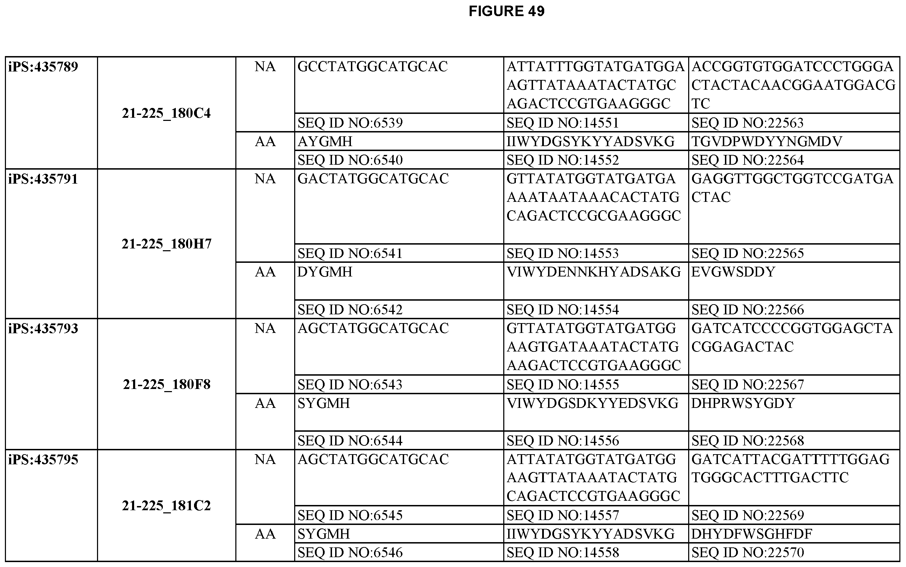

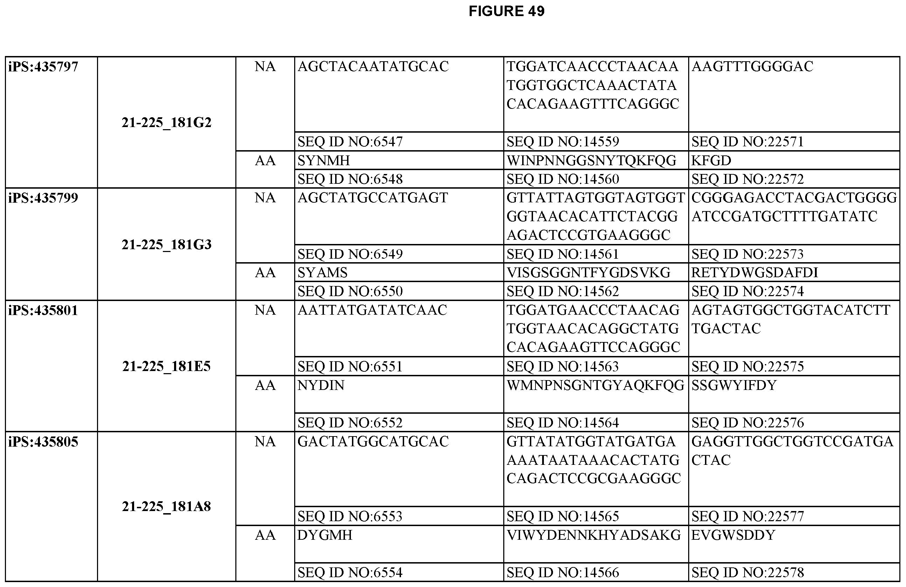

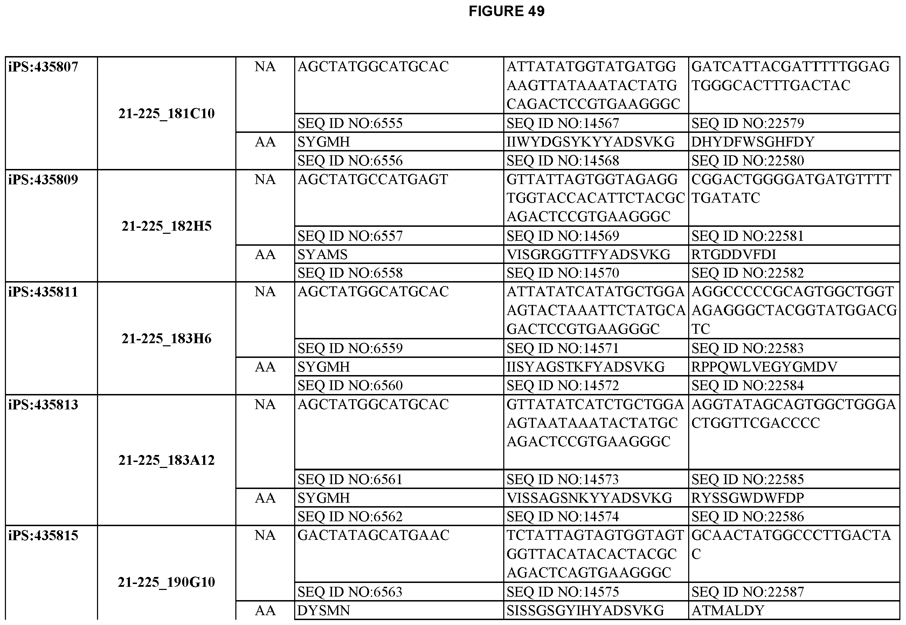

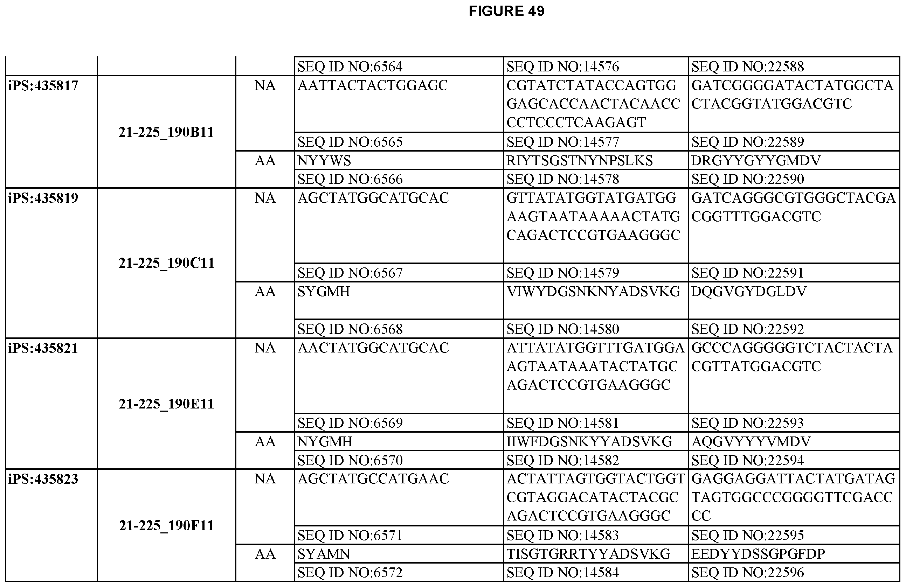

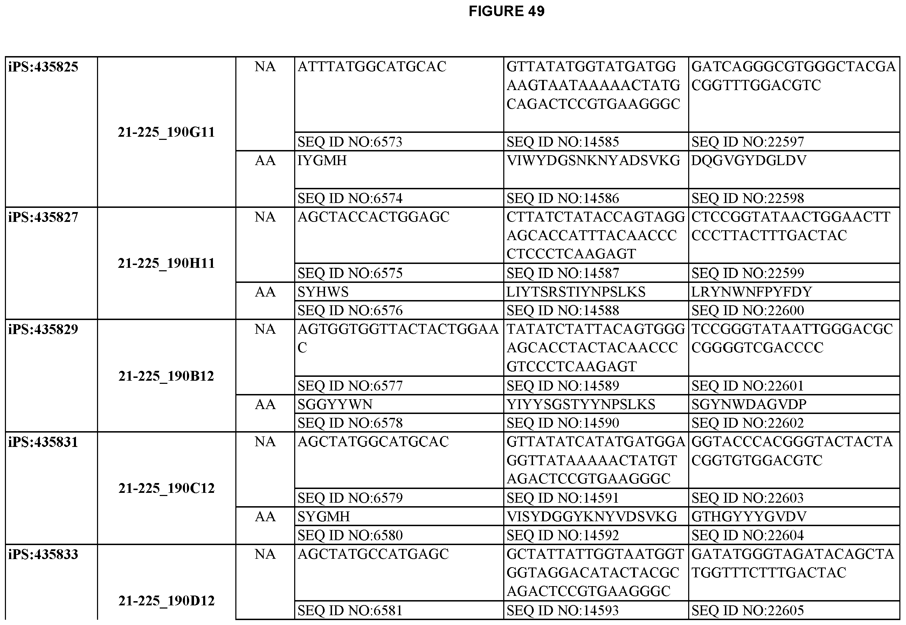

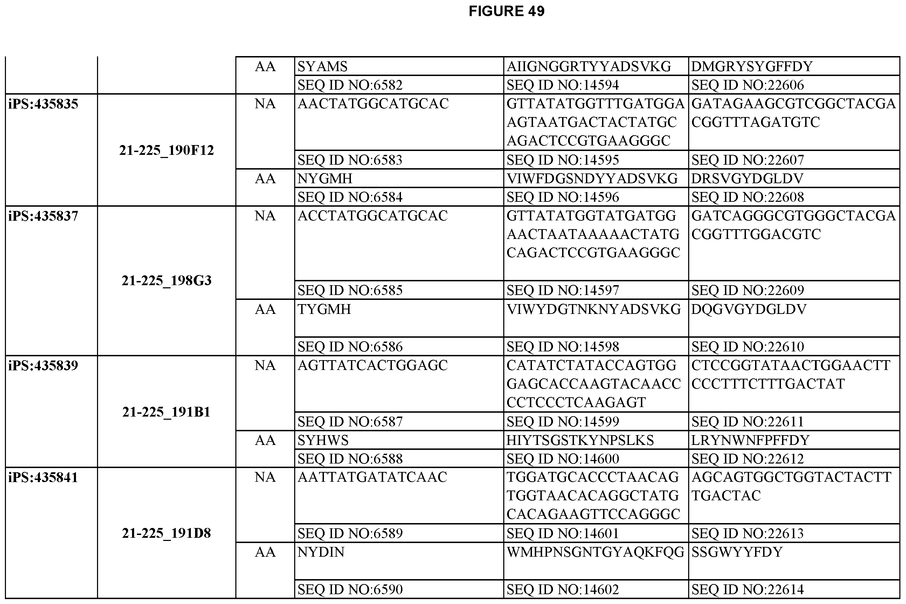

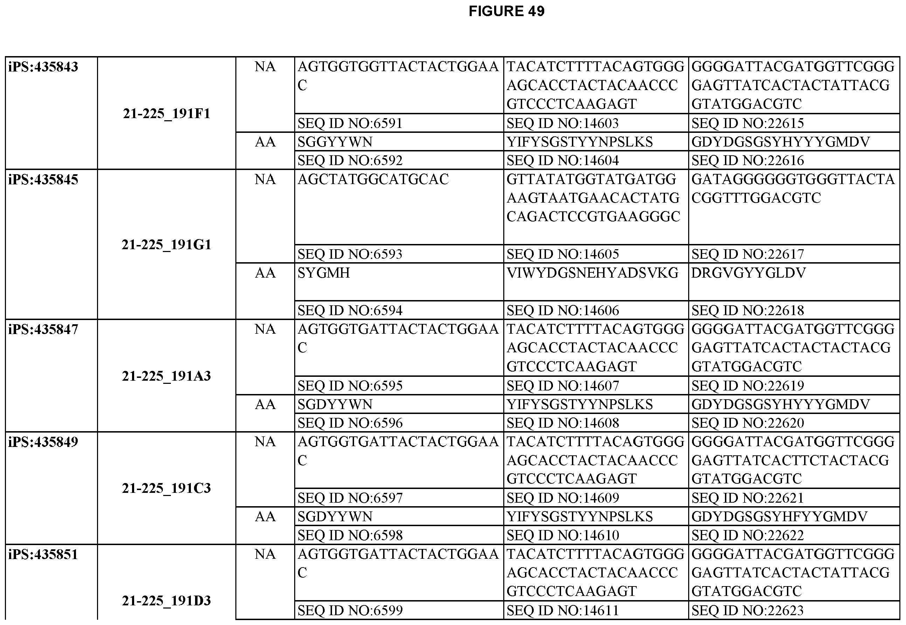

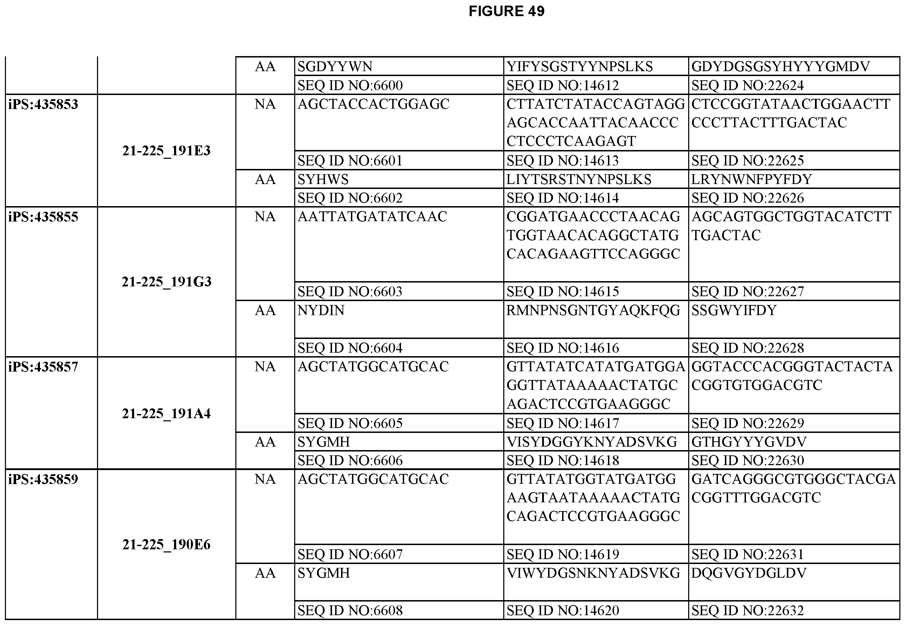

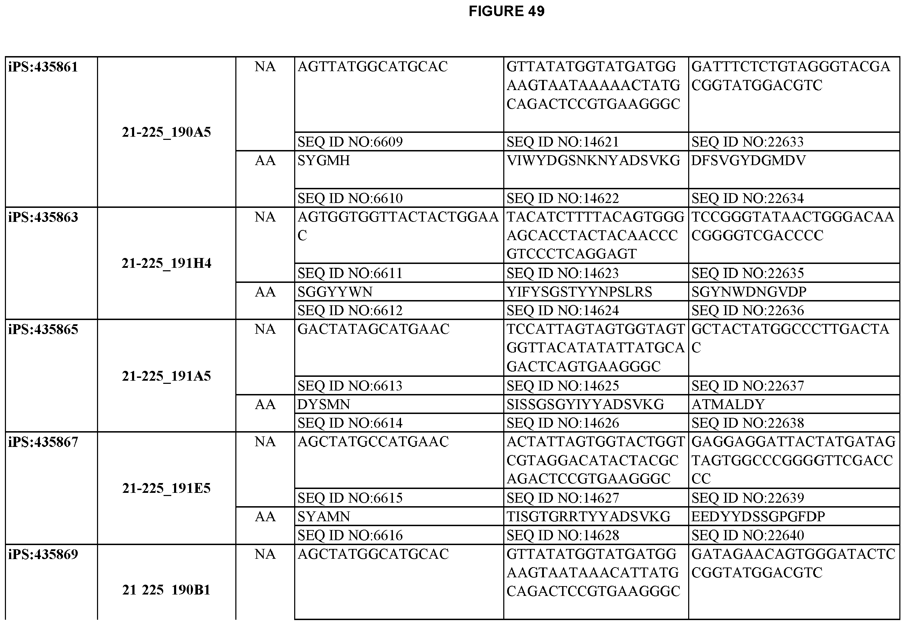

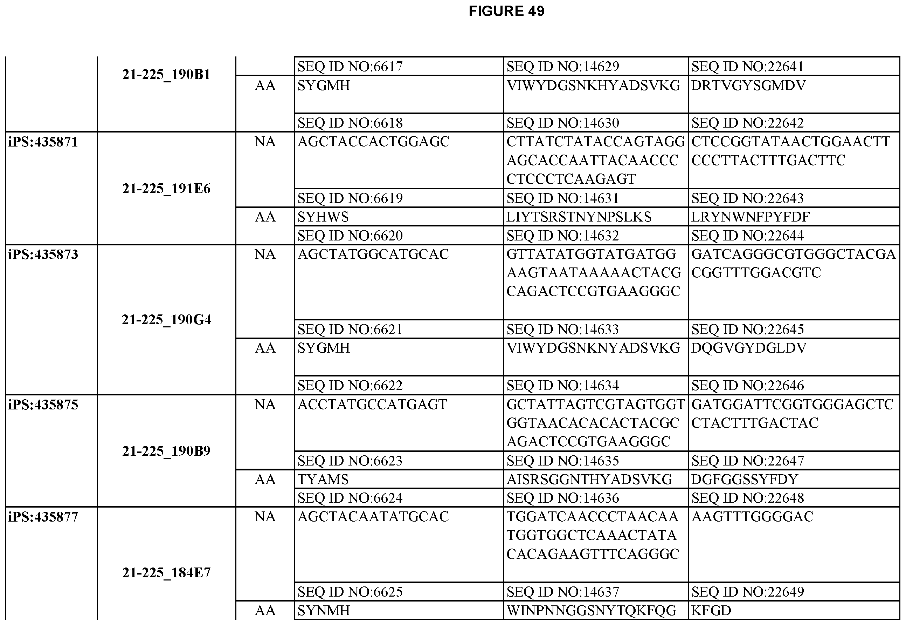

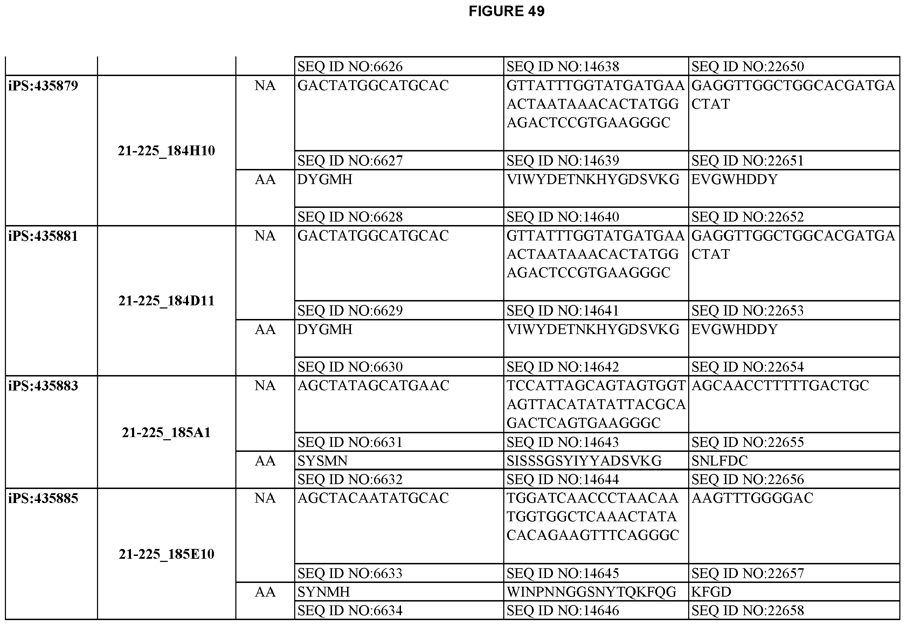

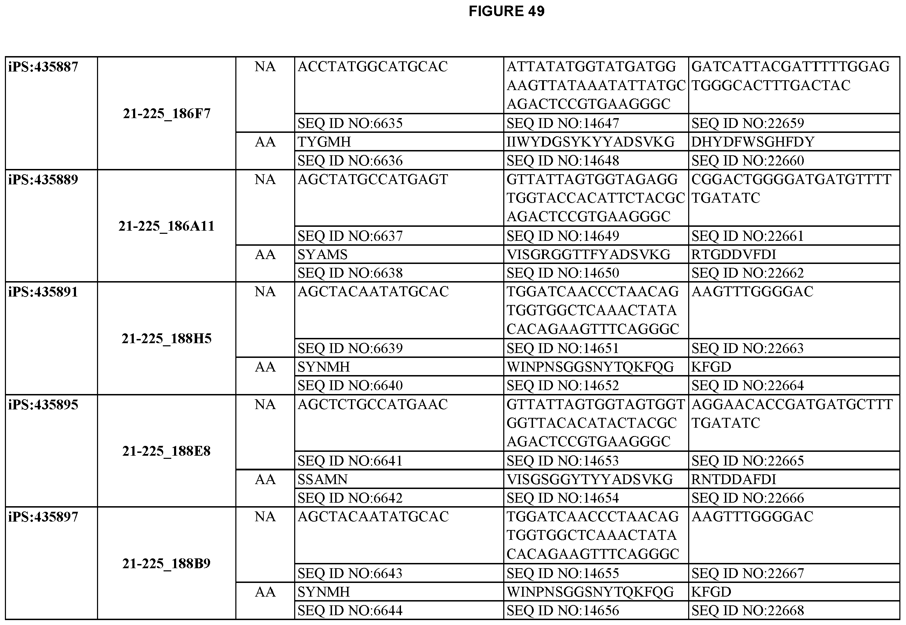

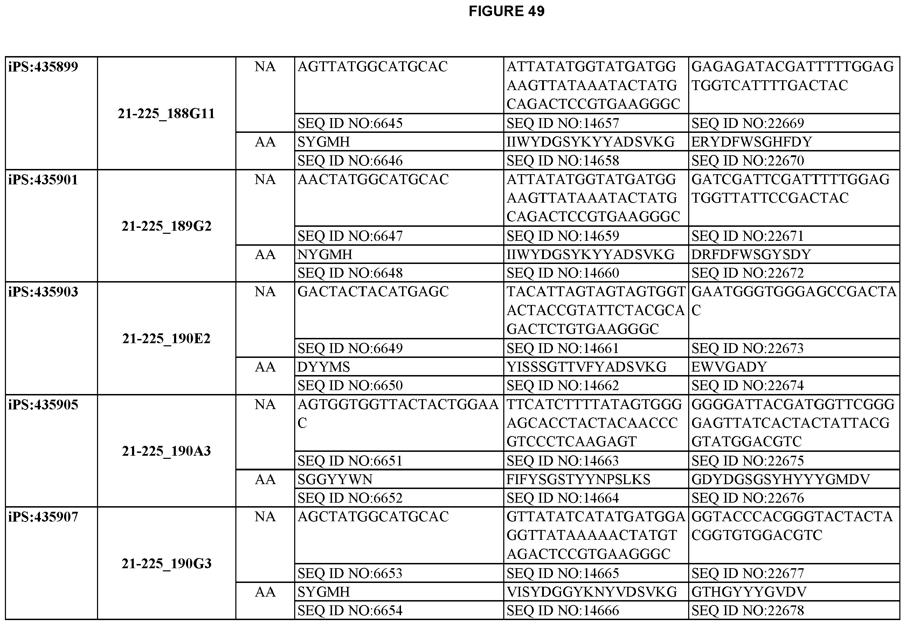

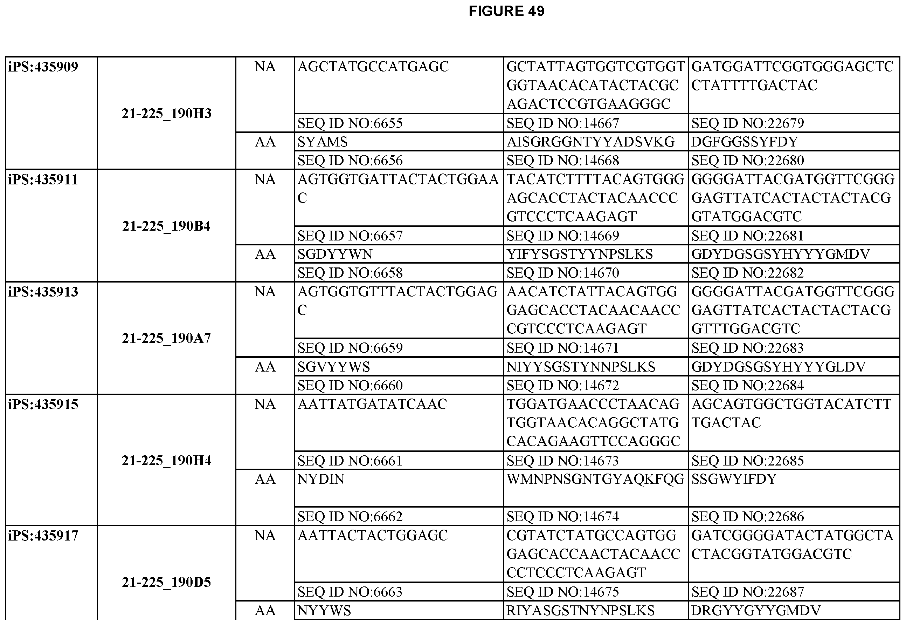

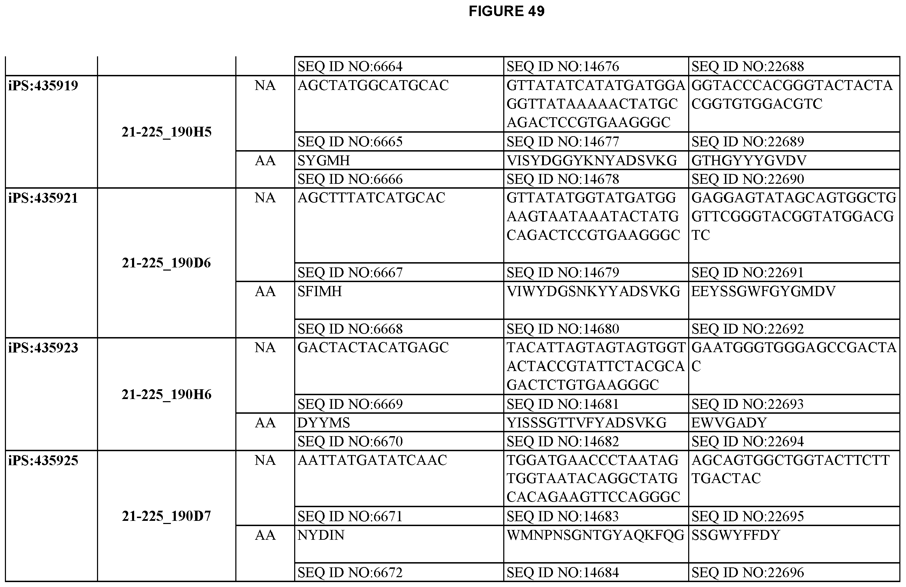

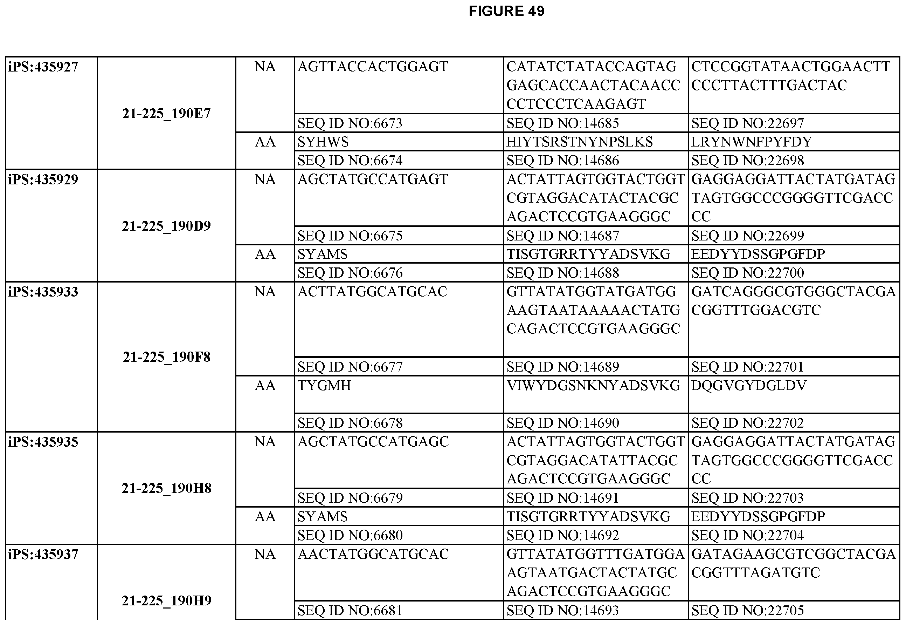

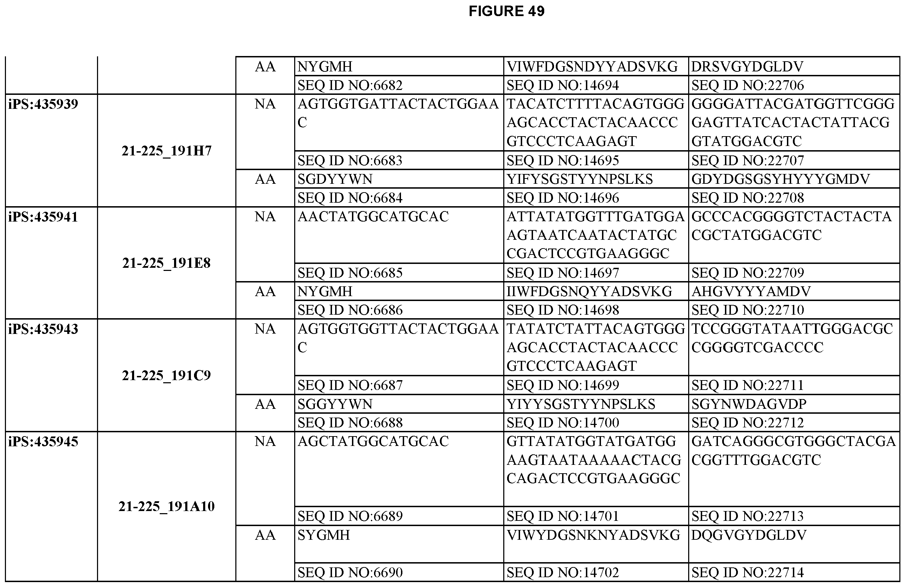

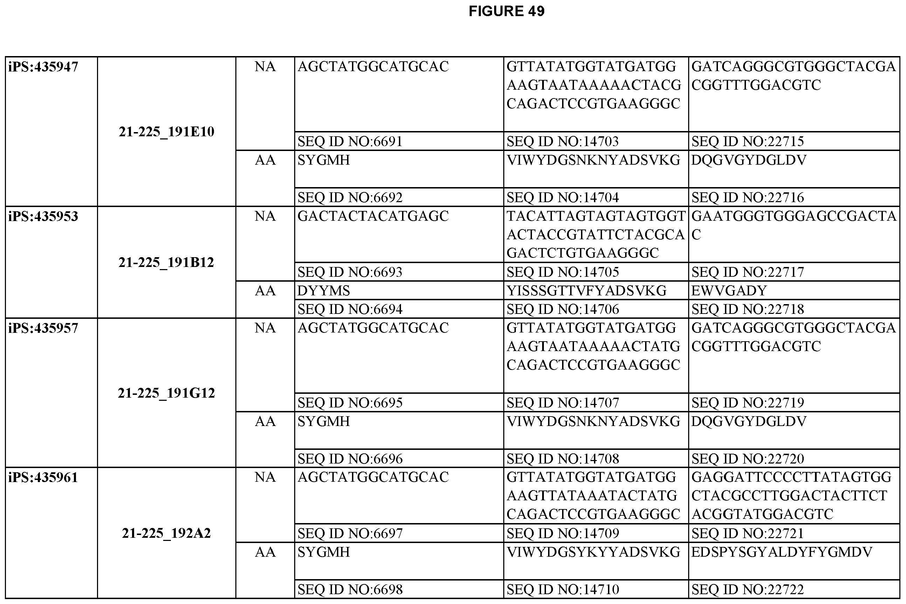

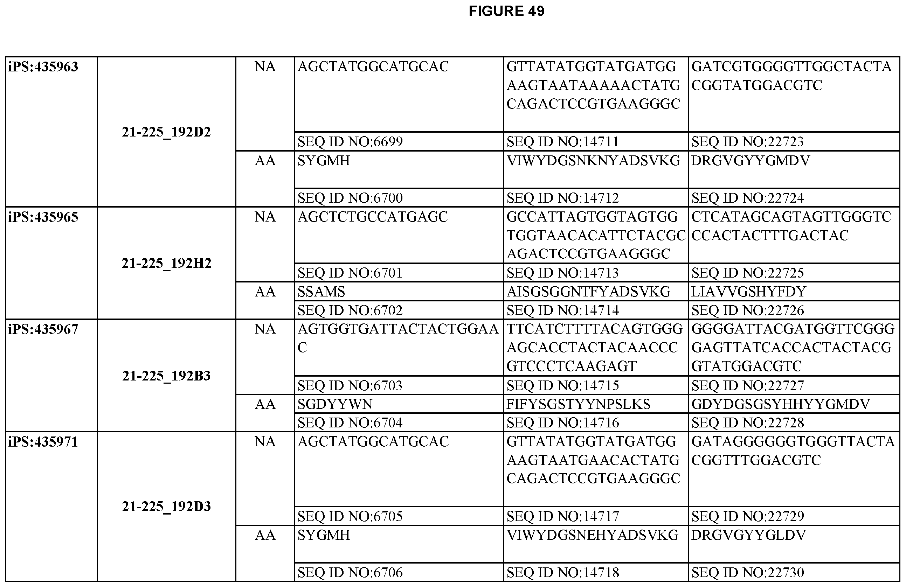

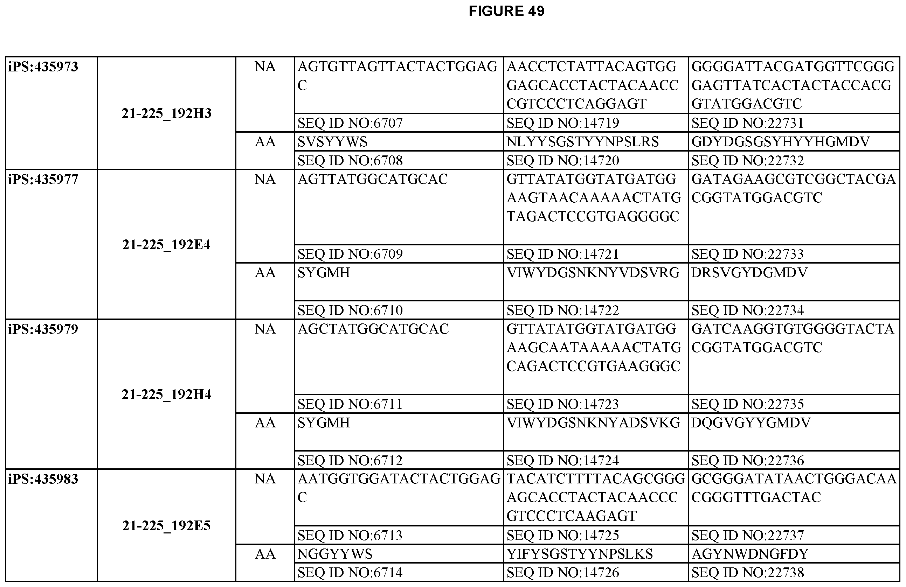

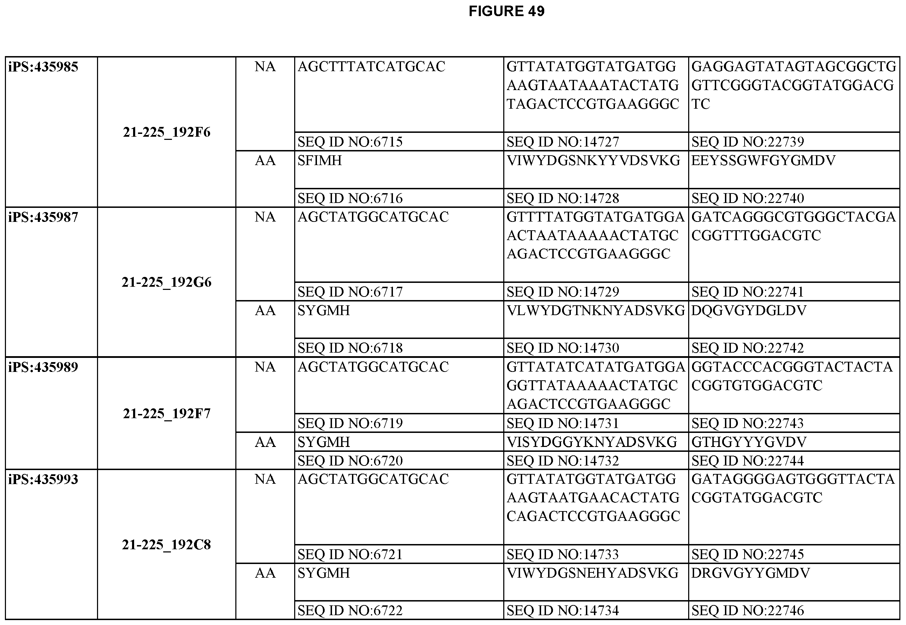

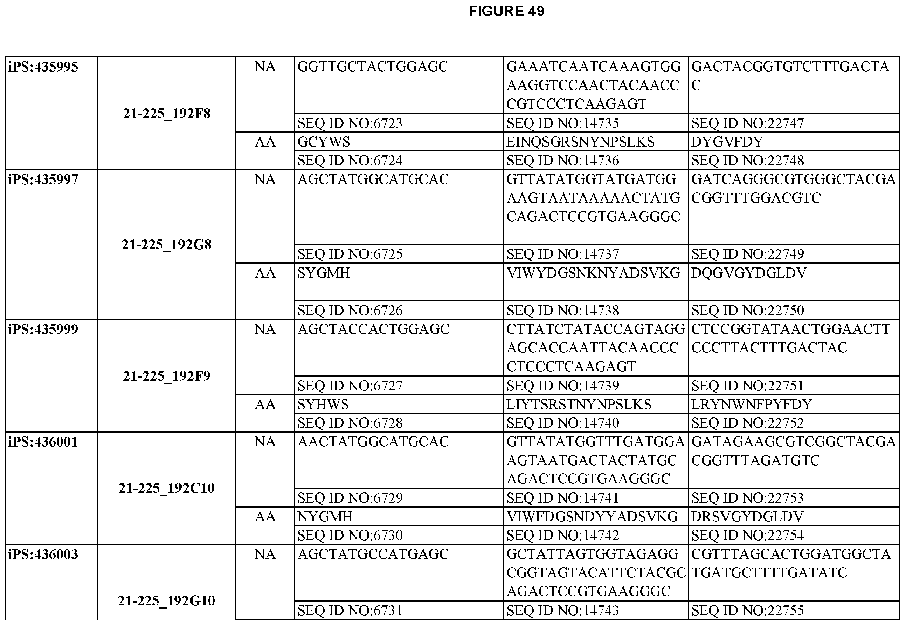

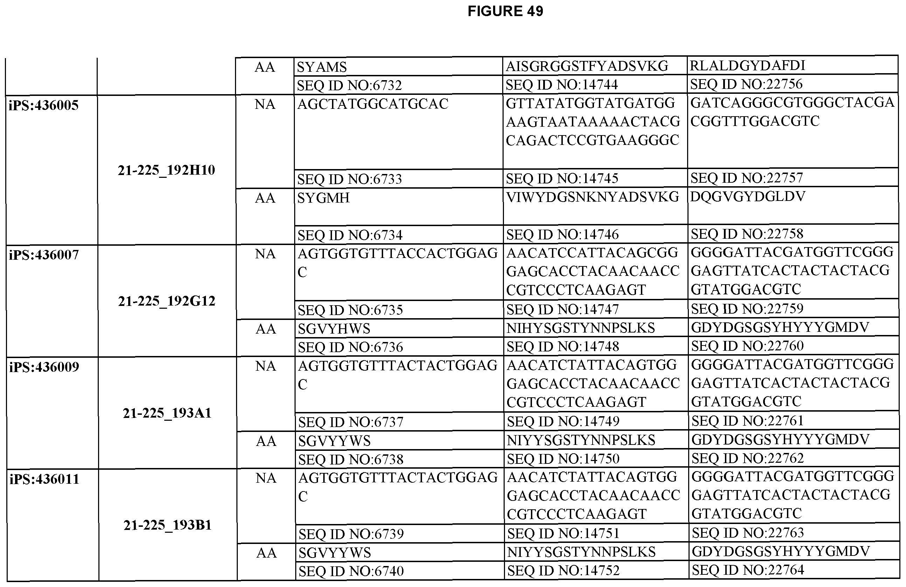

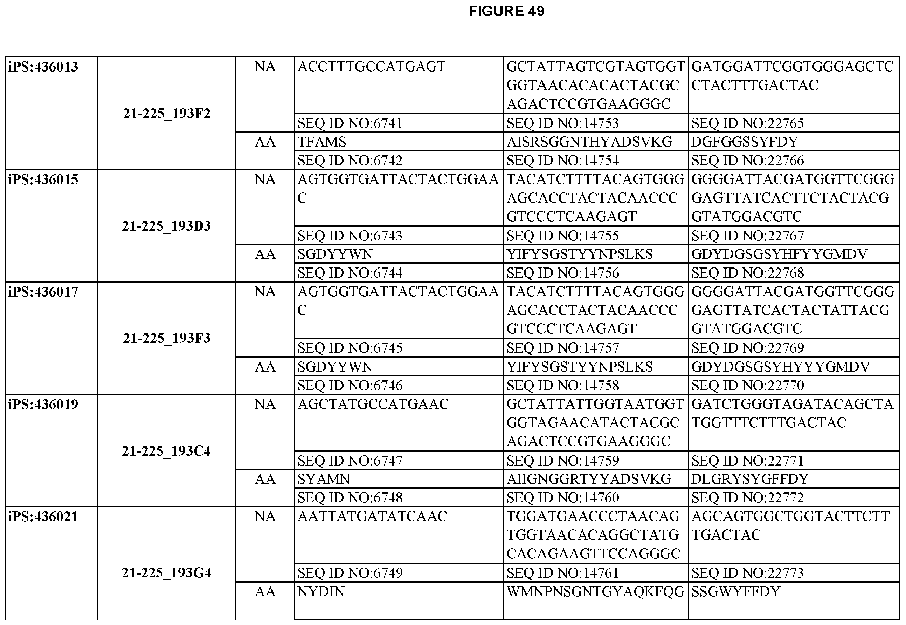

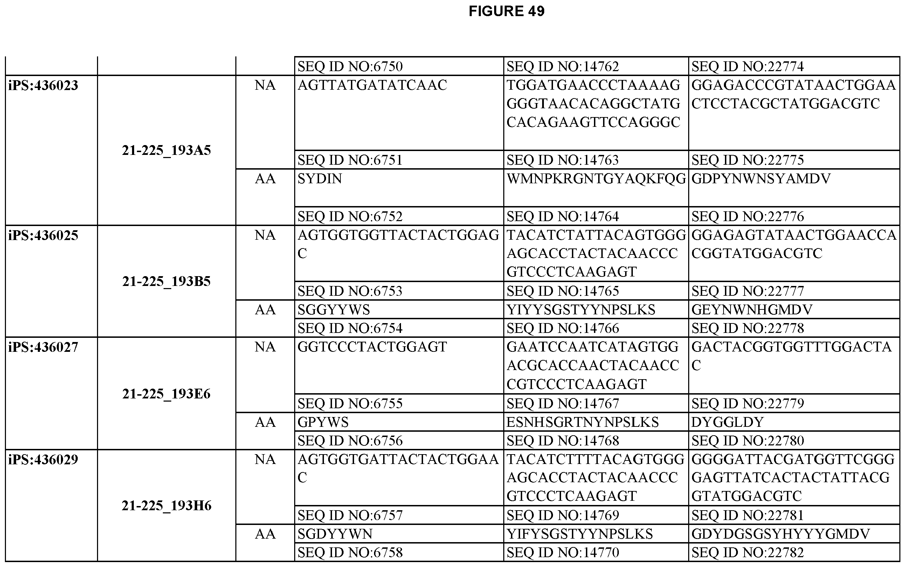

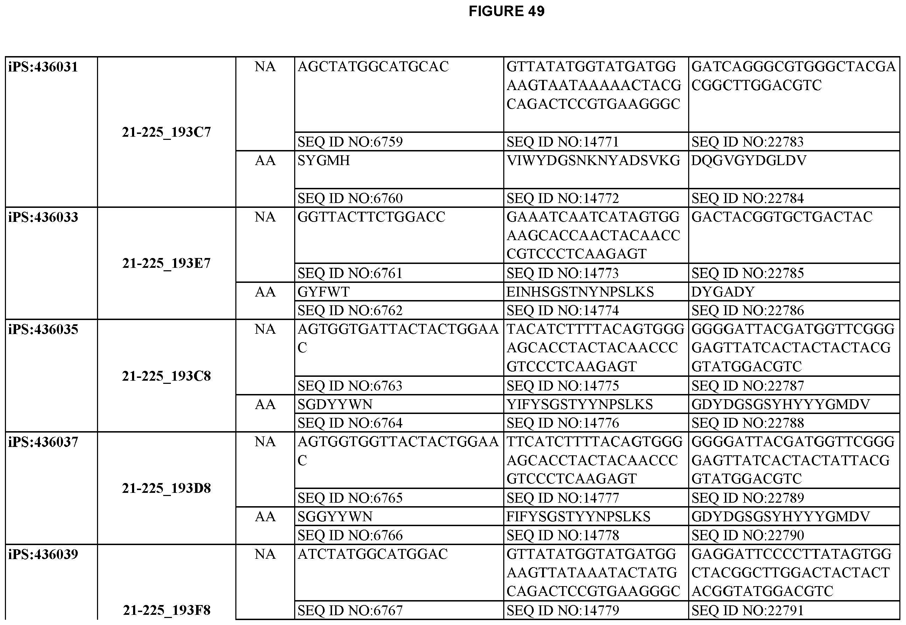

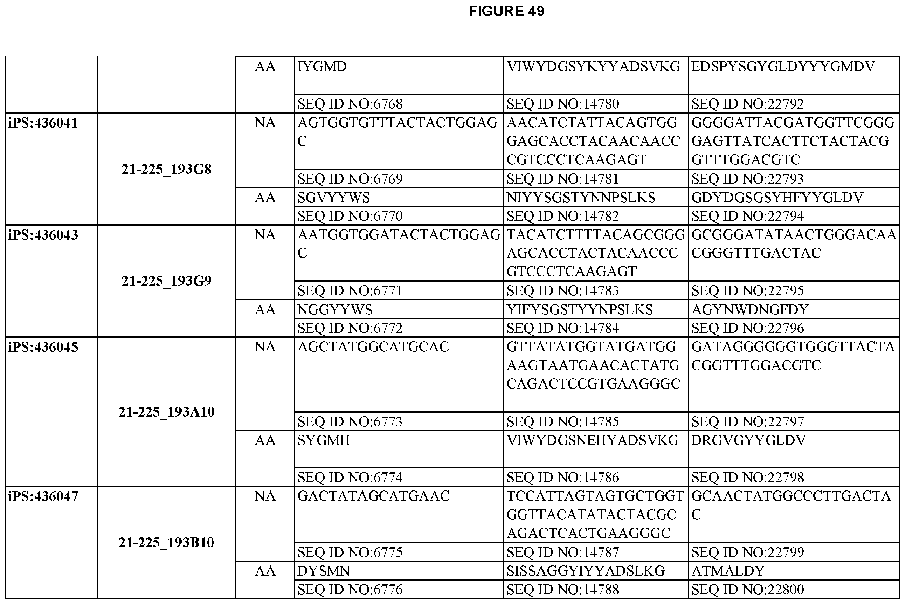

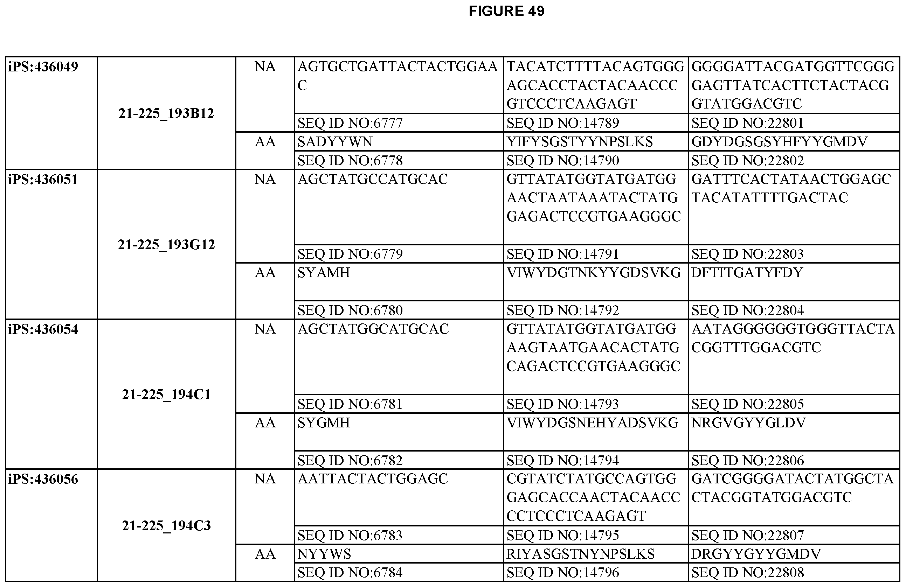

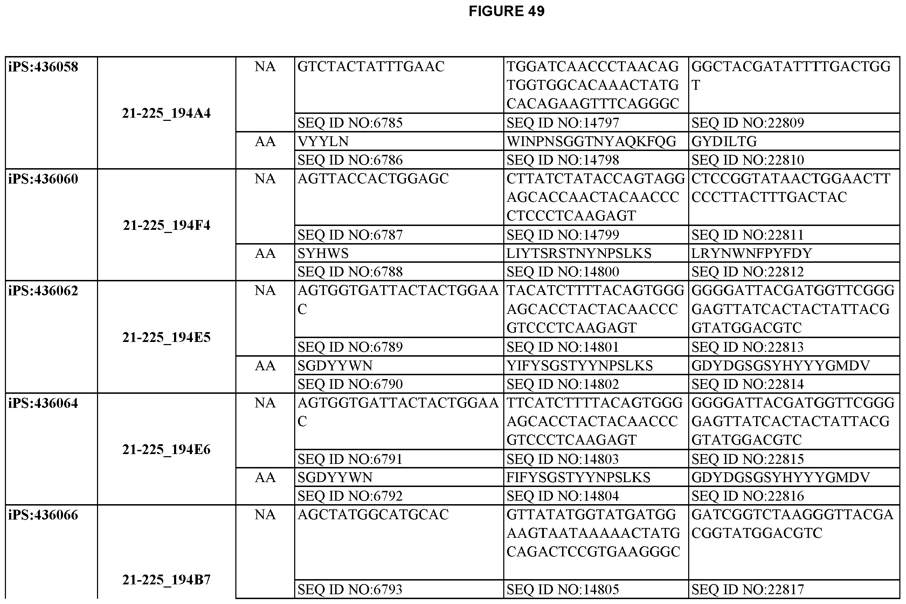

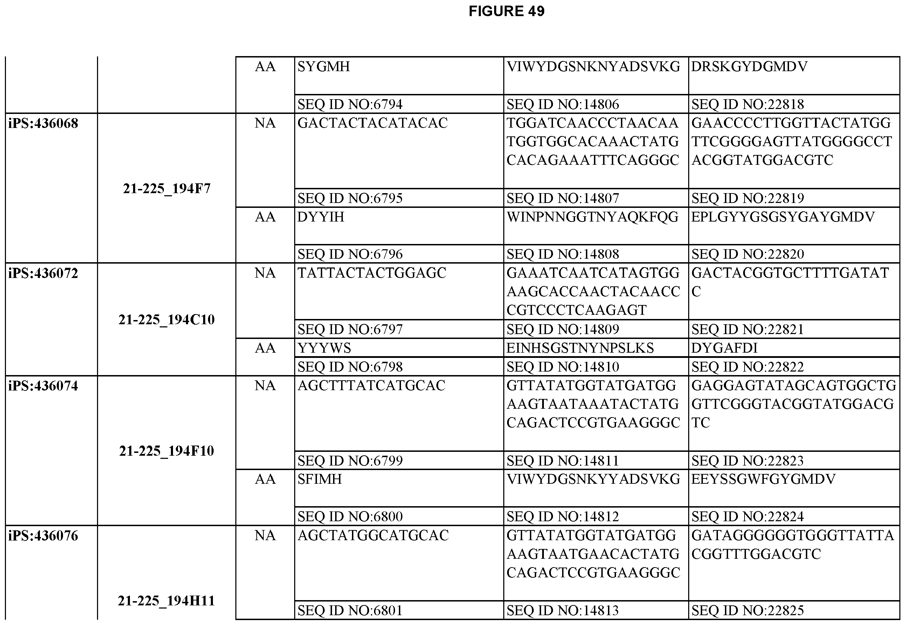

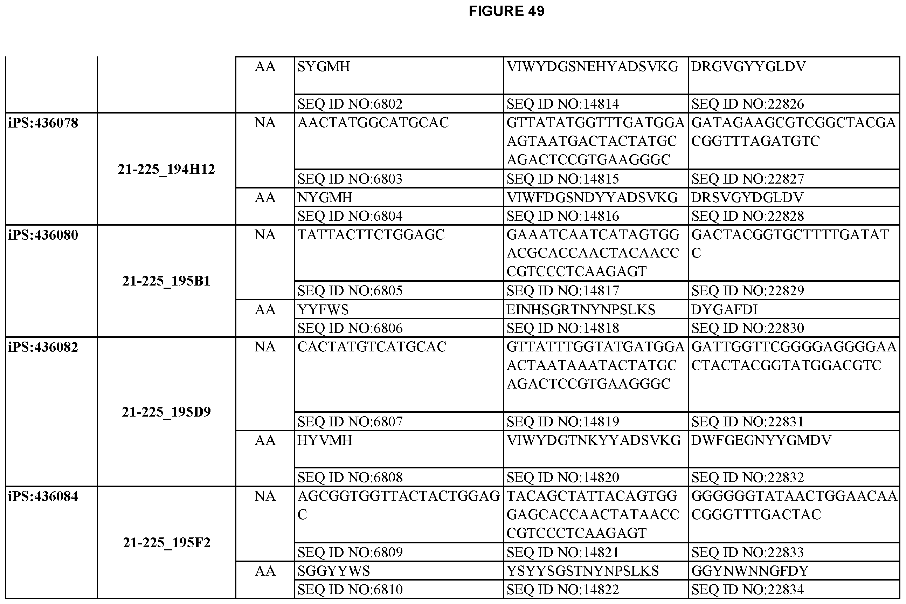

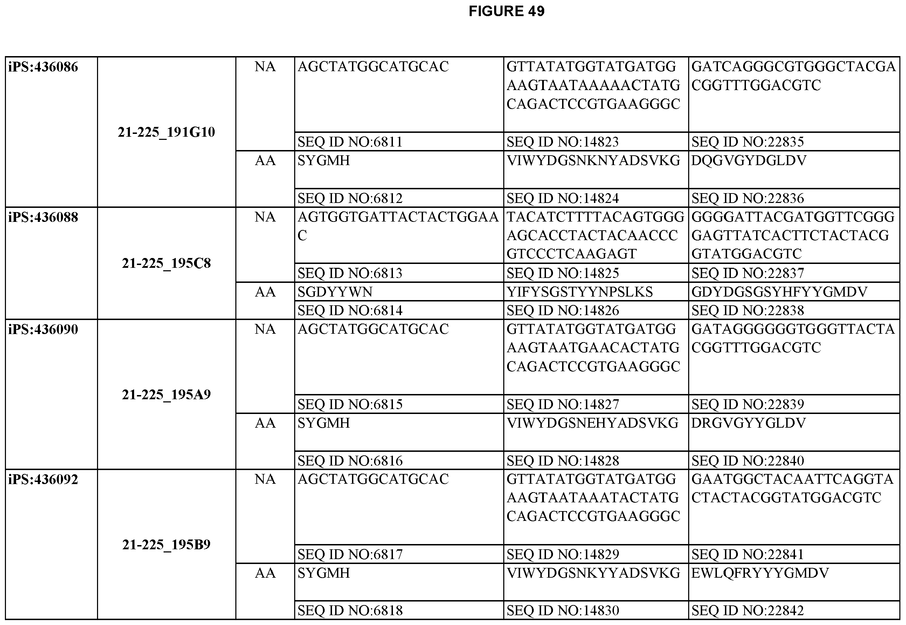

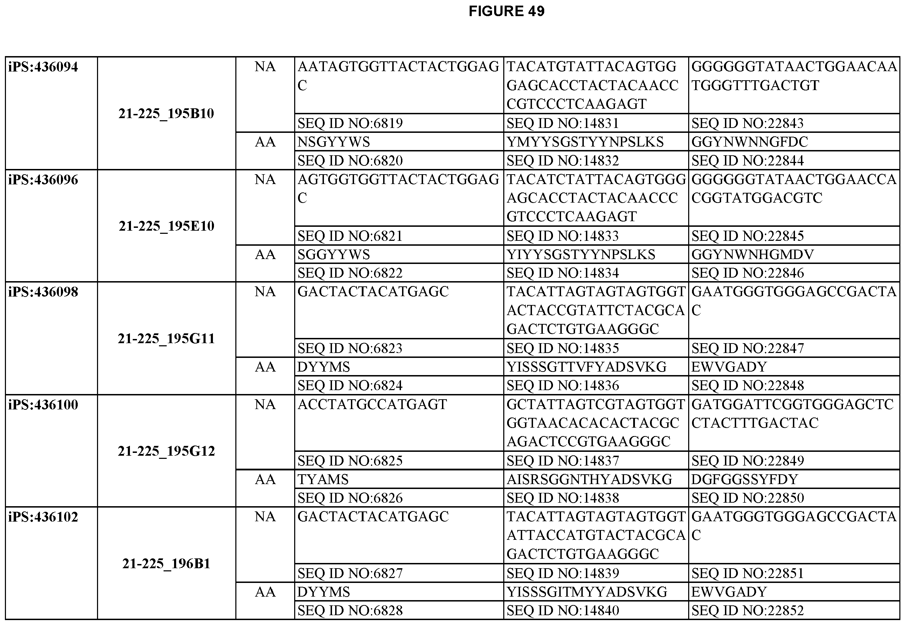

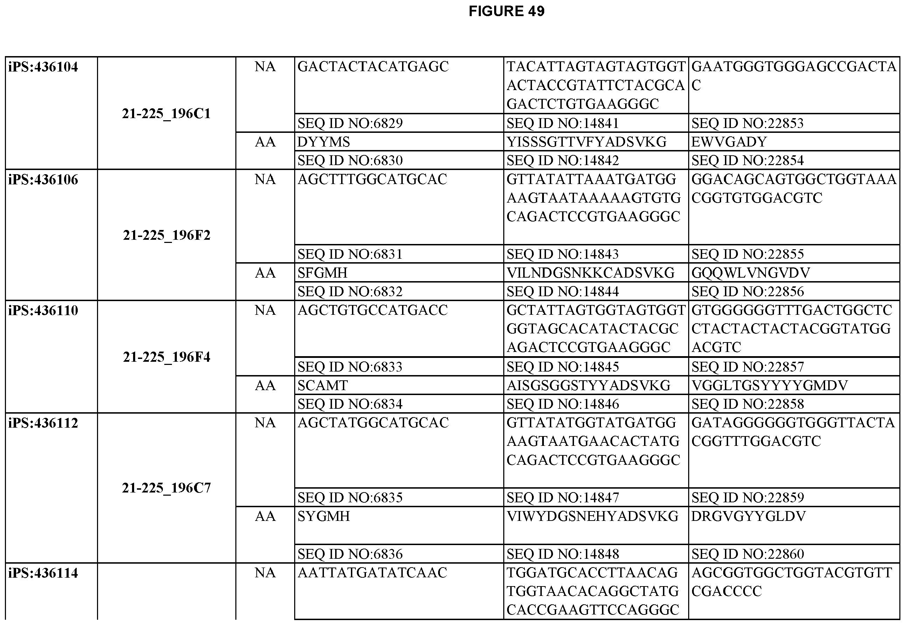

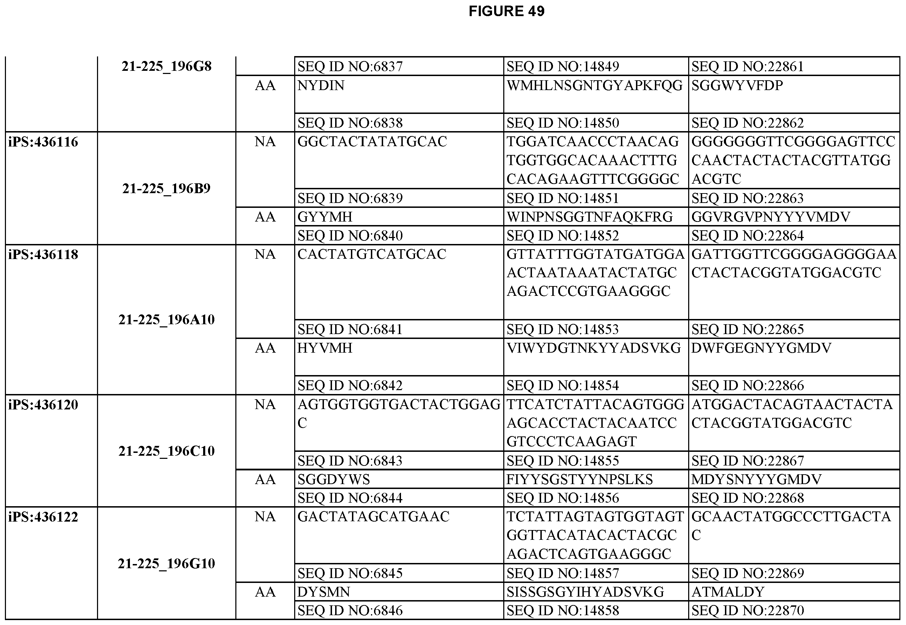

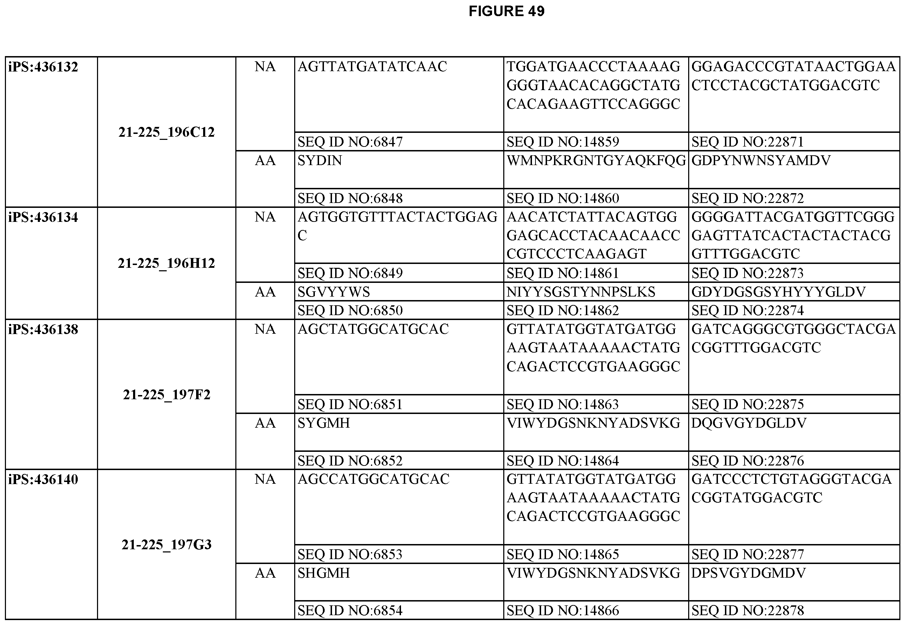

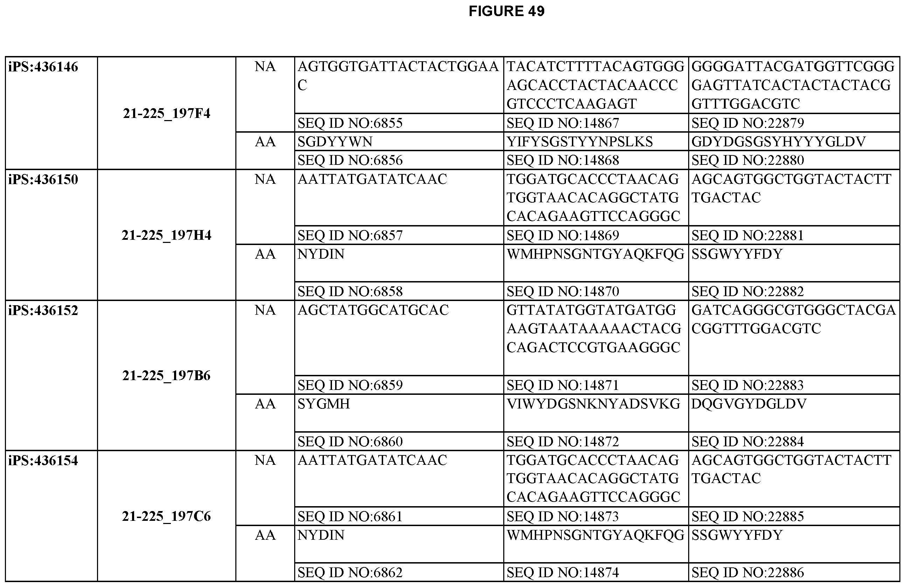

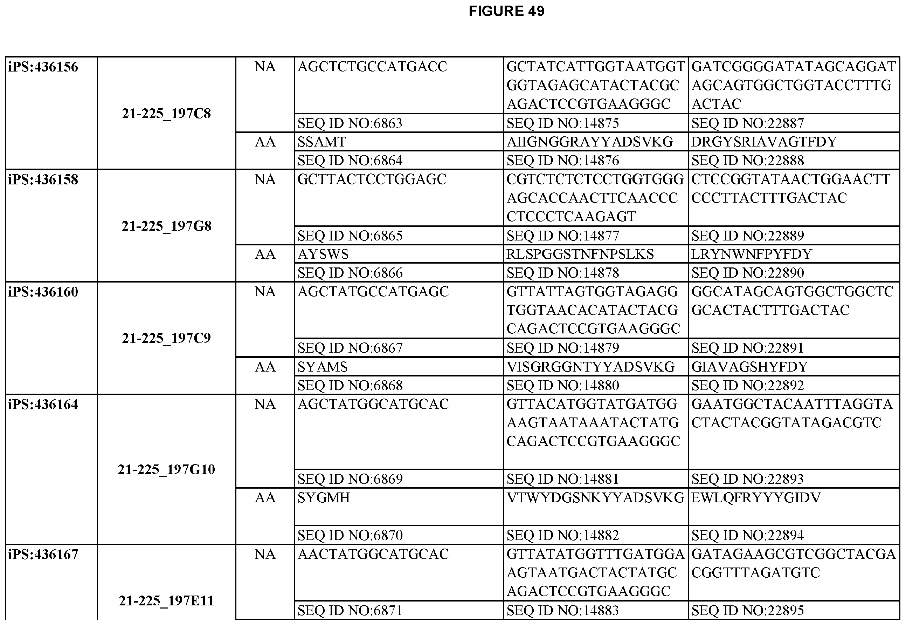

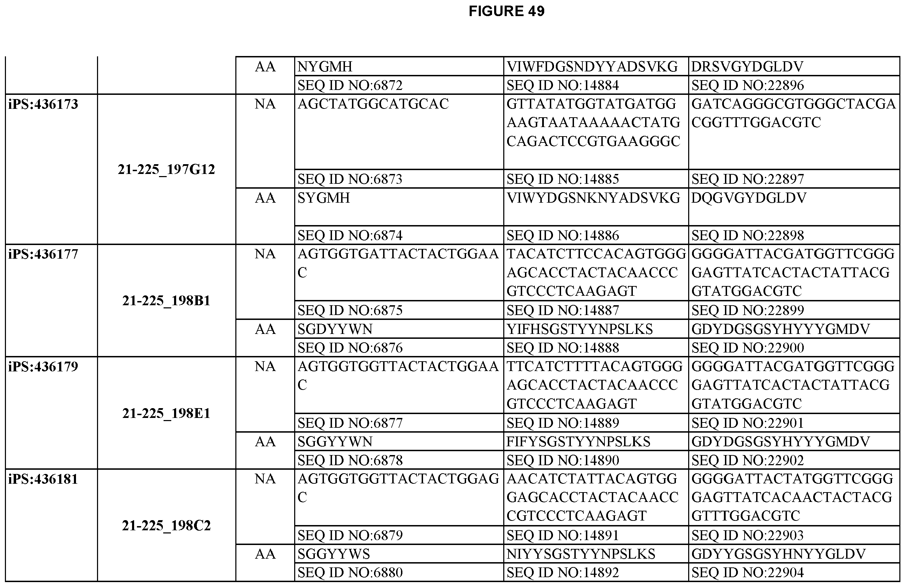

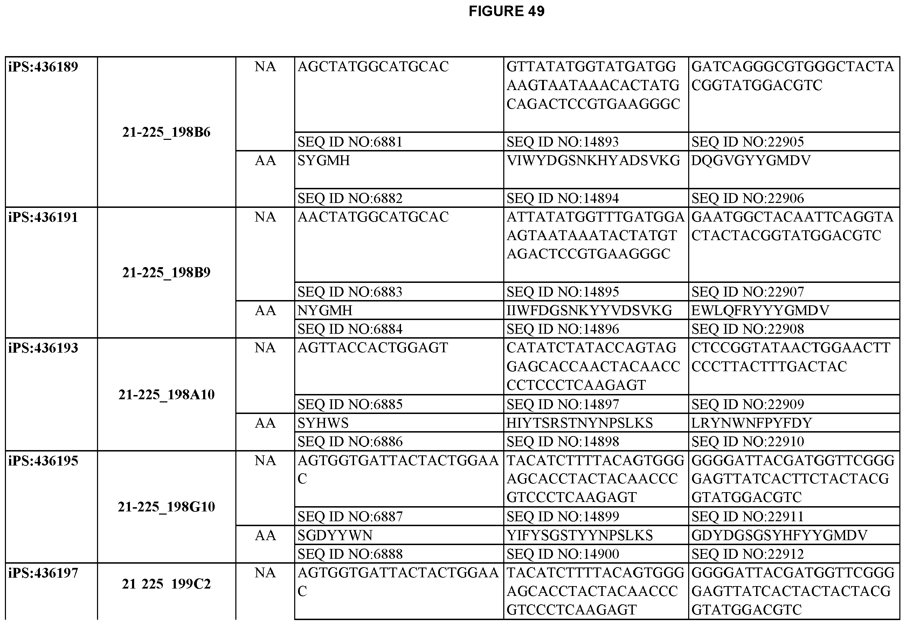

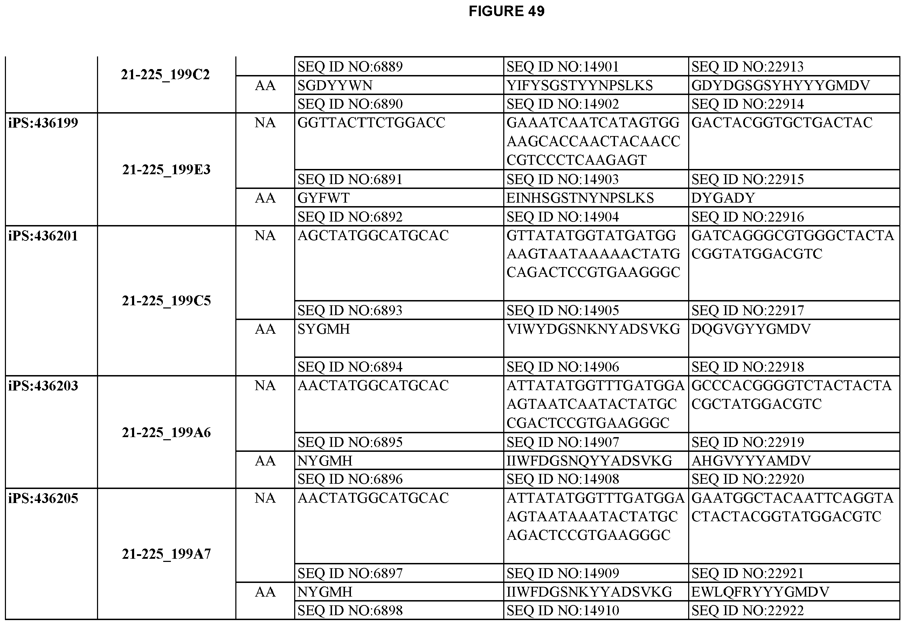

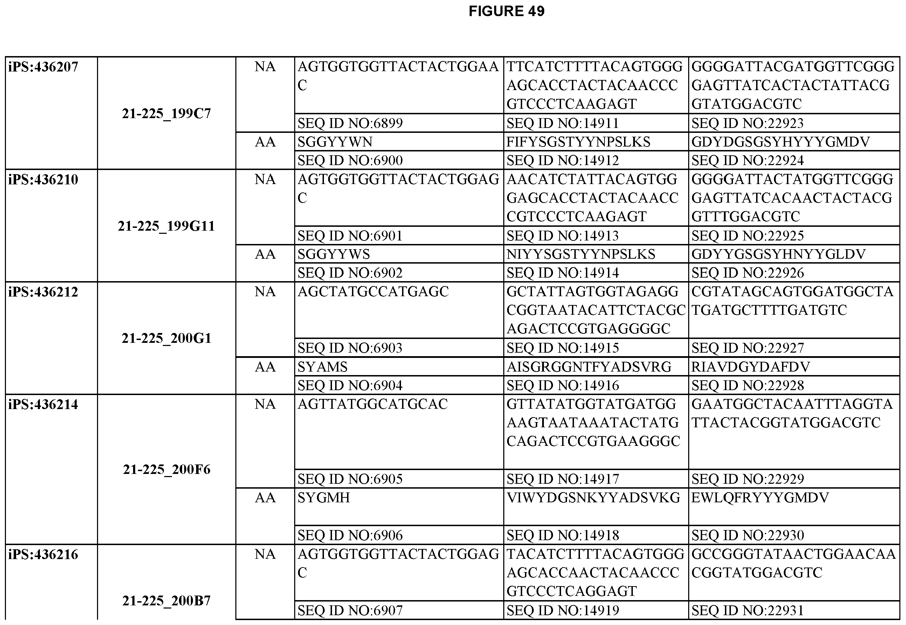

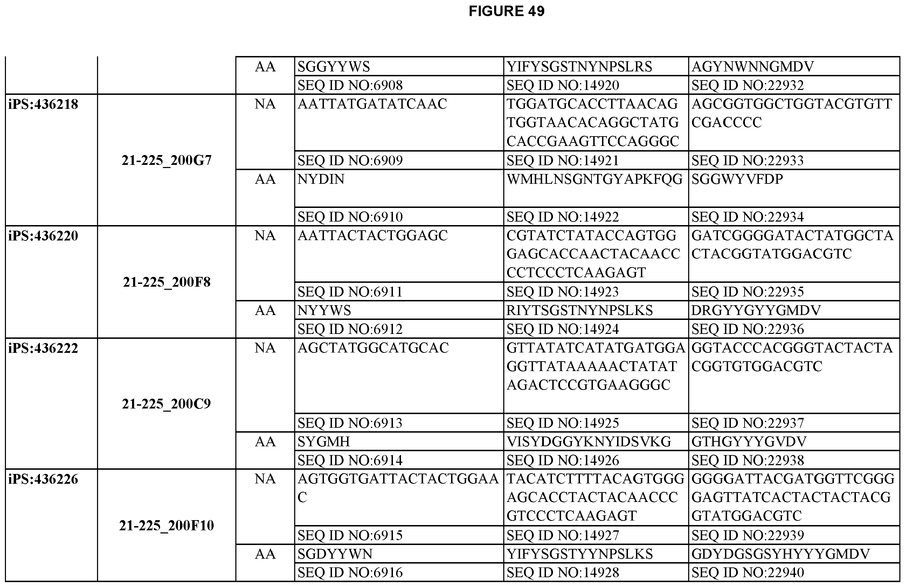

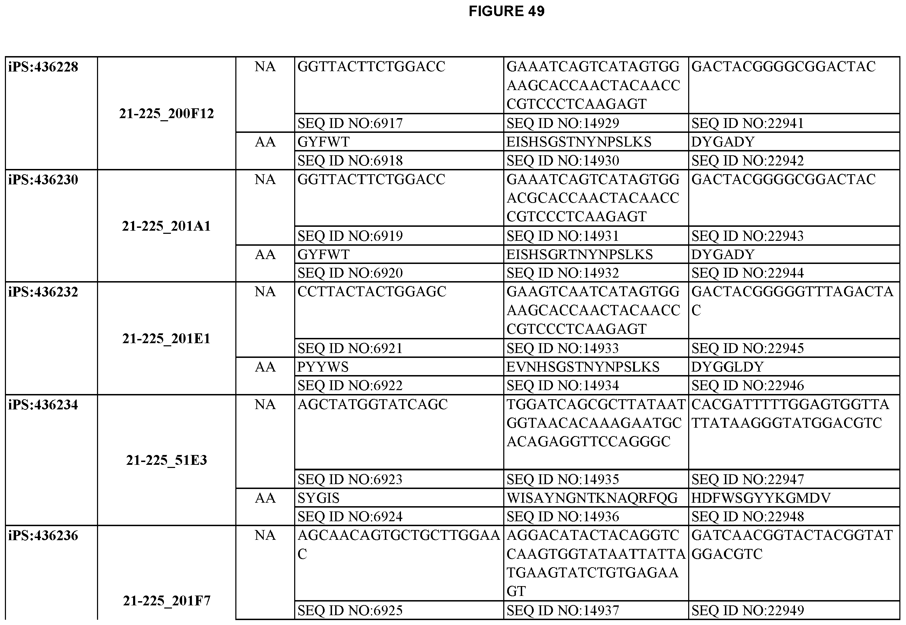

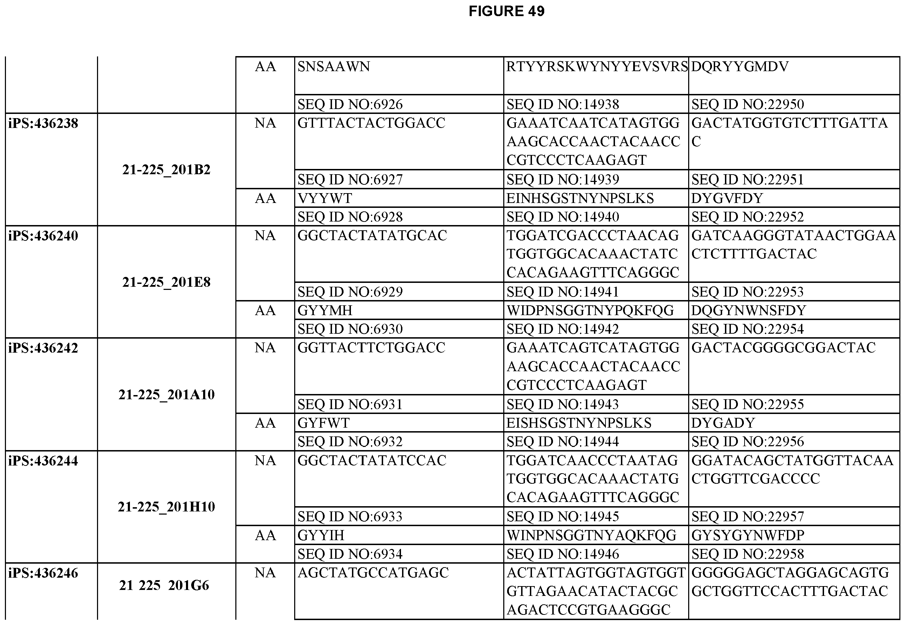

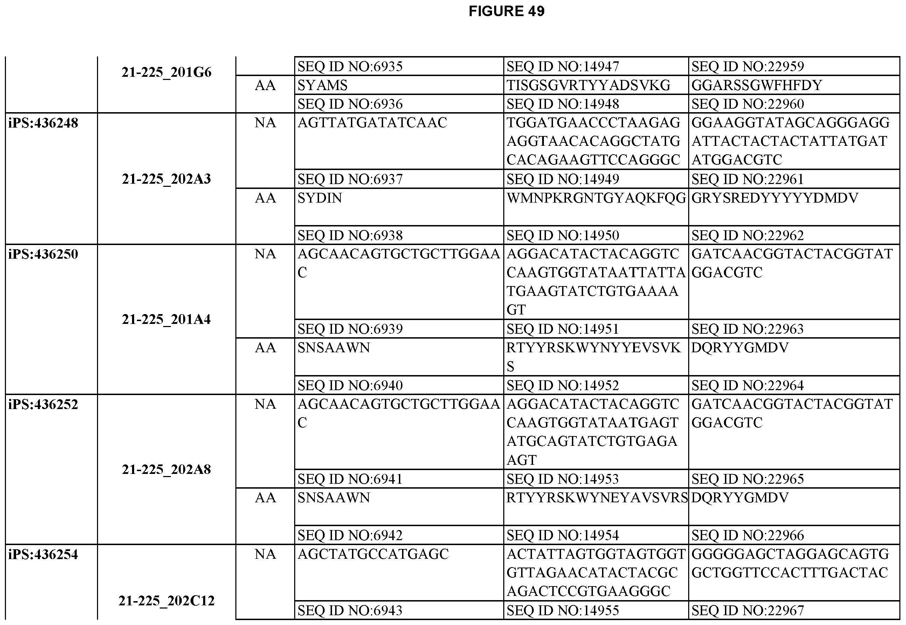

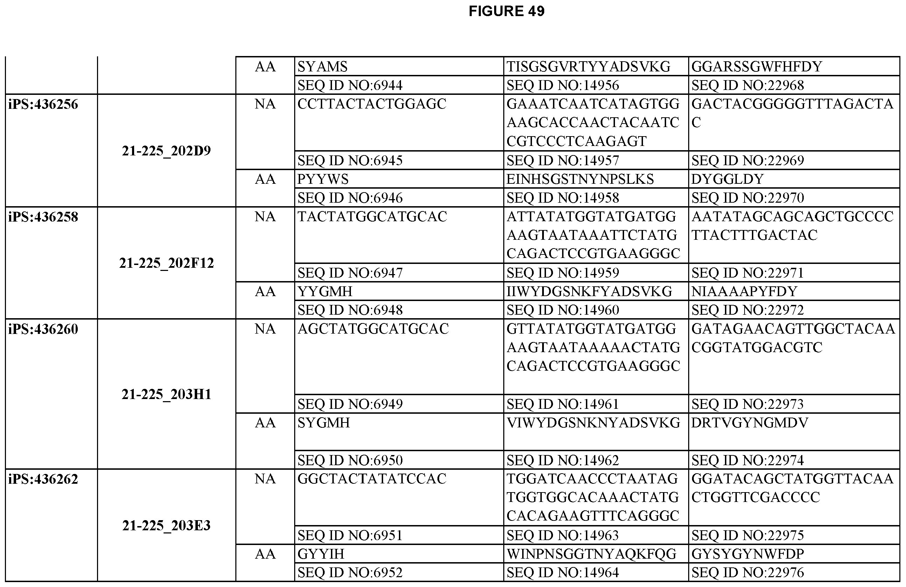

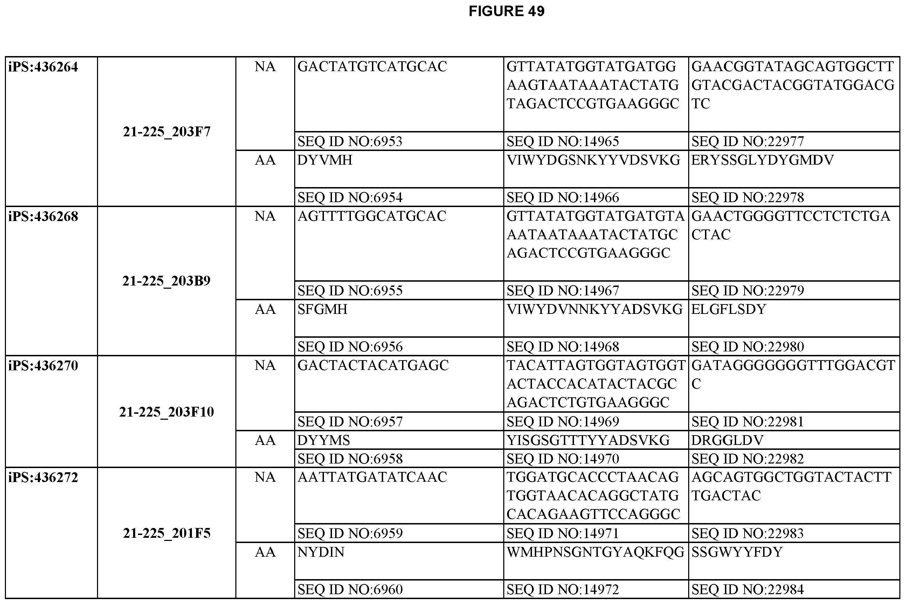

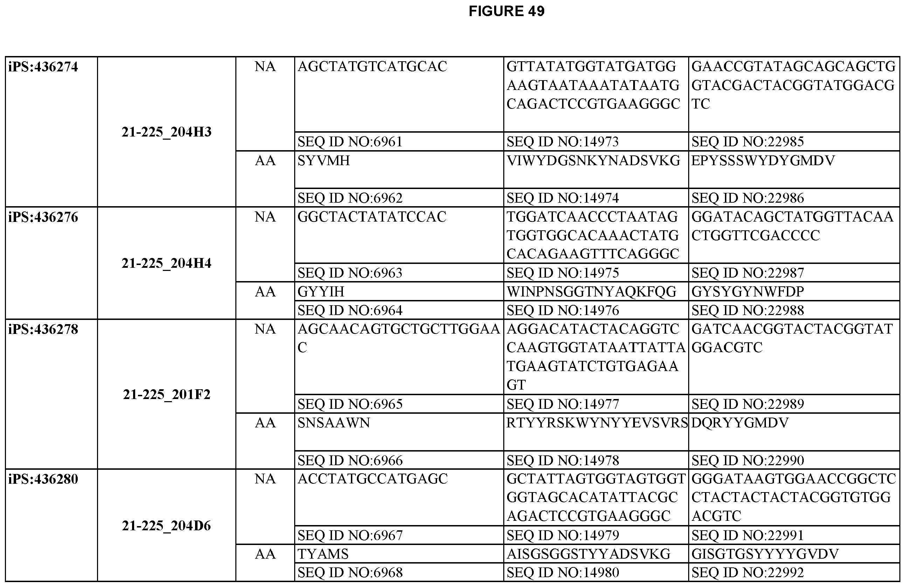

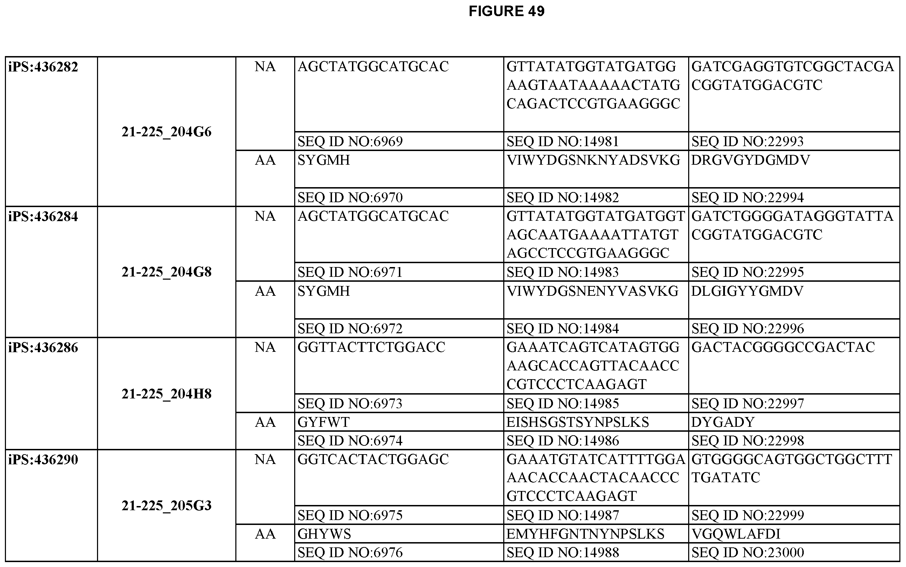

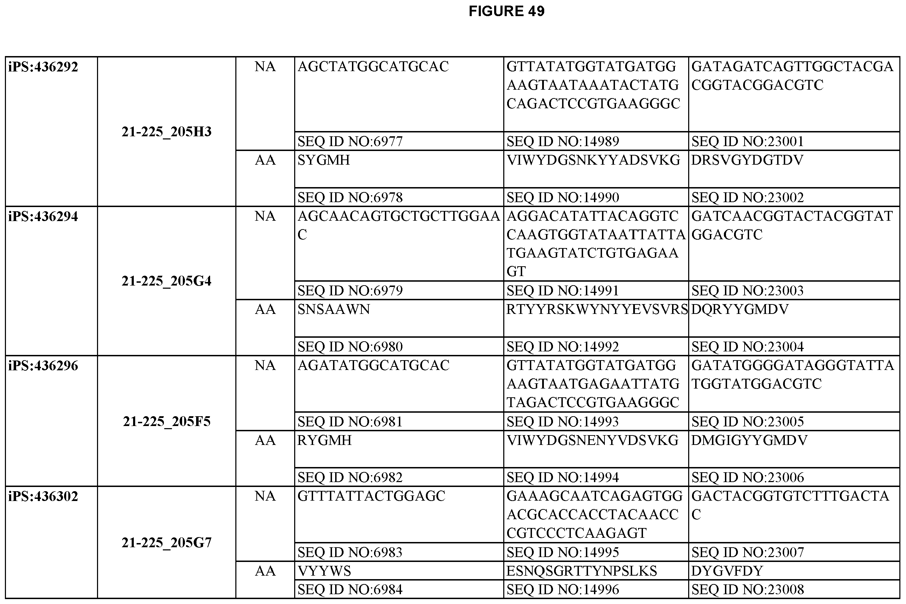

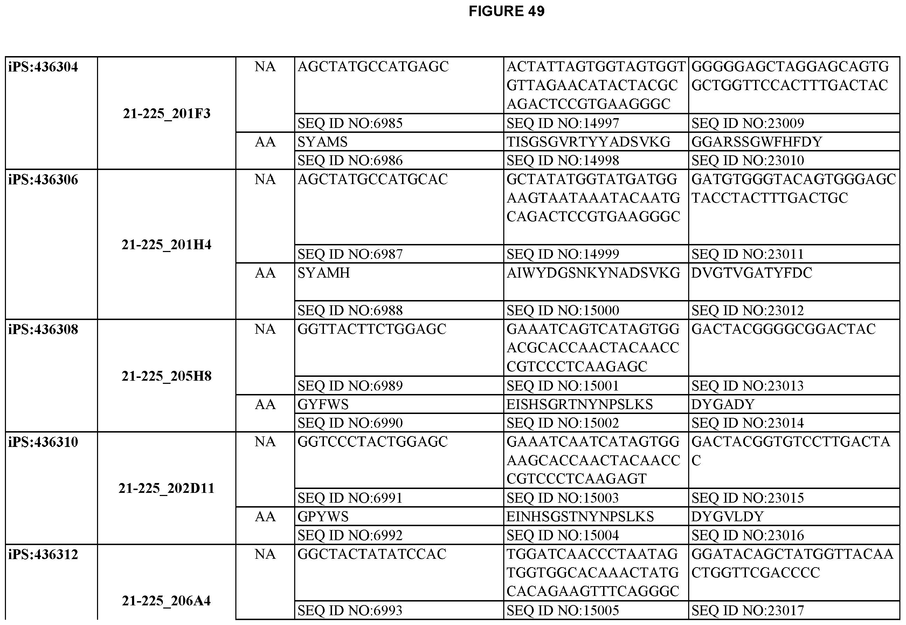

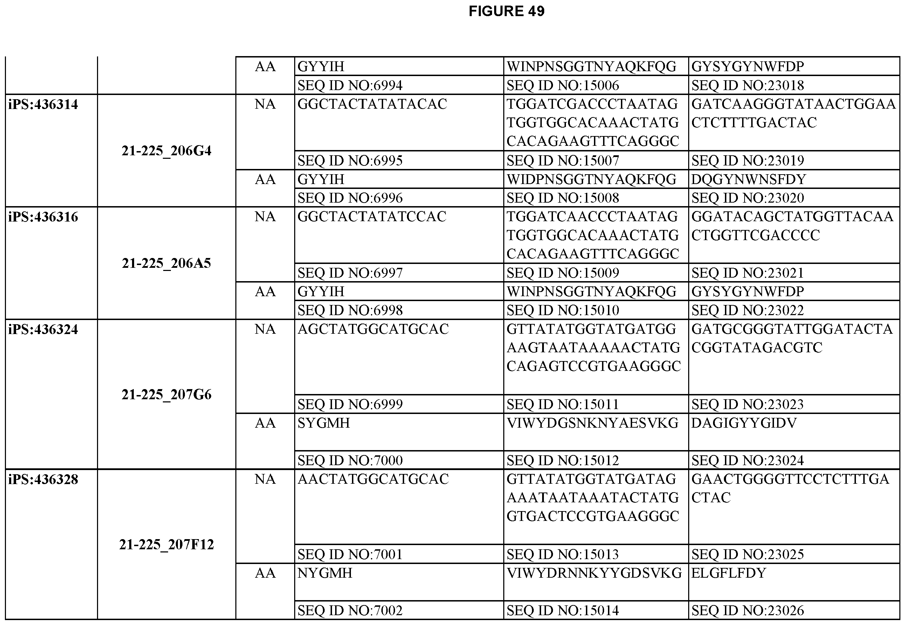

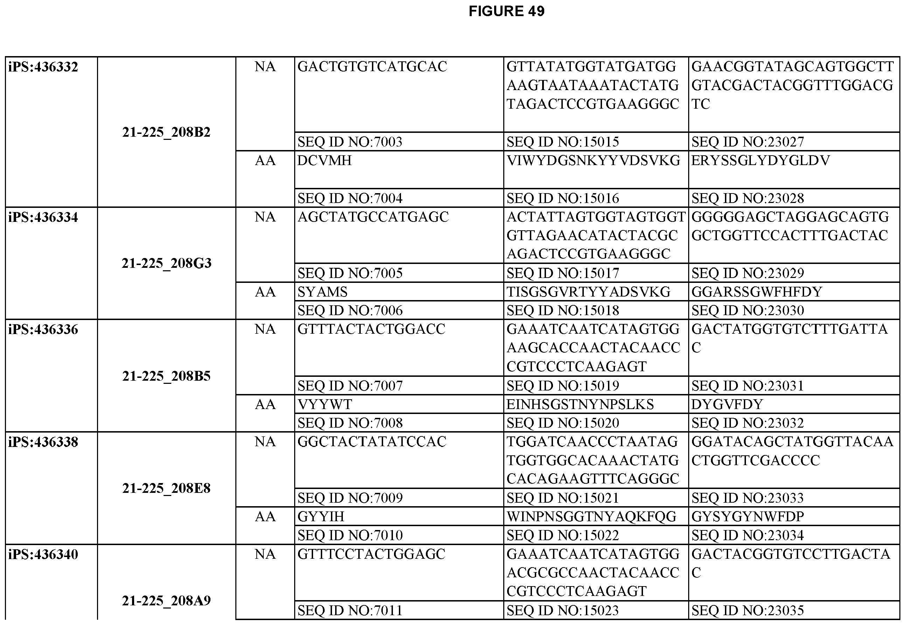

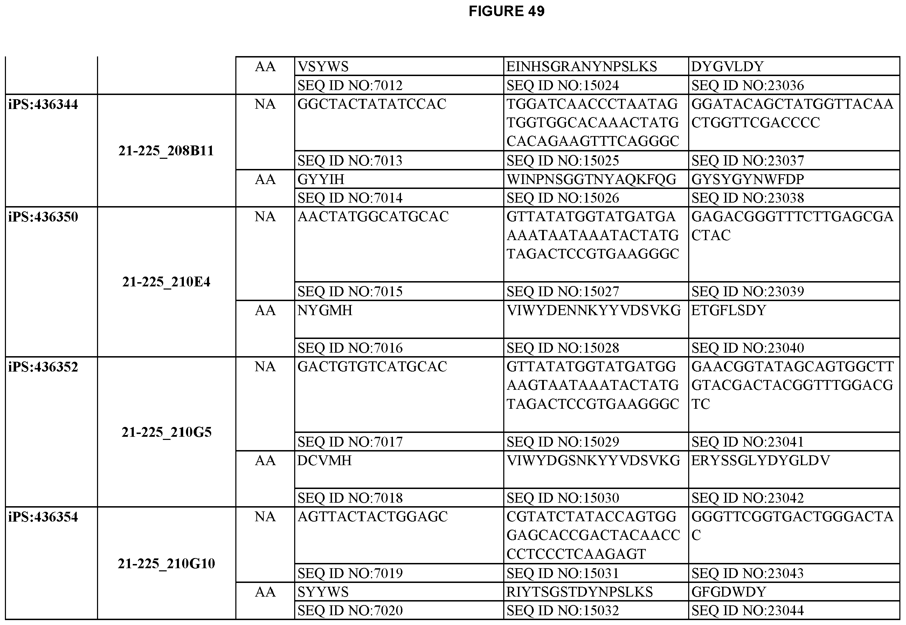

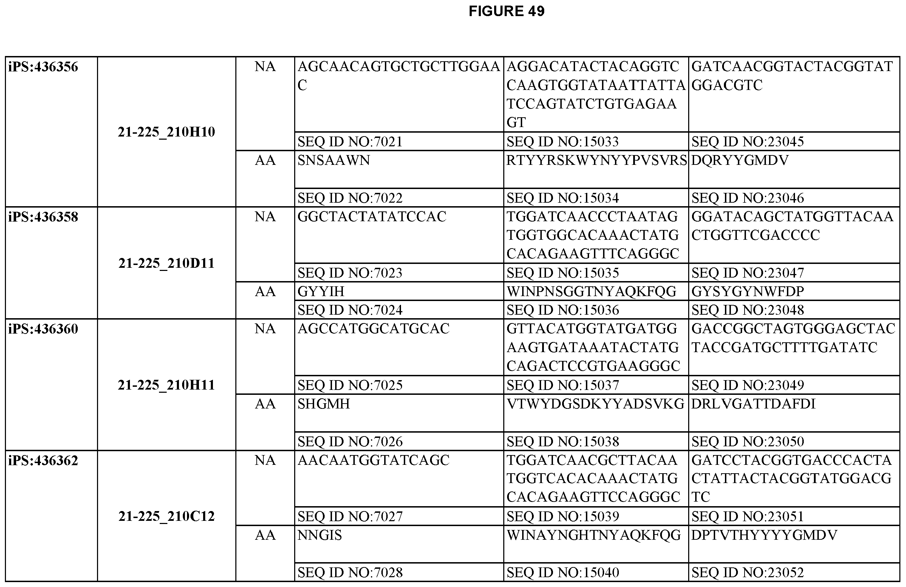

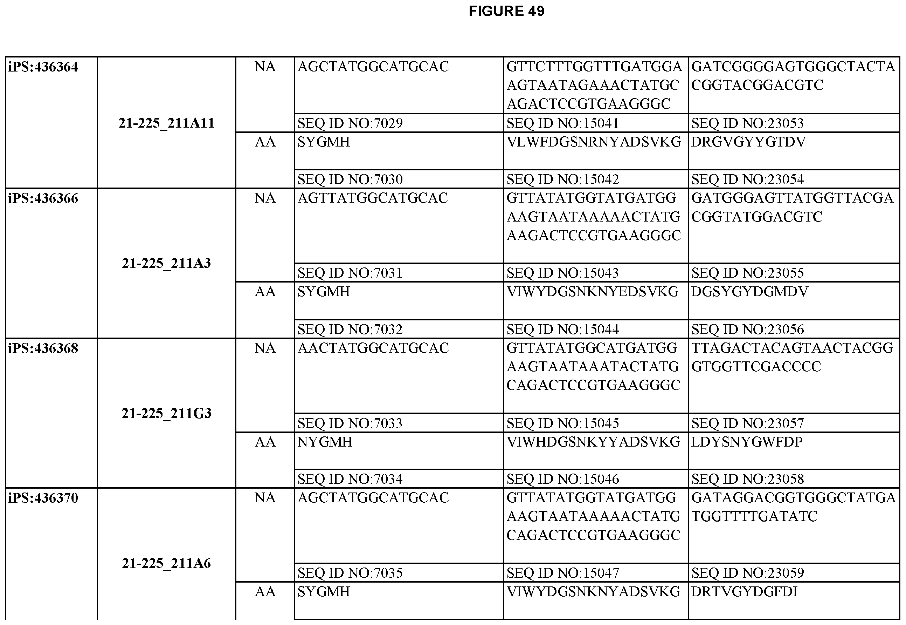

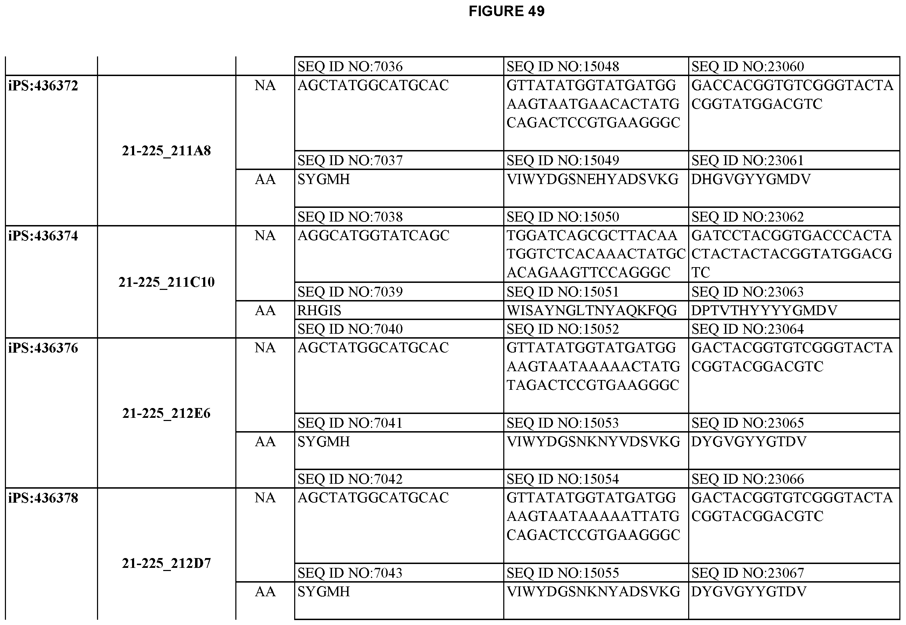

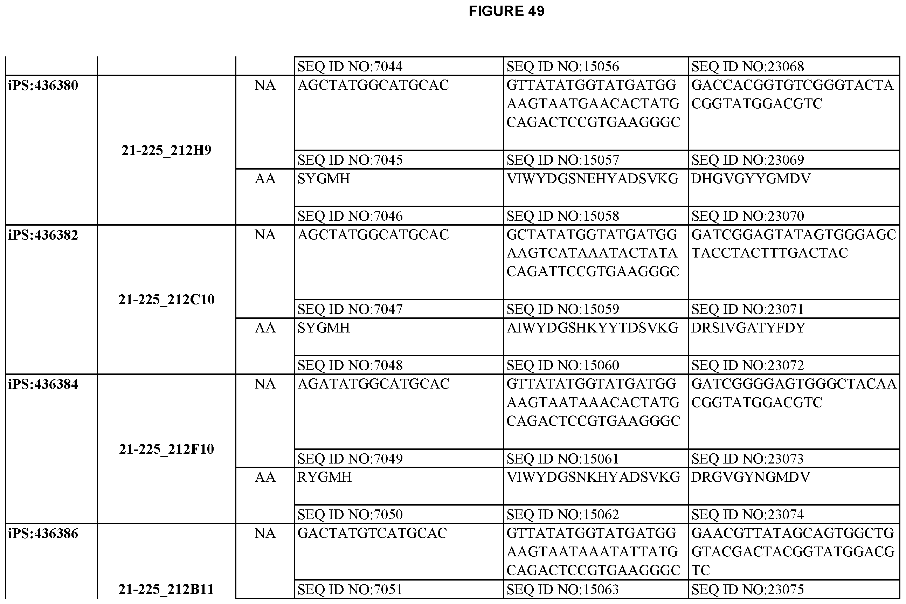

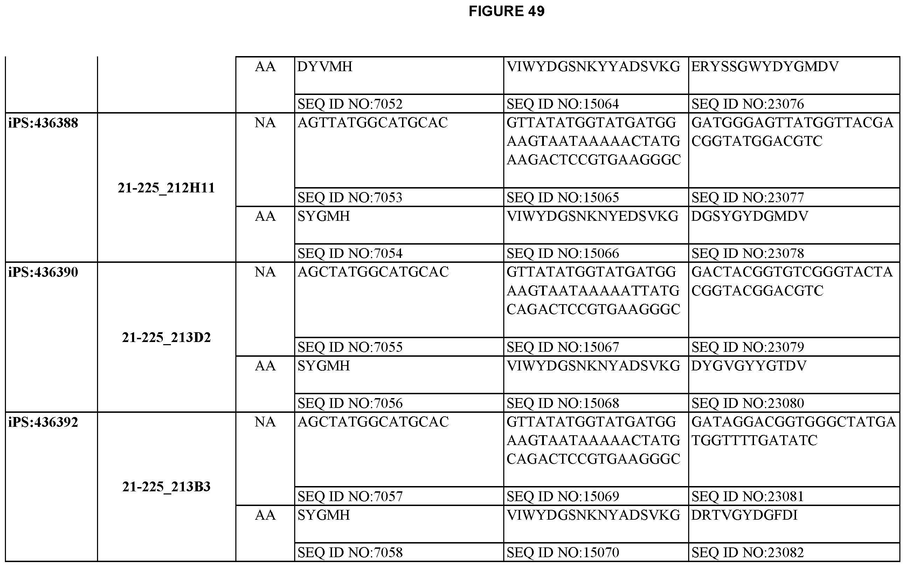

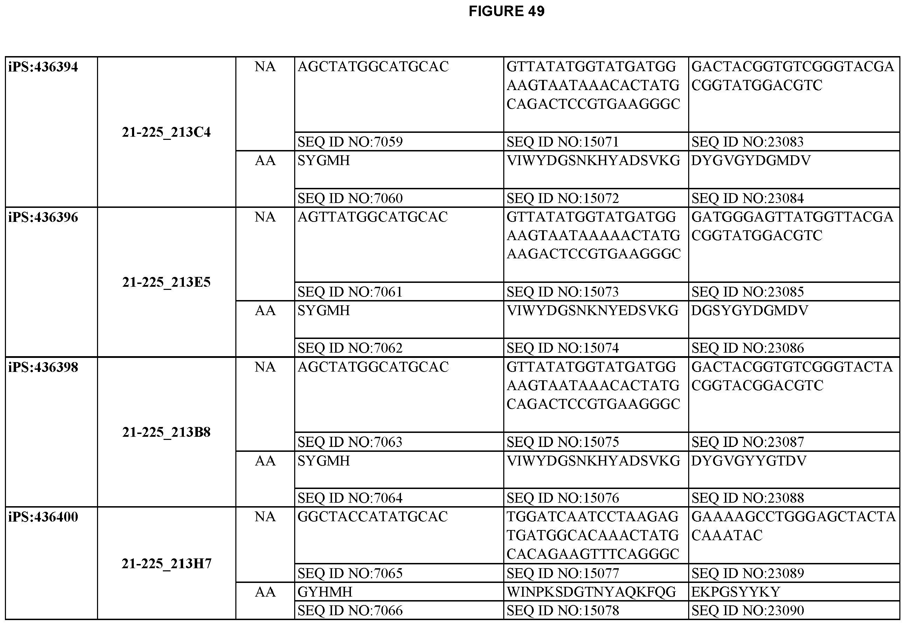

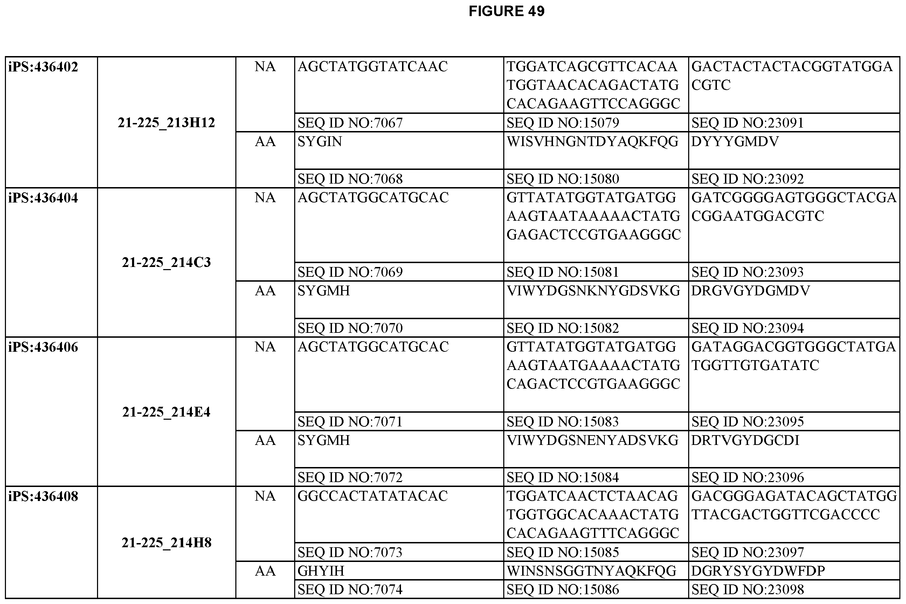

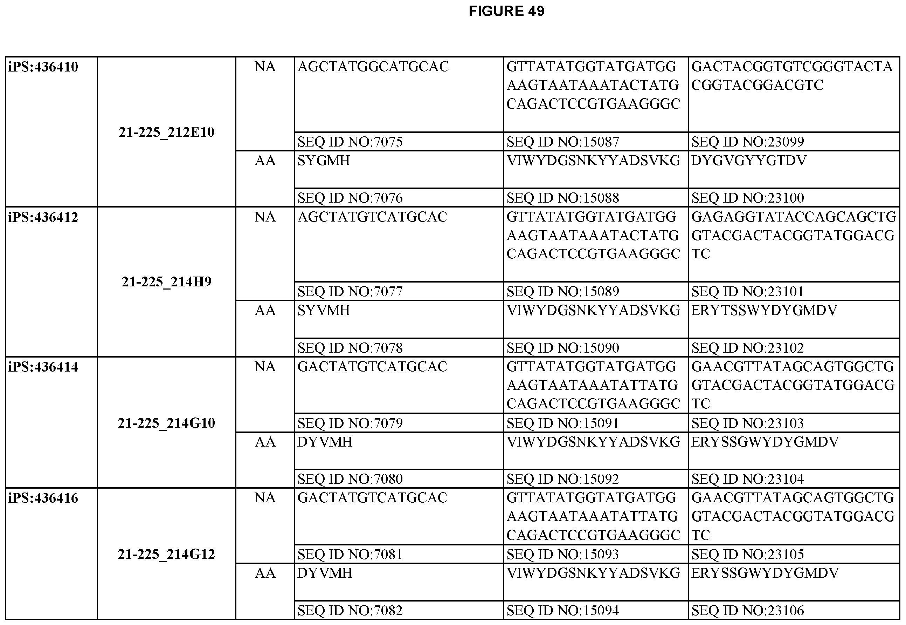

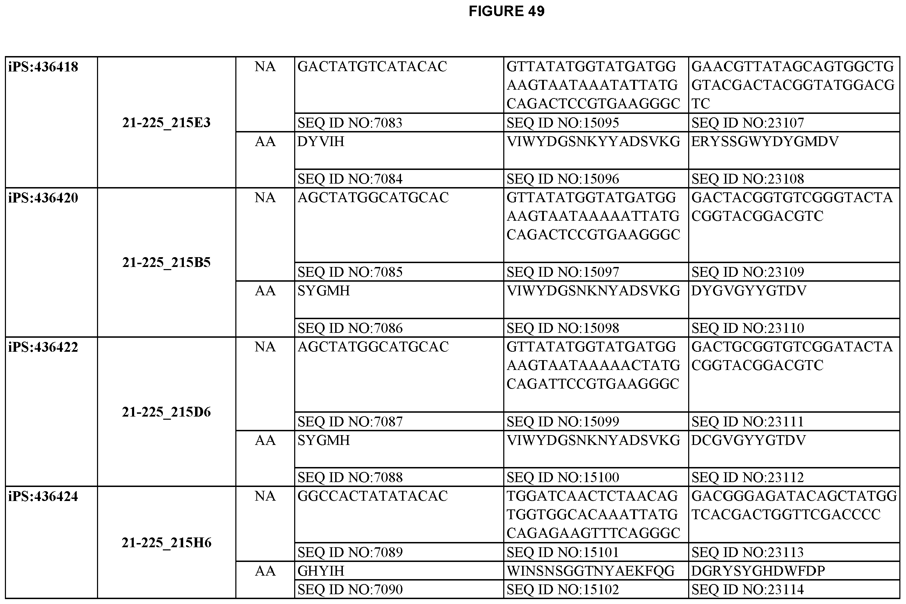

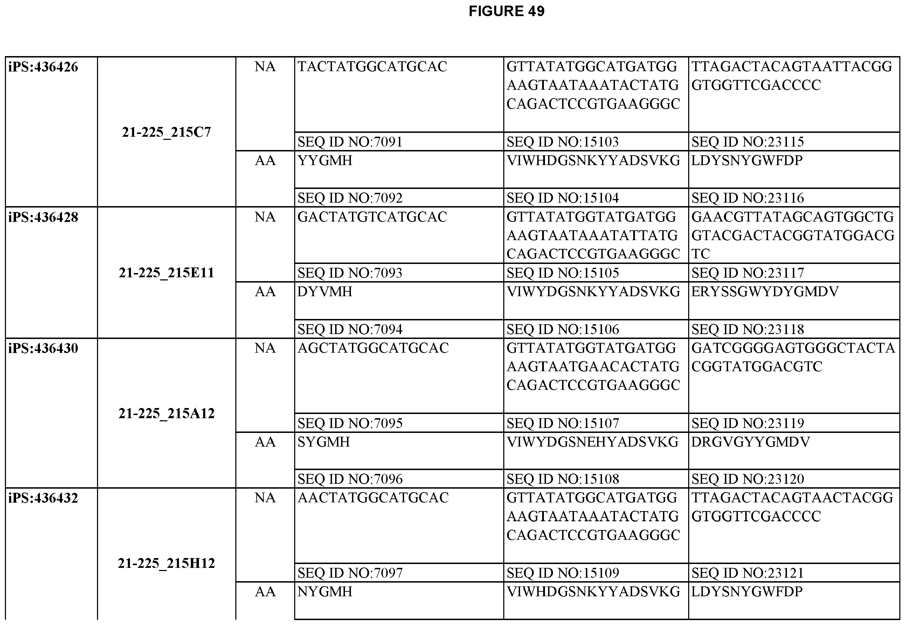

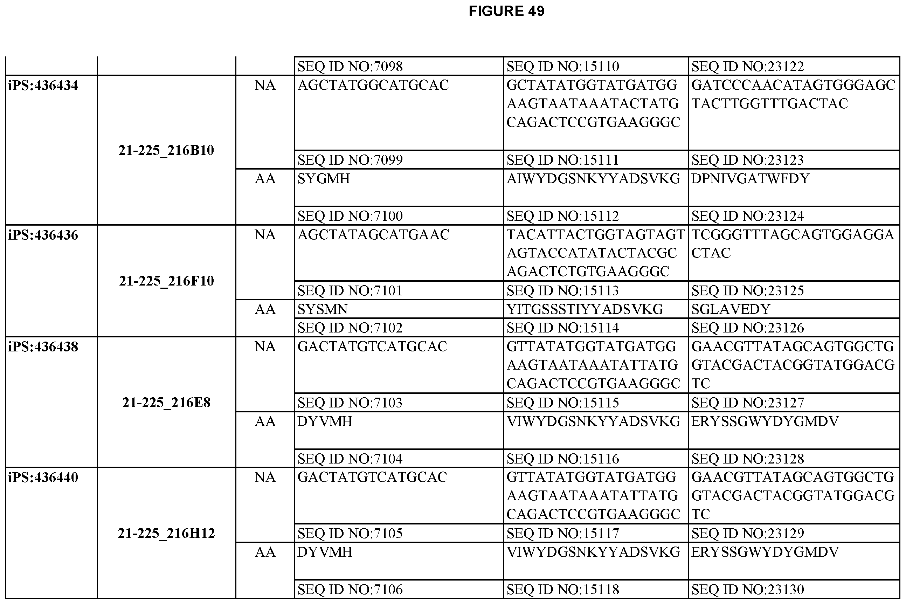

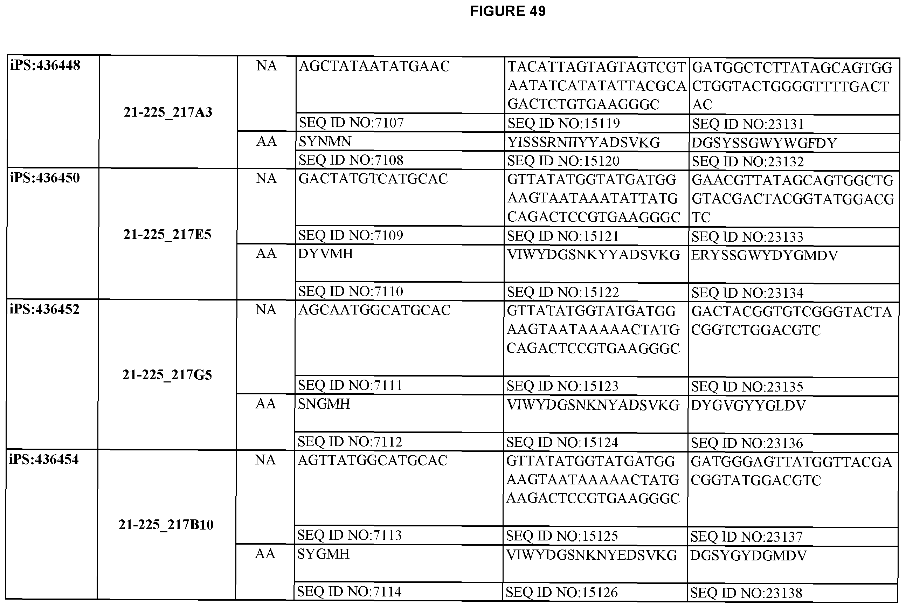

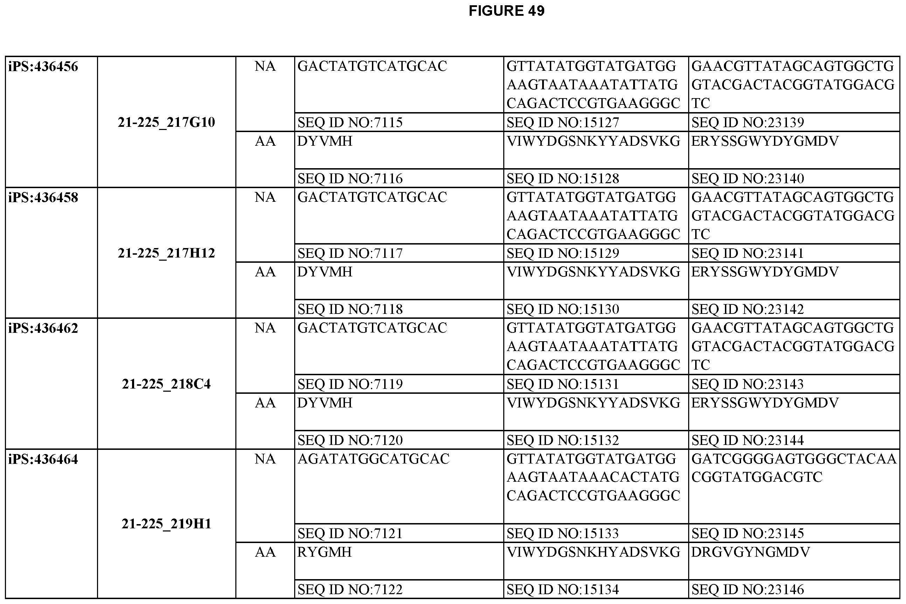

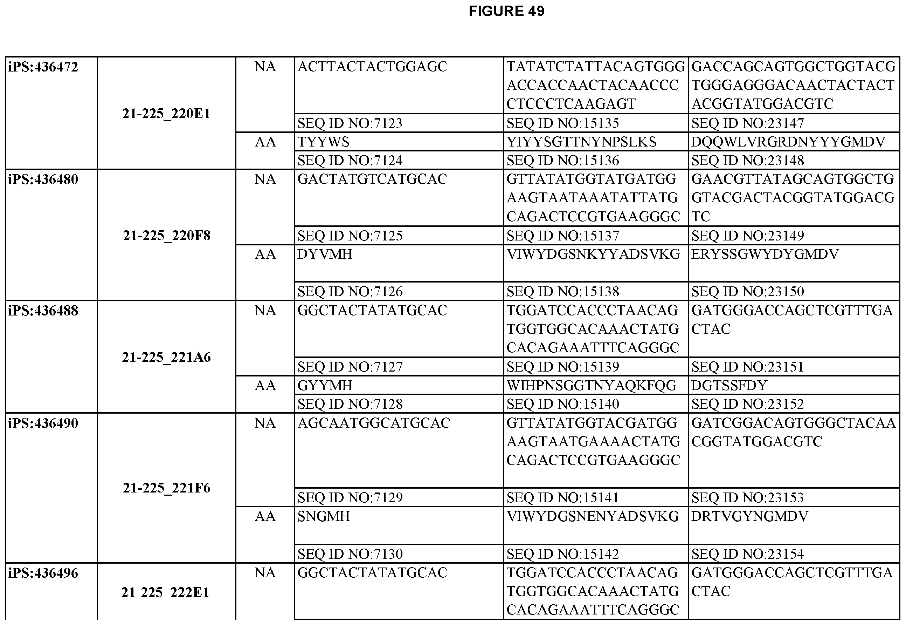

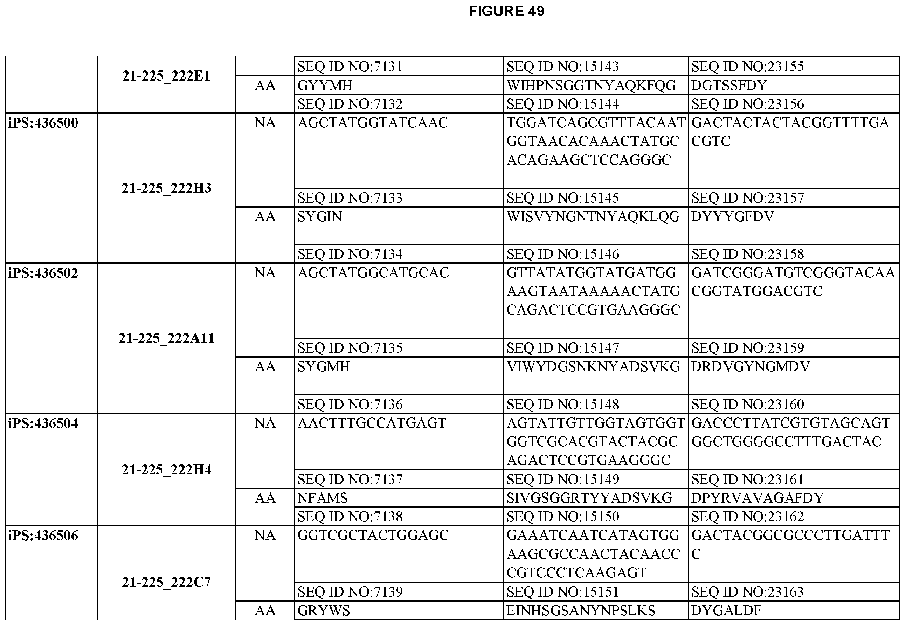

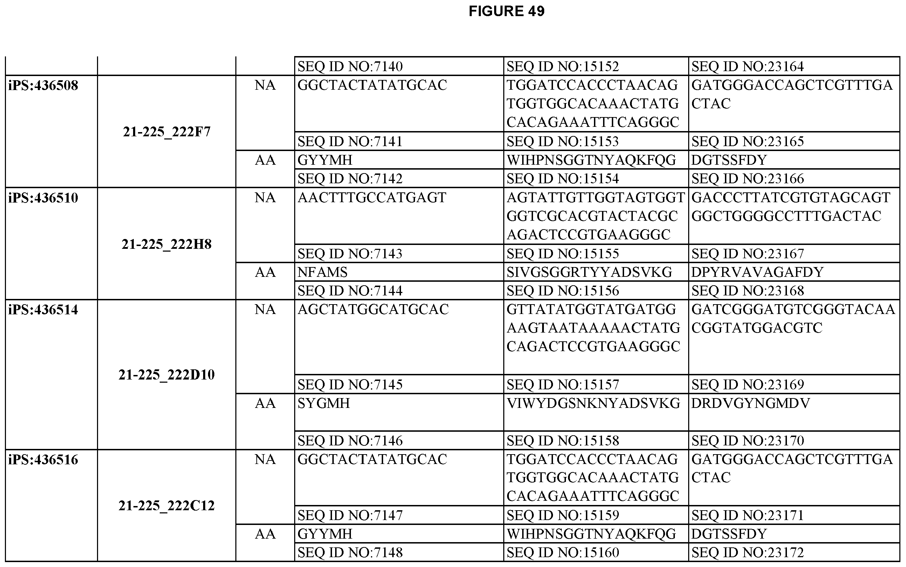

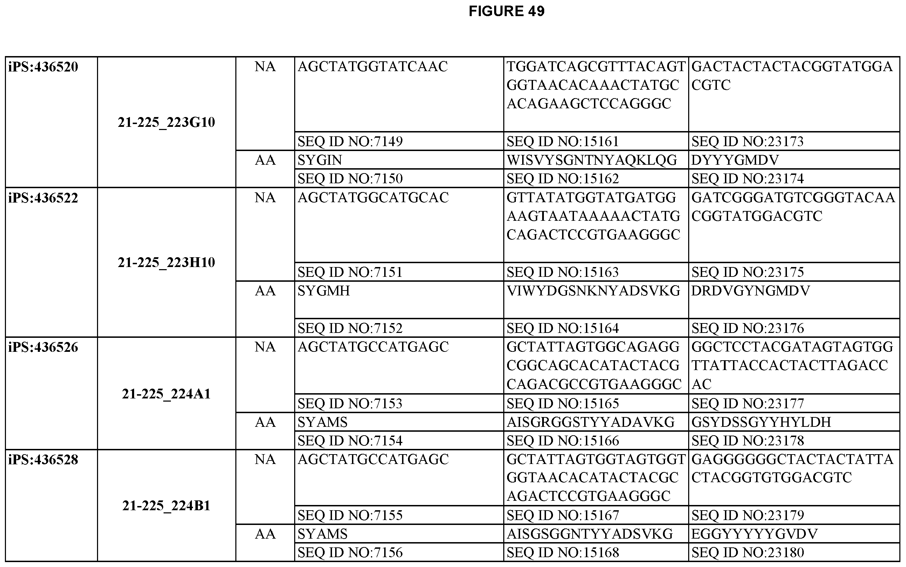

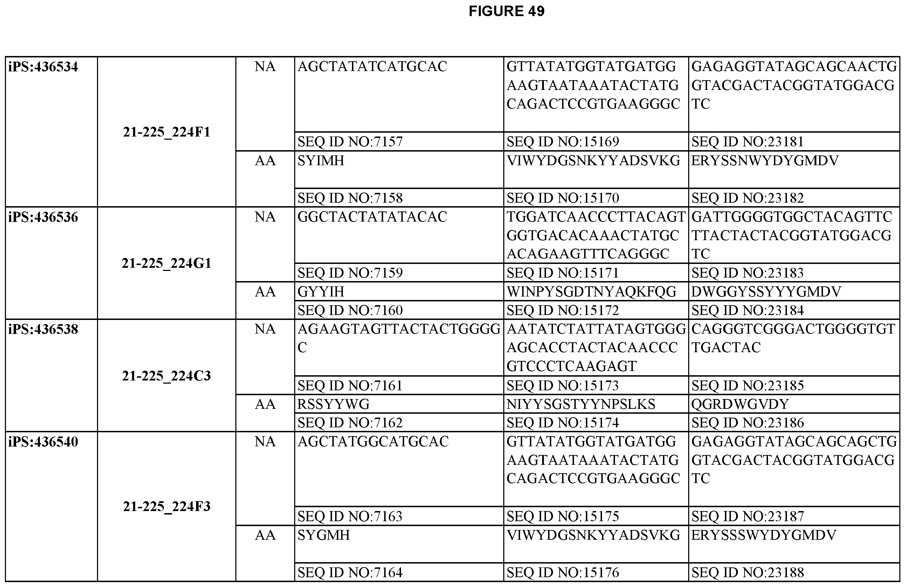

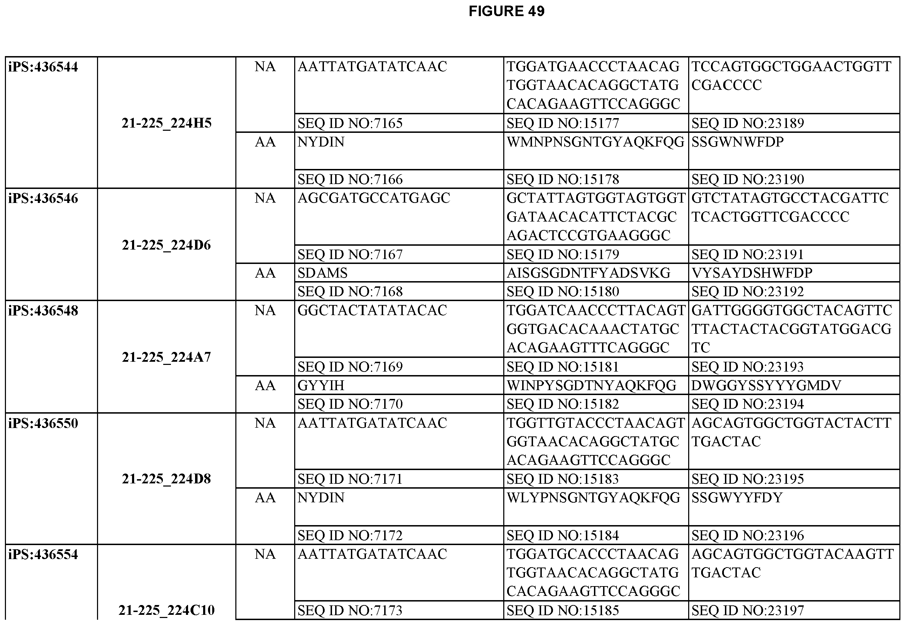

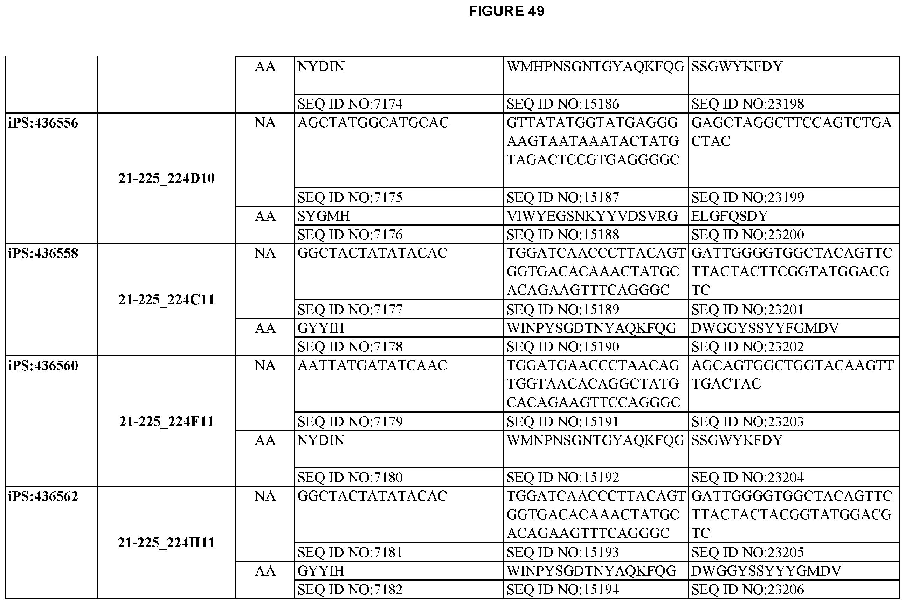

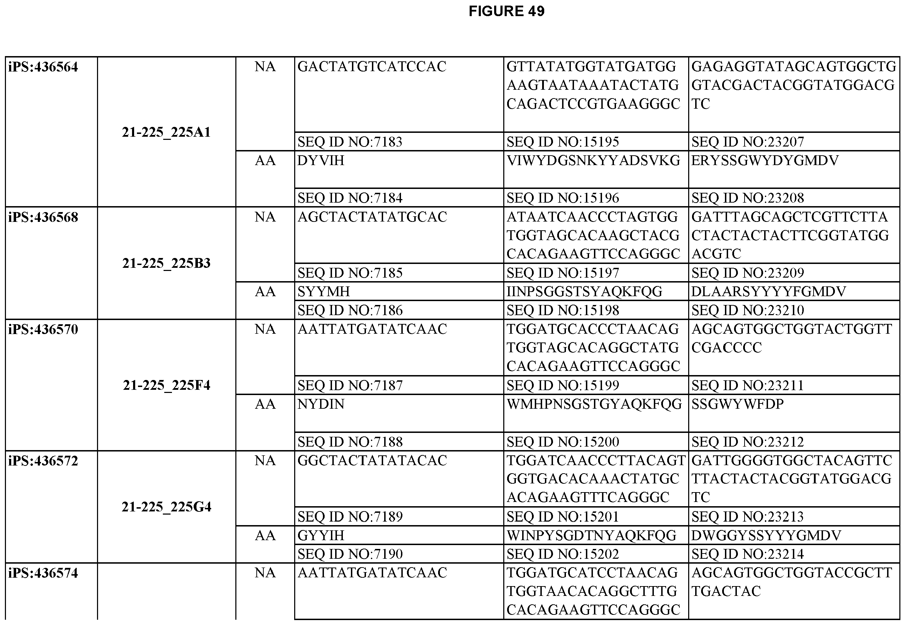

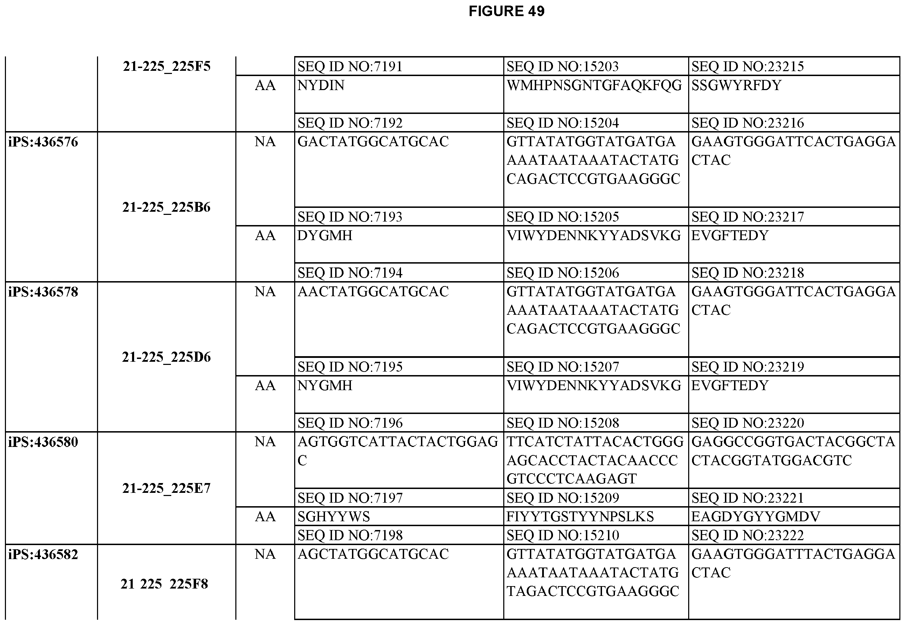

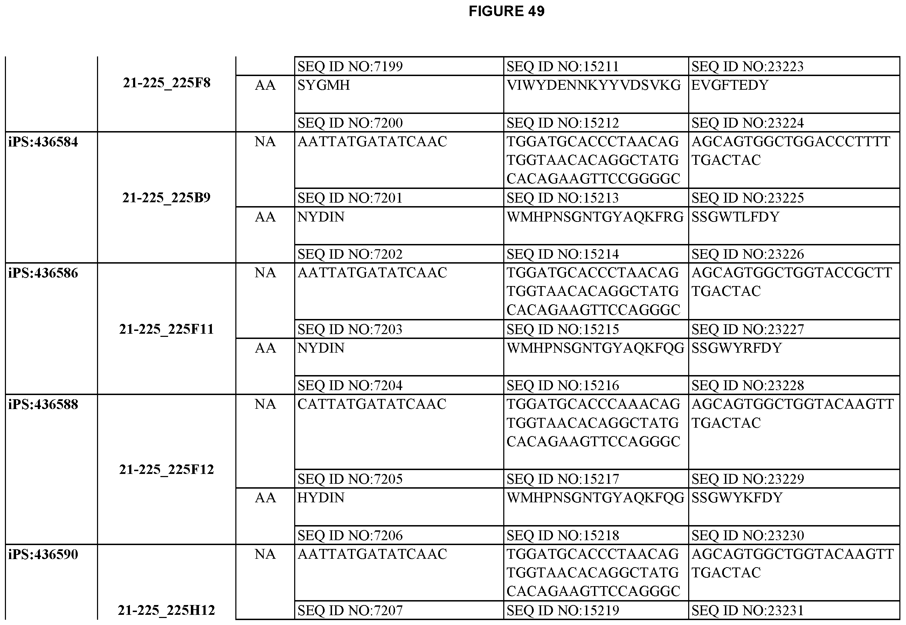

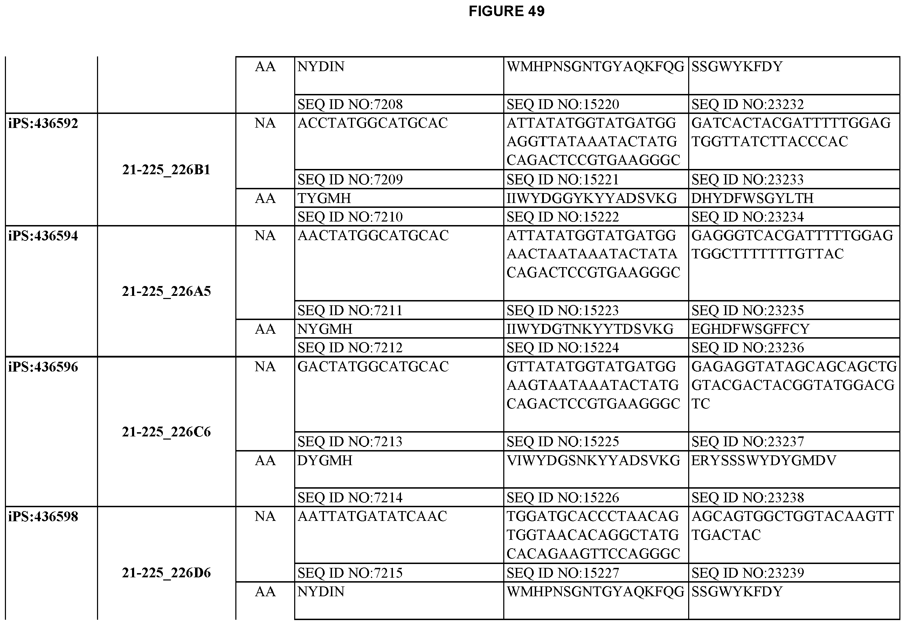

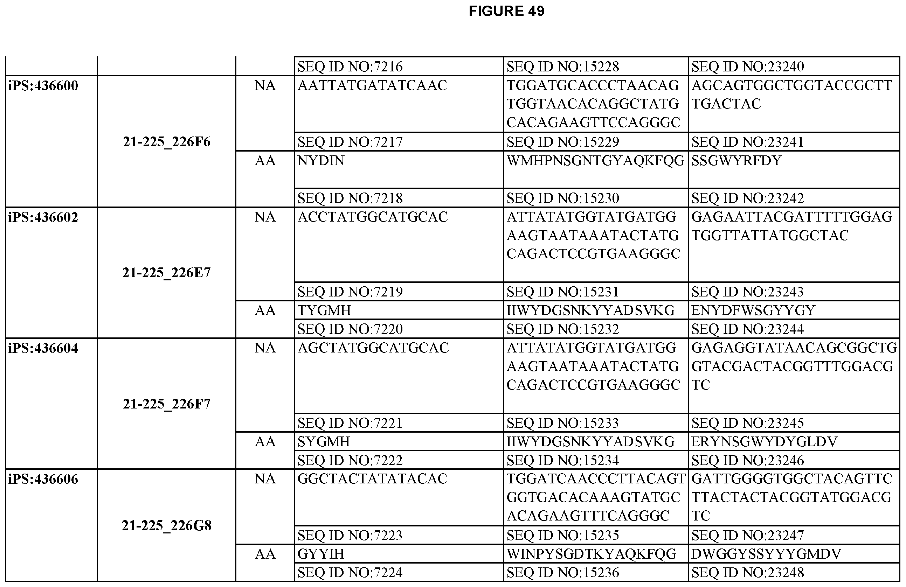

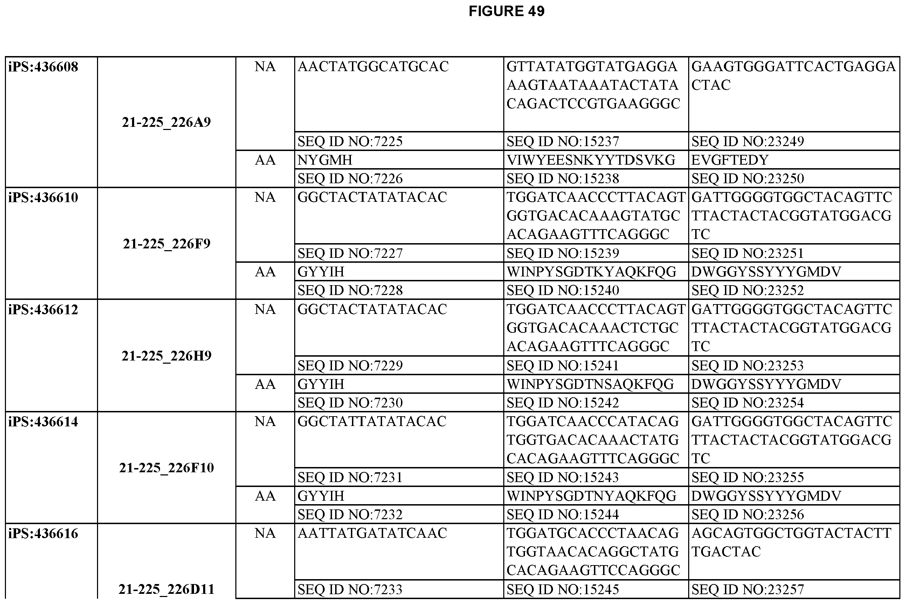

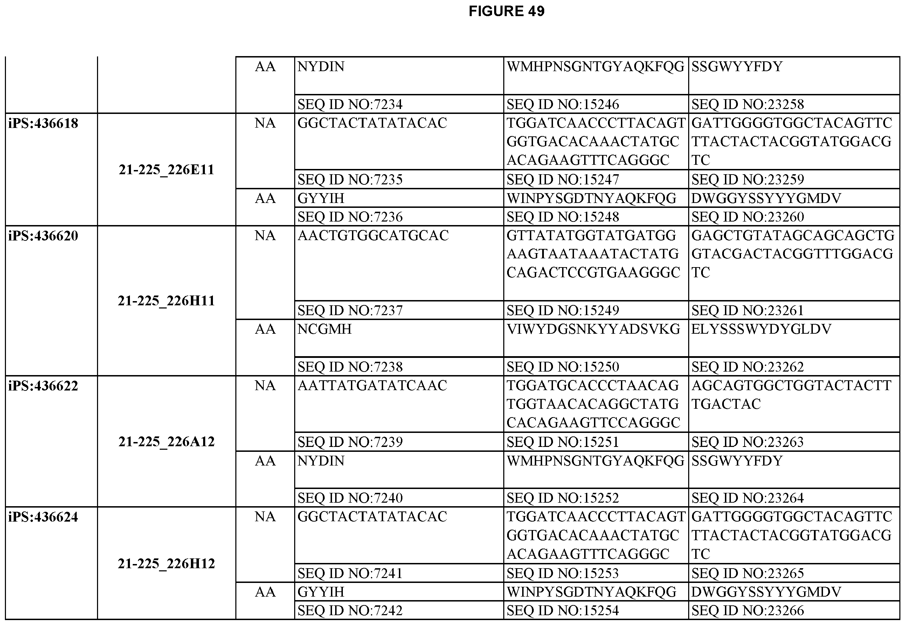

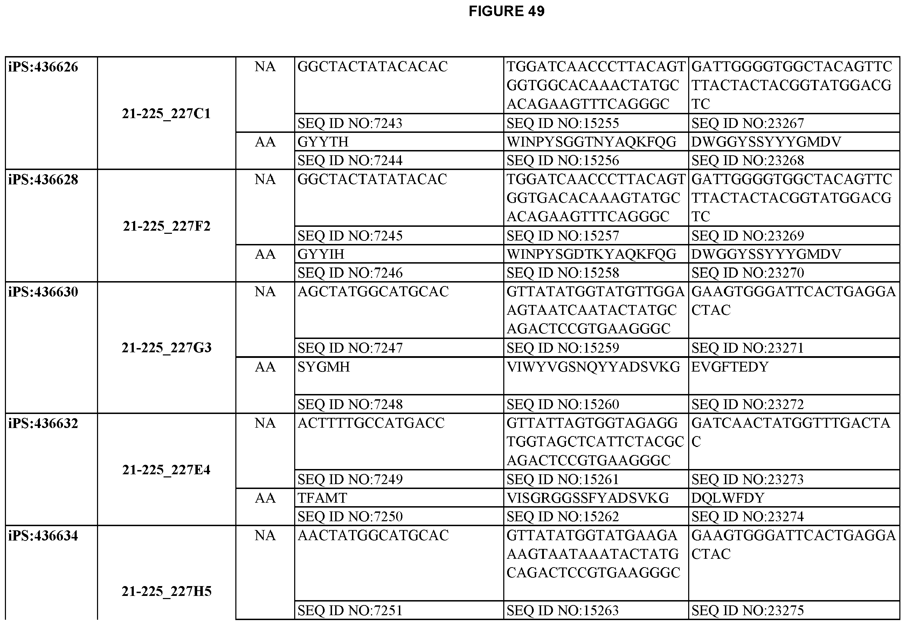

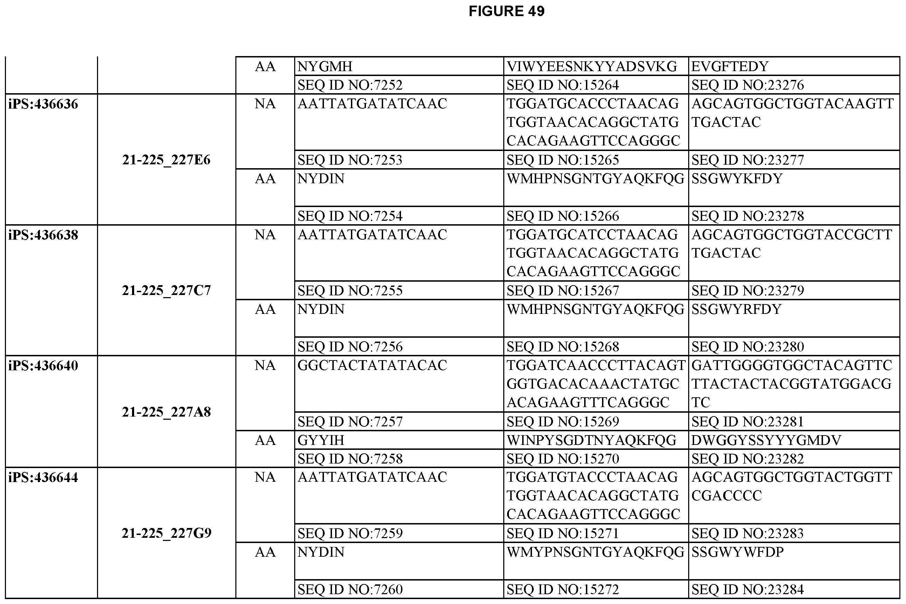

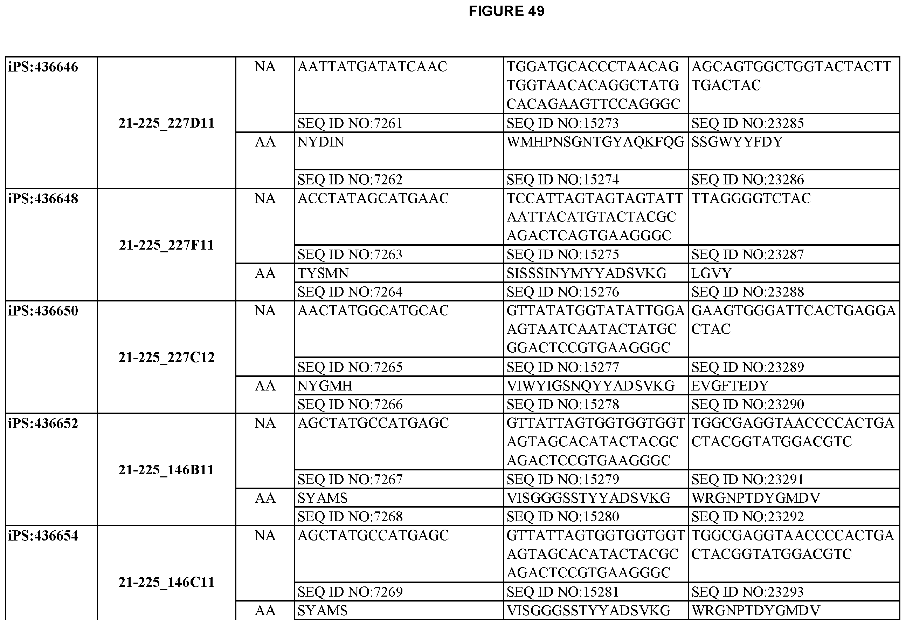

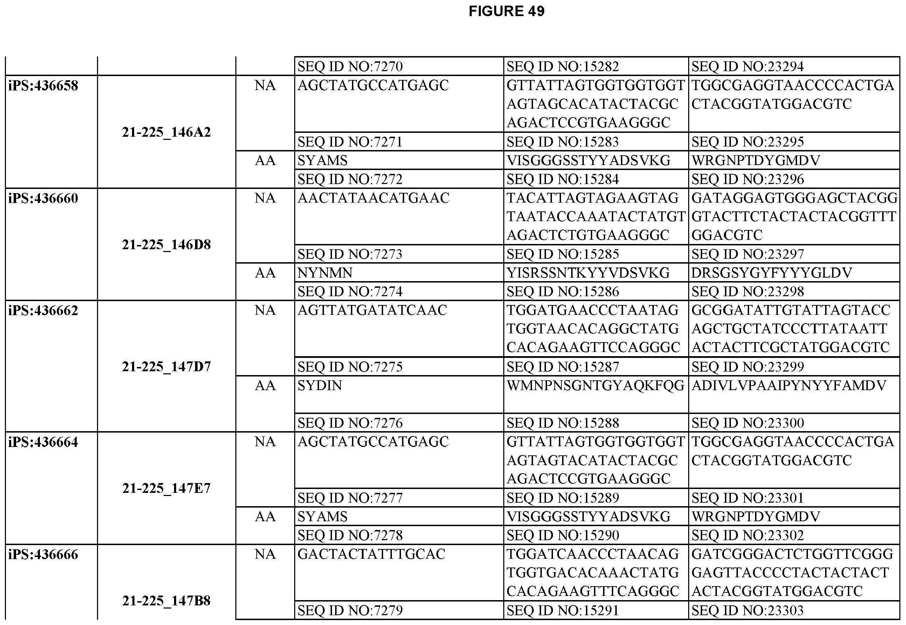

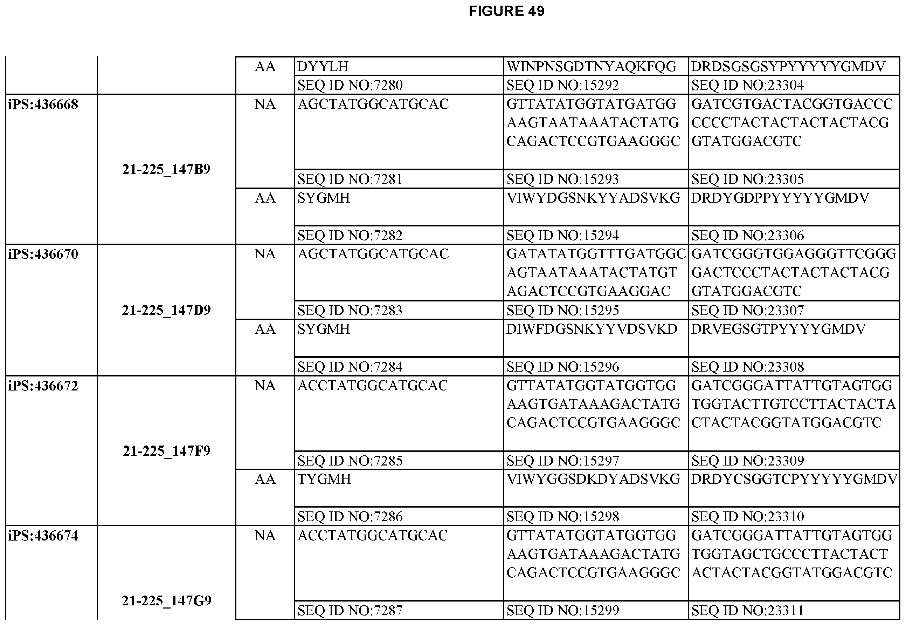

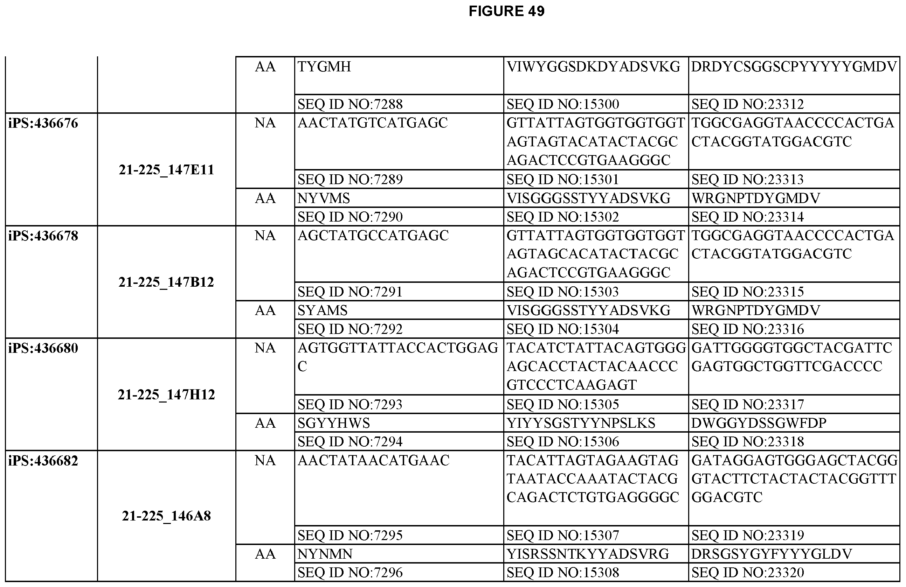

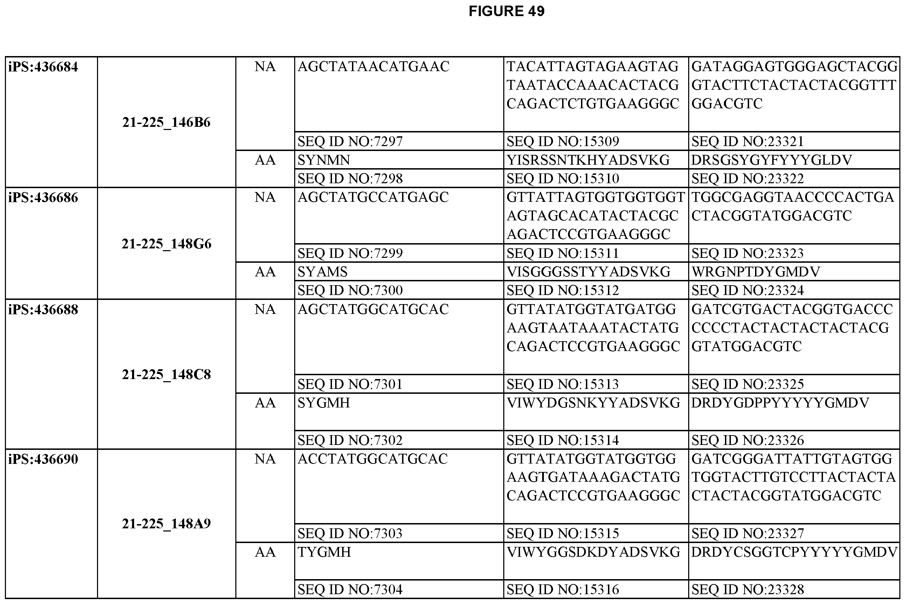

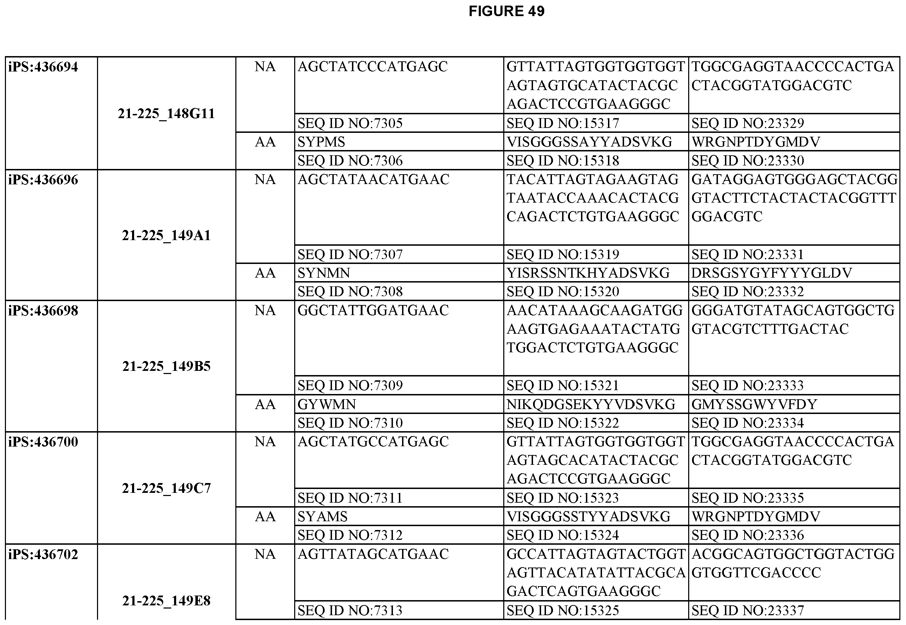

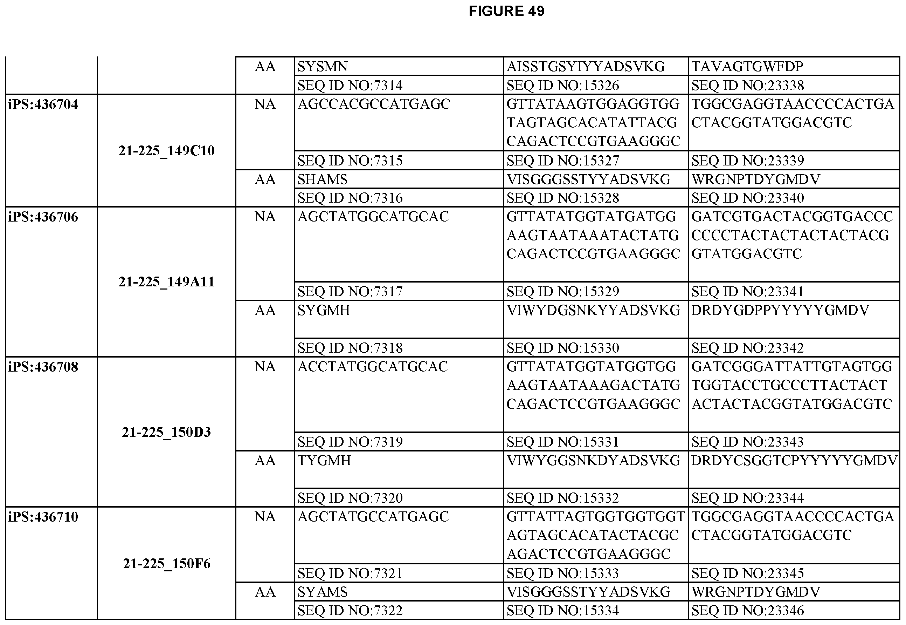

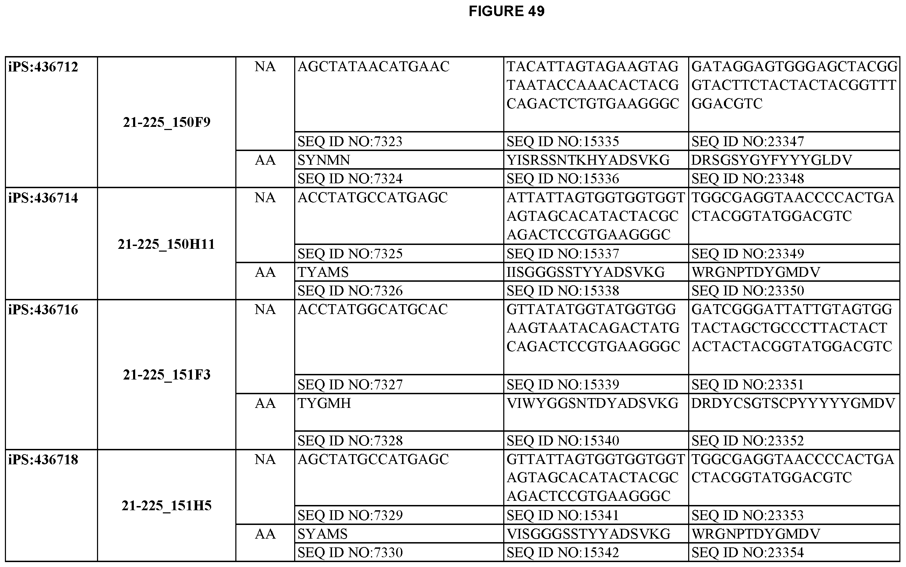

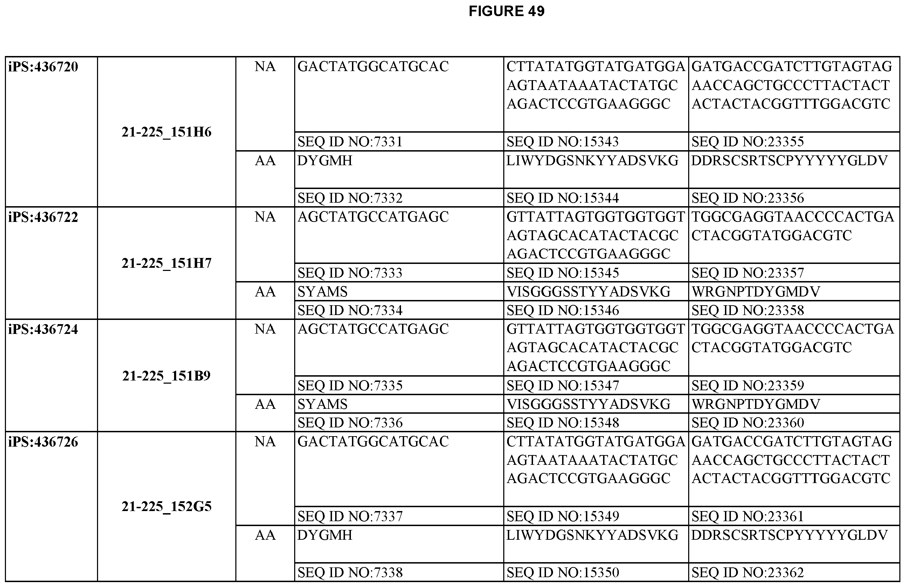

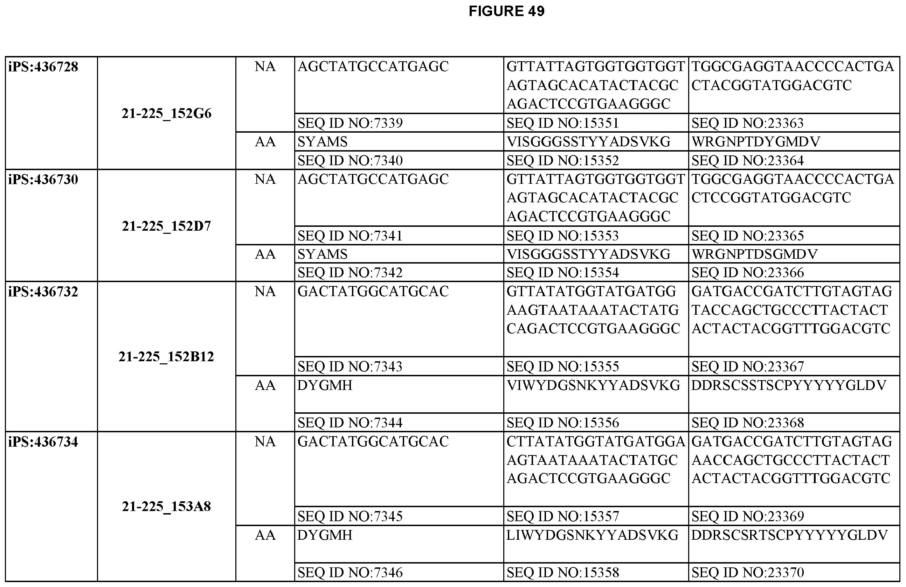

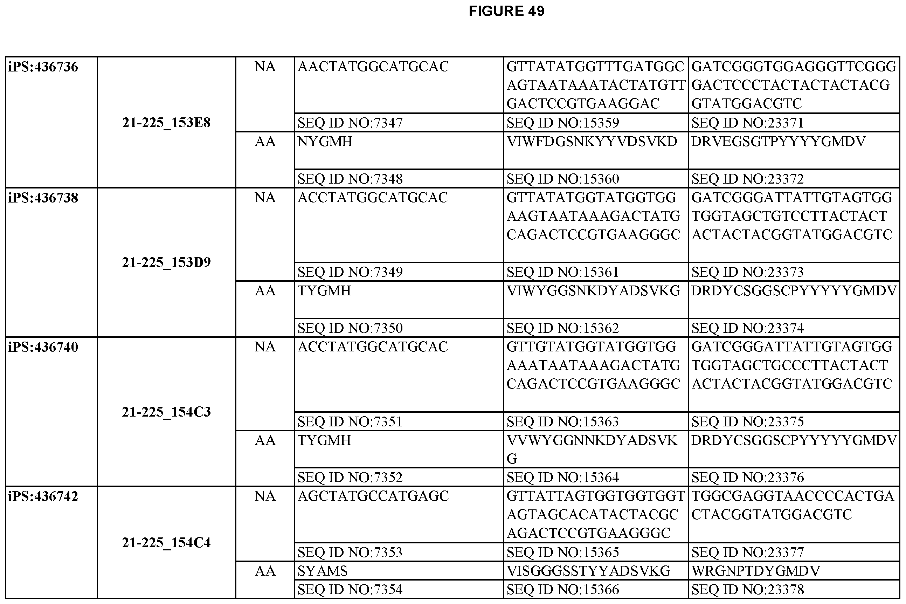

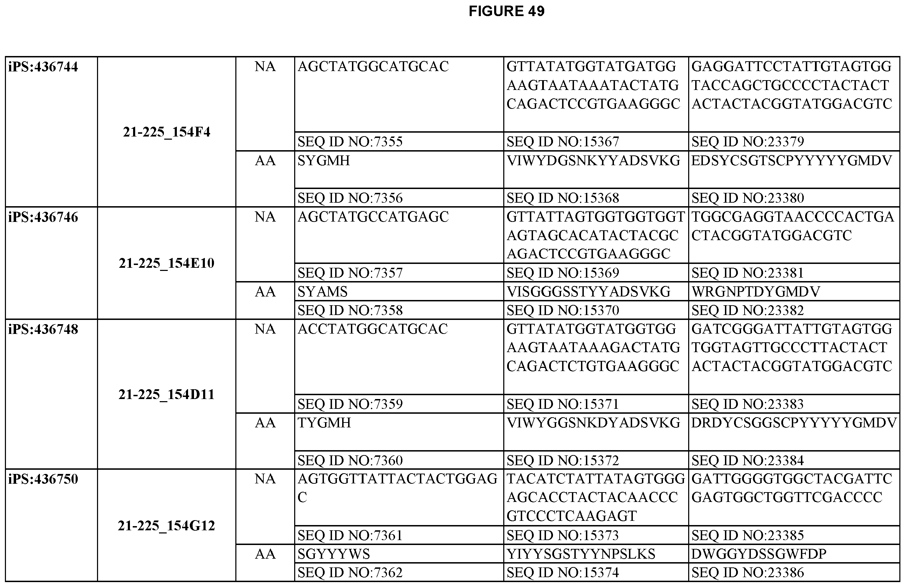

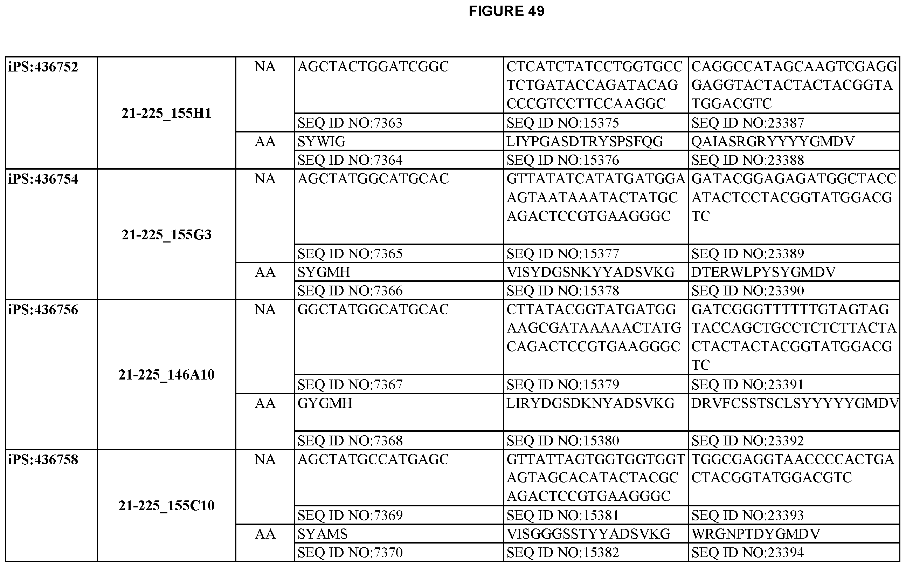

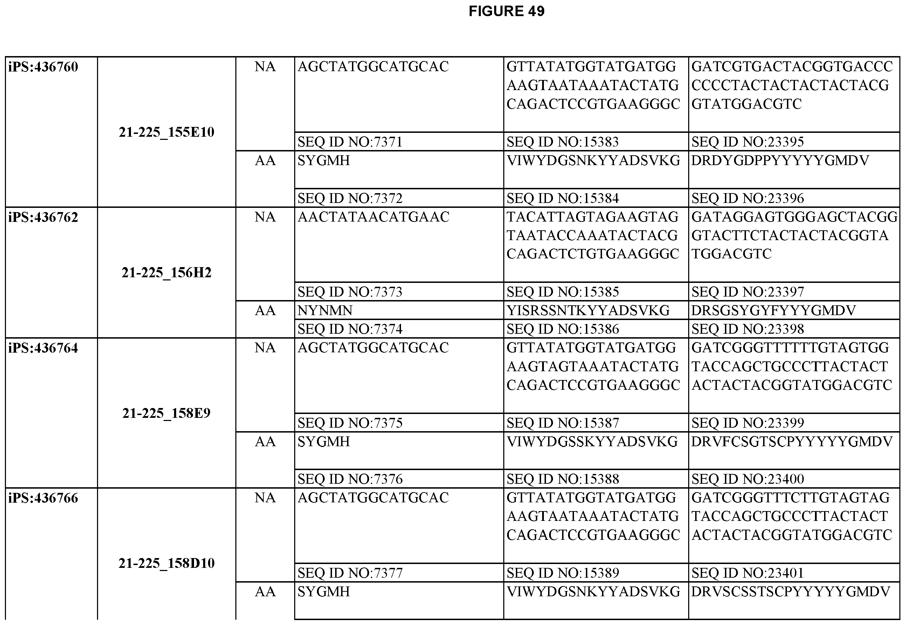

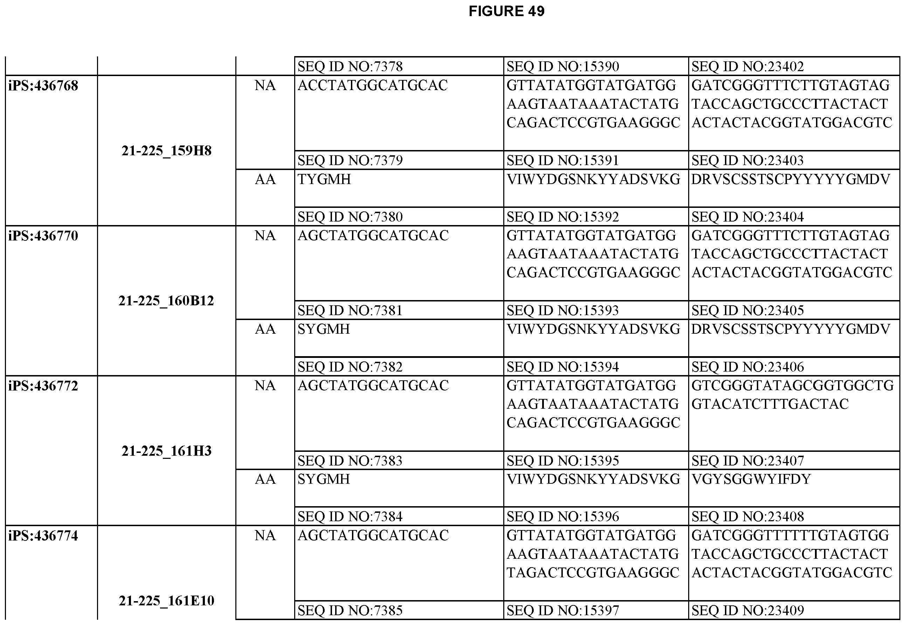

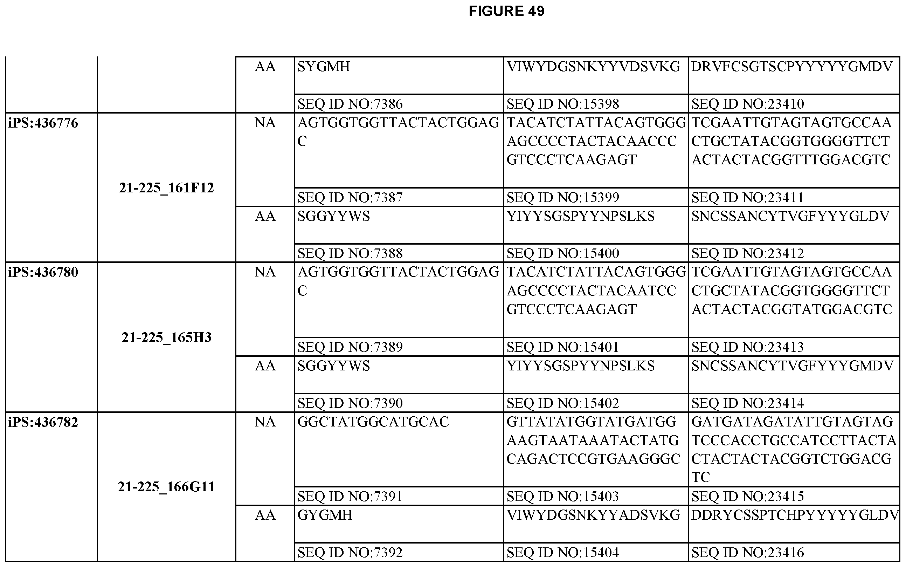

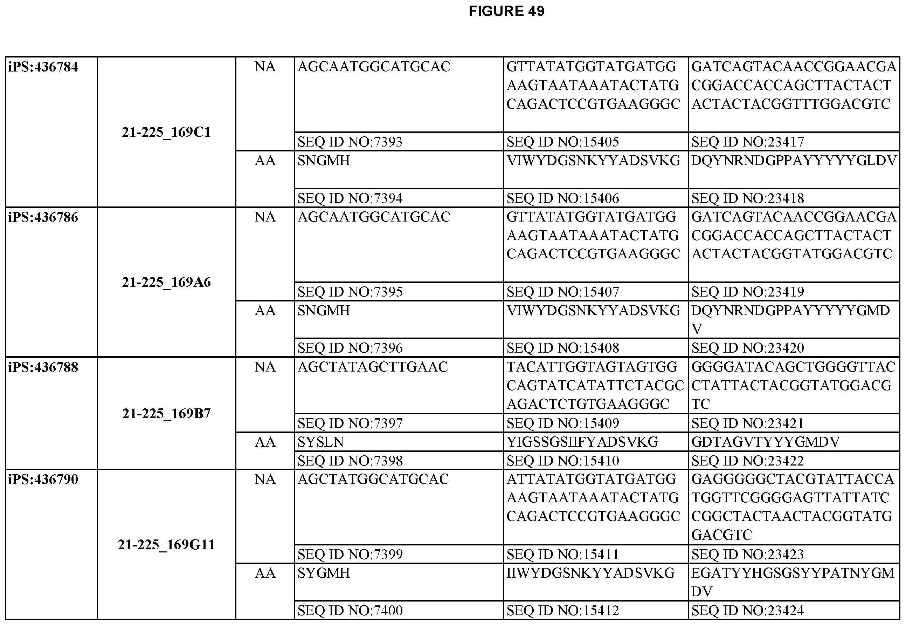

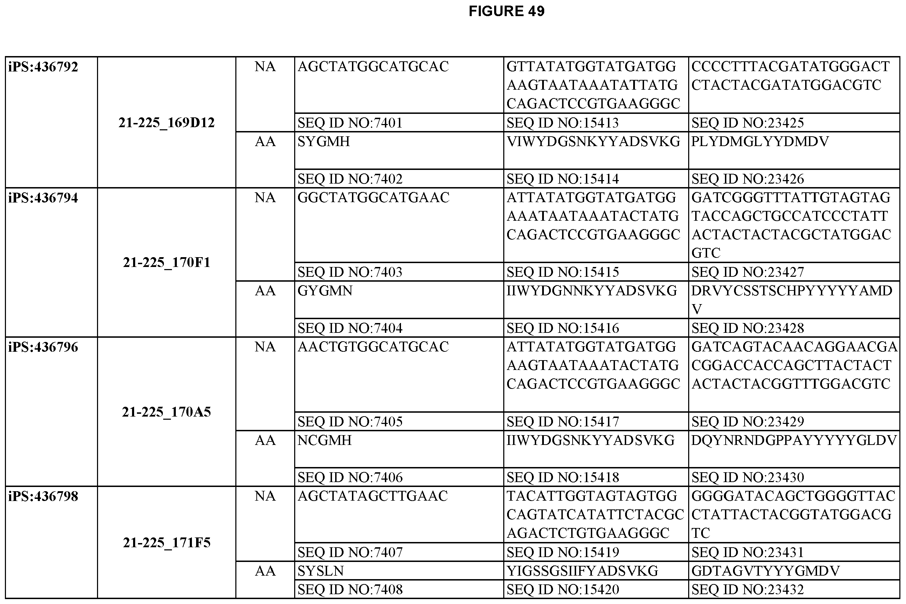

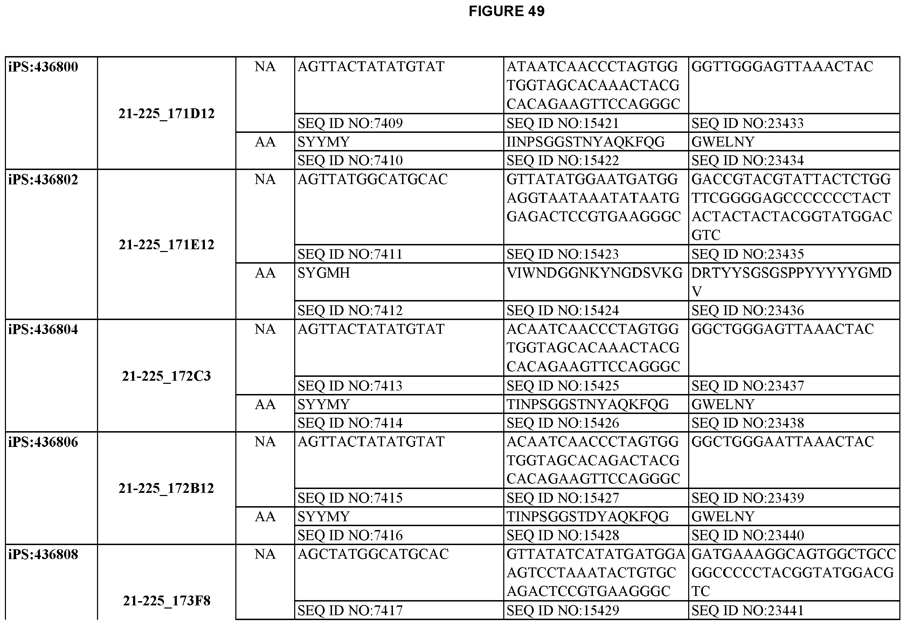

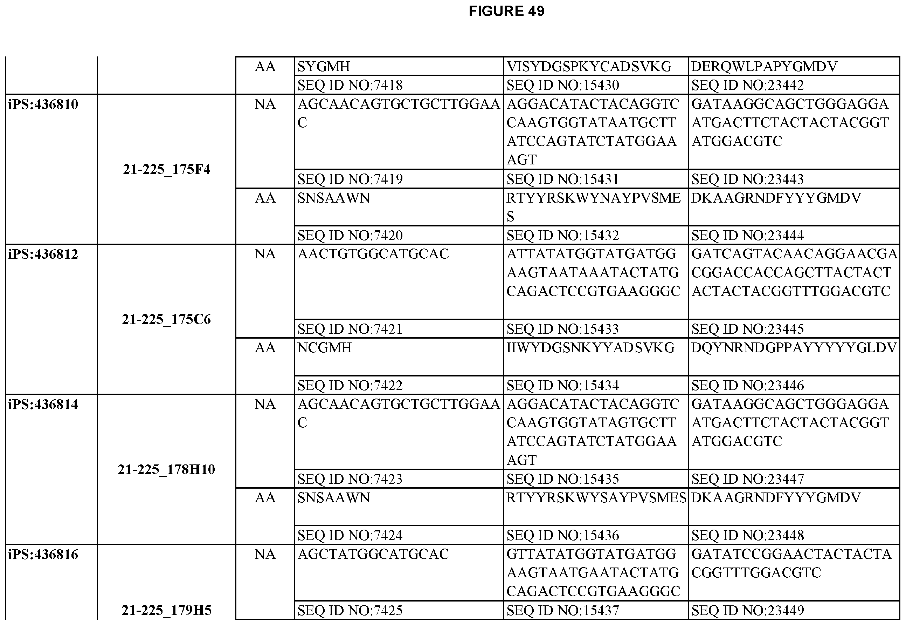

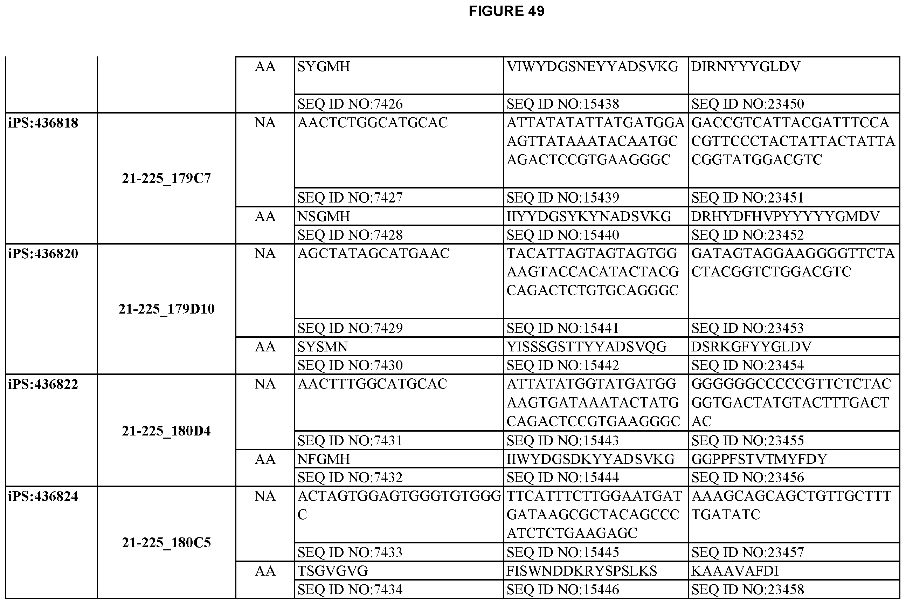

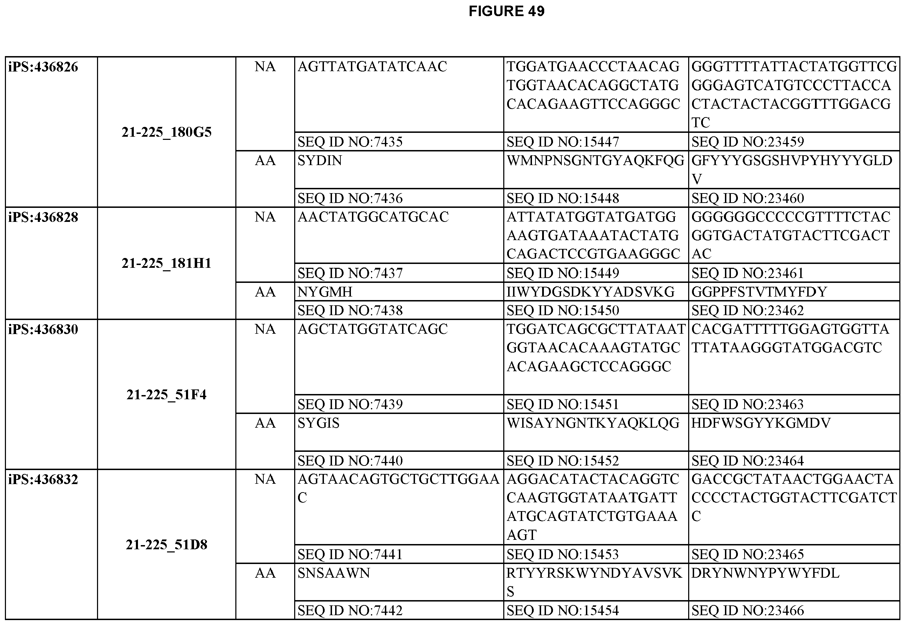

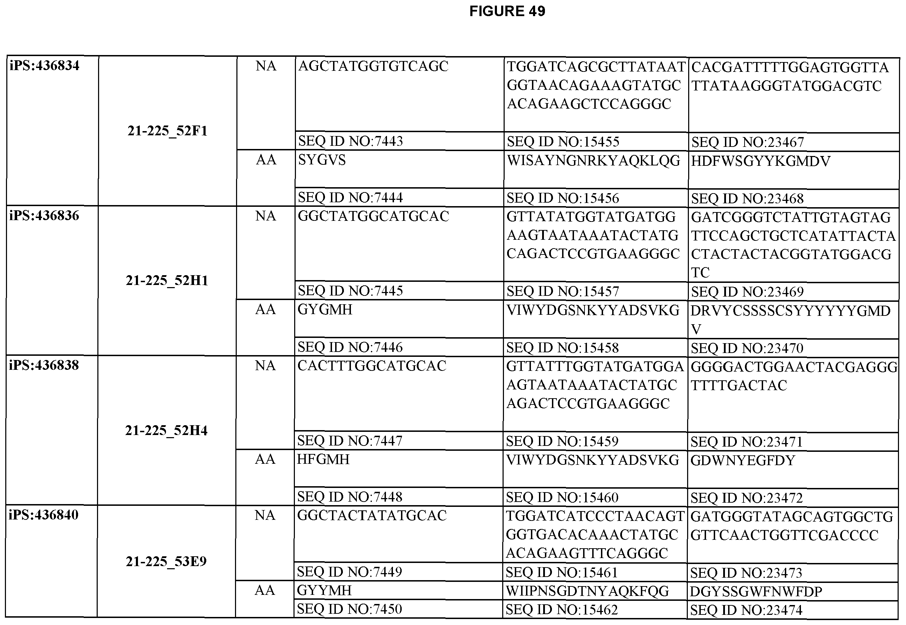

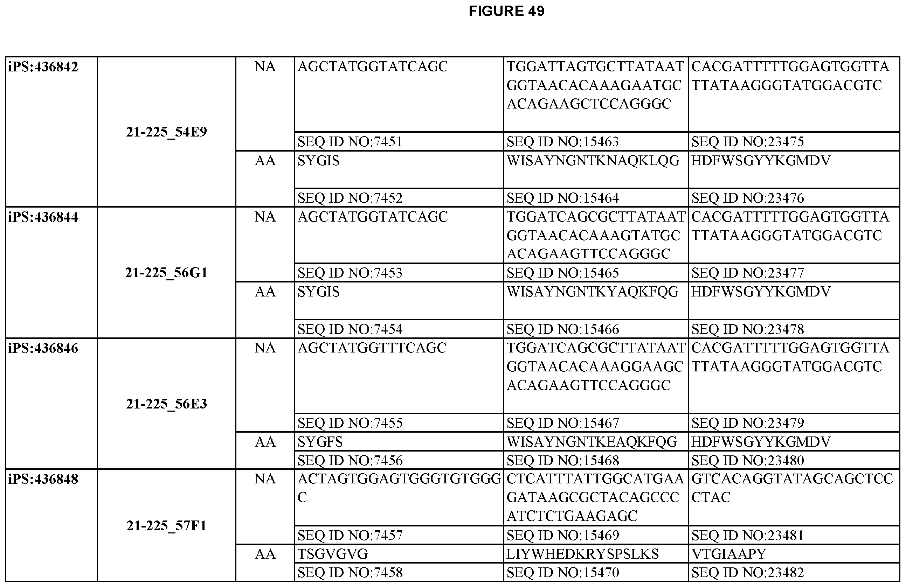

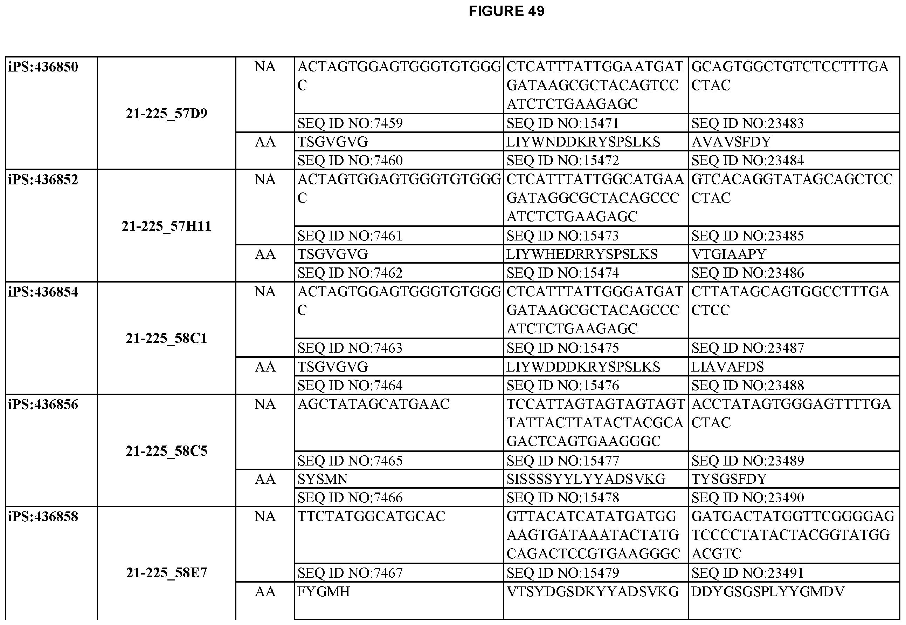

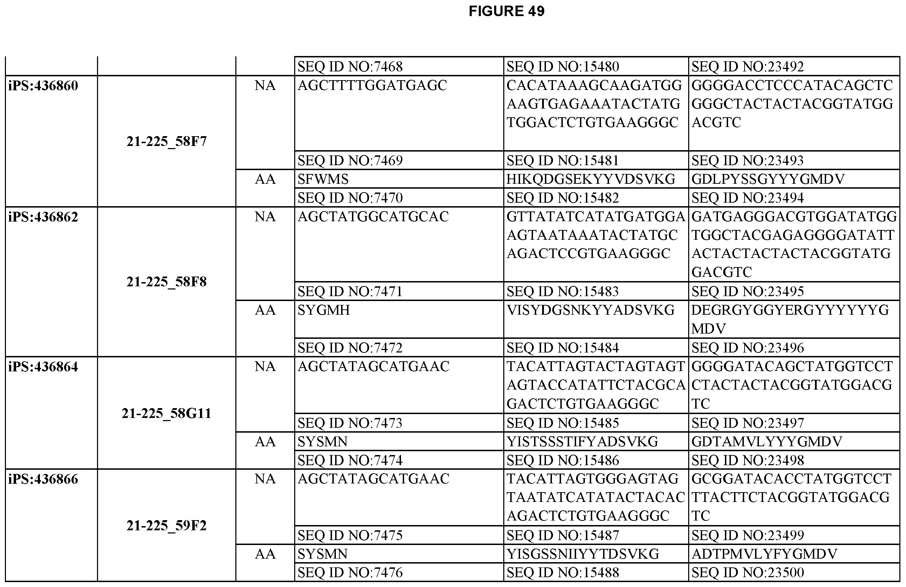

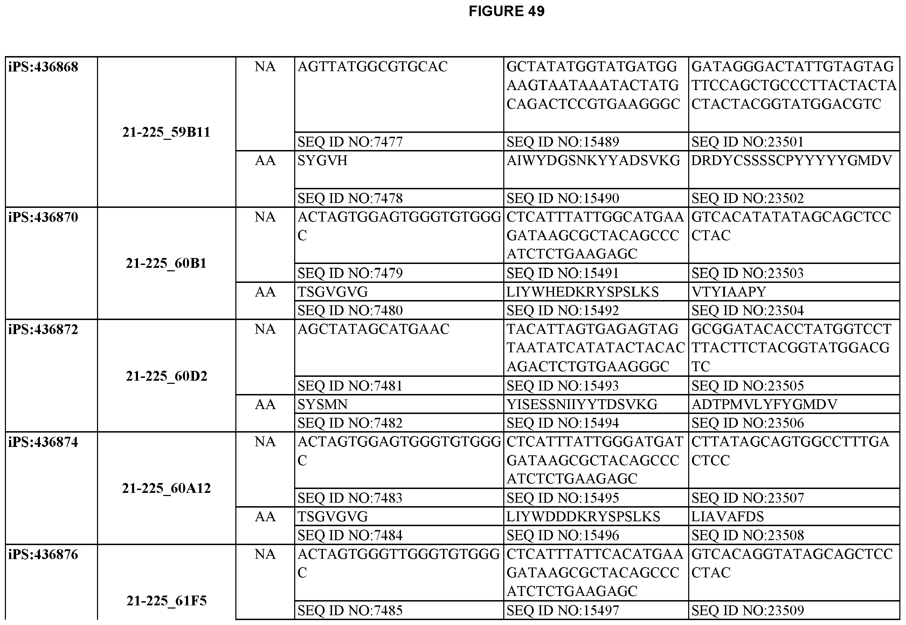

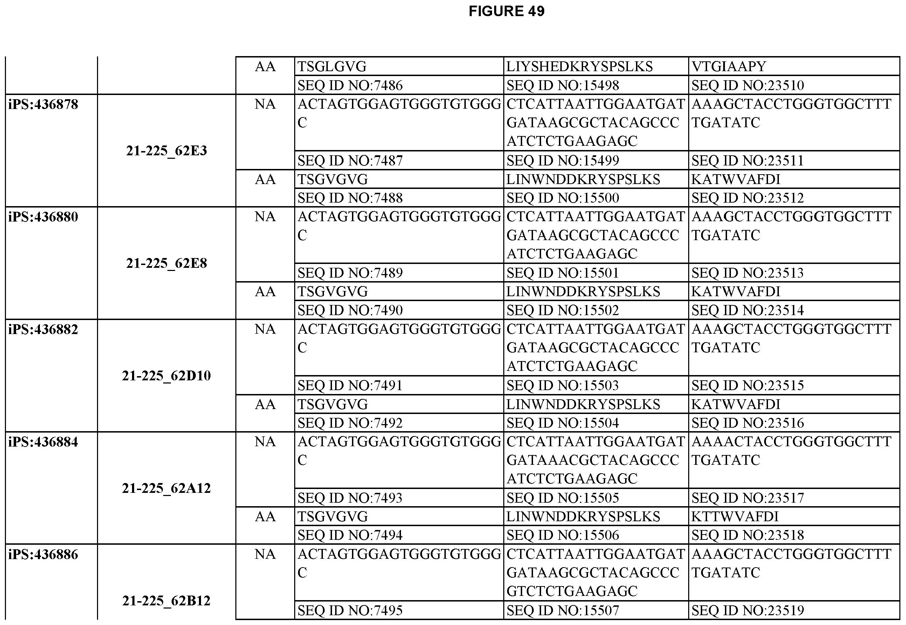

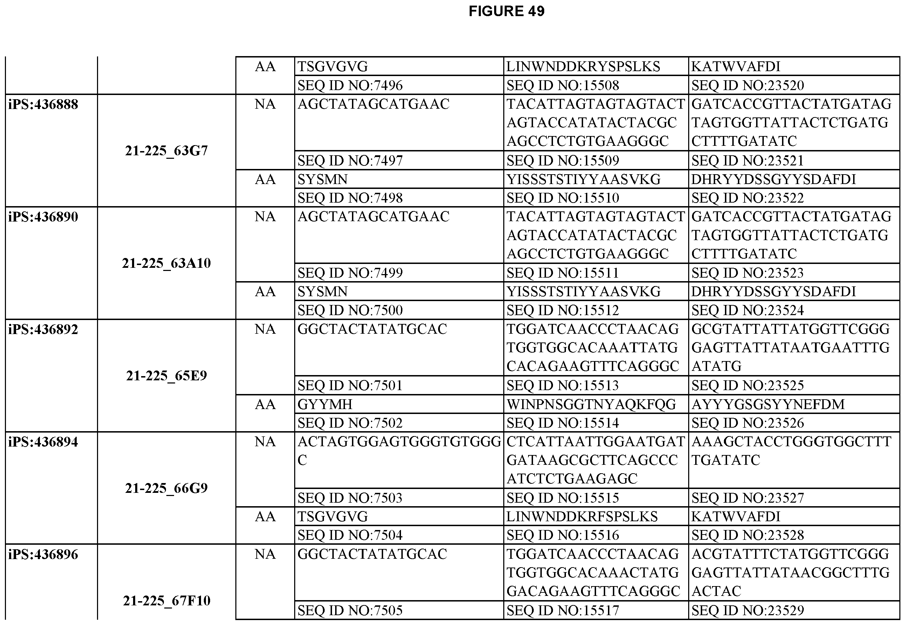

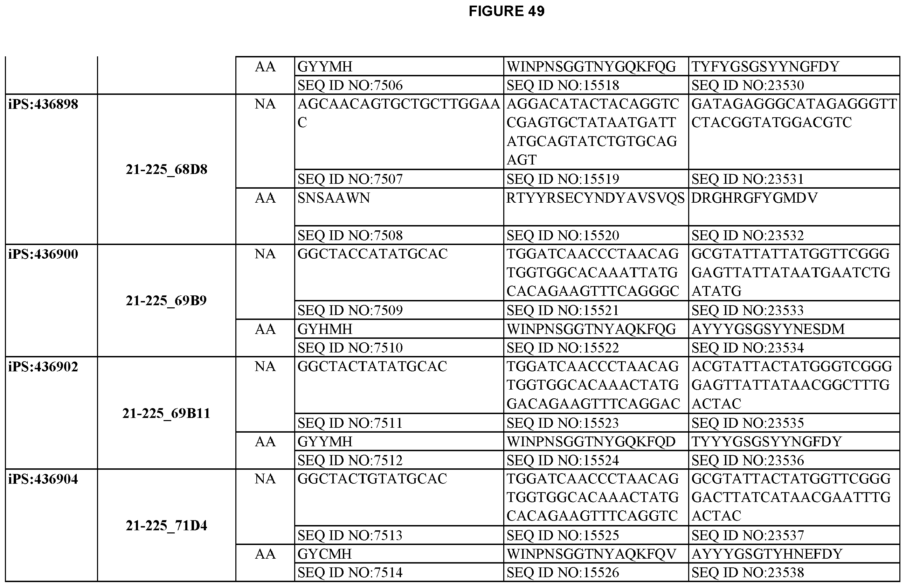

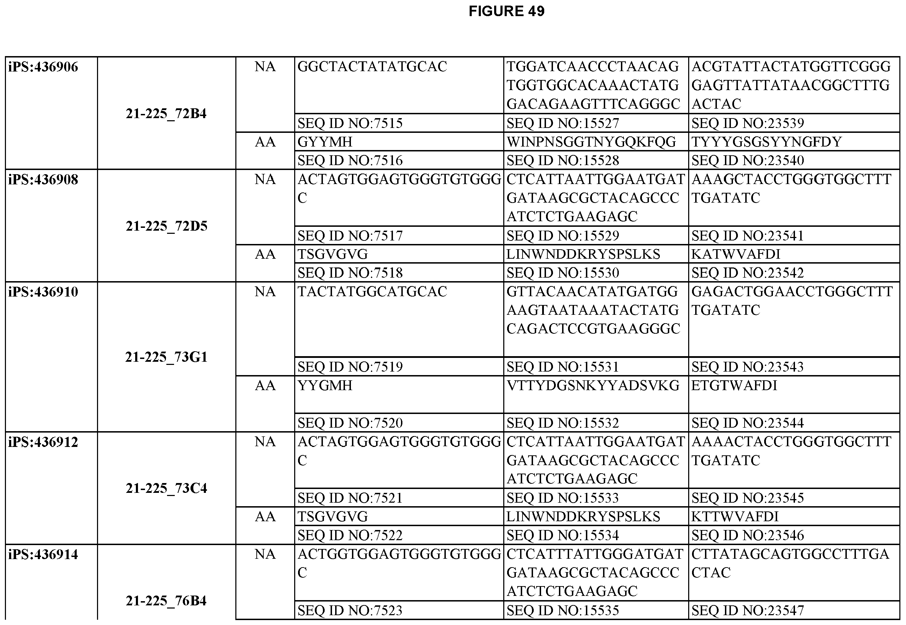

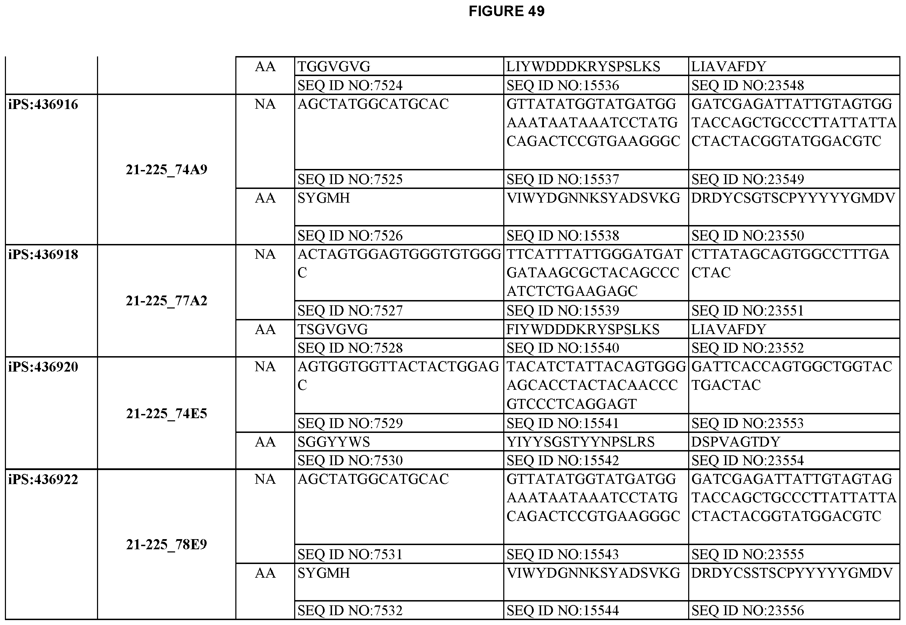

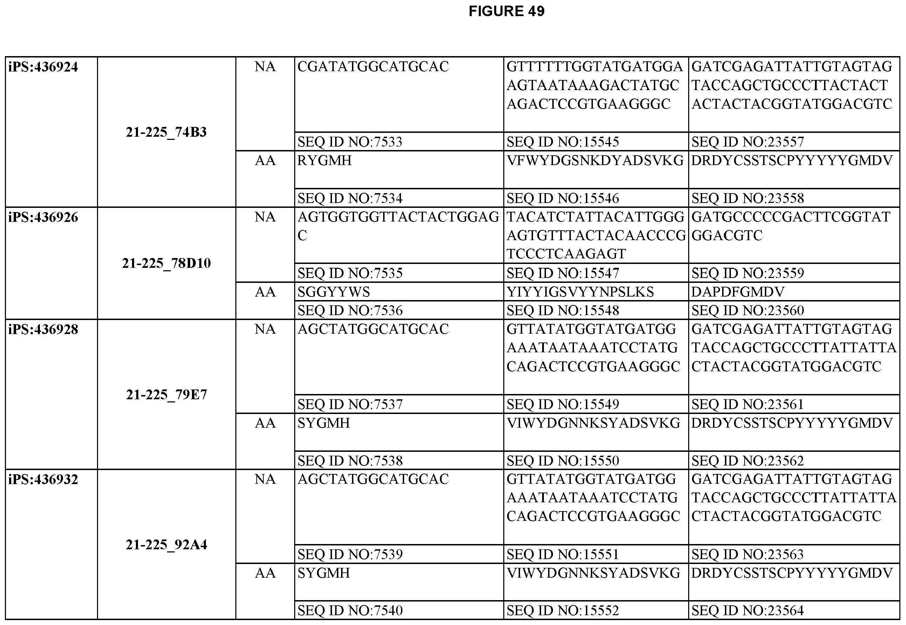

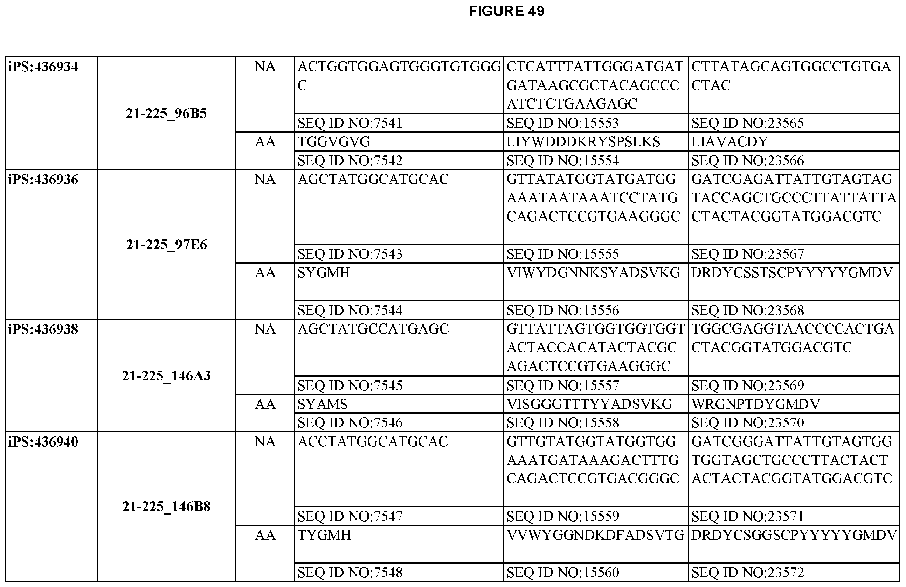

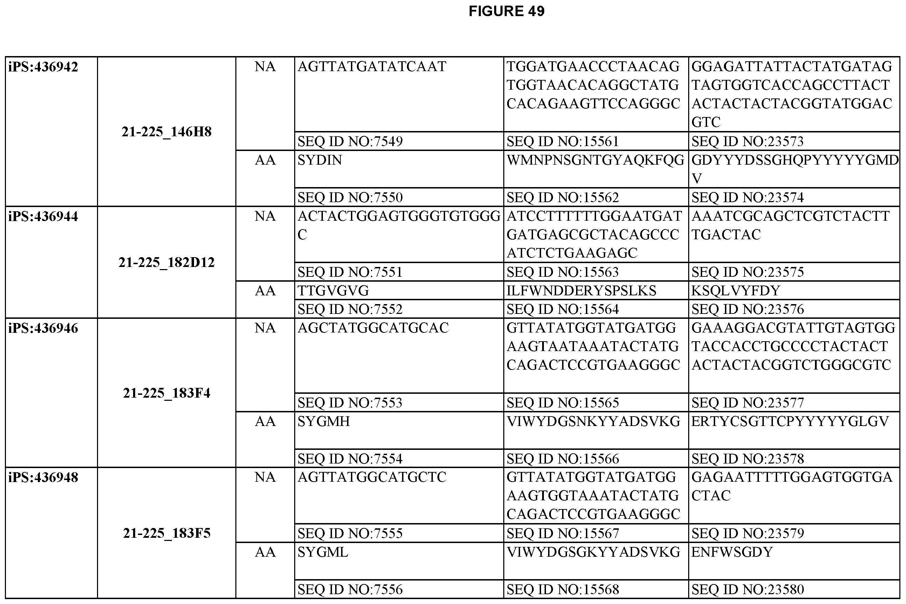

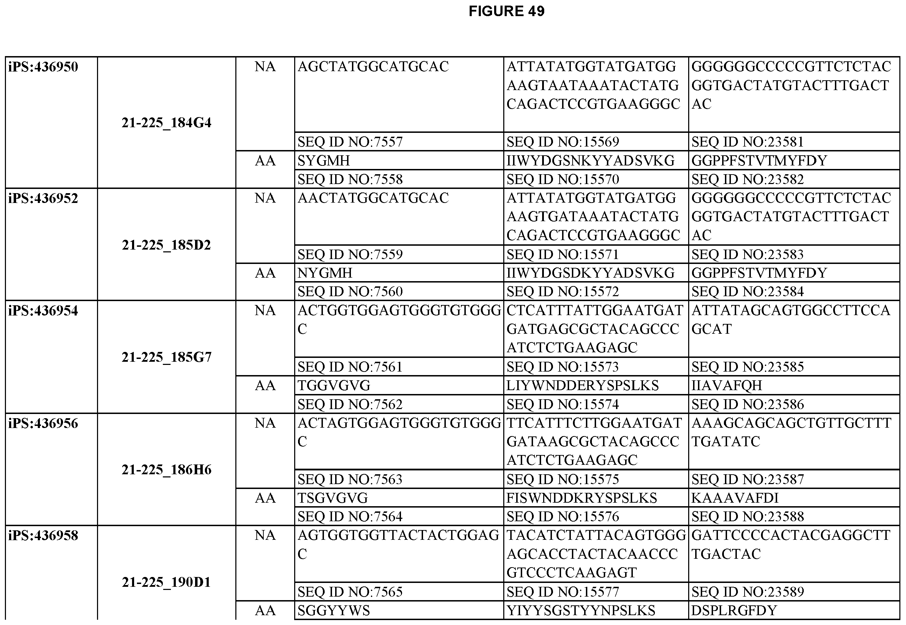

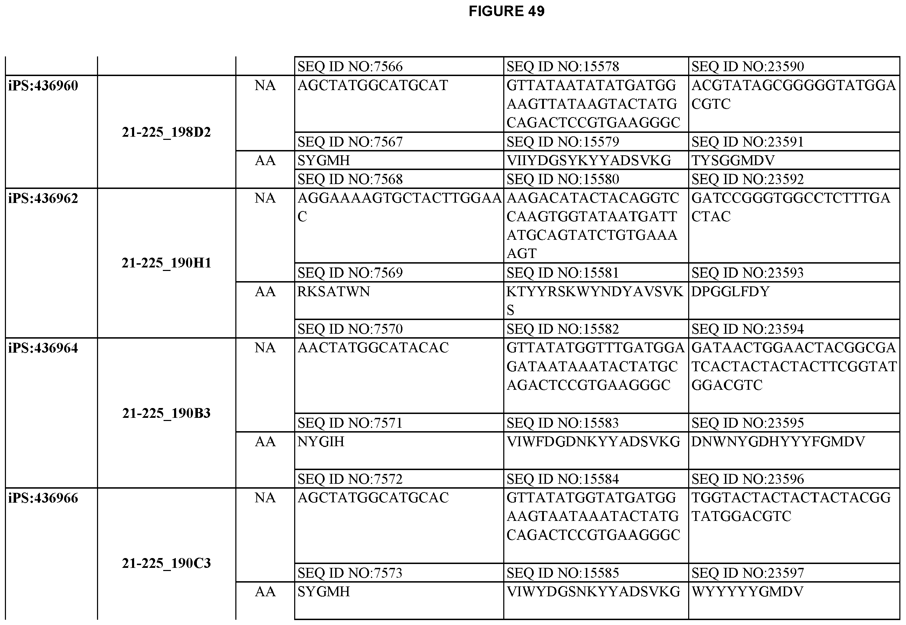

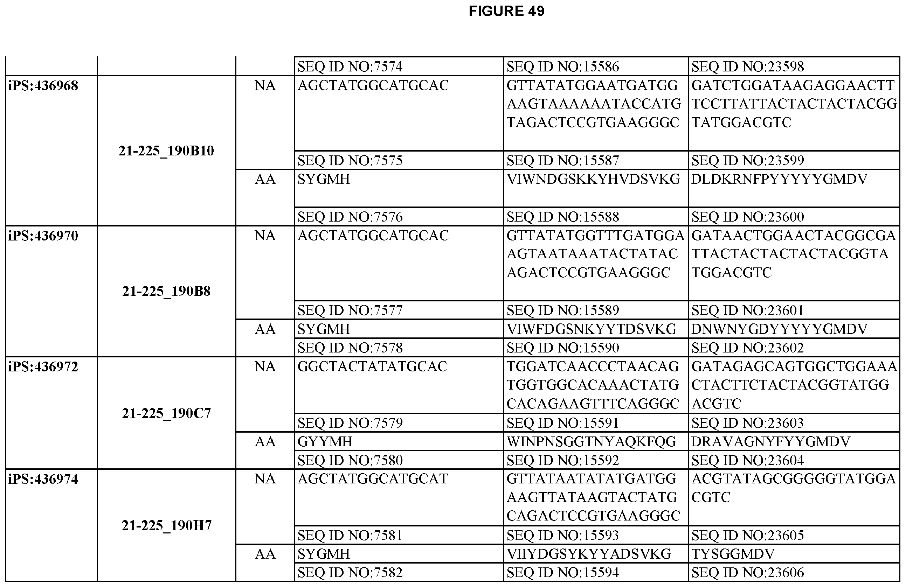

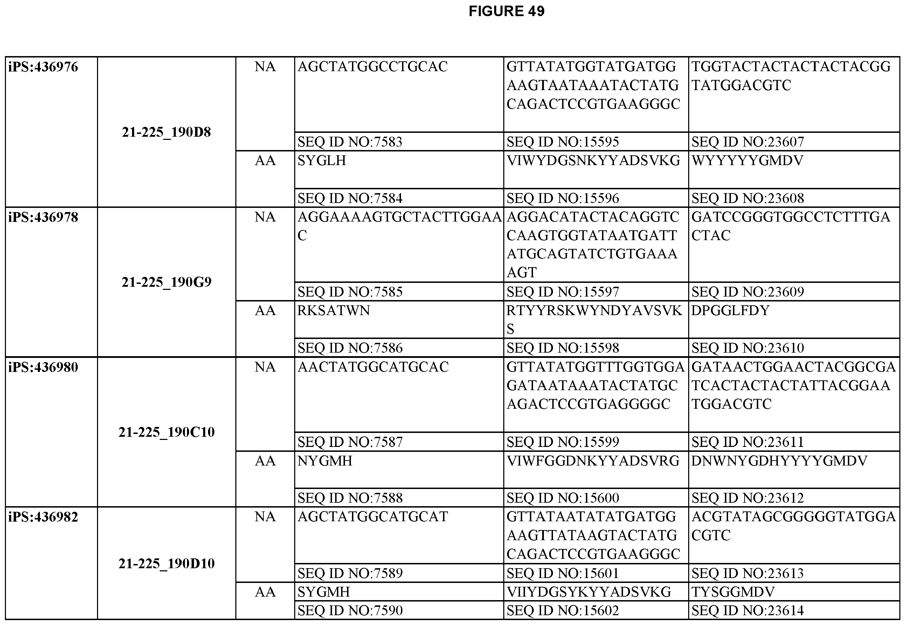

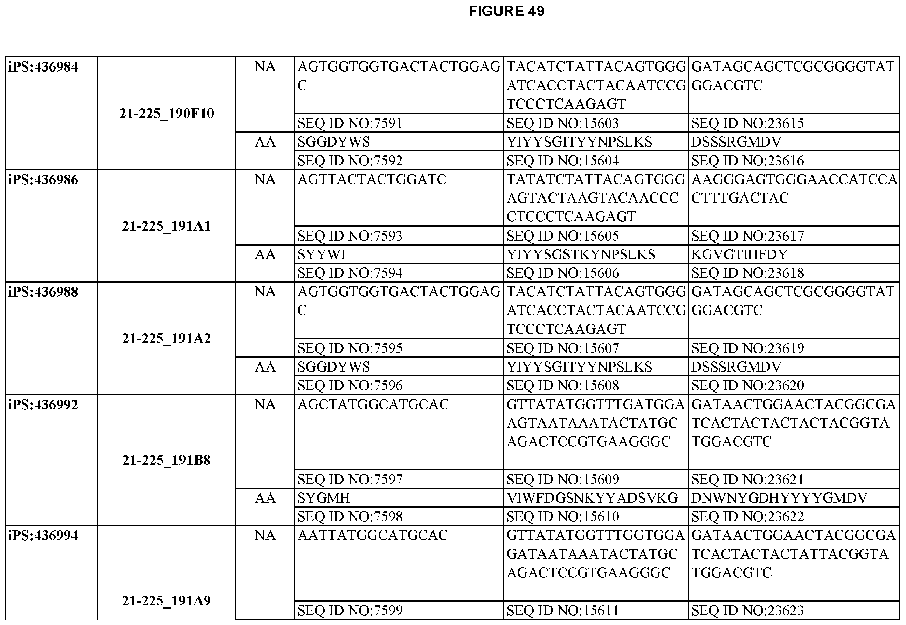

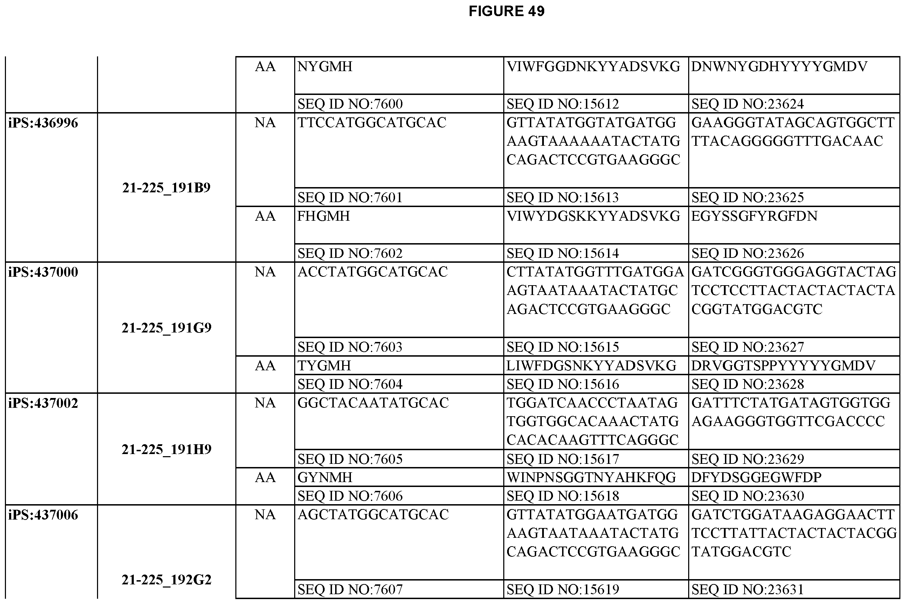

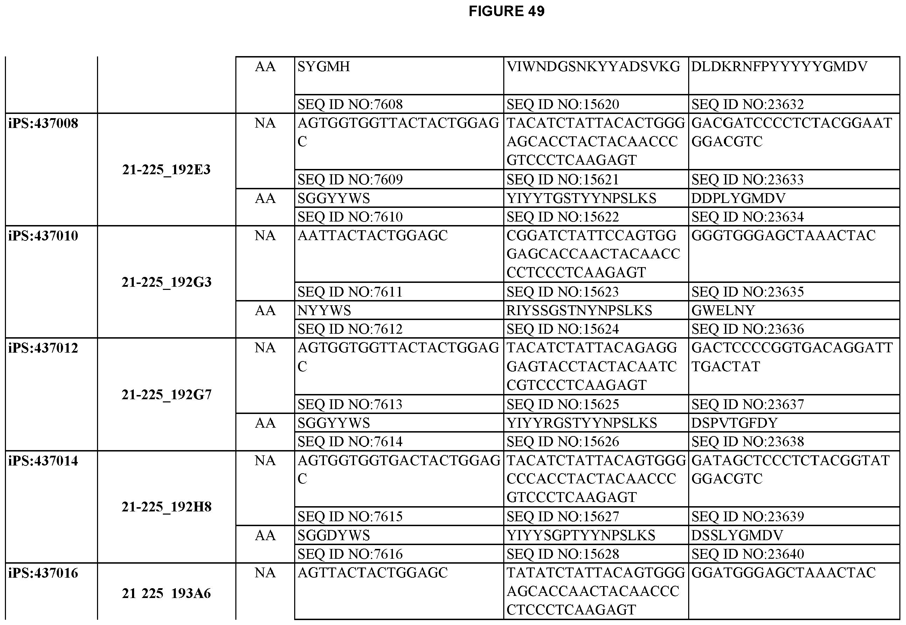

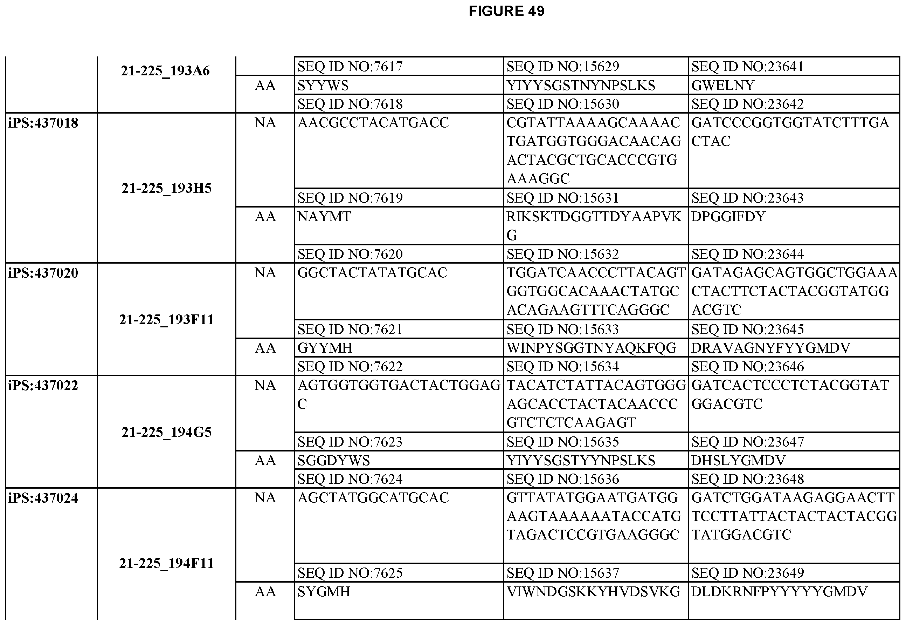

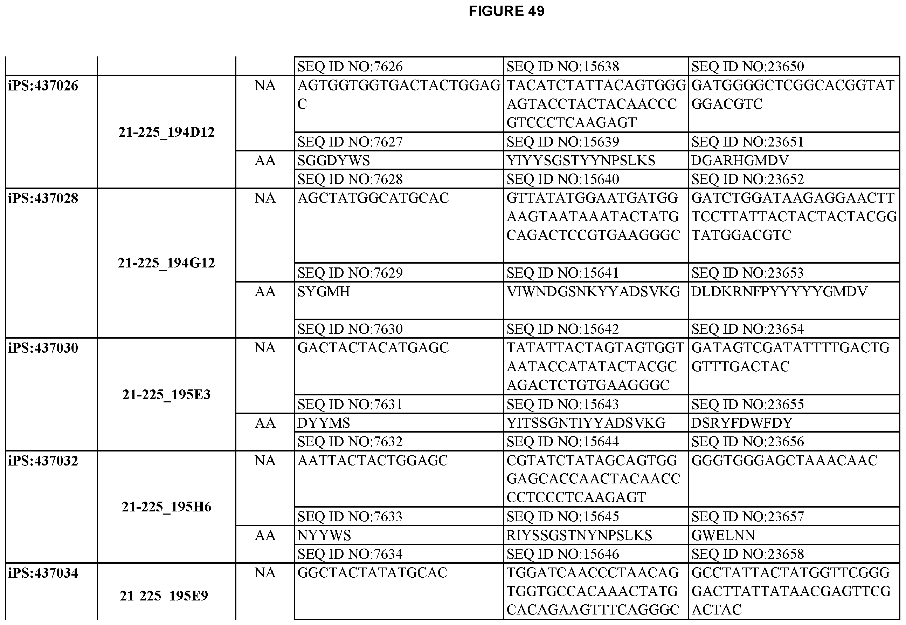

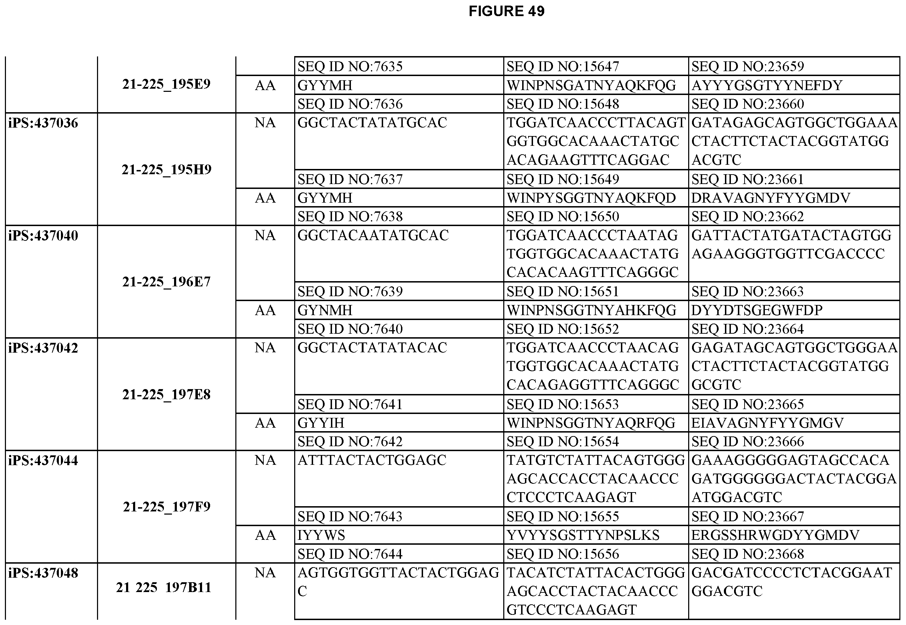

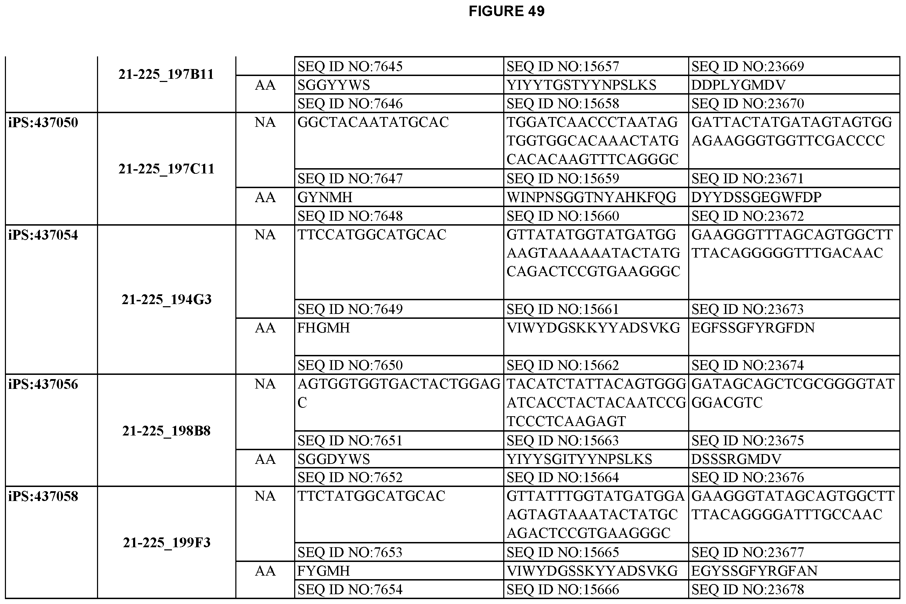

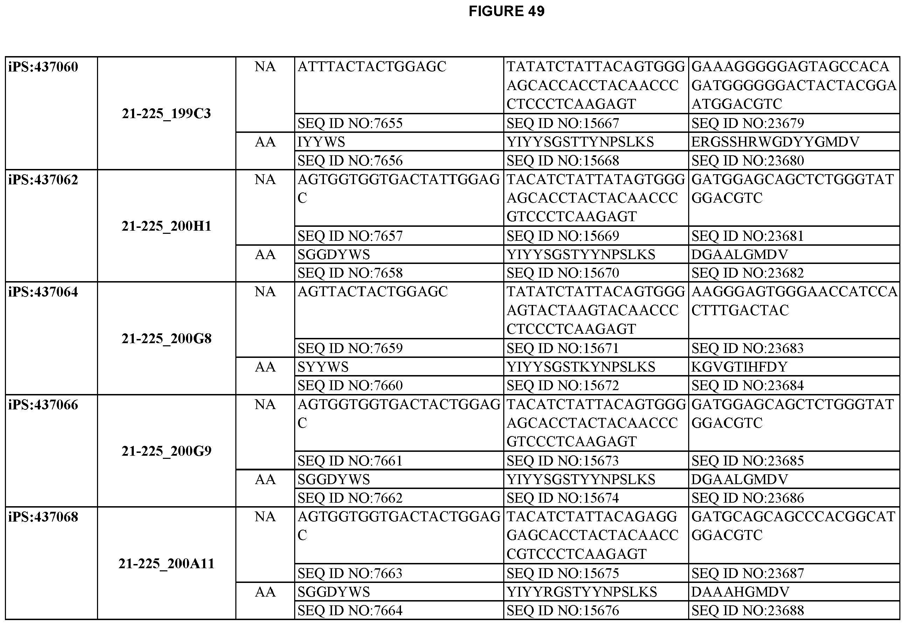

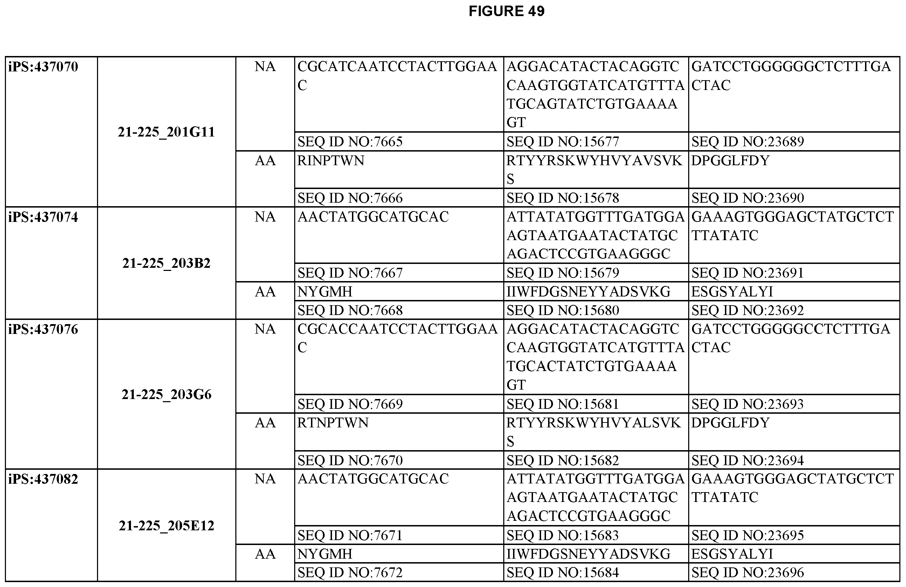

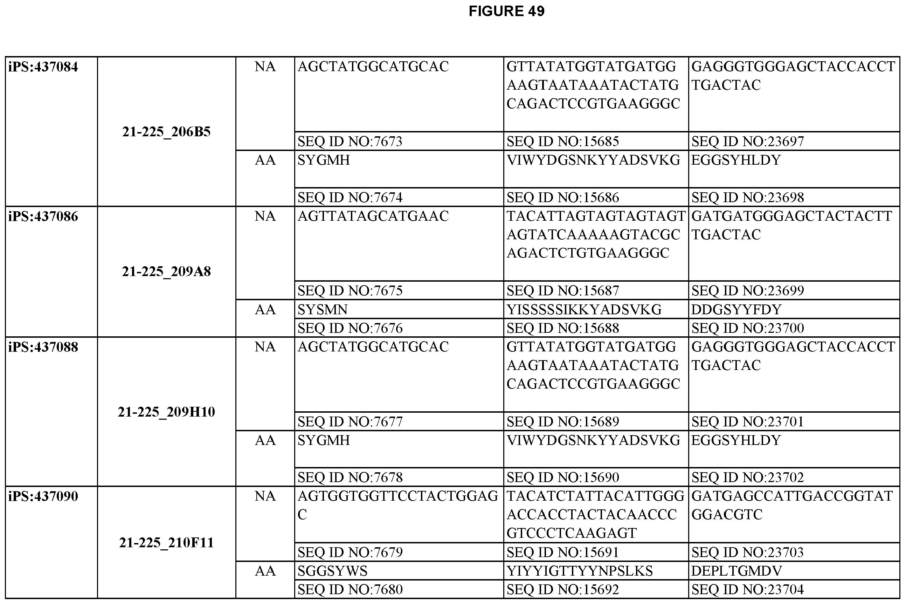

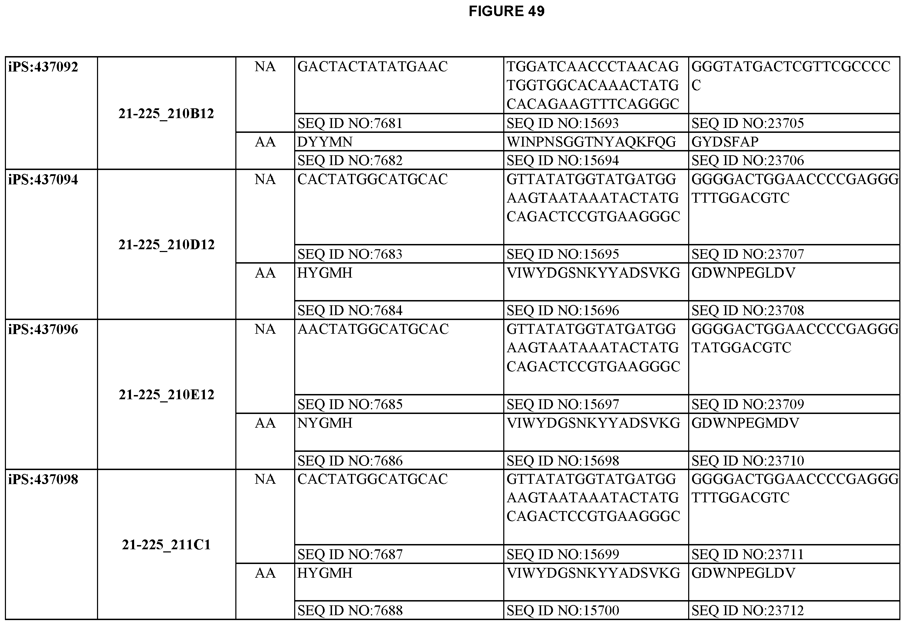

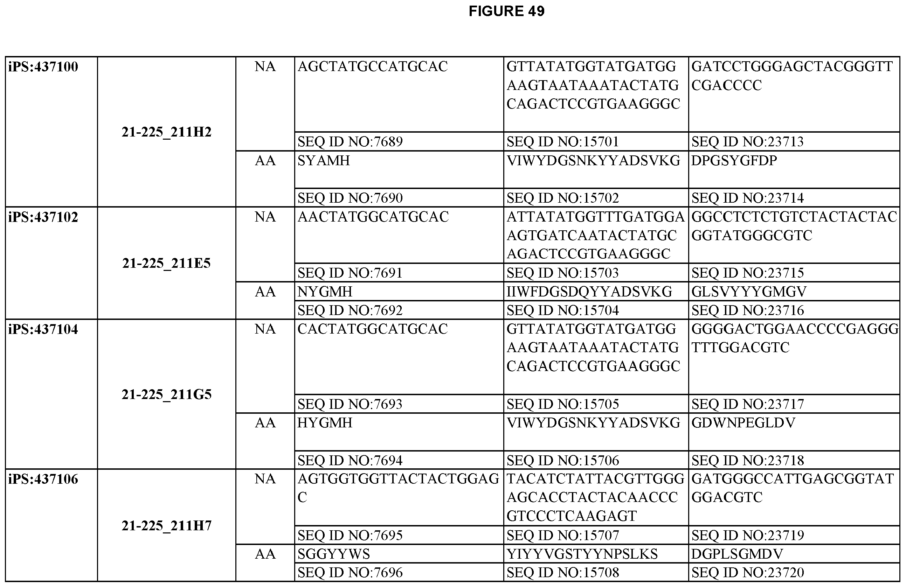

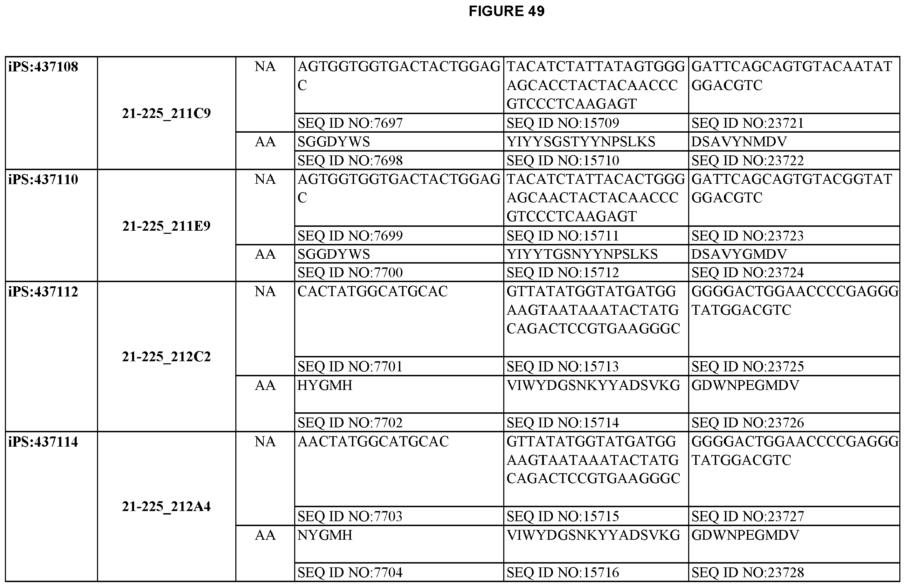

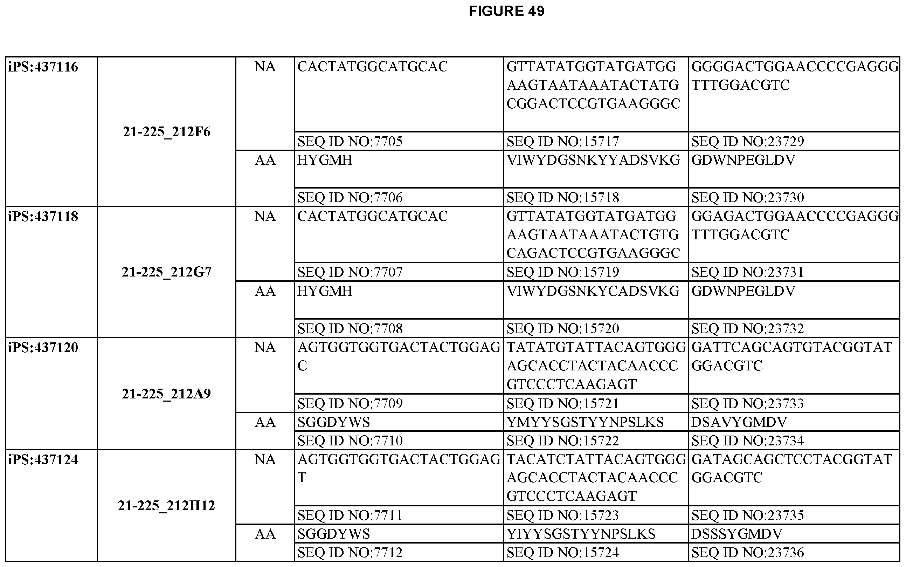

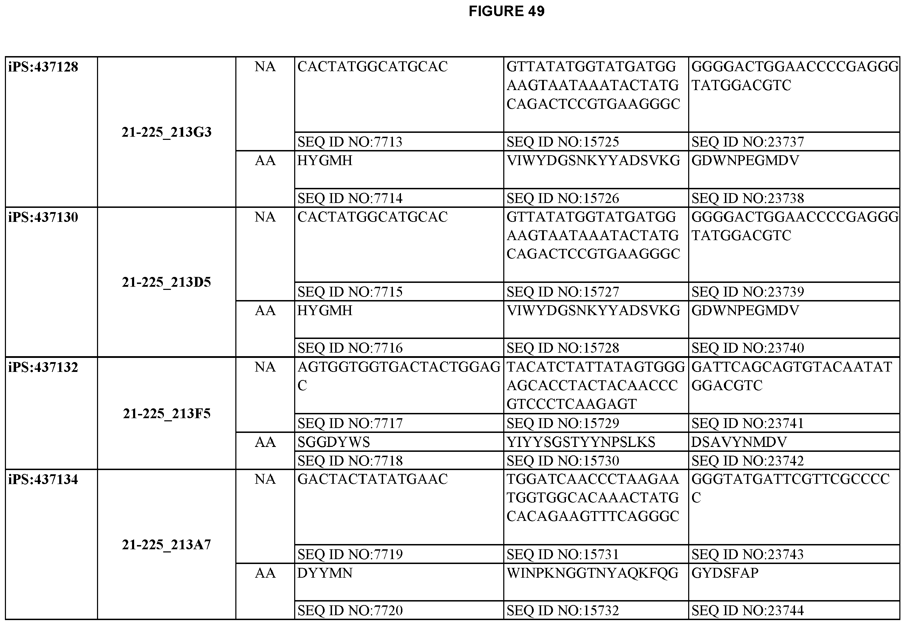

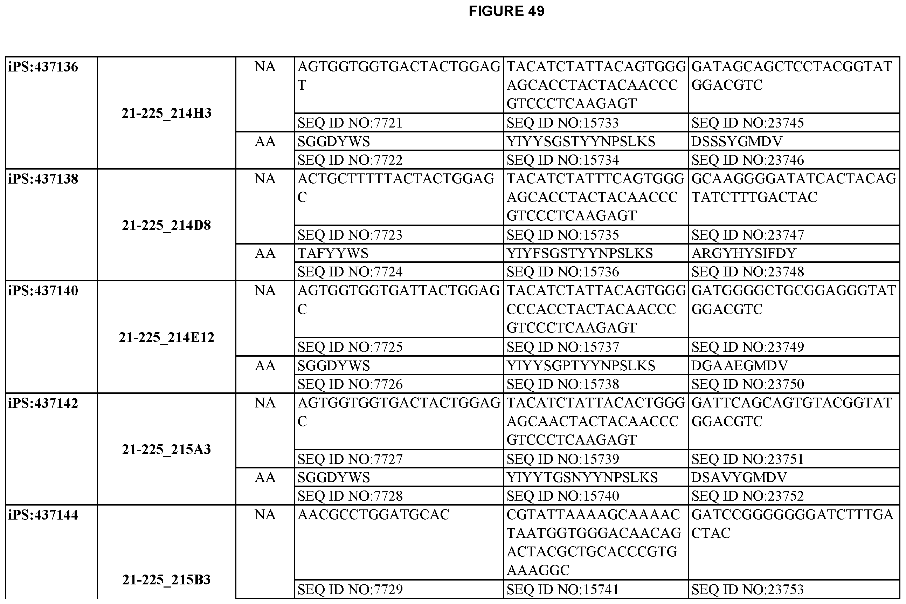

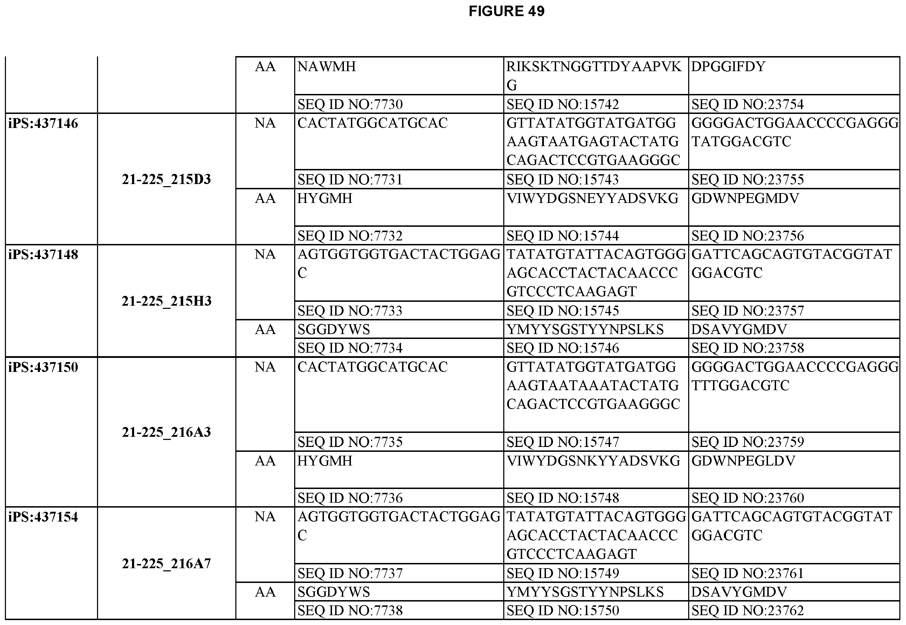

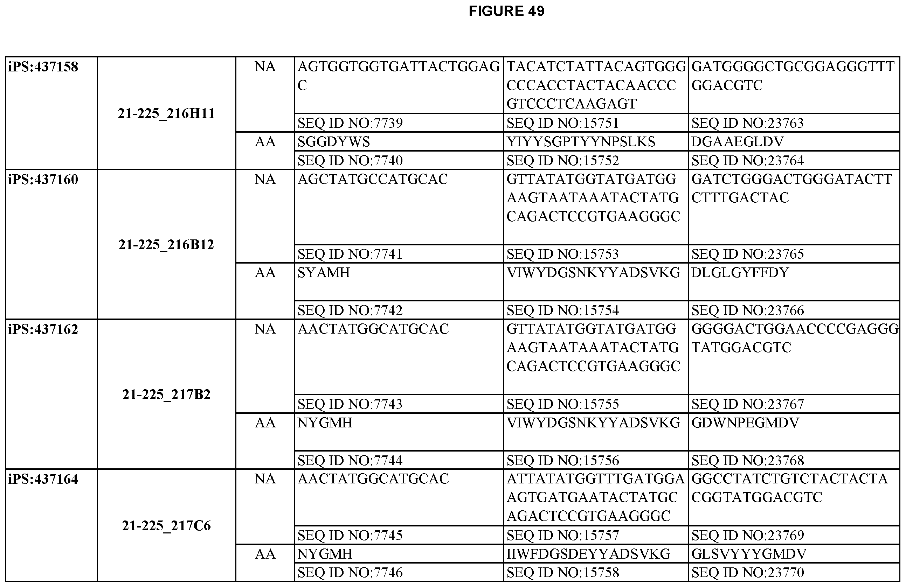

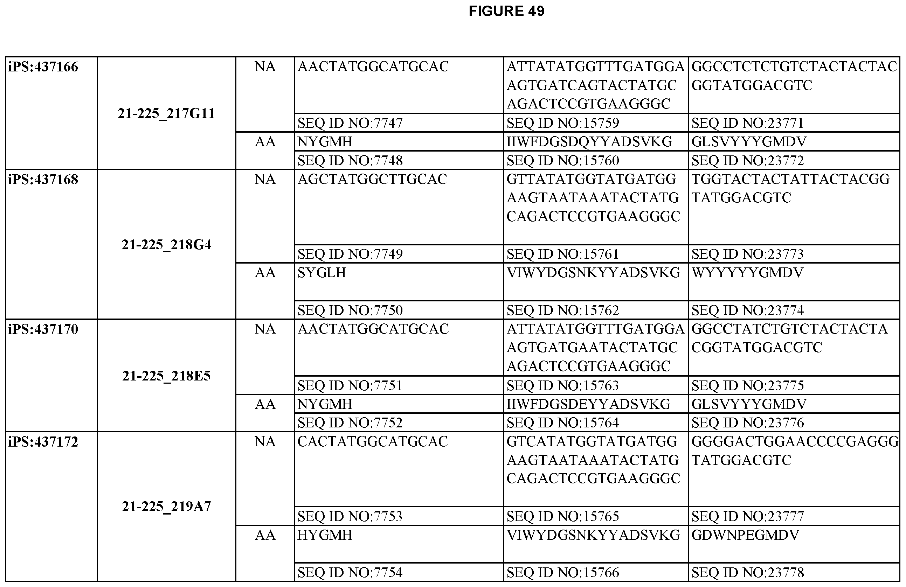

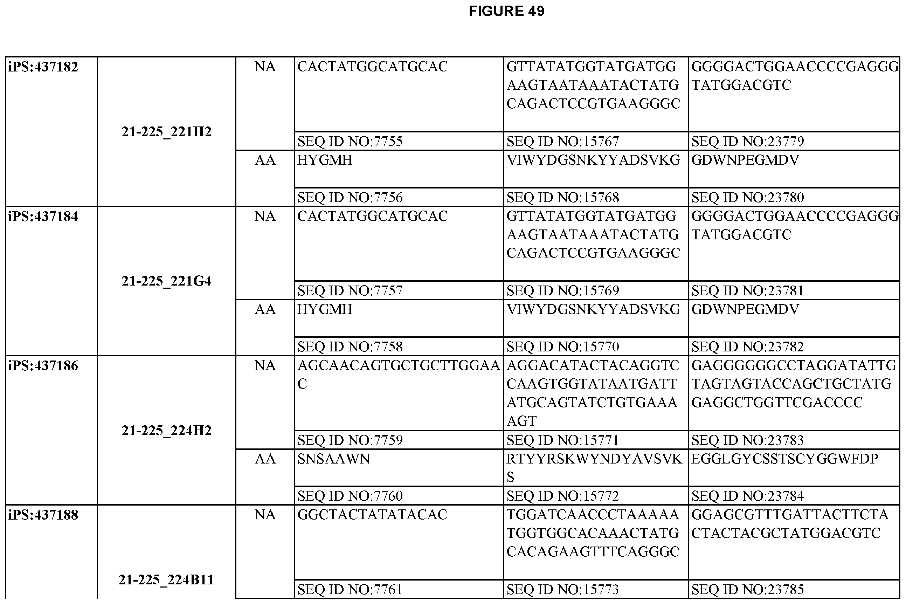

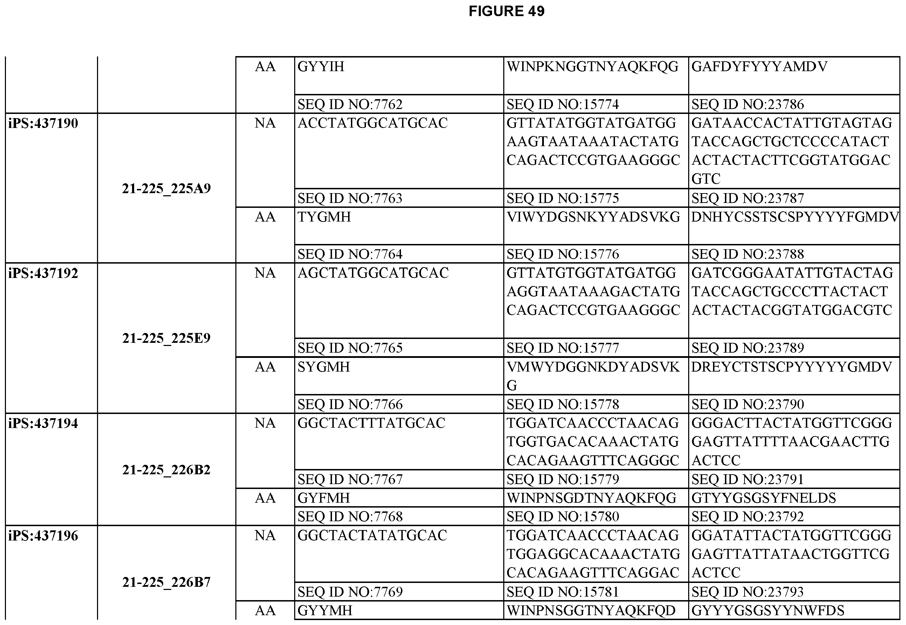

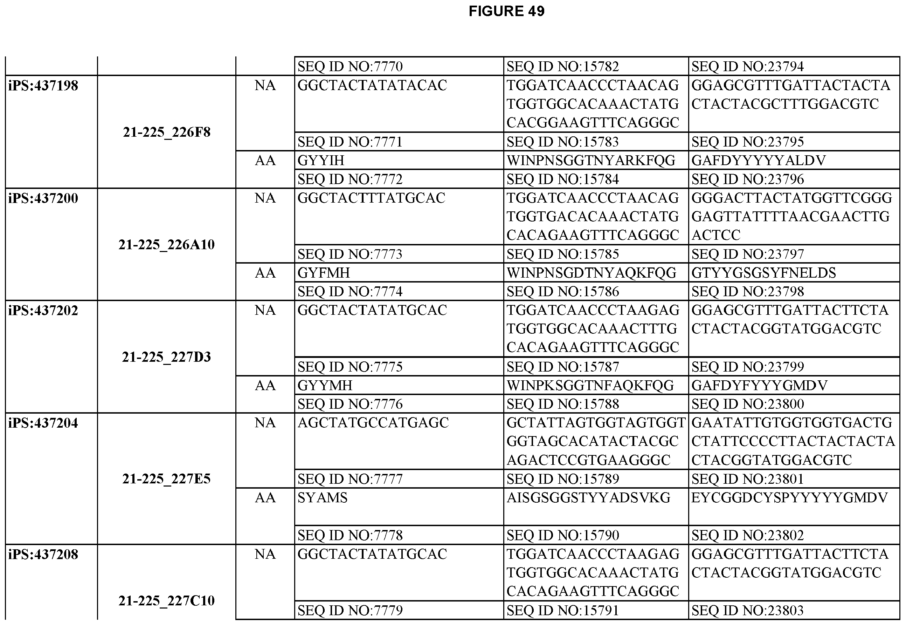

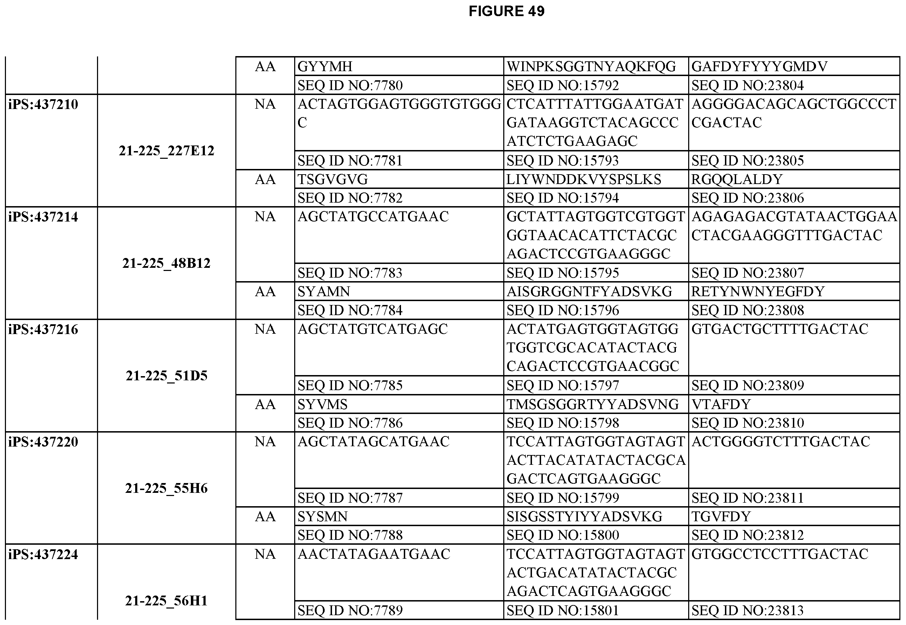

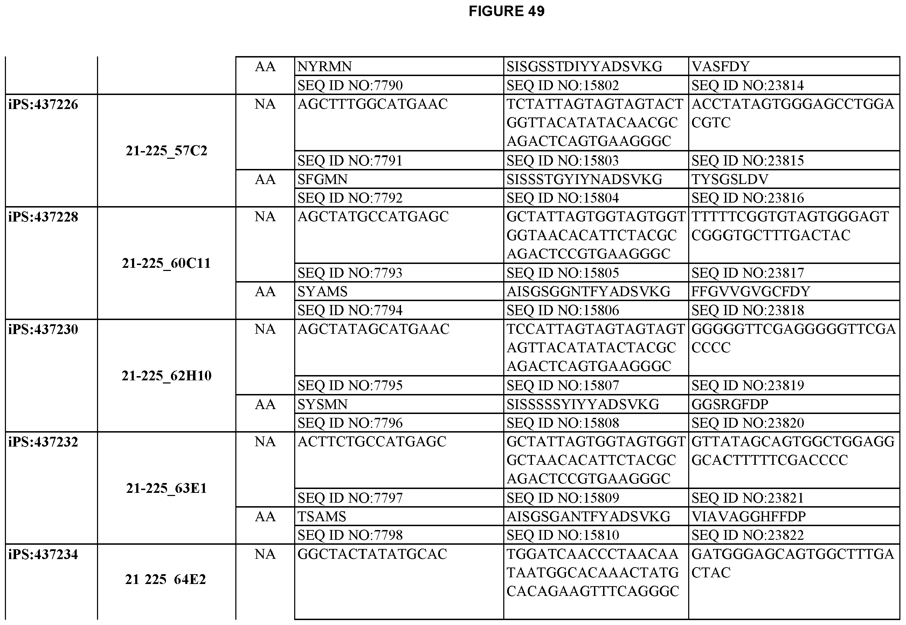

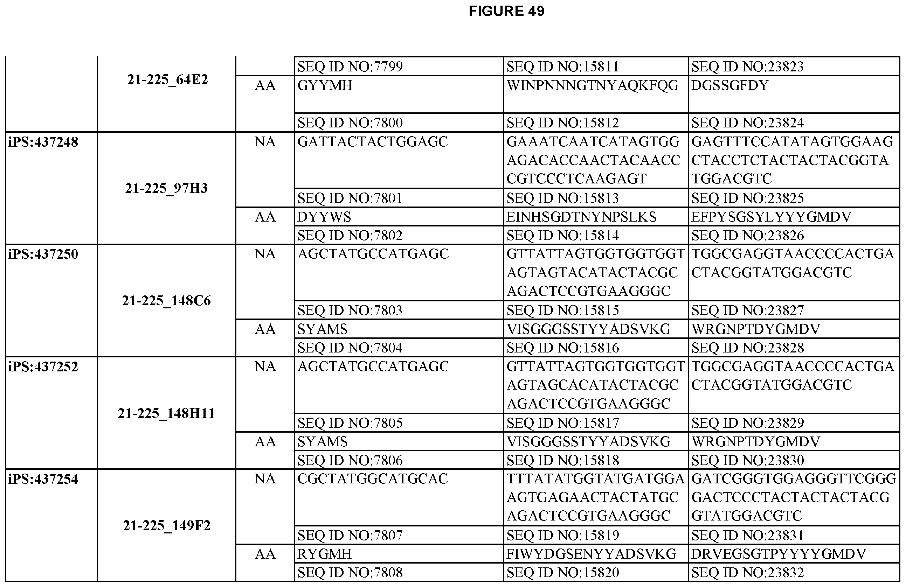

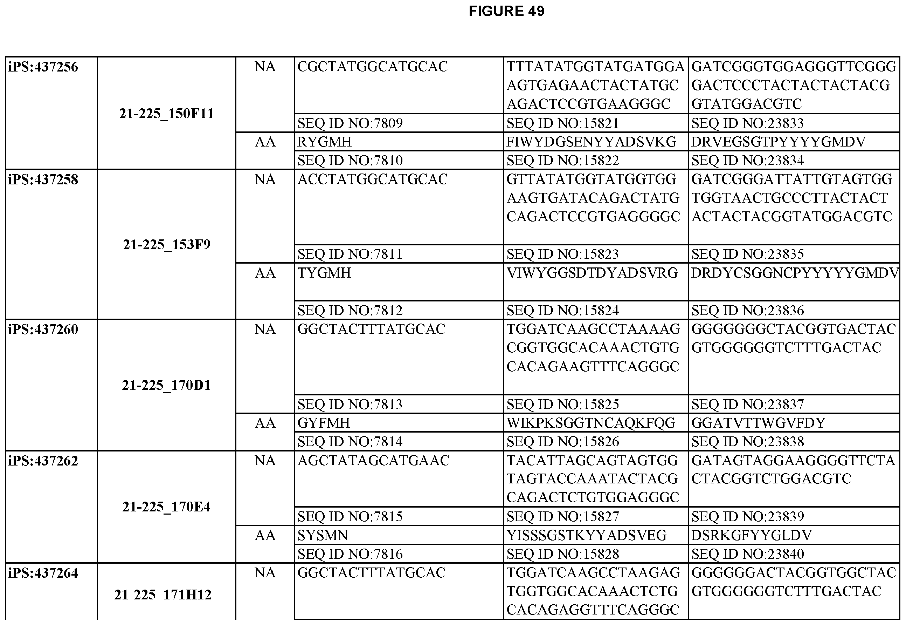

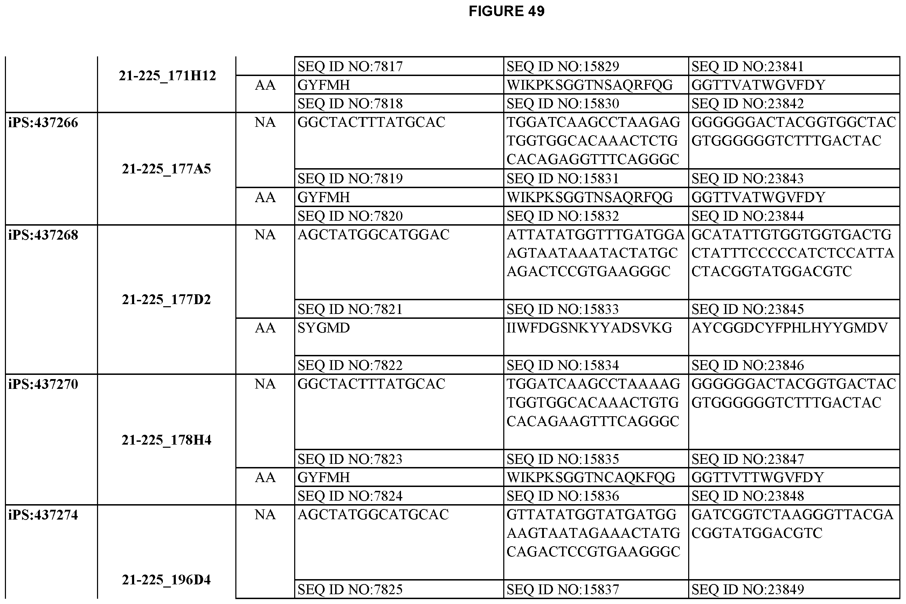

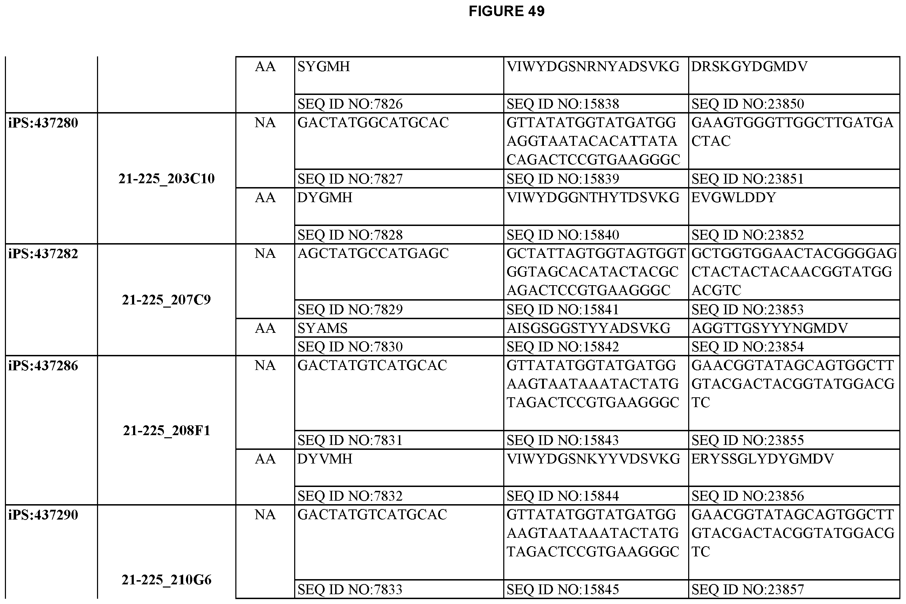

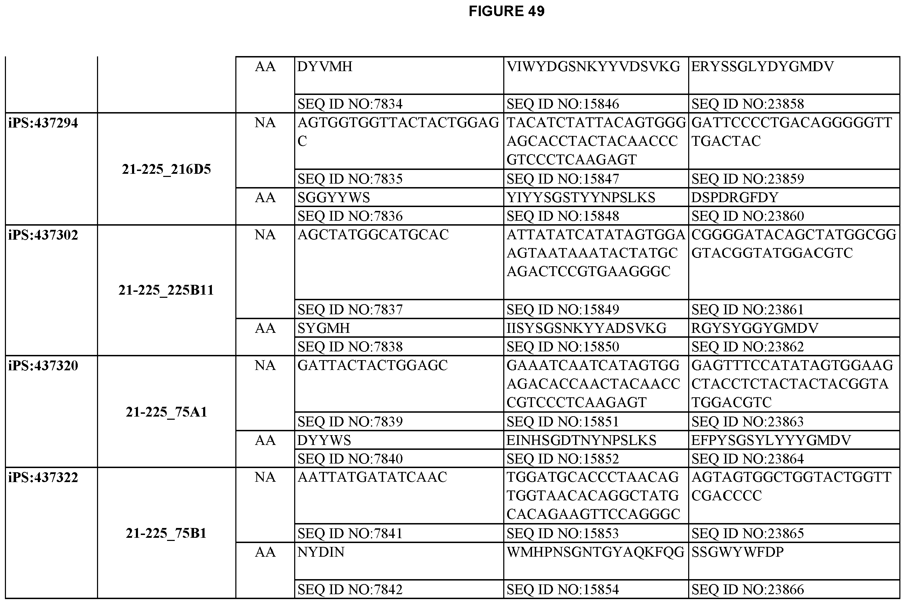

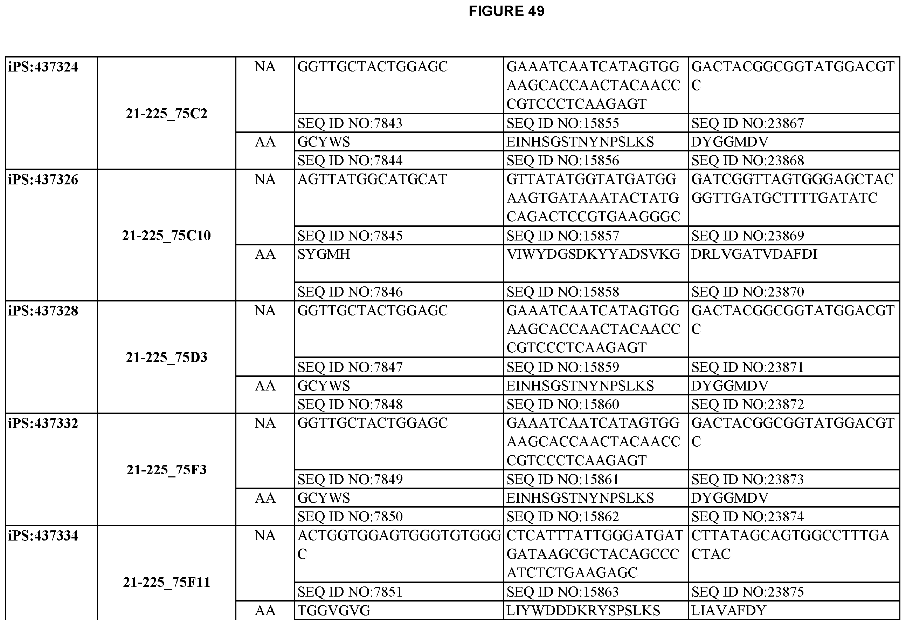

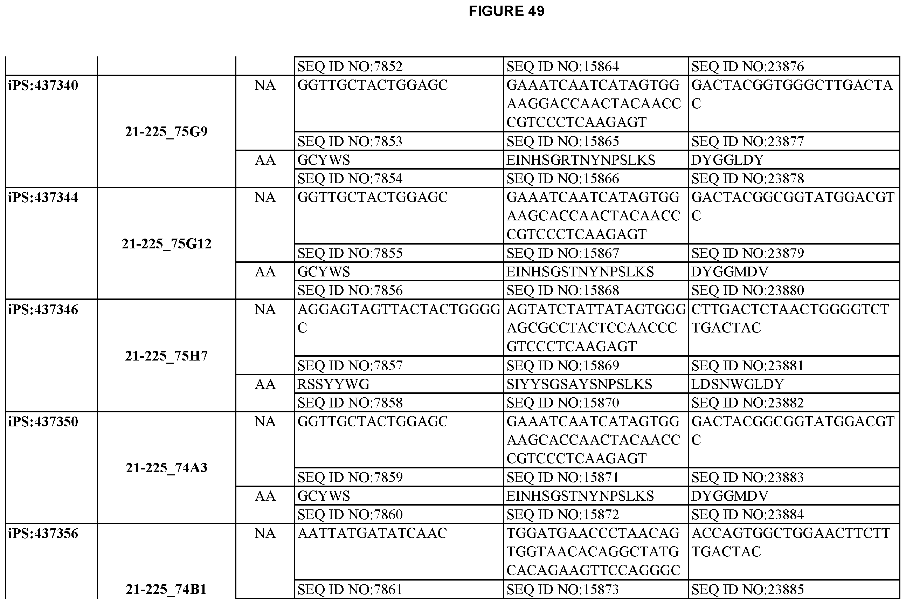

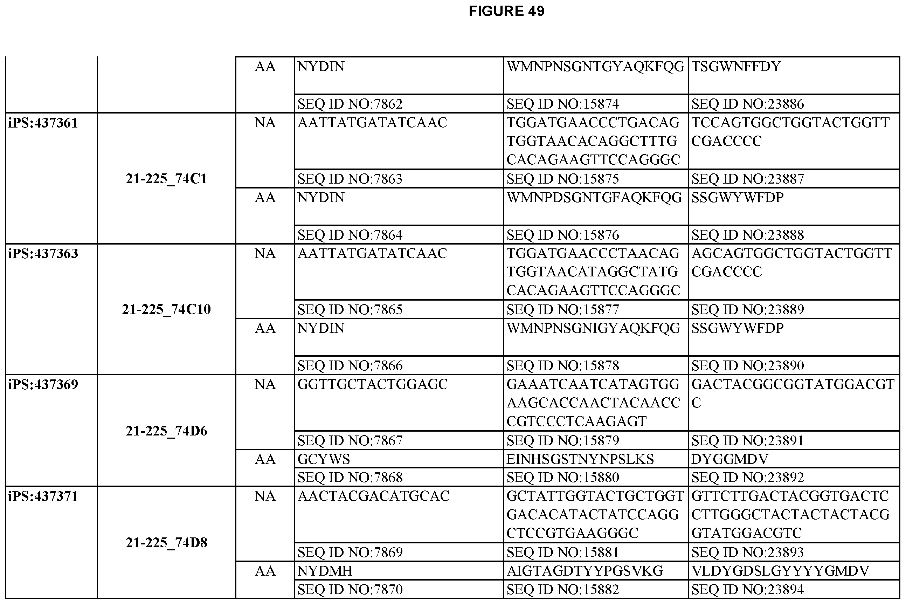

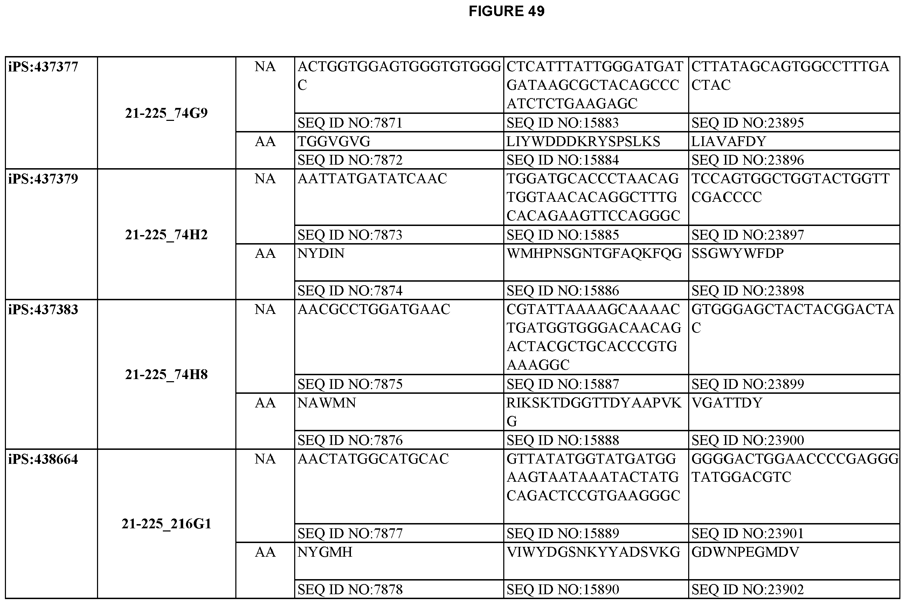

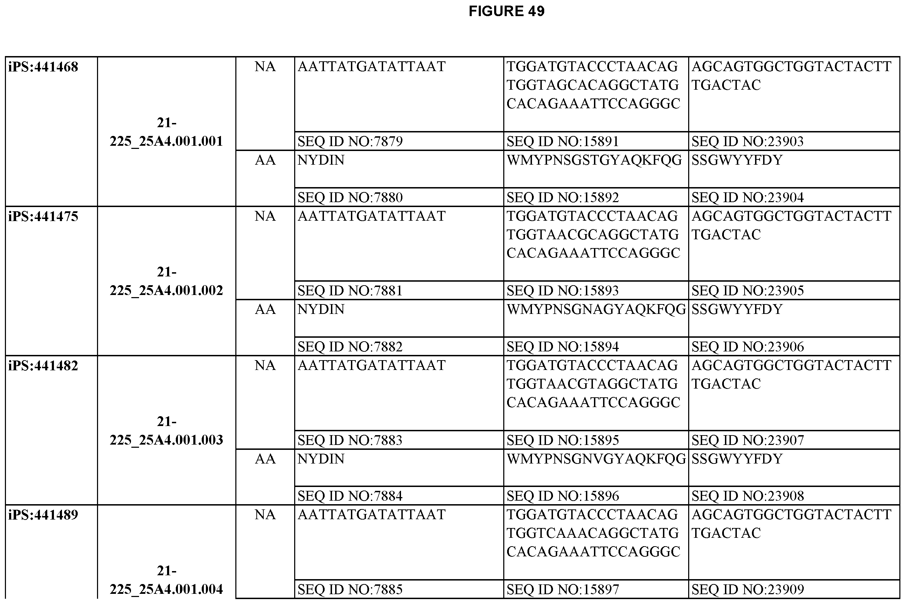

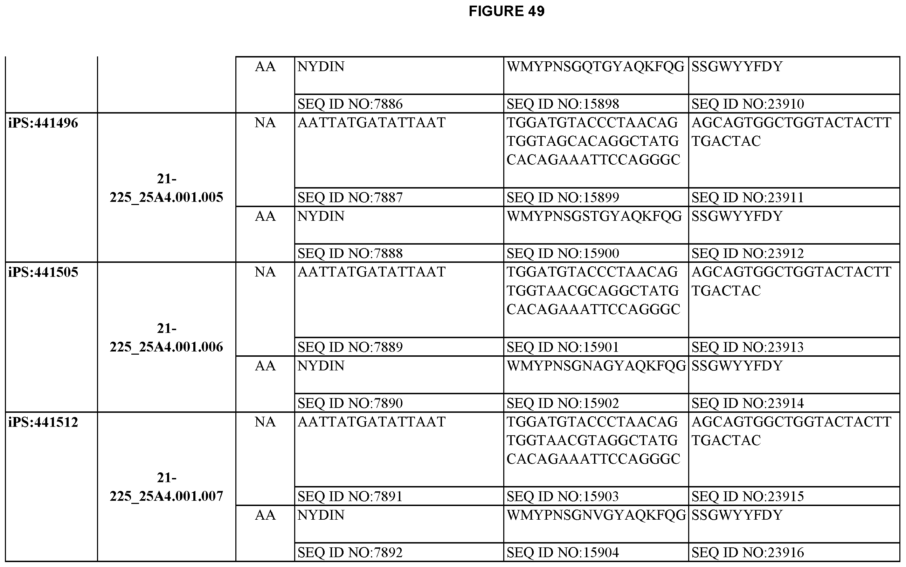

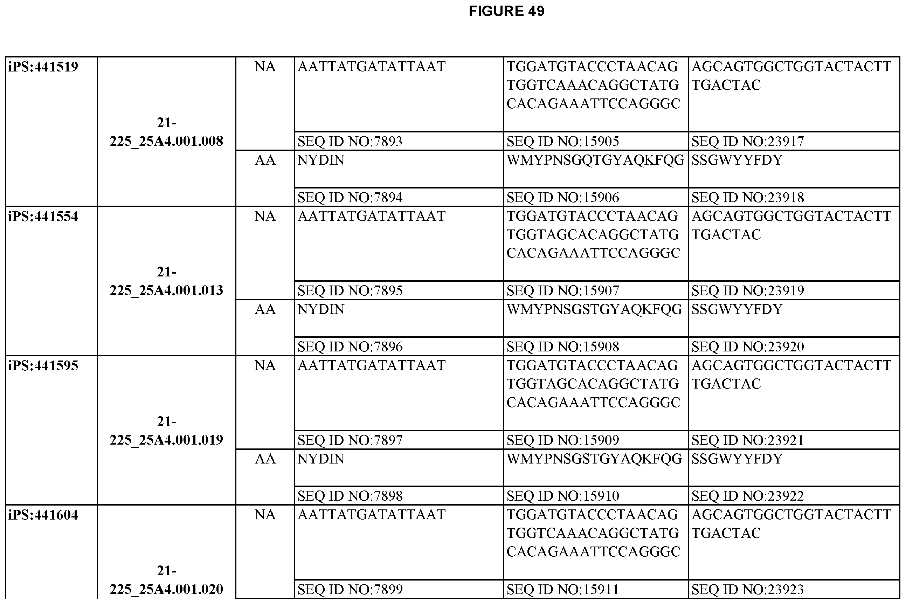

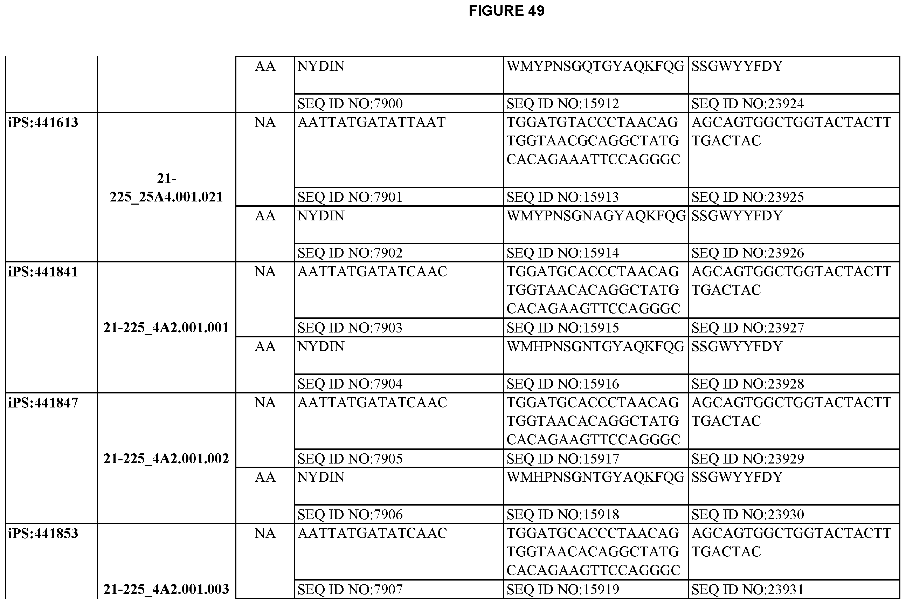

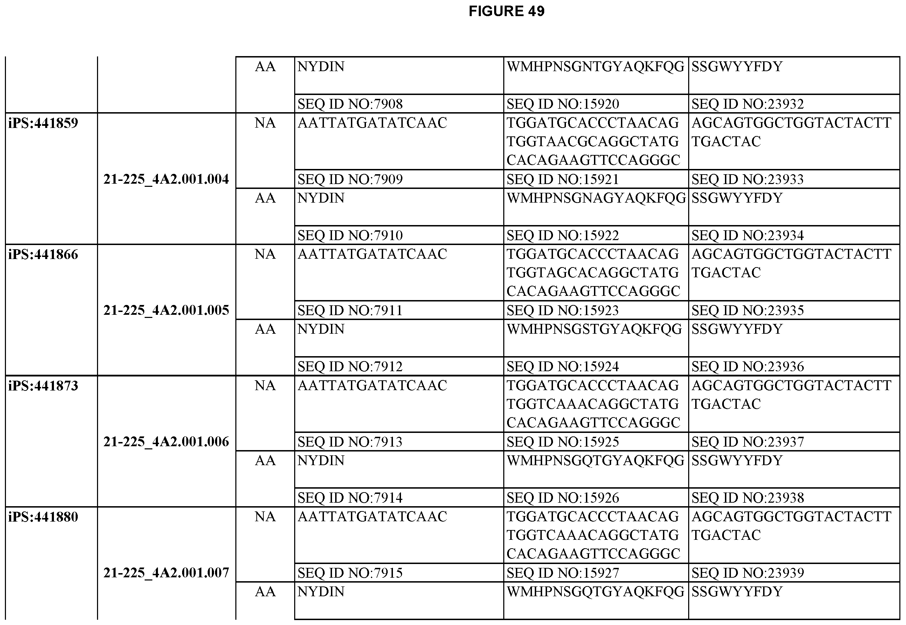

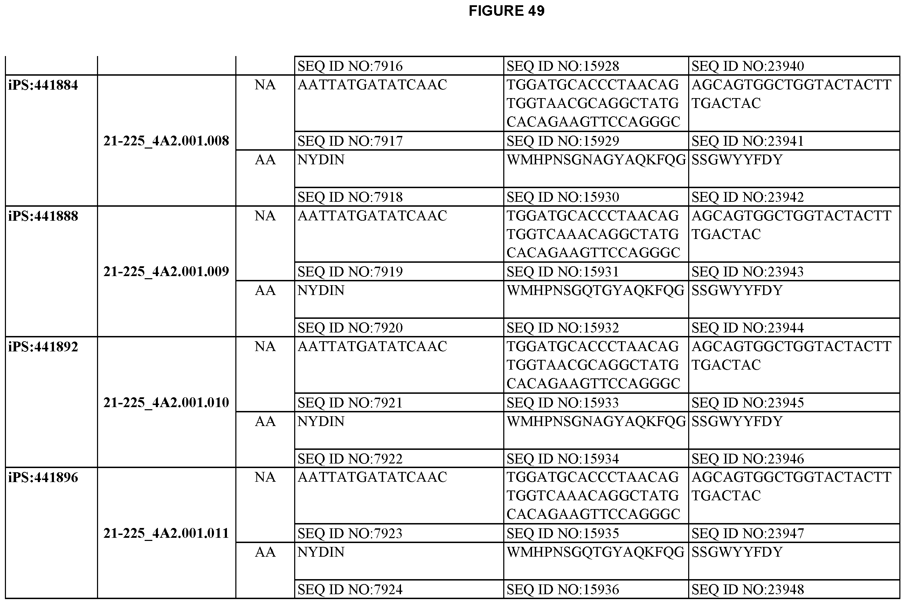

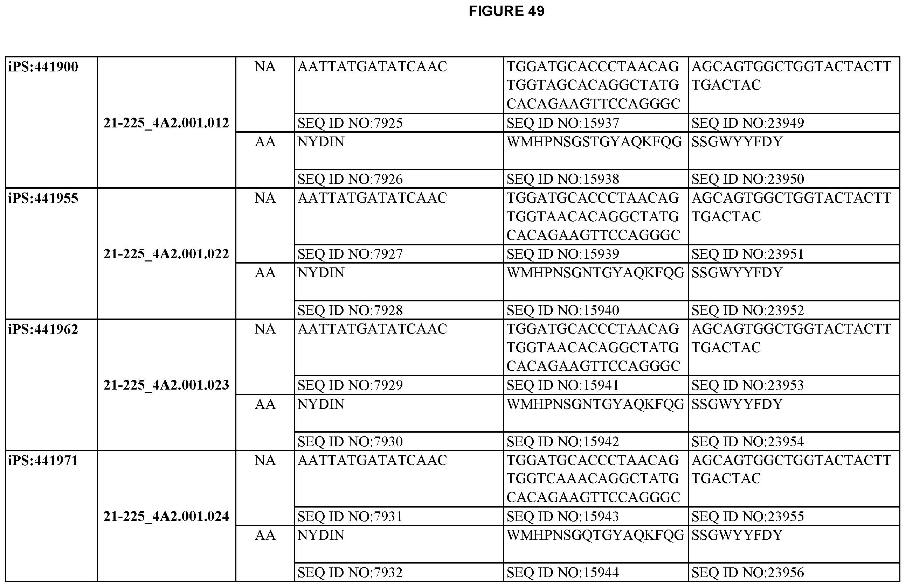

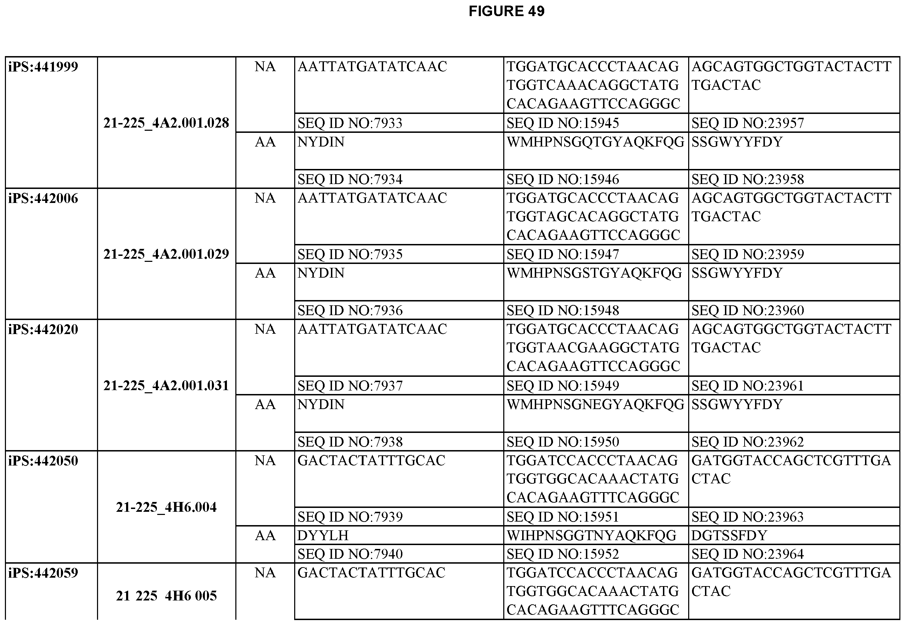

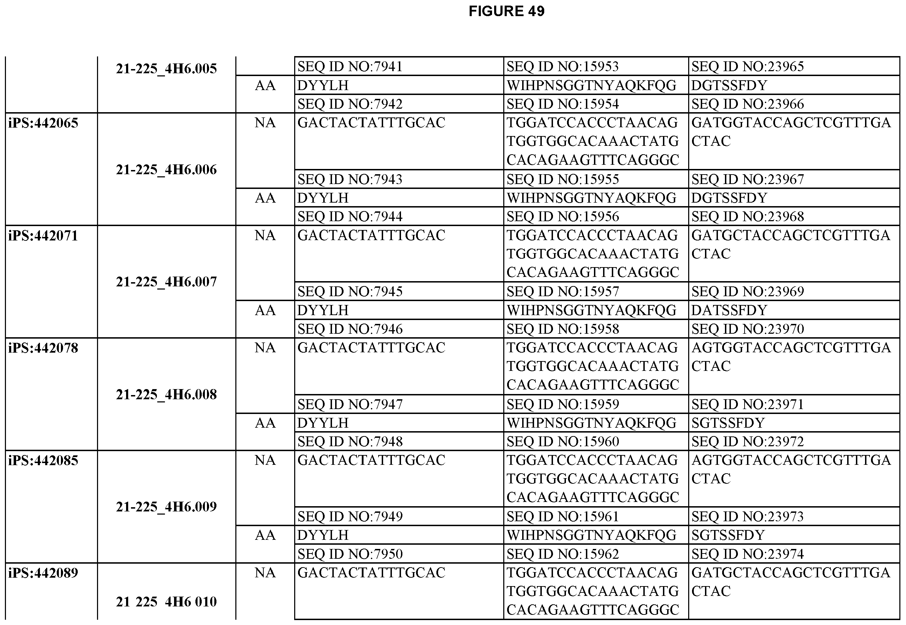

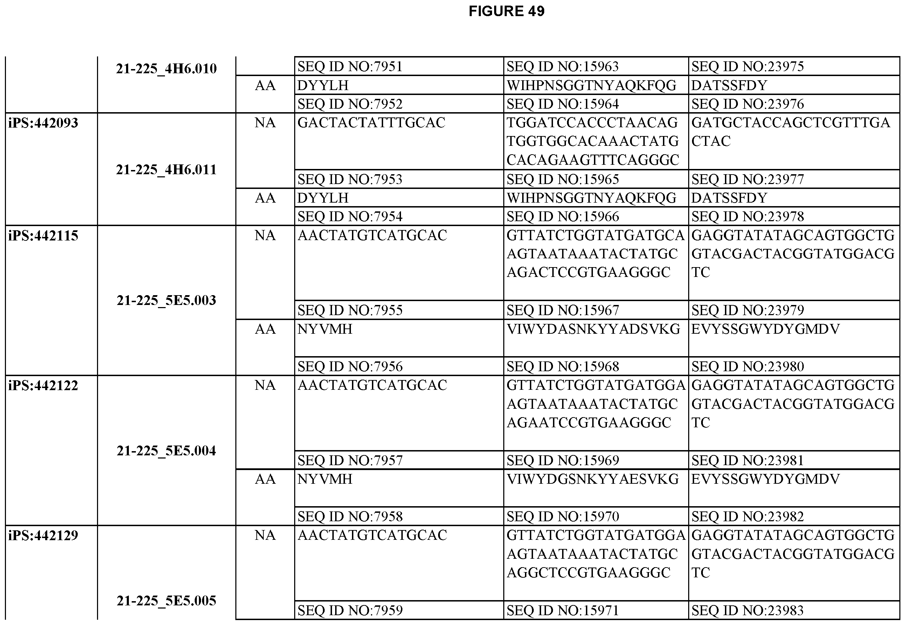

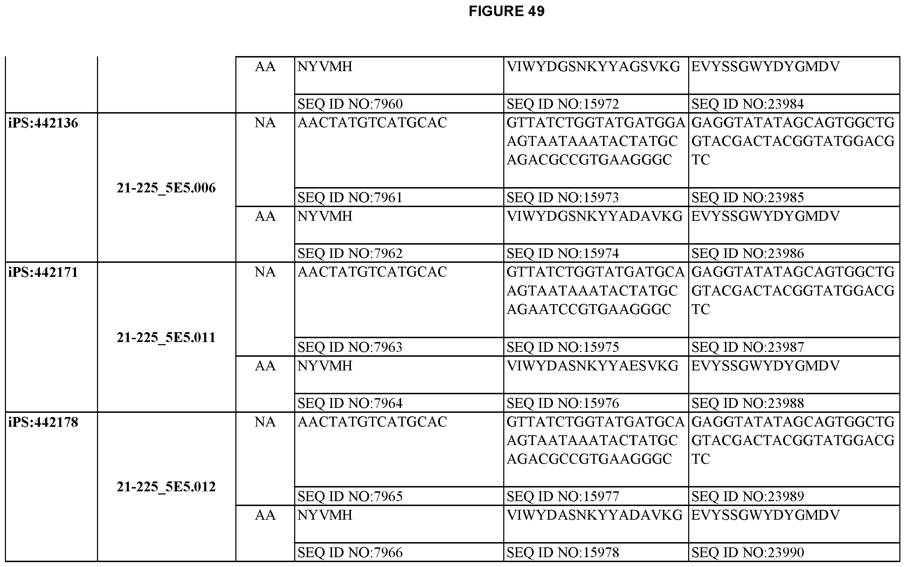

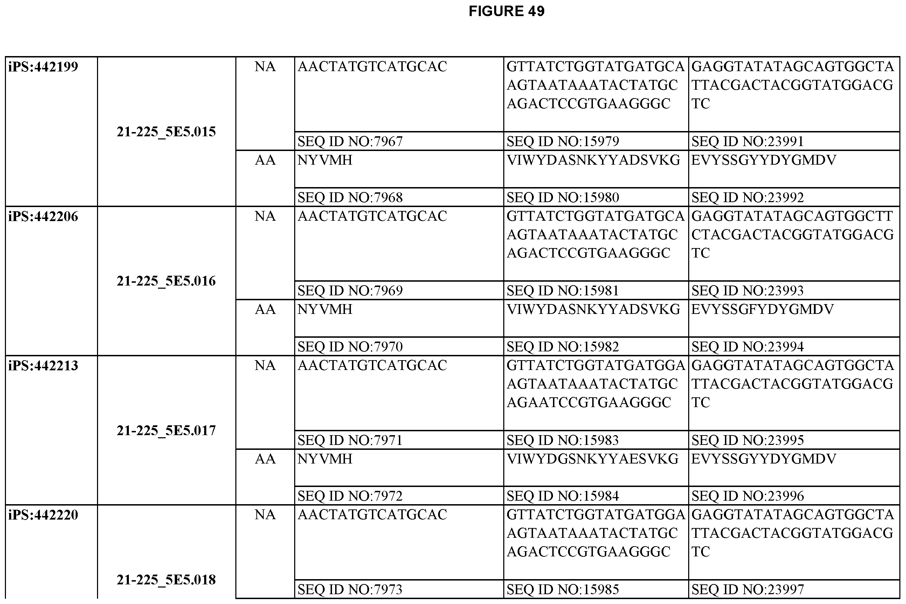

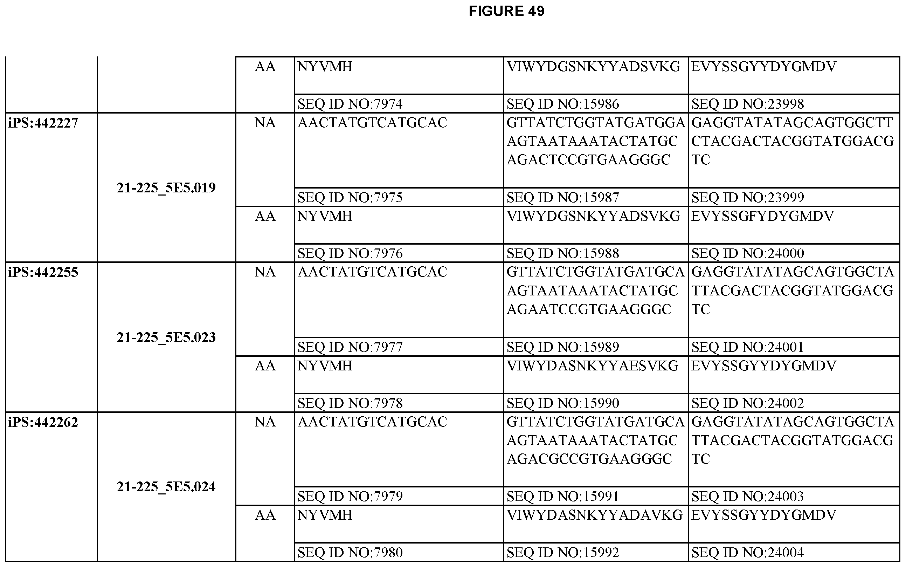

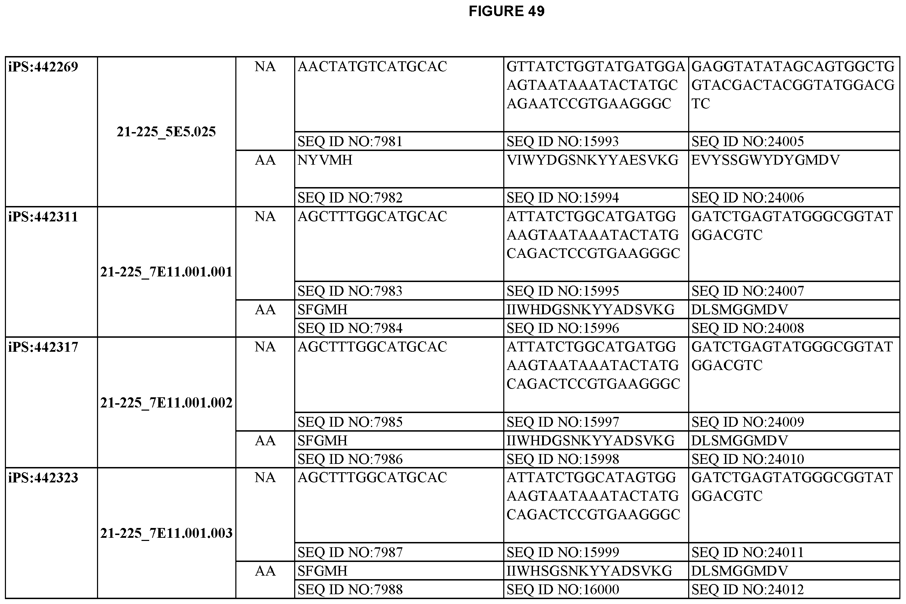

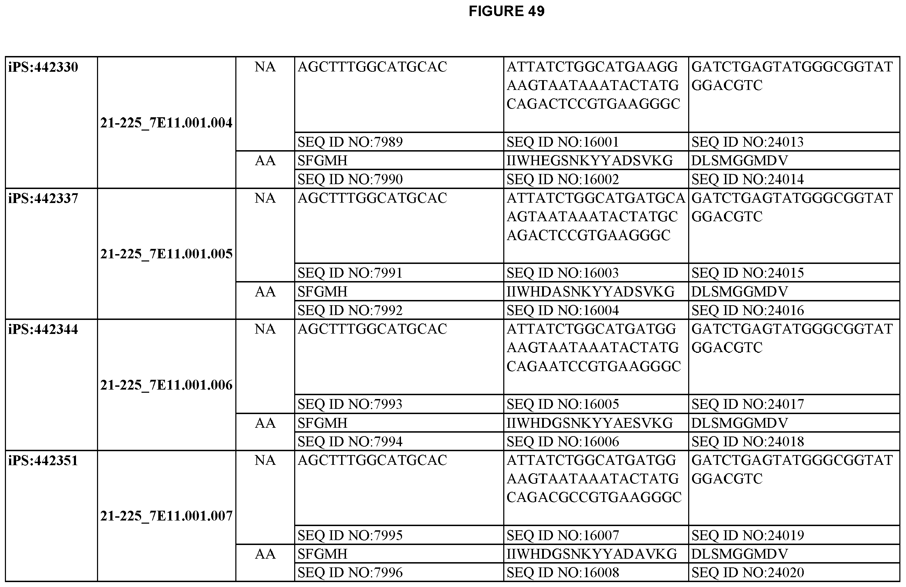

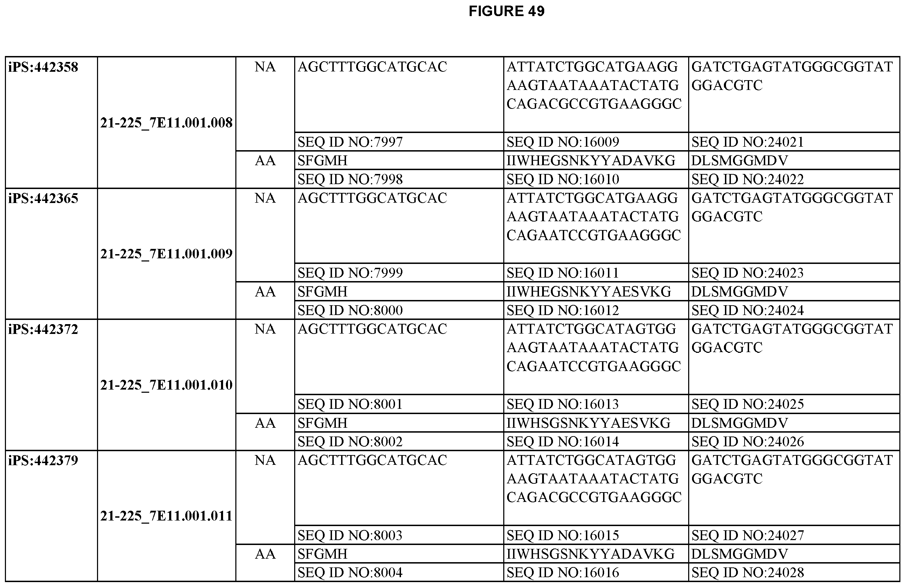

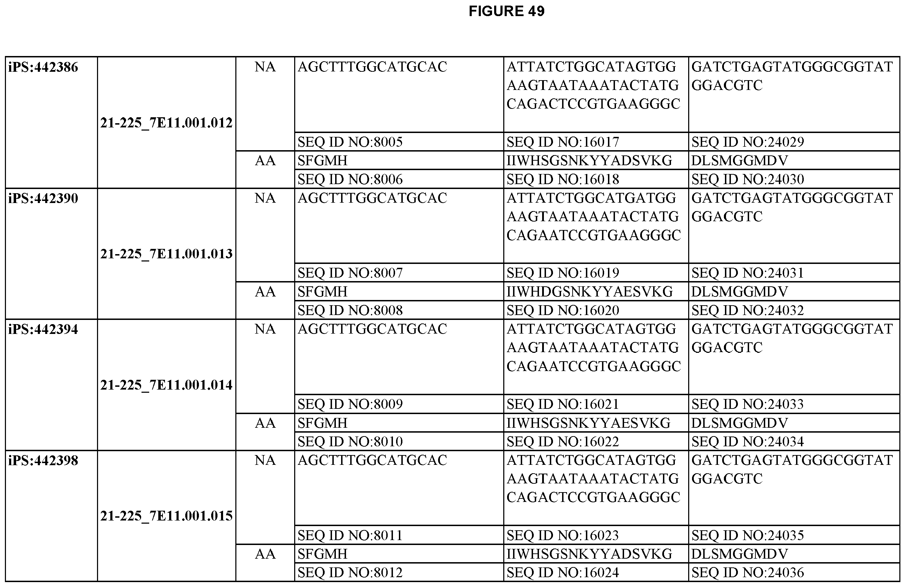

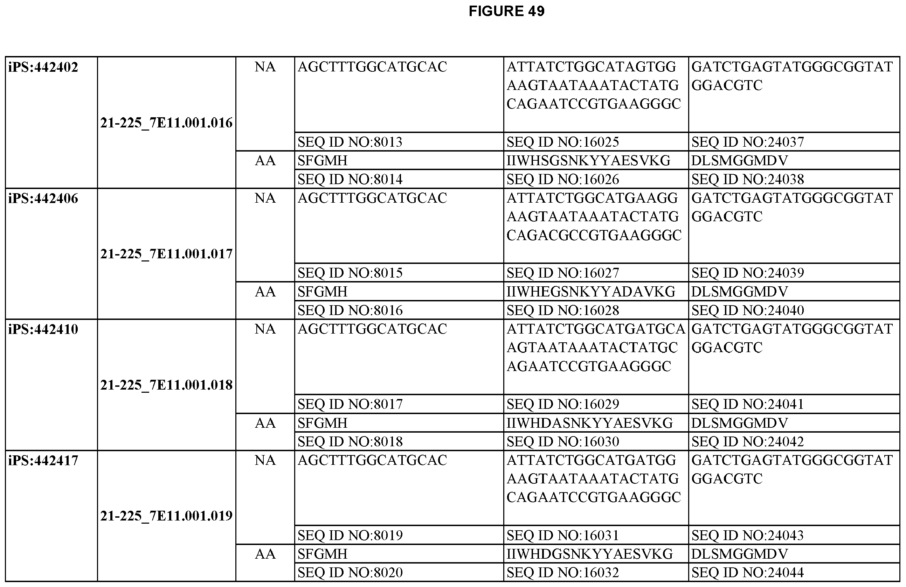

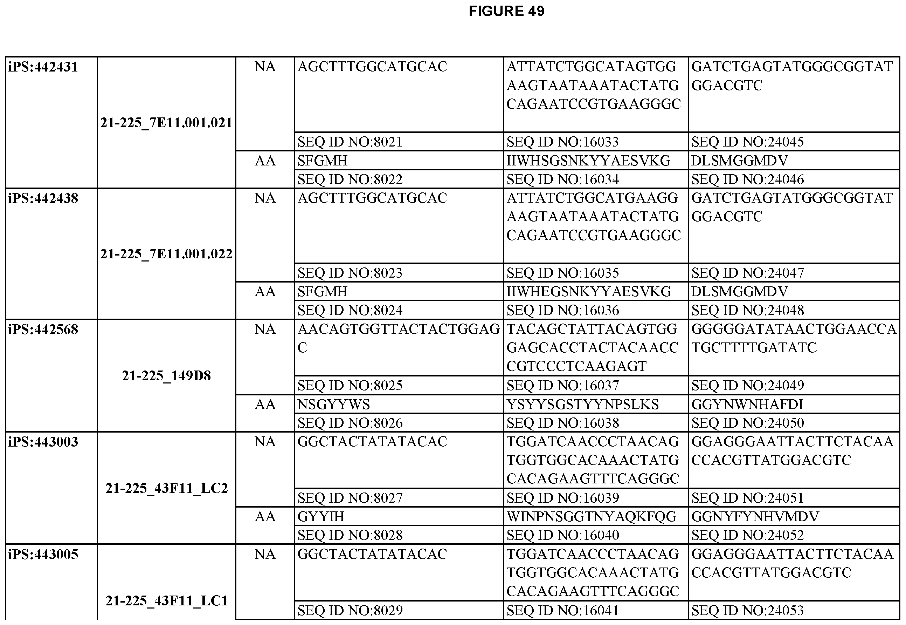

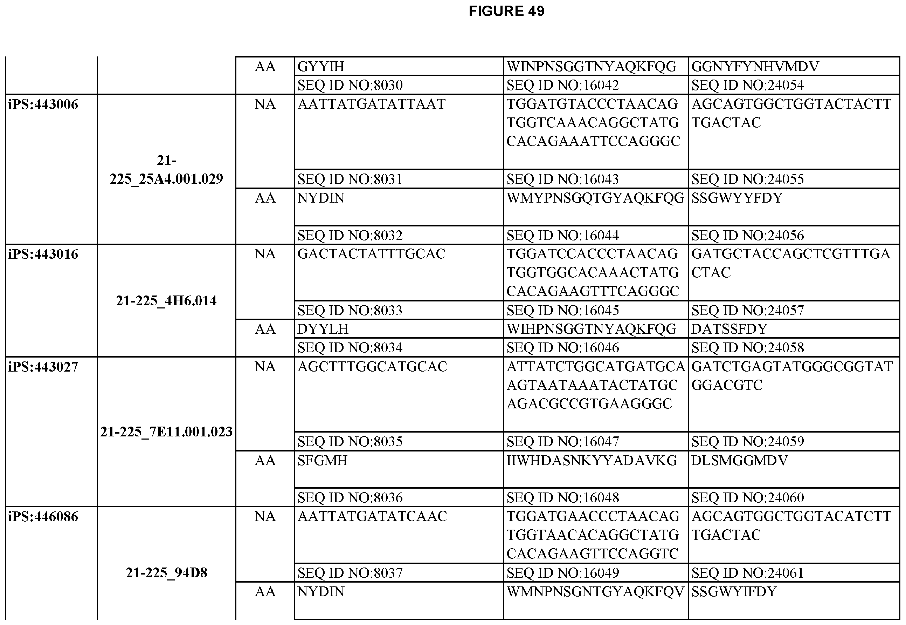

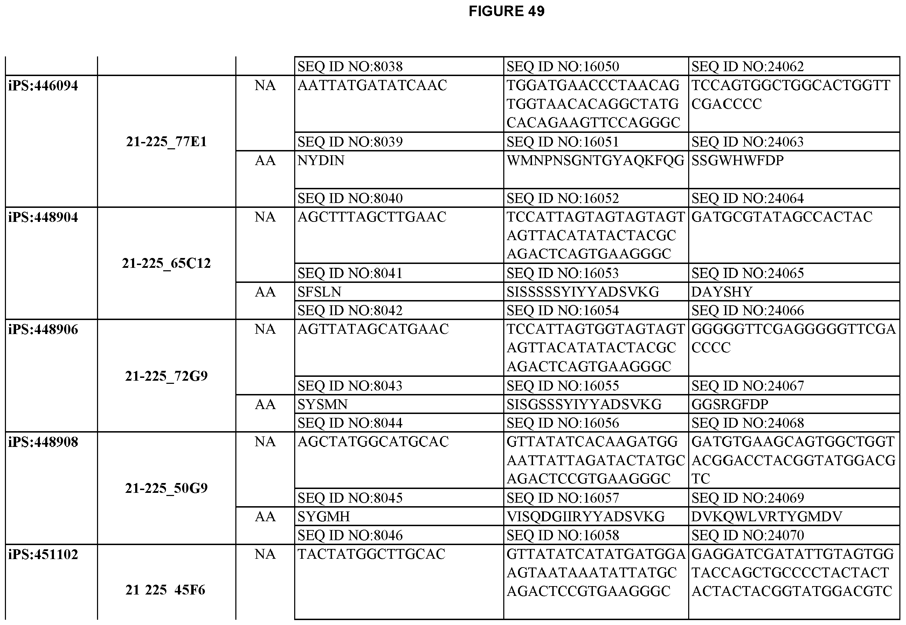

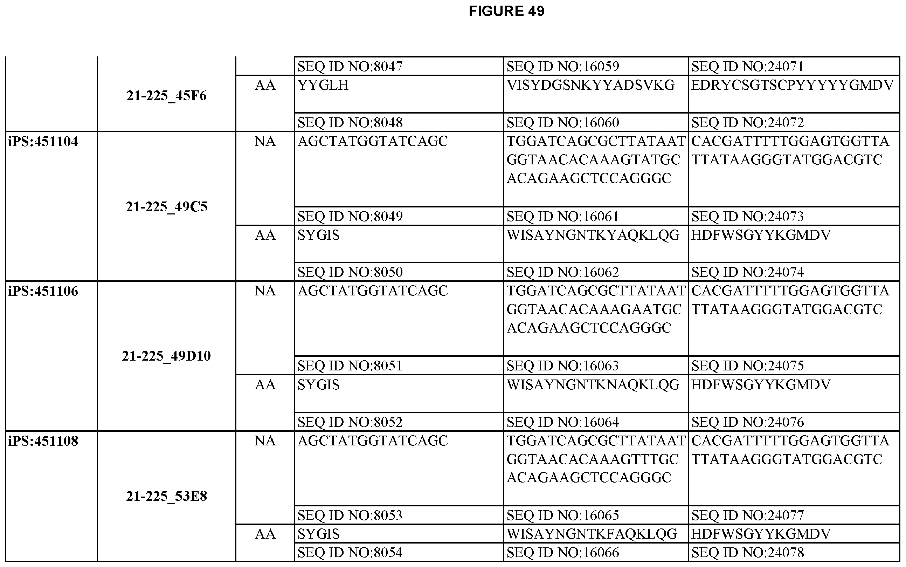

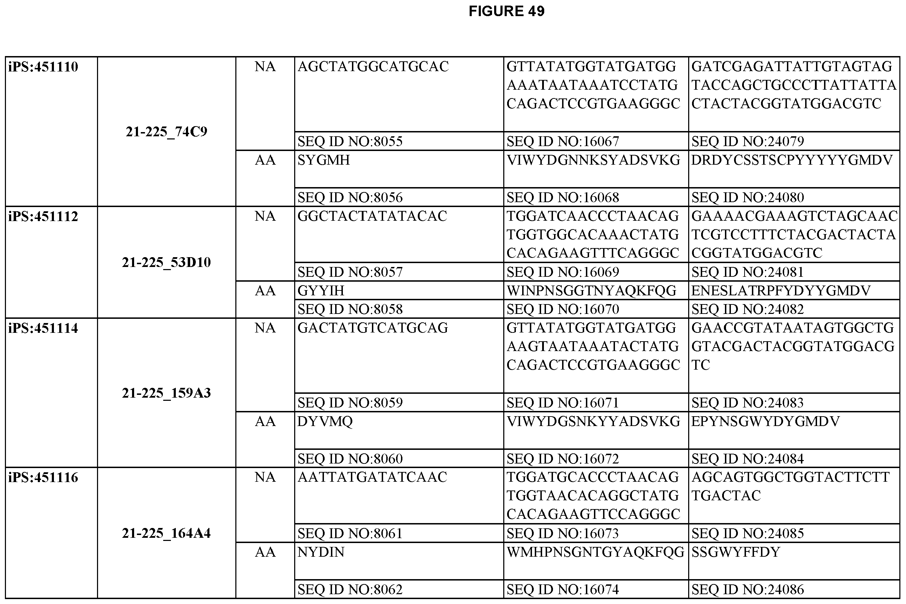

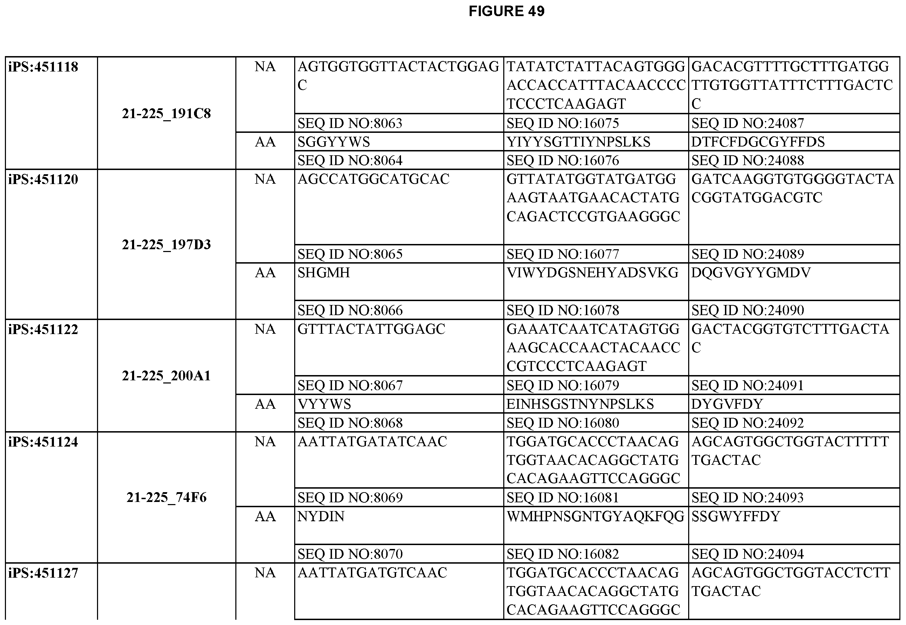

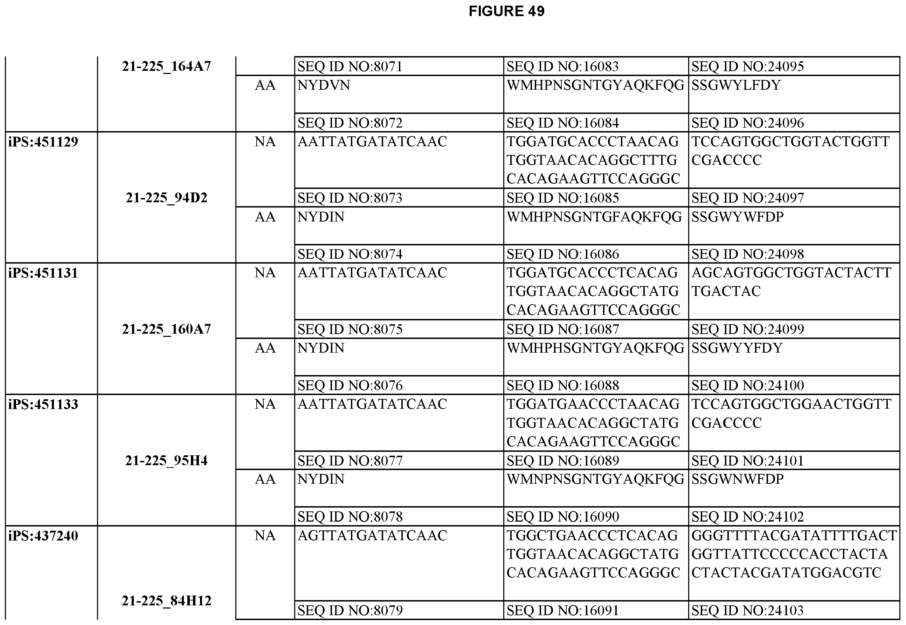

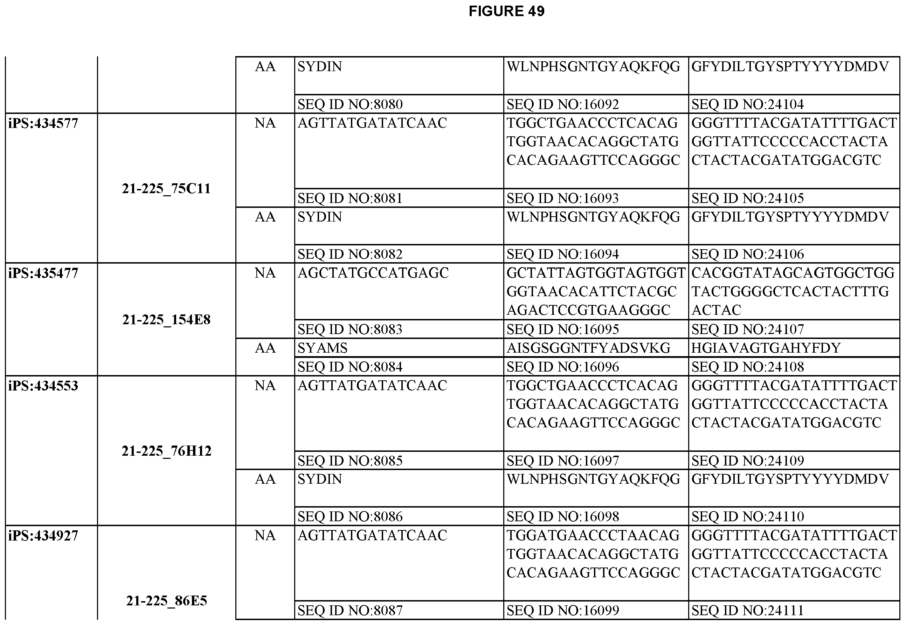

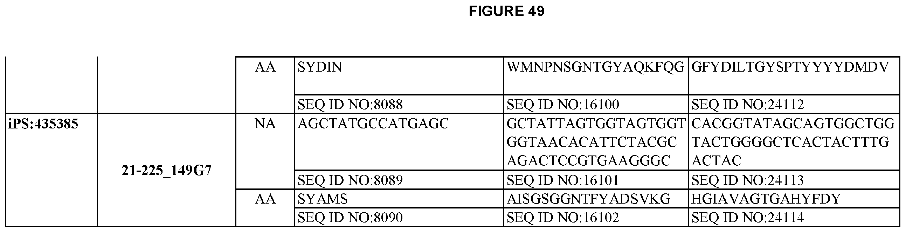

**Figure 49.** Two tables presenting variable light and heavy chain CDR1, CDR2 and CDR3 amino acid sequences for certain antigen binding proteins (Table 2A and Table 2B). Table 2A presents the Variable Light Chain CDR1, CDR2 and CDR3, while Table 2B presents the Variable Heavy Chain CDR1, CDR2, and CDR3. The CDR sequences in Tables 2A and 2B are wrapped due to space issues, and unless stated otherwise, should be understood to be a single amino acid sequence.

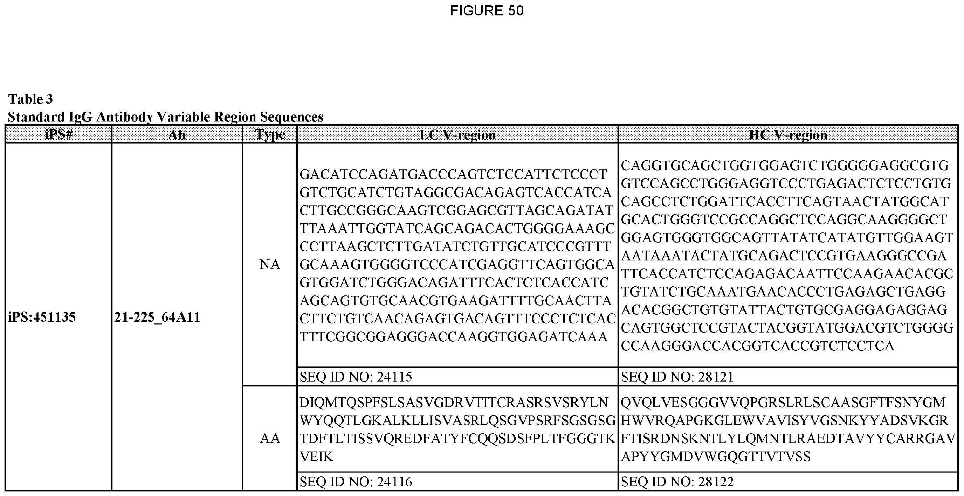

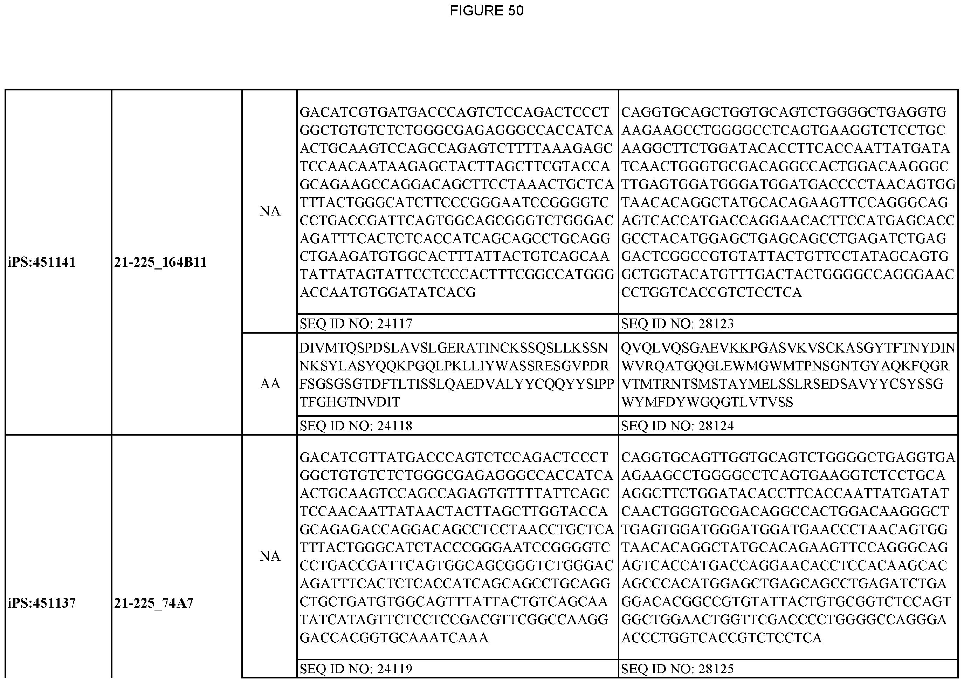

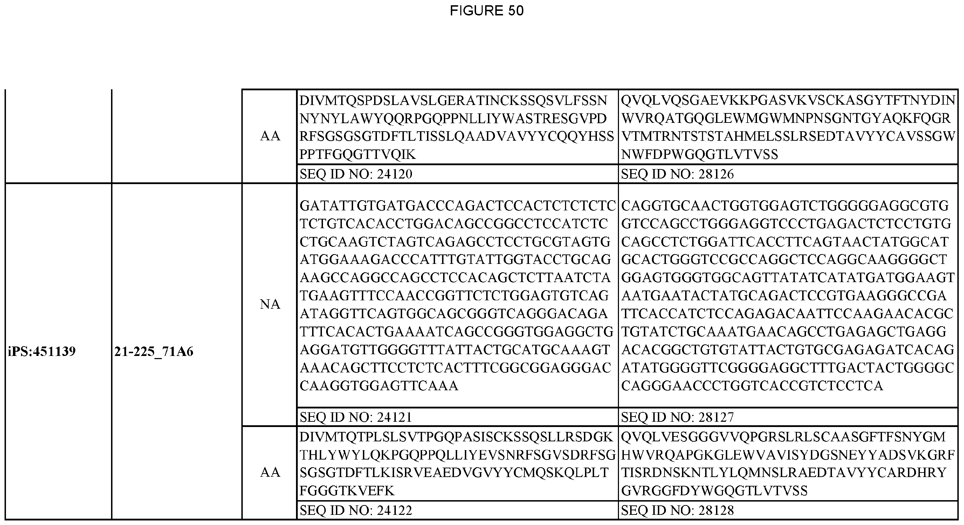

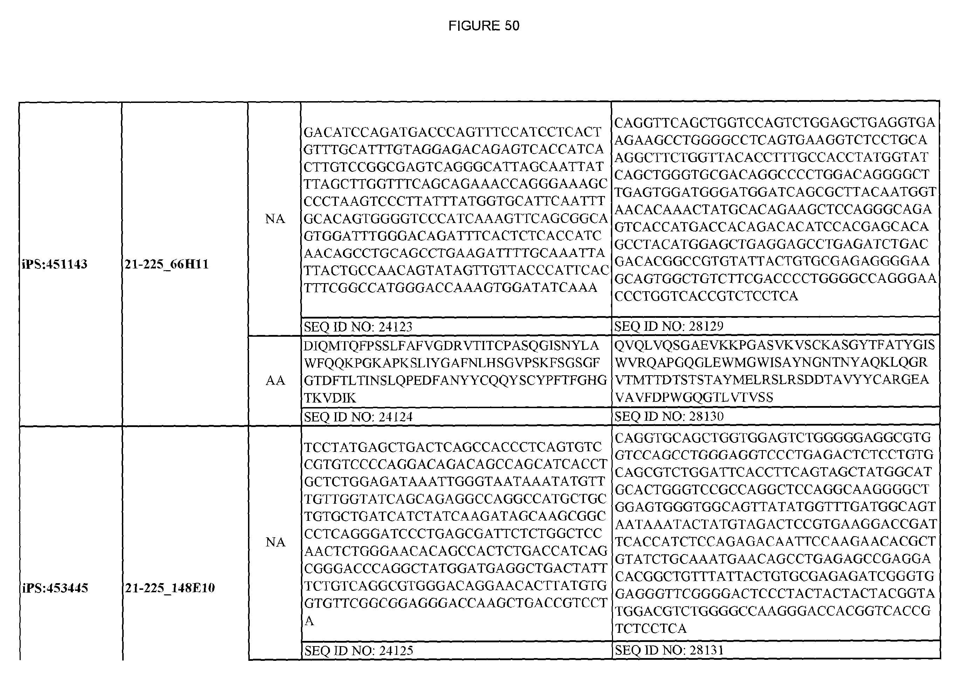

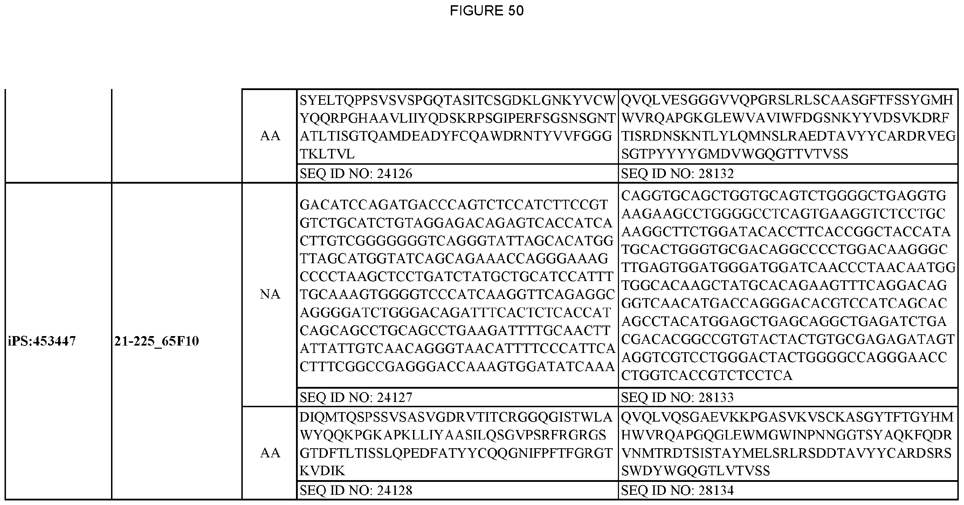

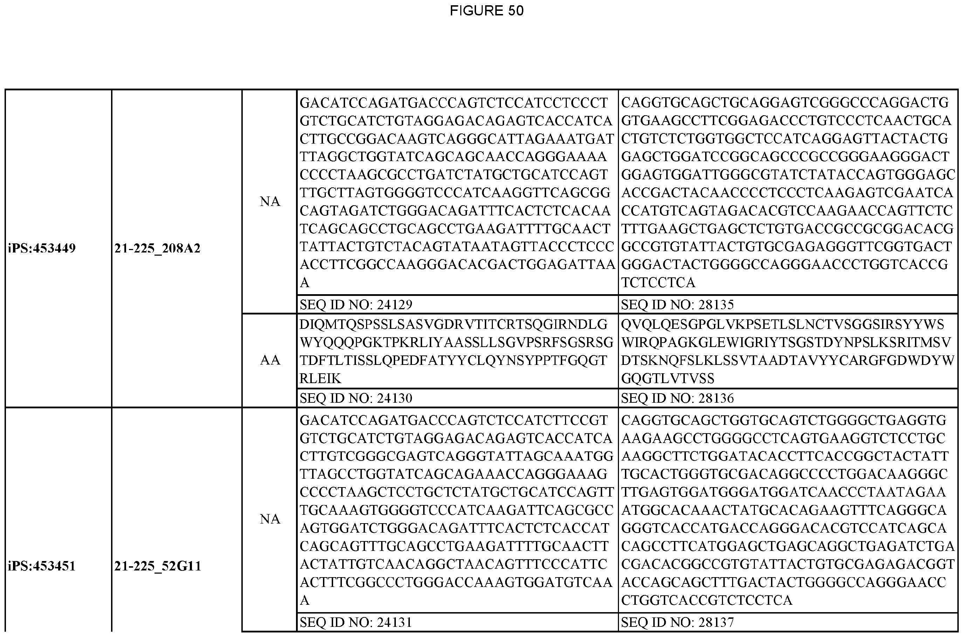

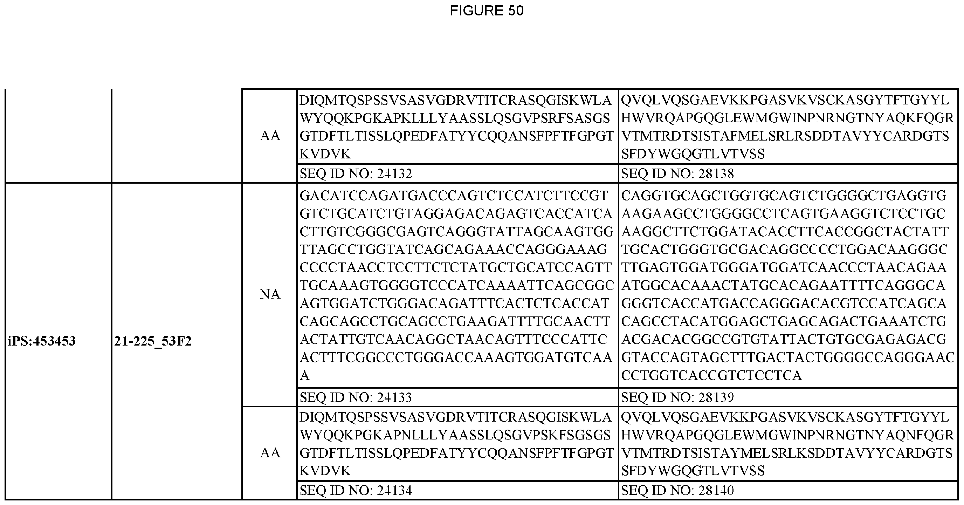

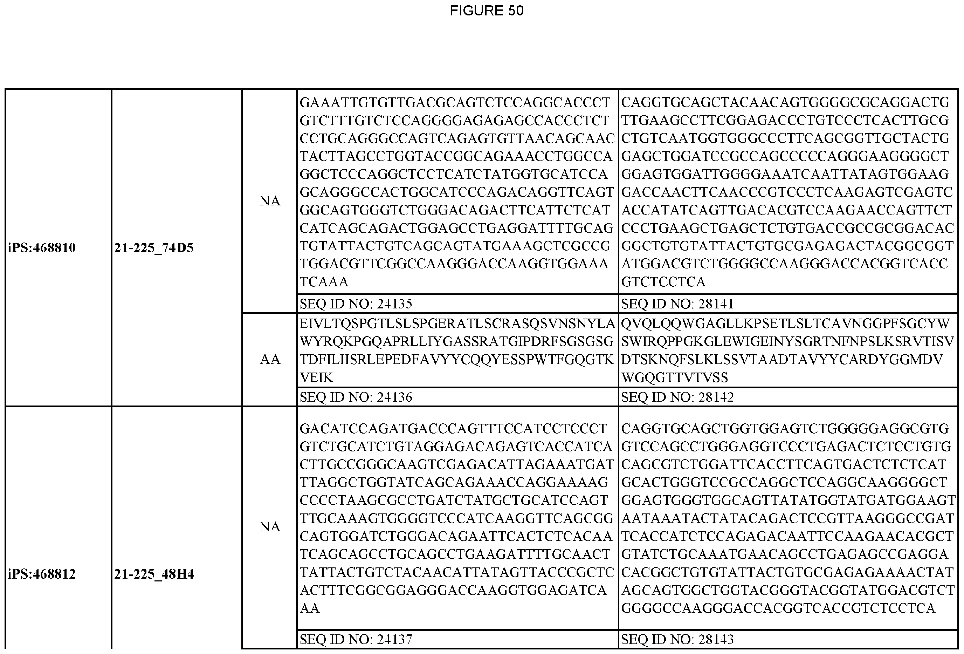

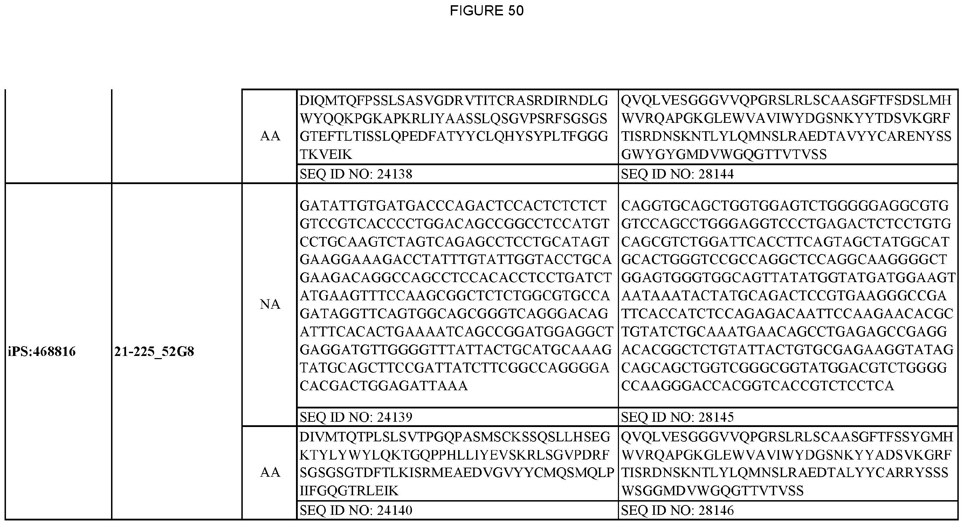

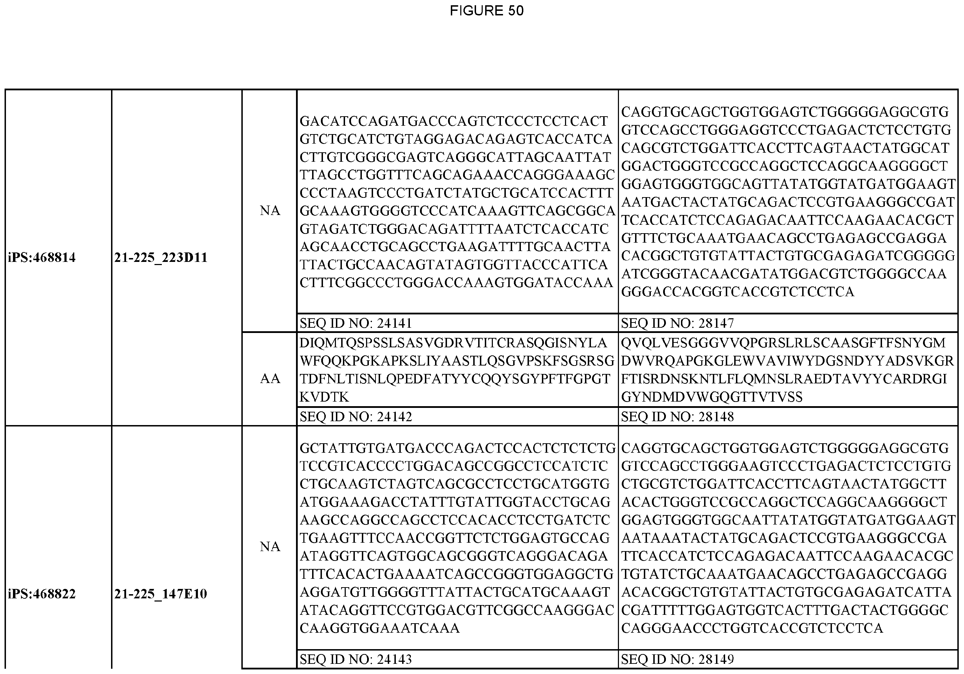

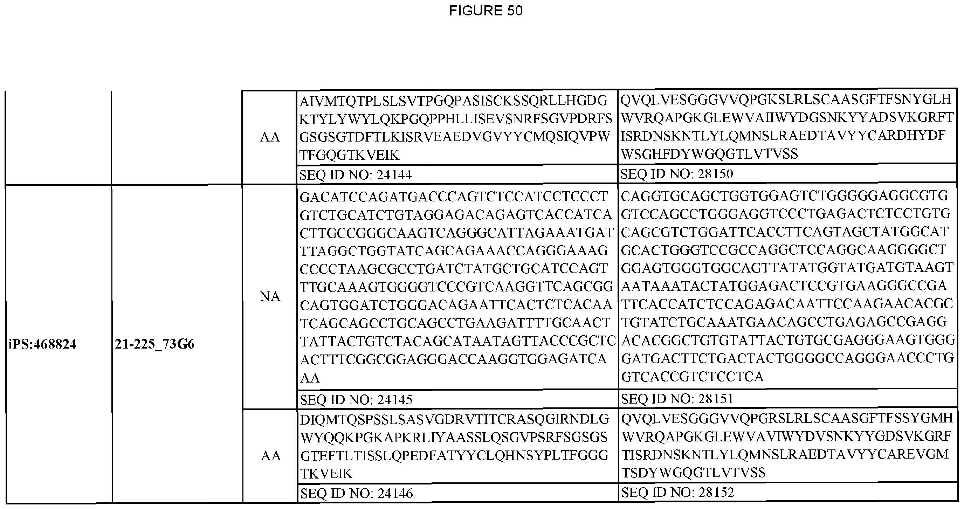

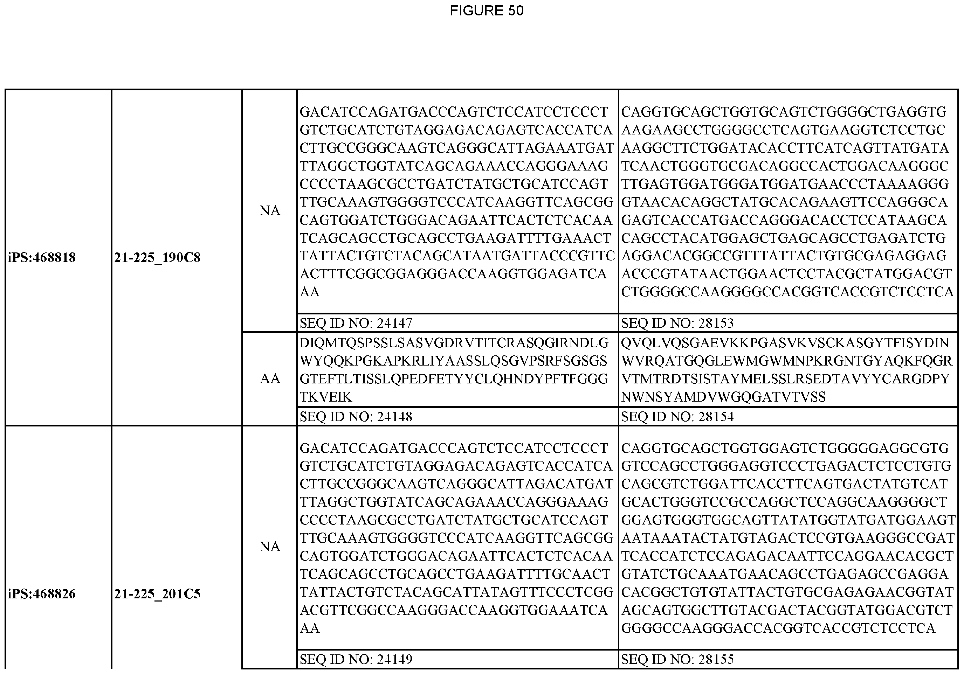

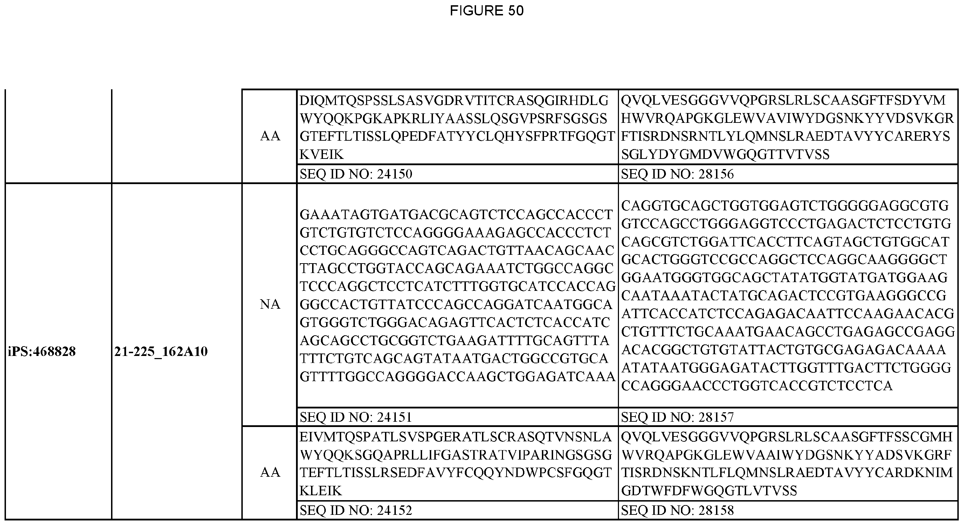

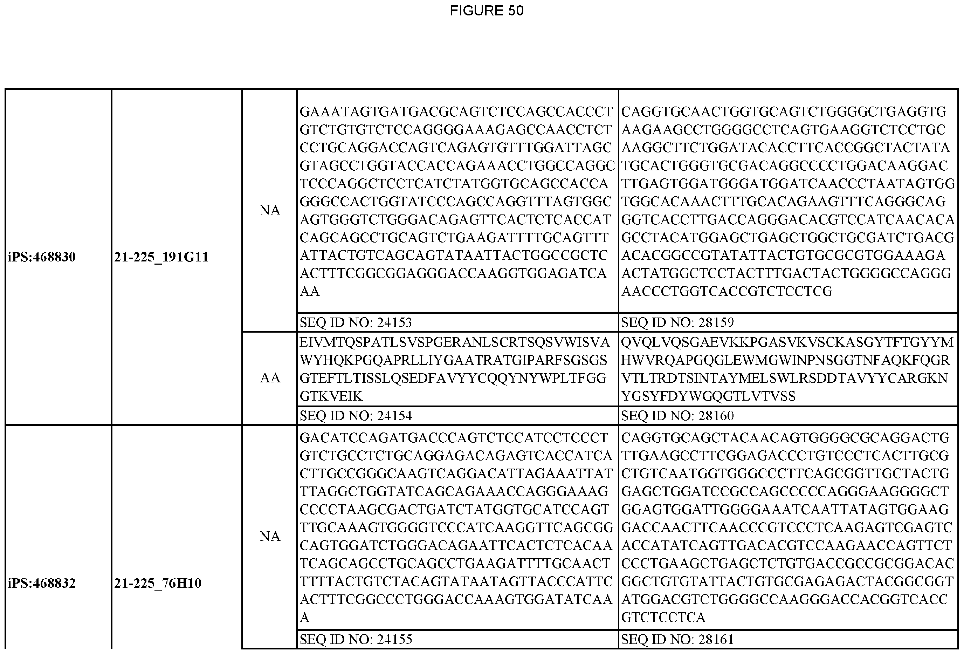

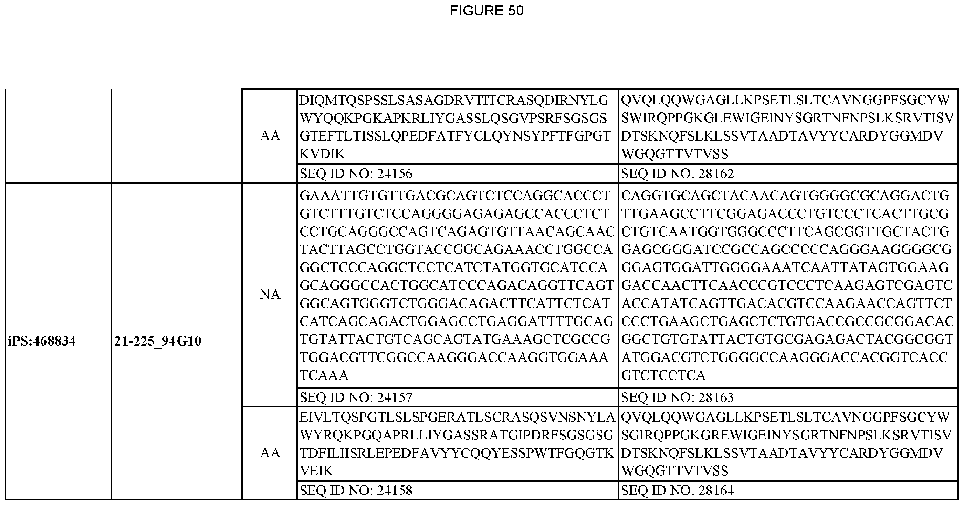

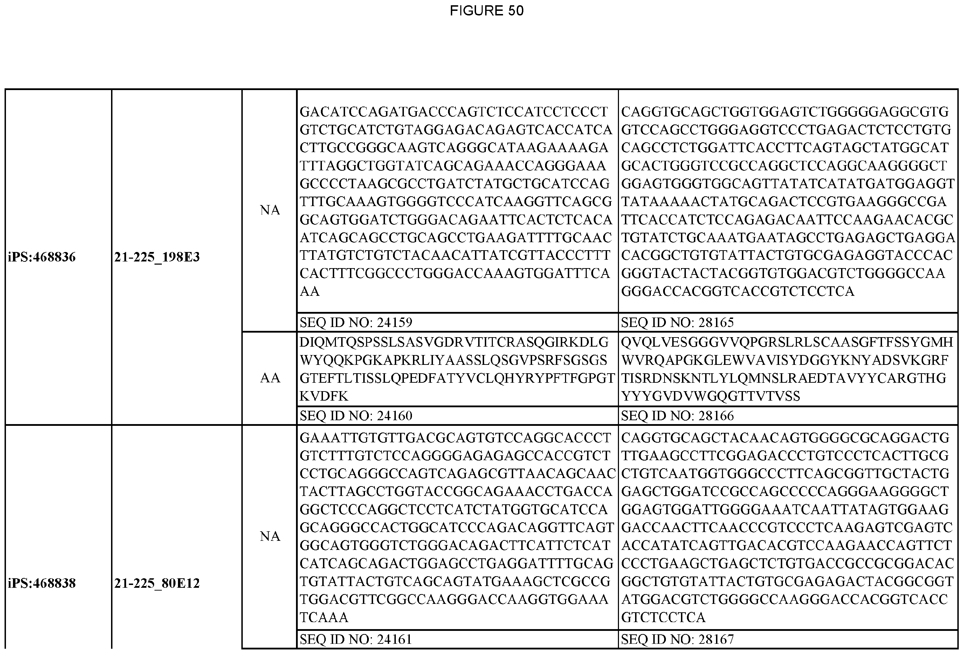

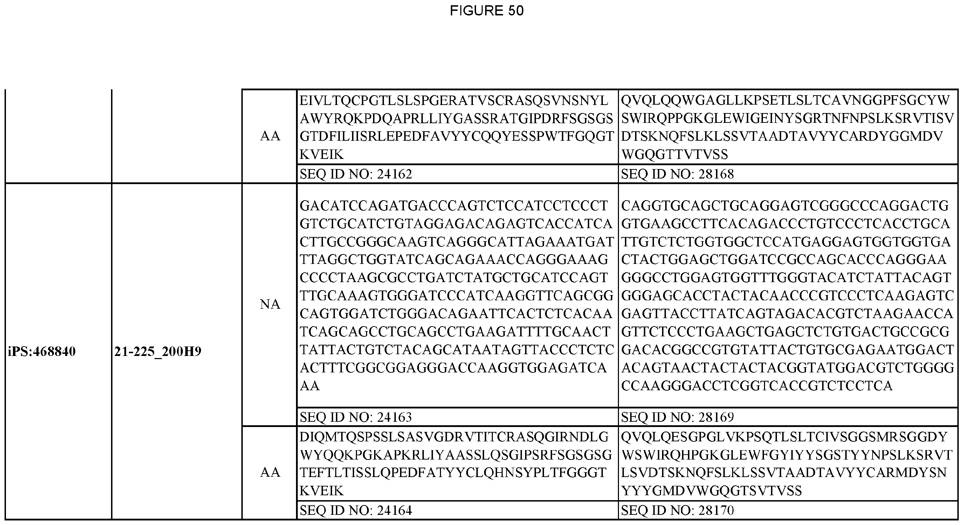

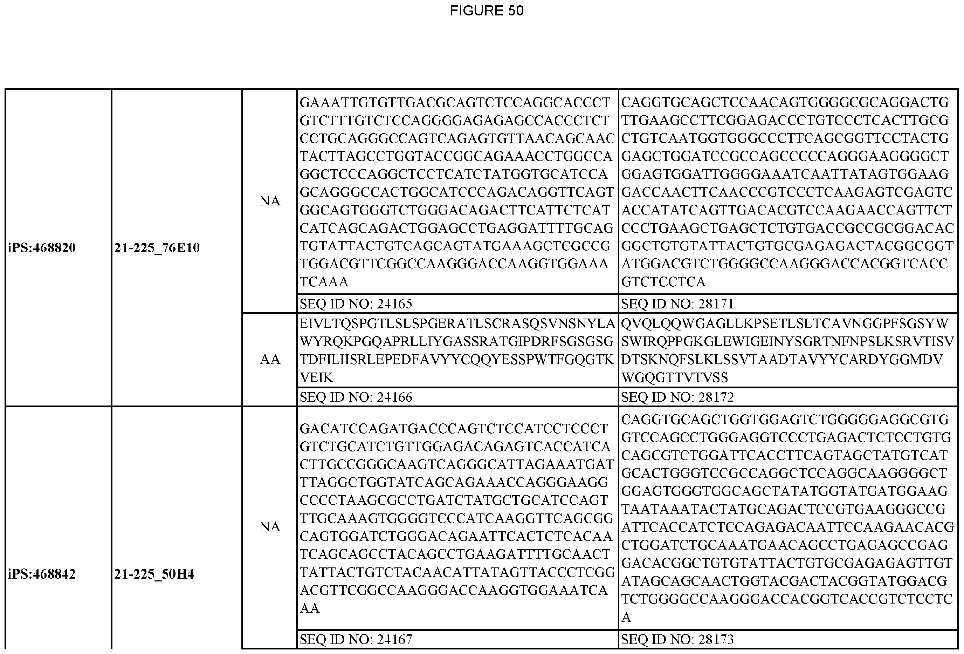

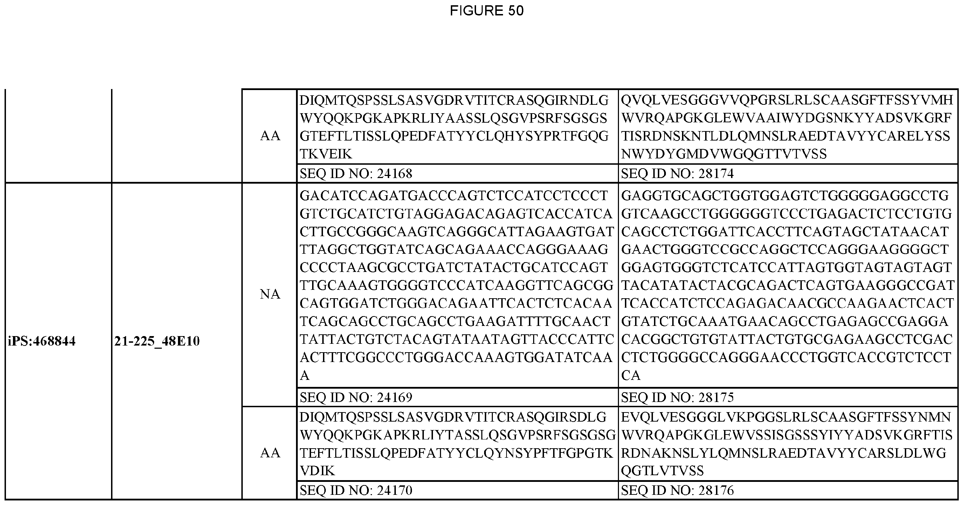

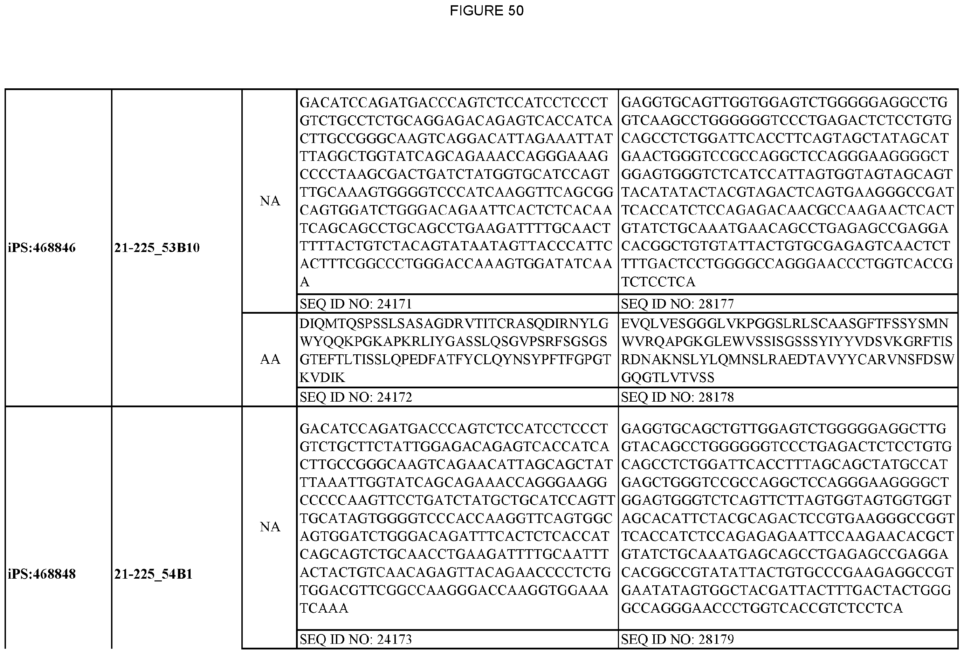

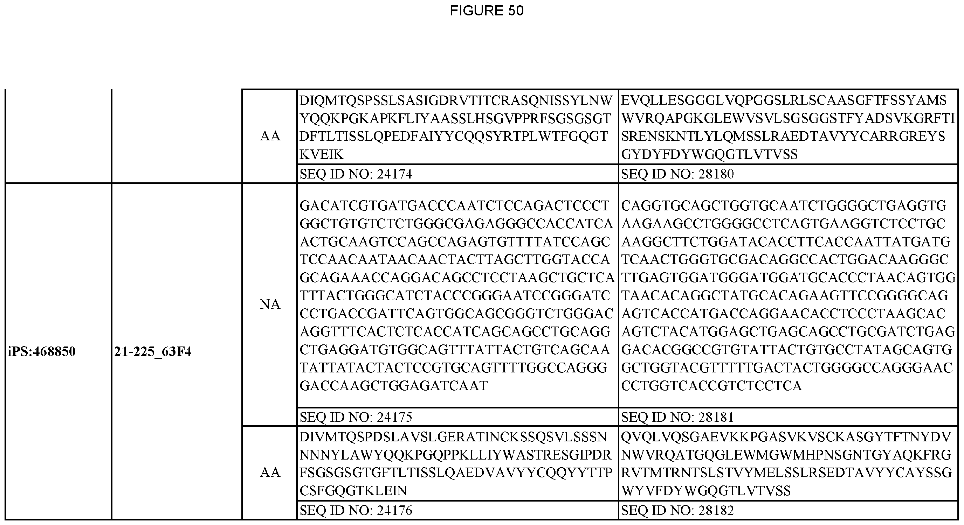

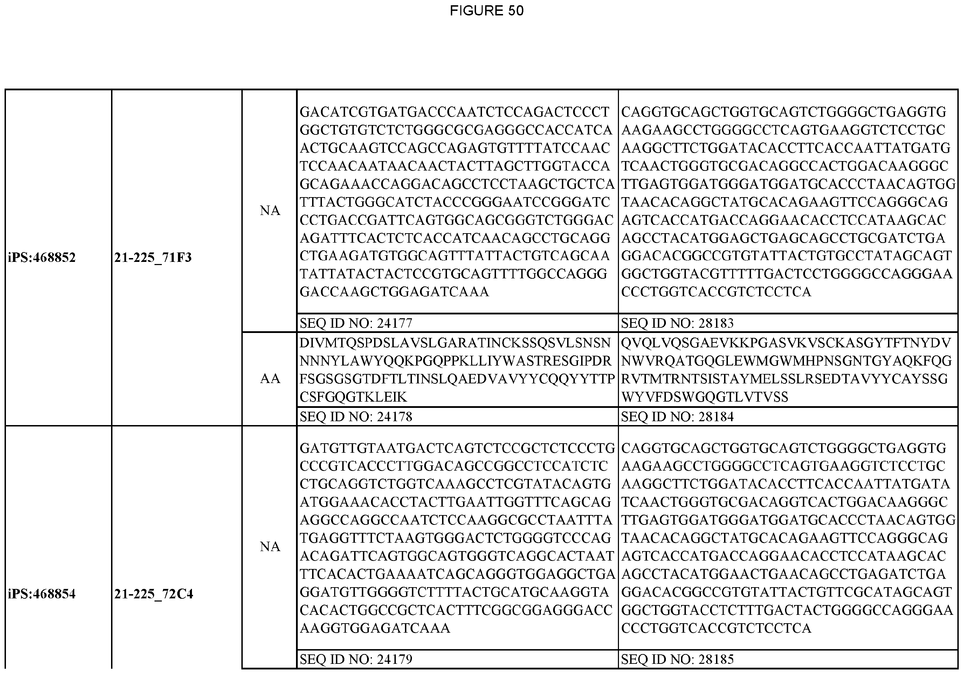

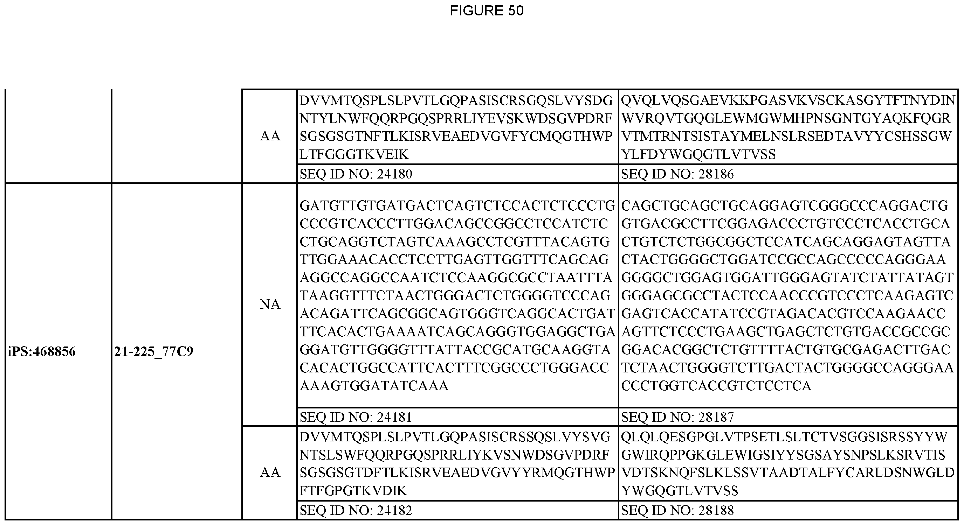

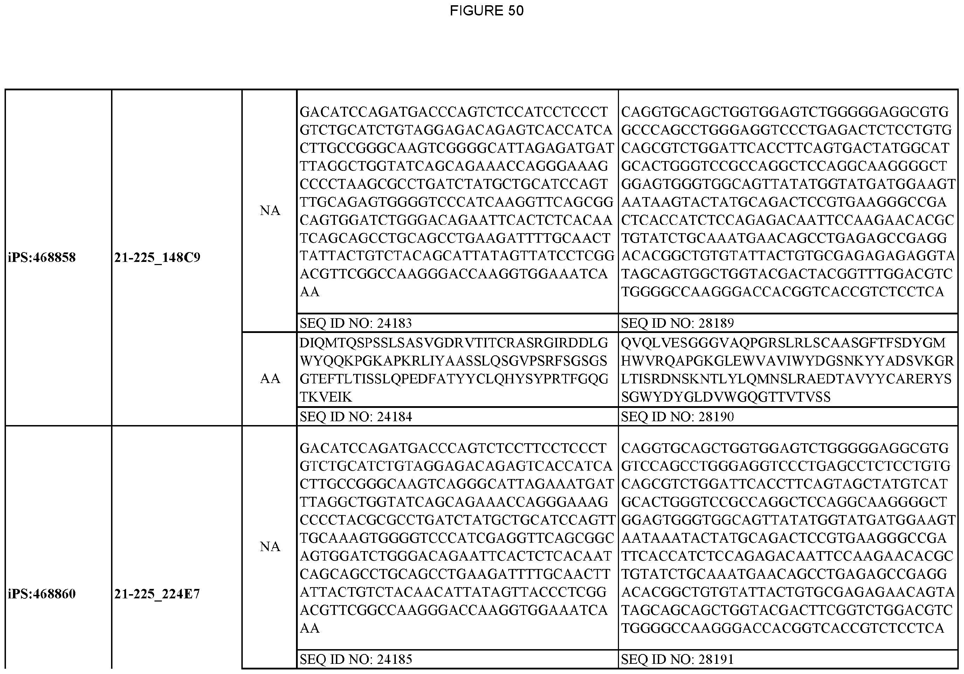

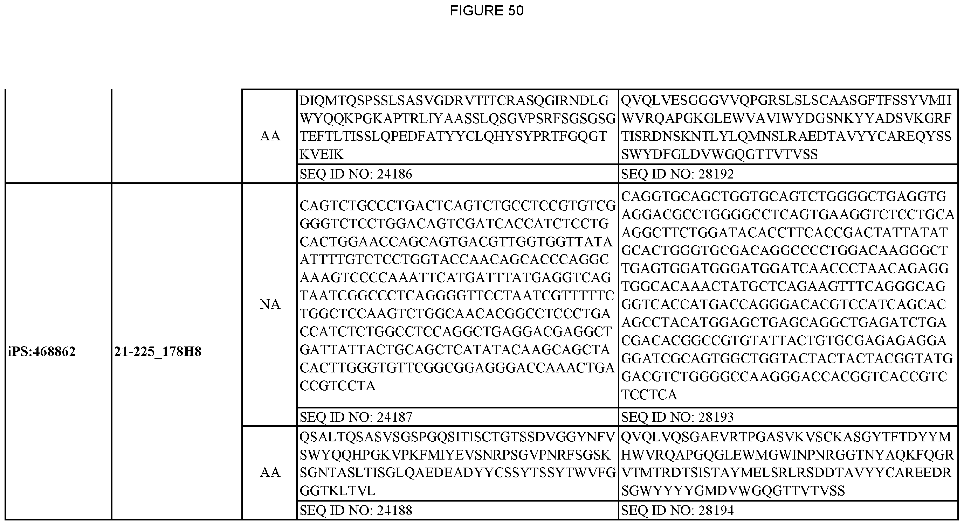

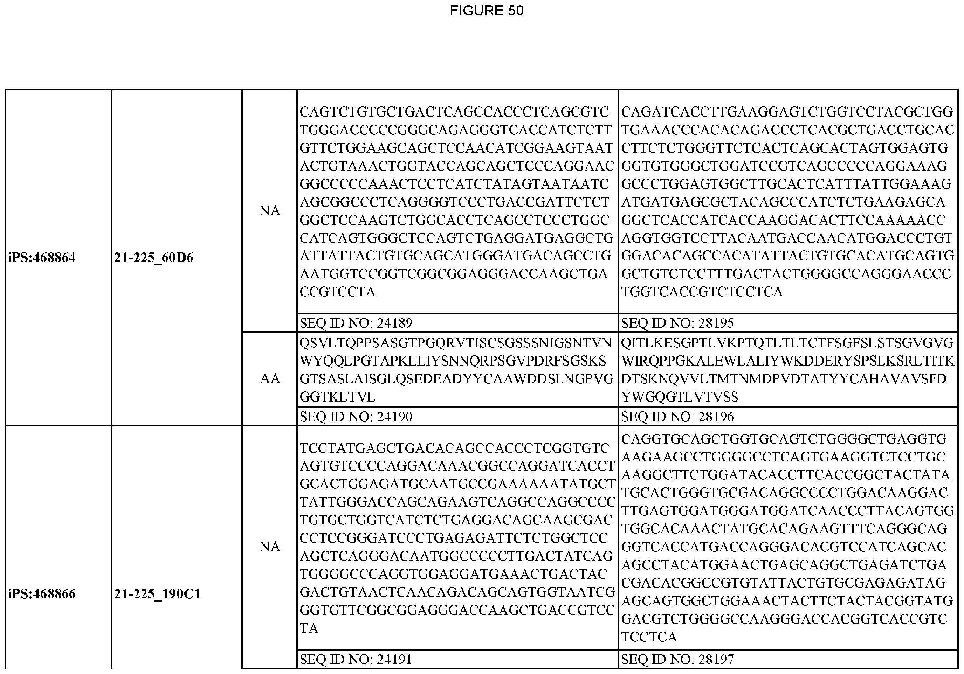

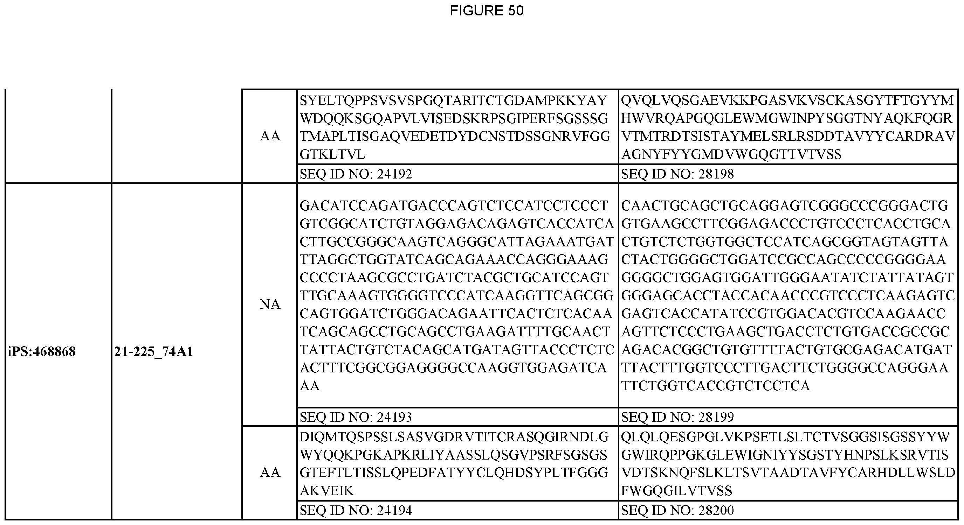

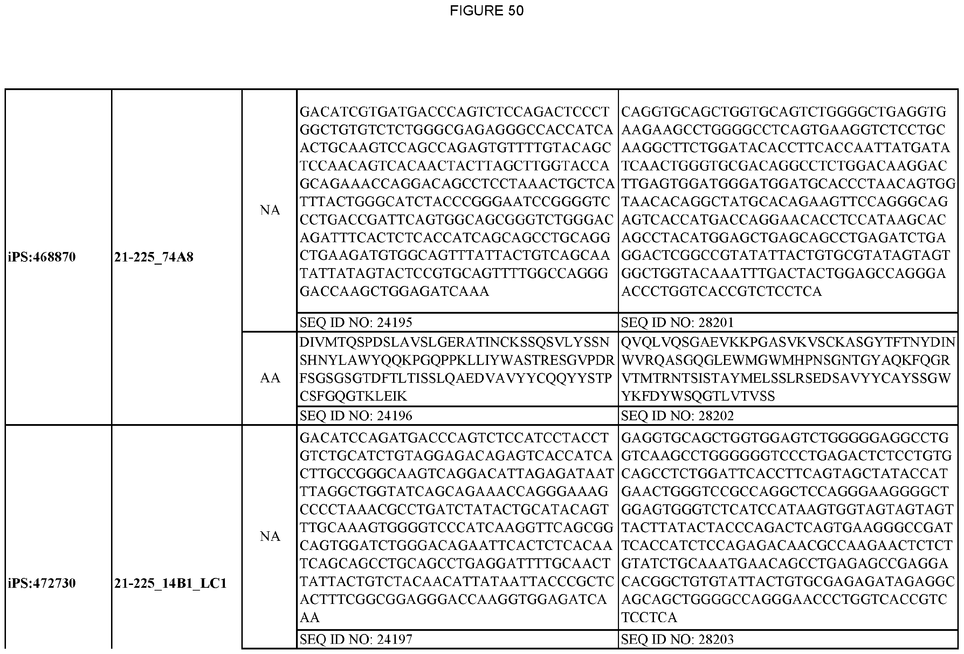

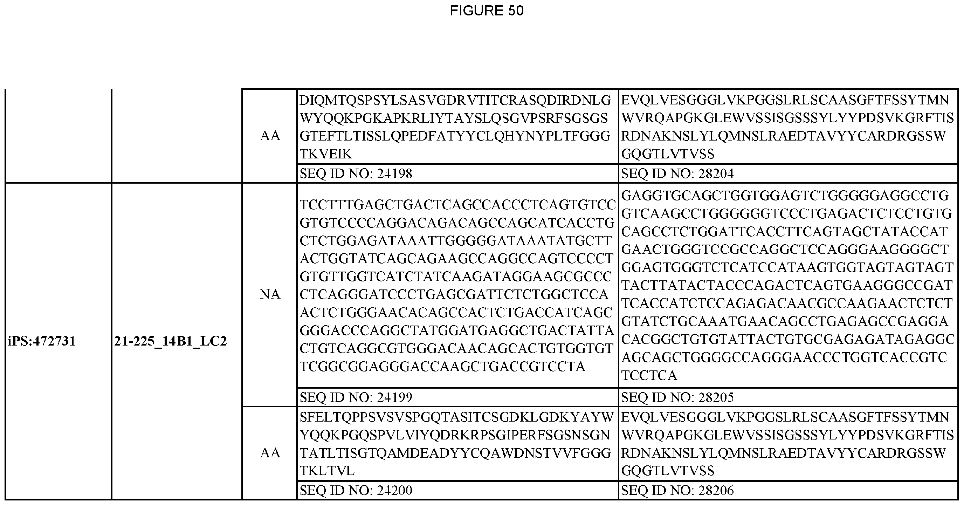

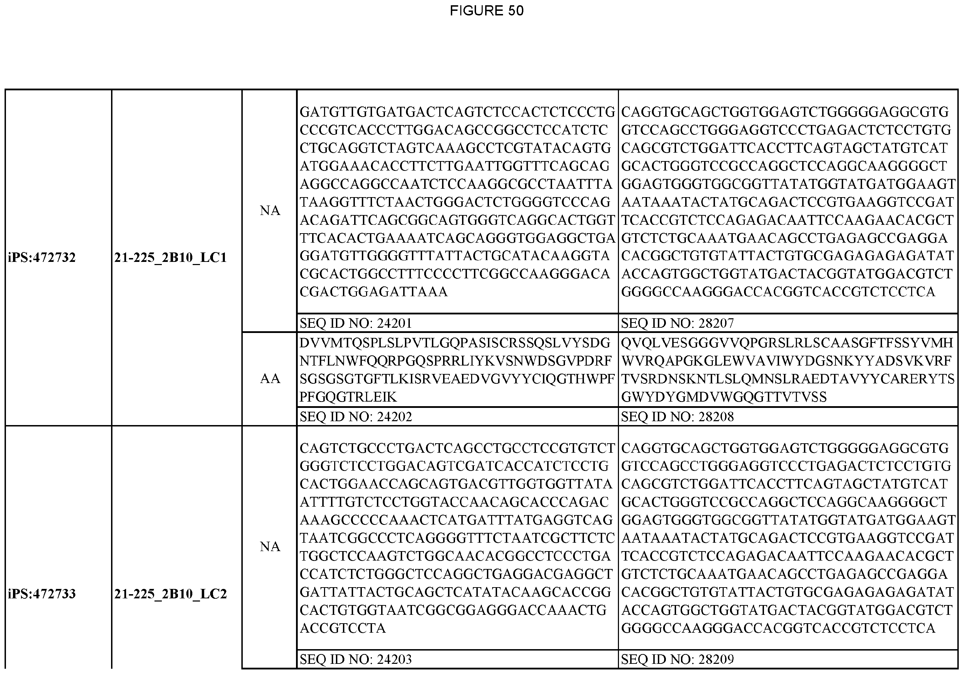

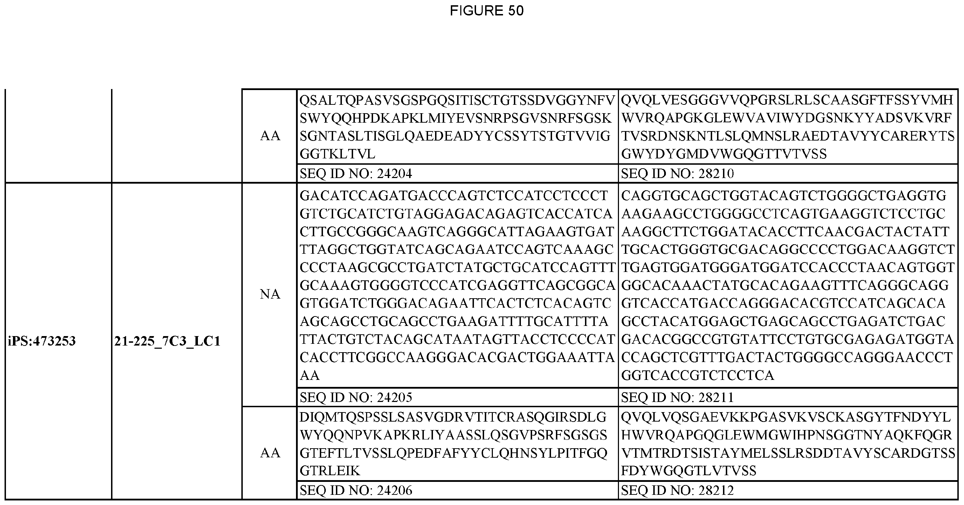

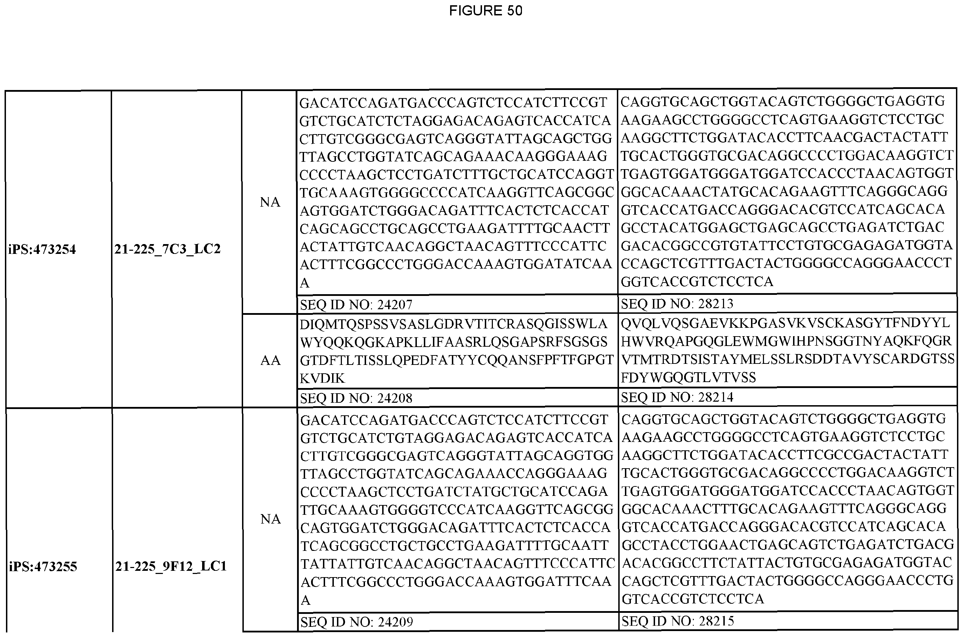

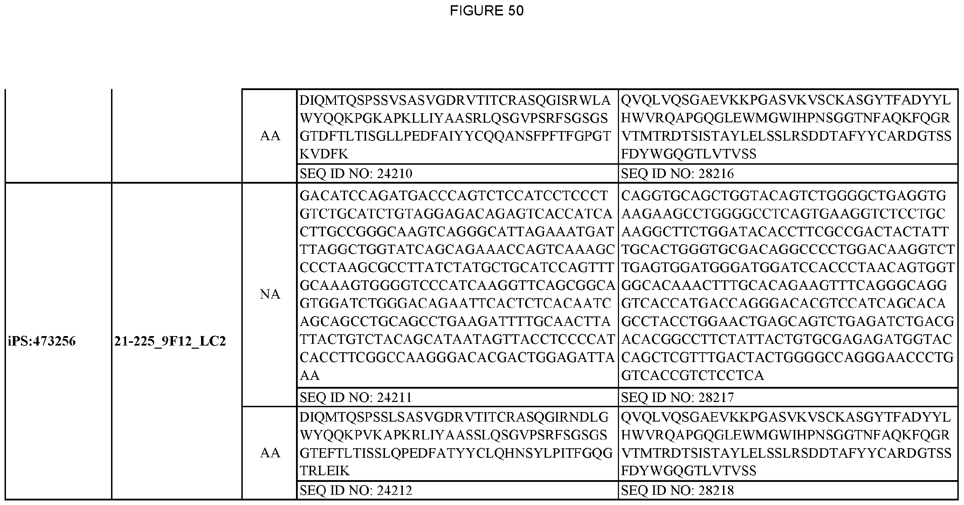

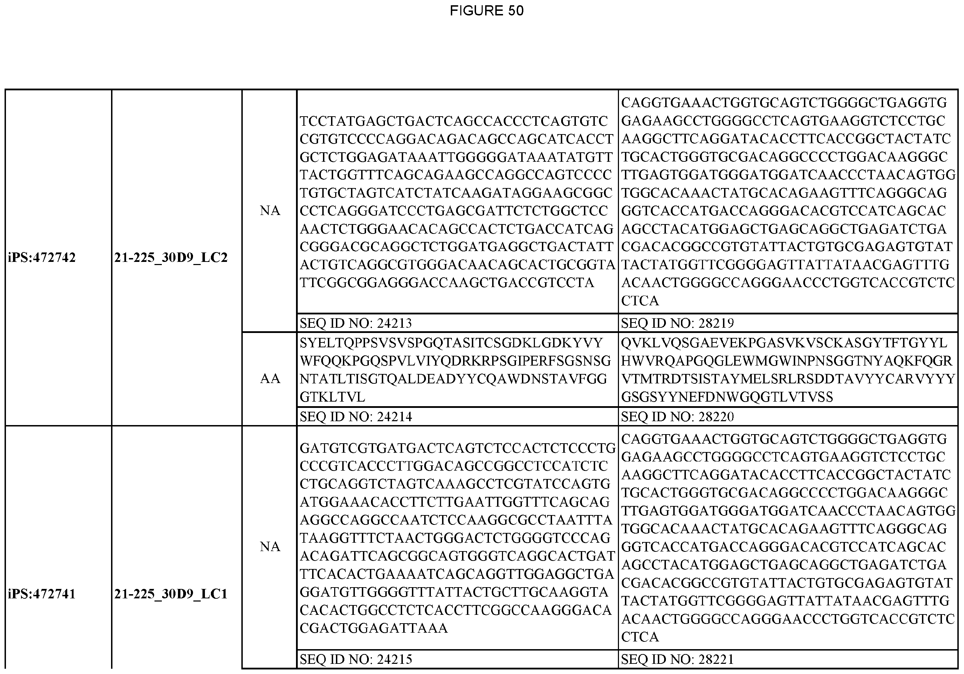

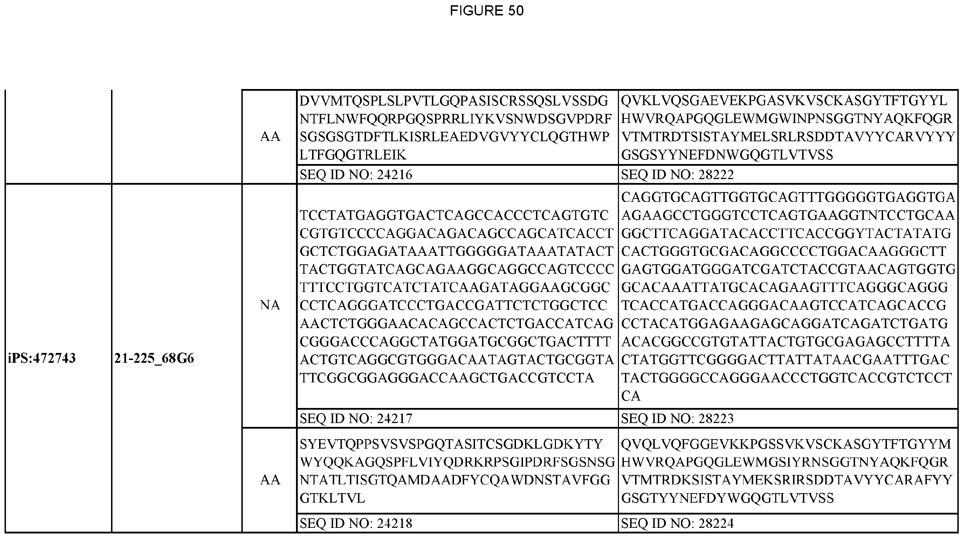

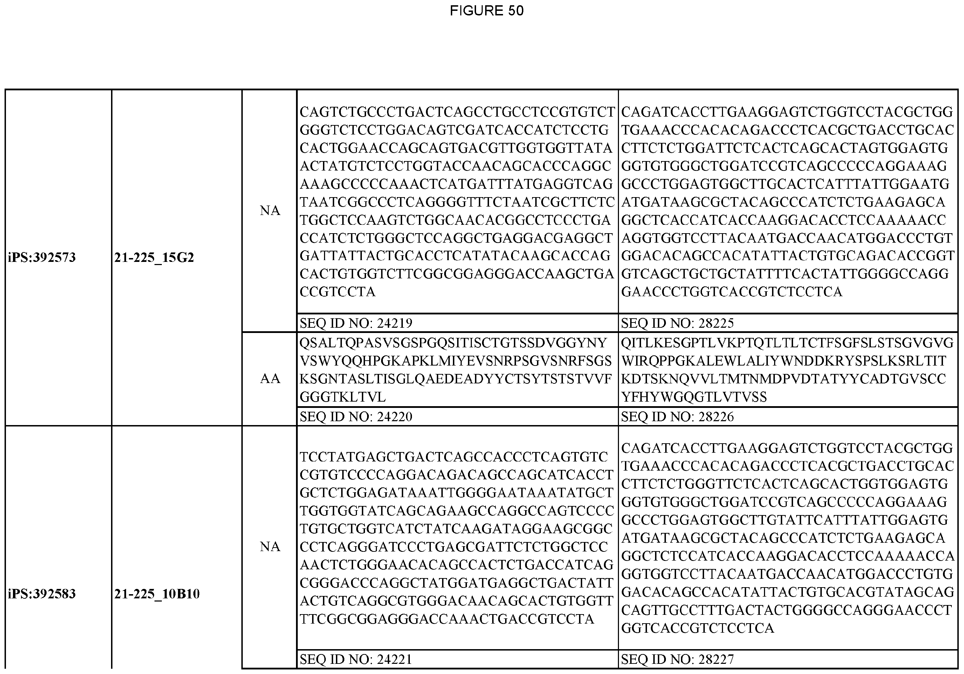

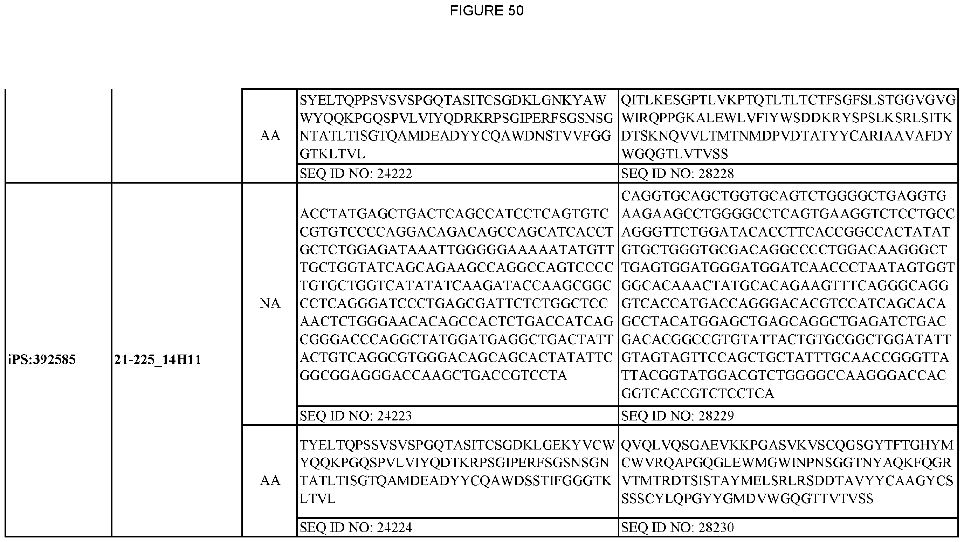

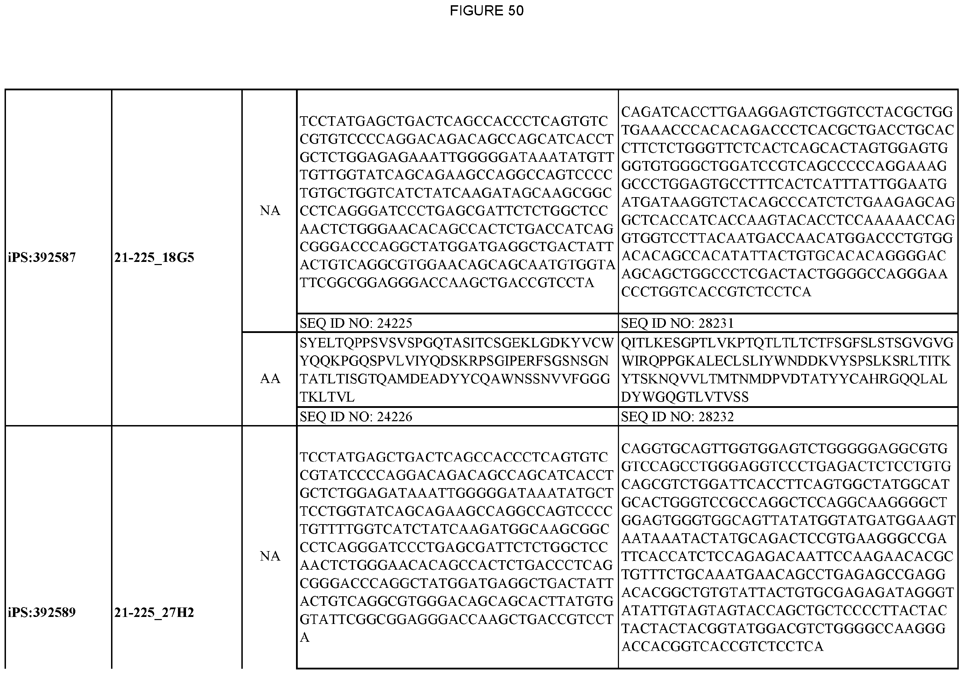

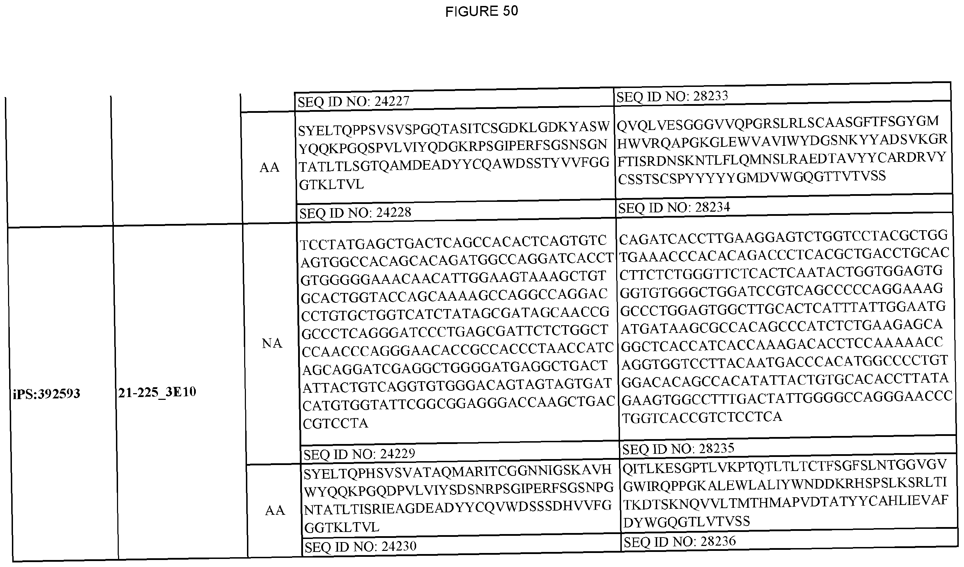

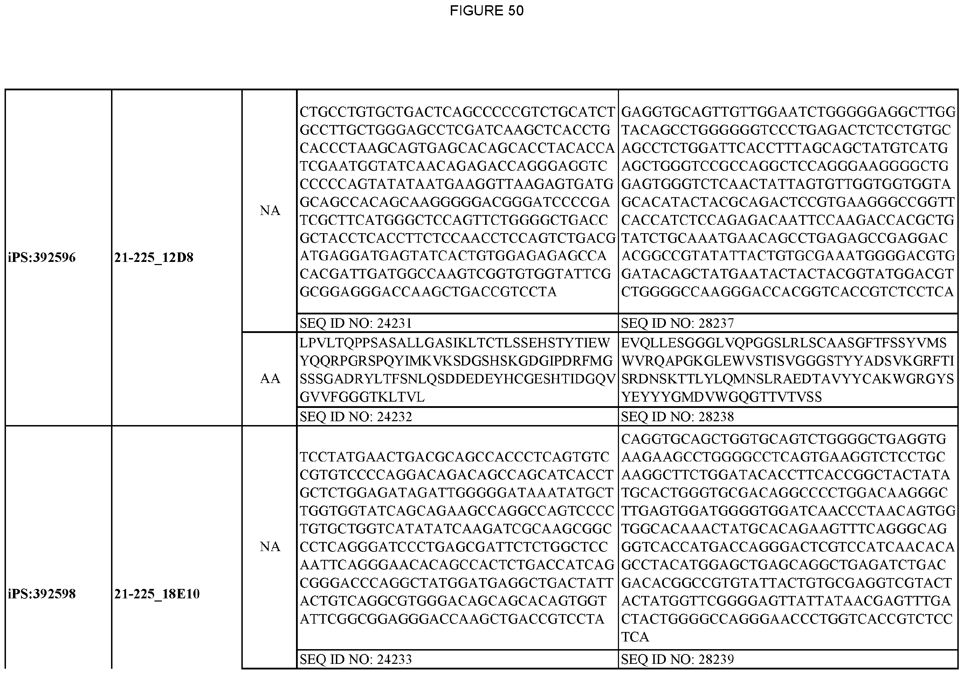

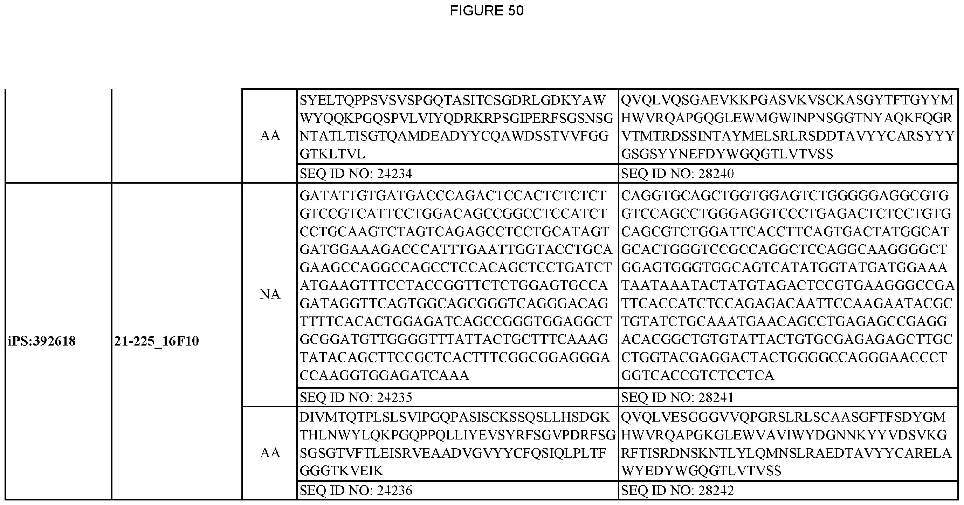

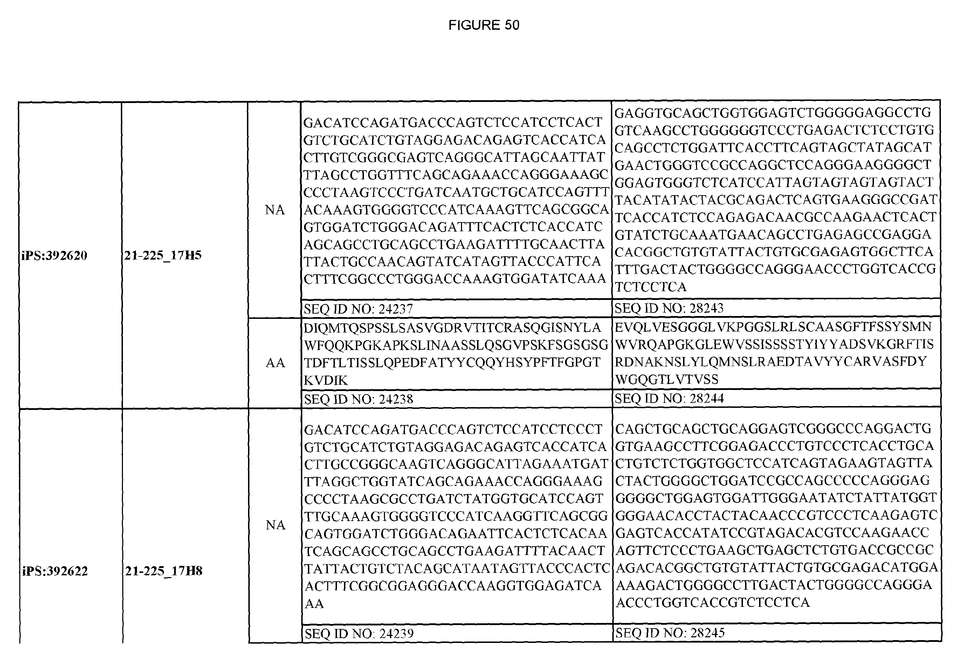

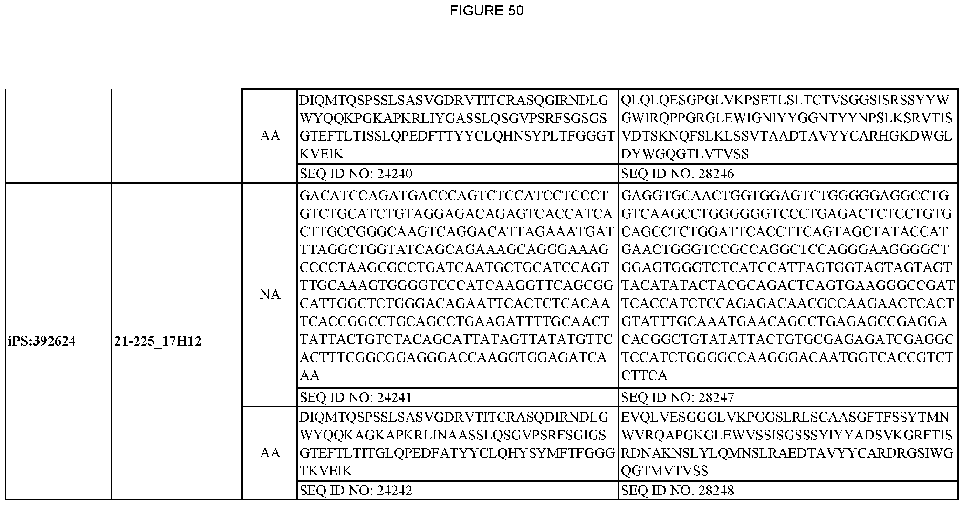

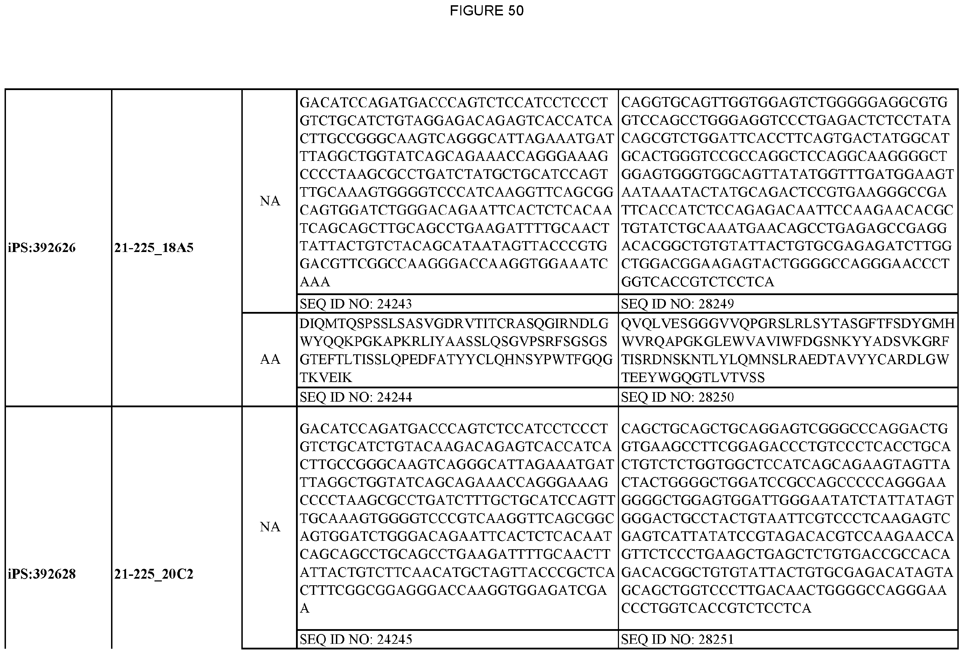

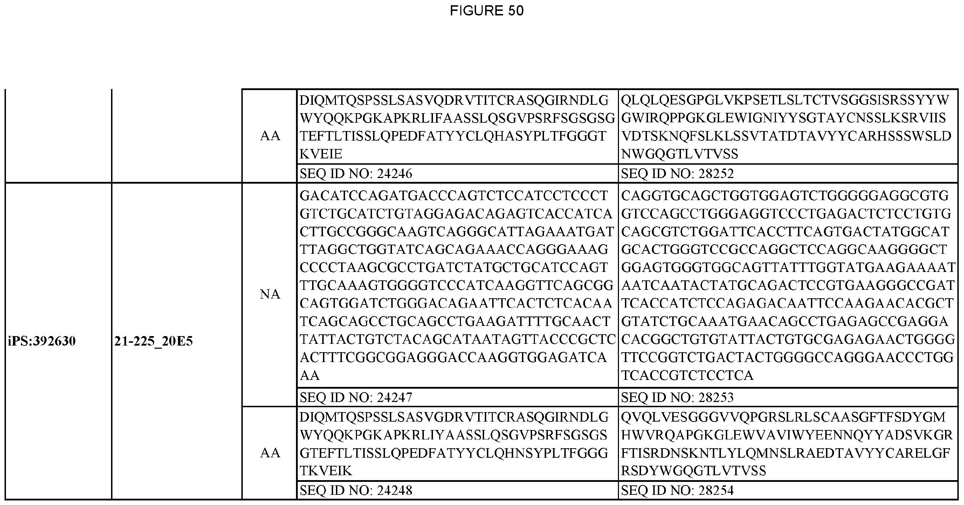

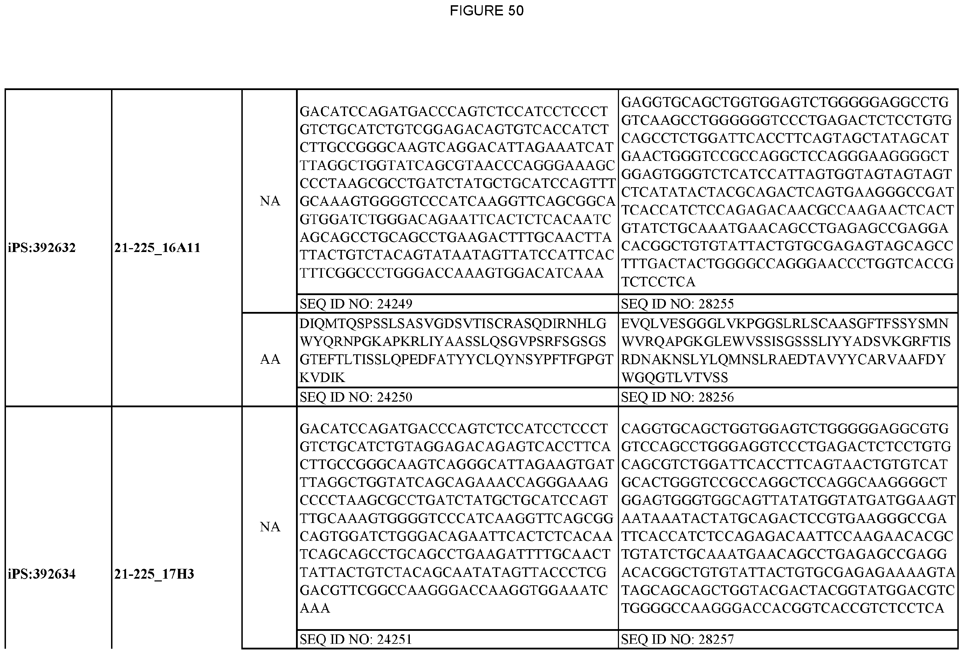

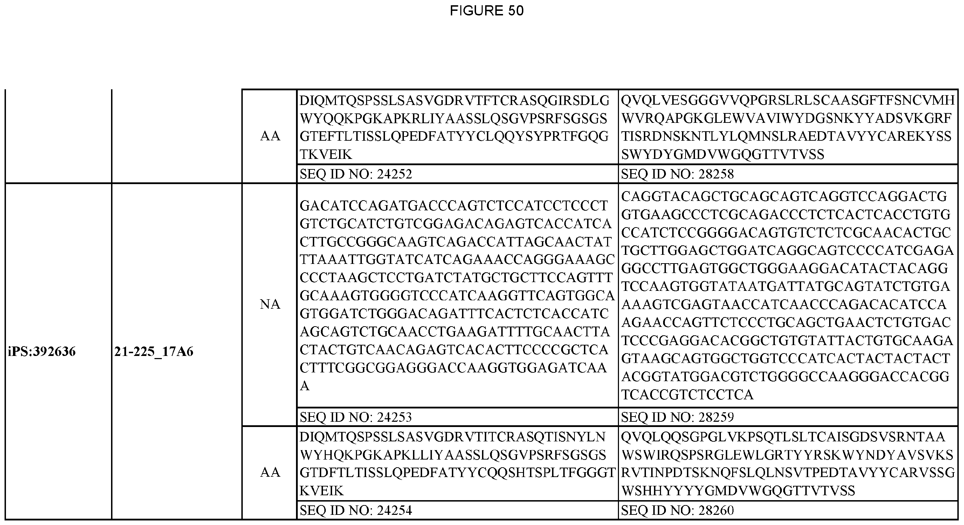

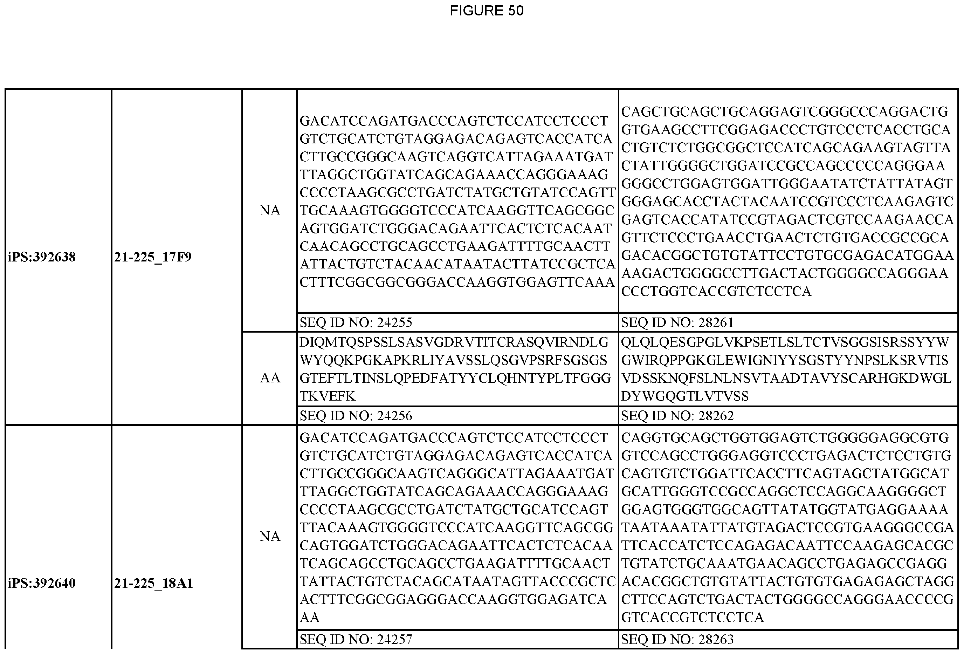

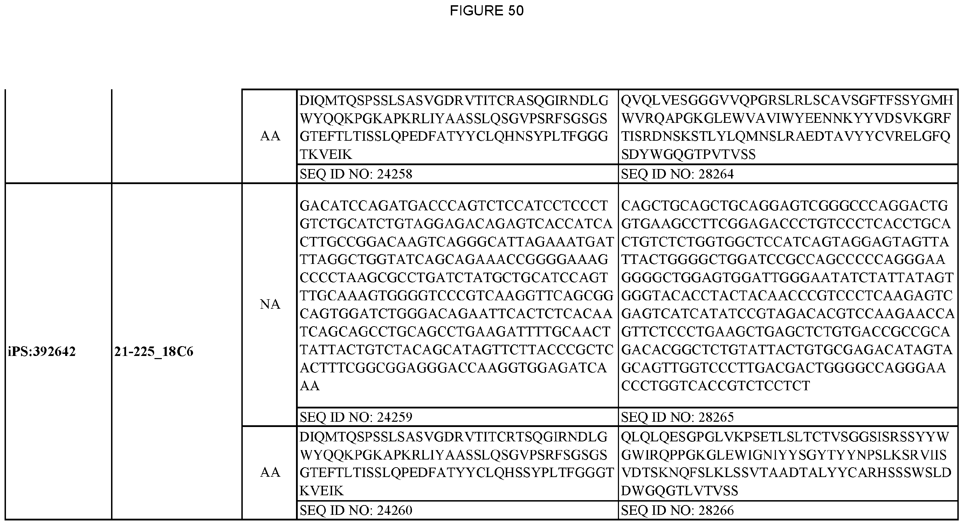

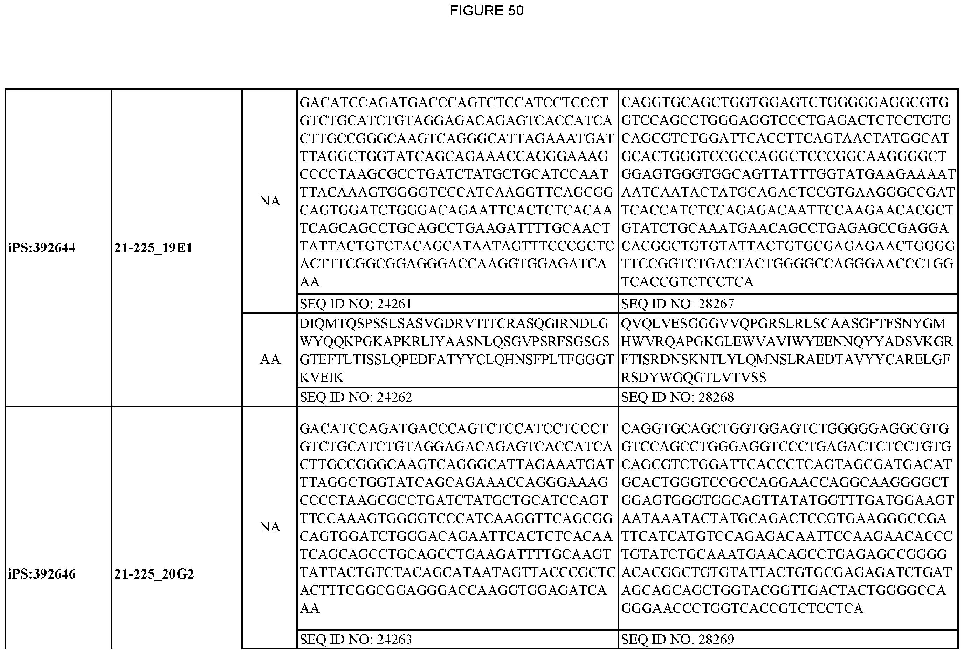

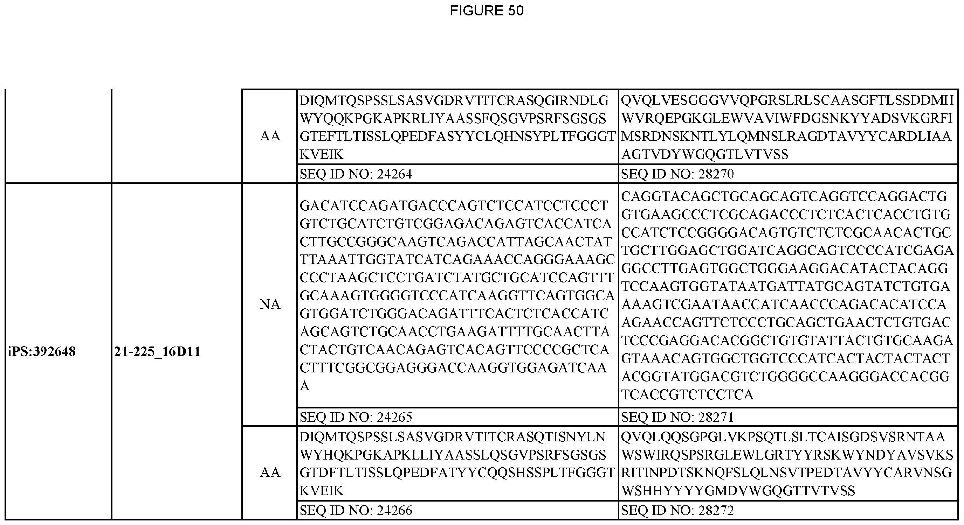

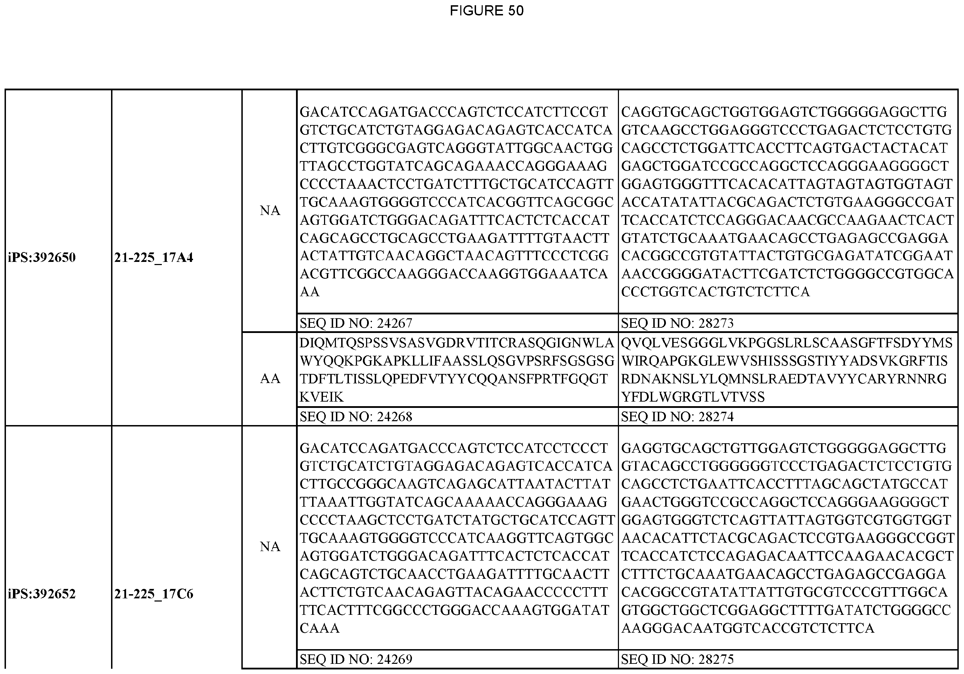

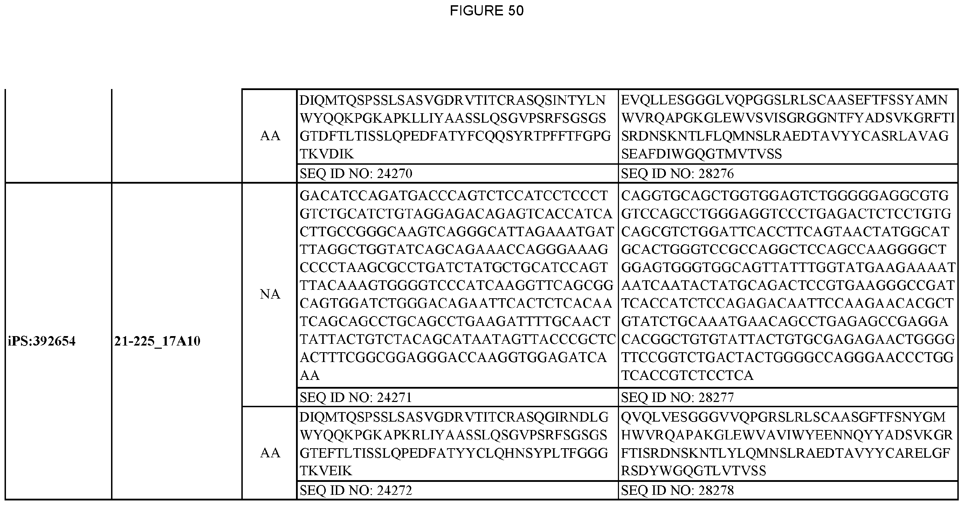

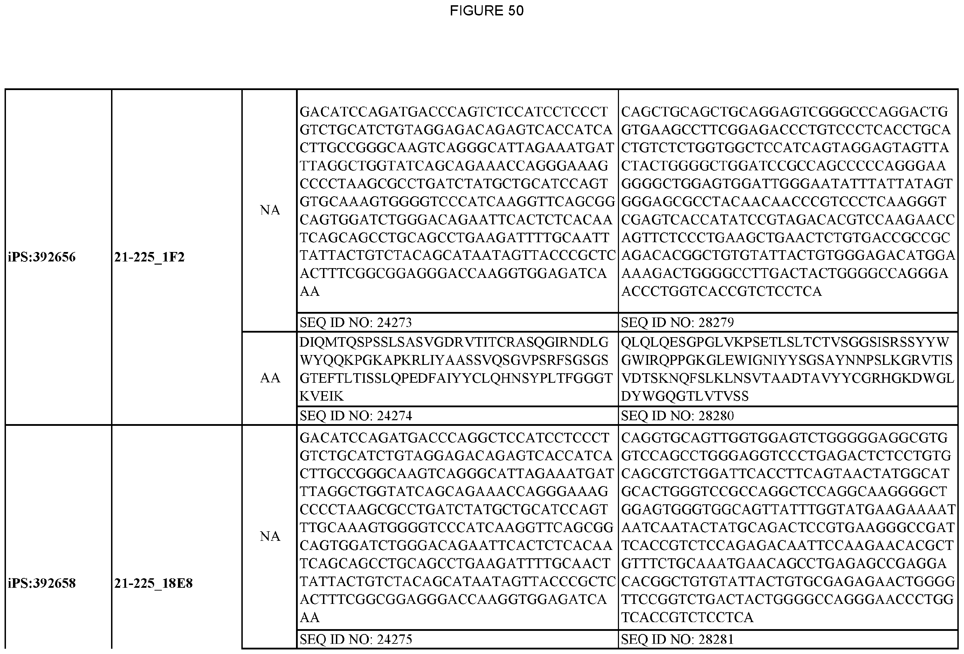

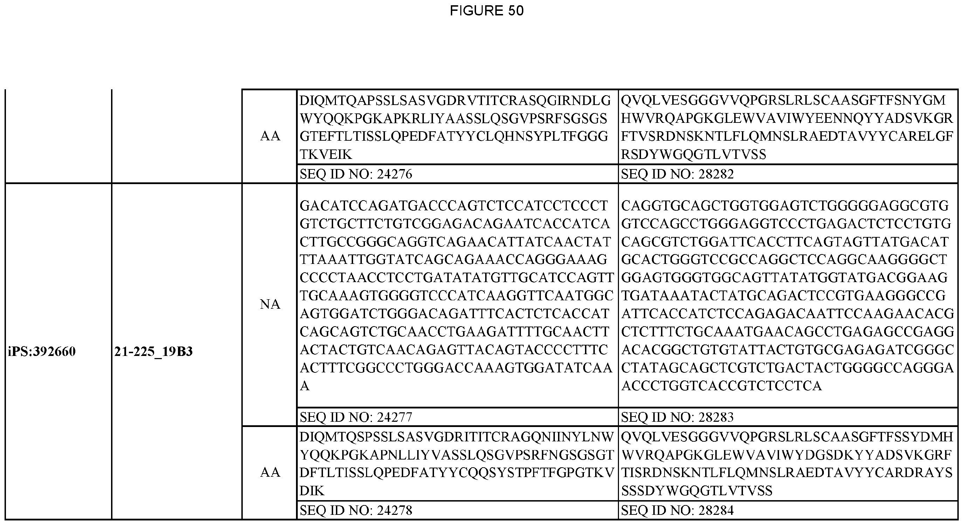

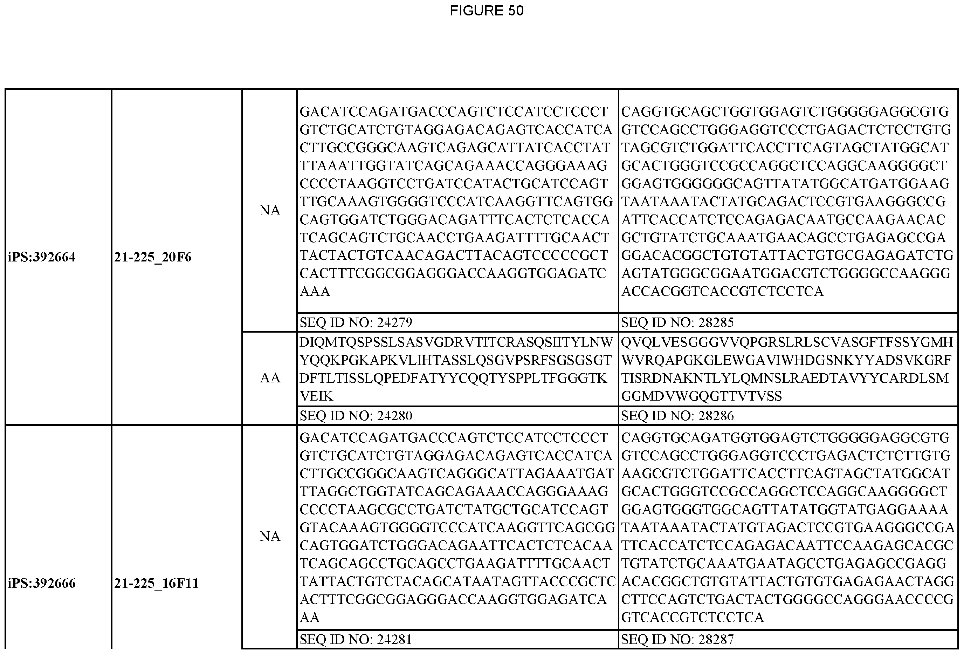

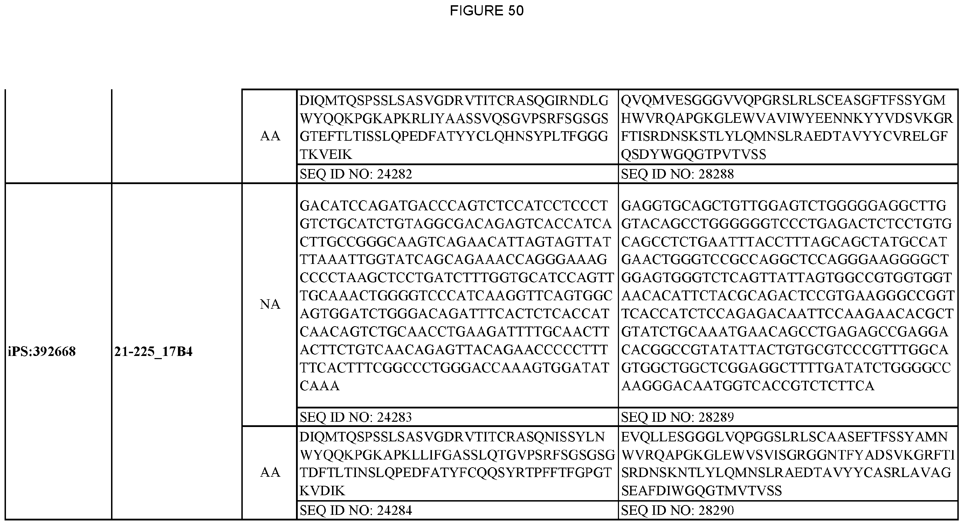

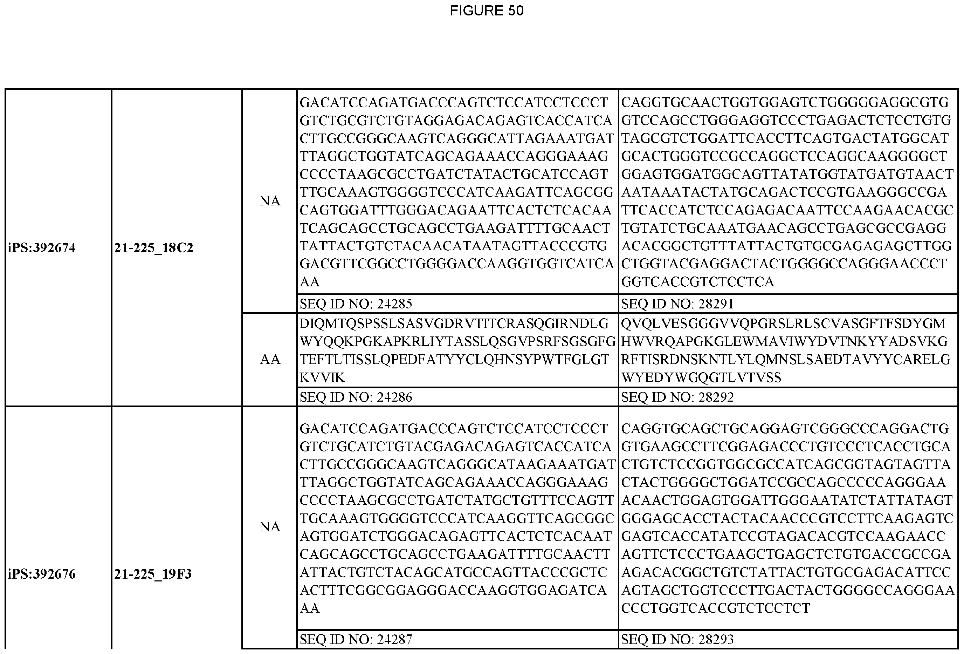

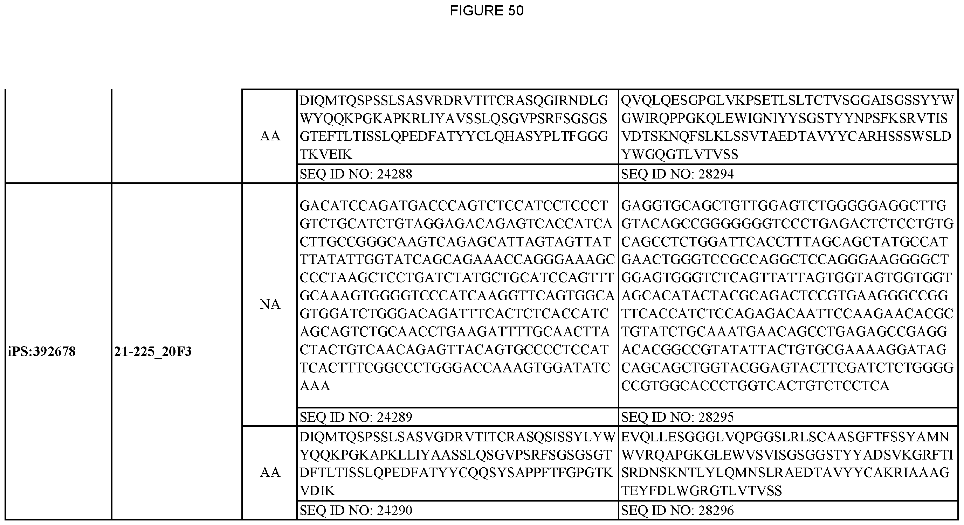

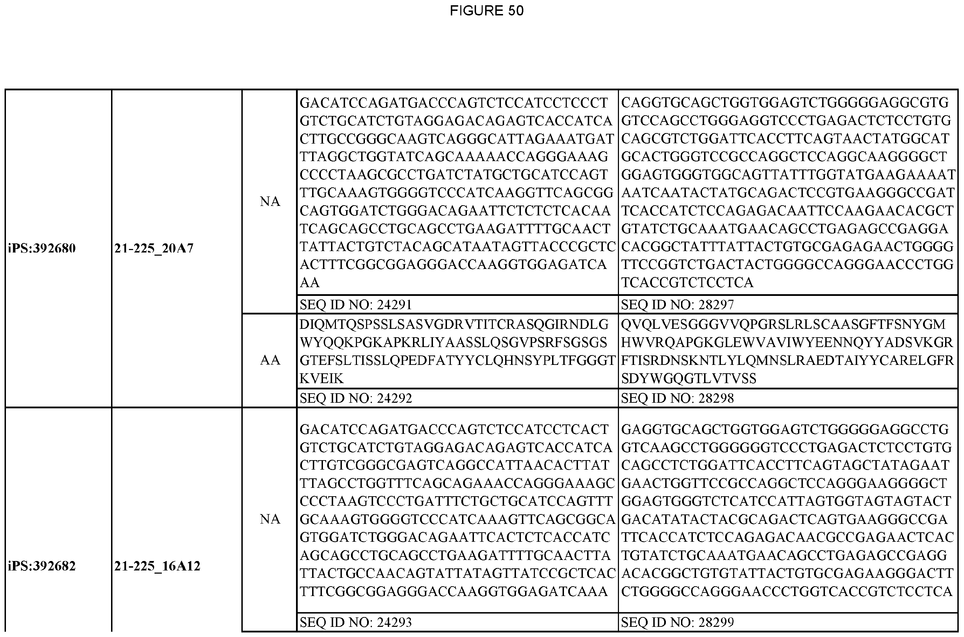

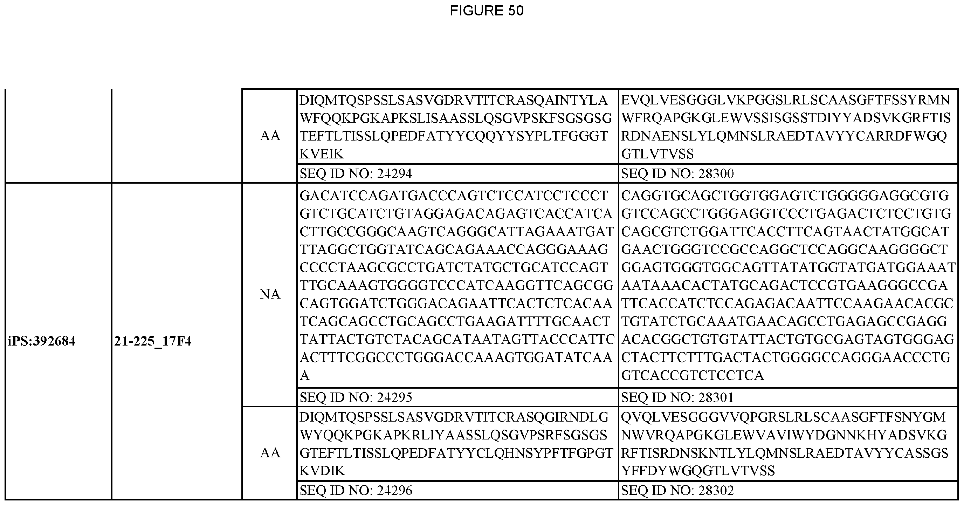

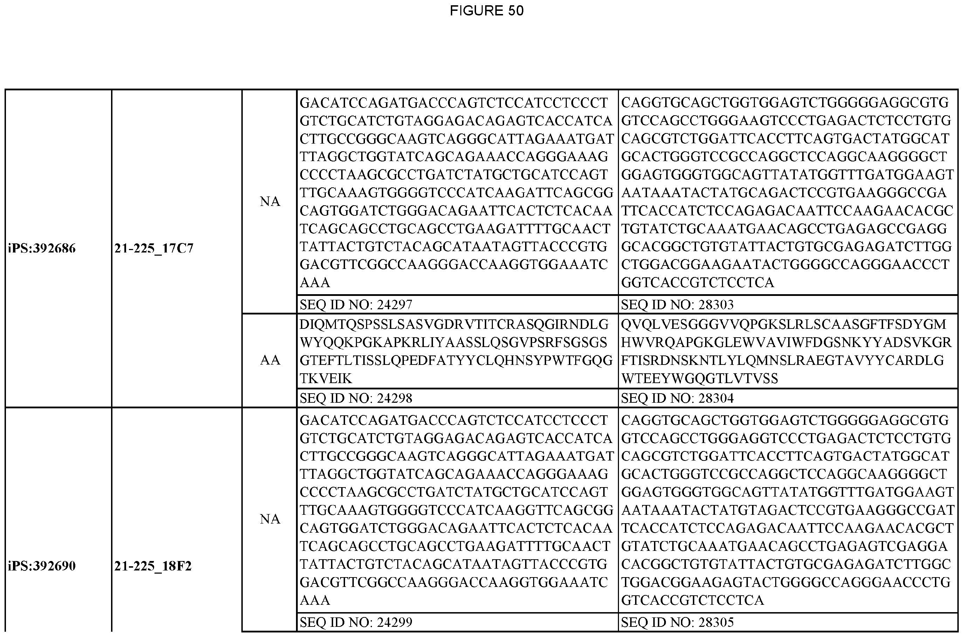

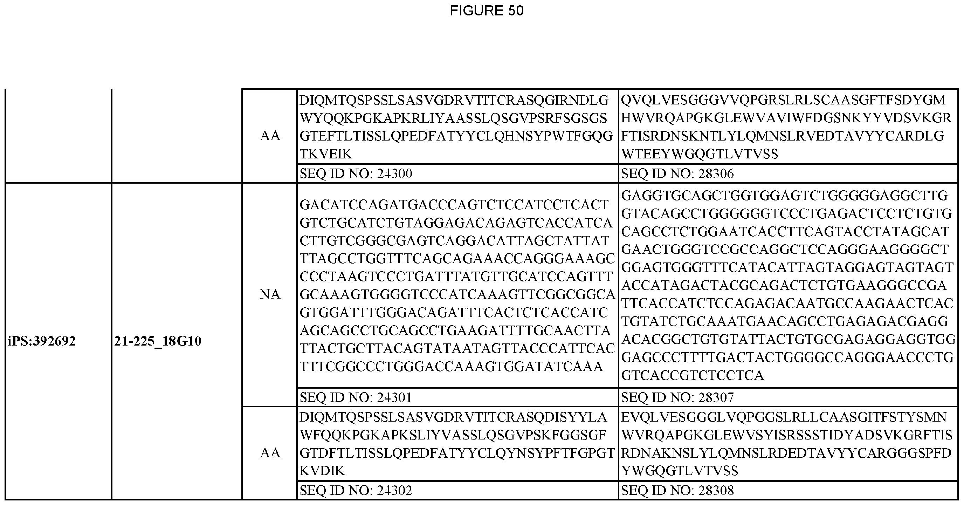

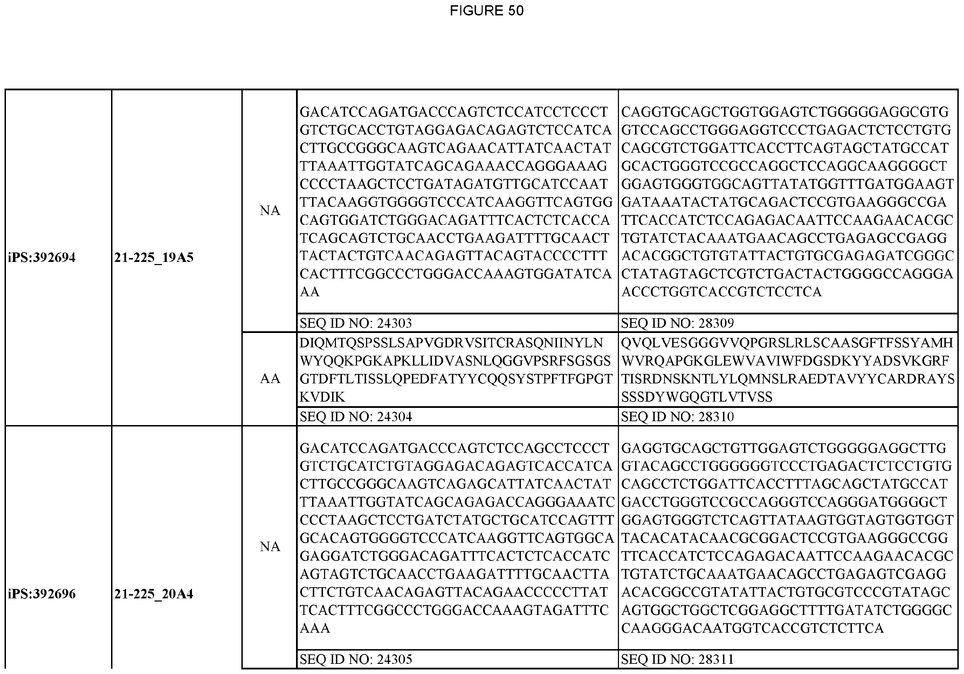

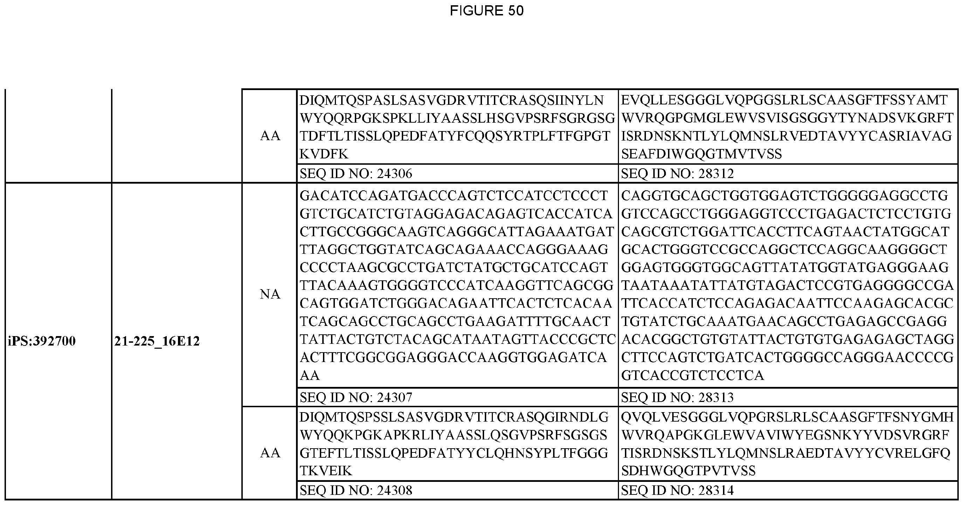

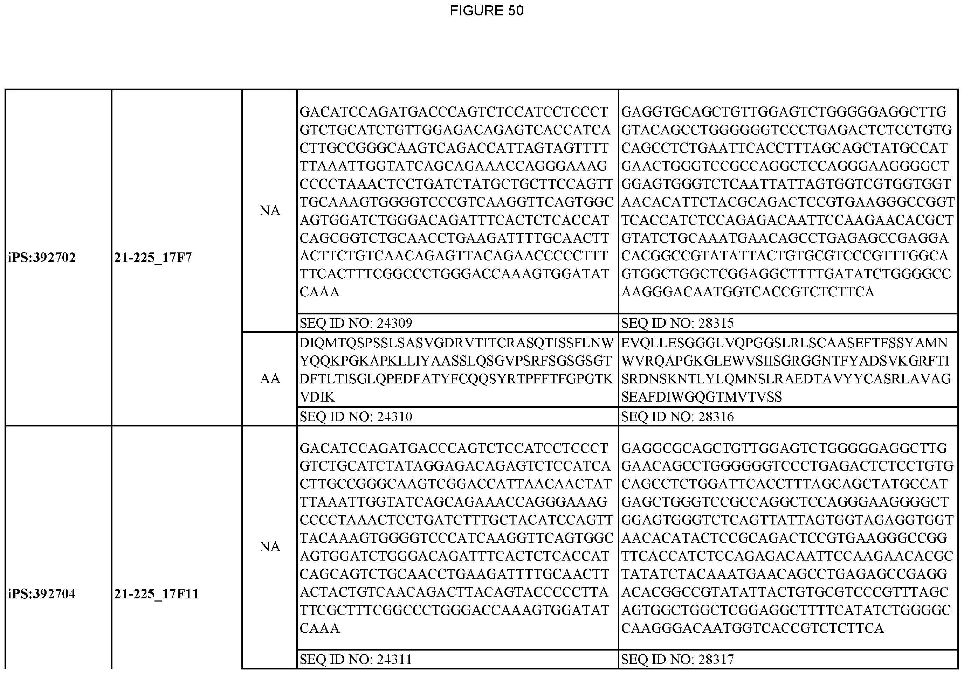

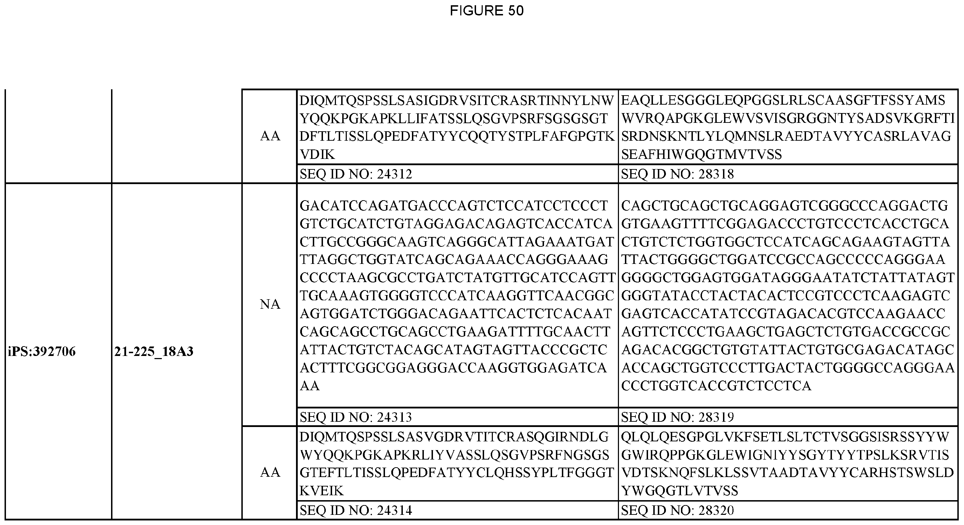

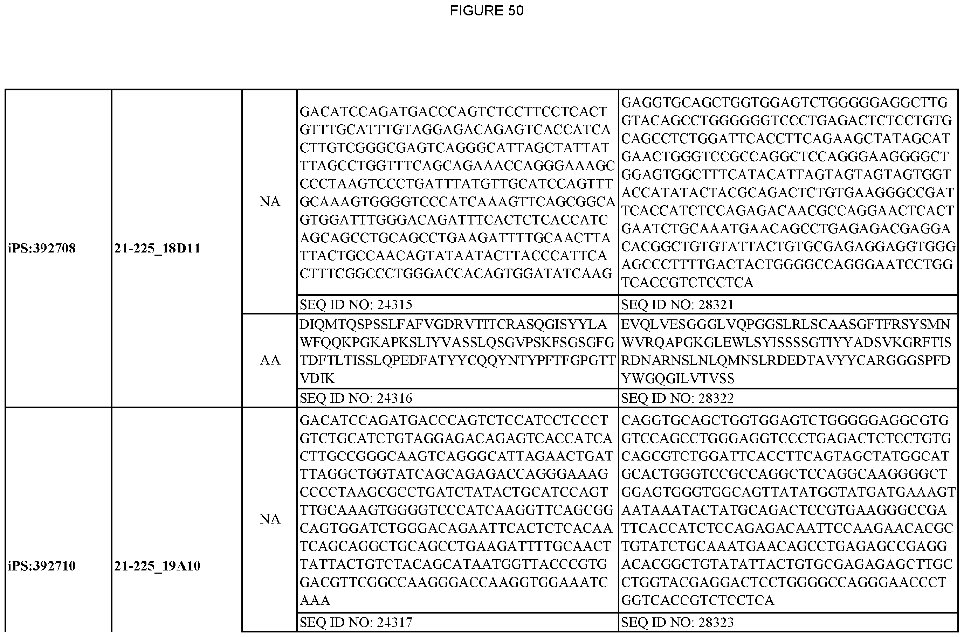

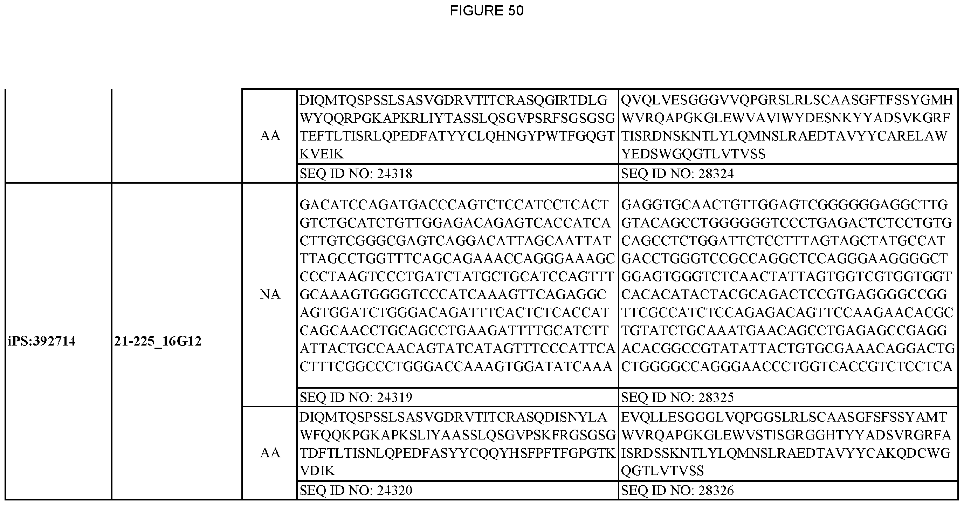

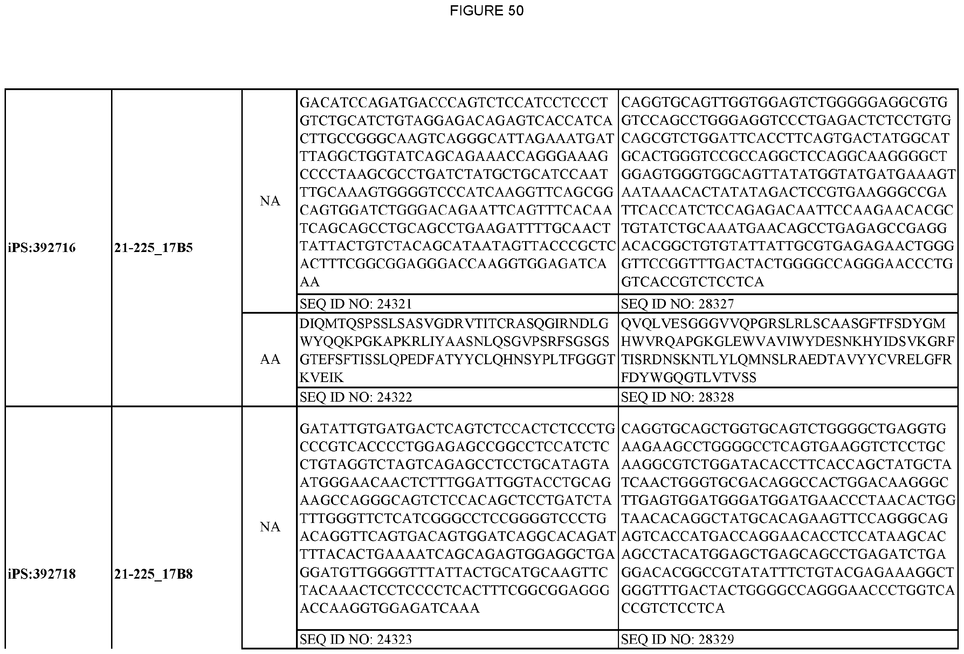

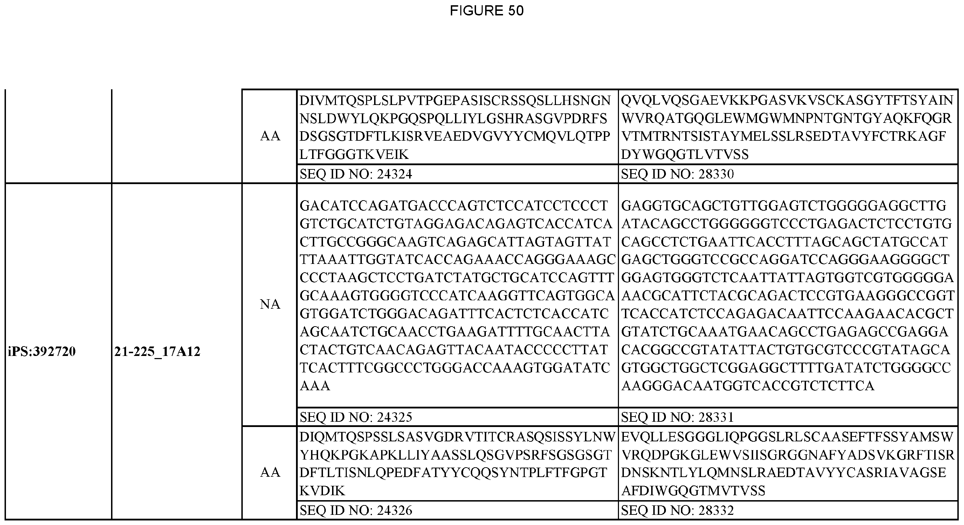

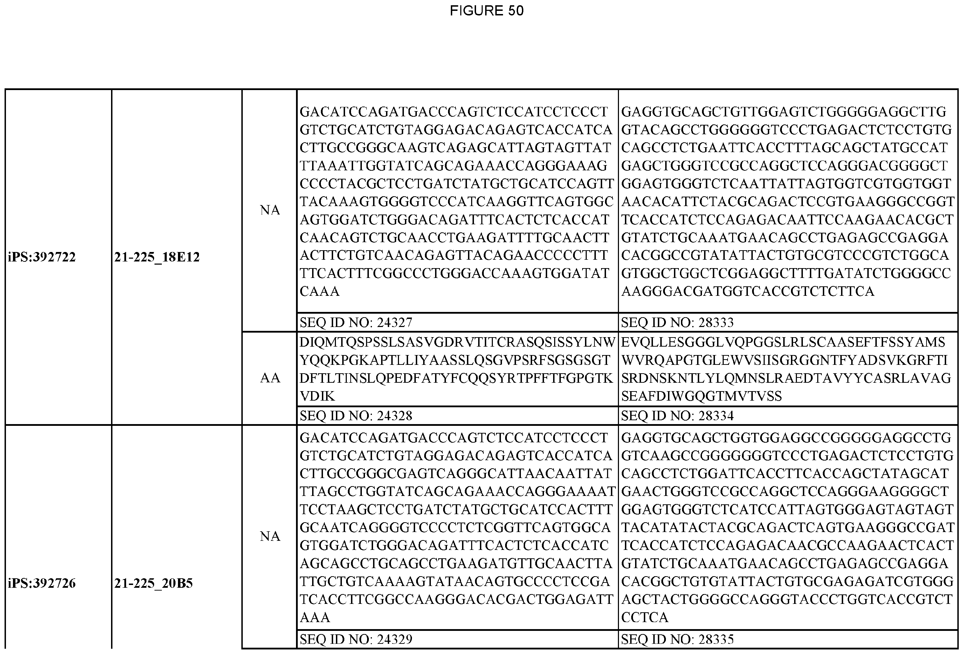

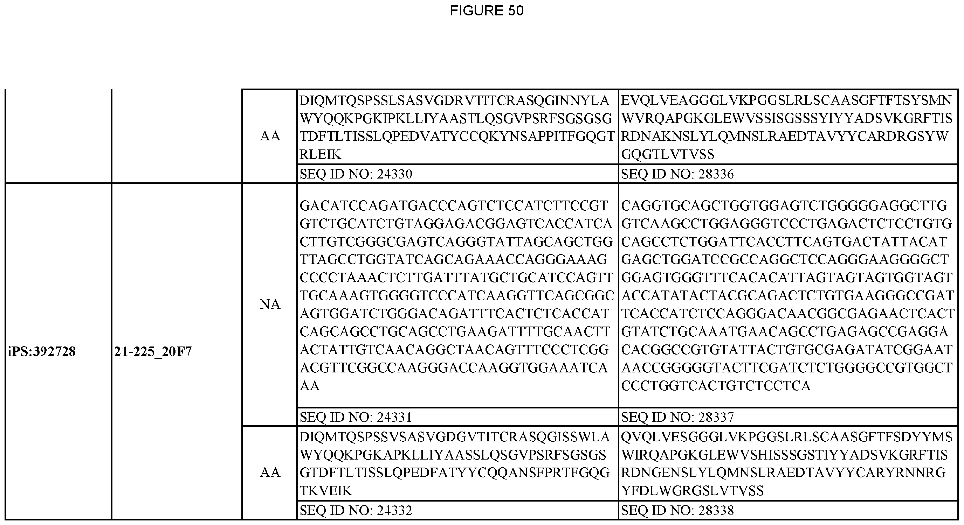

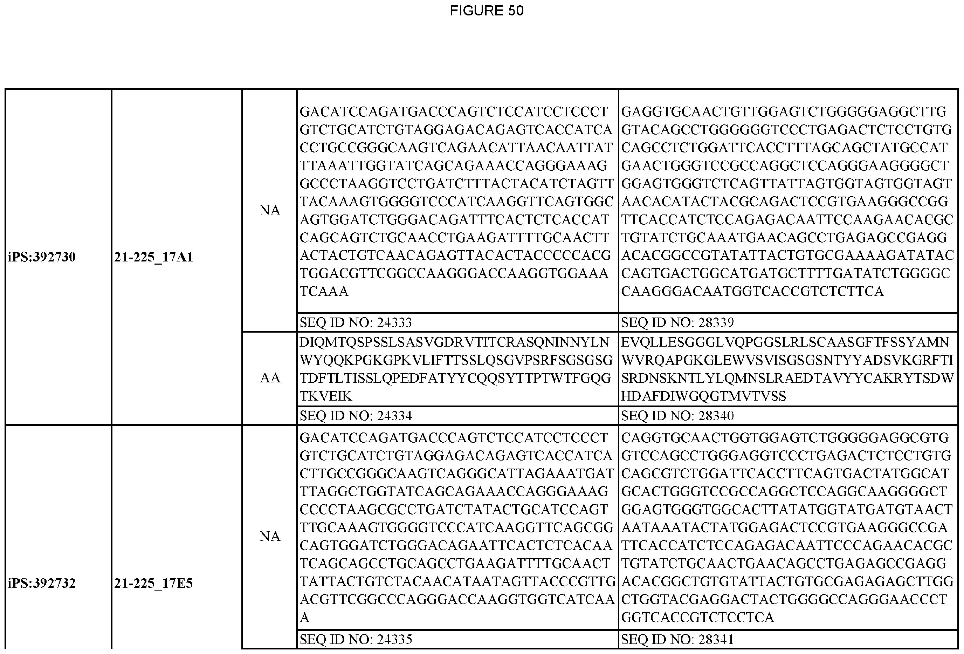

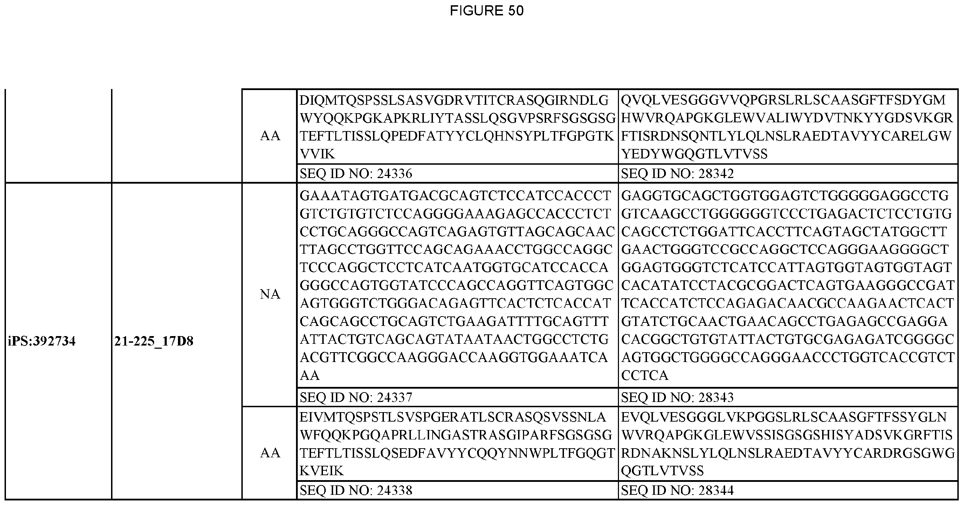

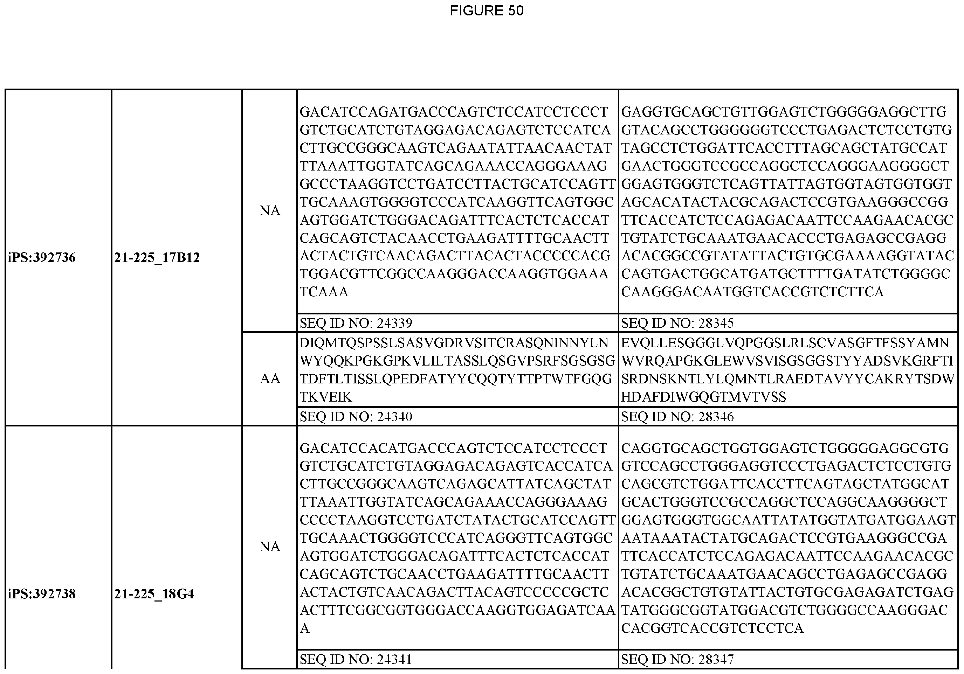

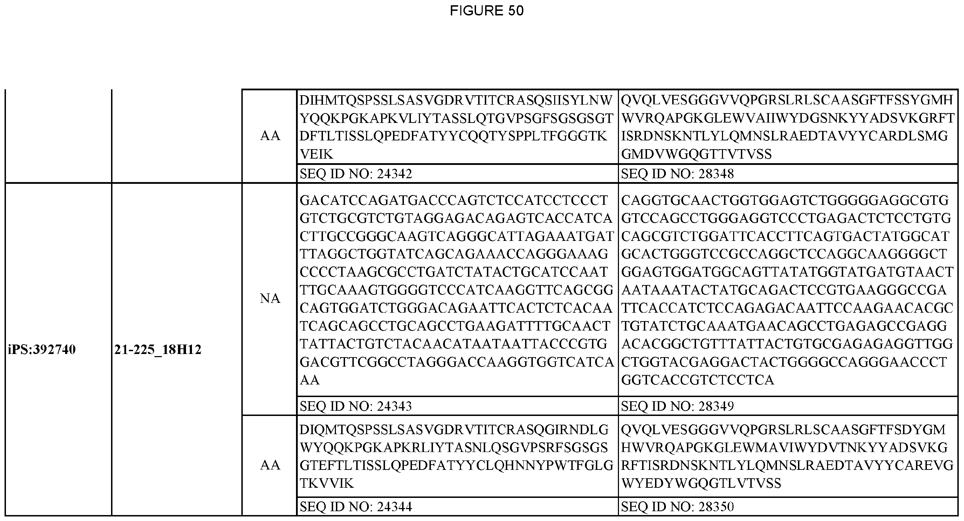

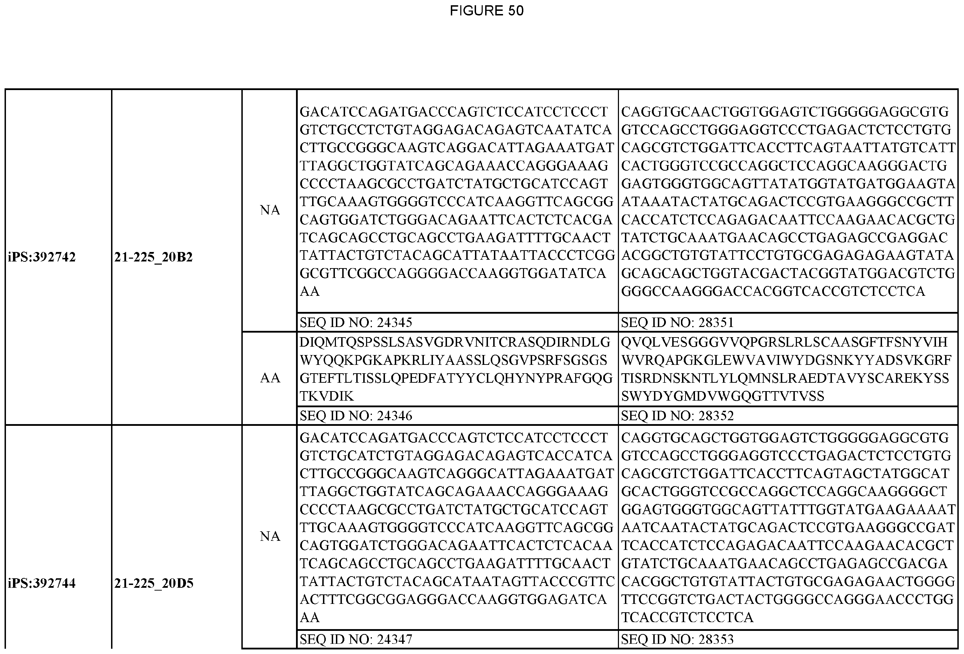

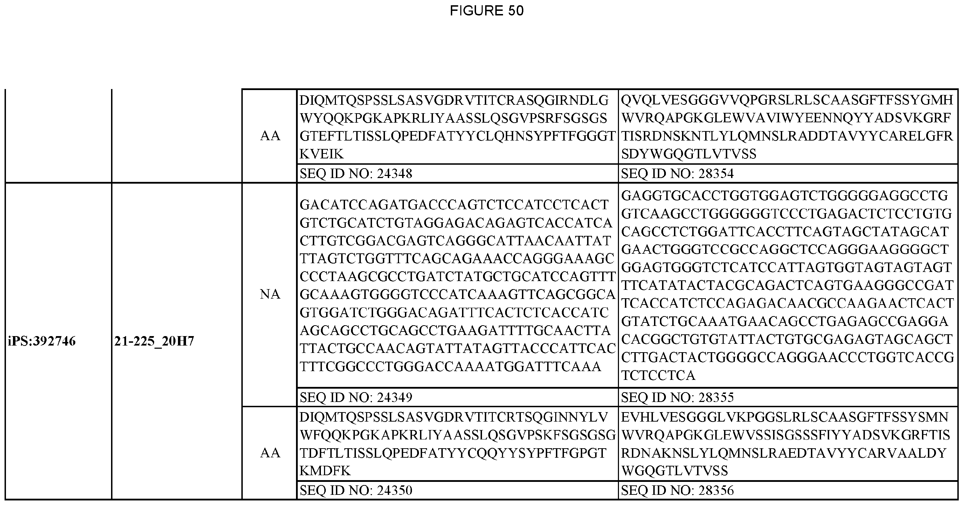

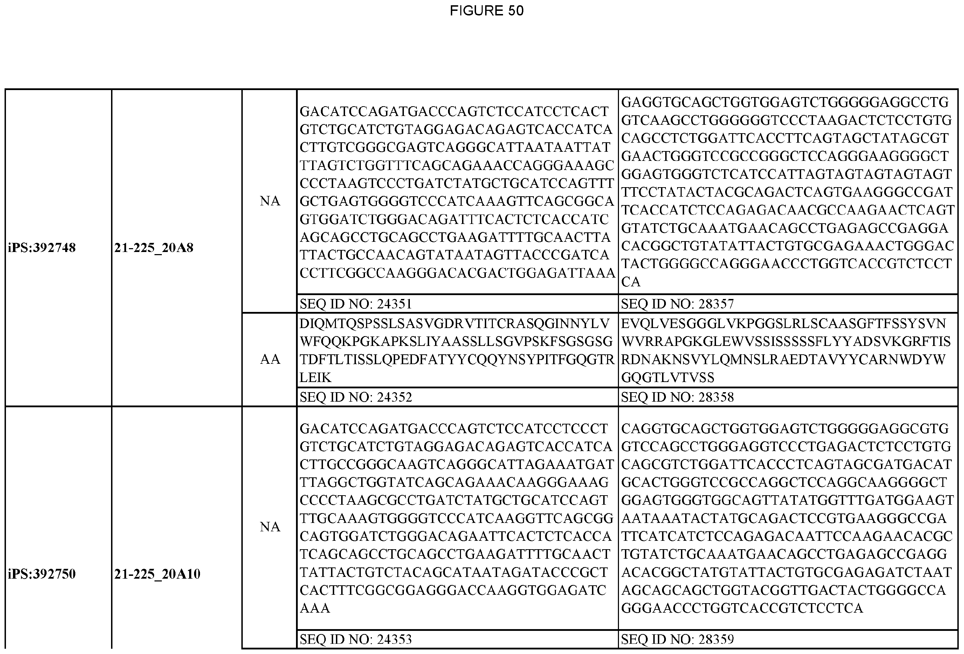

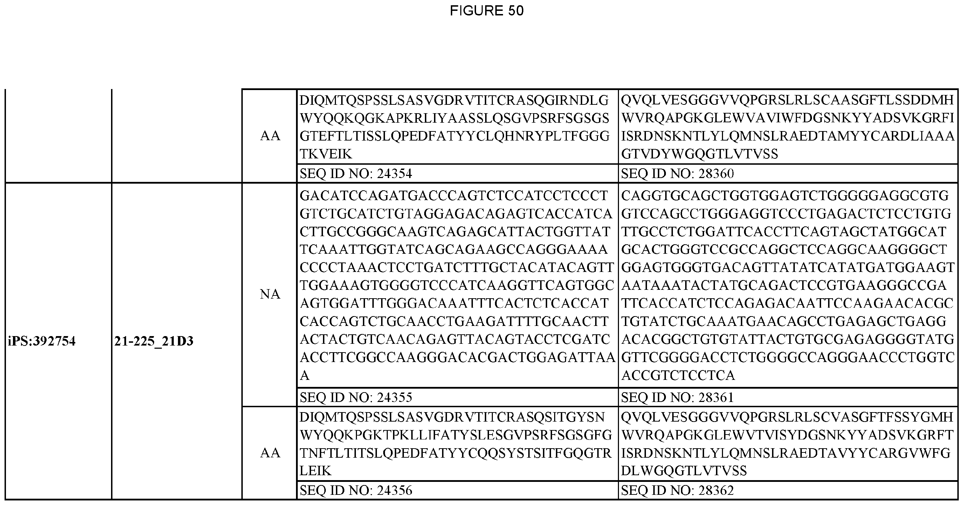

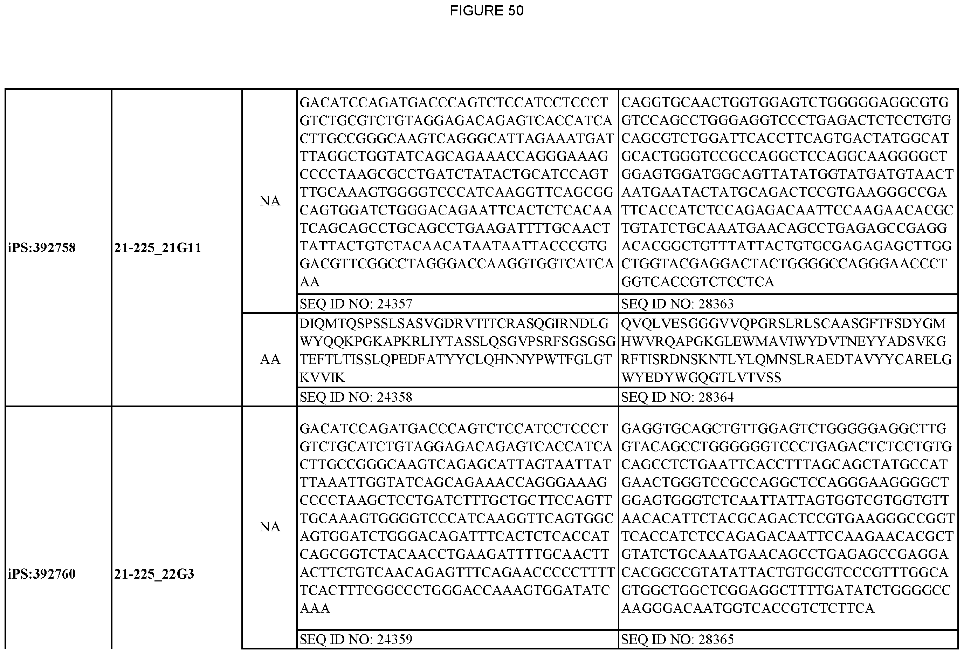

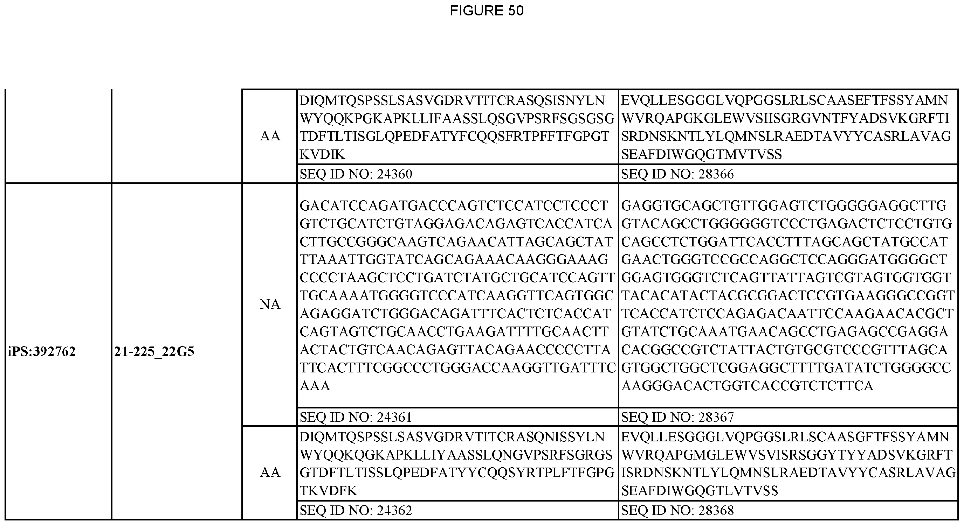

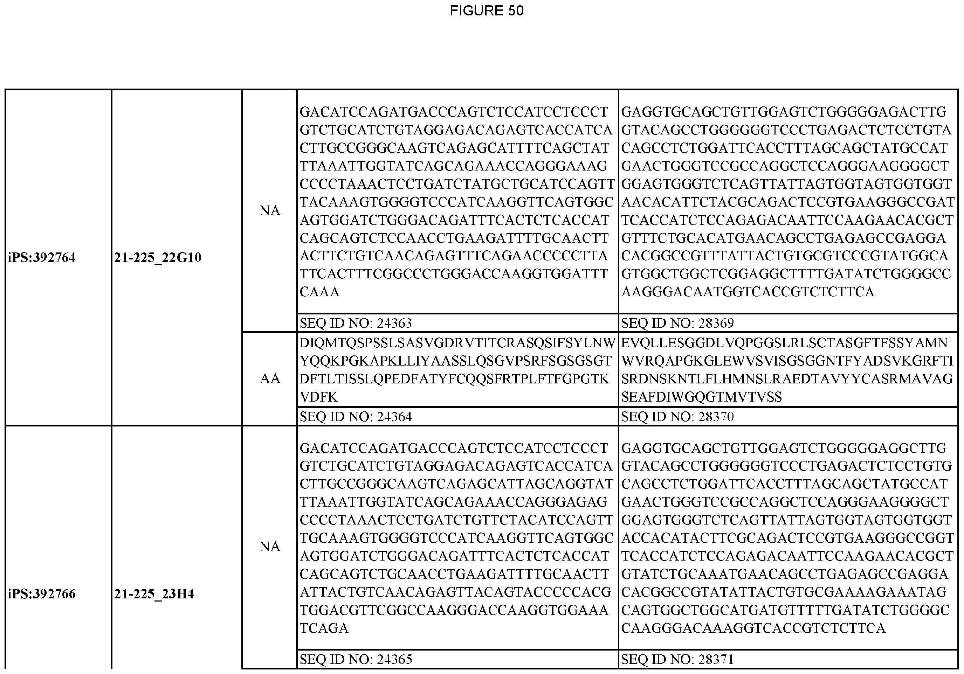

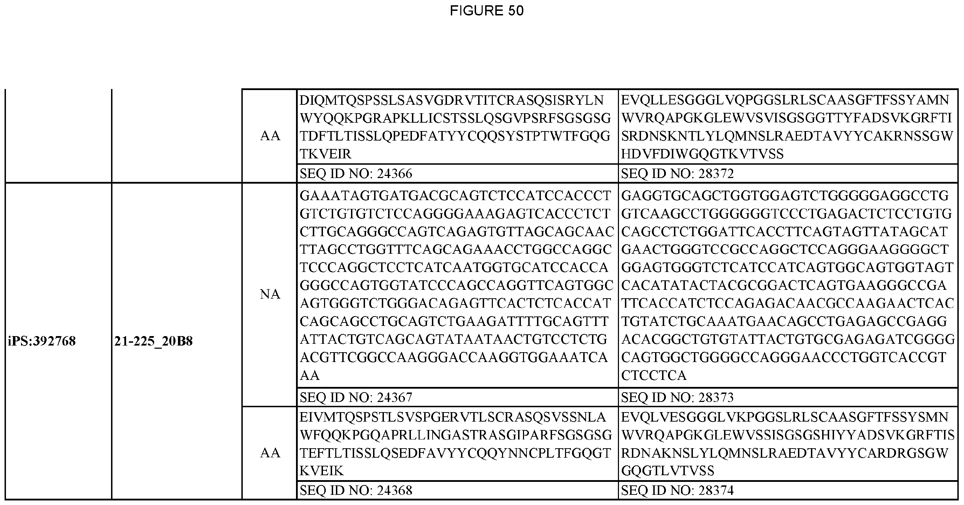

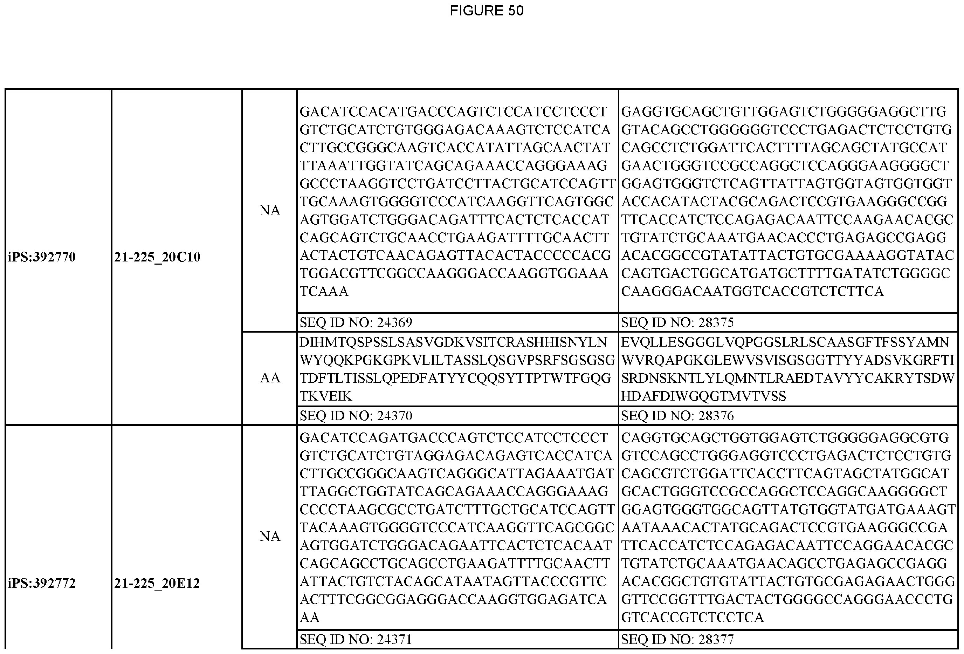

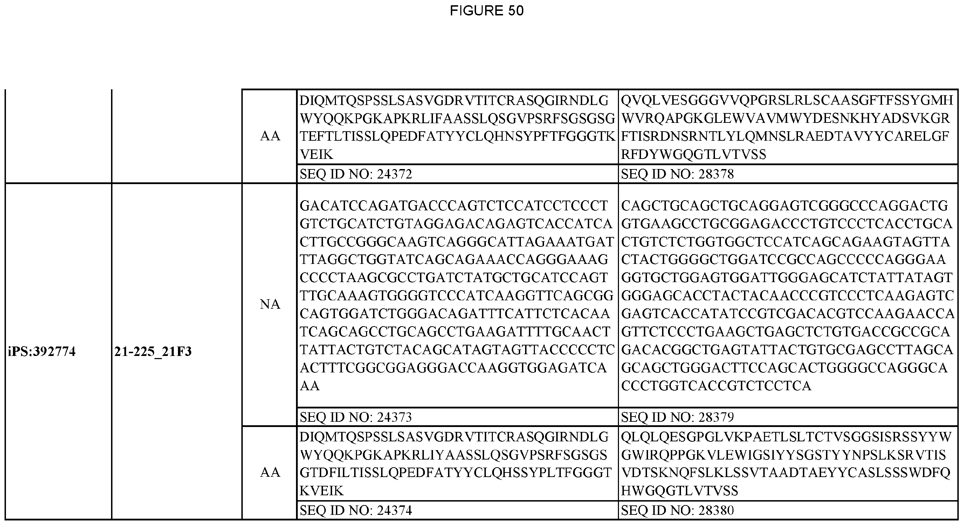

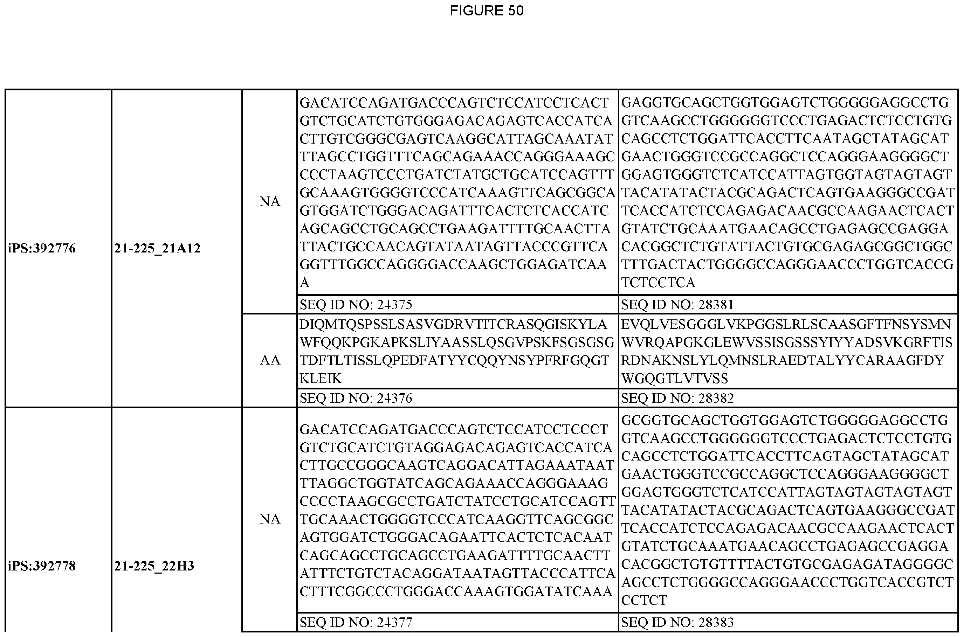

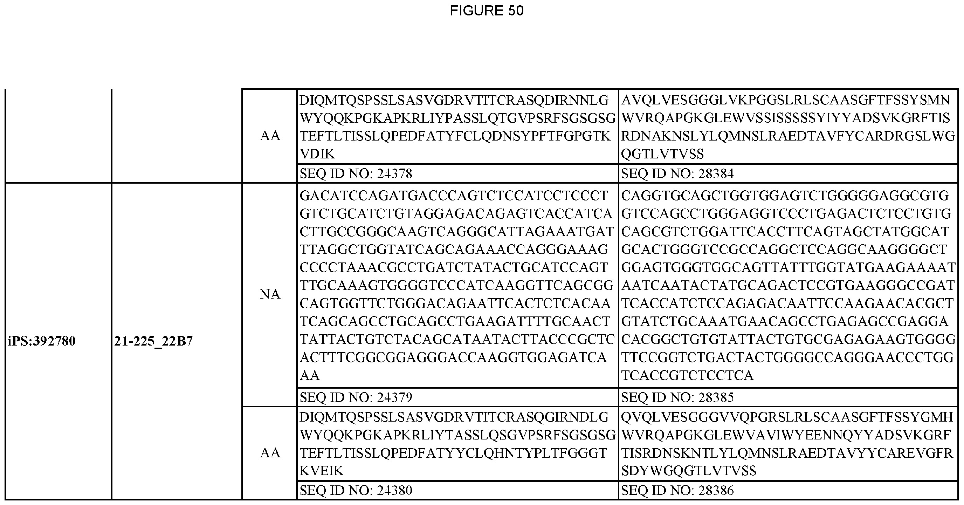

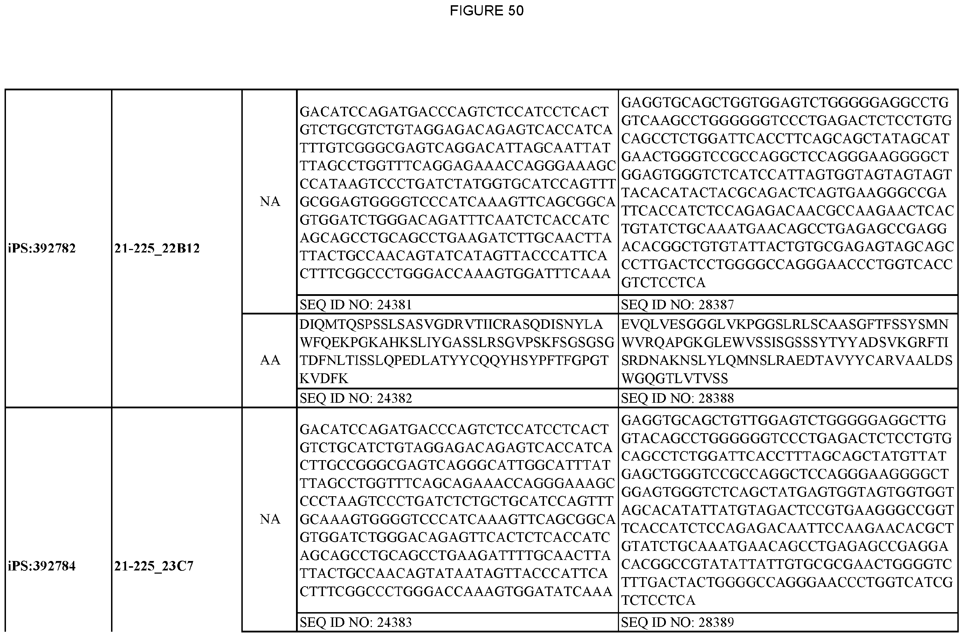

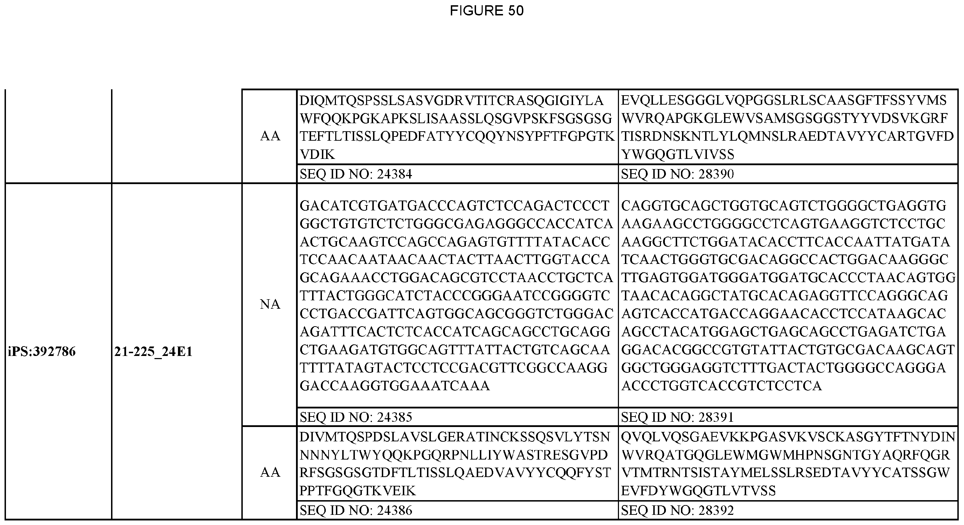

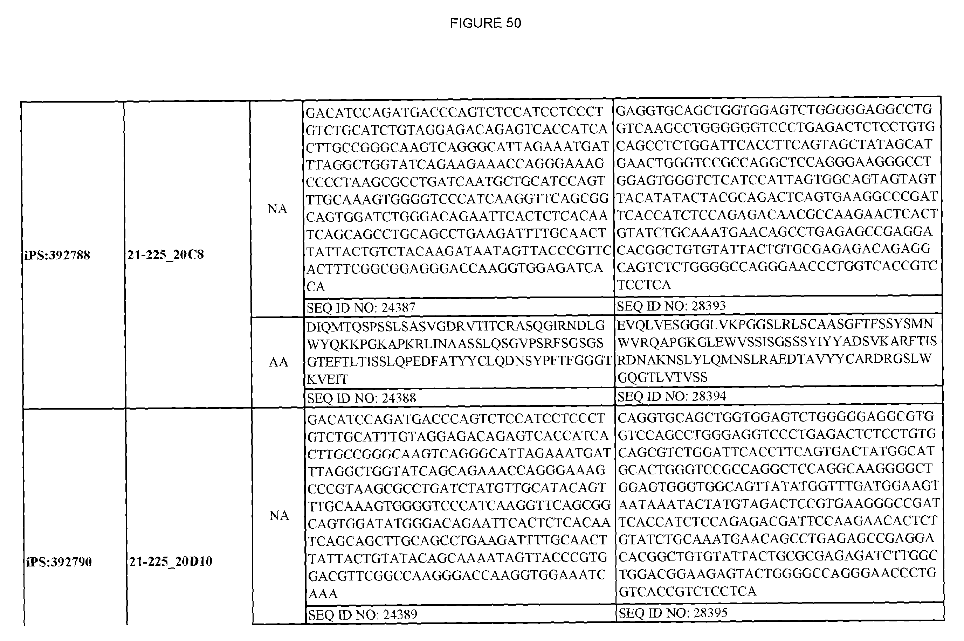

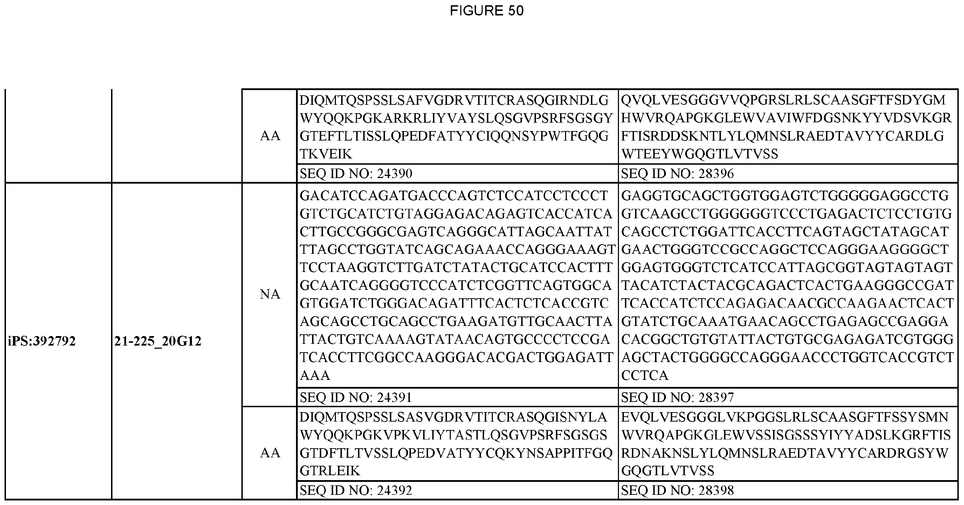

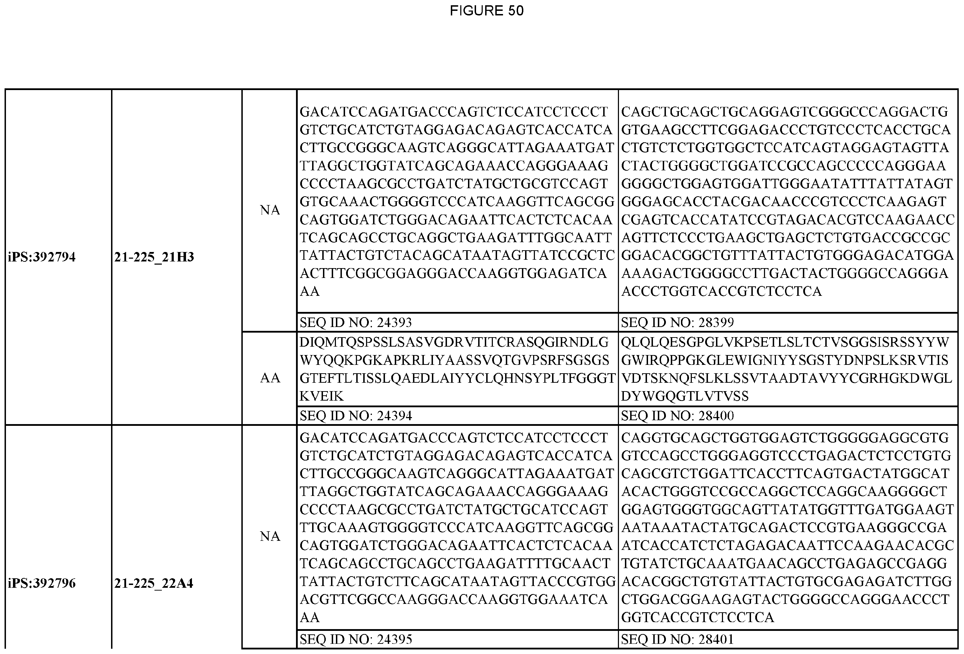

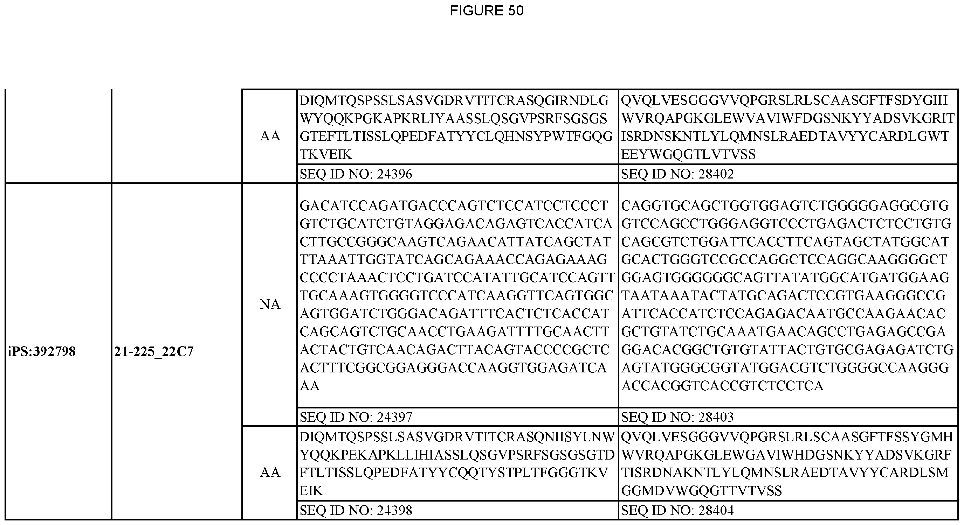

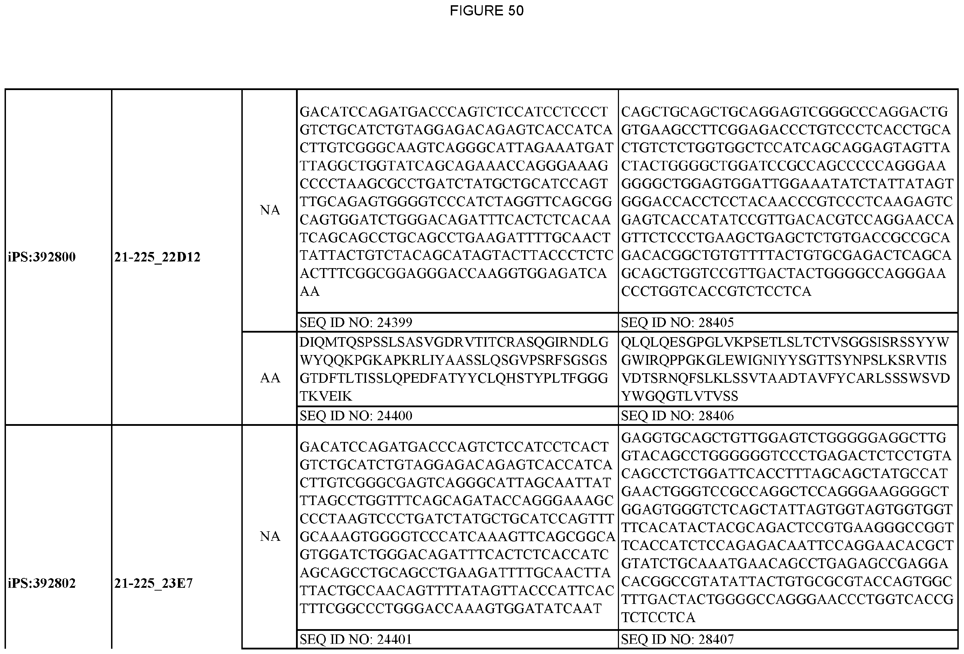

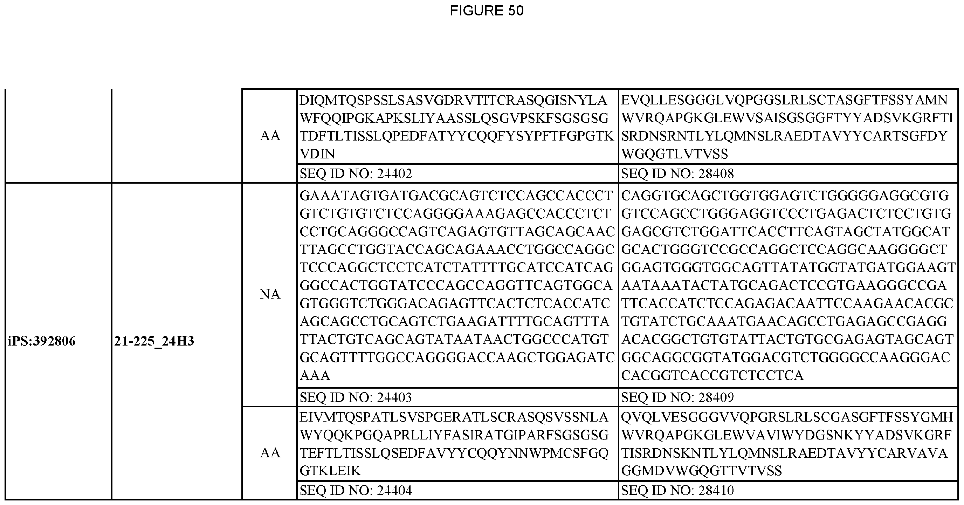

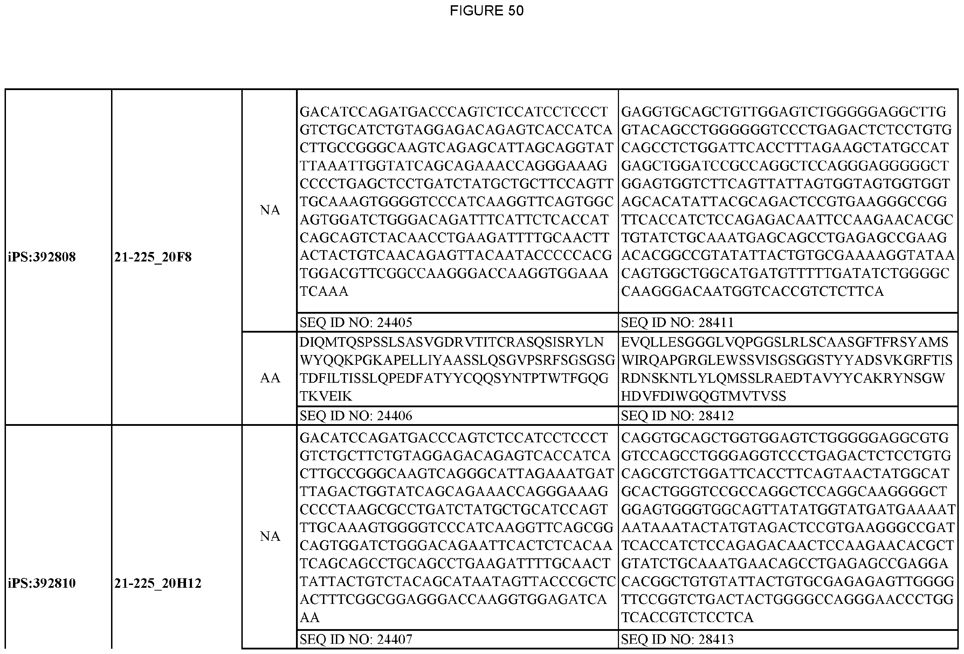

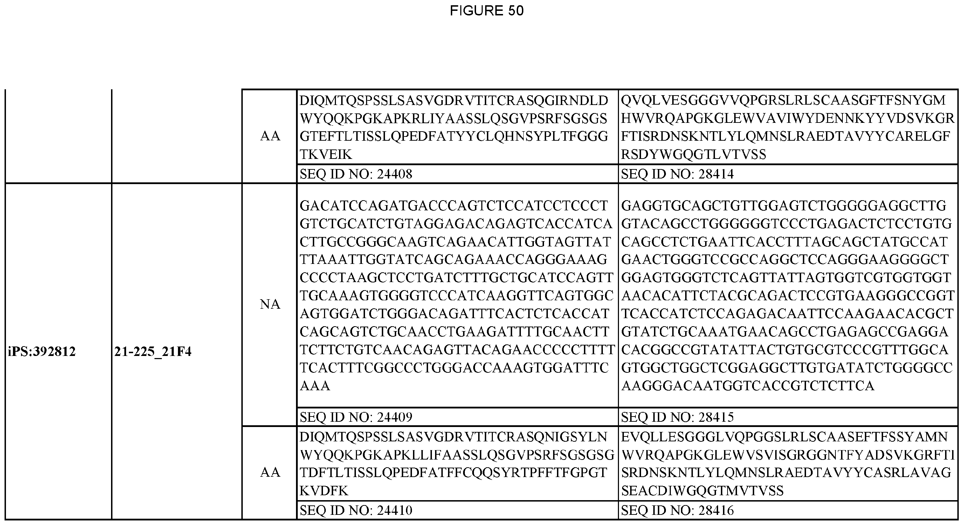

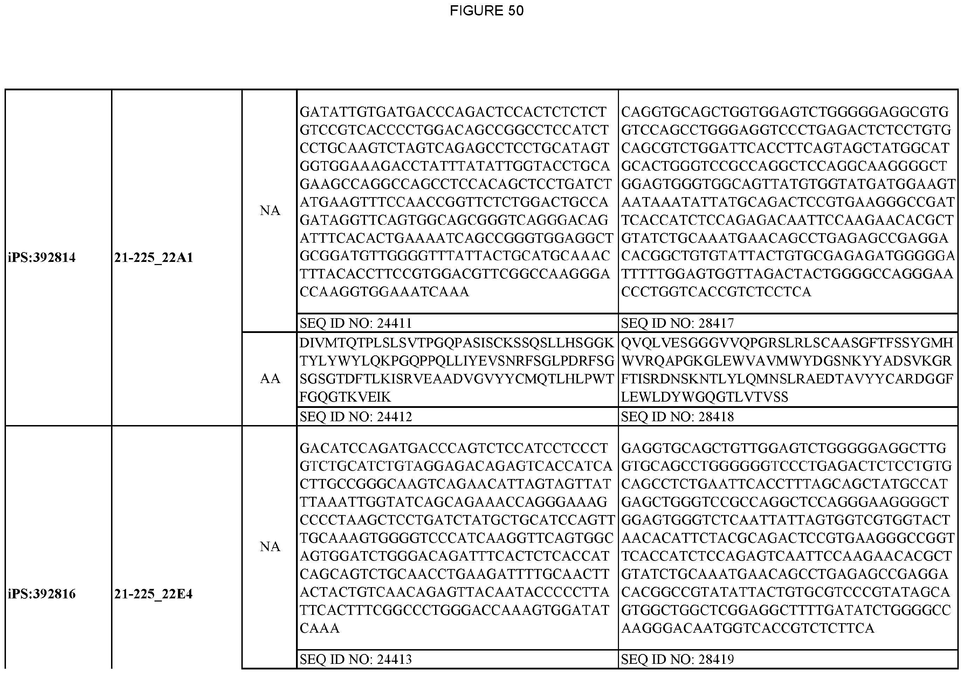

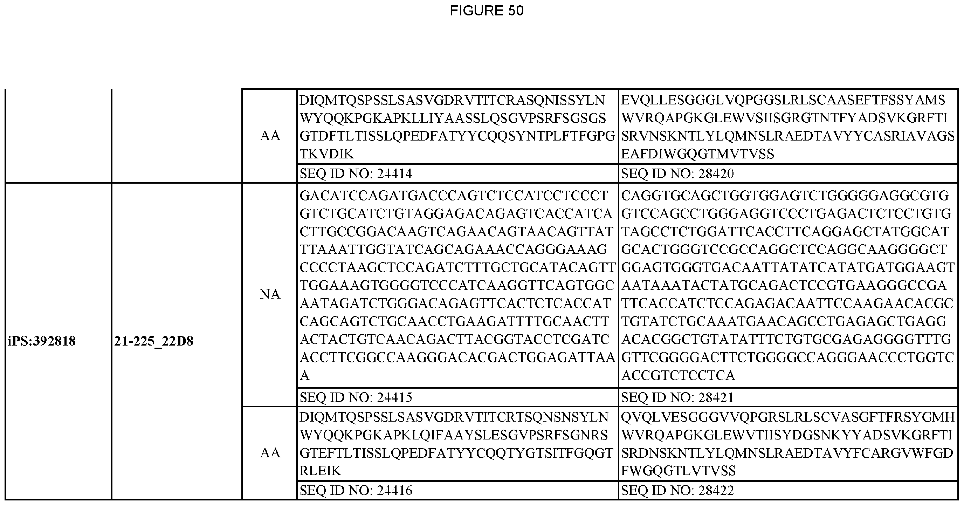

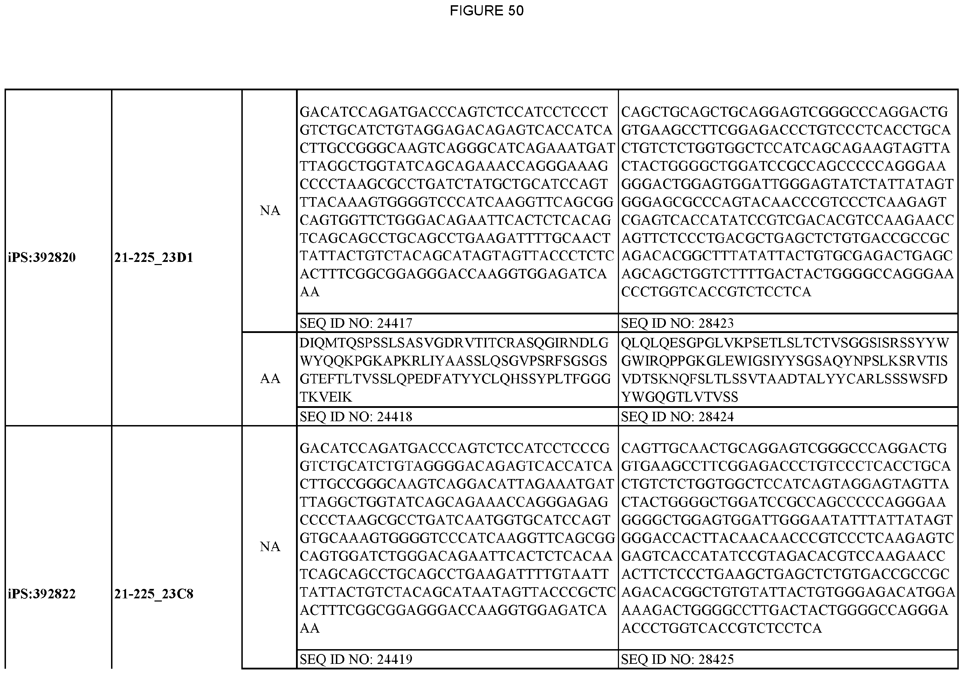

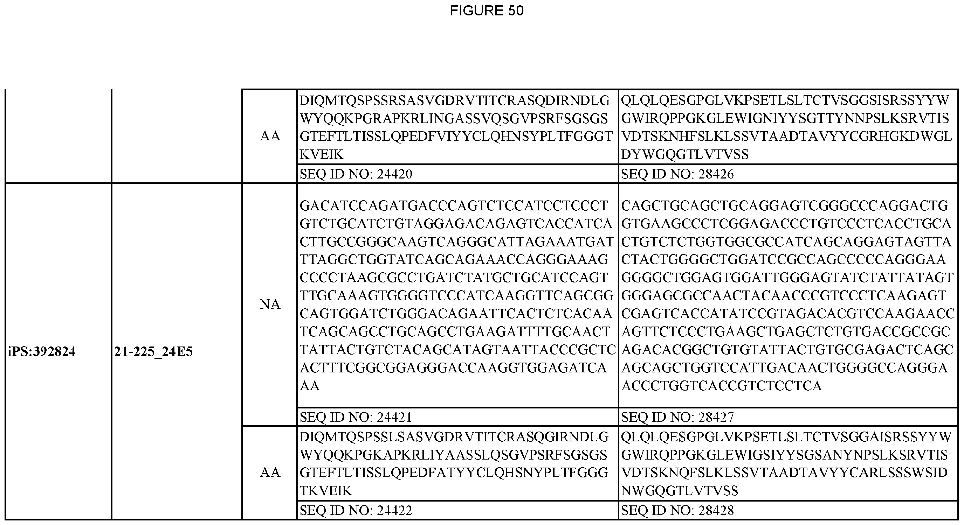

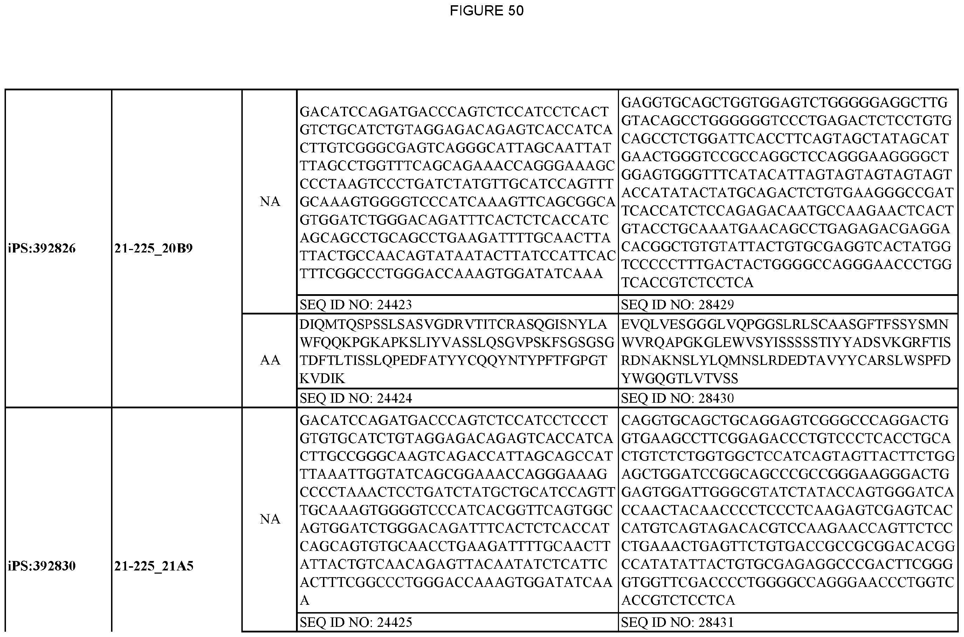

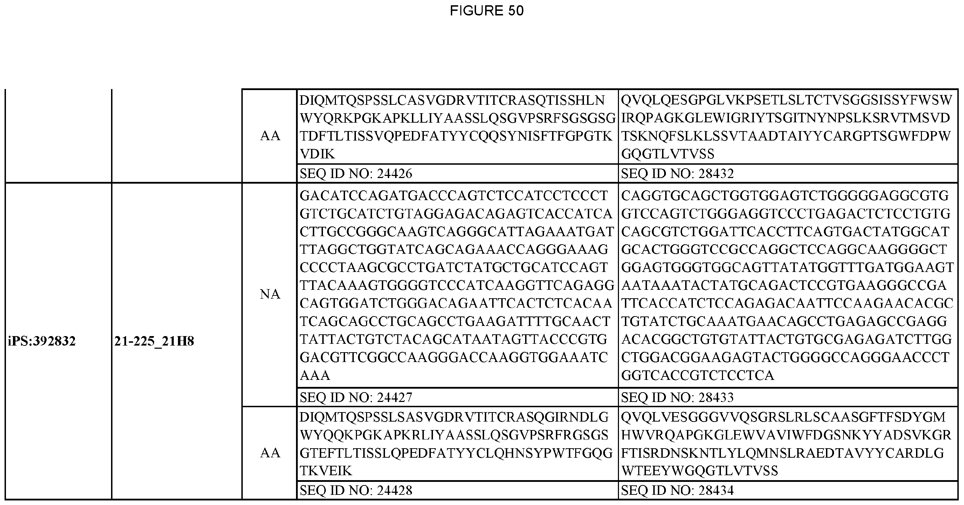

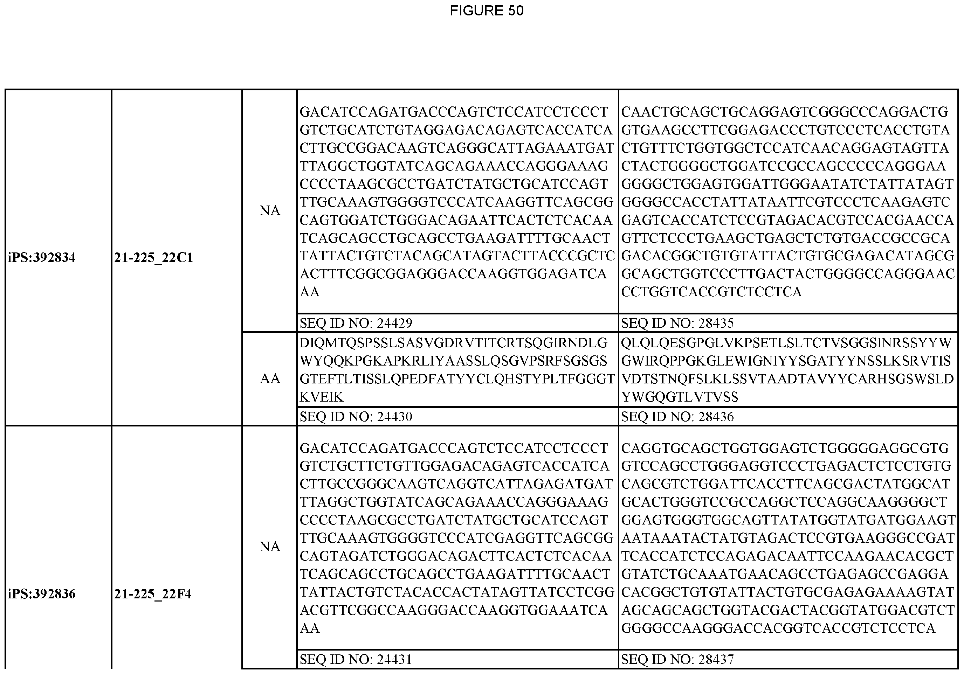

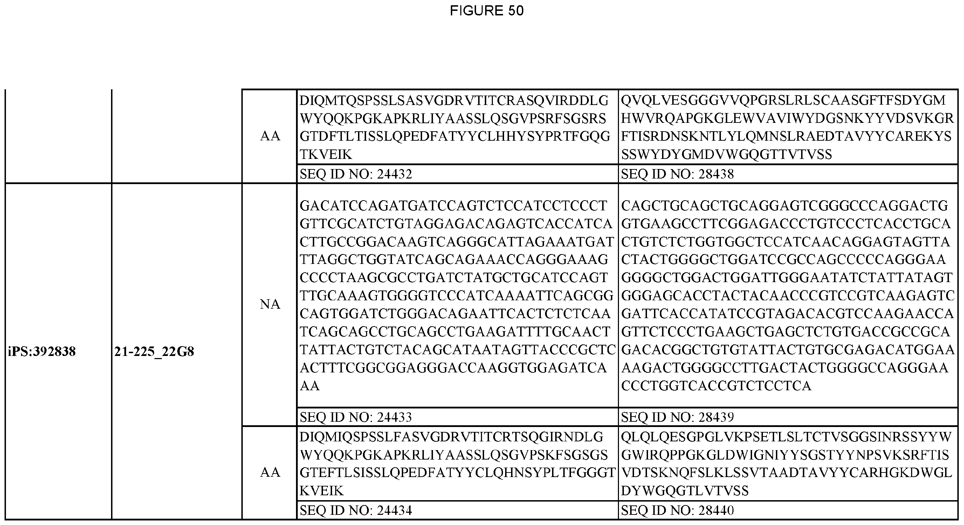

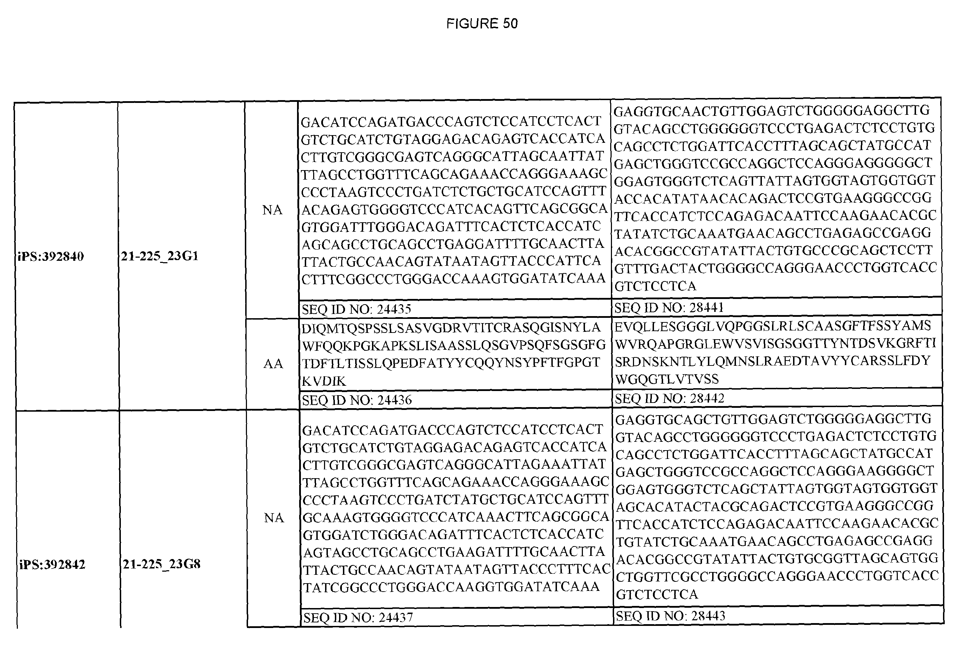

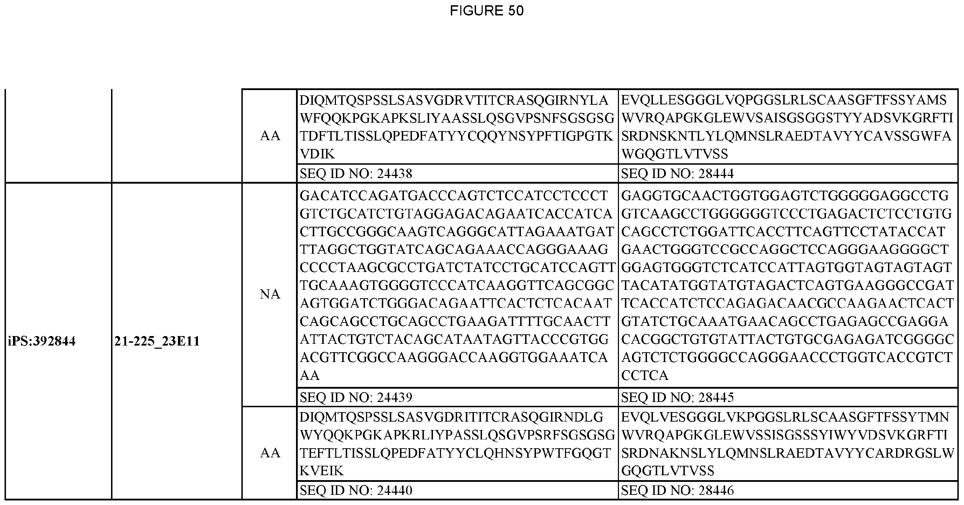

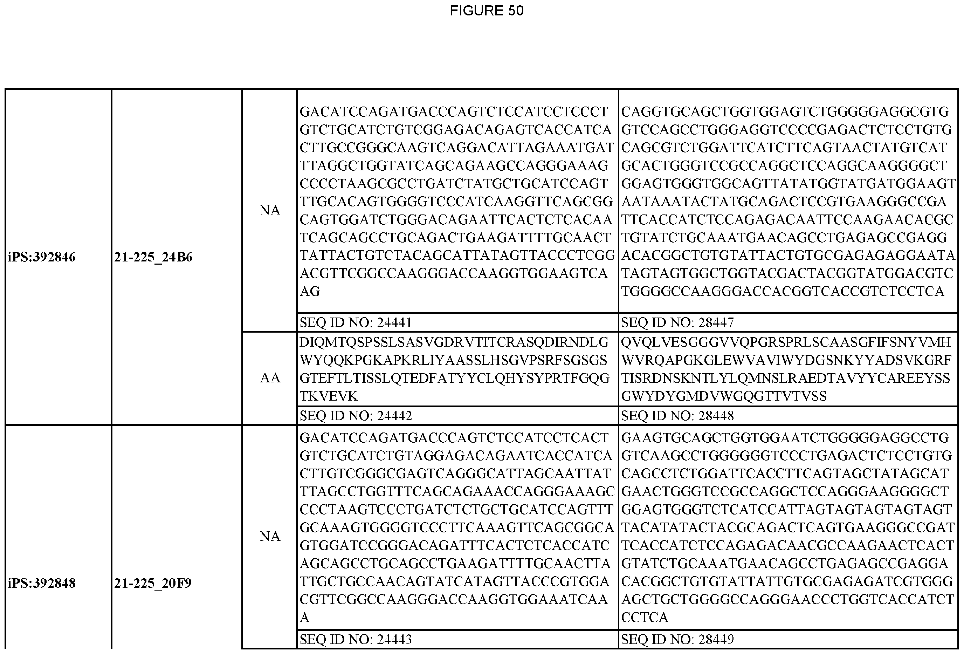

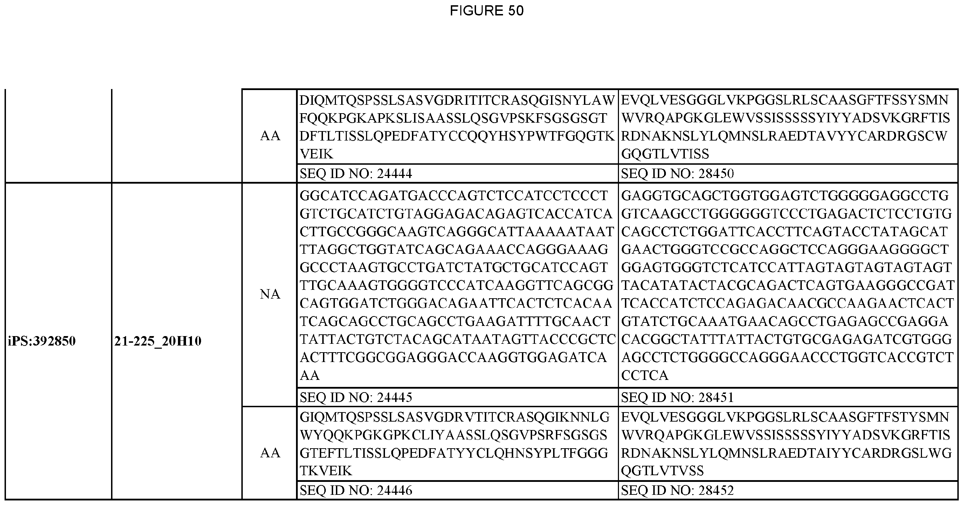

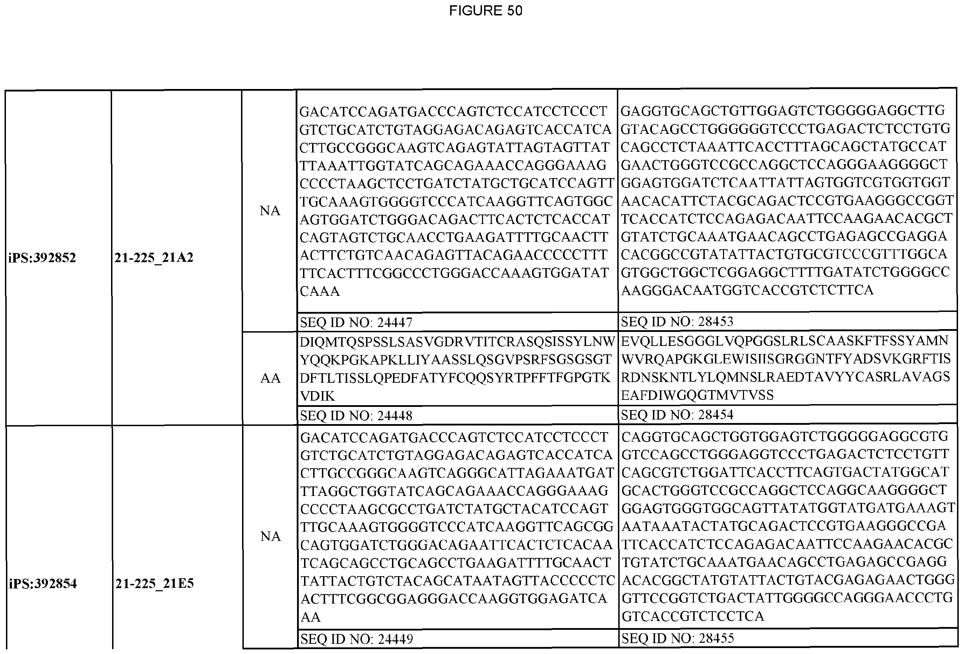

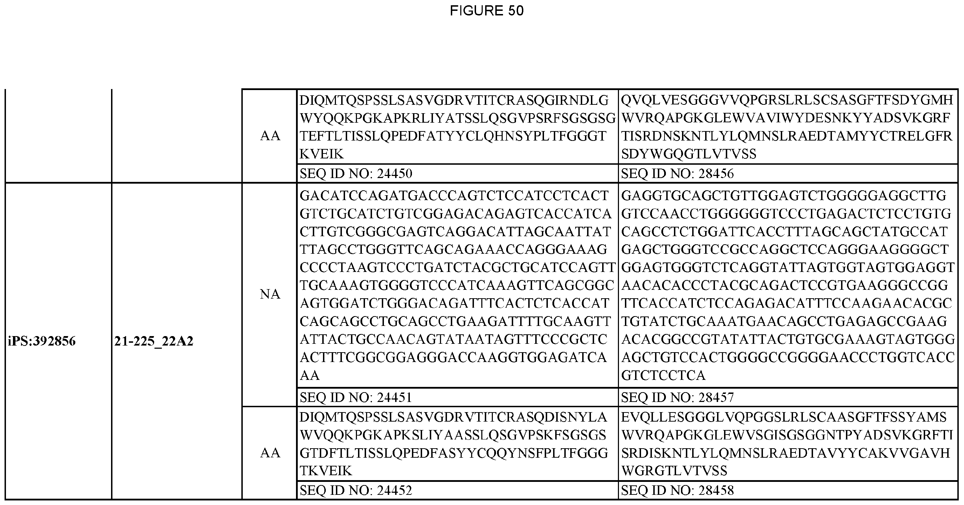

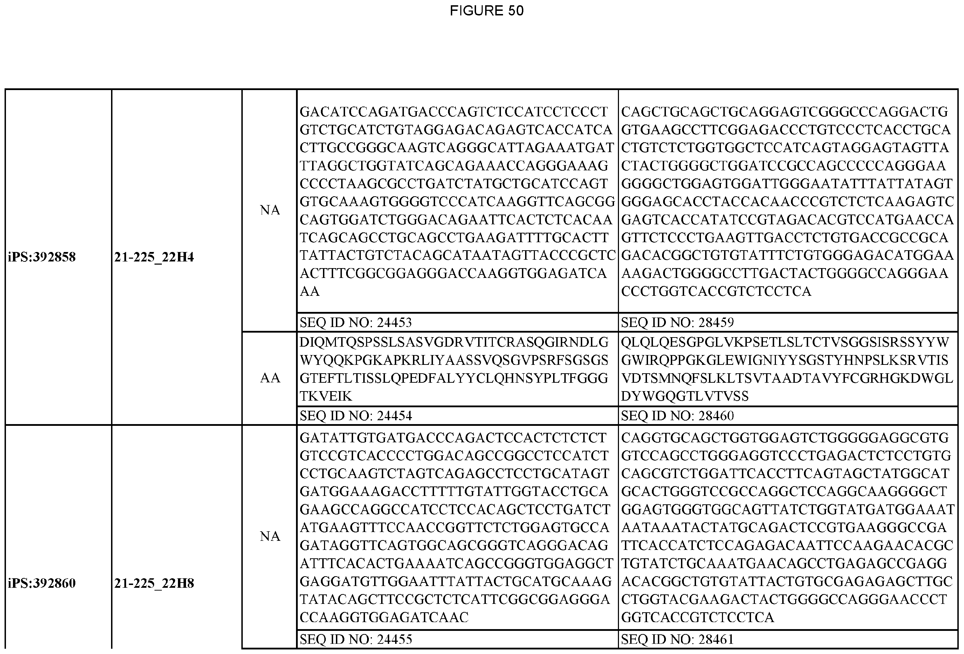

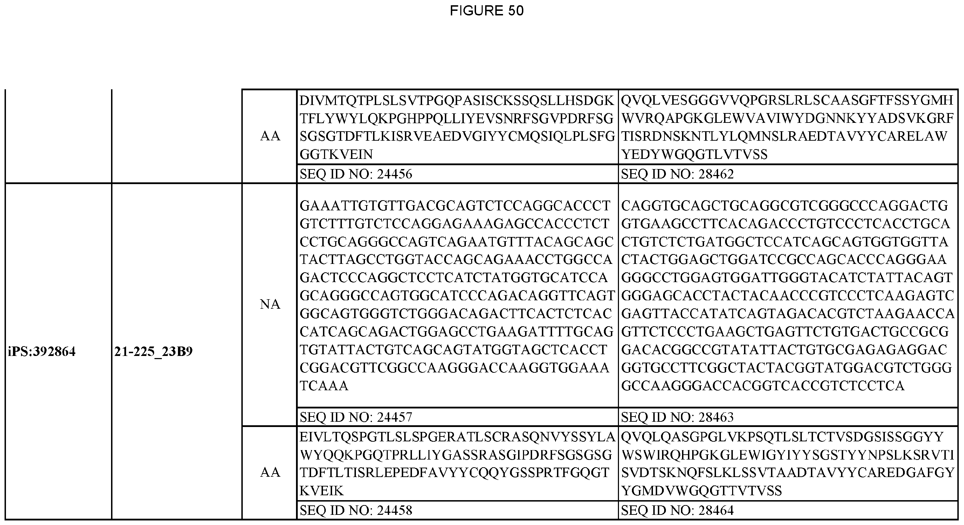

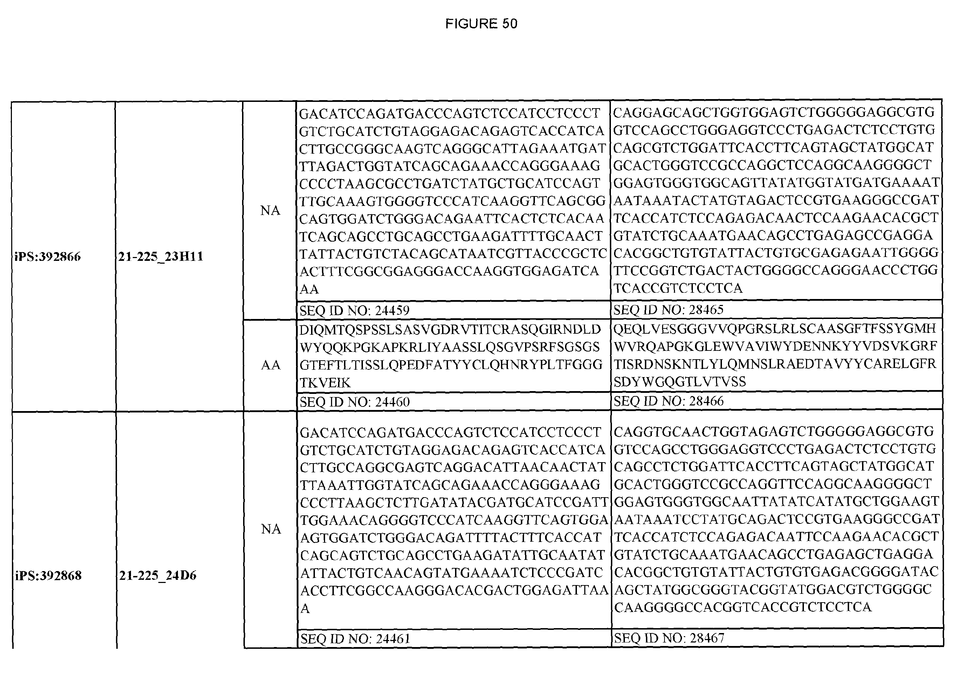

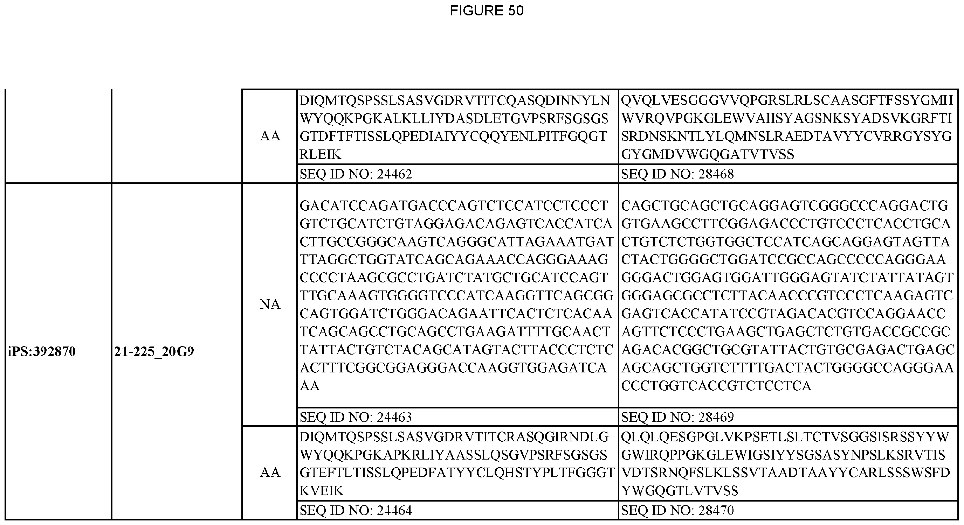

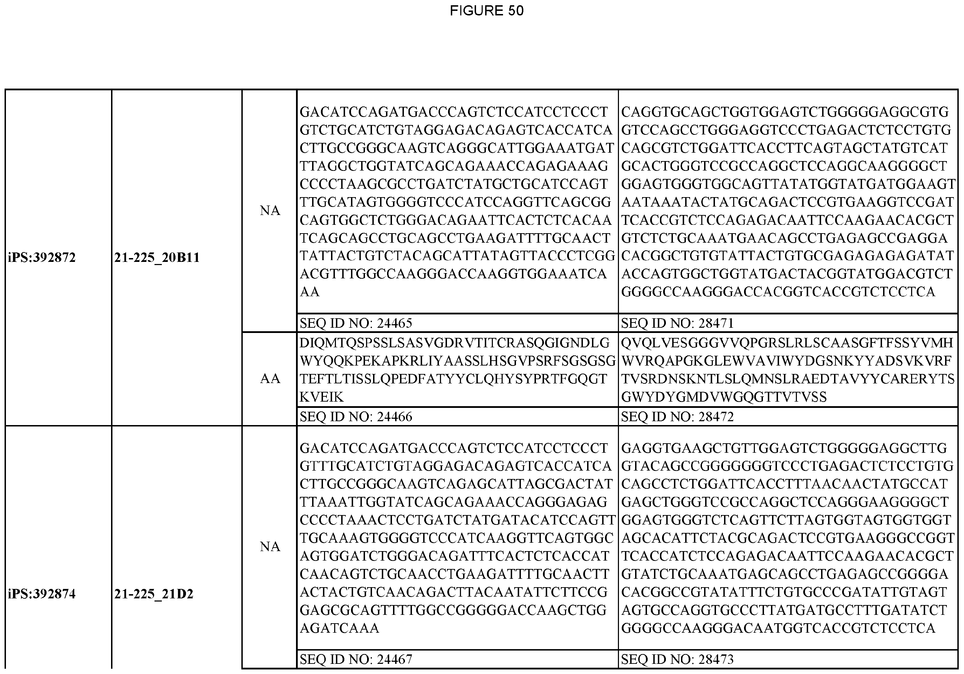

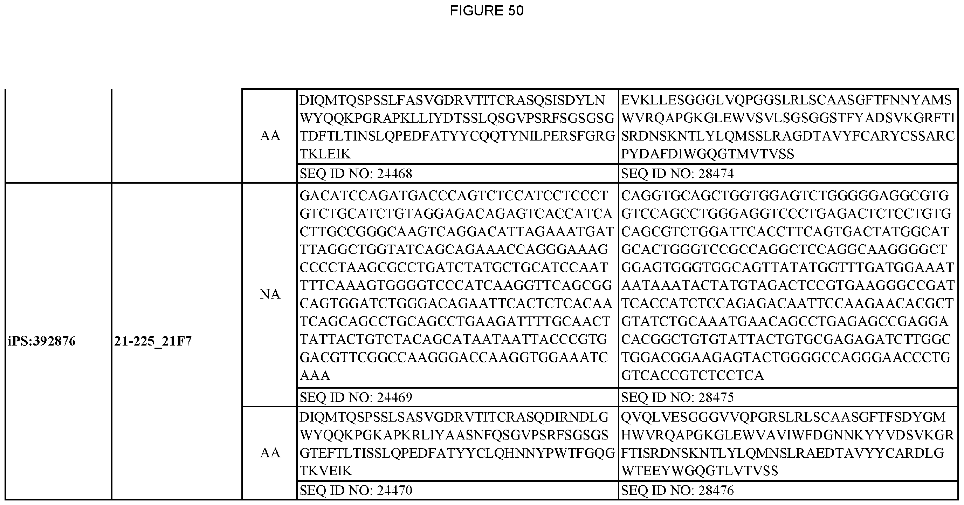

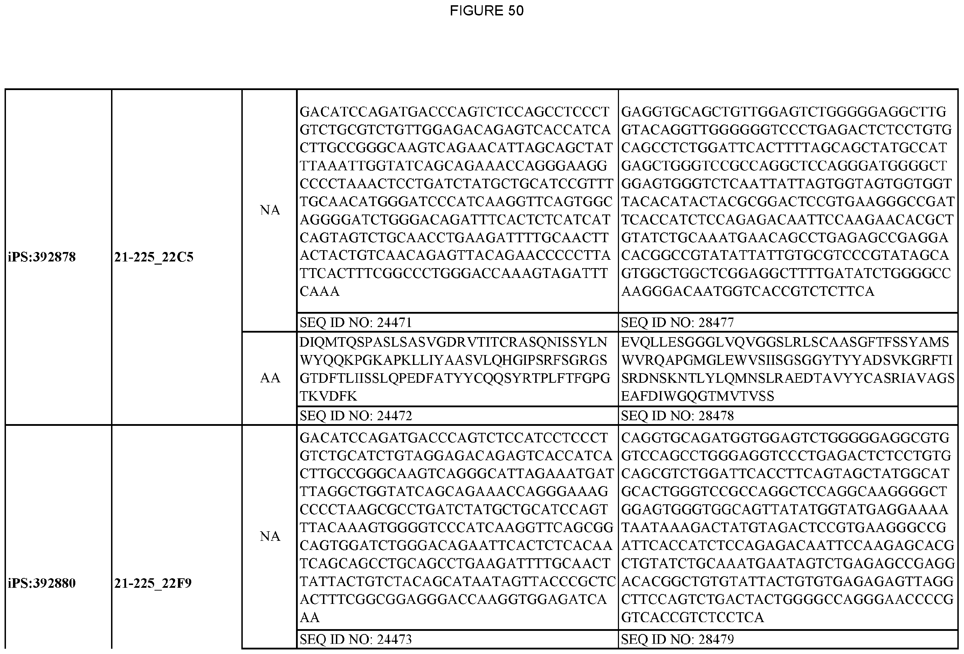

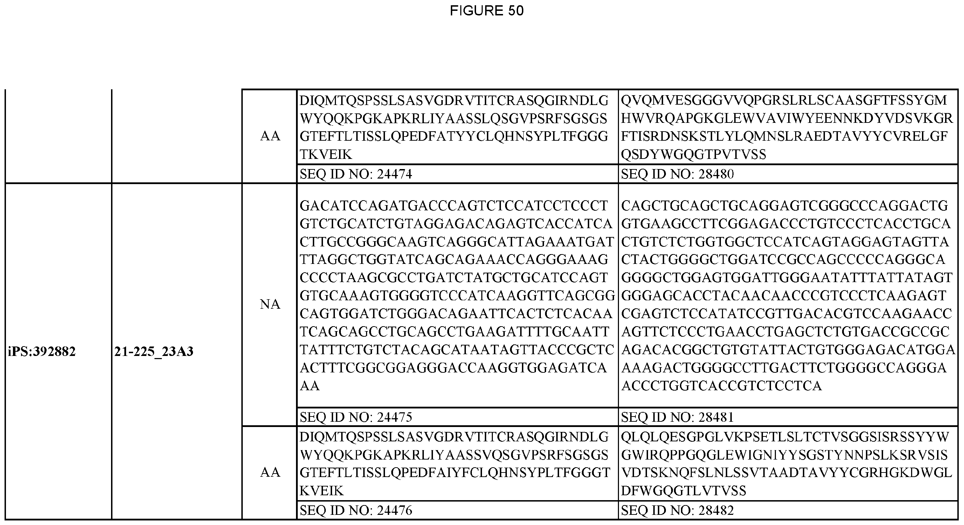

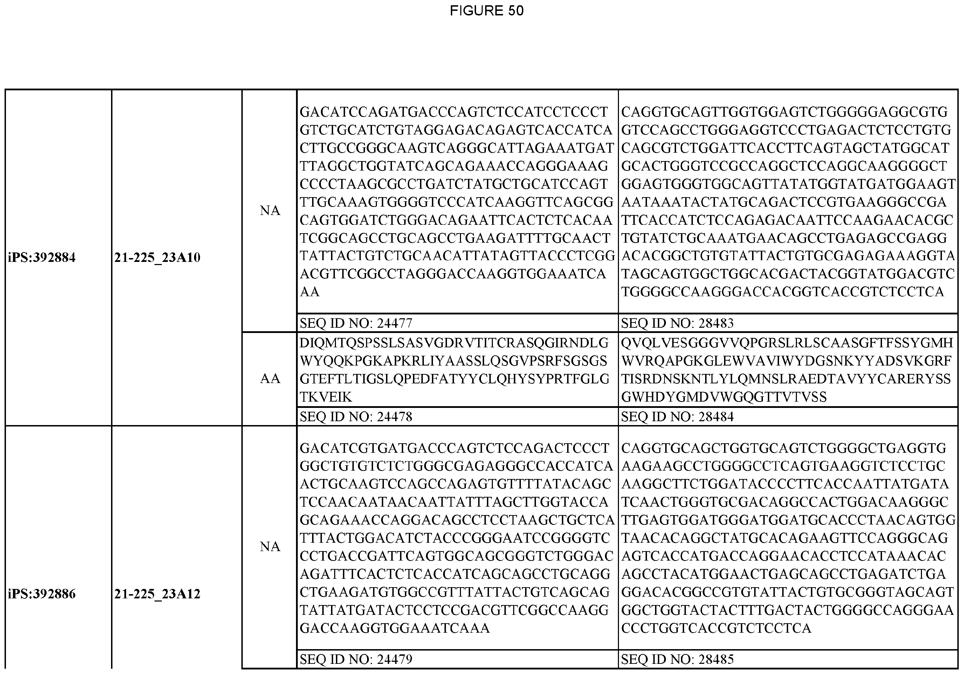

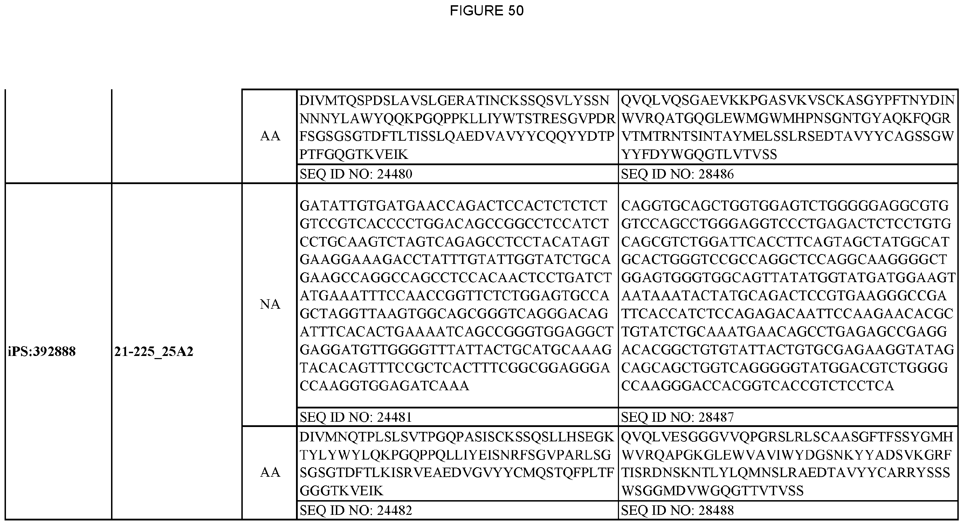

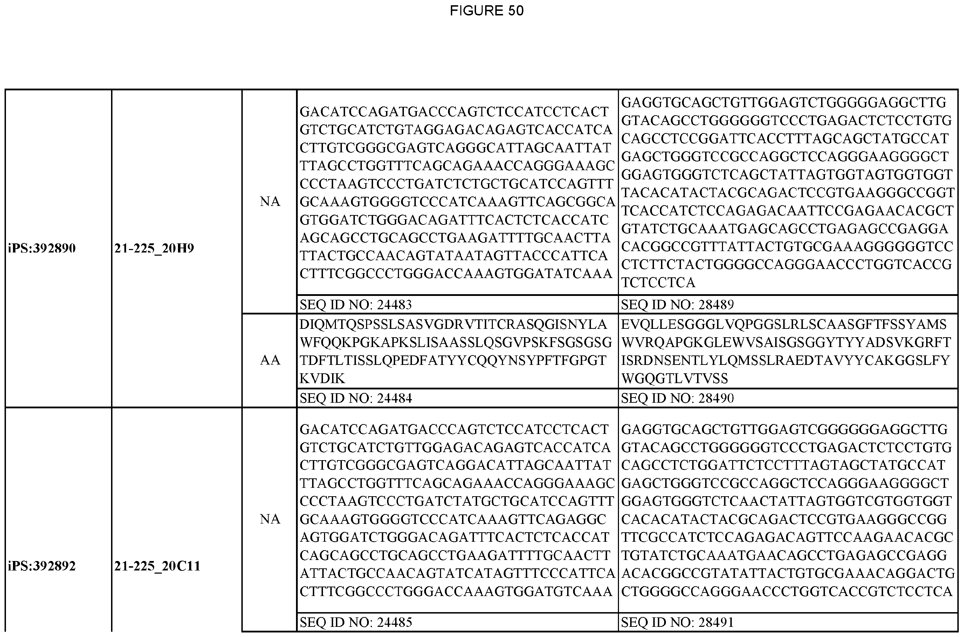

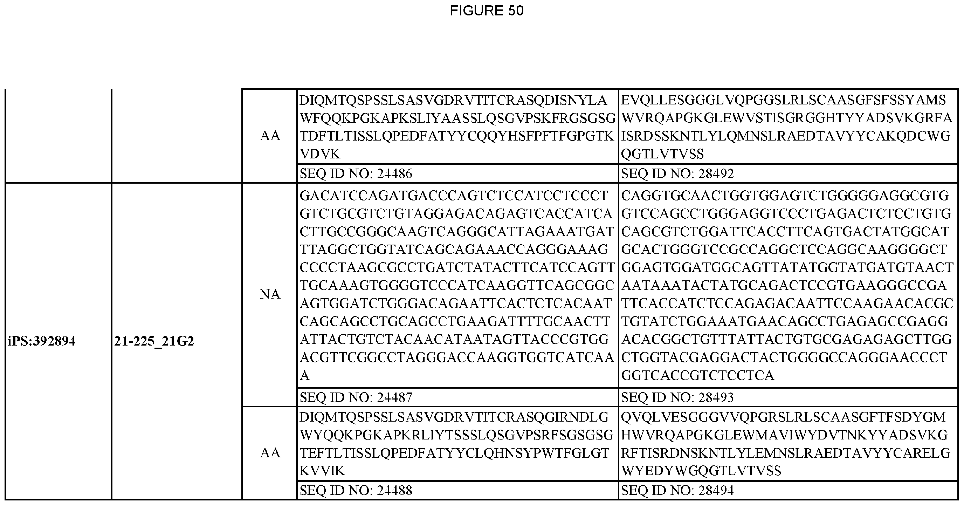

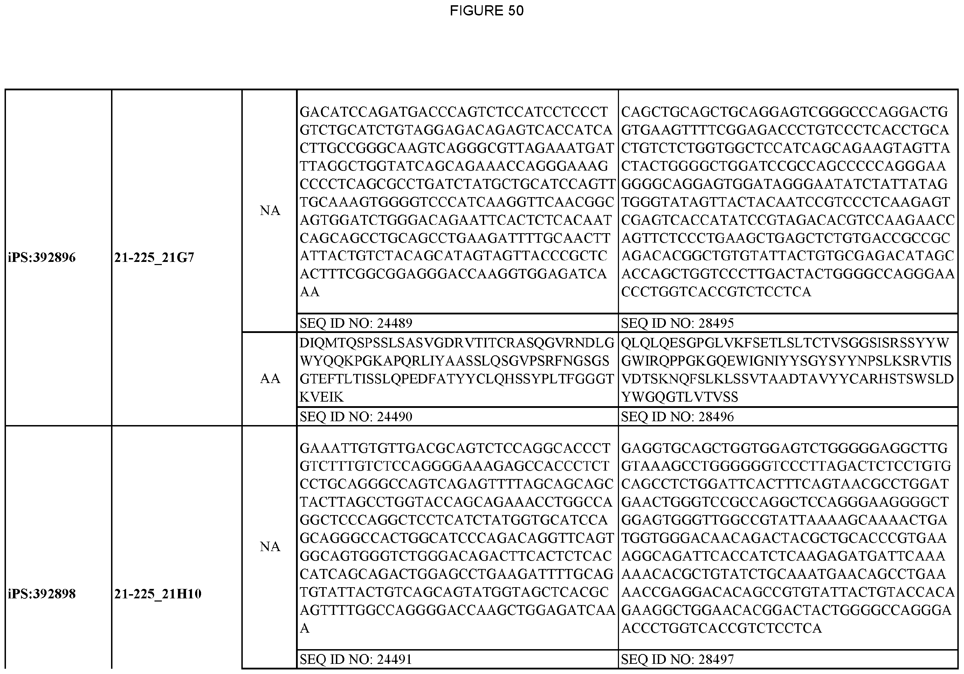

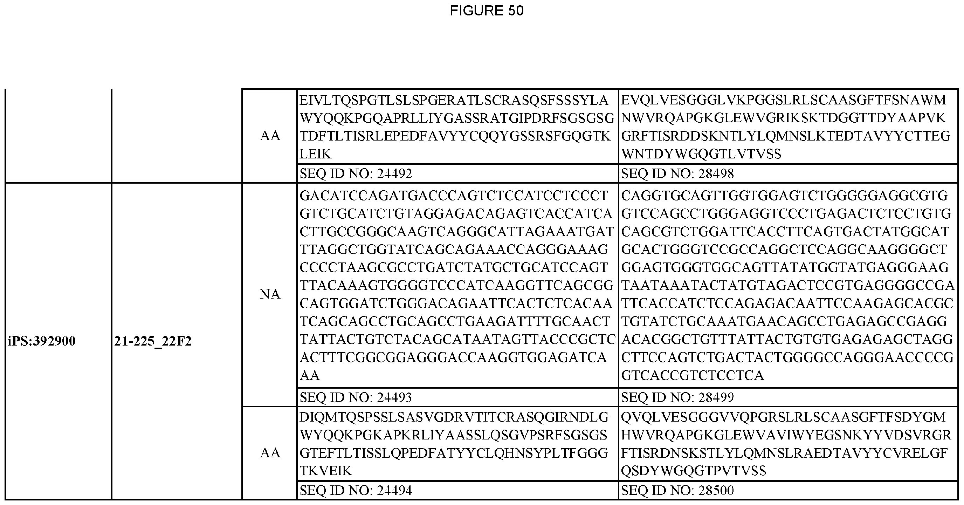

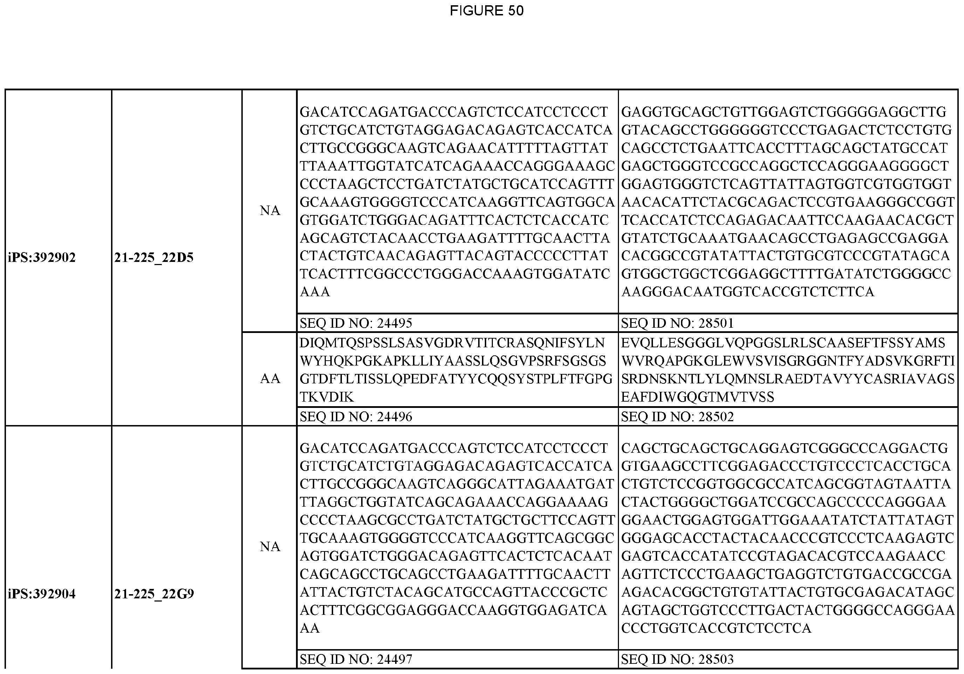

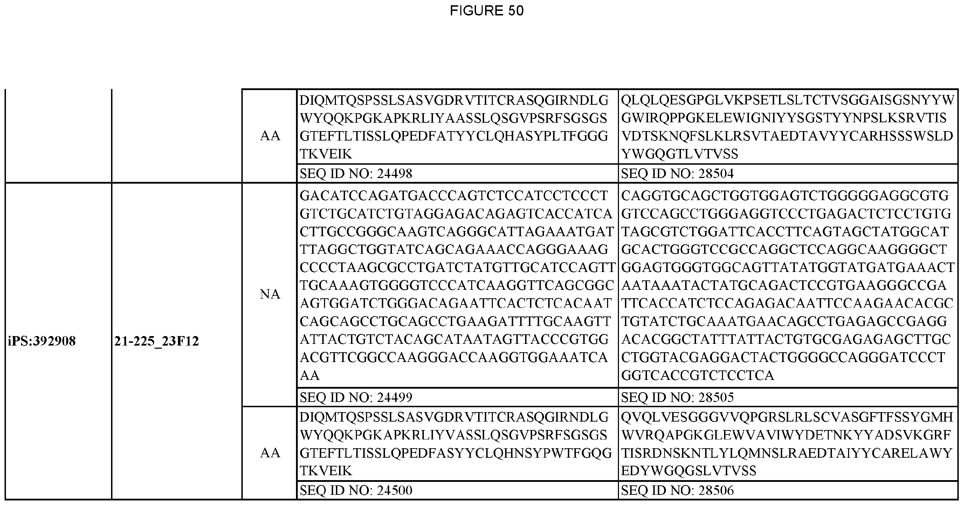

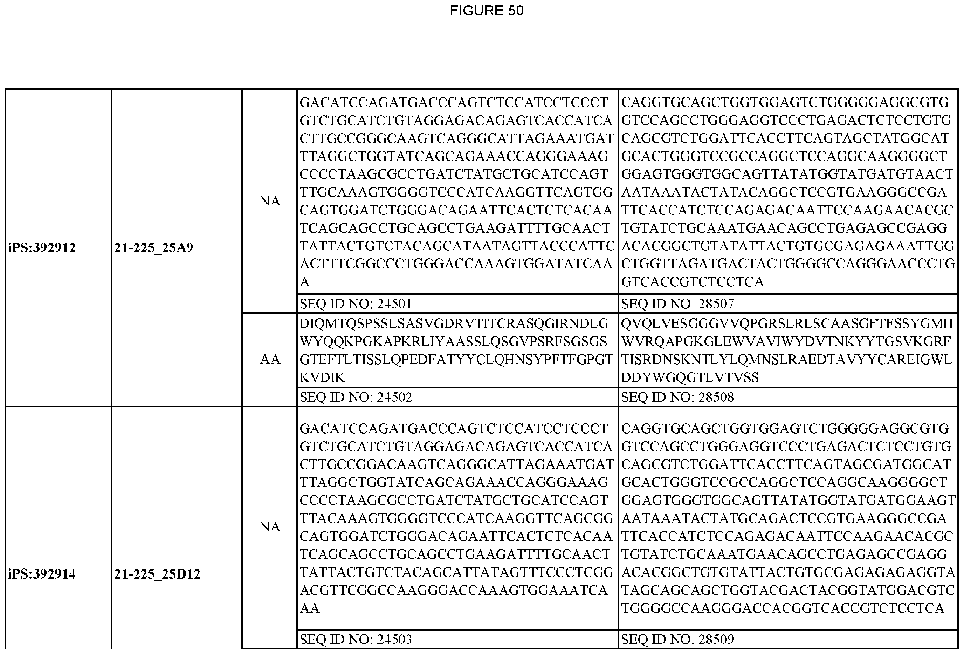

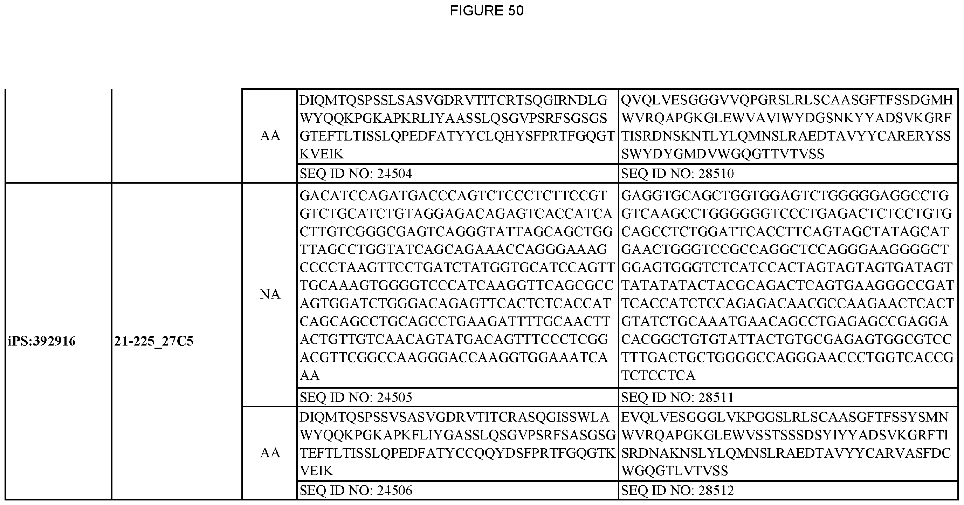

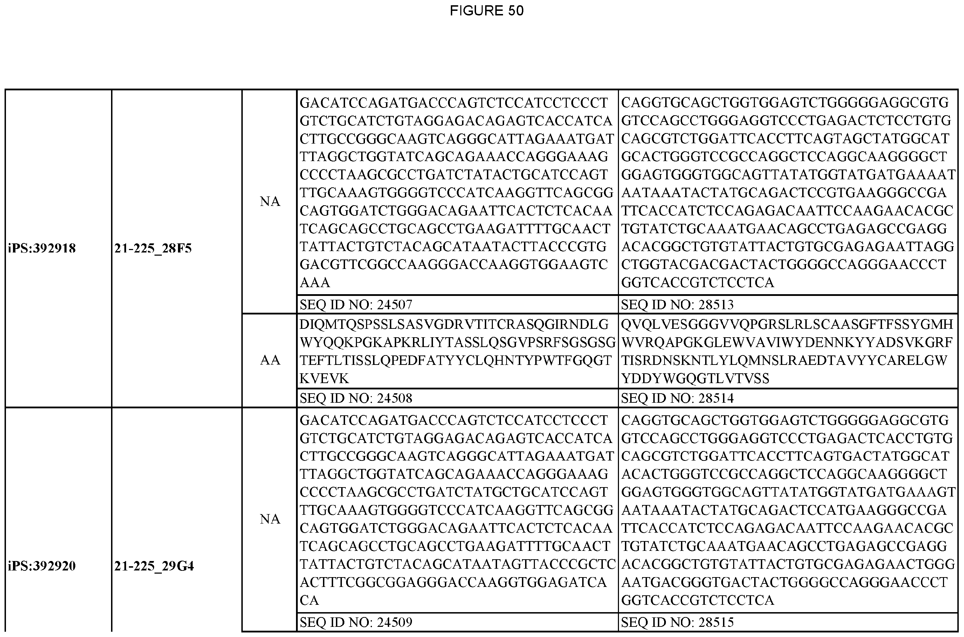

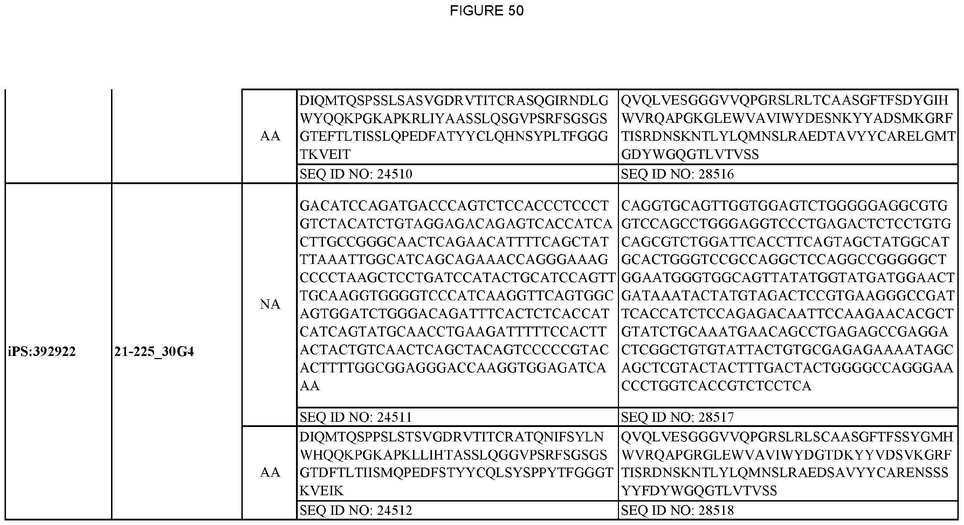

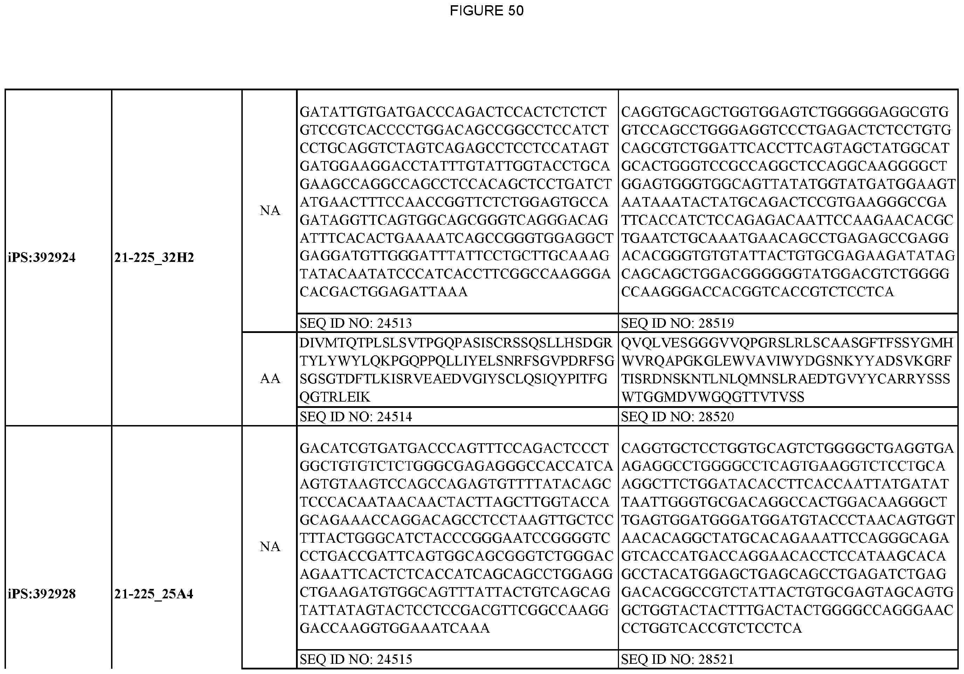

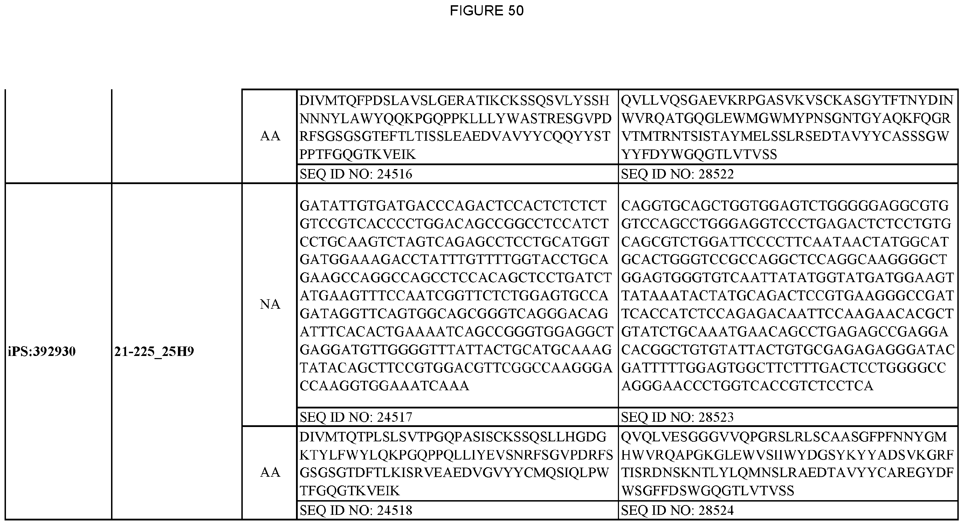

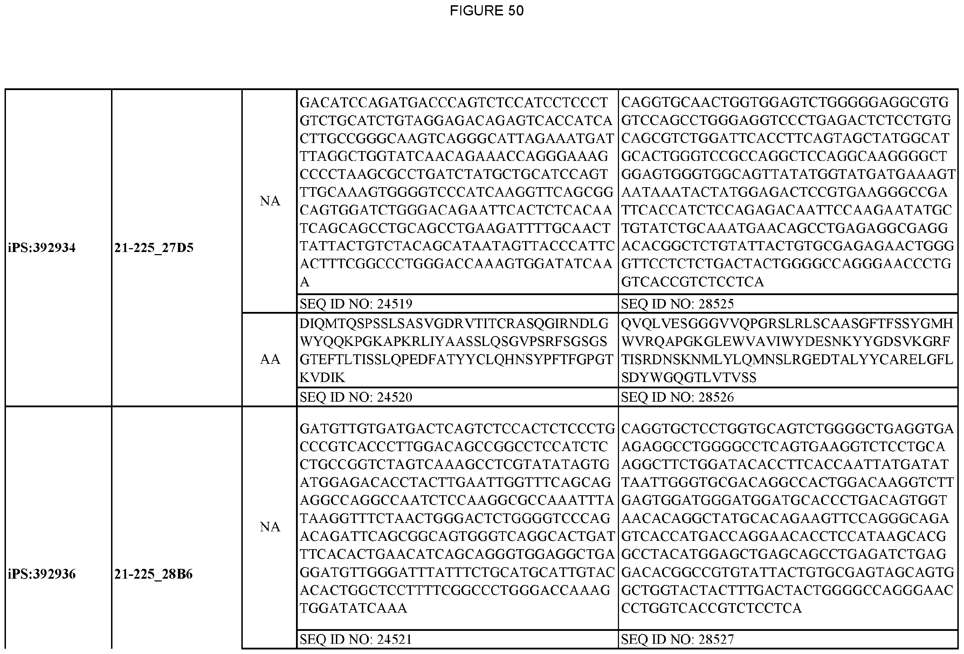

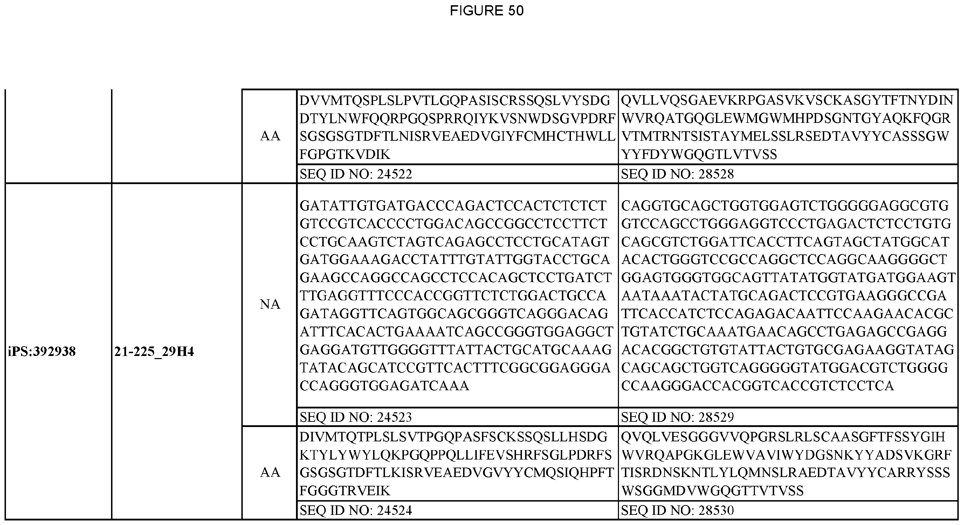

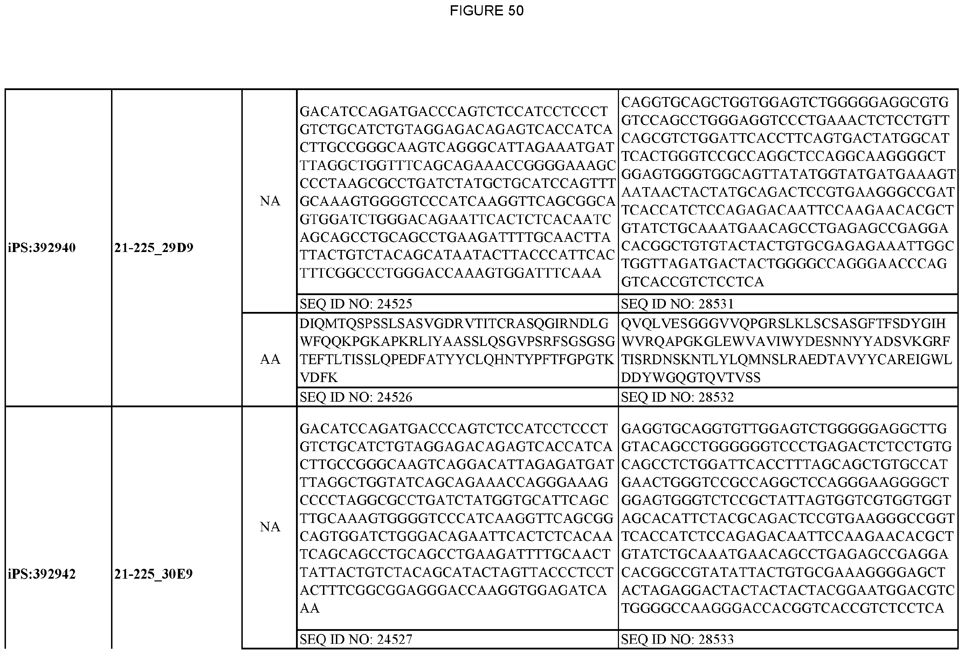

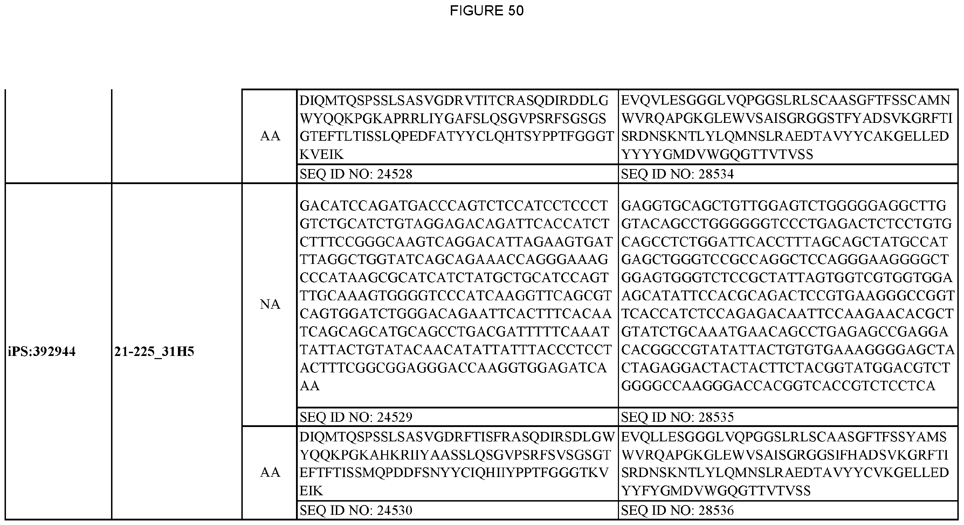

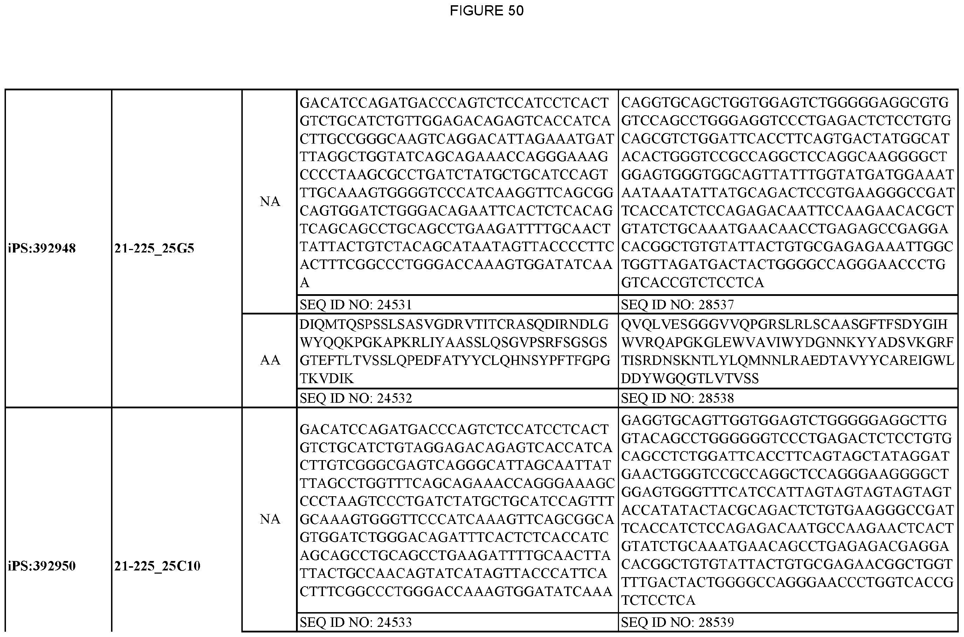

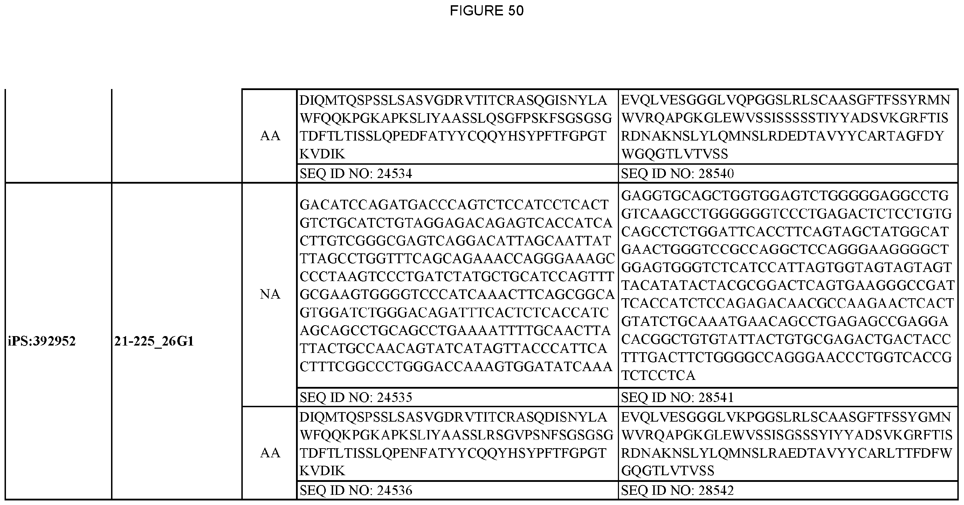

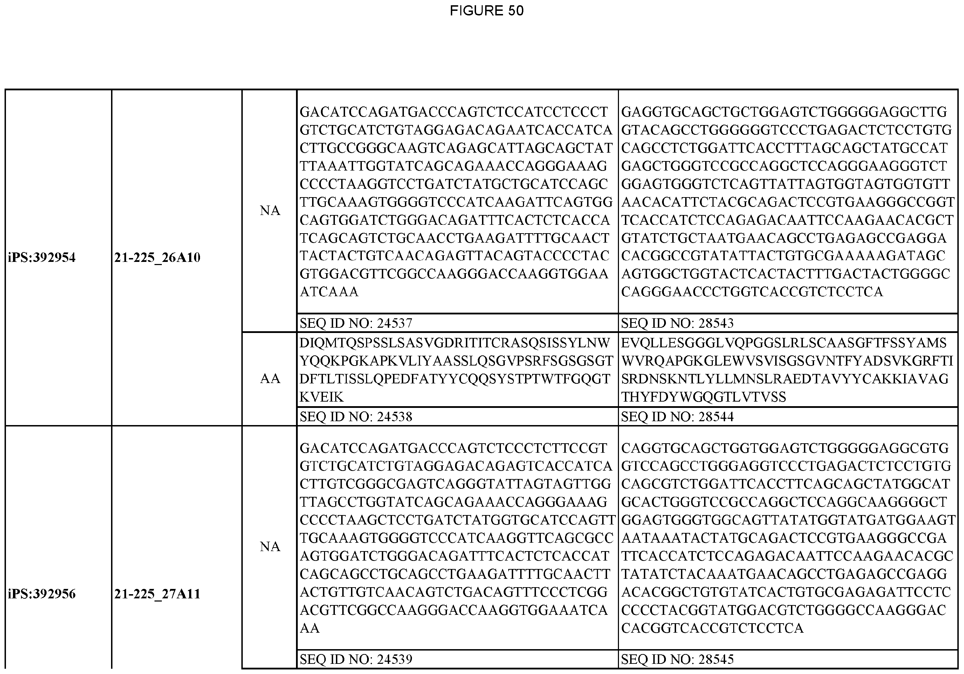

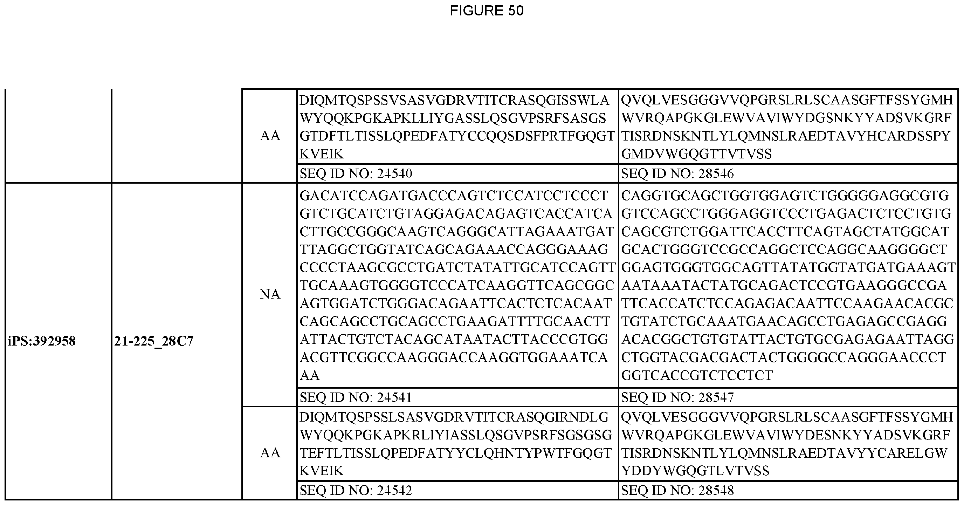

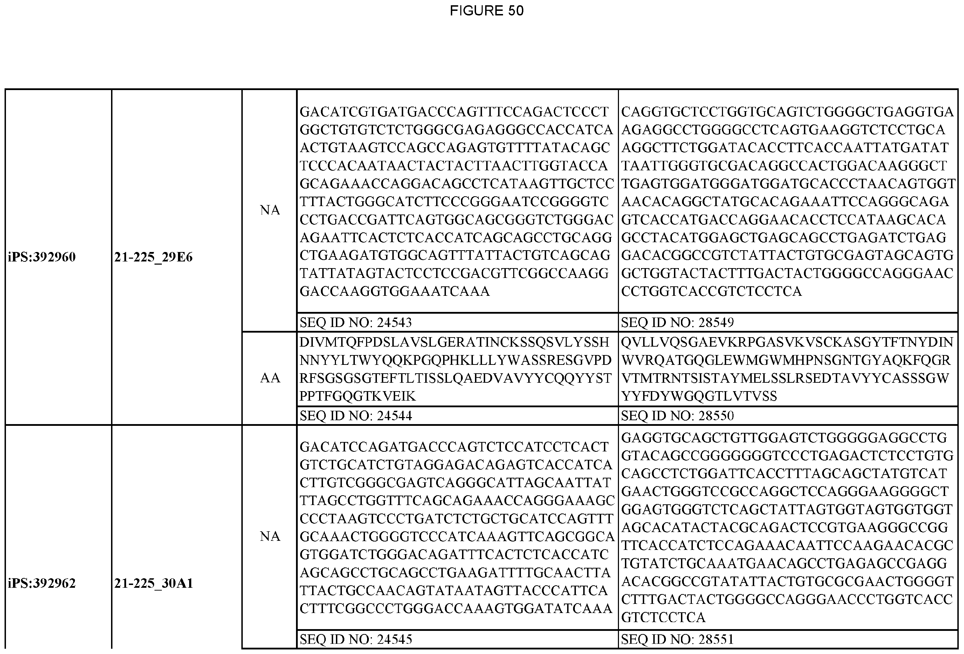

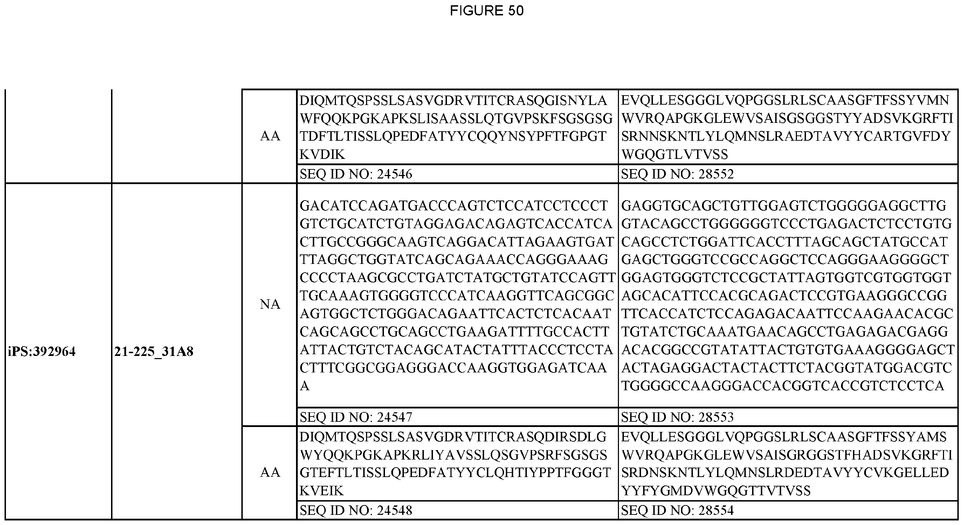

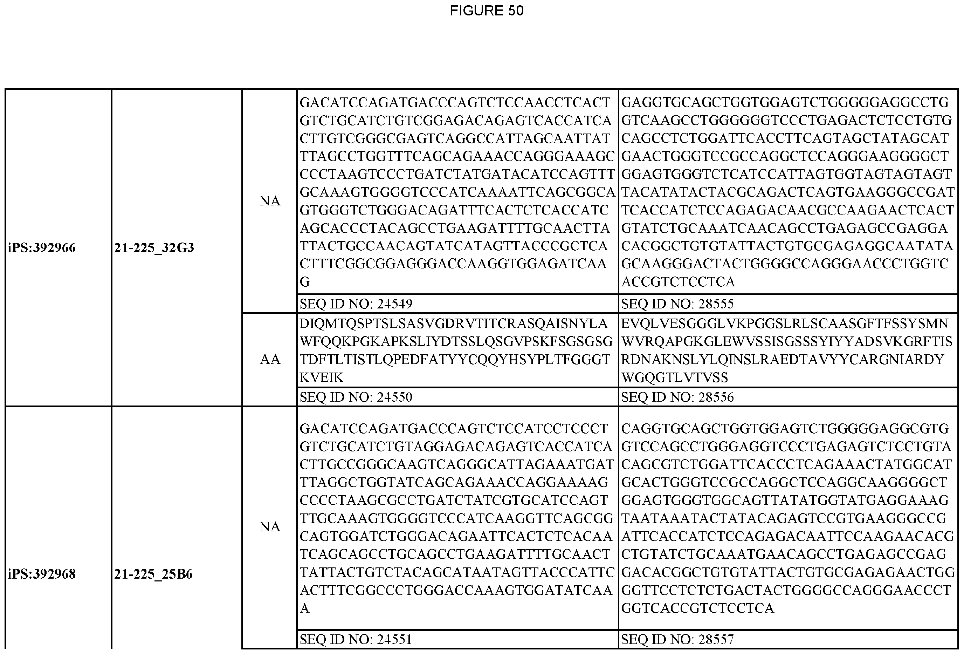

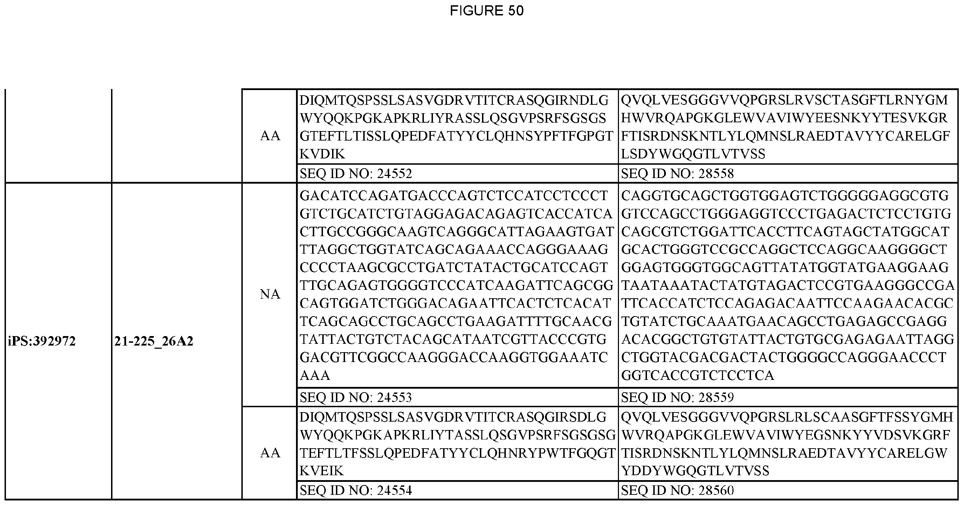

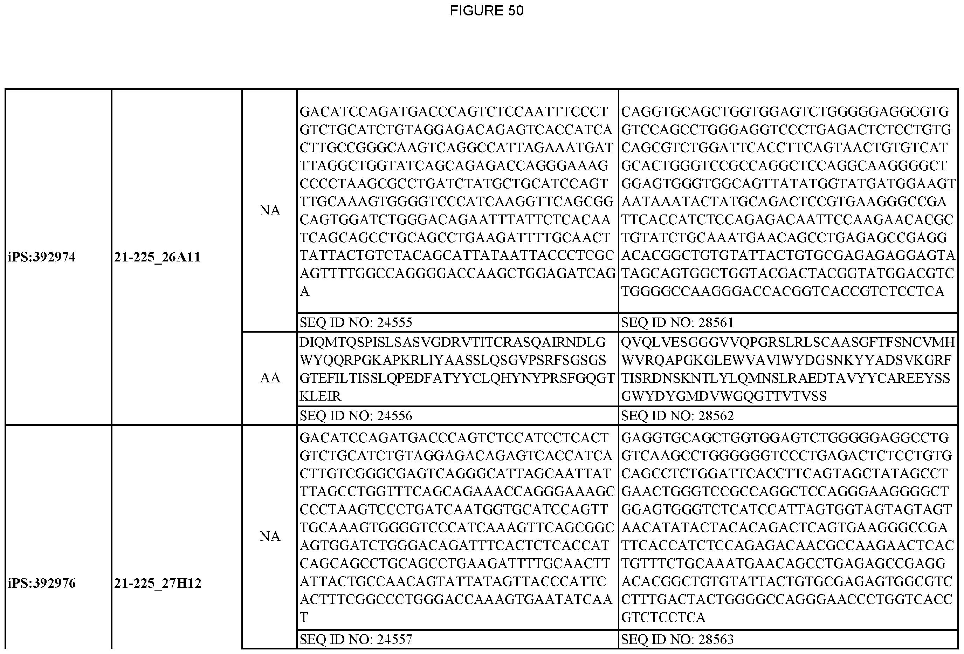

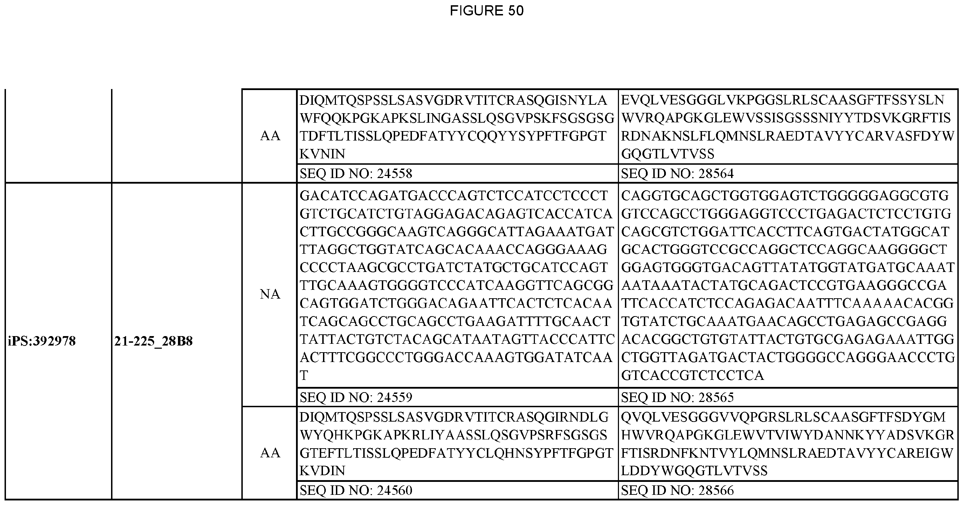

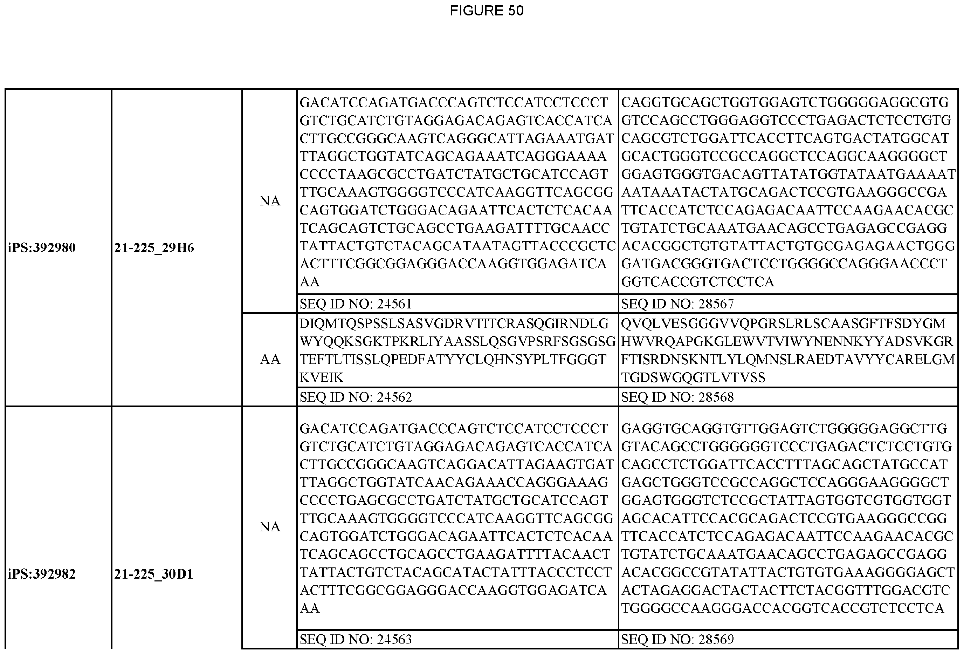

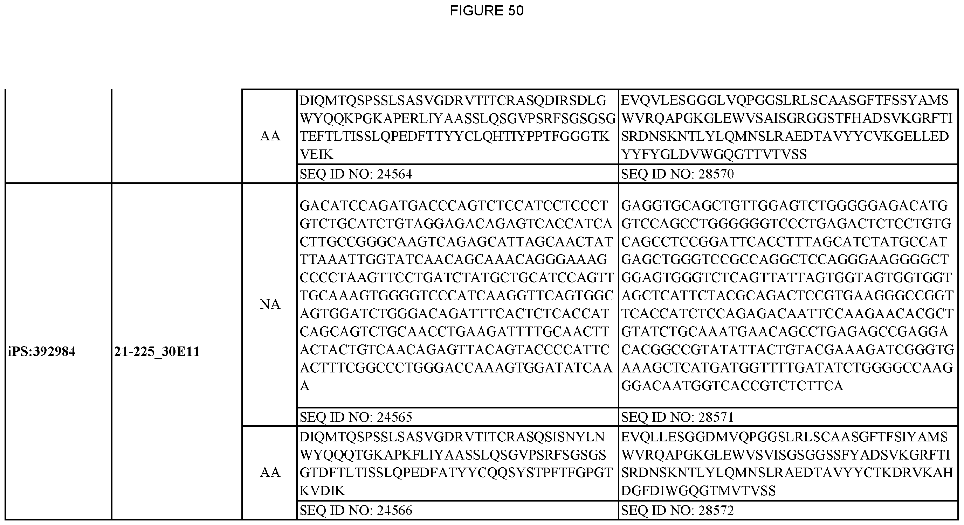

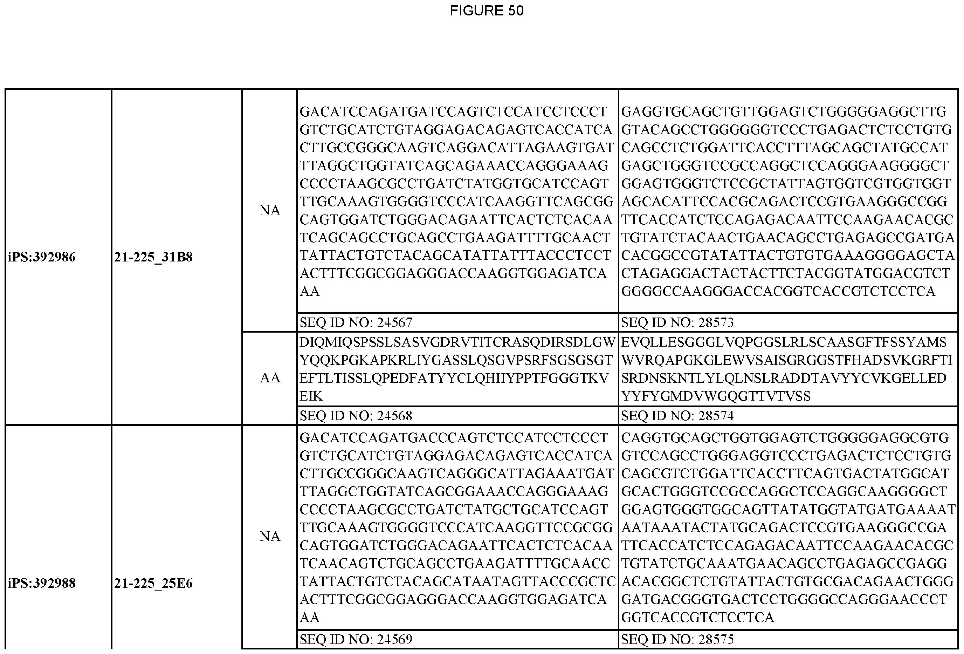

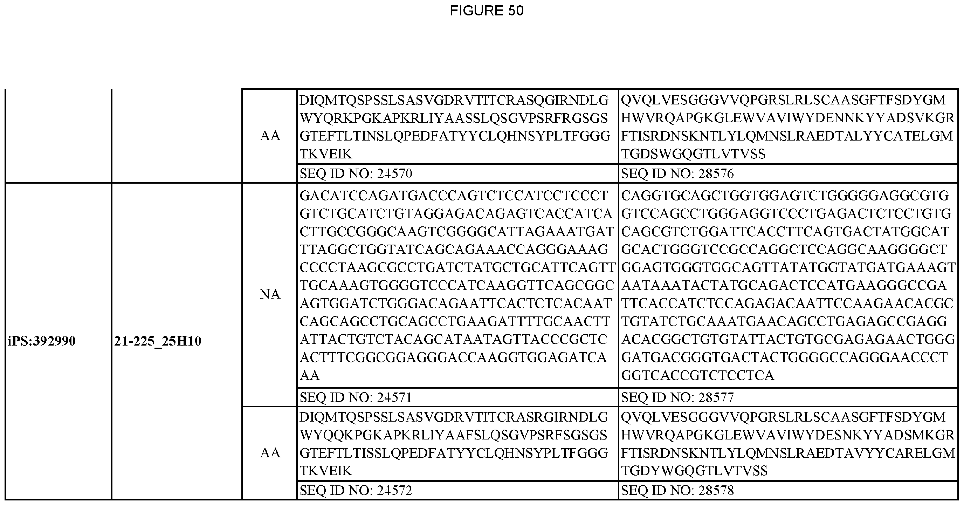

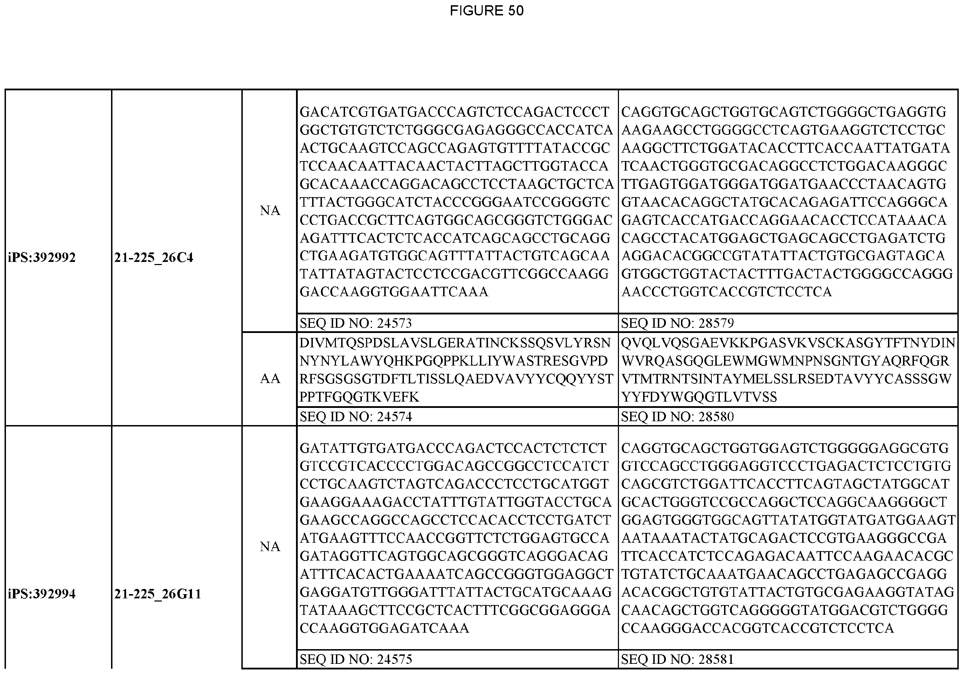

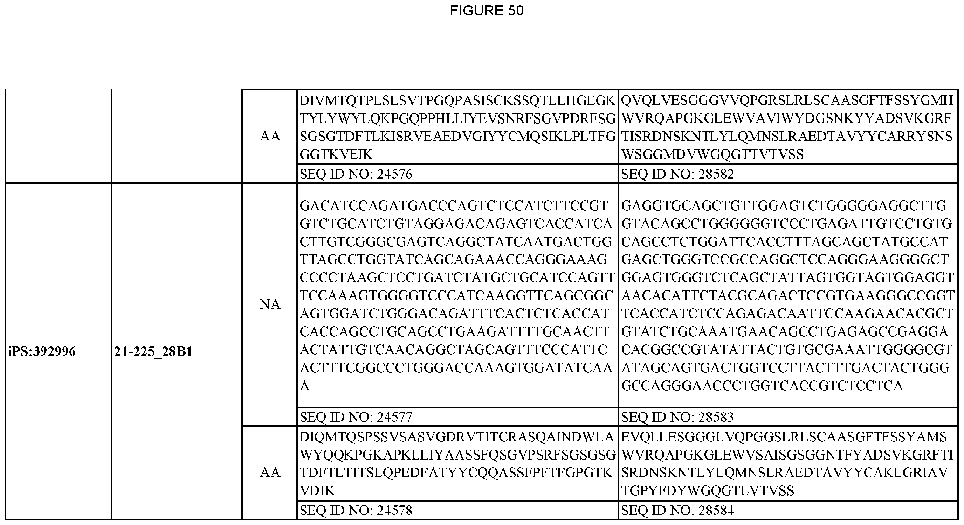

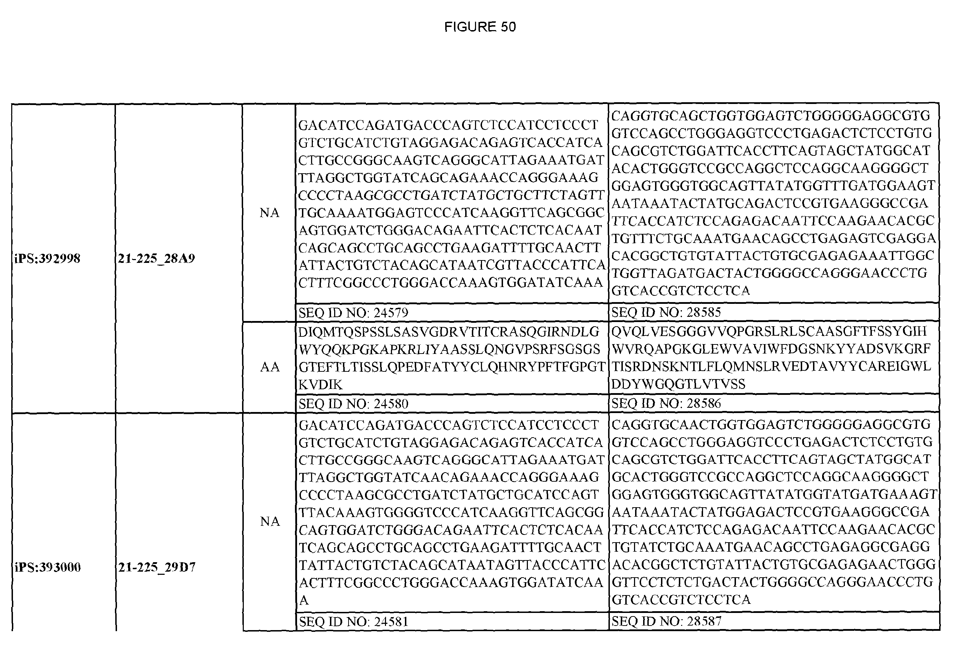

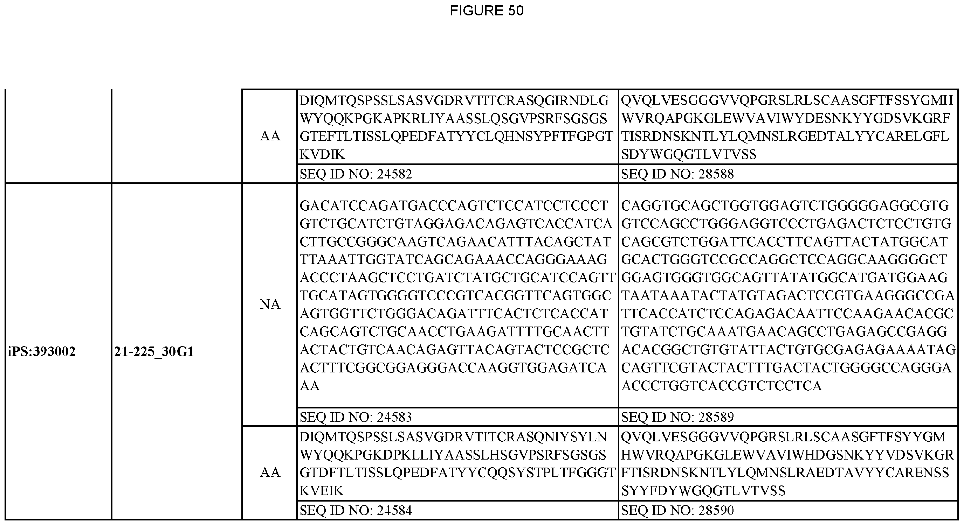

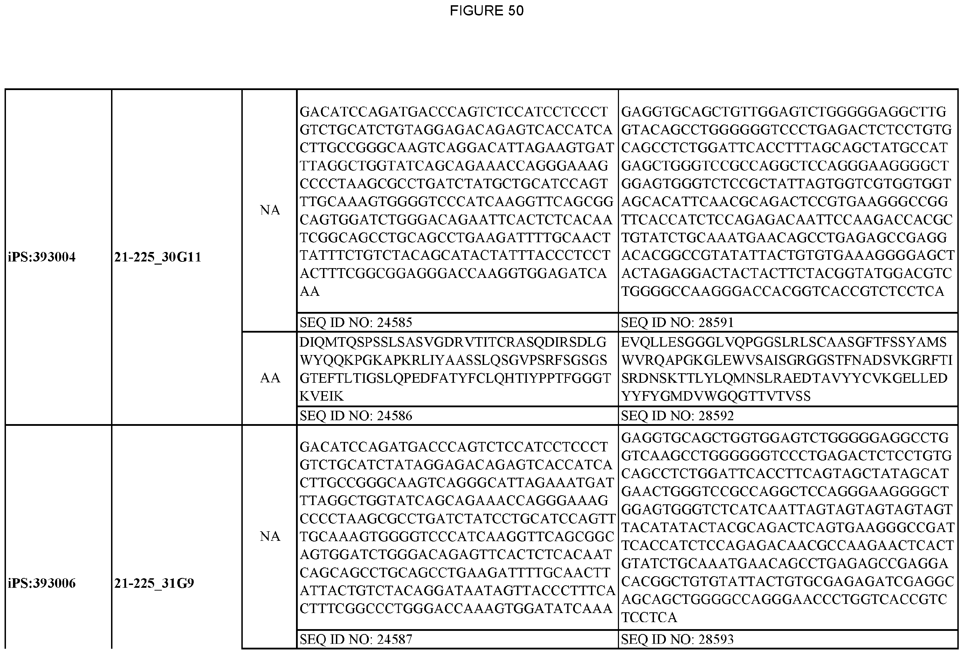

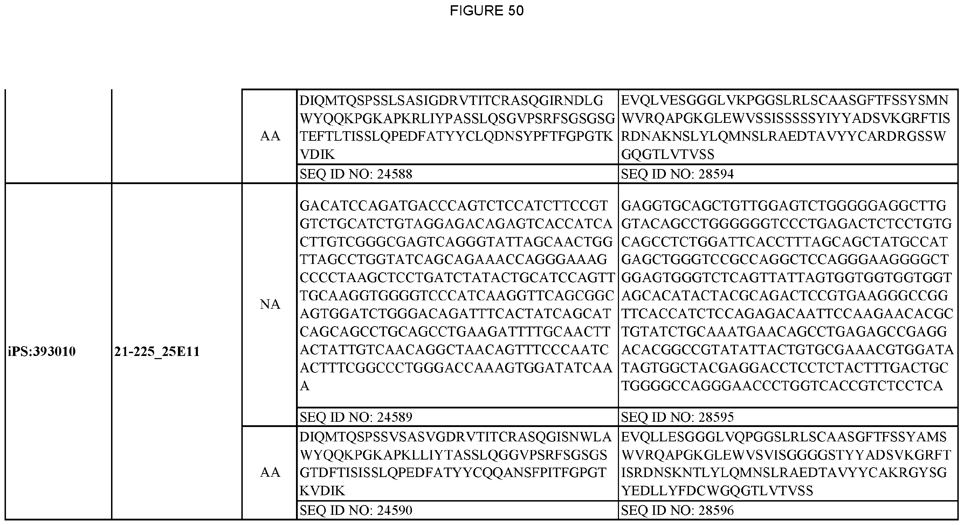

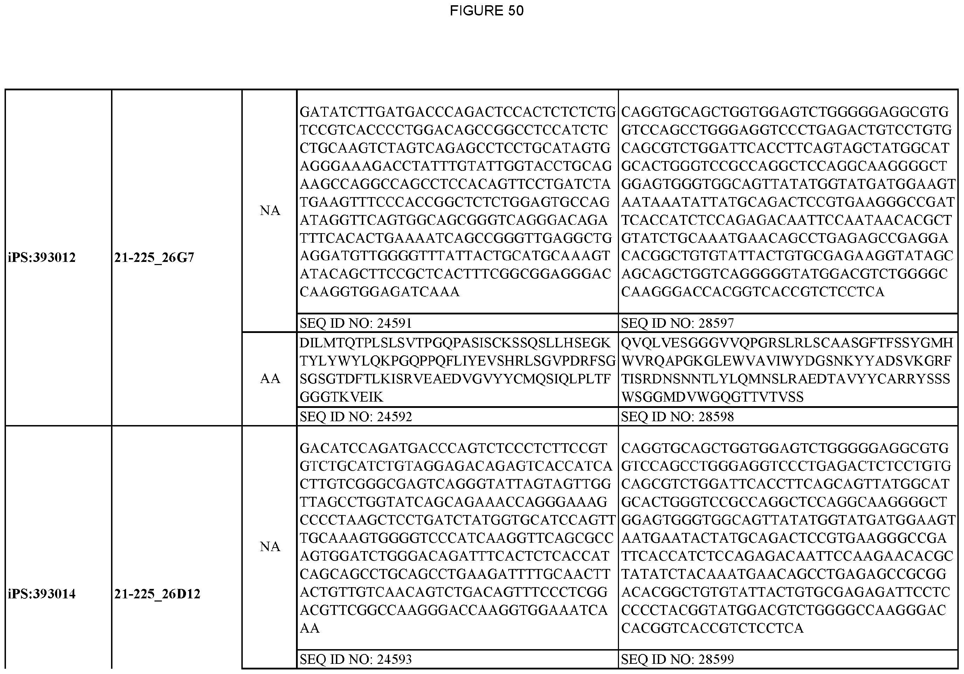

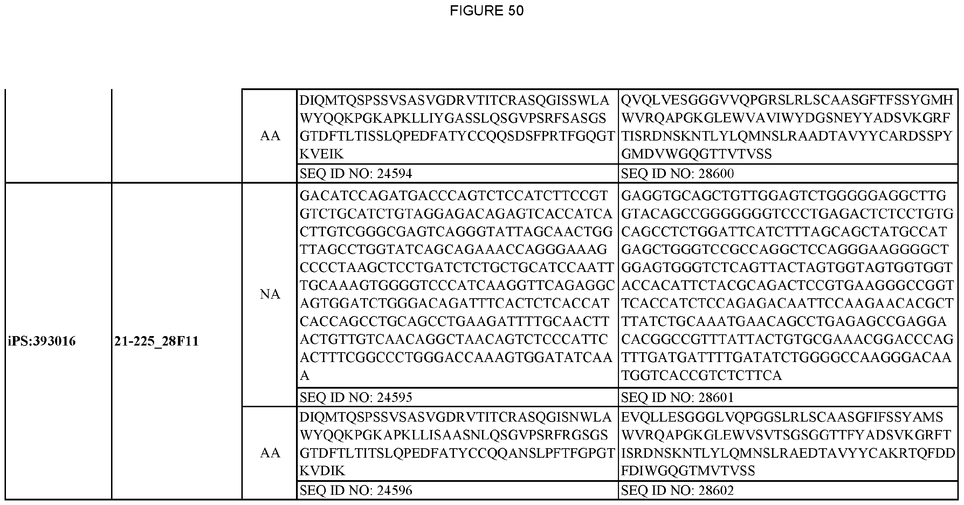

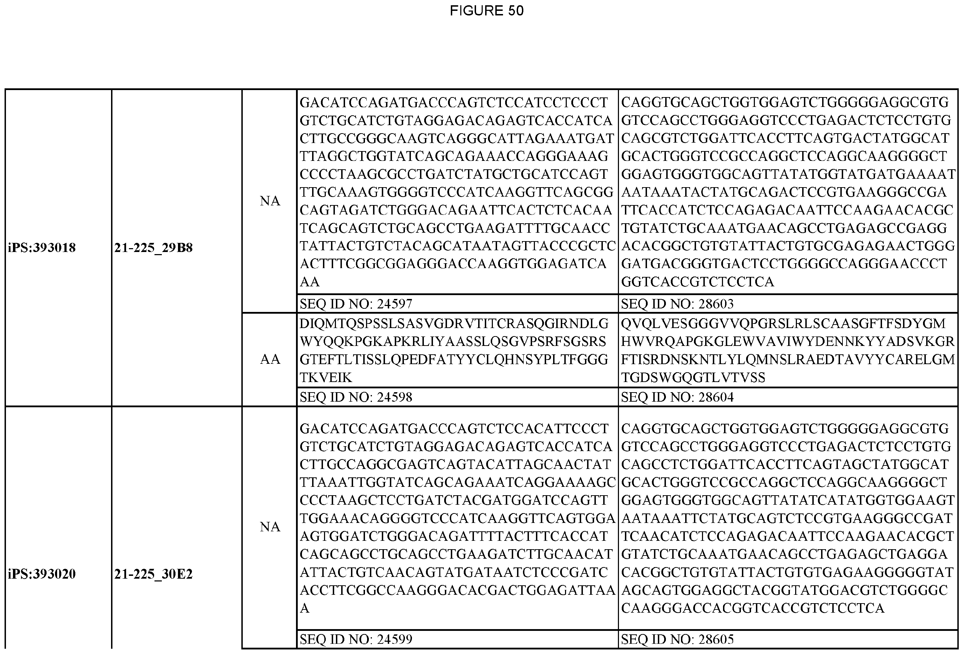

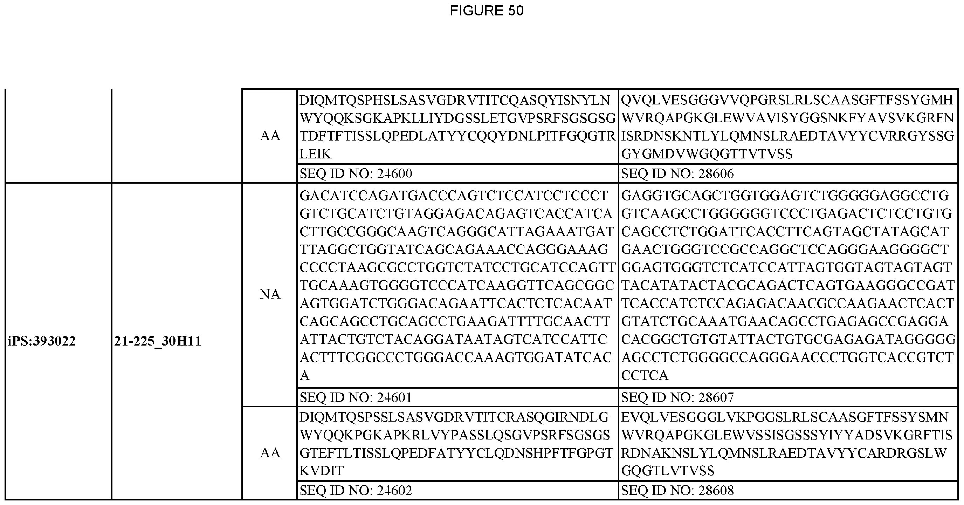

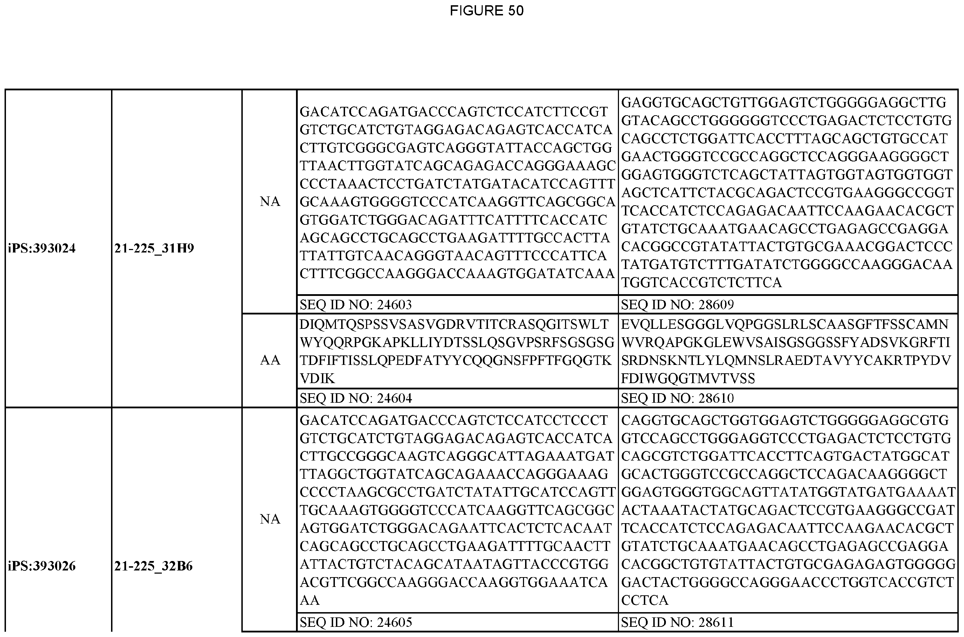

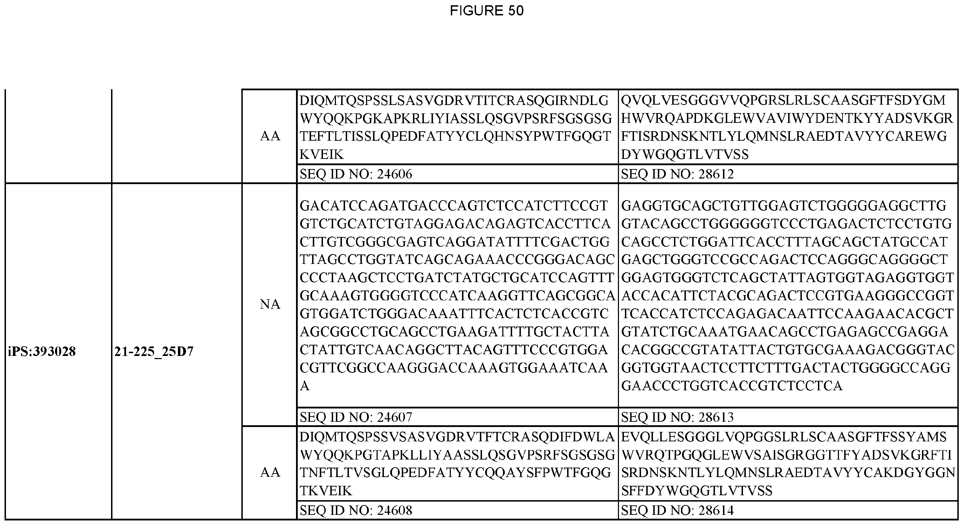

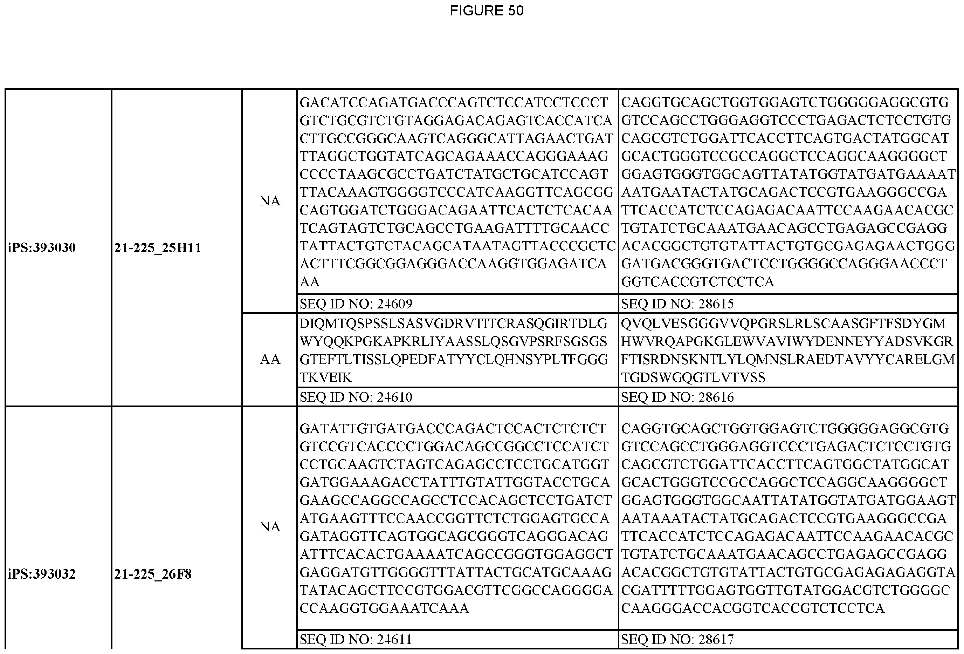

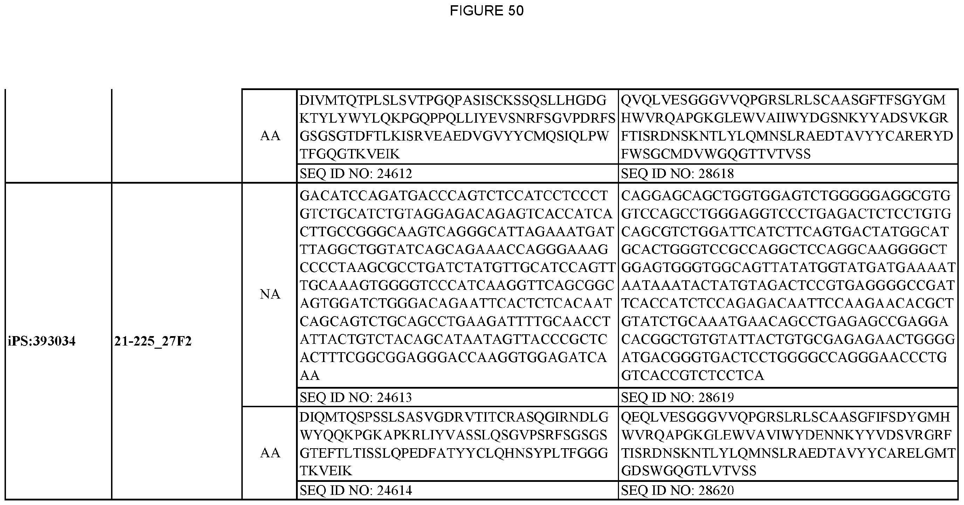

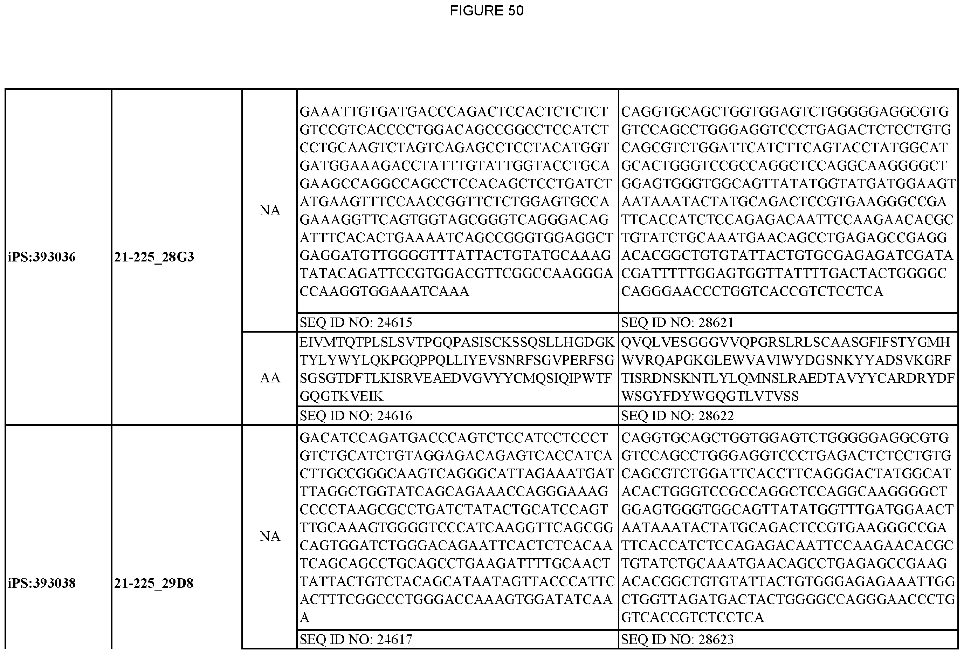

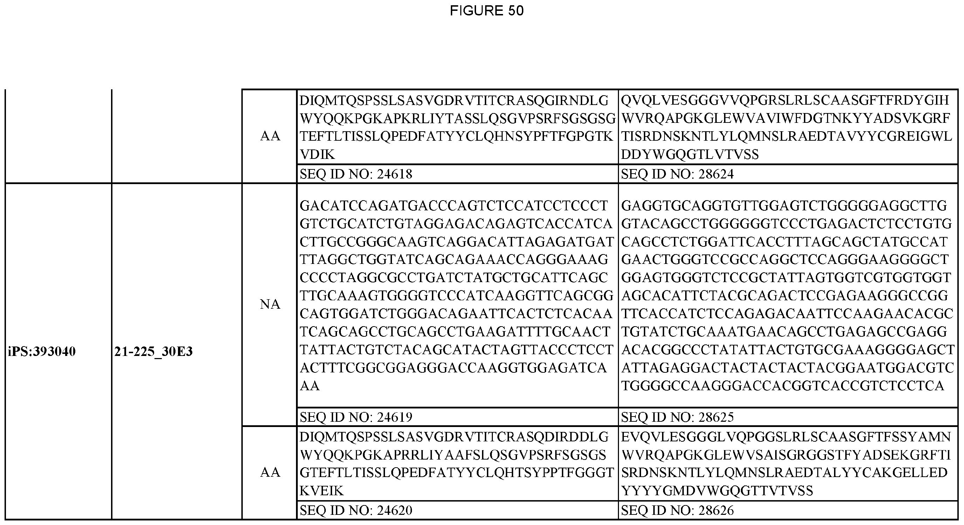

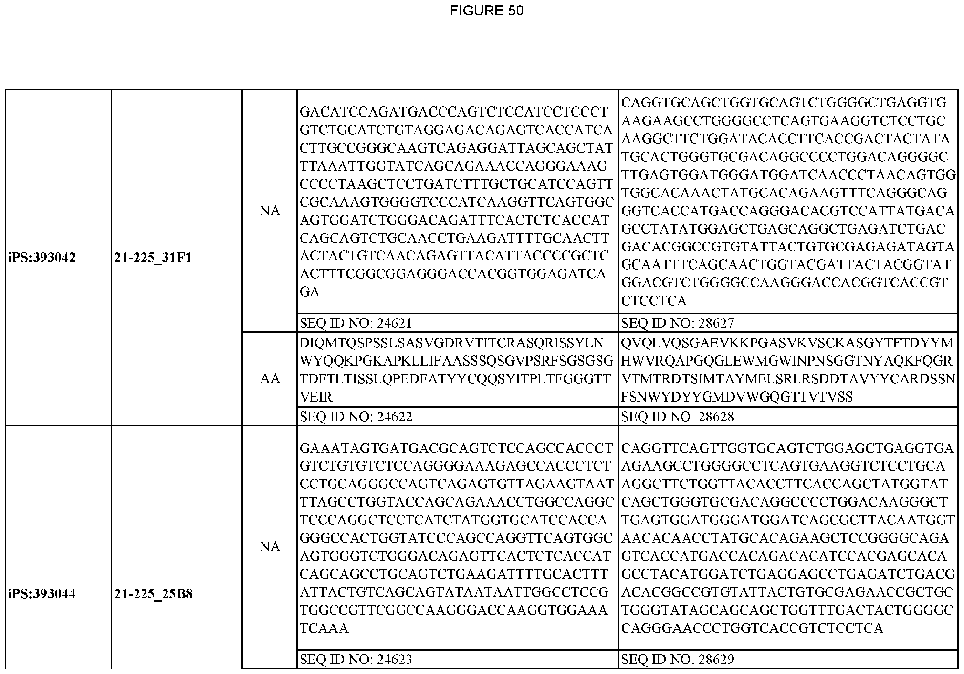

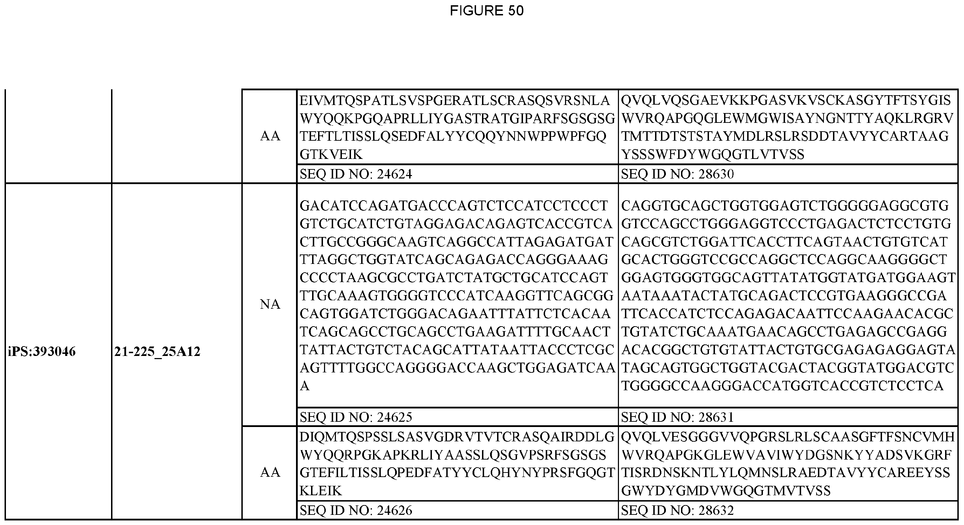

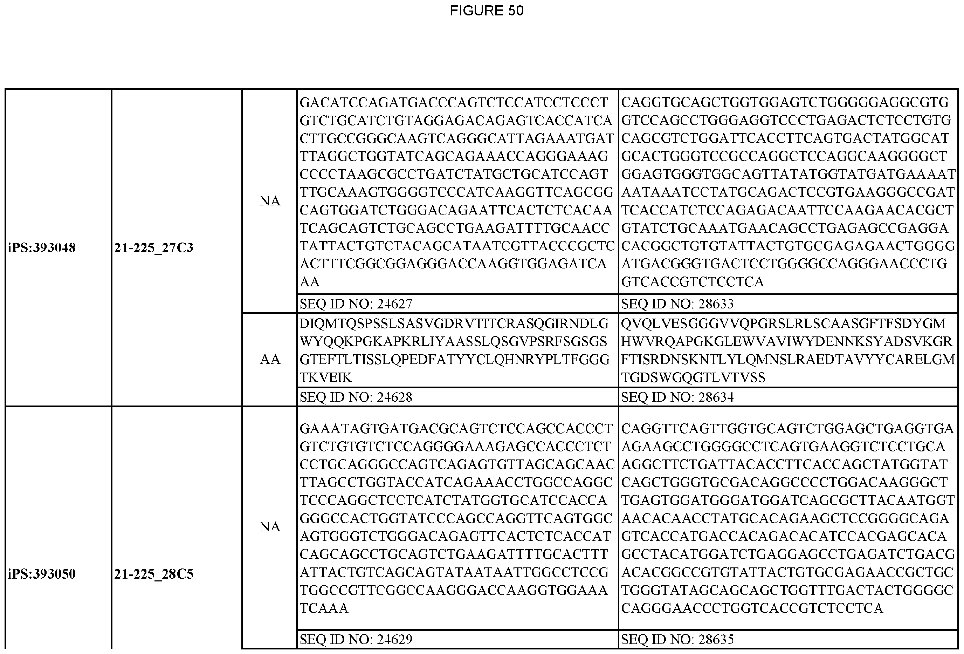

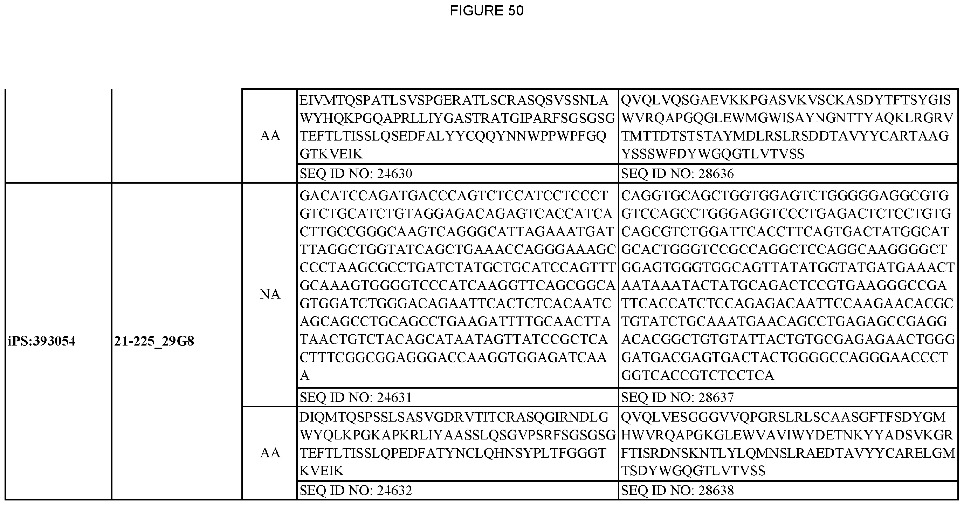

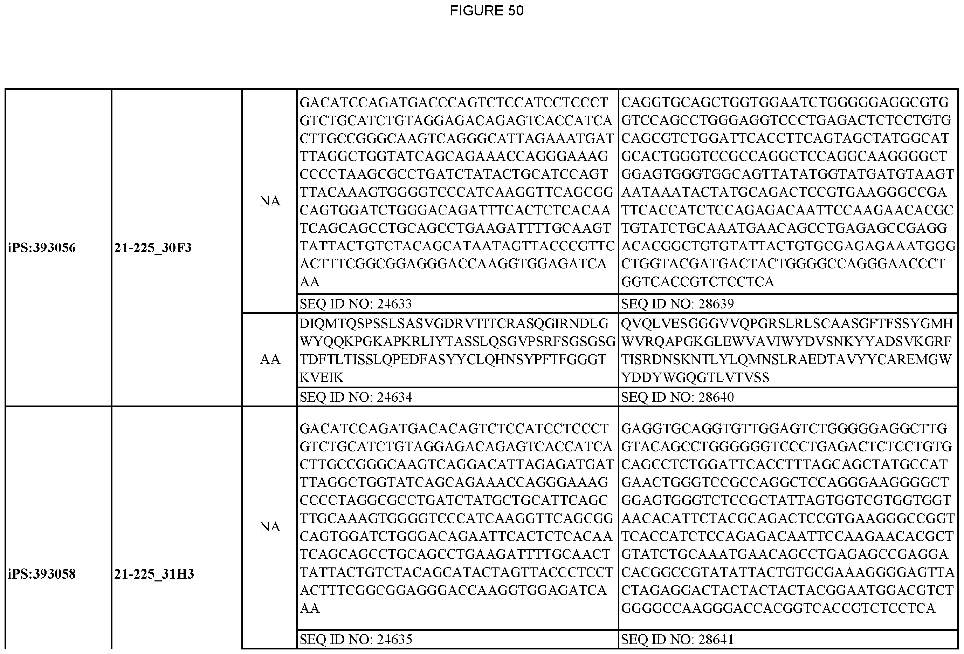

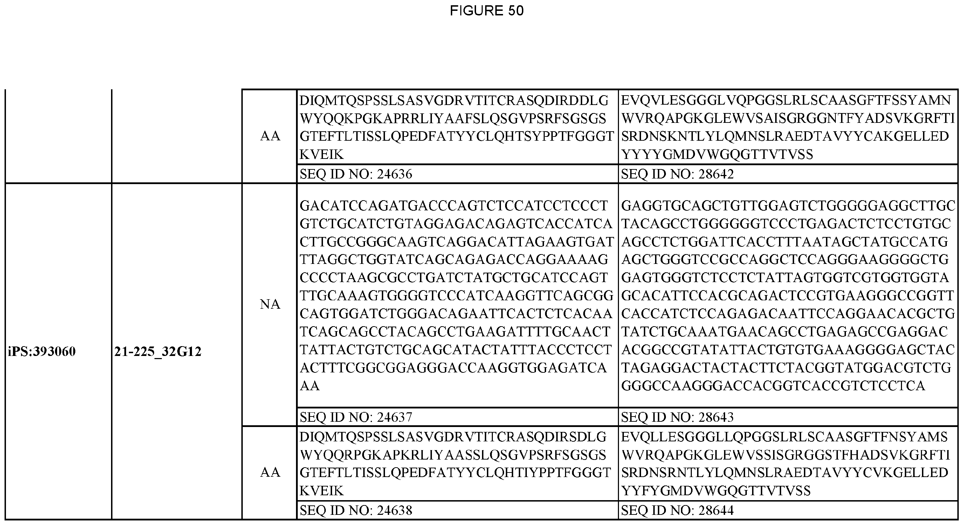

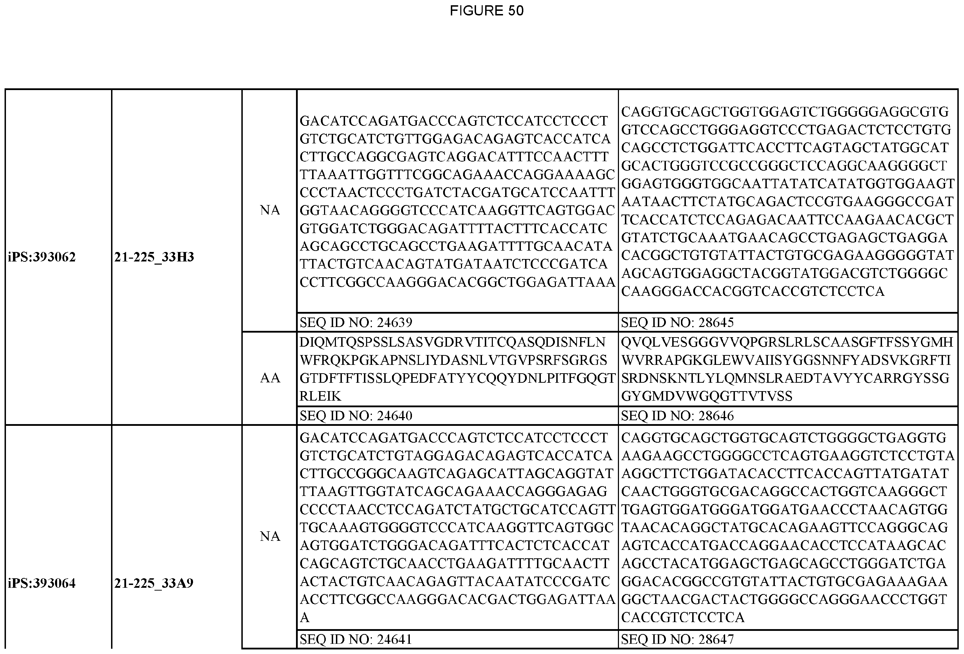

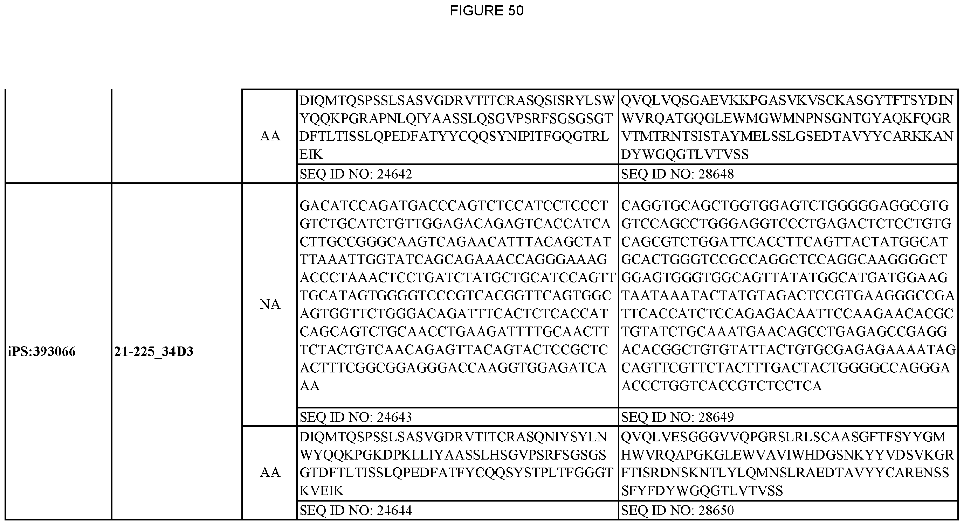

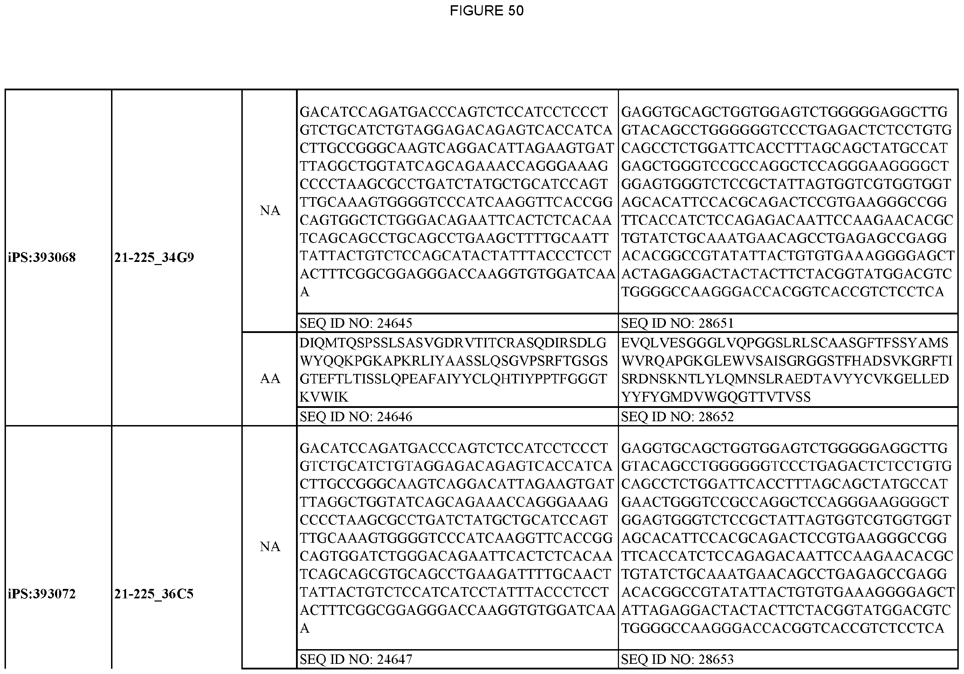

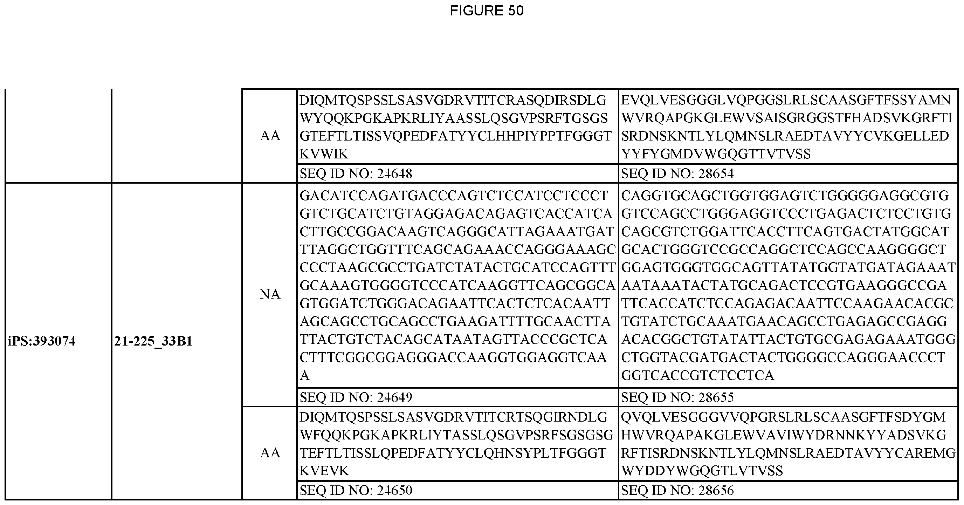

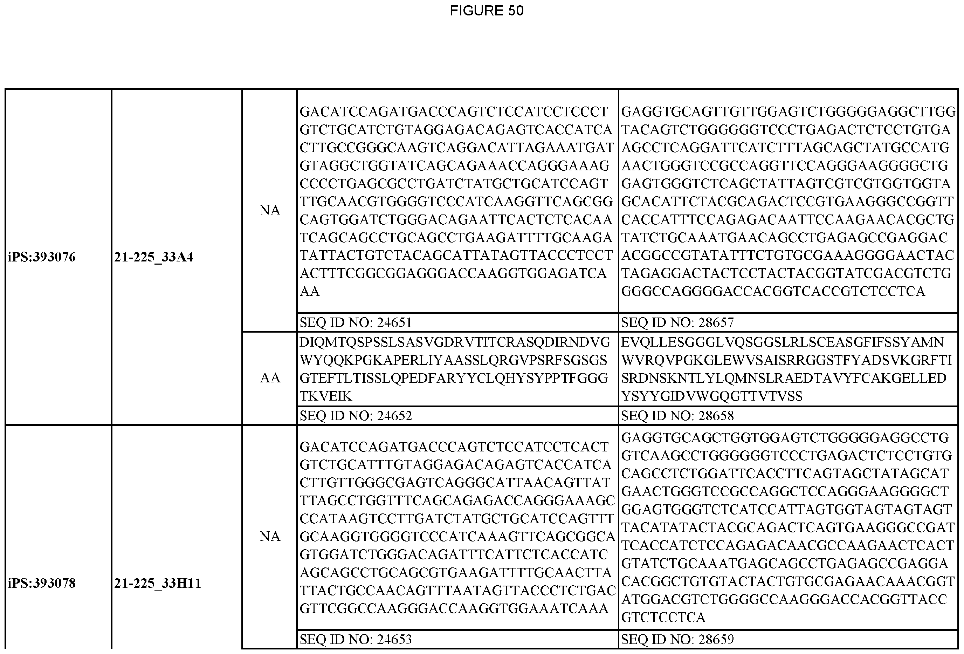

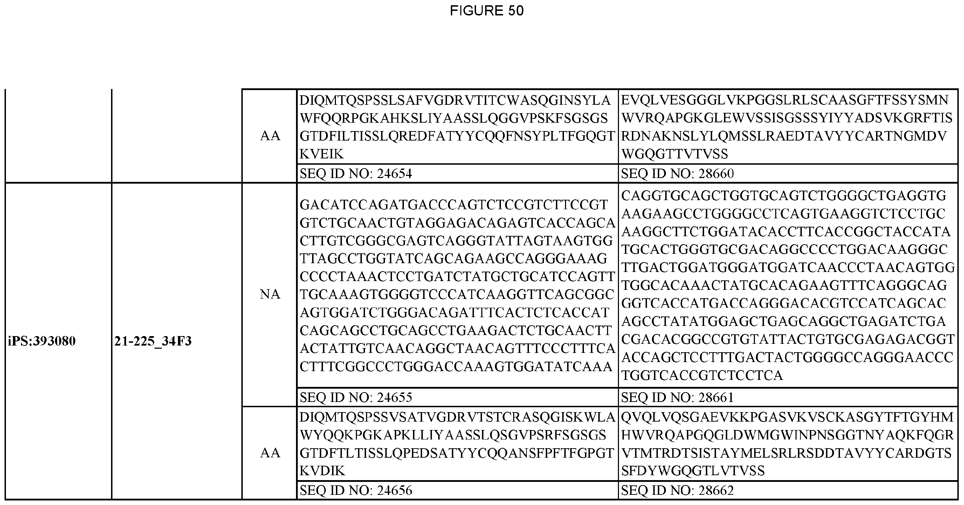

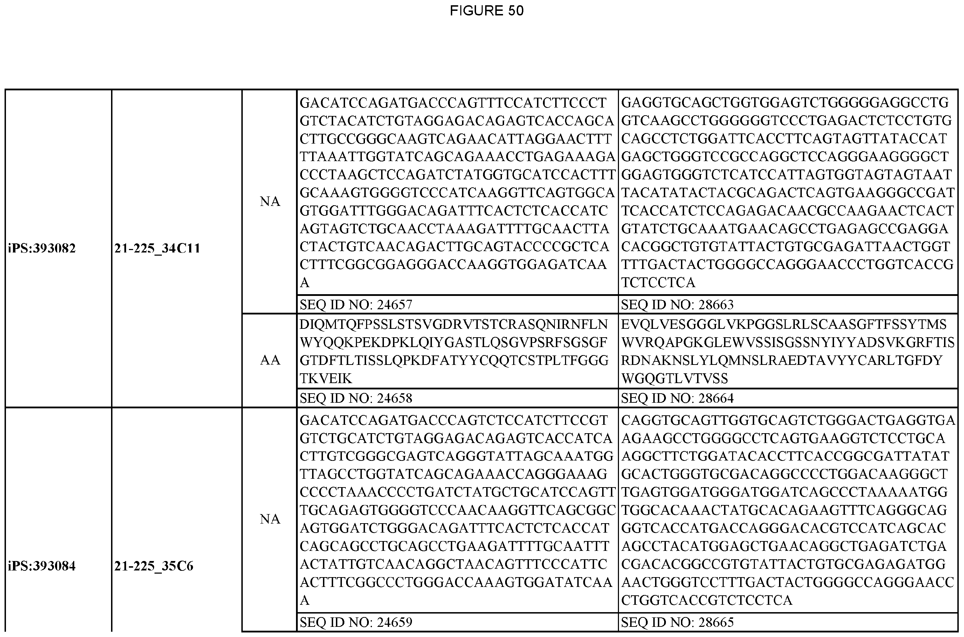

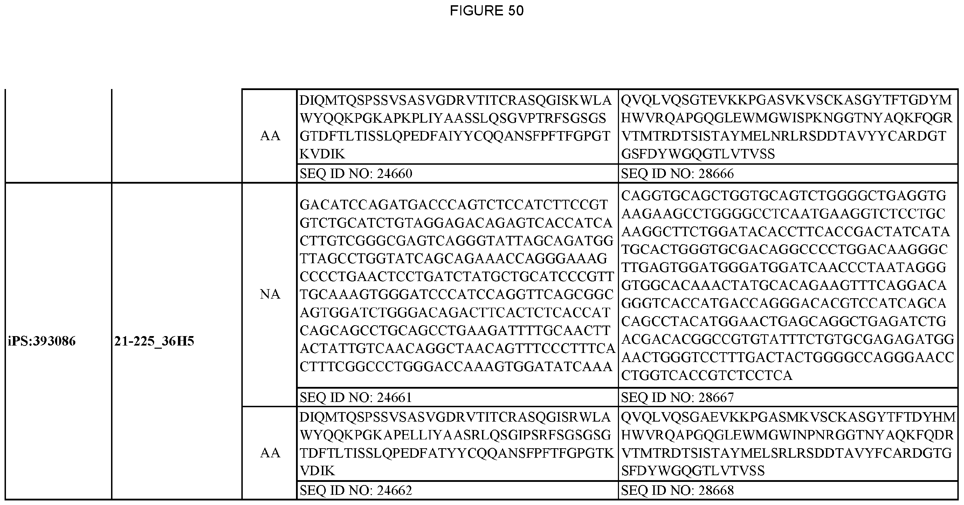

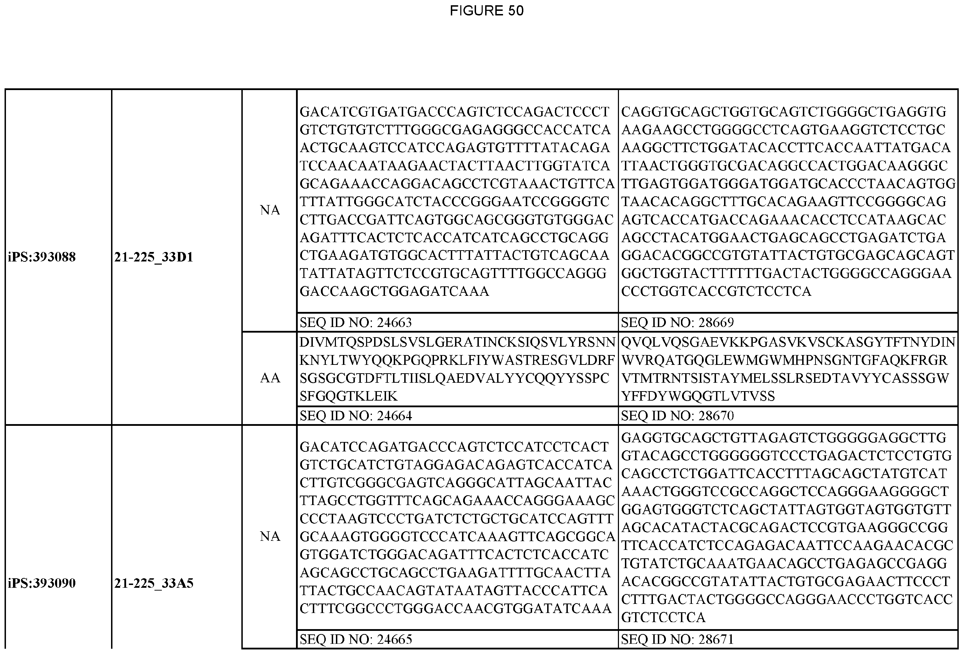

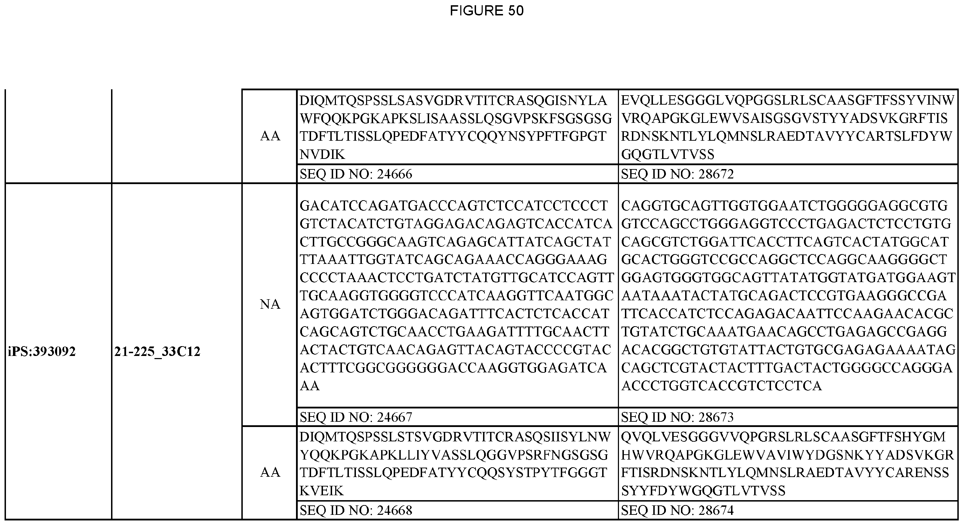

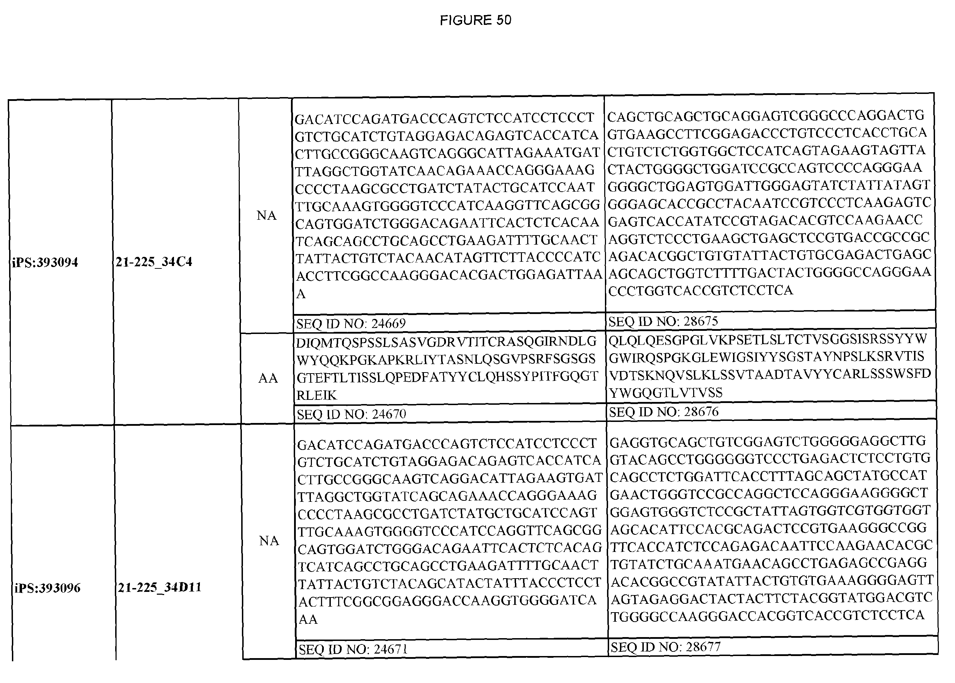

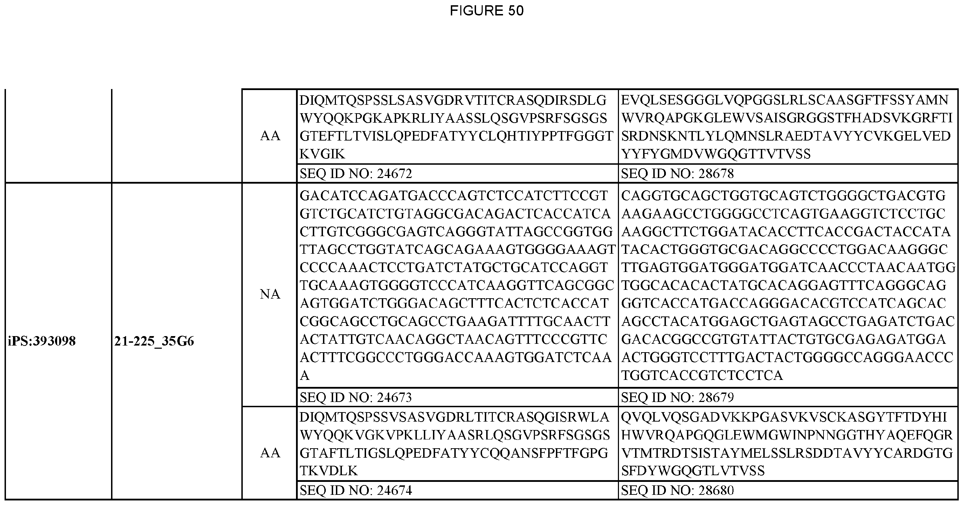

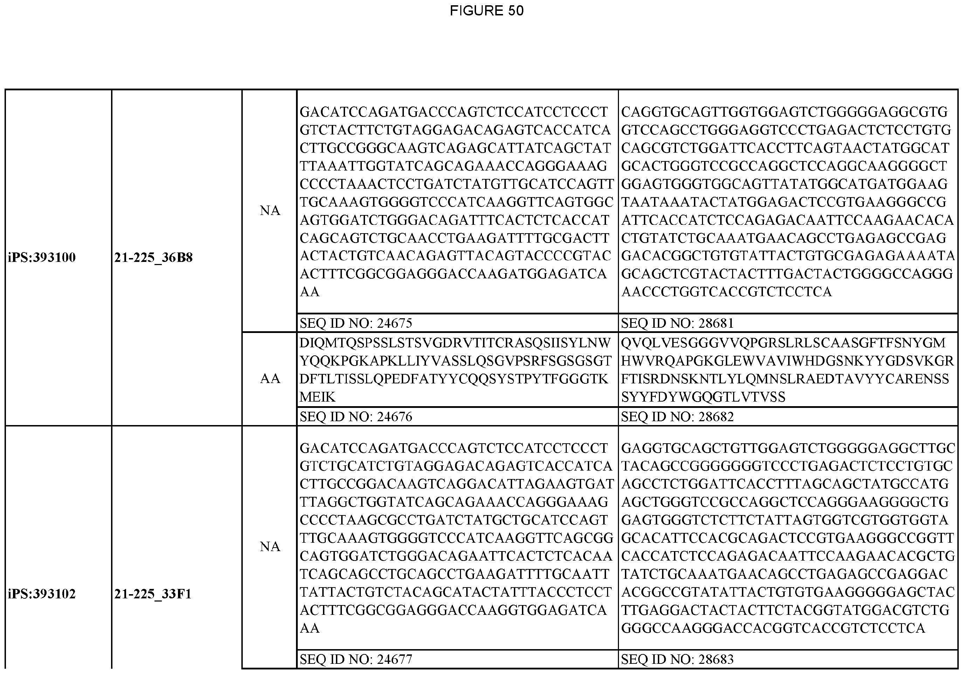

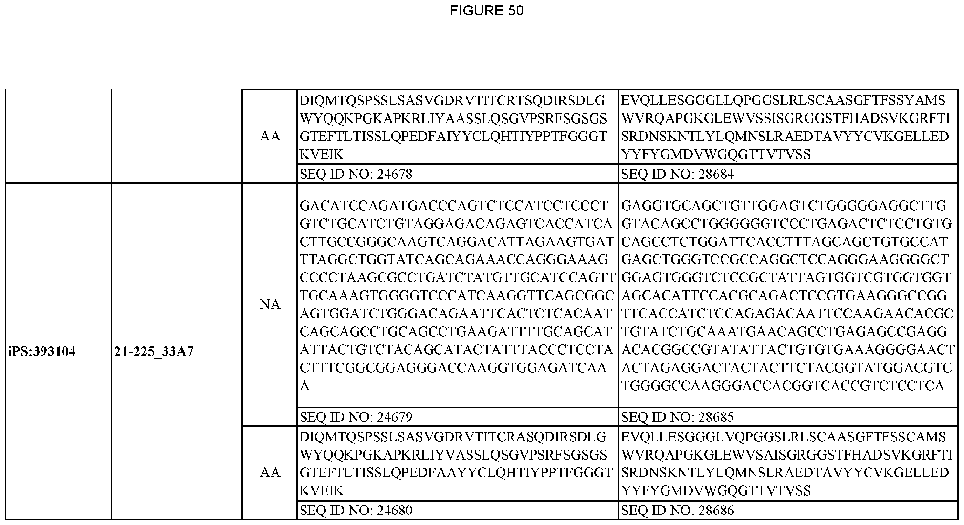

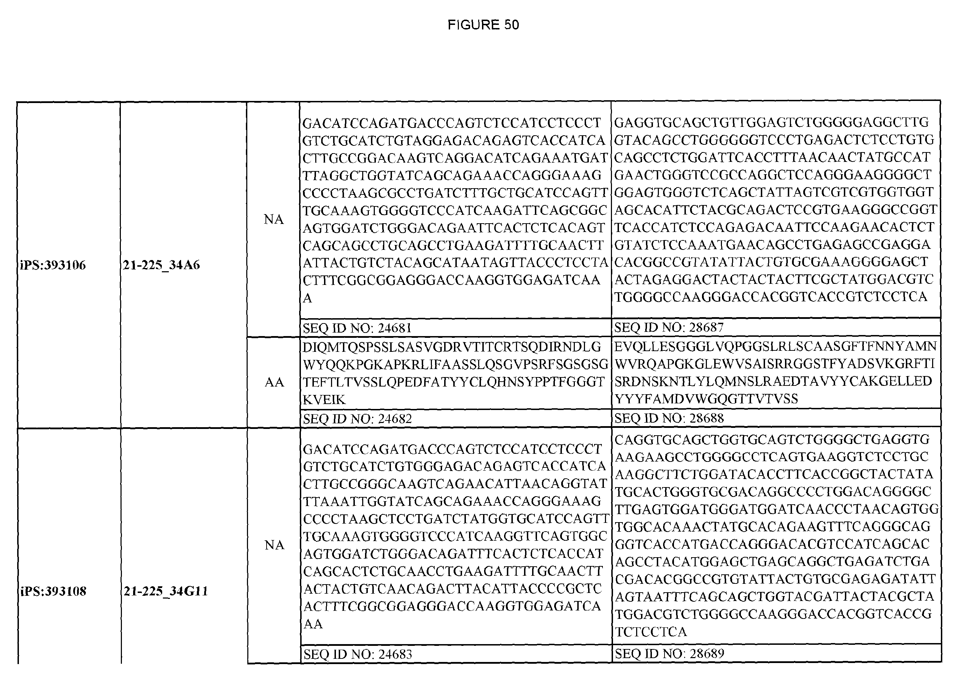

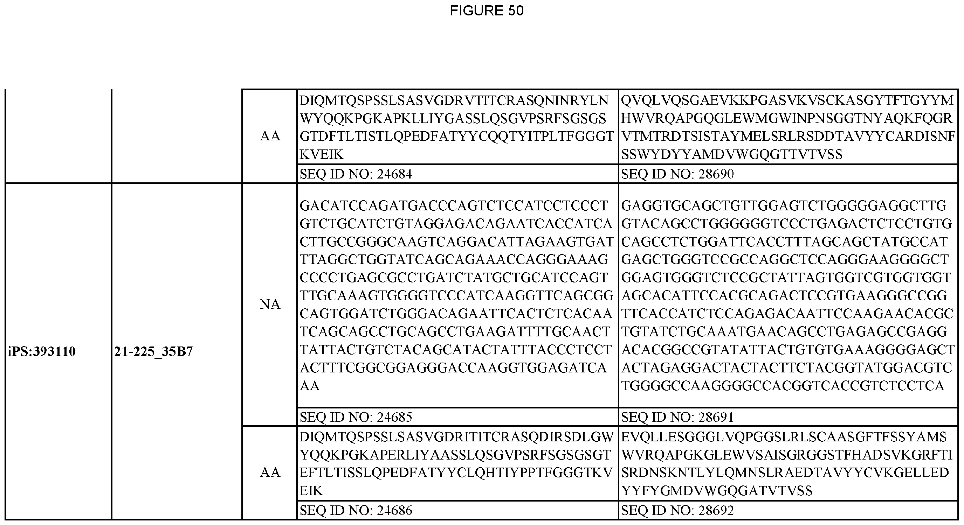

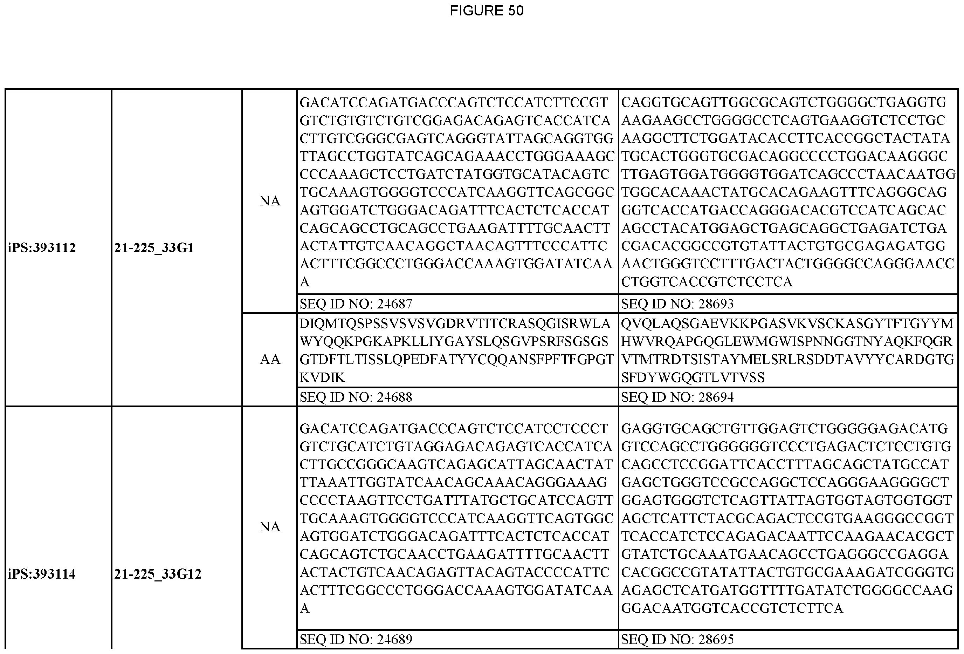

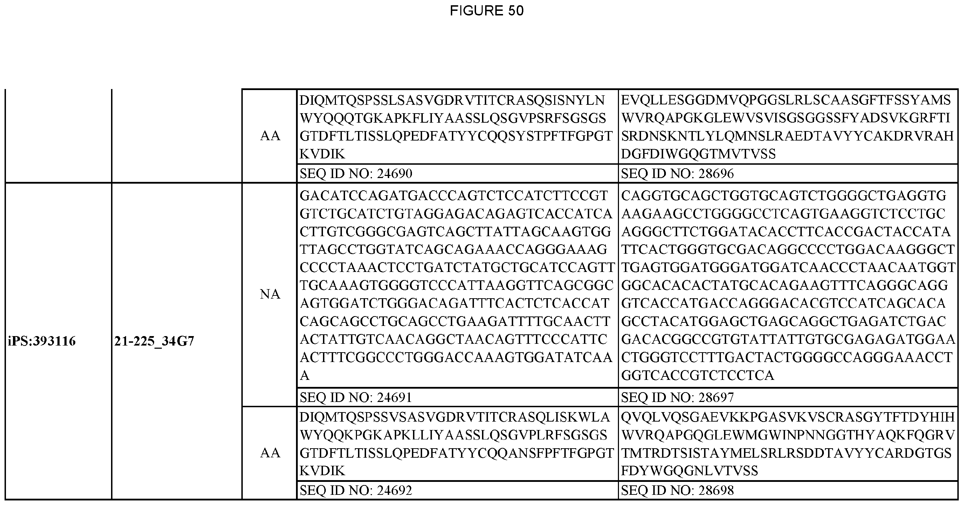

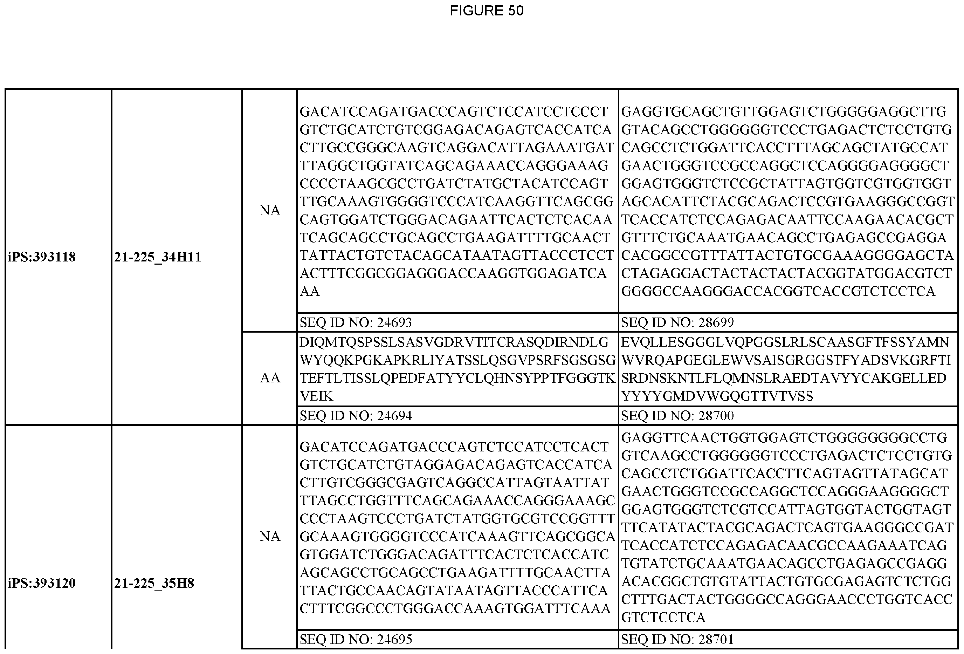

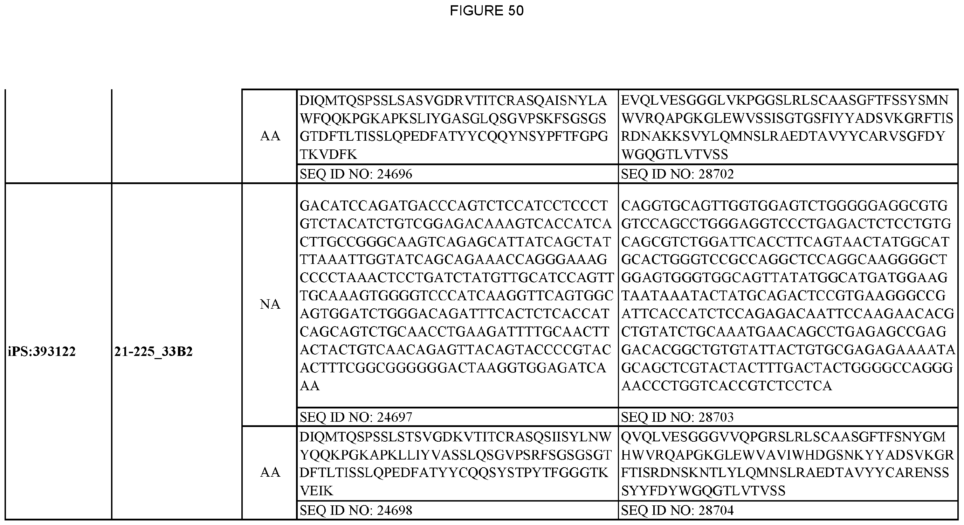

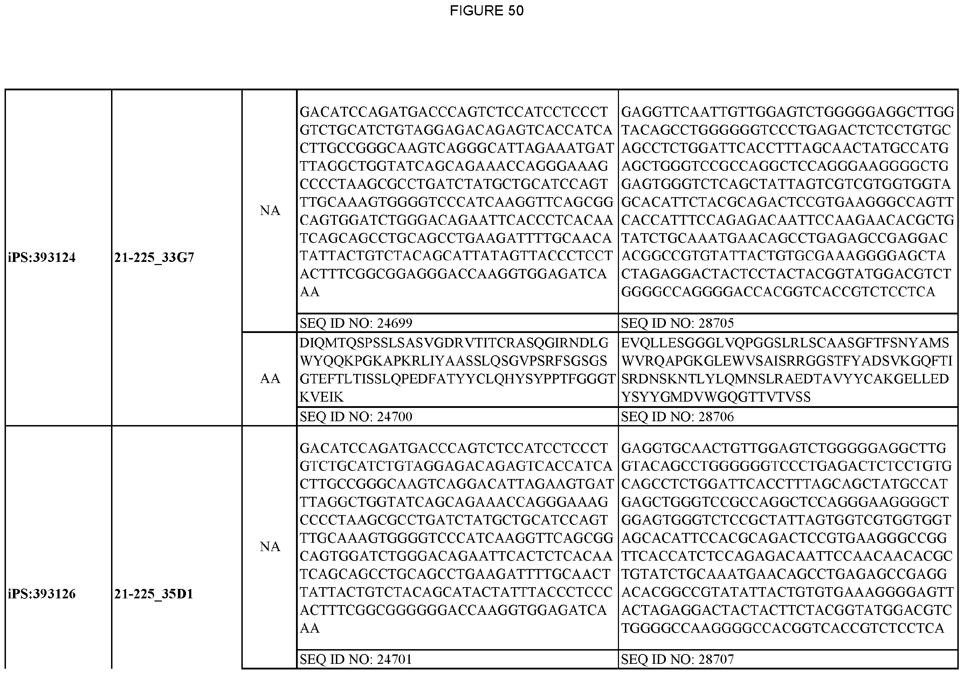

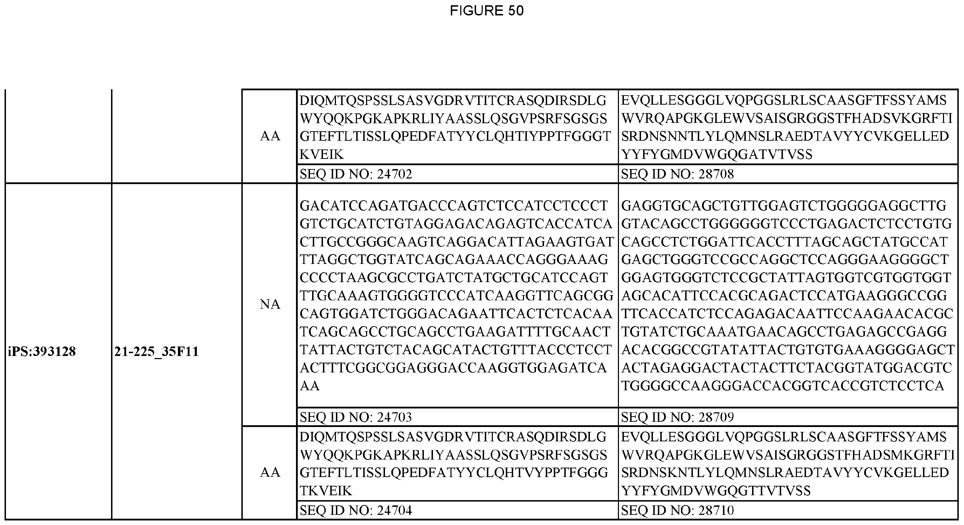

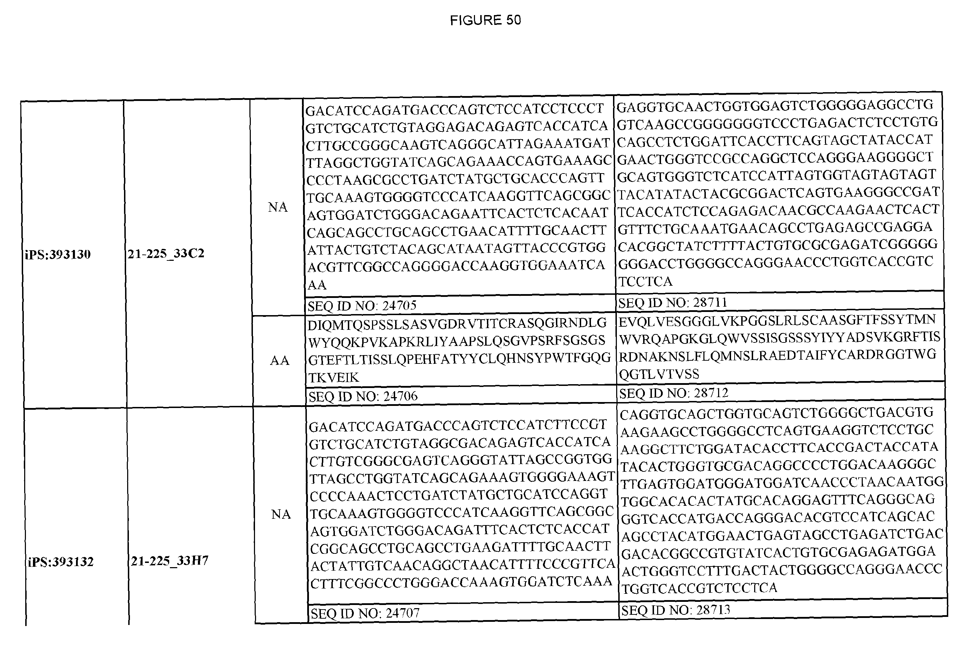

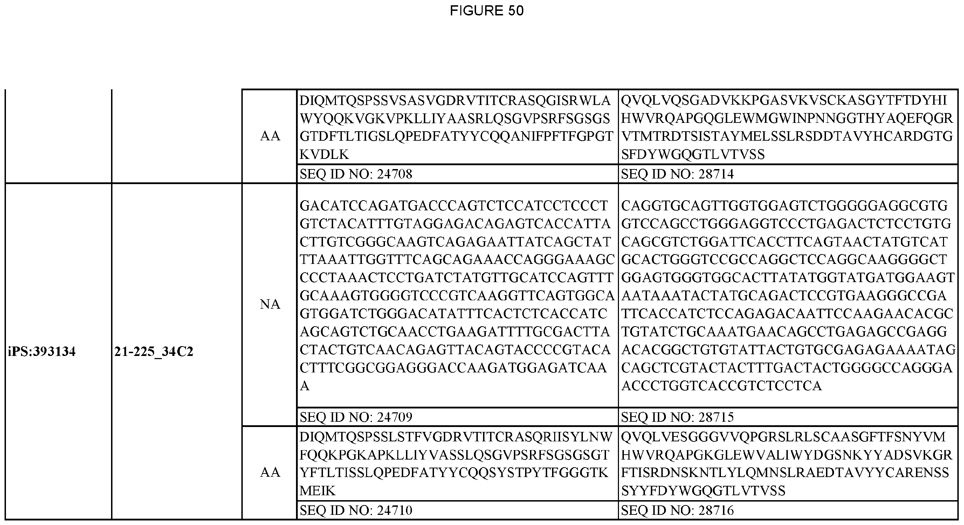

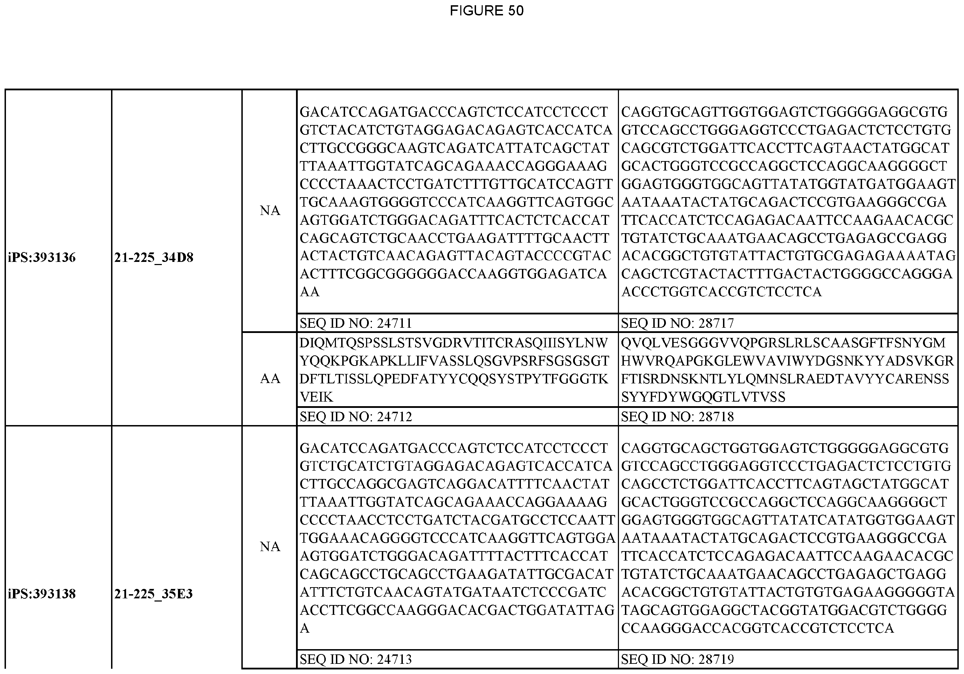

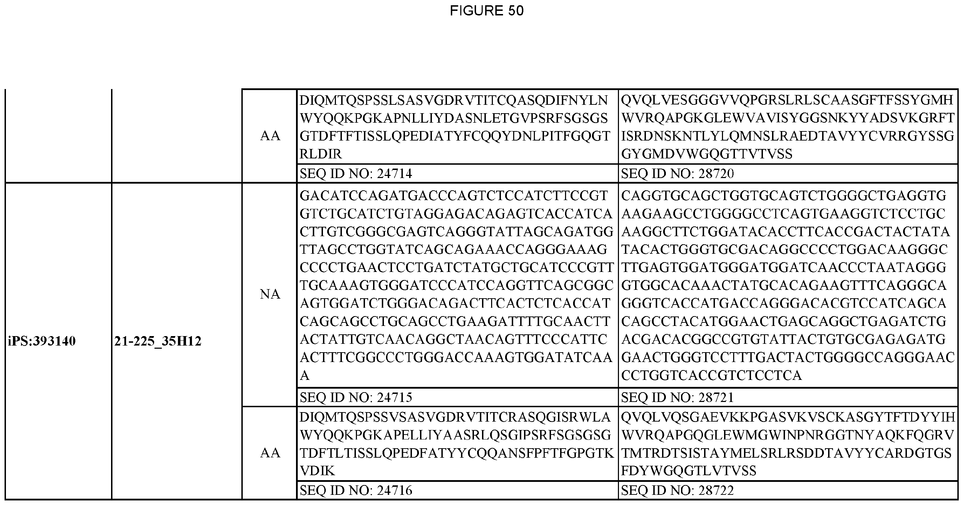

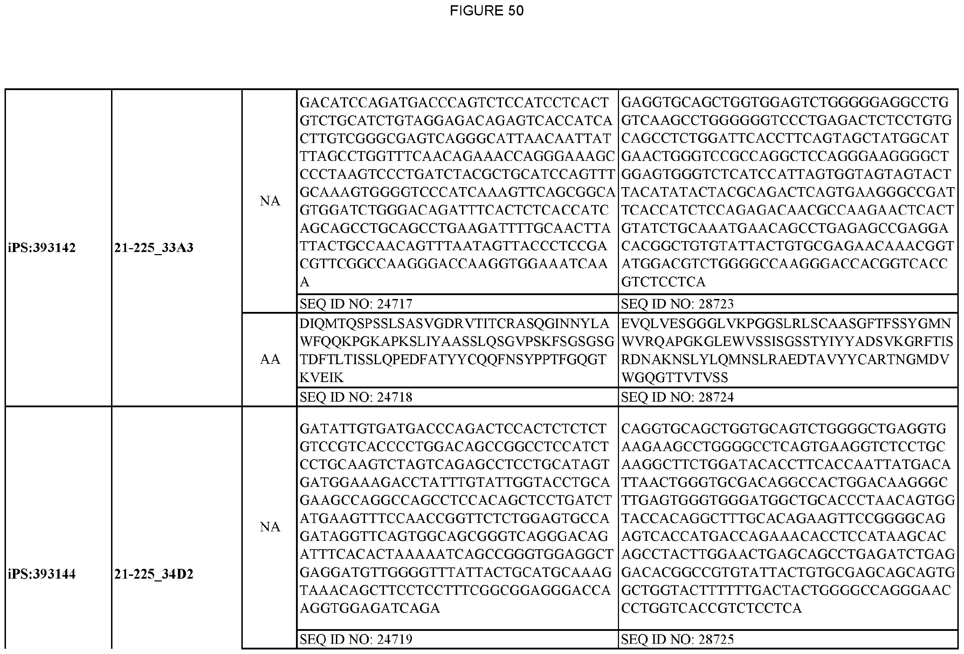

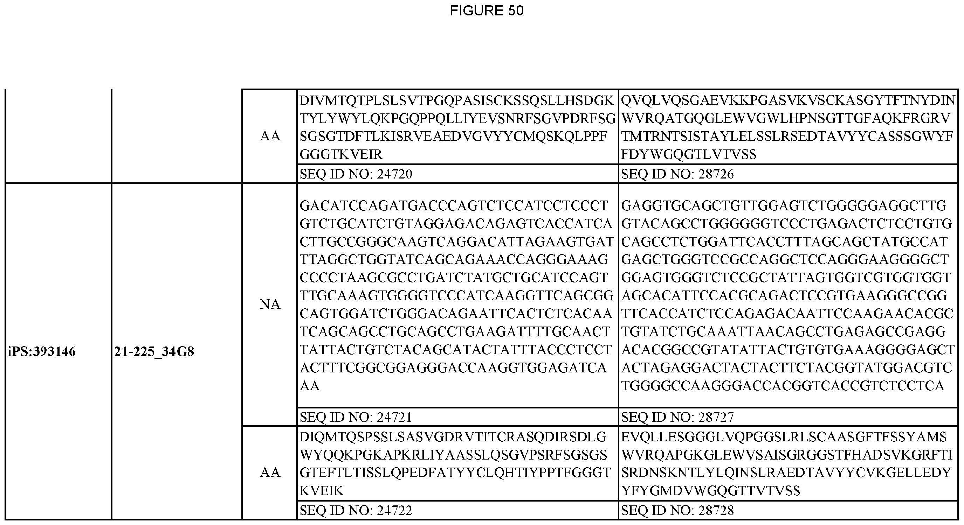

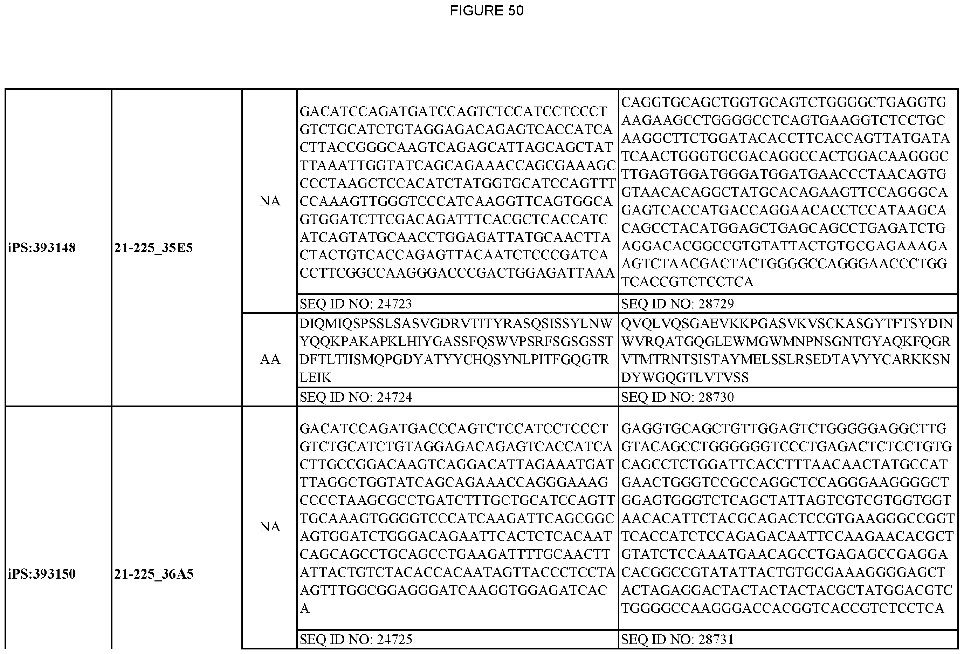

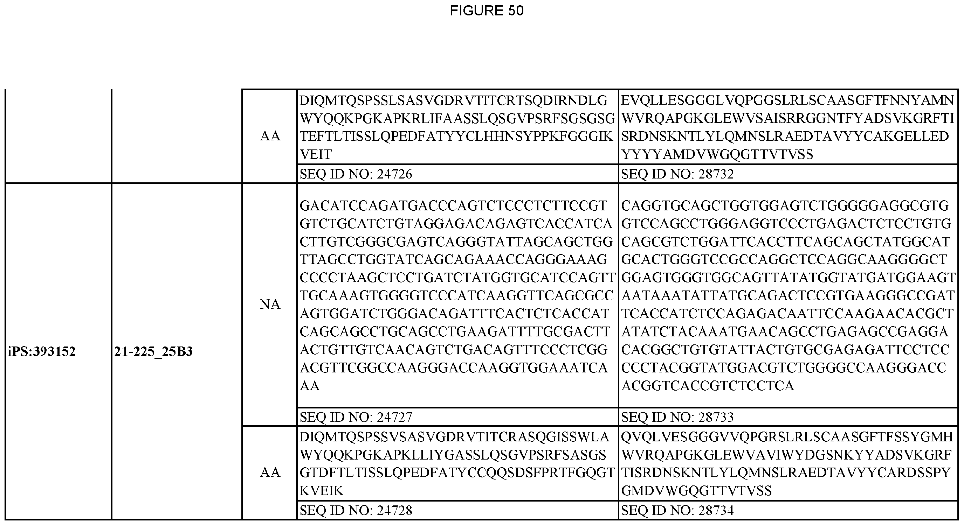

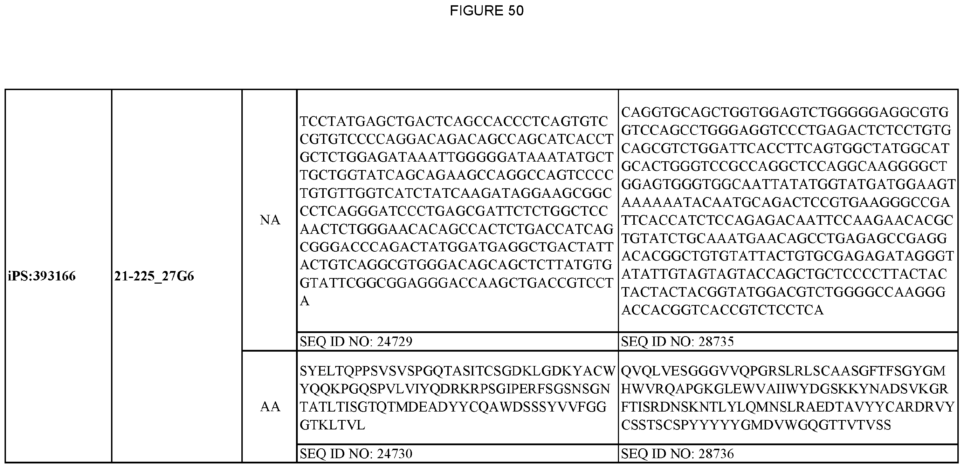

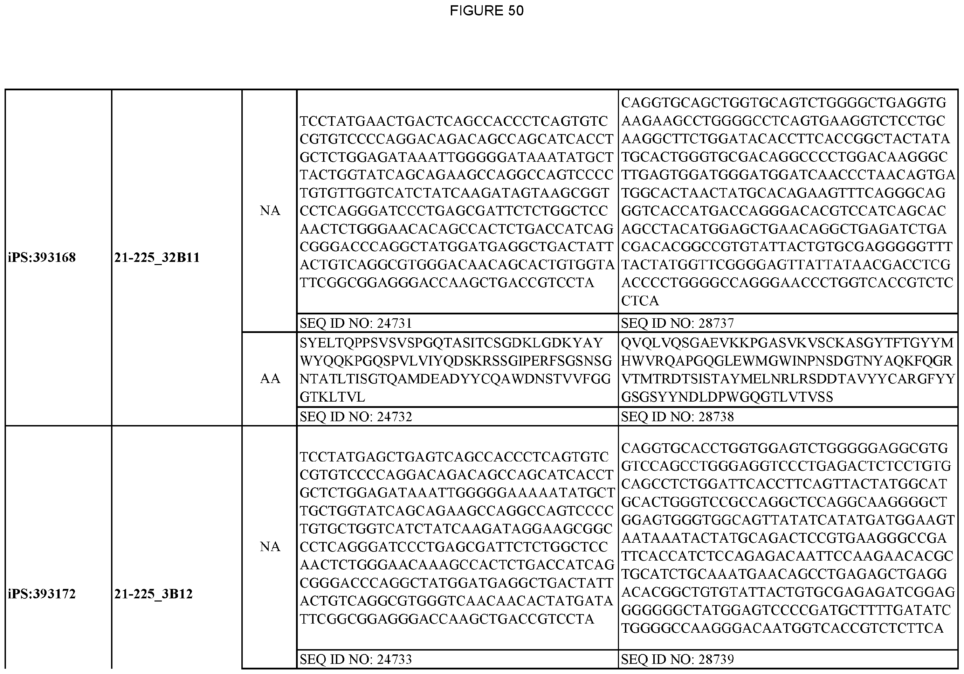

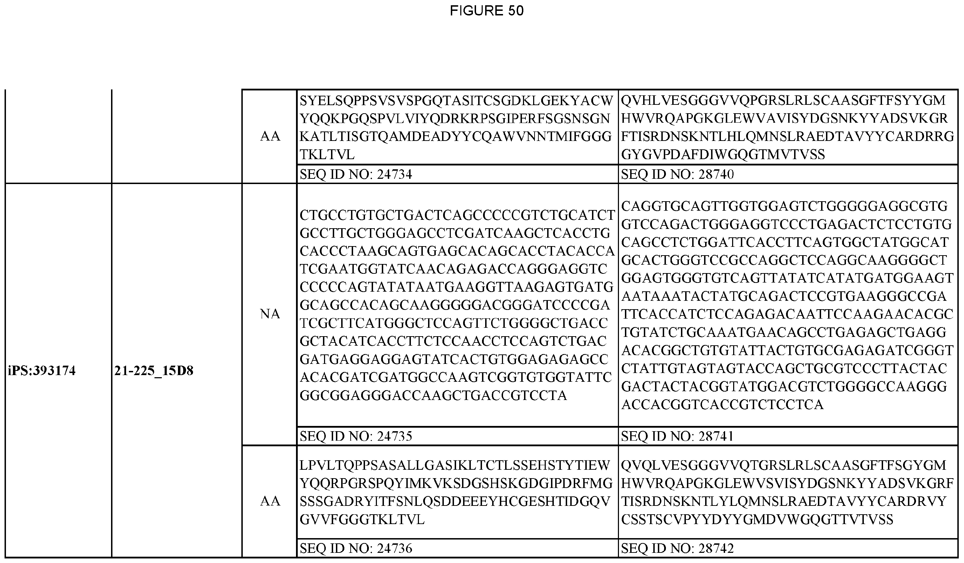

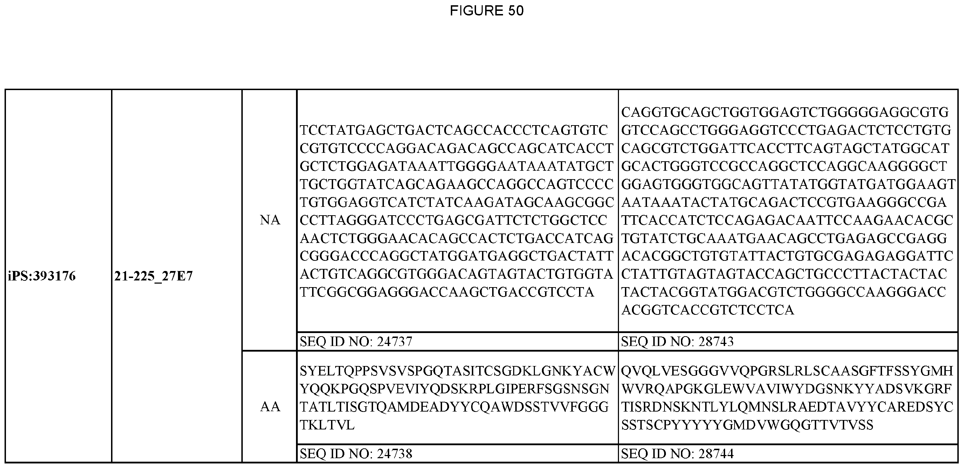

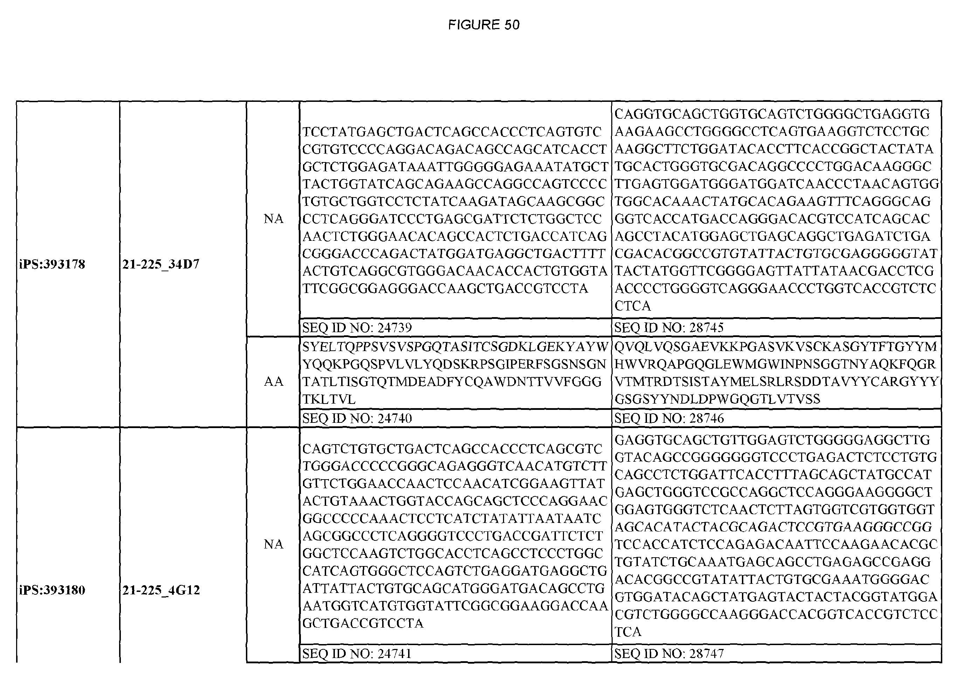

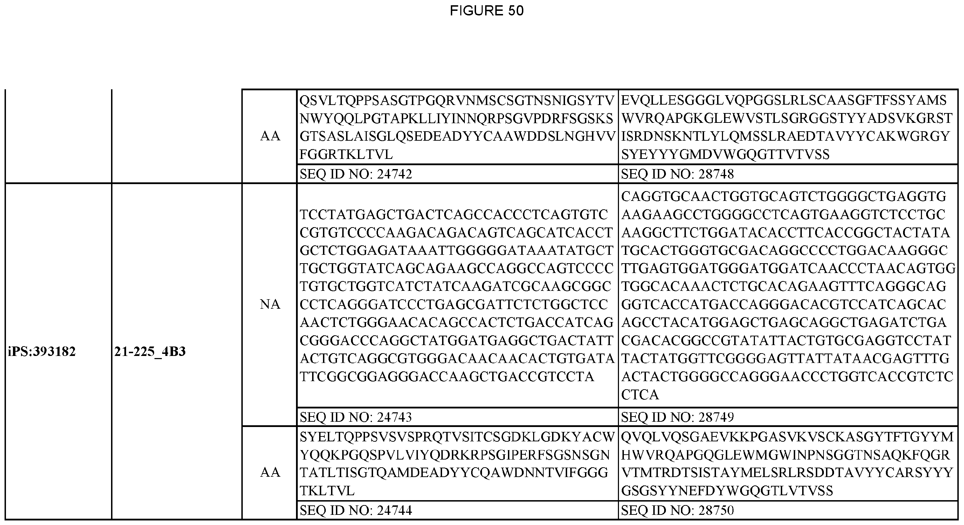

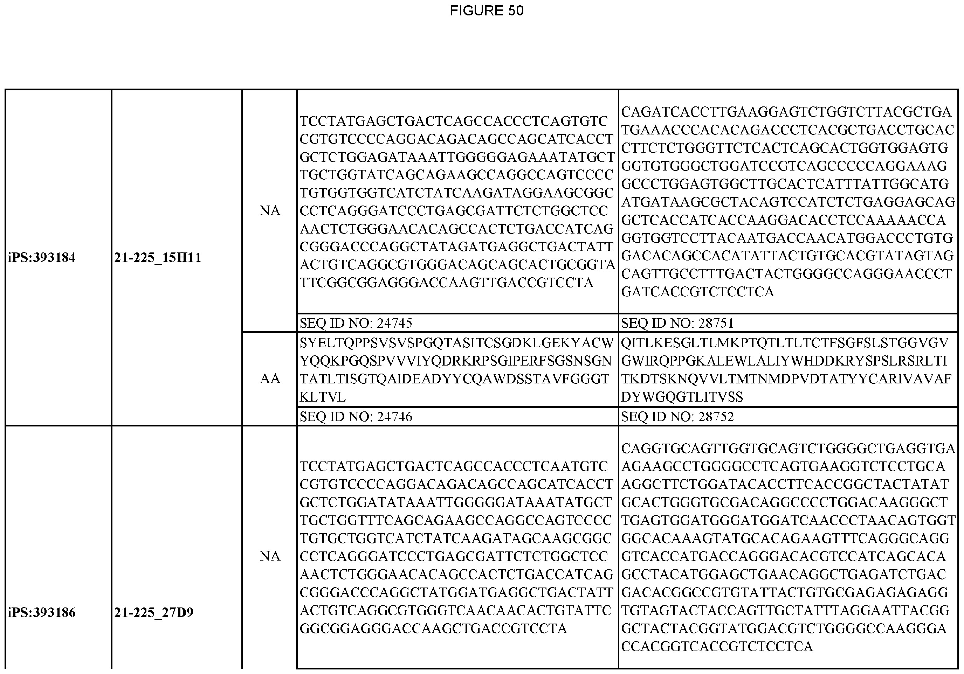

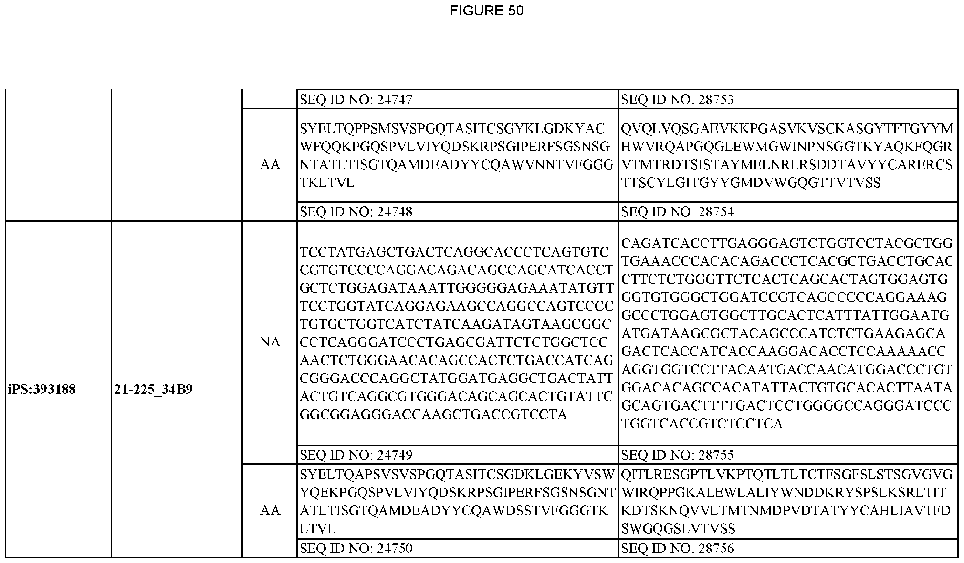

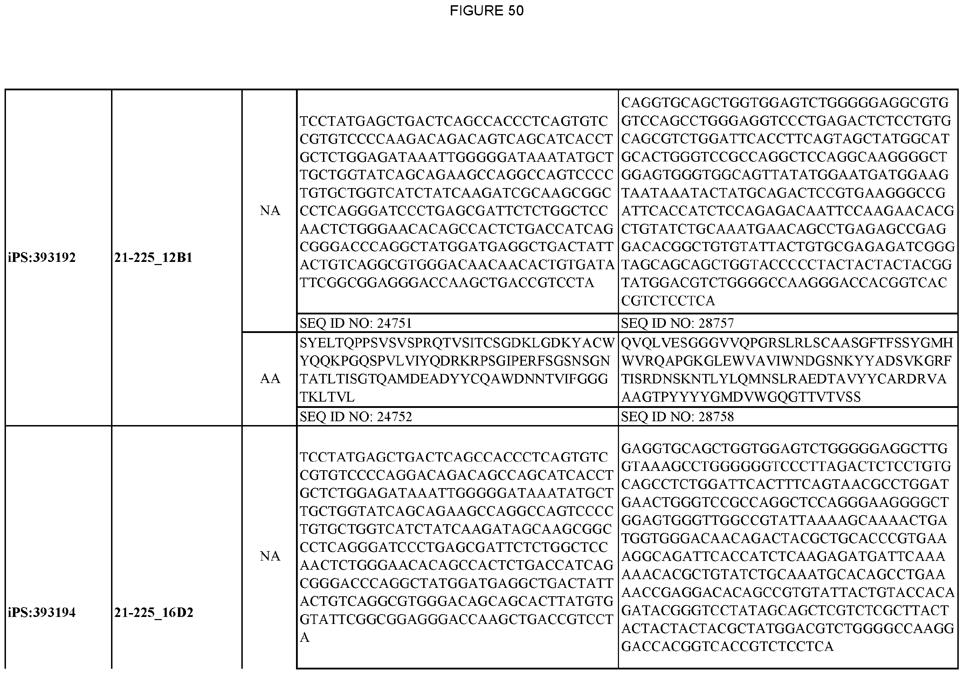

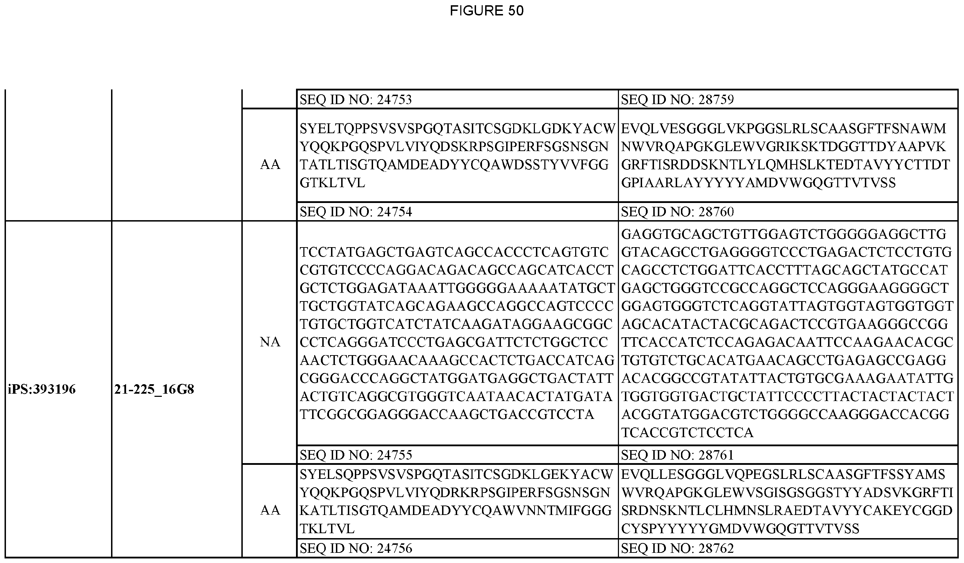

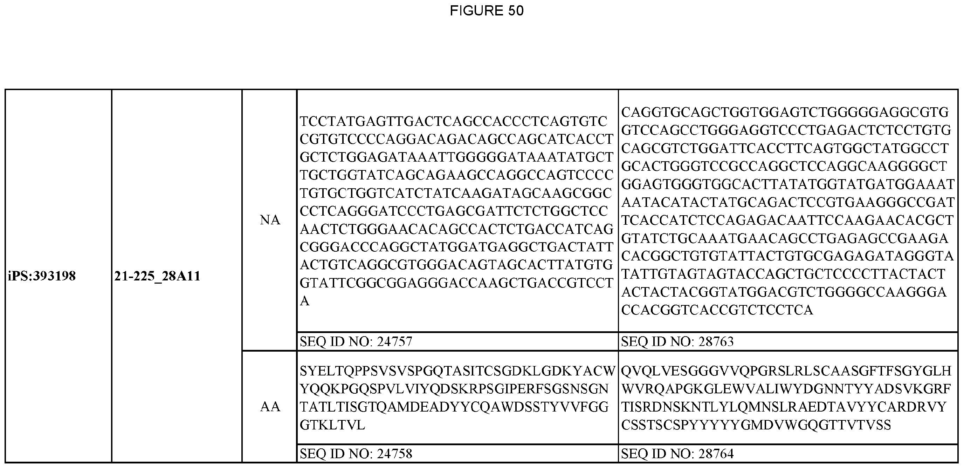

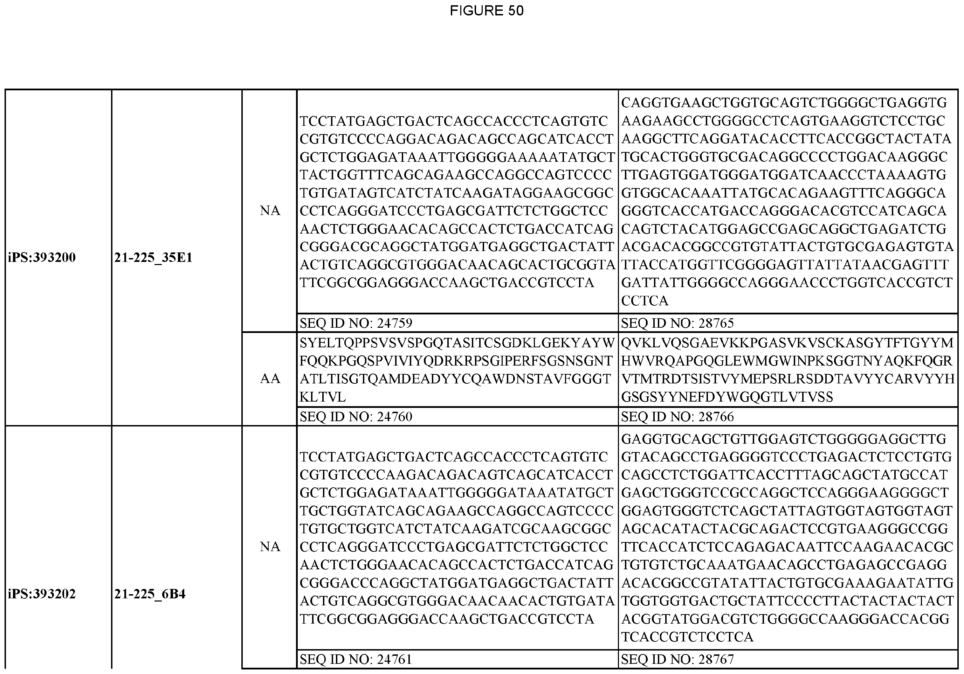

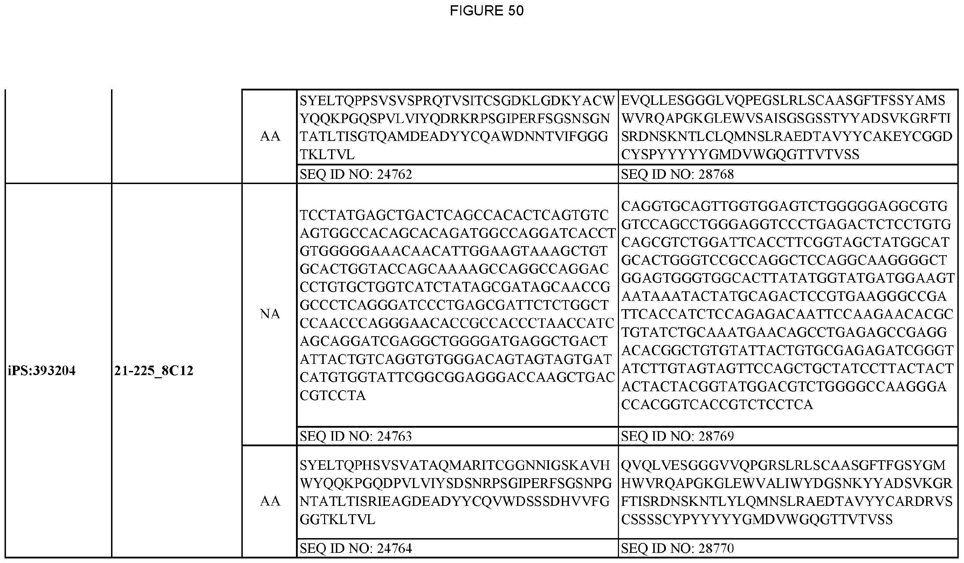

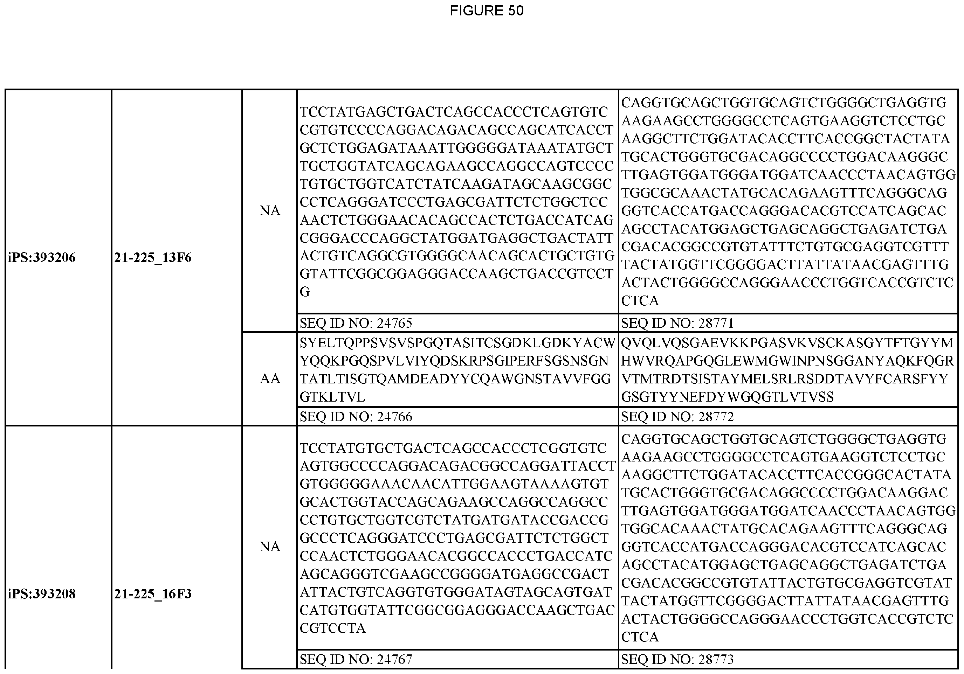

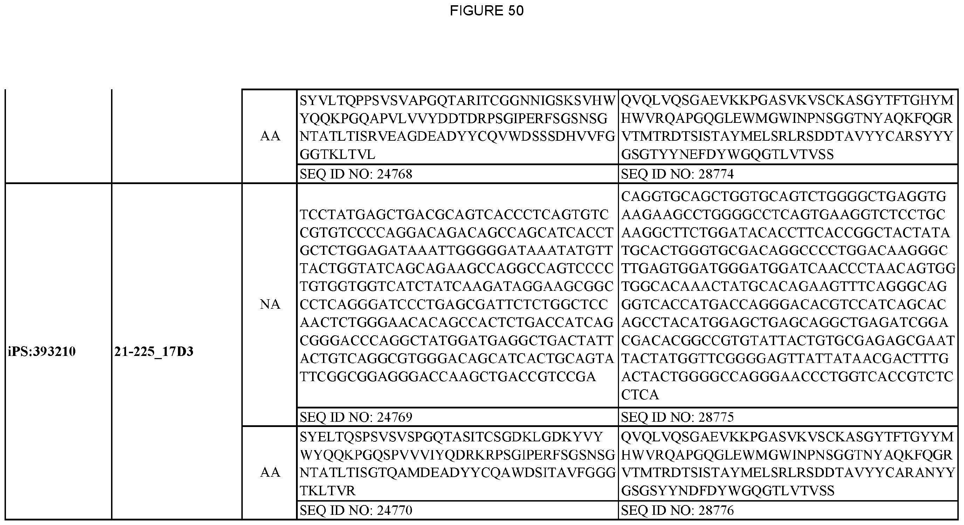

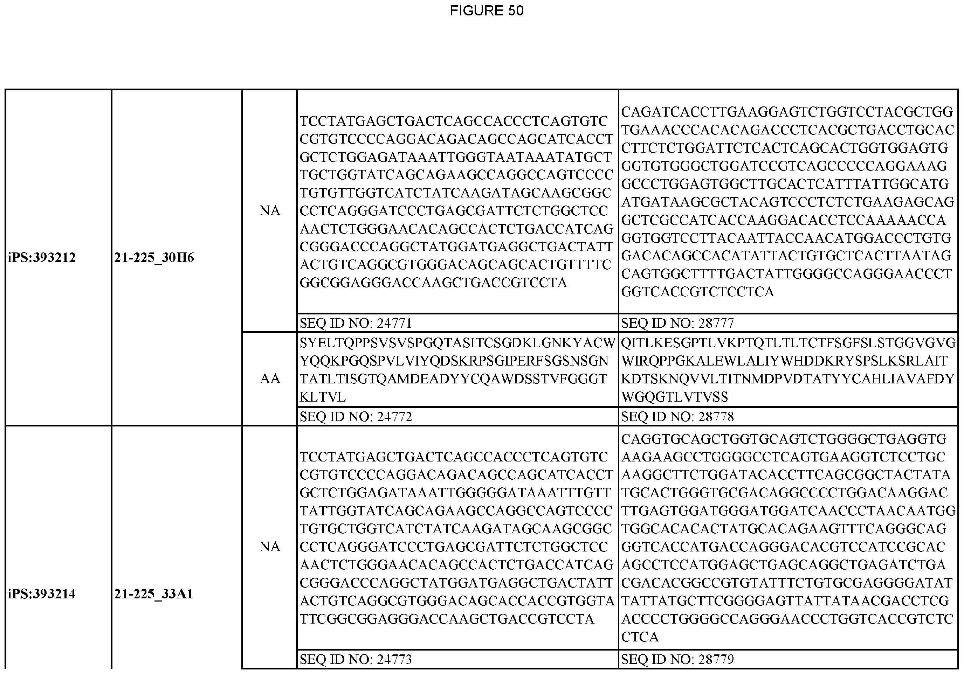

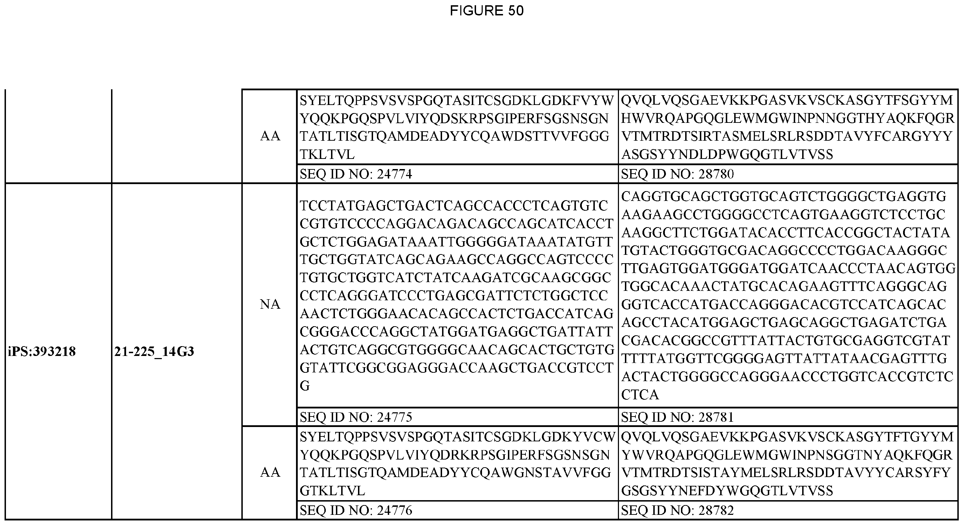

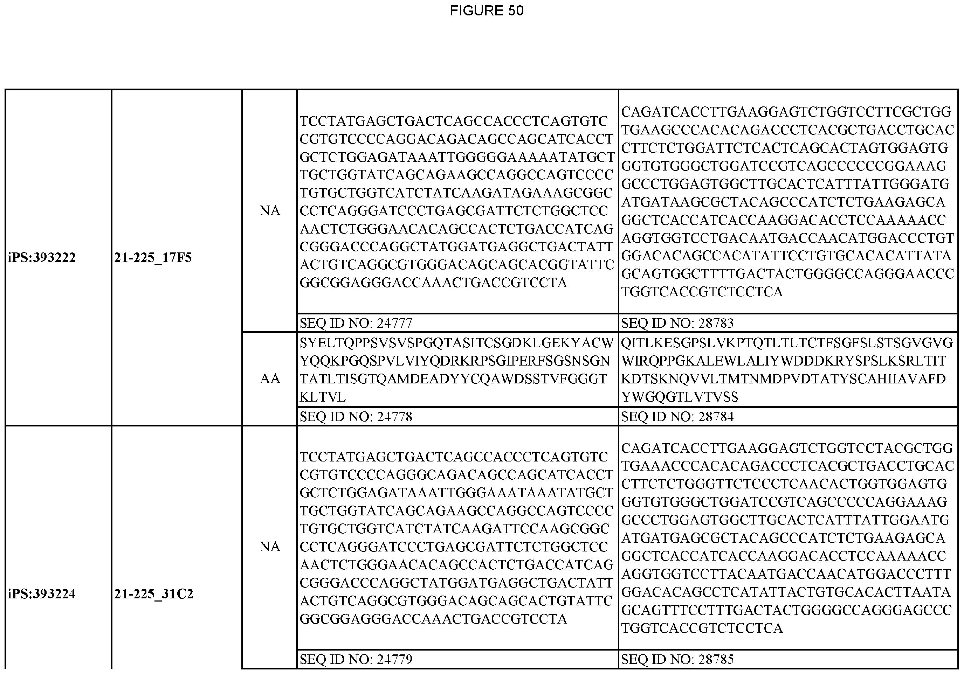

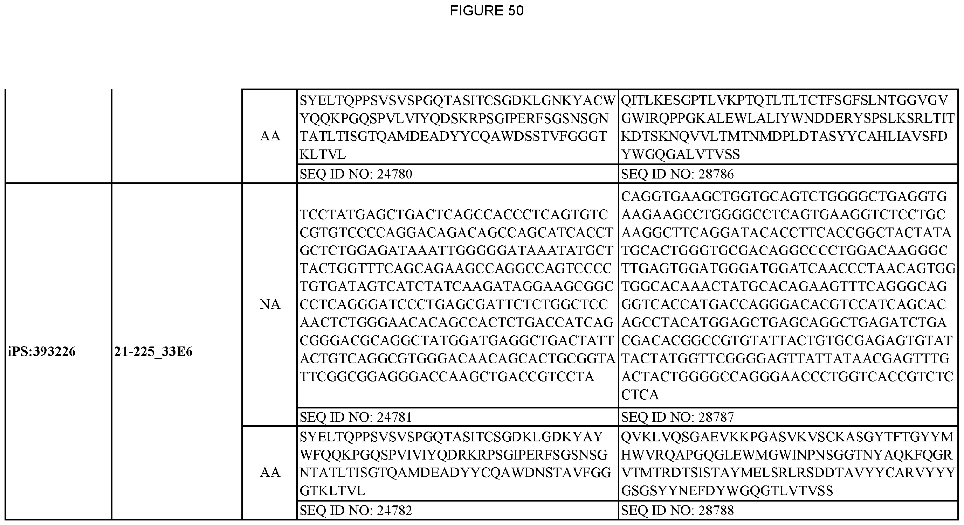

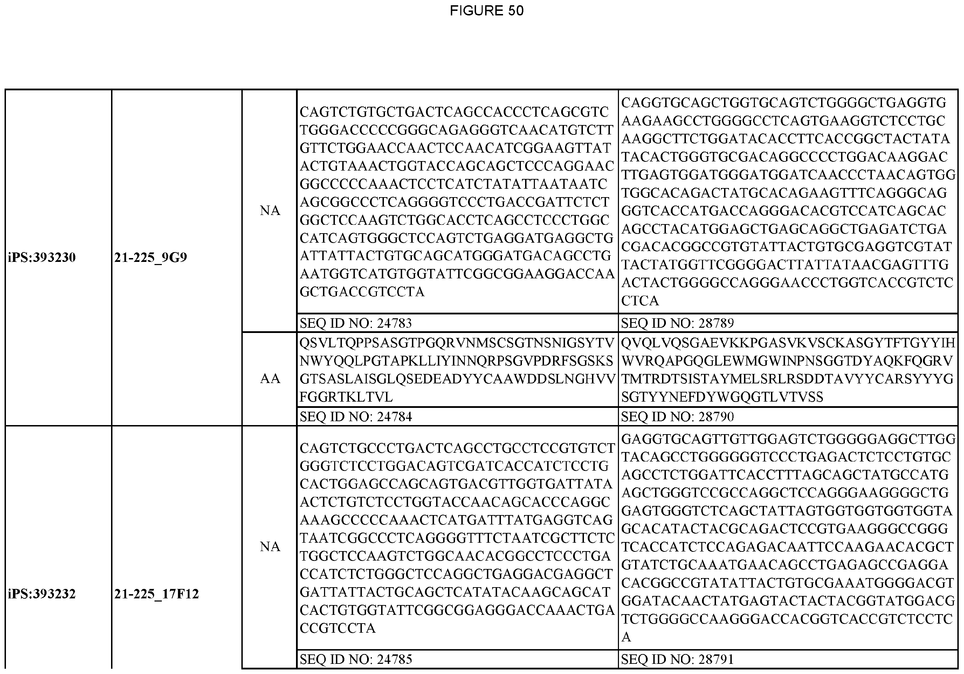

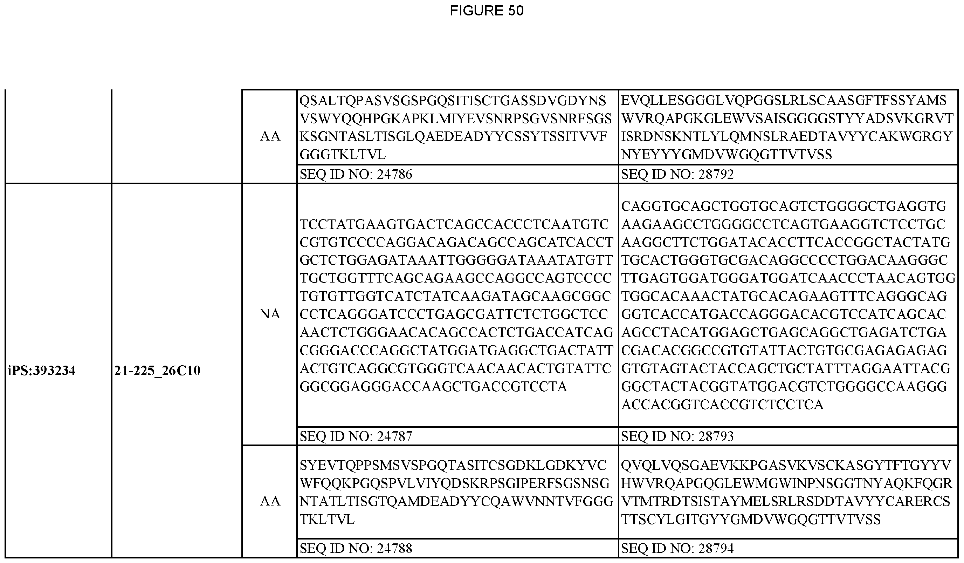

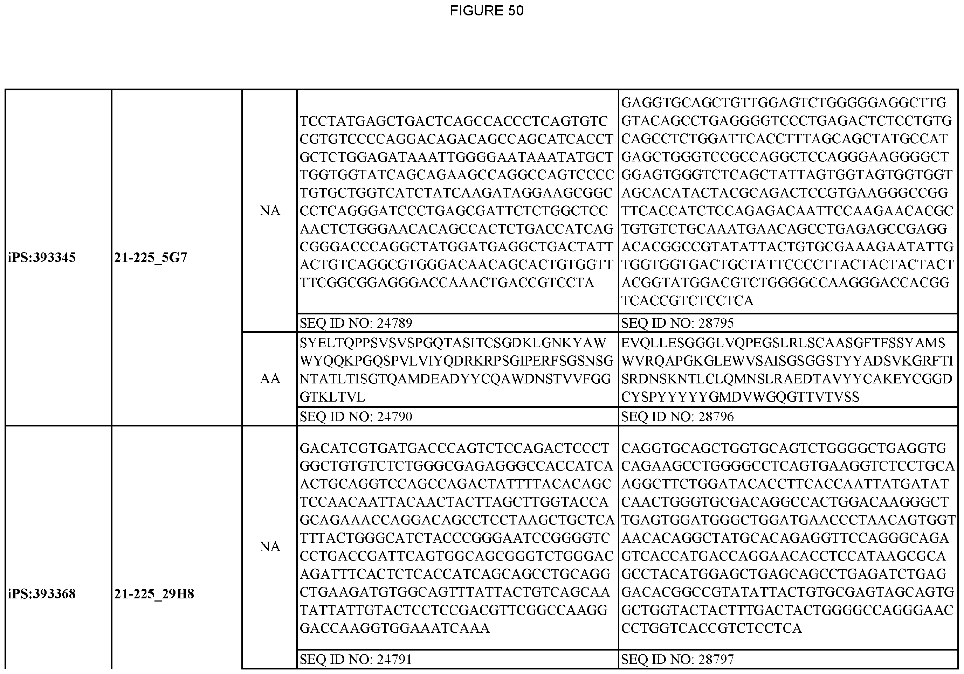

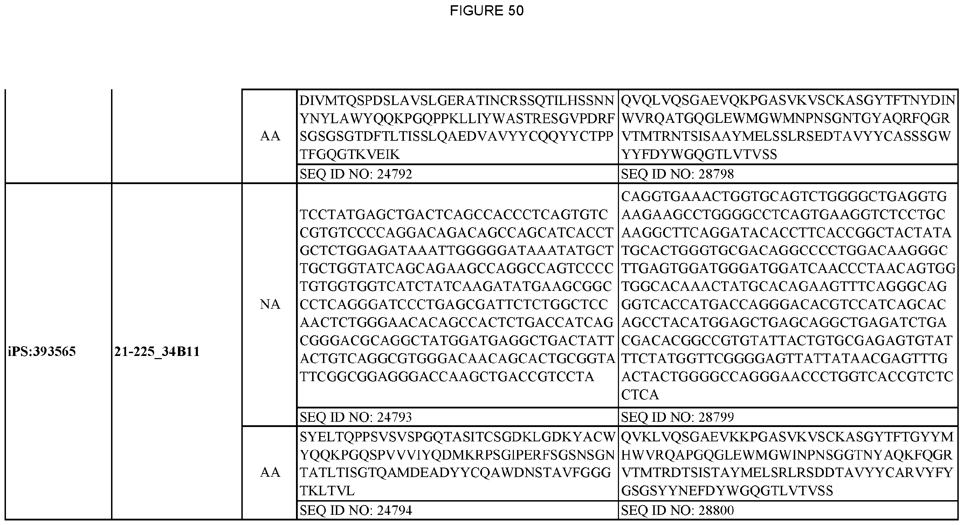

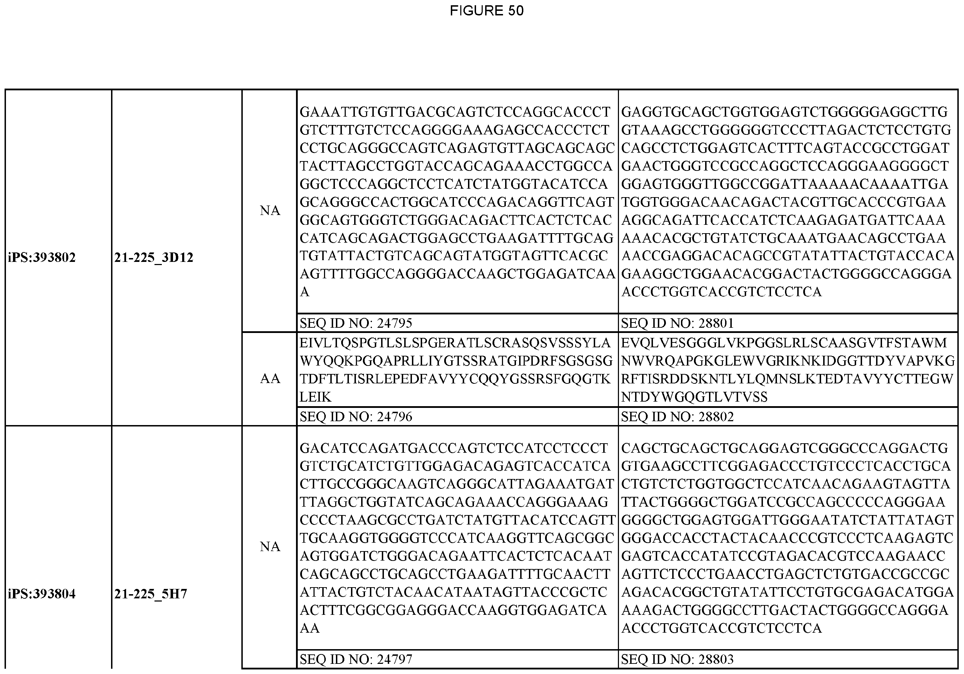

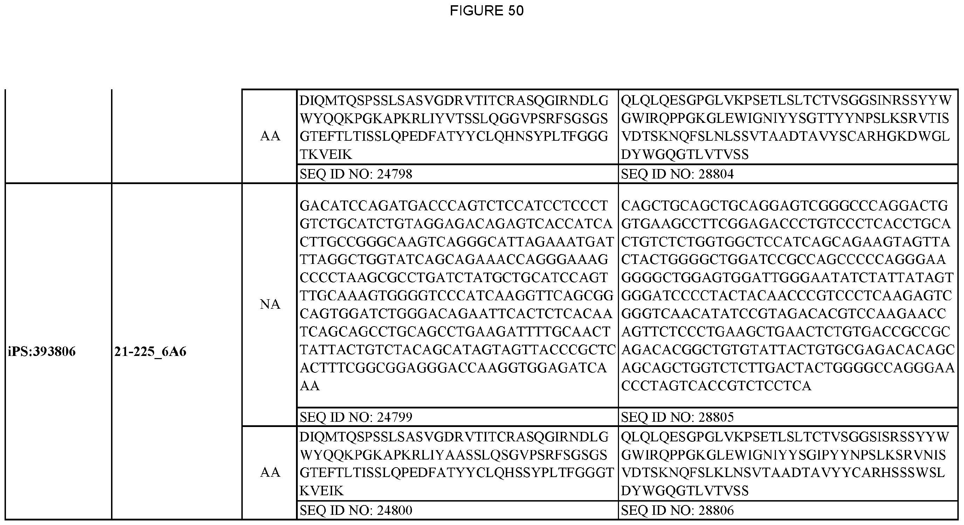

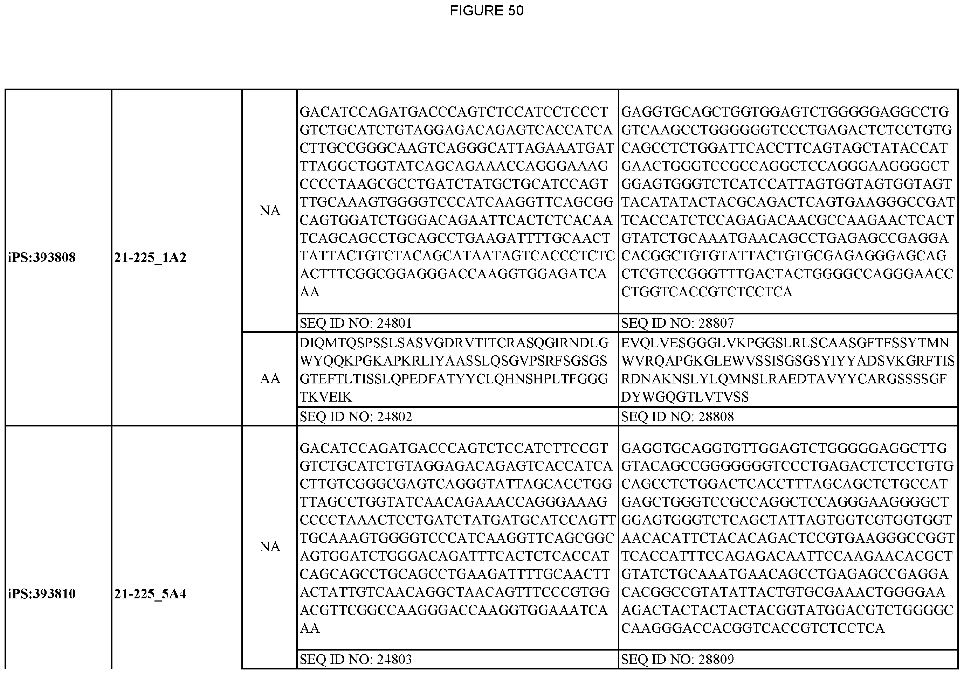

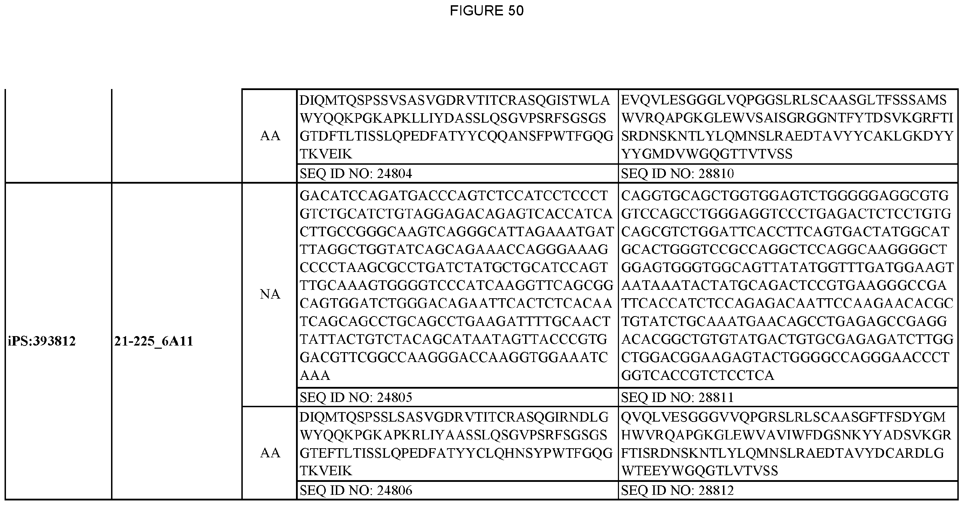

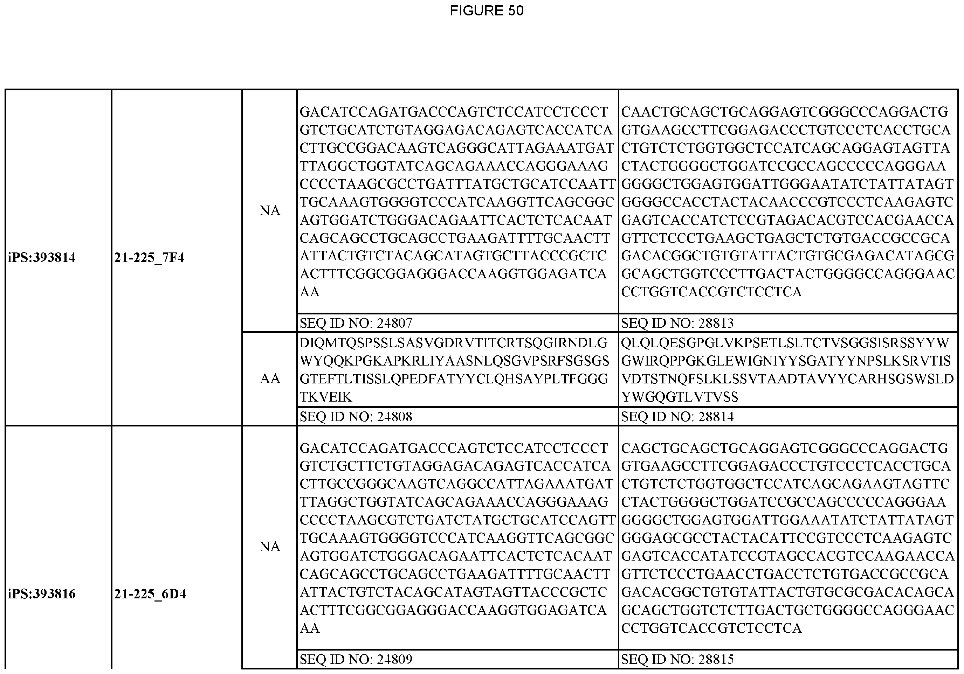

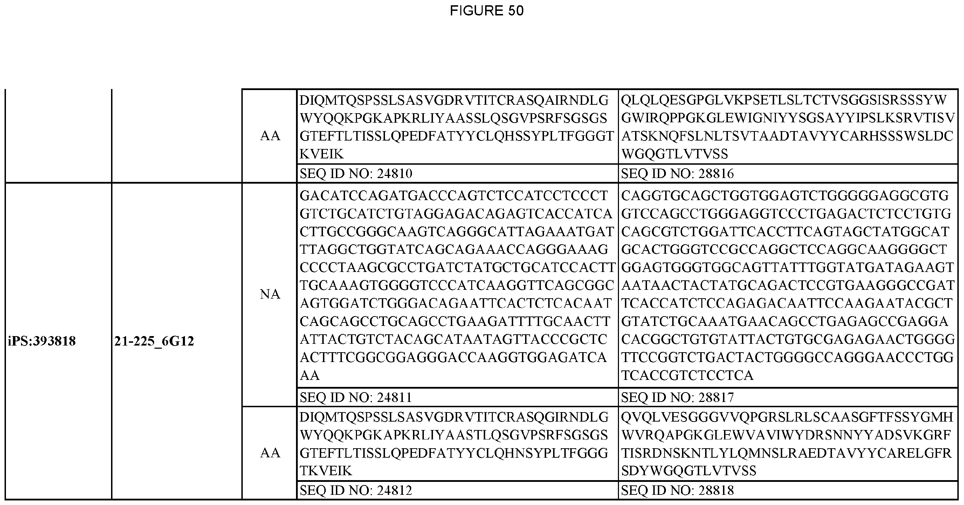

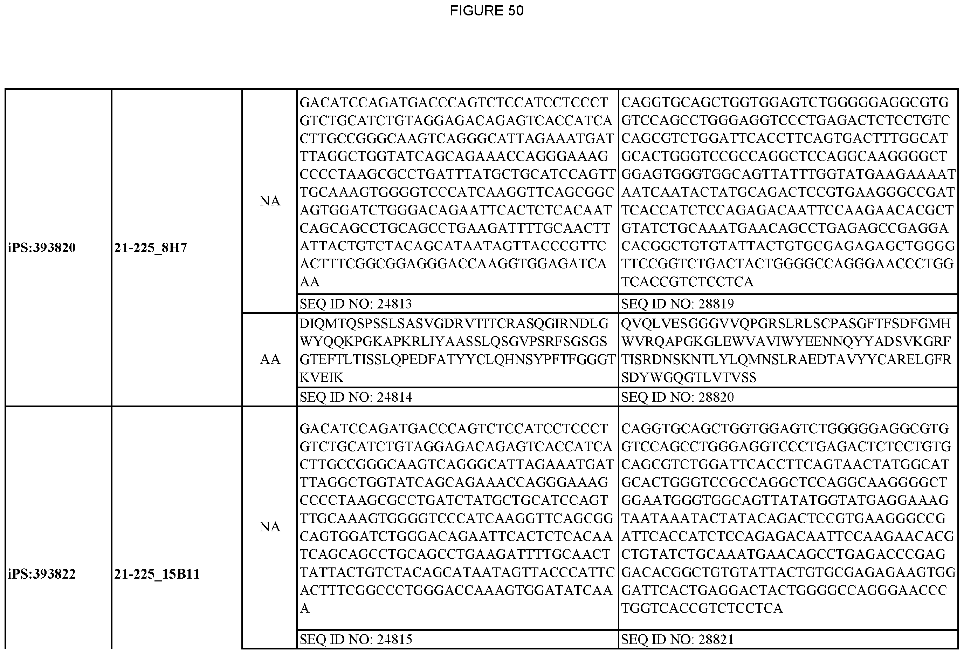

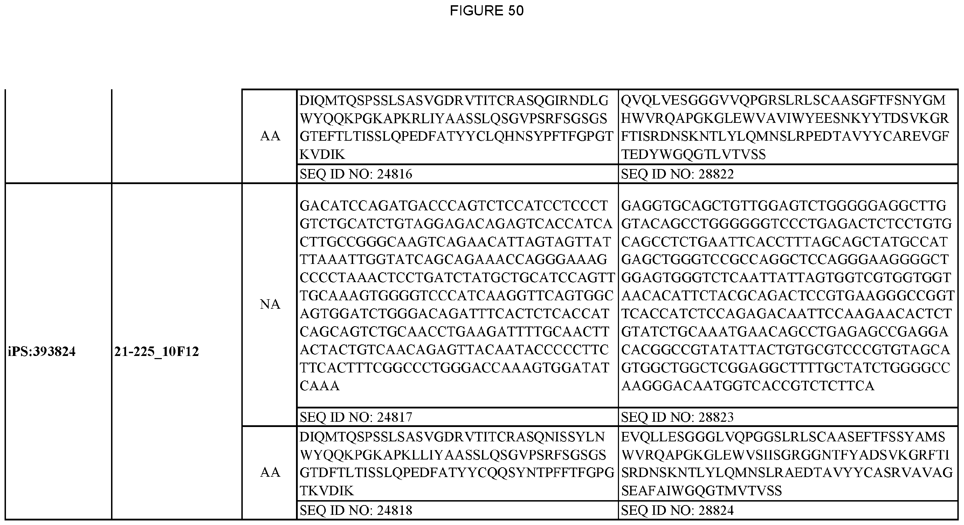

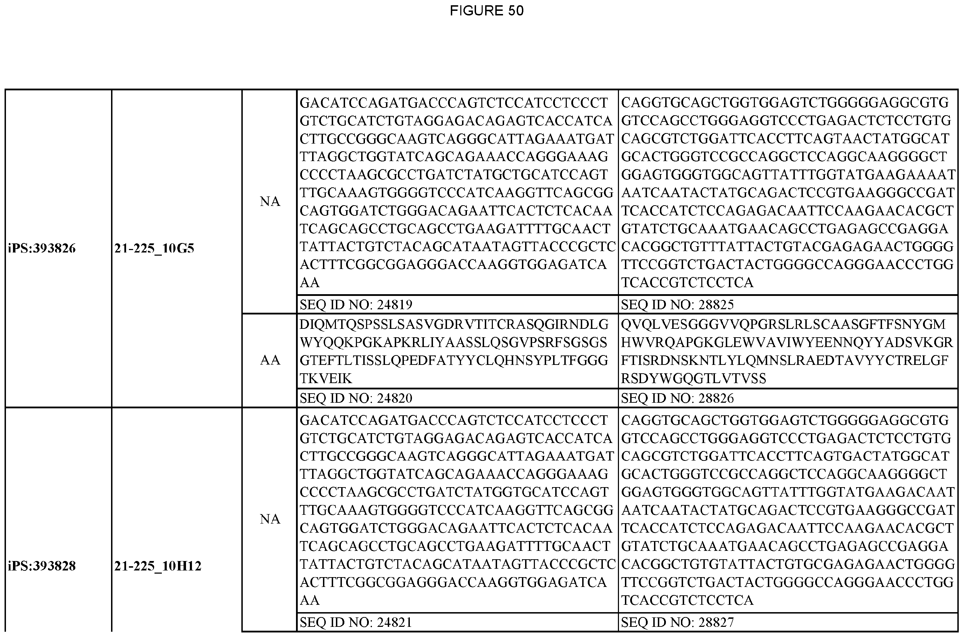

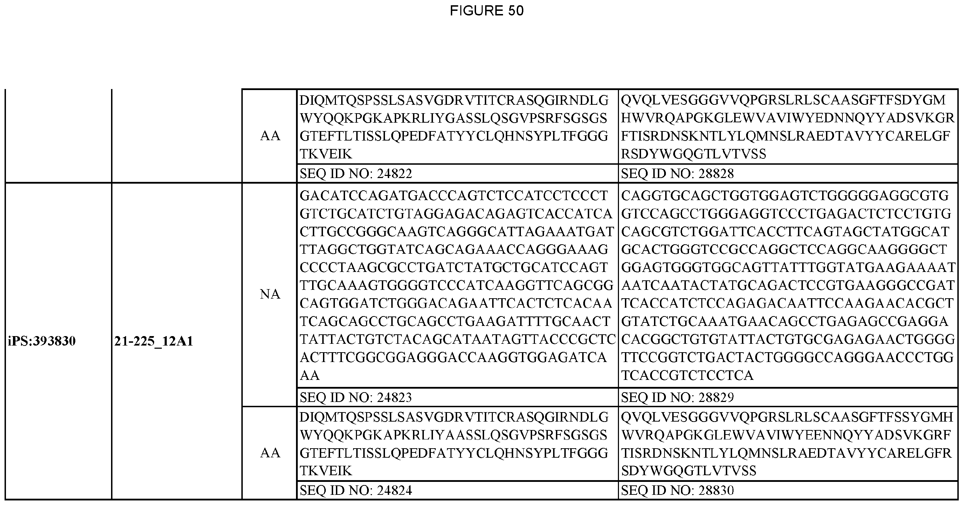

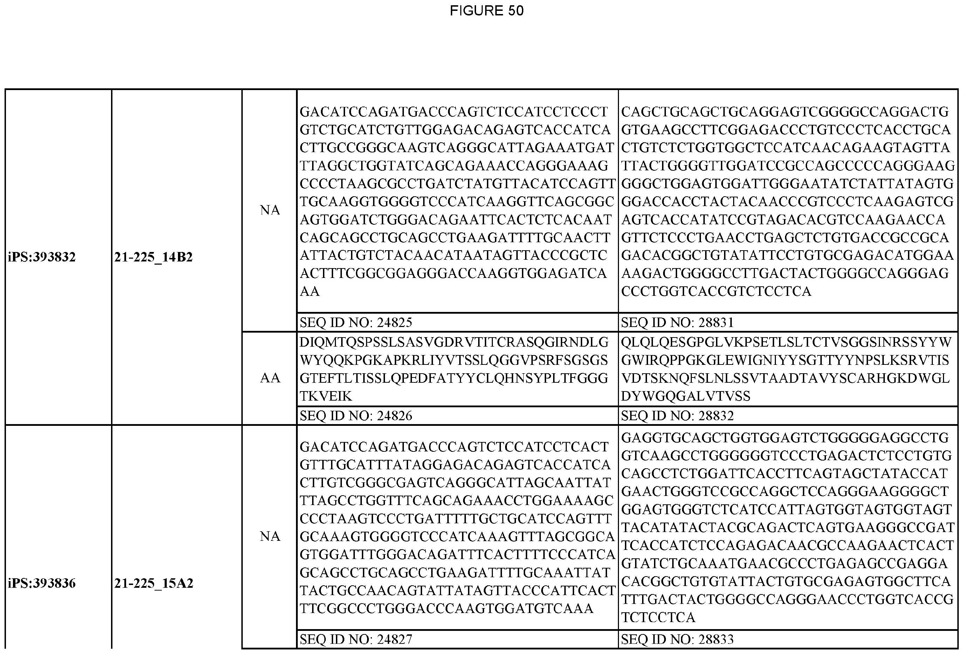

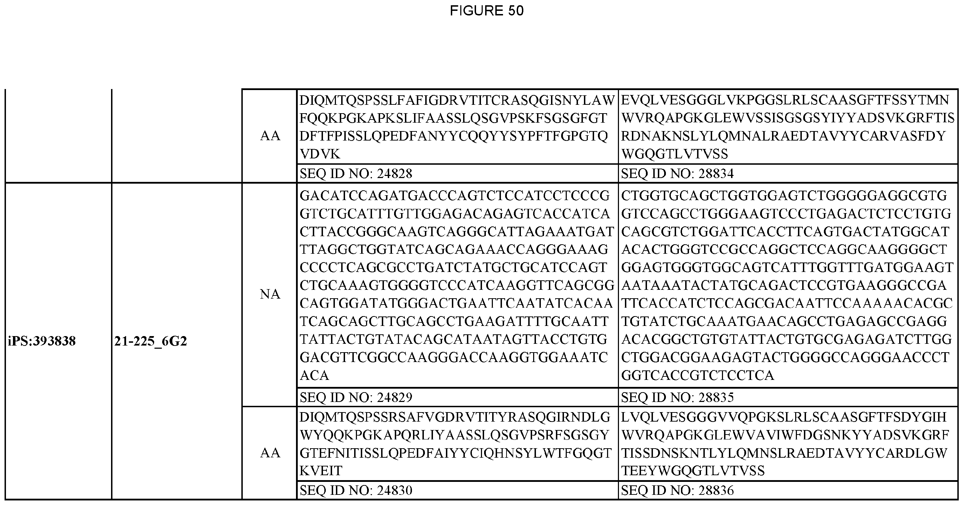

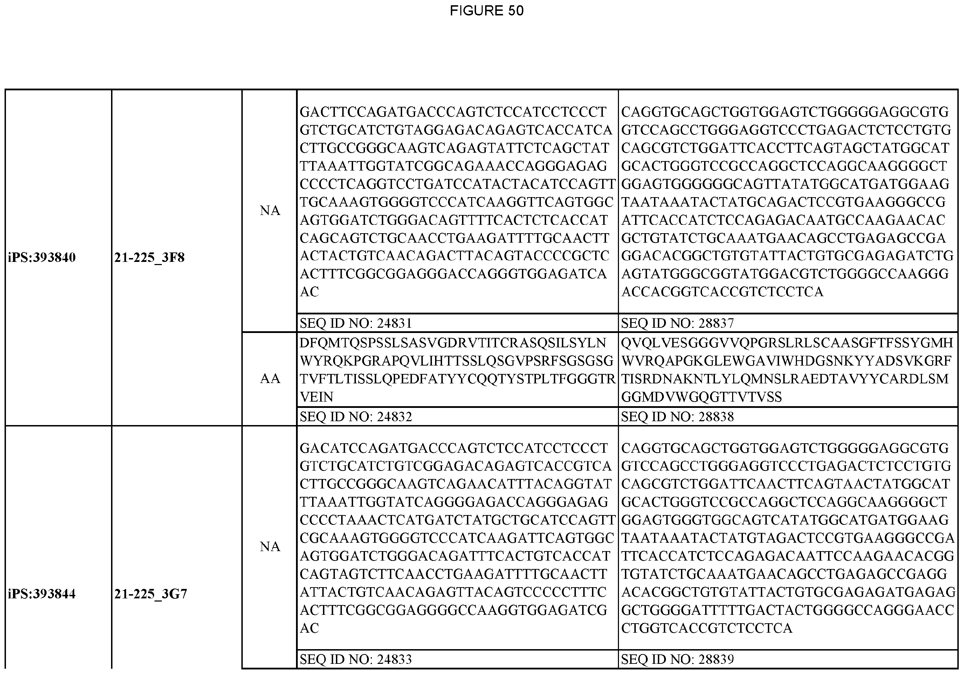

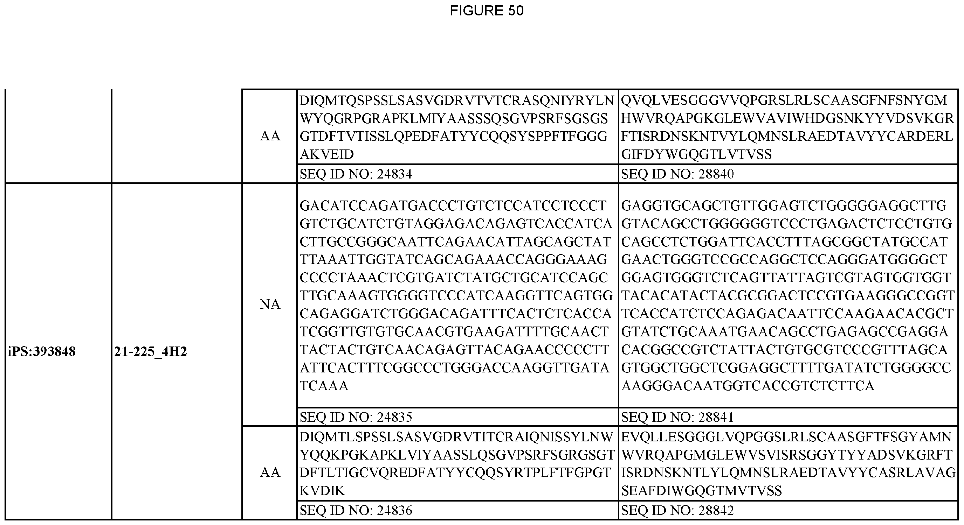

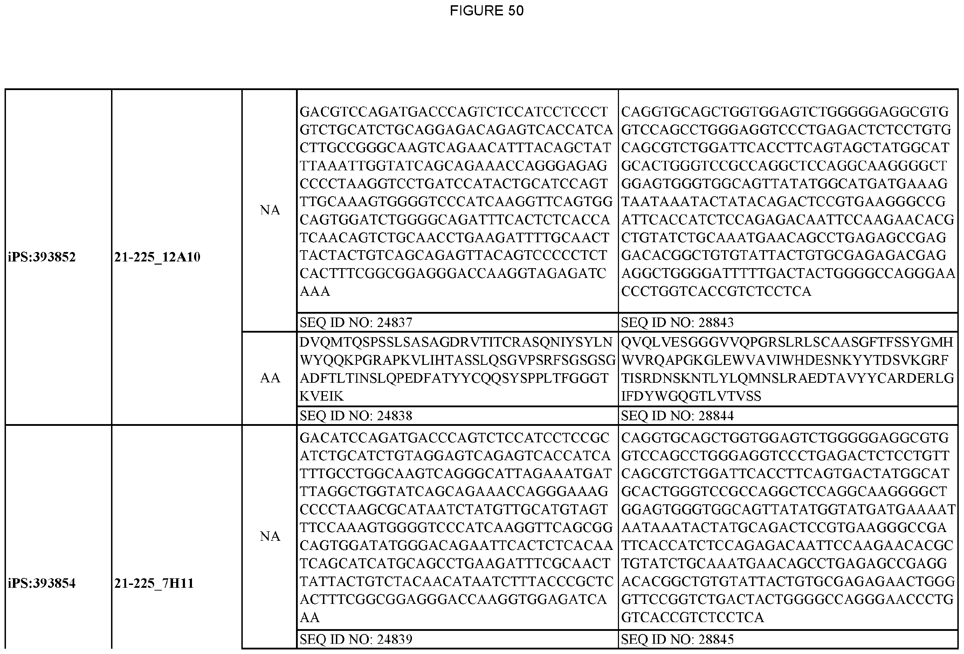

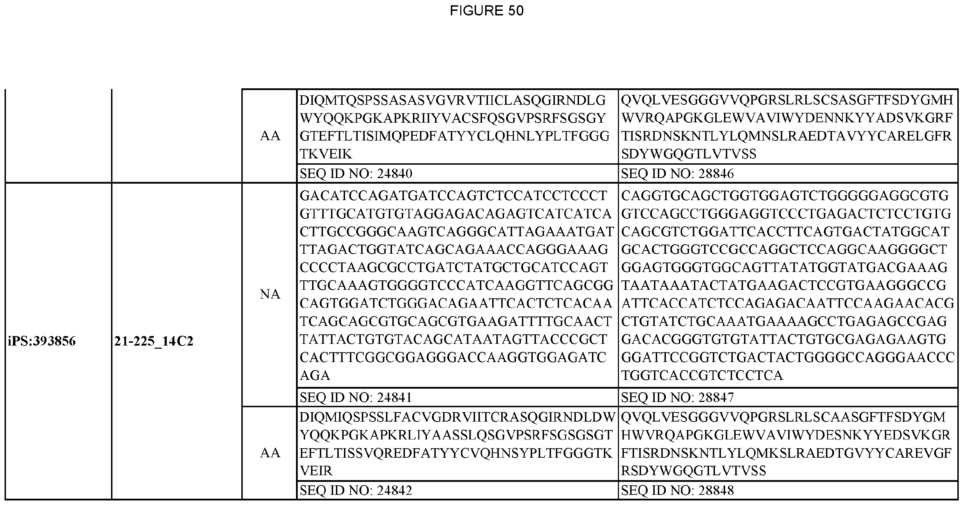

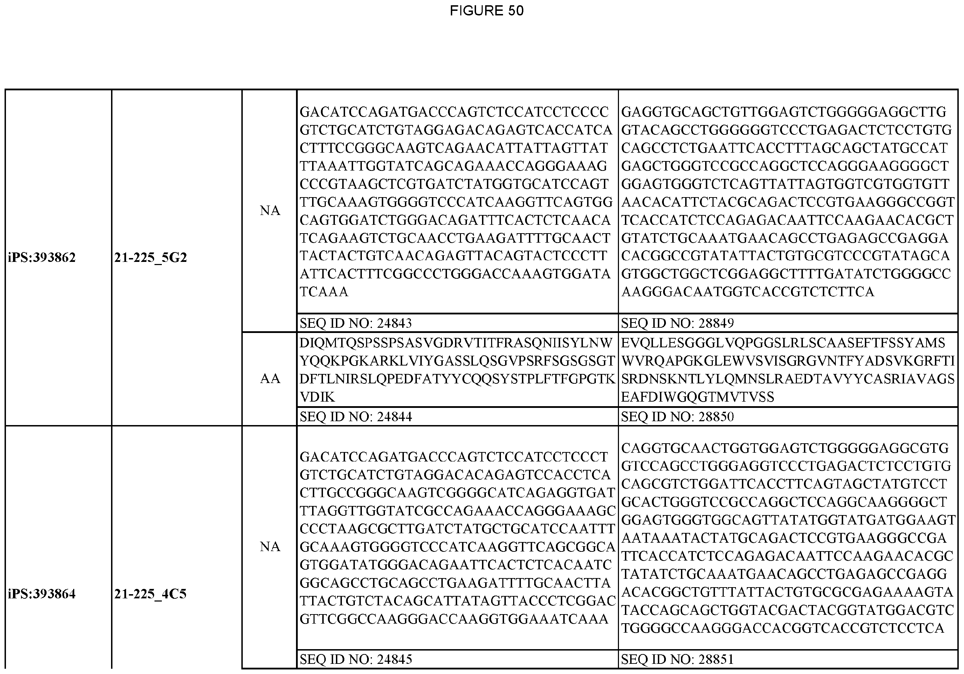

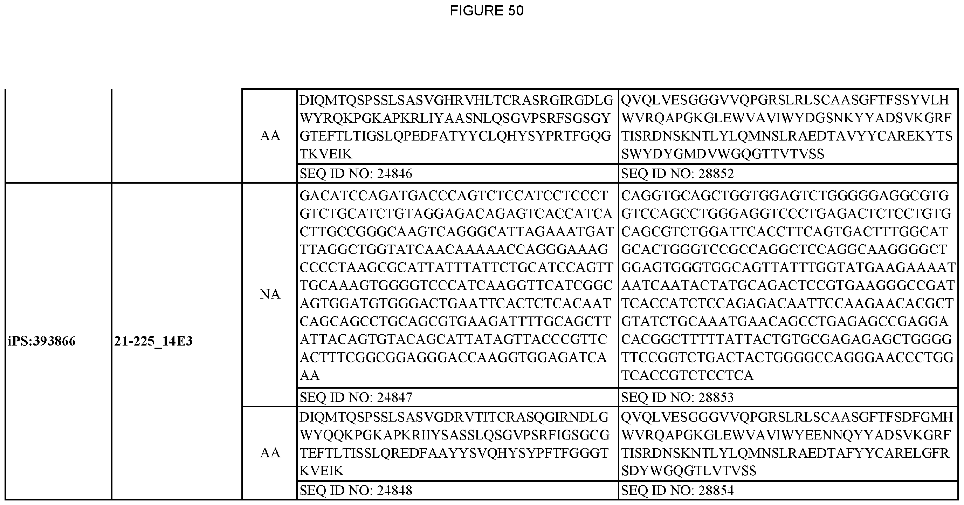

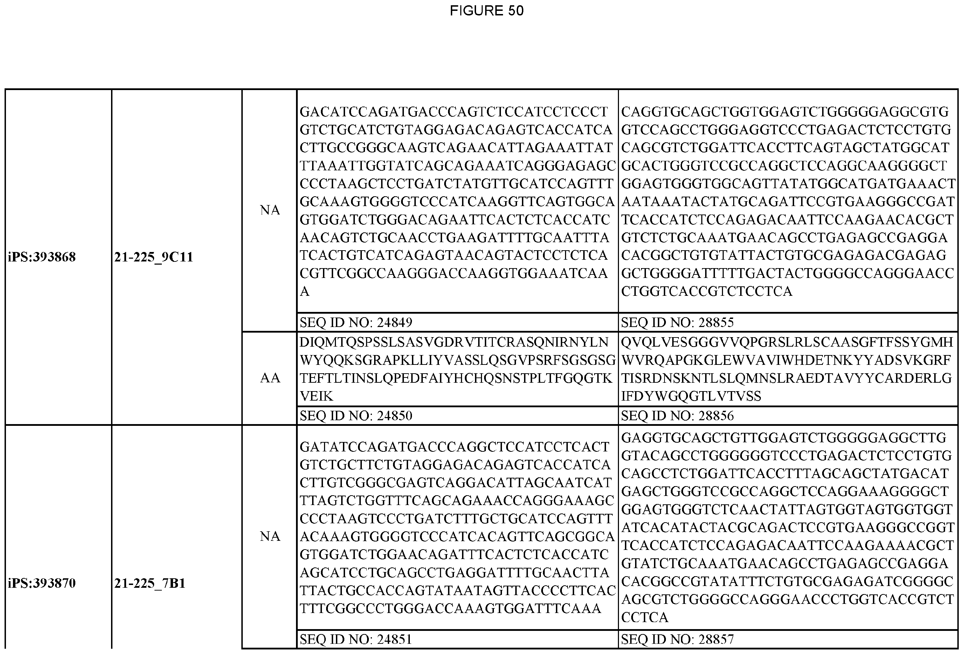

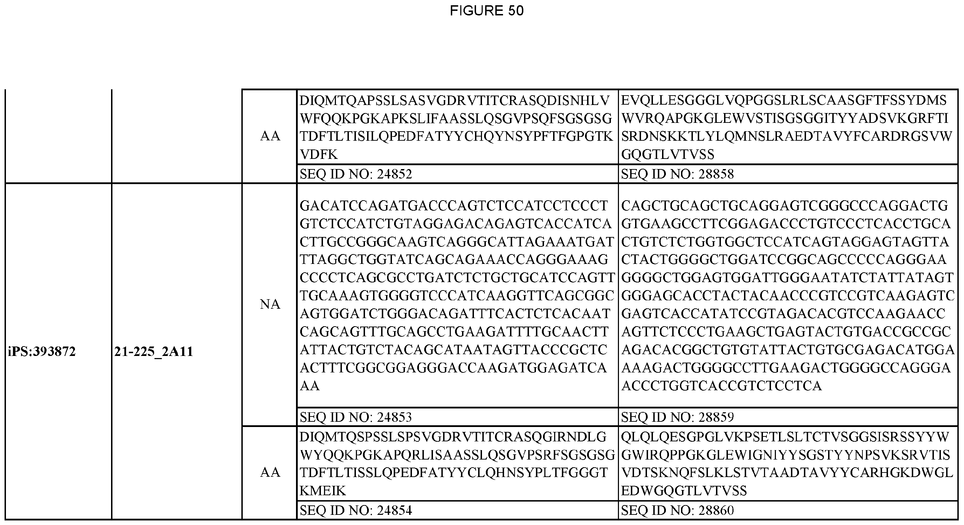

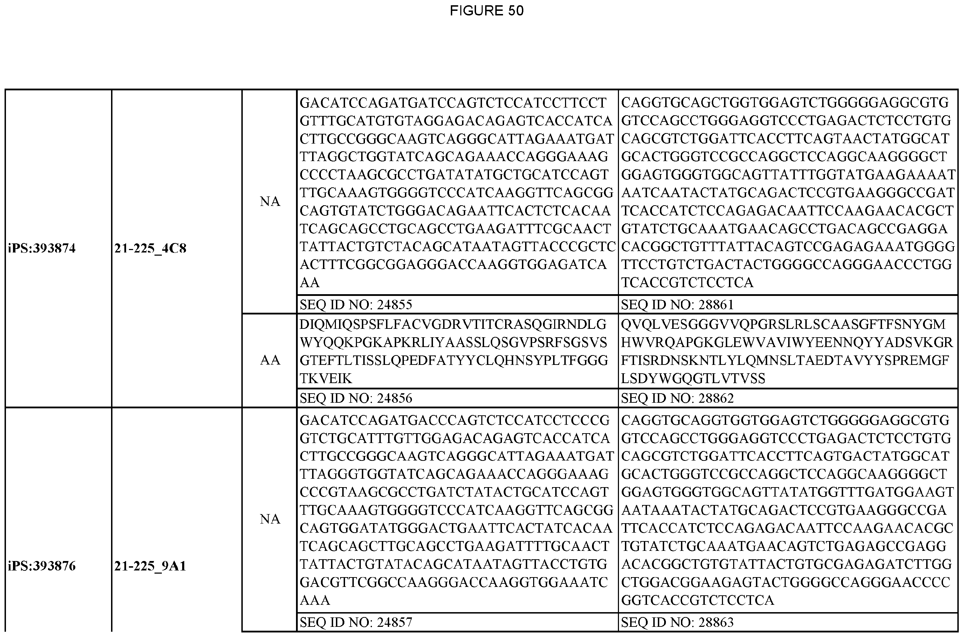

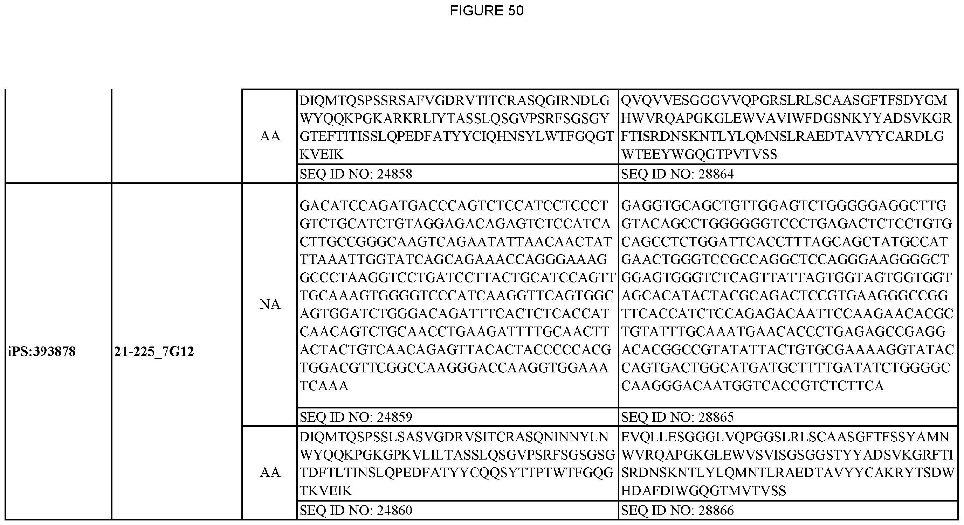

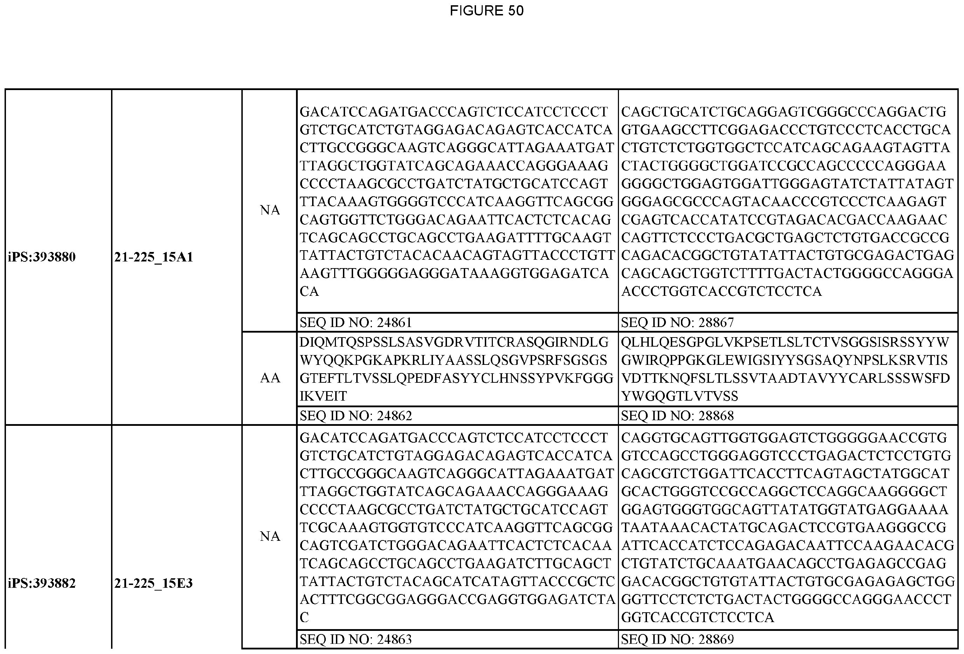

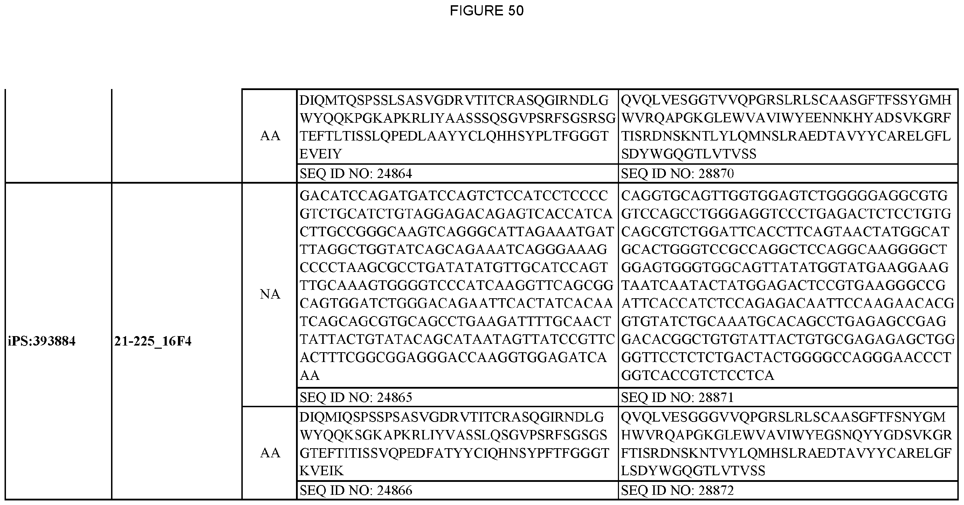

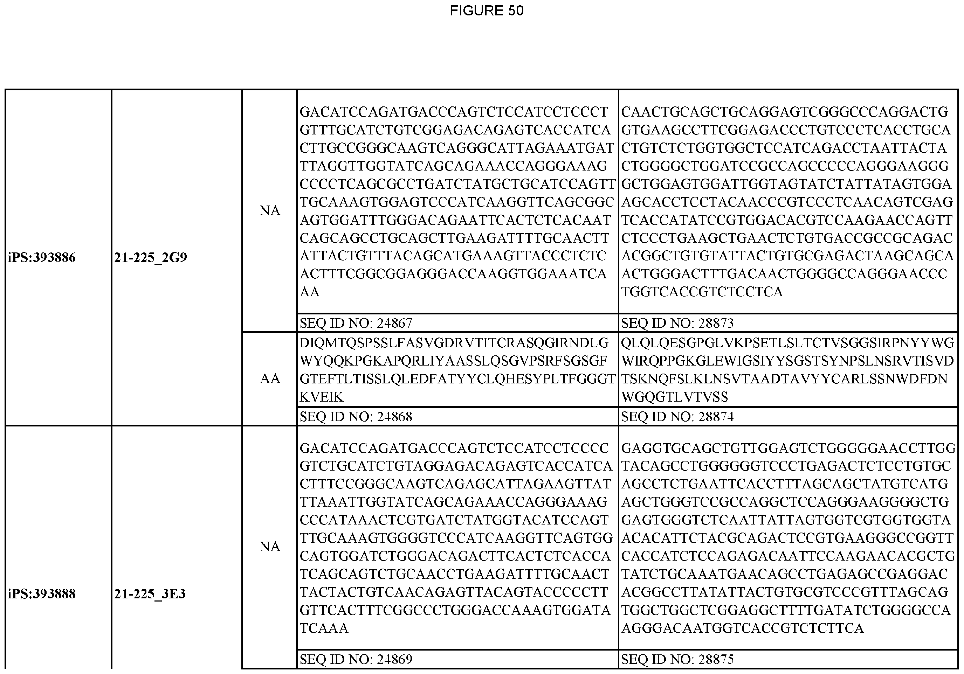

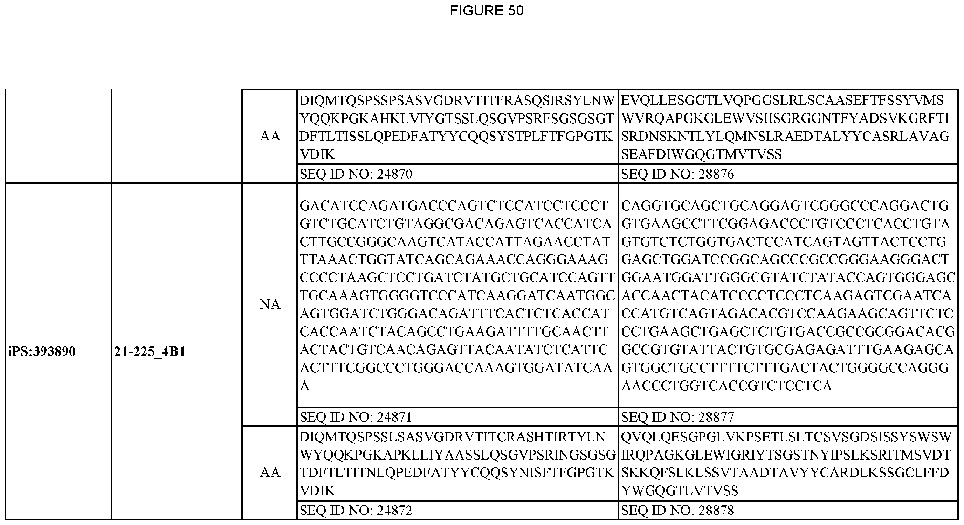

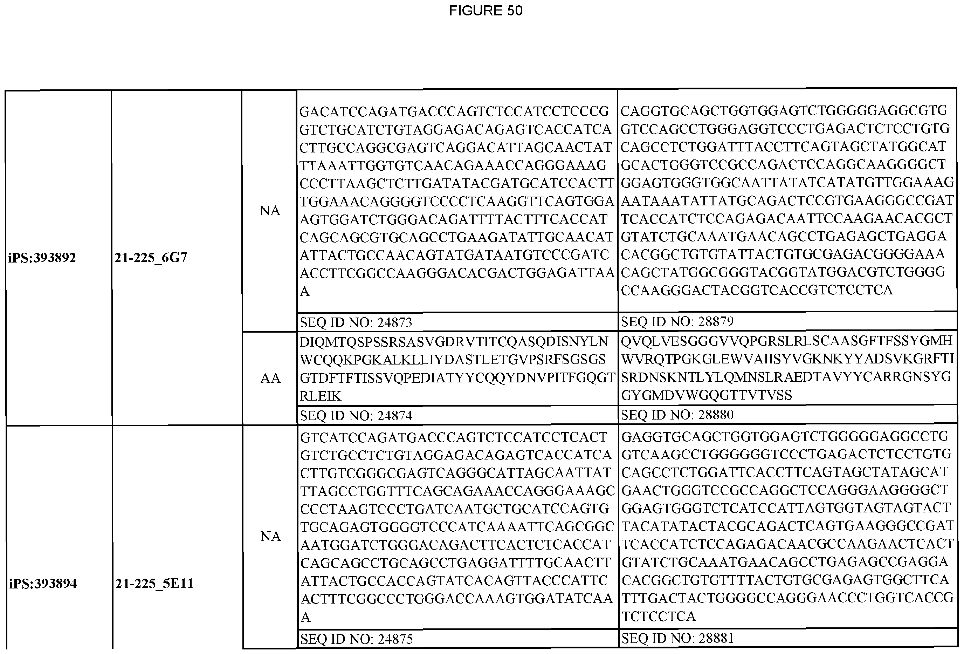

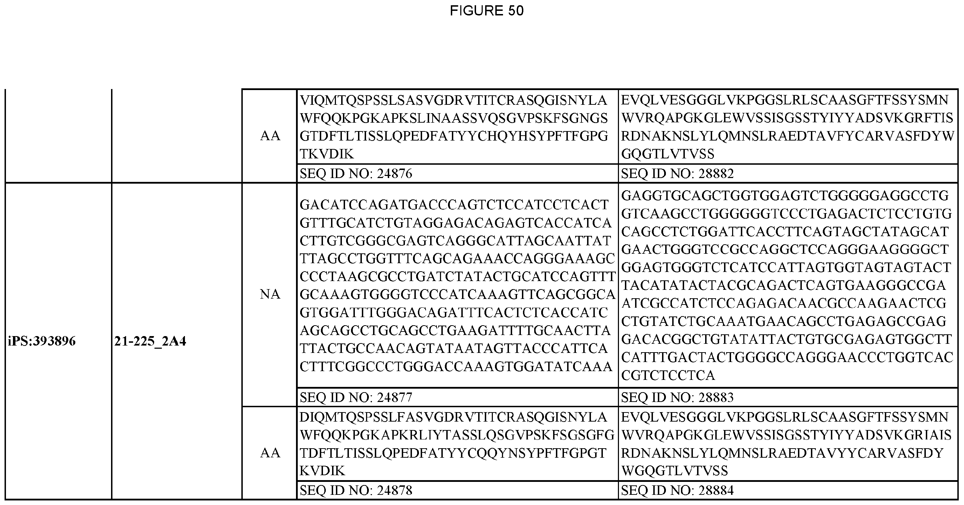

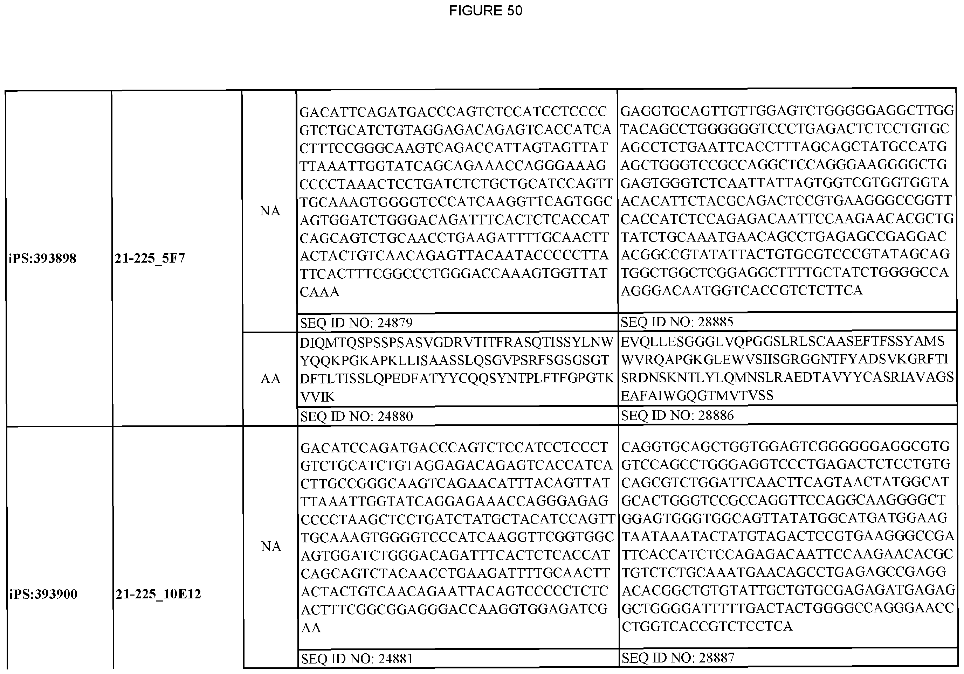

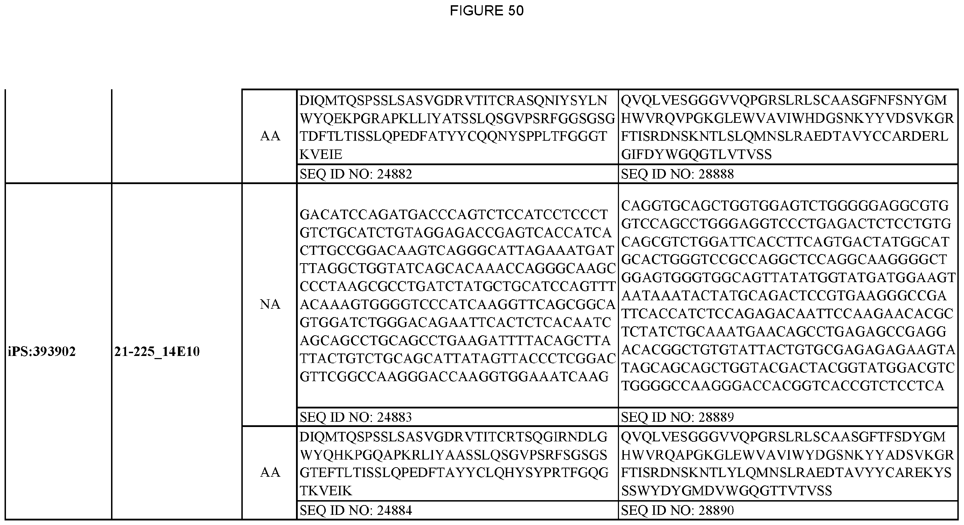

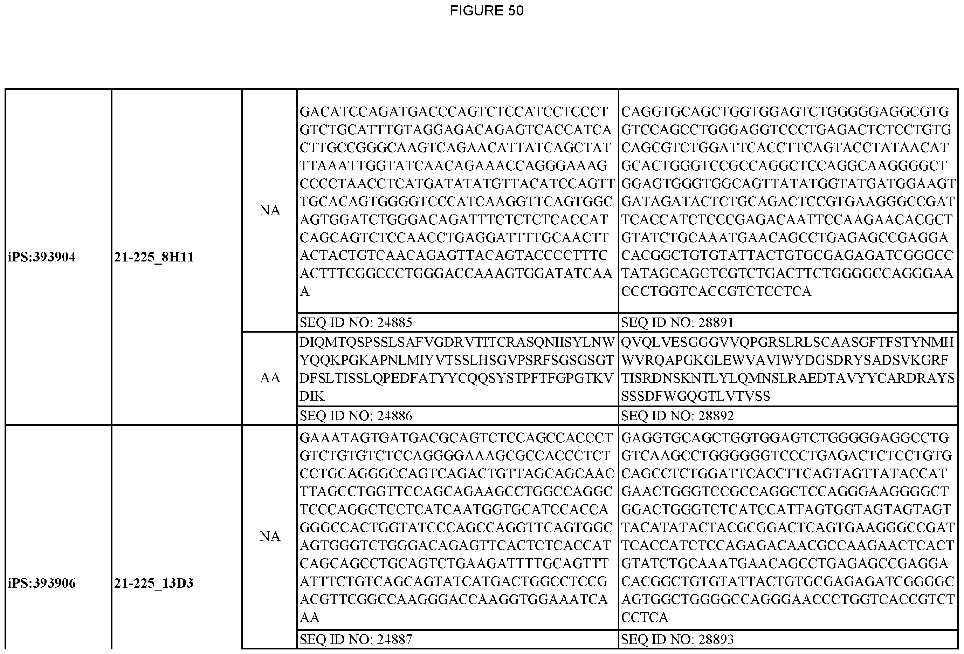

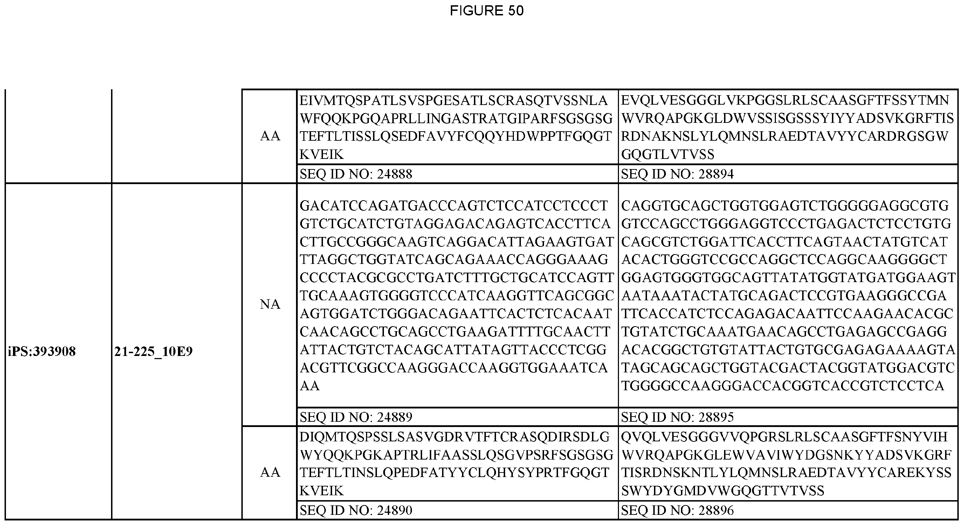

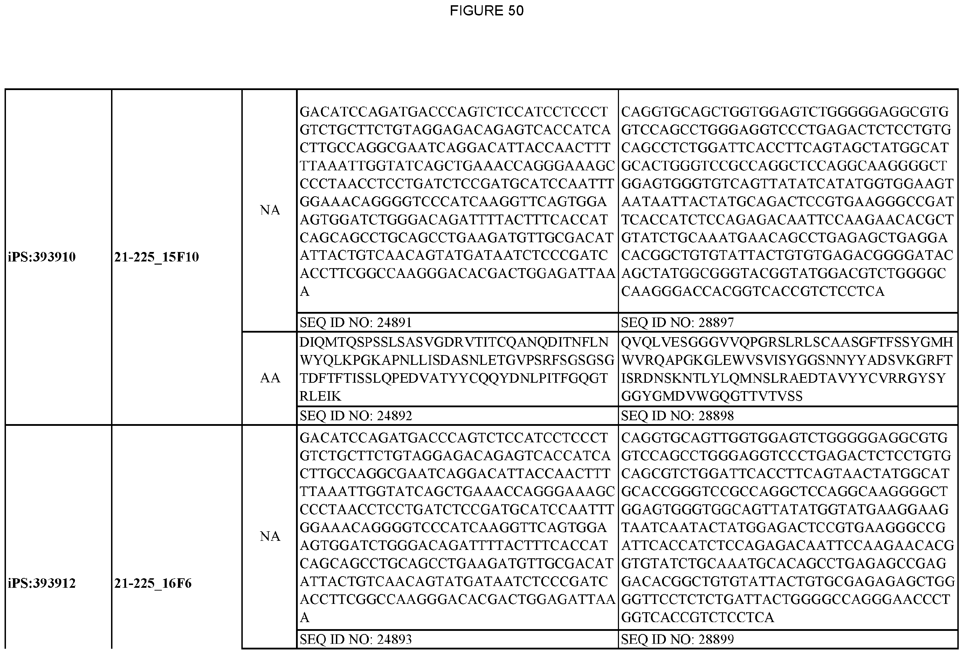

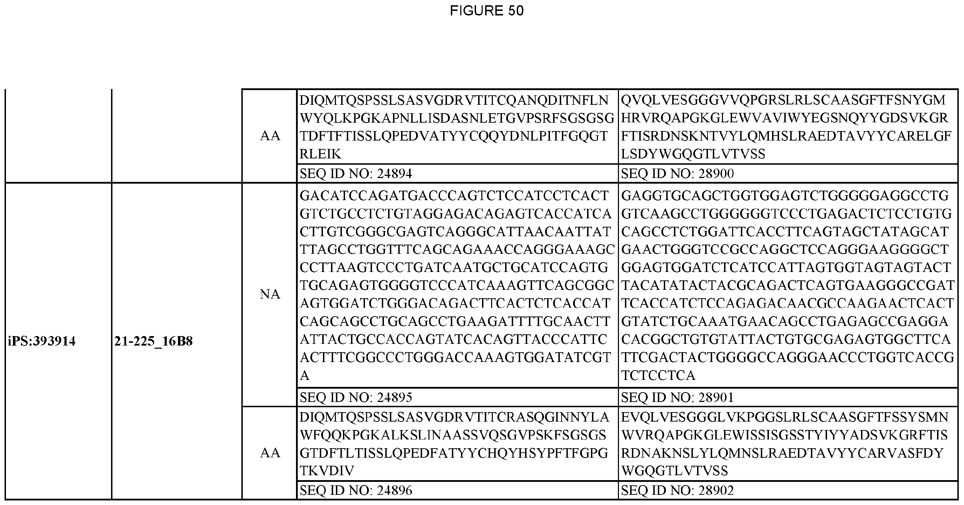

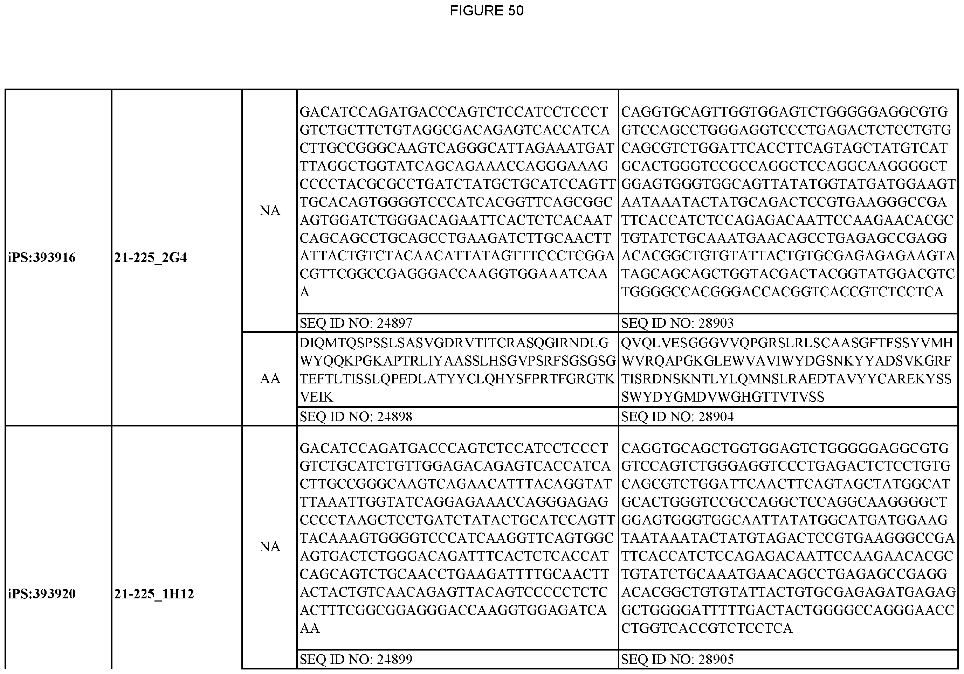

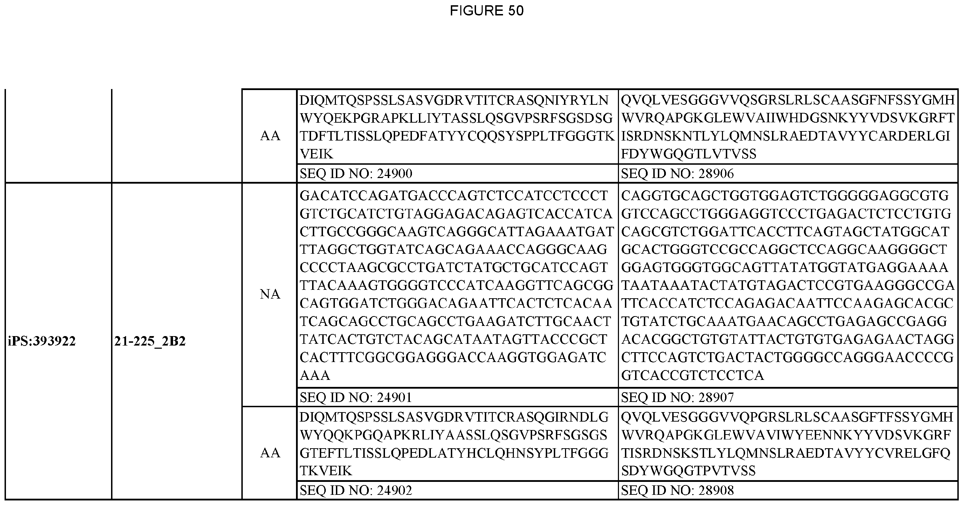

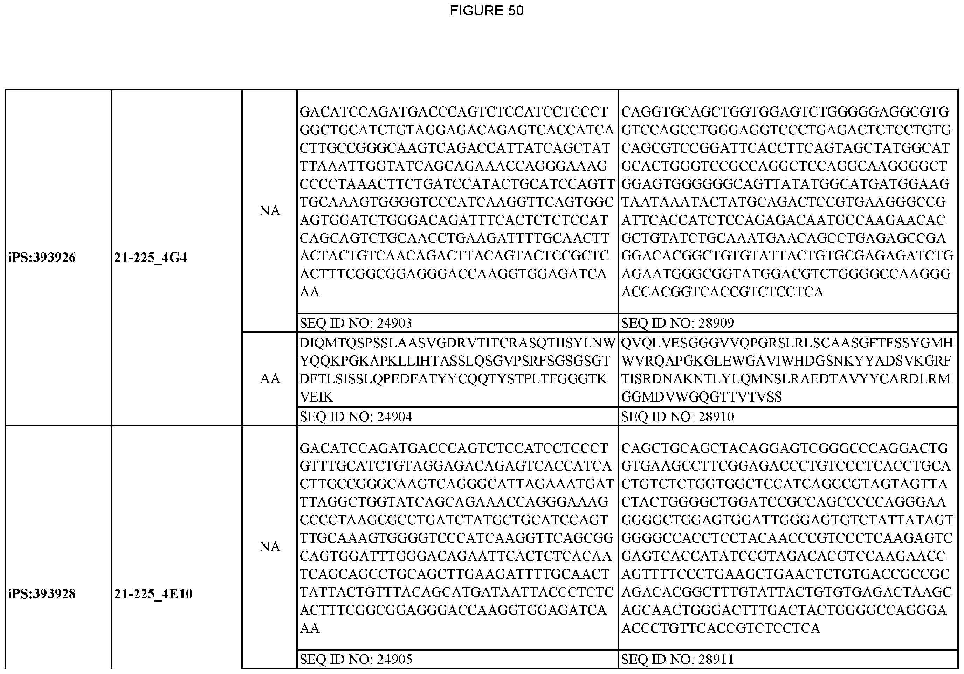

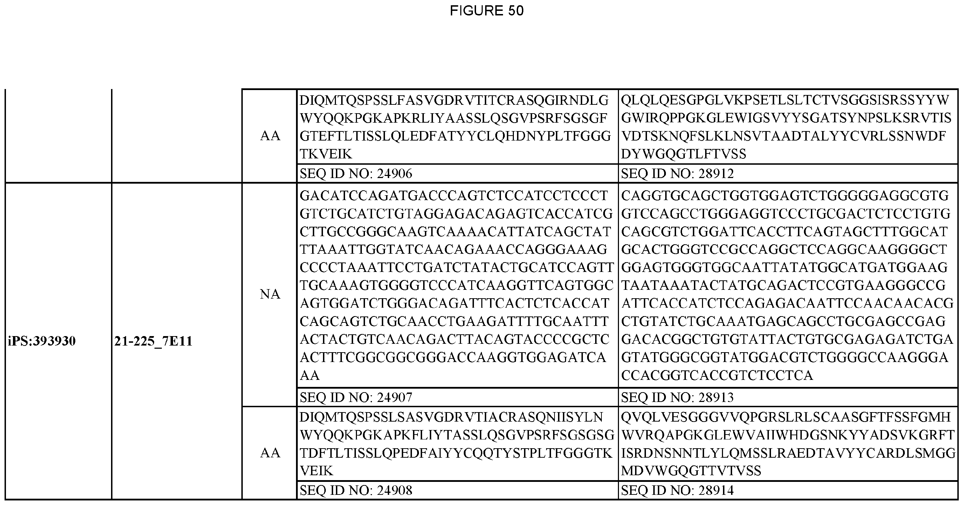

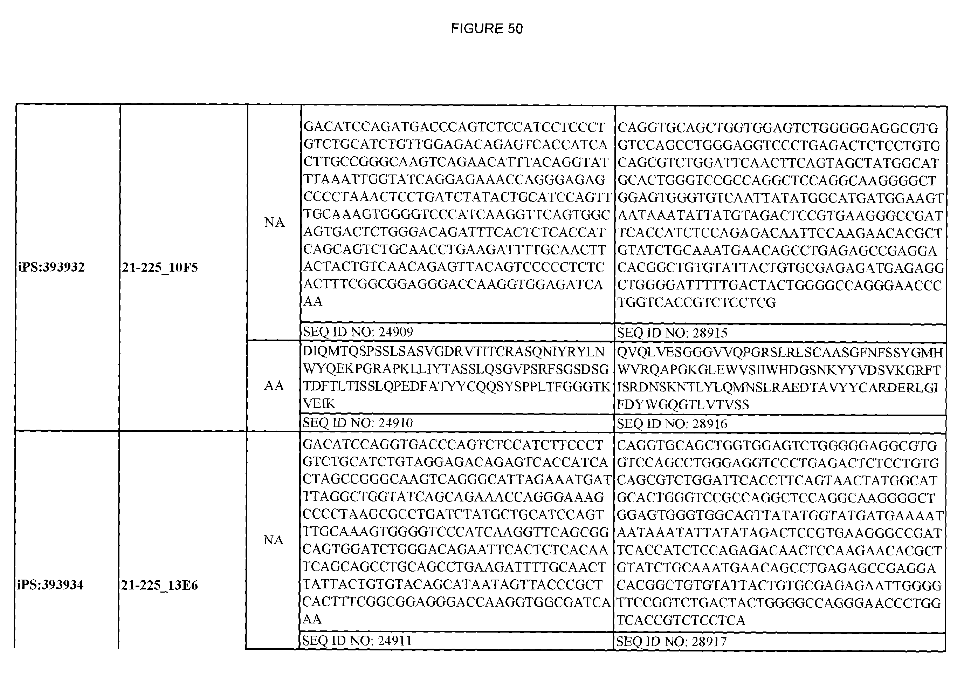

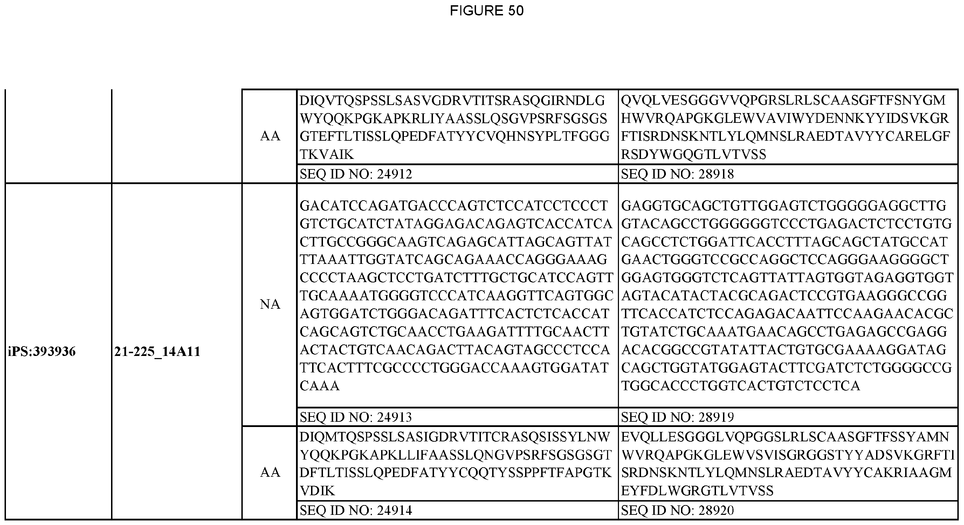

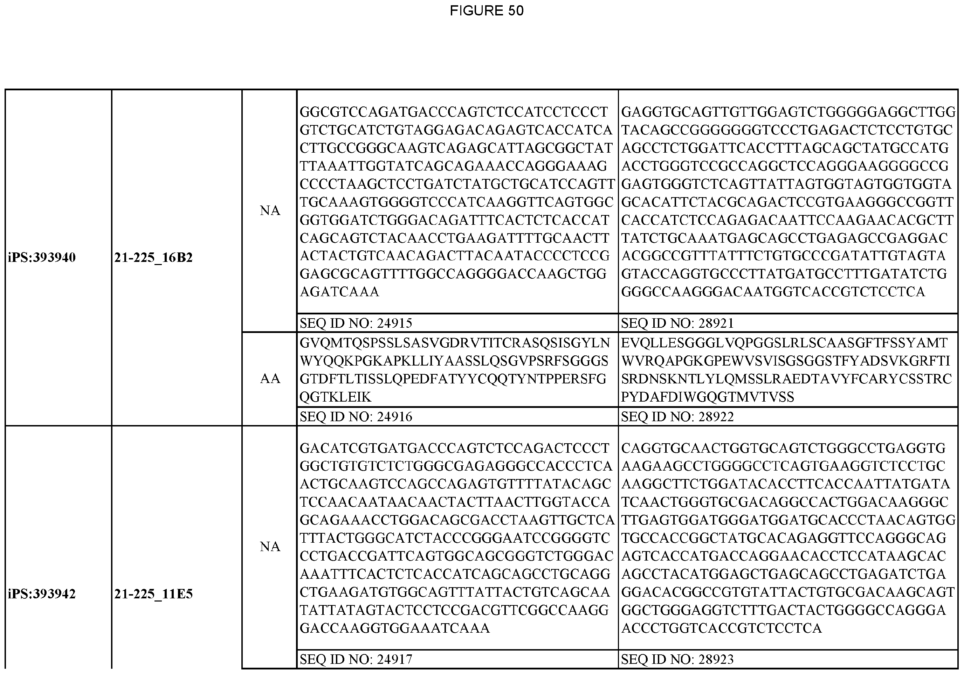

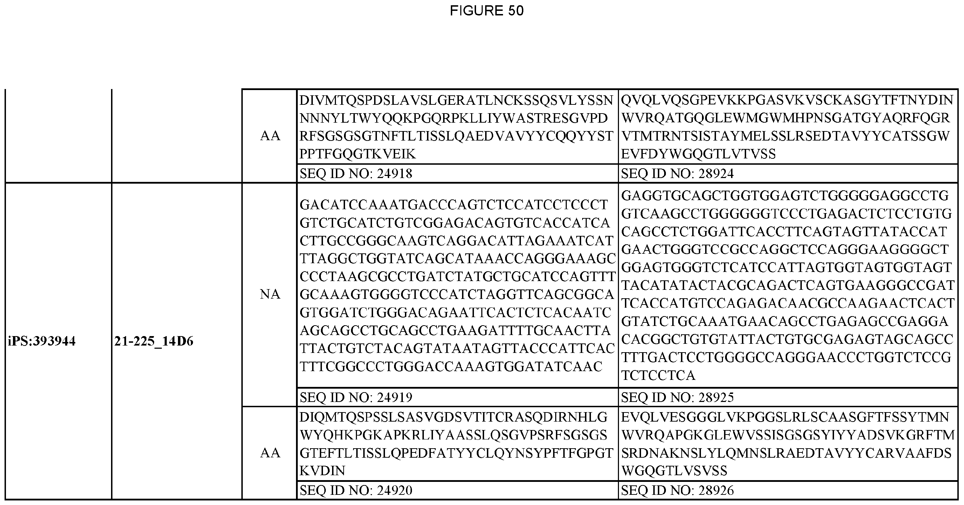

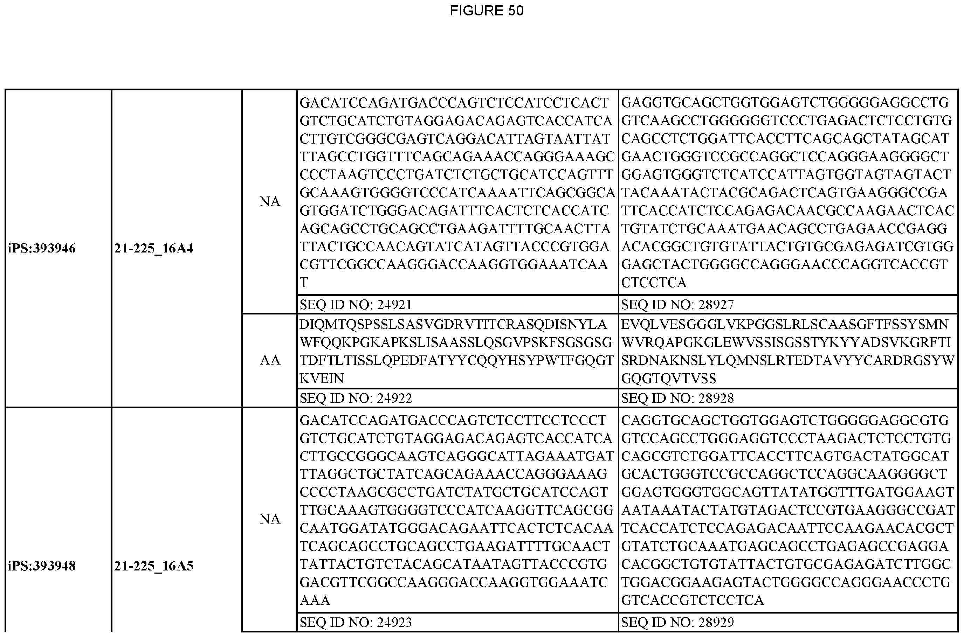

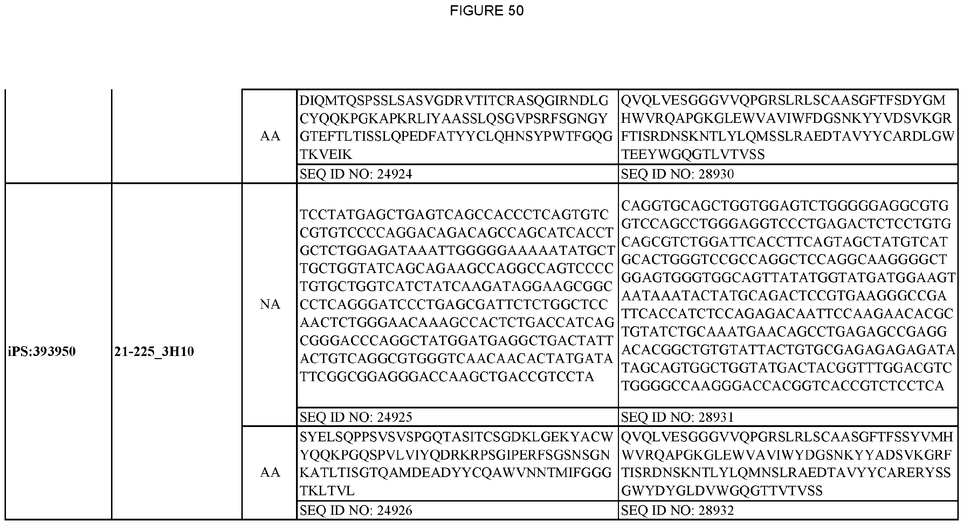

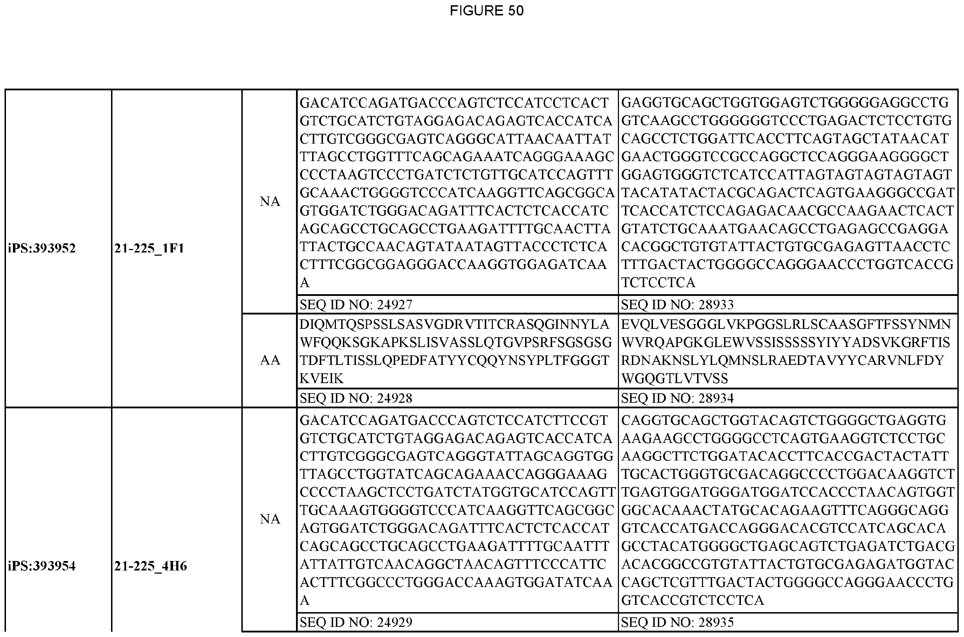

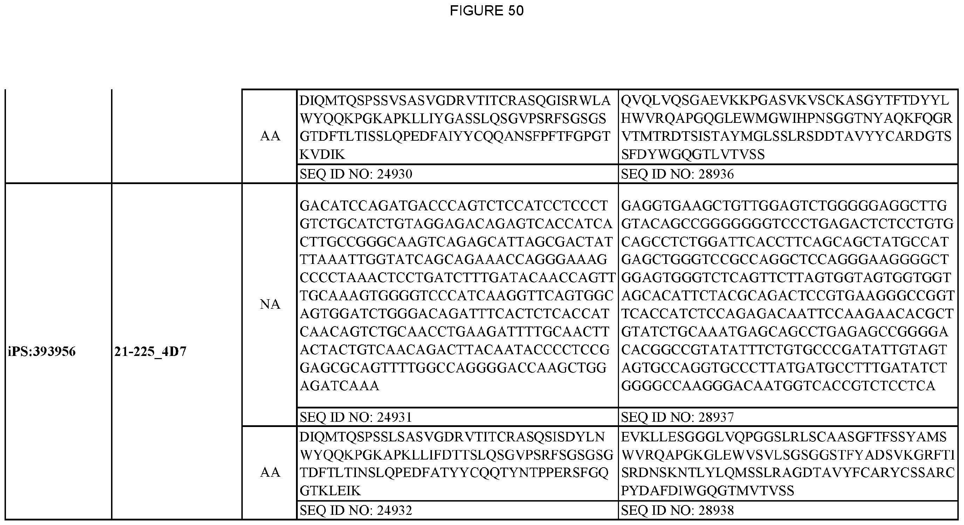

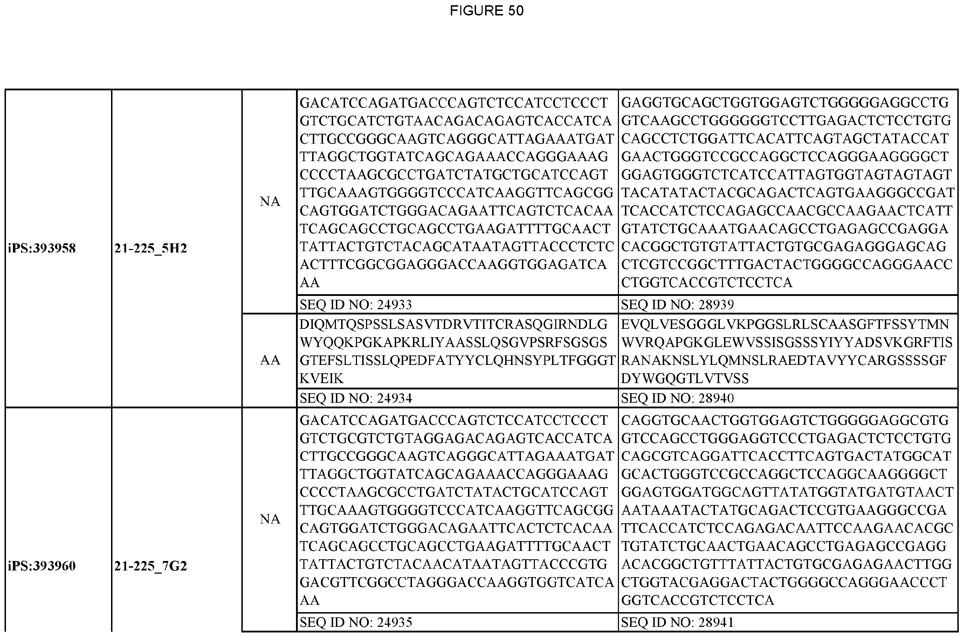

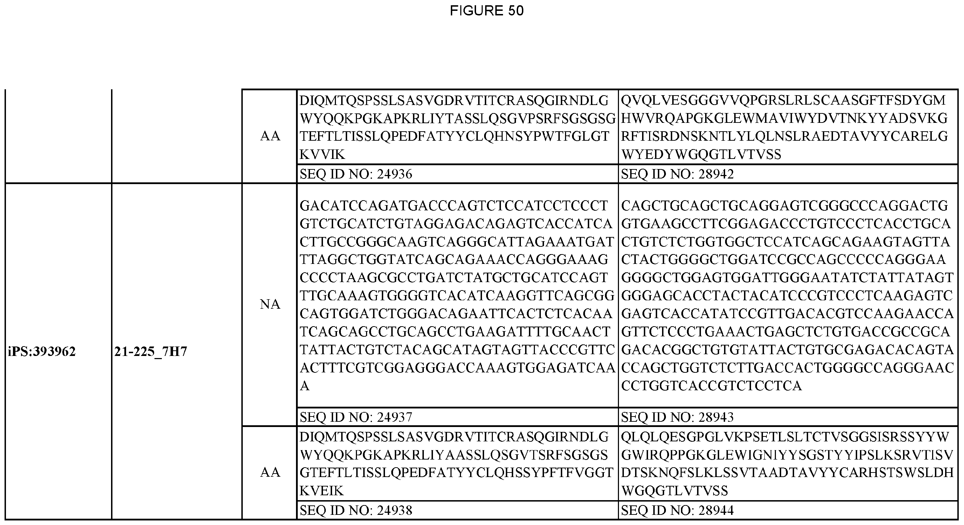

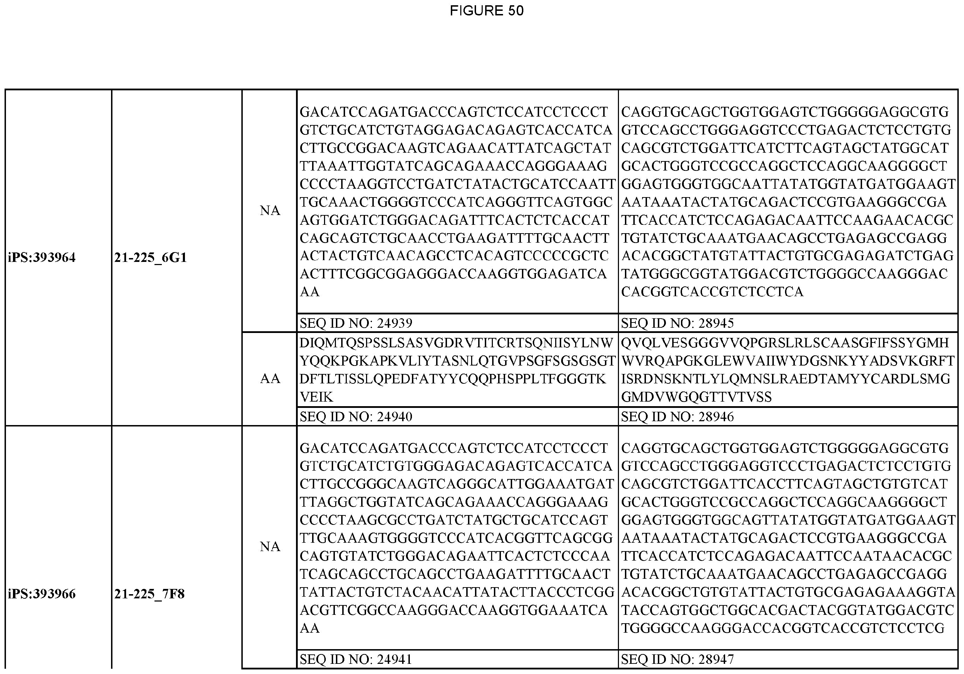

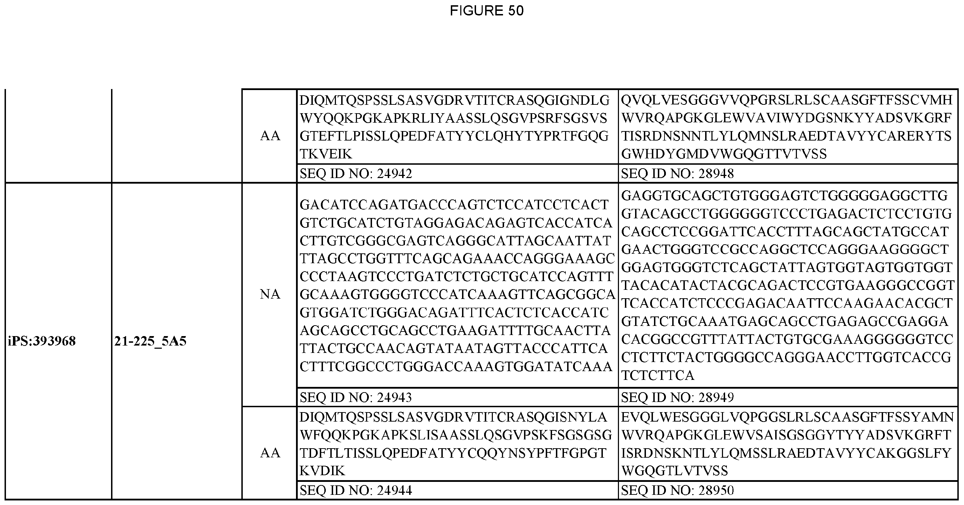

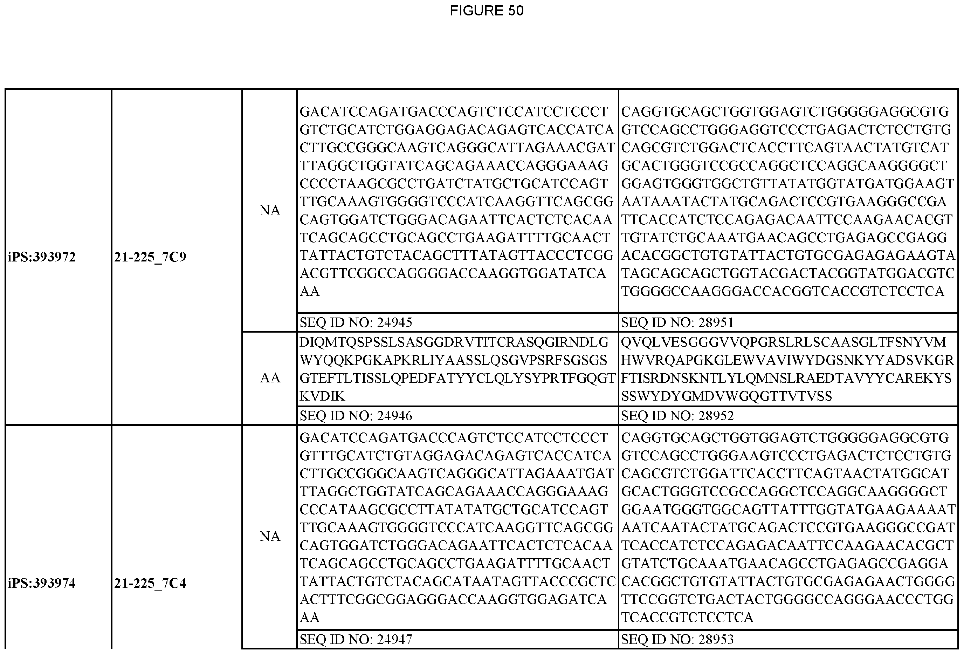

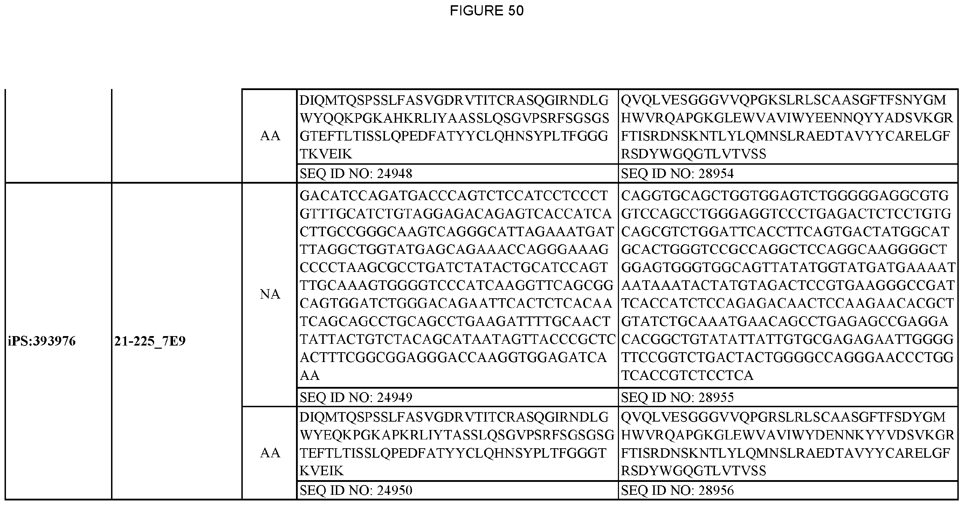

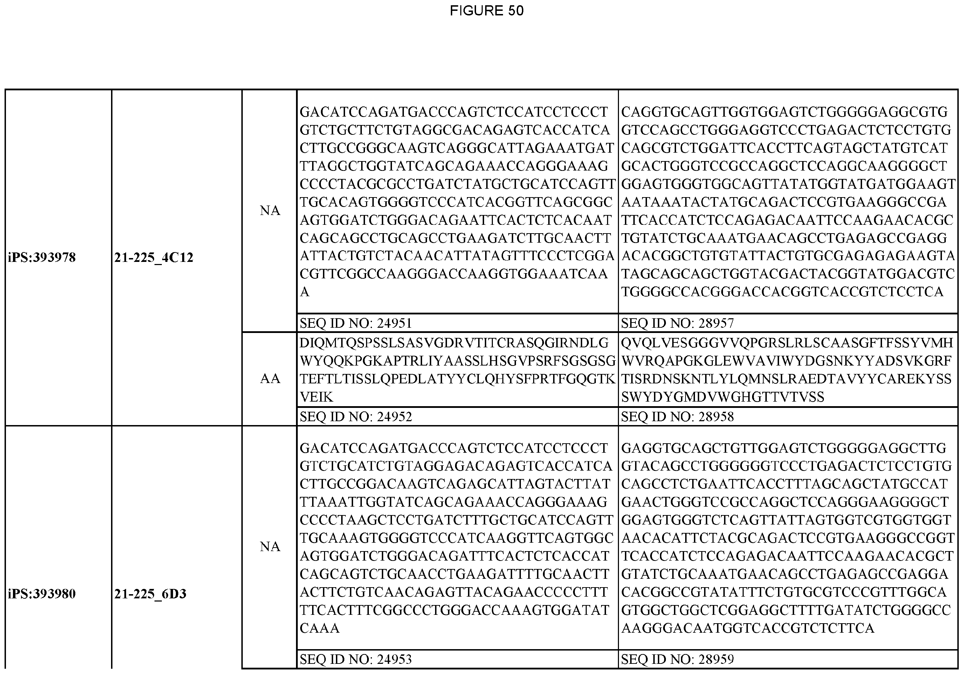

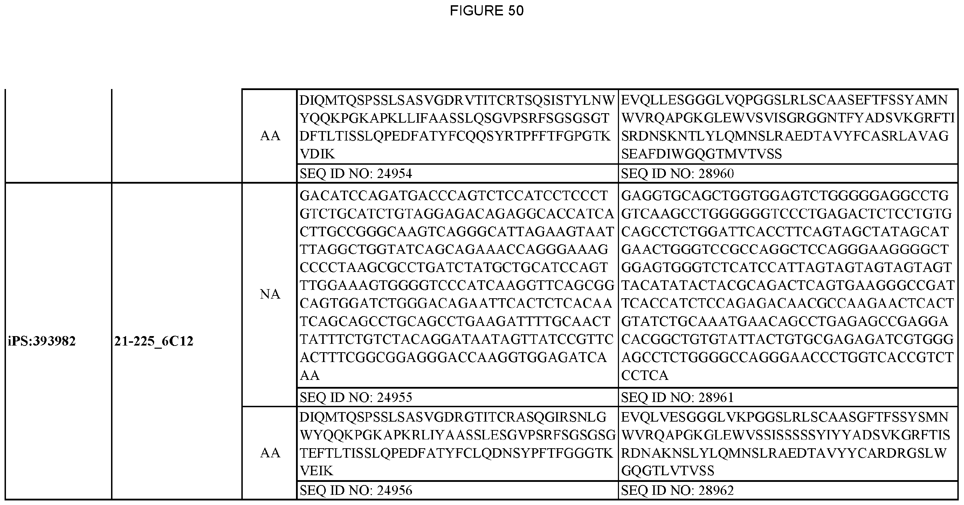

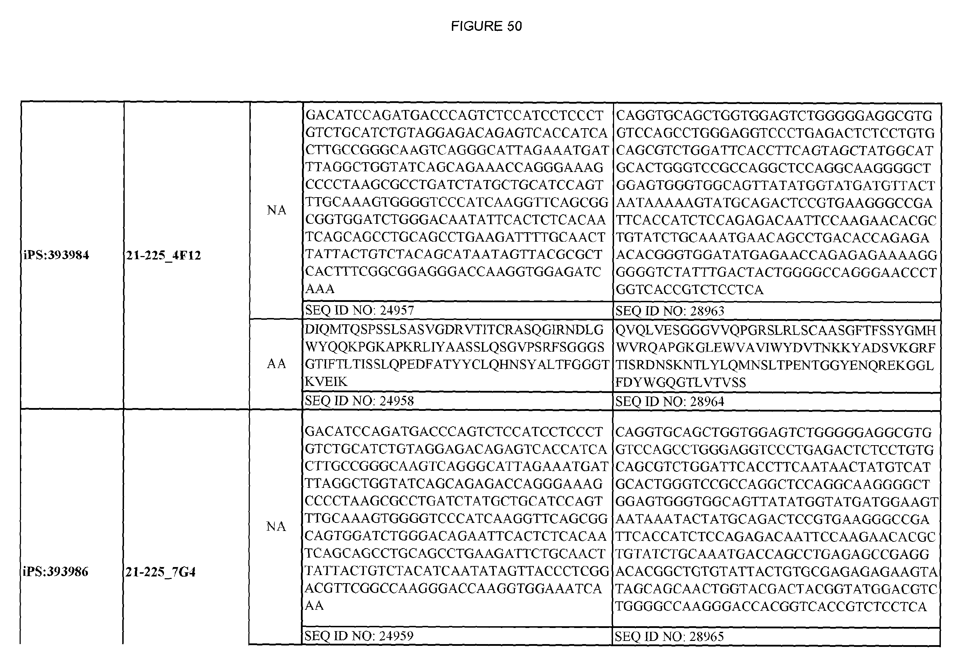

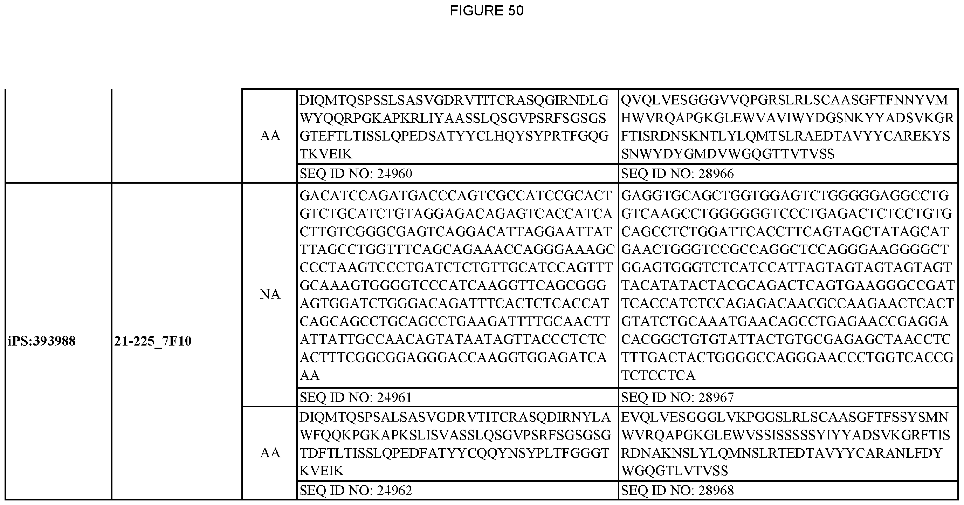

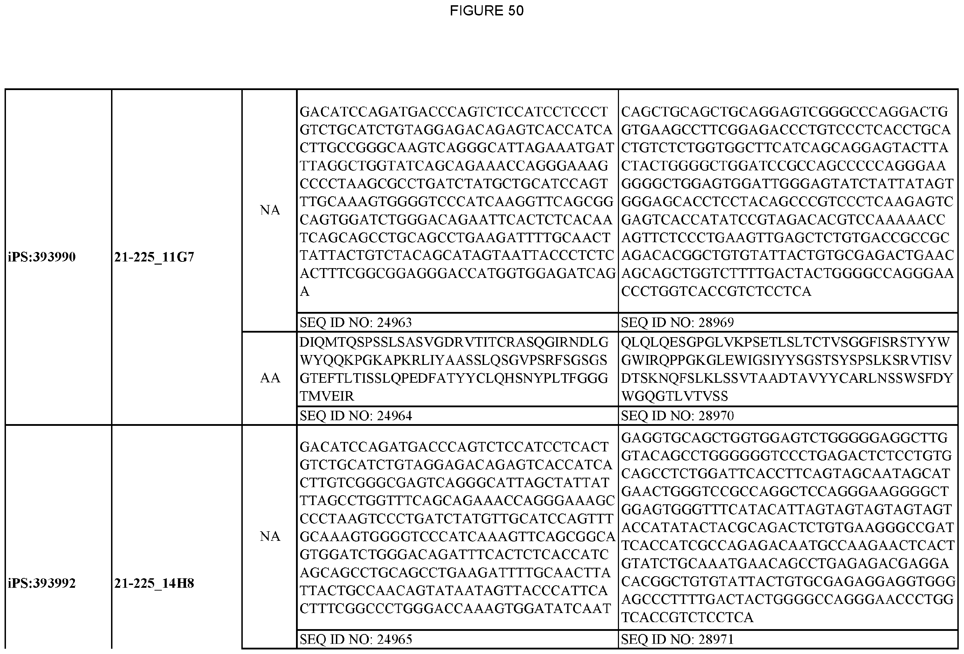

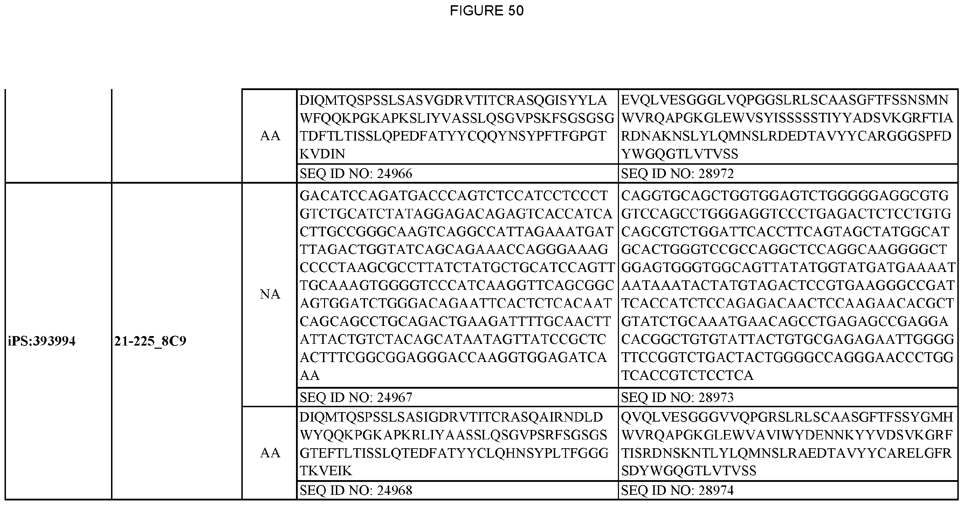

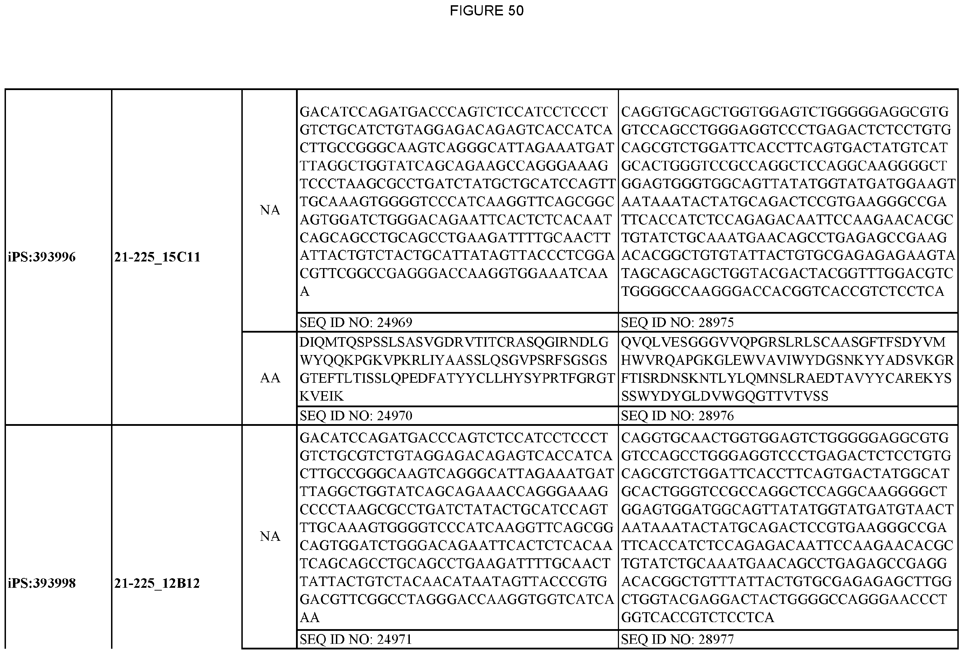

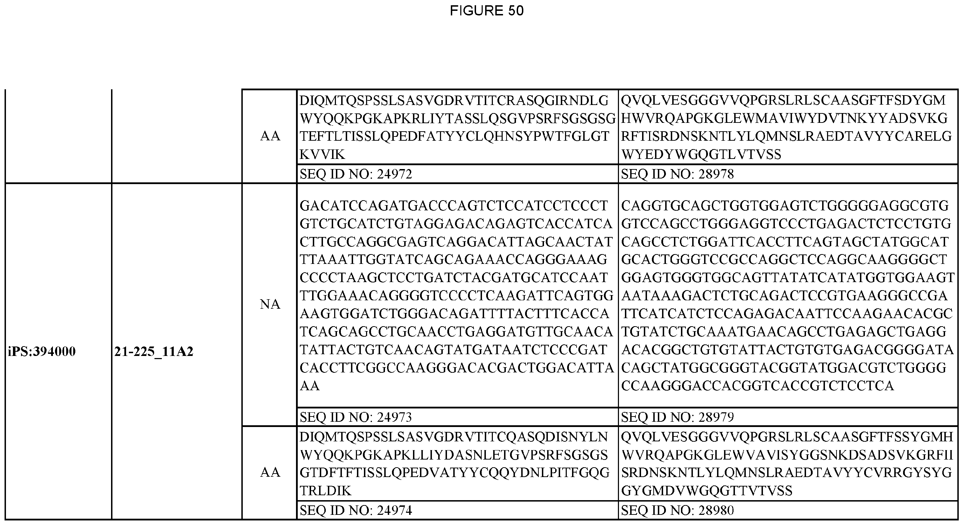

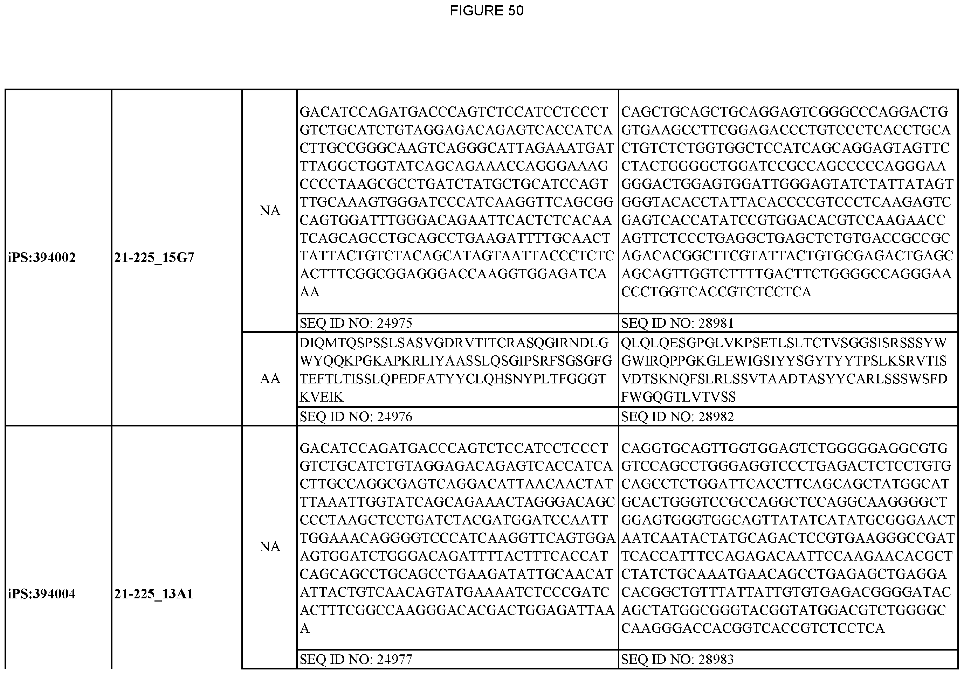

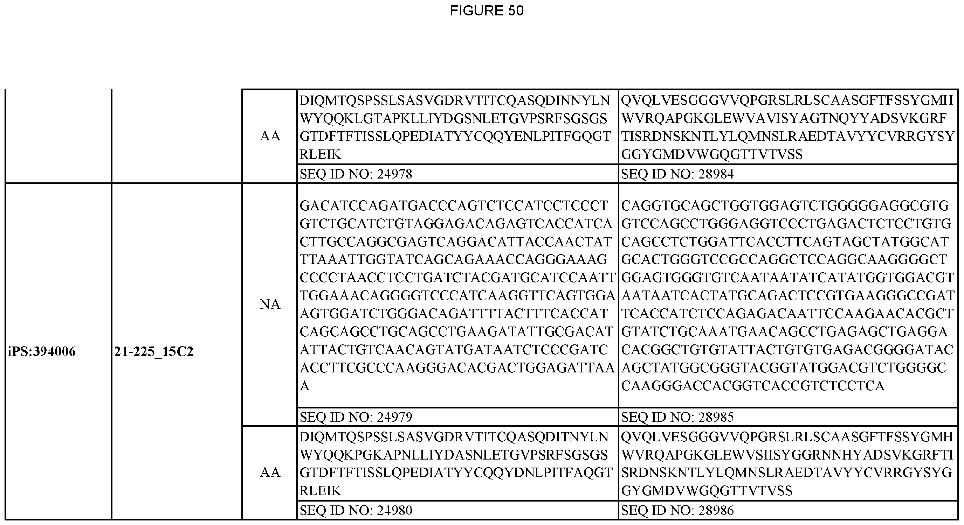

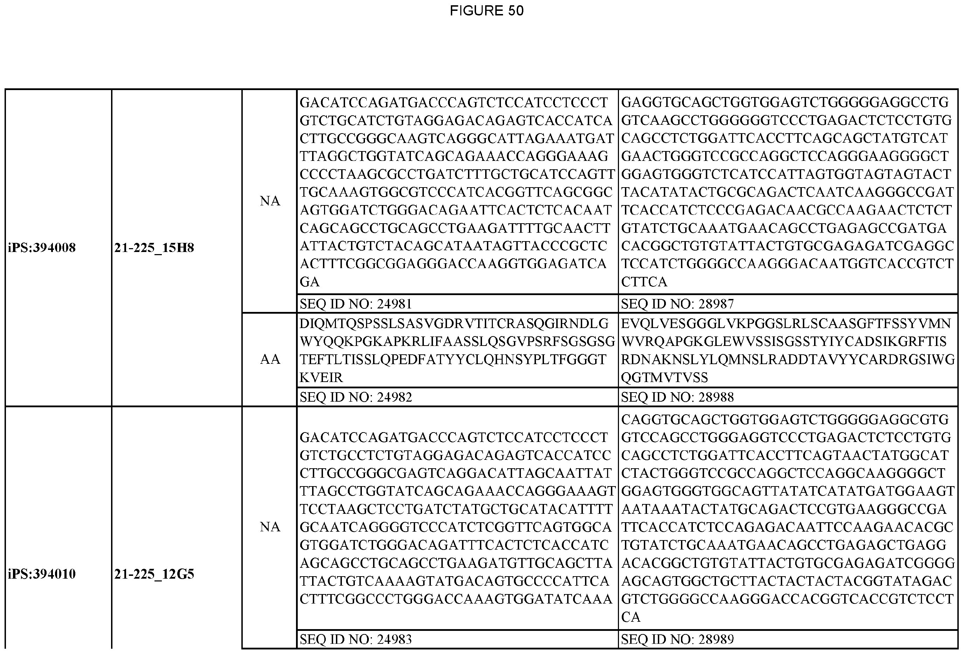

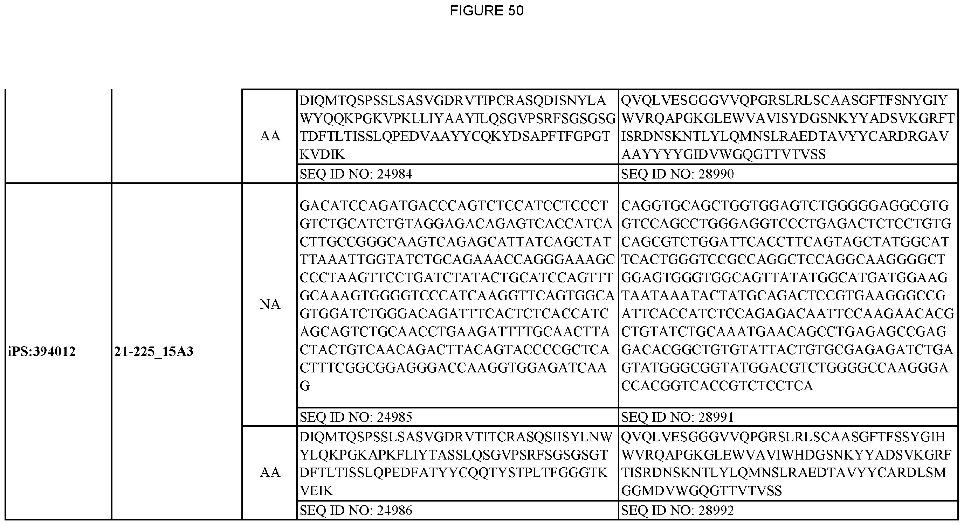

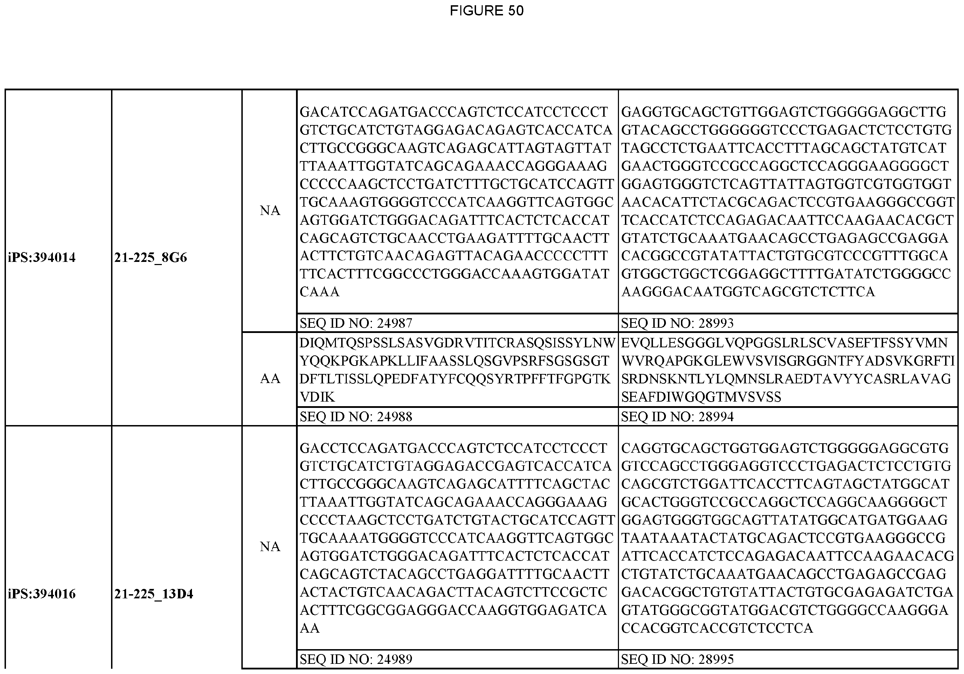

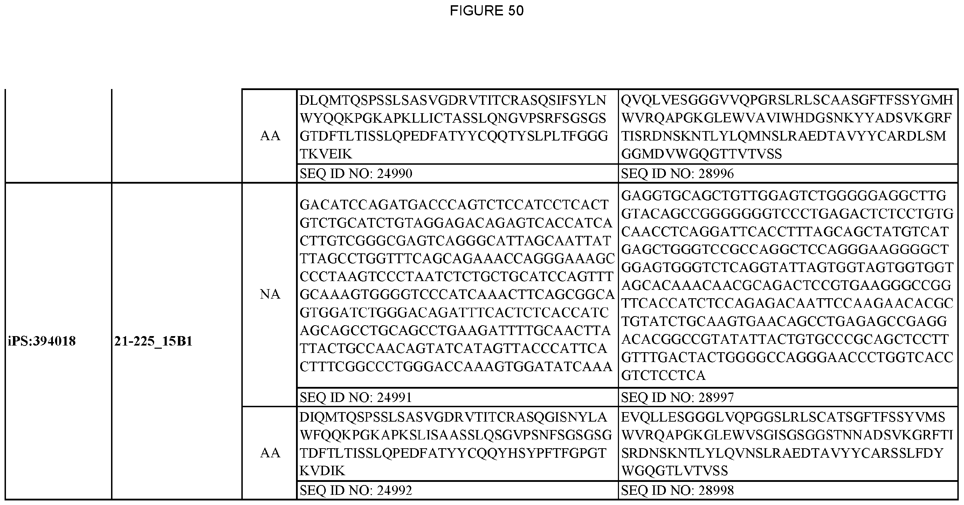

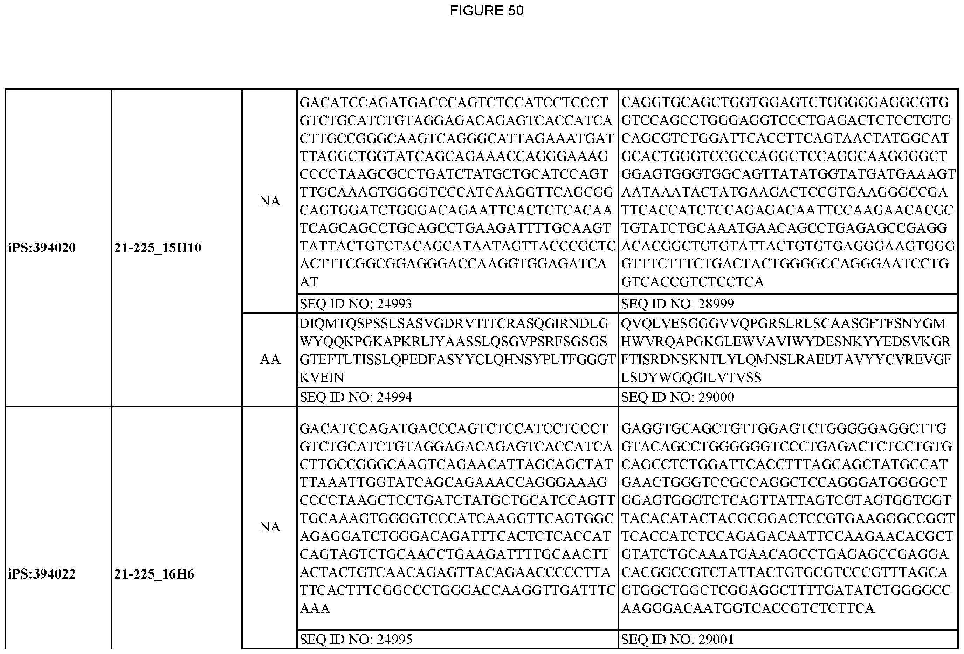

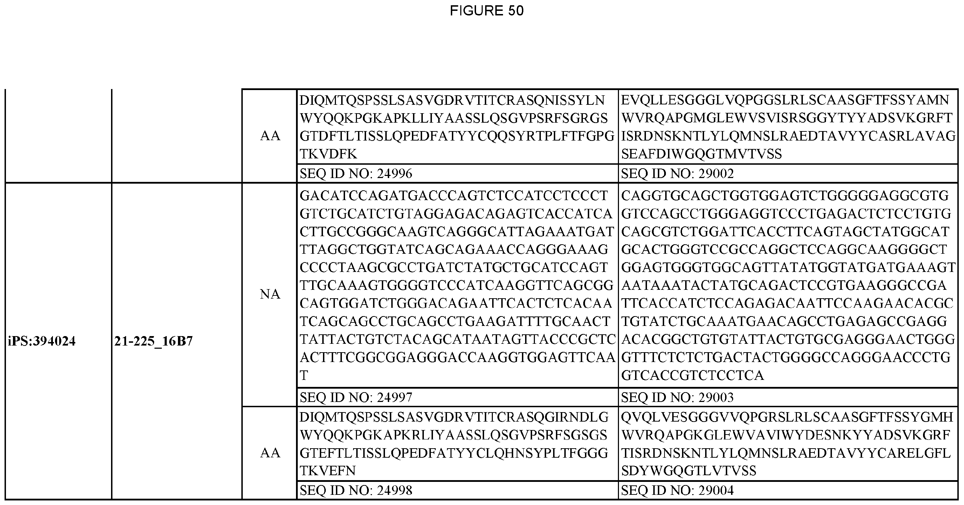

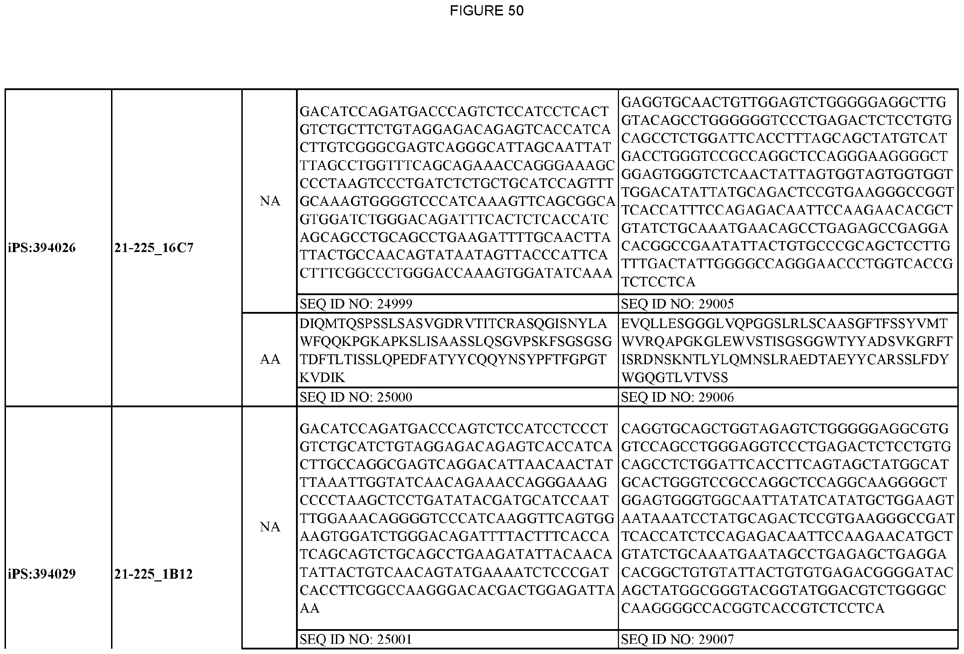

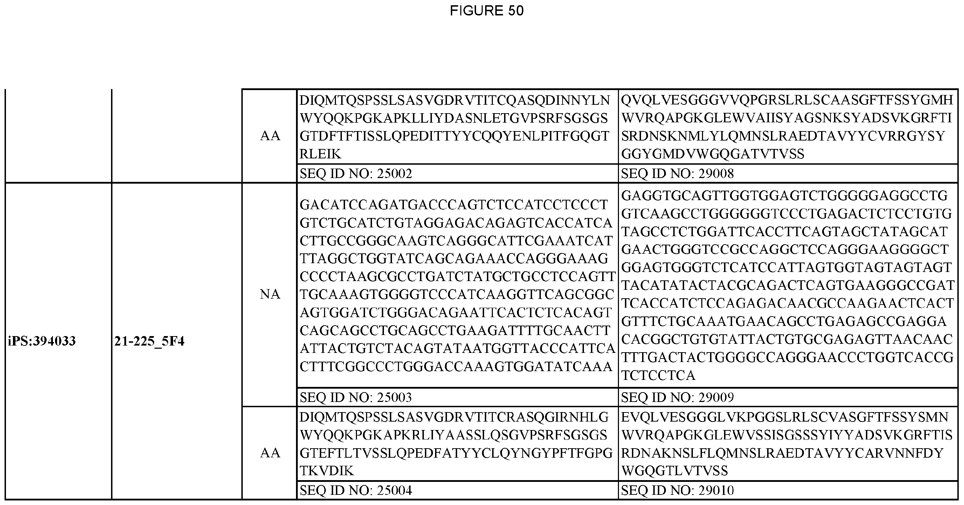

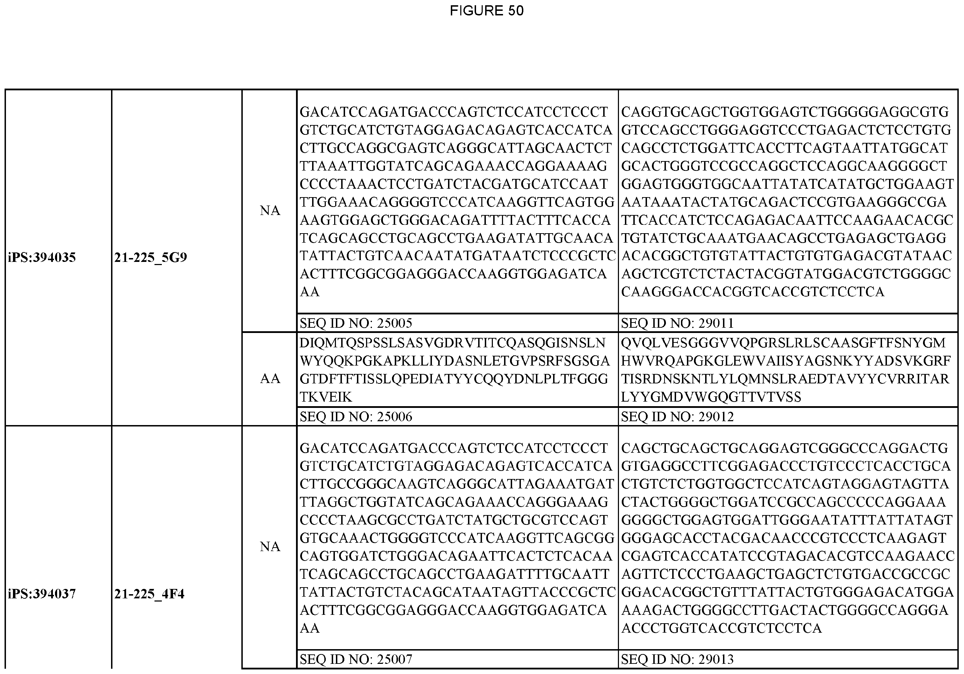

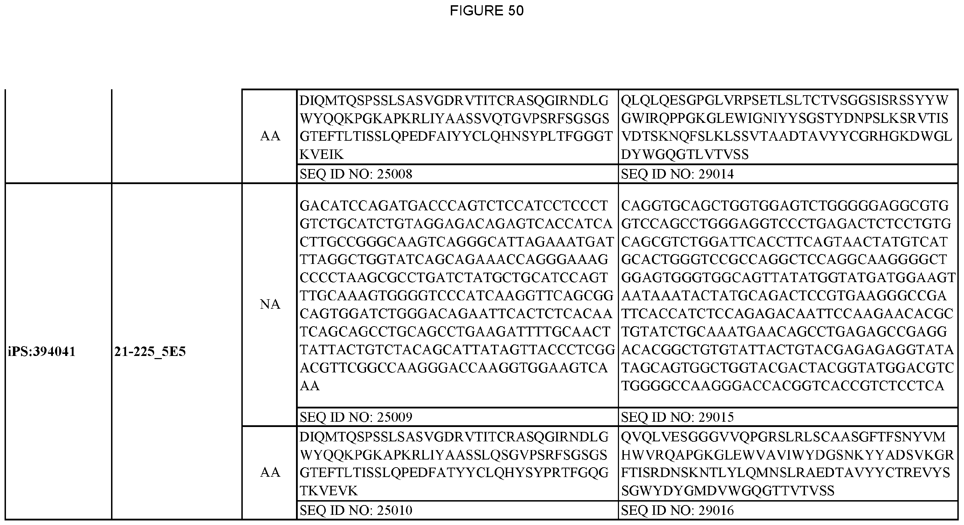

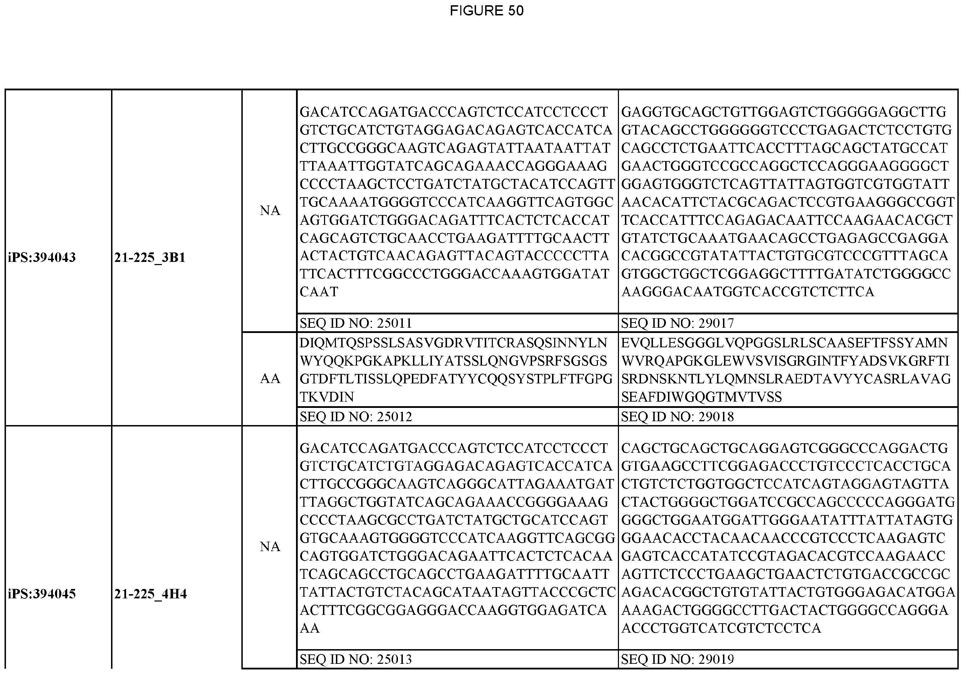

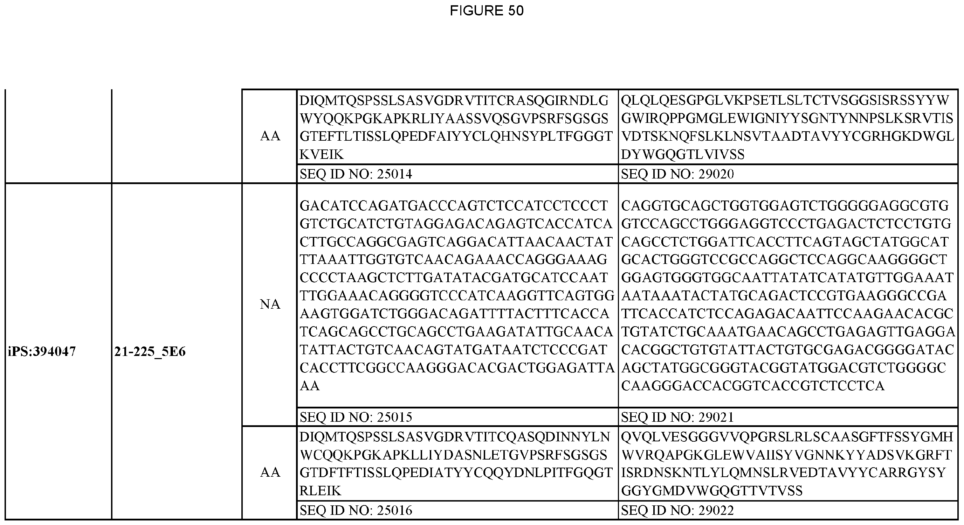

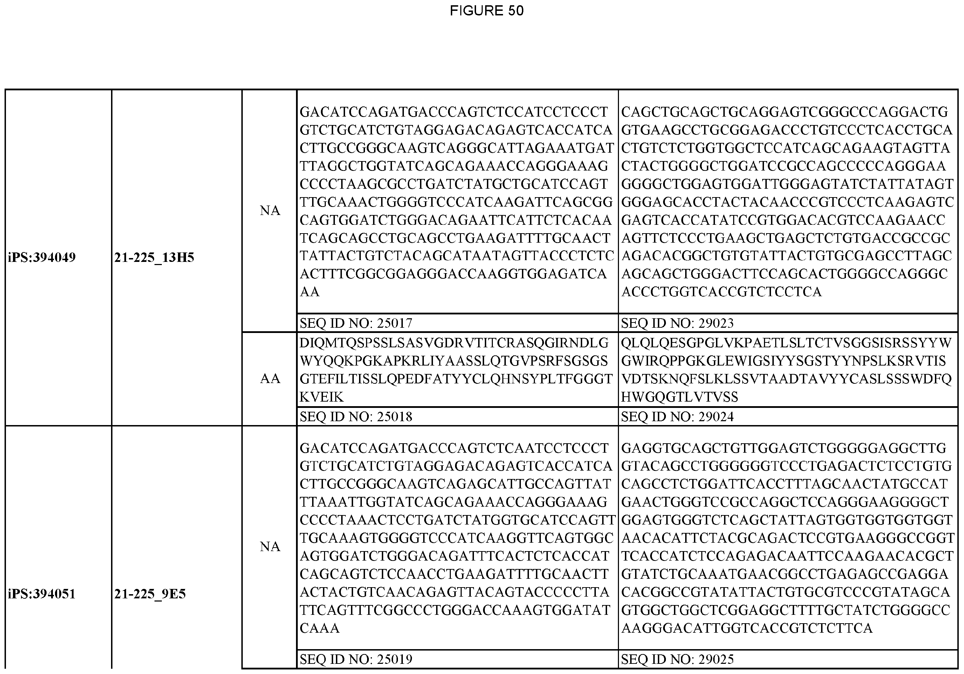

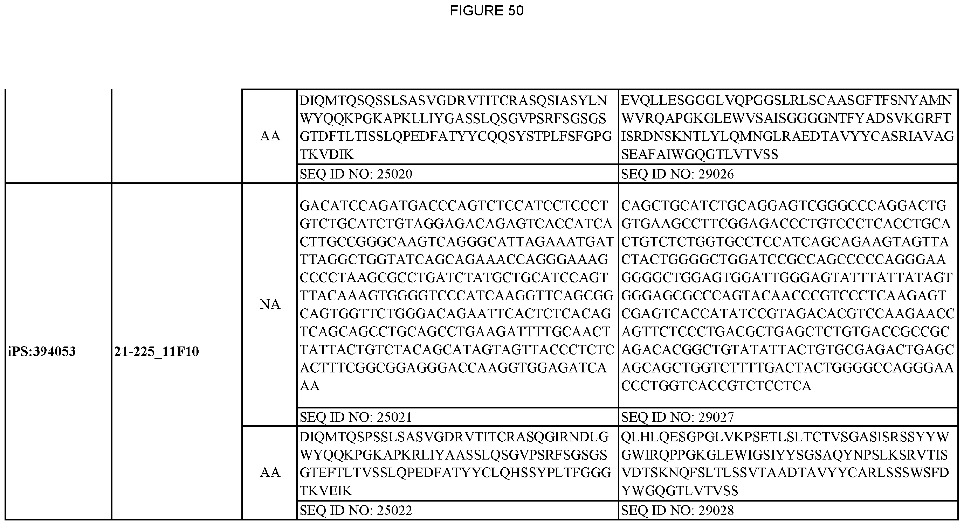

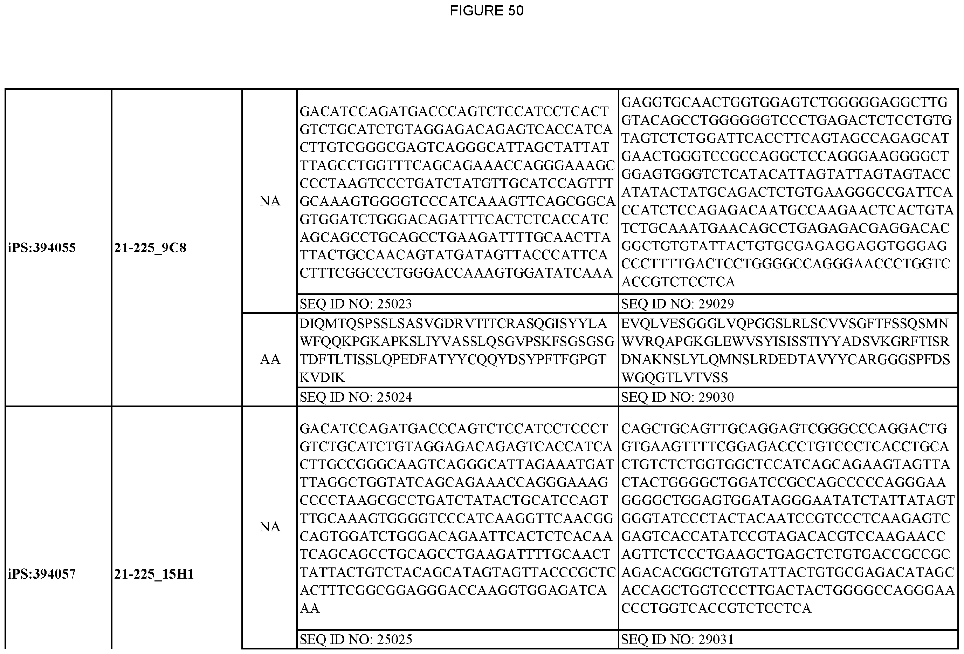

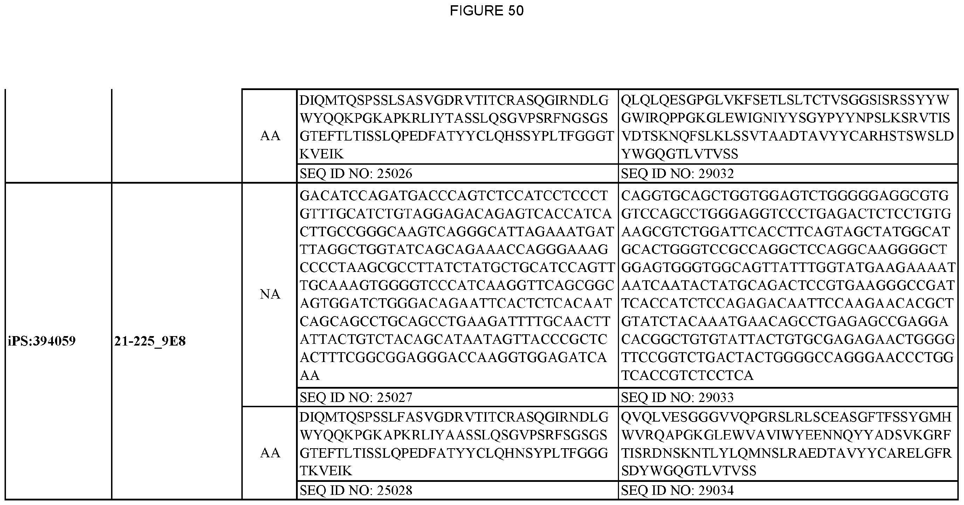

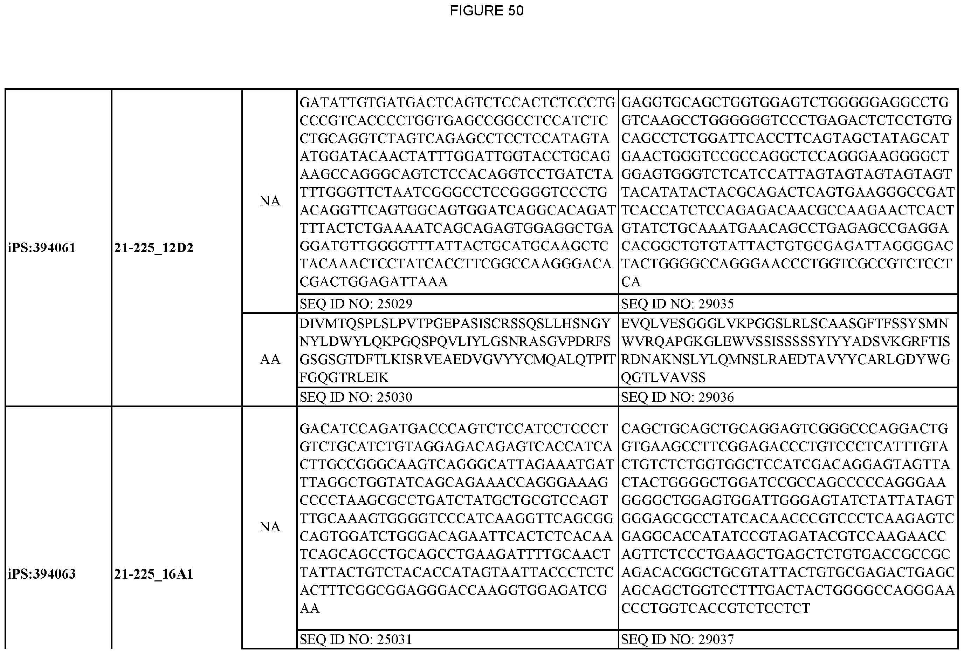

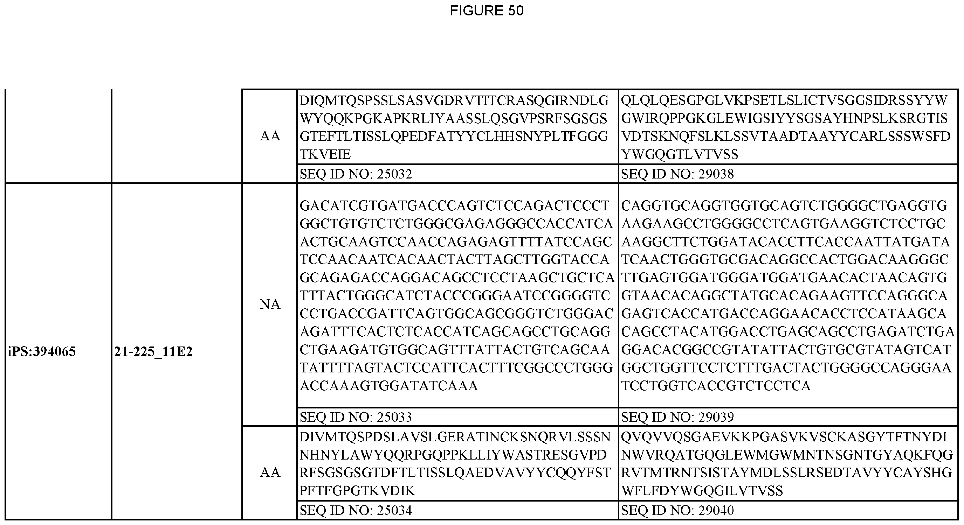

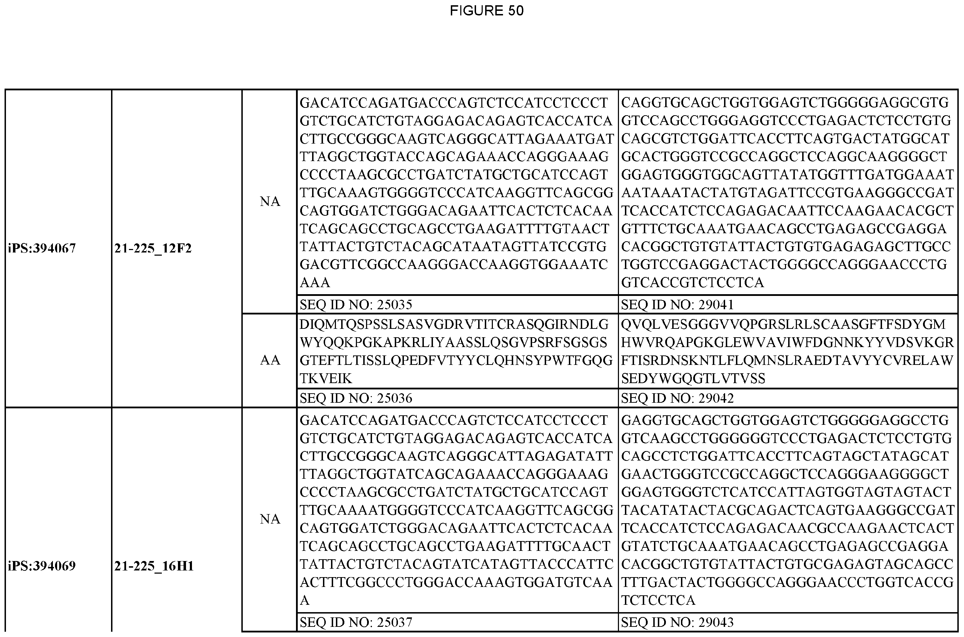

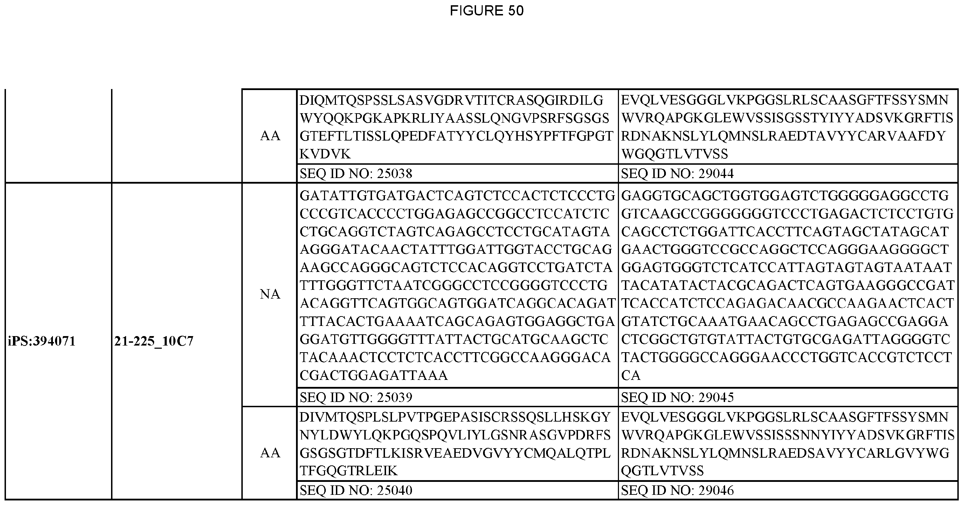

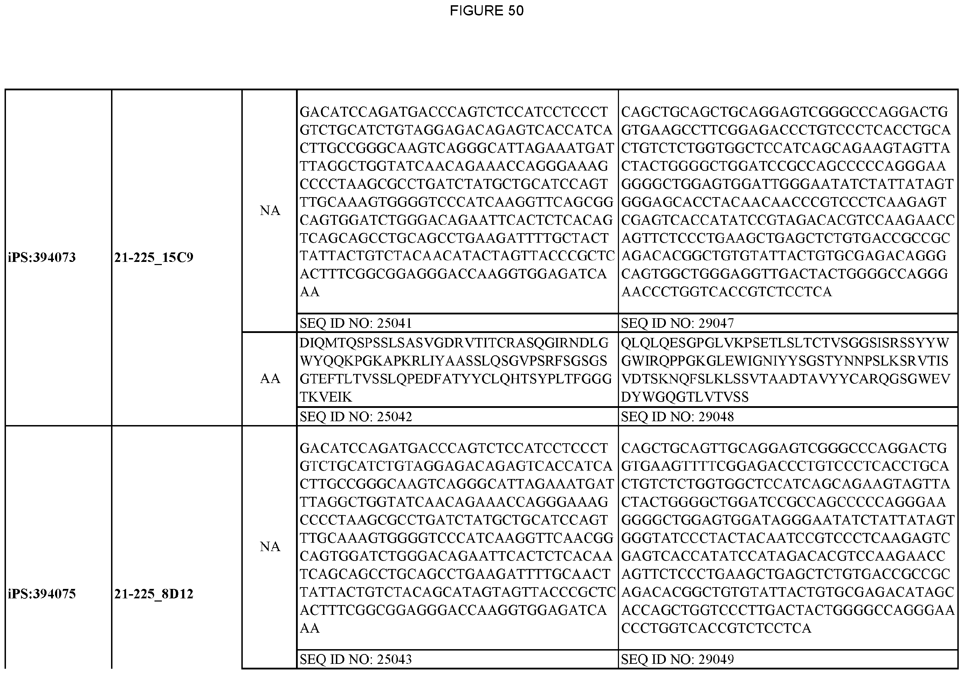

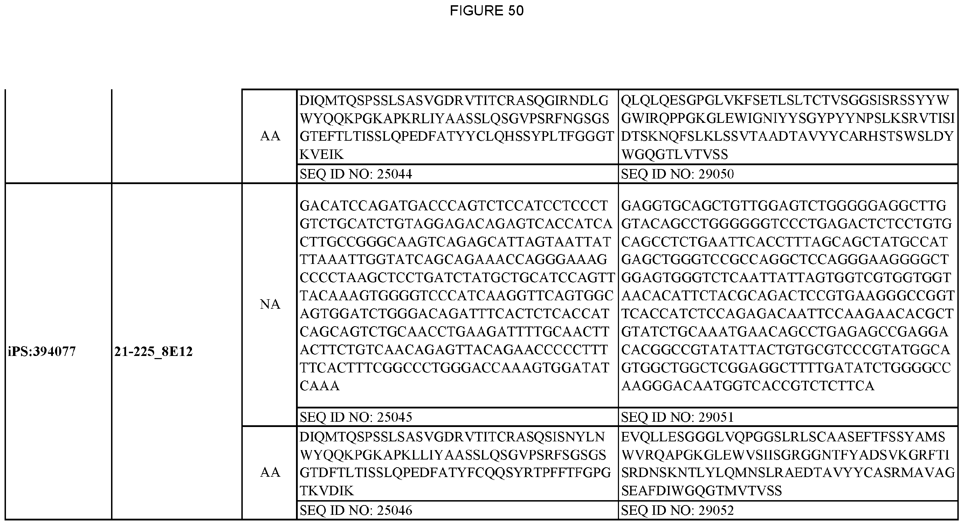

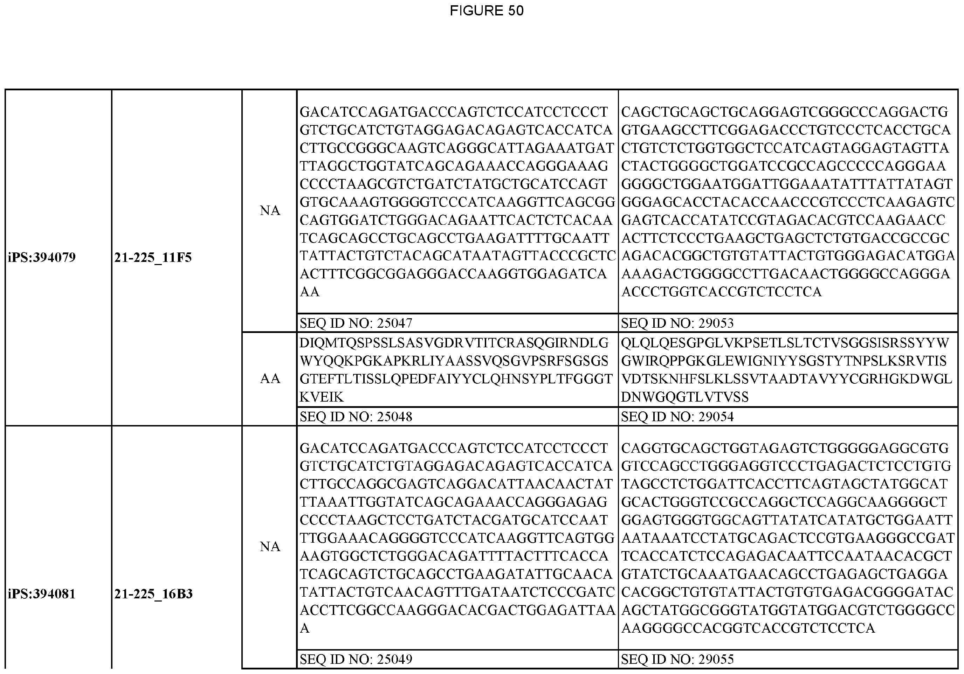

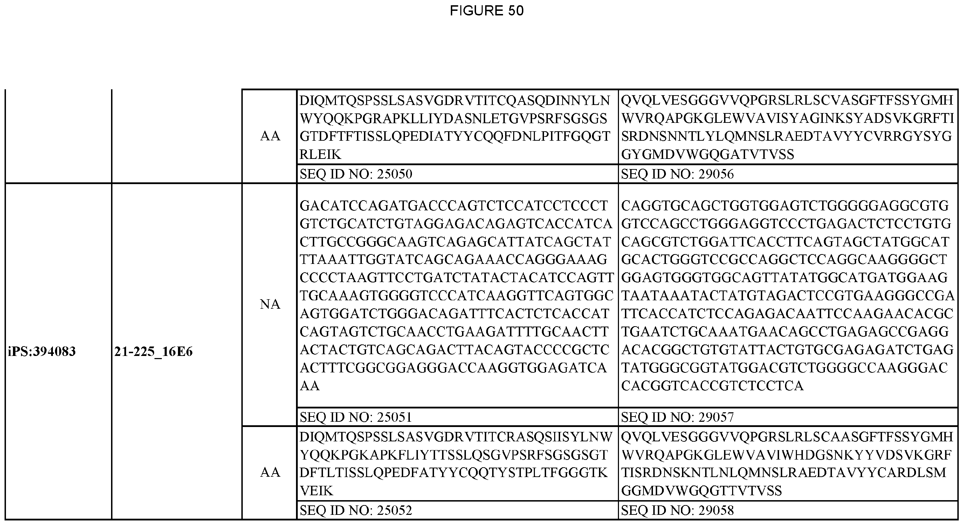

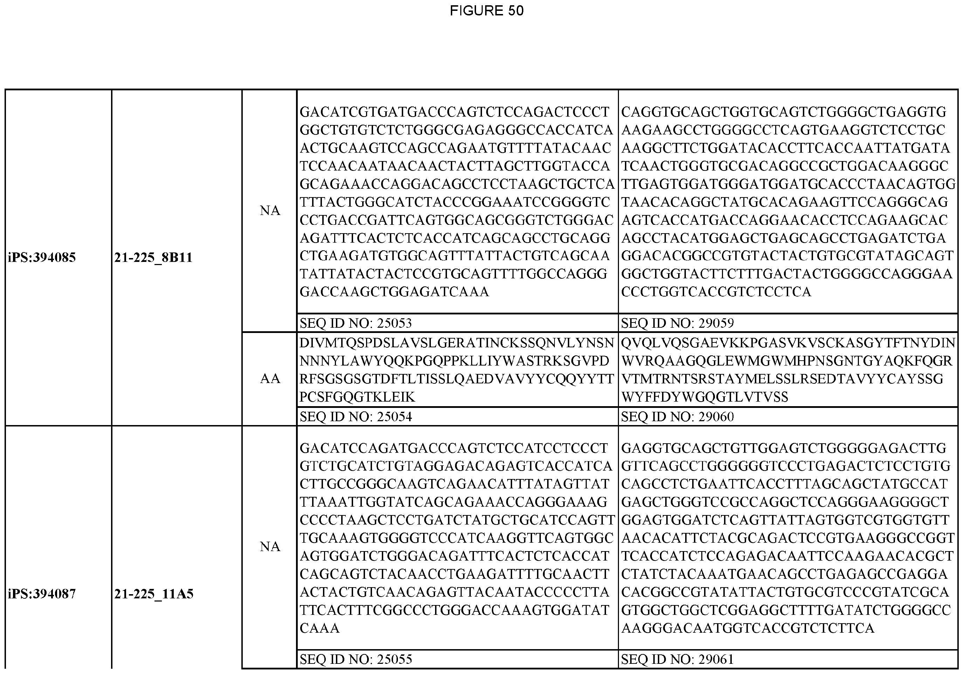

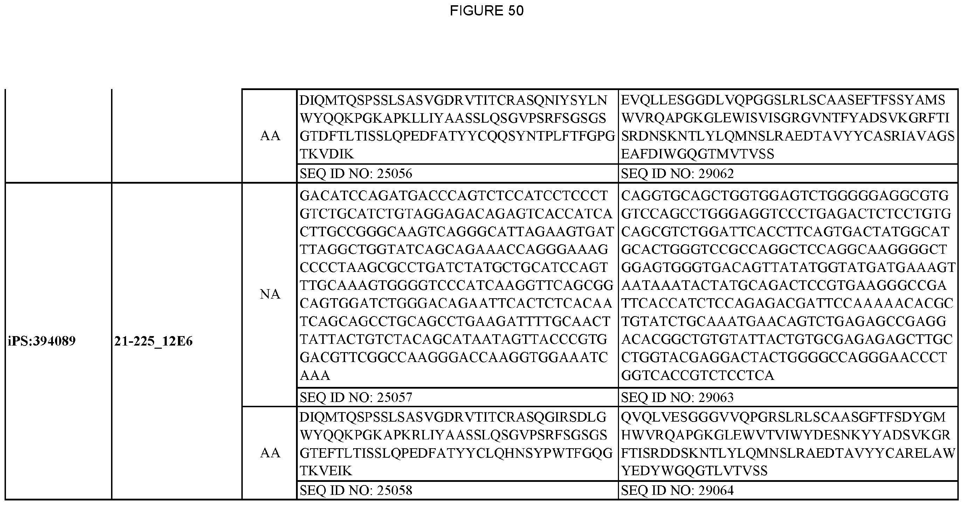

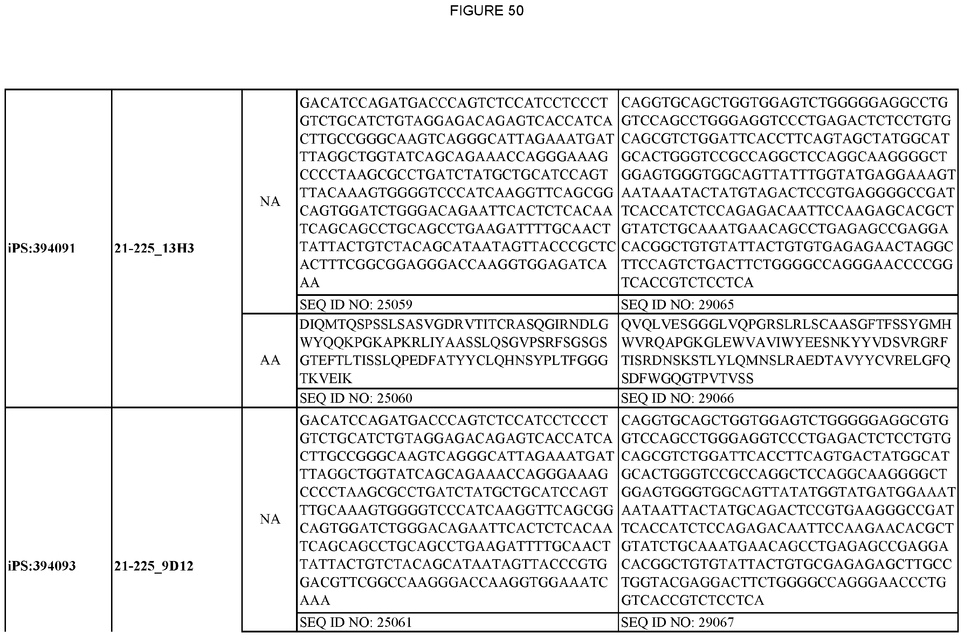

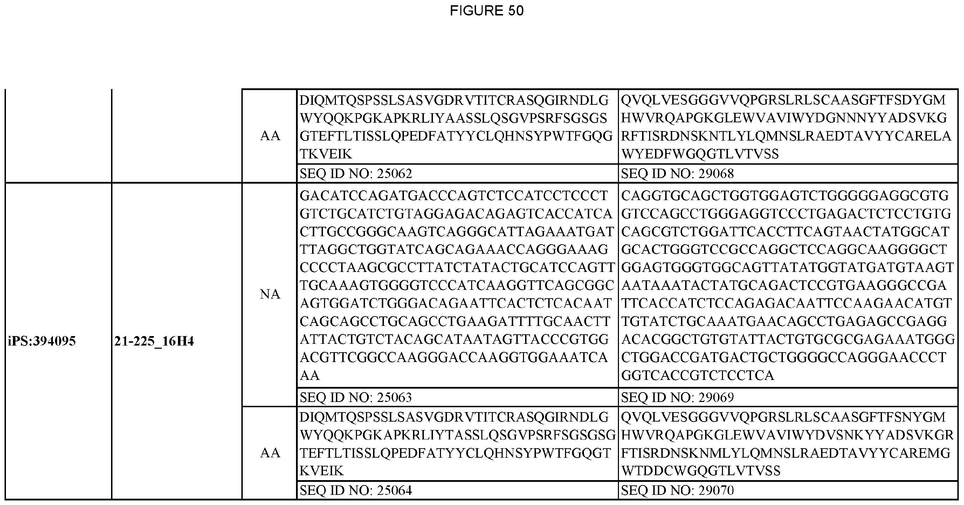

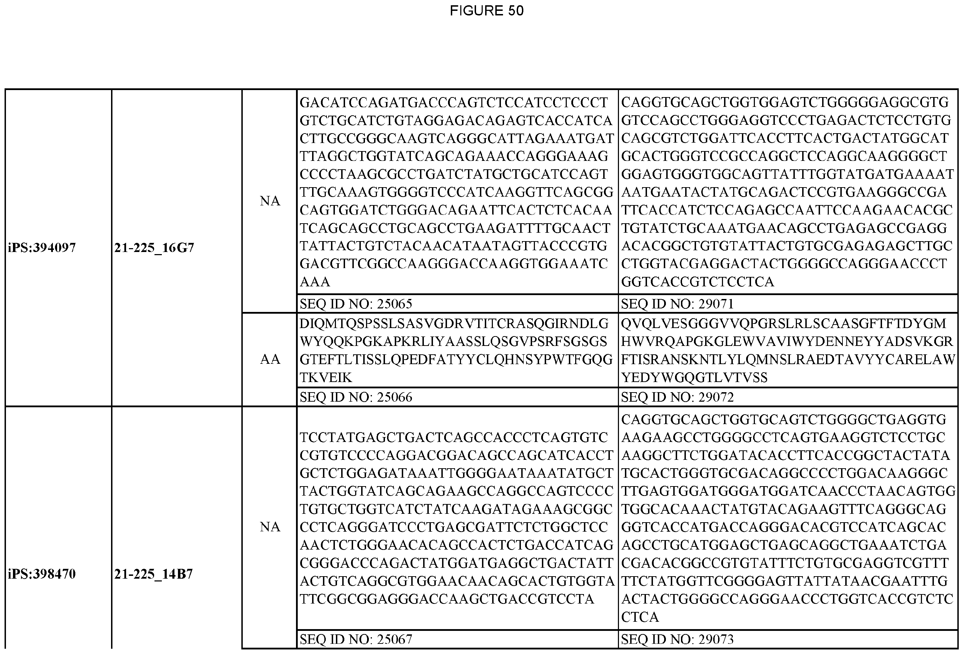

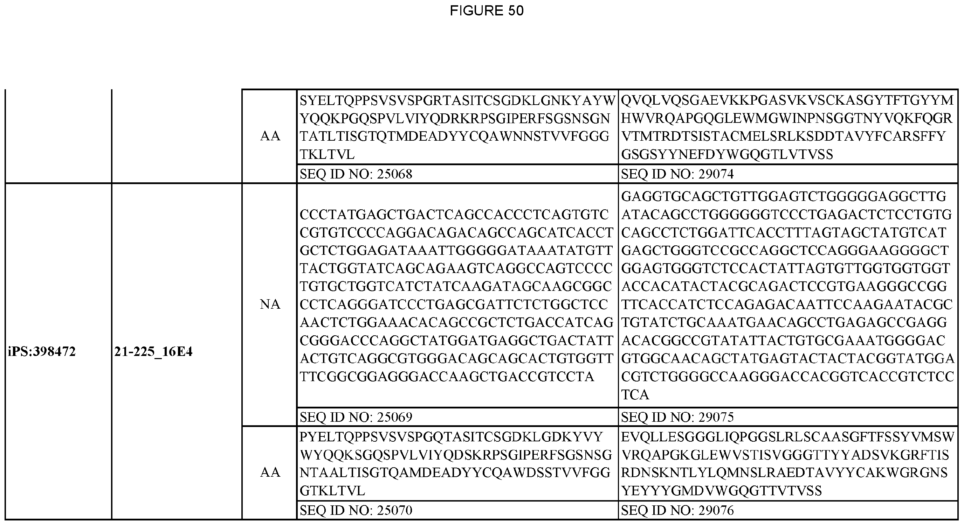

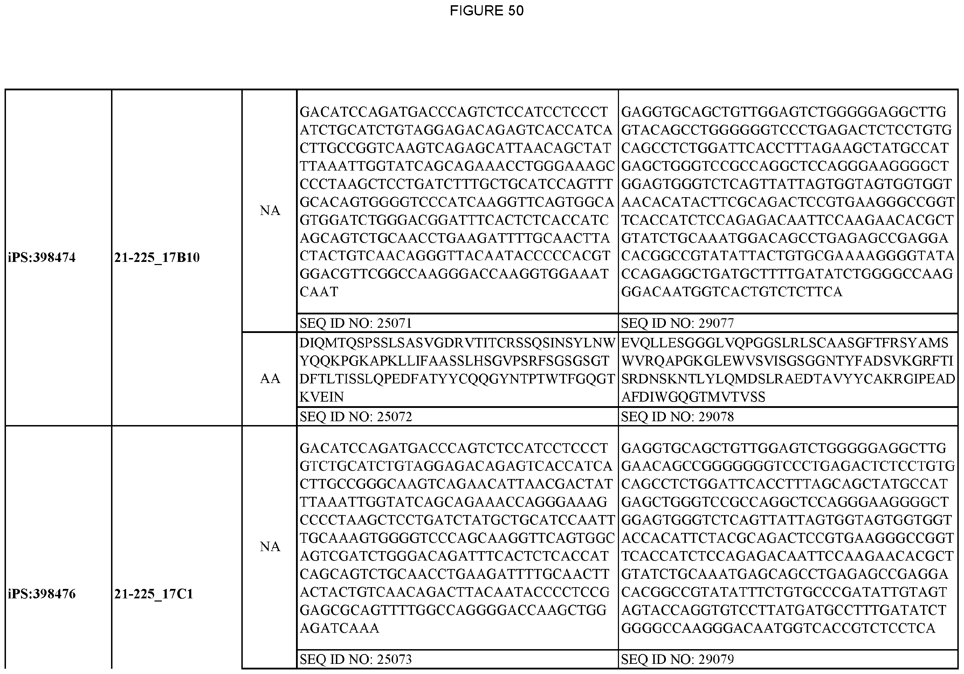

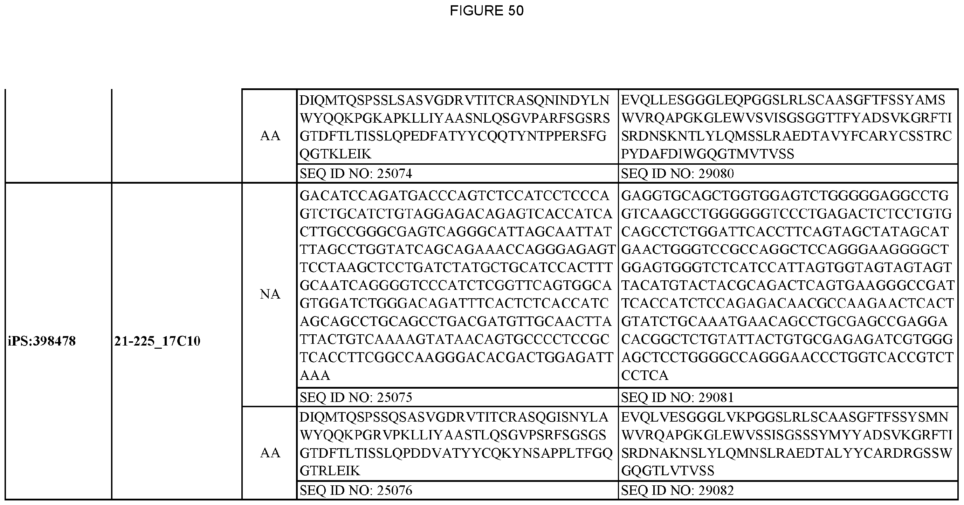

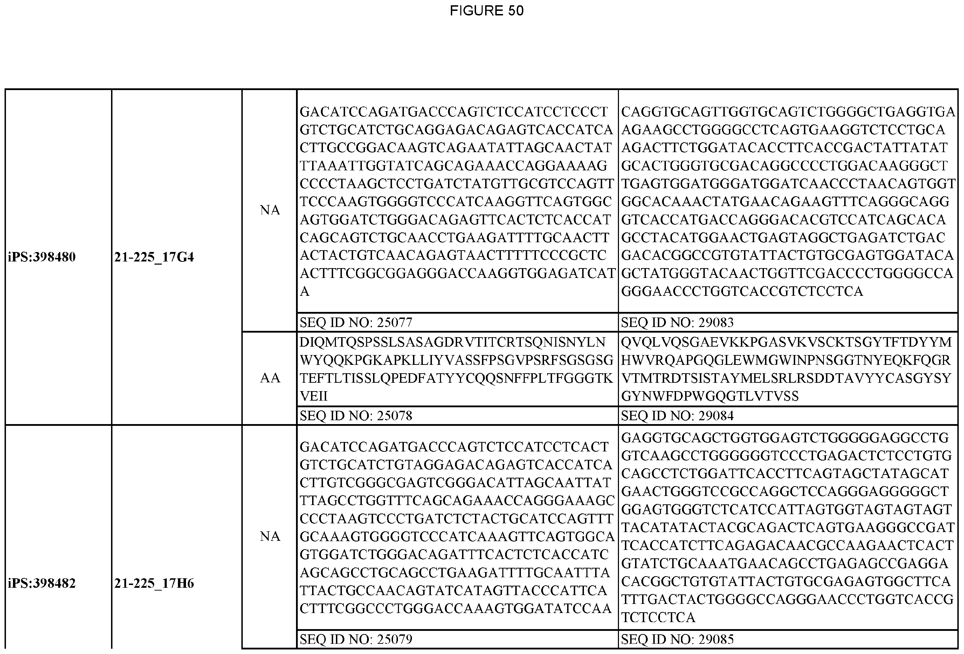

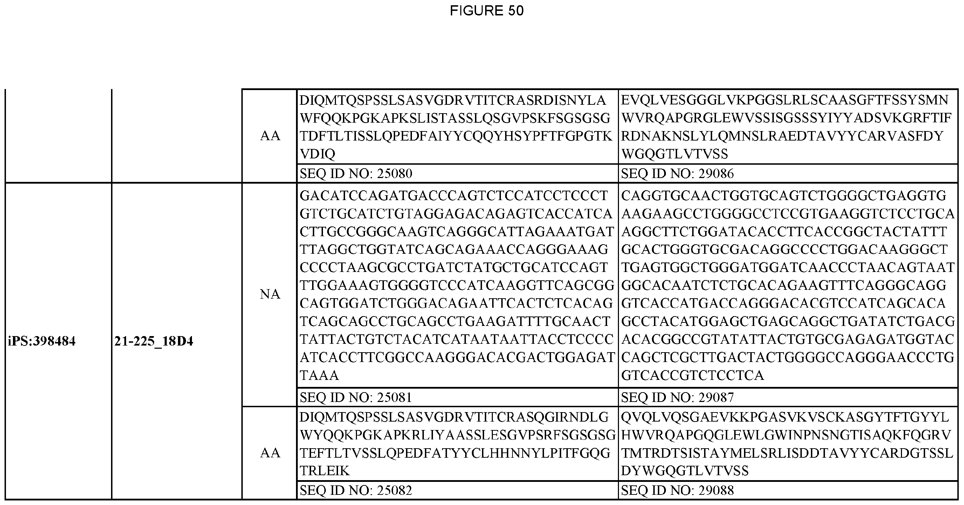

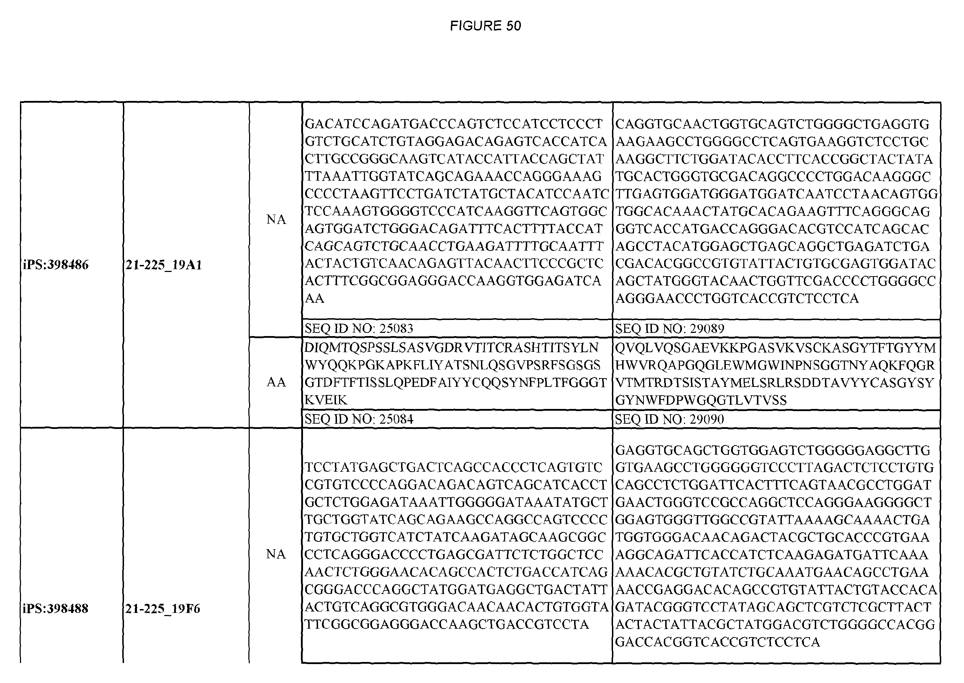

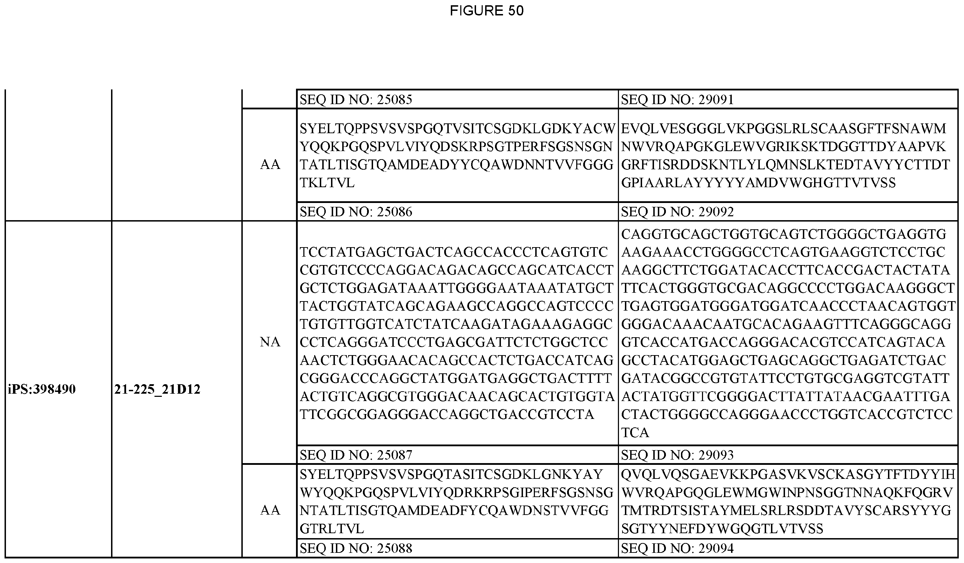

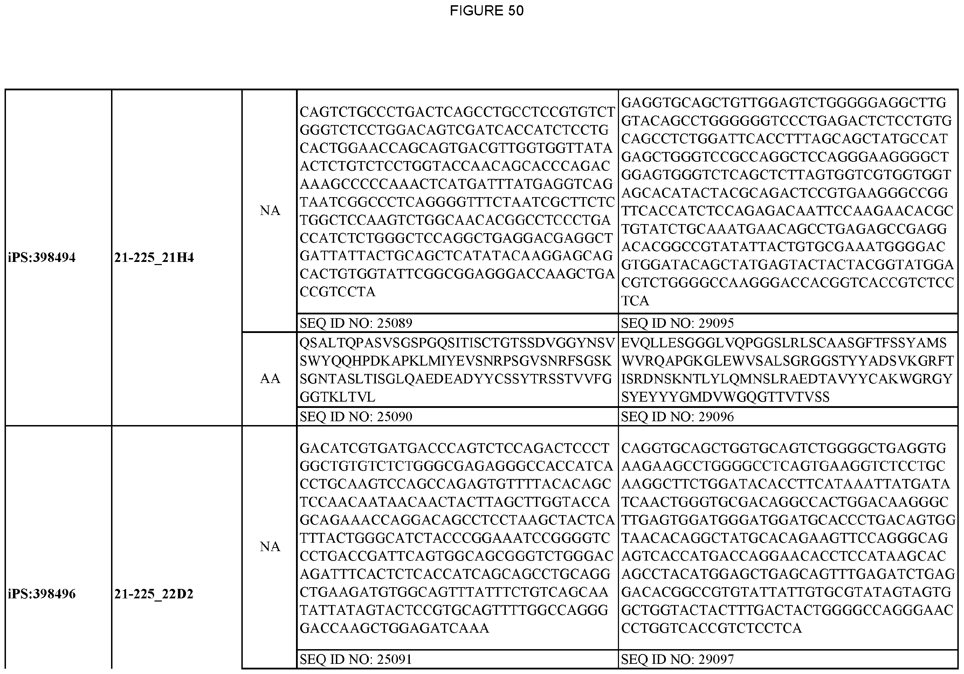

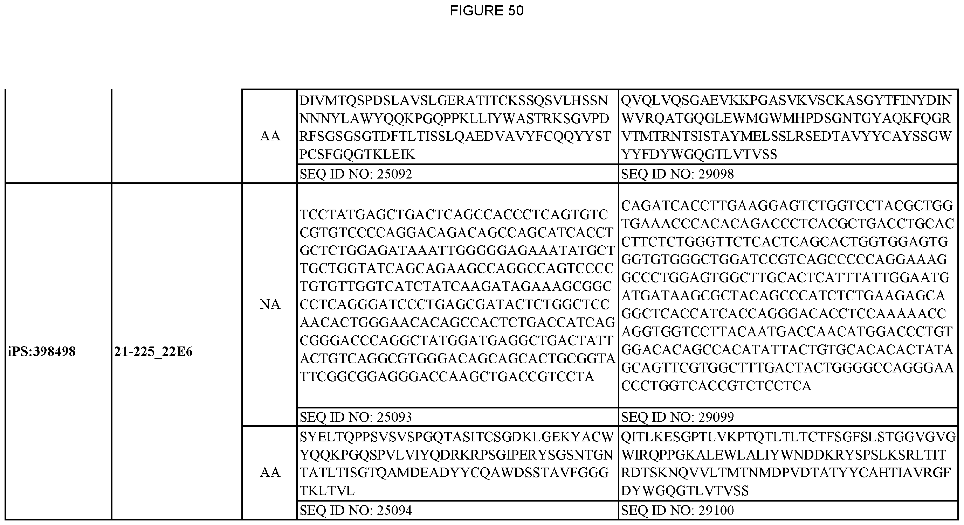

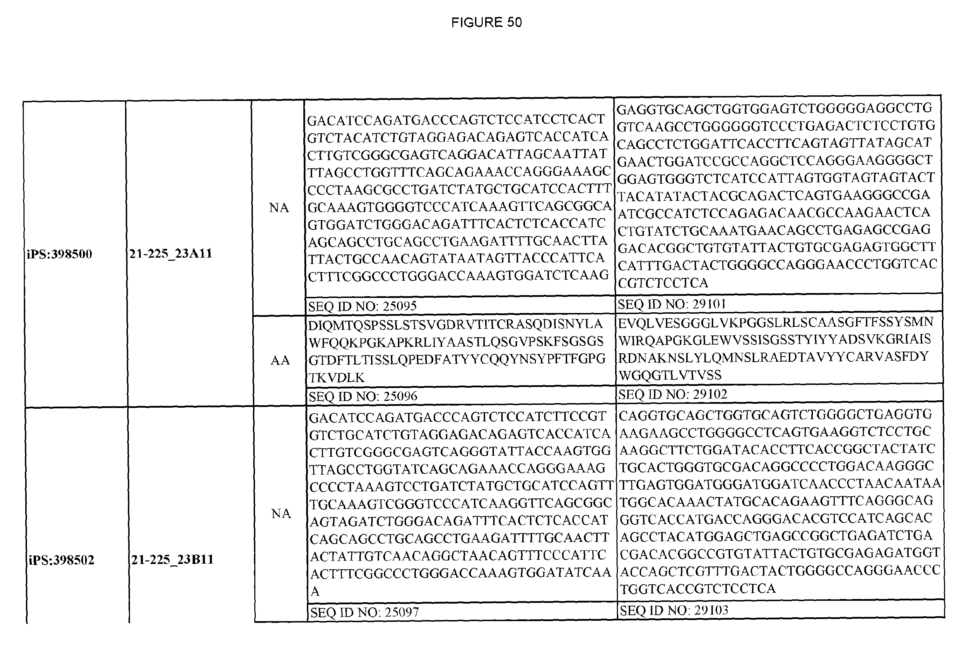

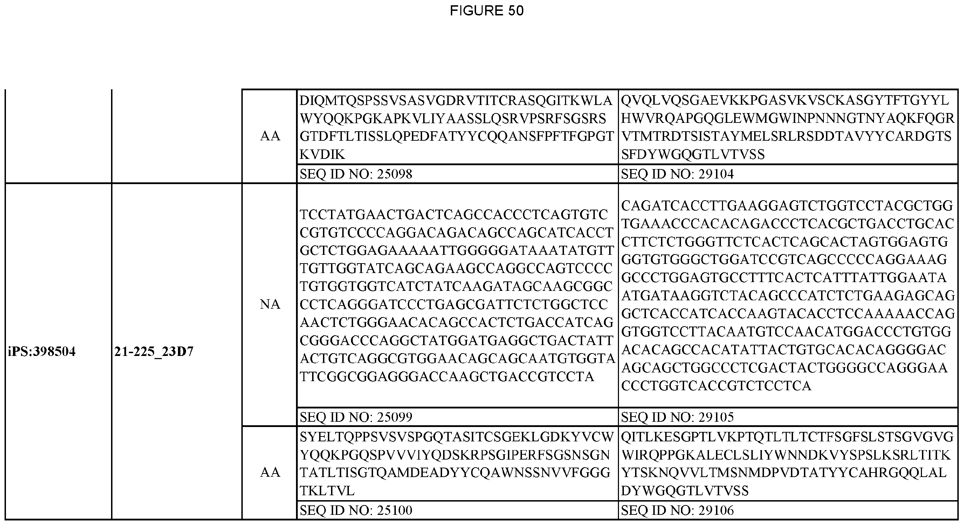

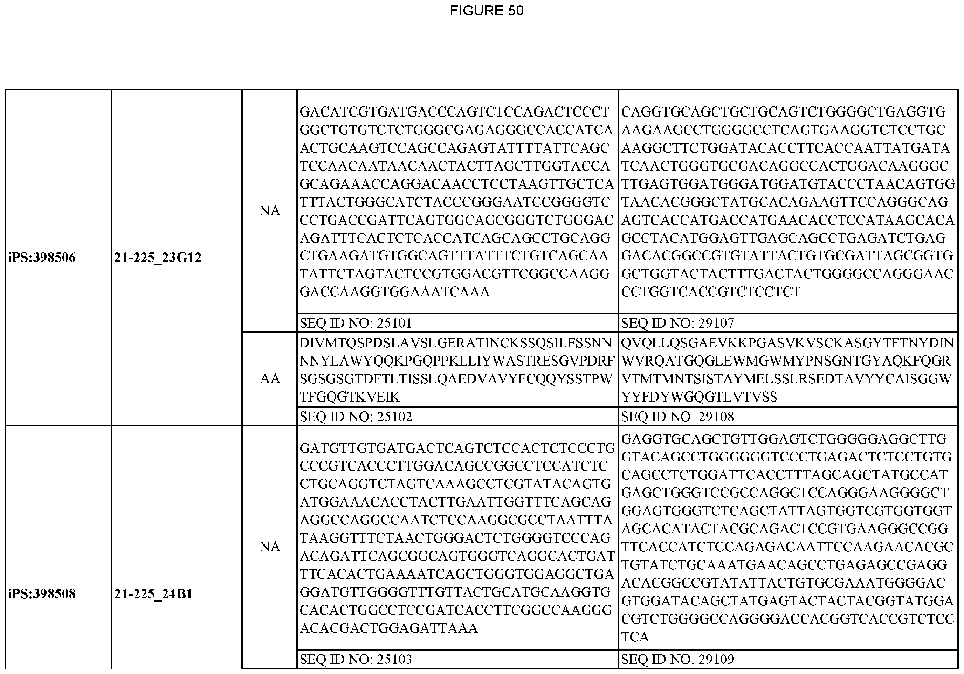

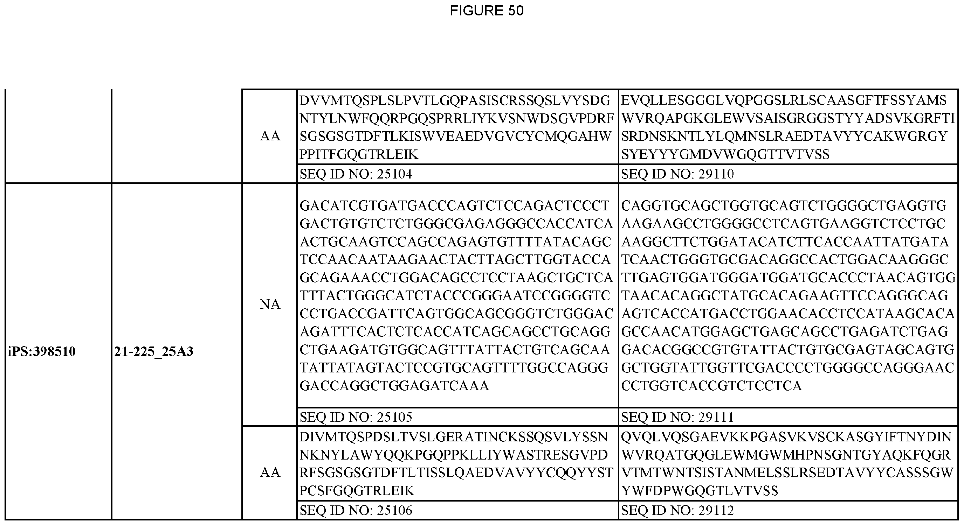

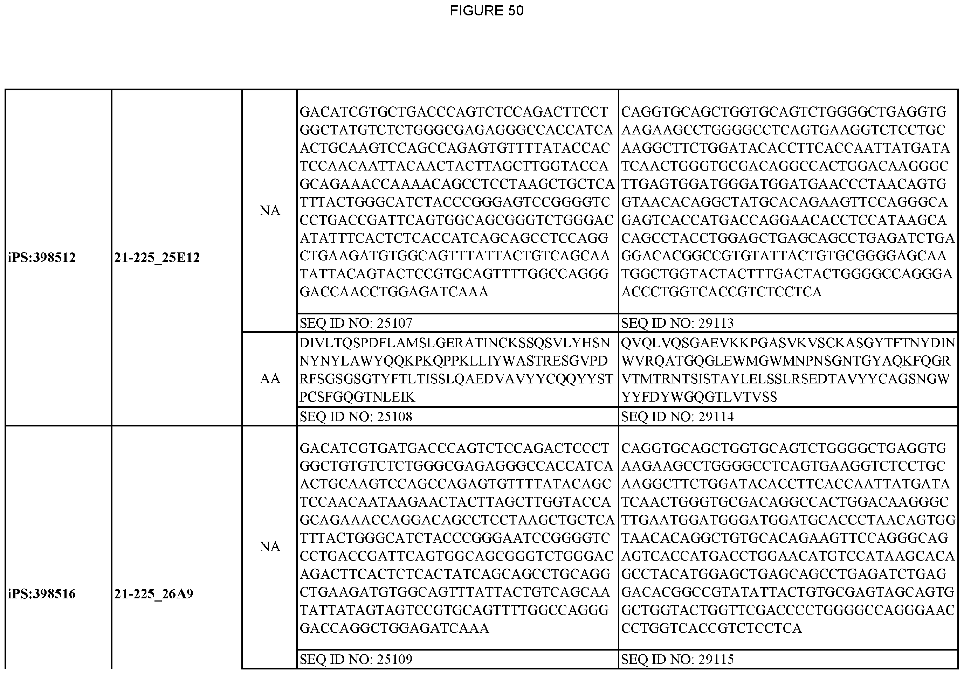

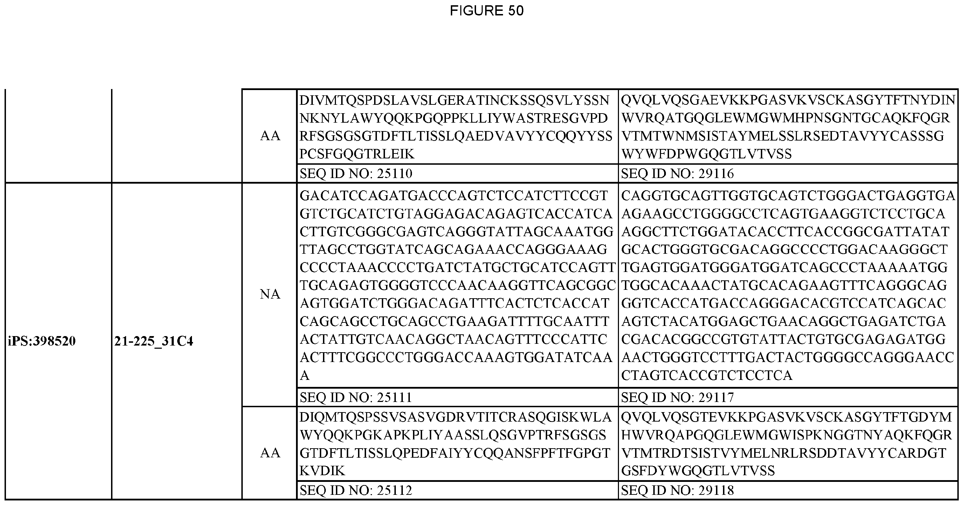

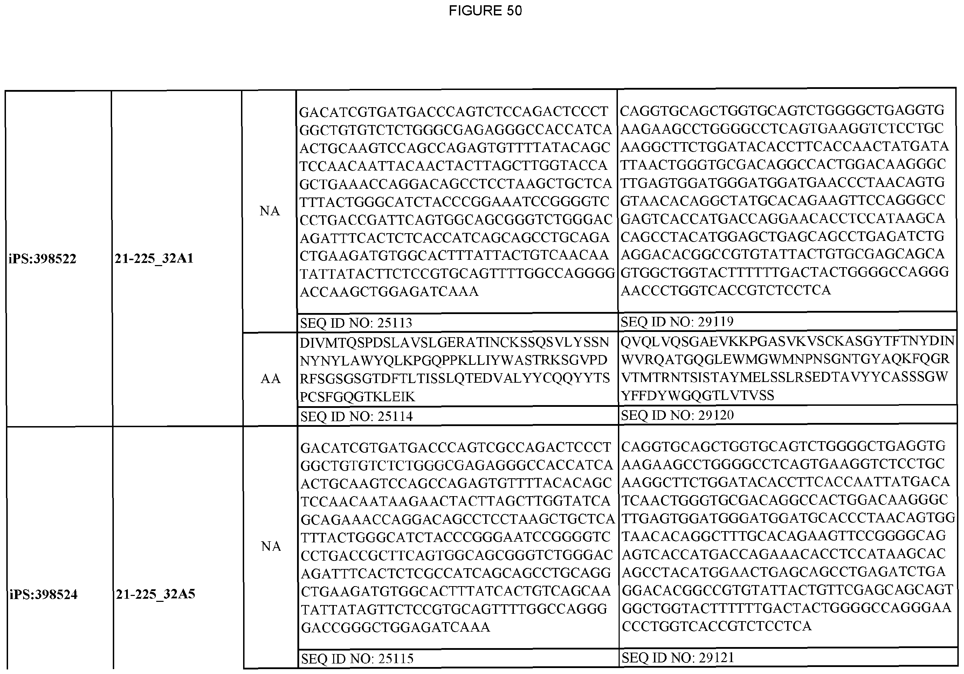

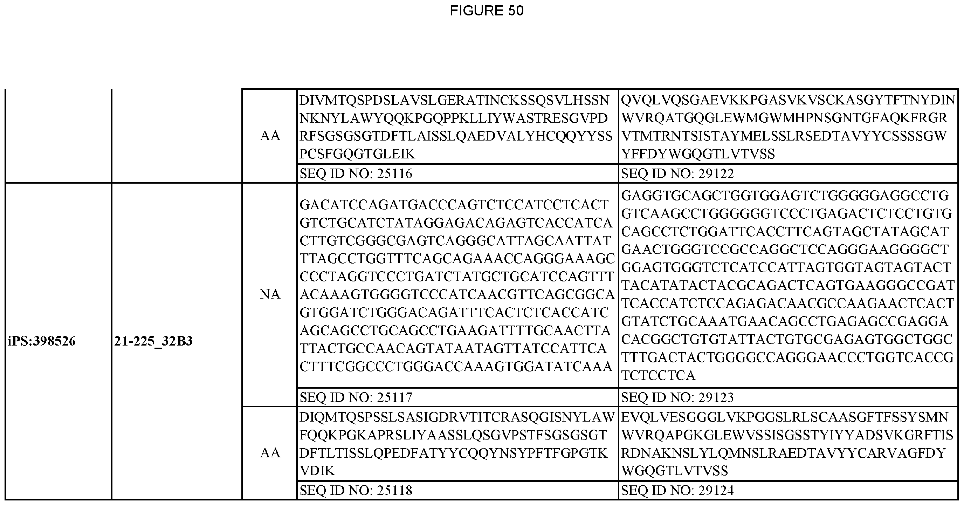

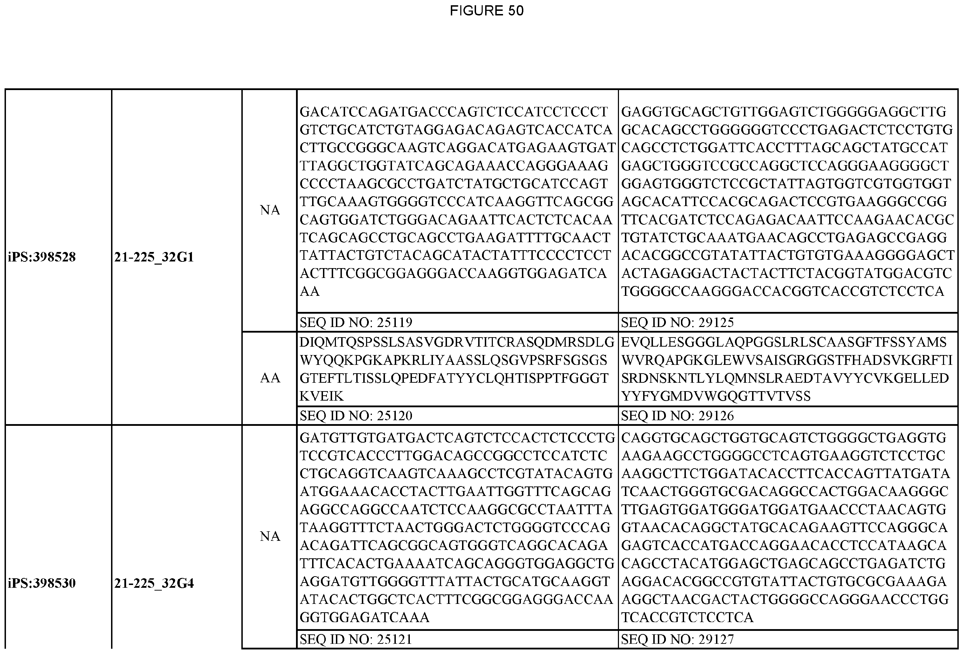

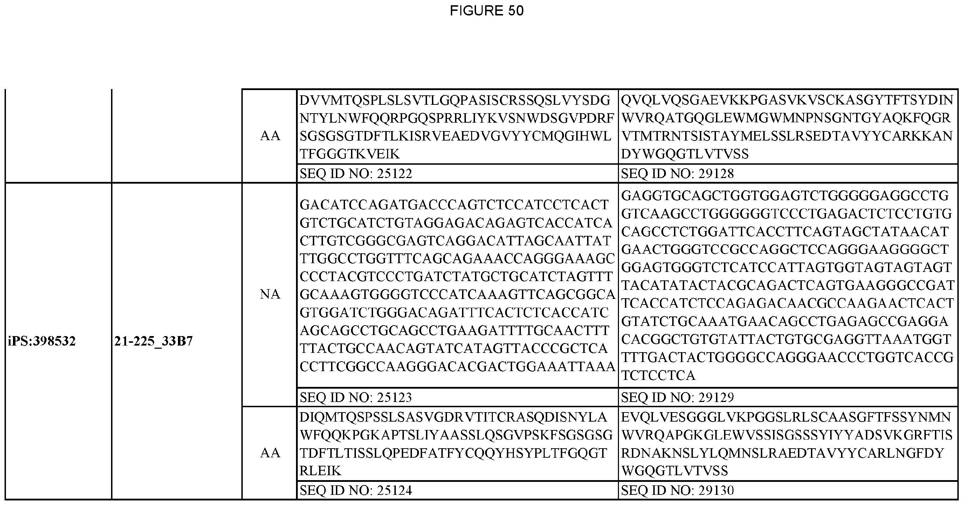

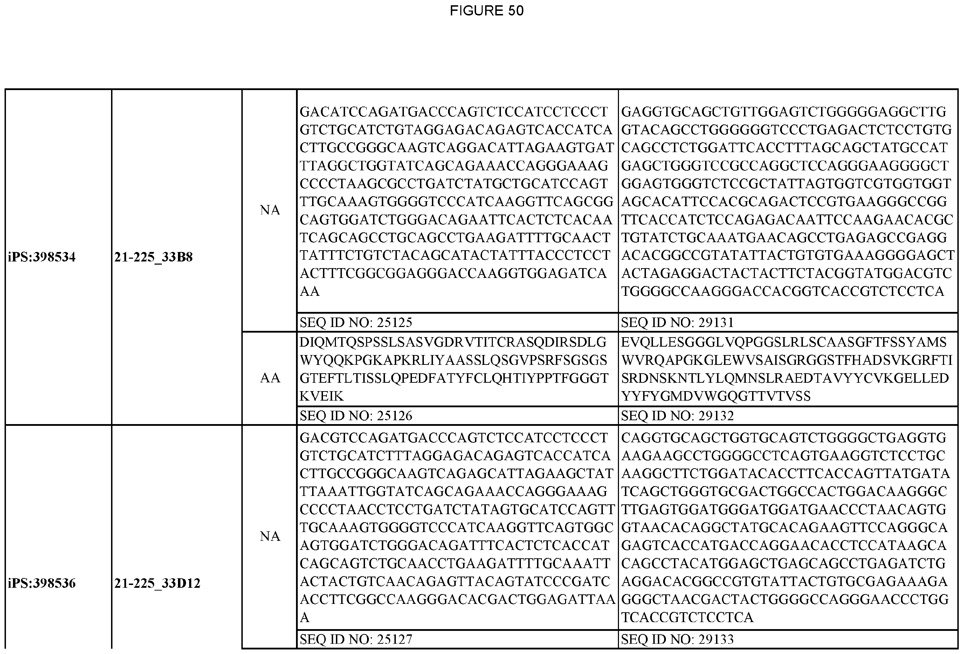

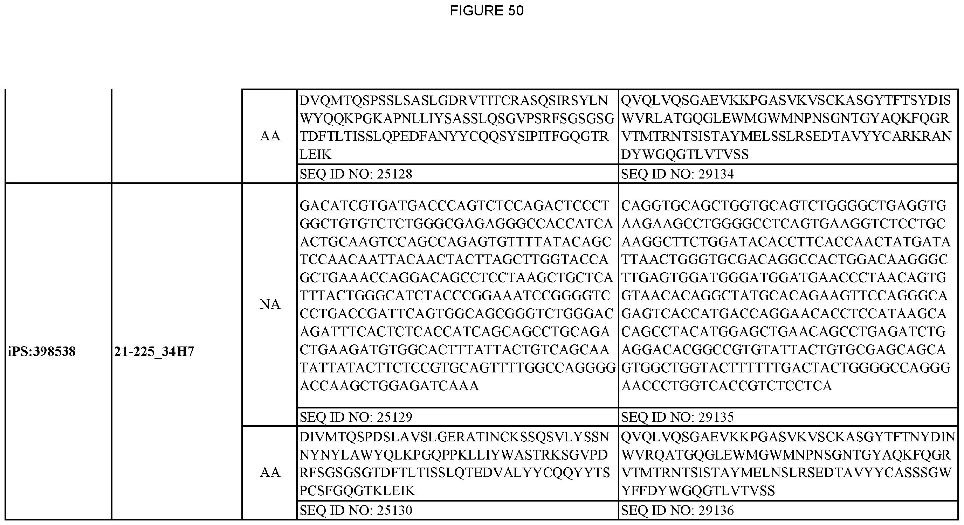

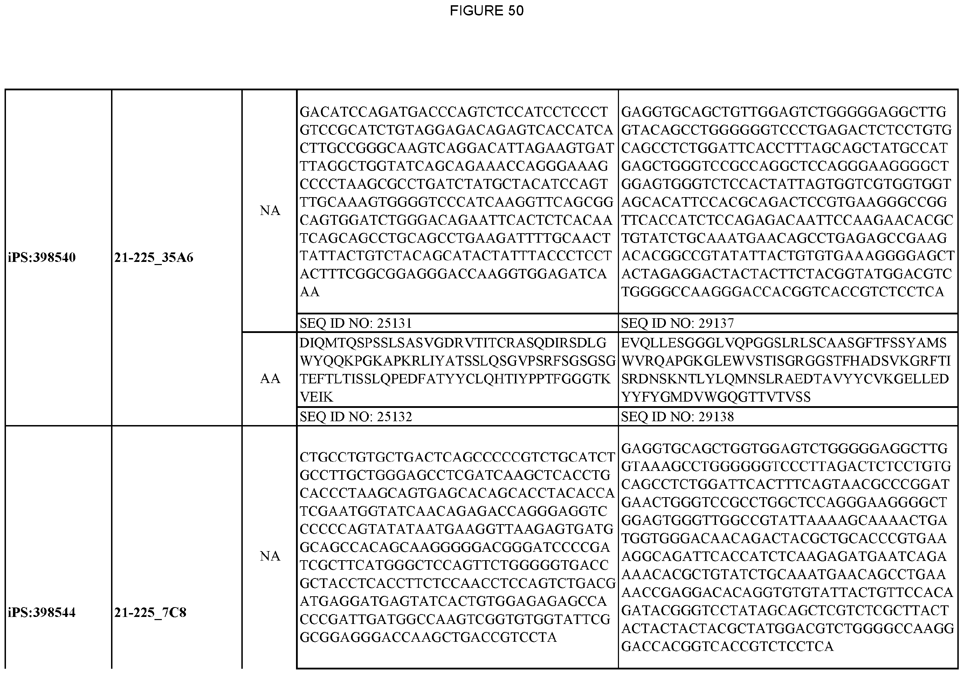

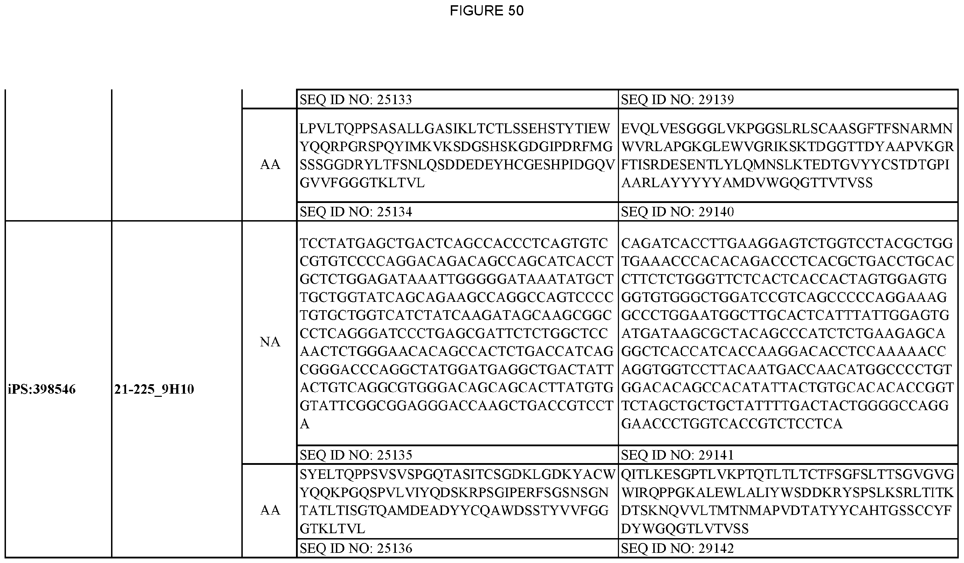

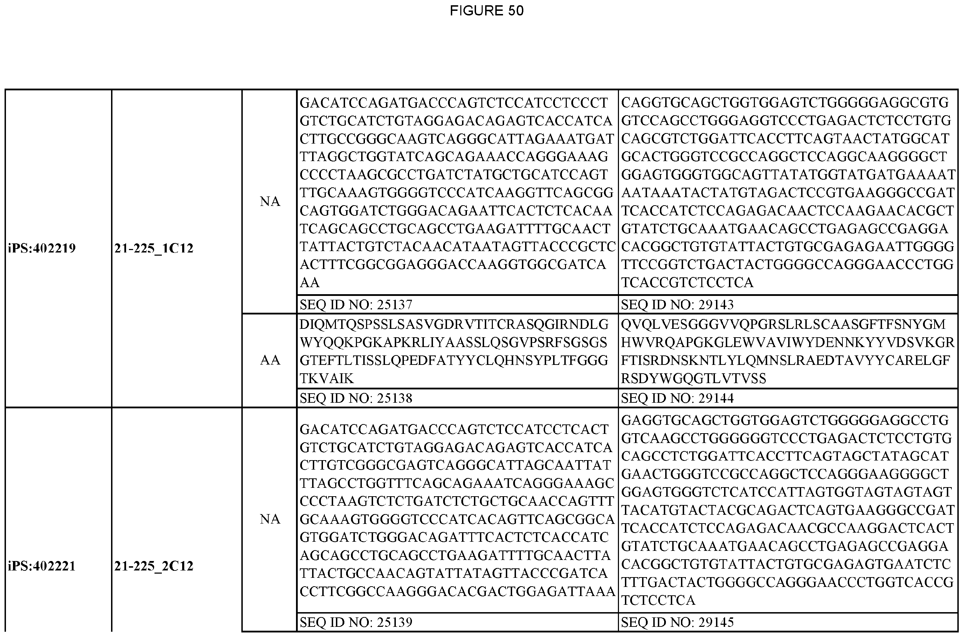

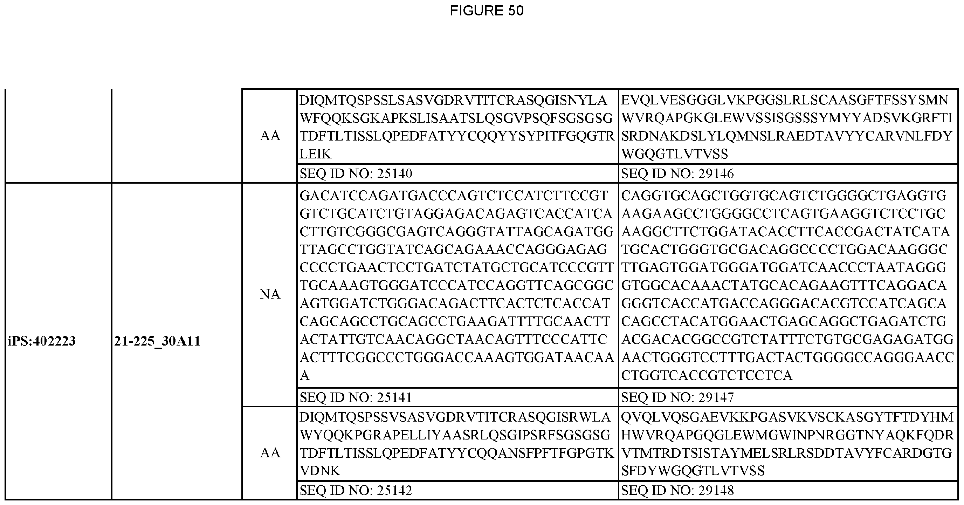

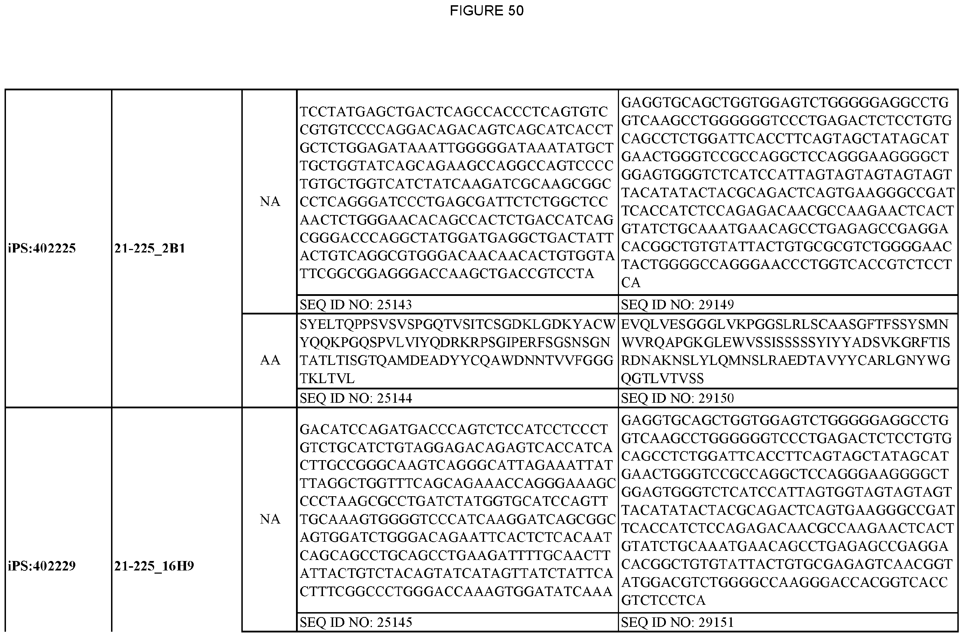

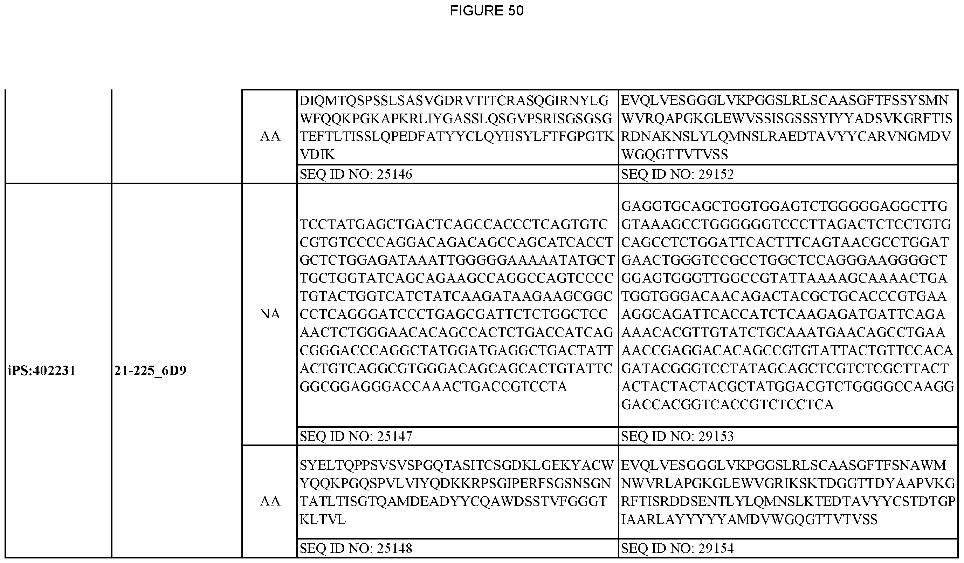

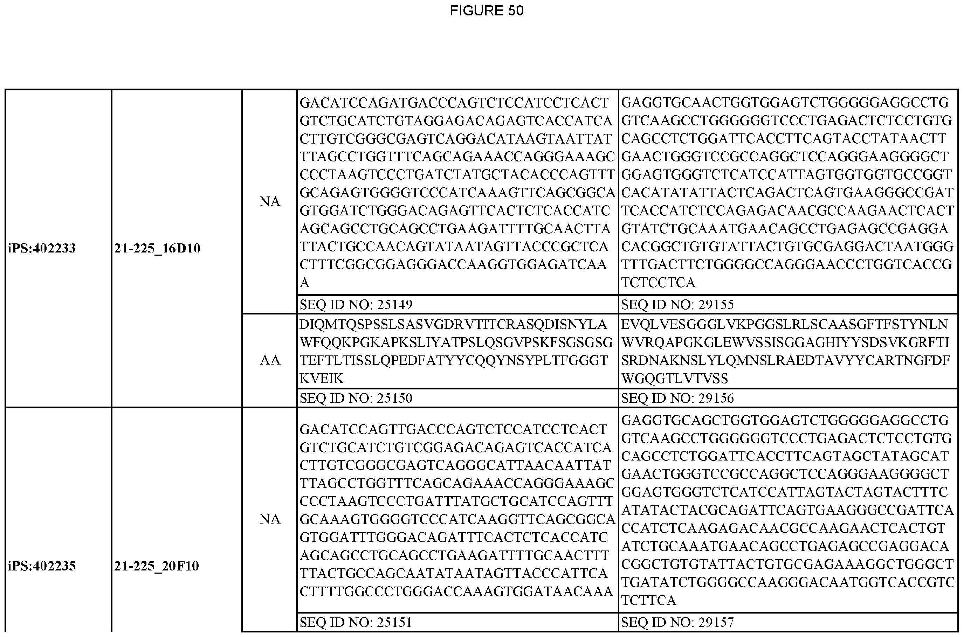

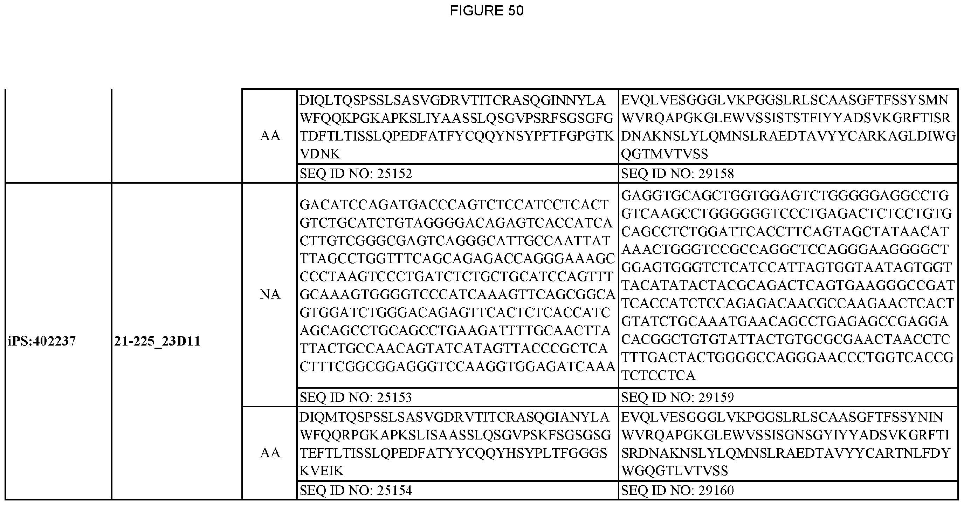

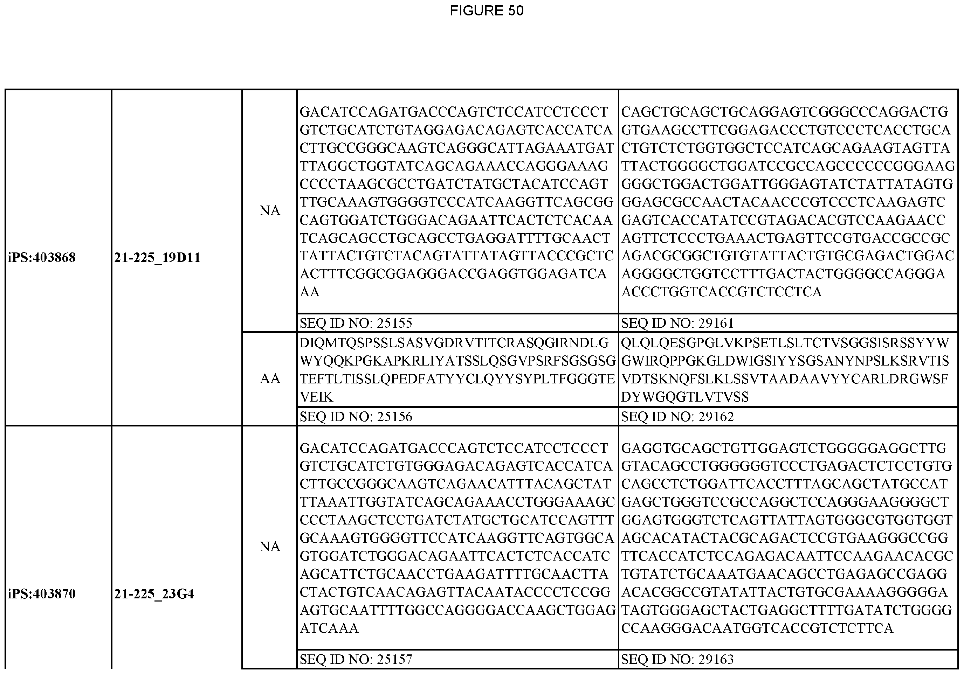

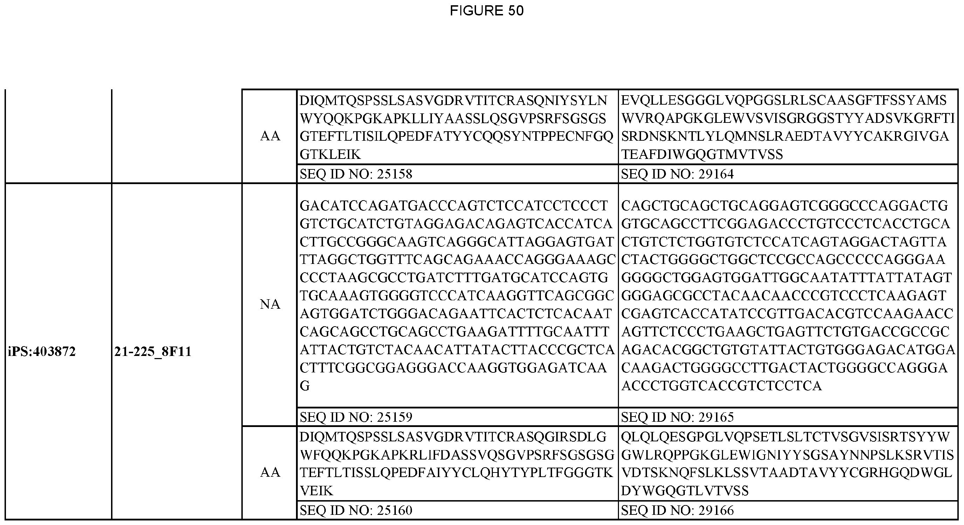

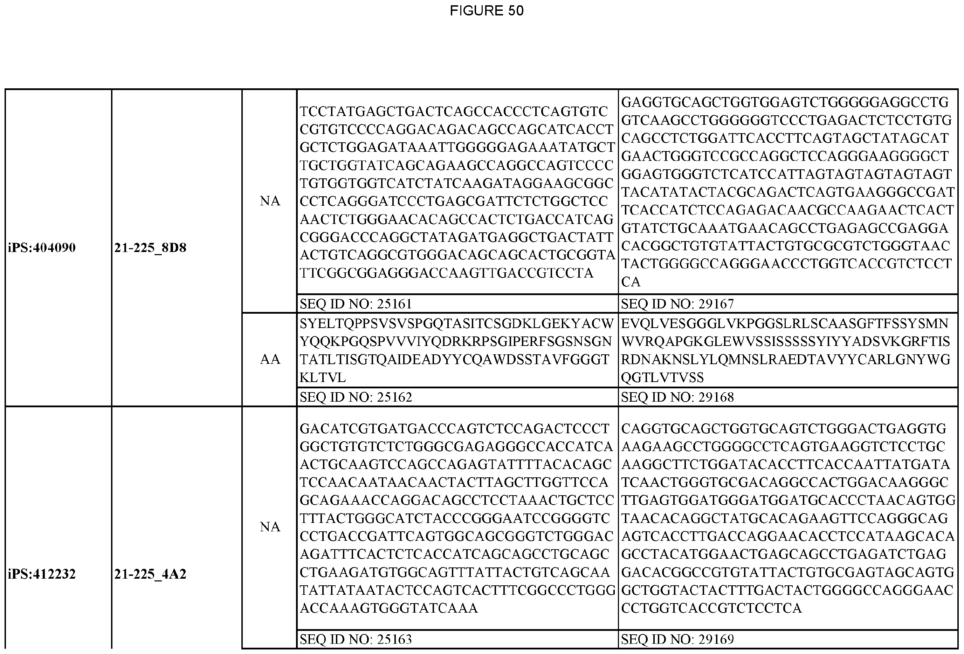

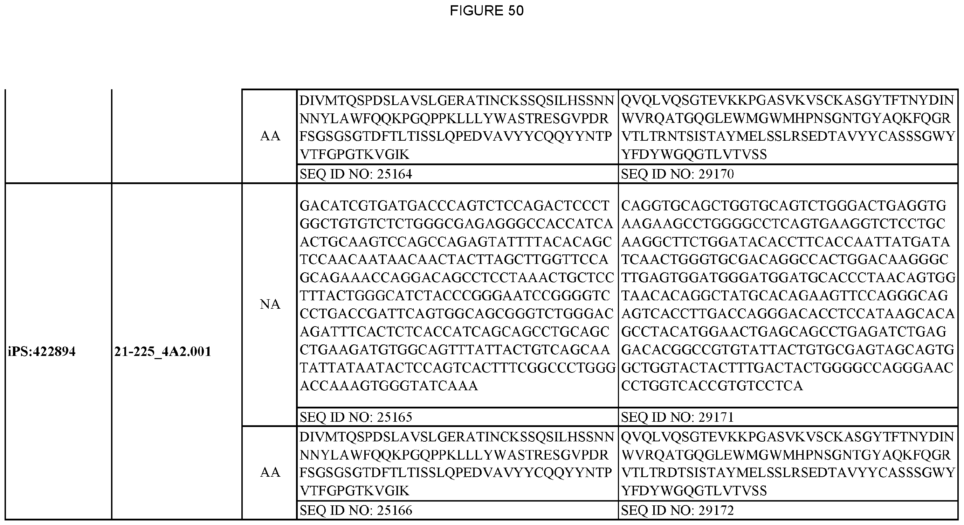

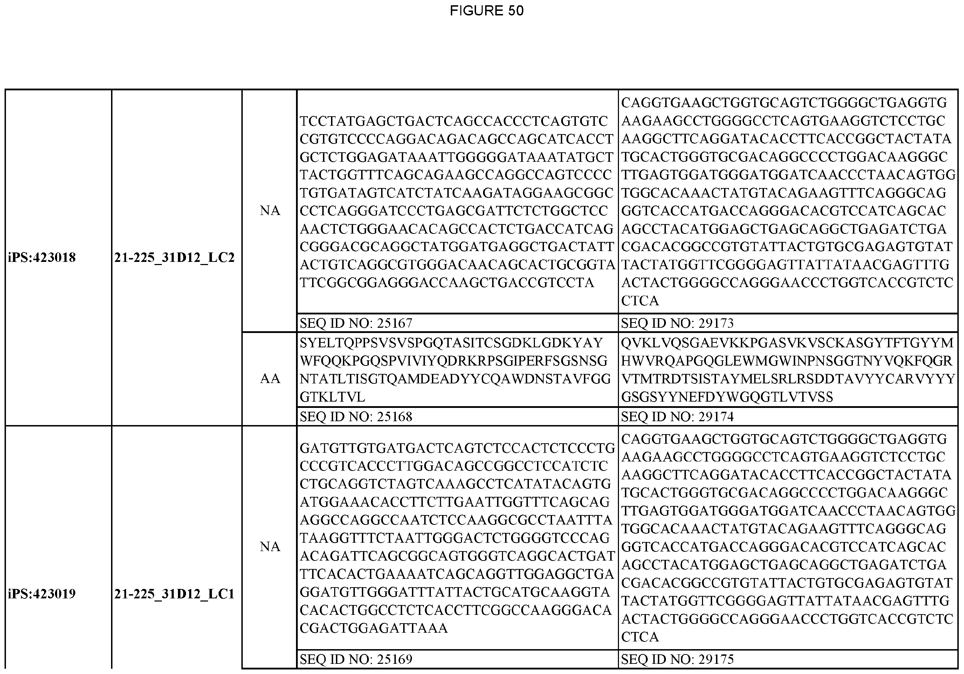

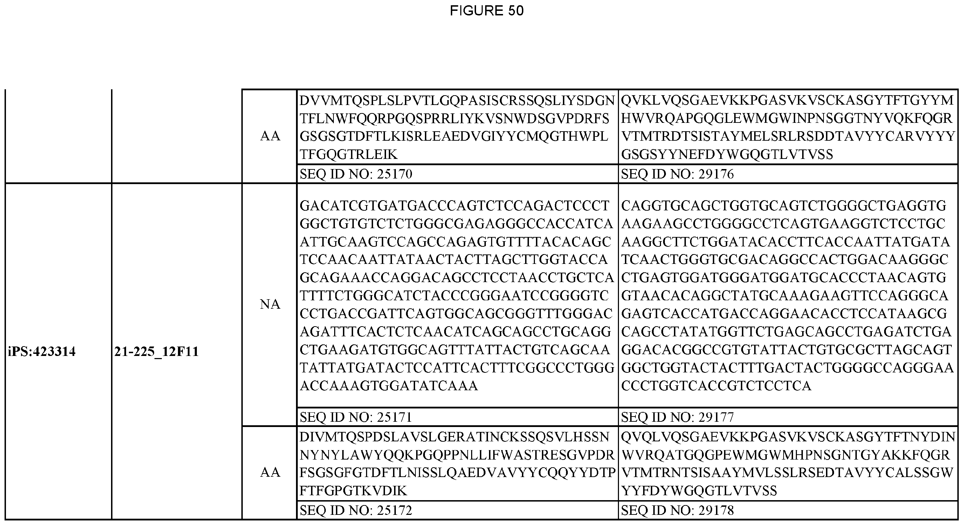

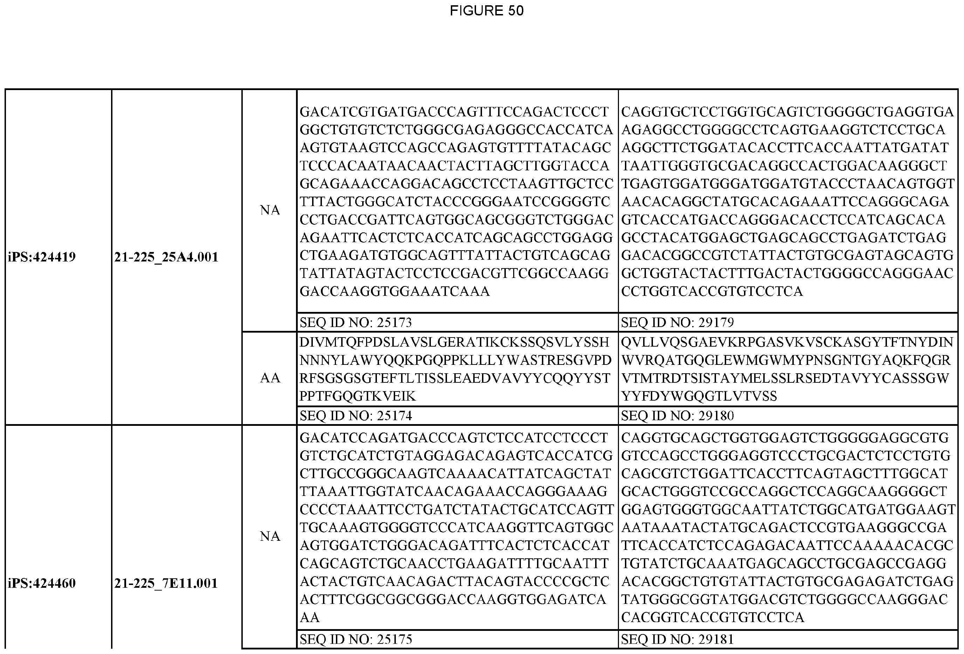

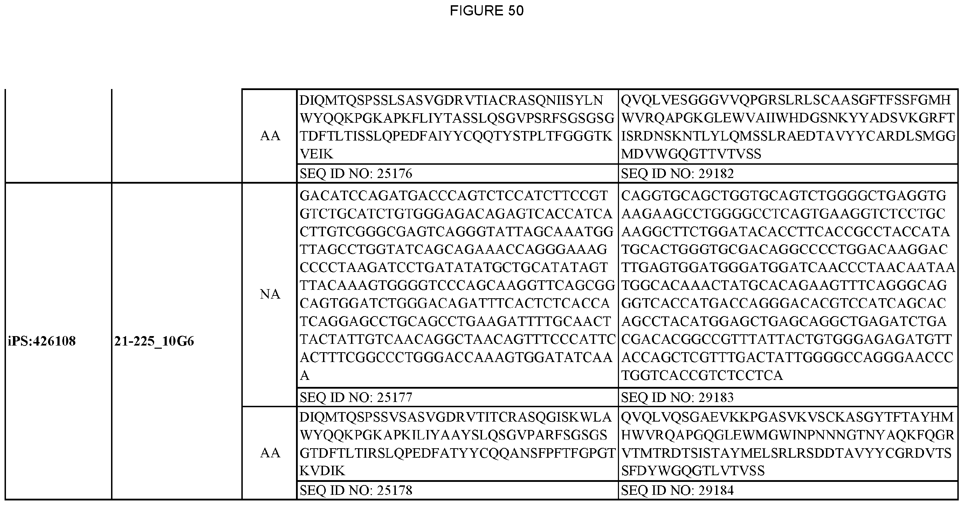

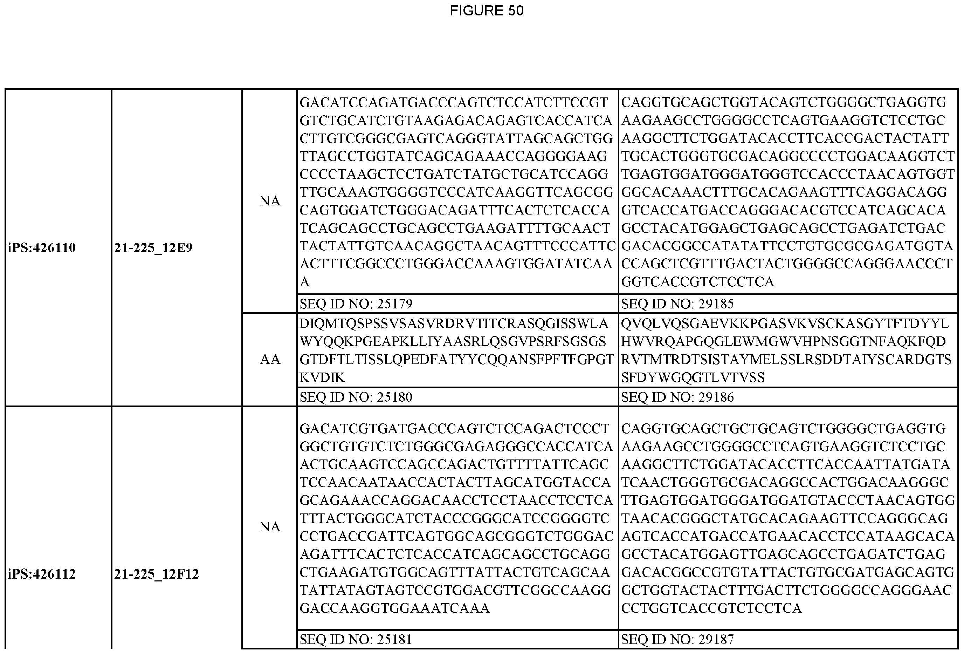

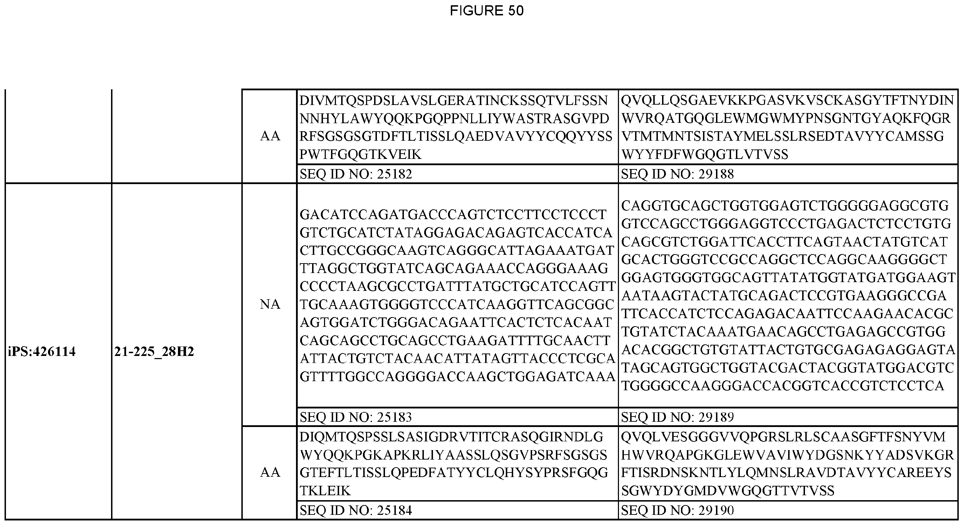

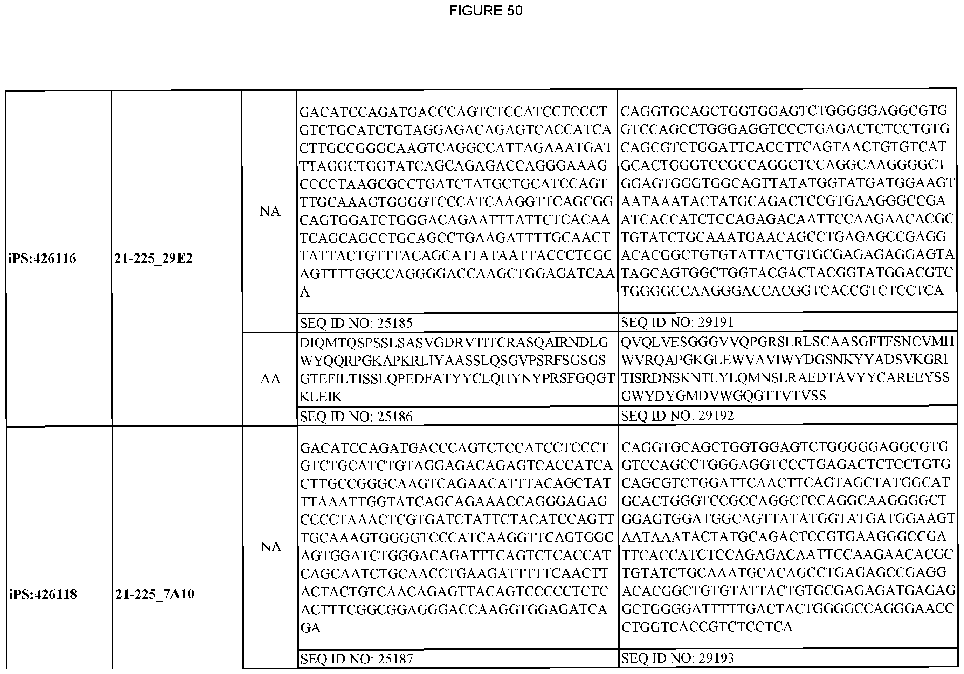

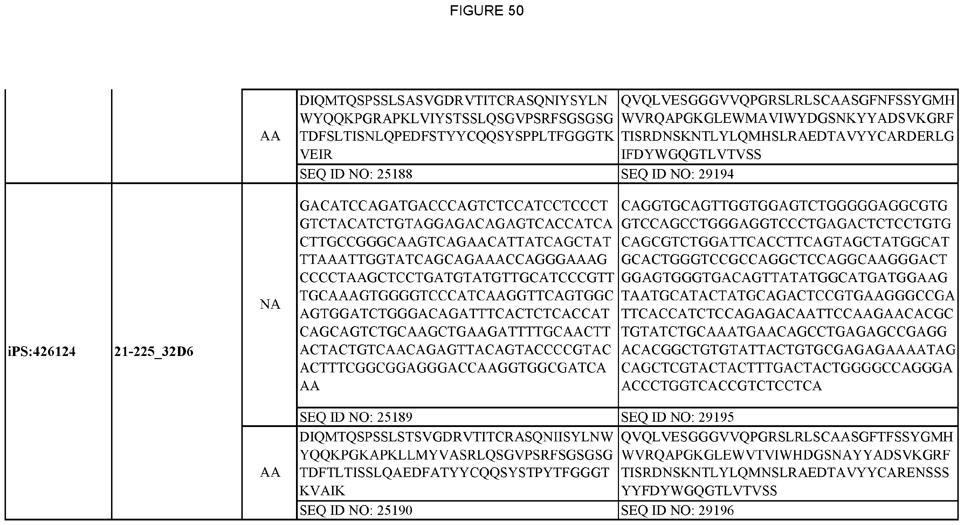

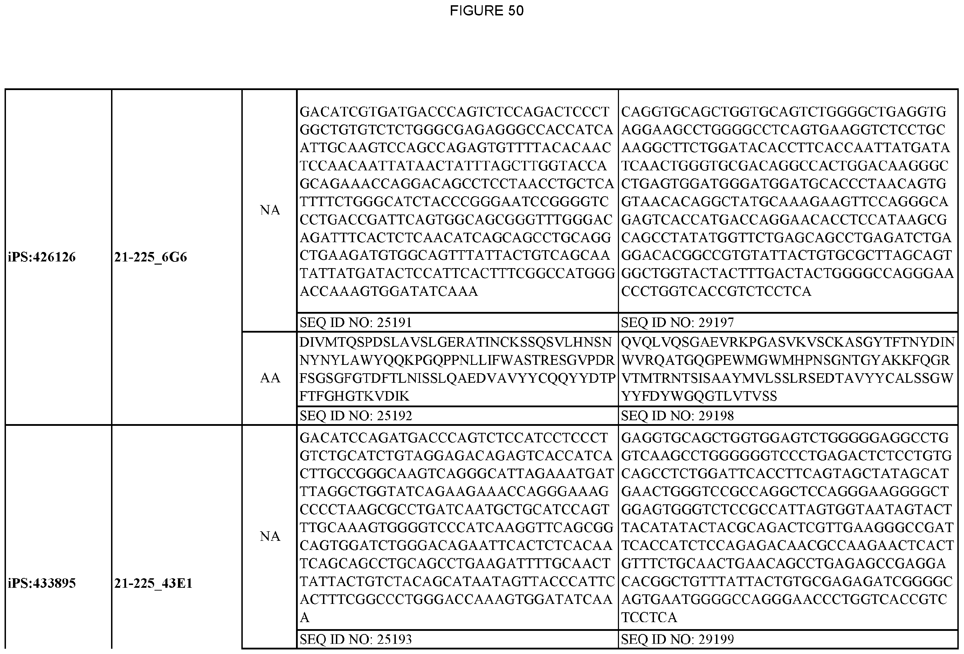

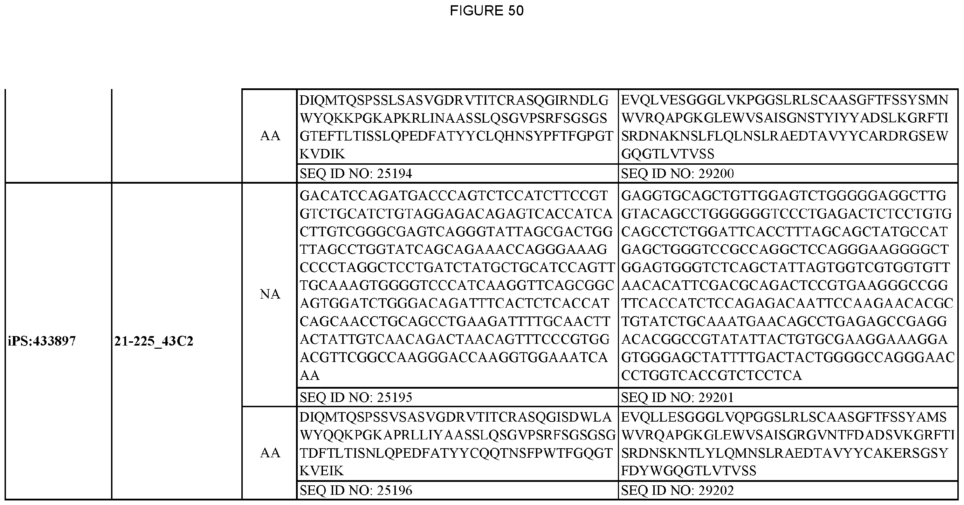

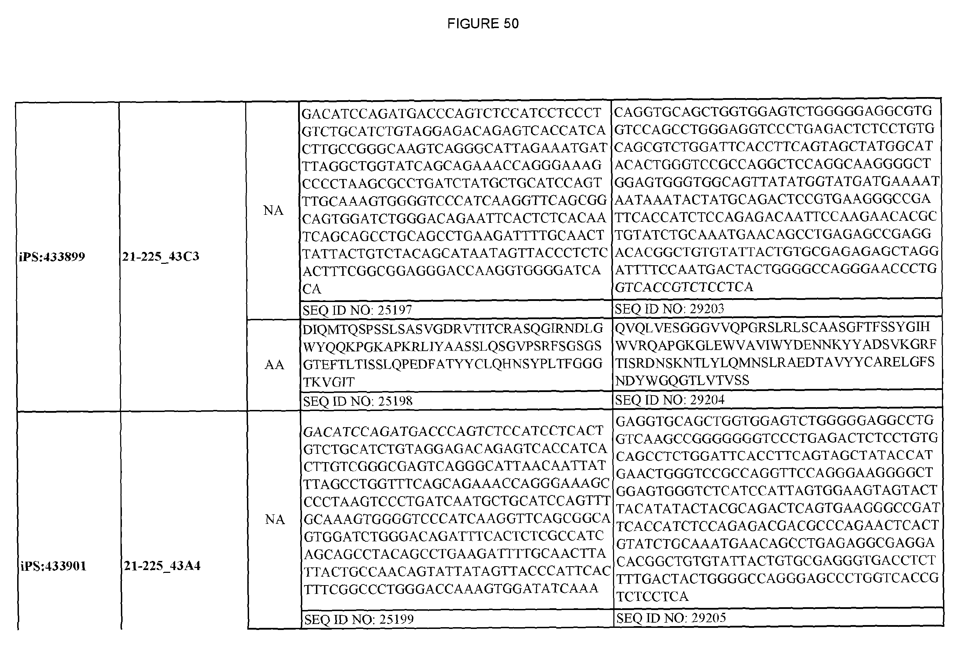

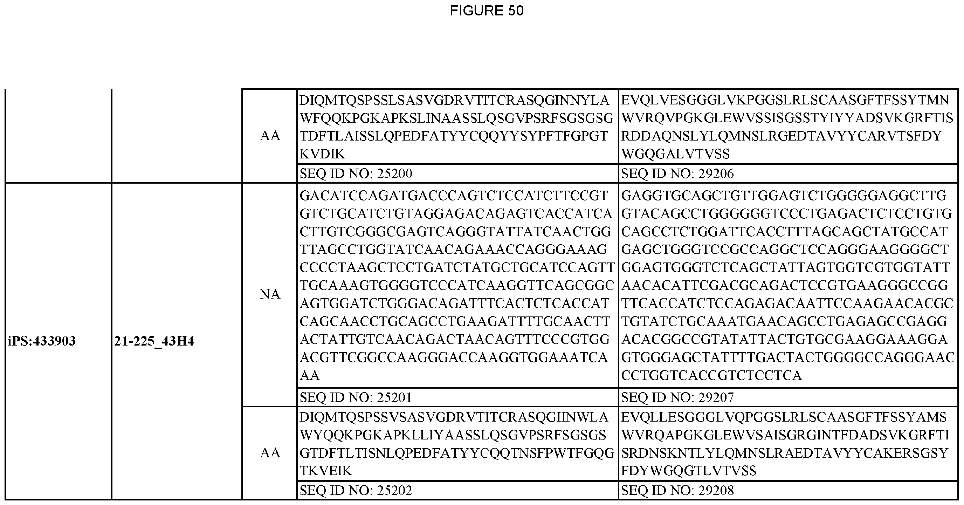

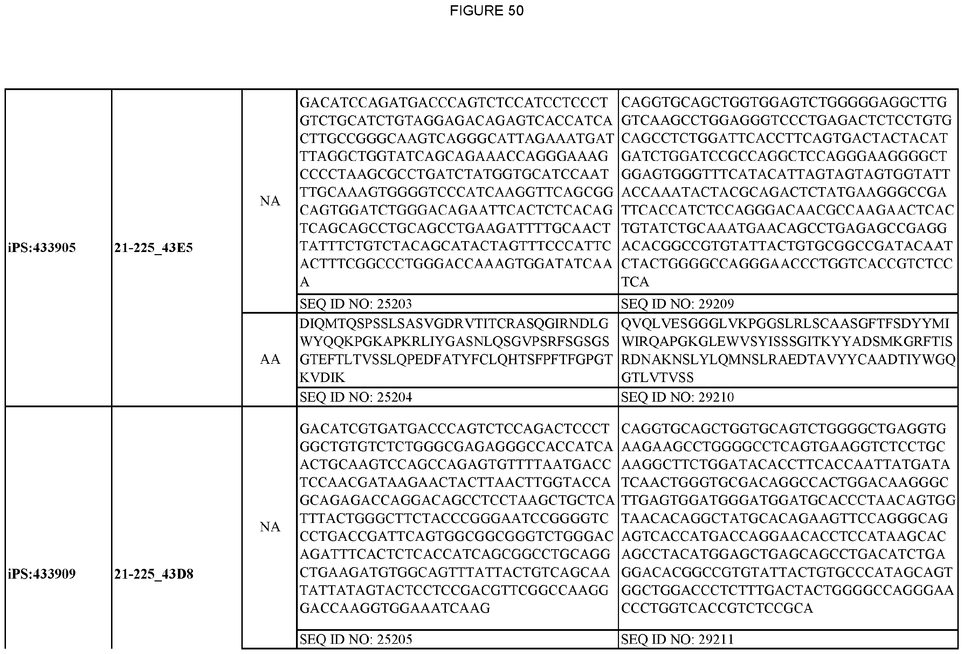

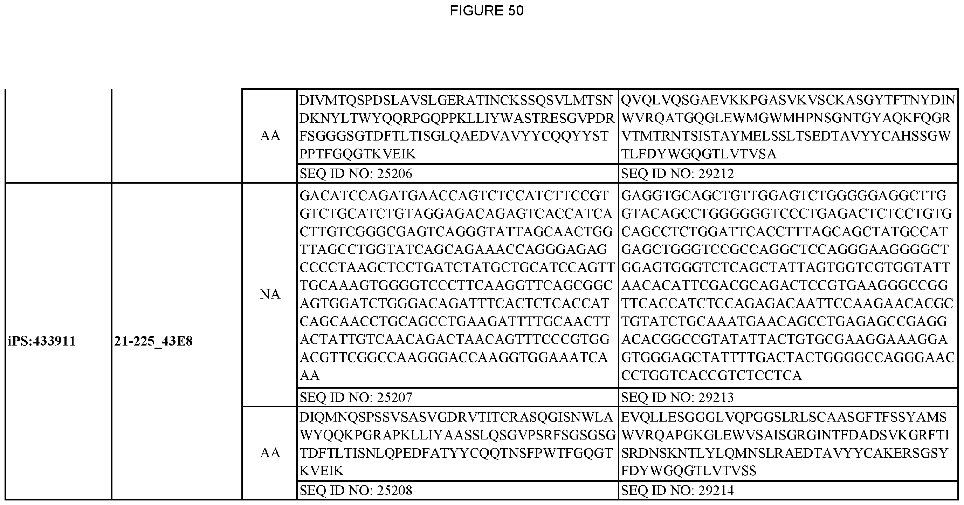

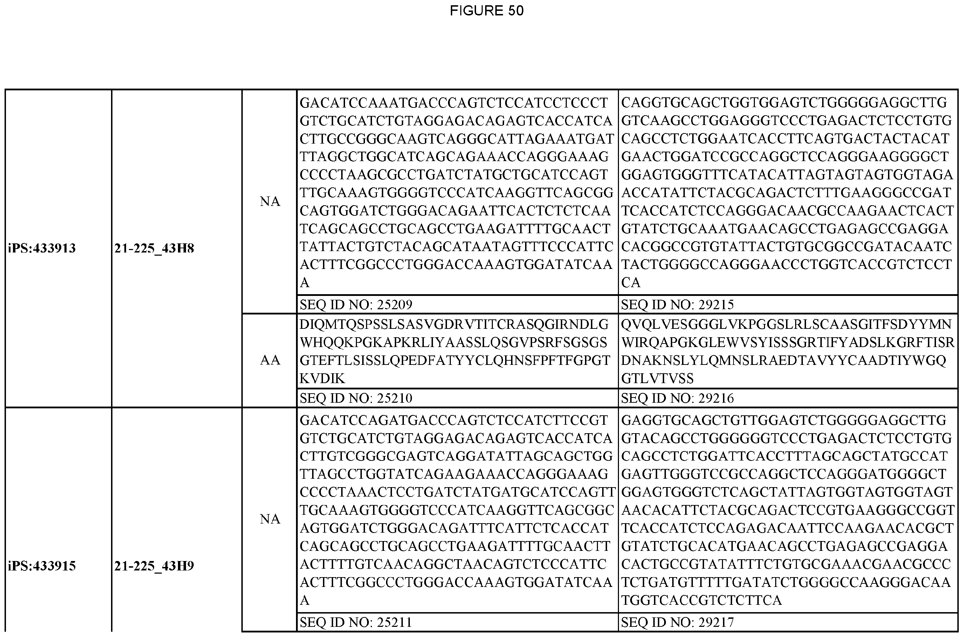

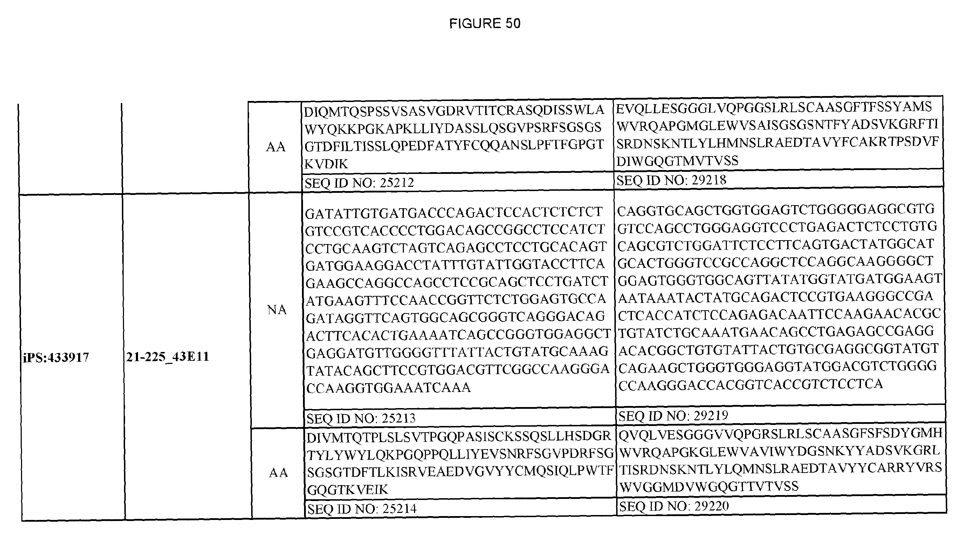

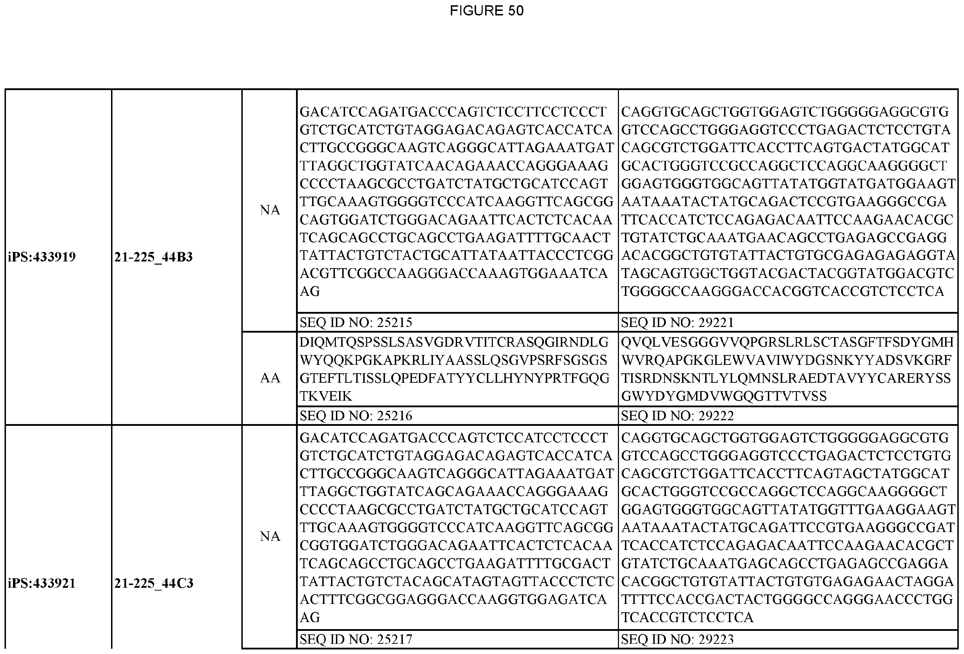

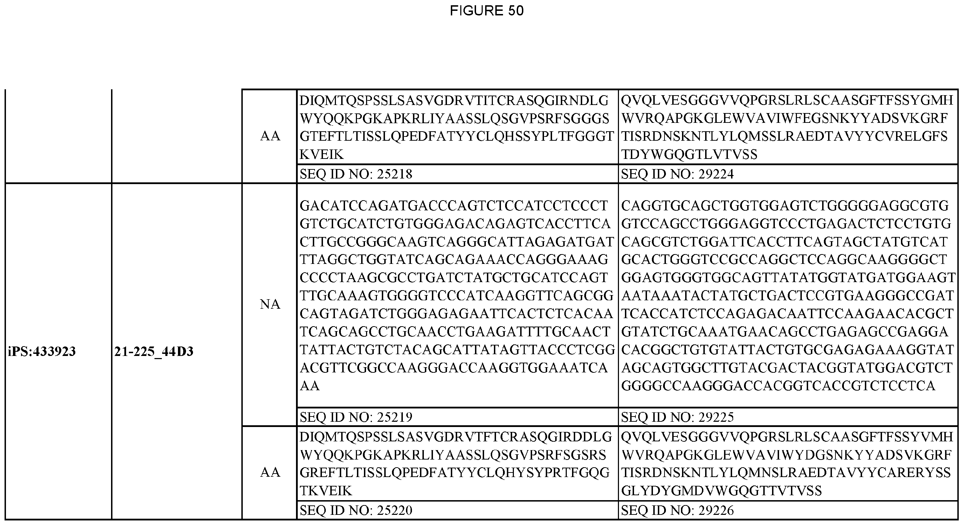

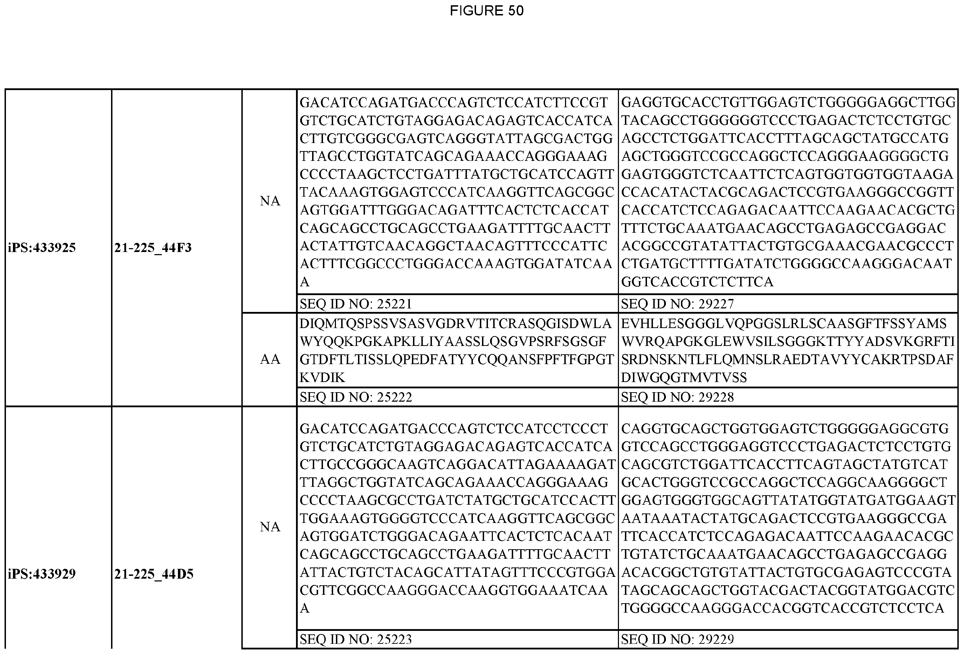

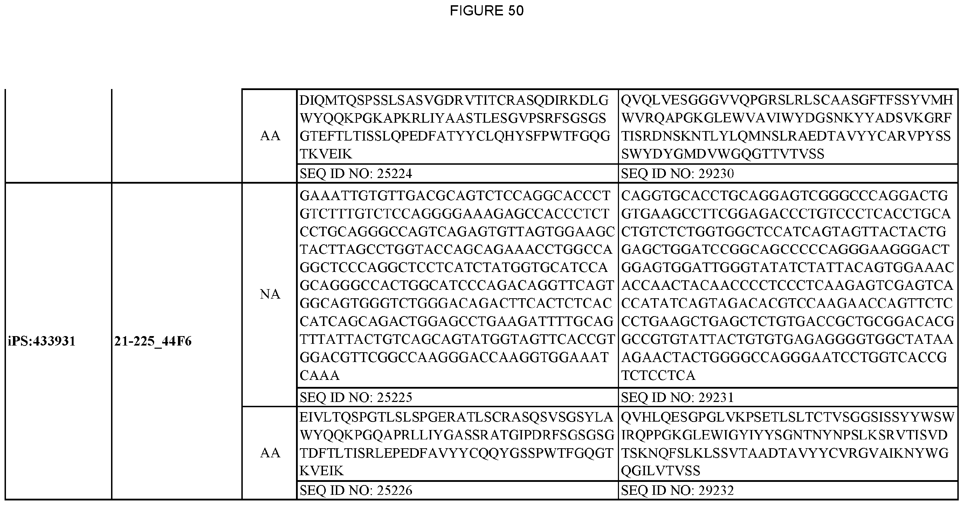

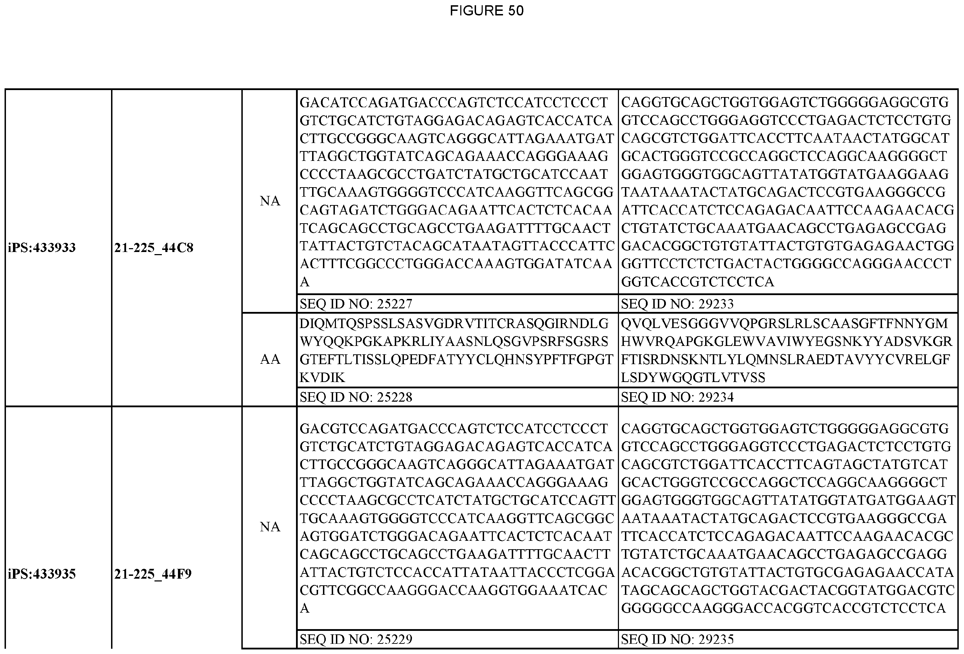

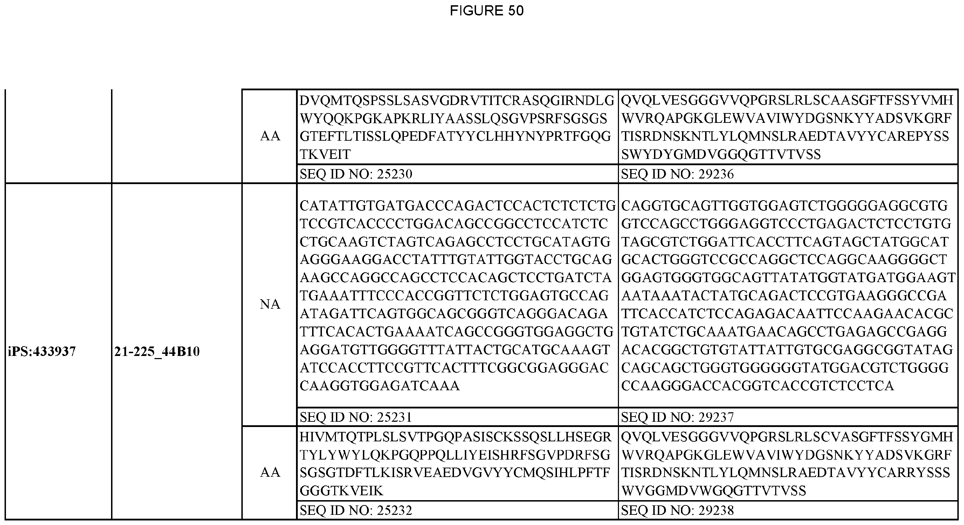

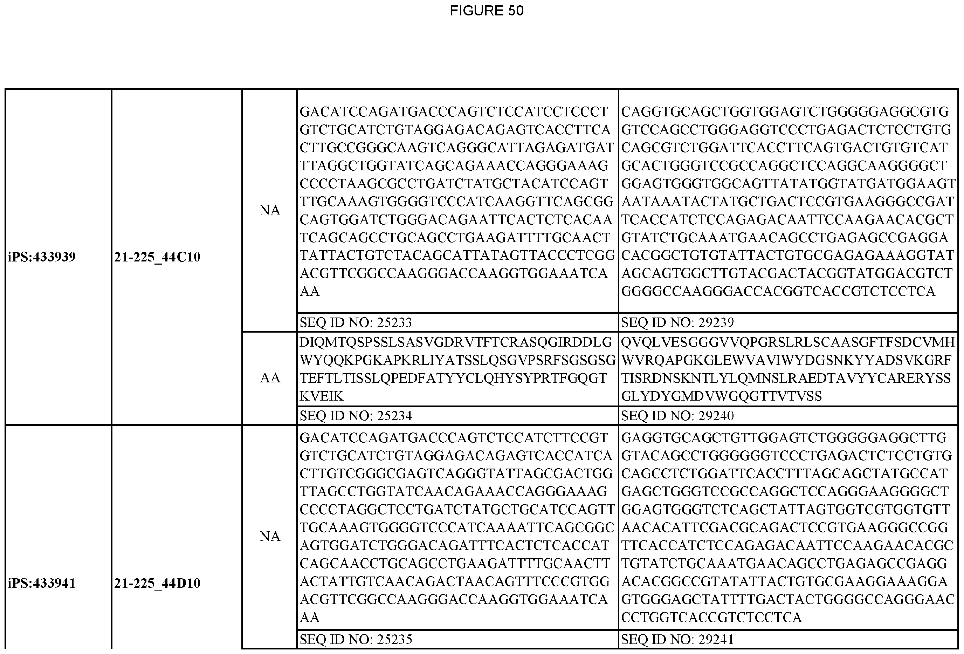

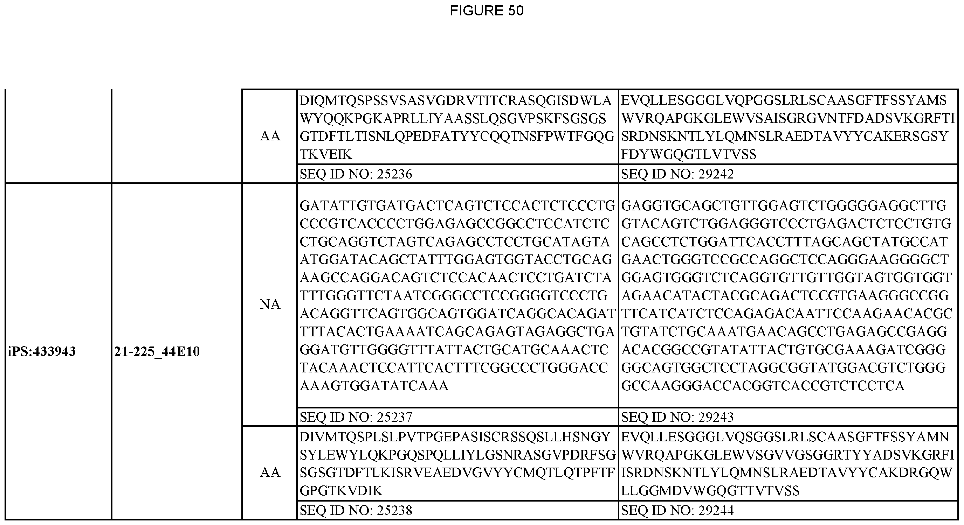

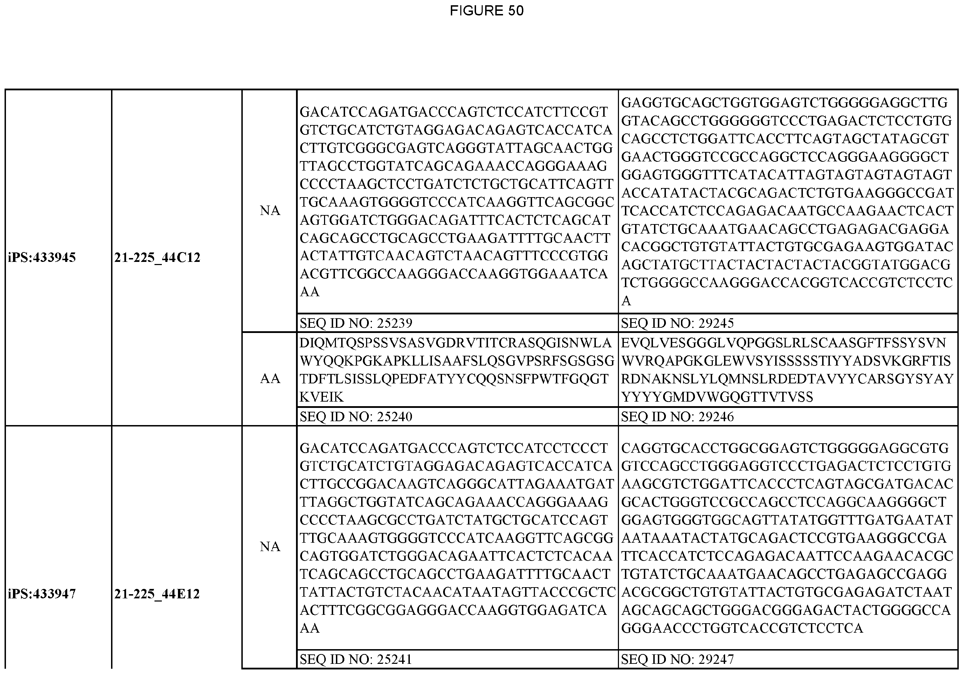

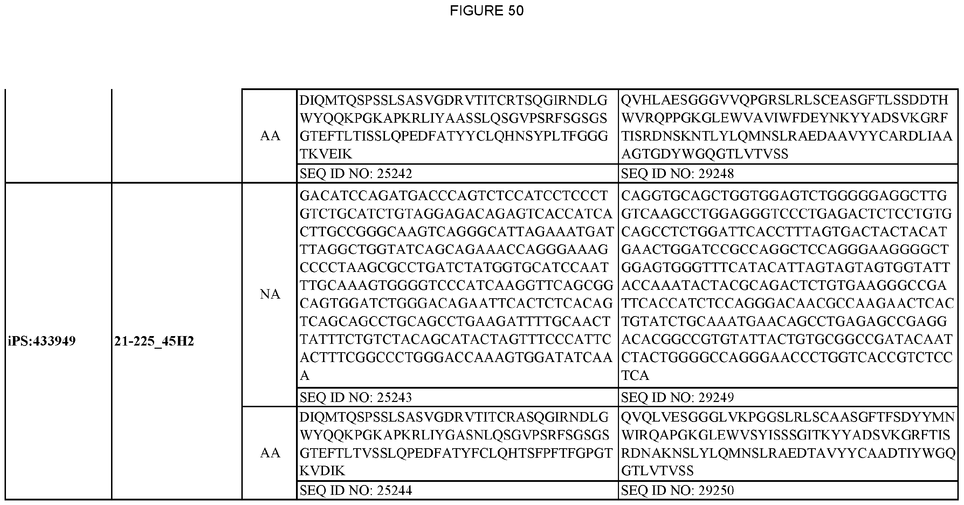

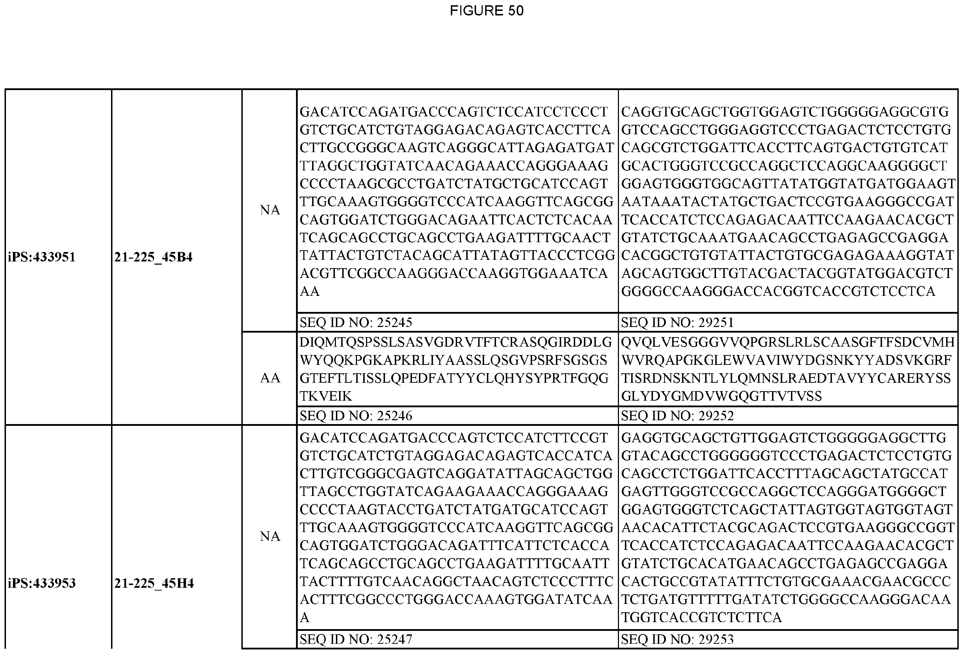

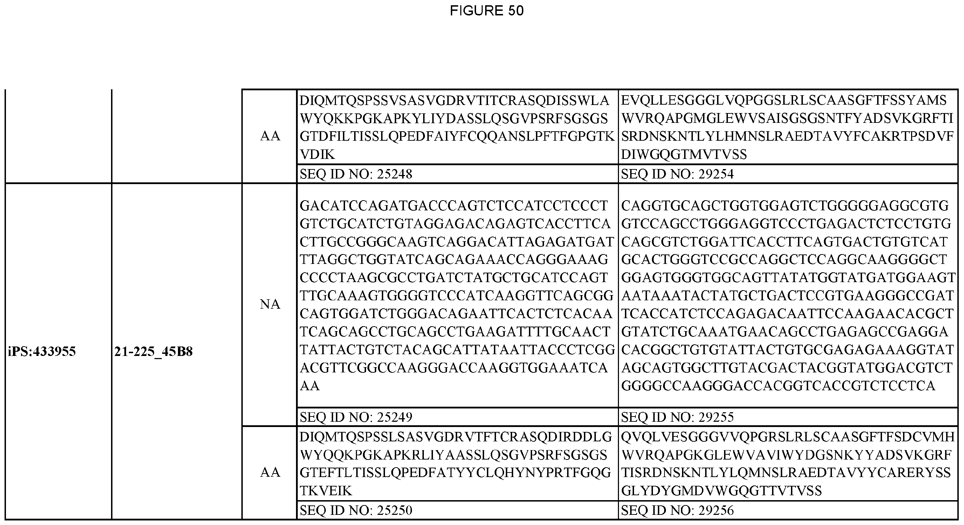

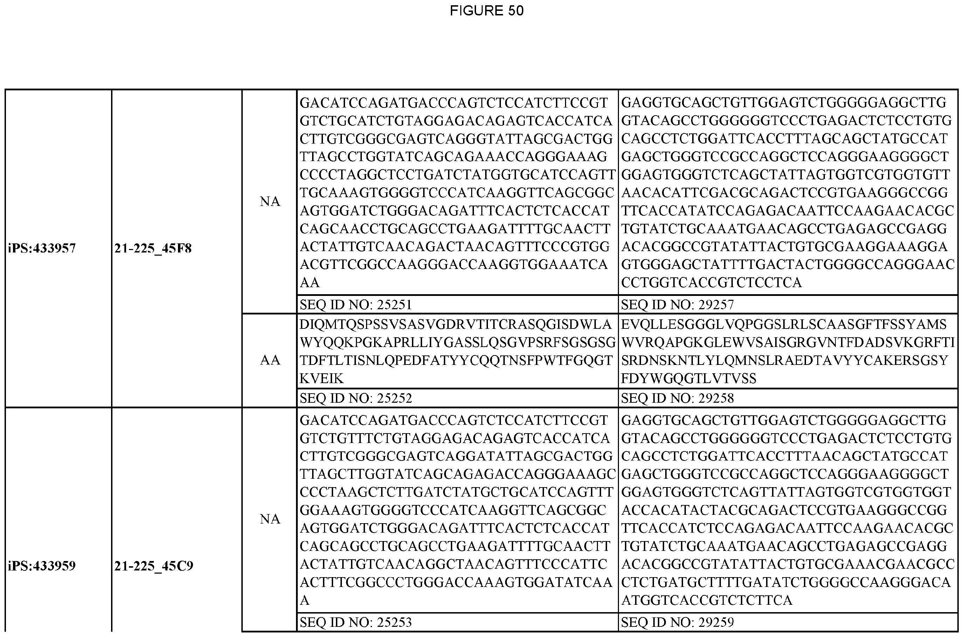

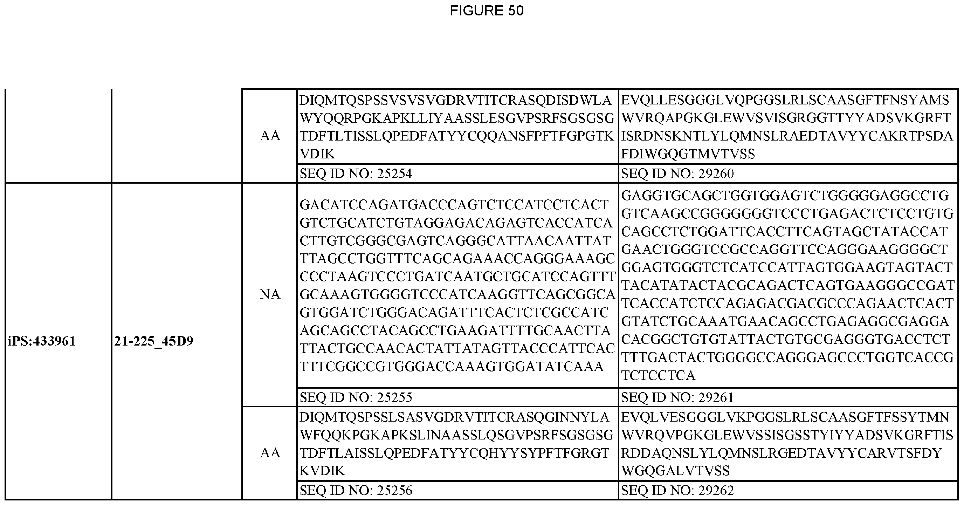

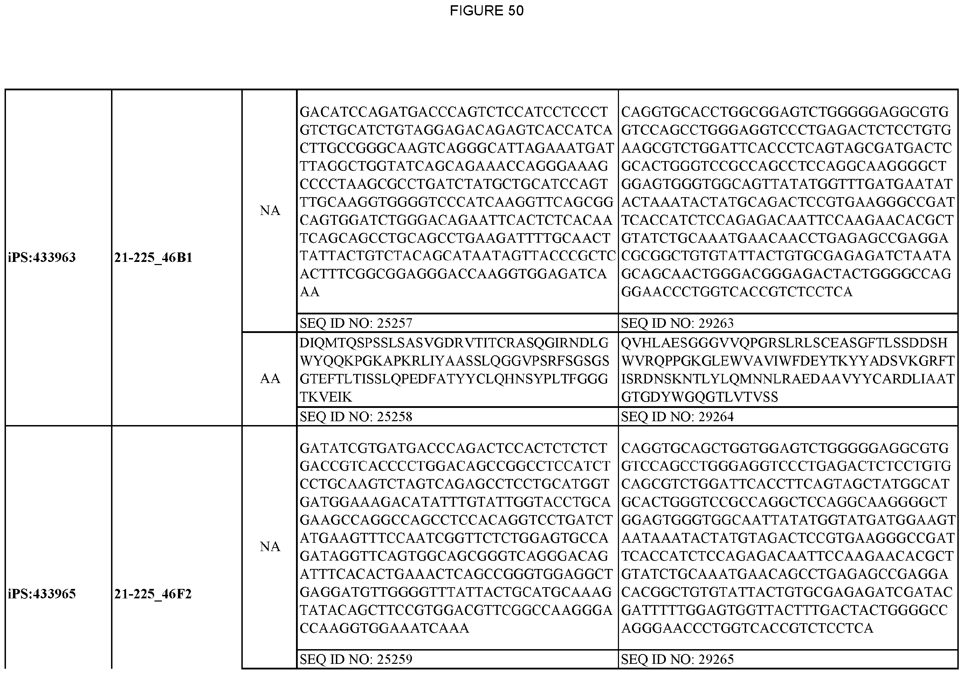

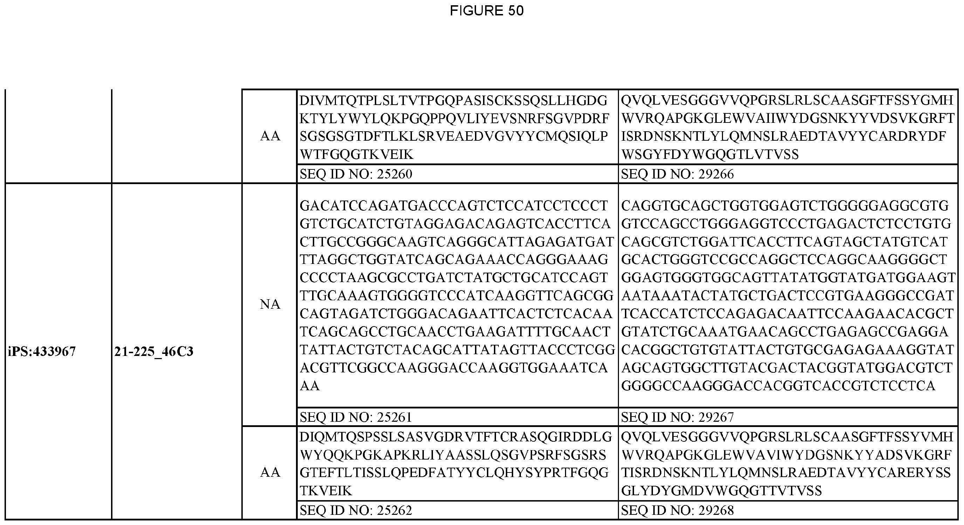

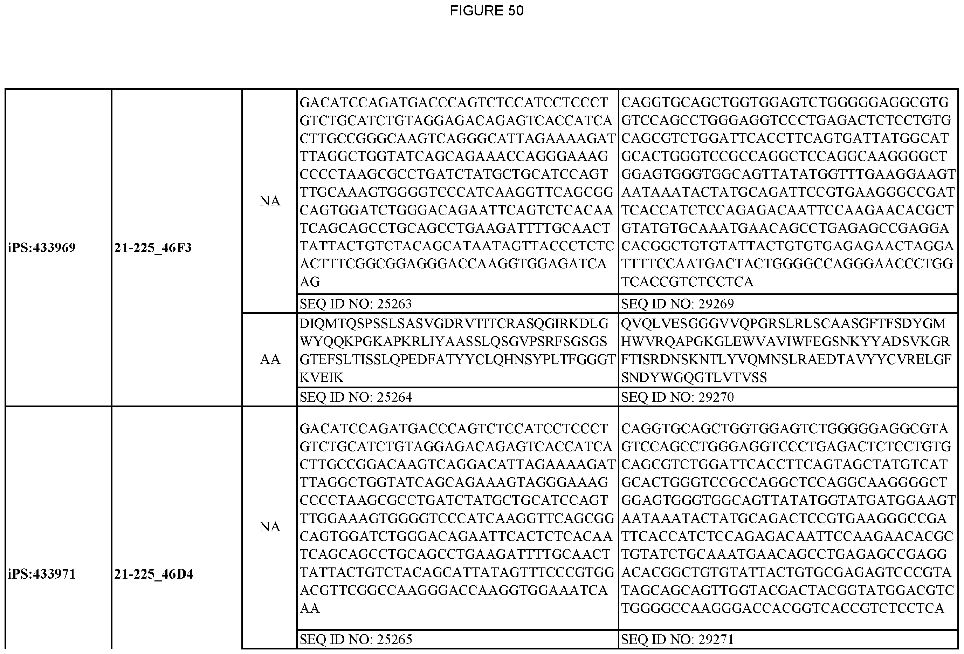

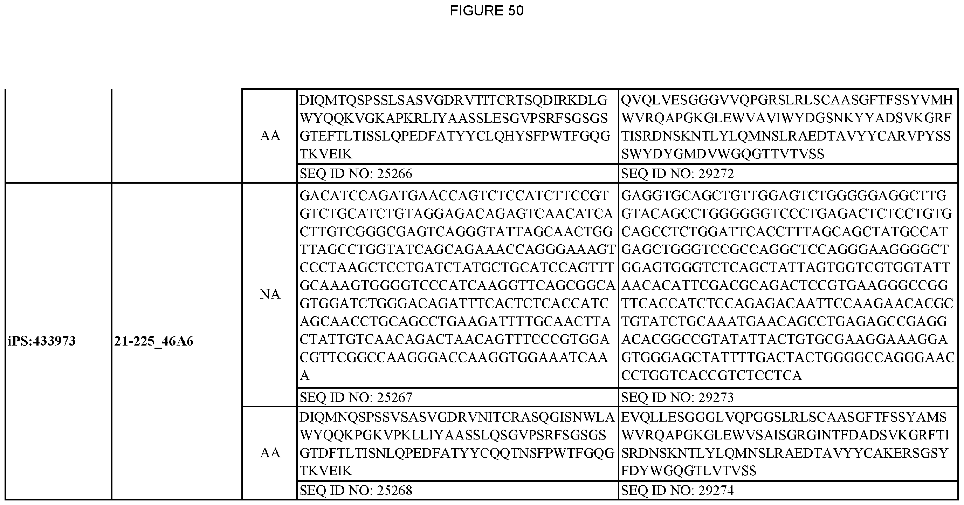

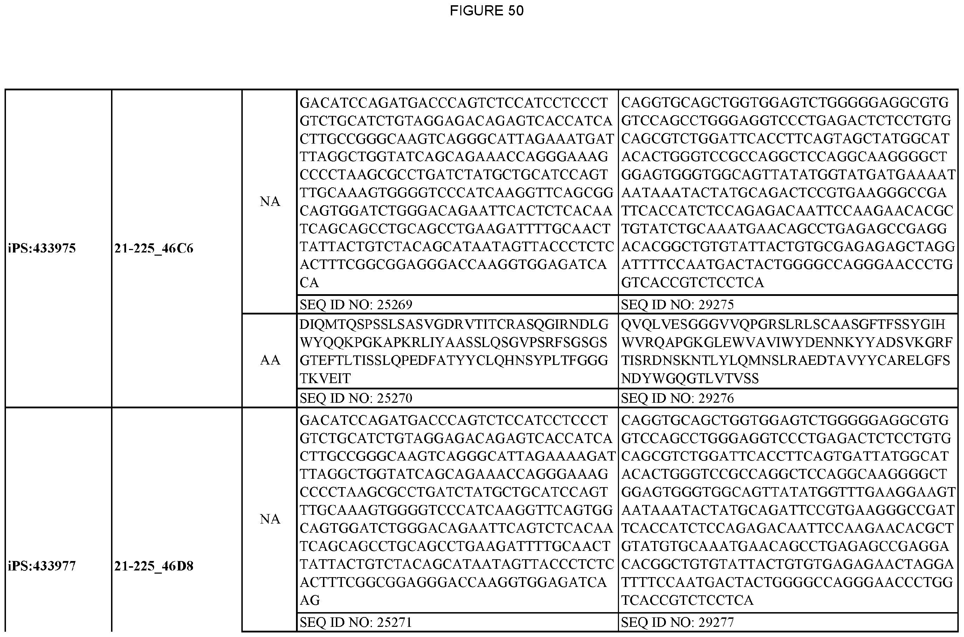

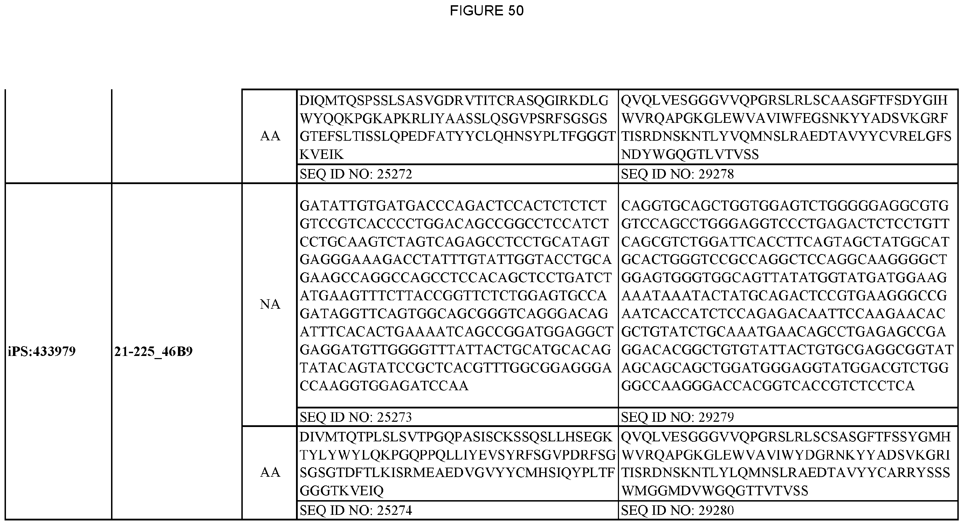

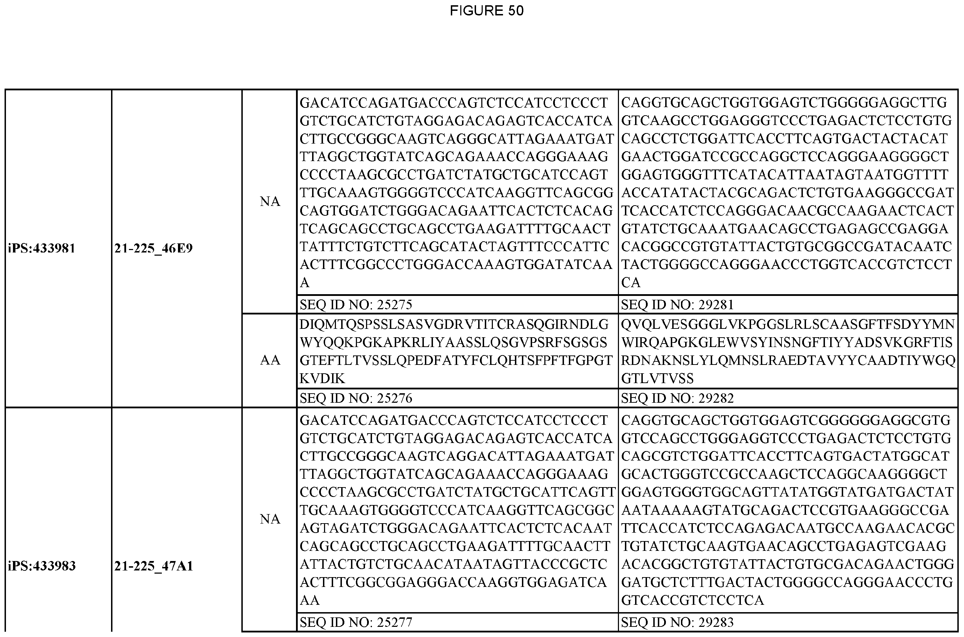

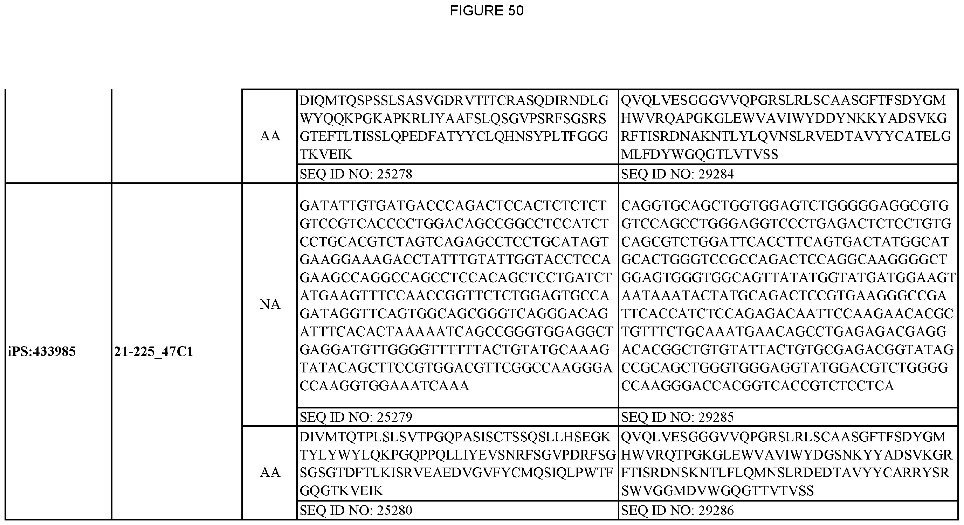

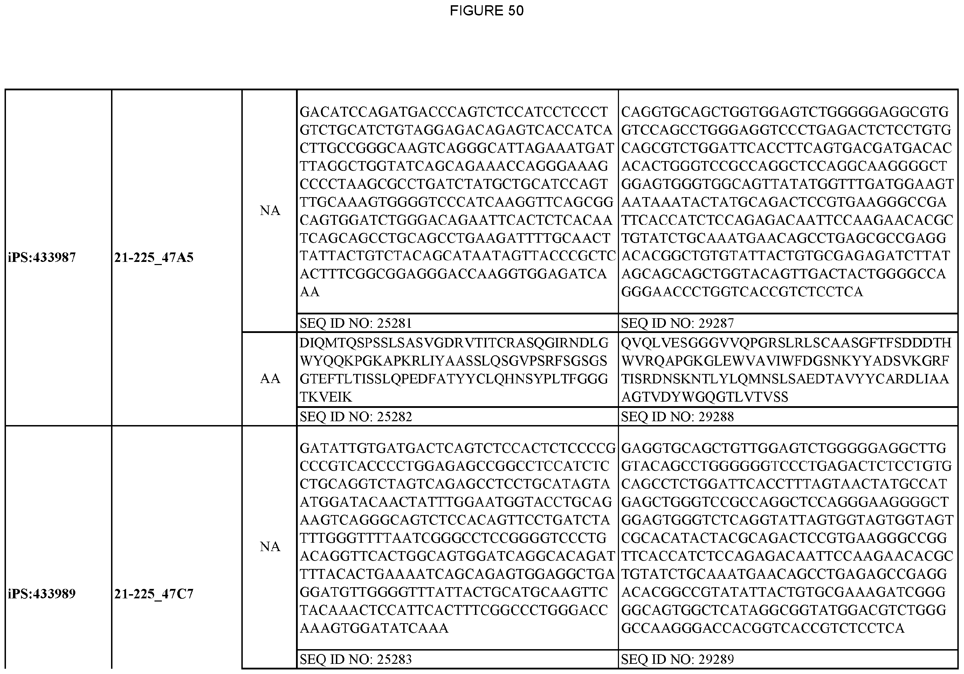

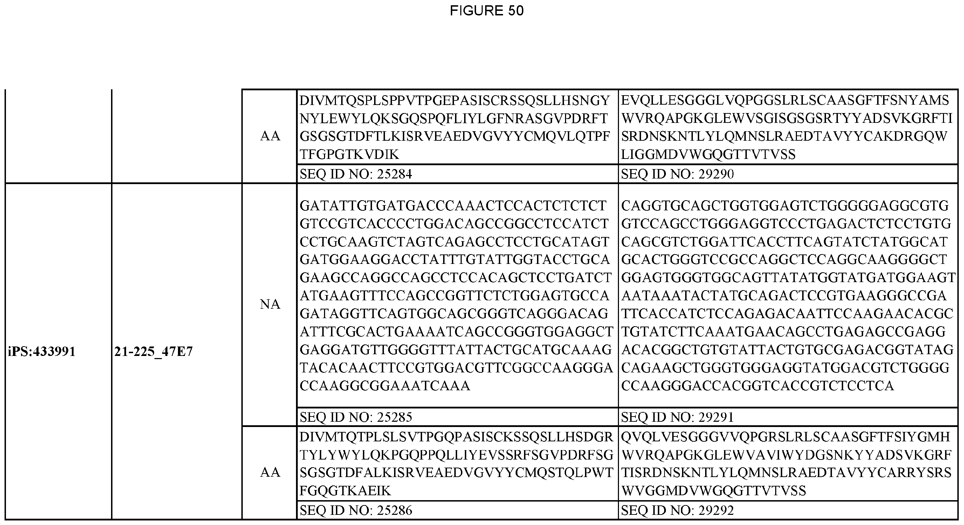

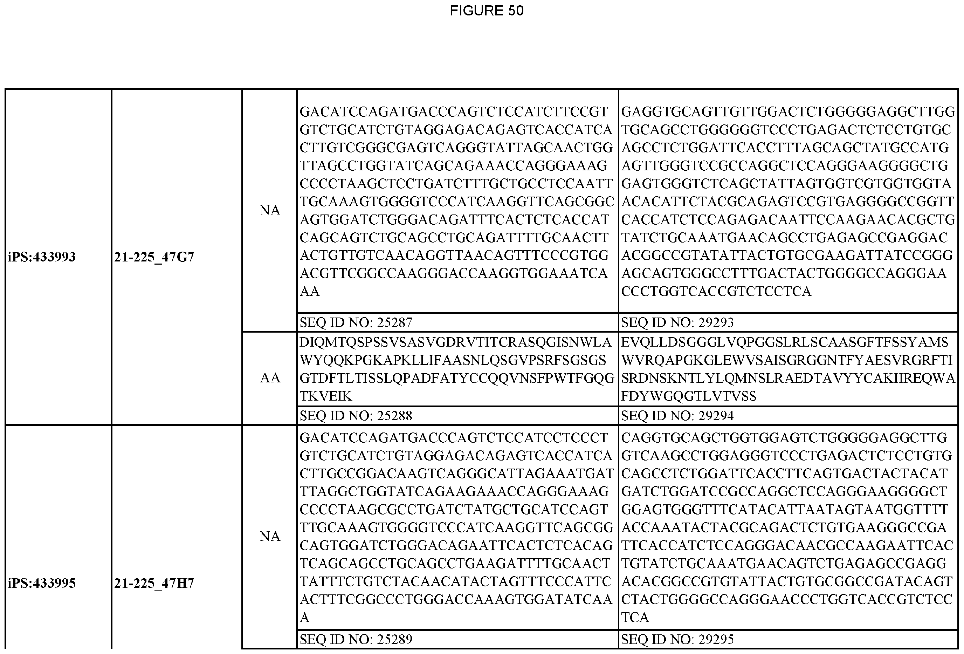

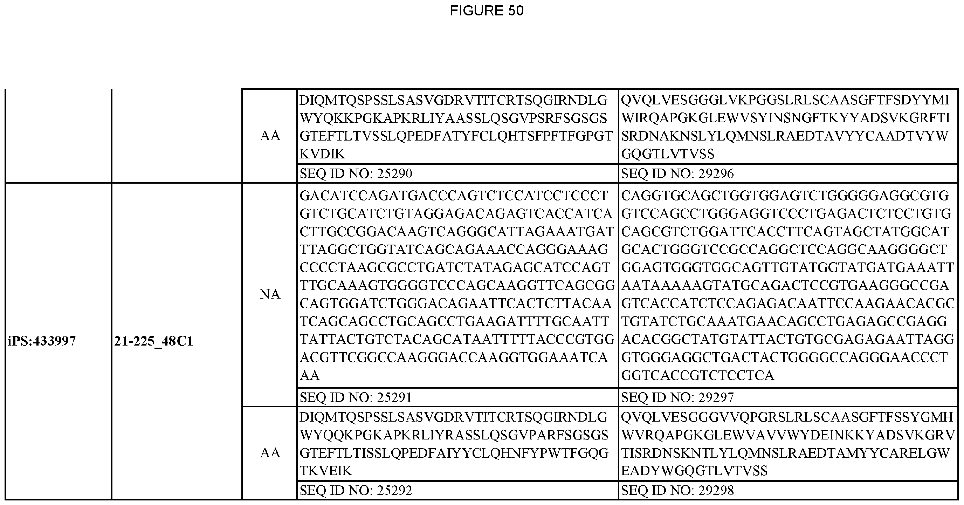

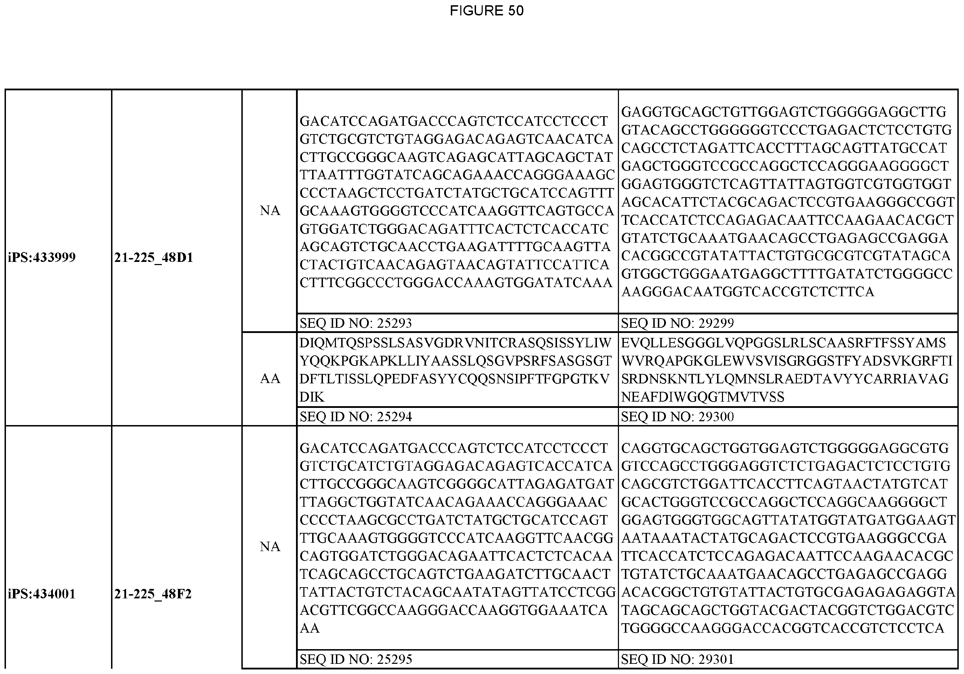

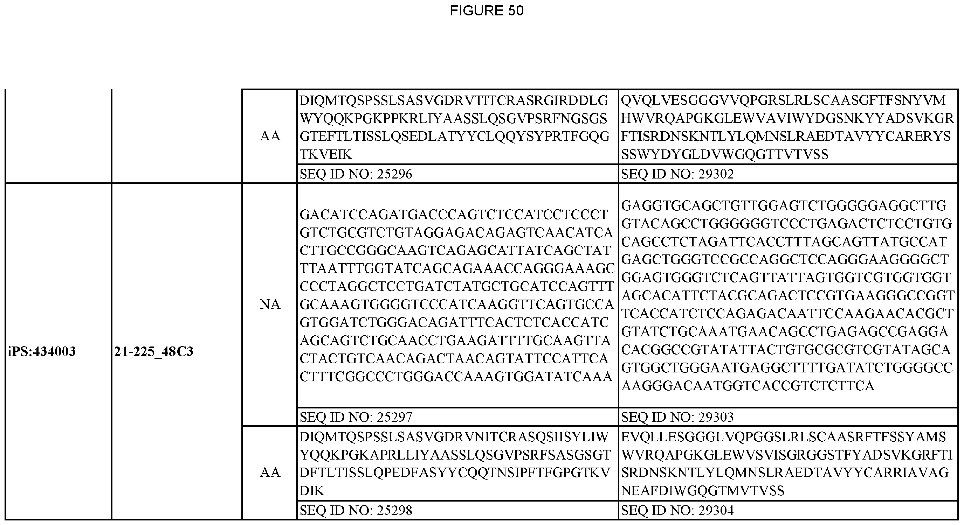

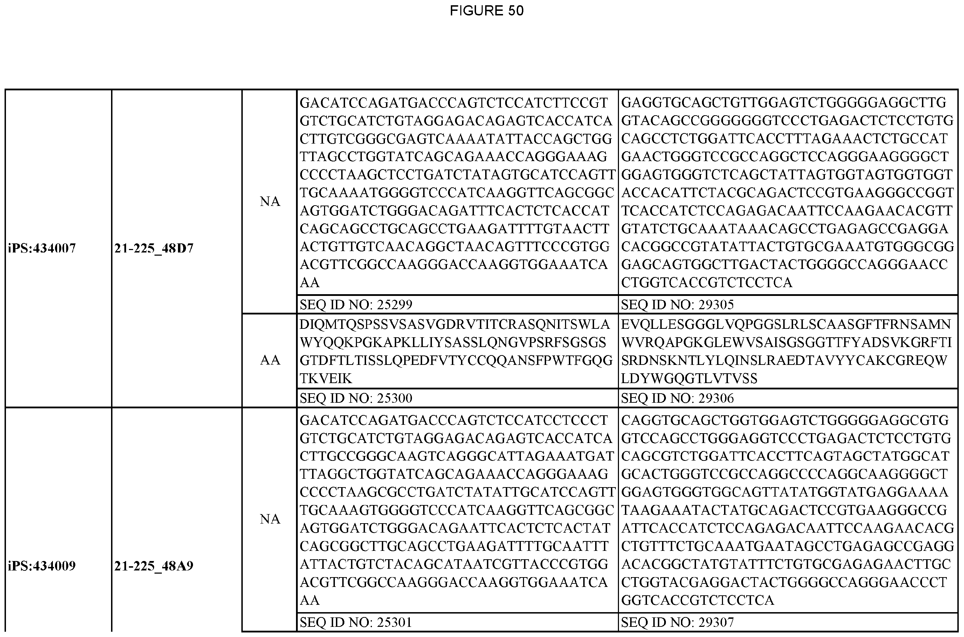

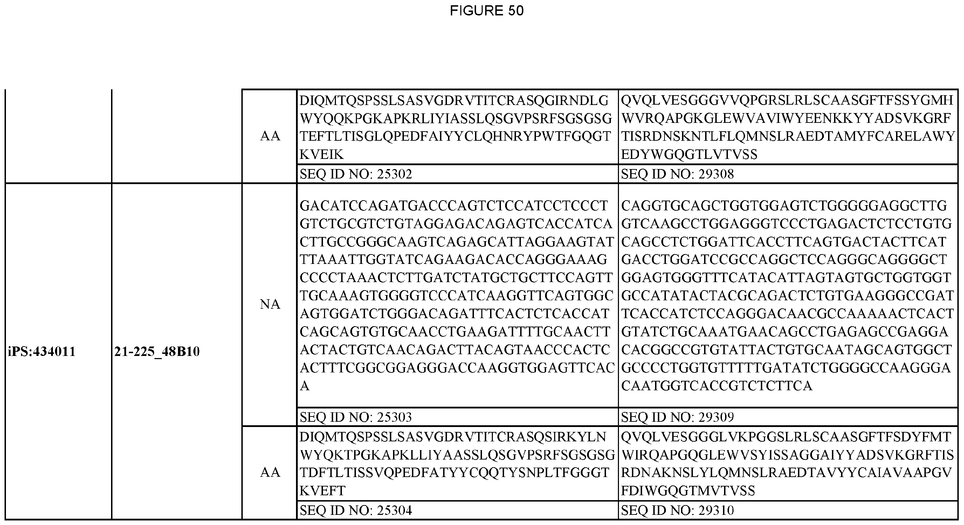

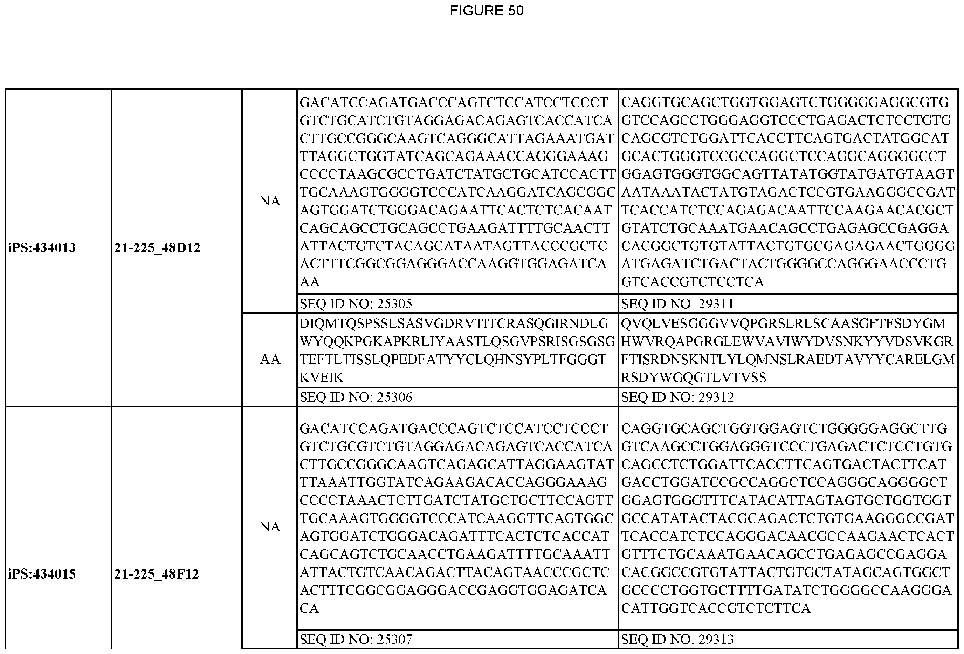

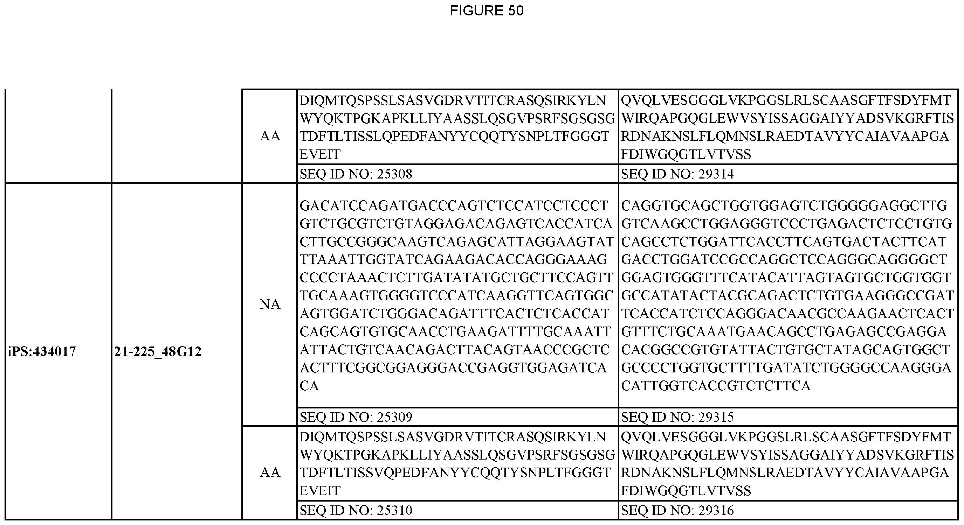

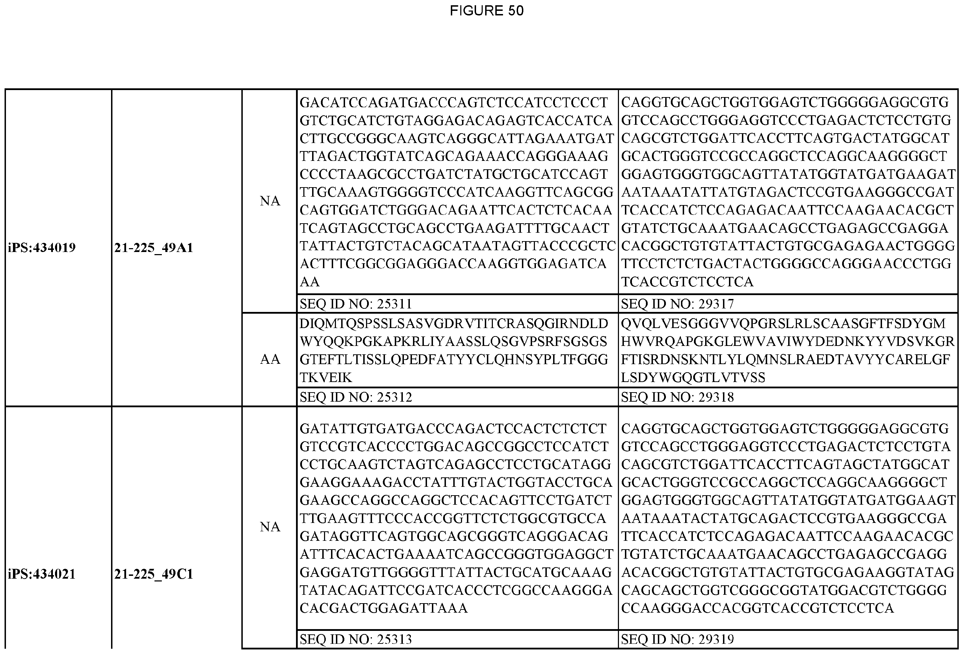

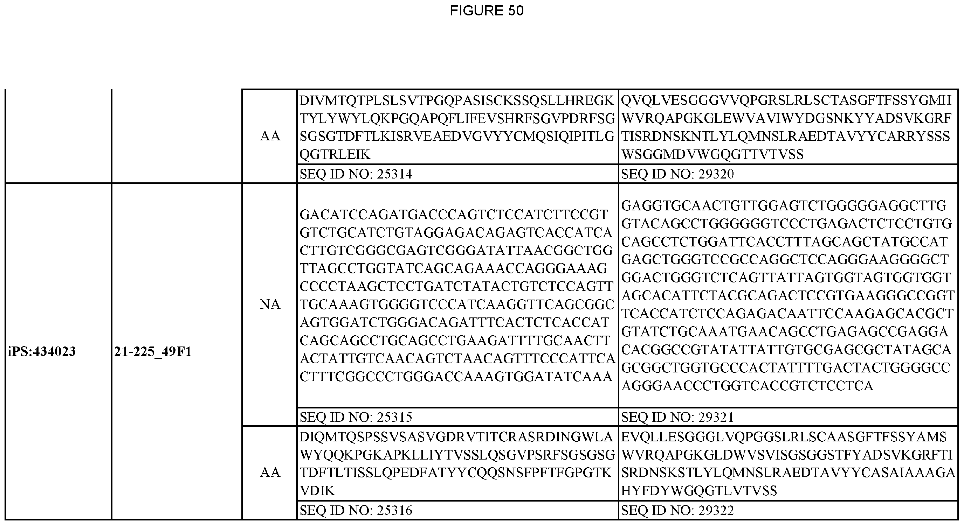

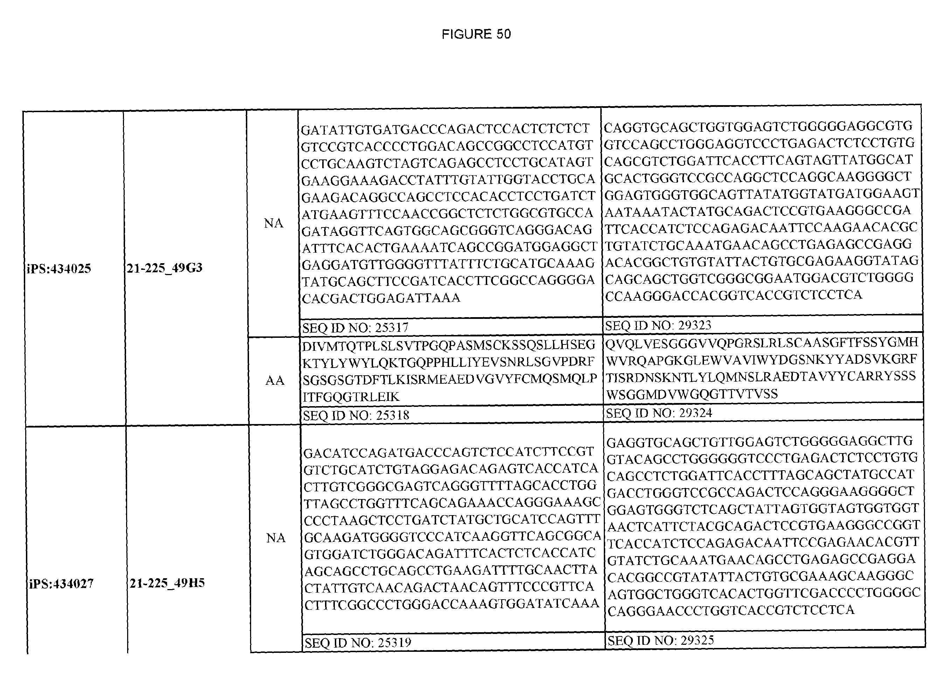

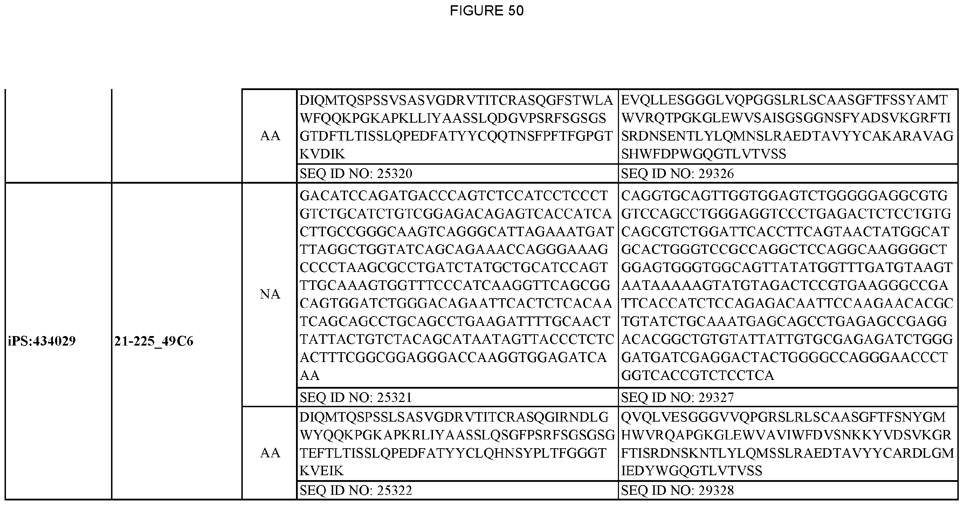

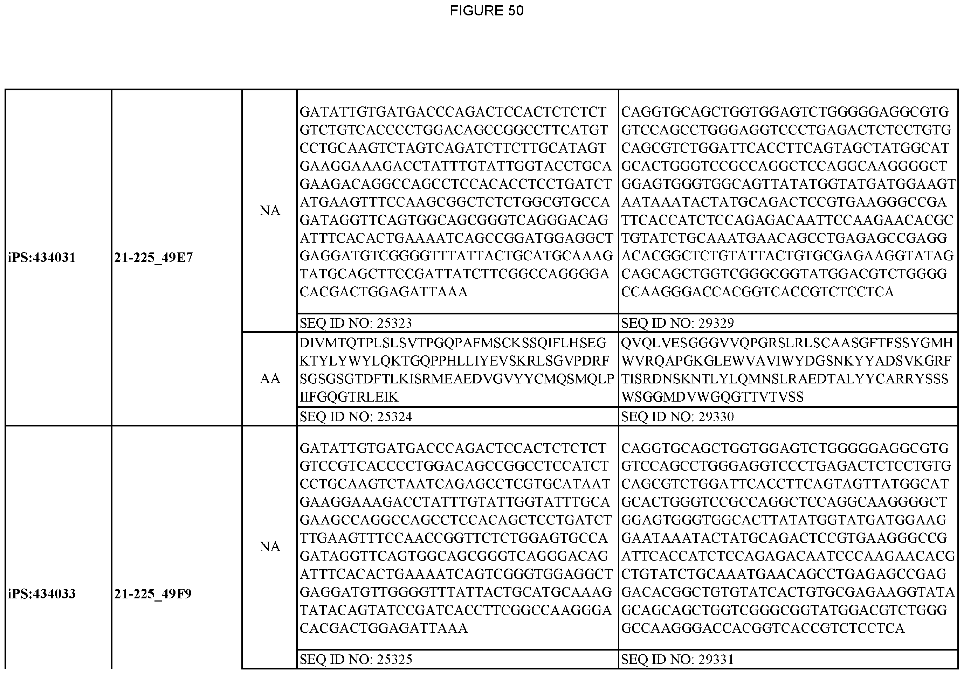

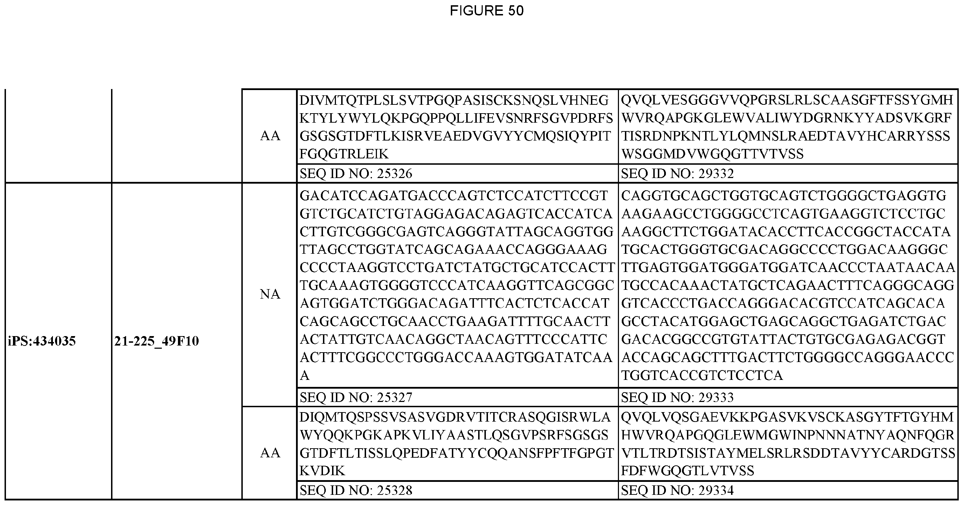

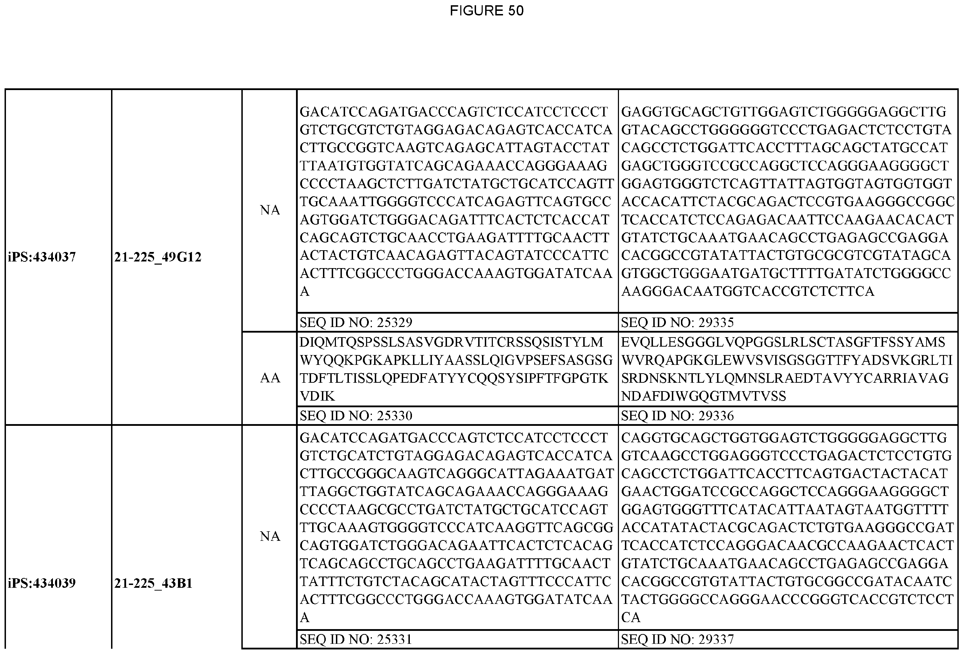

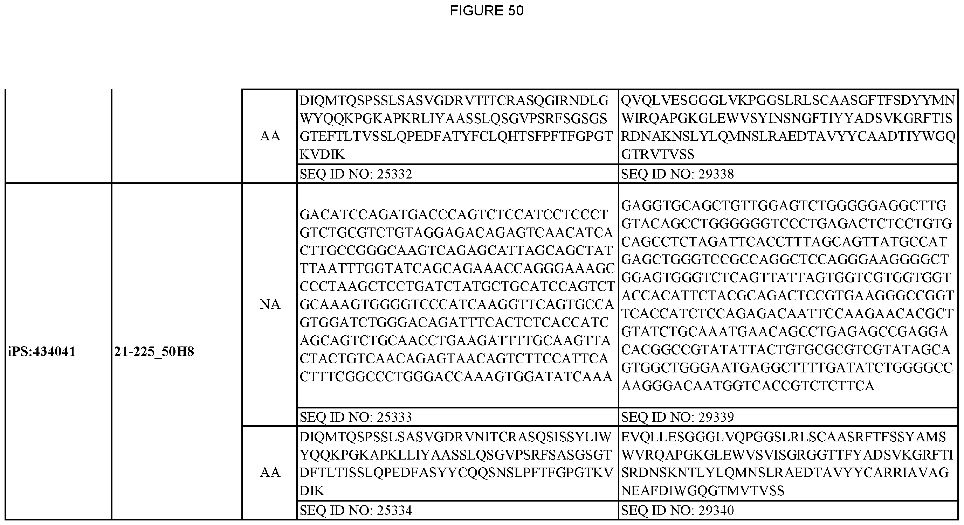

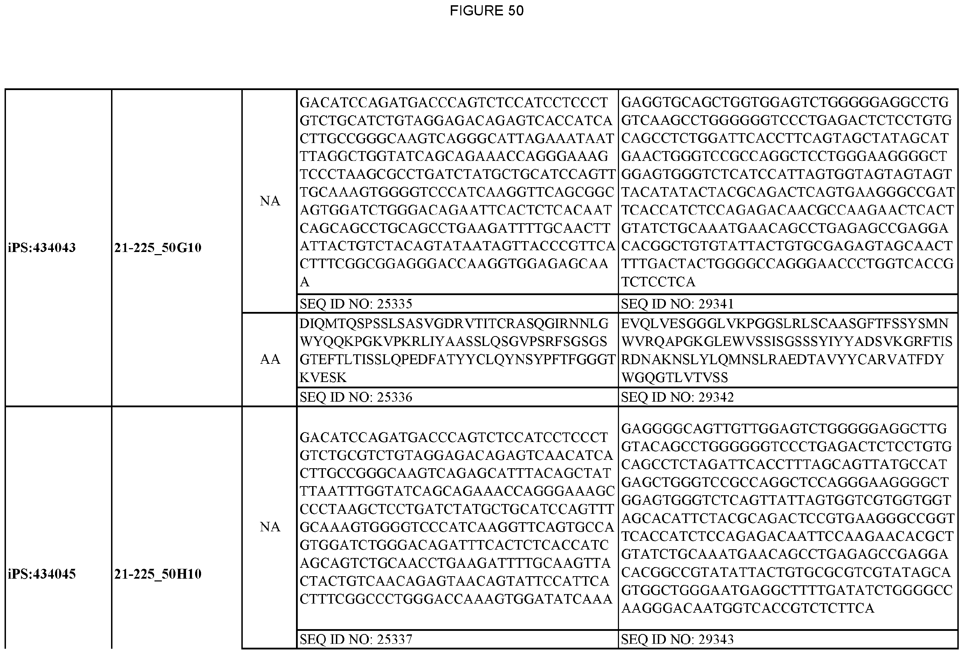

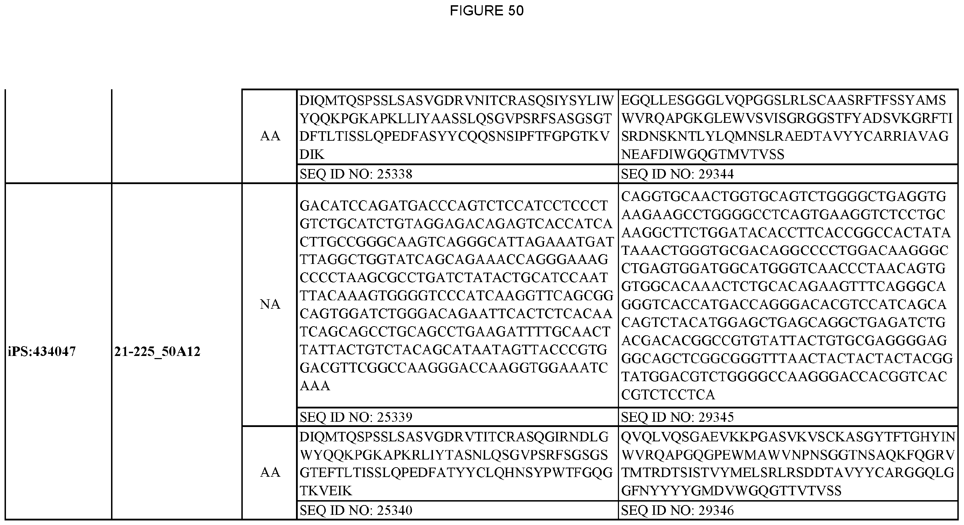

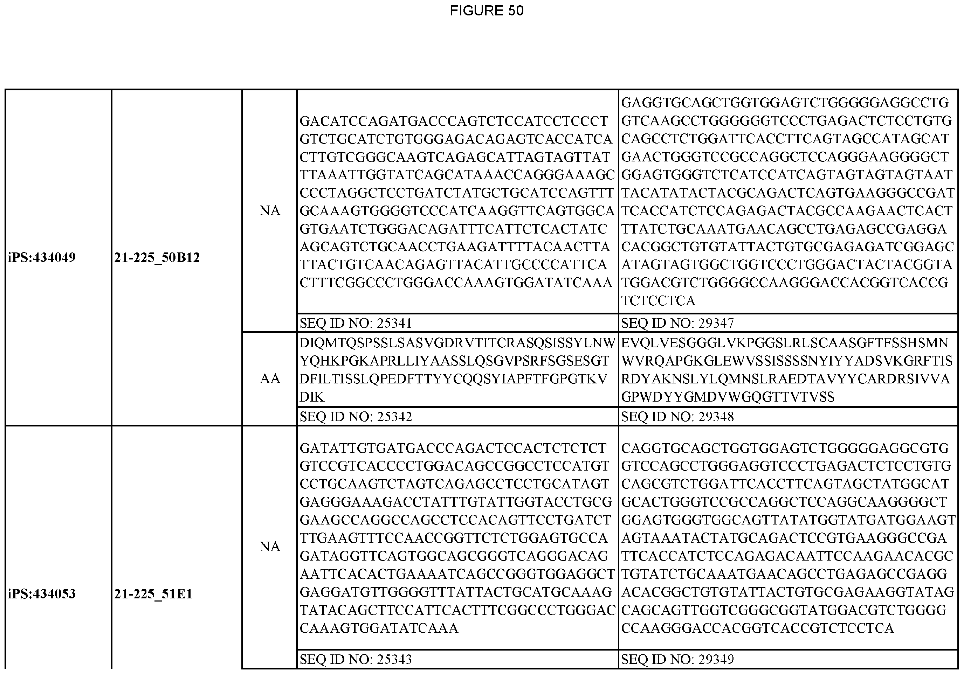

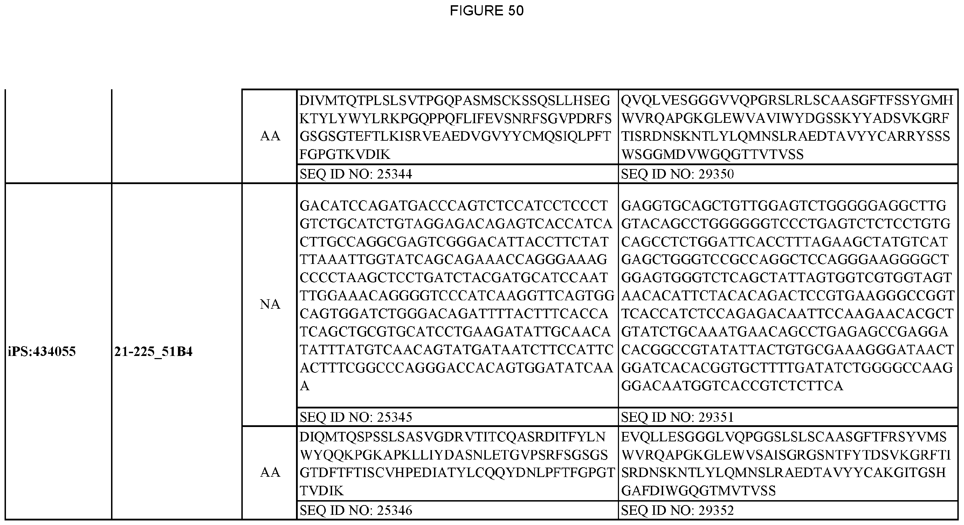

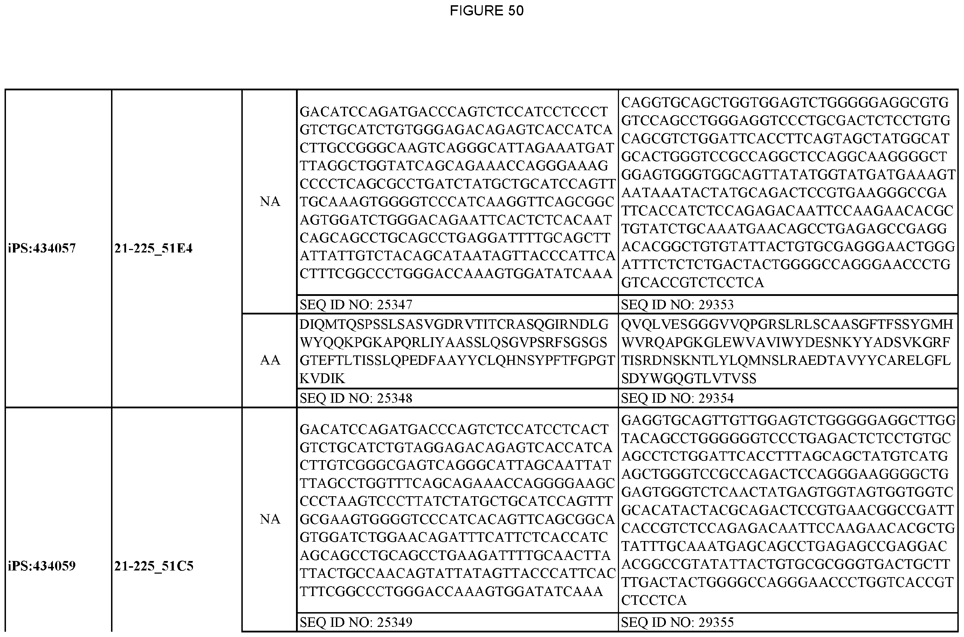

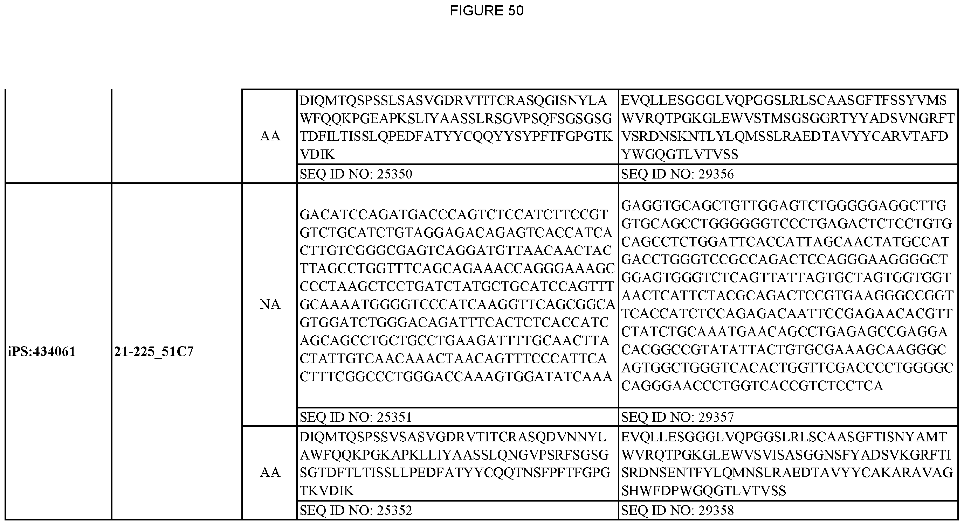

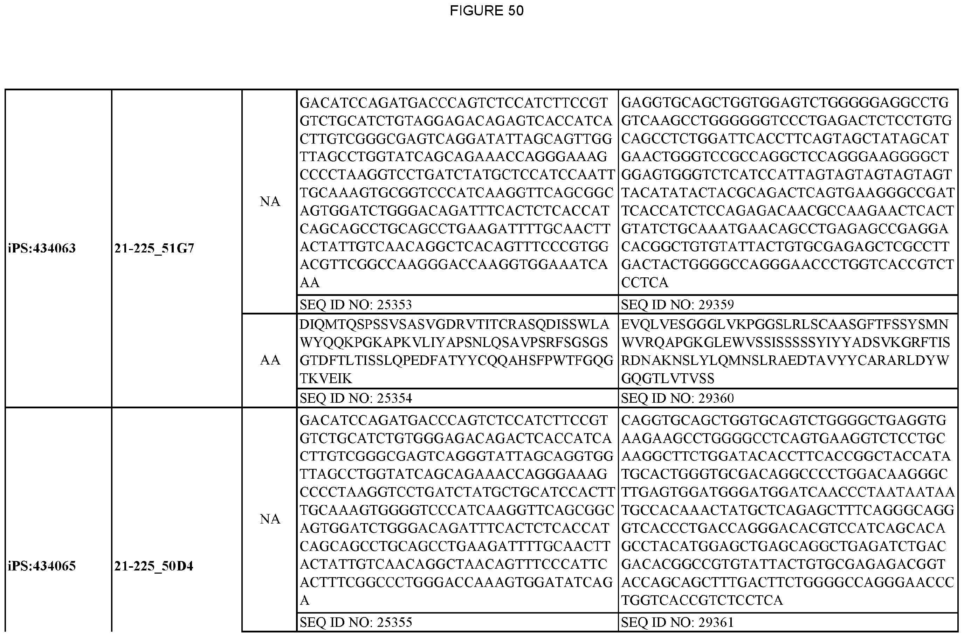

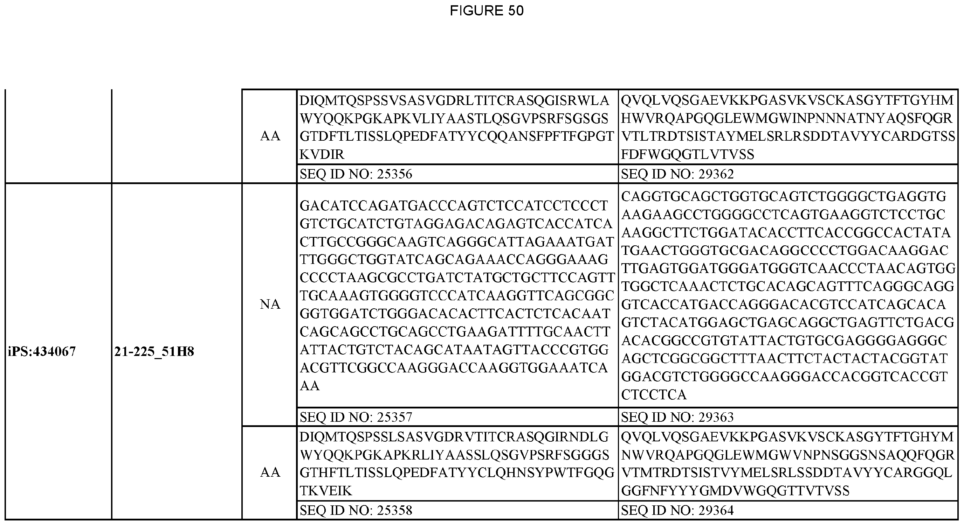

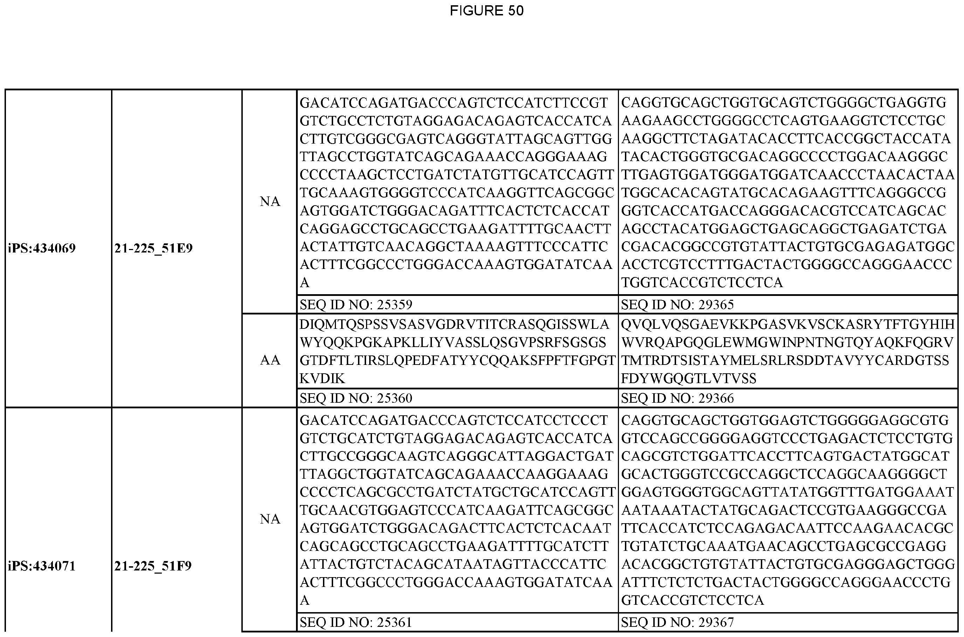

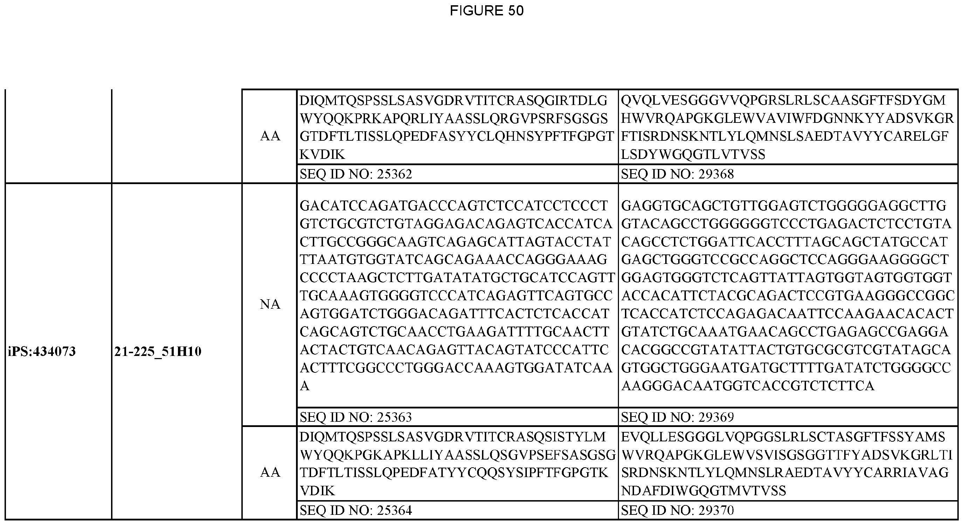

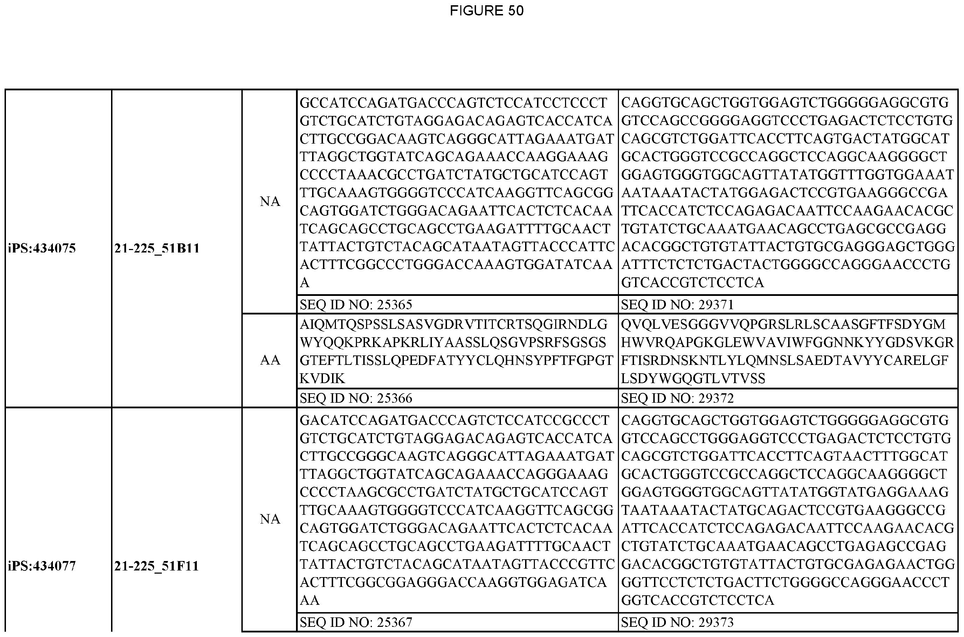

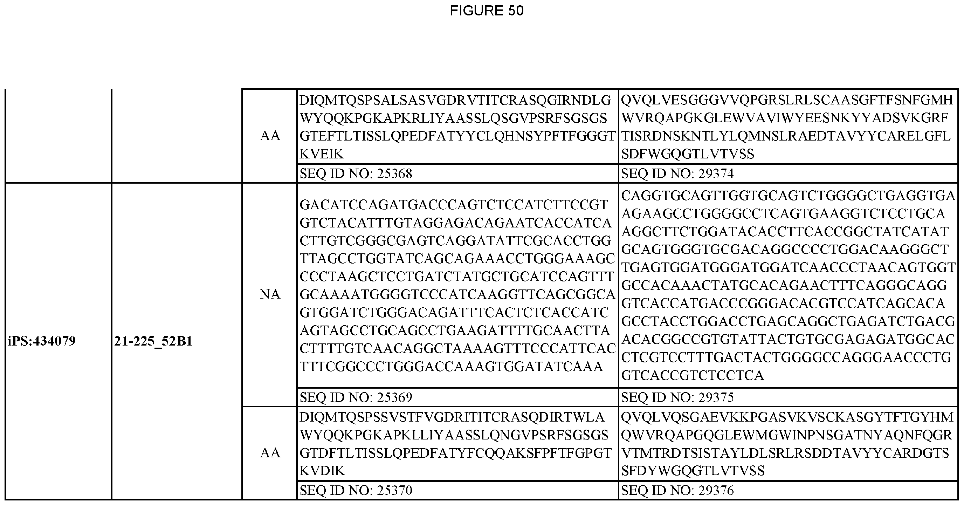

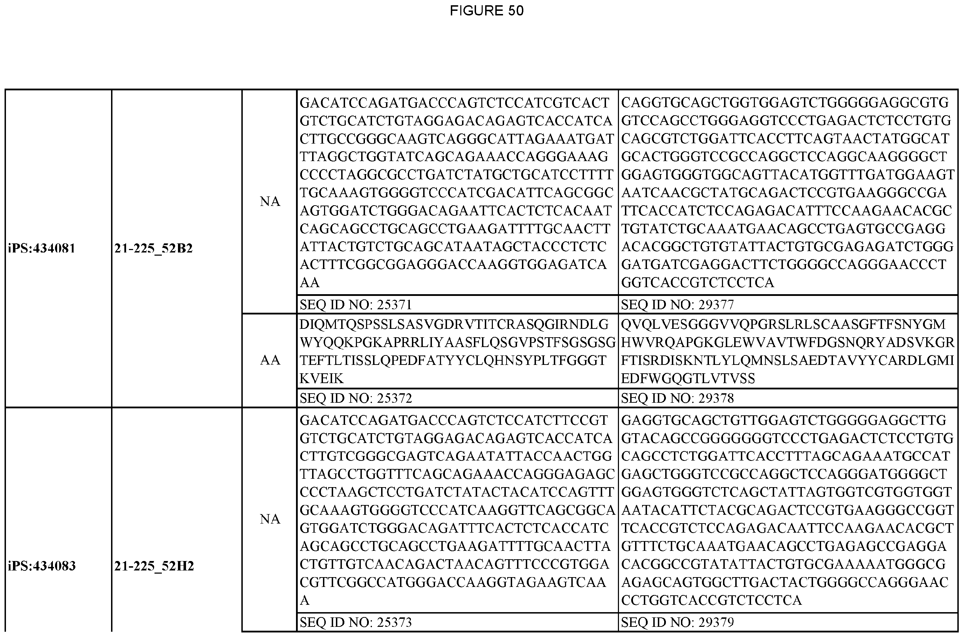

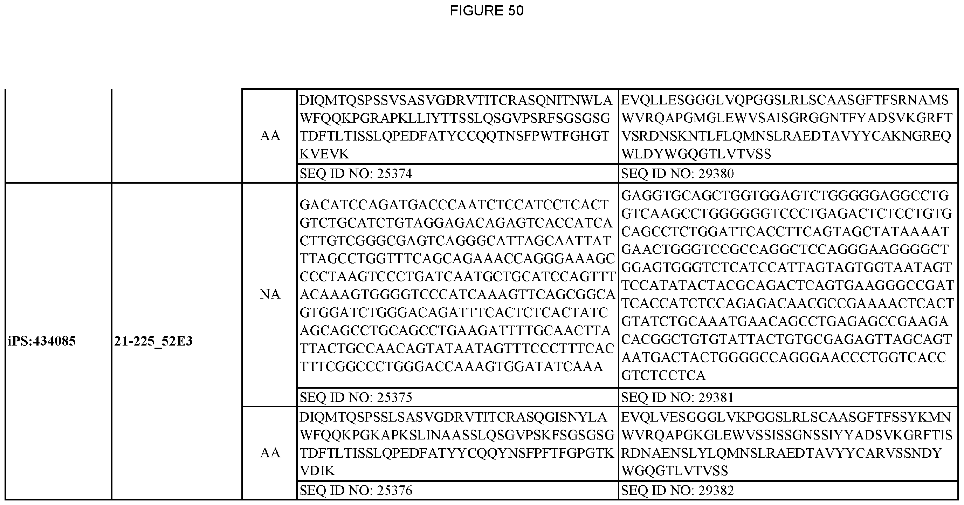

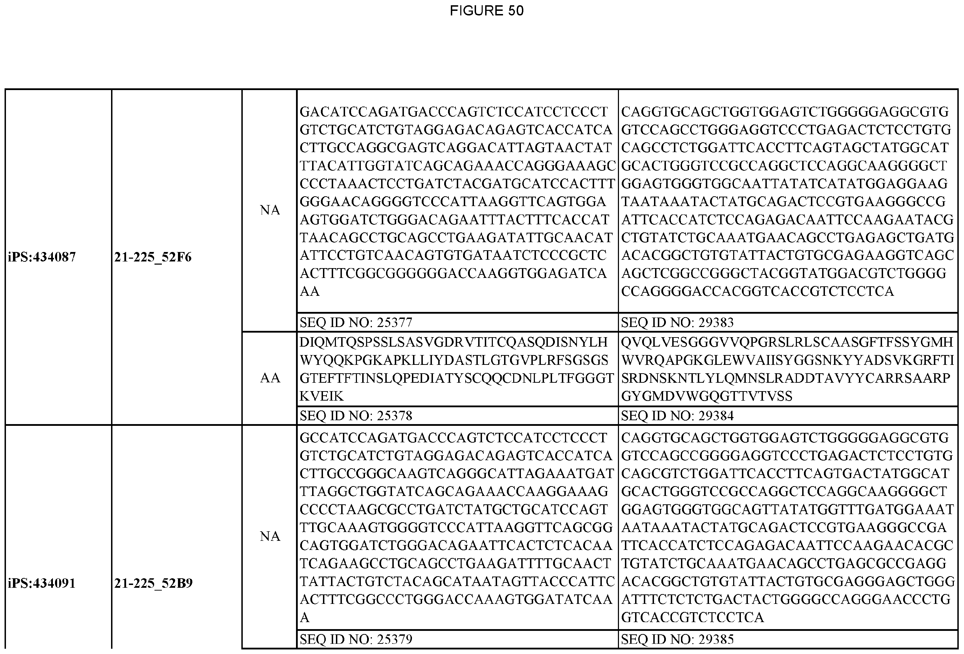

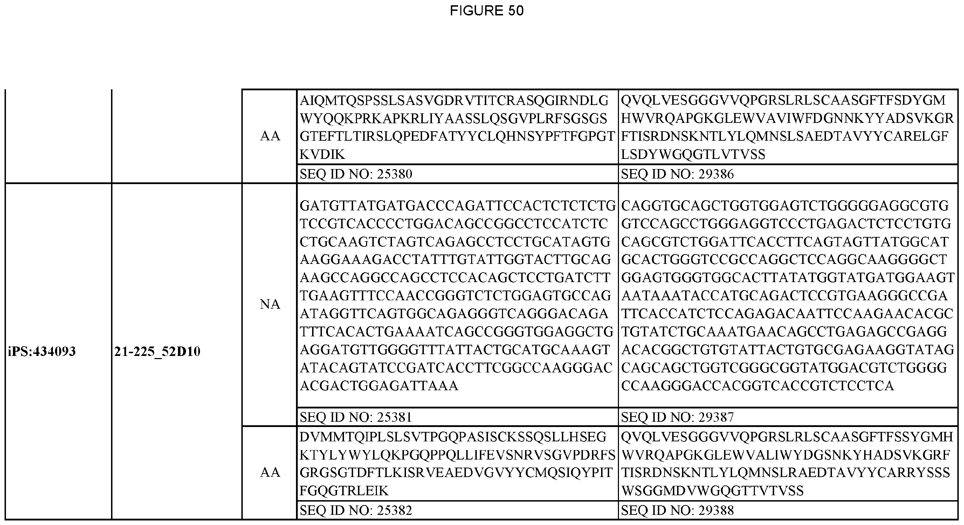

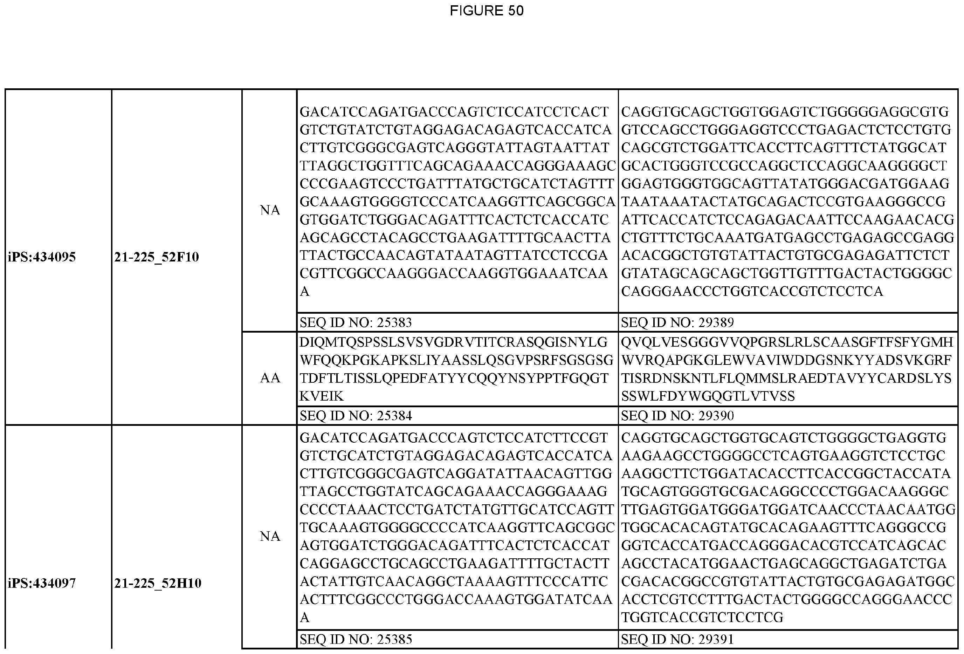

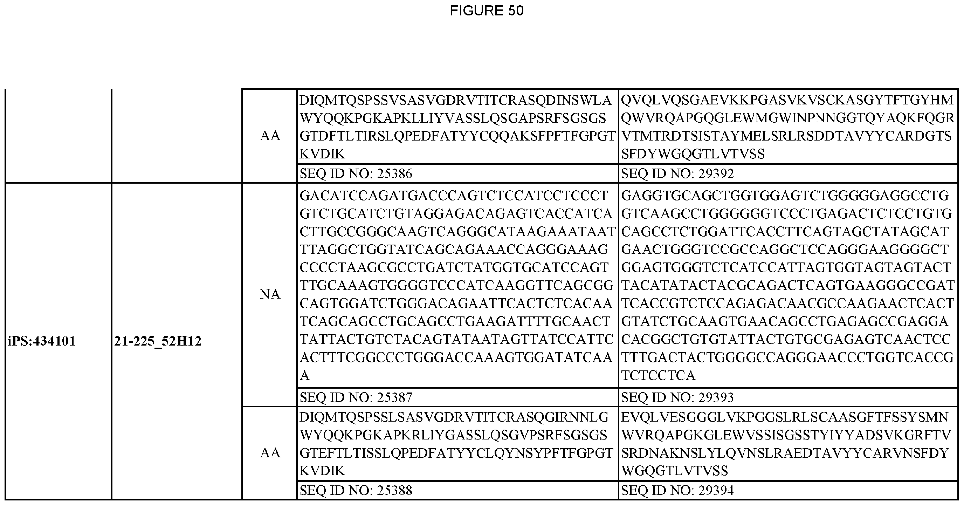

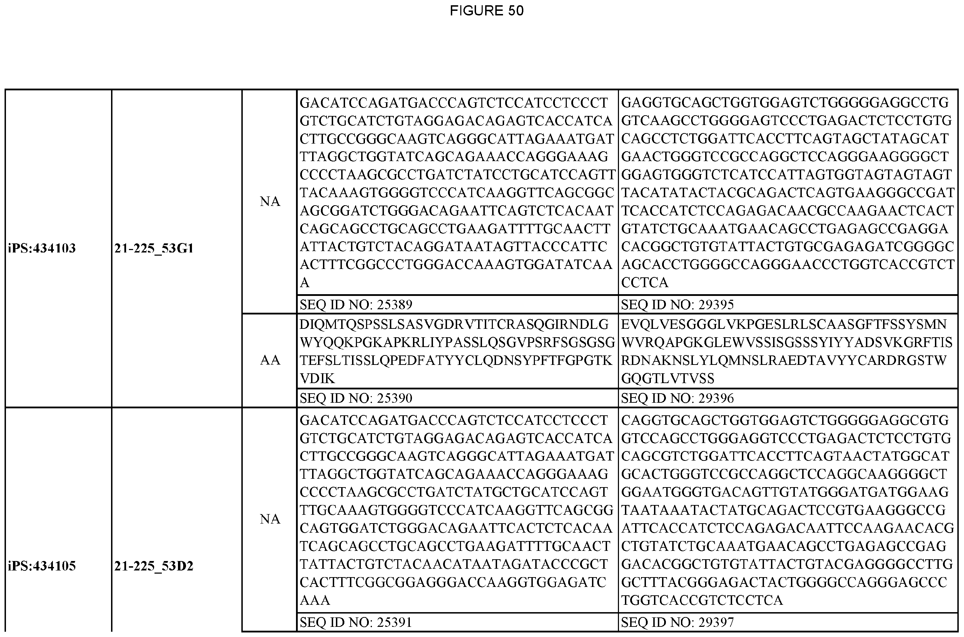

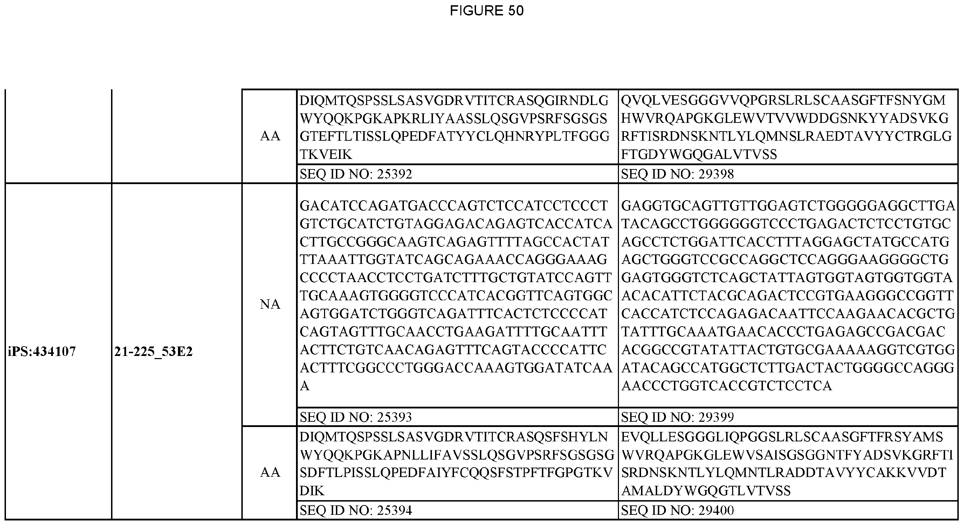

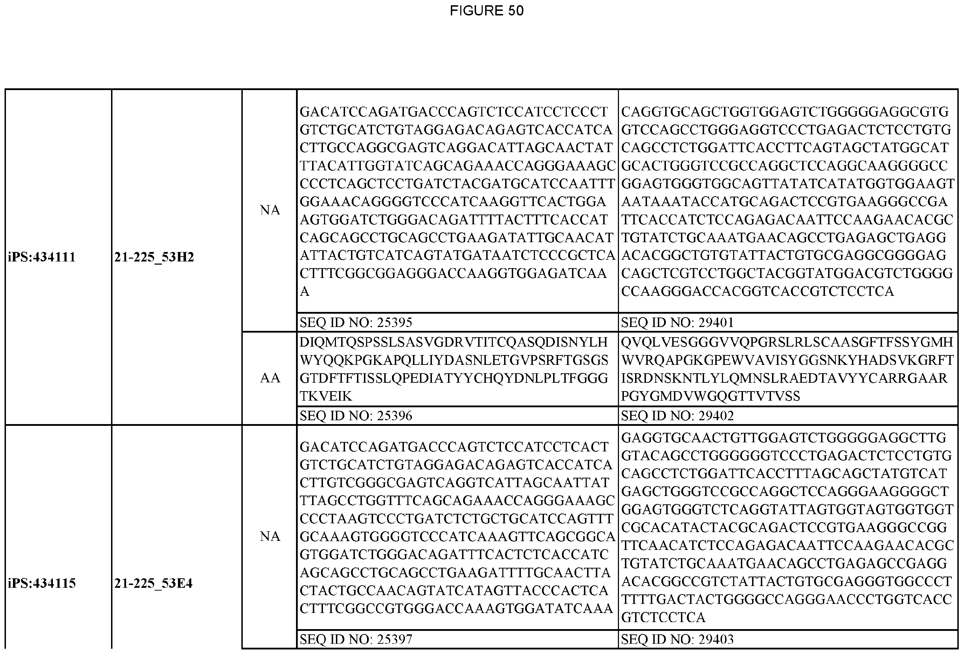

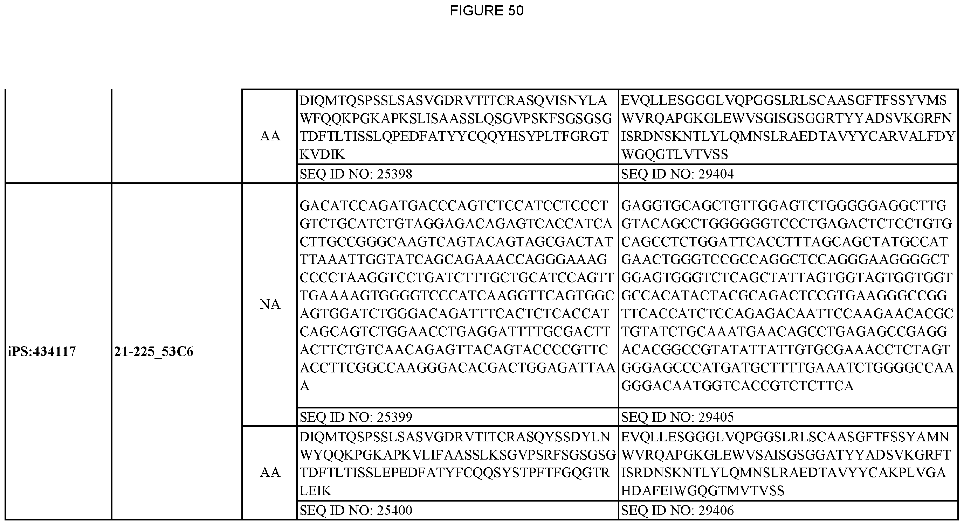

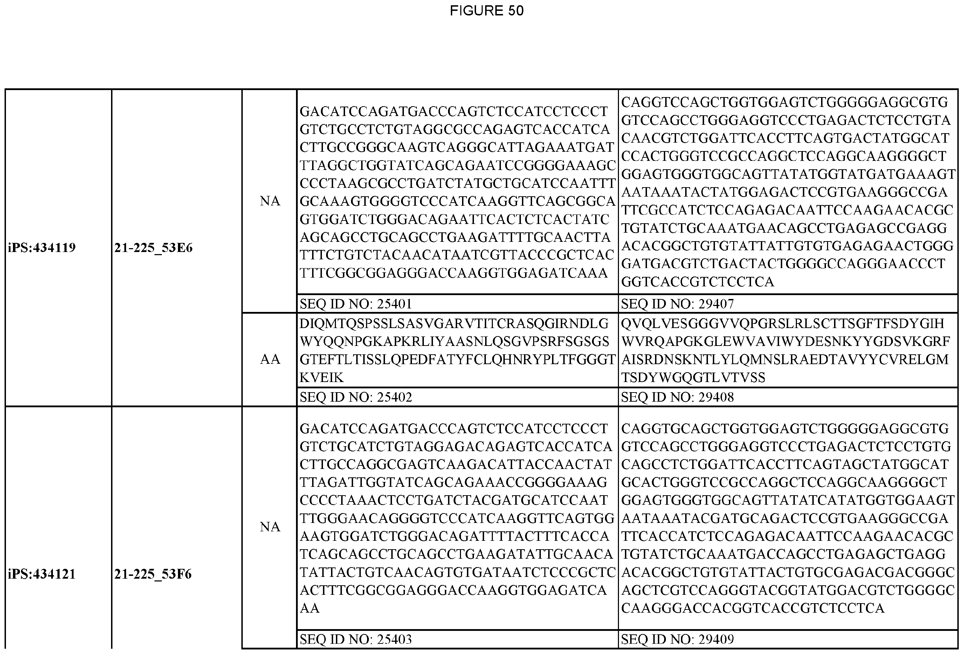

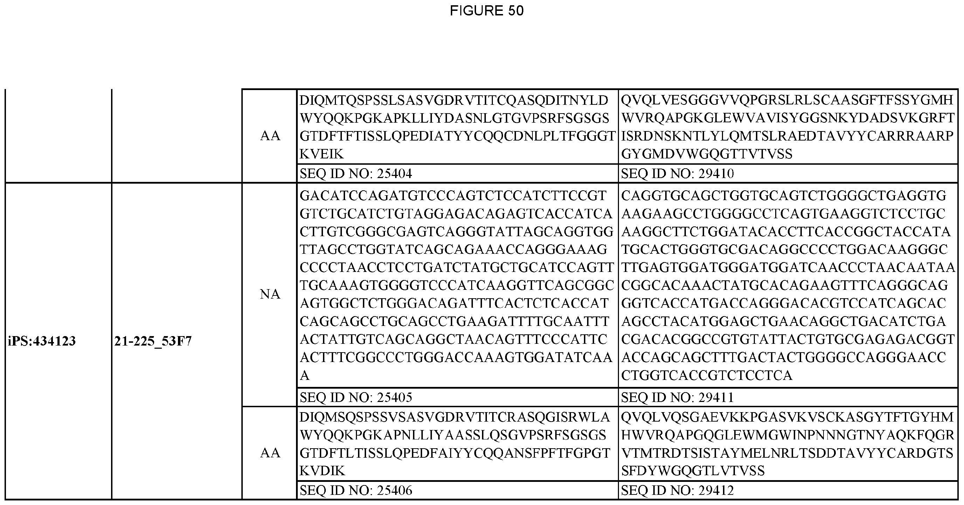

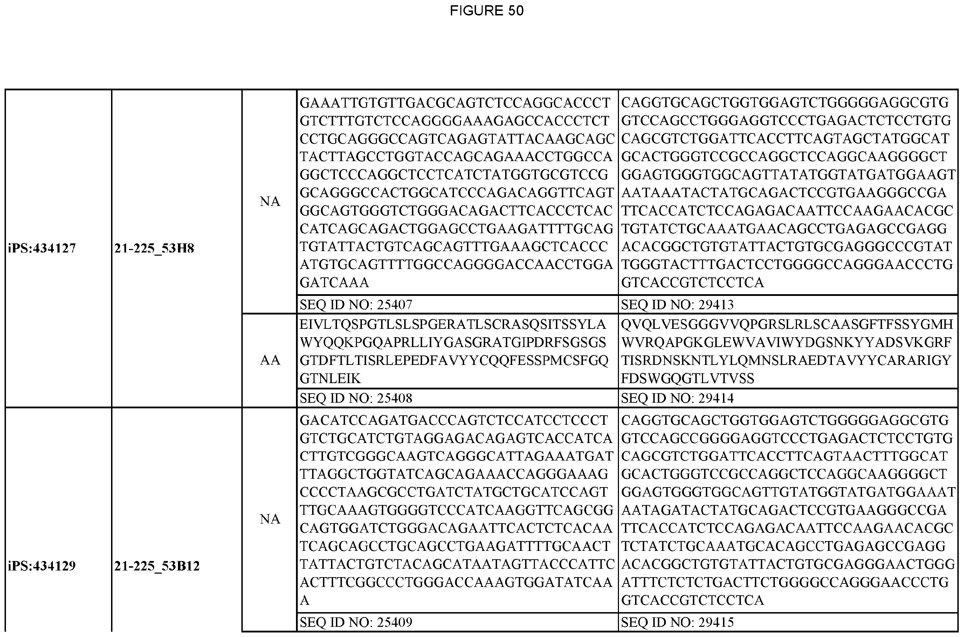

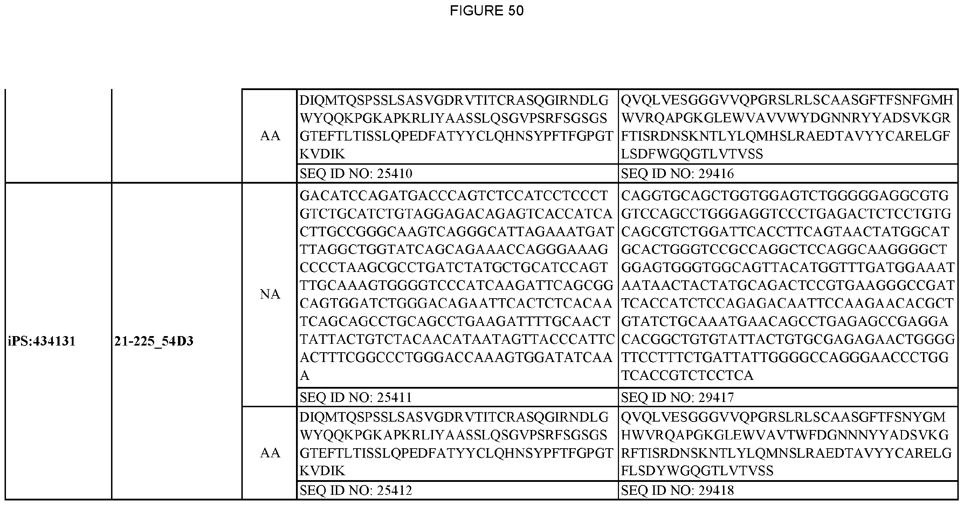

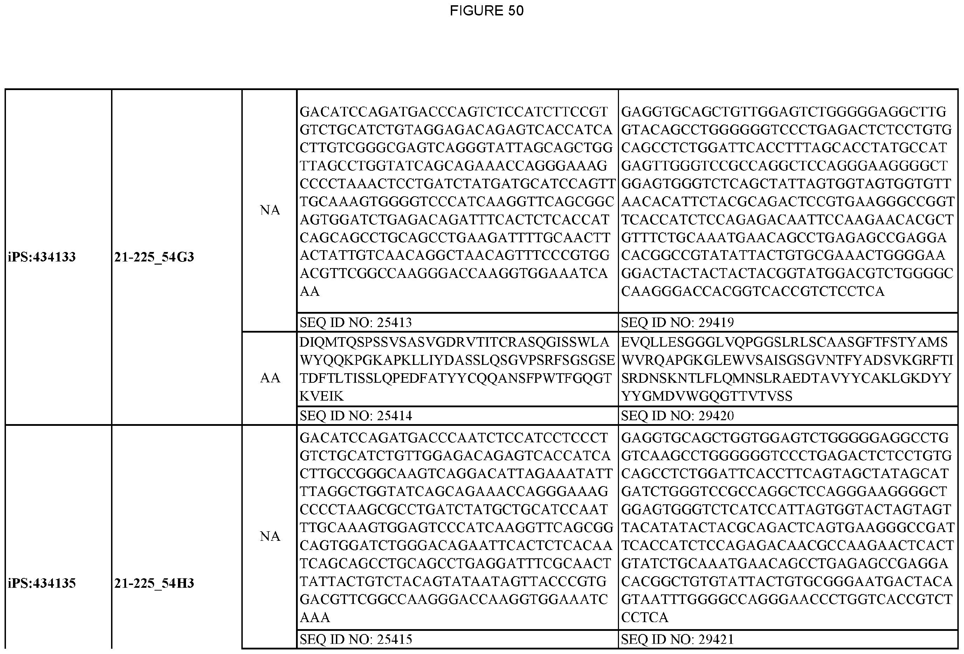

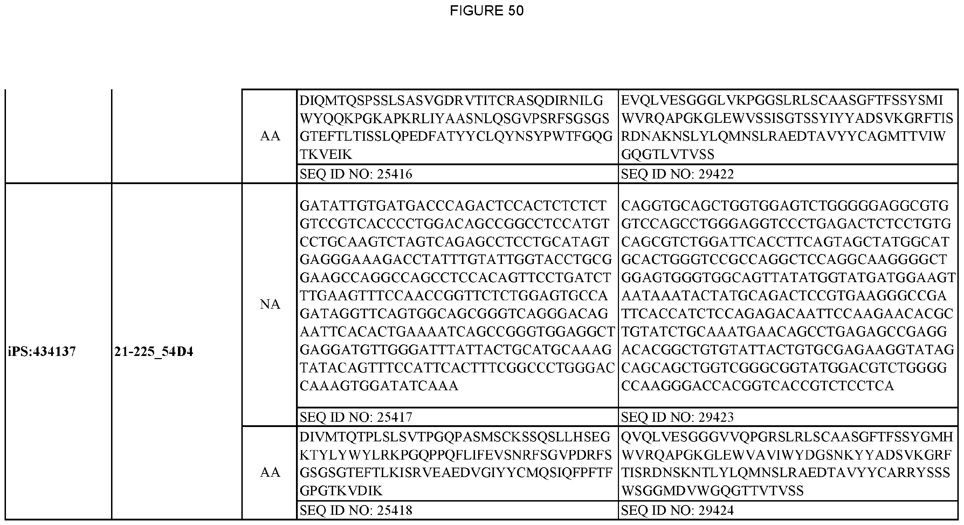

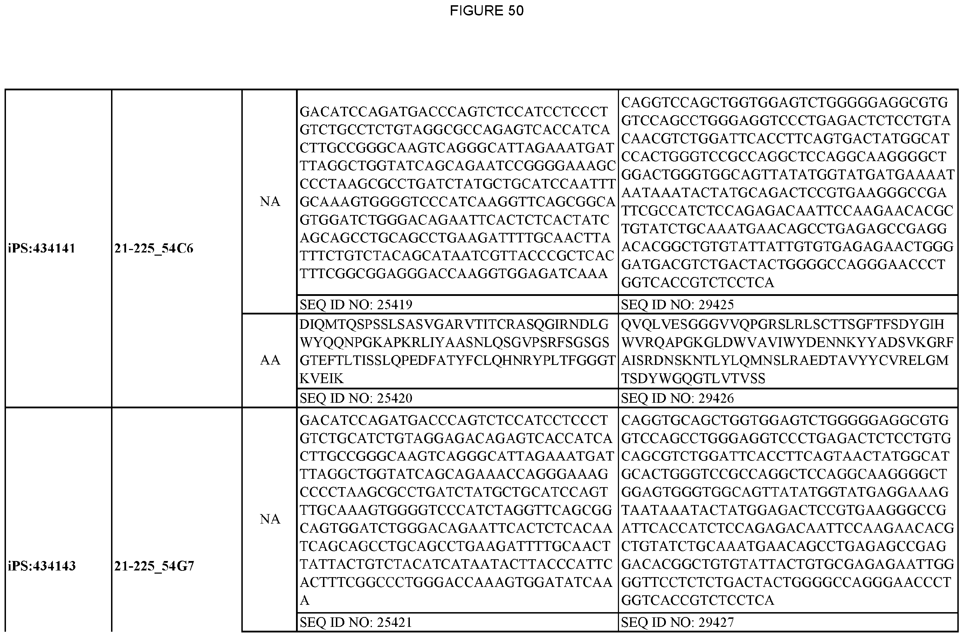

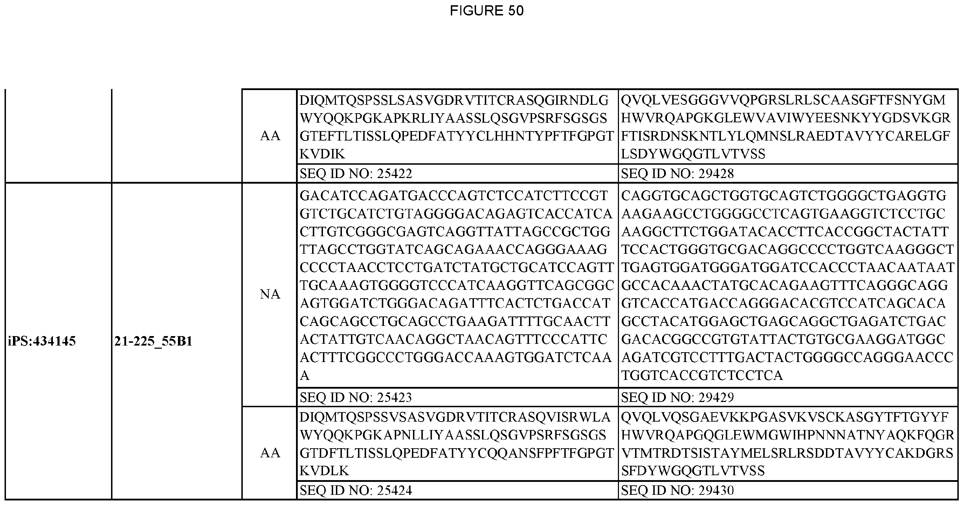

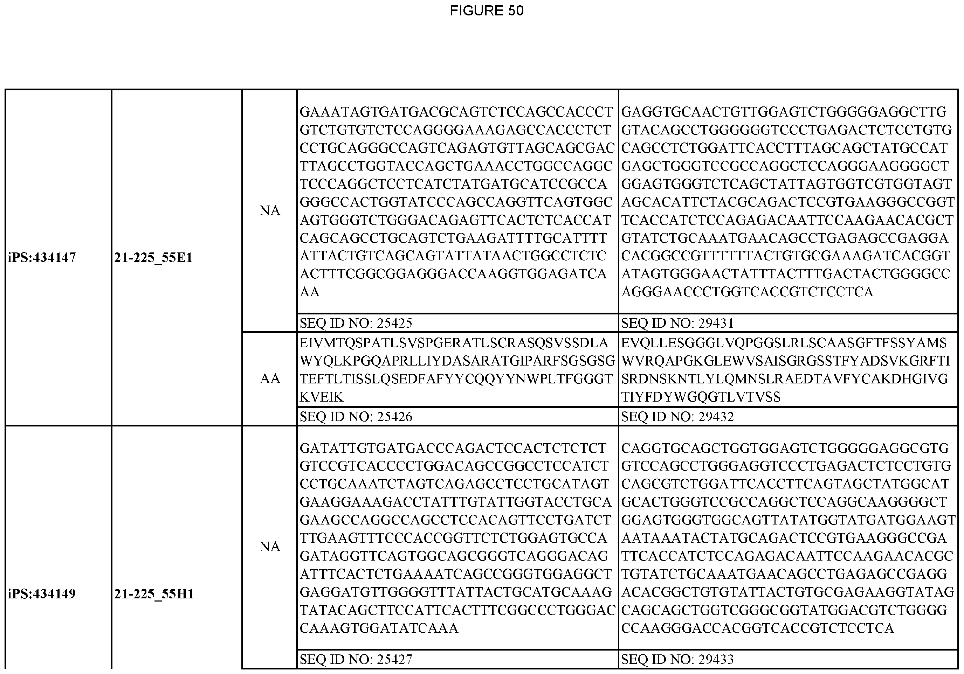

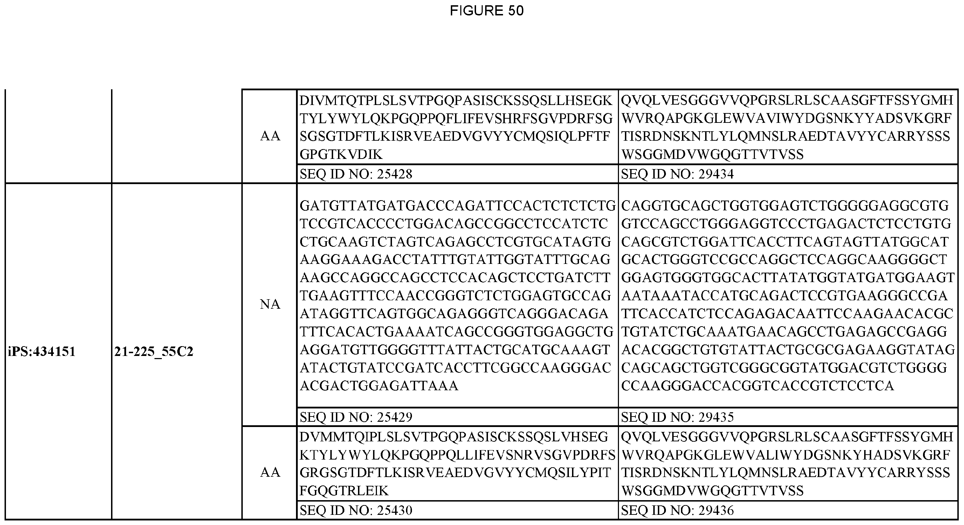

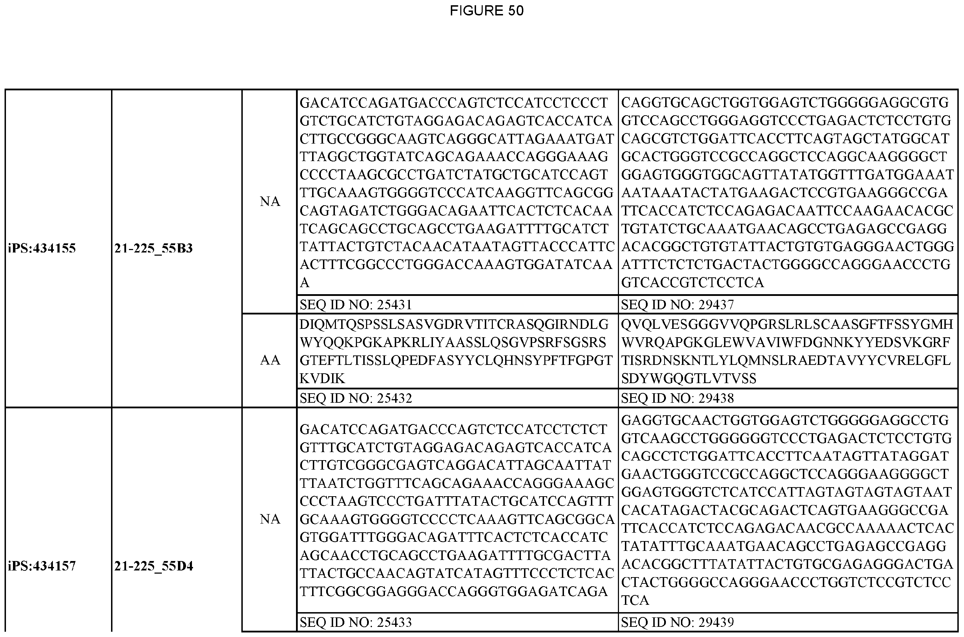

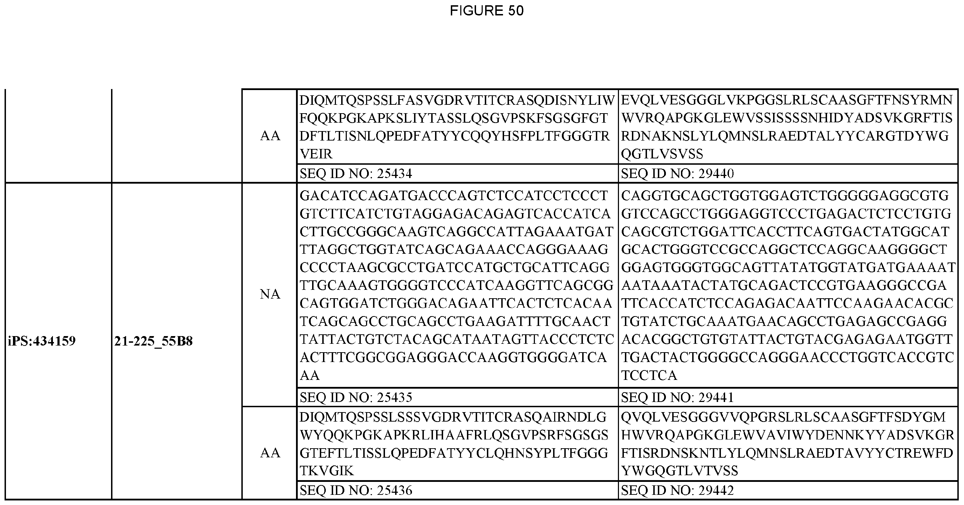

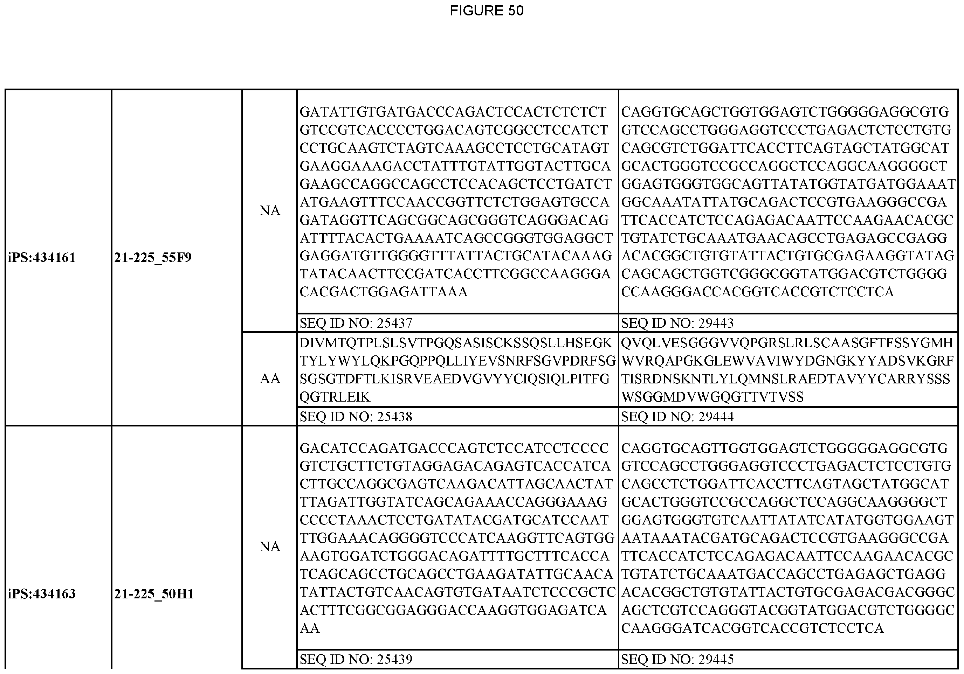

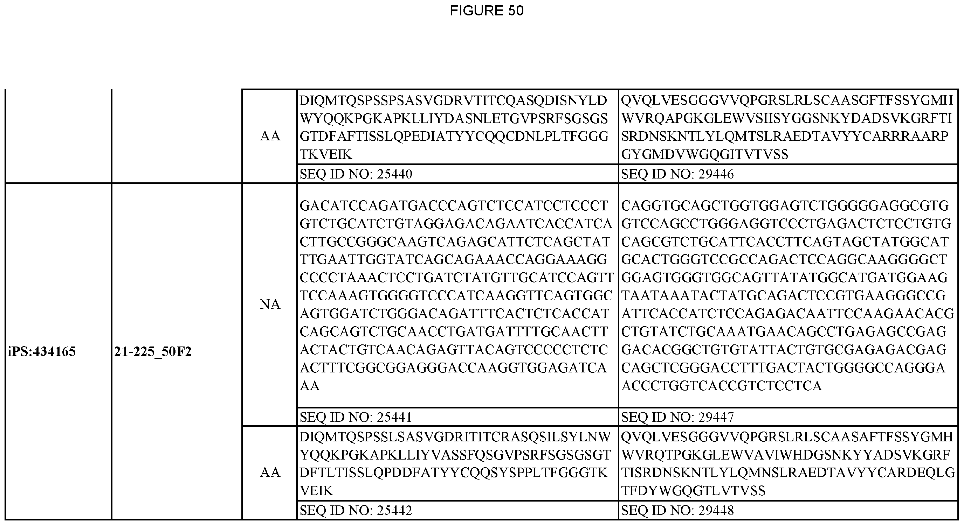

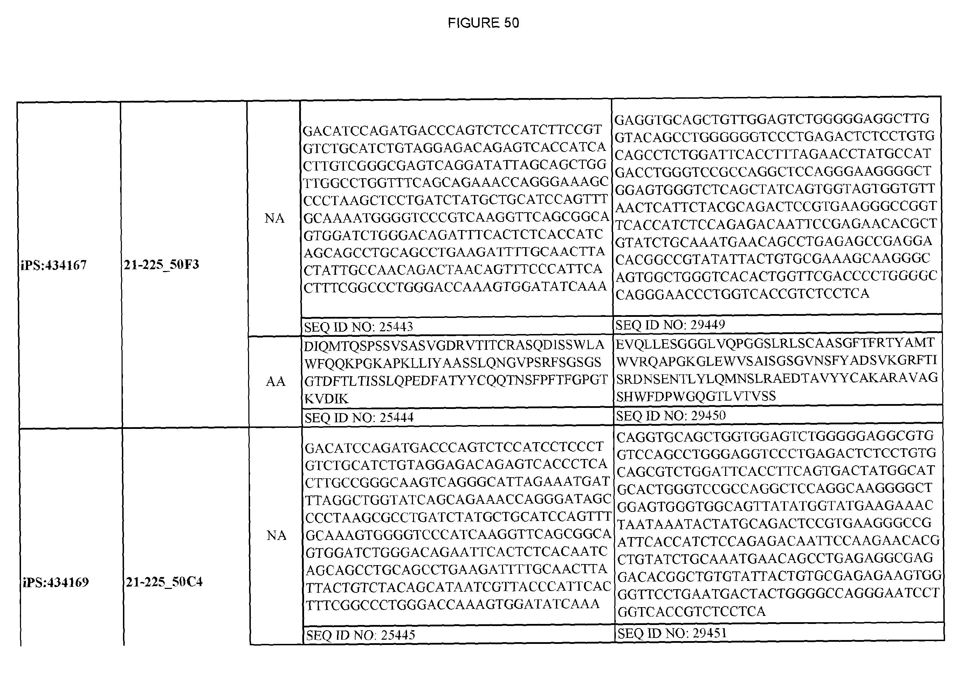

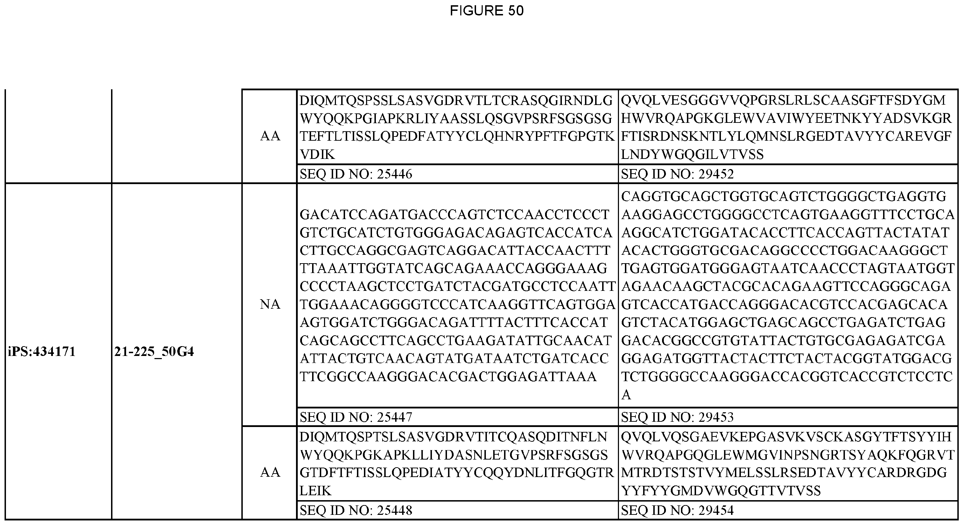

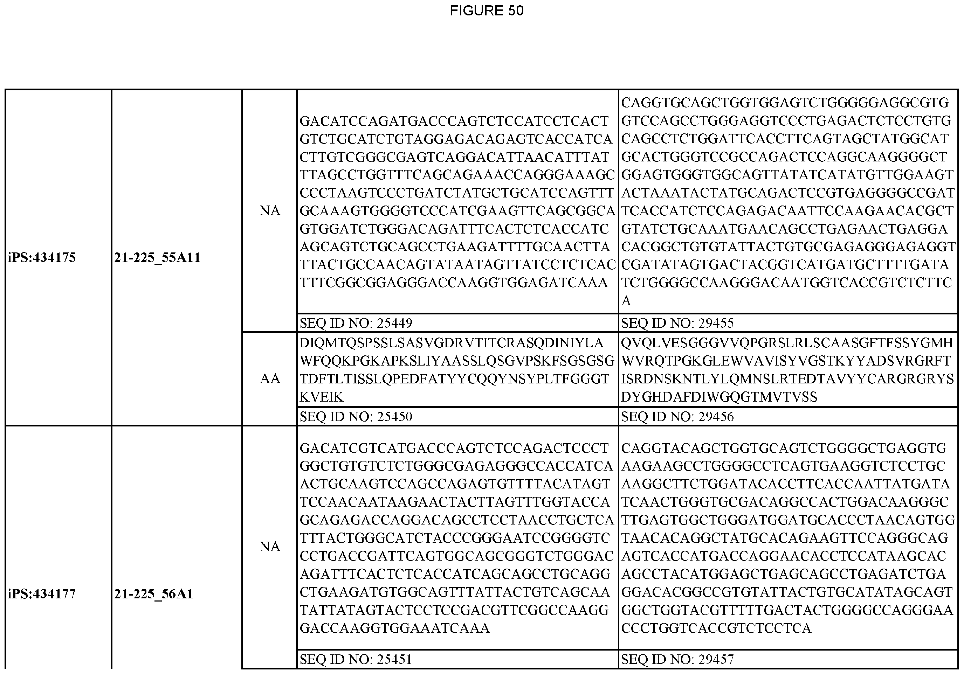

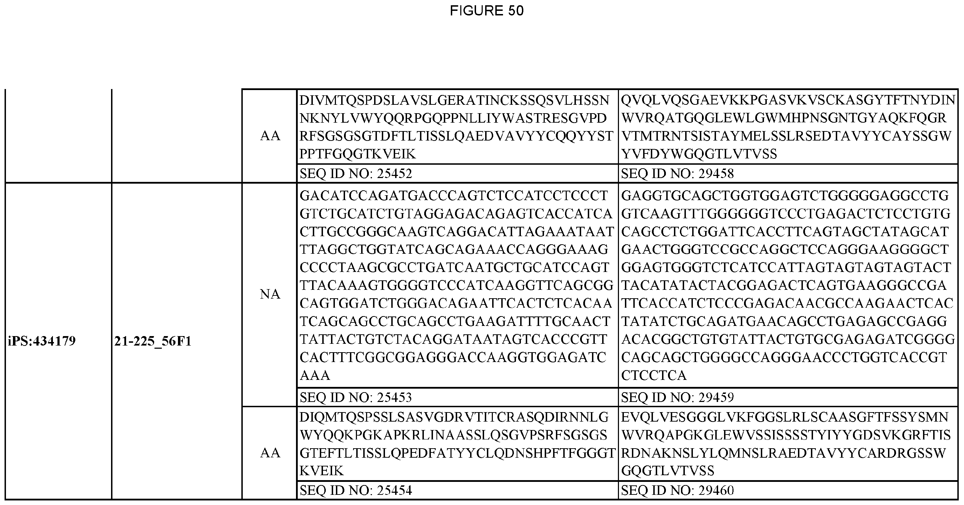

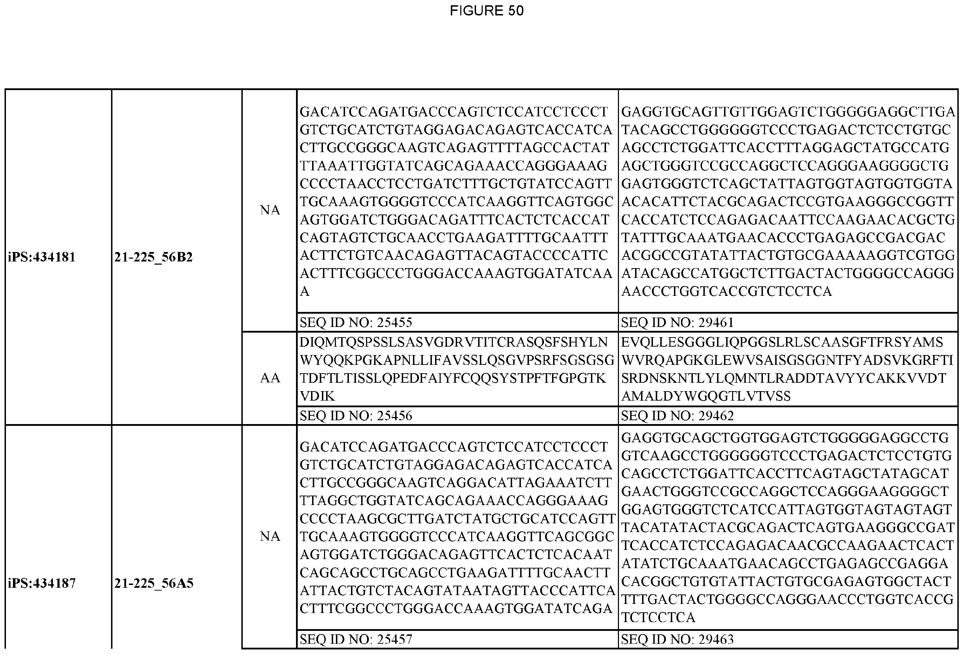

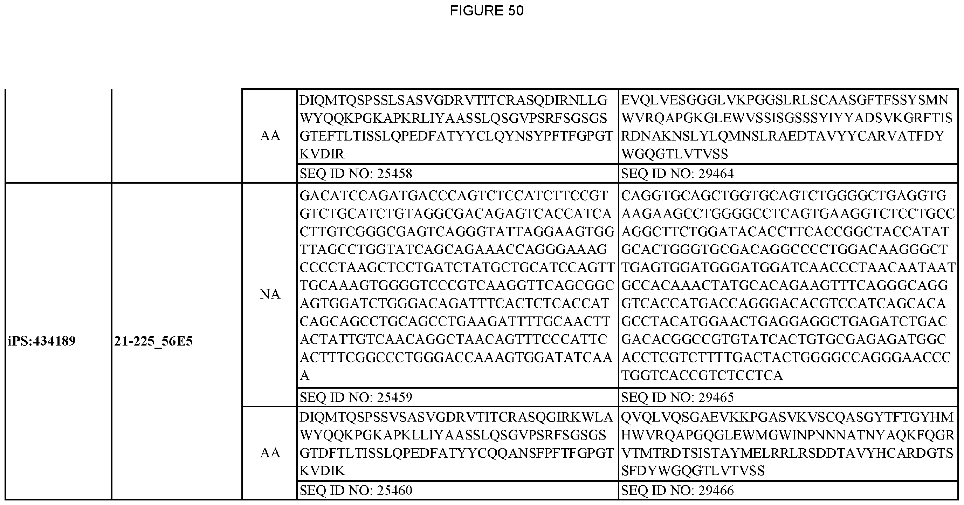

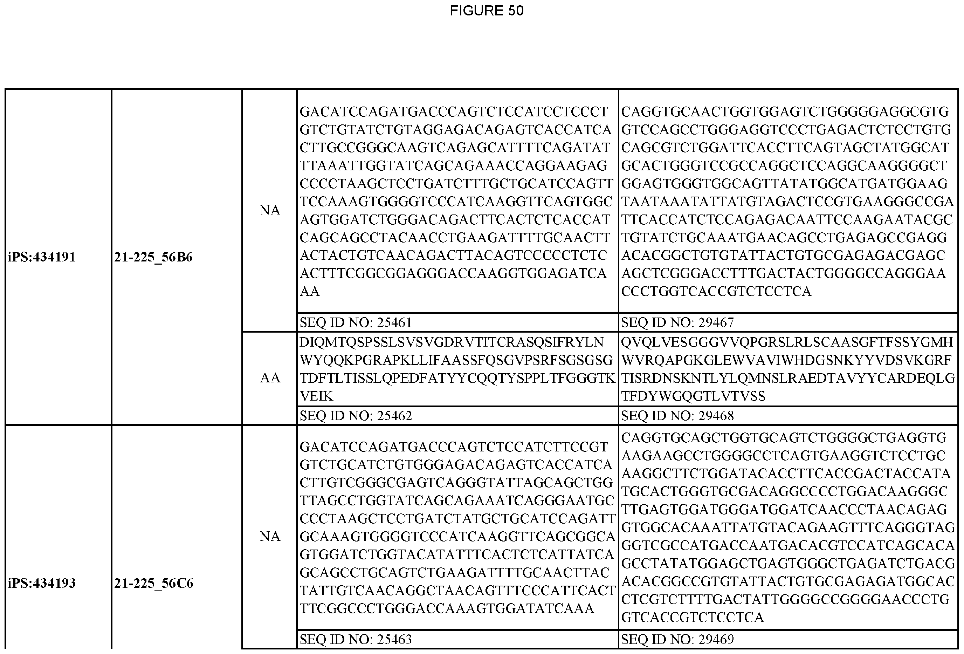

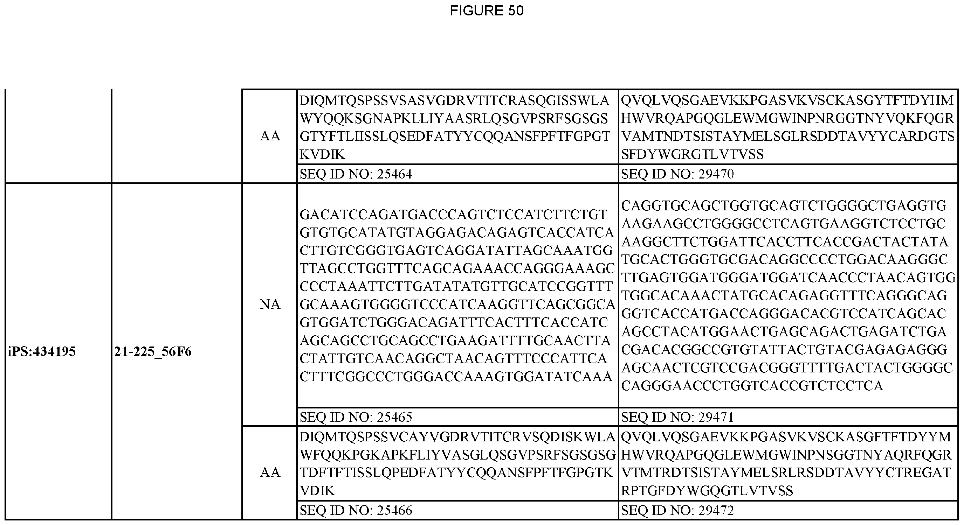

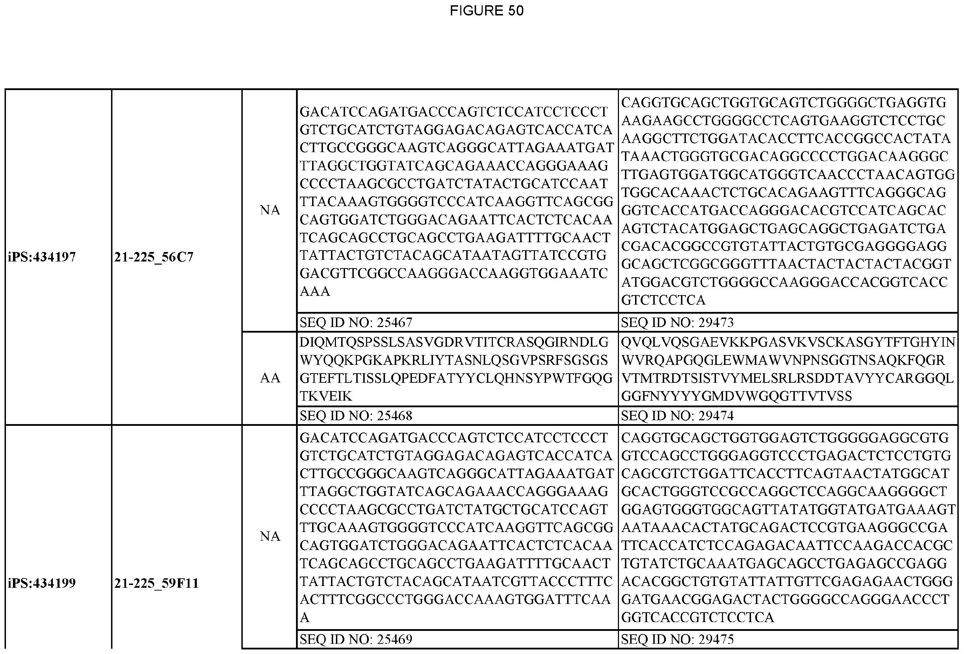

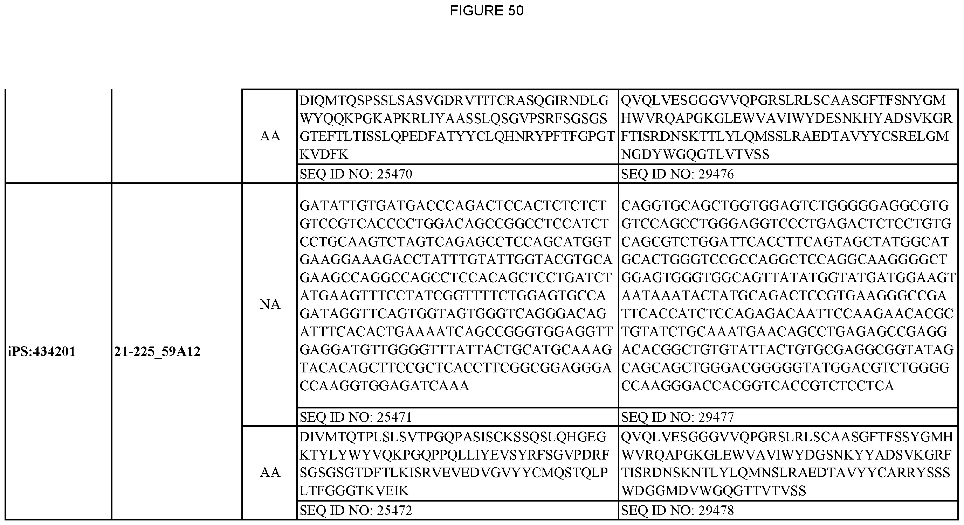

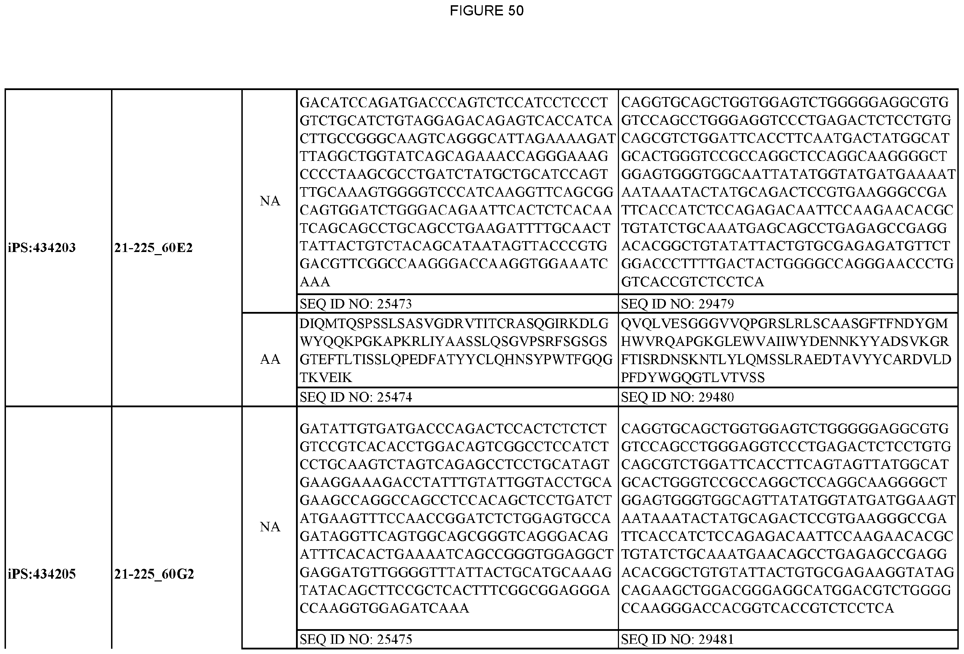

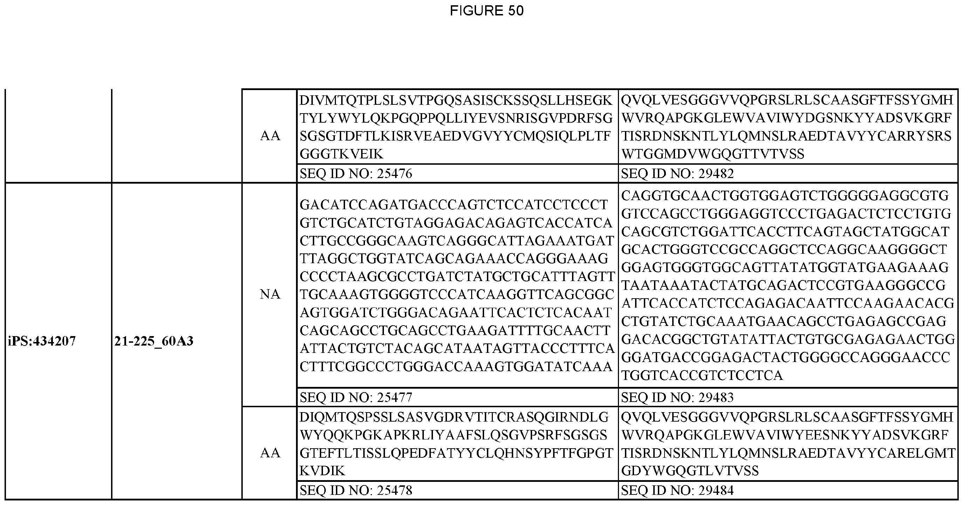

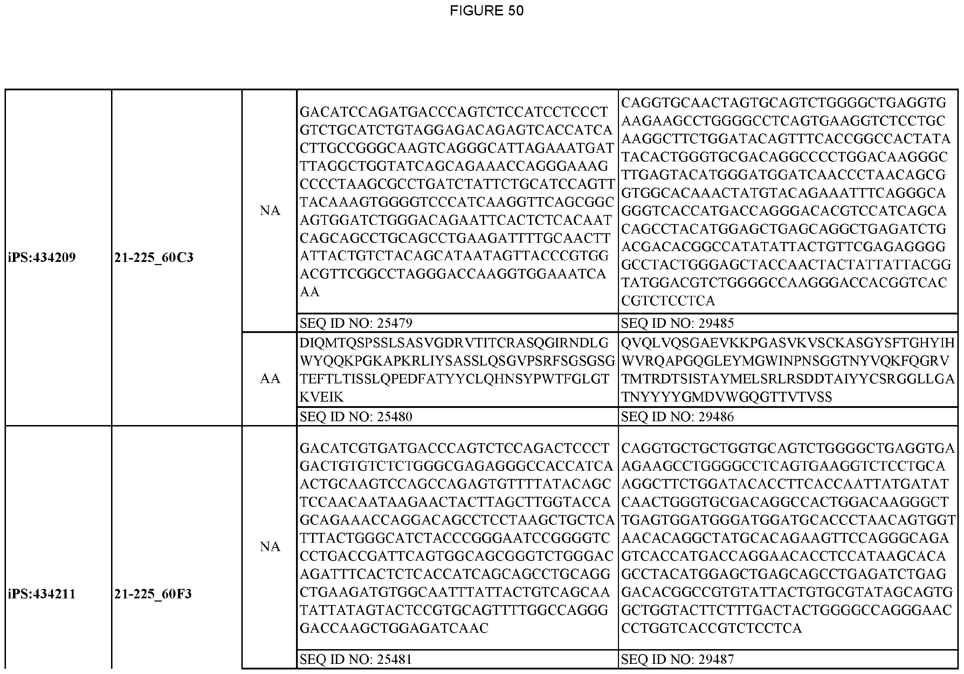

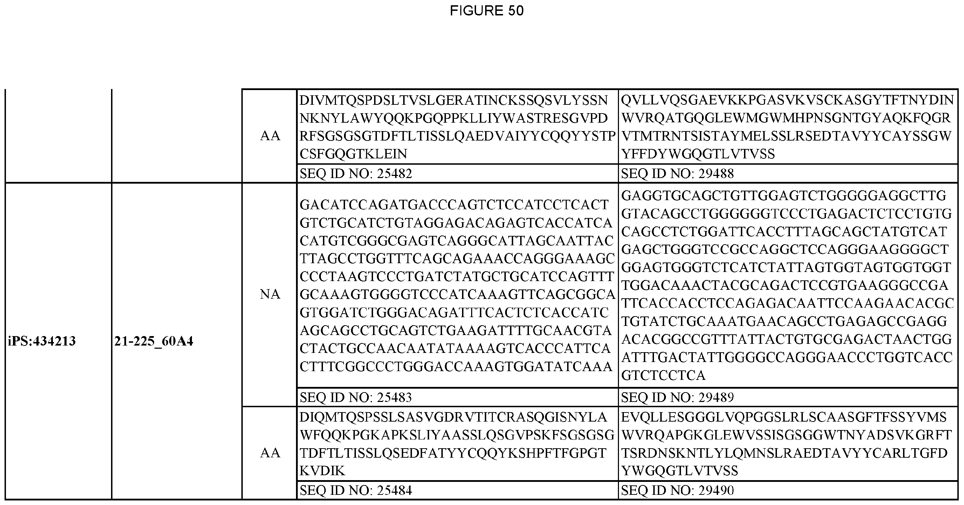

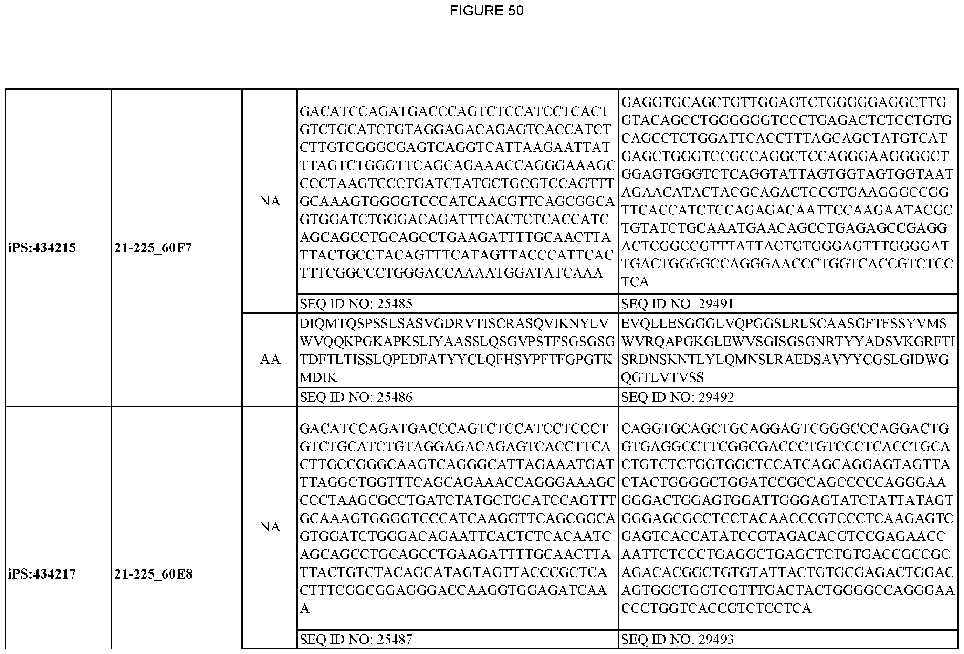

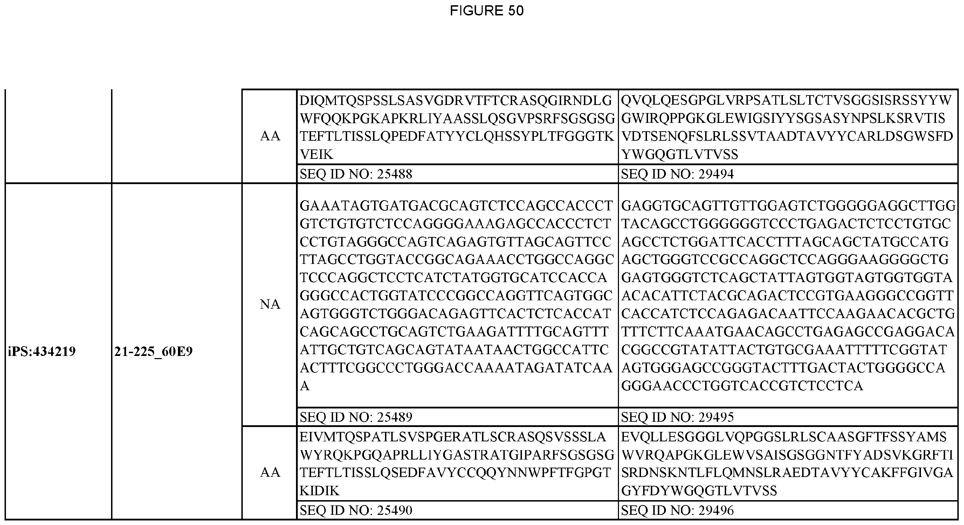

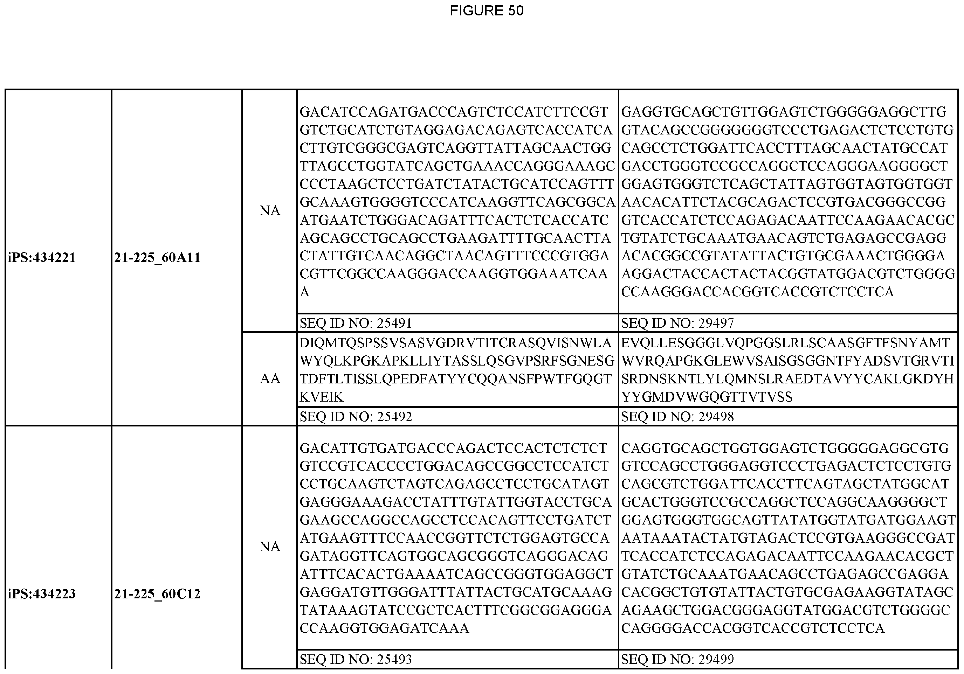

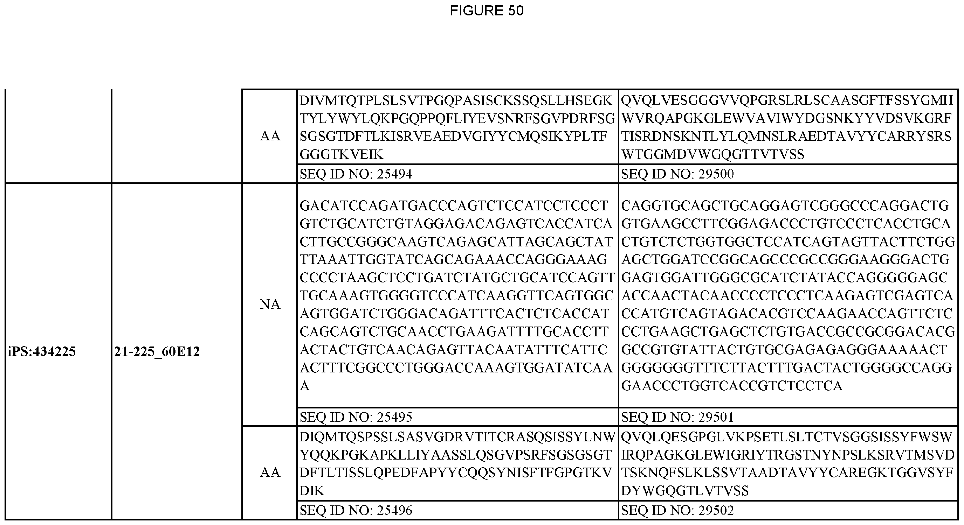

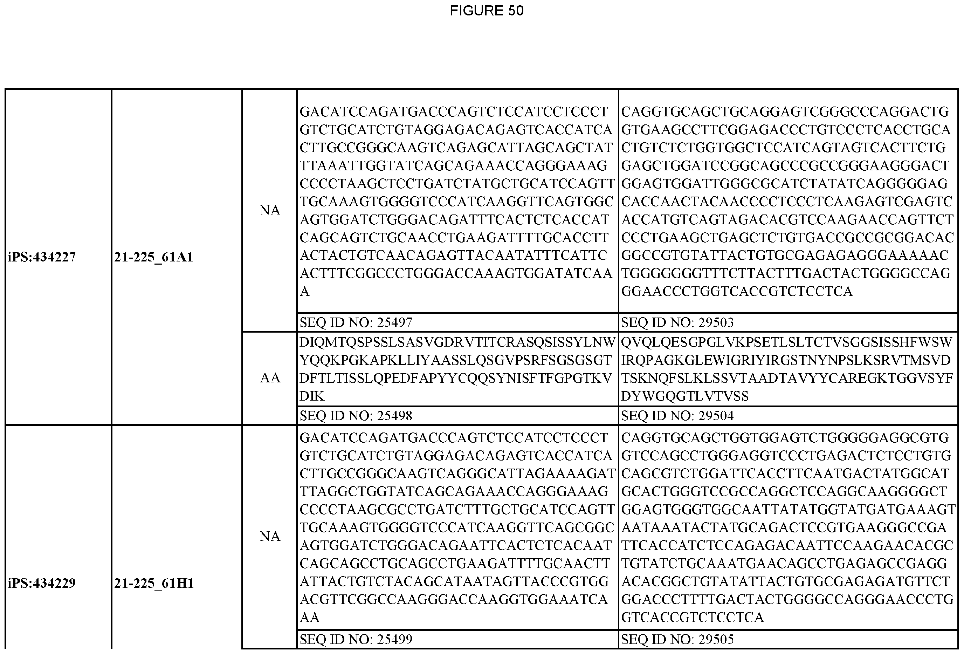

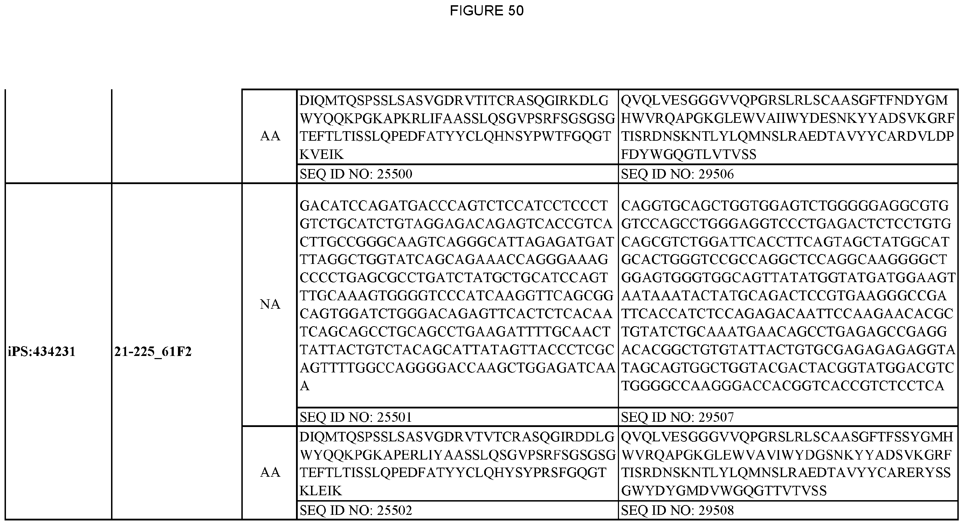

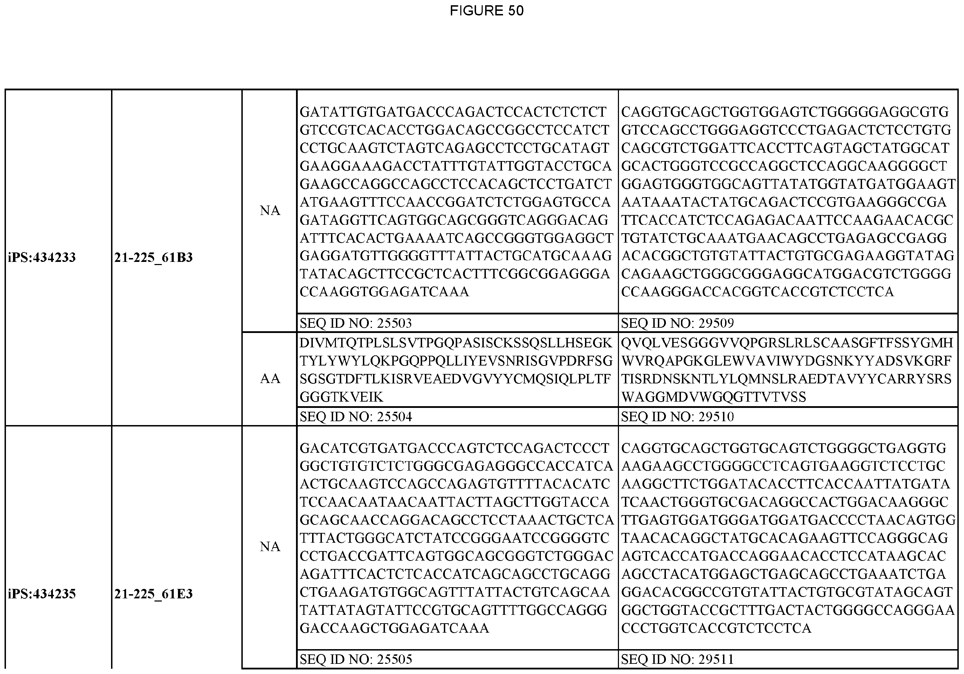

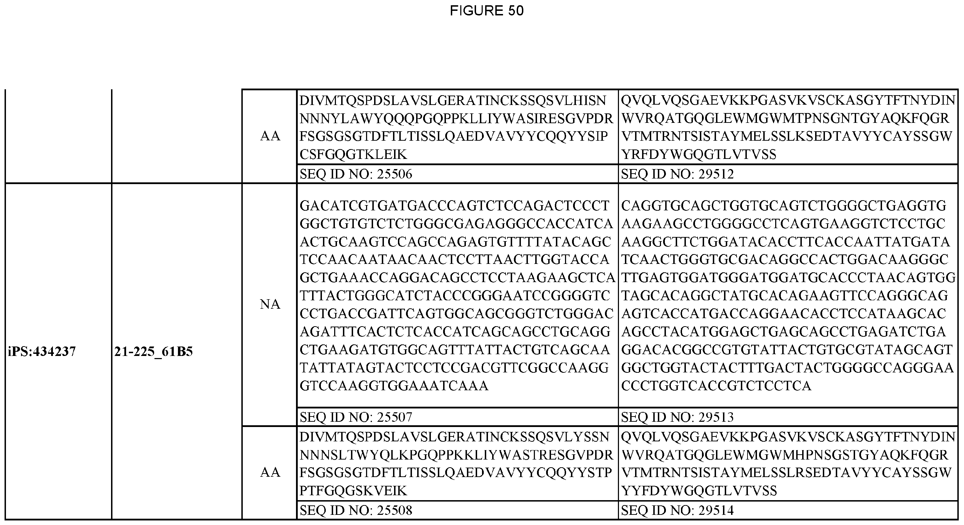

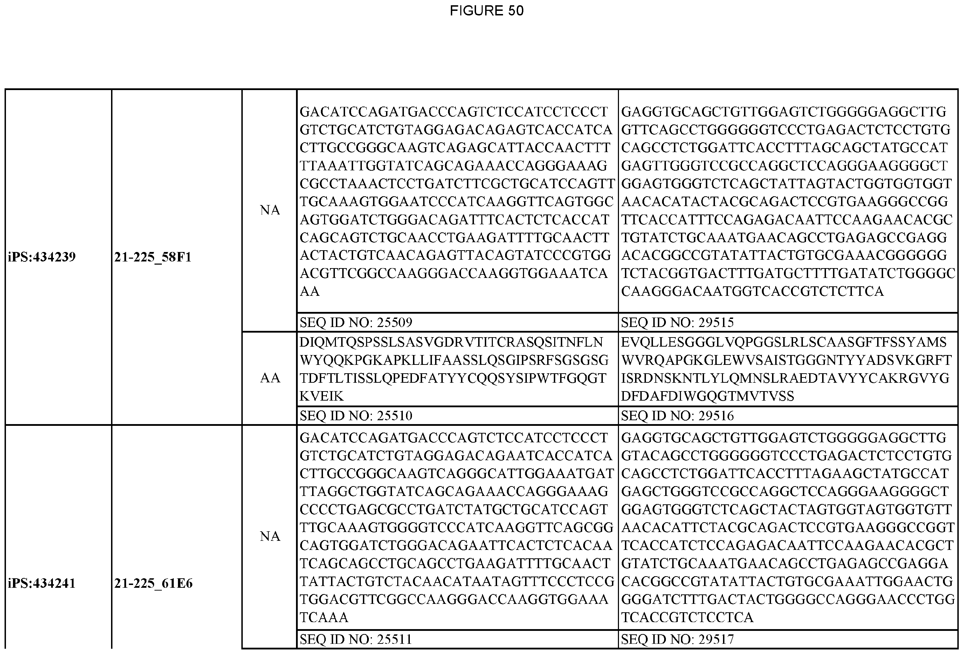

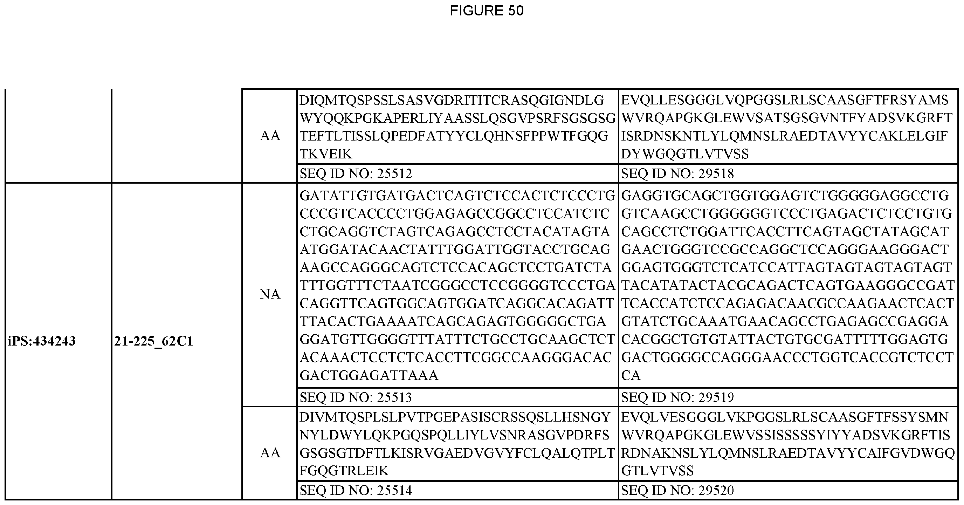

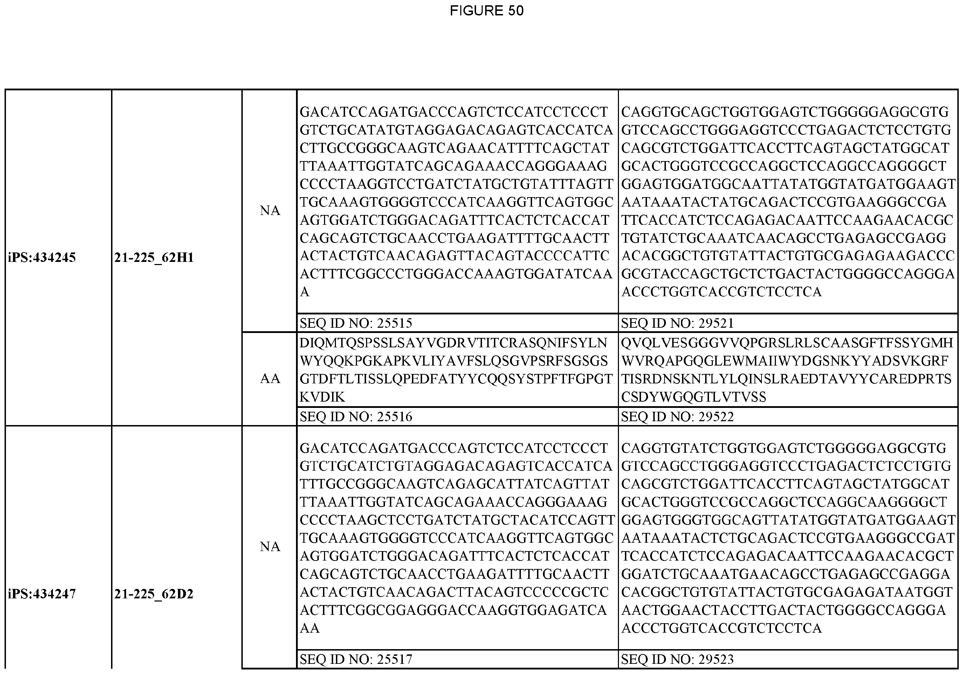

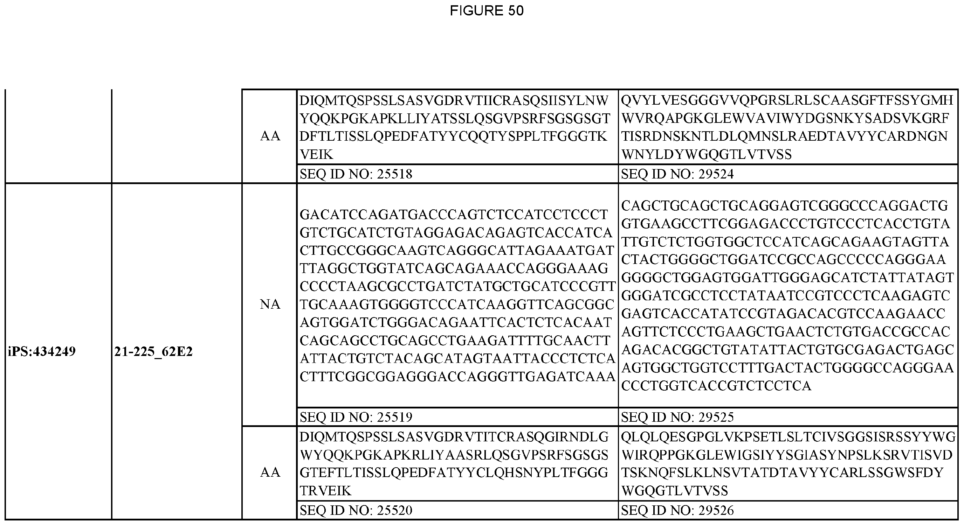

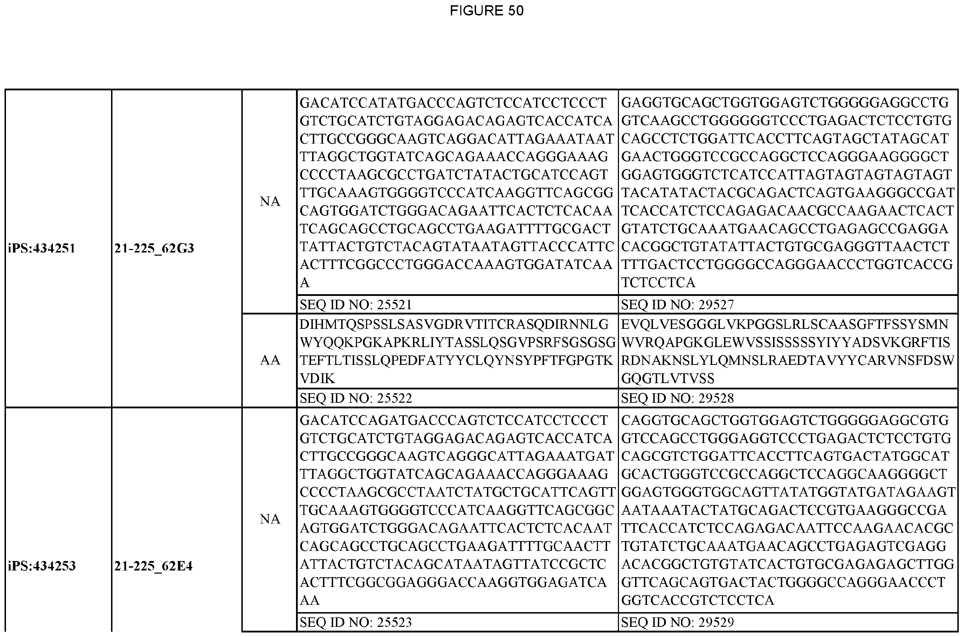

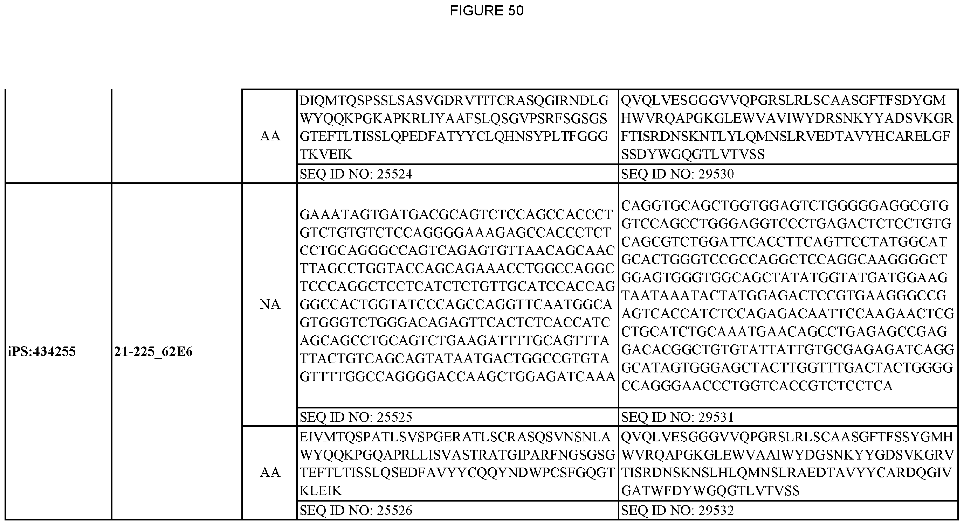

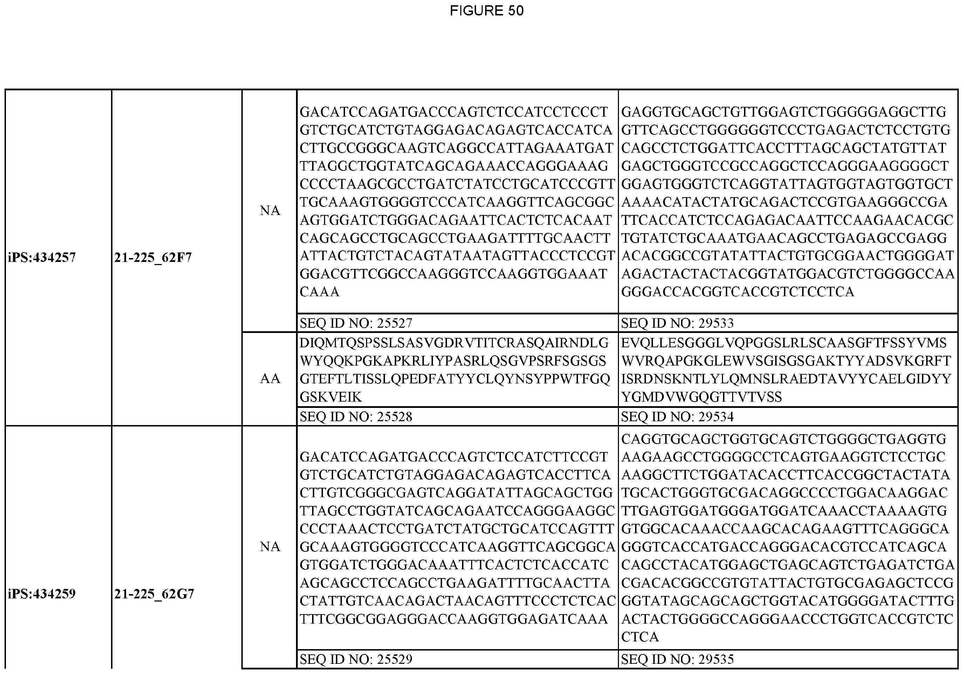

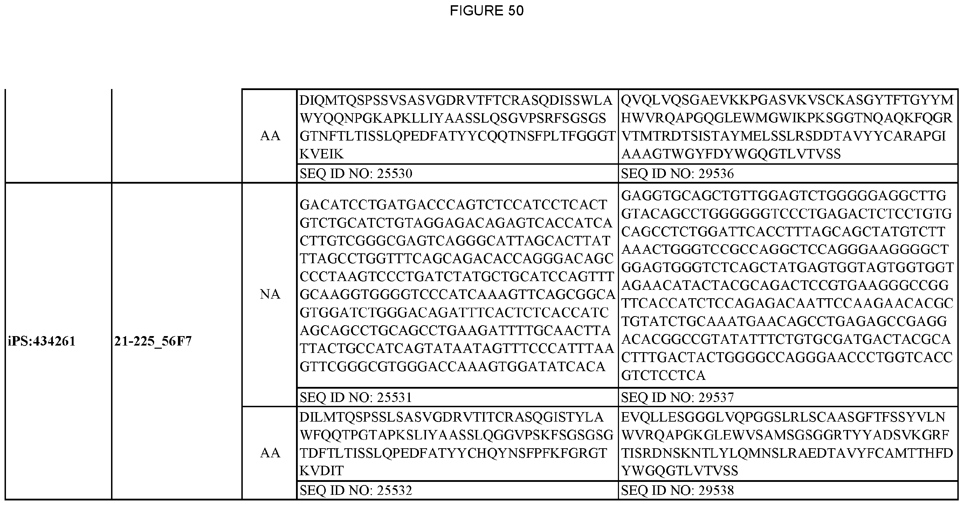

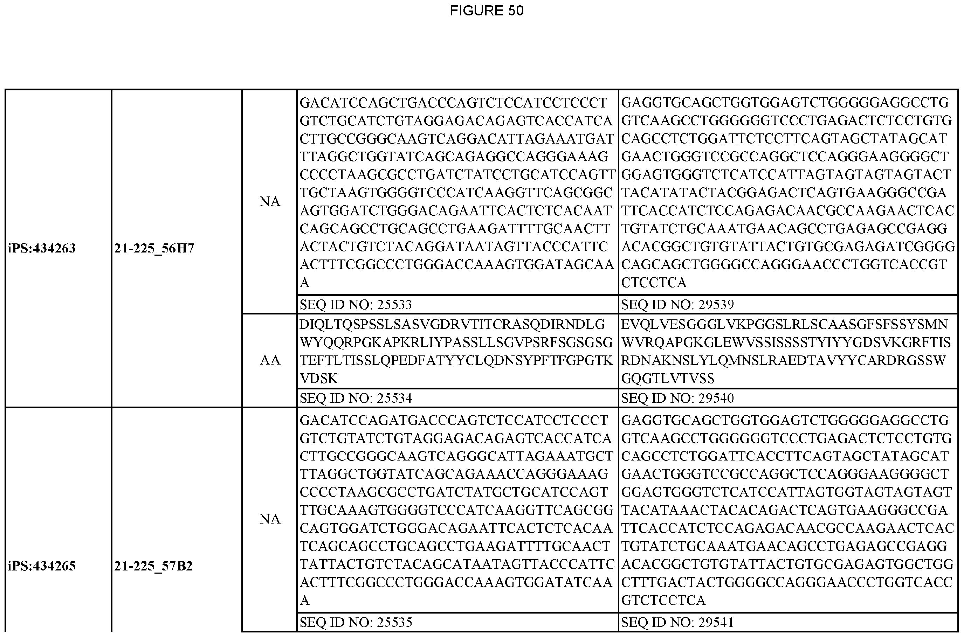

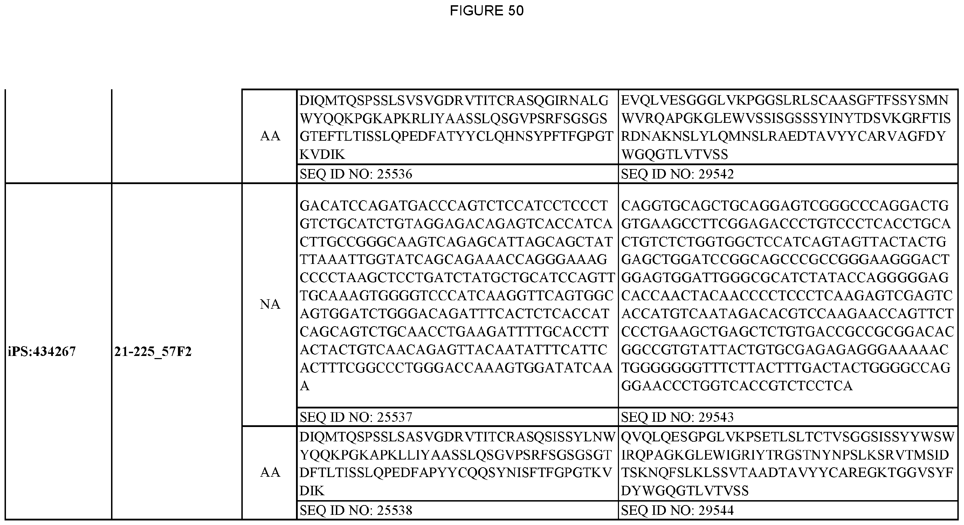

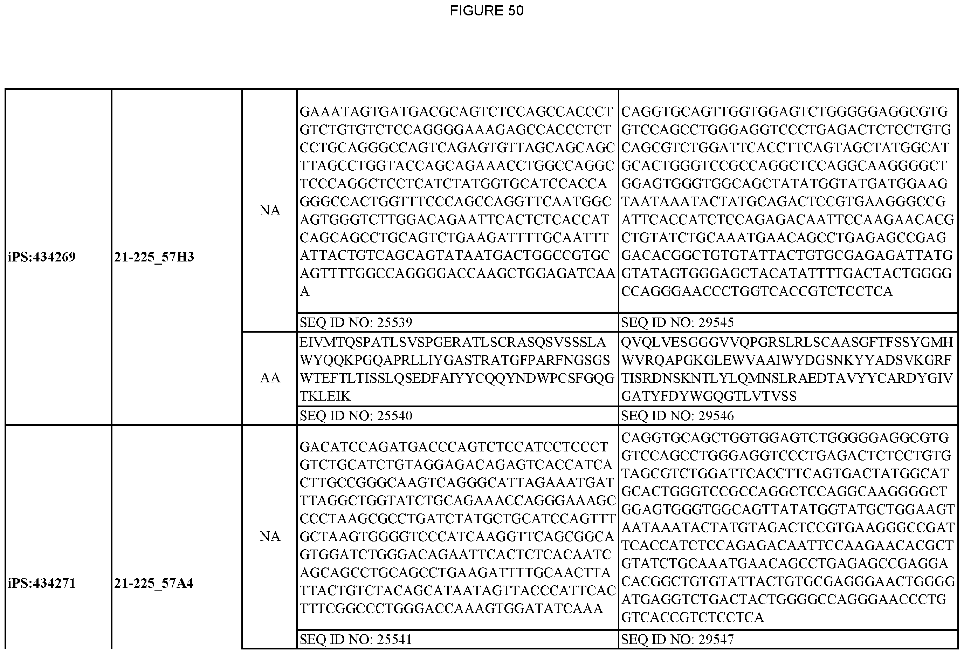

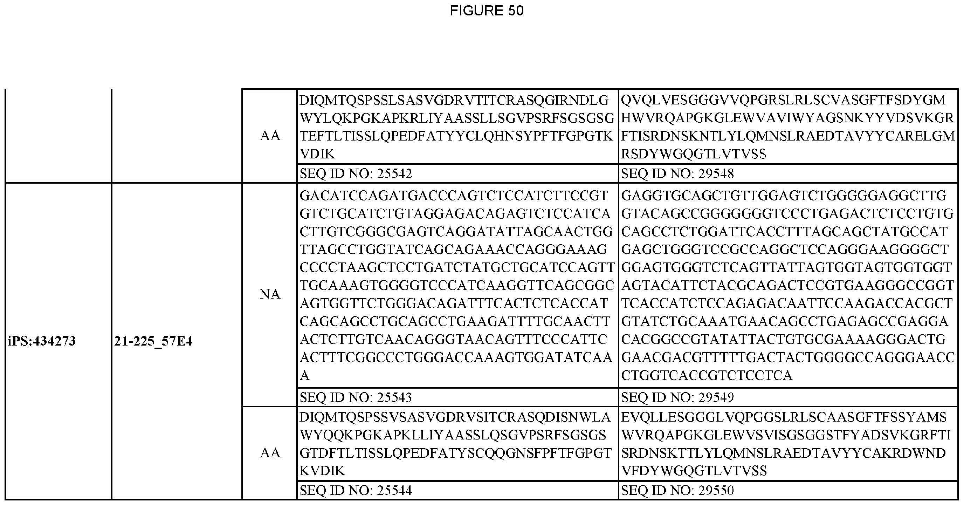

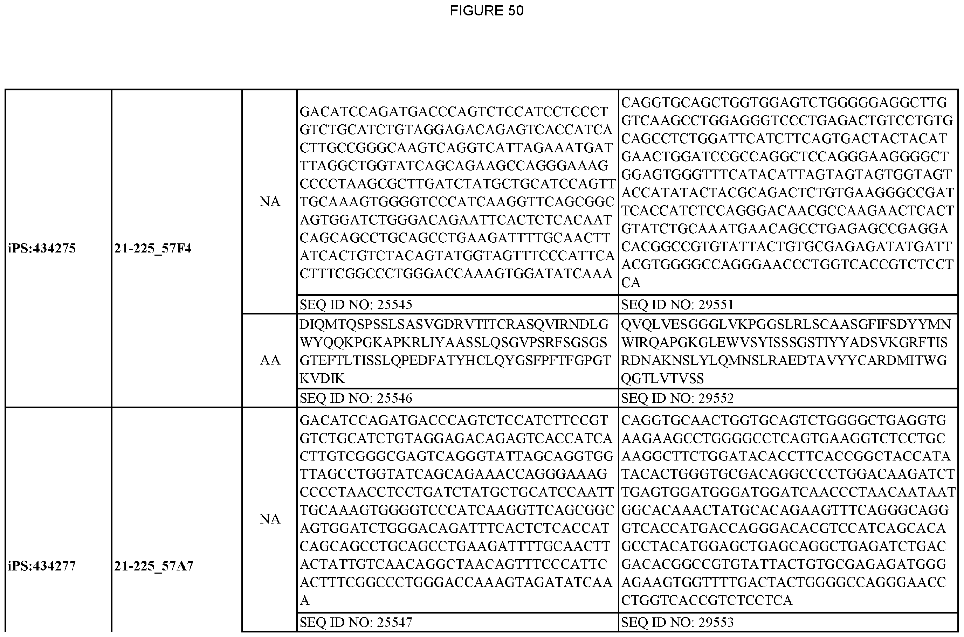

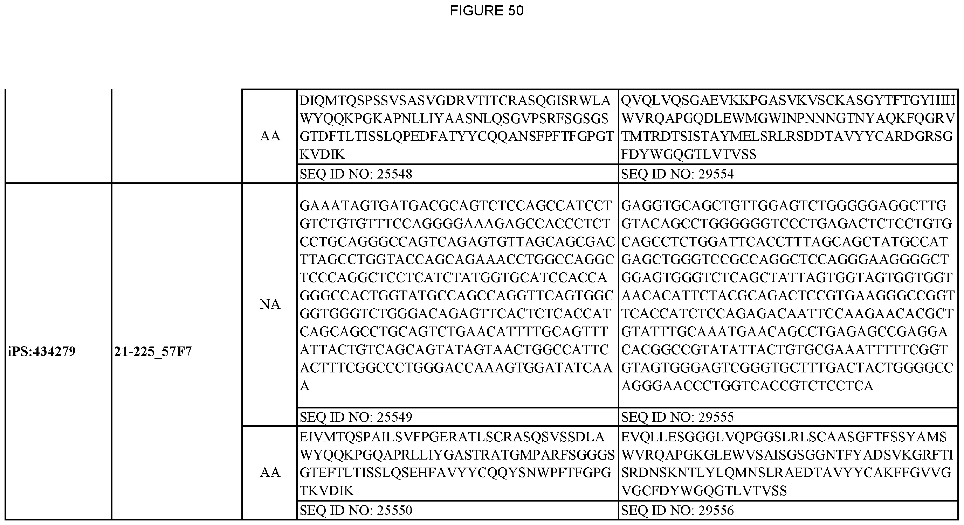

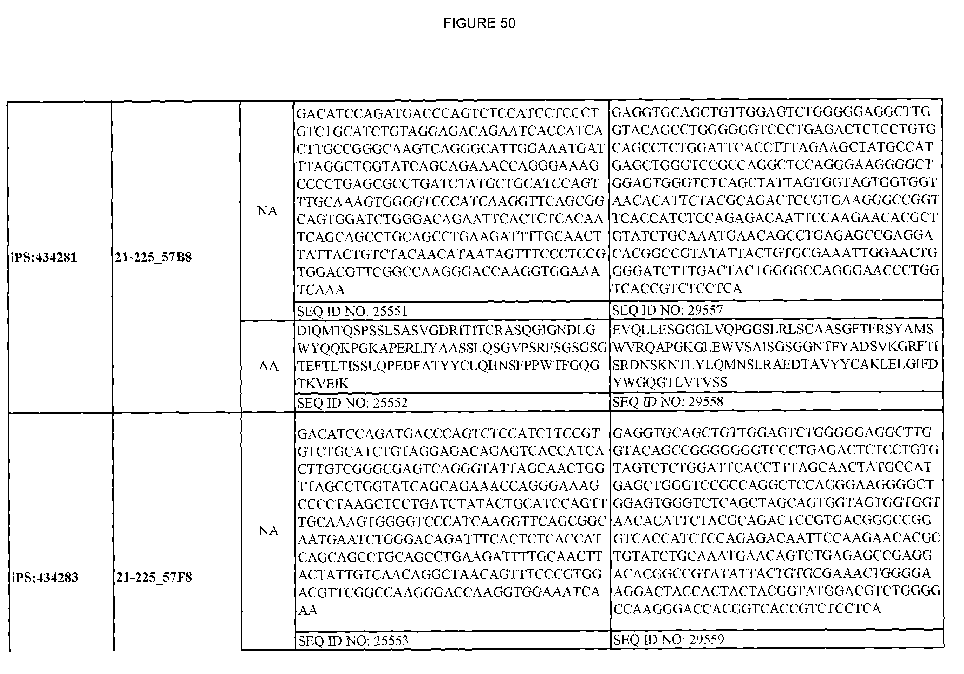

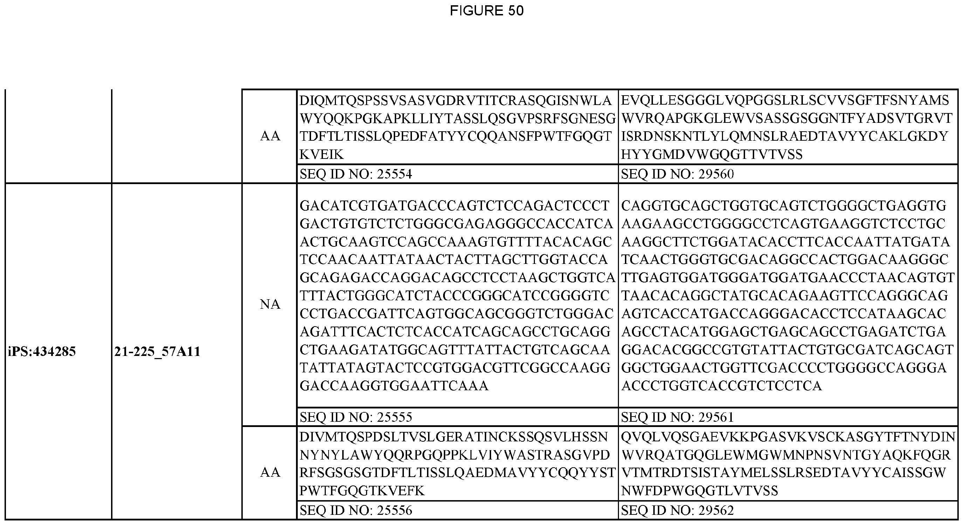

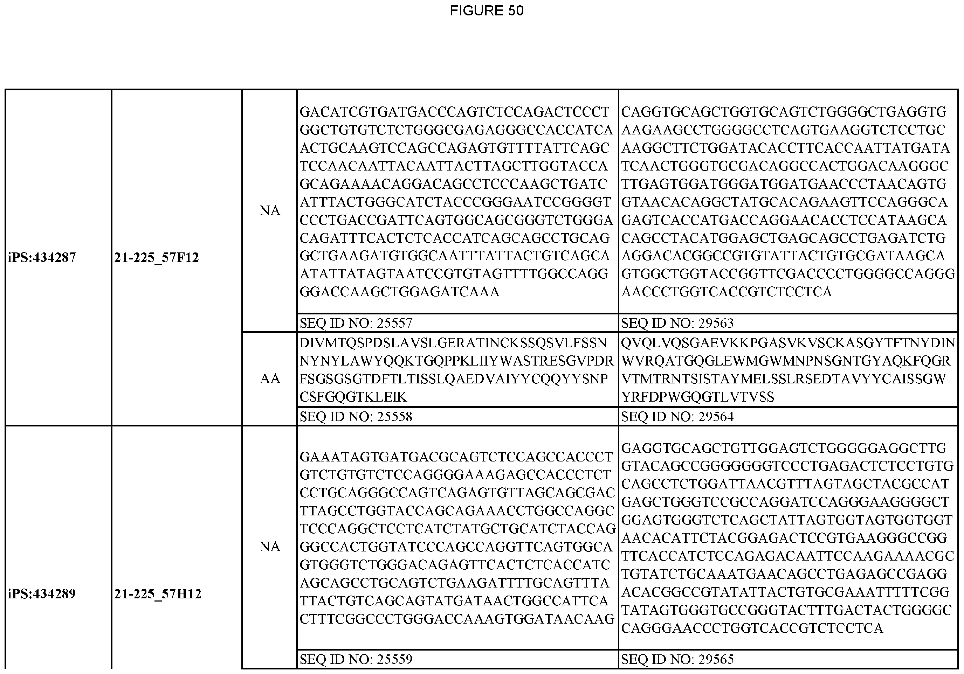

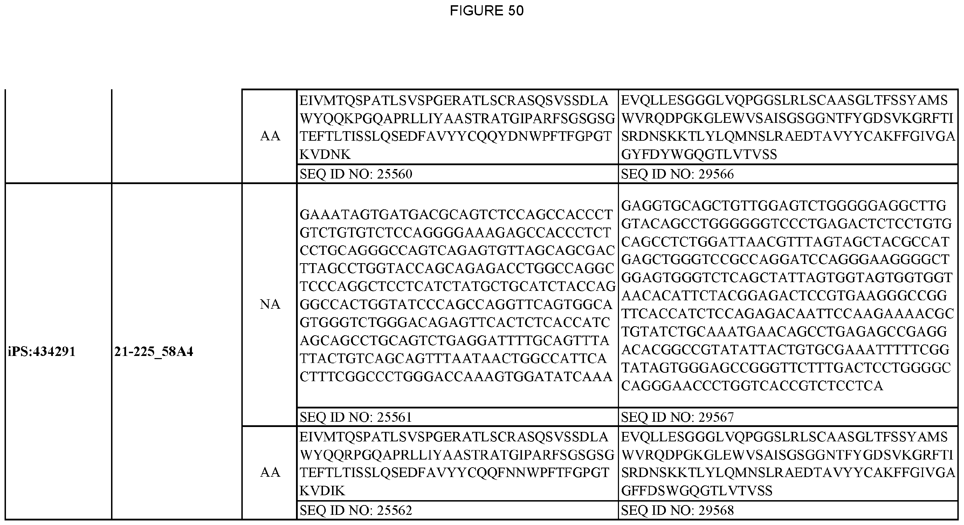

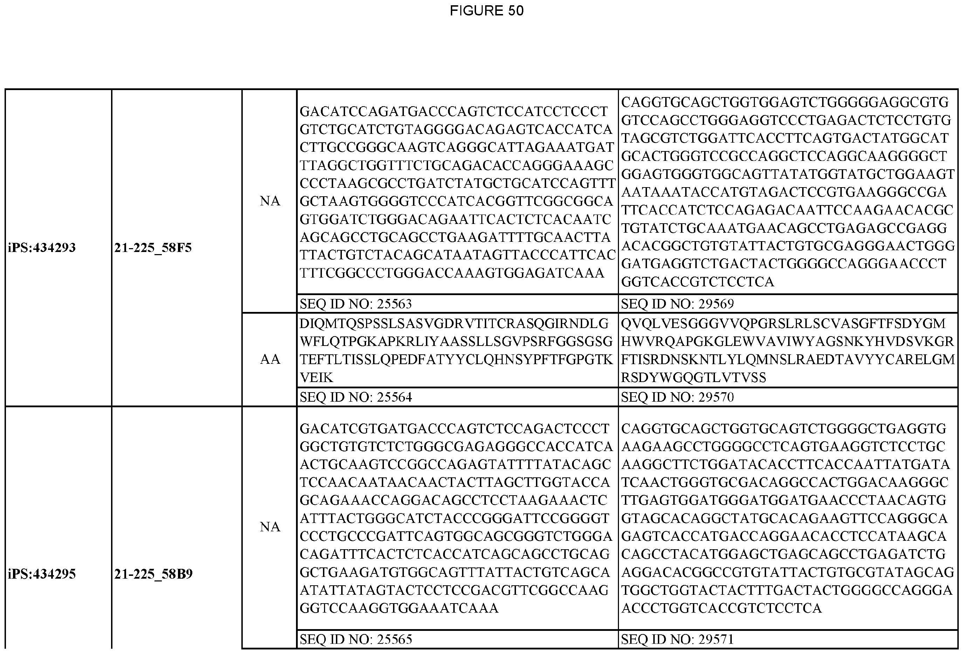

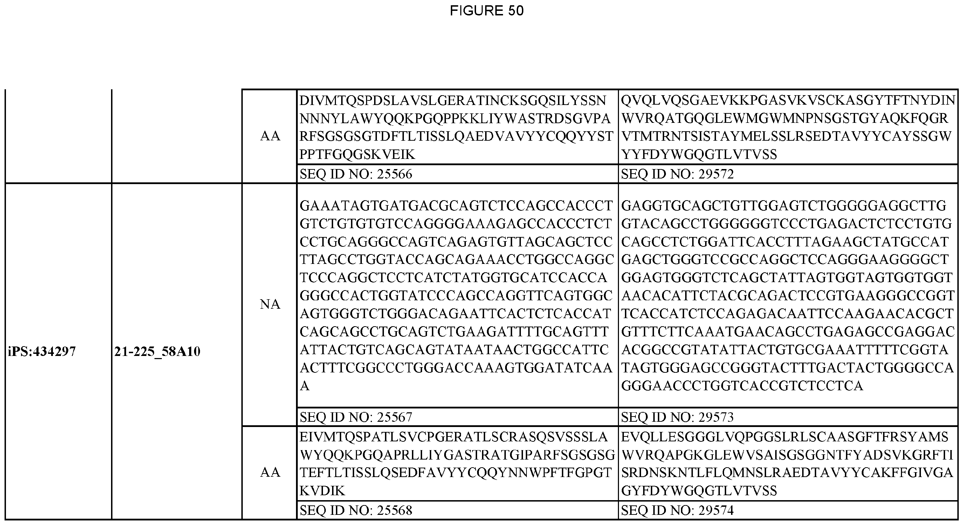

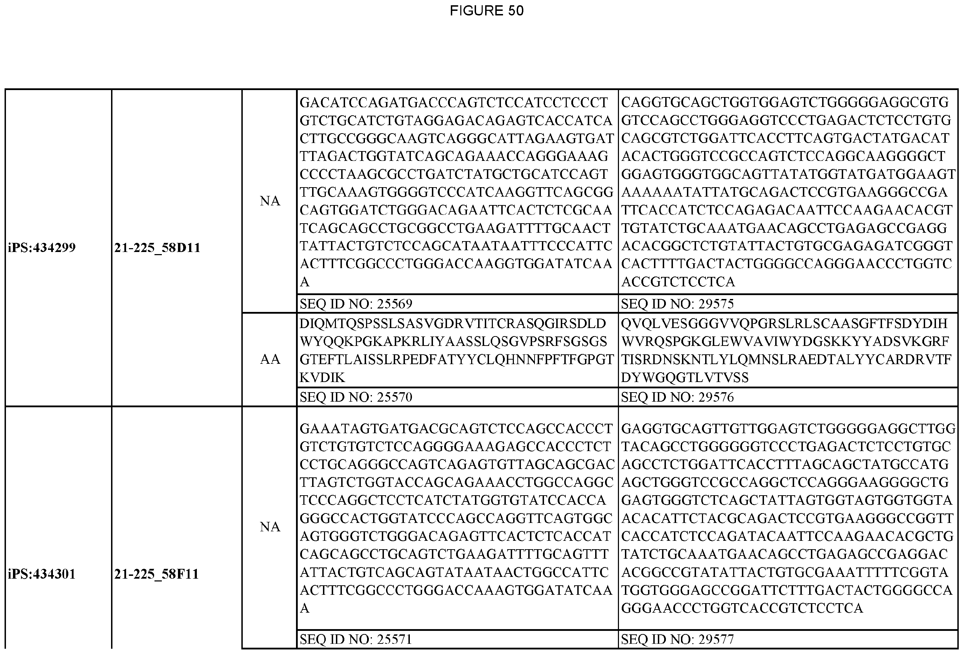

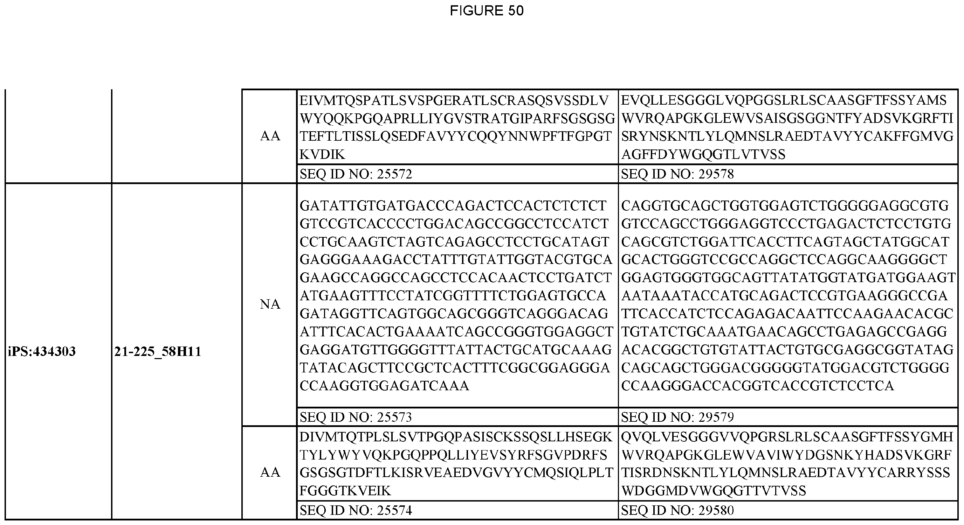

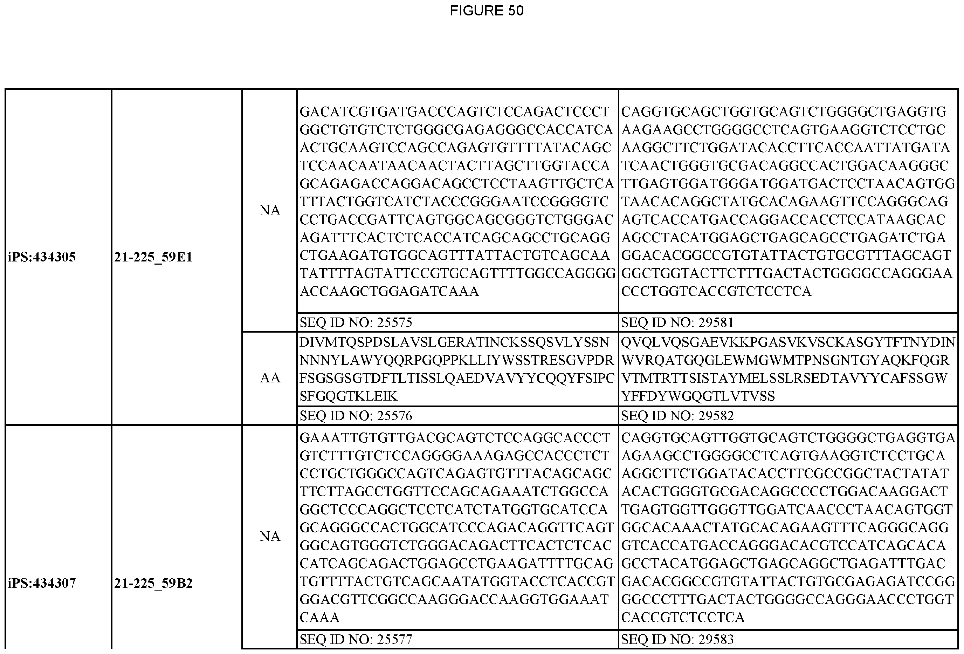

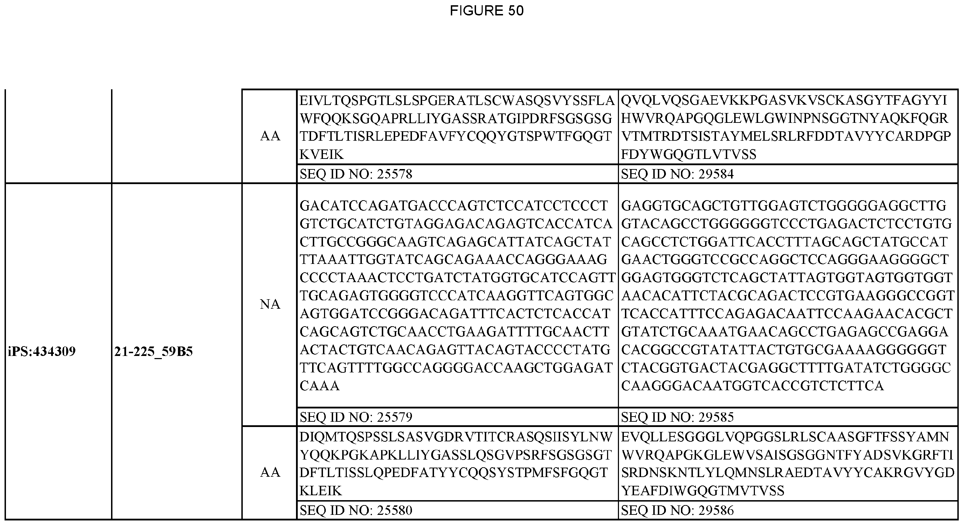

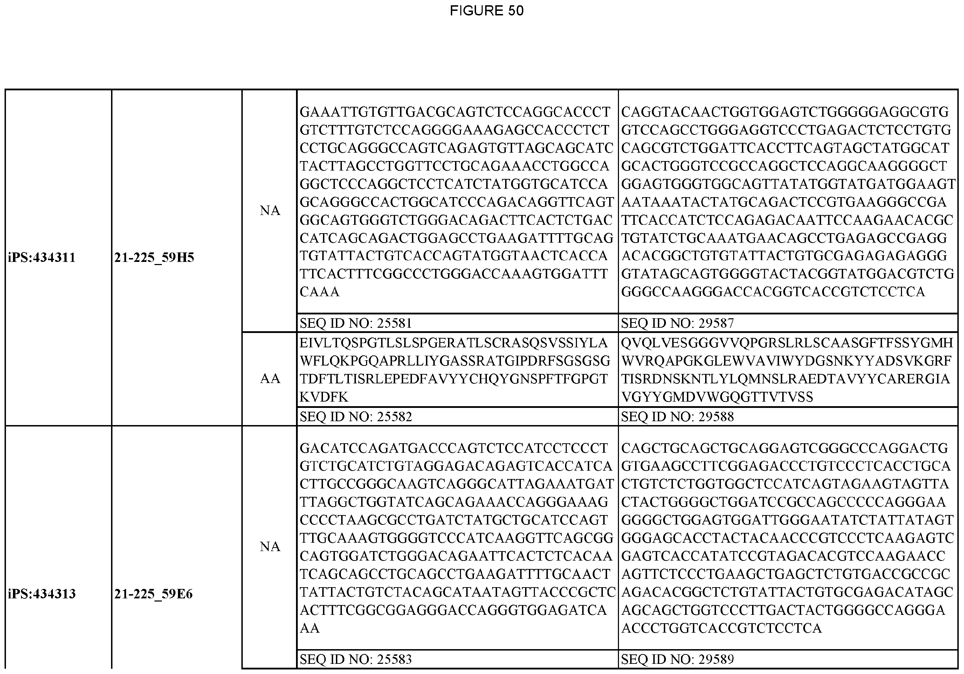

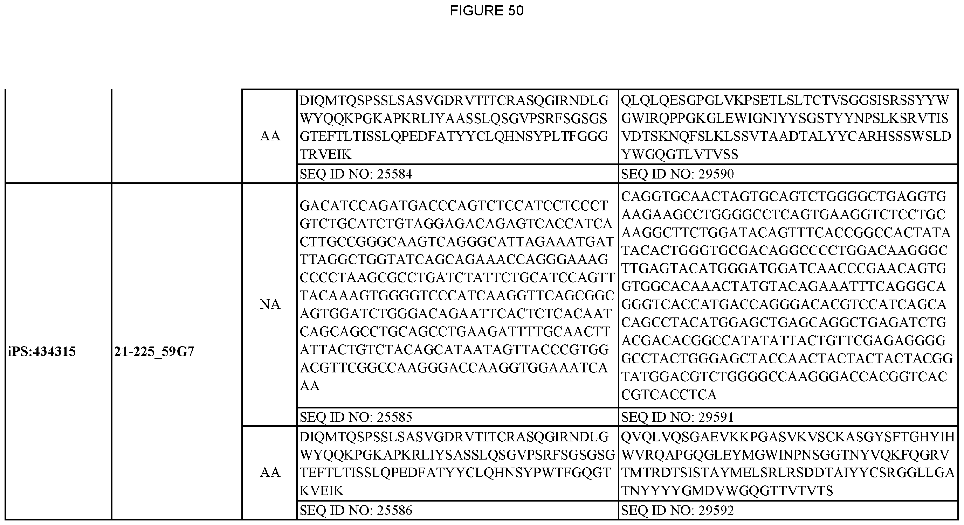

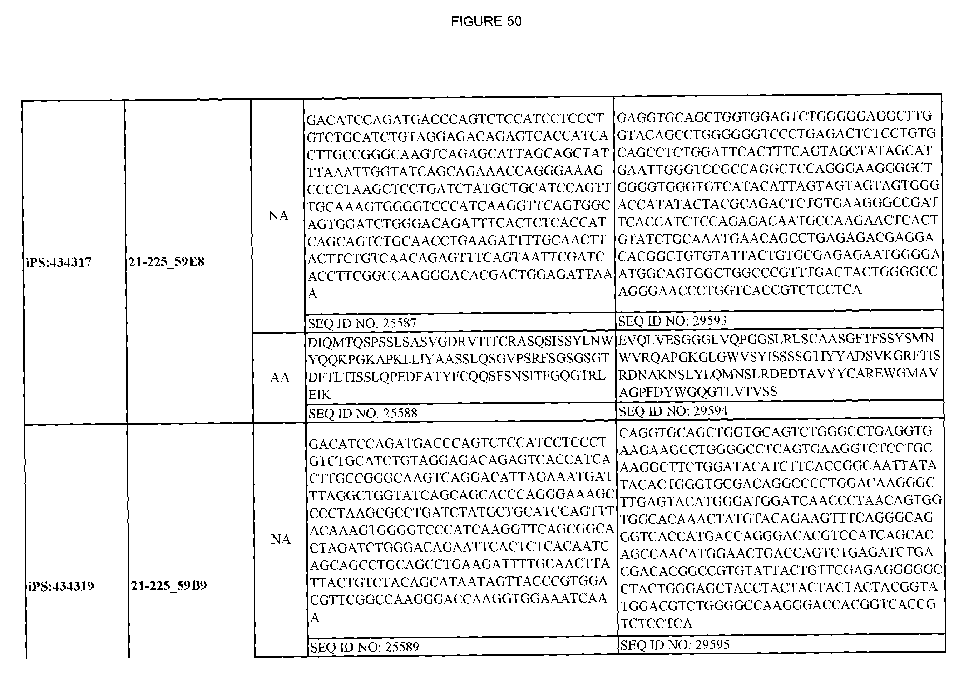

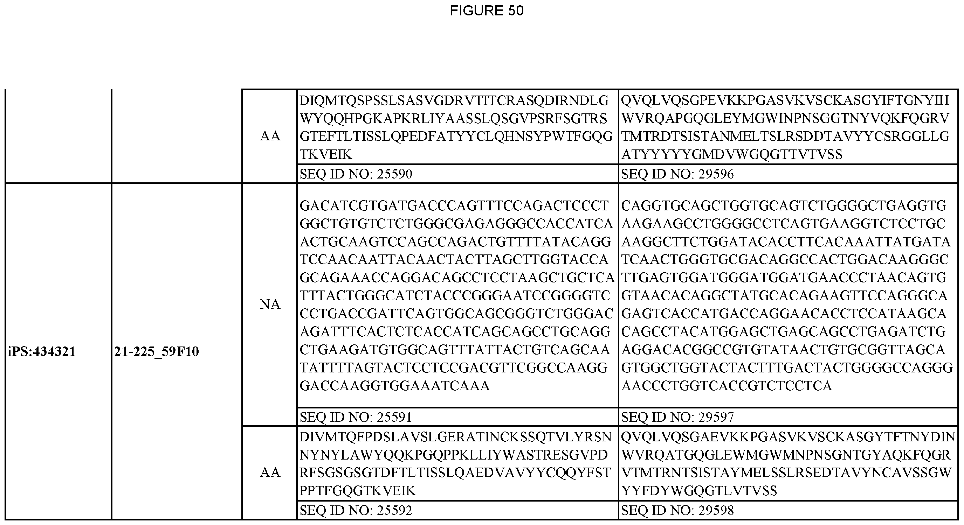

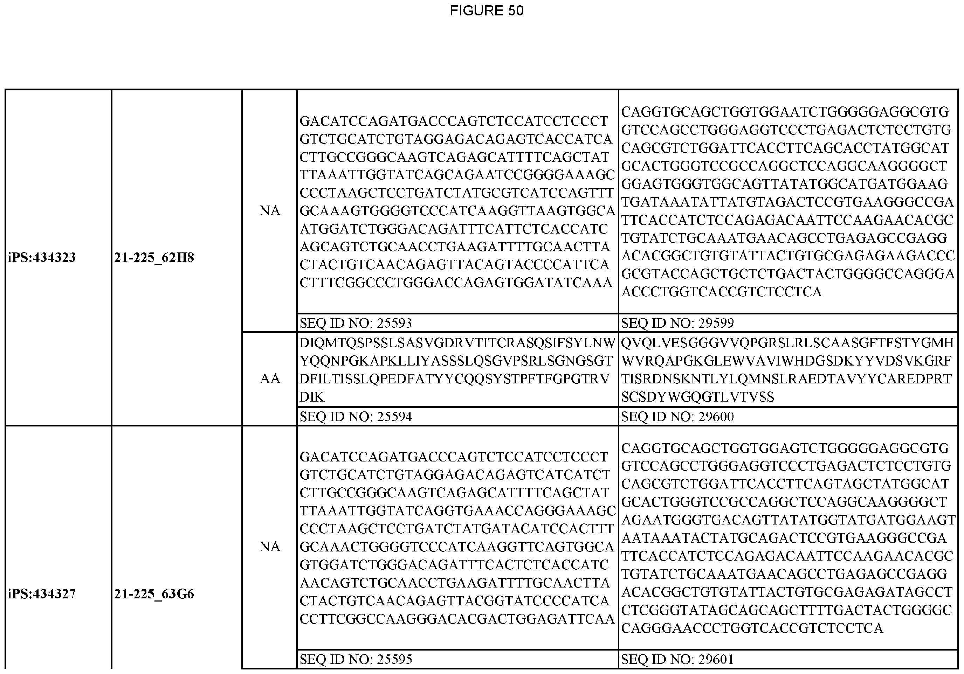

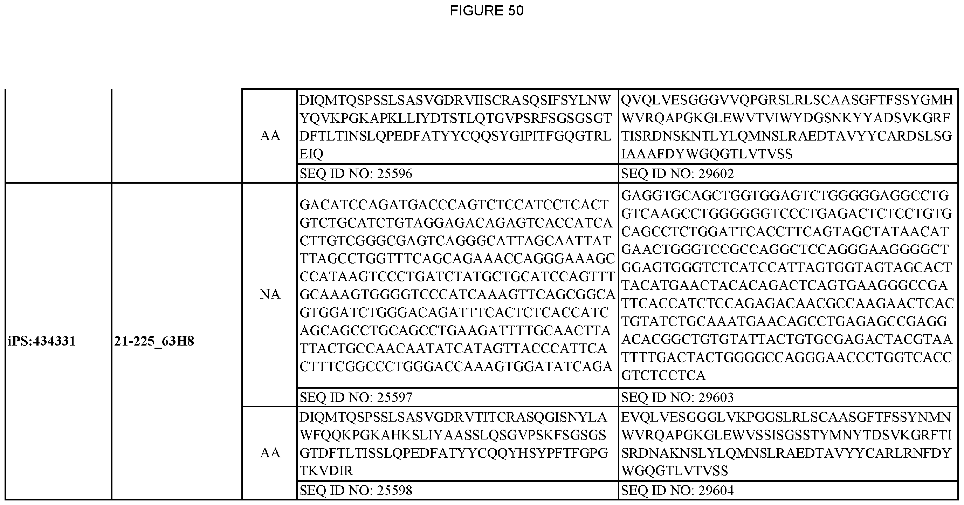

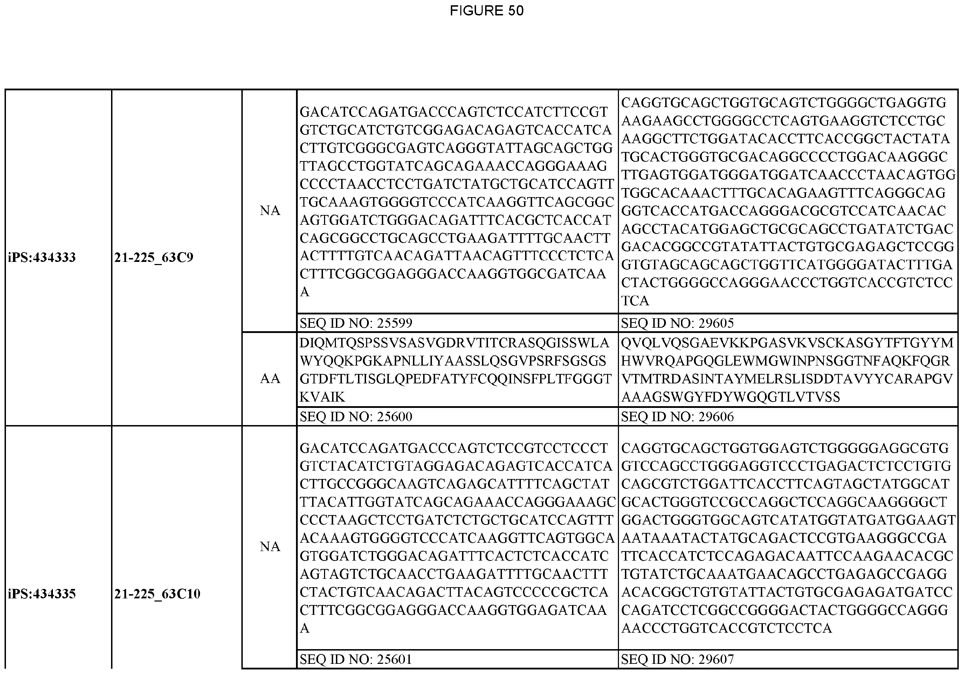

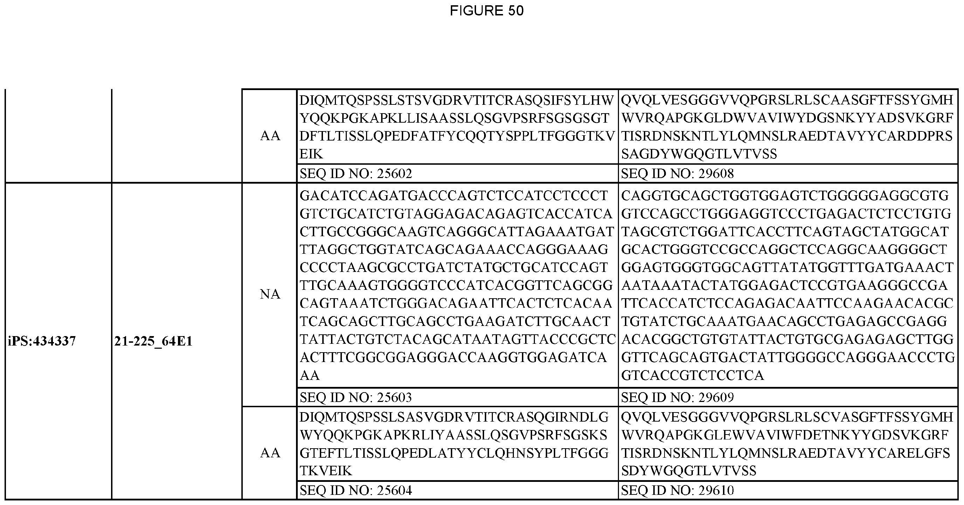

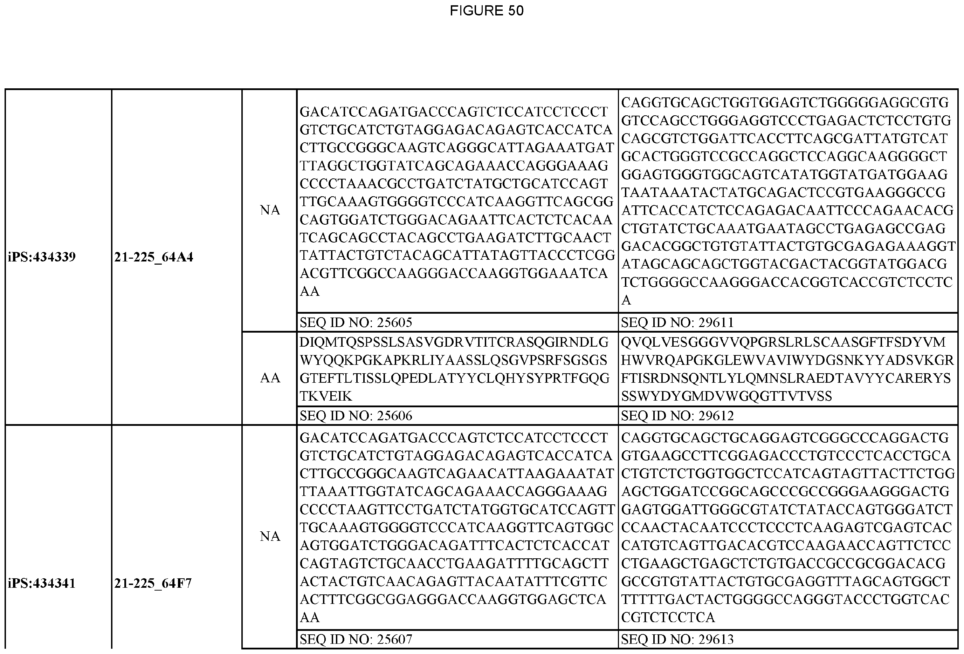

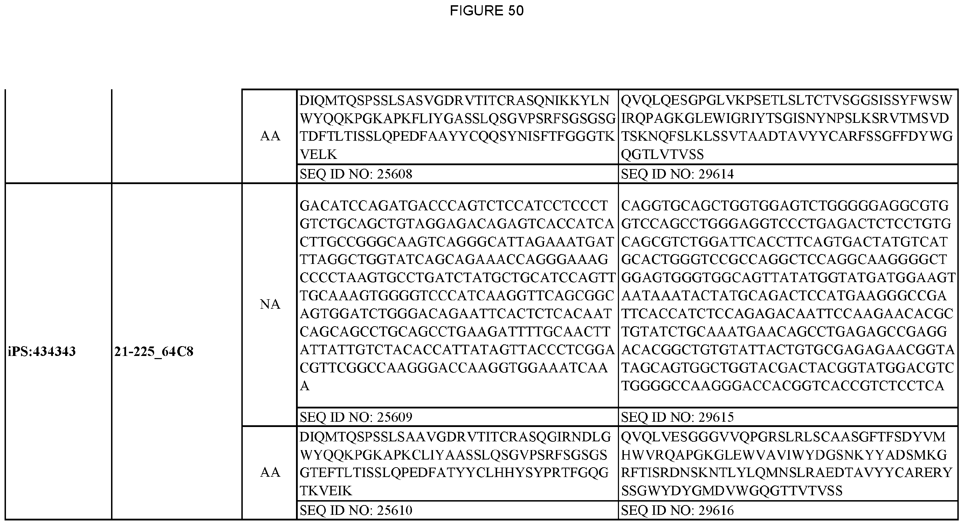

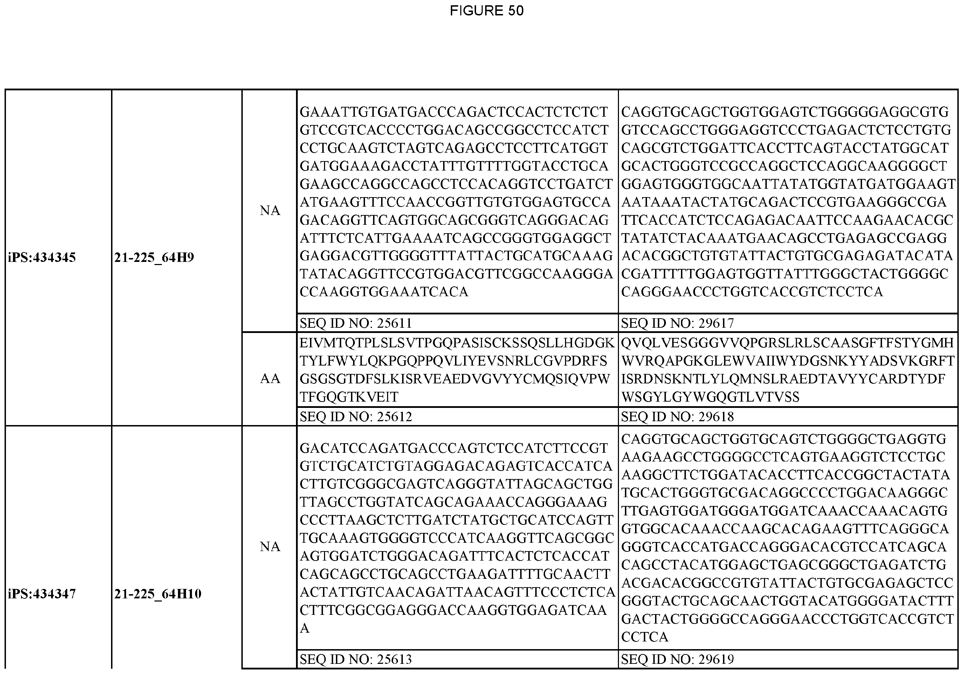

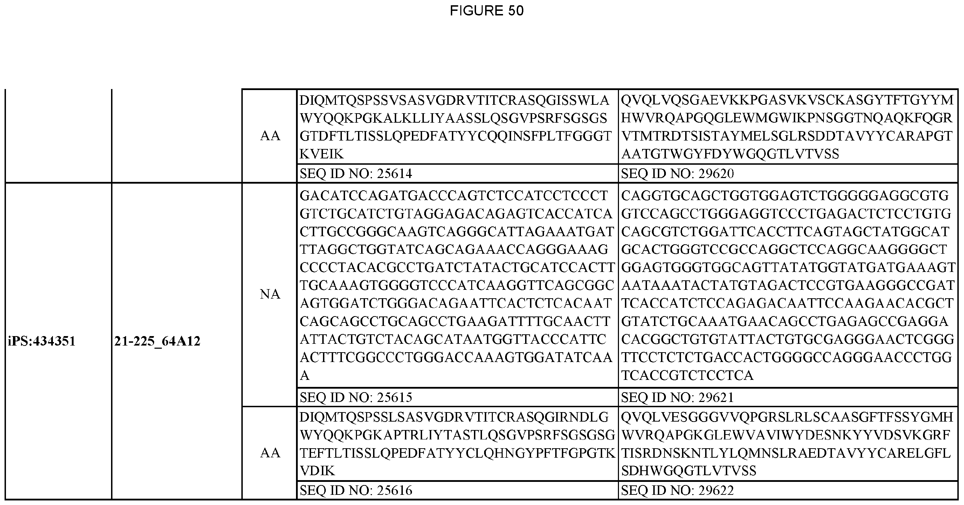

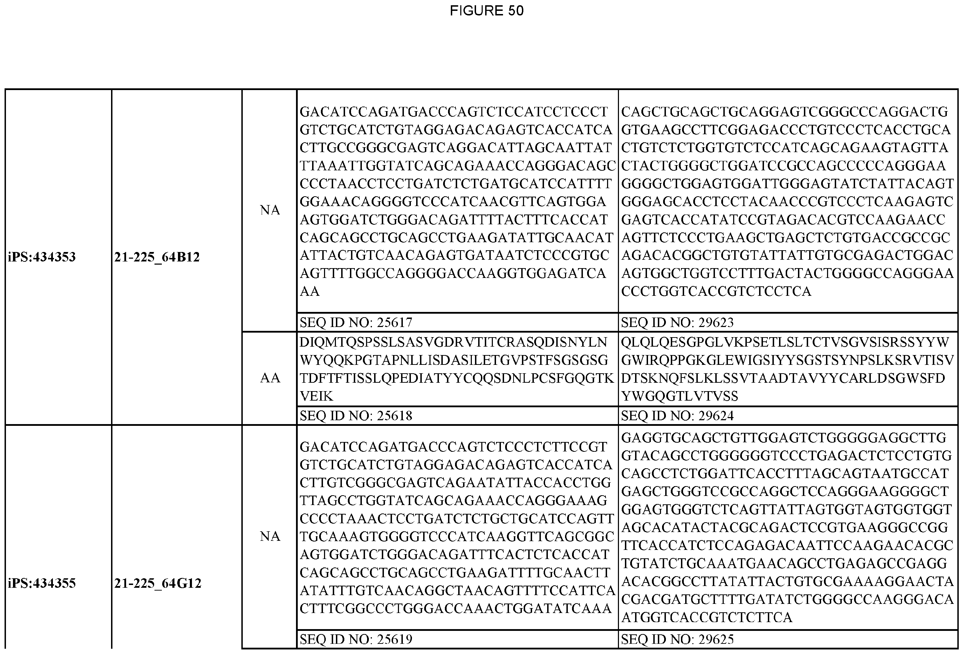

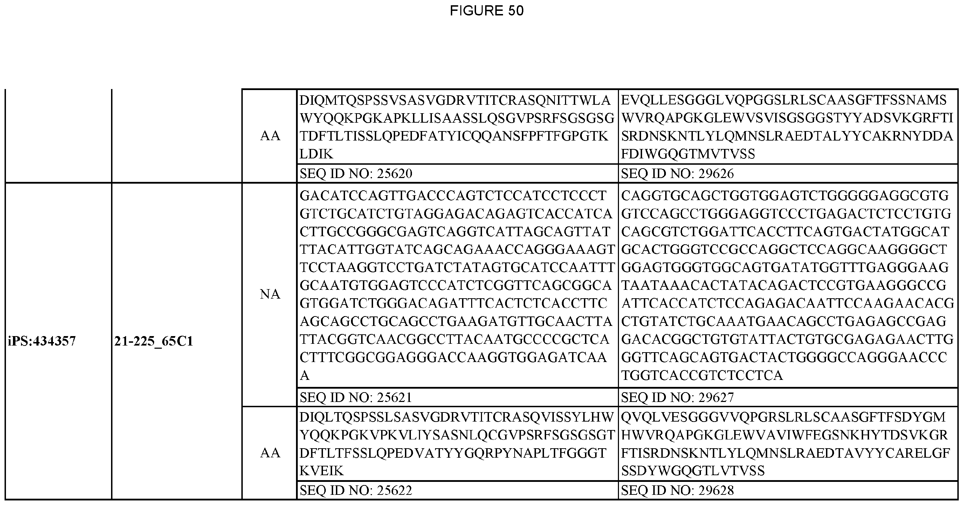

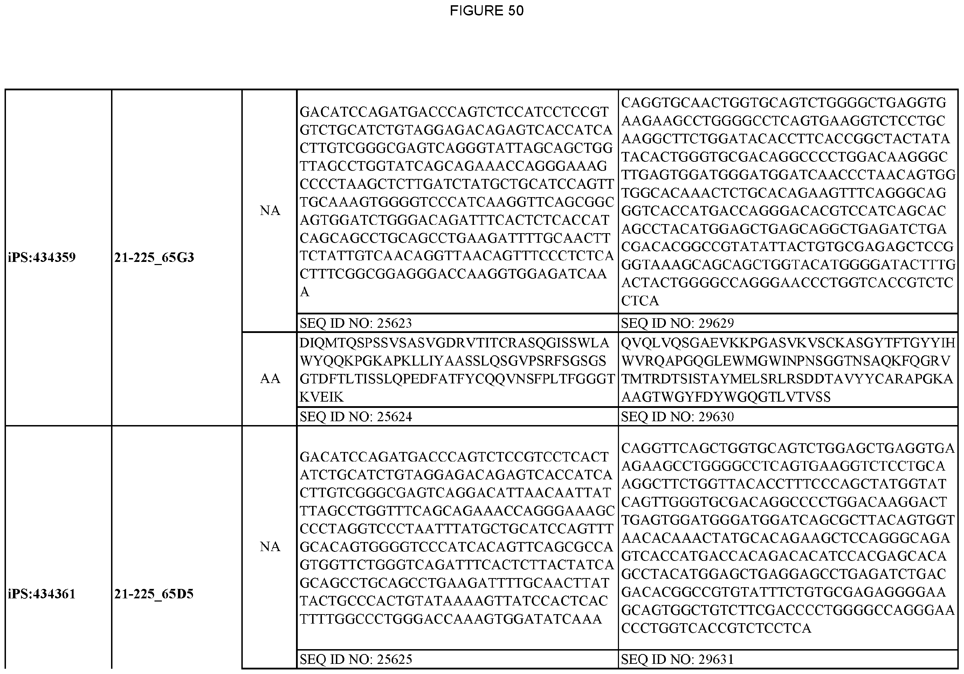

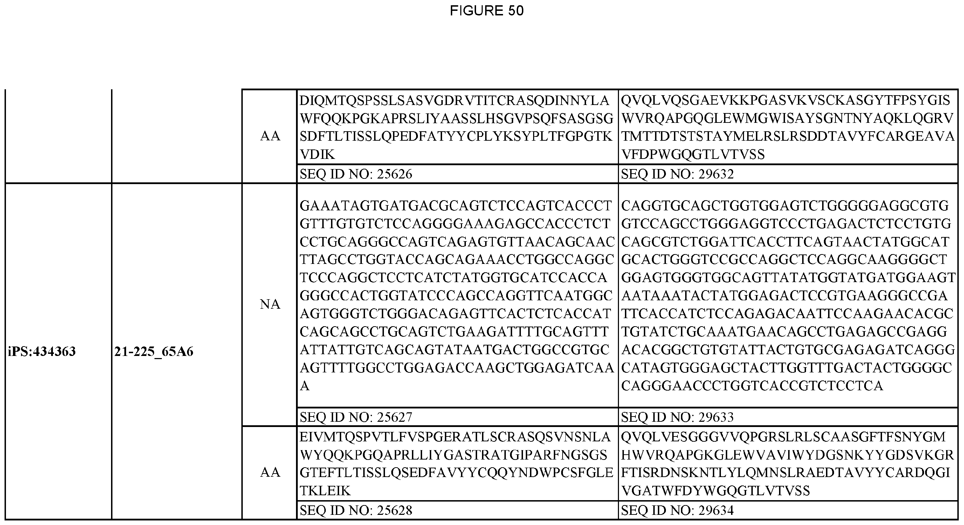

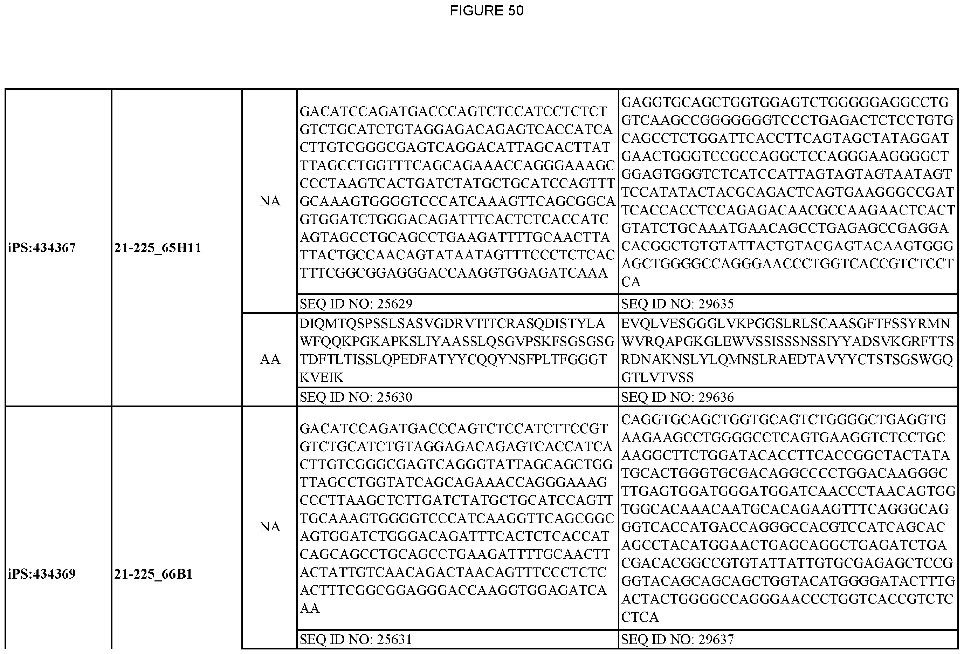

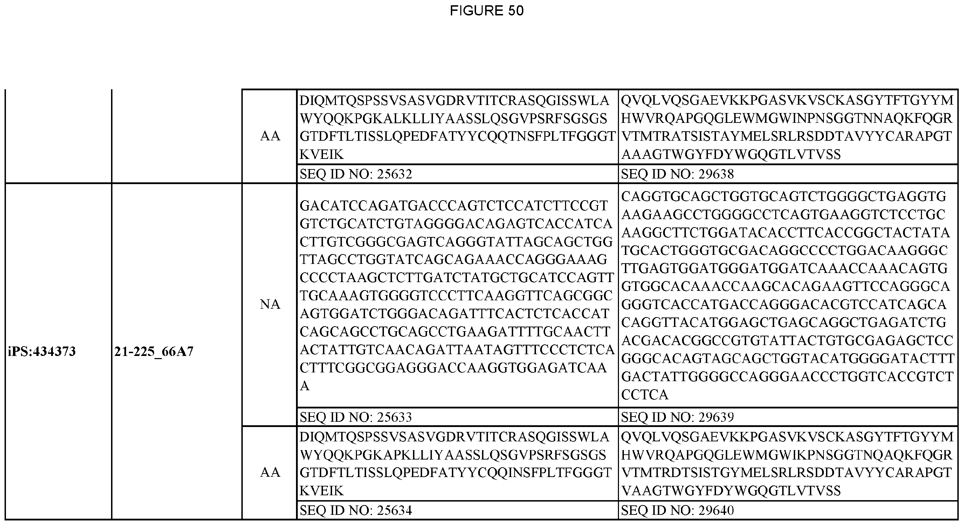

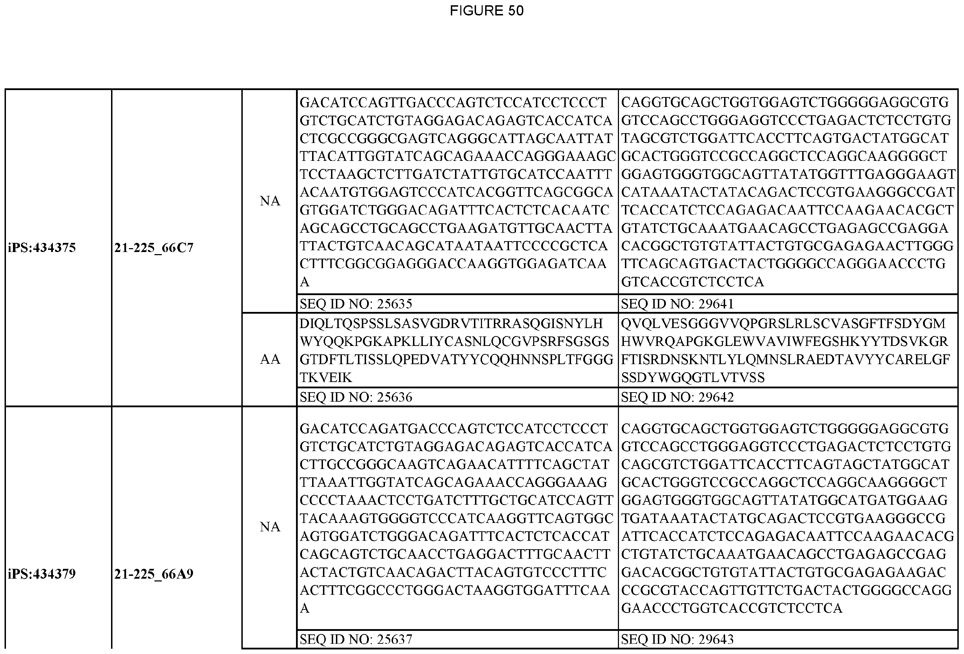

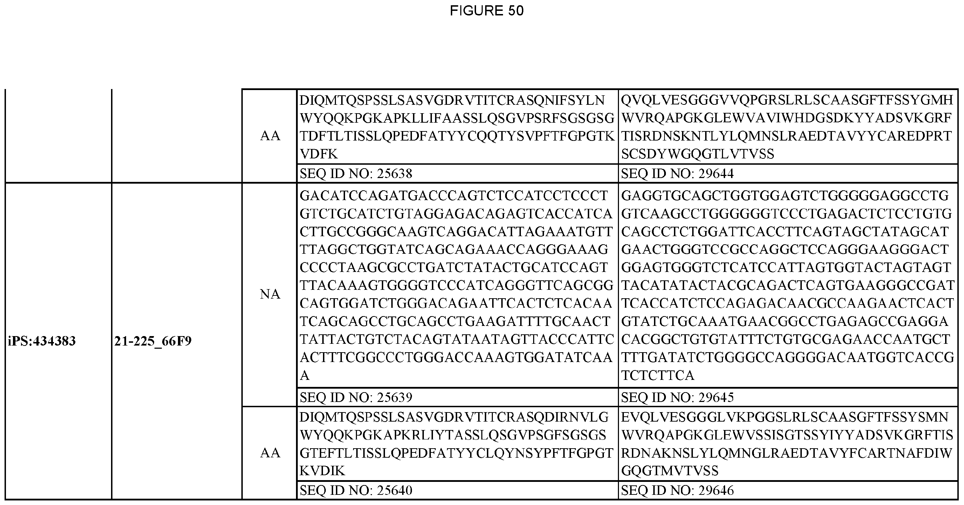

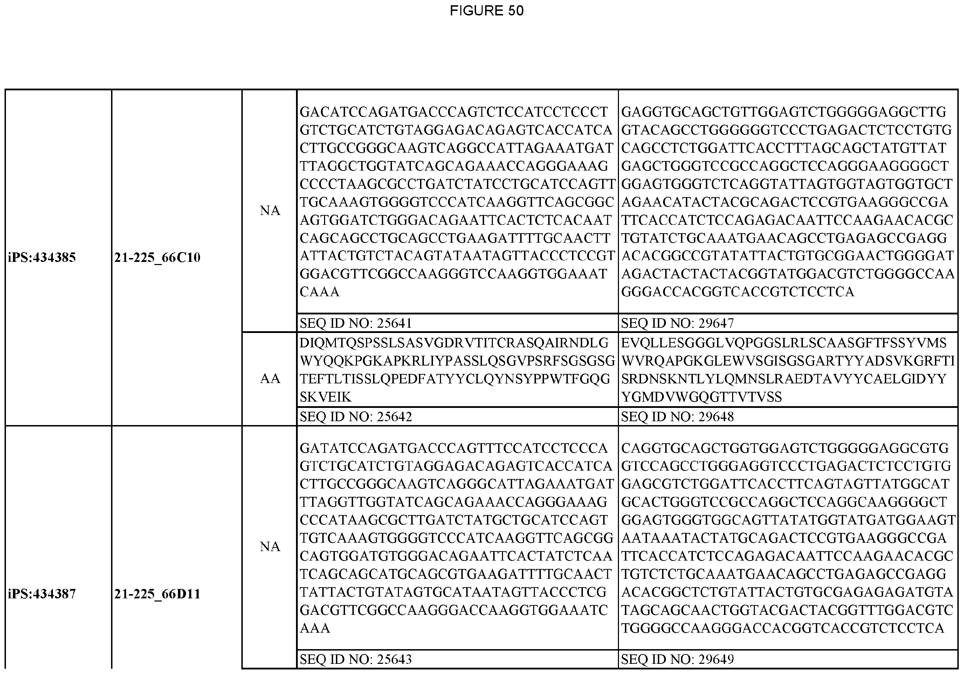

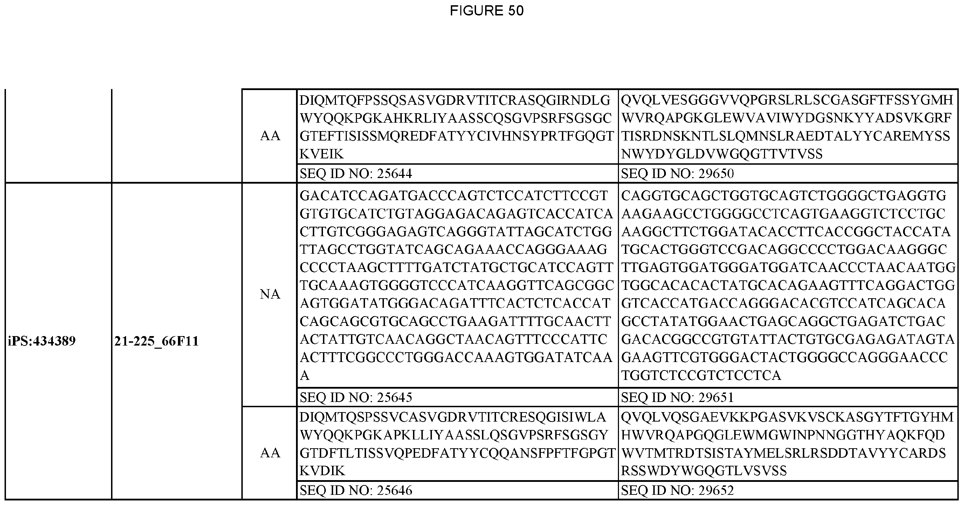

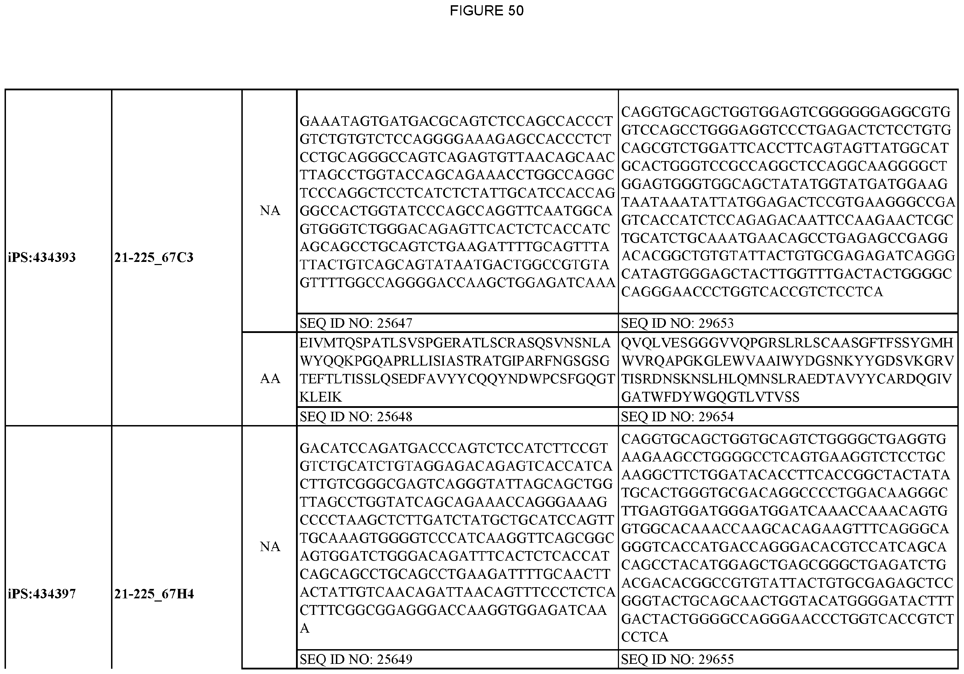

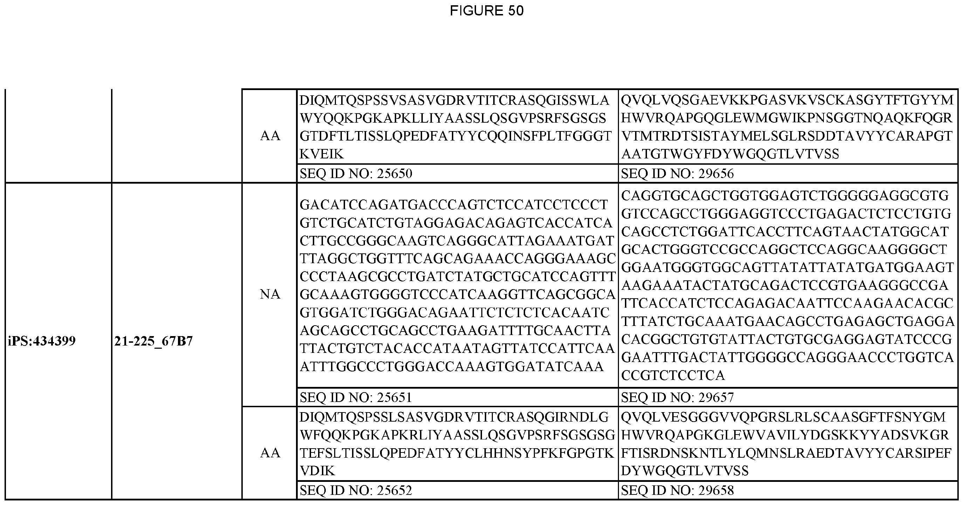

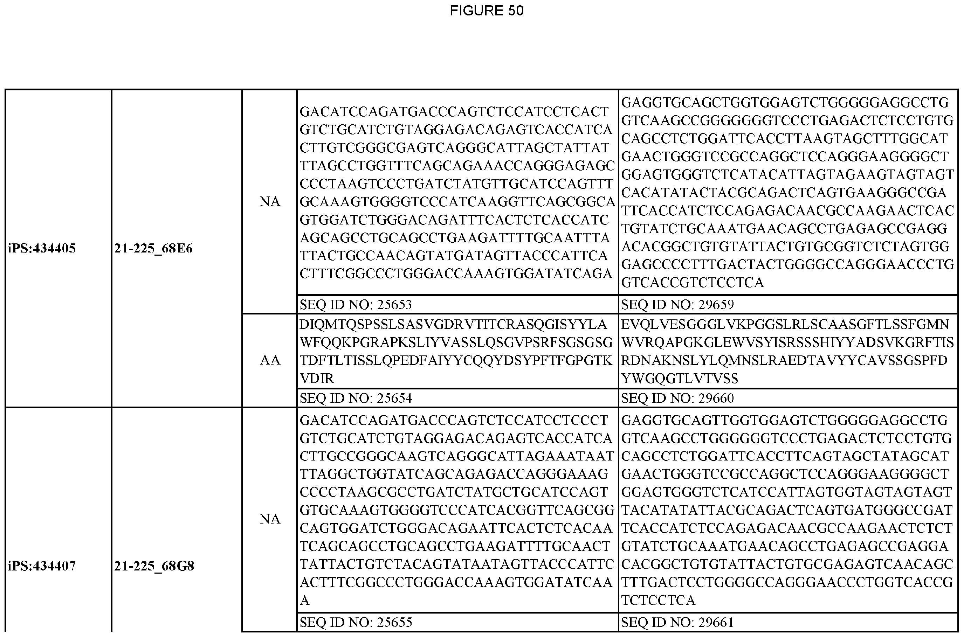

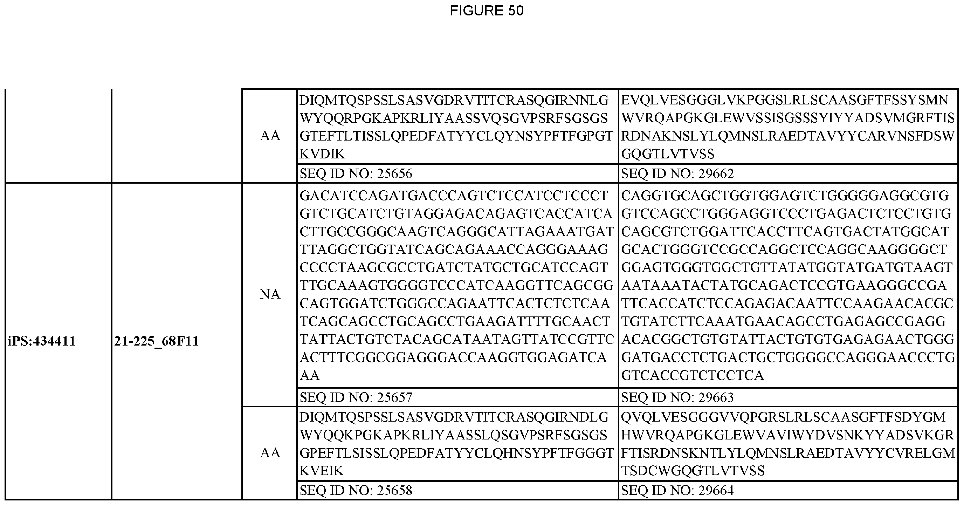

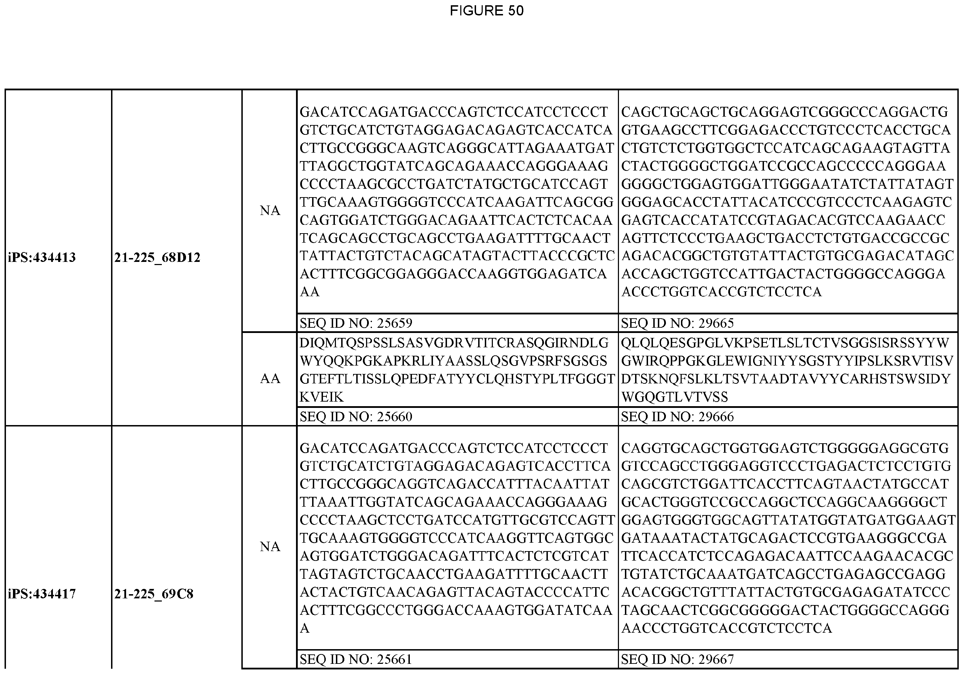

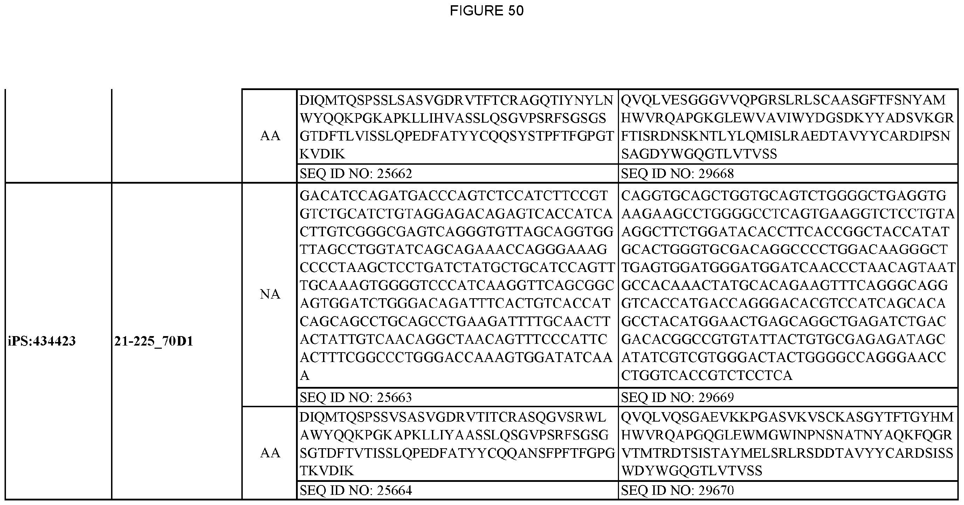

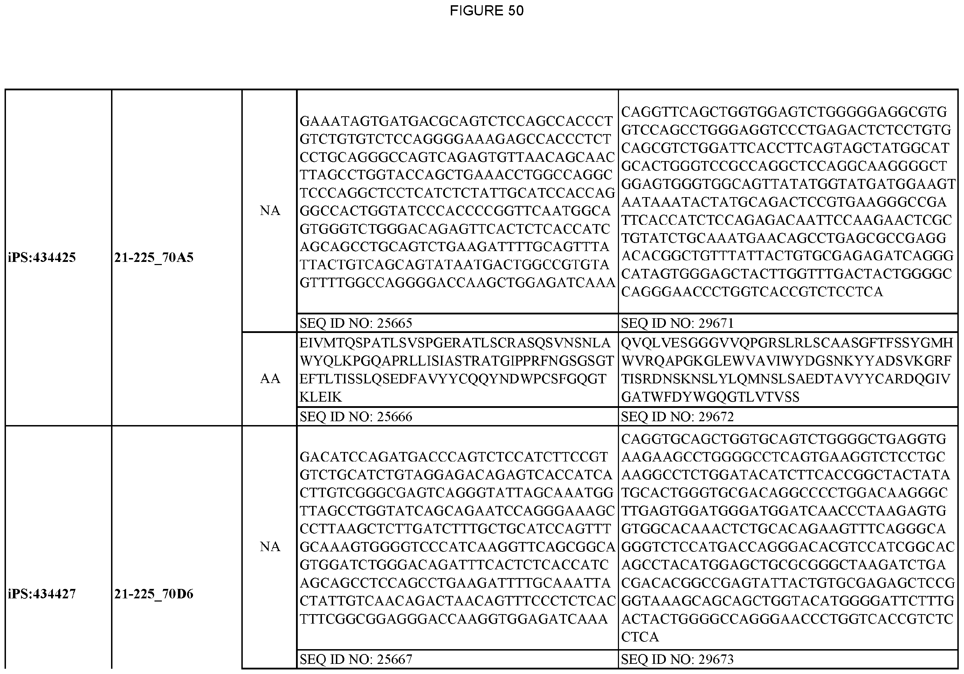

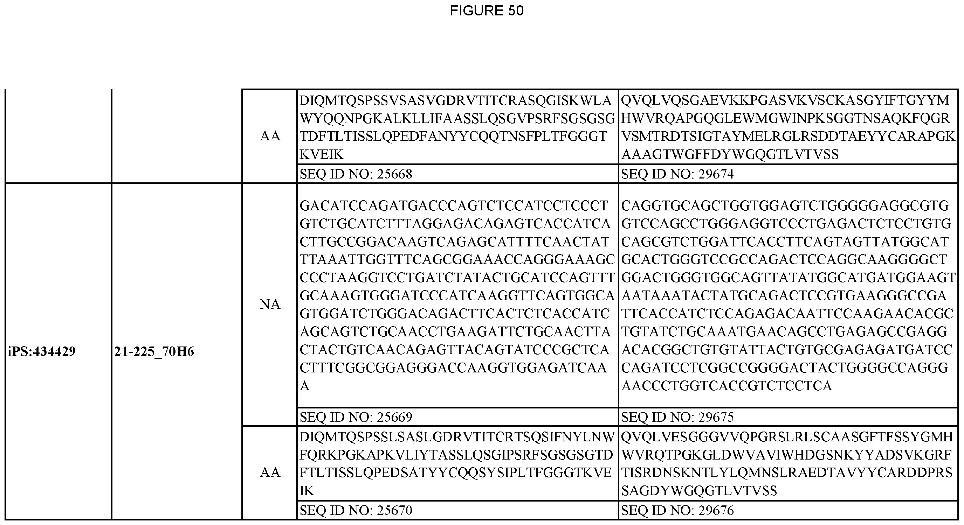

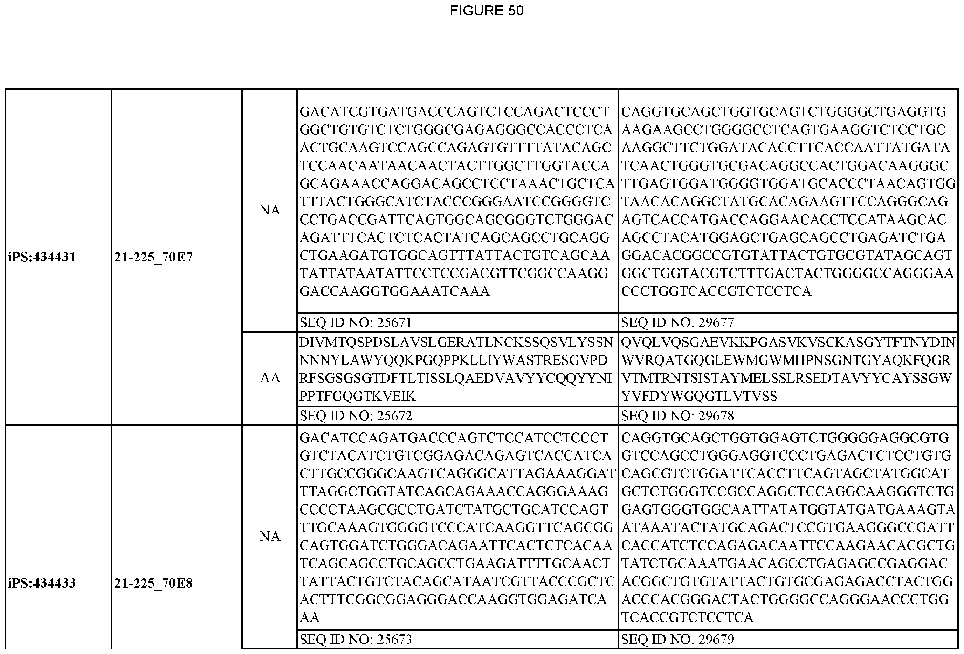

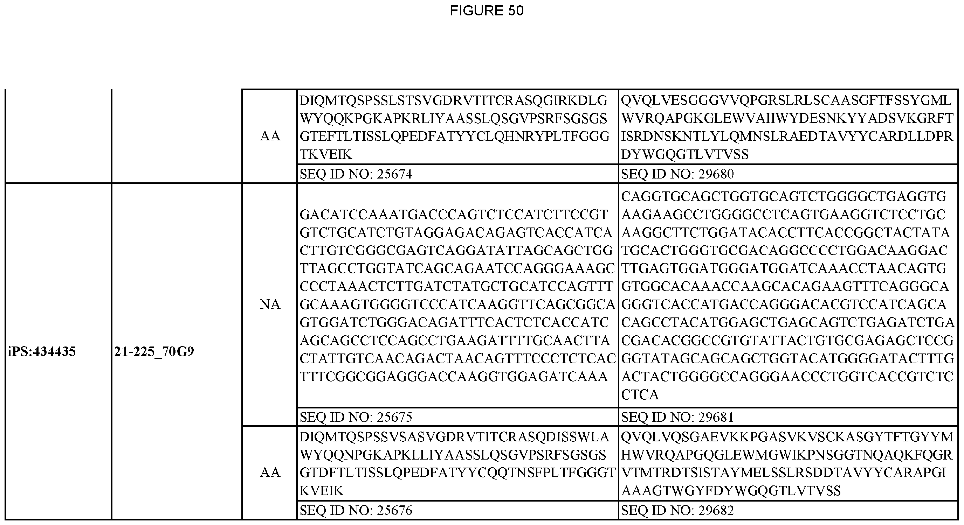

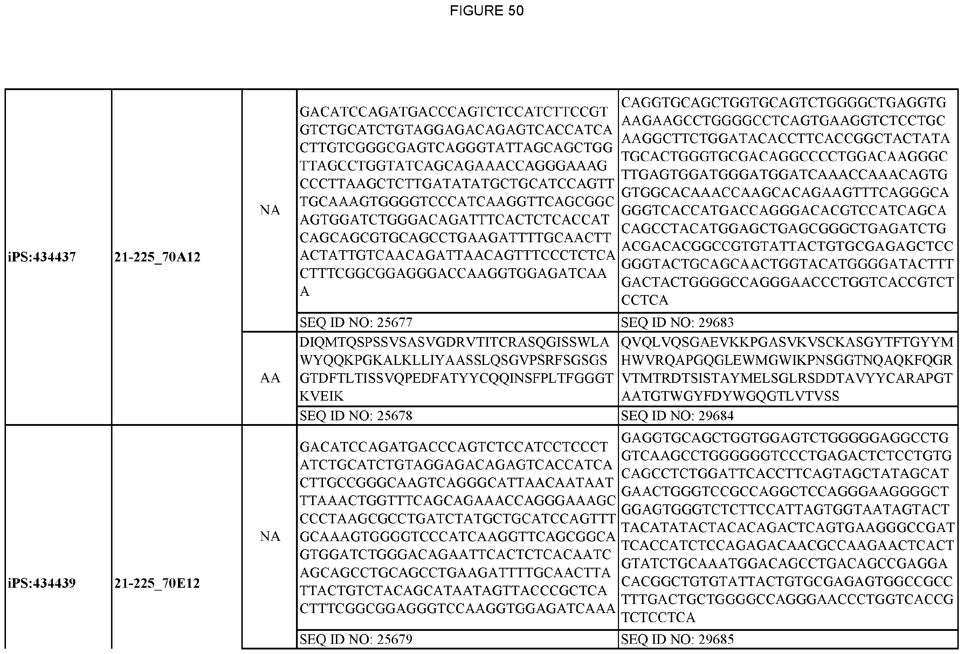

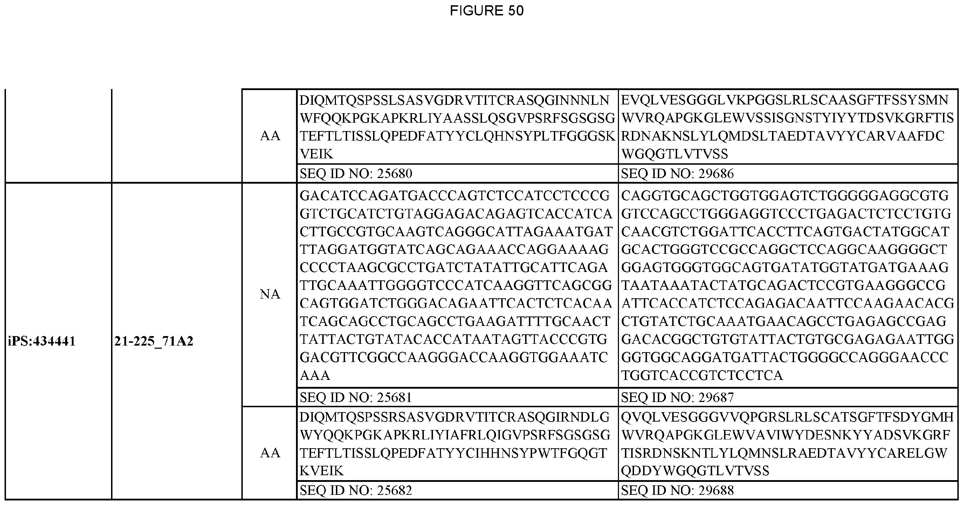

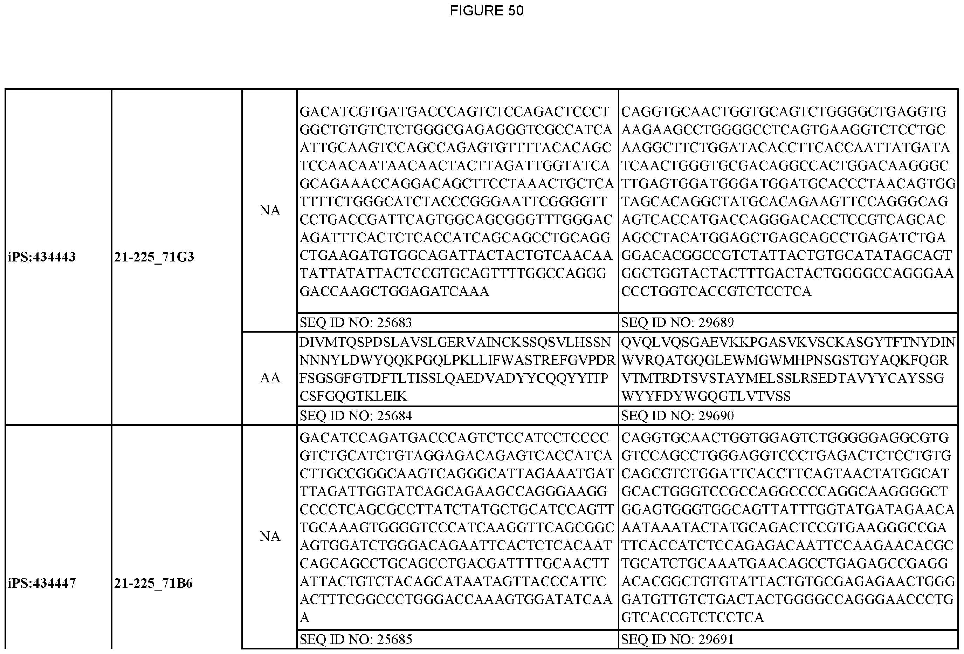

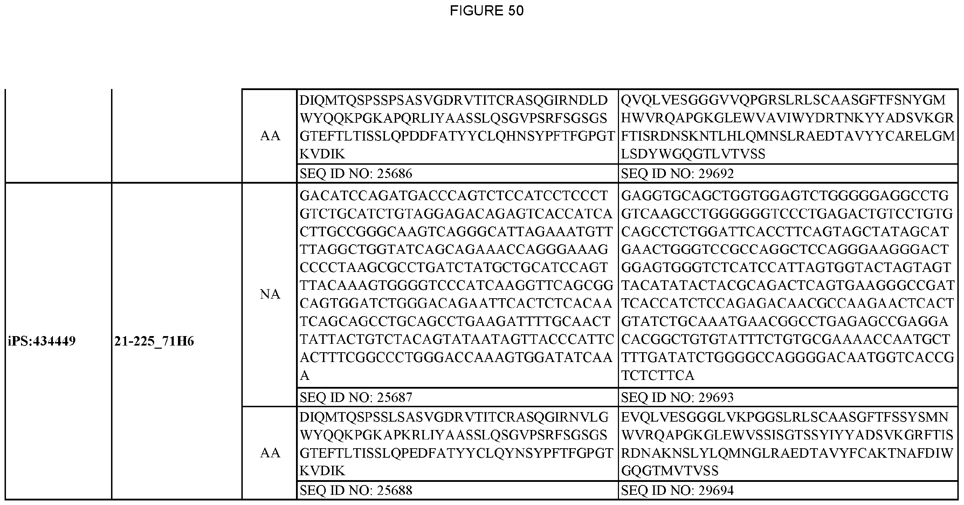

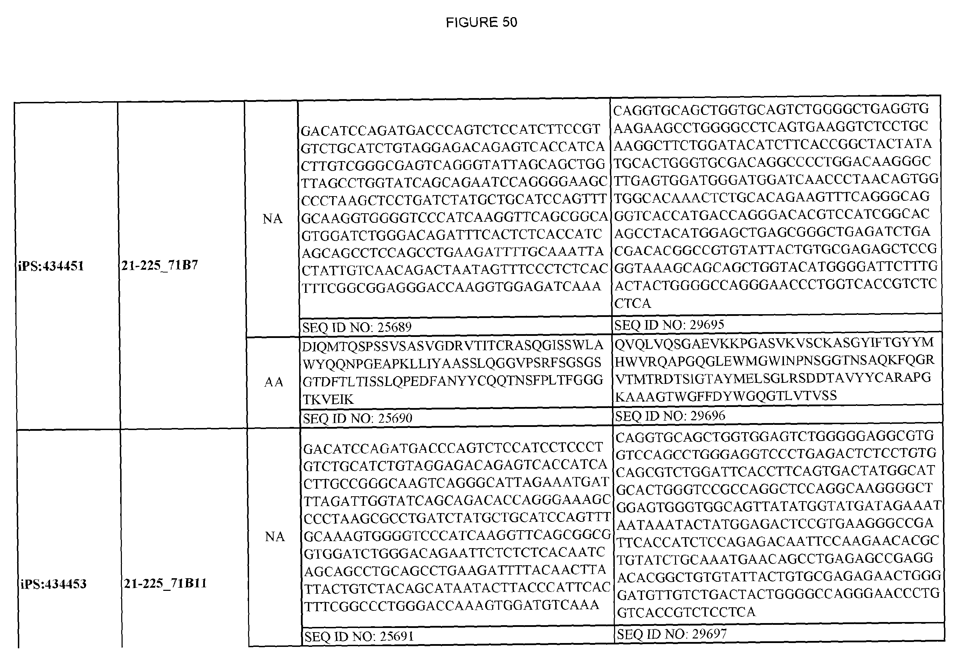

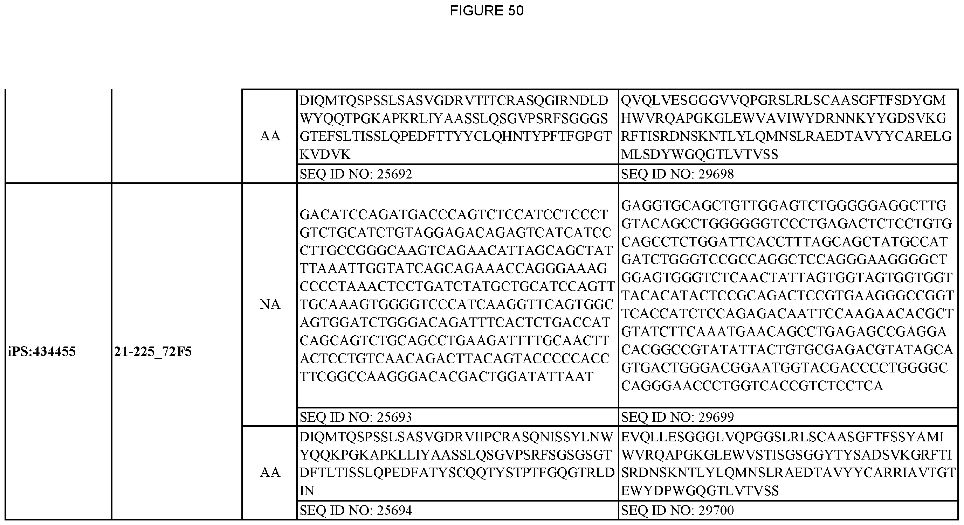

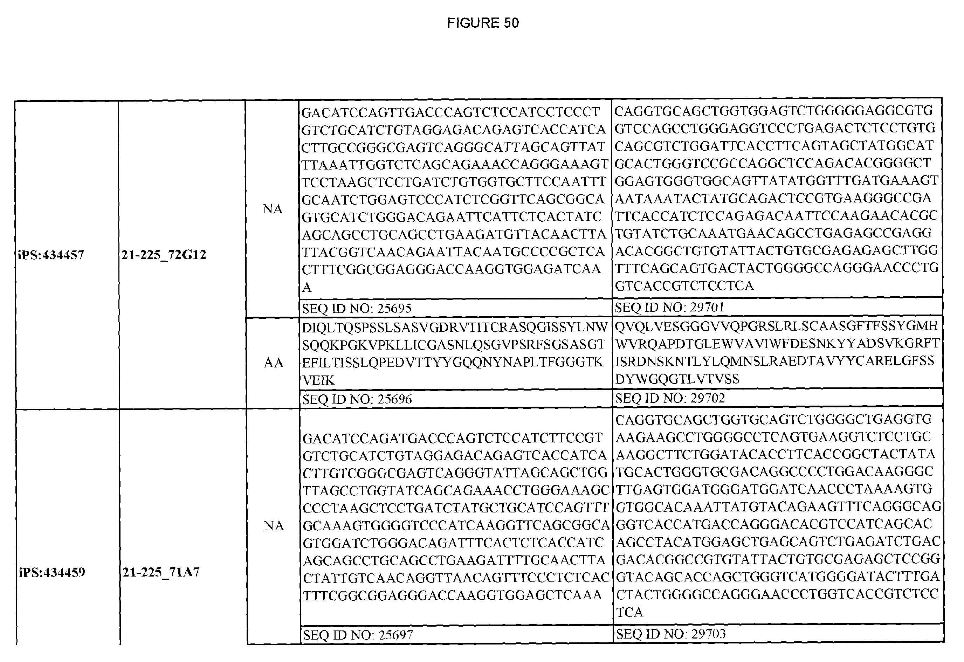

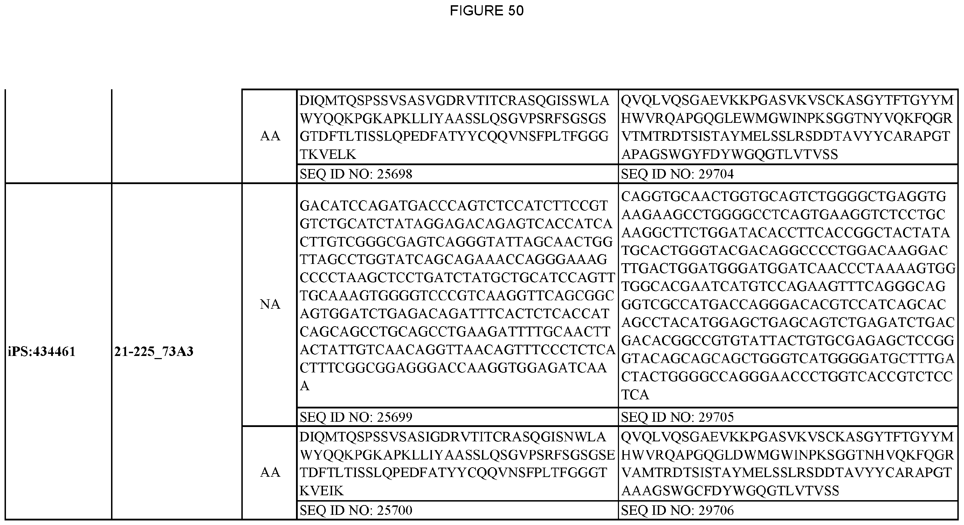

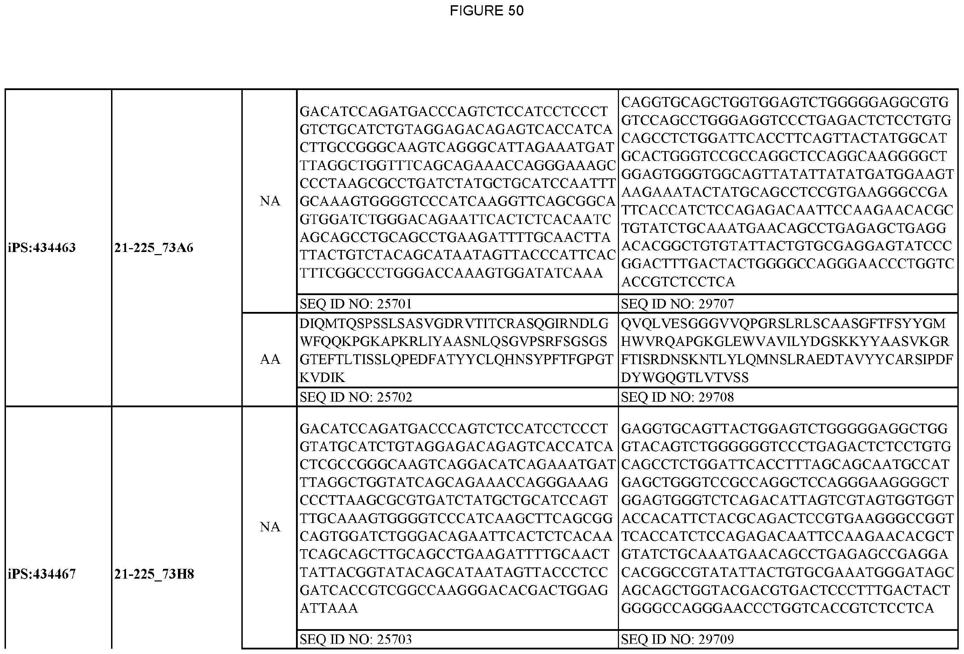

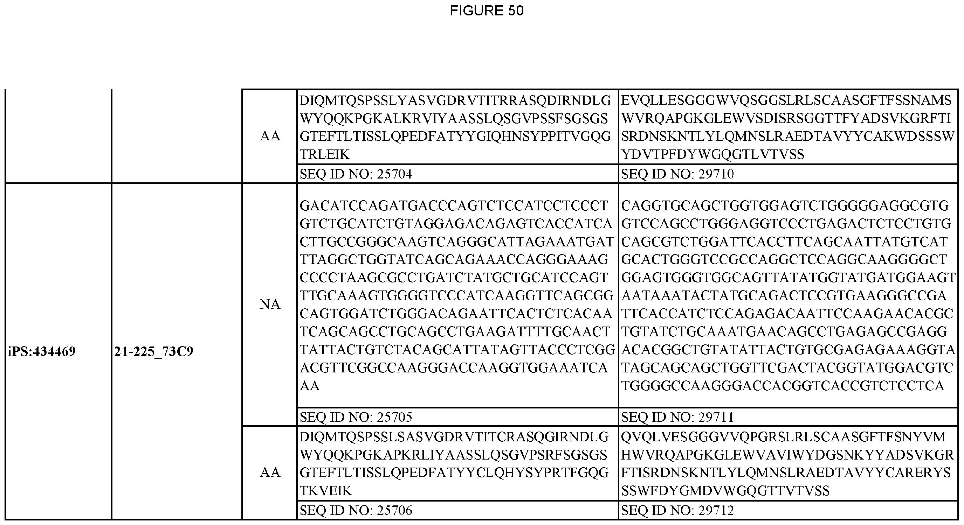

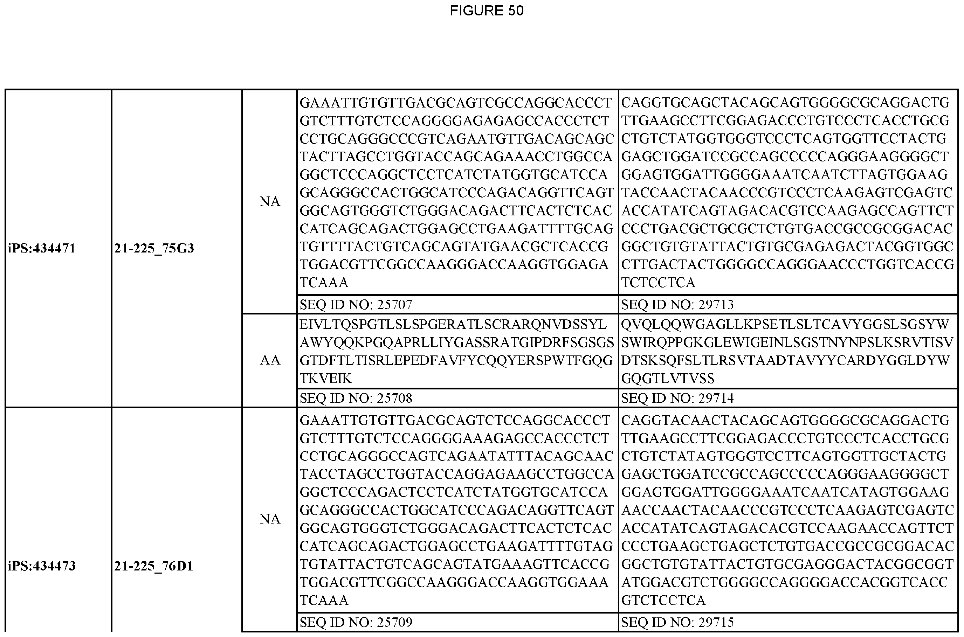

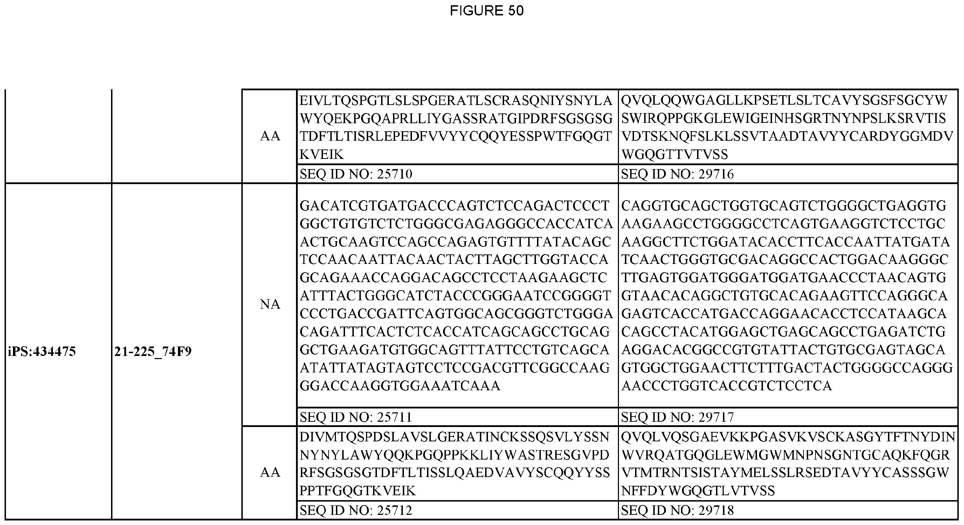

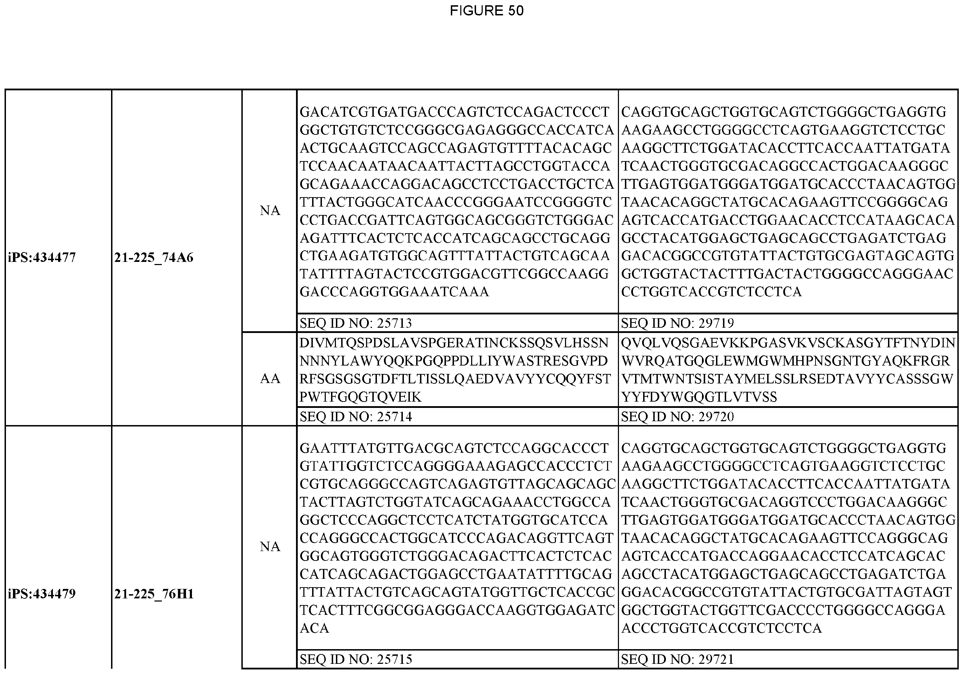

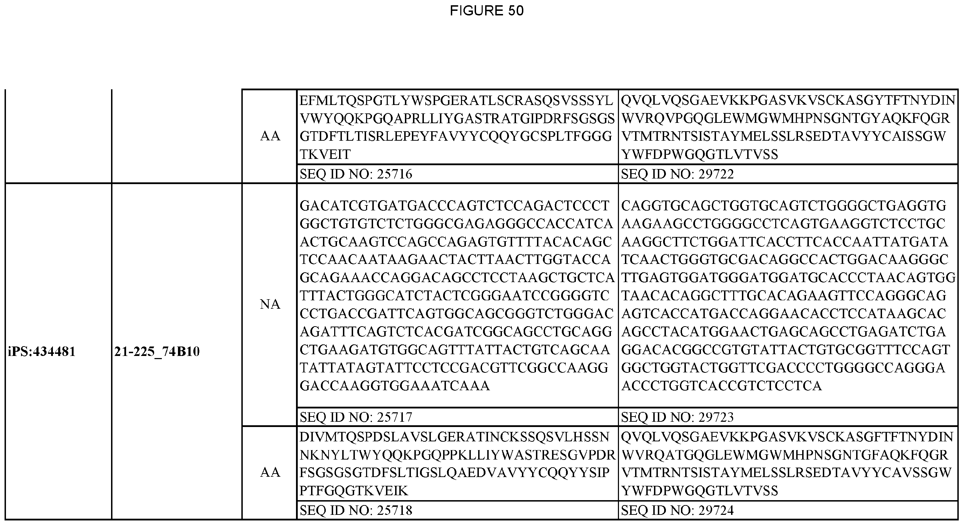

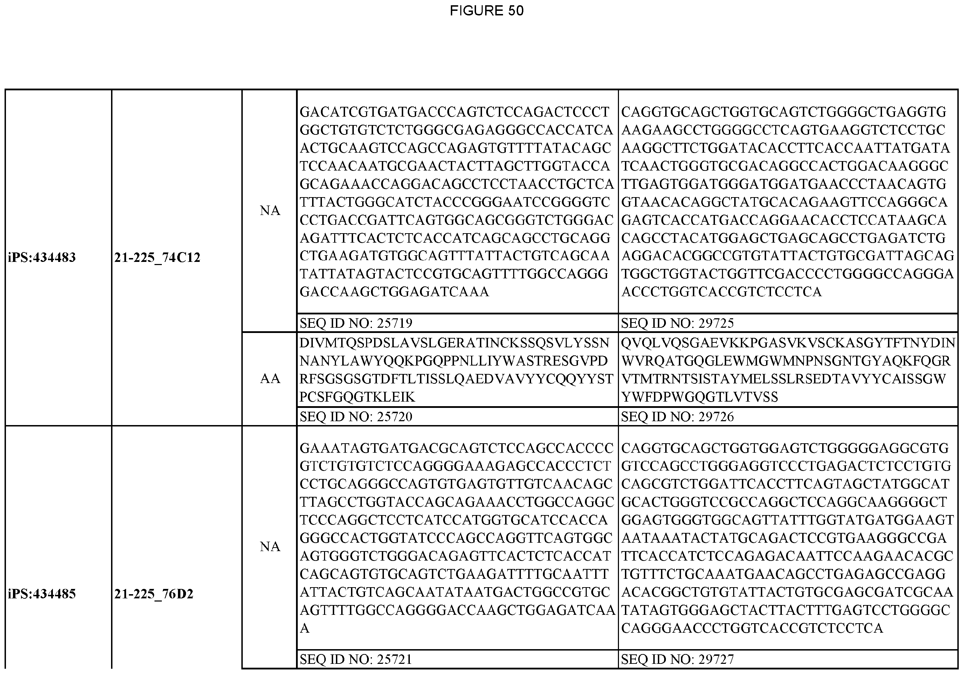

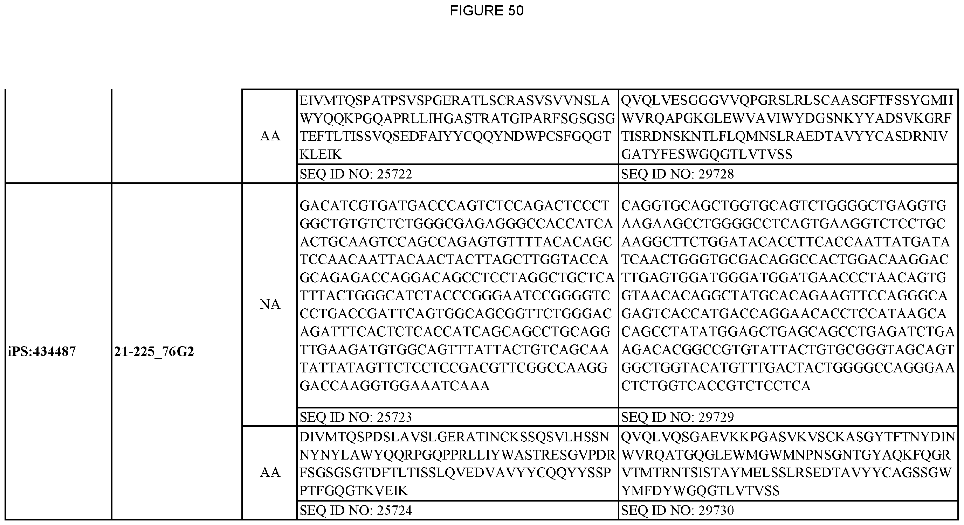

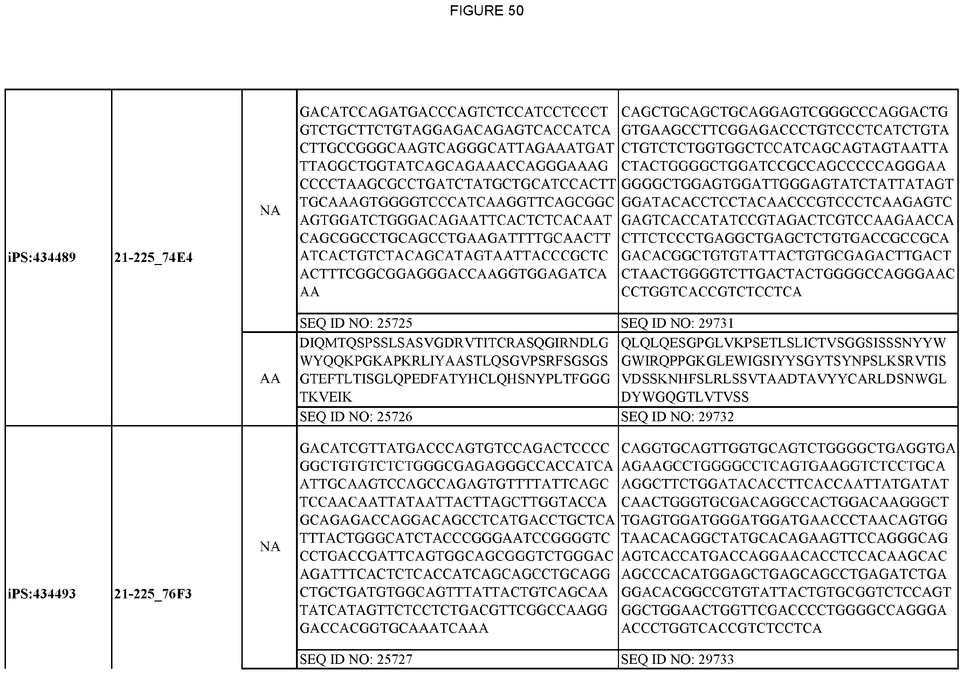

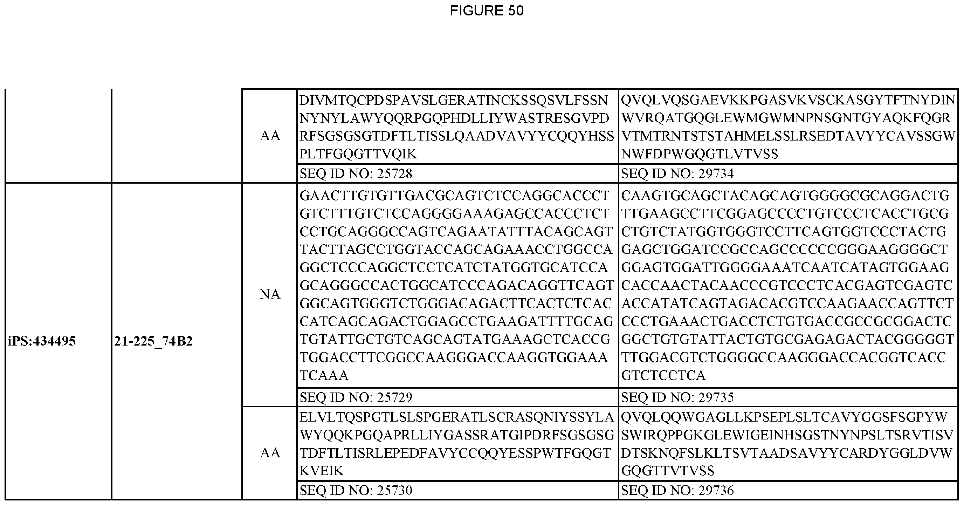

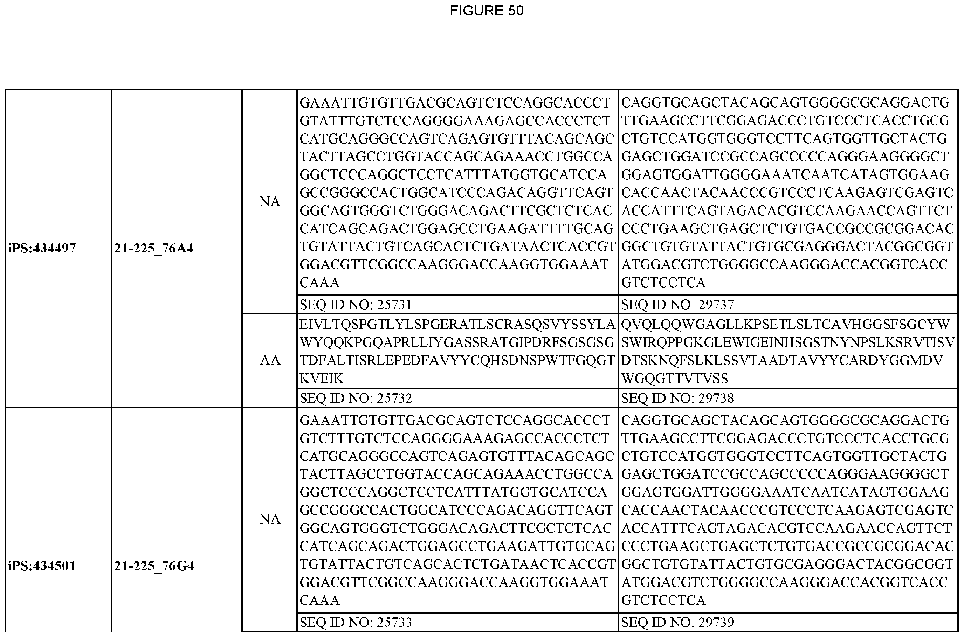

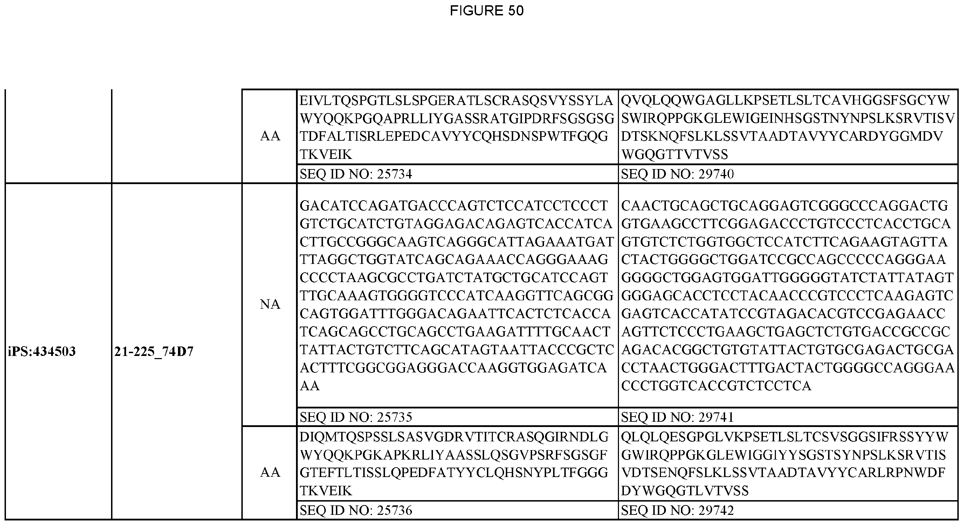

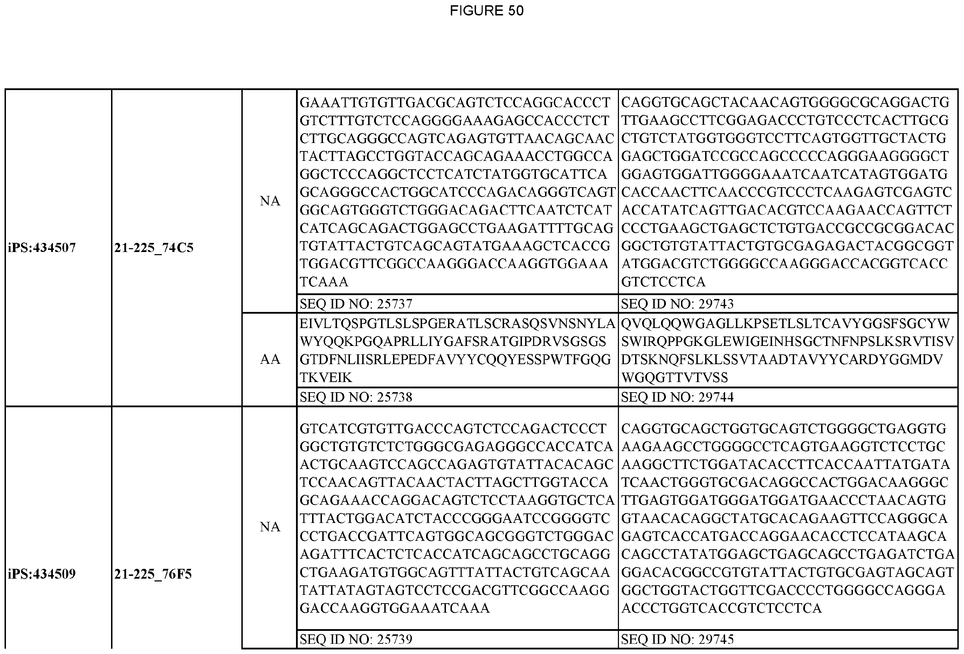

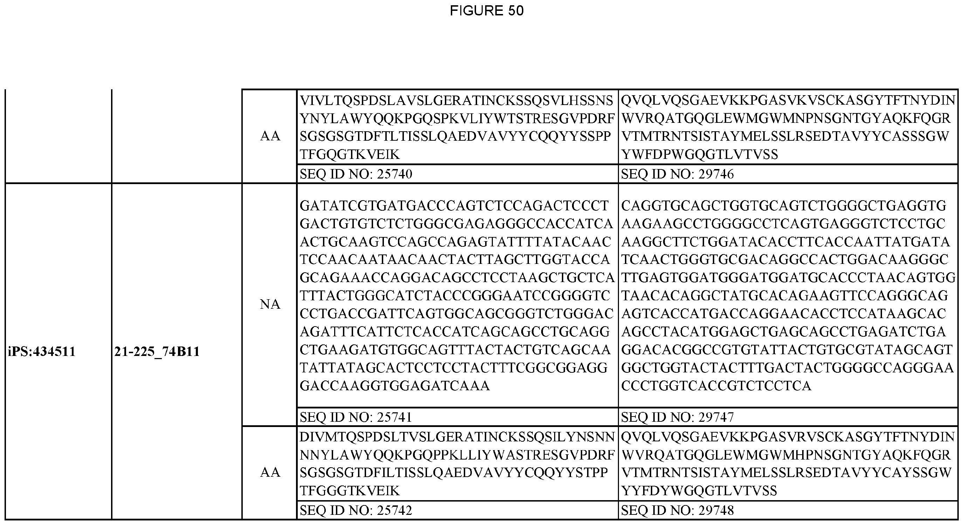

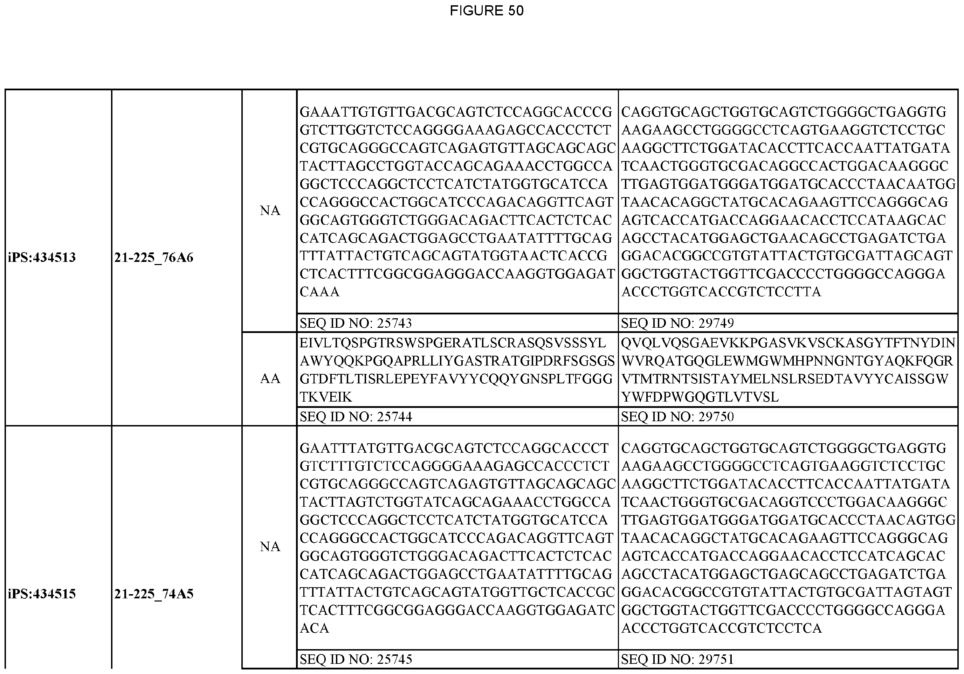

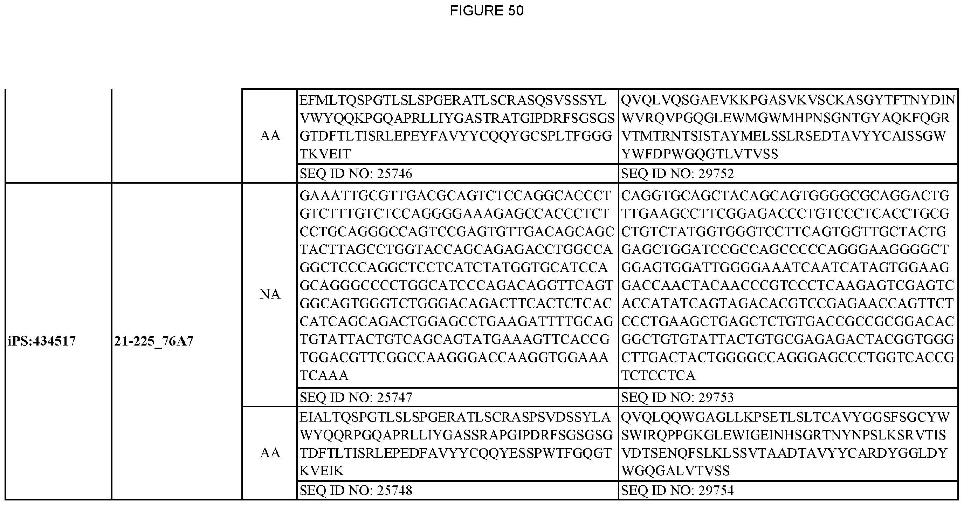

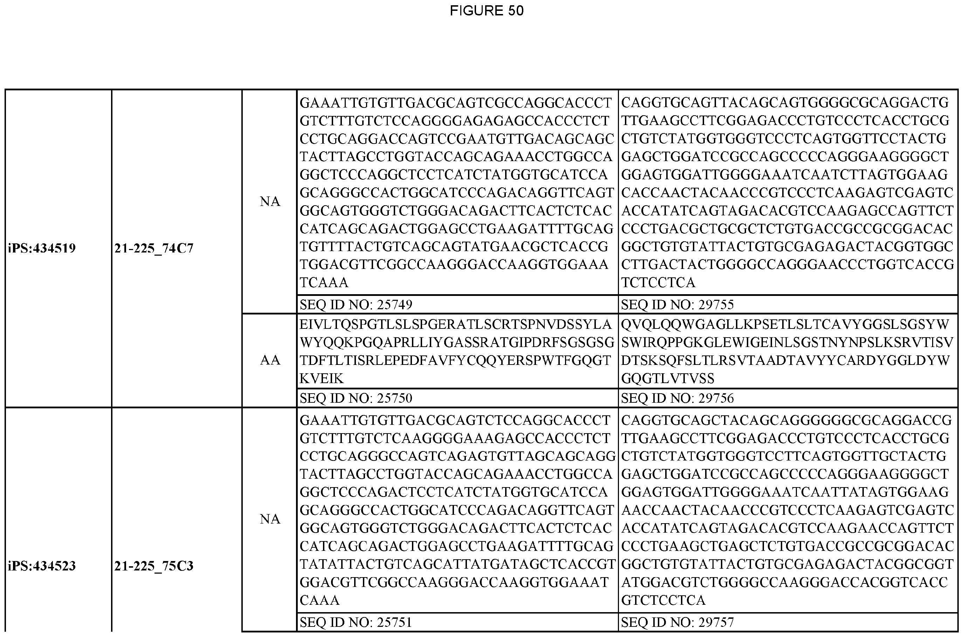

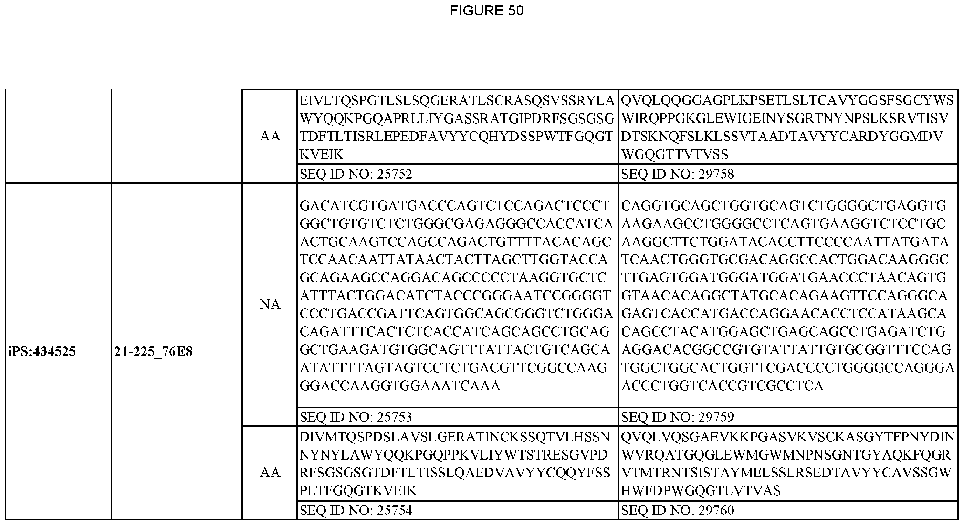

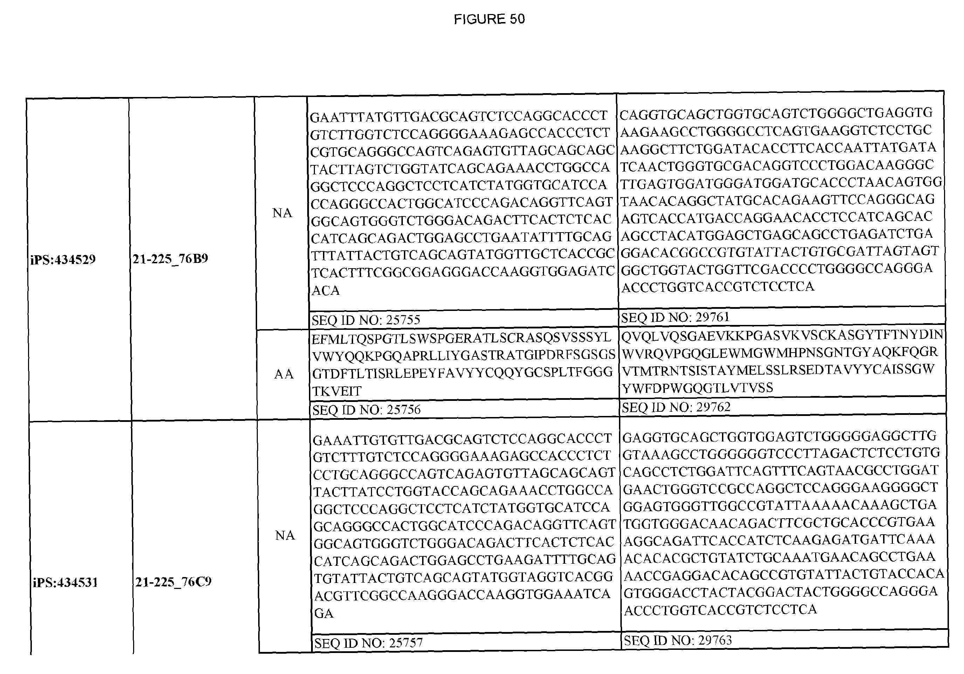

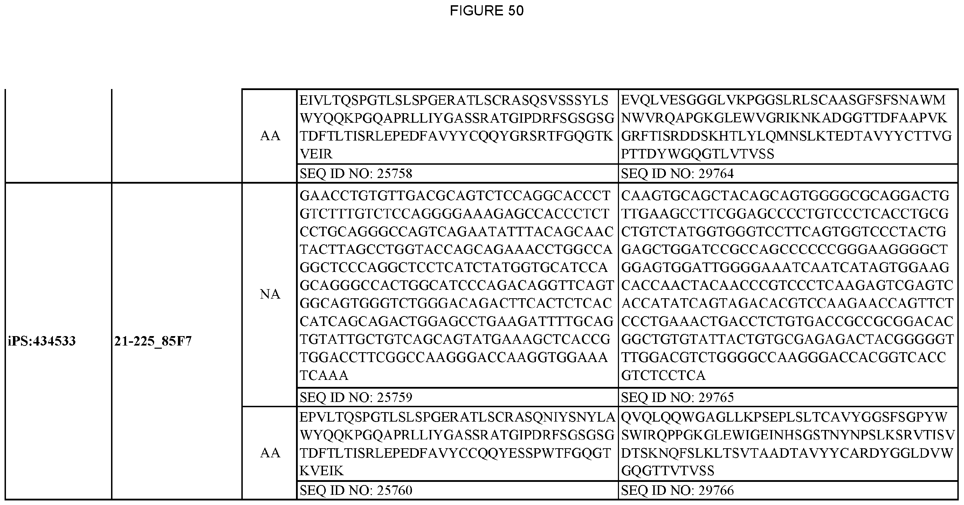

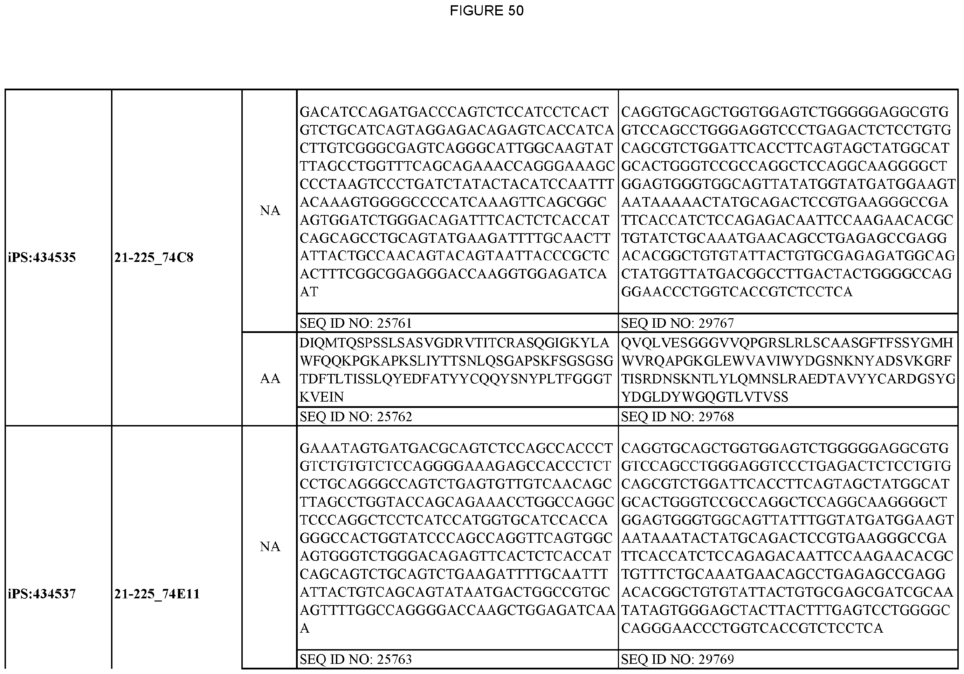

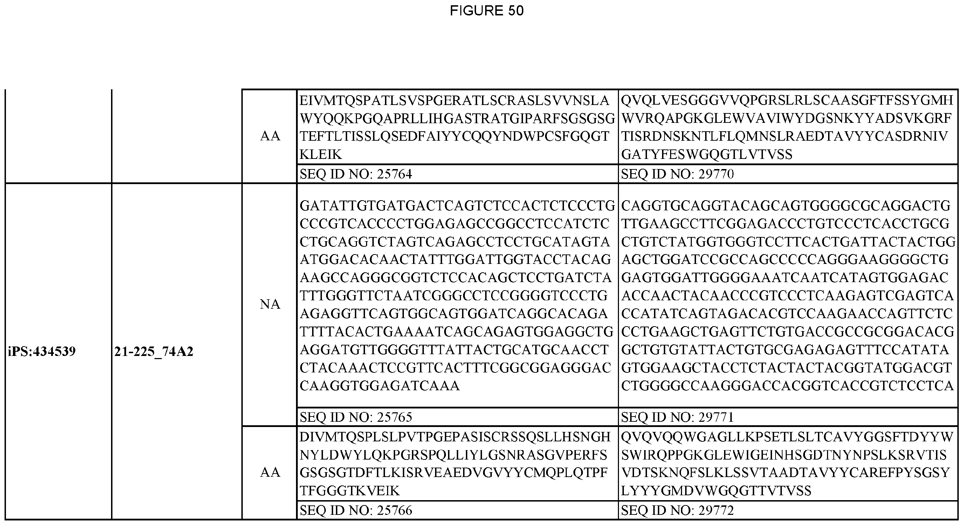

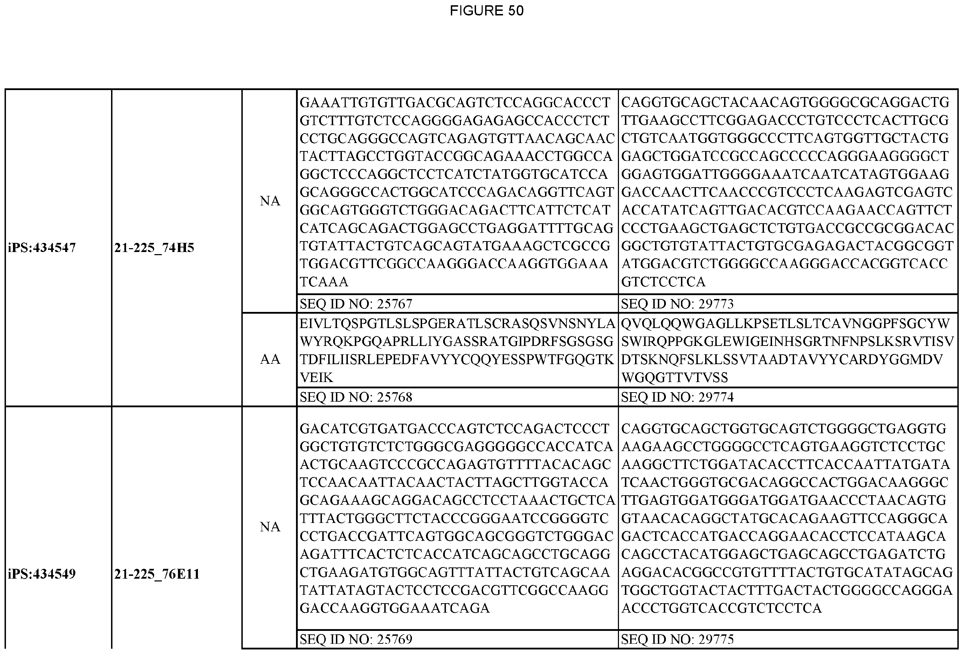

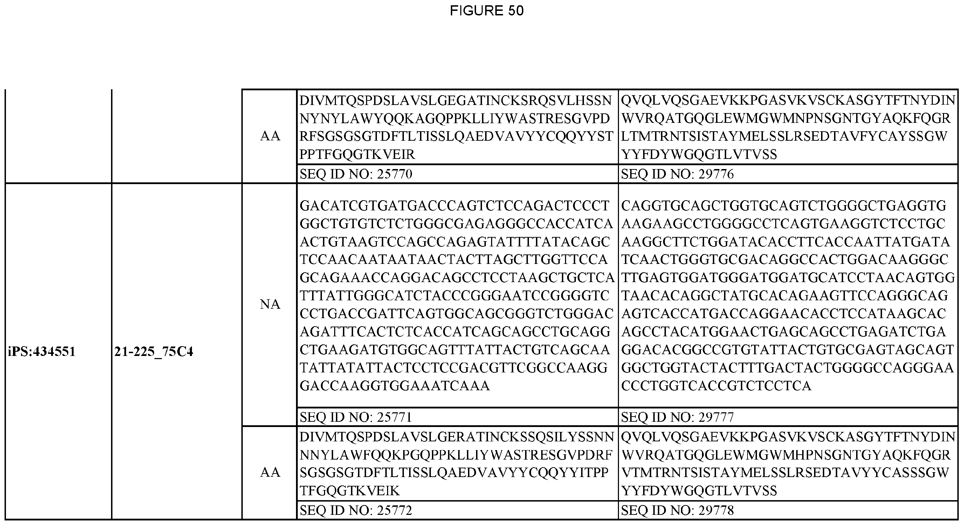

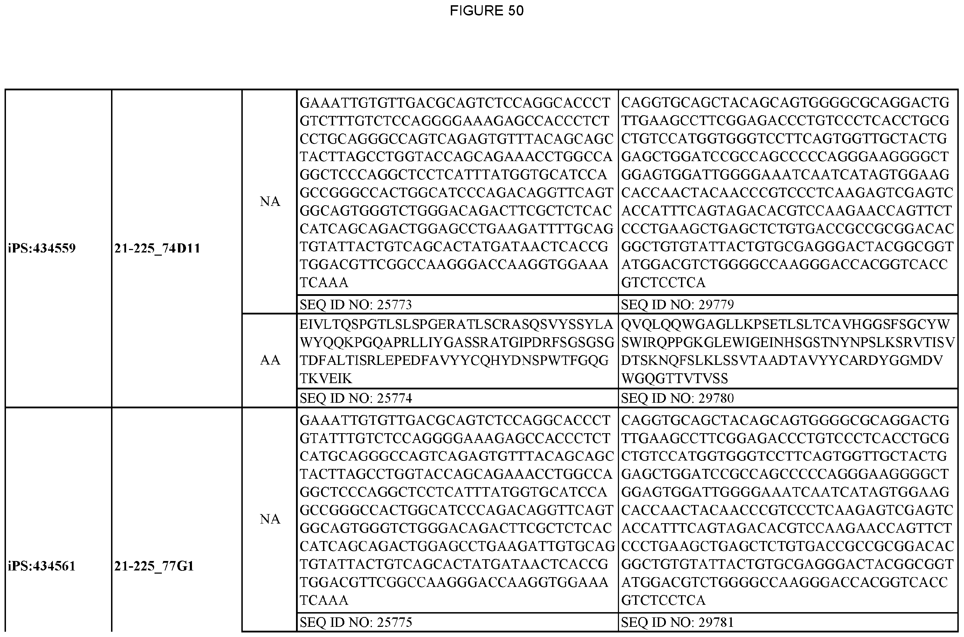

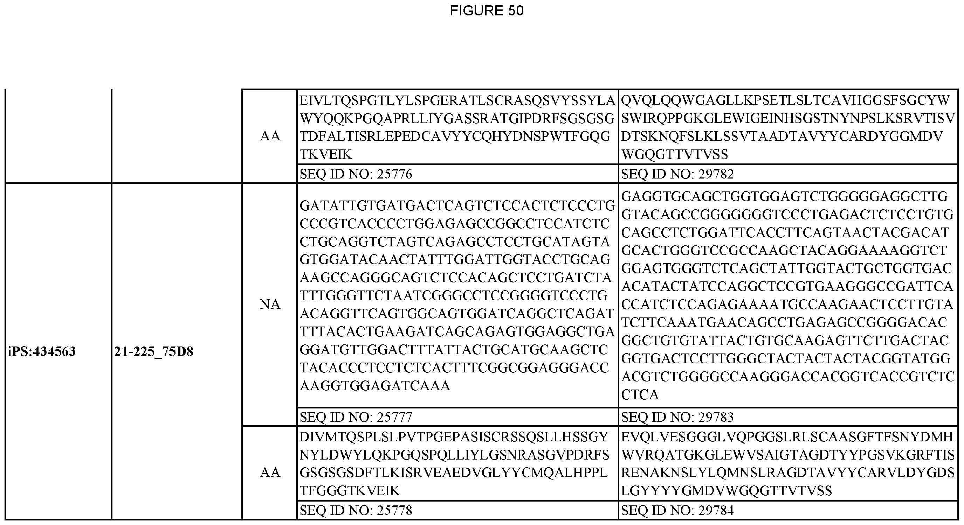

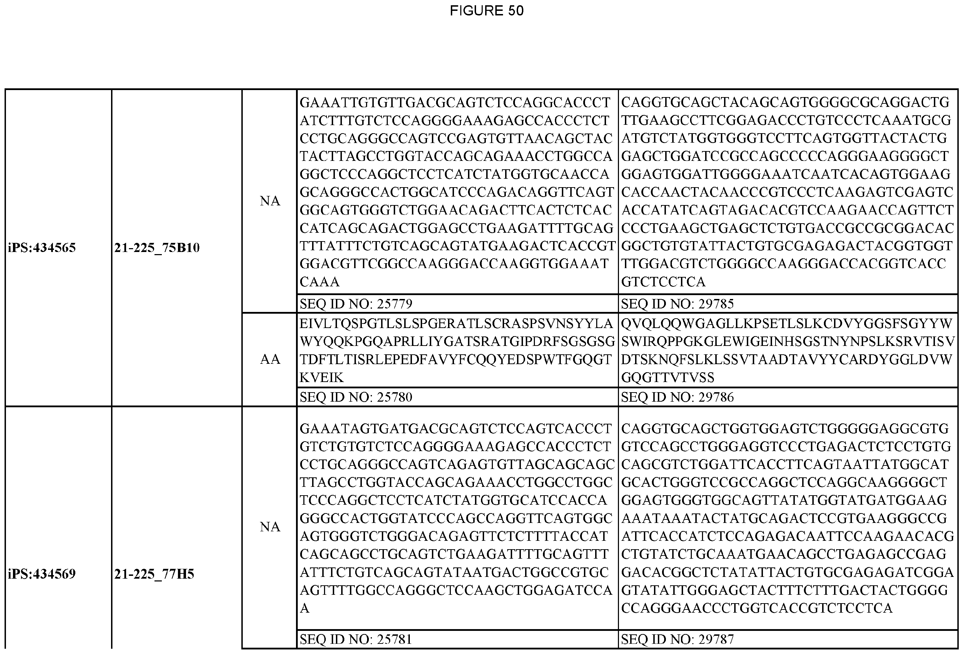

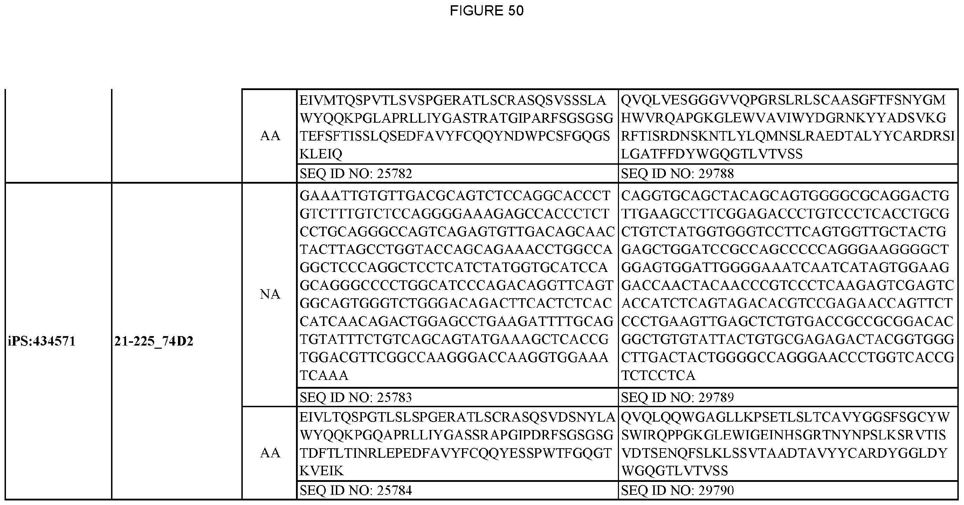

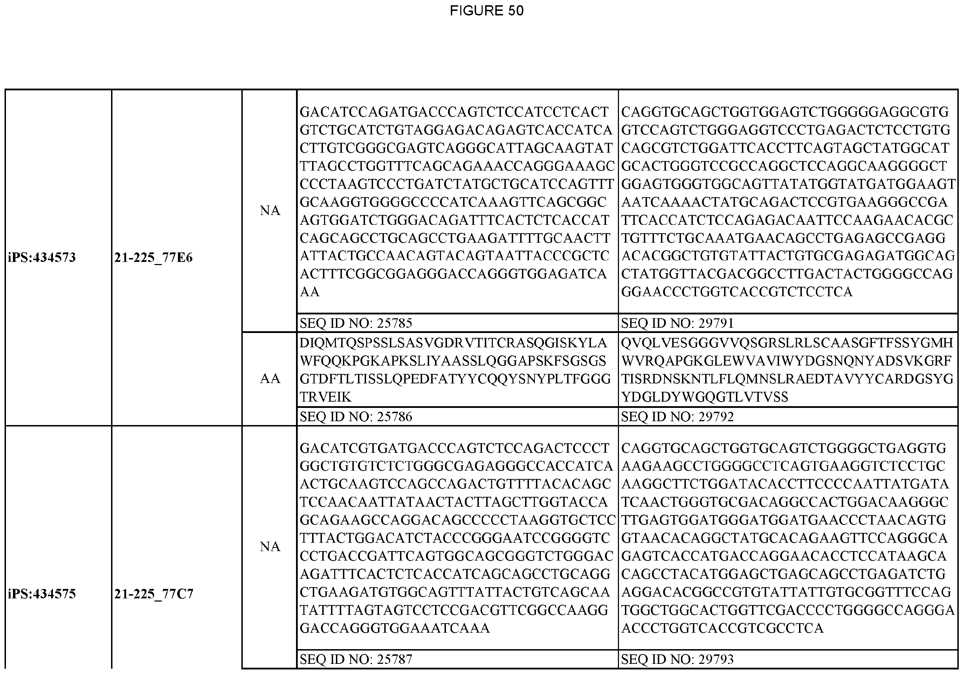

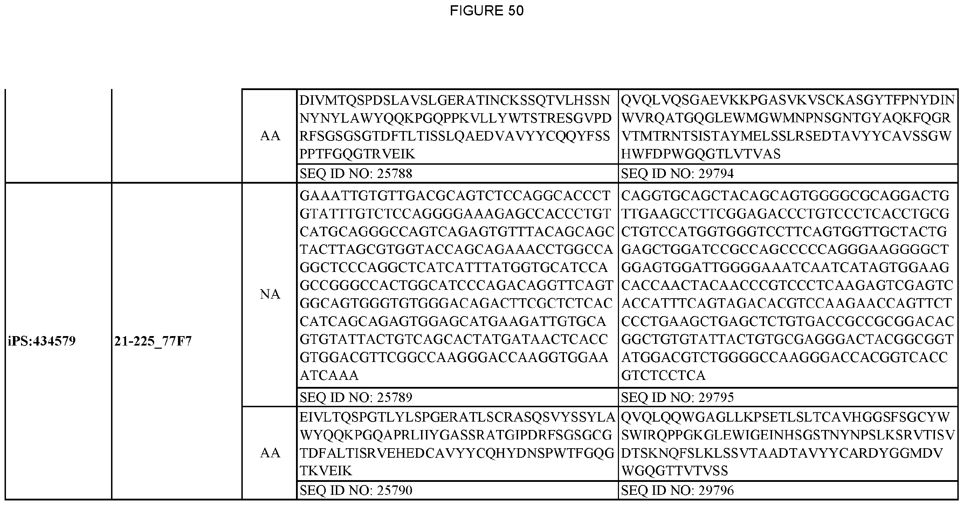

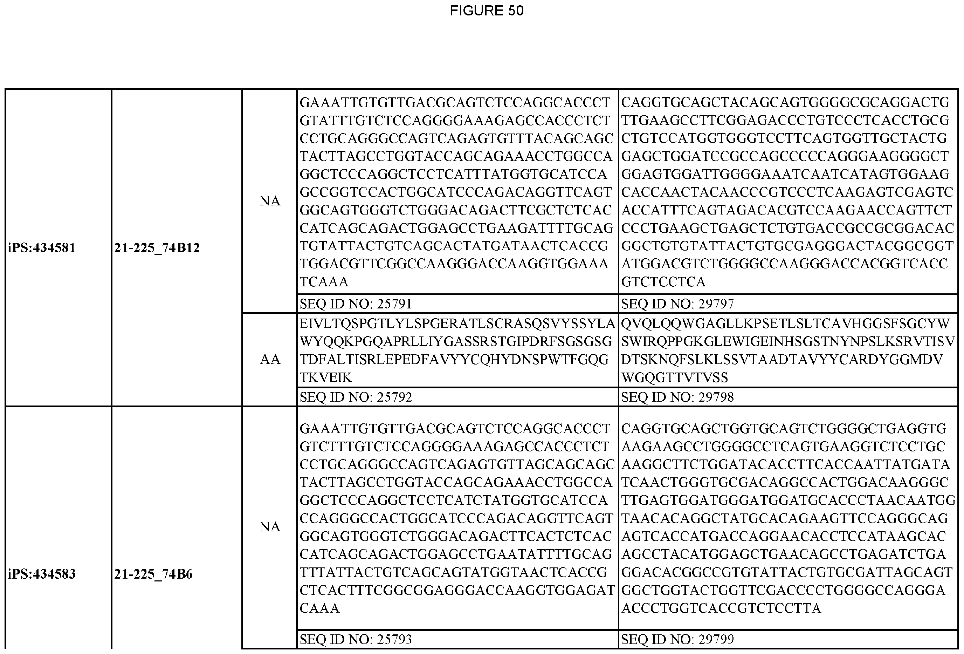

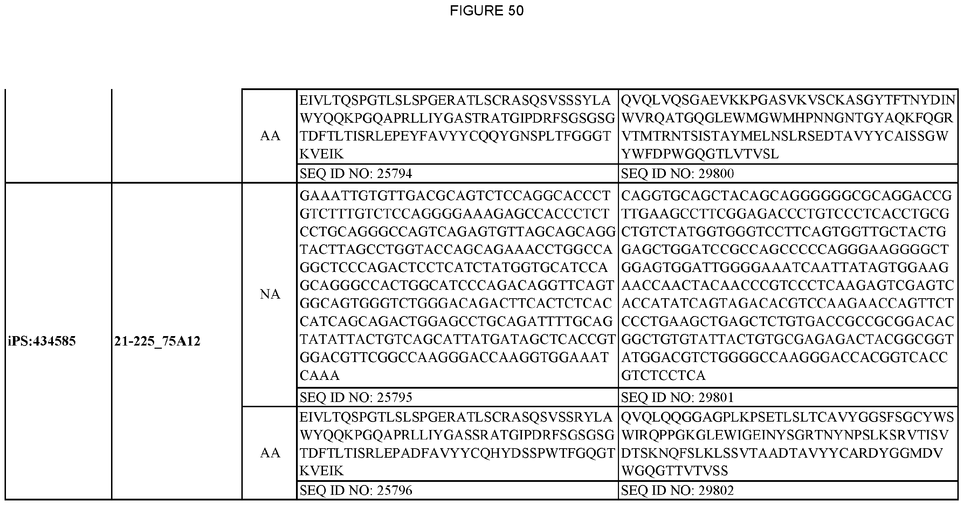

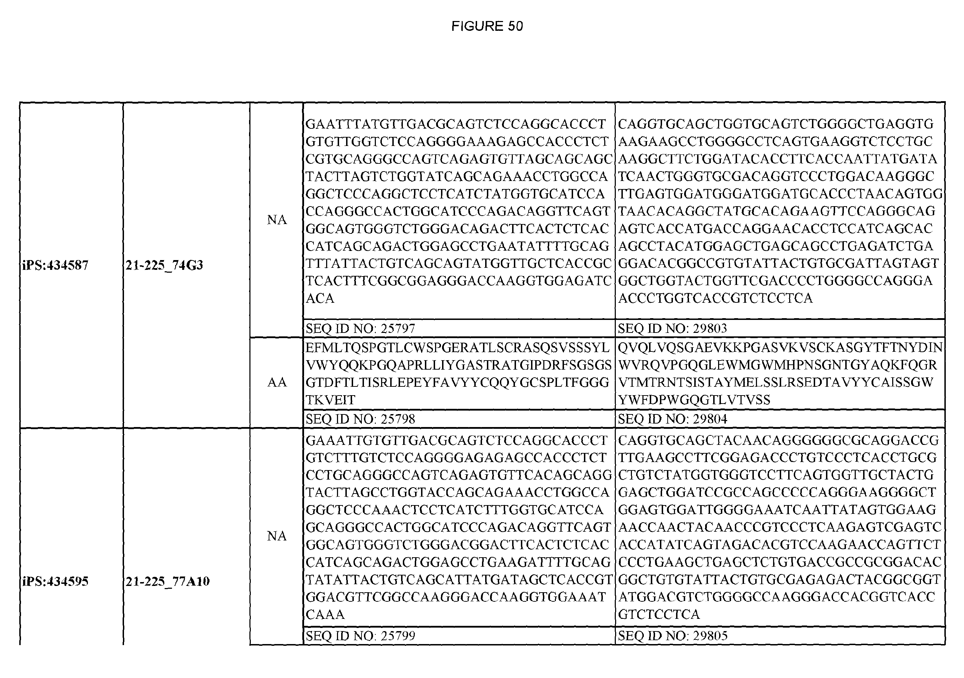

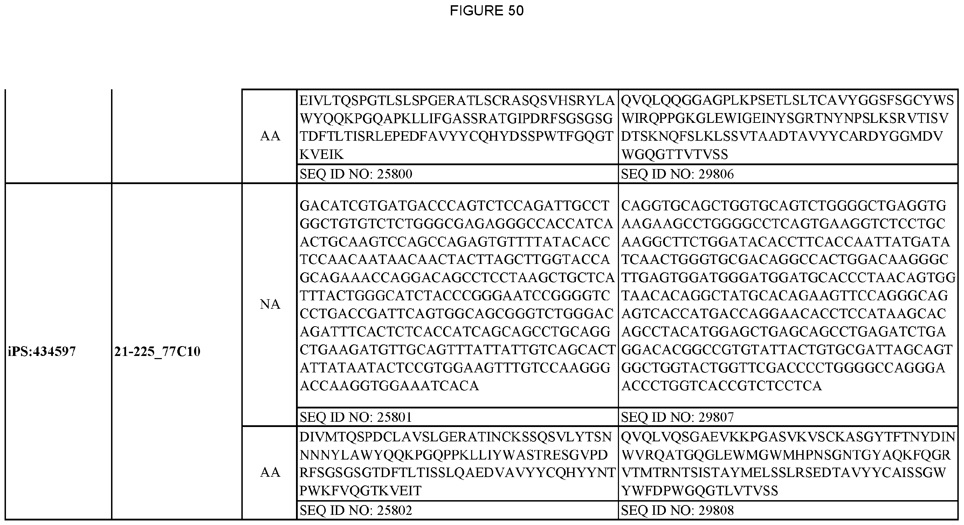

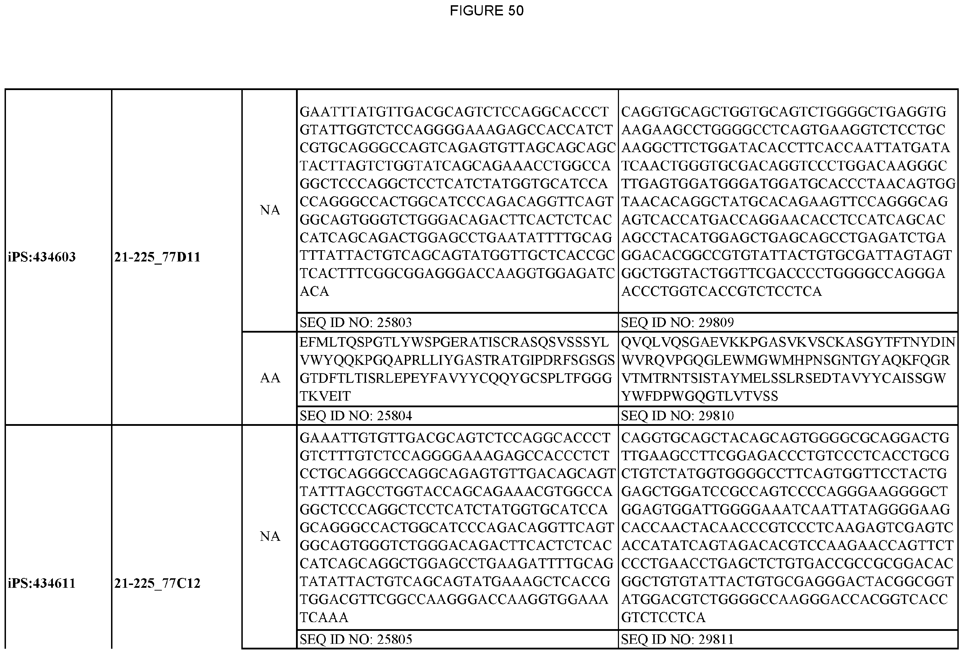

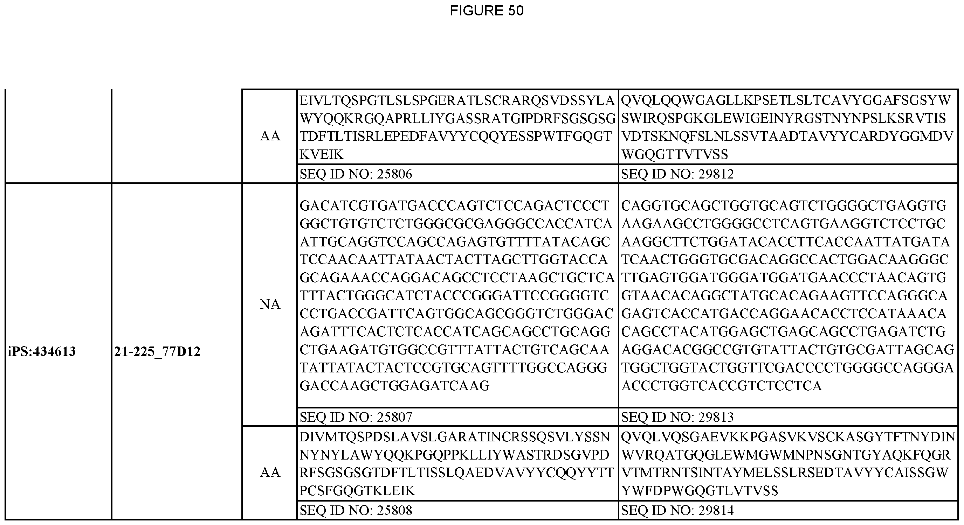

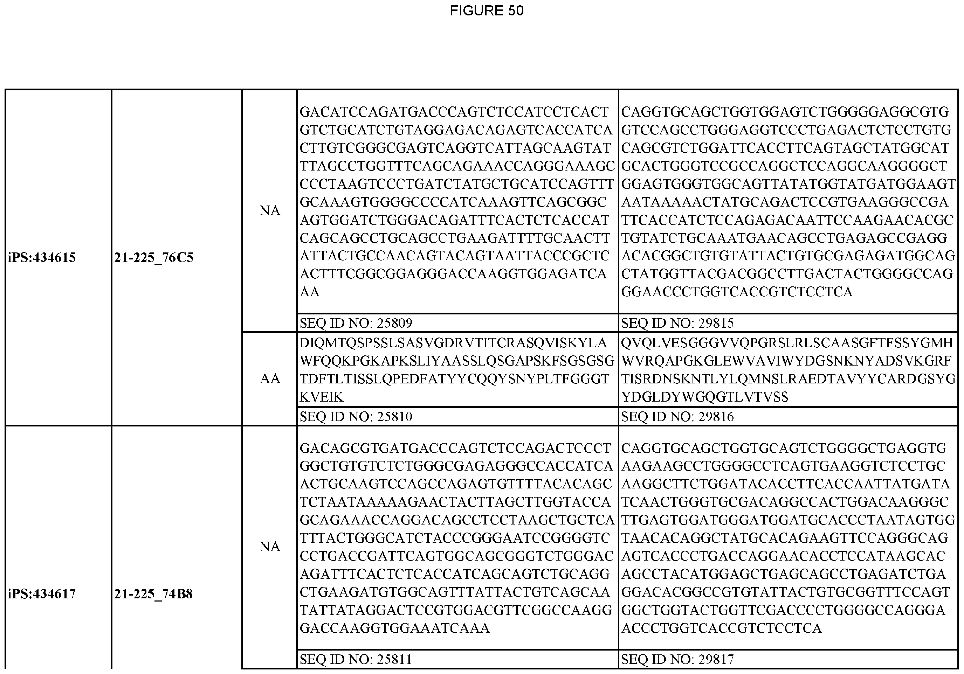

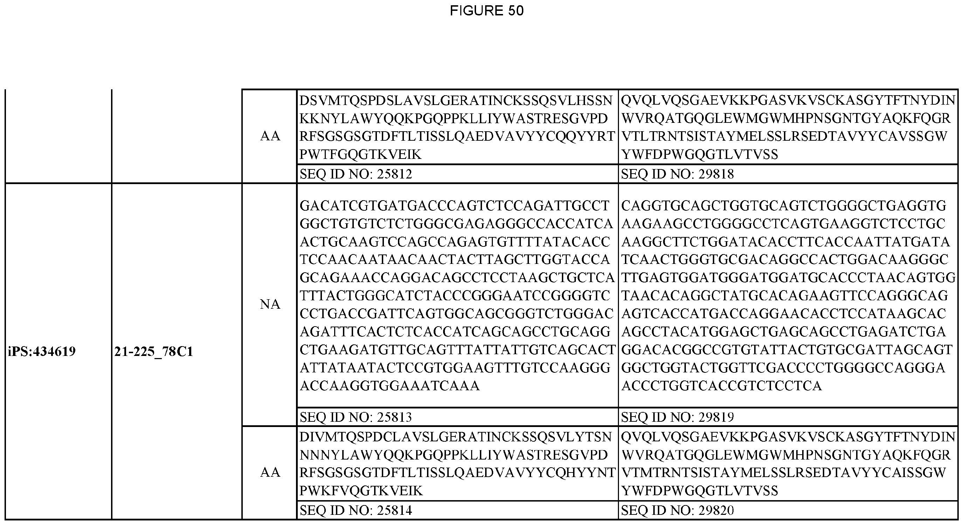

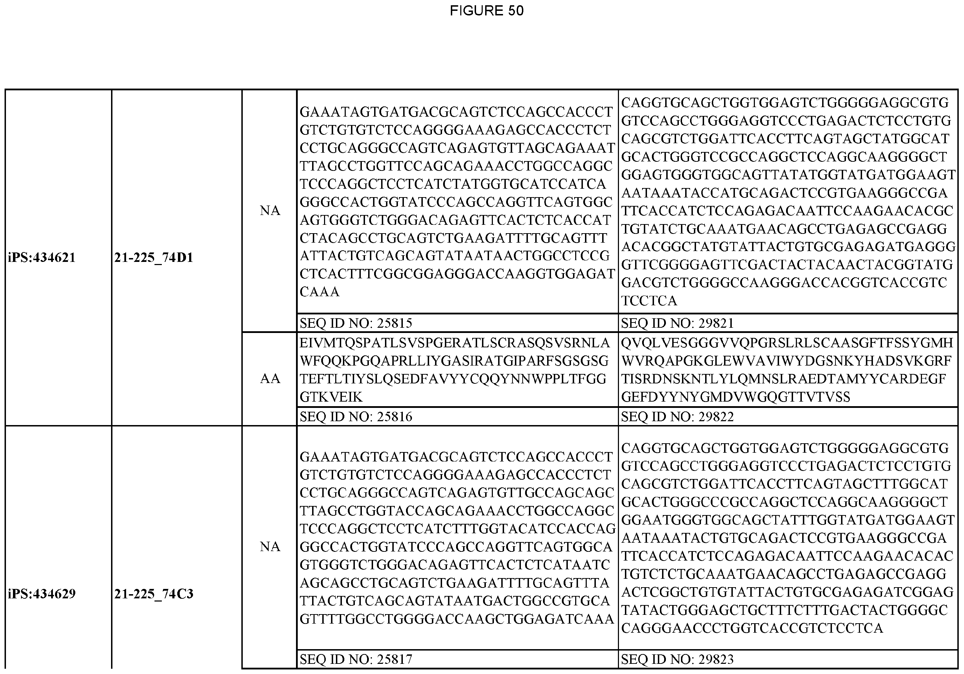

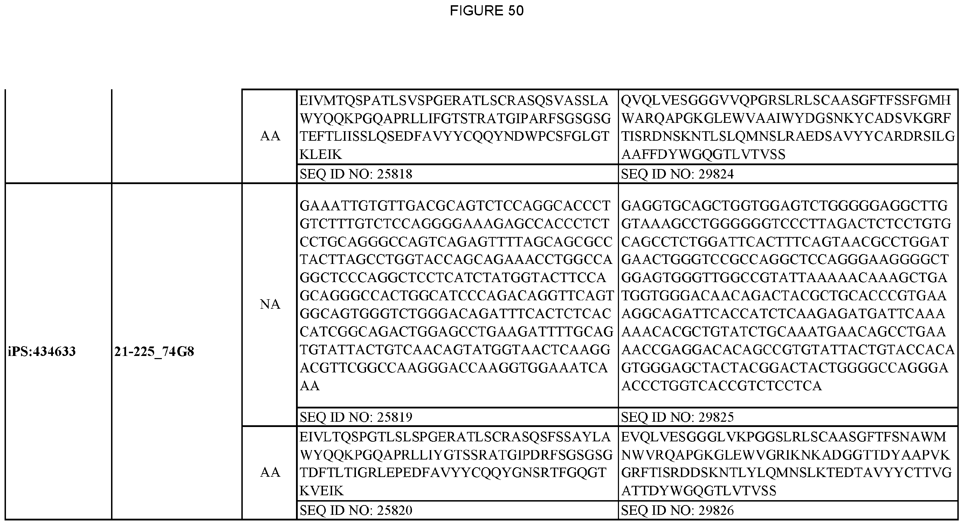

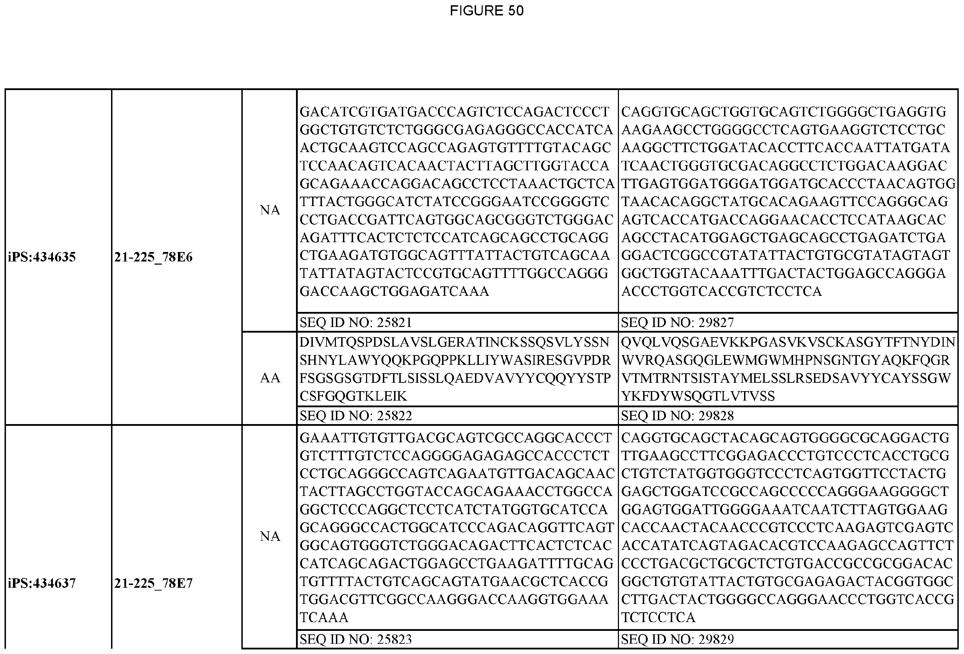

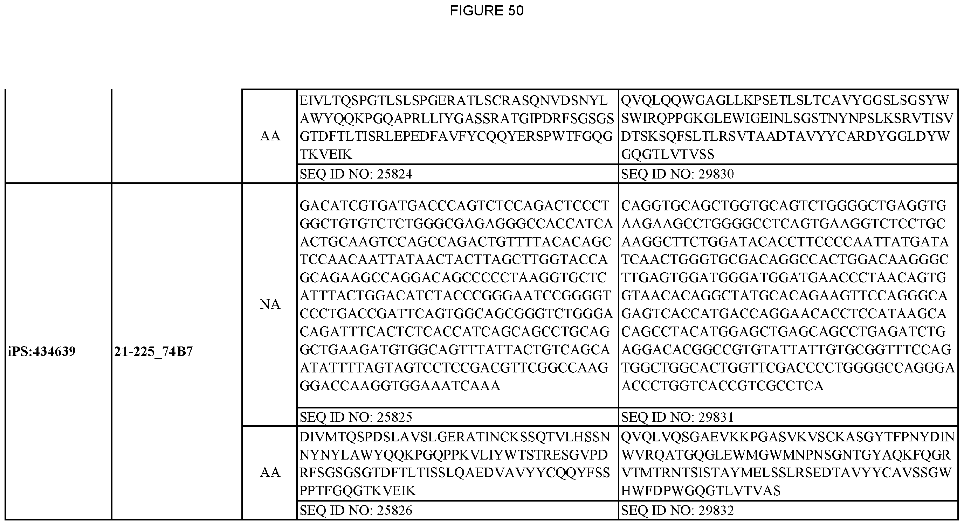

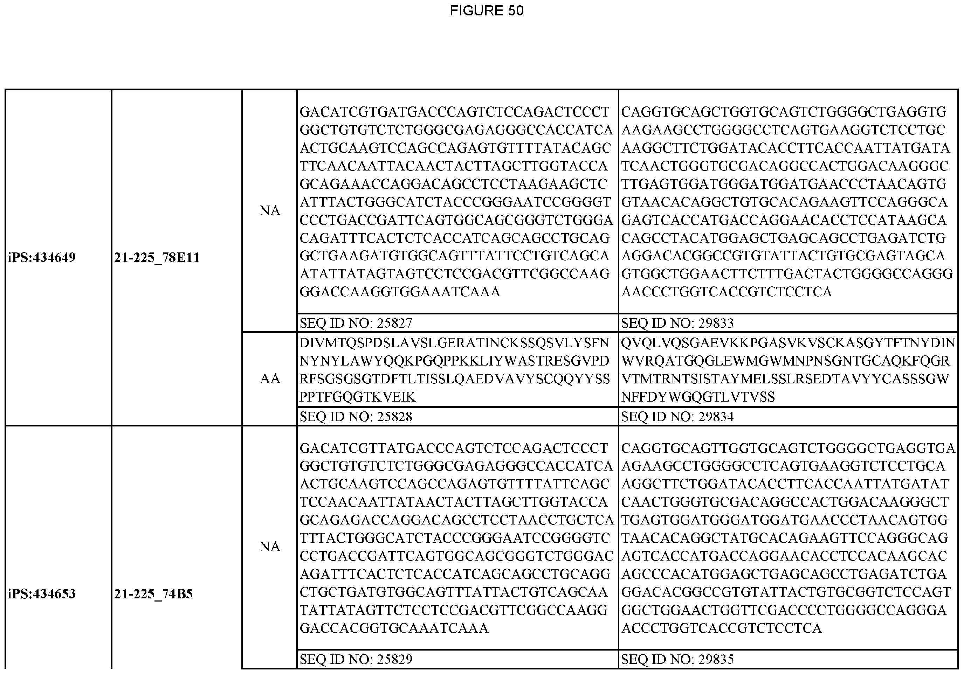

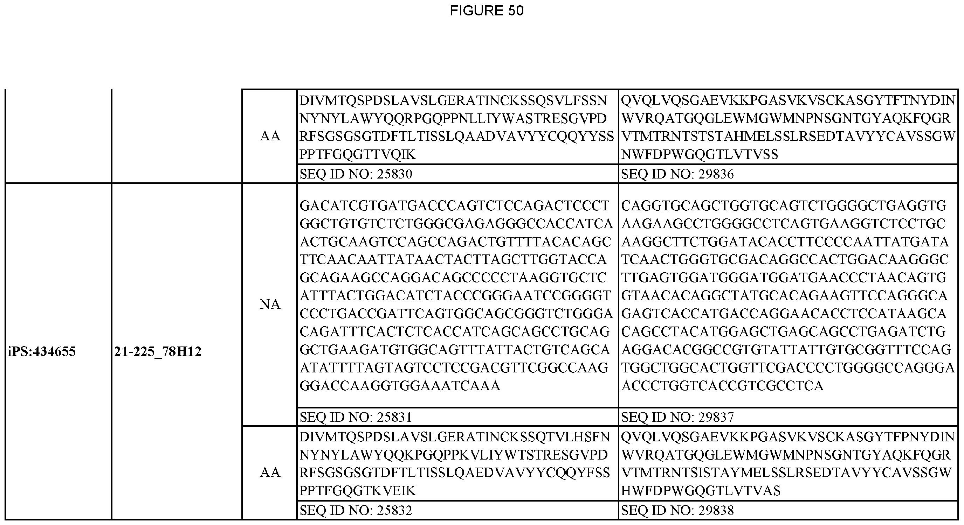

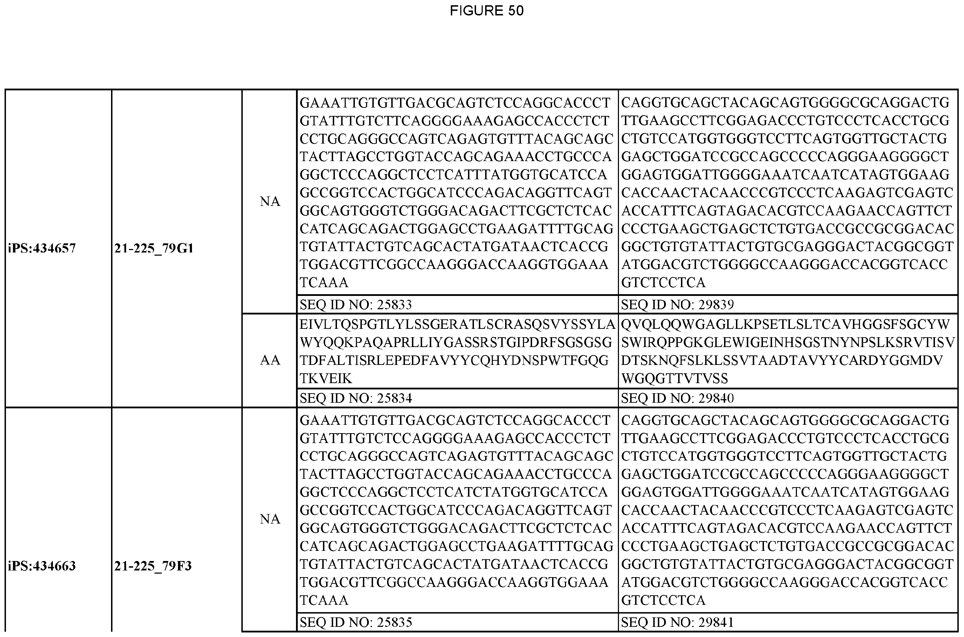

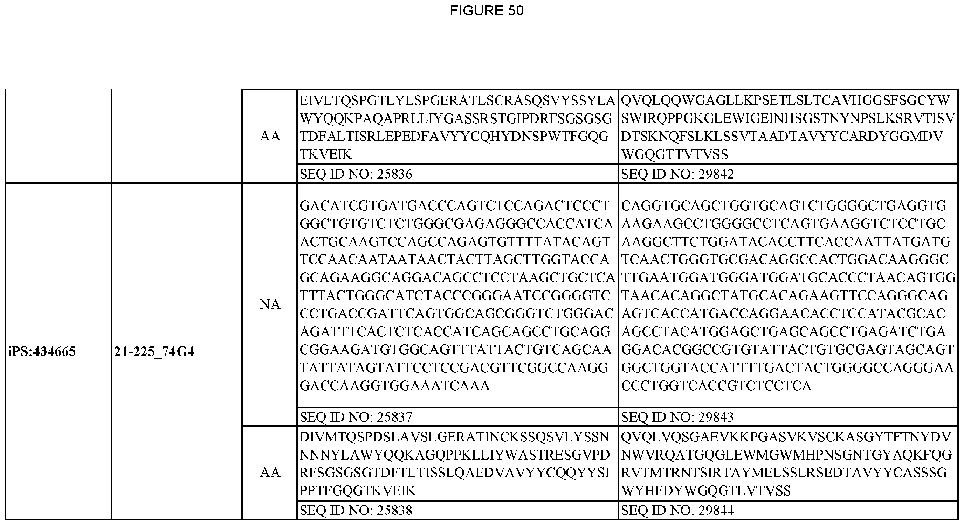

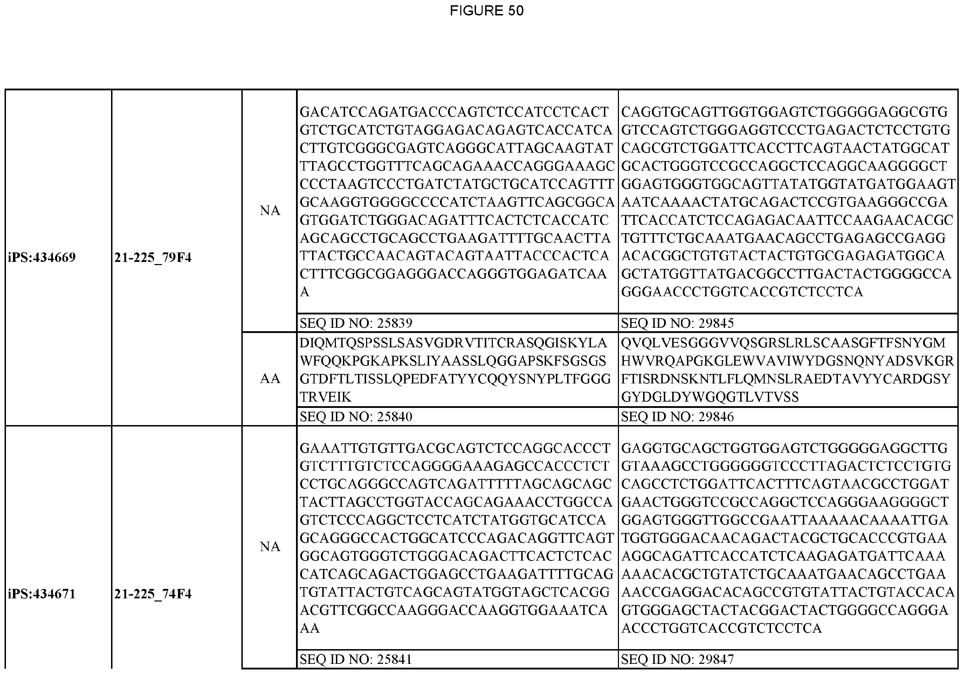

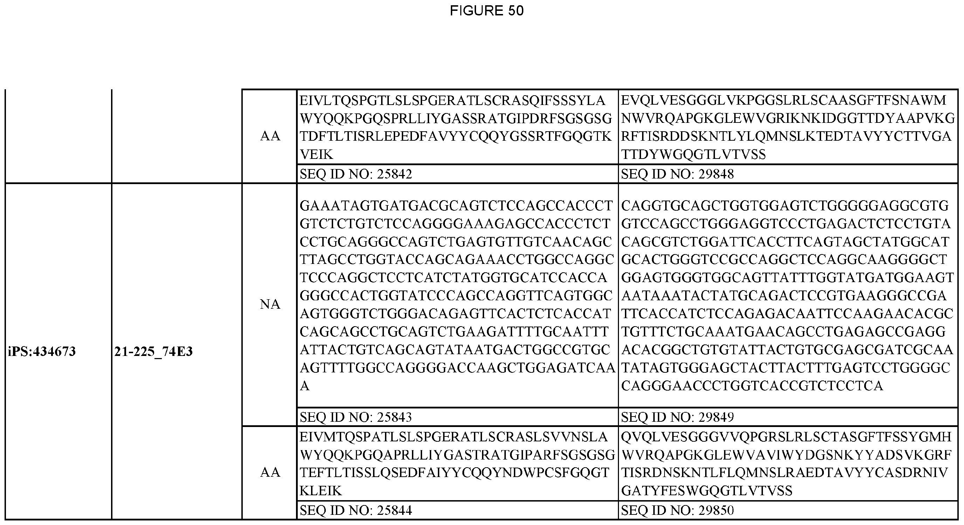

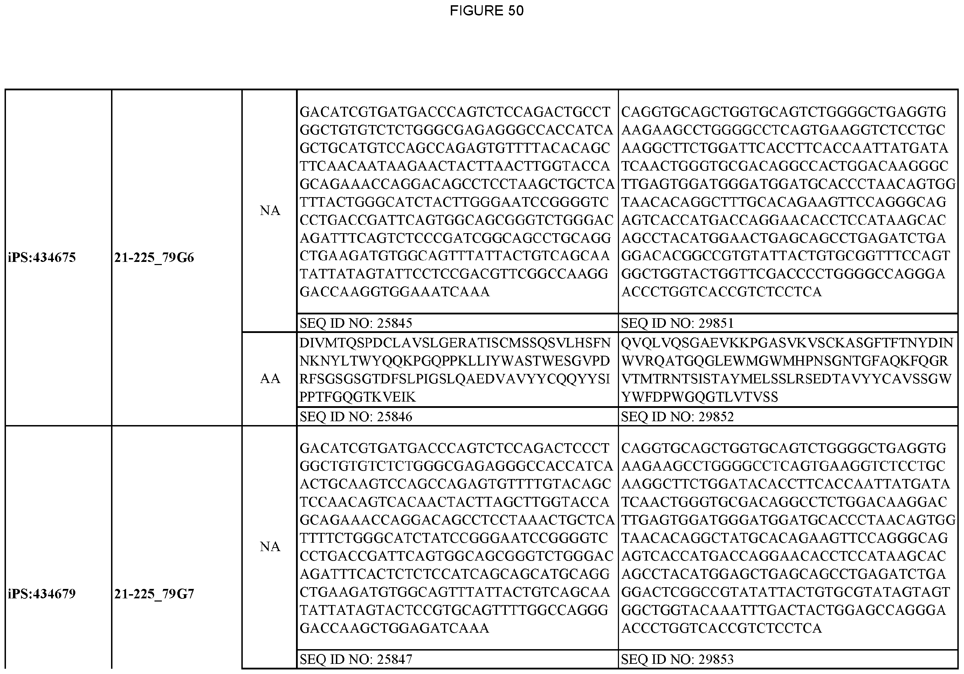

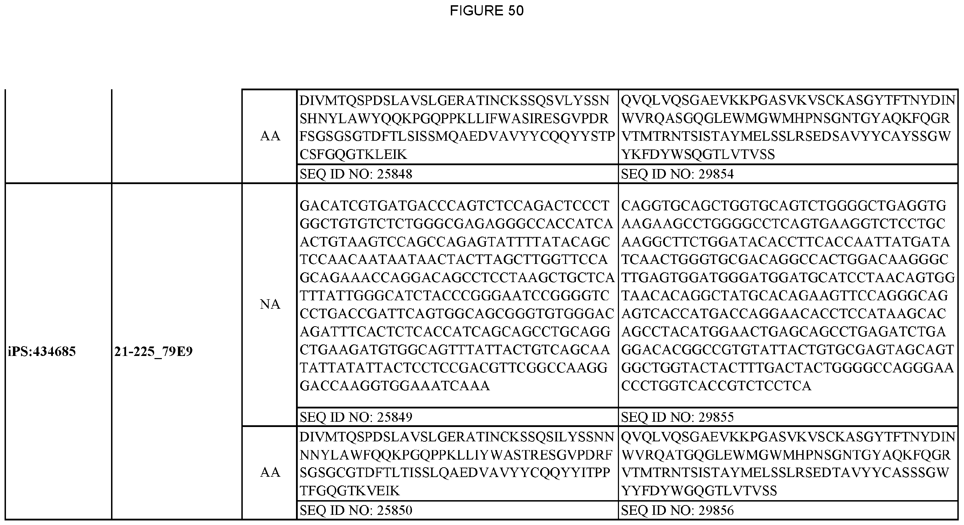

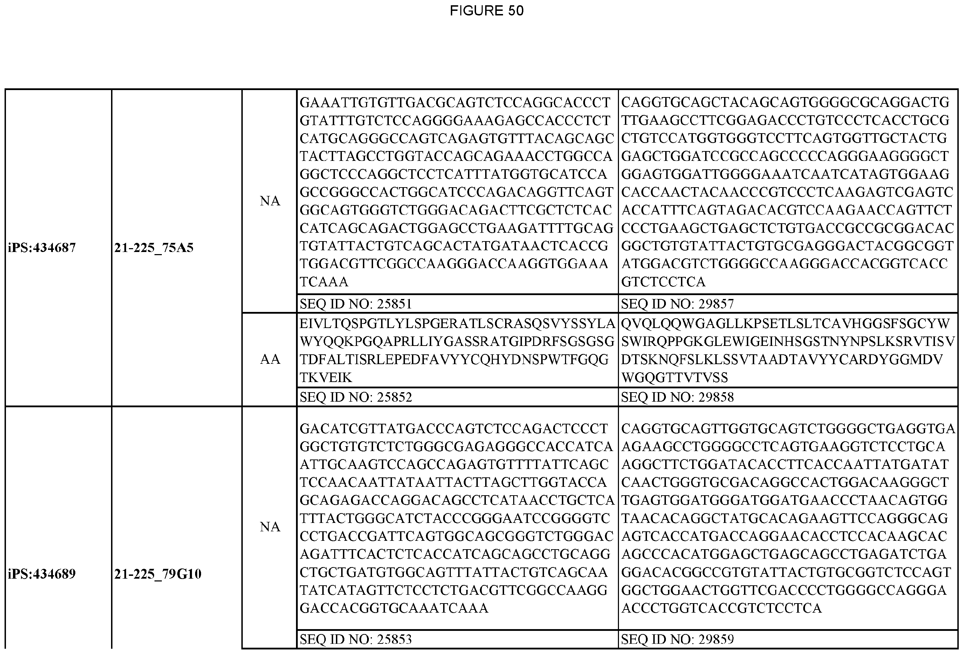

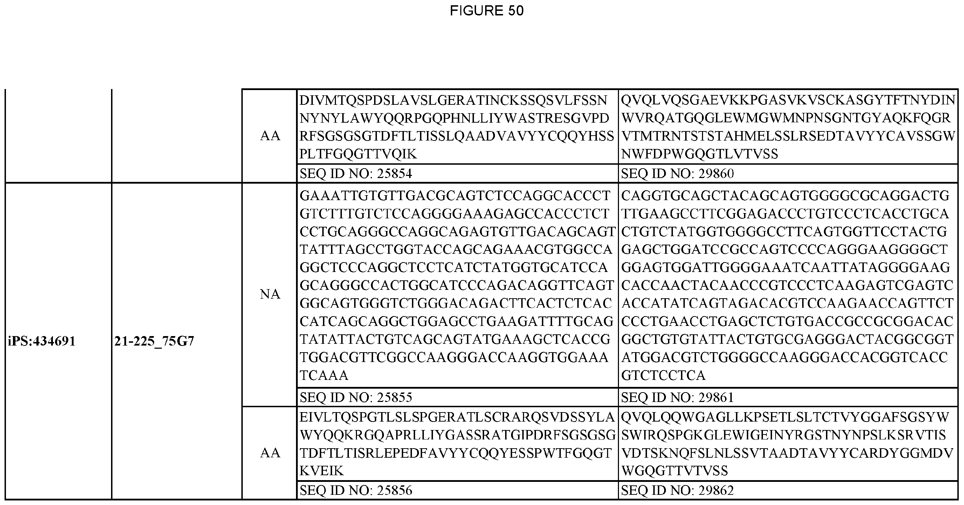

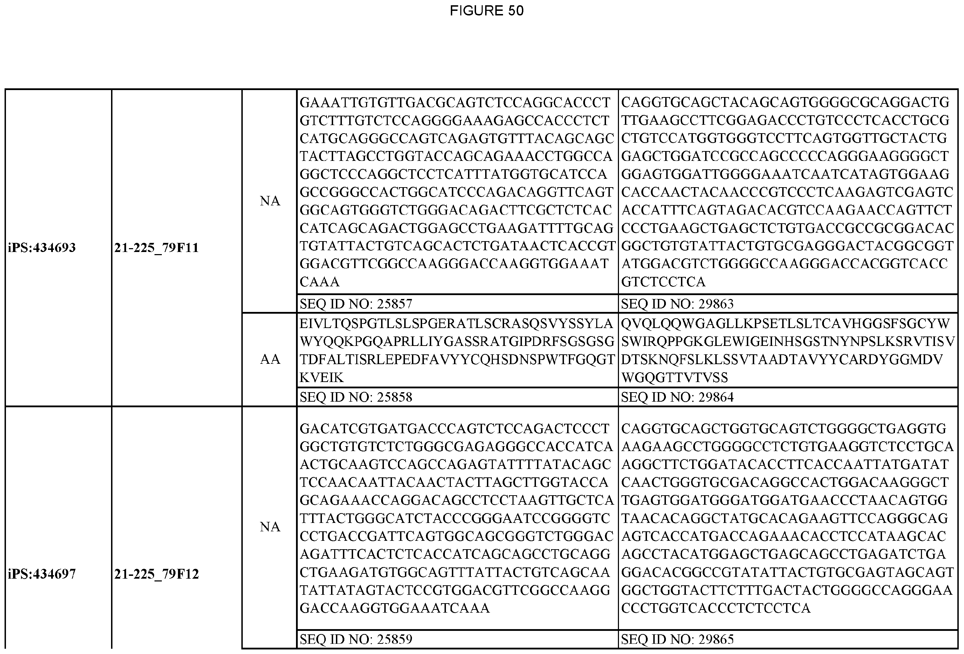

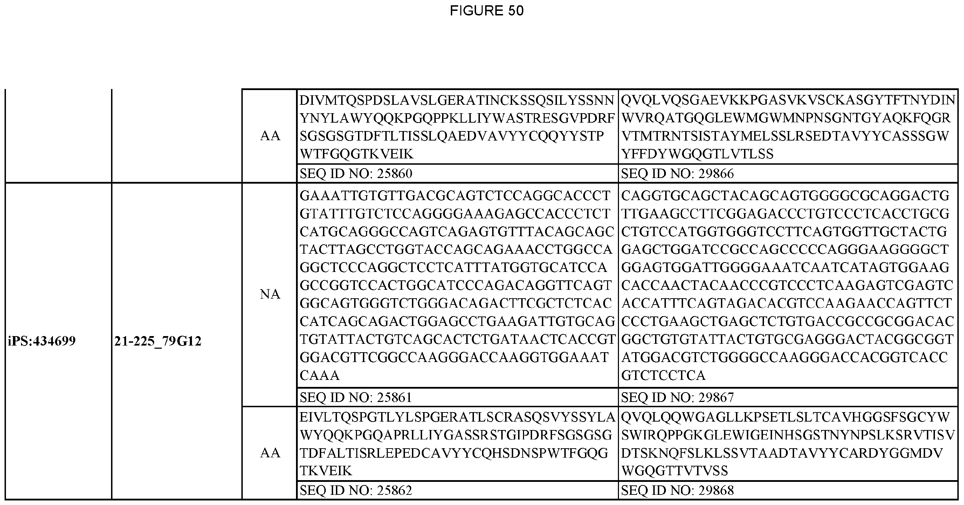

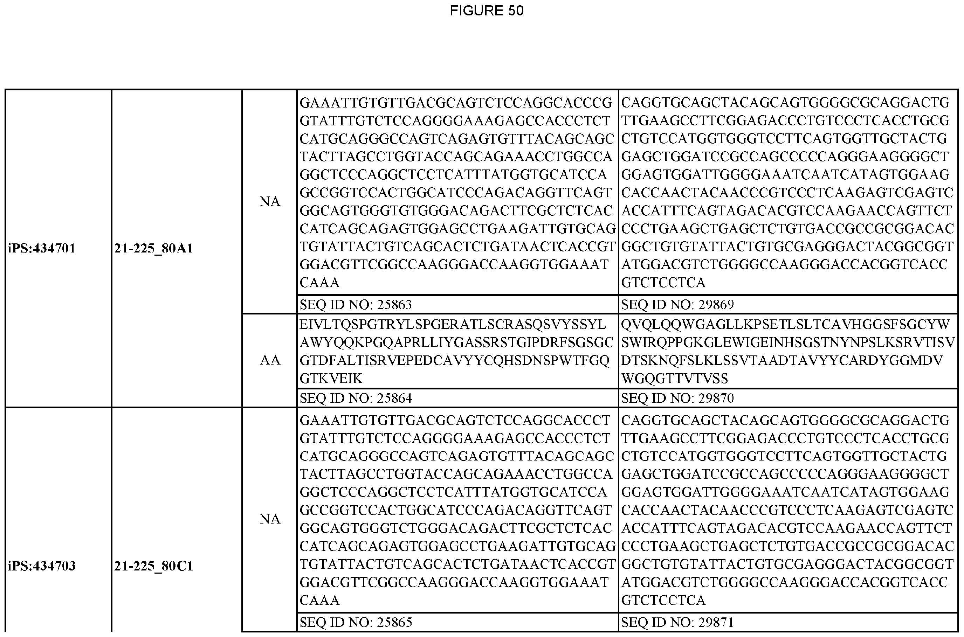

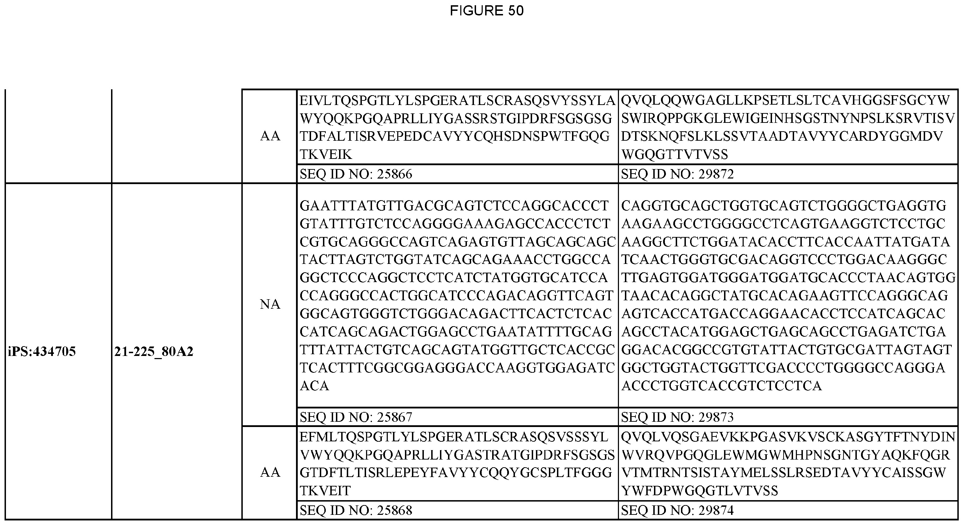

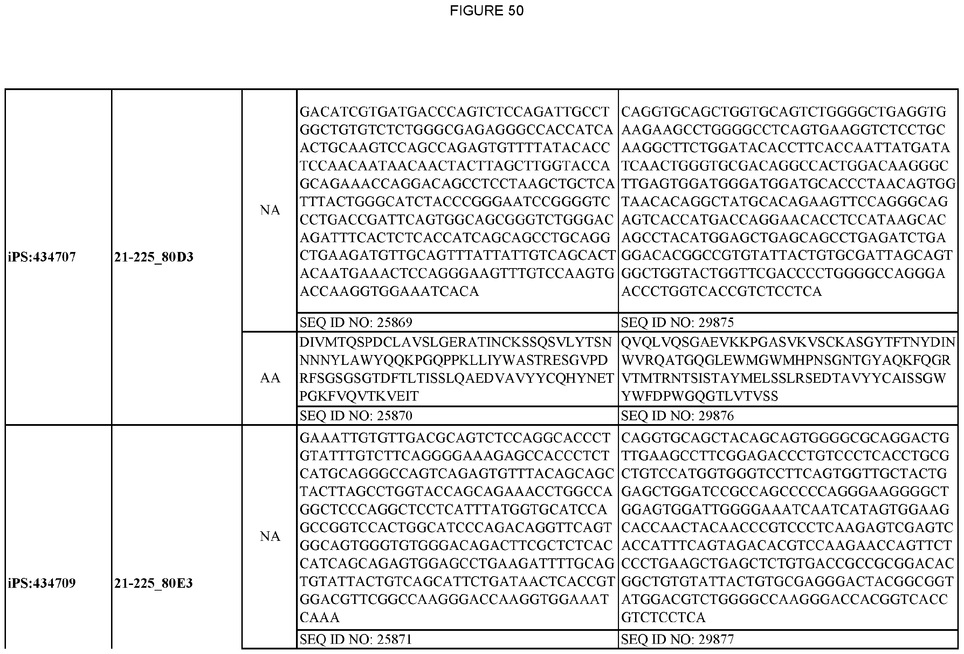

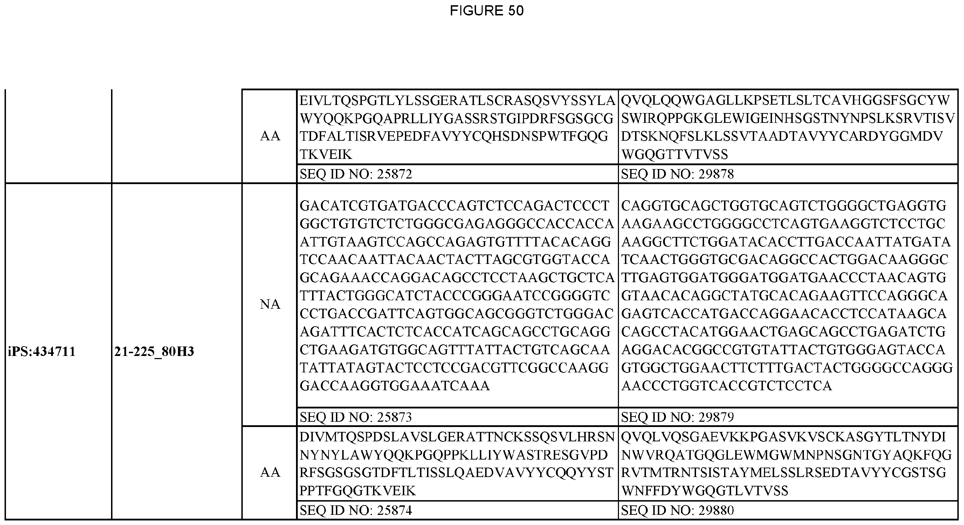

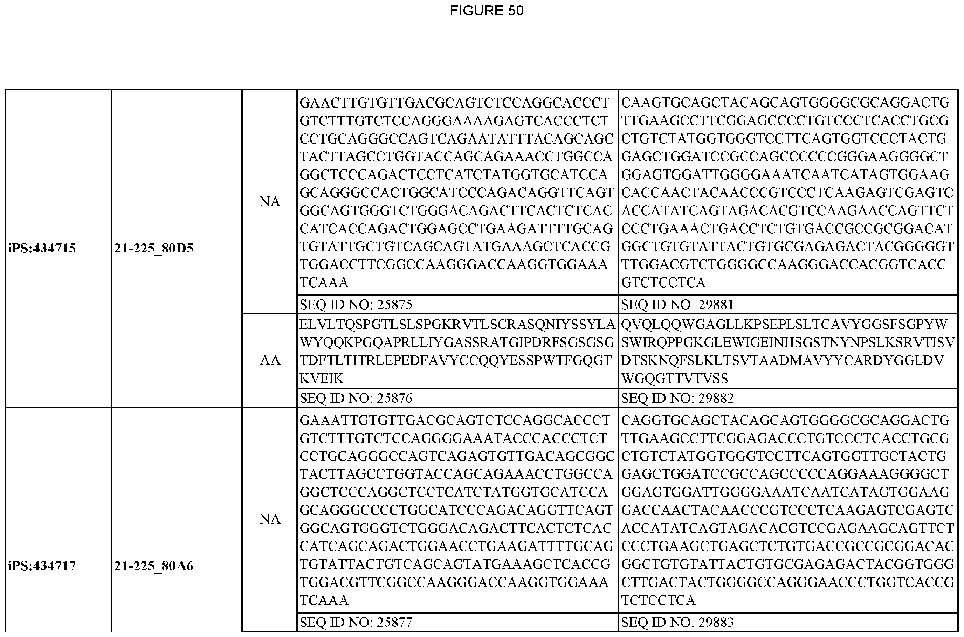

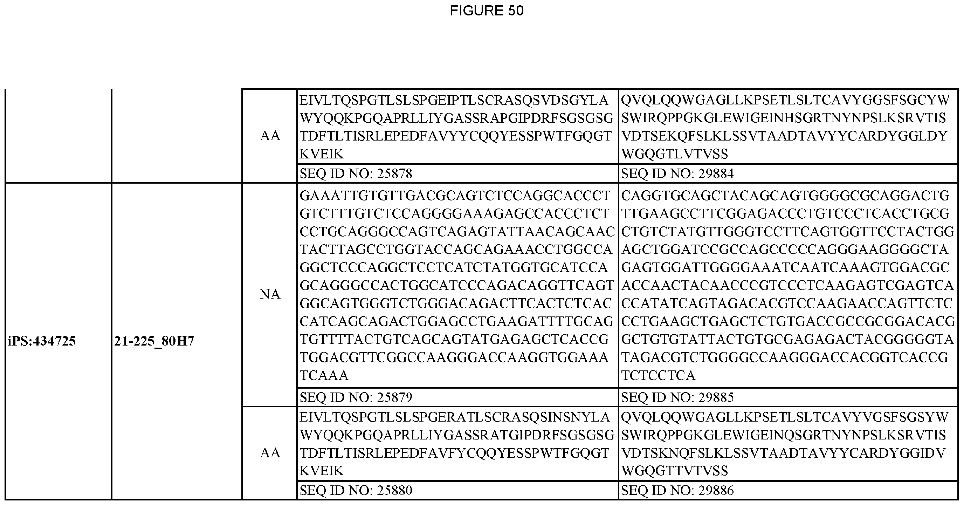

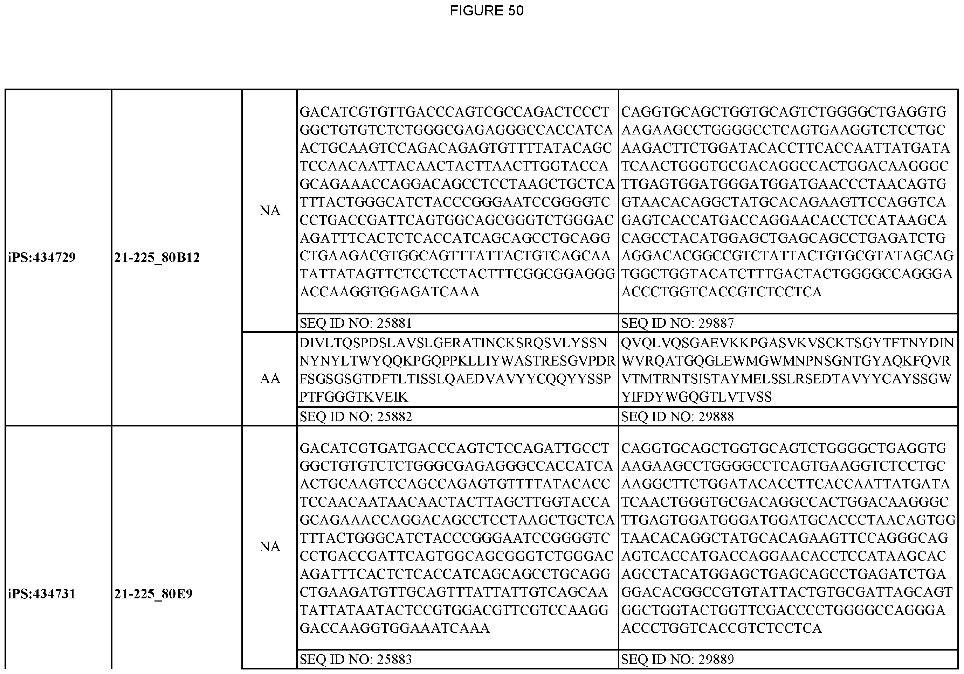

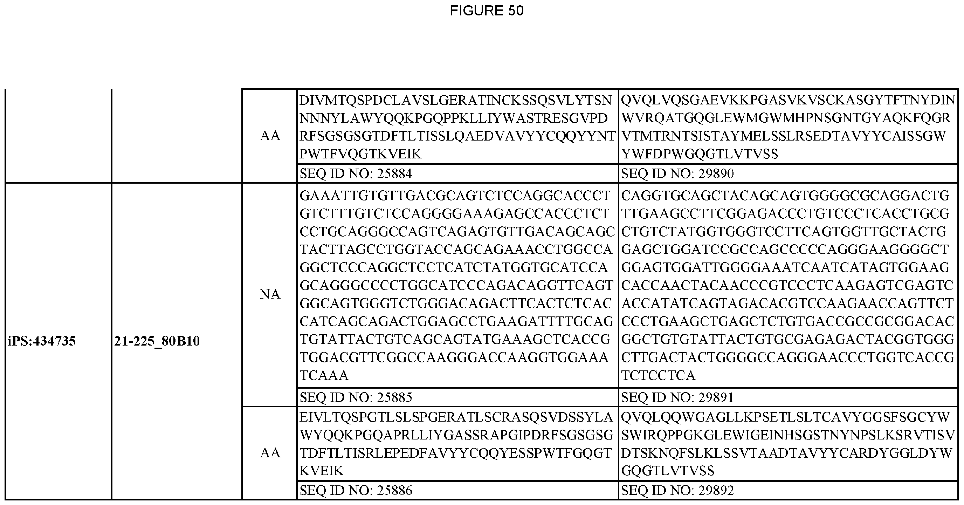

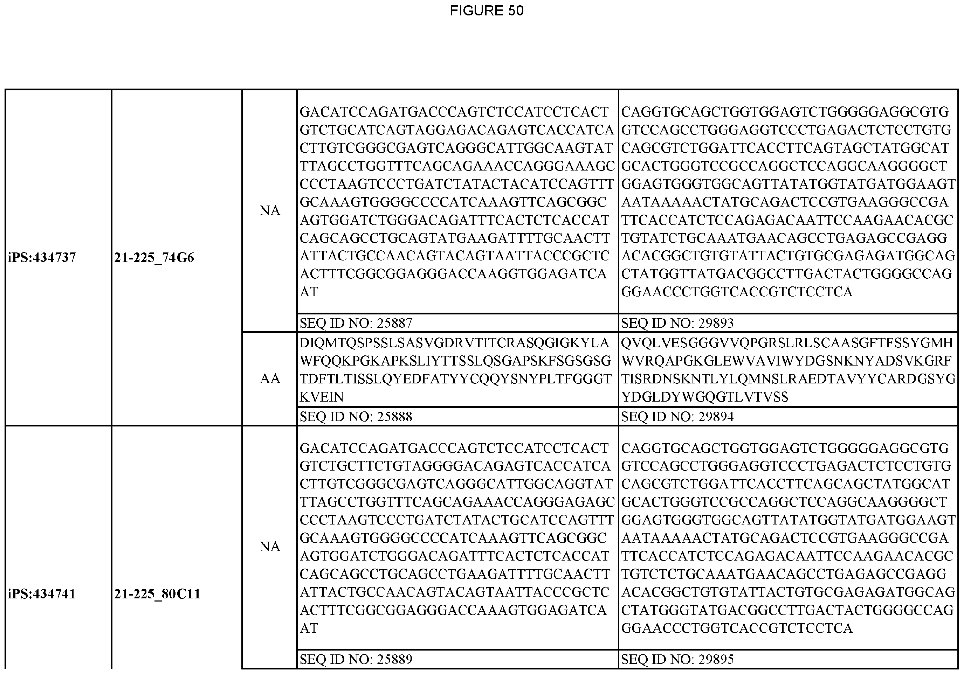

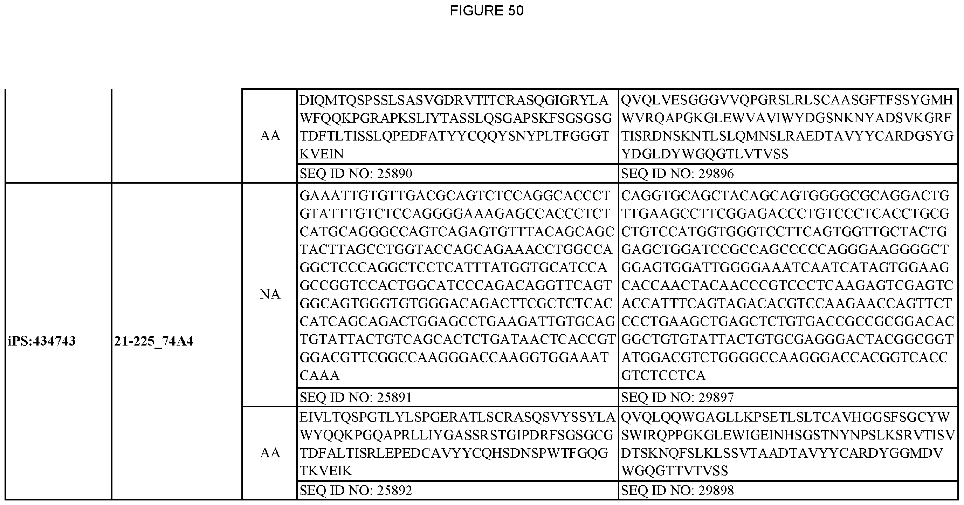

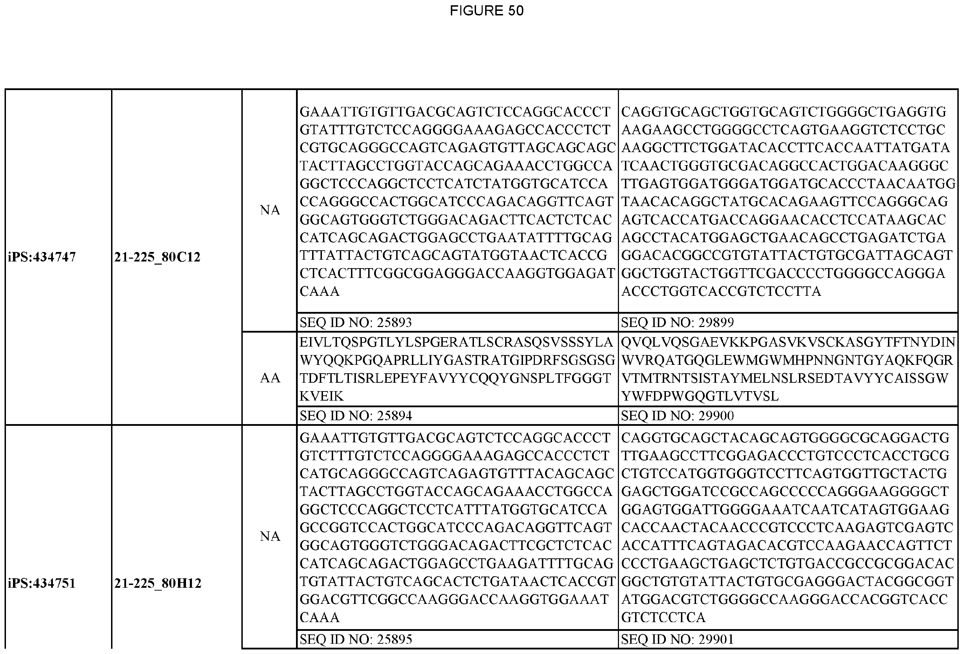

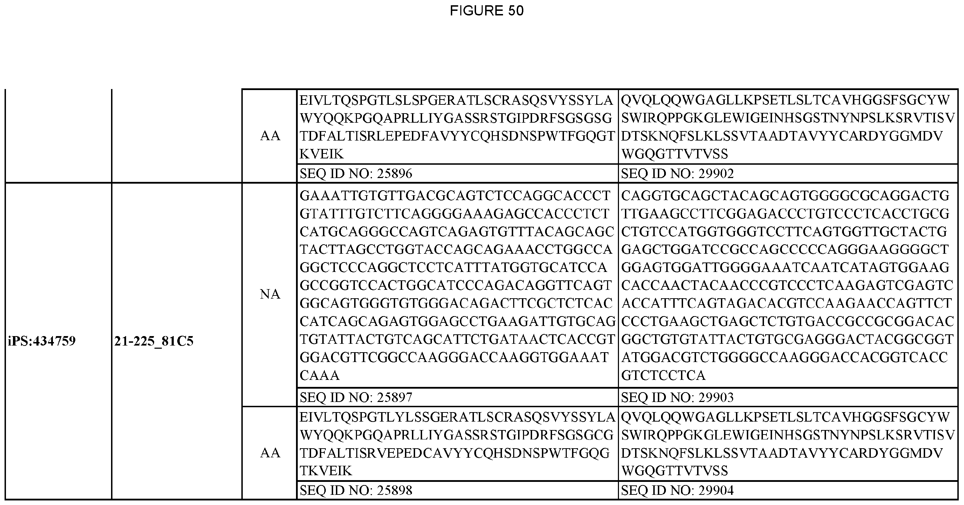

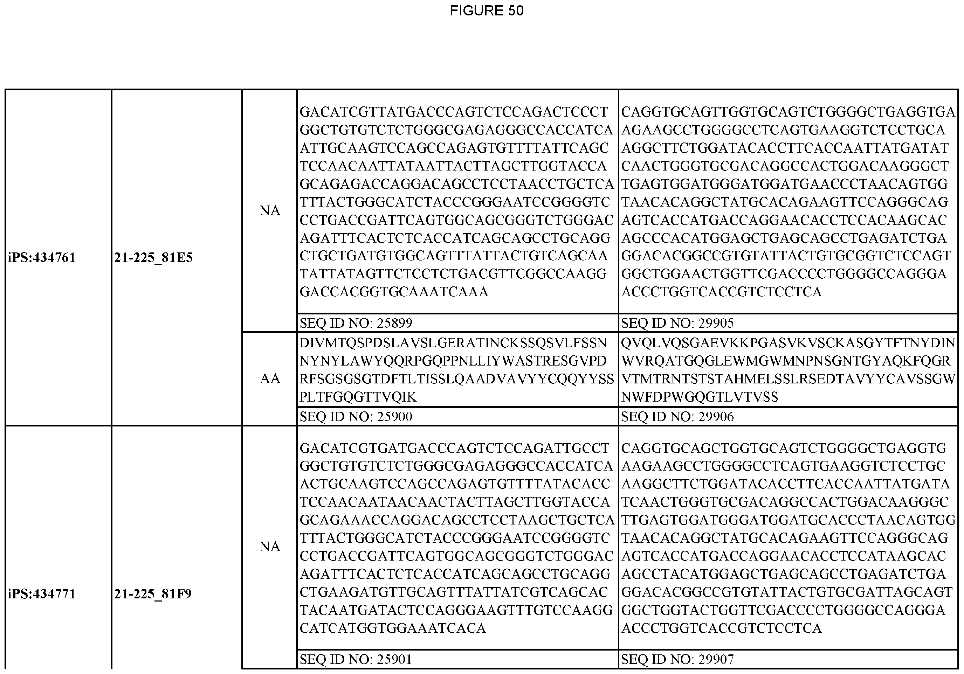

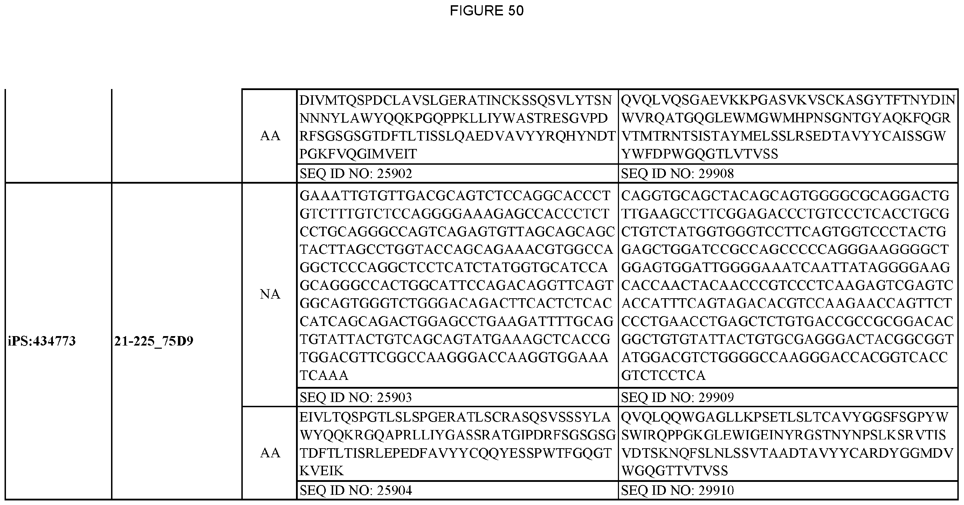

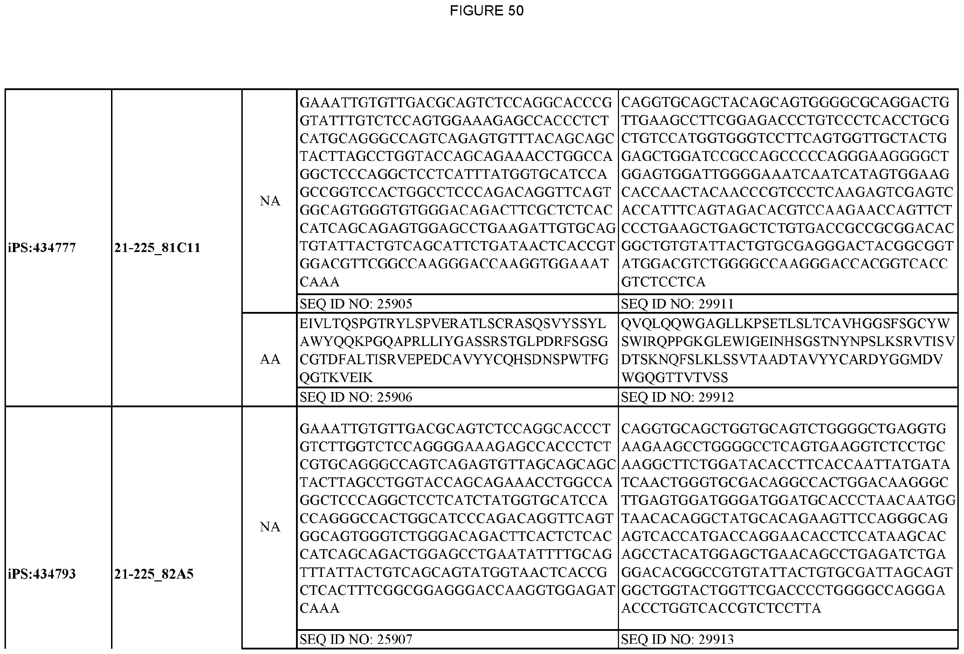

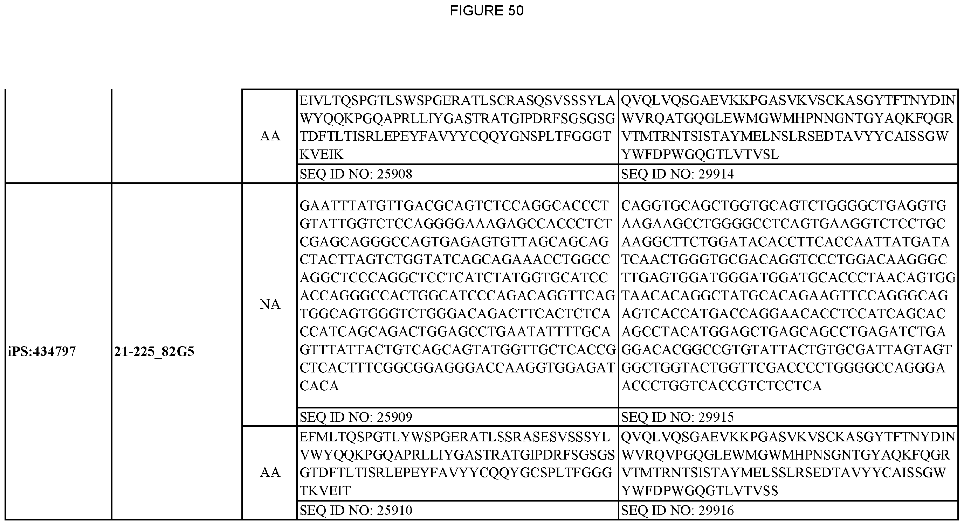

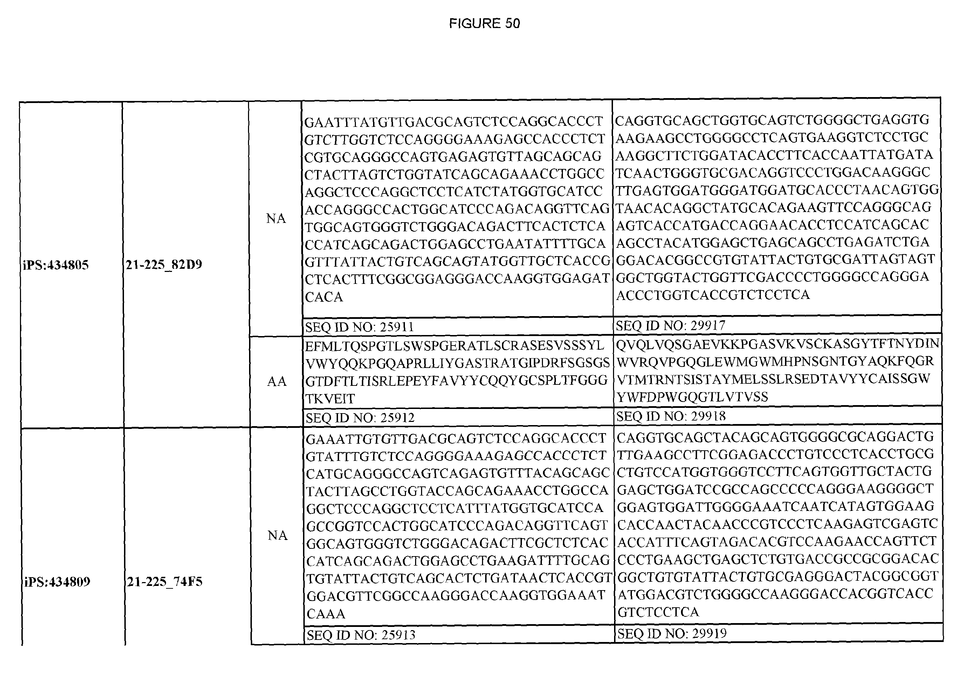

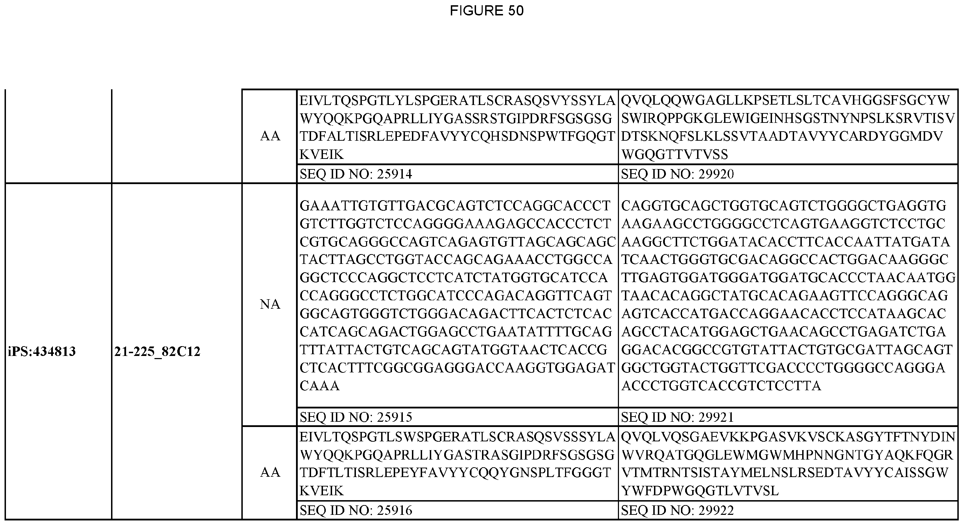

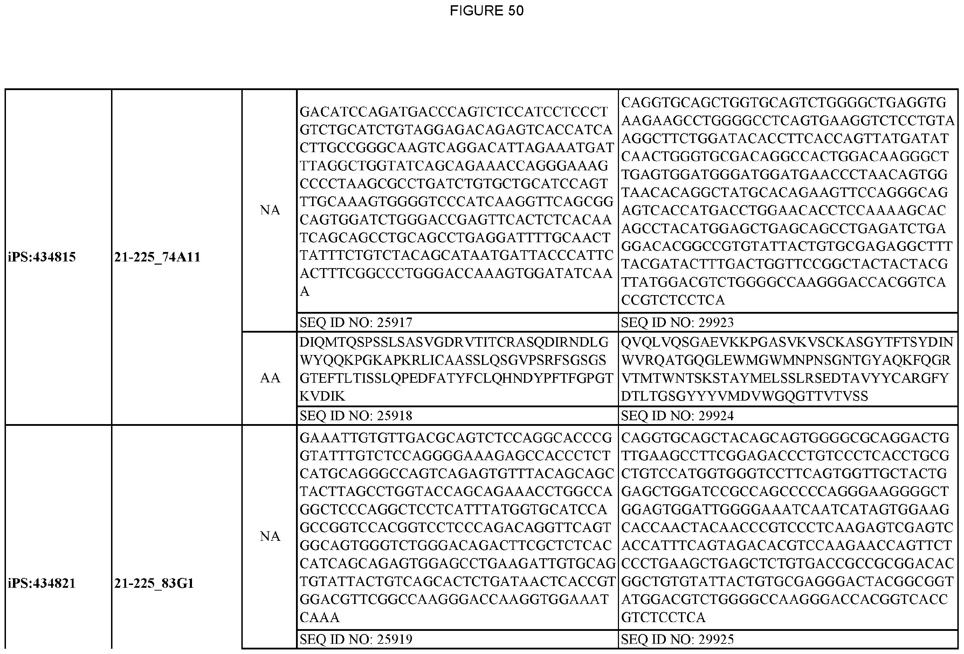

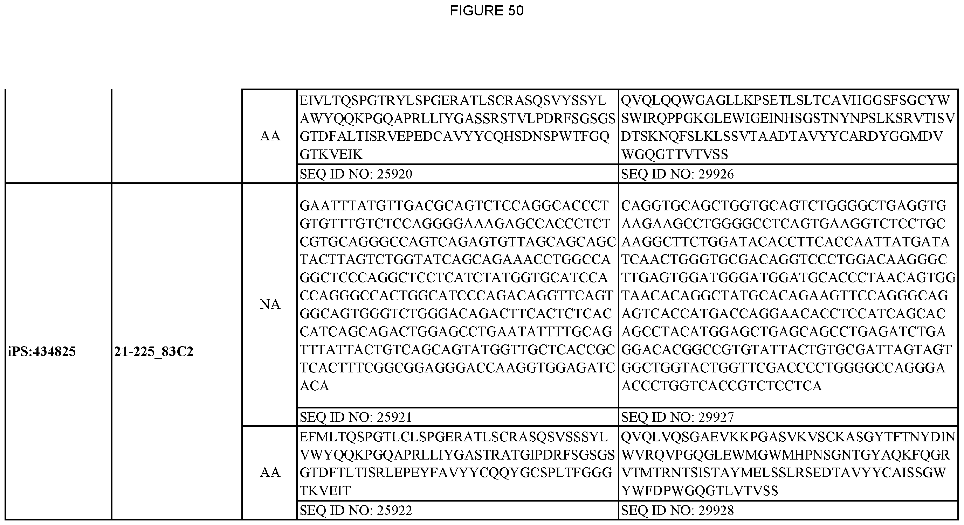

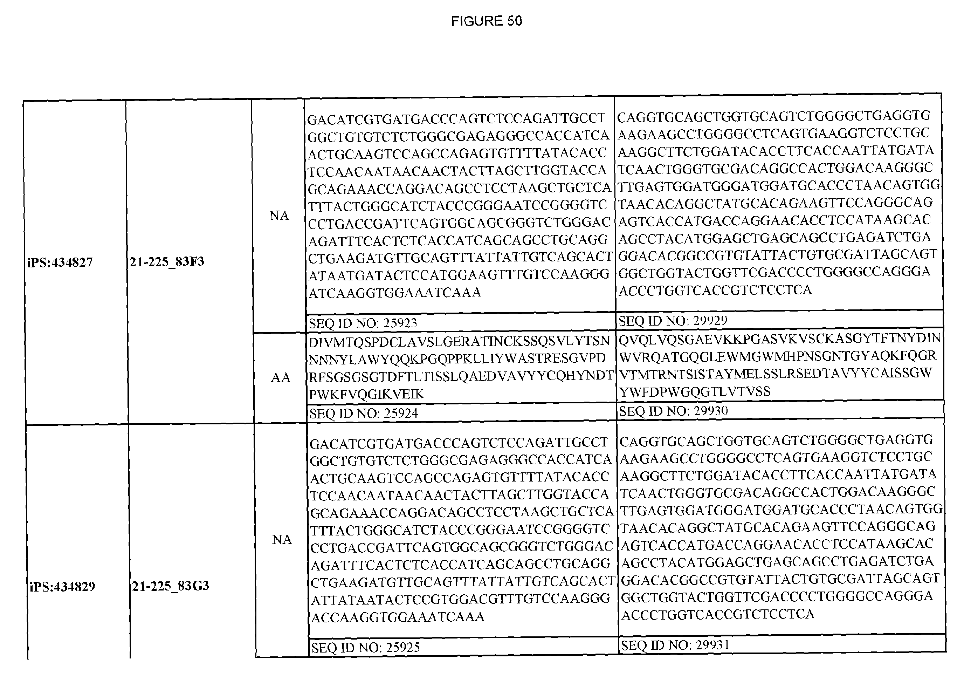

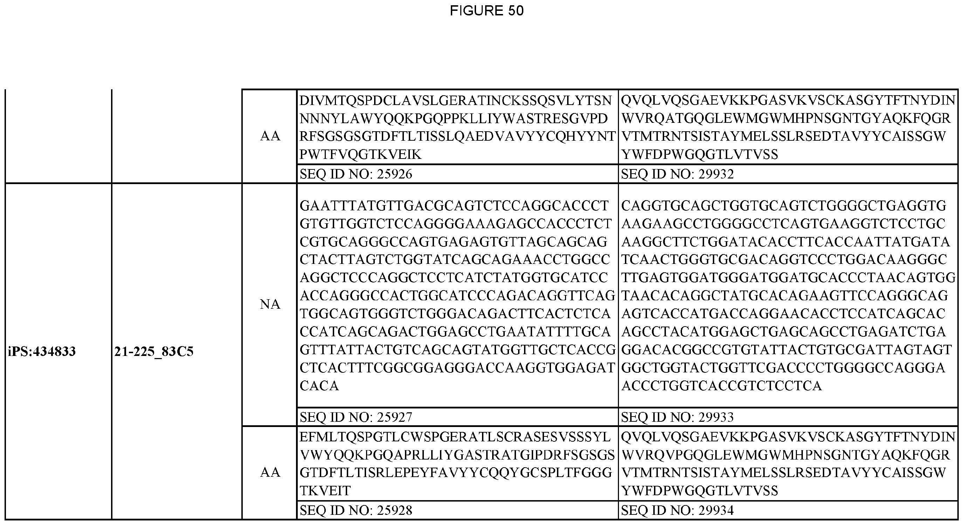

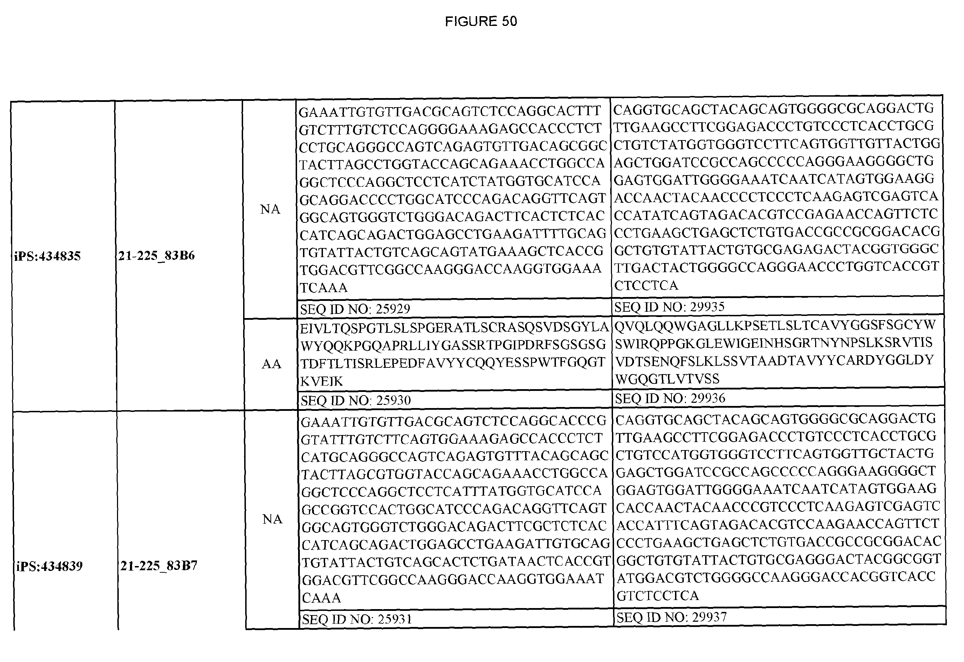

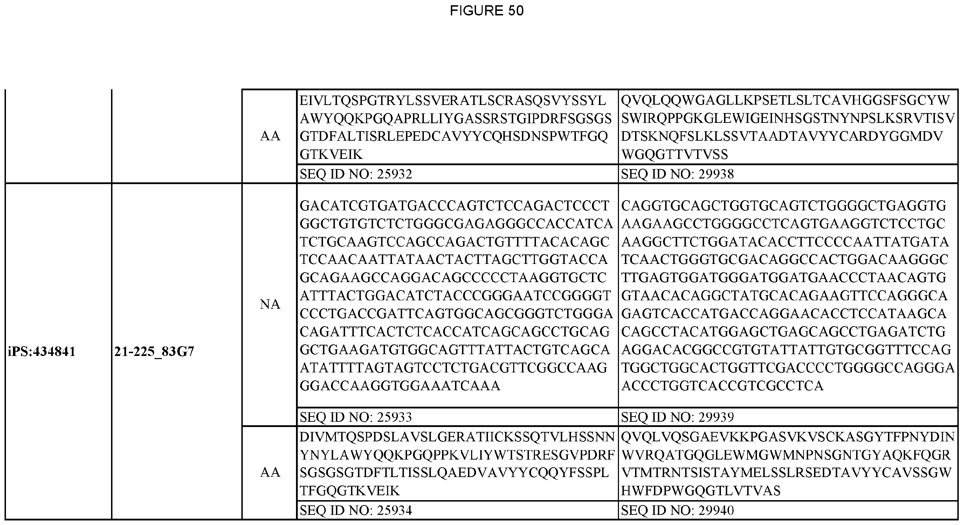

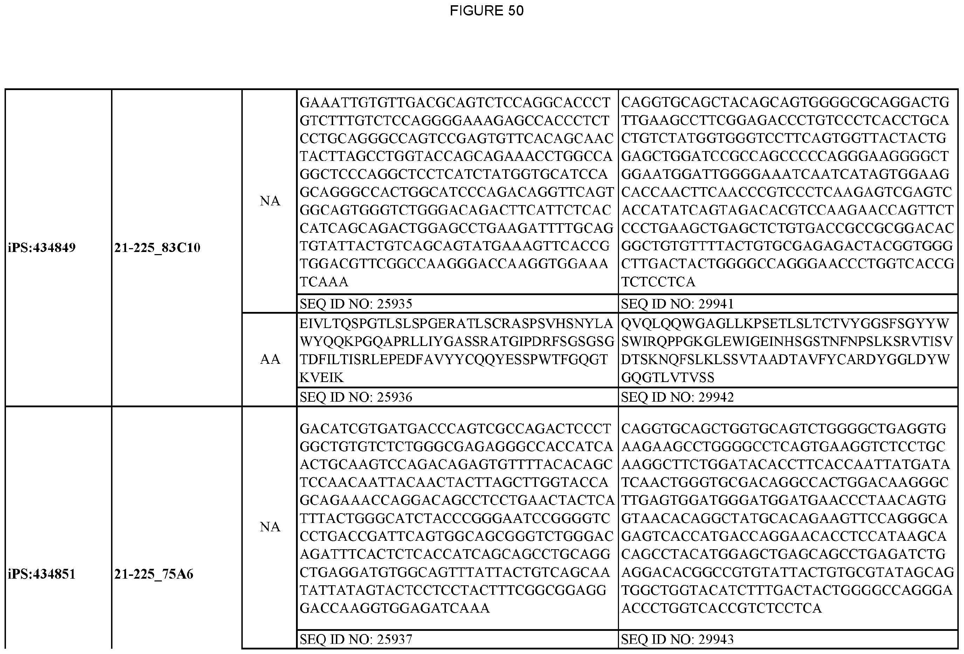

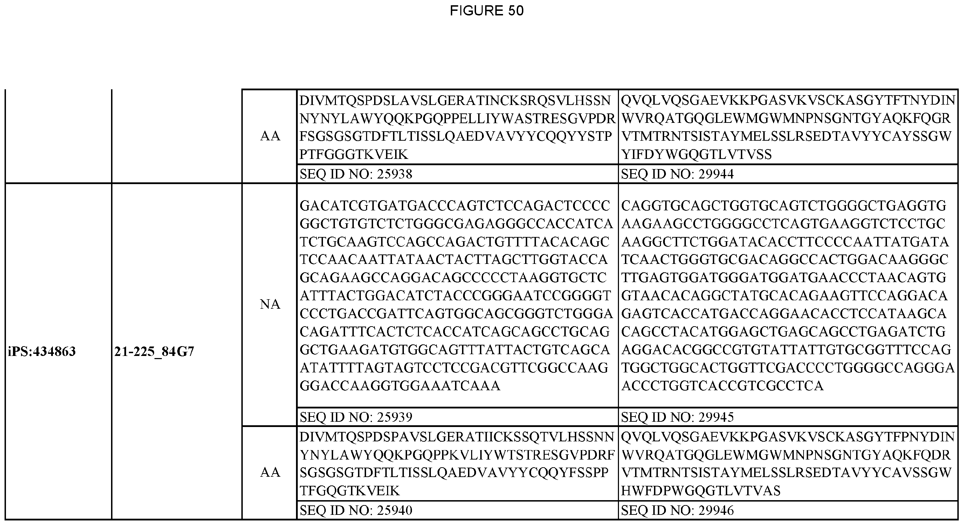

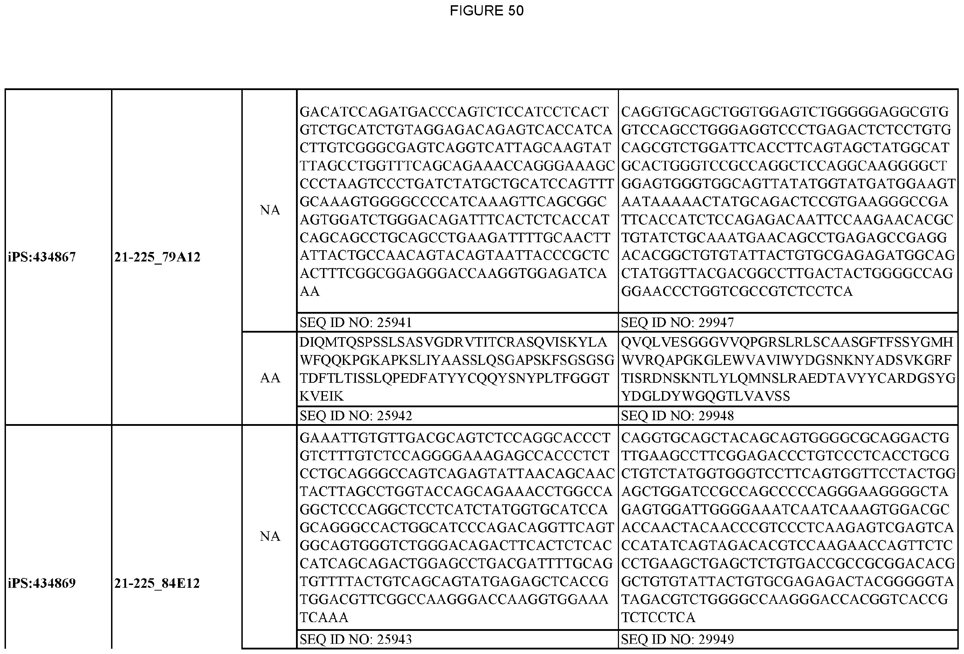

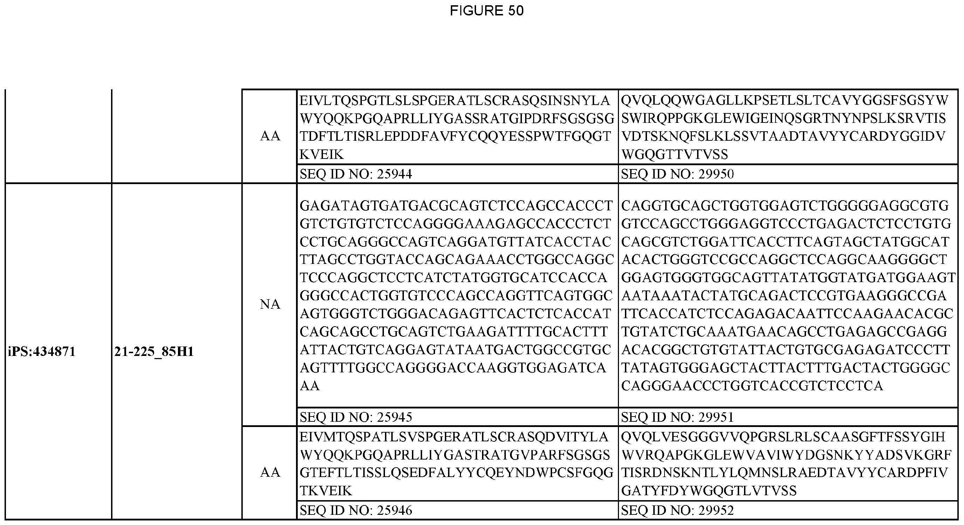

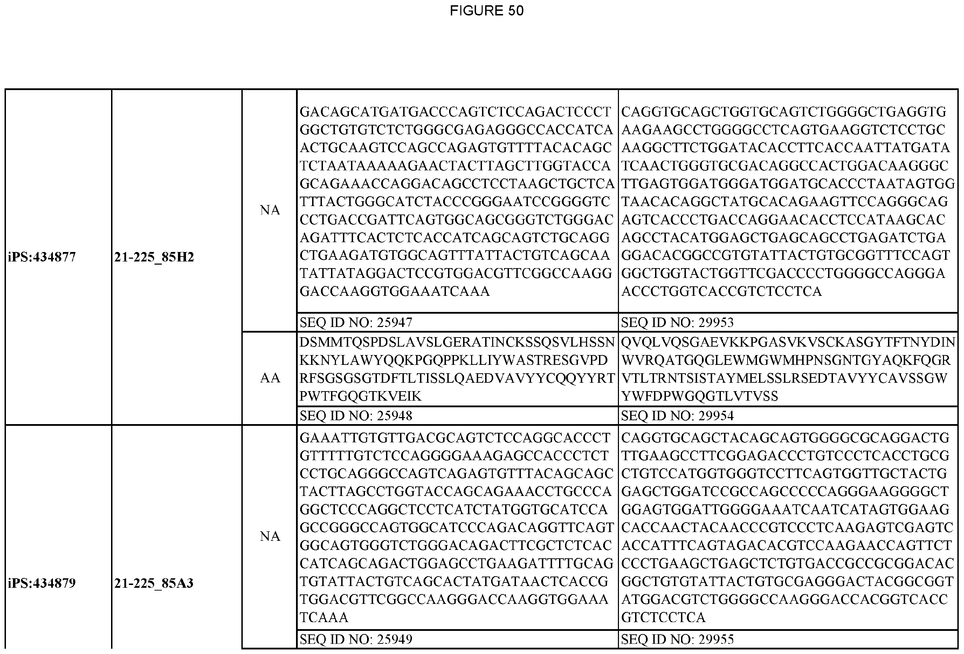

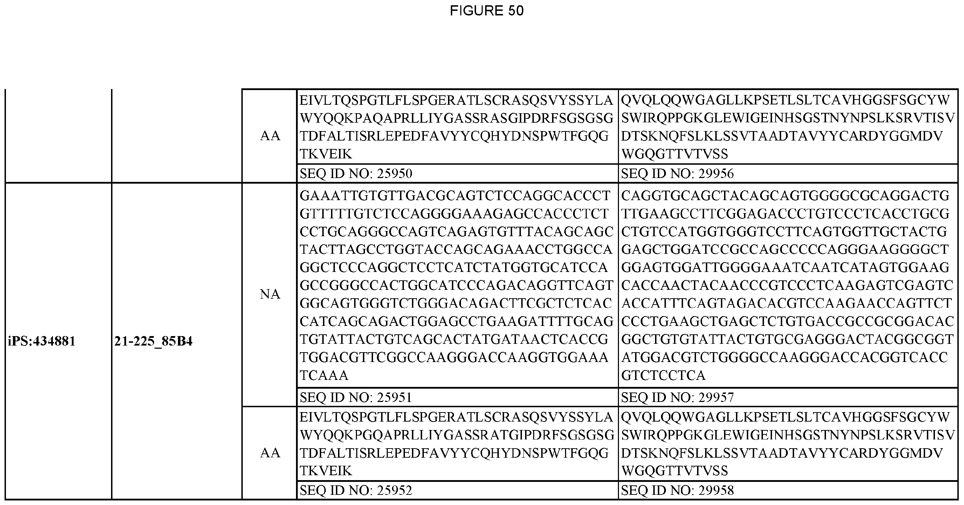

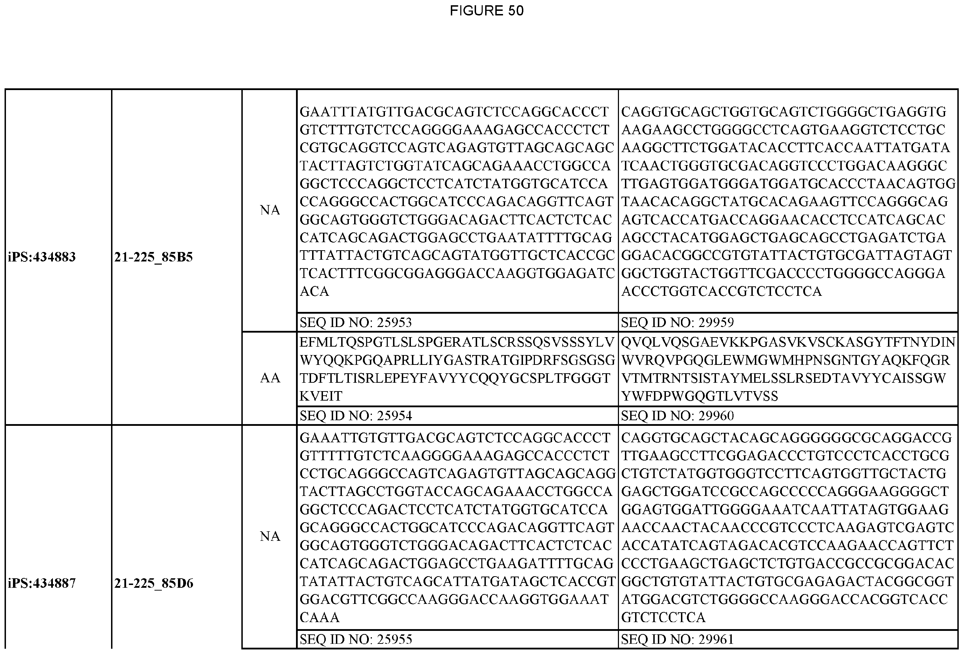

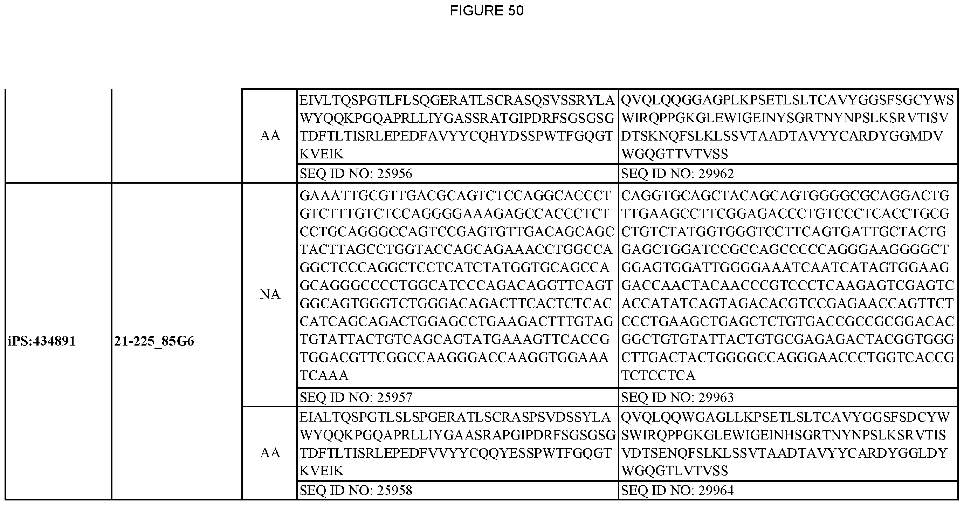

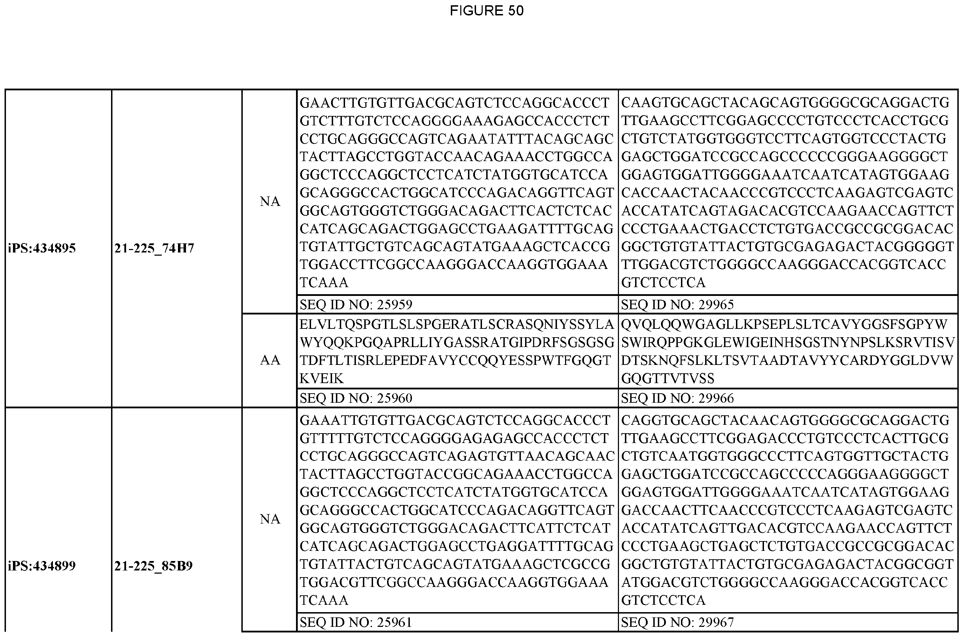

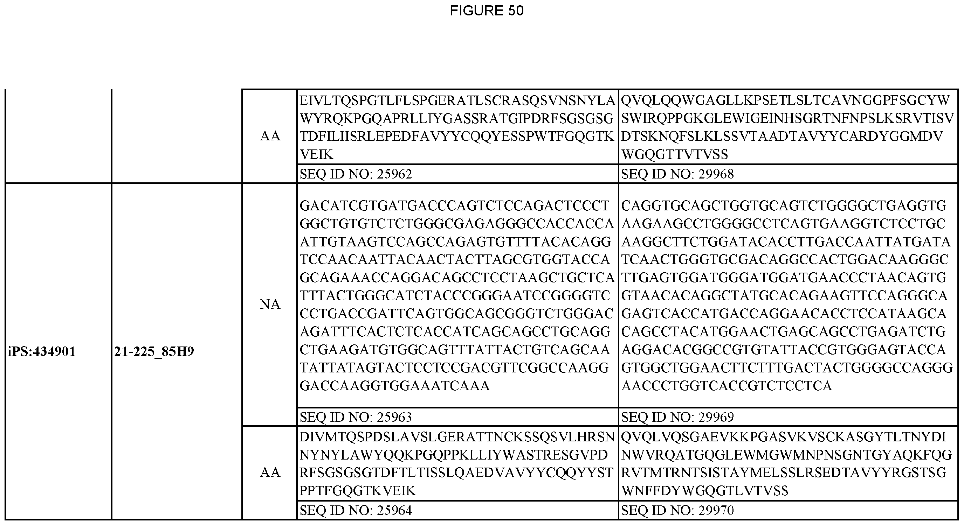

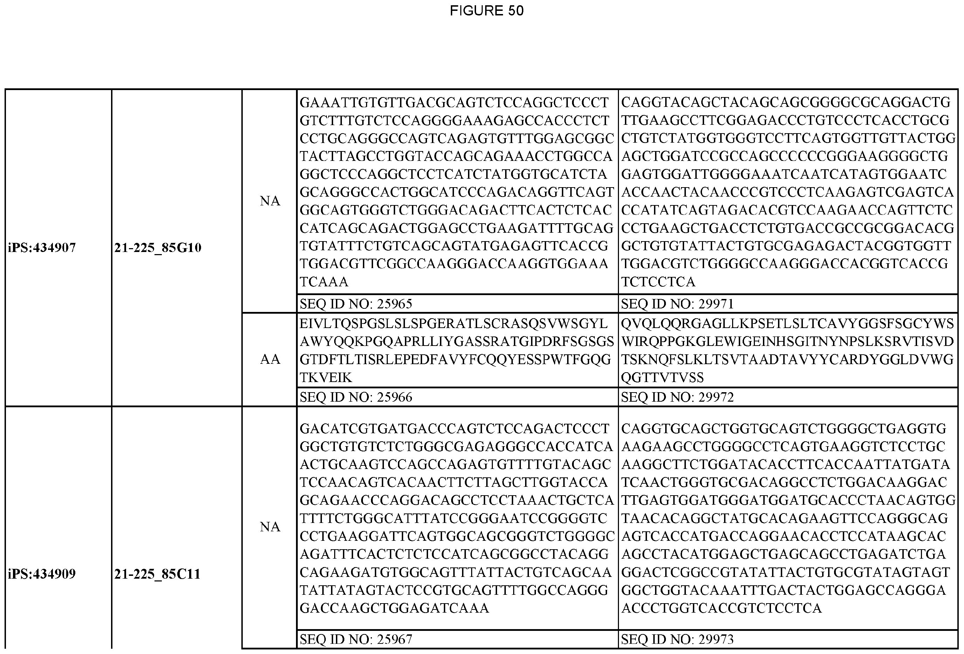

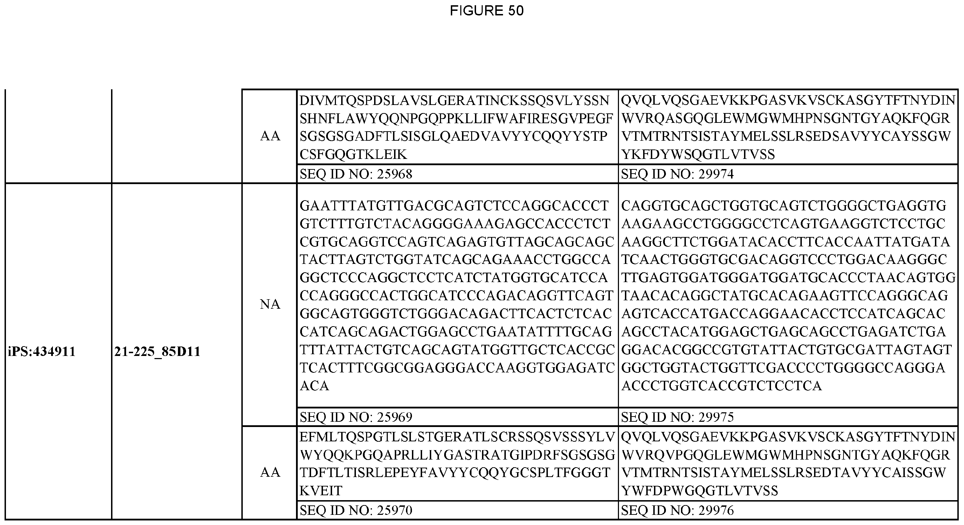

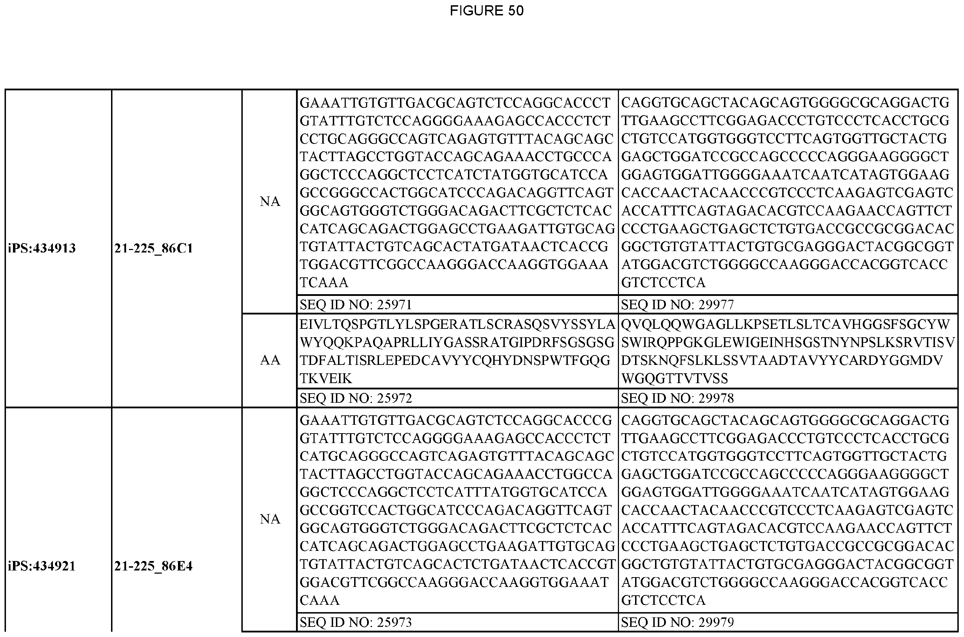

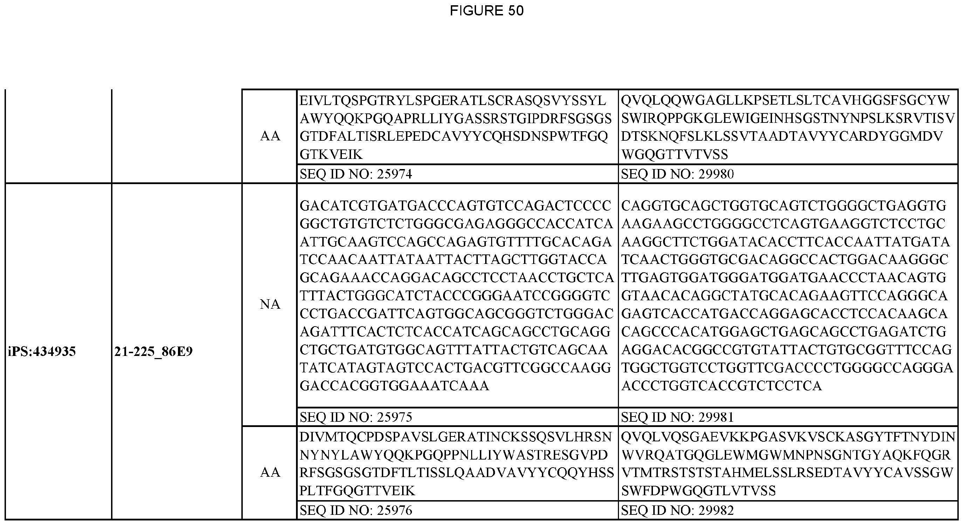

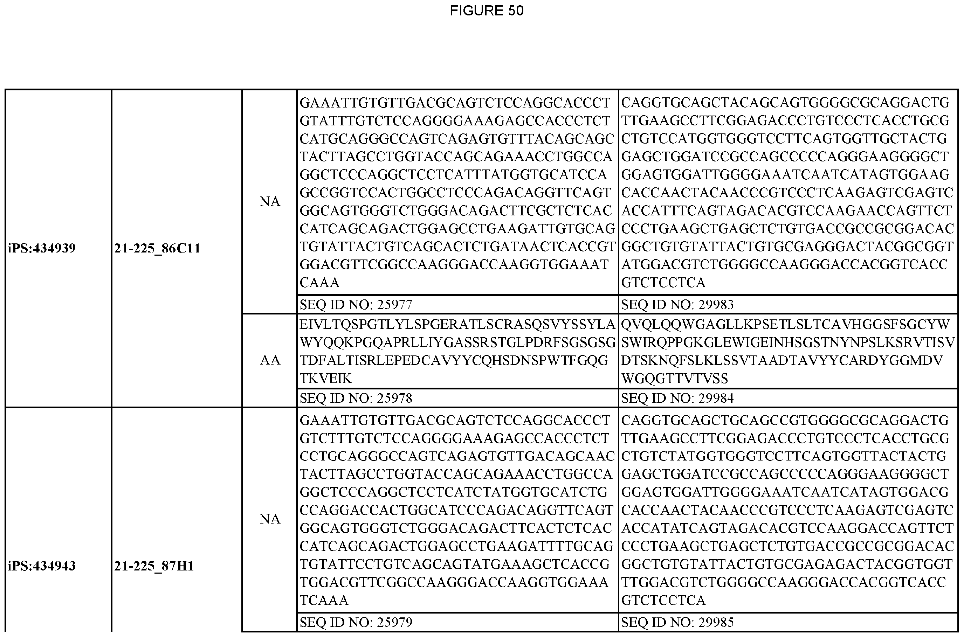

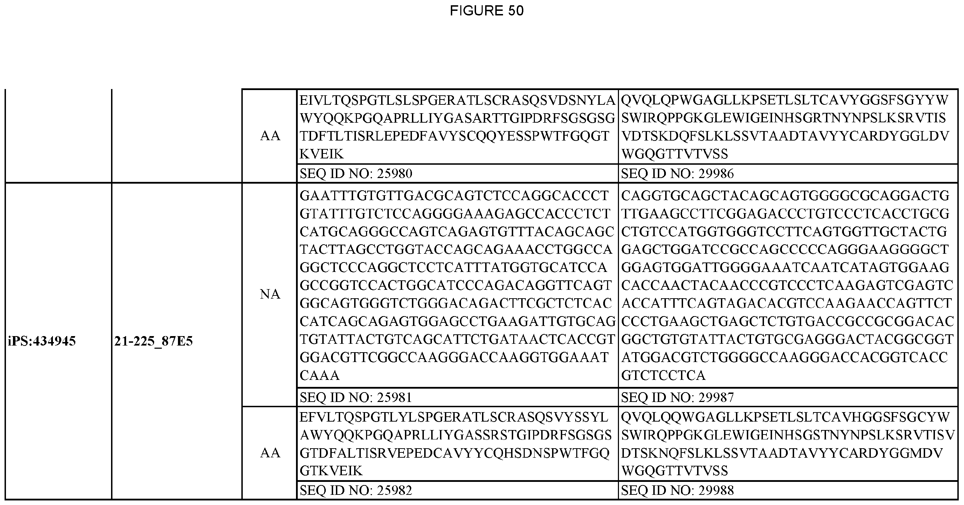

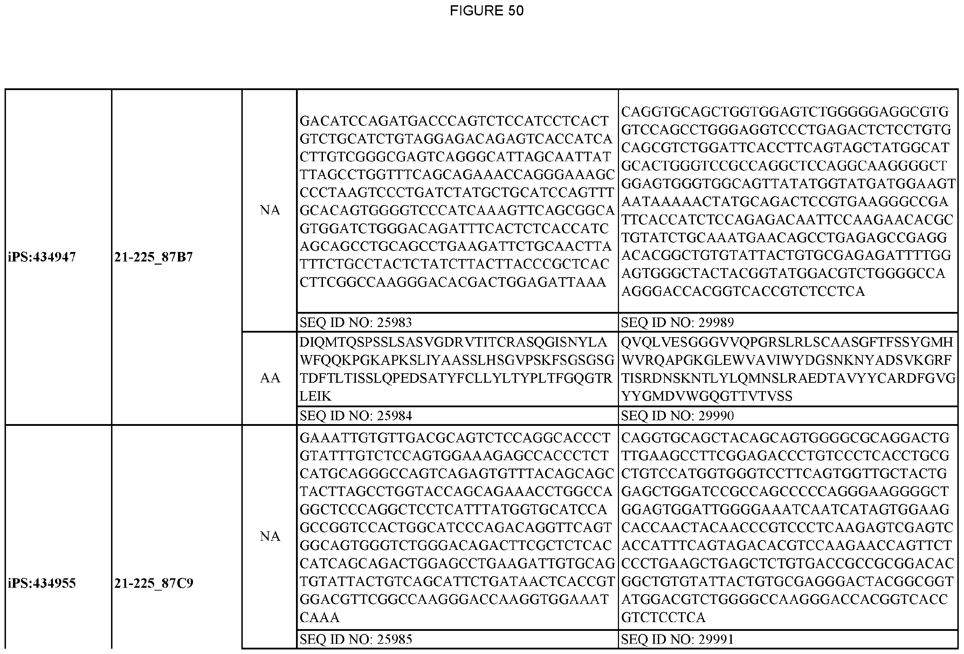

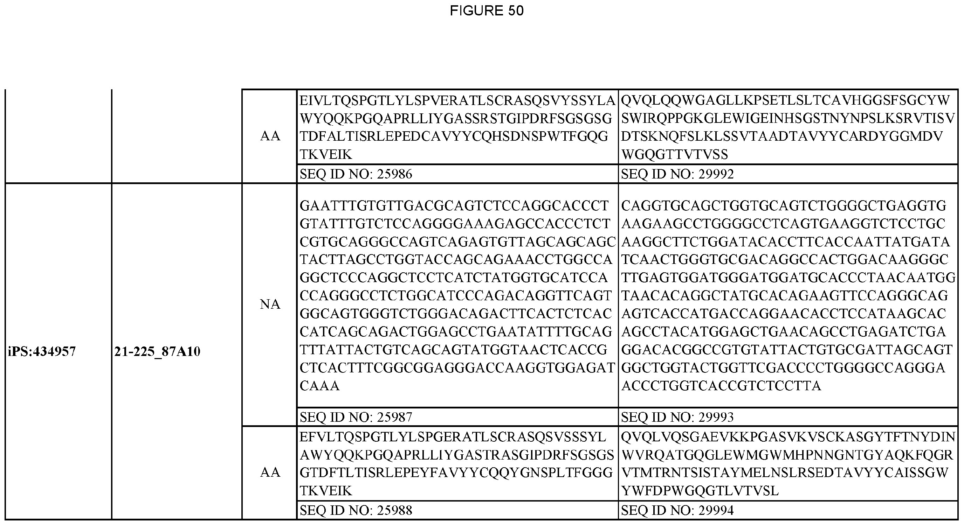

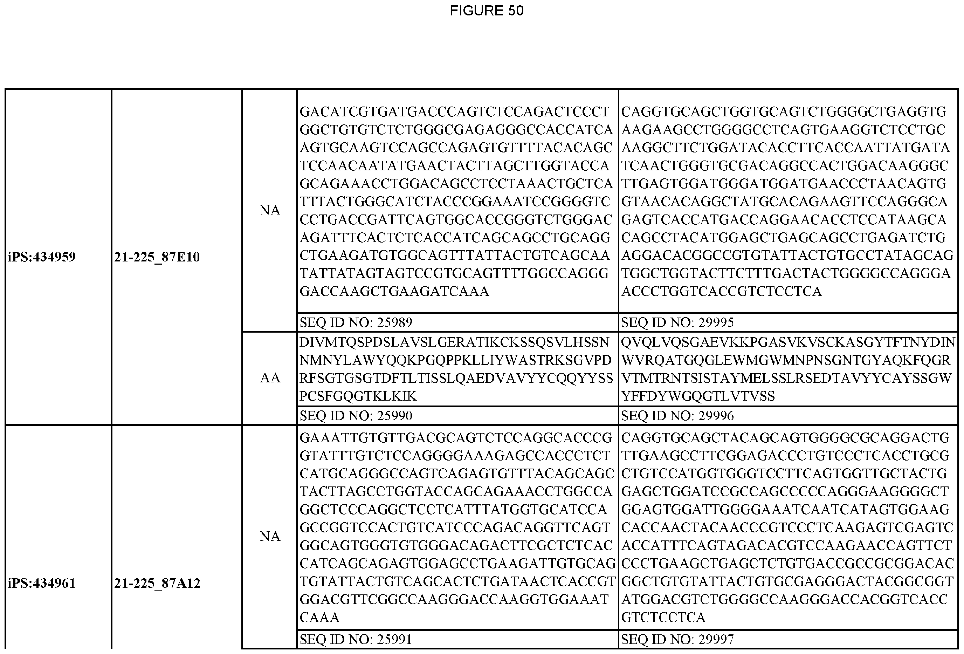

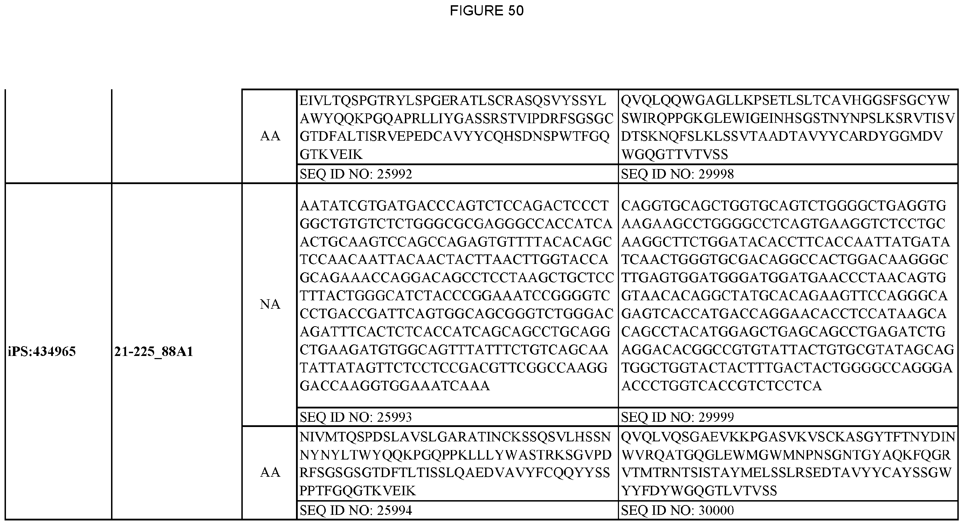

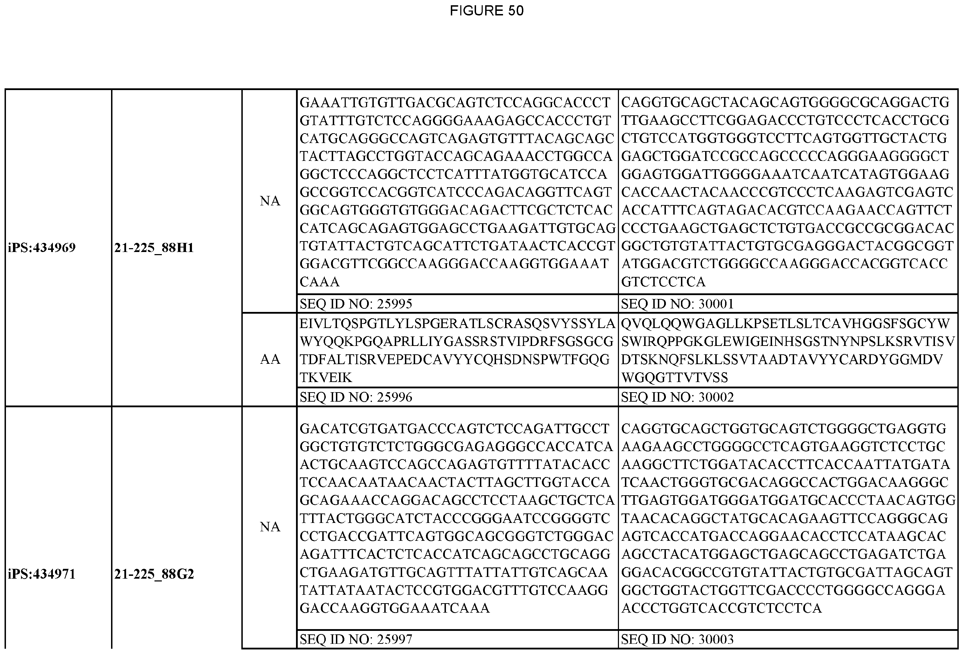

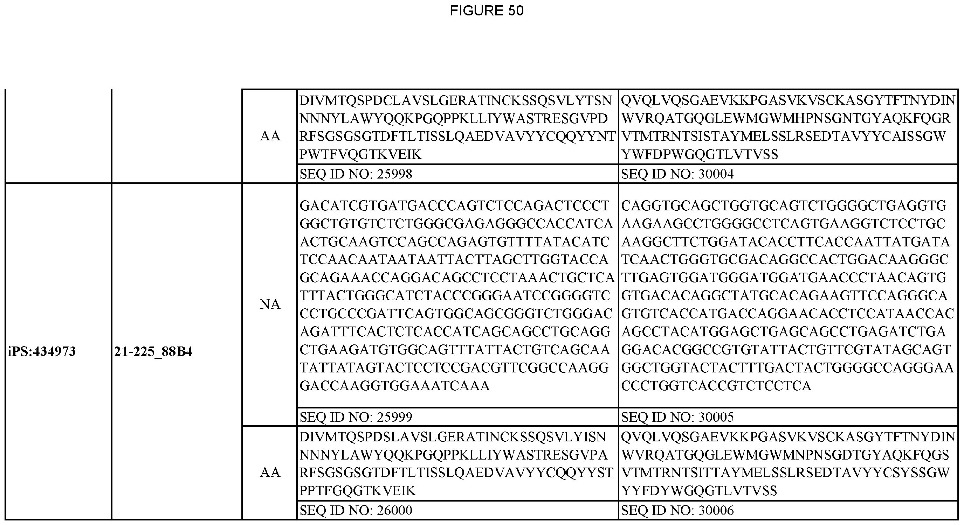

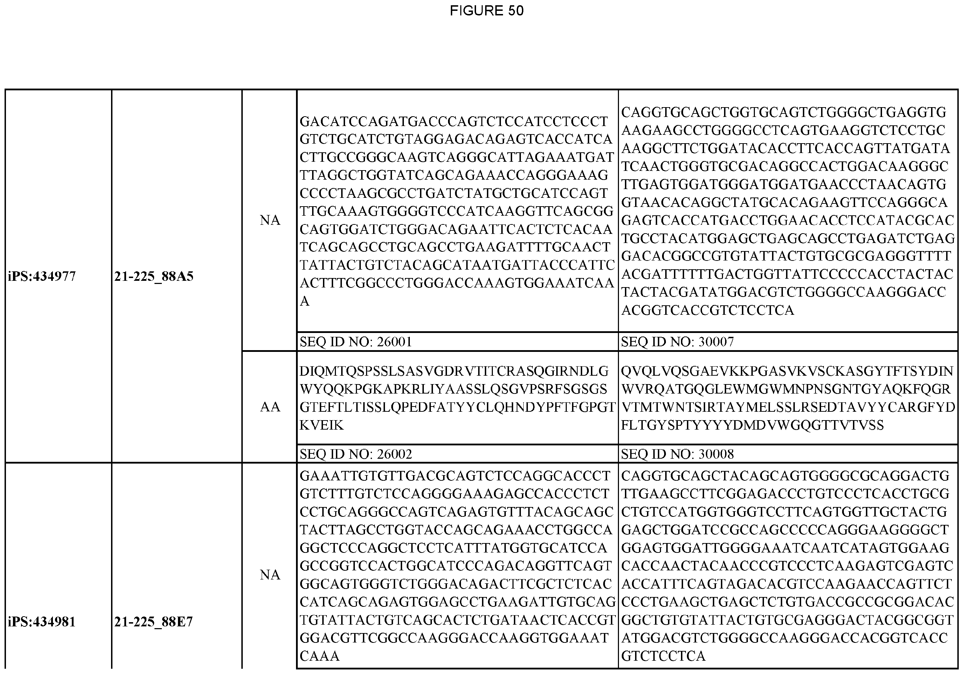

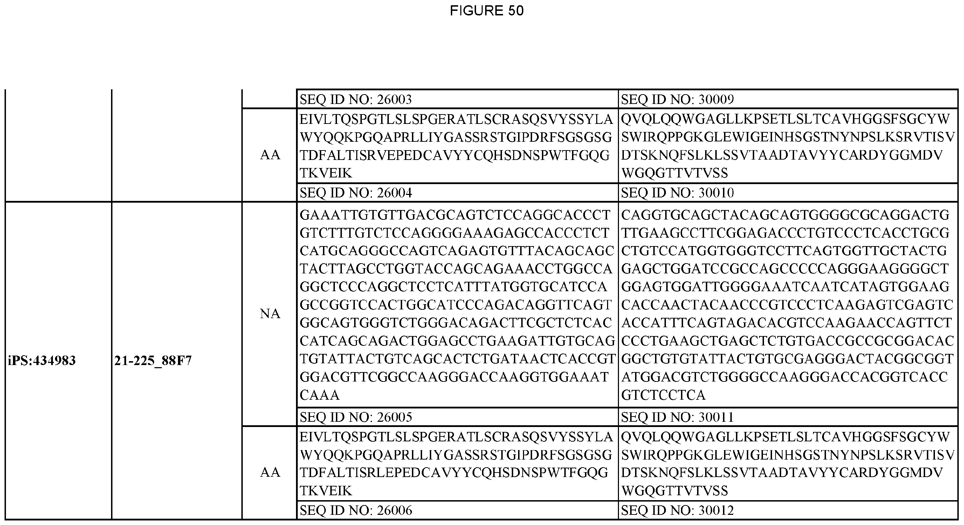

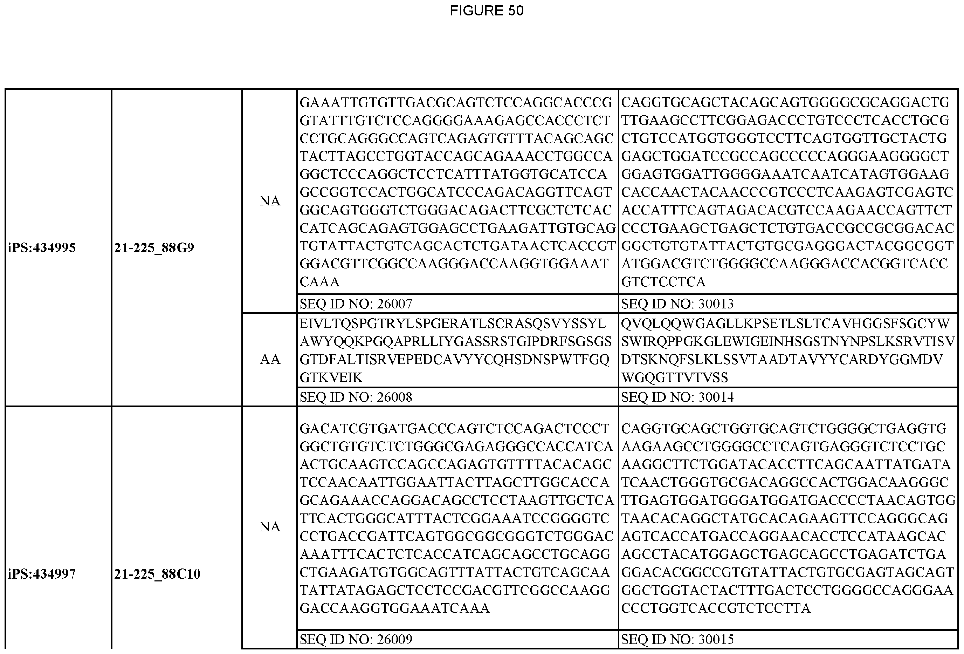

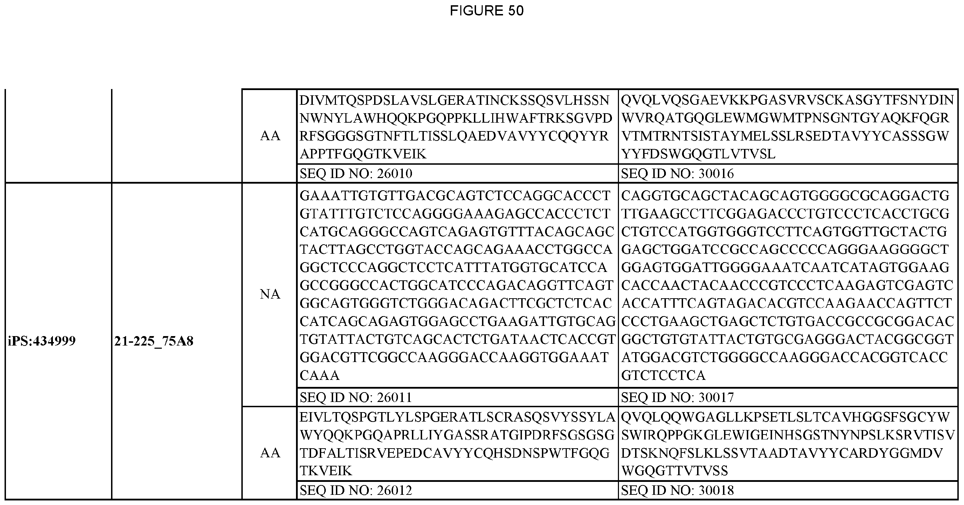

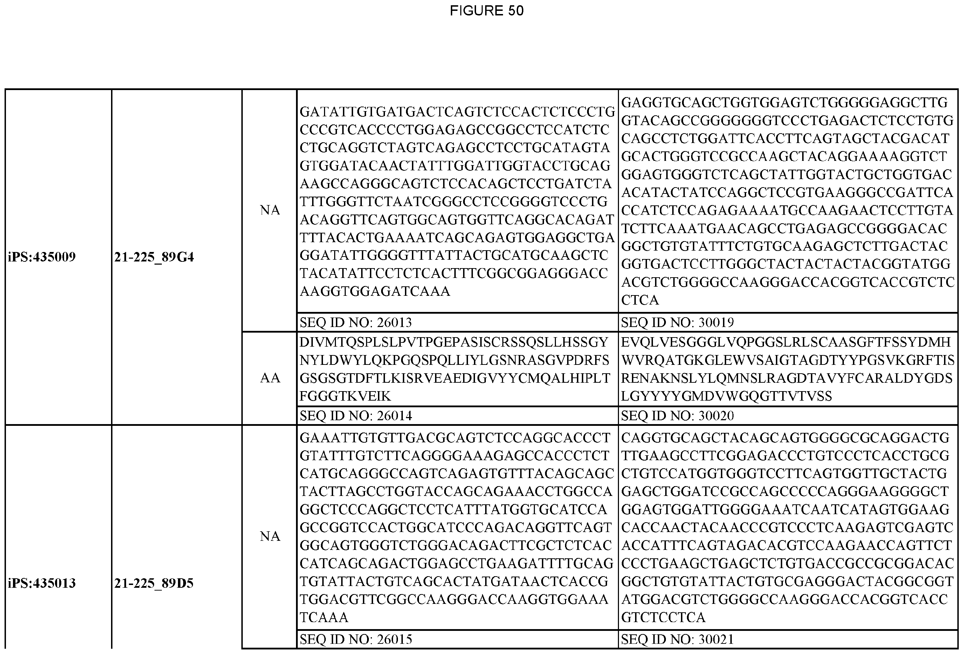

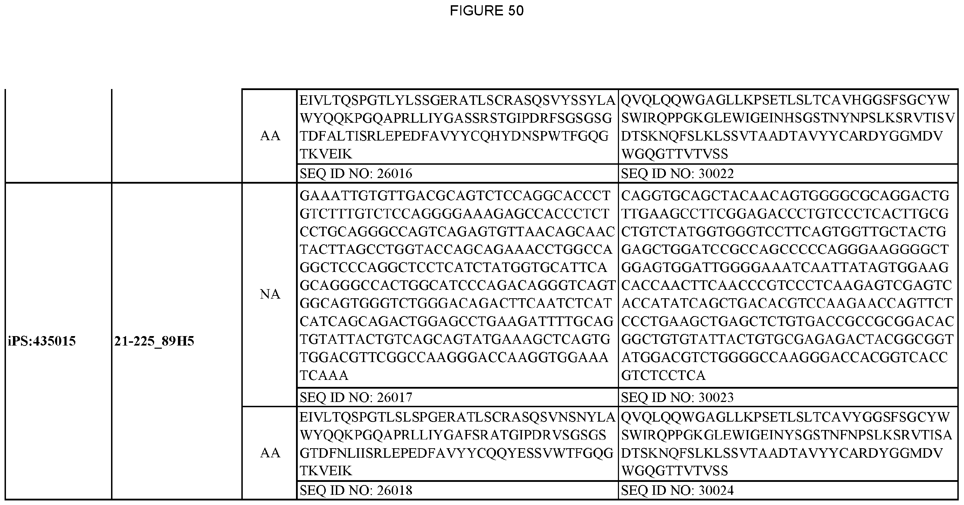

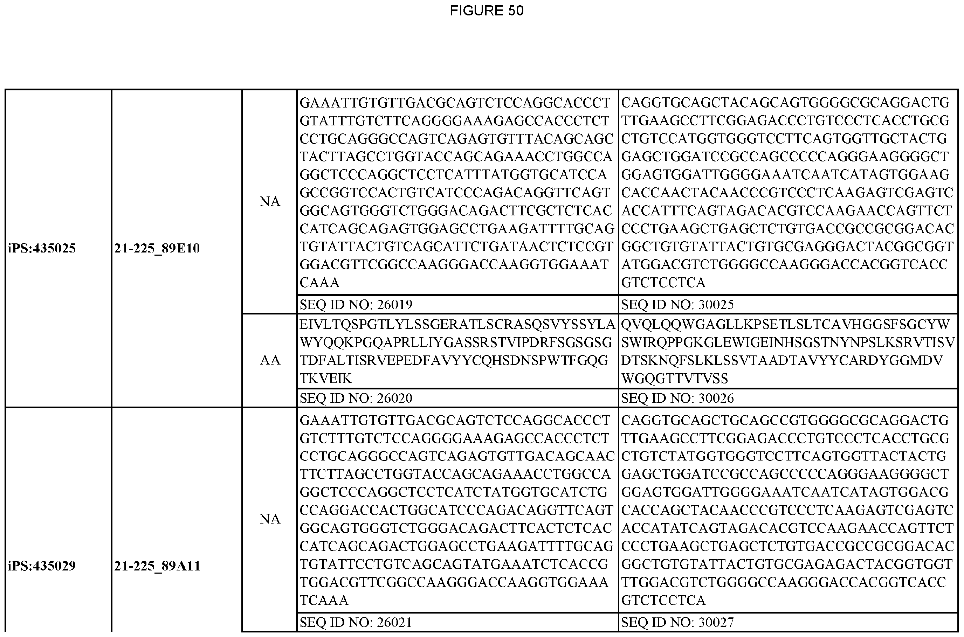

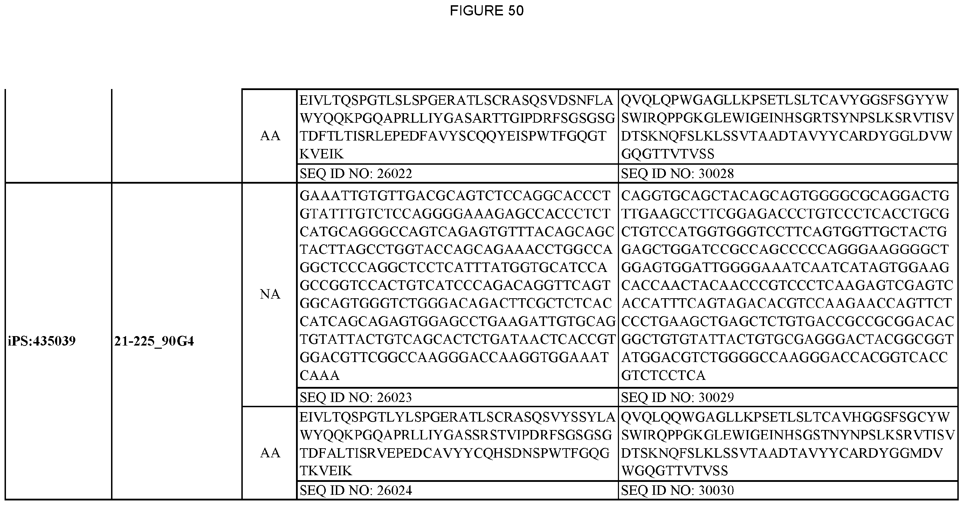

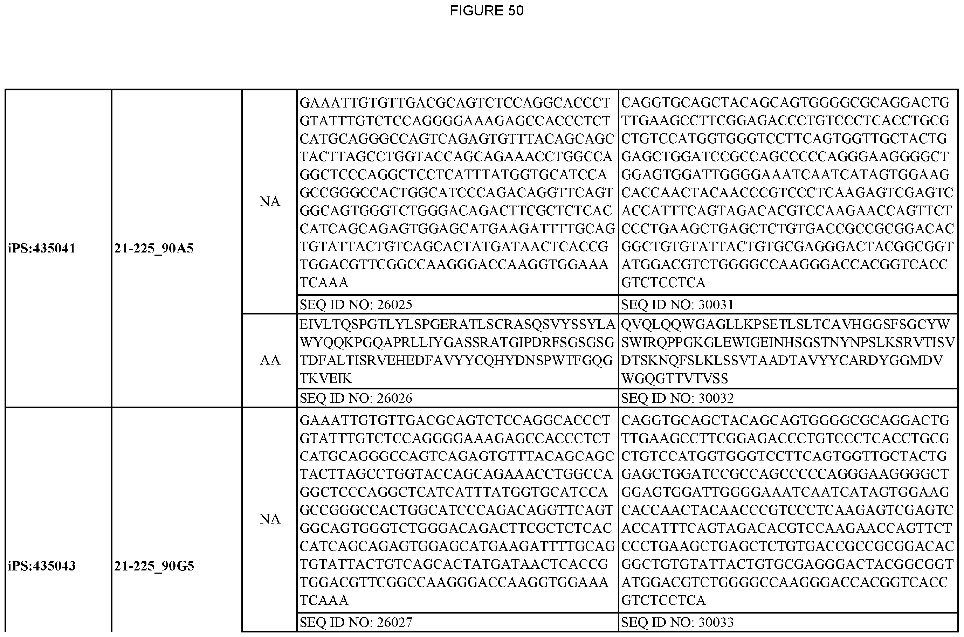

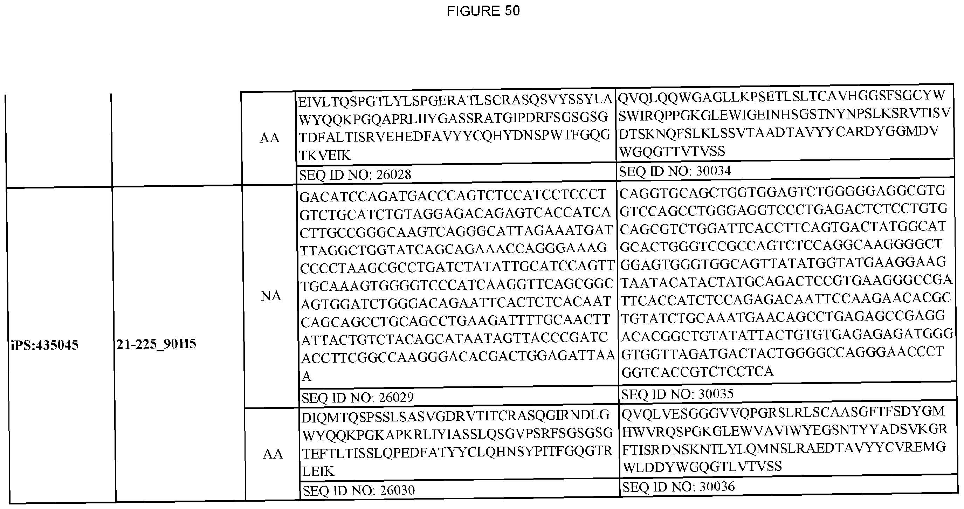

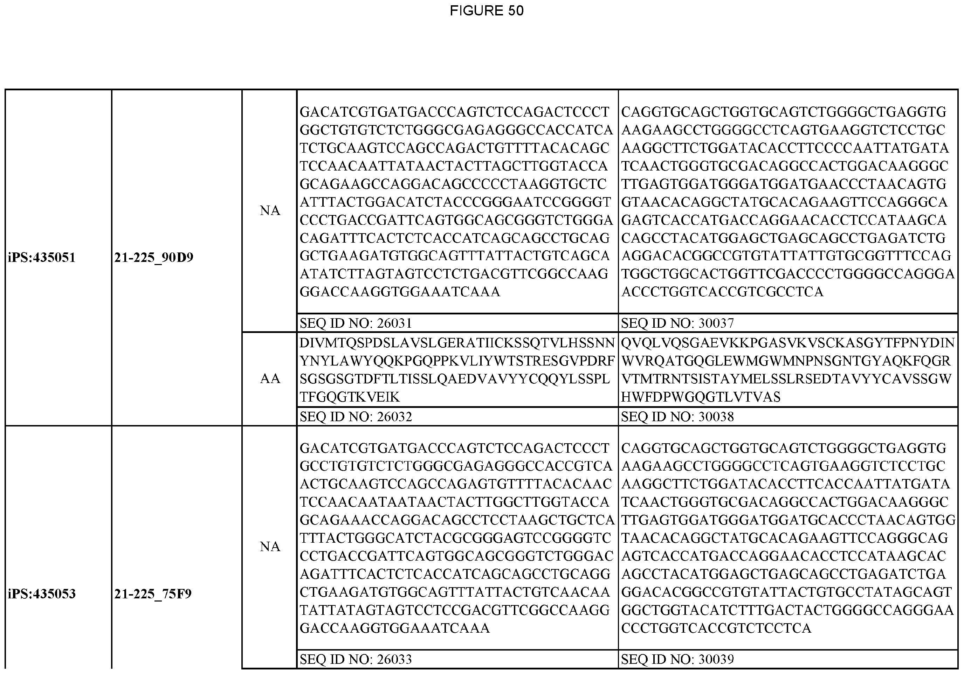

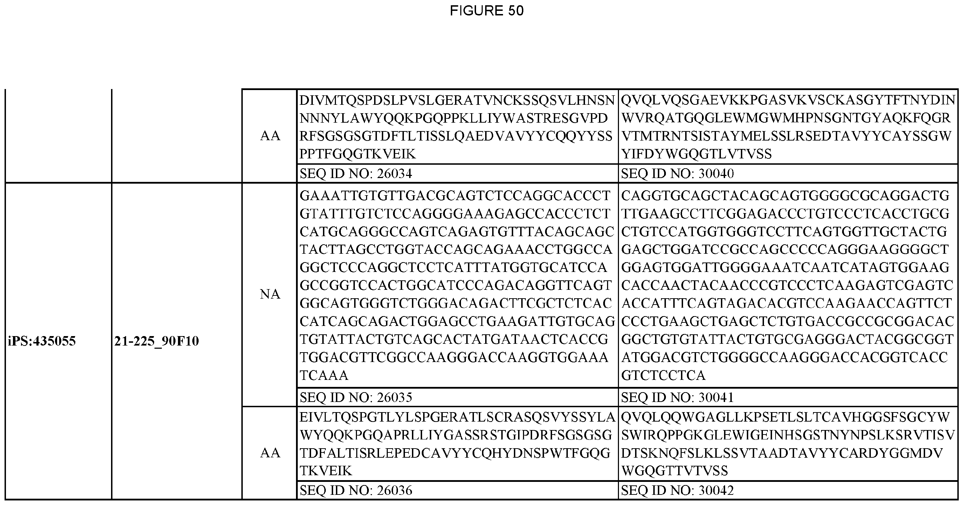

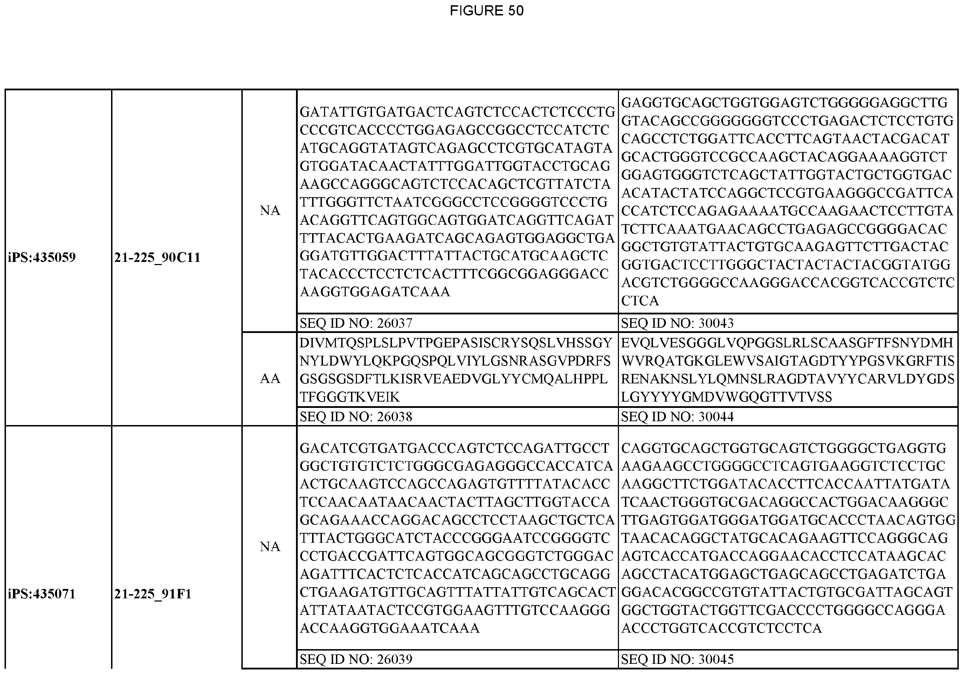

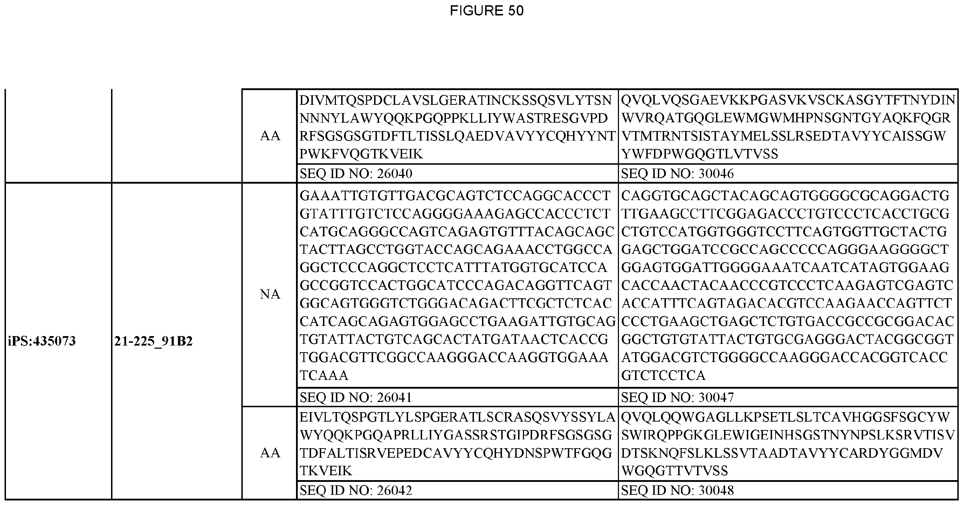

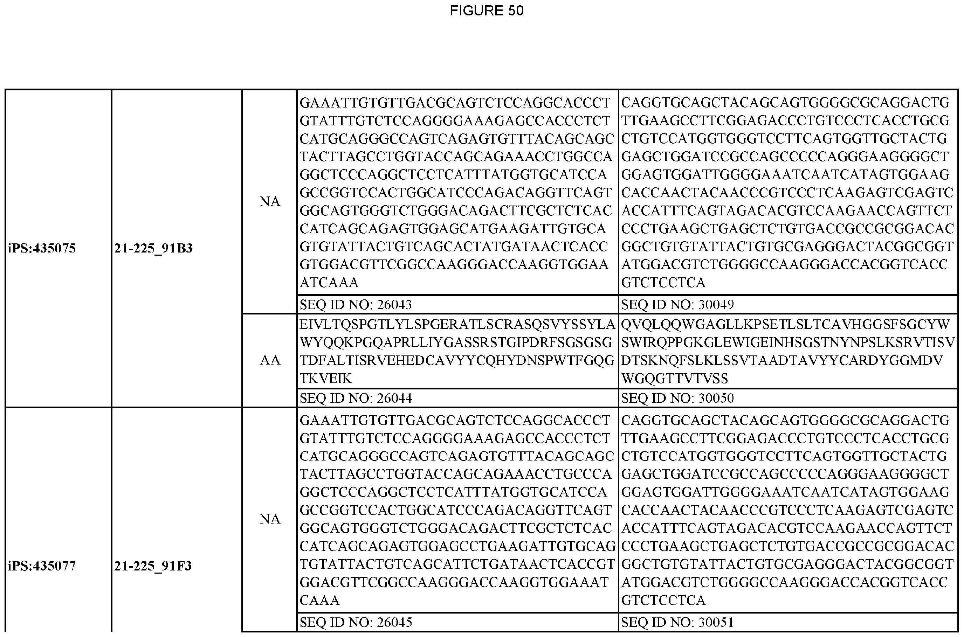

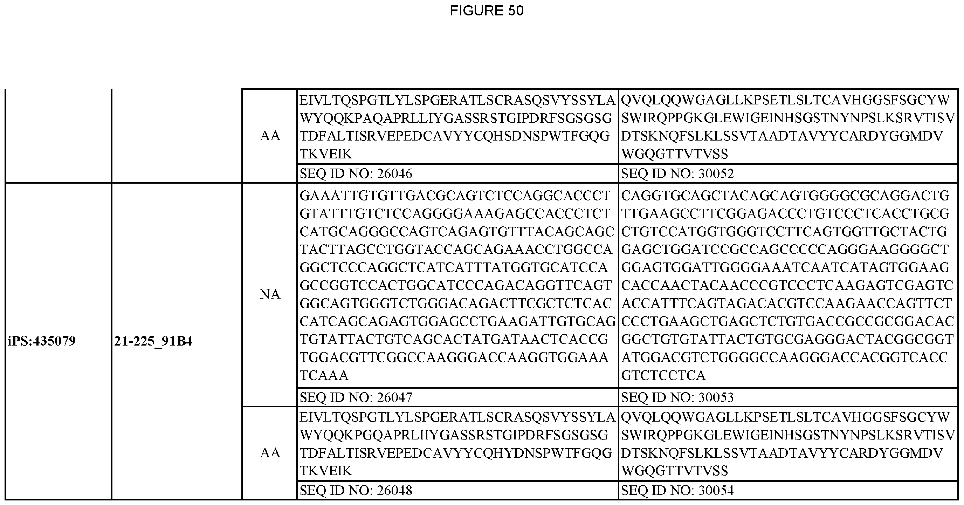

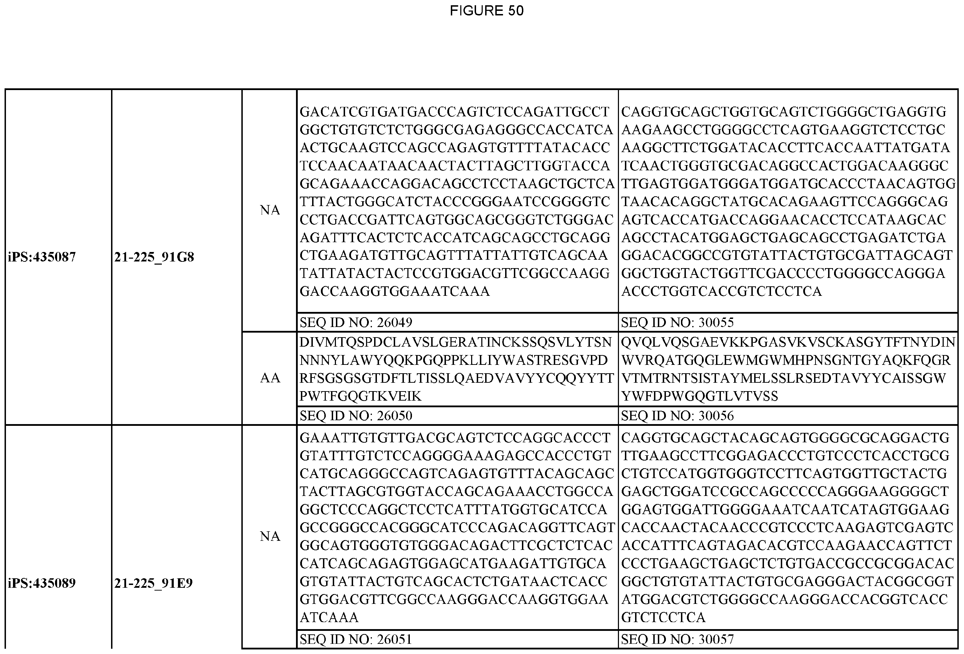

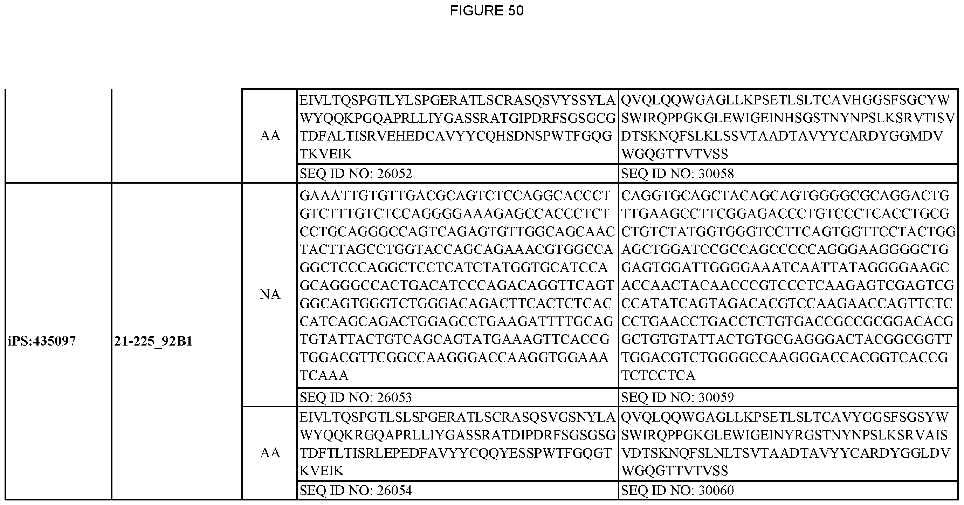

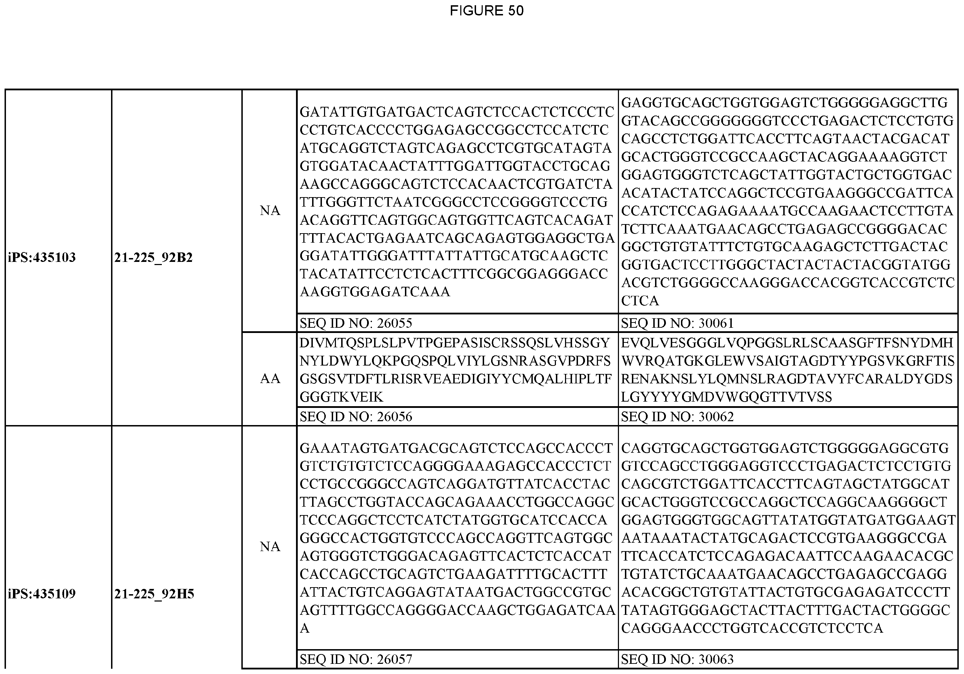

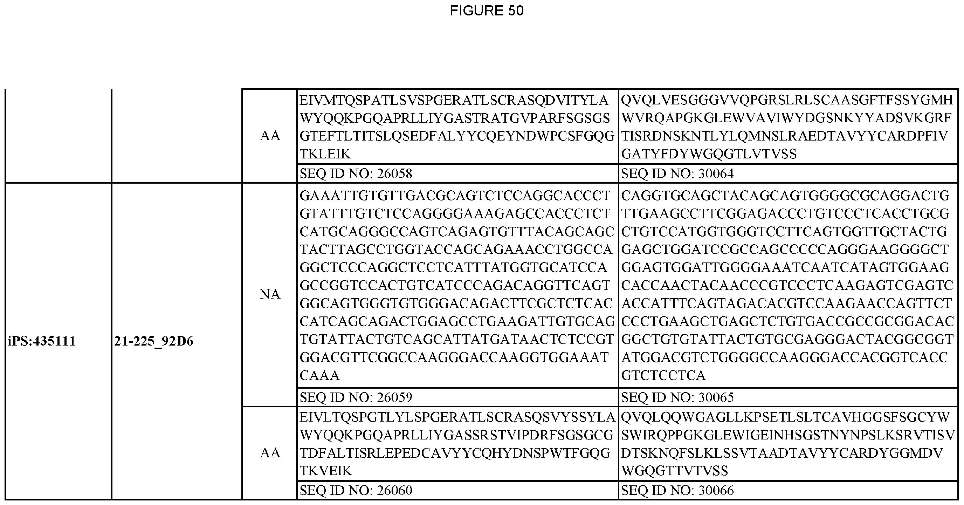

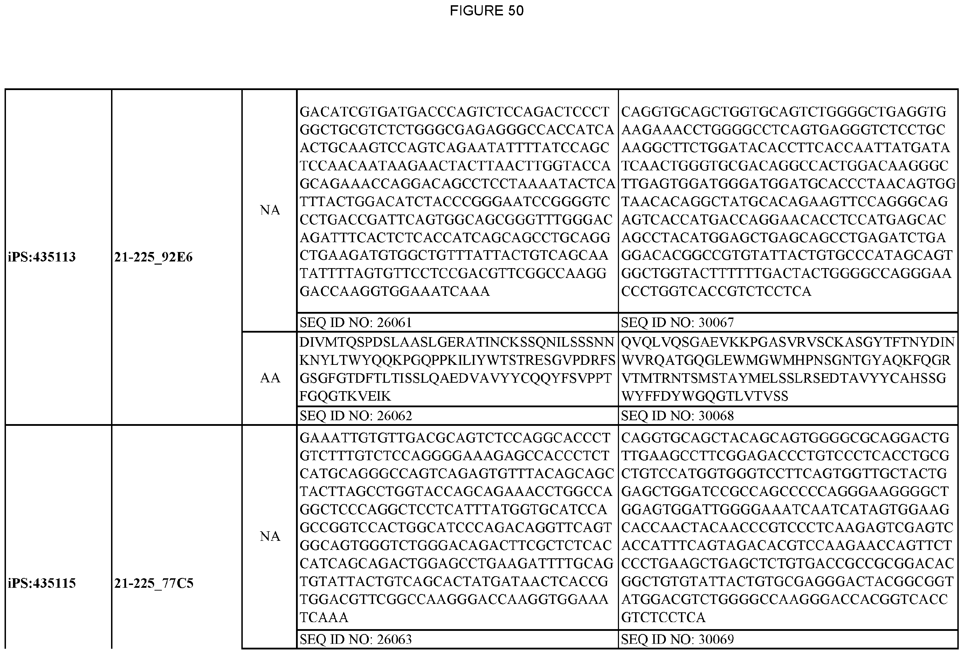

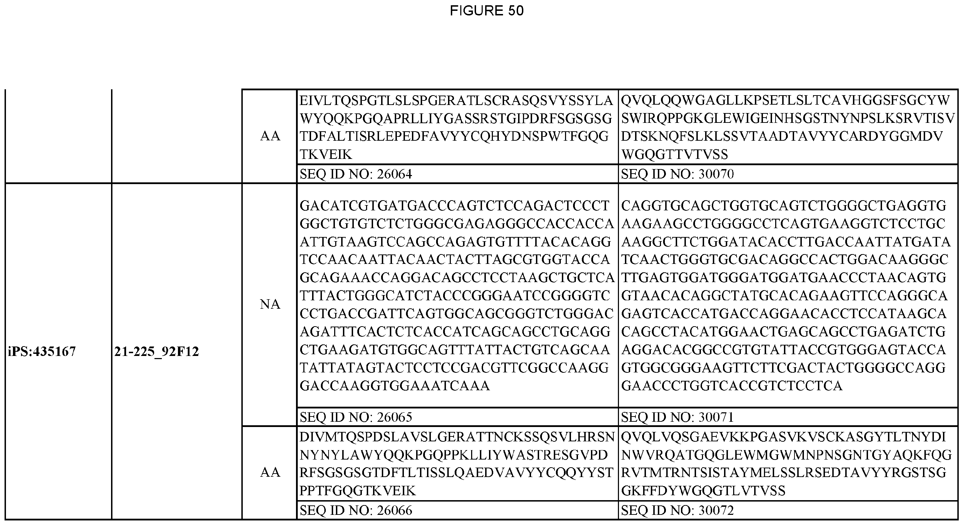

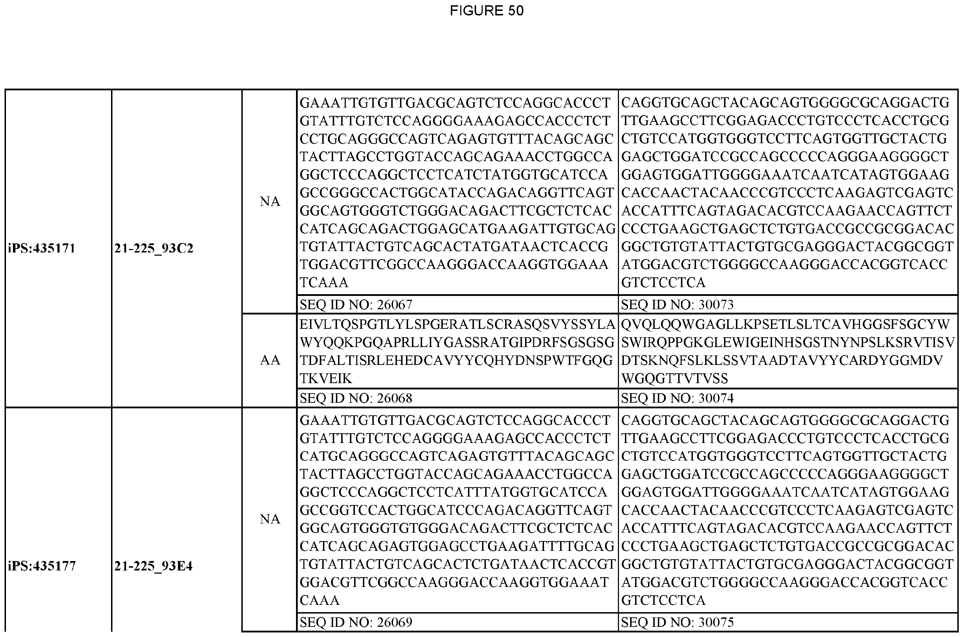

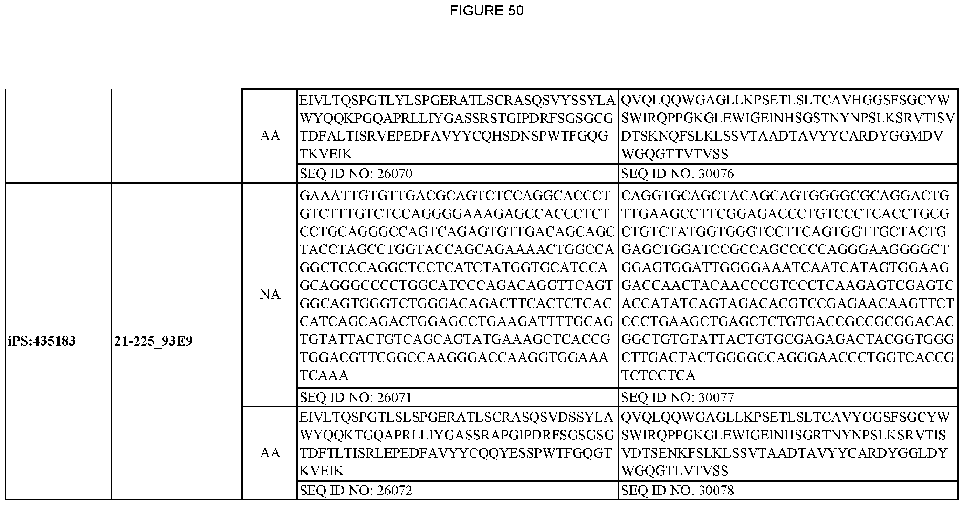

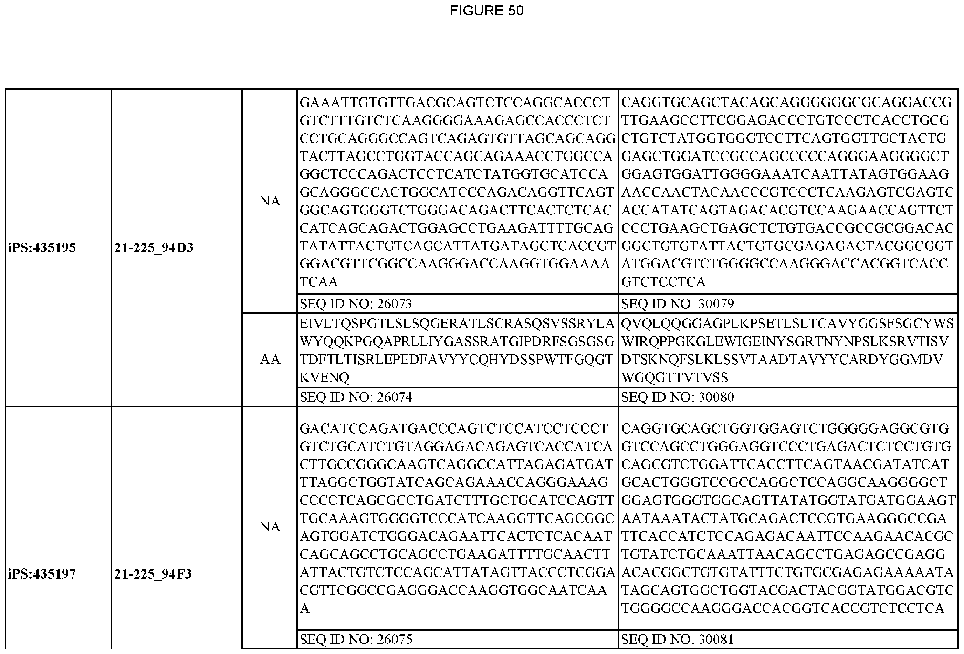

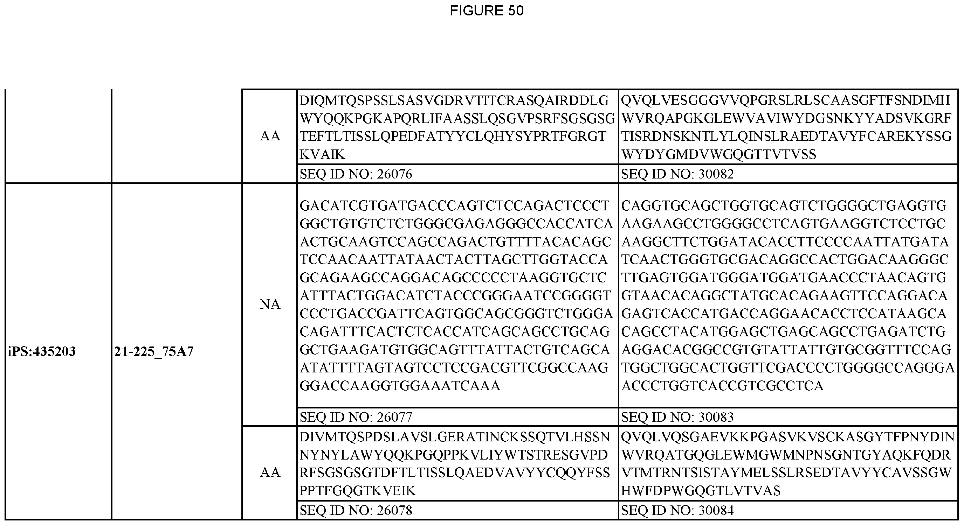

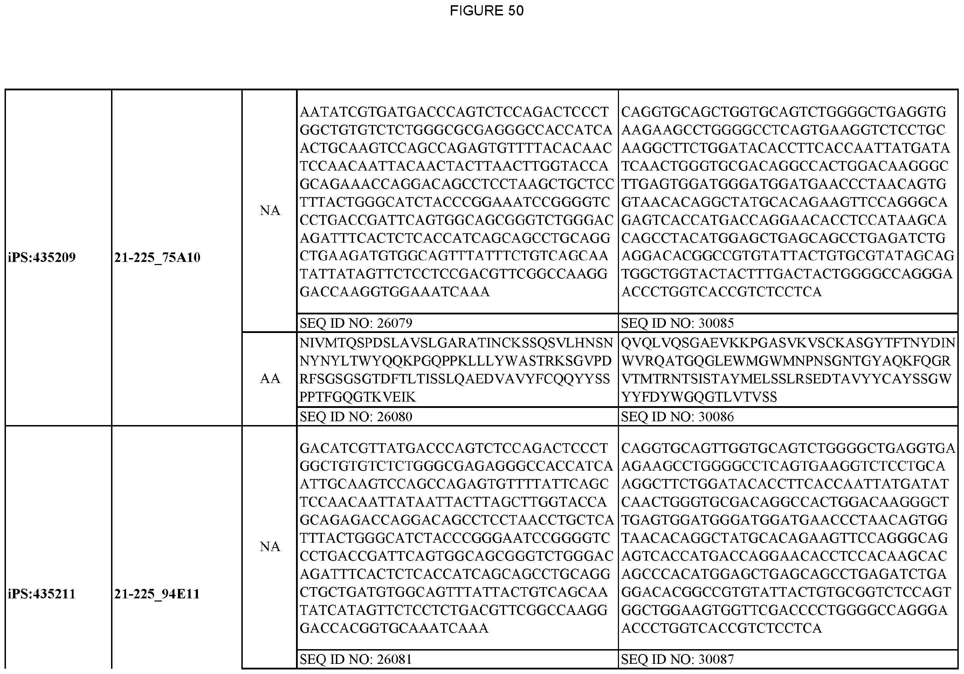

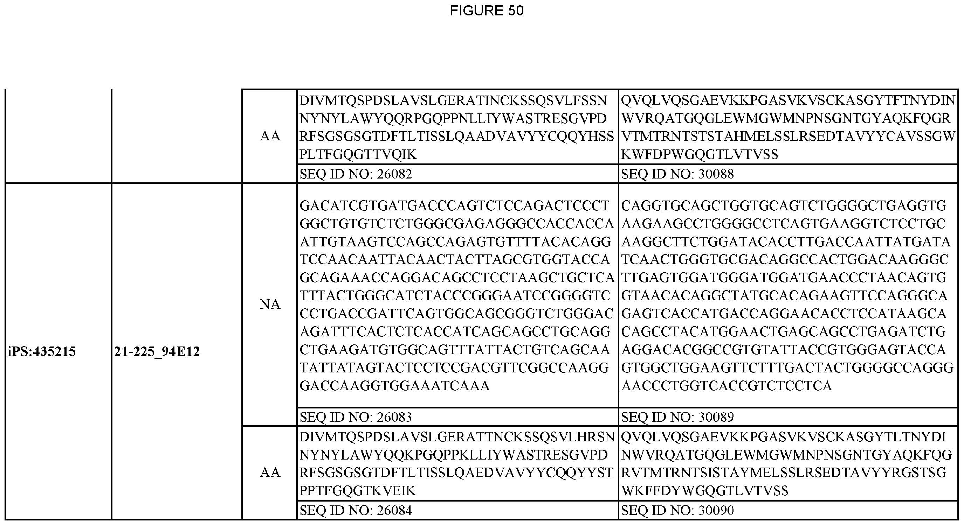

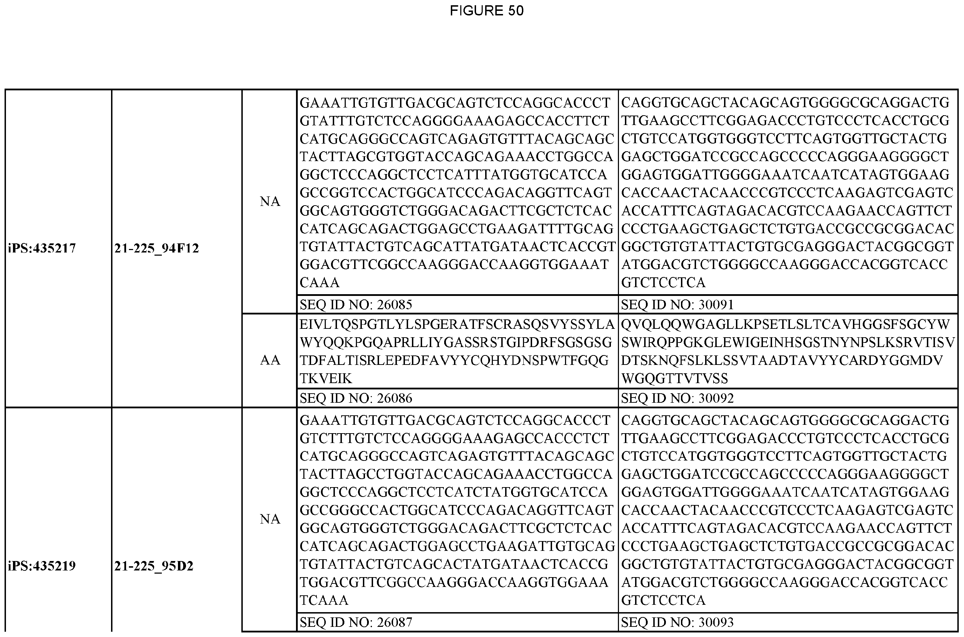

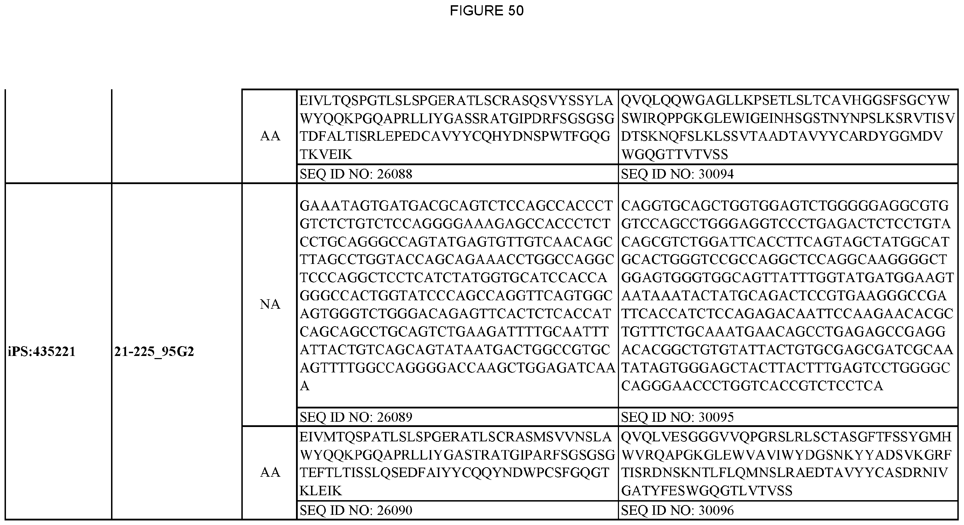

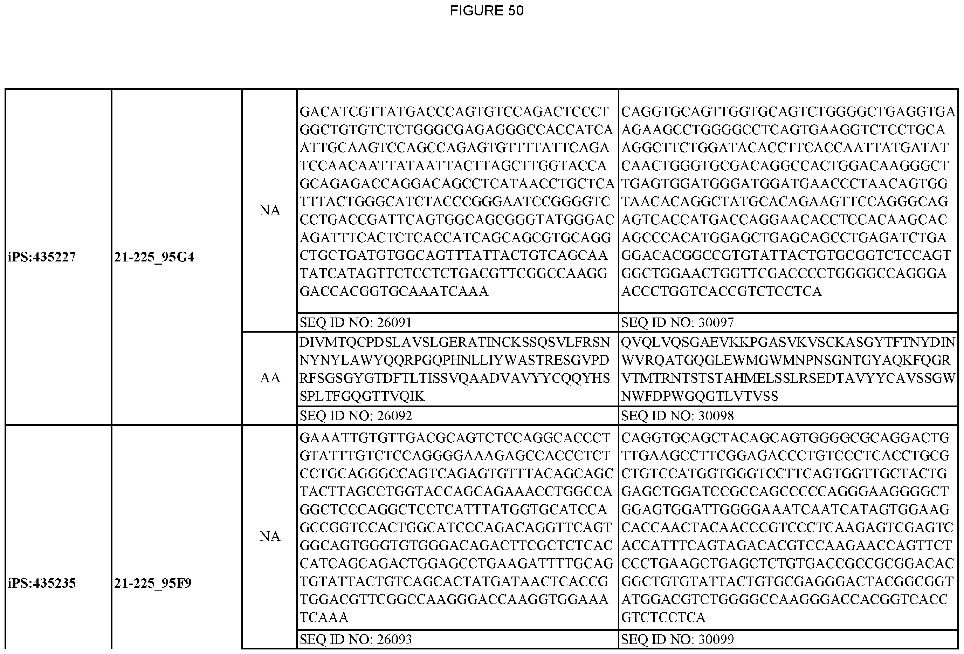

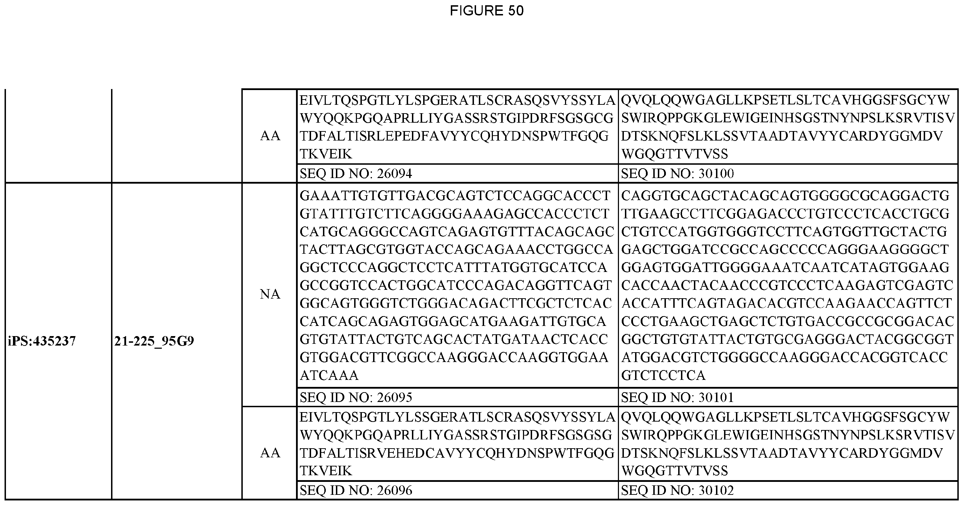

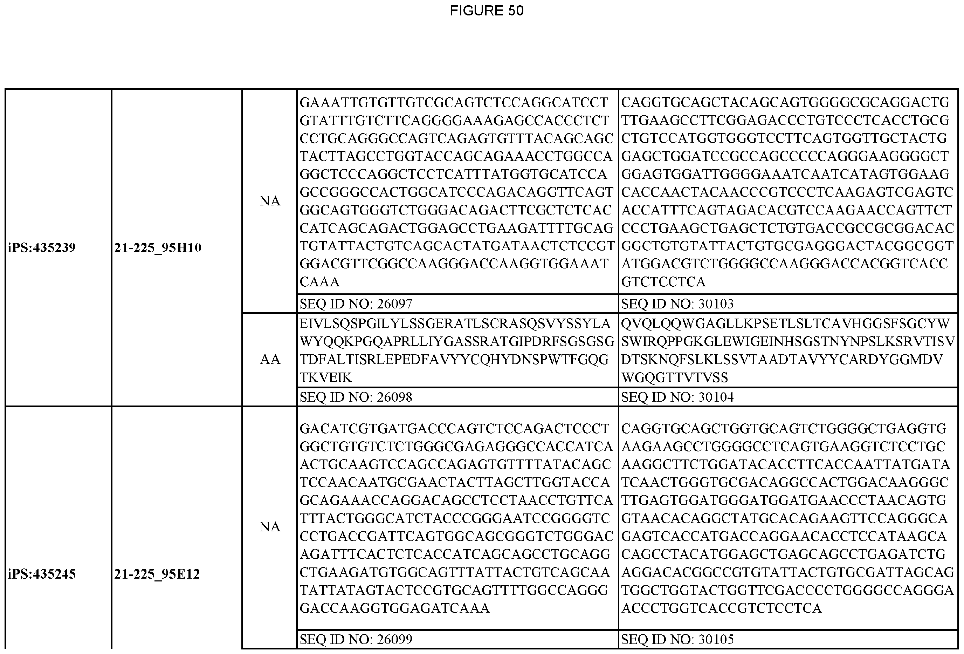

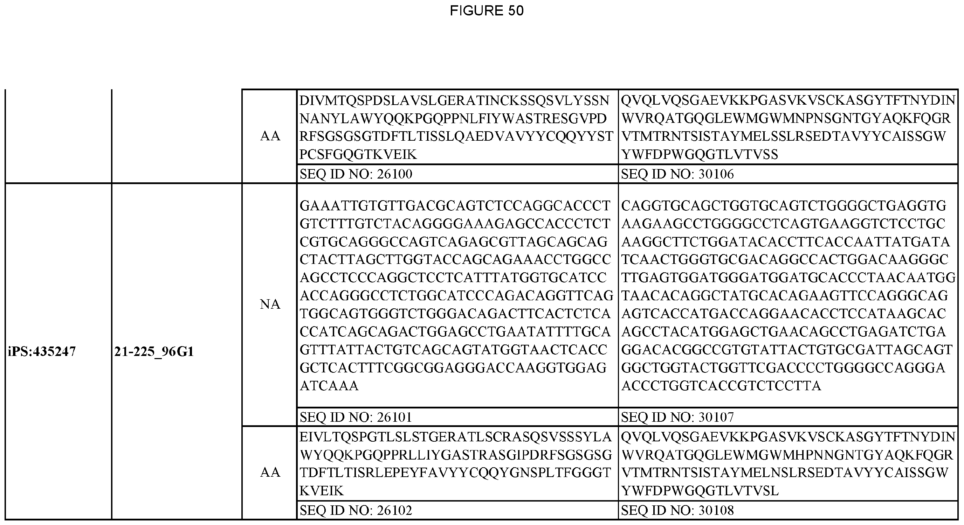

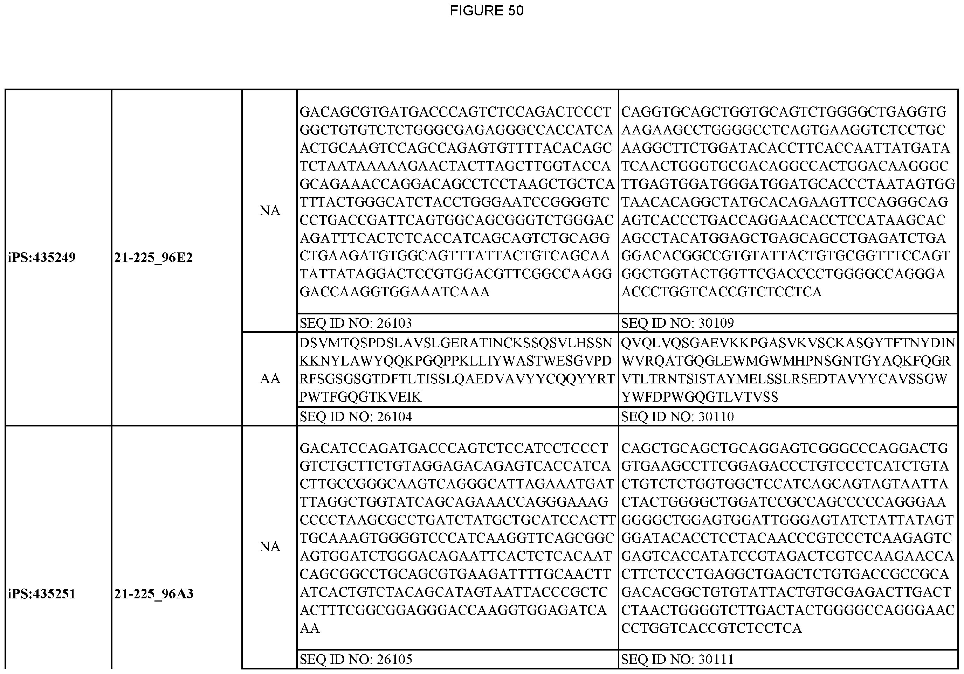

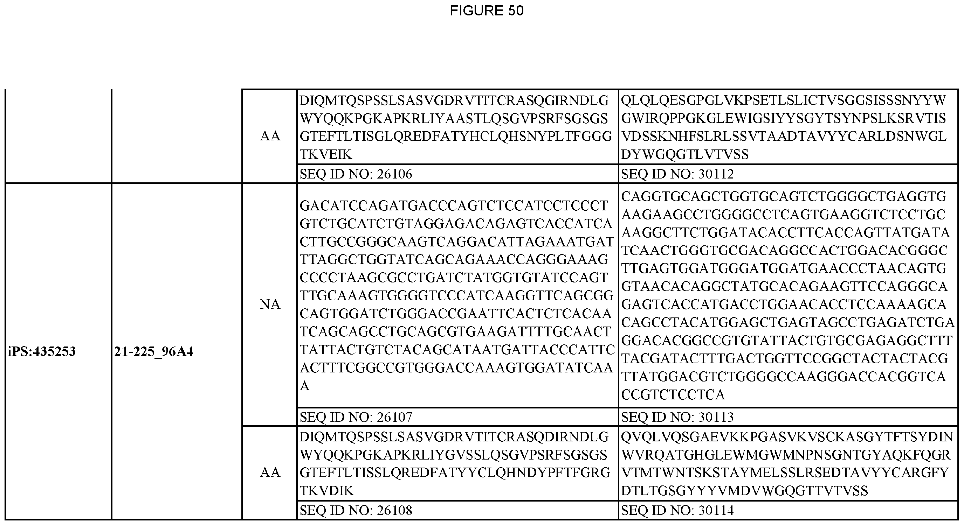

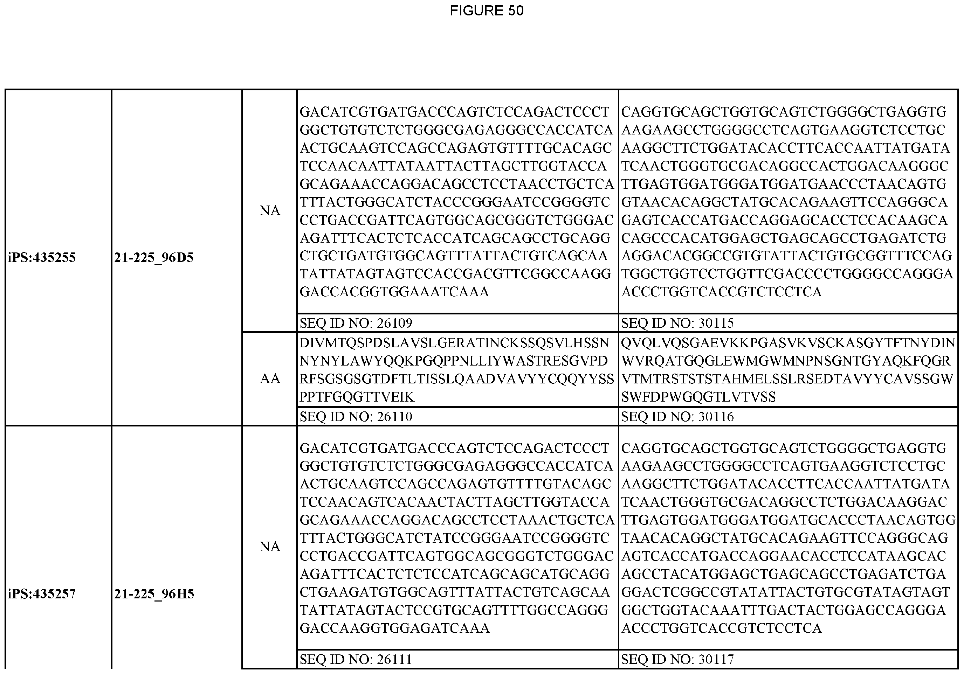

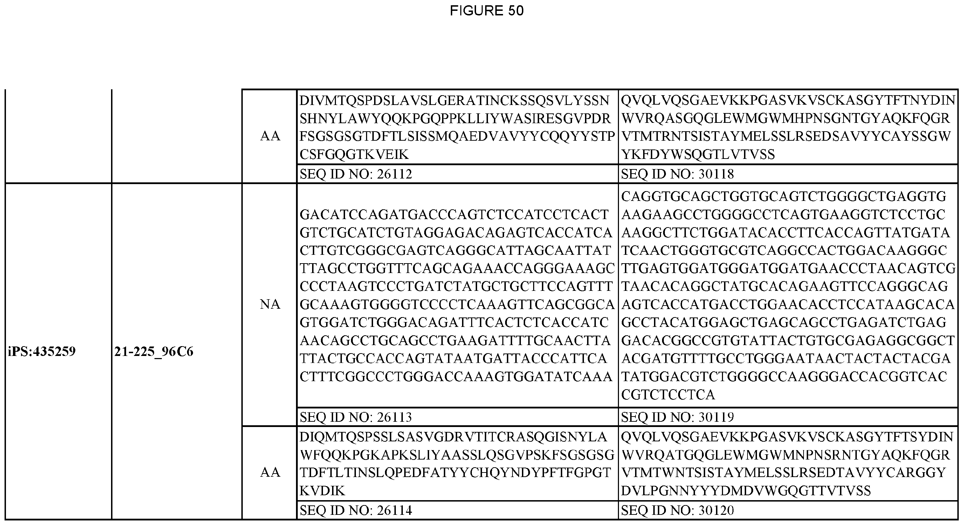

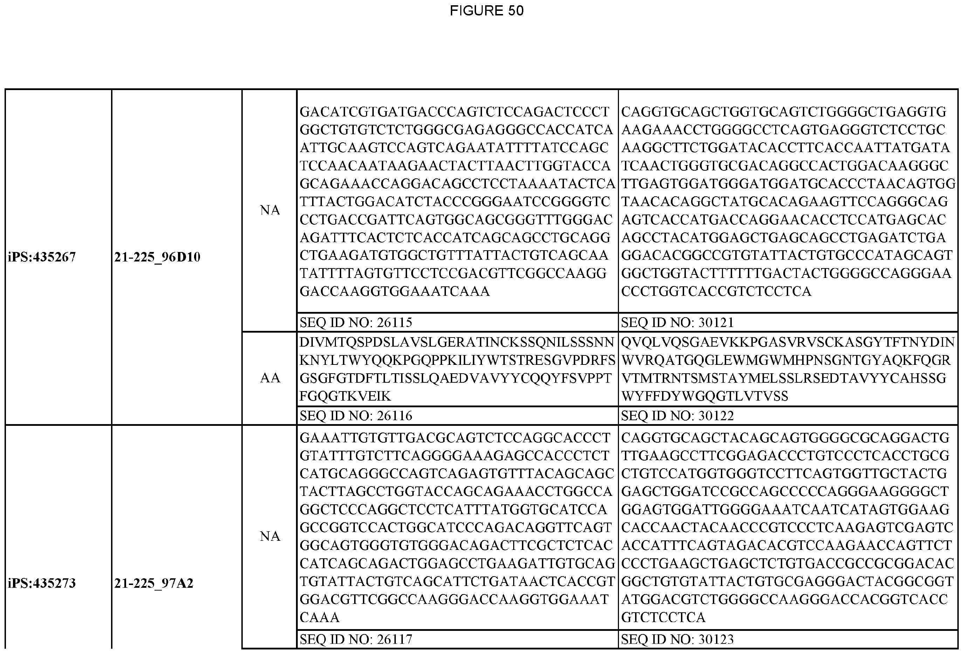

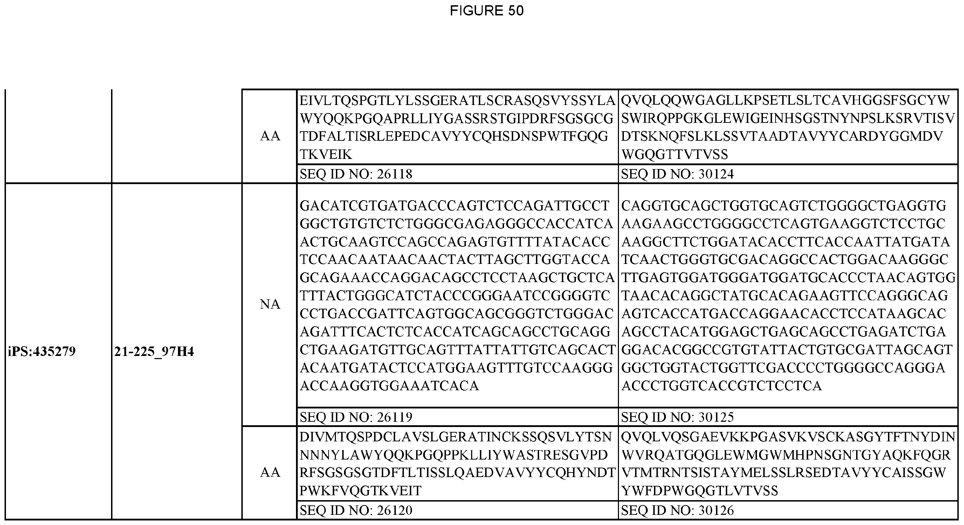

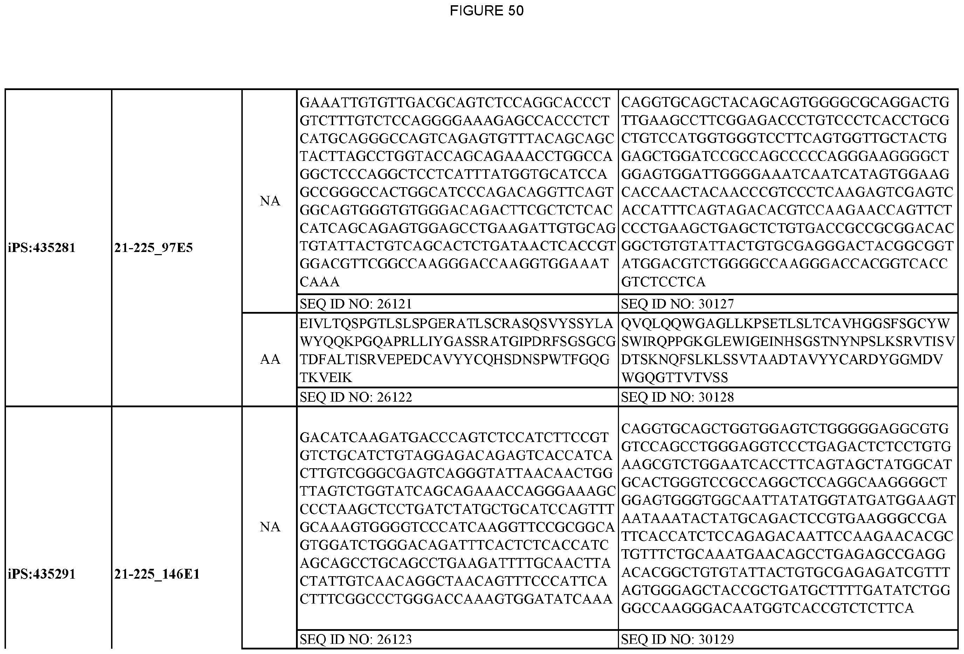

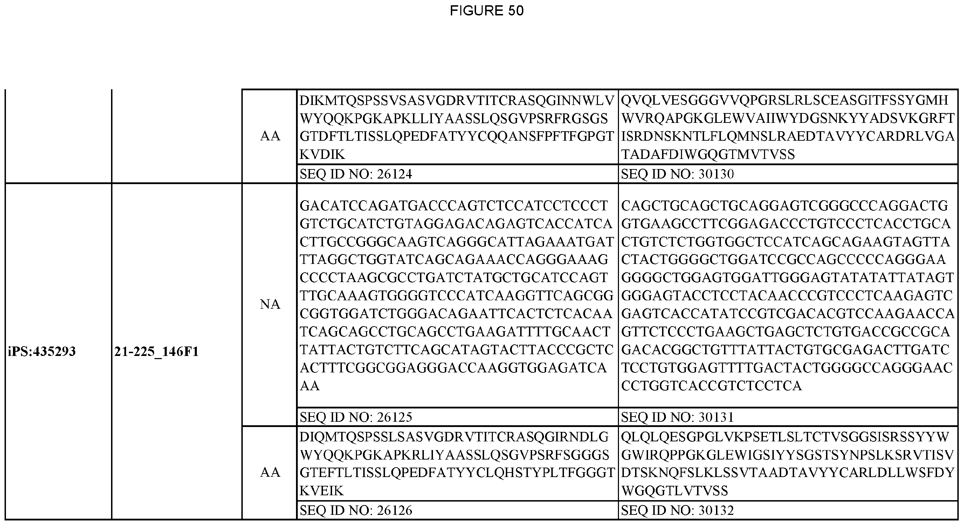

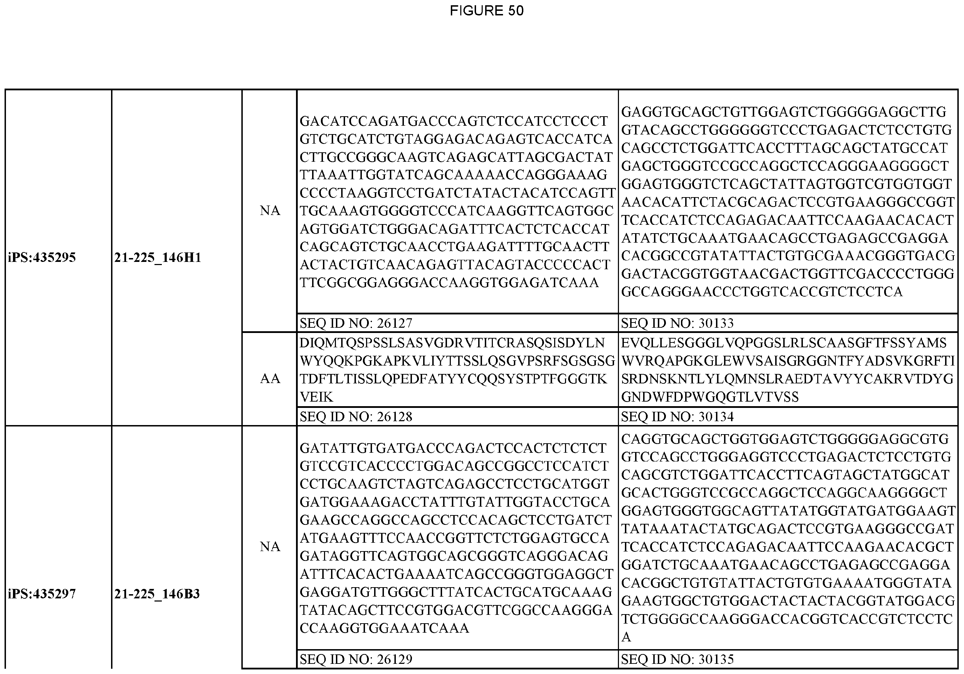

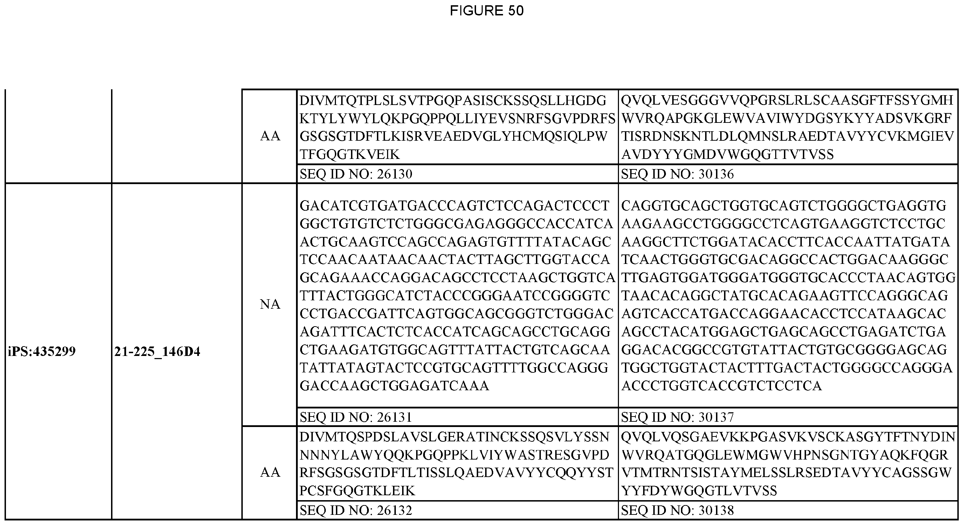

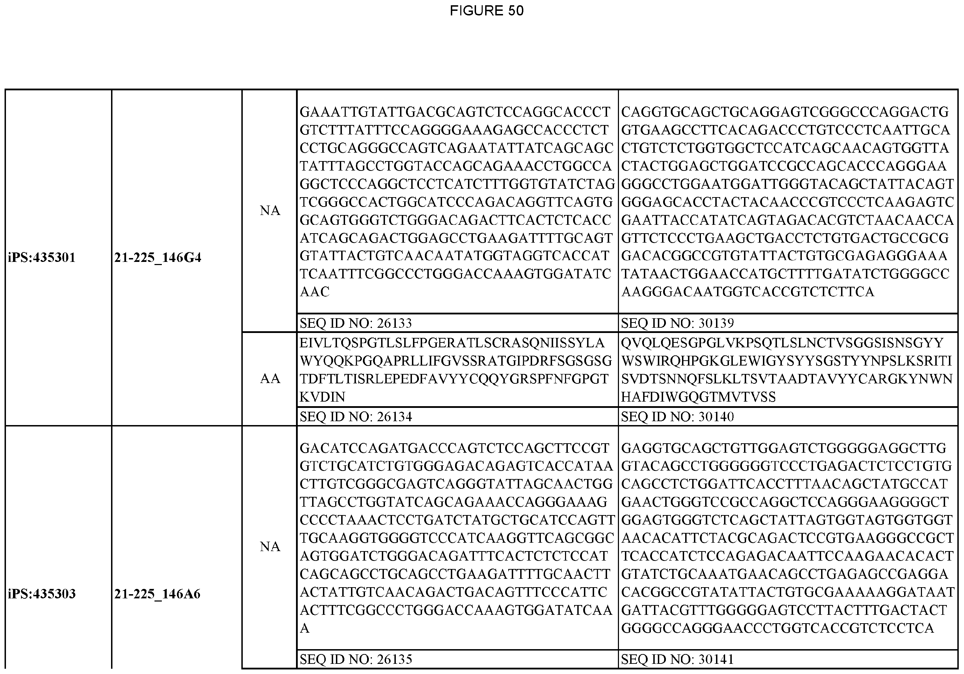

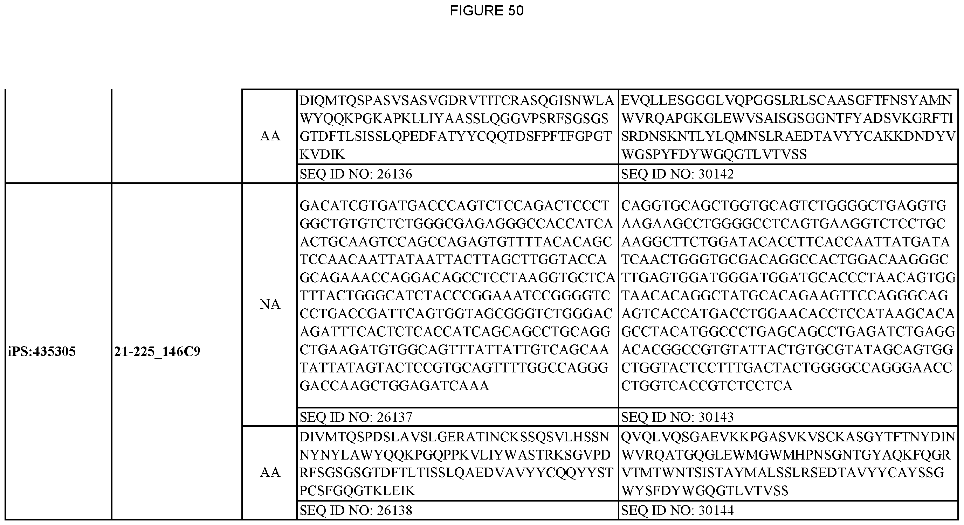

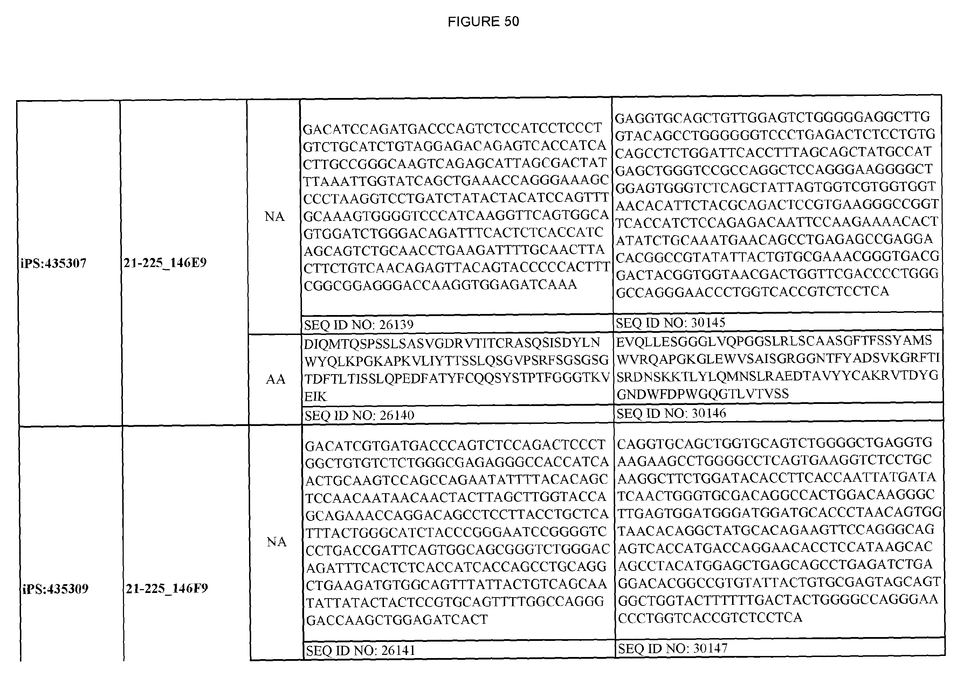

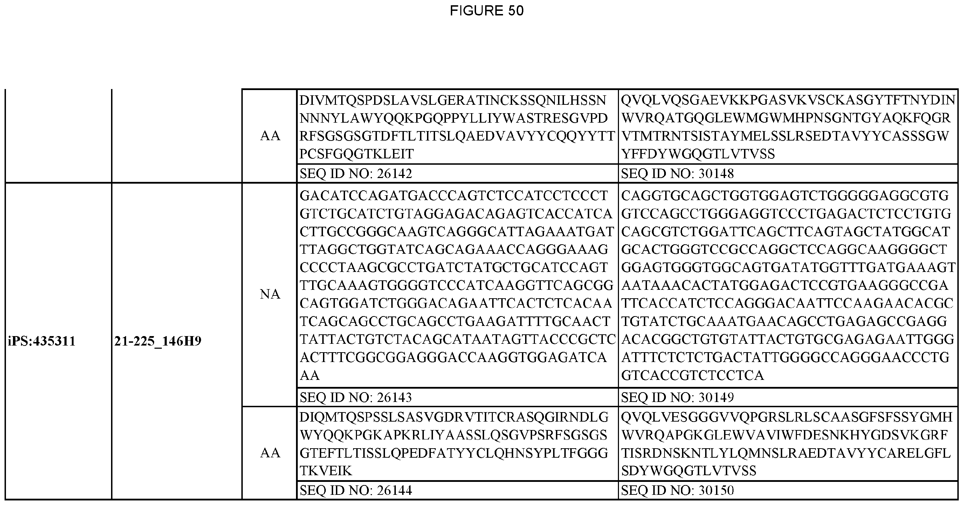

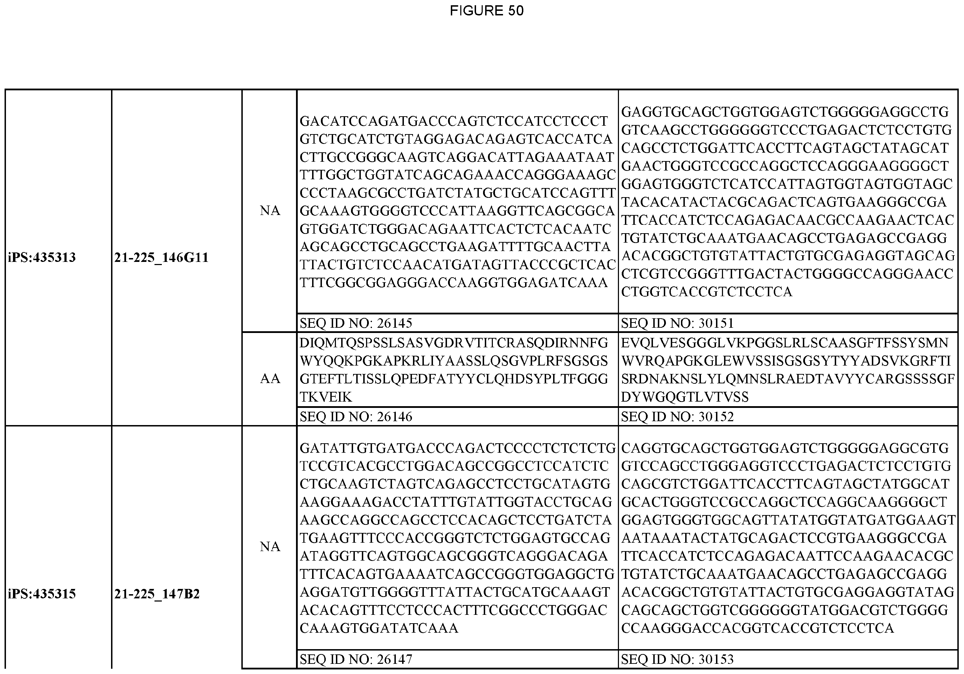

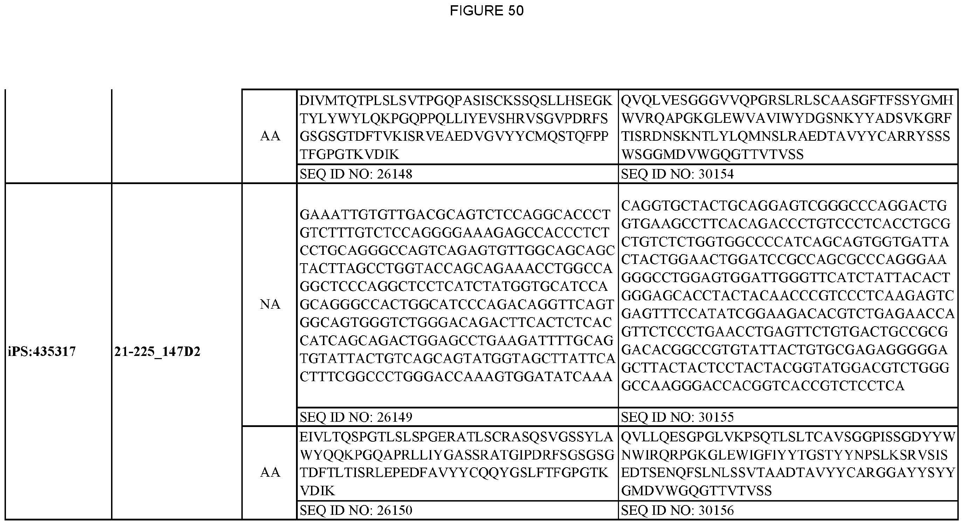

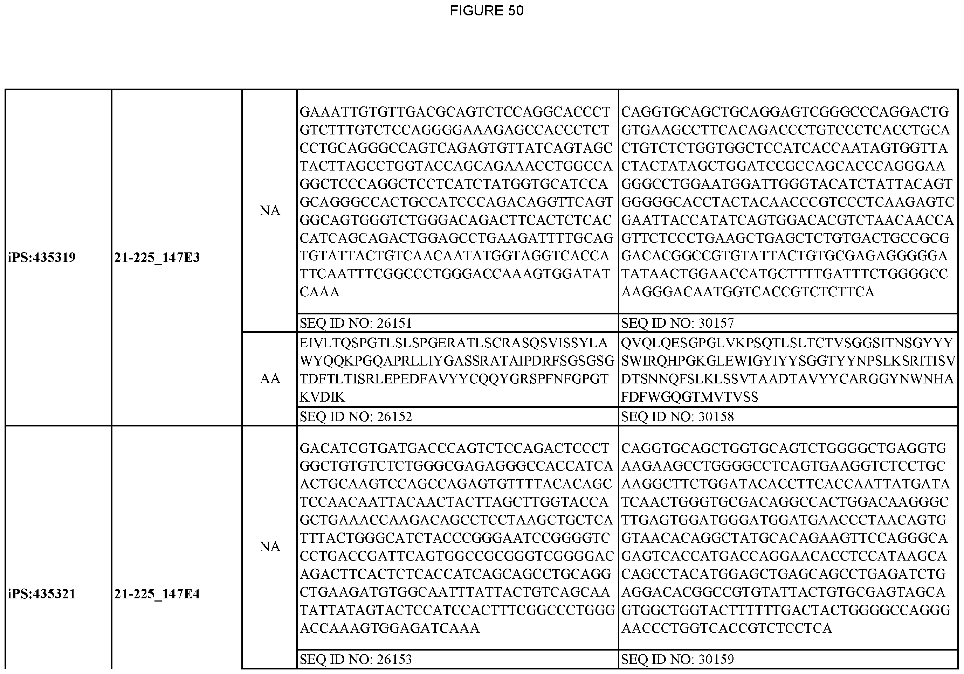

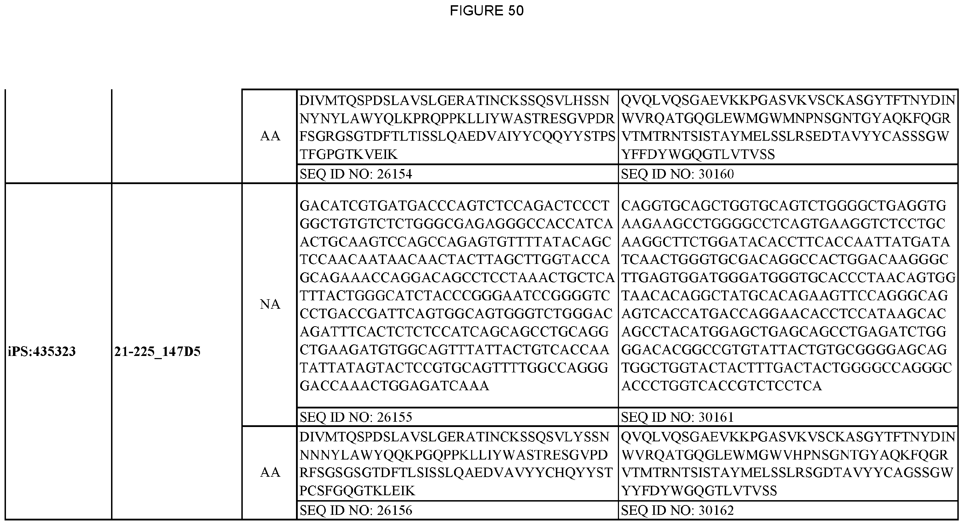

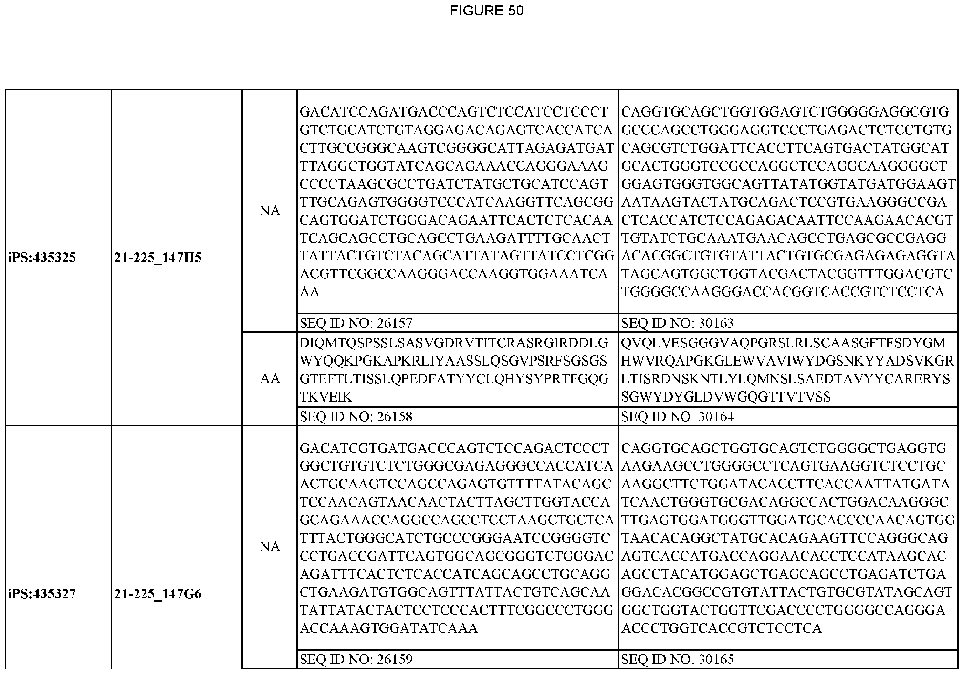

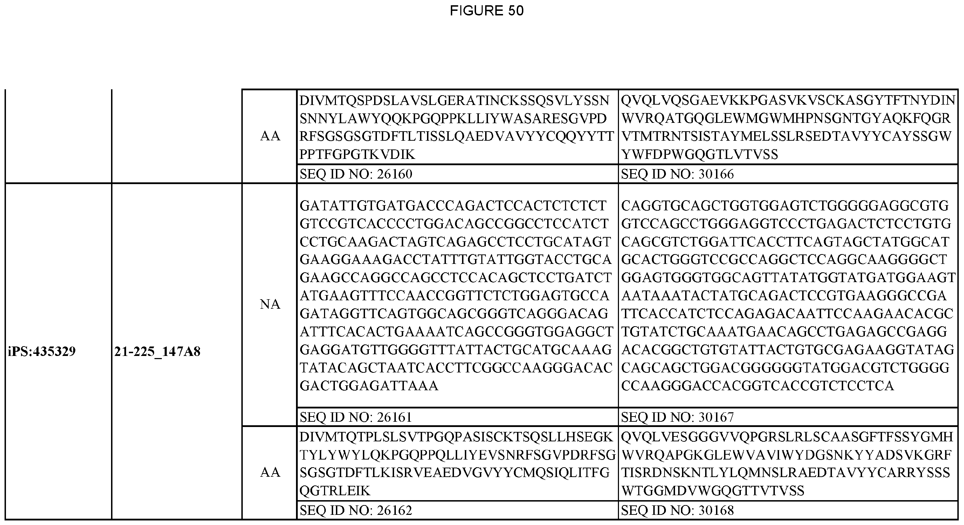

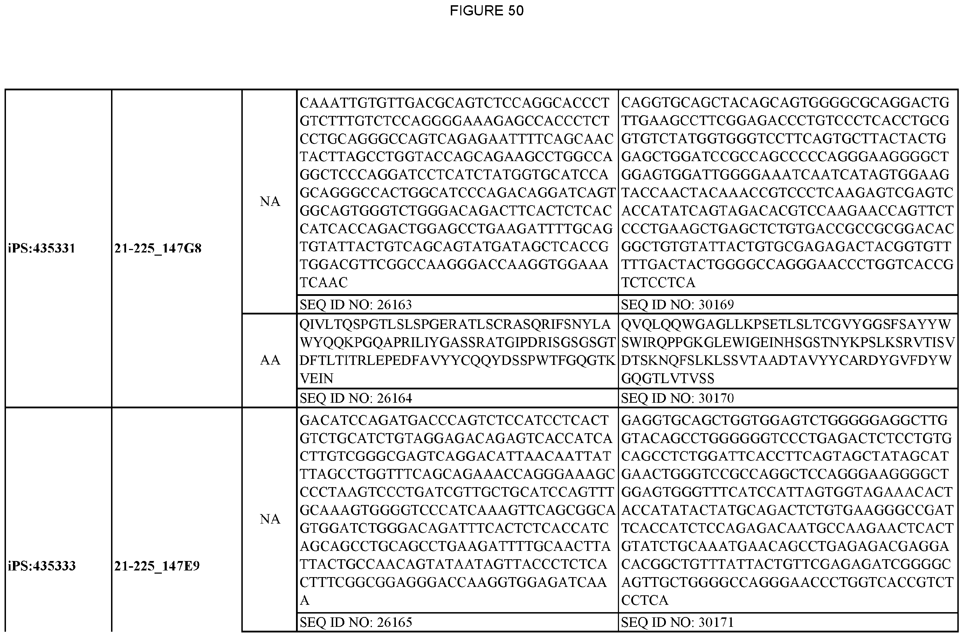

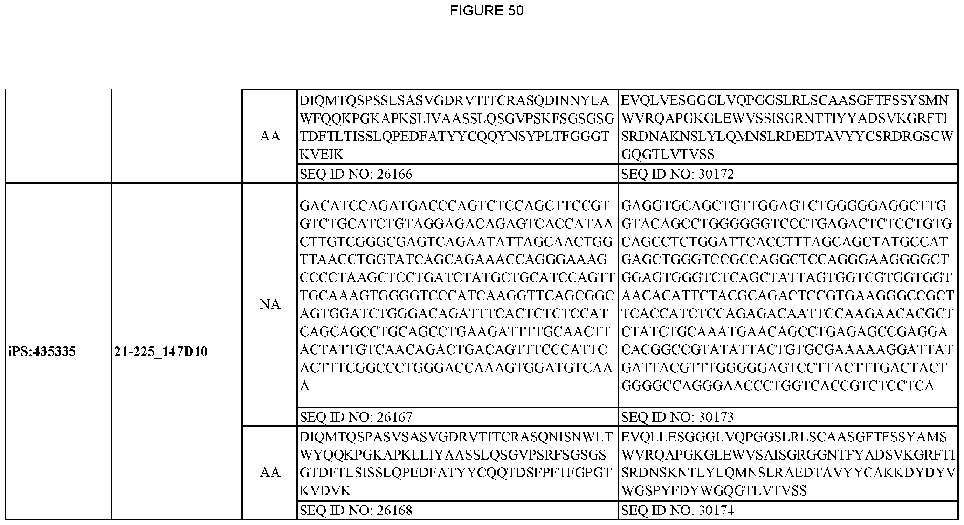

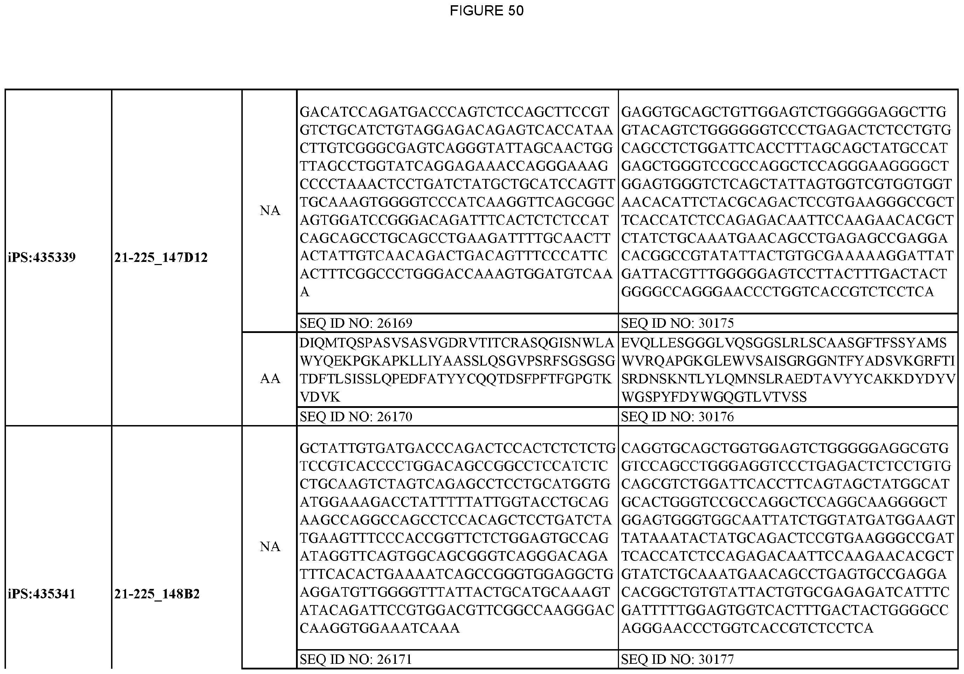

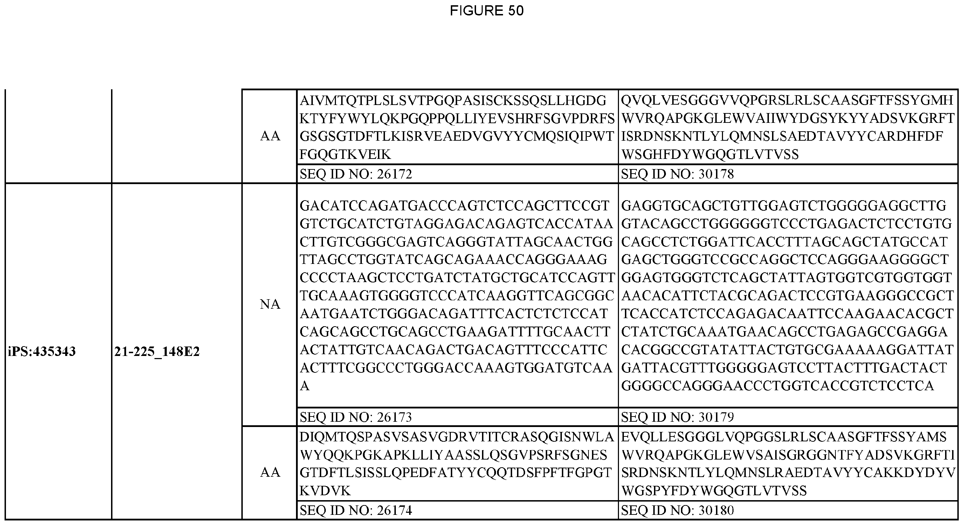

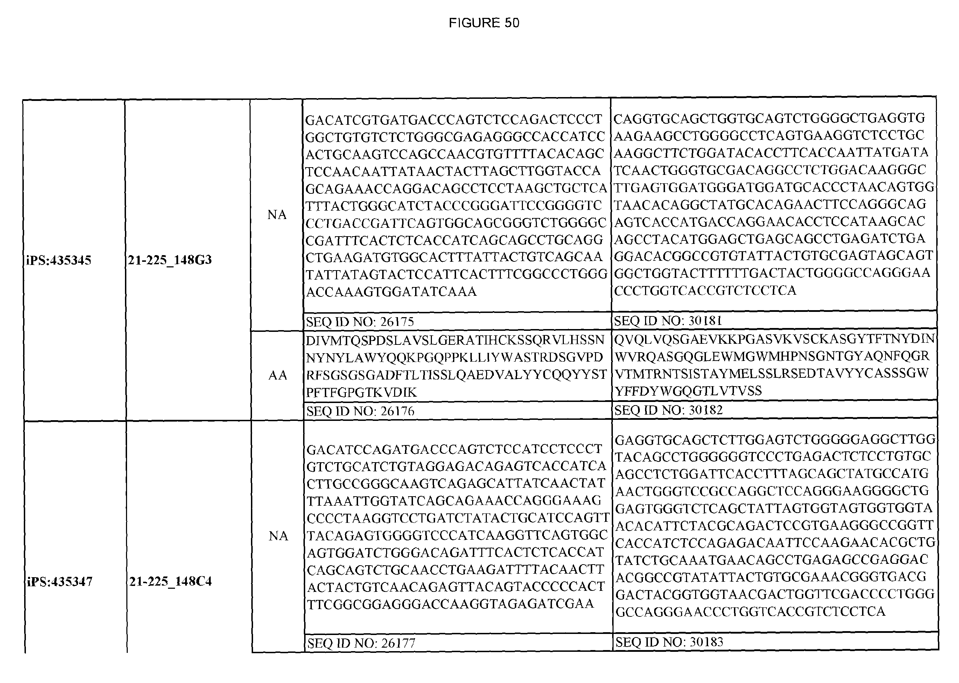

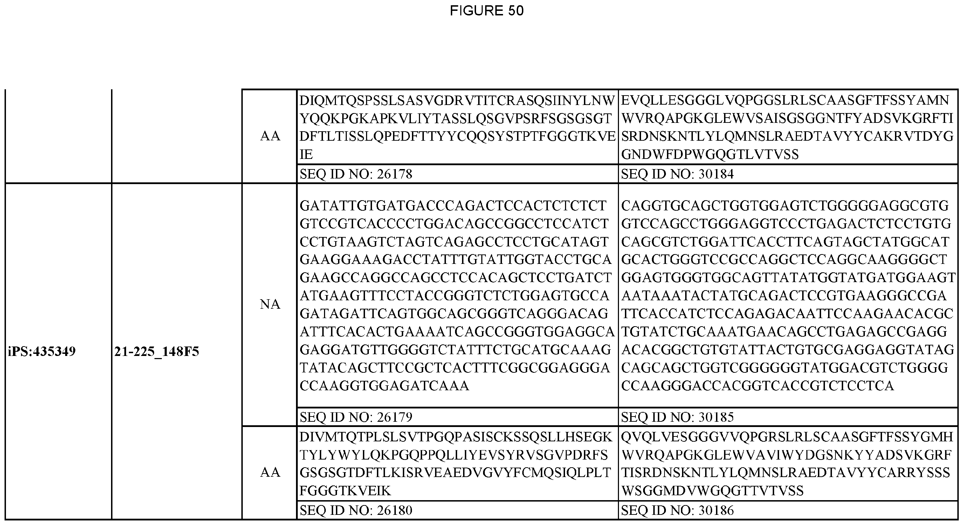

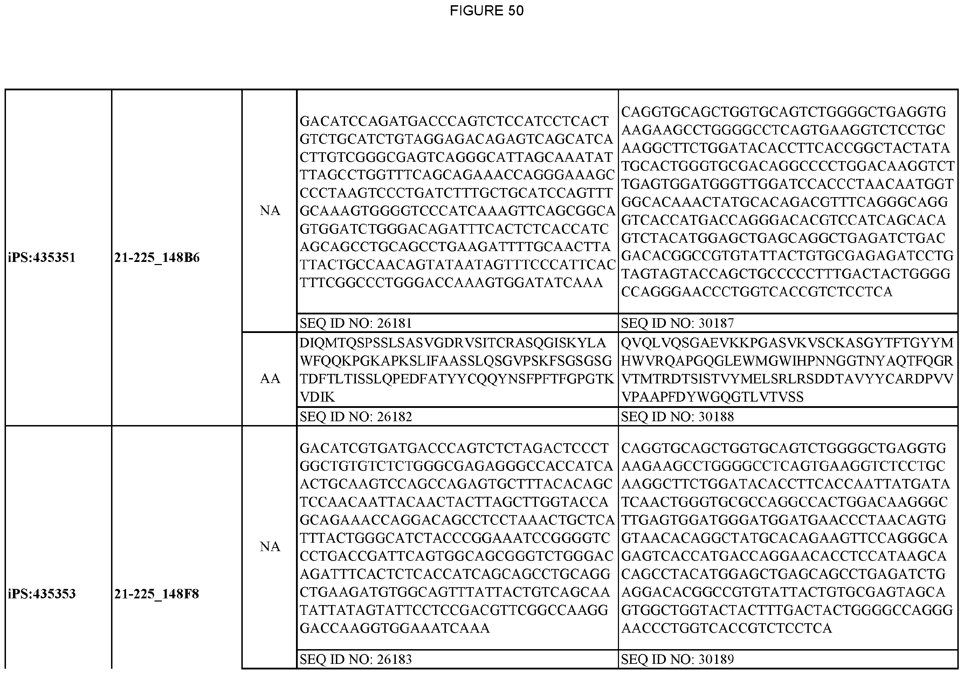

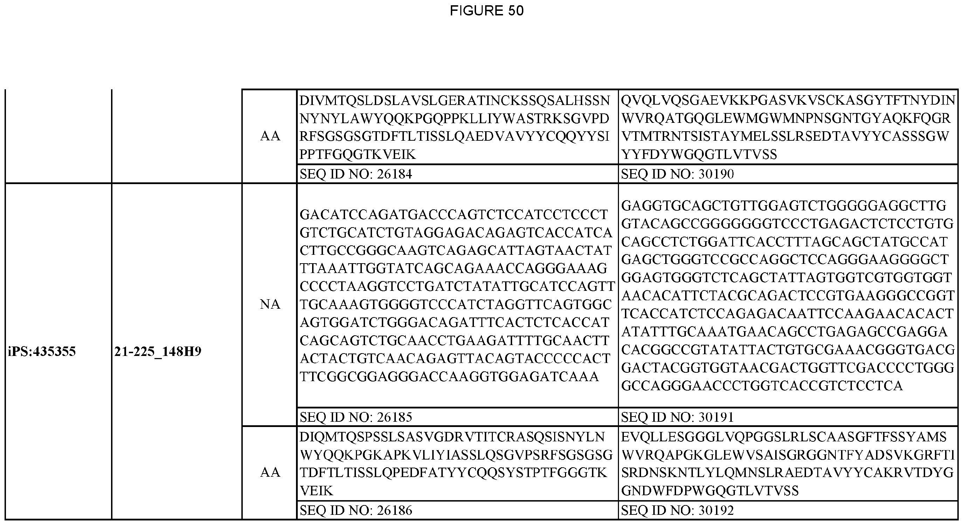

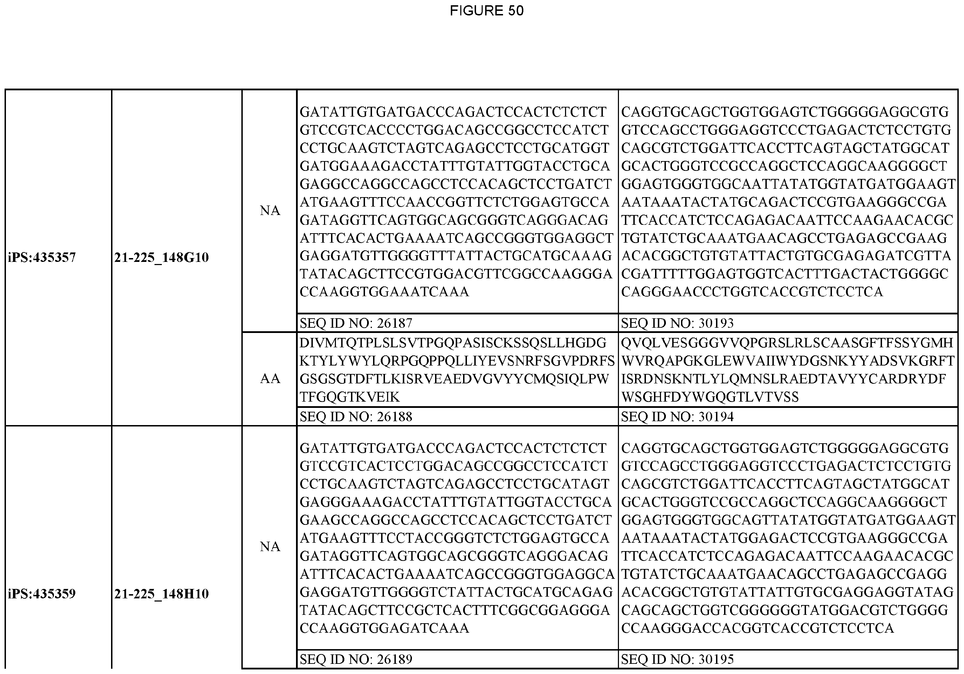

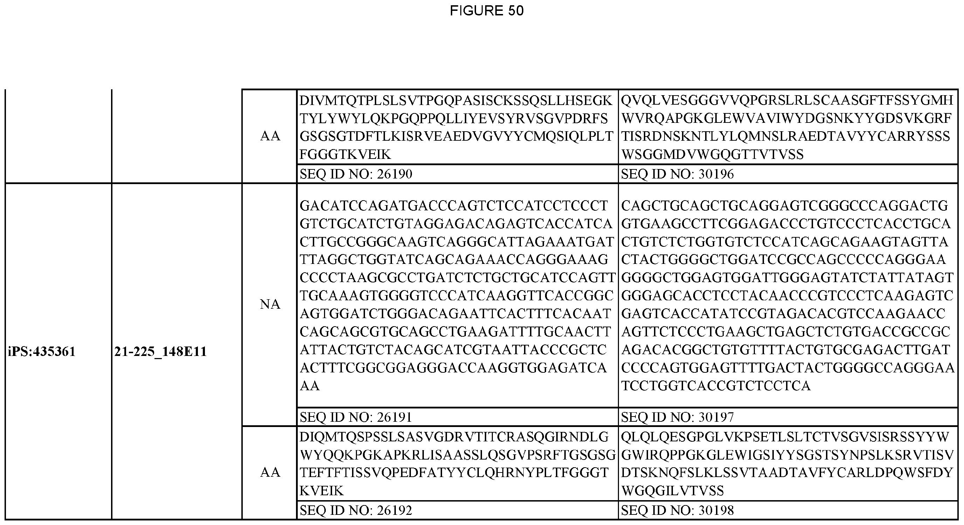

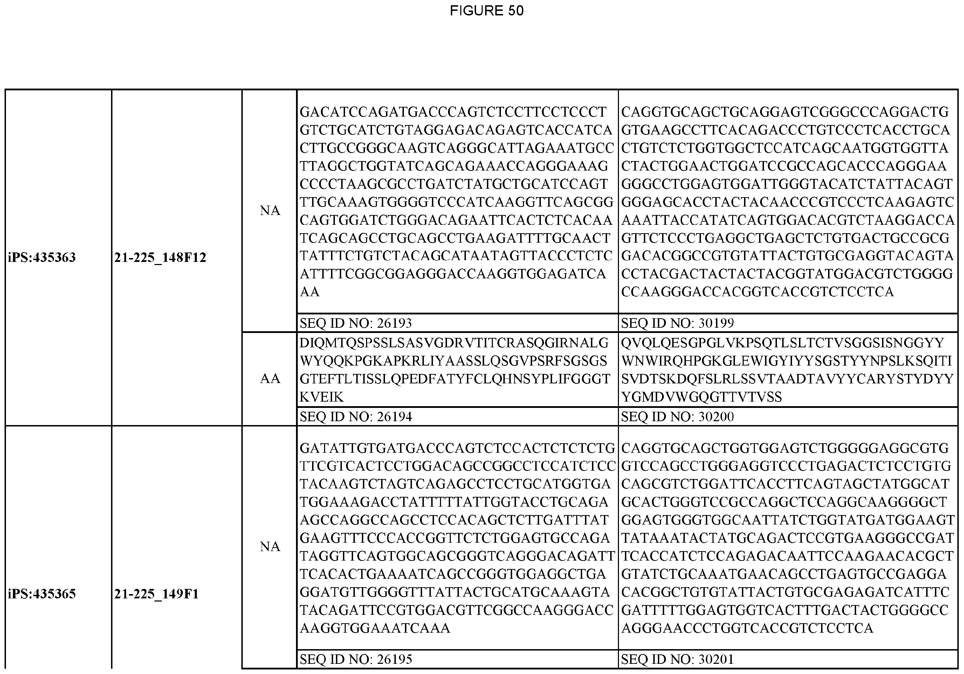

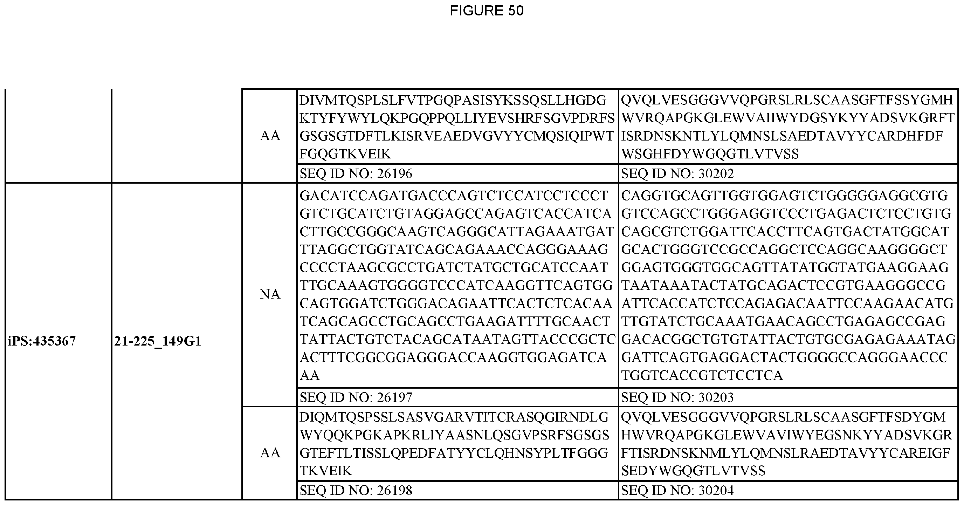

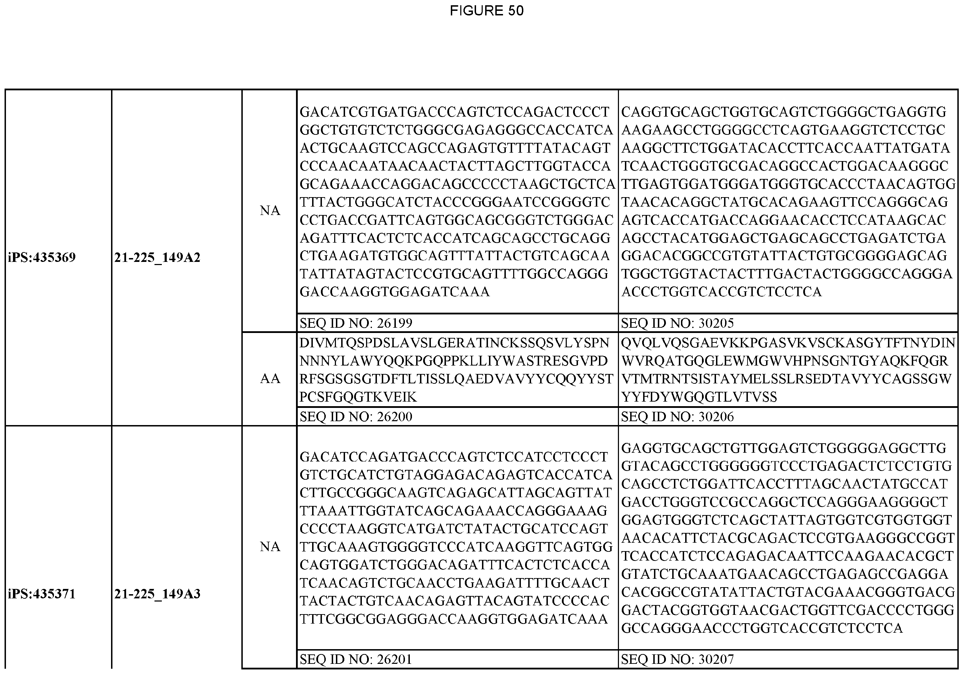

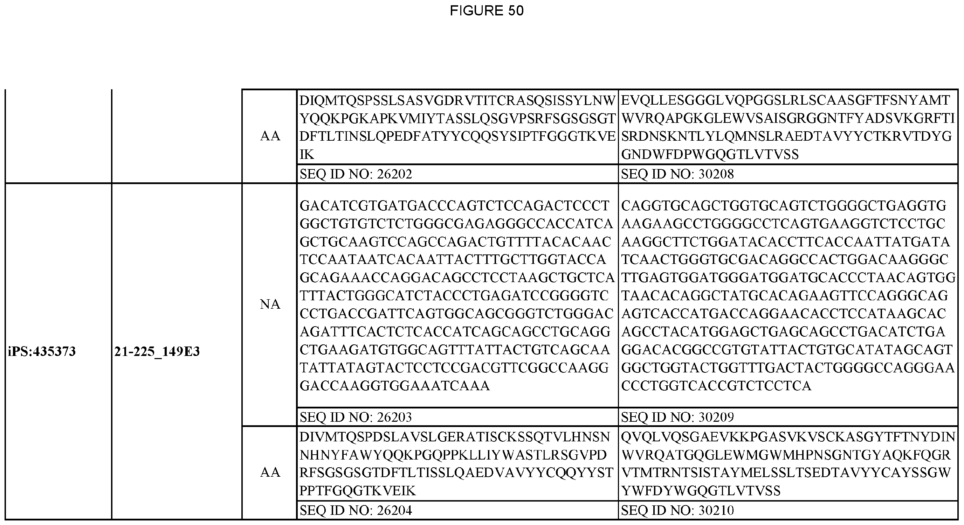

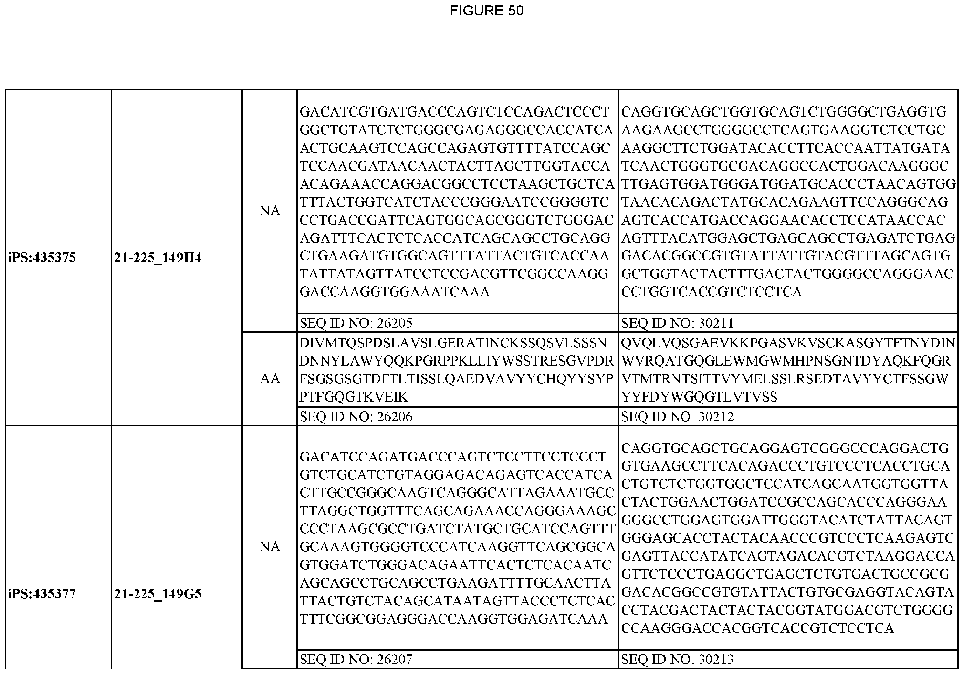

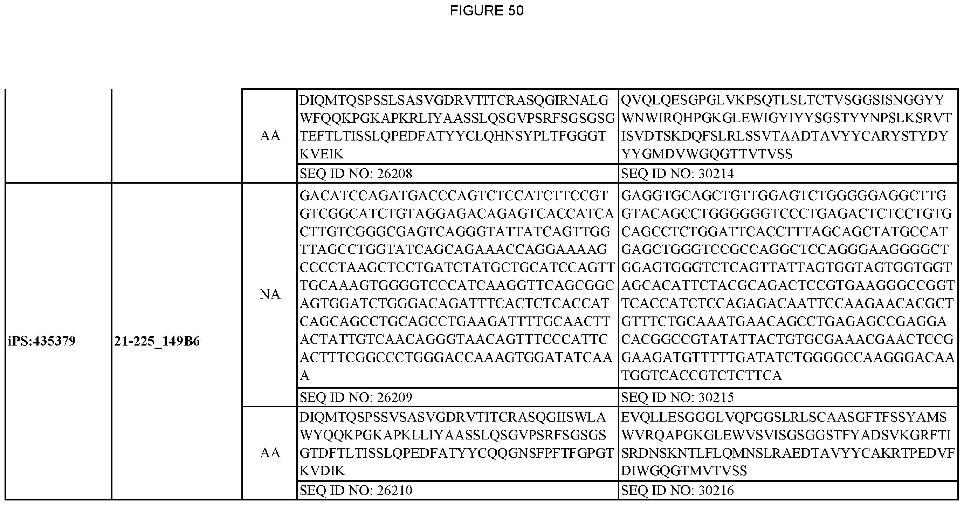

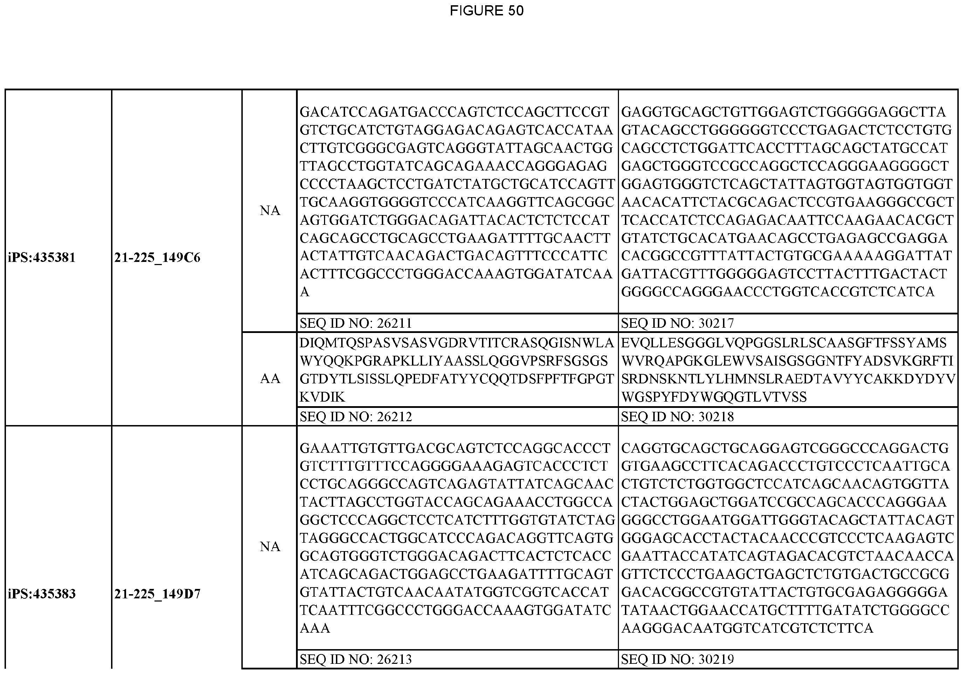

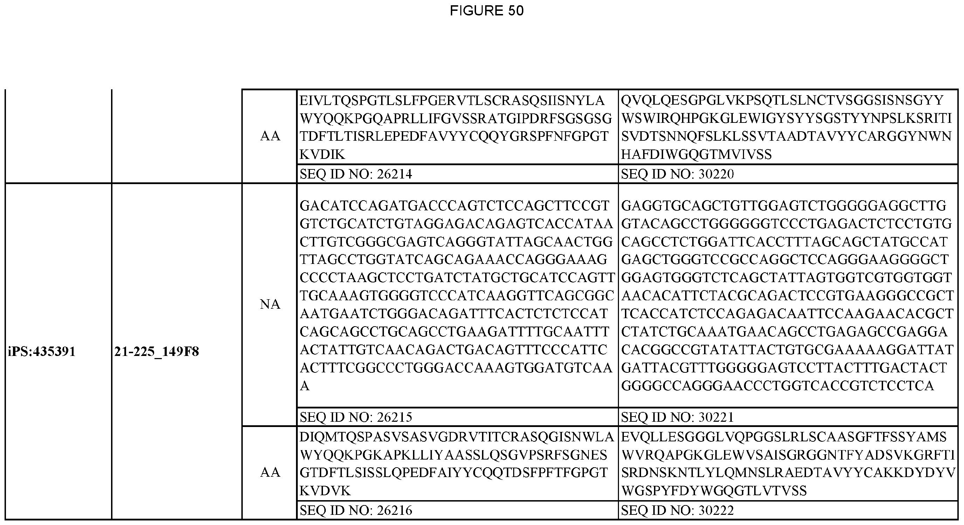

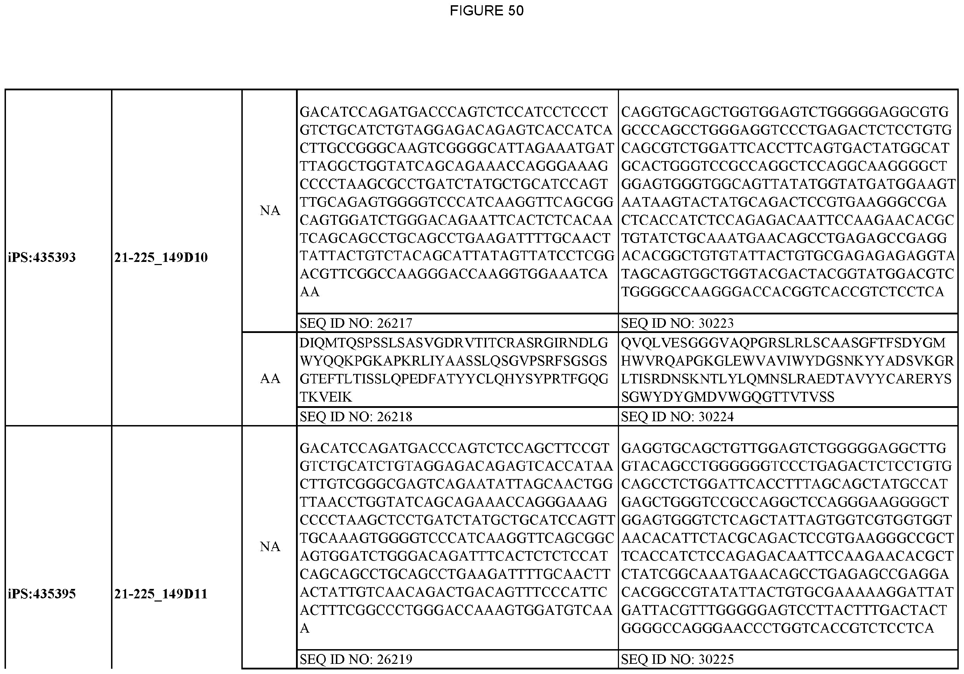

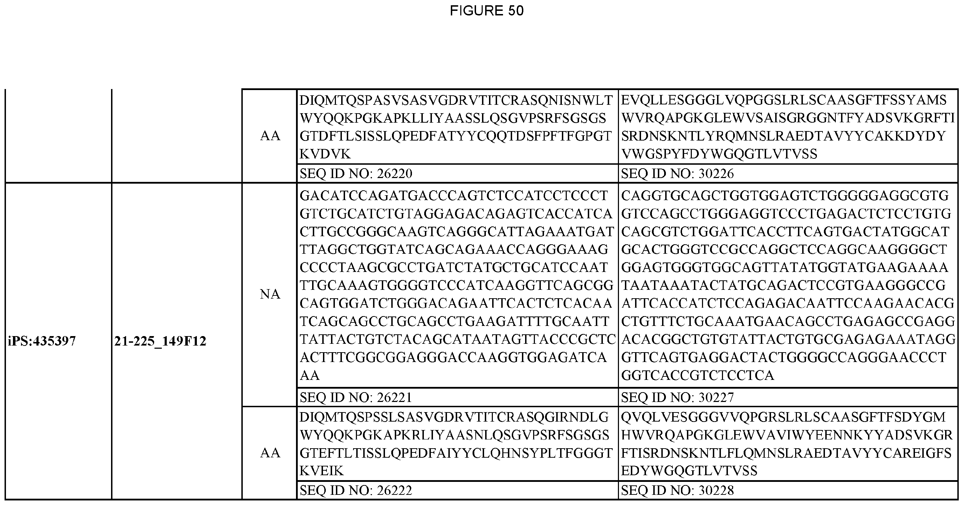

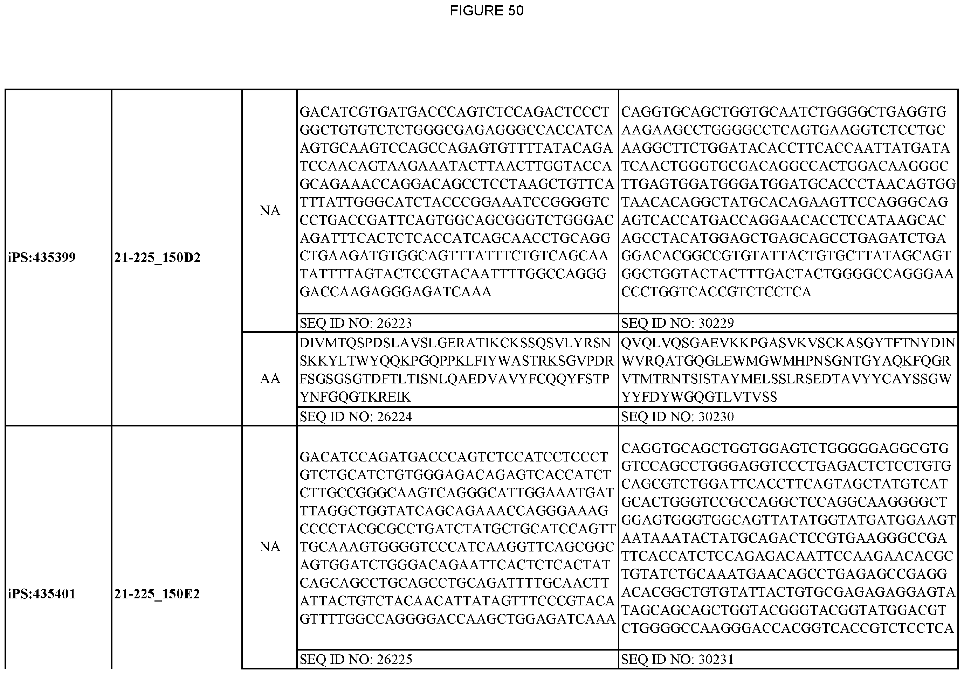

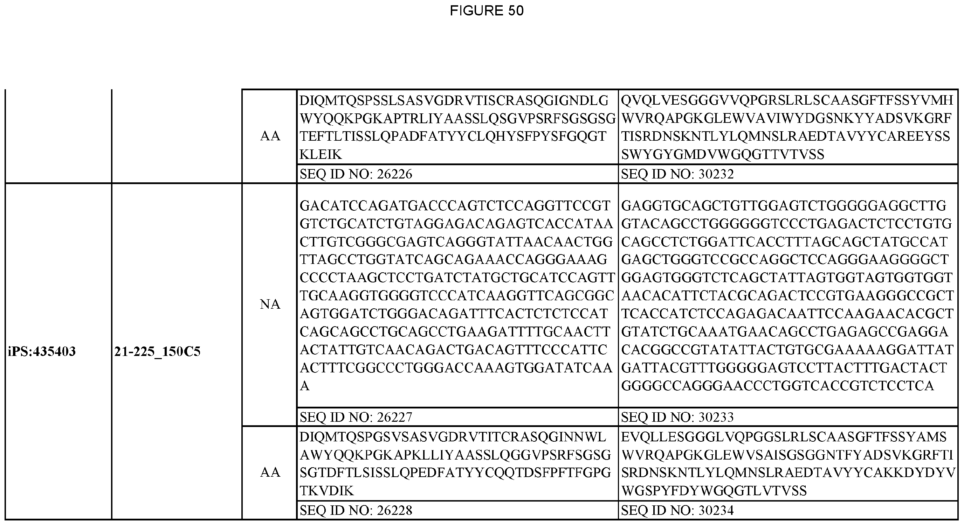

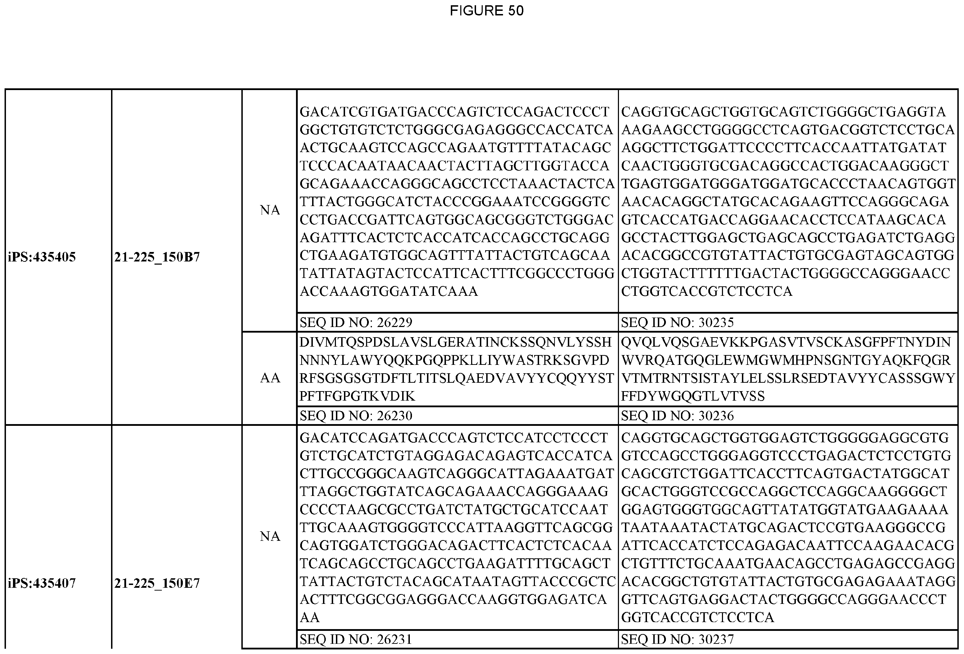

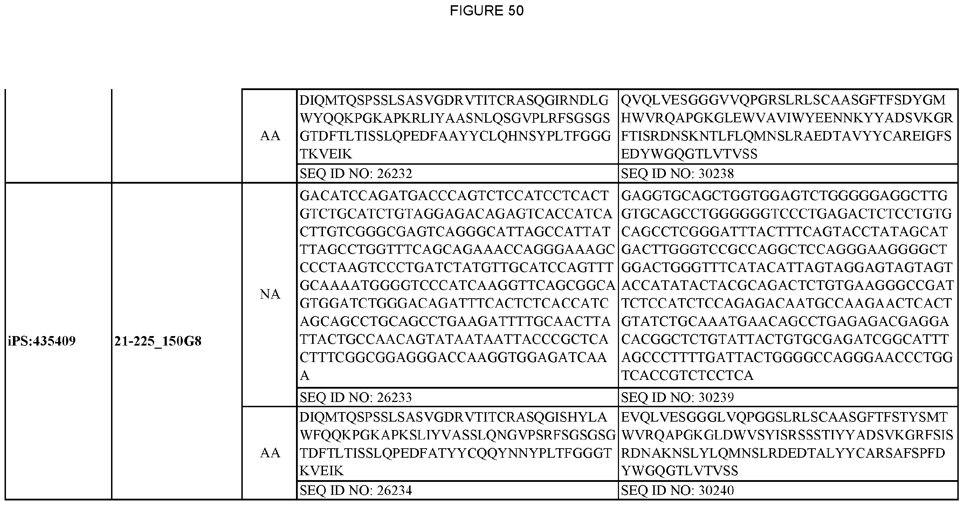

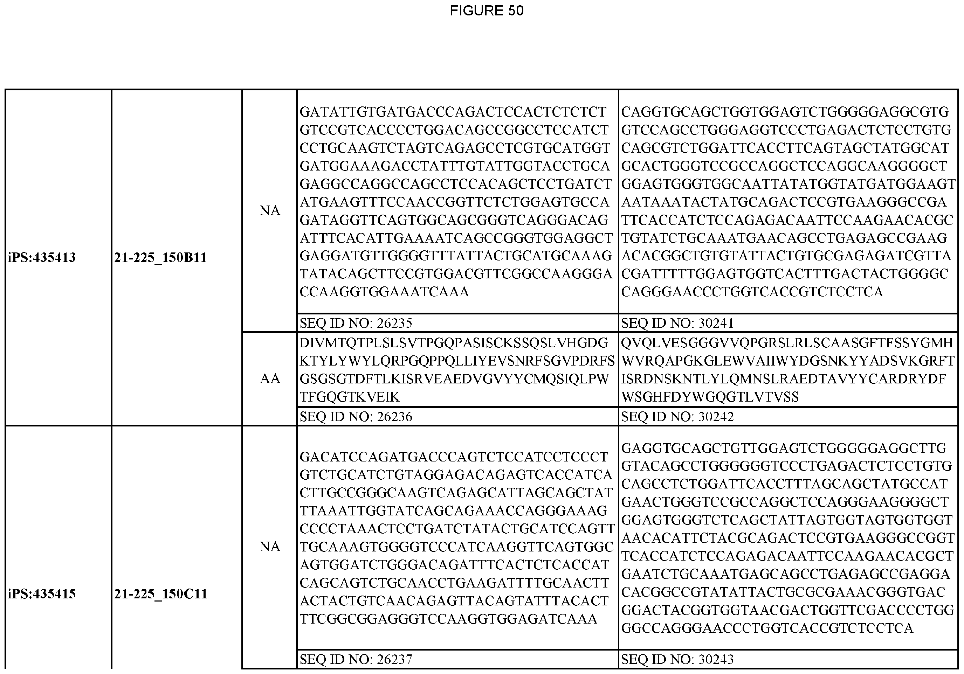

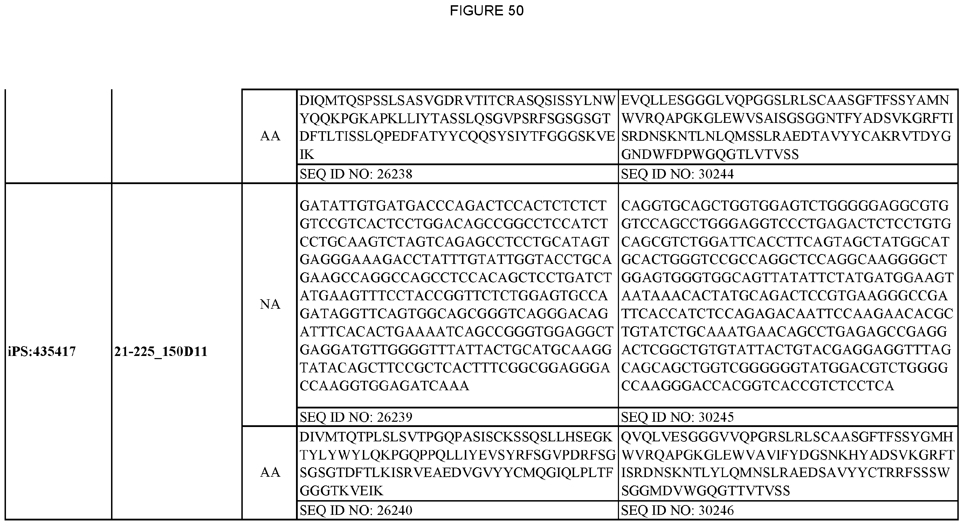

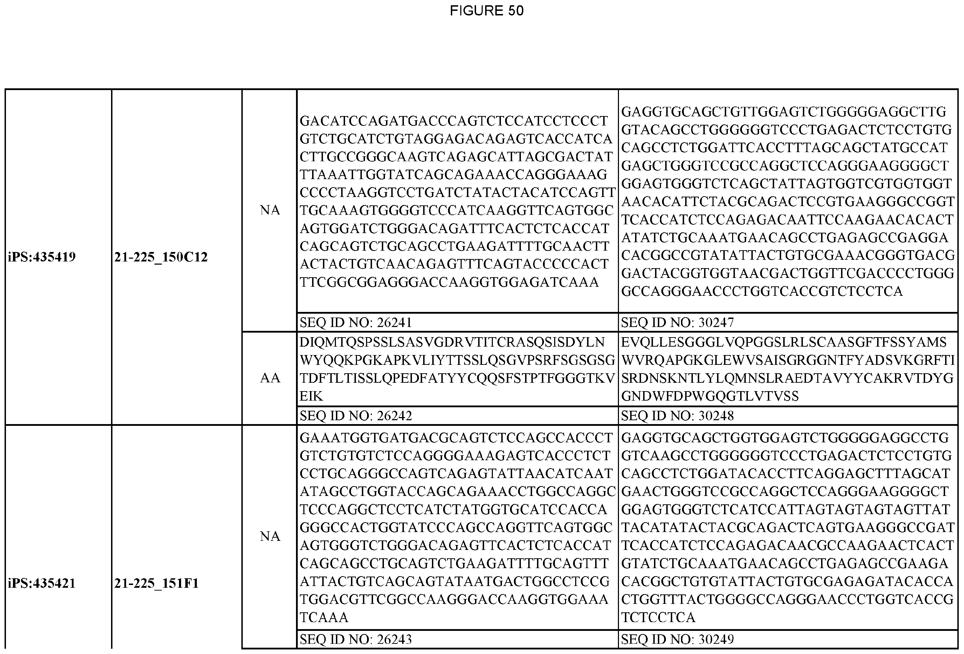

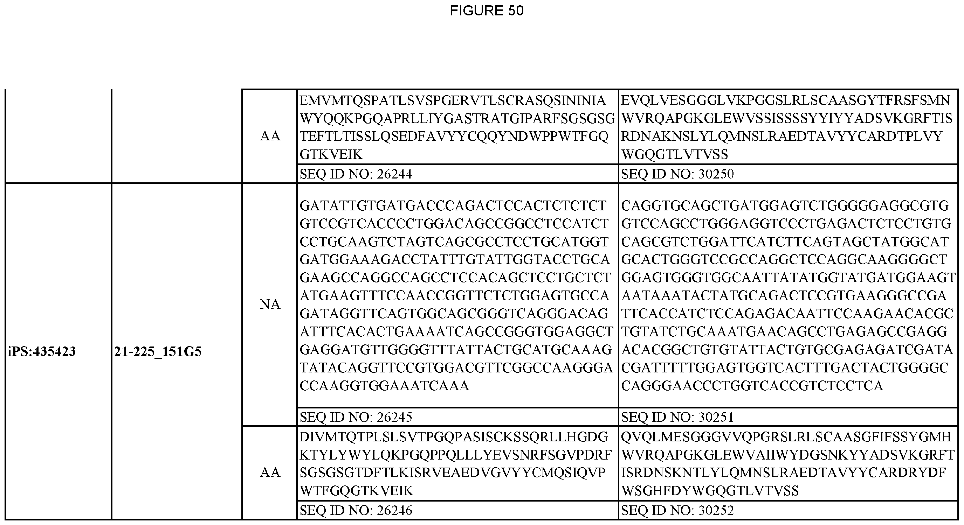

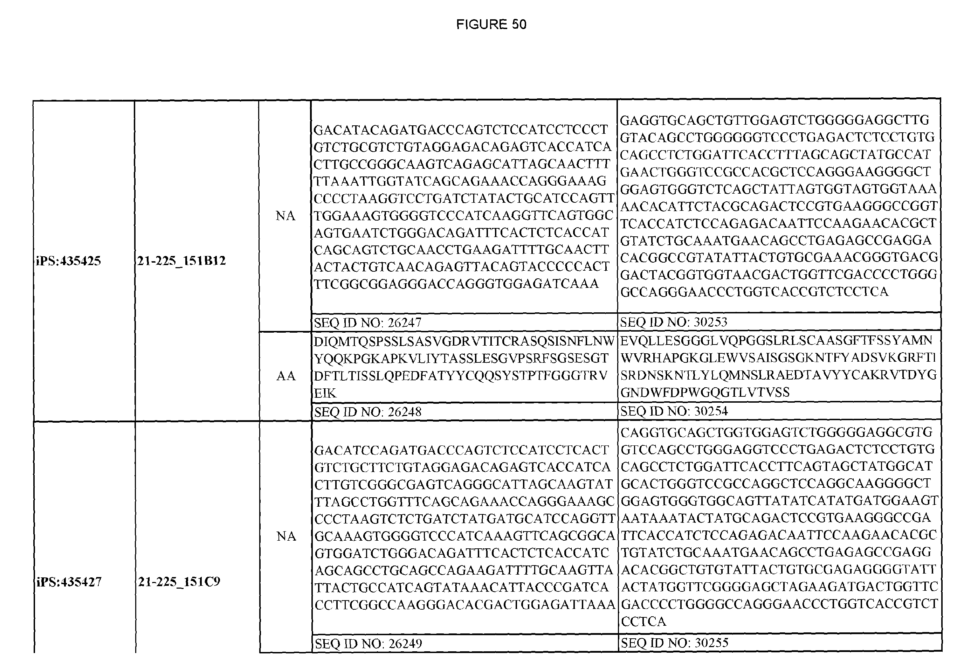

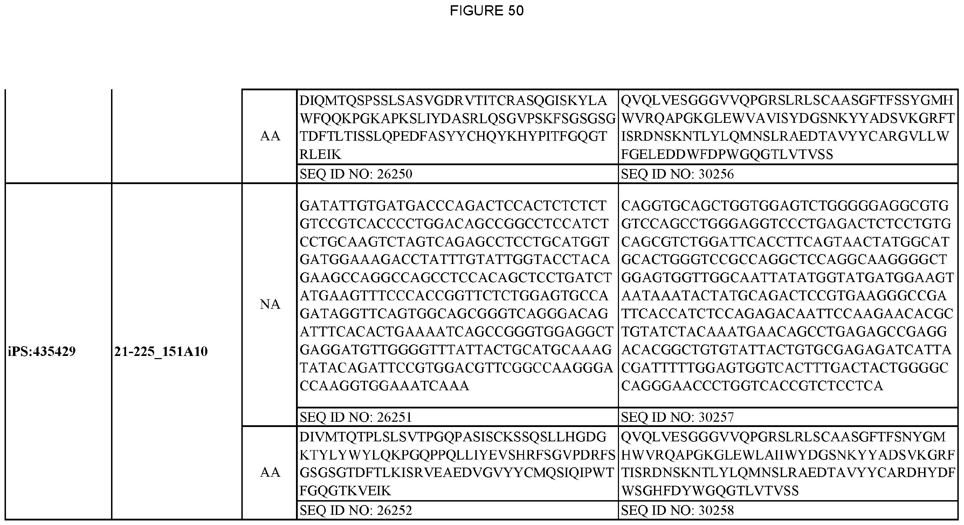

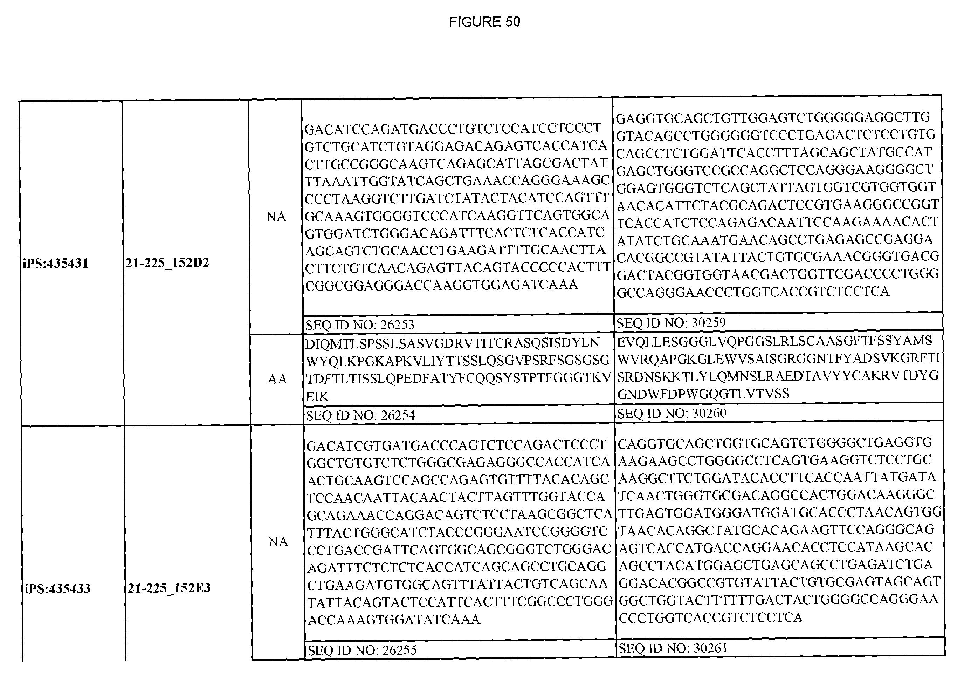

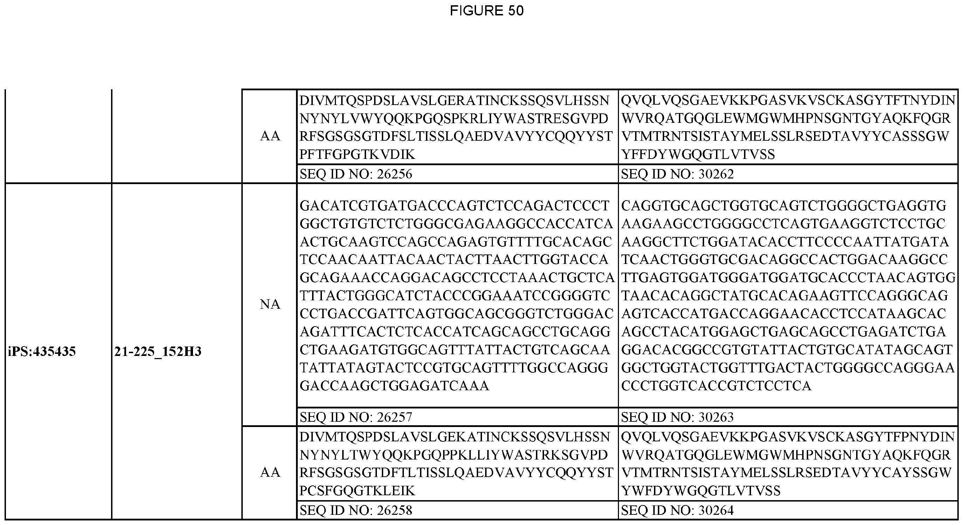

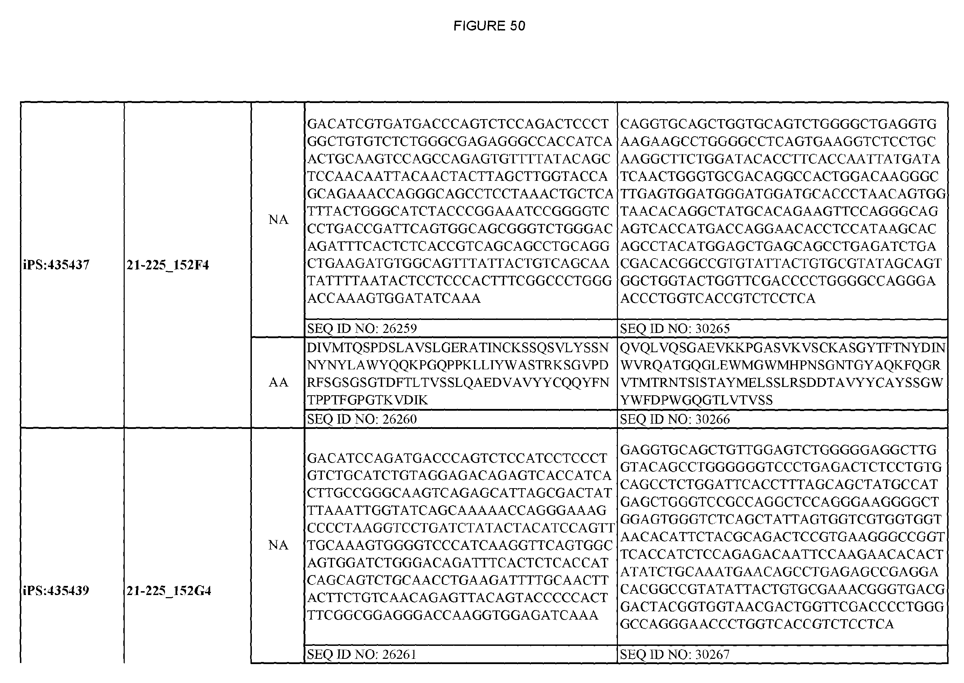

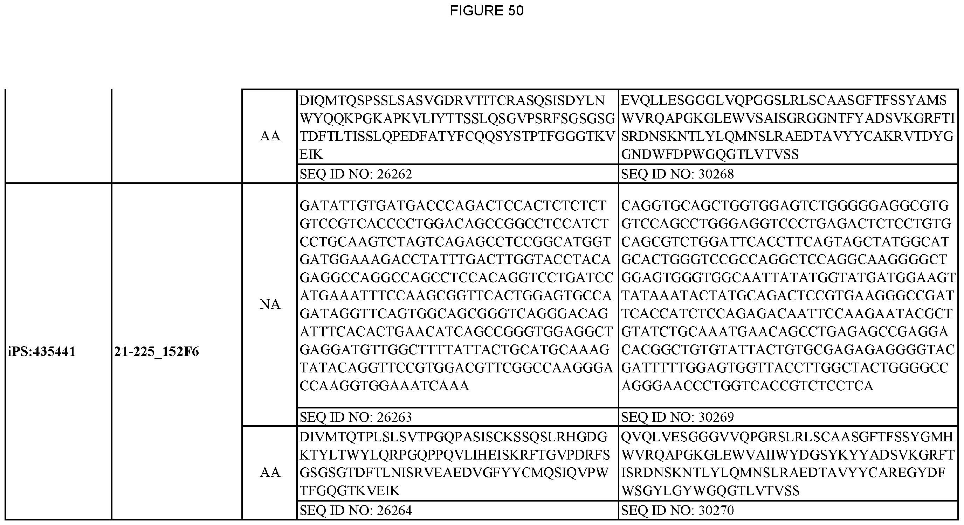

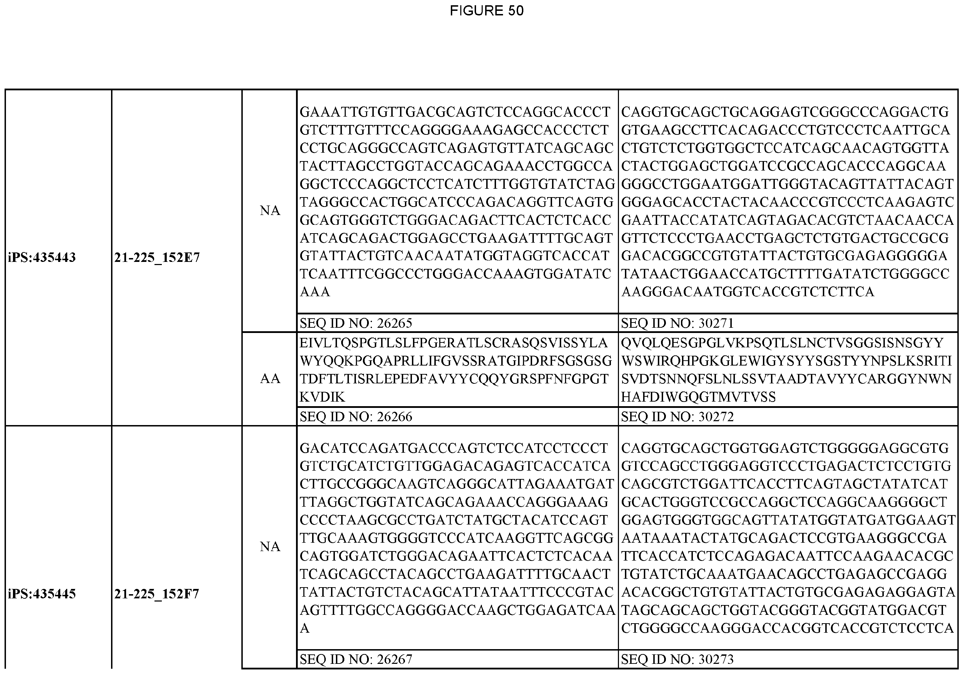

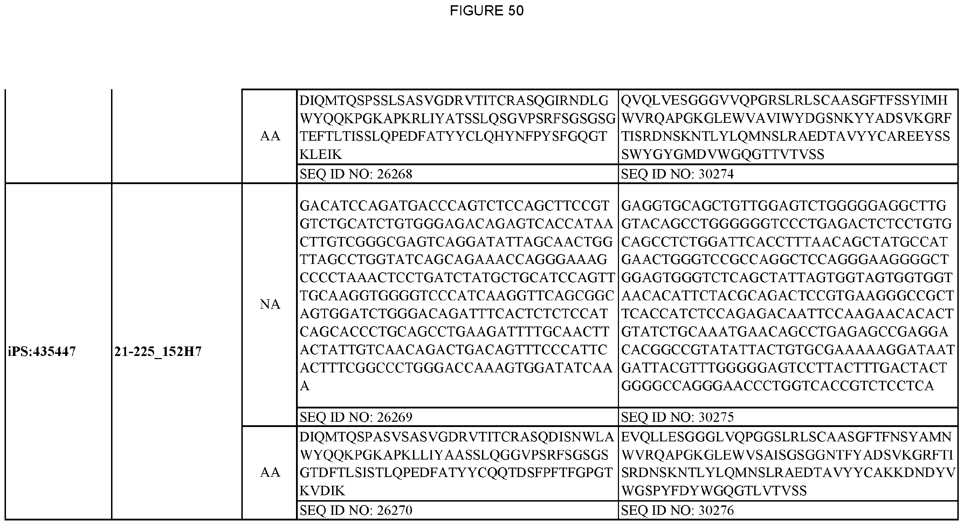

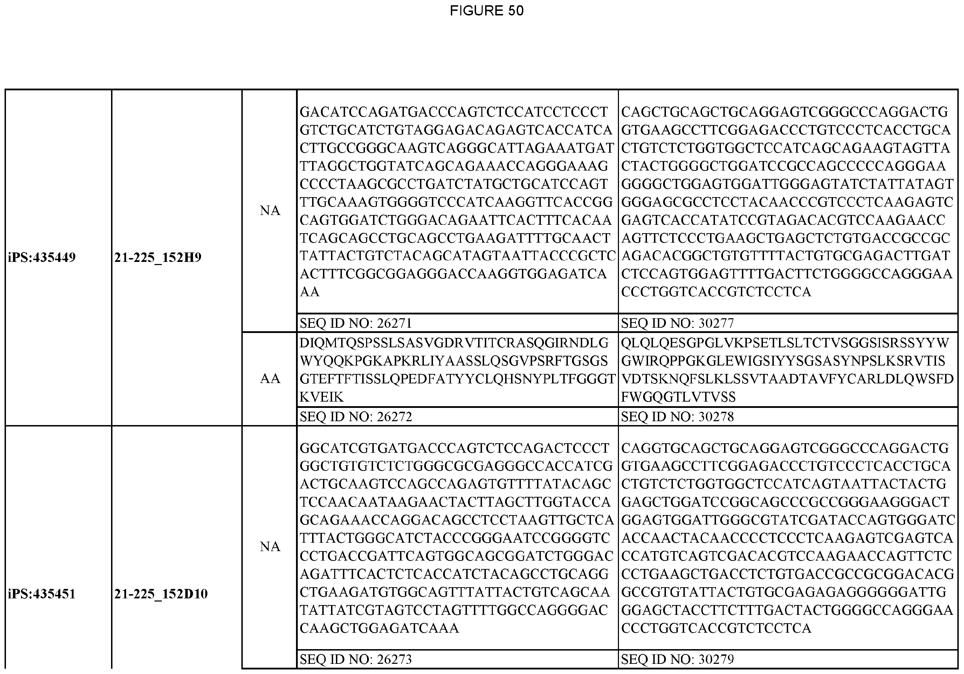

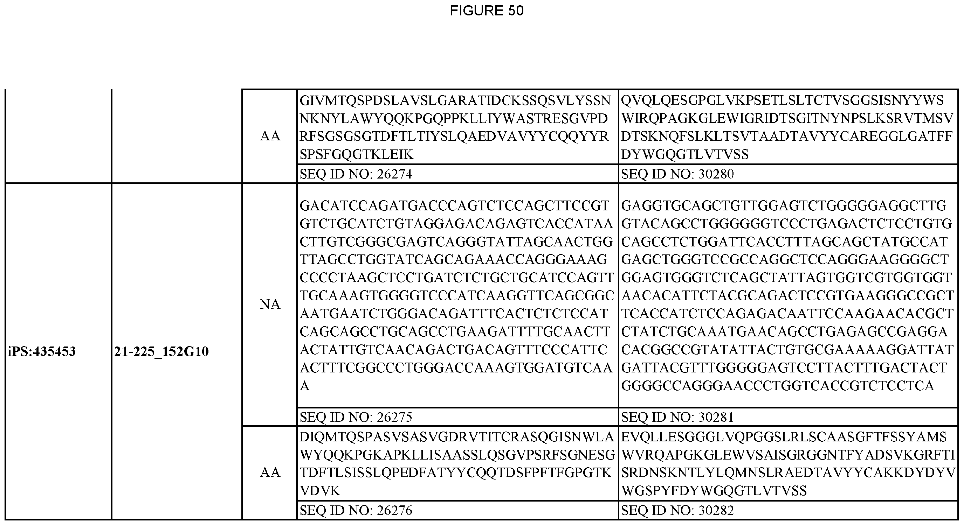

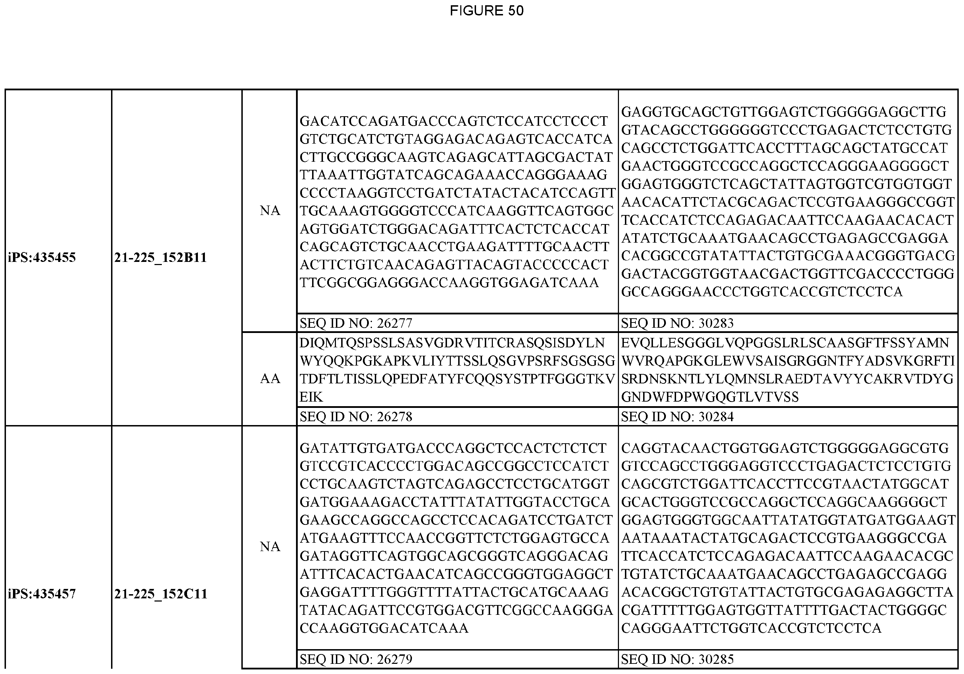

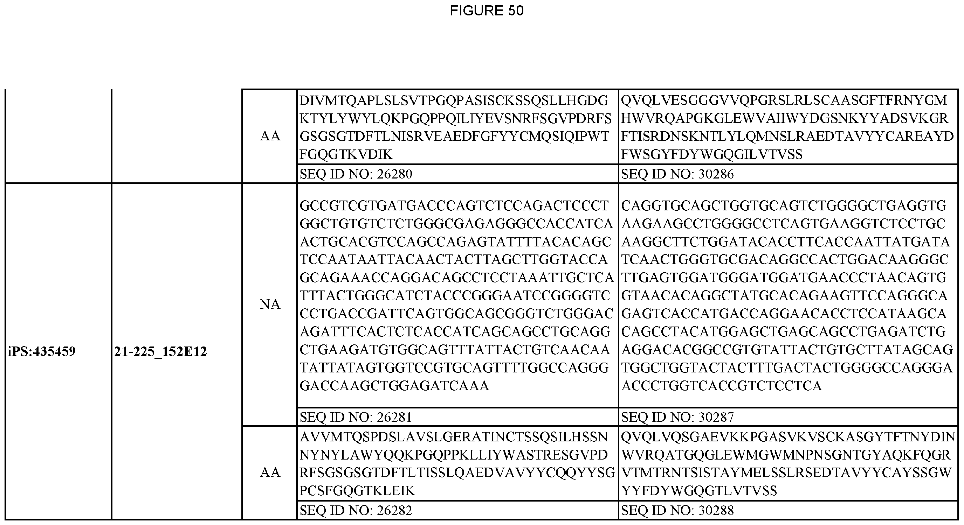

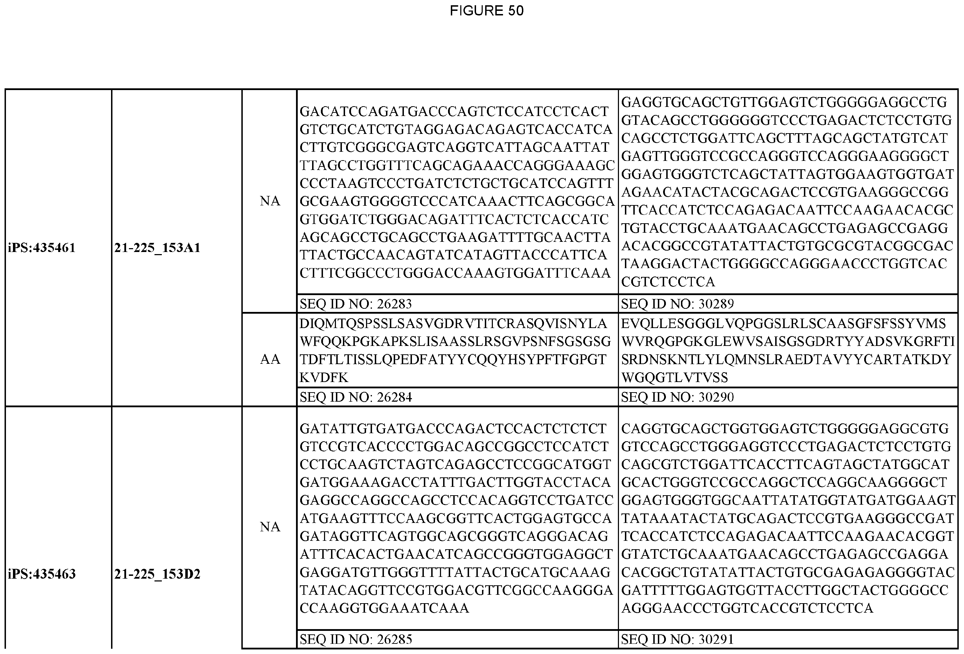

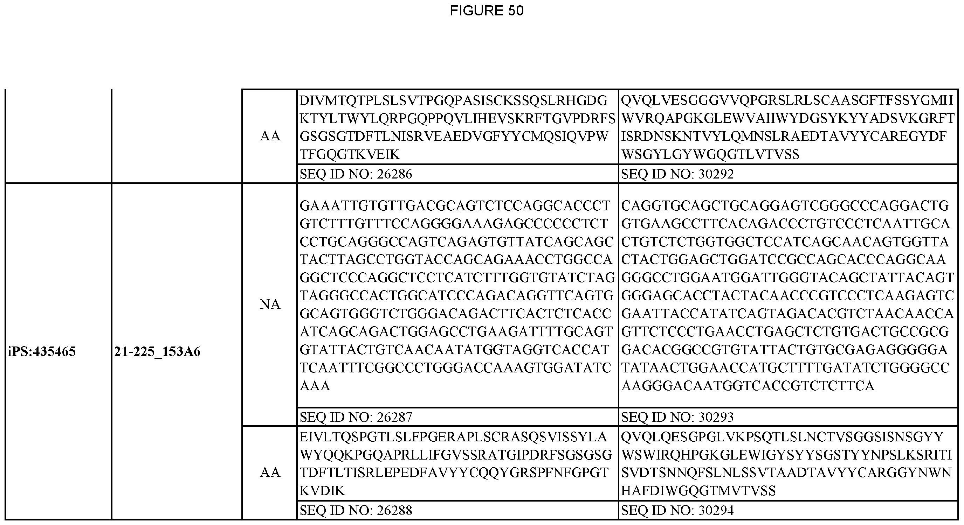

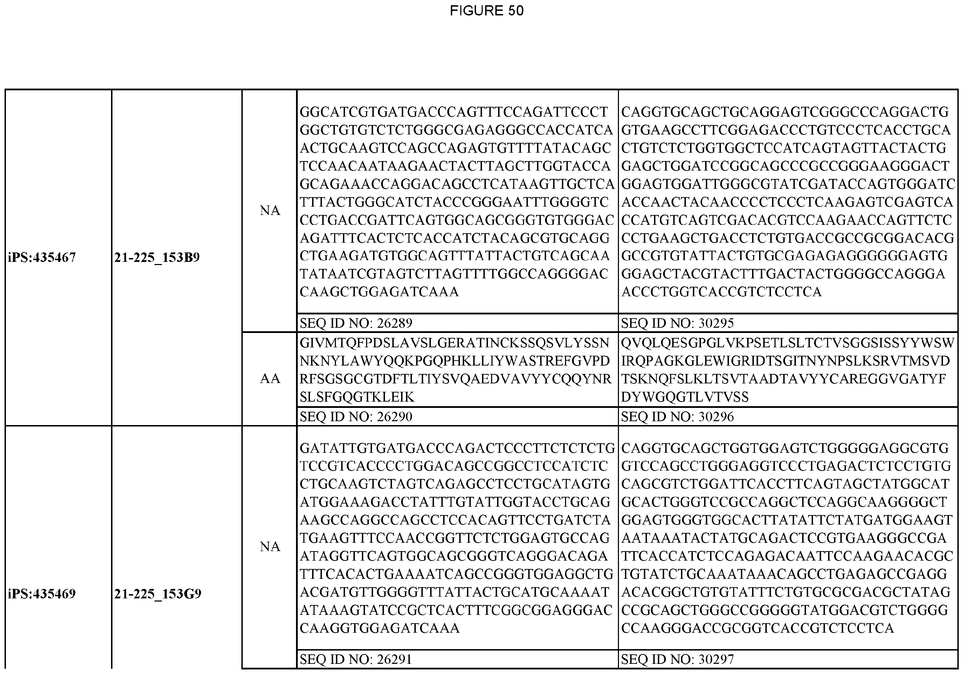

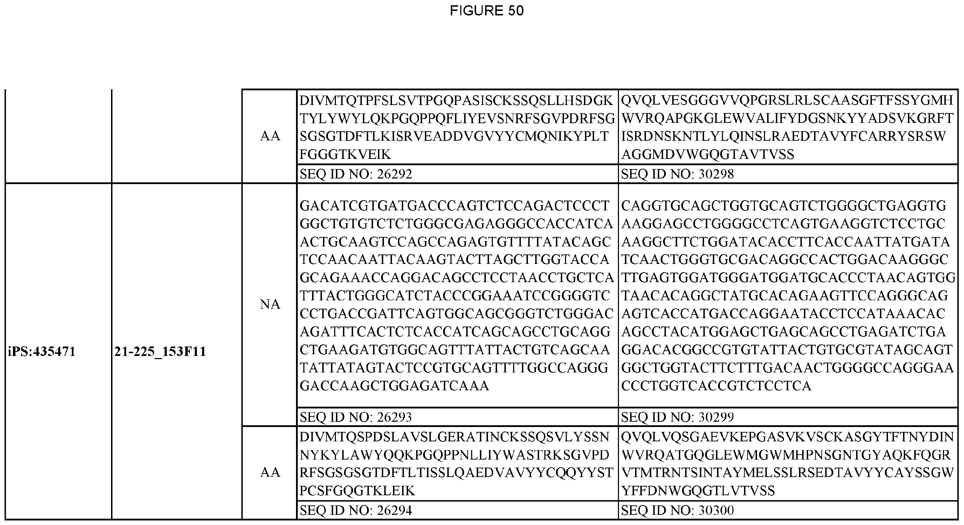

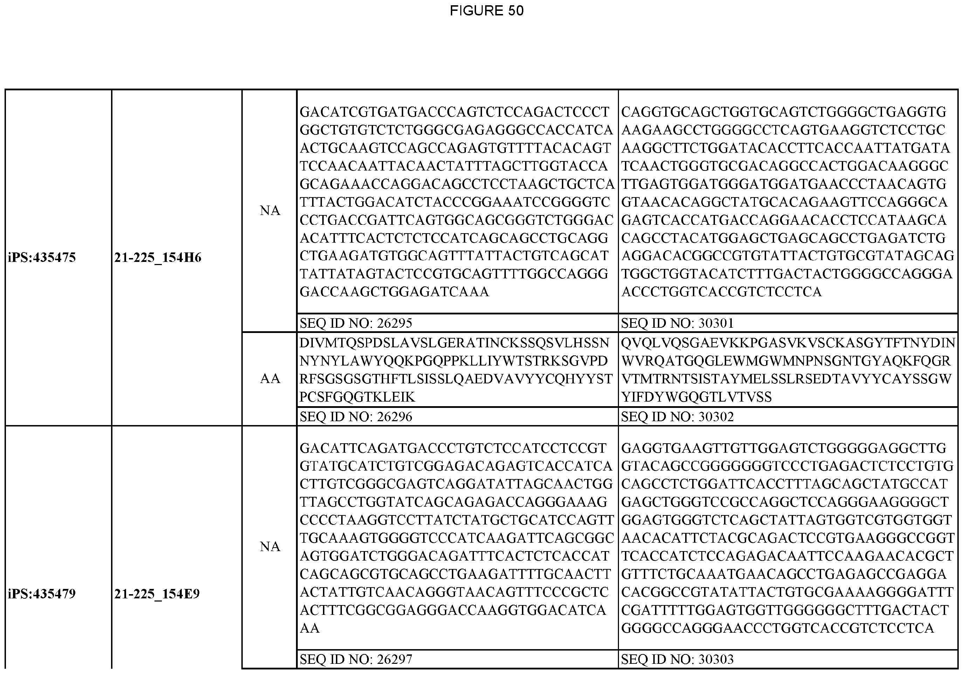

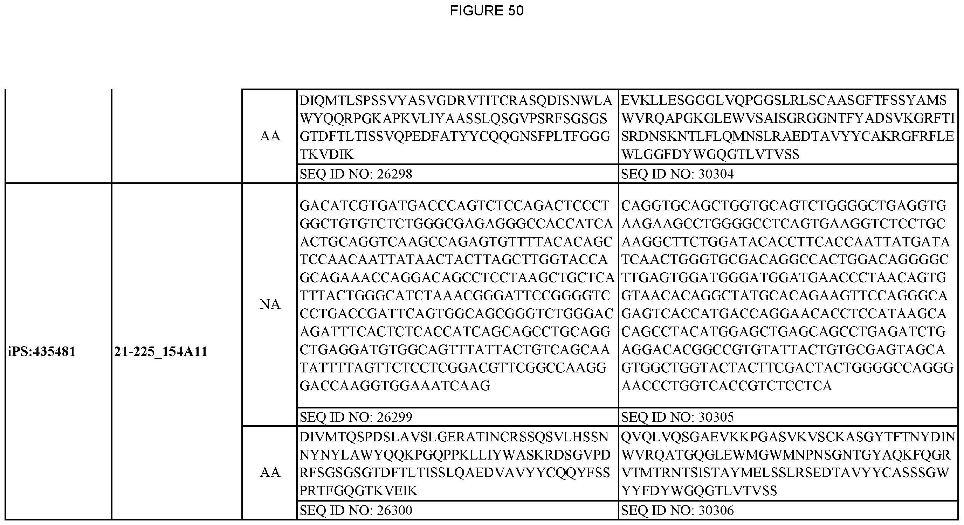

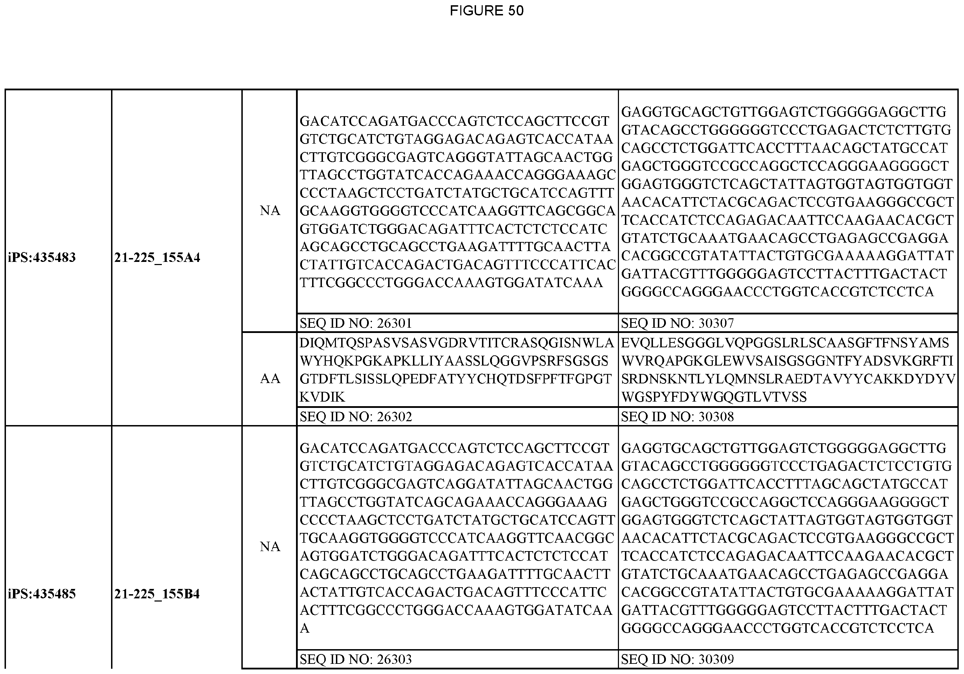

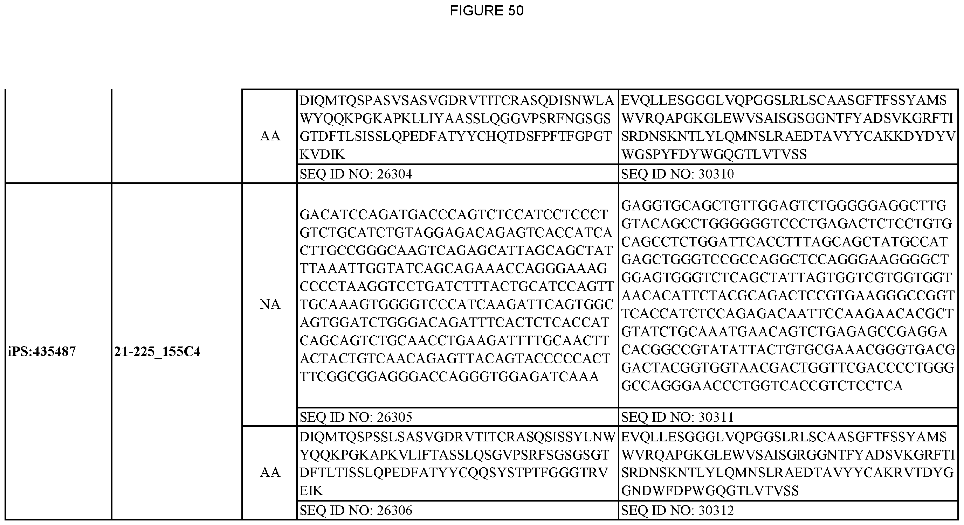

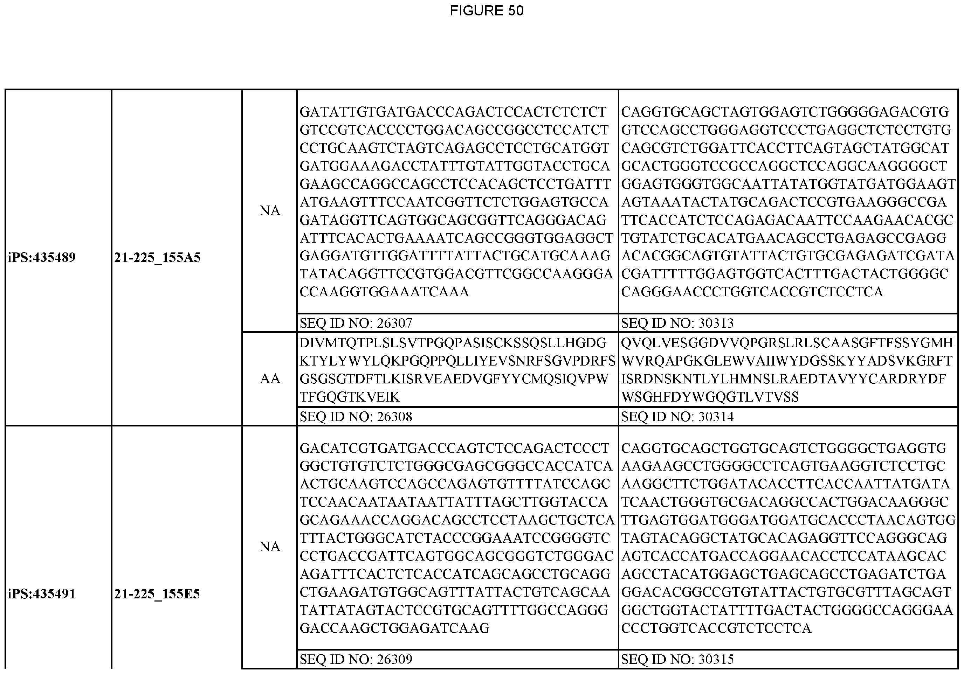

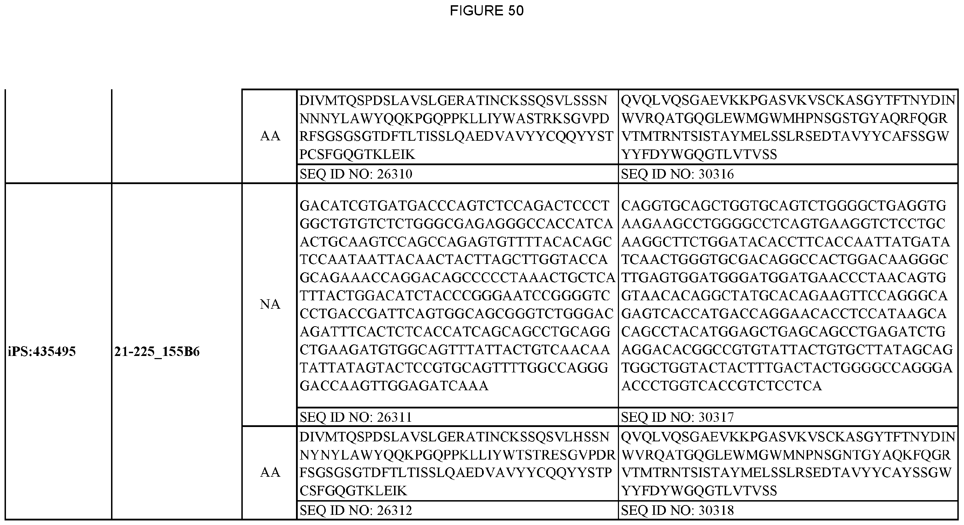

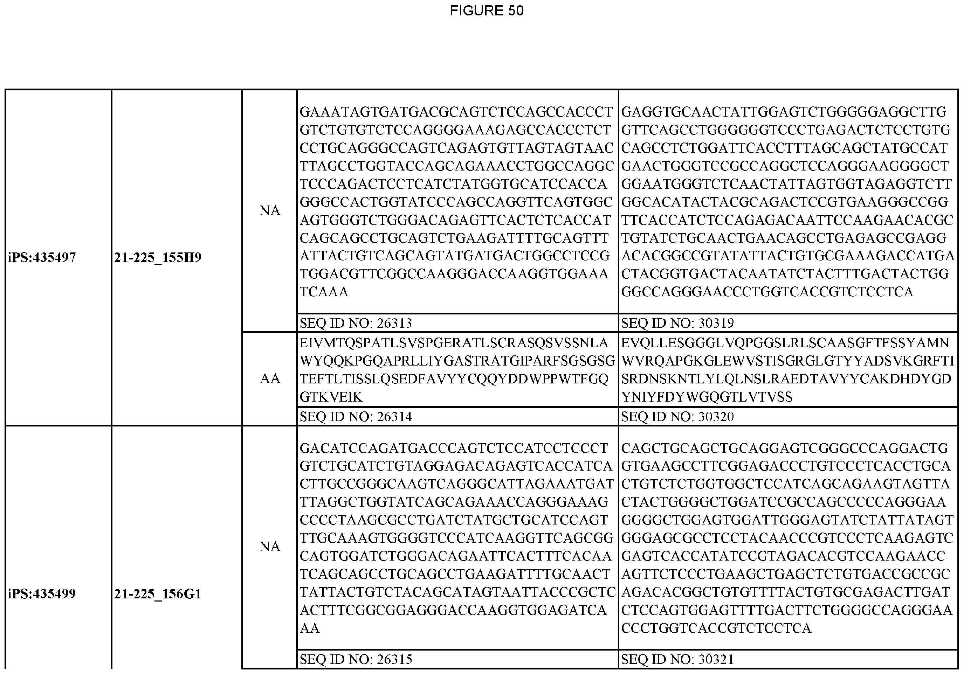

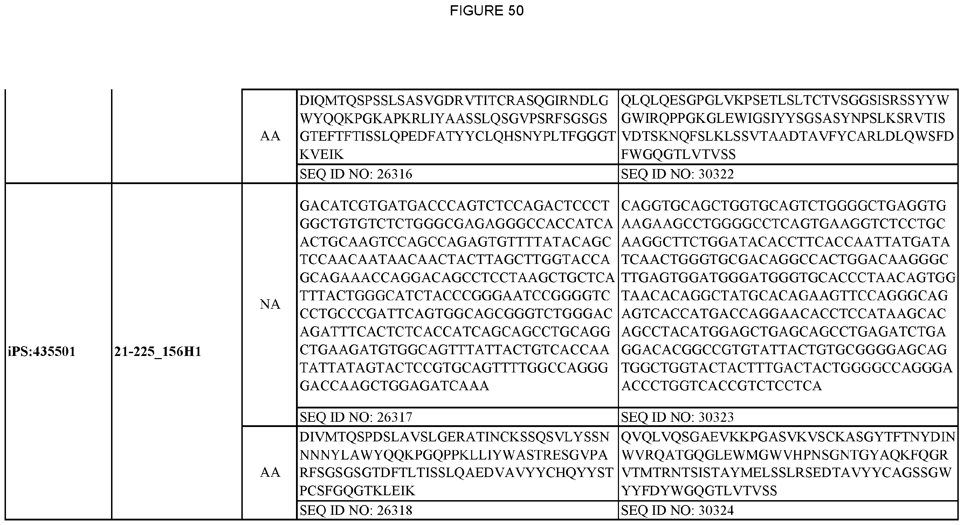

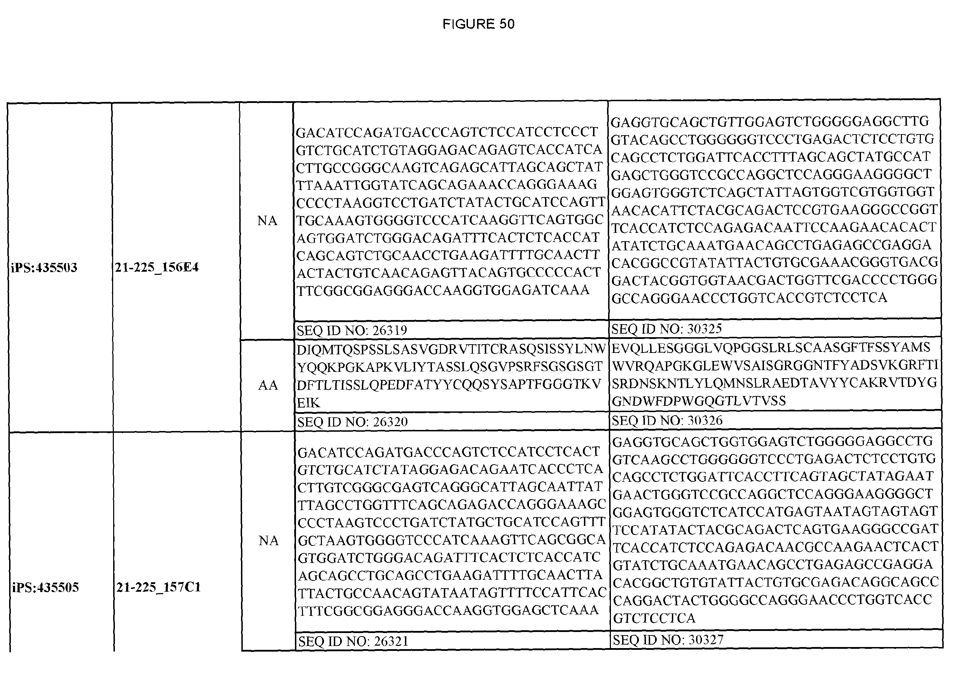

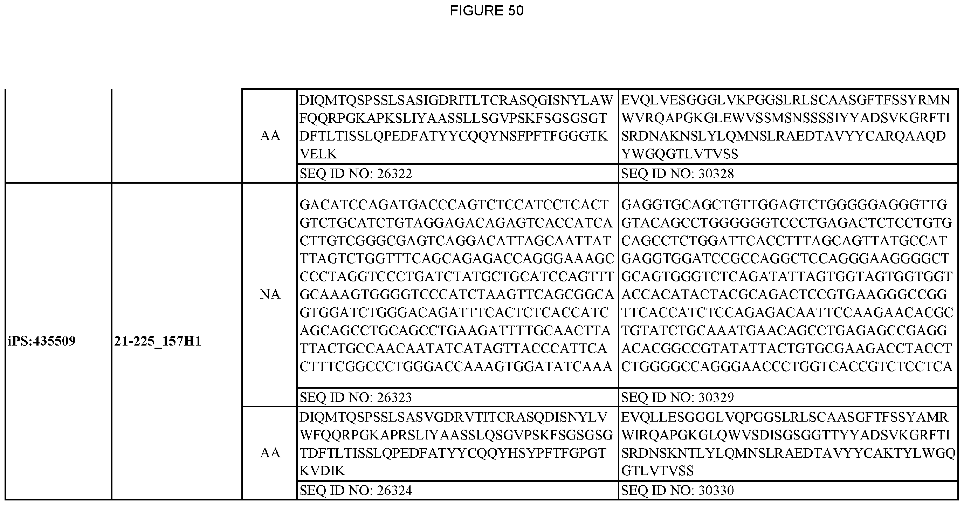

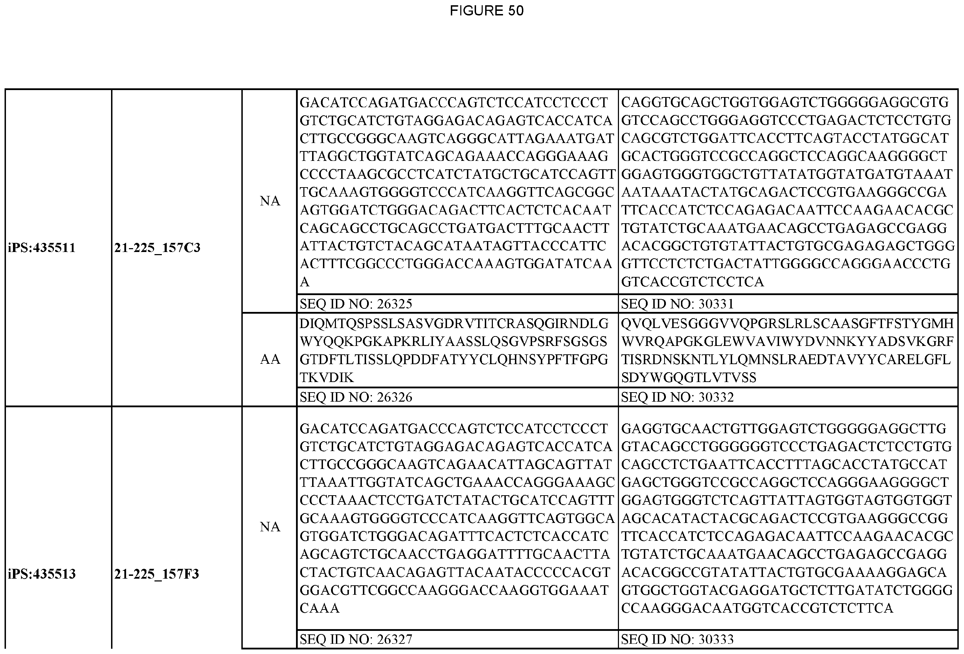

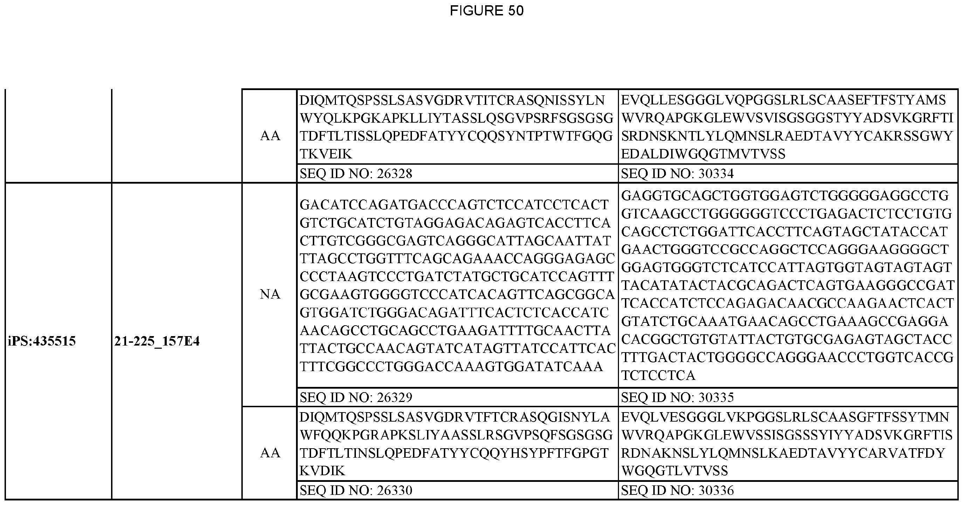

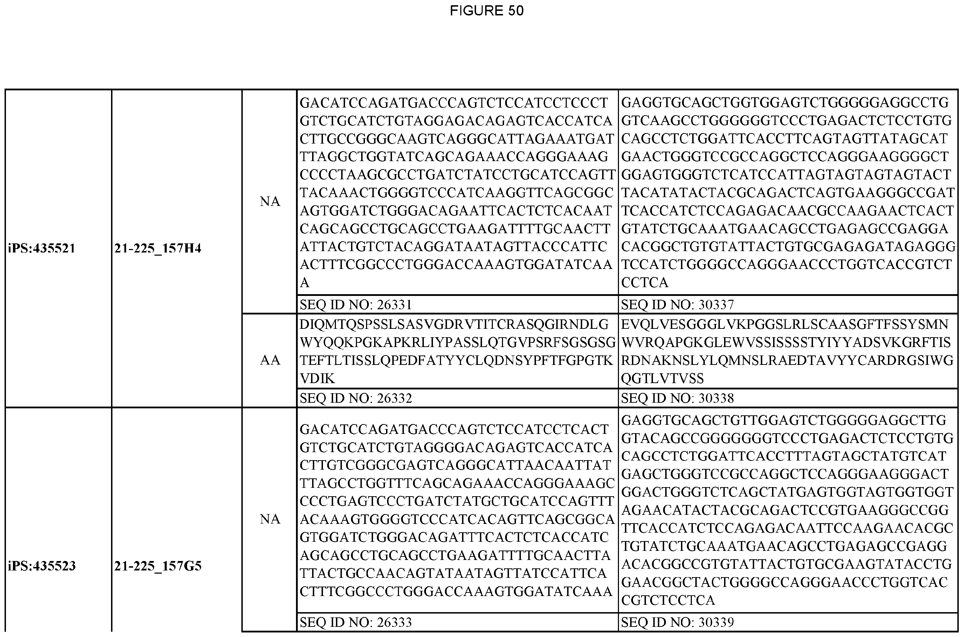

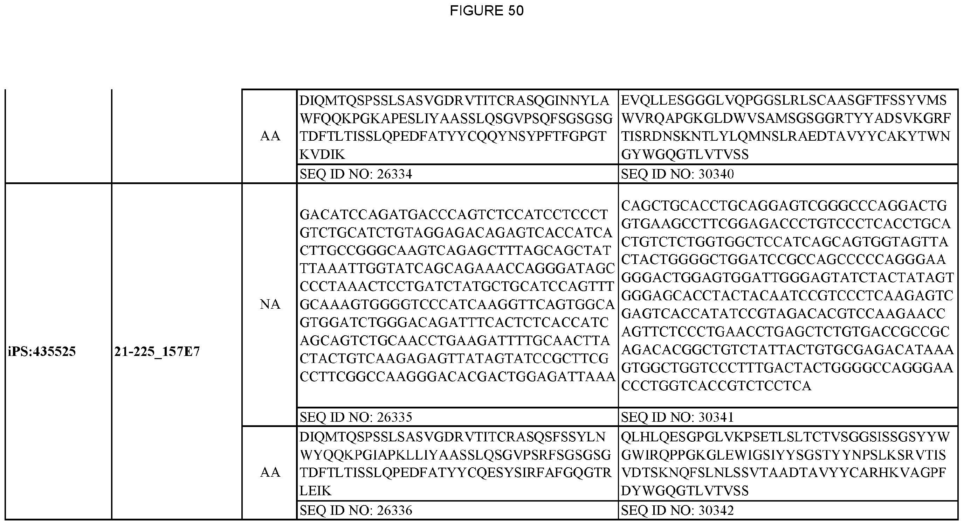

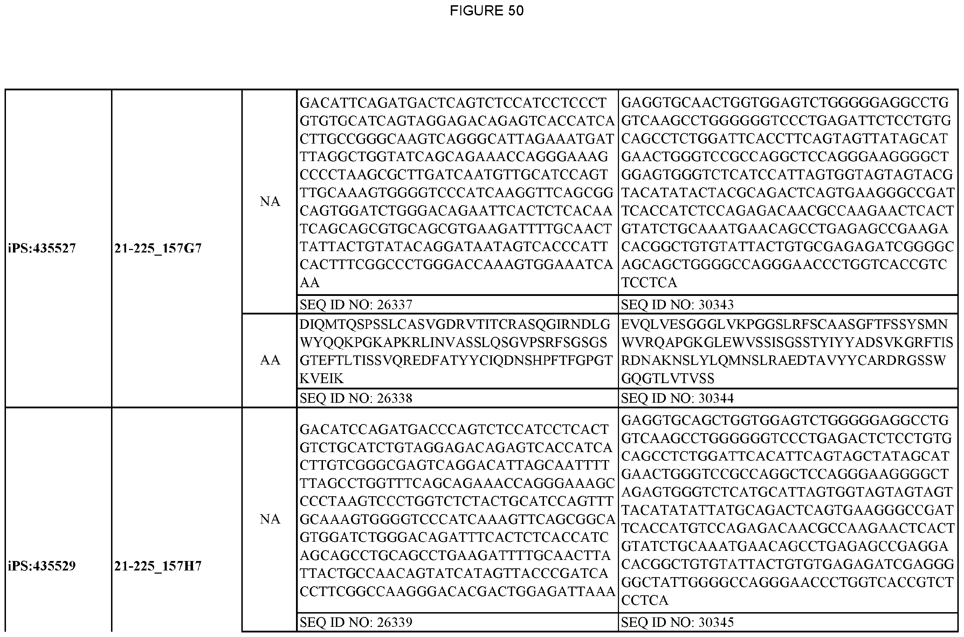

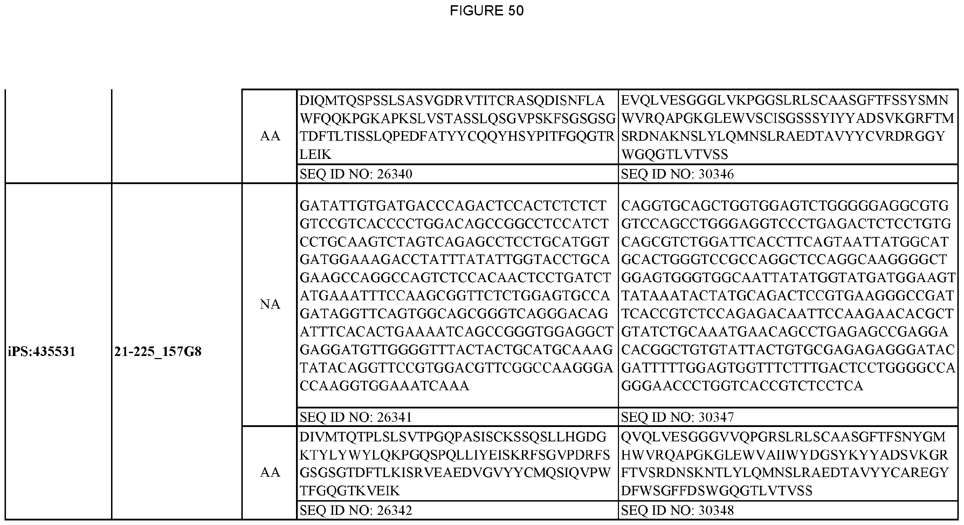

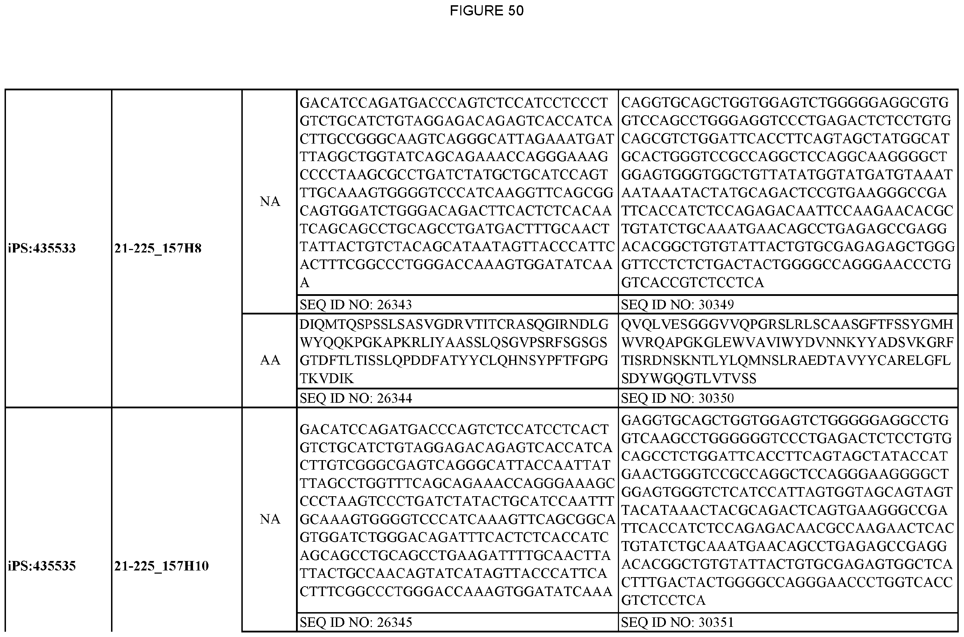

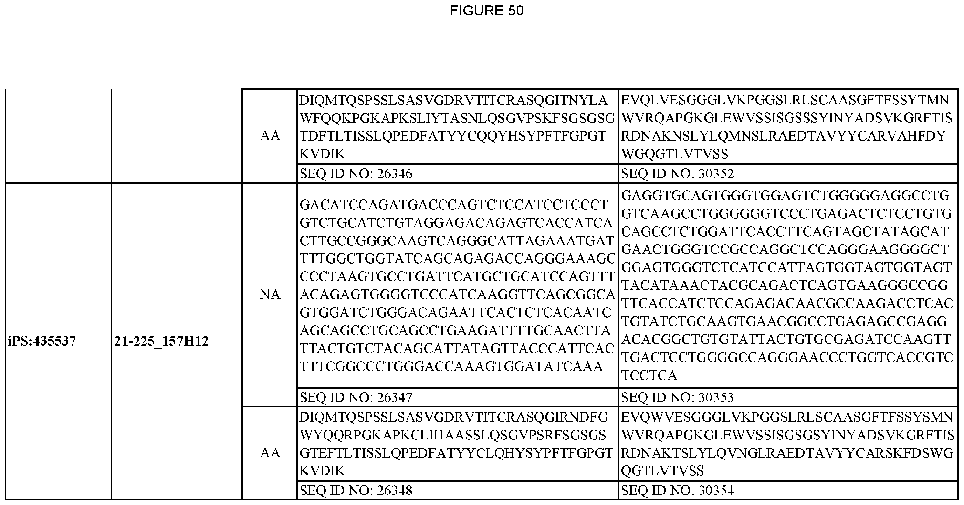

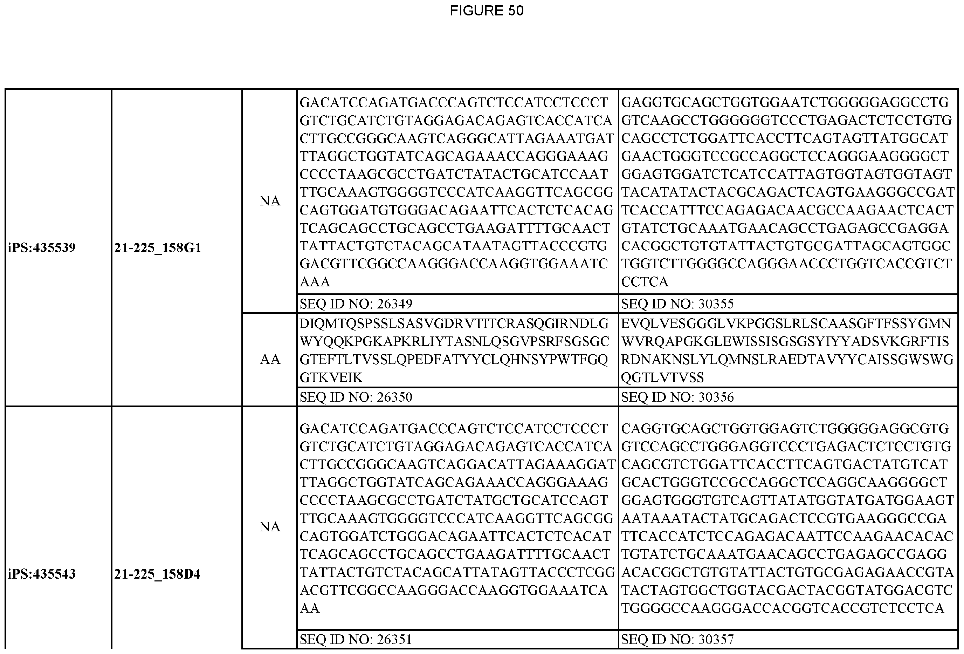

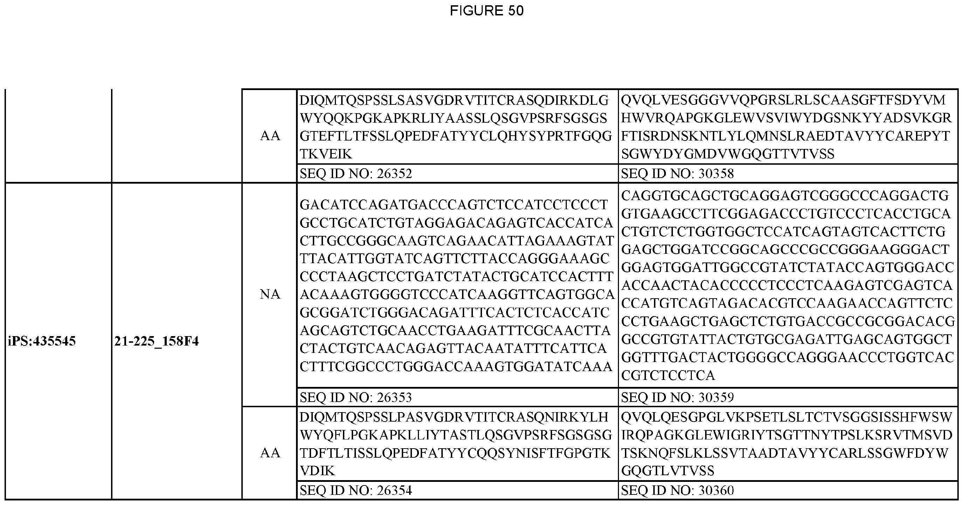

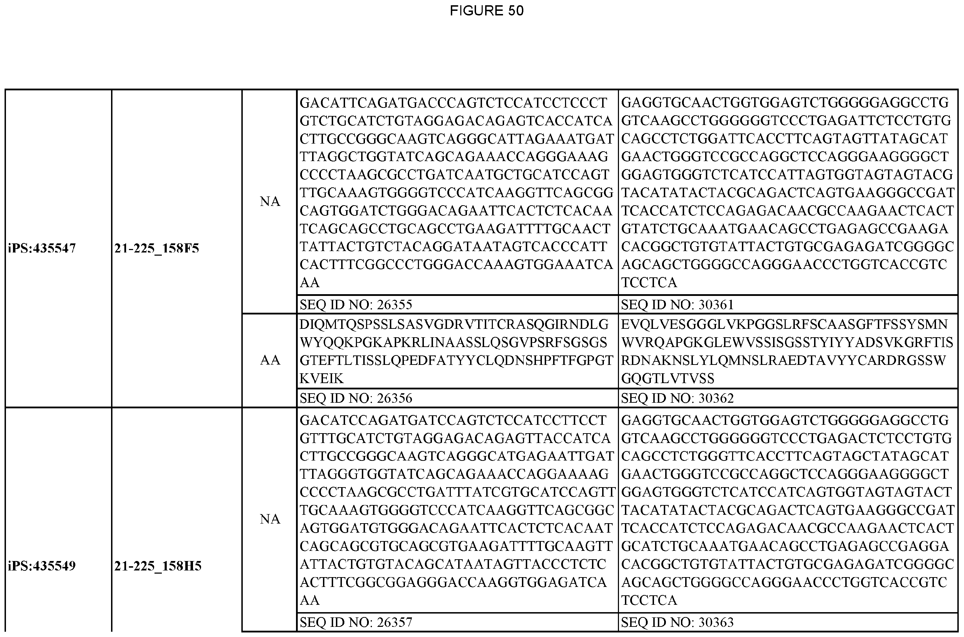

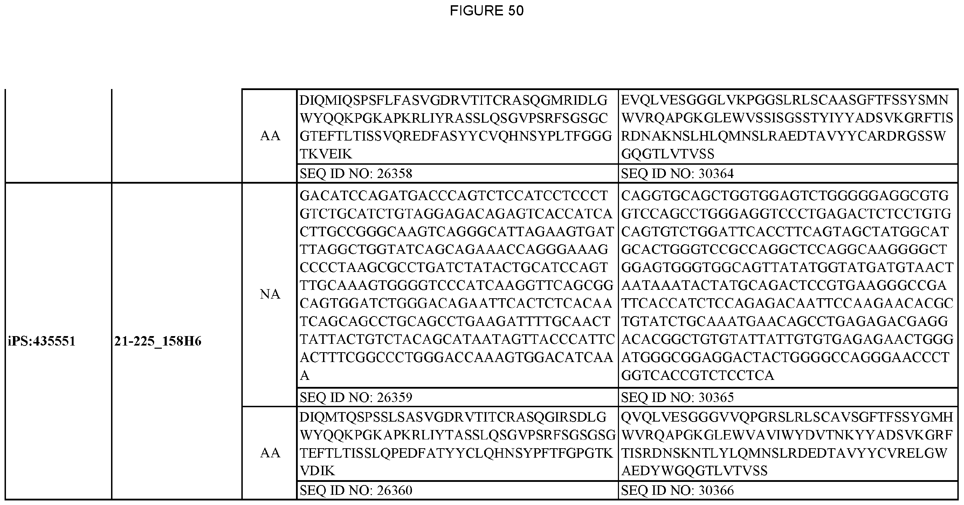

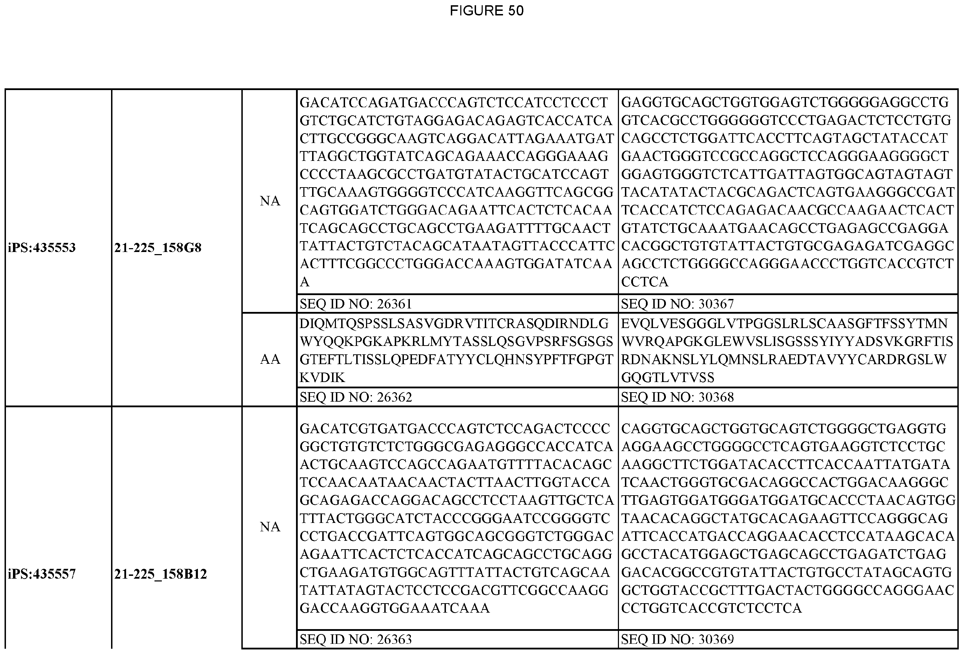

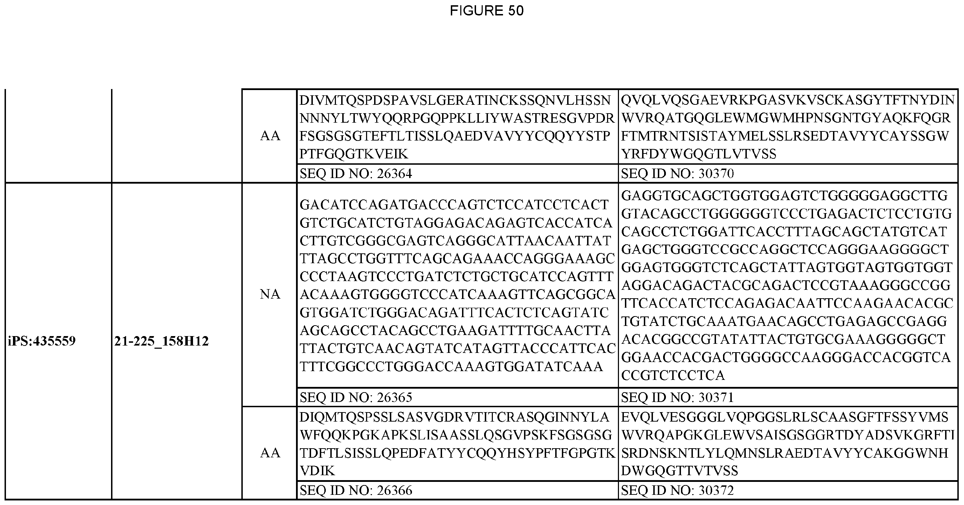

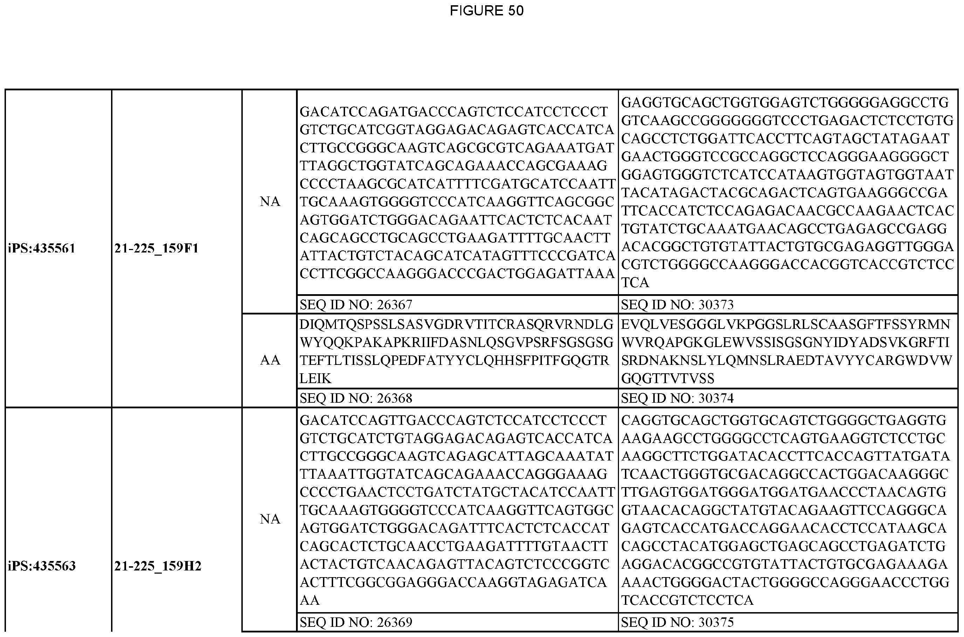

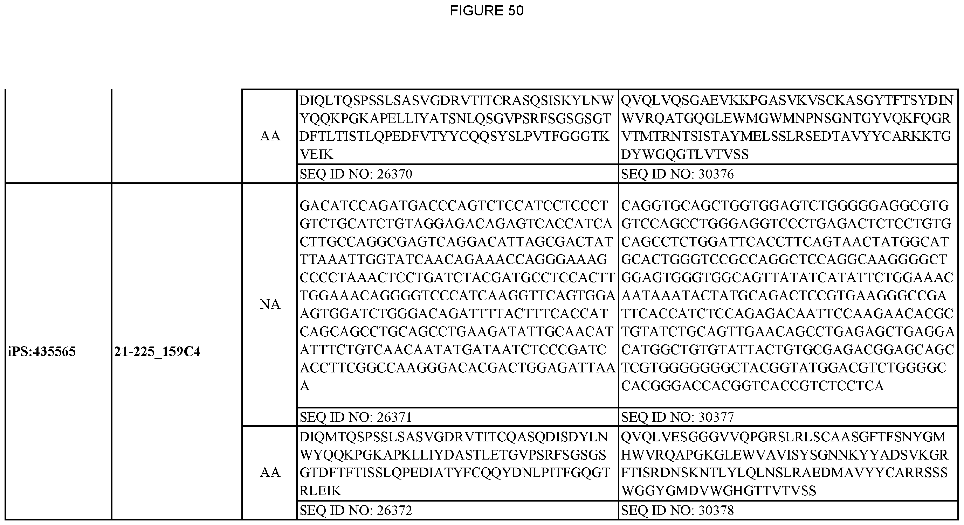

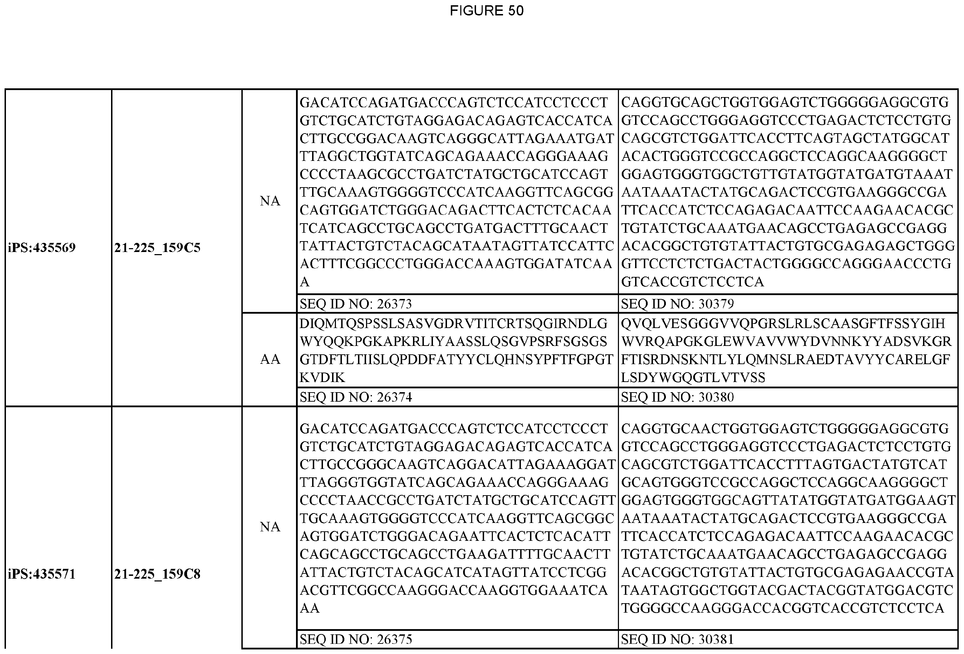

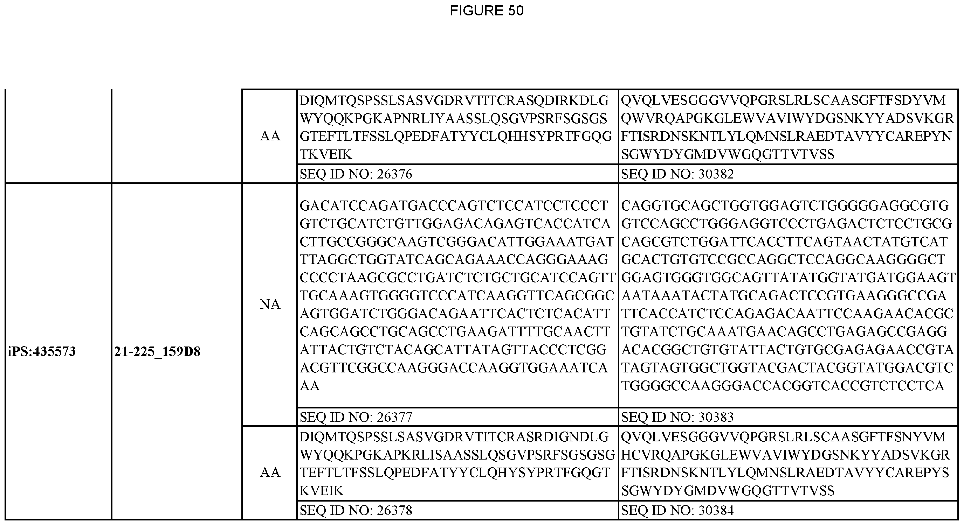

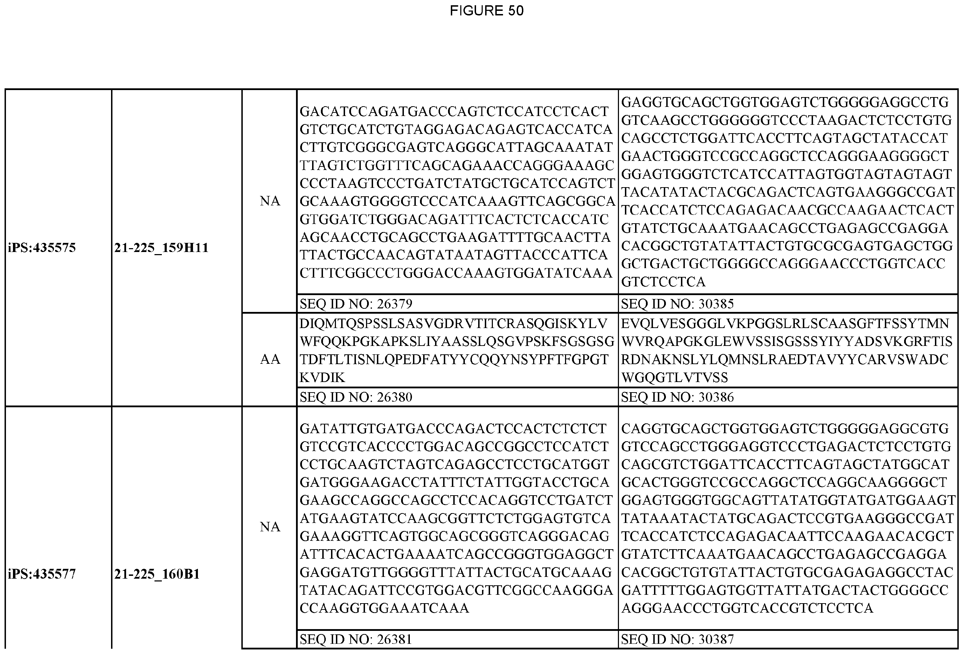

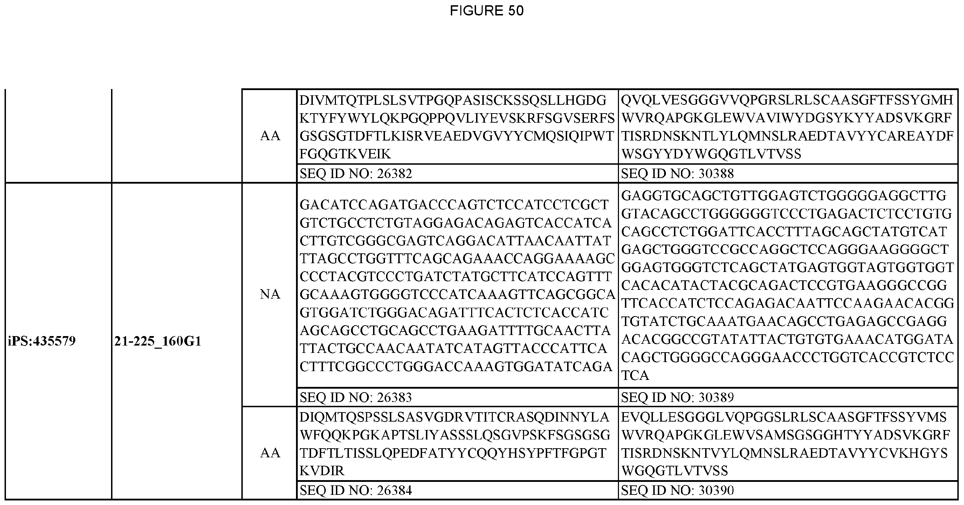

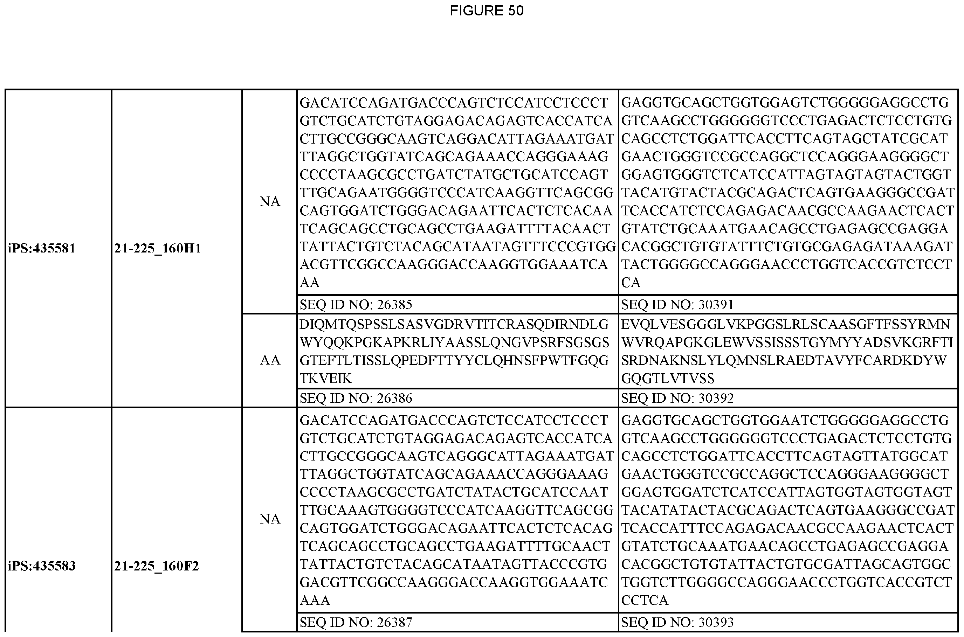

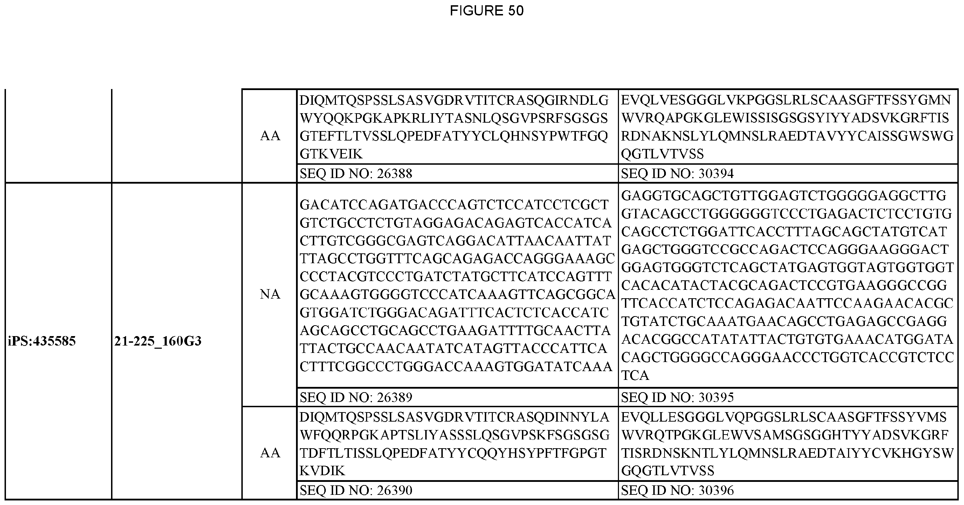

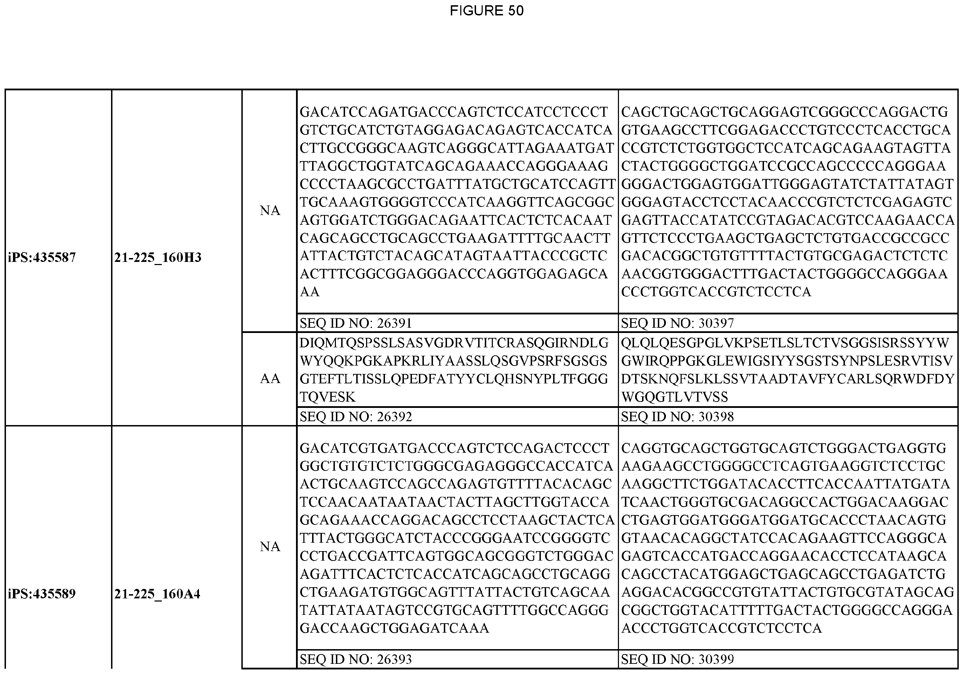

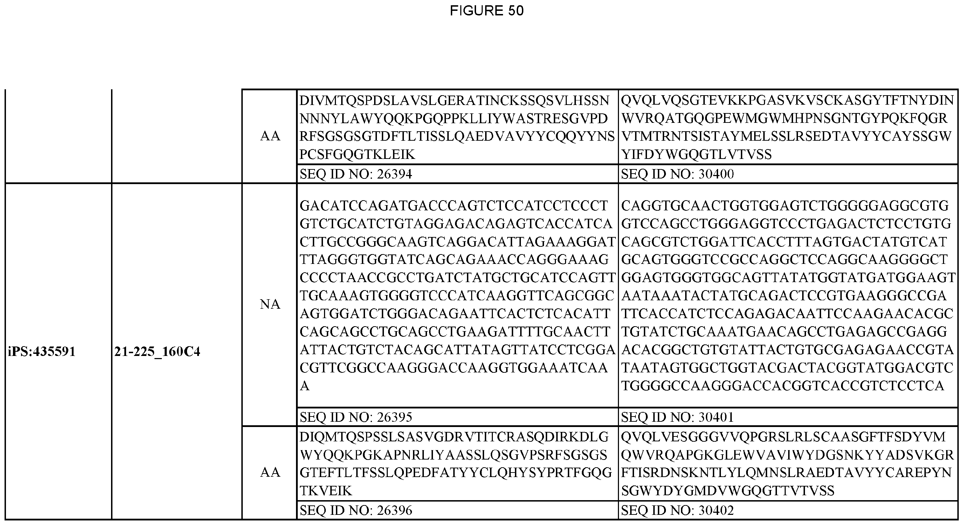

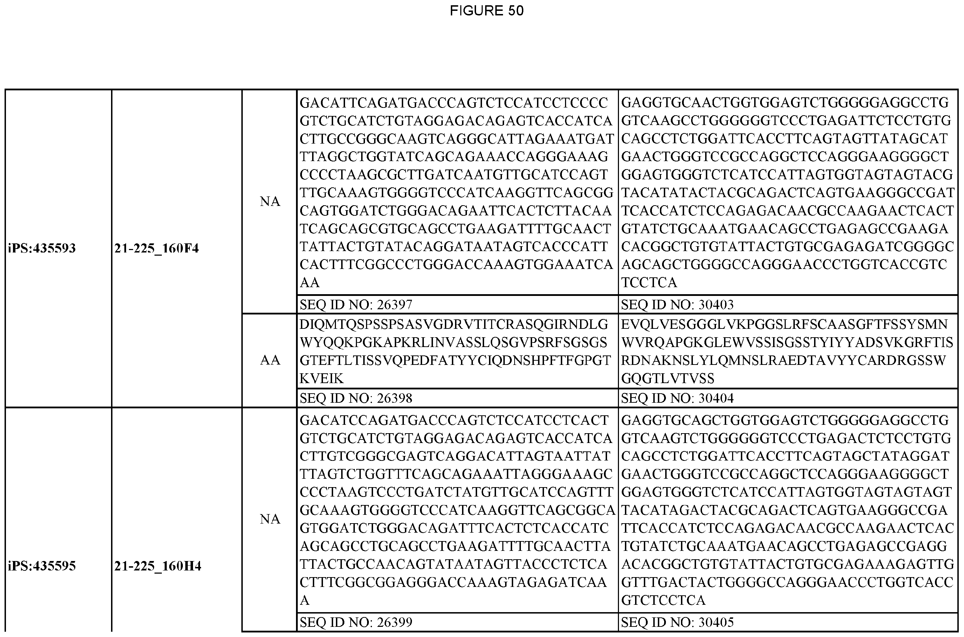

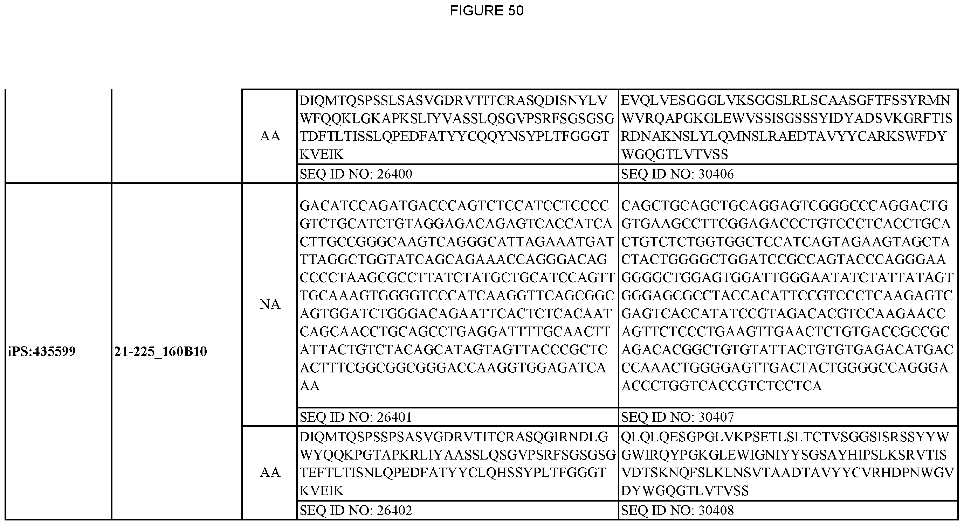

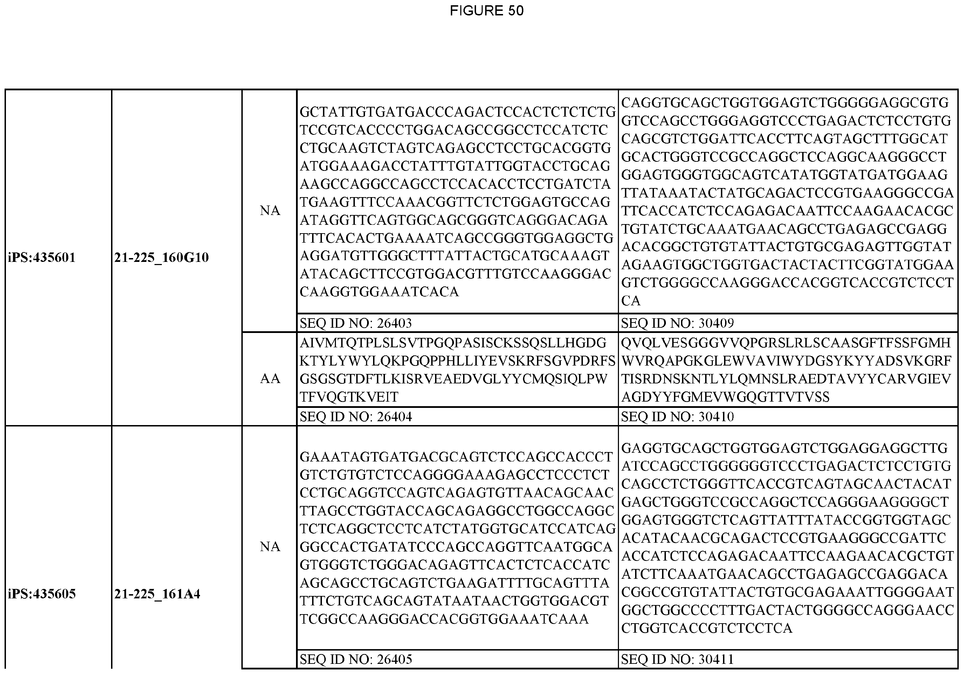

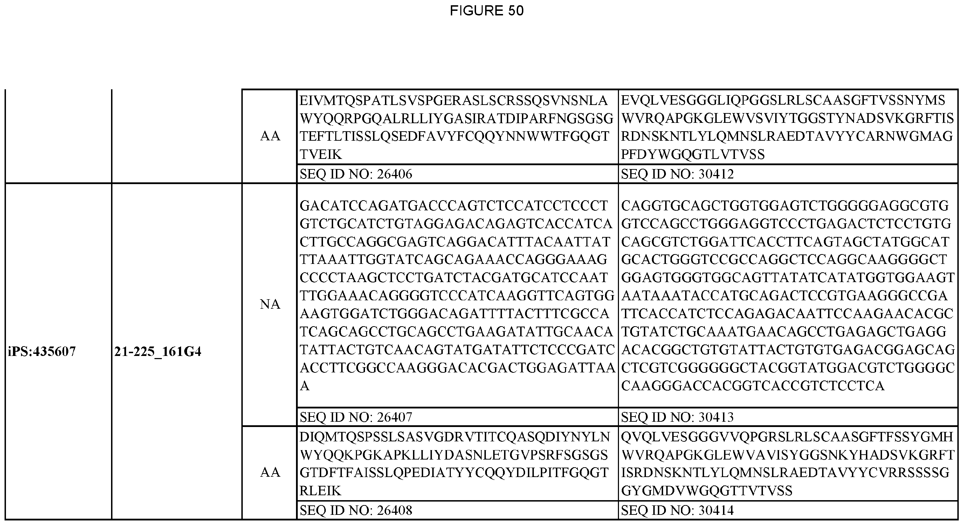

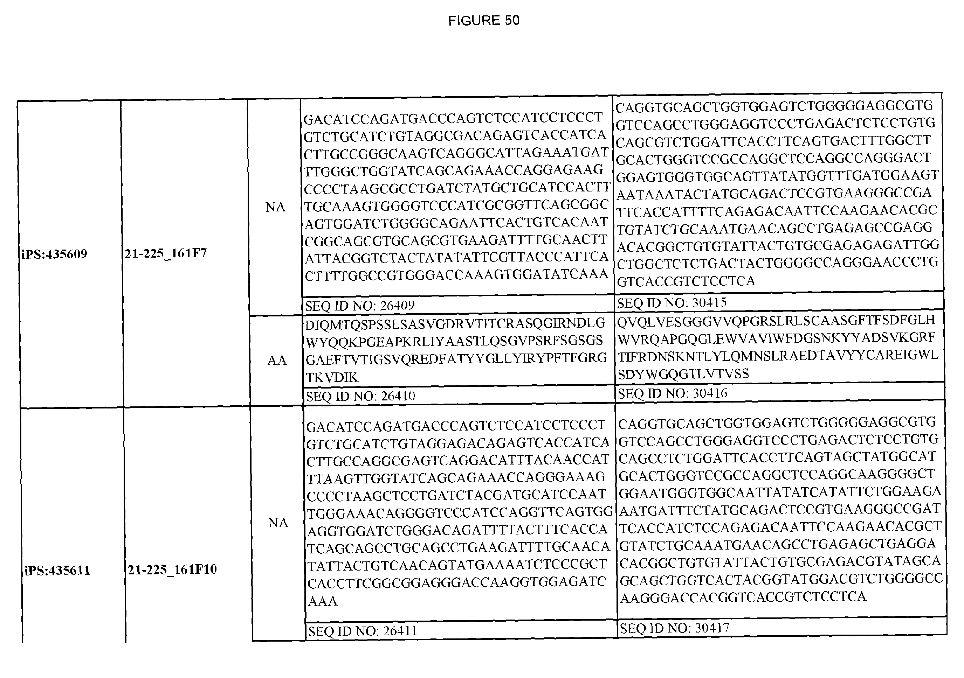

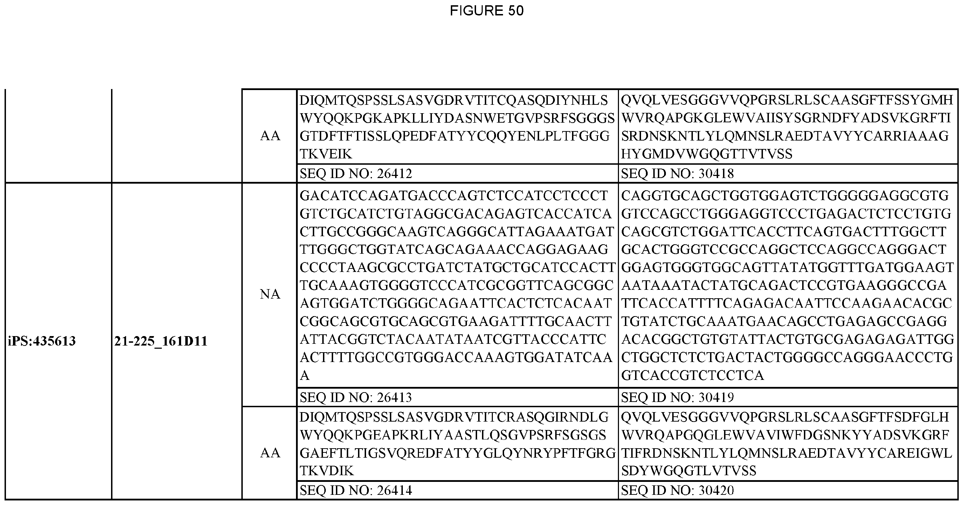

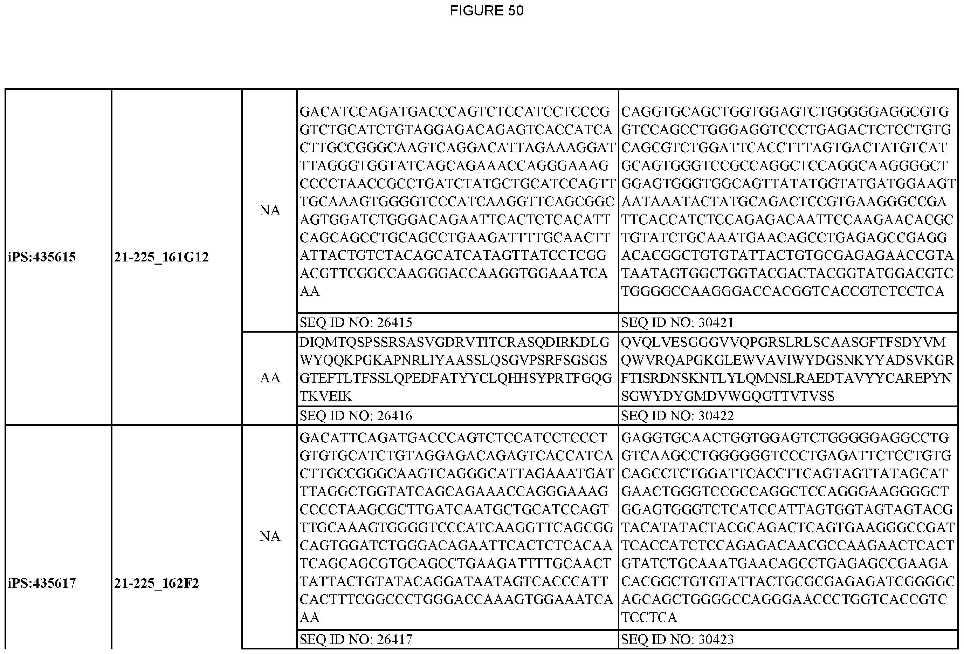

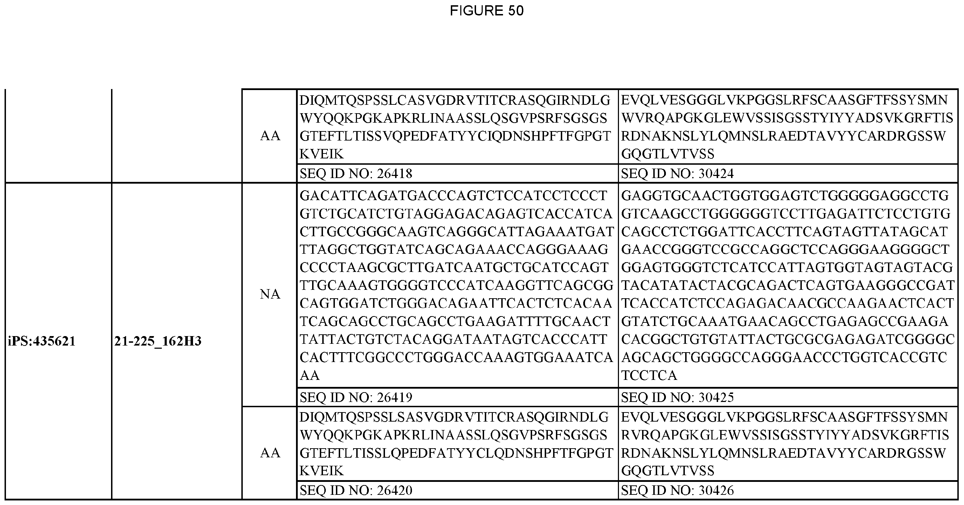

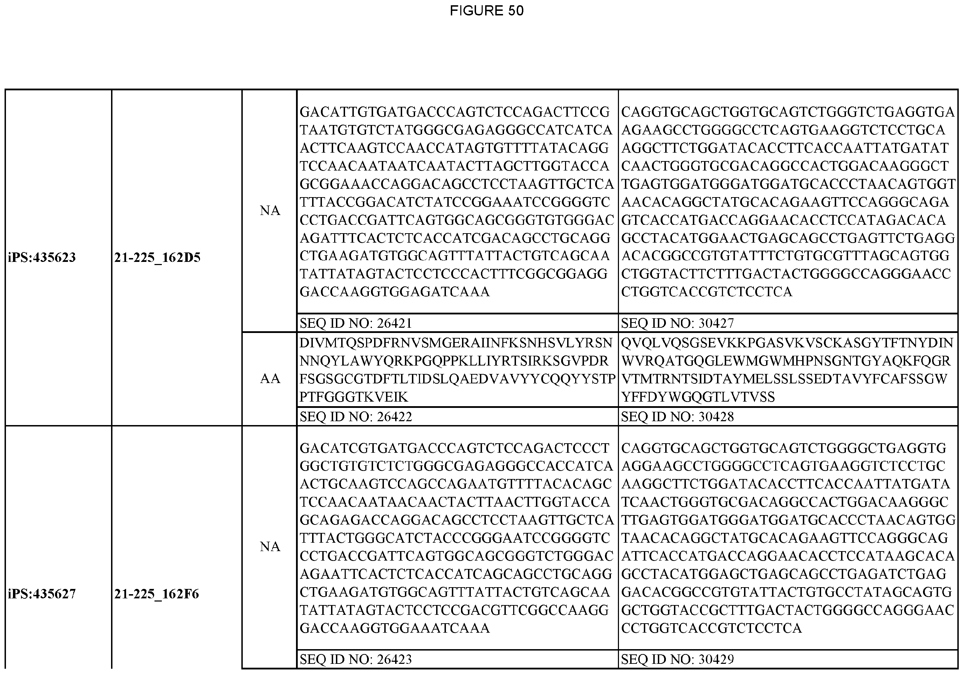

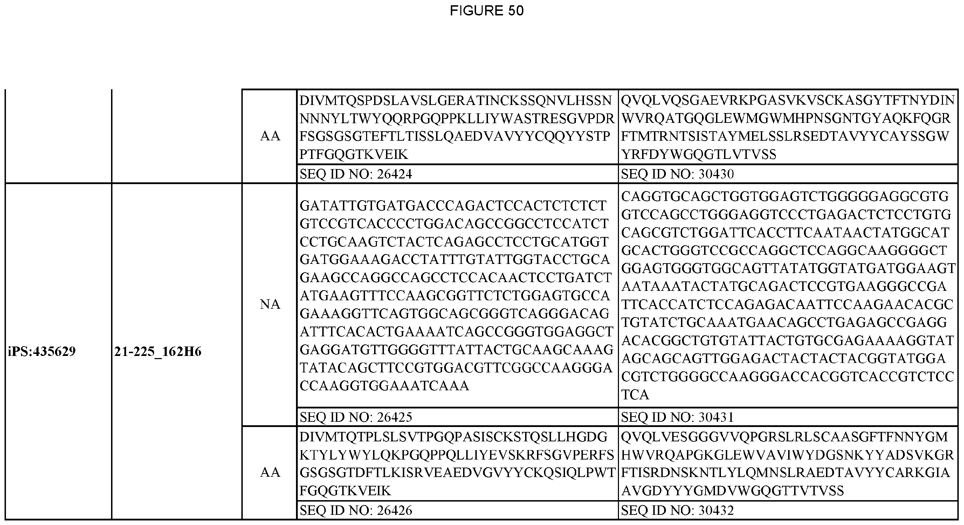

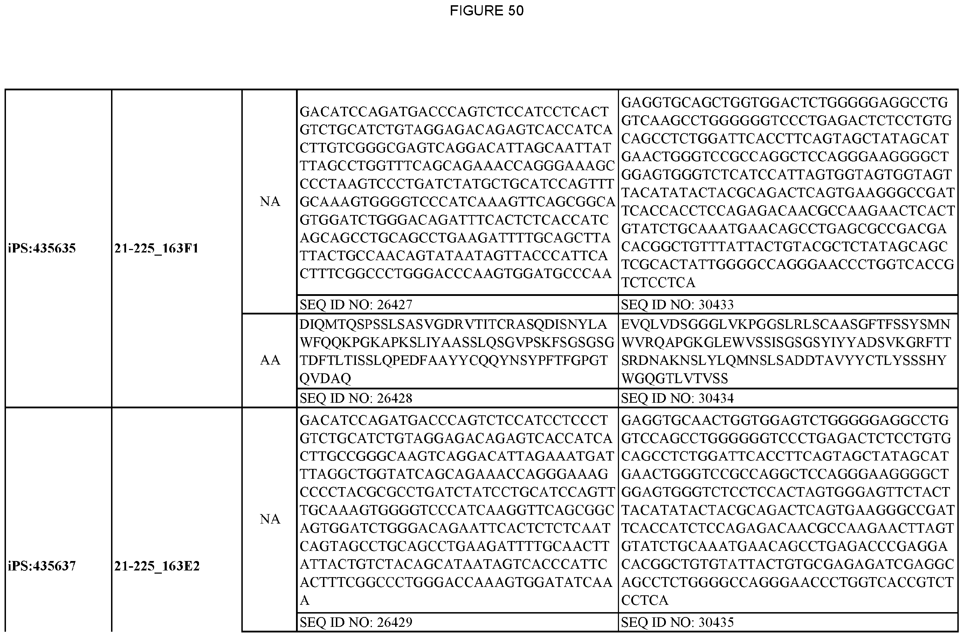

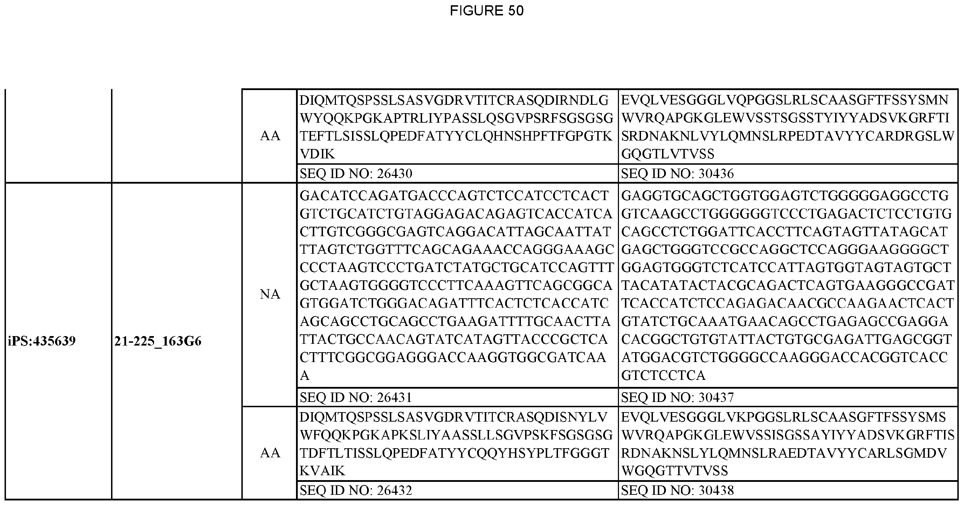

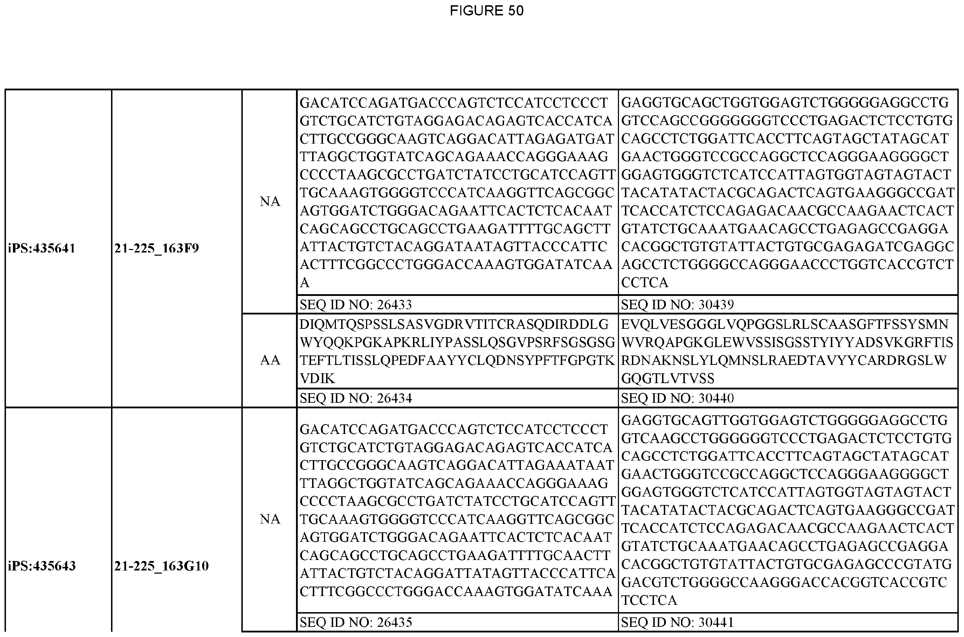

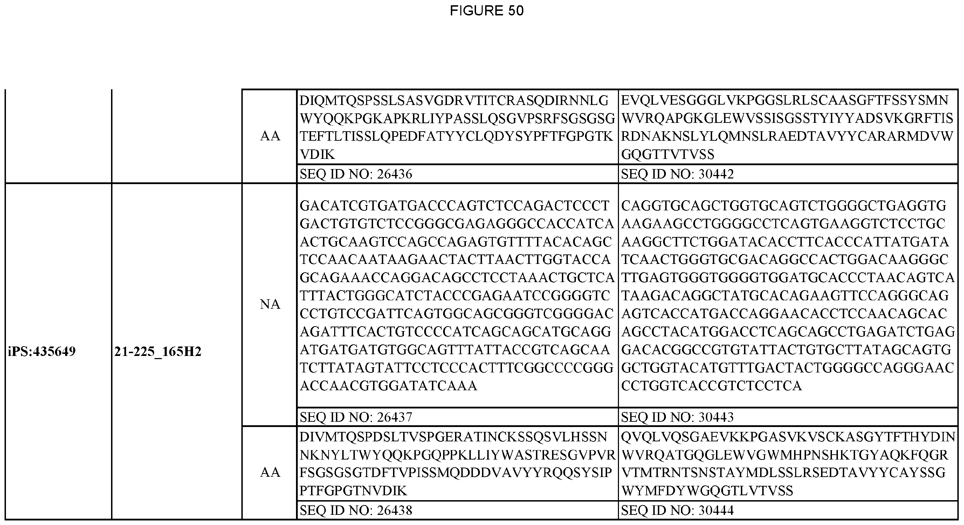

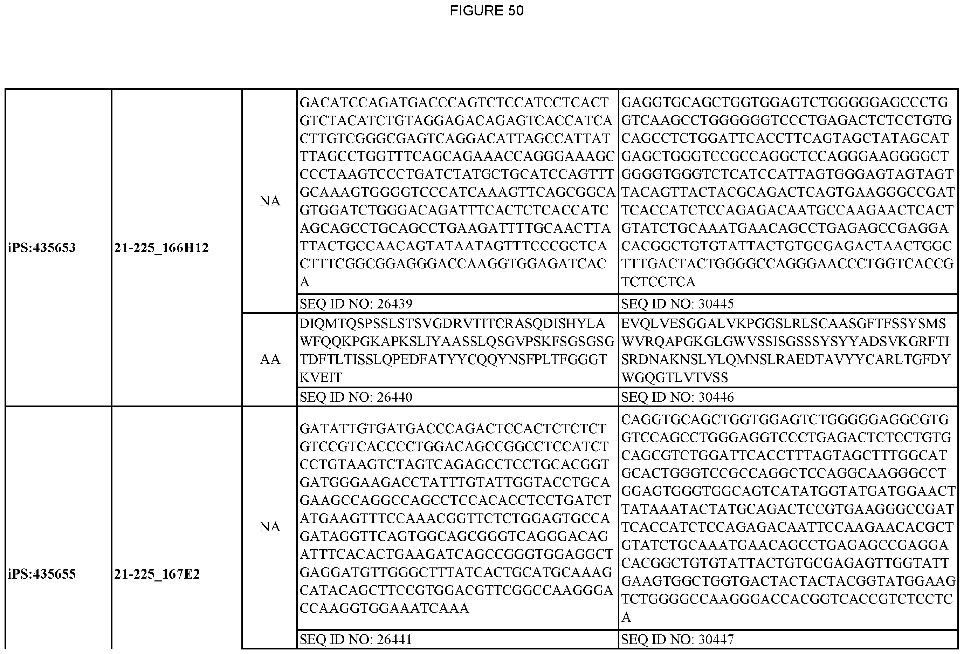

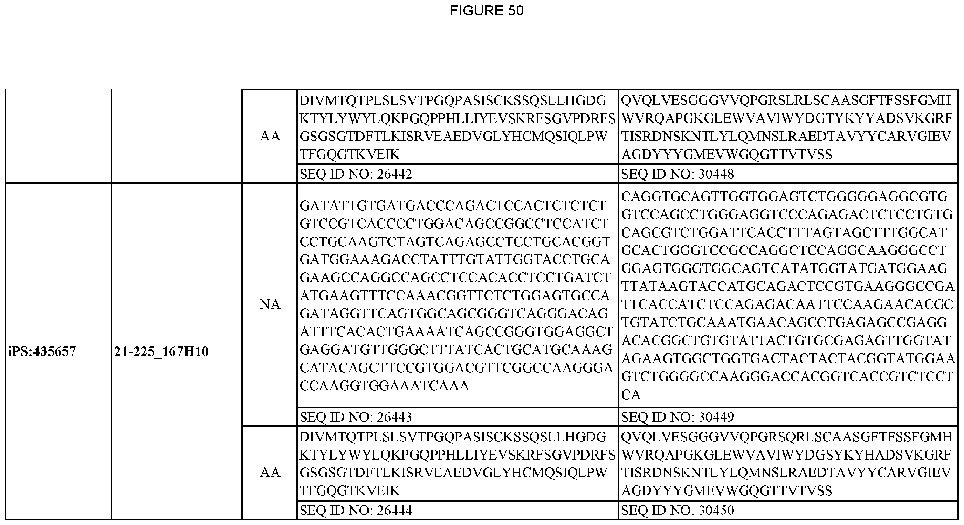

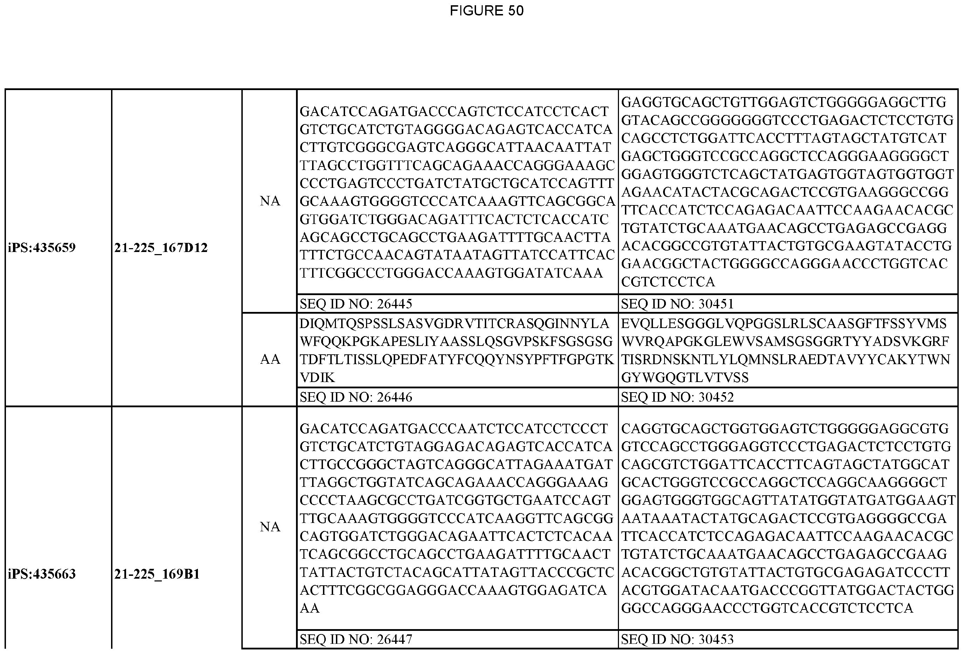

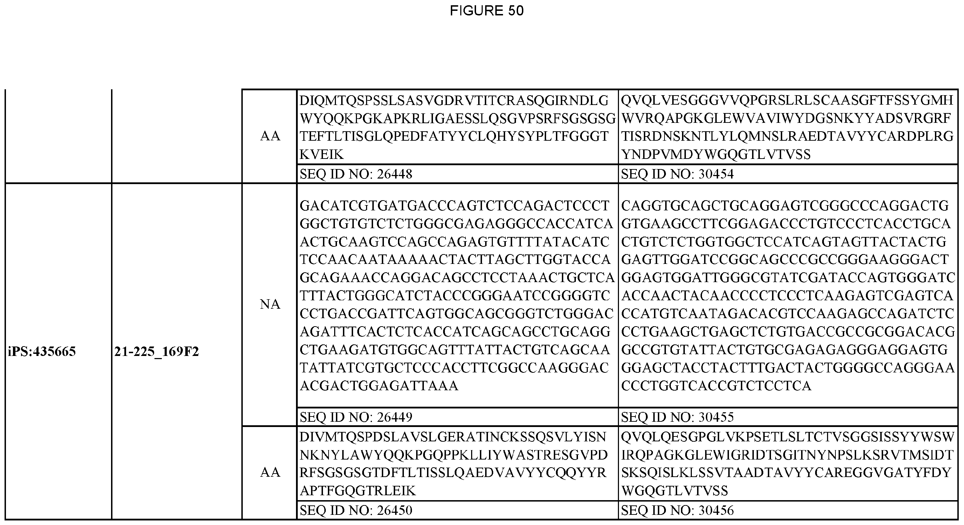

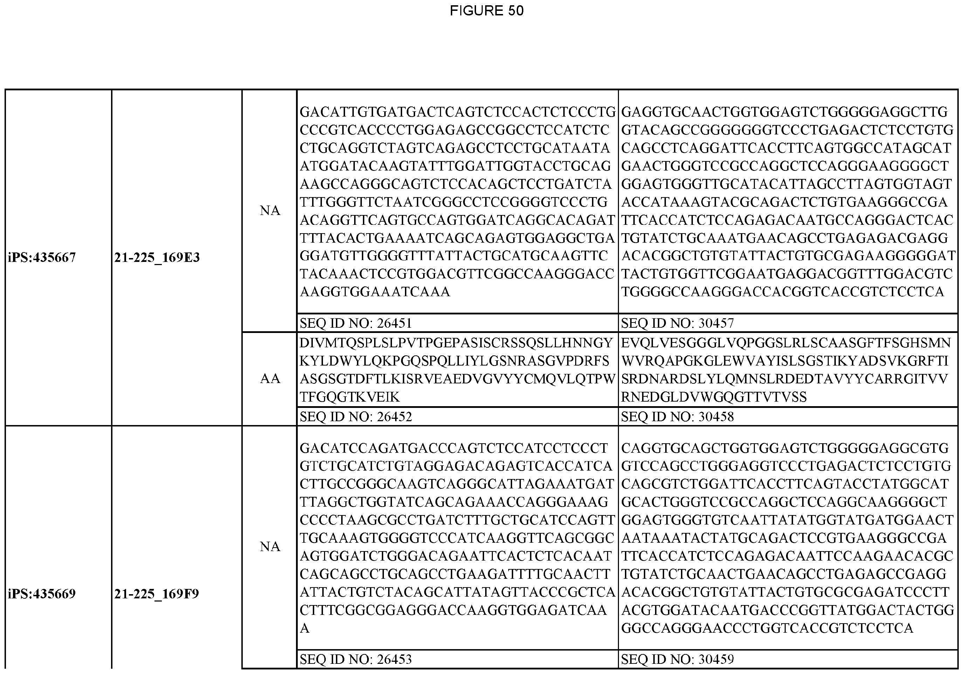

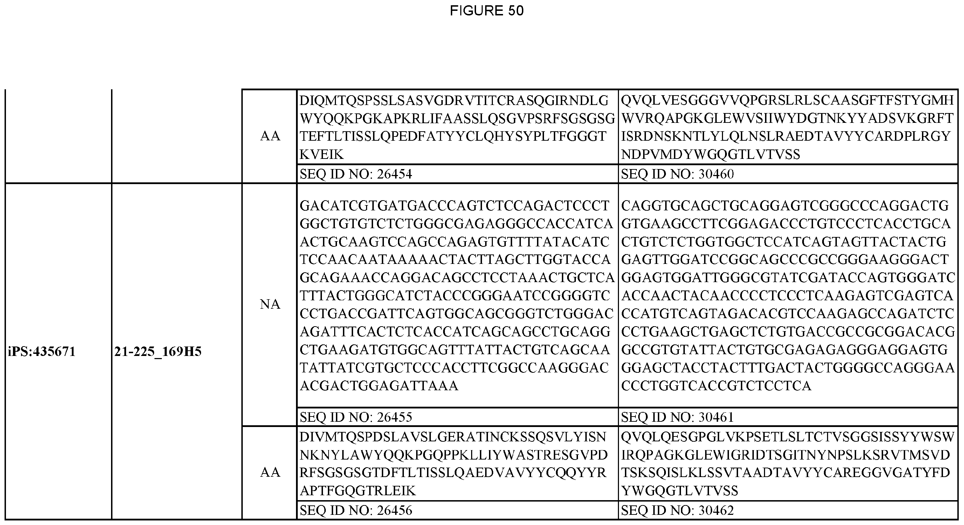

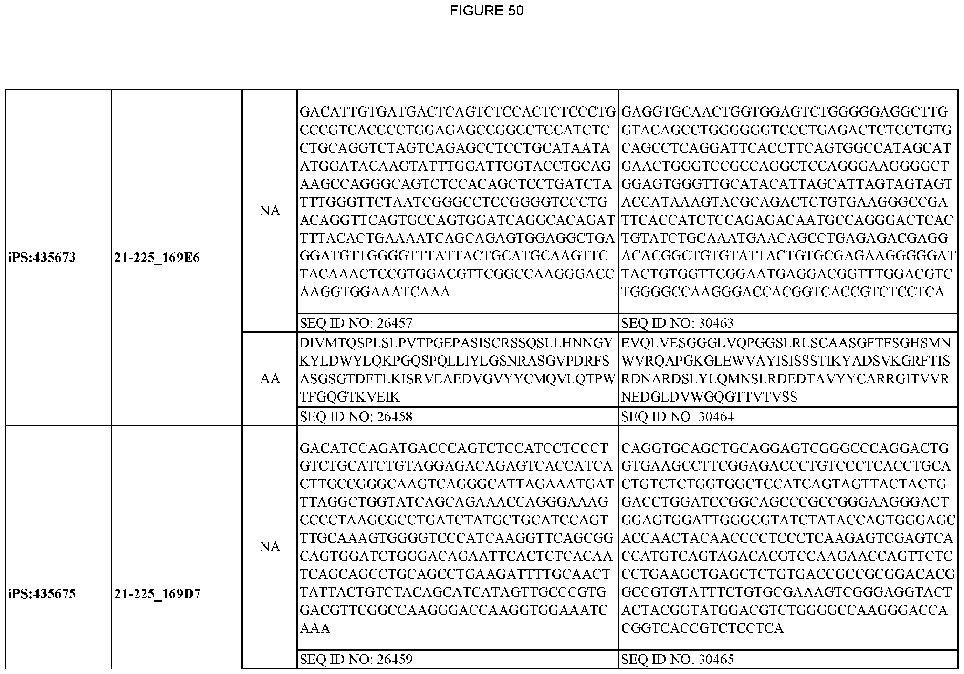

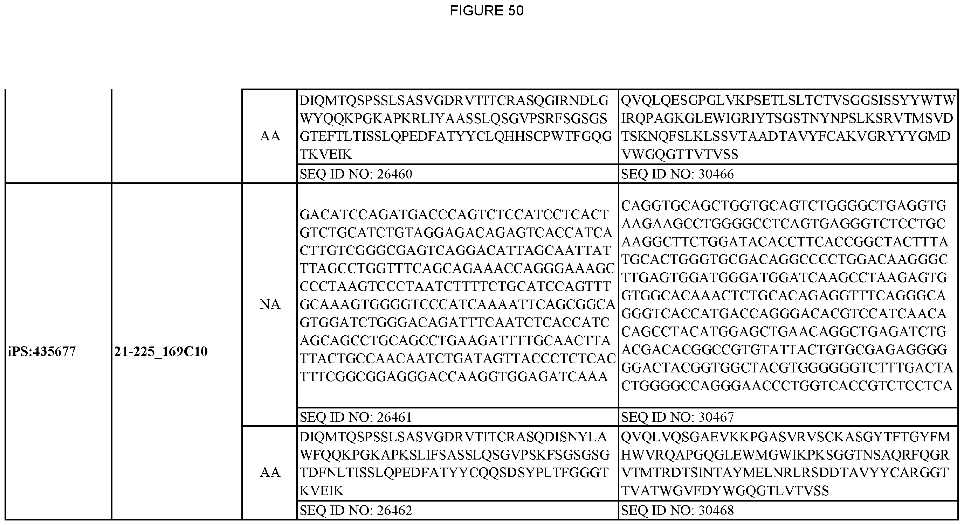

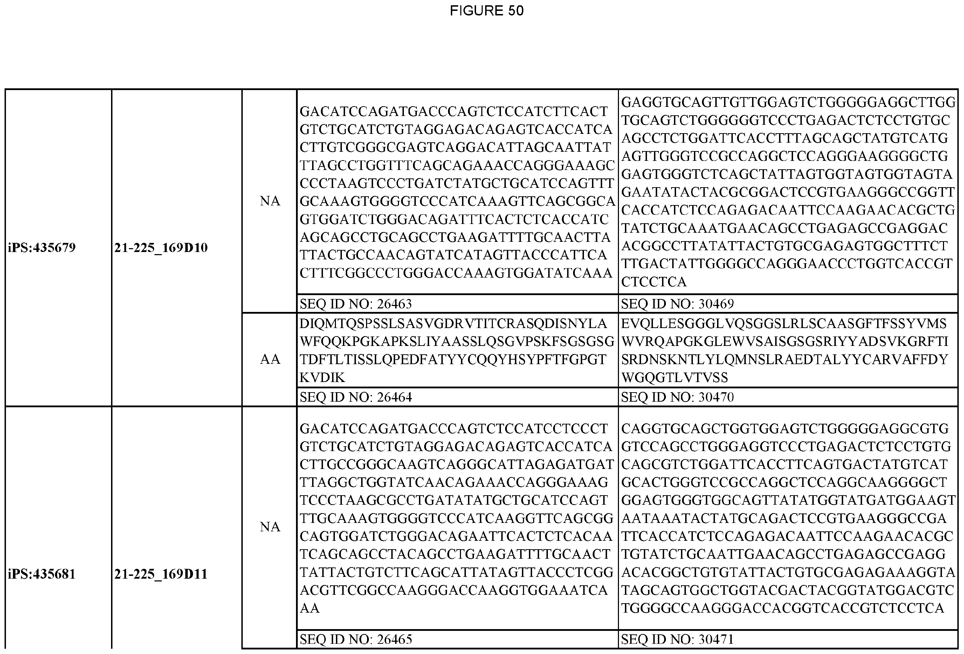

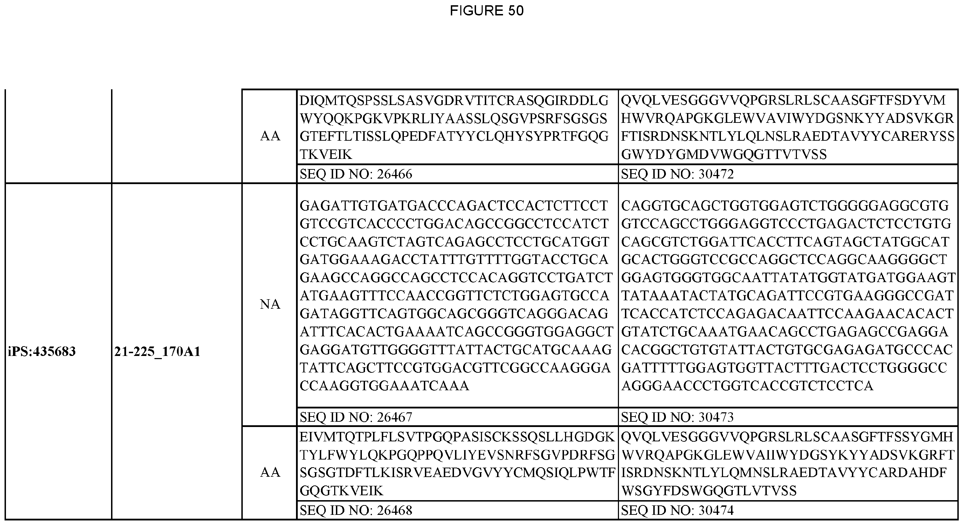

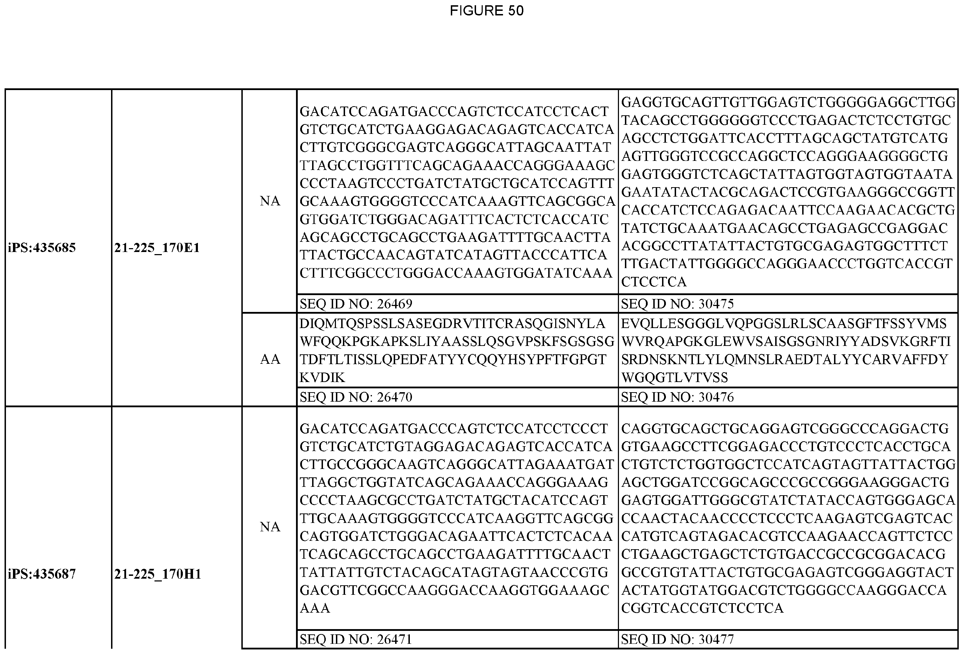

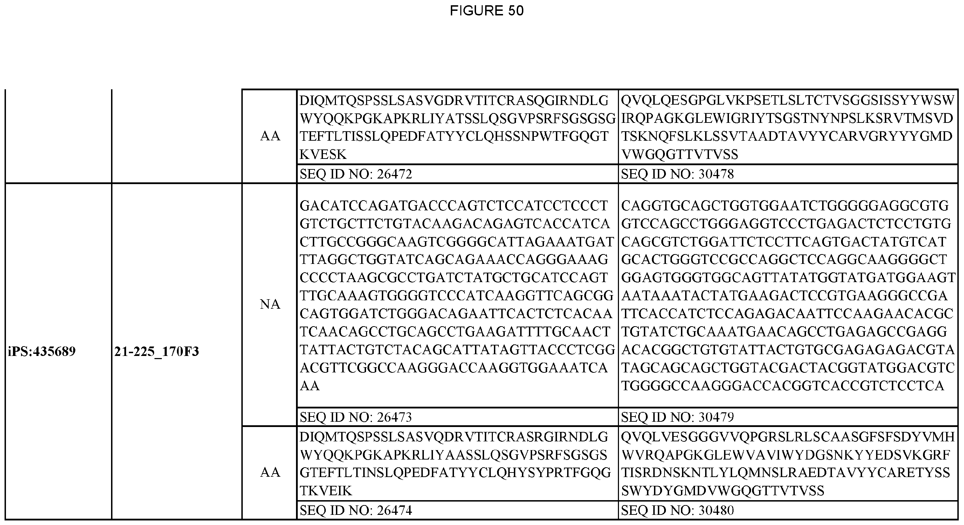

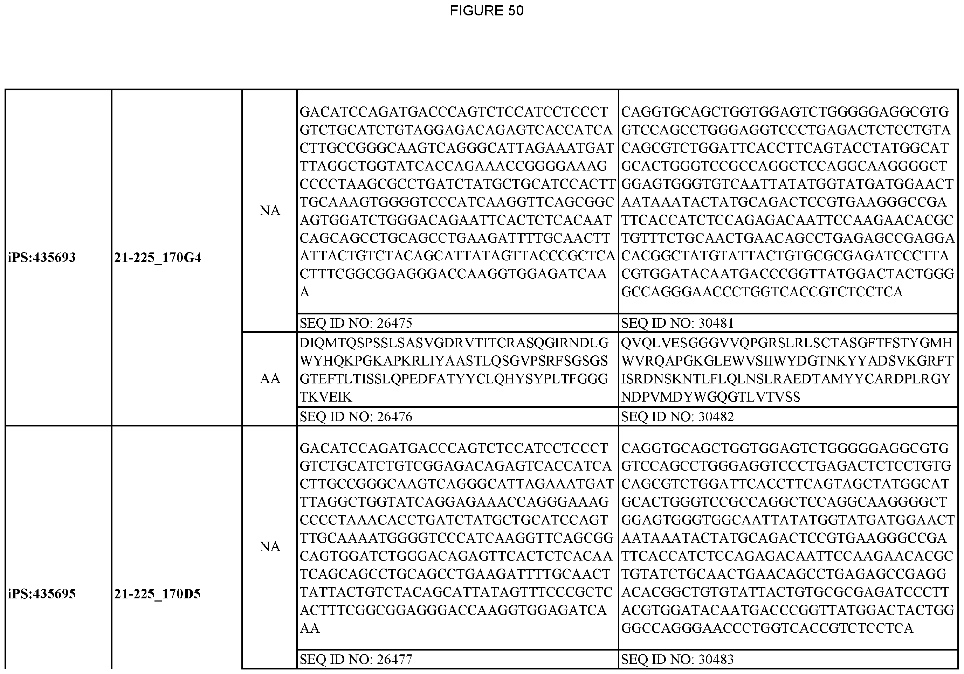

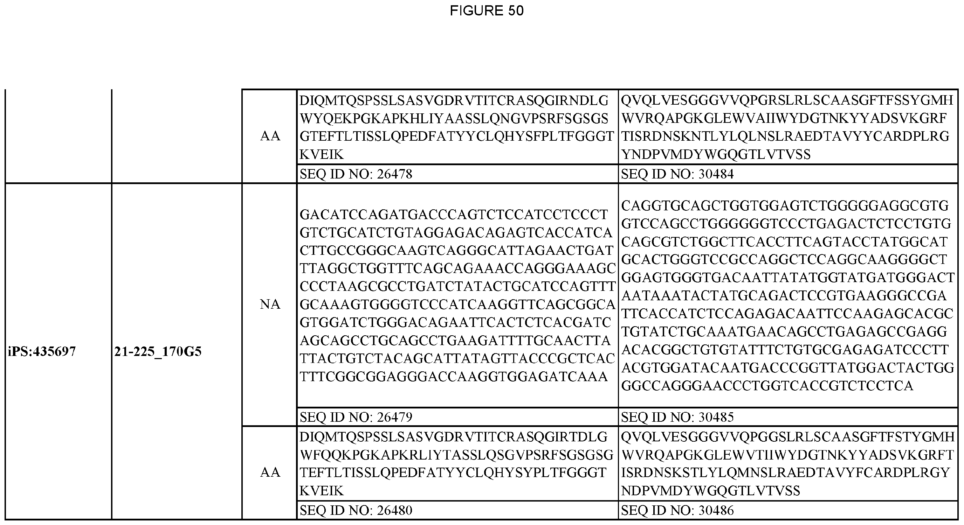

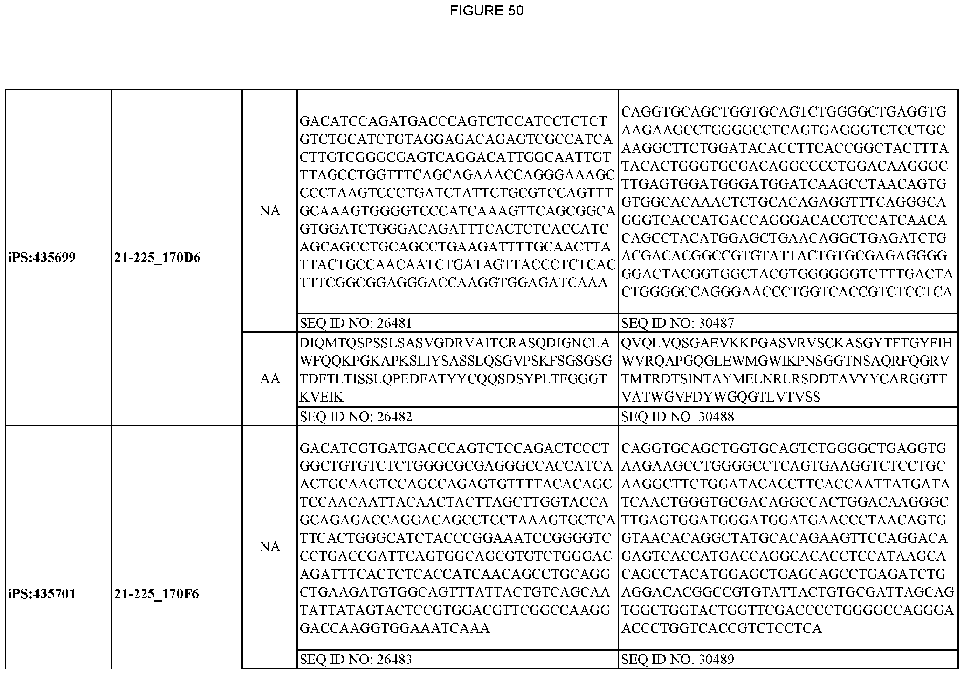

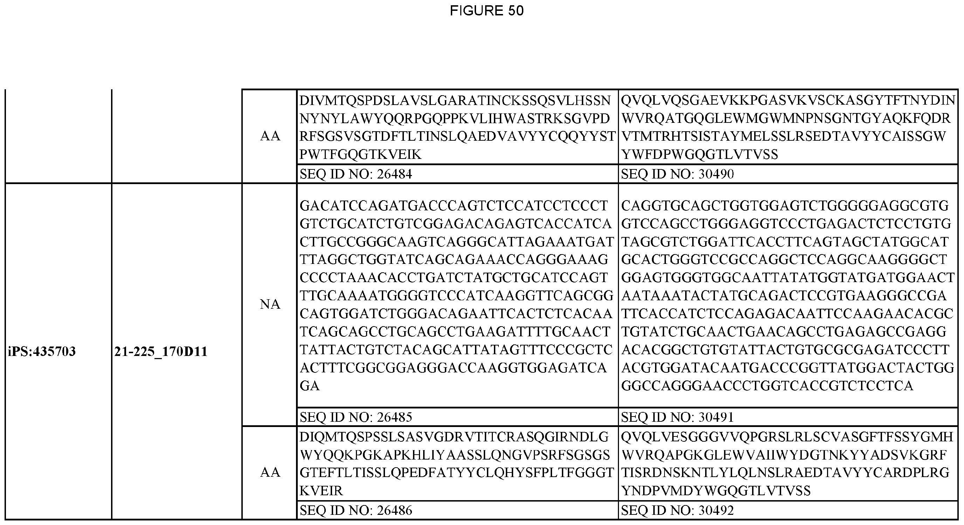

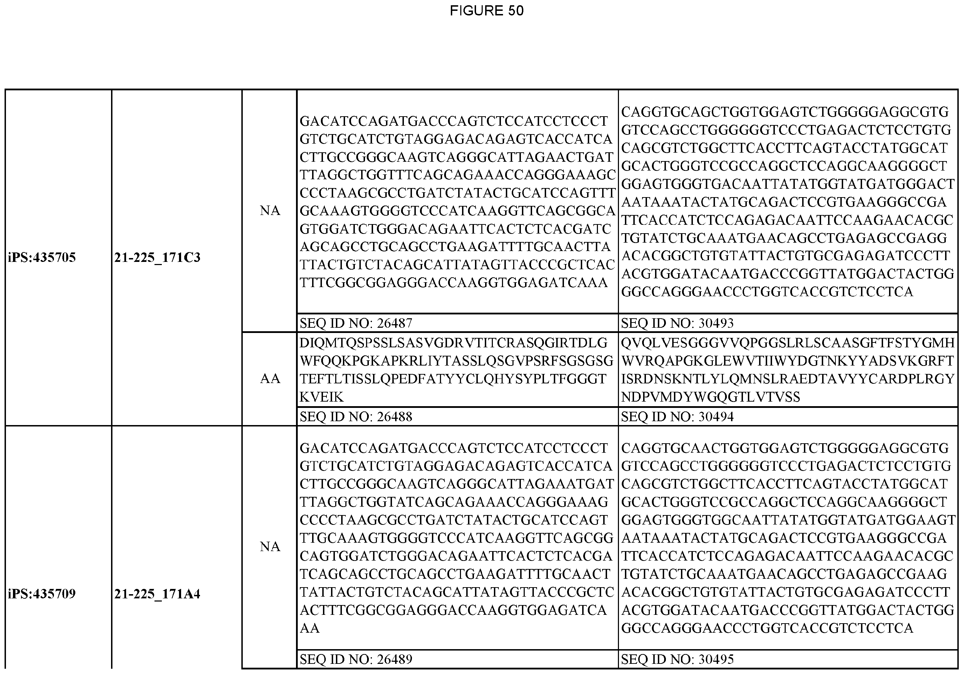

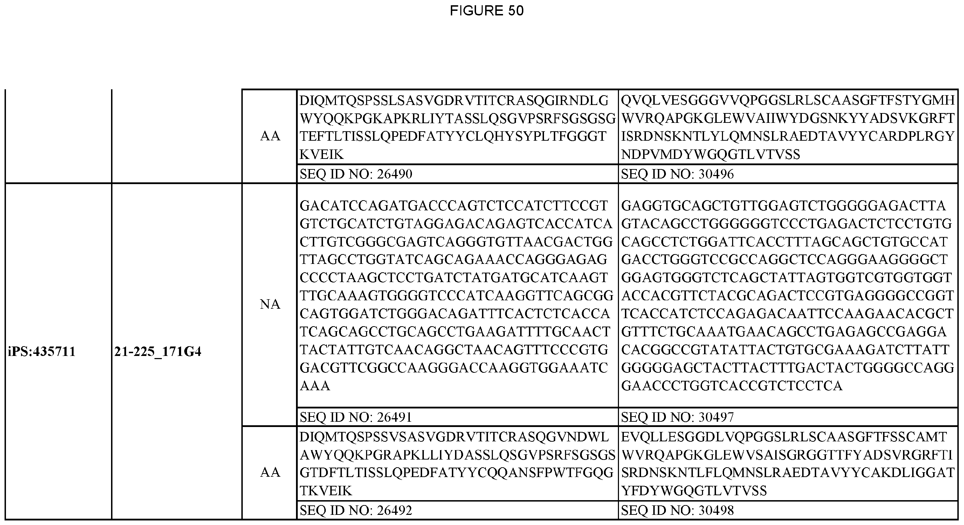

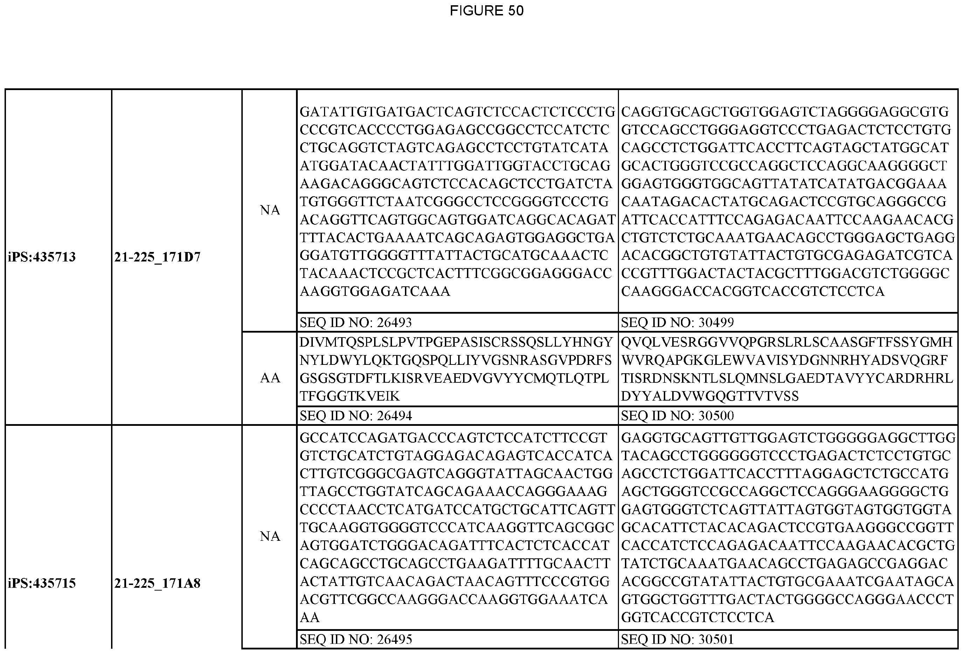

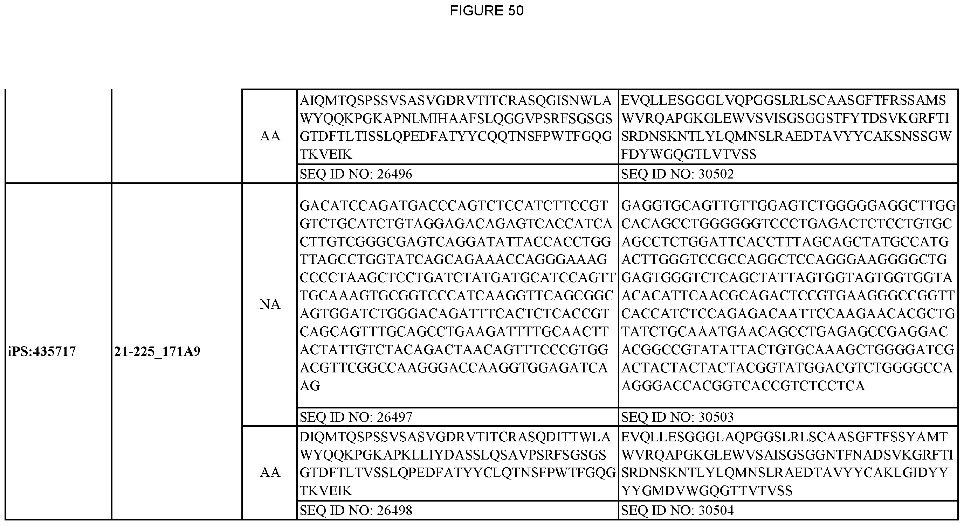

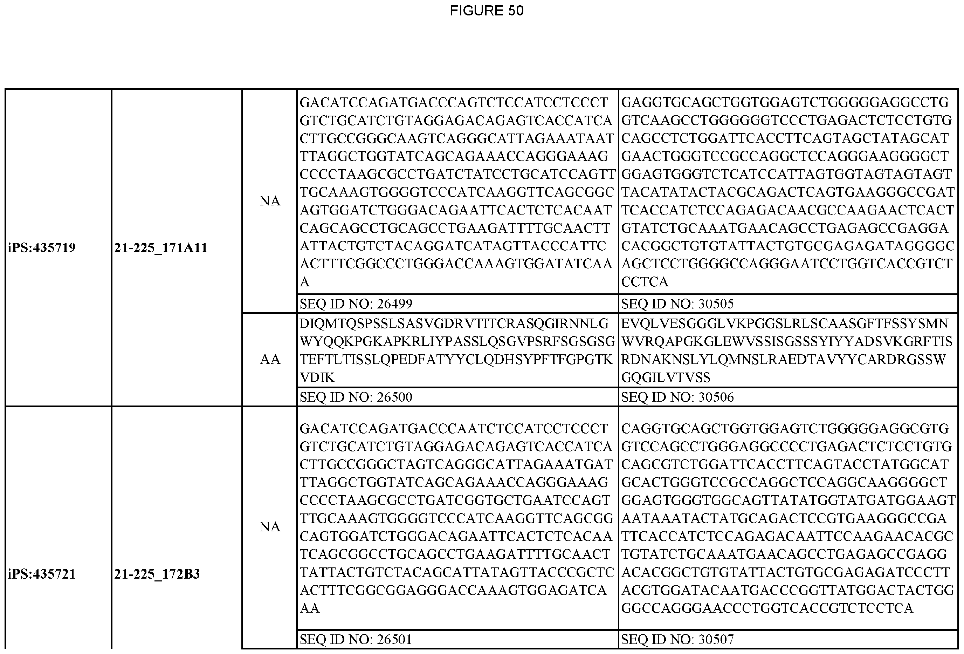

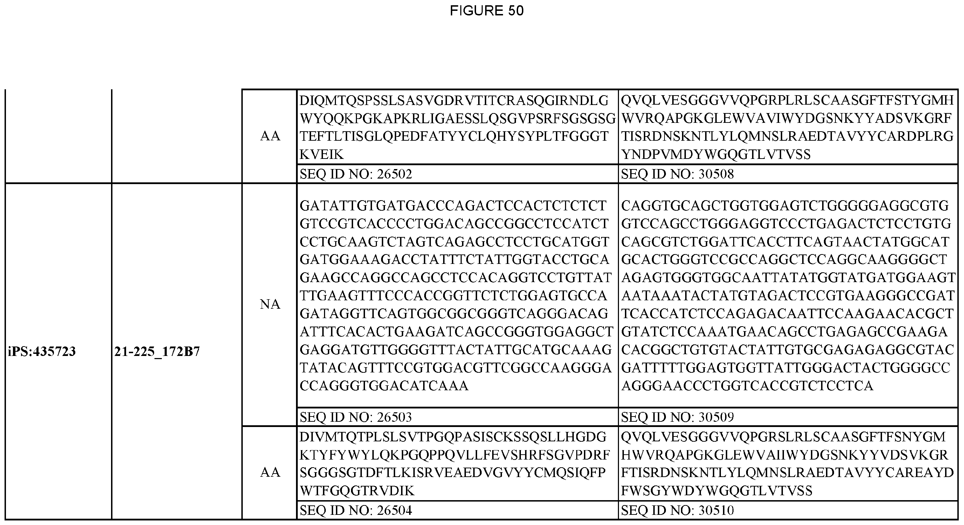

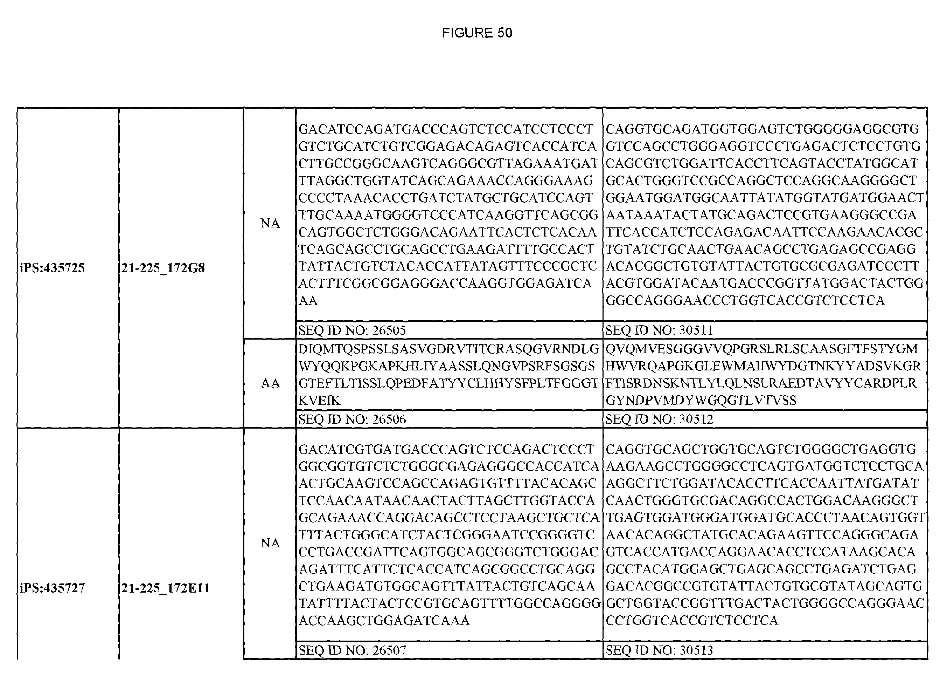

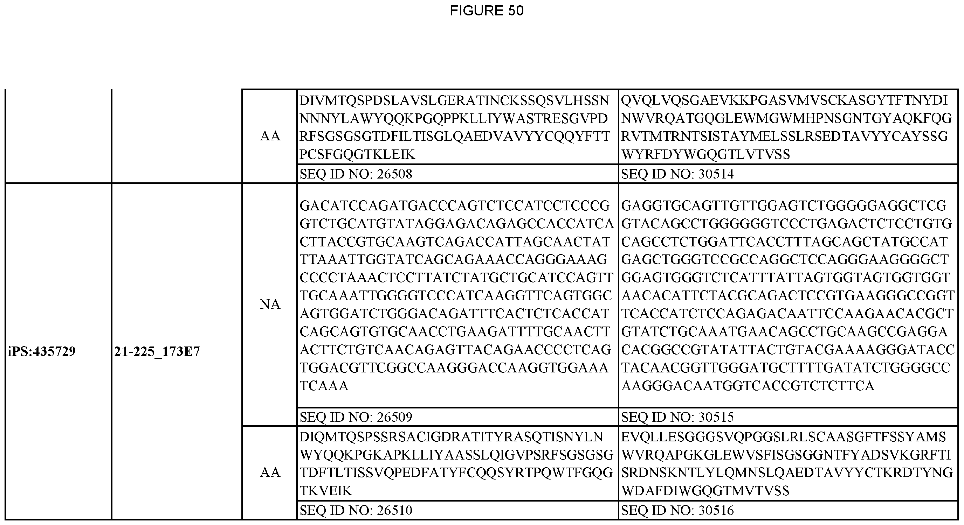

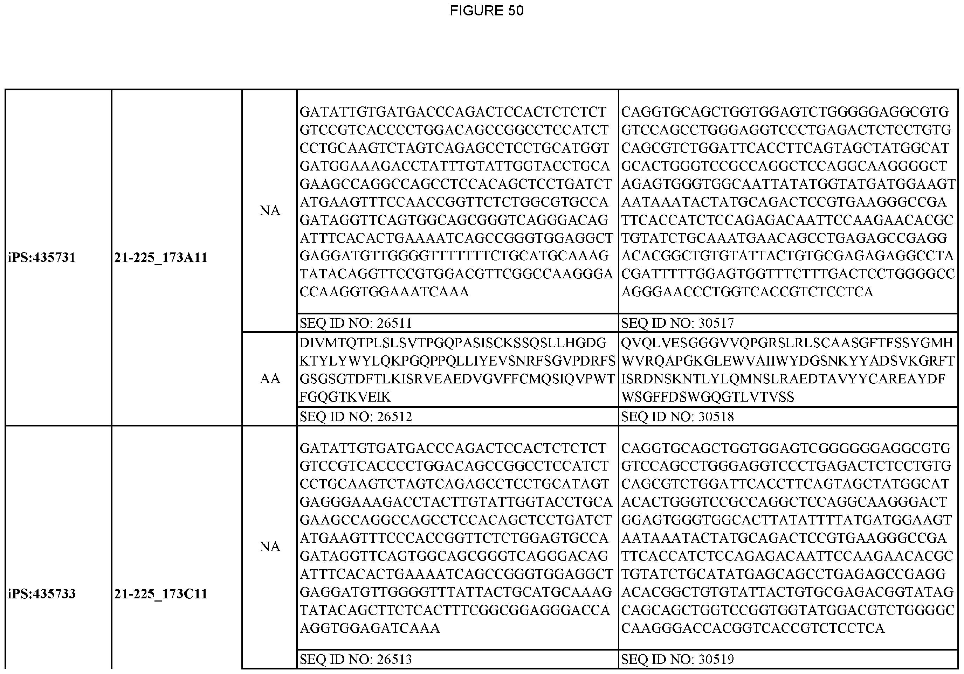

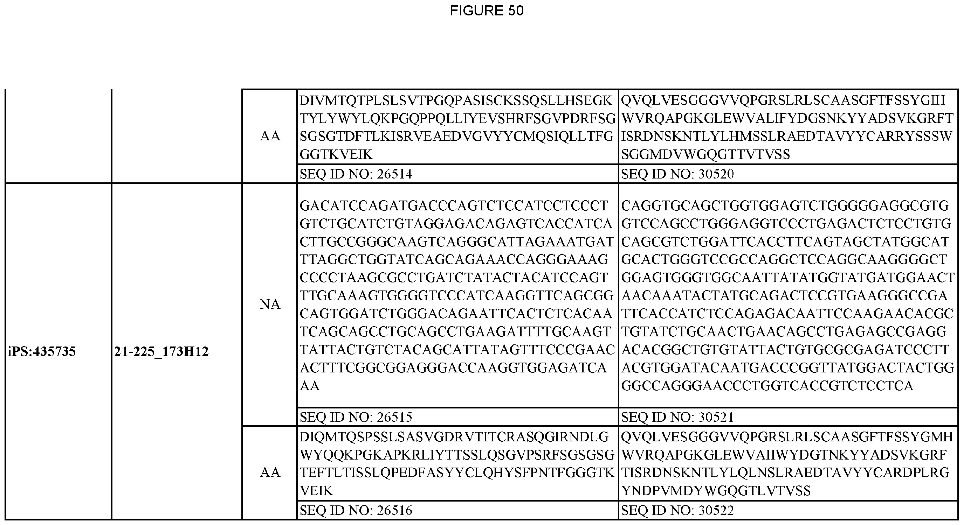

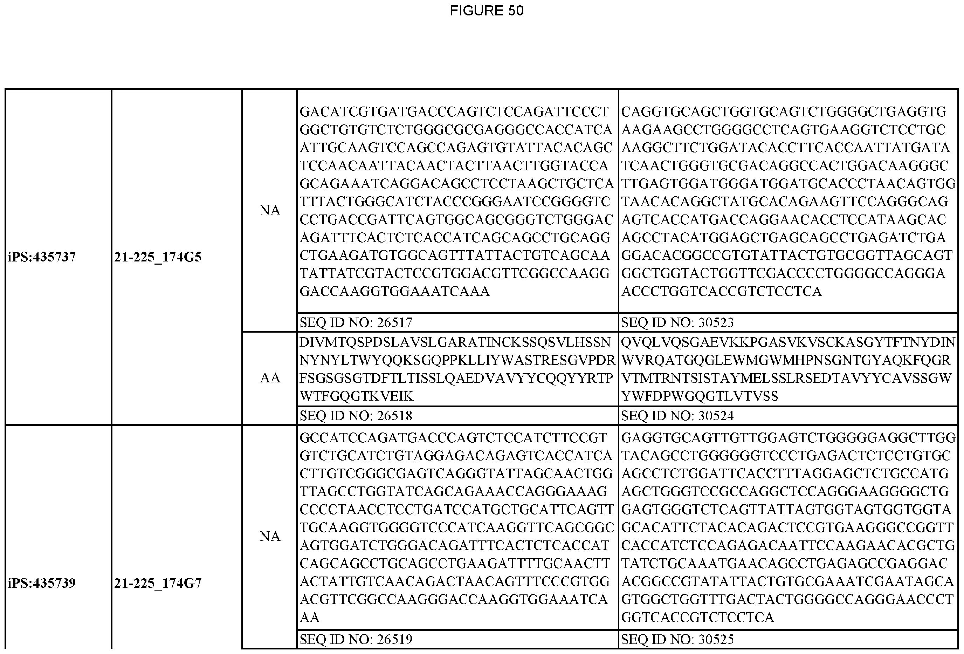

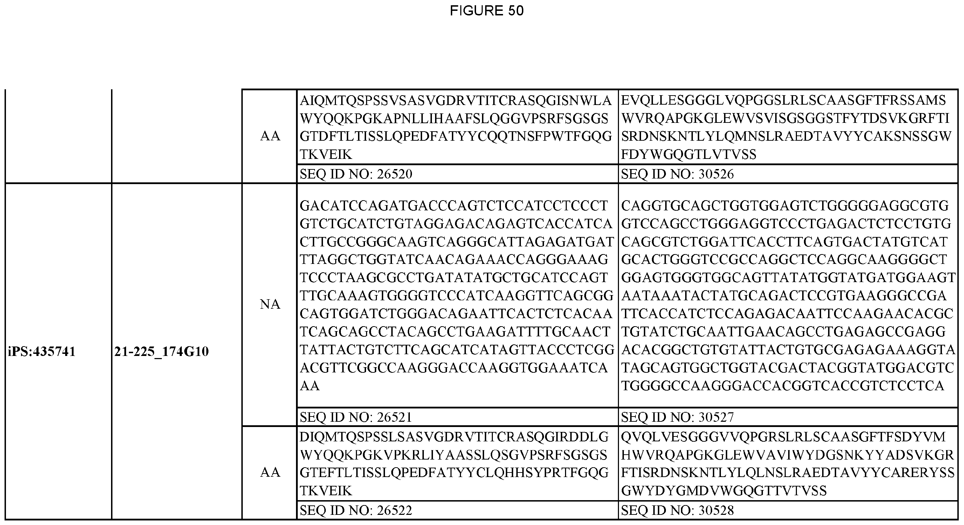

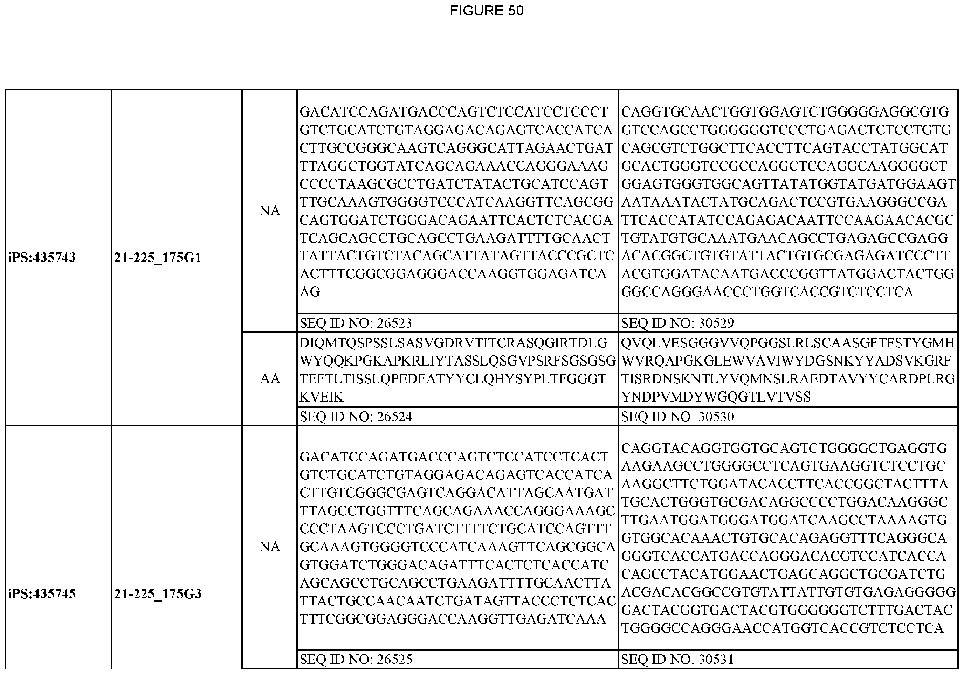

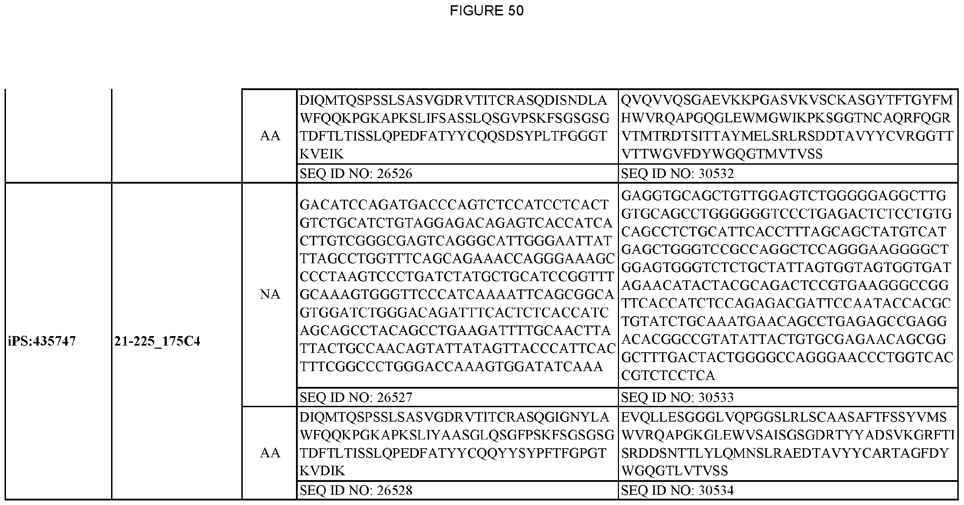

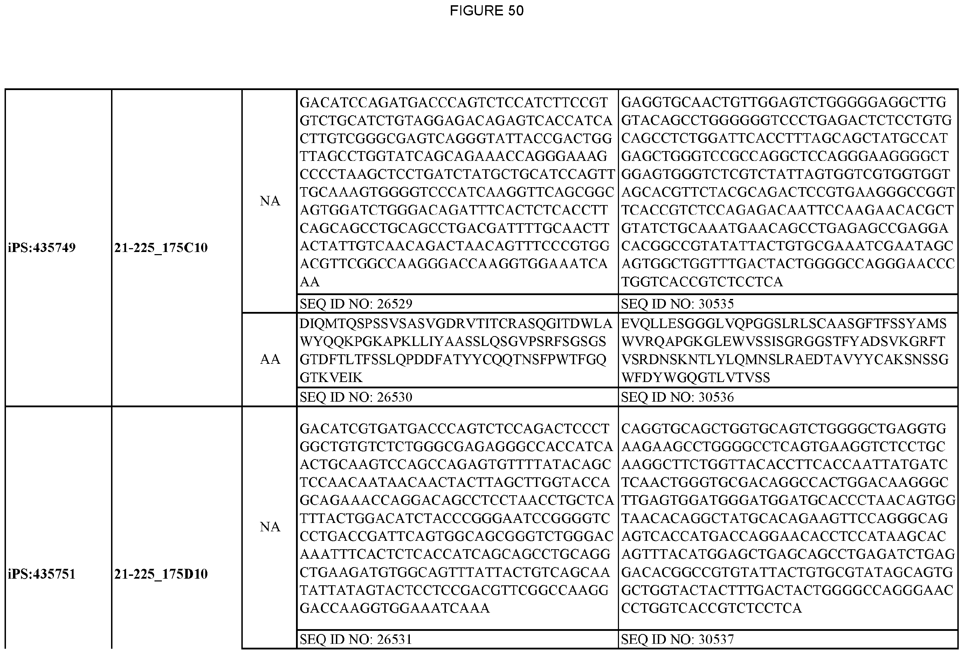

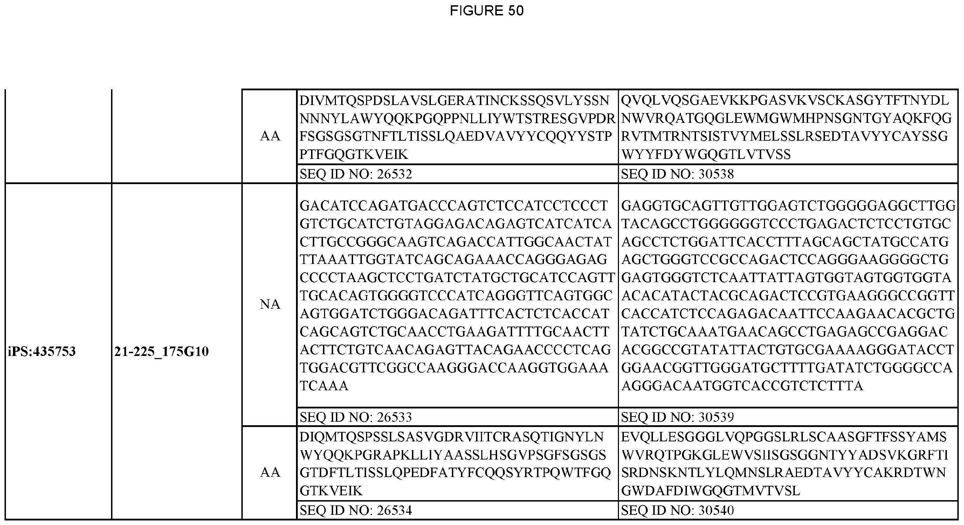

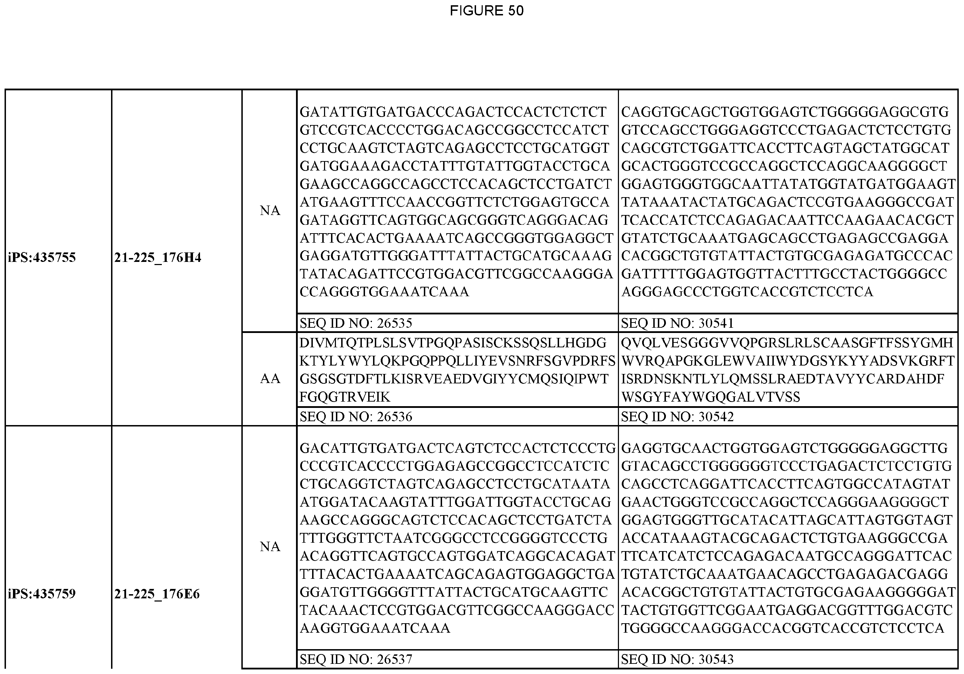

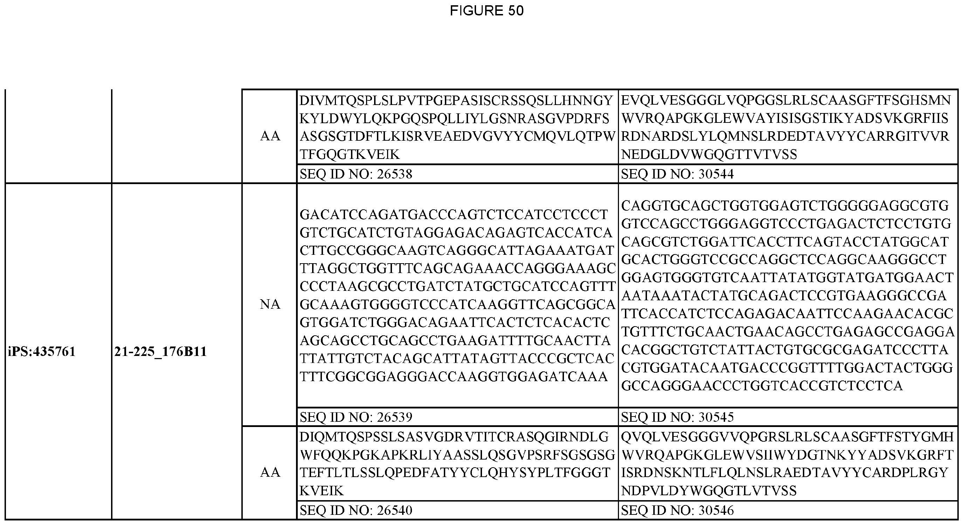

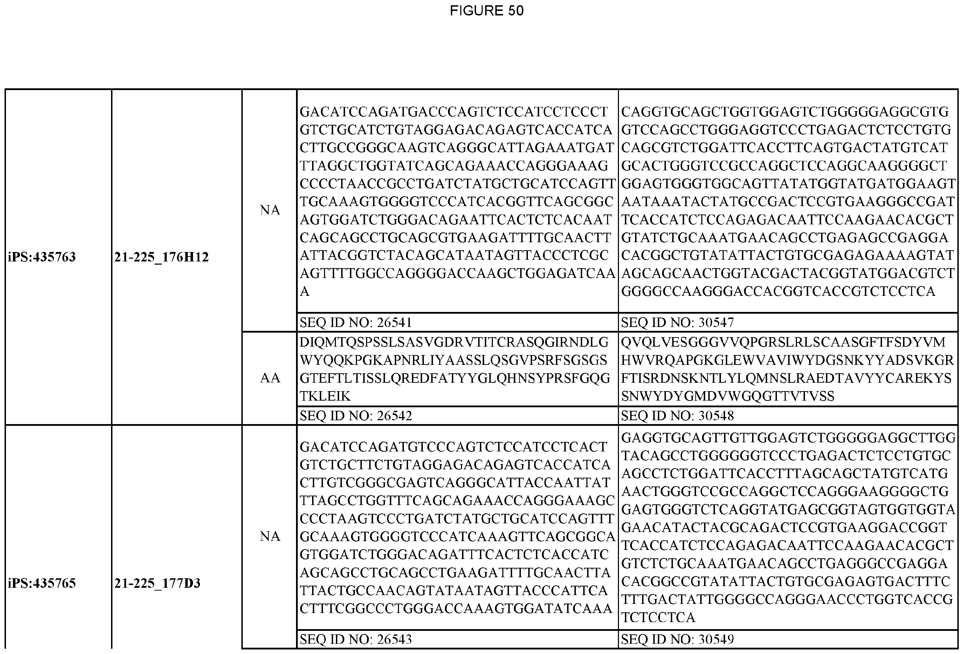

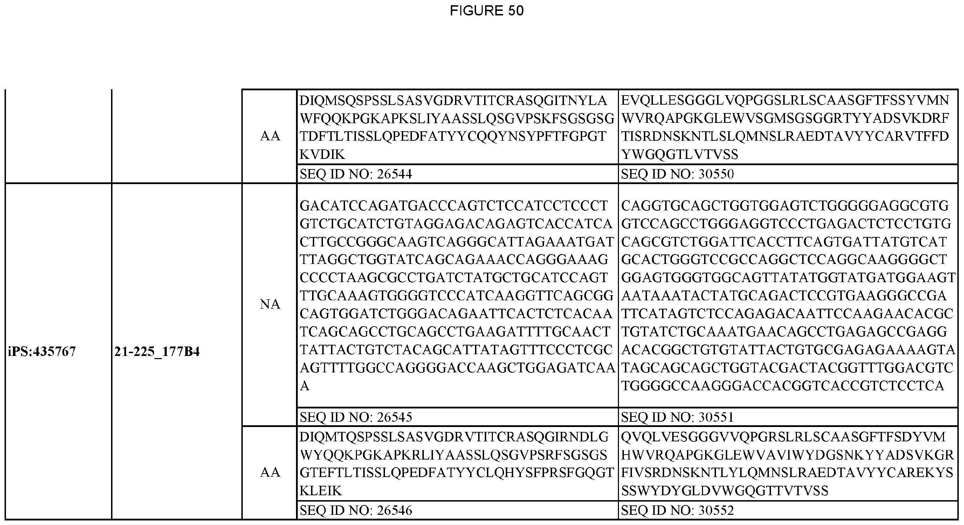

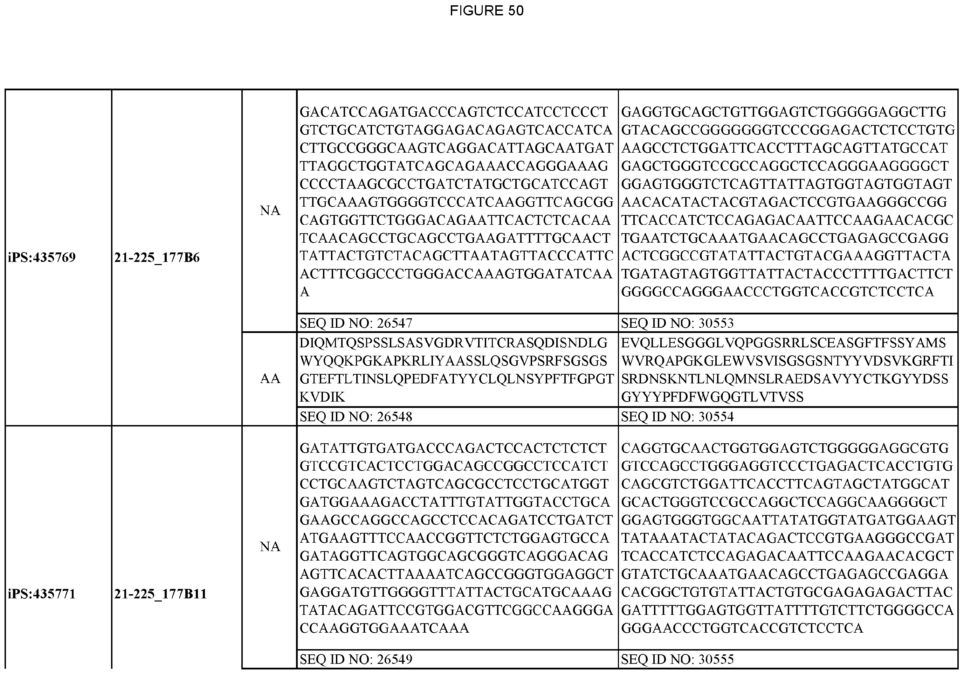

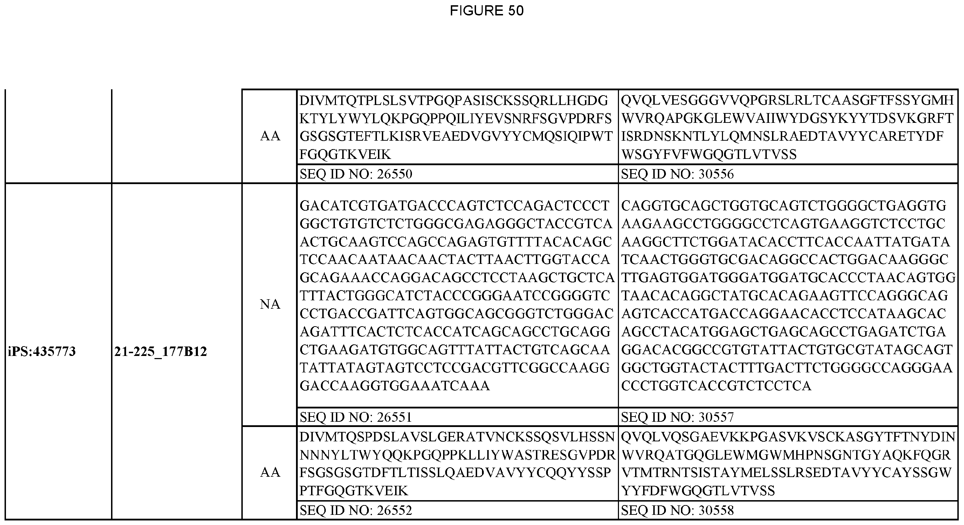

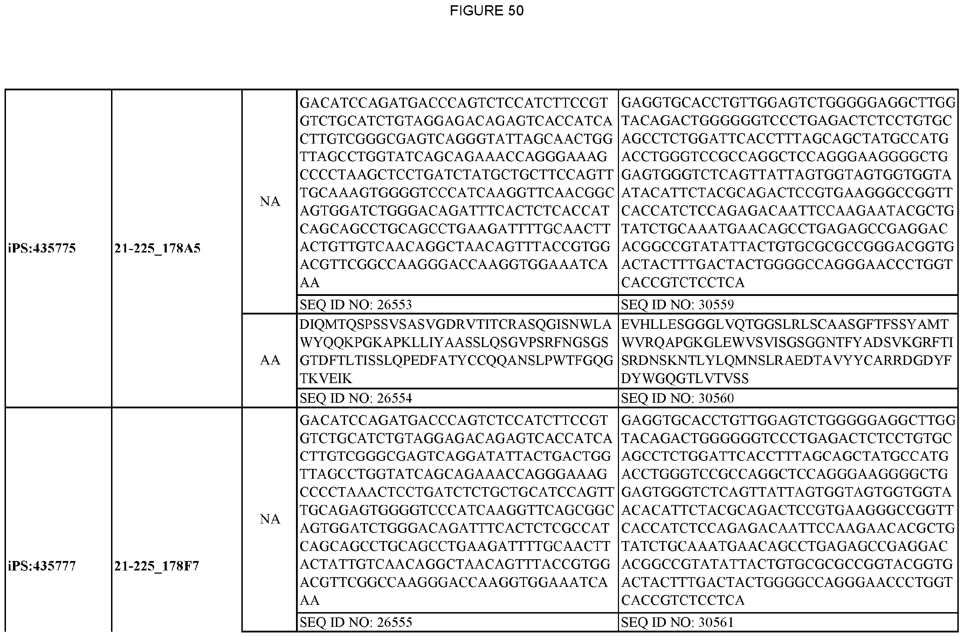

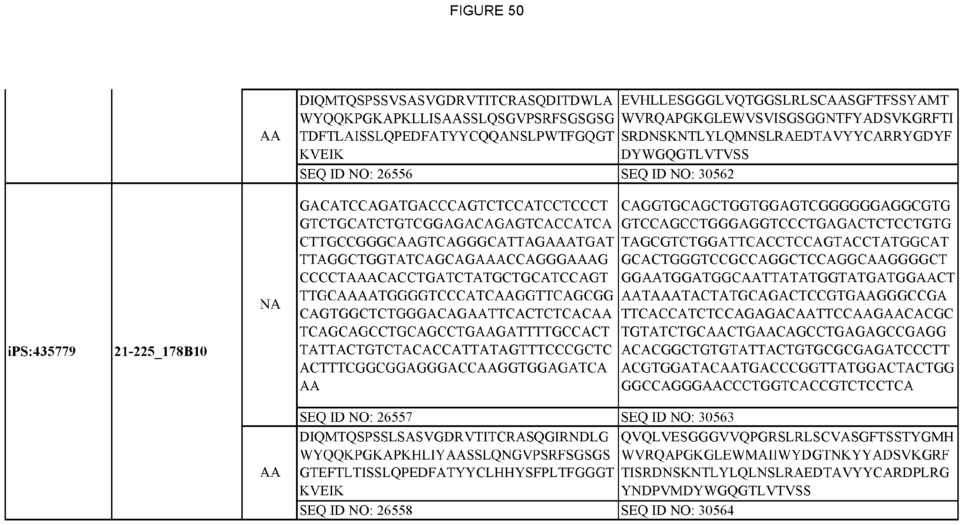

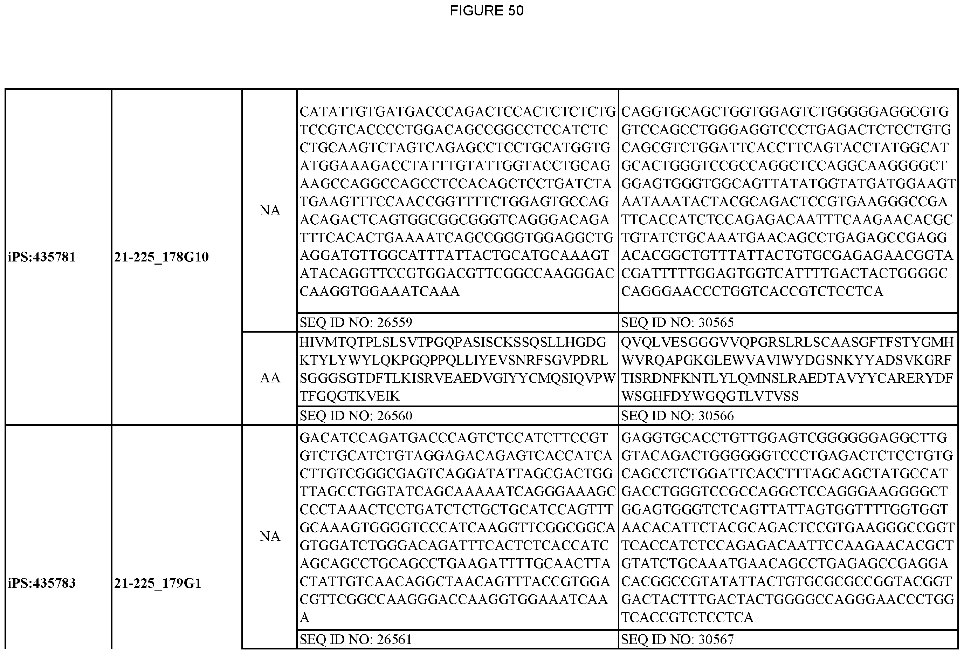

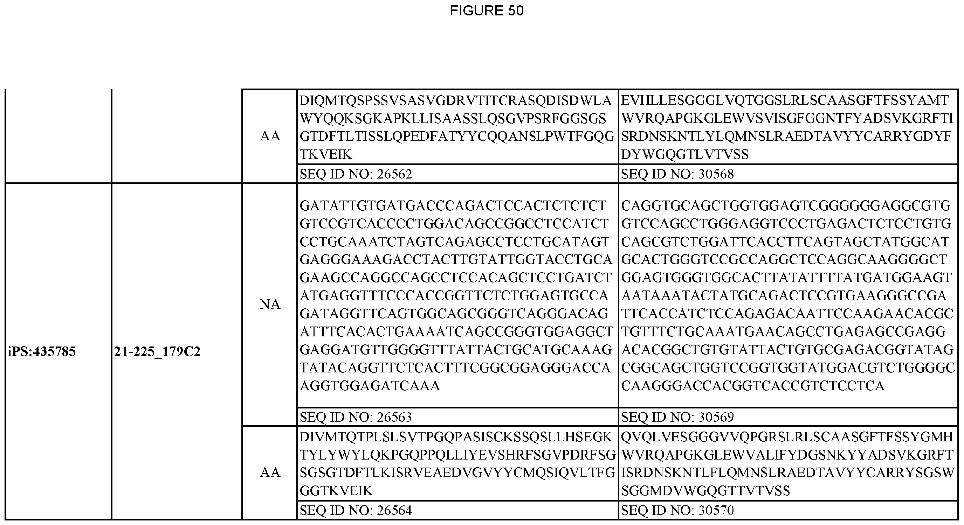

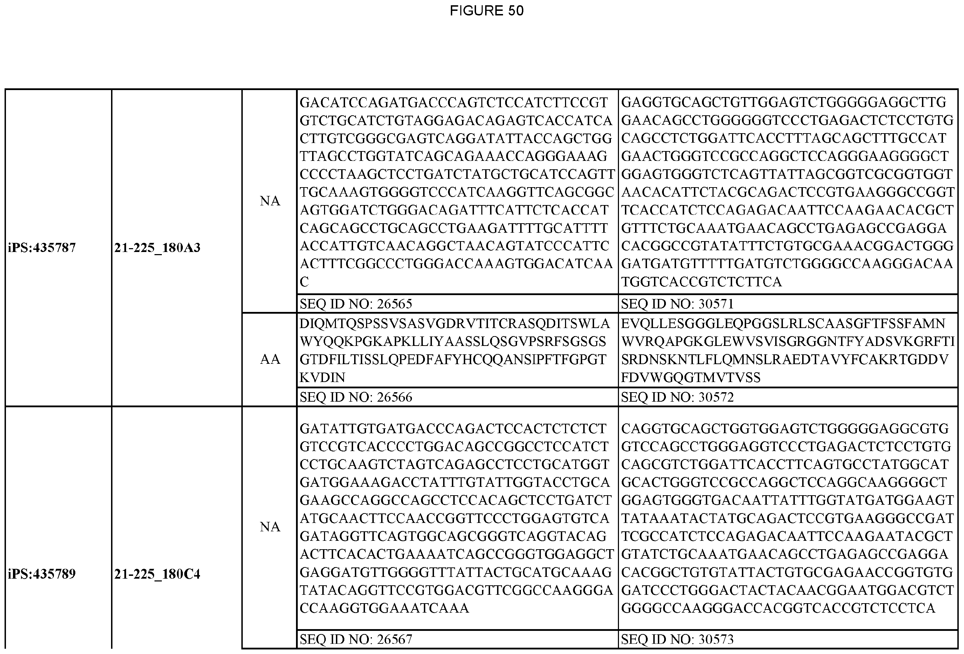

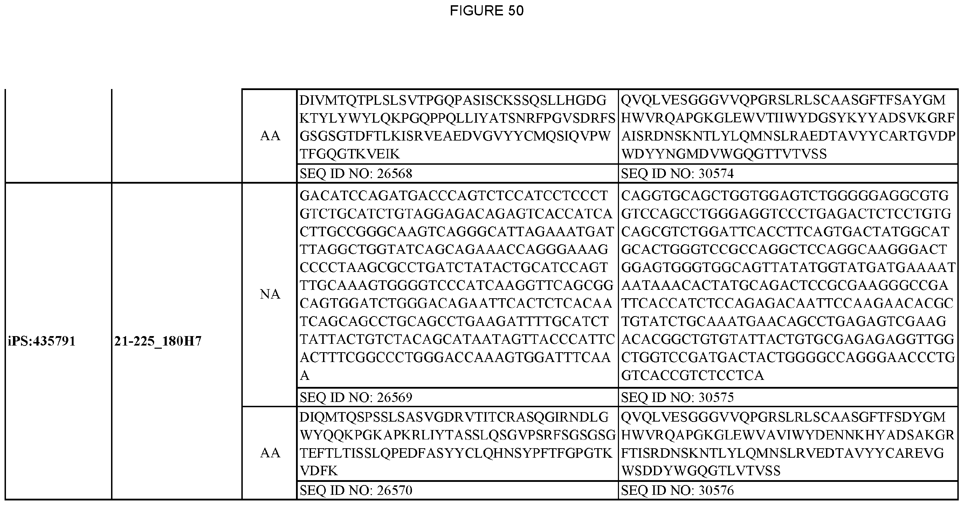

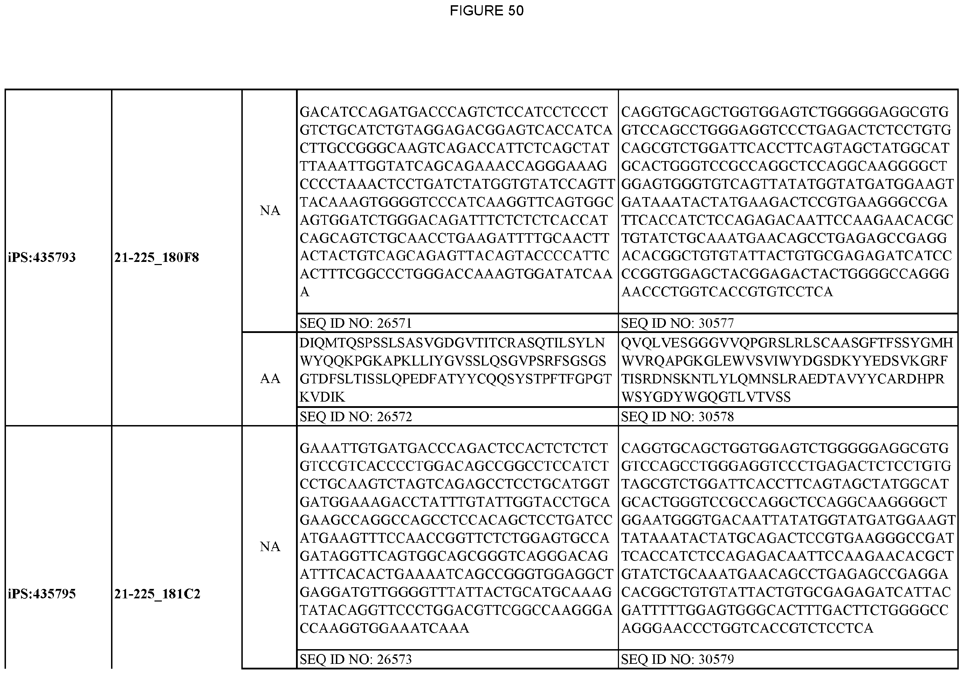

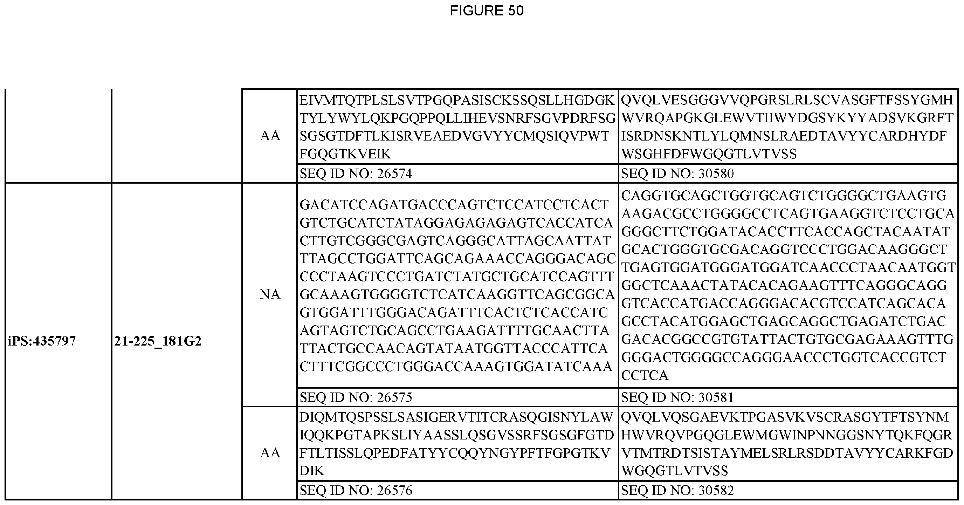

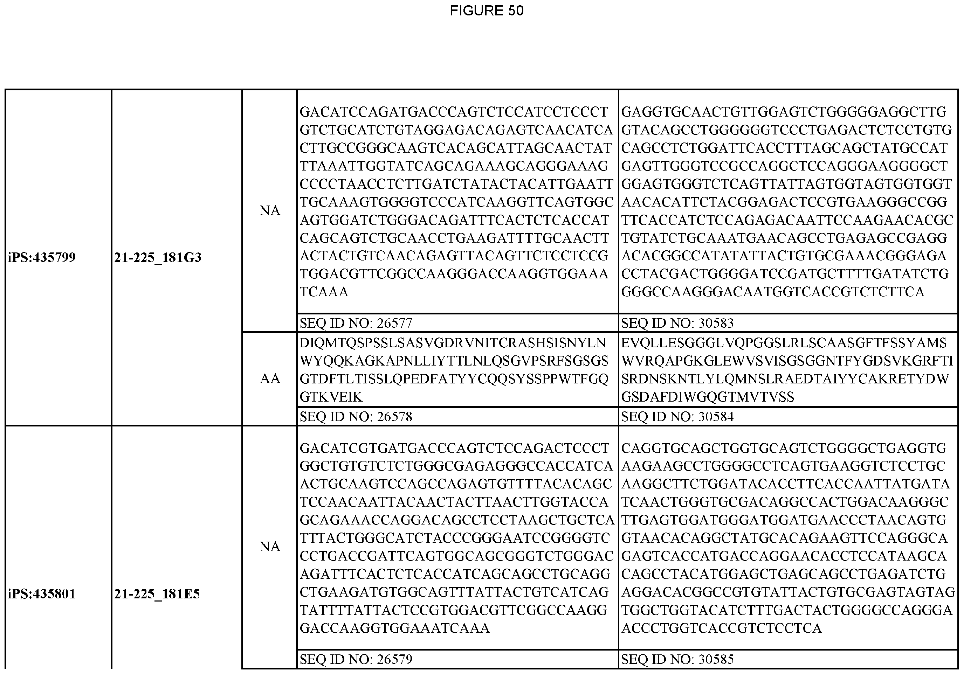

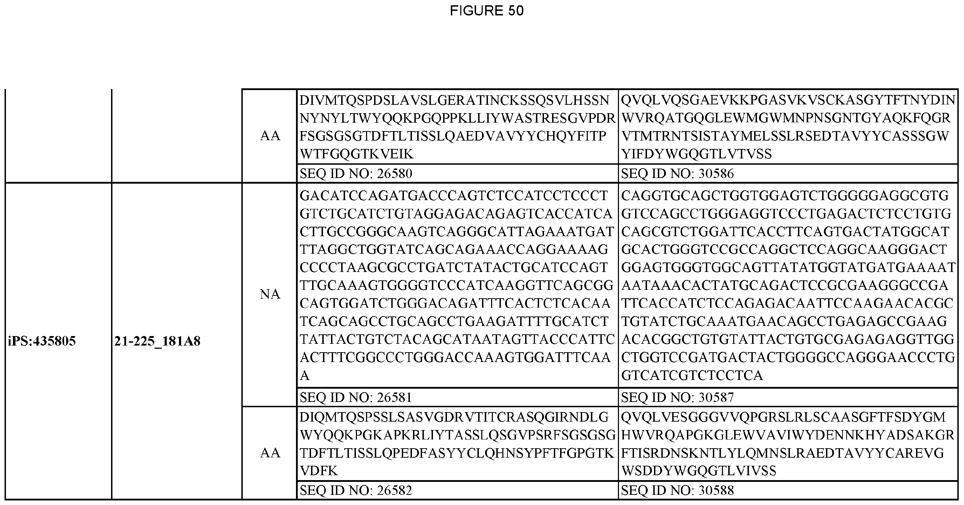

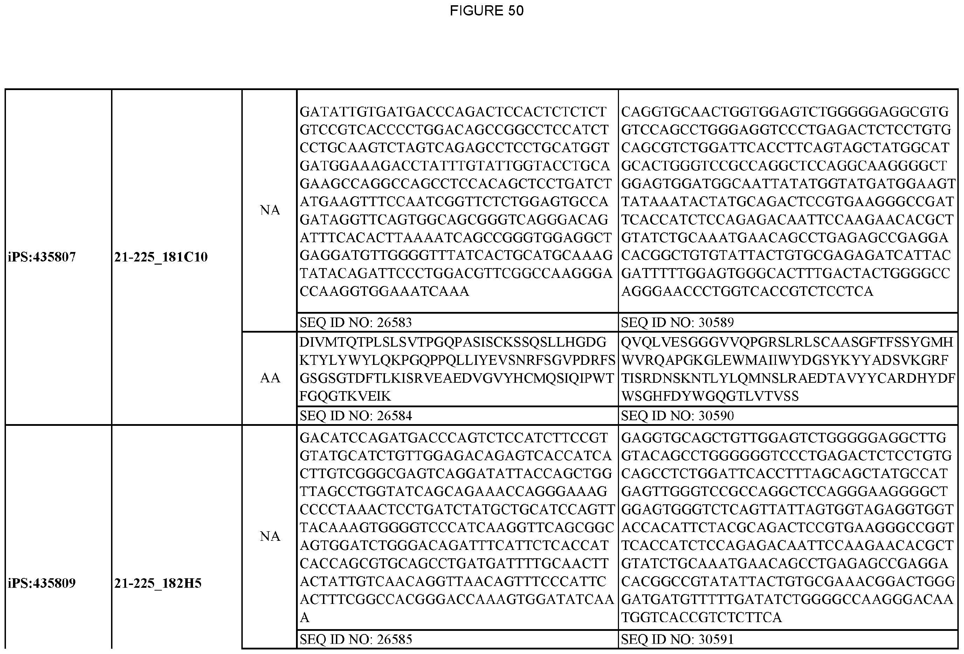

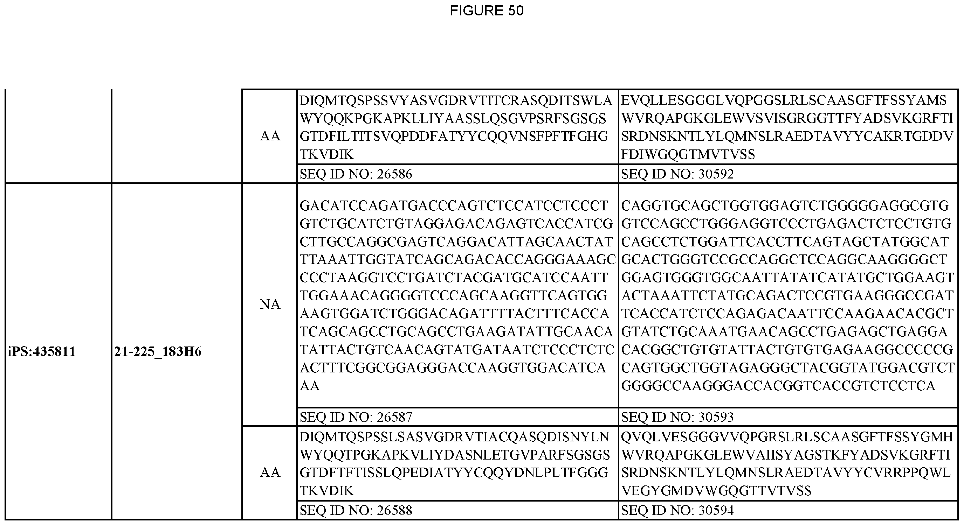

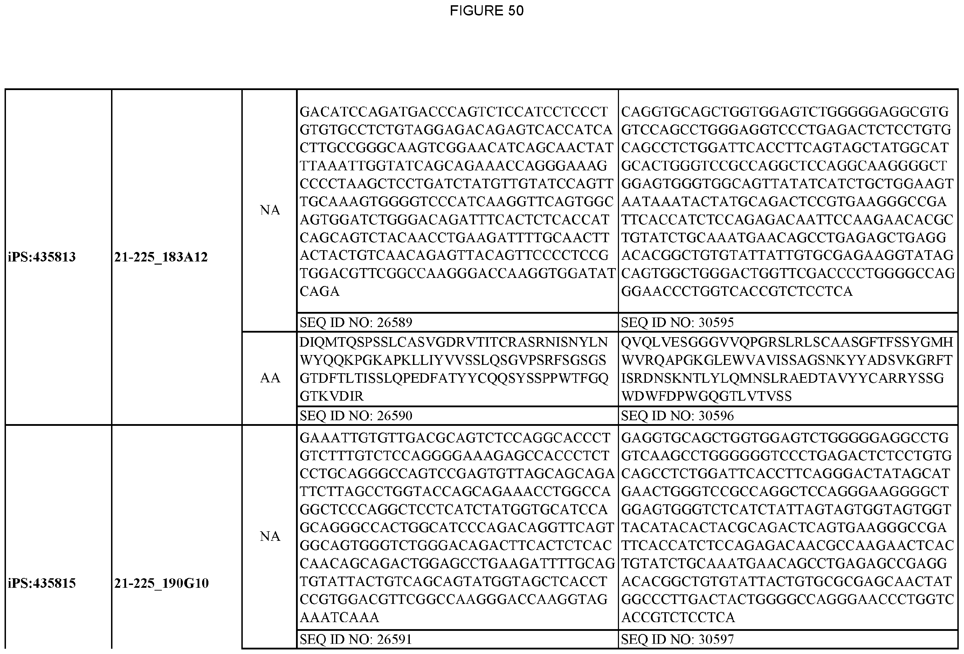

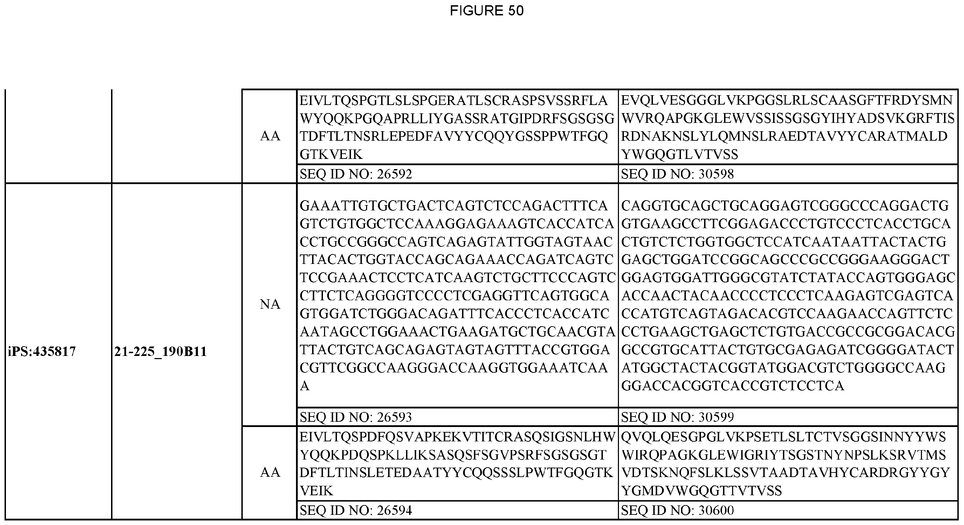

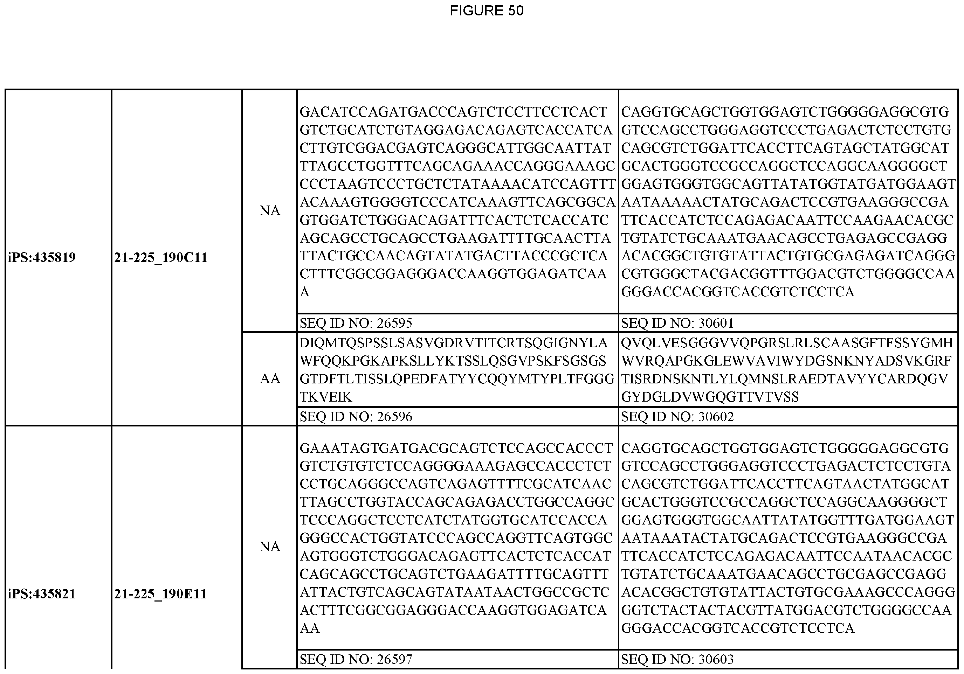

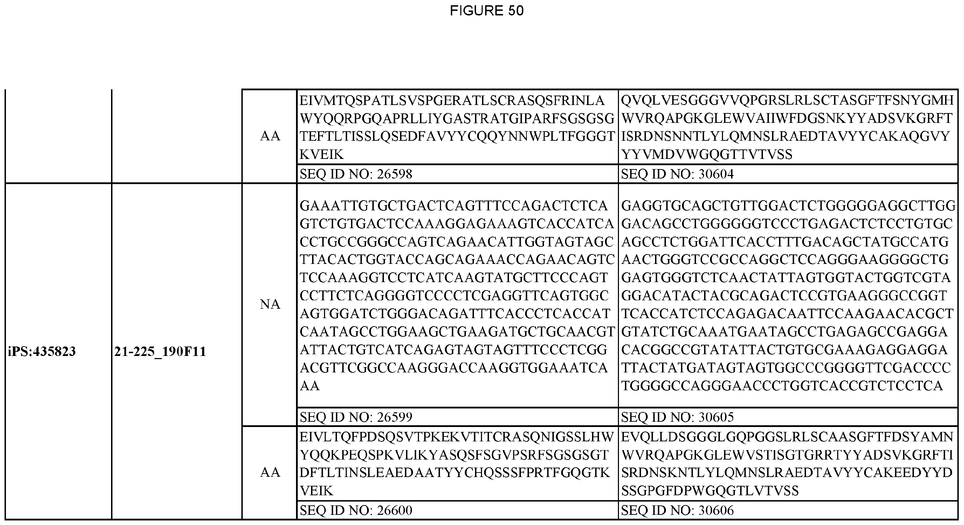

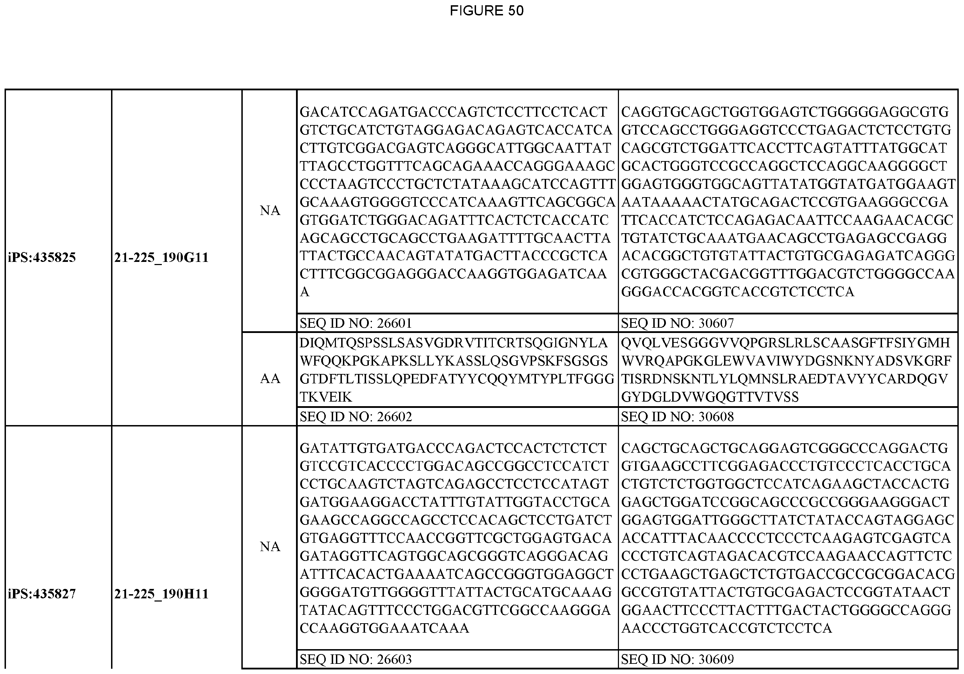

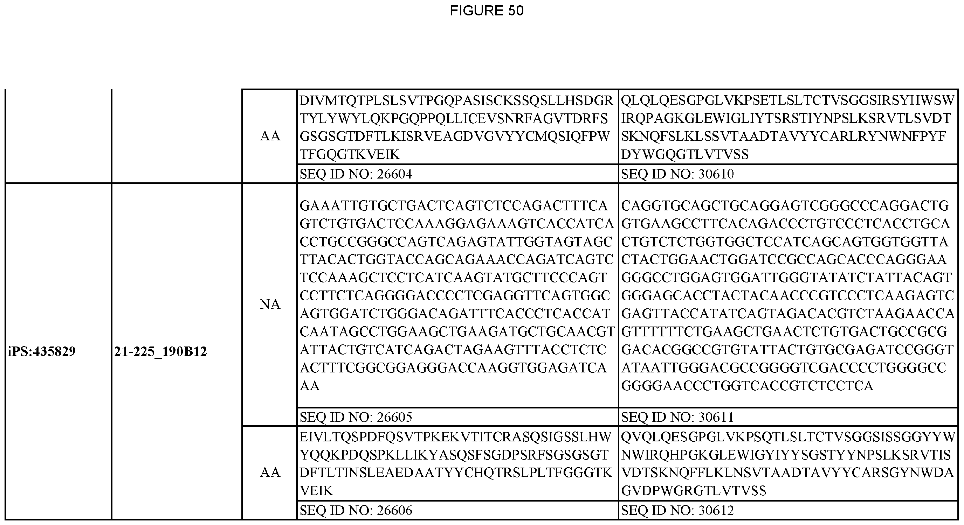

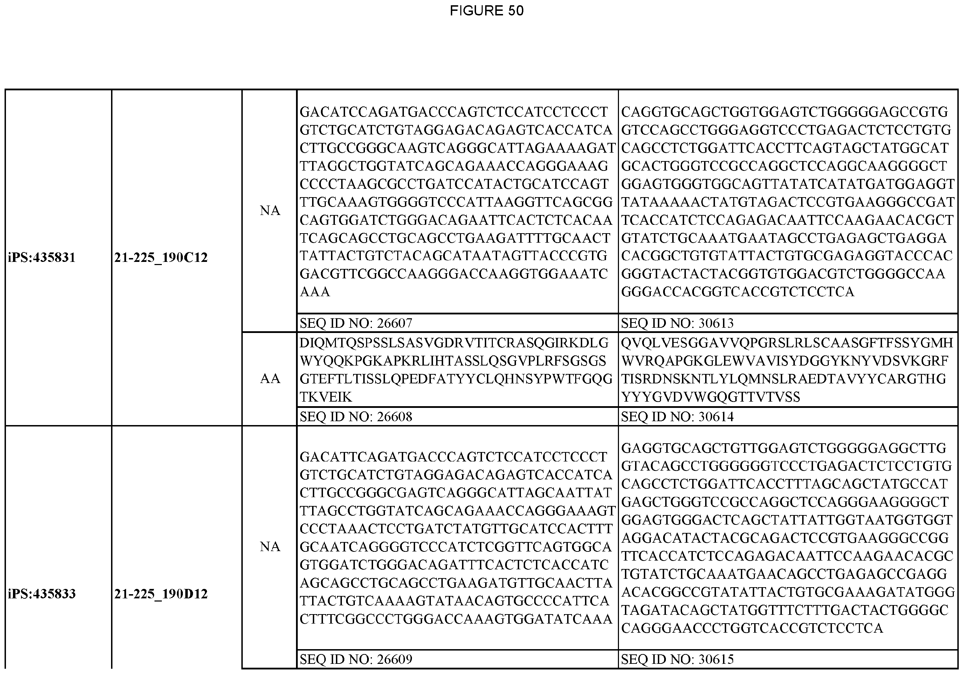

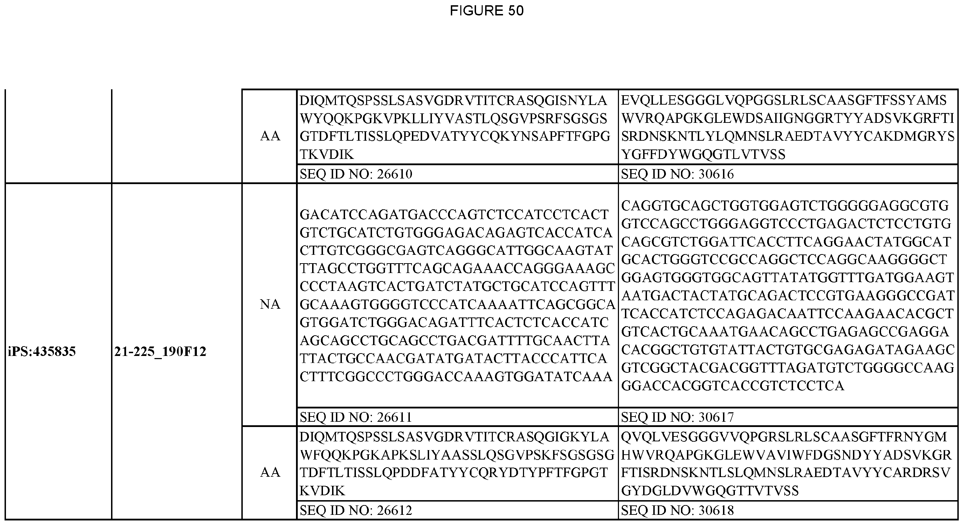

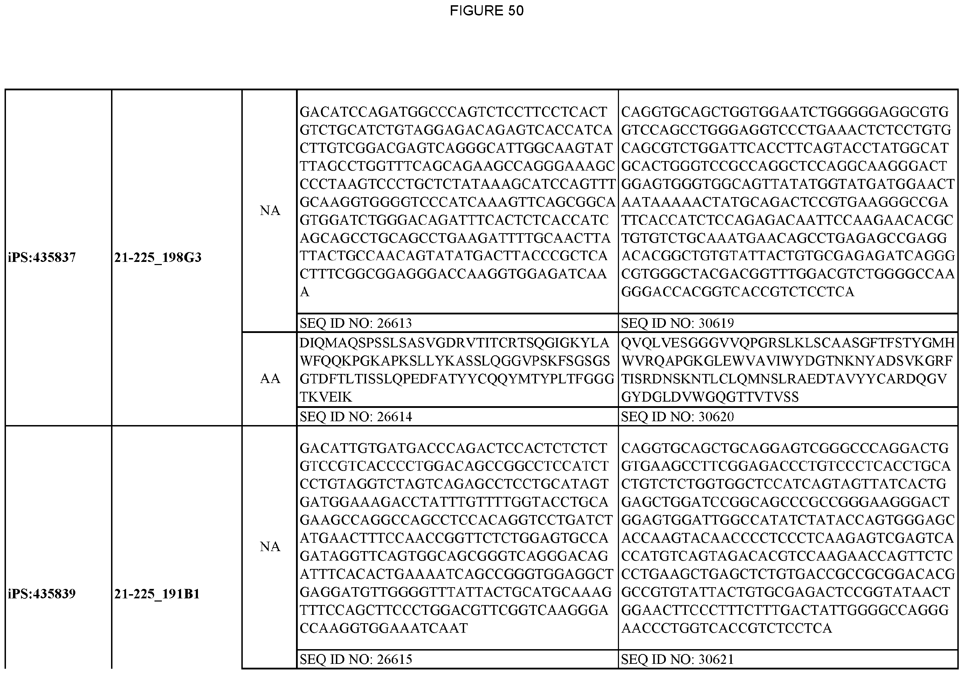

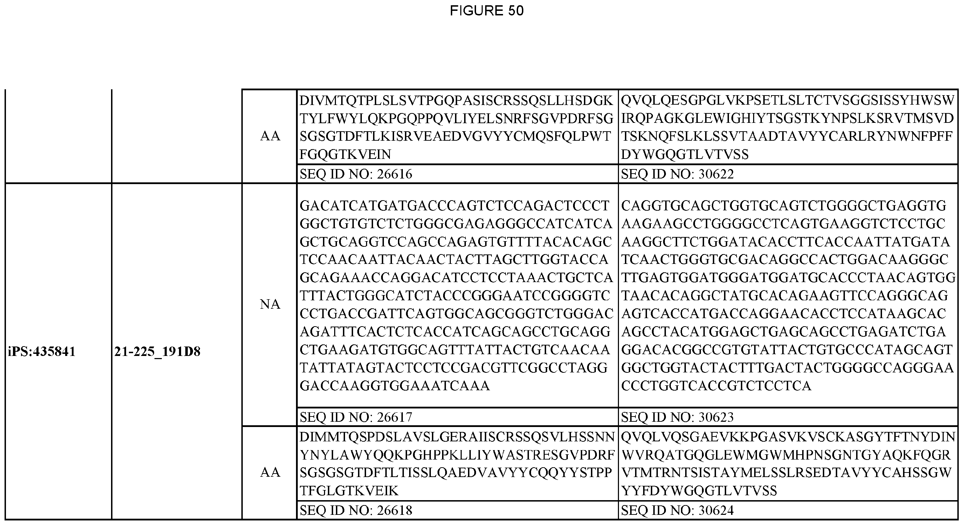

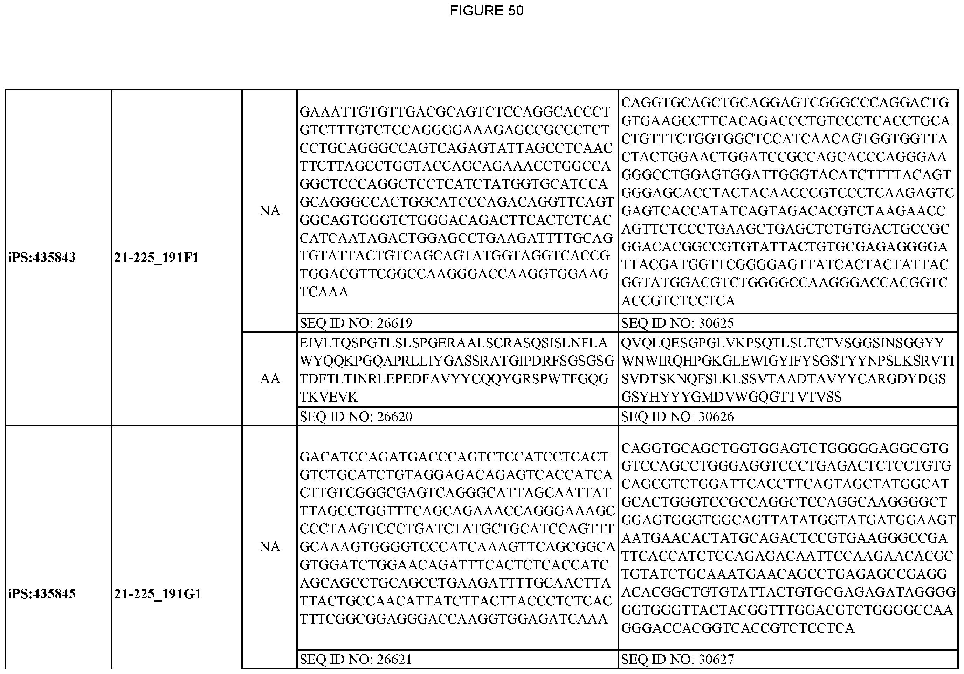

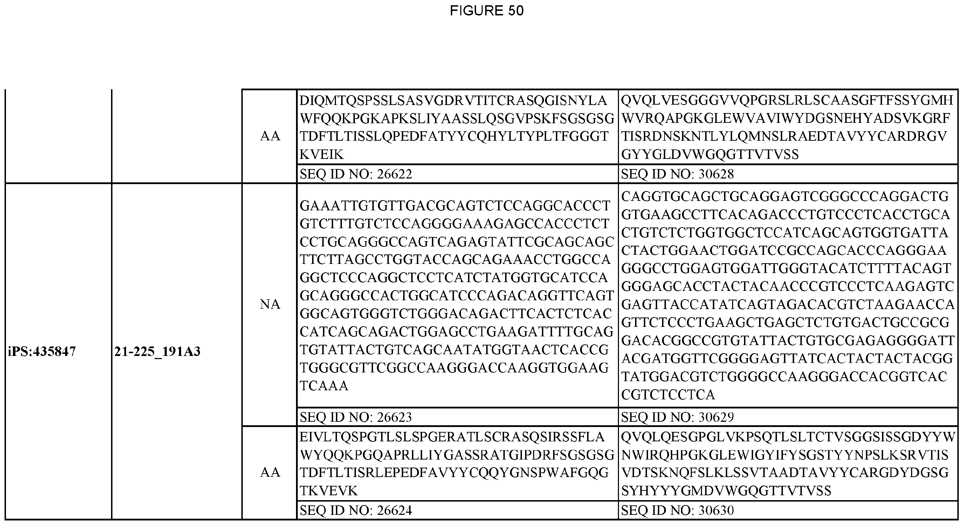

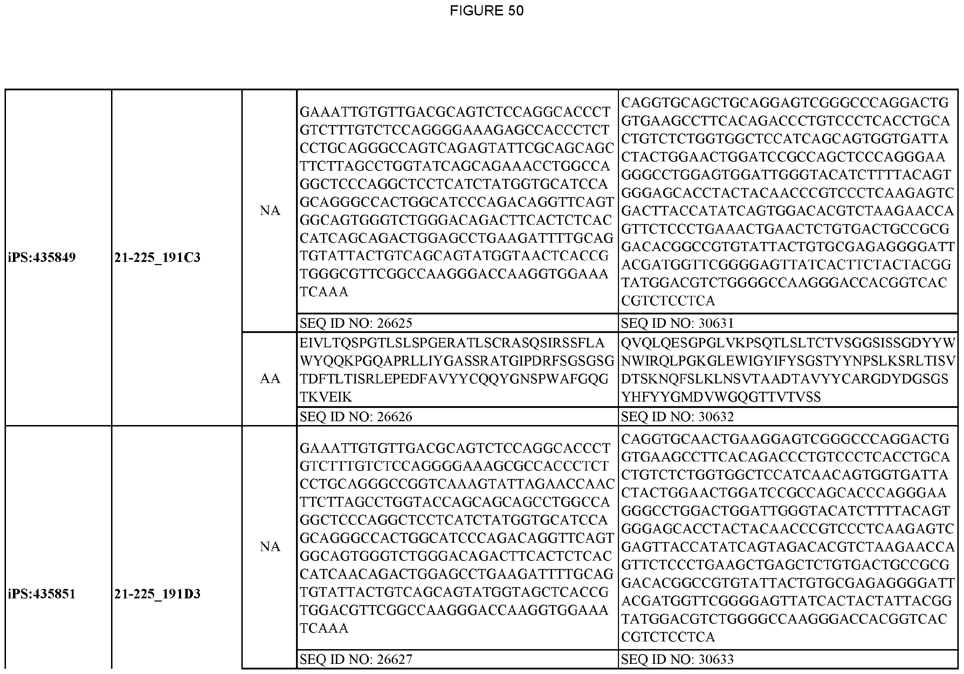

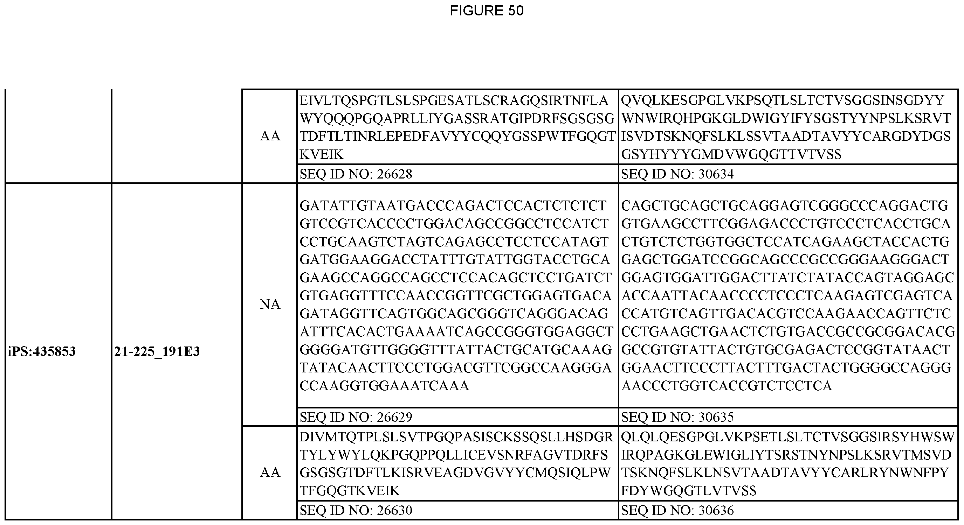

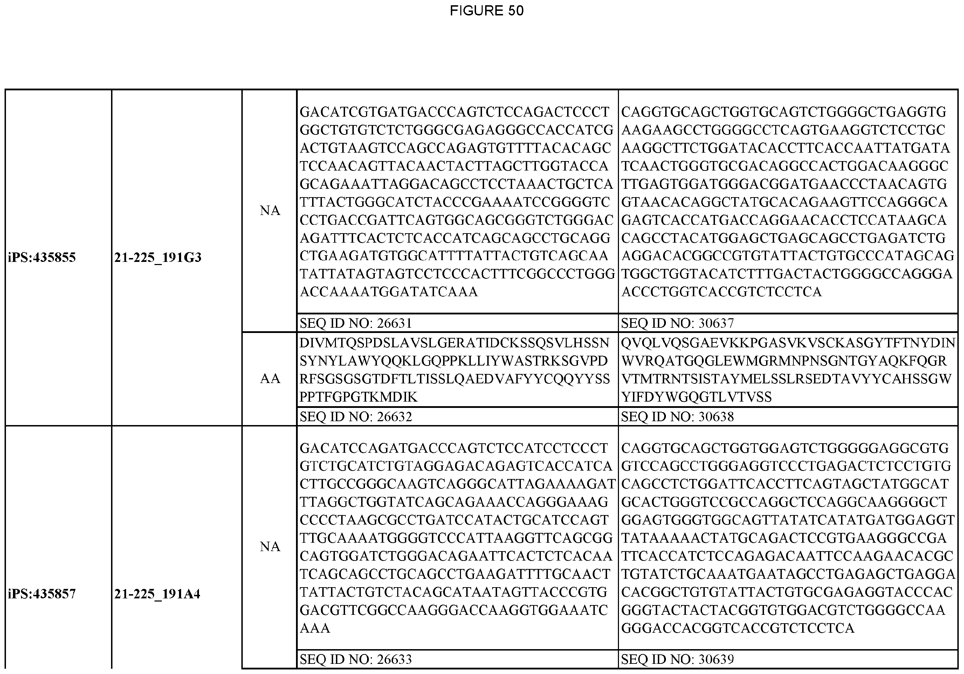

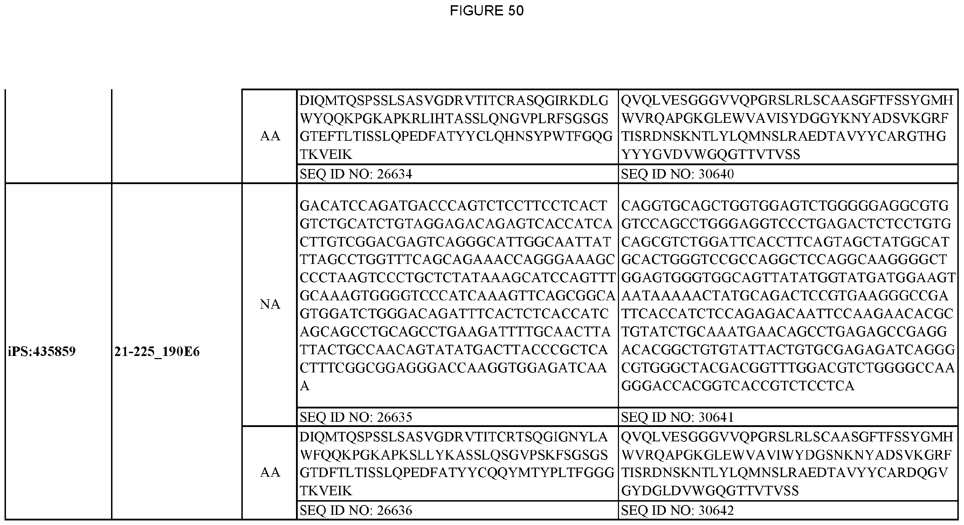

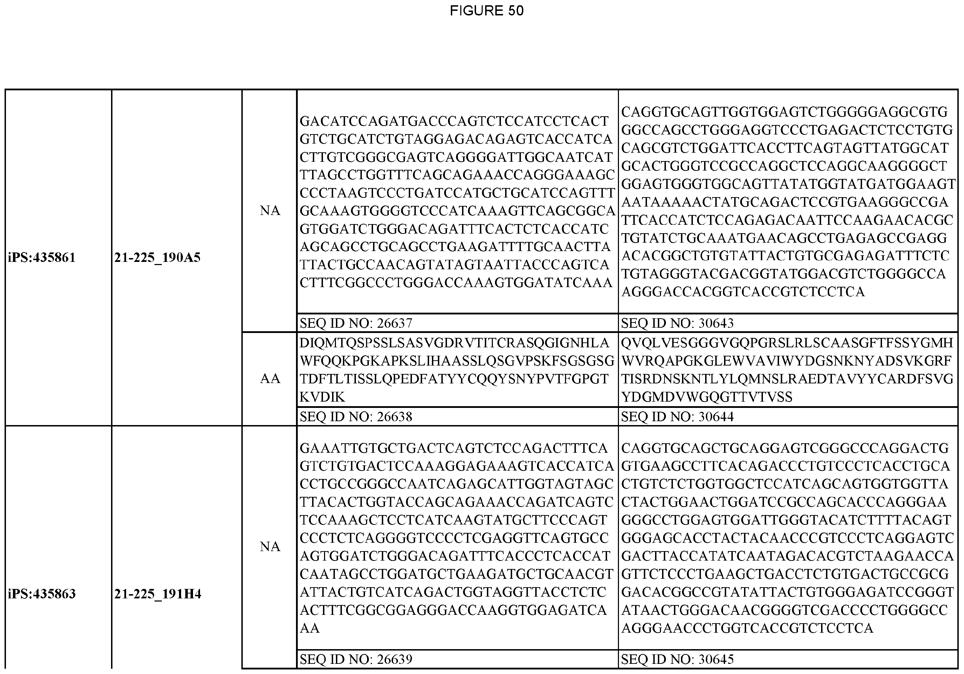

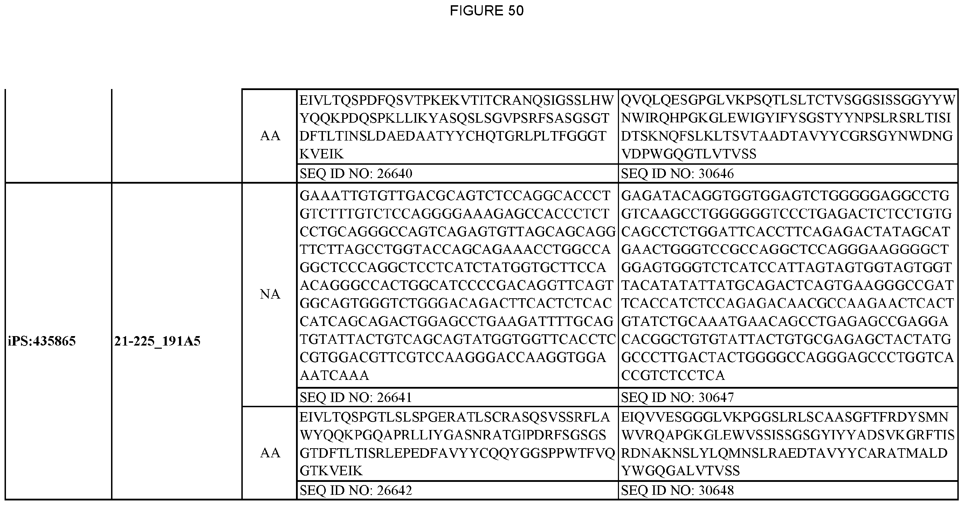

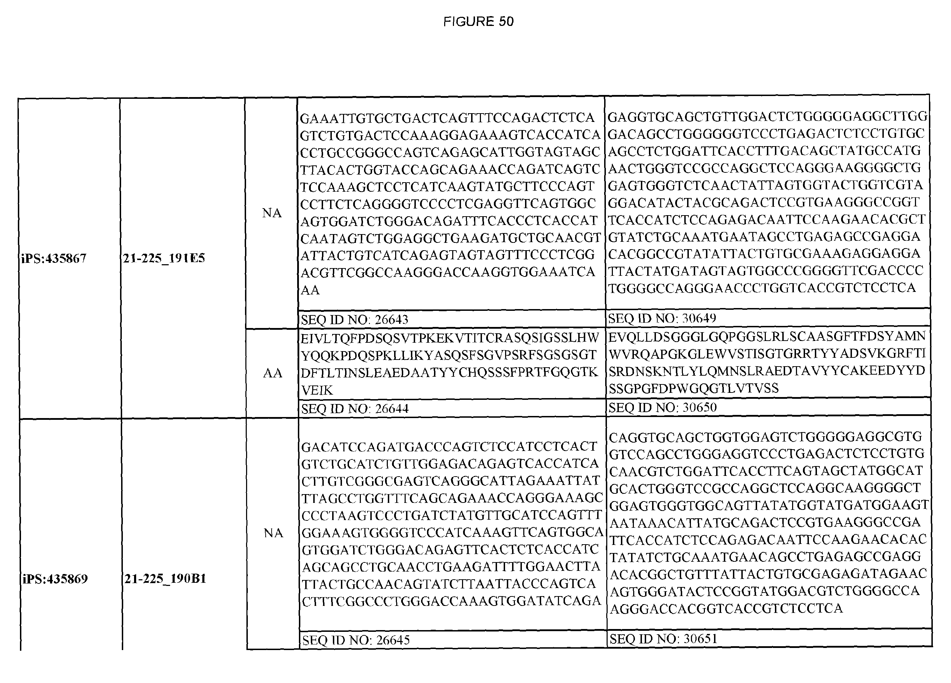

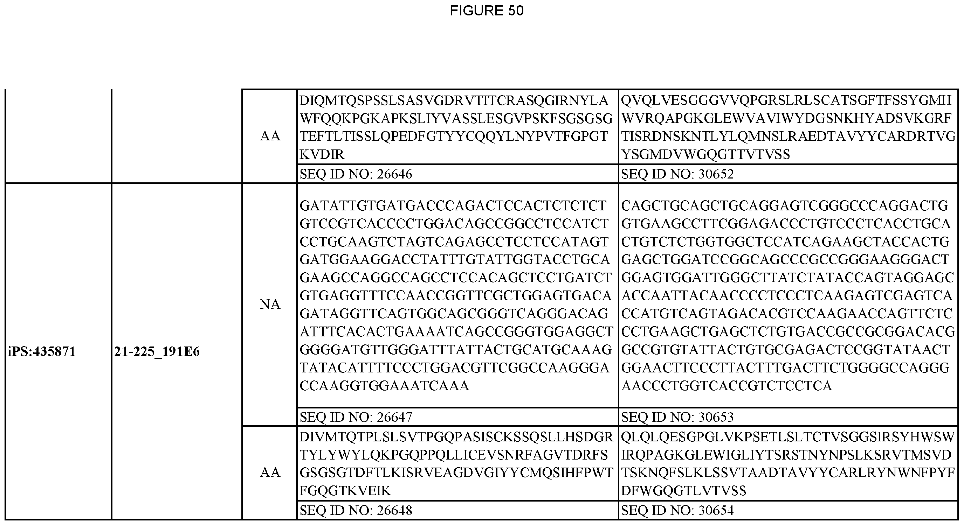

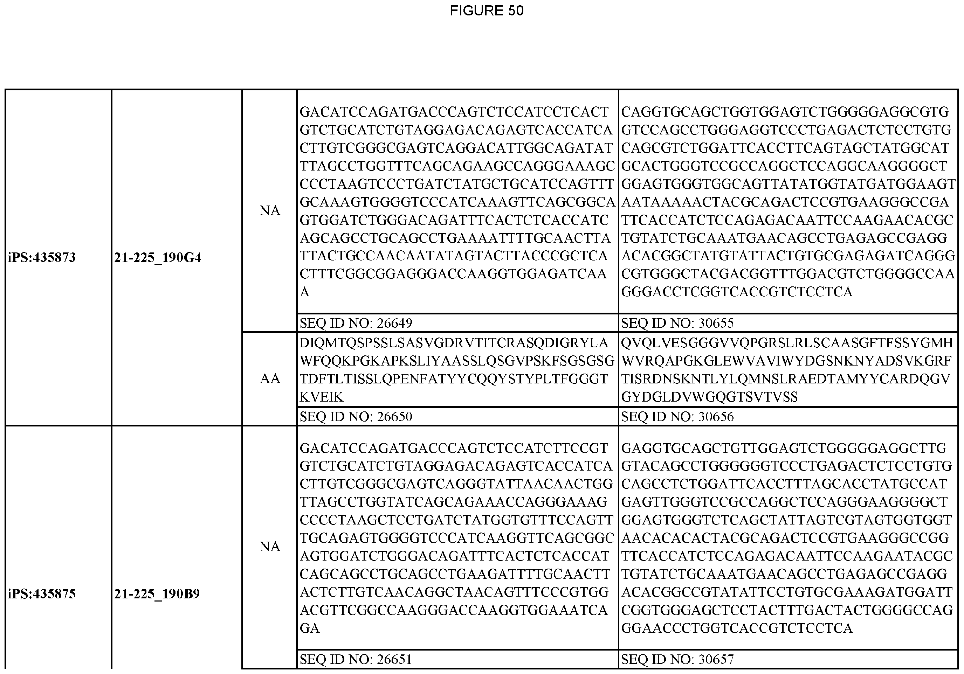

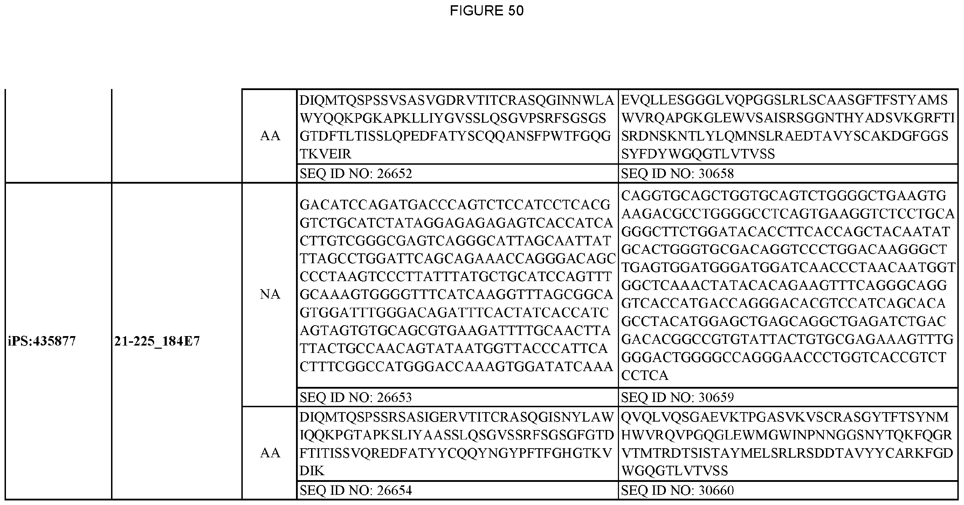

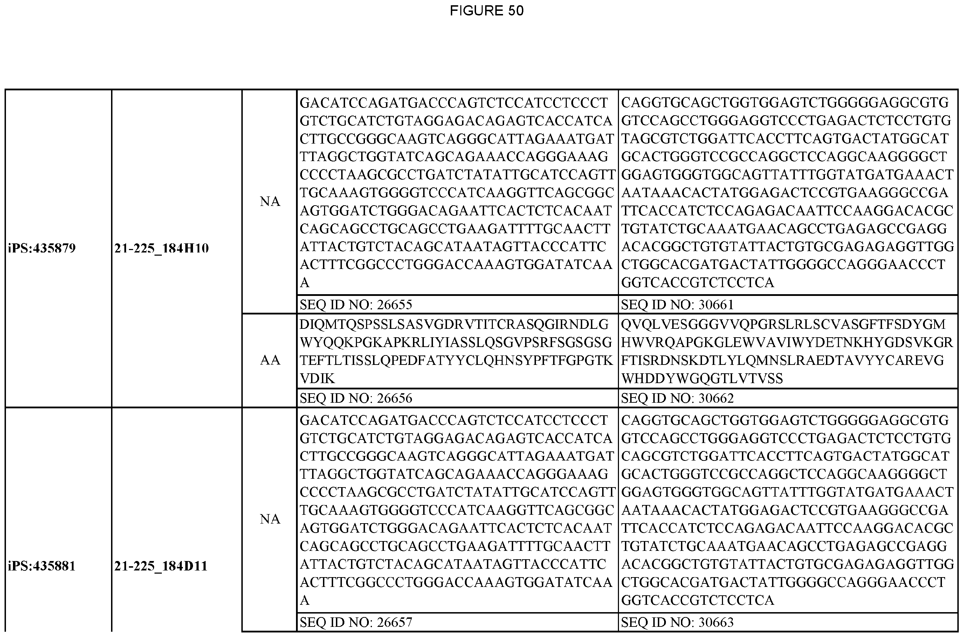

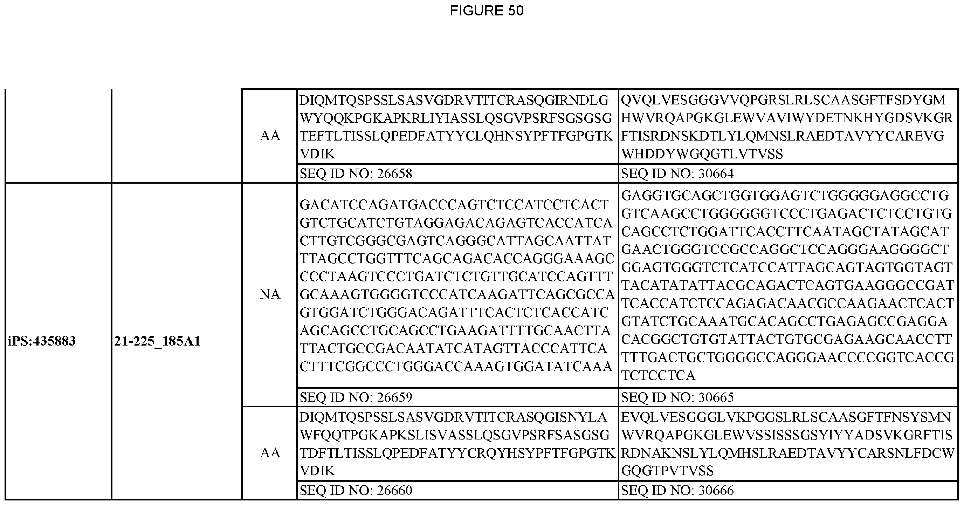

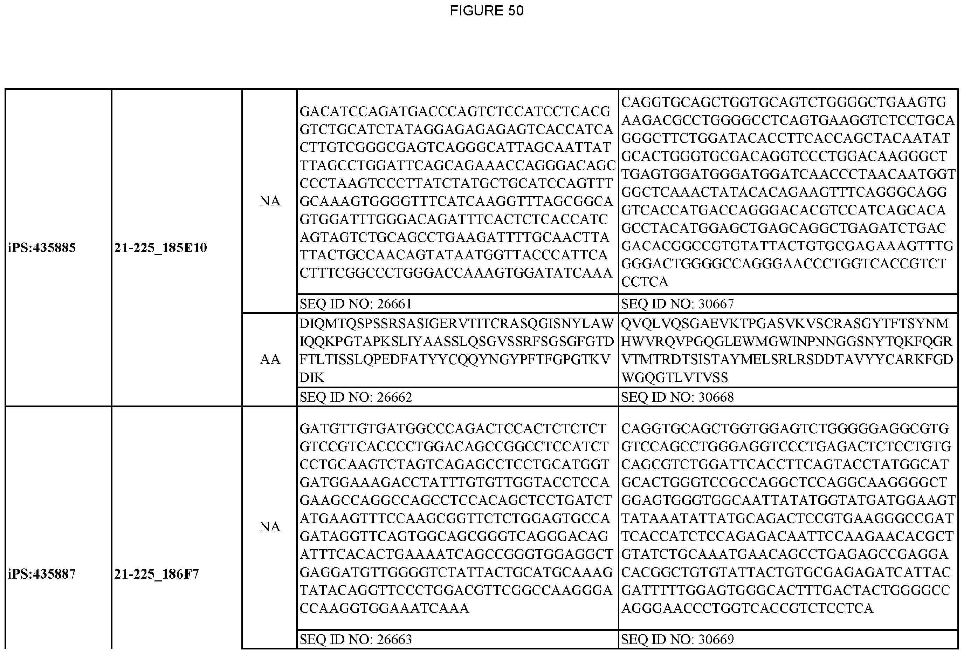

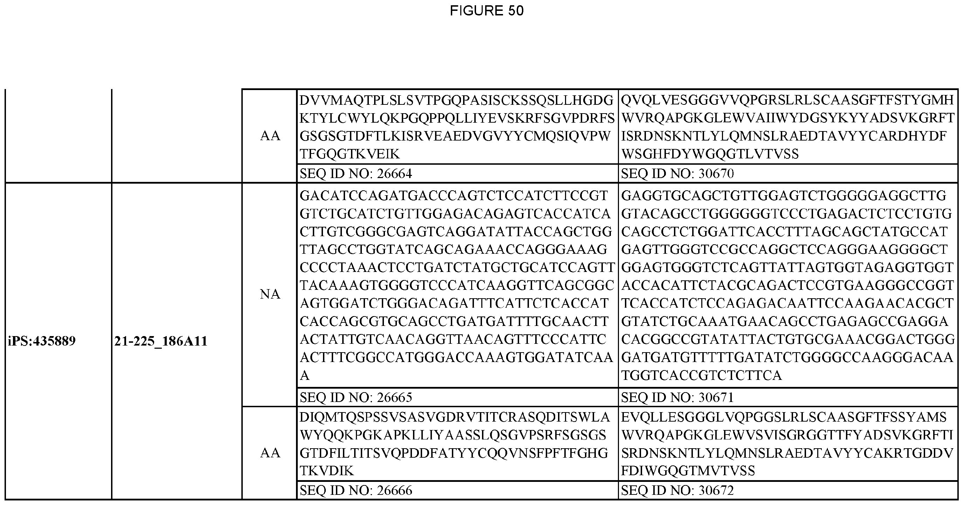

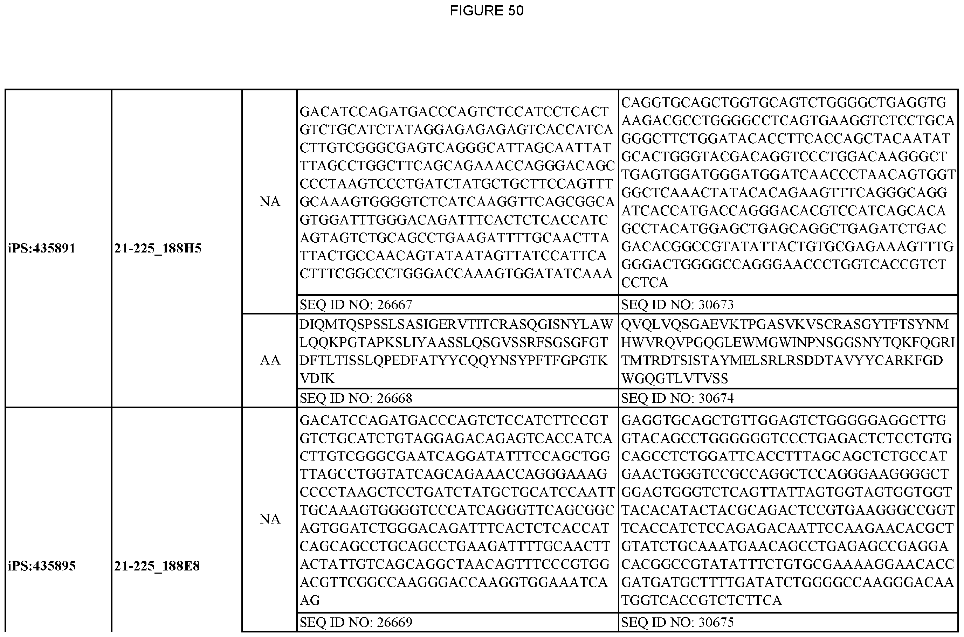

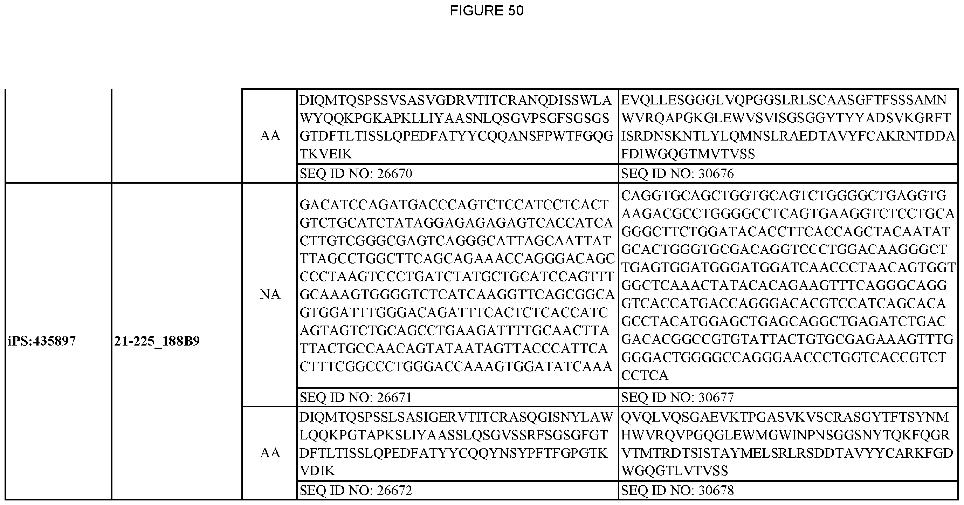

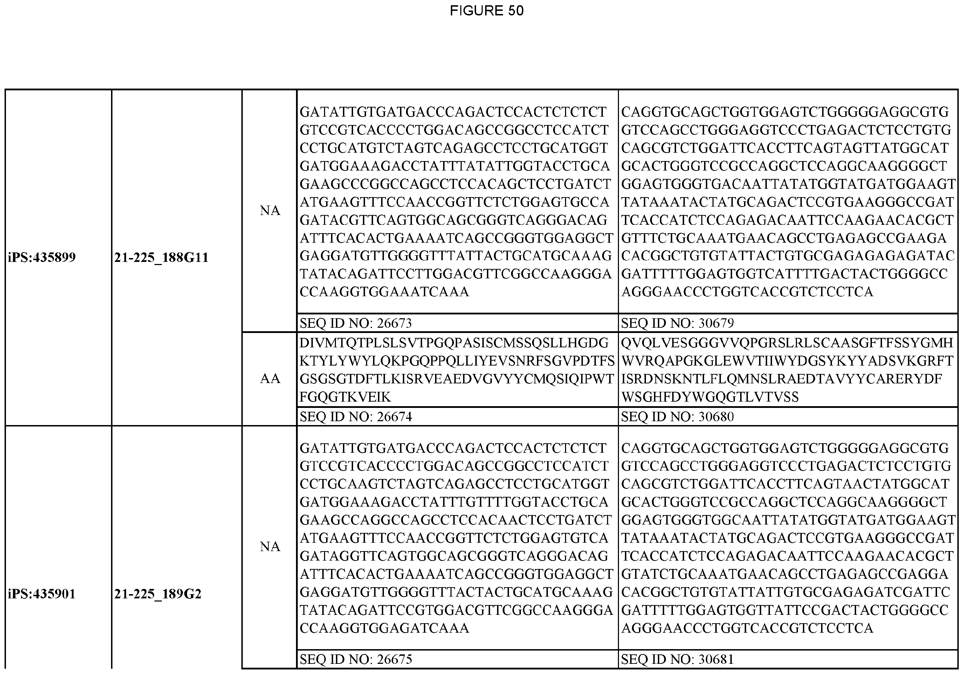

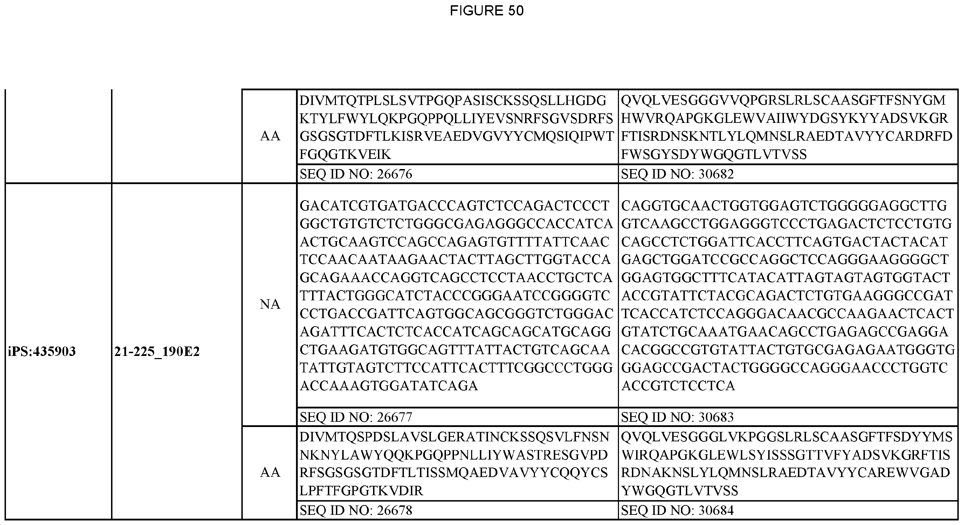

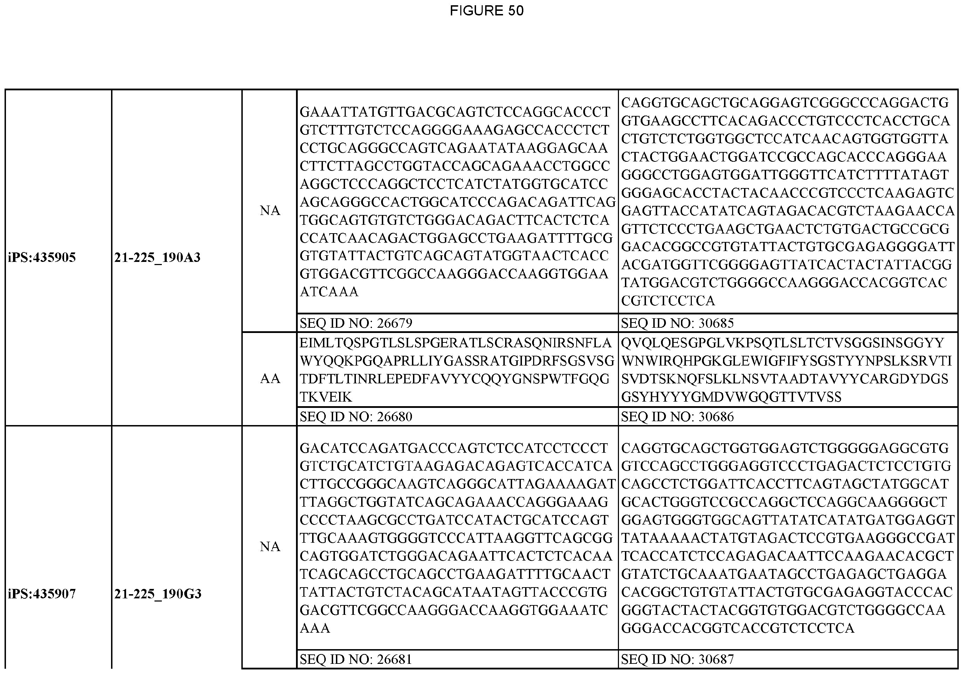

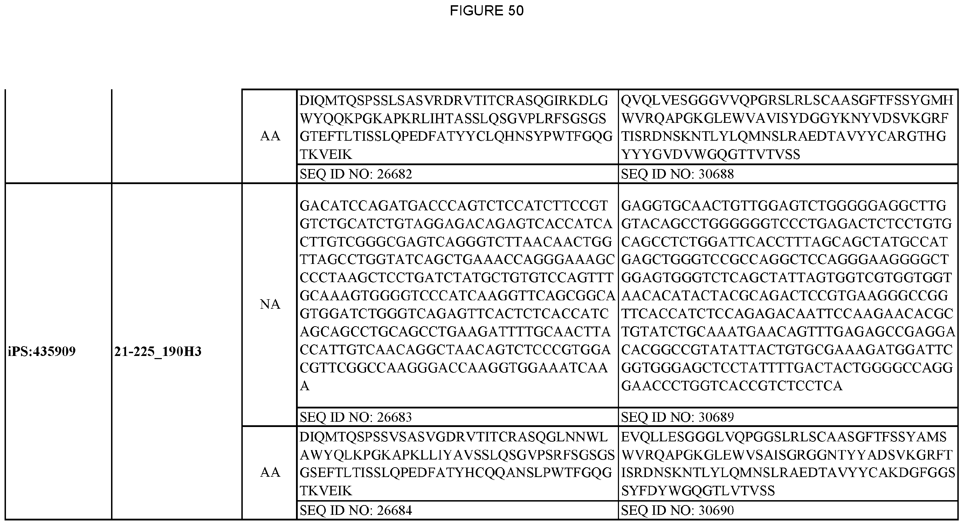

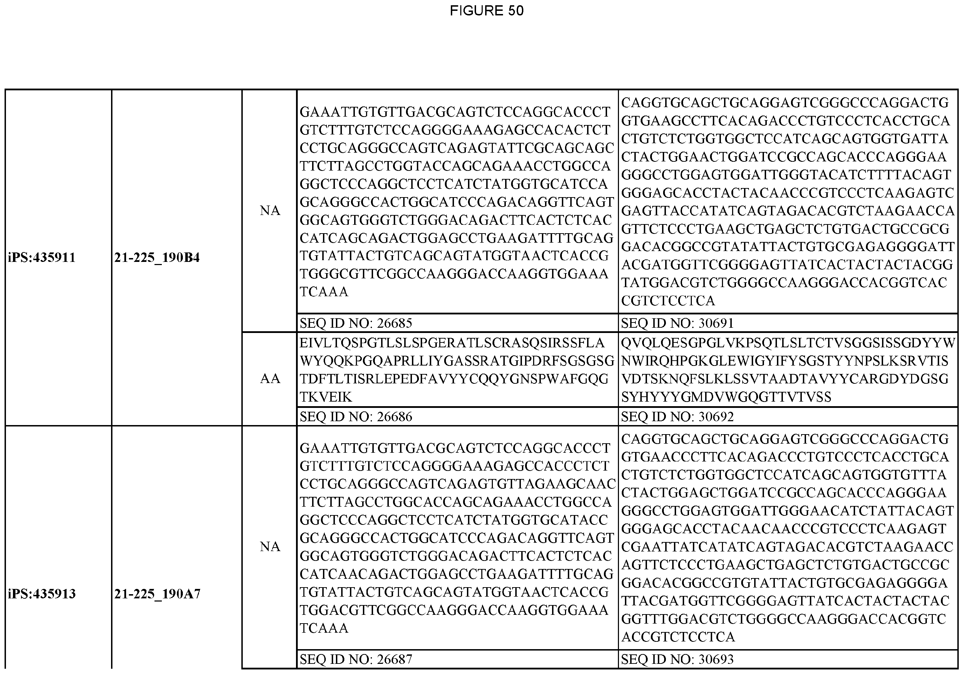

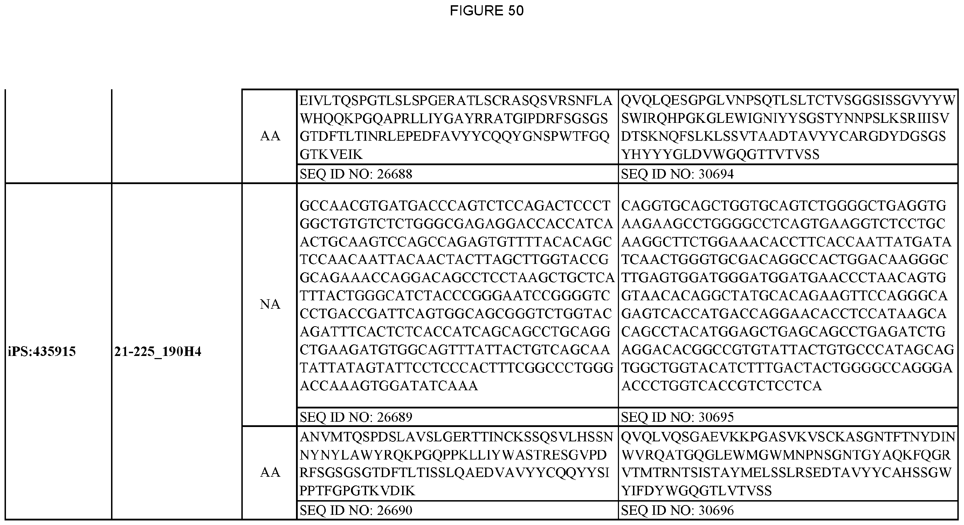

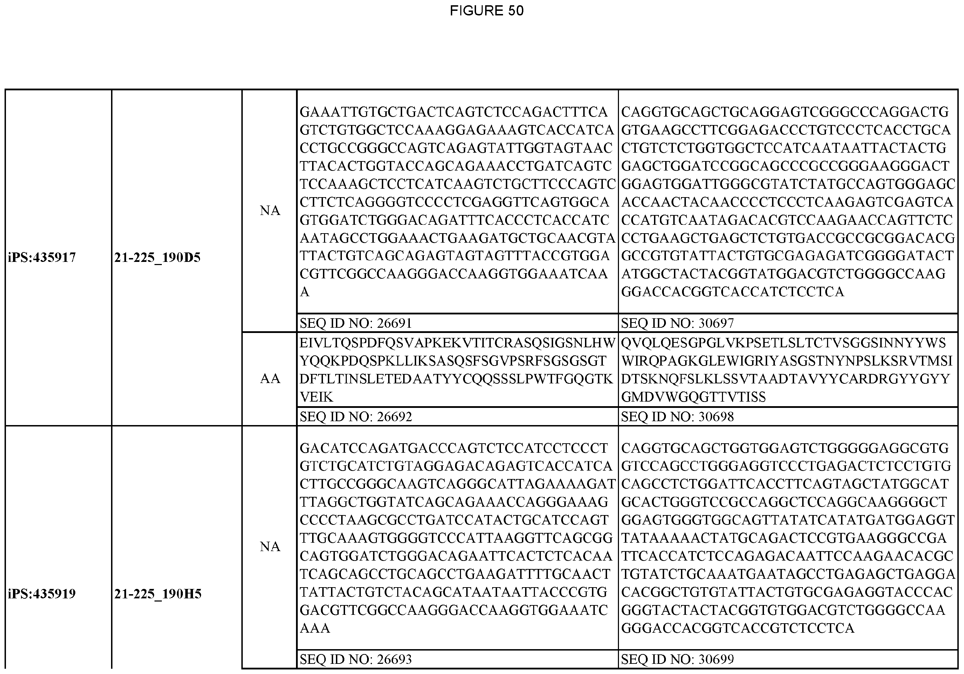

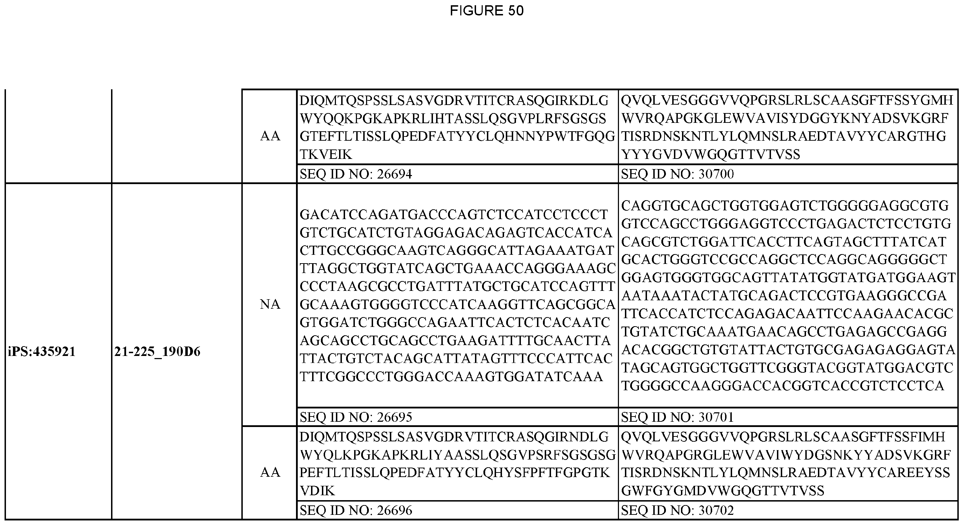

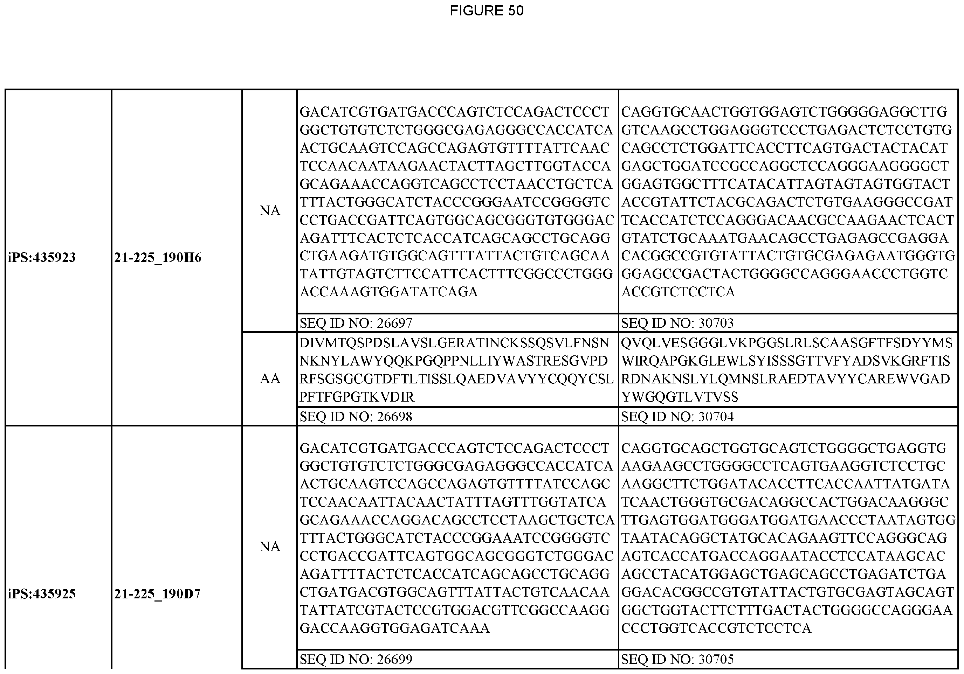

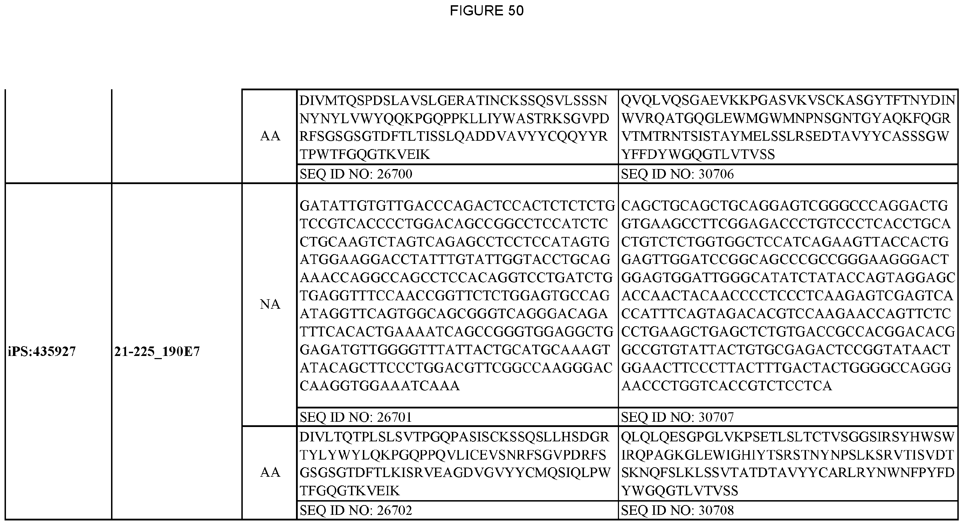

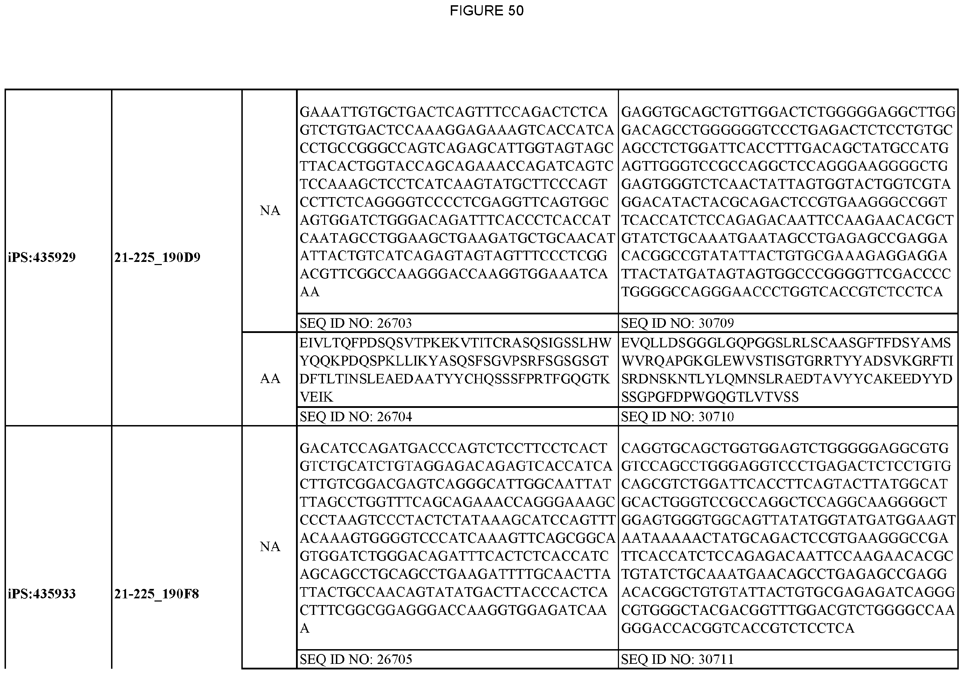

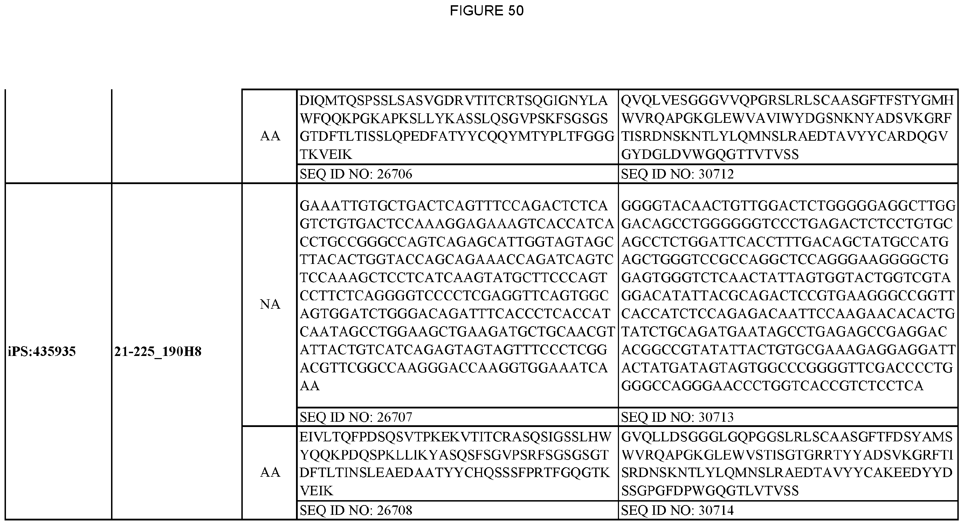

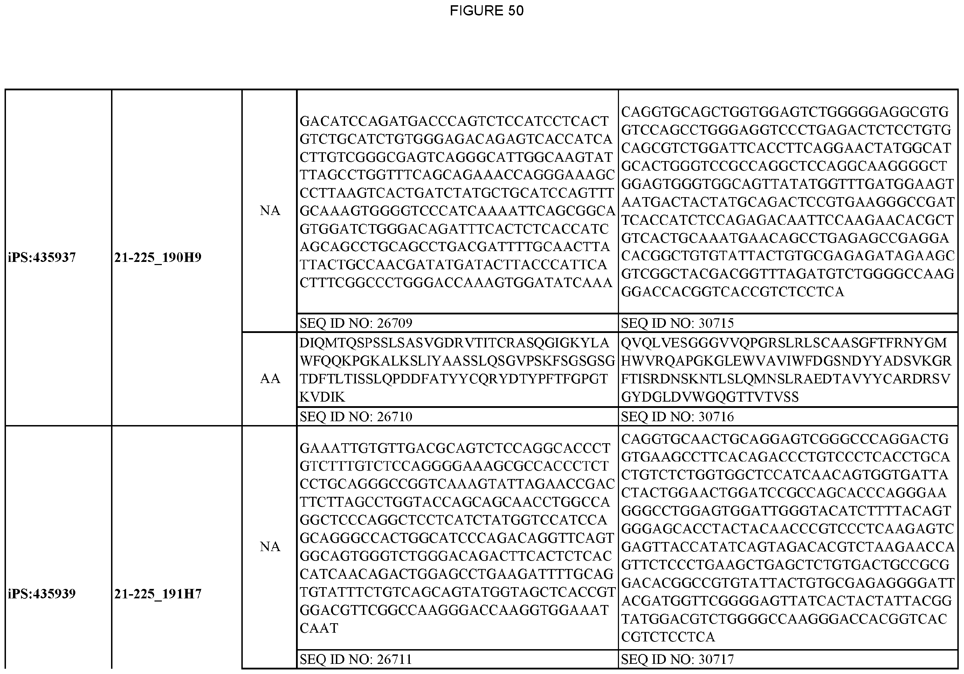

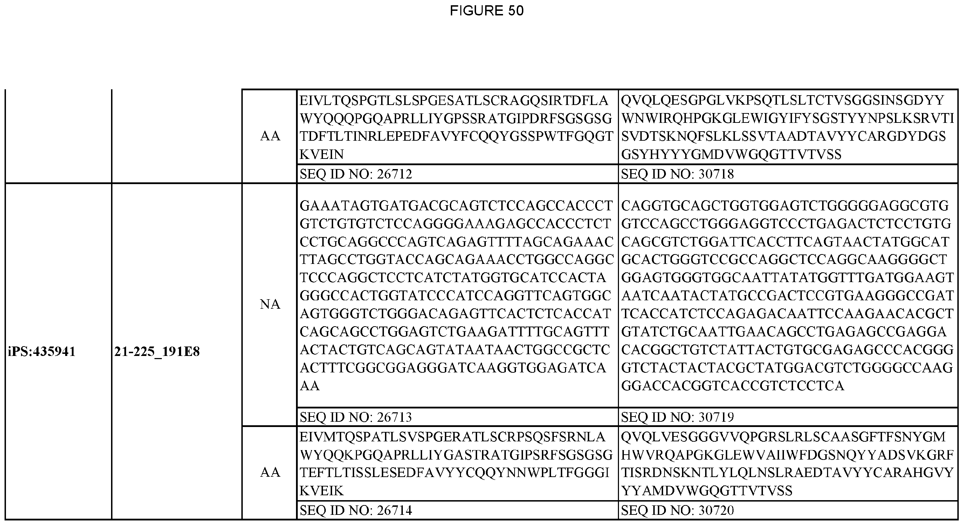

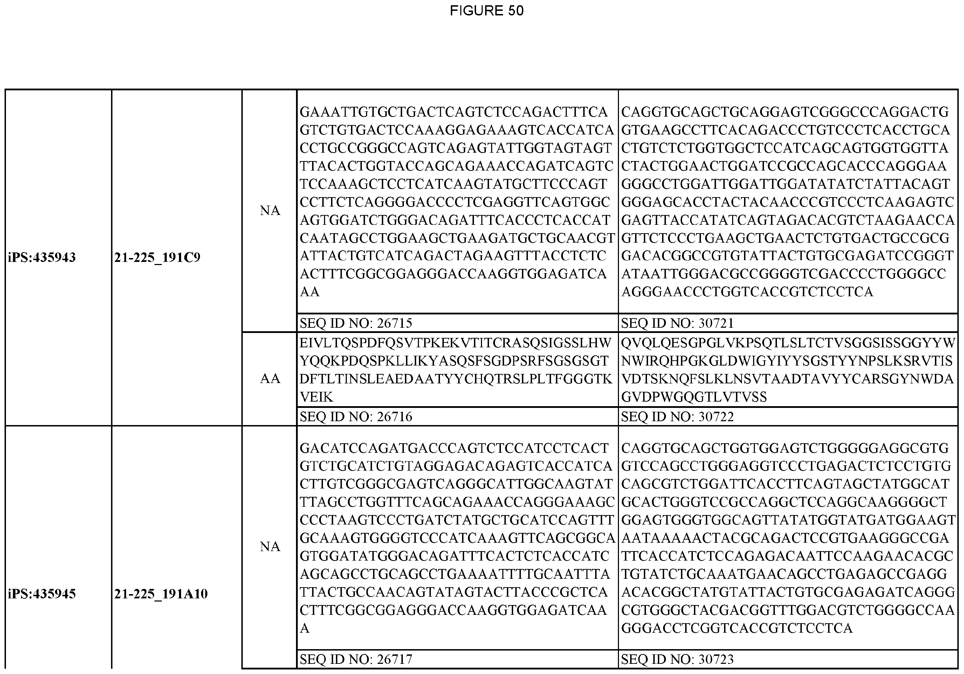

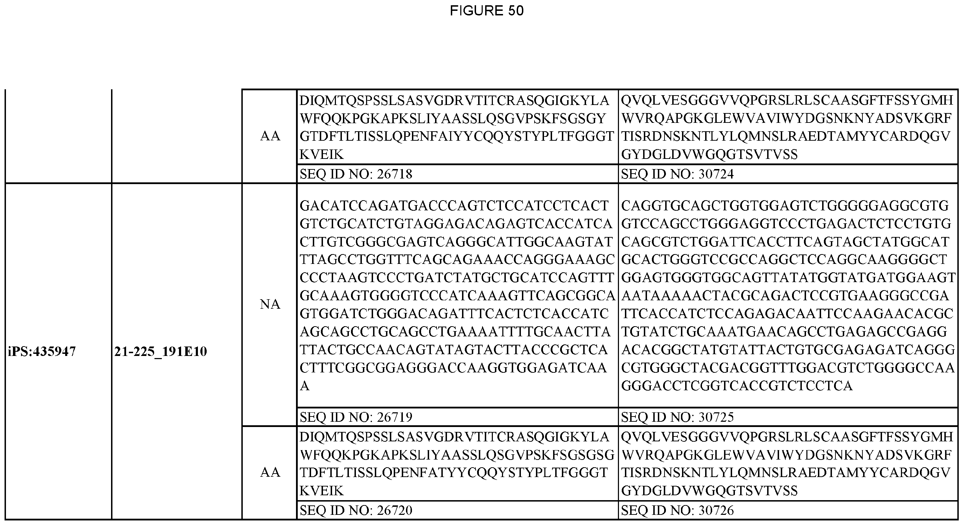

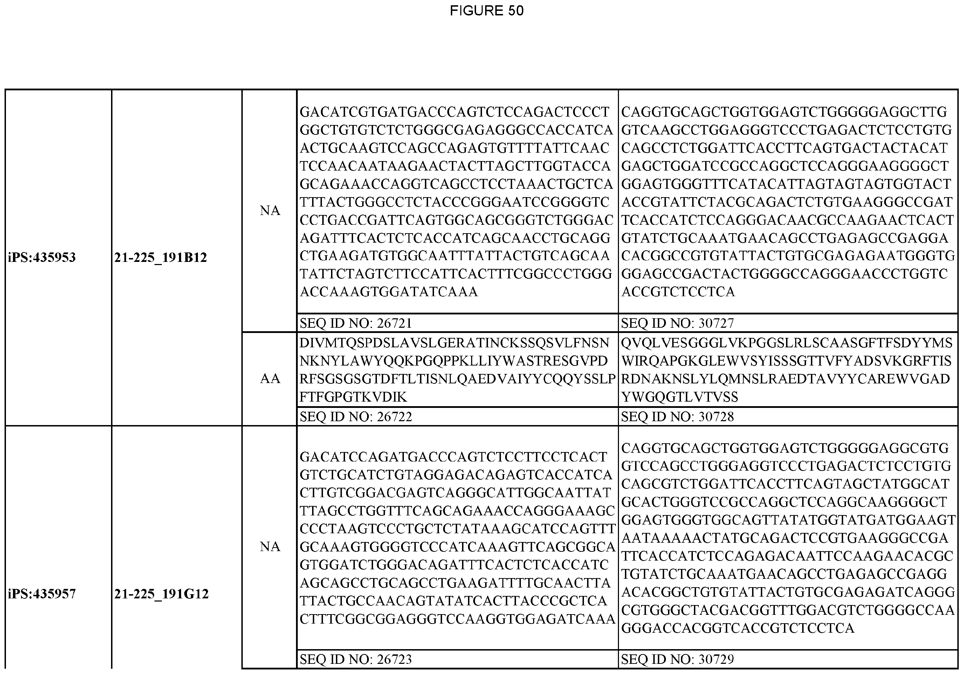

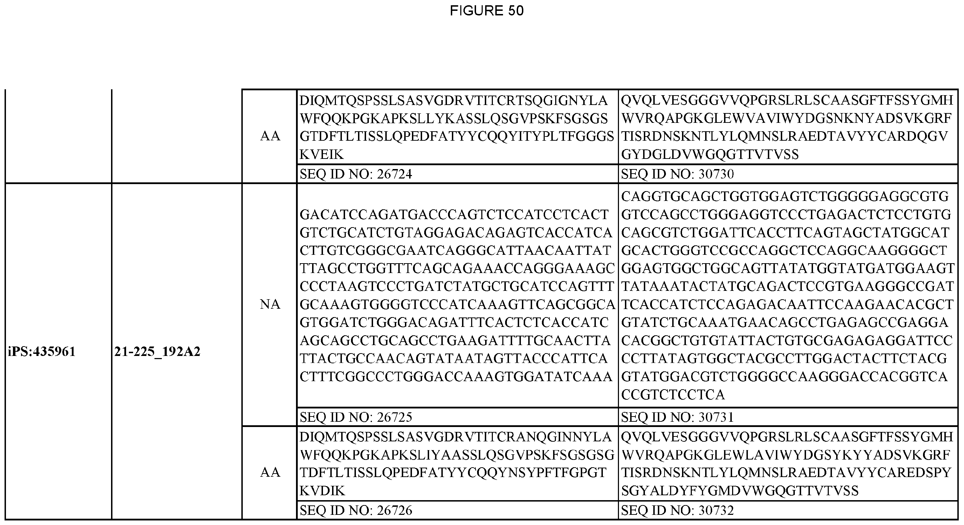

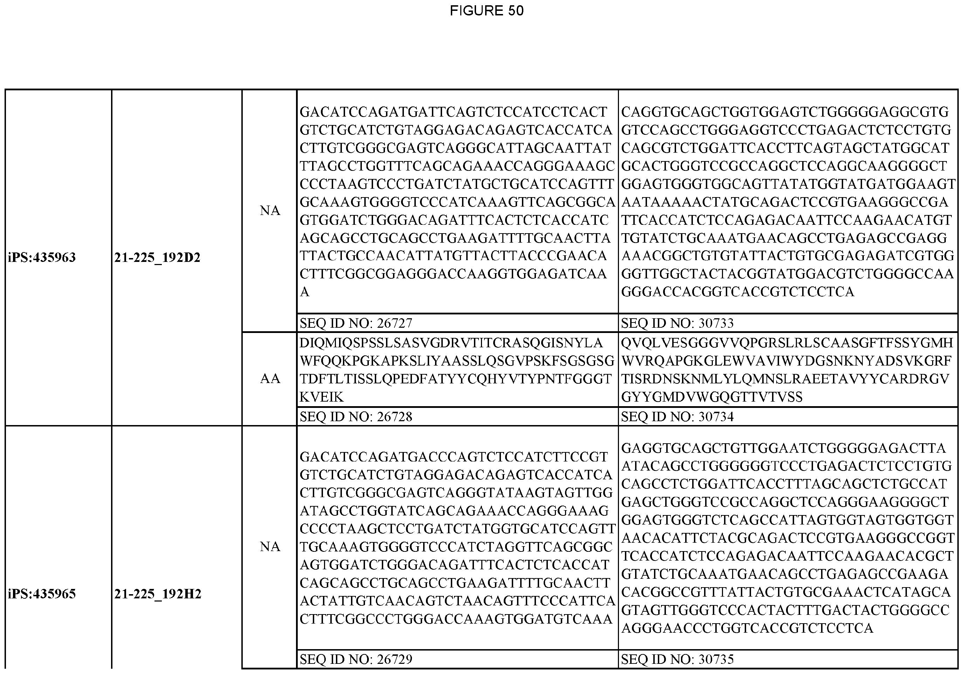

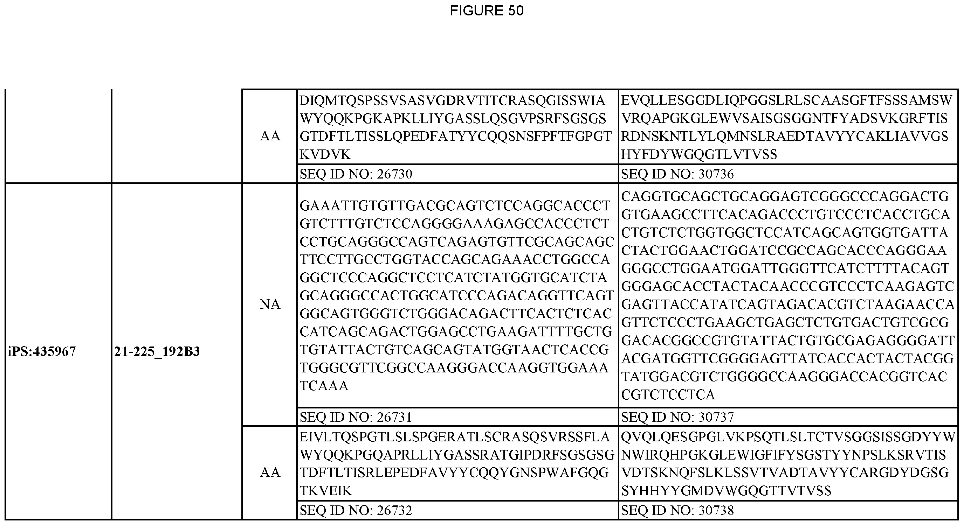

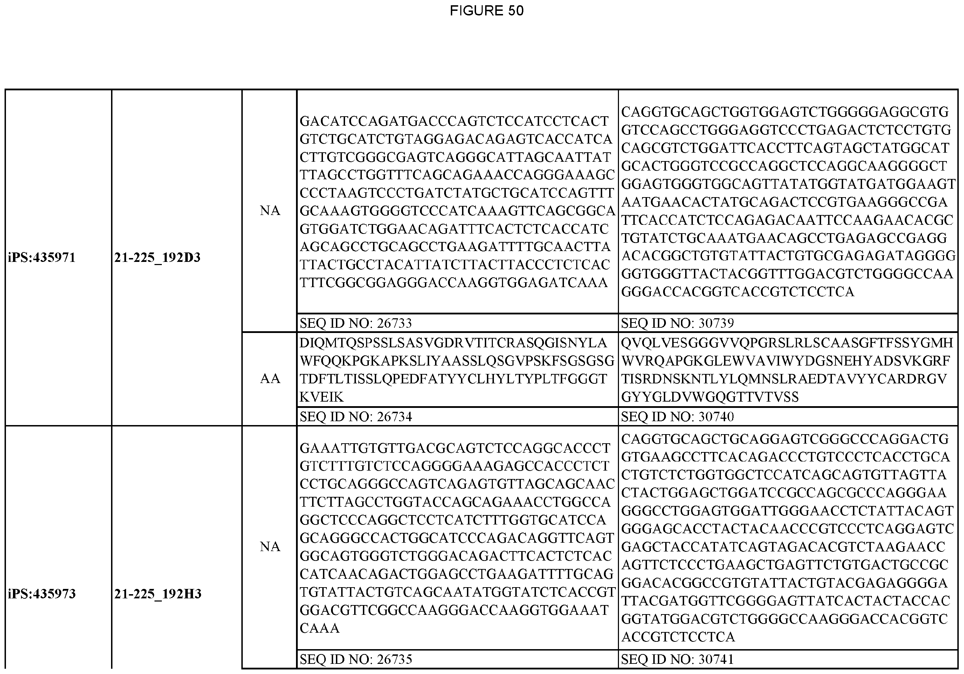

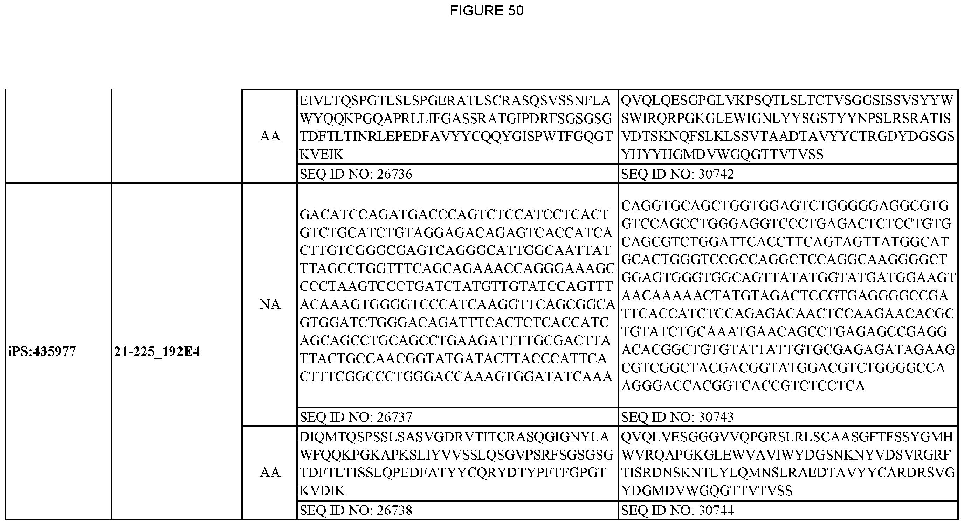

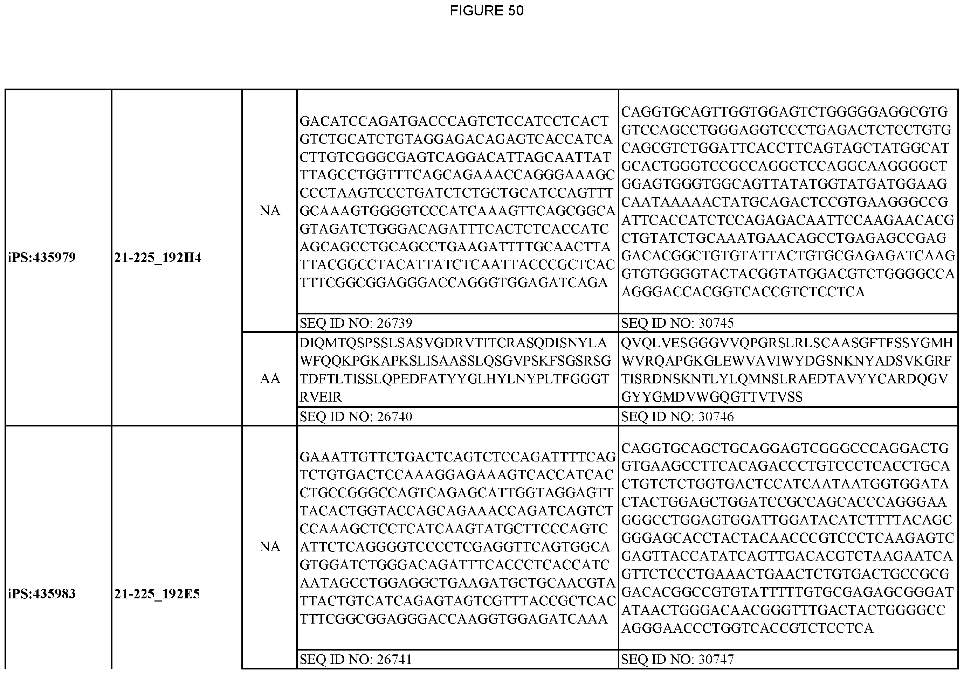

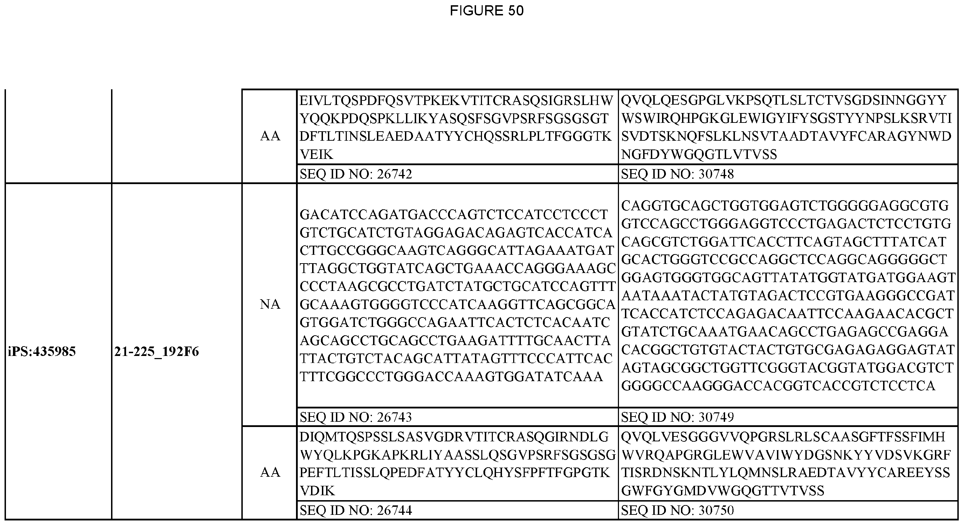

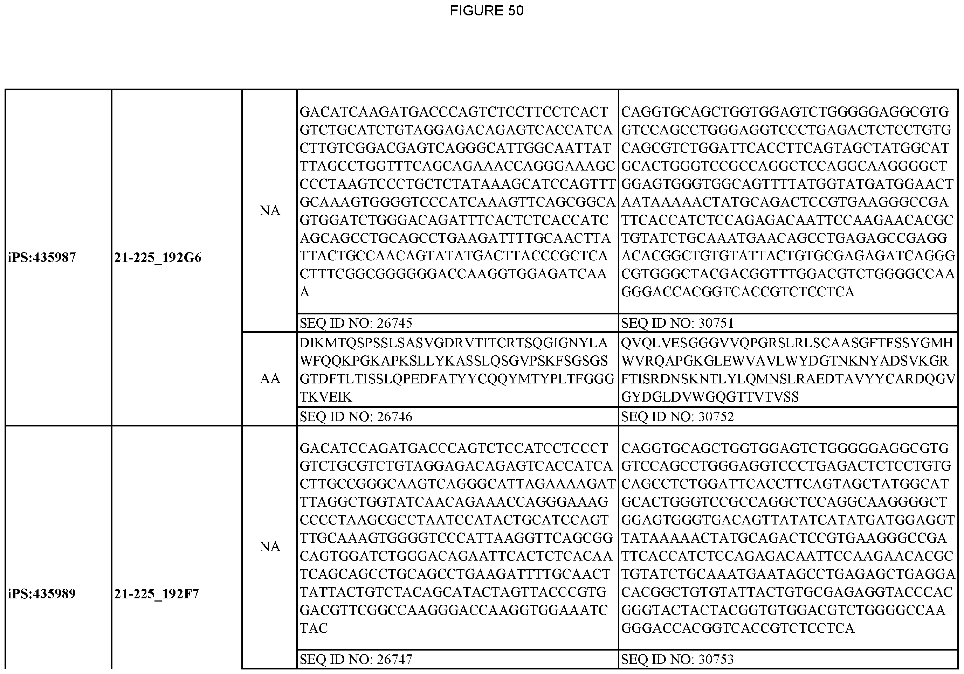

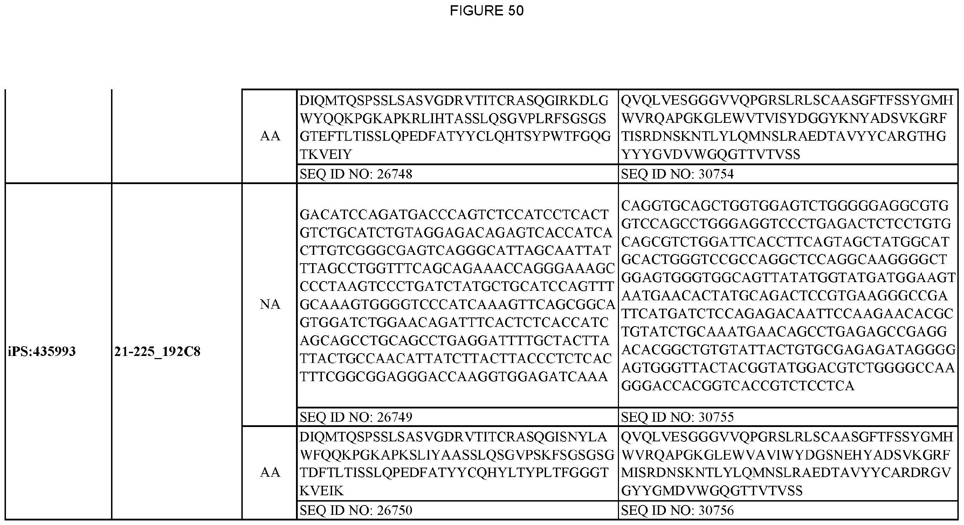

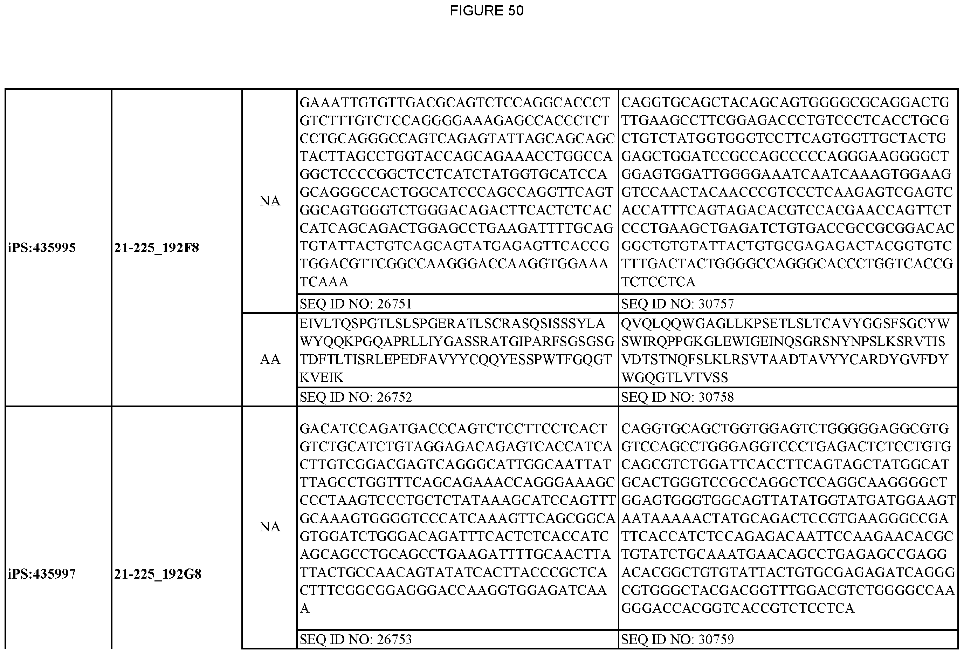

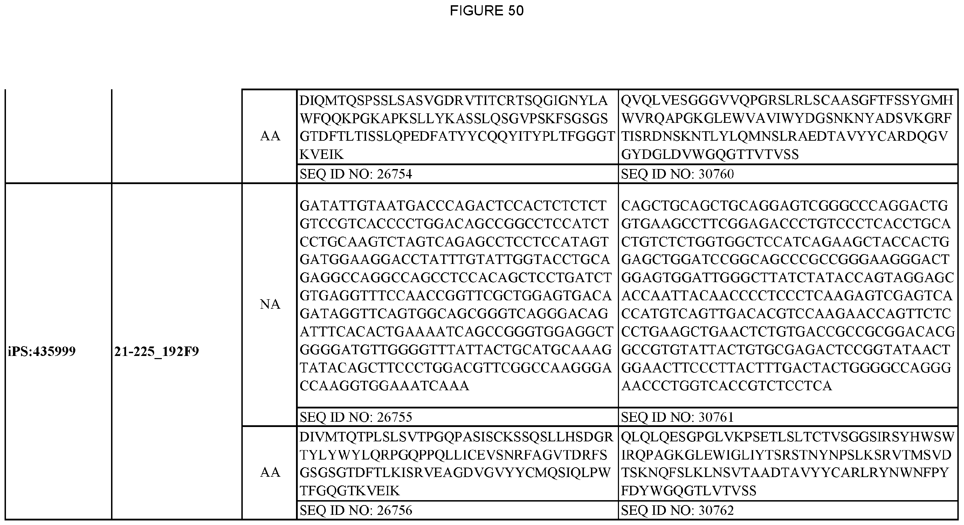

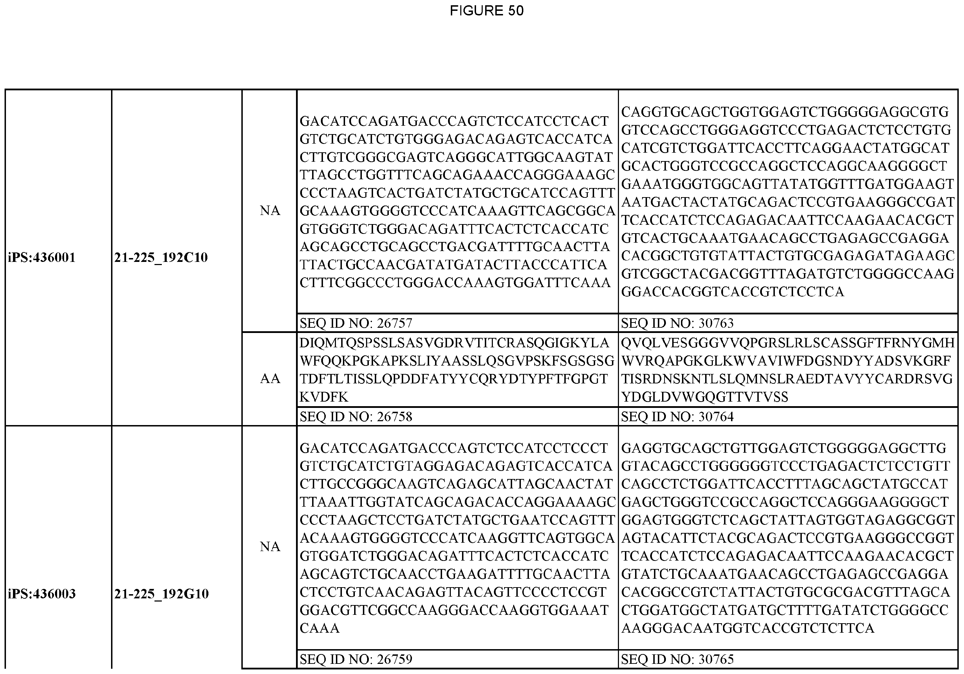

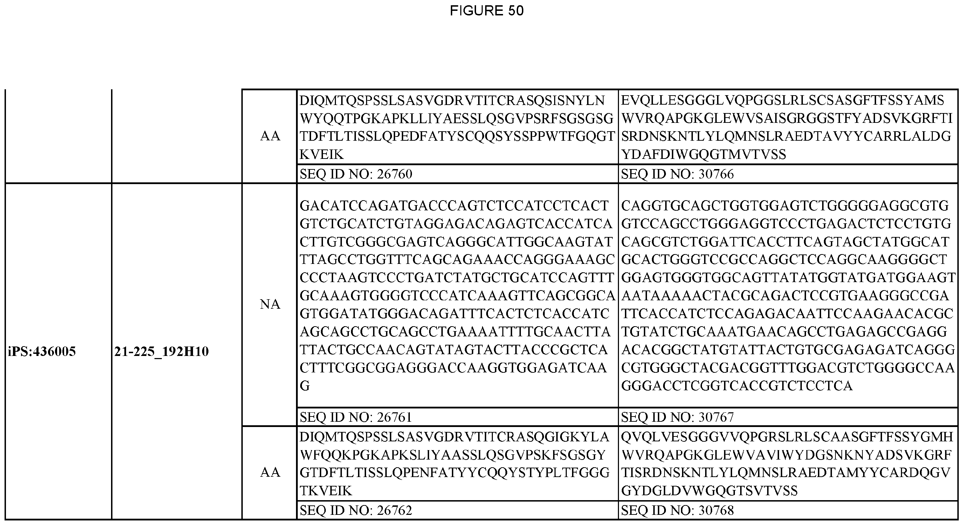

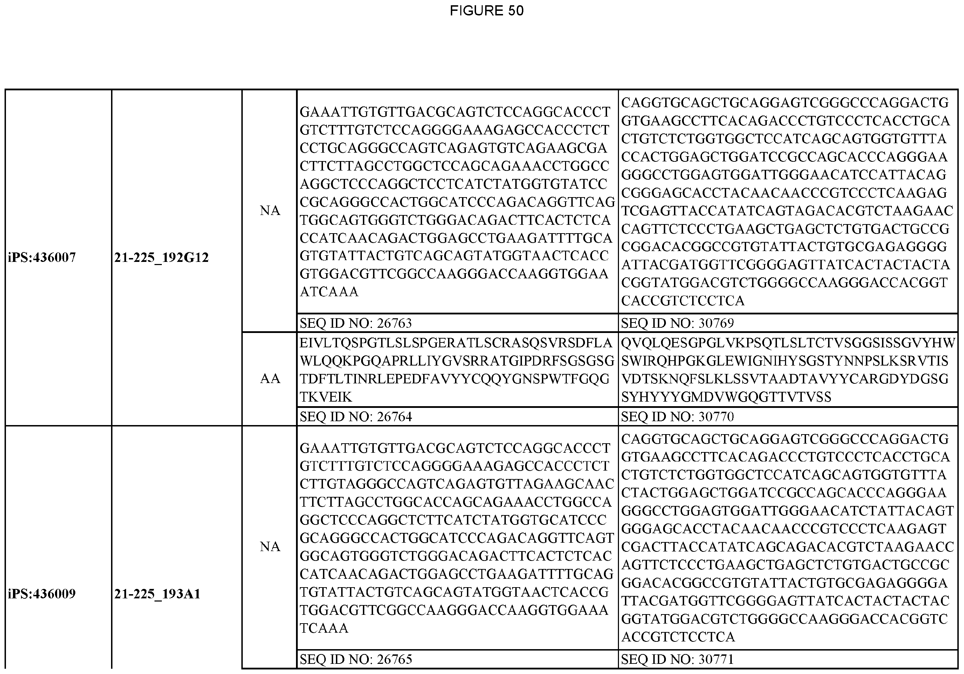

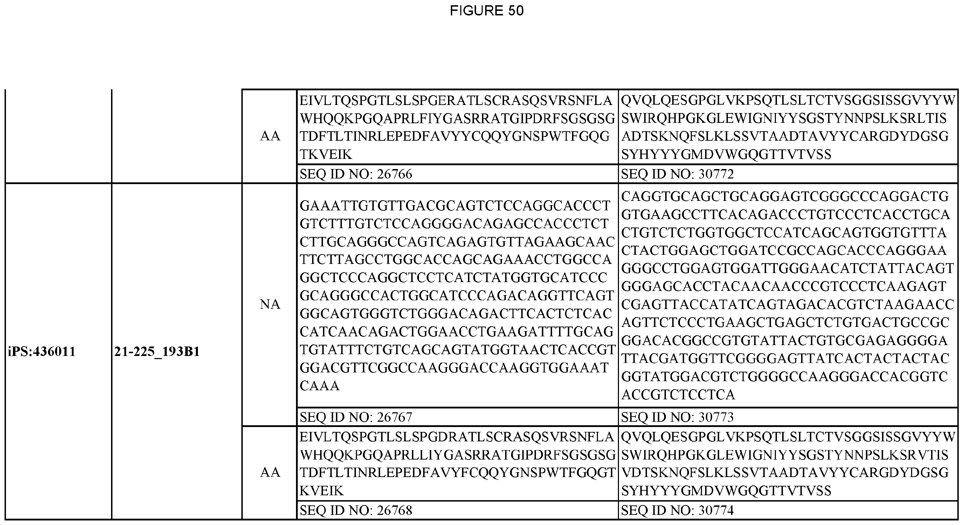

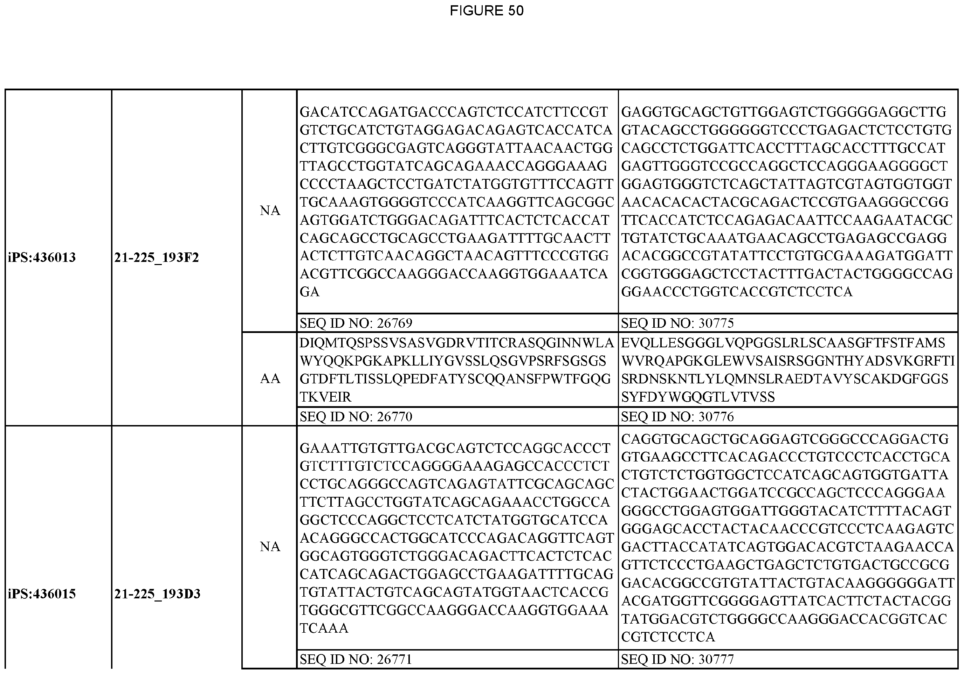

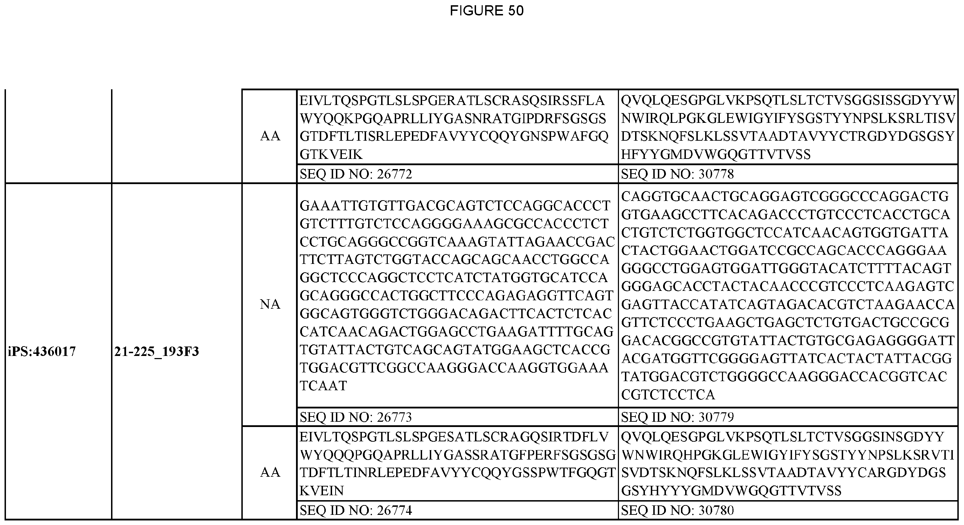

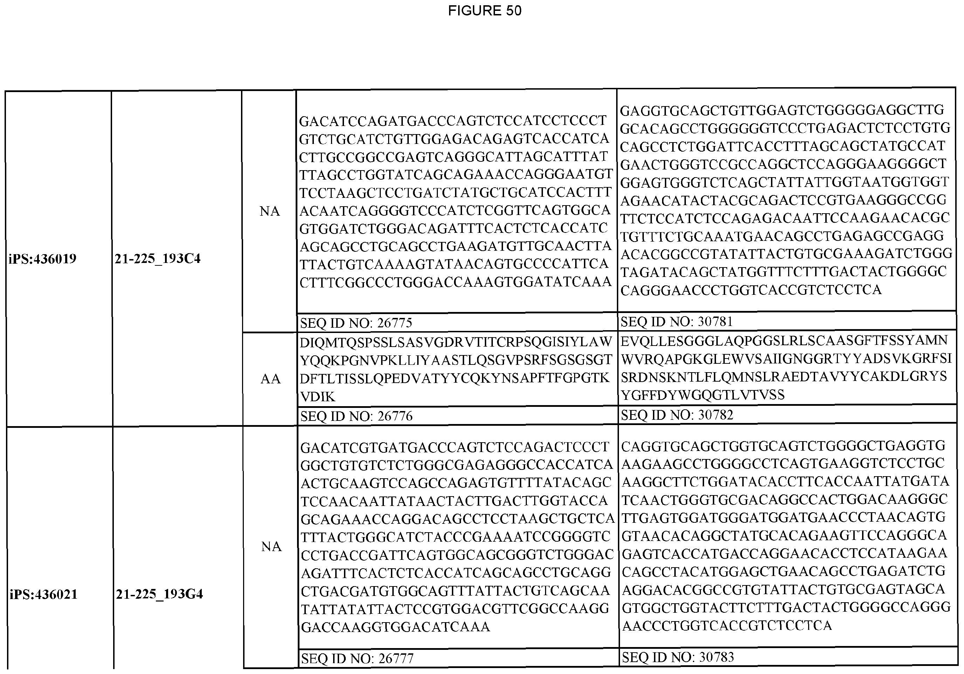

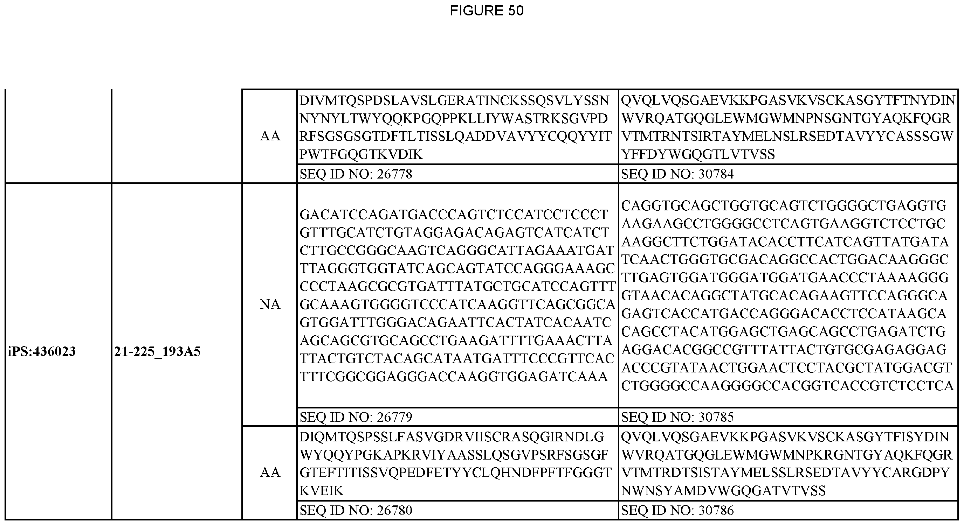

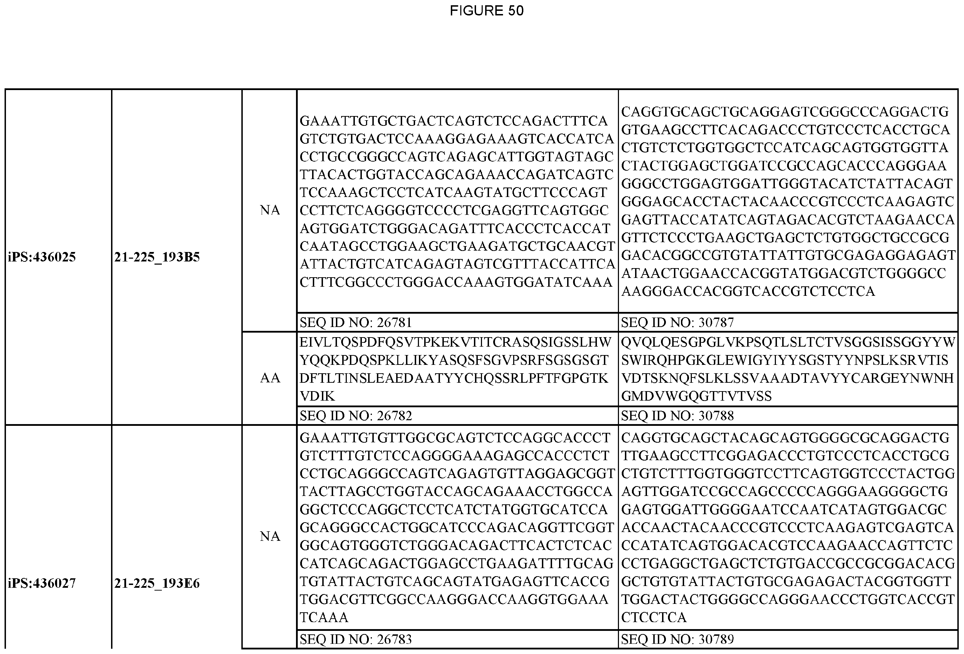

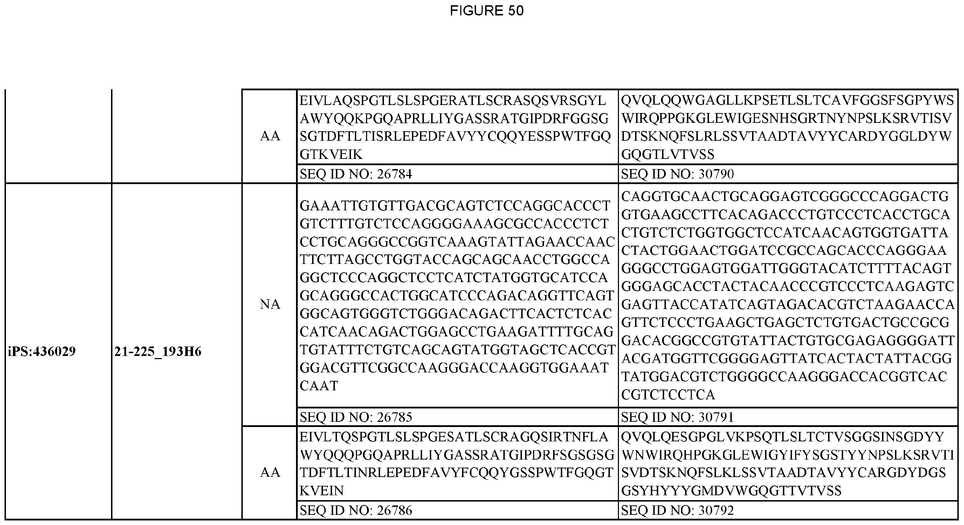

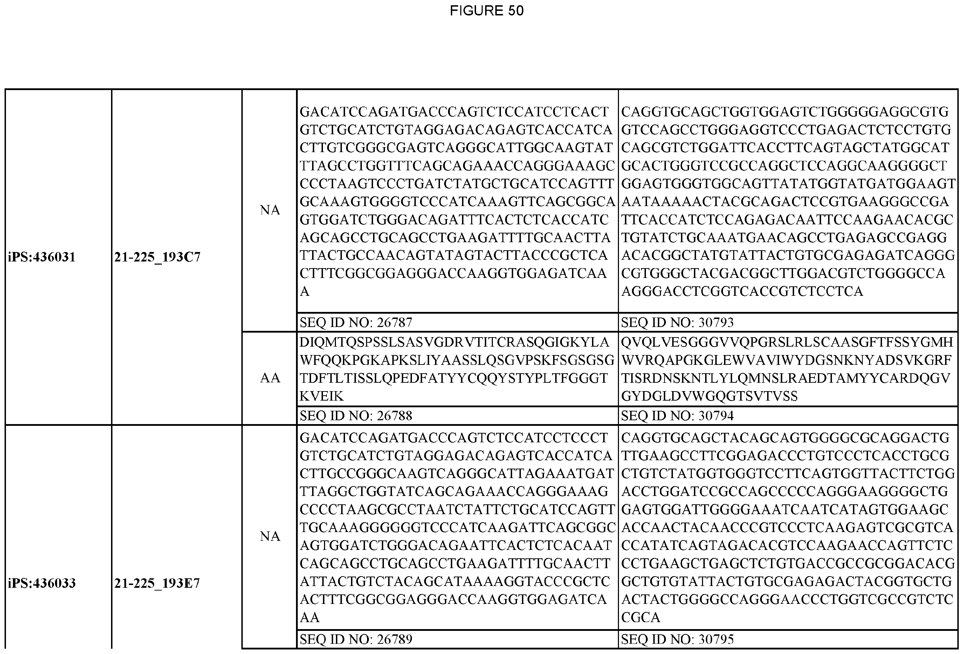

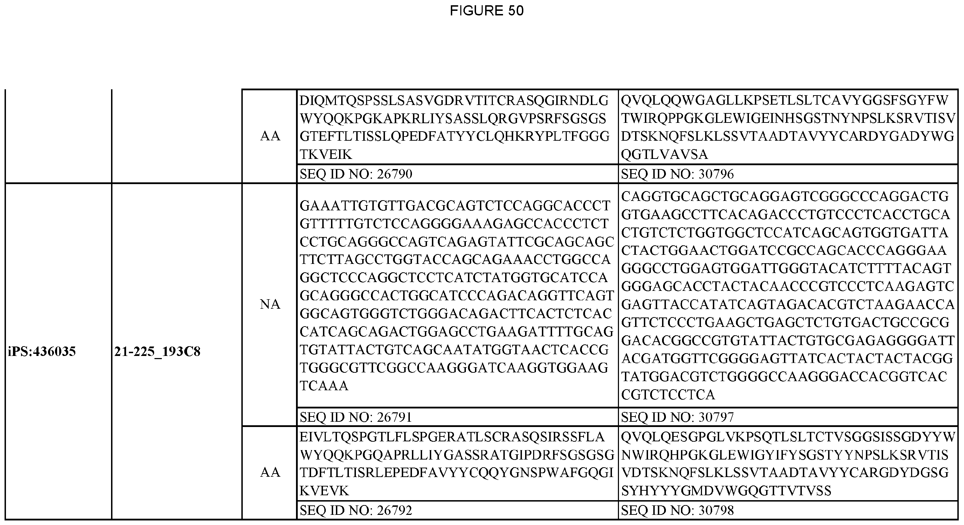

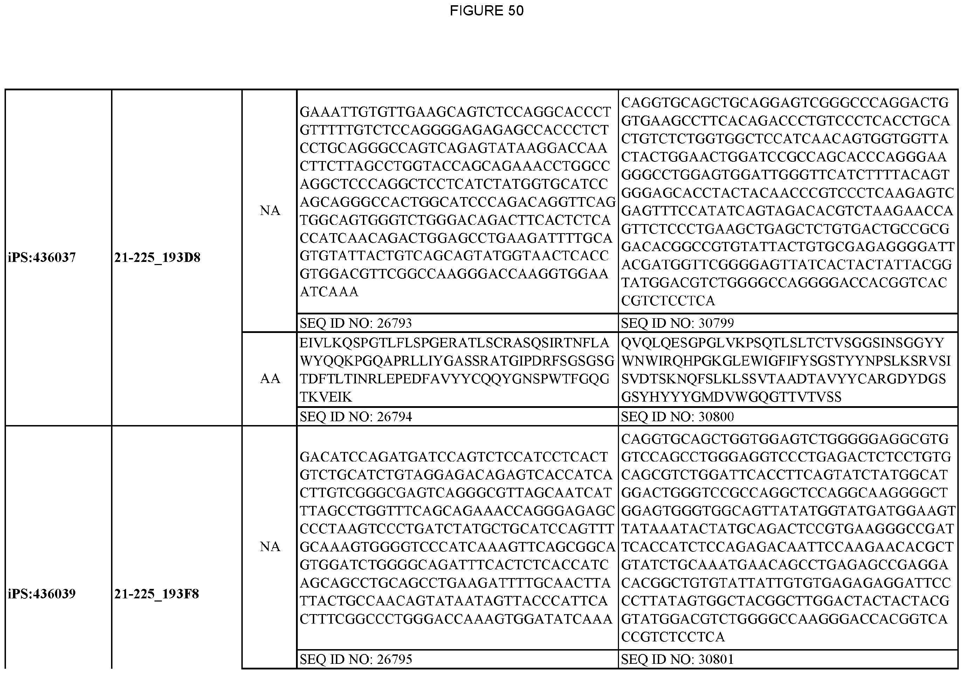

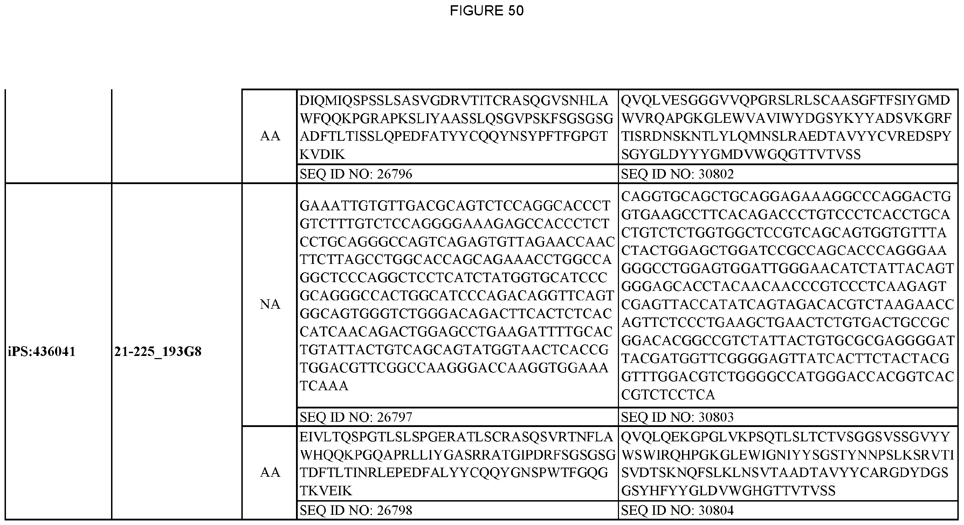

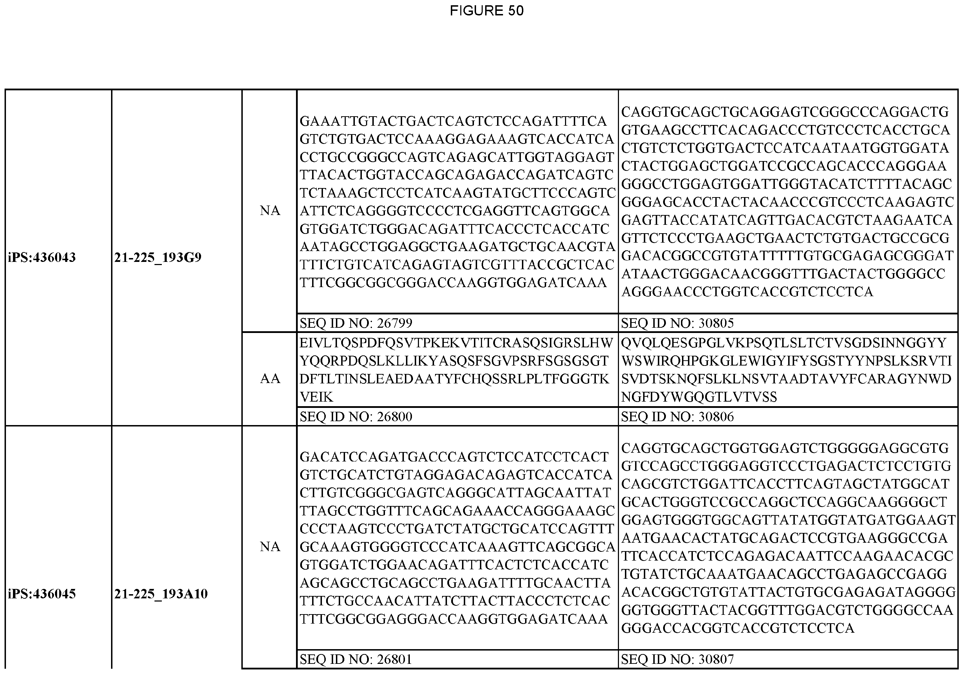

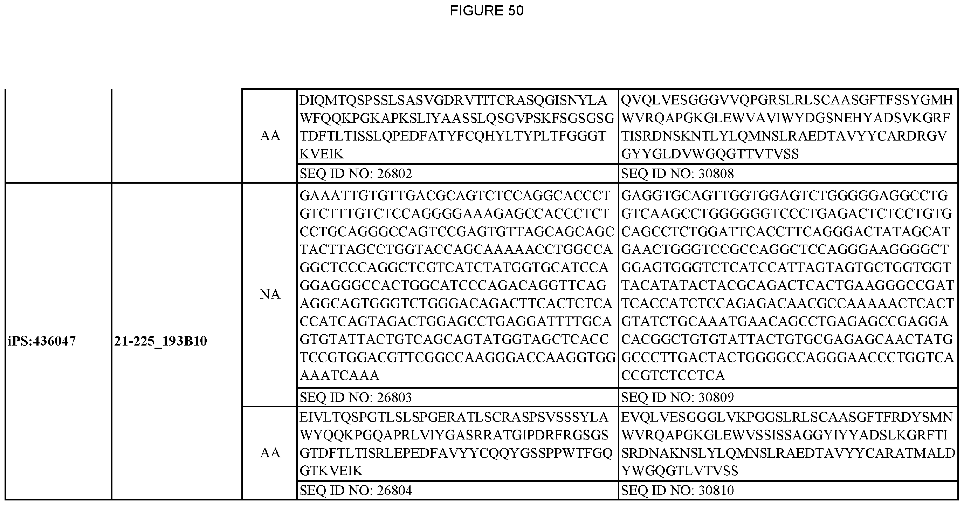

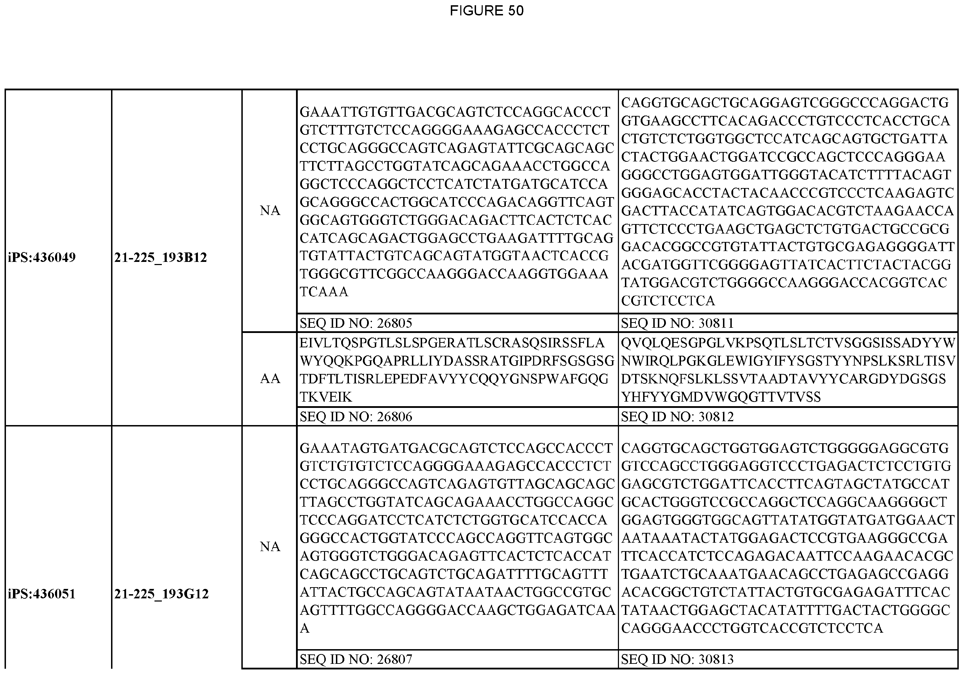

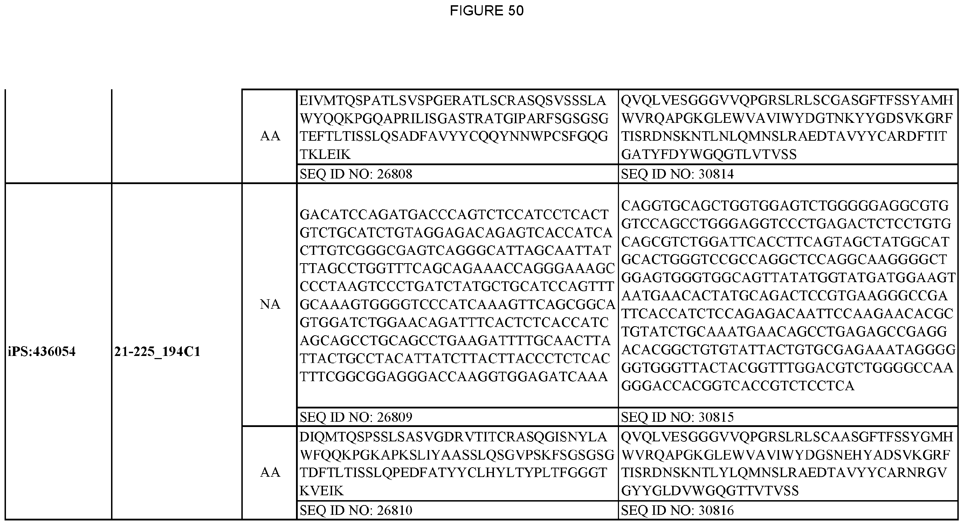

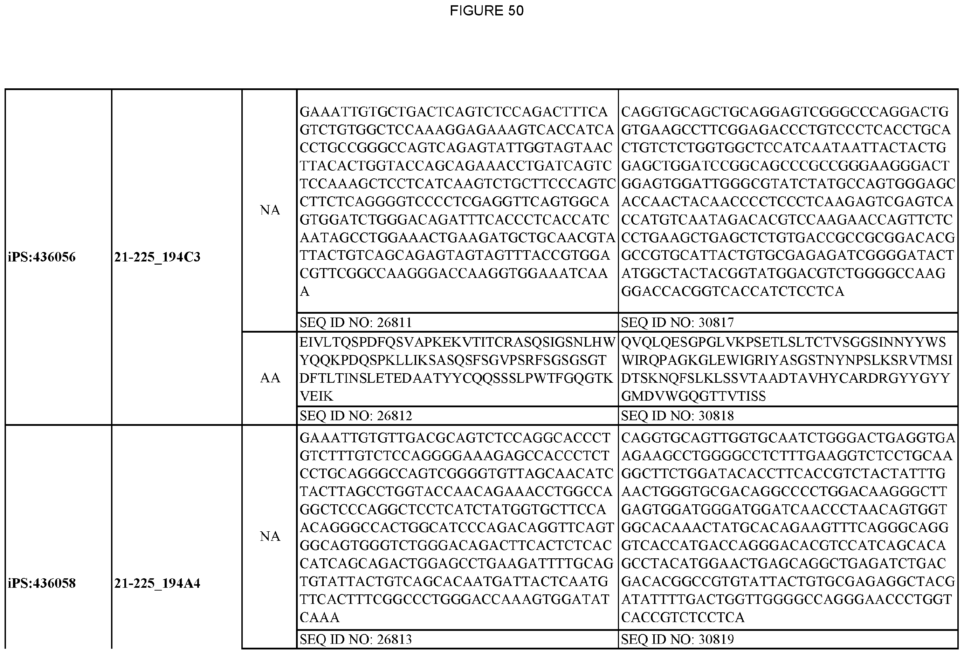

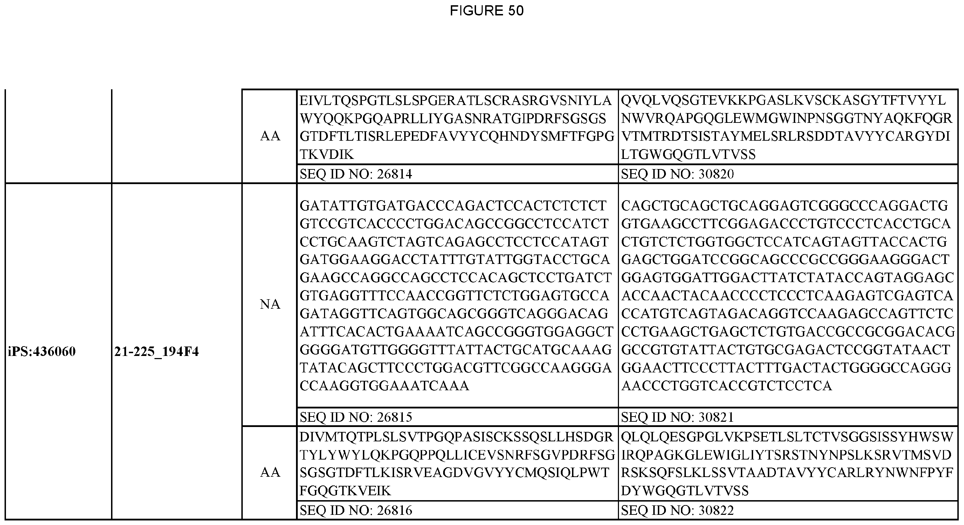

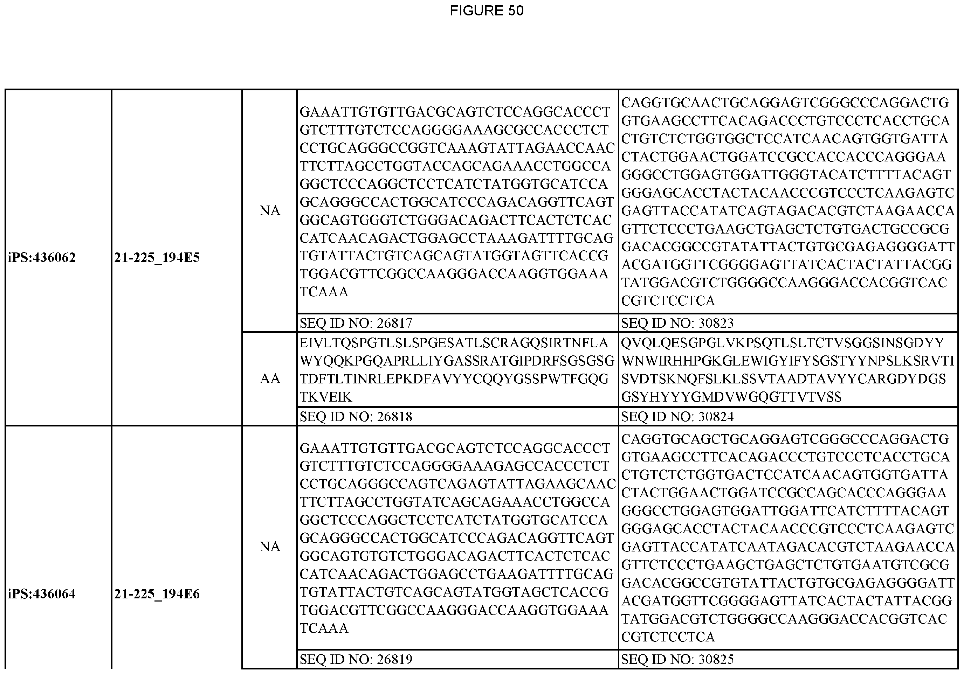

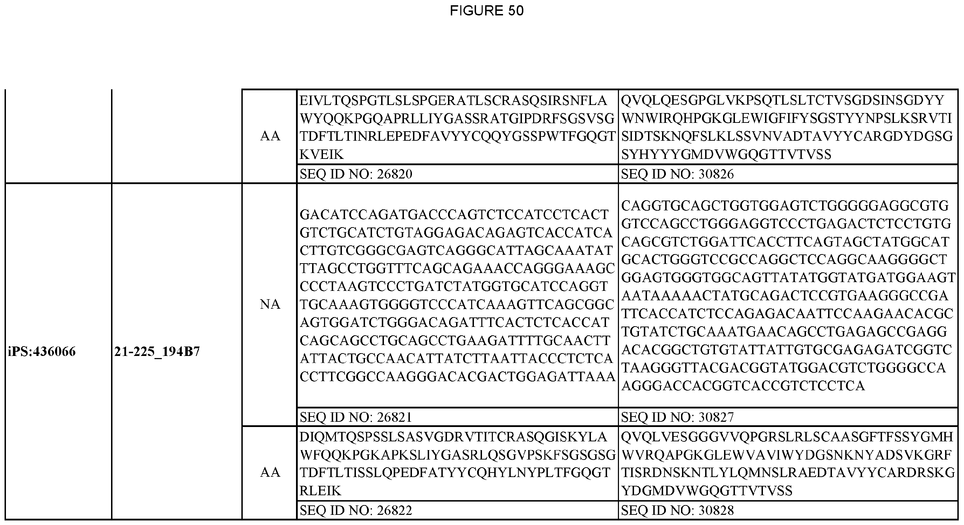

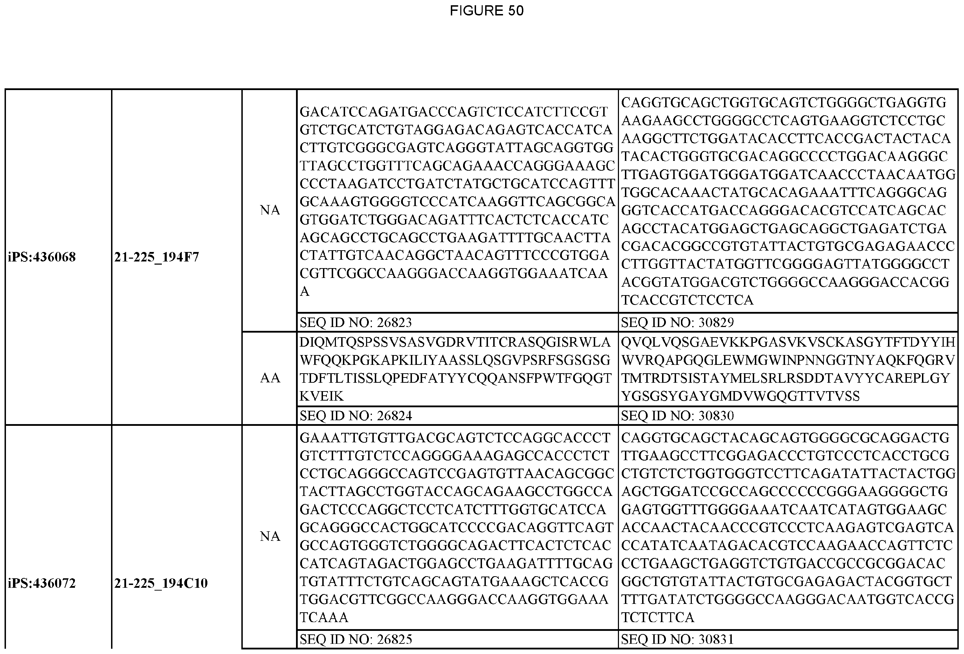

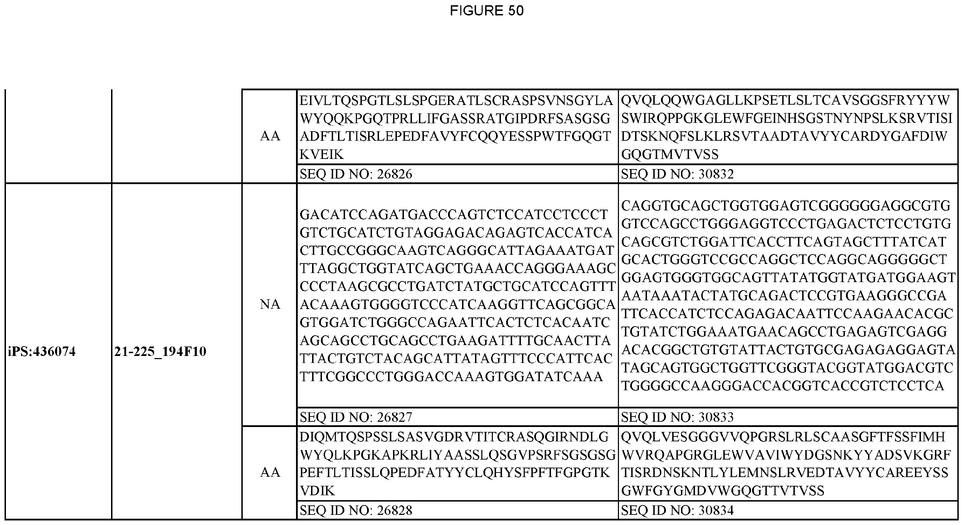

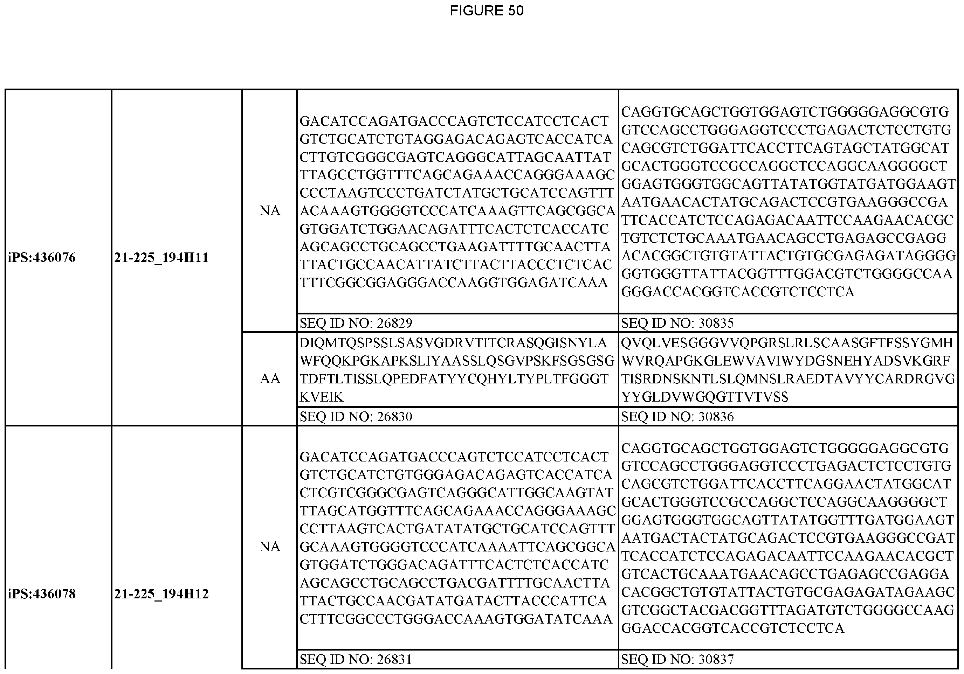

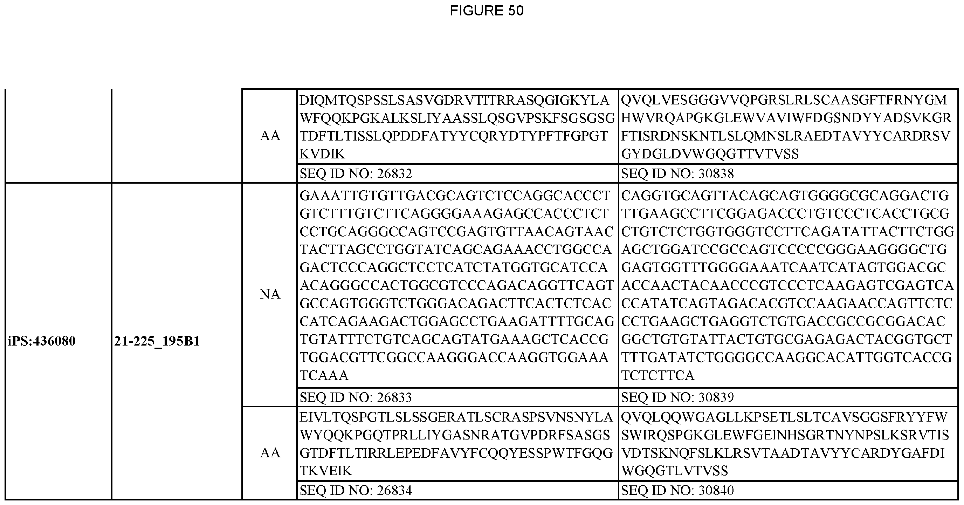

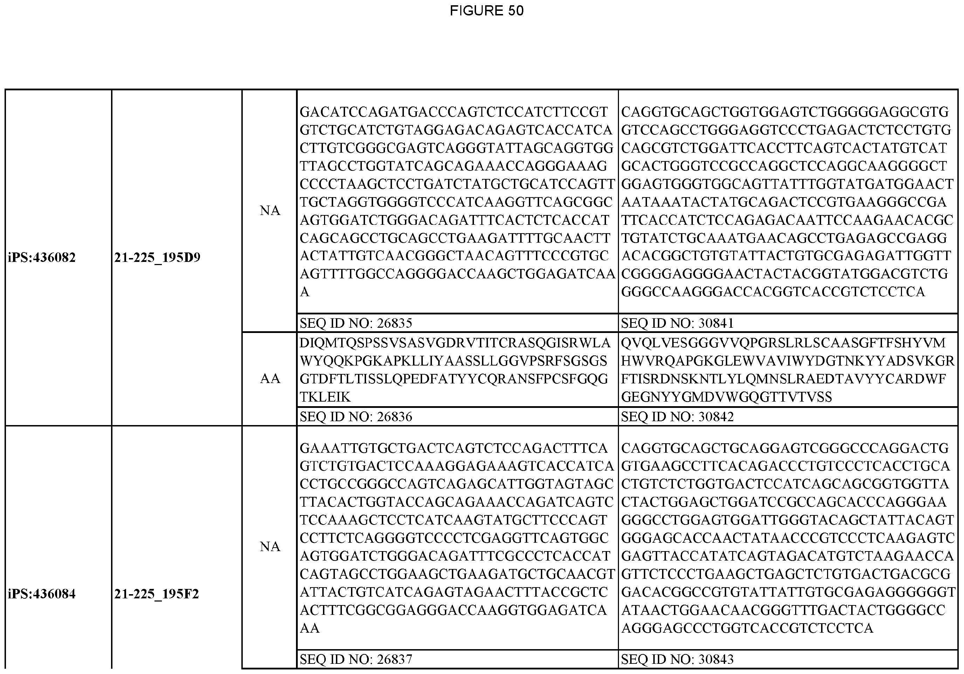

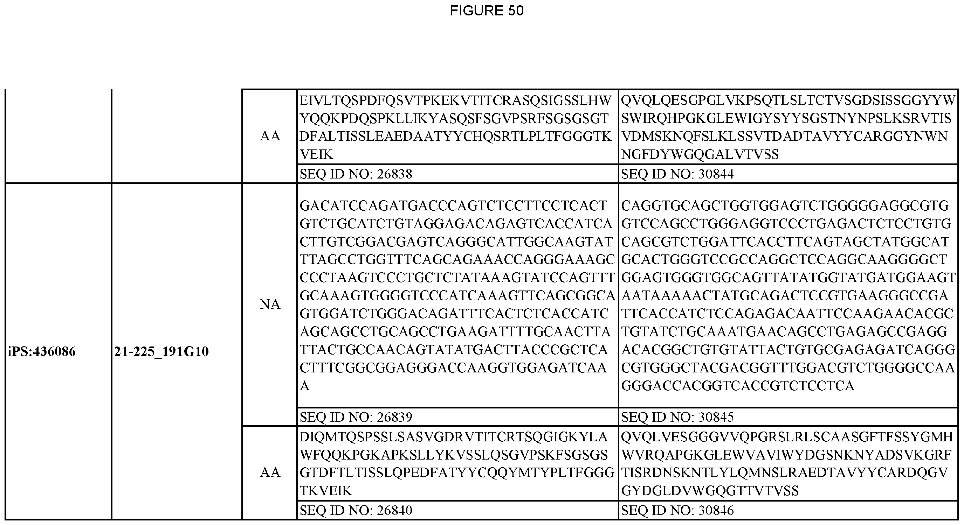

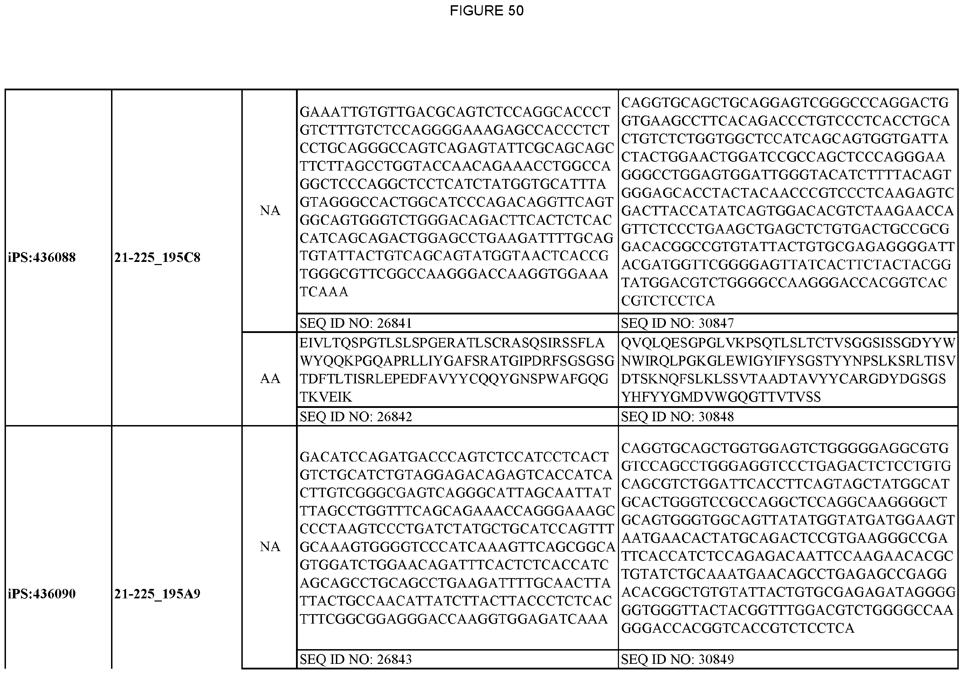

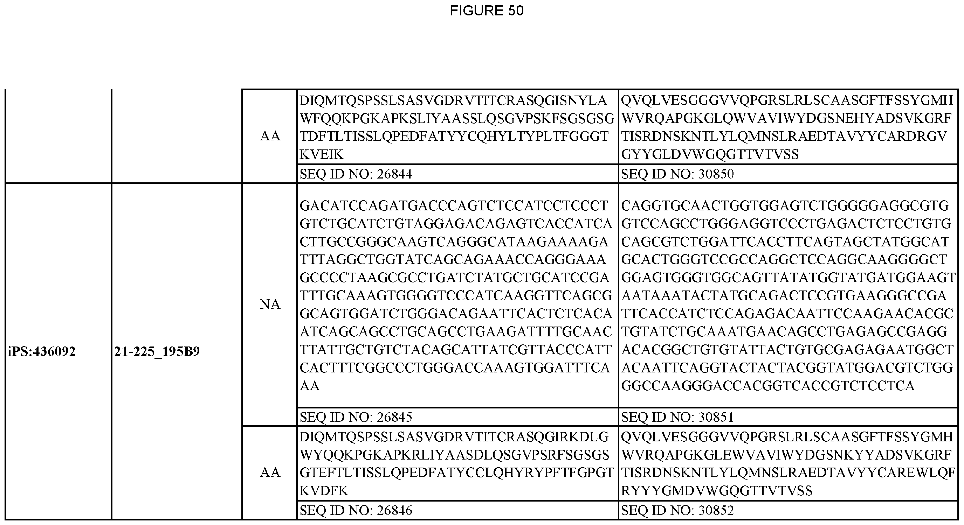

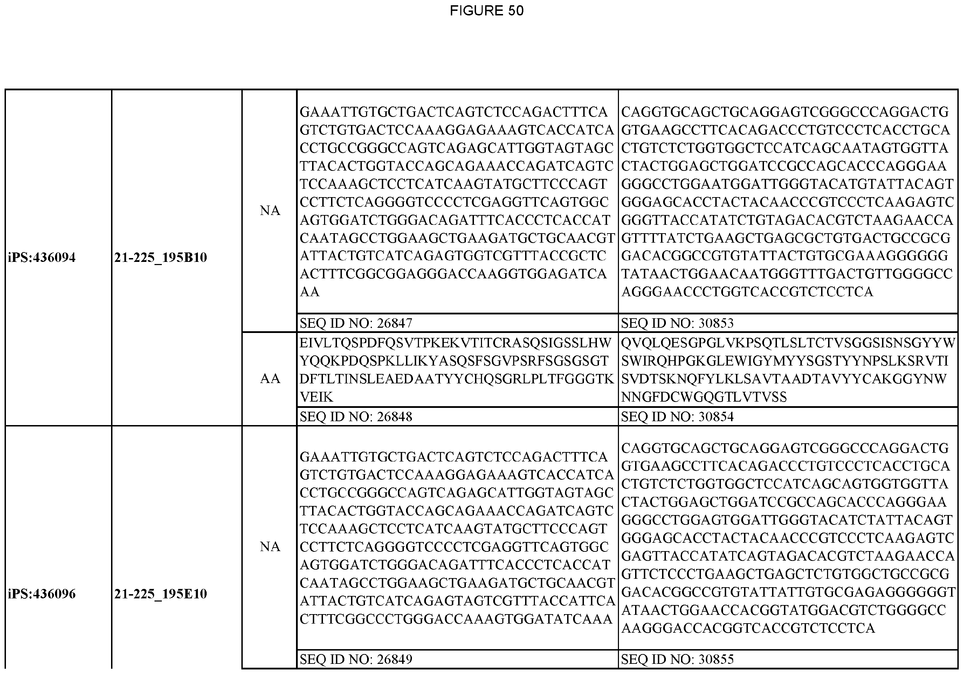

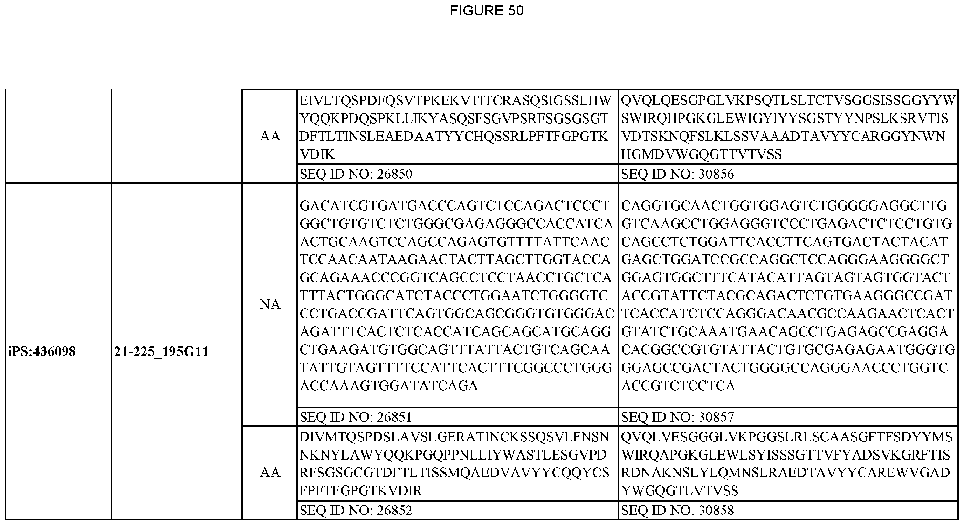

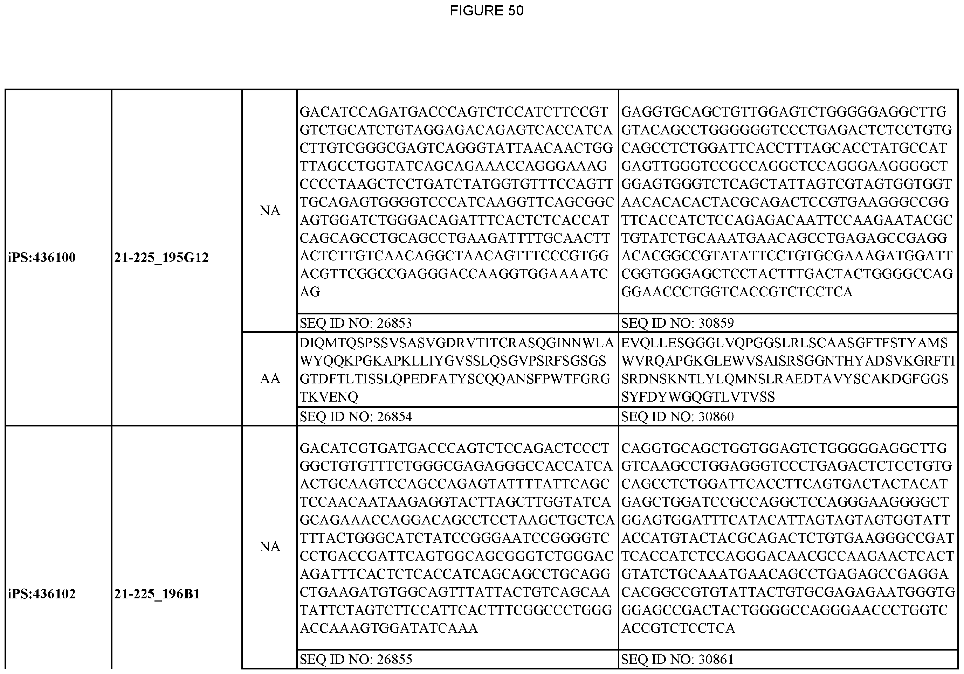

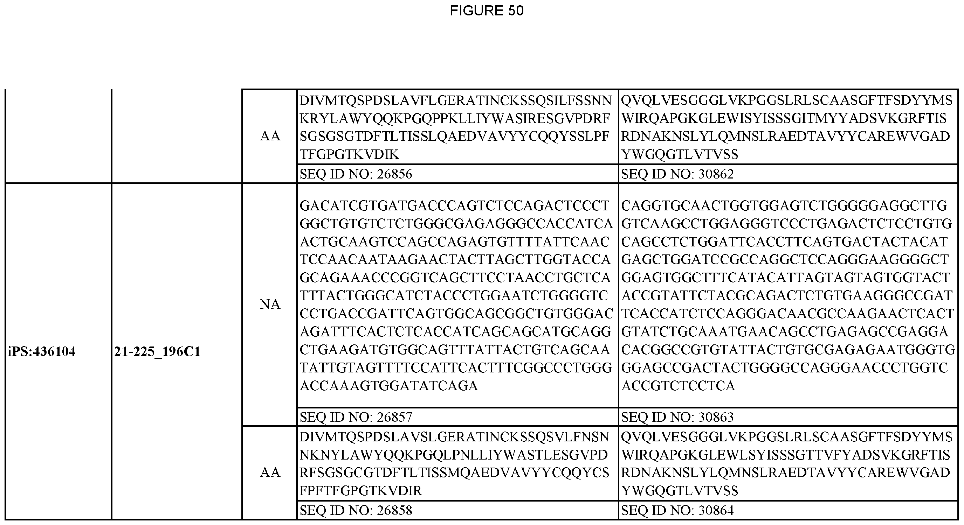

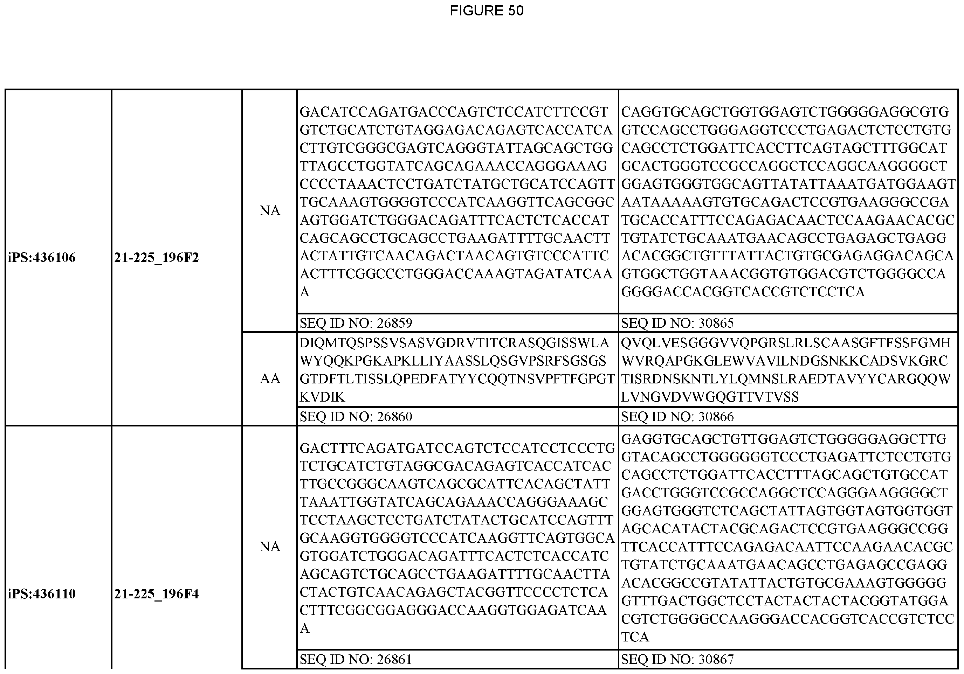

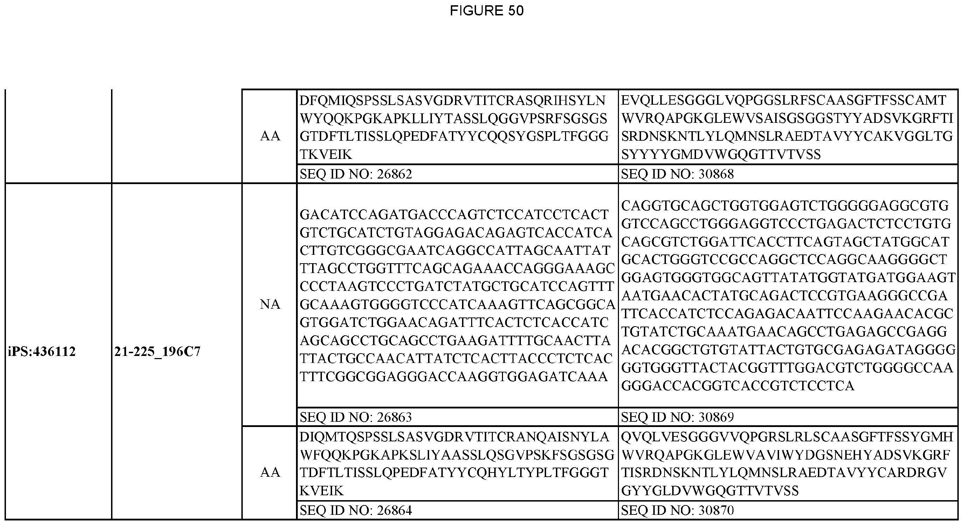

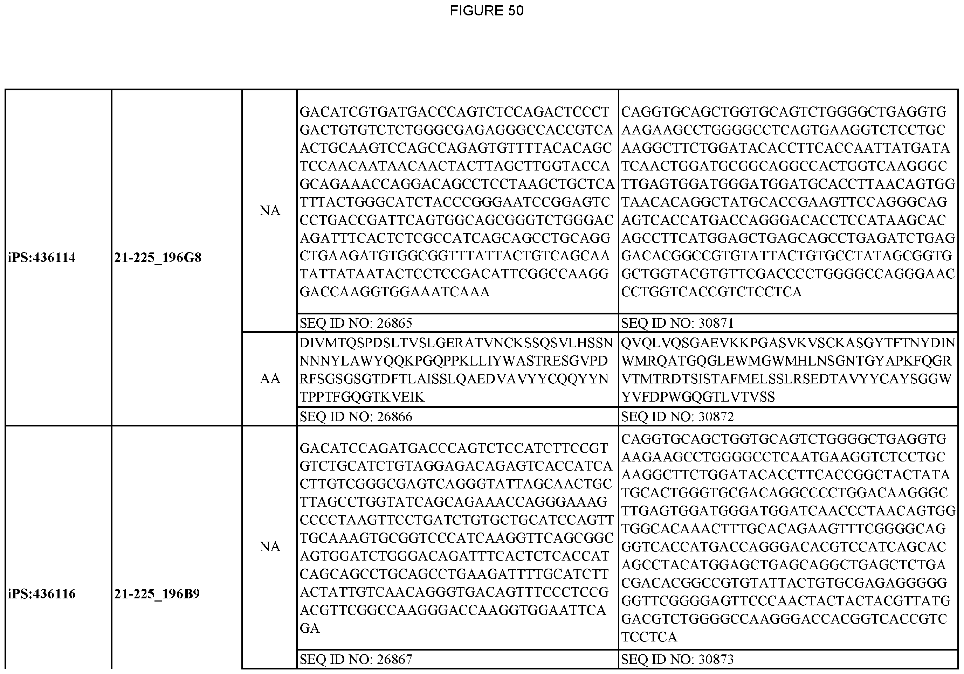

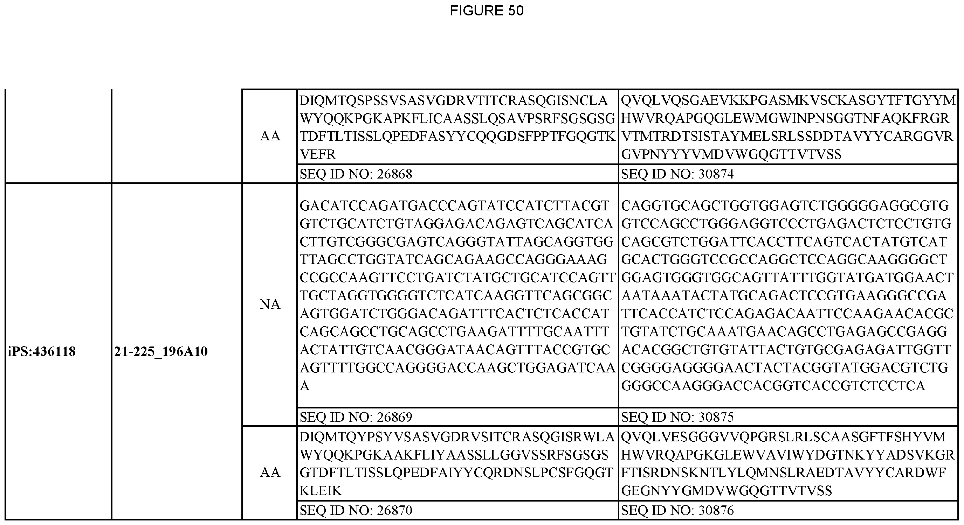

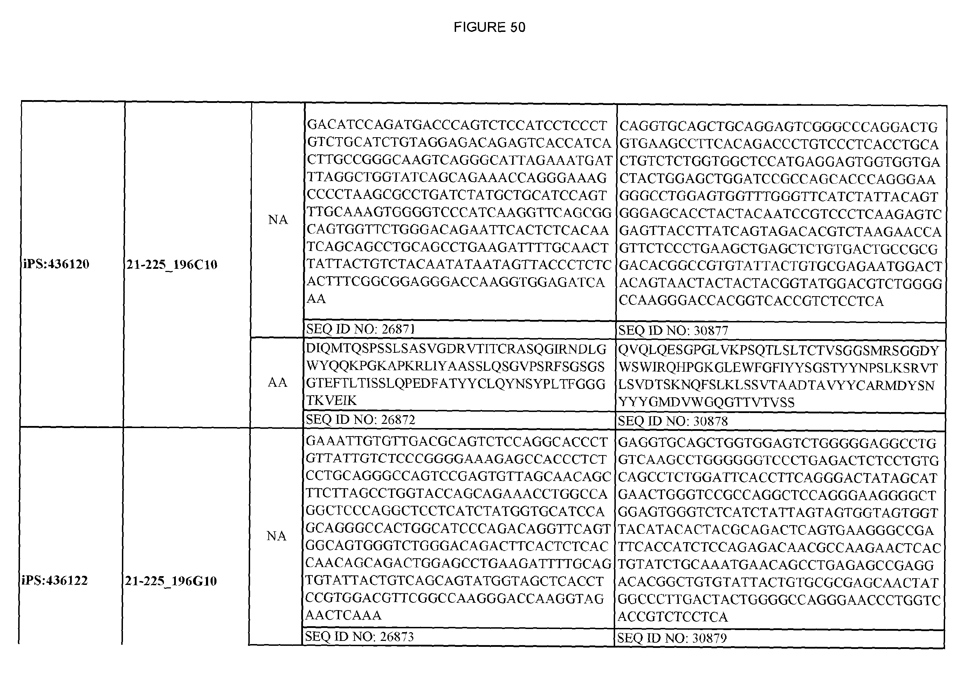

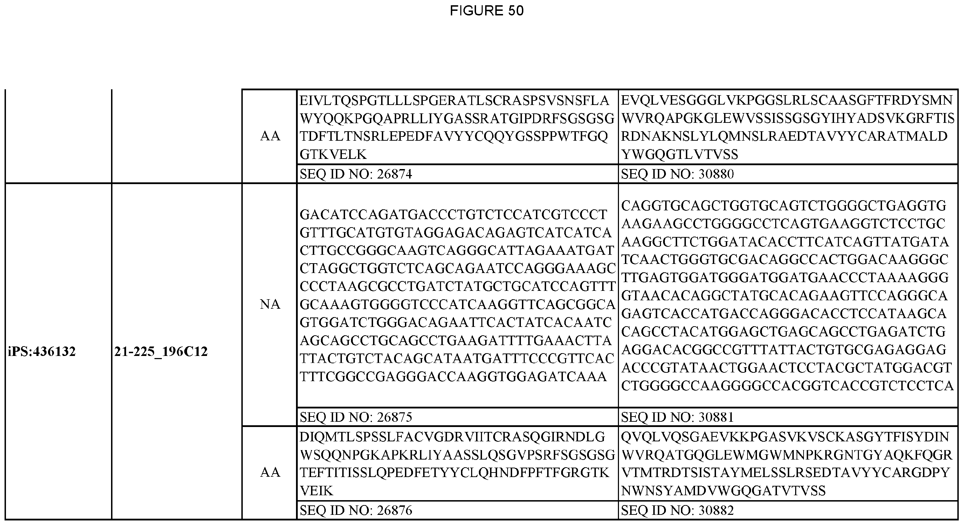

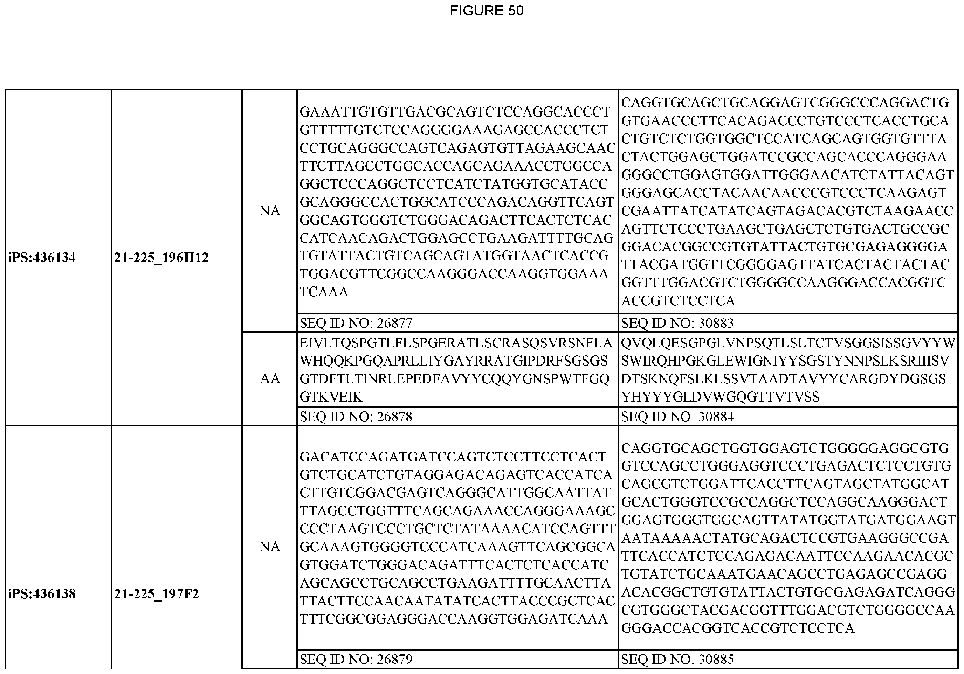

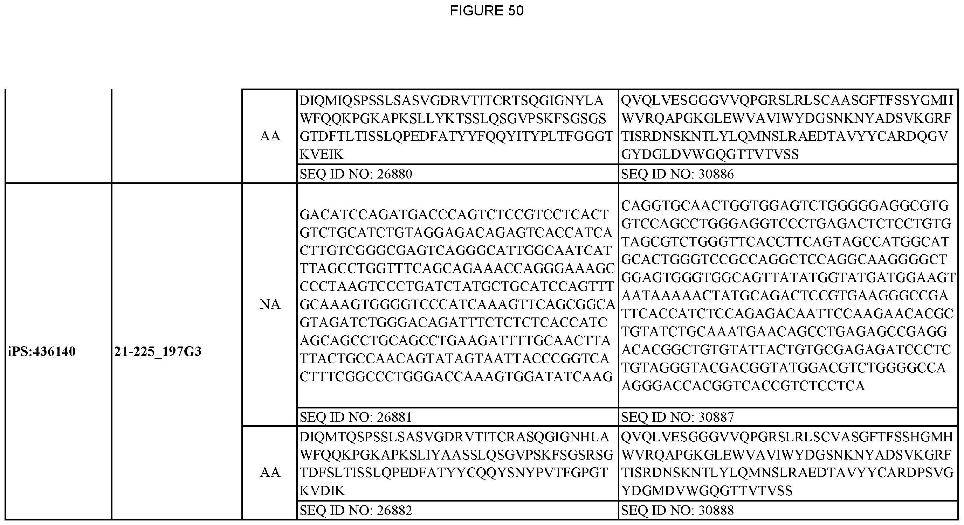

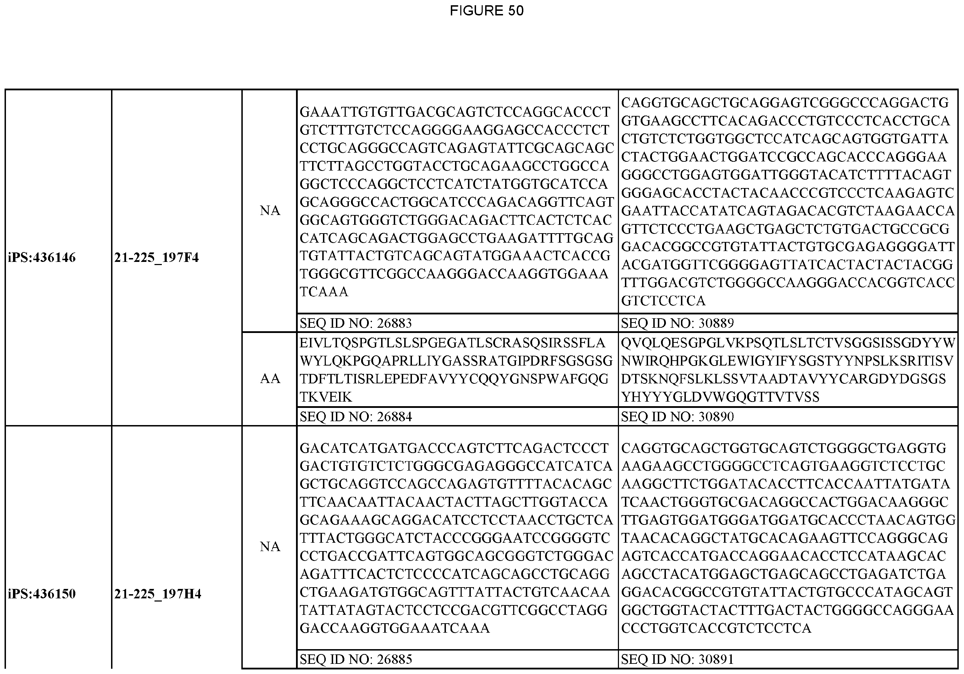

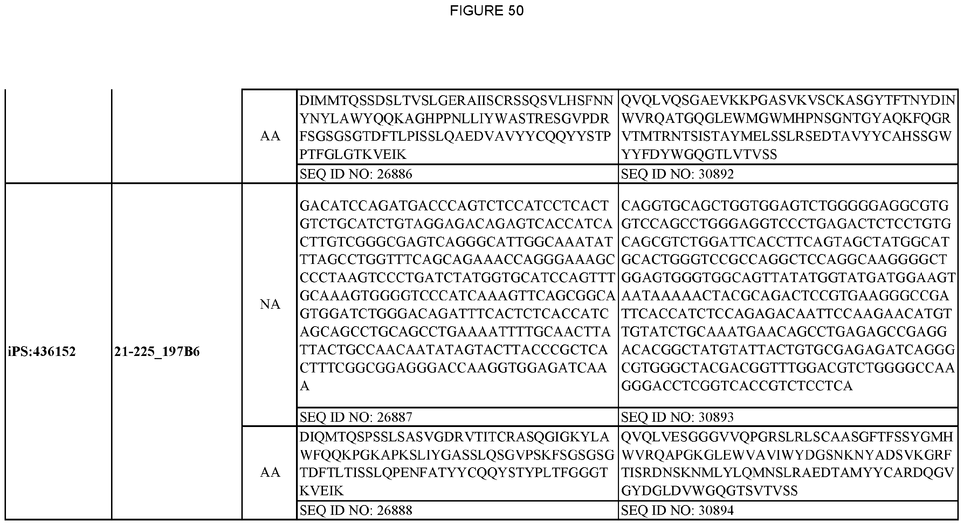

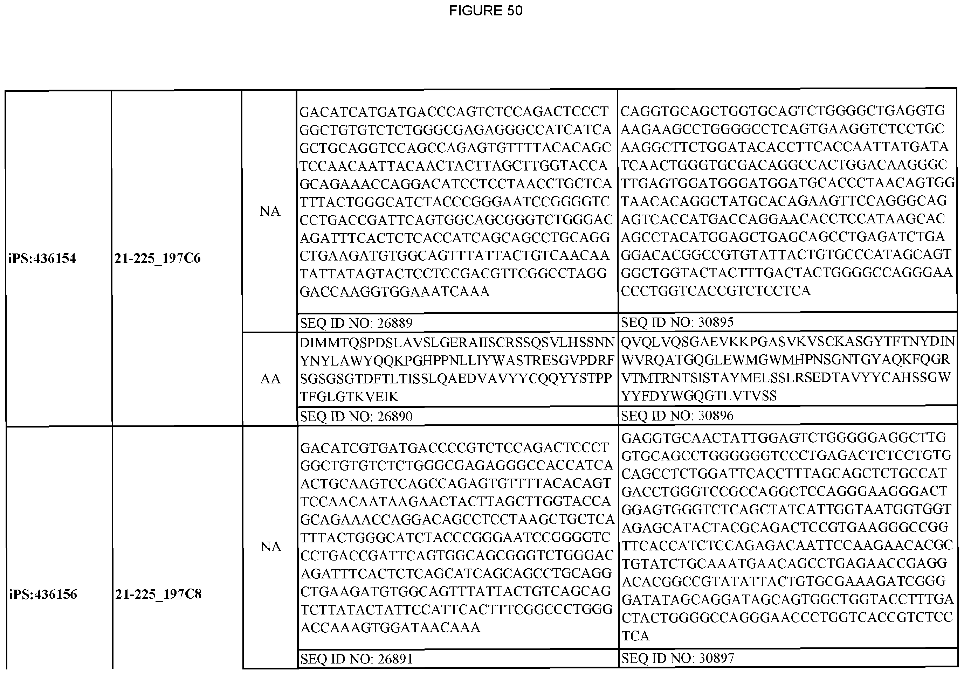

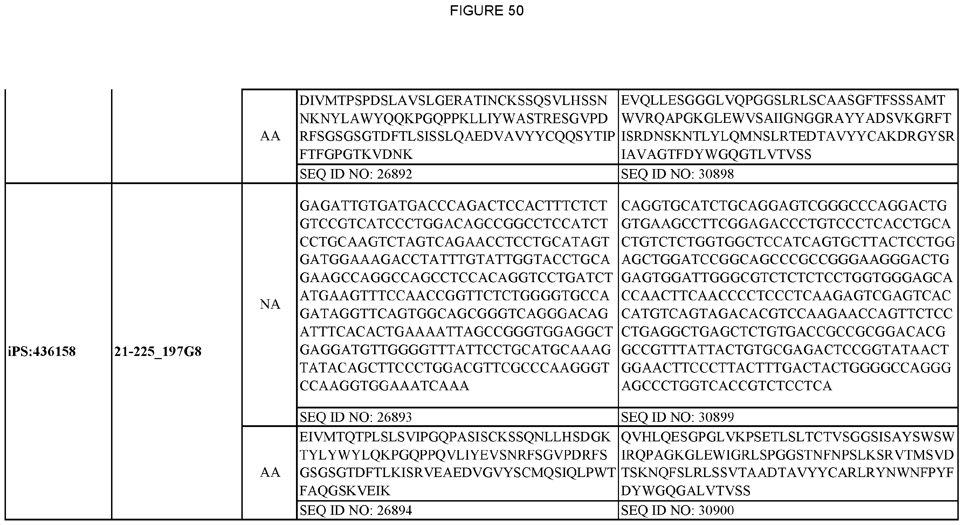

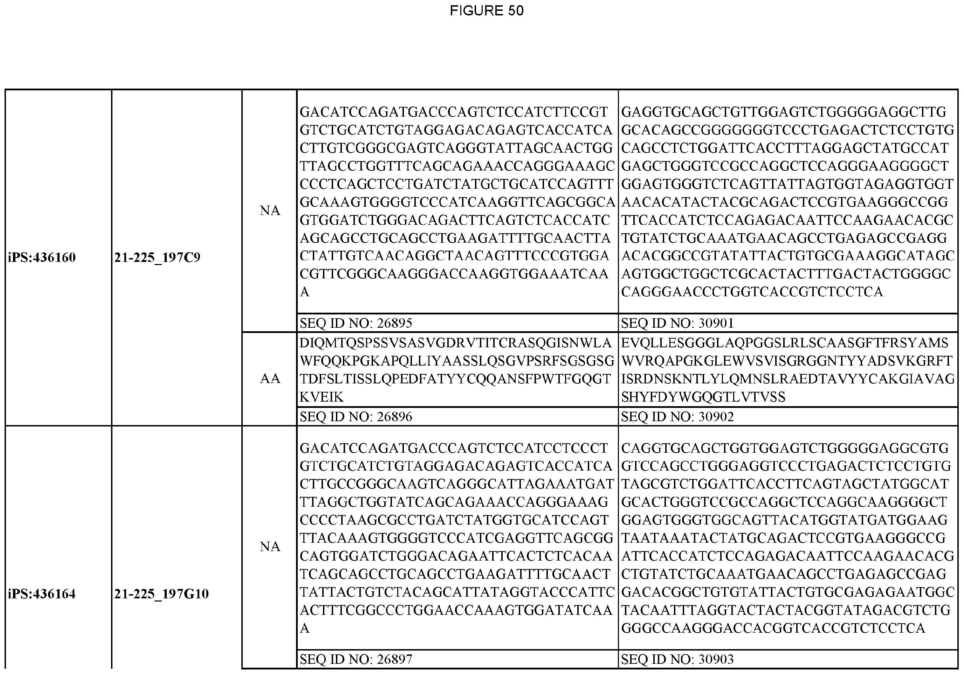

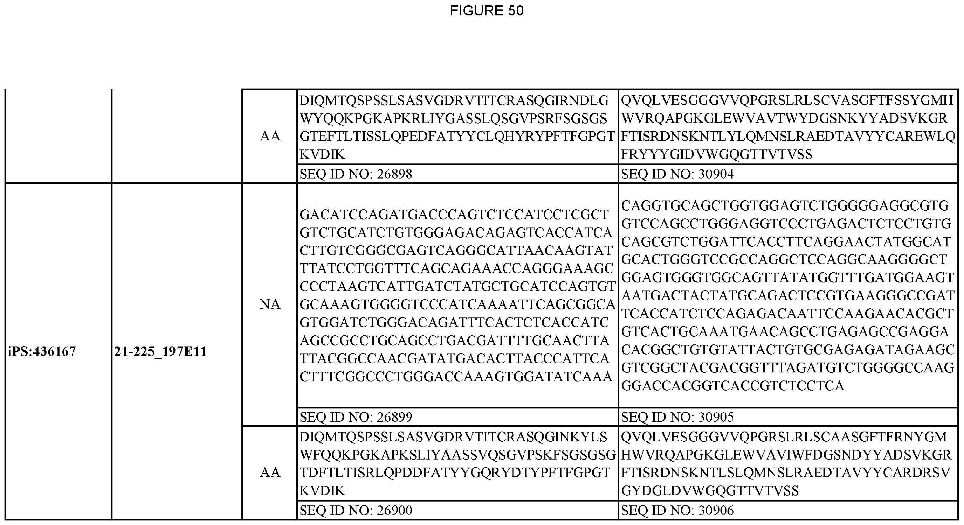

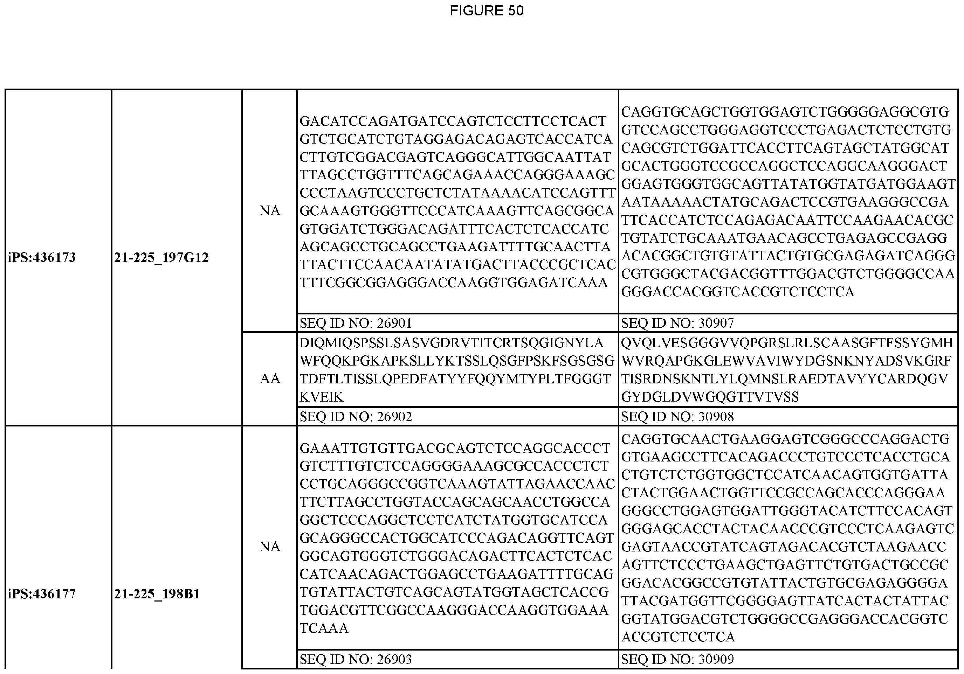

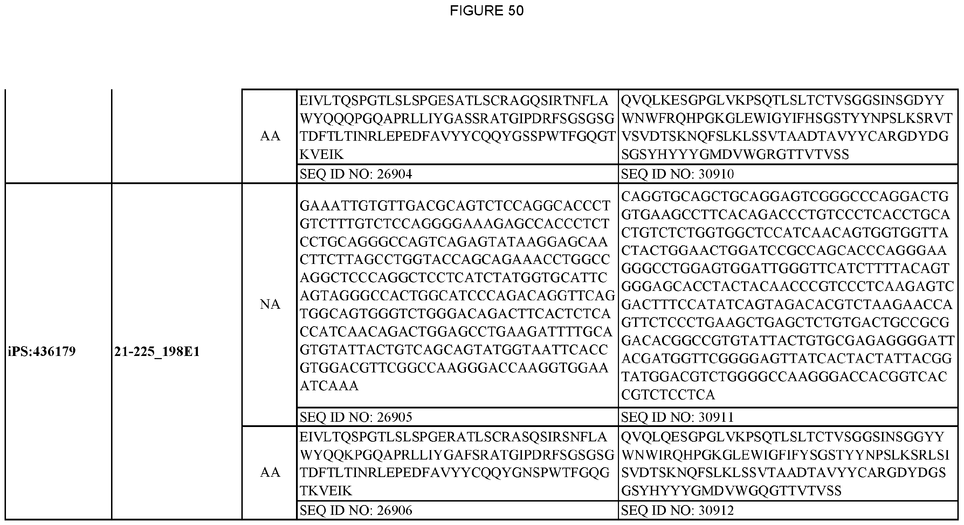

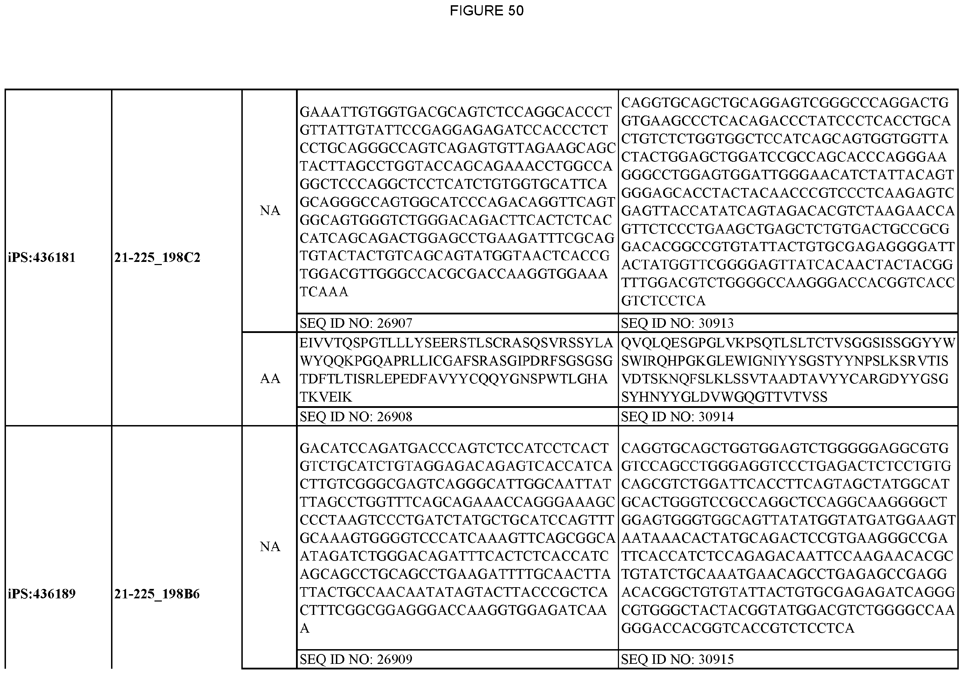

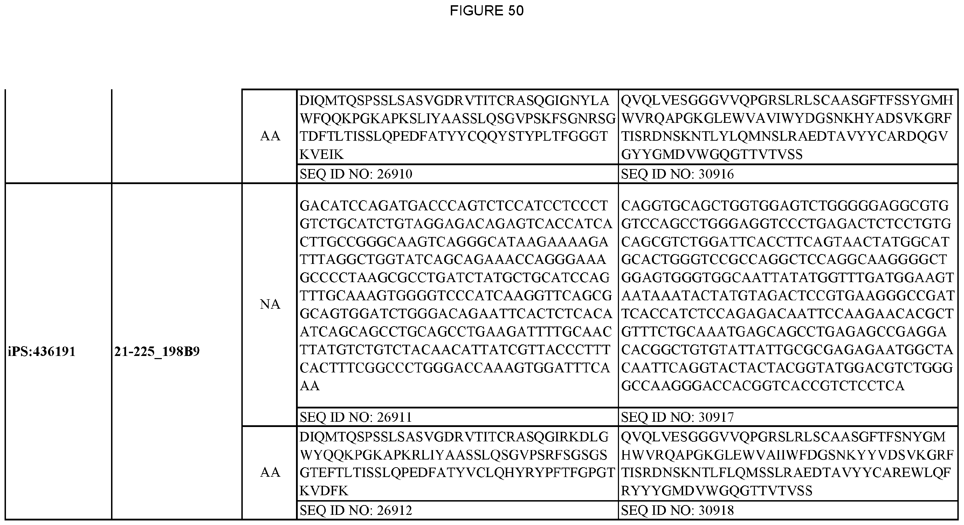

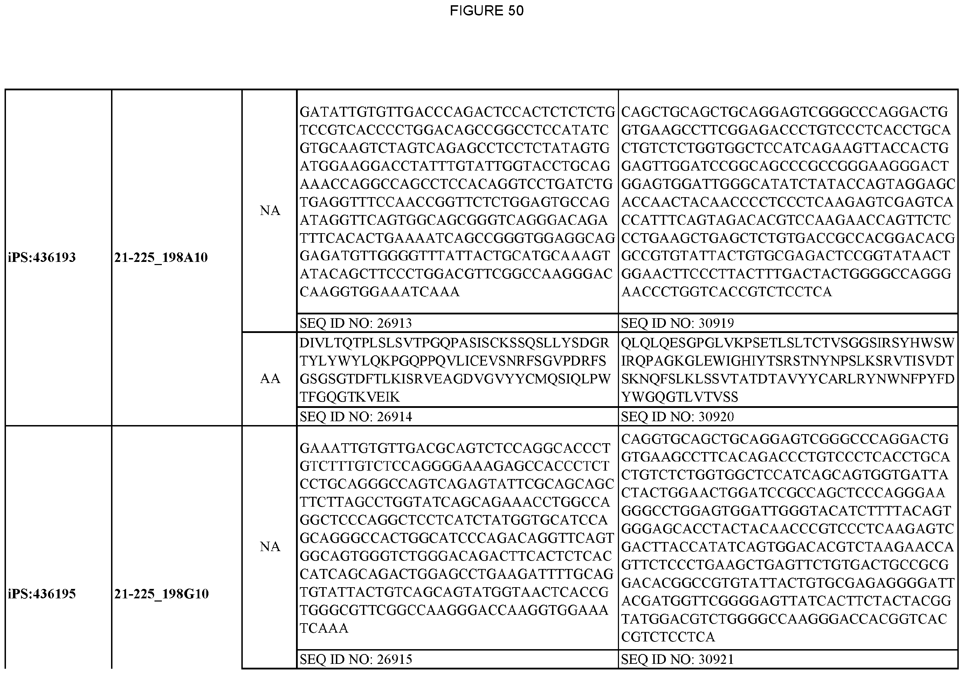

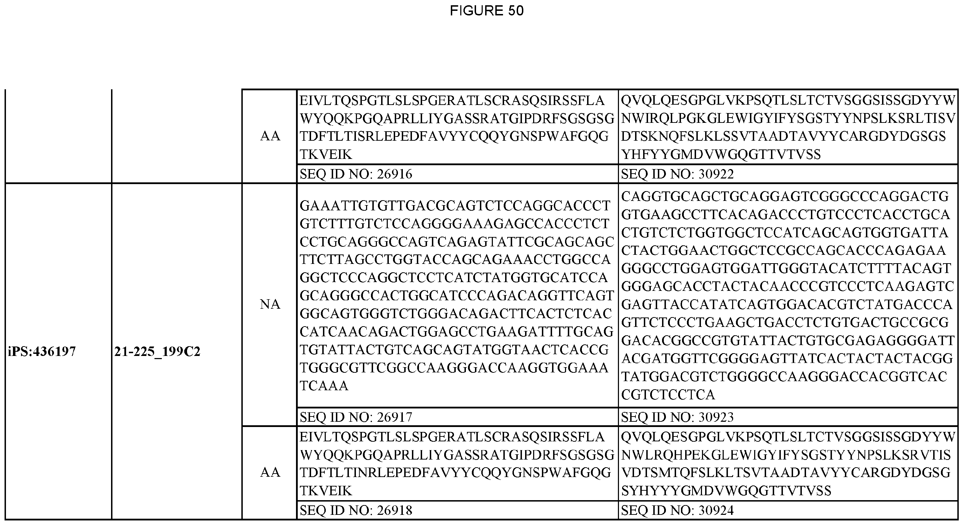

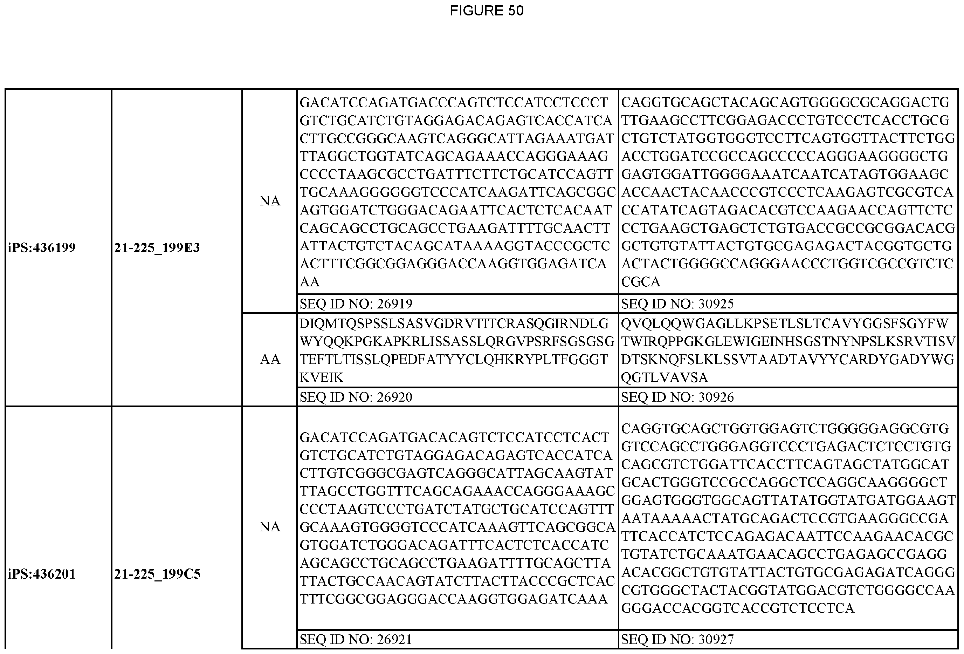

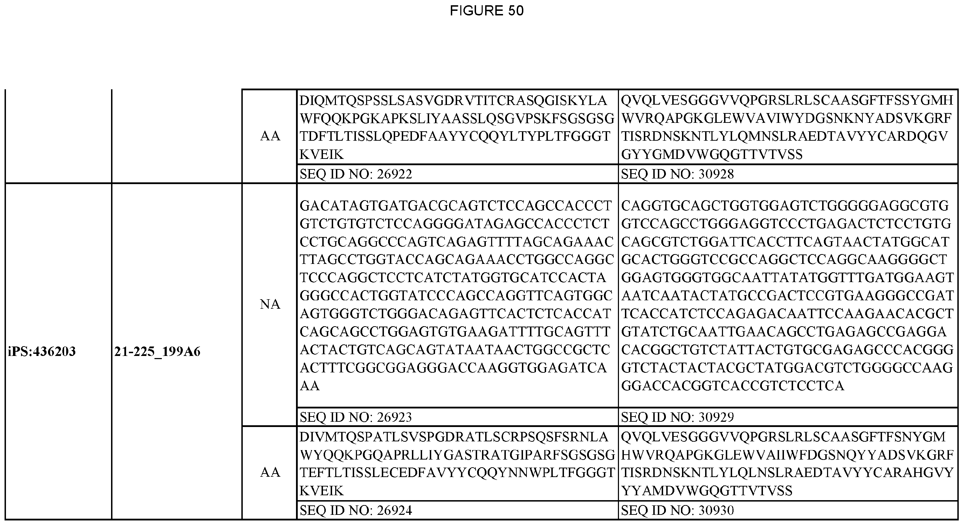

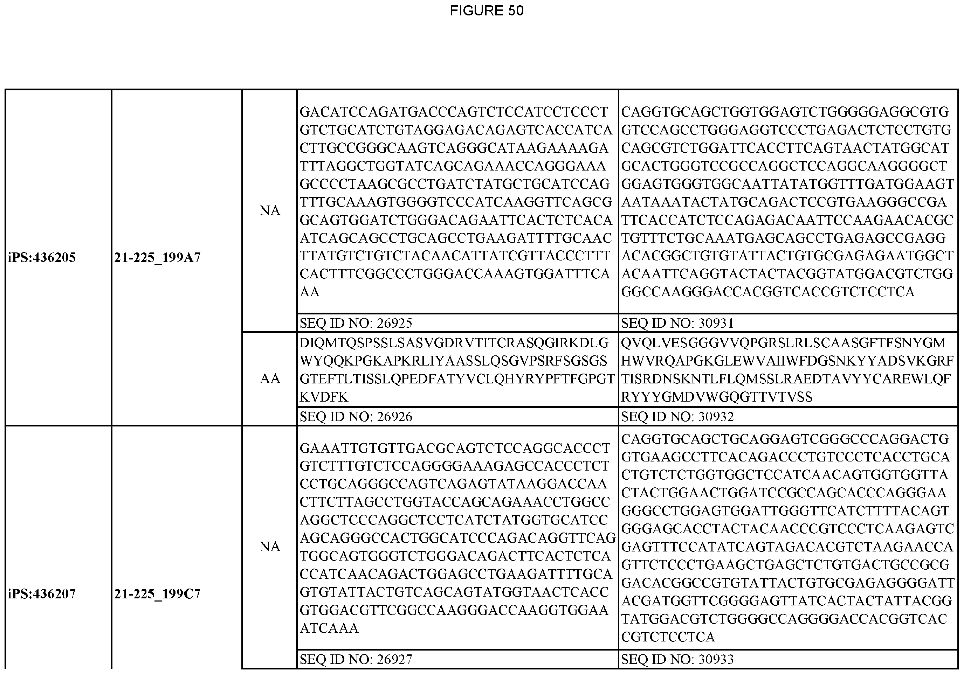

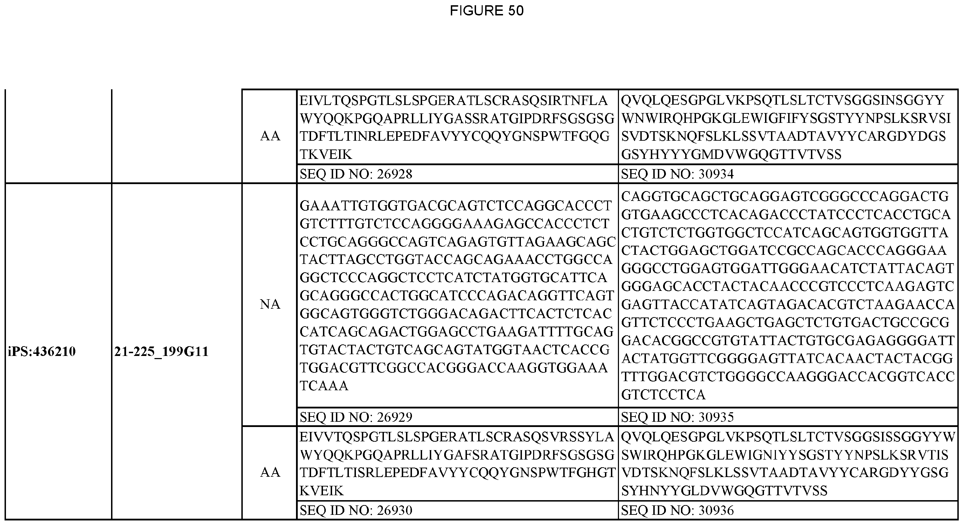

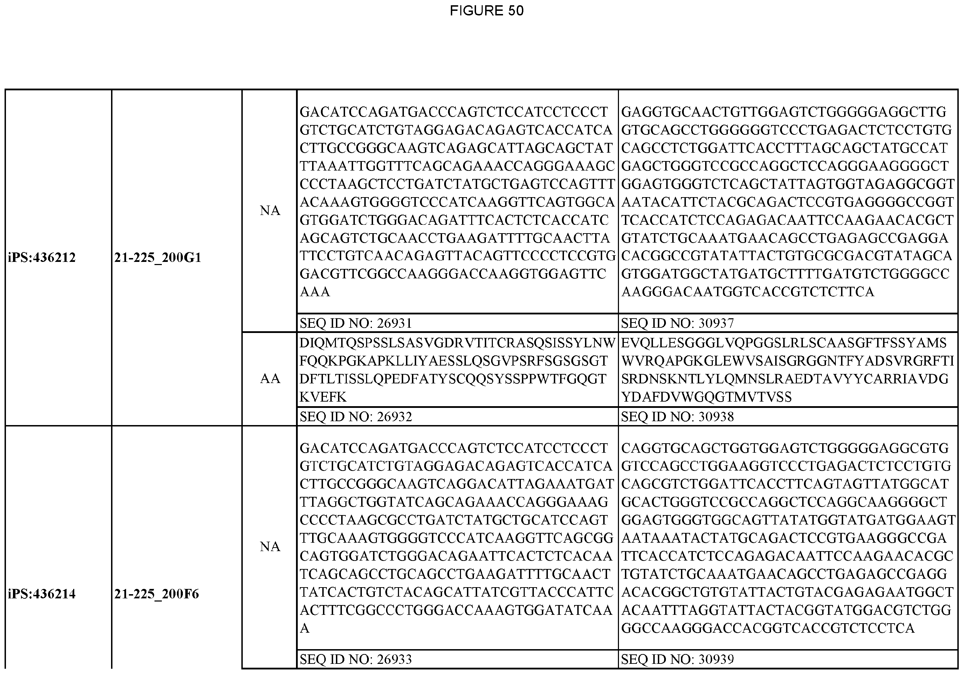

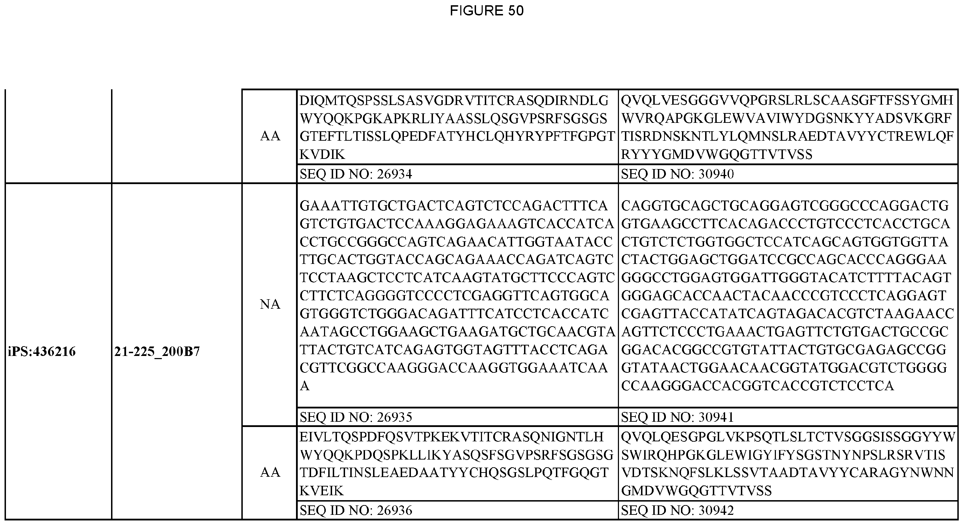

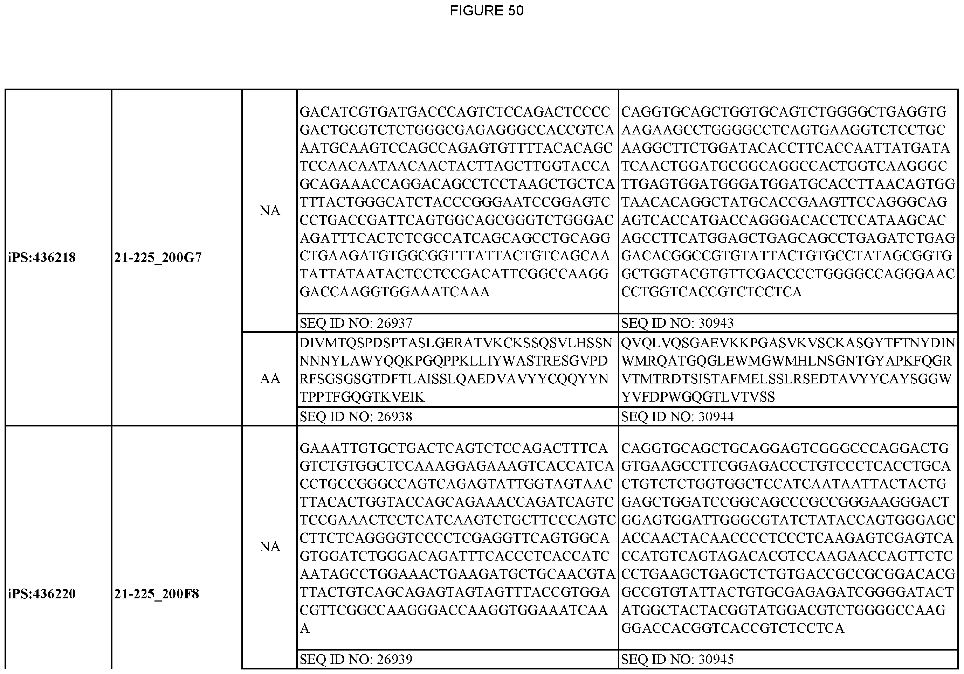

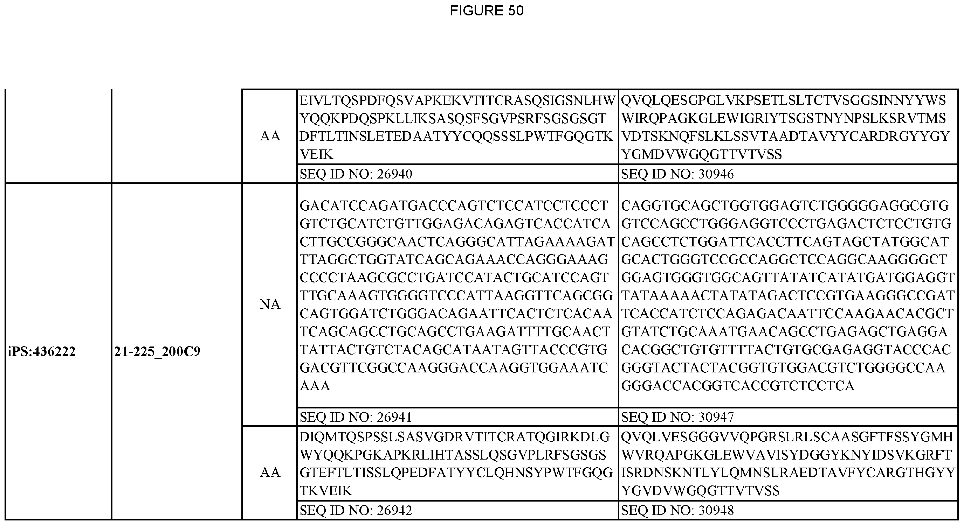

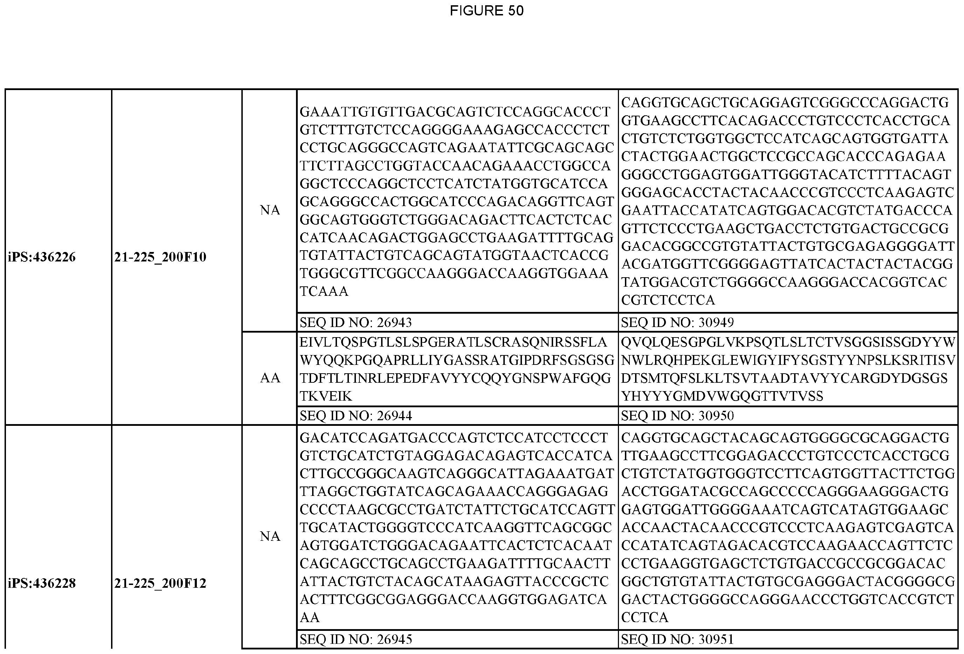

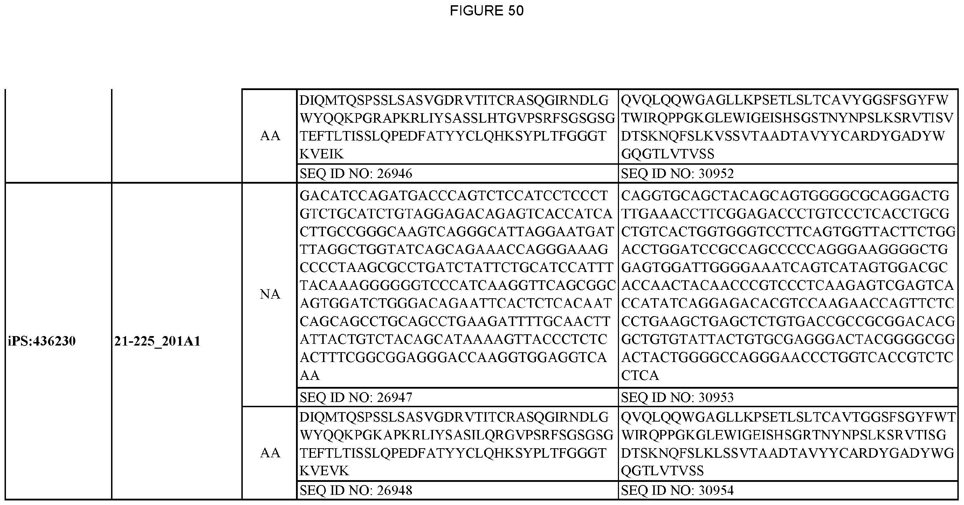

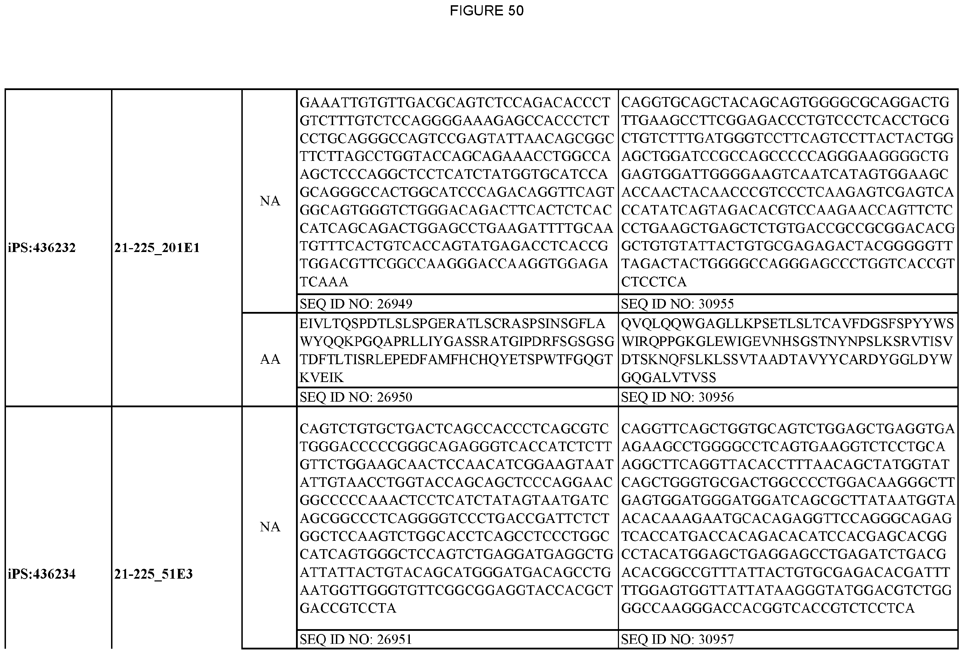

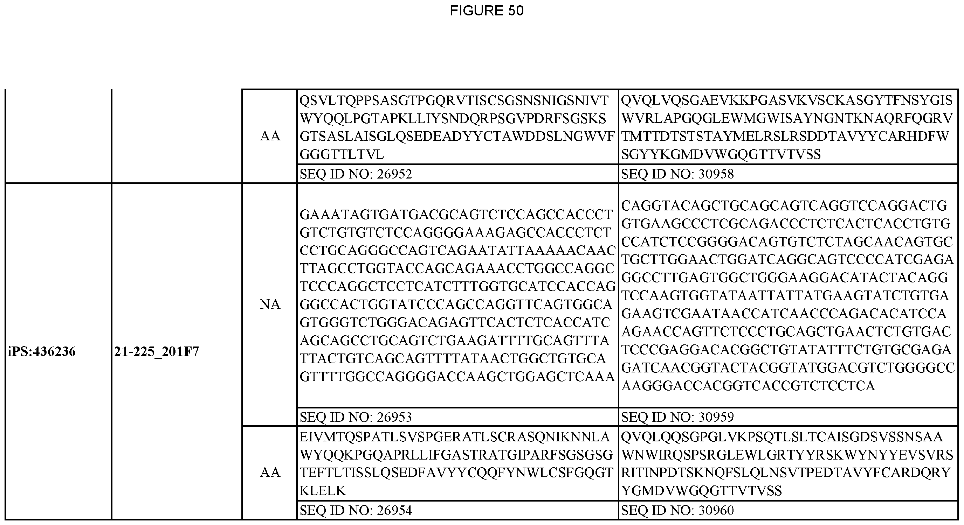

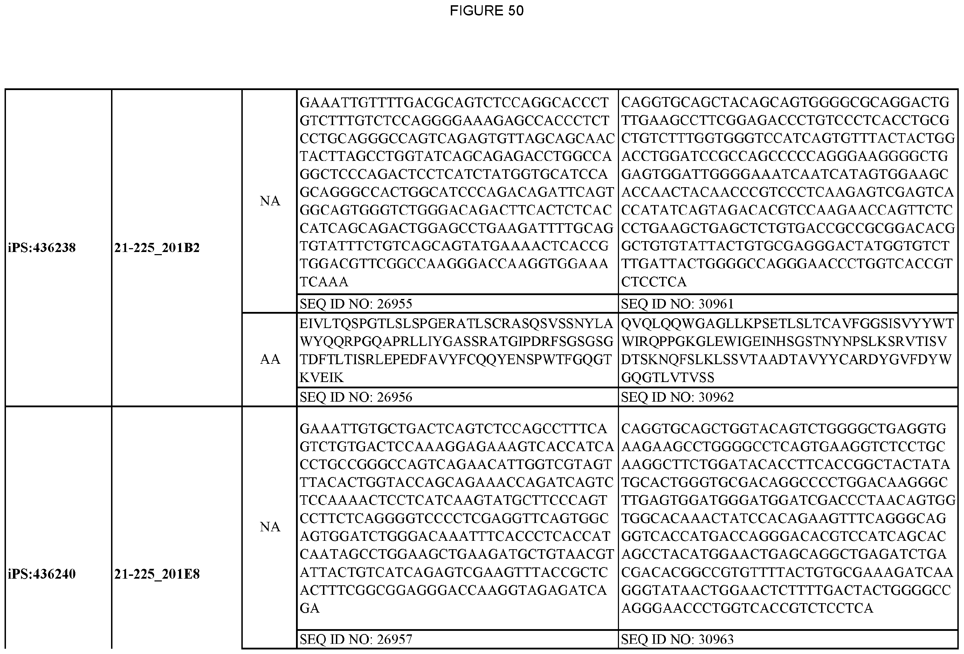

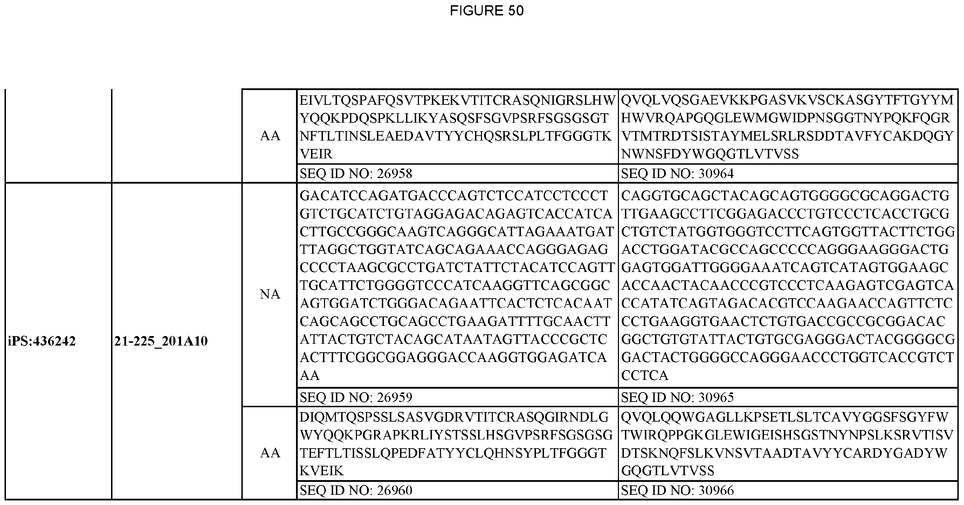

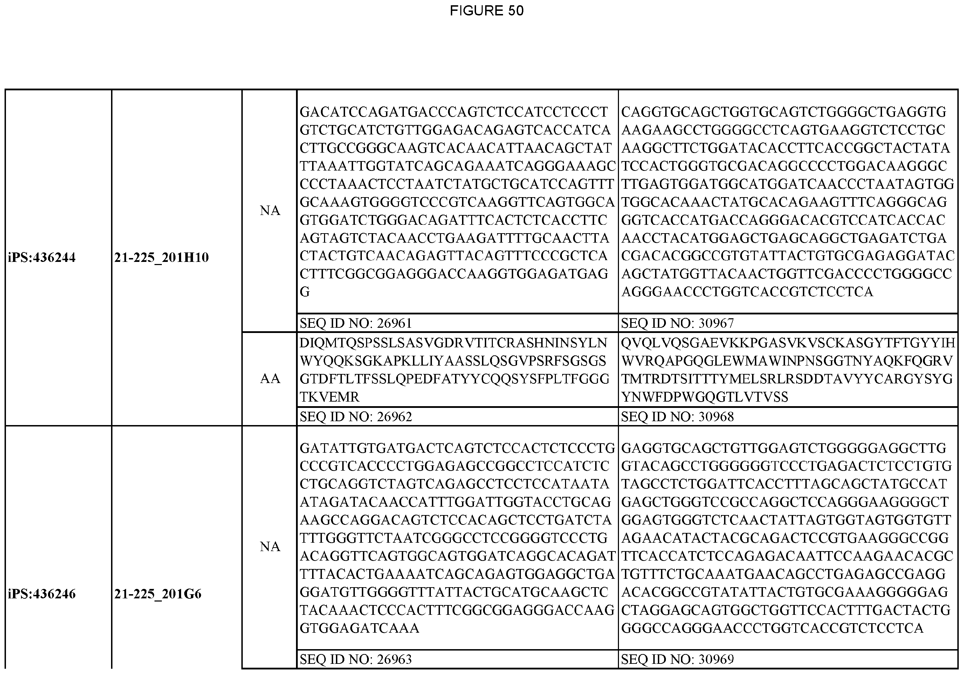

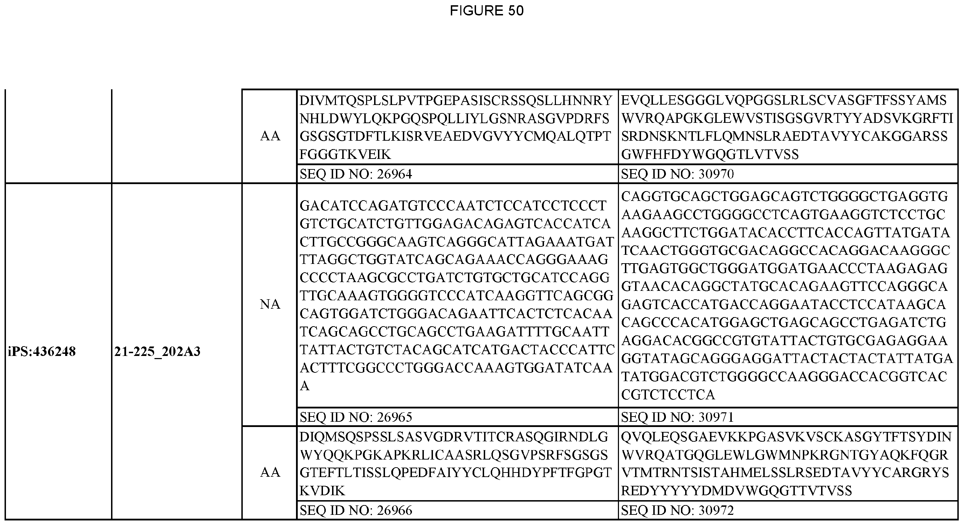

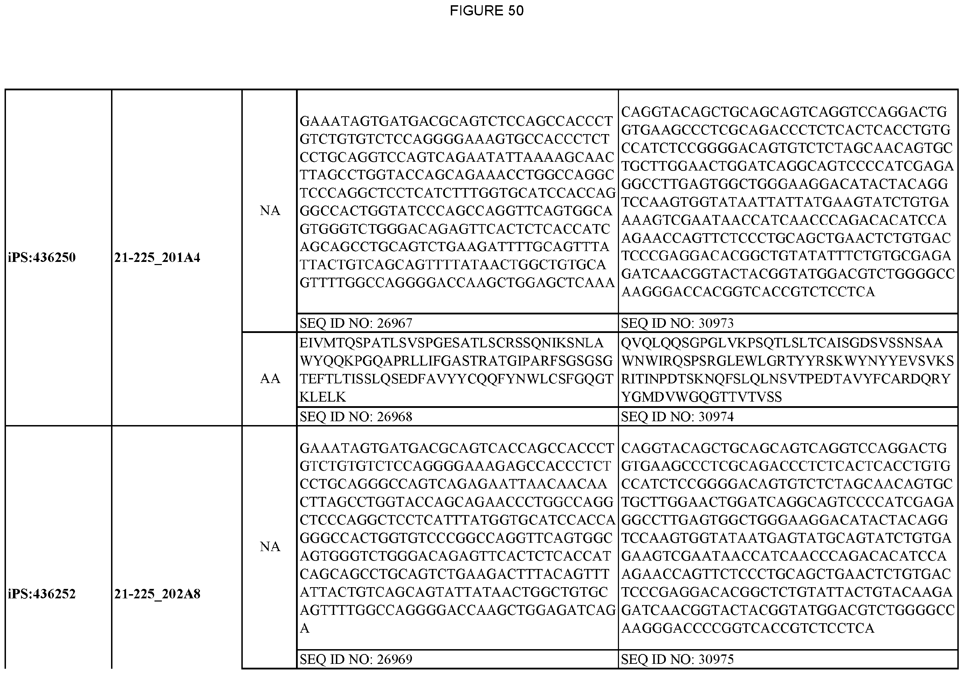

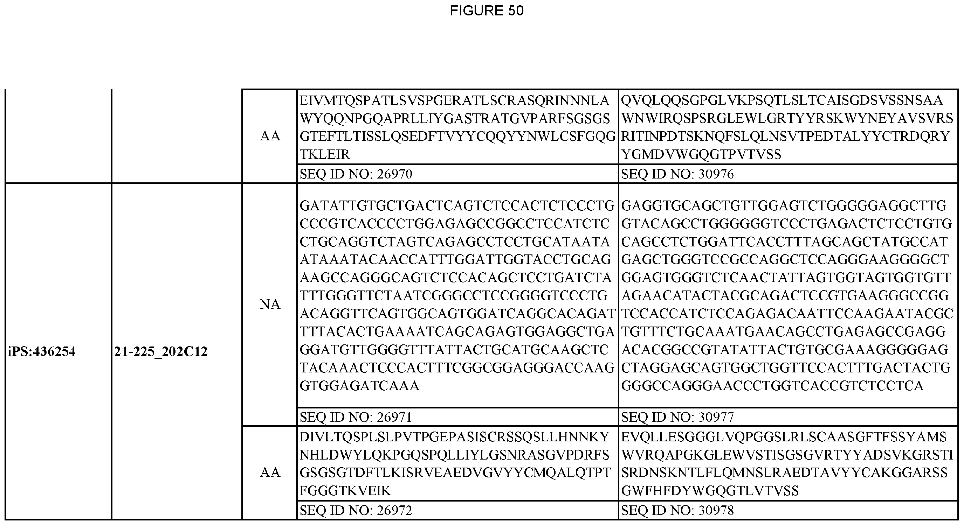

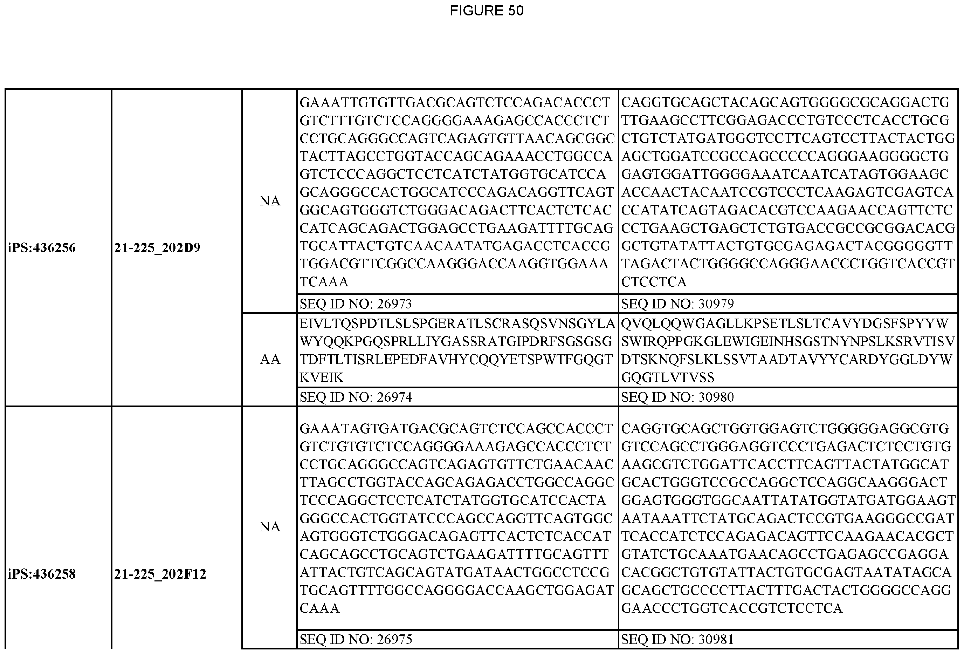

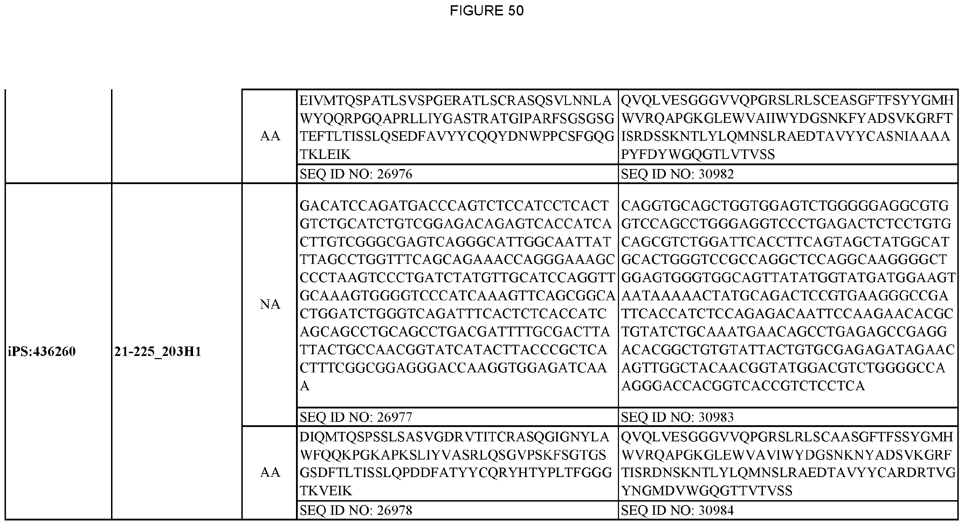

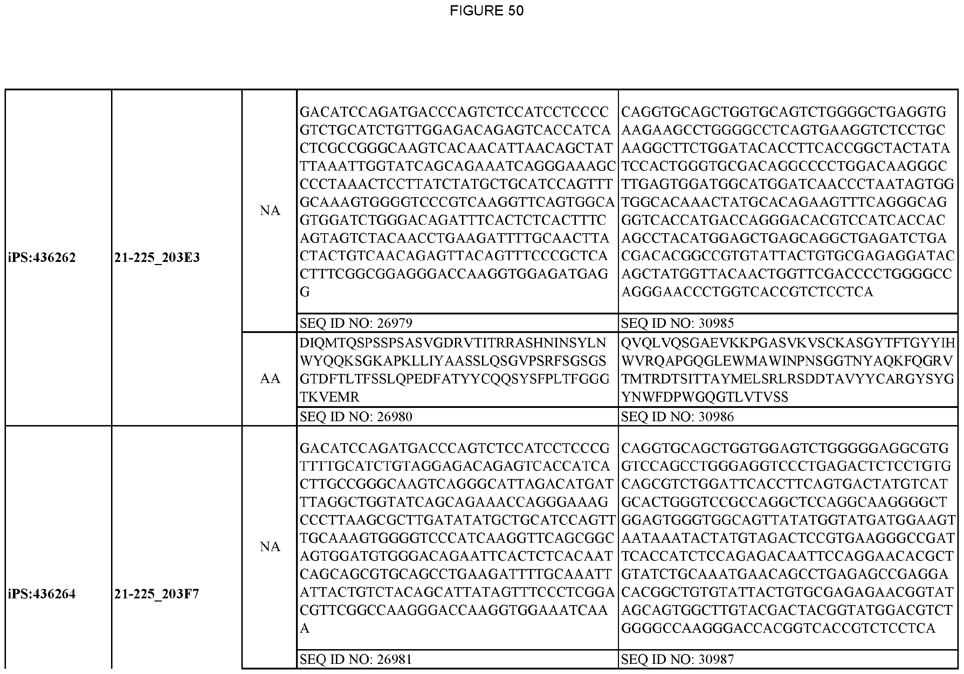

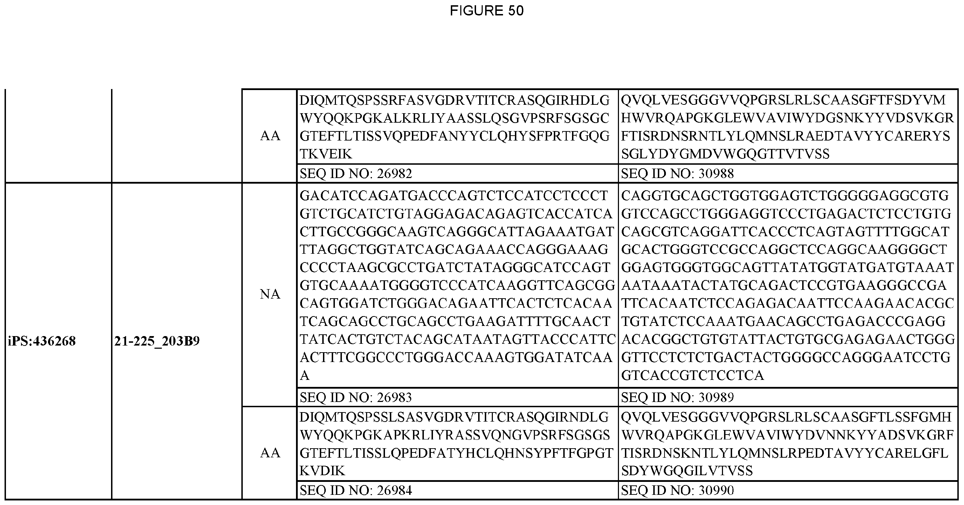

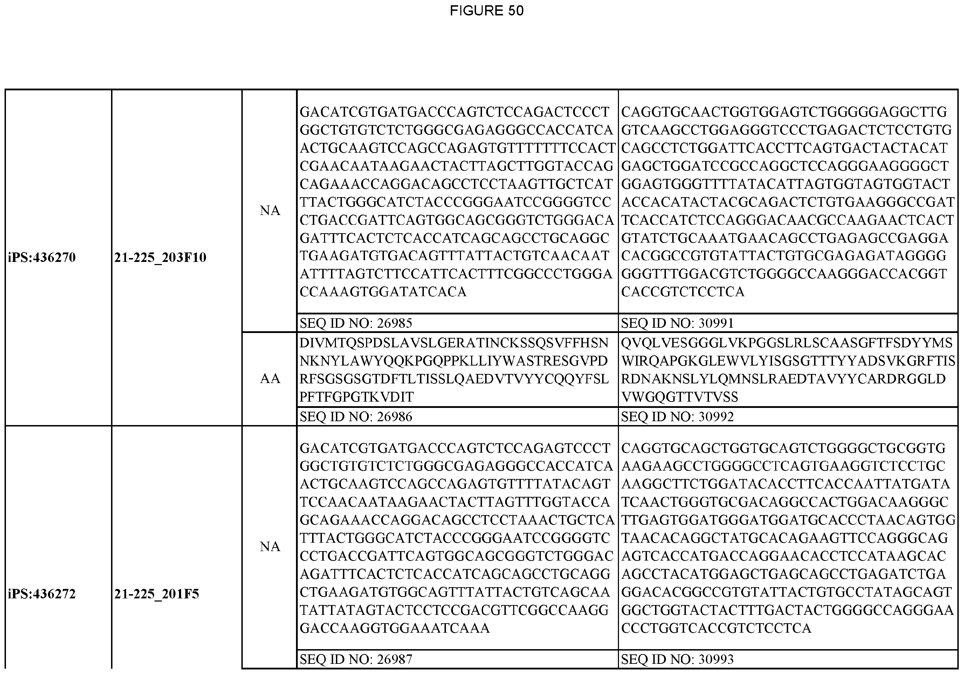

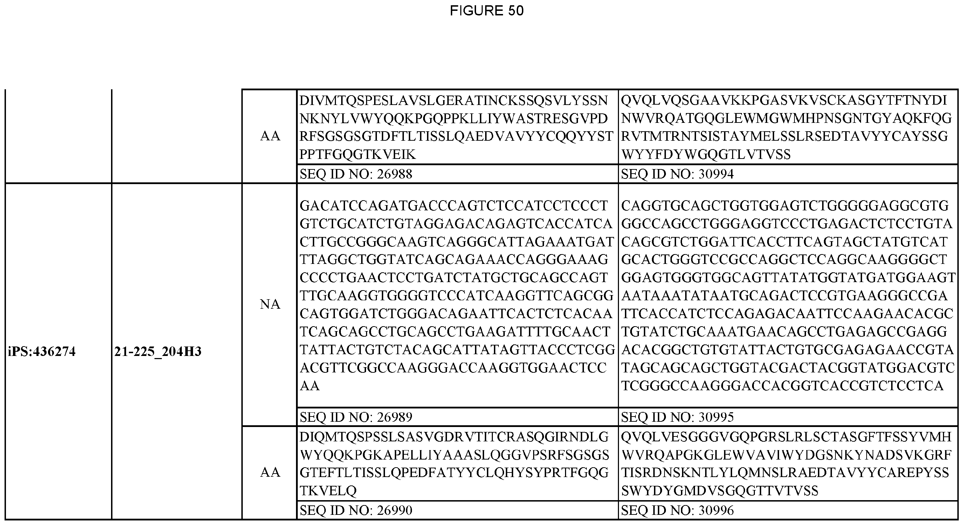

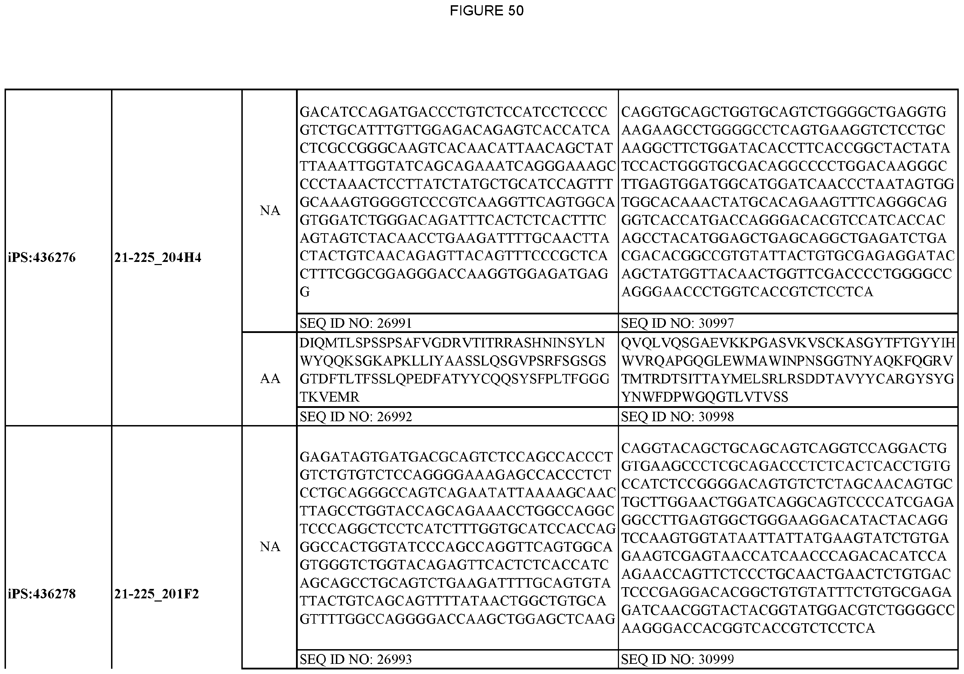

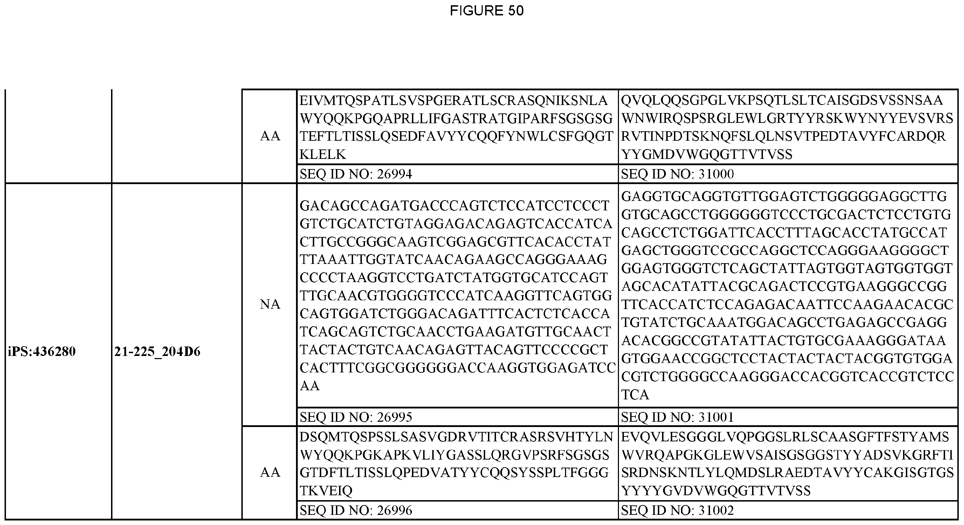

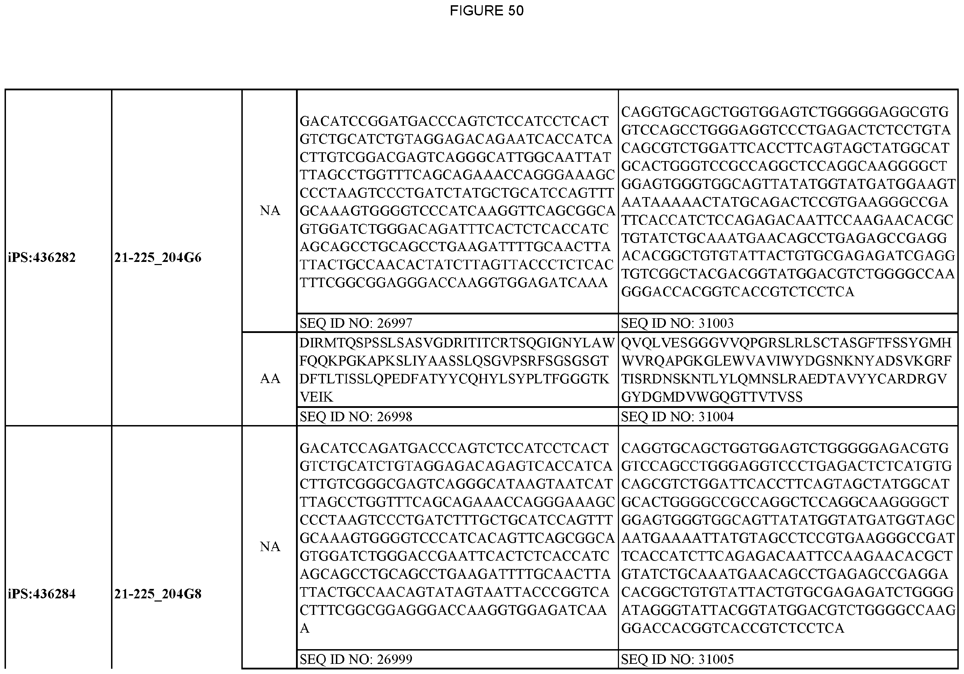

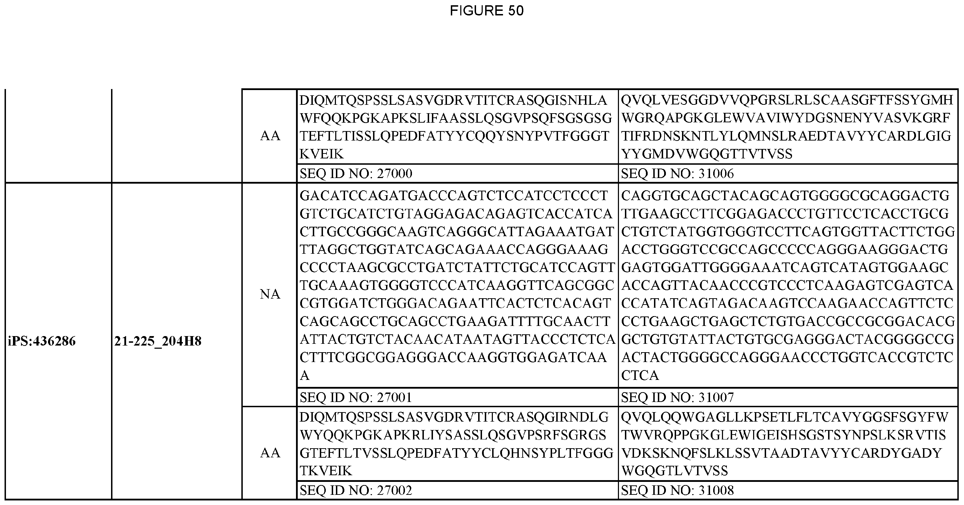

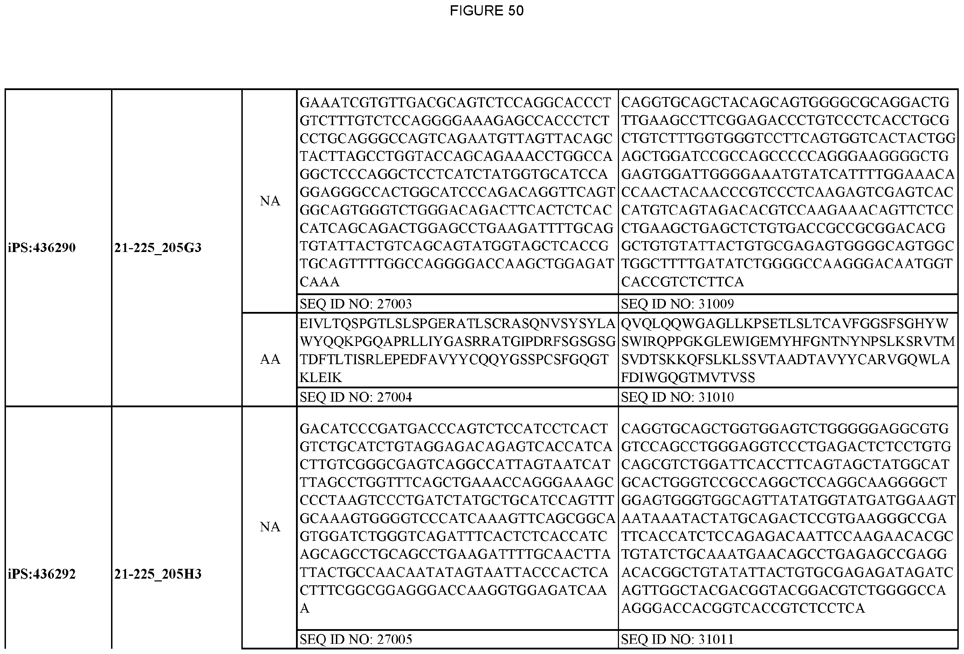

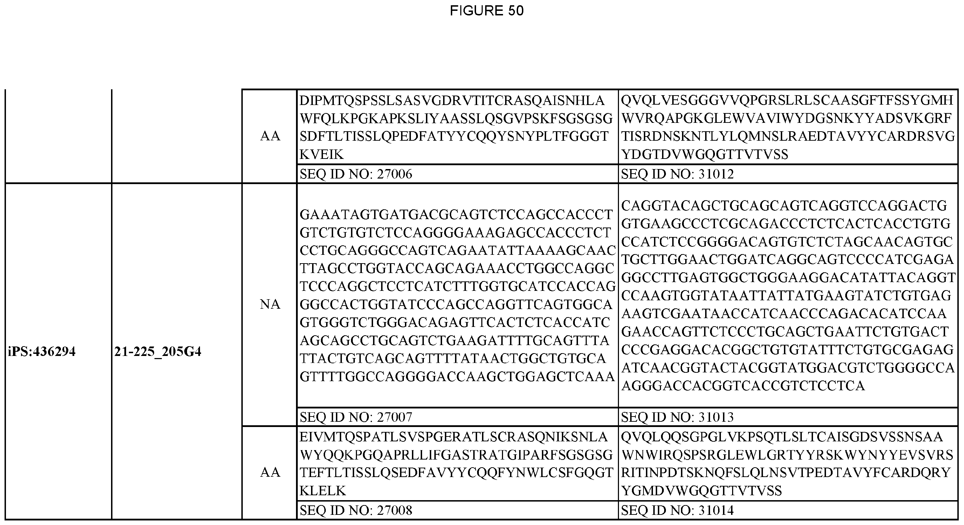

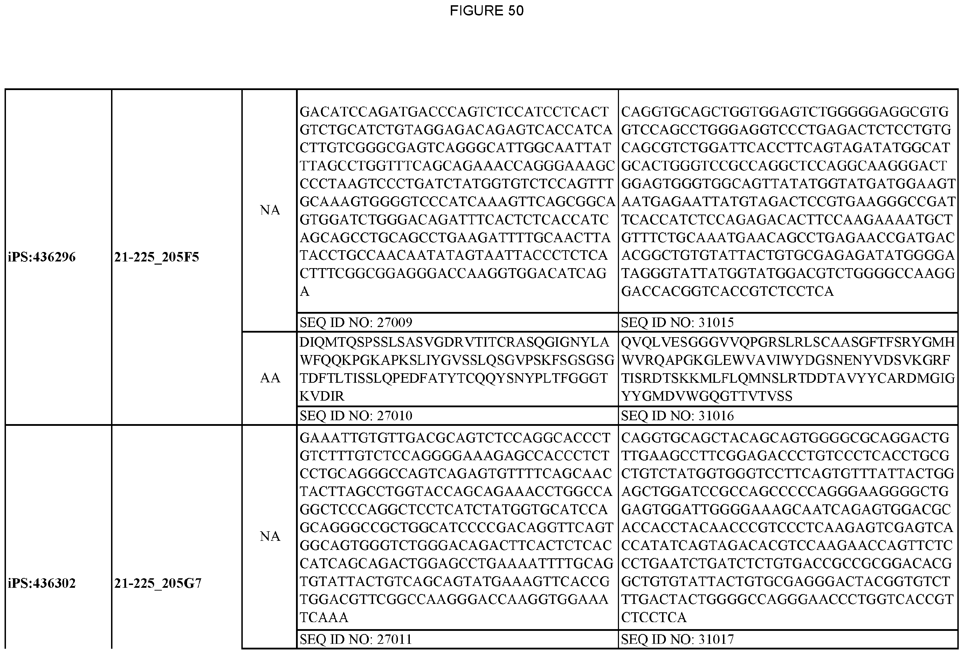

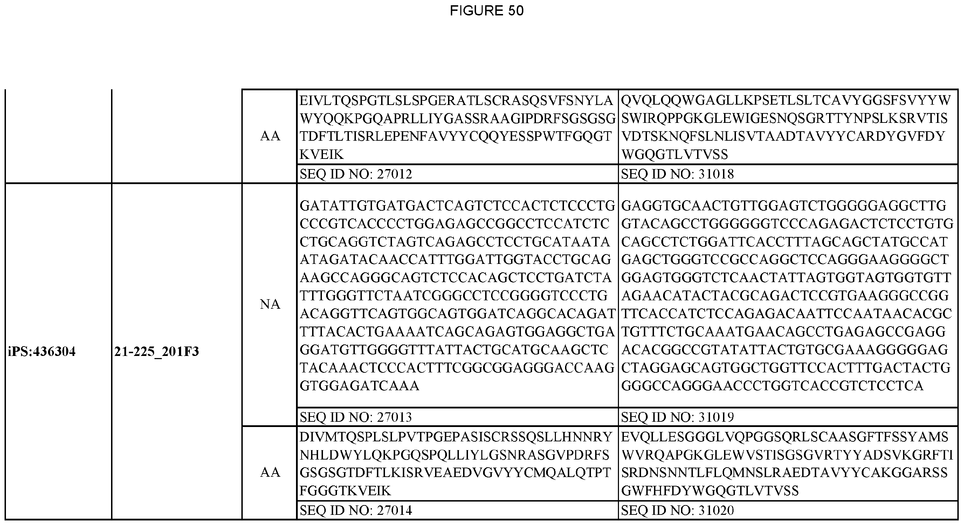

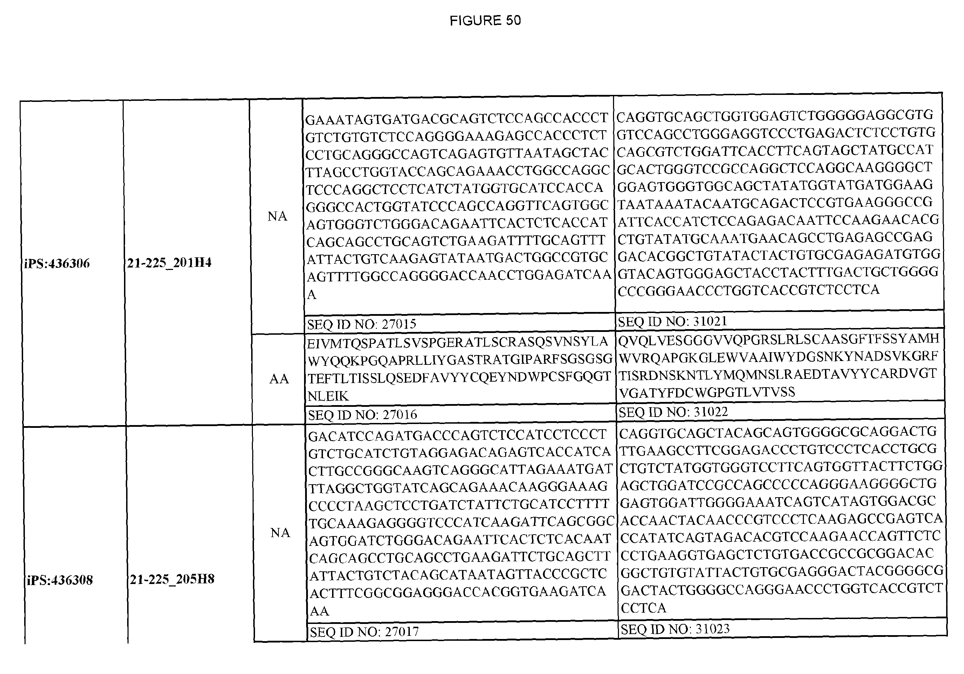

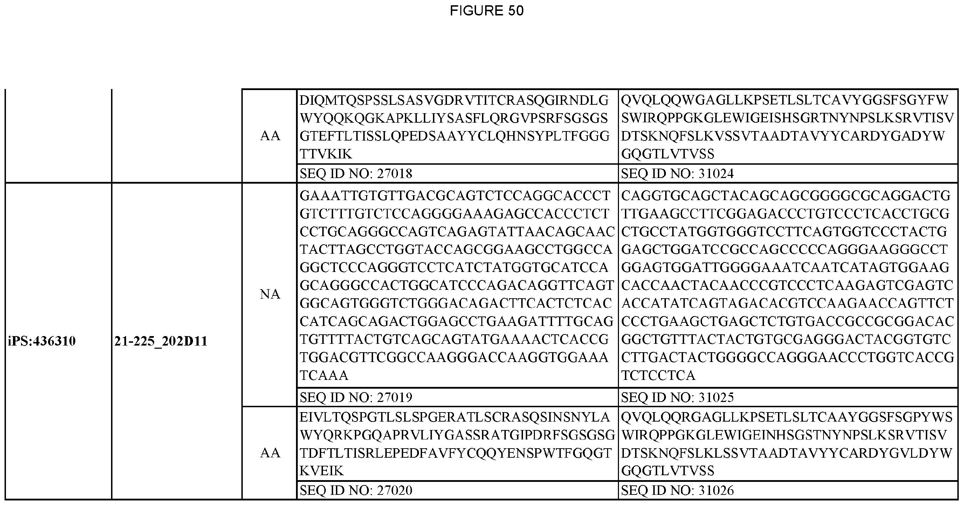

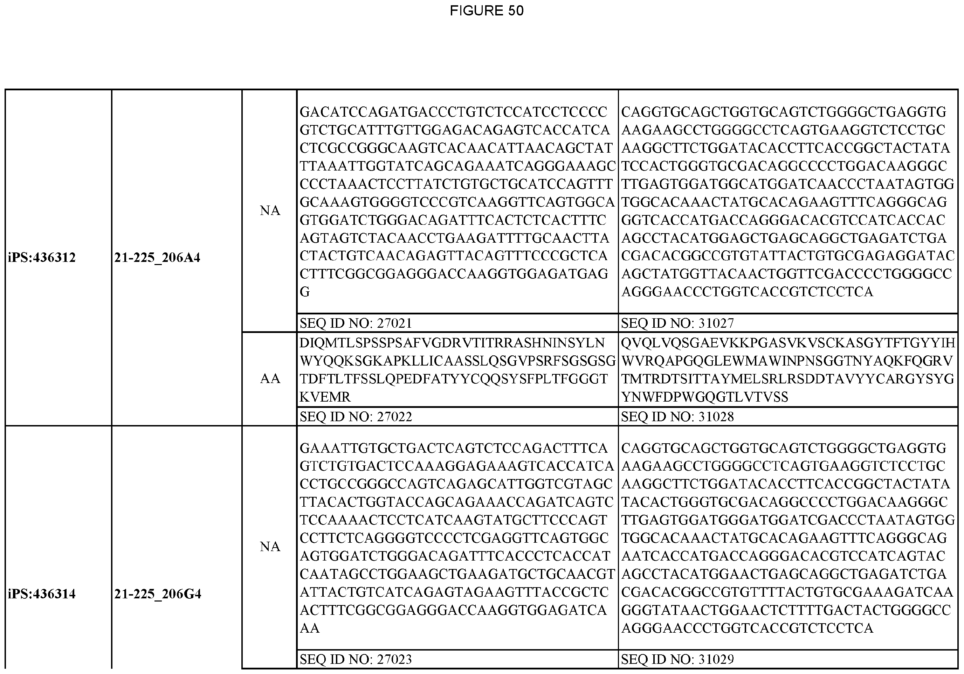

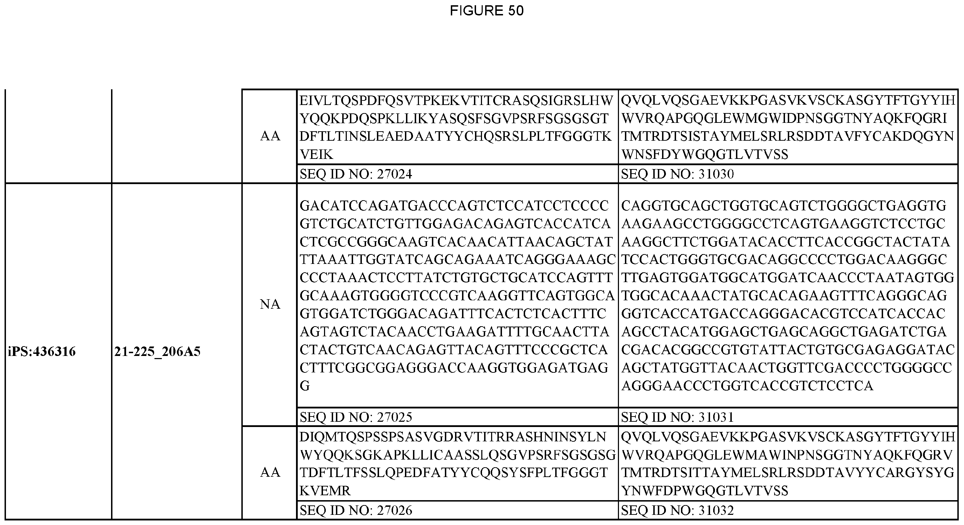

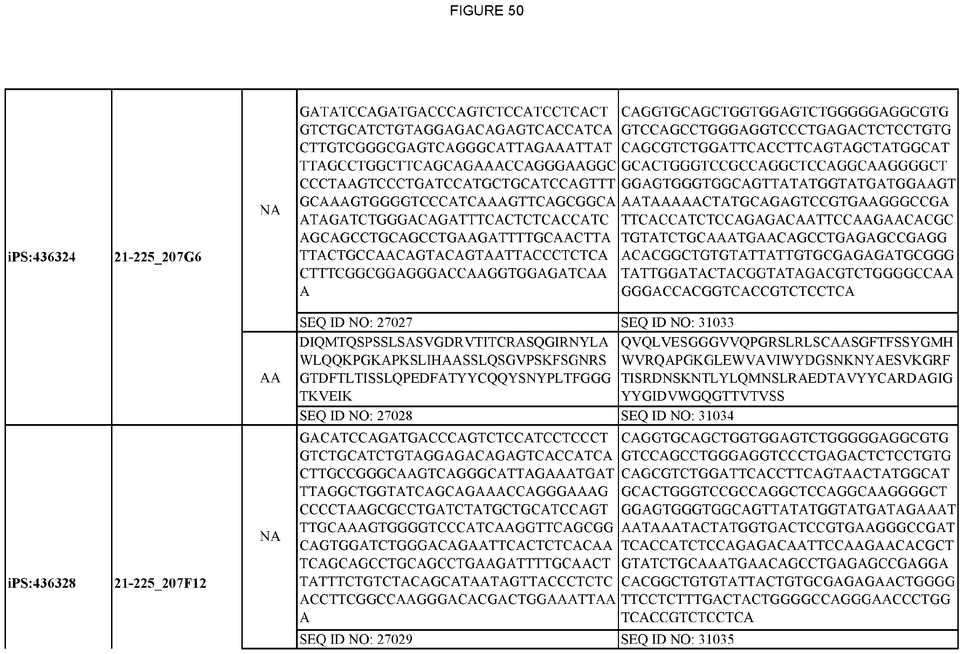

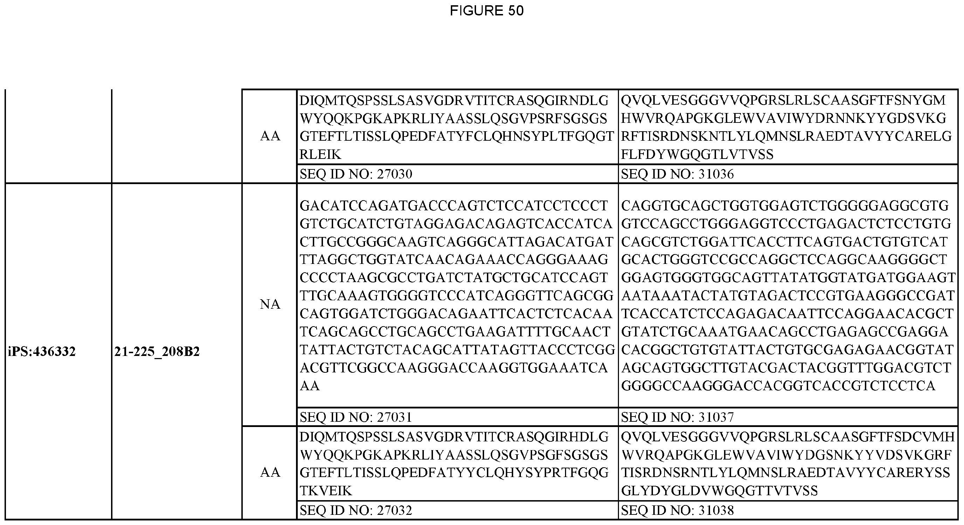

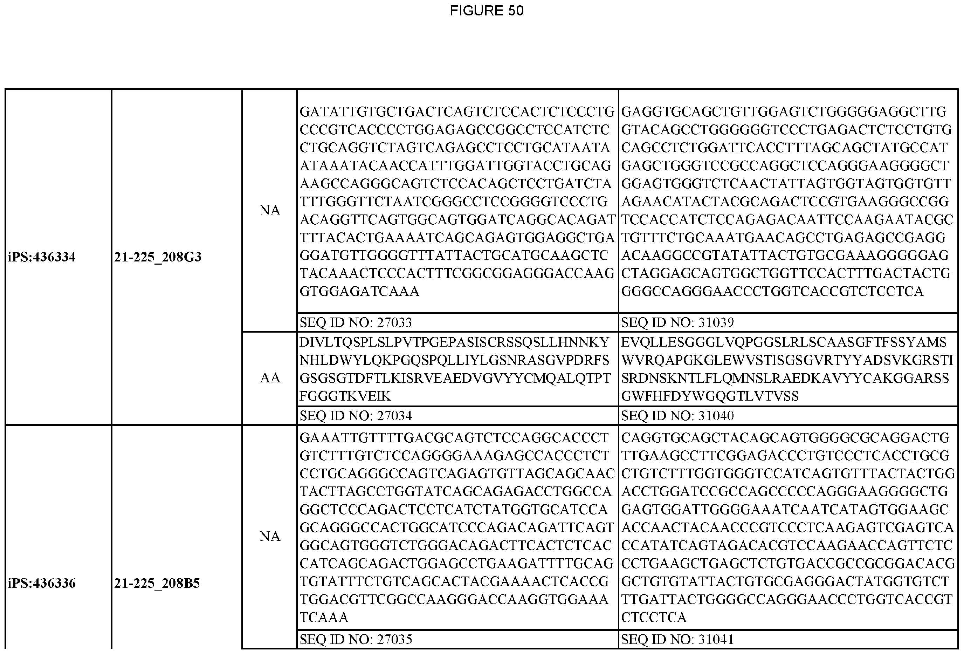

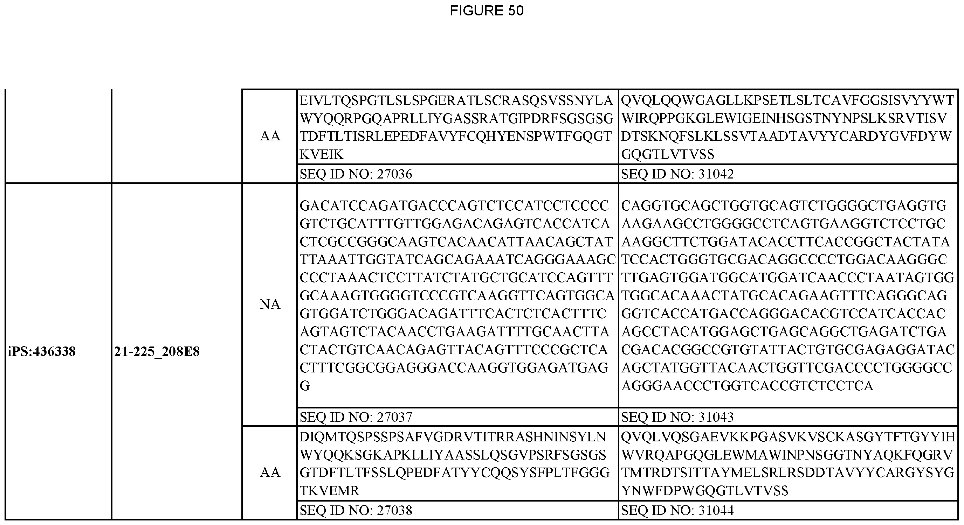

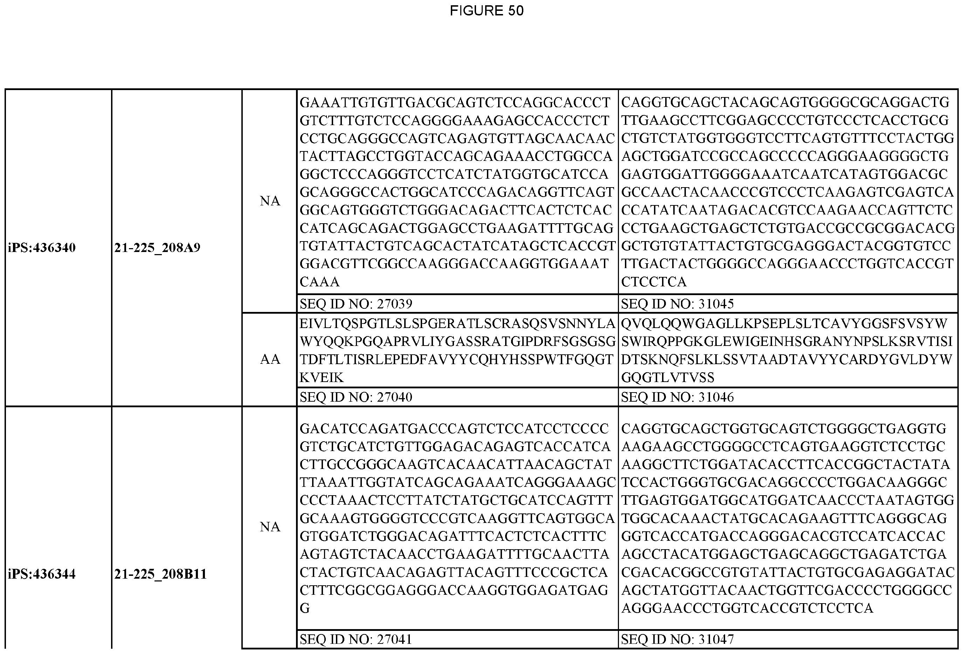

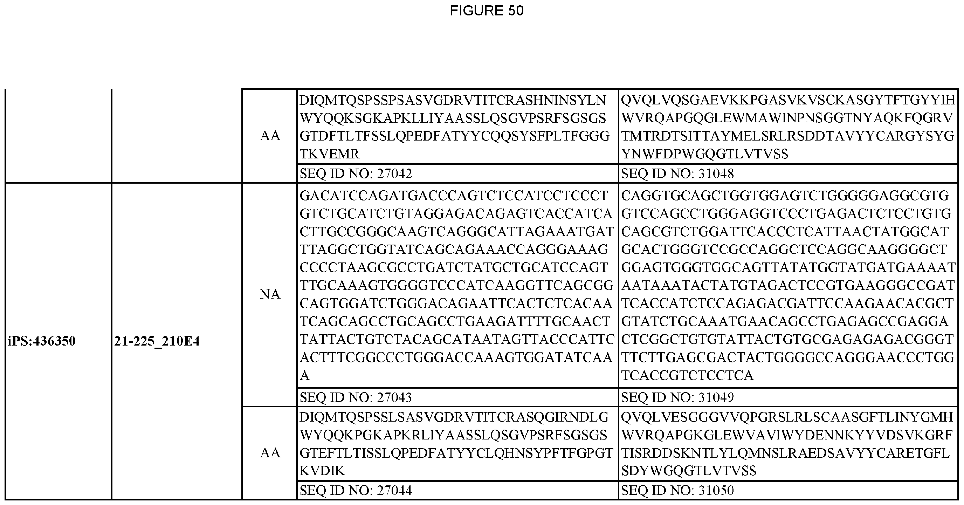

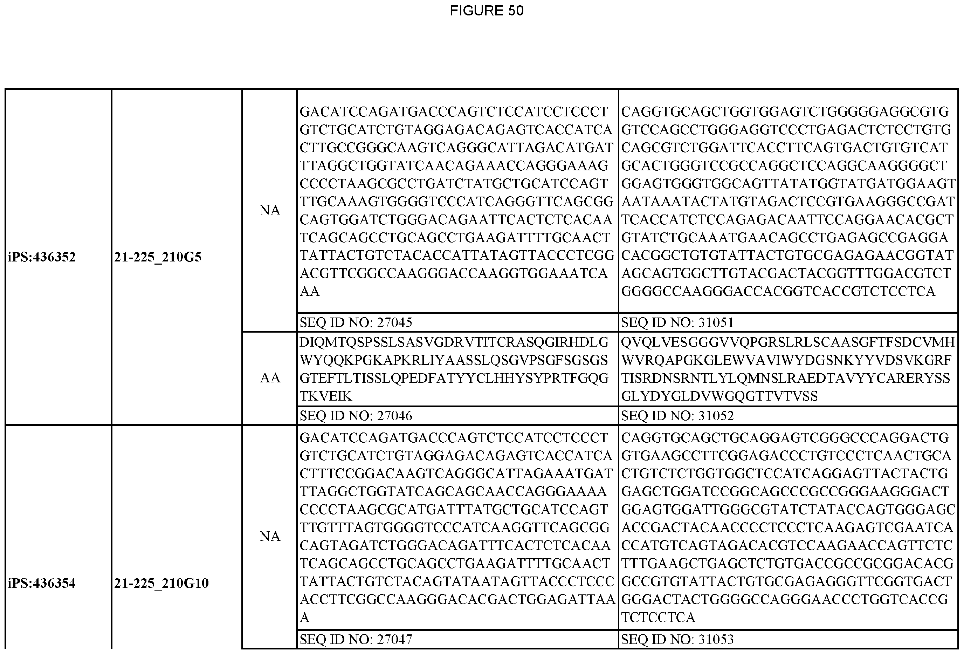

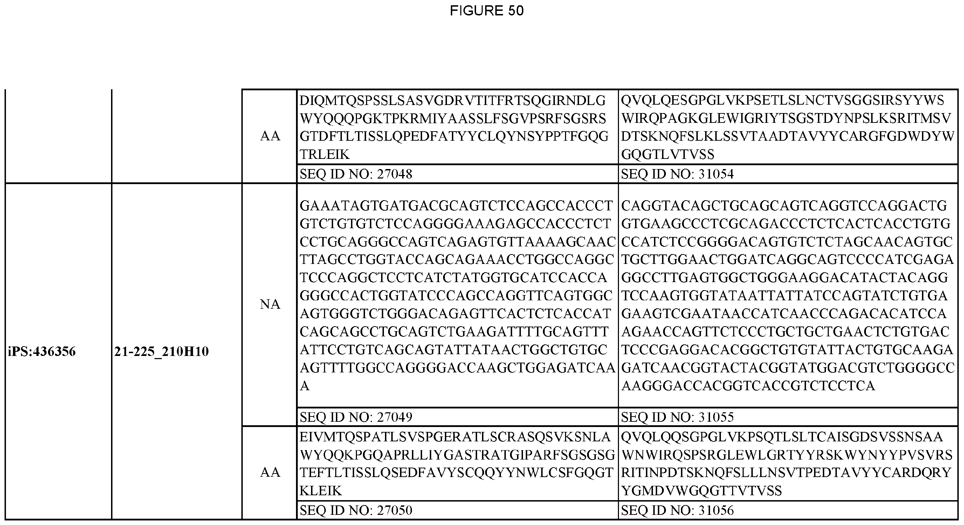

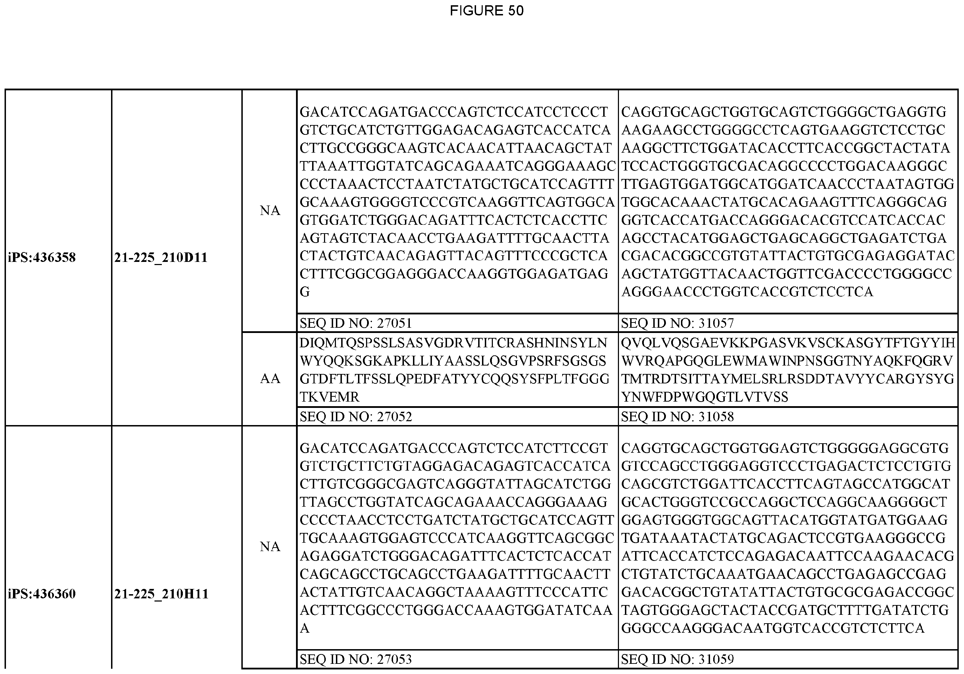

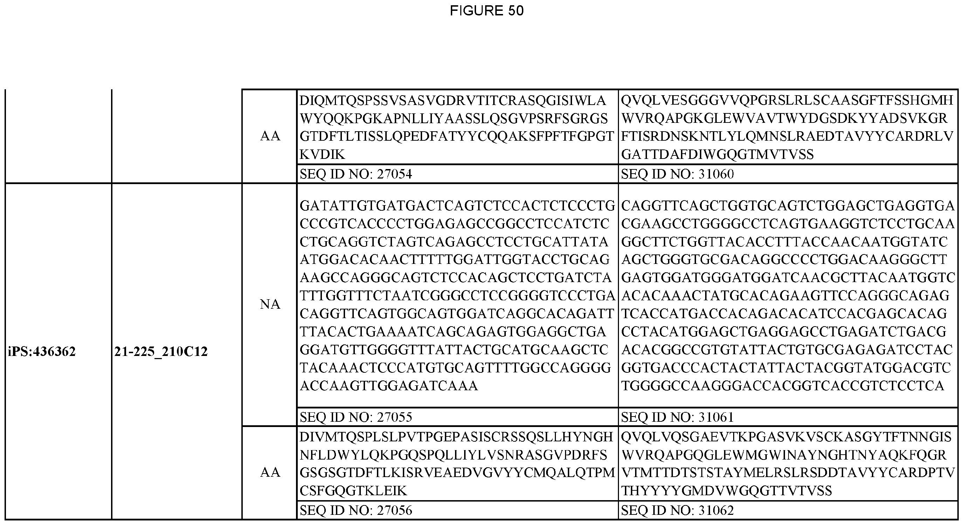

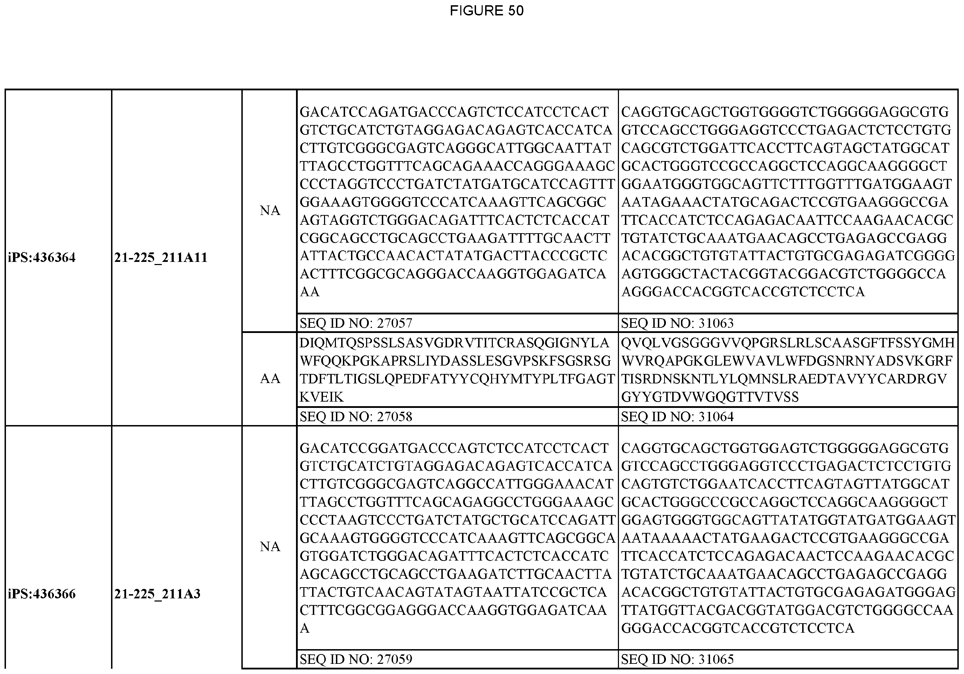

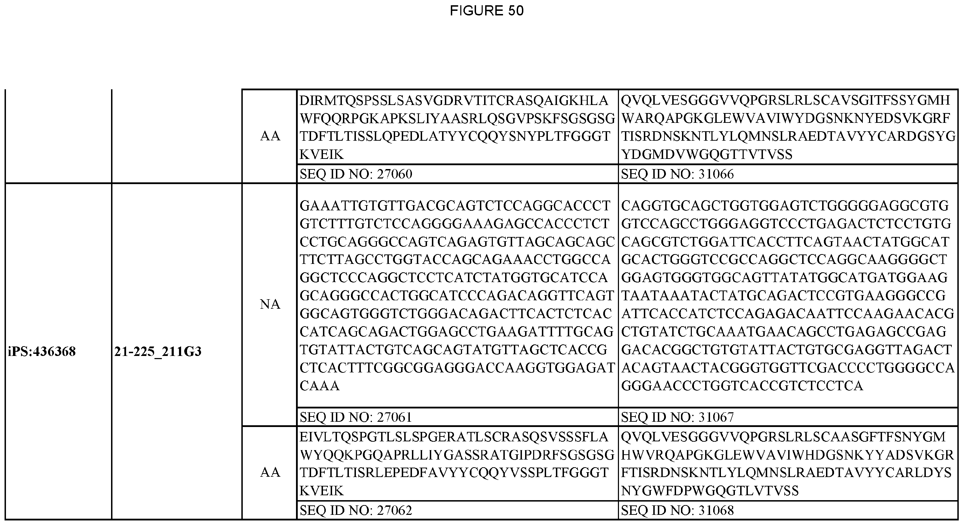

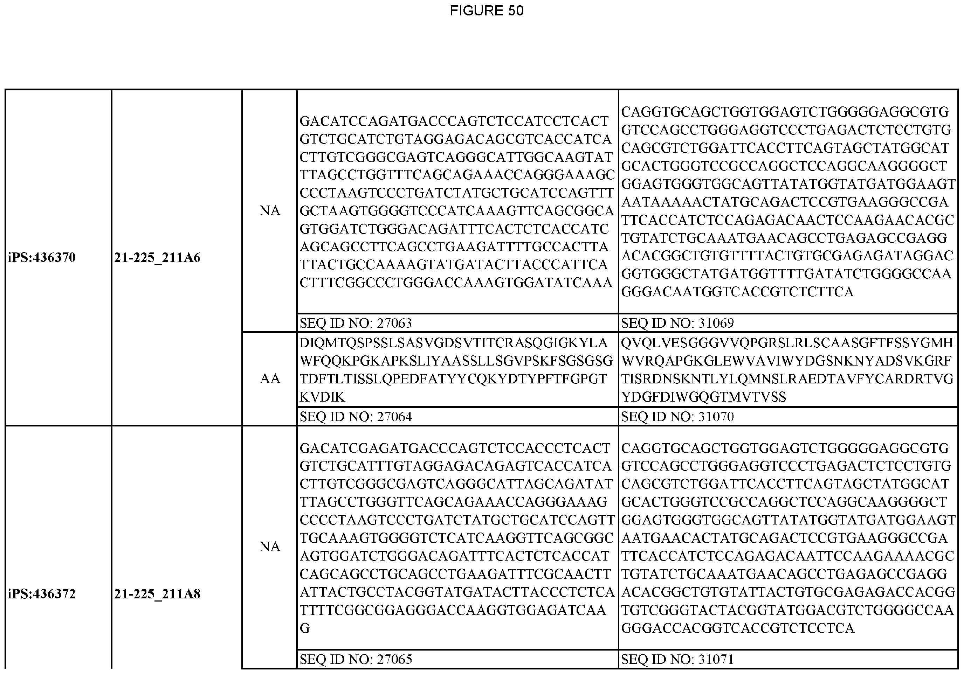

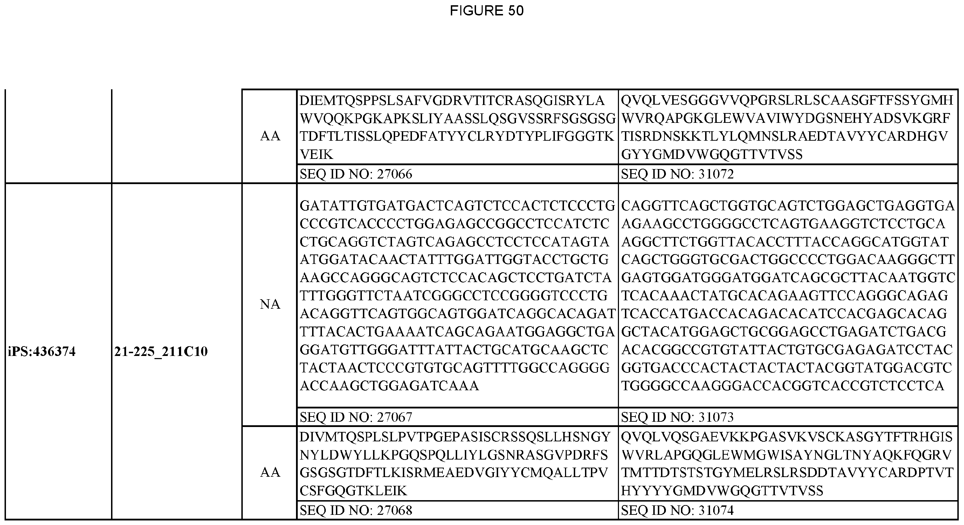

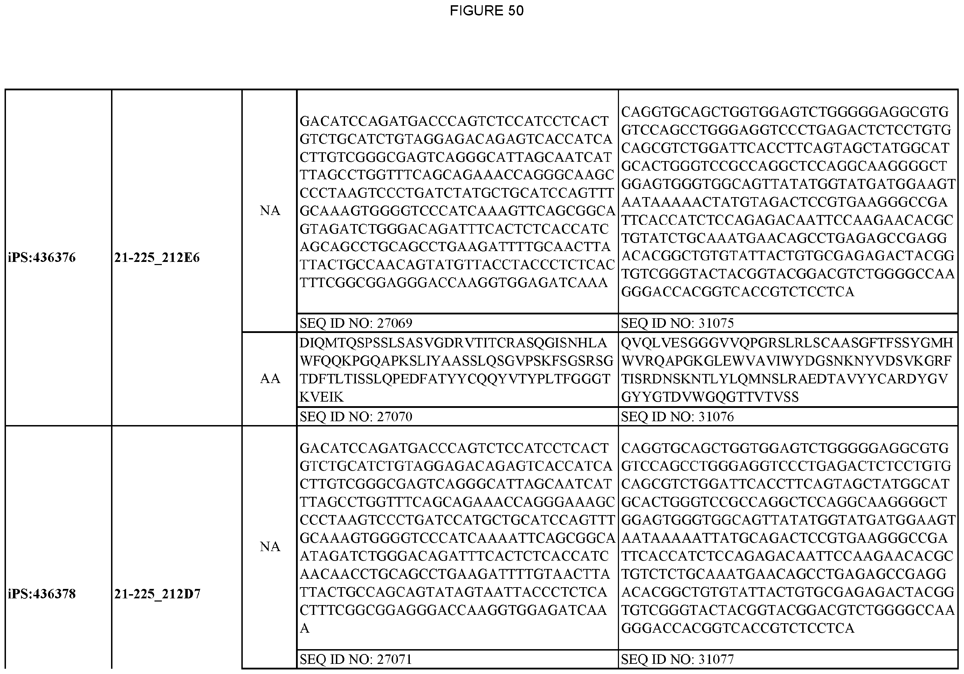

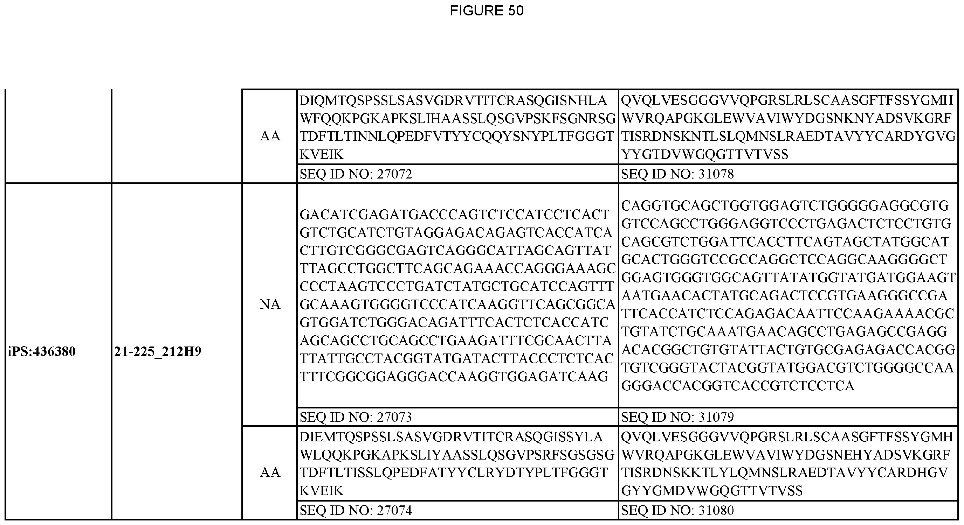

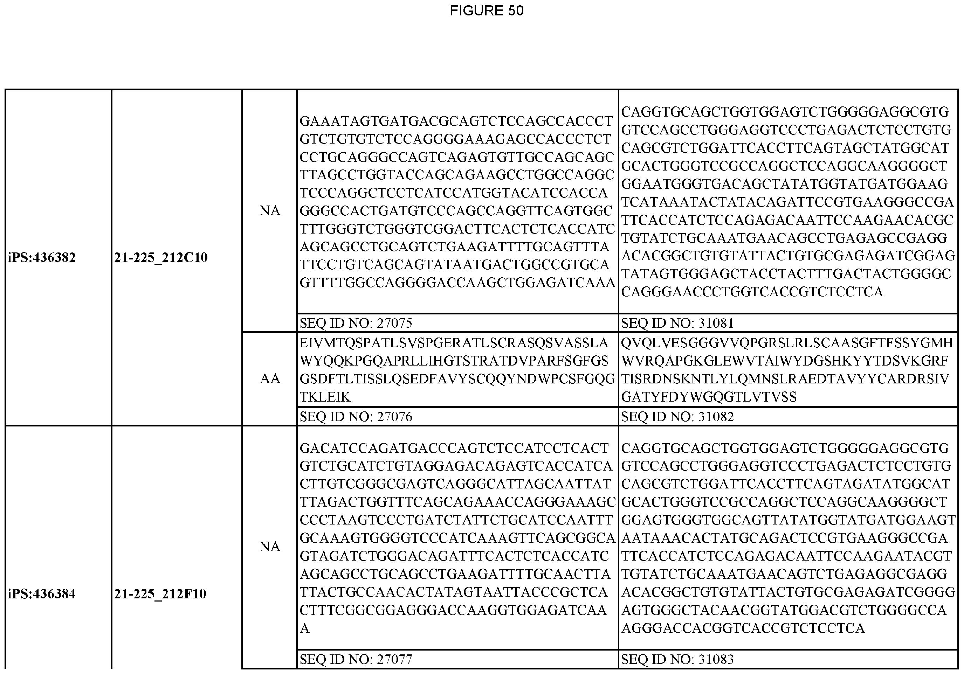

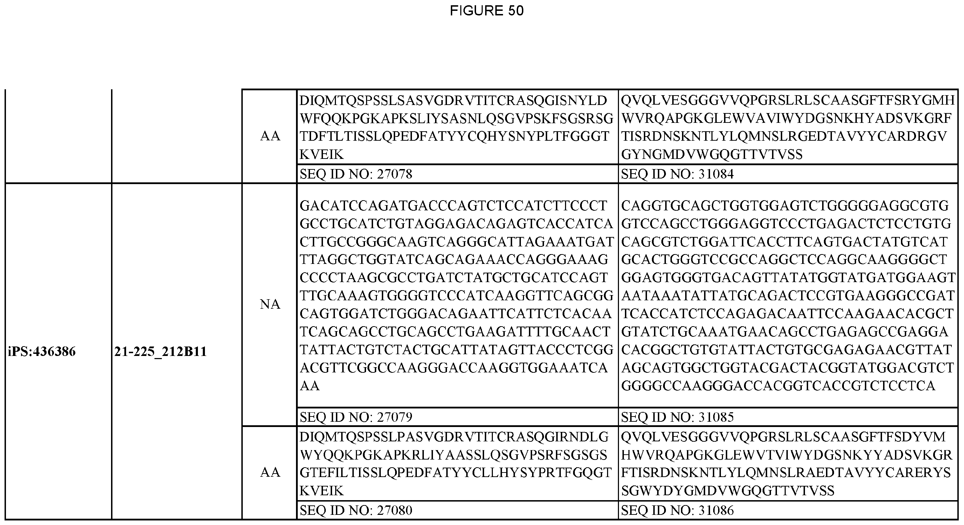

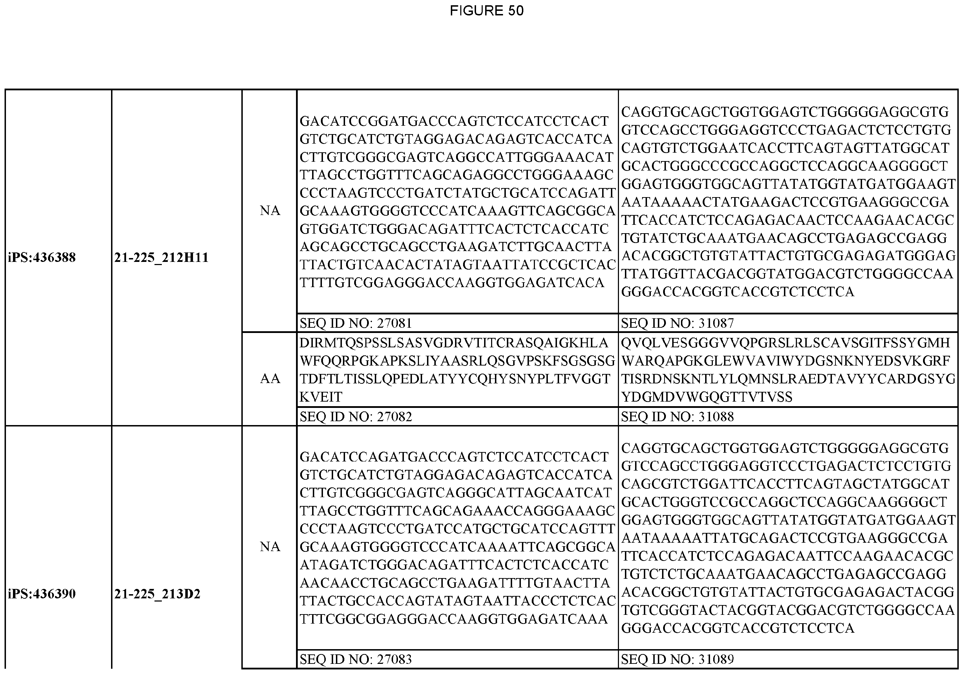

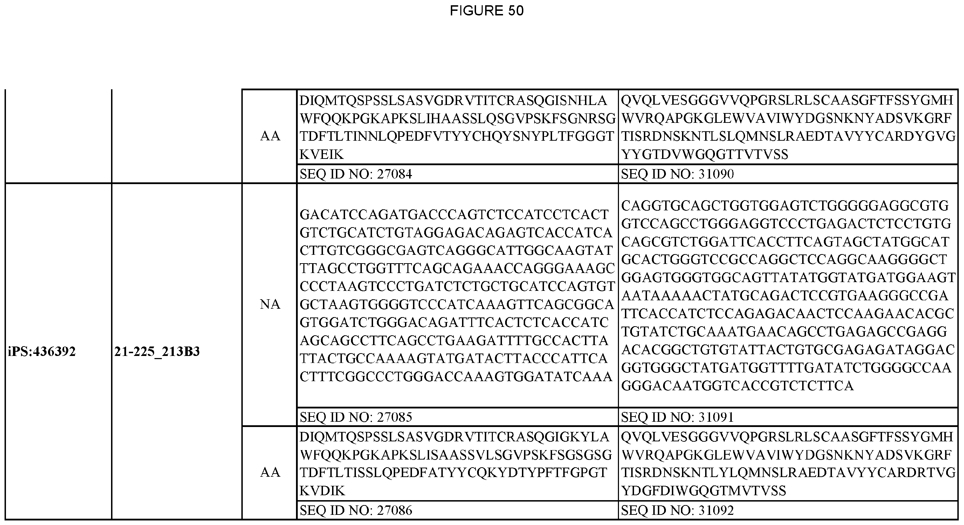

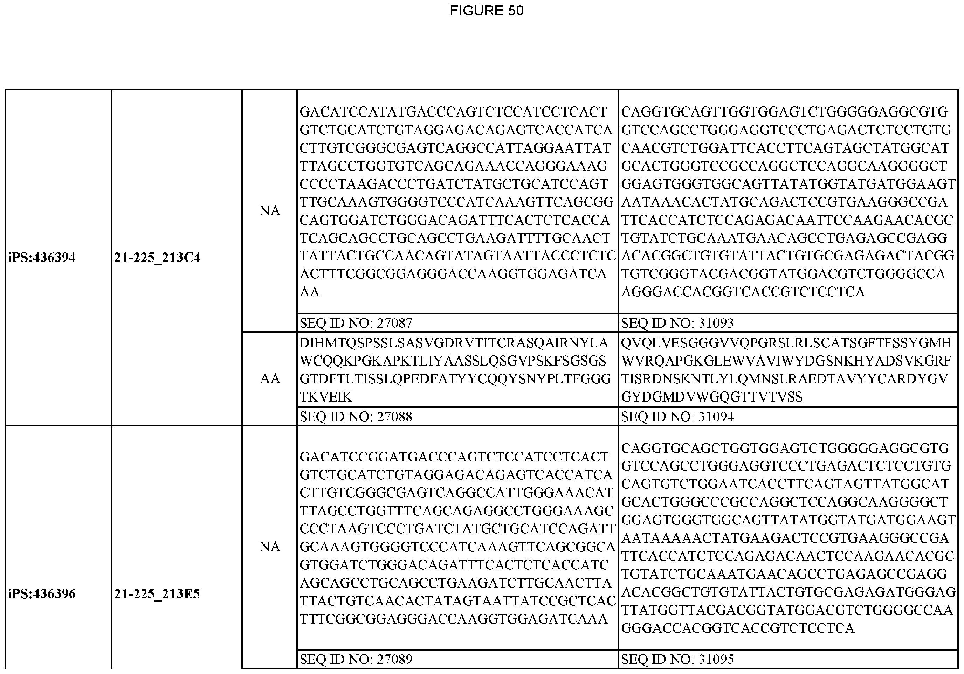

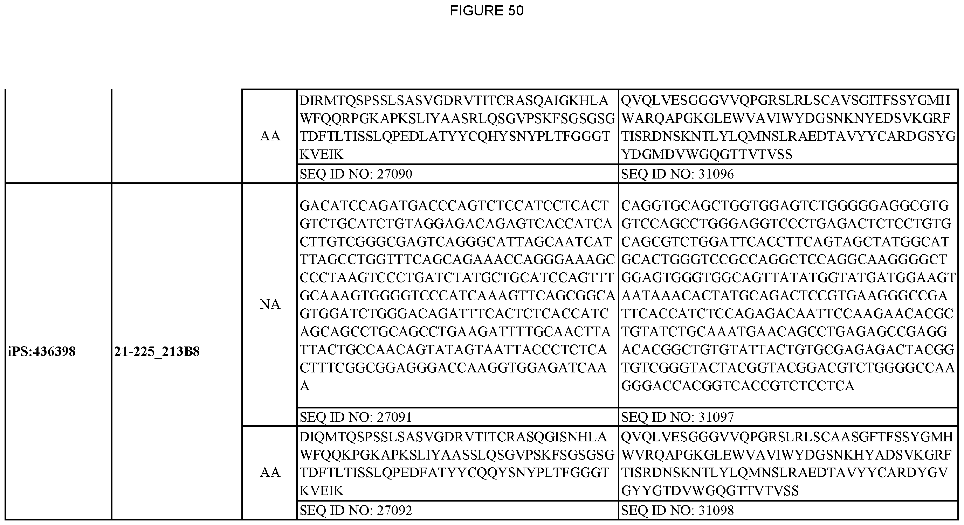

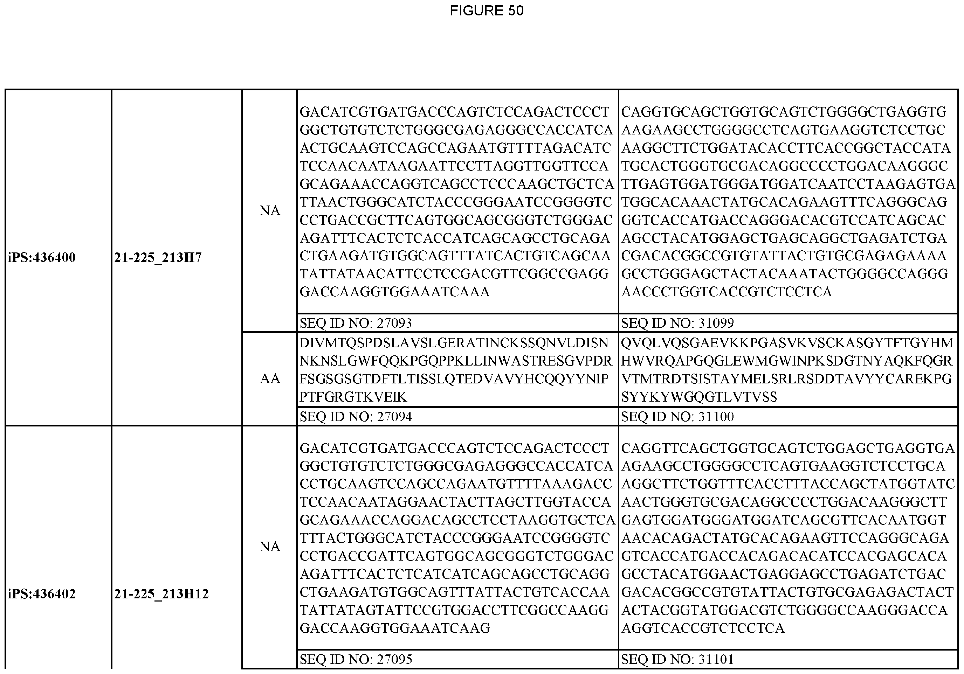

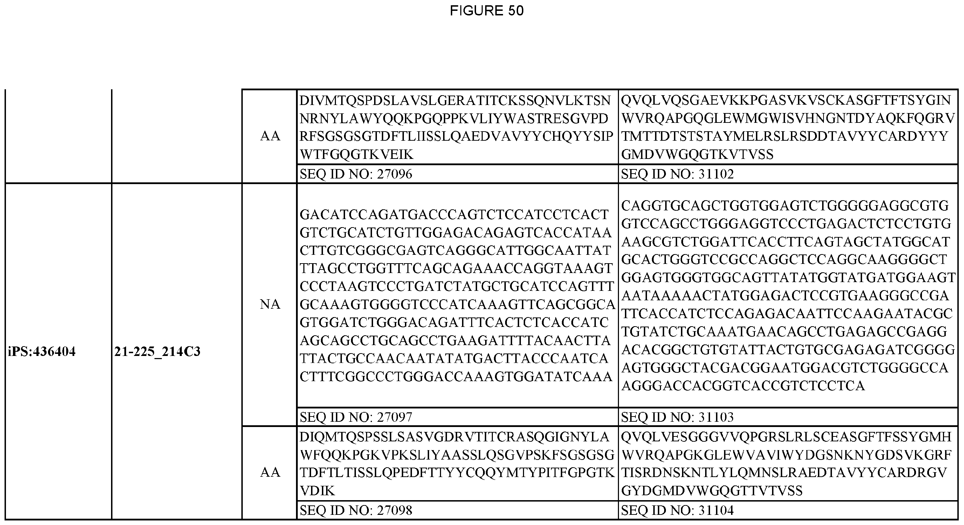

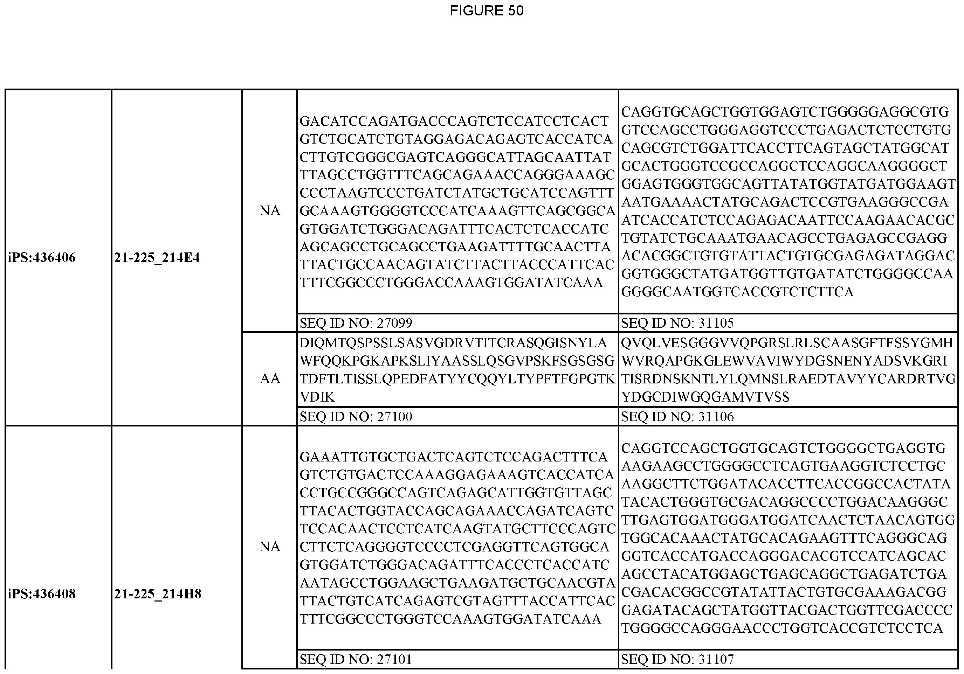

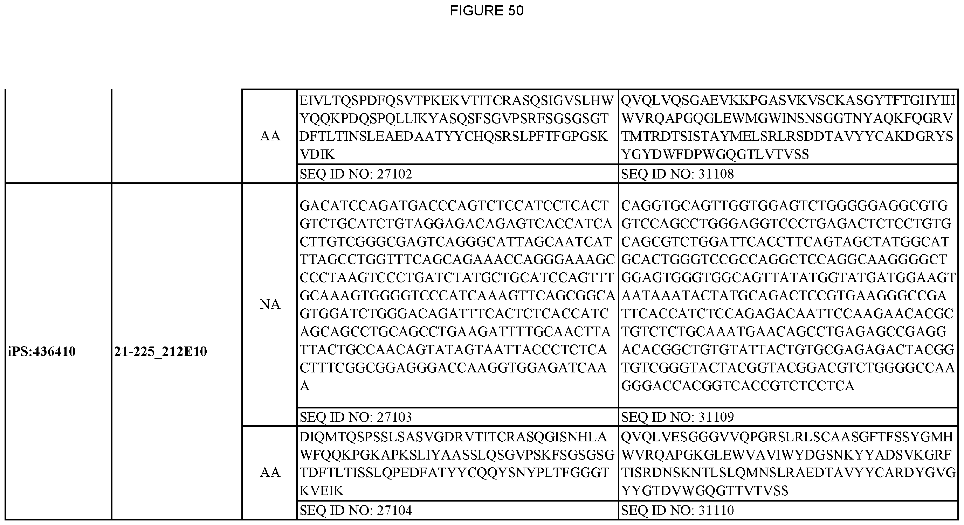

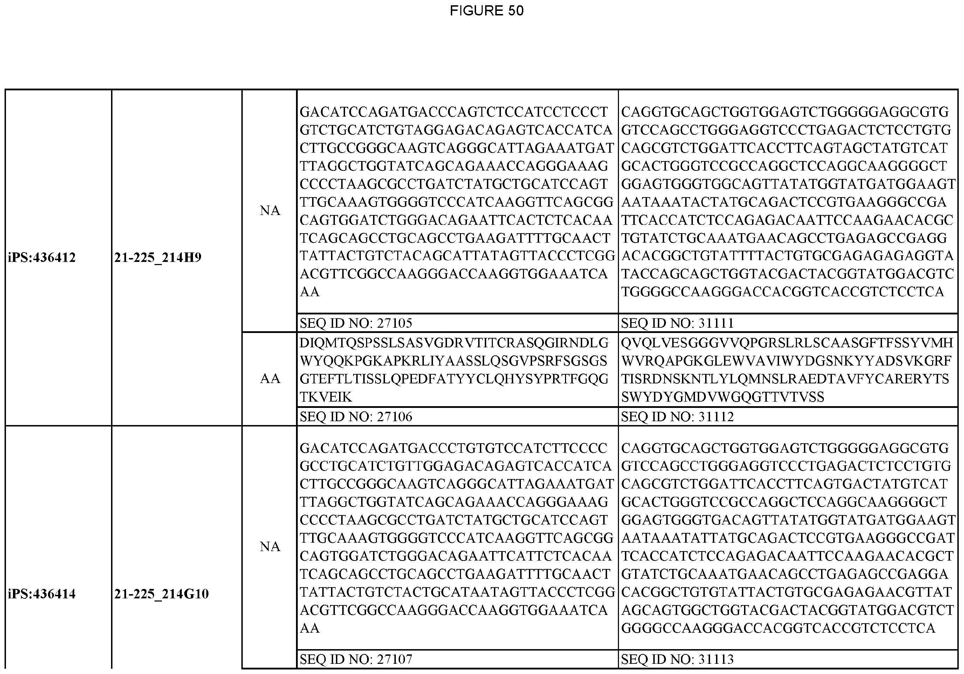

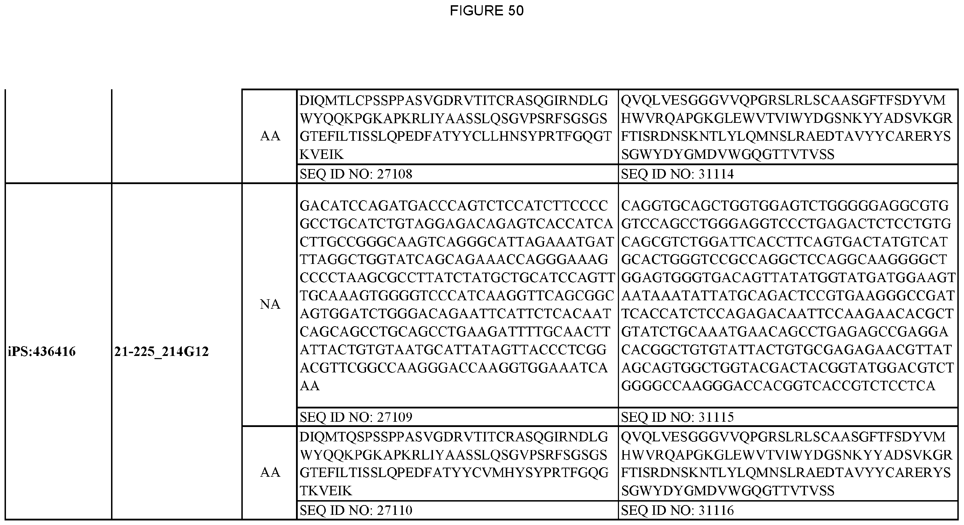

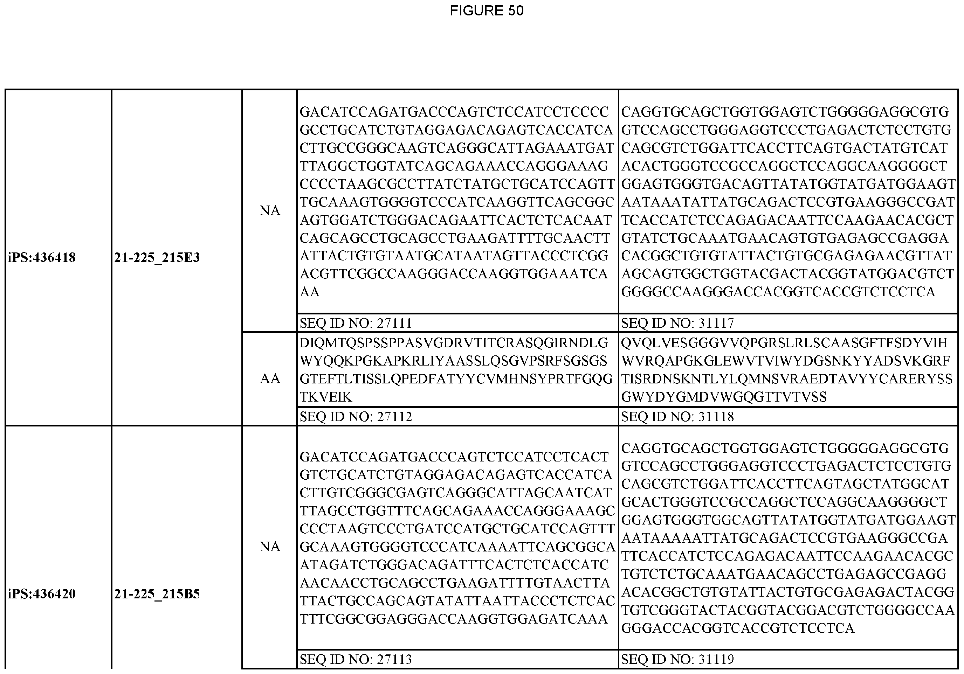

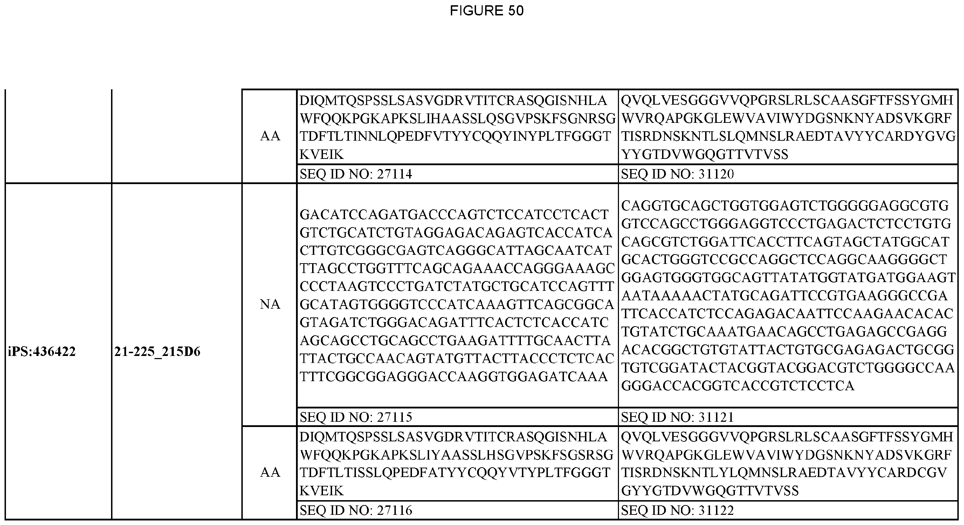

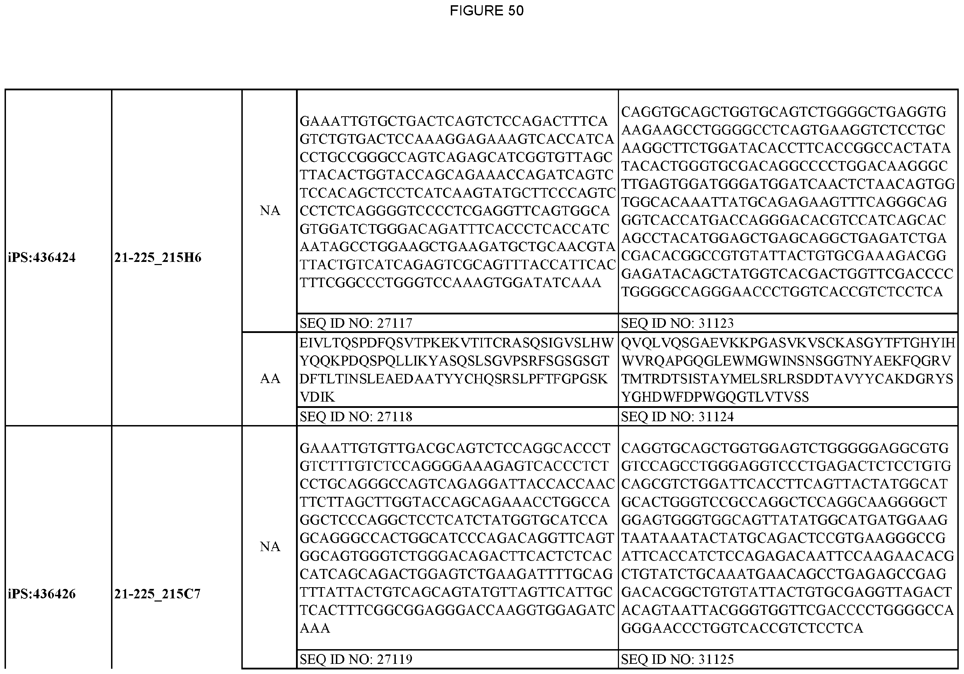

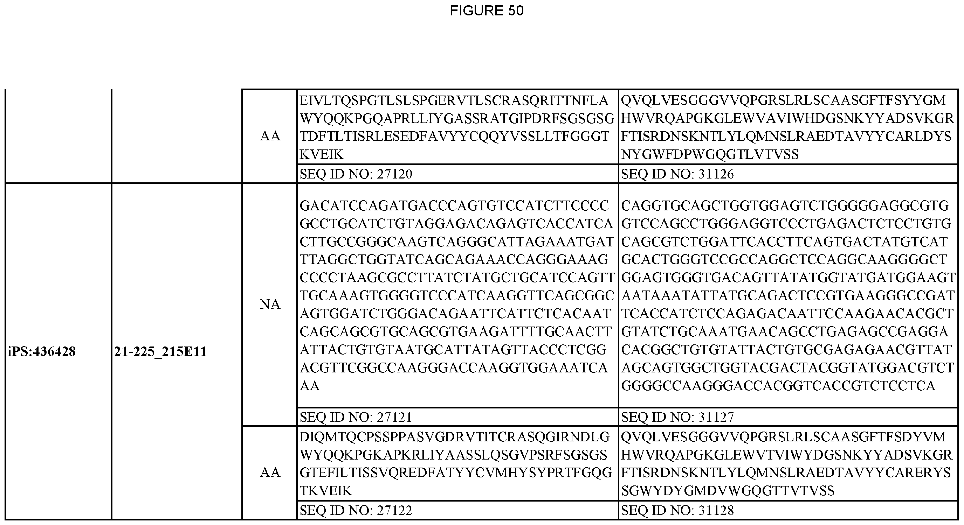

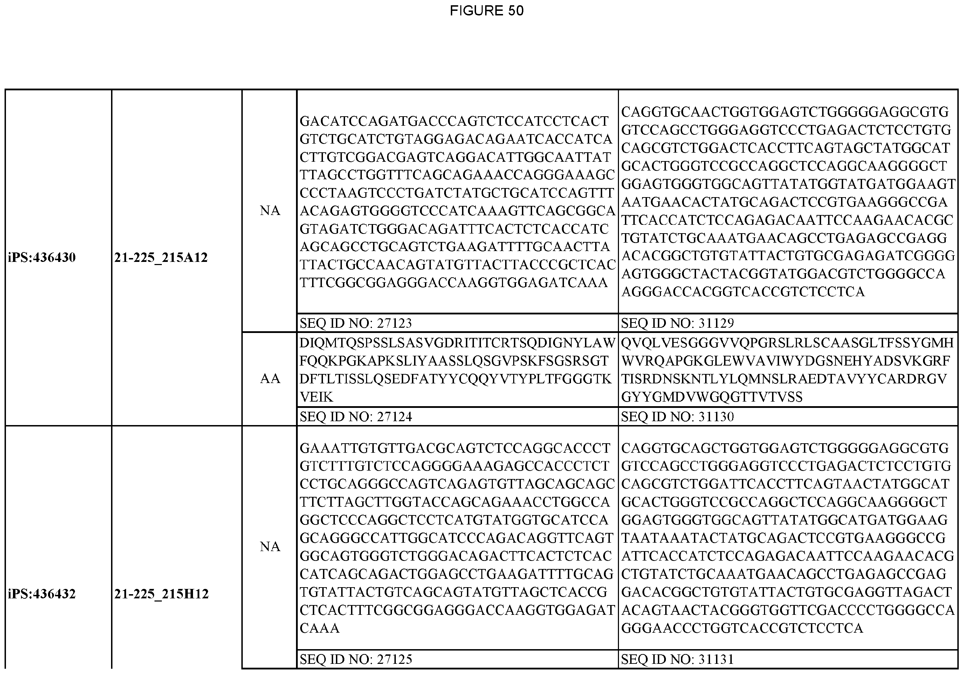

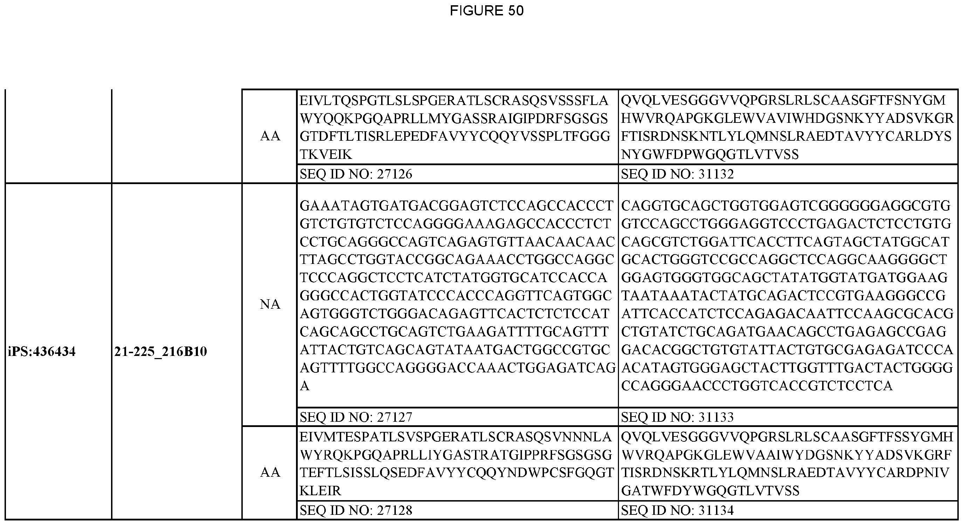

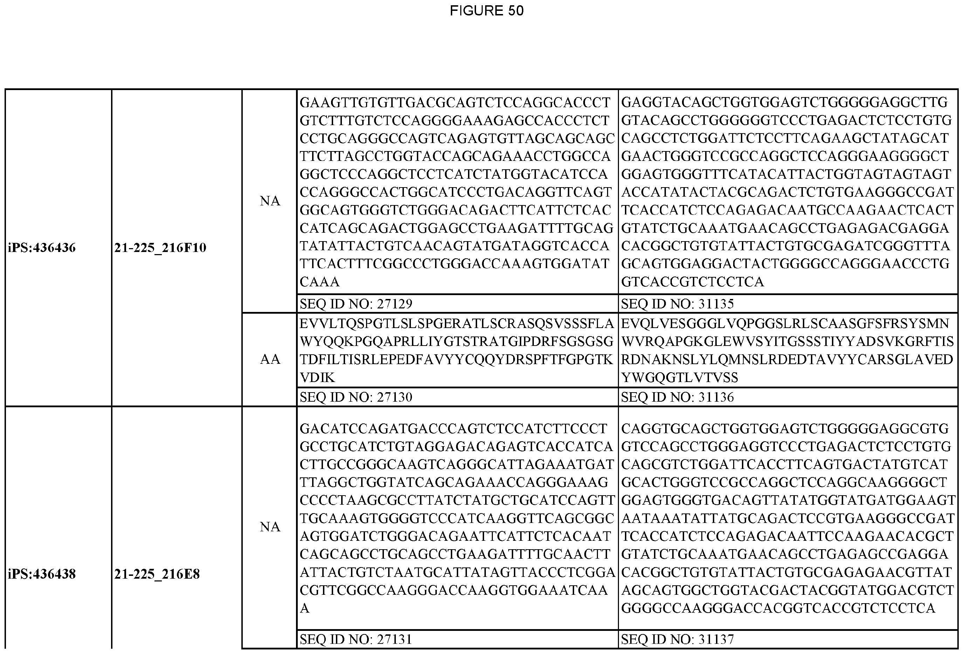

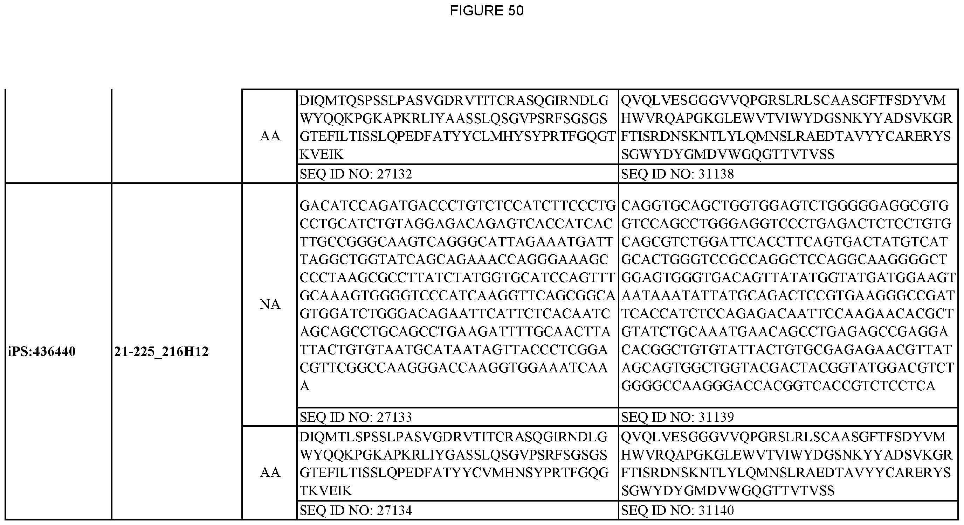

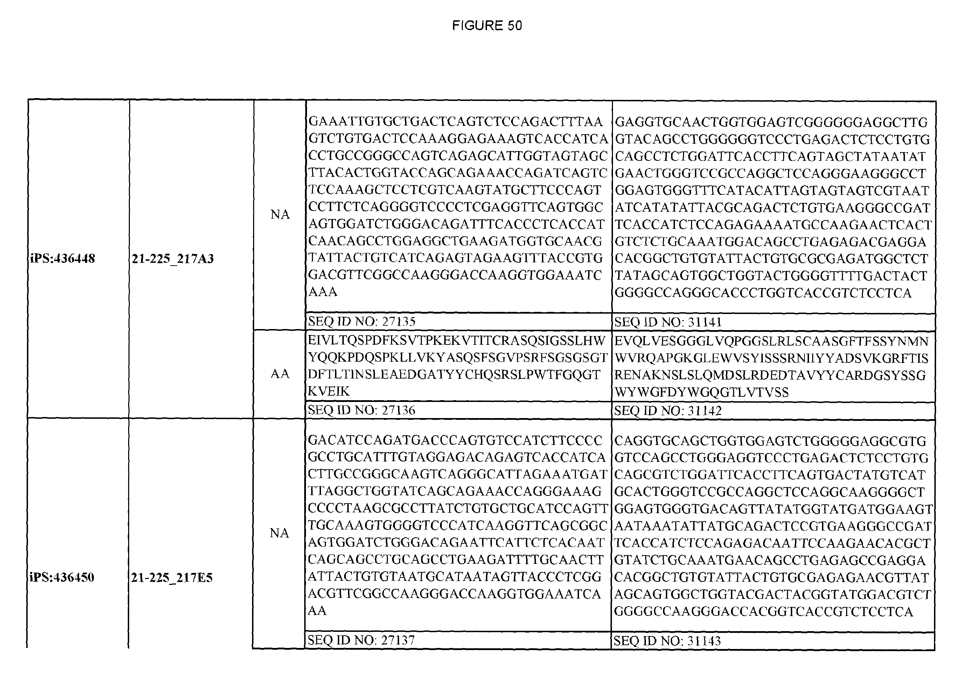

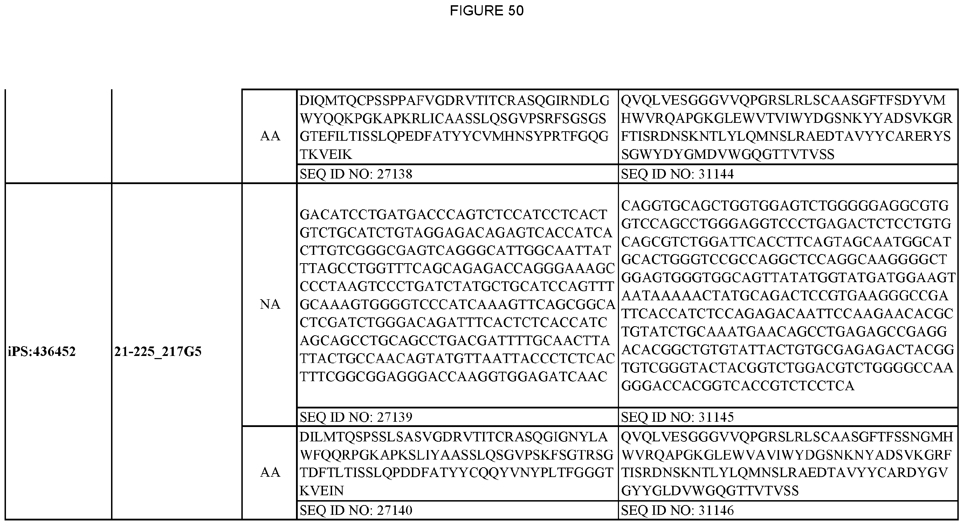

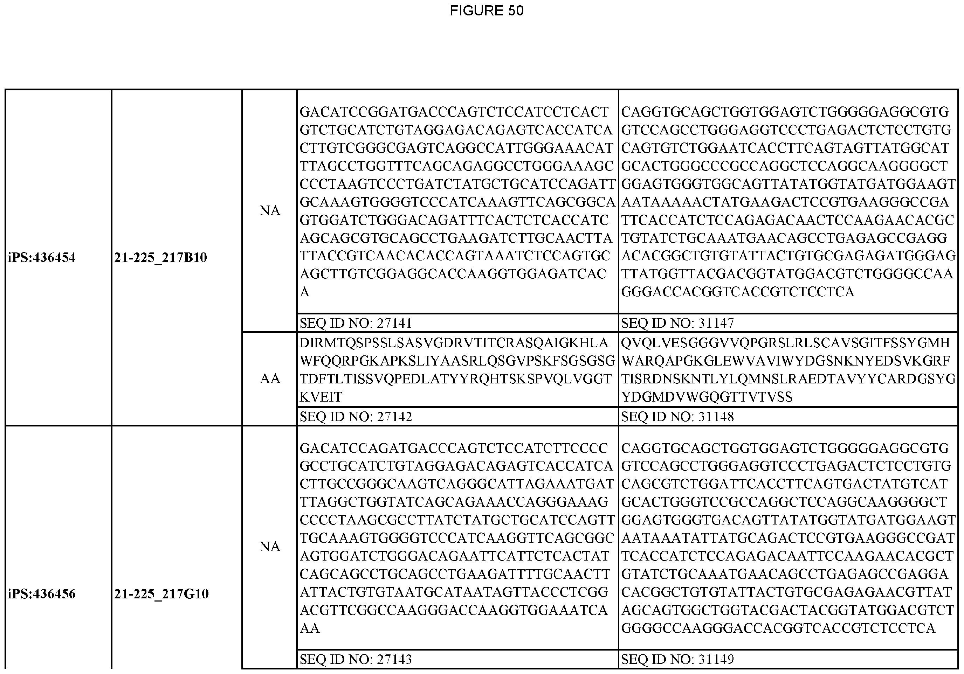

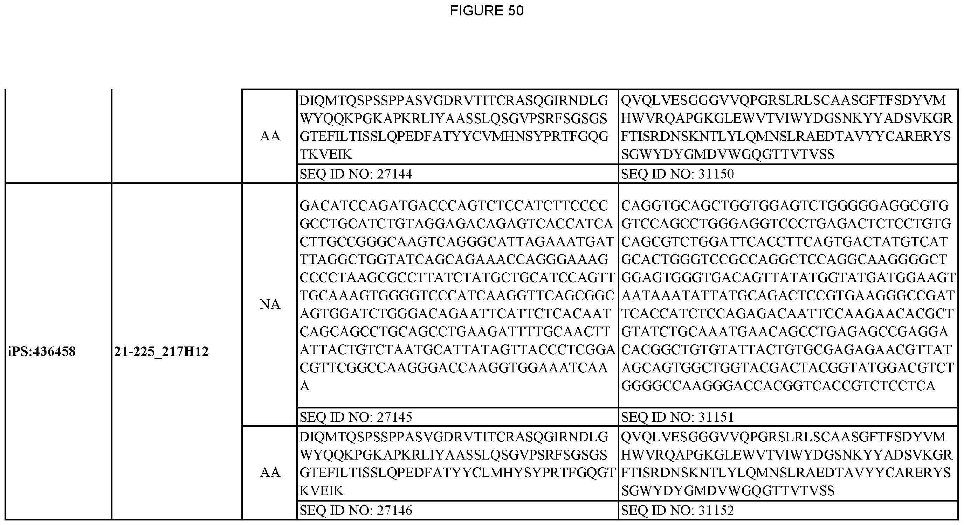

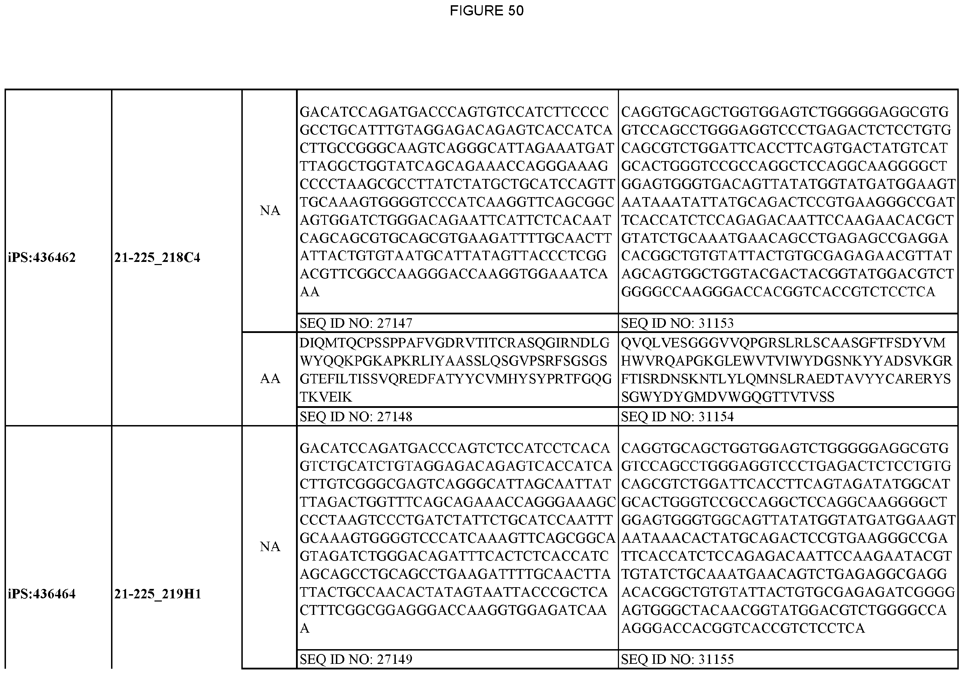

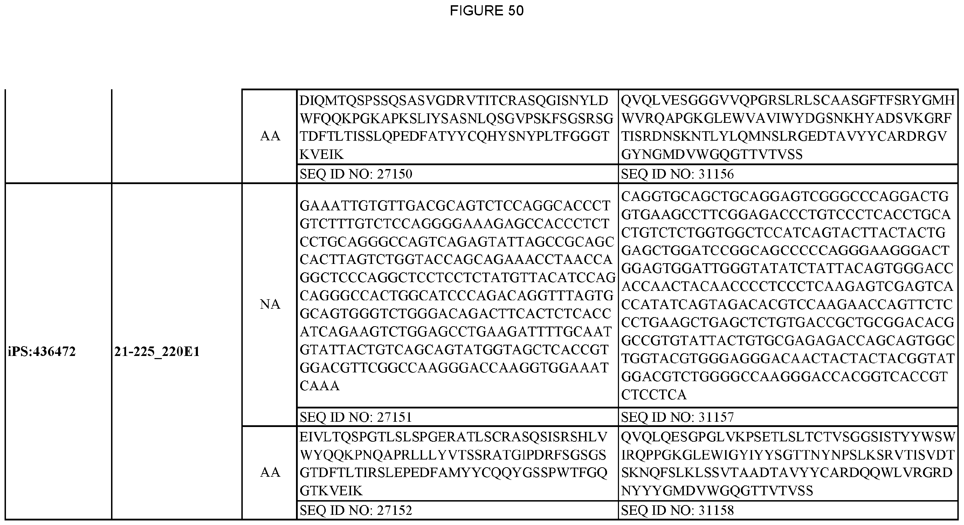

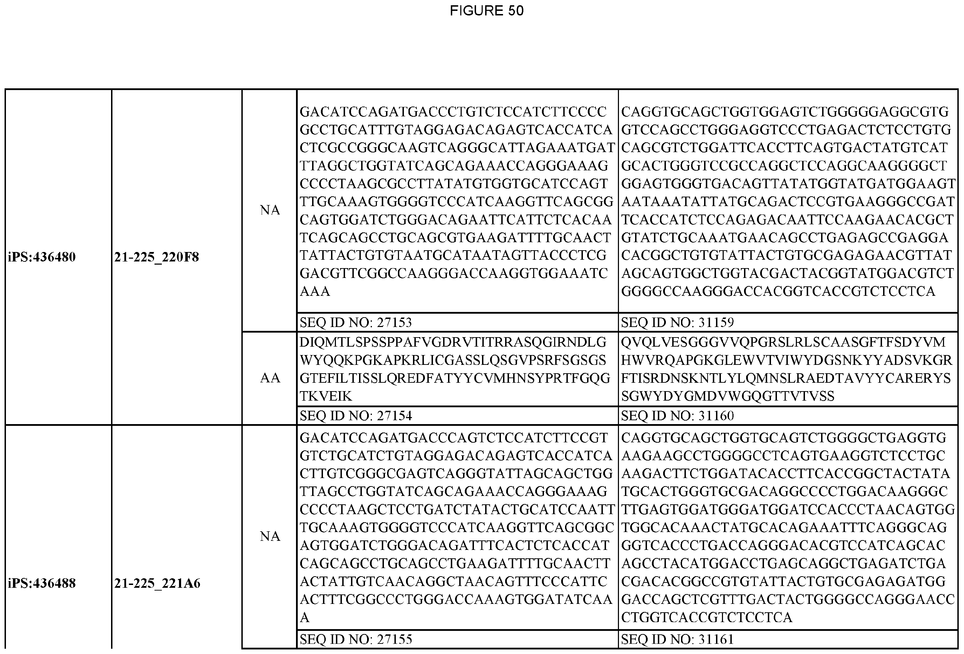

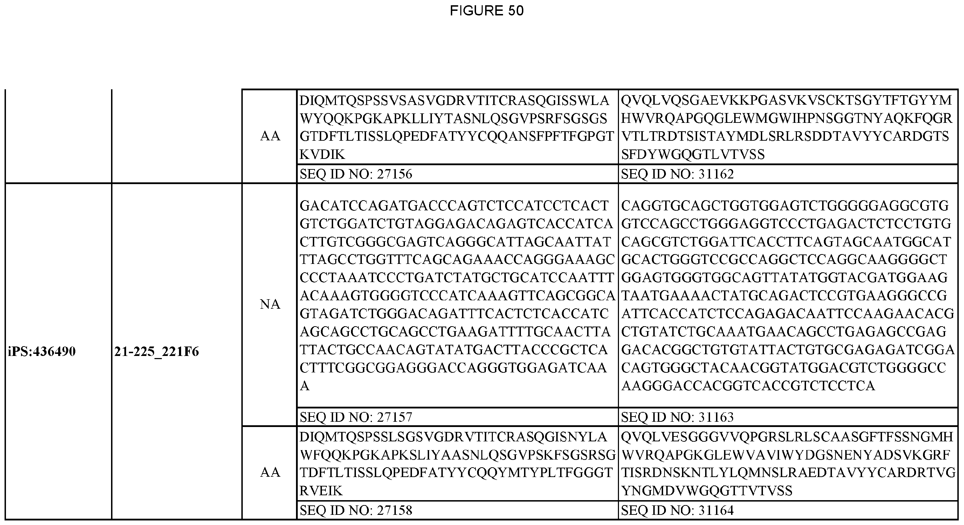

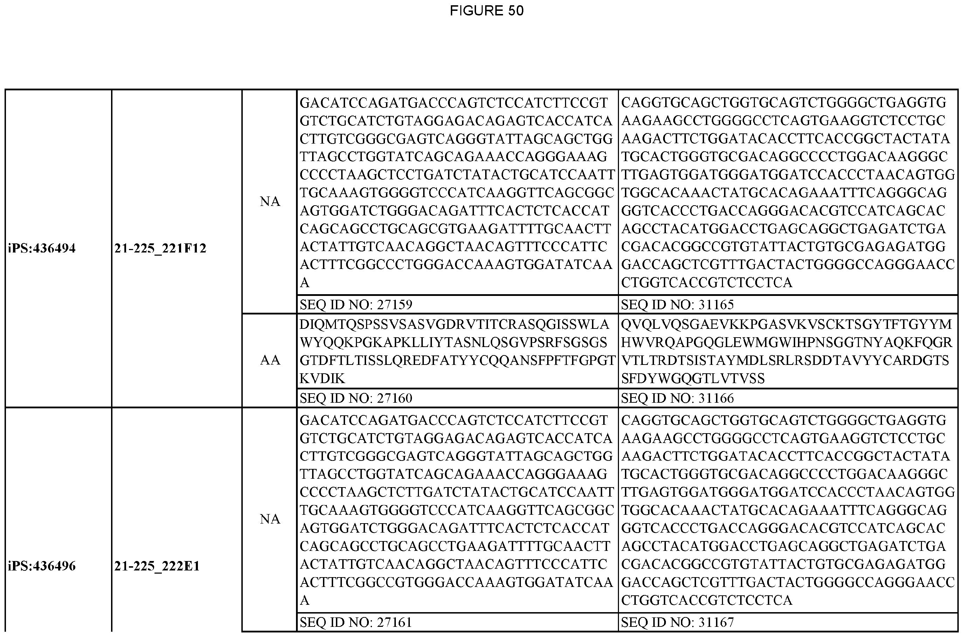

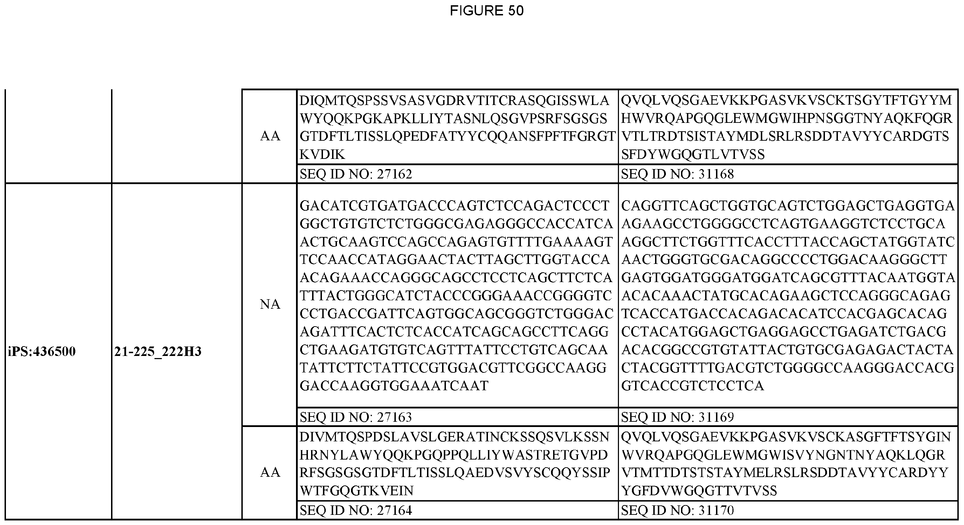

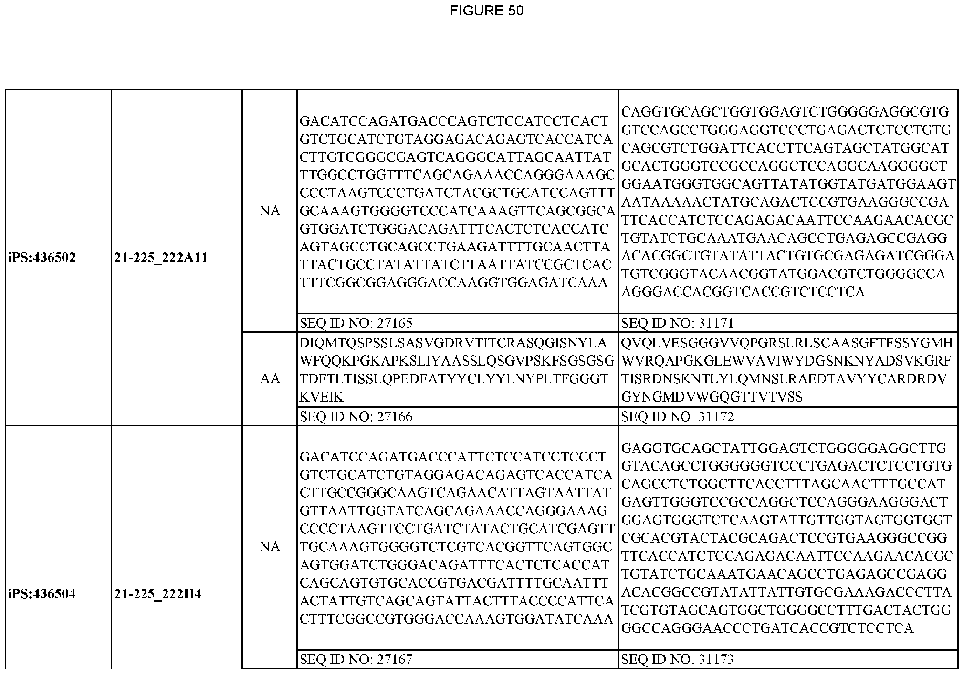

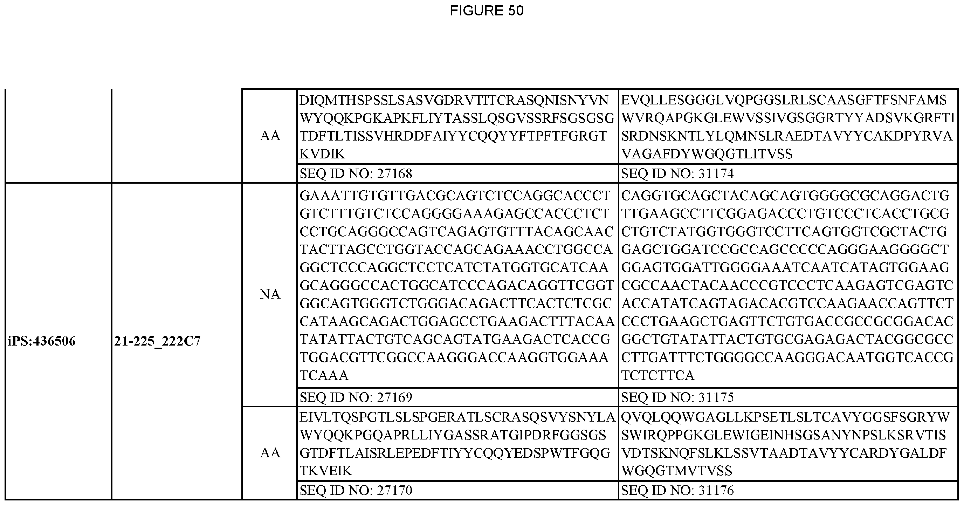

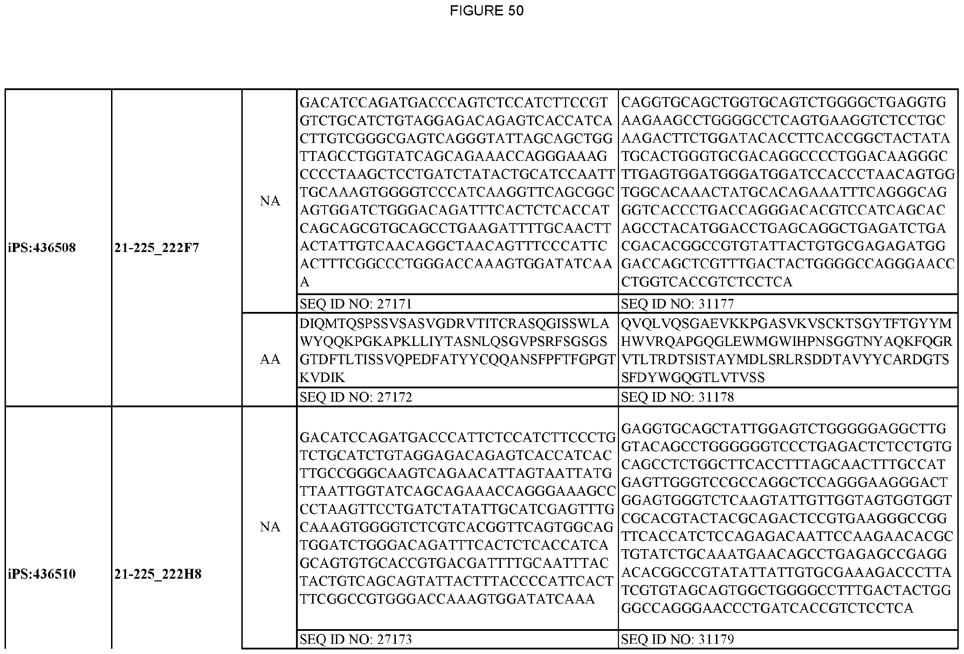

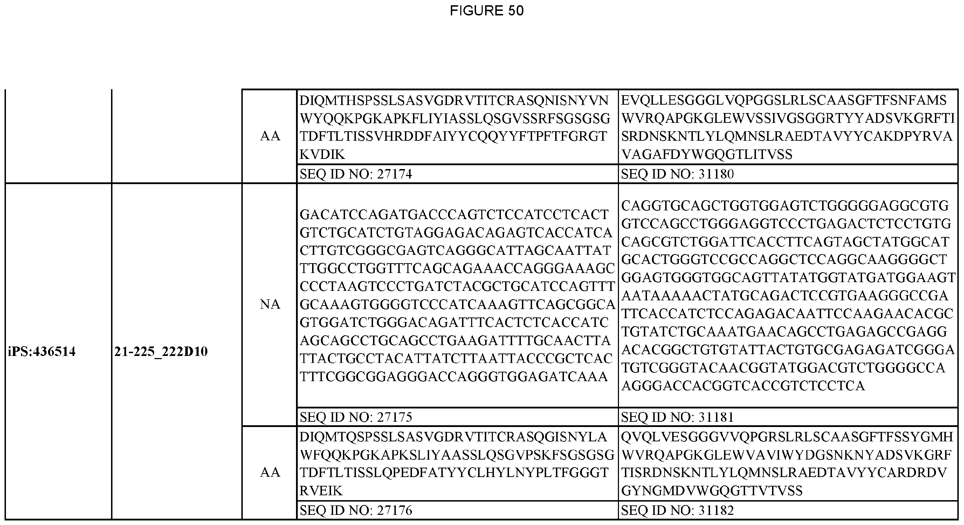

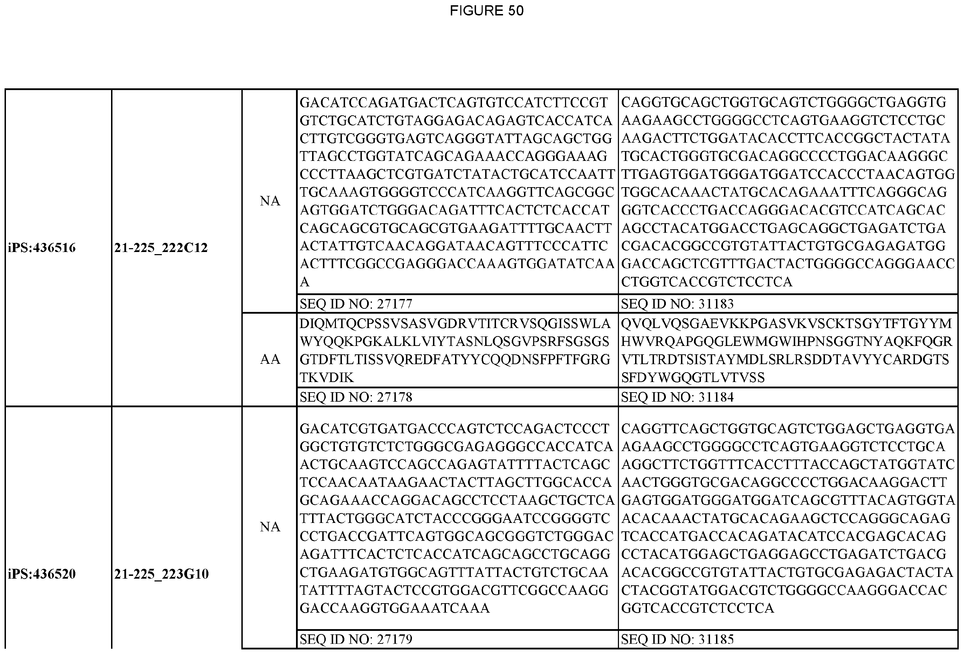

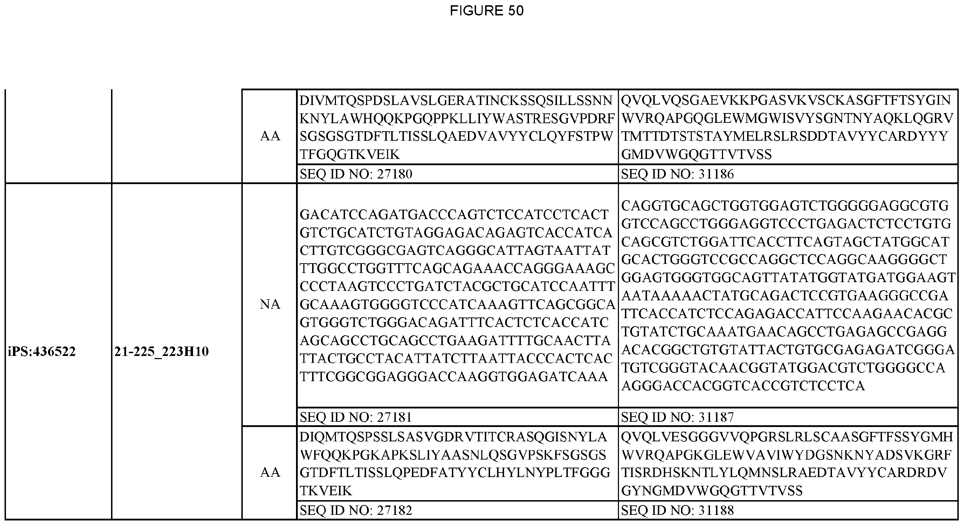

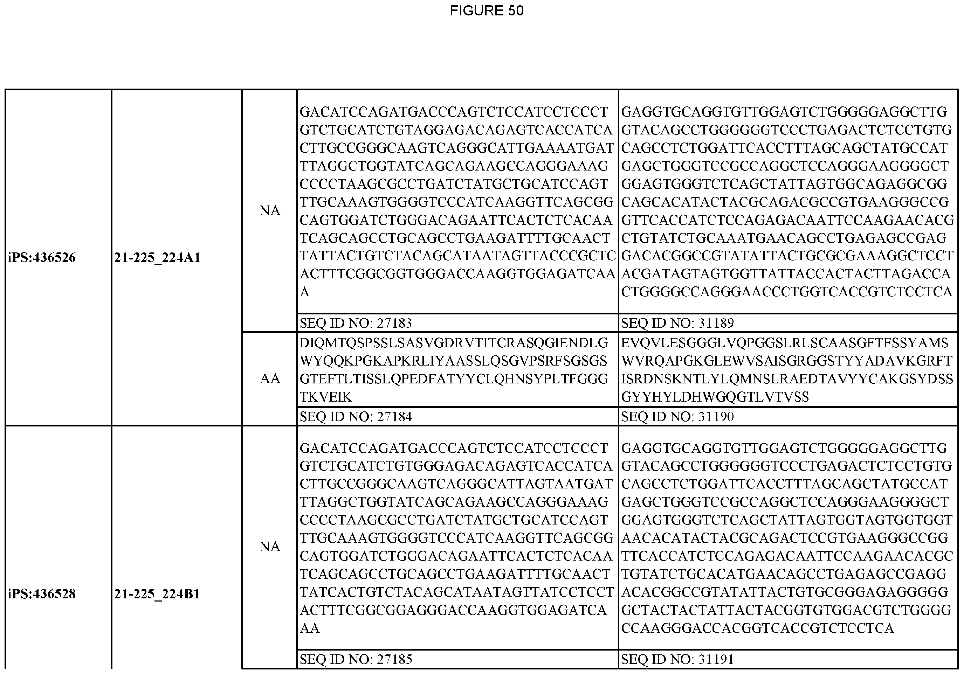

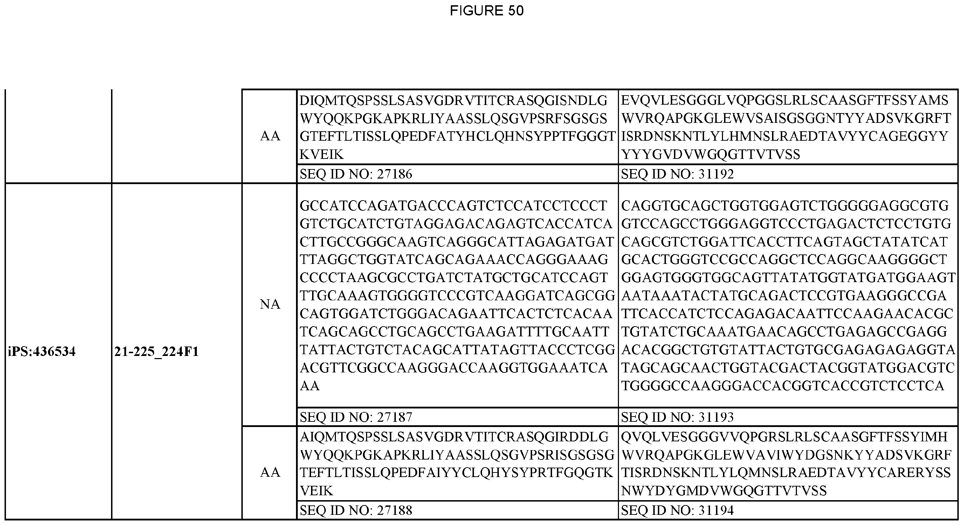

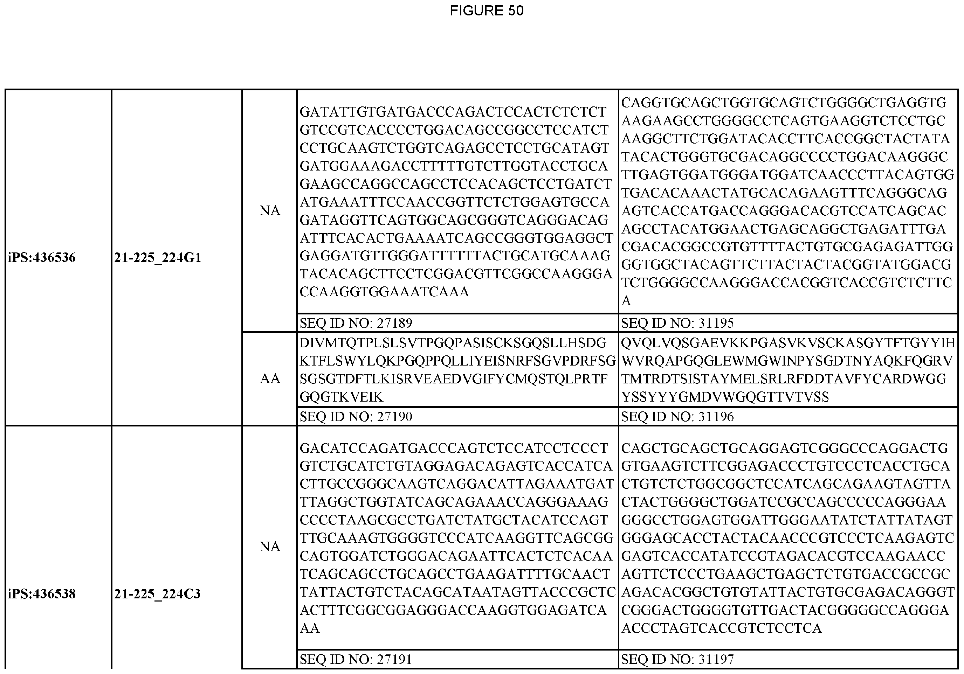

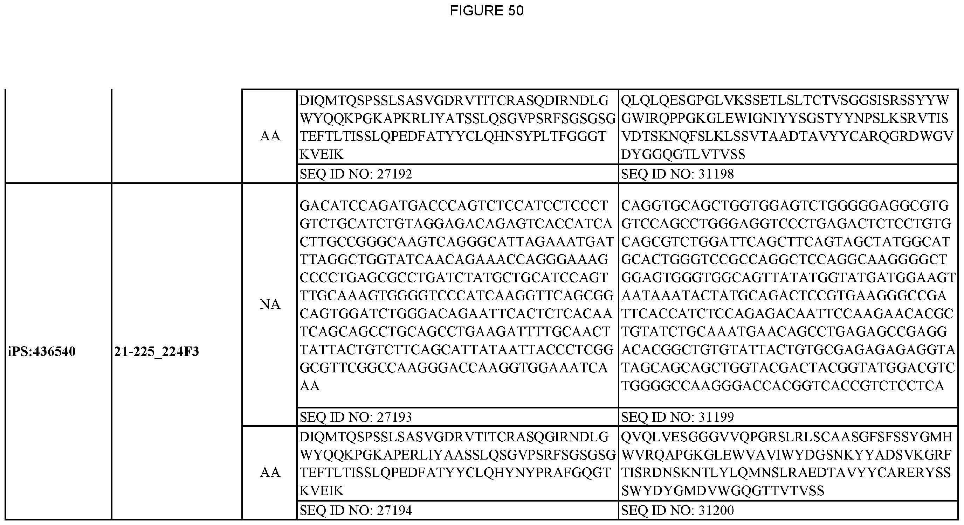

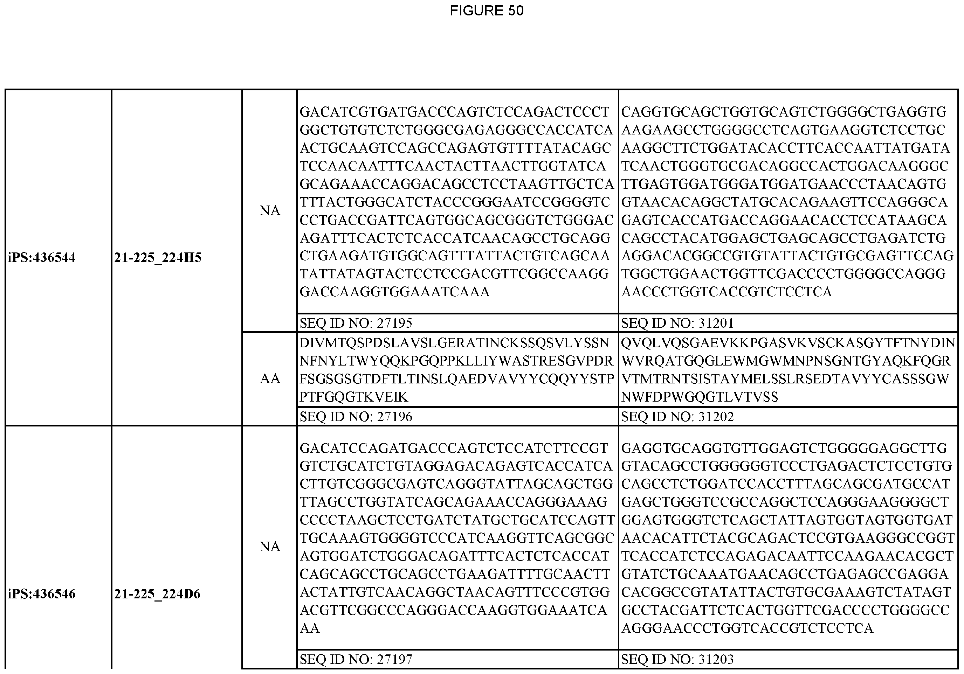

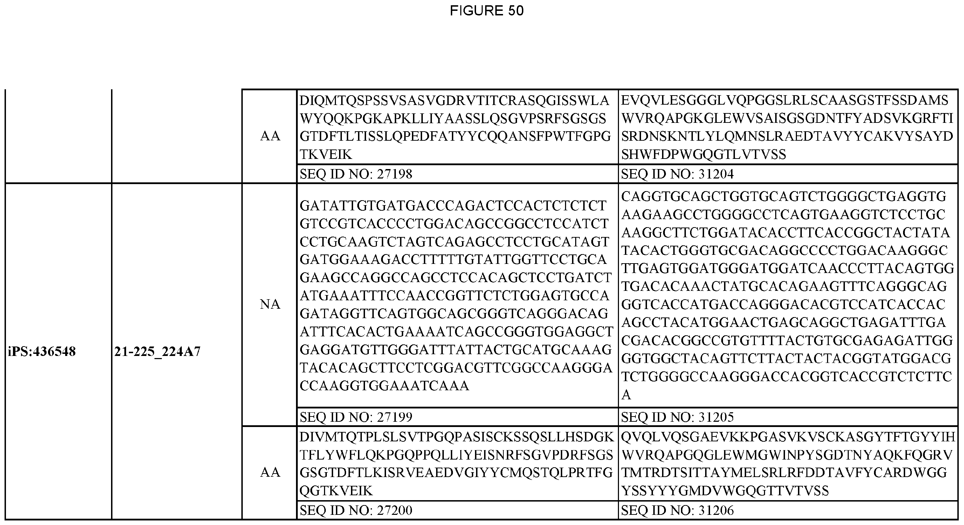

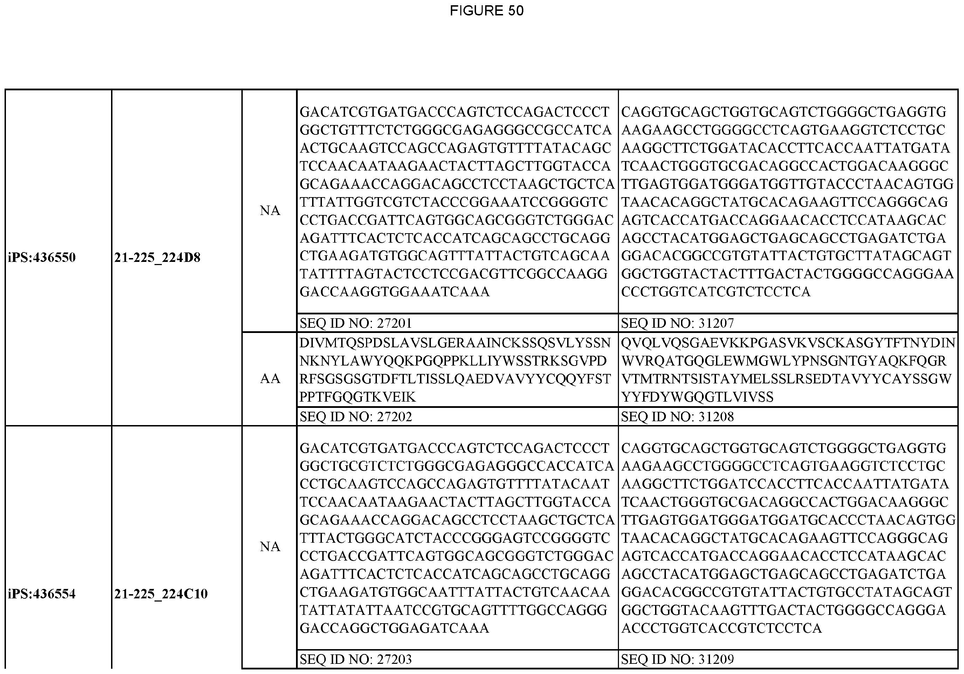

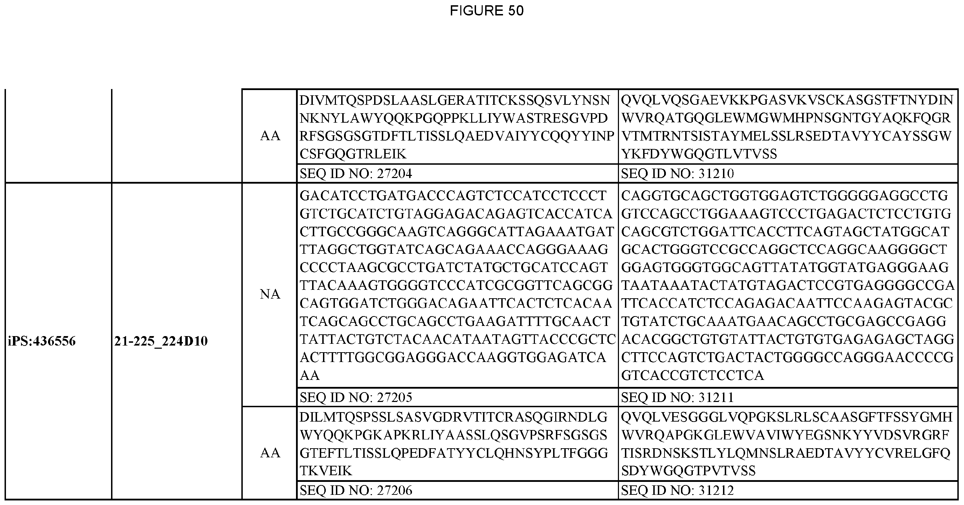

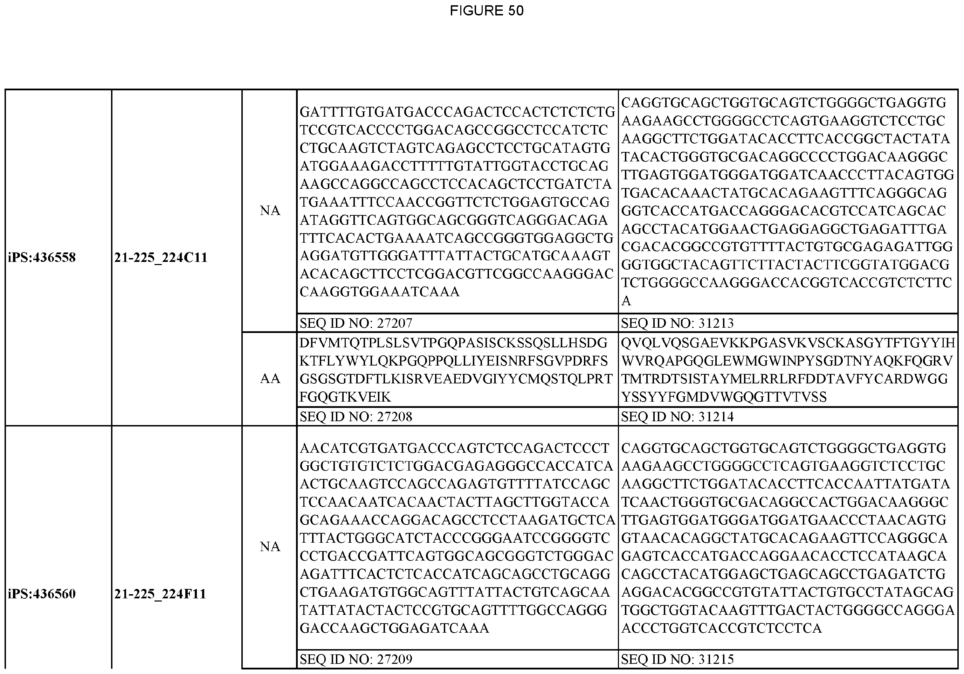

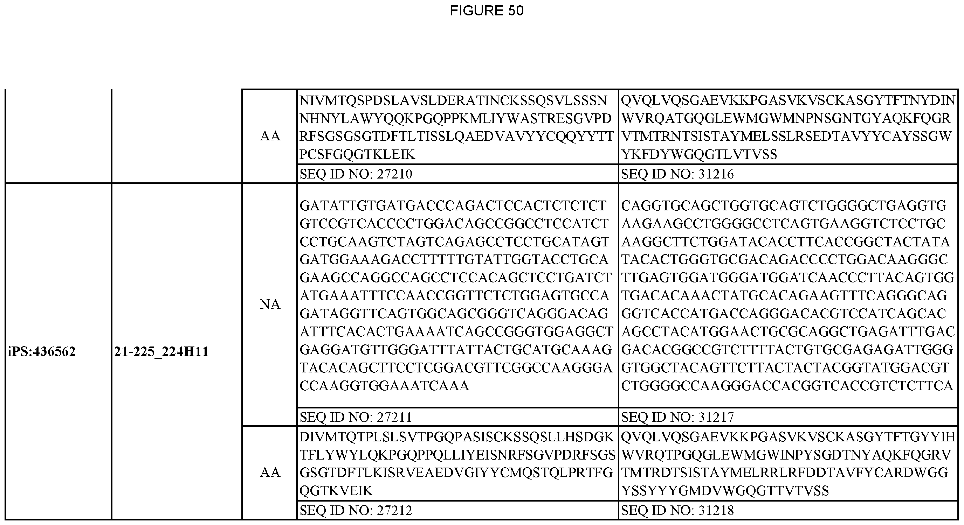

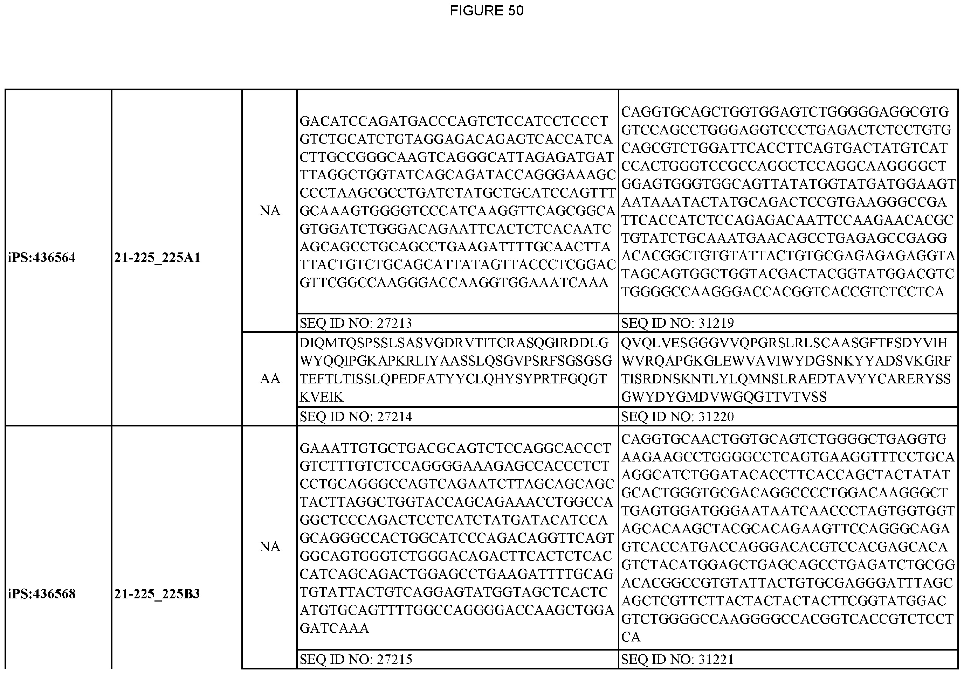

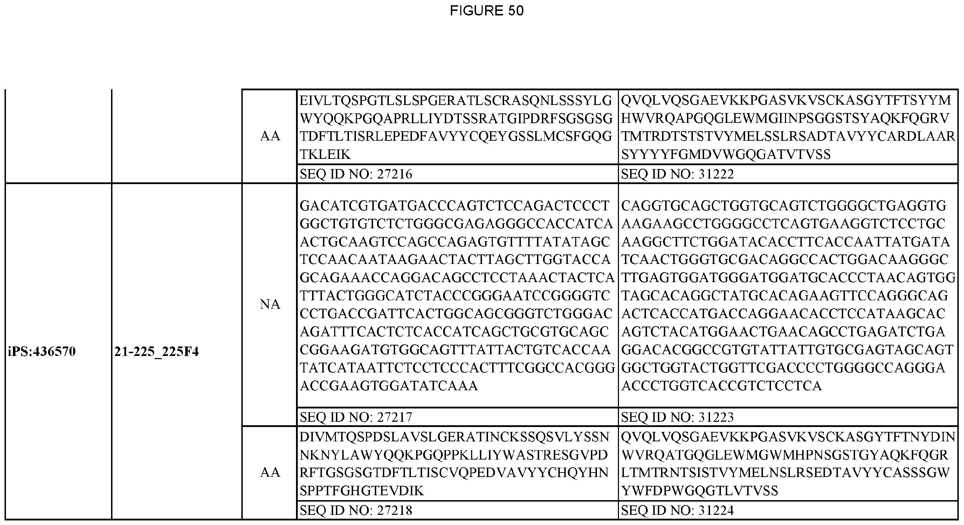

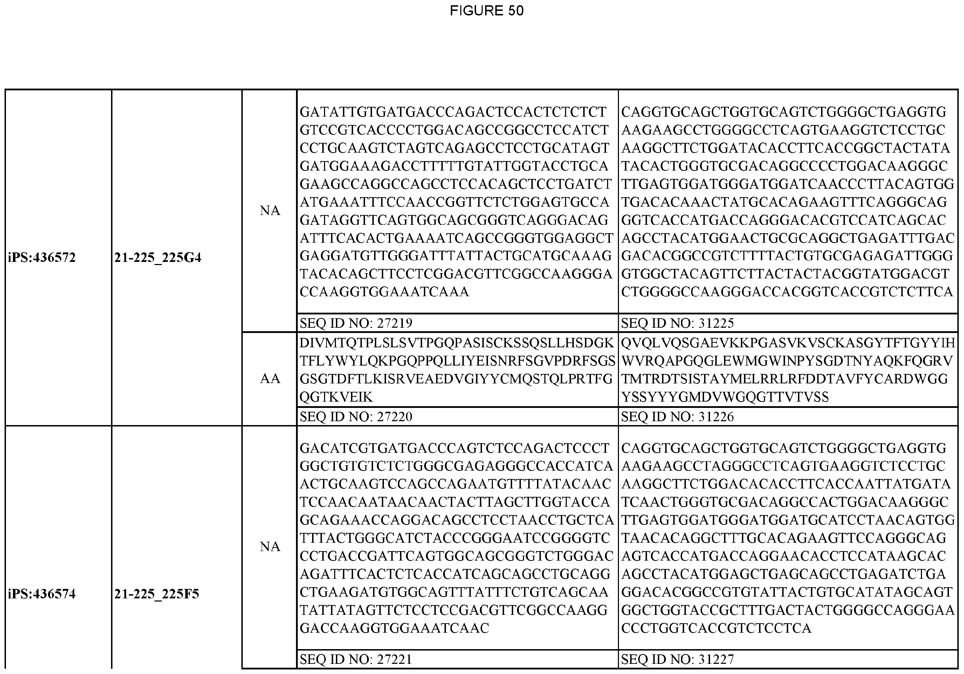

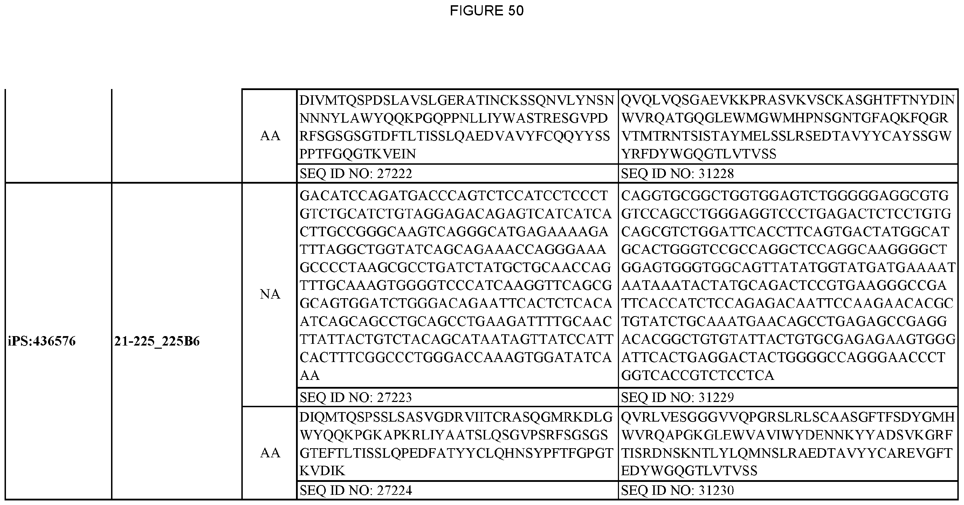

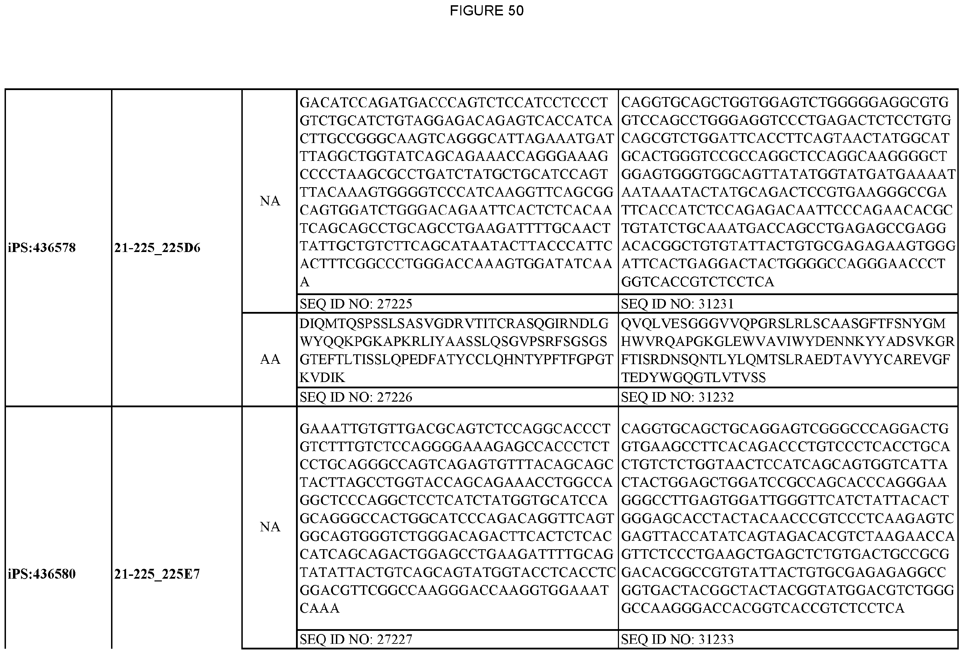

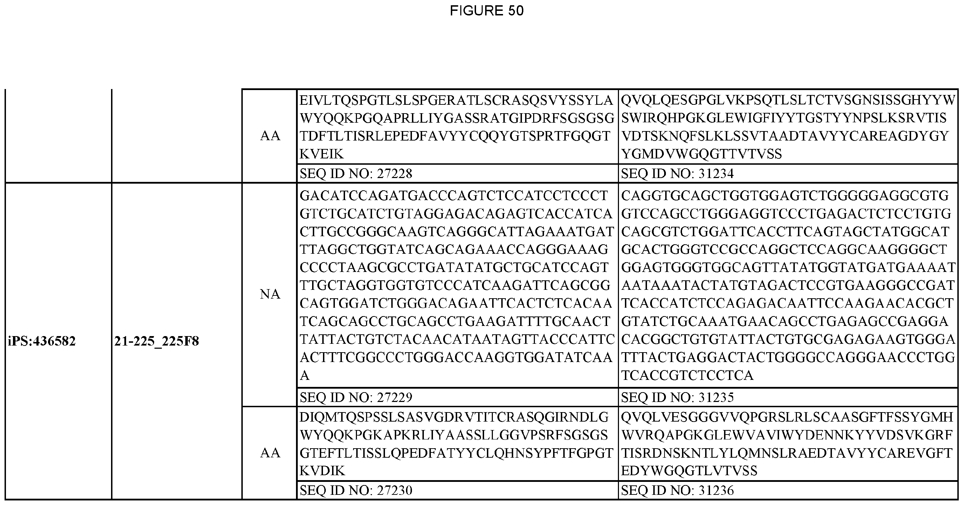

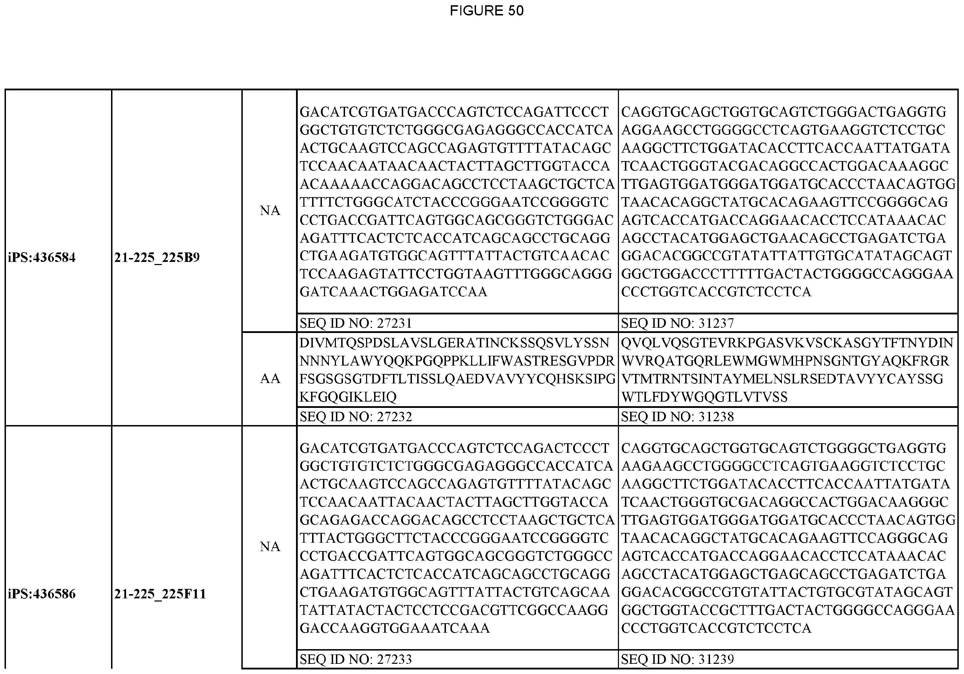

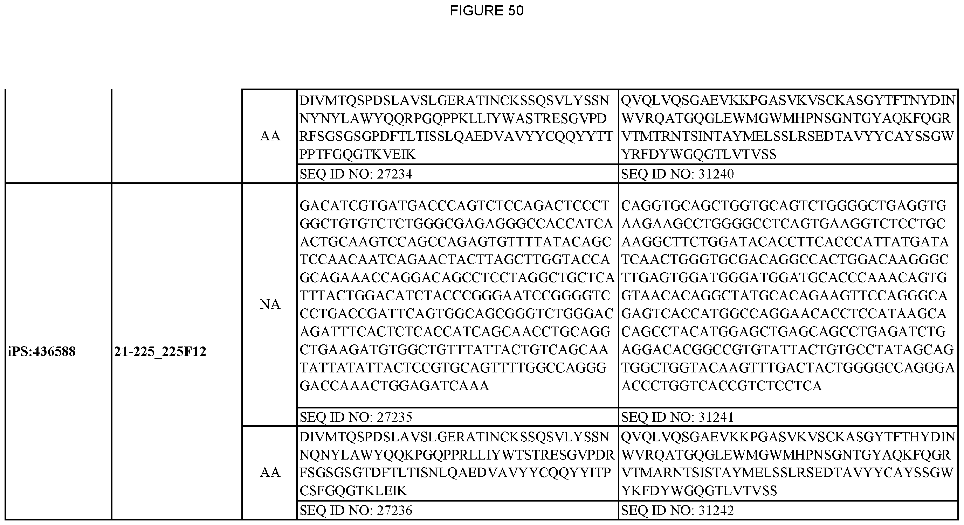

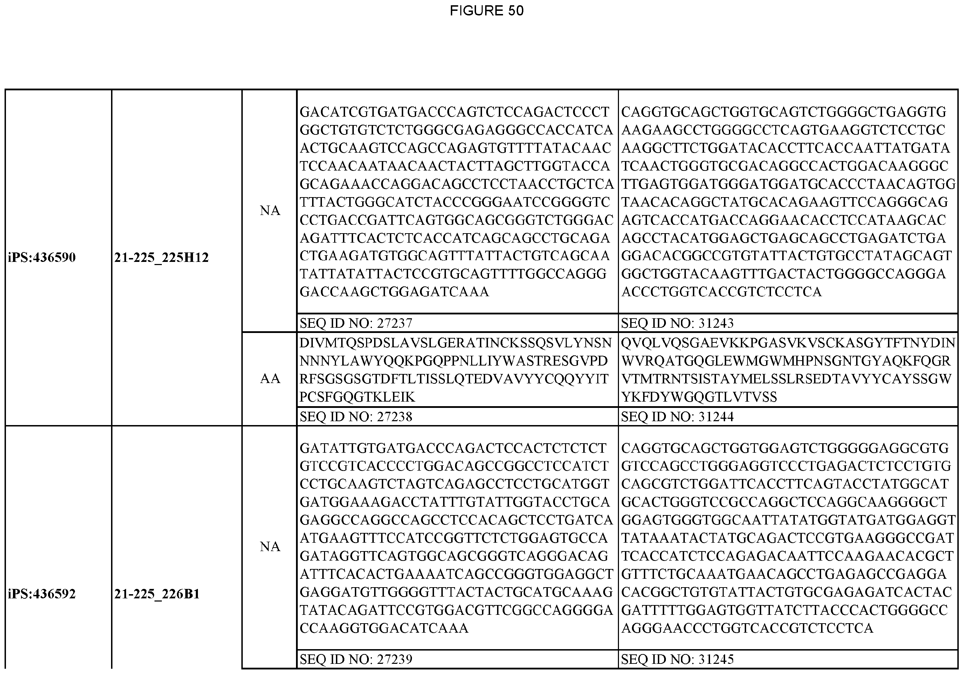

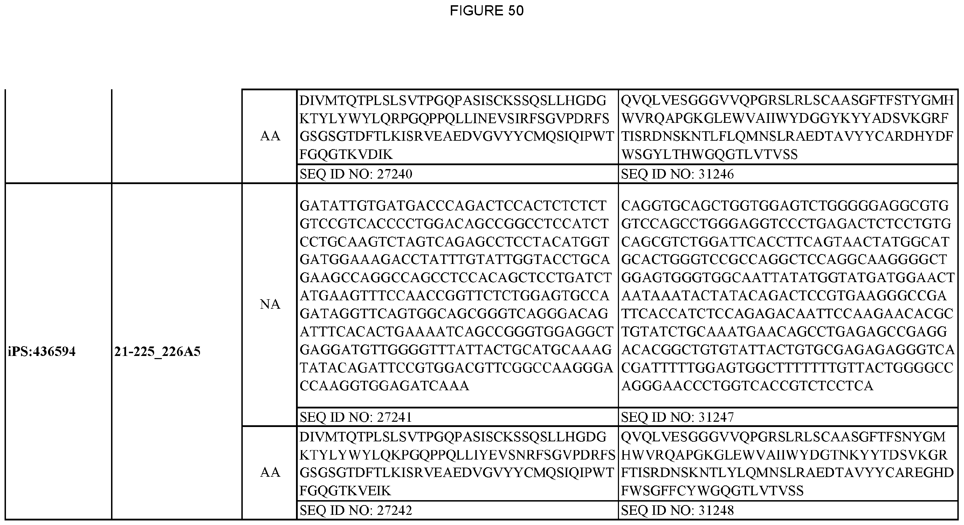

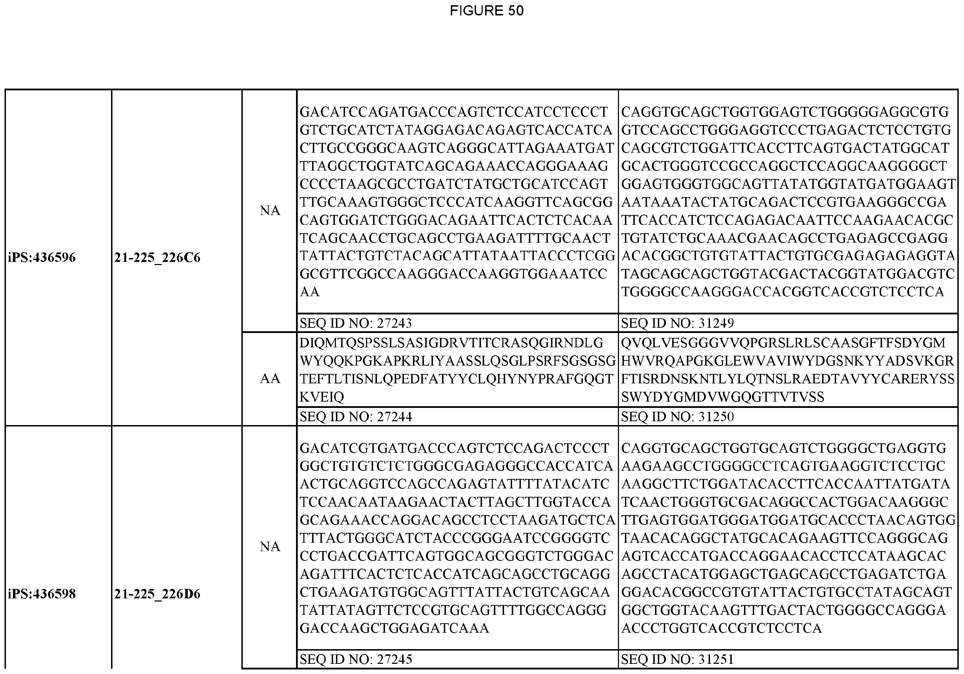

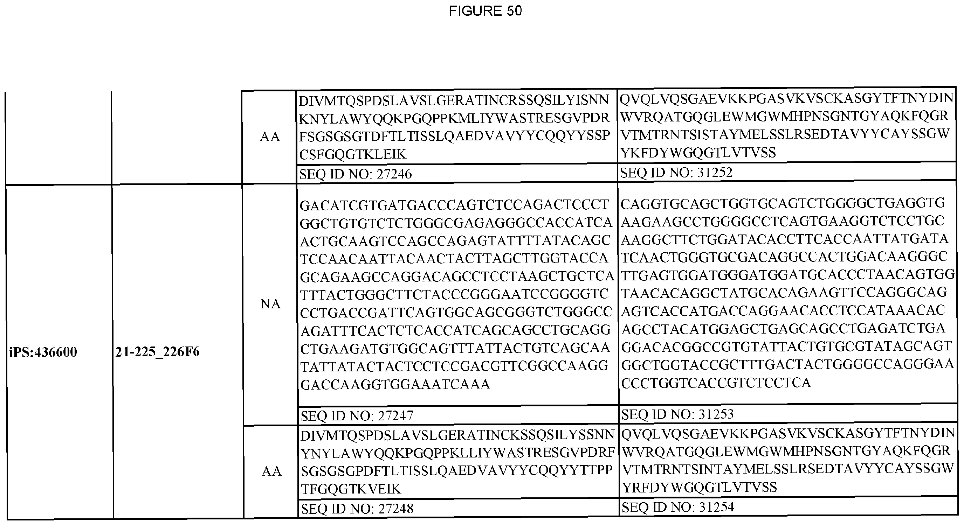

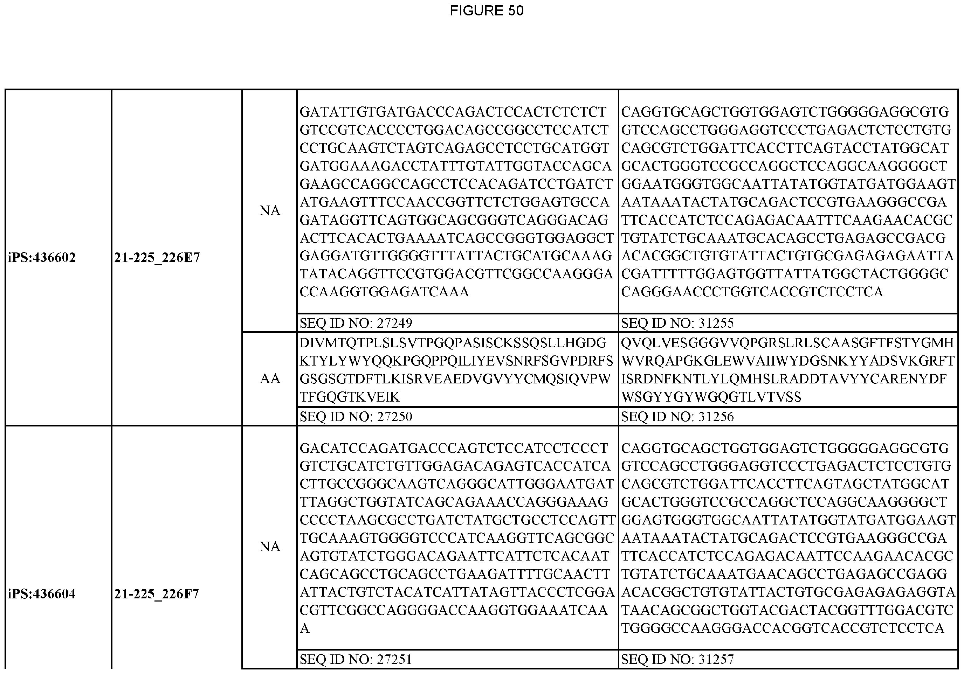

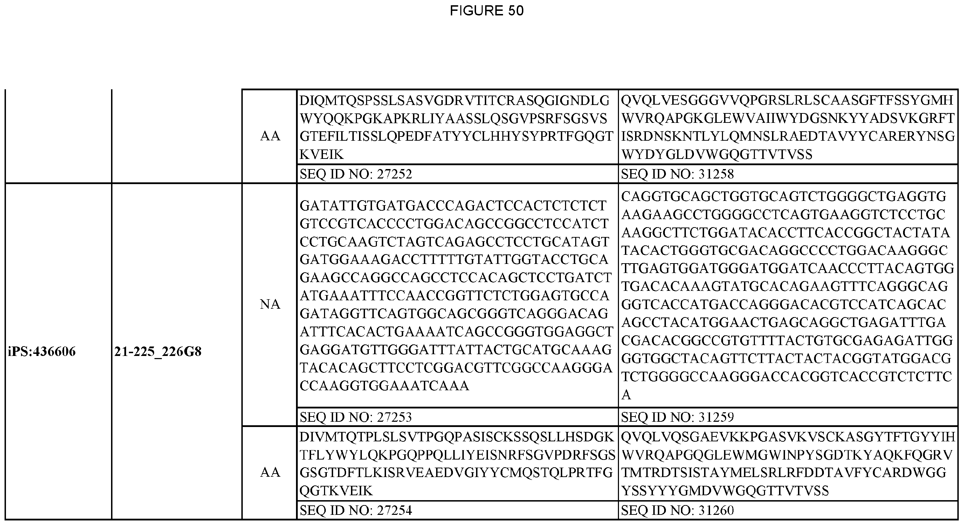

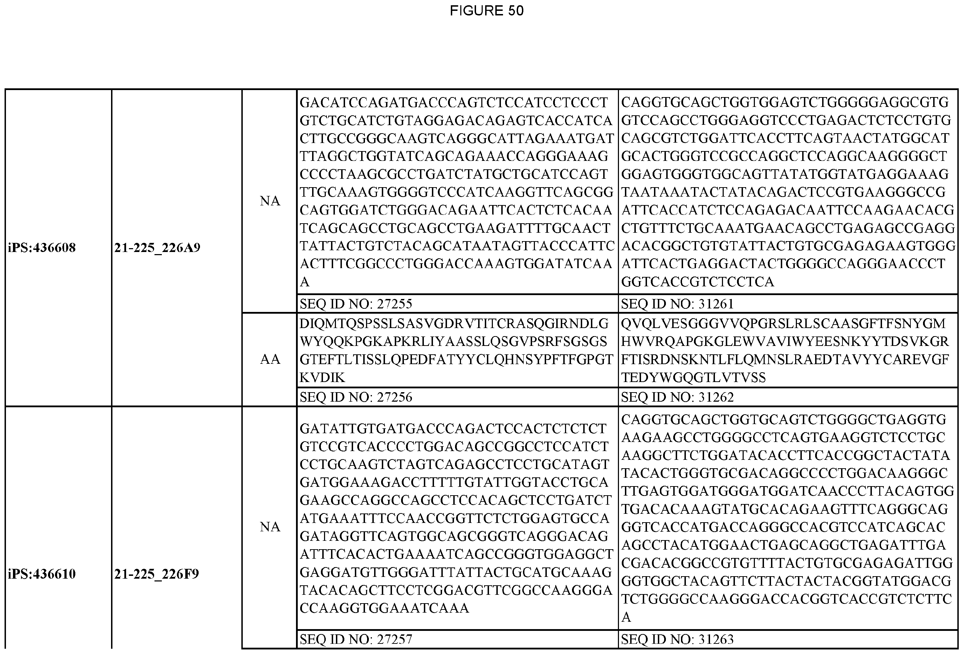

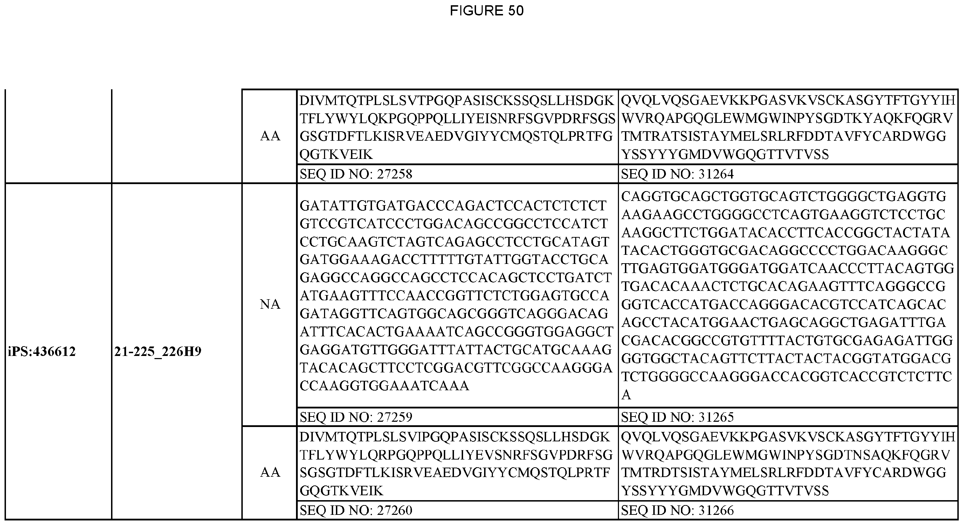

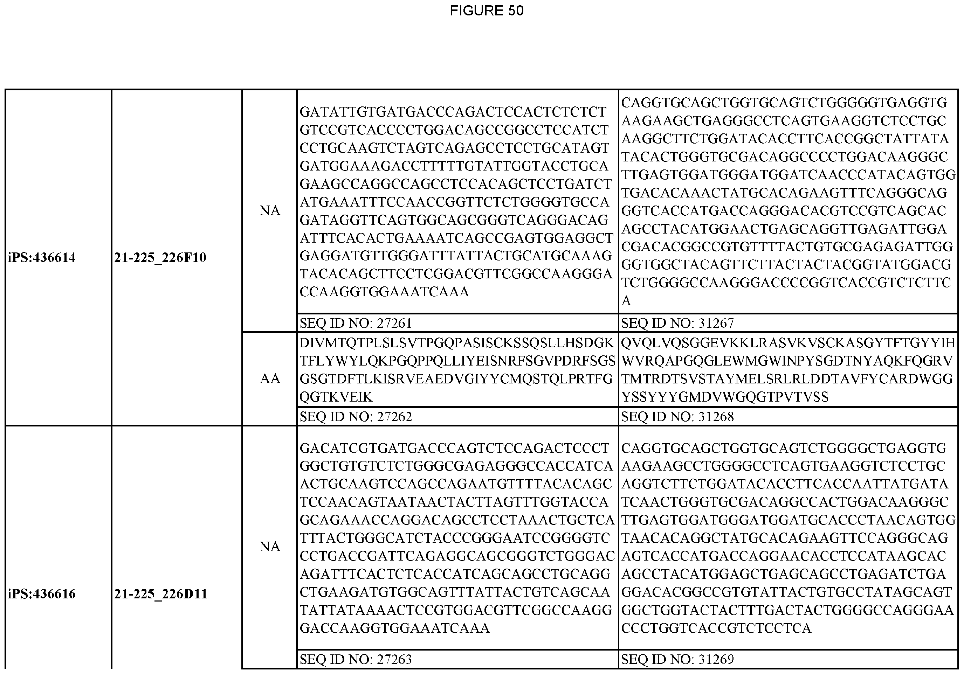

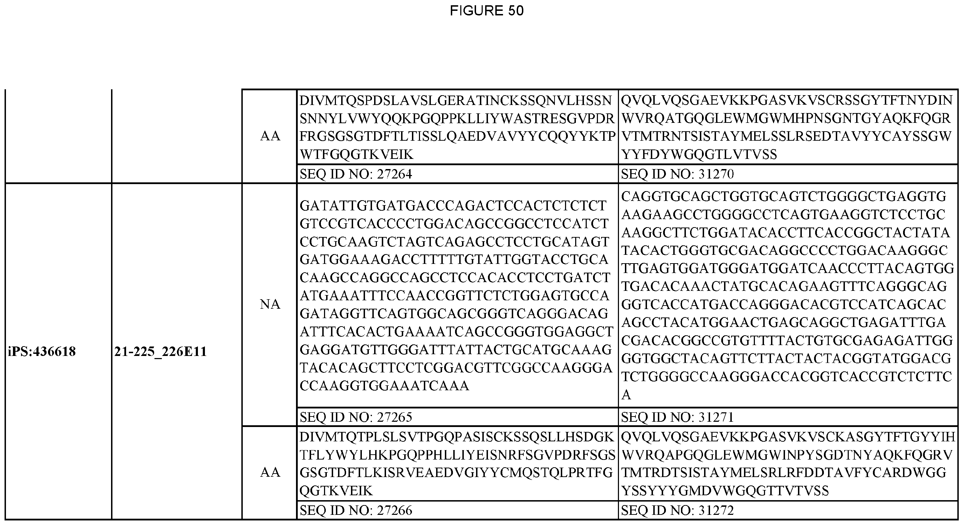

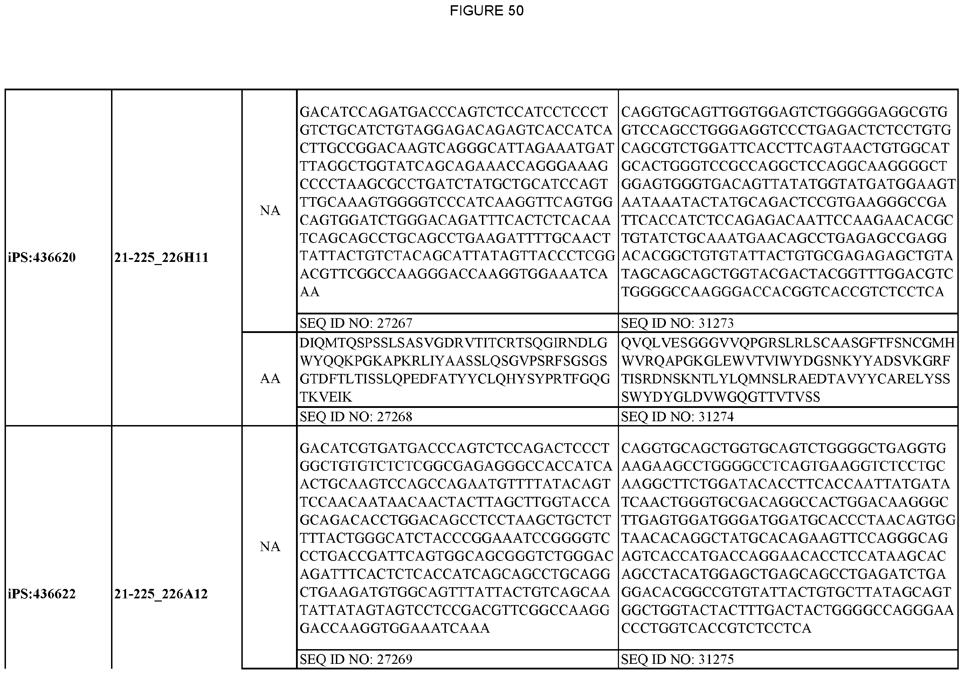

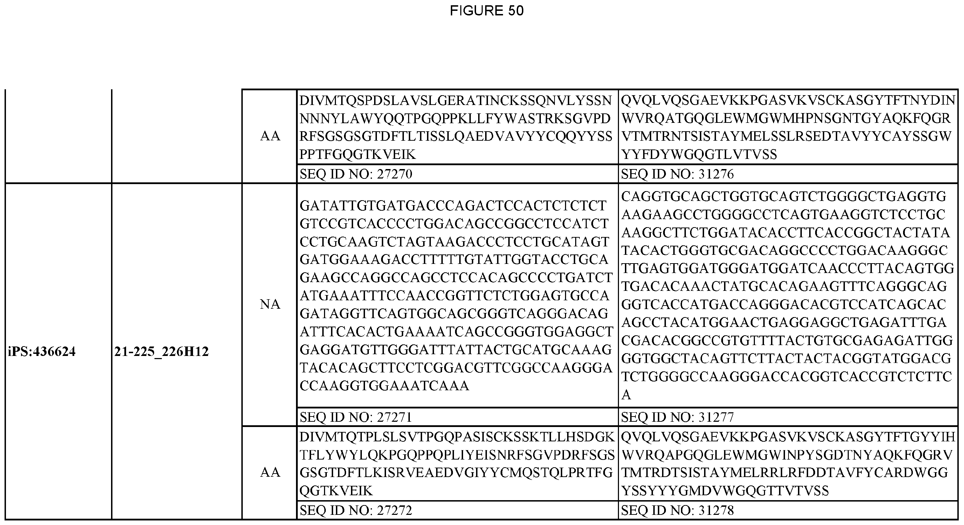

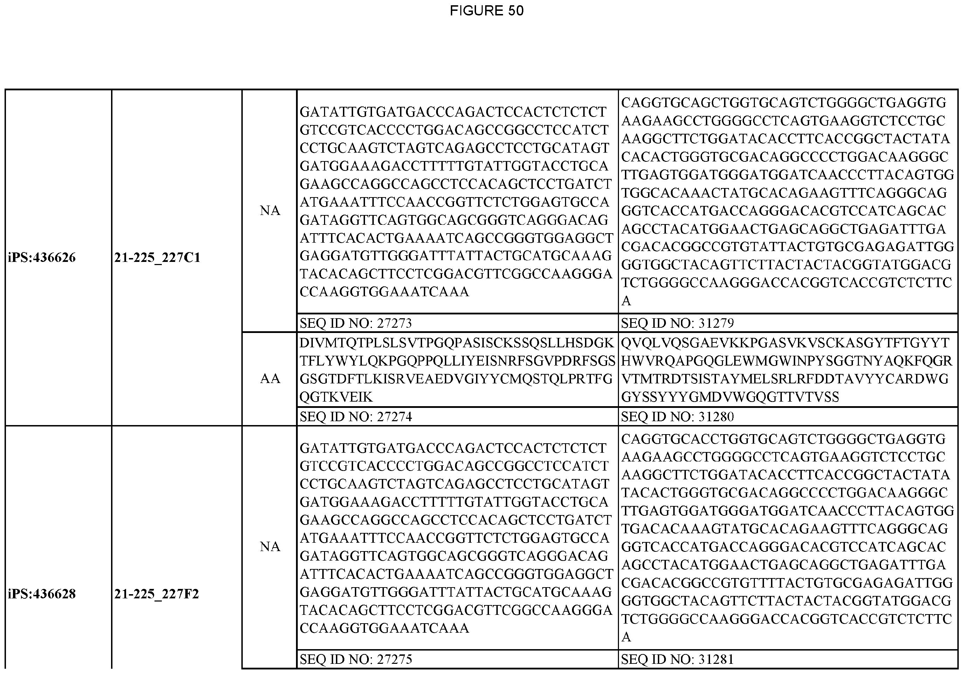

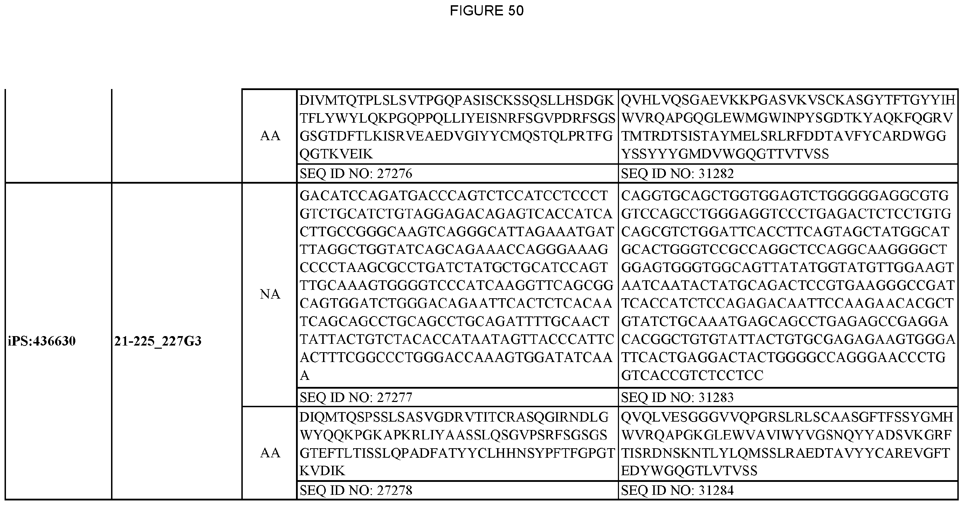

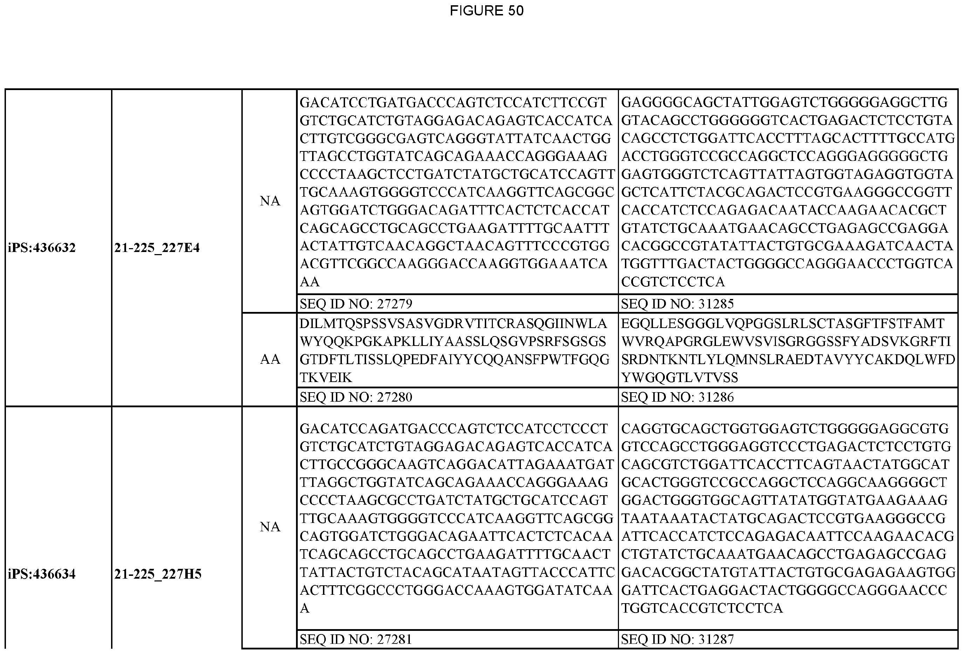

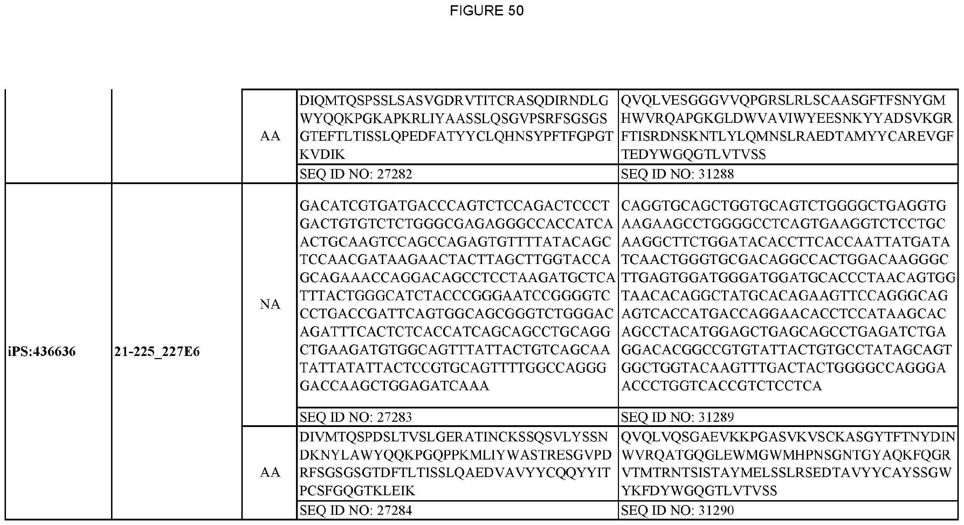

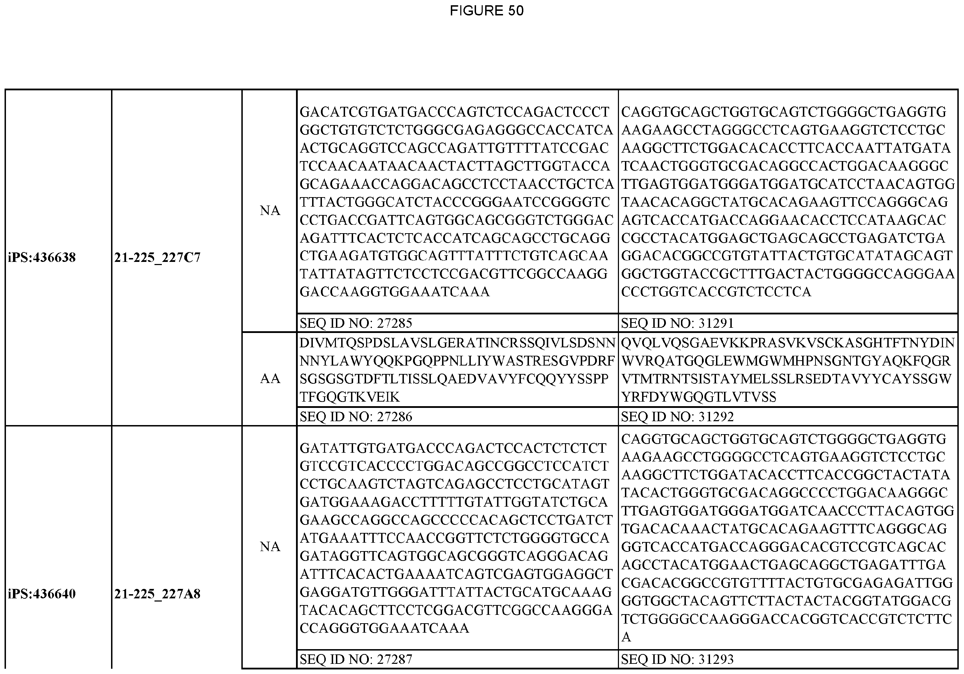

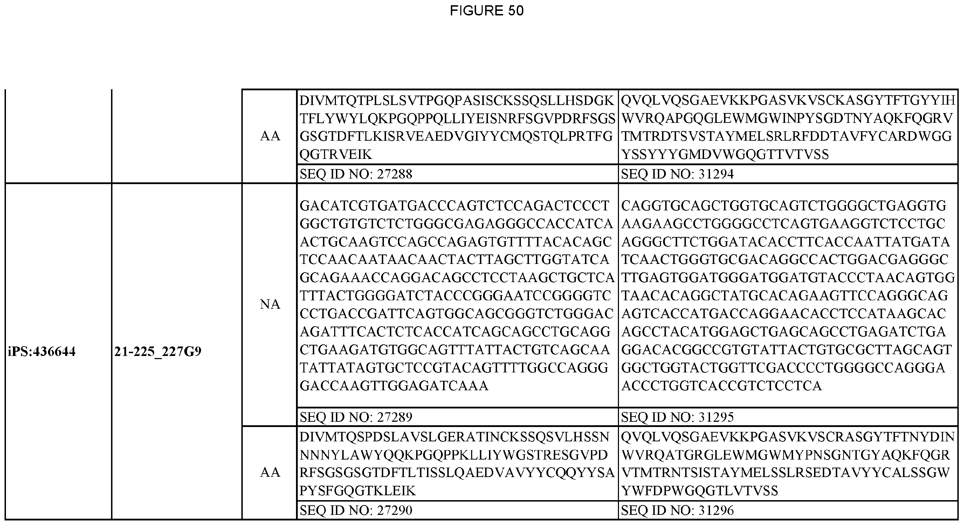

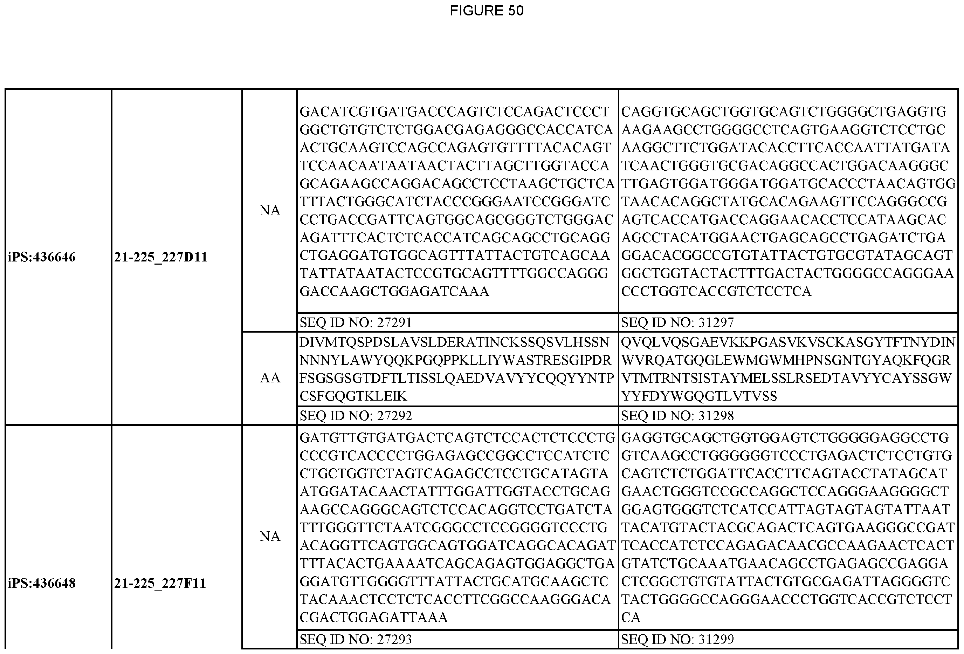

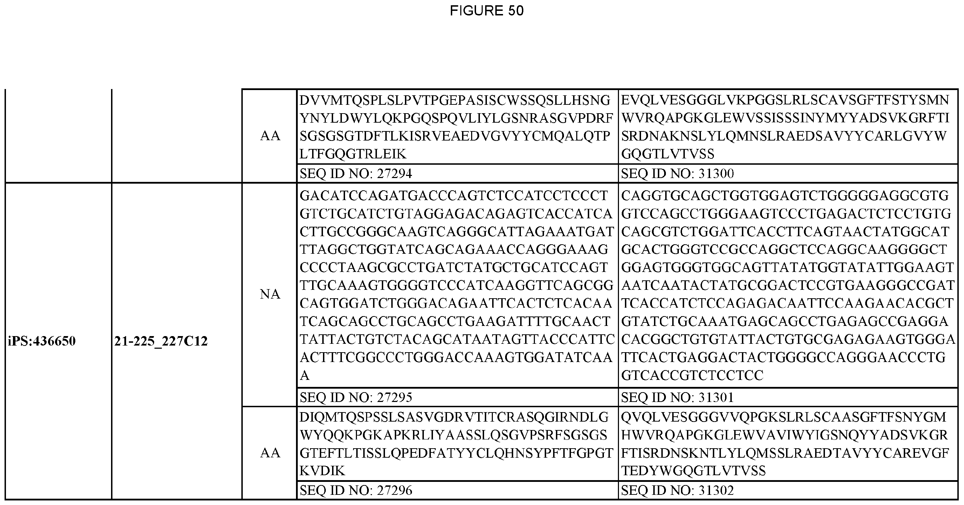

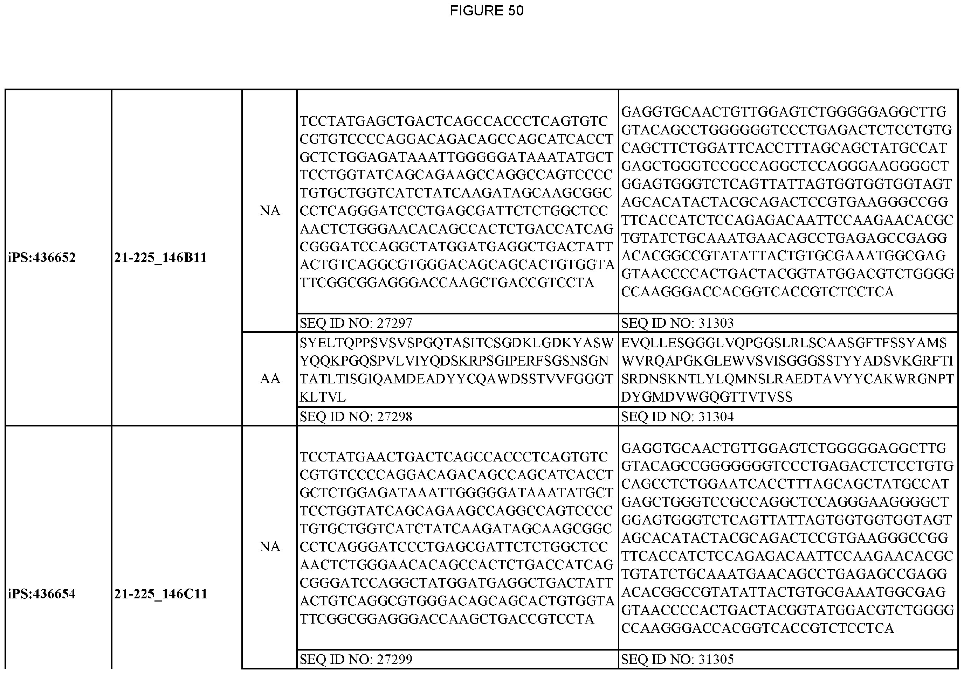

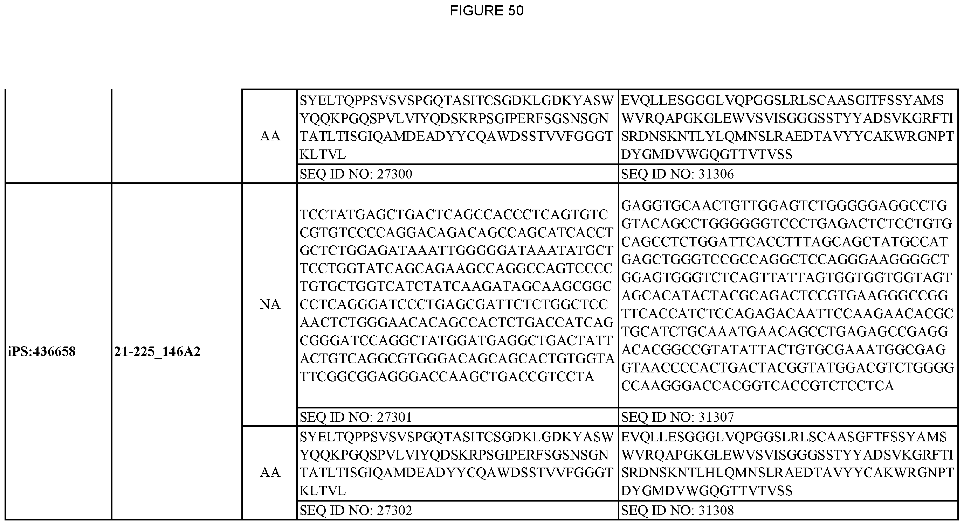

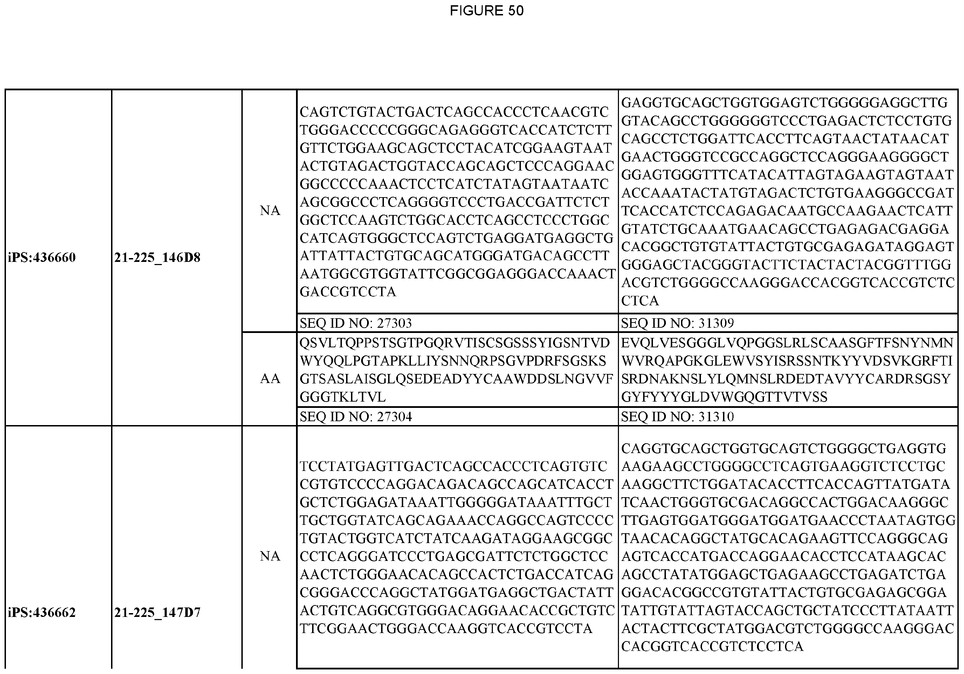

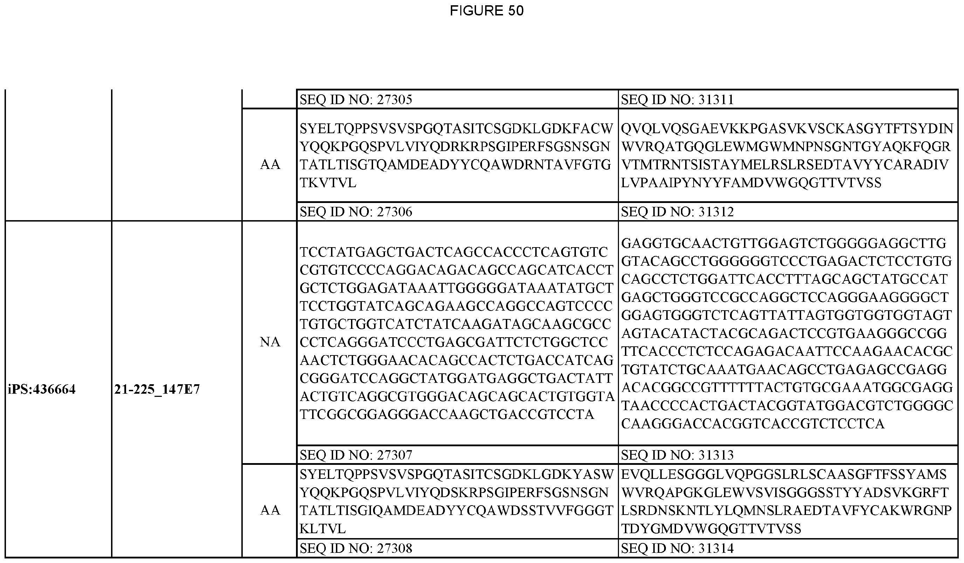

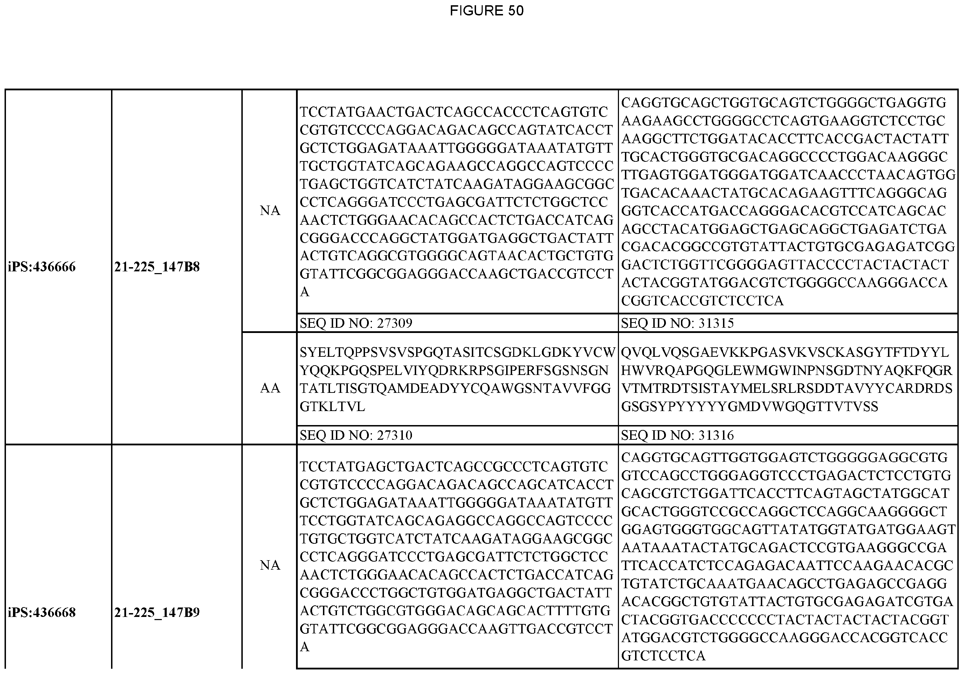

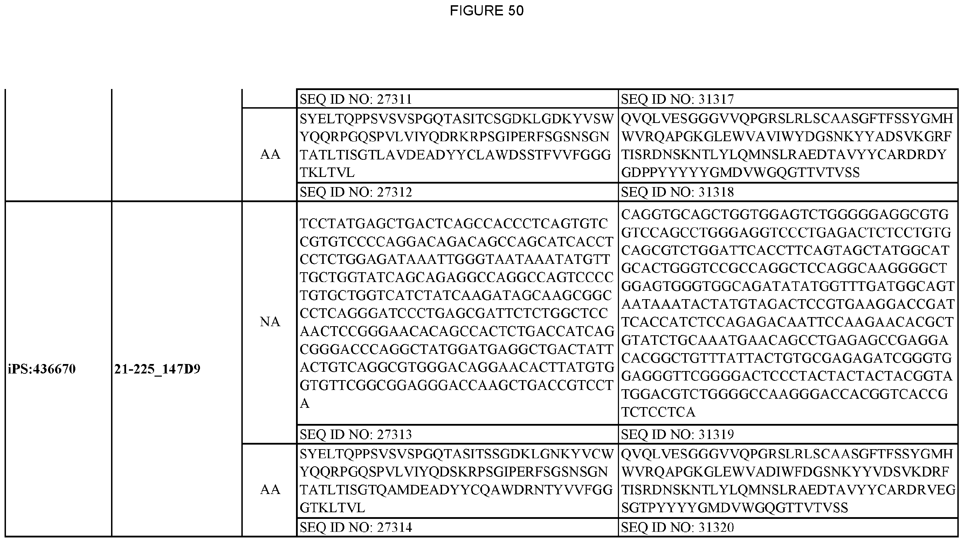

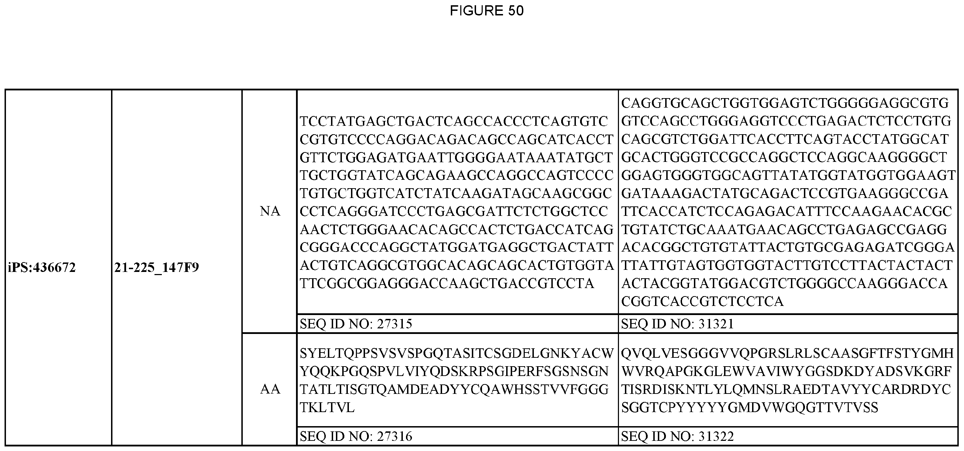

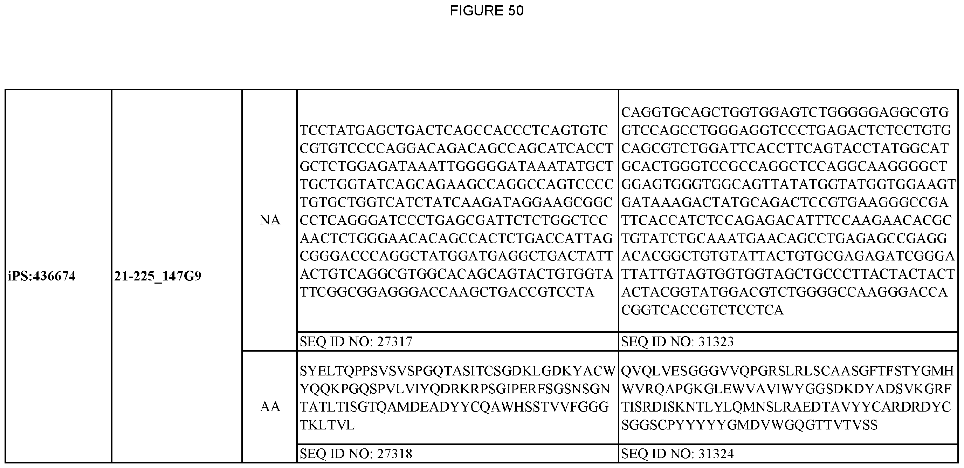

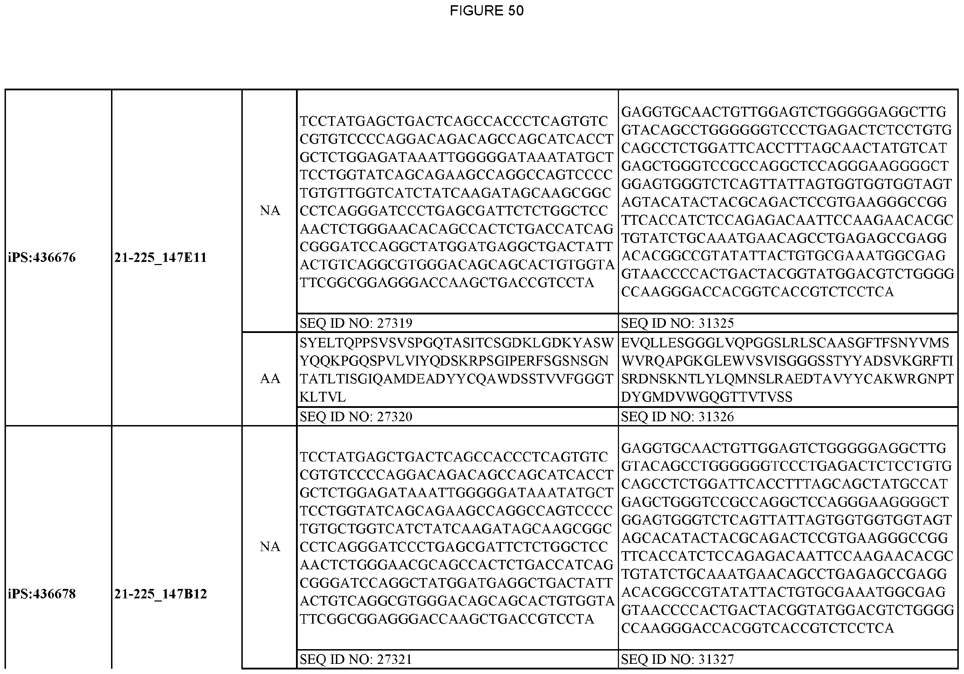

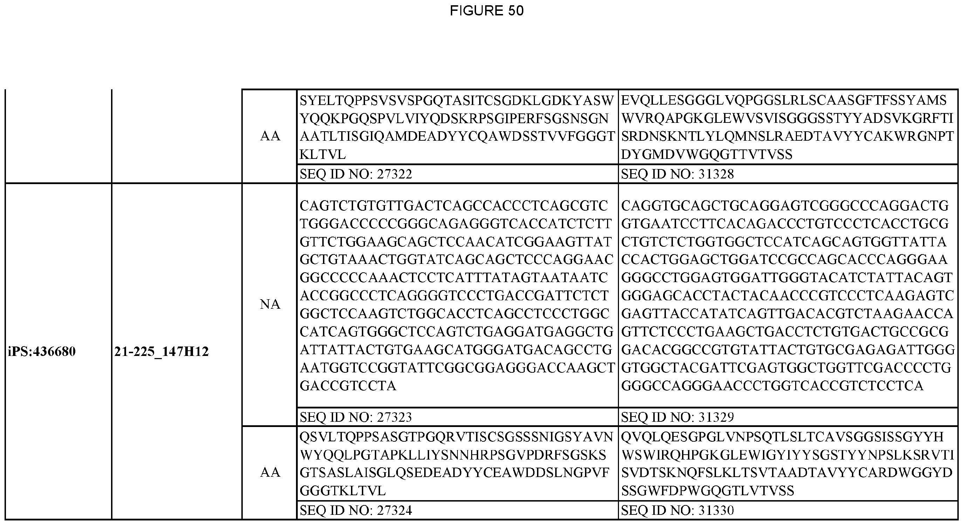

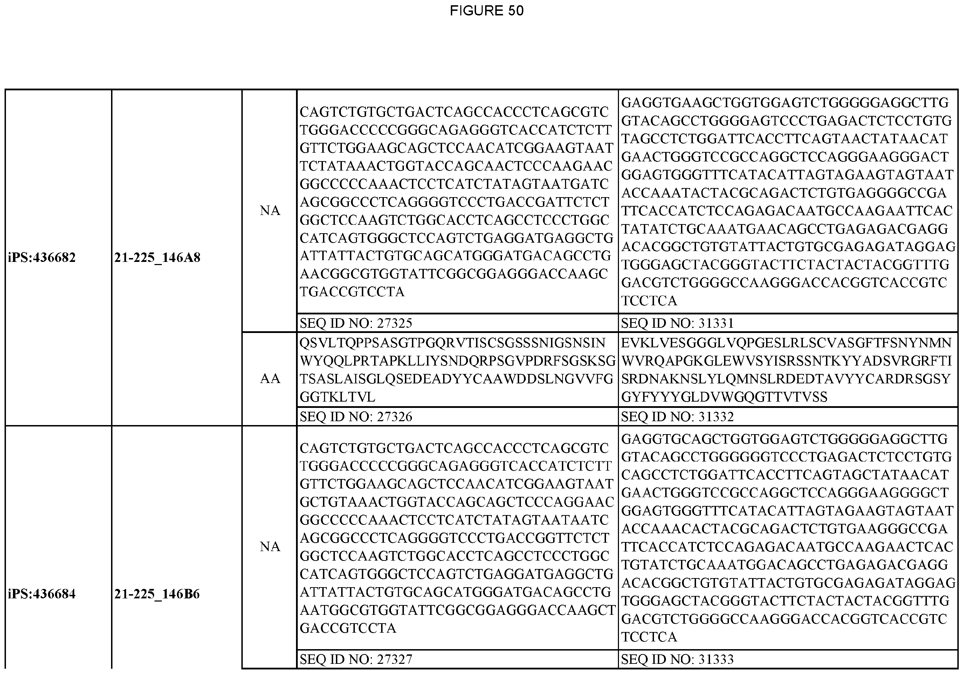

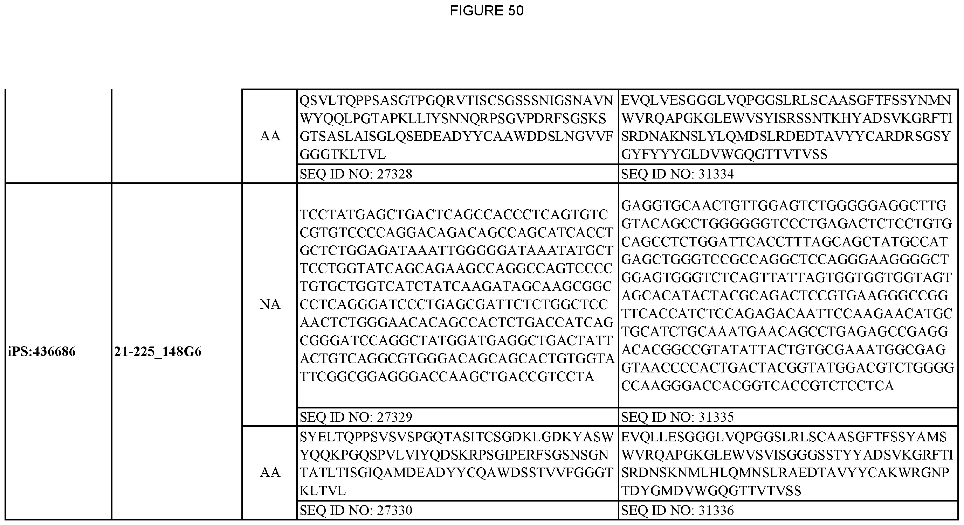

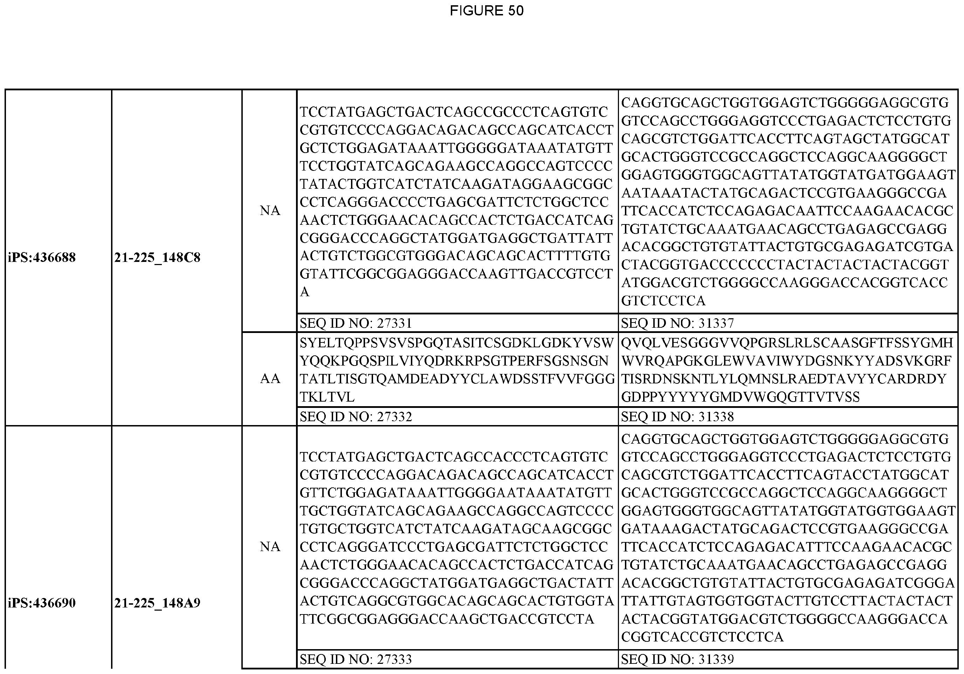

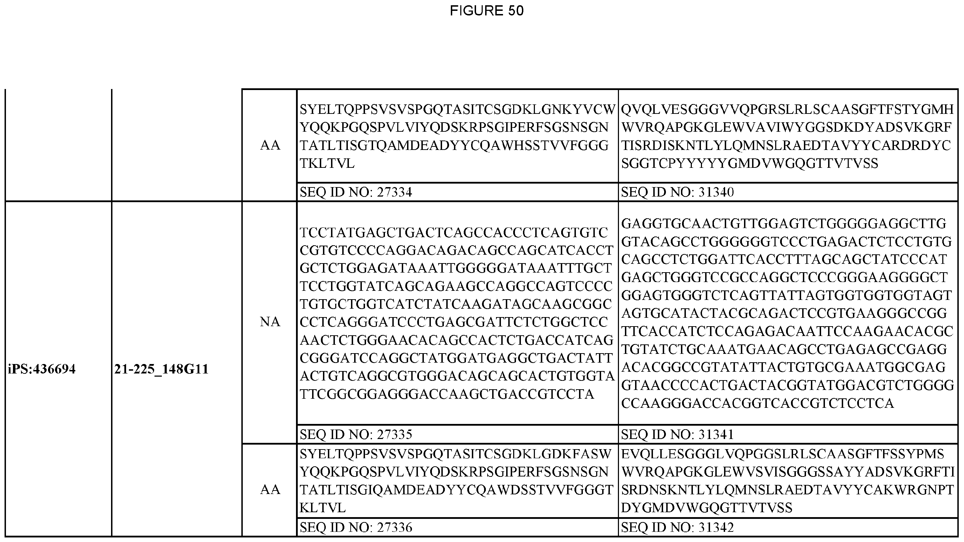

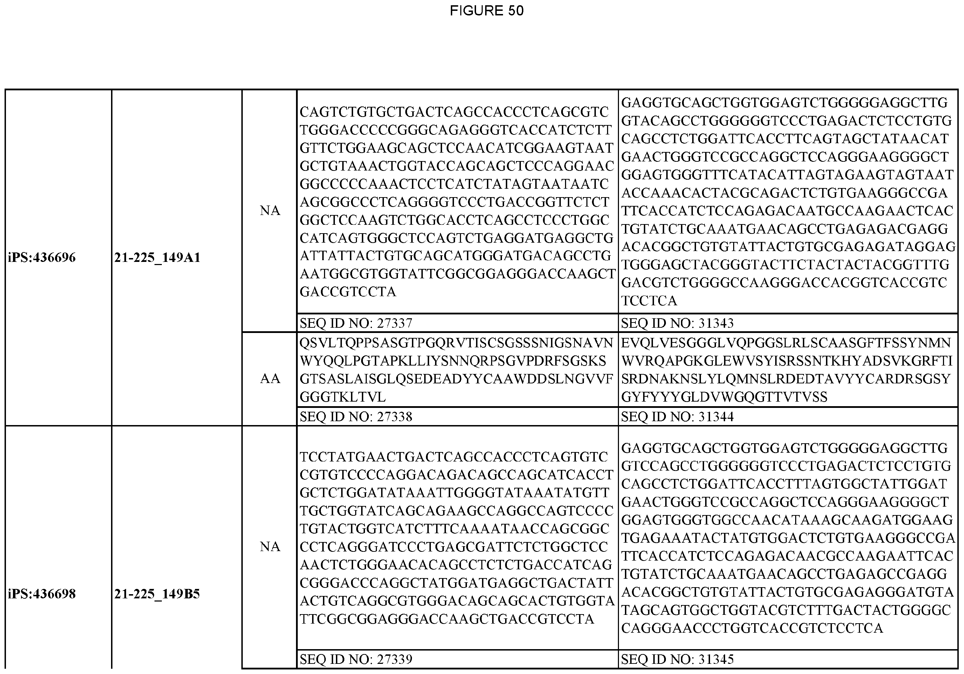

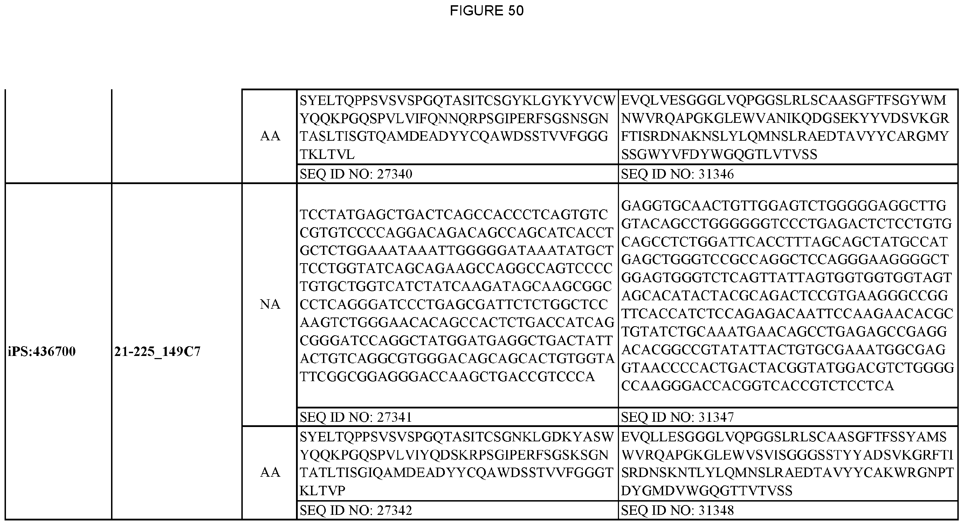

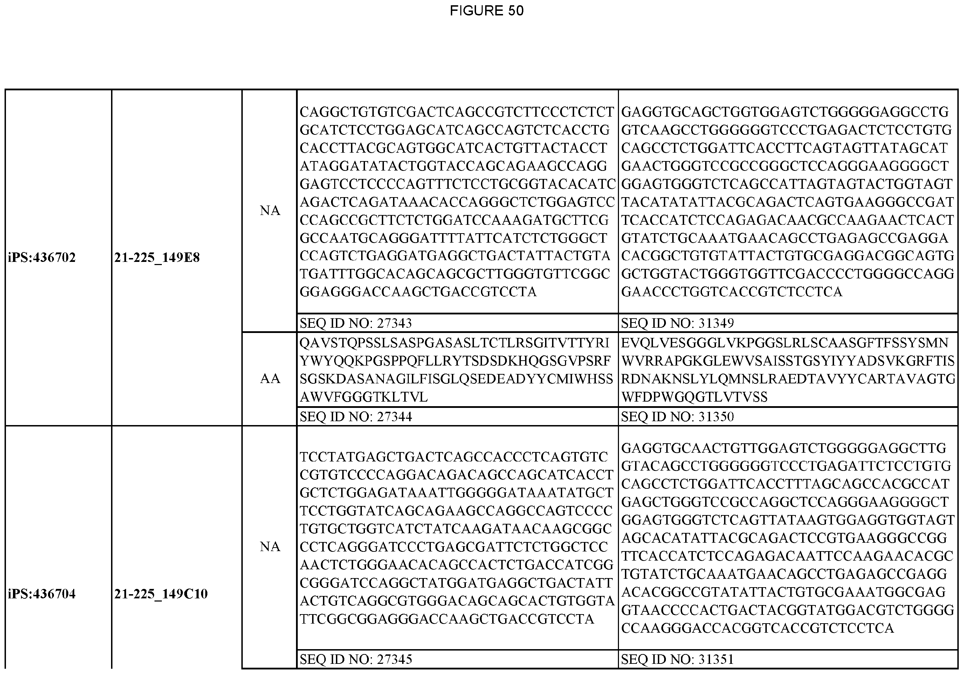

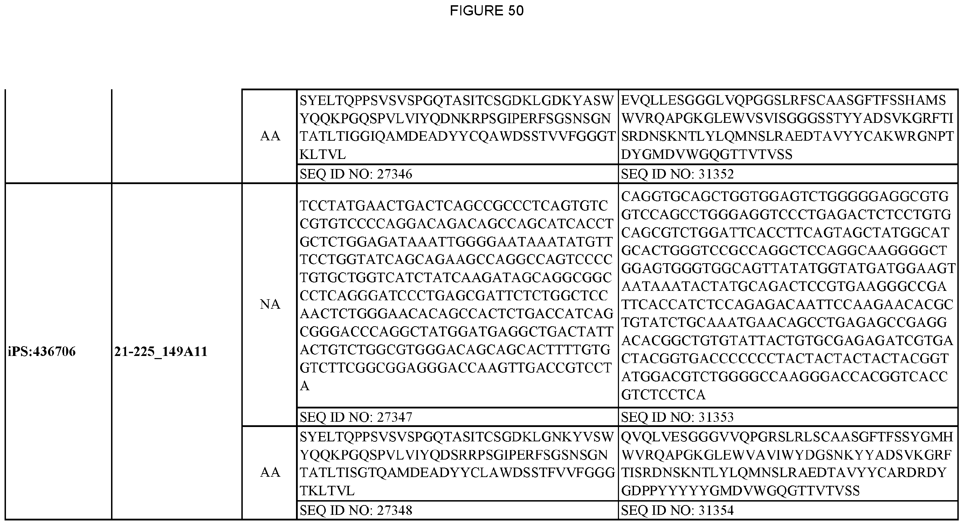

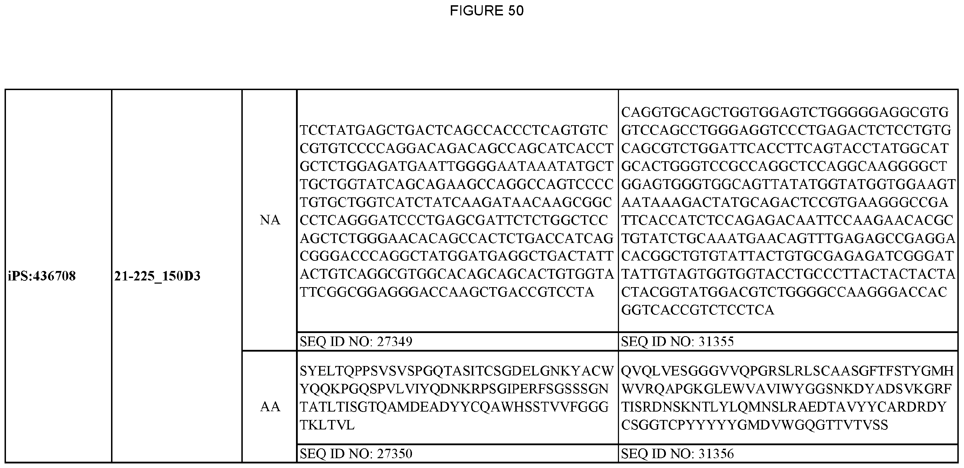

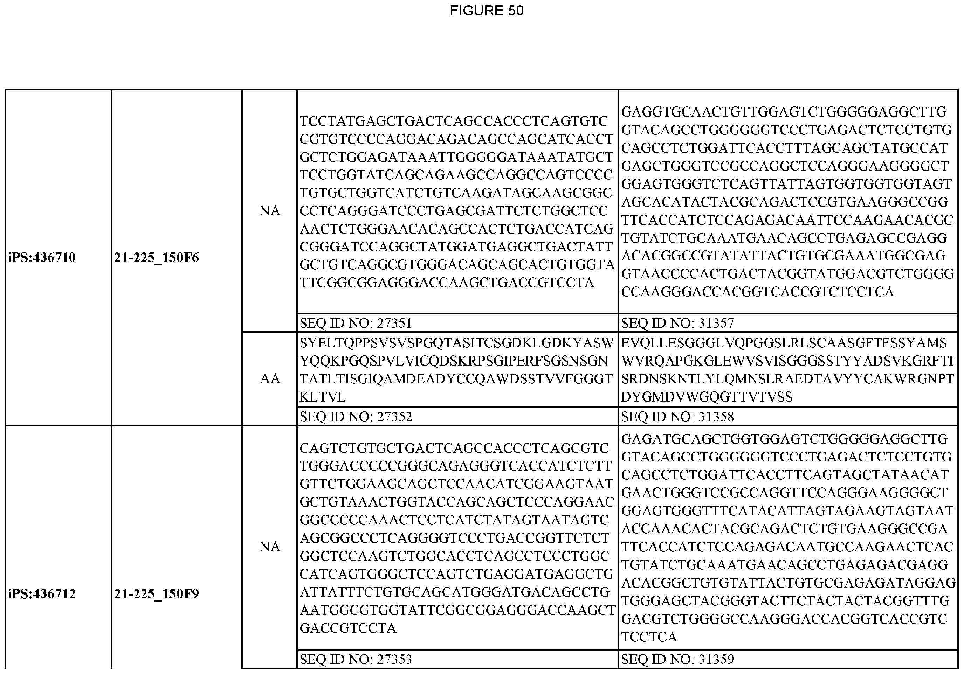

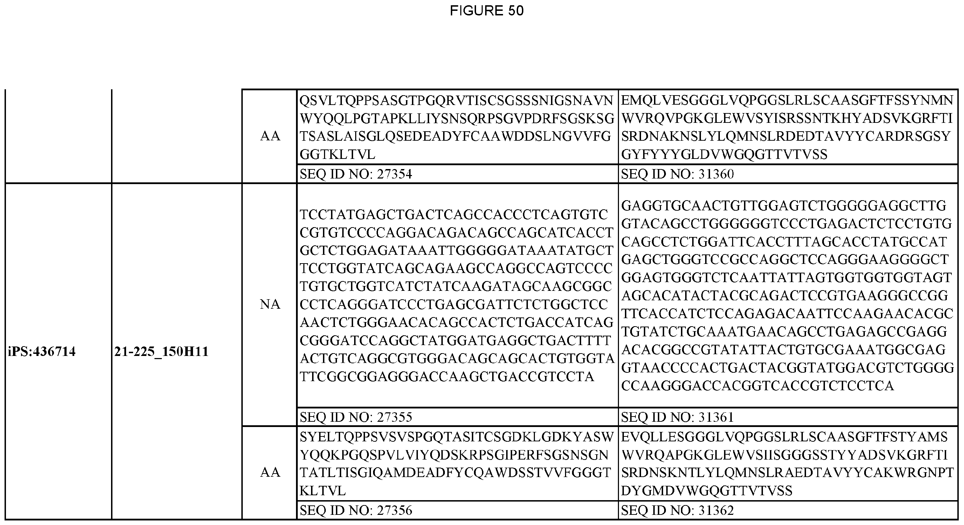

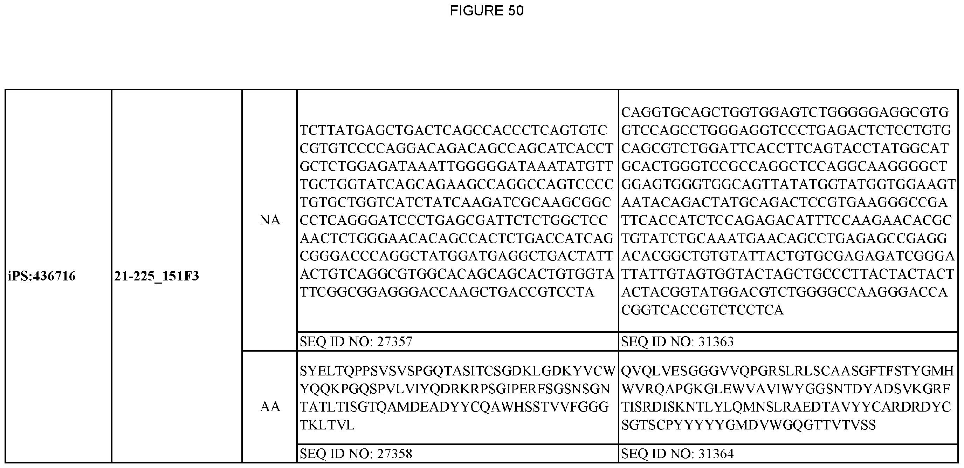

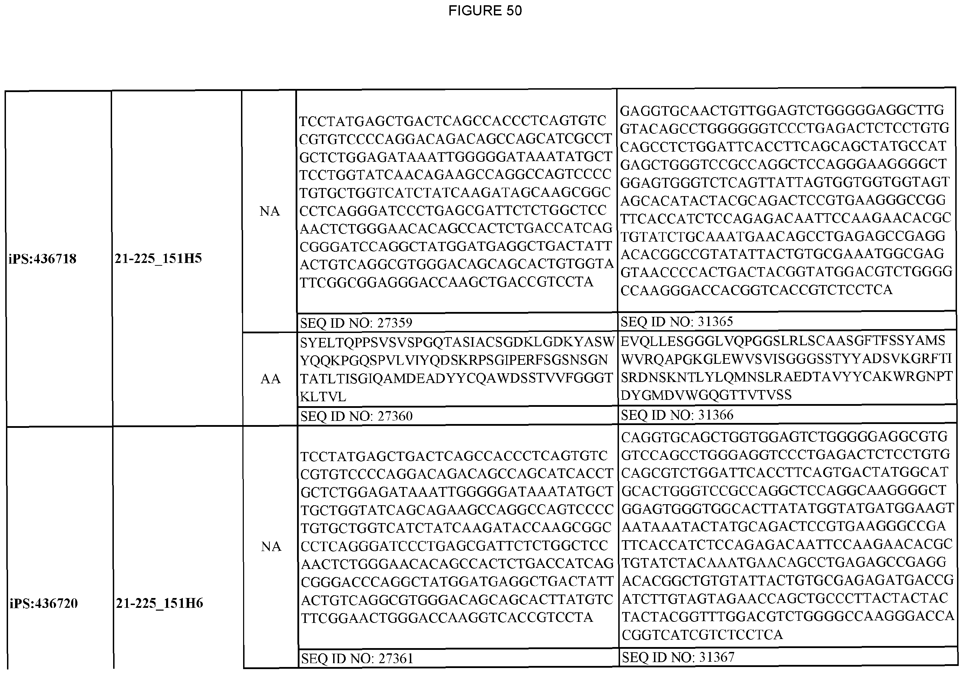

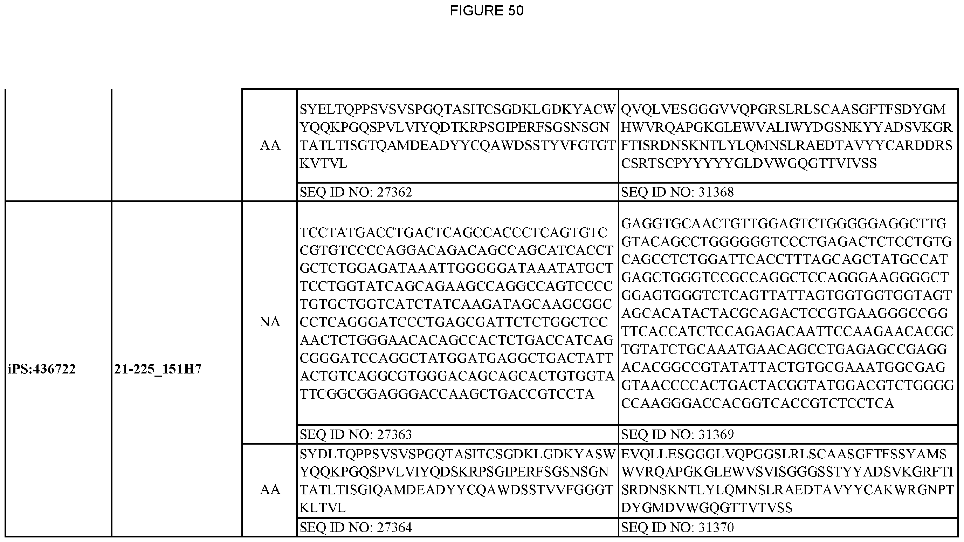

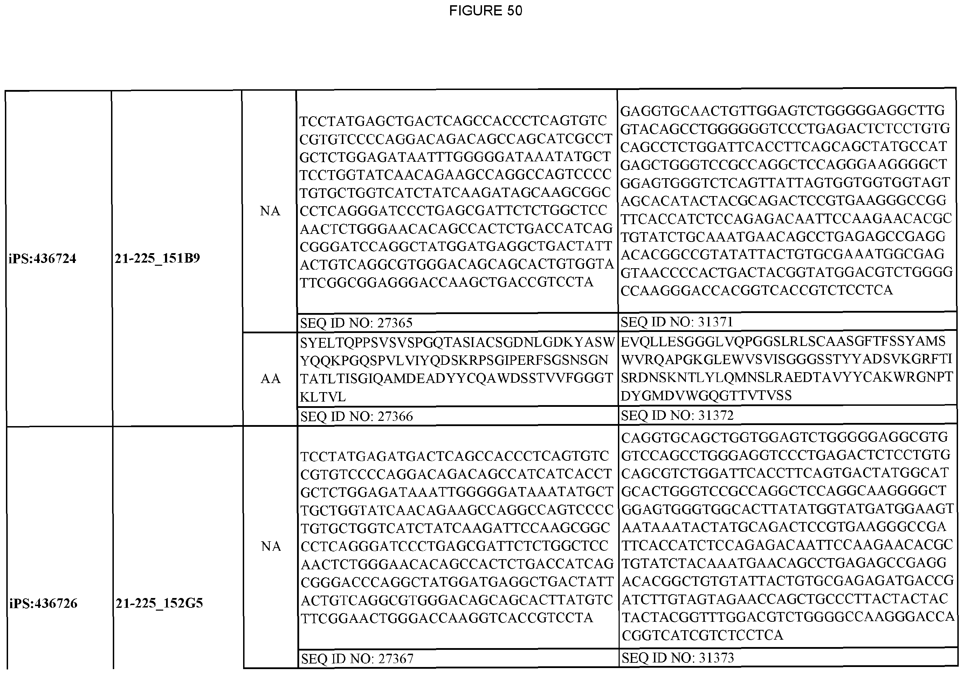

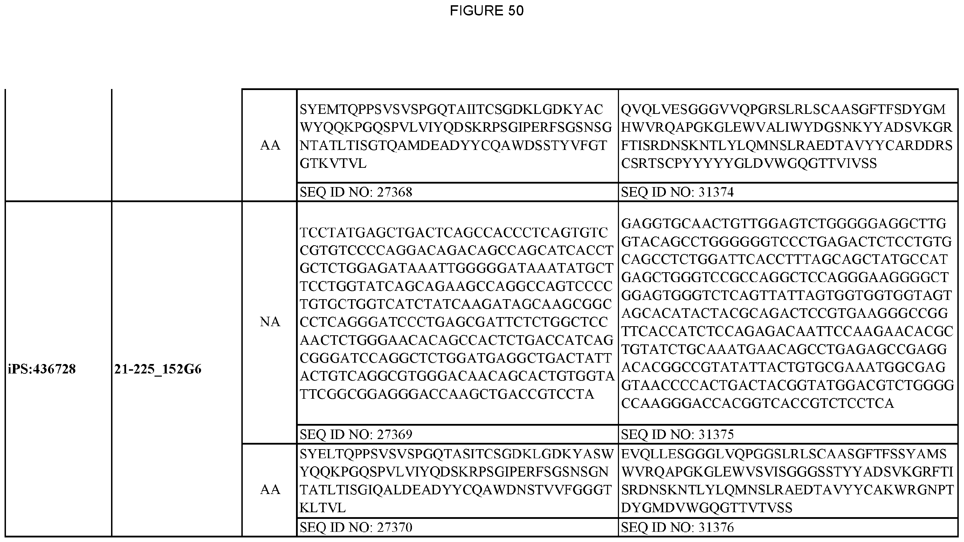

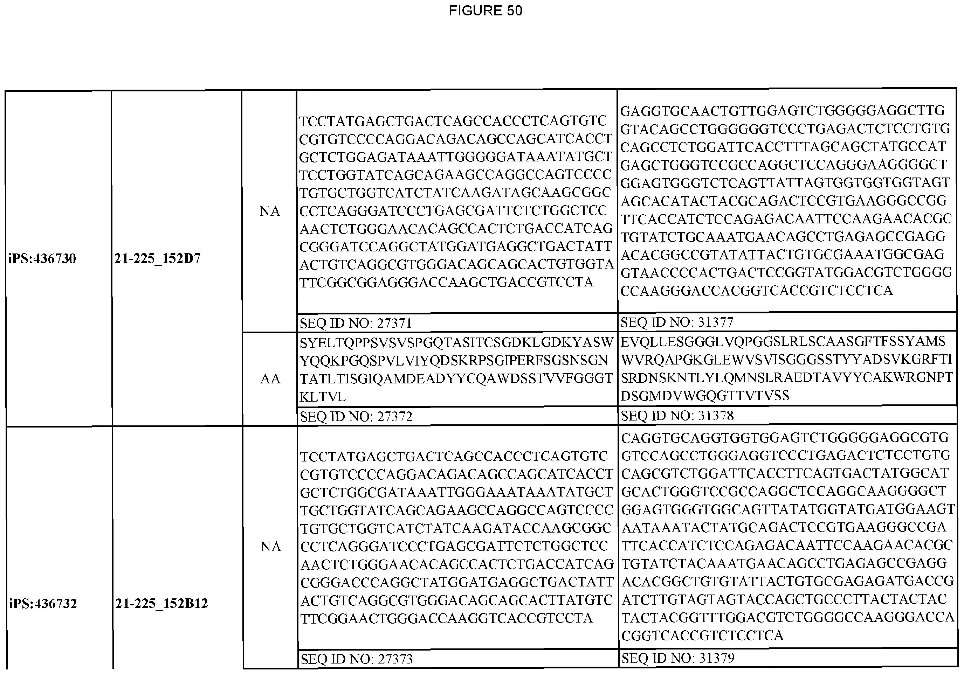

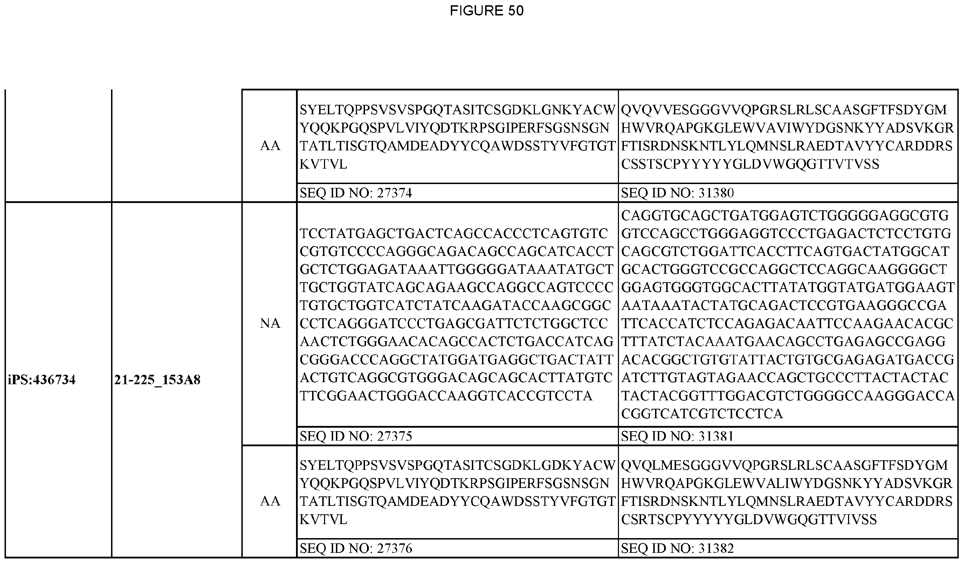

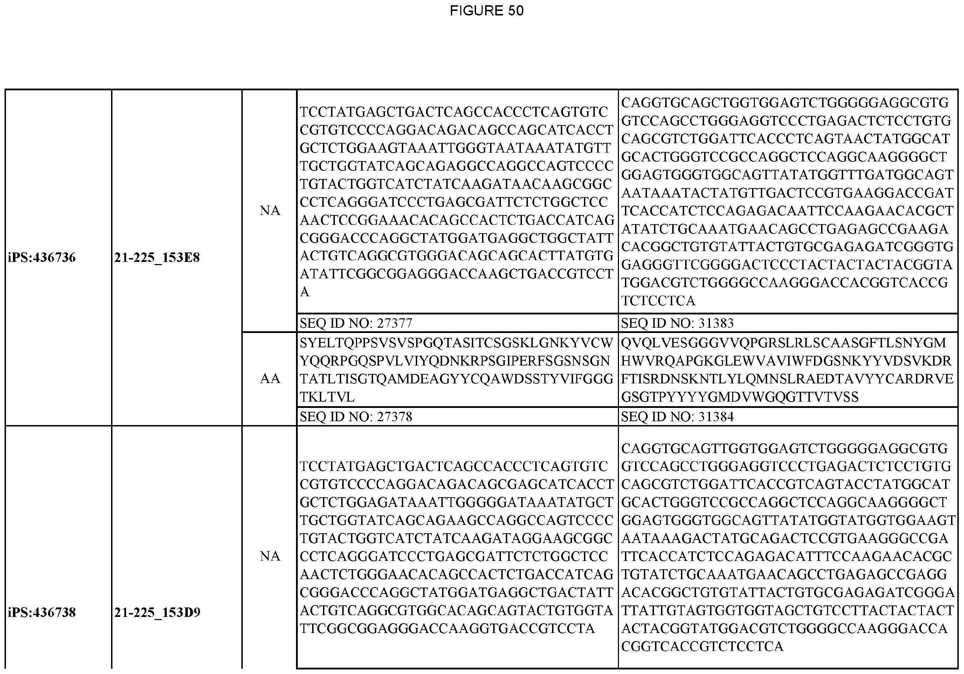

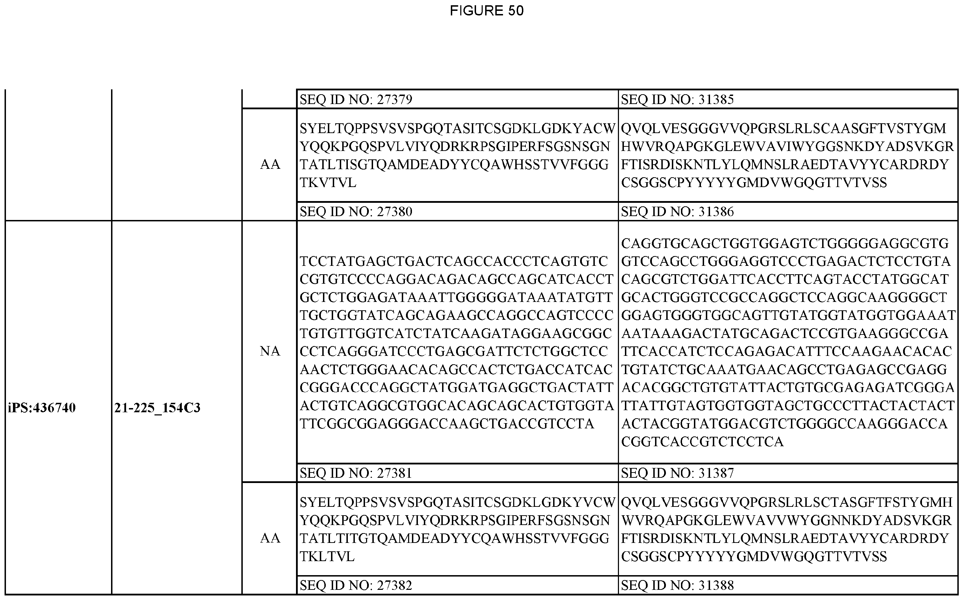

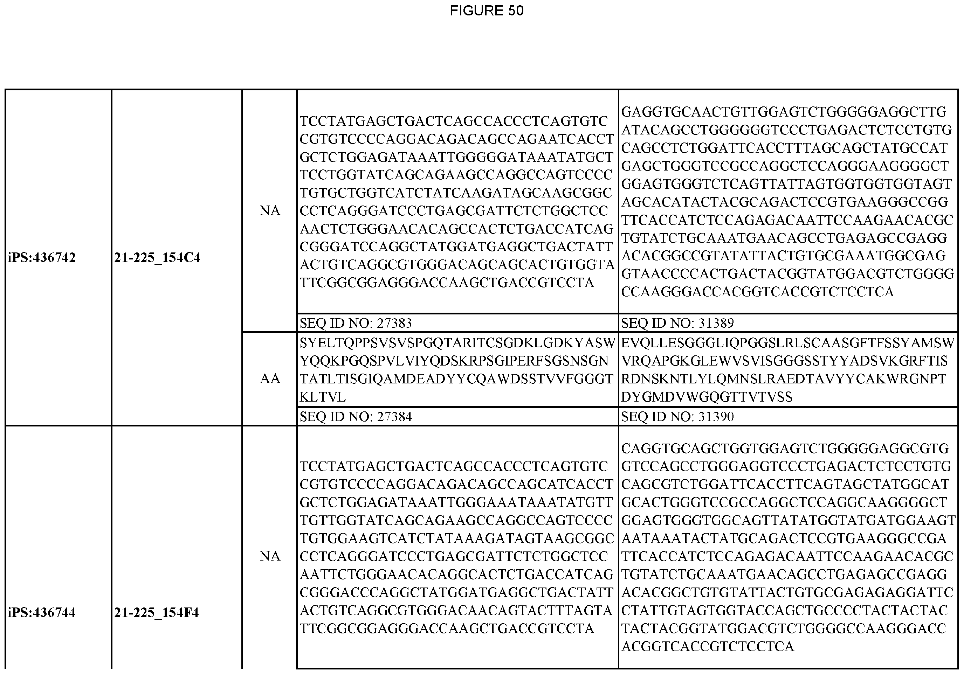

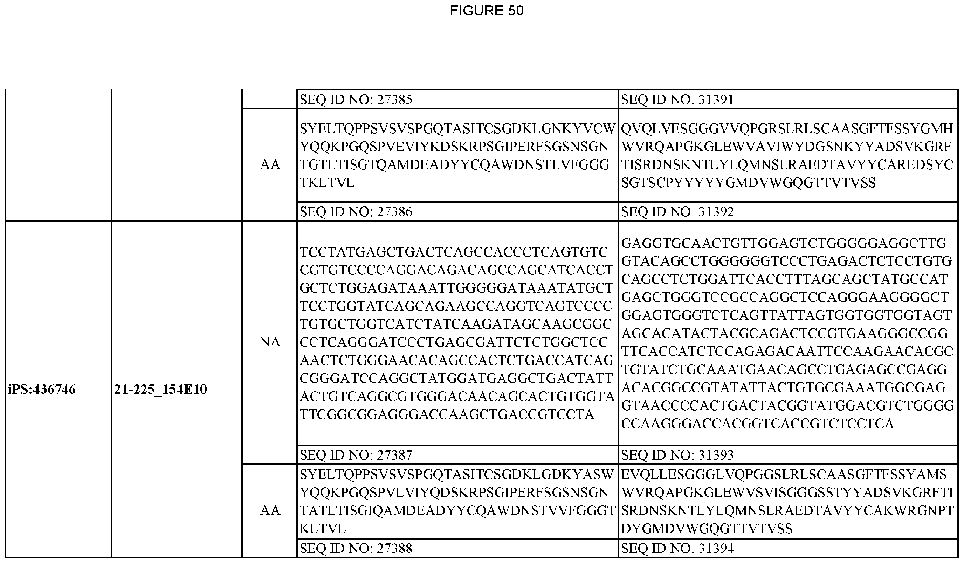

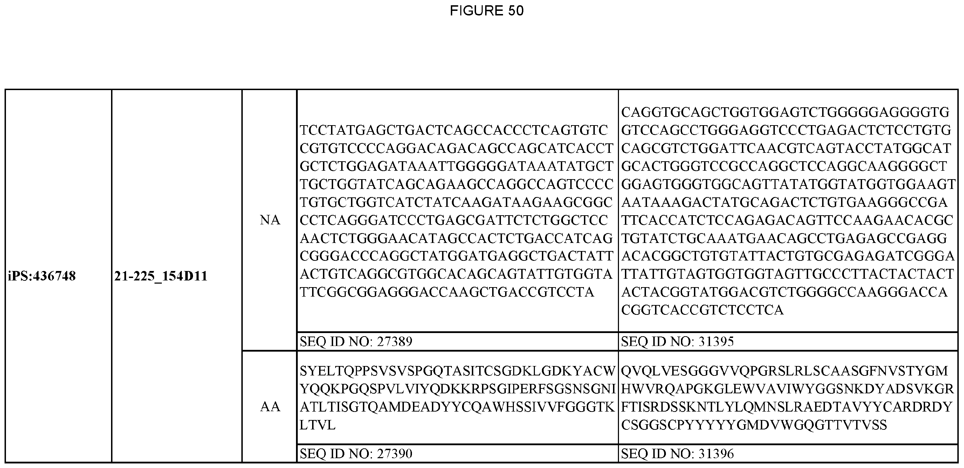

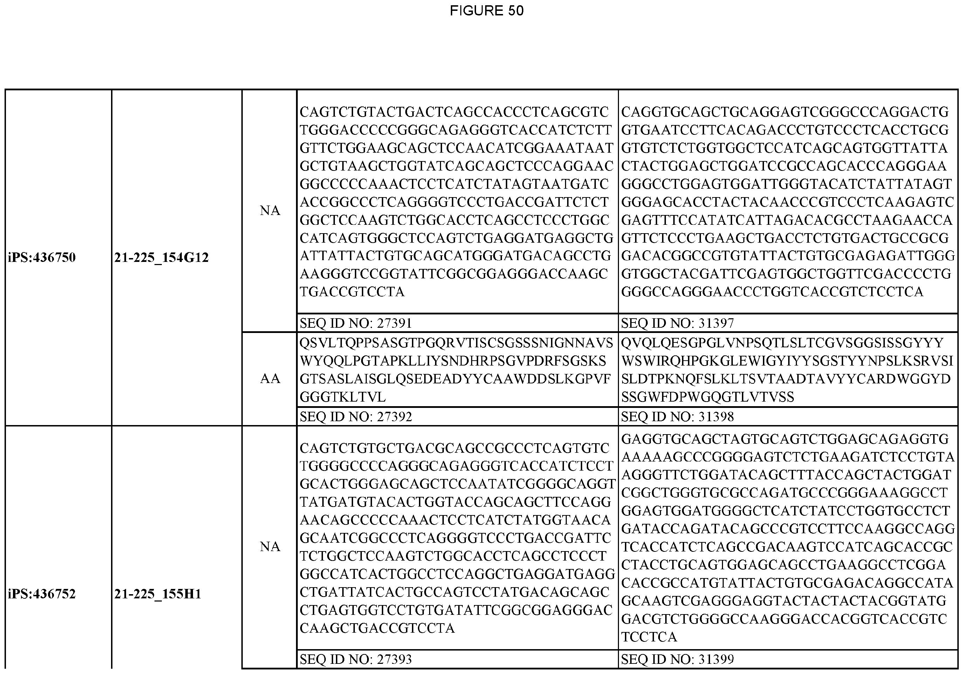

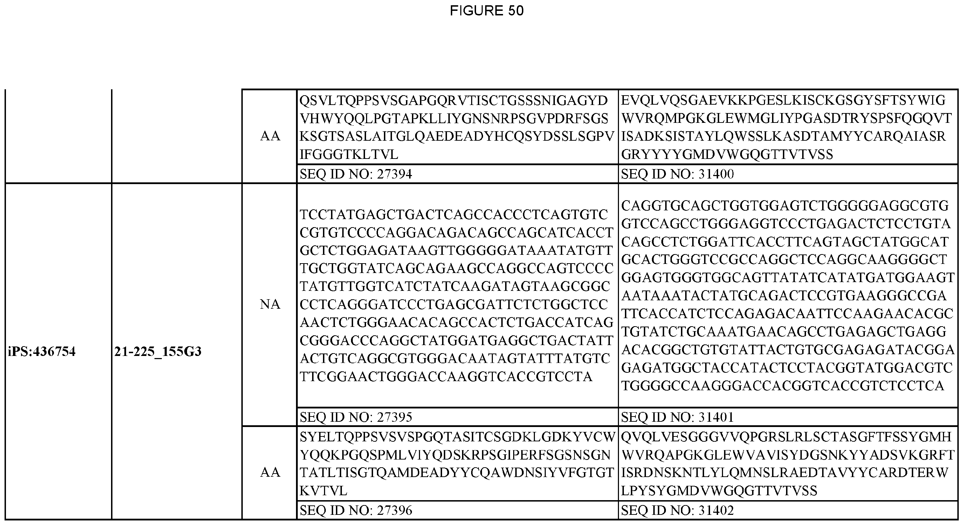

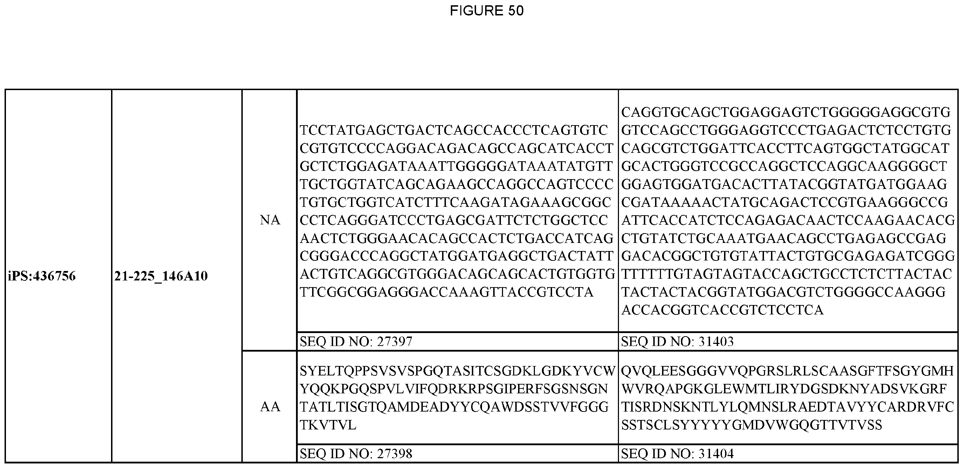

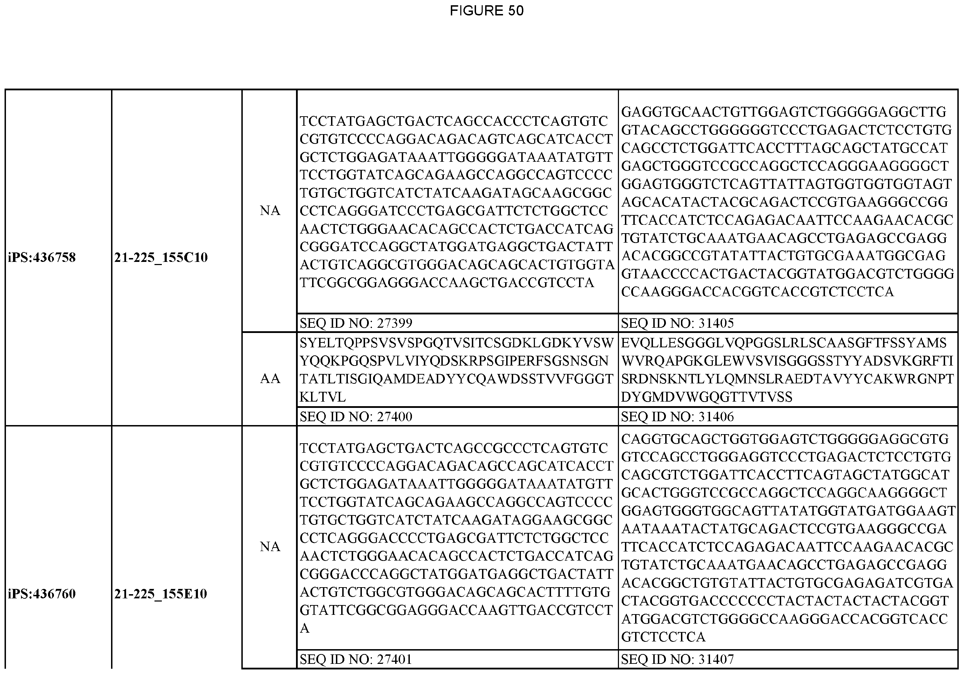

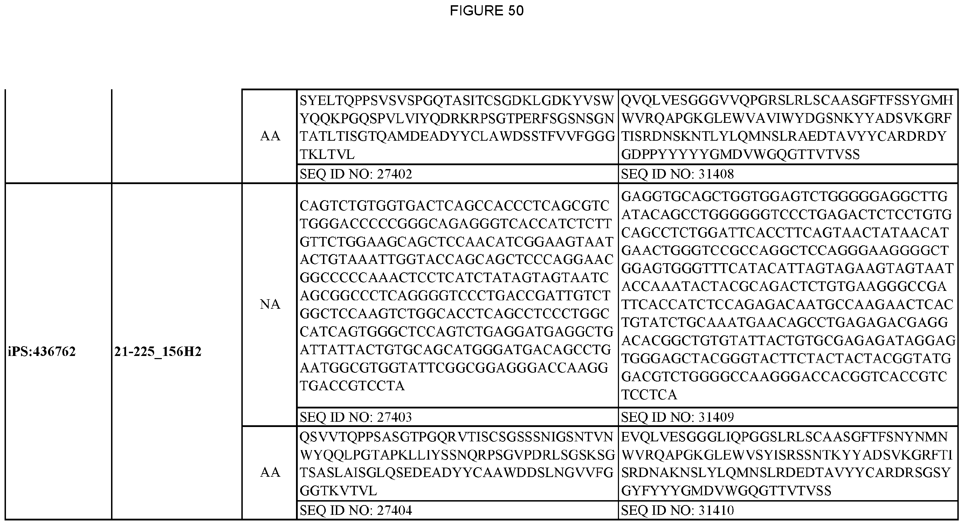

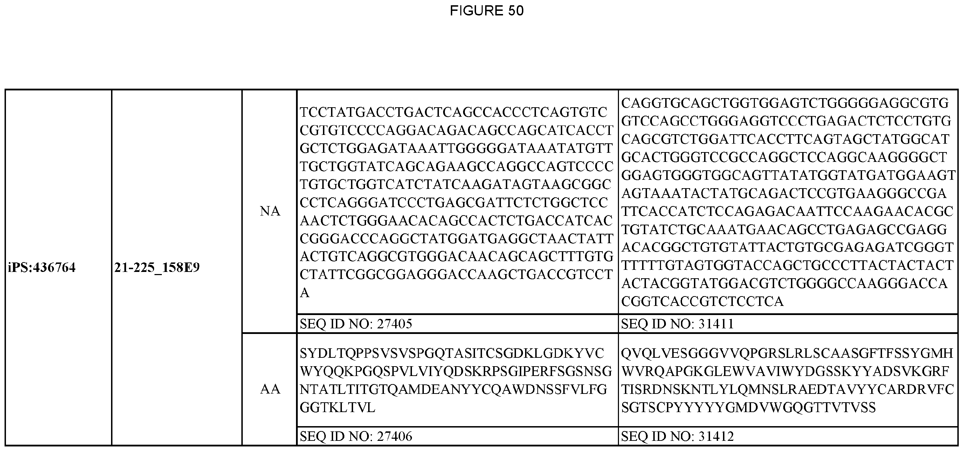

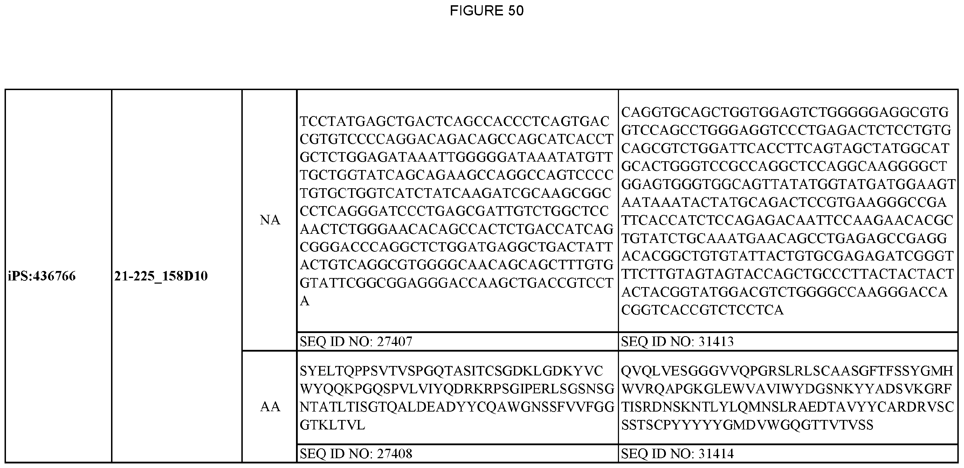

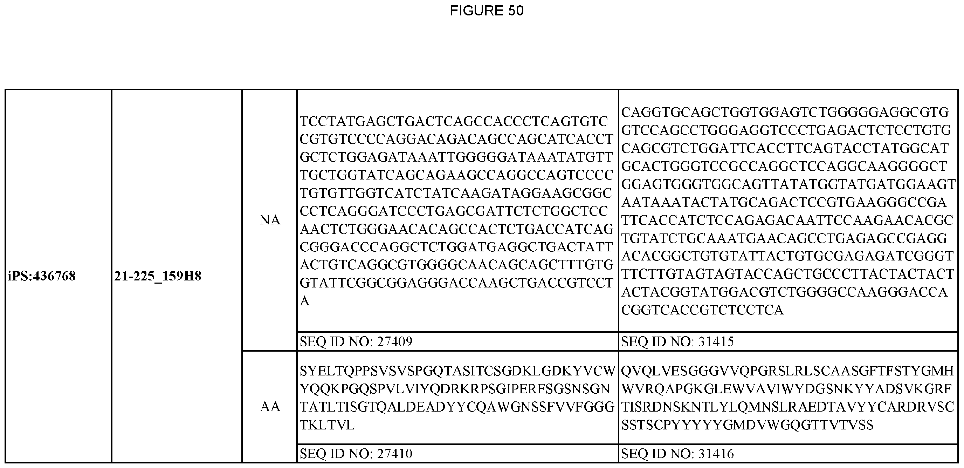

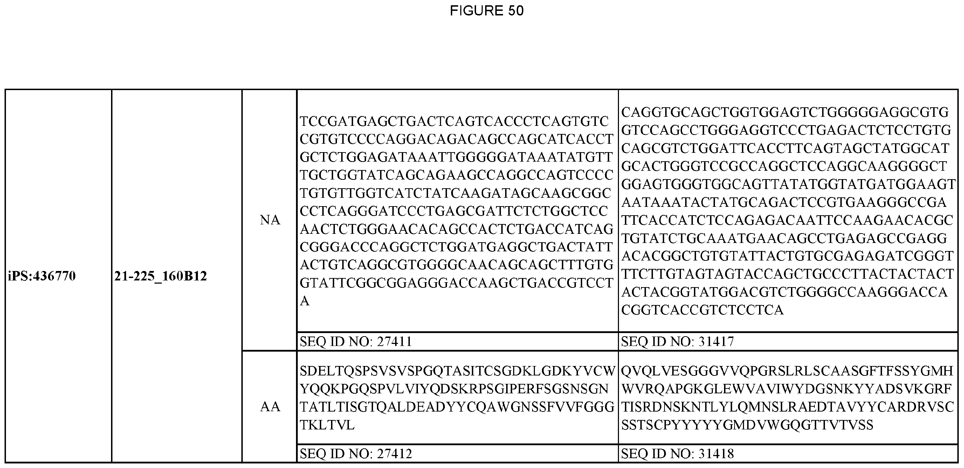

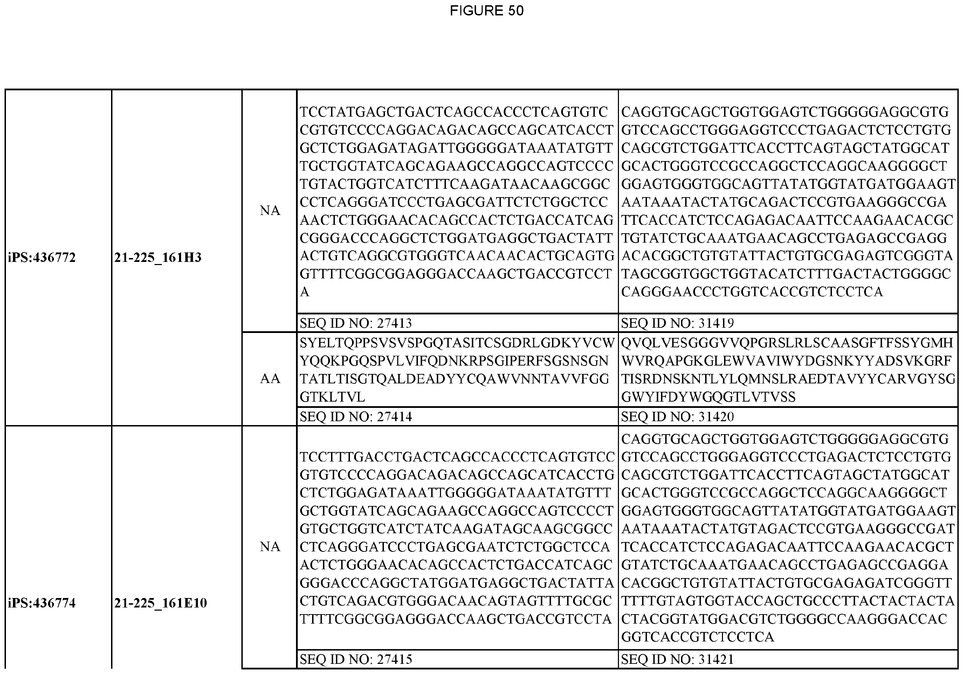

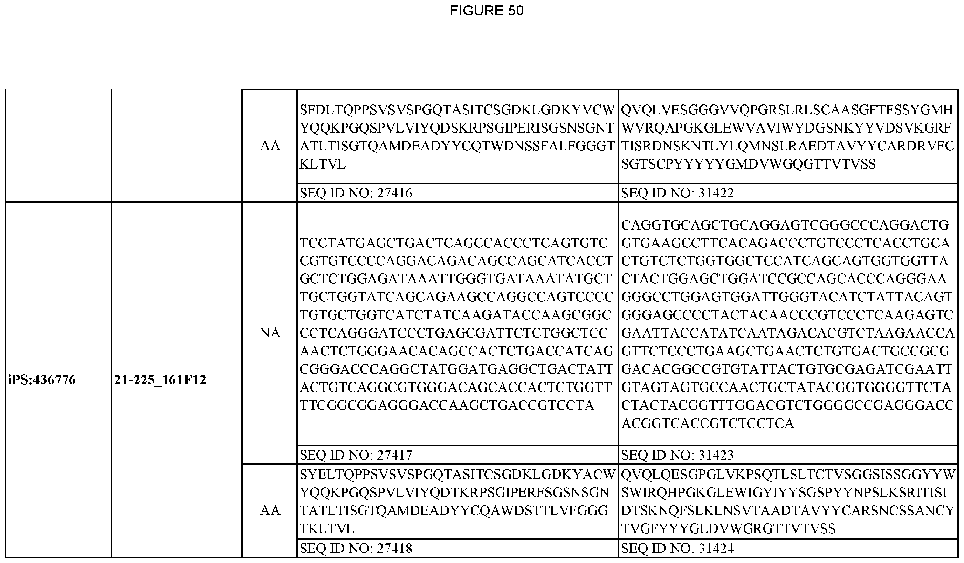

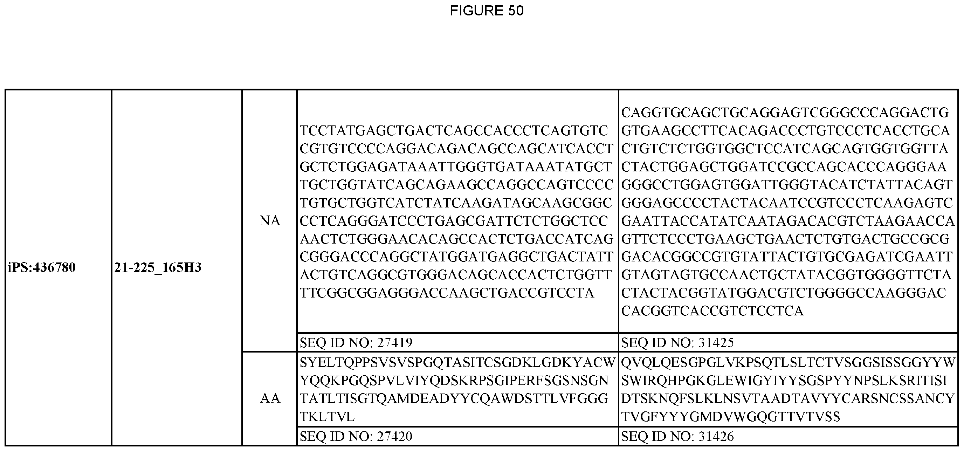

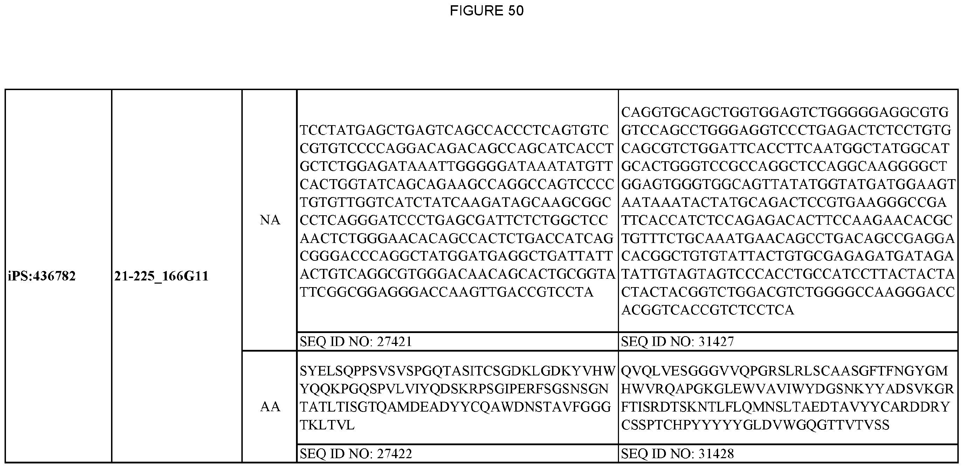

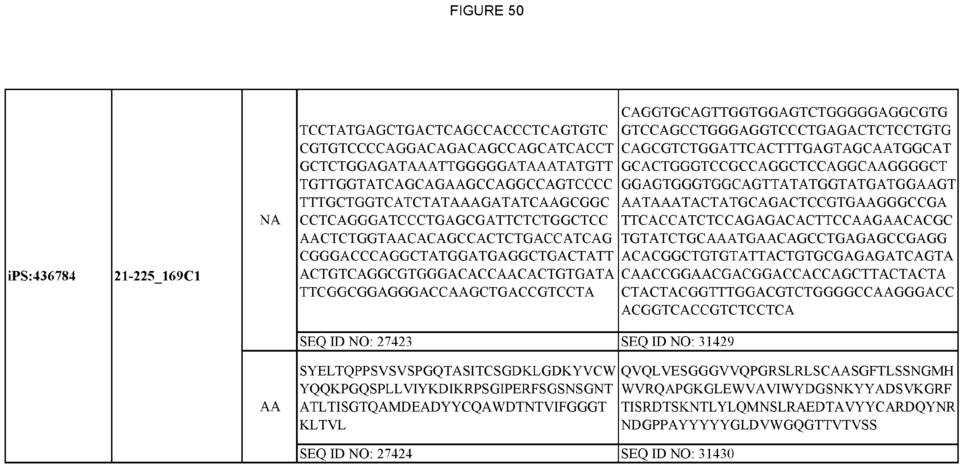

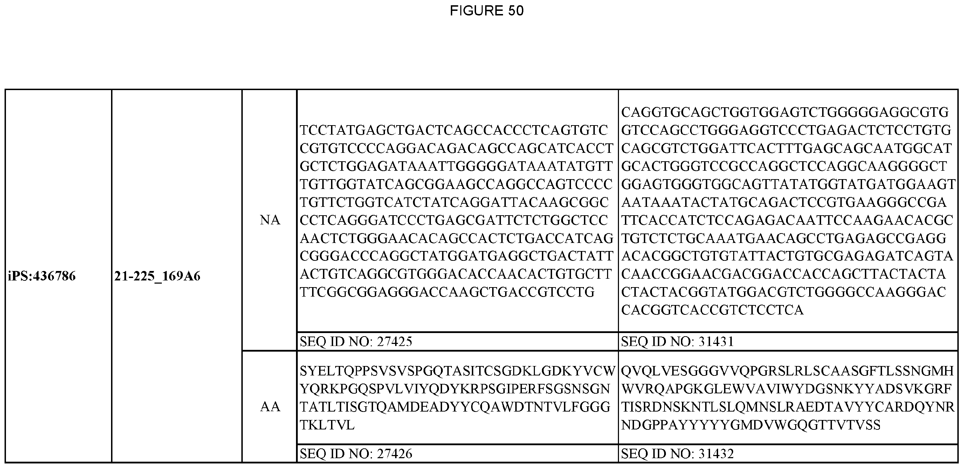

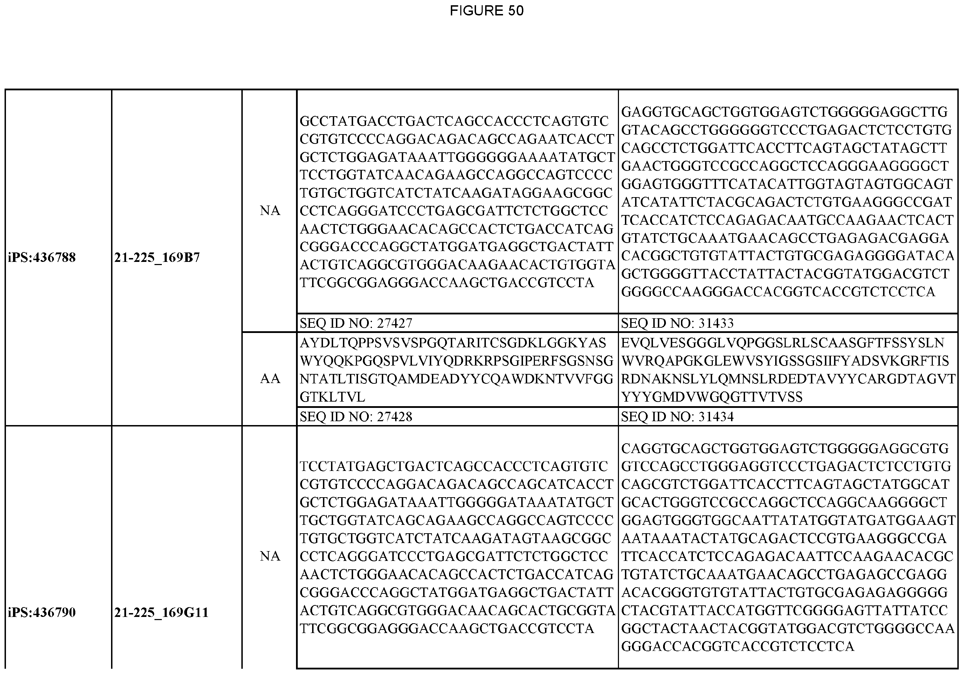

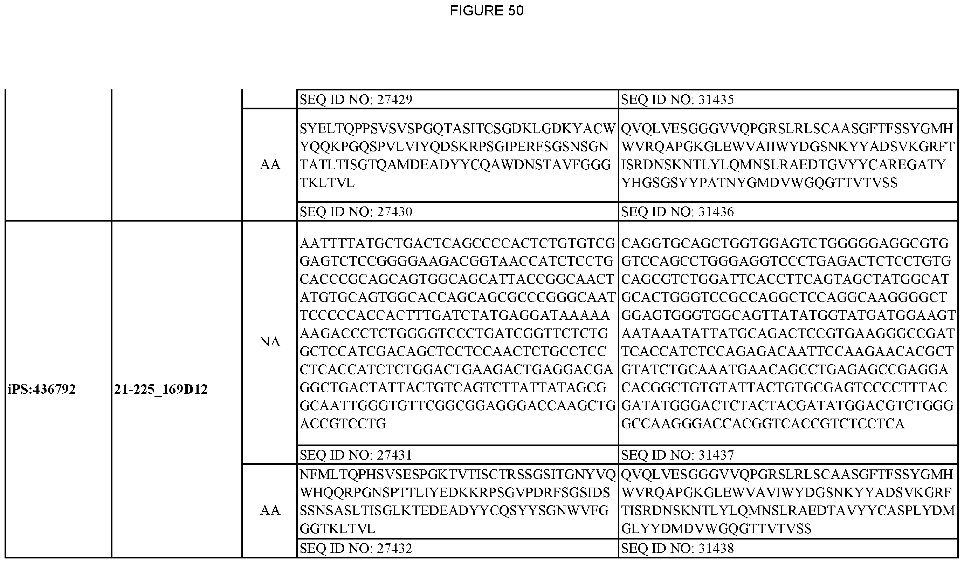

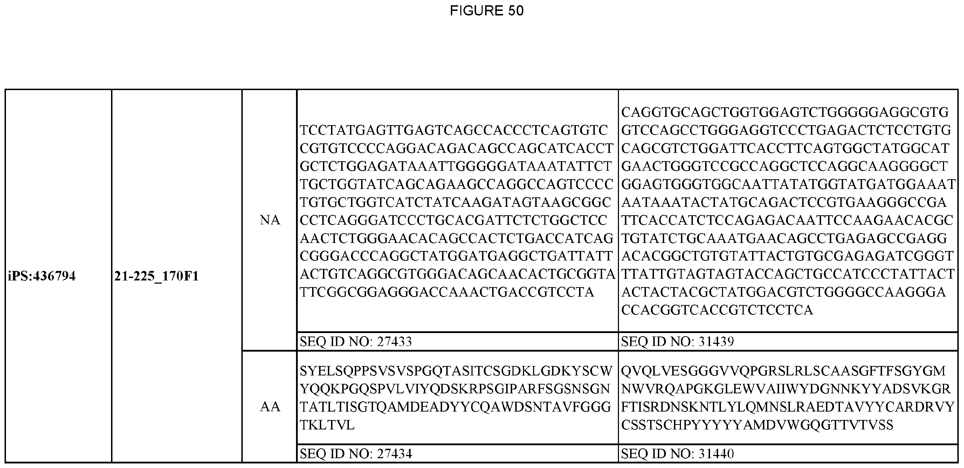

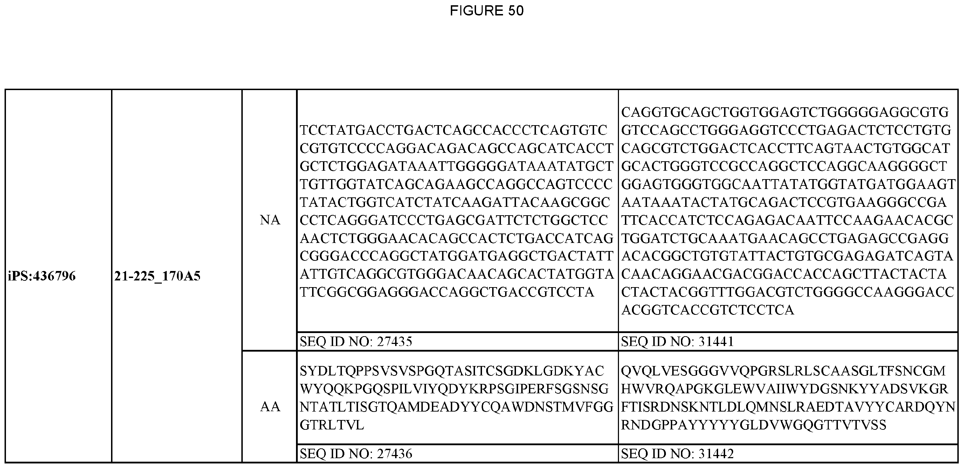

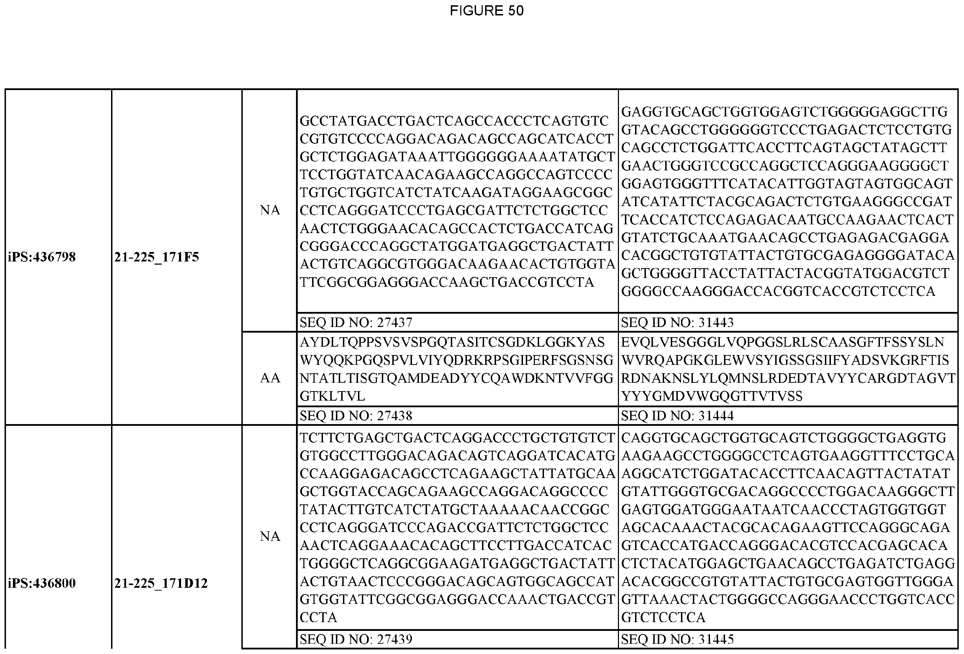

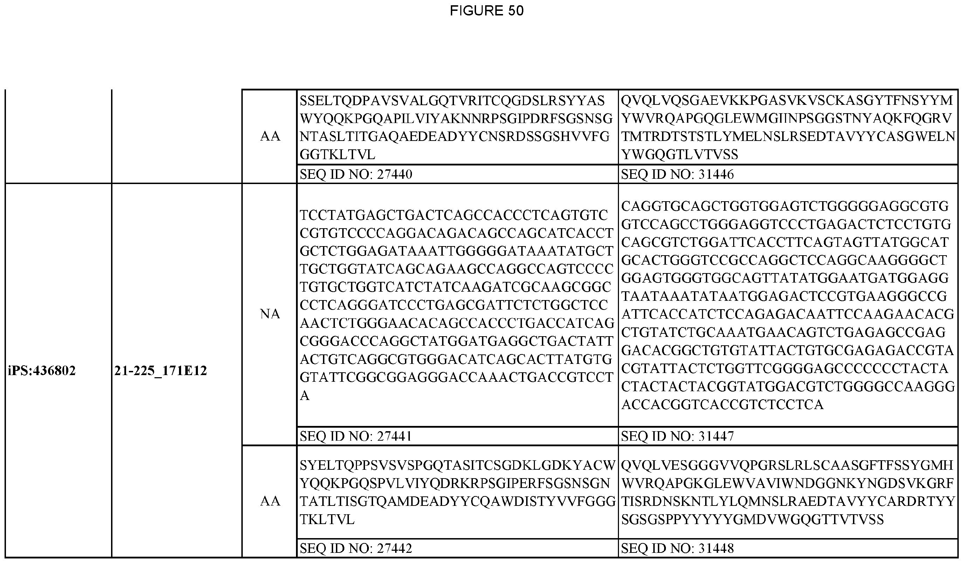

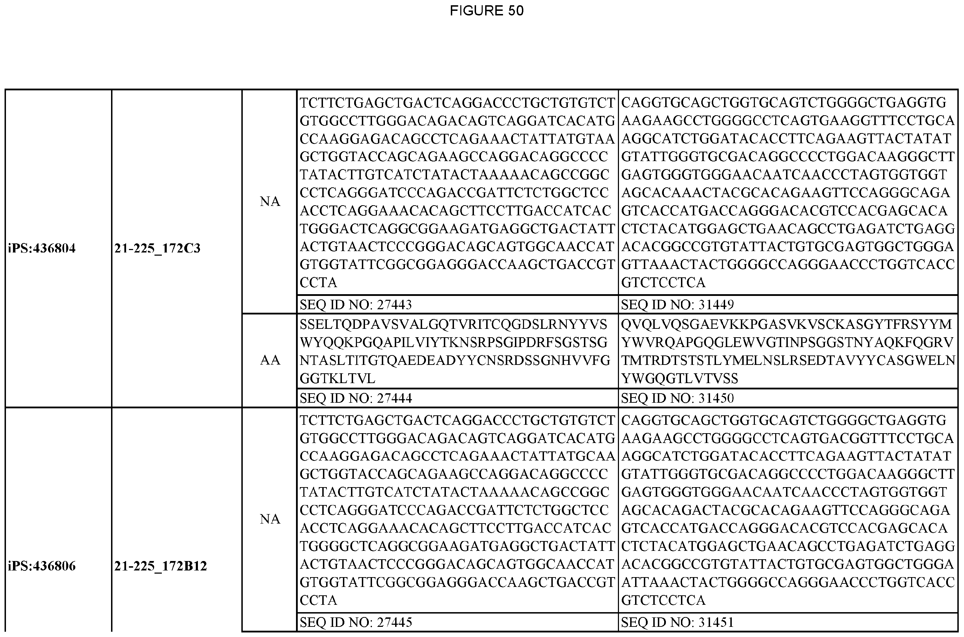

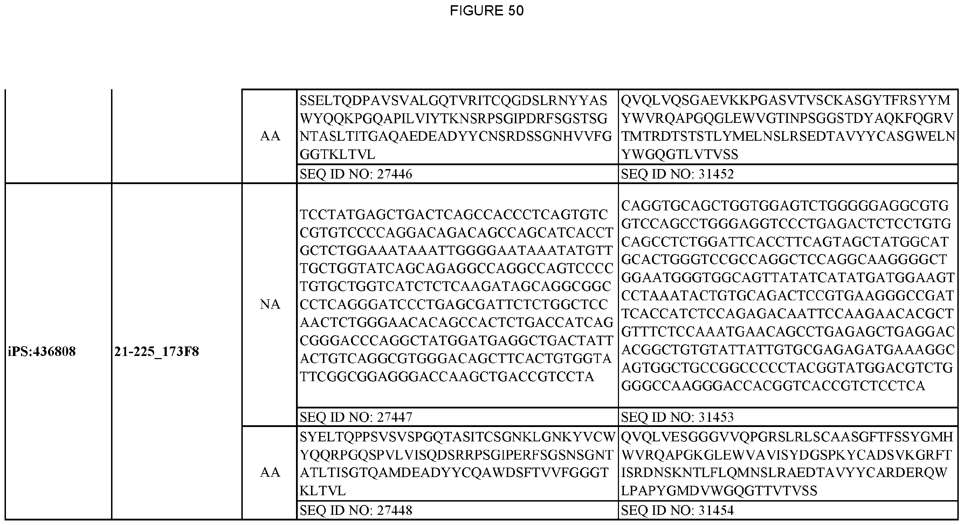

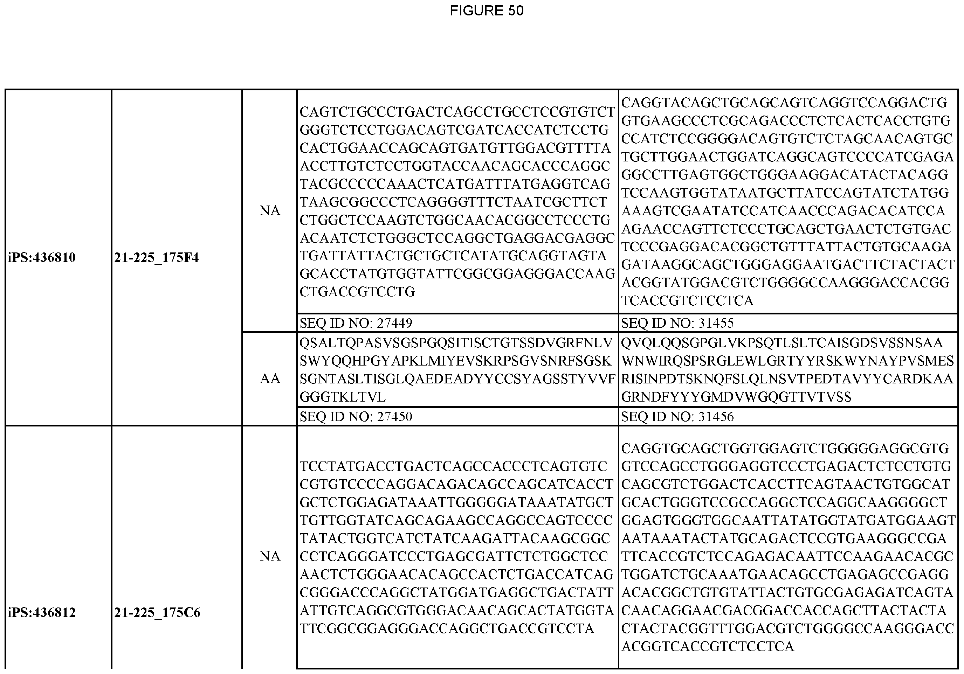

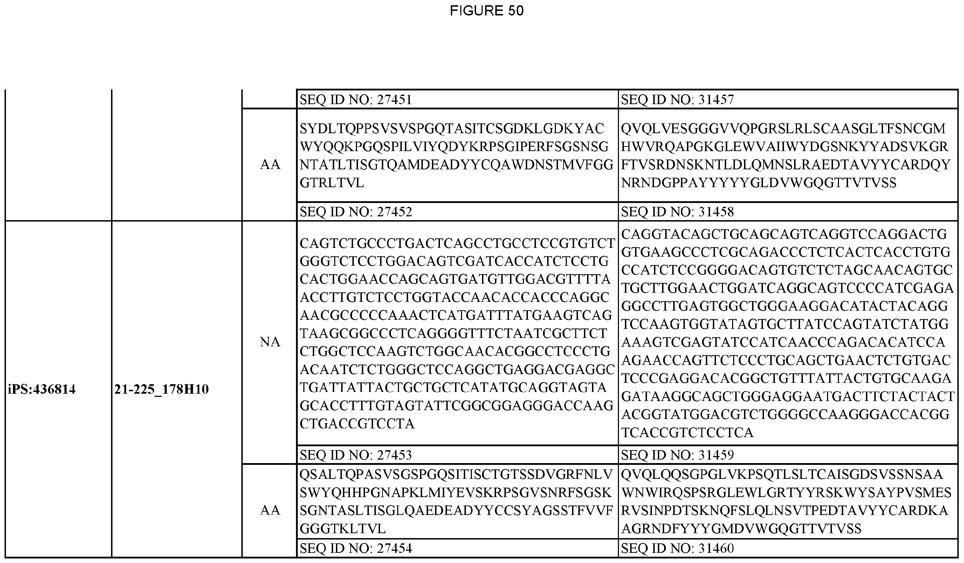

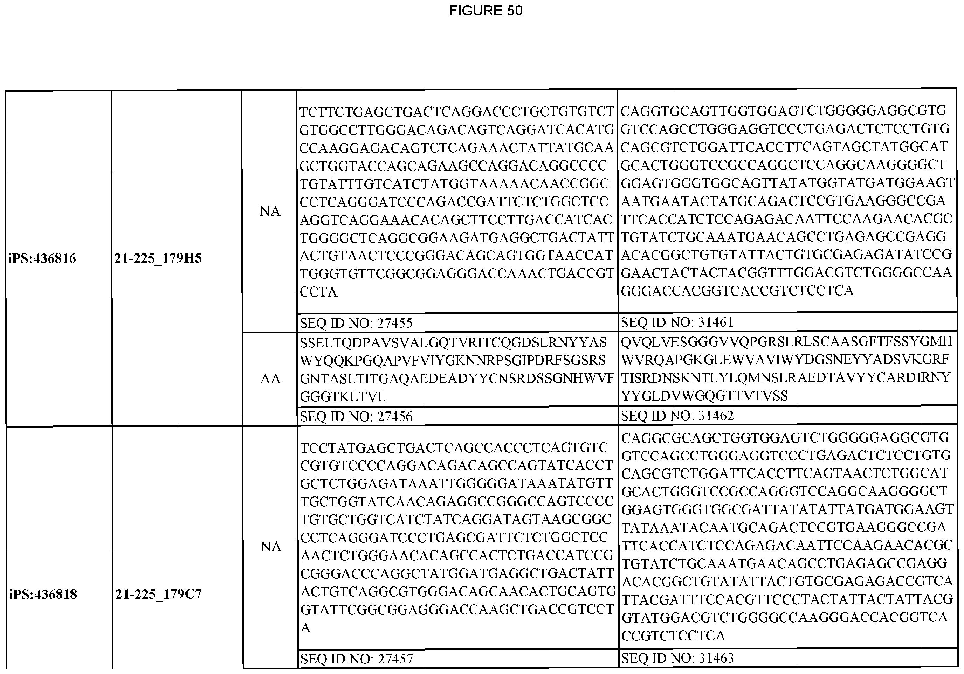

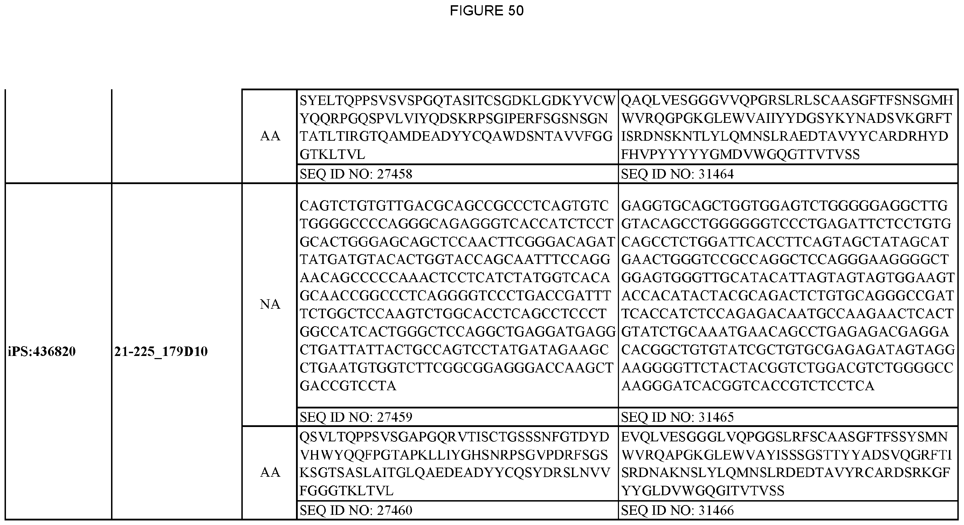

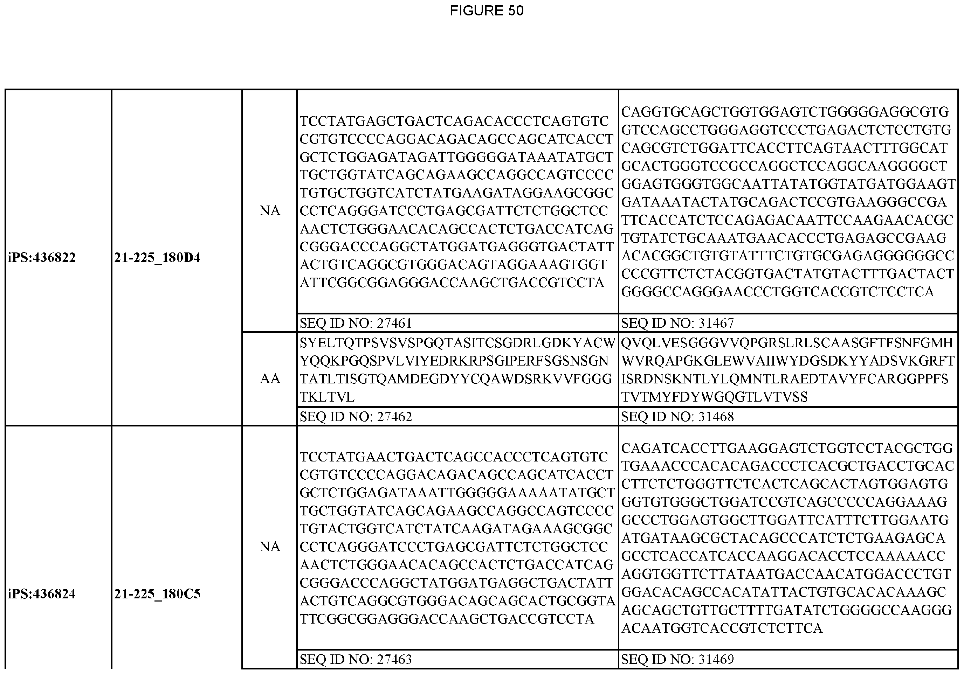

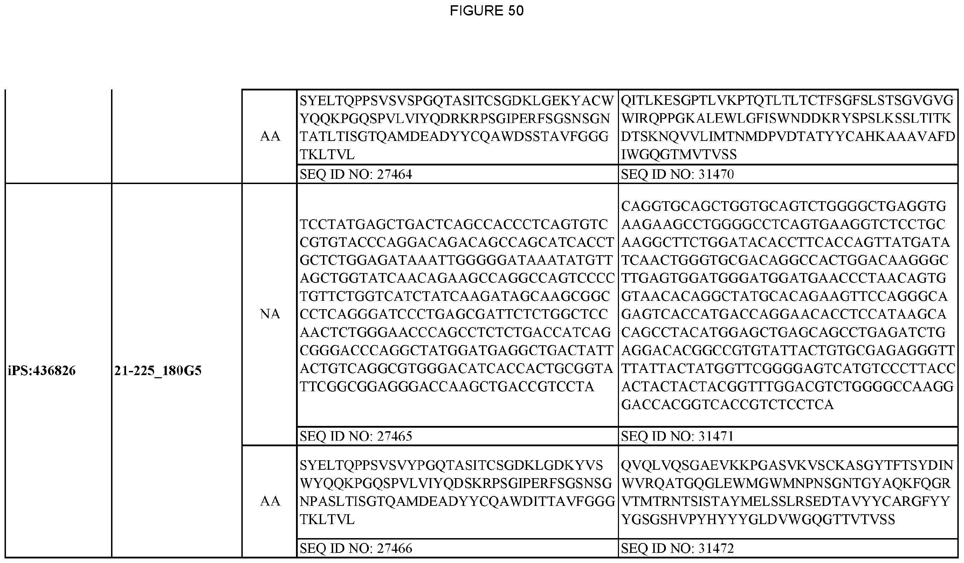

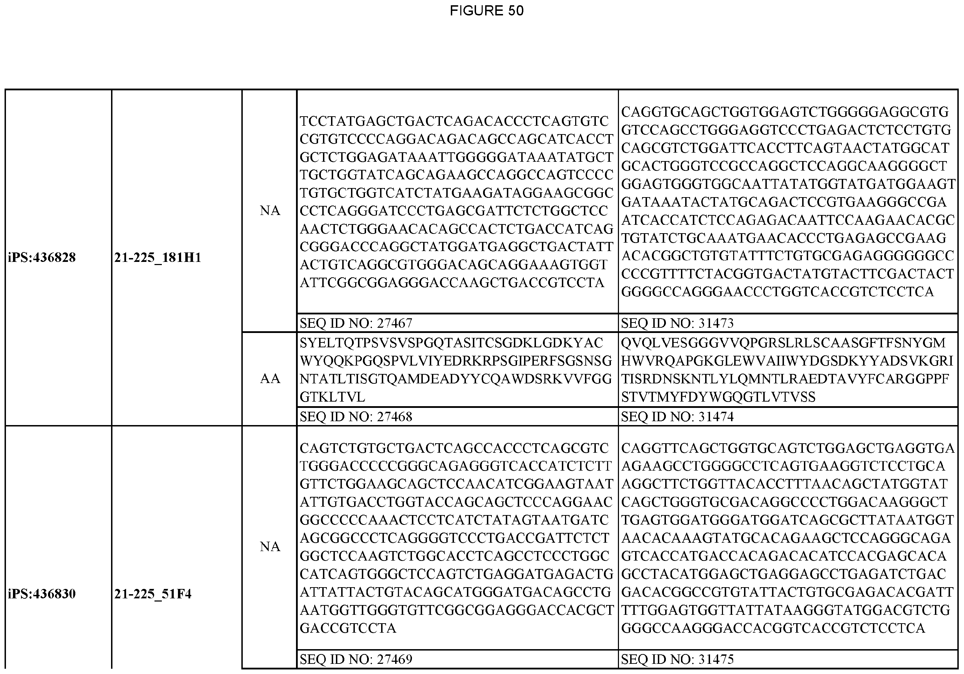

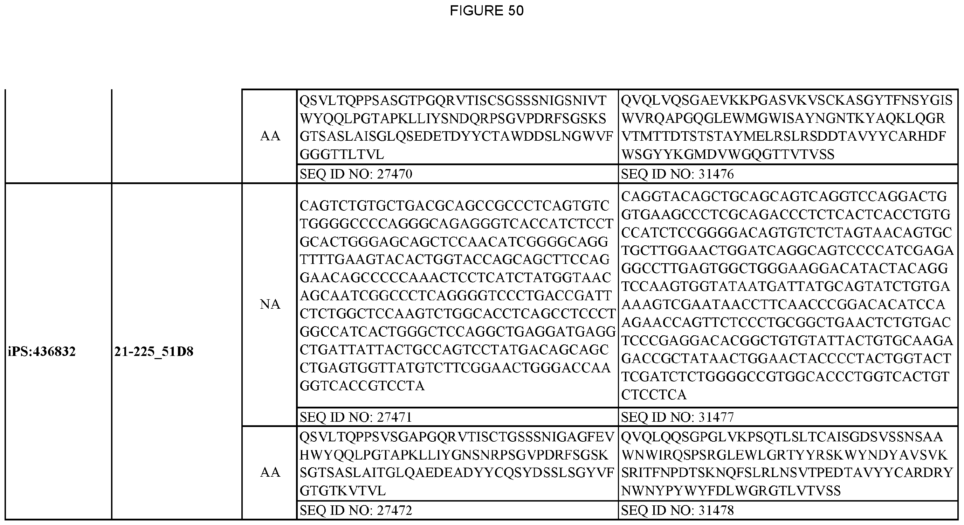

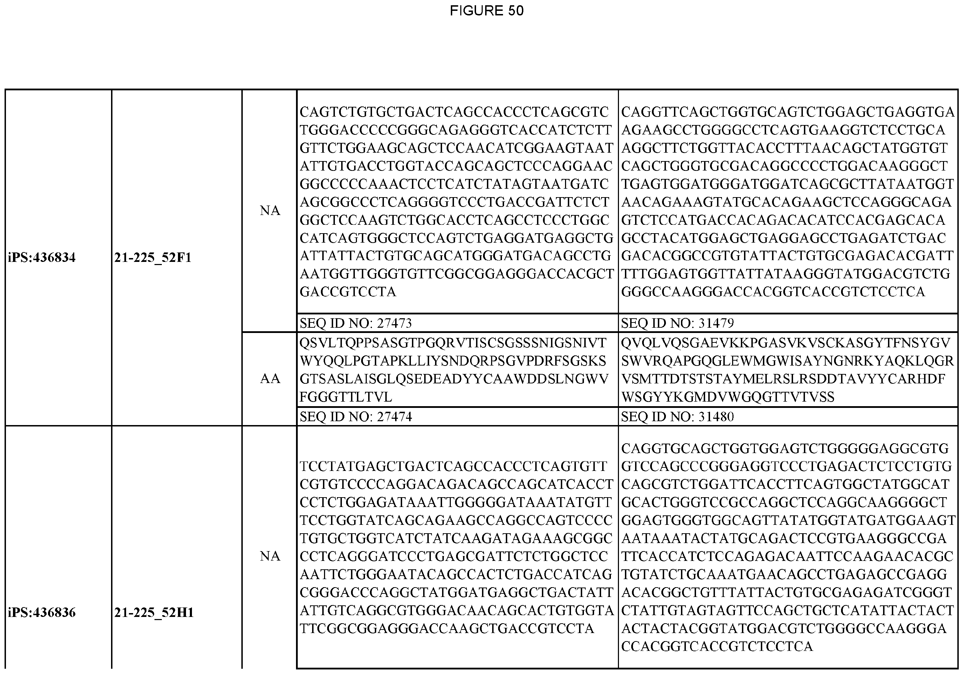

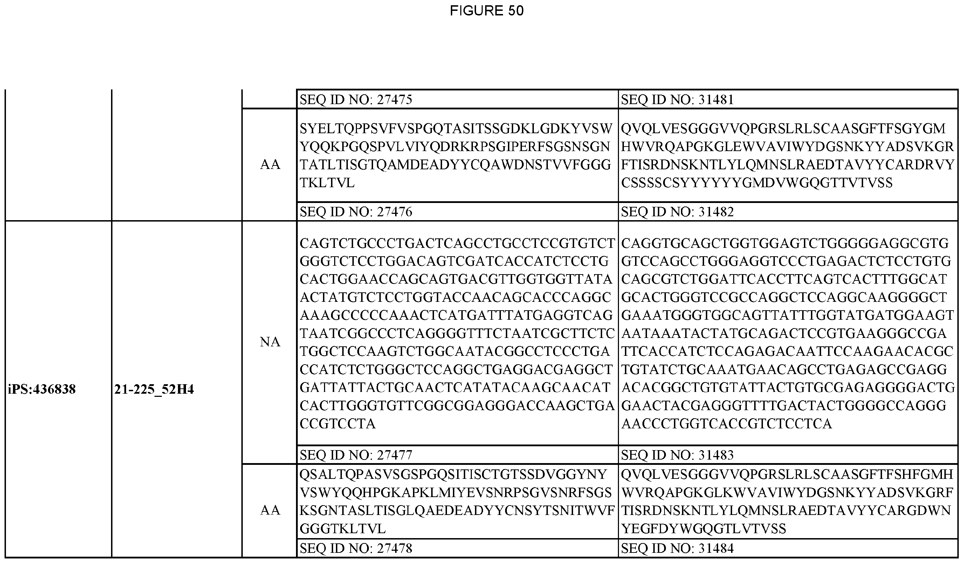

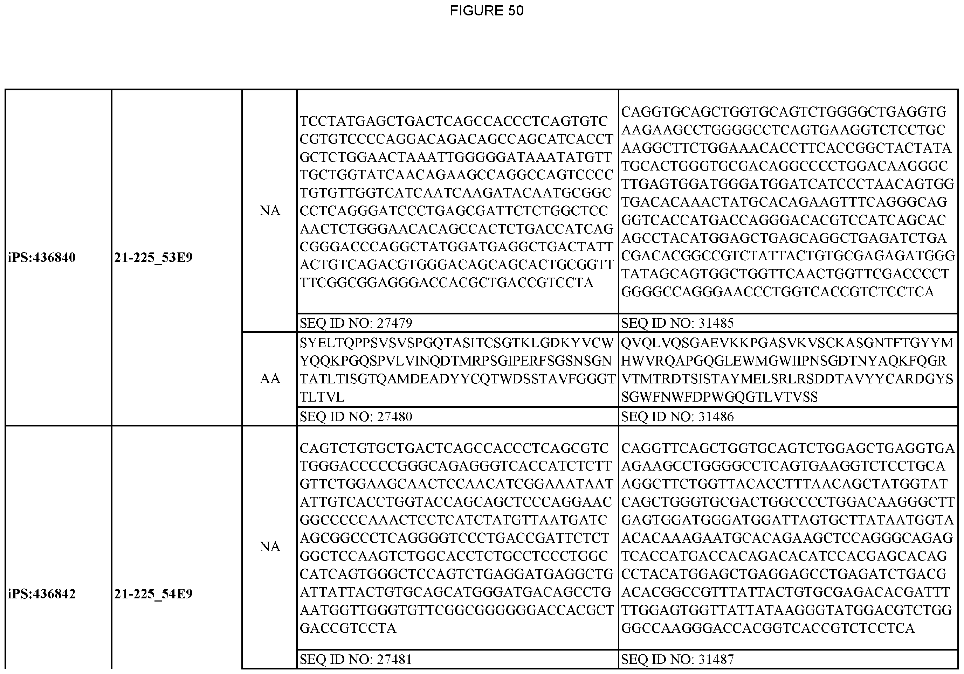

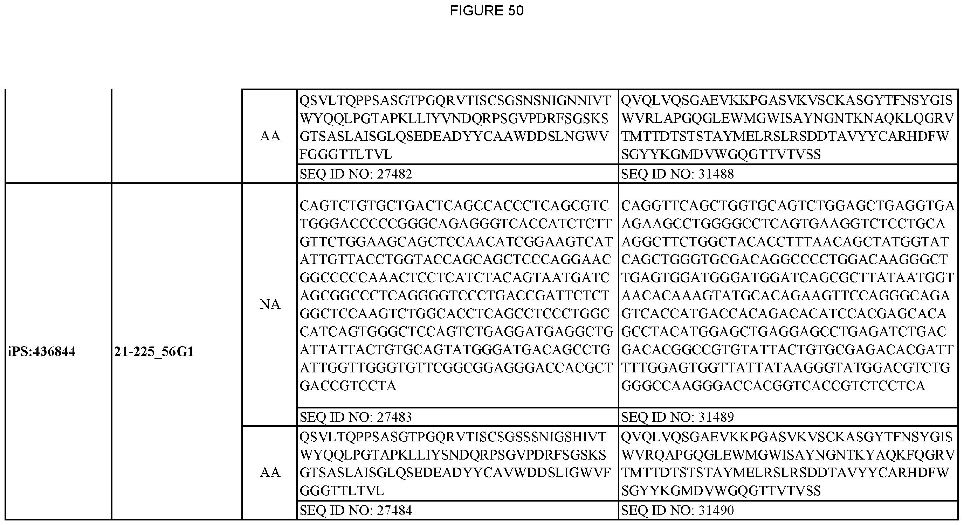

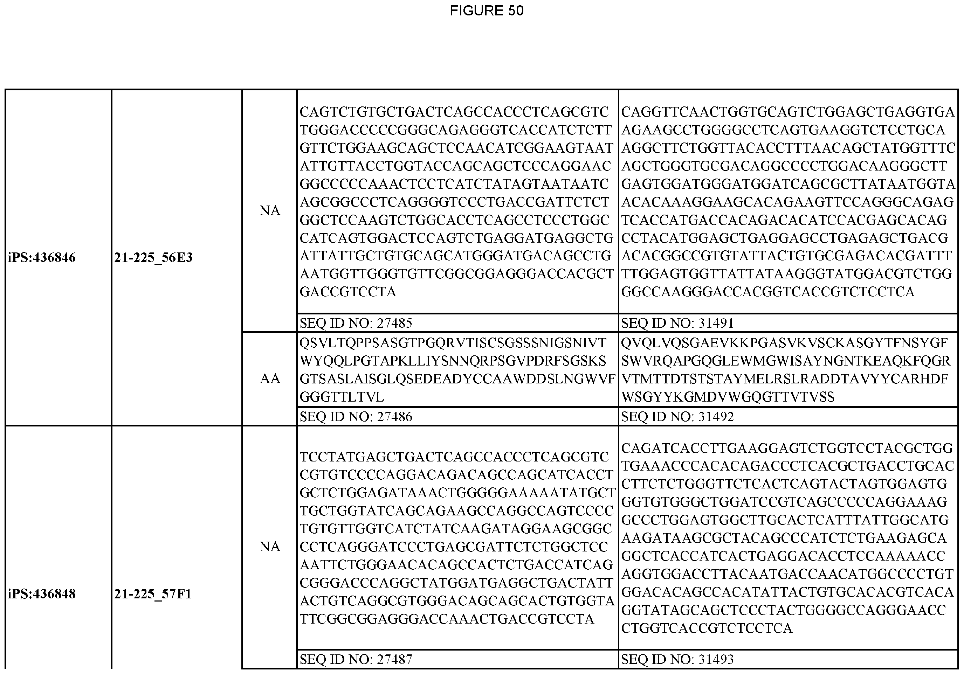

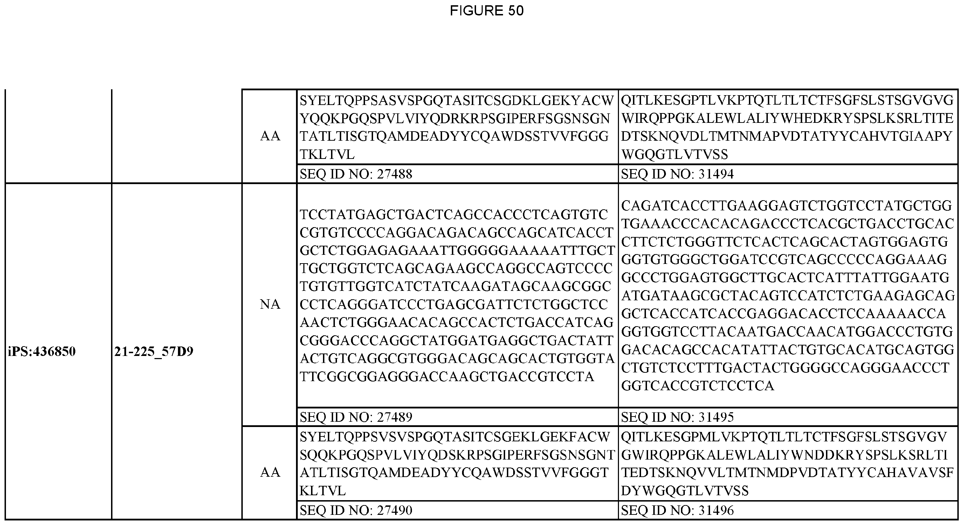

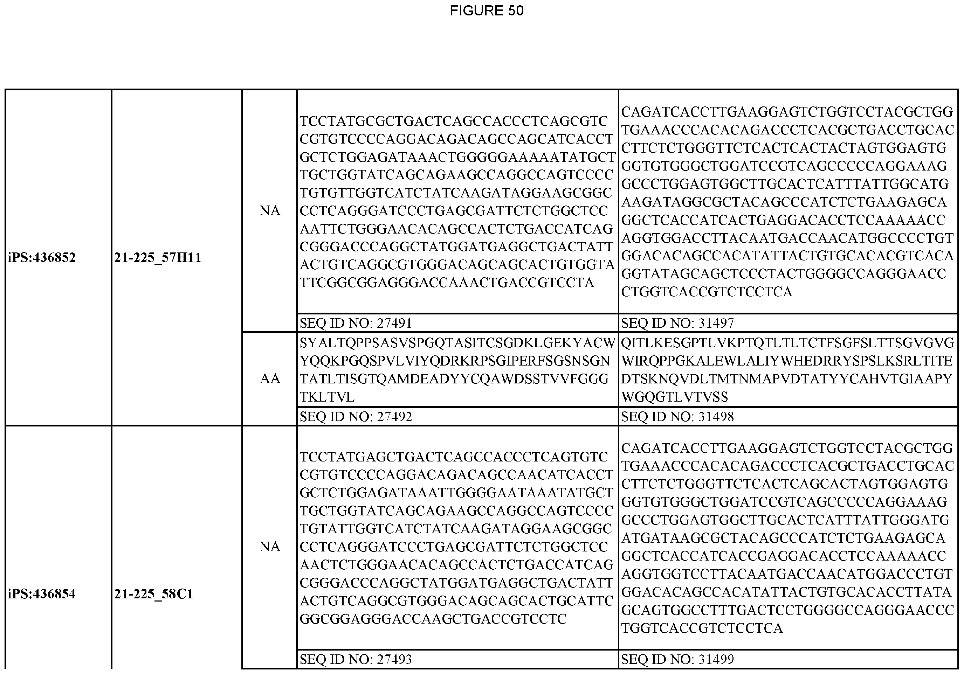

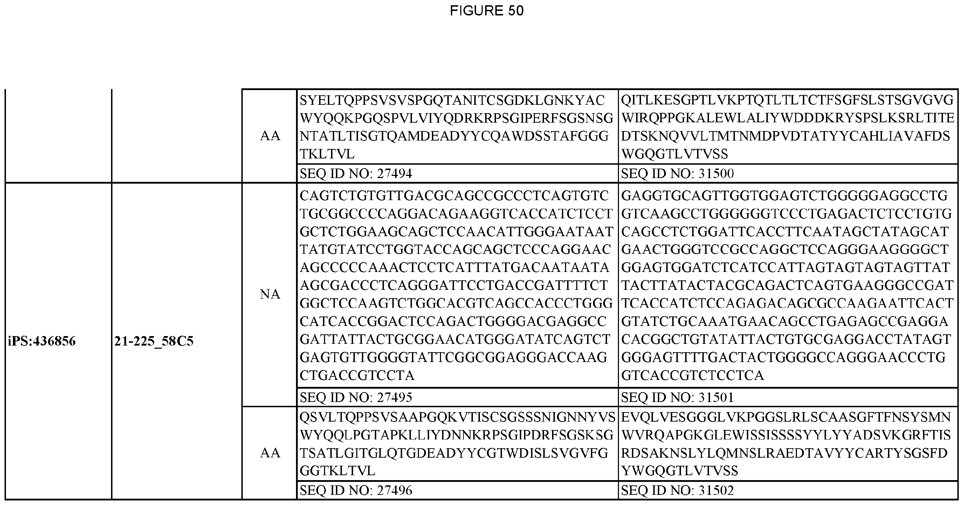

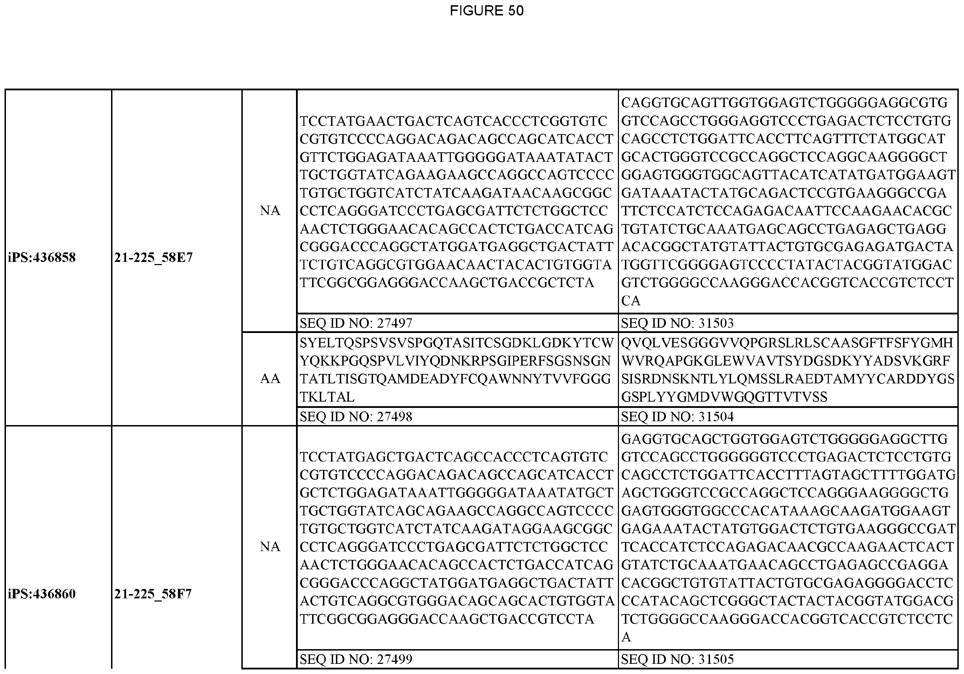

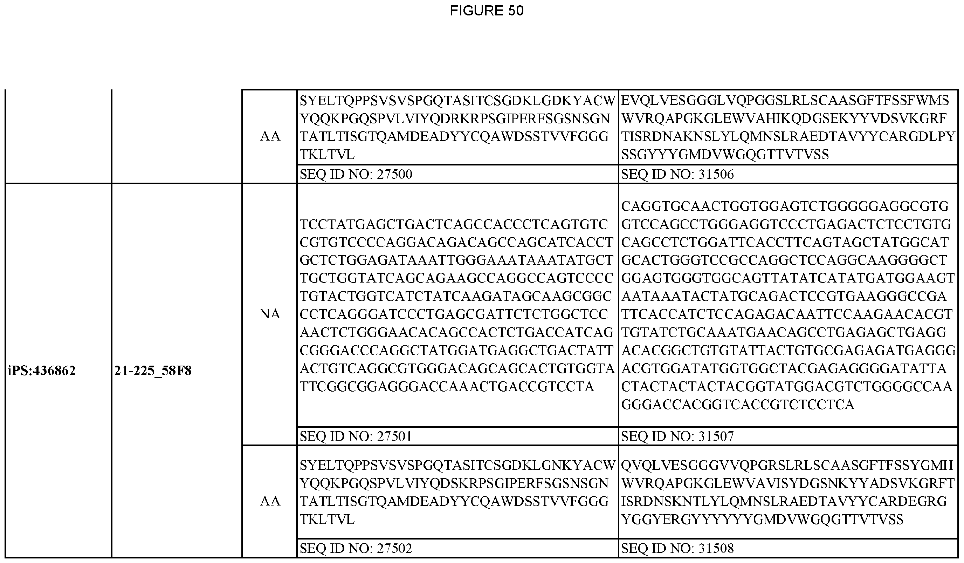

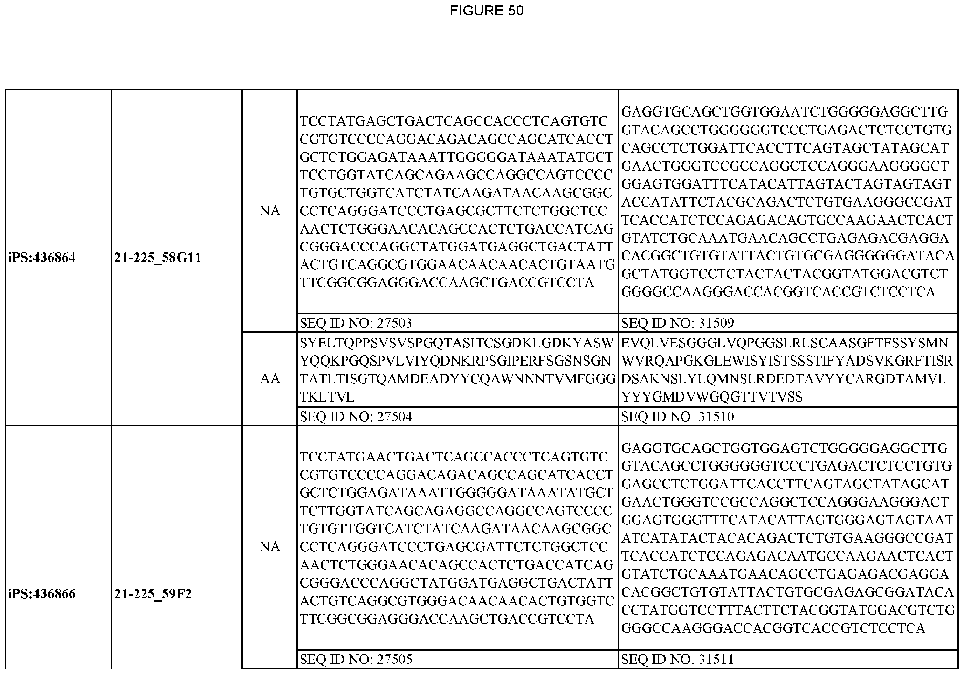

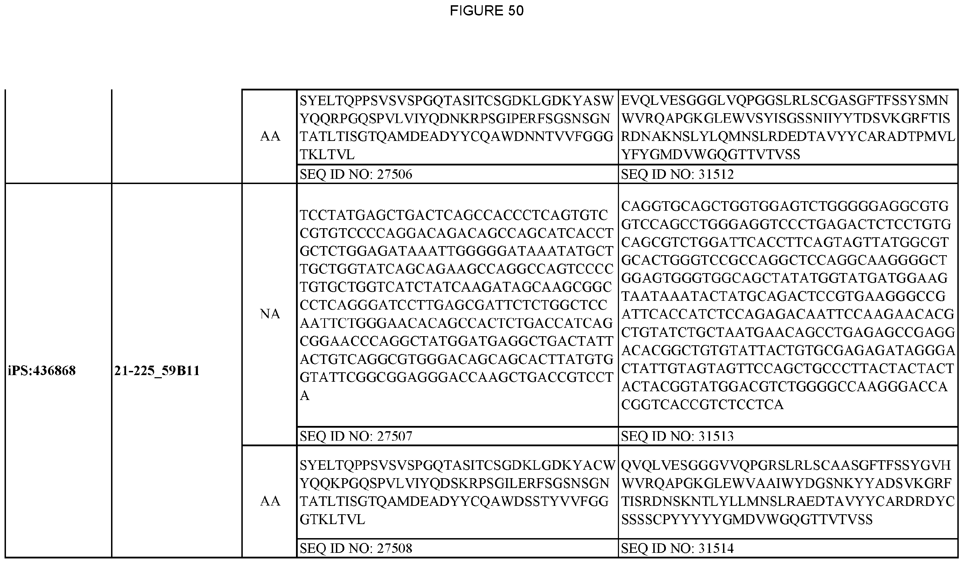

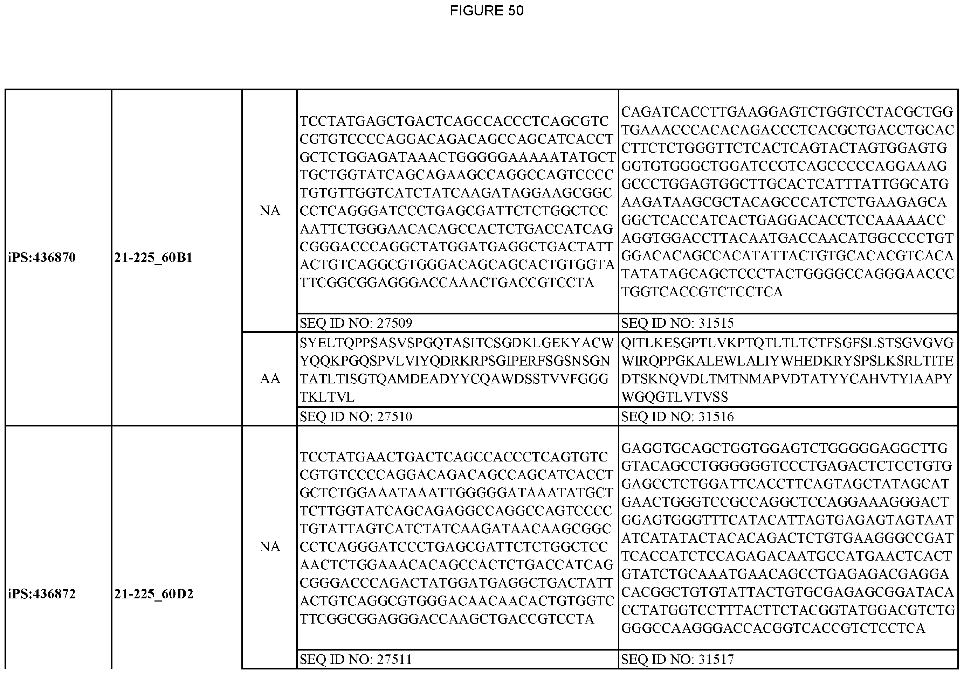

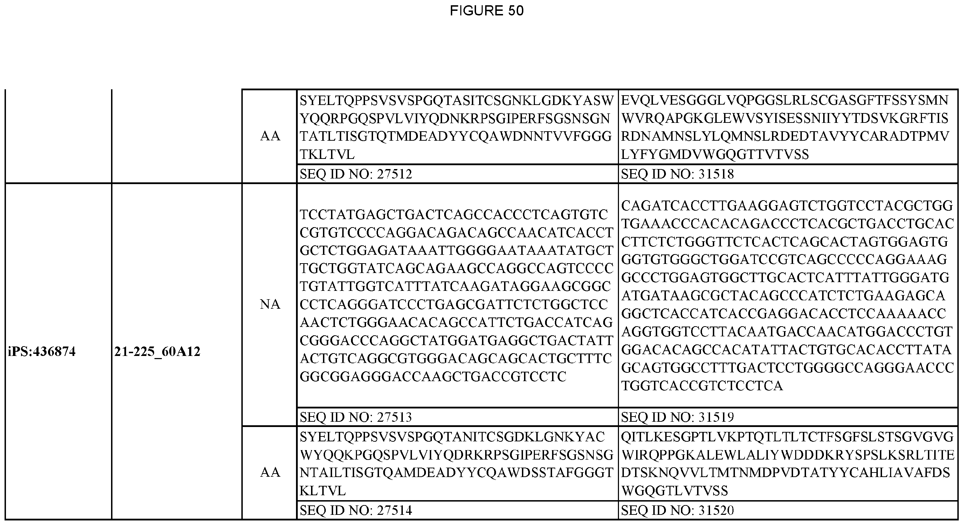

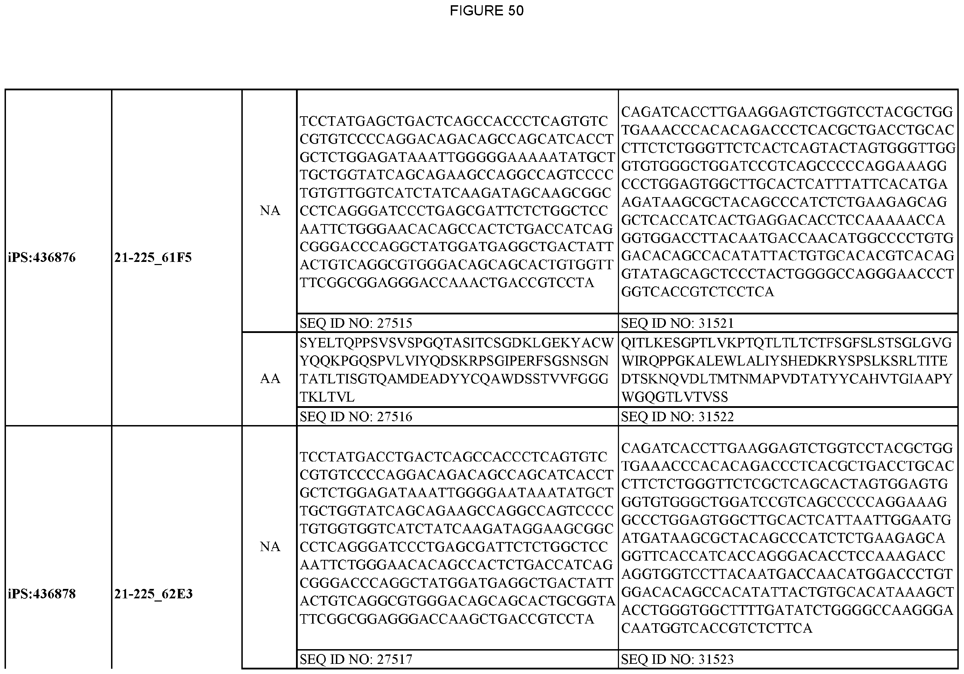

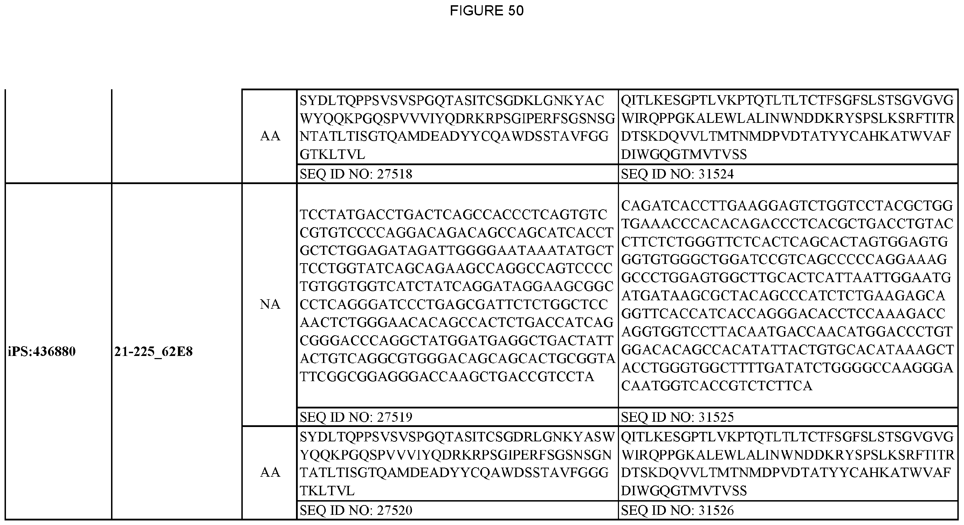

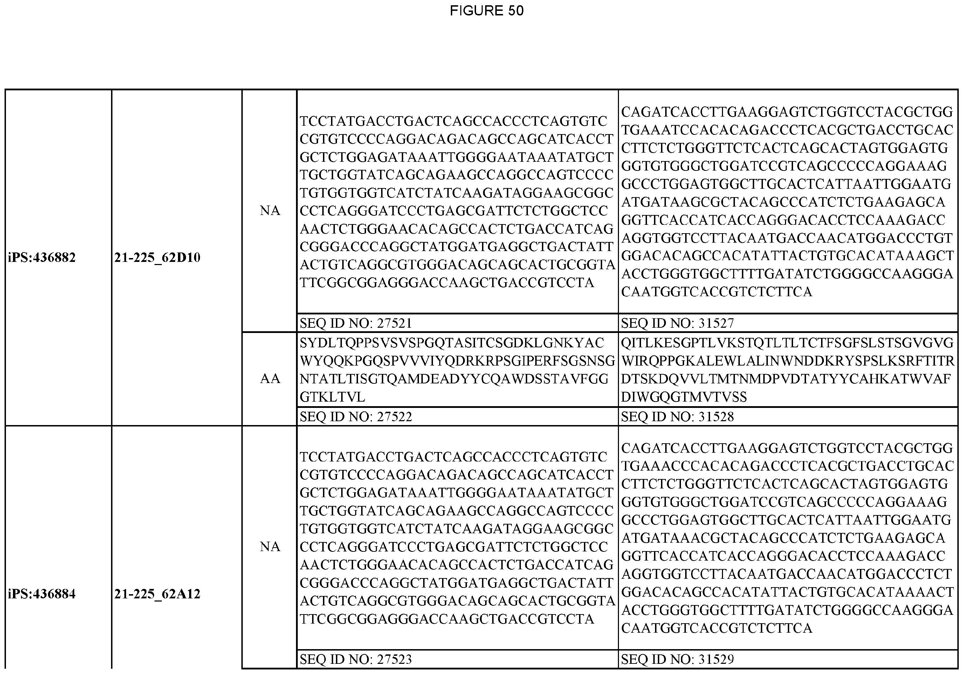

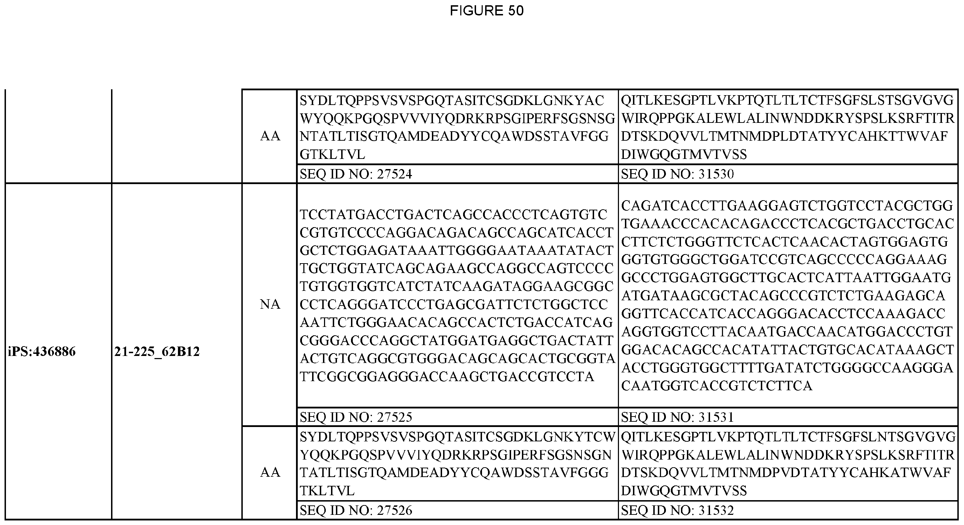

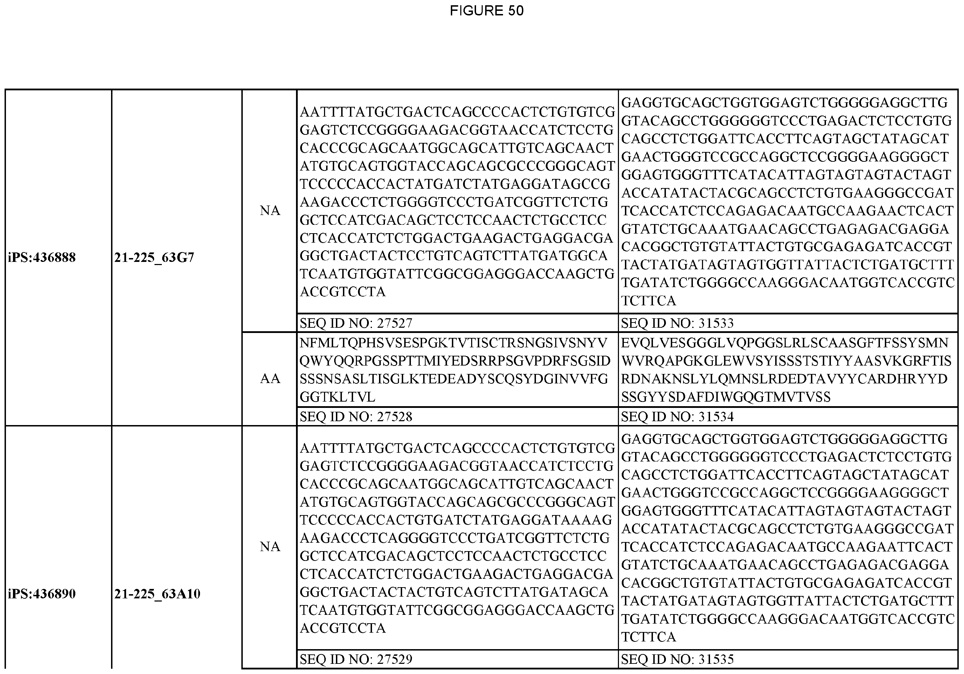

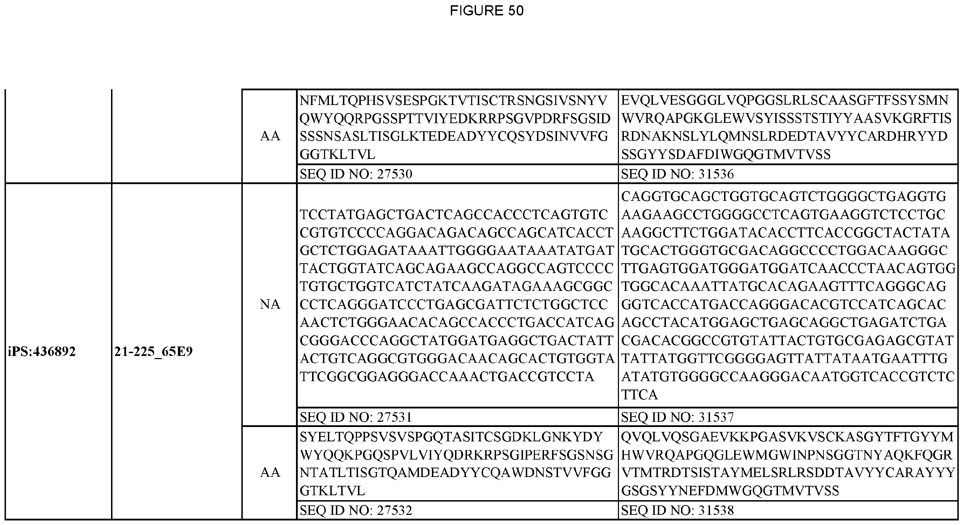

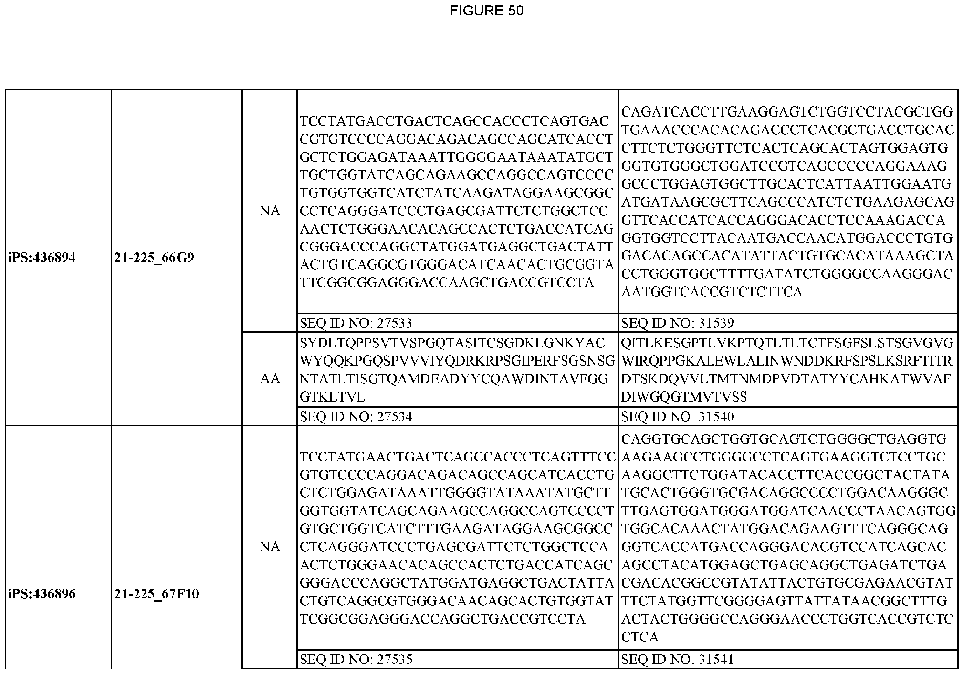

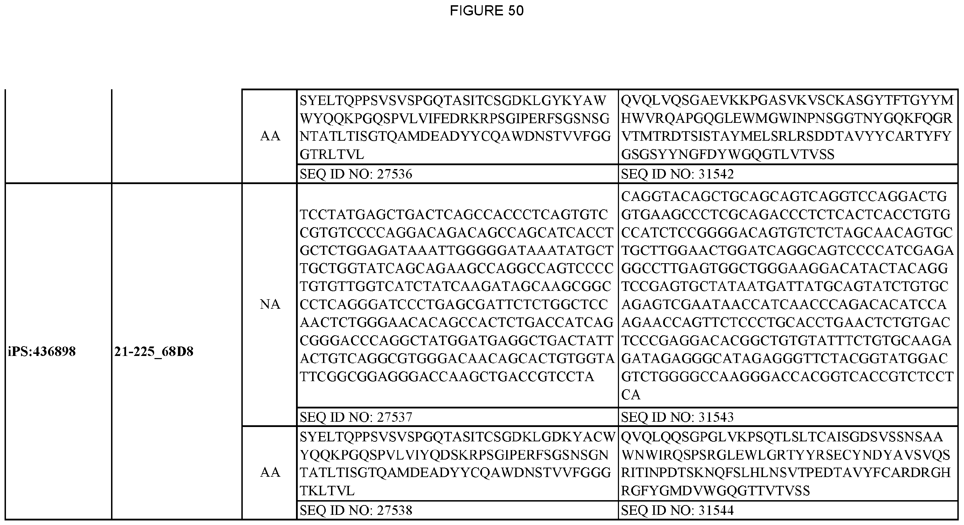

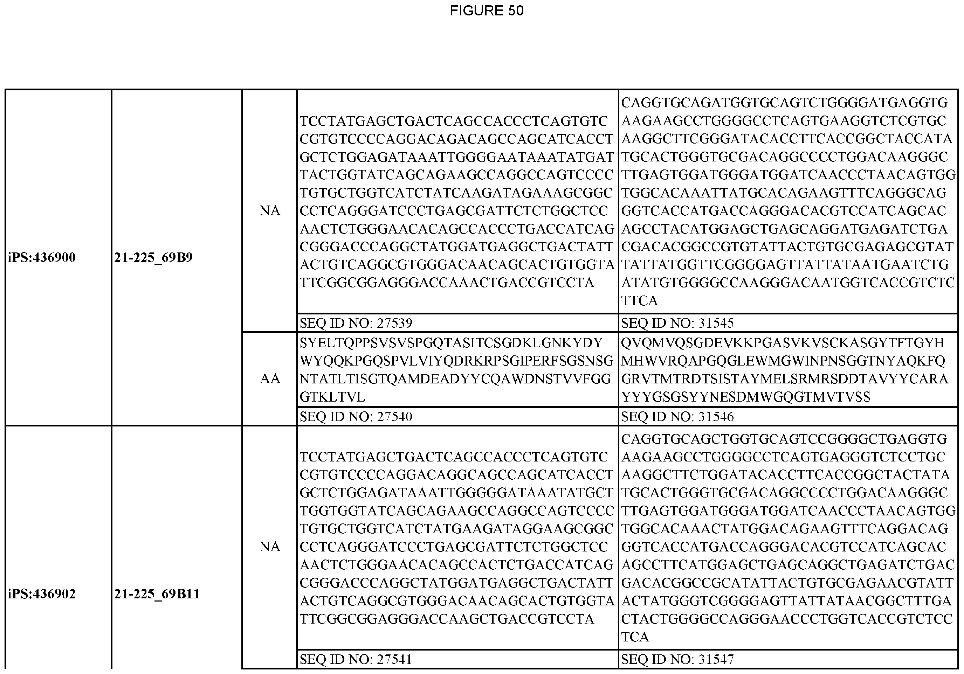

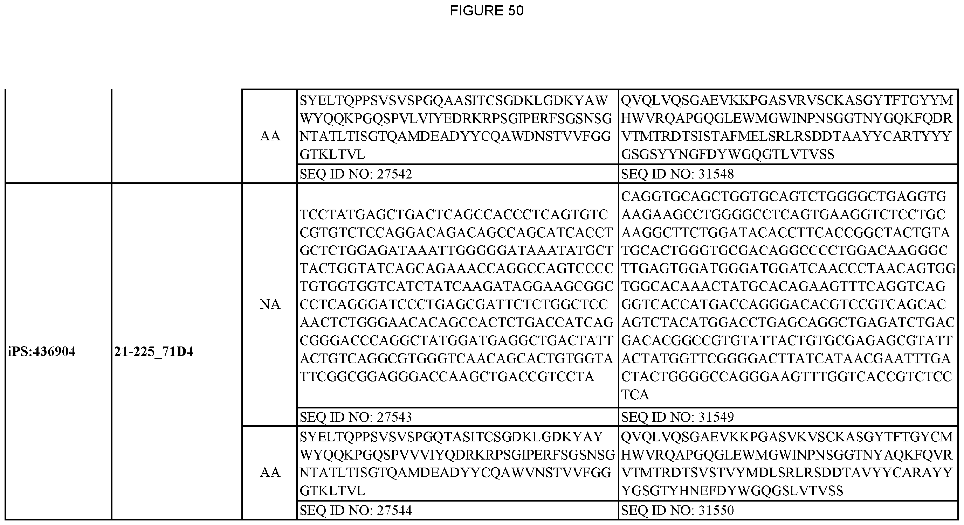

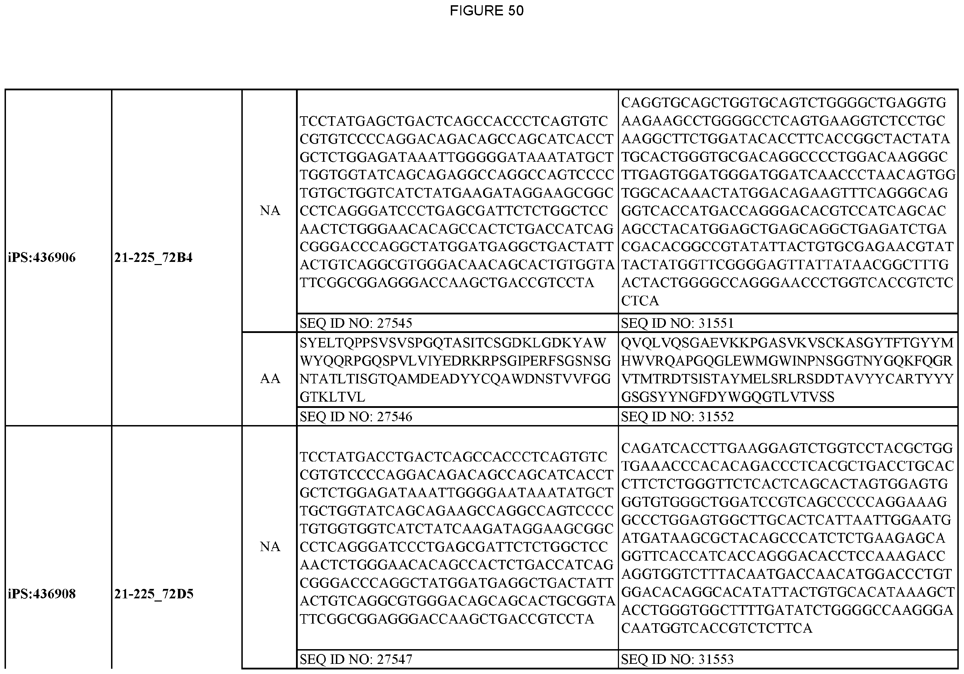

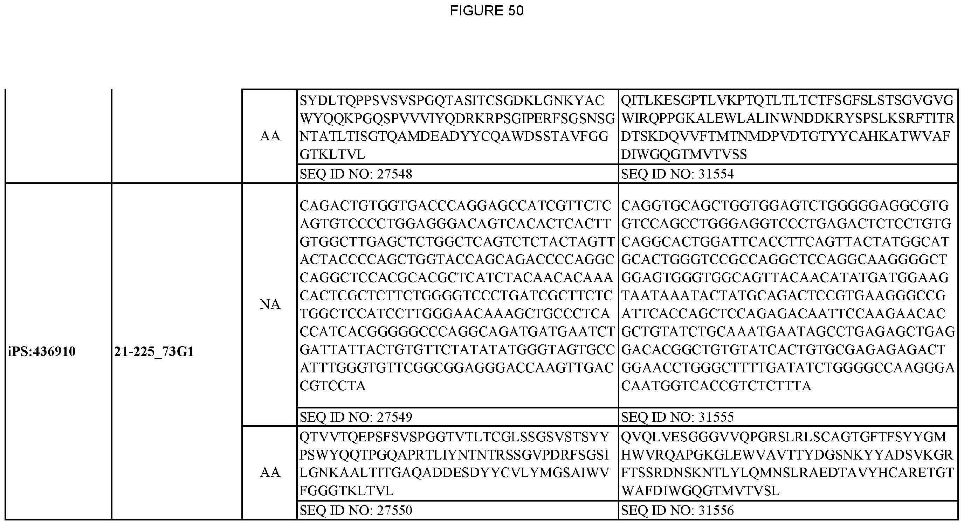

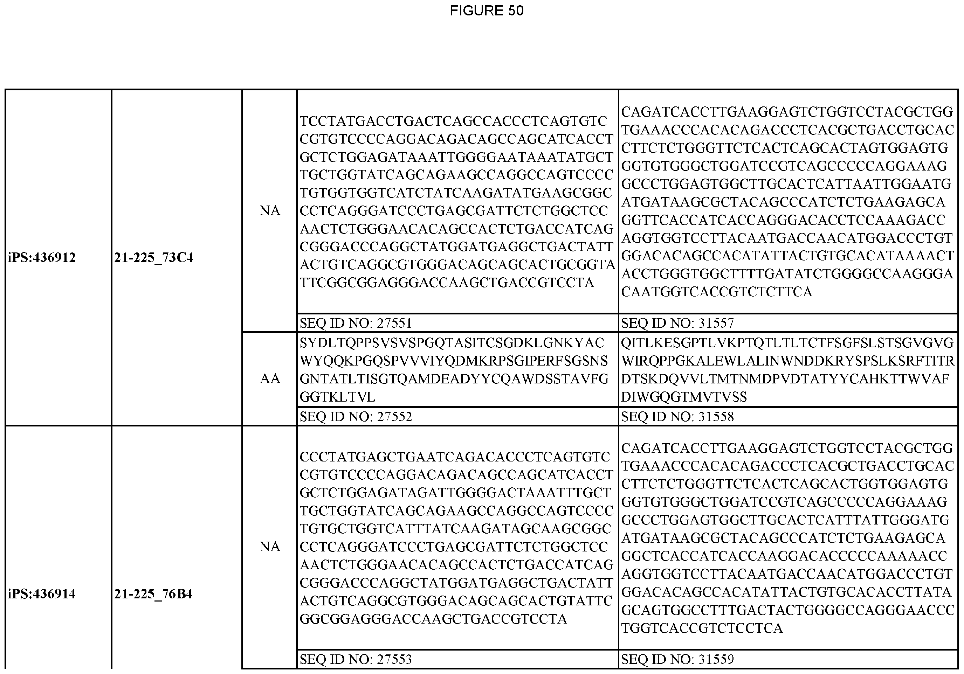

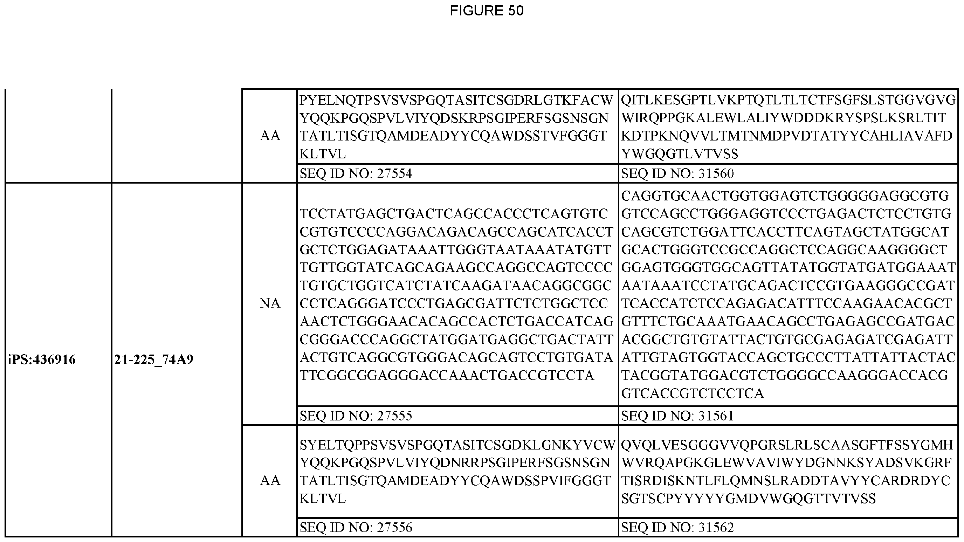

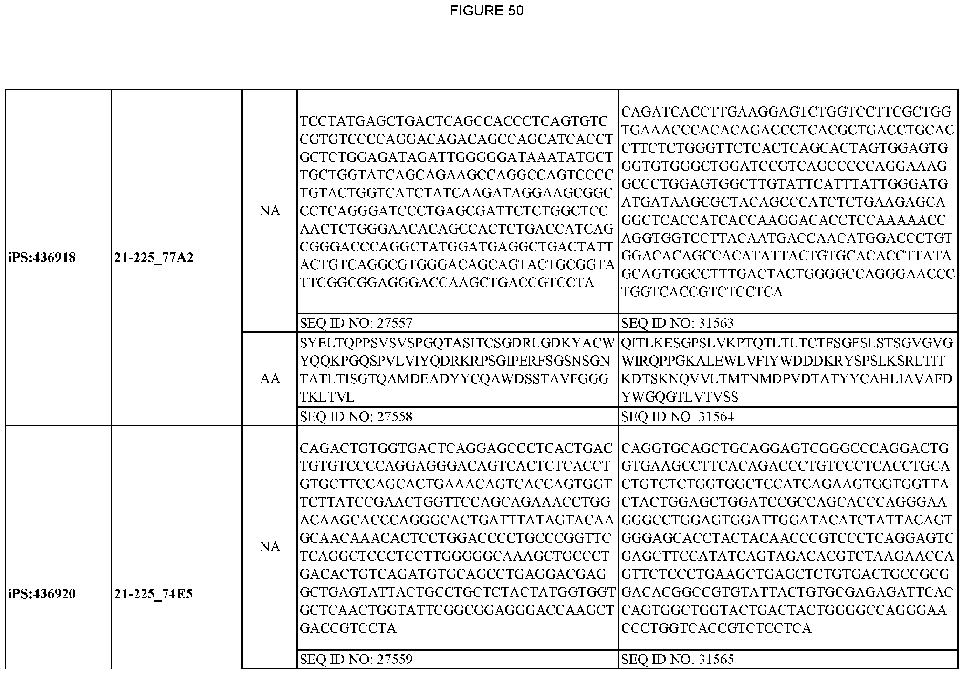

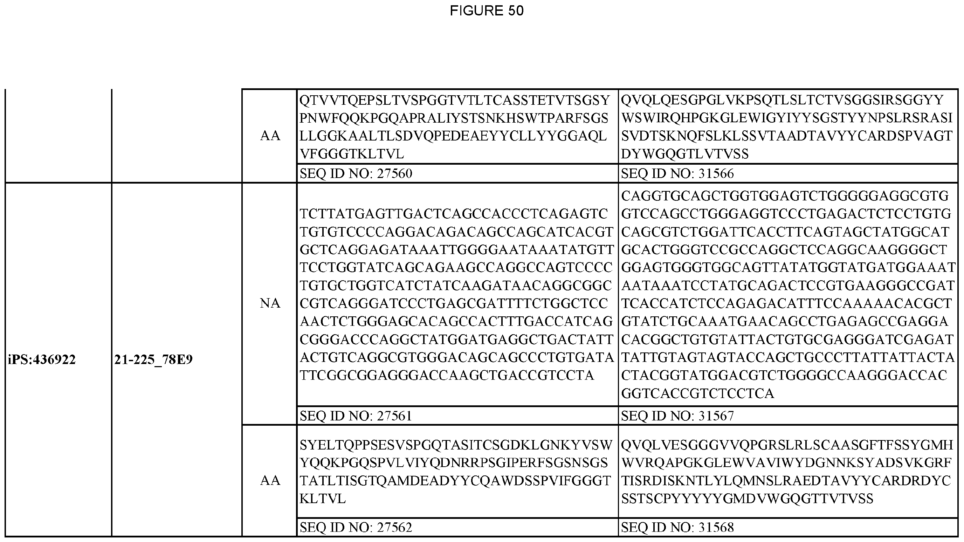

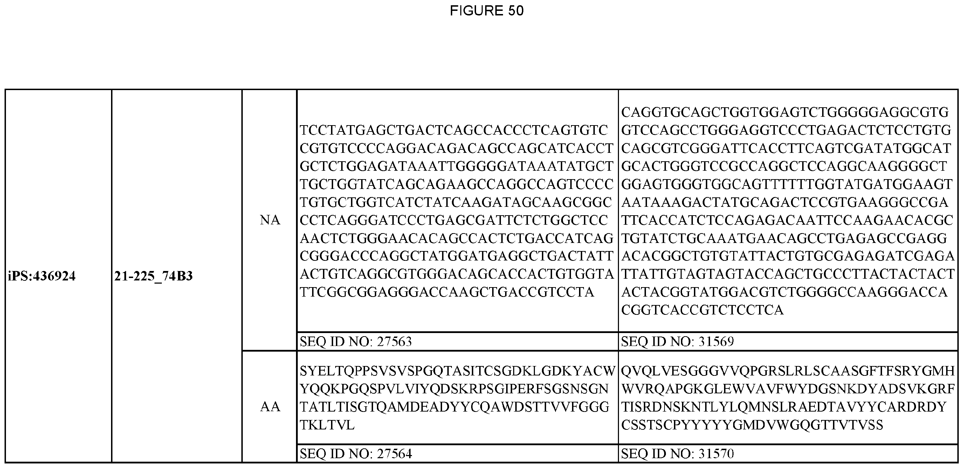

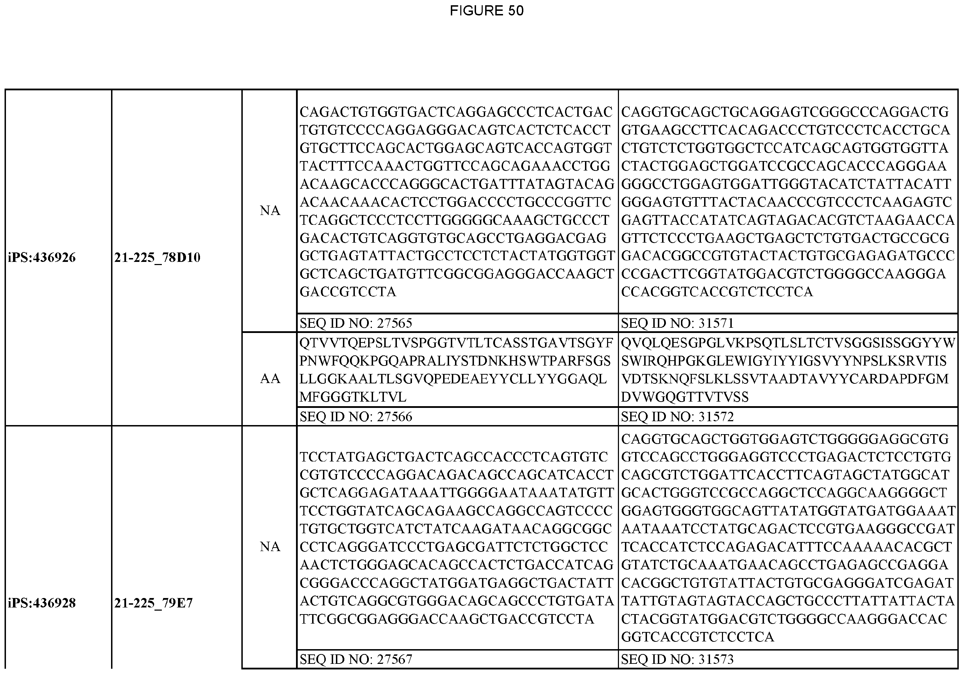

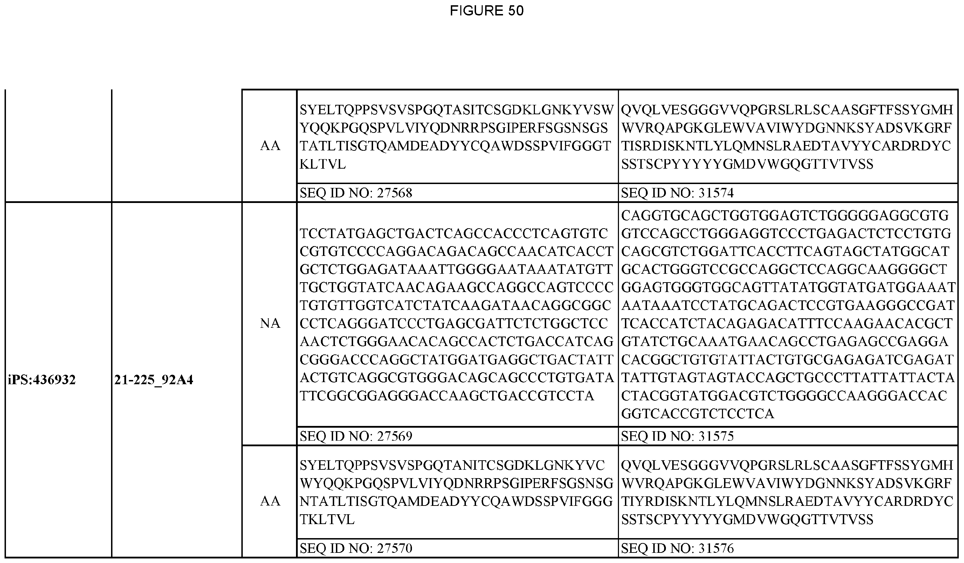

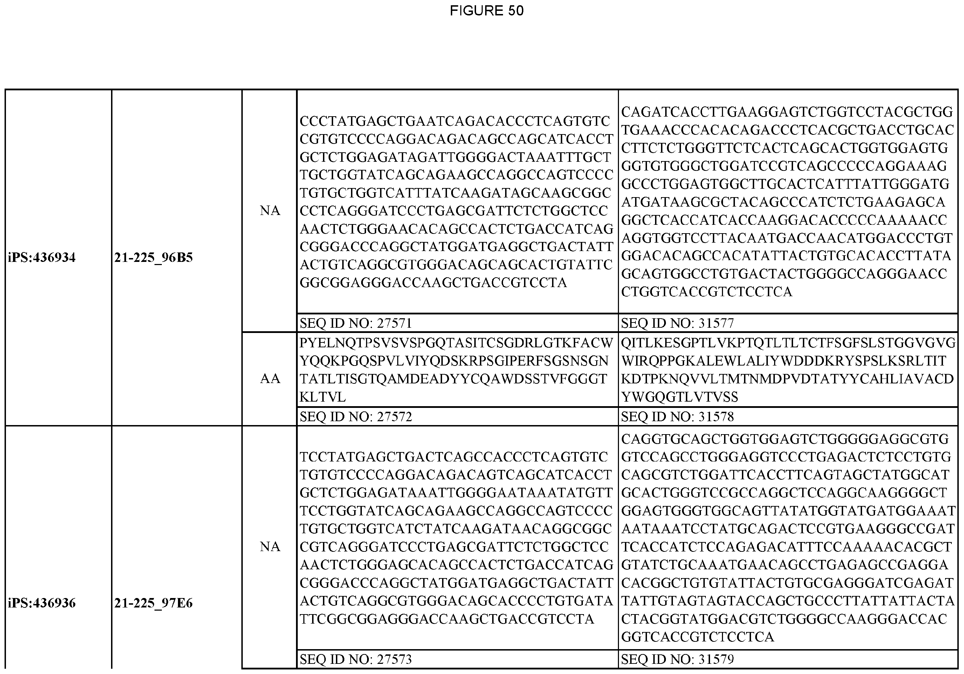

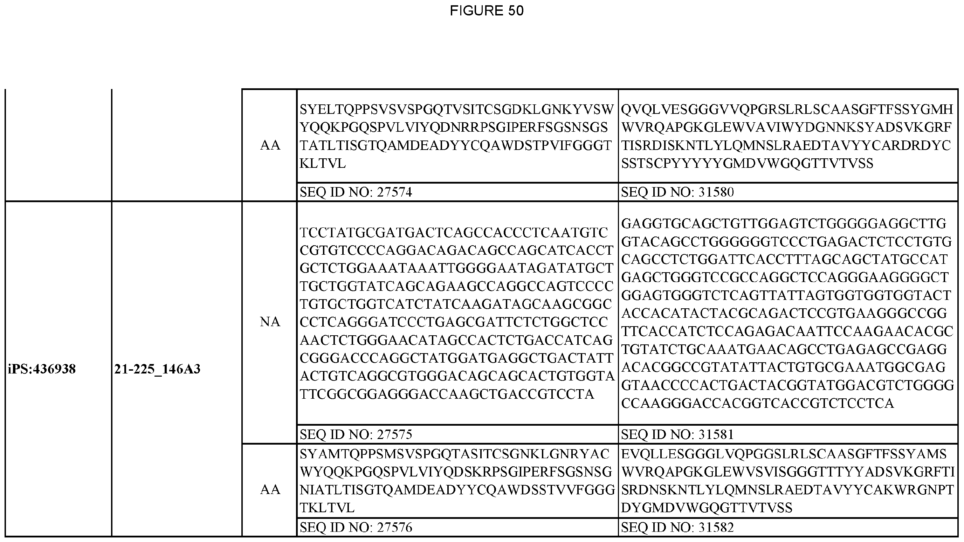

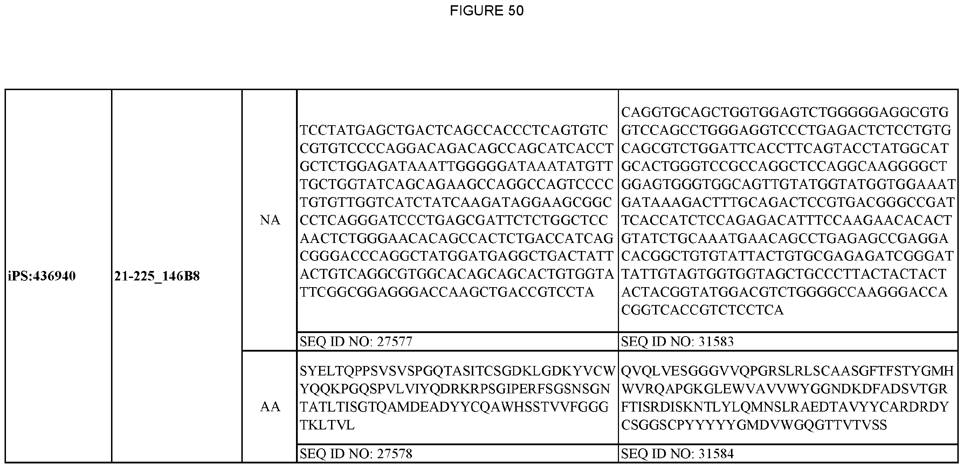

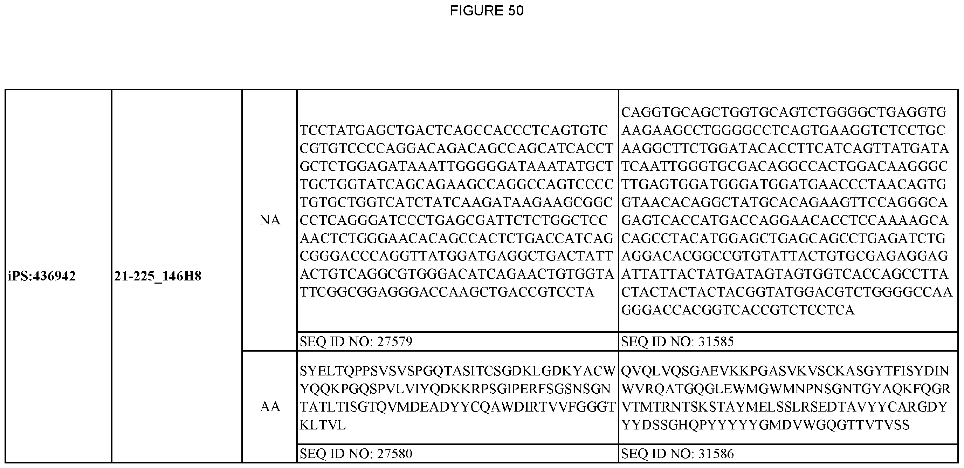

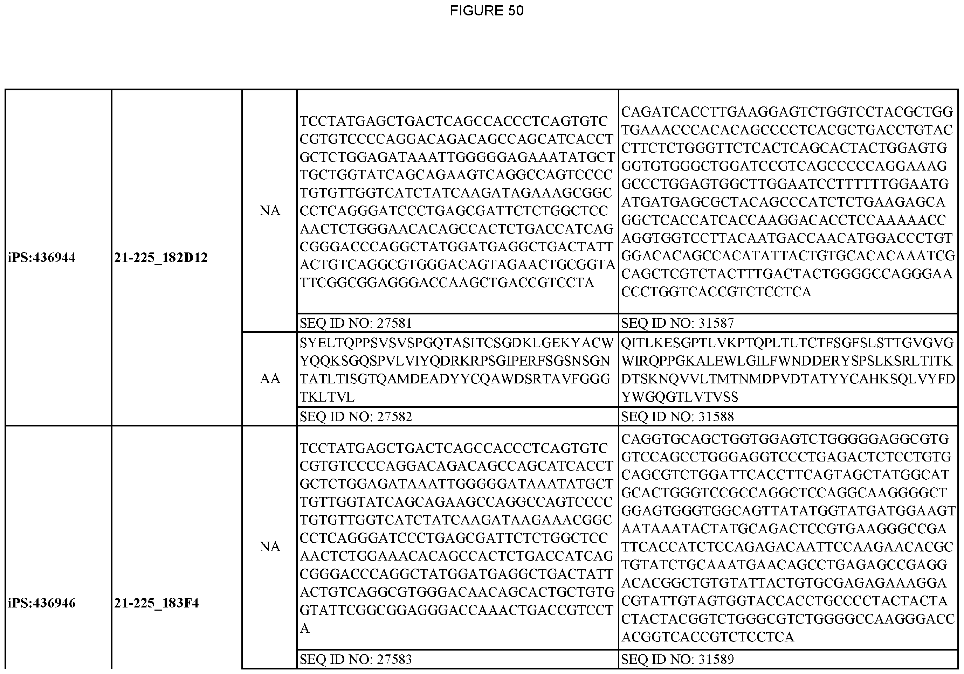

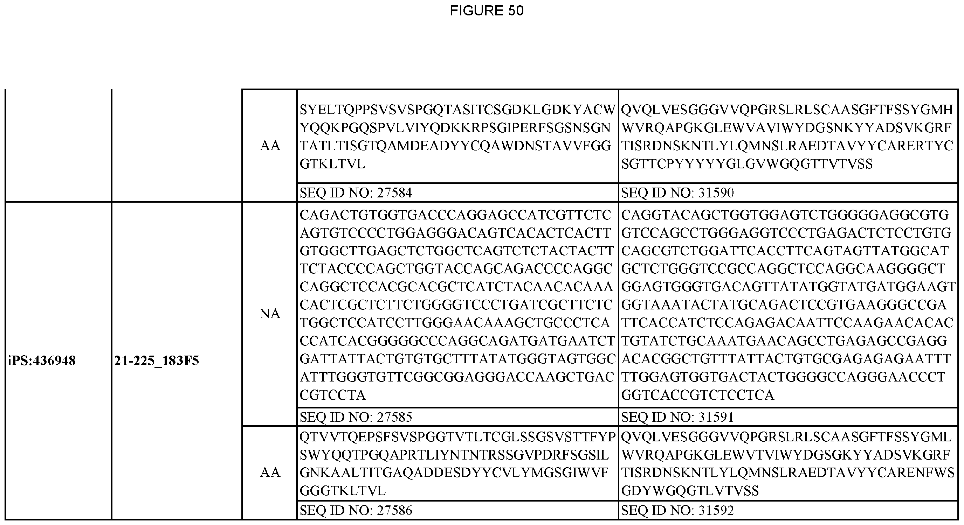

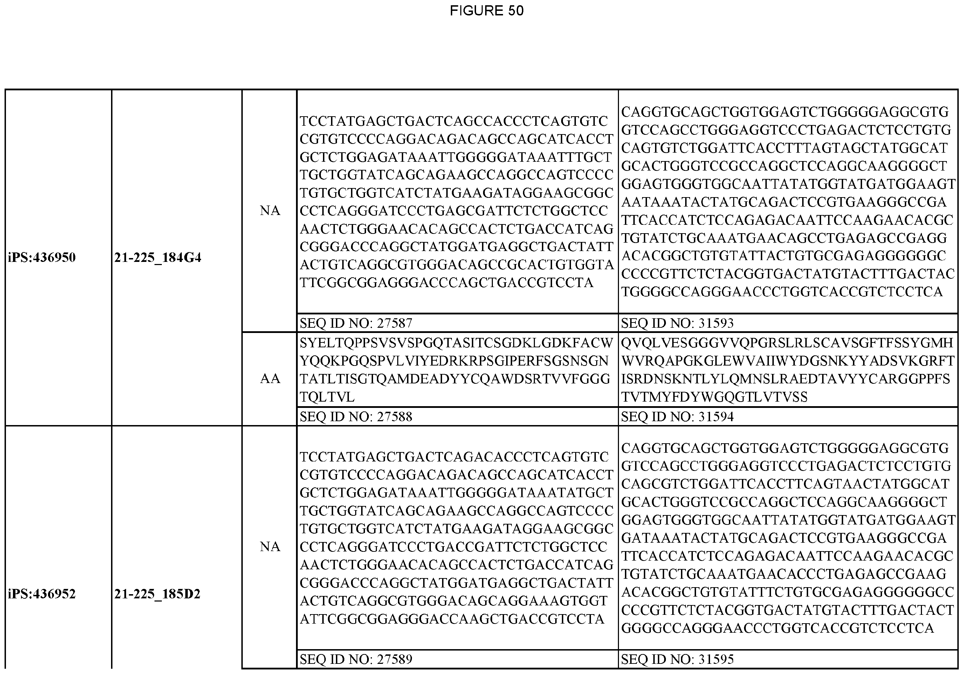

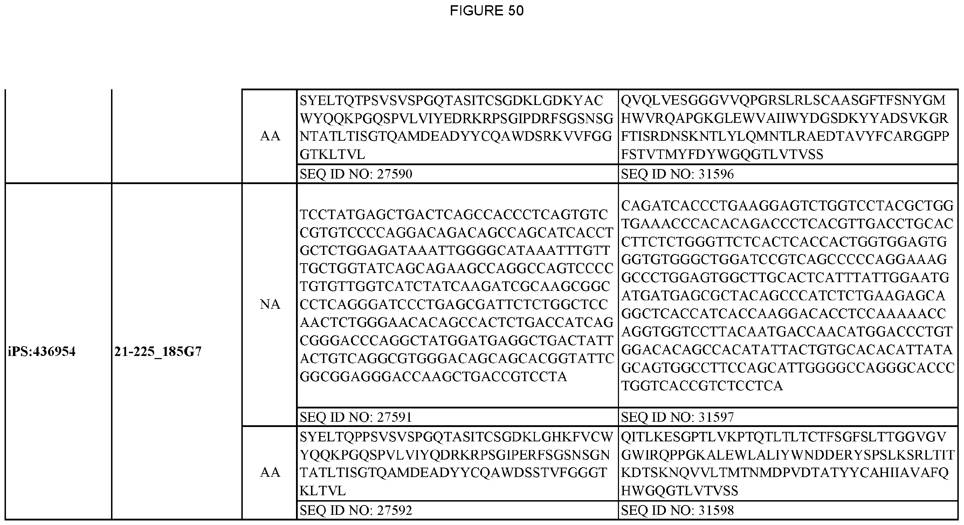

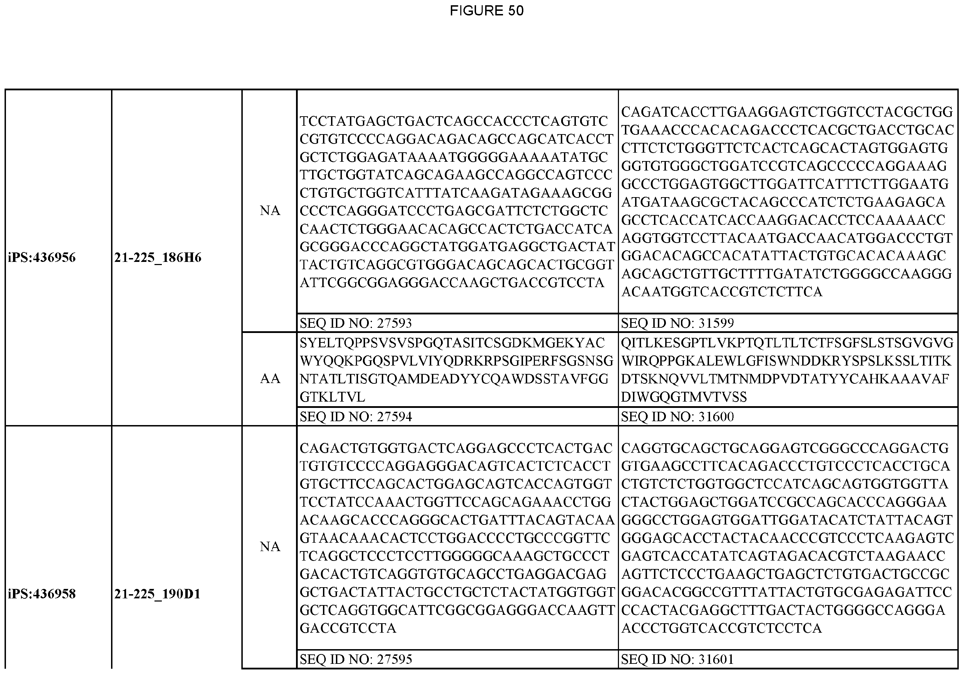

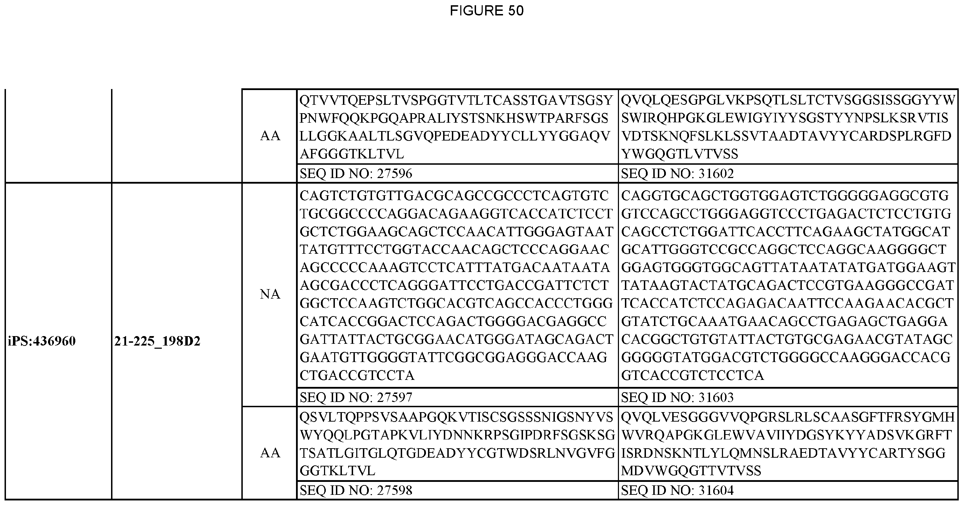

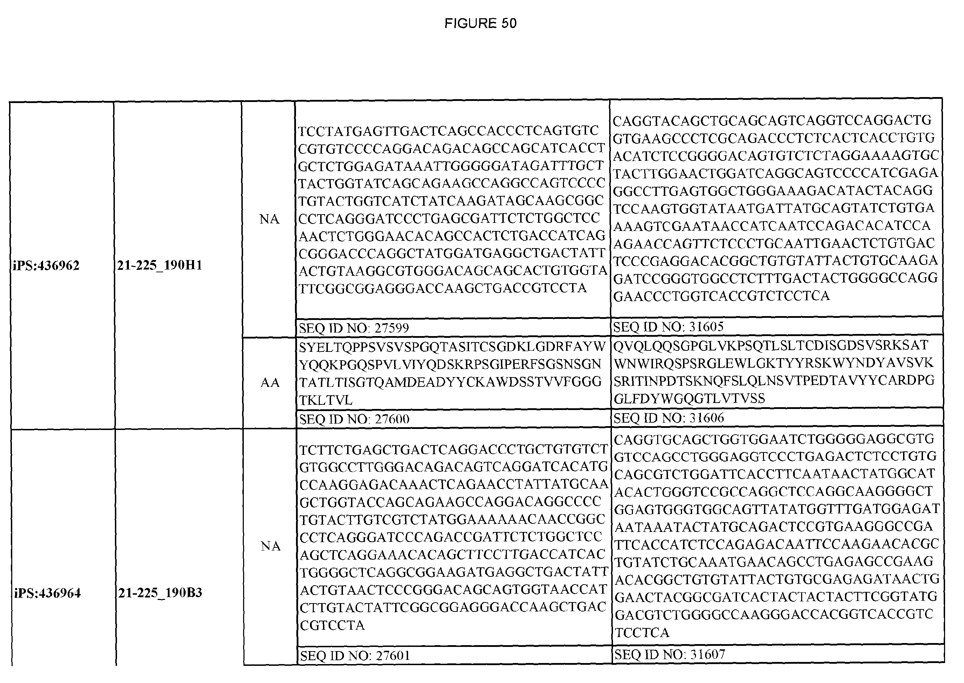

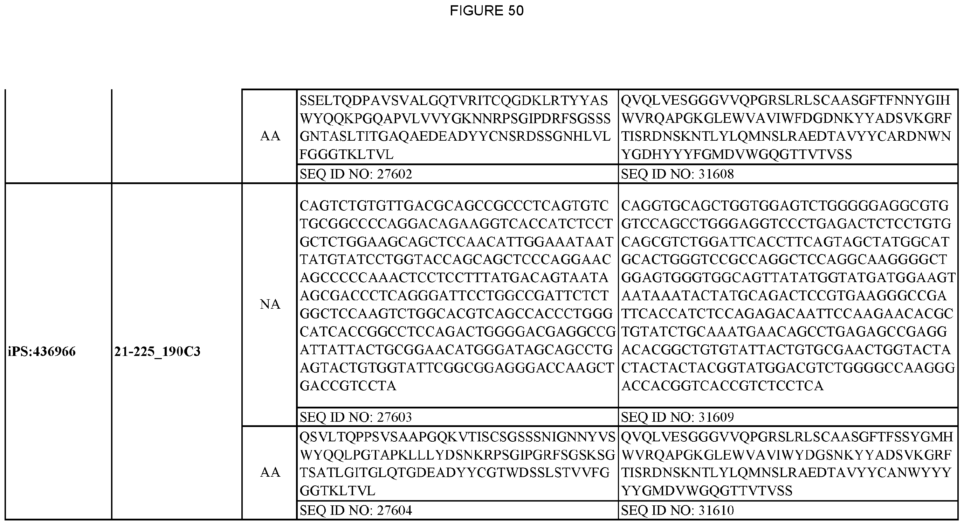

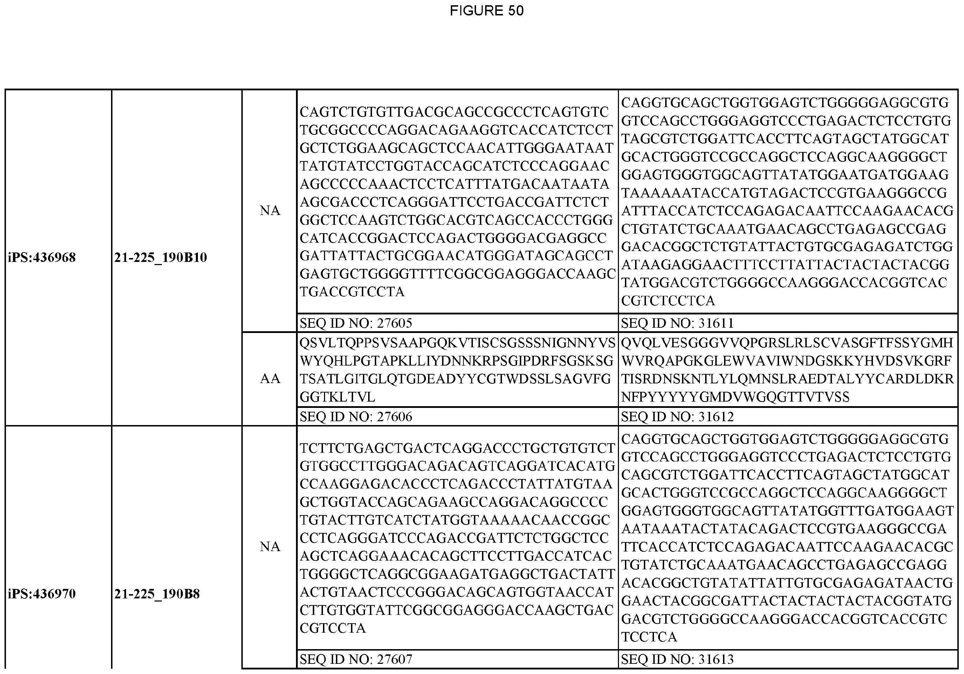

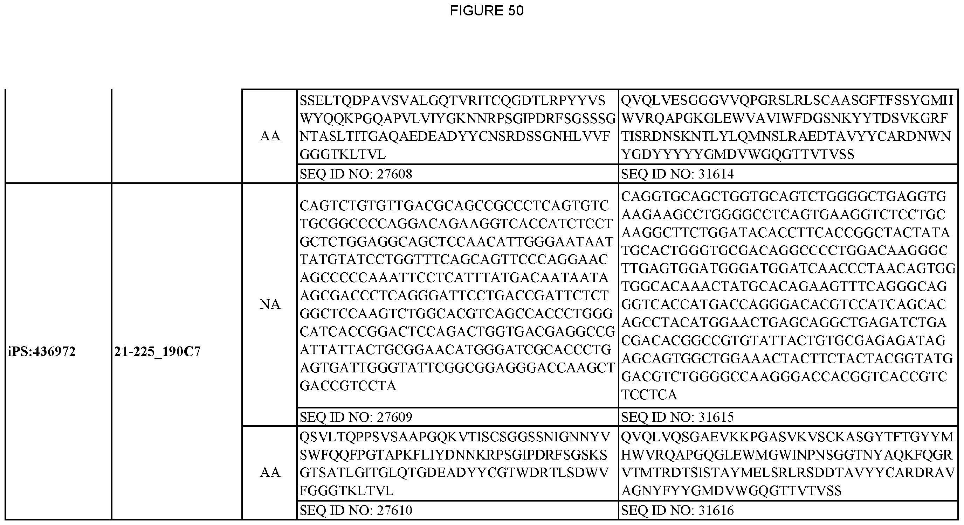

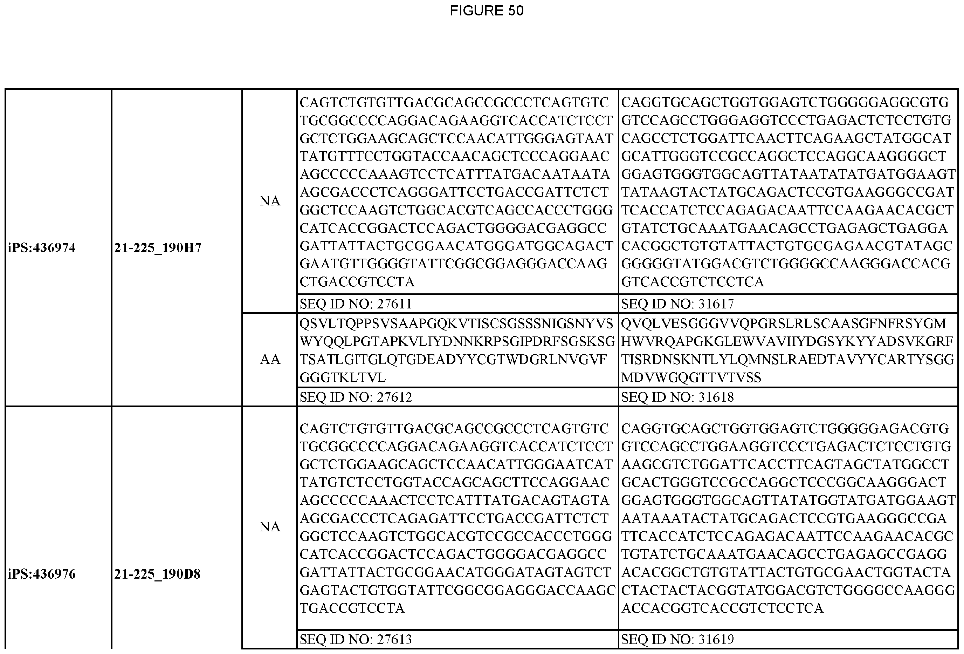

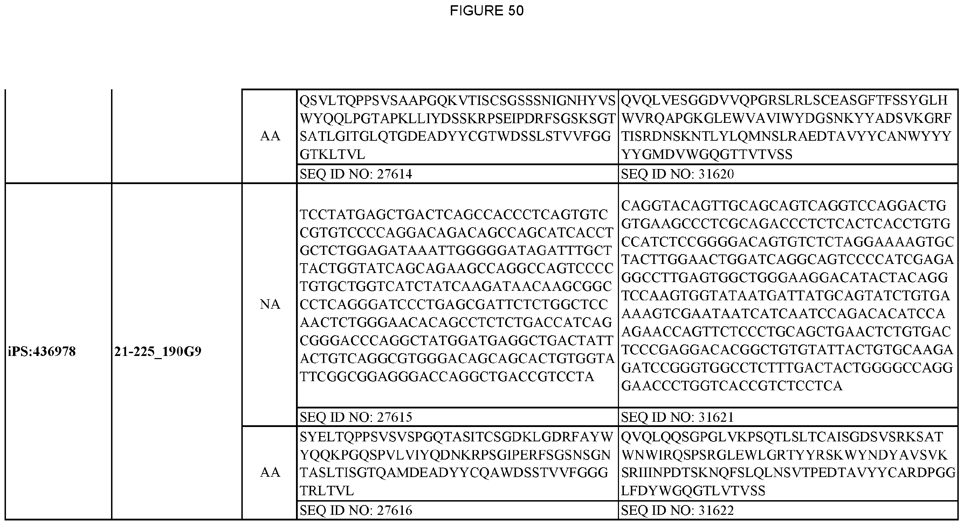

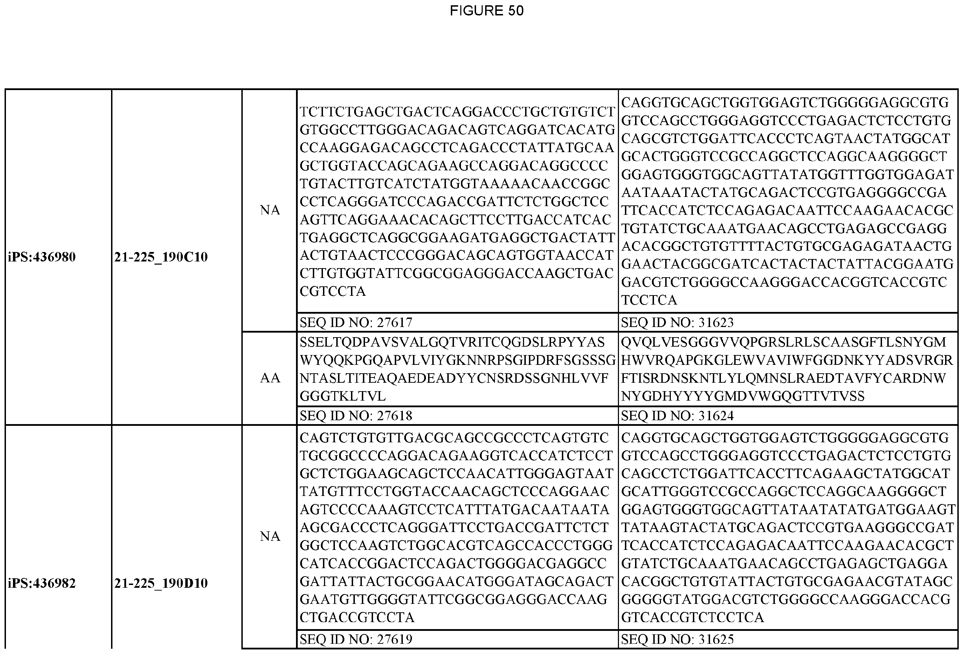

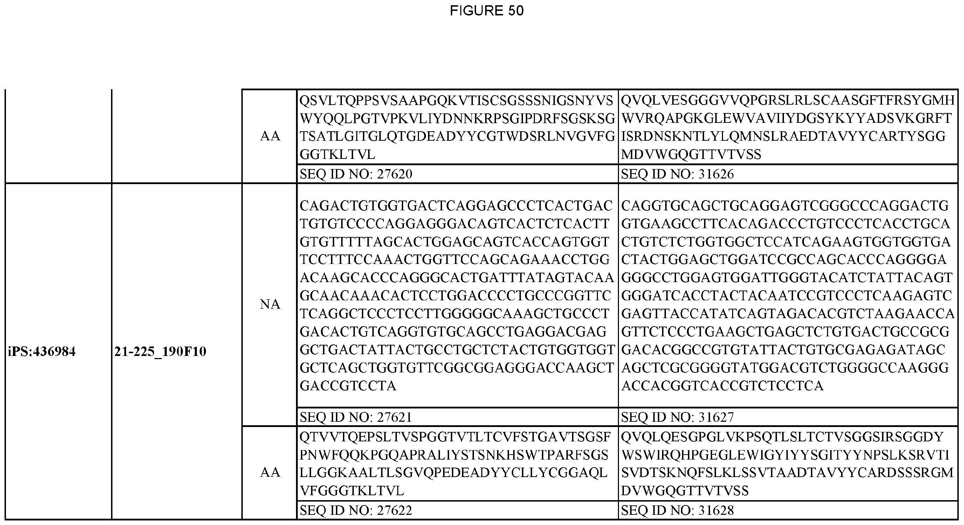

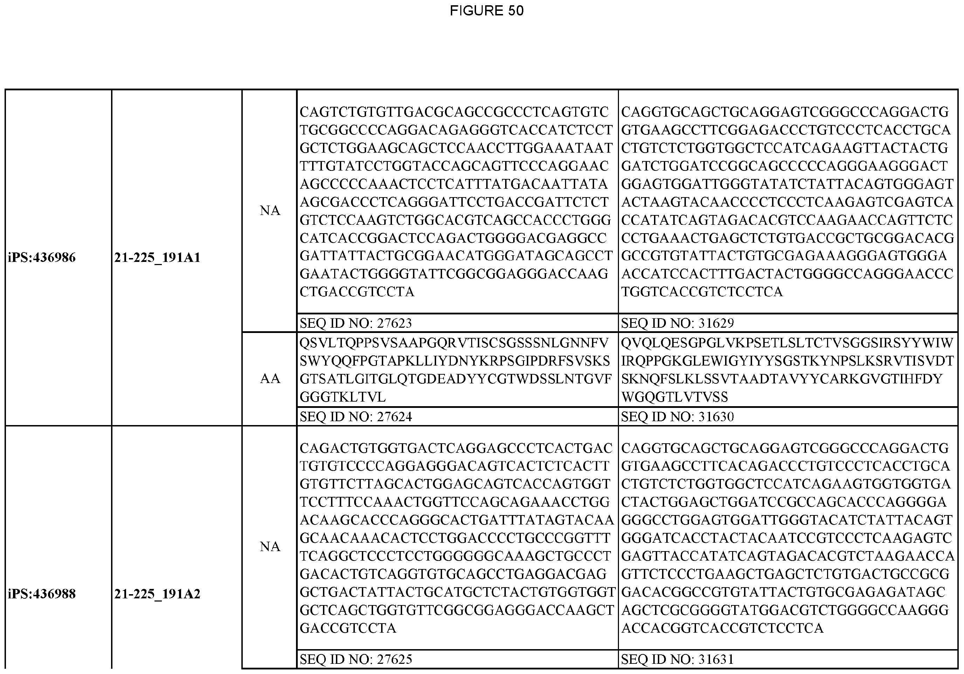

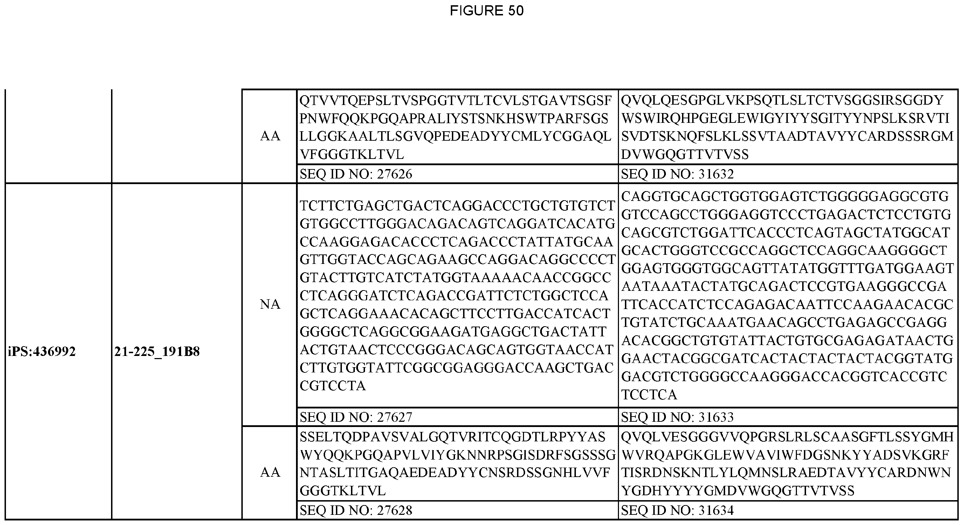

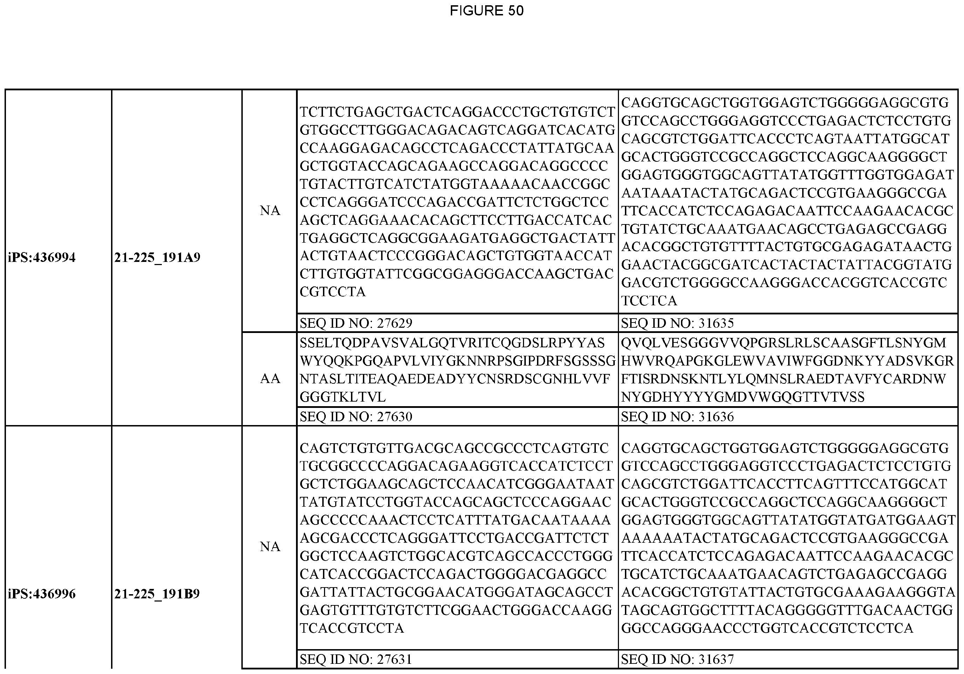

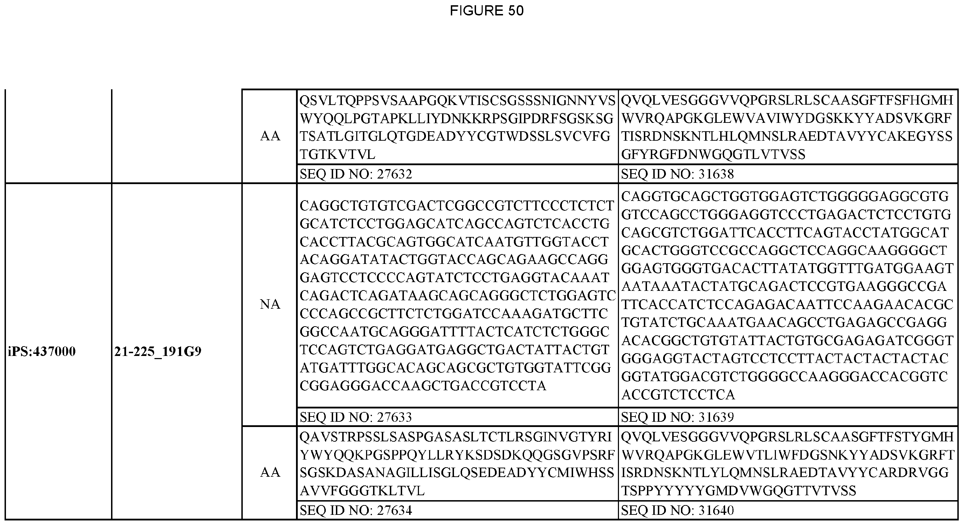

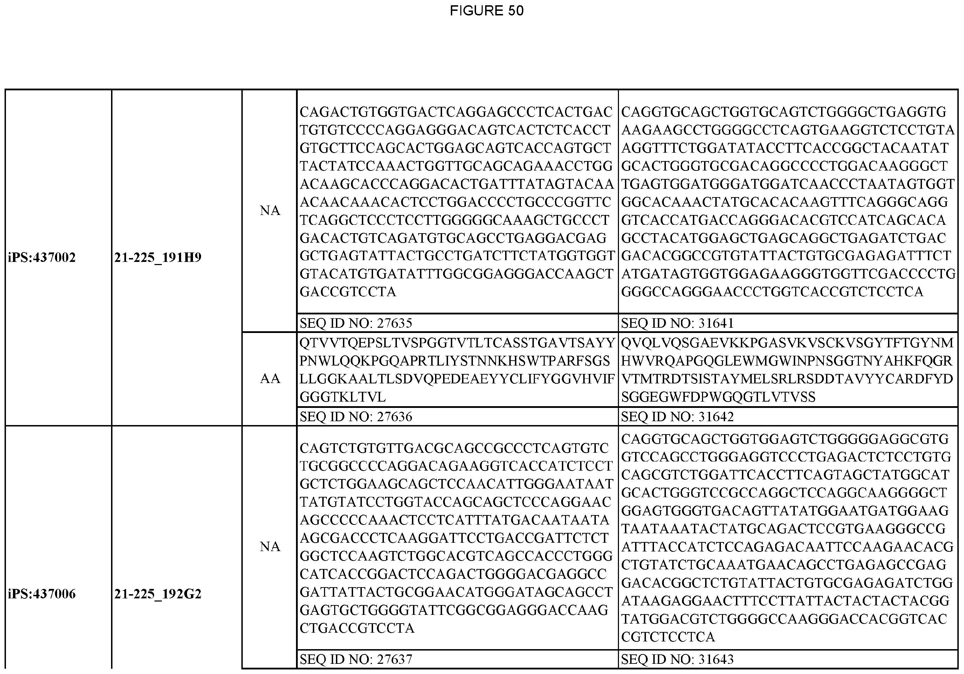

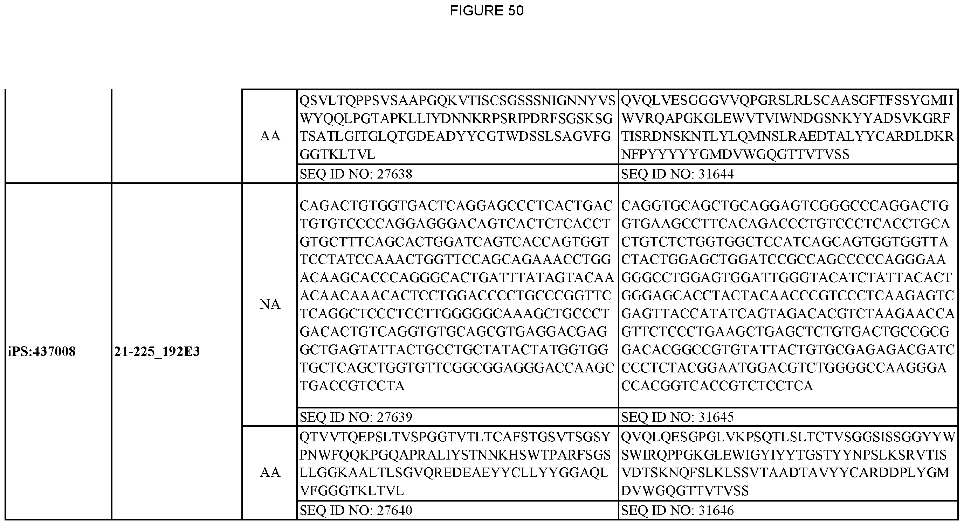

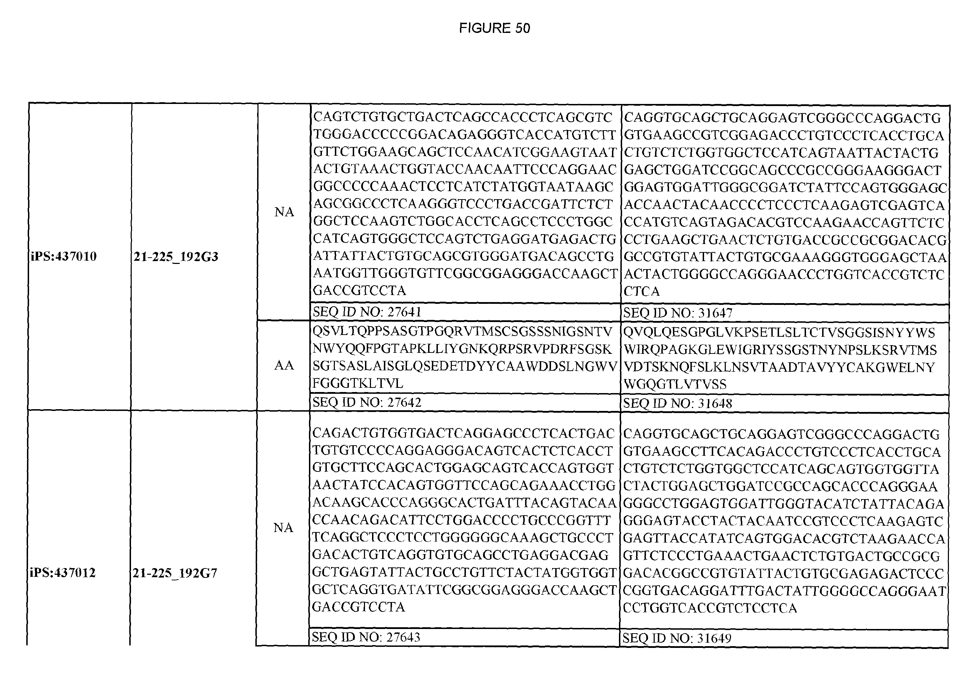

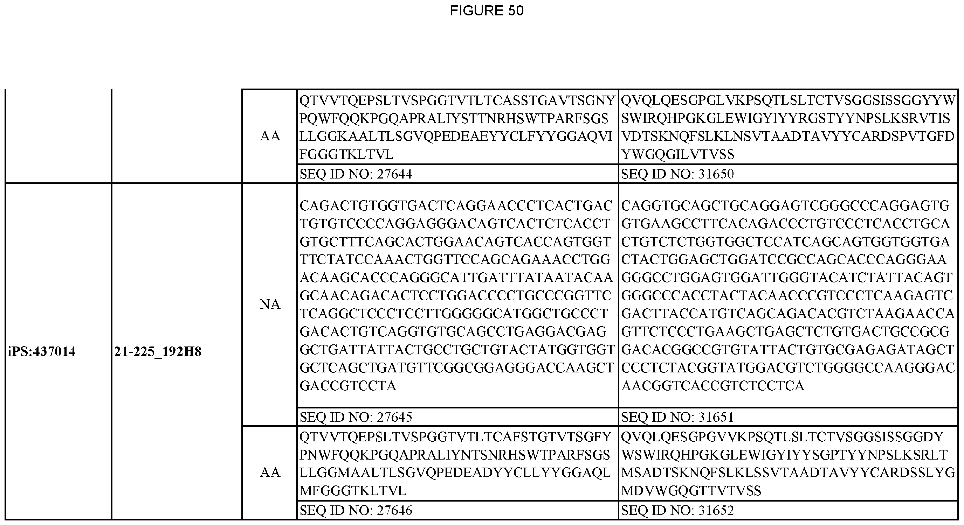

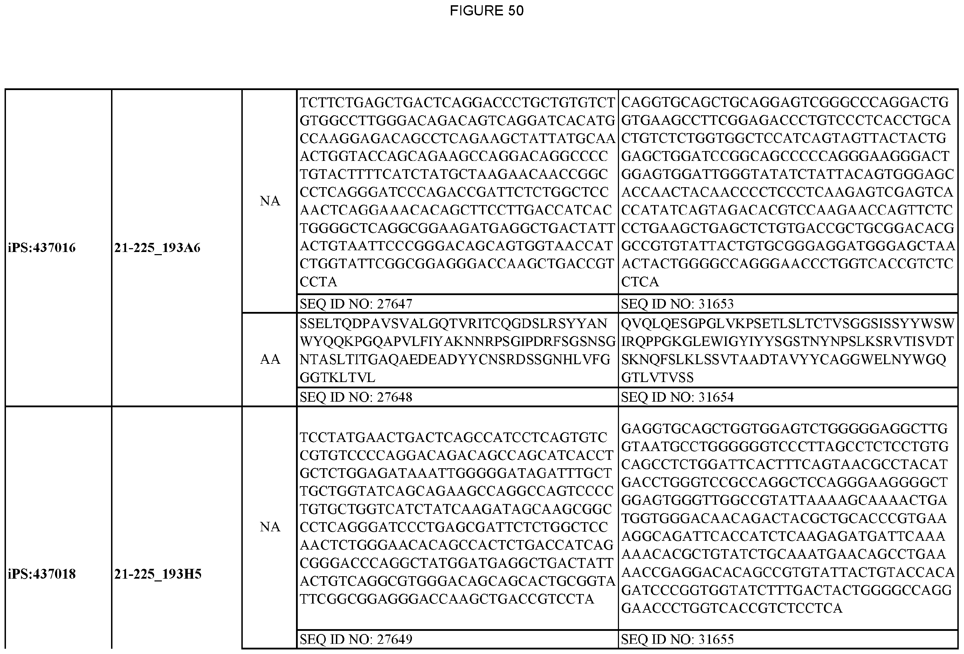

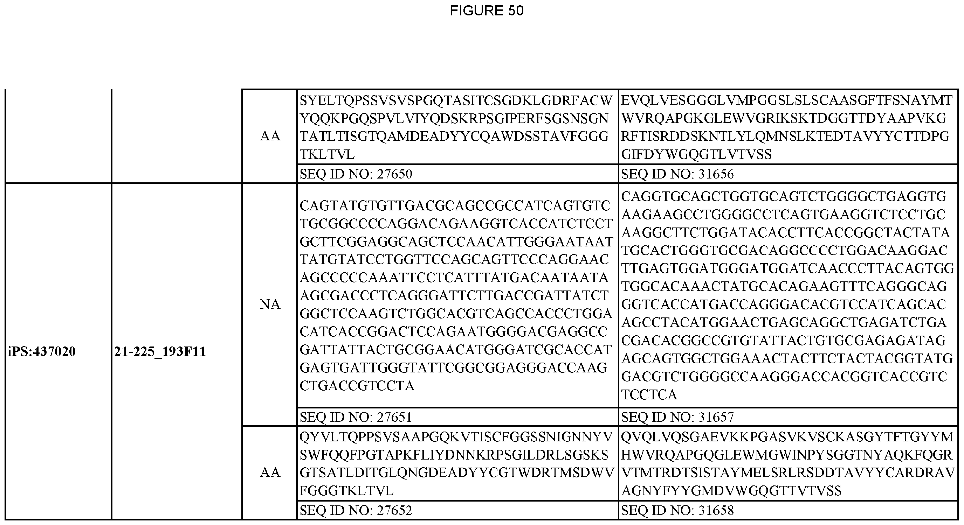

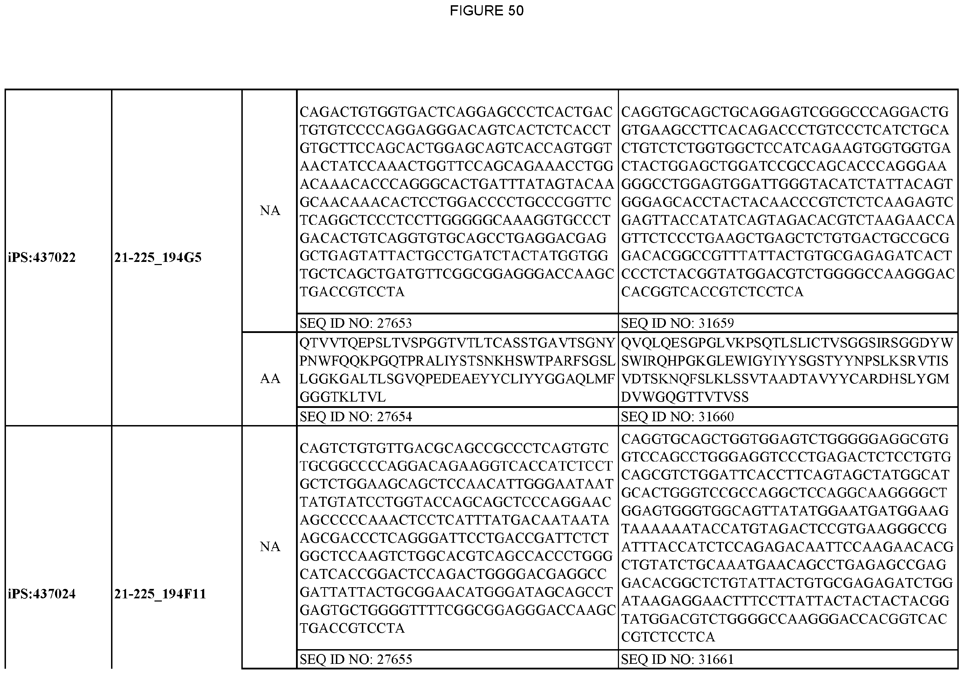

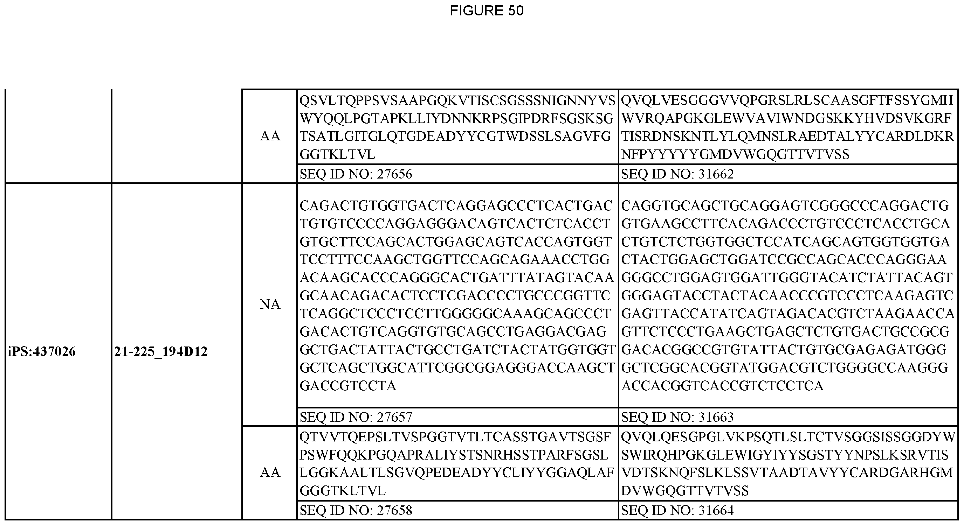

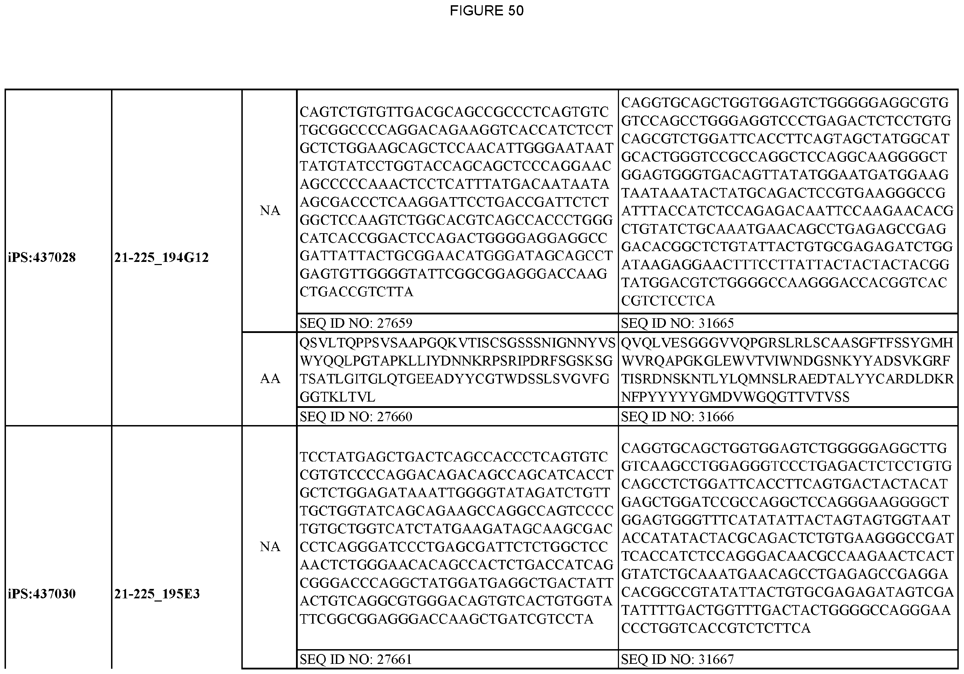

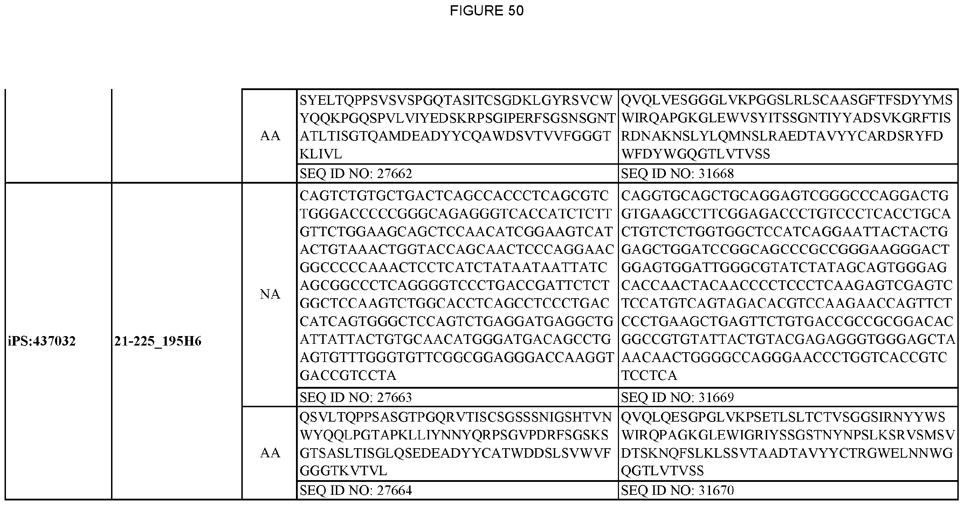

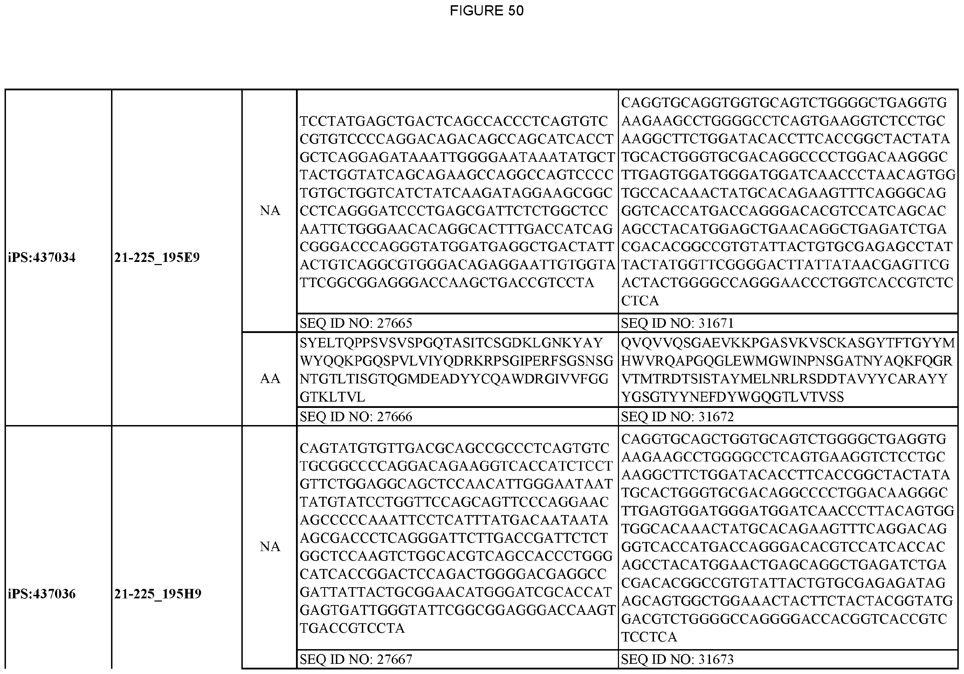

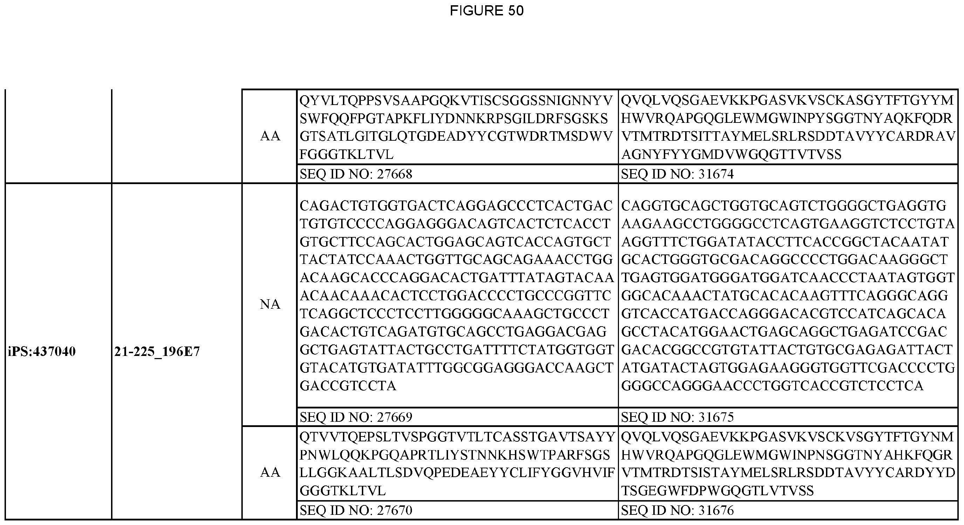

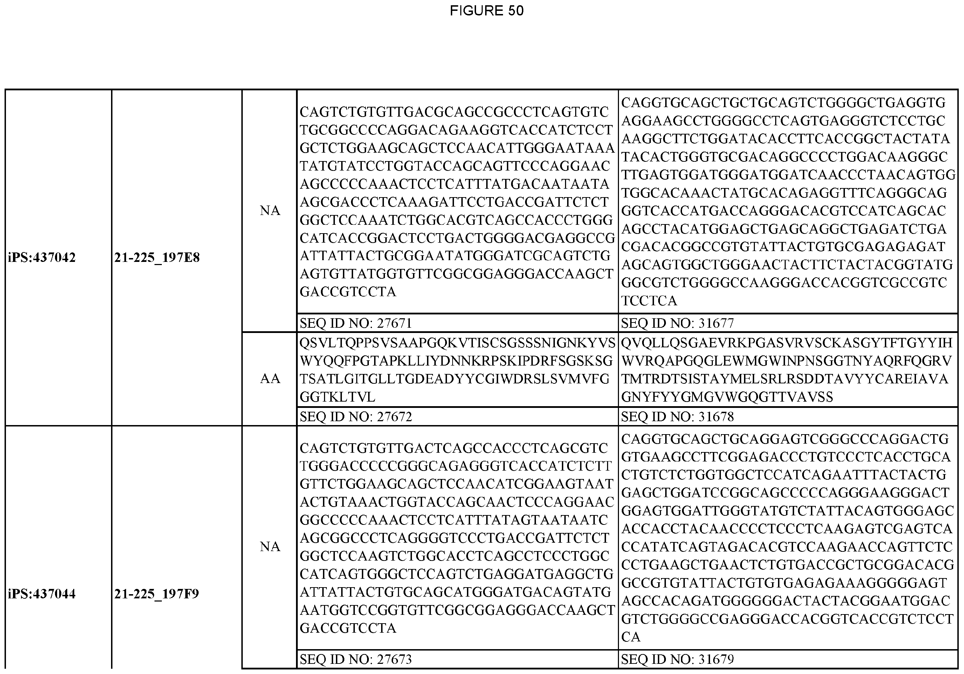

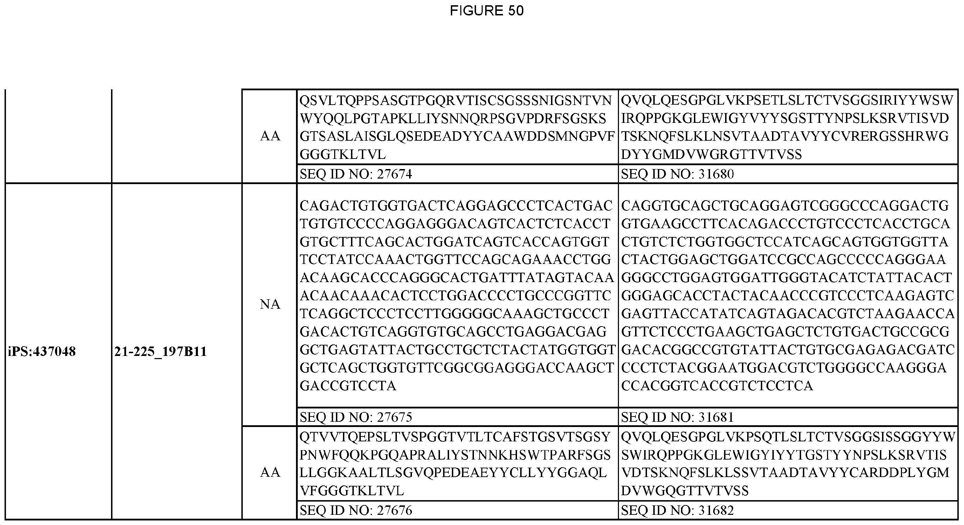

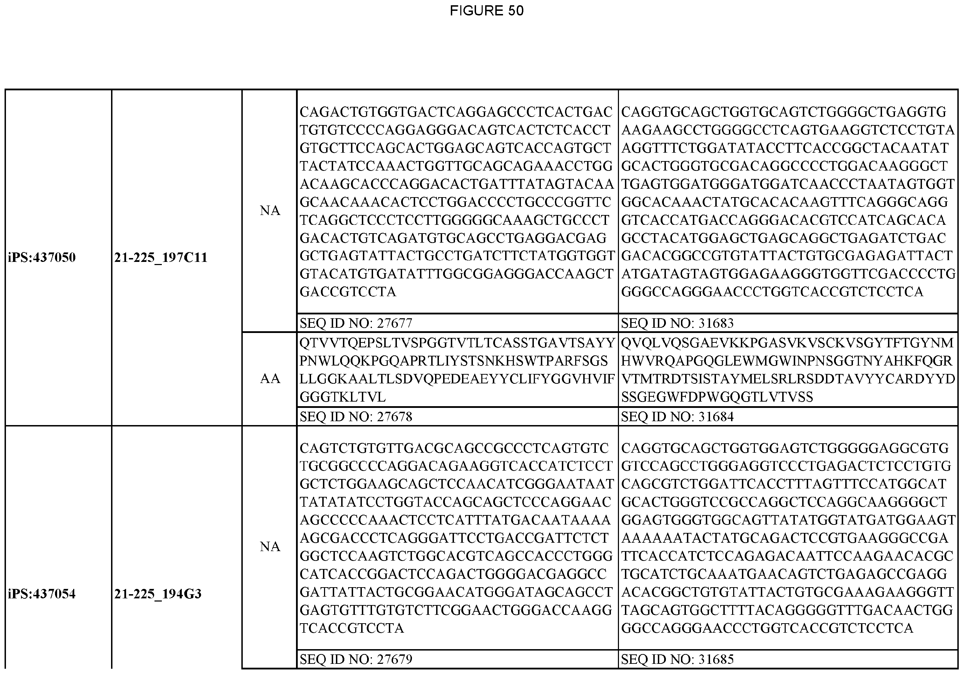

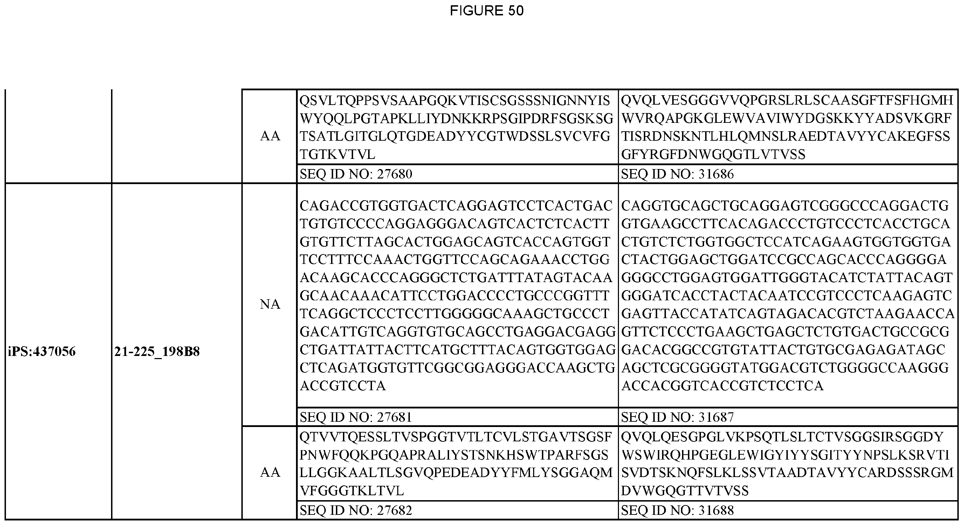

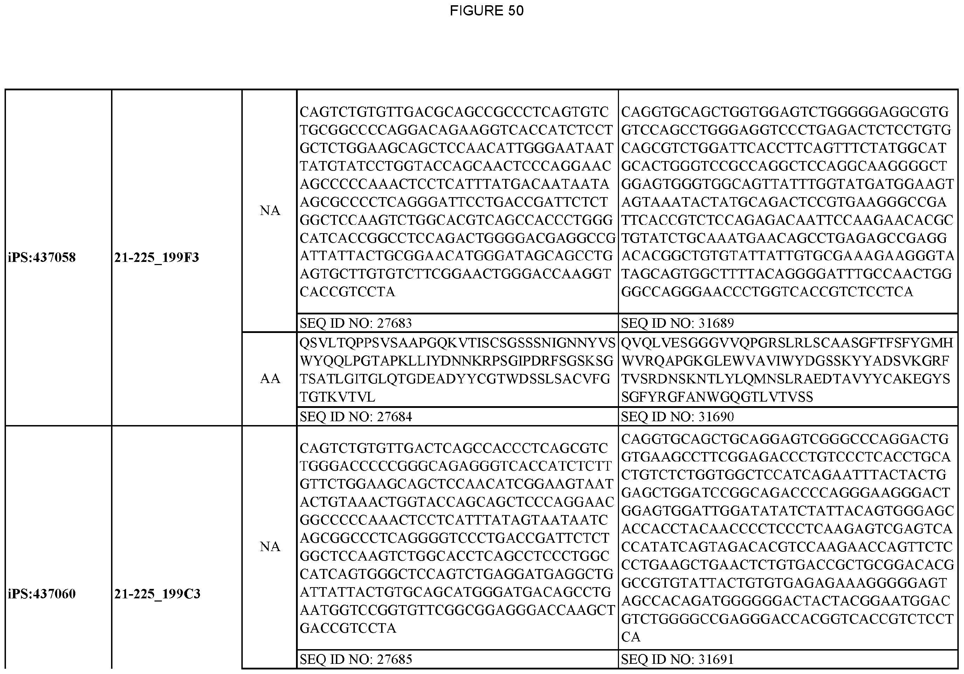

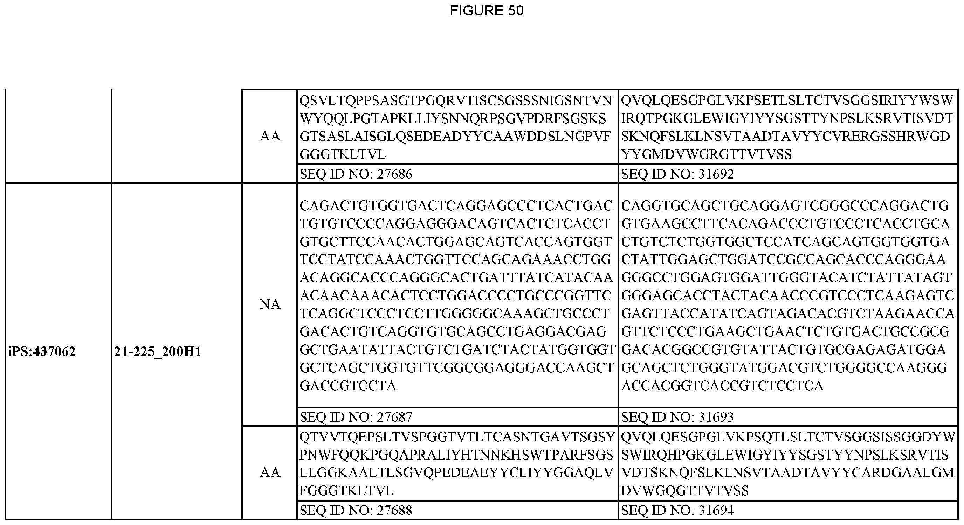

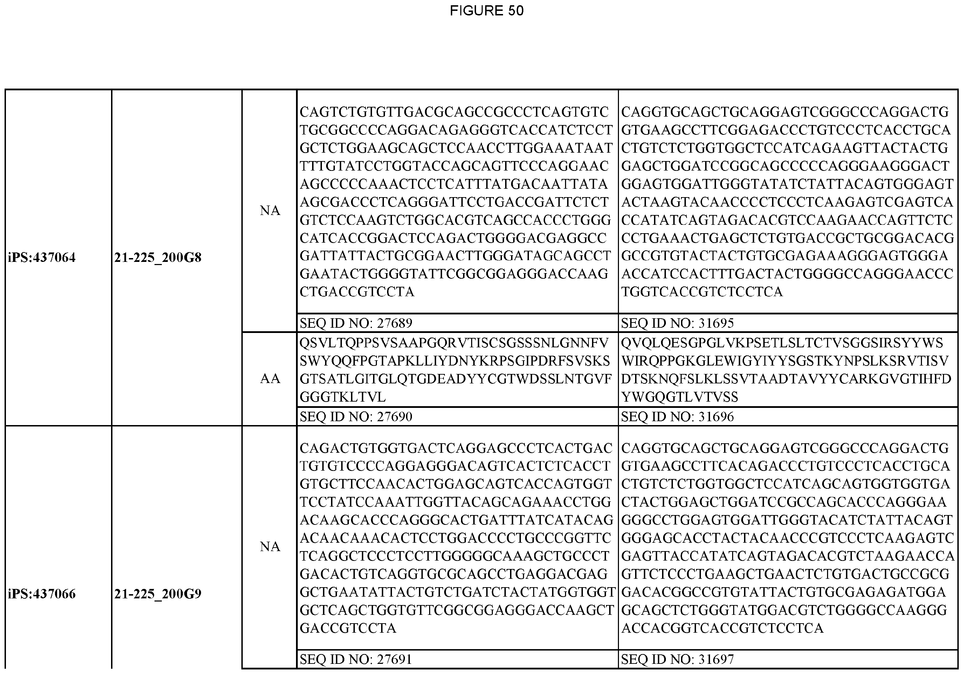

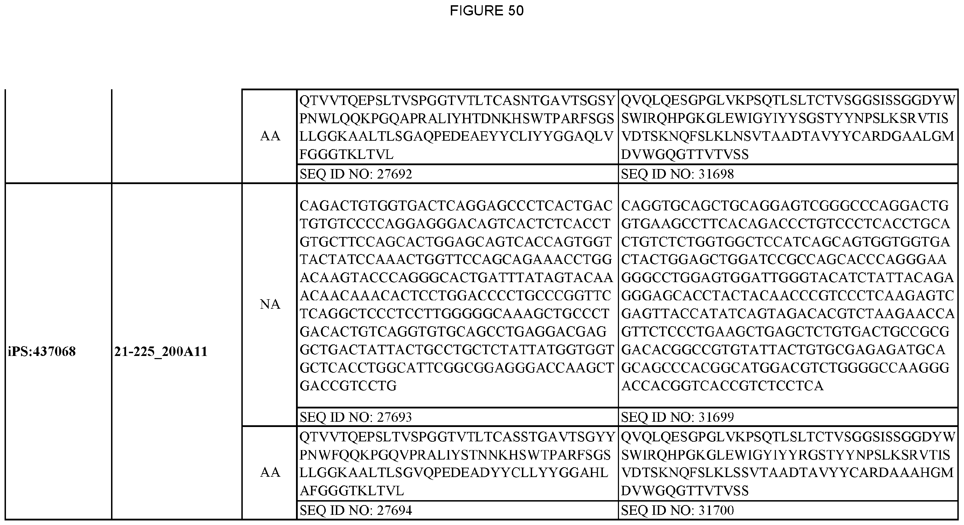

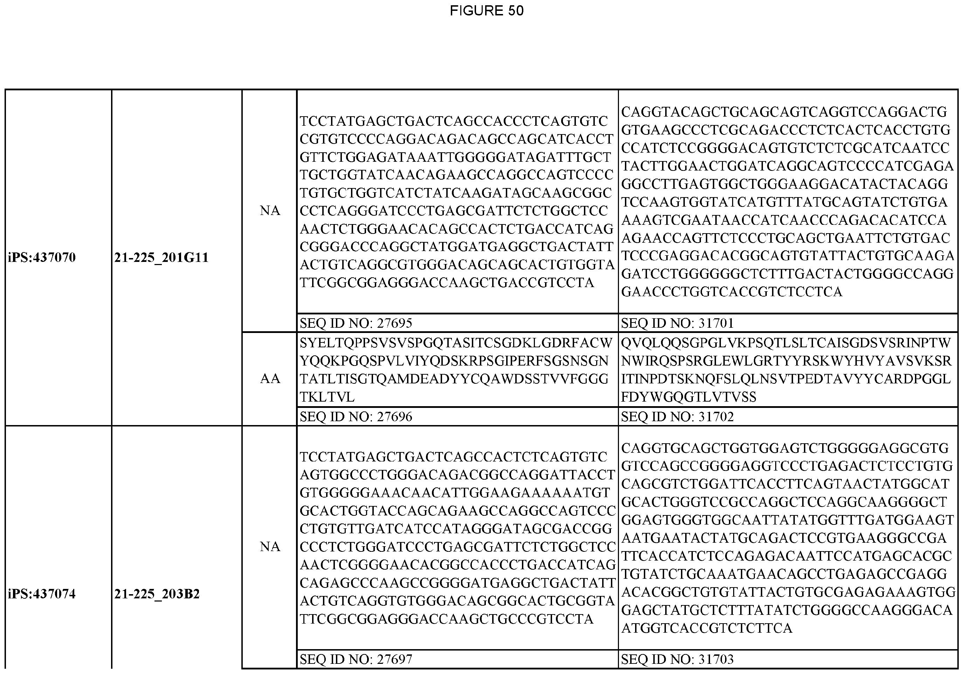

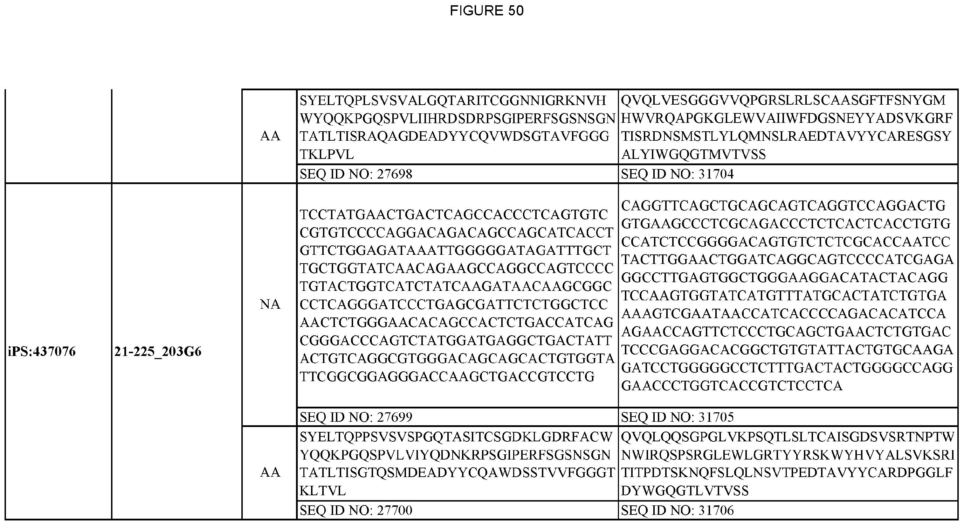

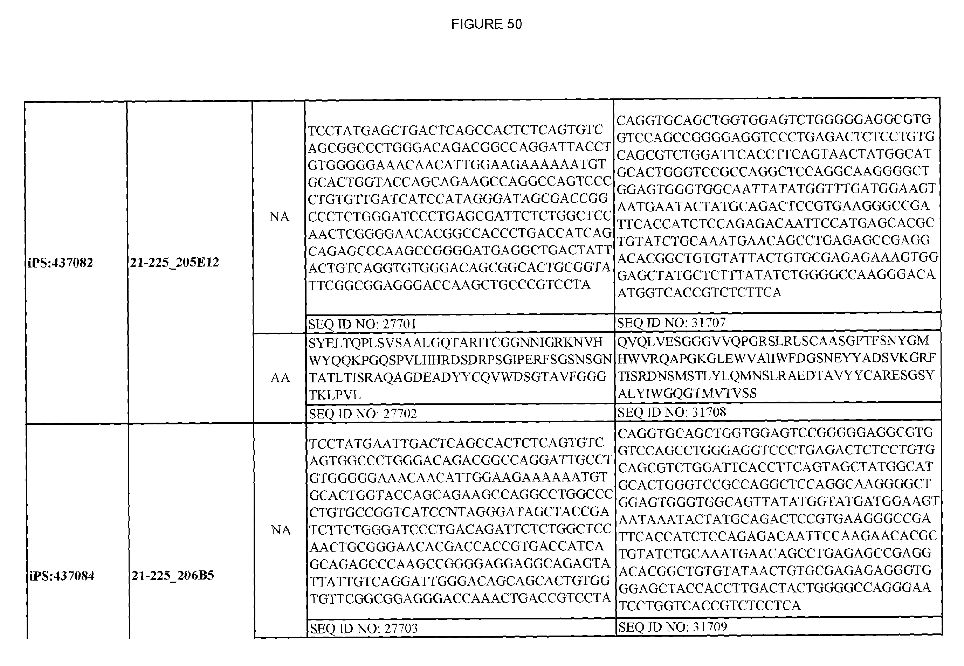

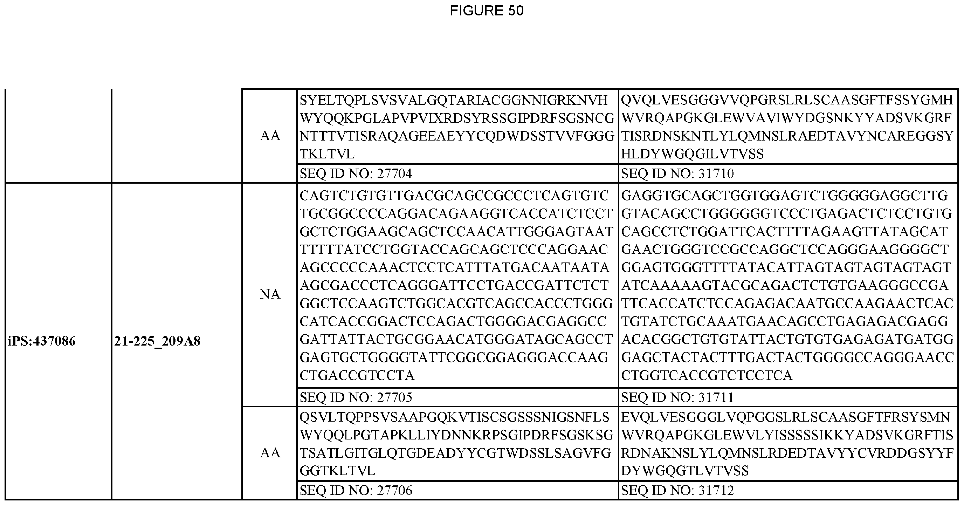

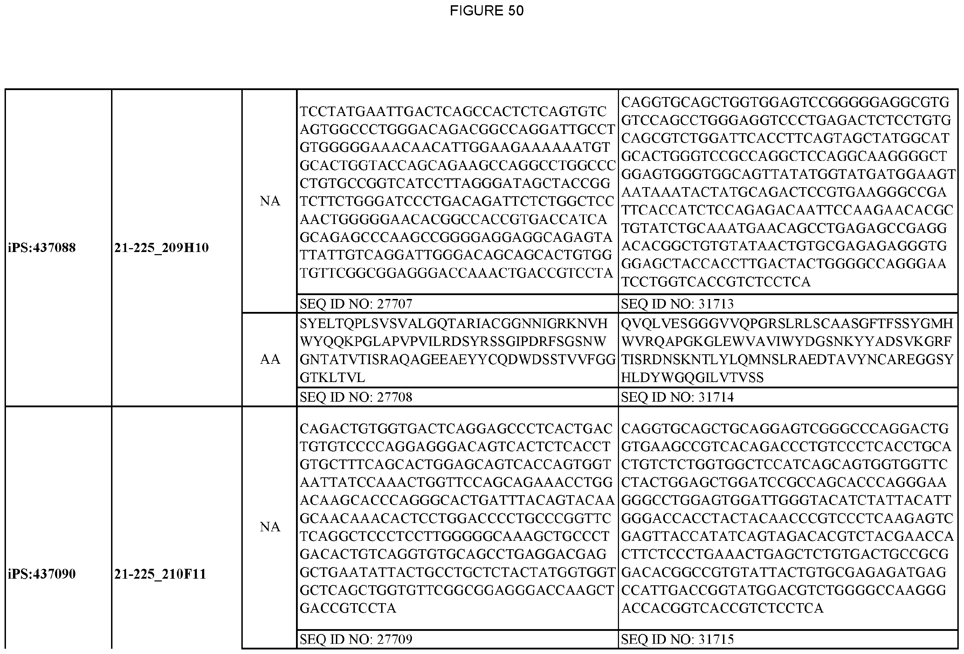

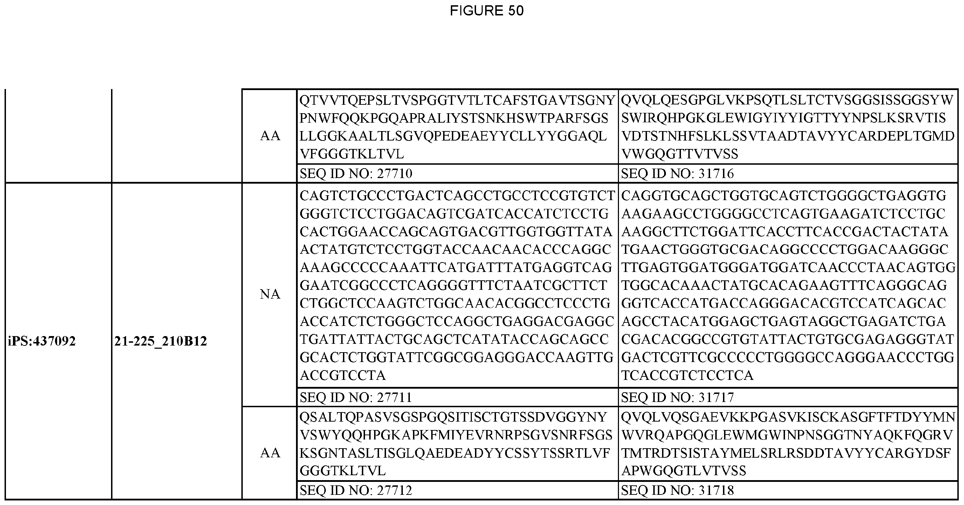

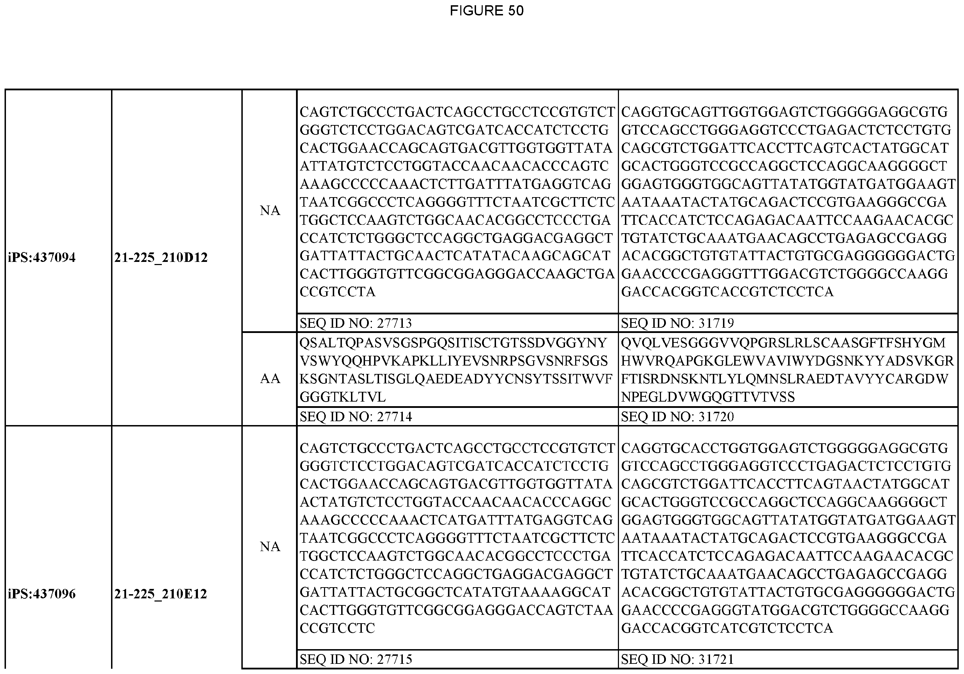

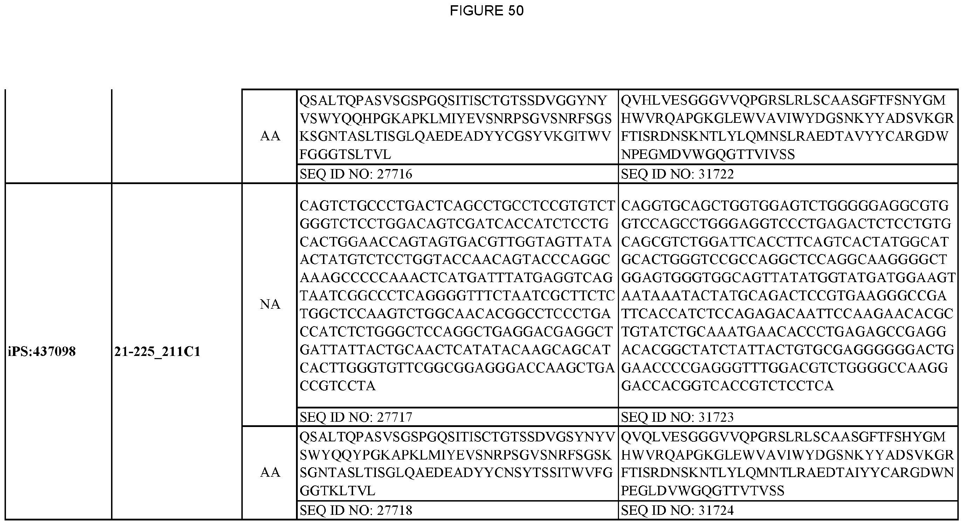

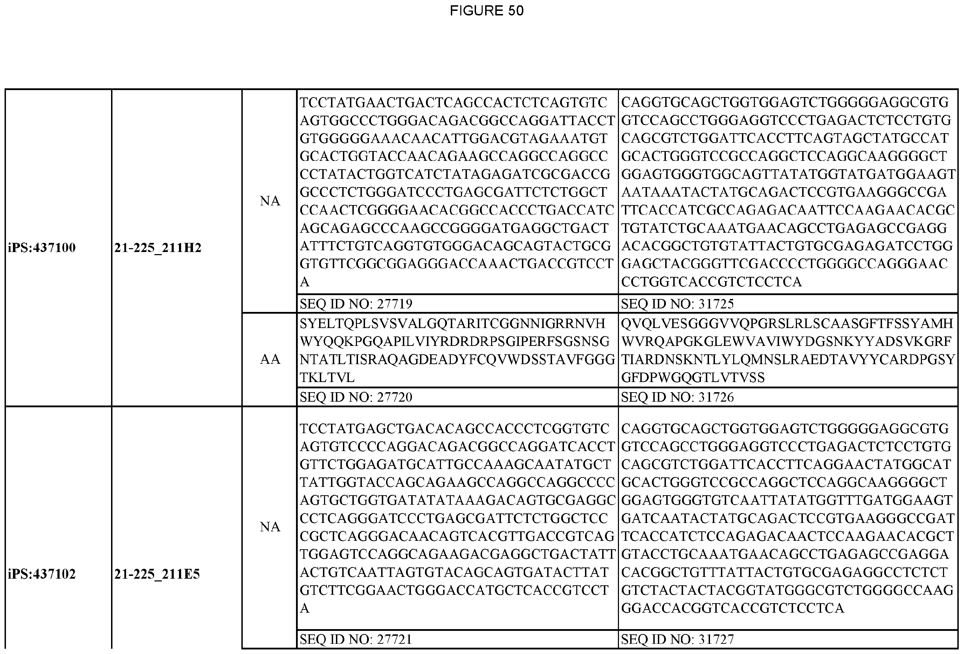

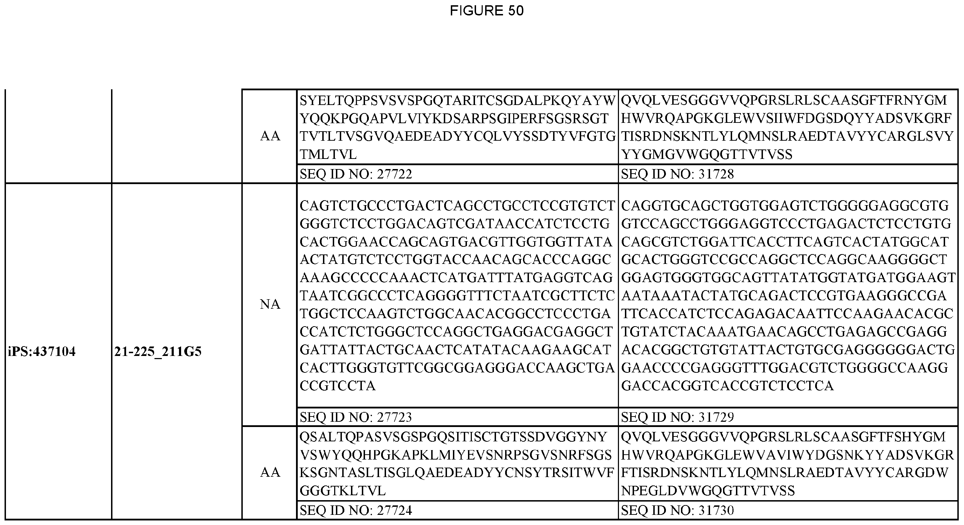

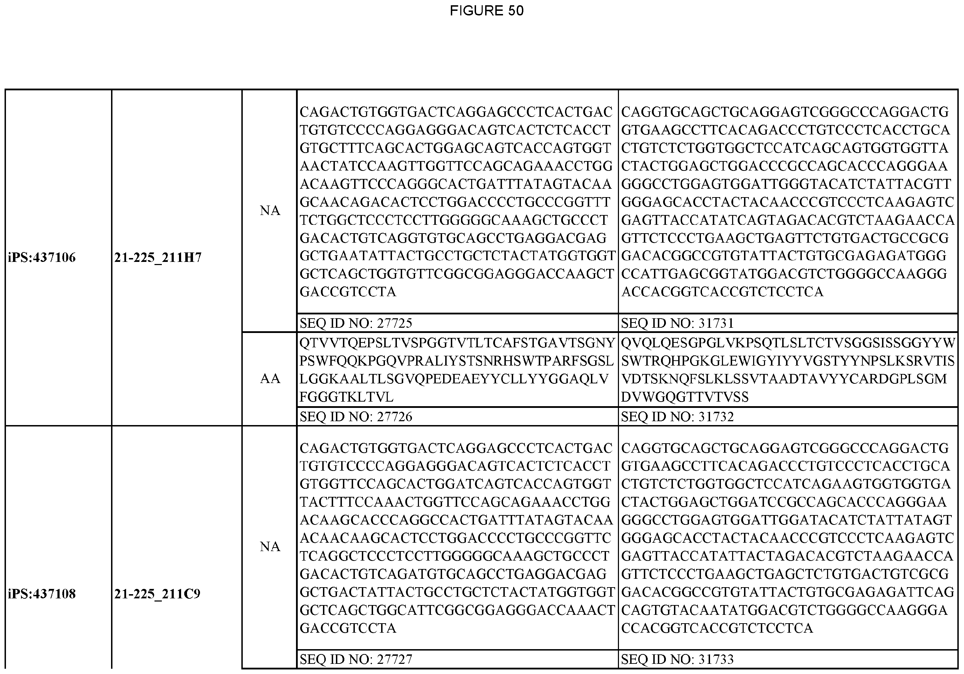

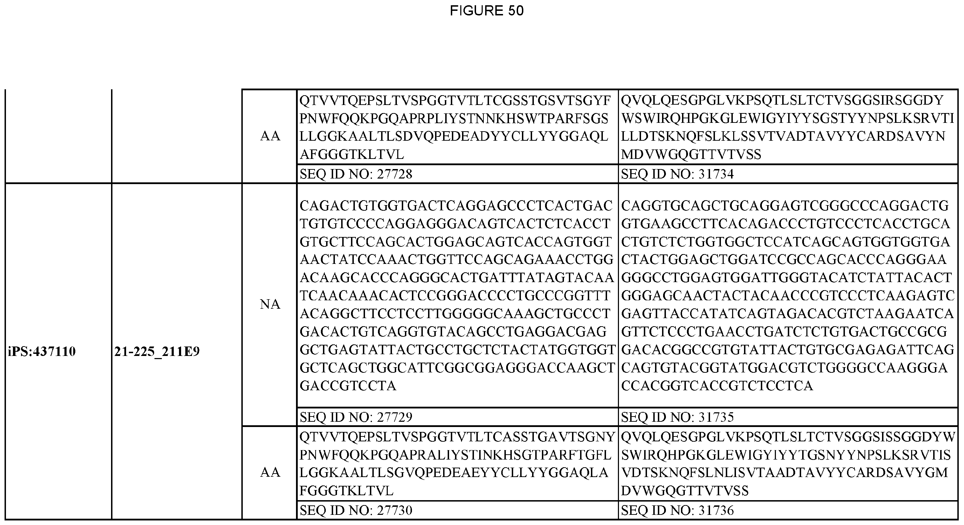

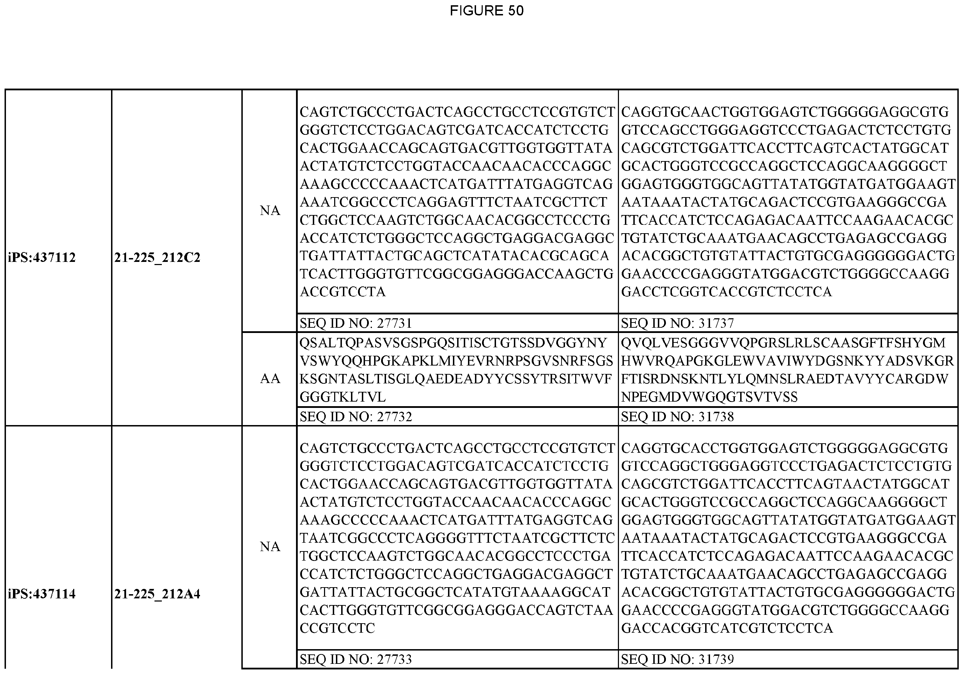

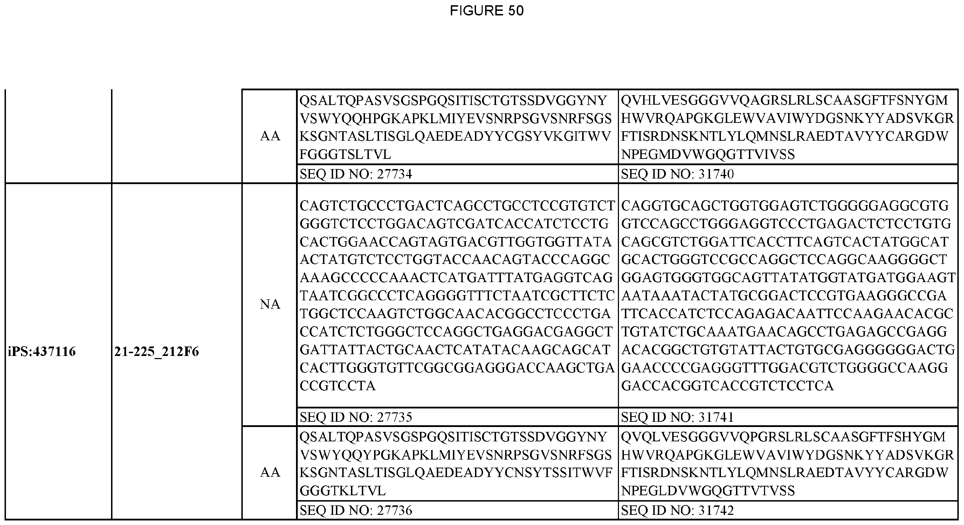

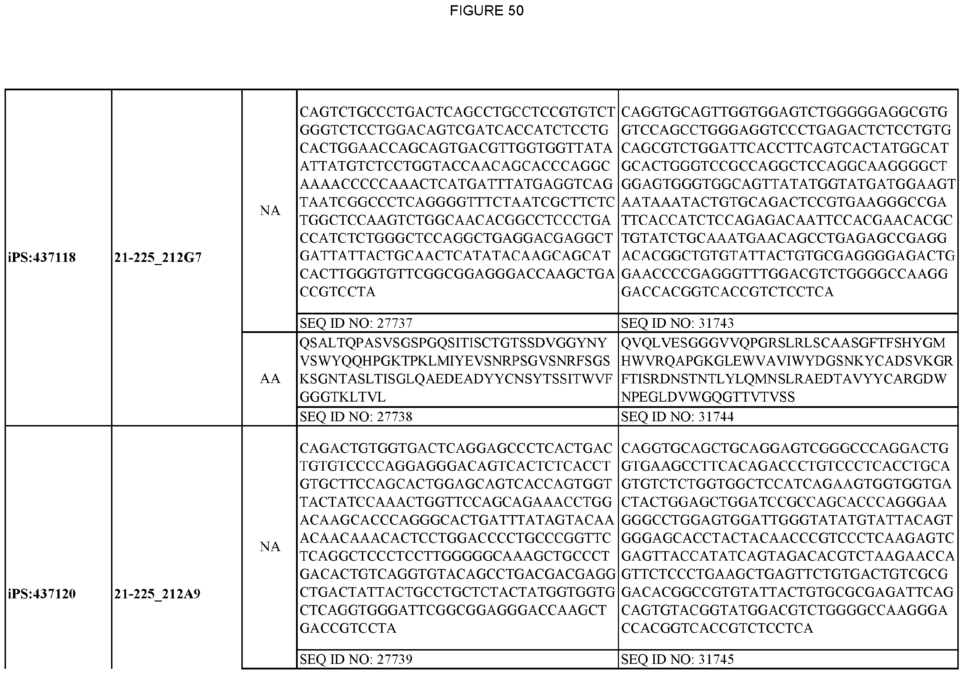

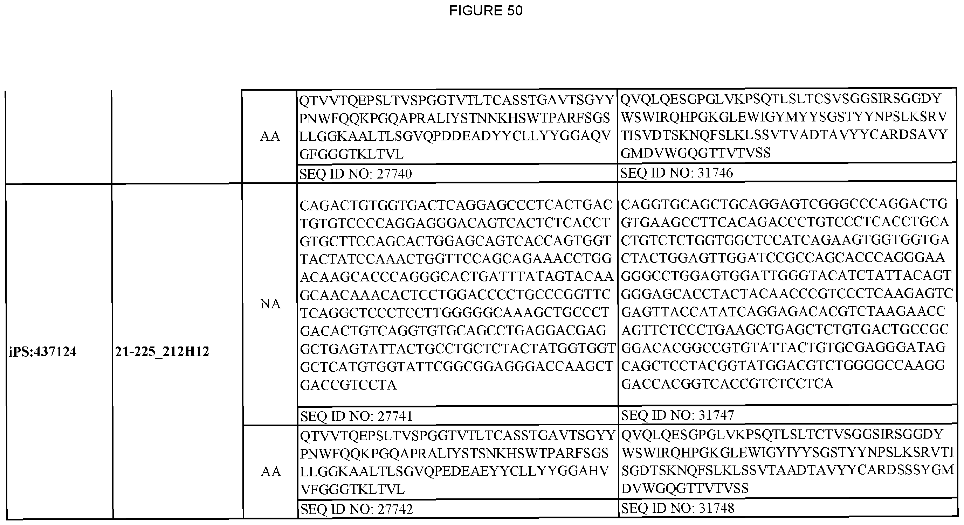

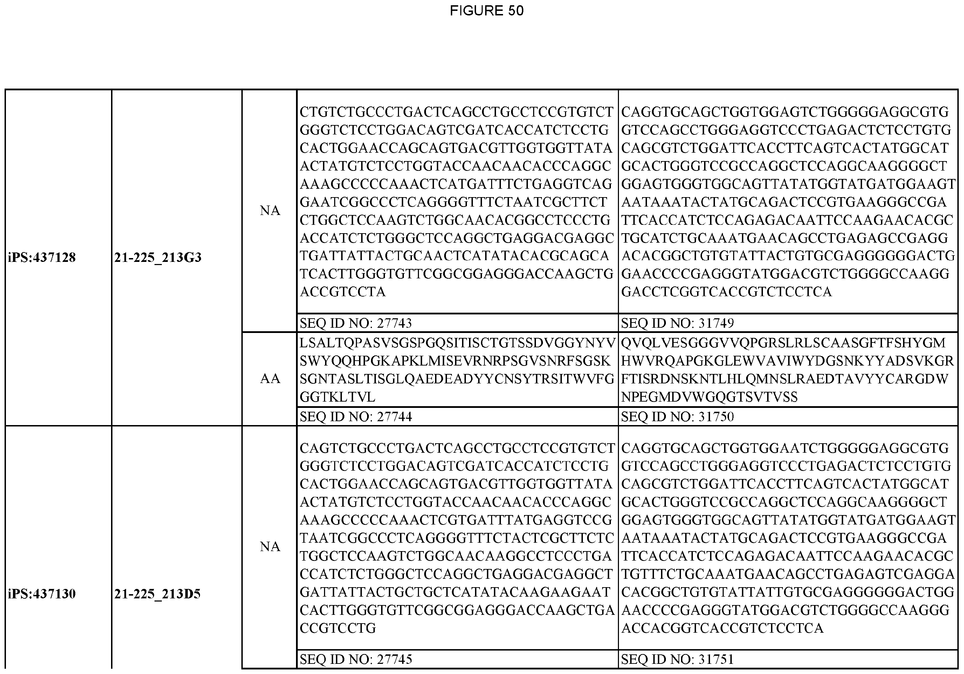

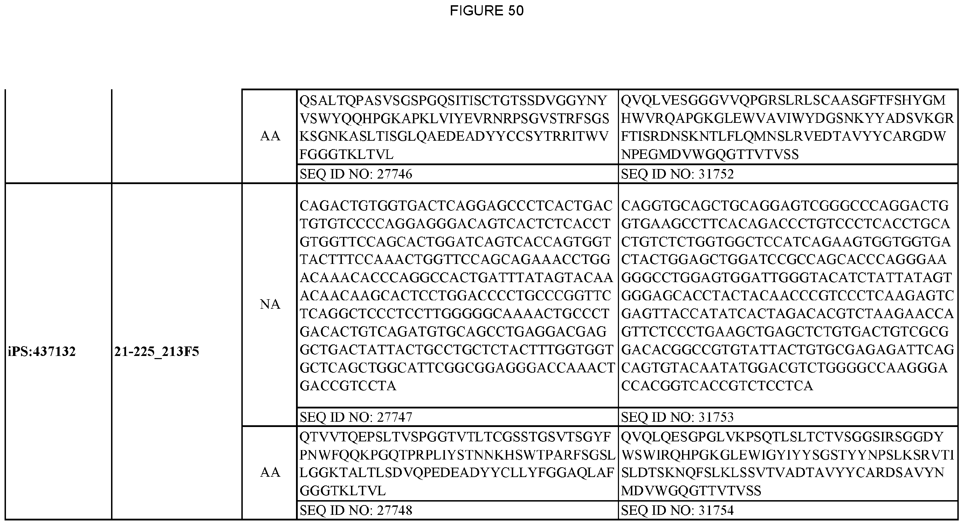

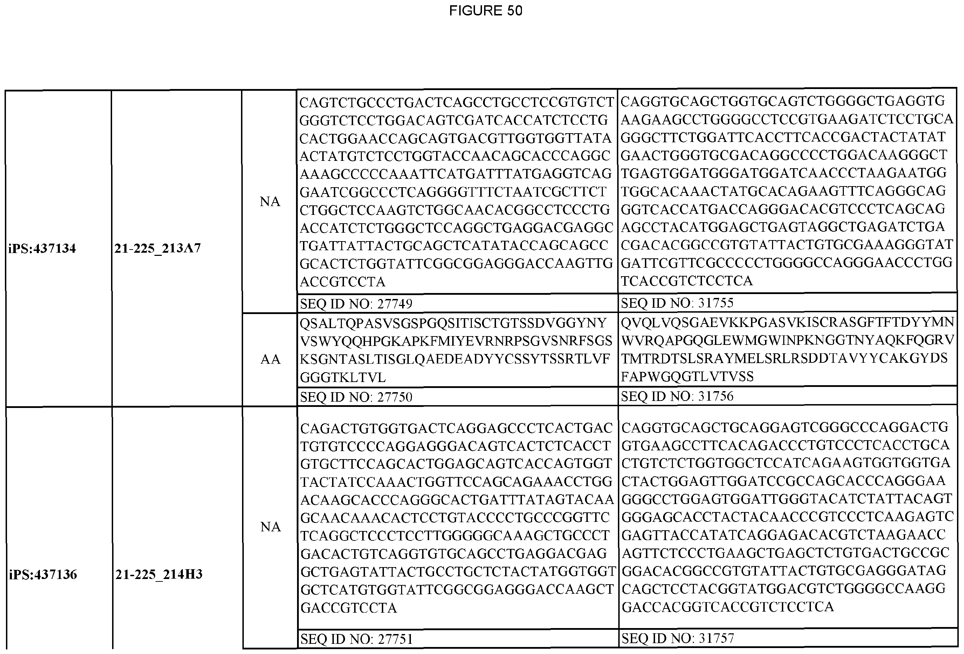

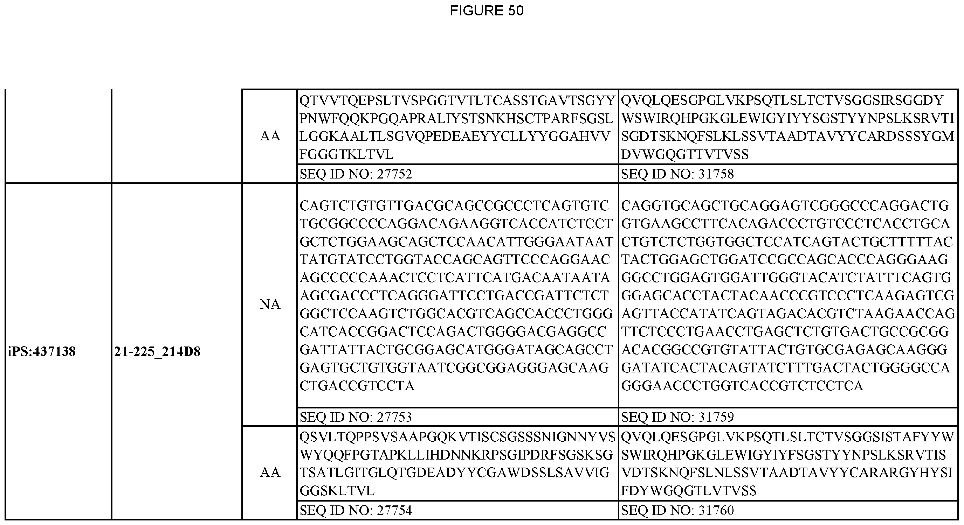

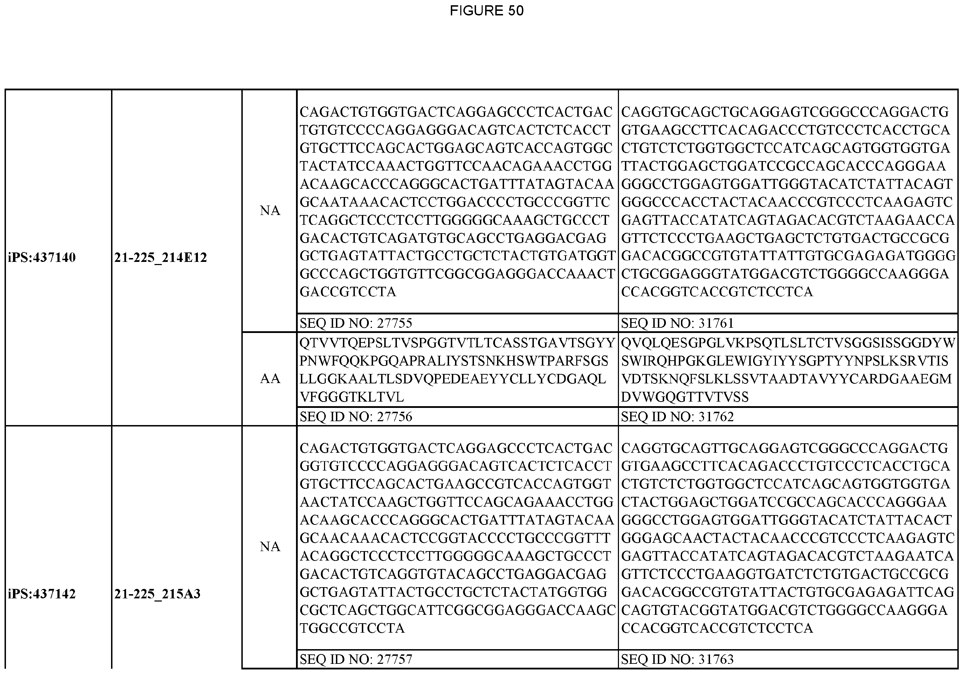

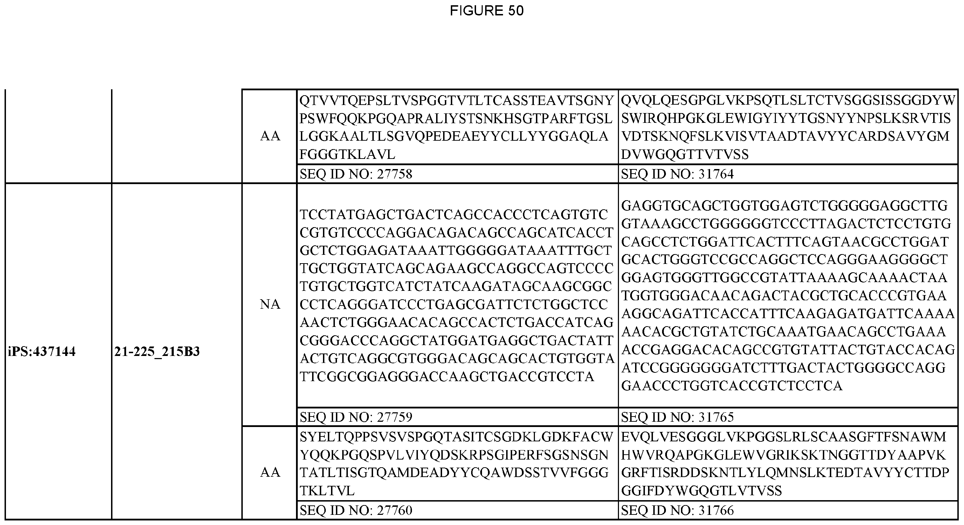

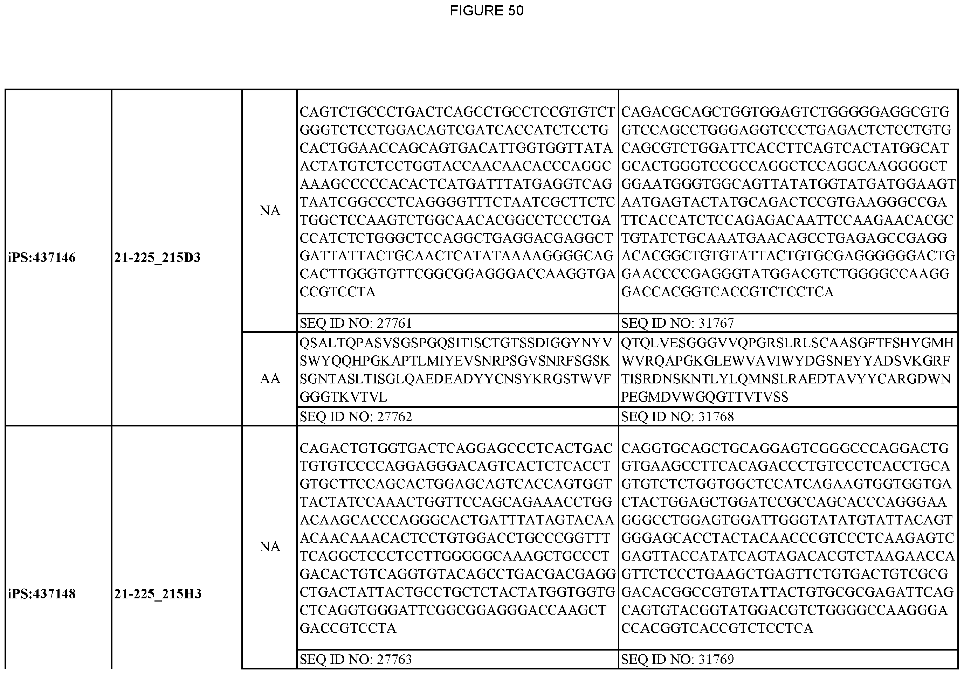

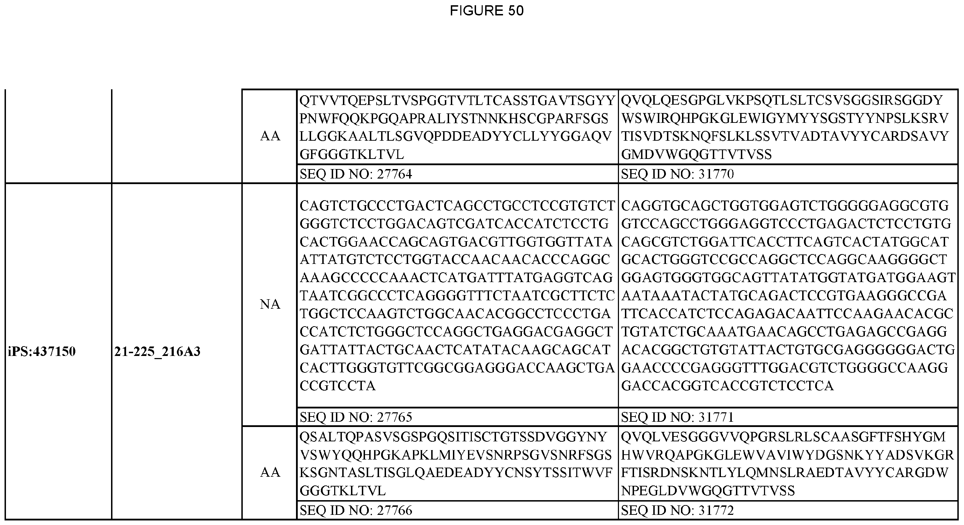

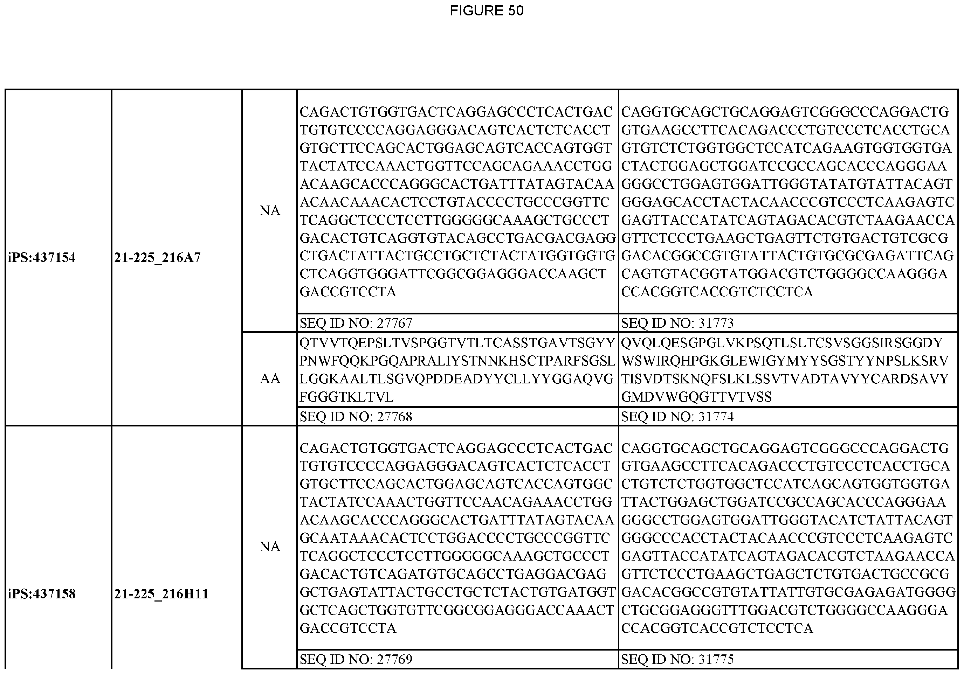

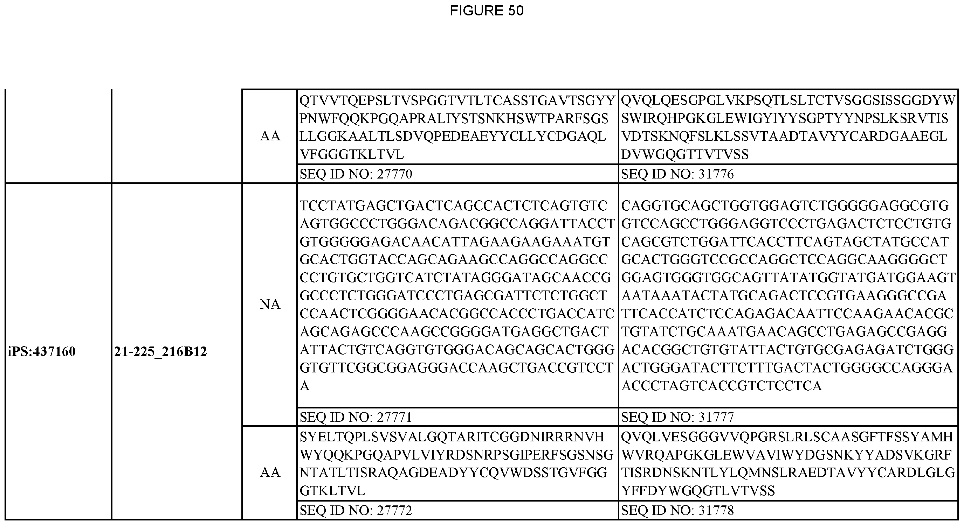

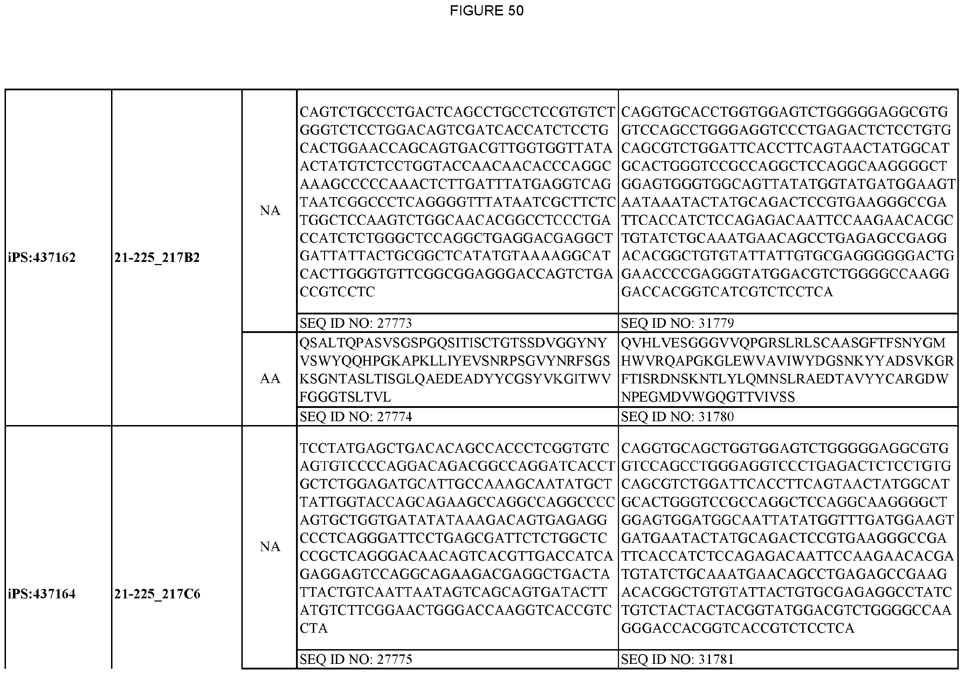

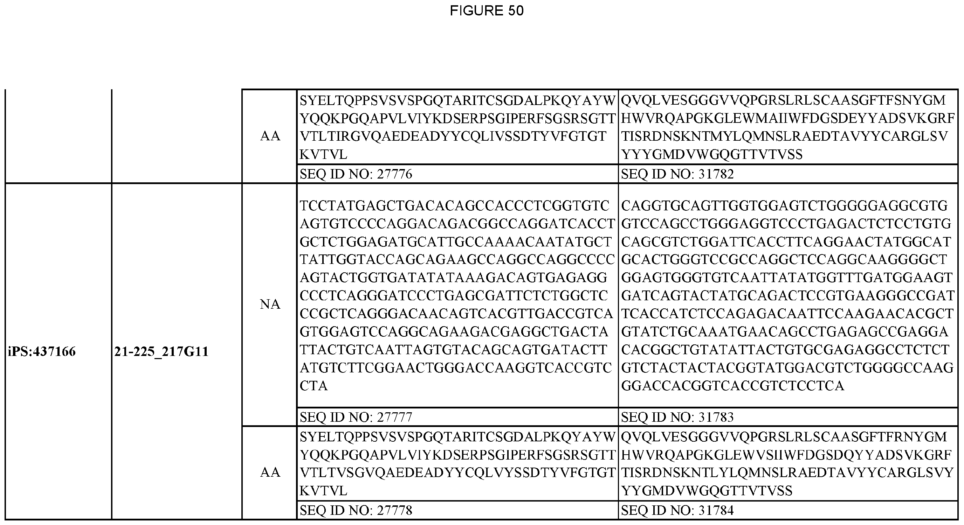

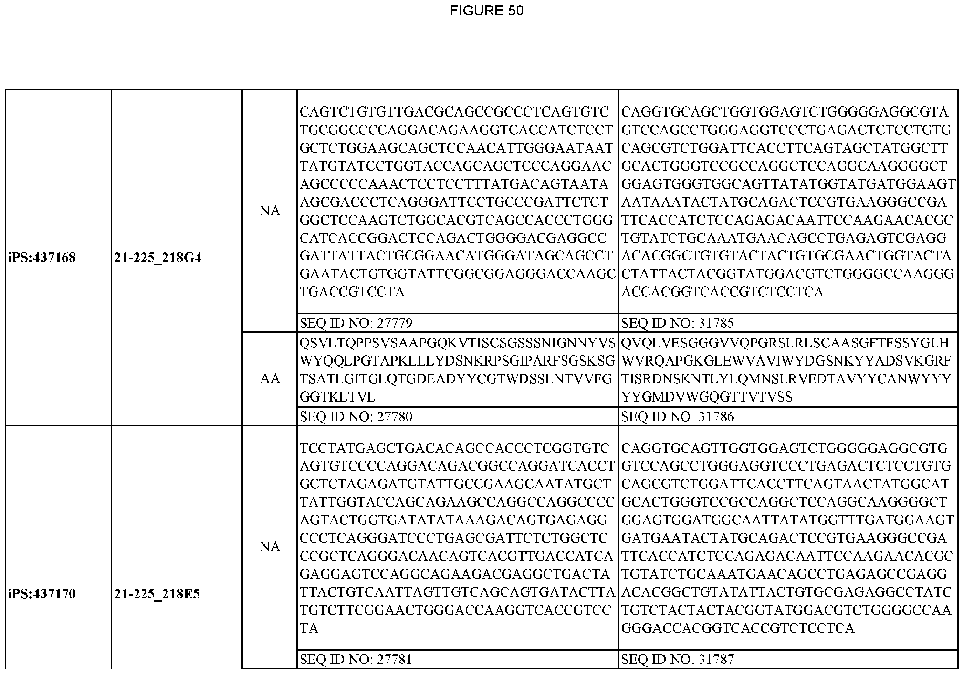

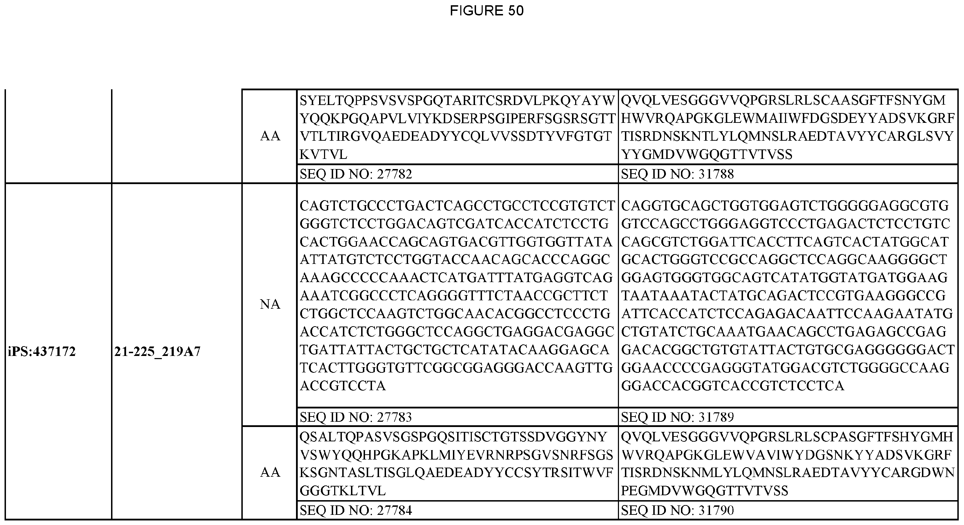

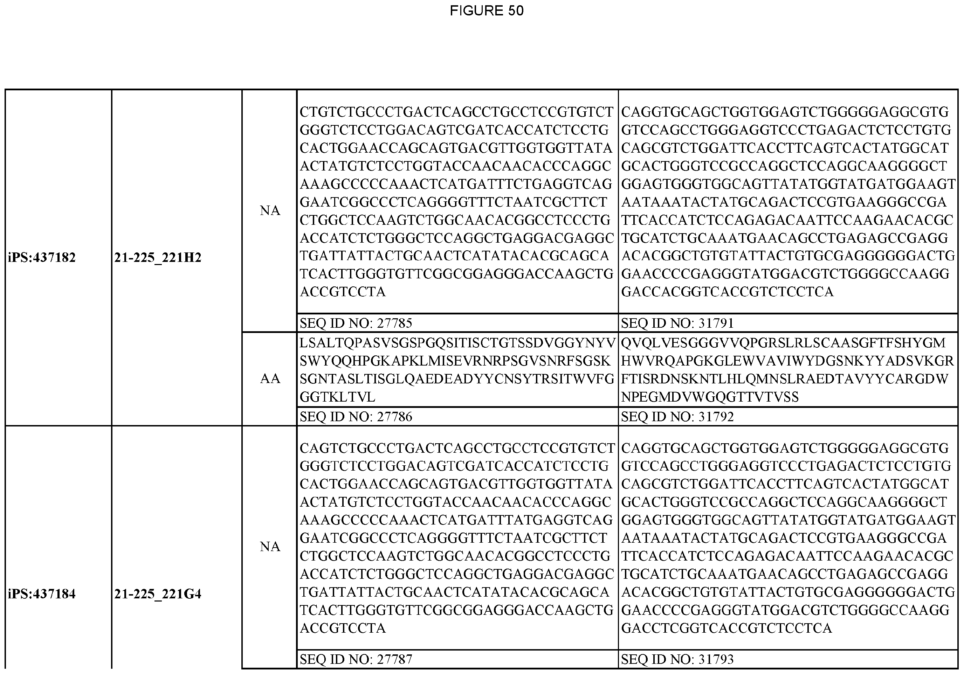

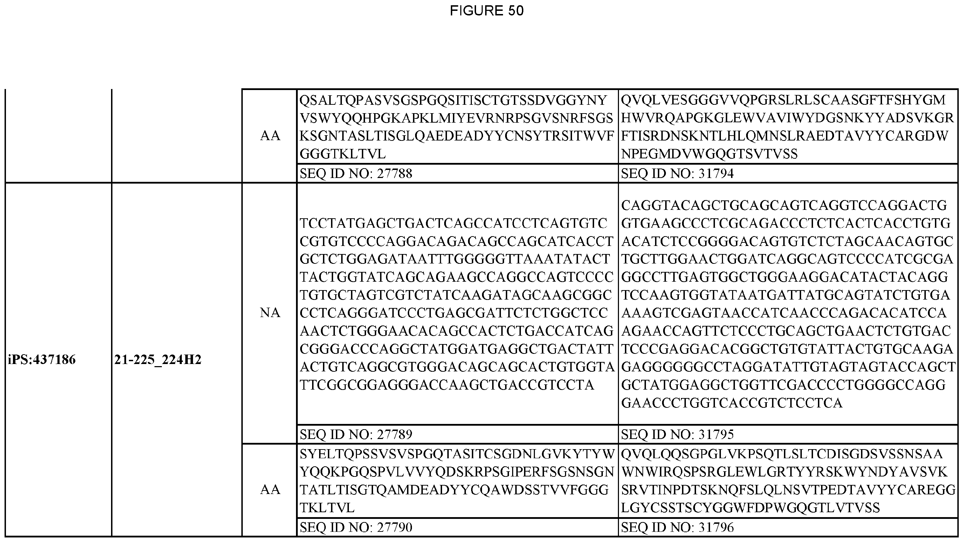

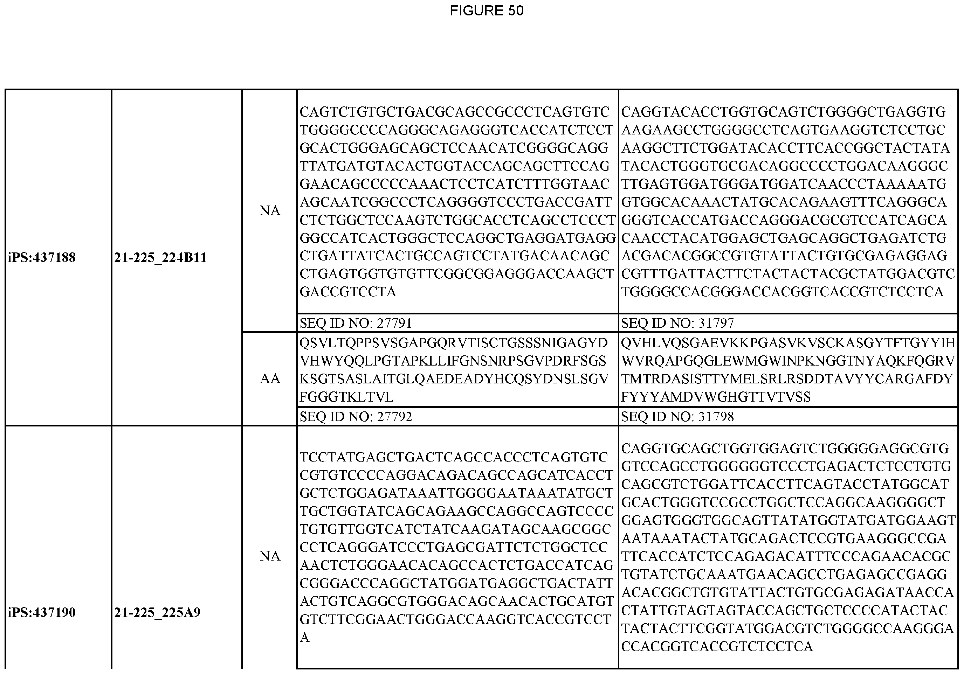

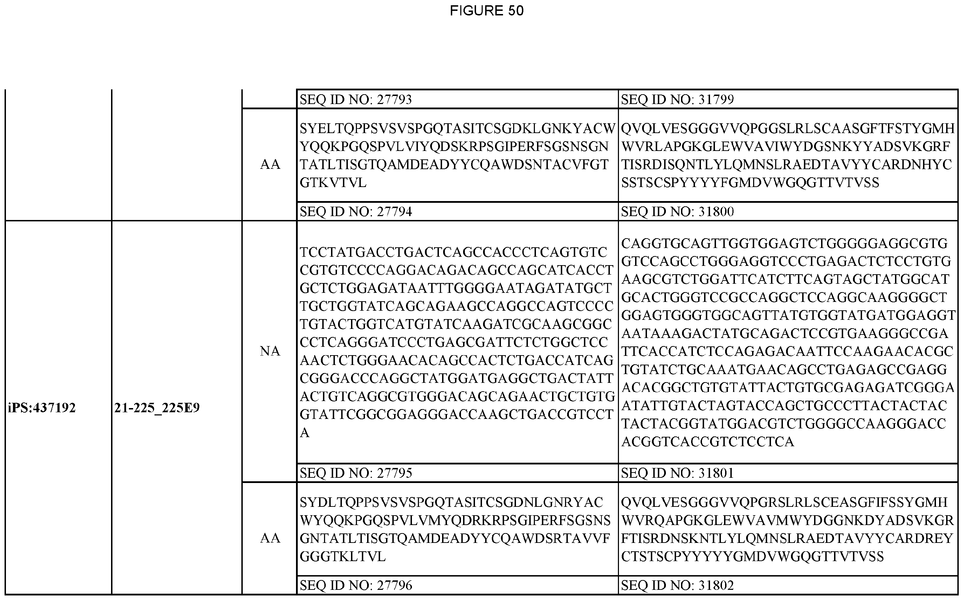

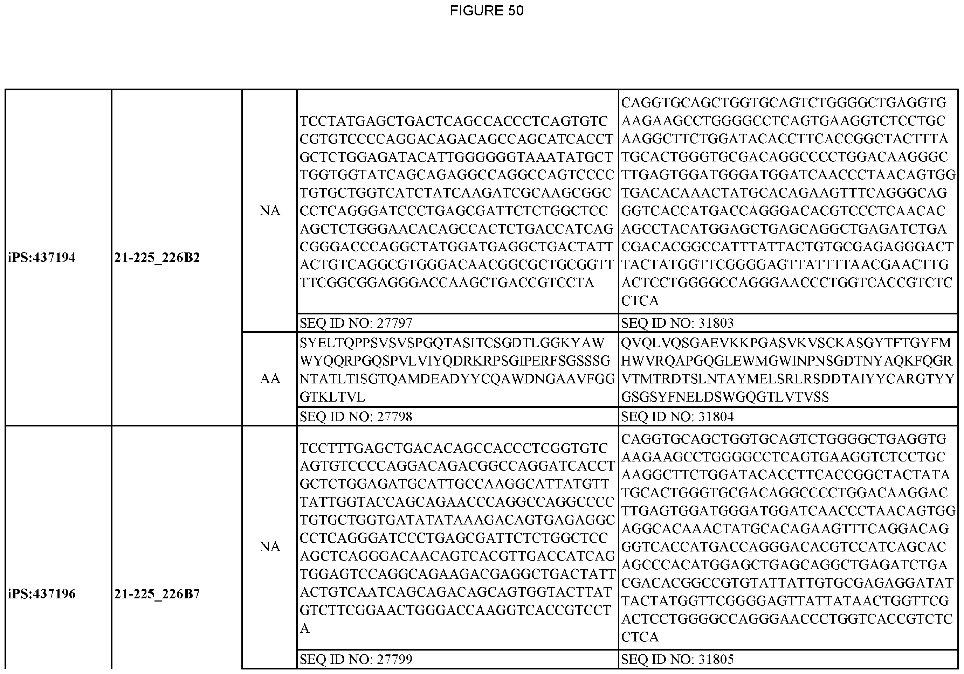

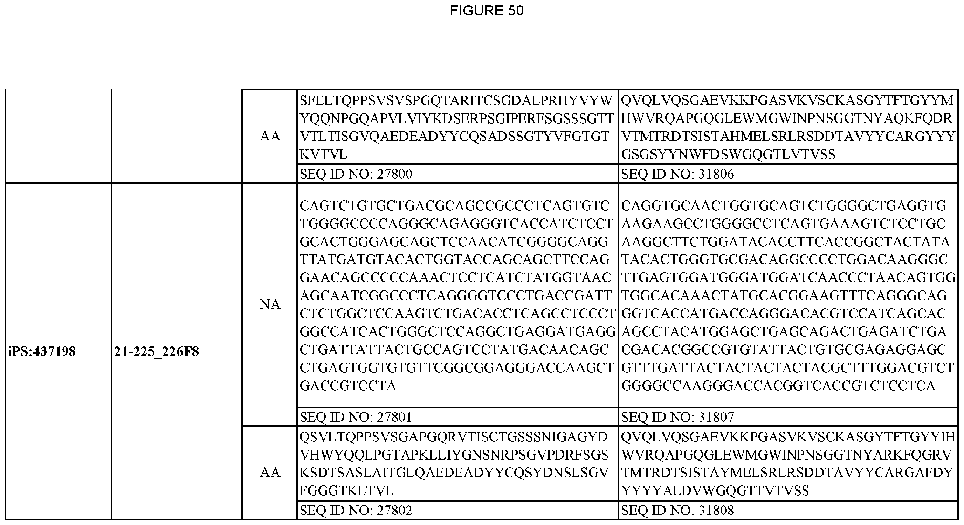

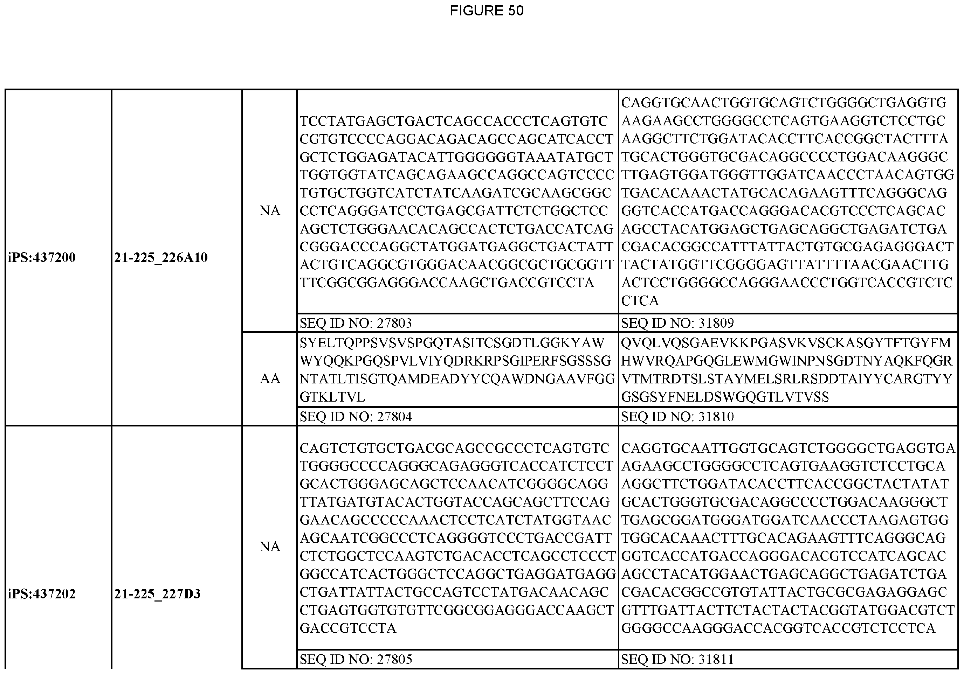

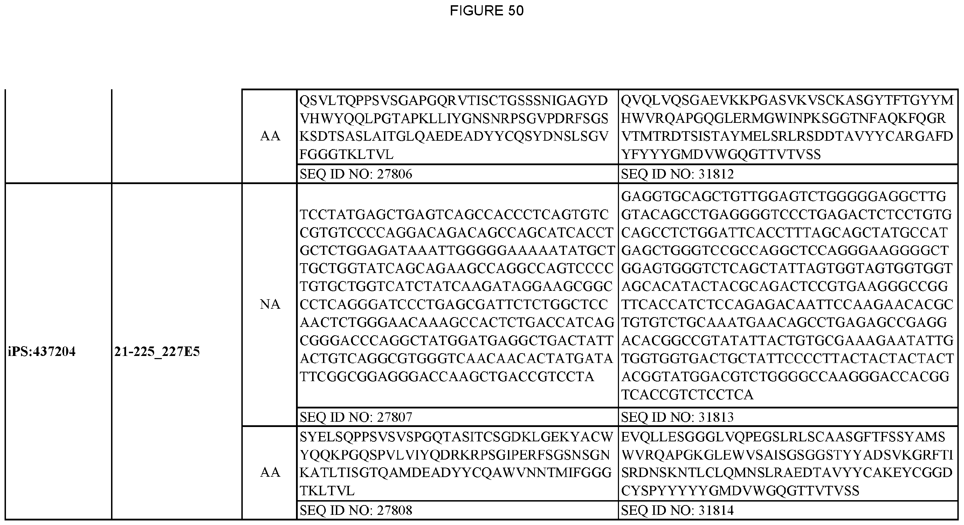

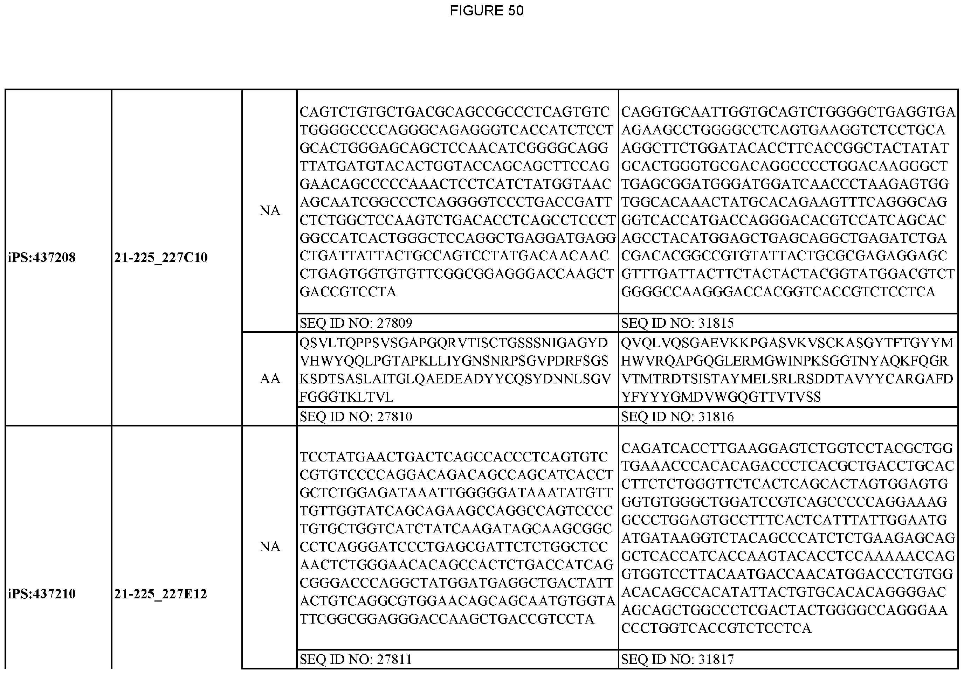

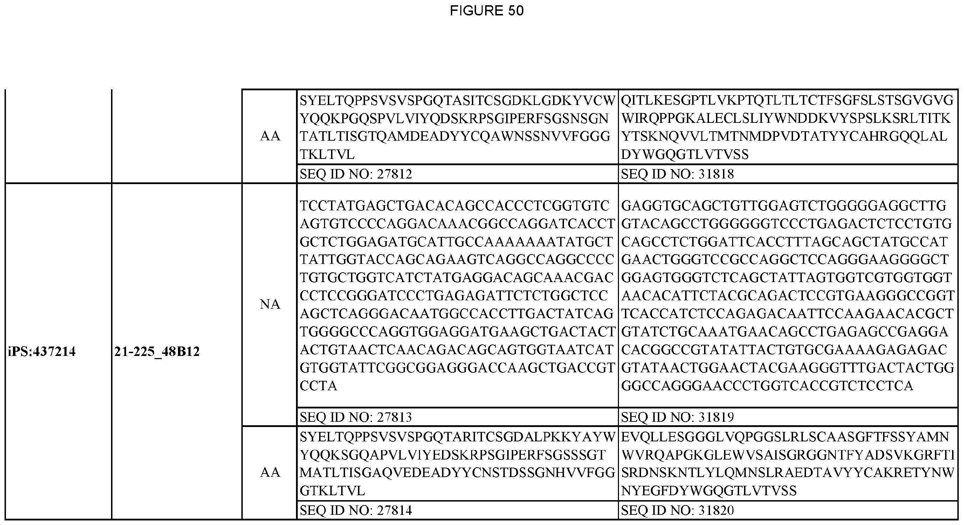

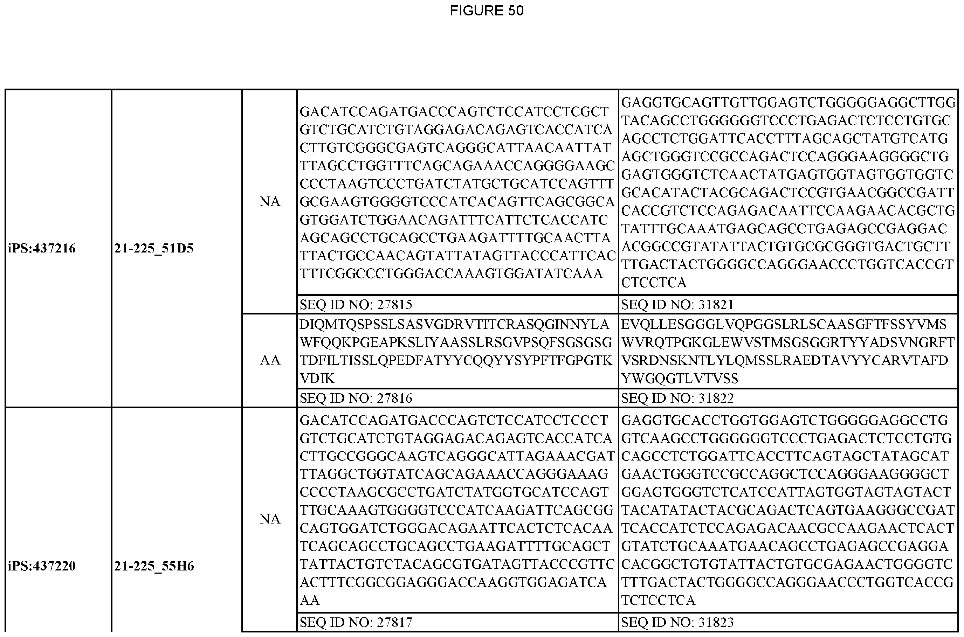

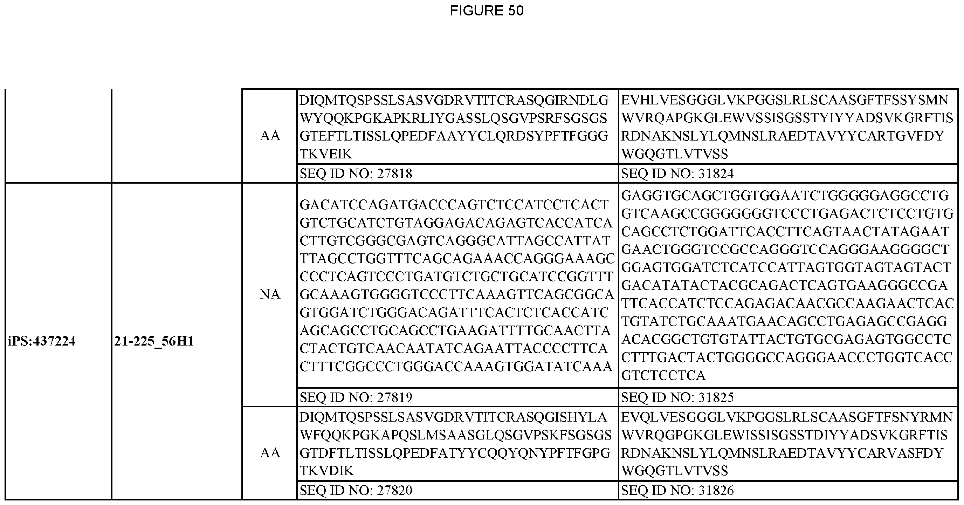

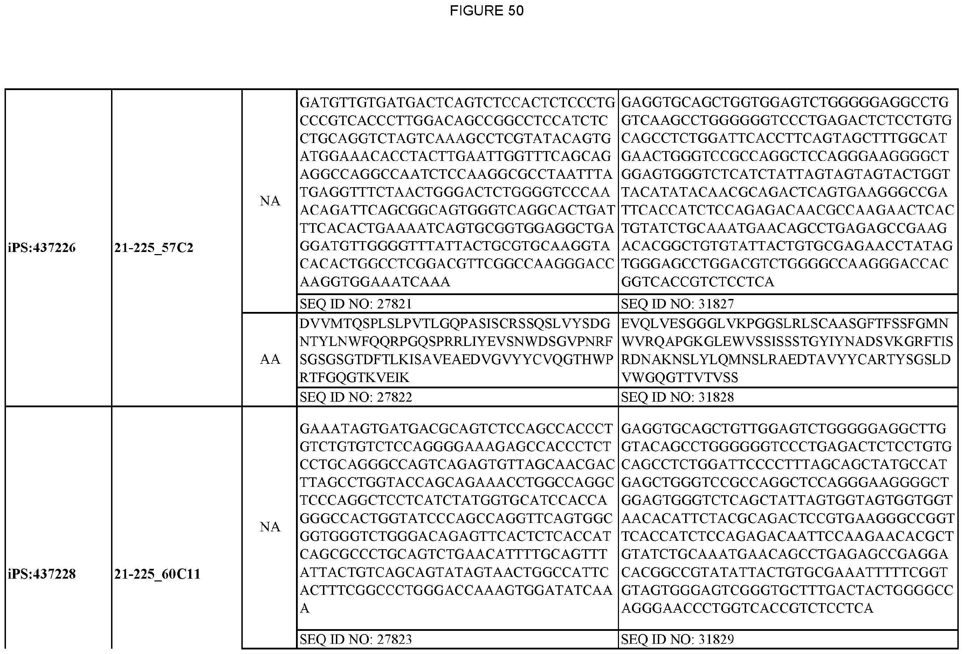

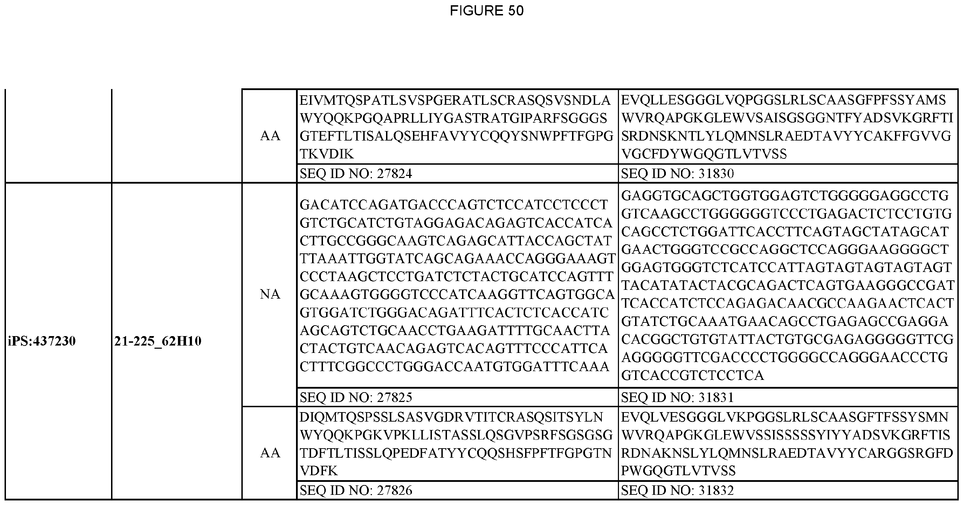

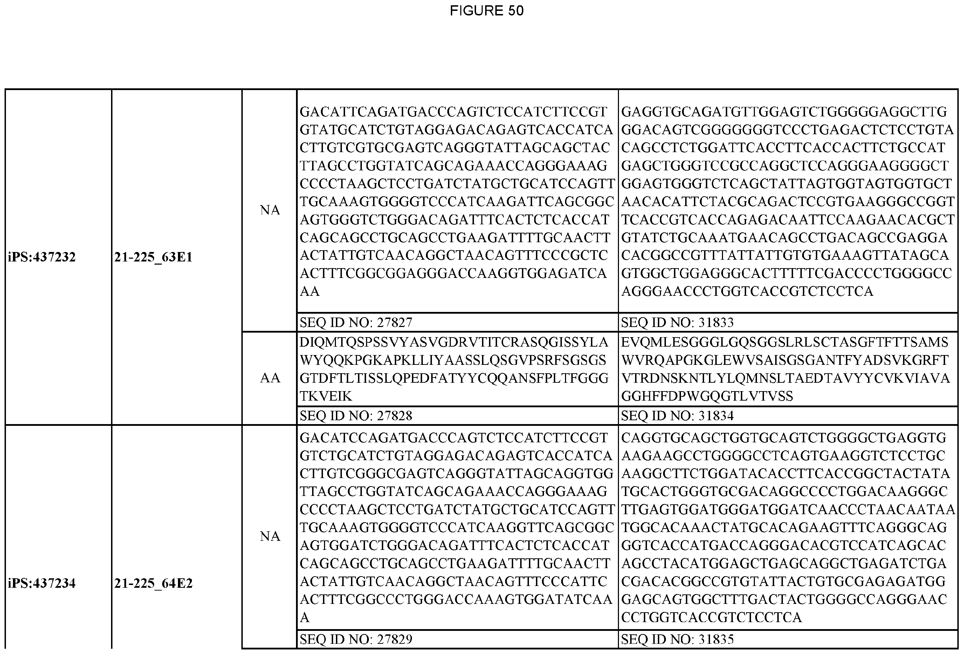

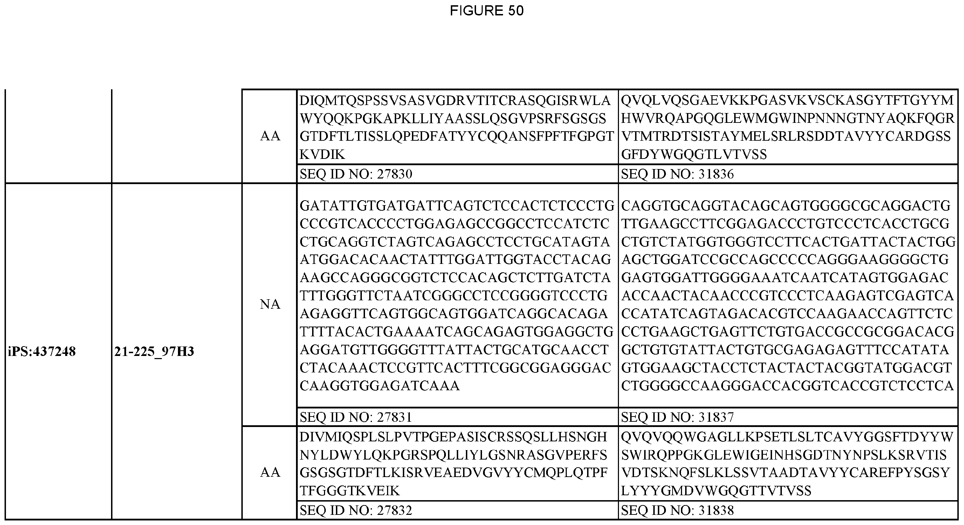

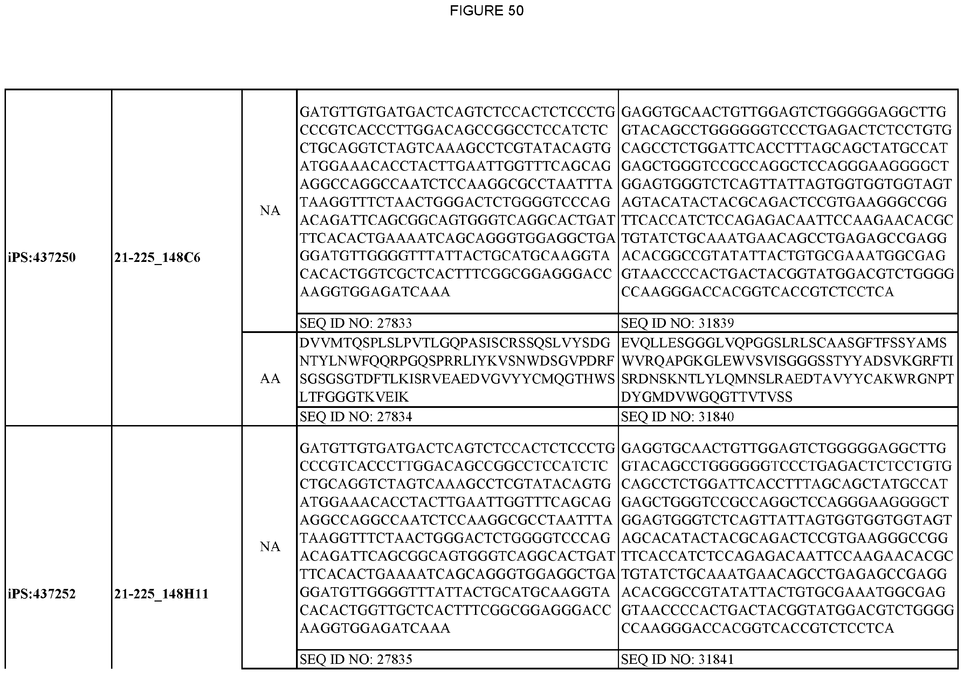

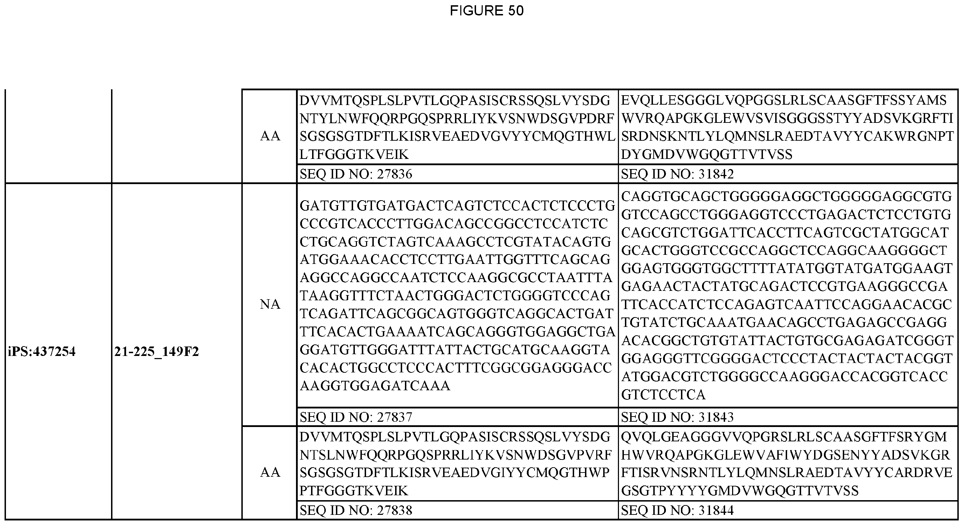

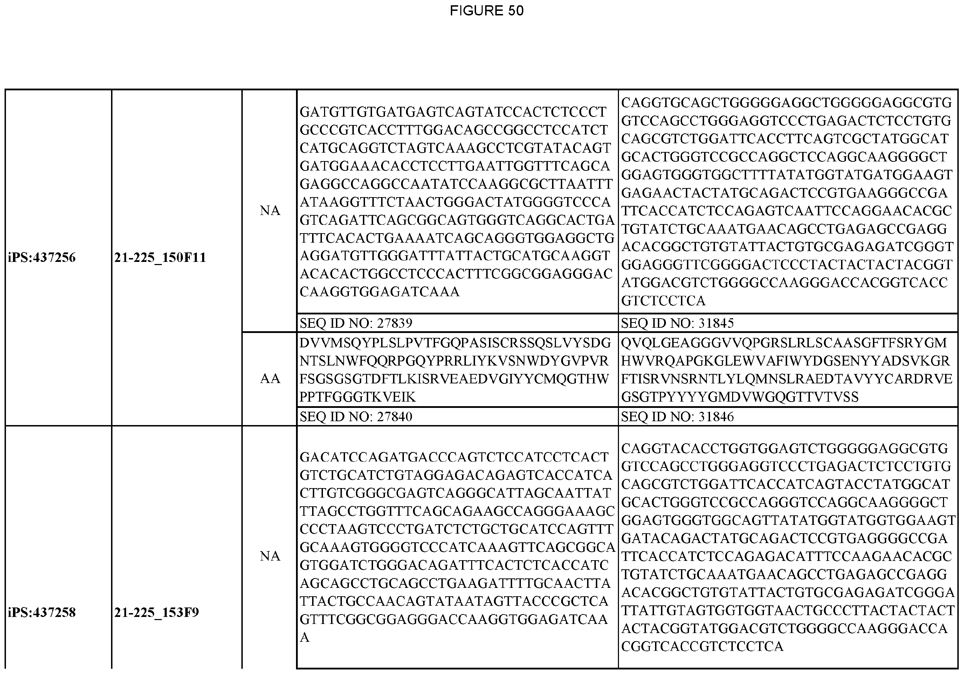

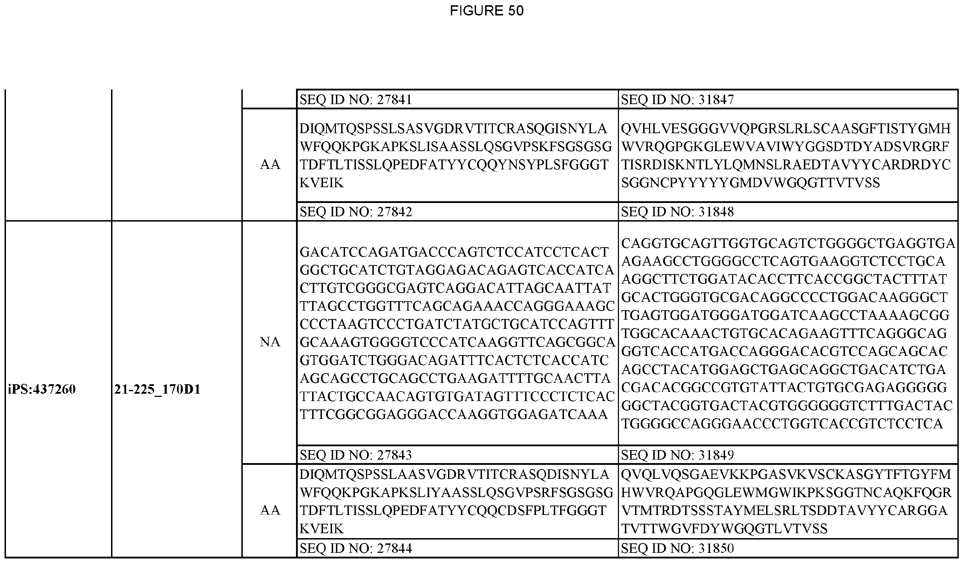

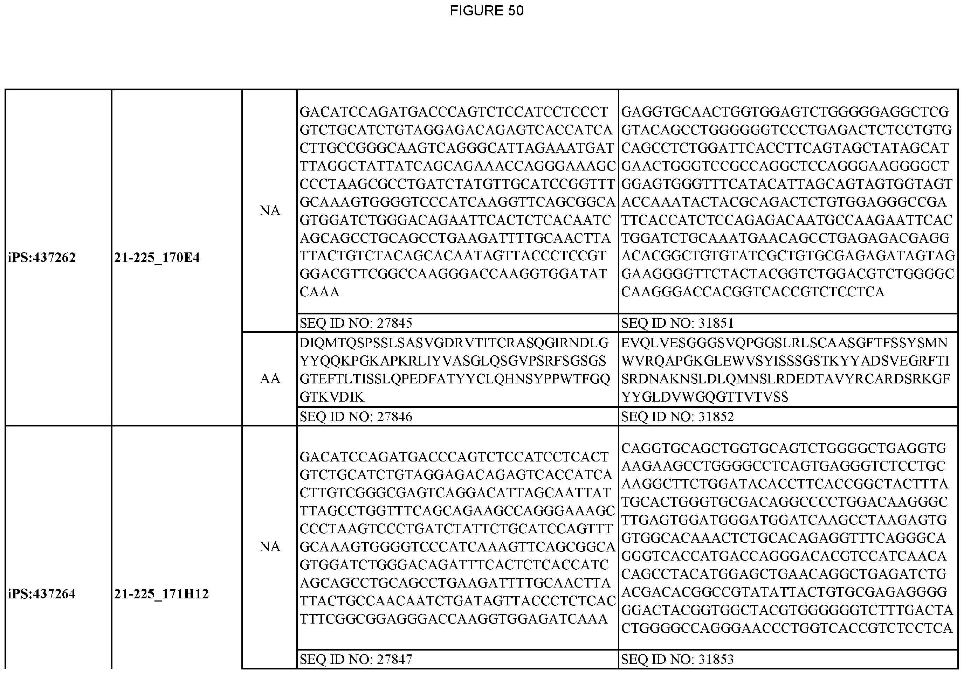

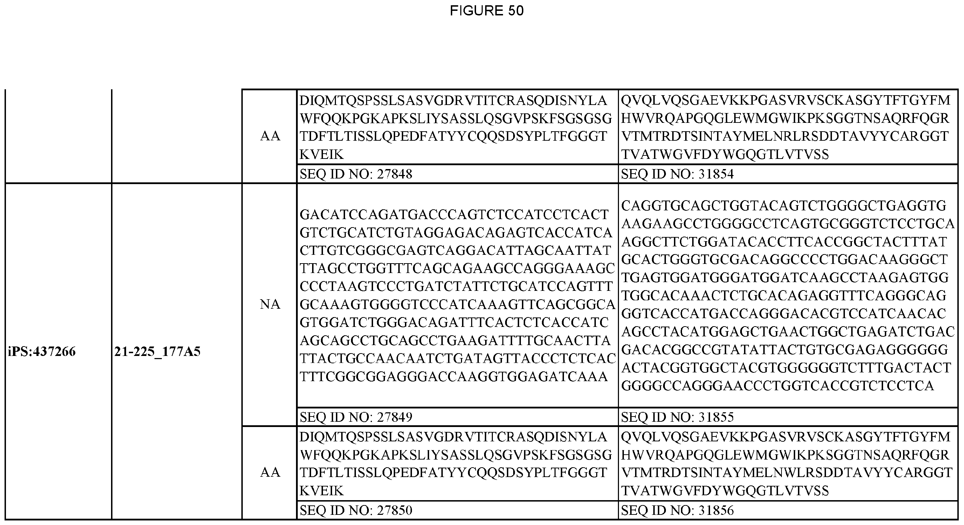

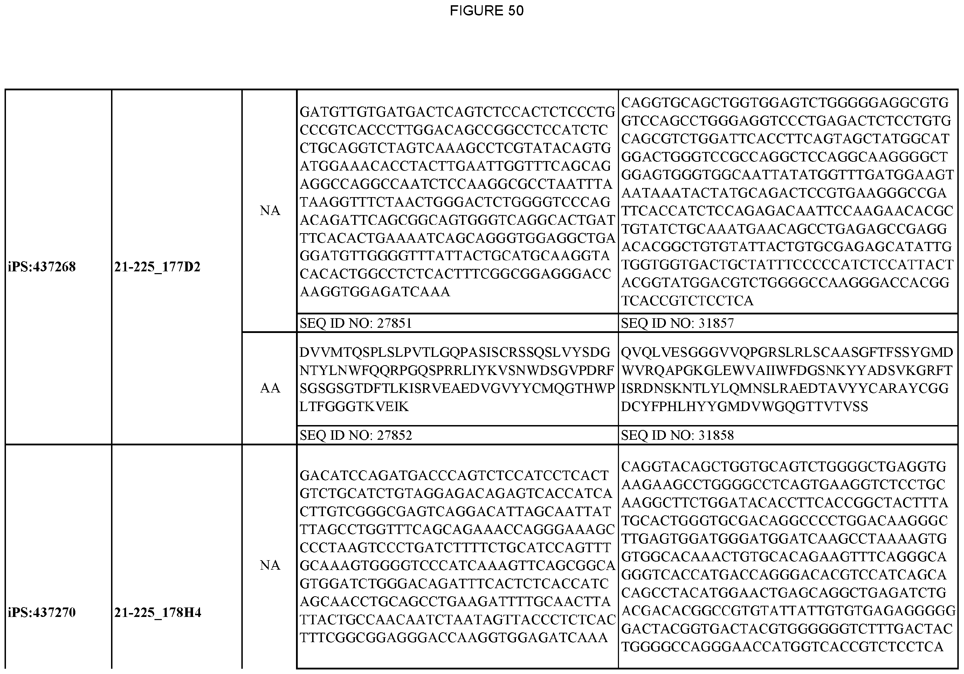

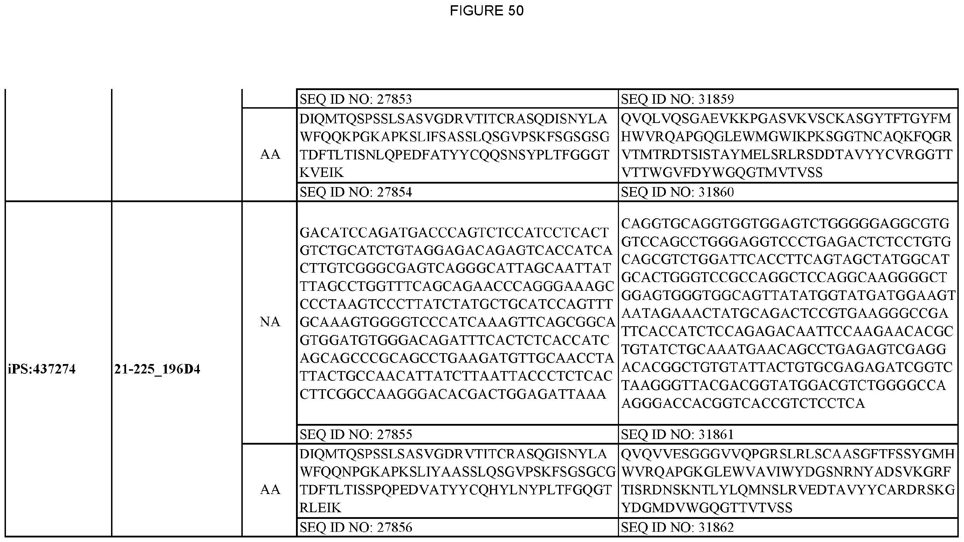

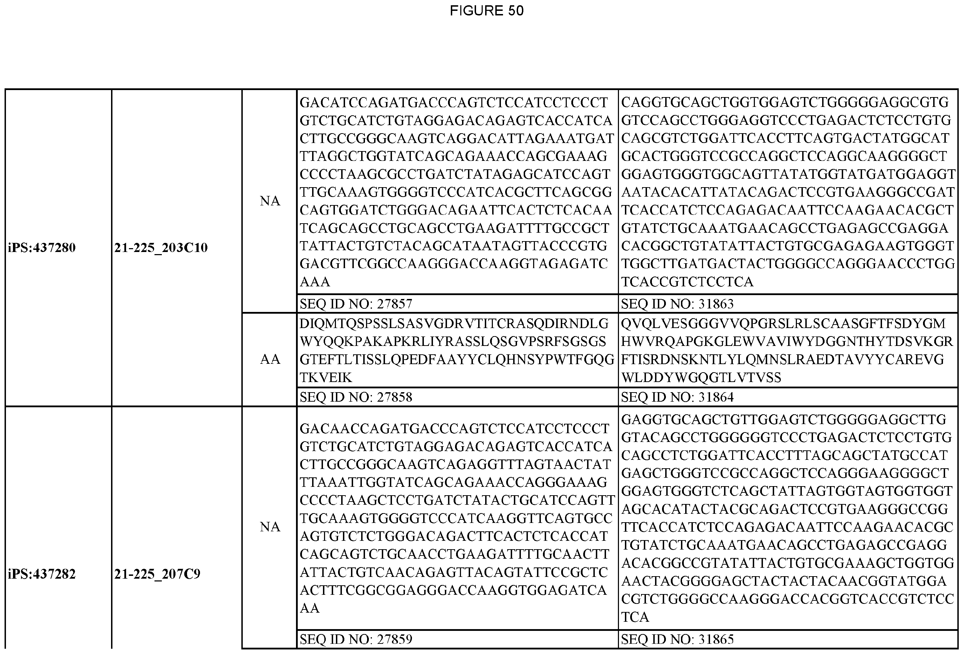

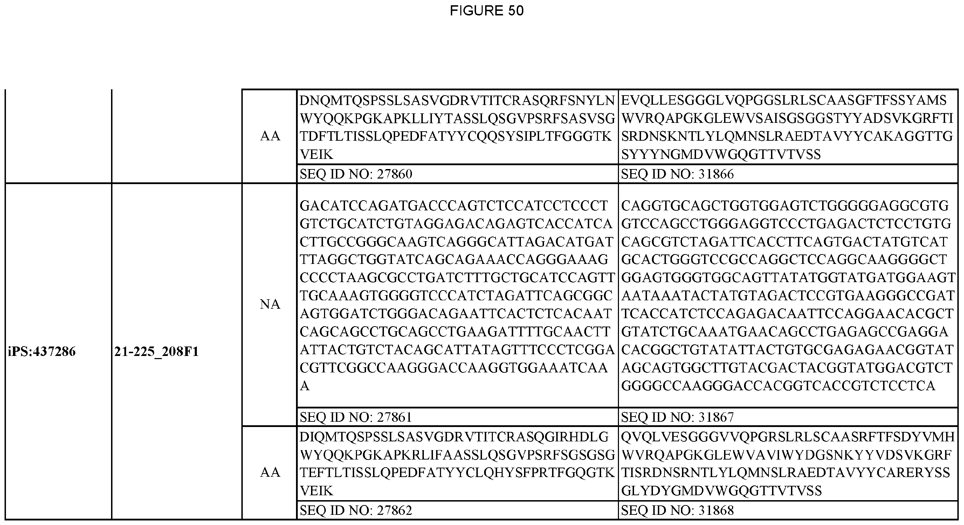

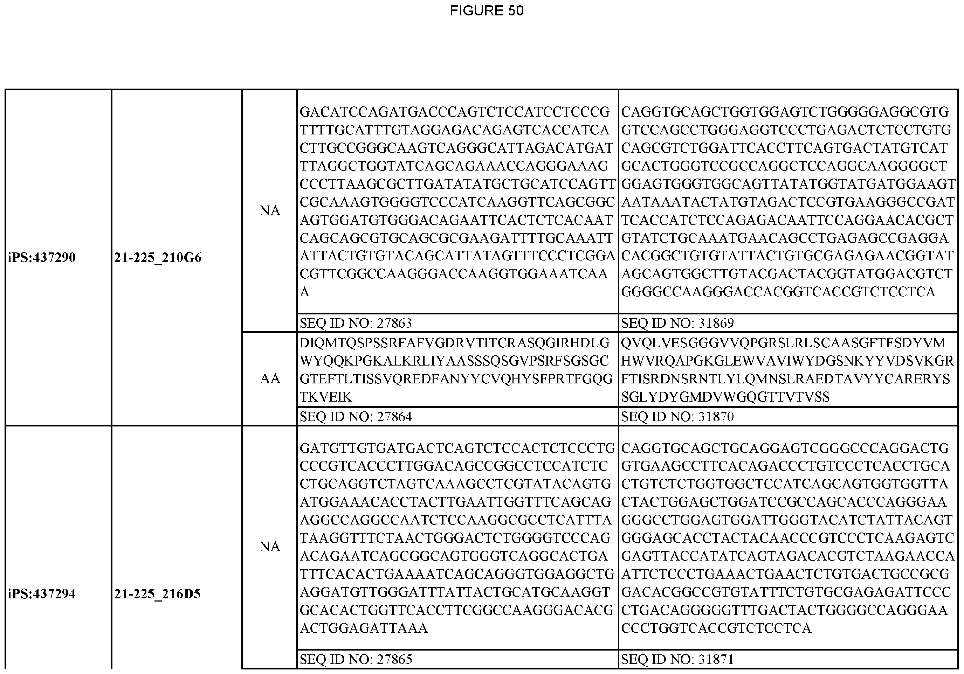

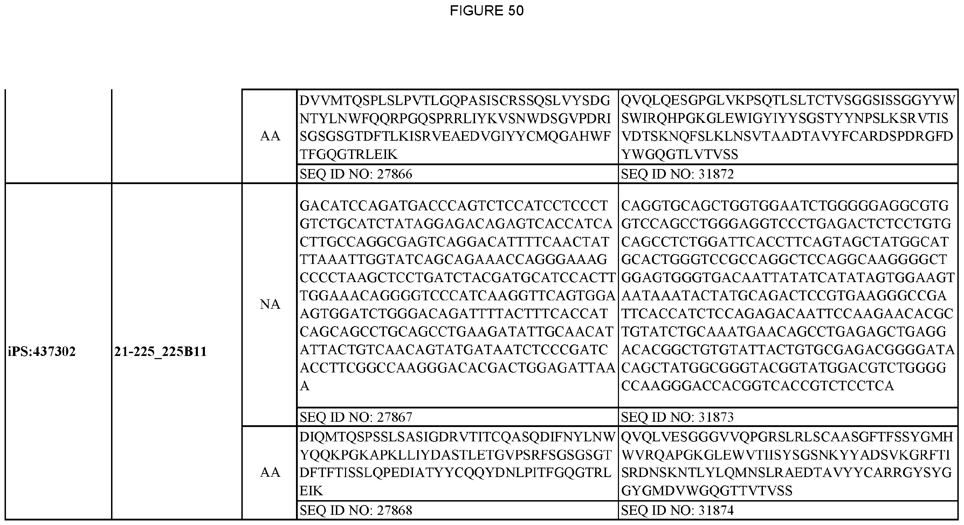

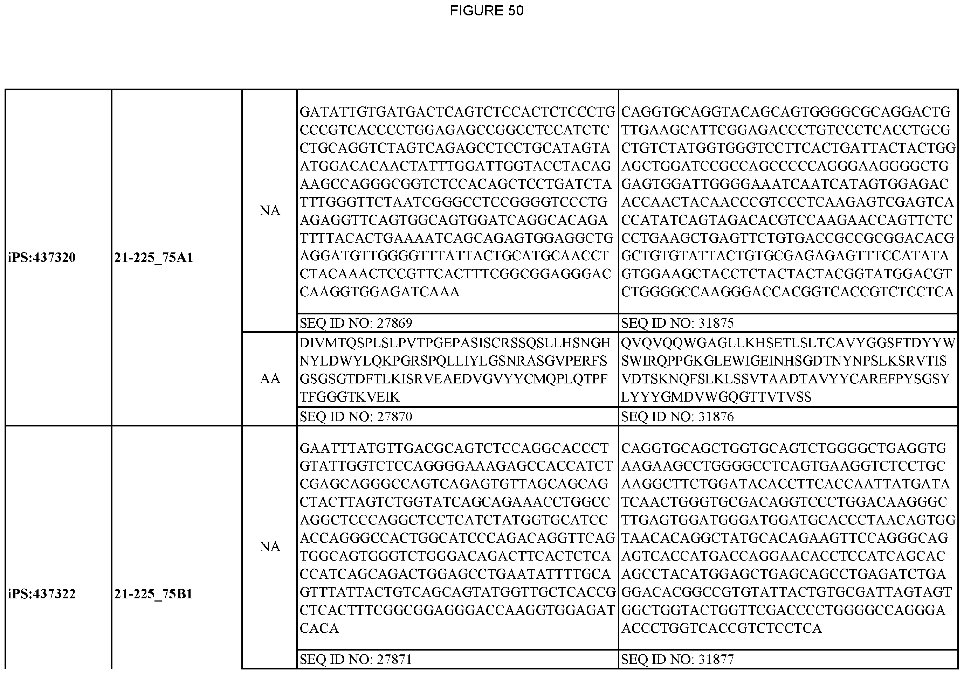

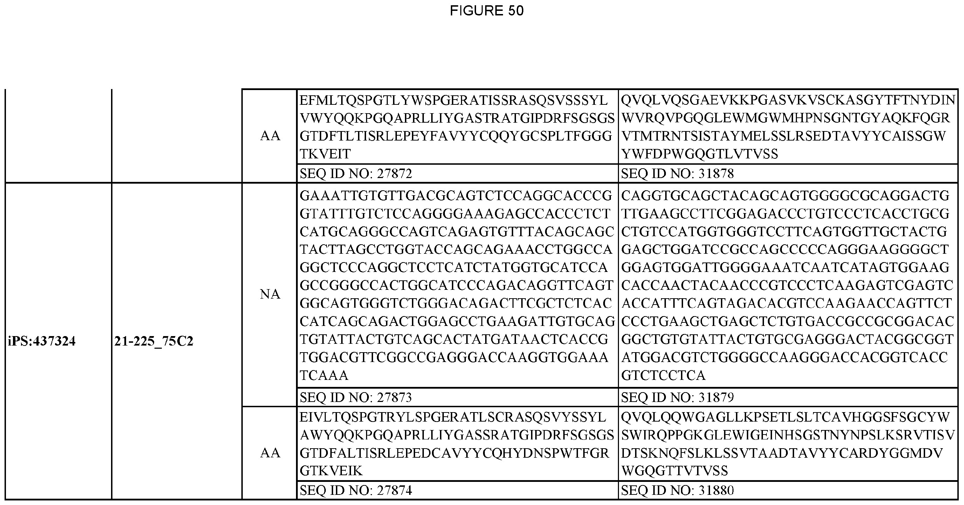

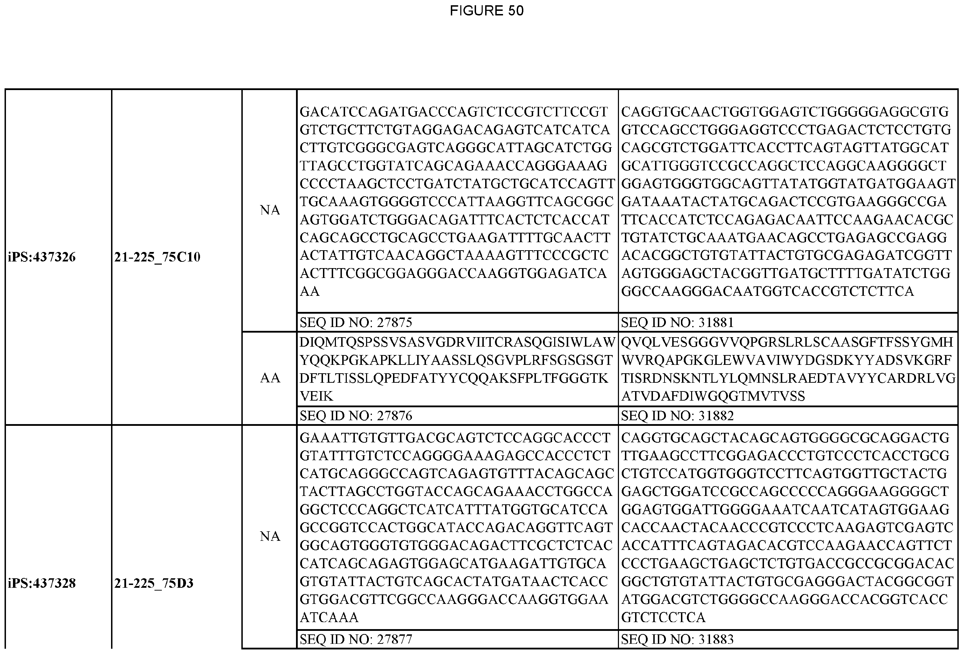

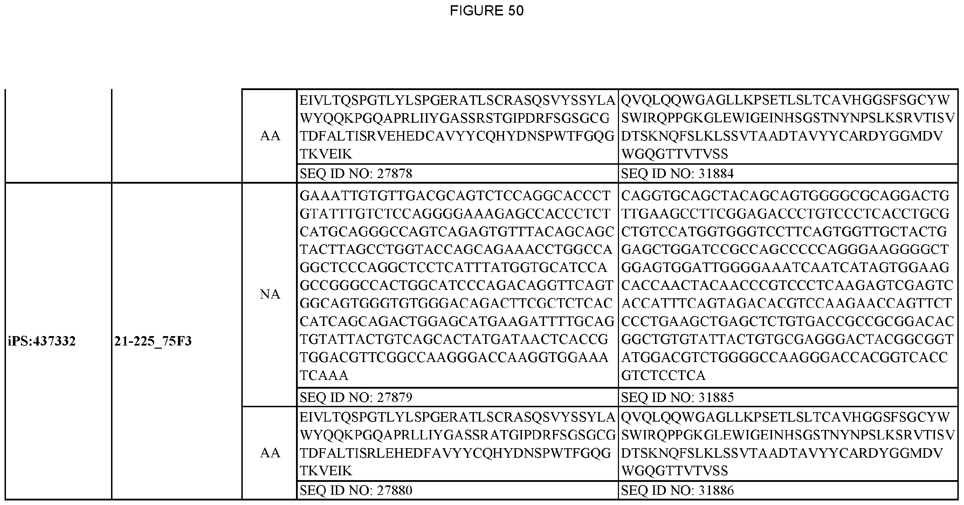

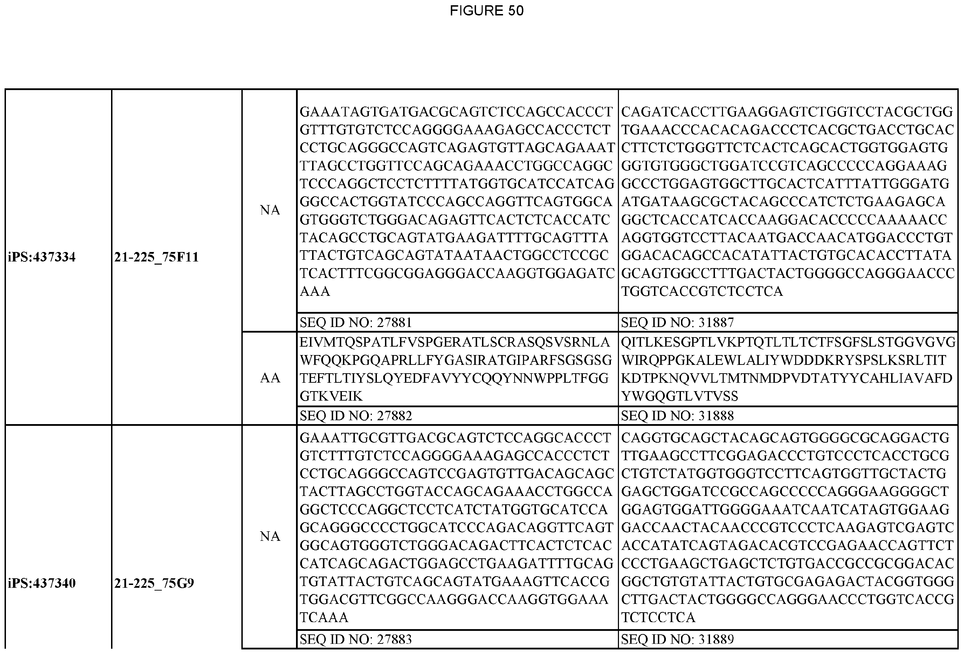

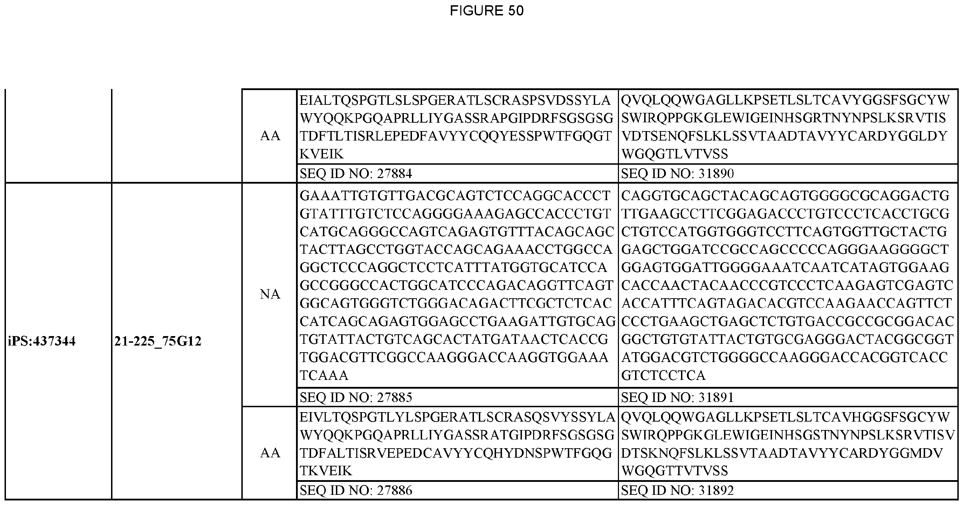

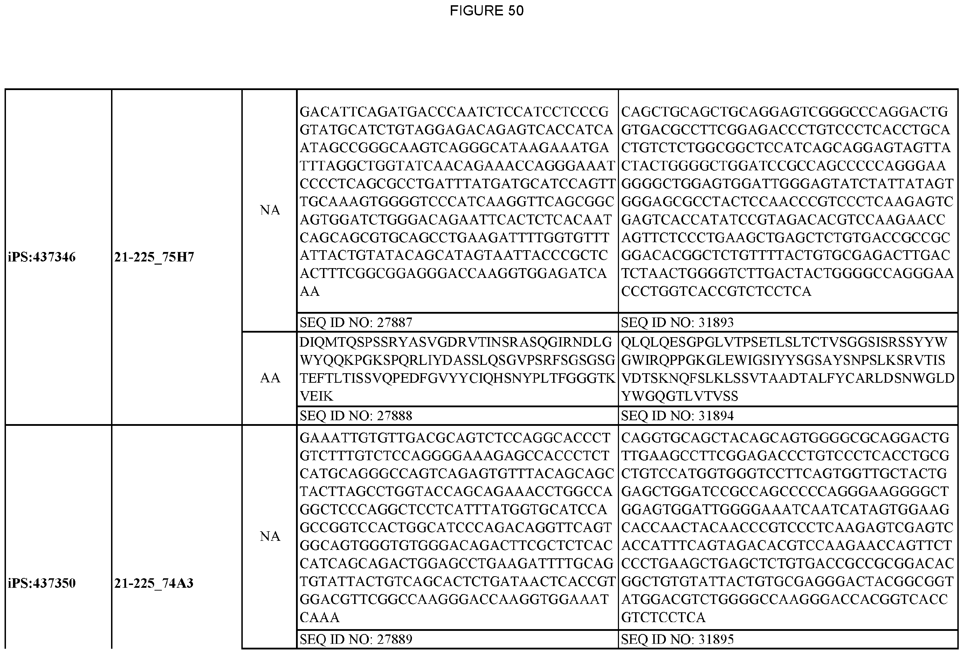

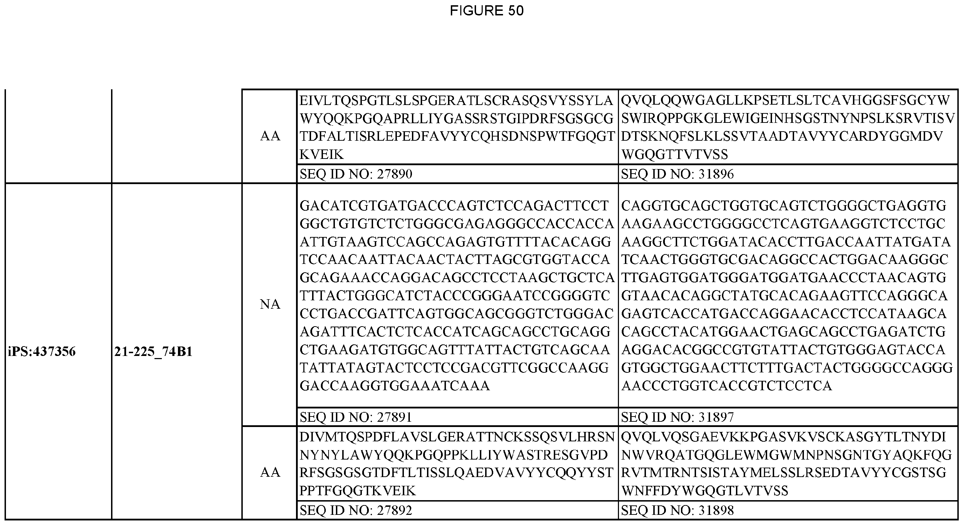

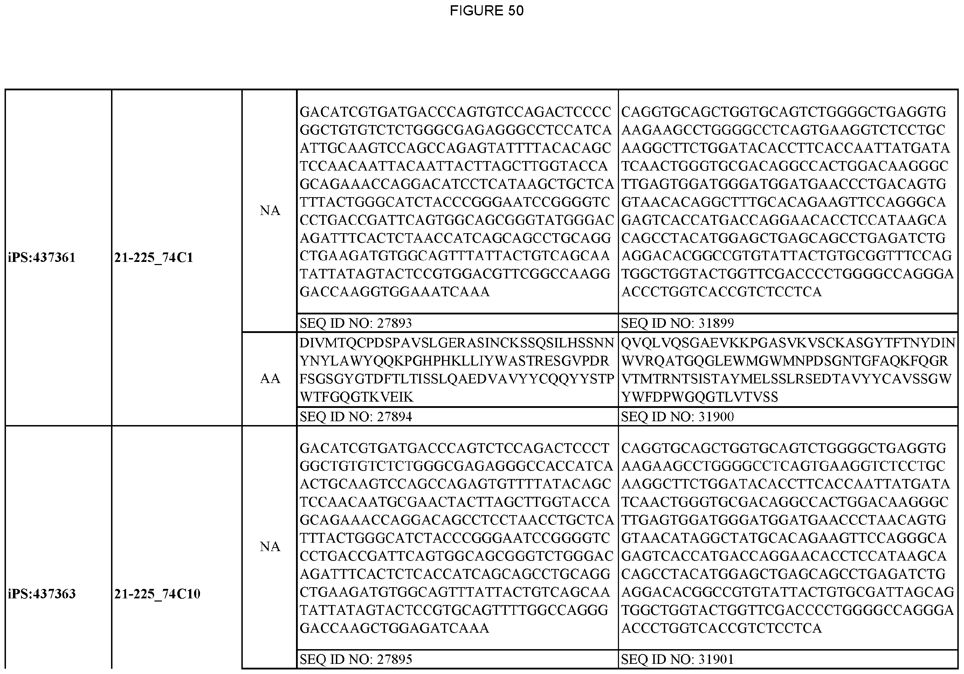

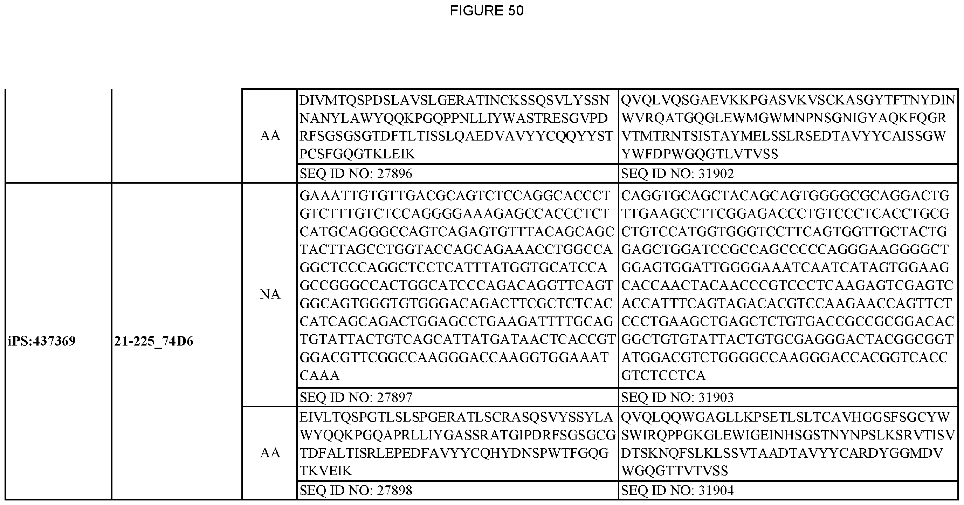

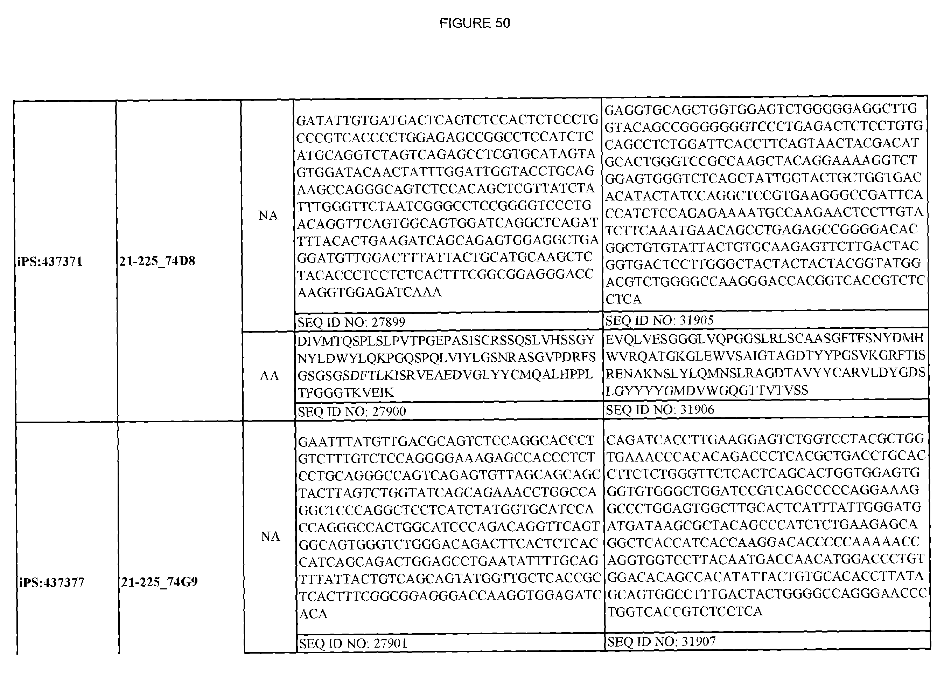

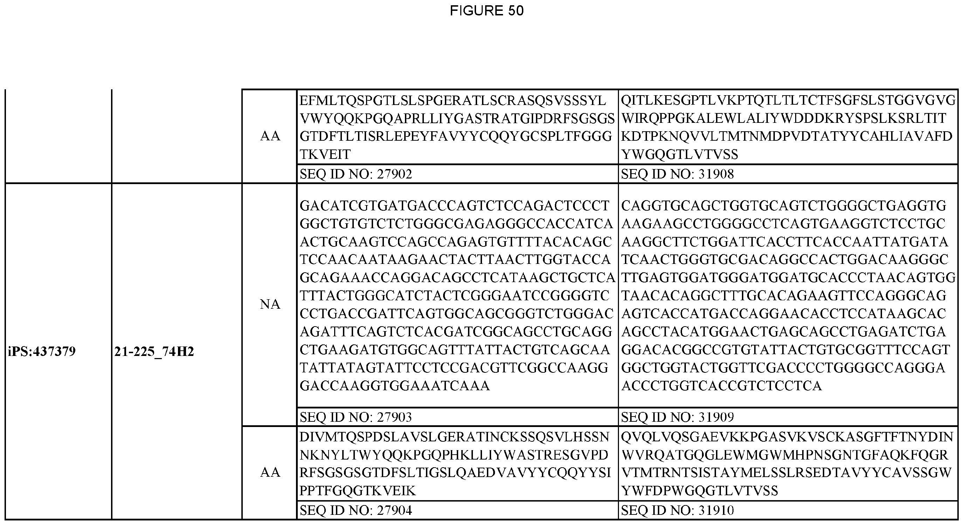

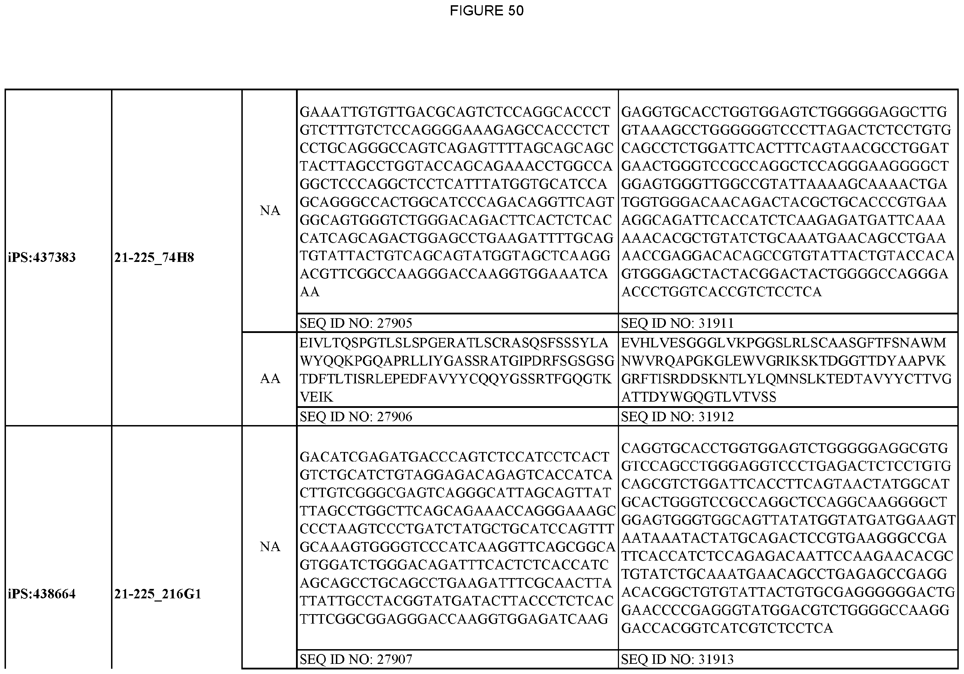

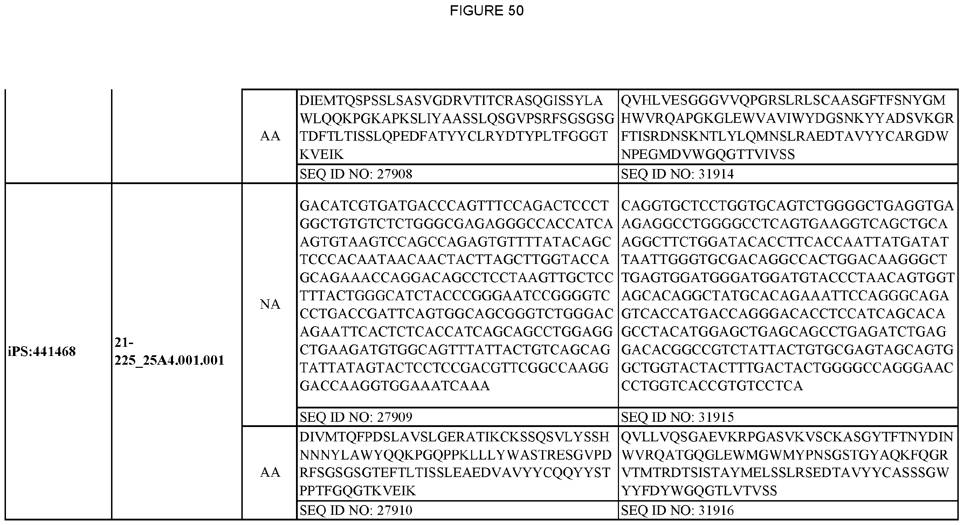

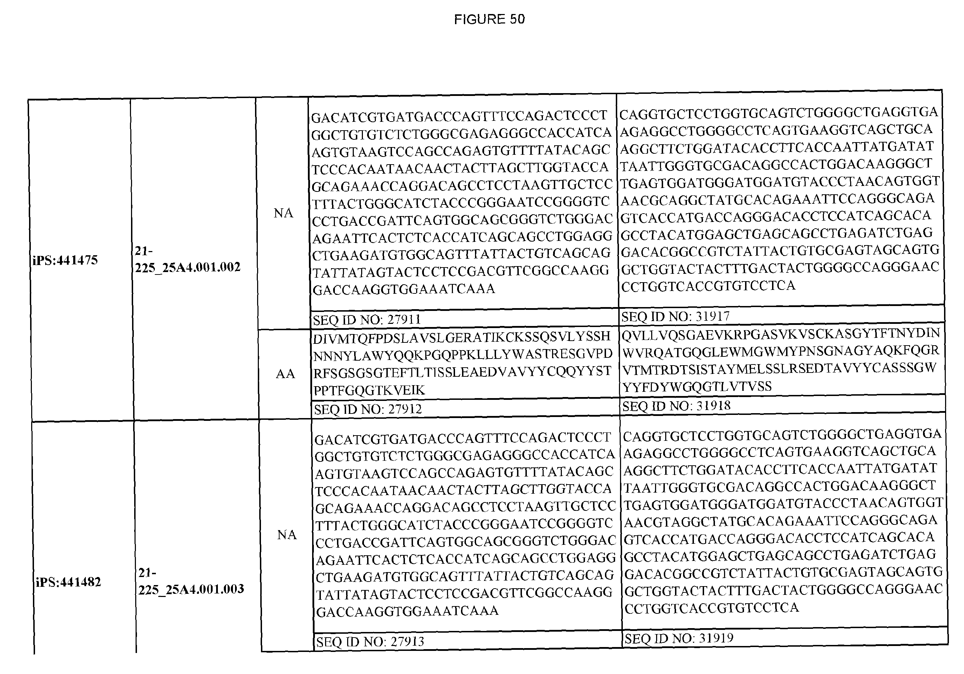

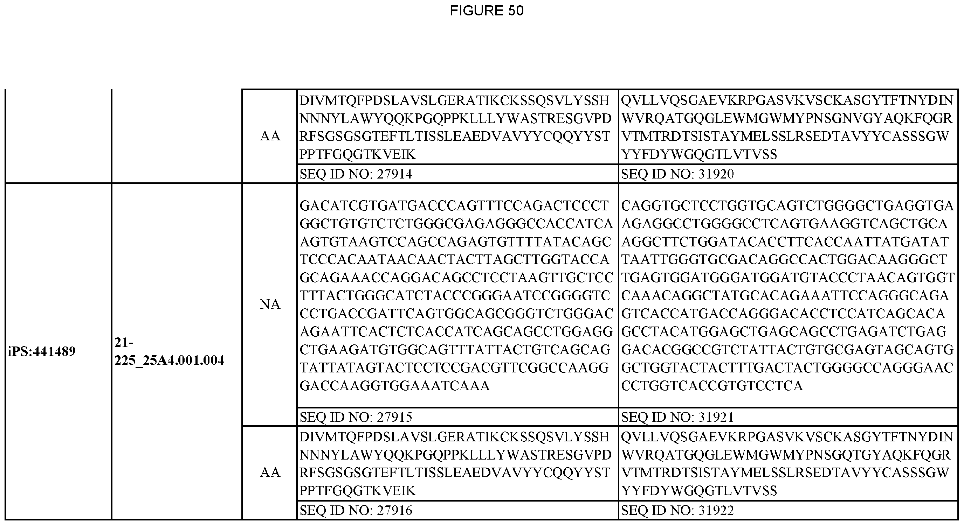

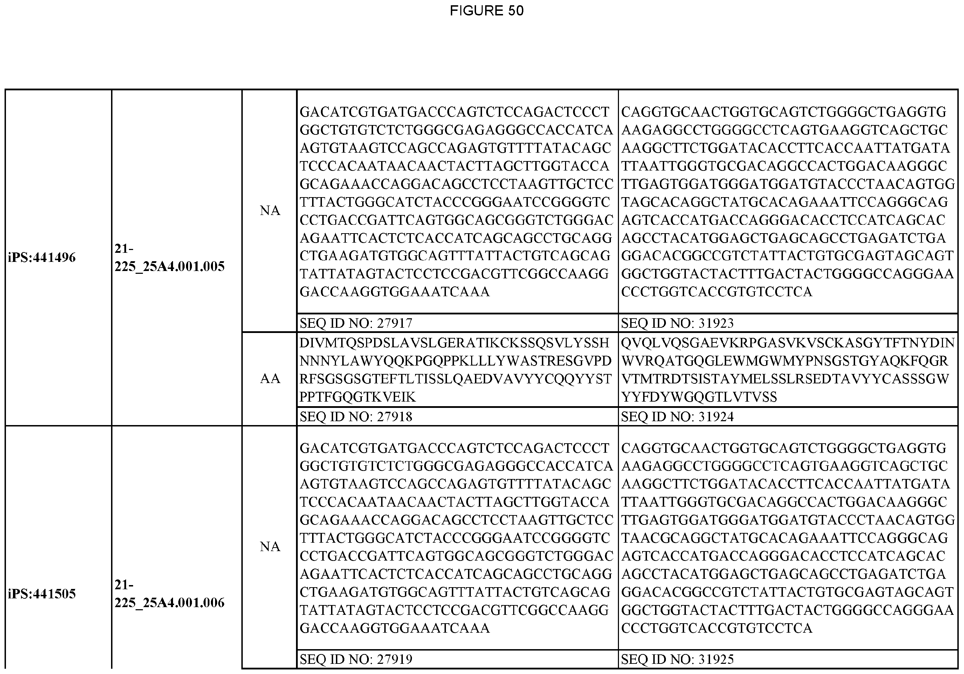

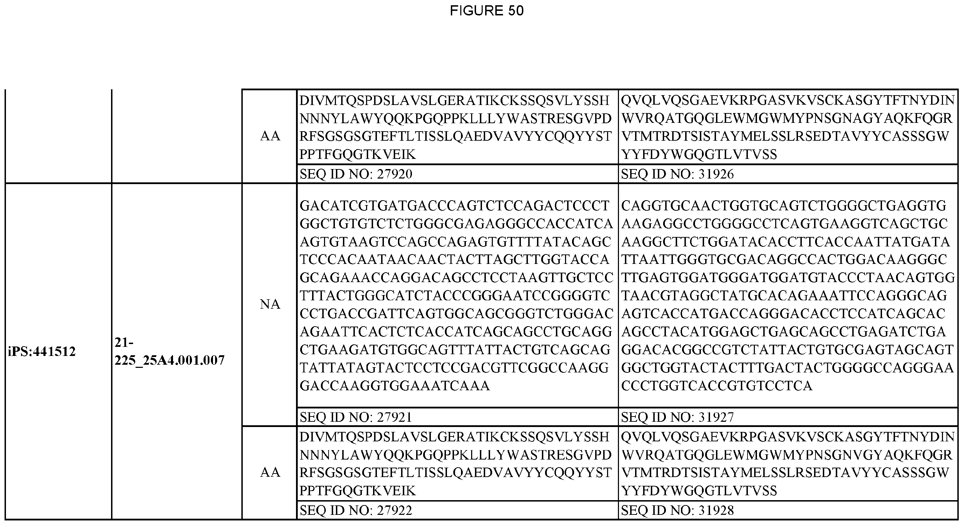

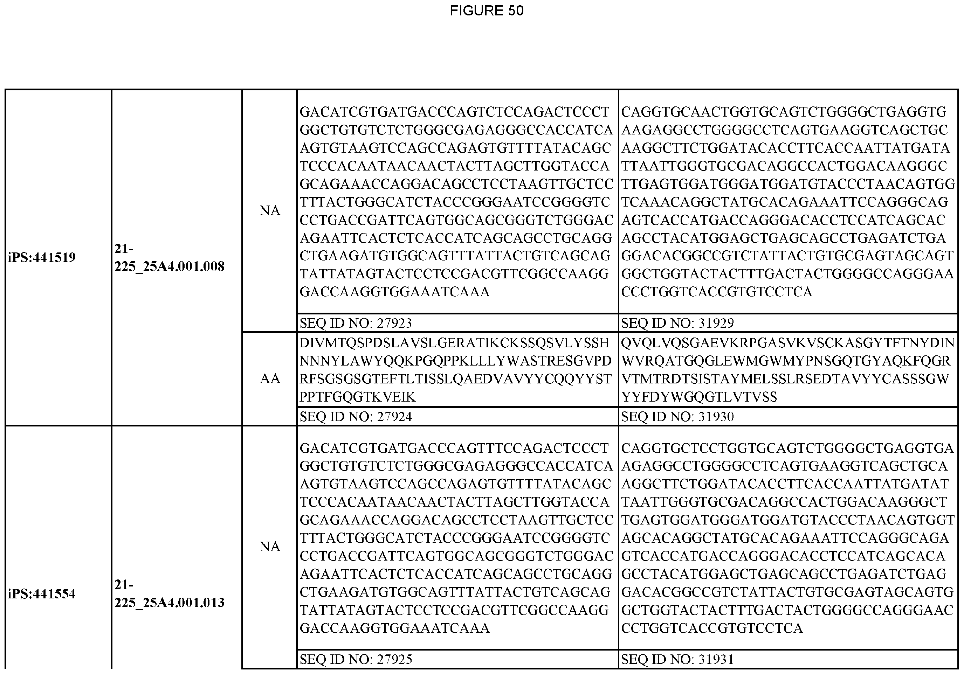

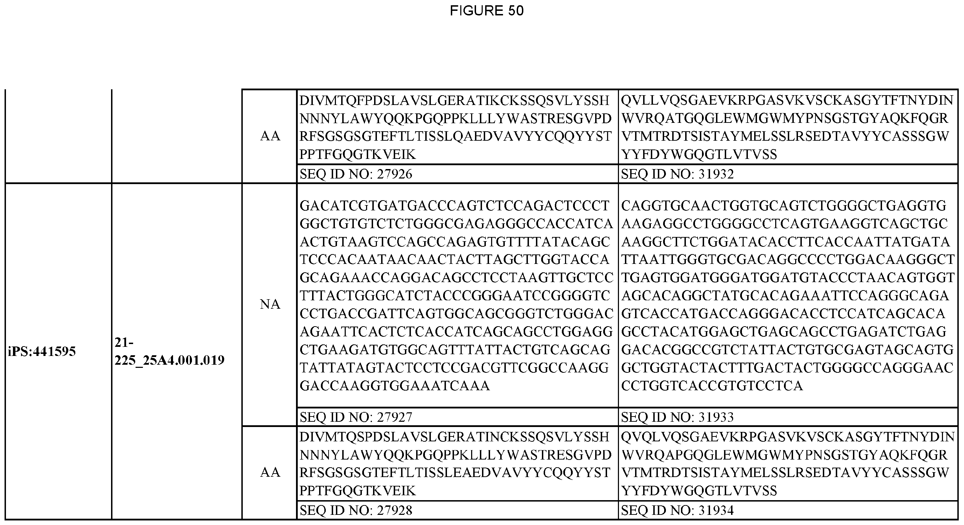

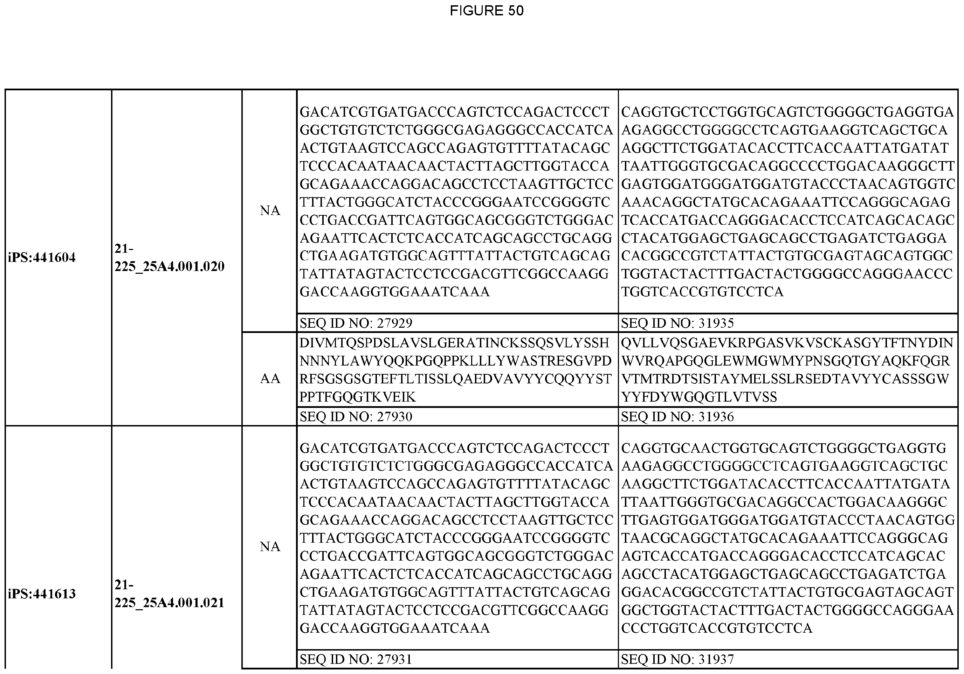

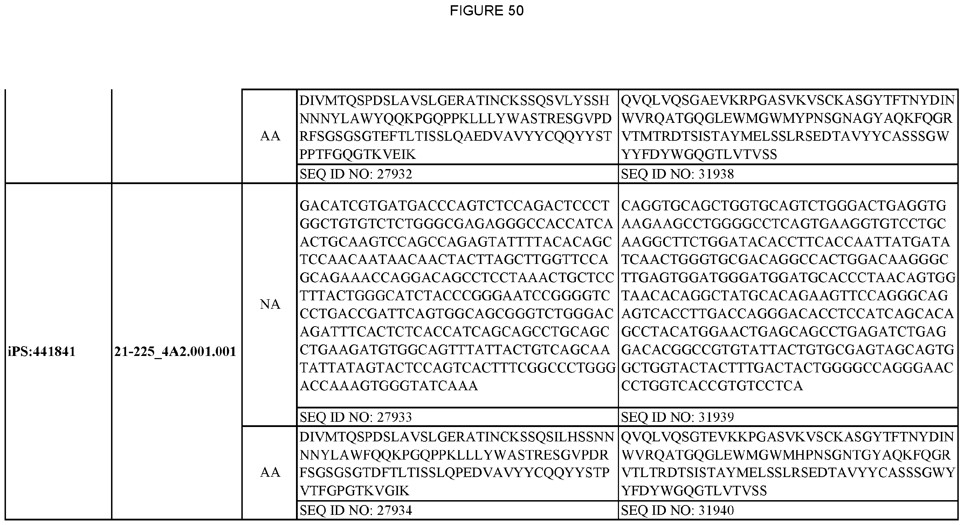

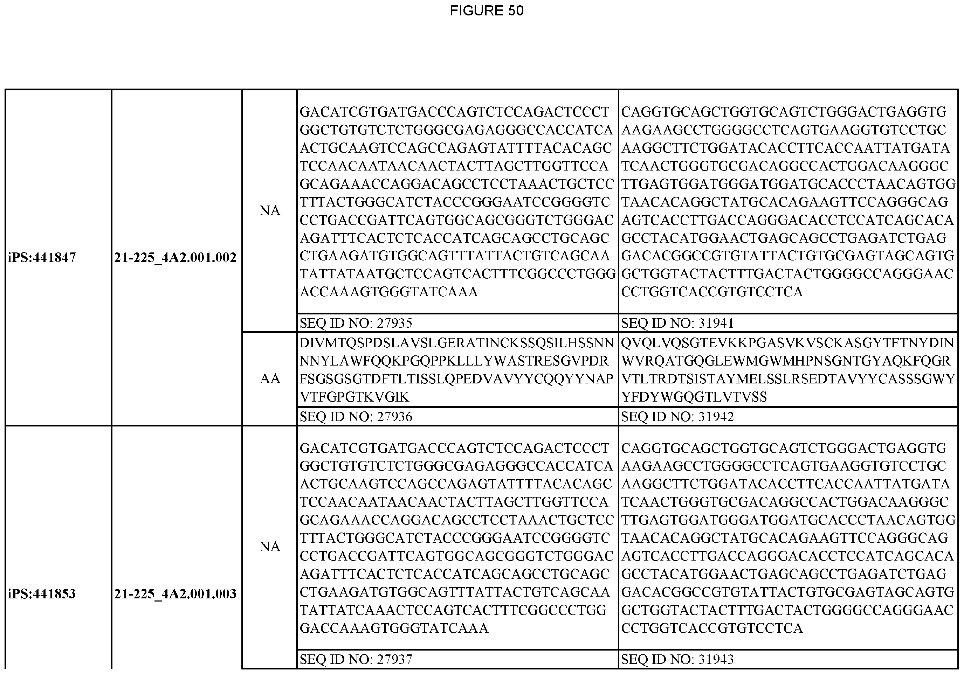

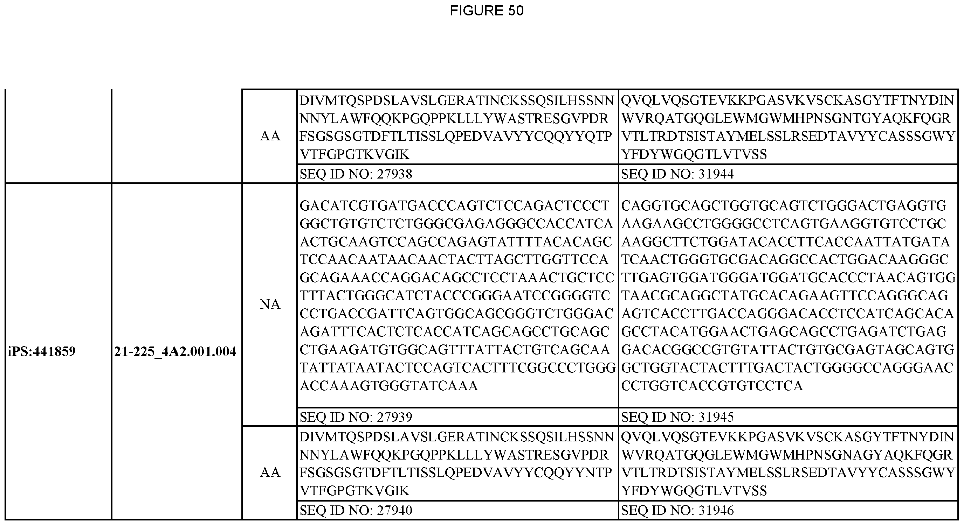

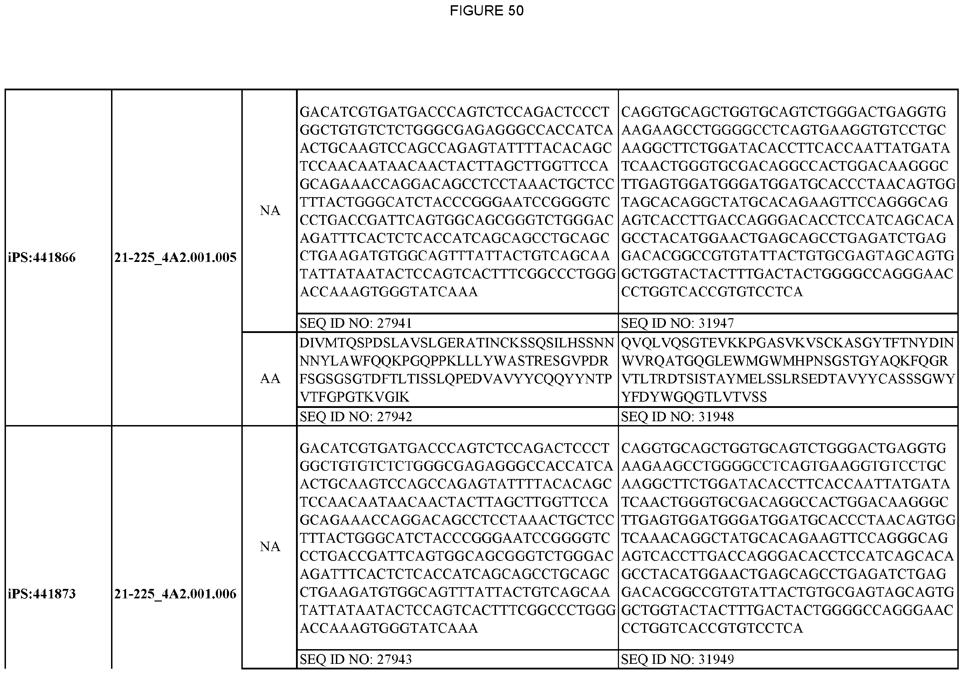

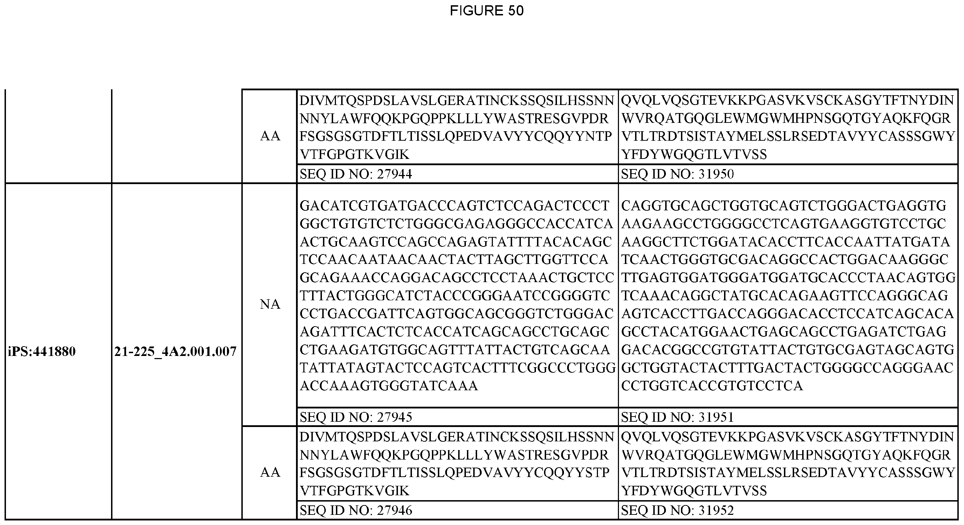

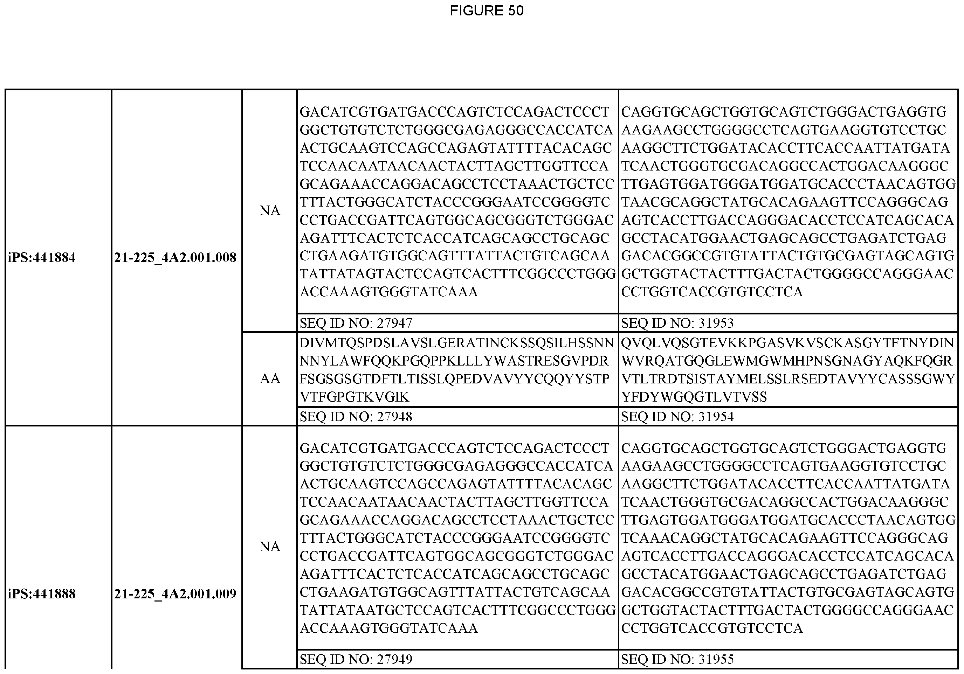

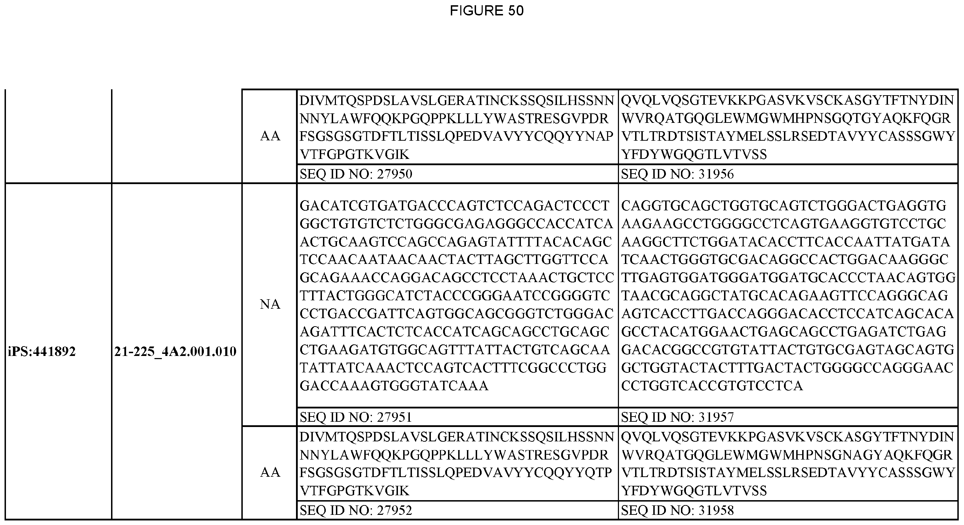

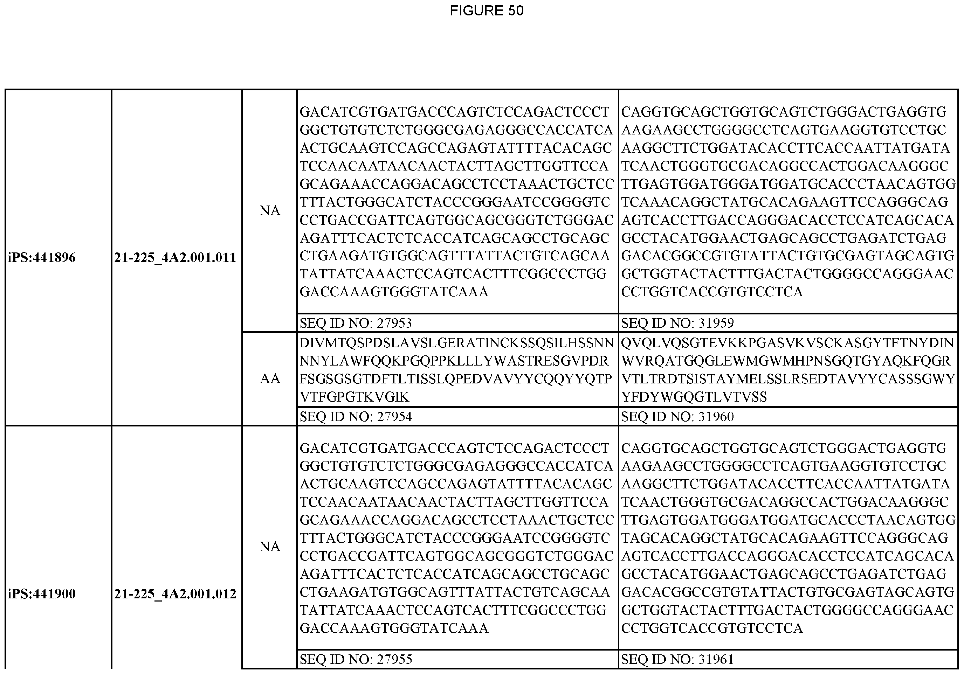

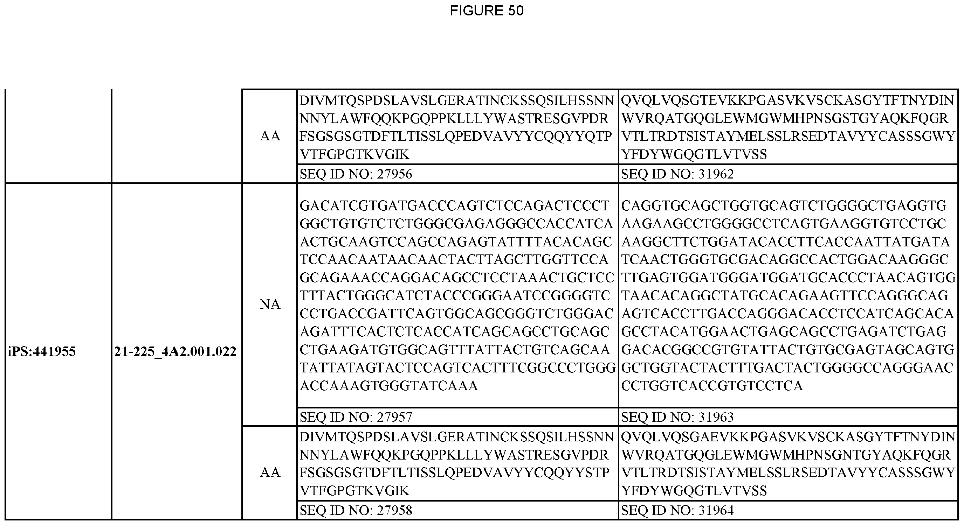

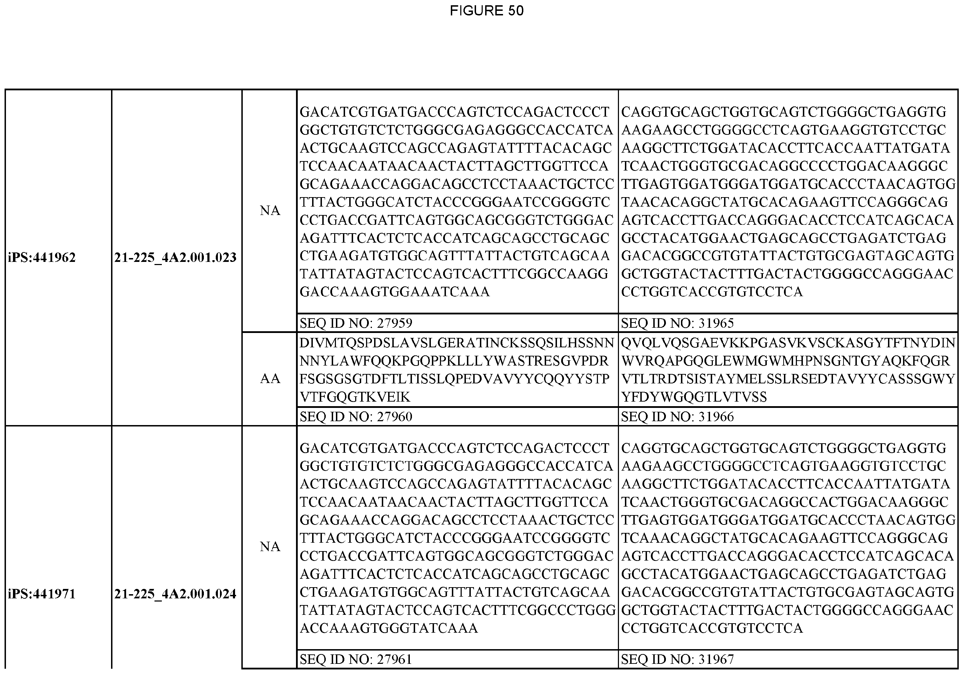

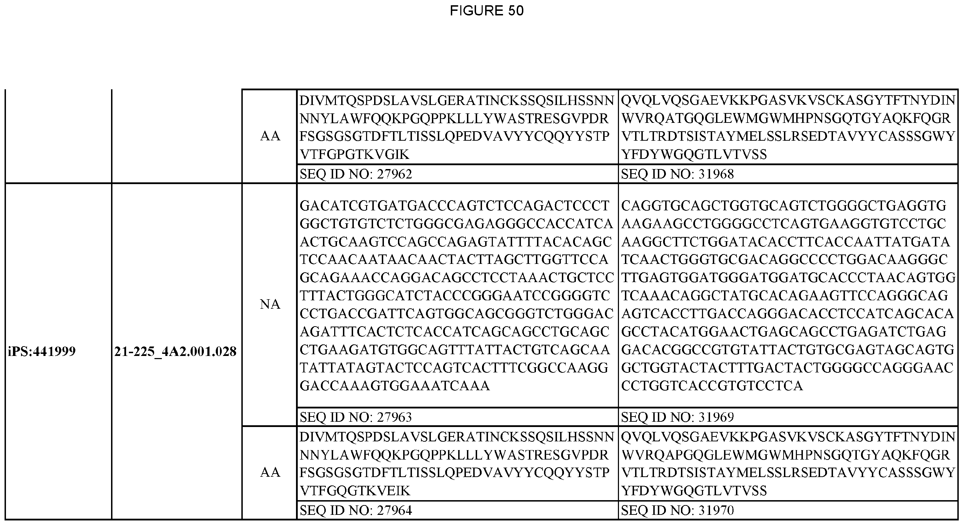

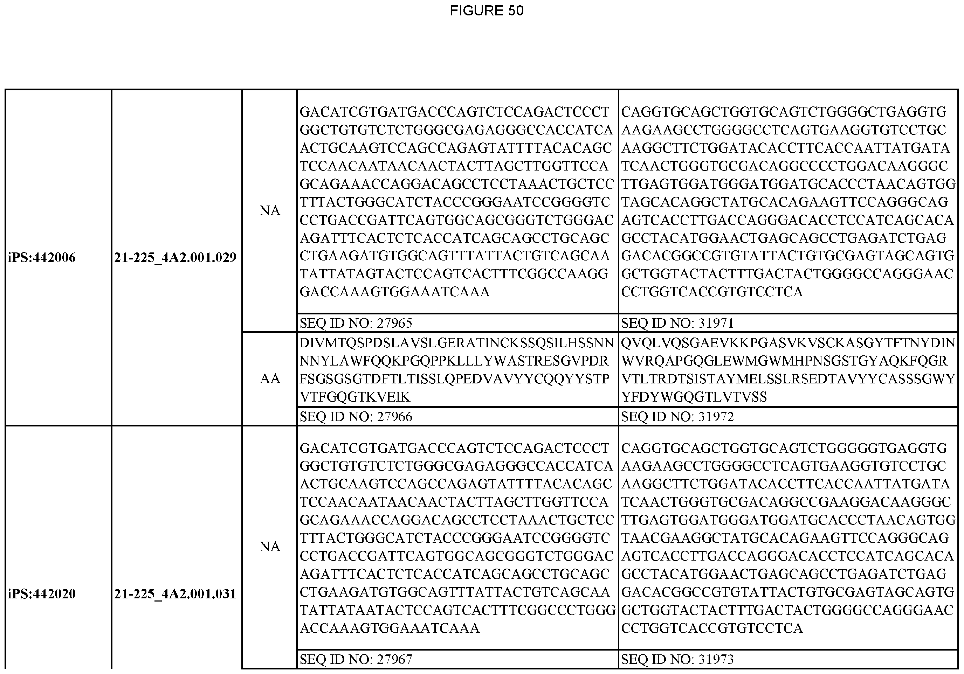

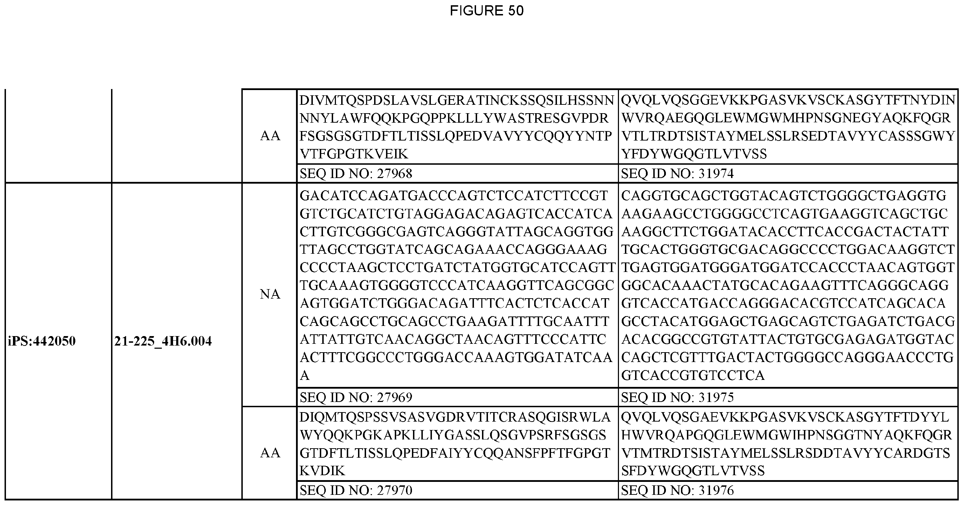

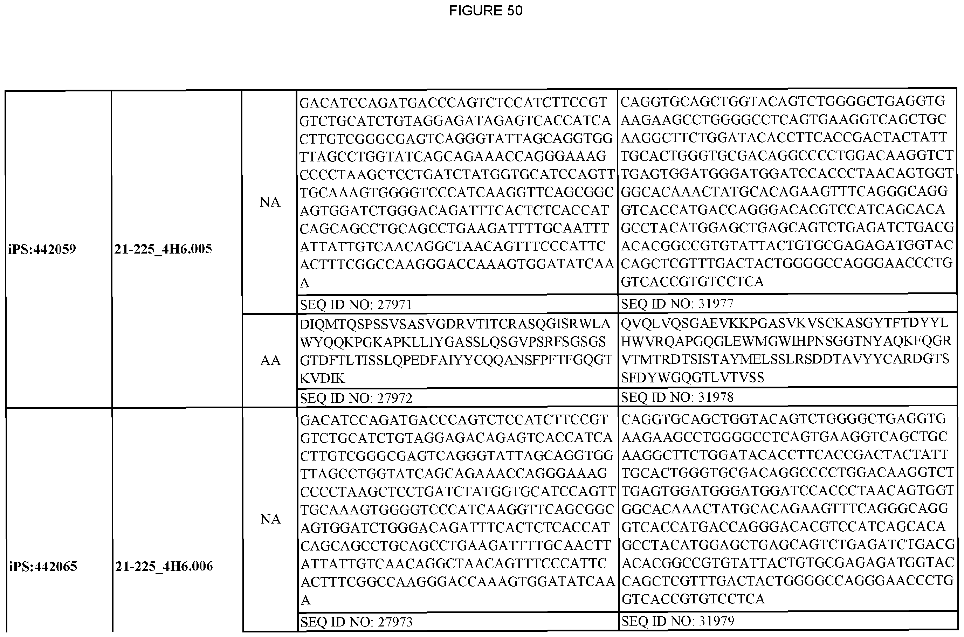

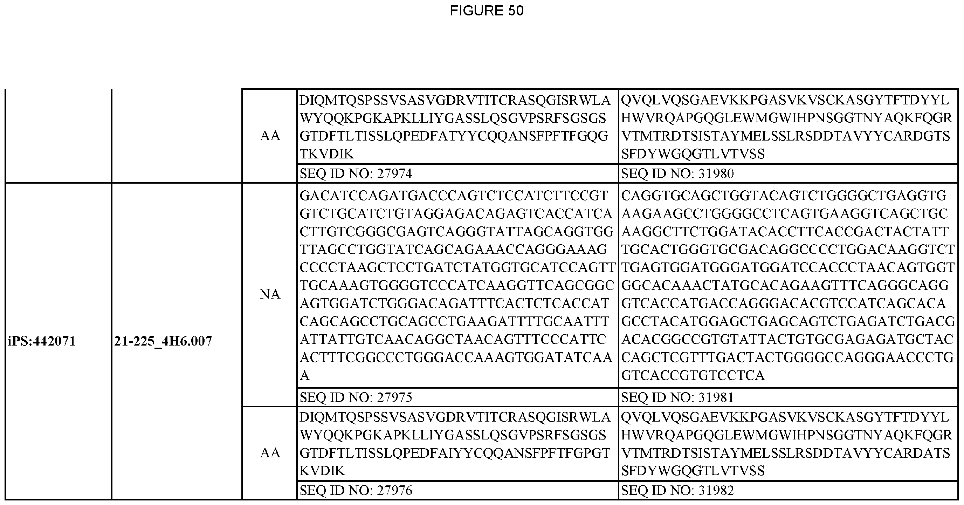

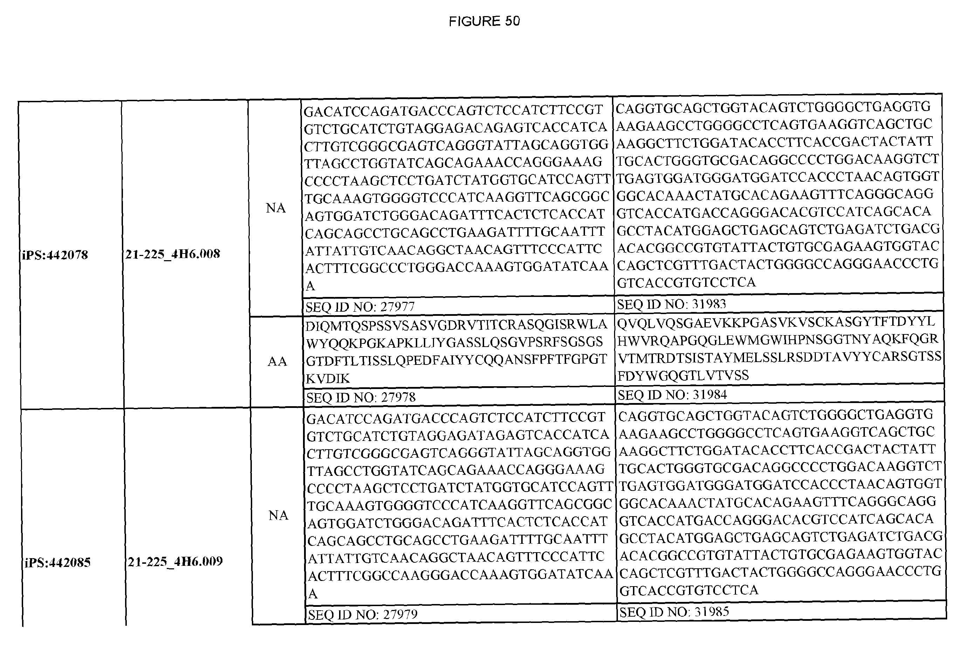

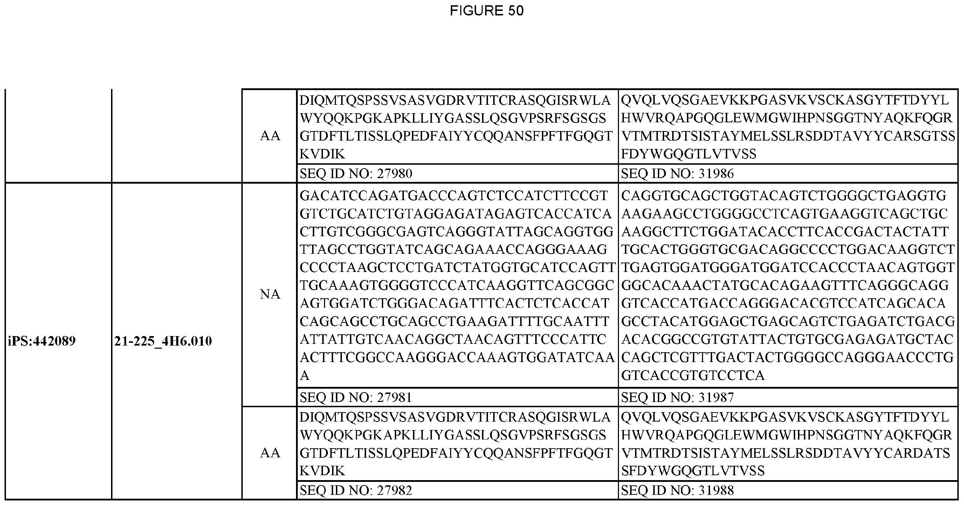

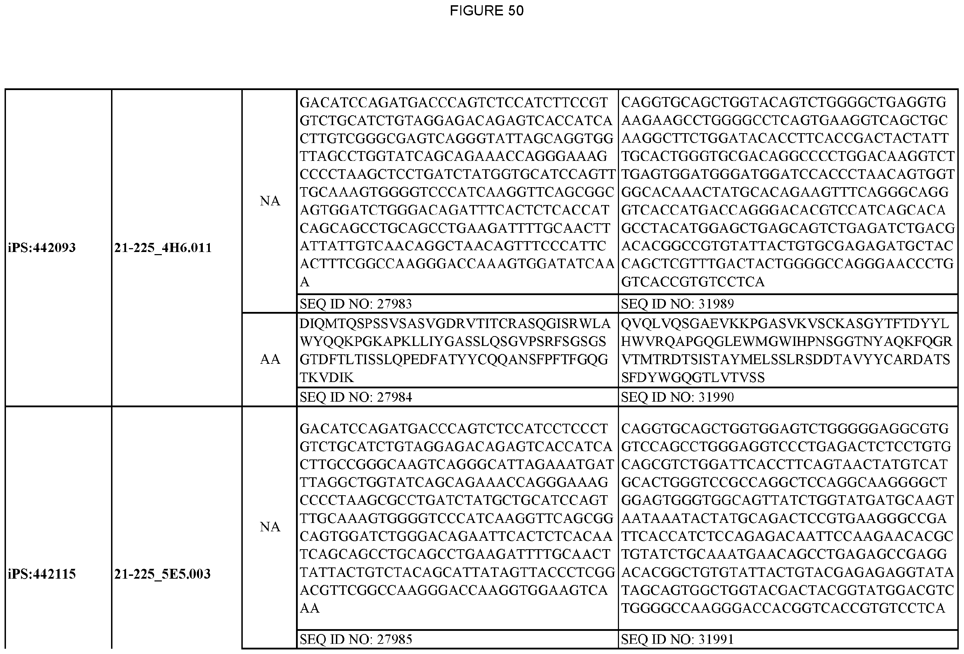

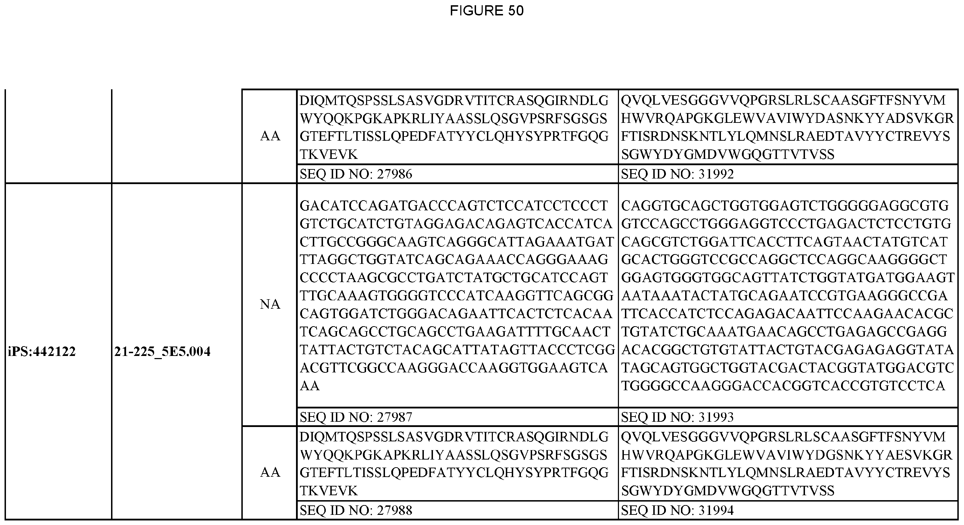

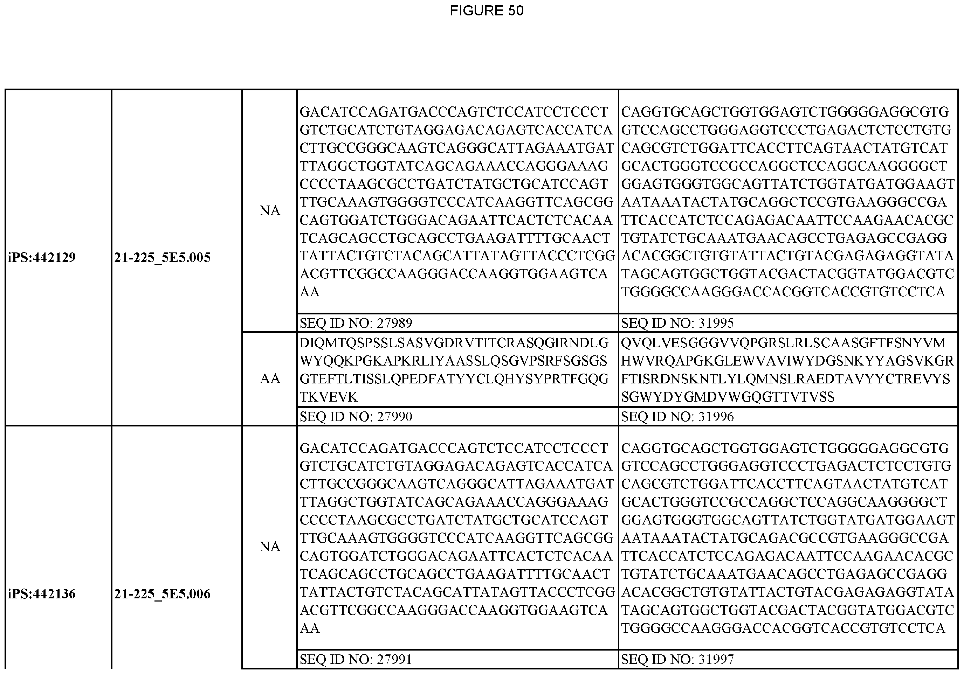

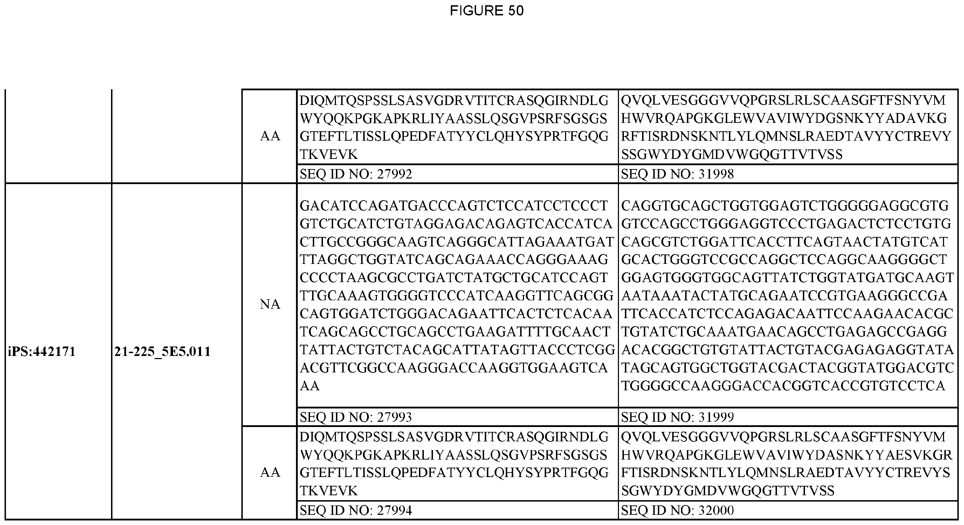

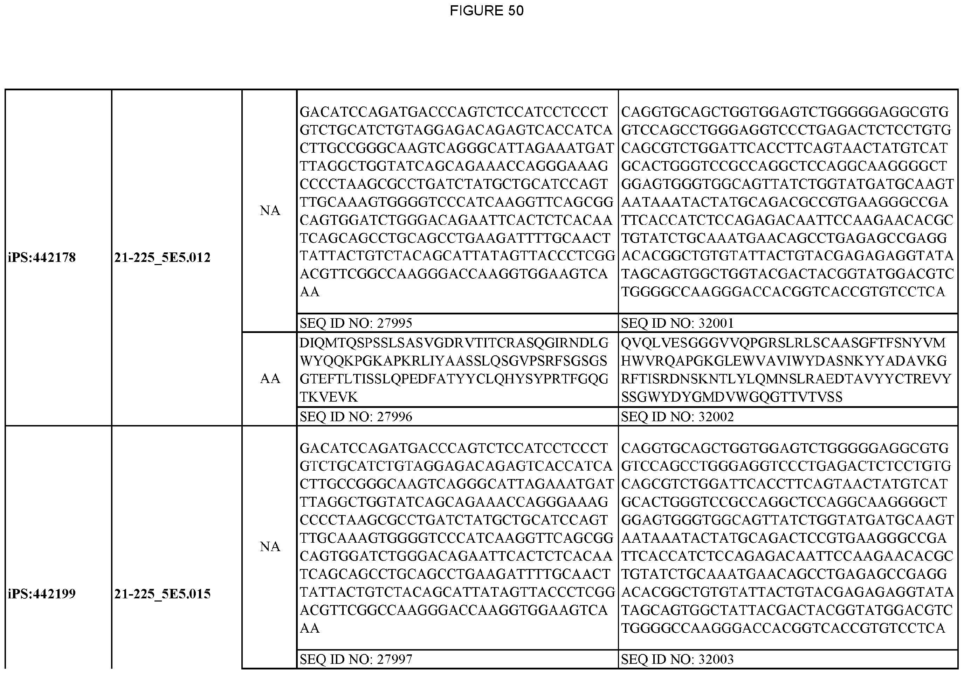

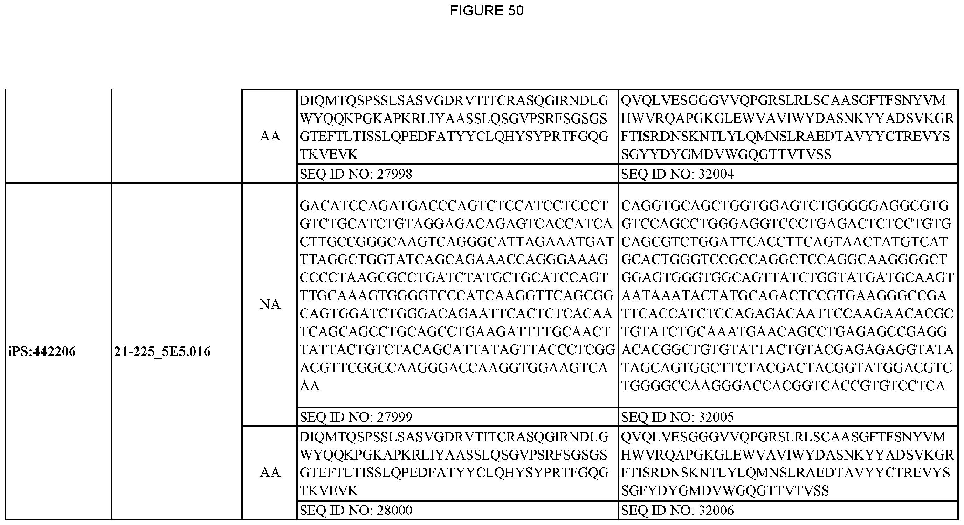

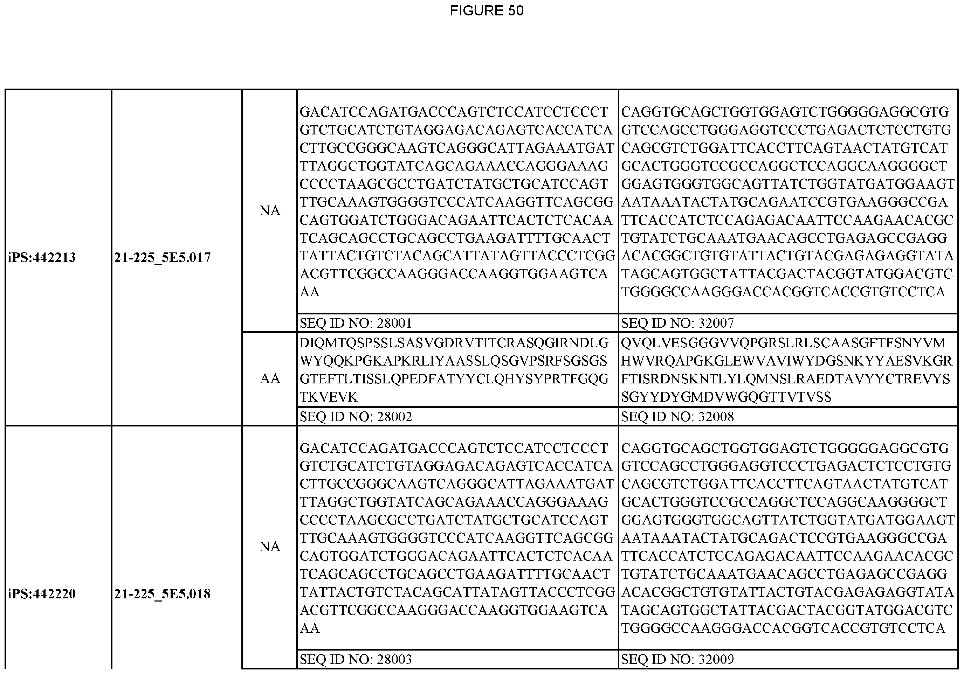

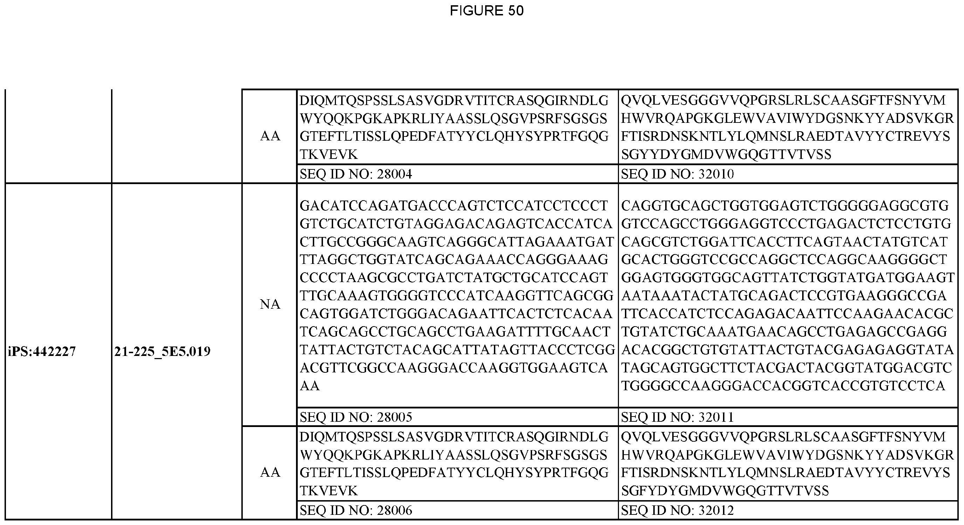

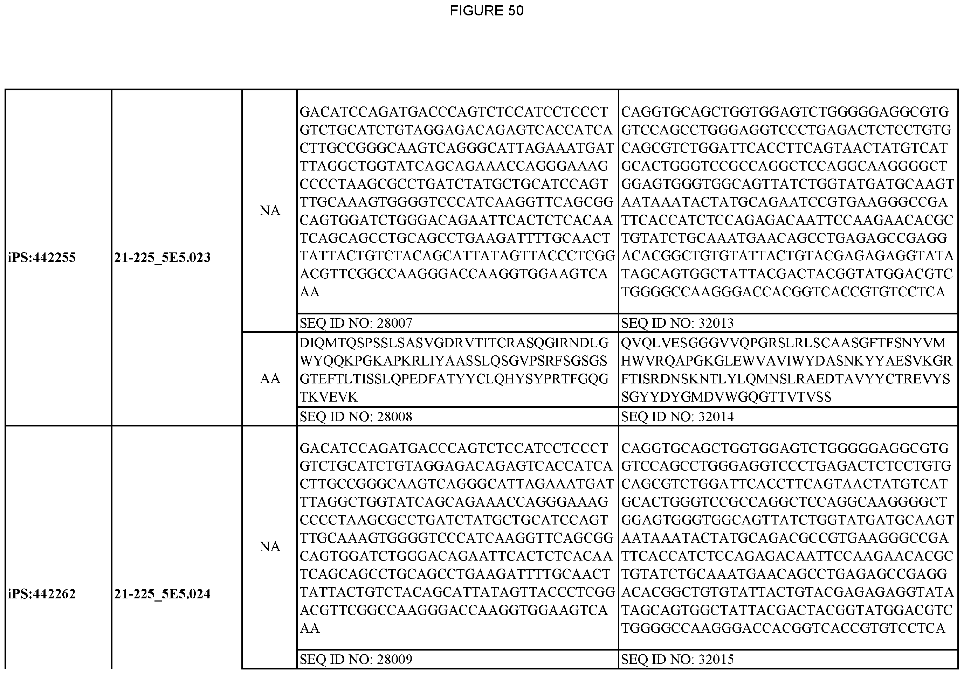

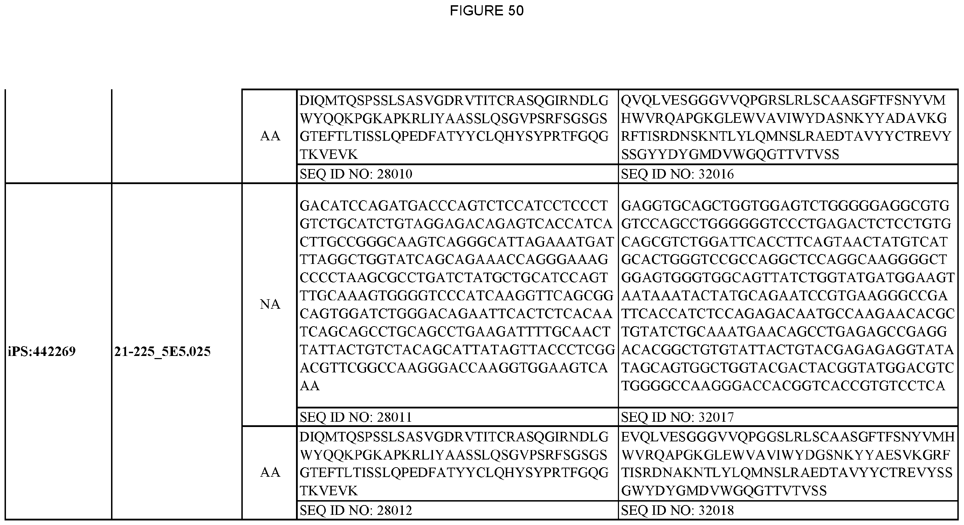

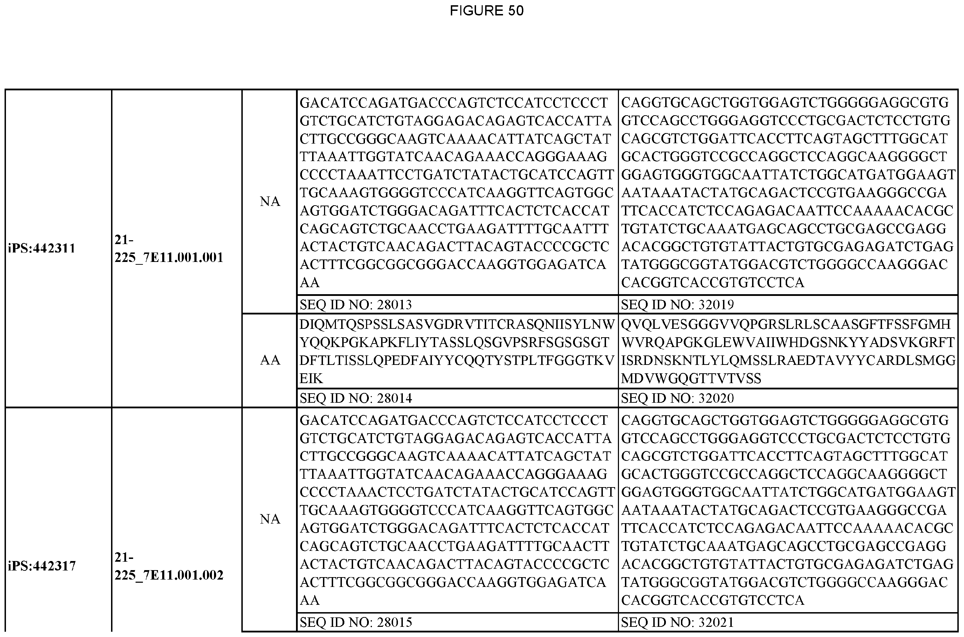

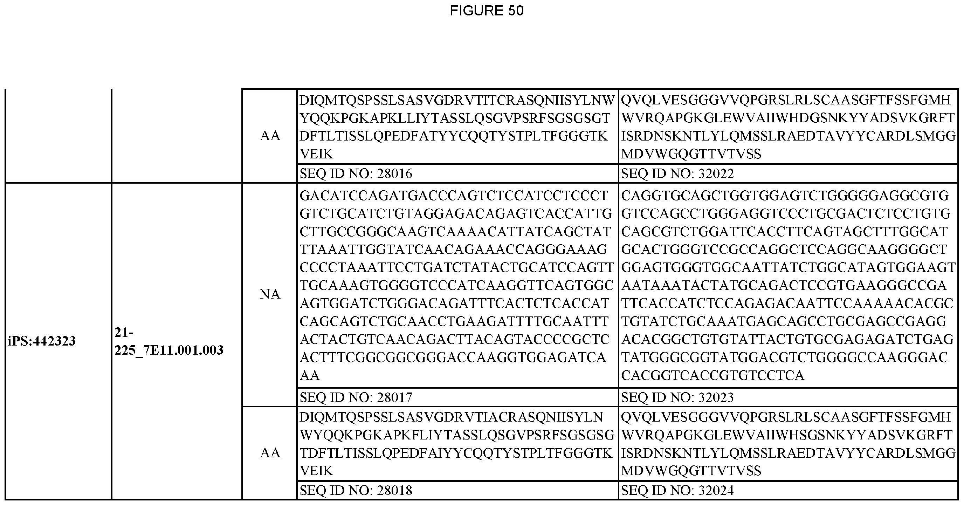

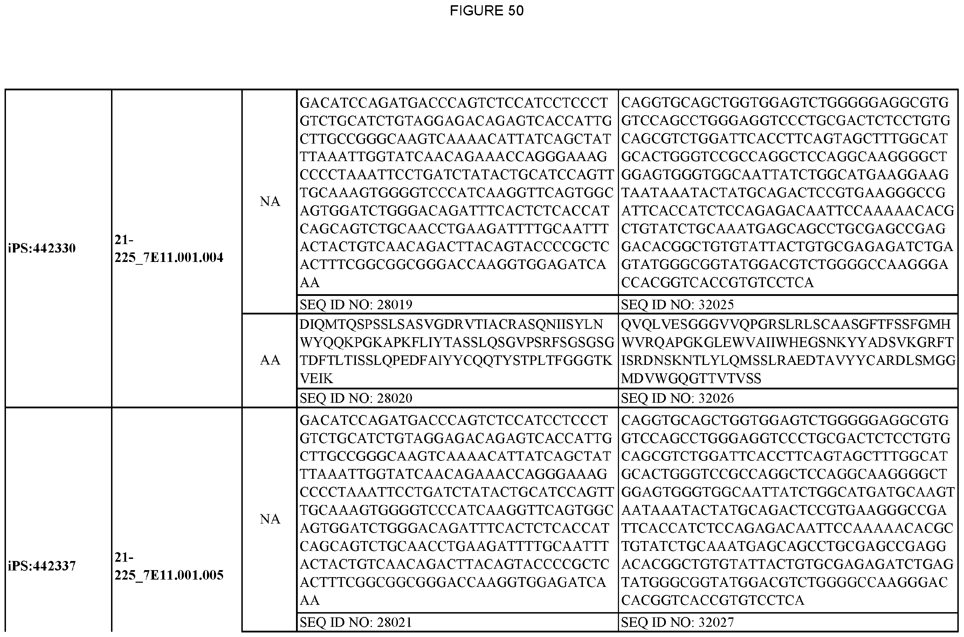

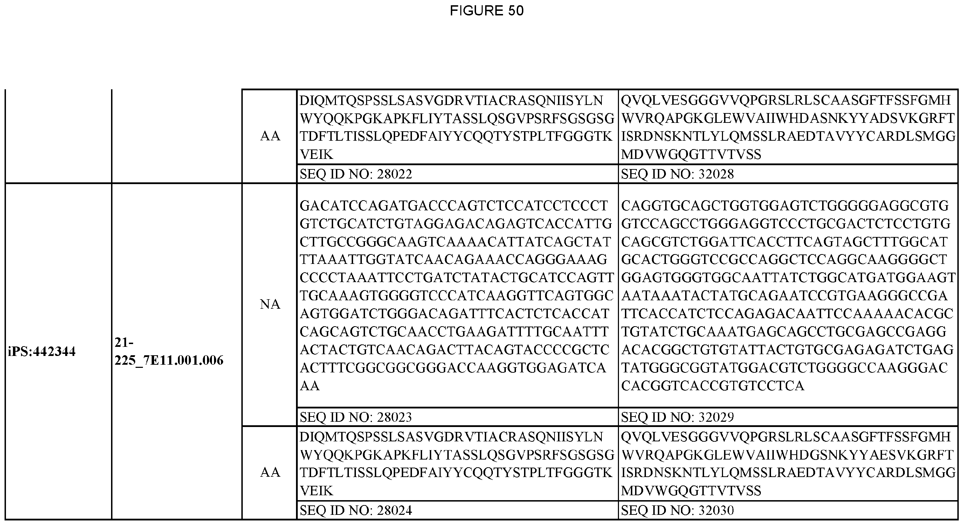

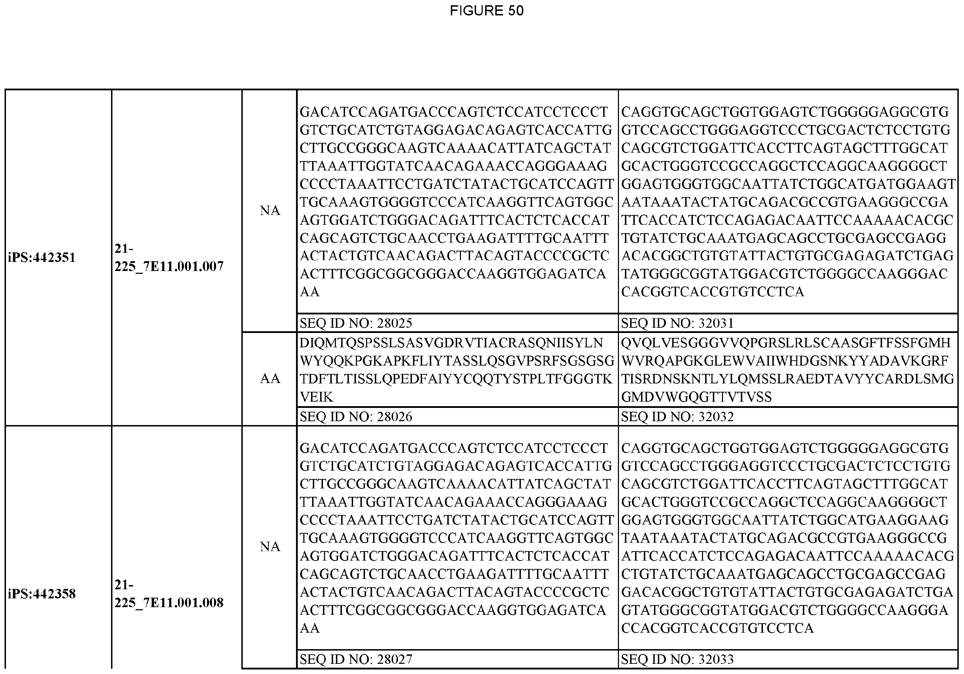

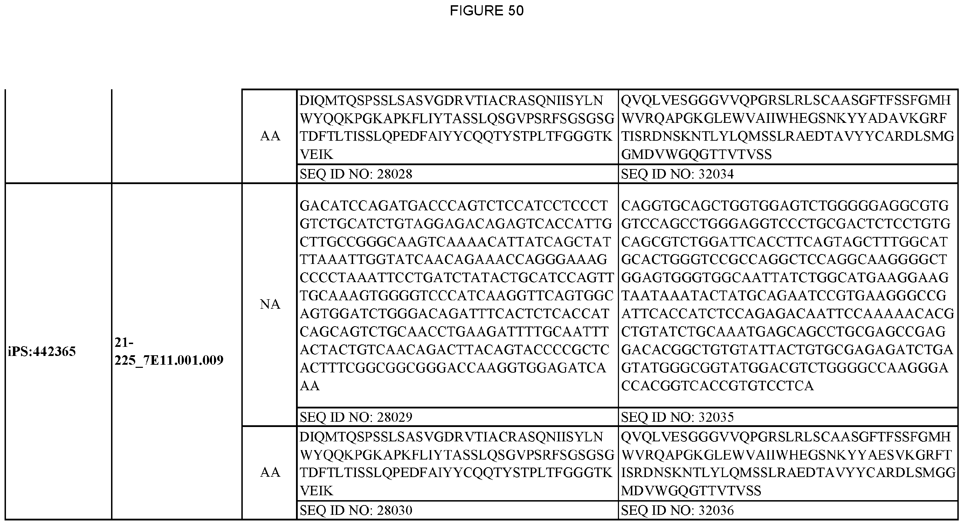

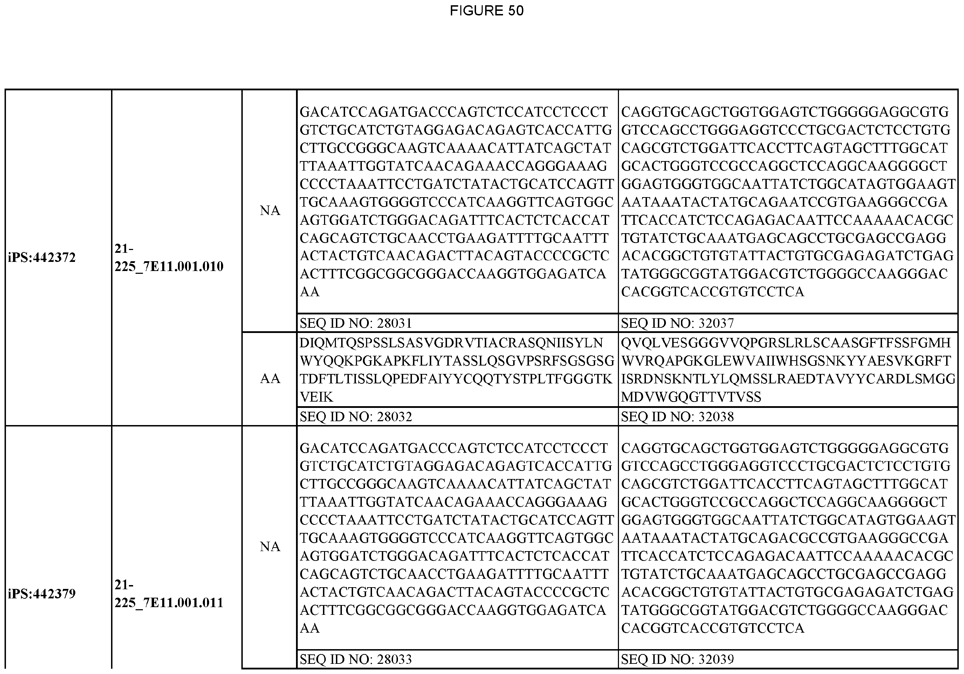

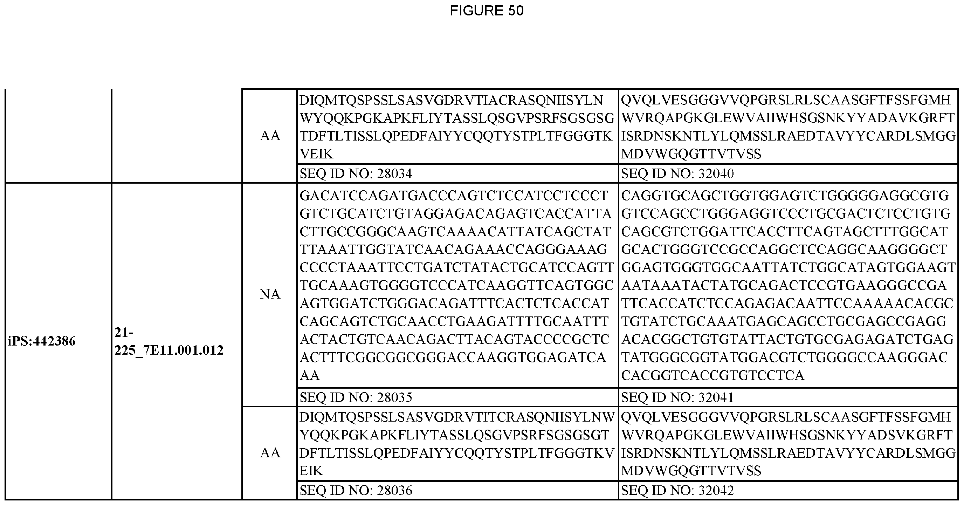

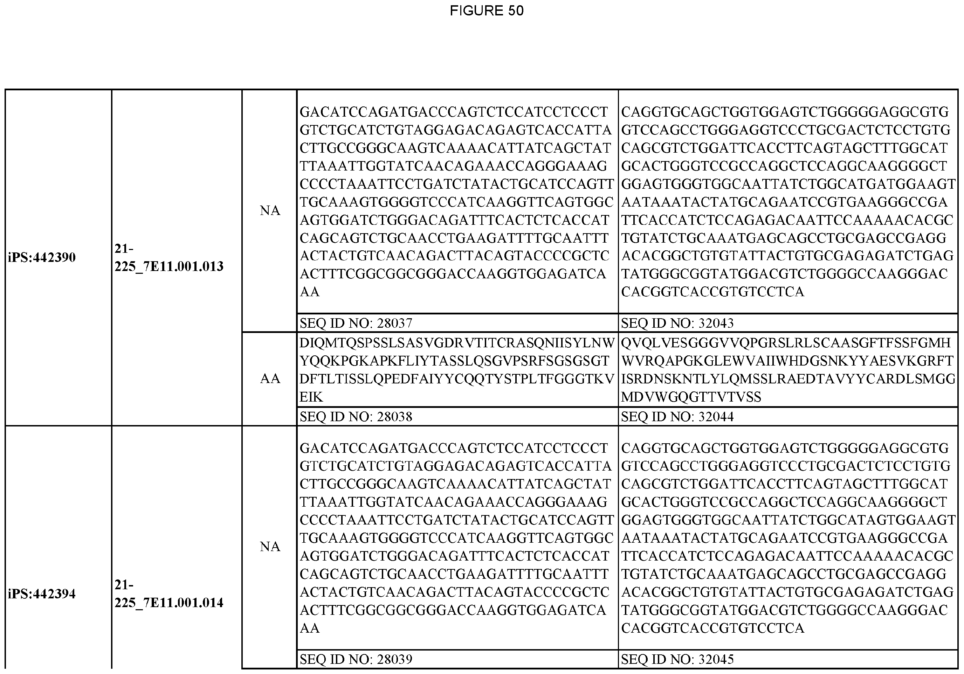

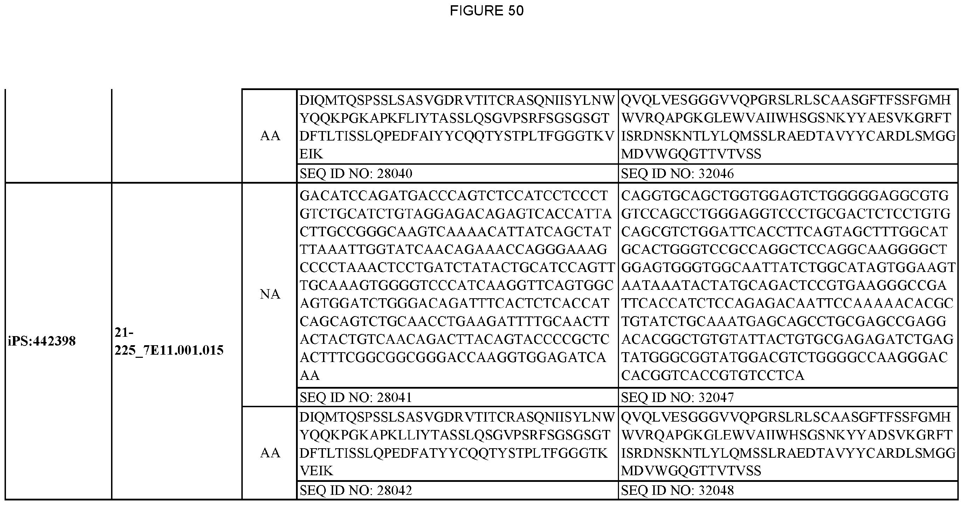

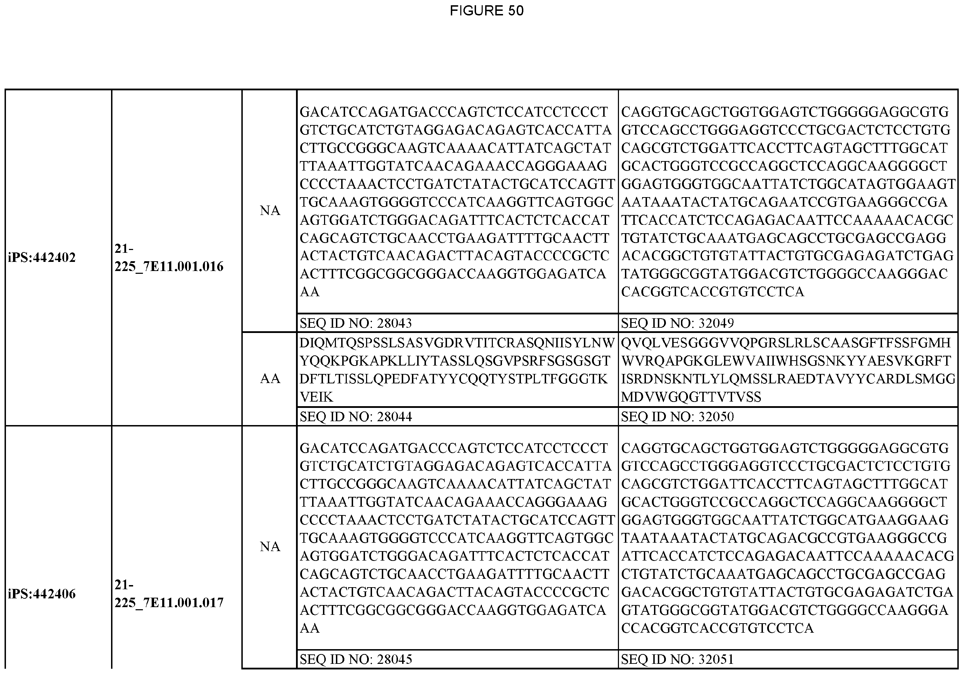

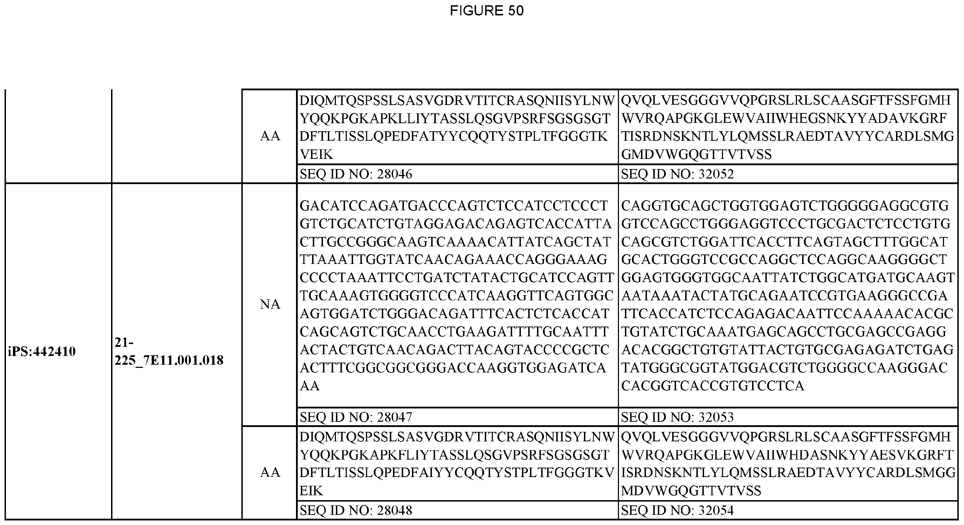

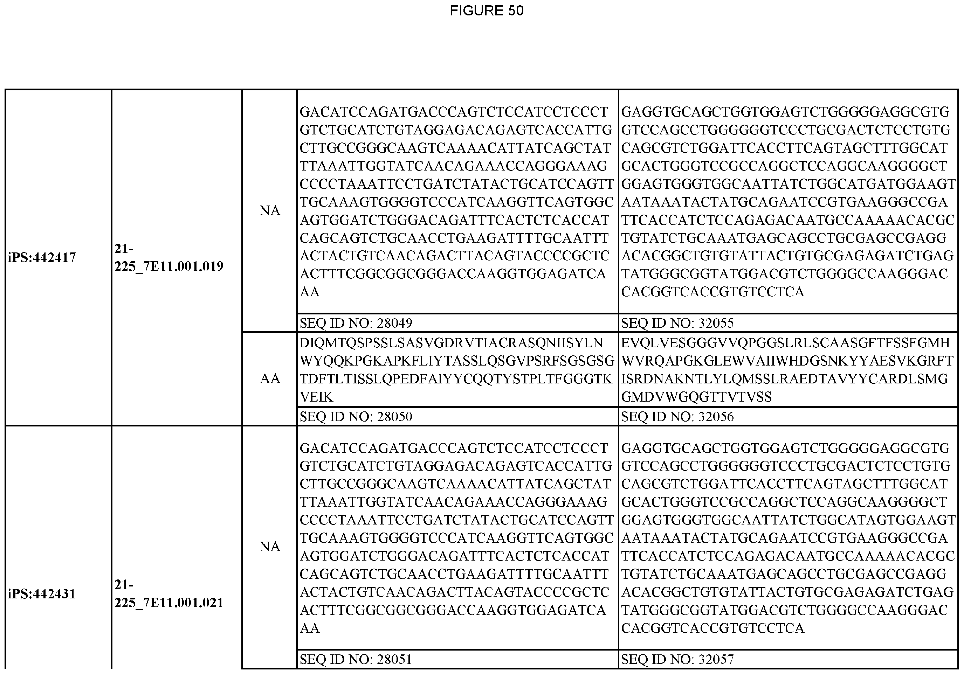

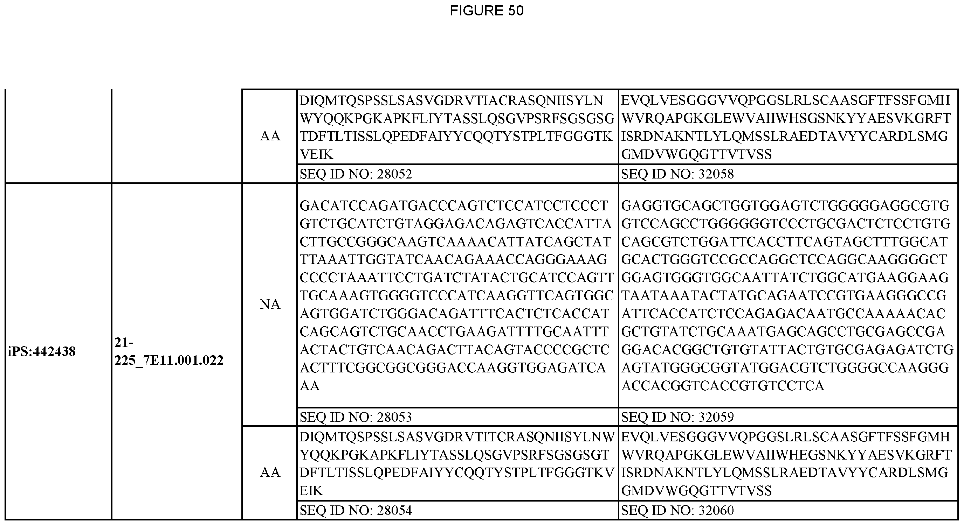

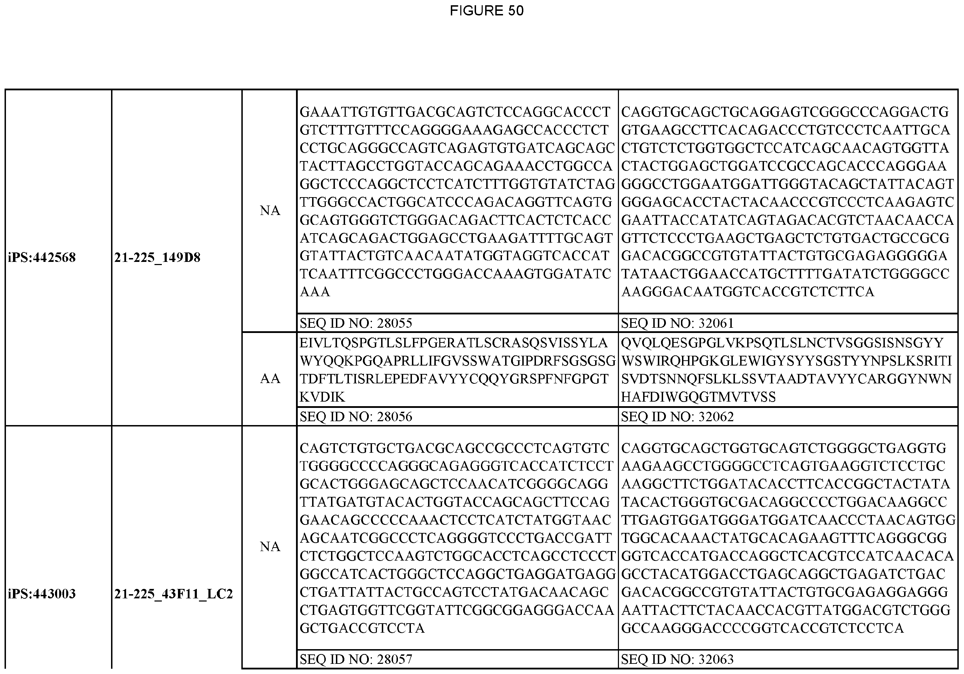

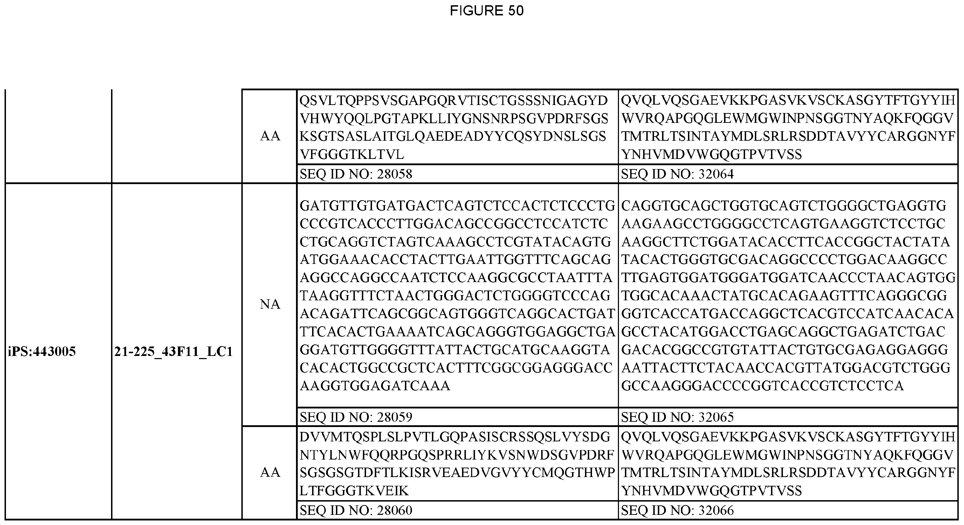

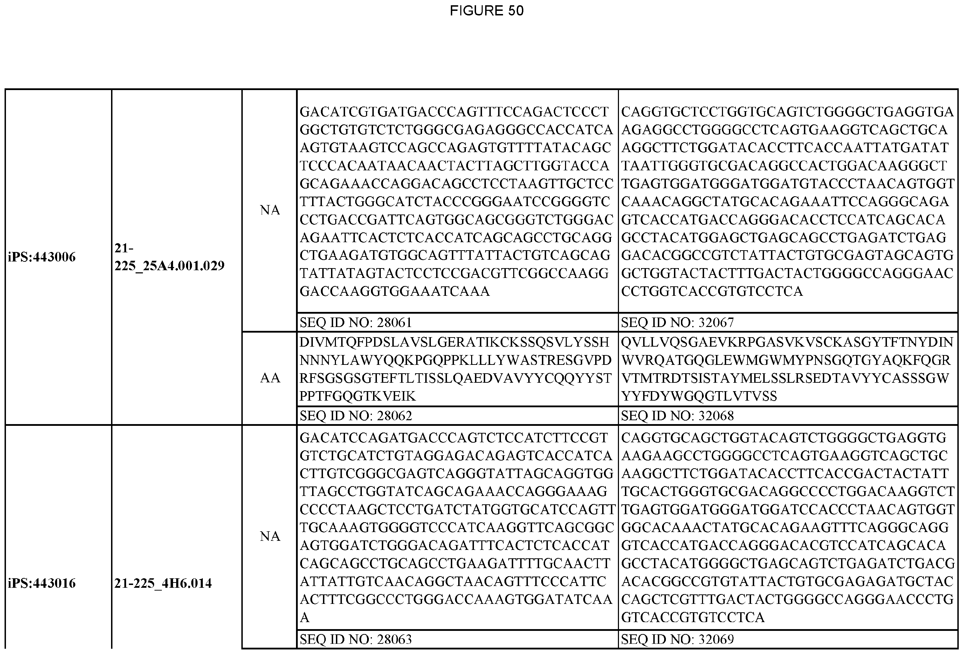

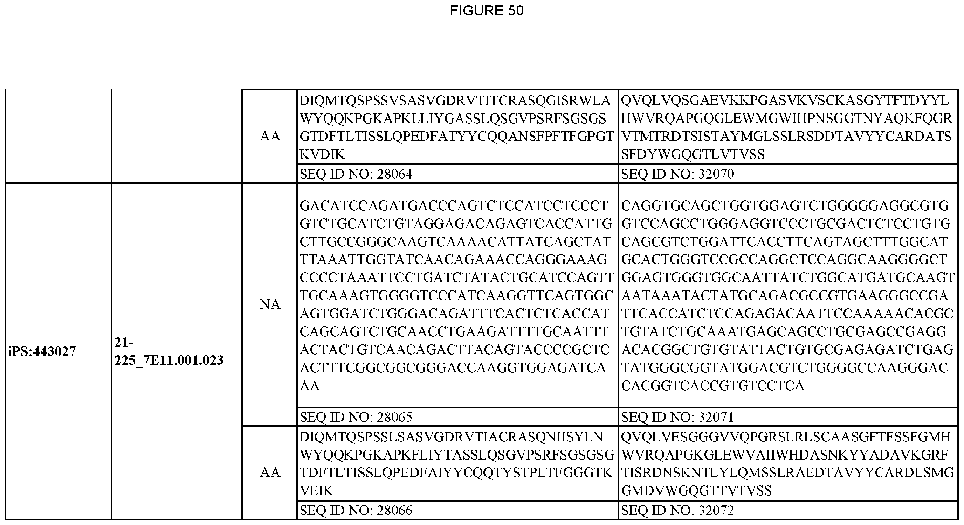

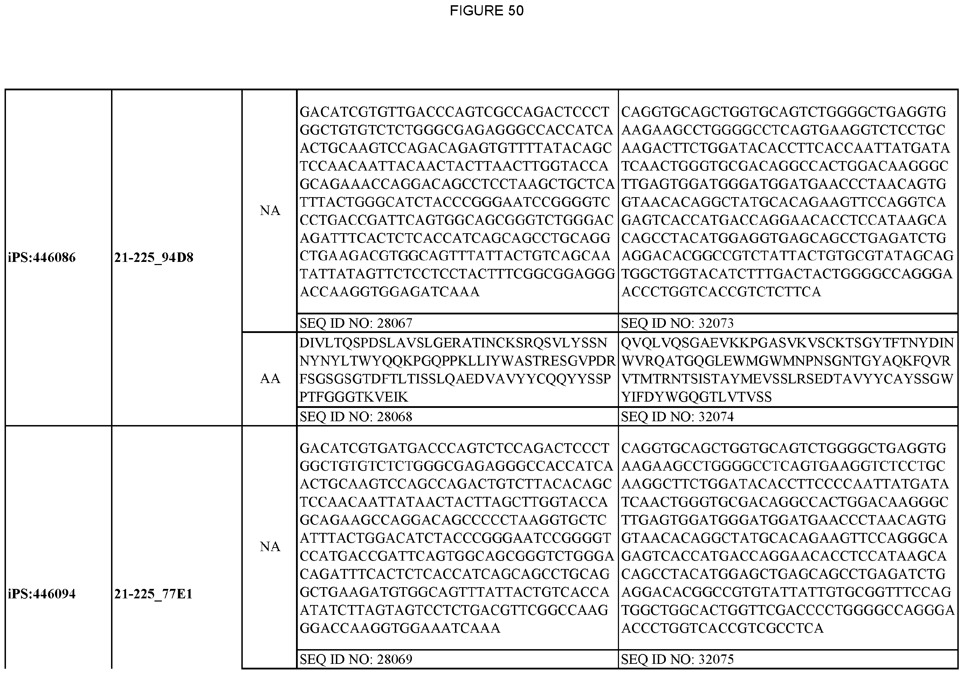

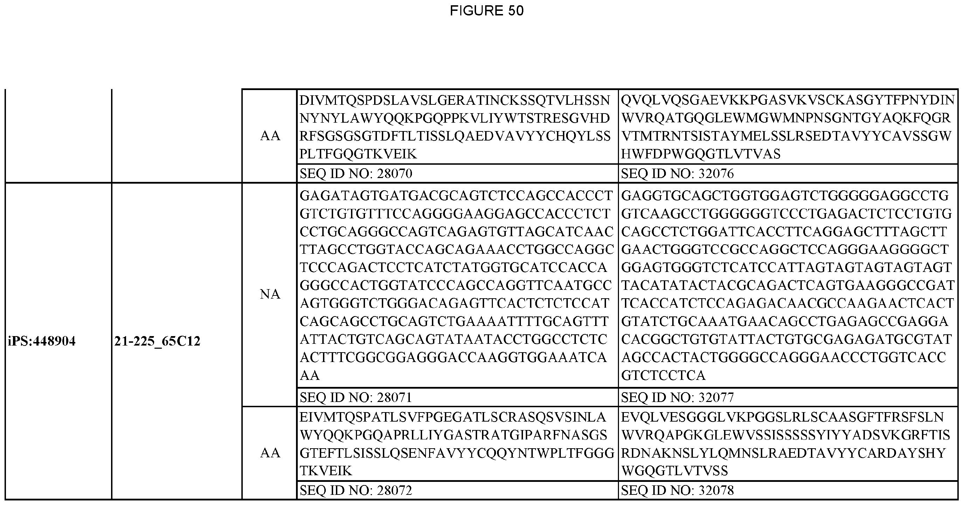

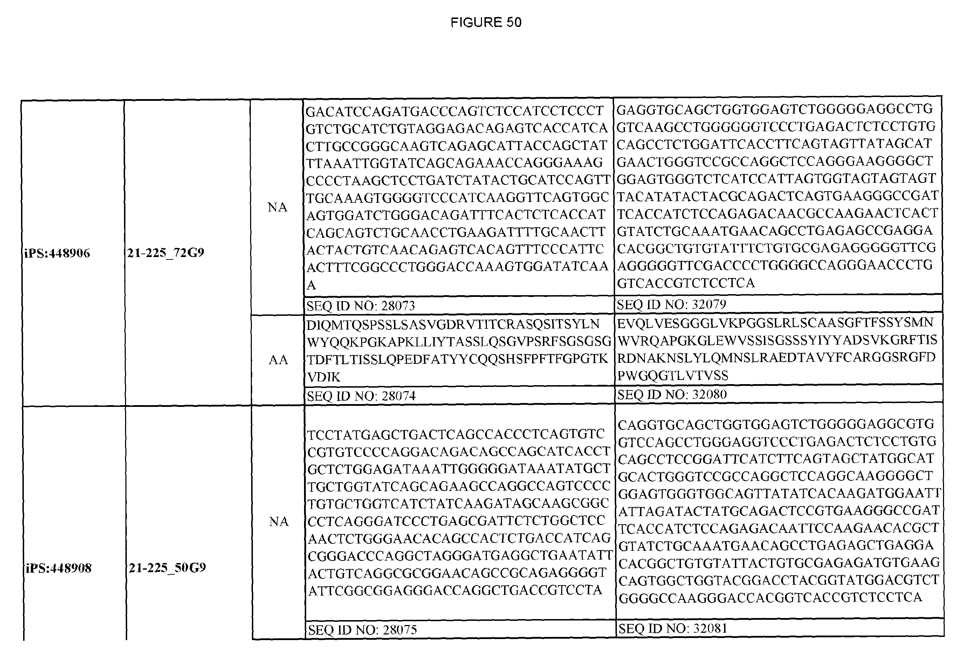

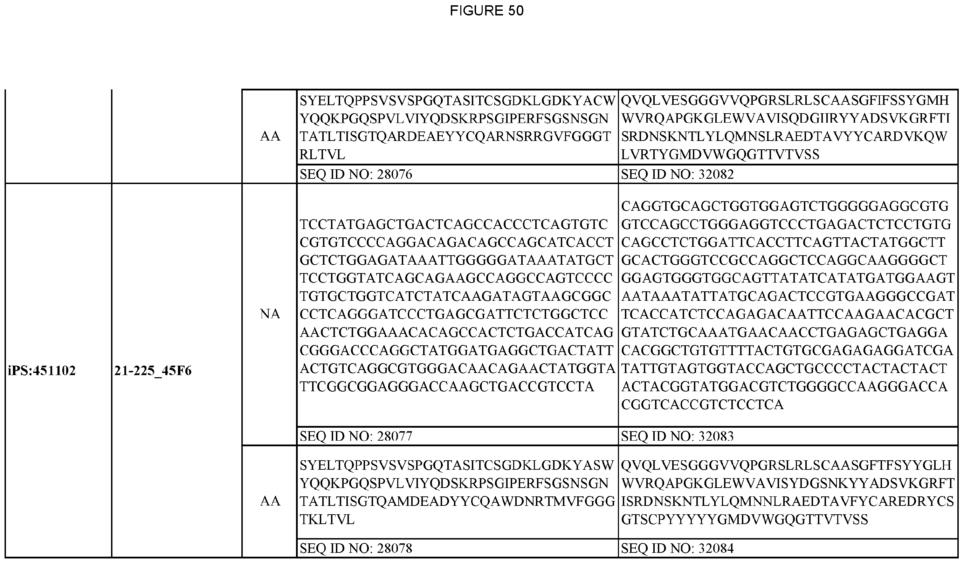

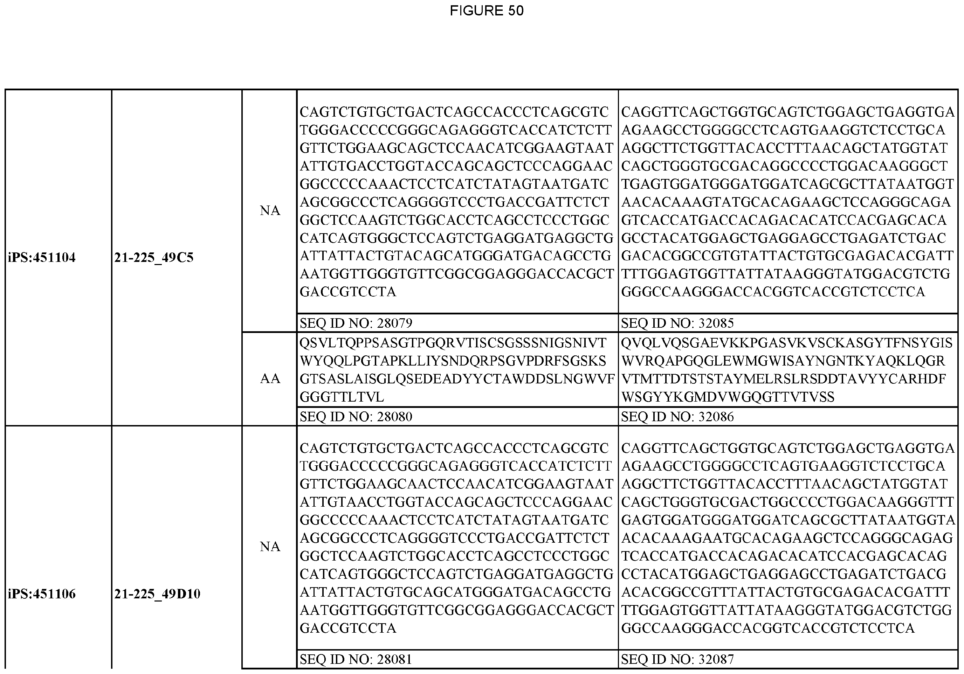

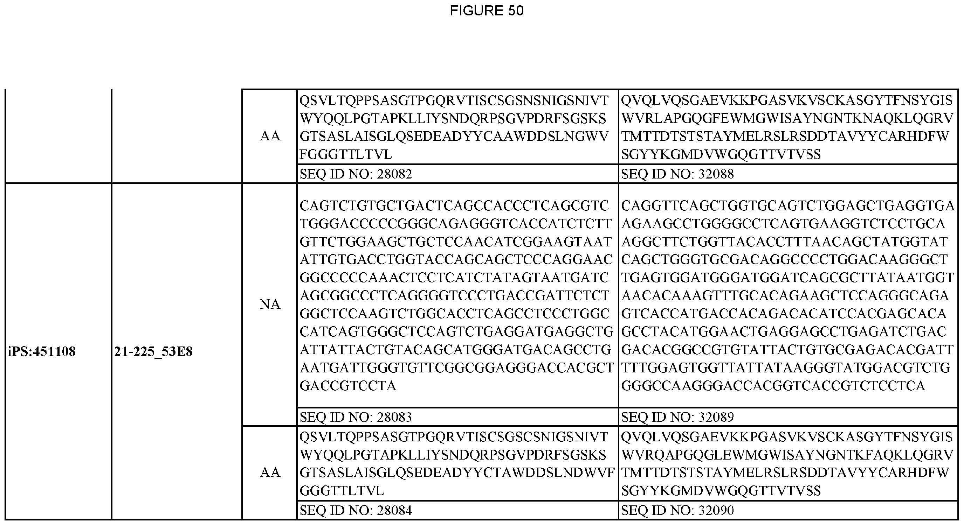

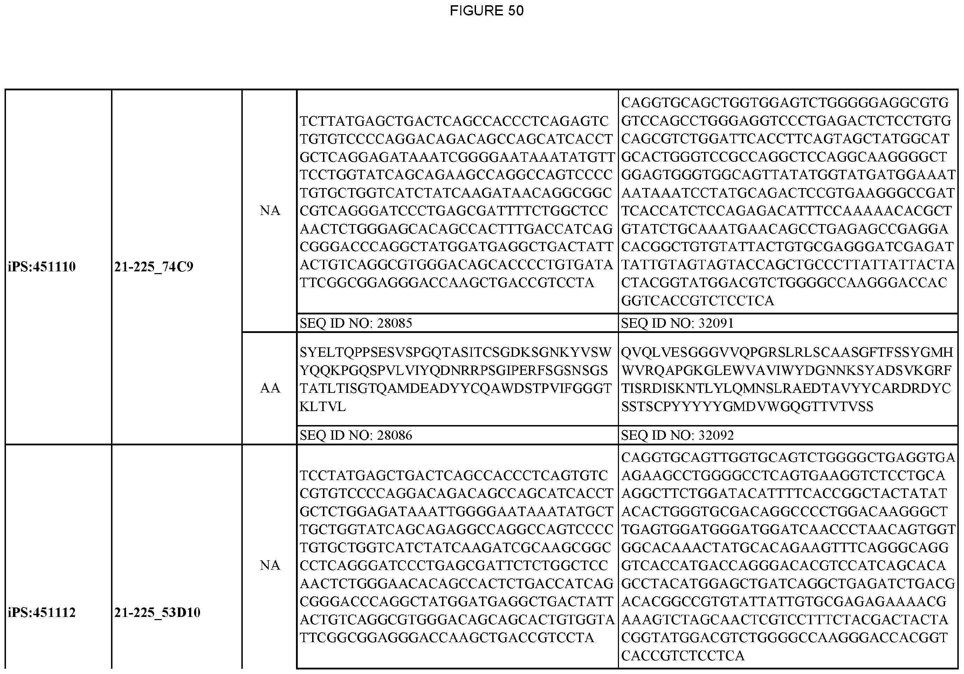

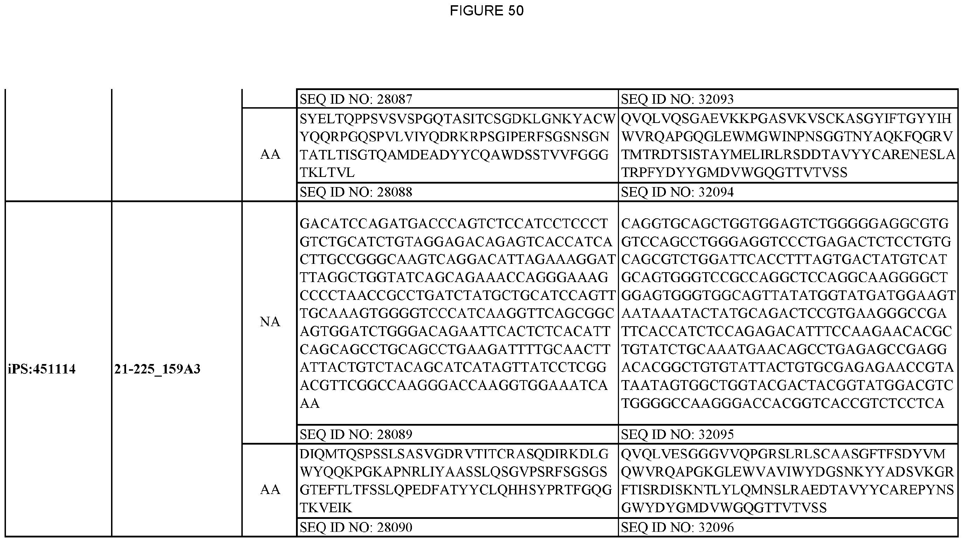

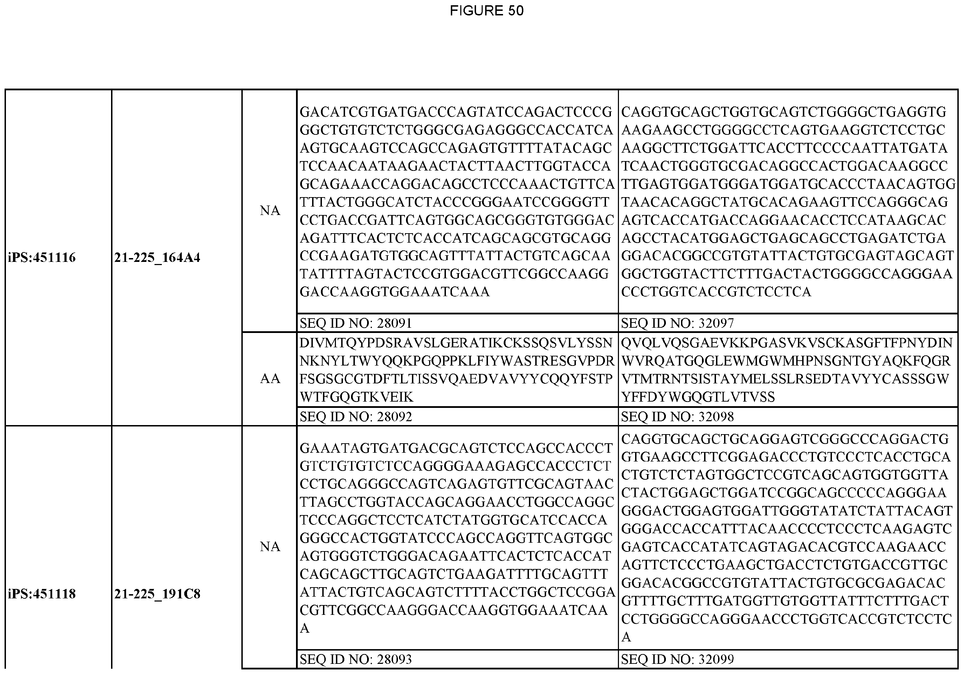

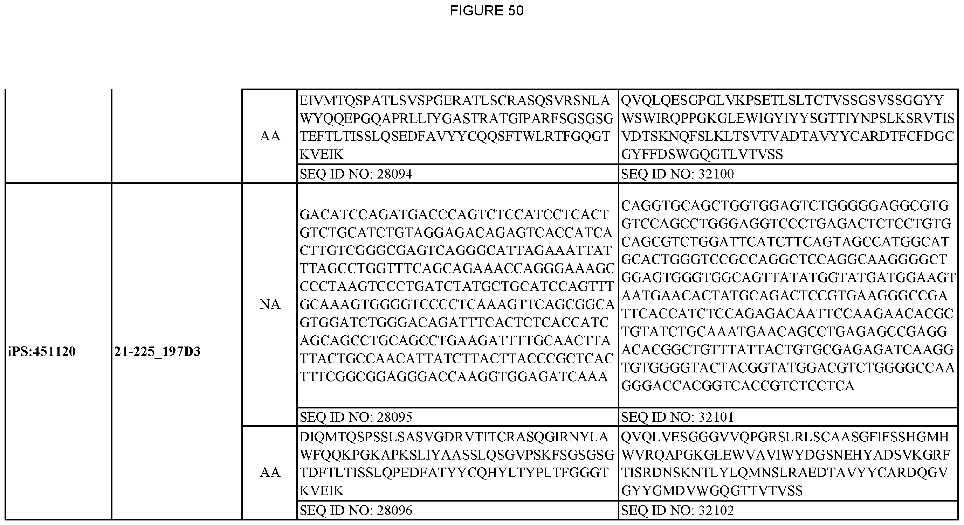

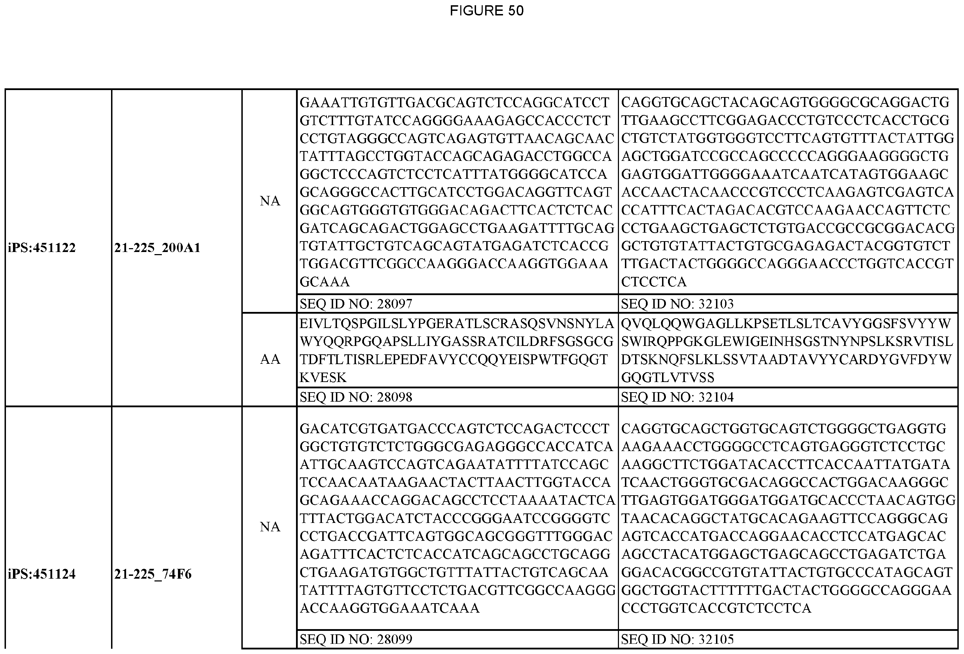

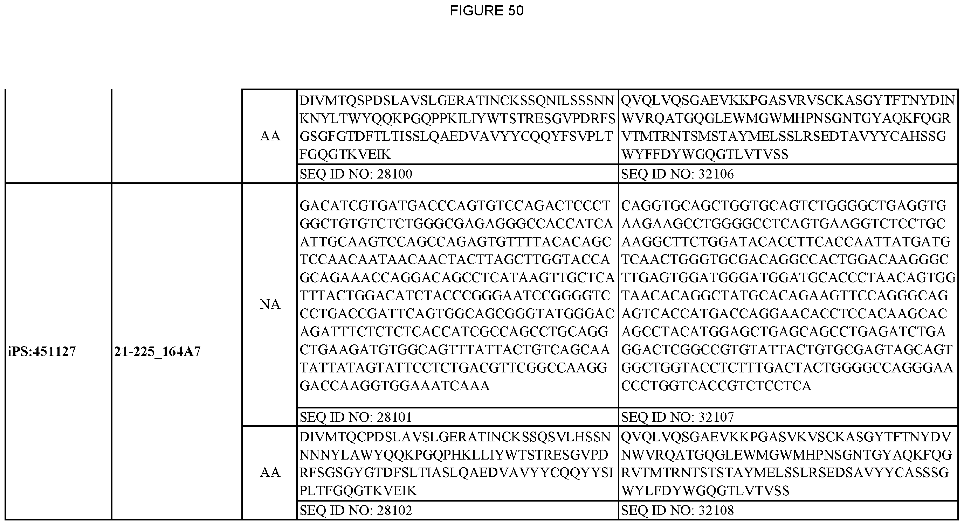

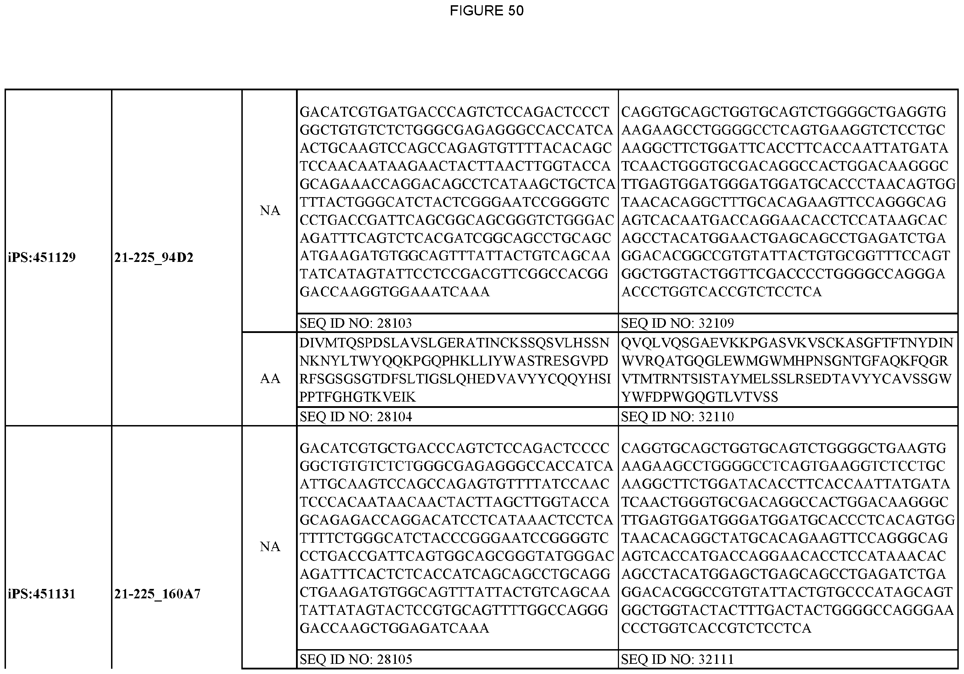

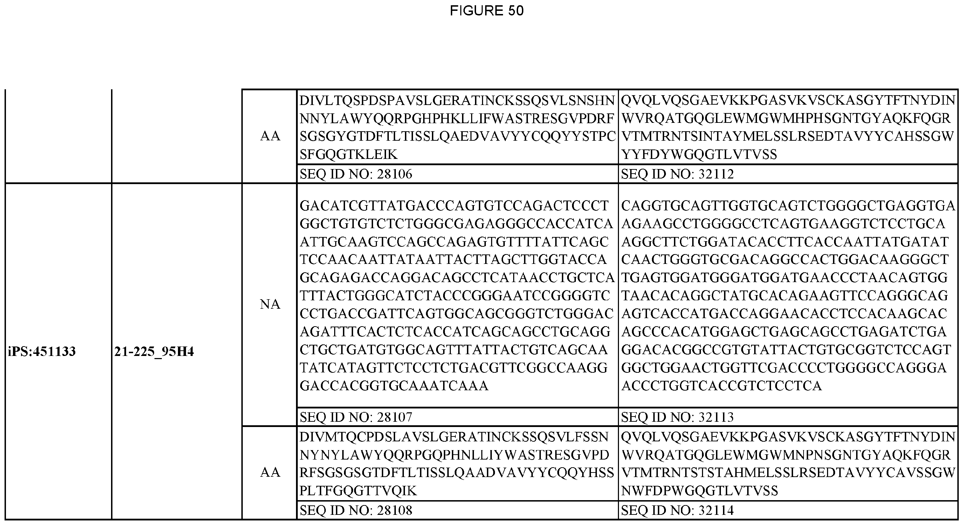

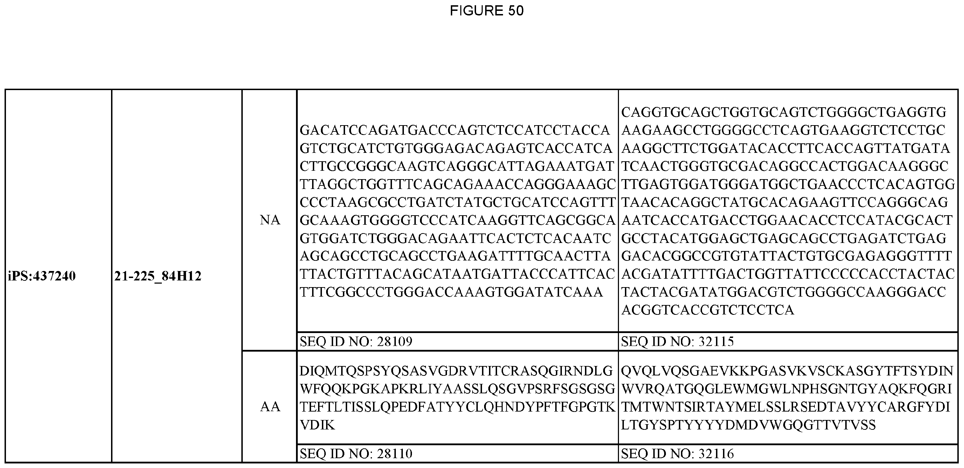

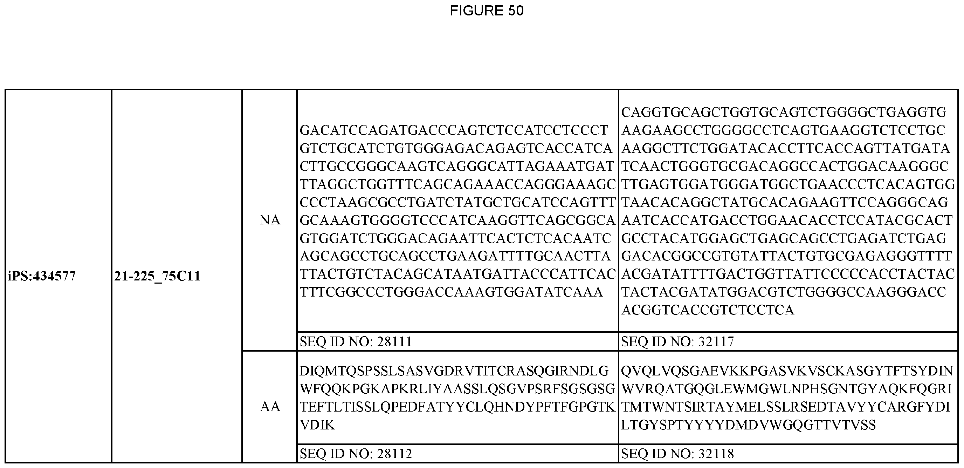

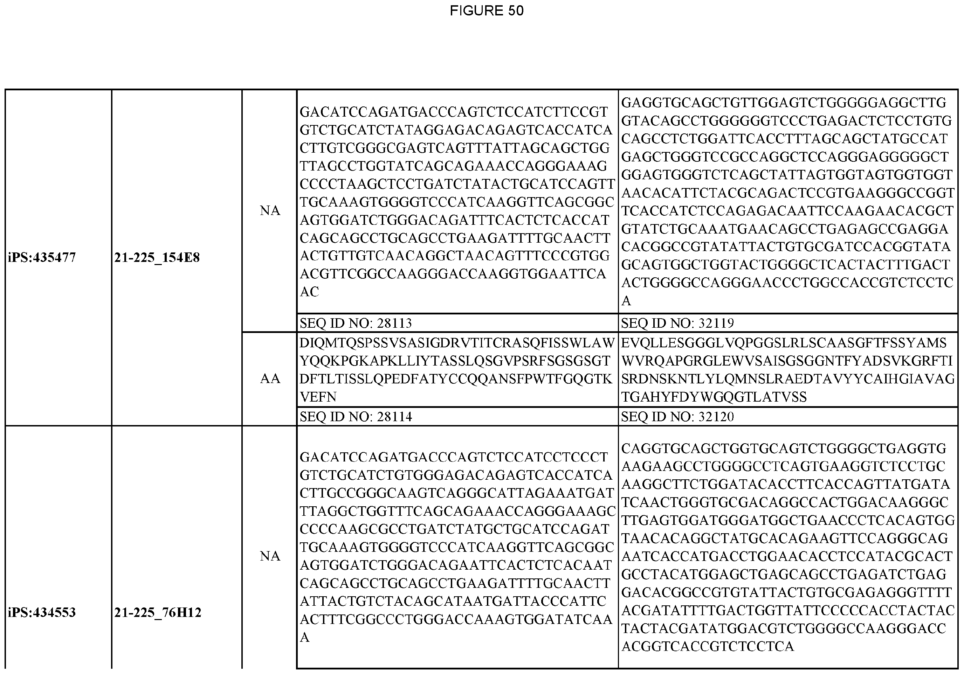

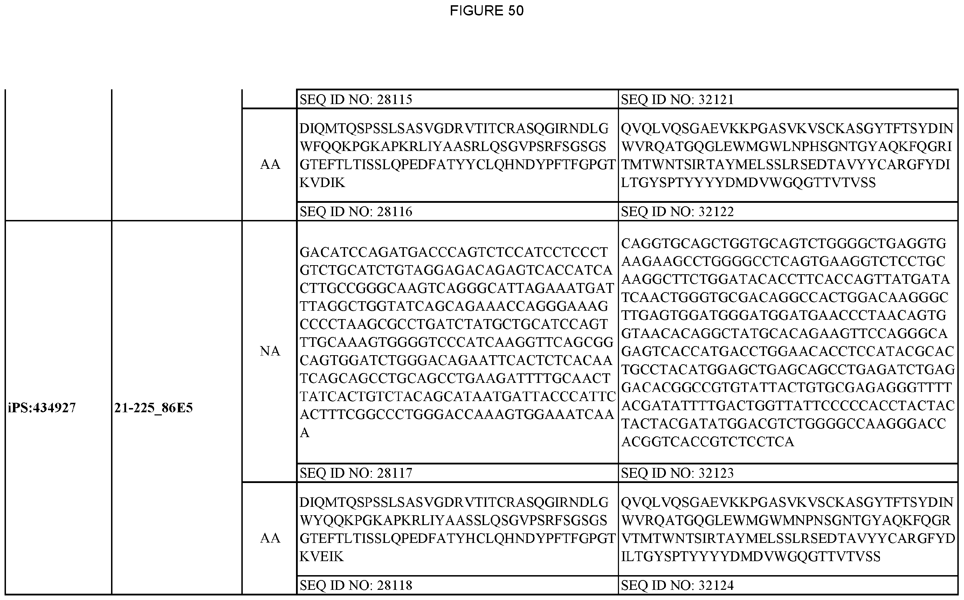

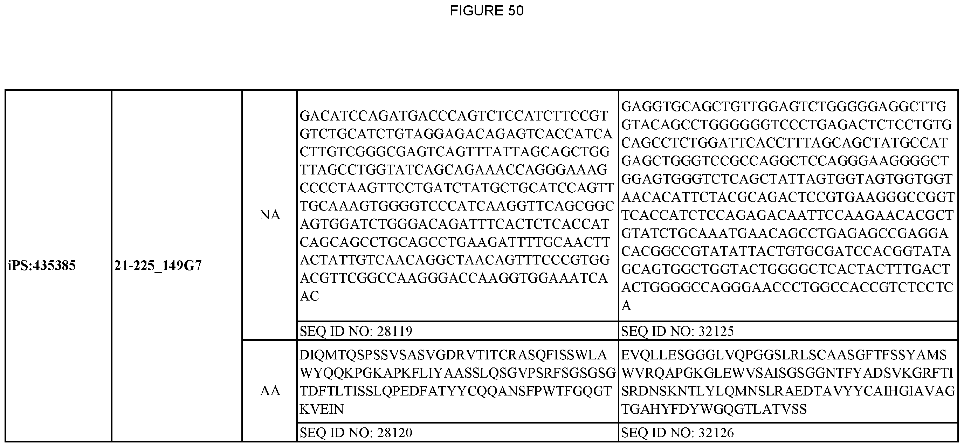

**Figure 50.** A table presenting the amino acid sequences of the light and heavy chain variable domains for certain antigen binding proteins are displayed in a table (Table 3). The amino acid sequences of the light and heavy chain variable domains in Table 3 are wrapped due to space issues, and unless stated otherwise, should be understood to be single amino acid sequences.

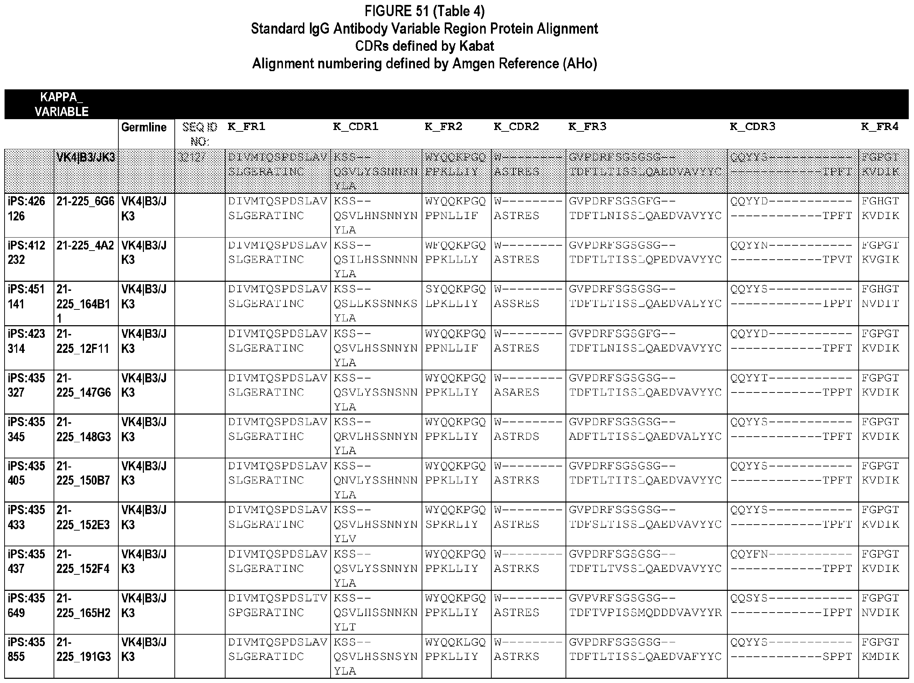

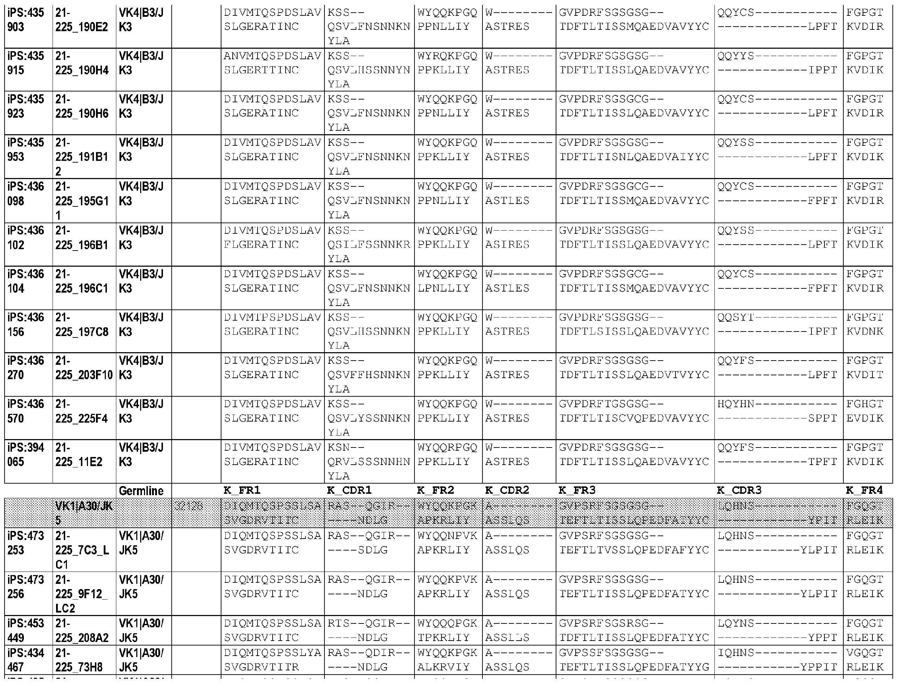

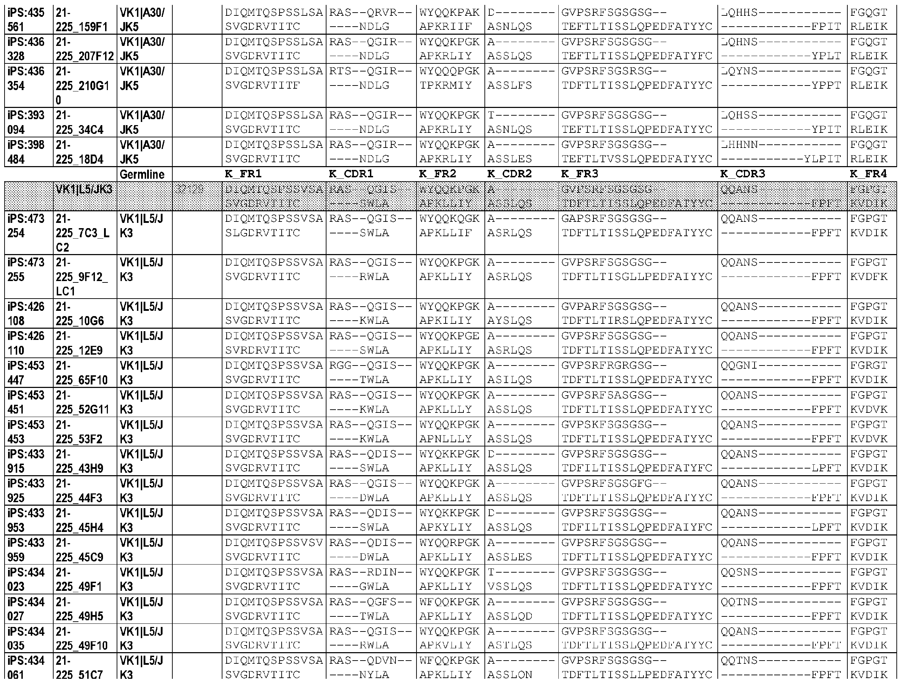

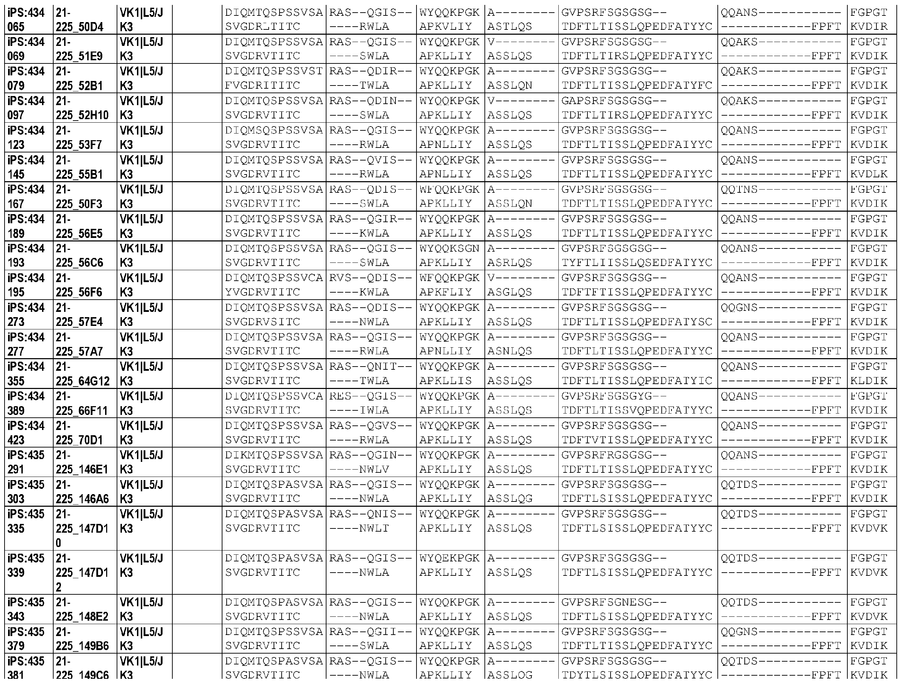

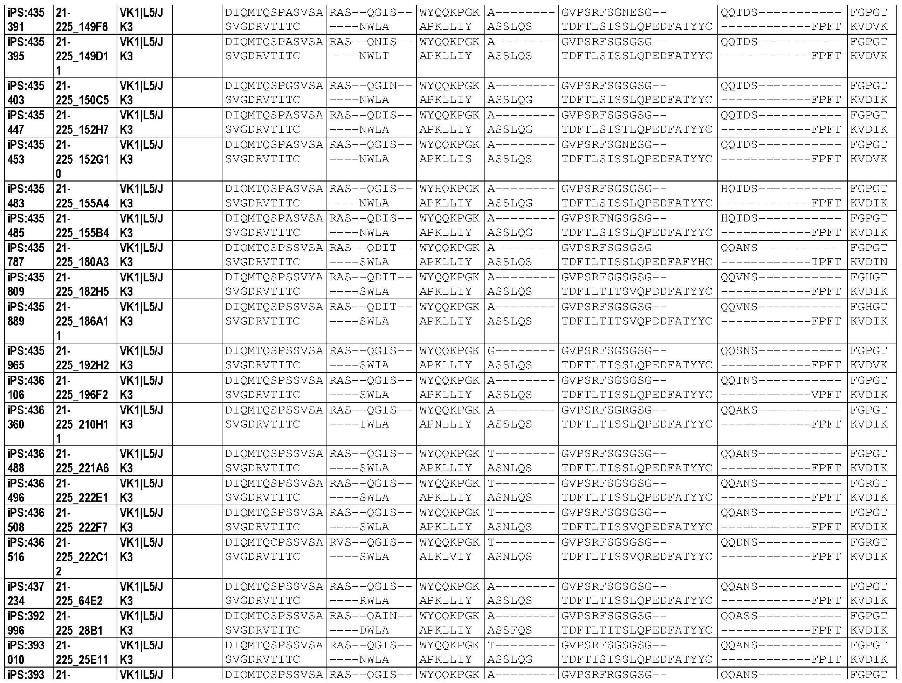

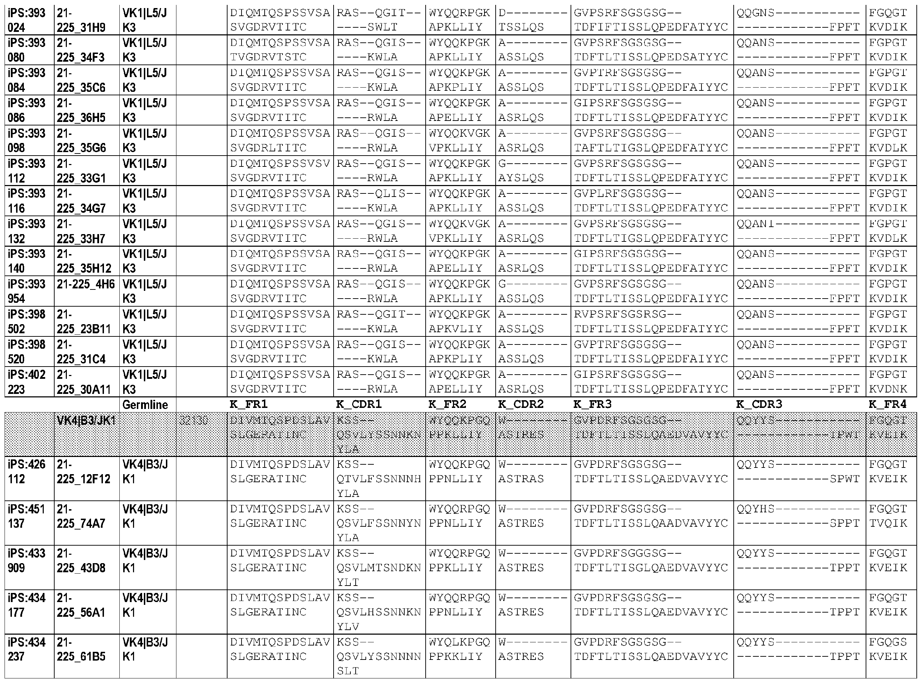

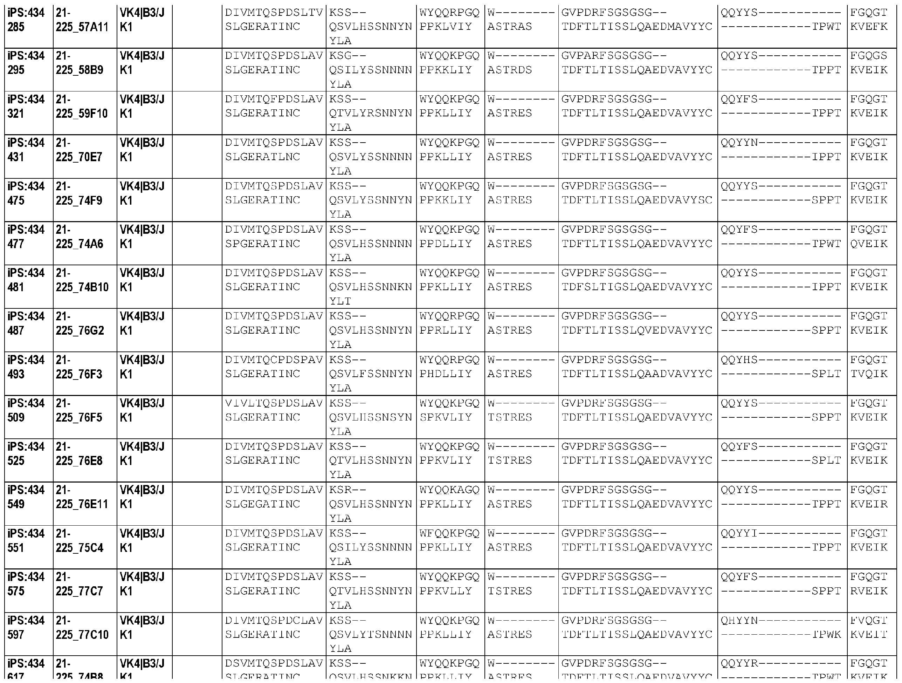

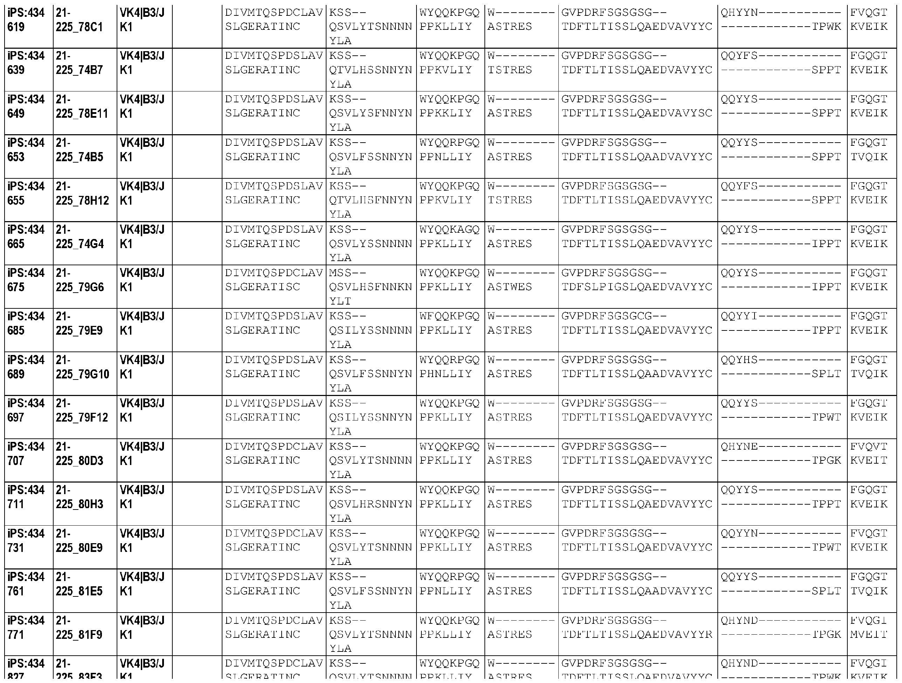

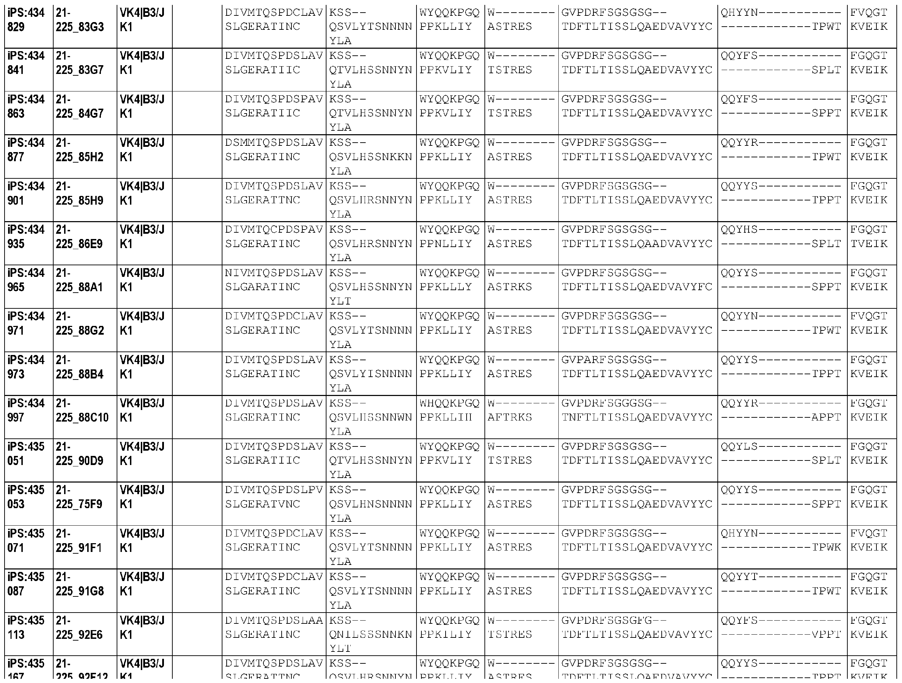

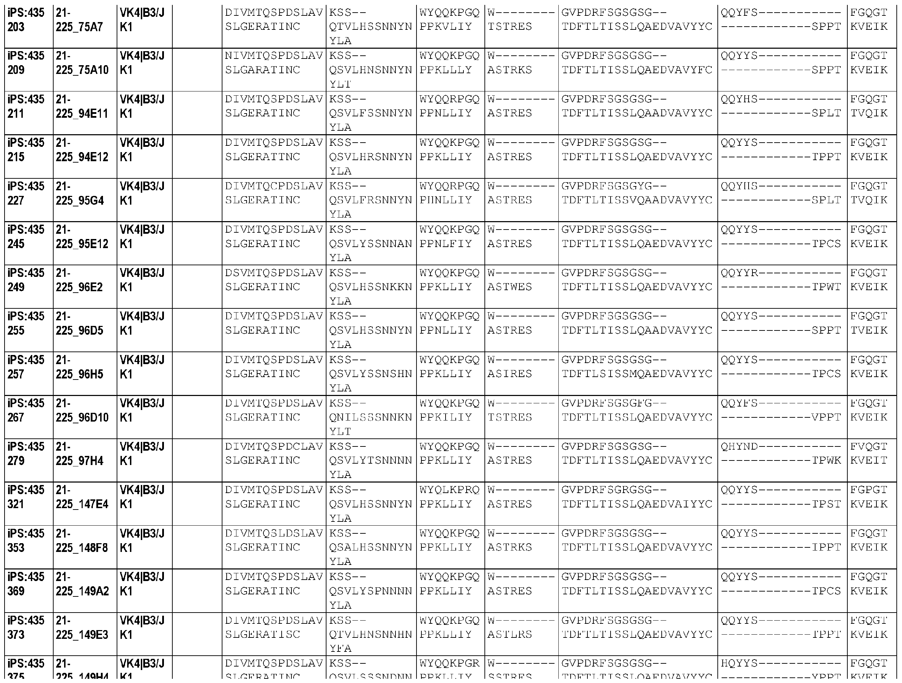

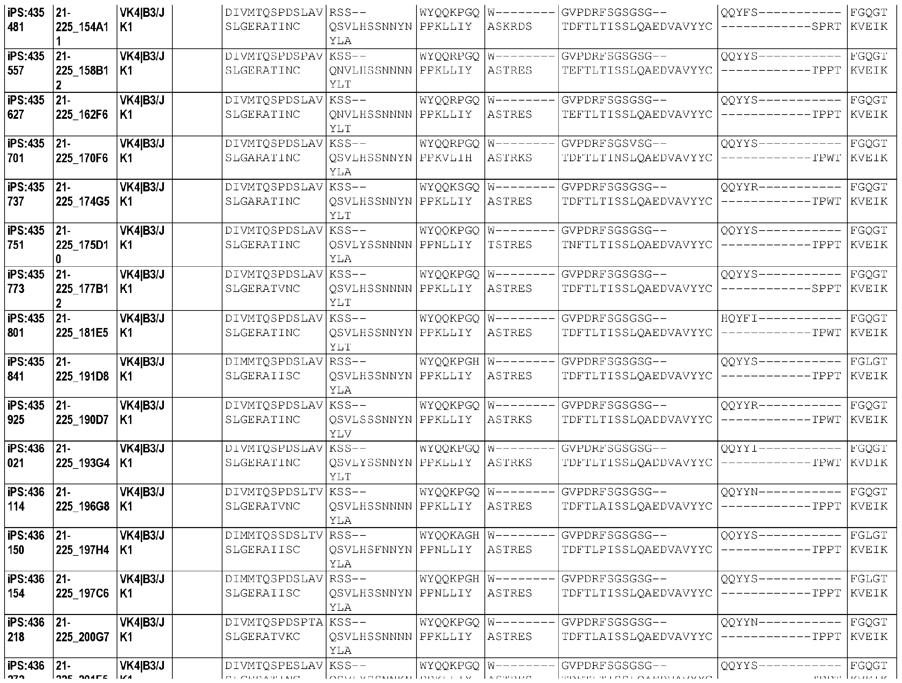

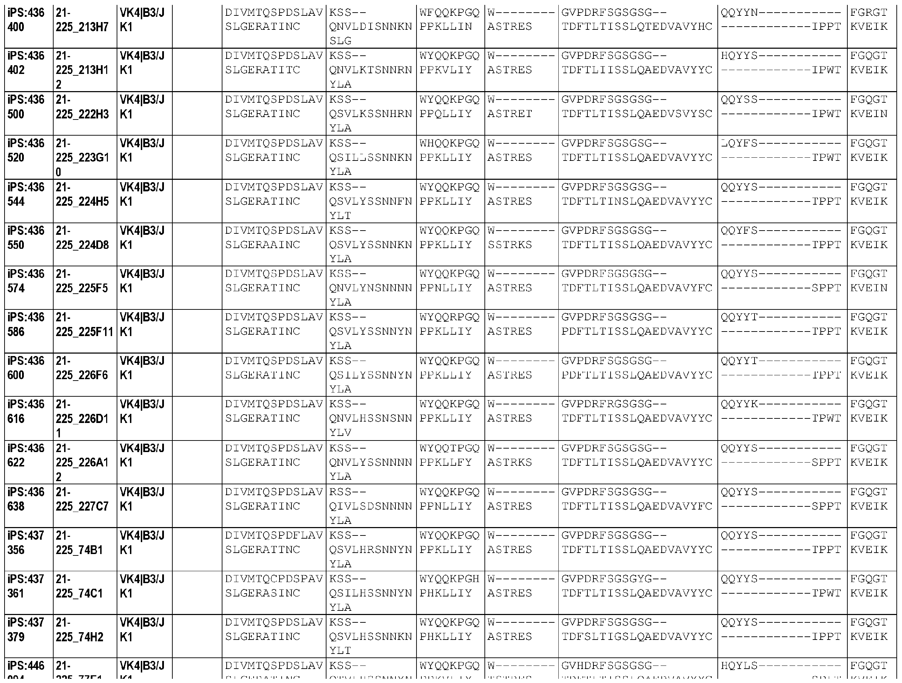

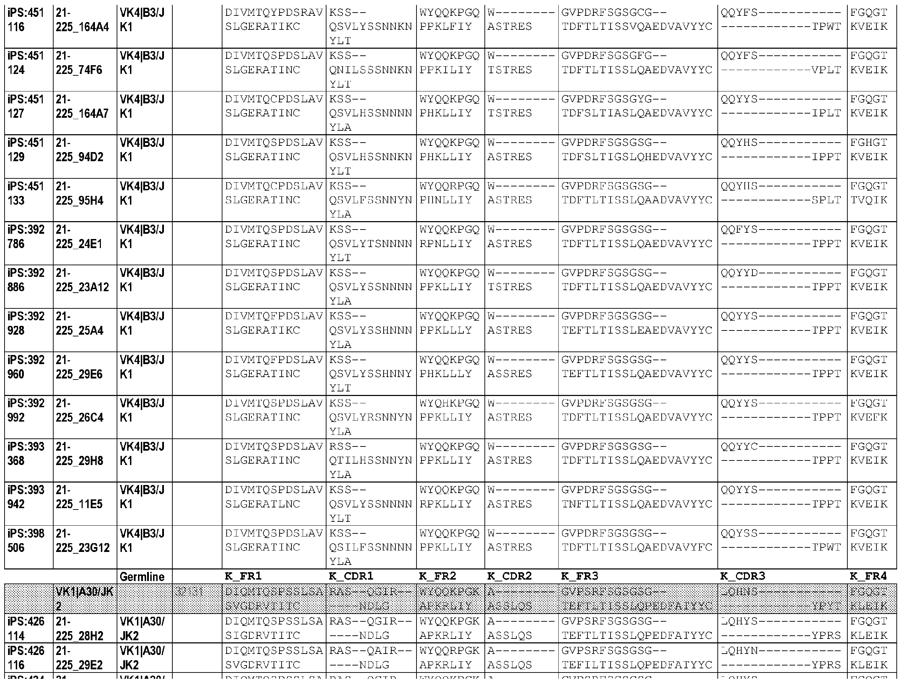

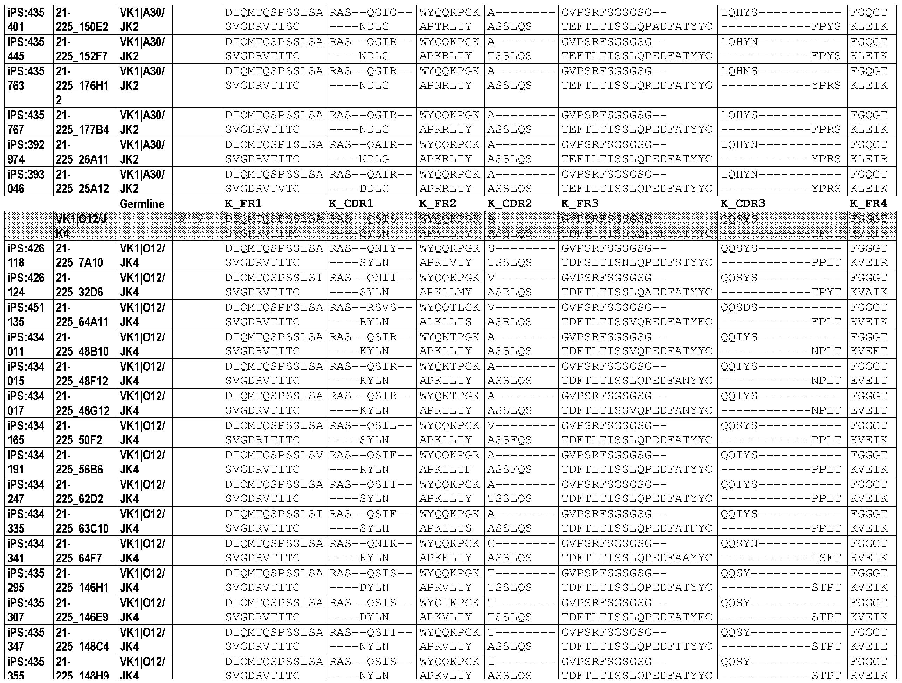

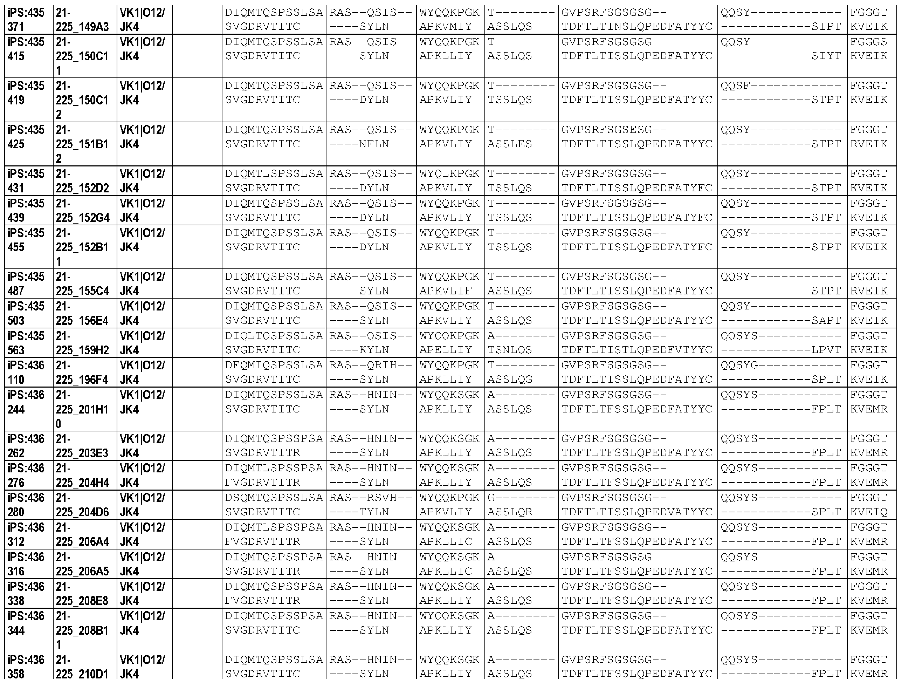

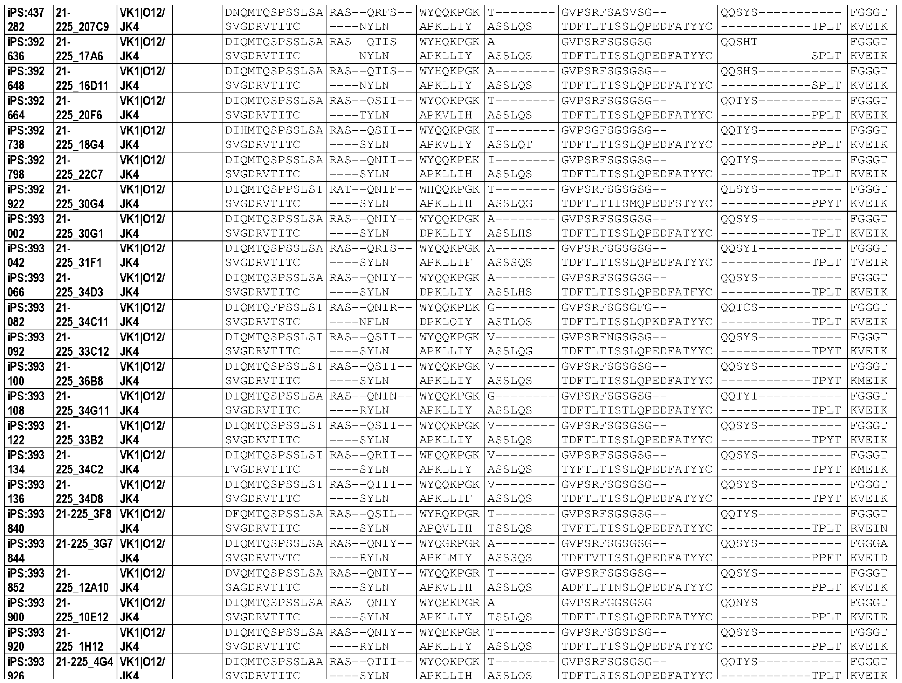

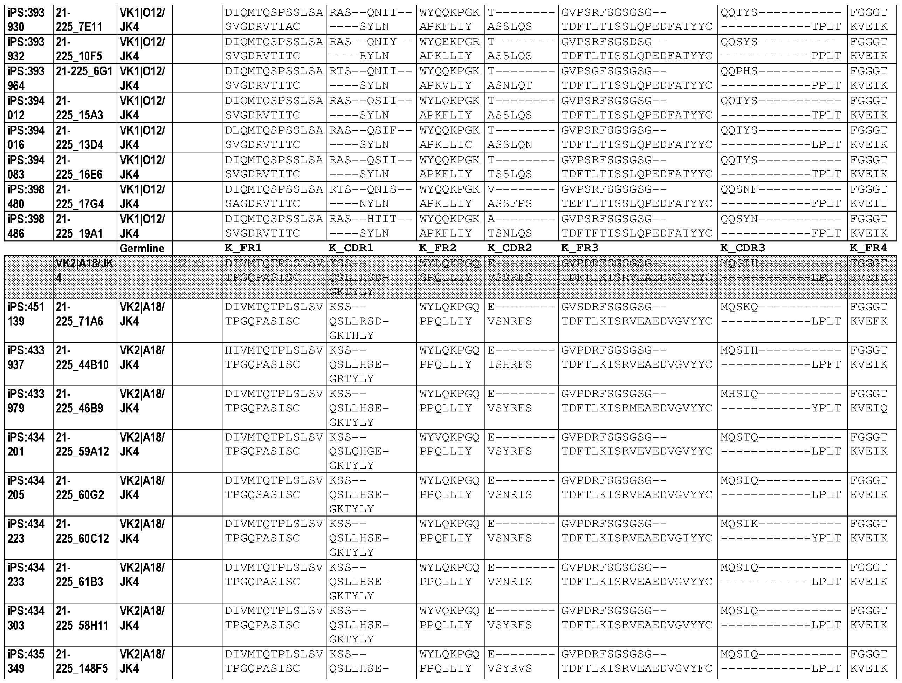

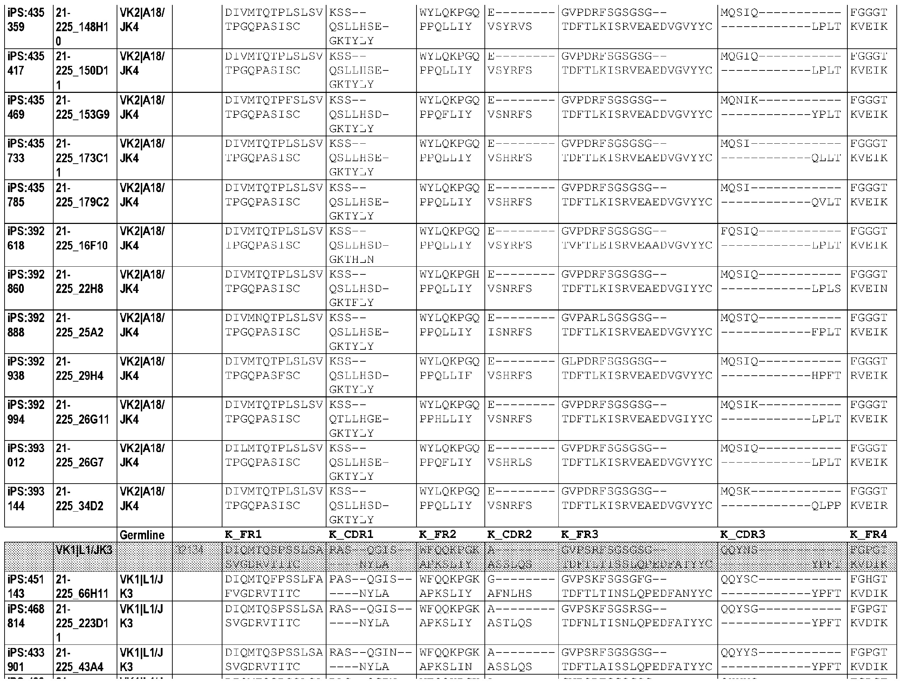

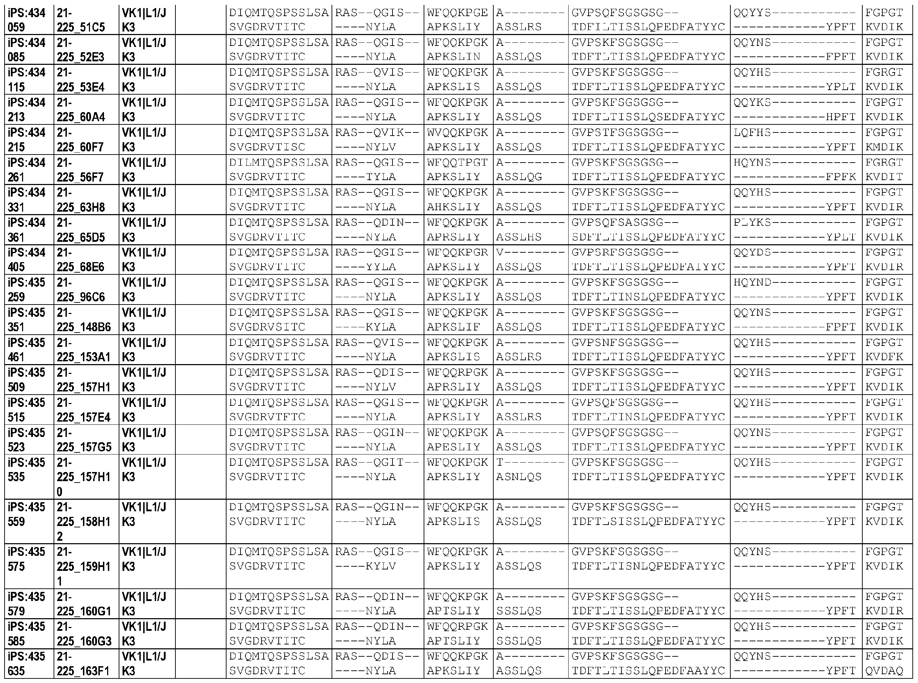

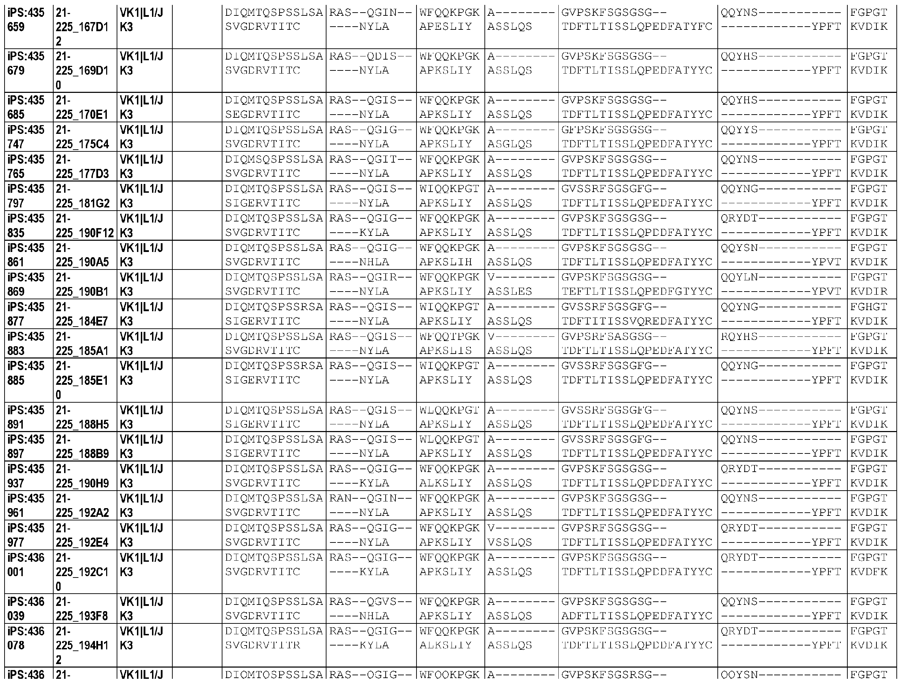

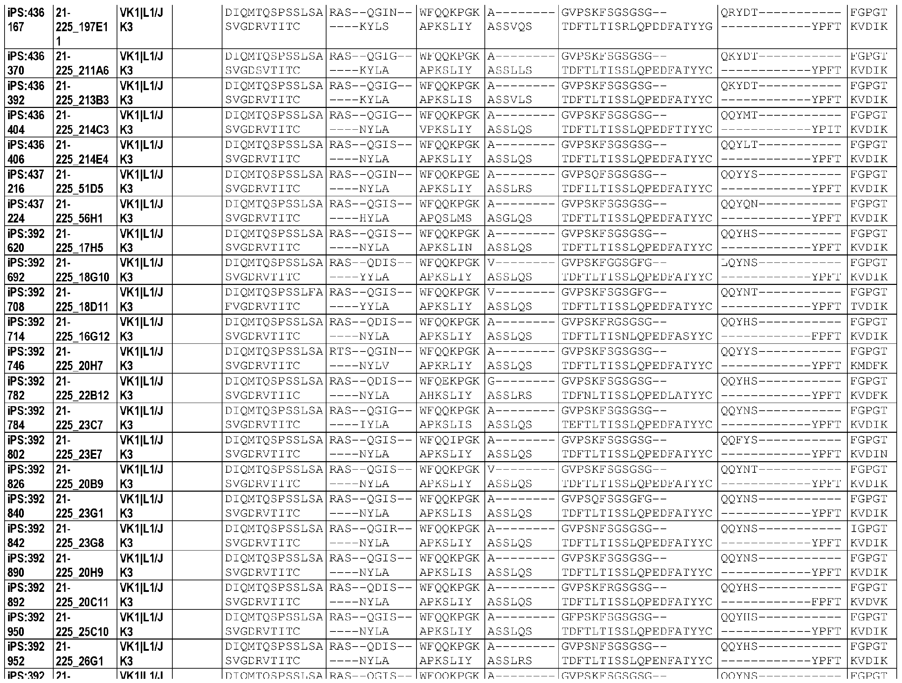

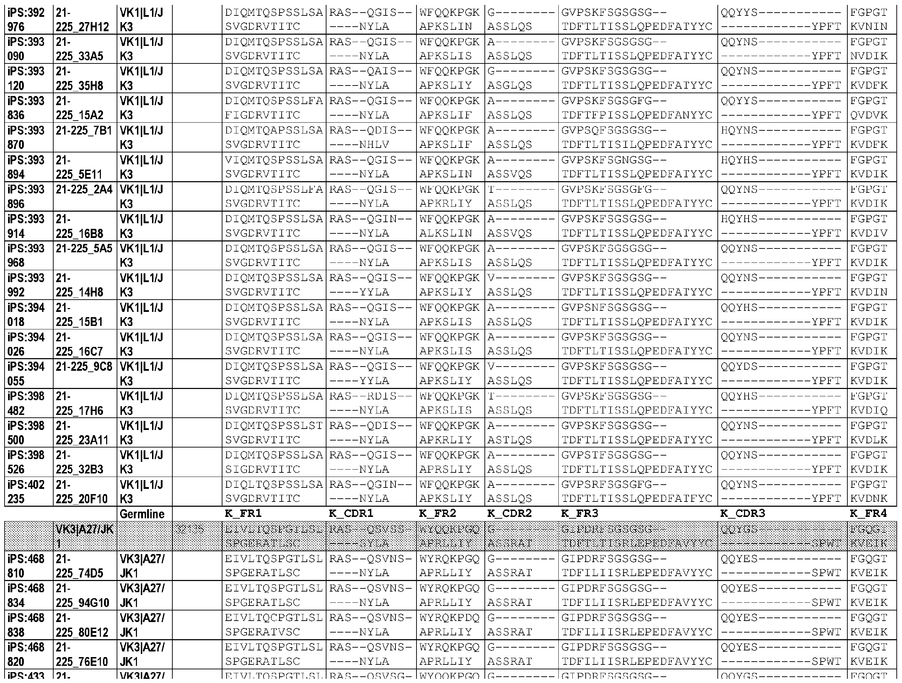

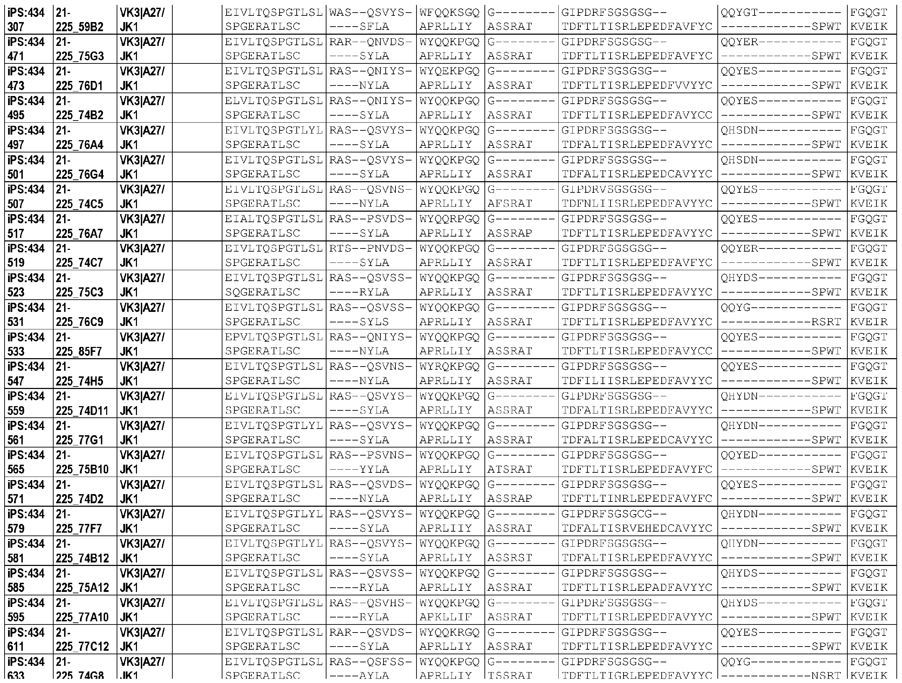

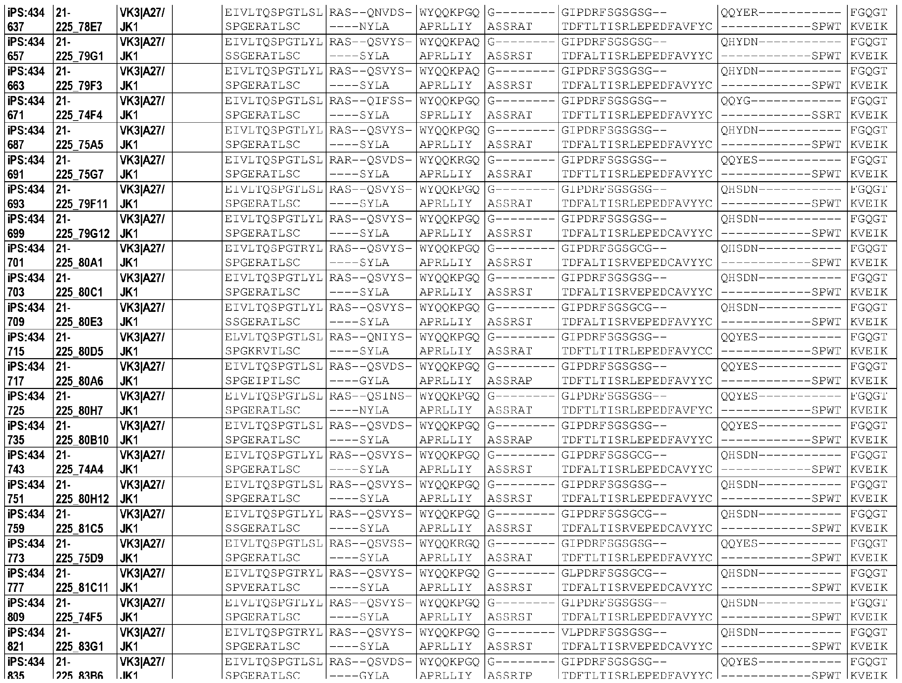

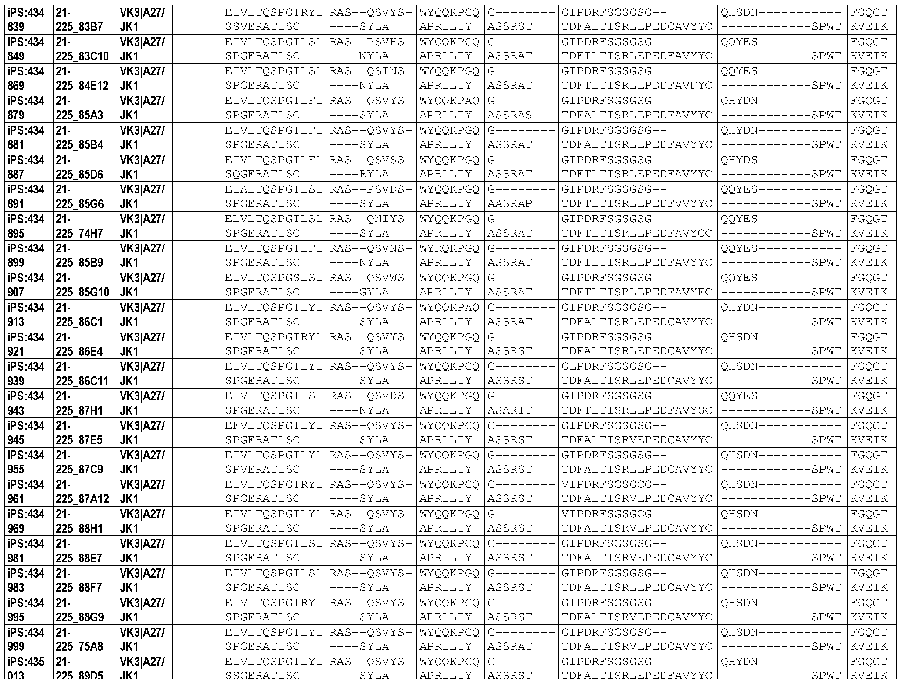

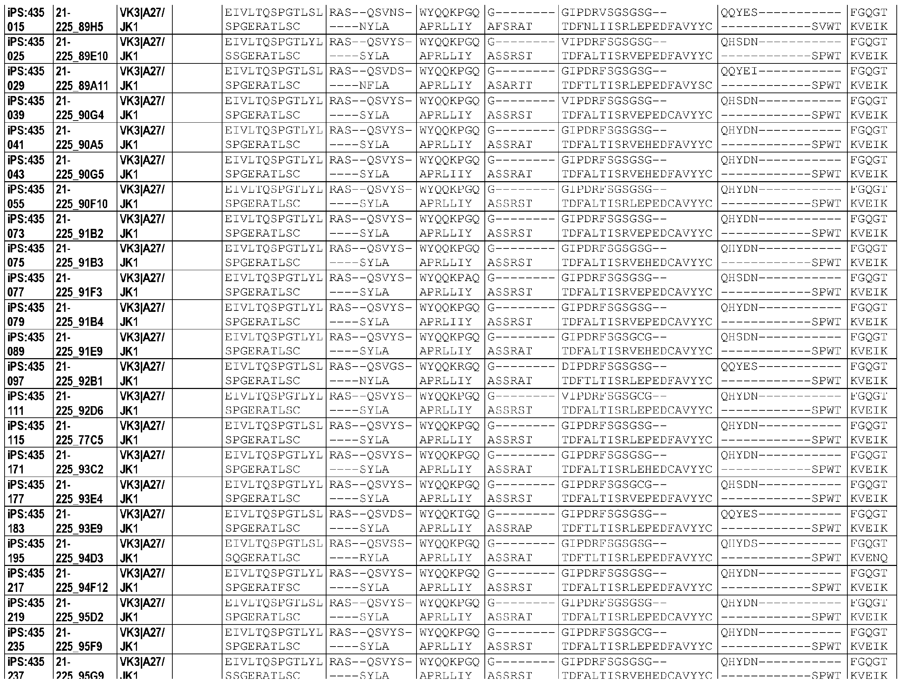

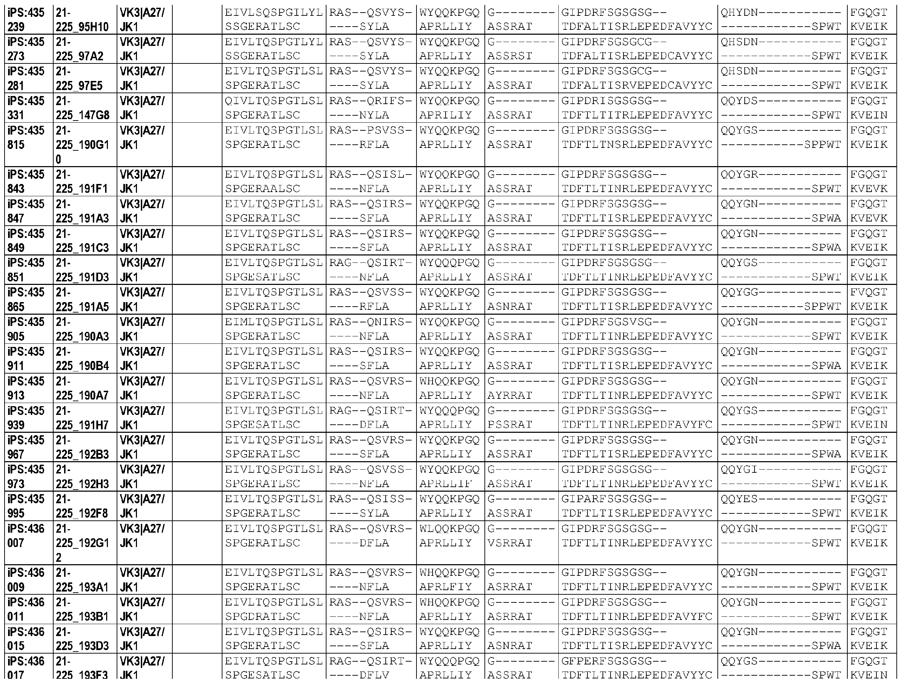

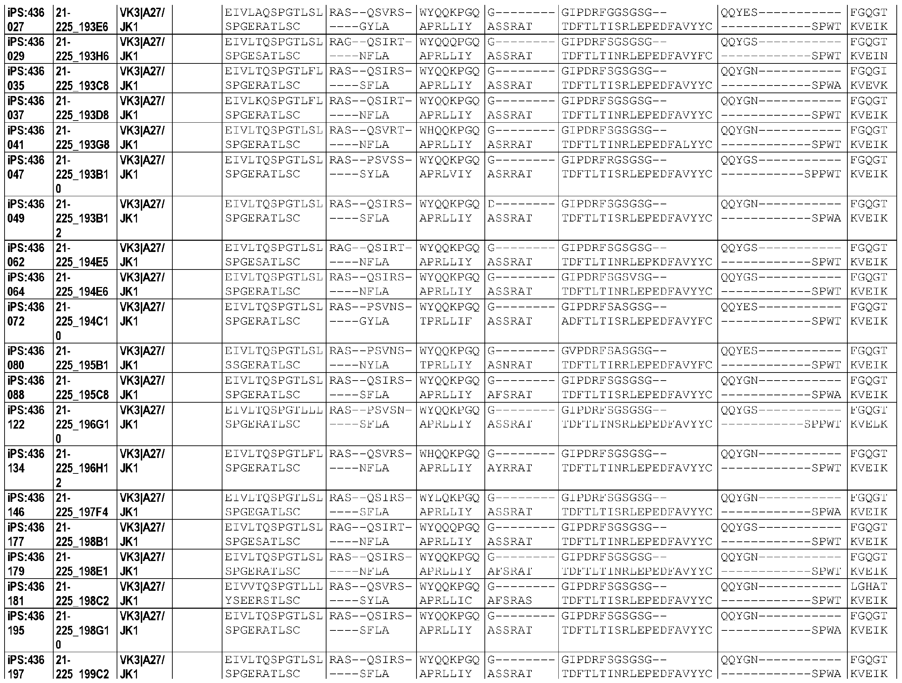

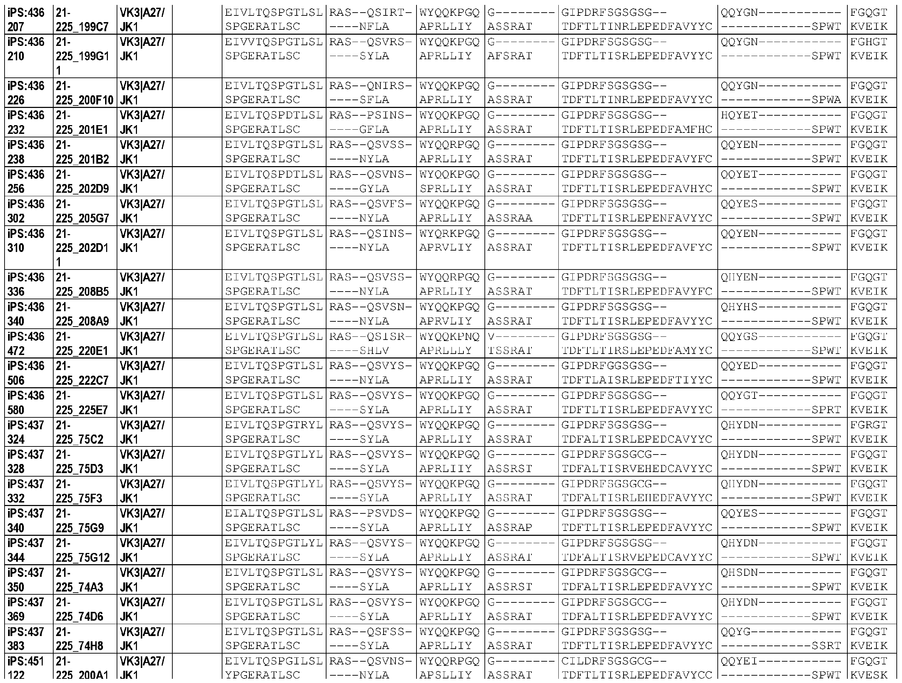

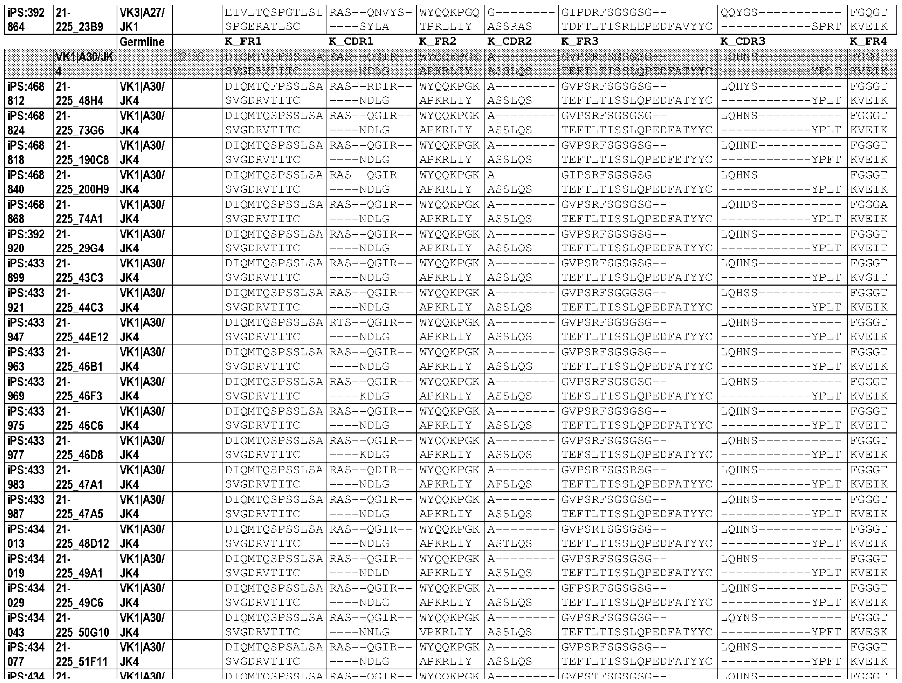

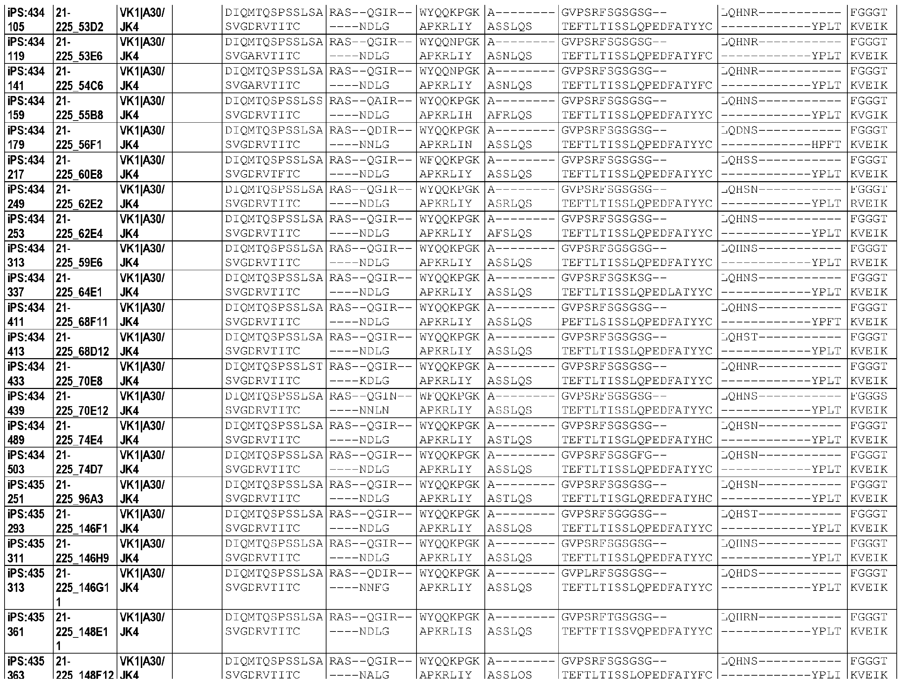

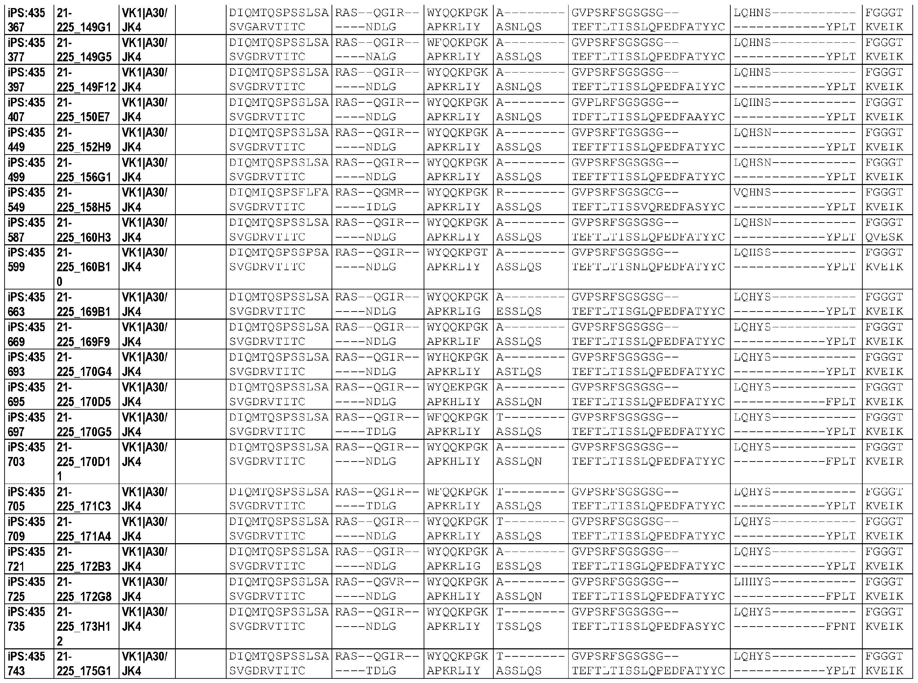

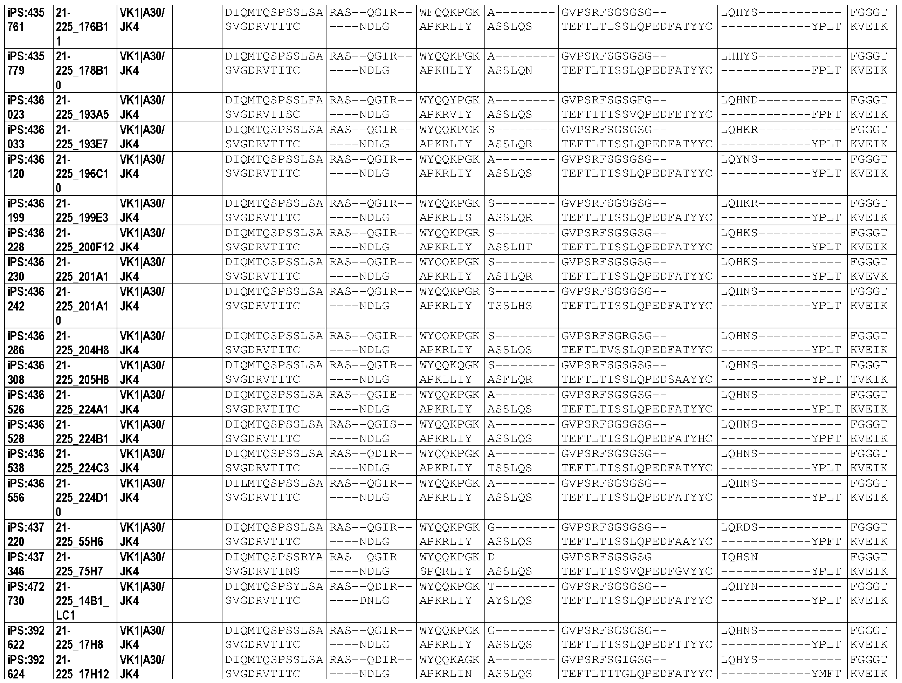

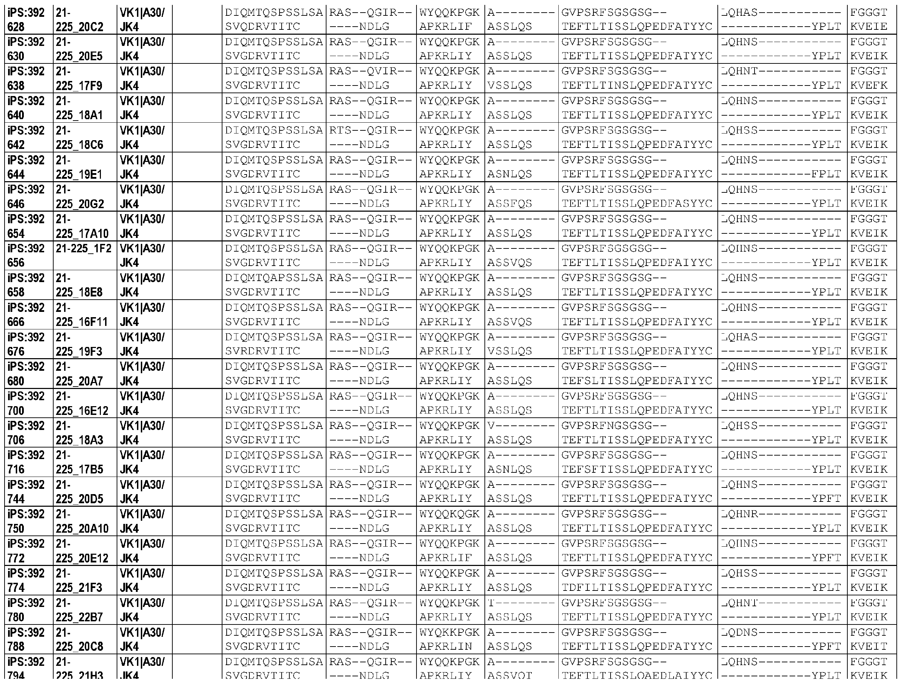

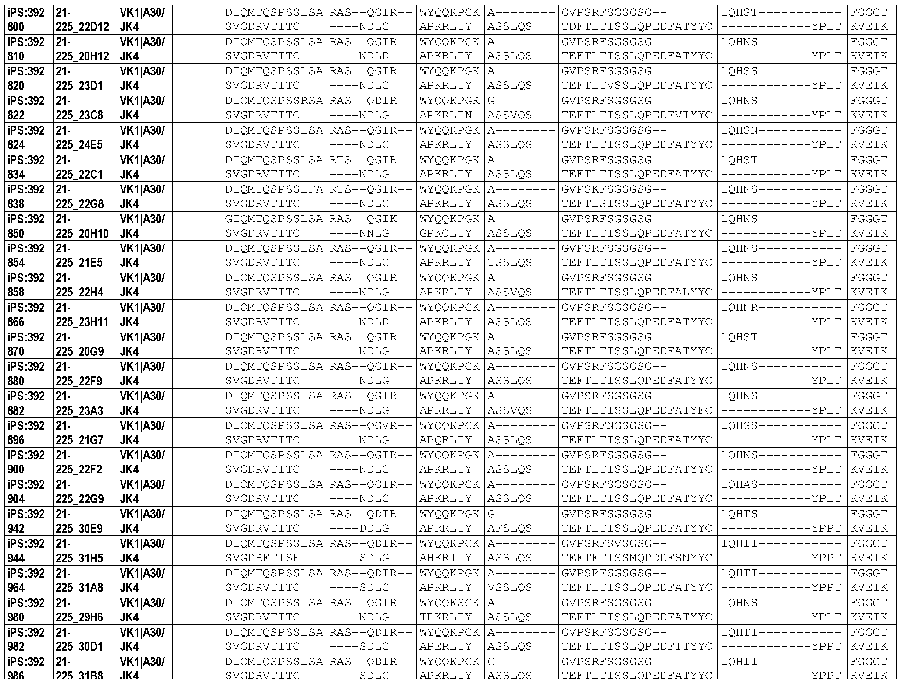

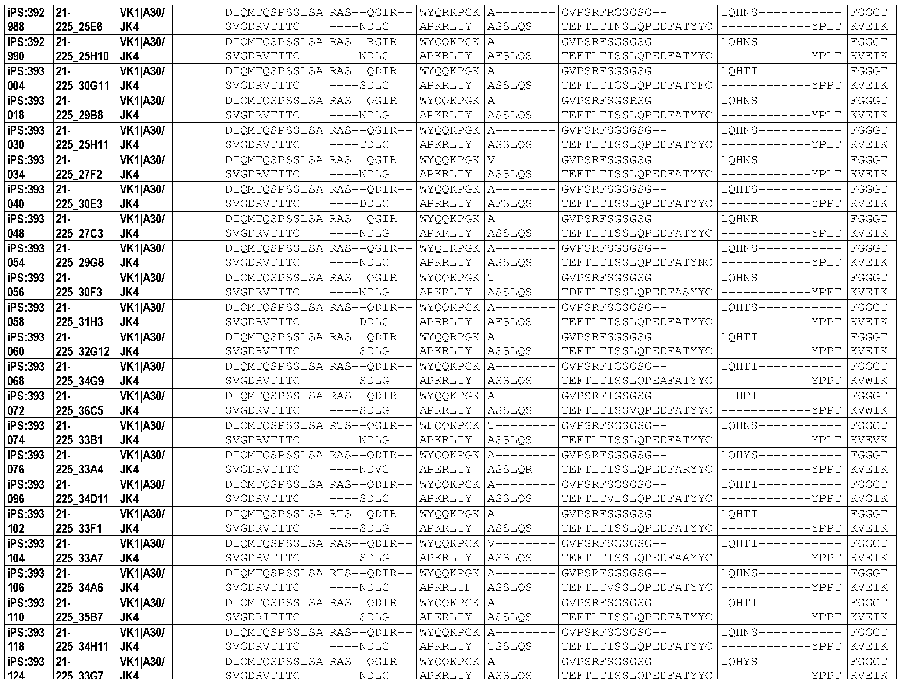

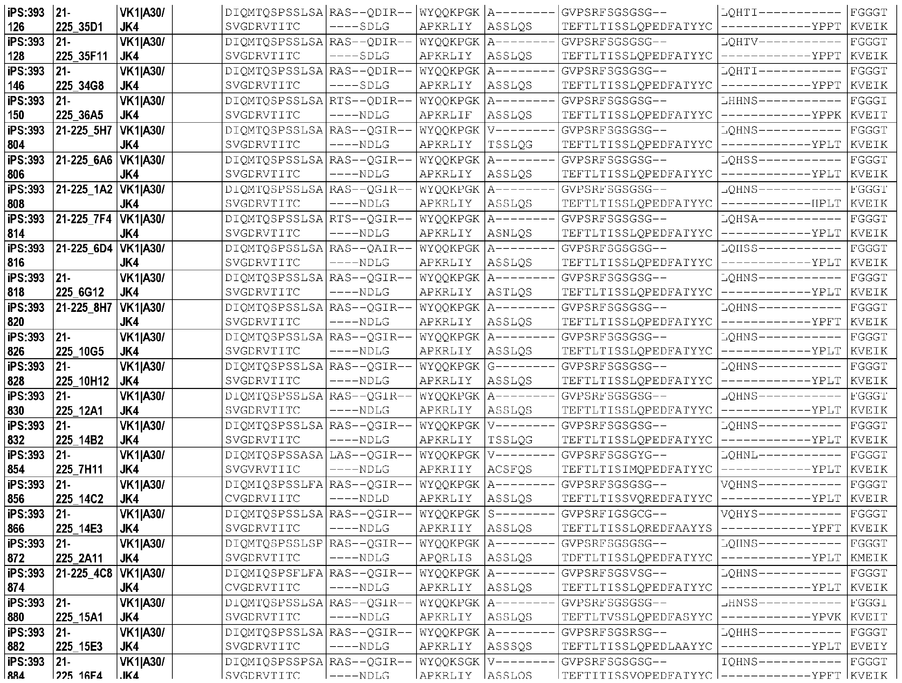

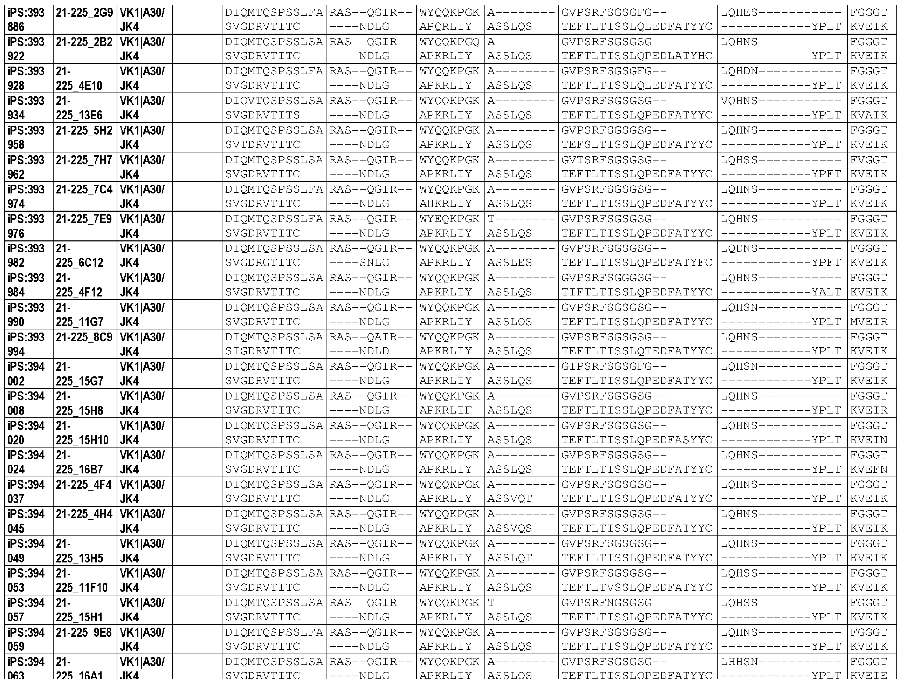

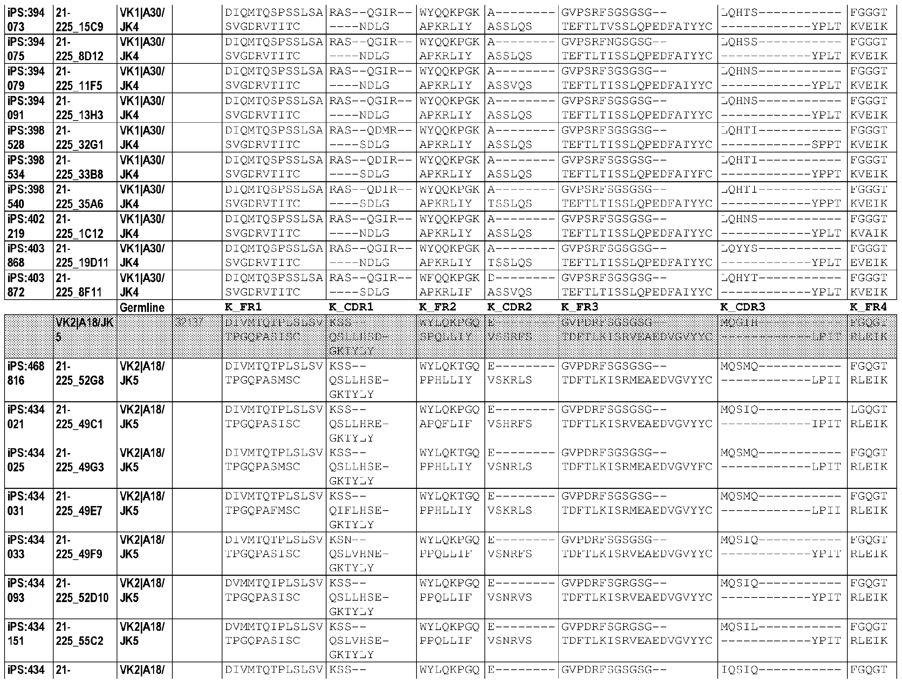

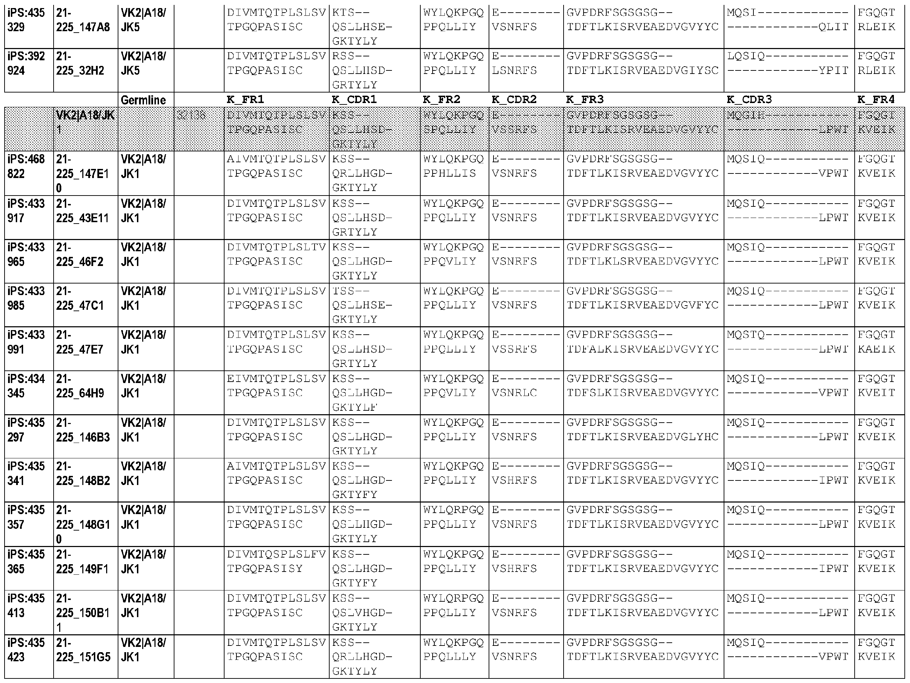

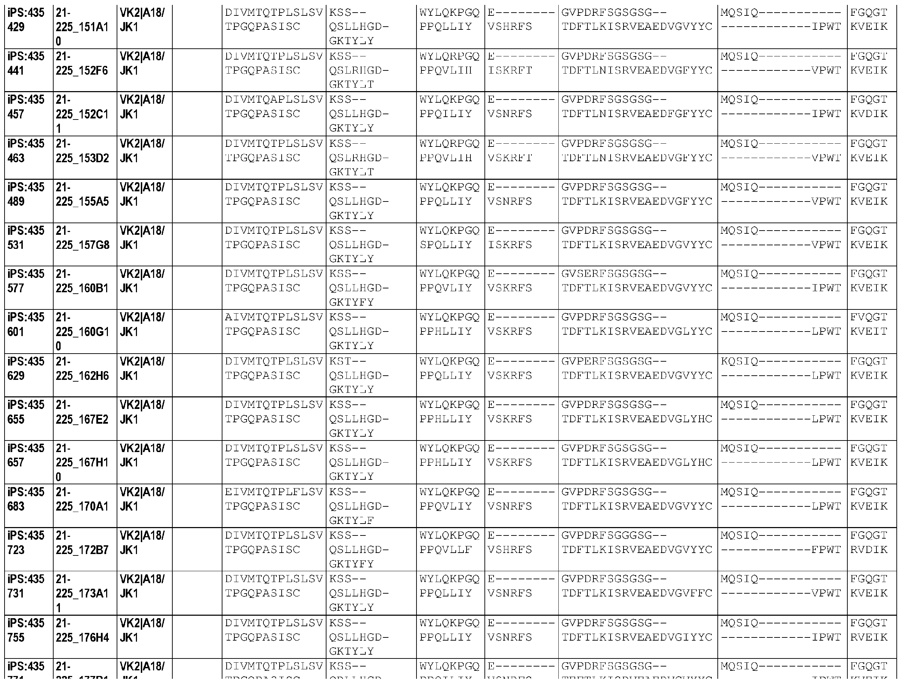

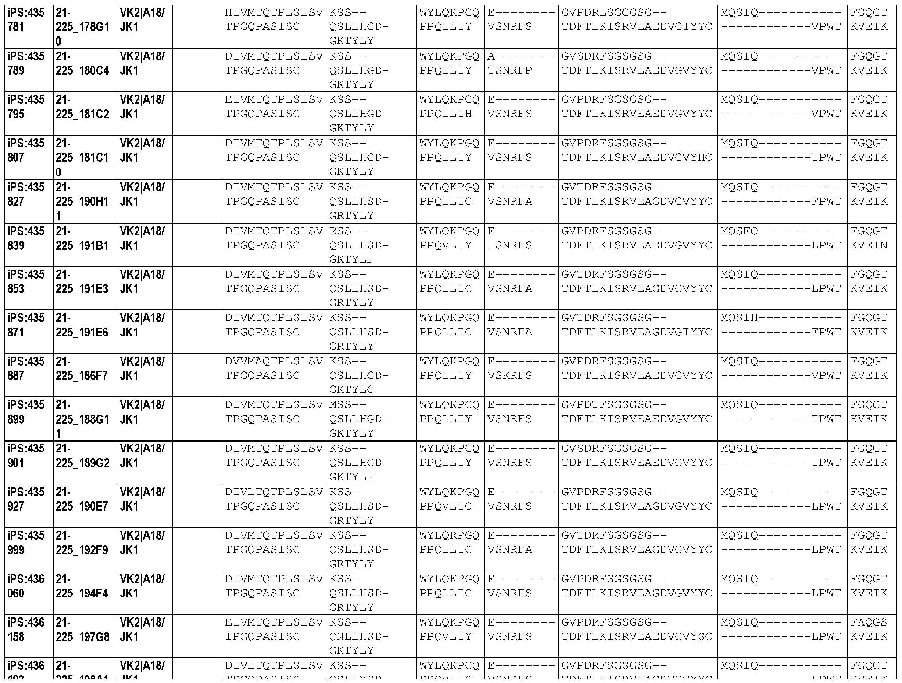

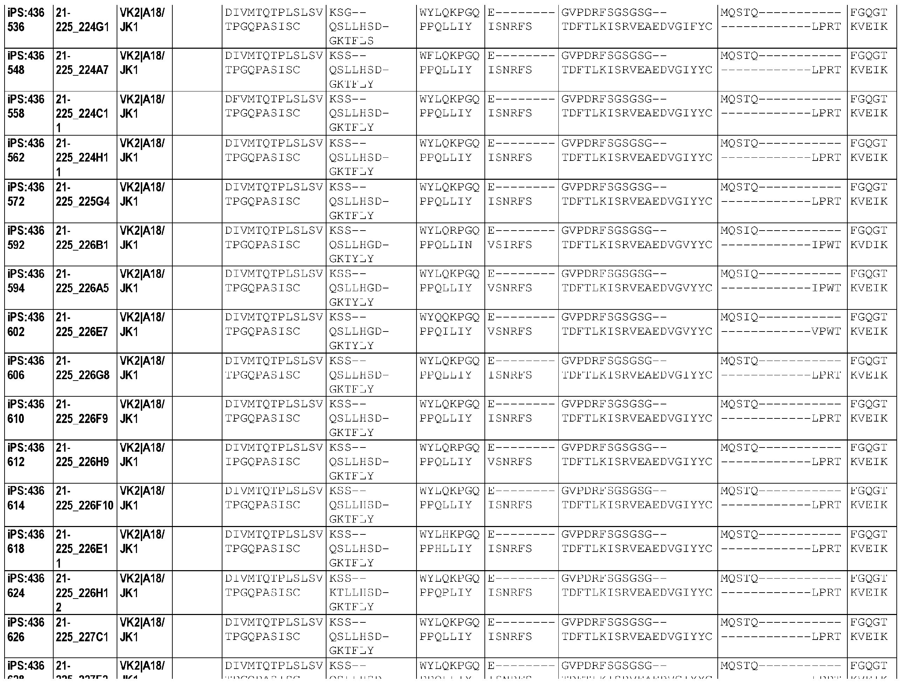

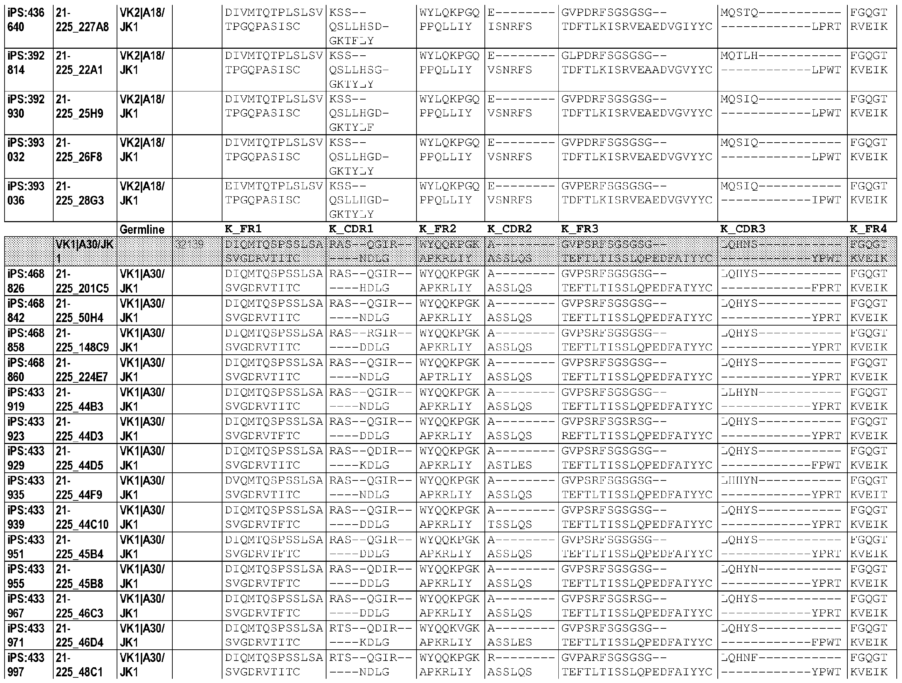

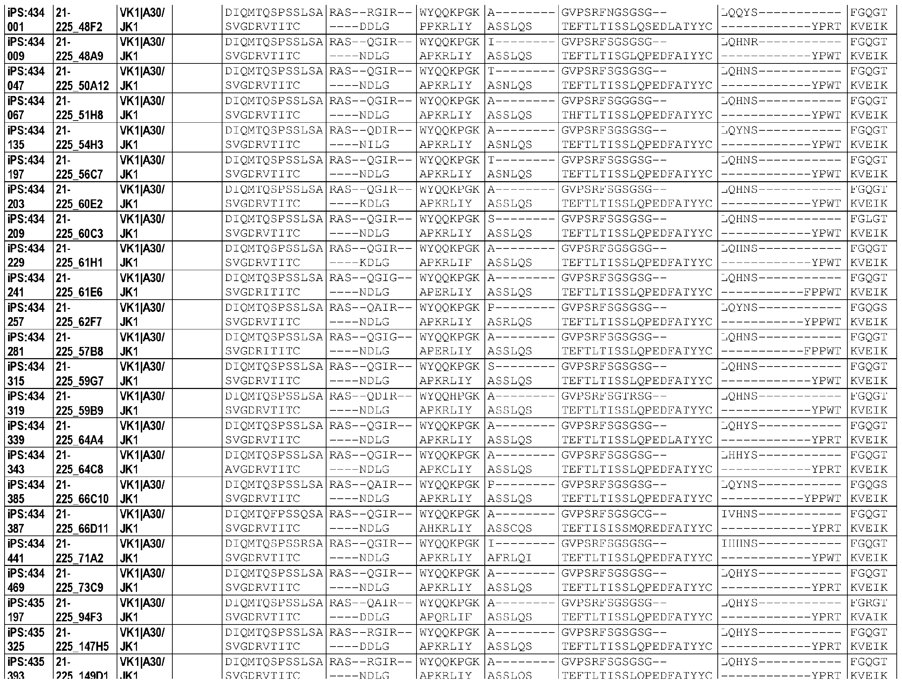

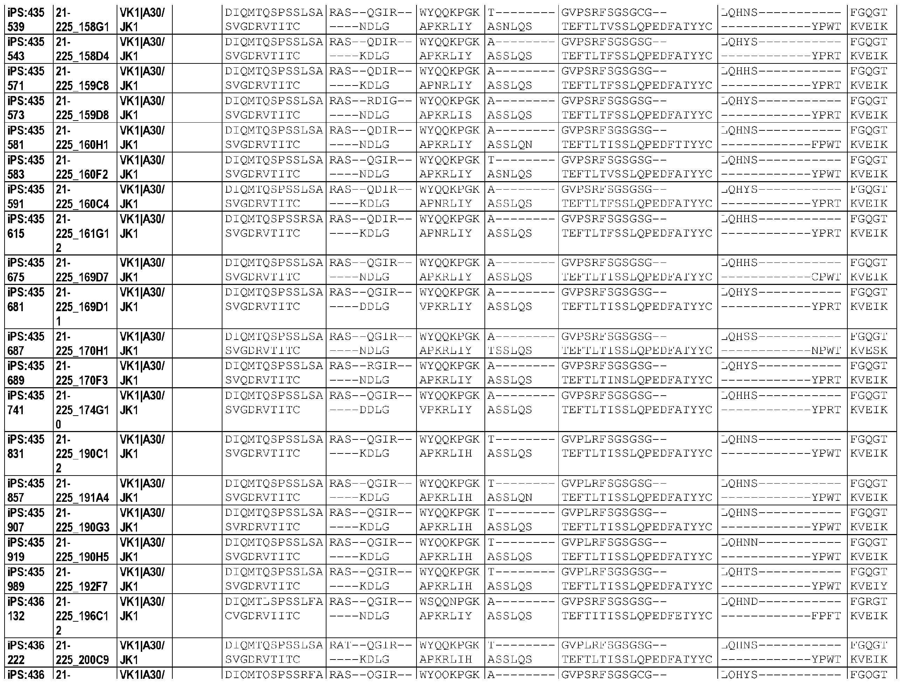

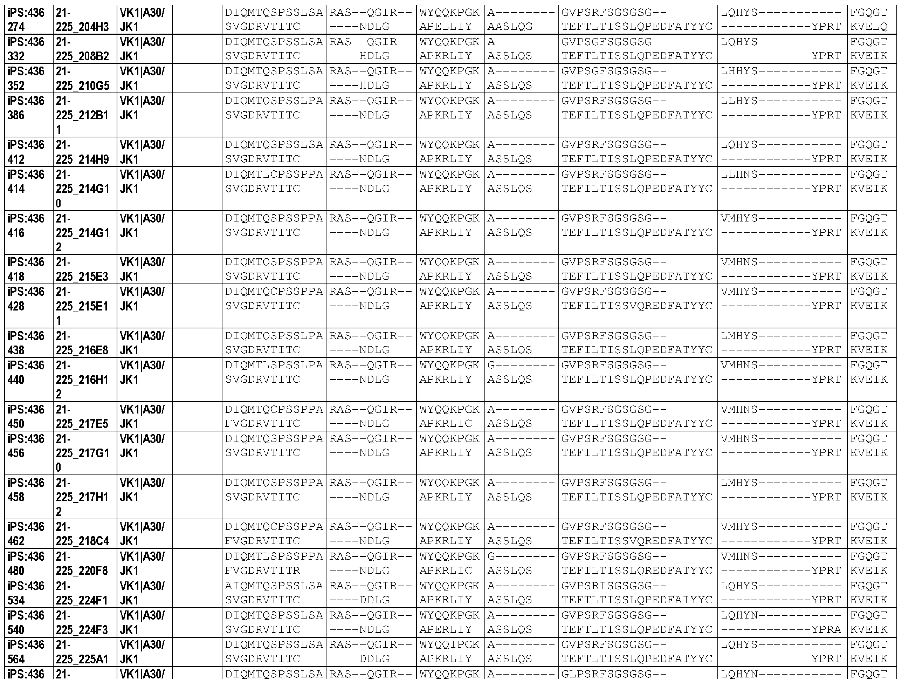

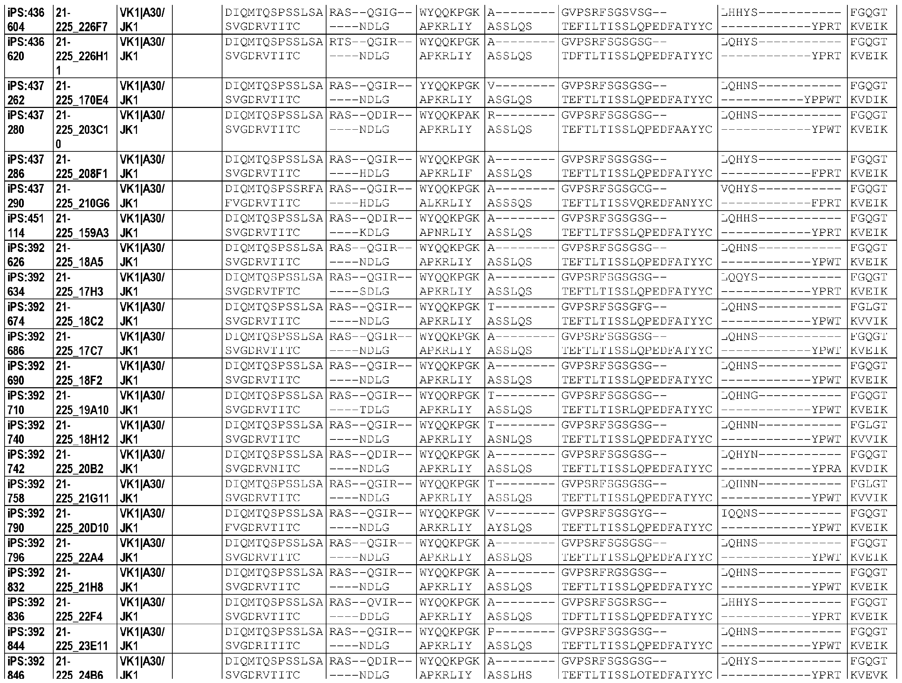

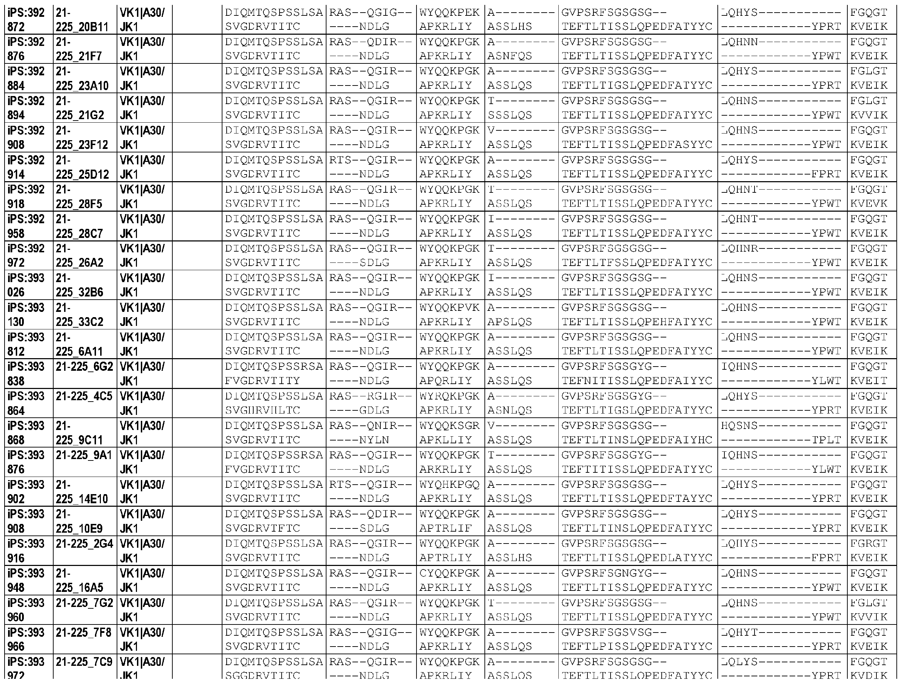

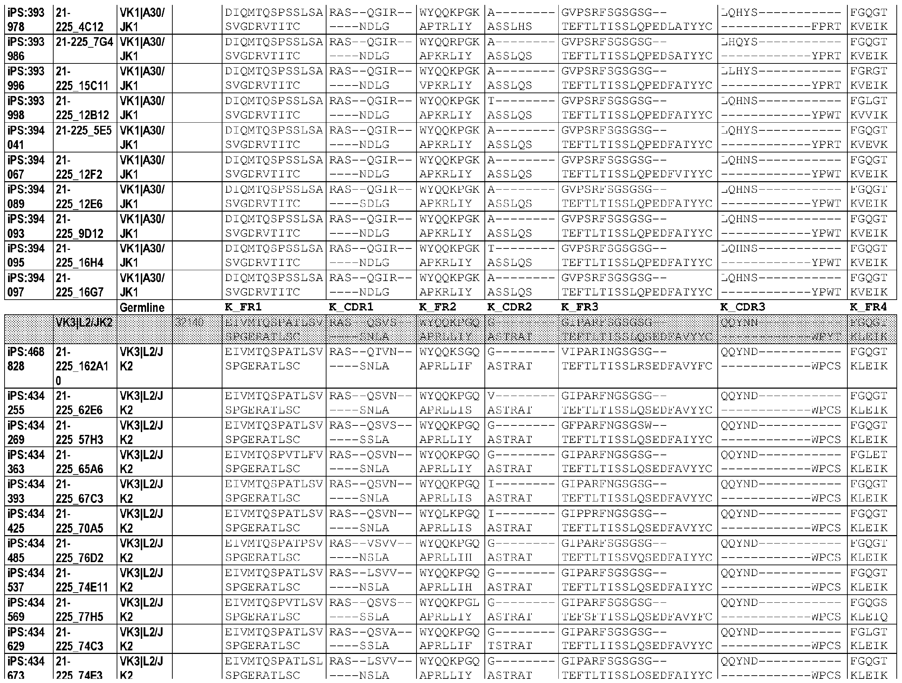

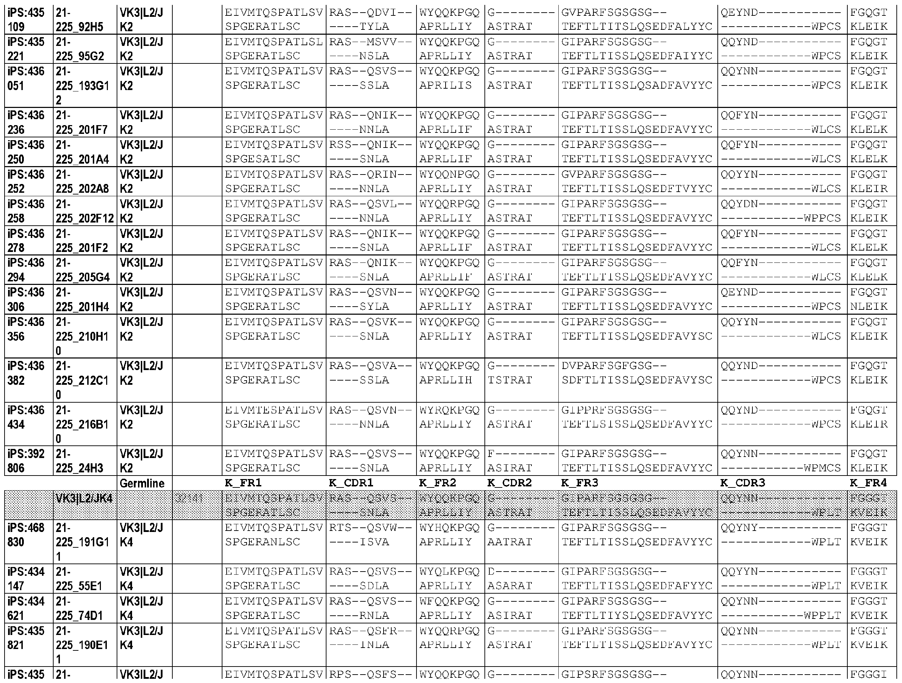

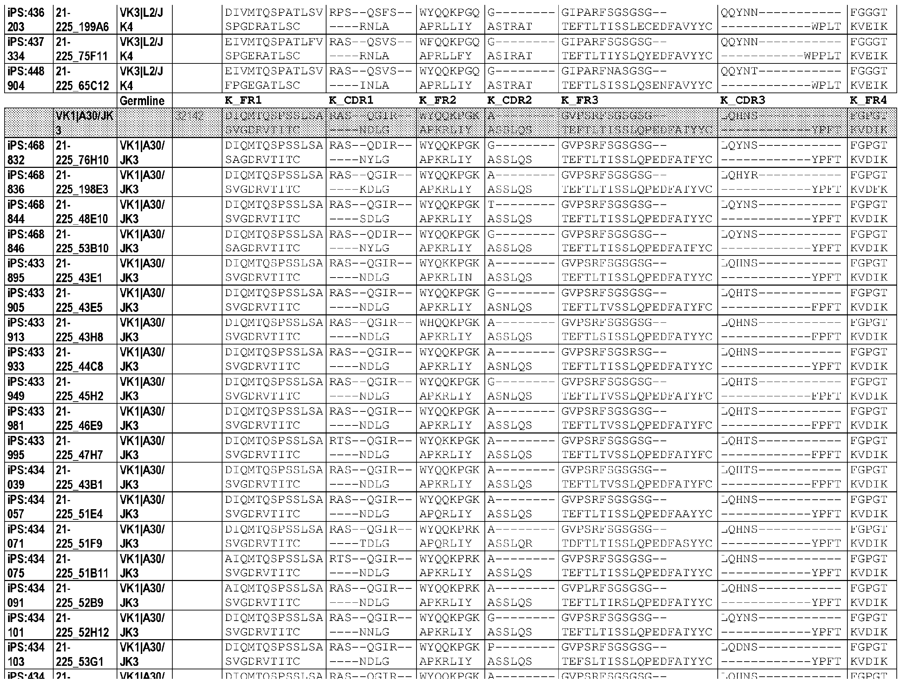

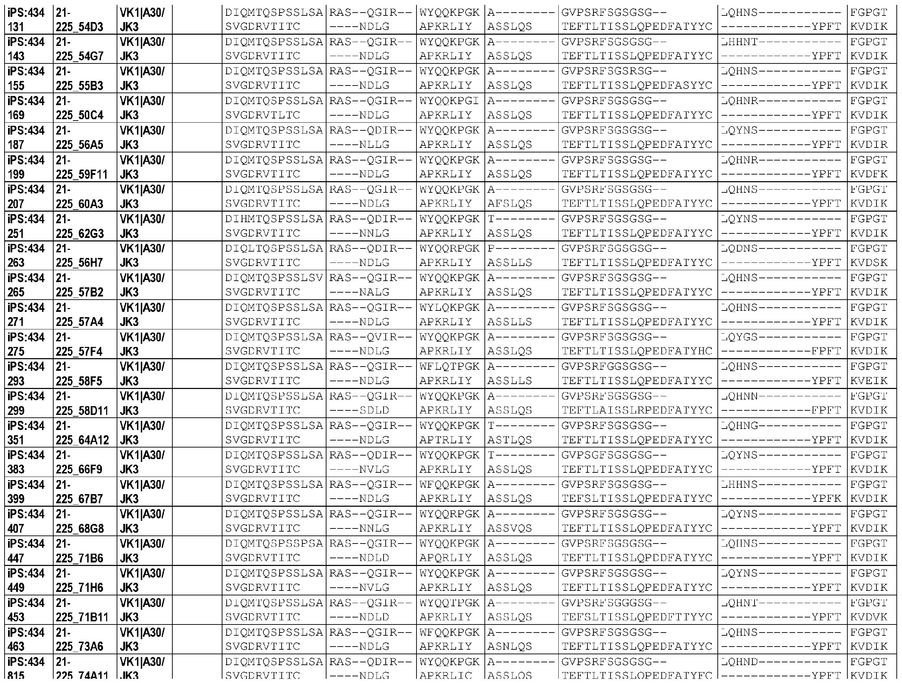

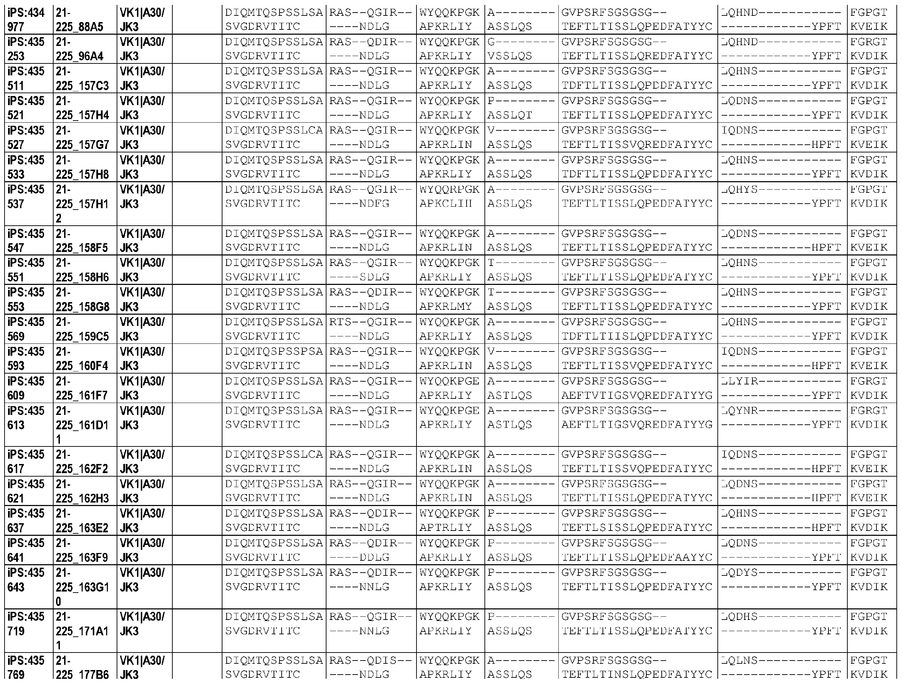

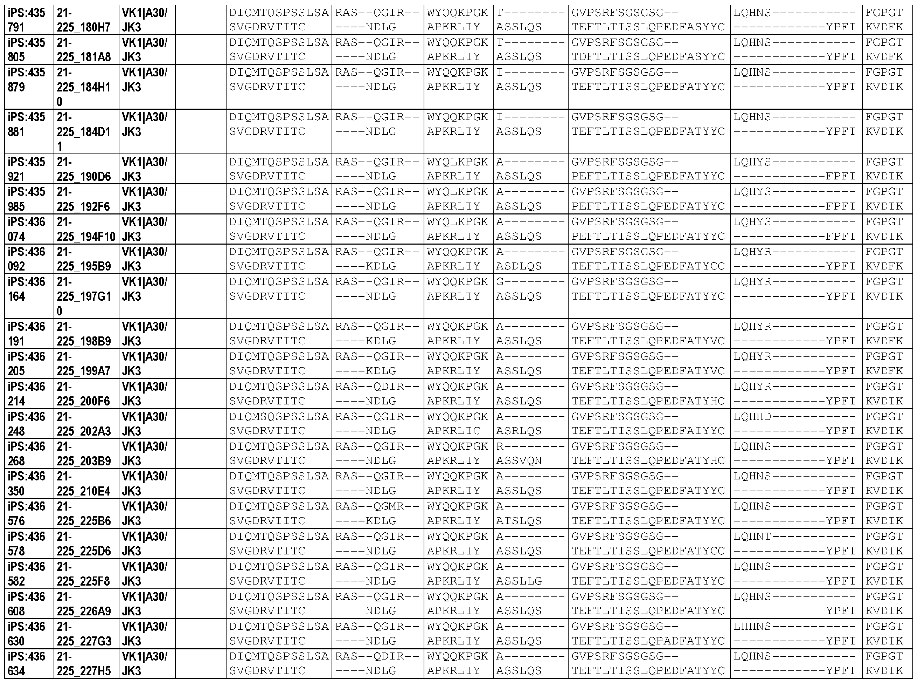

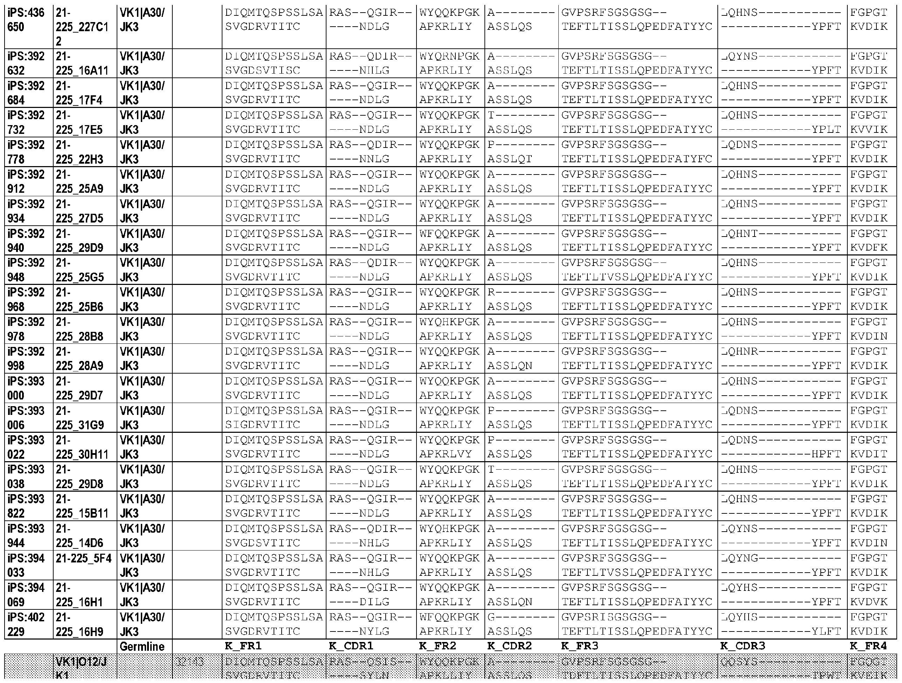

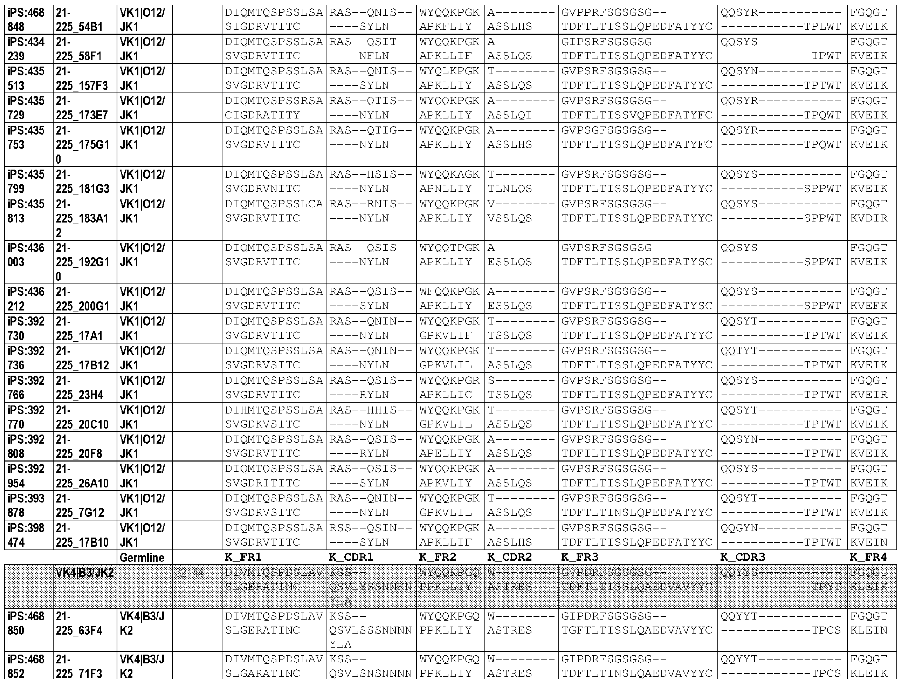

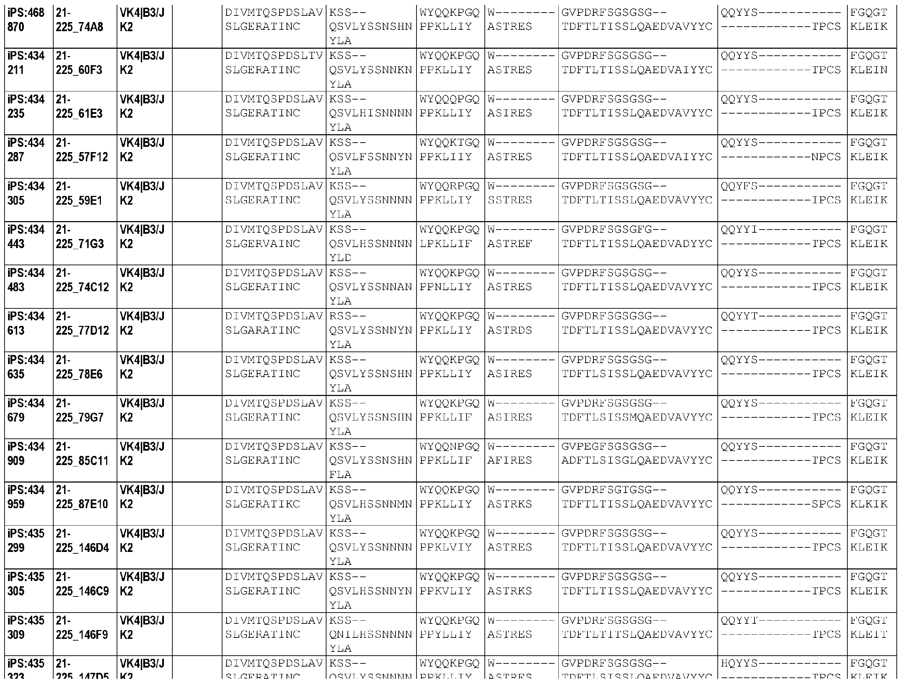

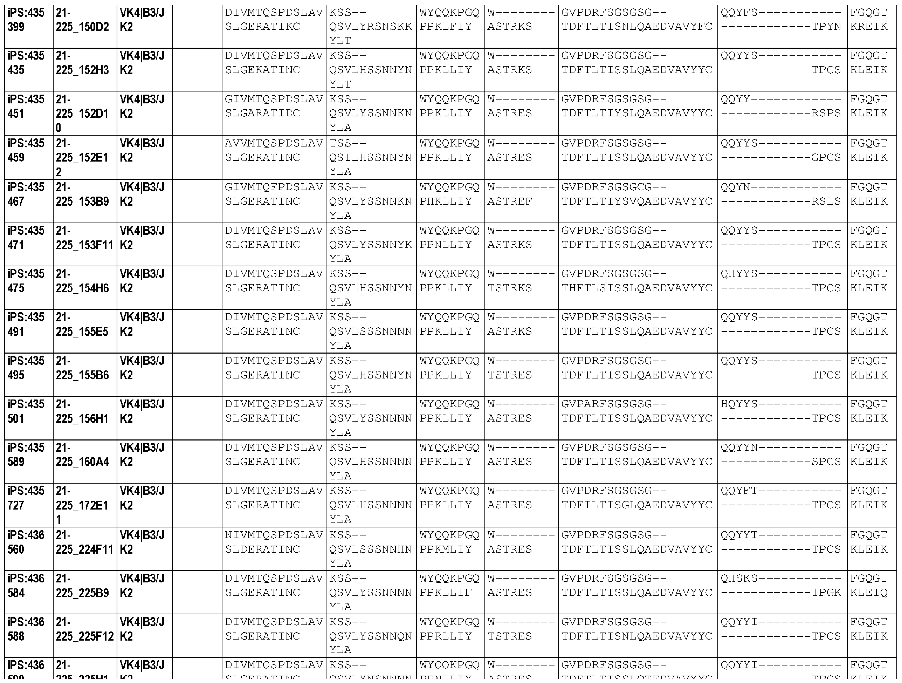

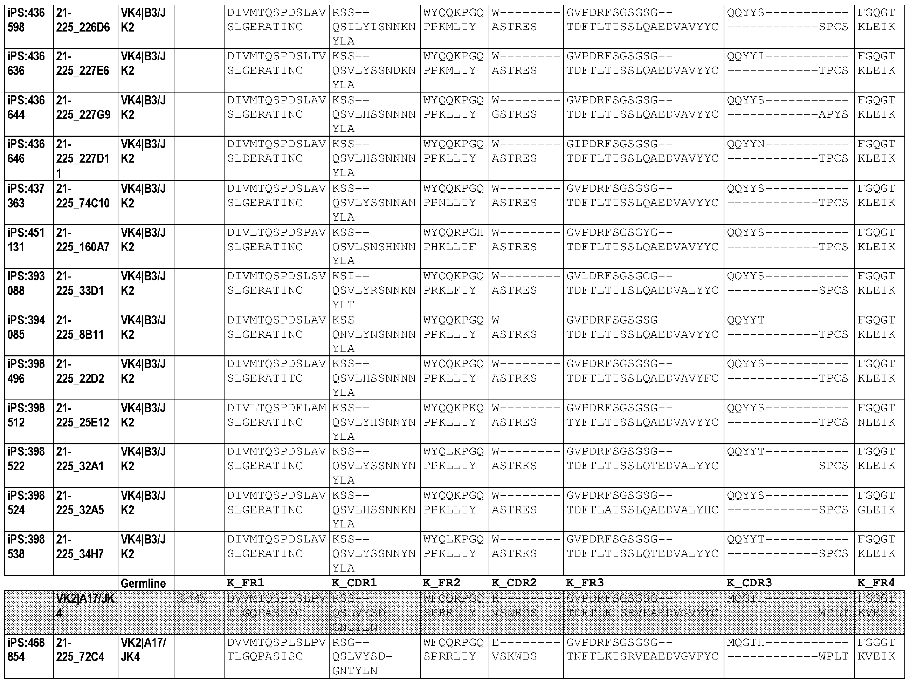

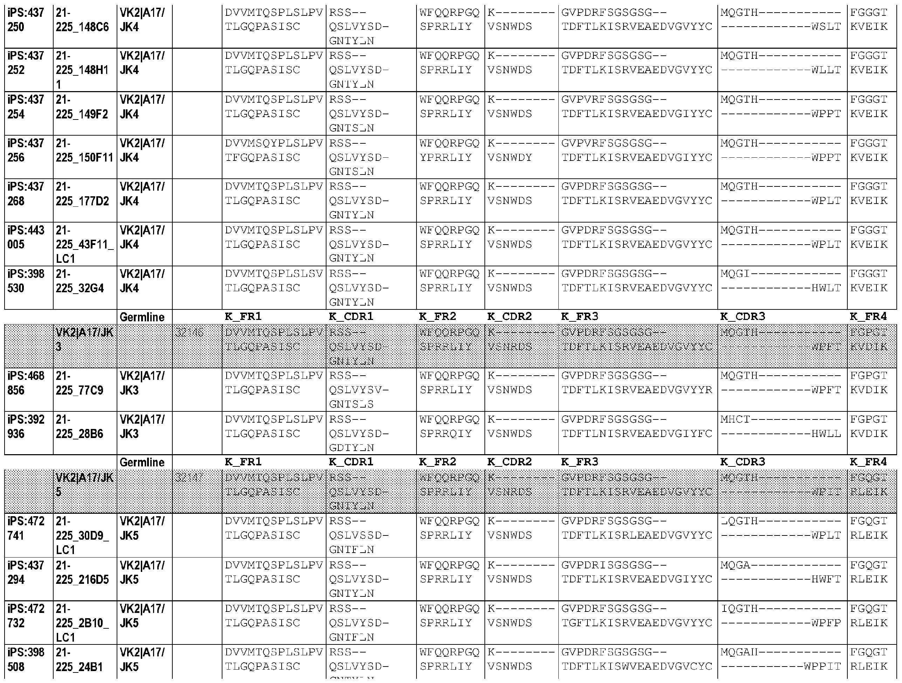

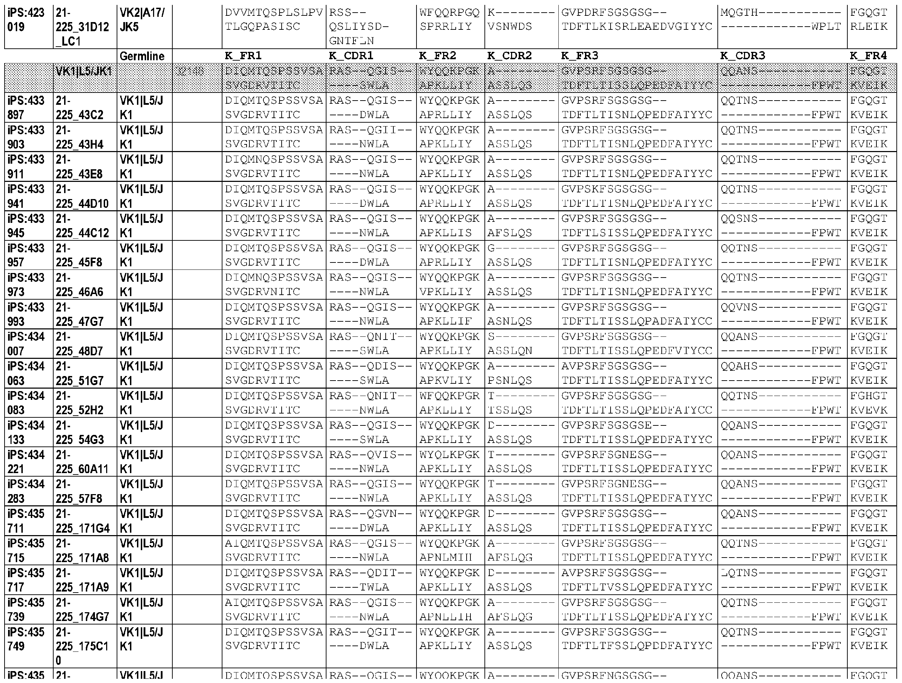

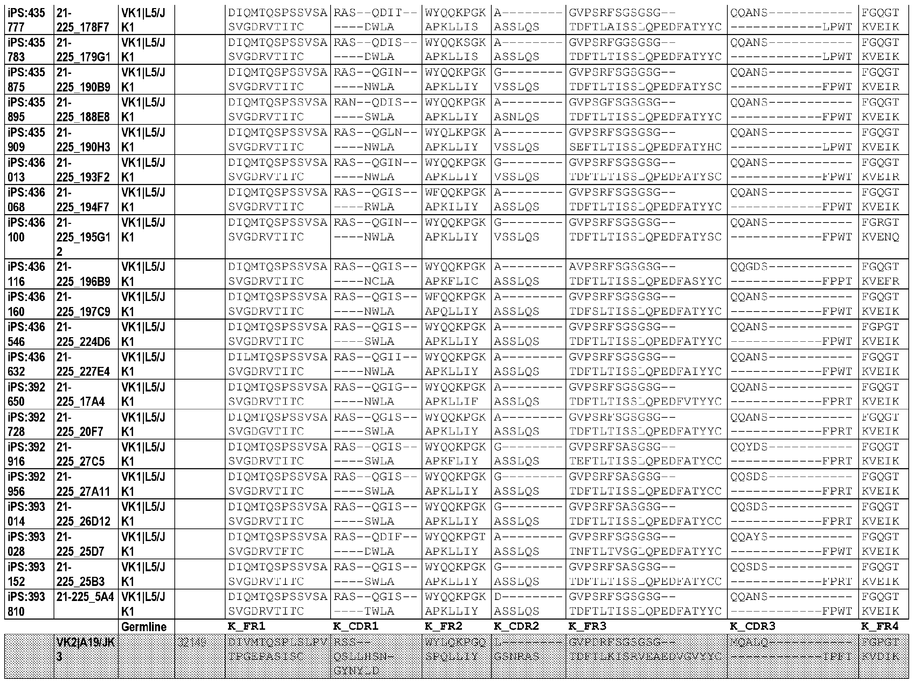

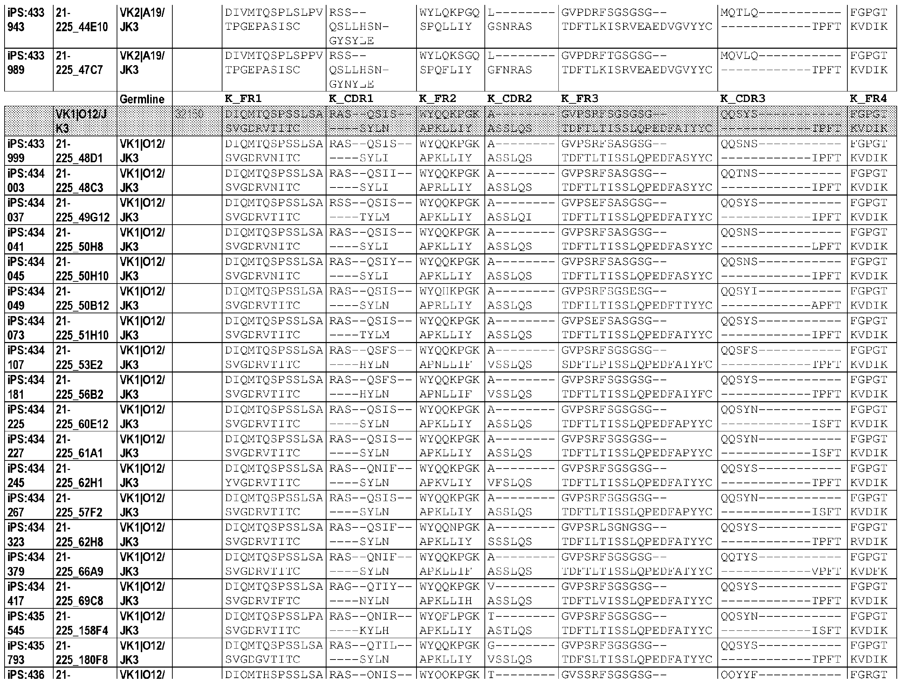

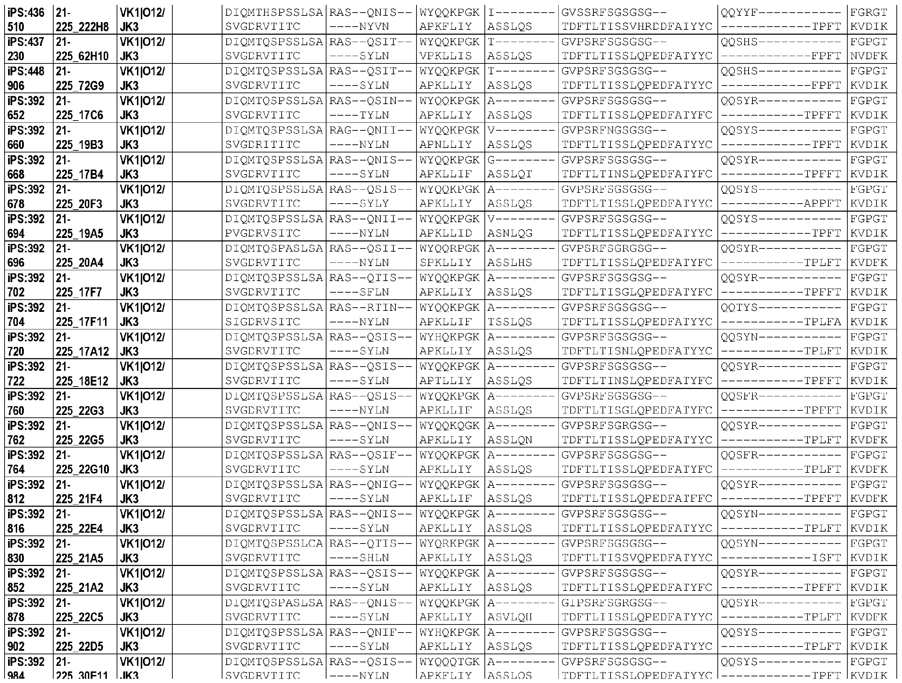

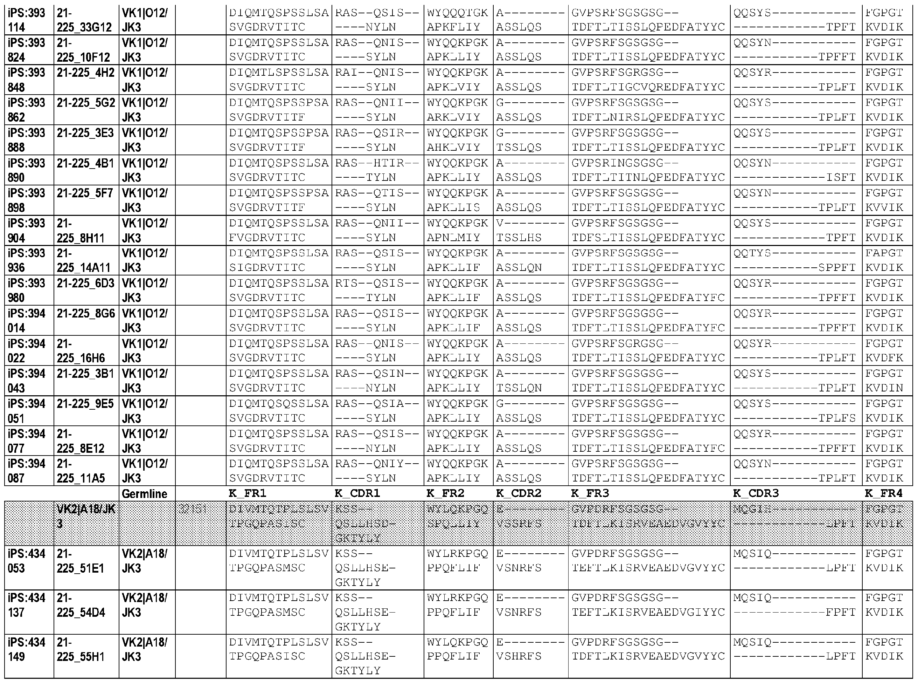

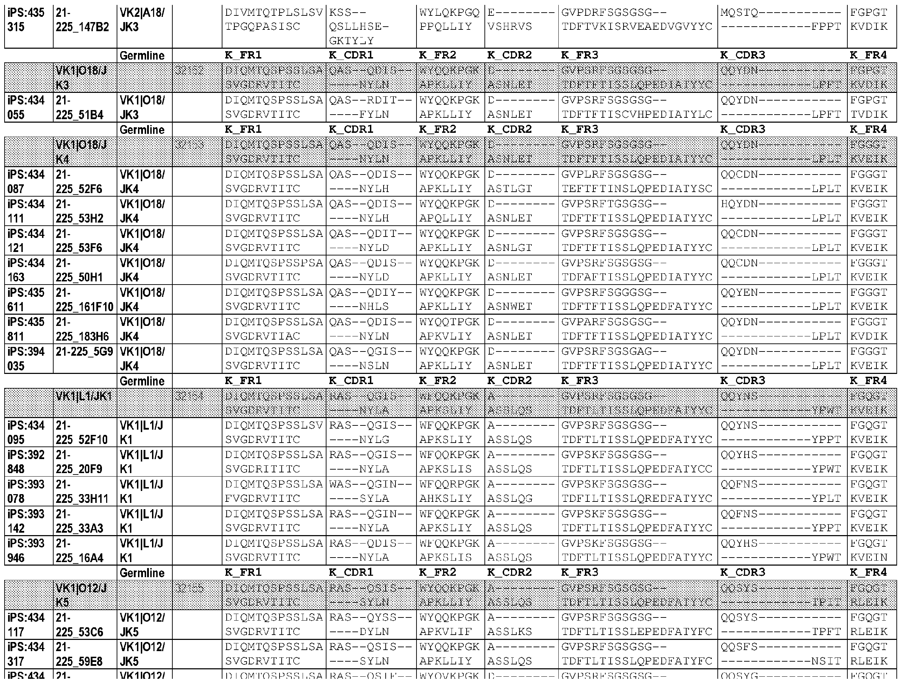

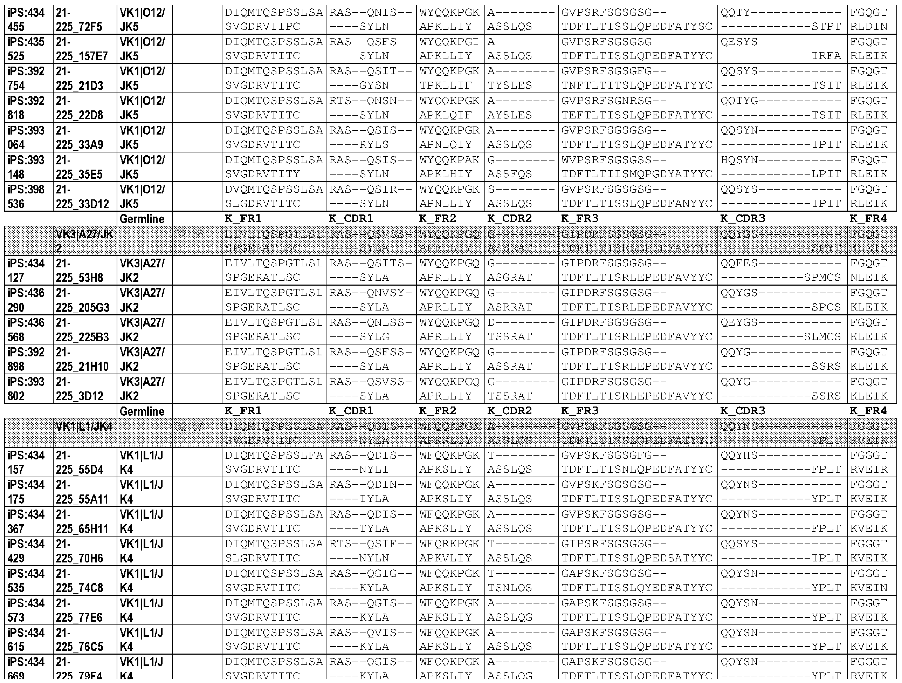

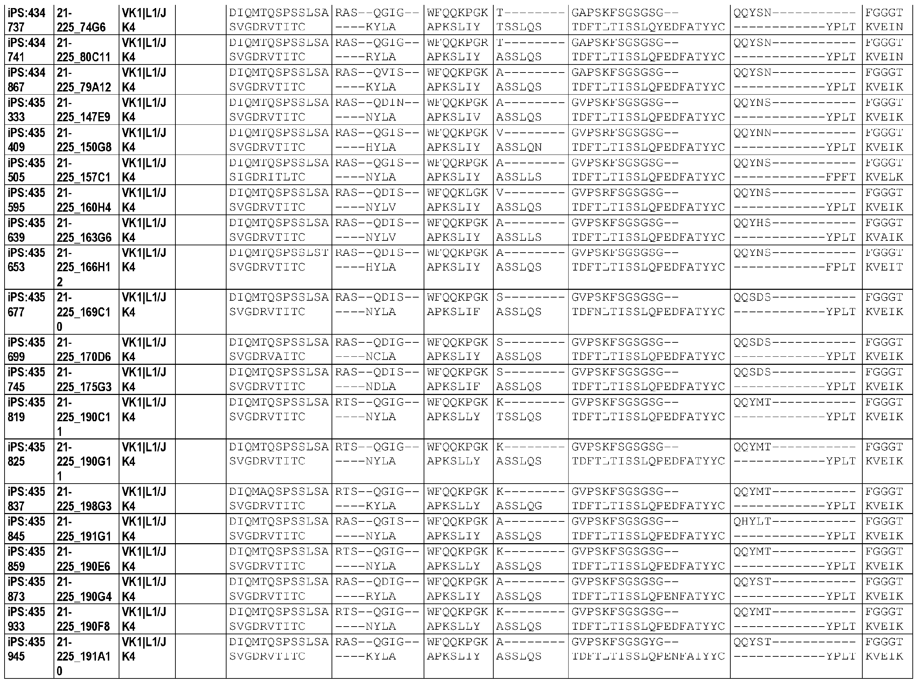

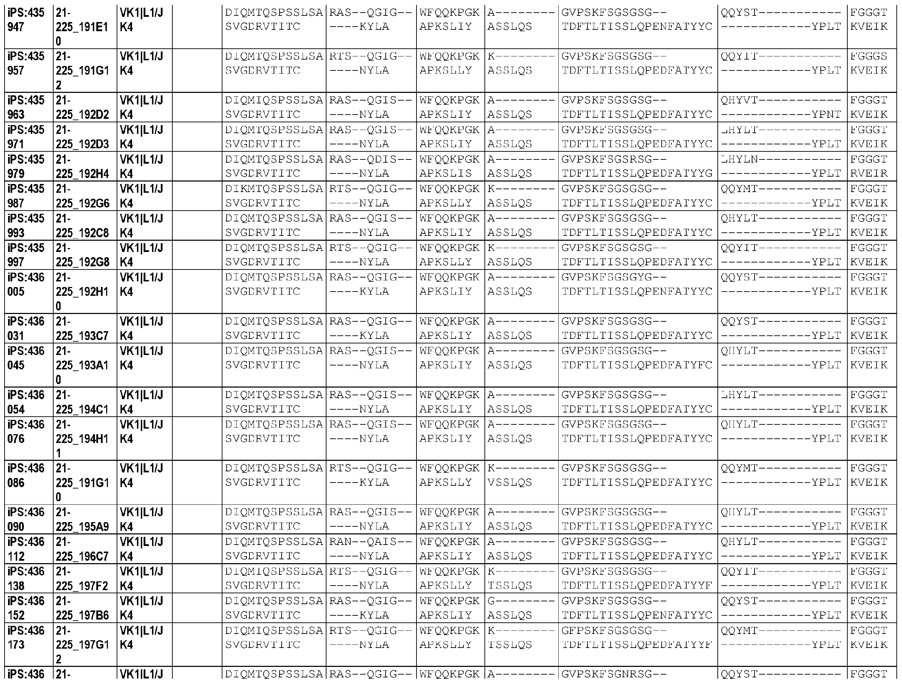

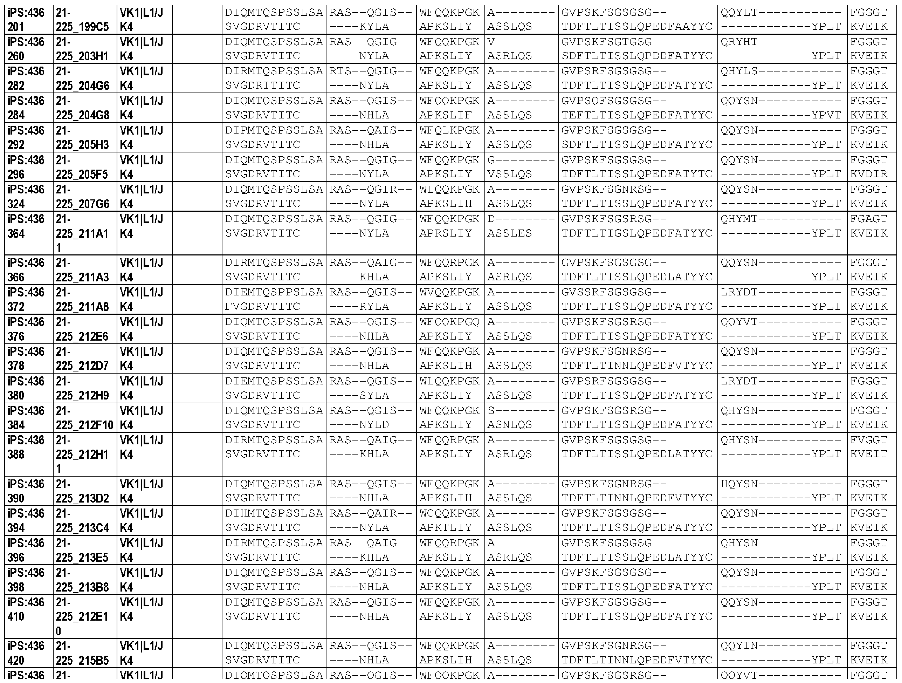

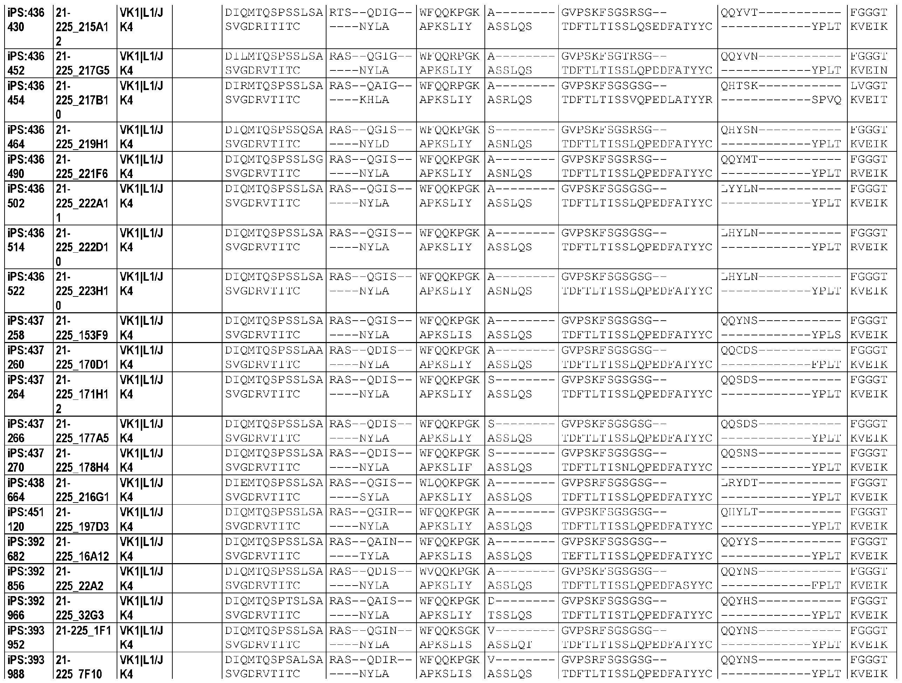

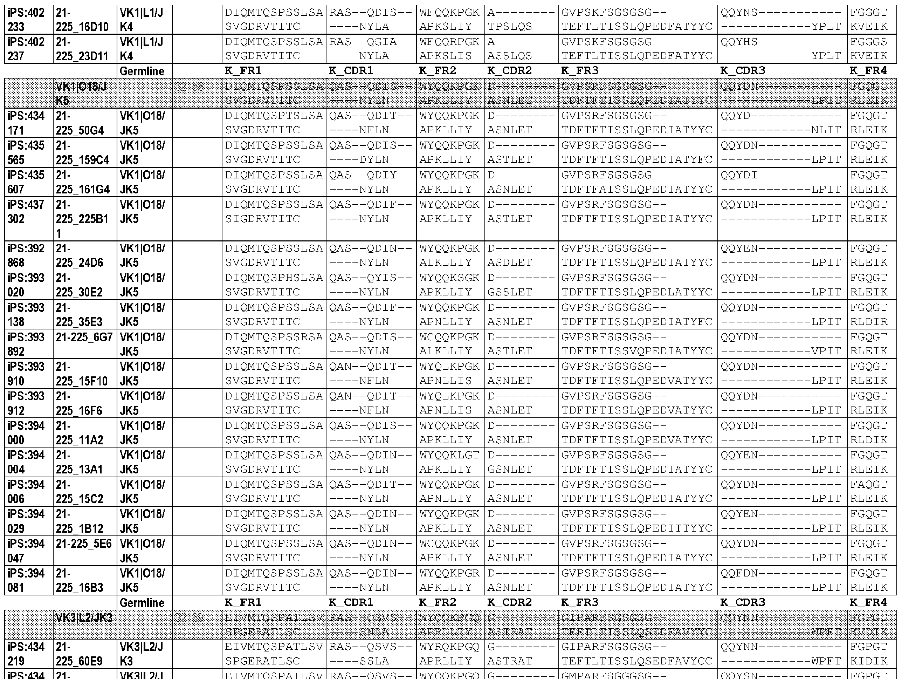

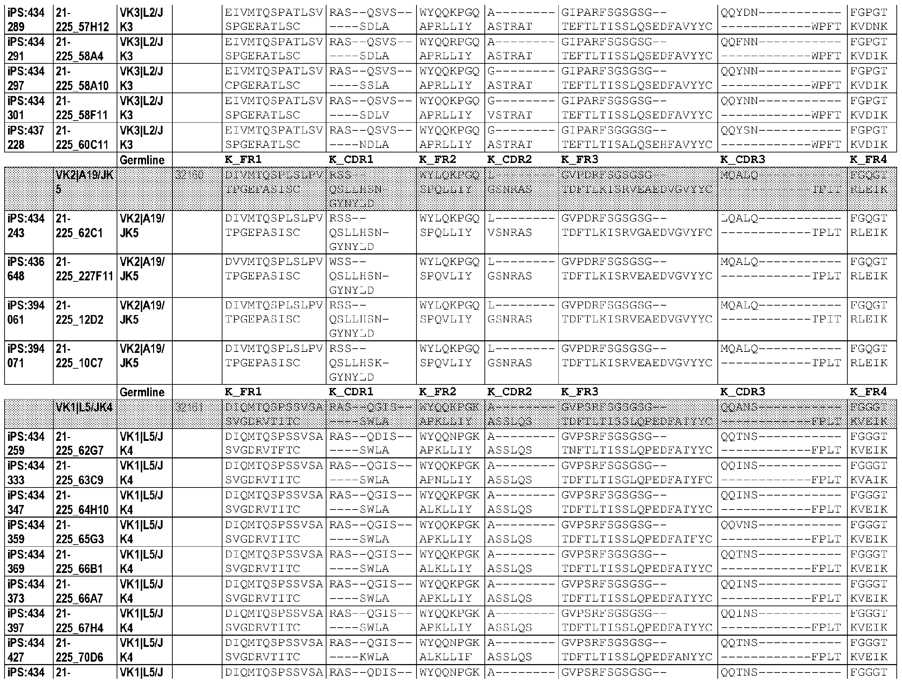

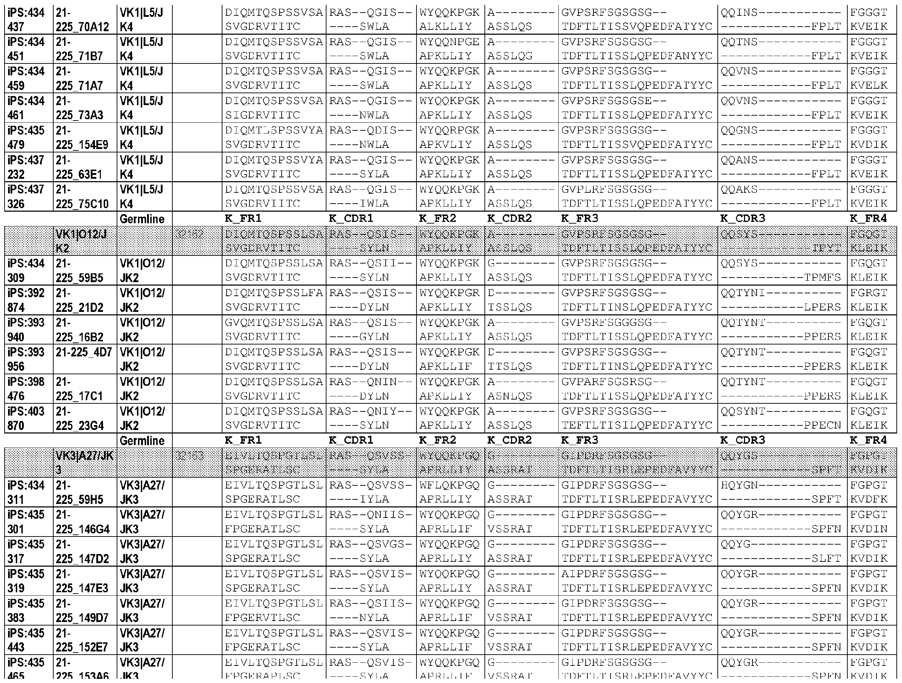

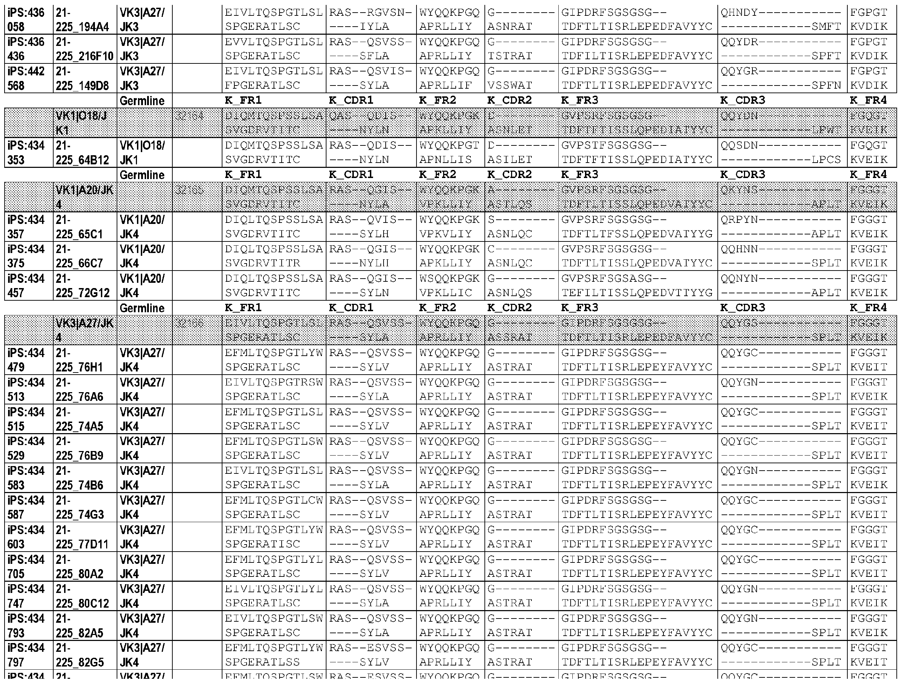

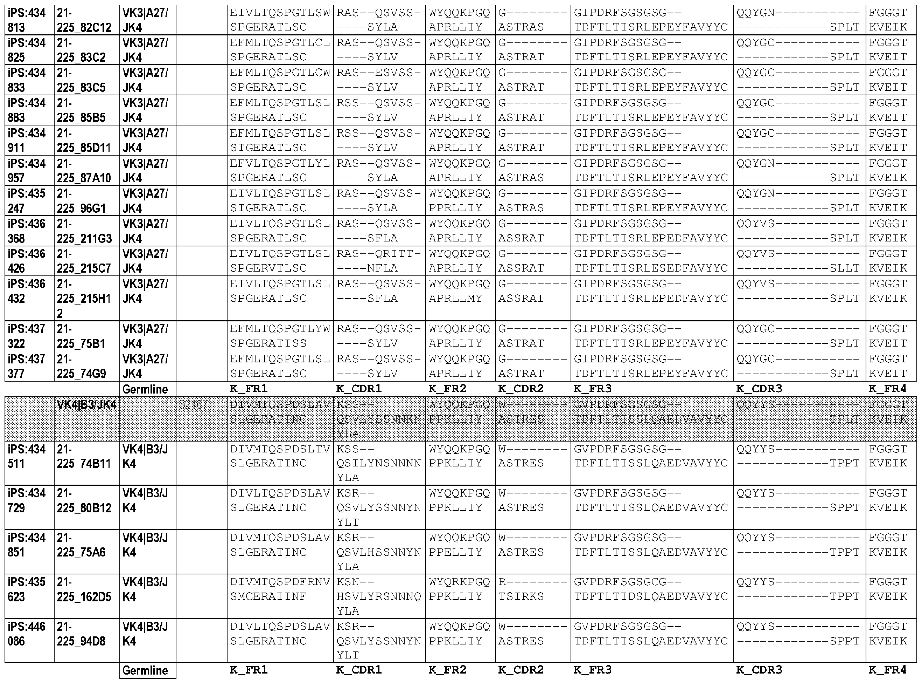

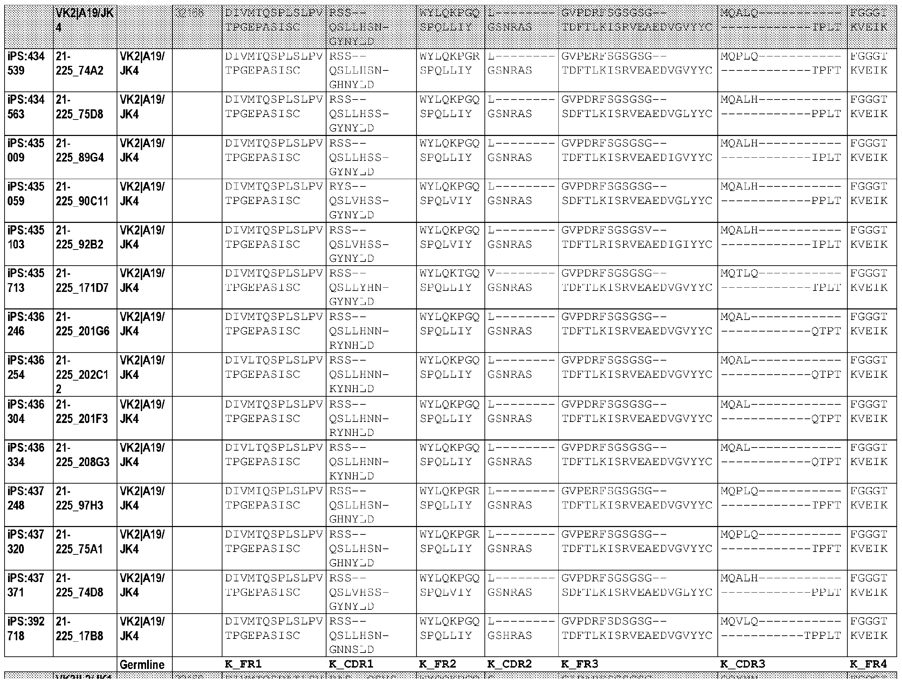

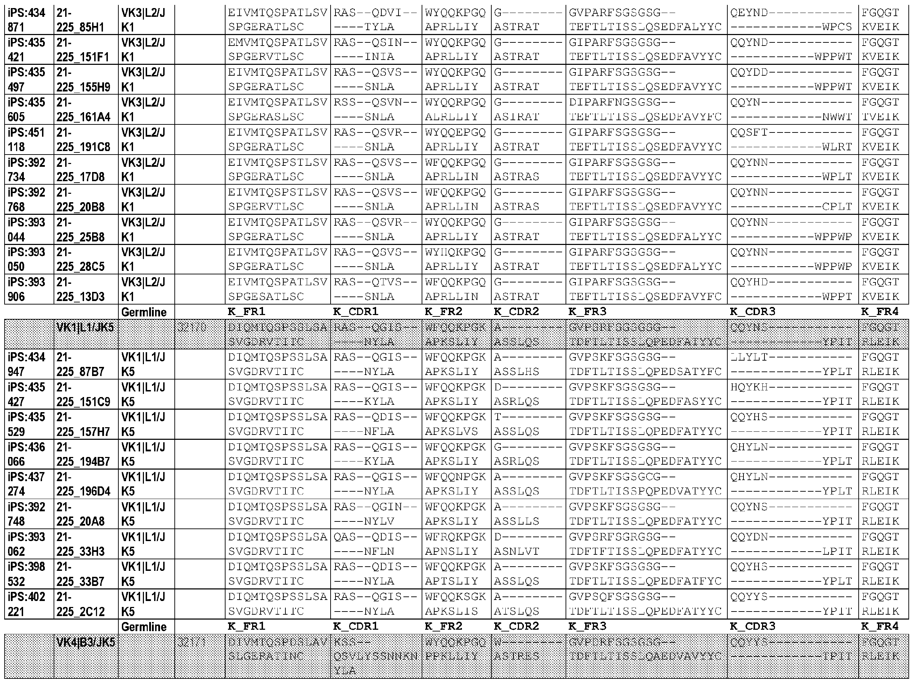

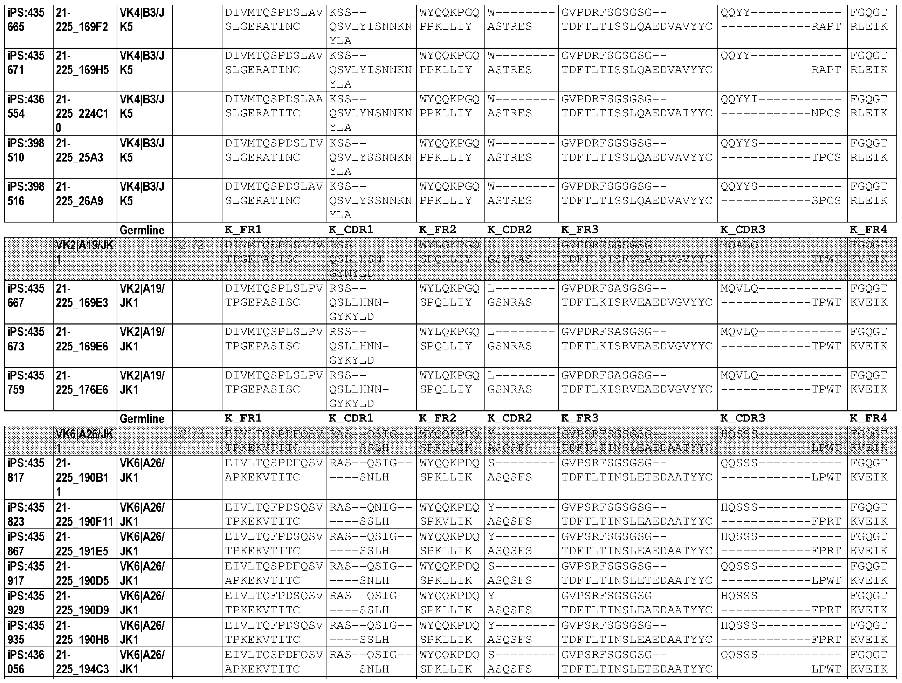

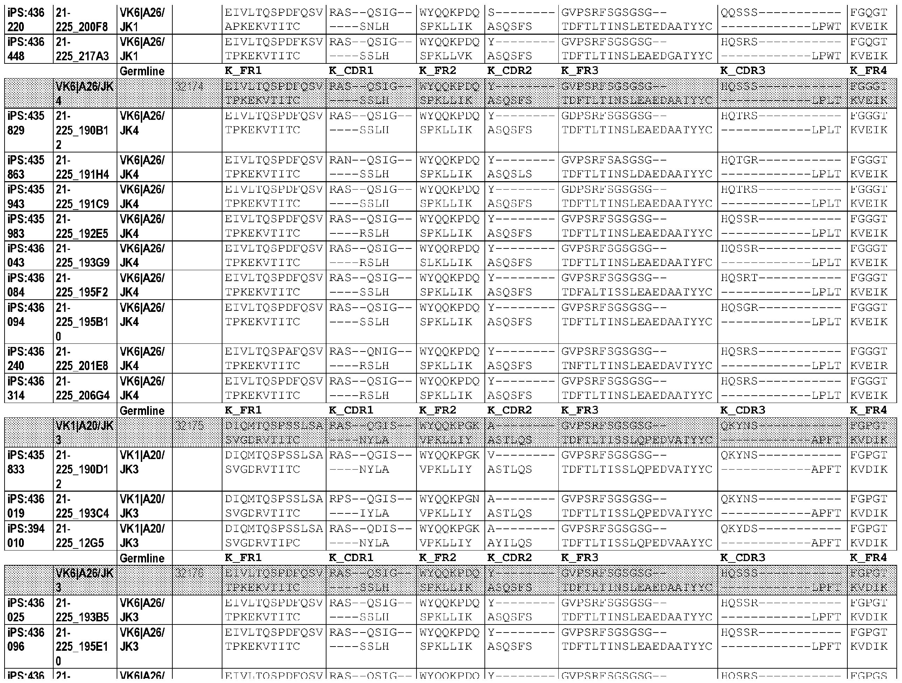

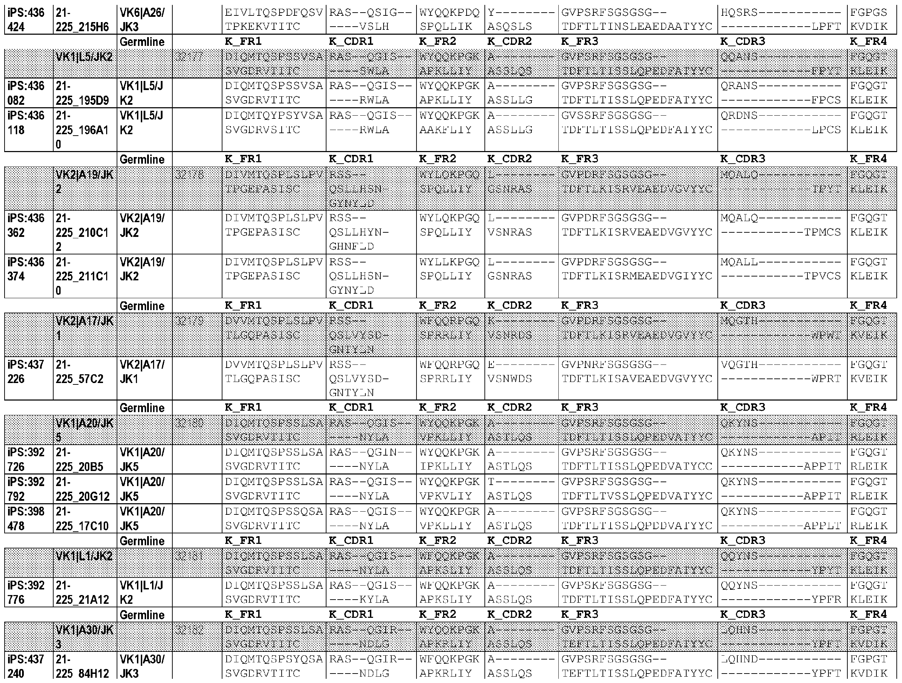

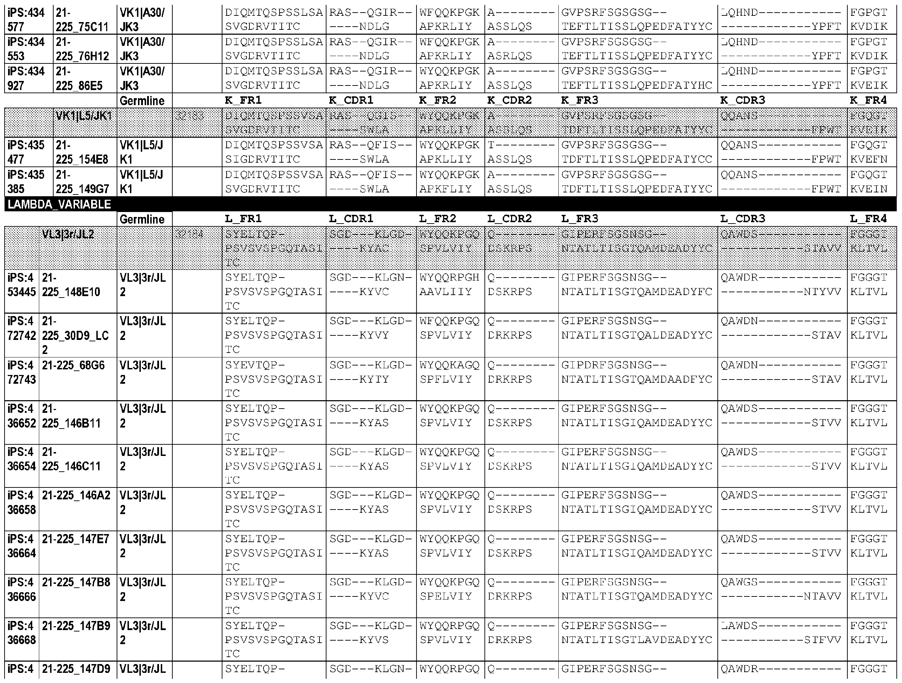

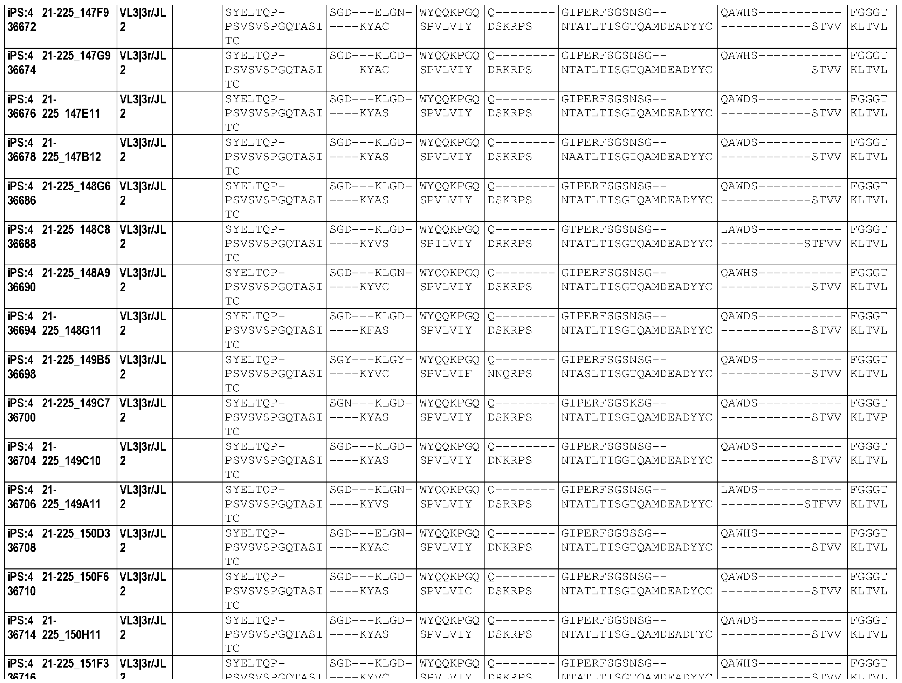

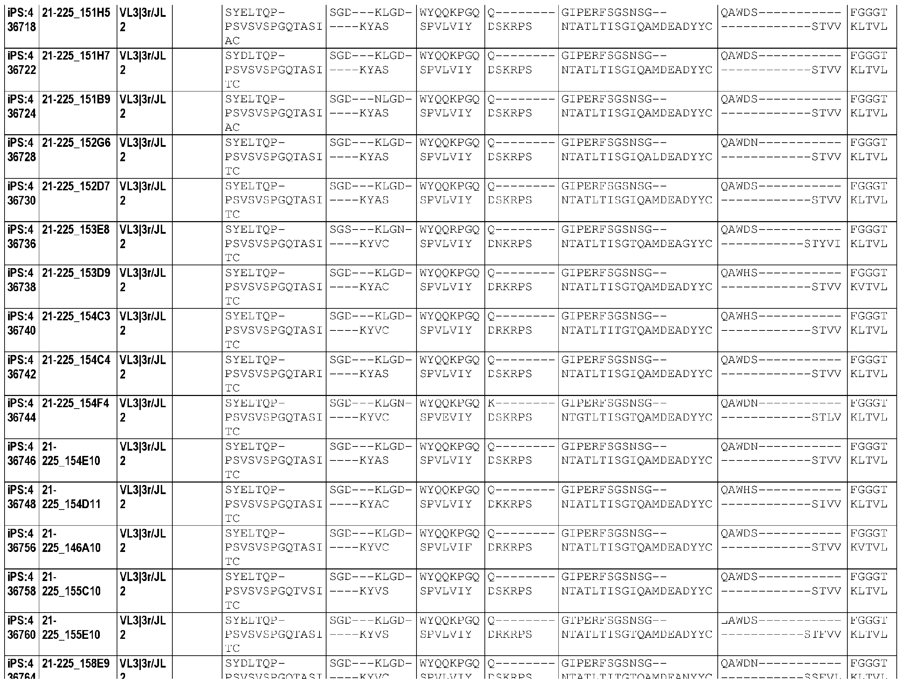

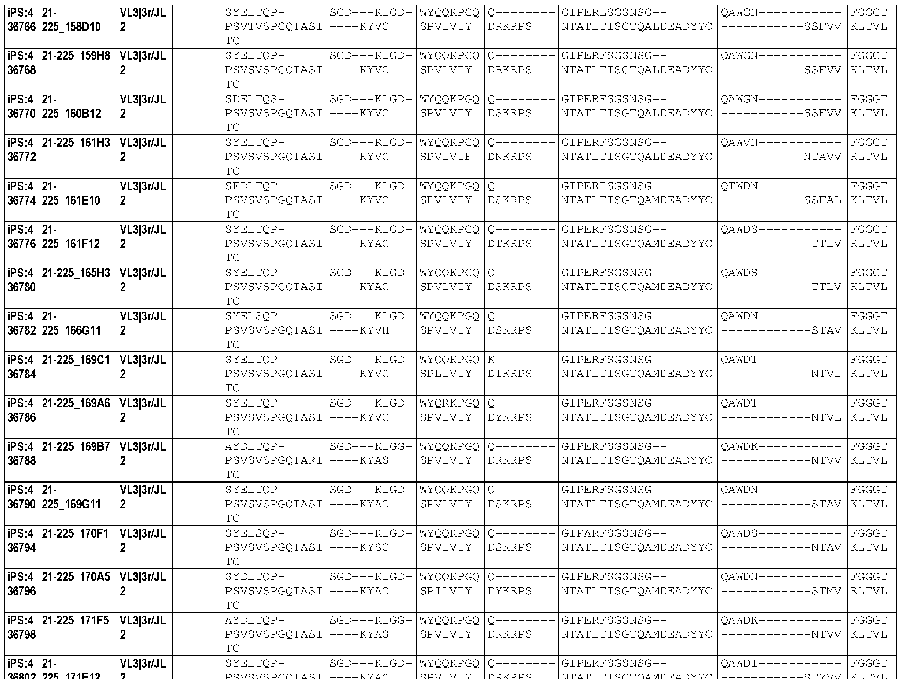

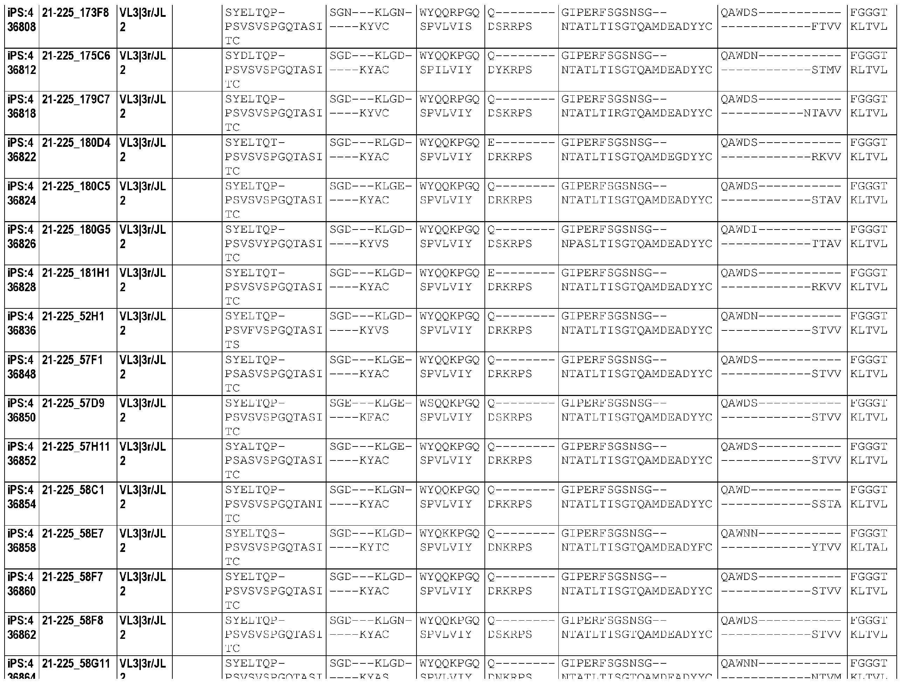

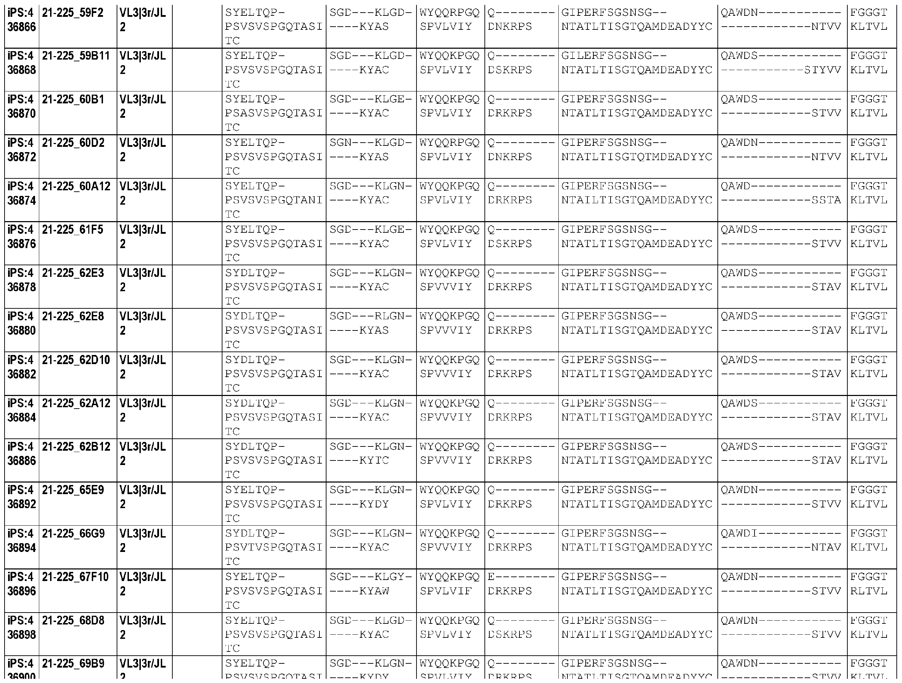

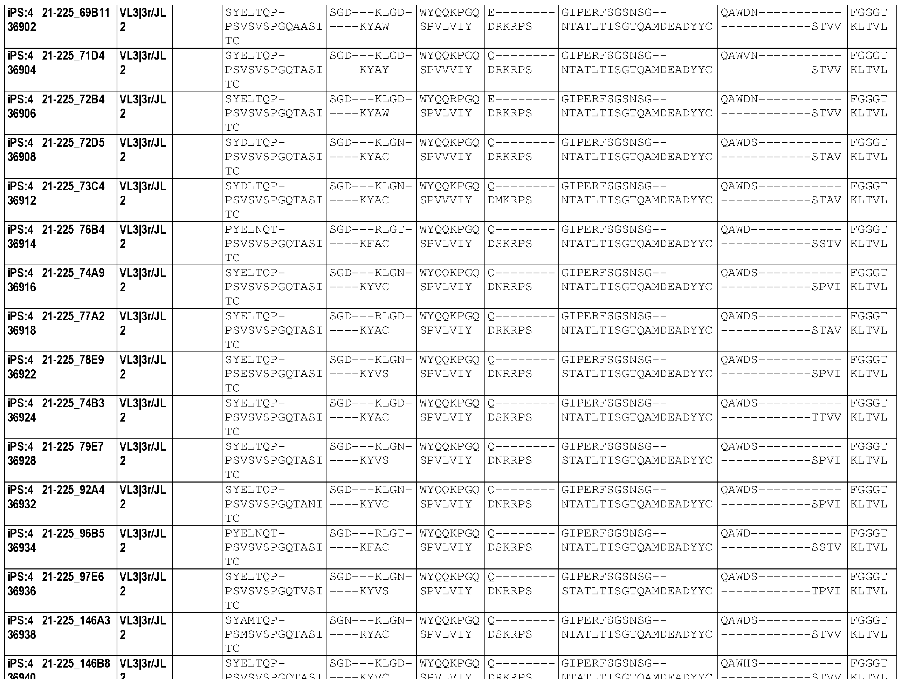

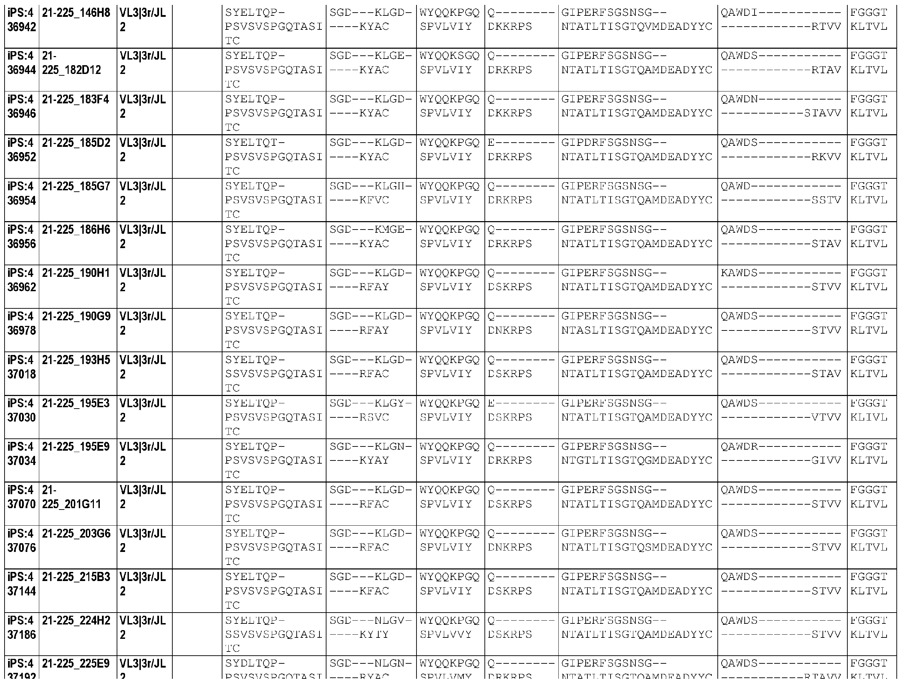

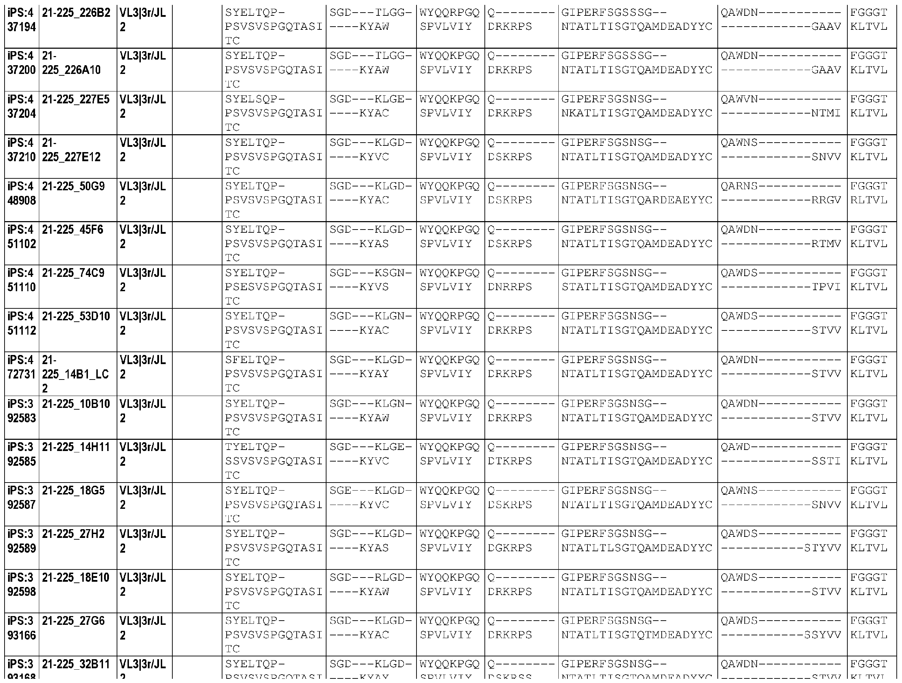

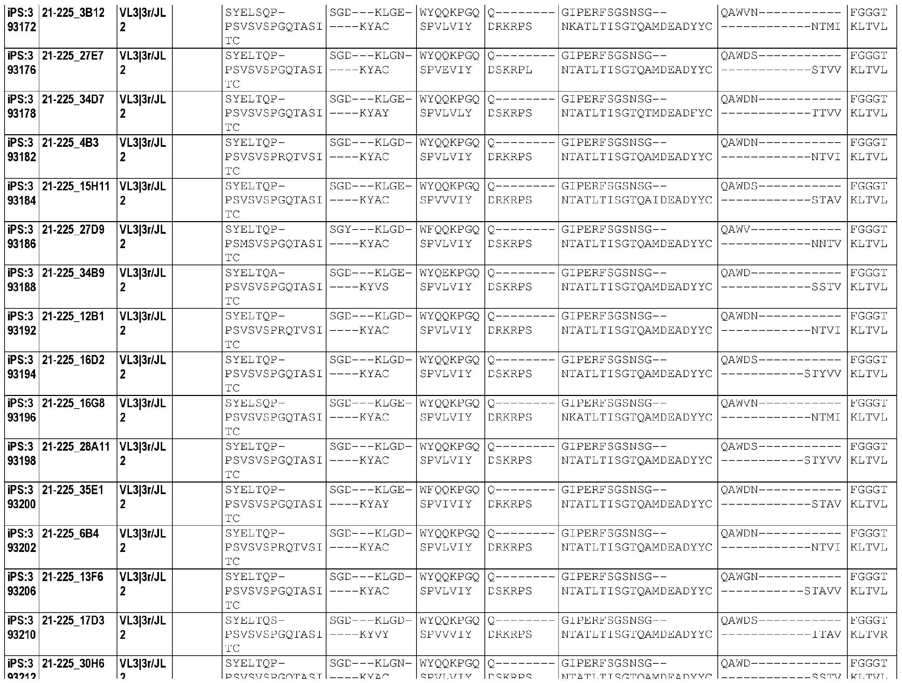

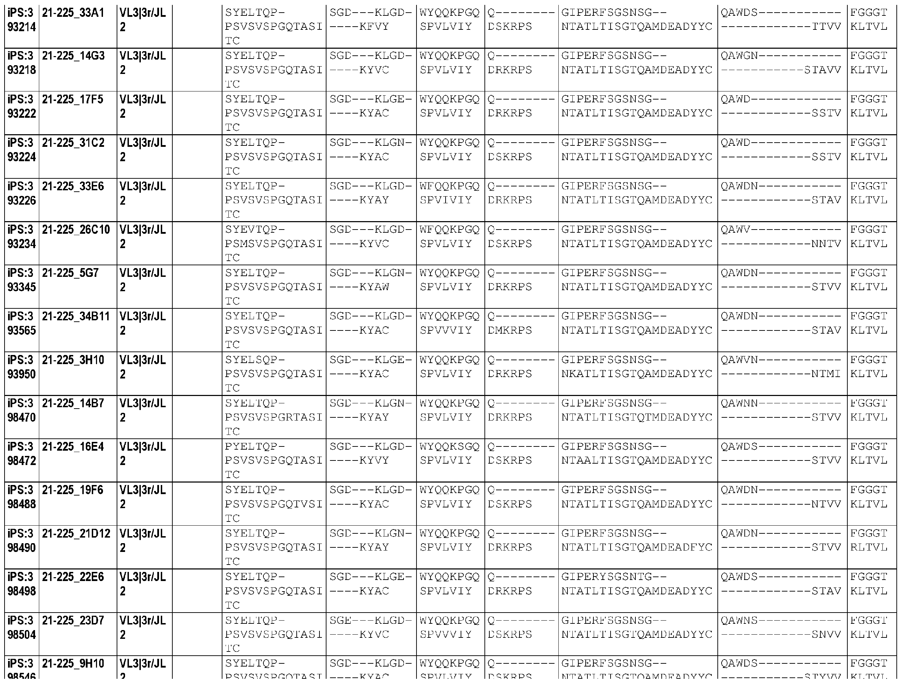

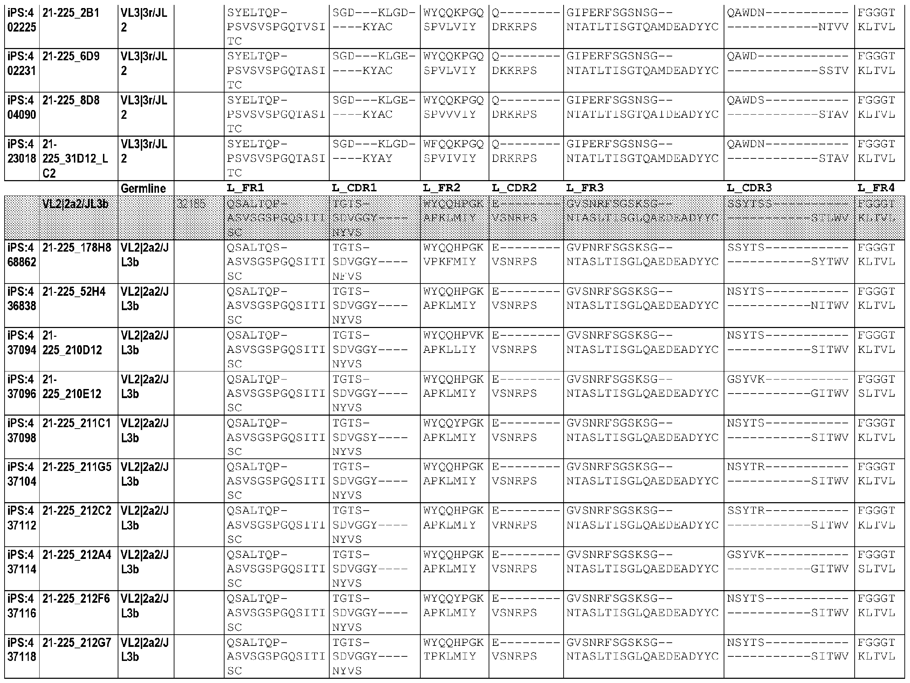

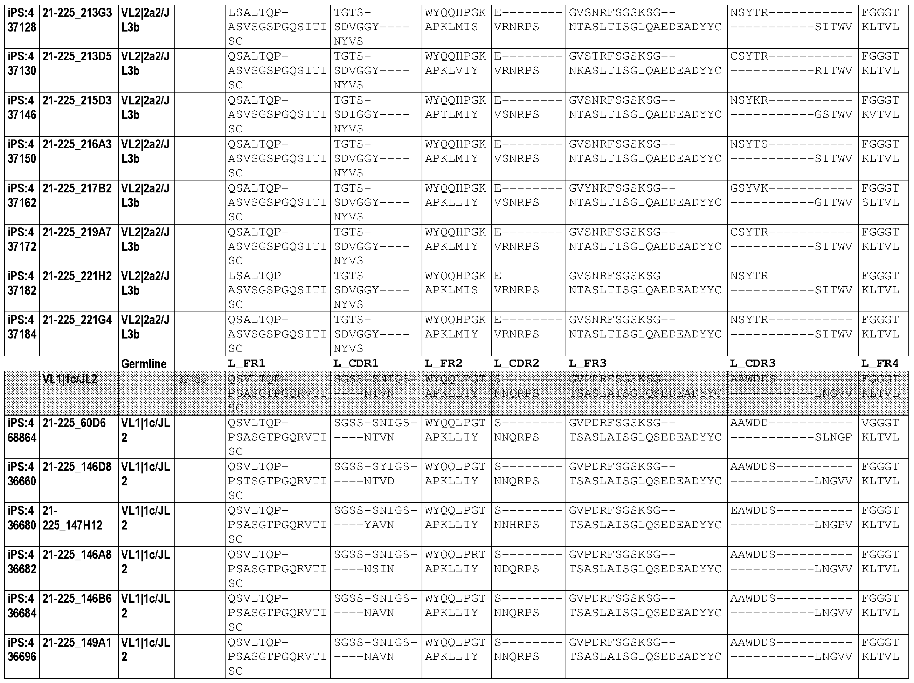

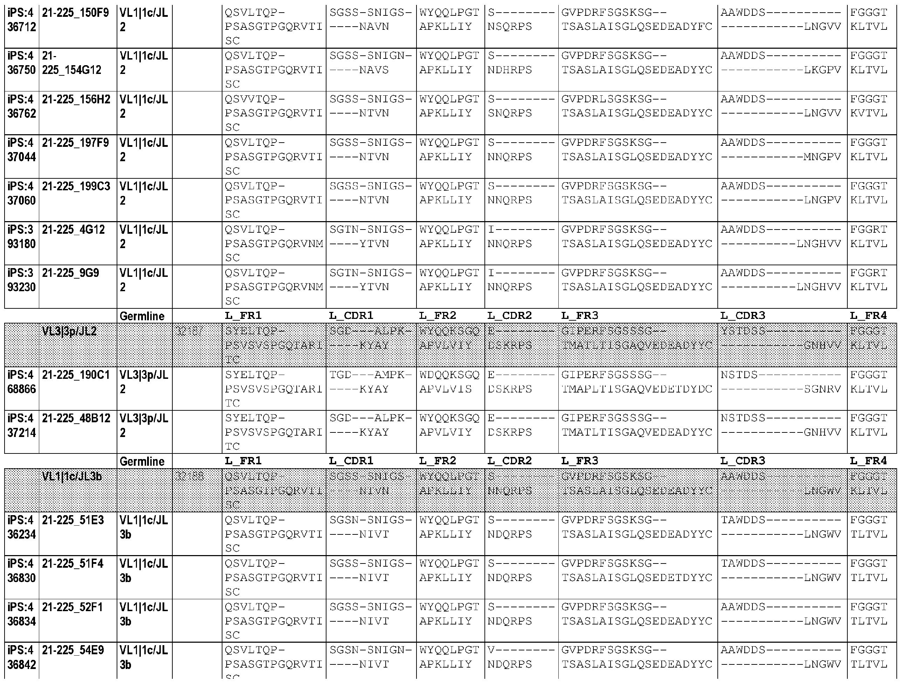

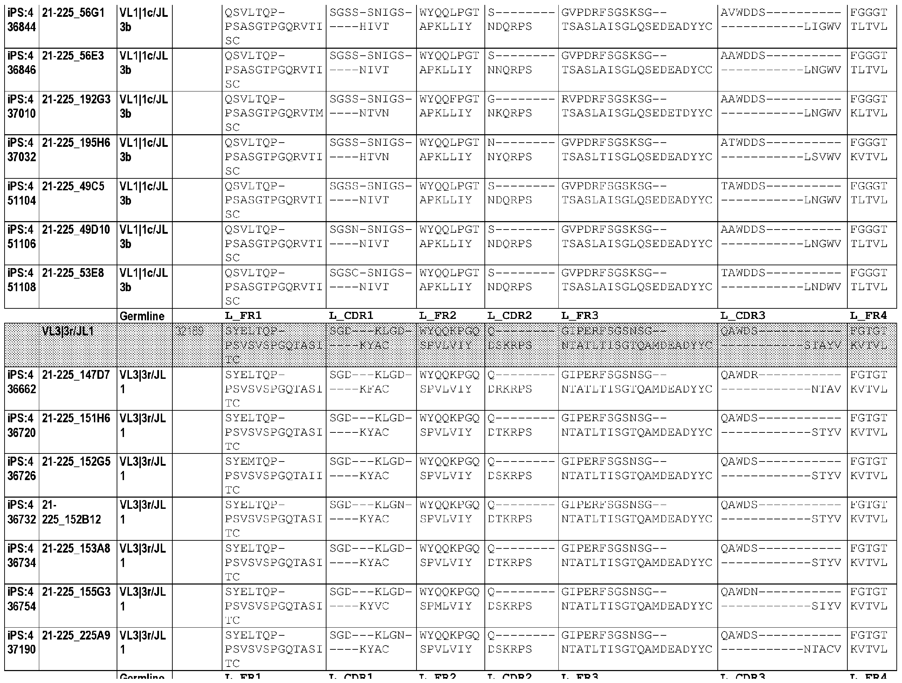

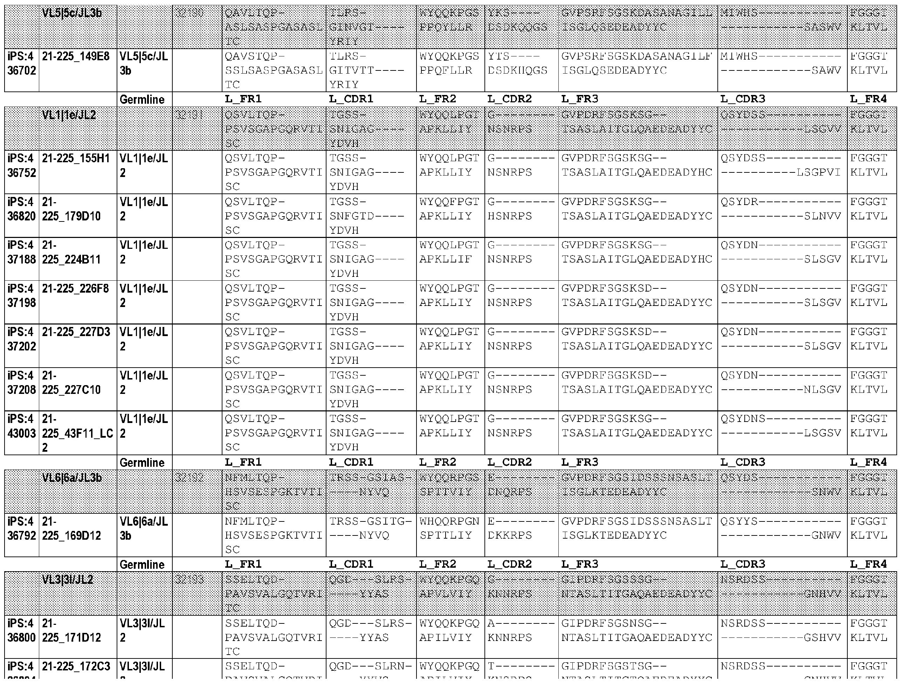

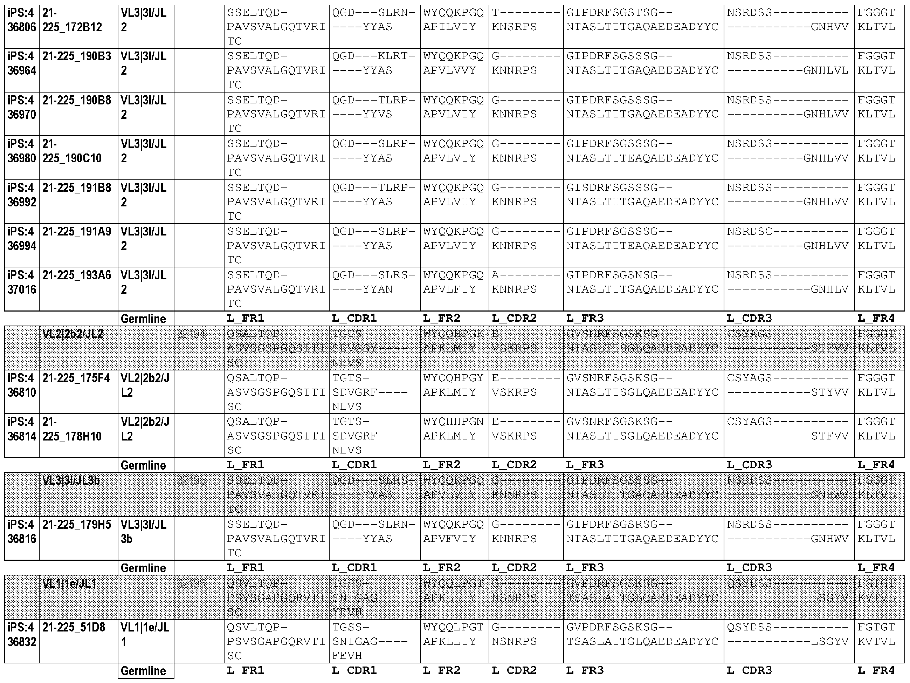

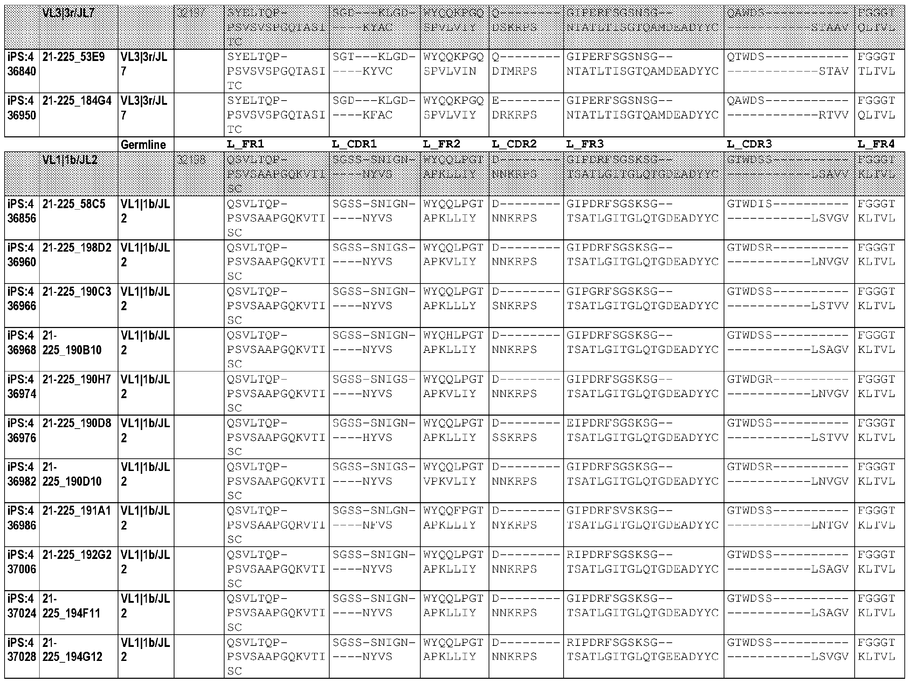

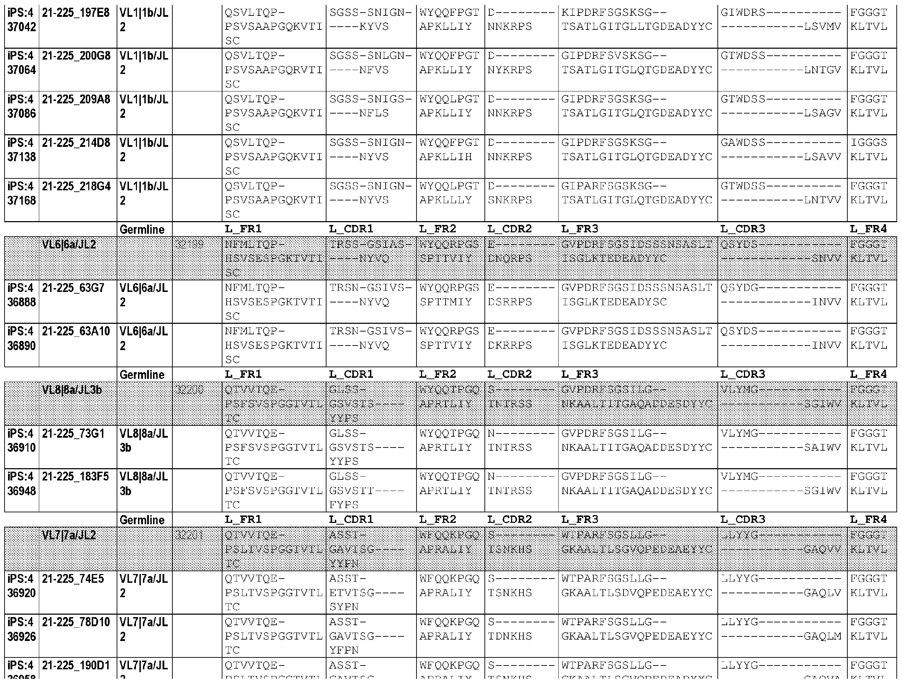

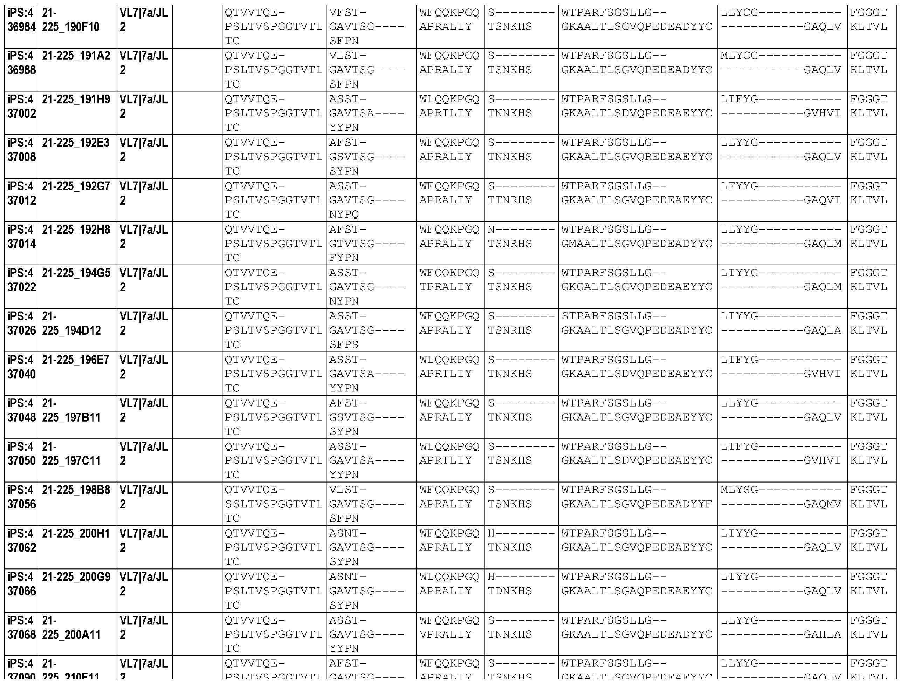

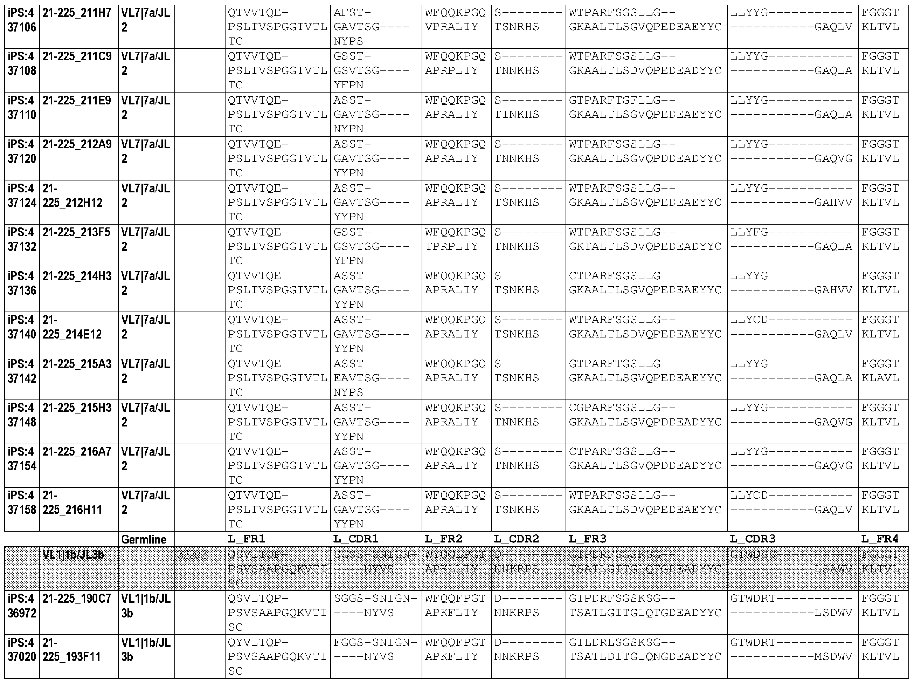

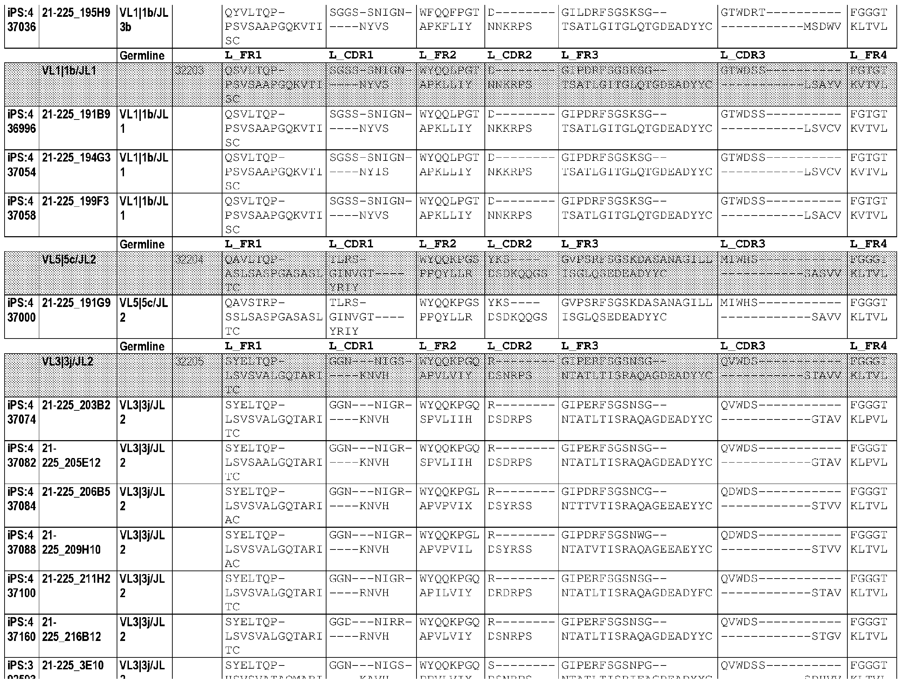

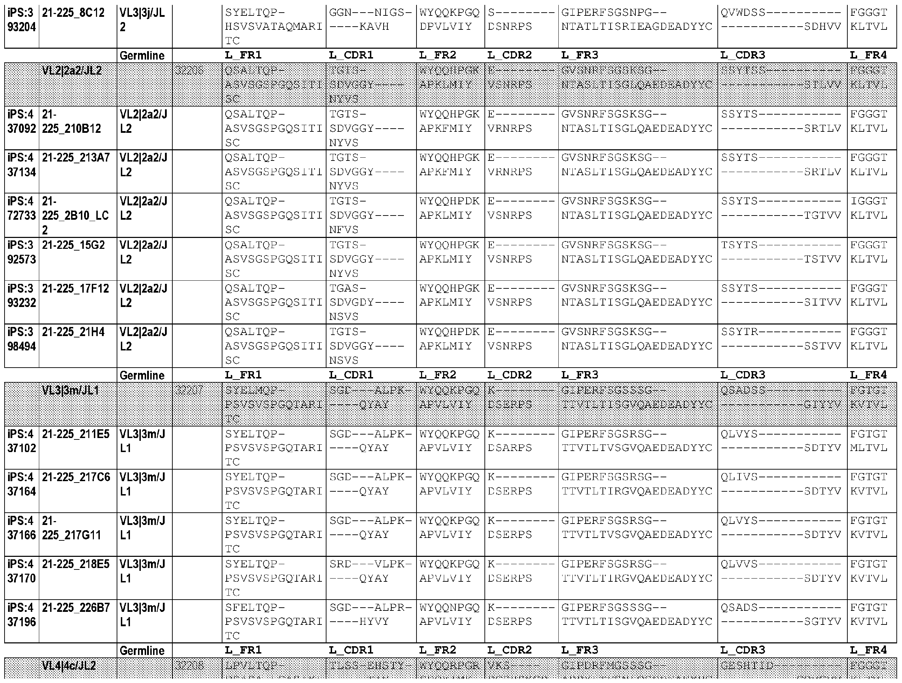

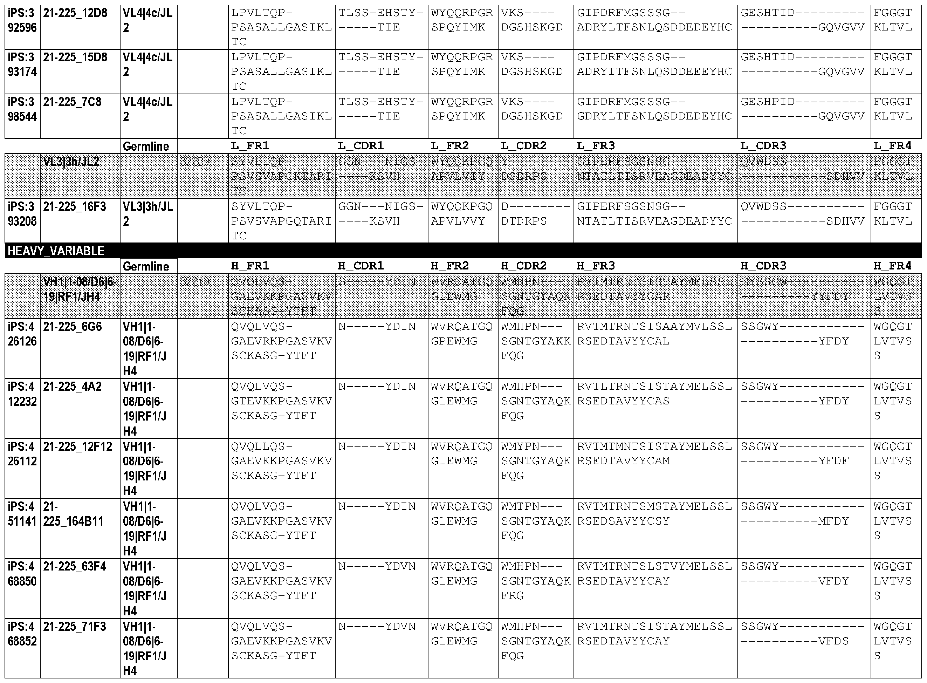

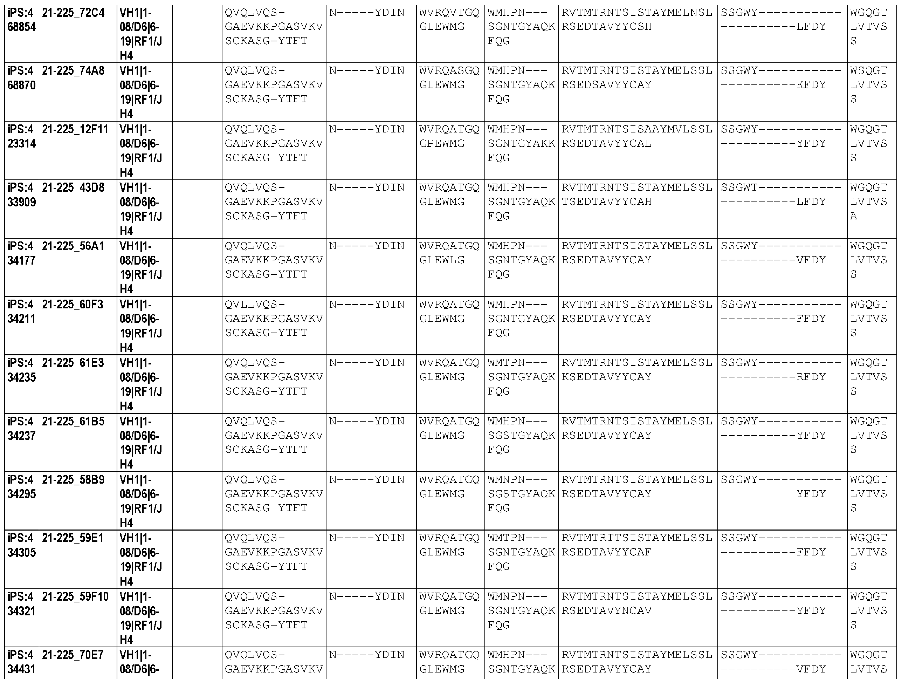

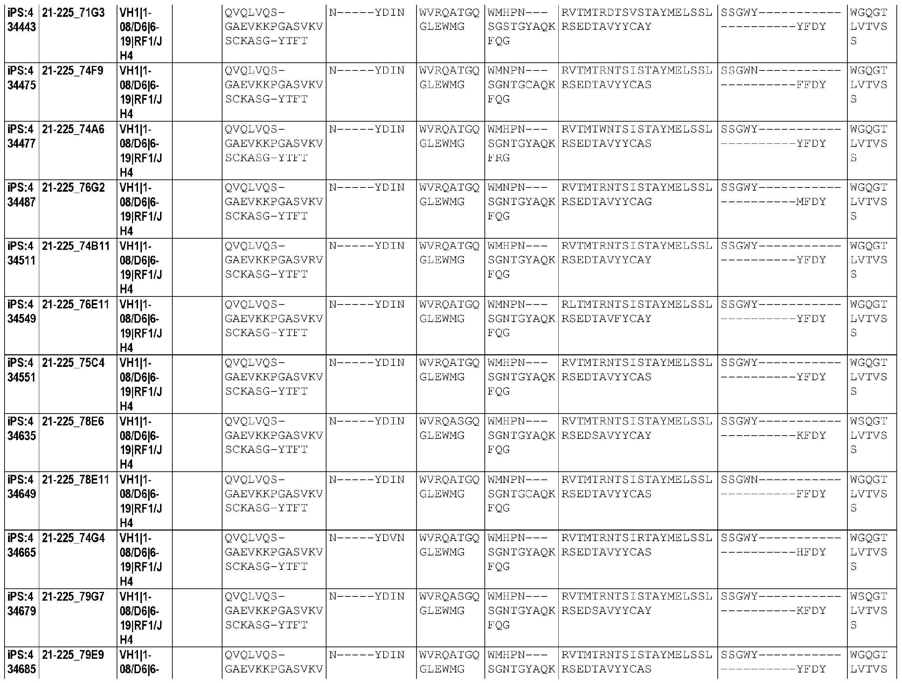

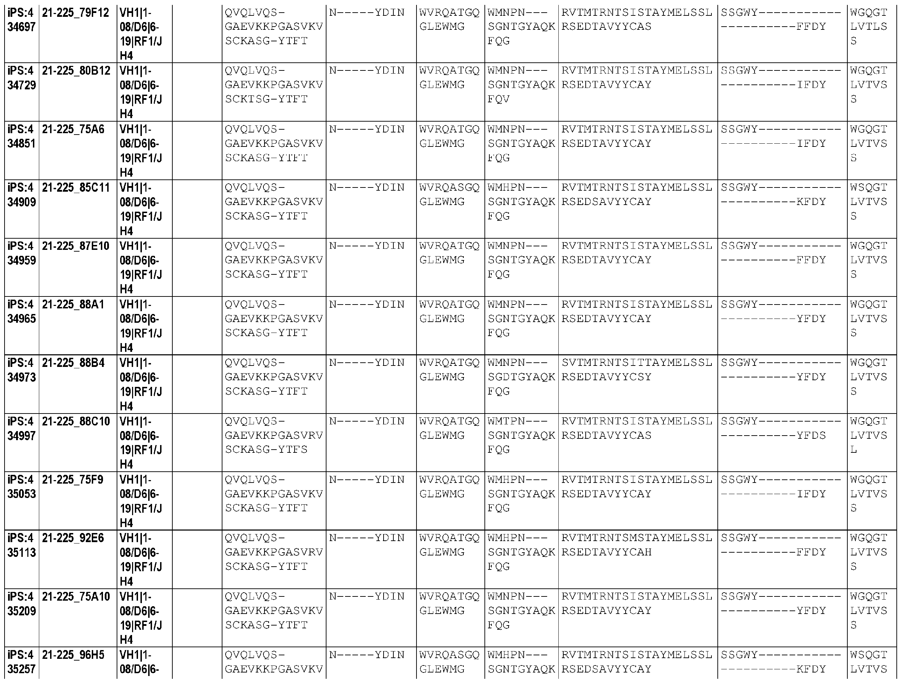

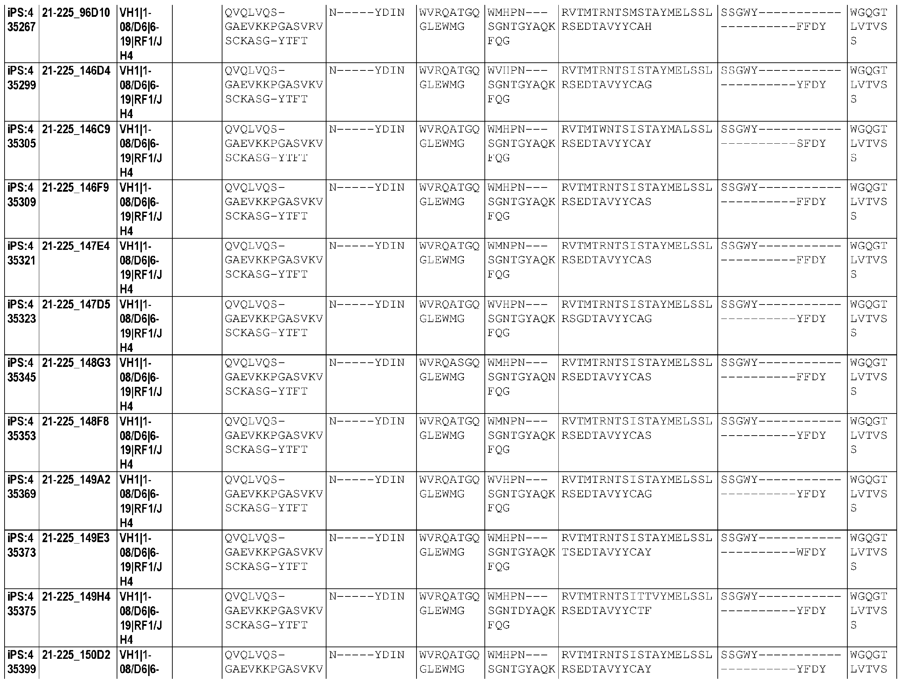

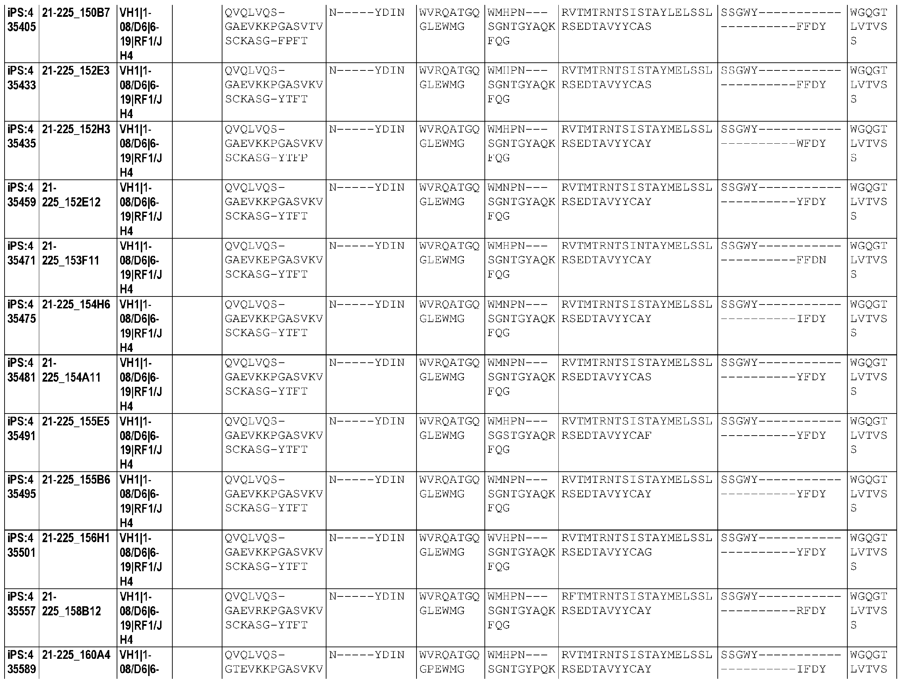

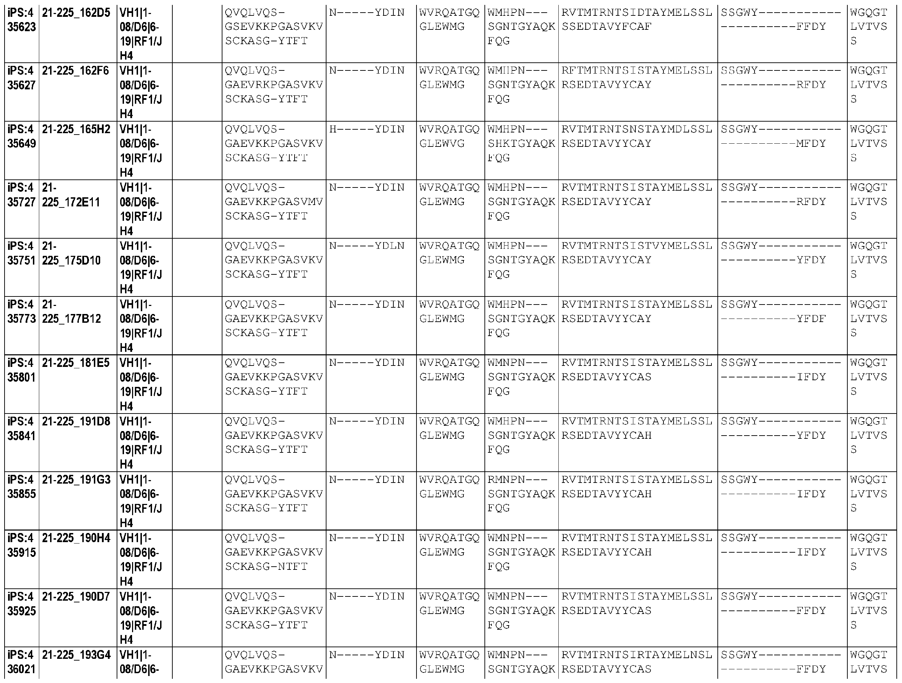

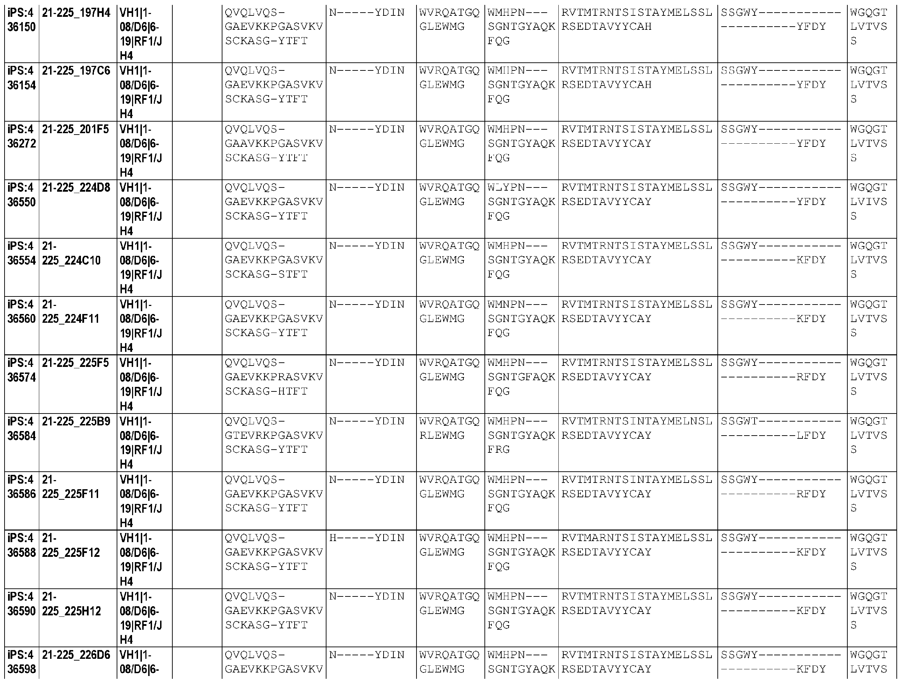

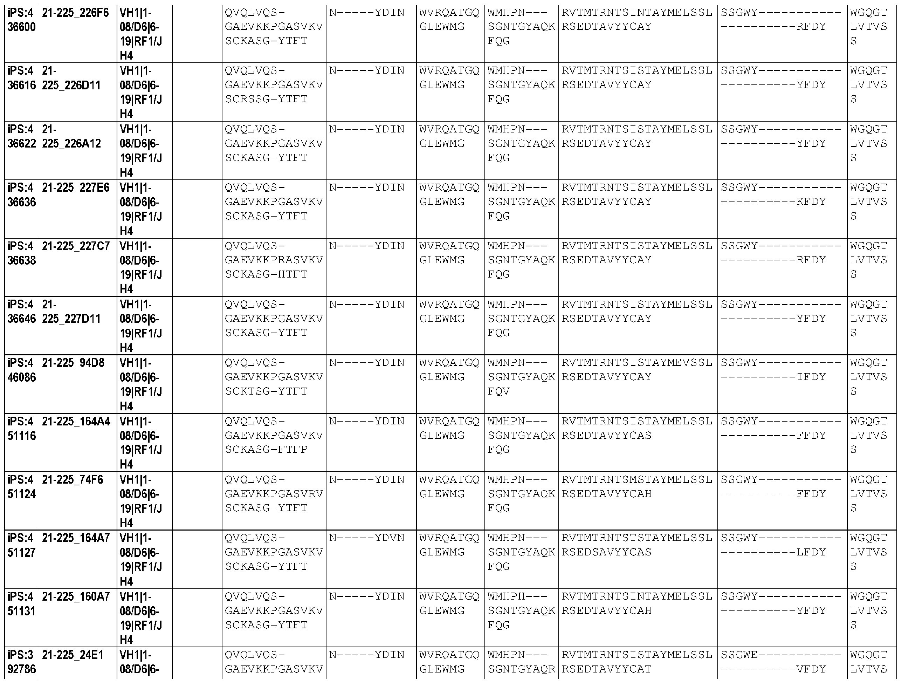

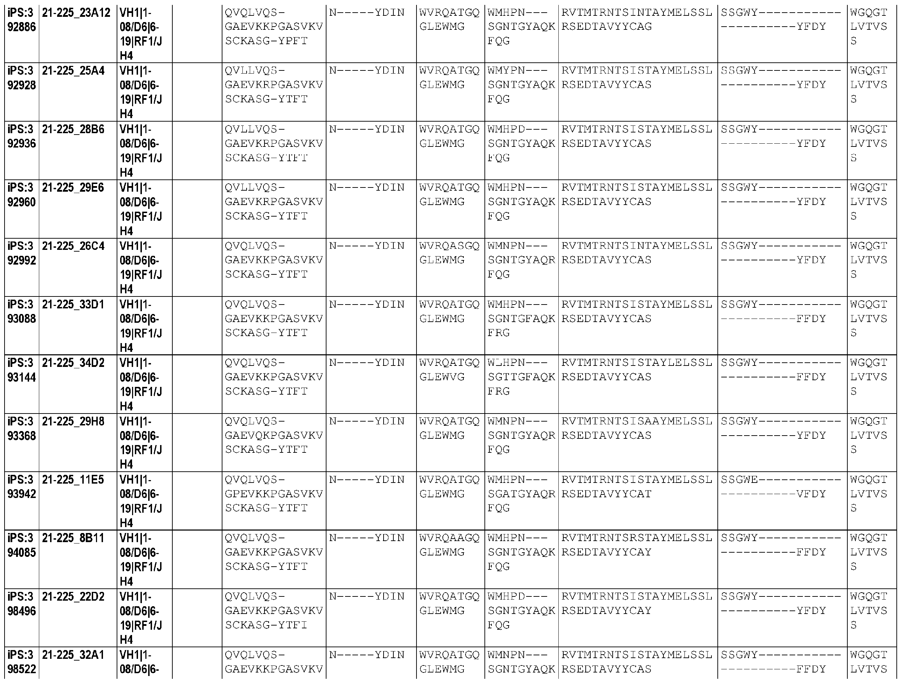

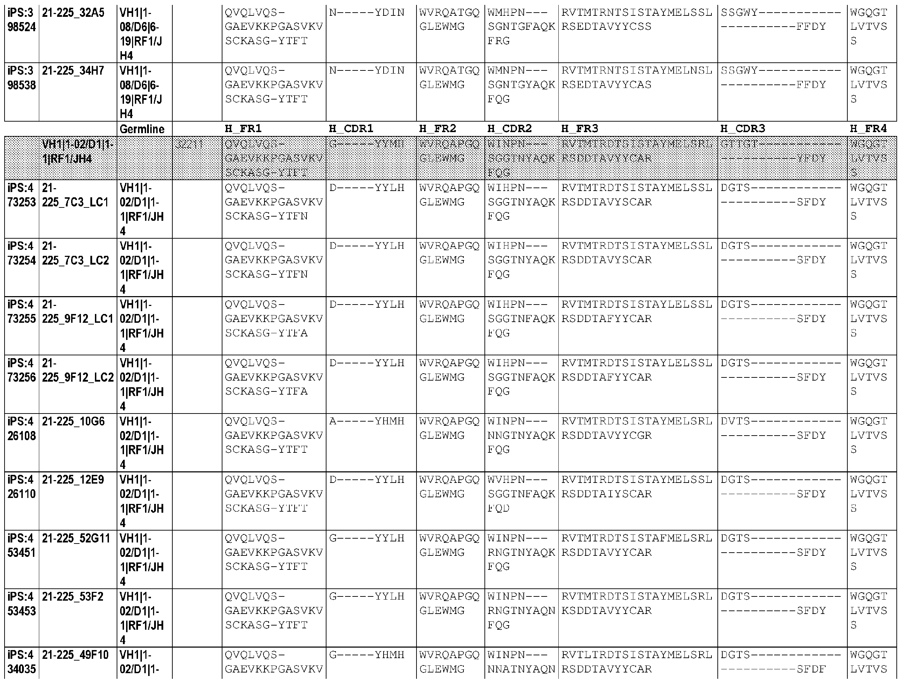

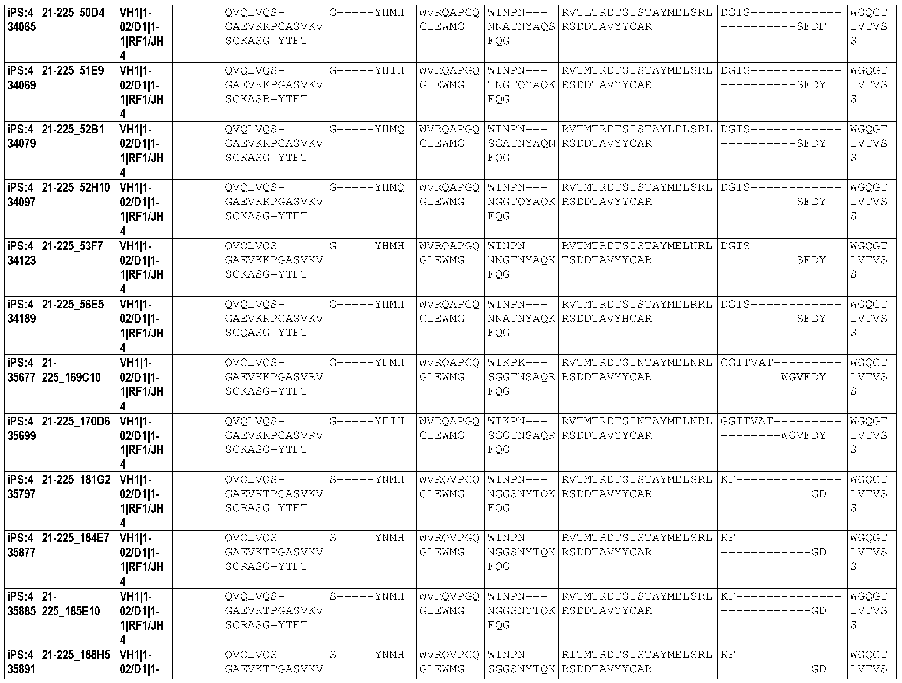

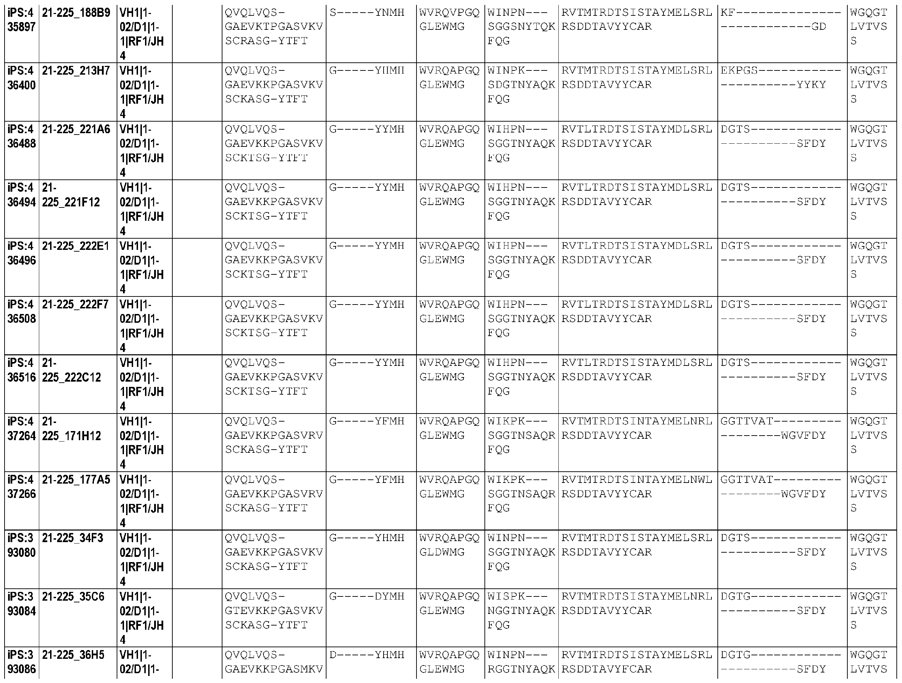

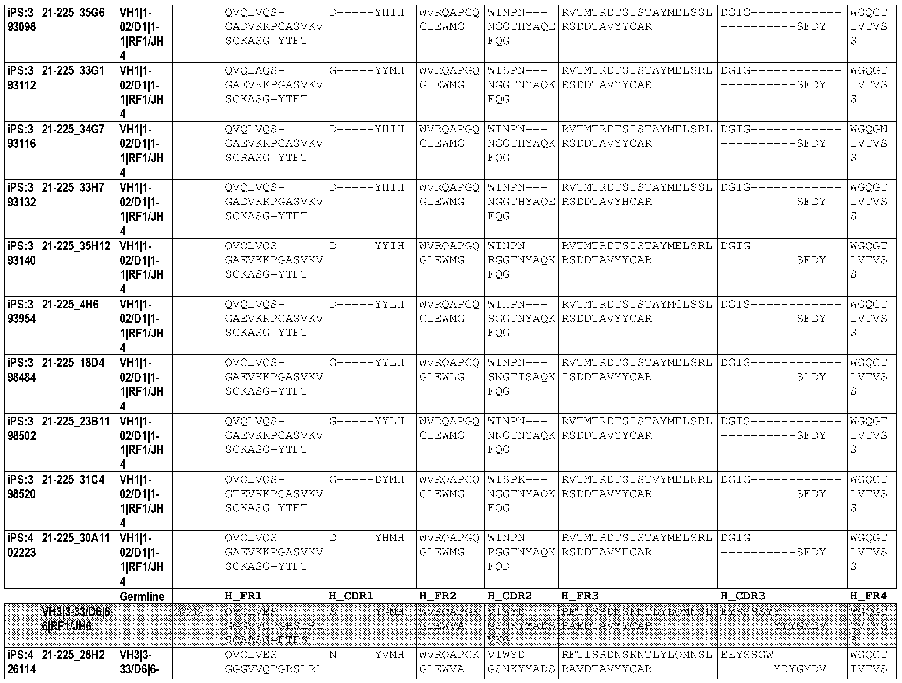

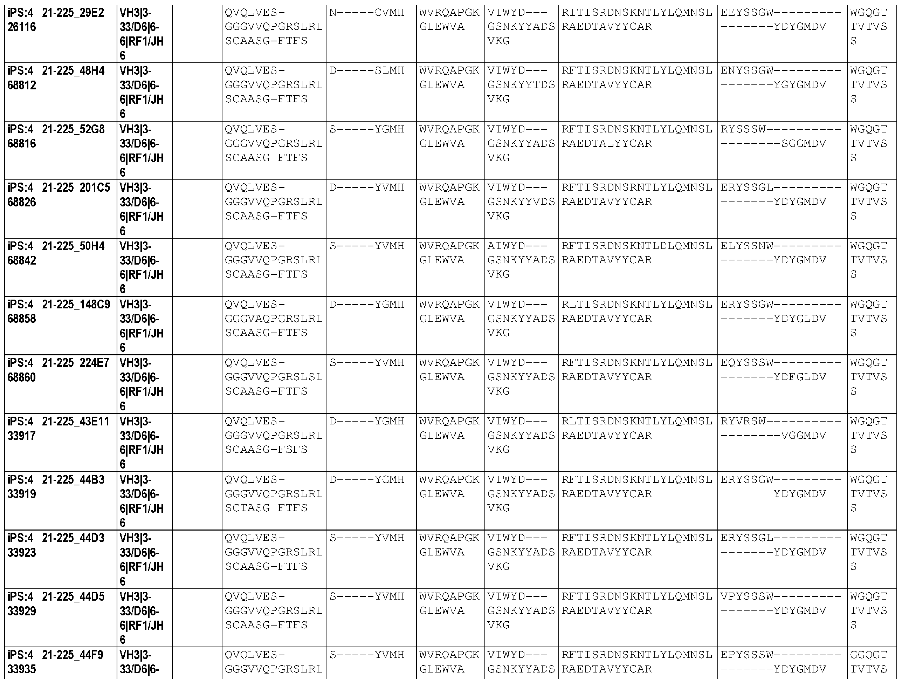

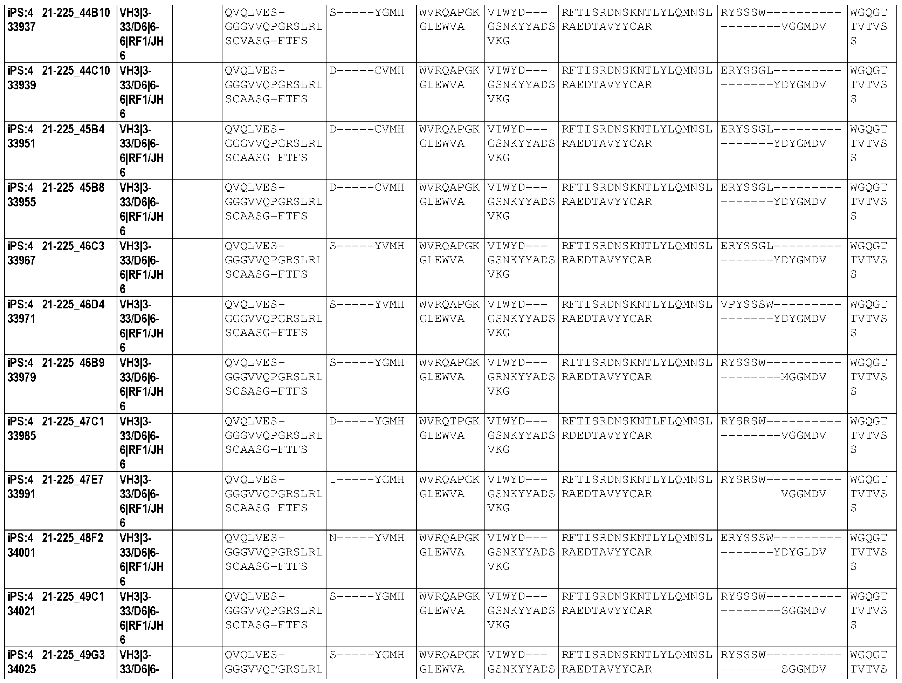

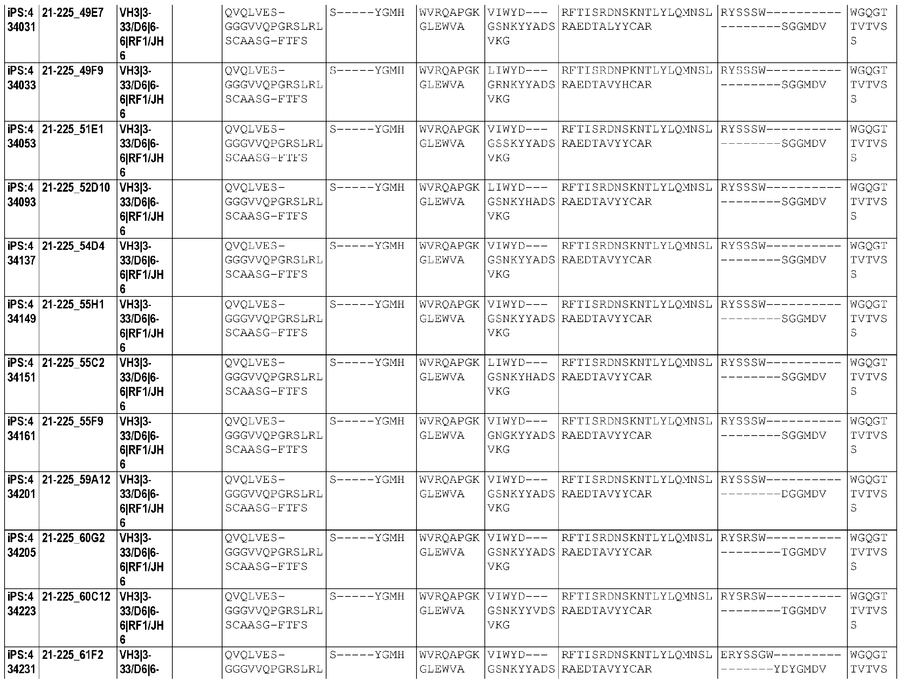

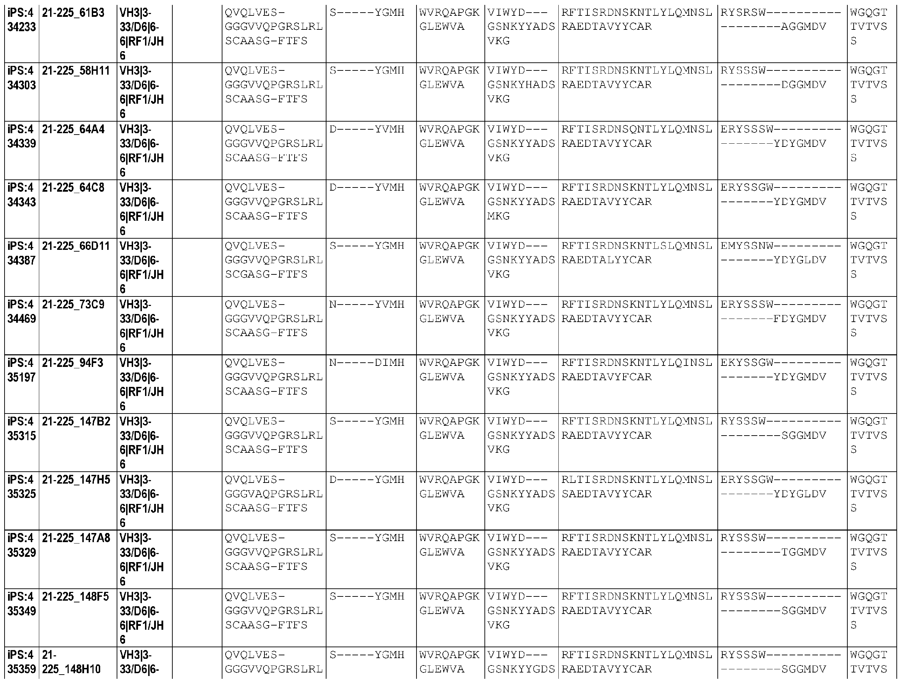

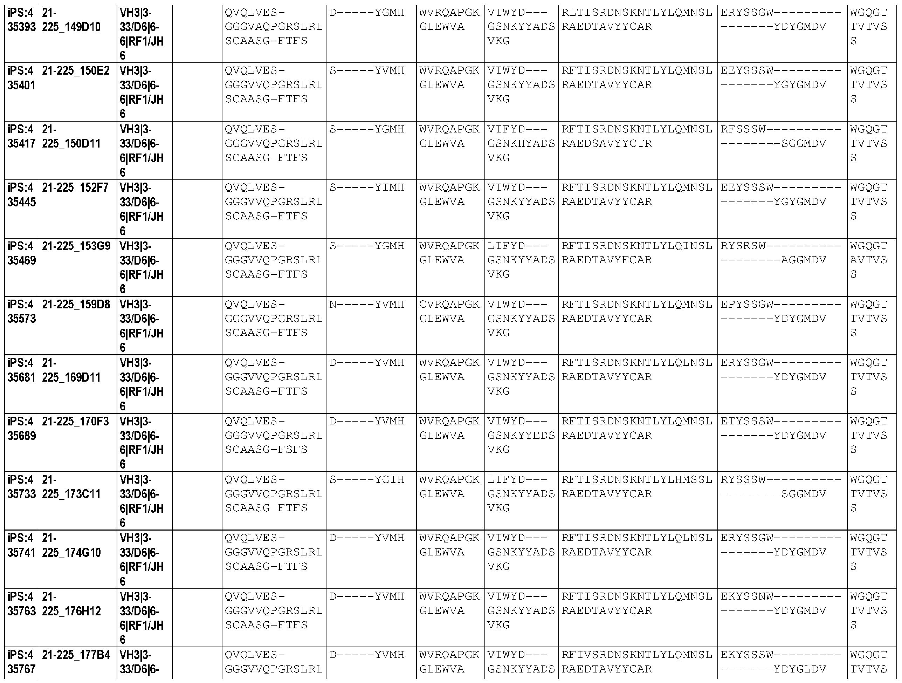

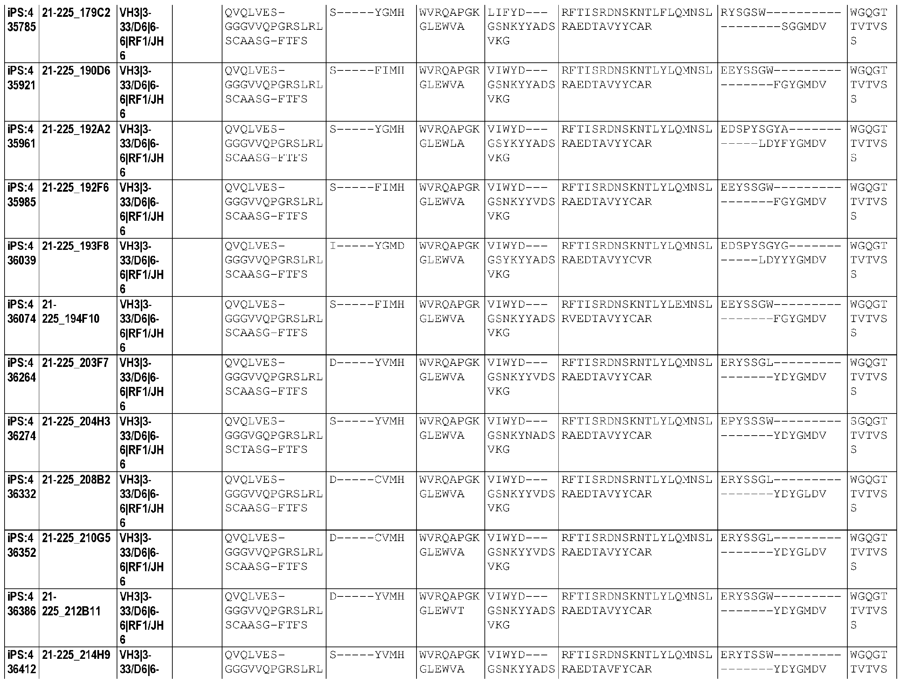

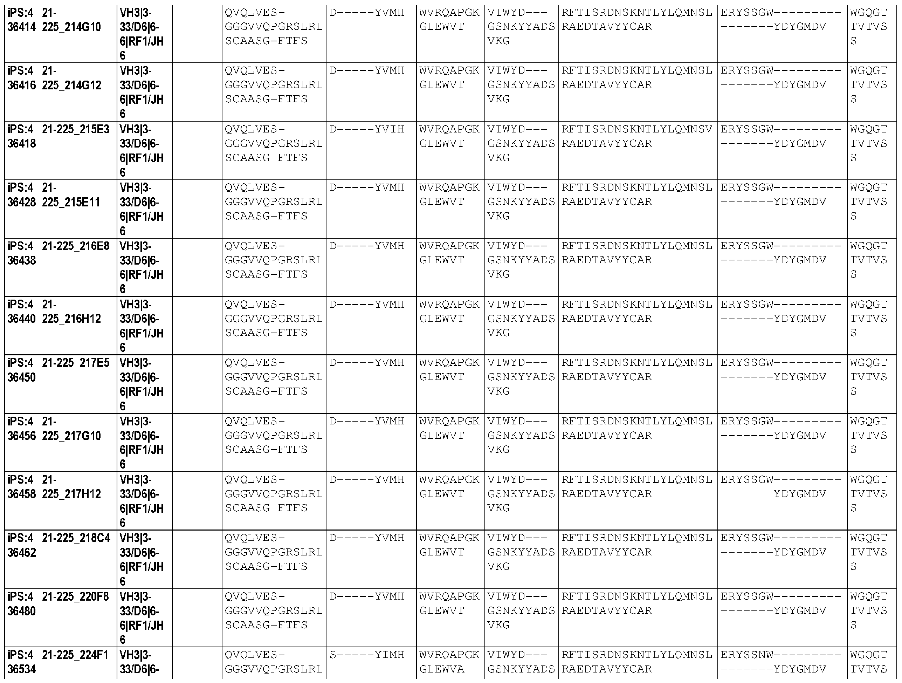

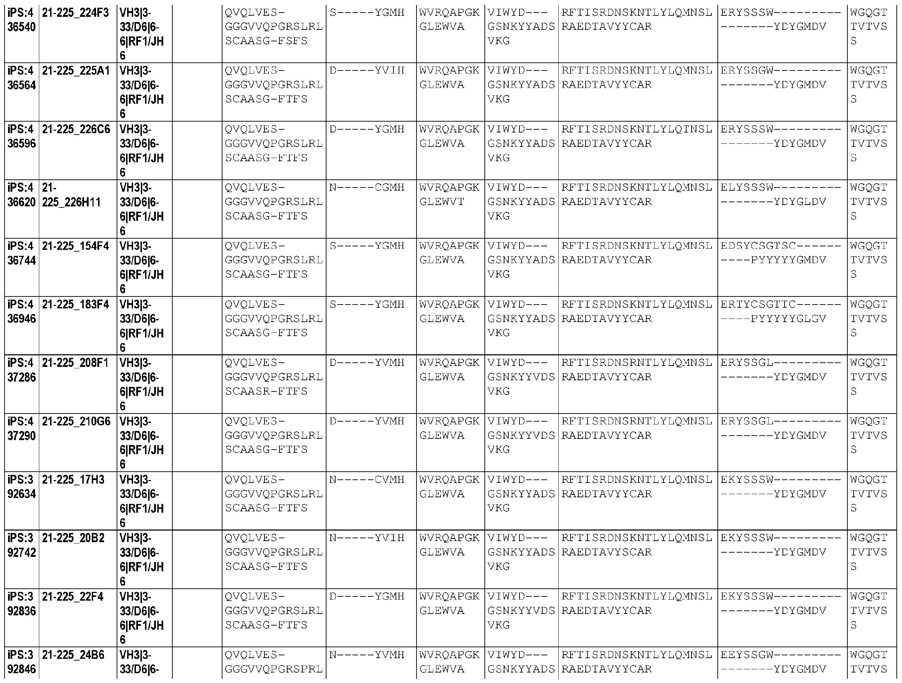

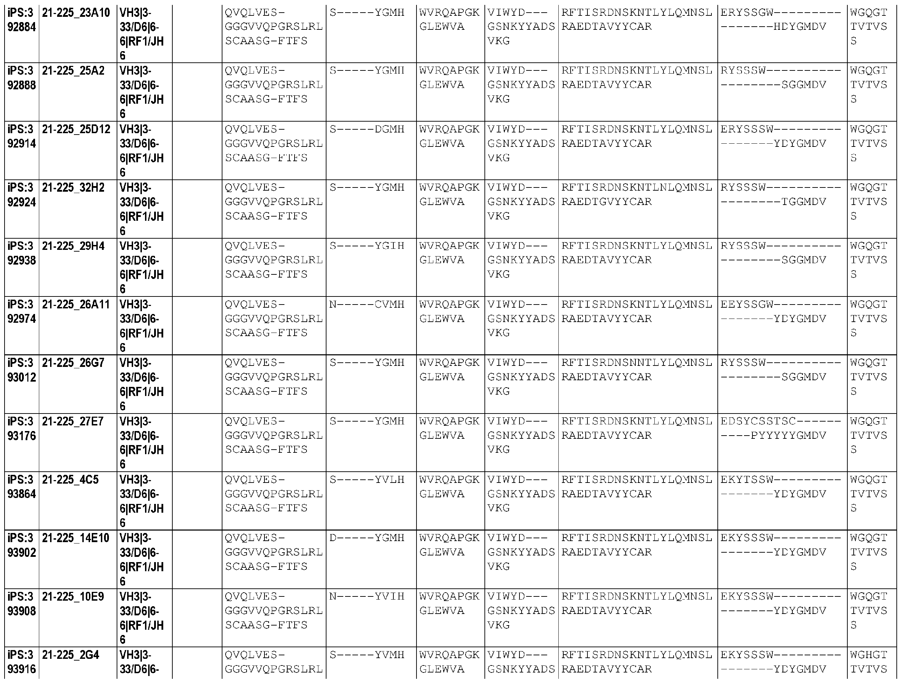

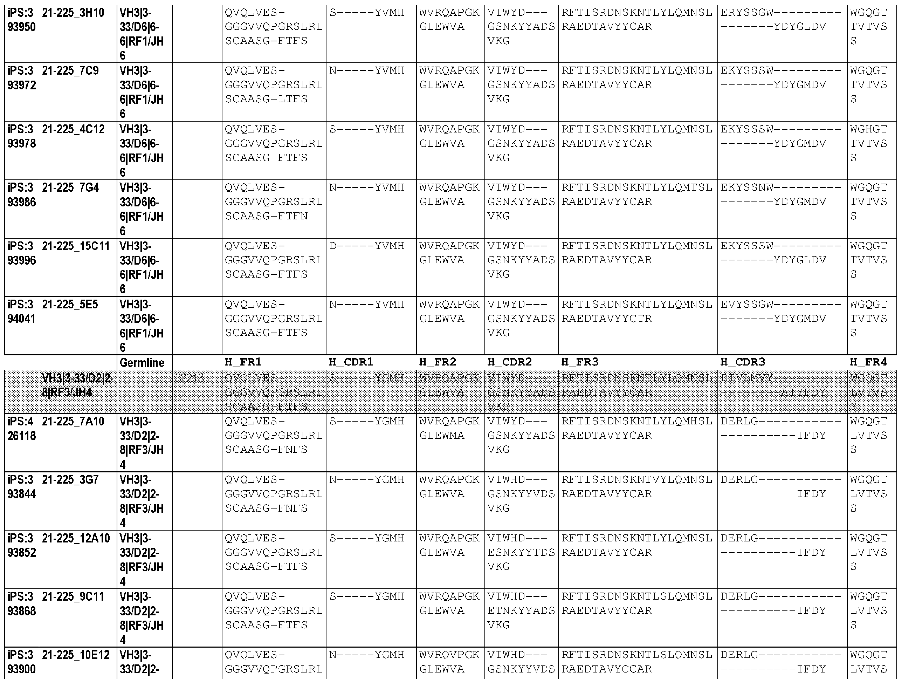

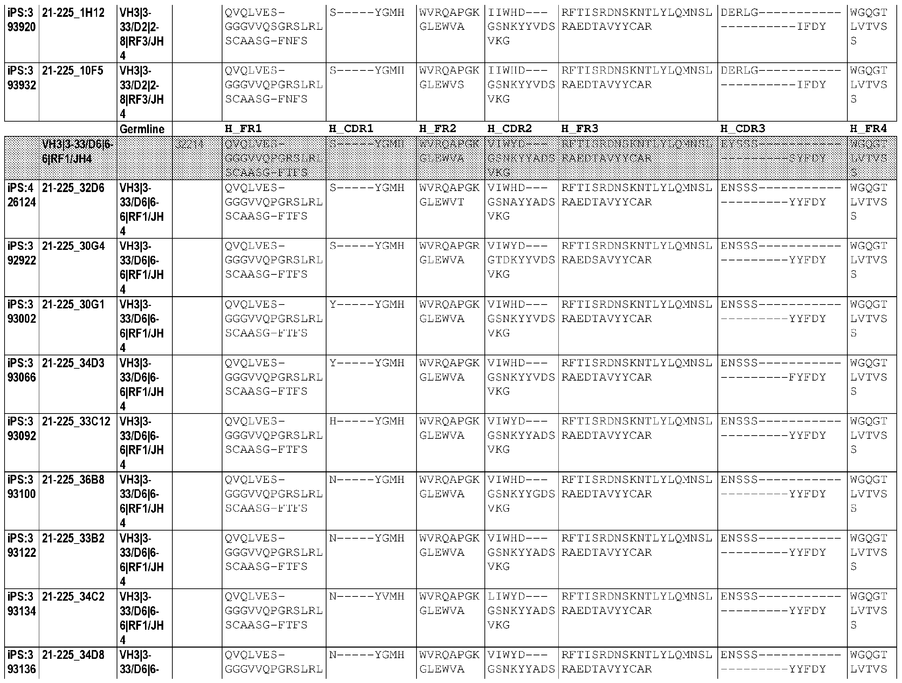

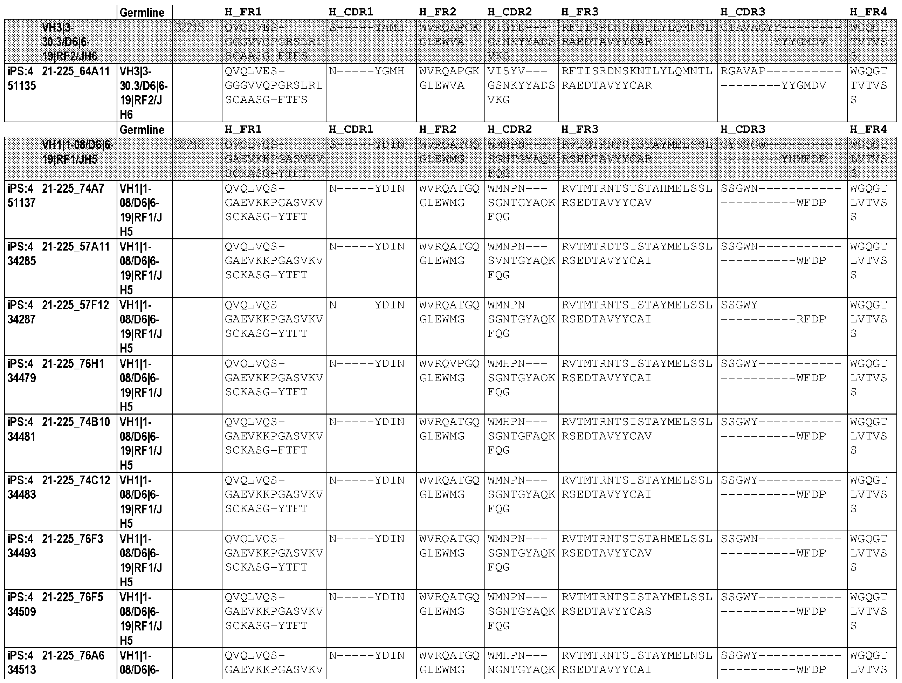

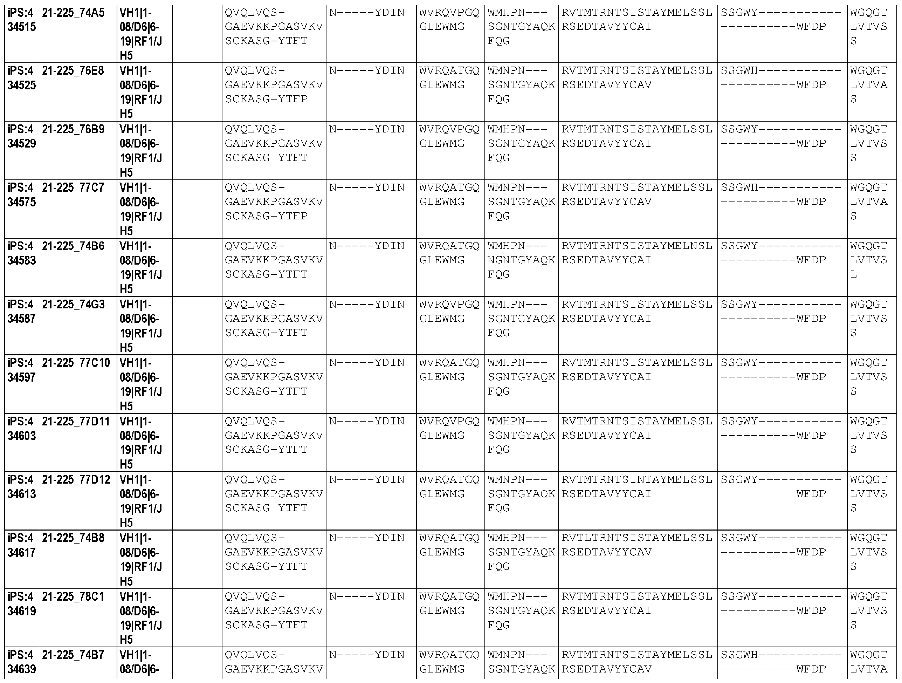

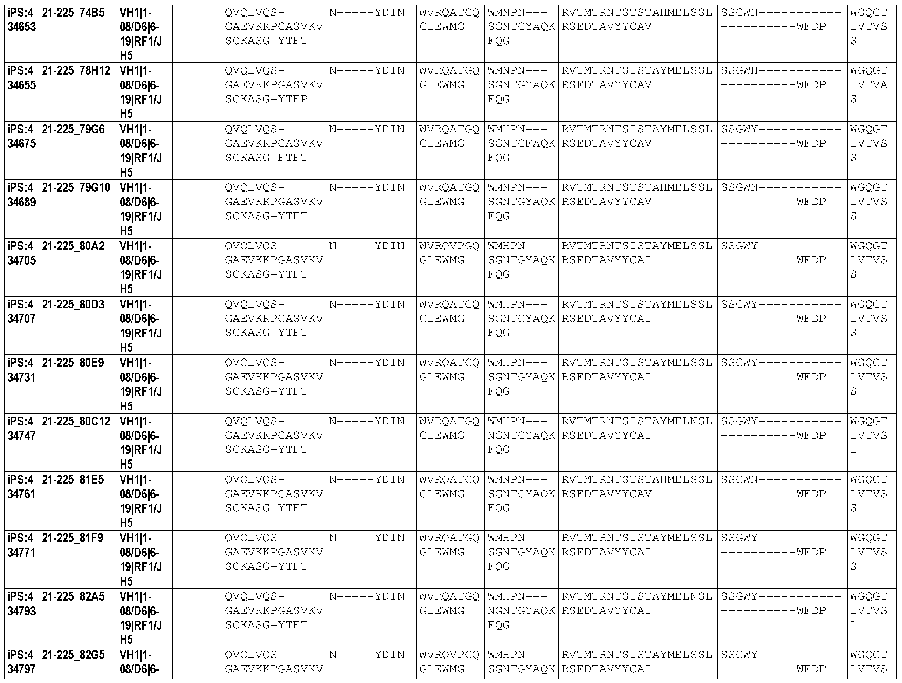

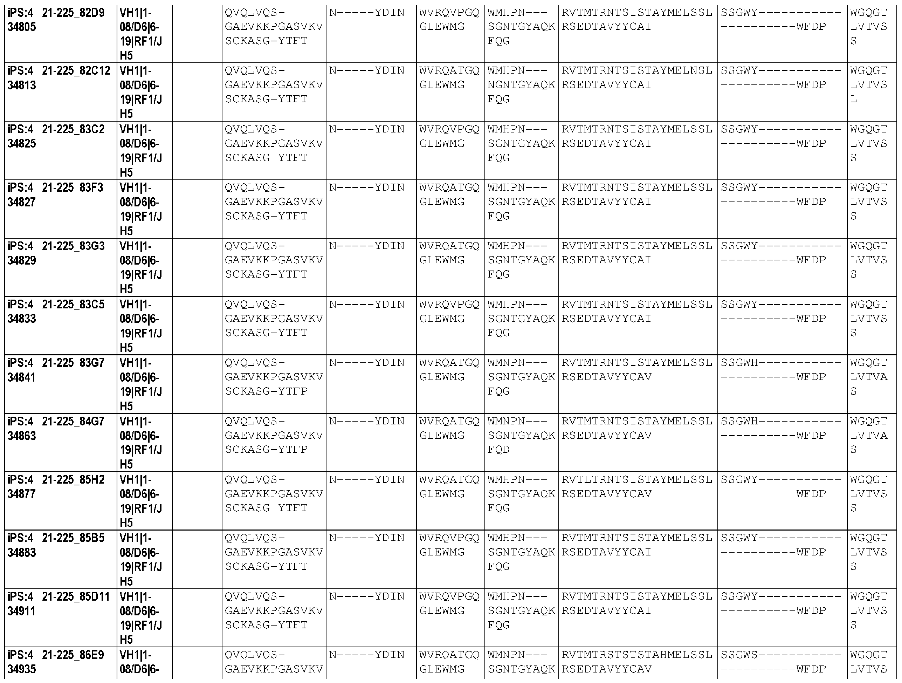

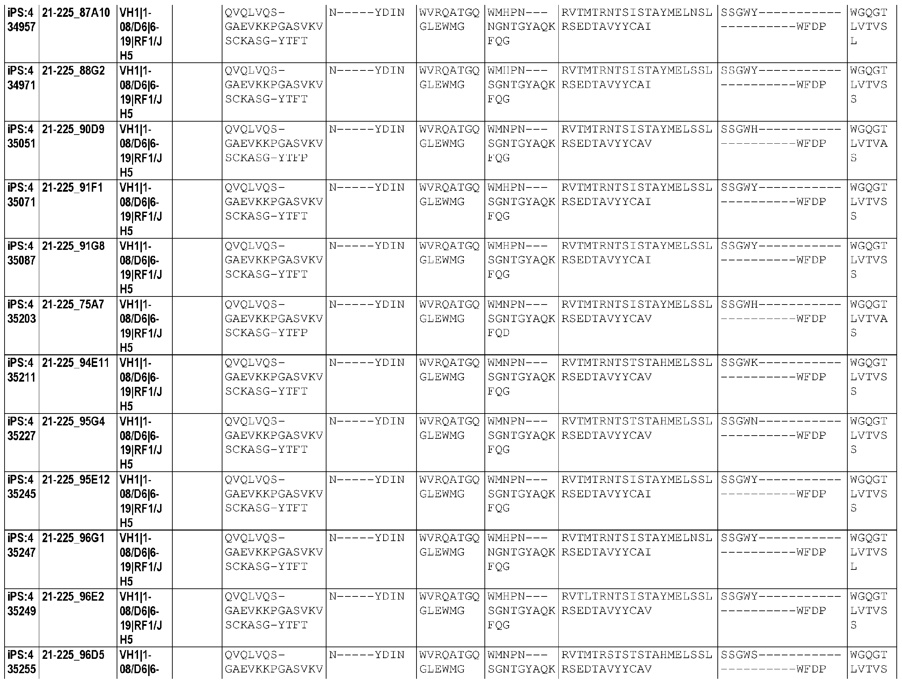

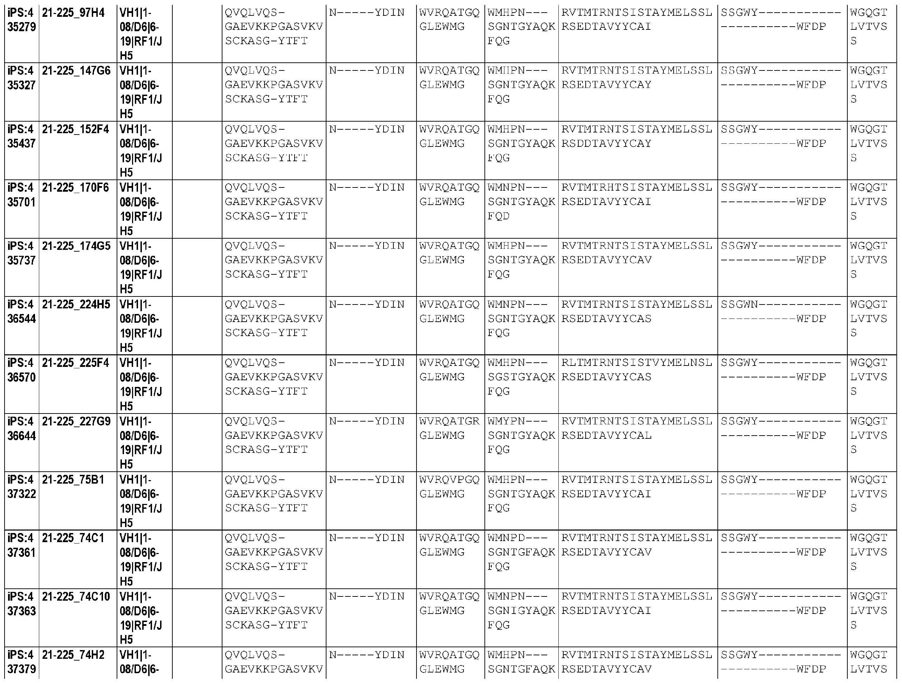

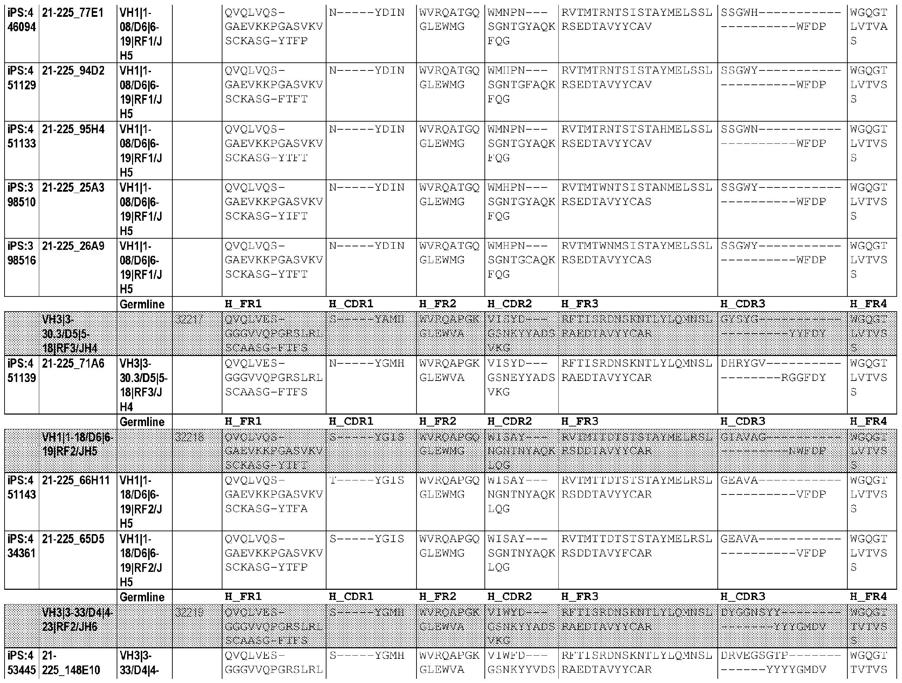

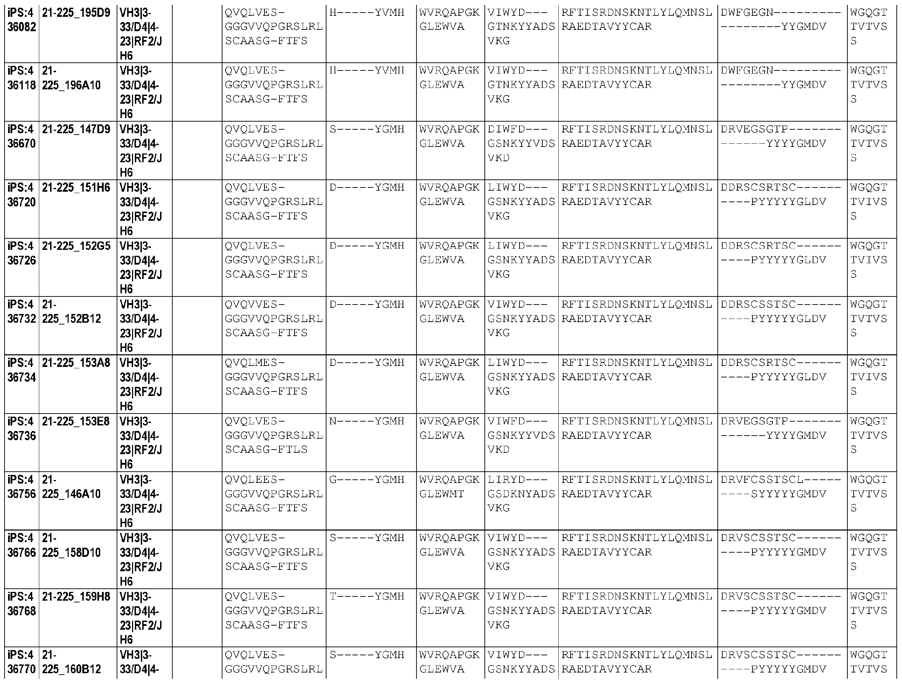

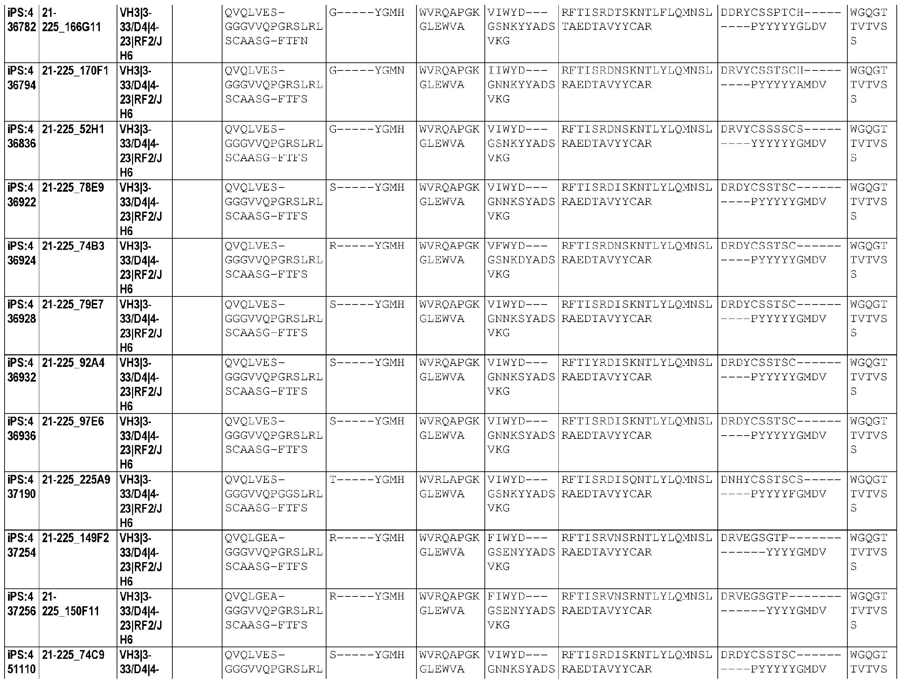

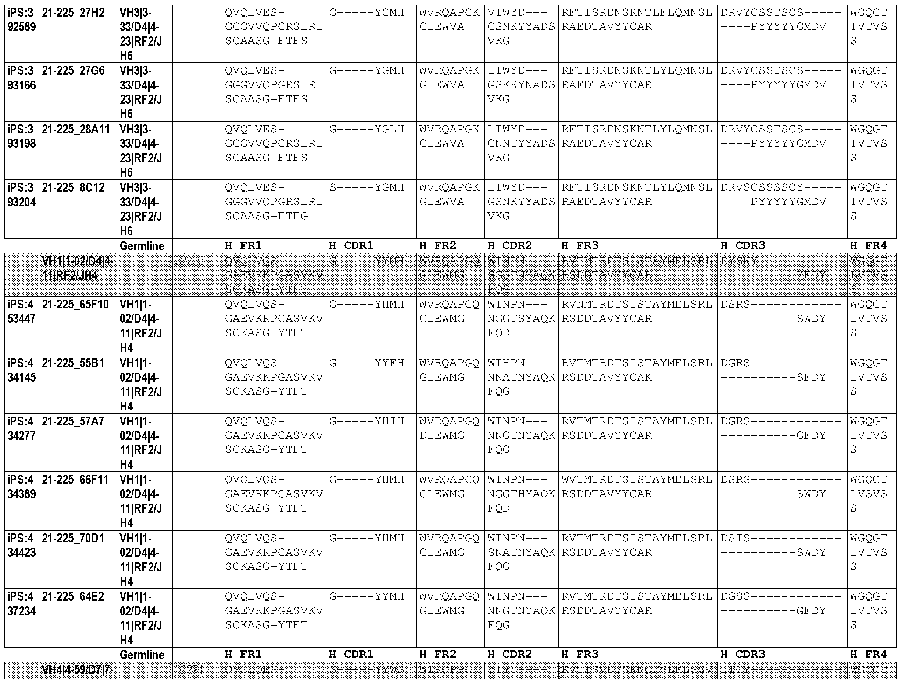

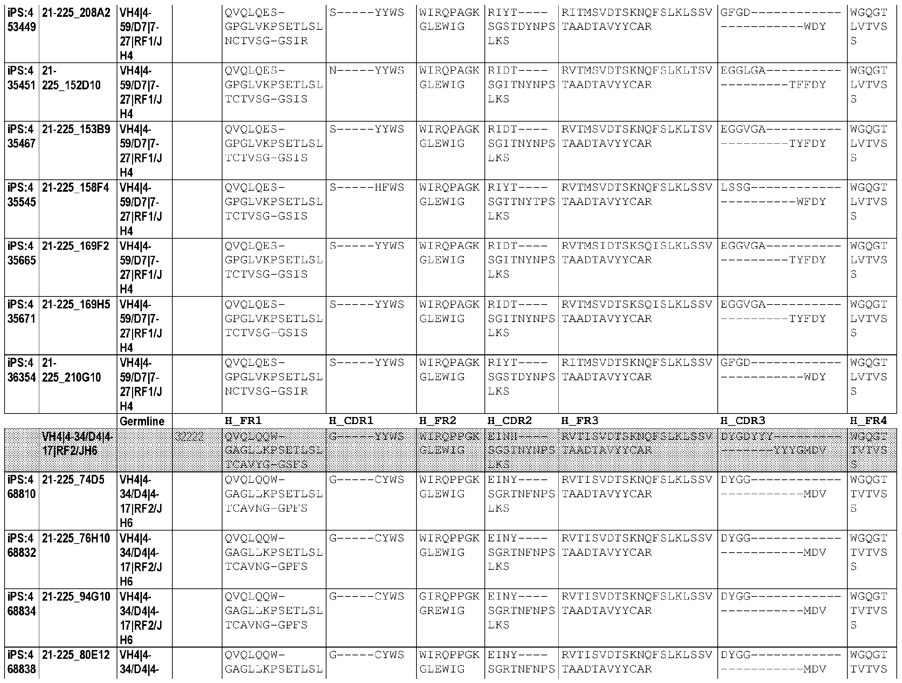

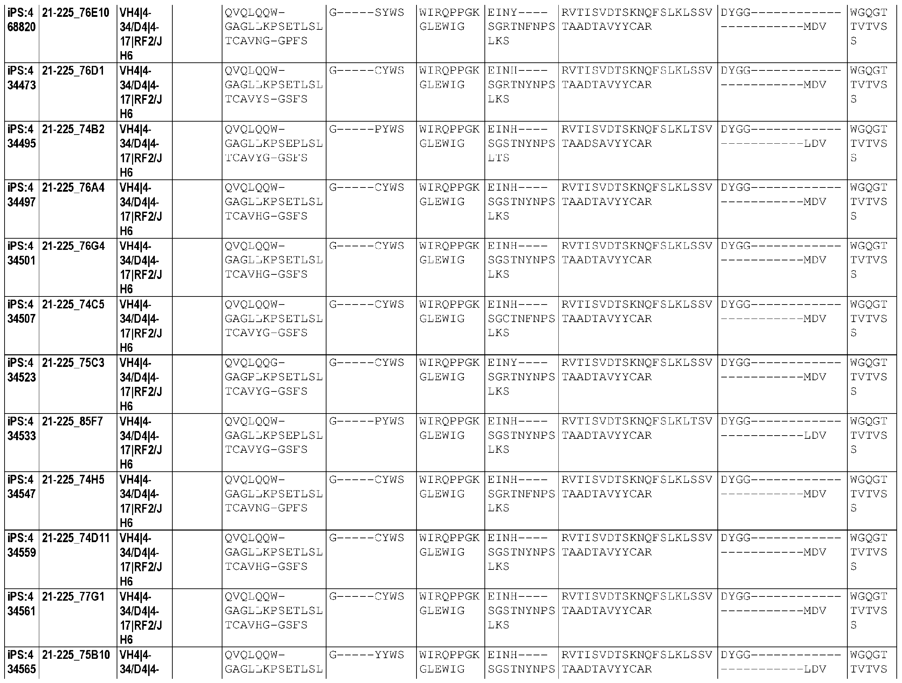

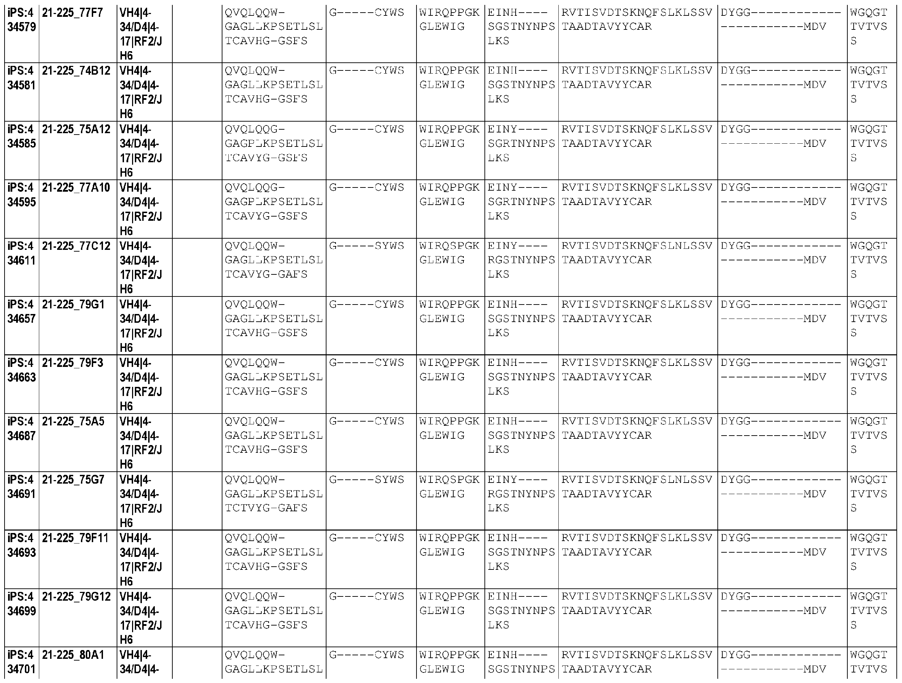

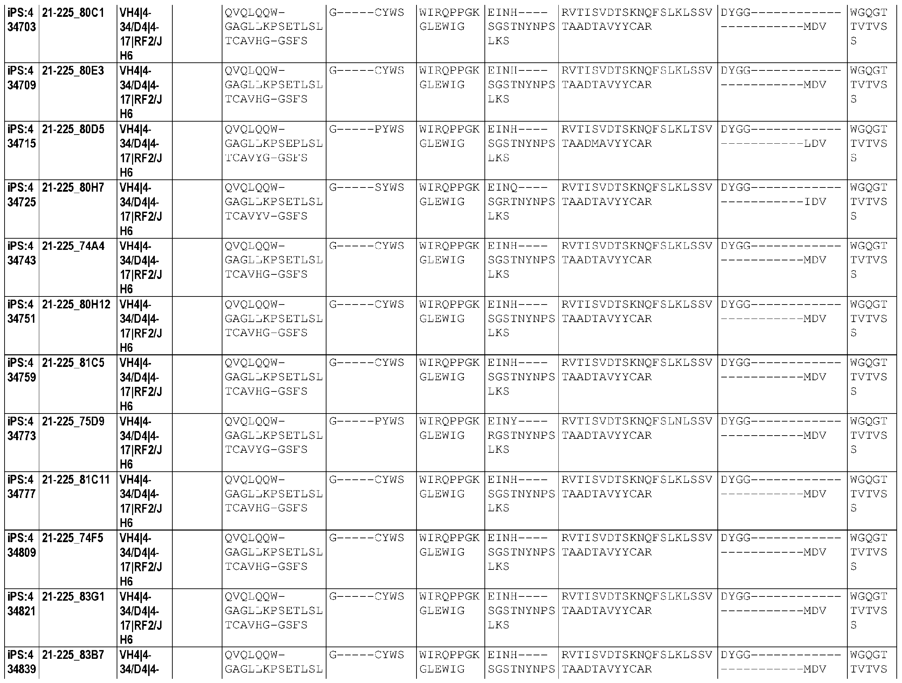

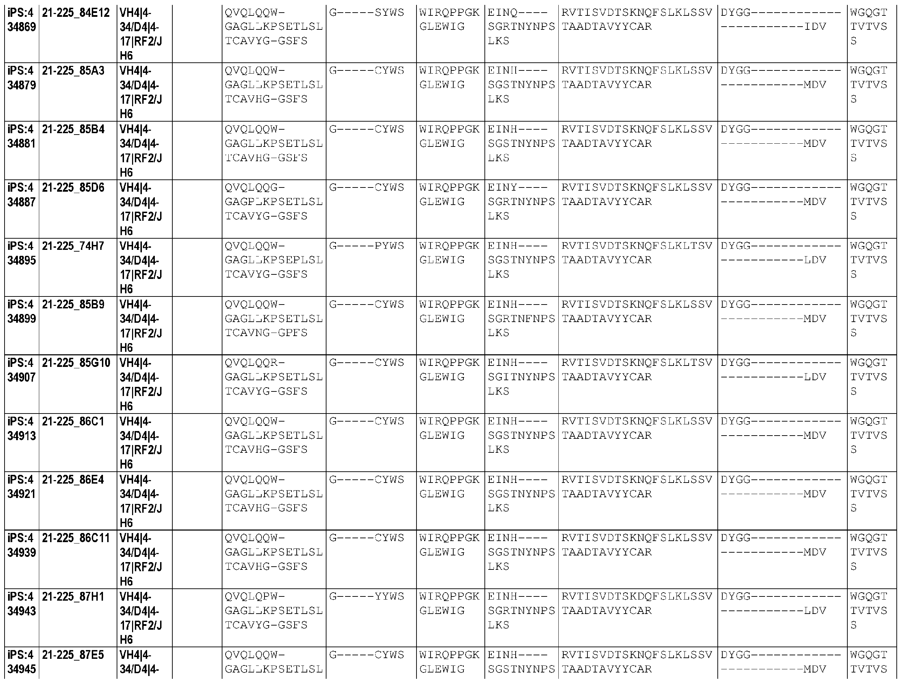

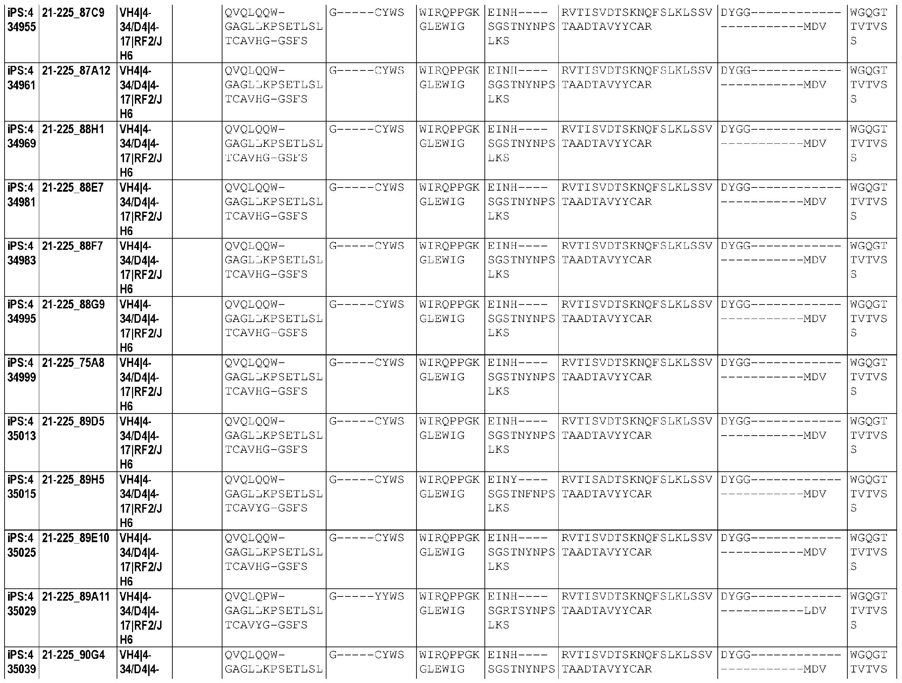

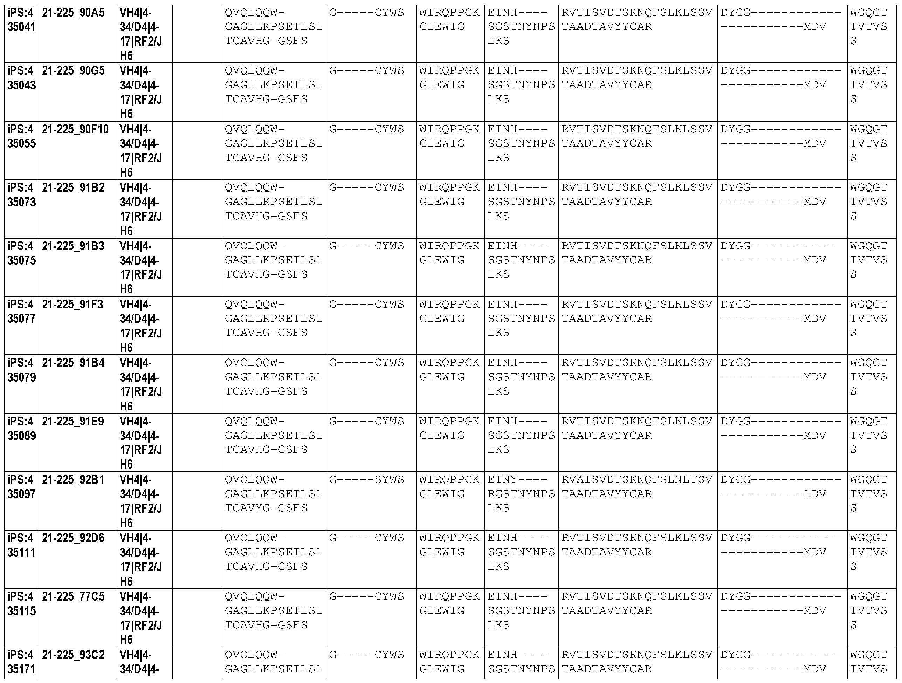

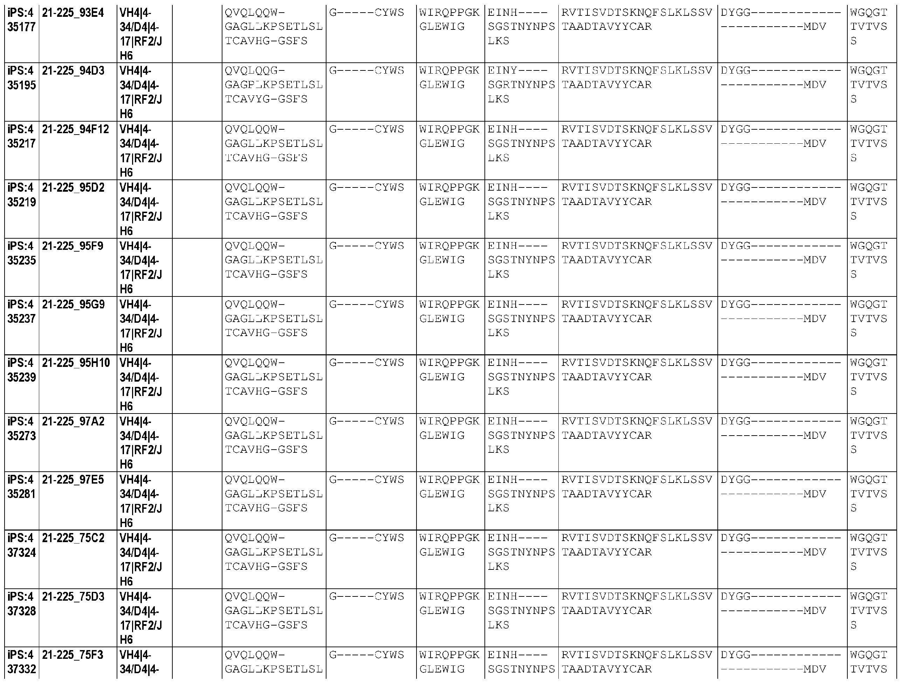

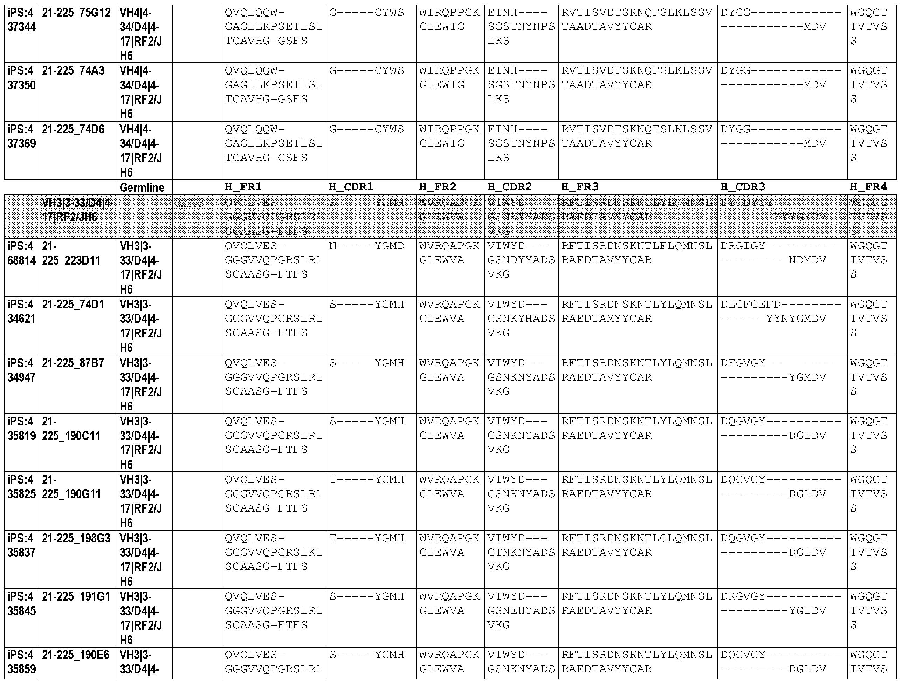

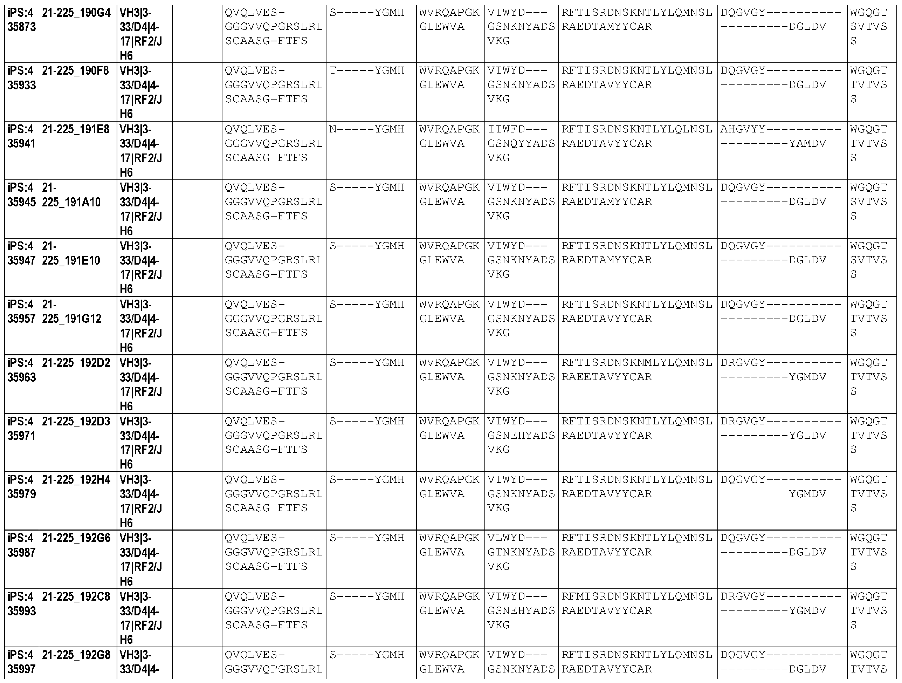

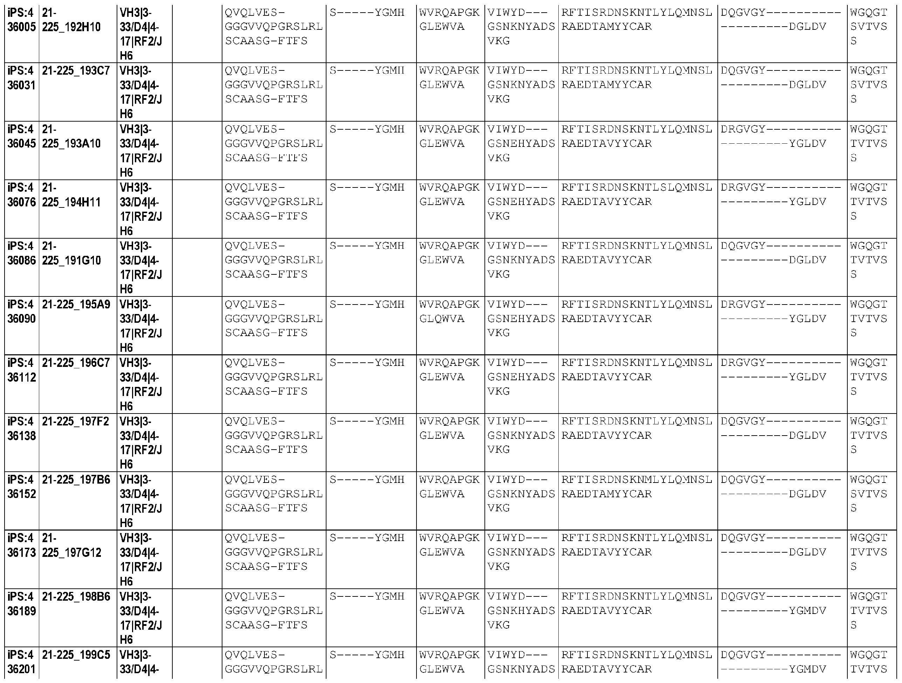

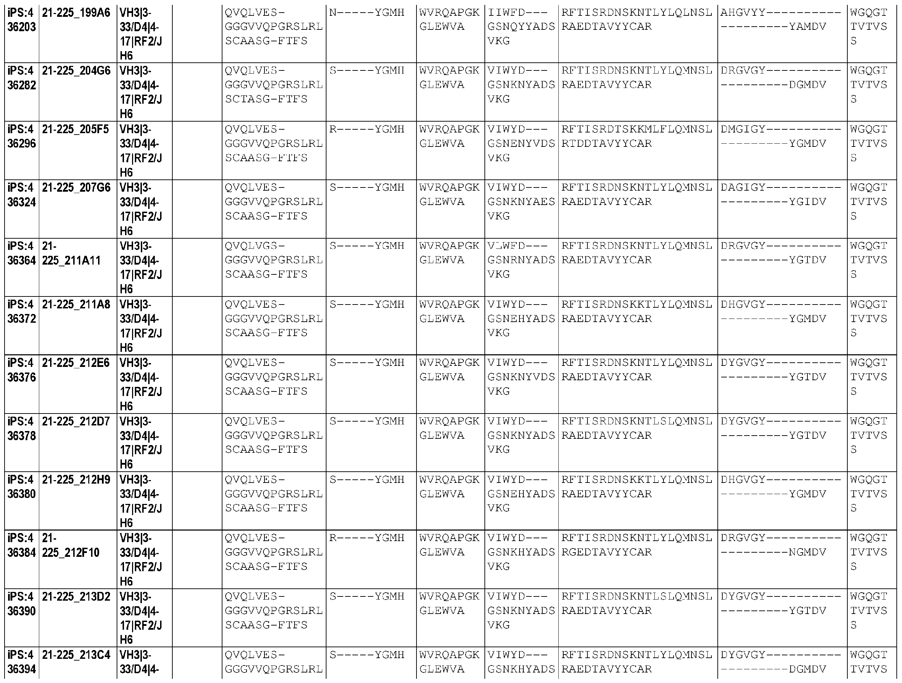

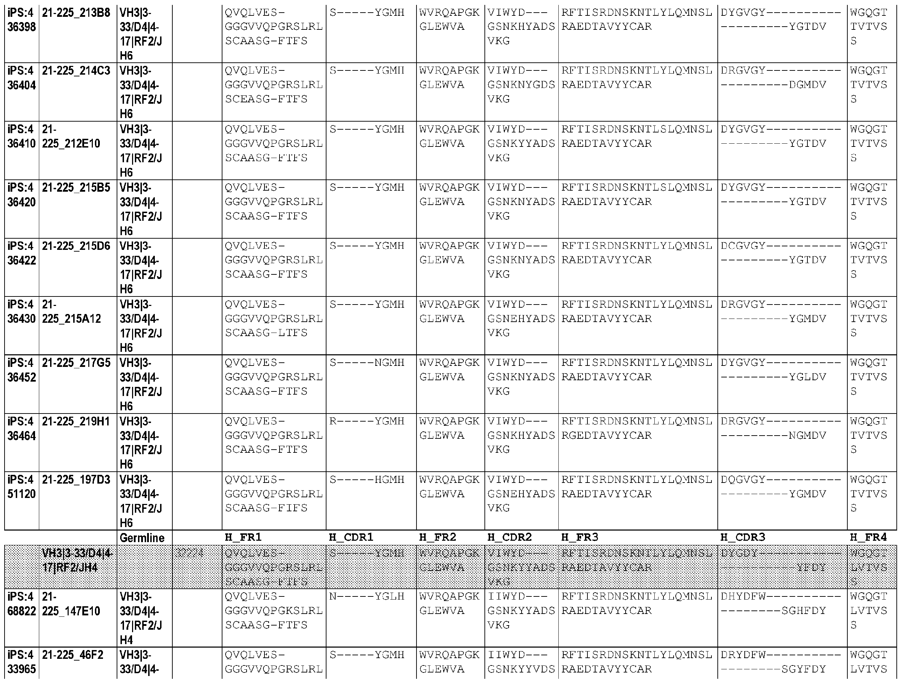

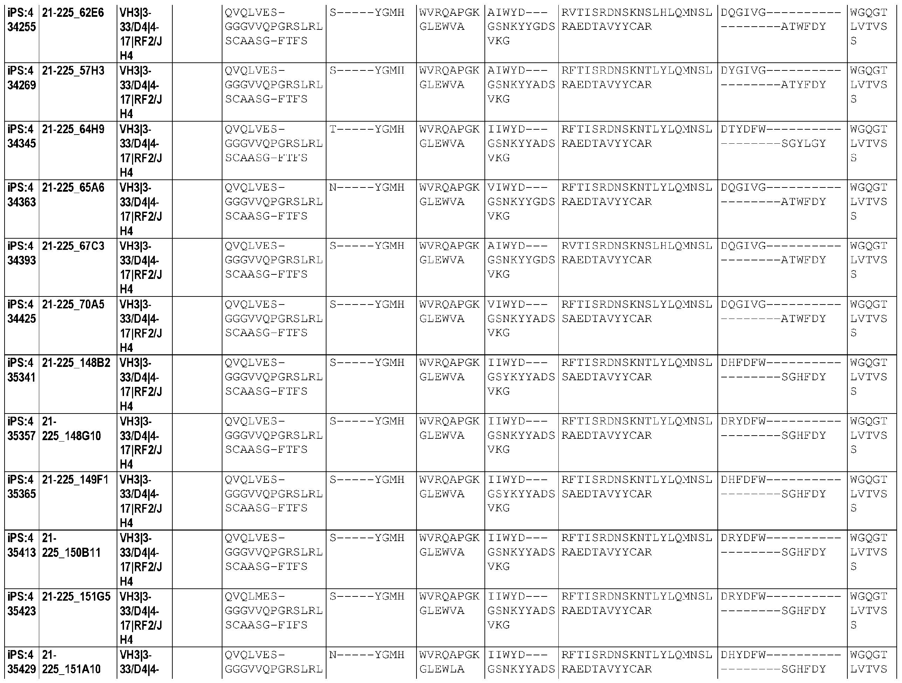

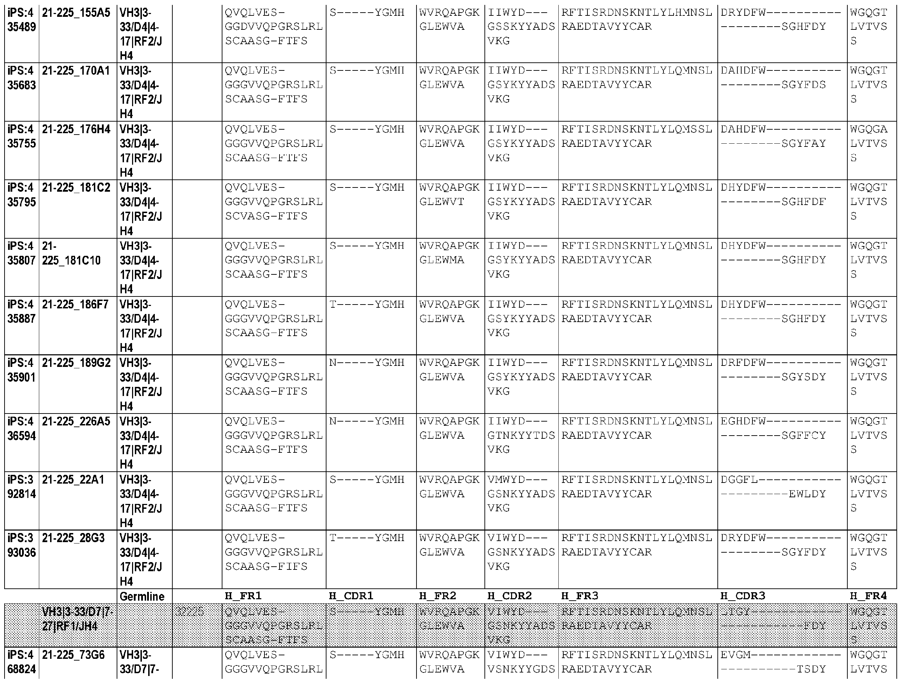

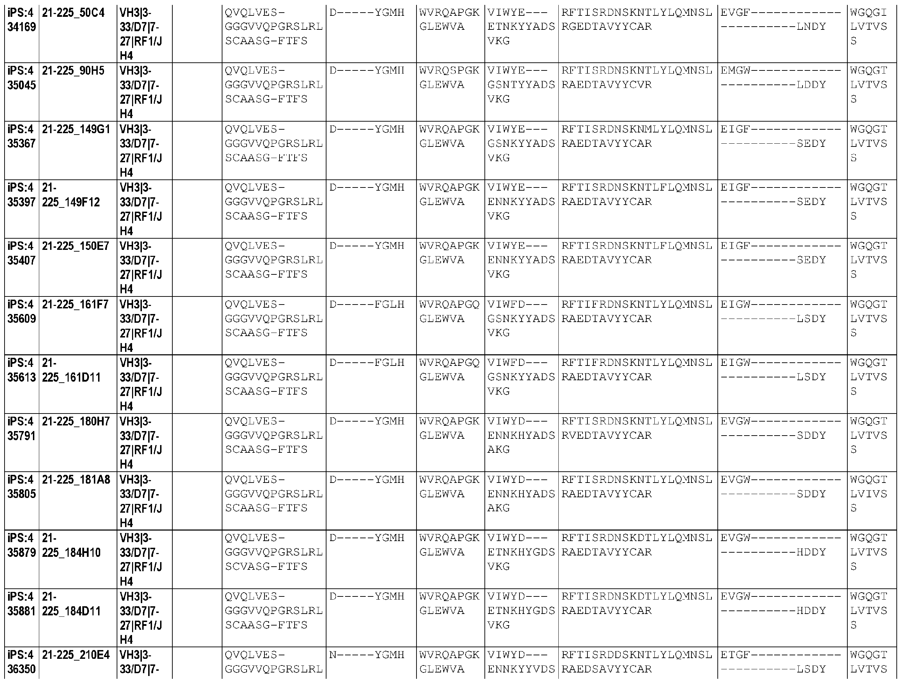

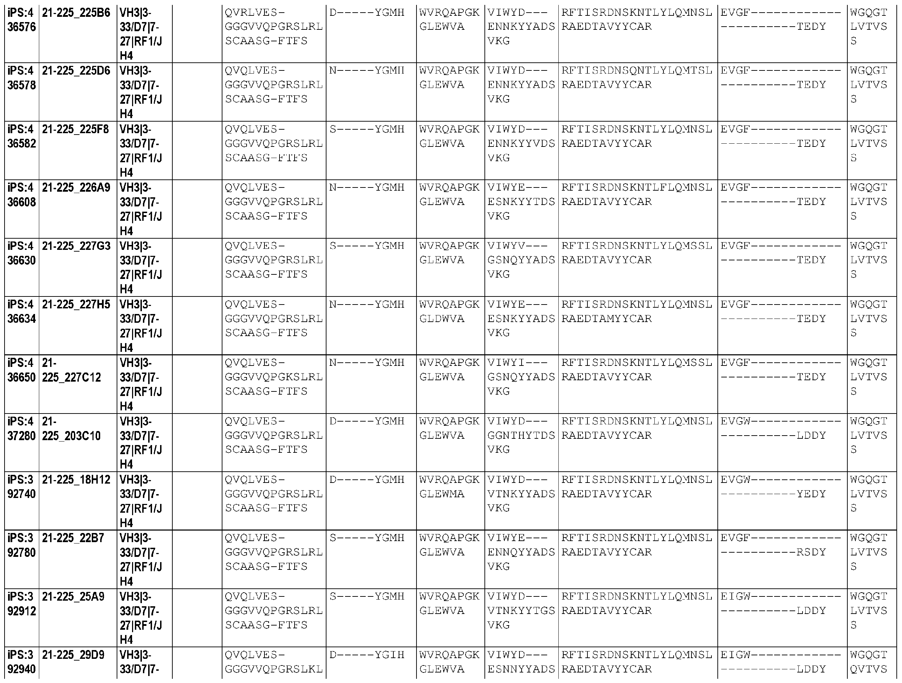

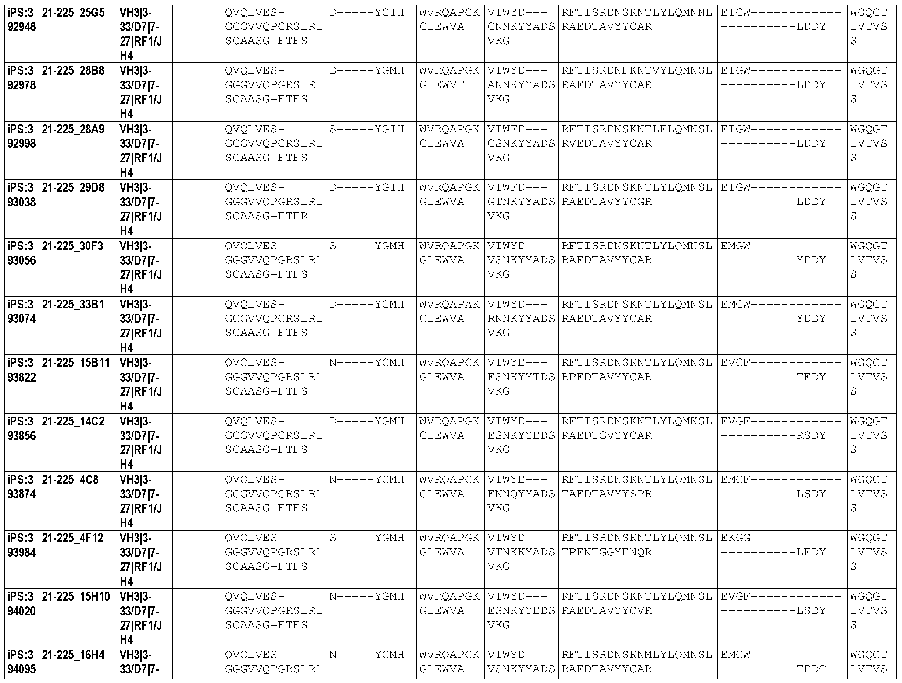

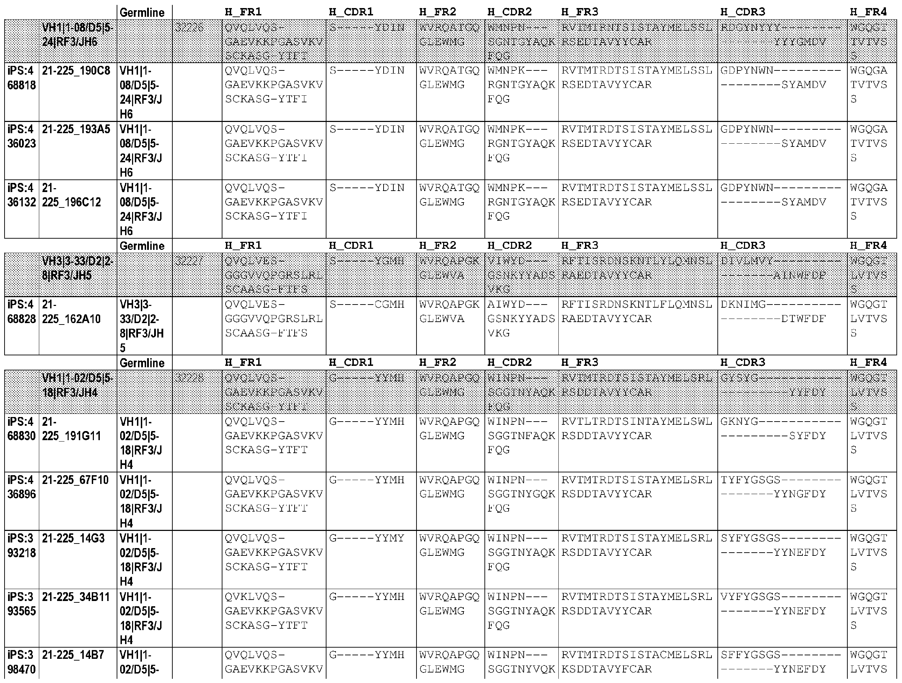

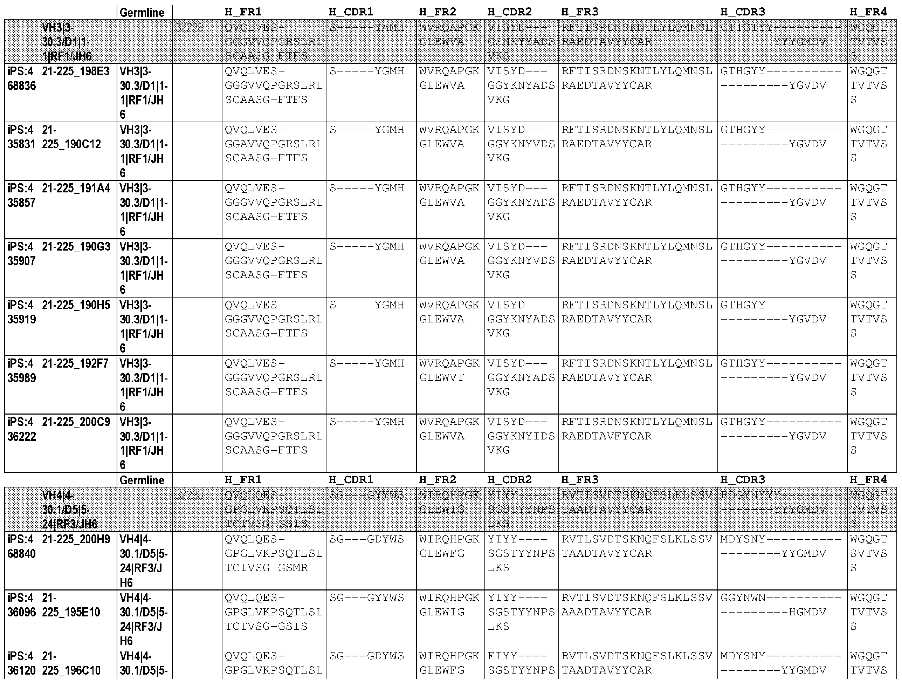

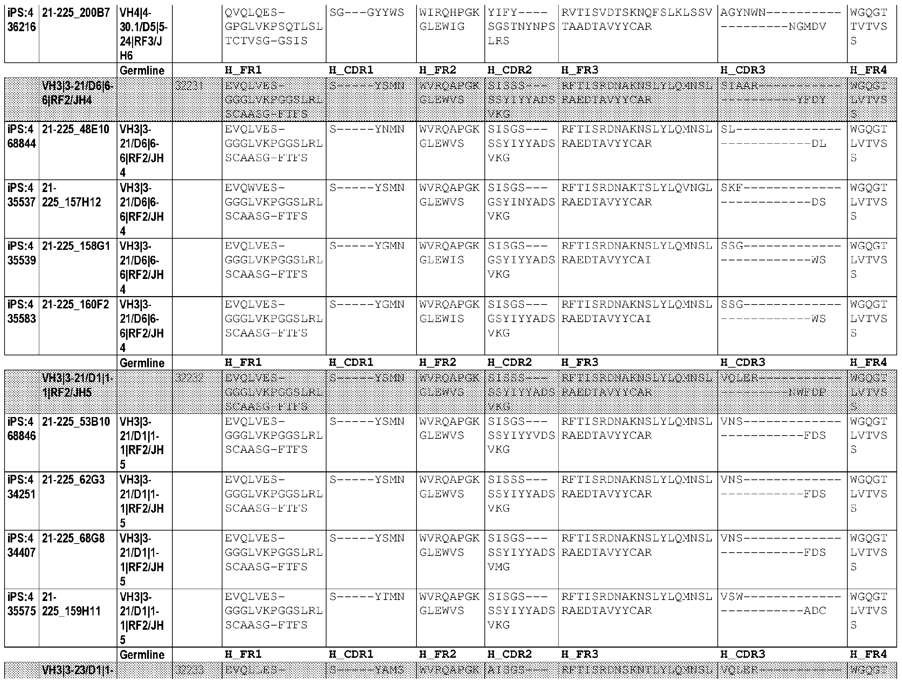

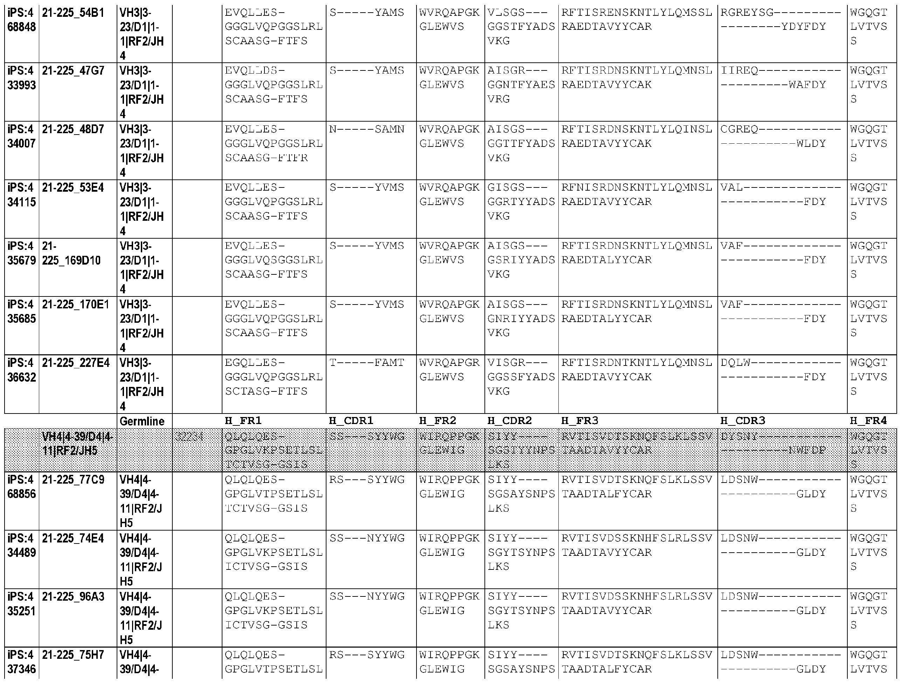

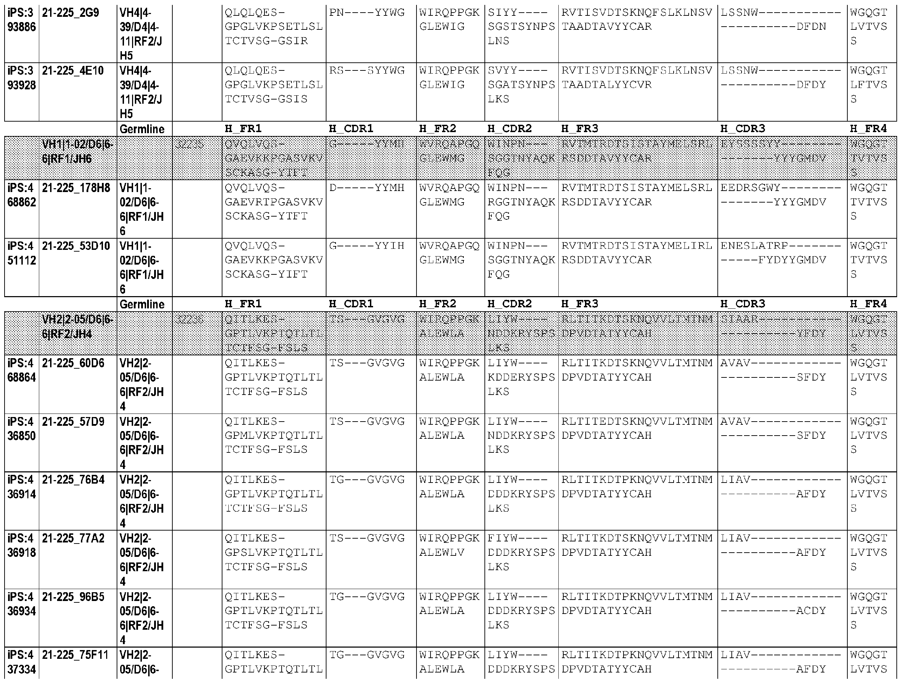

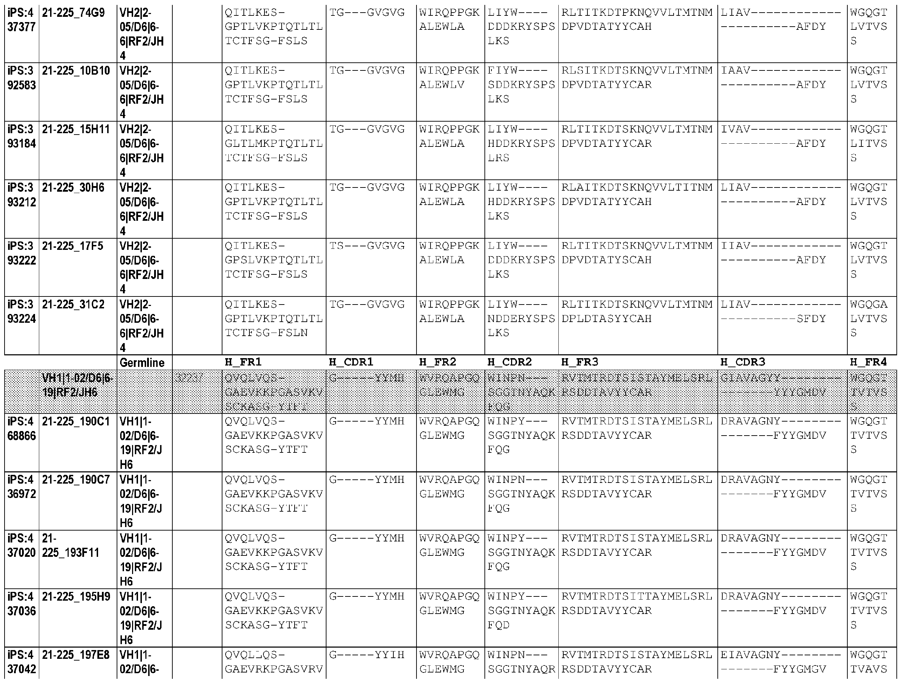

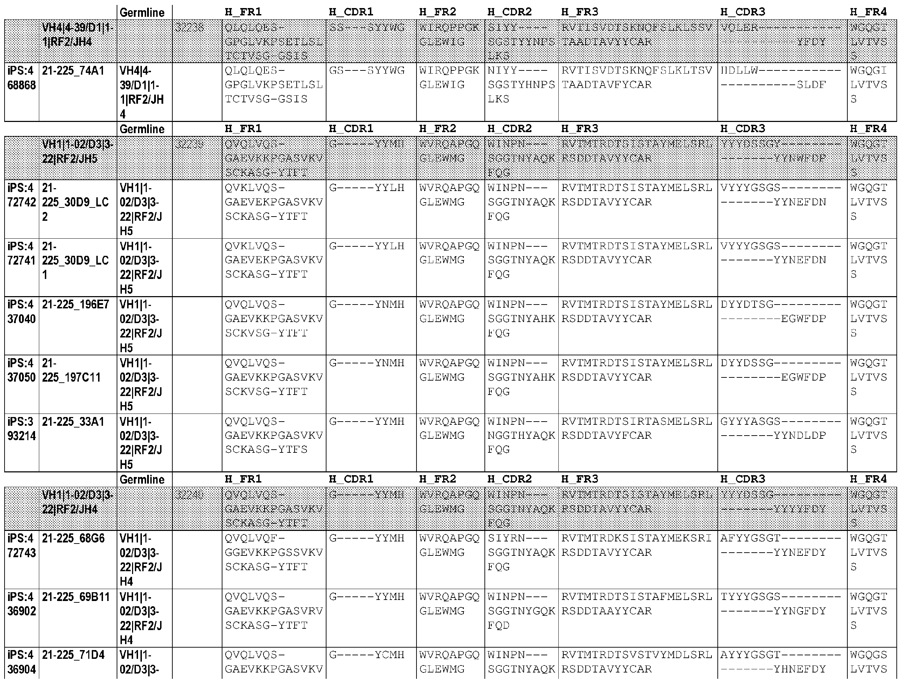

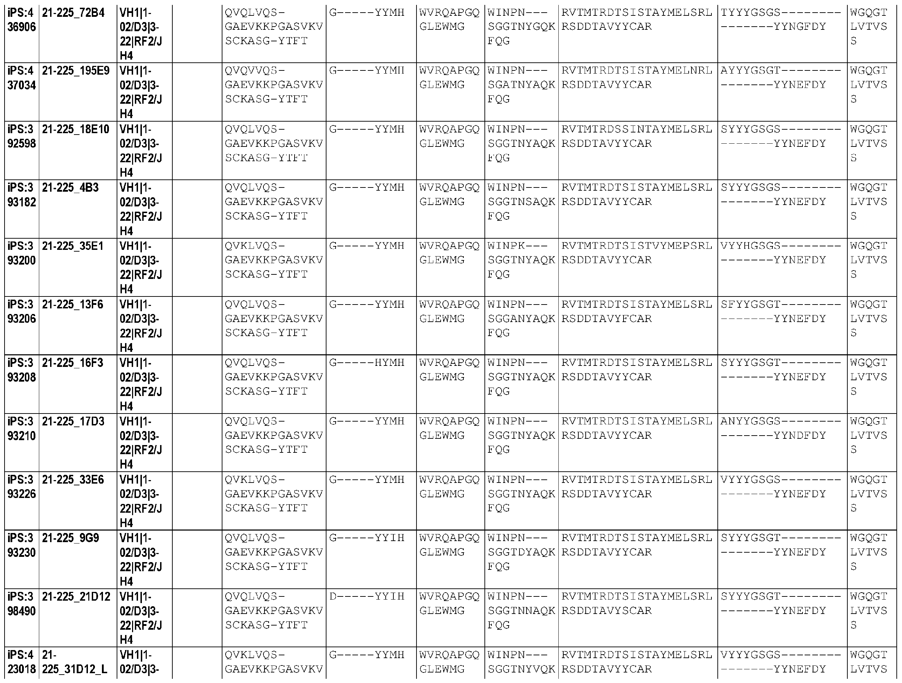

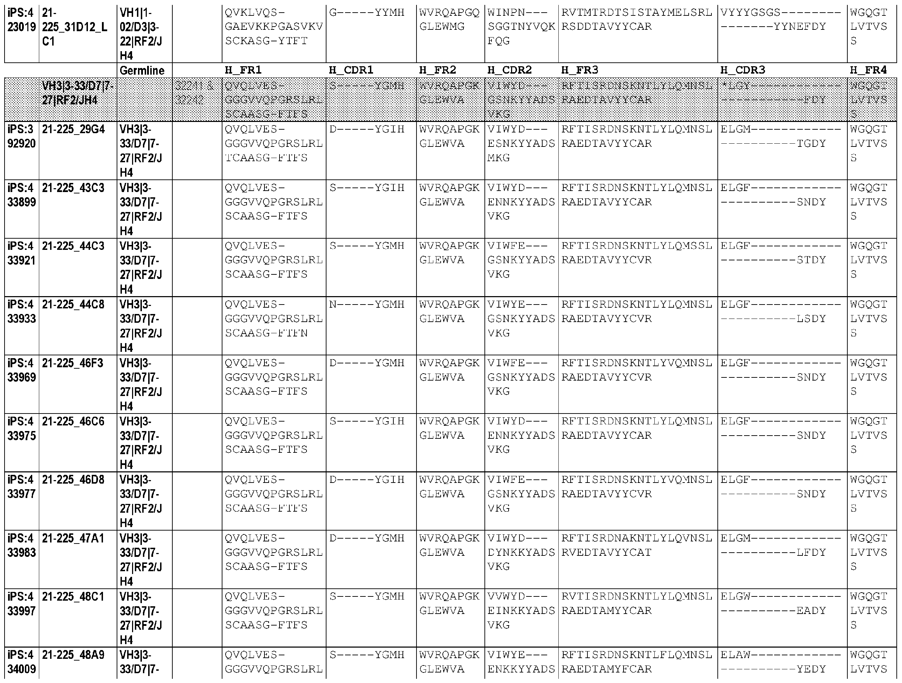

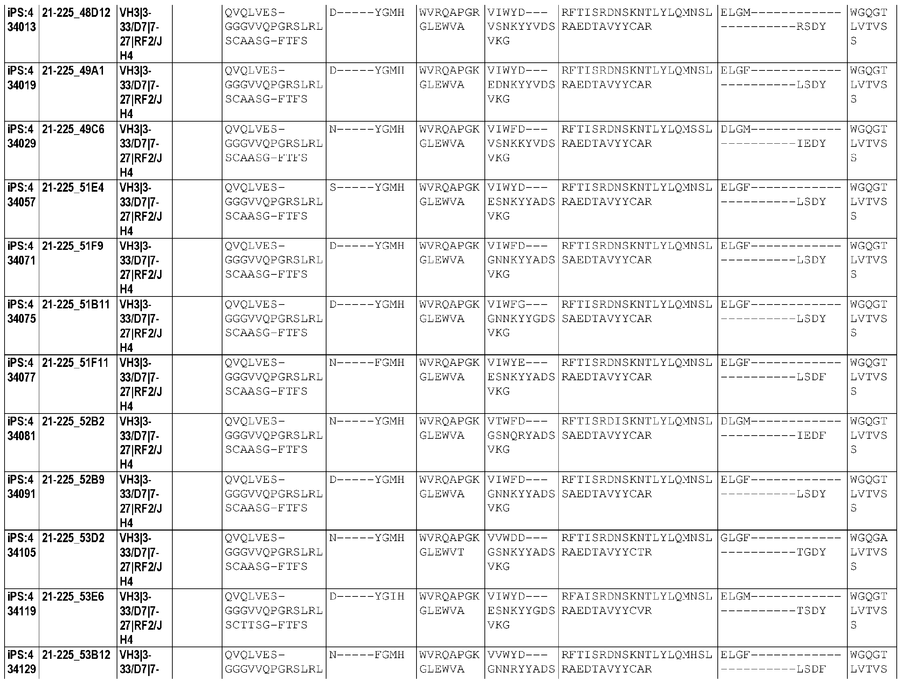

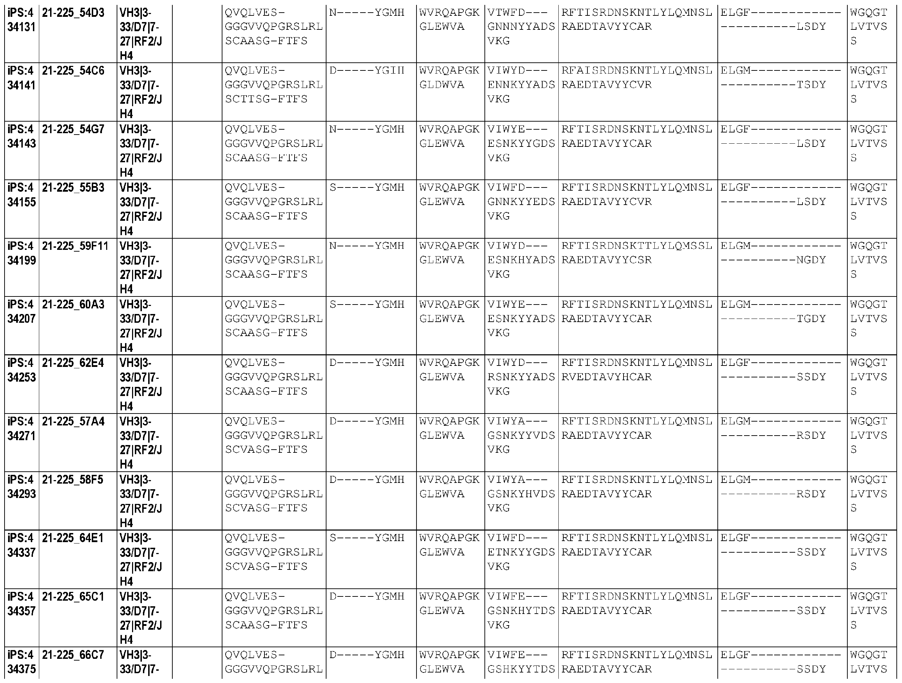

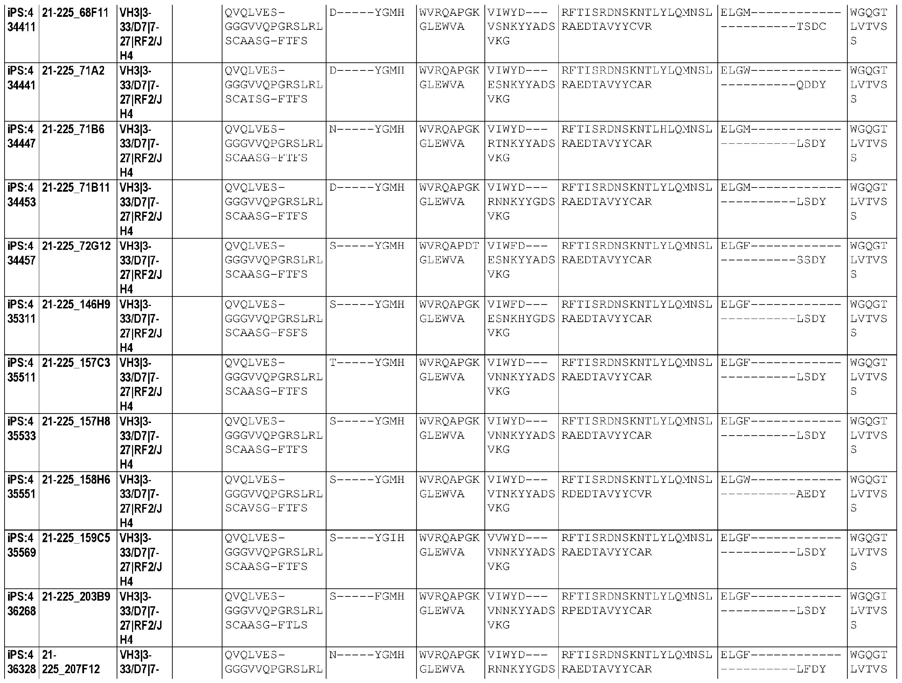

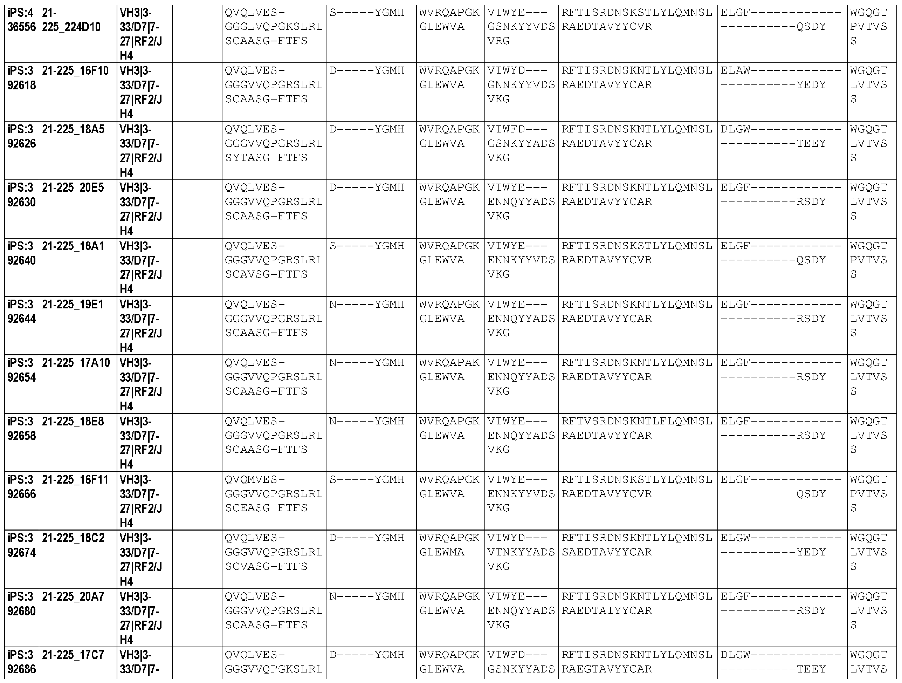

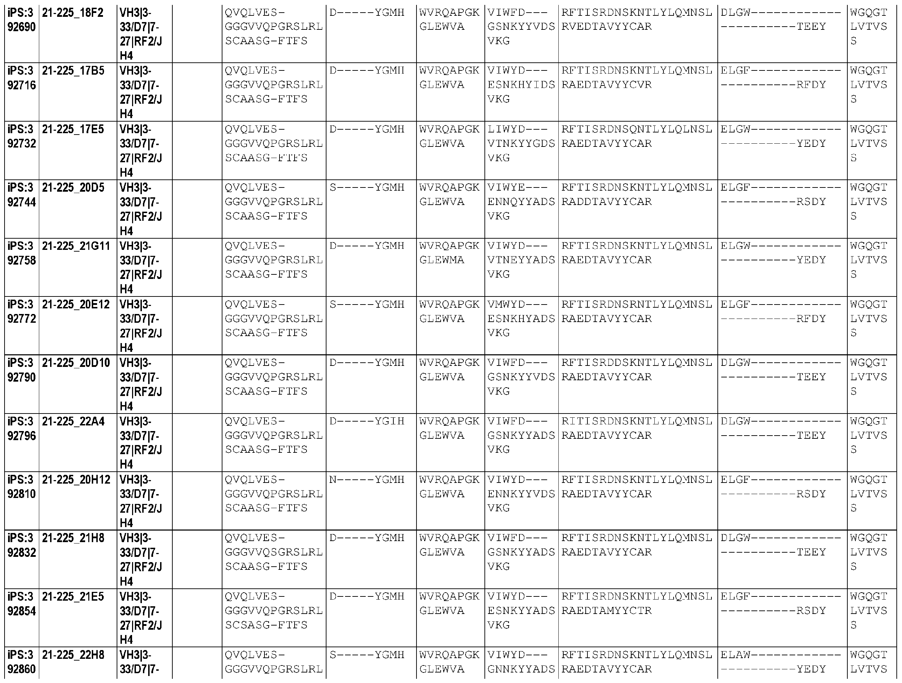

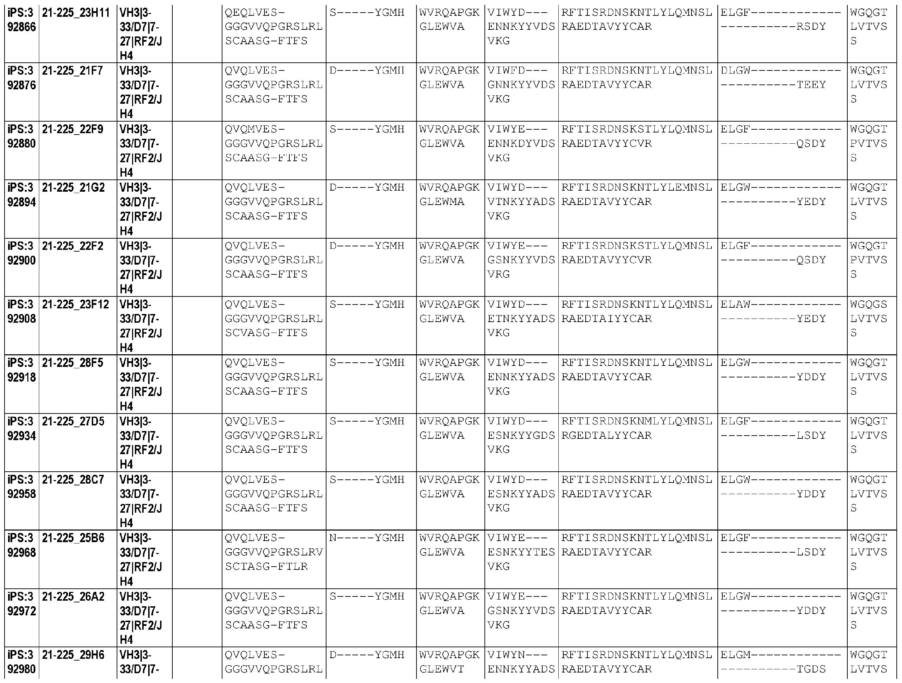

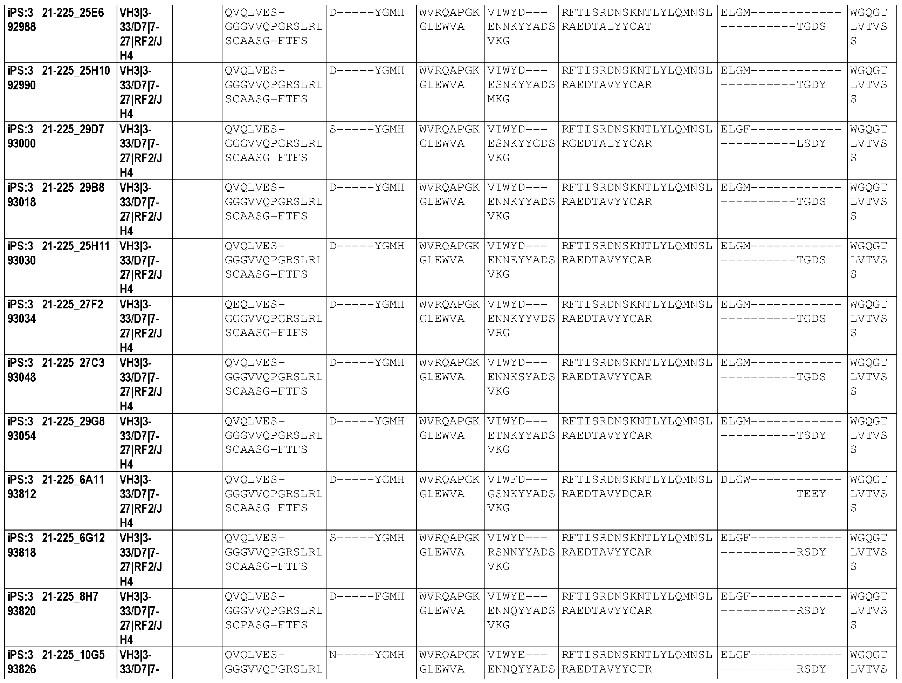

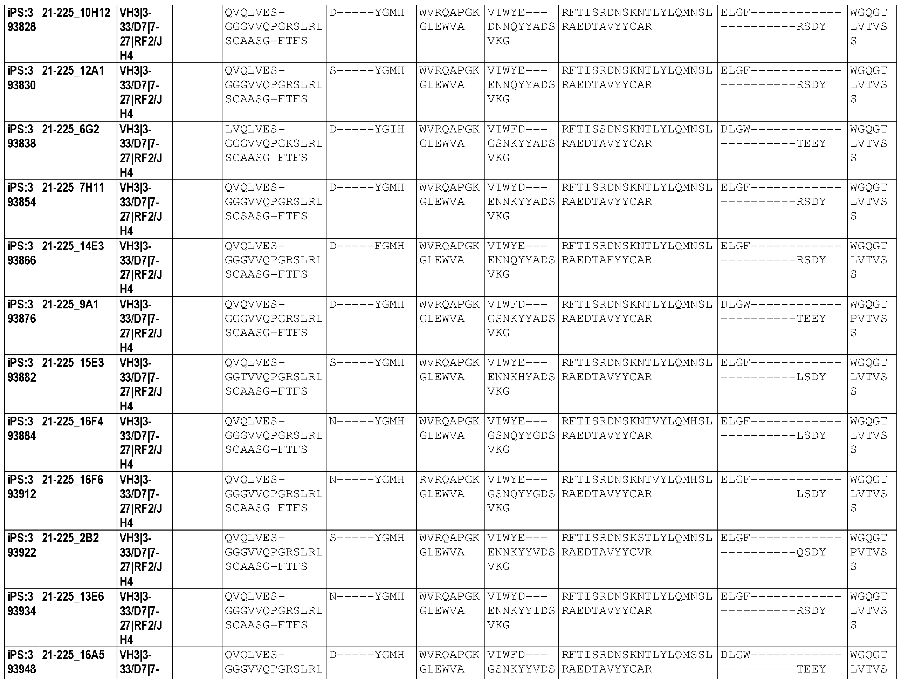

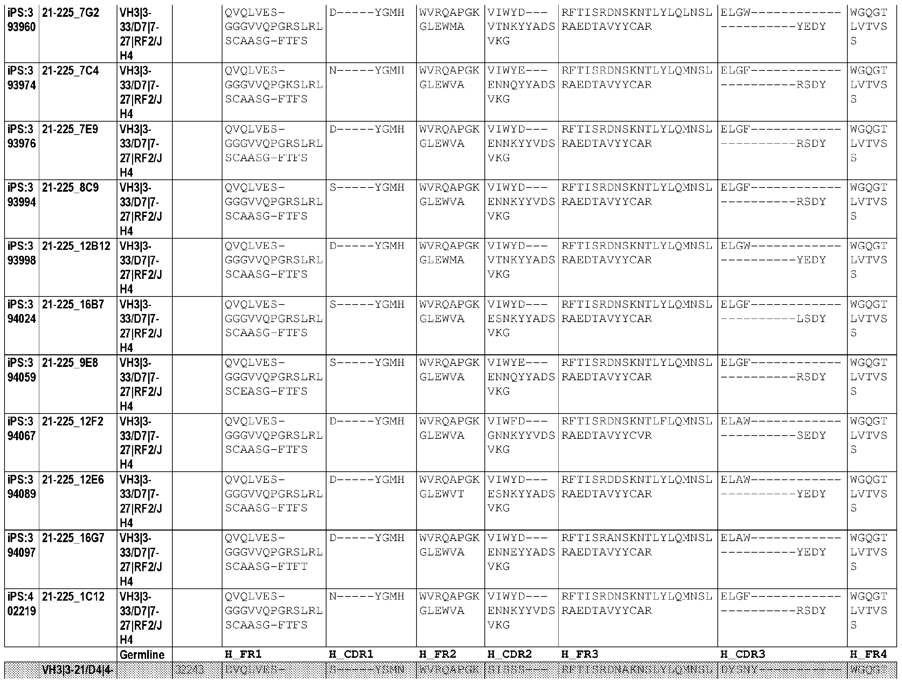

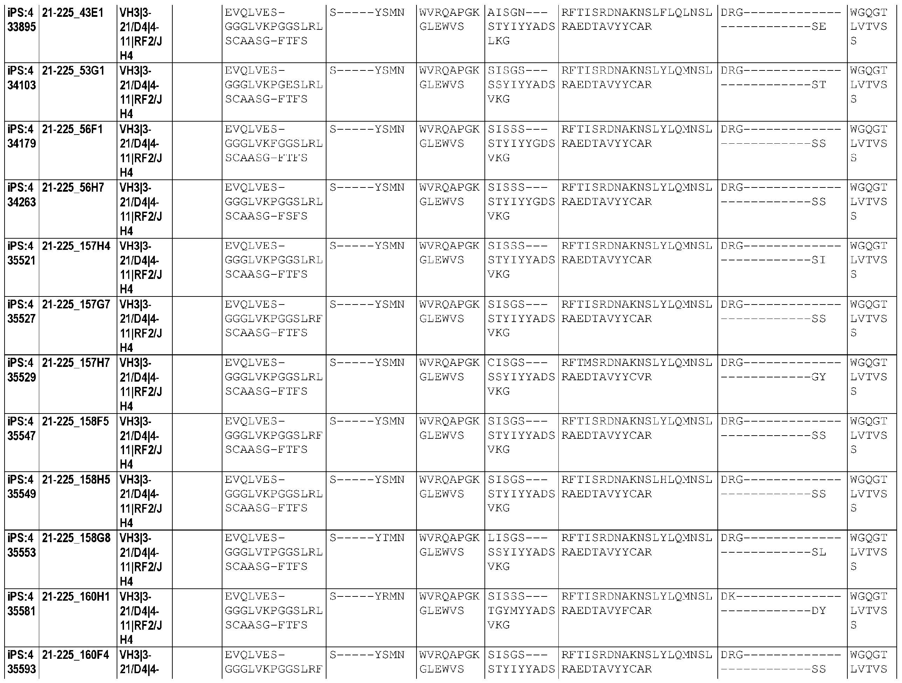

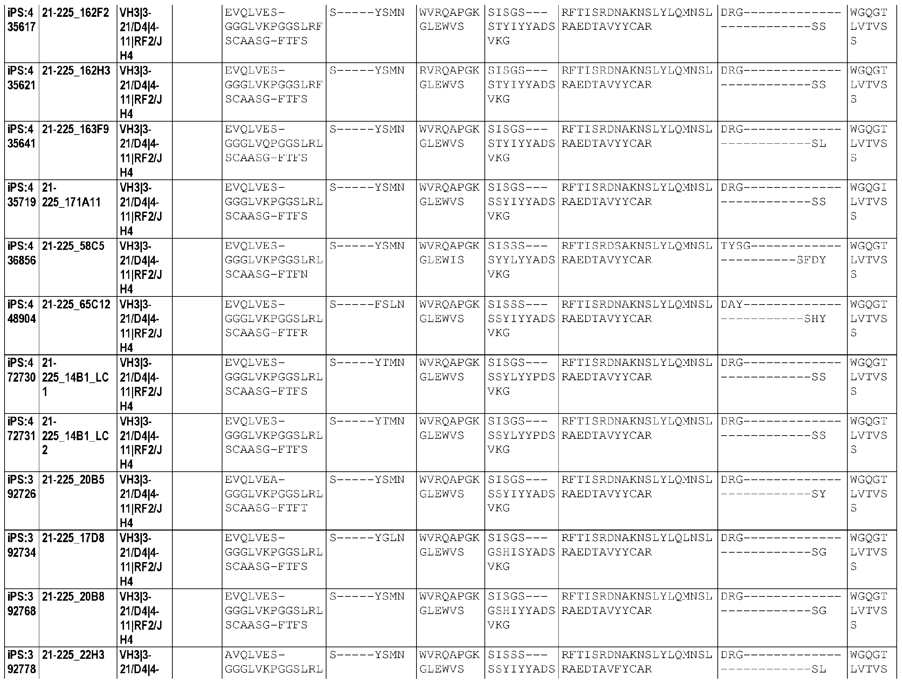

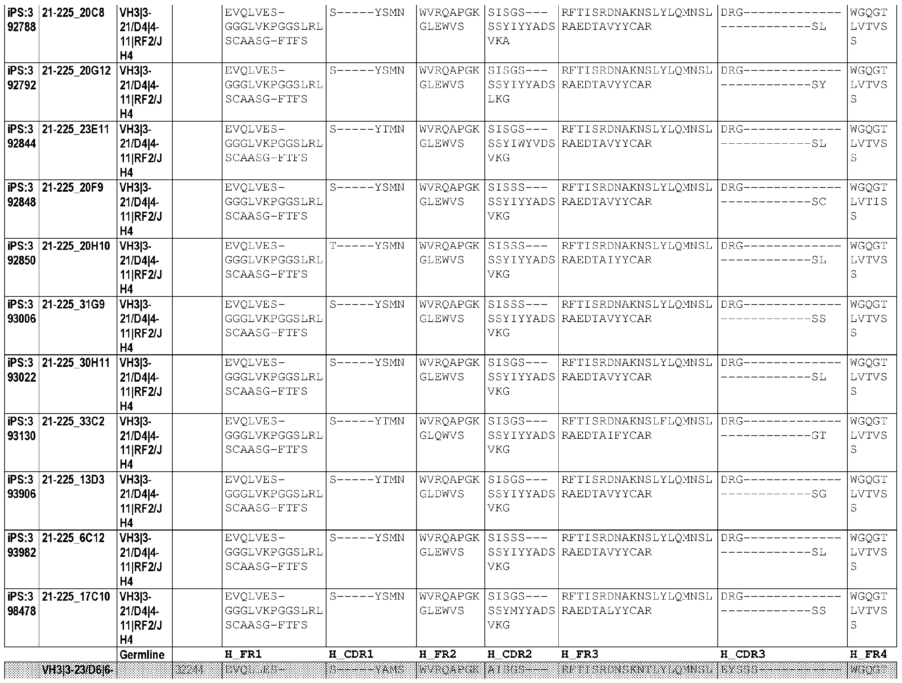

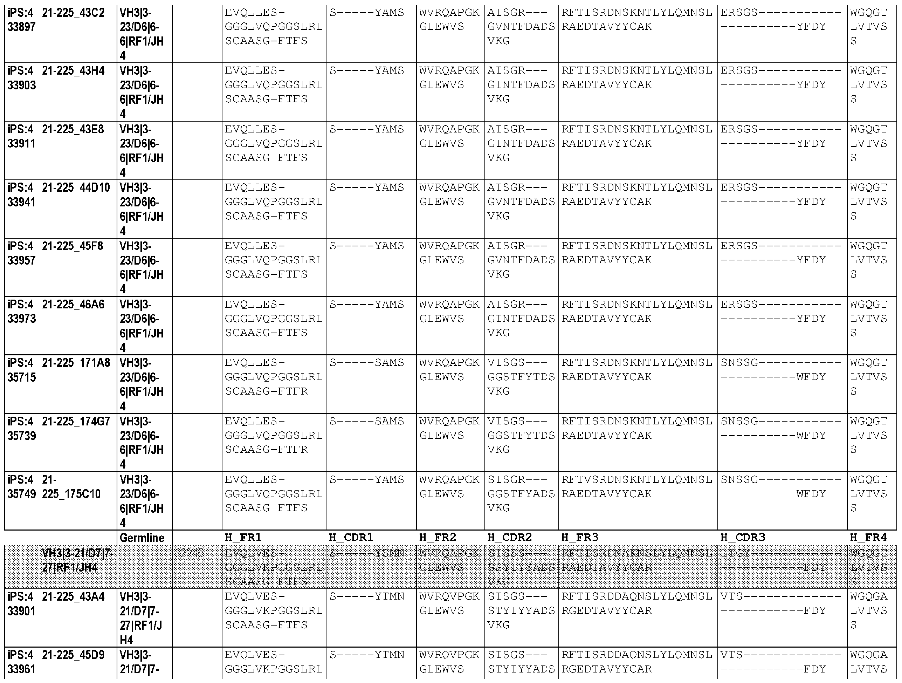

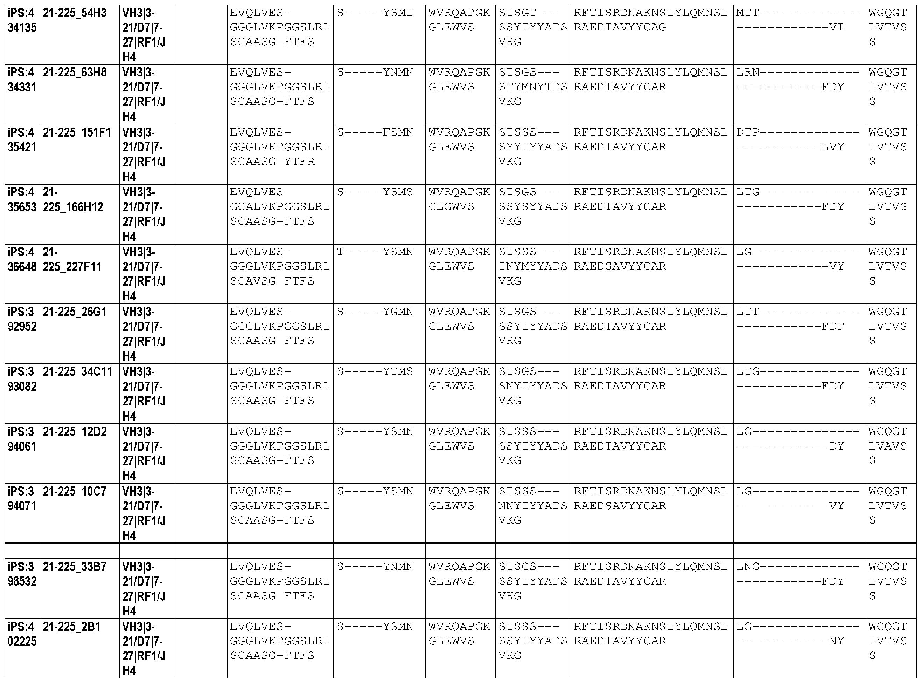

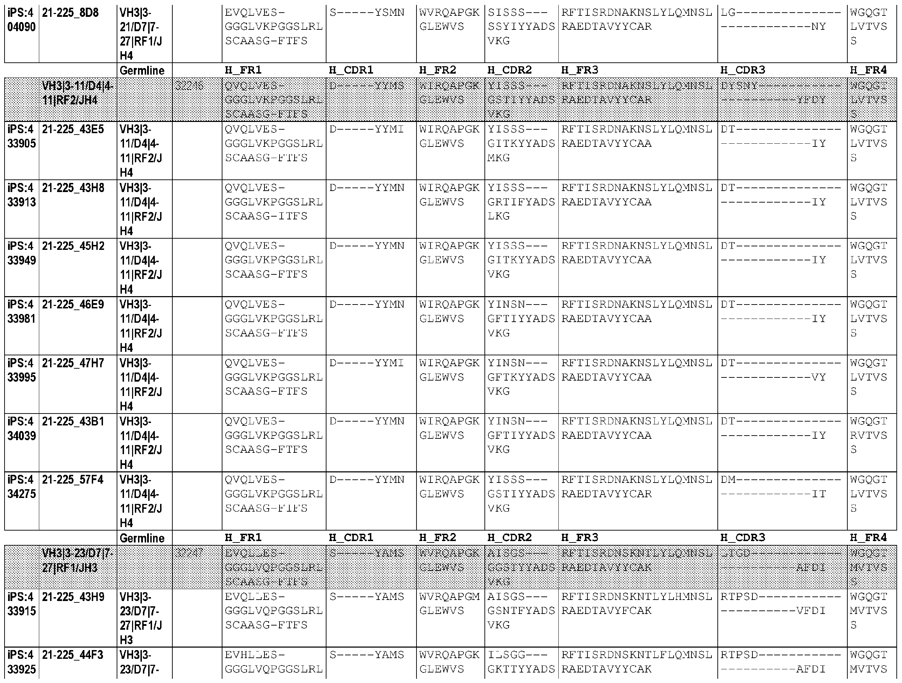

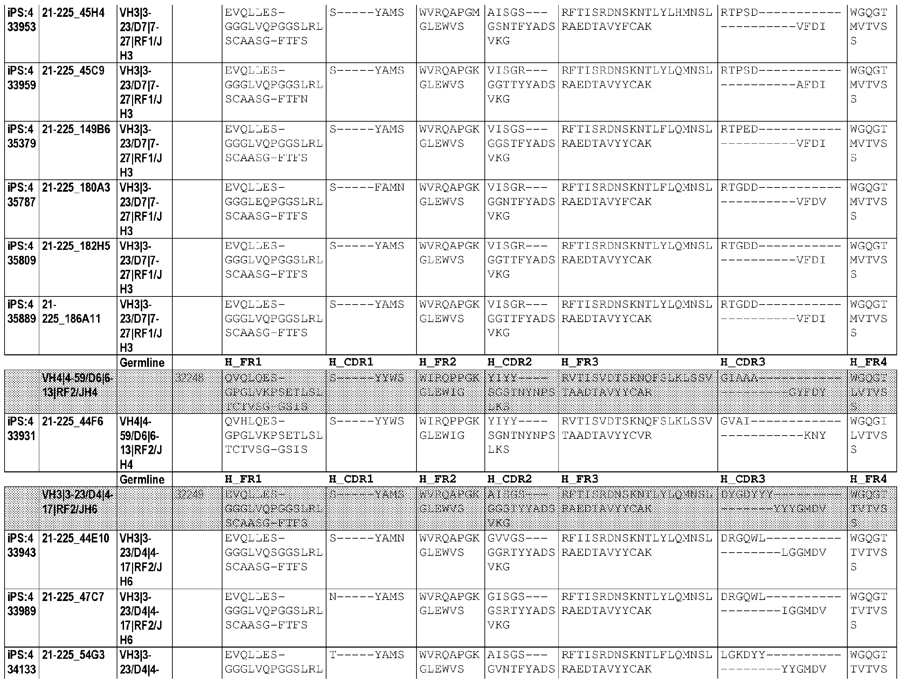

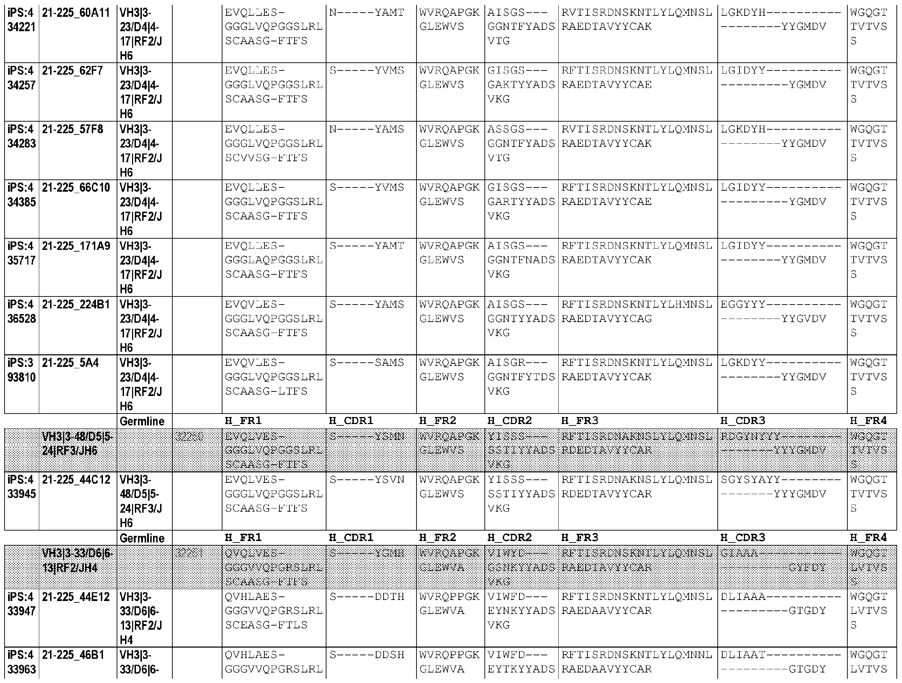

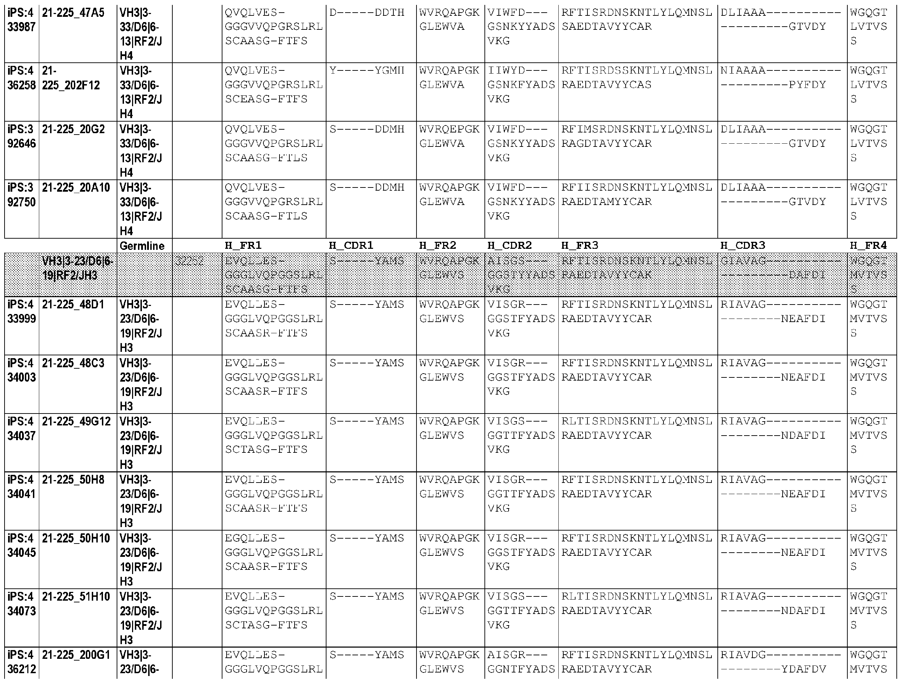

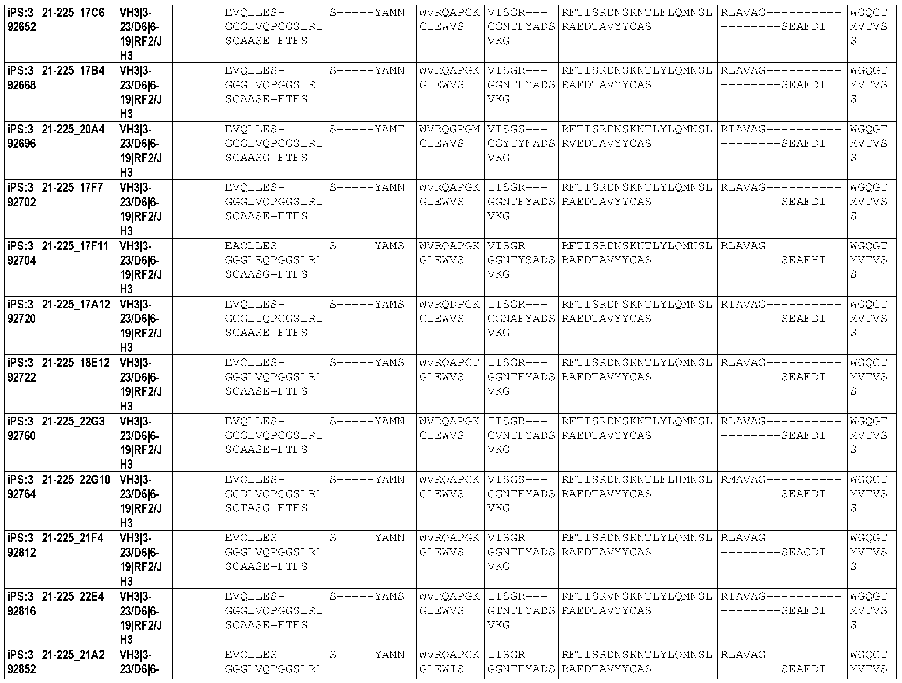

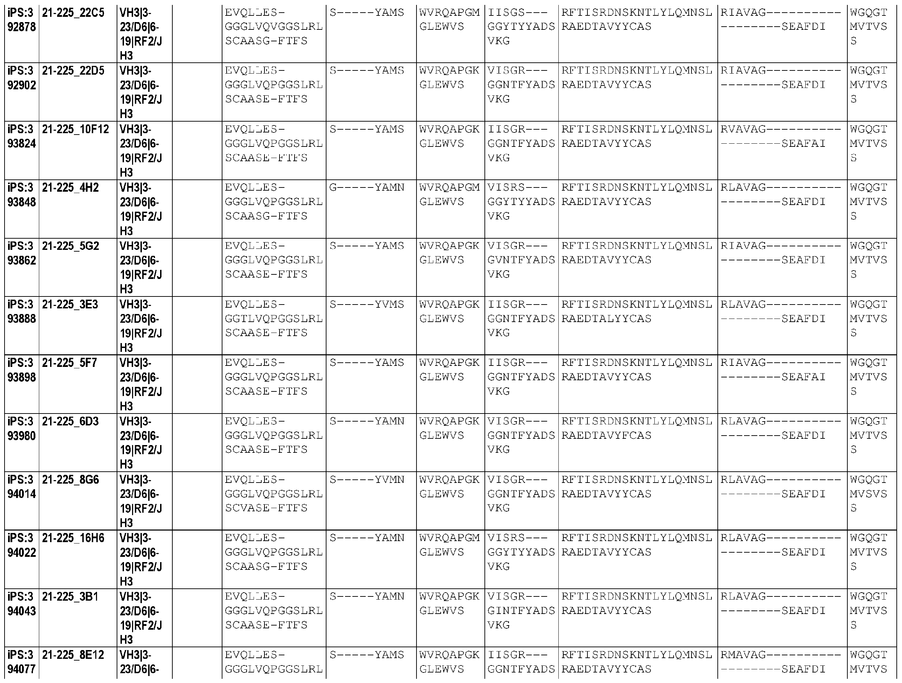

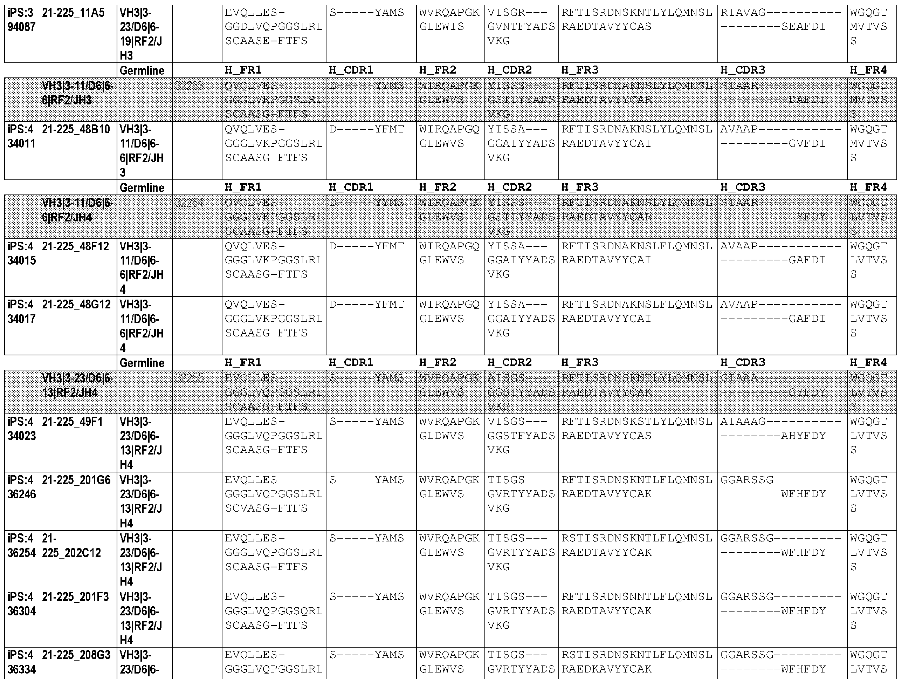

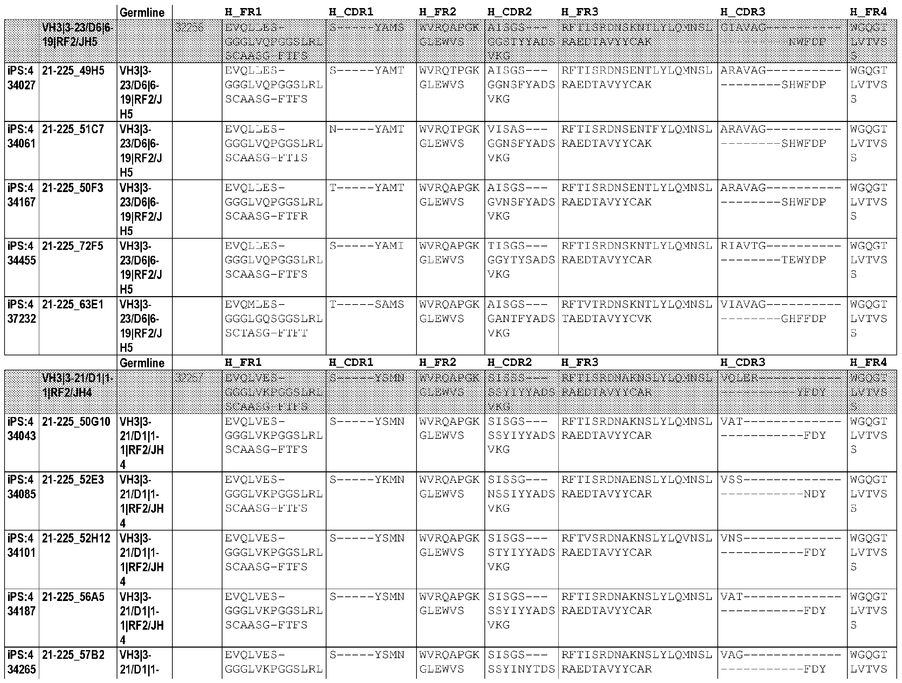

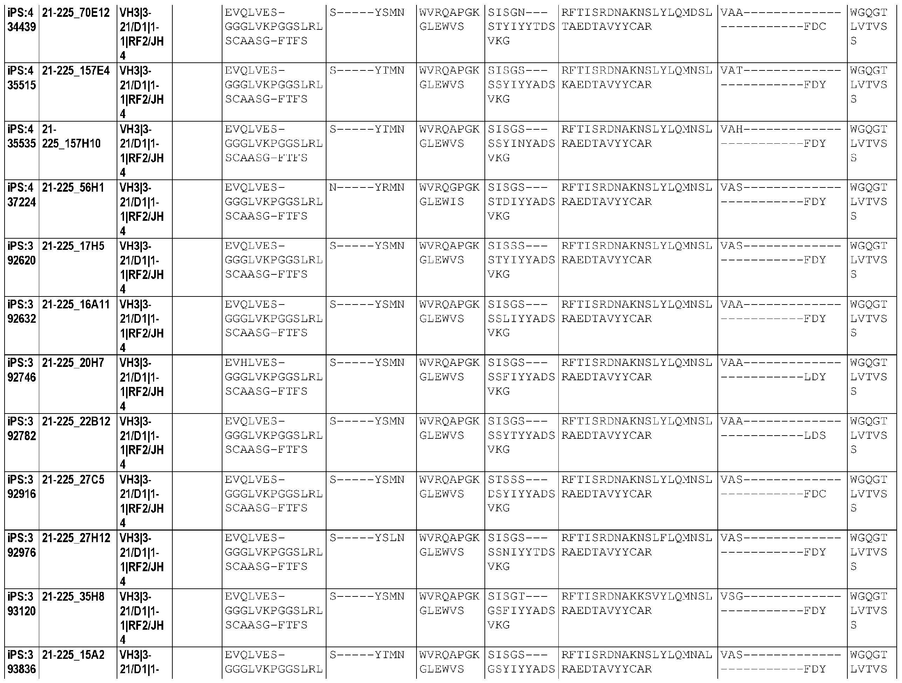

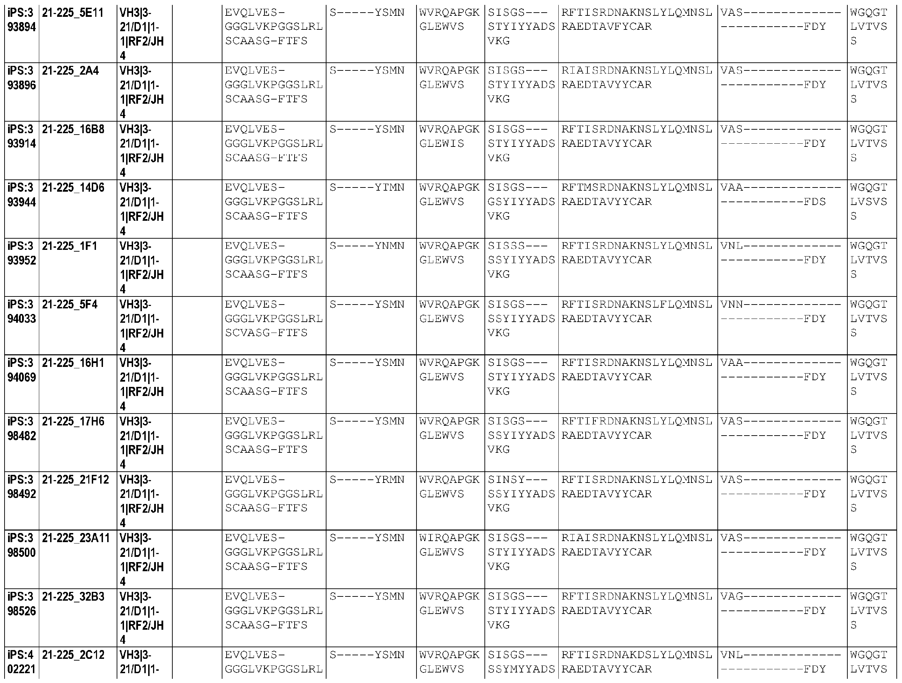

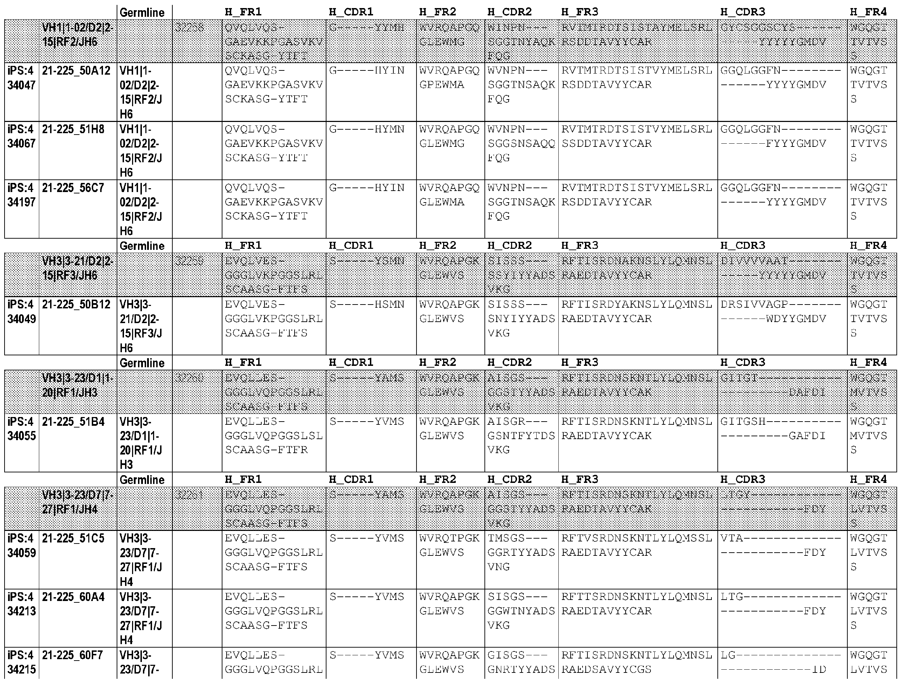

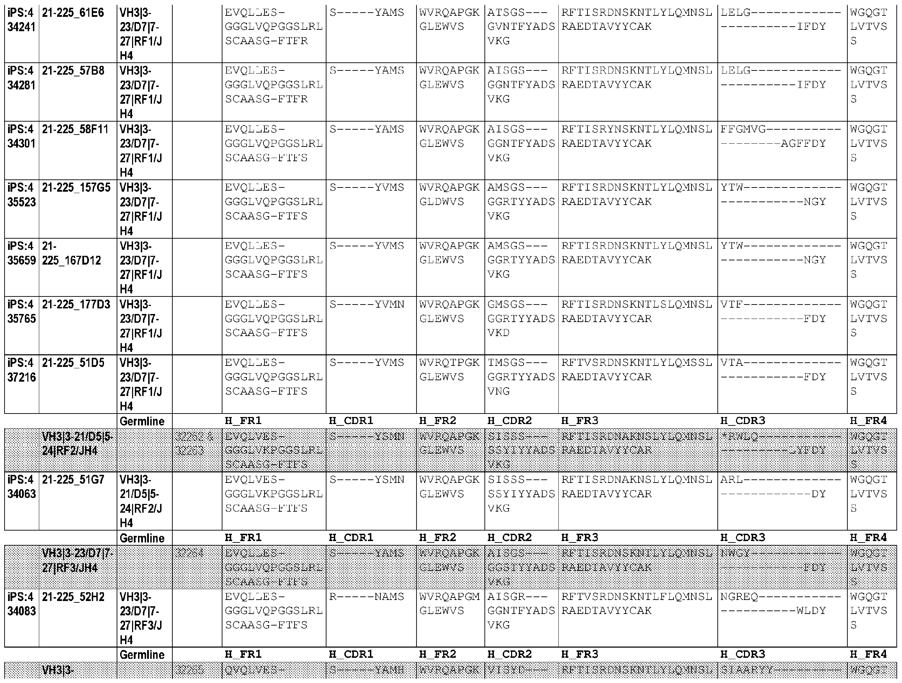

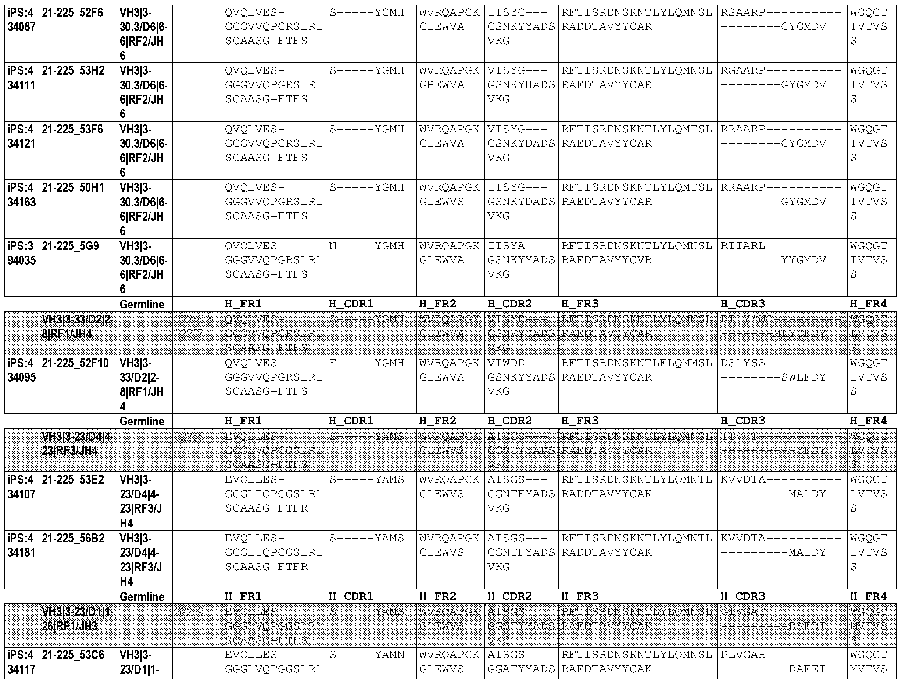

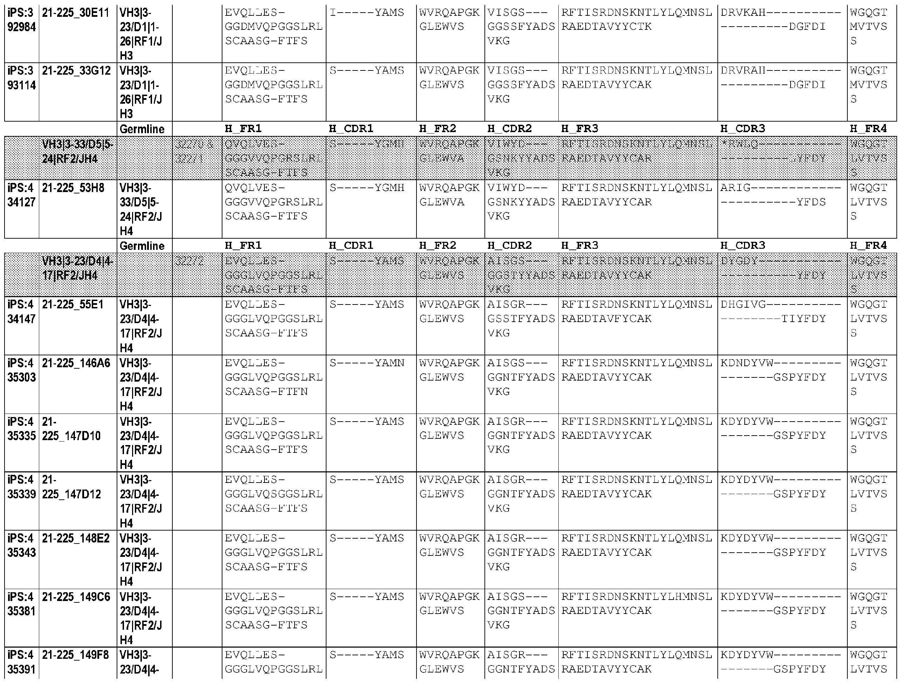

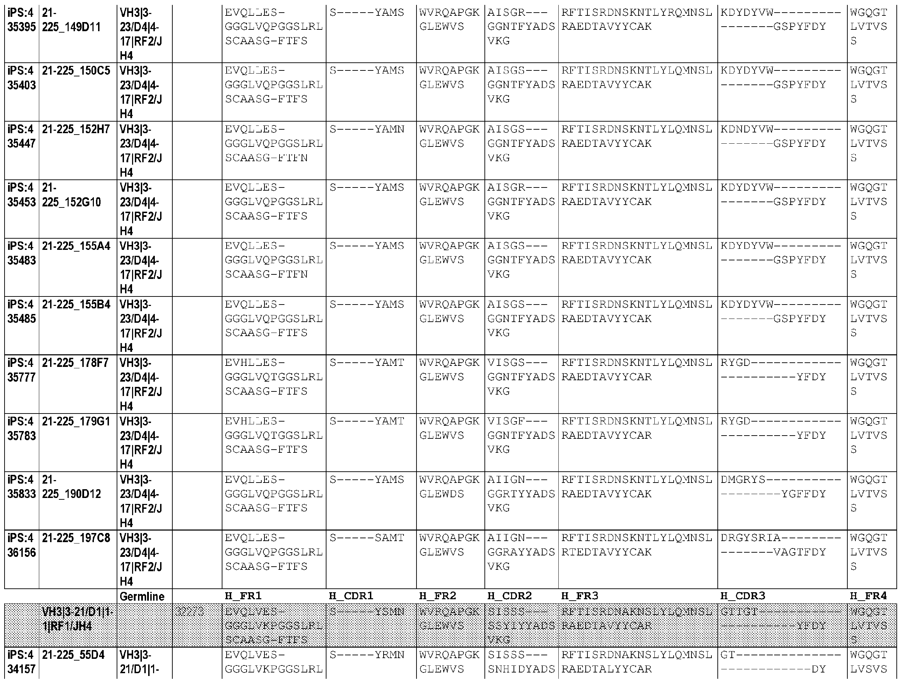

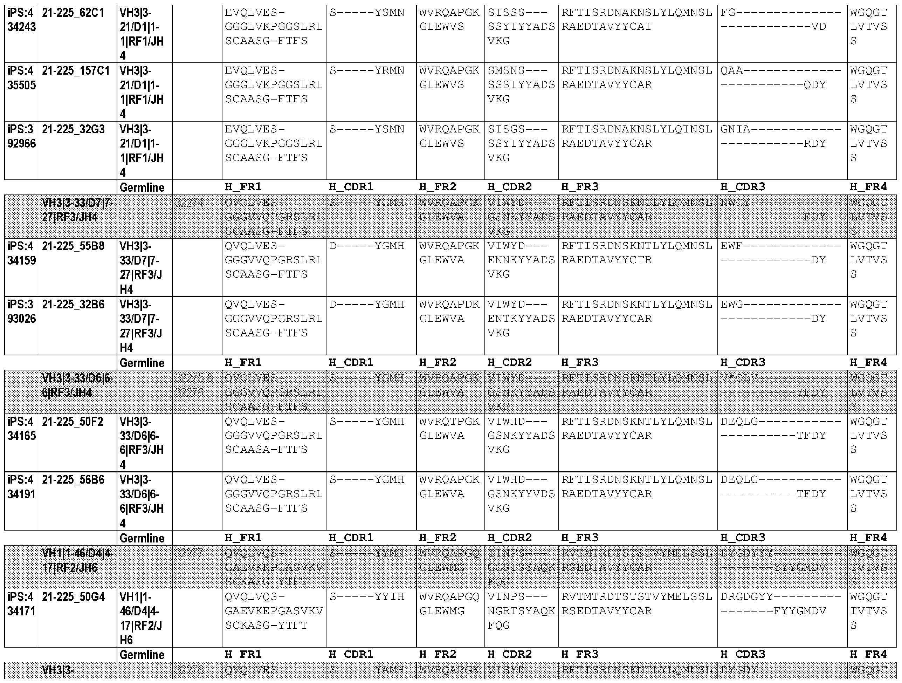

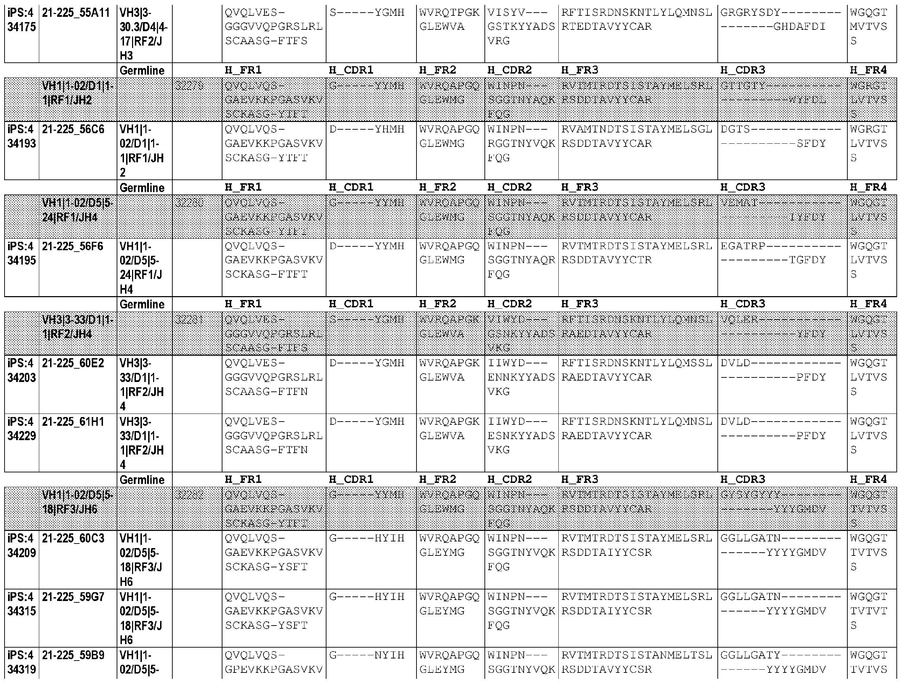

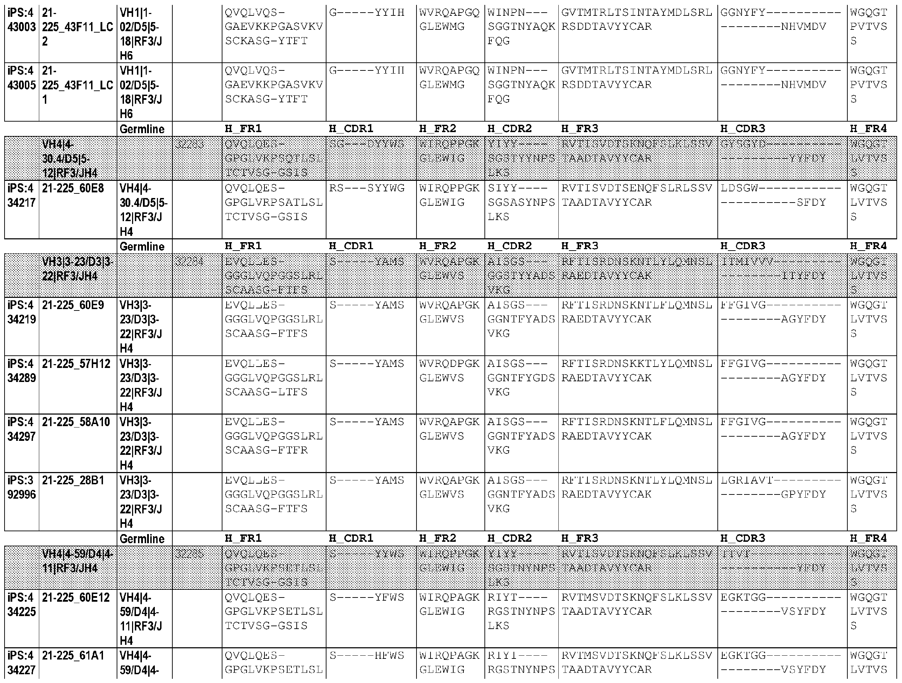

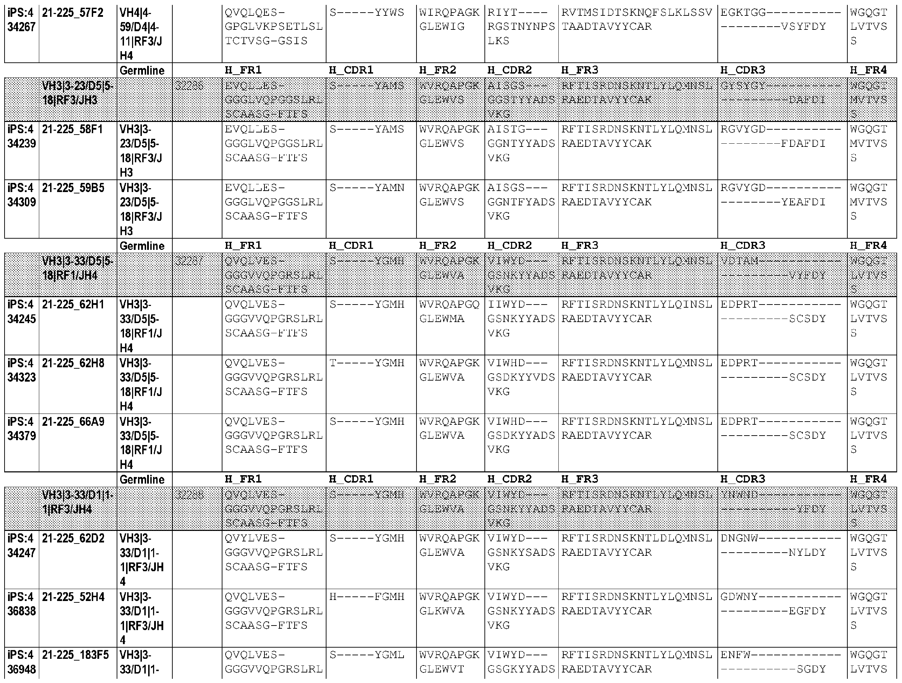

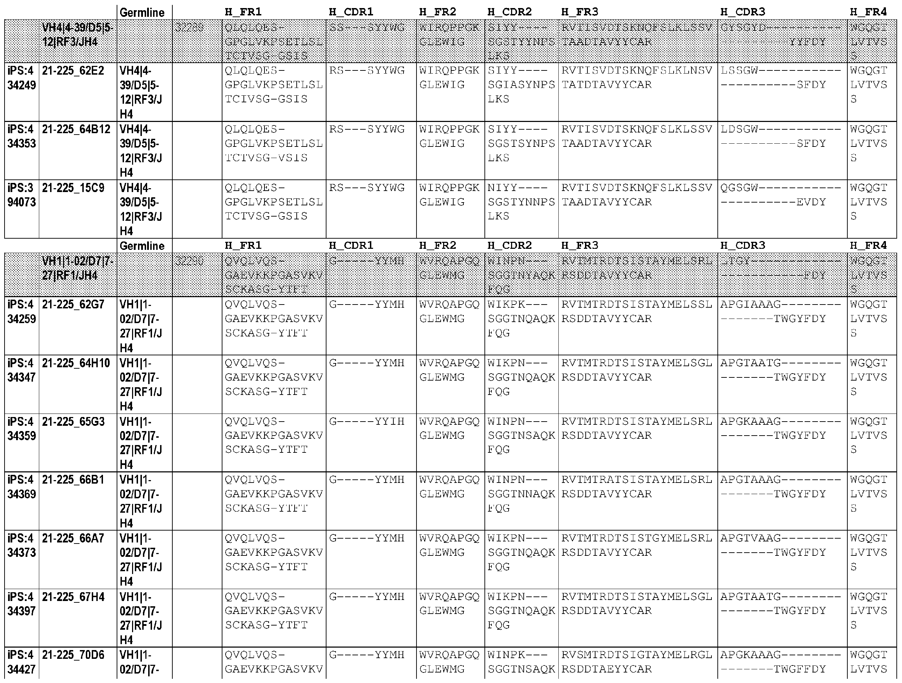

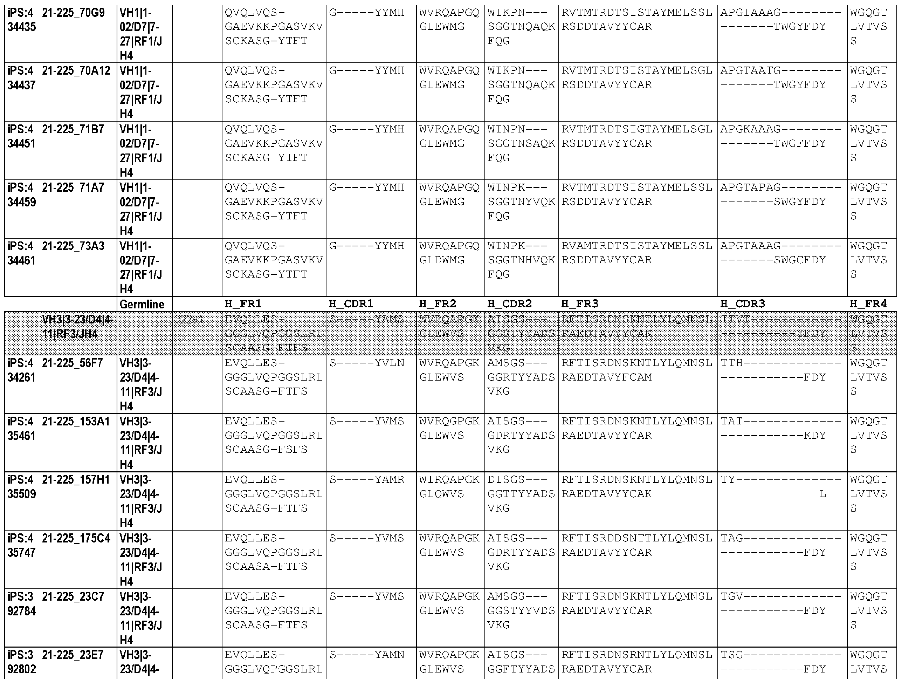

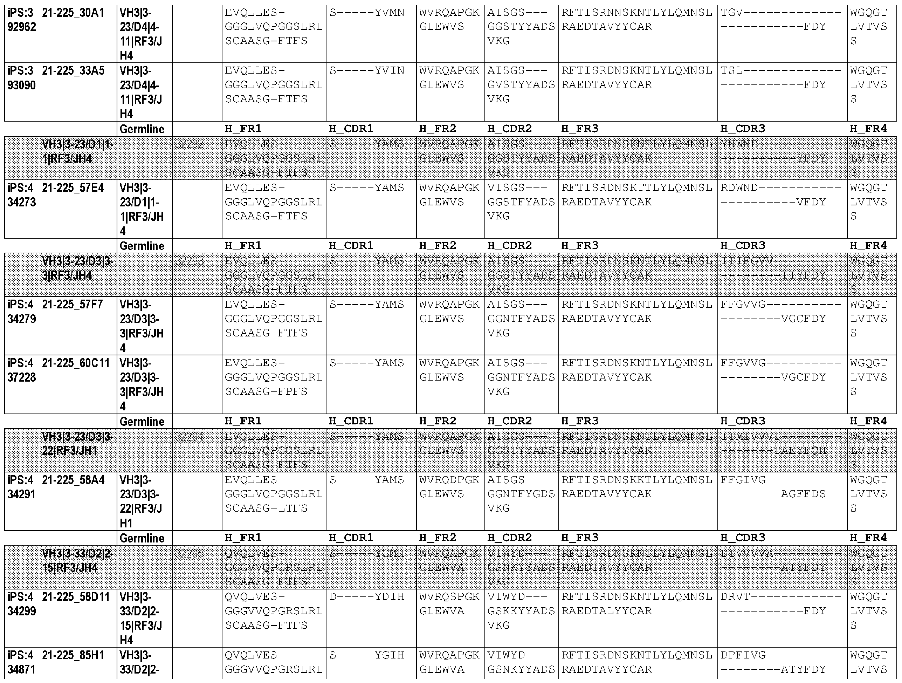

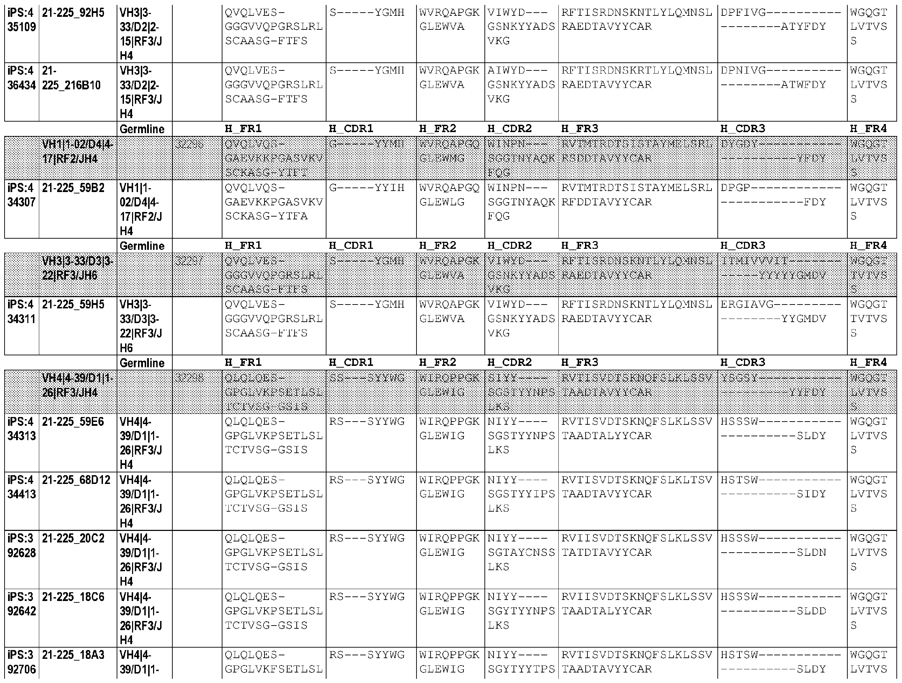

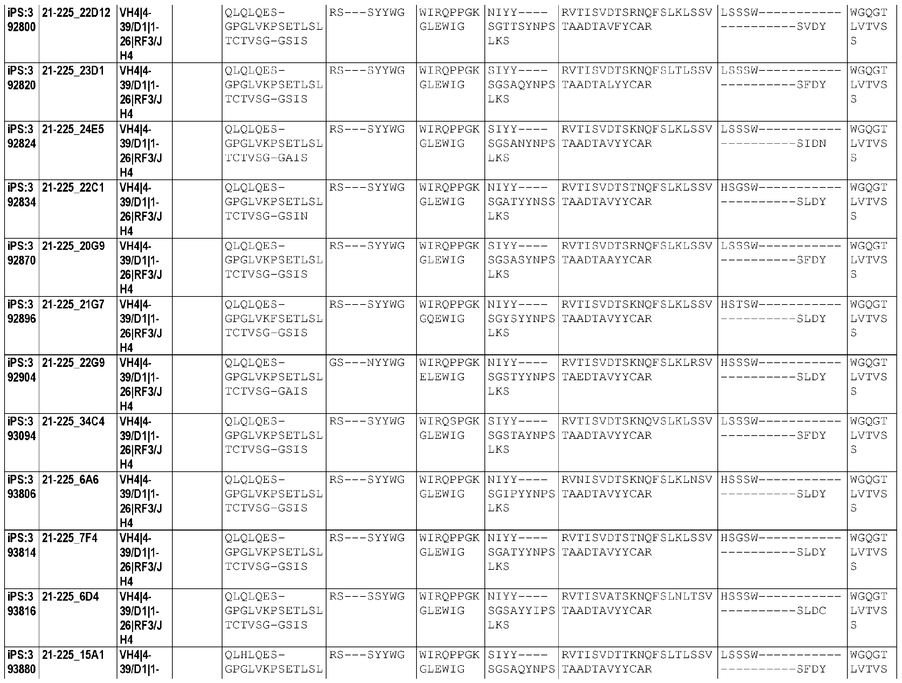

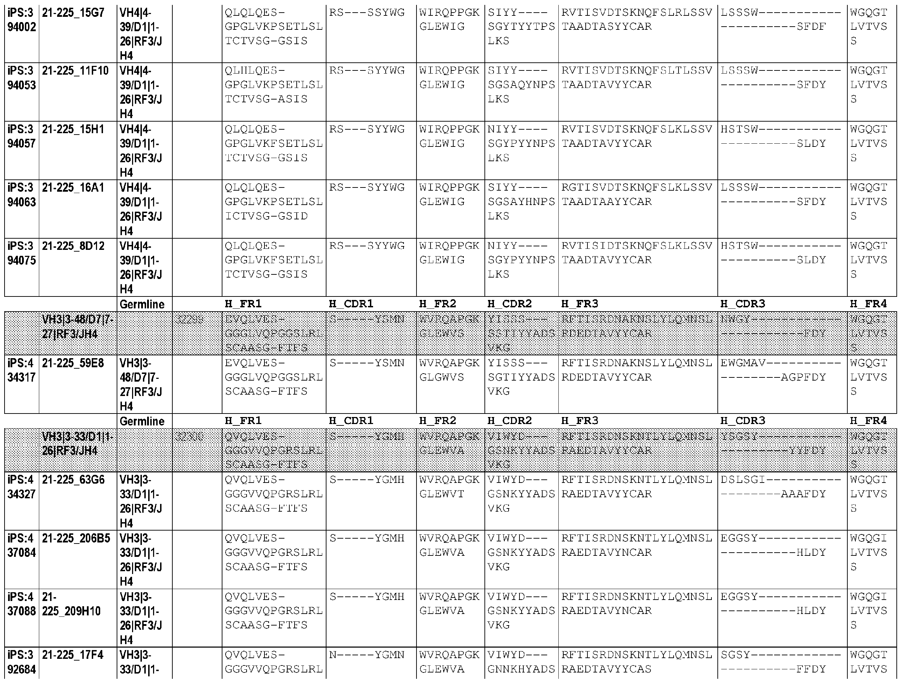

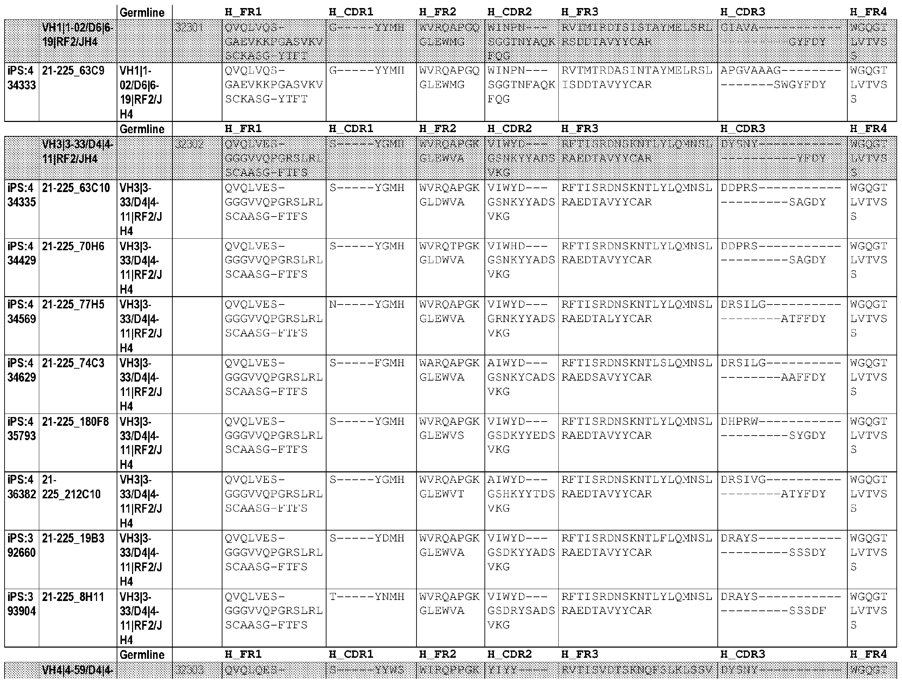

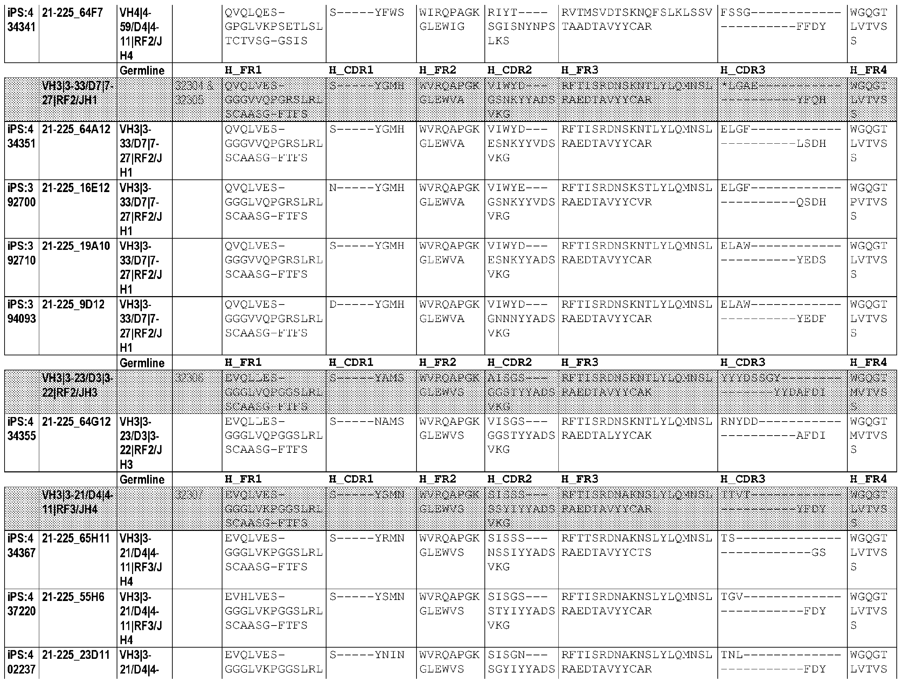

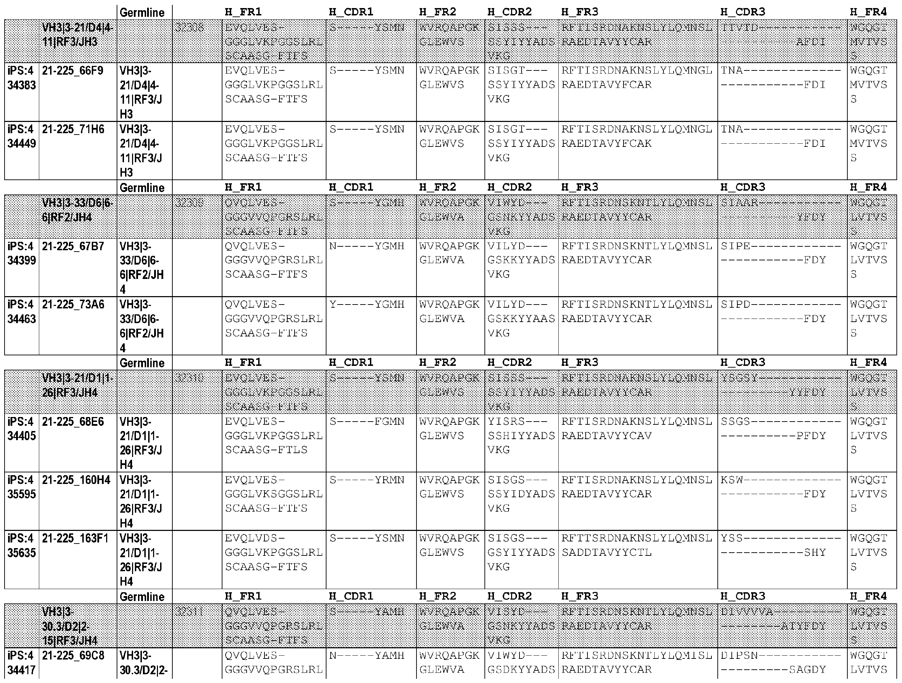

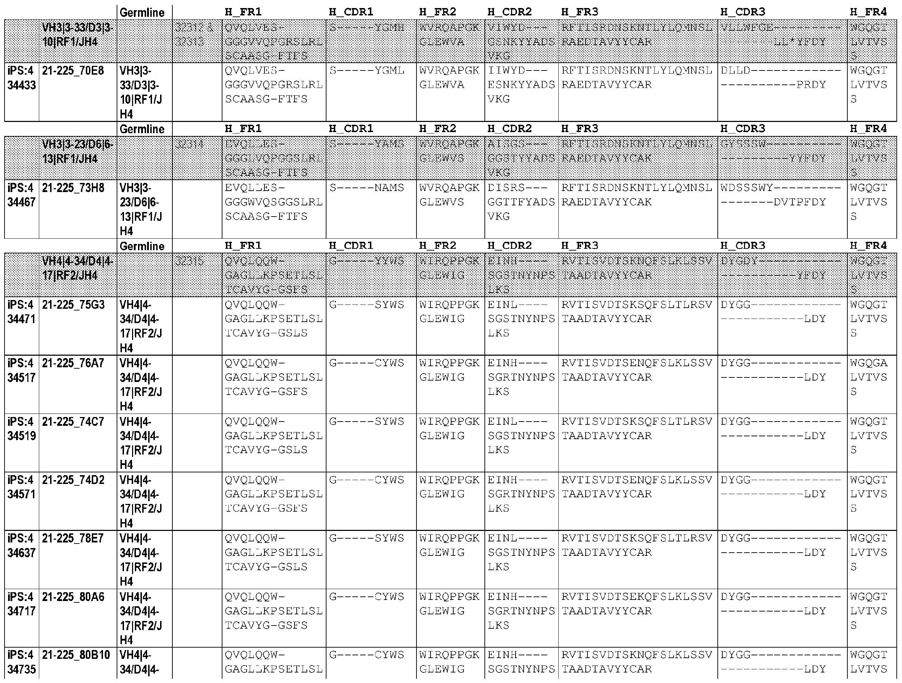

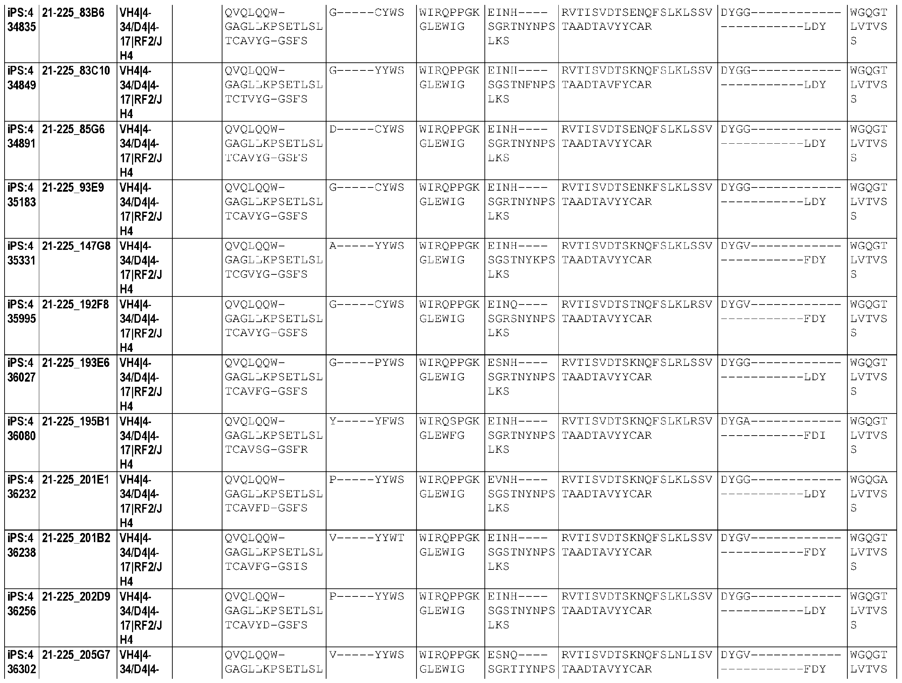

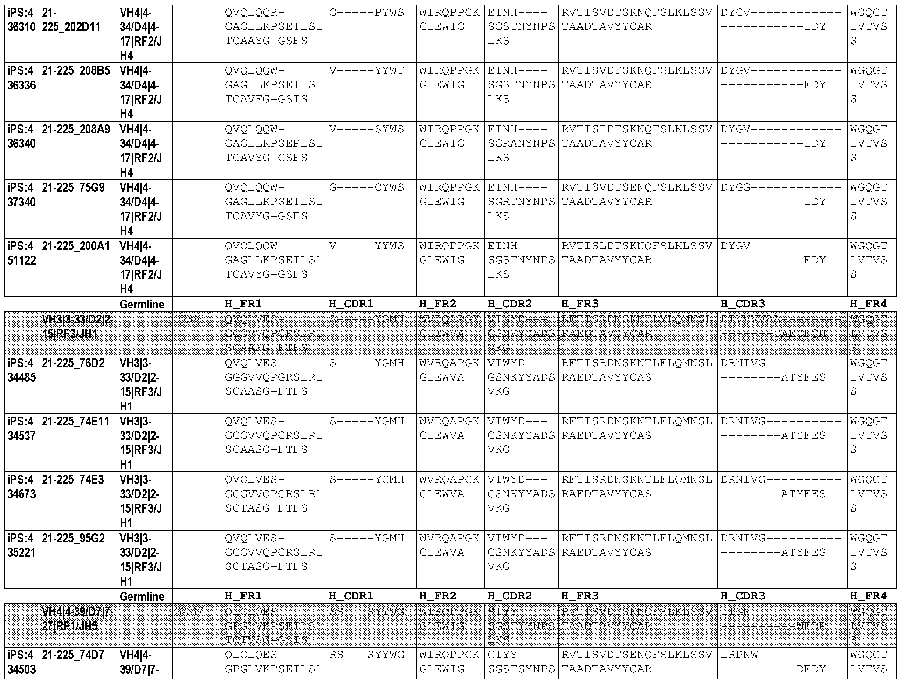

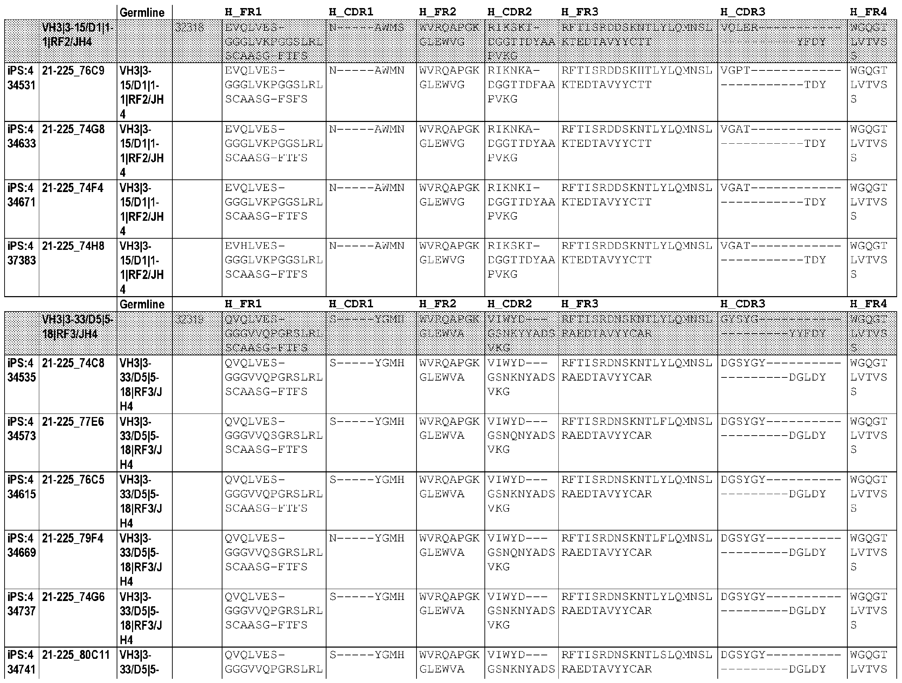

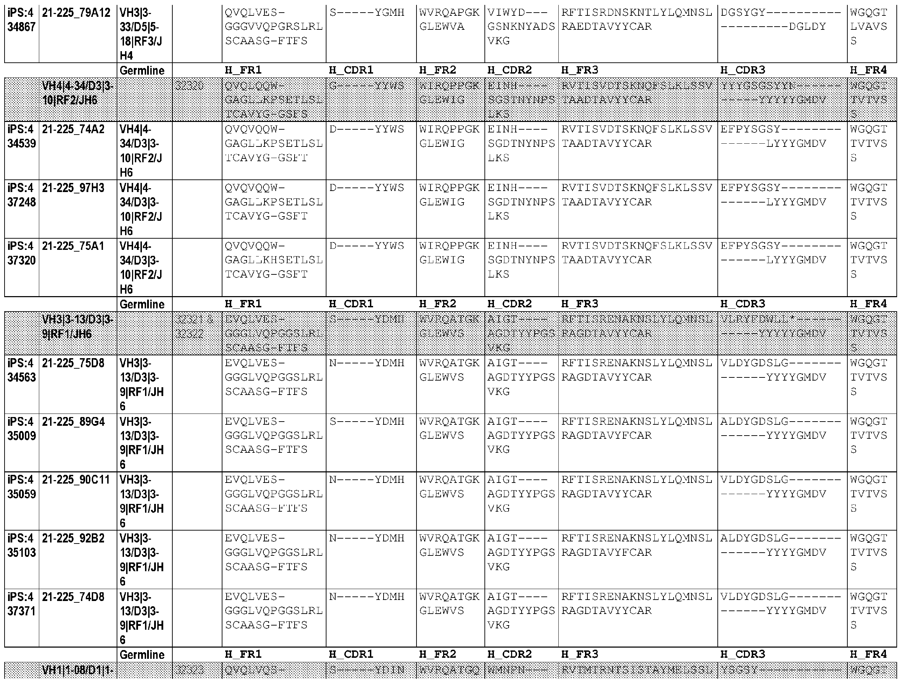

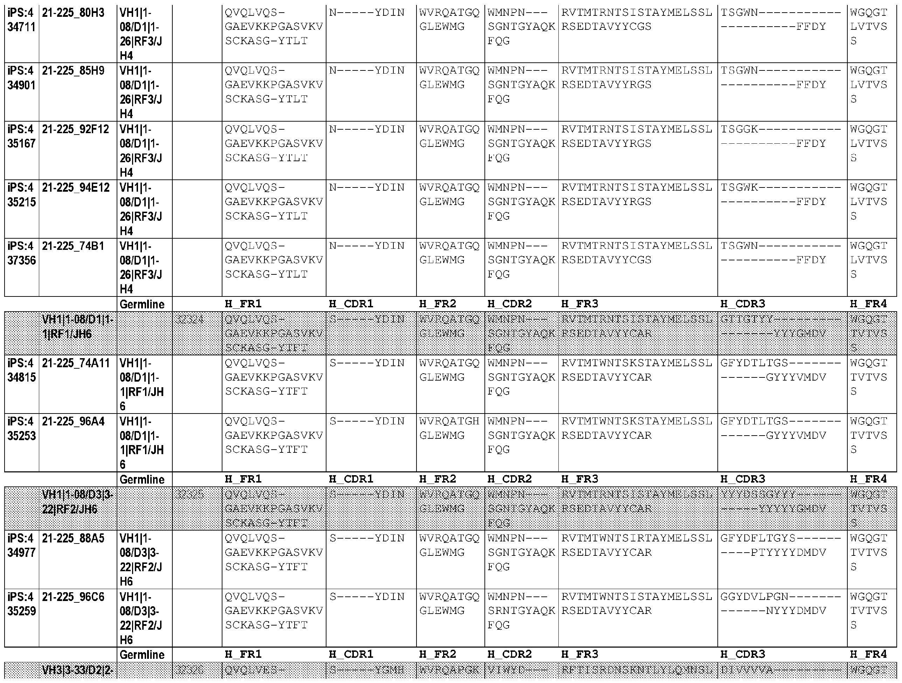

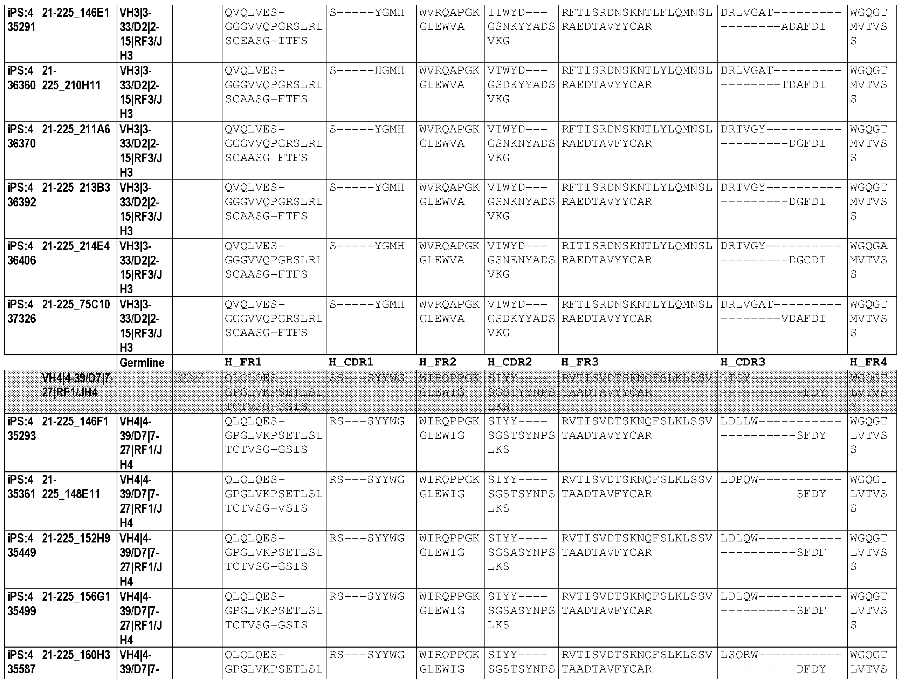

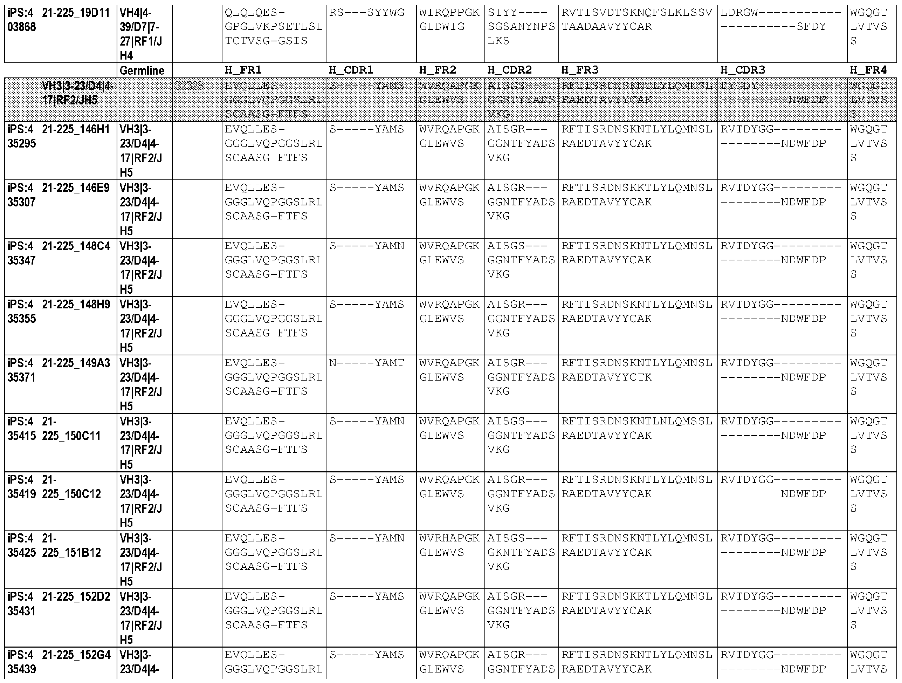

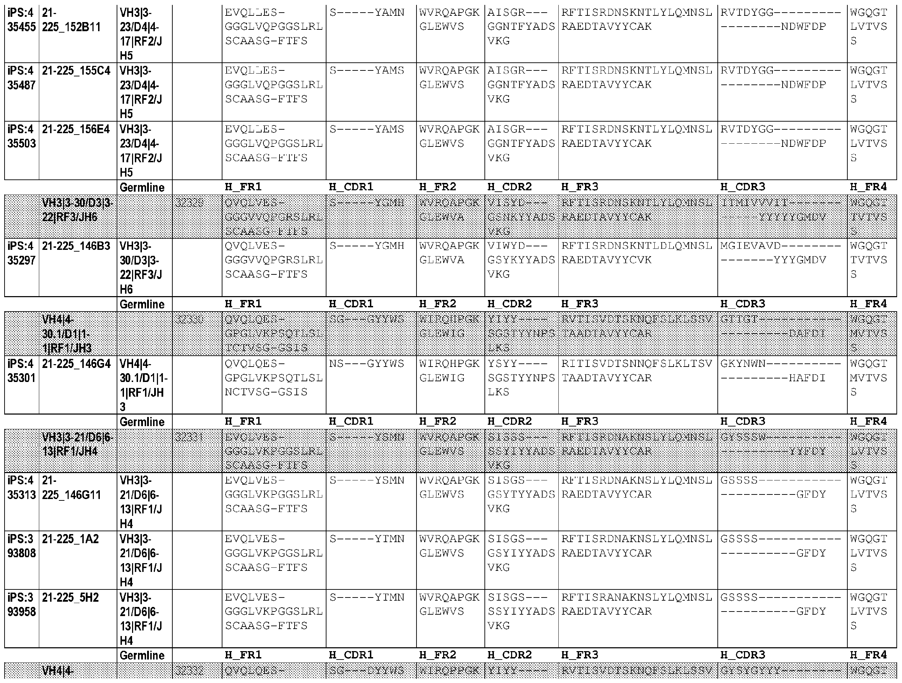

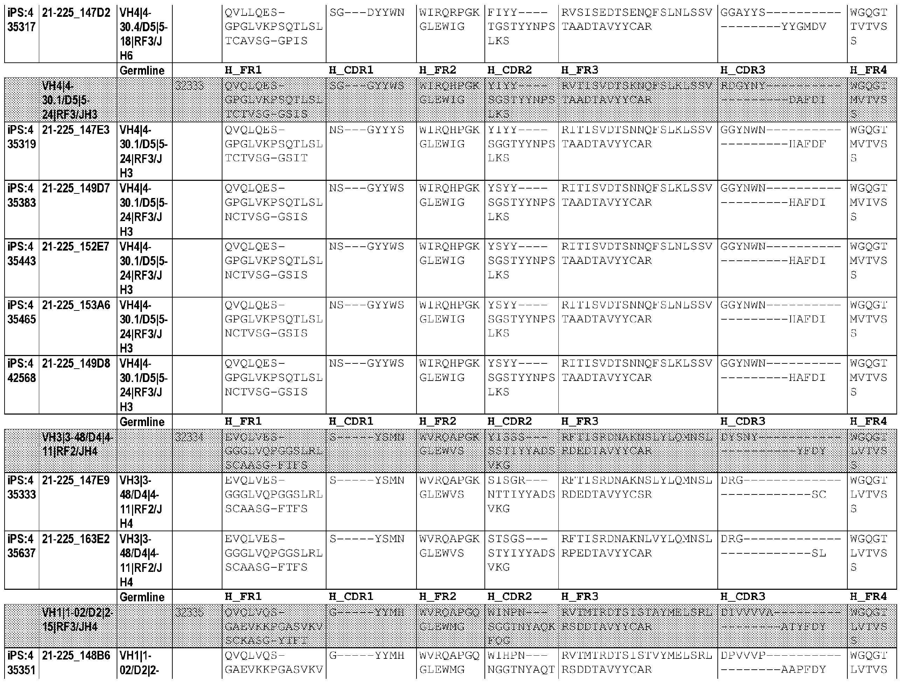

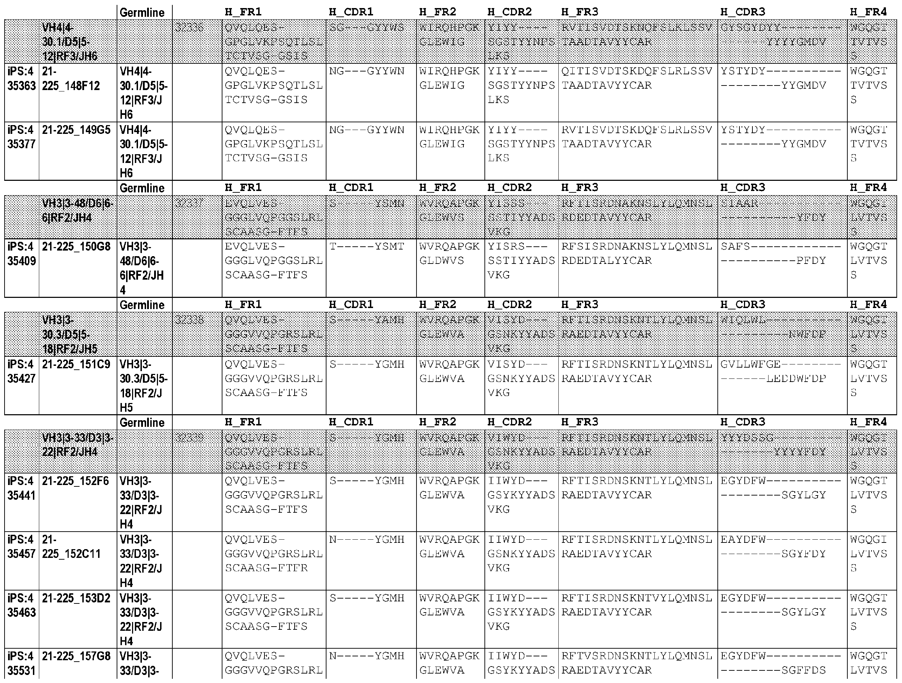

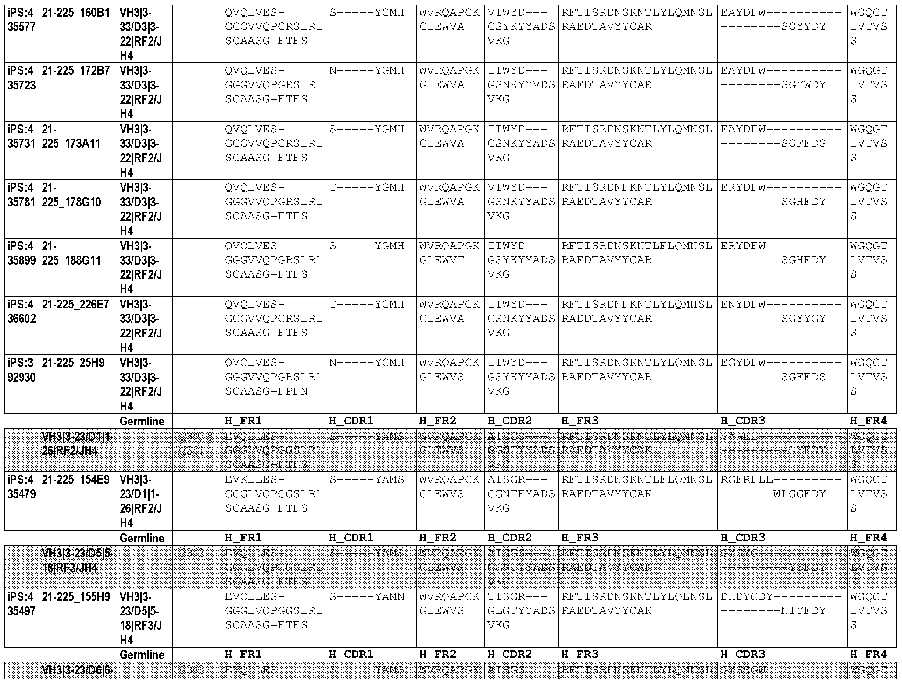

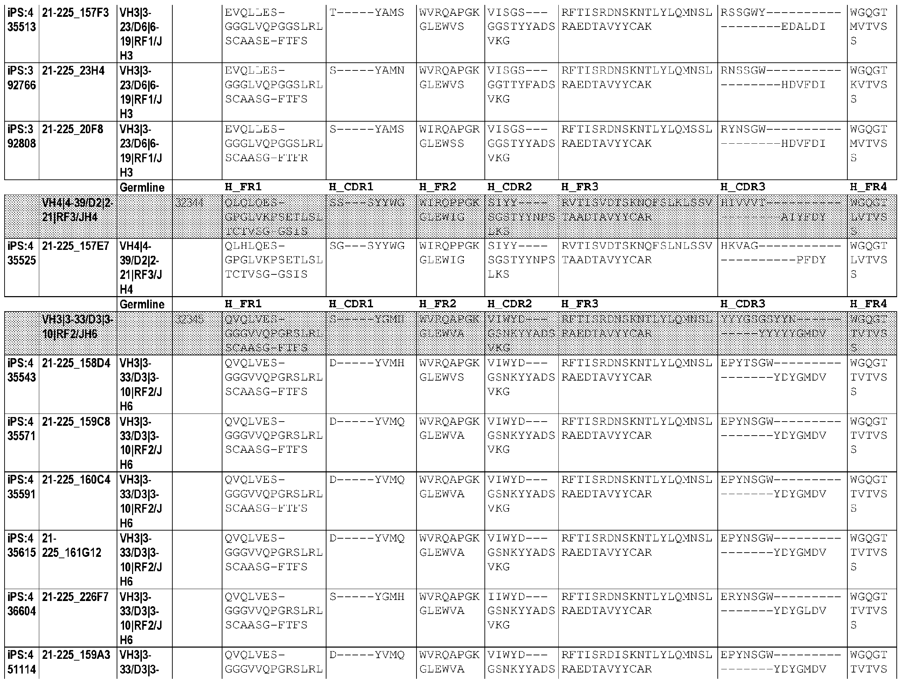

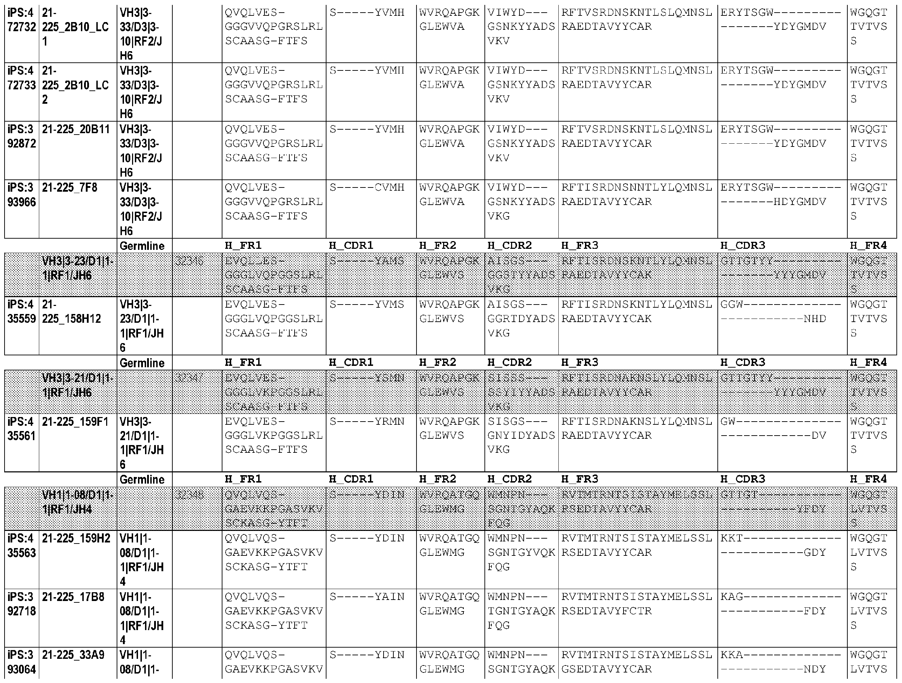

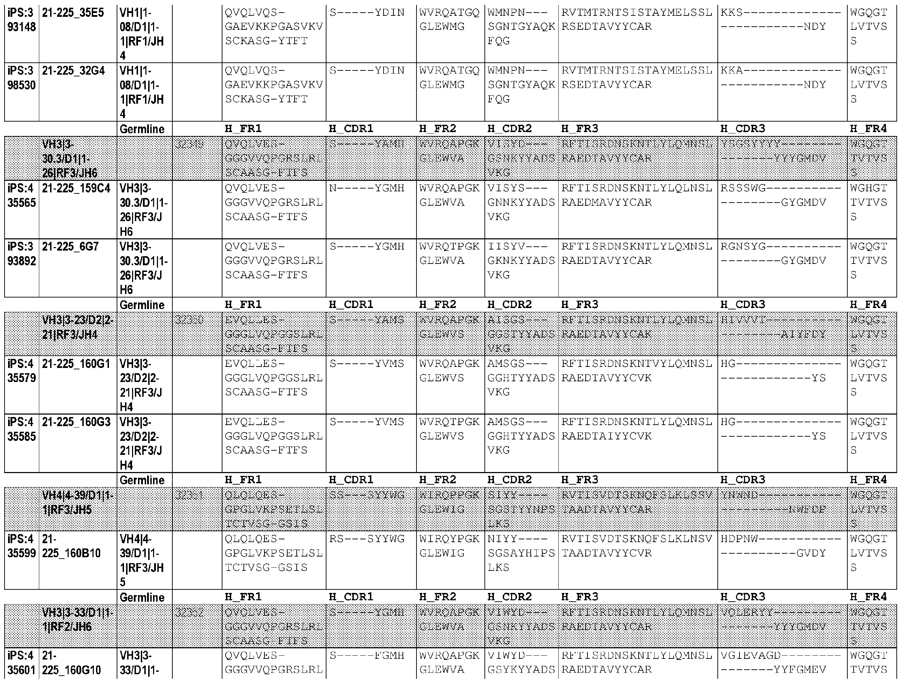

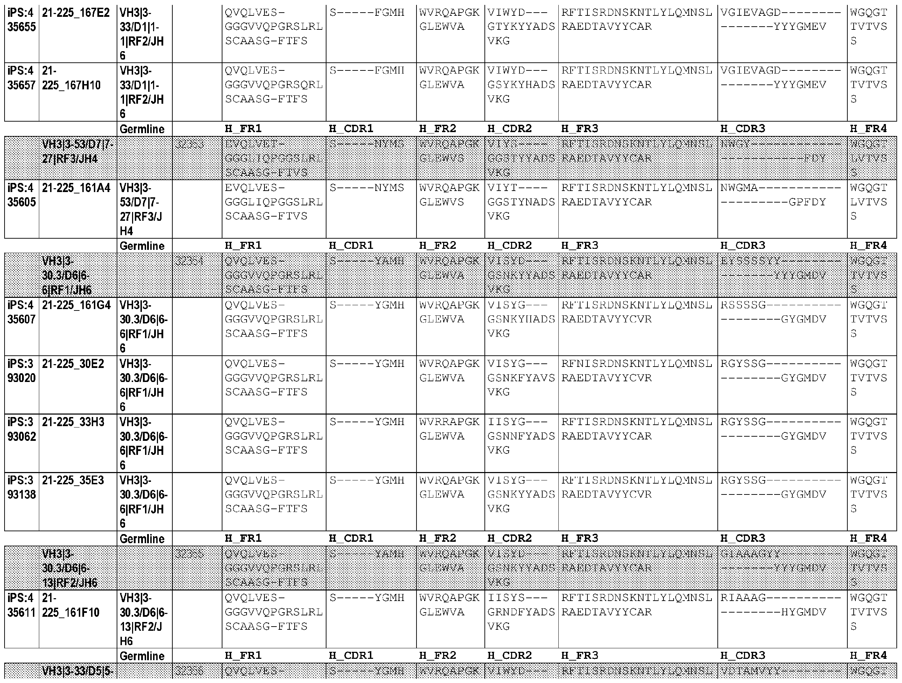

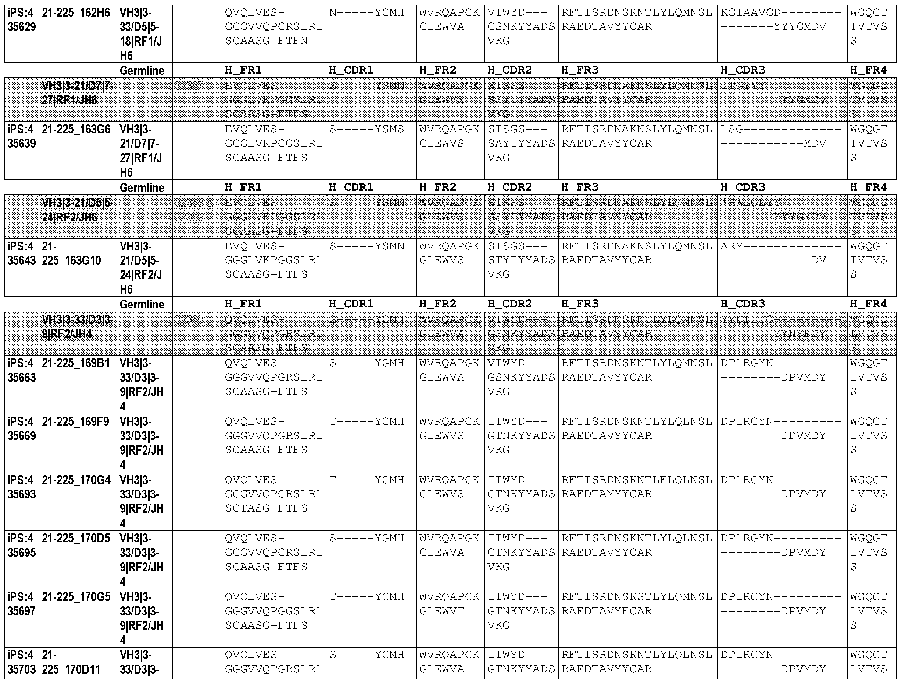

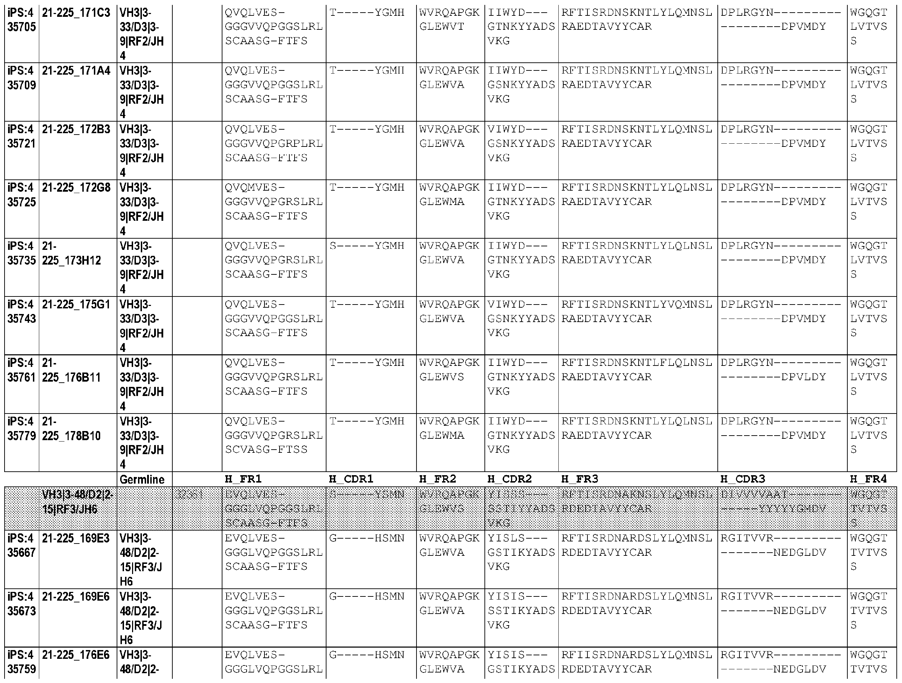

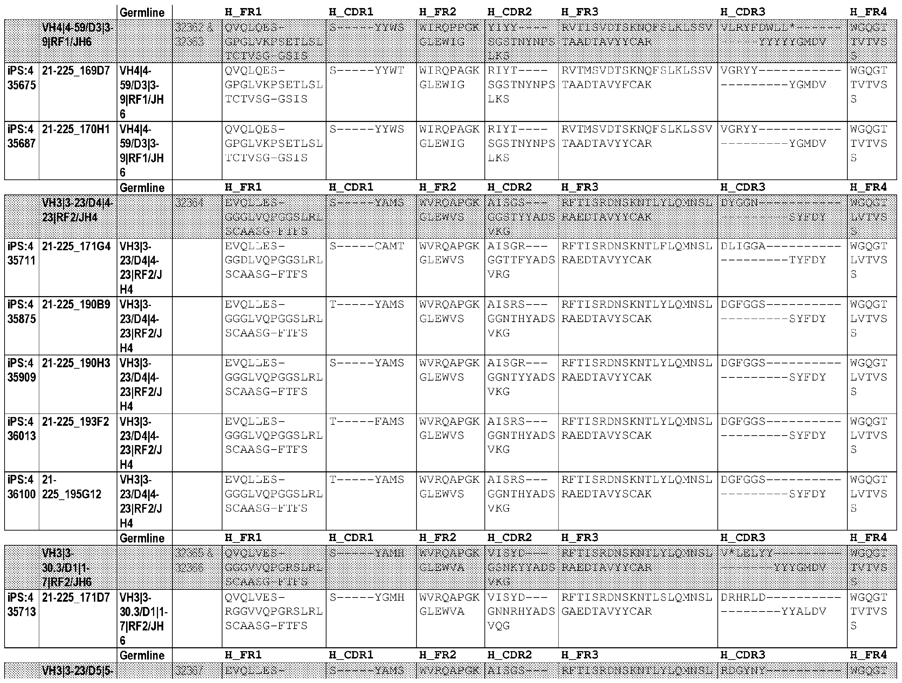

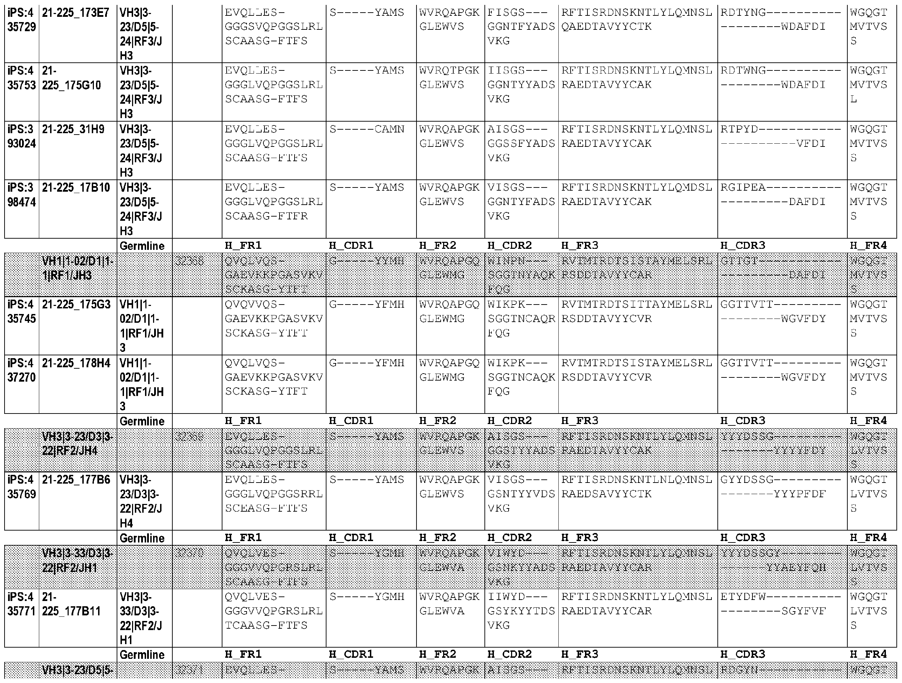

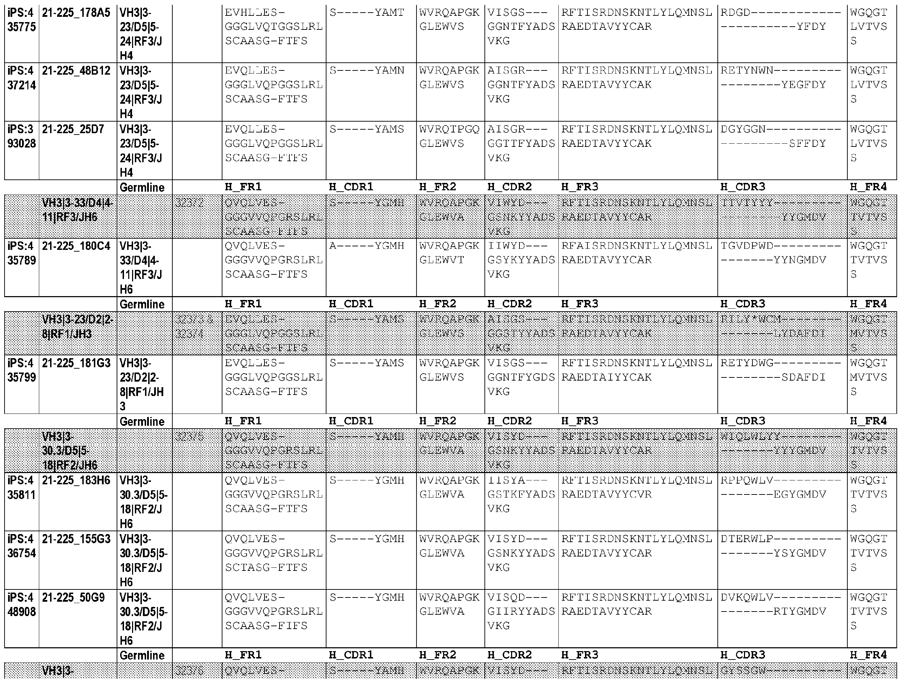

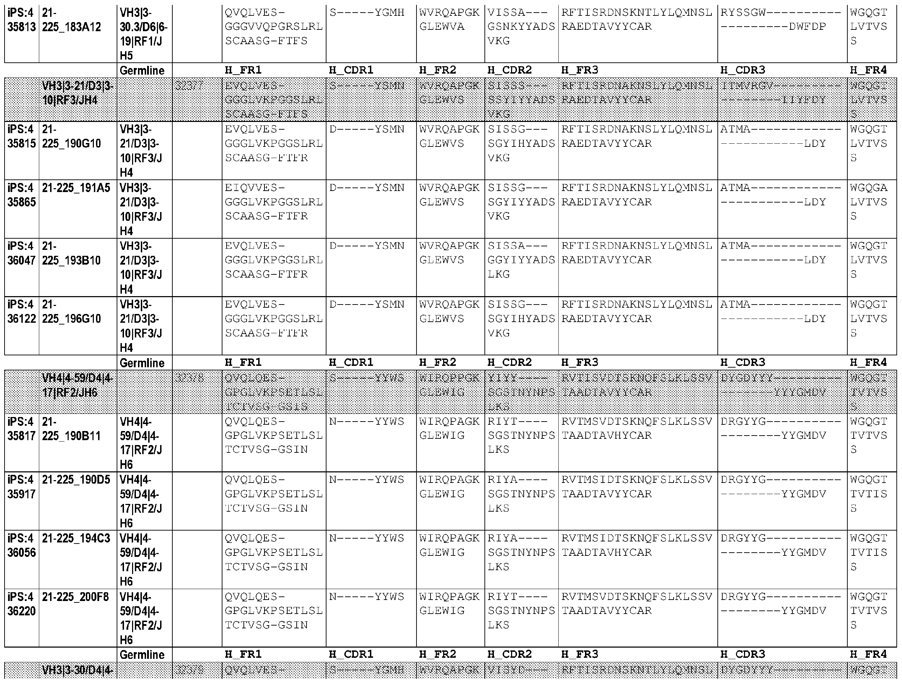

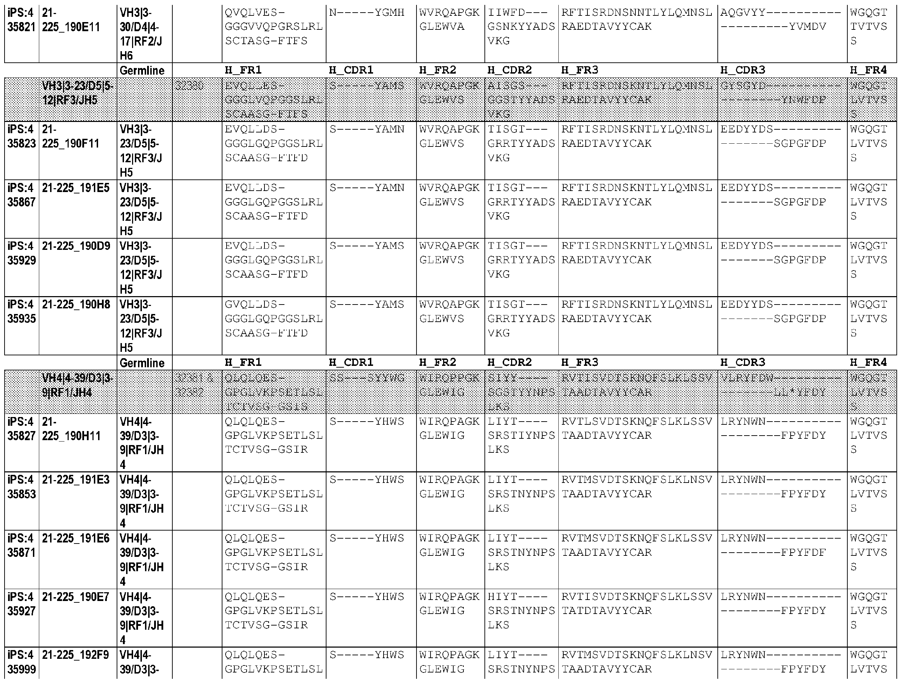

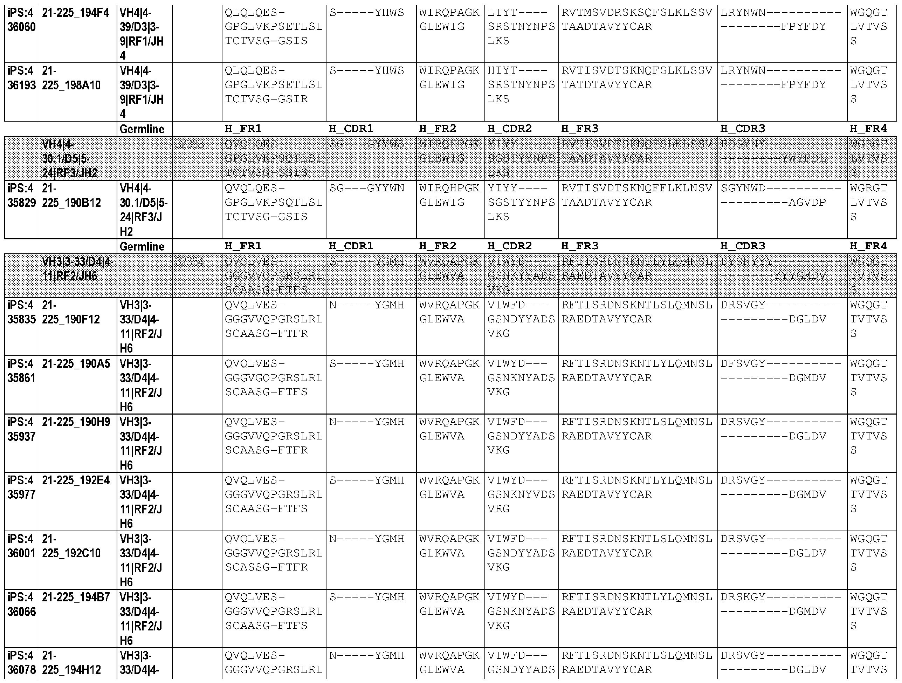

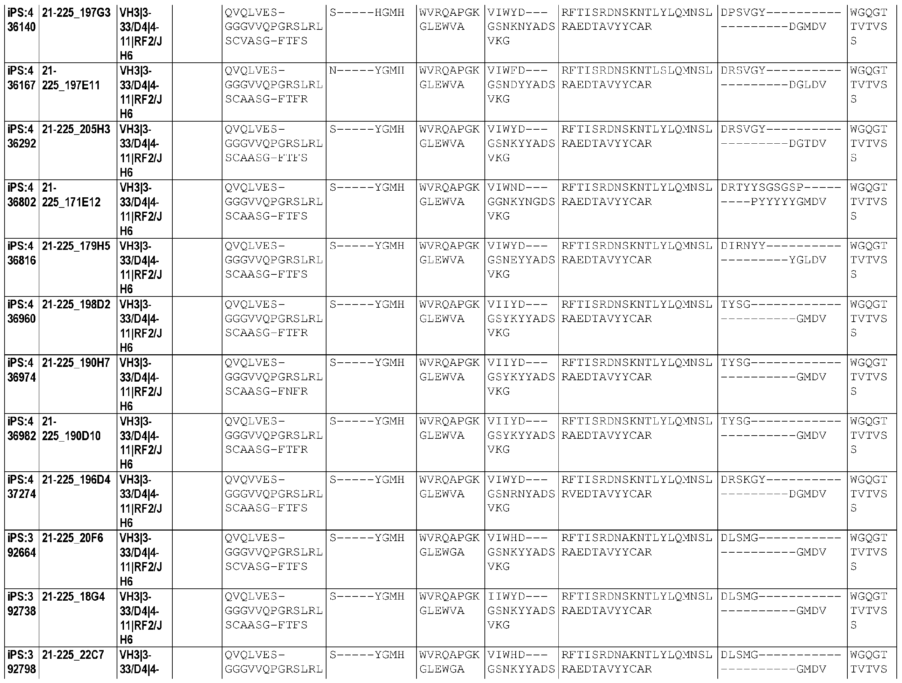

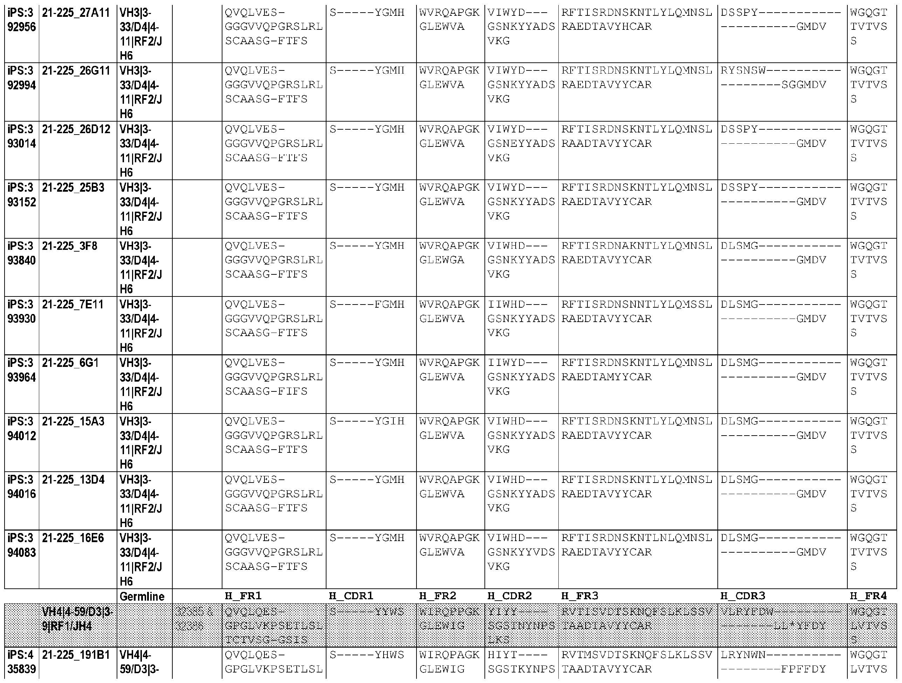

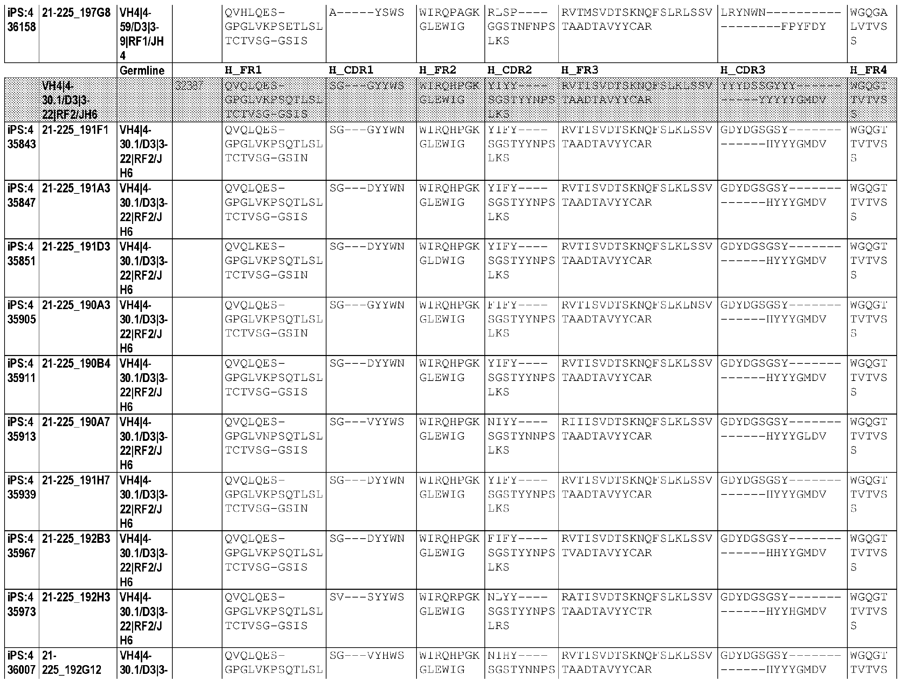

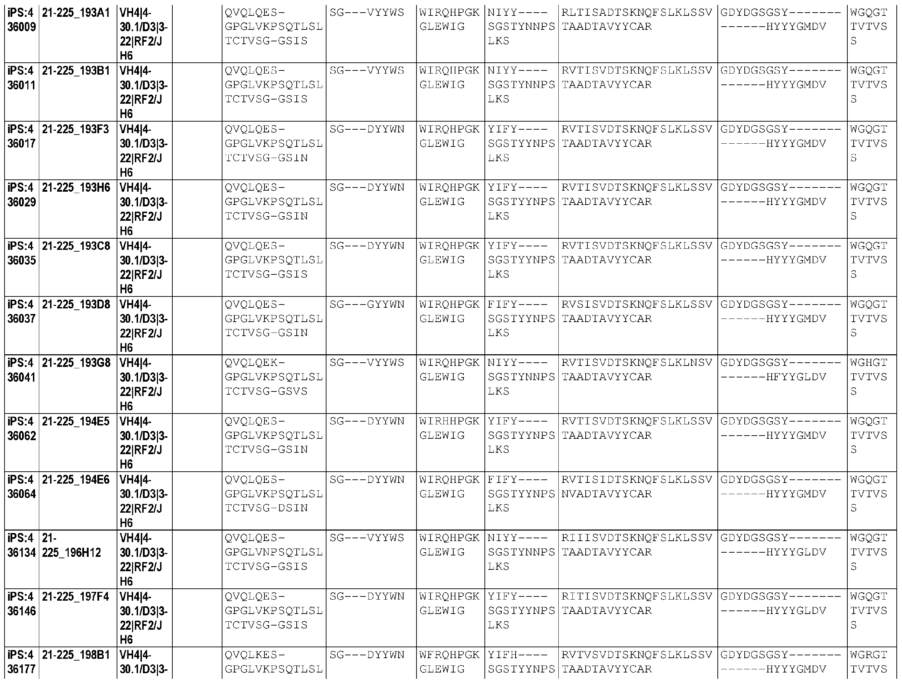

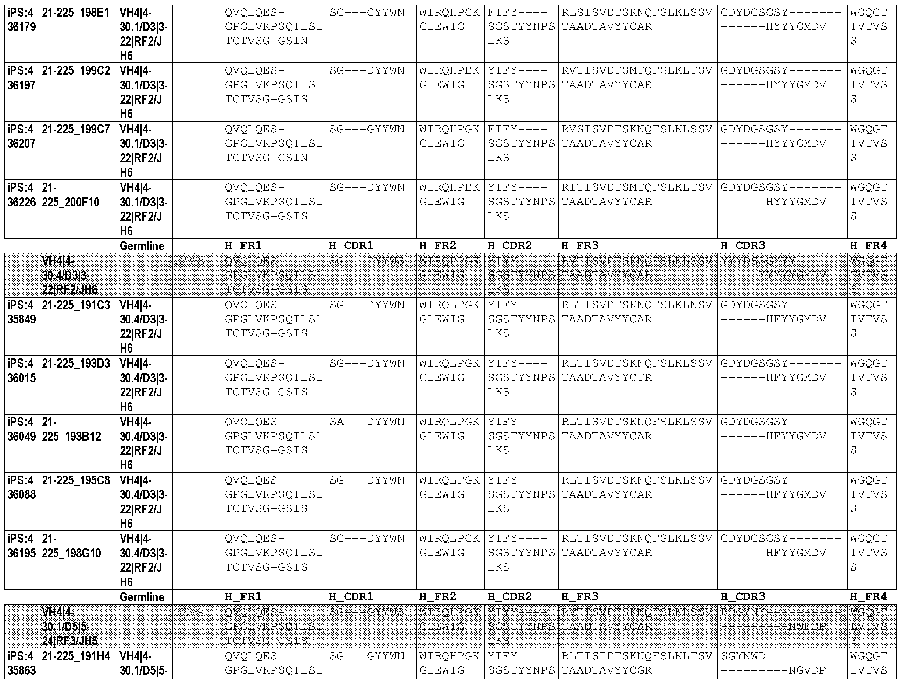

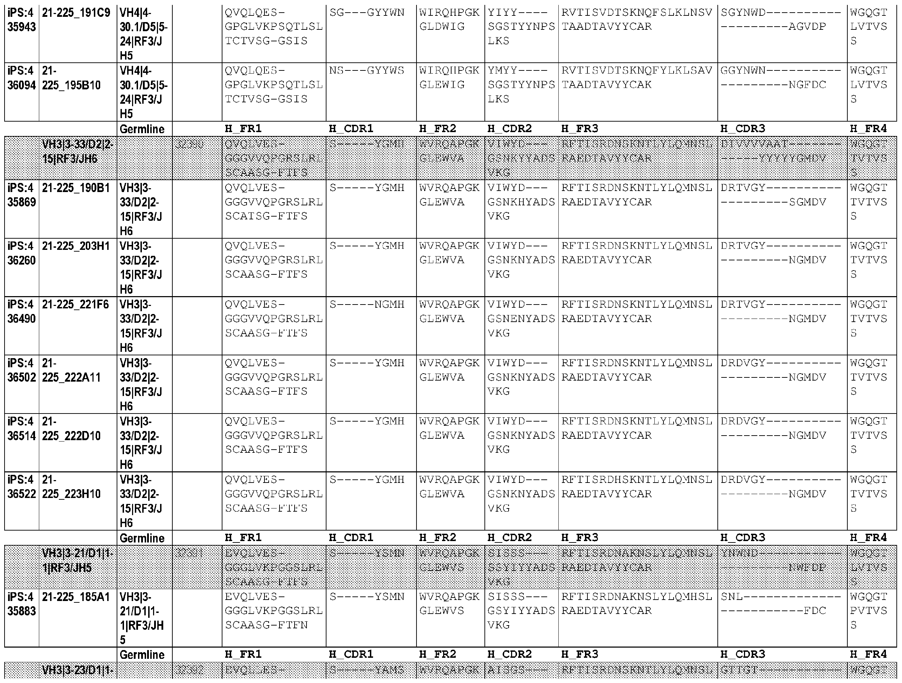

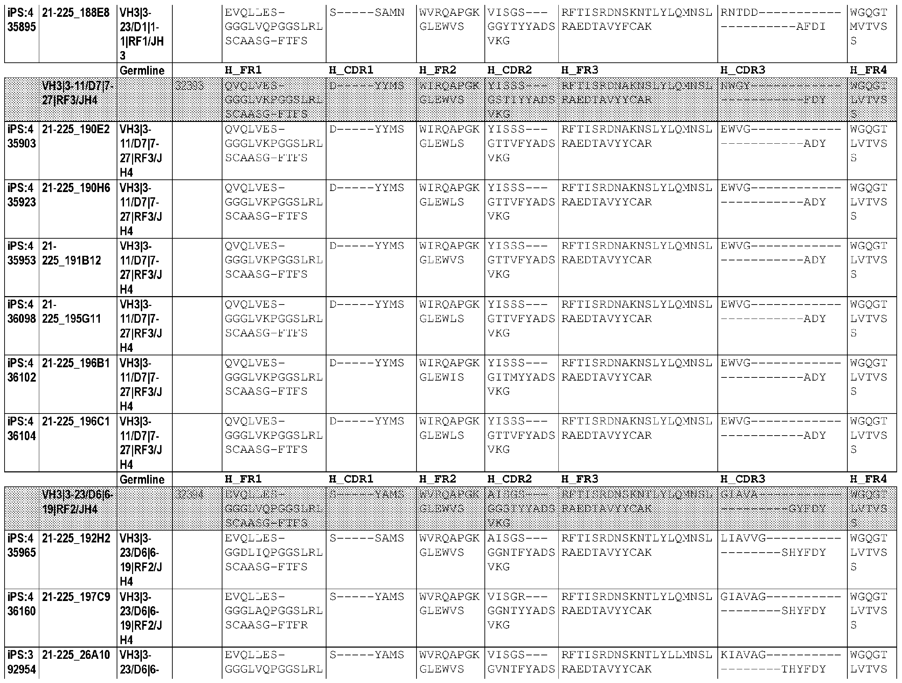

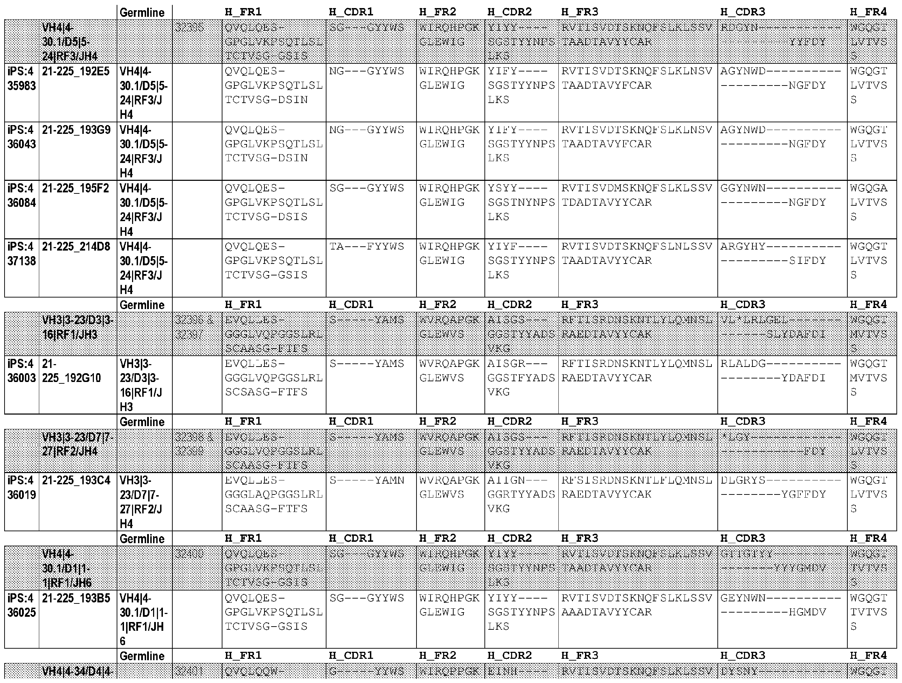

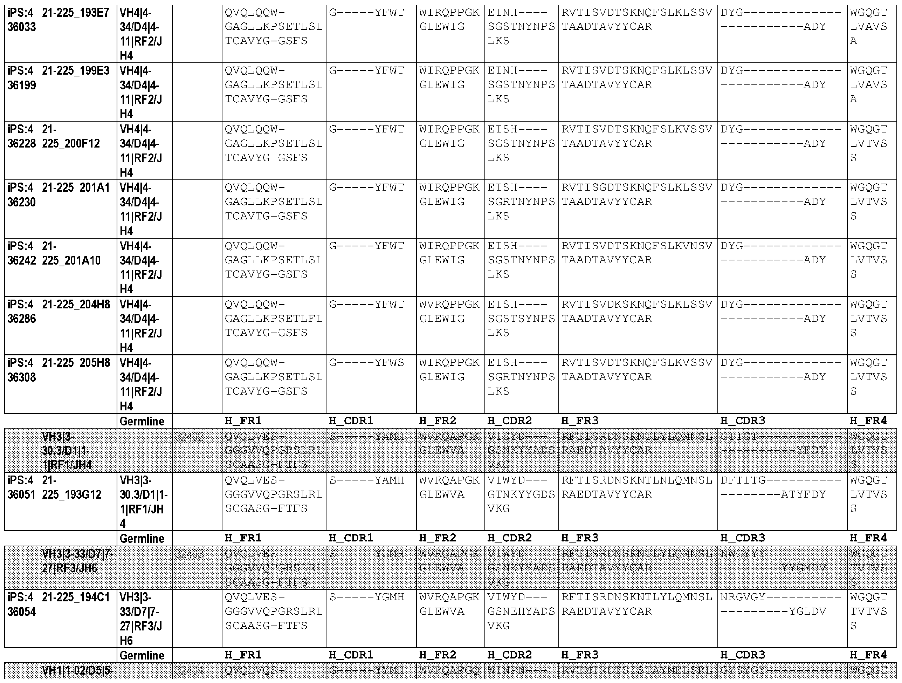

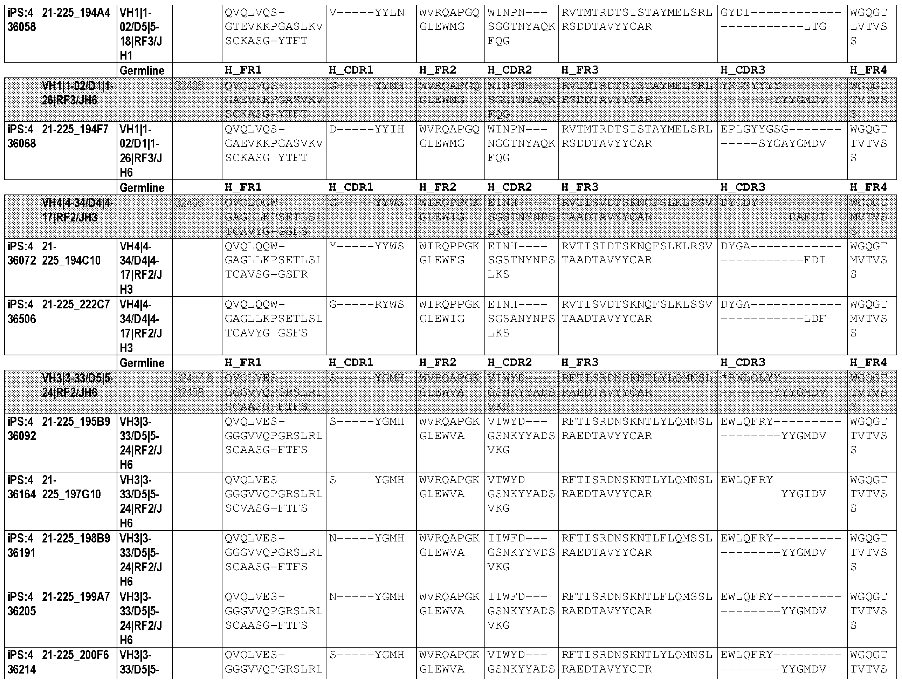

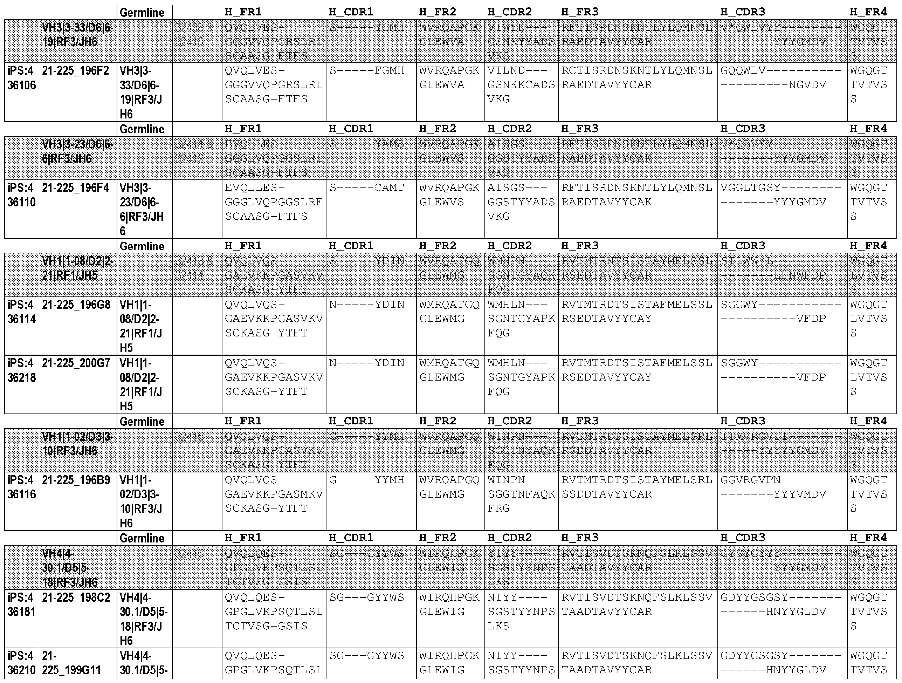

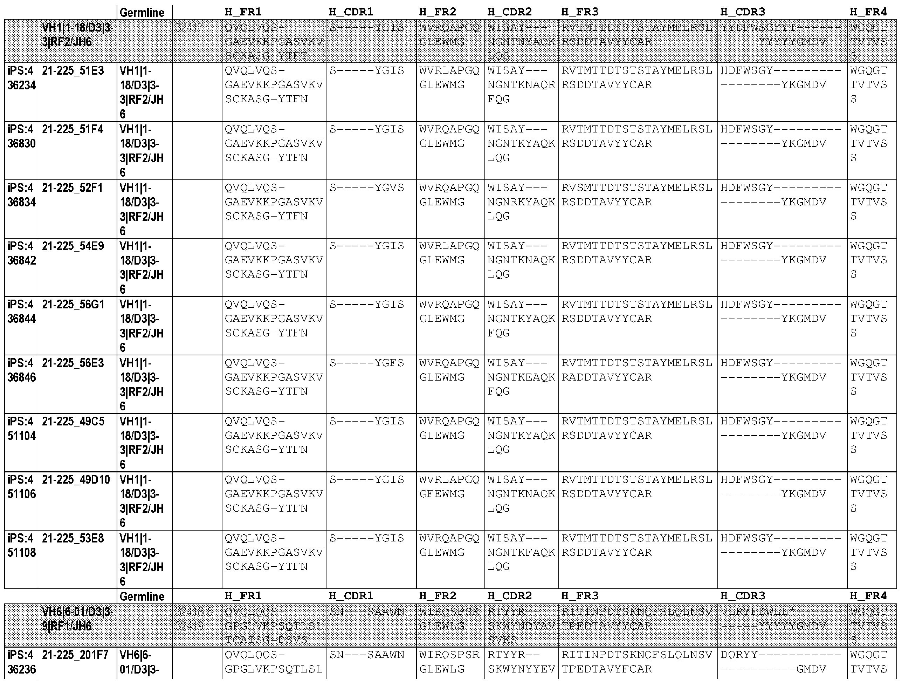

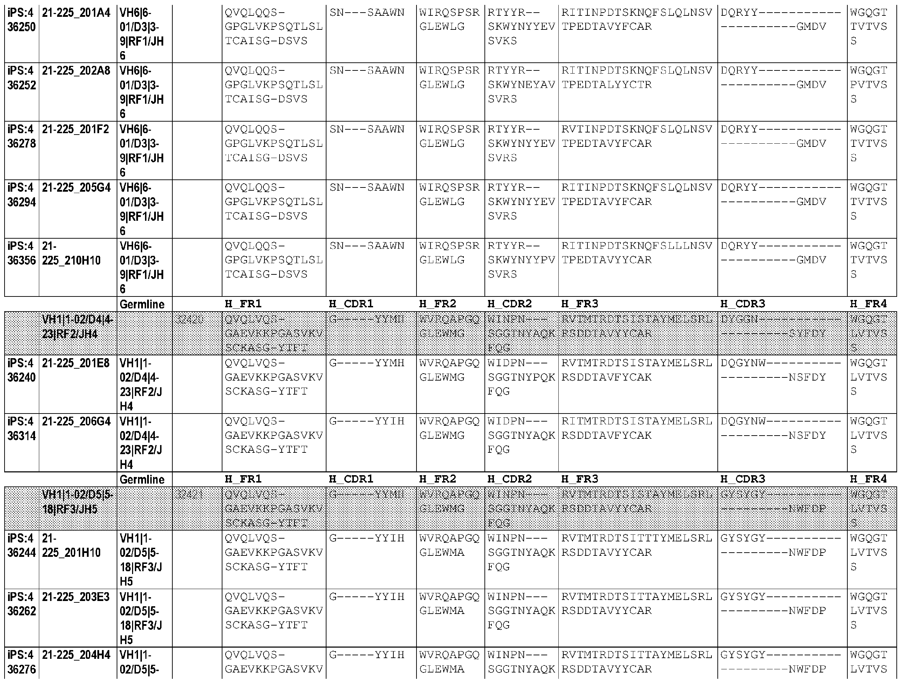

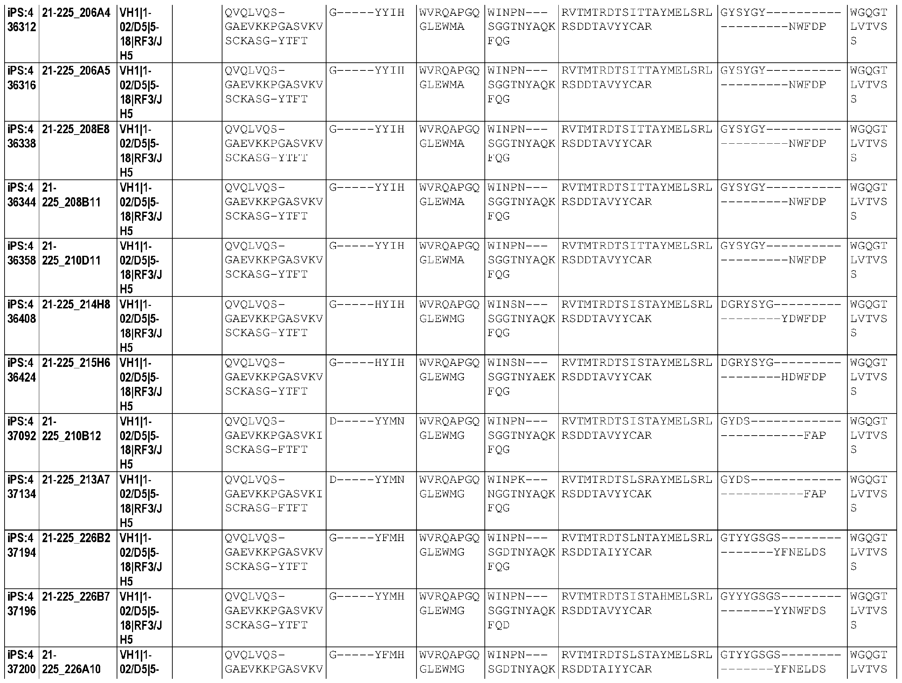

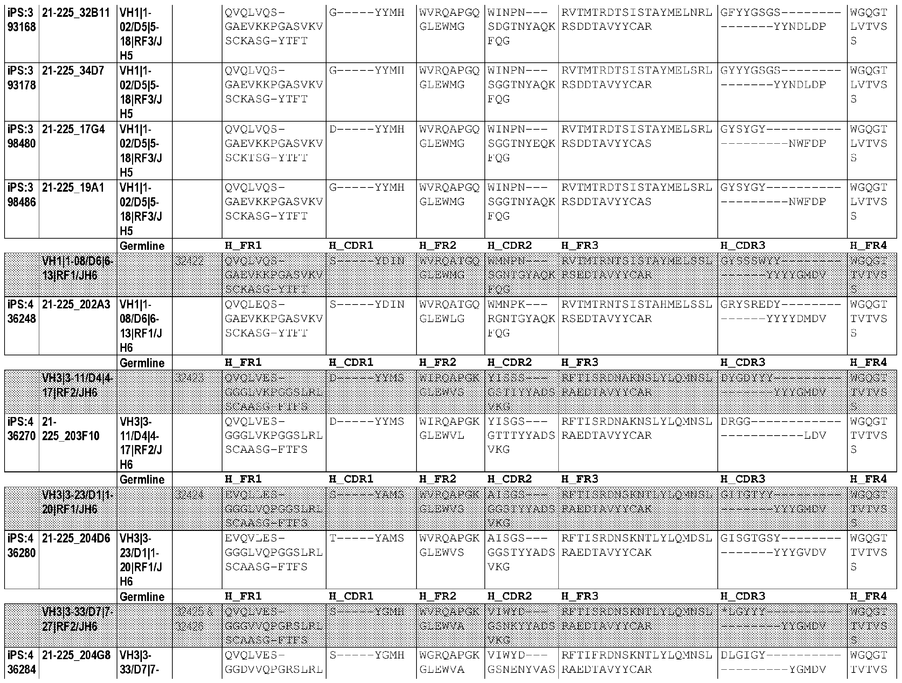

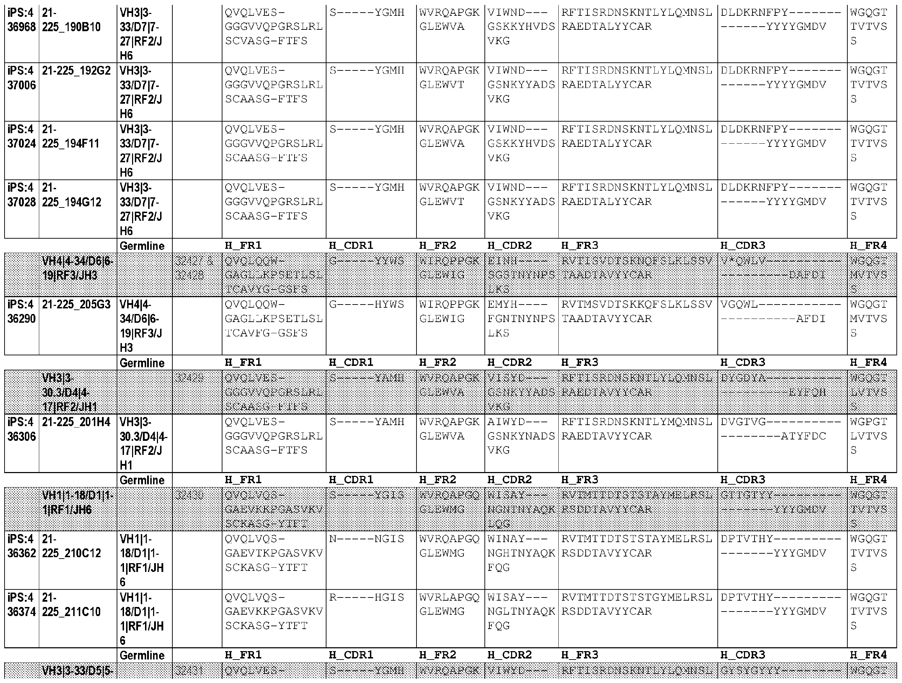

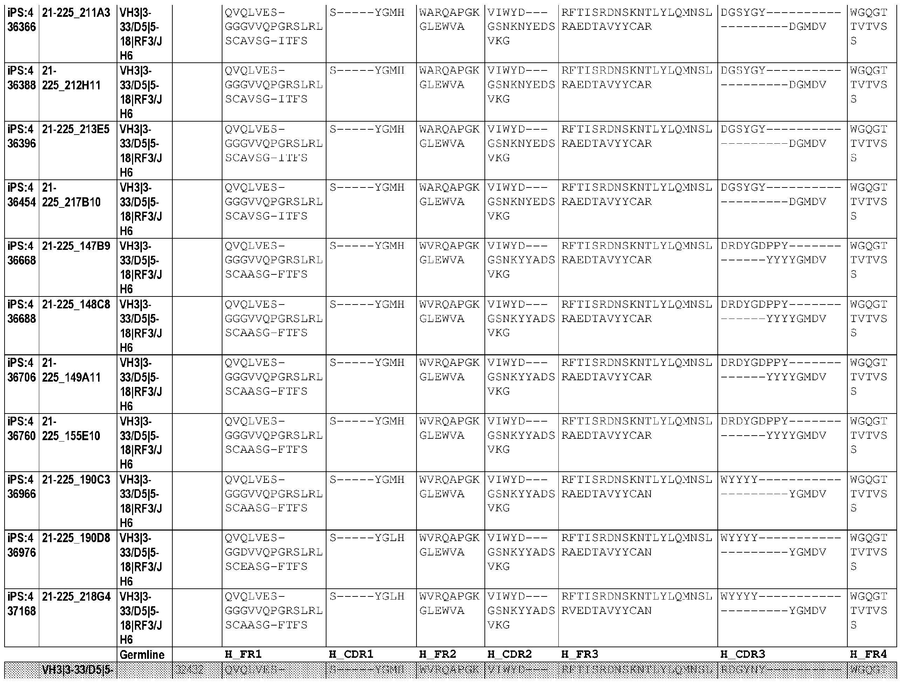

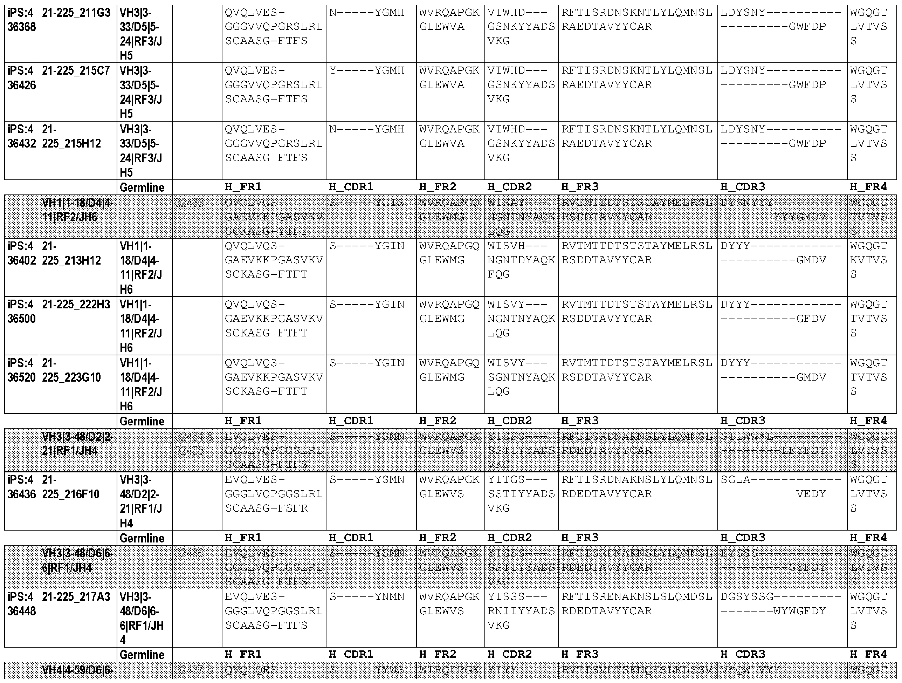

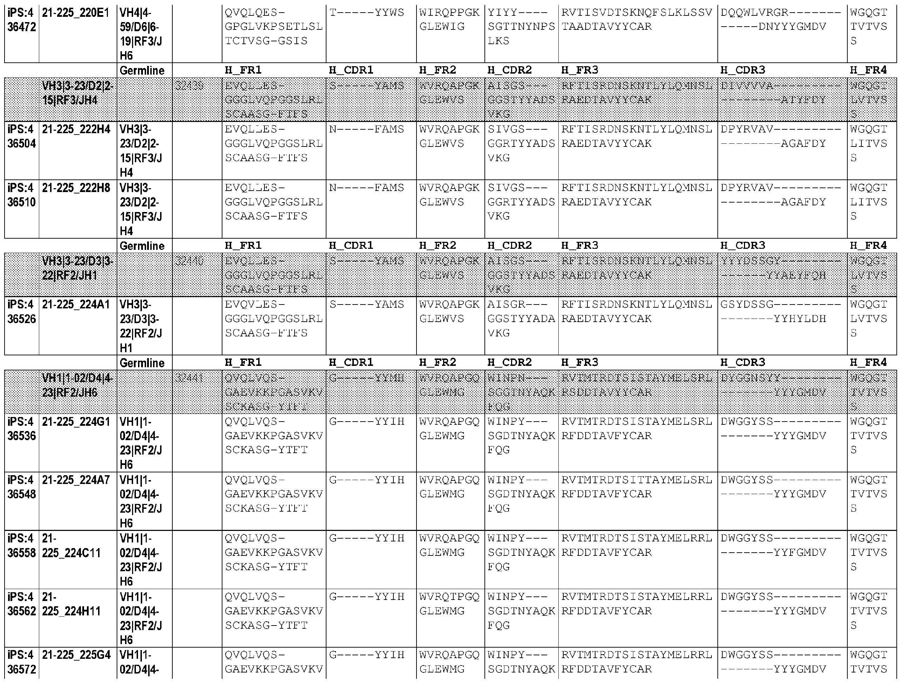

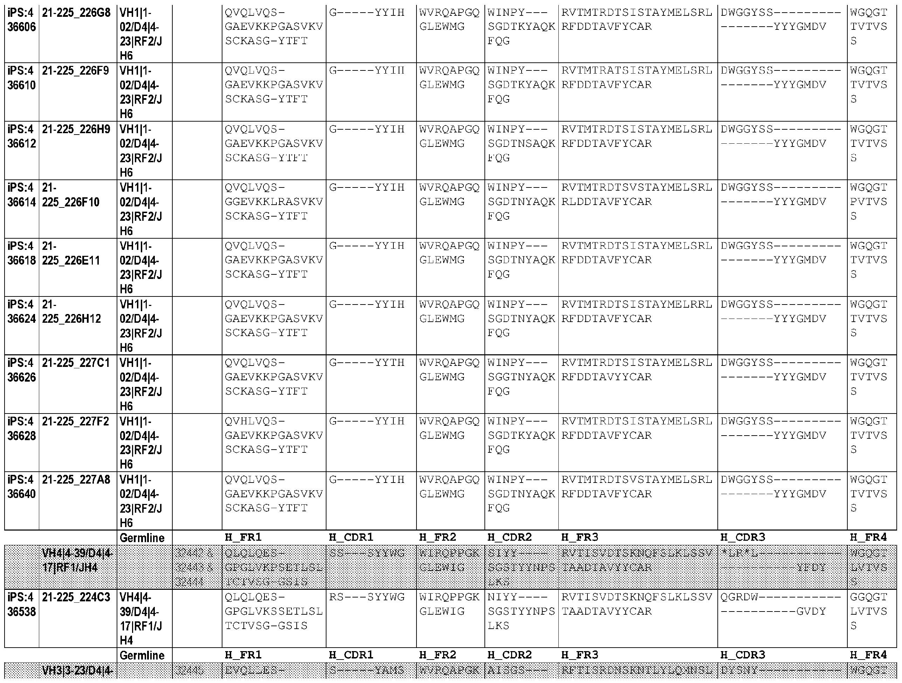

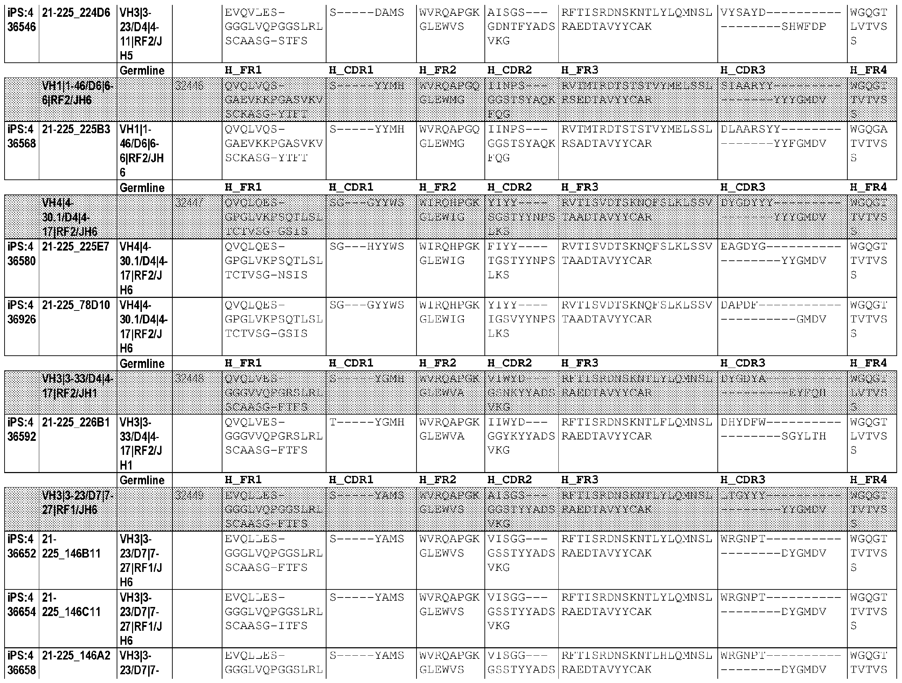

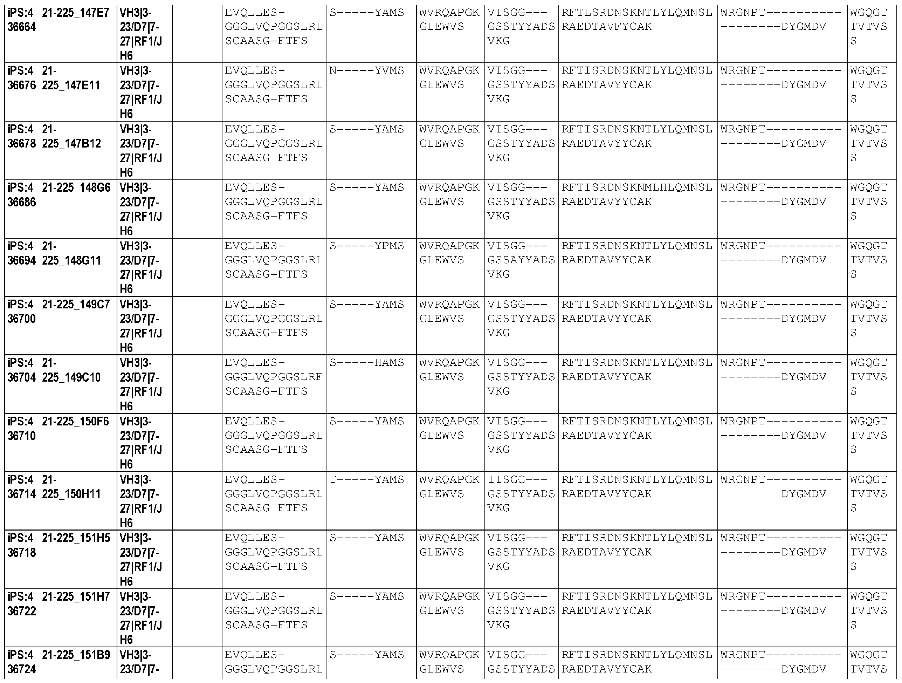

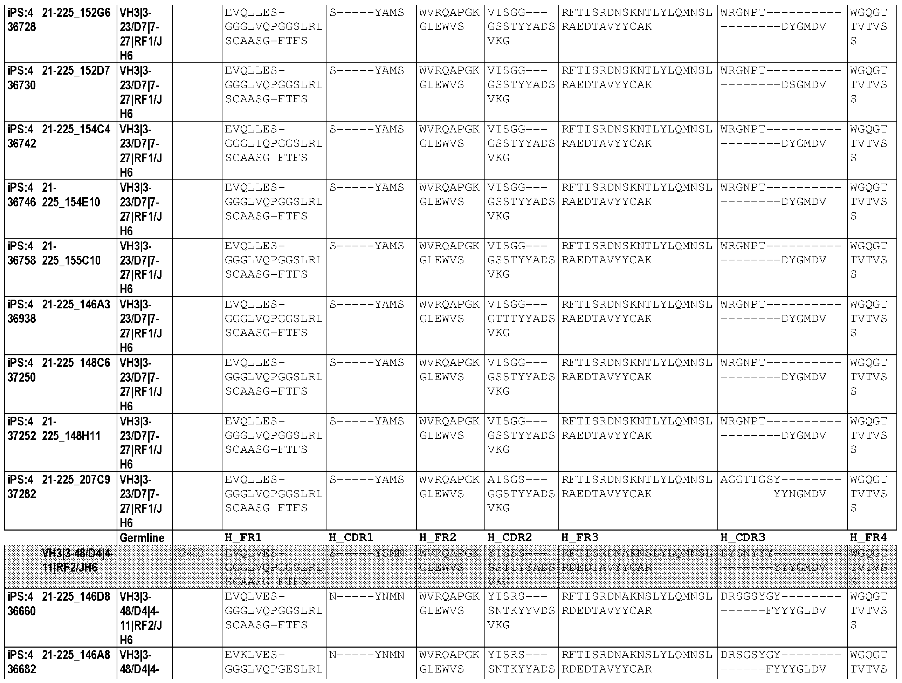

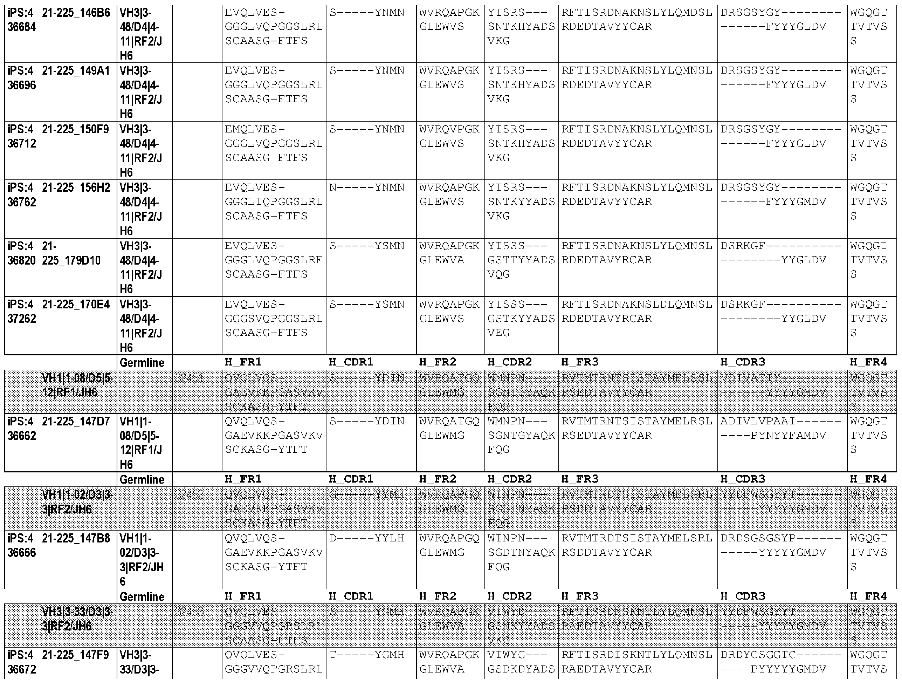

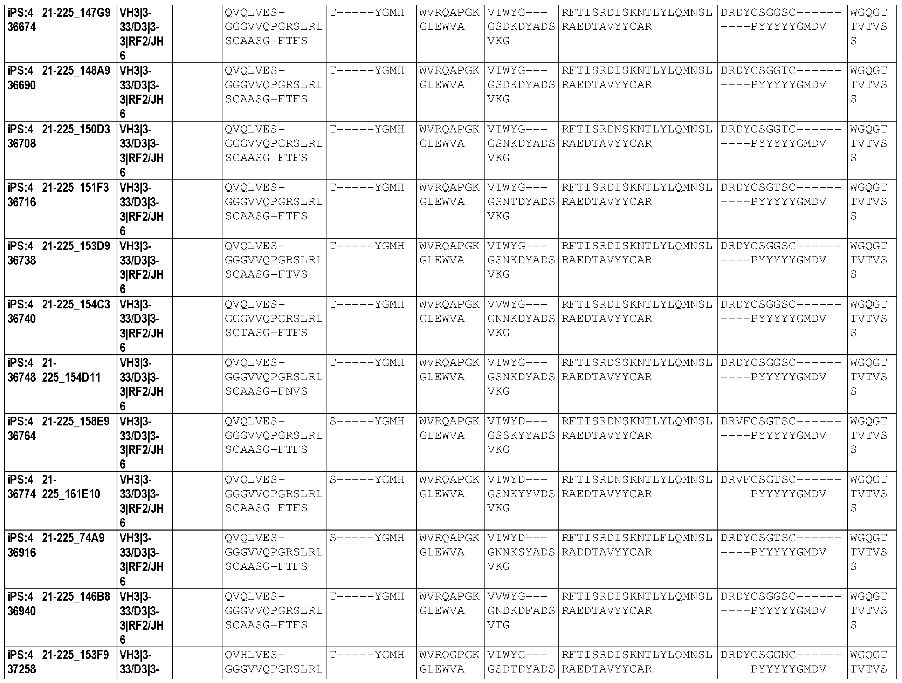

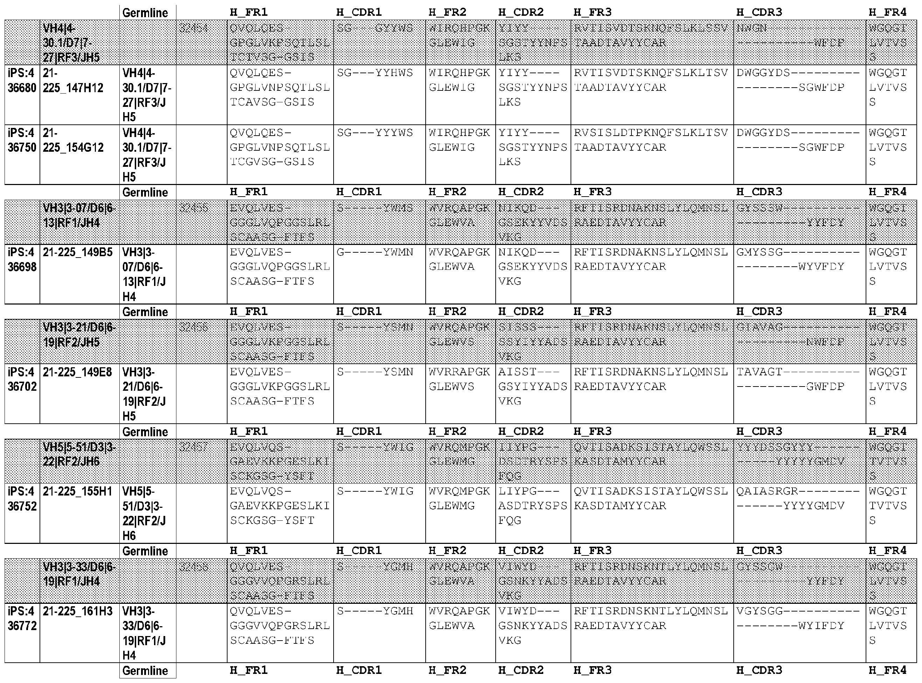

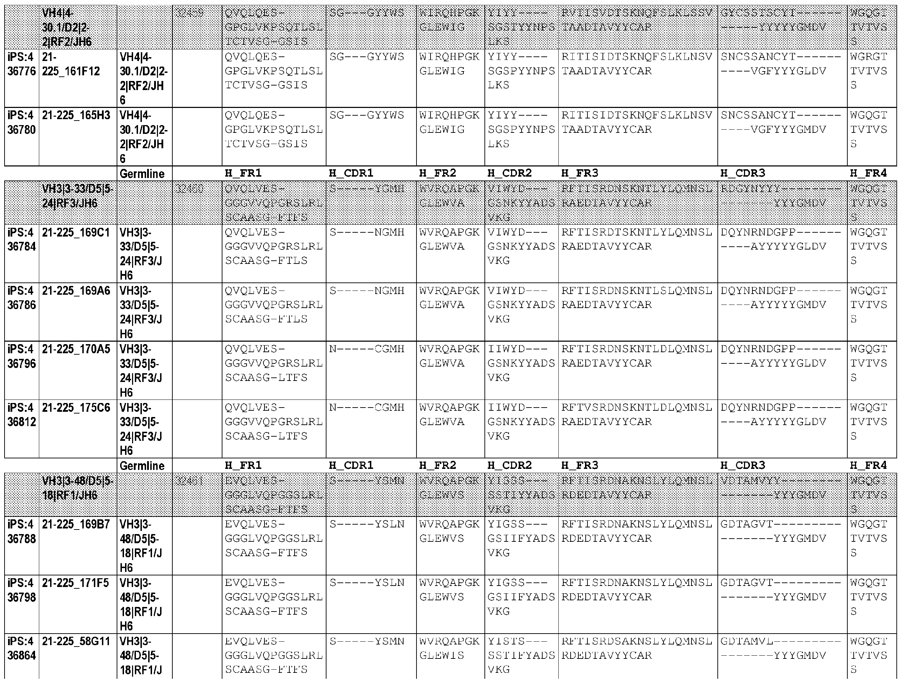

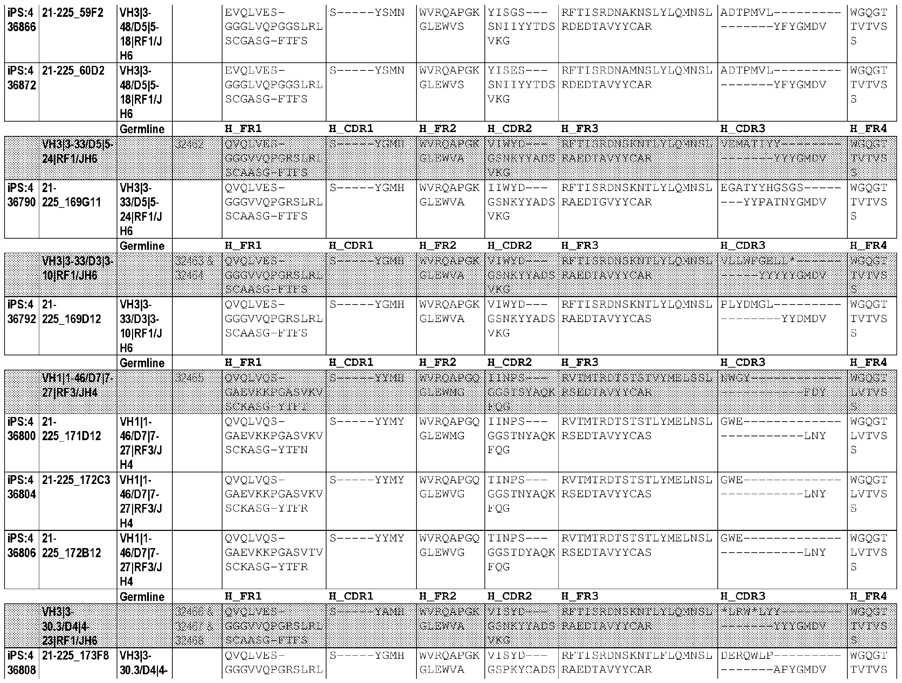

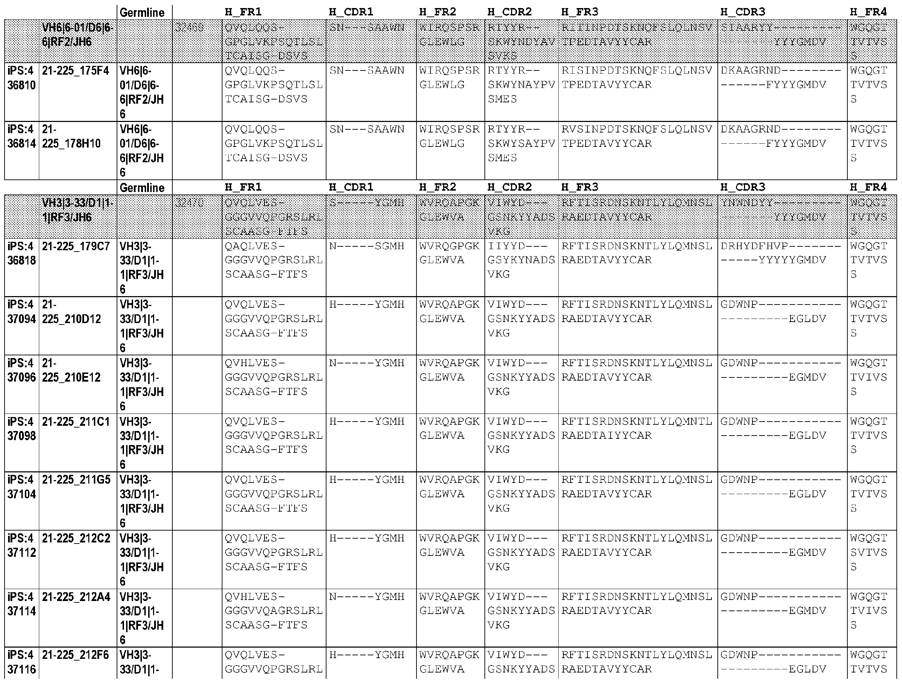

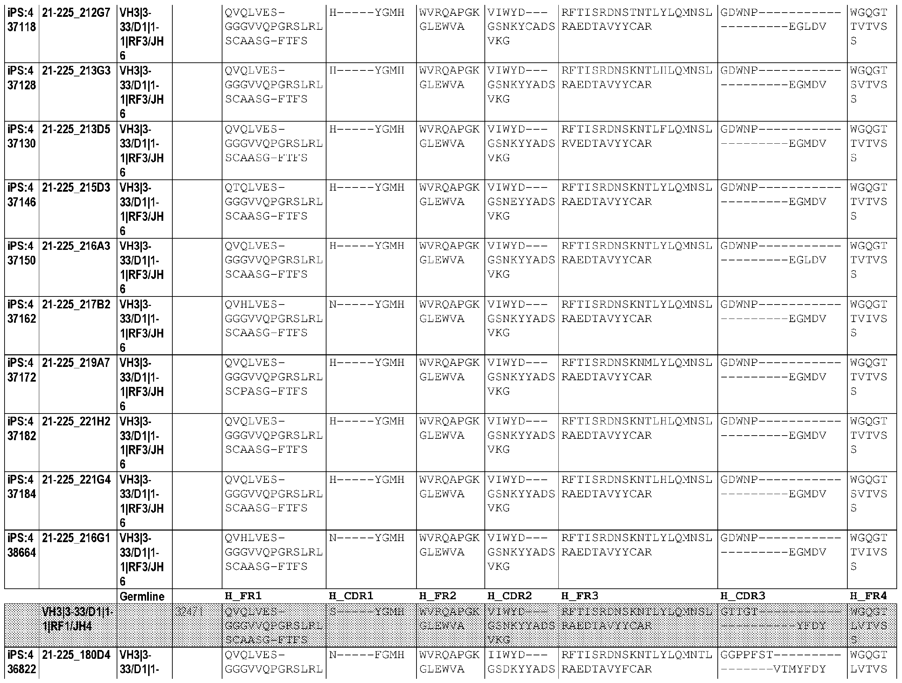

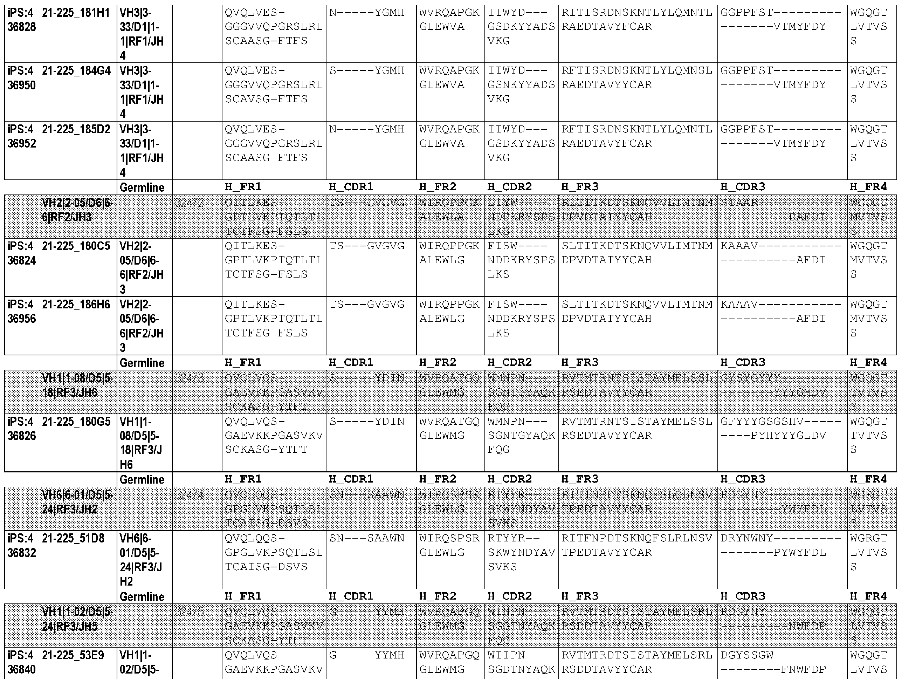

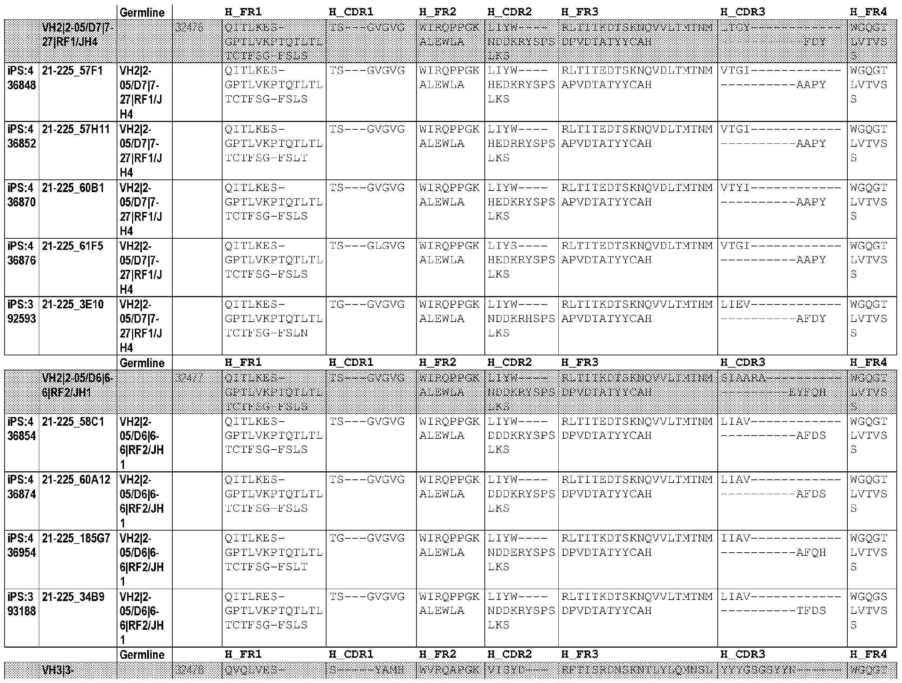

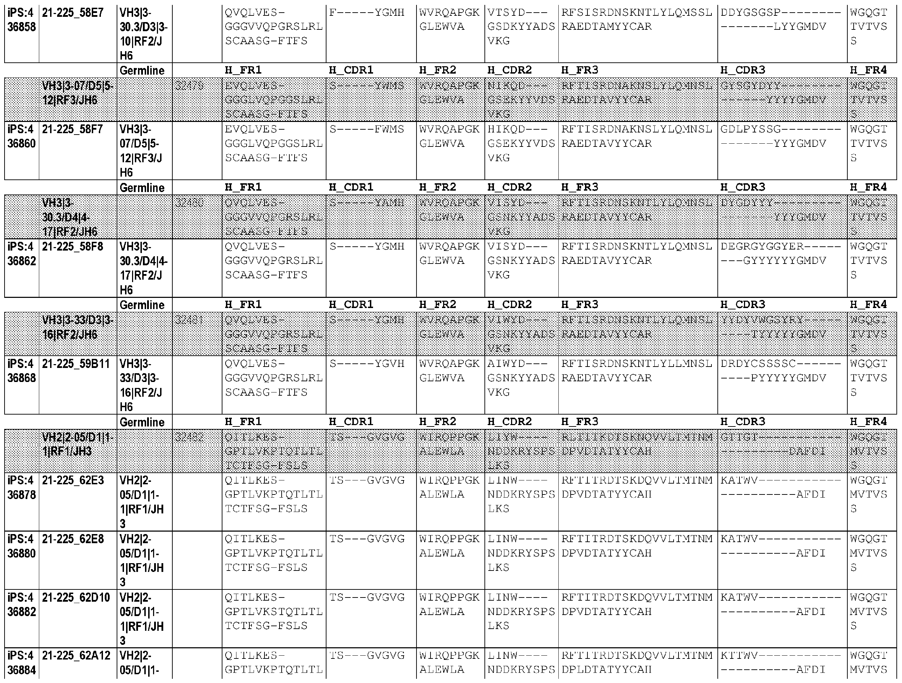

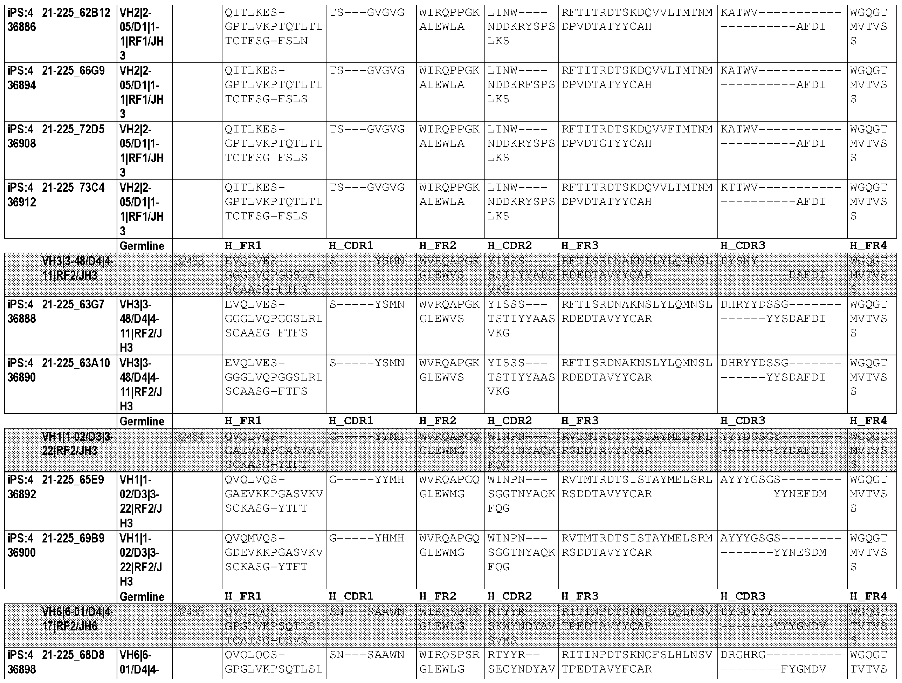

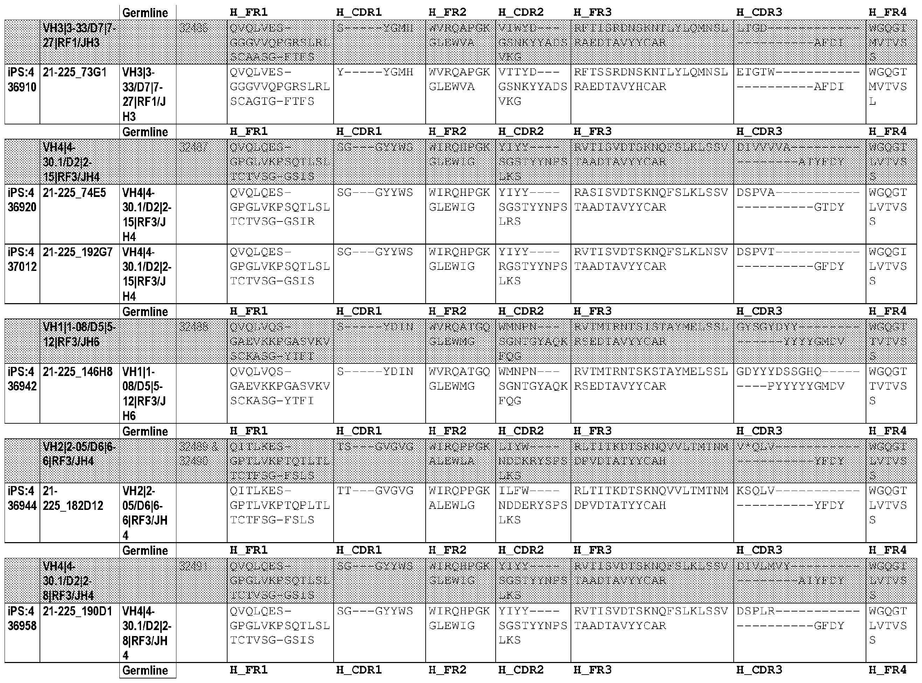

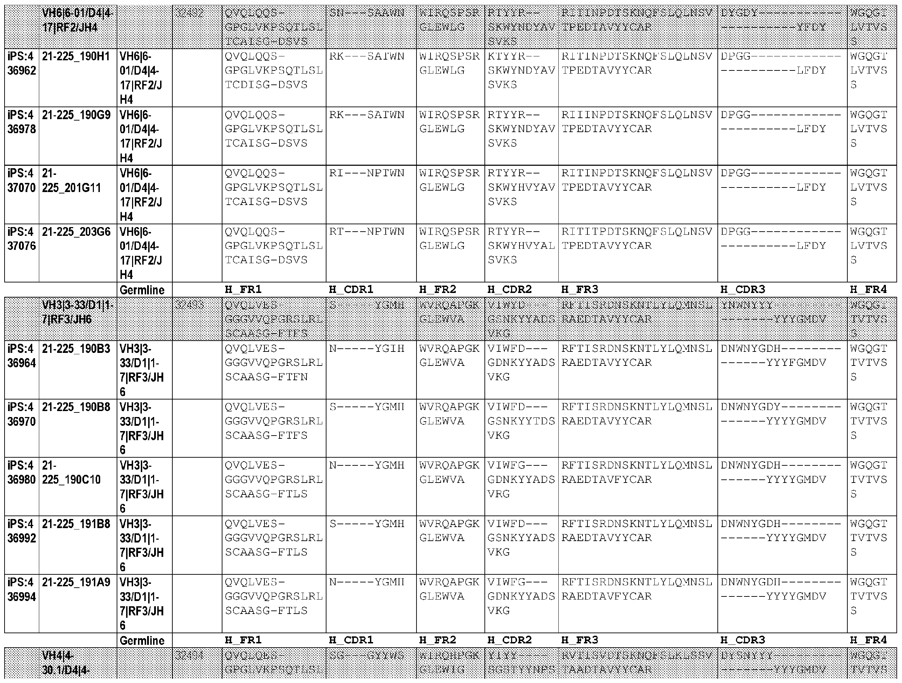

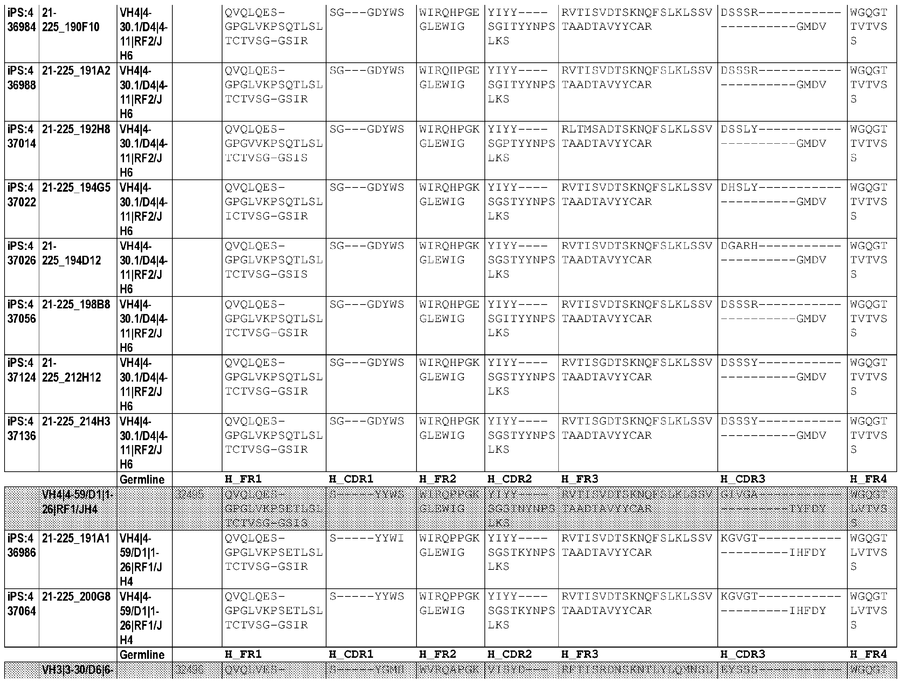

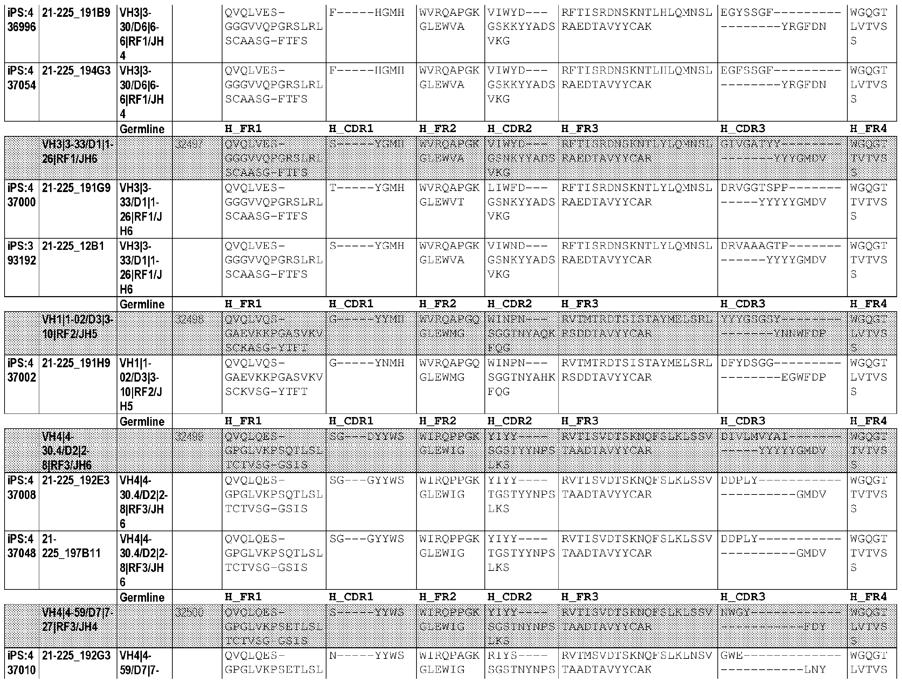

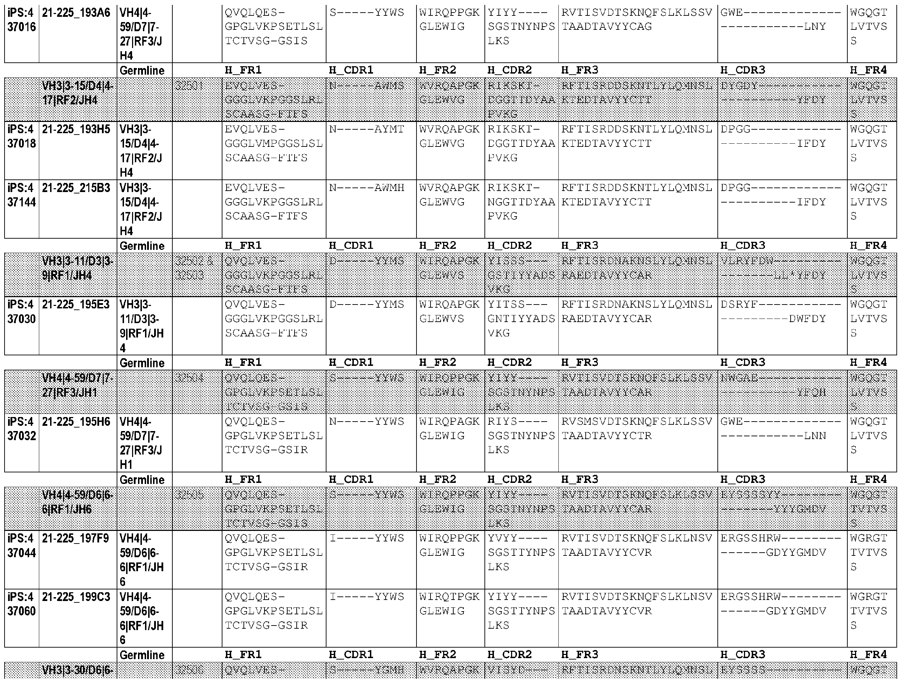

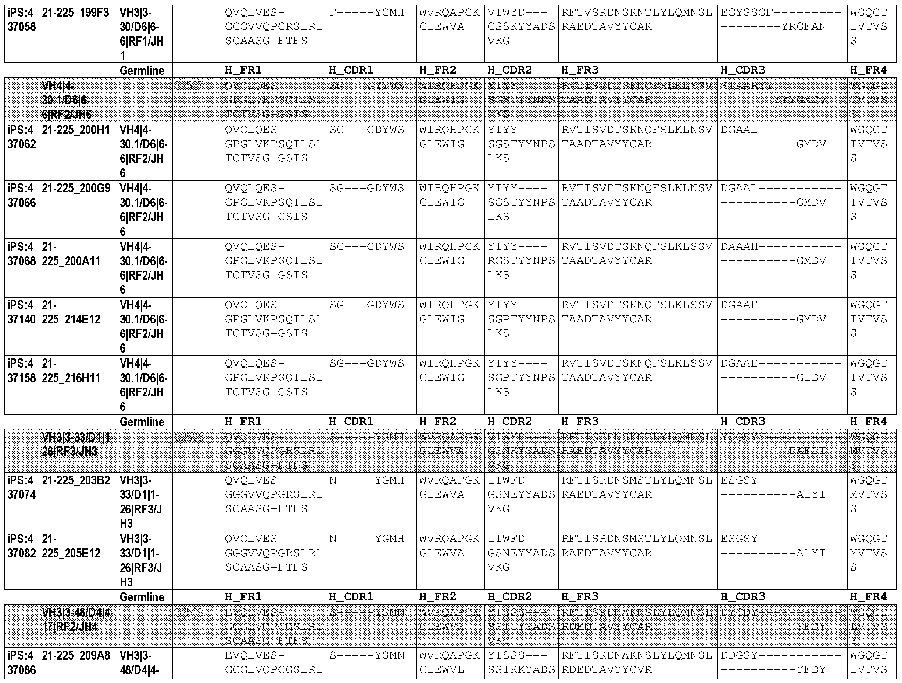

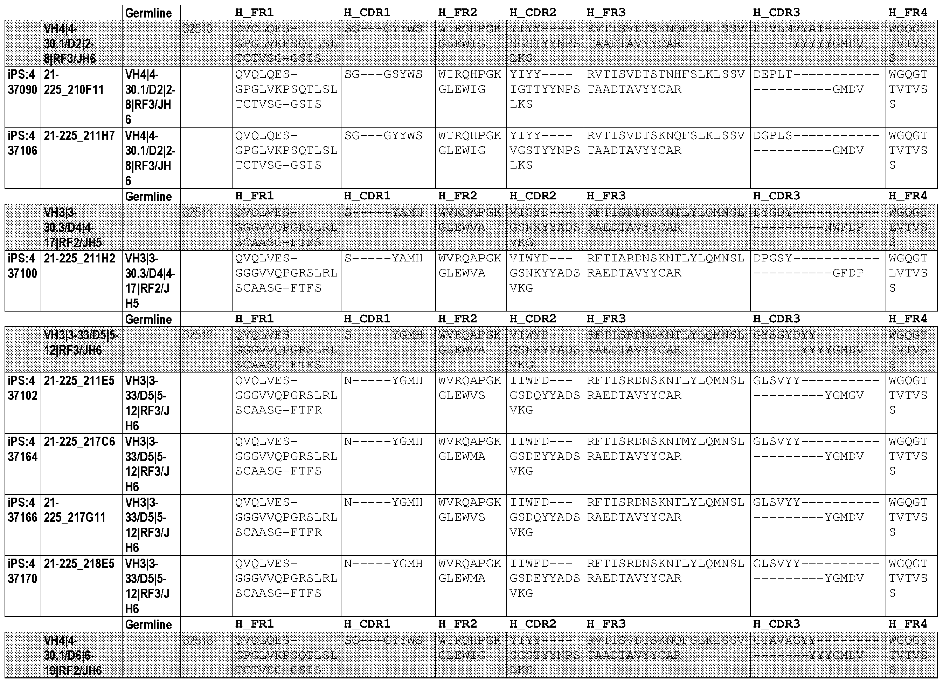

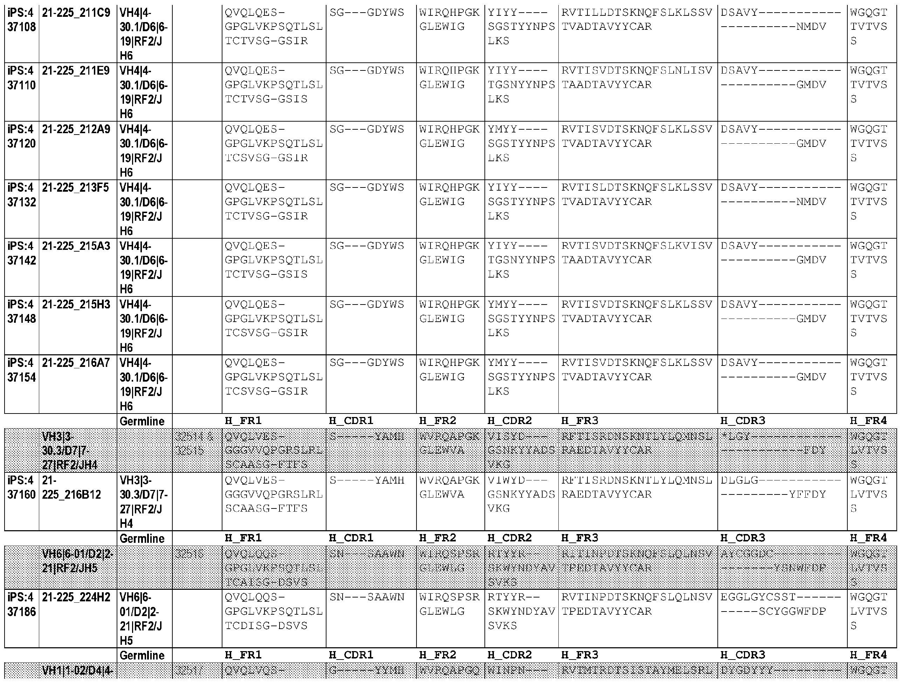

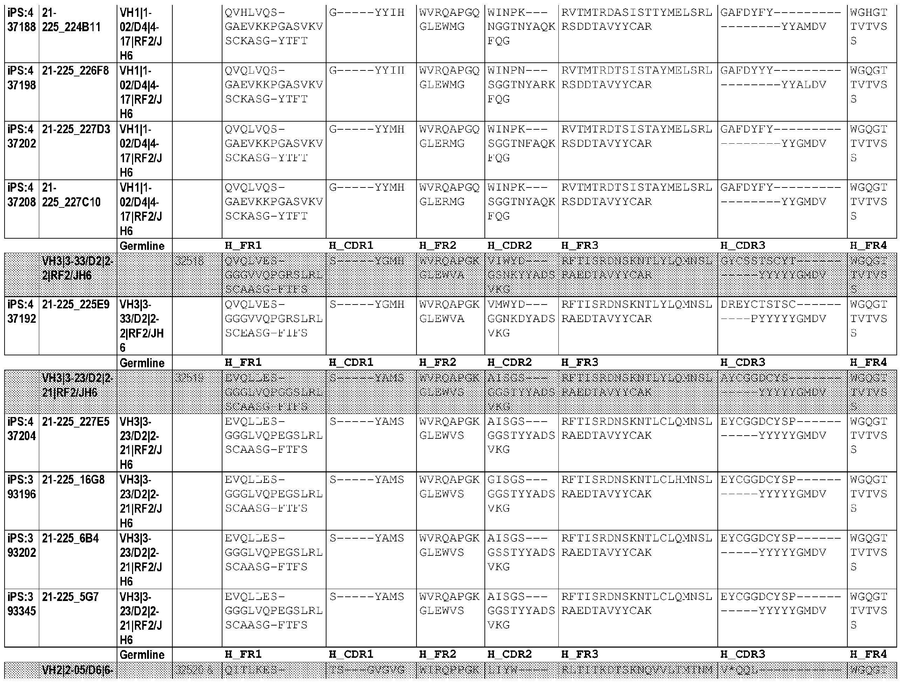

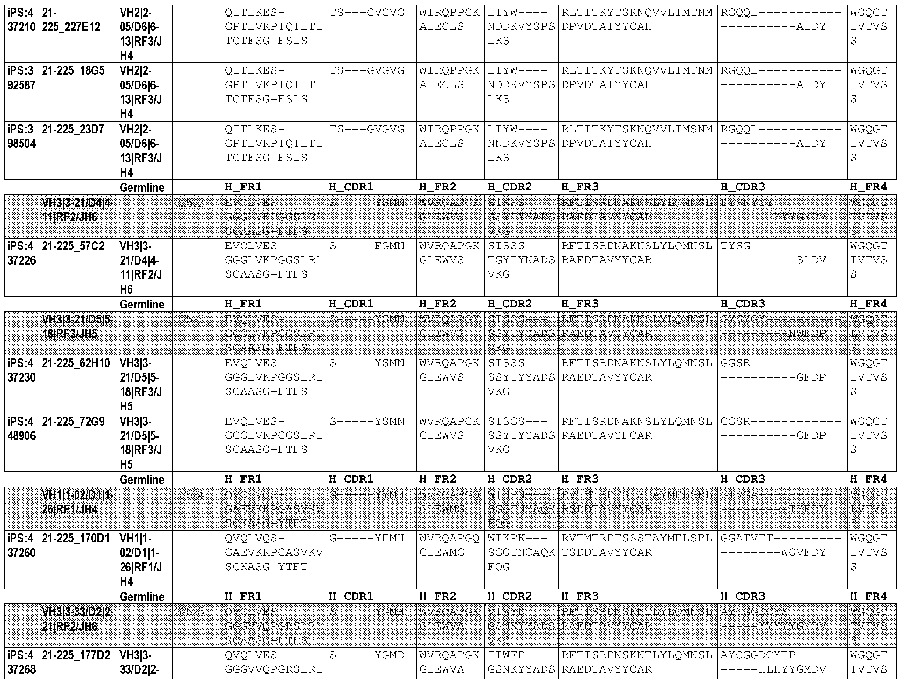

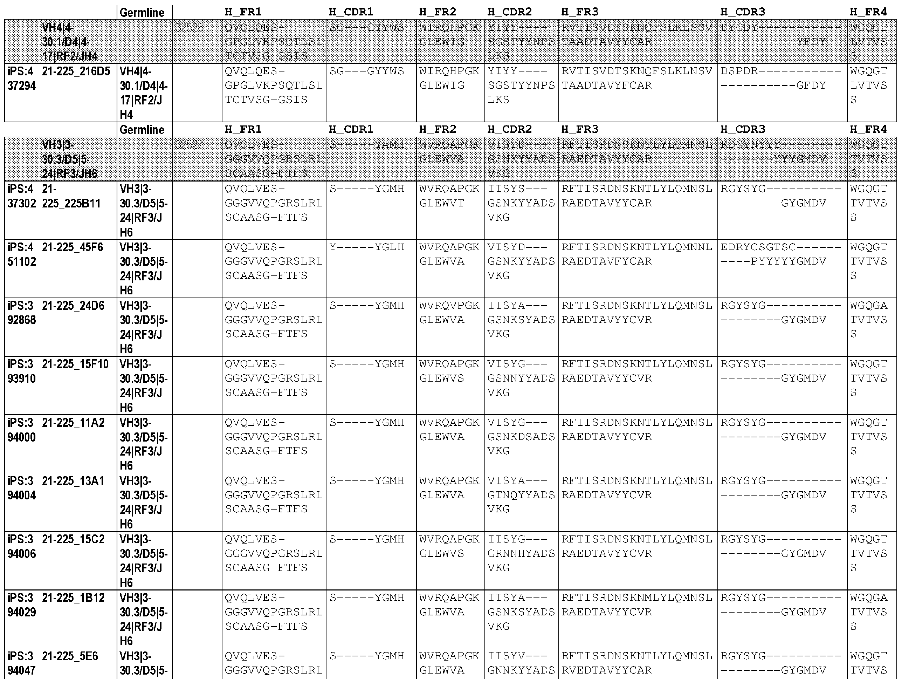

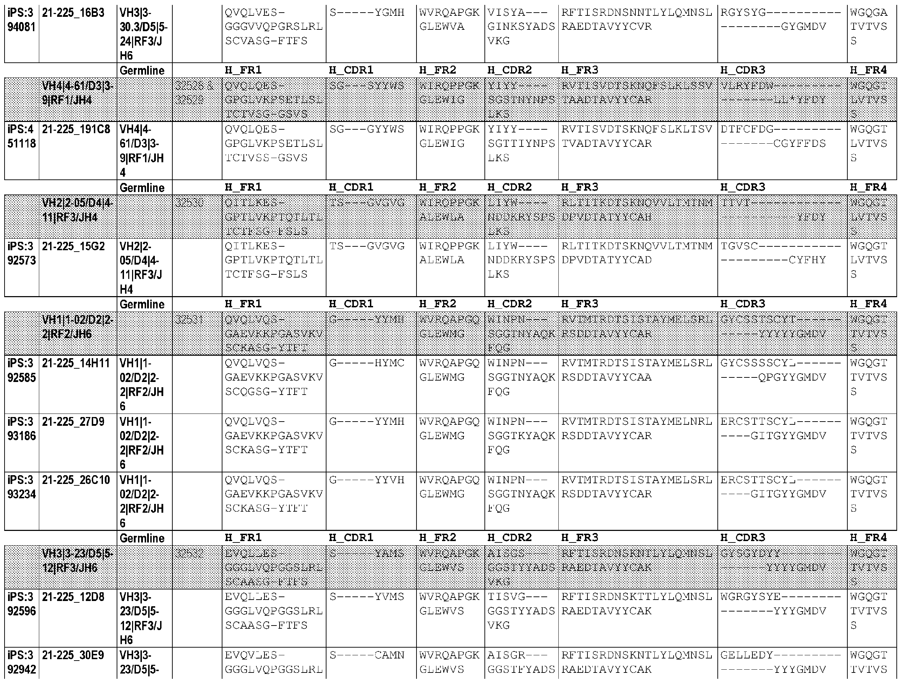

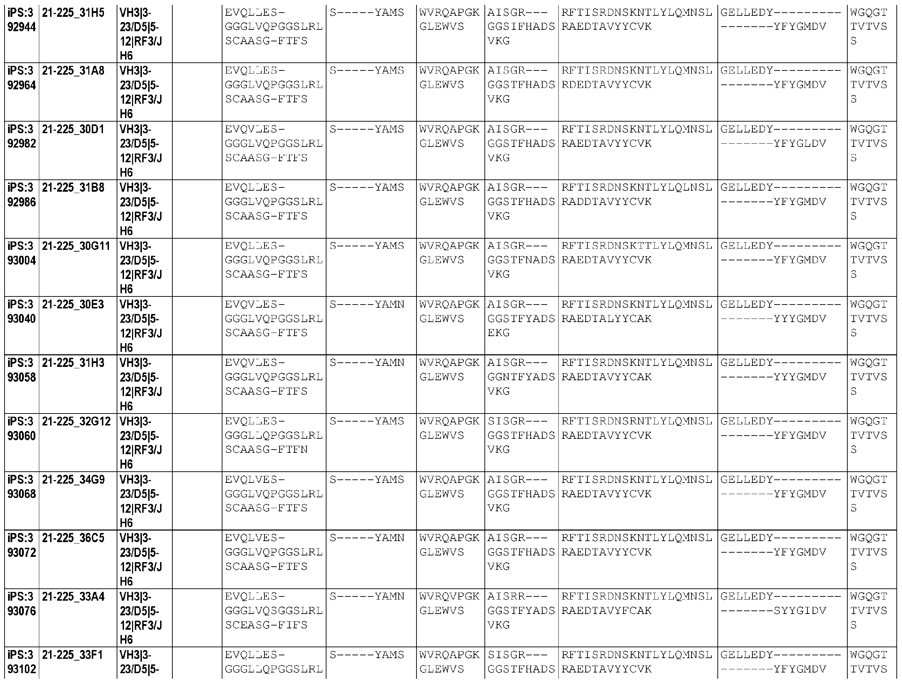

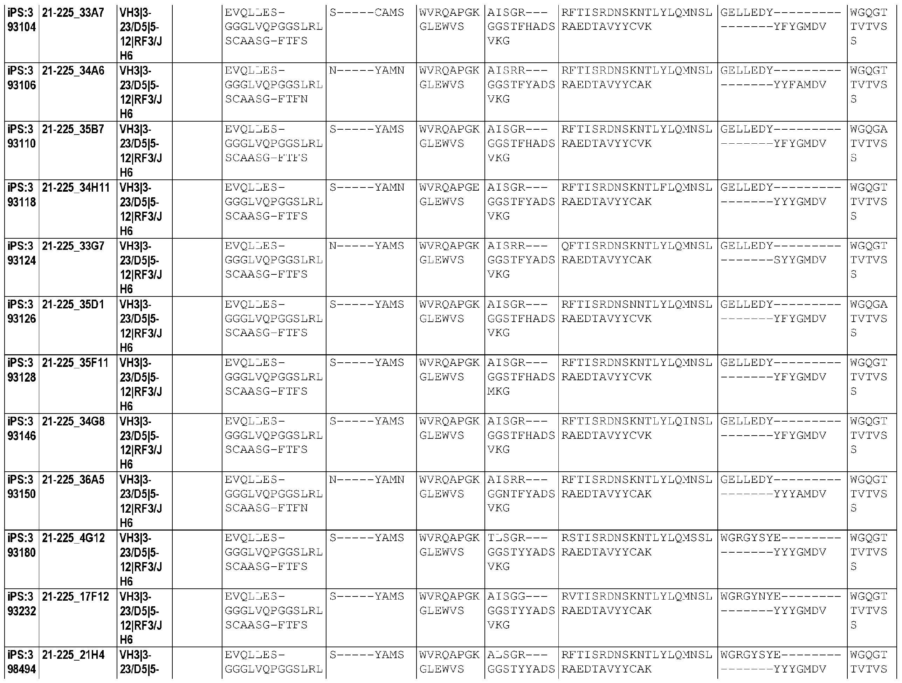

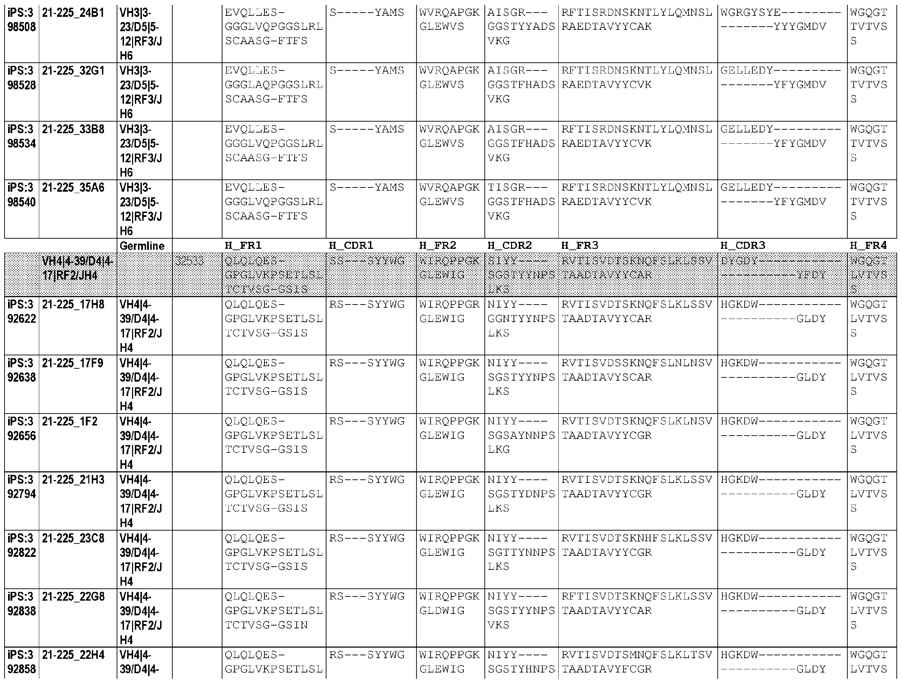

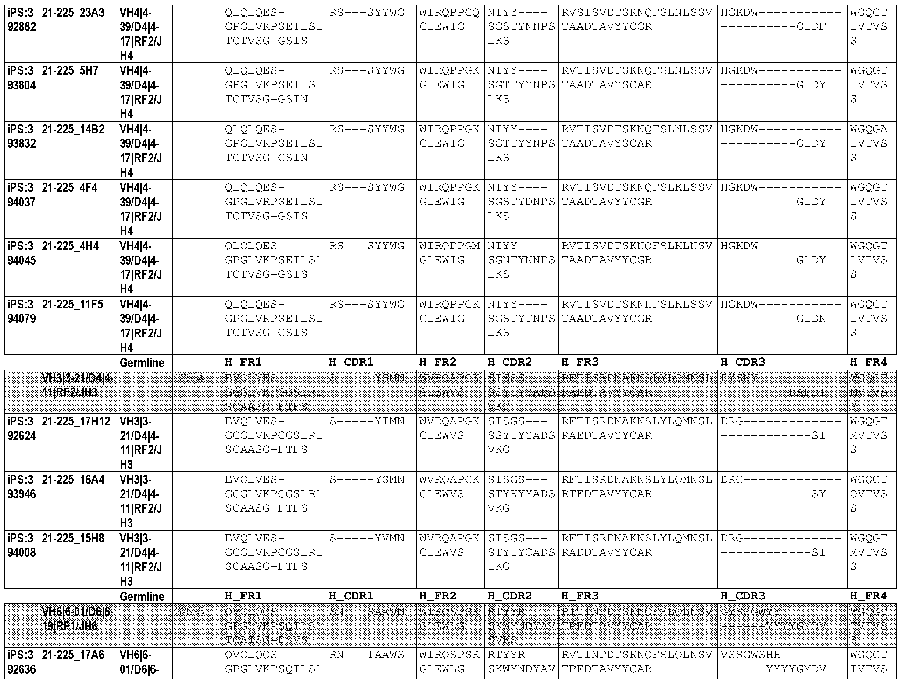

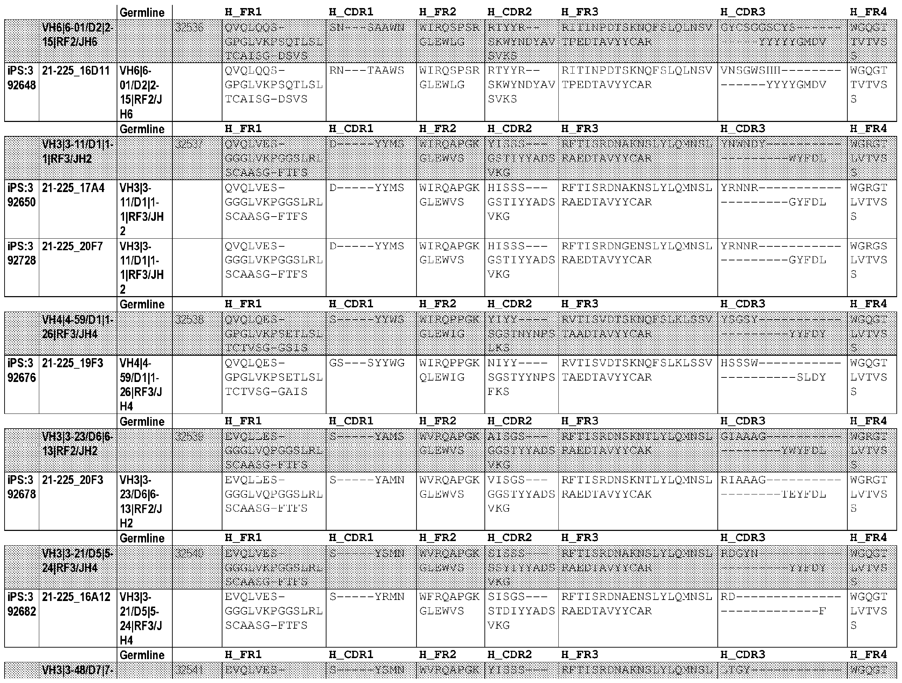

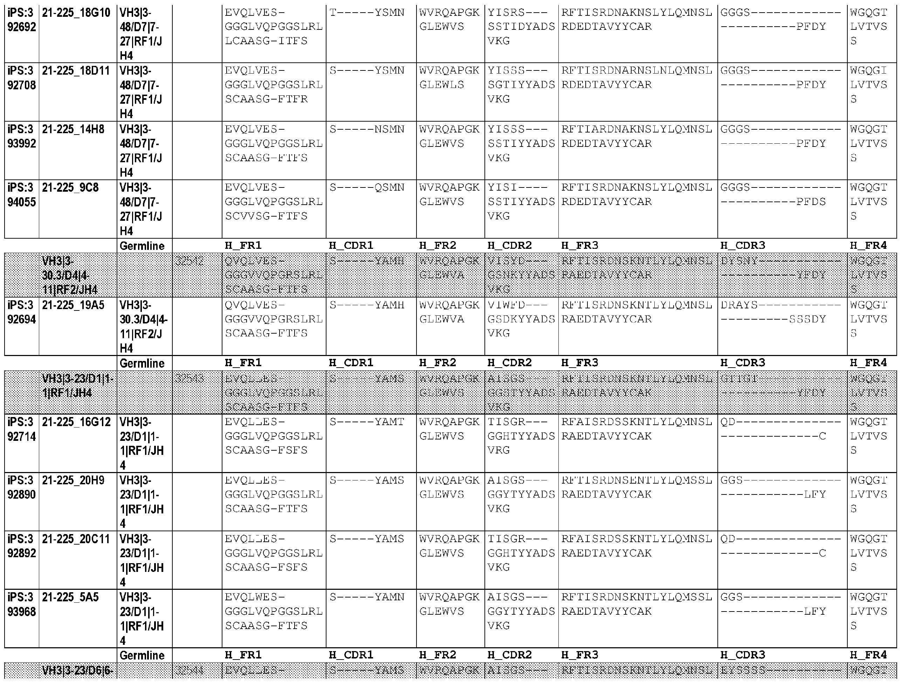

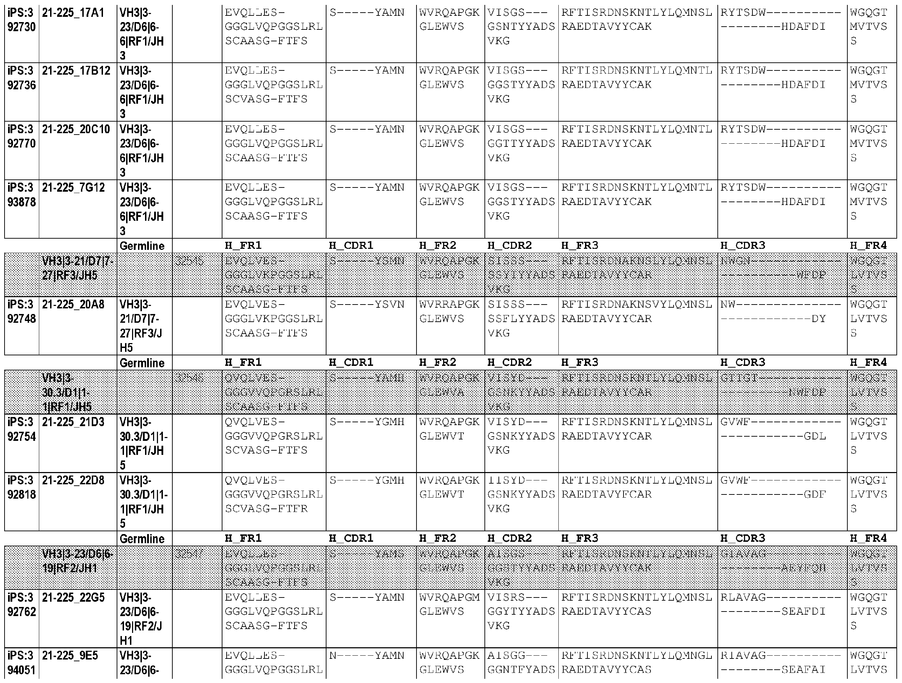

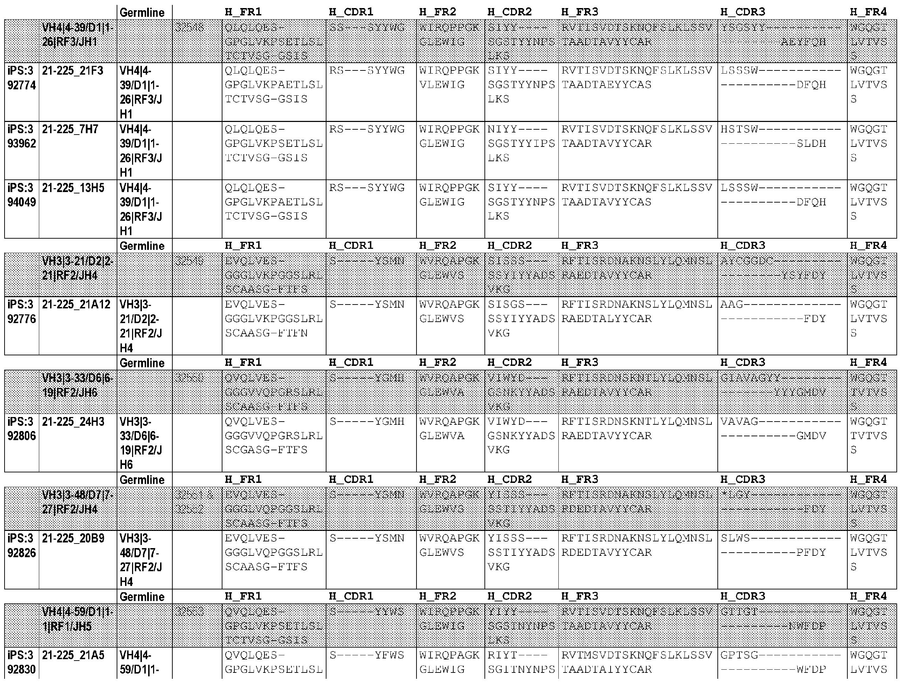

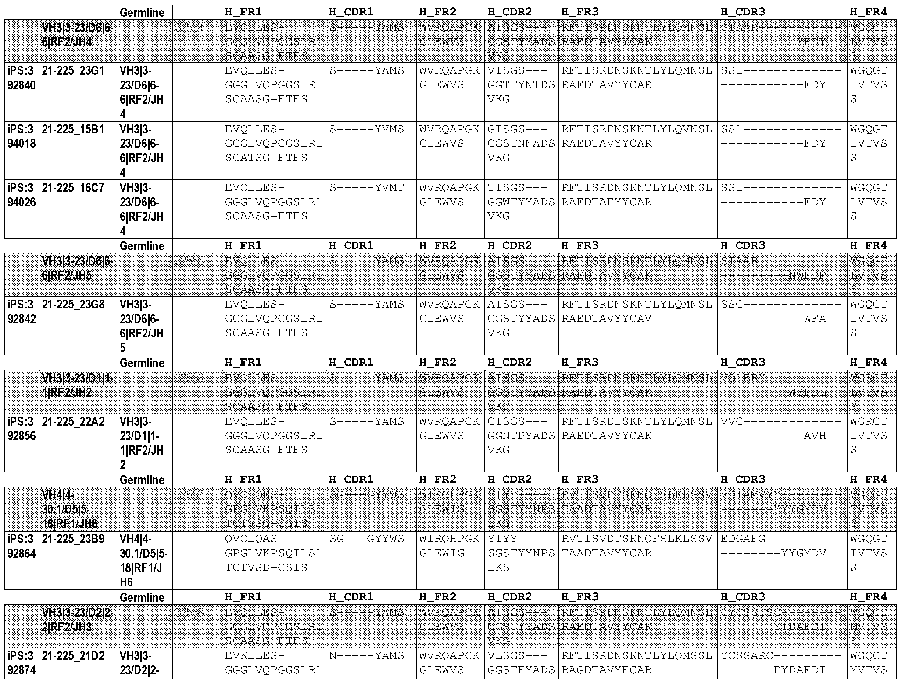

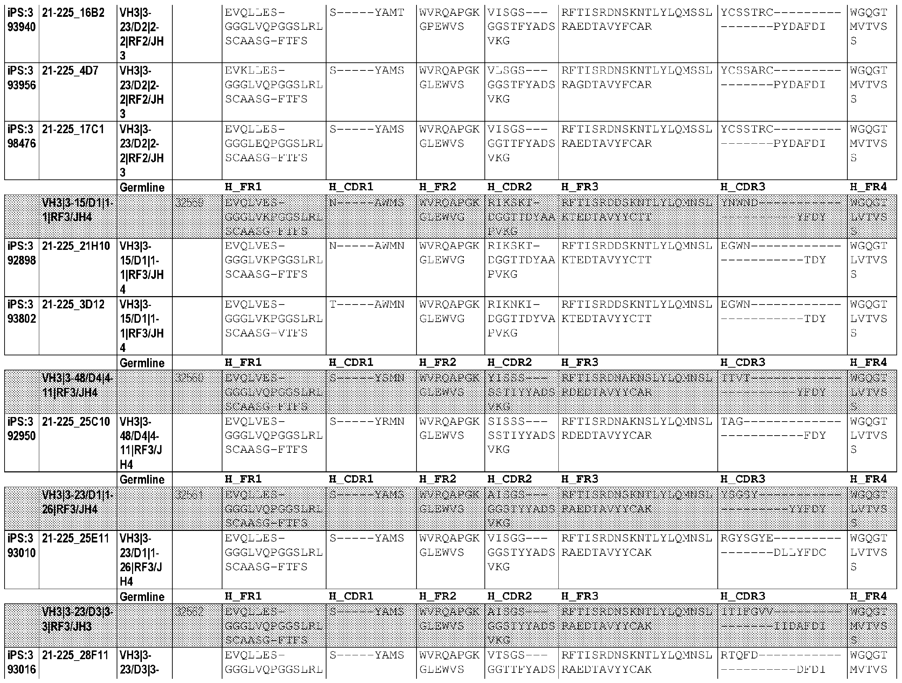

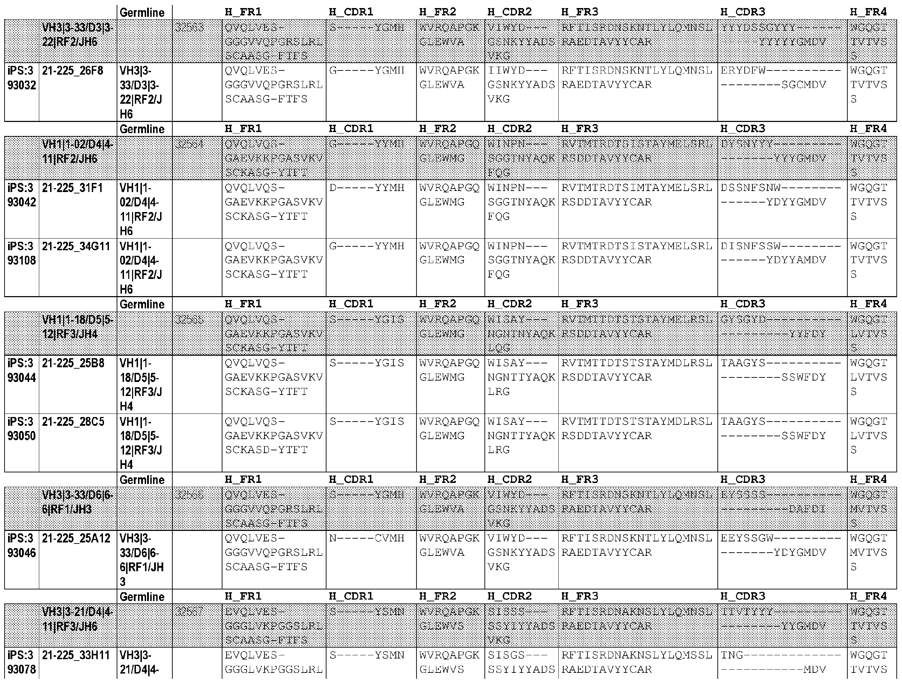

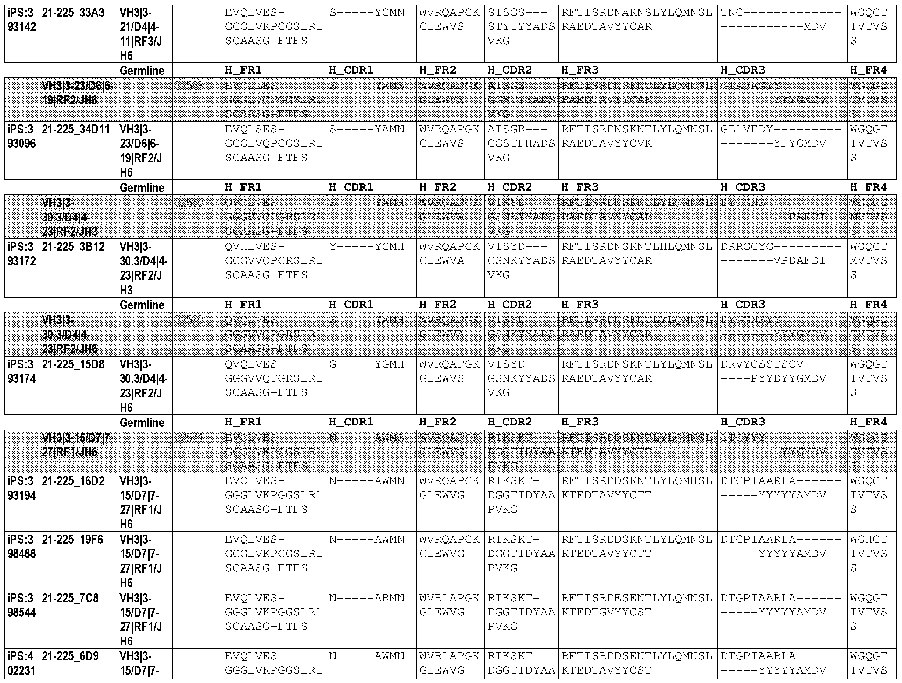

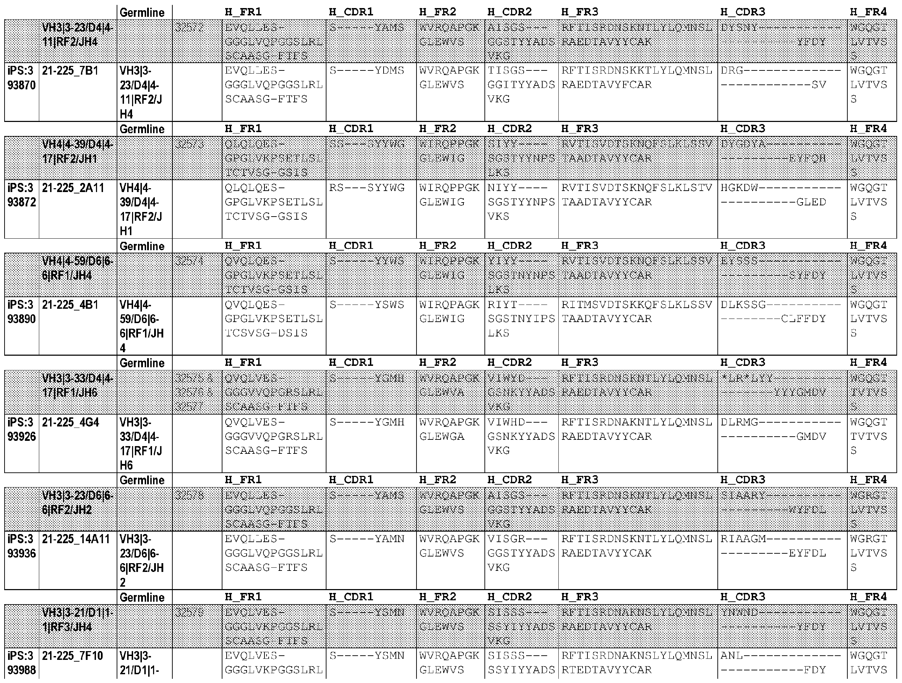

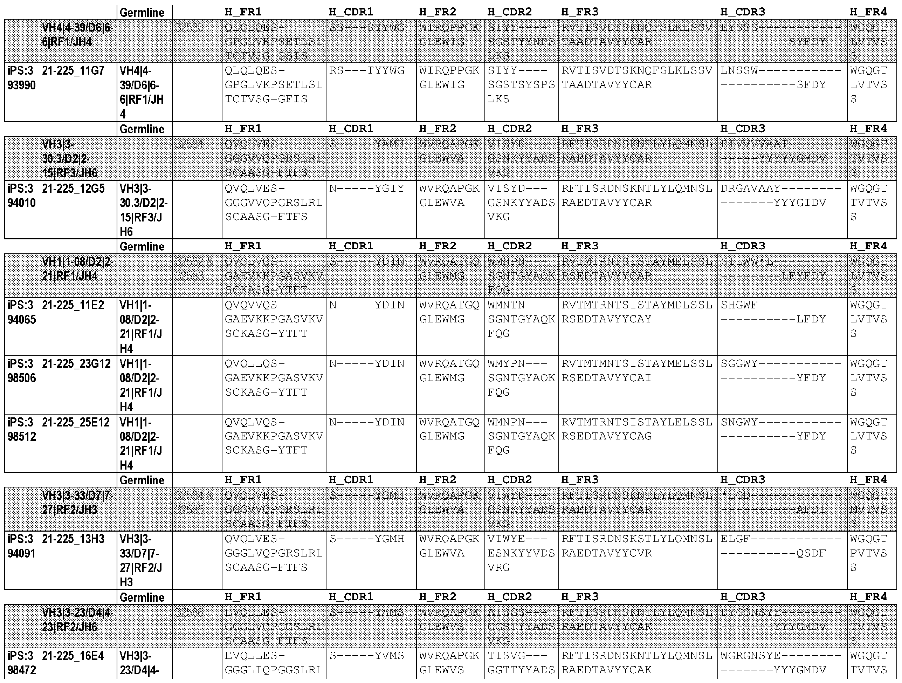

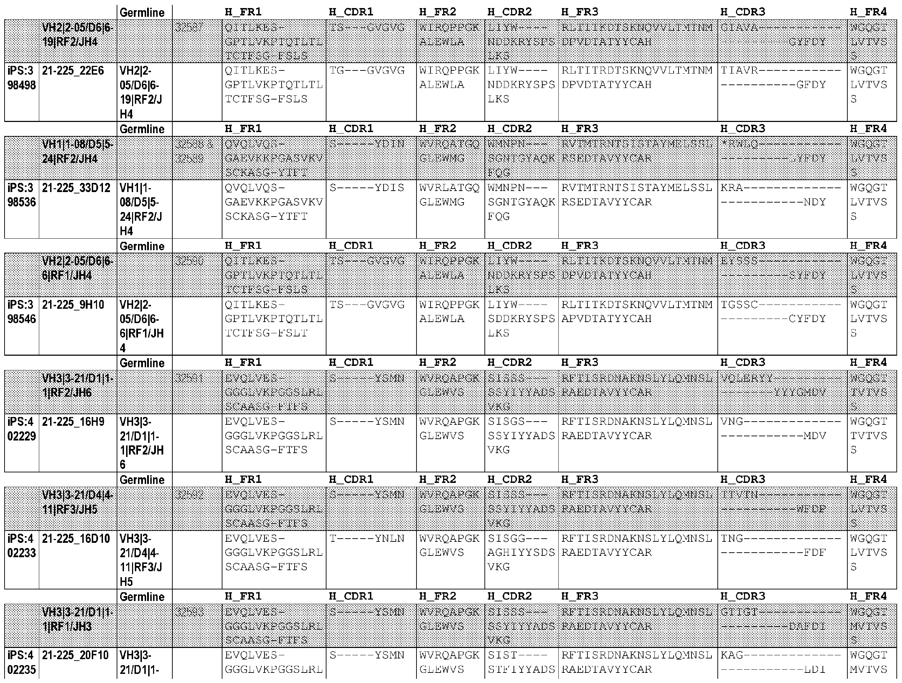

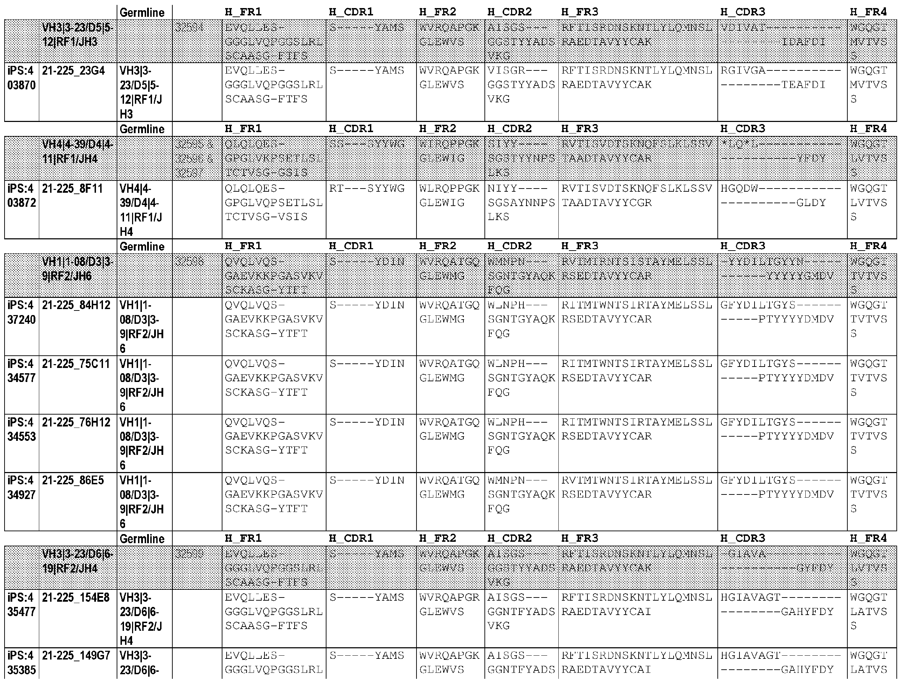

**Figure 51.** A table presenting a protein alignment of light and heavy variable regions for certain antigen binding proteins (Table 4). An asterisk "*" denotes a stop codon. Sequences containing a stop codon are represented as distinct sequences in the Sequence Listing, however, these sequences are related. Generally speaking, however, the amino acid sequences of the light and heavy chain variable domains in the protein alignment presented in Table 4 are wrapped due to space issues, and unless stated otherwise, like in the case of sequences with one or more stop codons, should be understood to be single amino acid sequences.

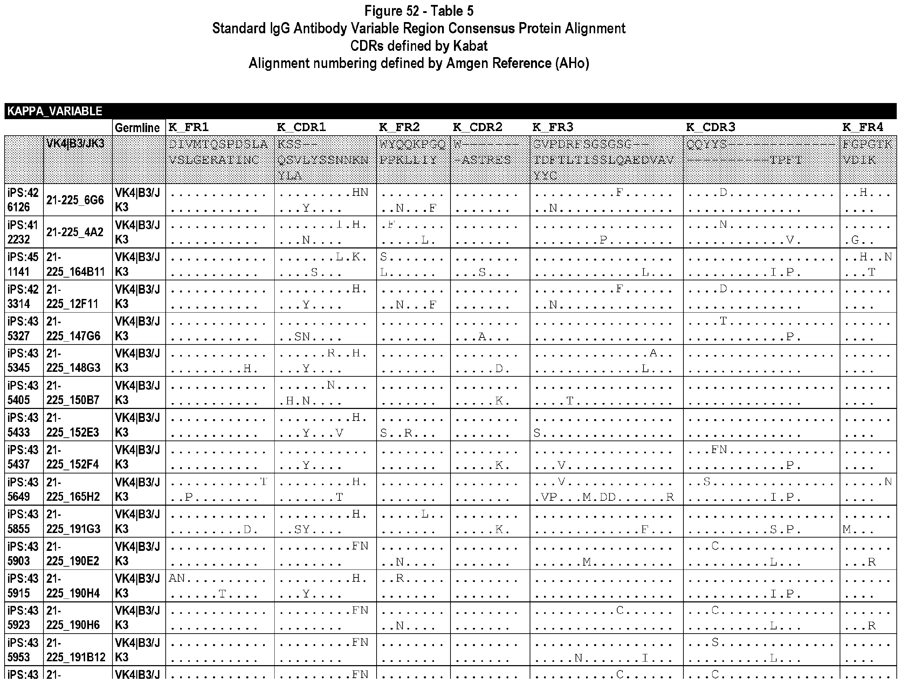

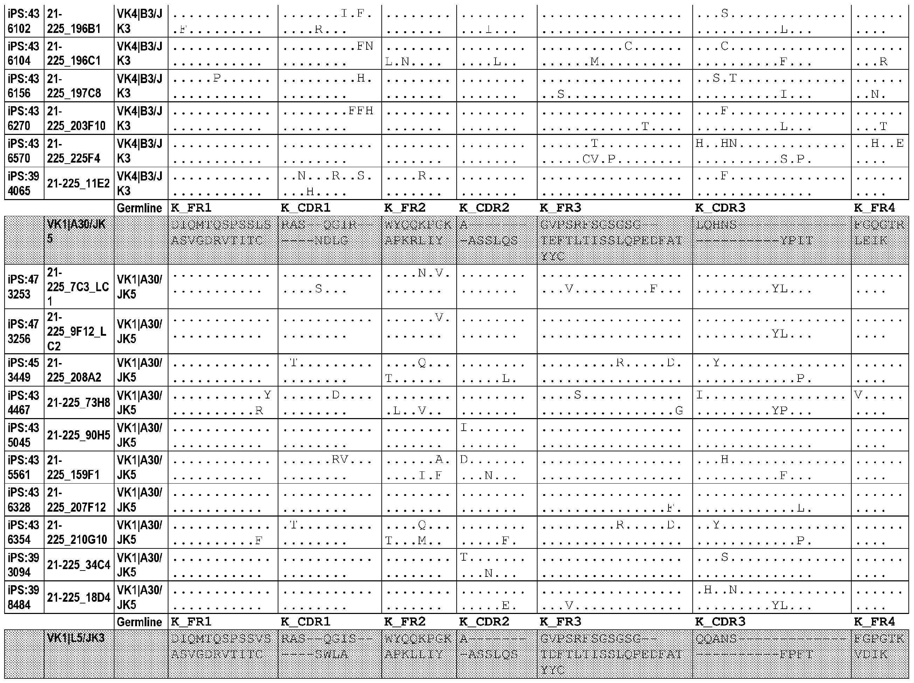

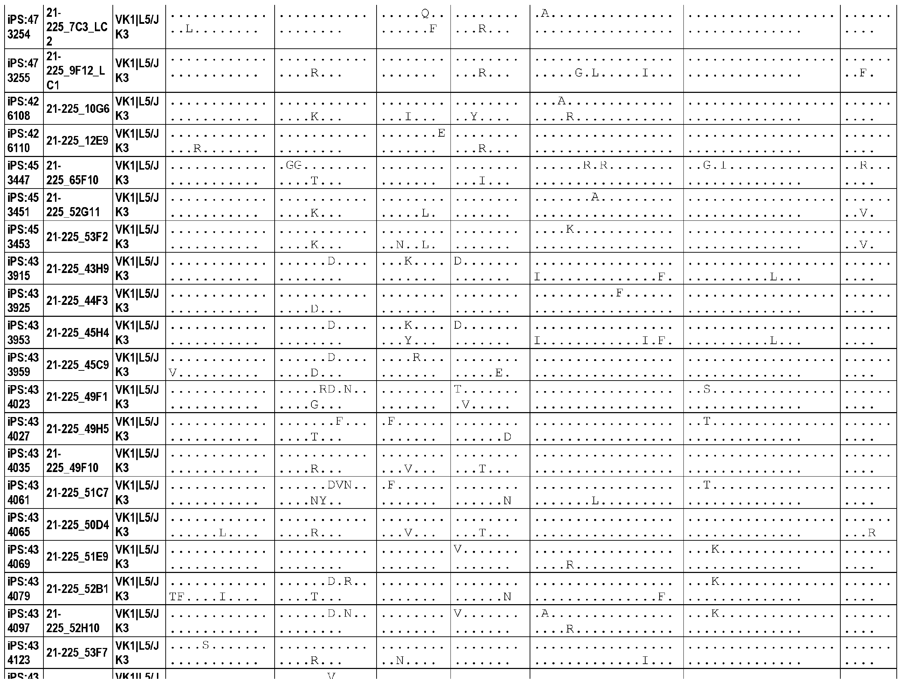

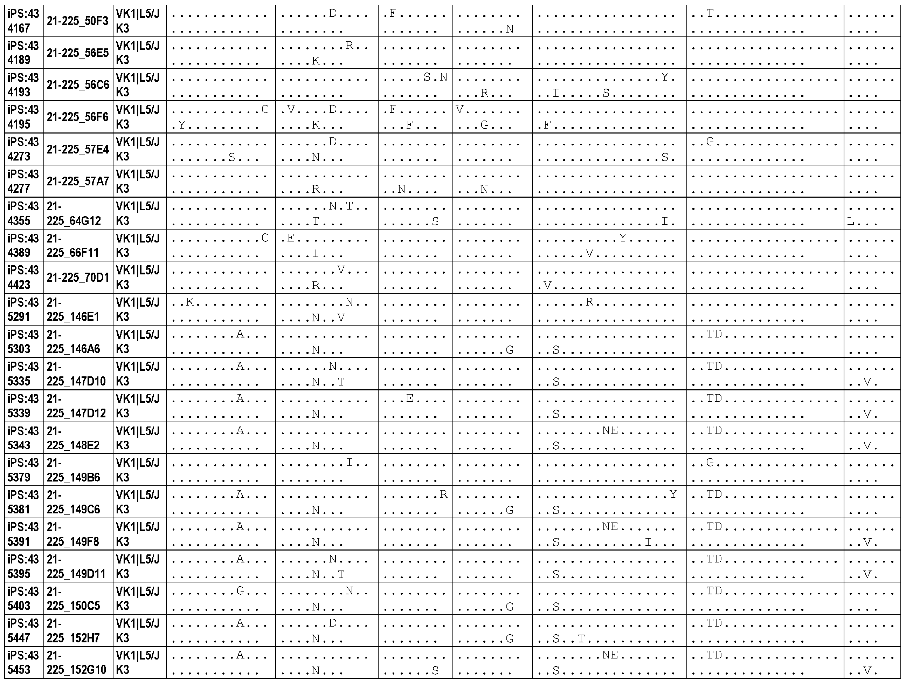

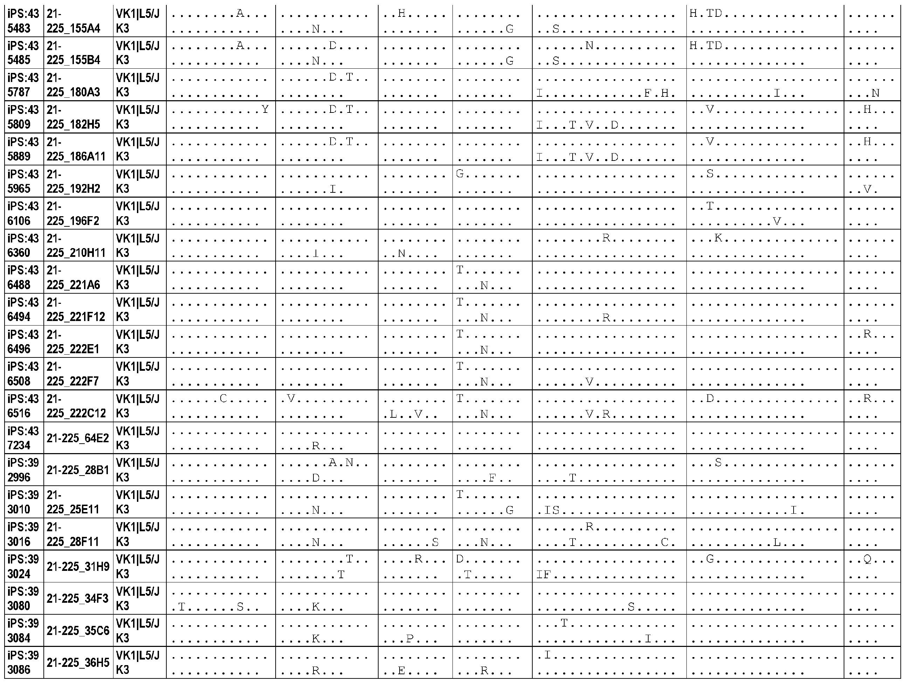

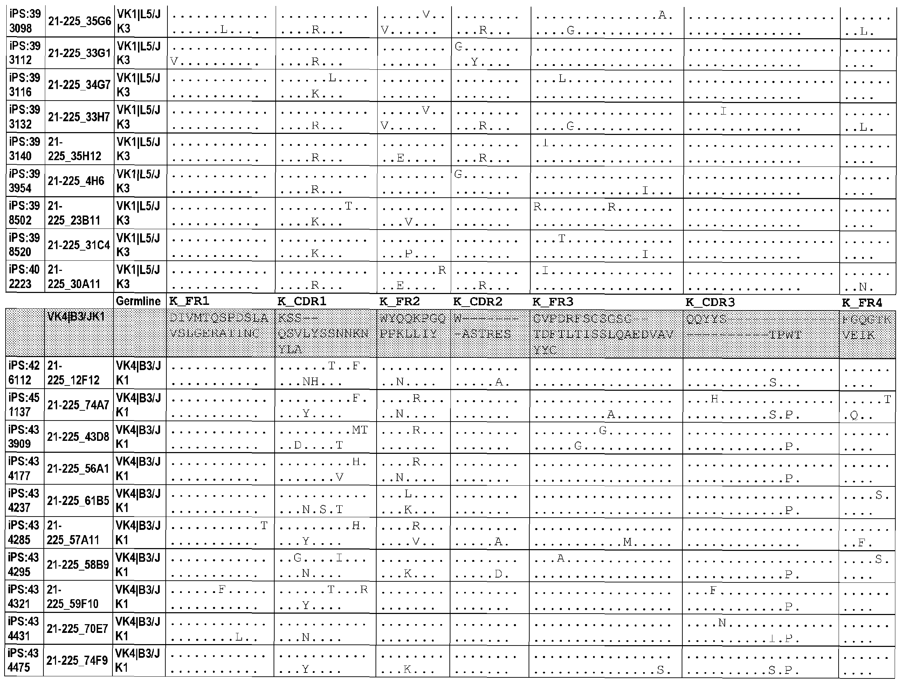

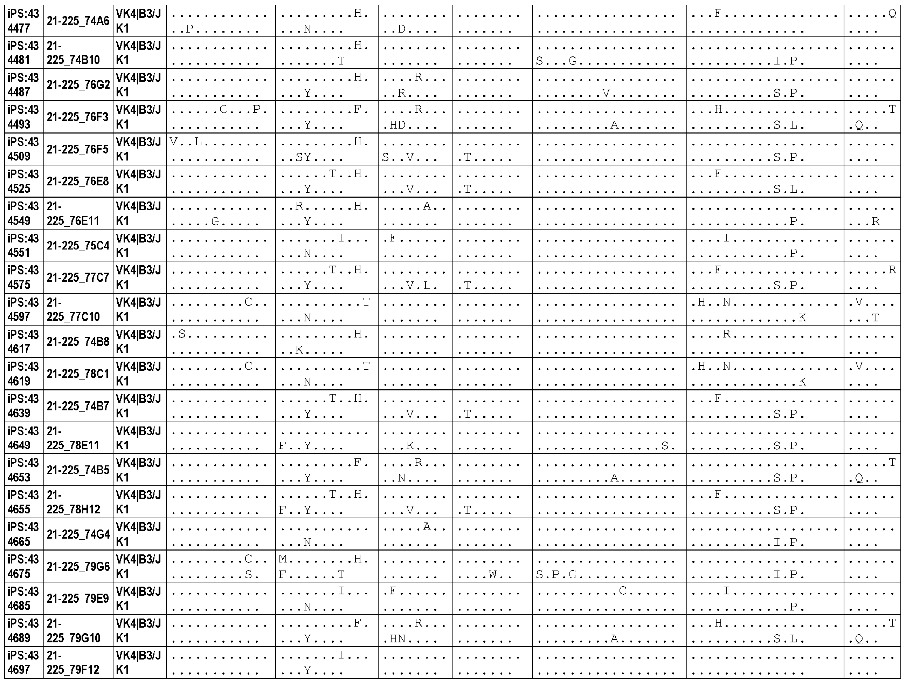

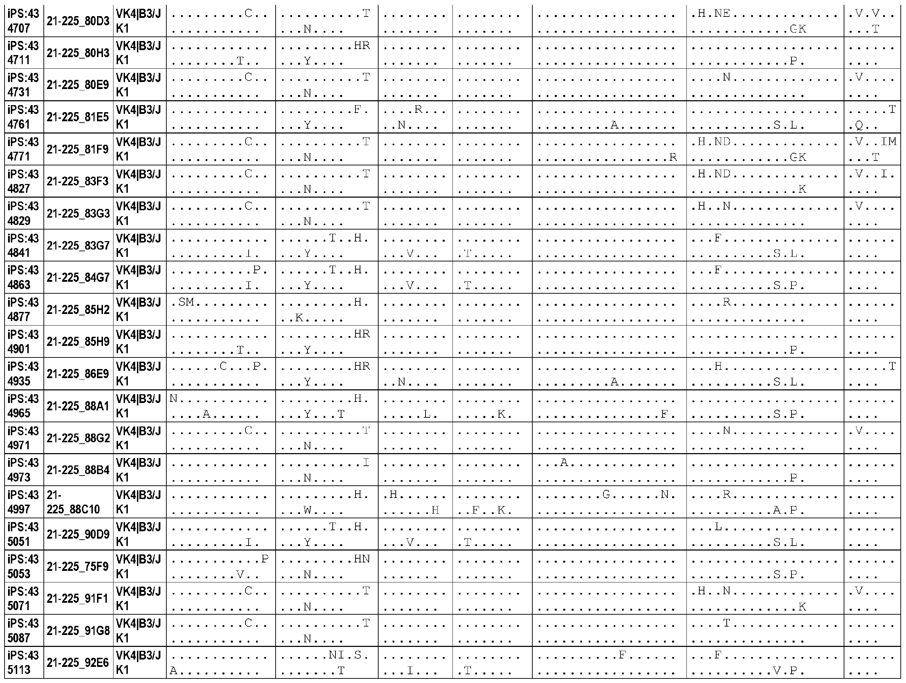

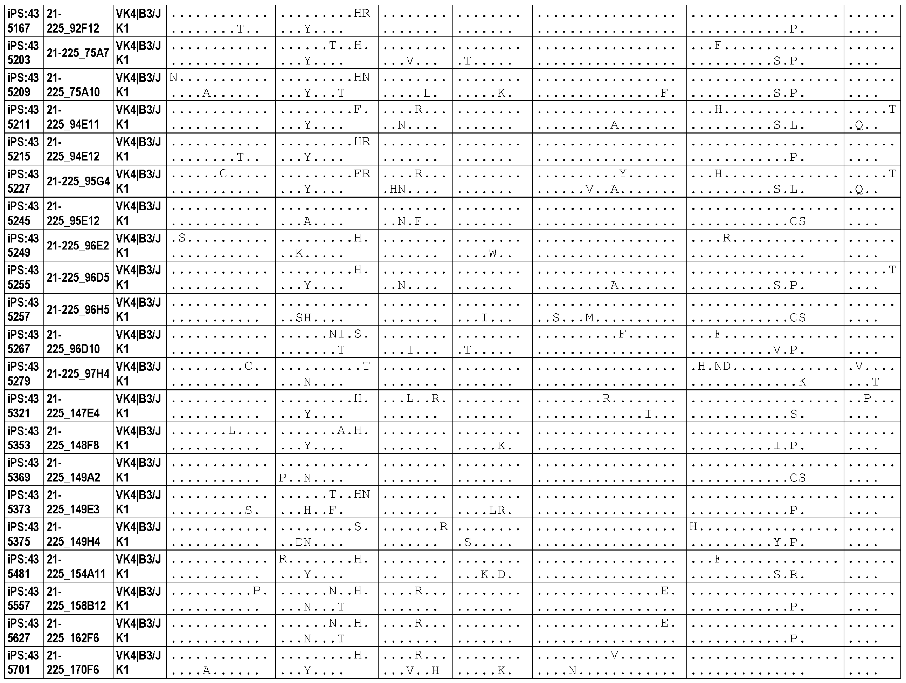

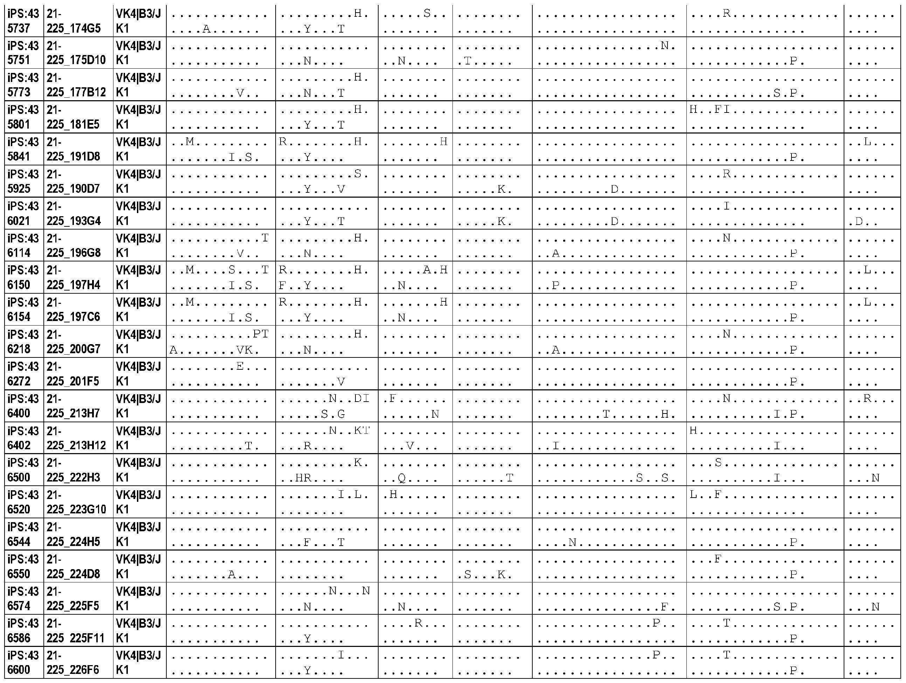

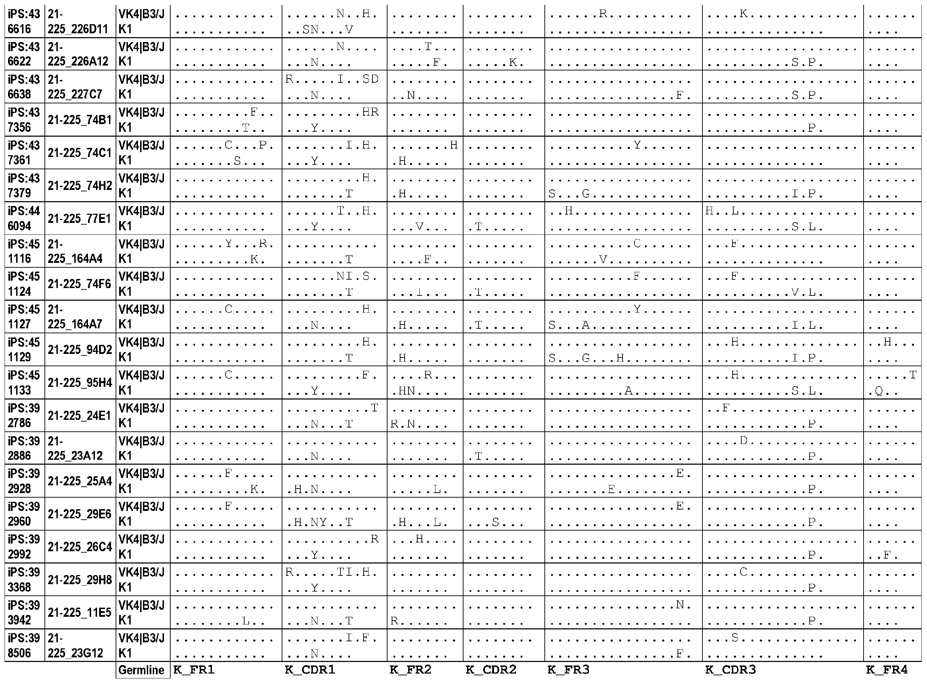

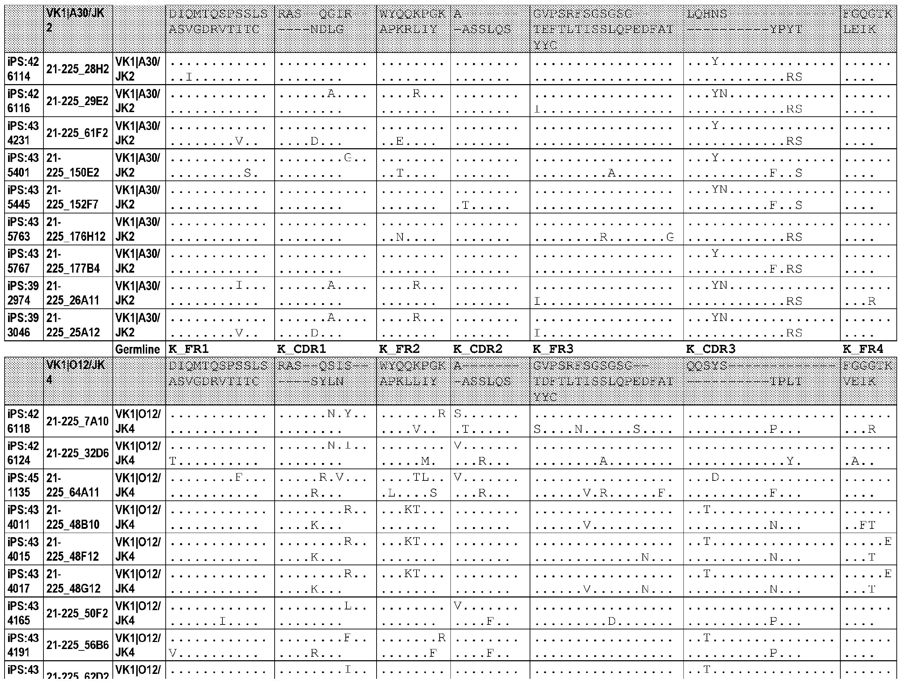

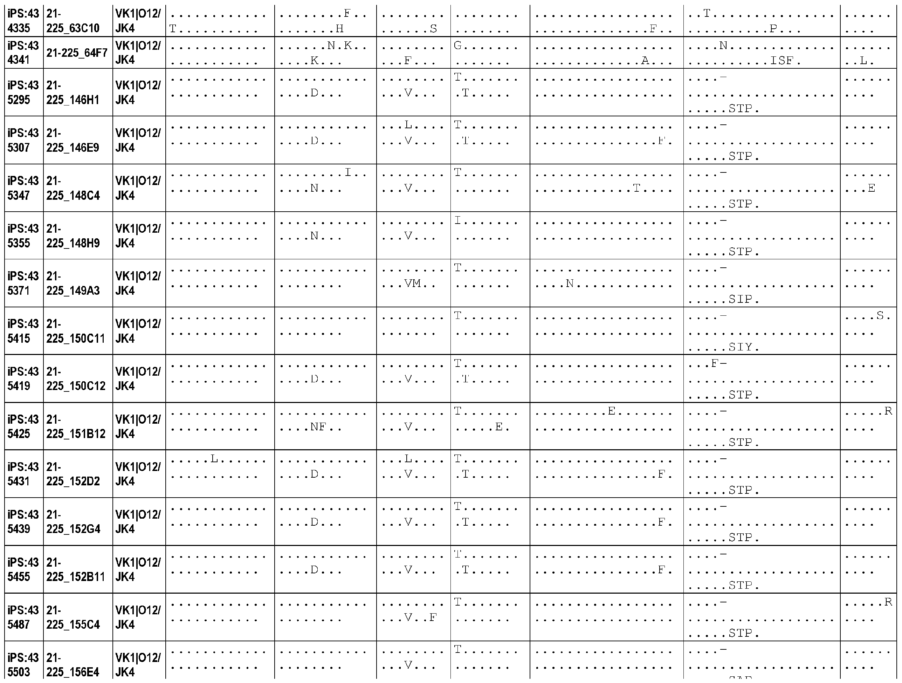

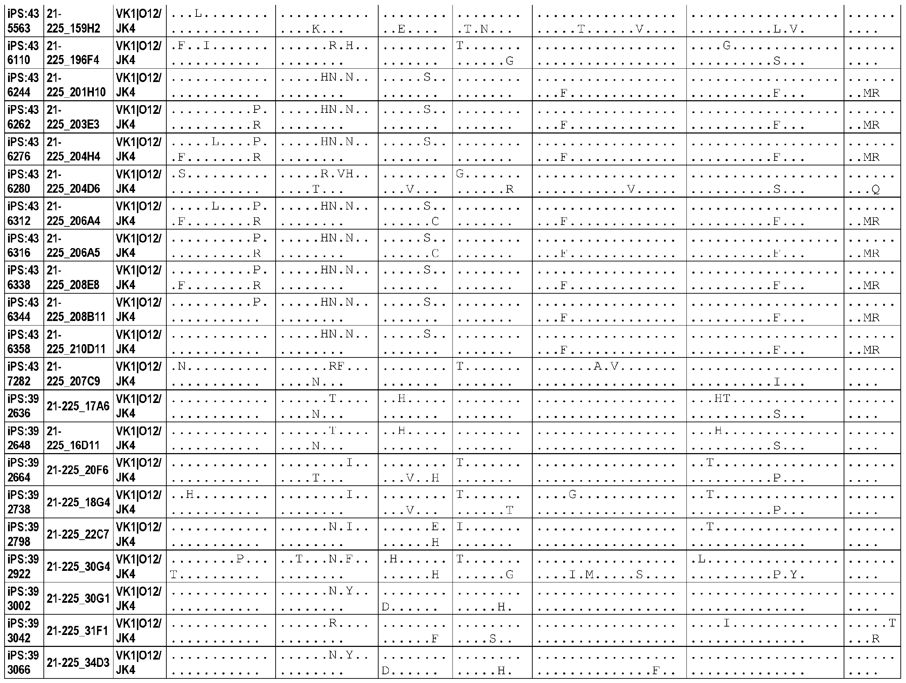

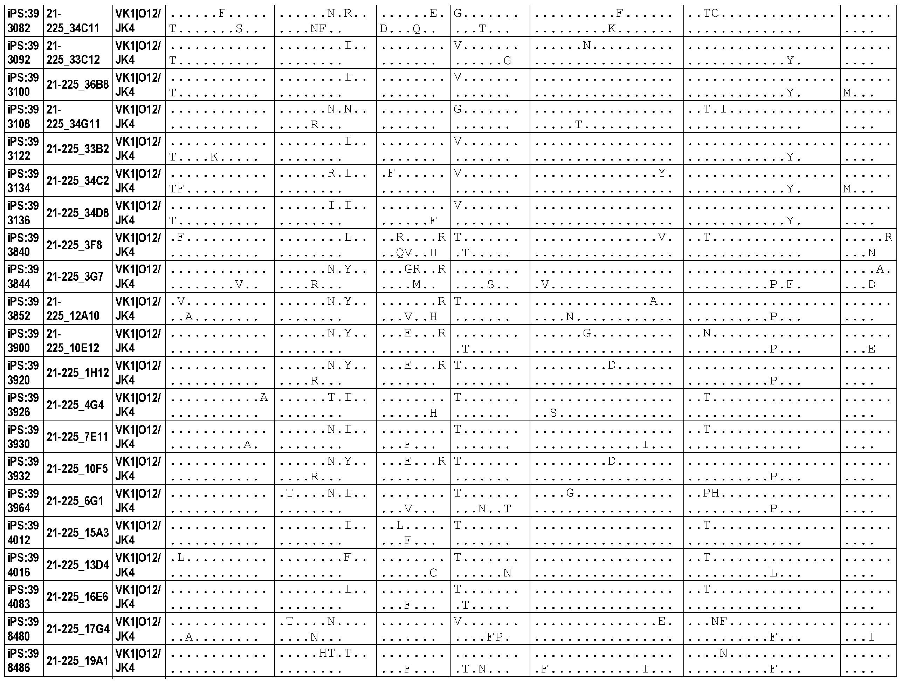

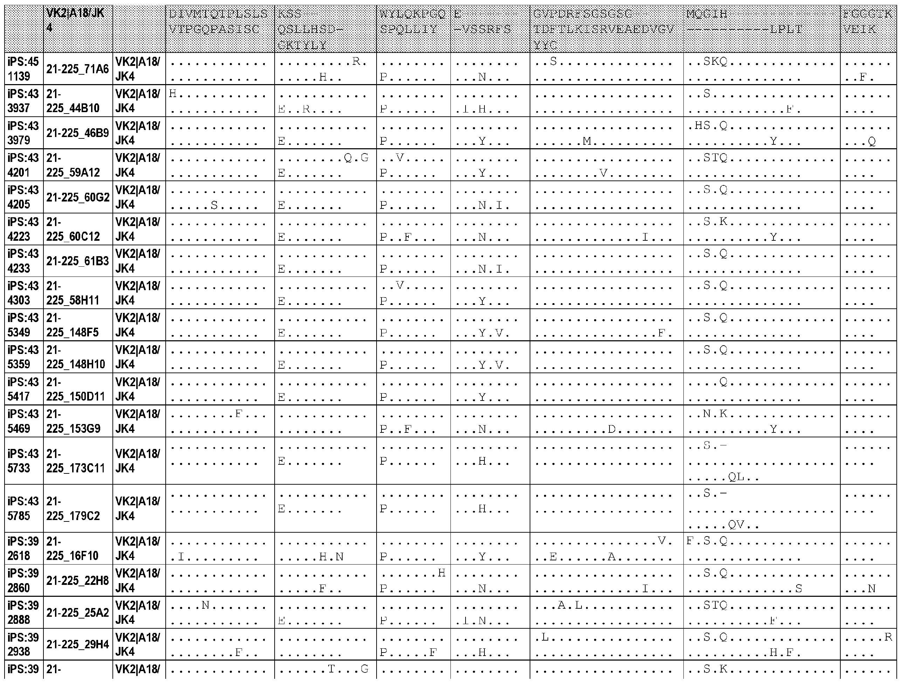

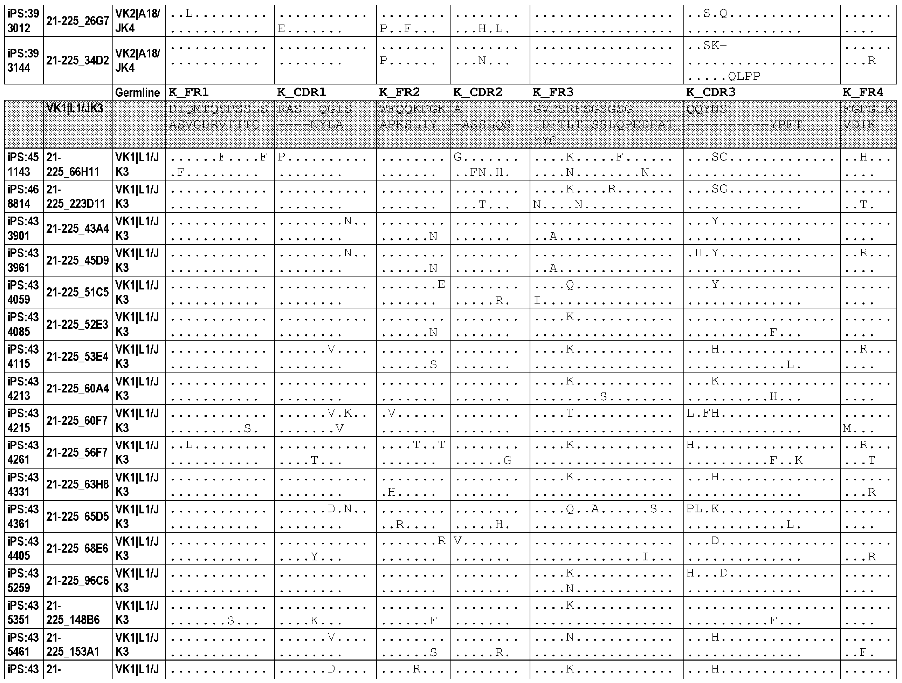

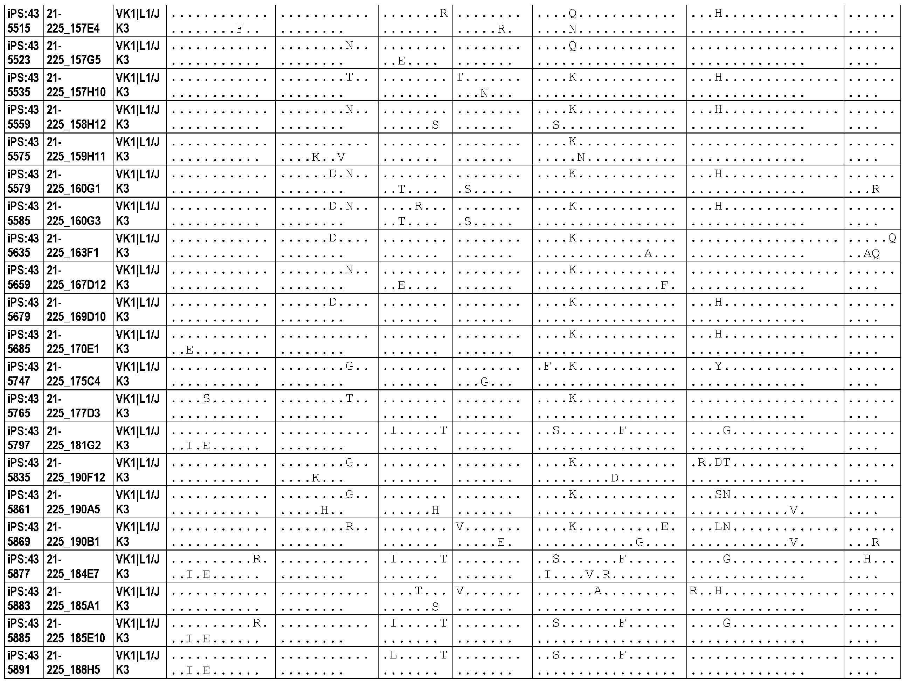

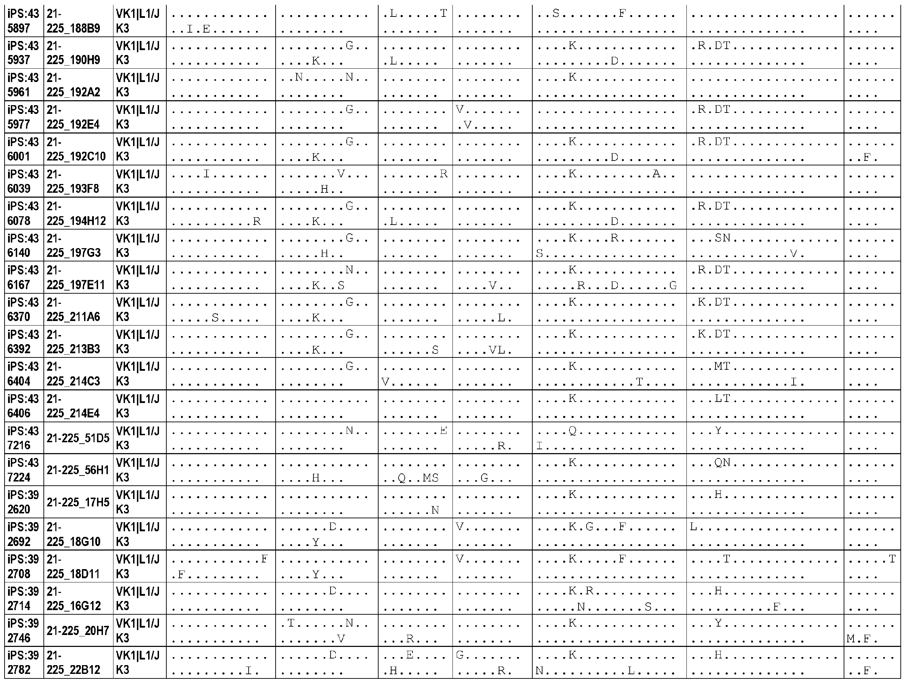

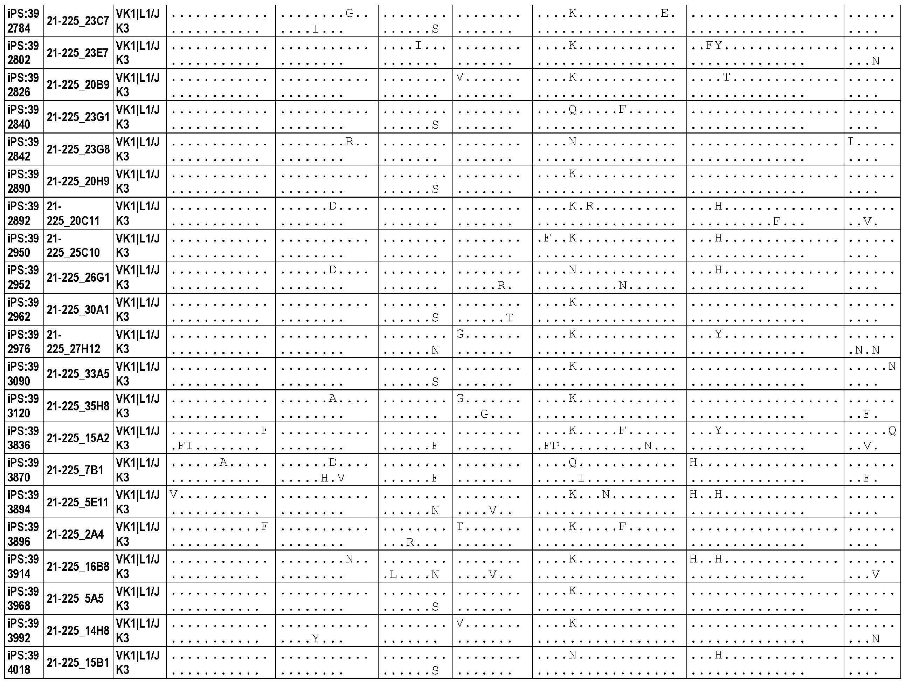

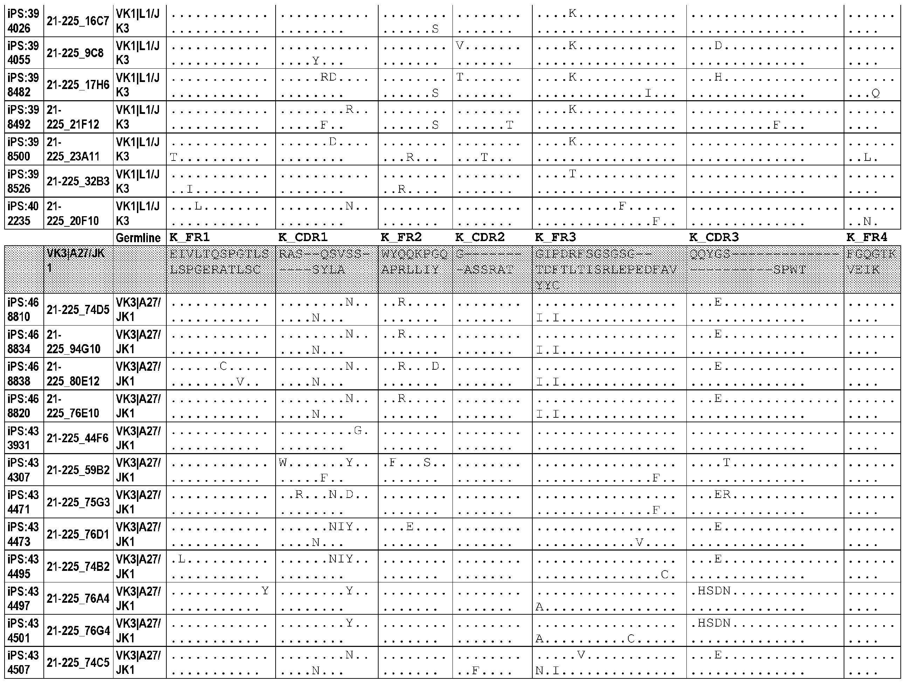

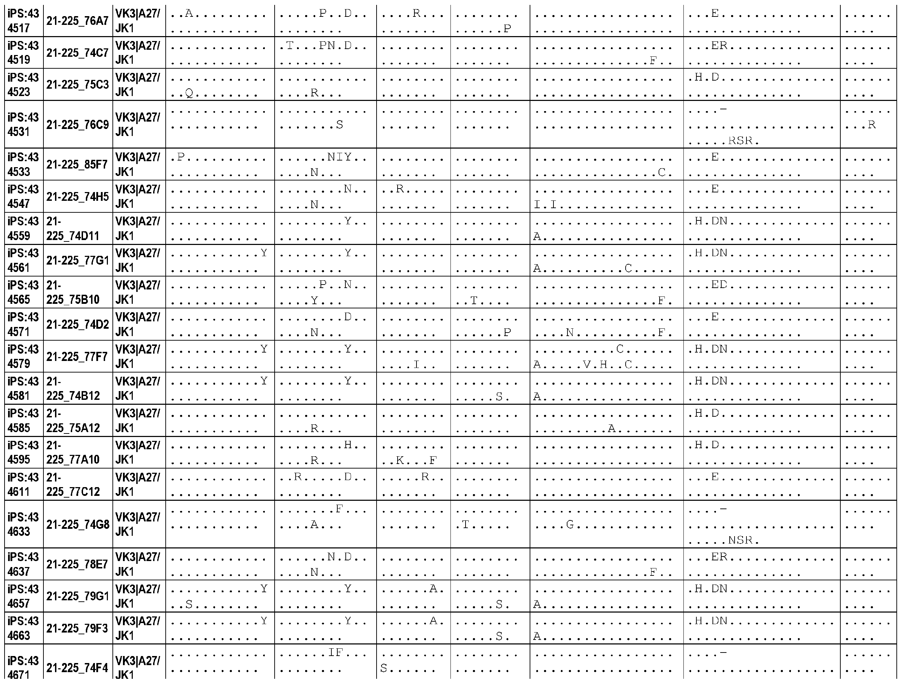

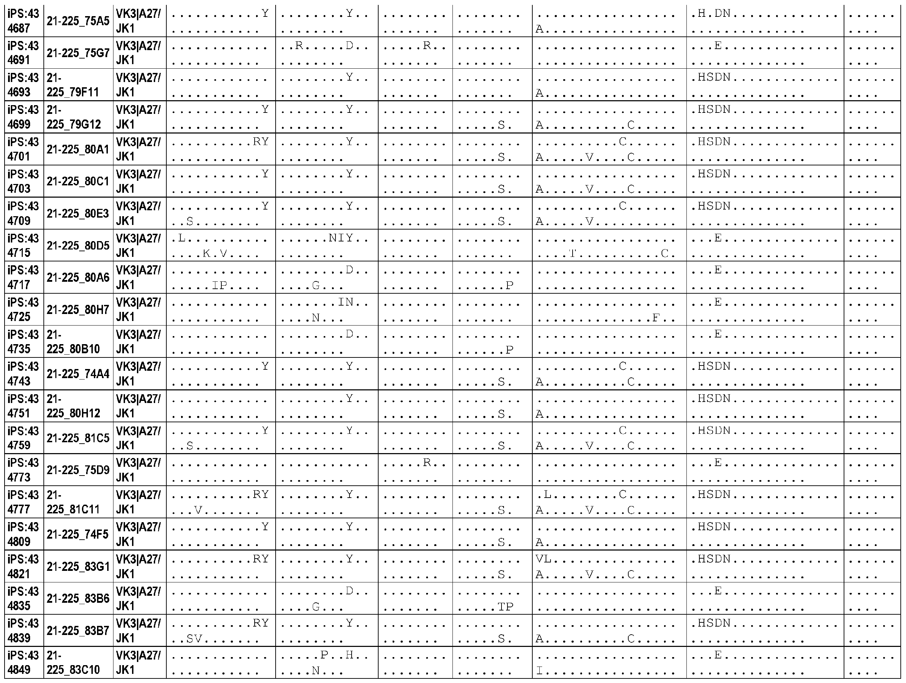

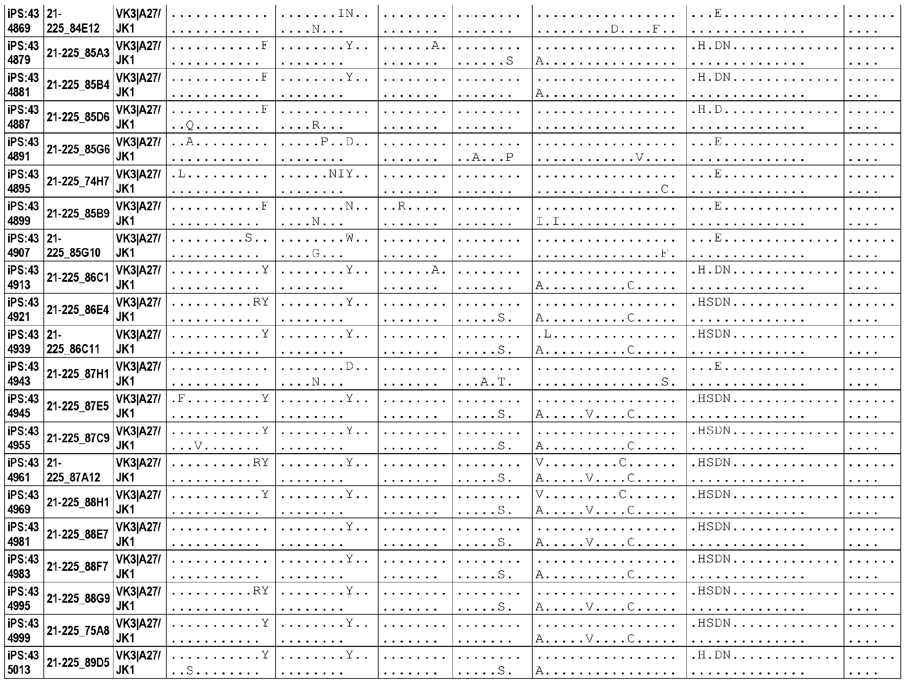

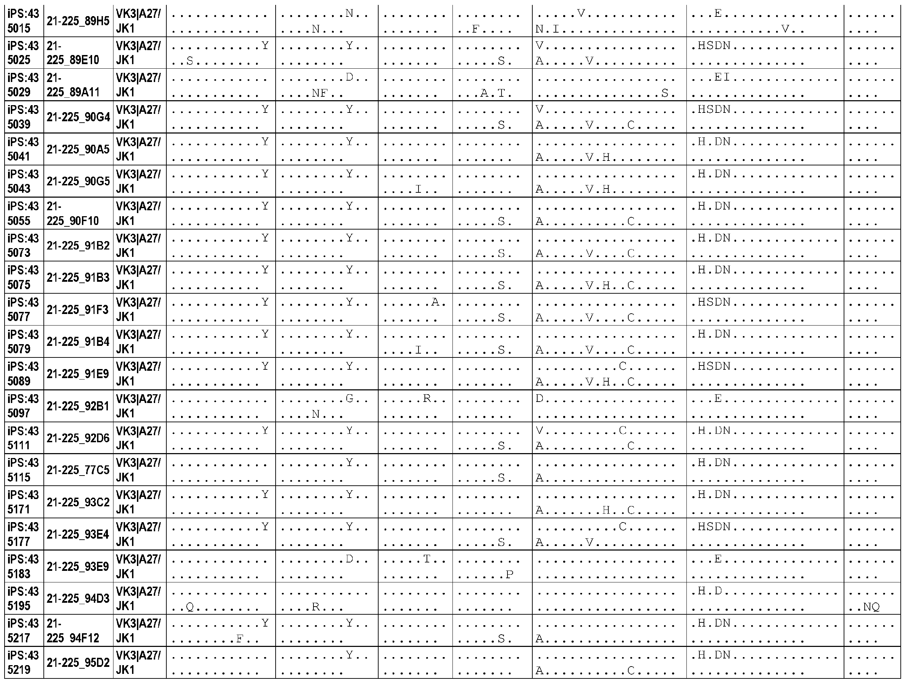

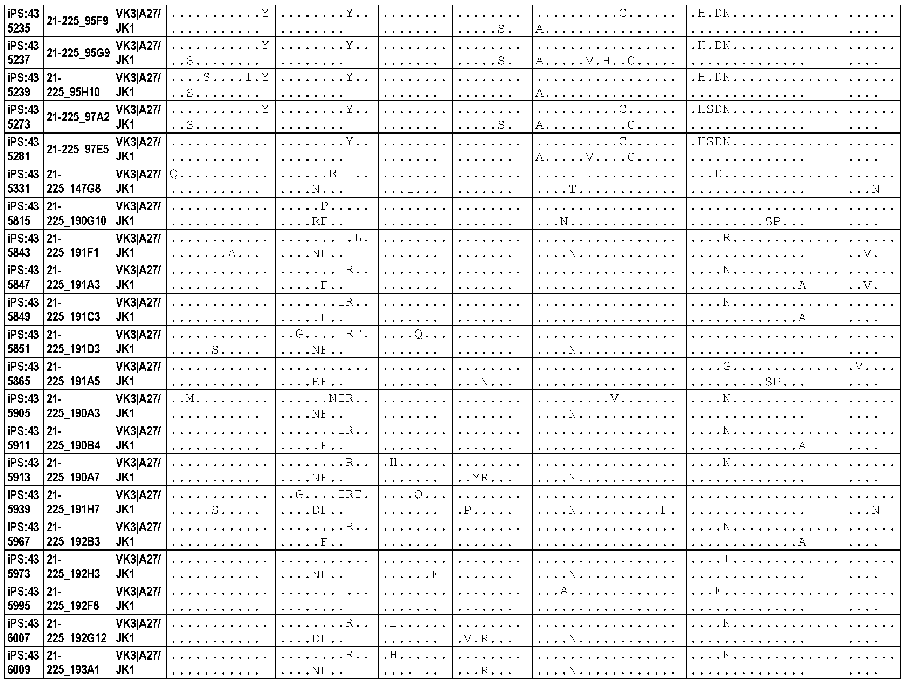

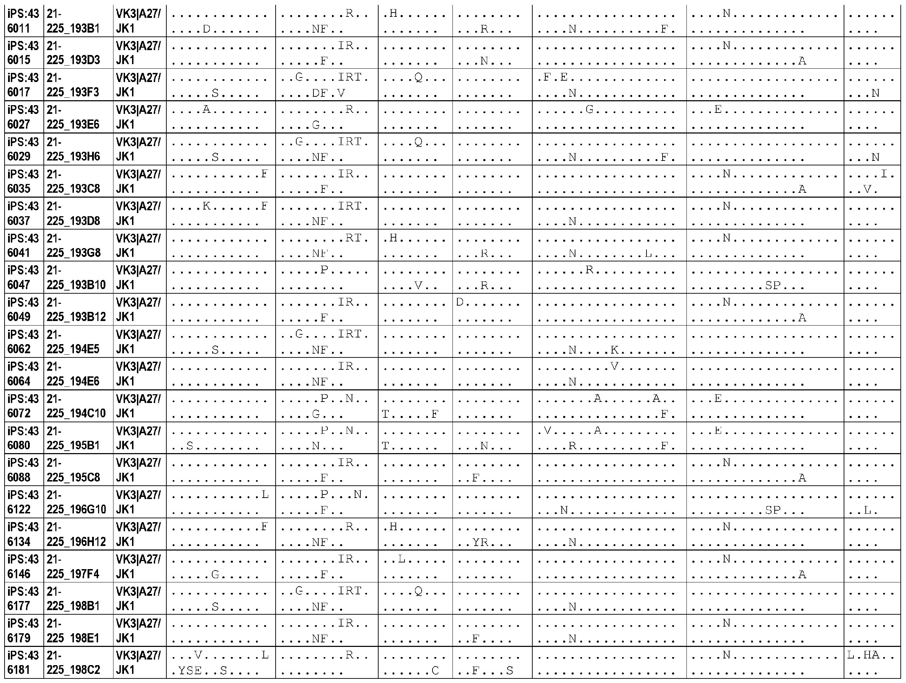

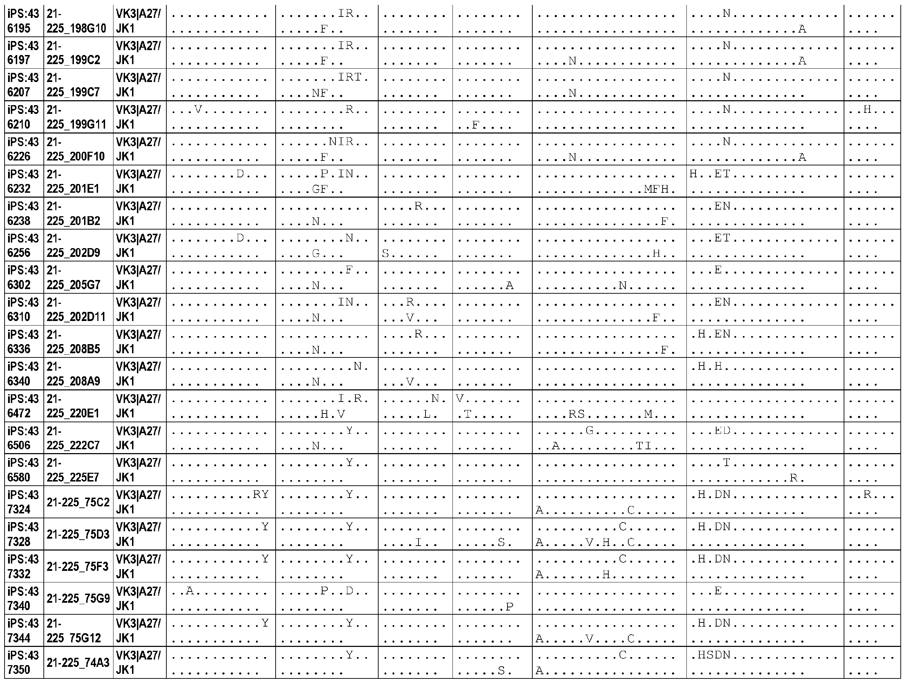

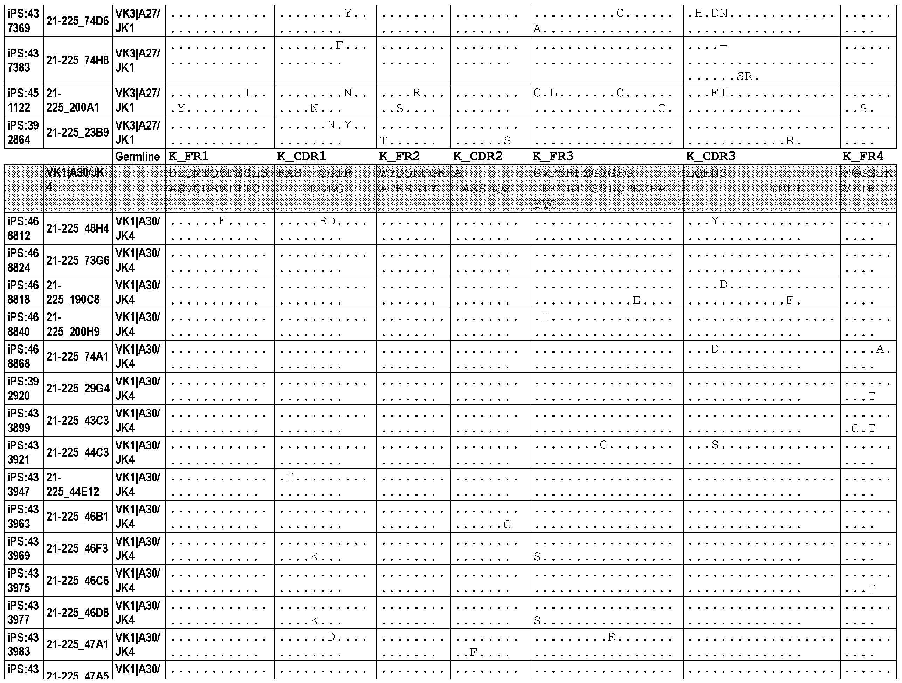

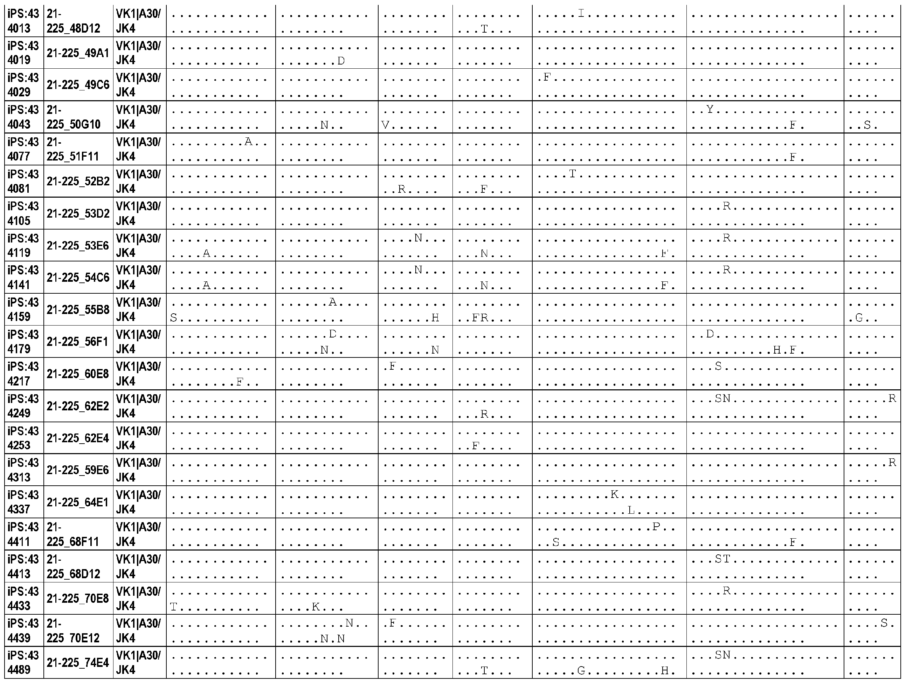

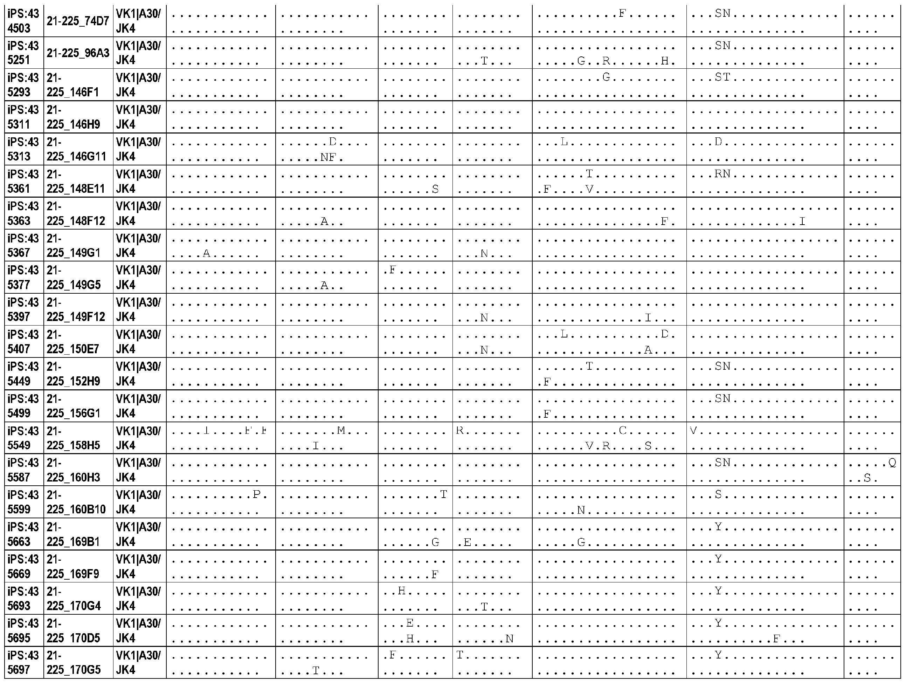

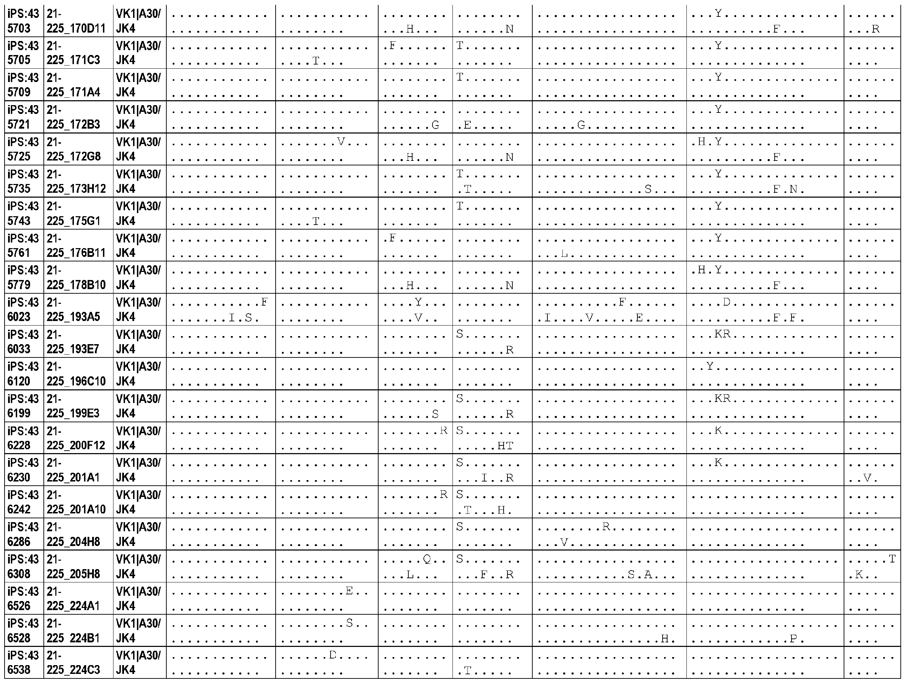

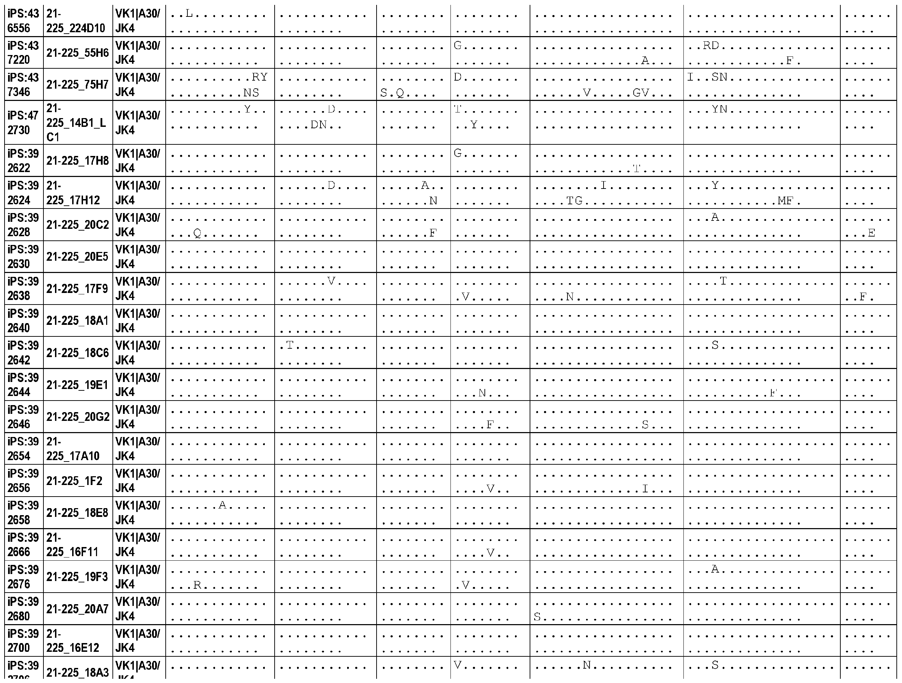

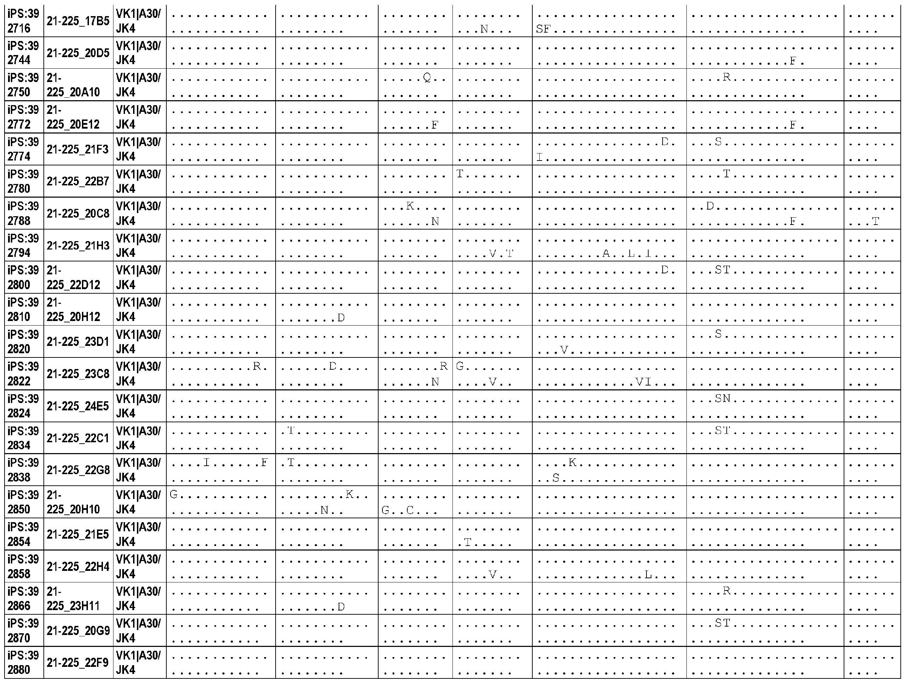

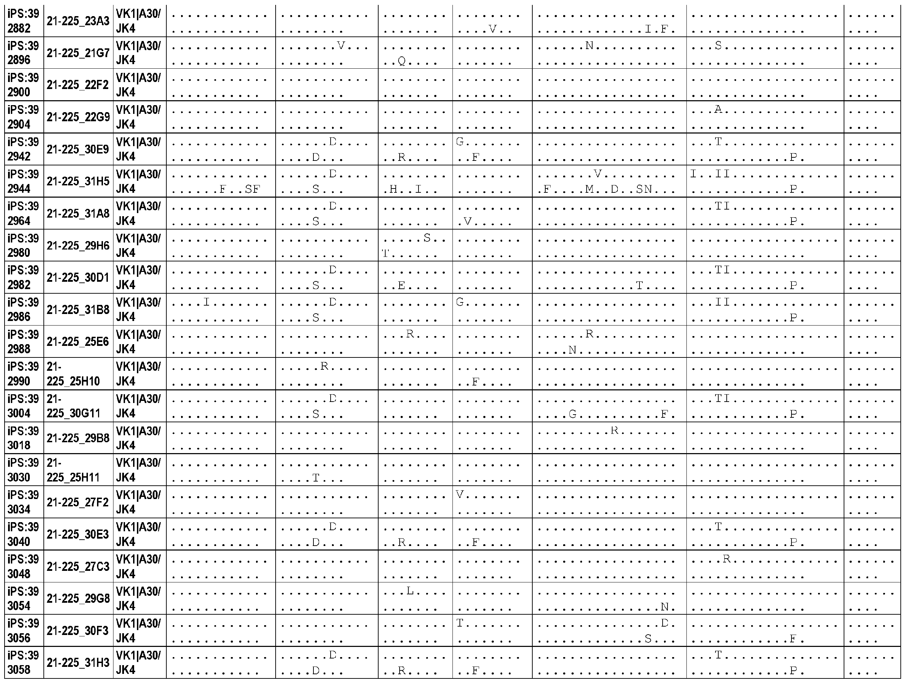

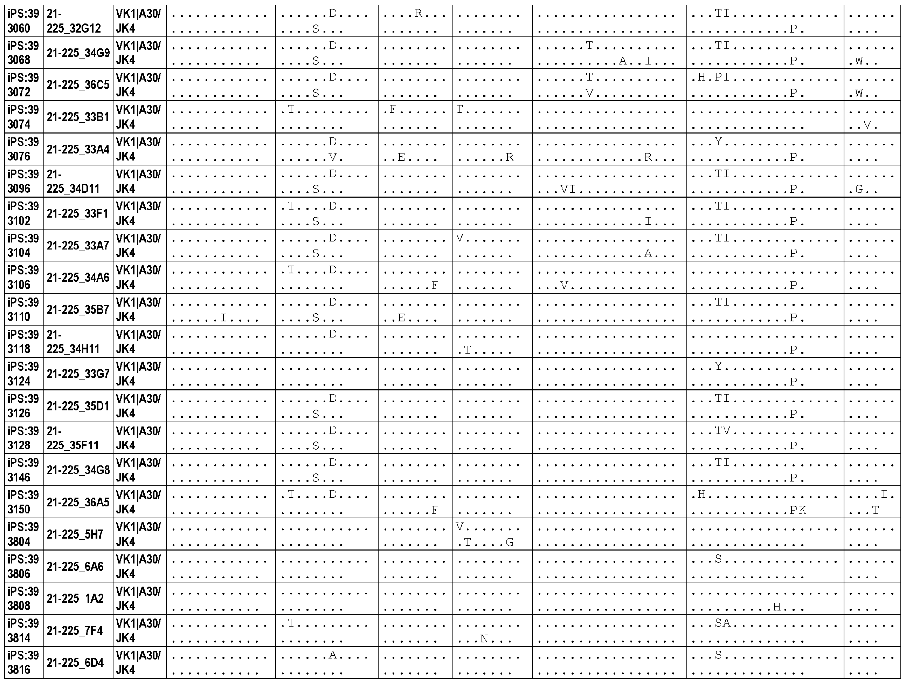

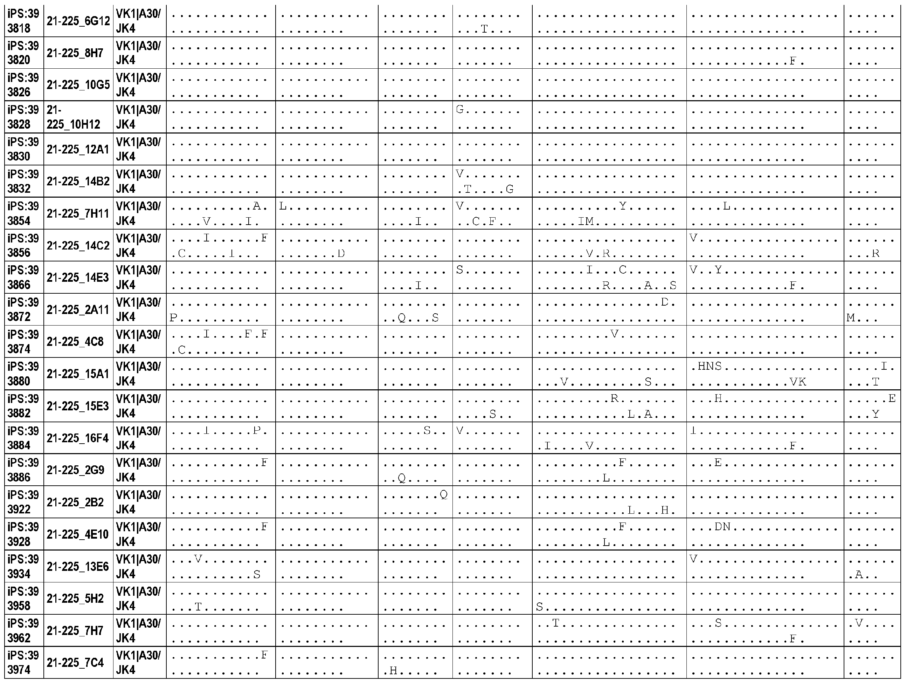

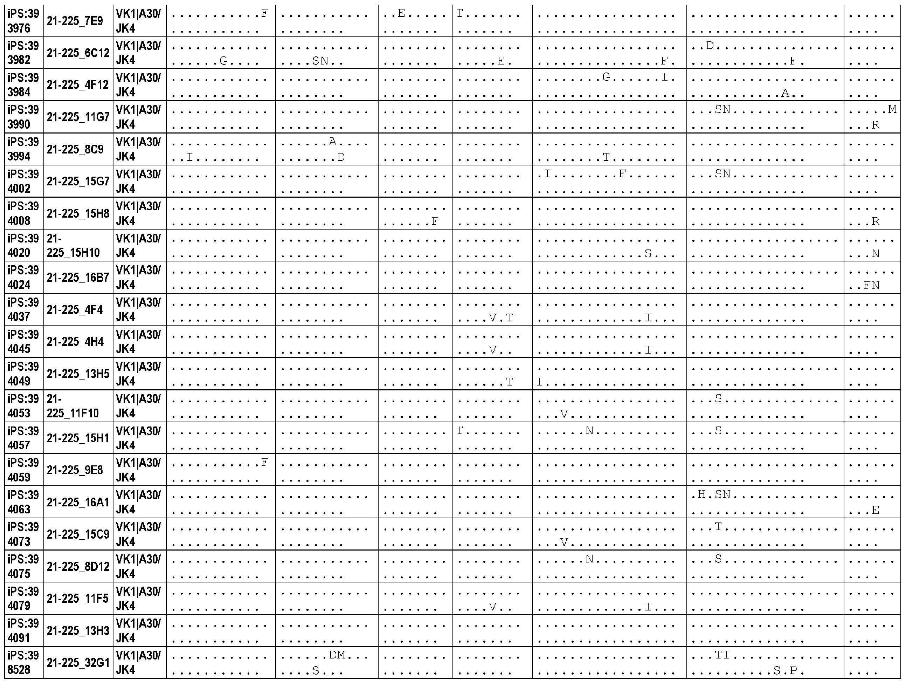

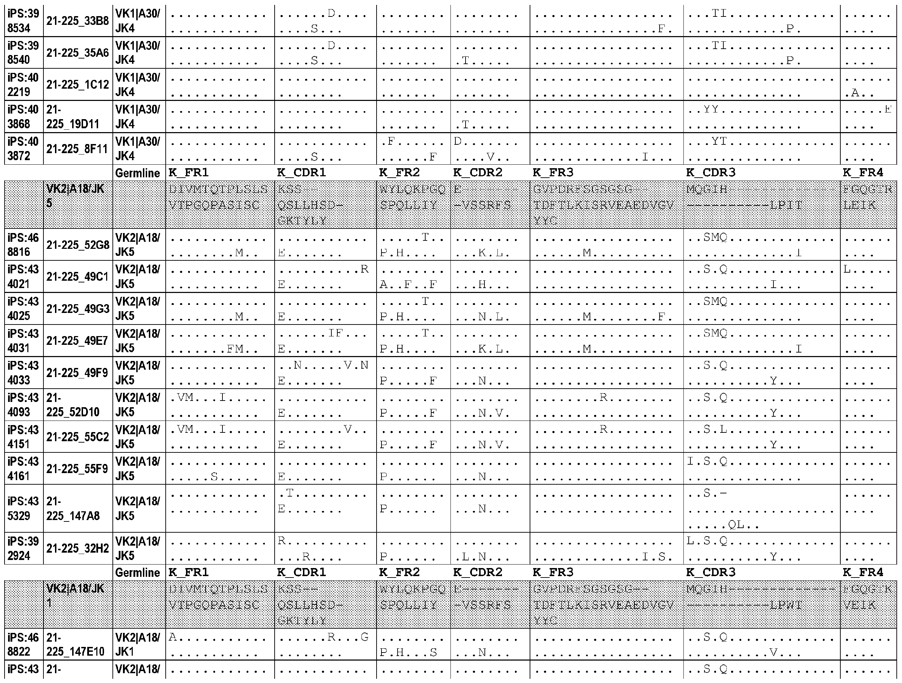

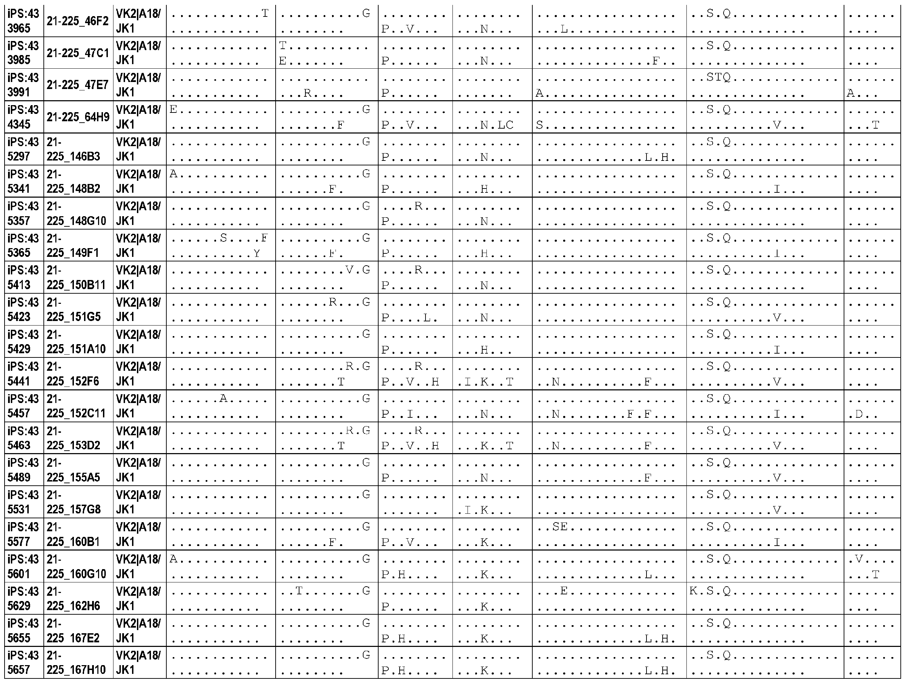

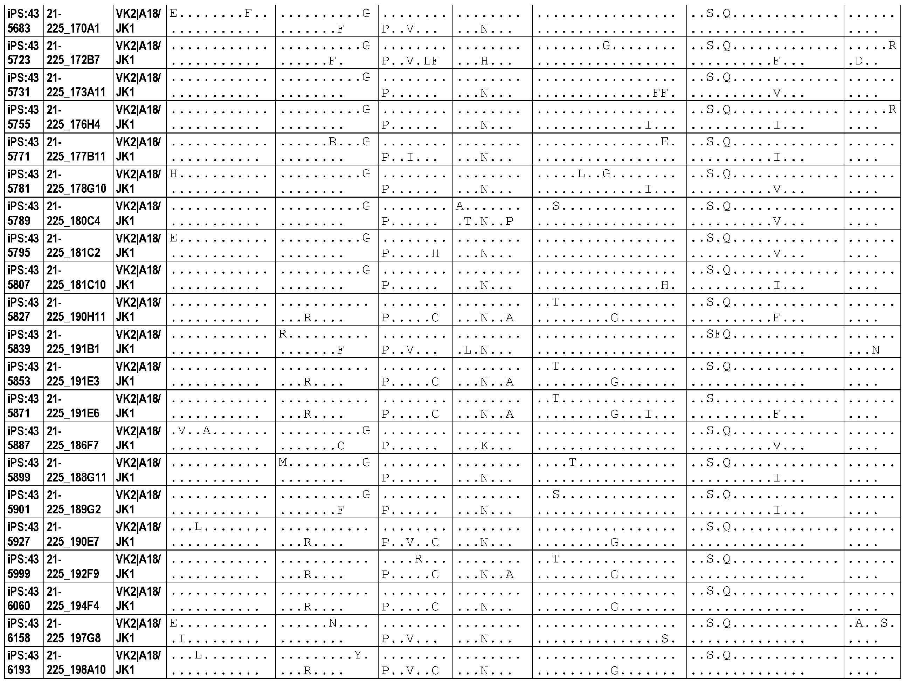

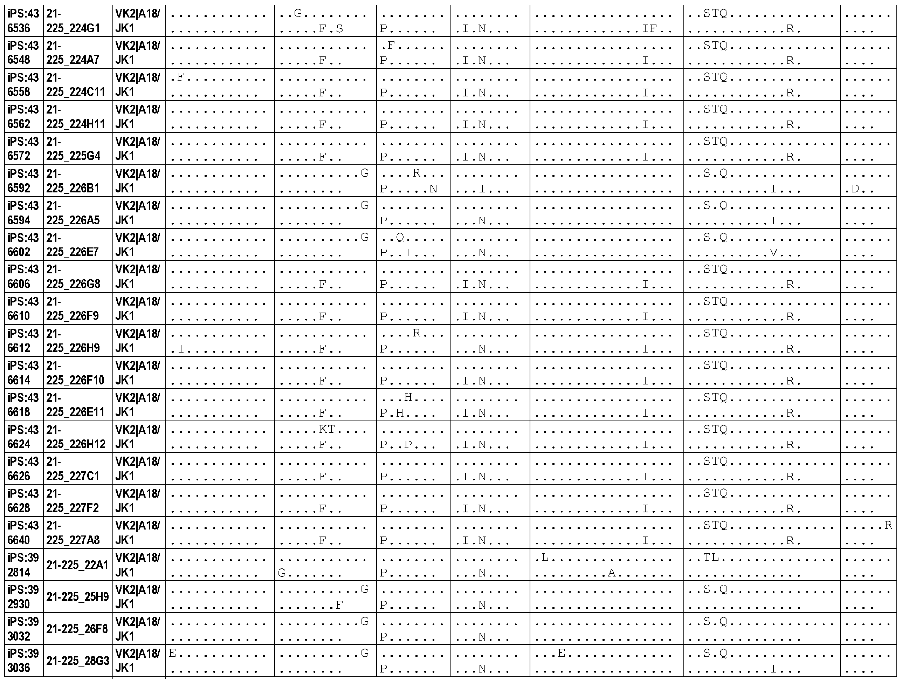

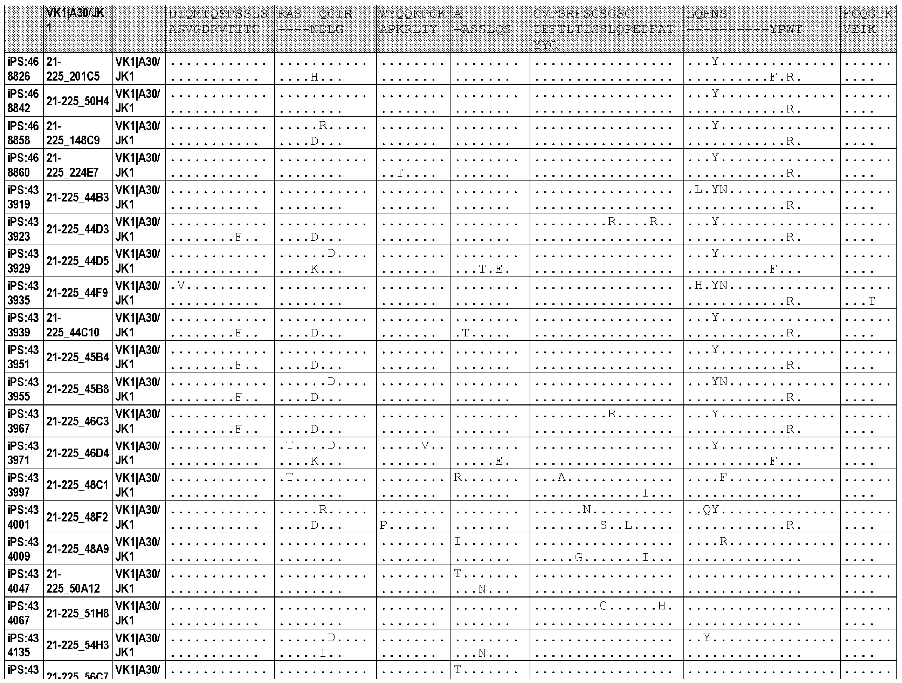

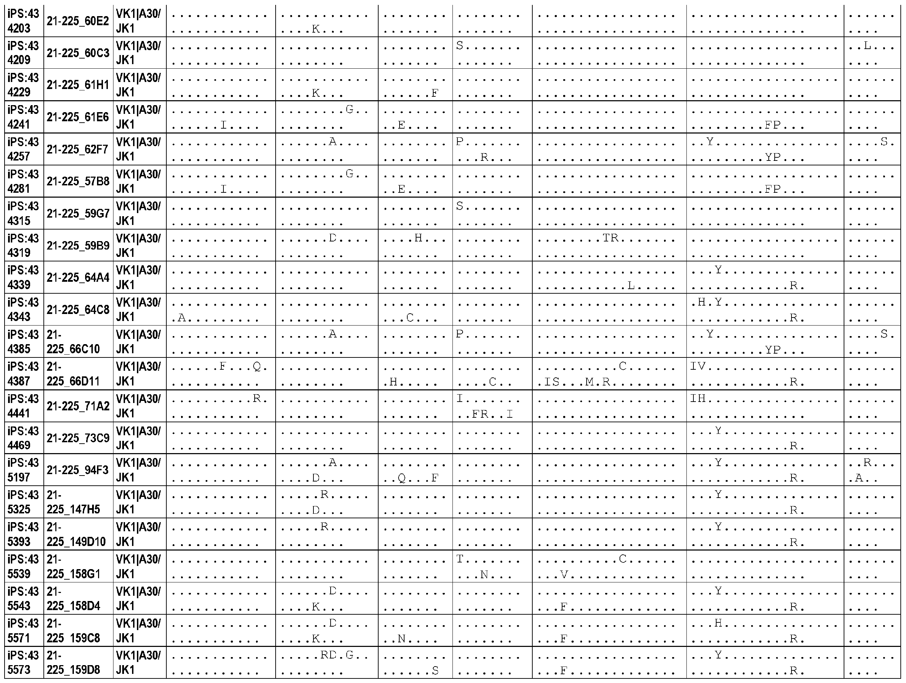

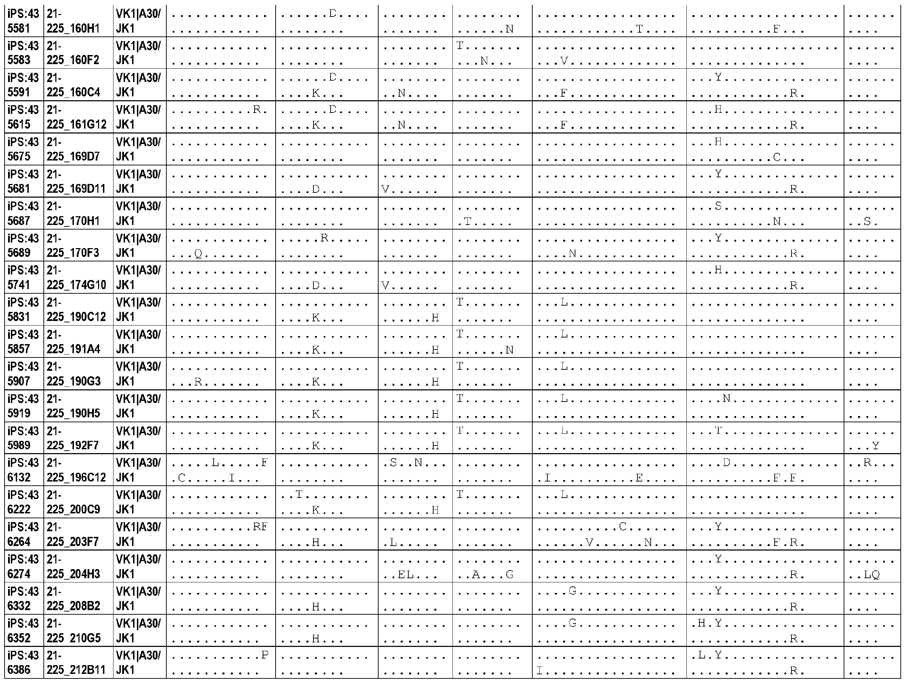

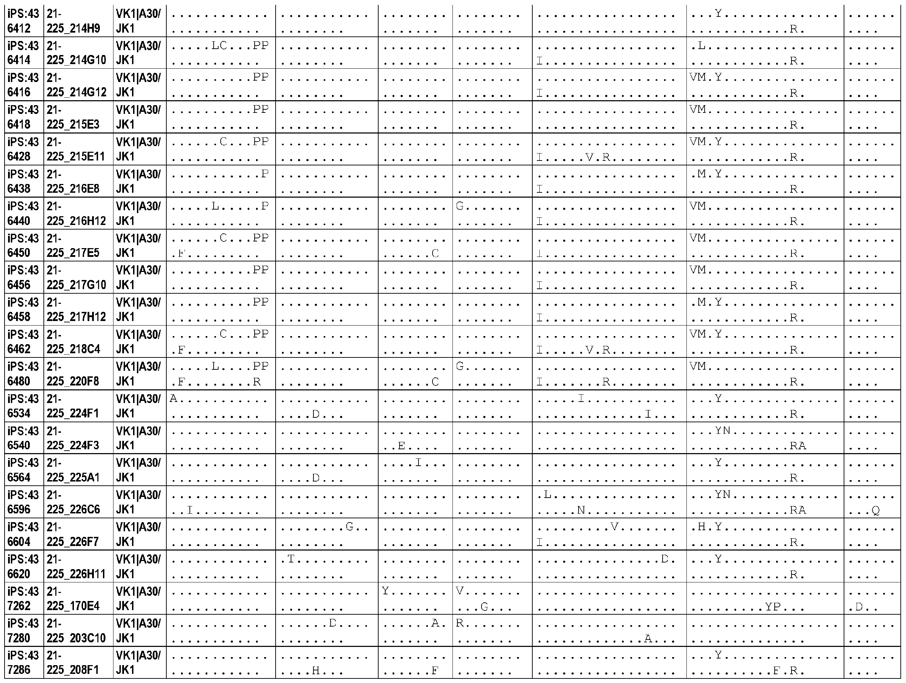

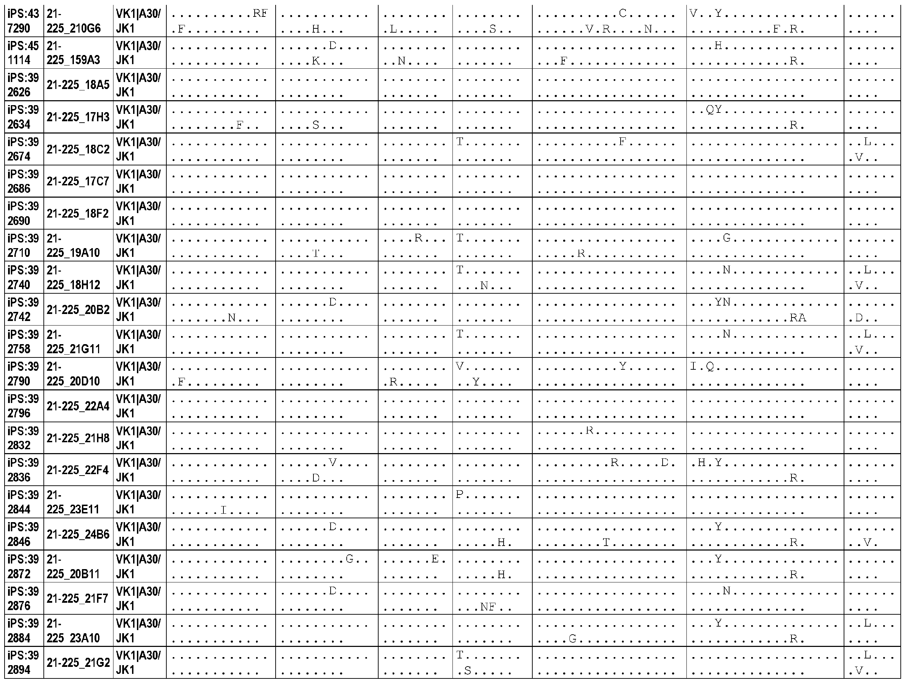

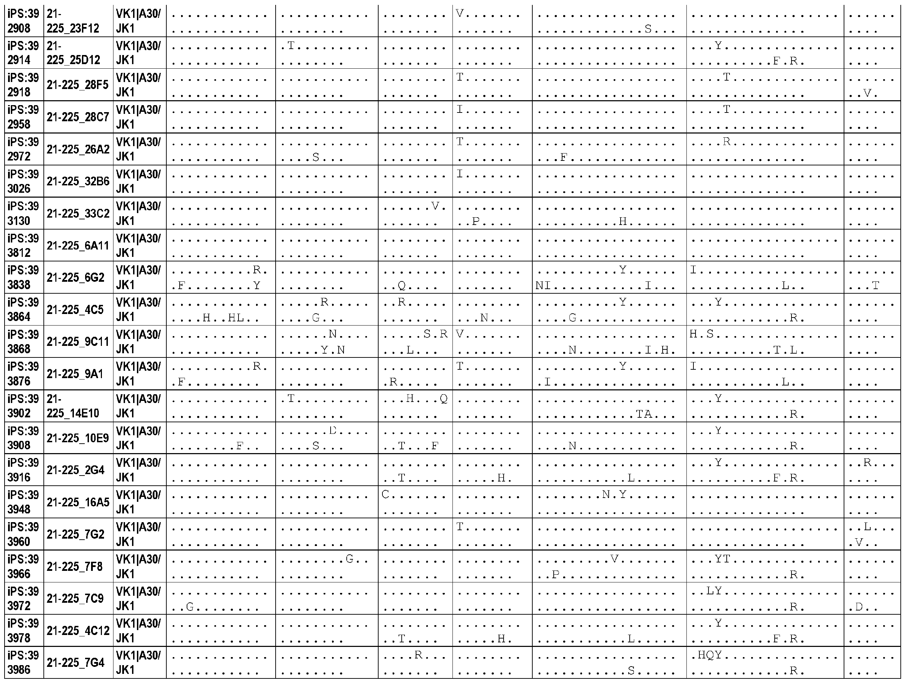

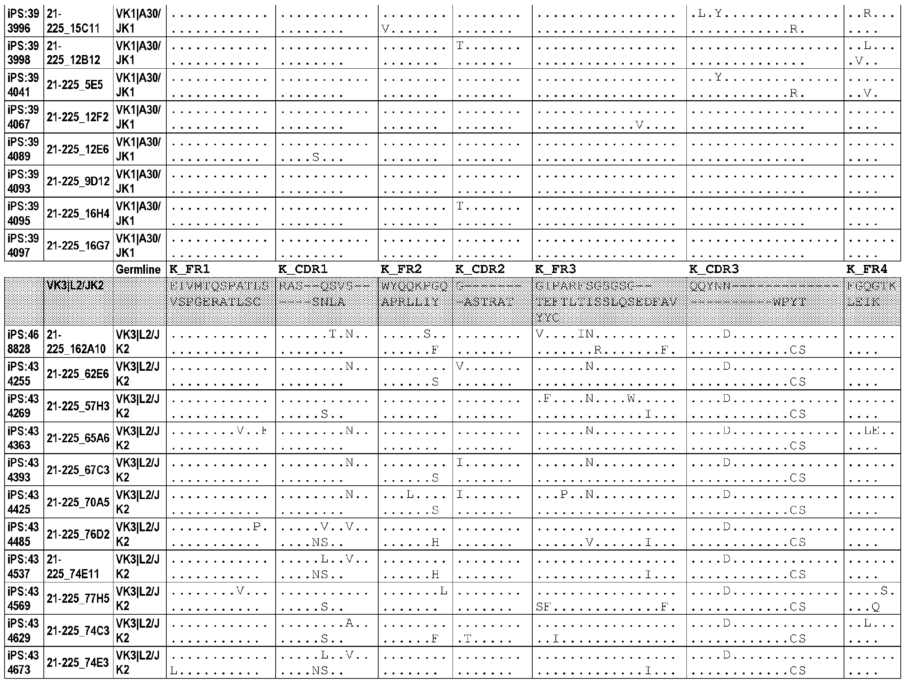

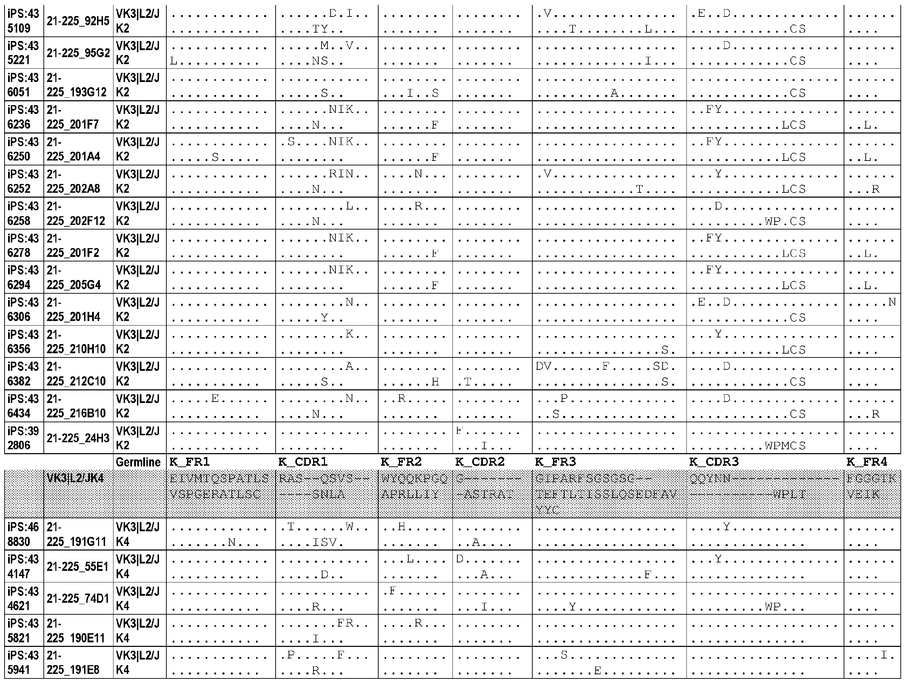

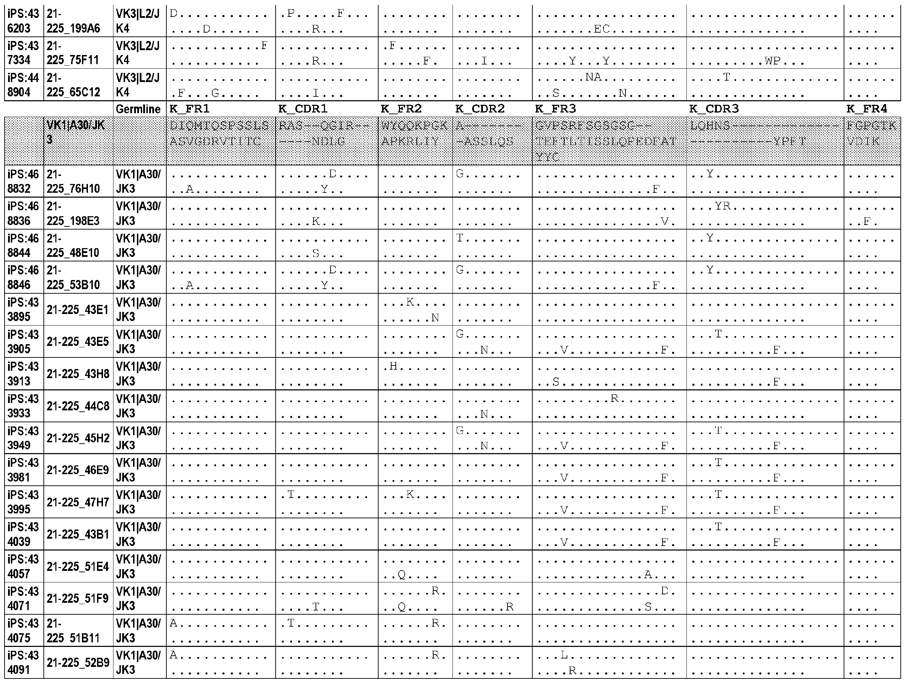

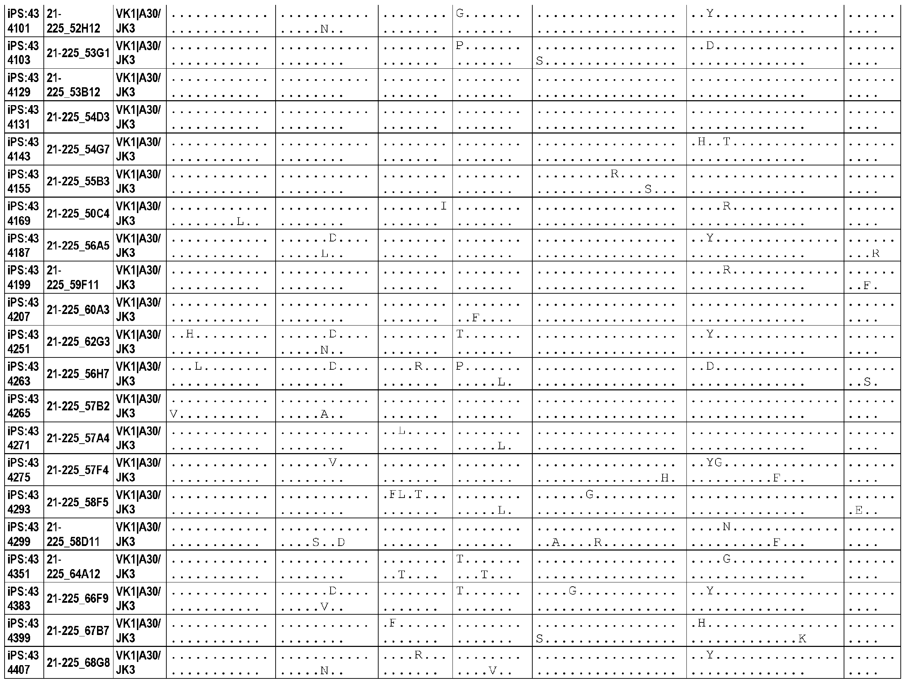

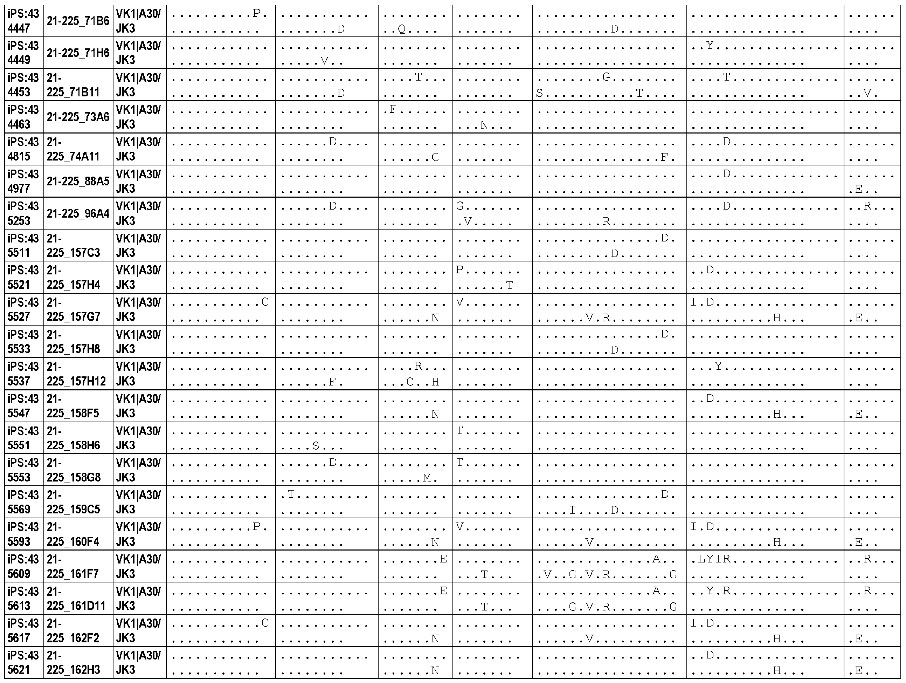

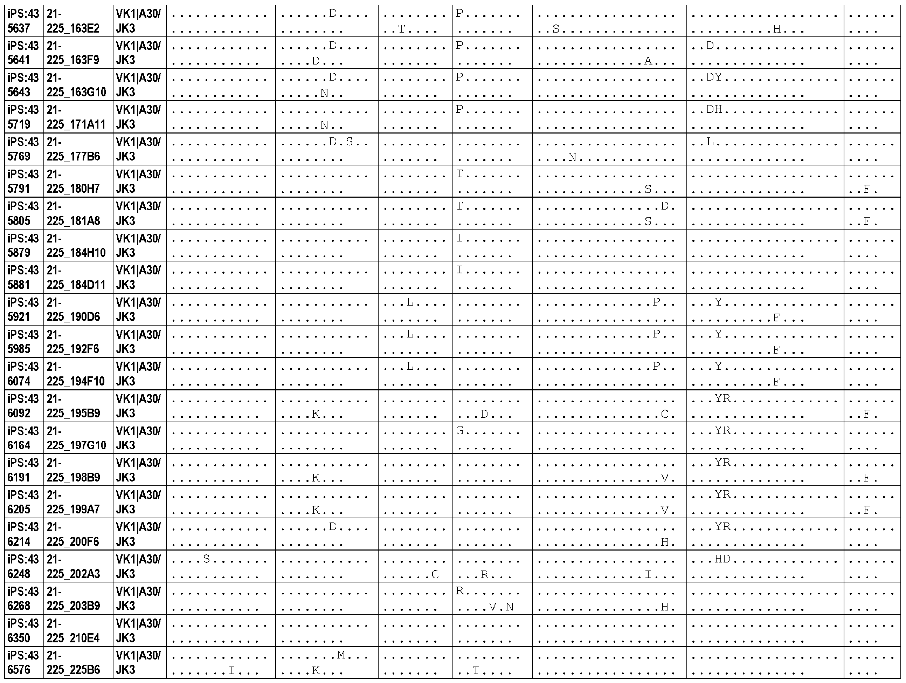

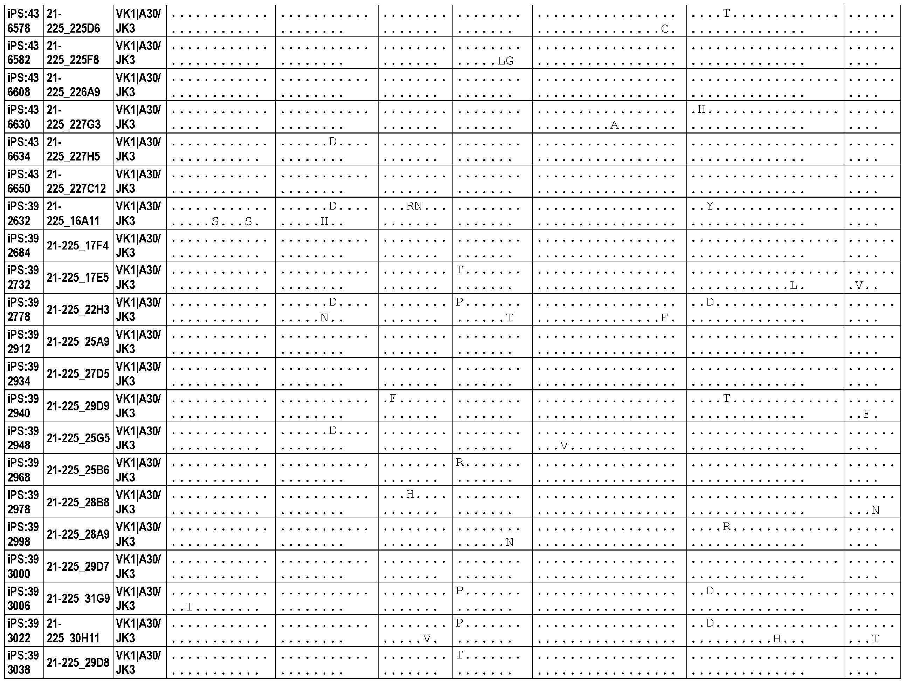

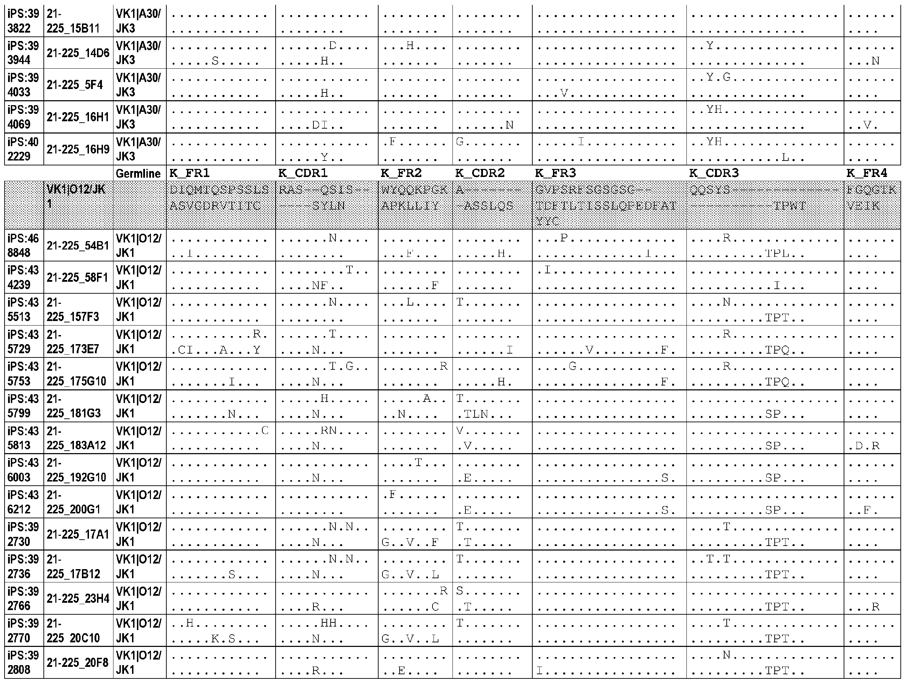

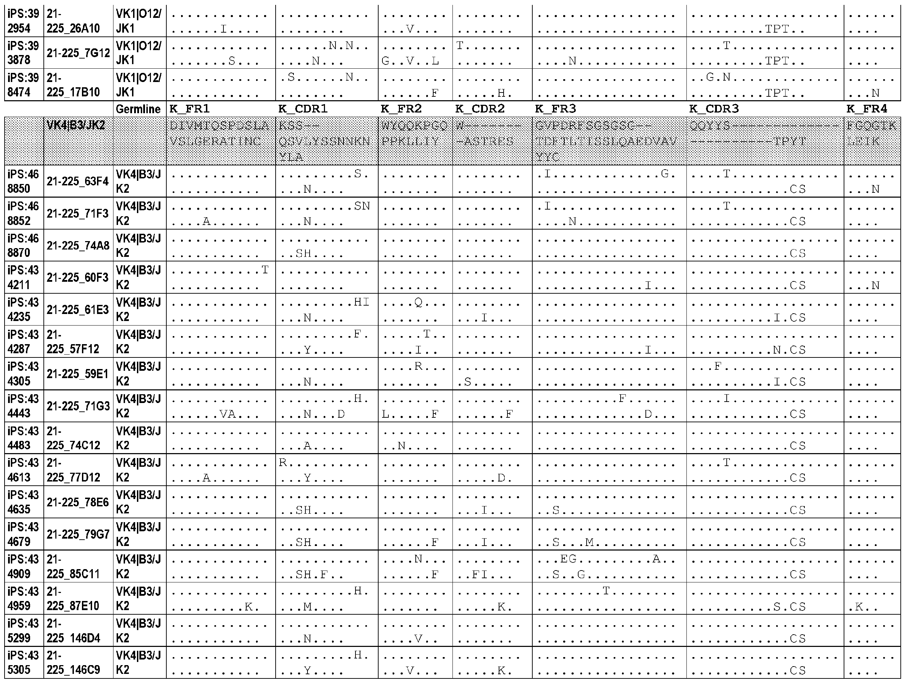

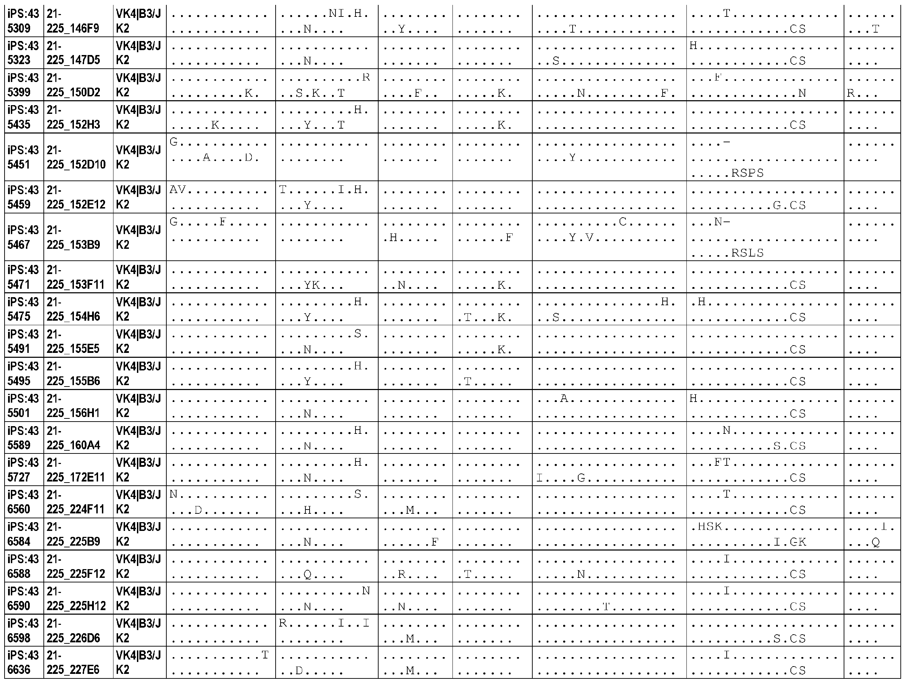

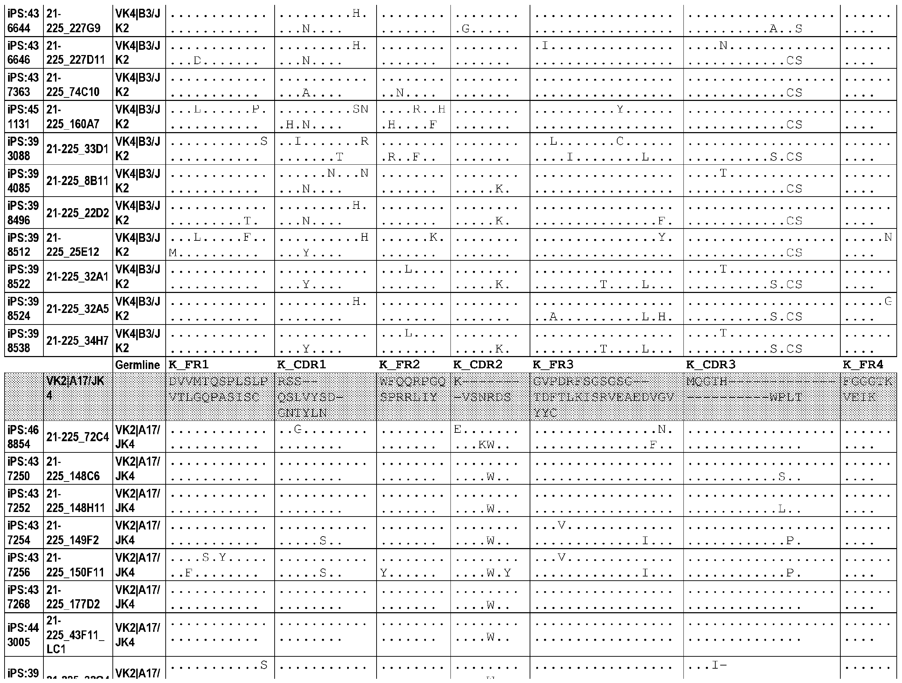

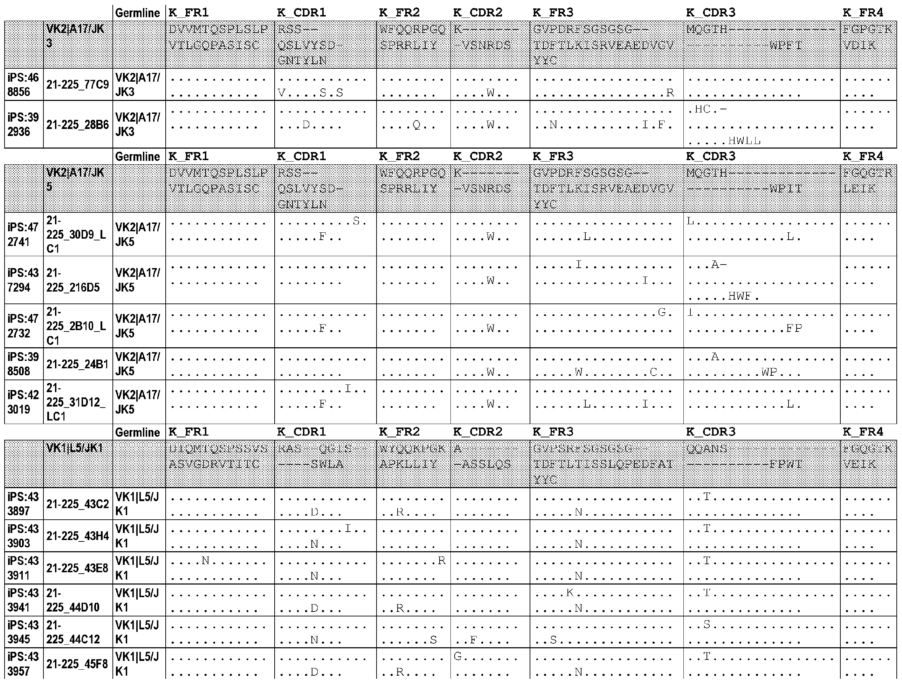

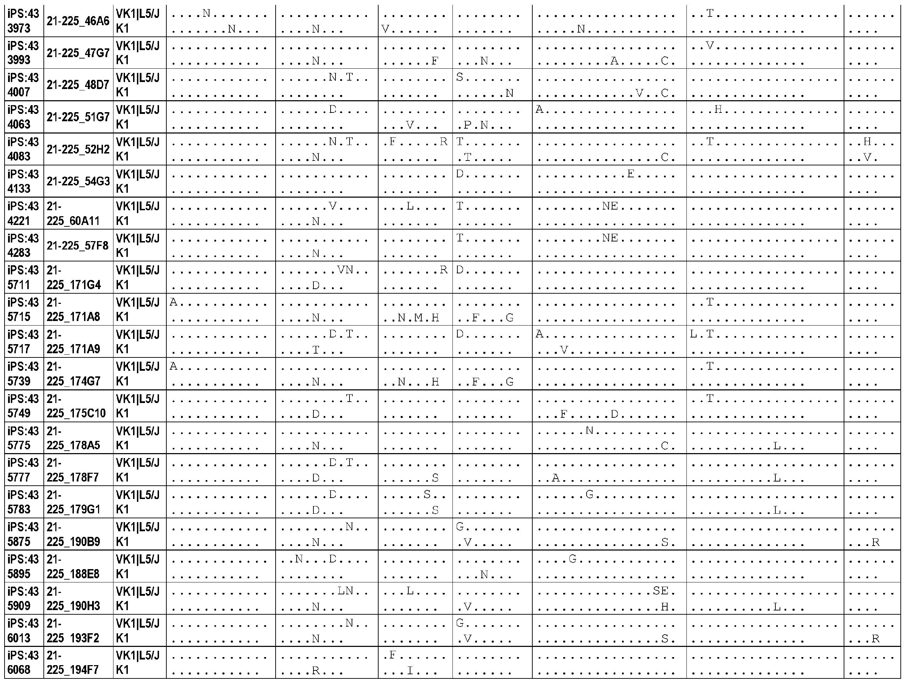

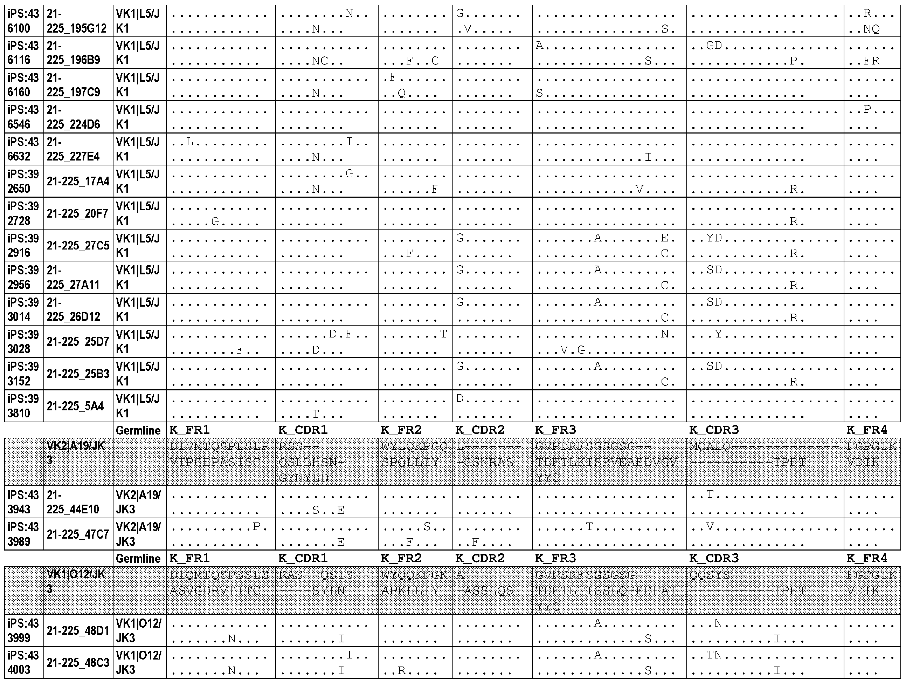

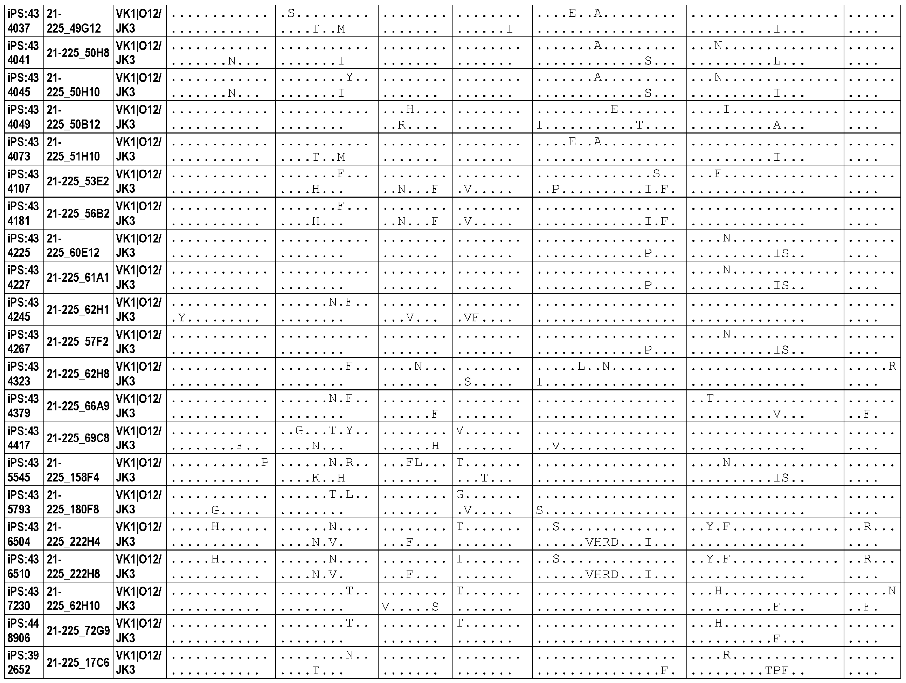

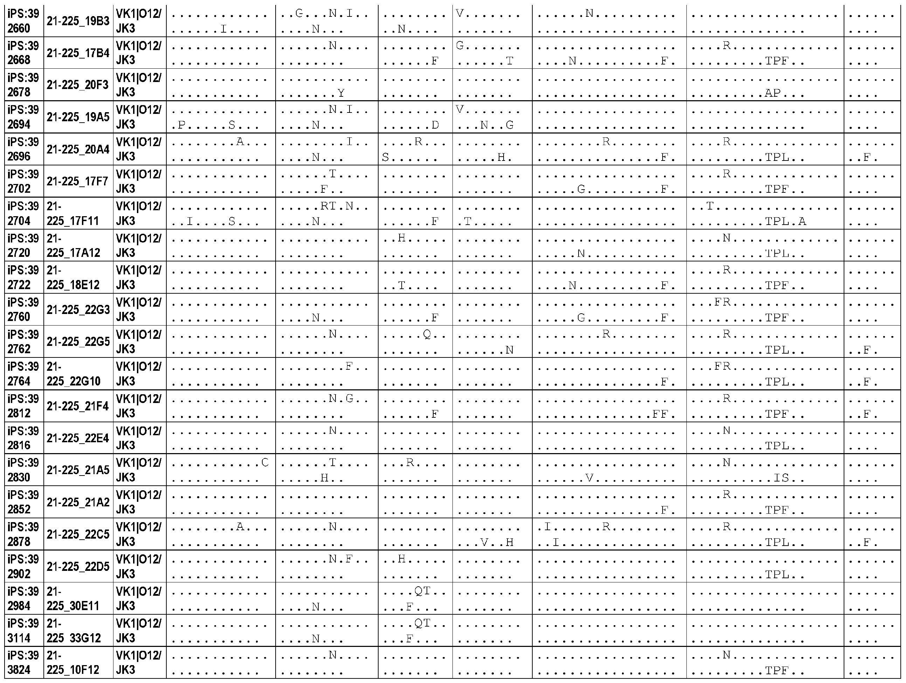

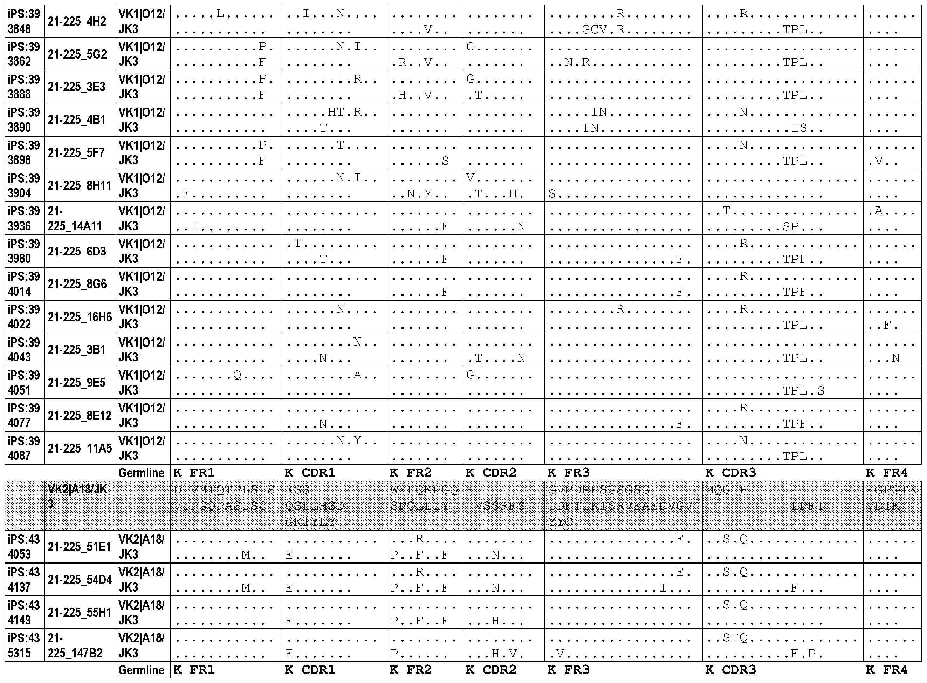

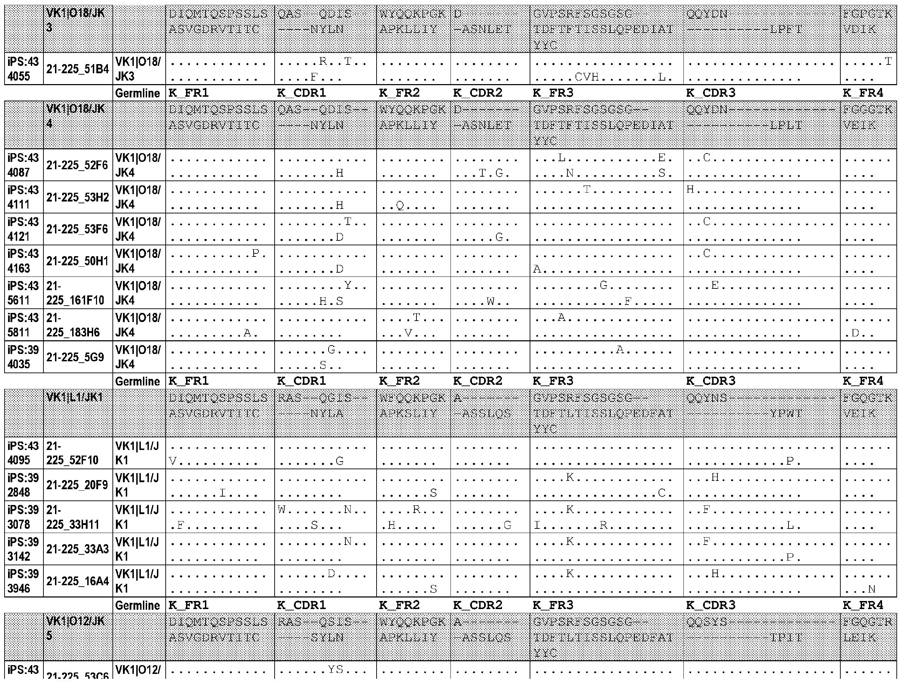

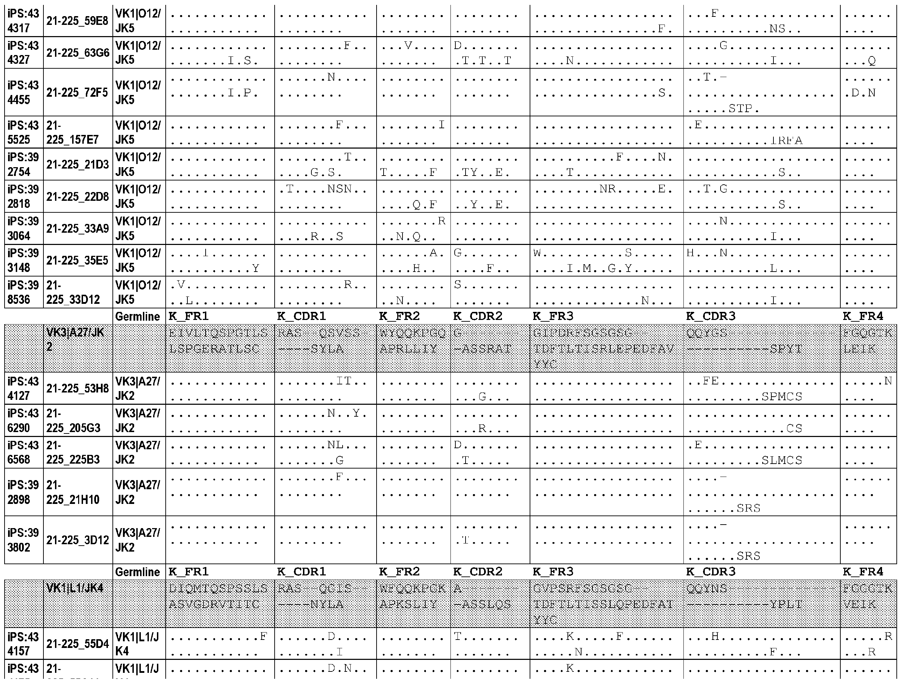

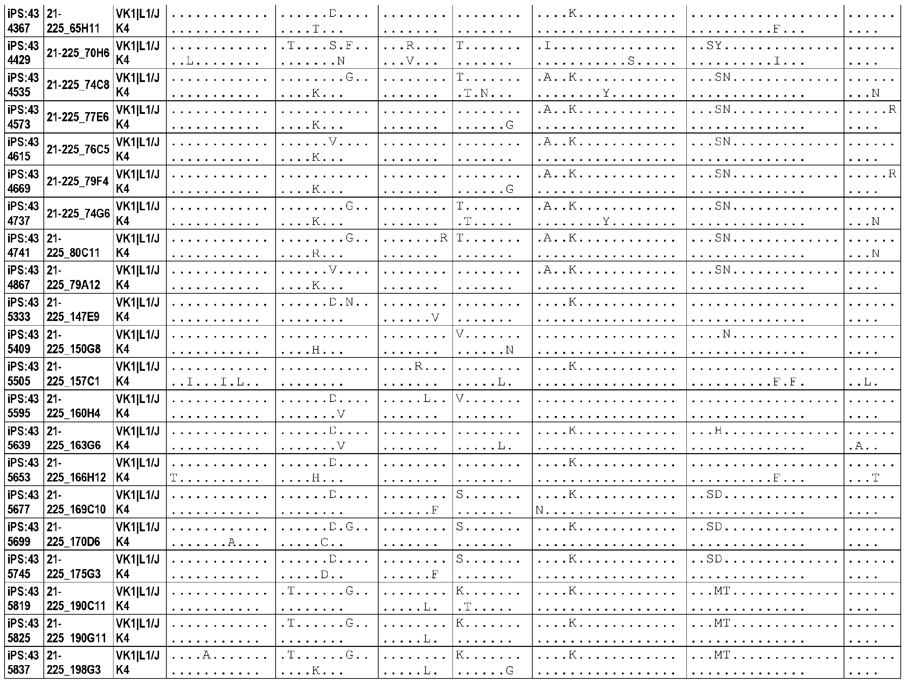

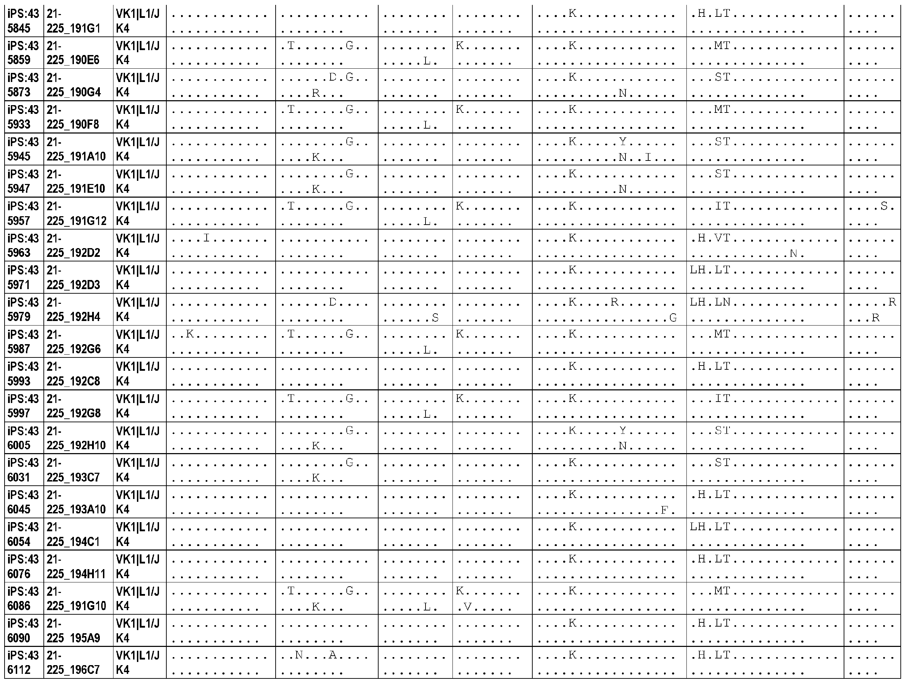

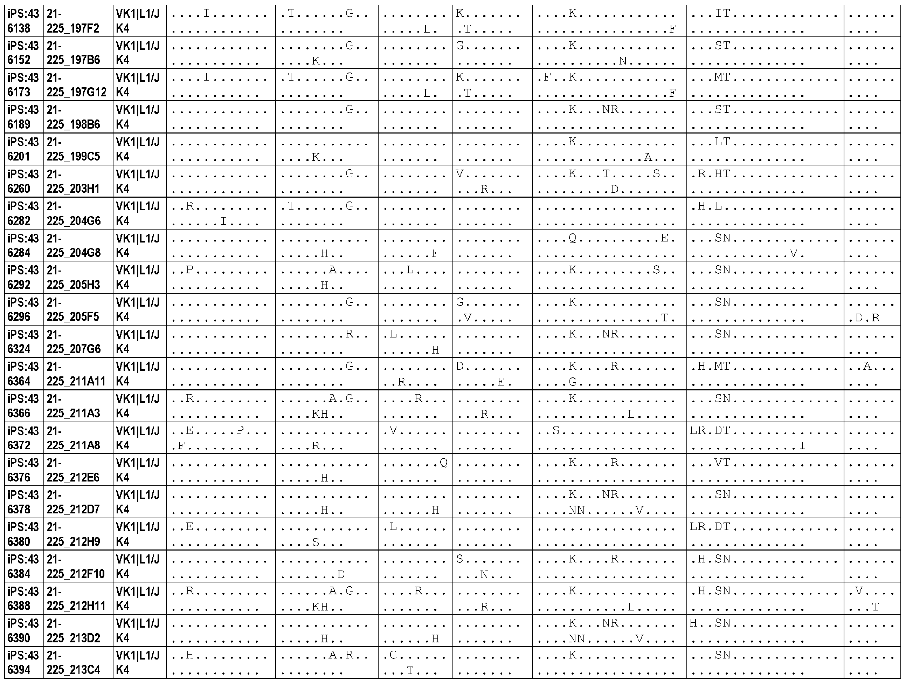

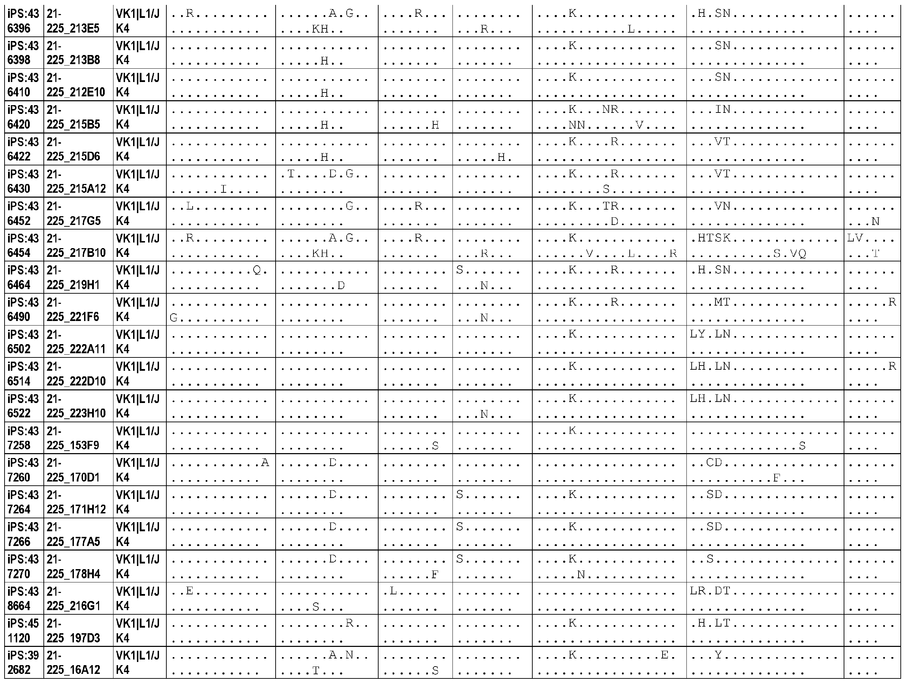

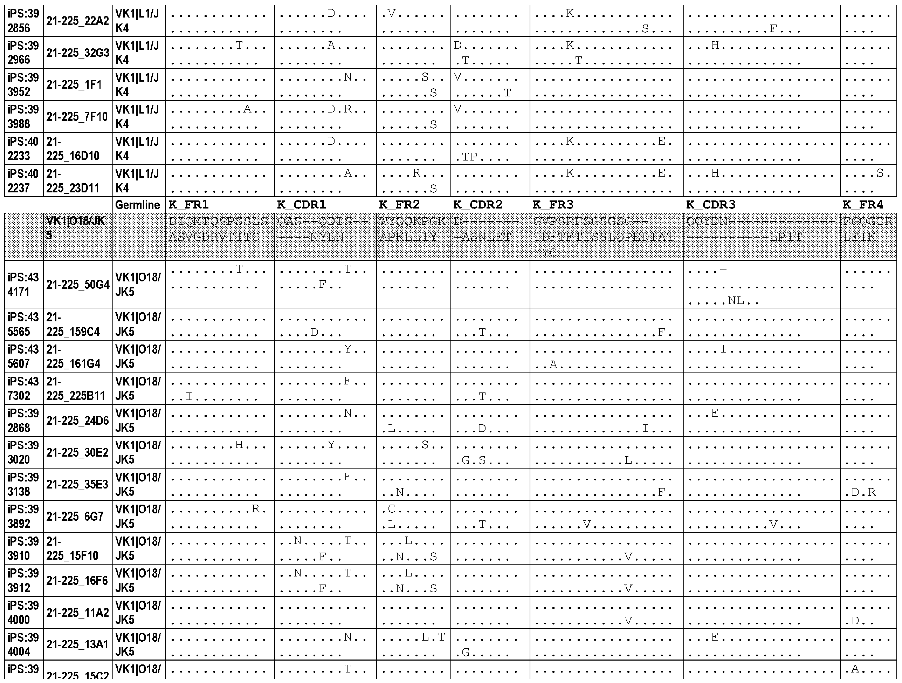

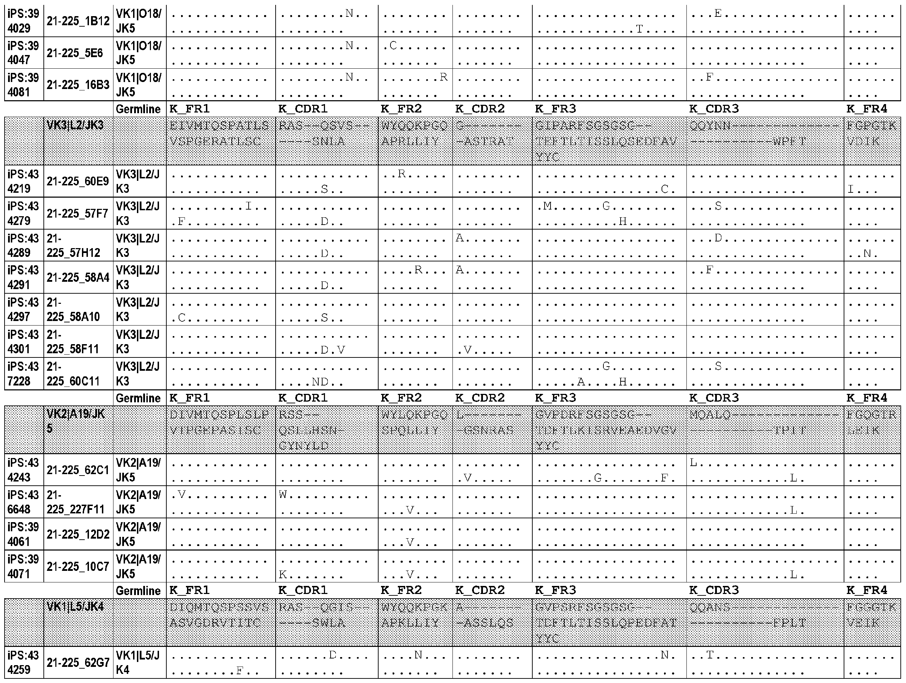

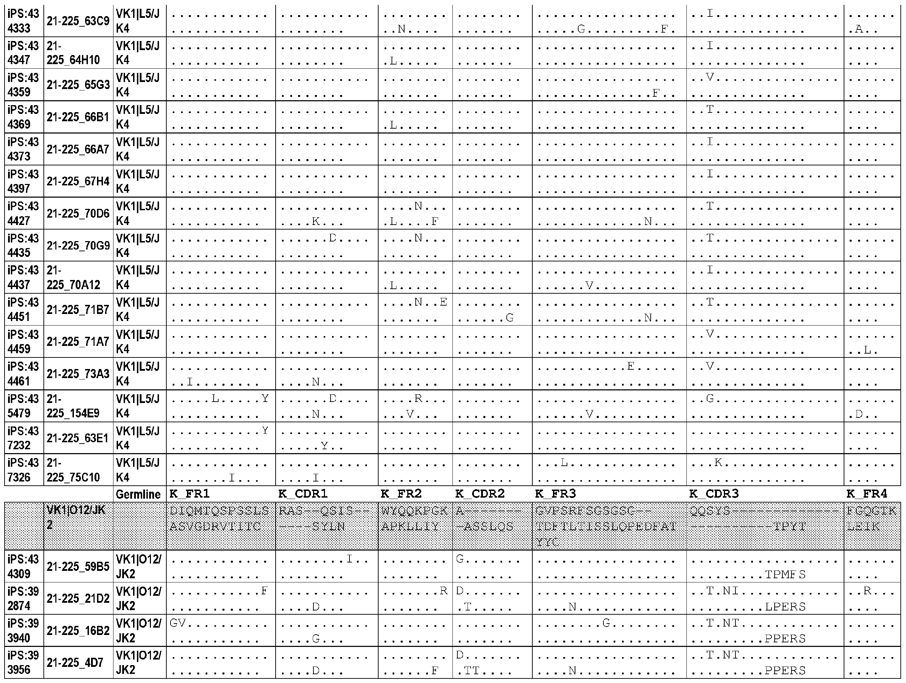

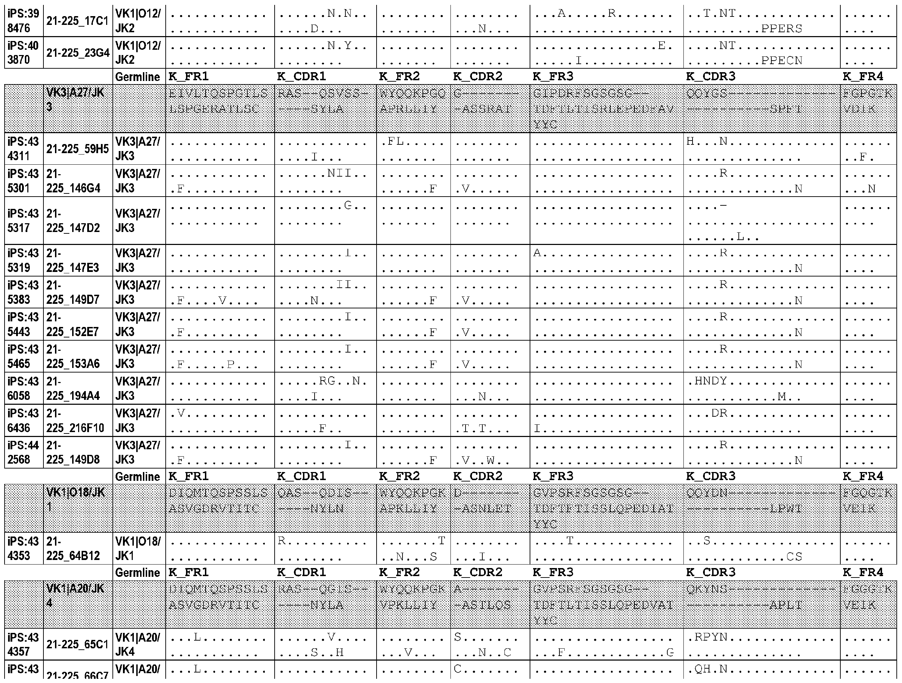

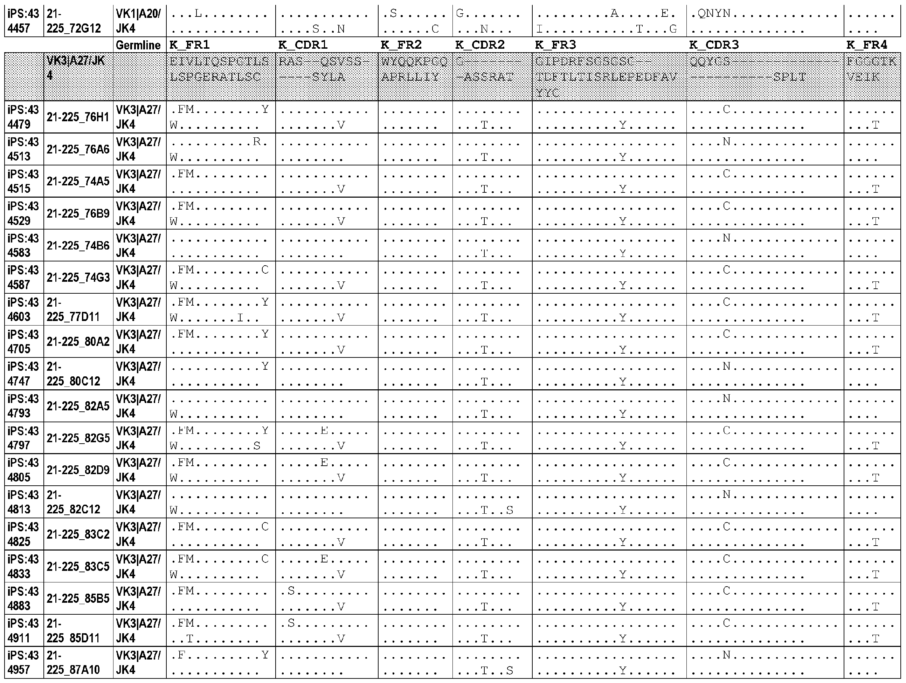

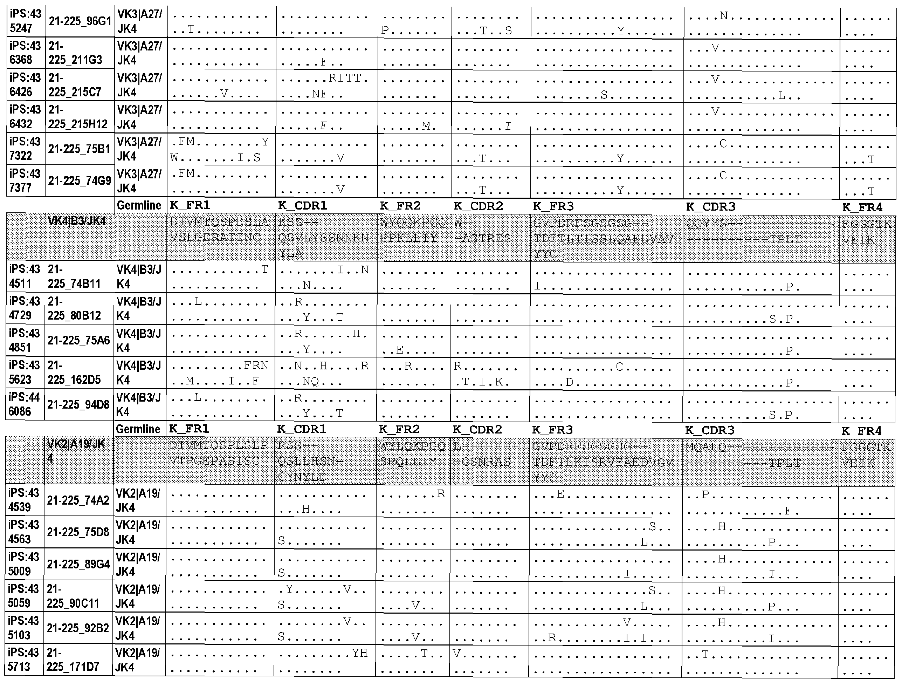

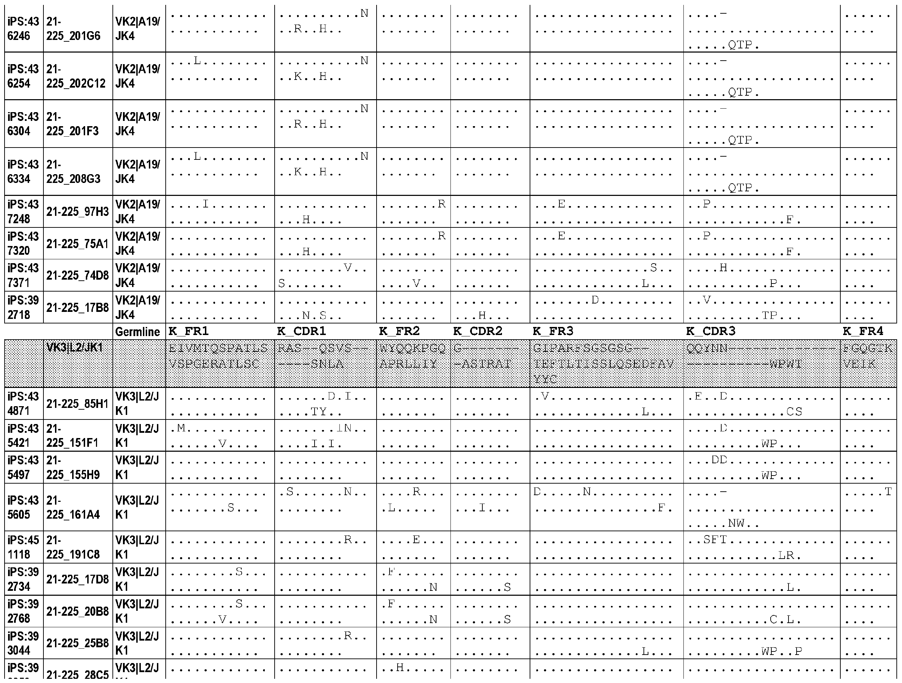

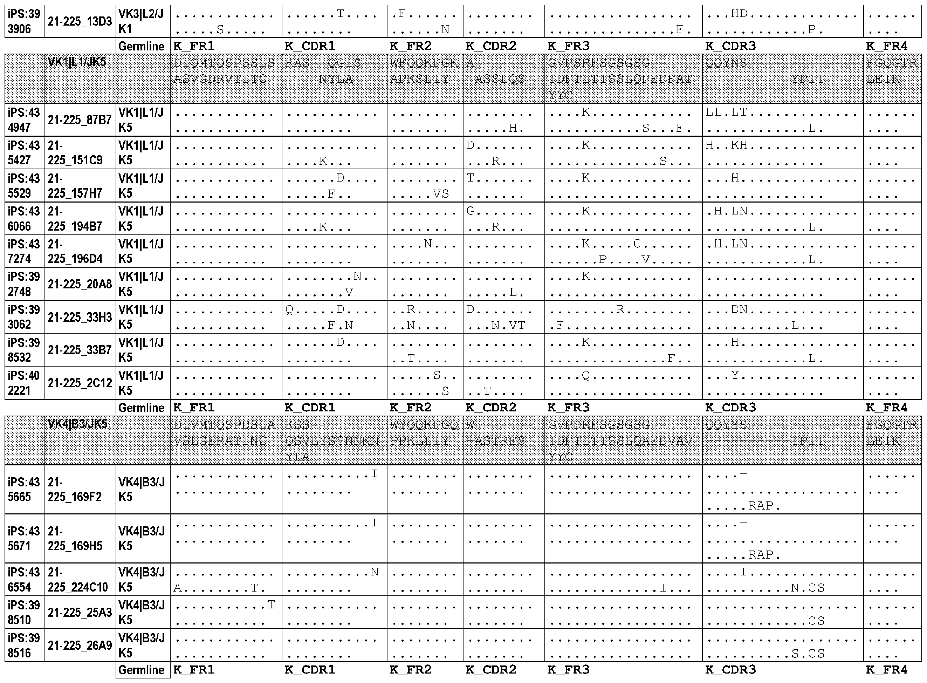

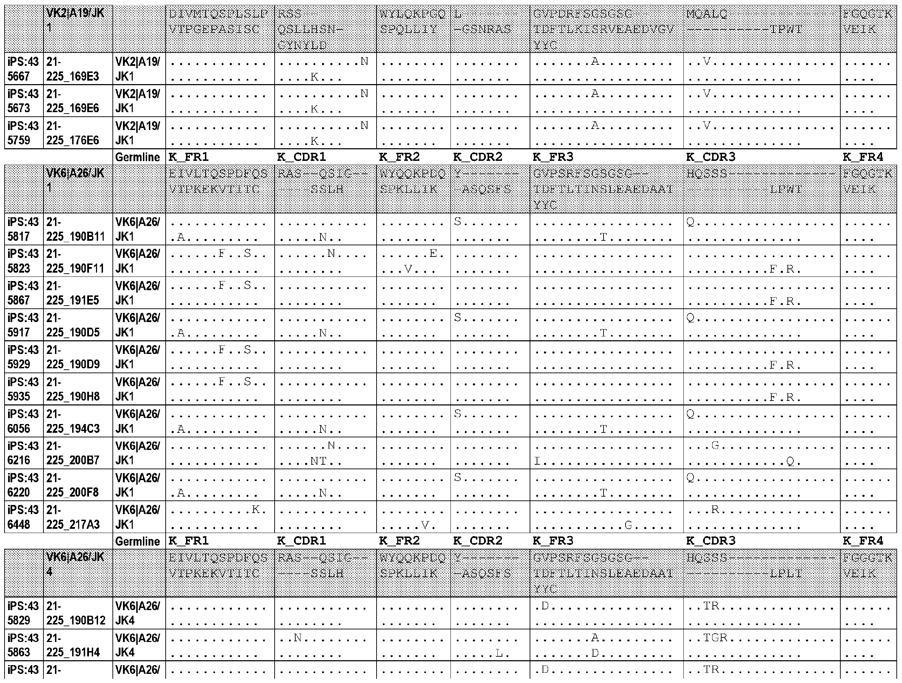

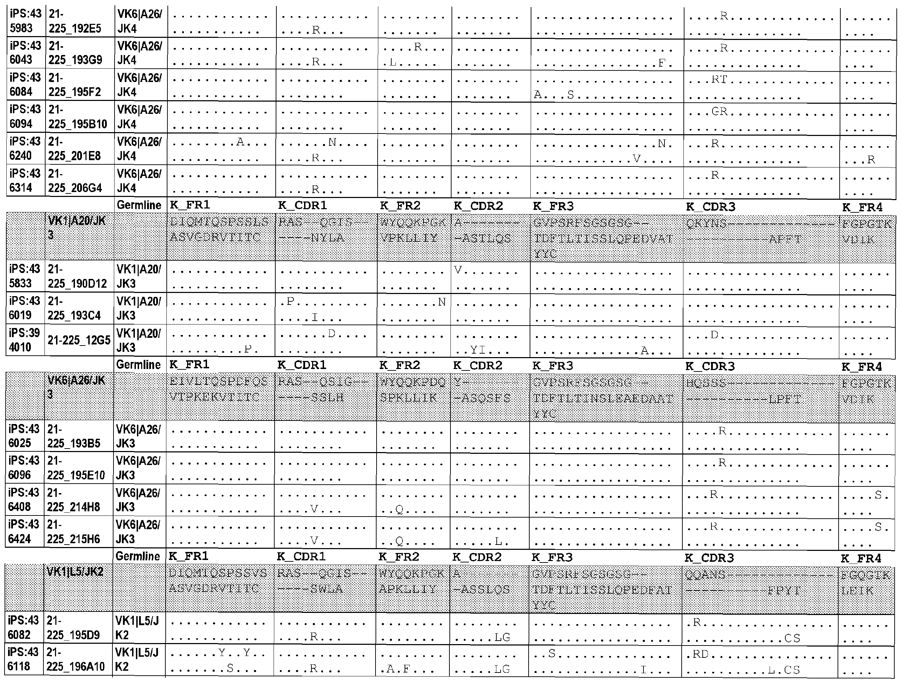

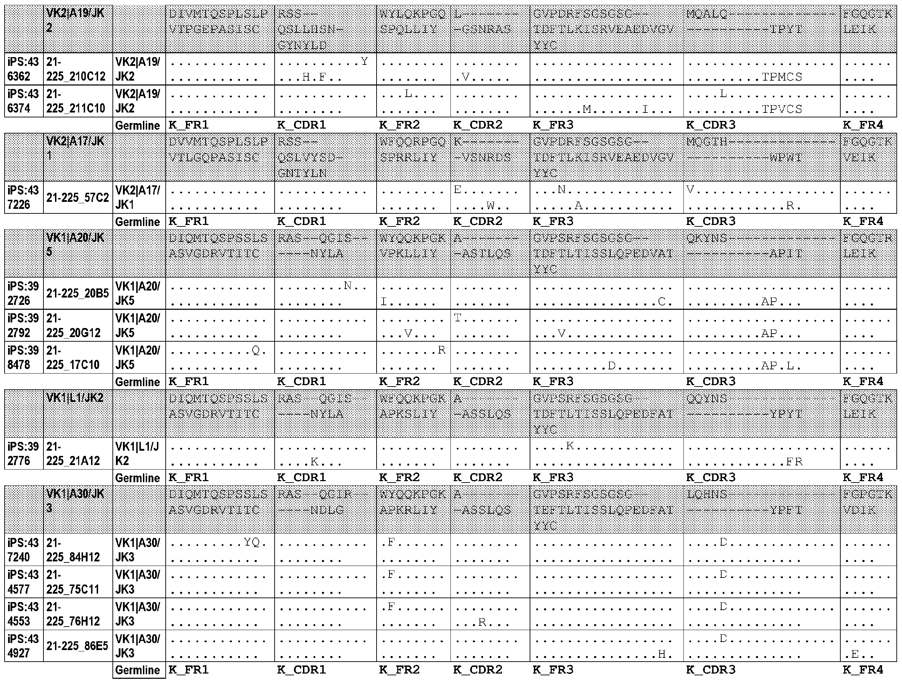

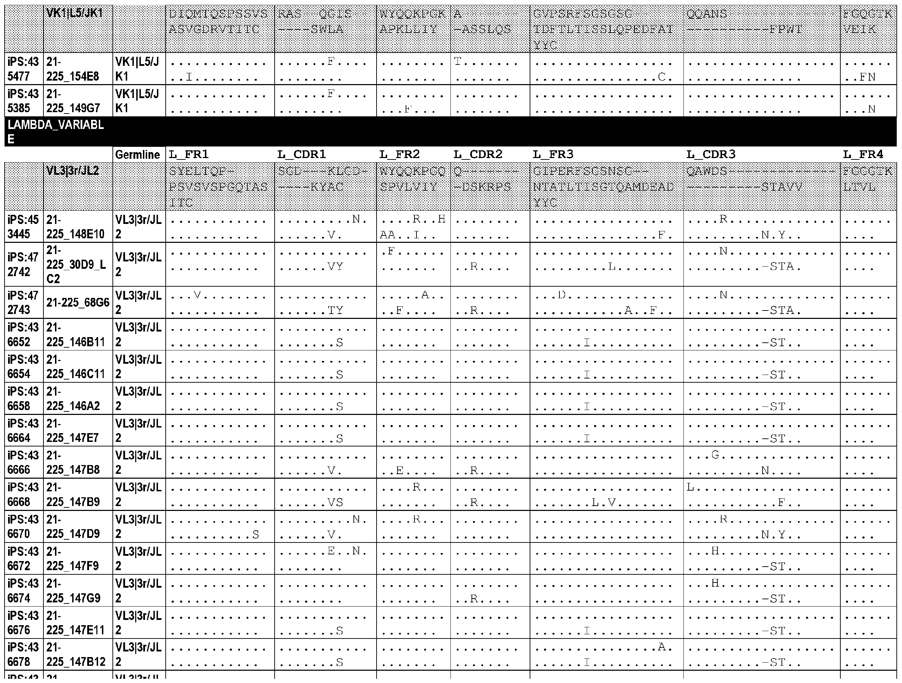

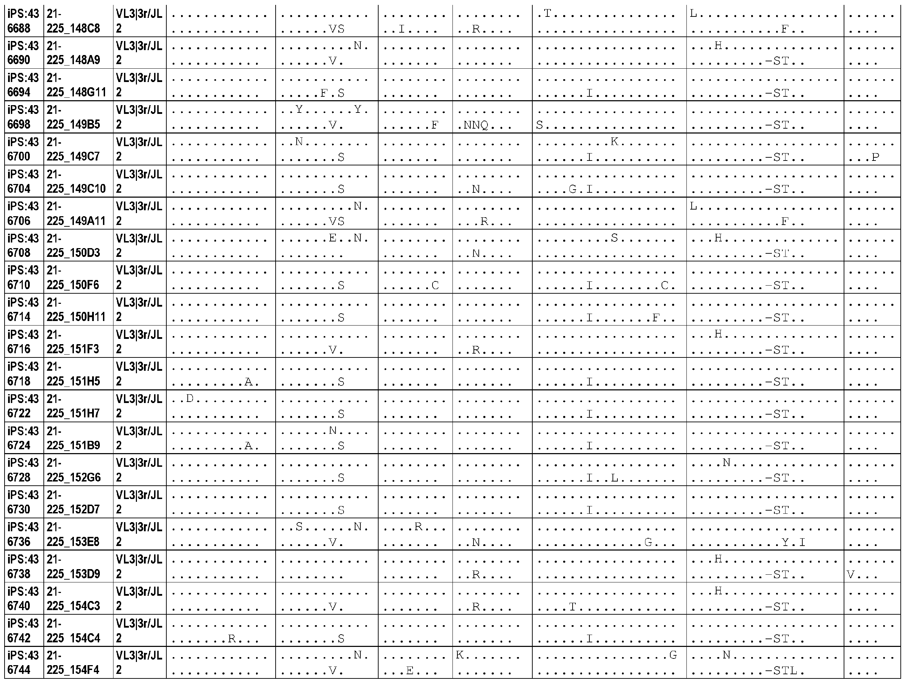

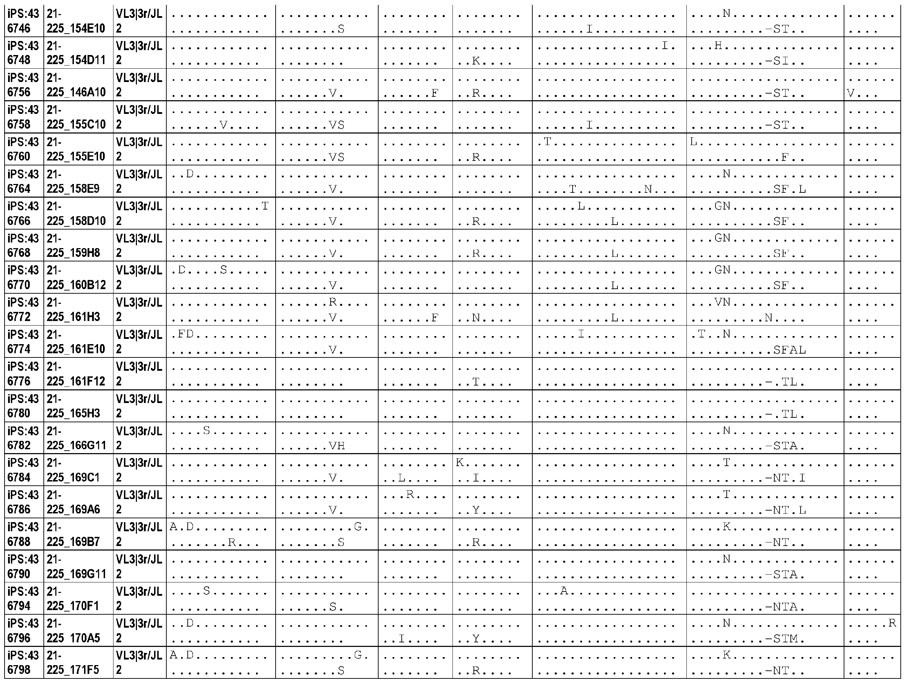

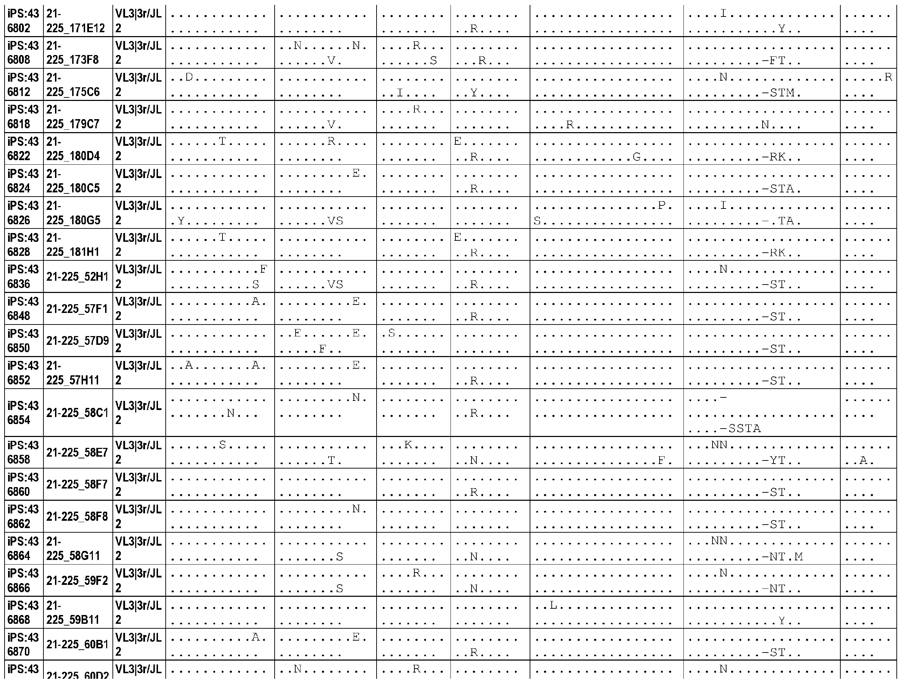

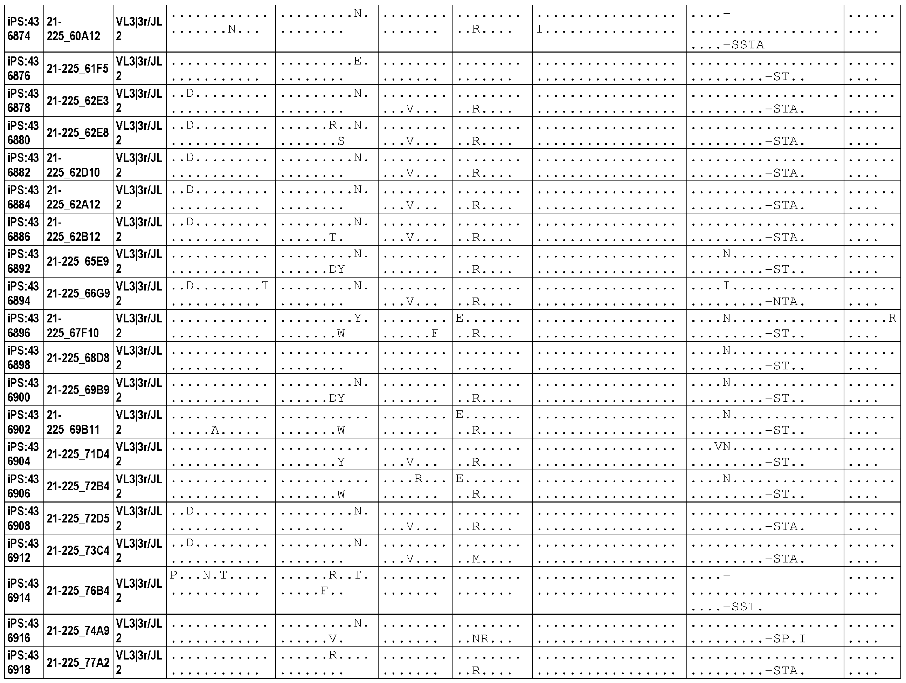

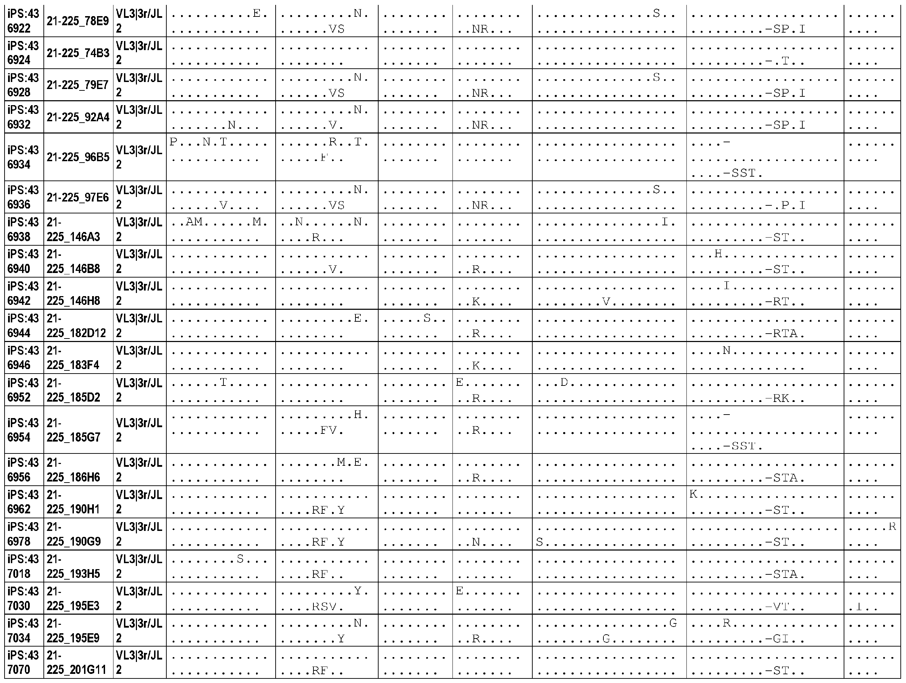

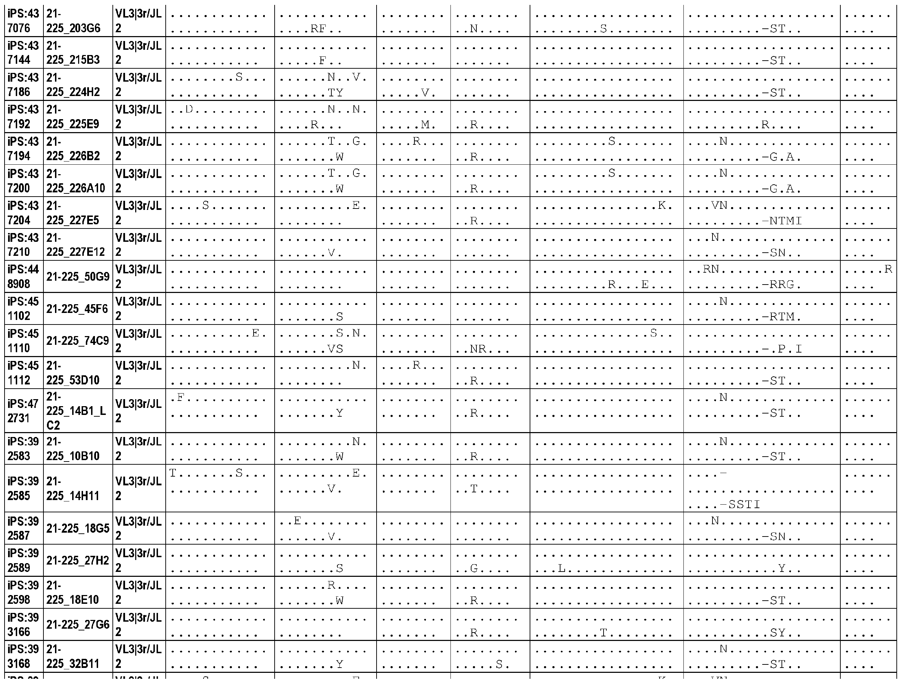

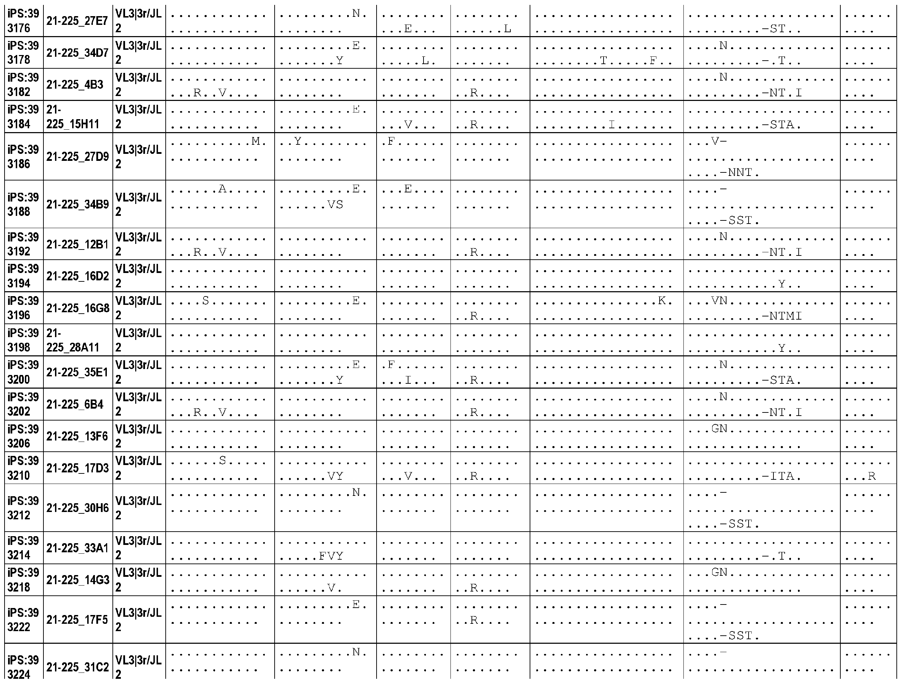

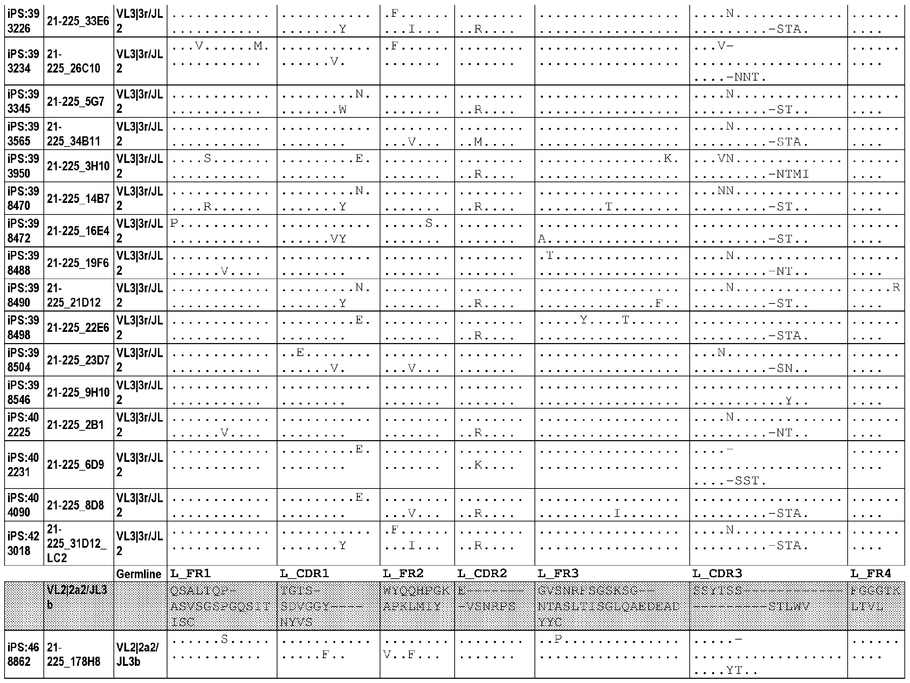

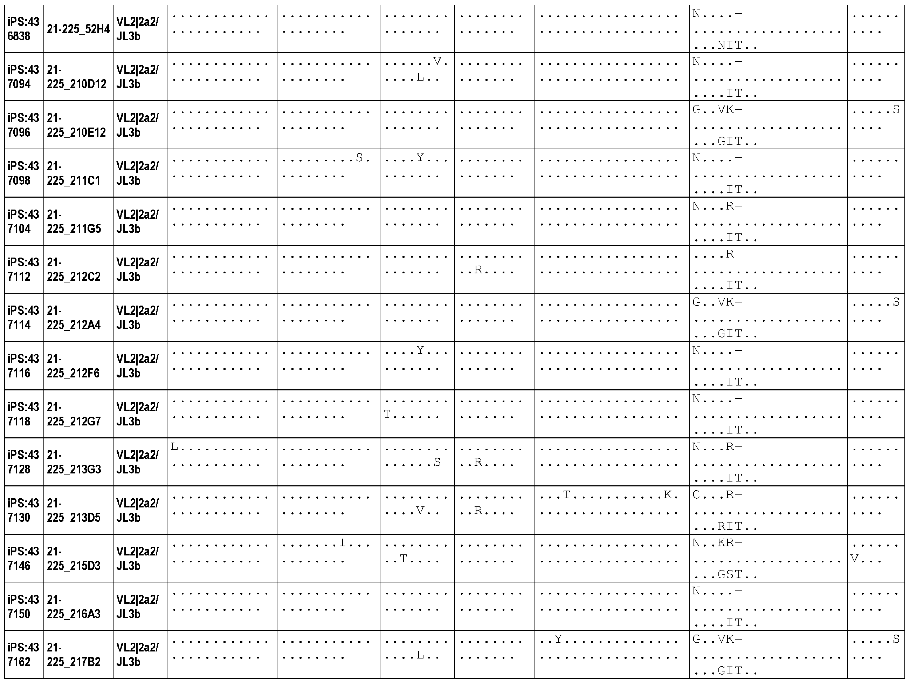

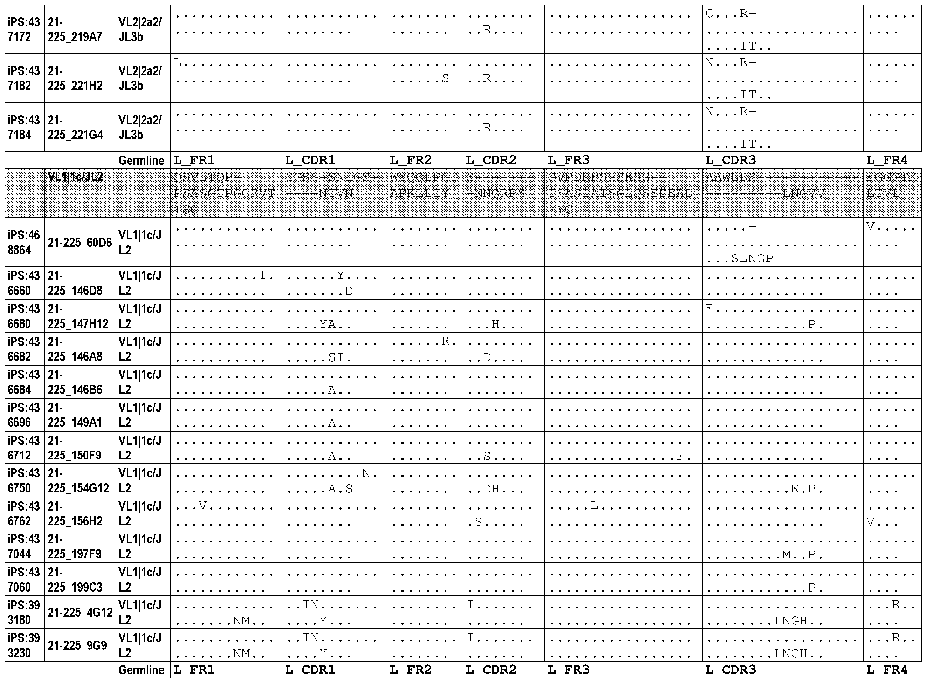

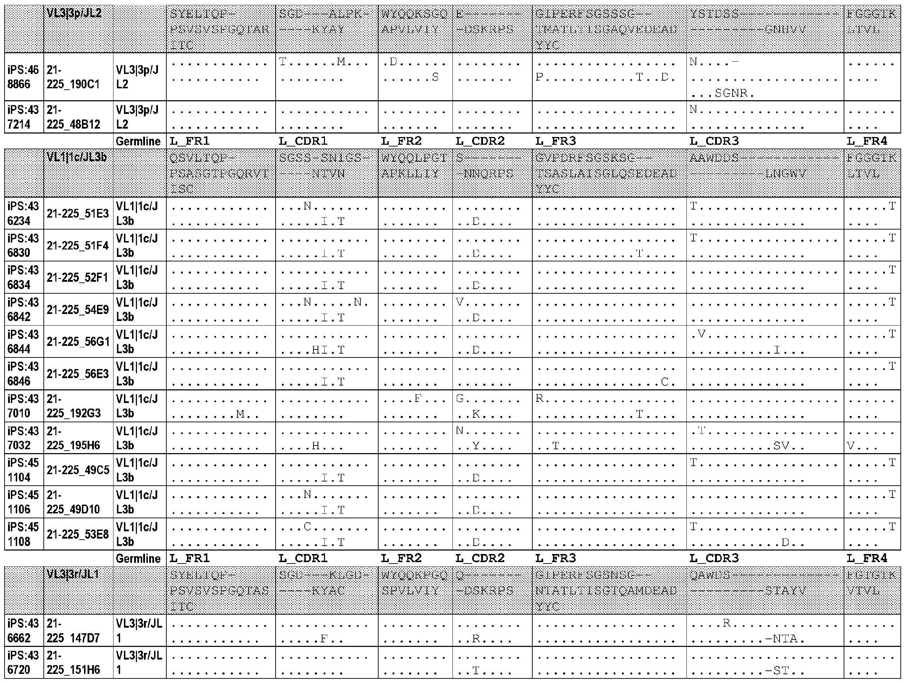

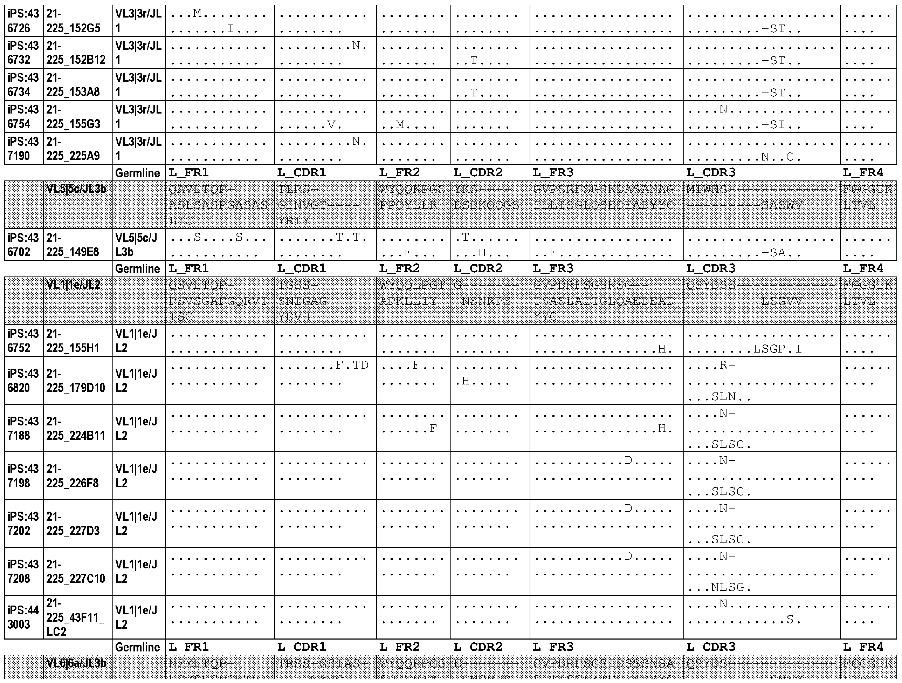

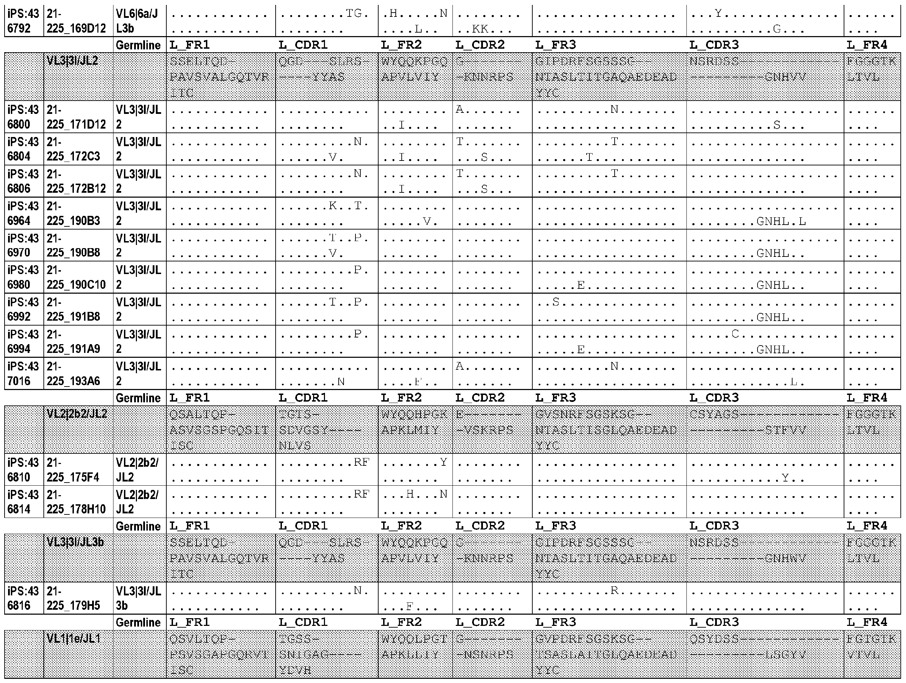

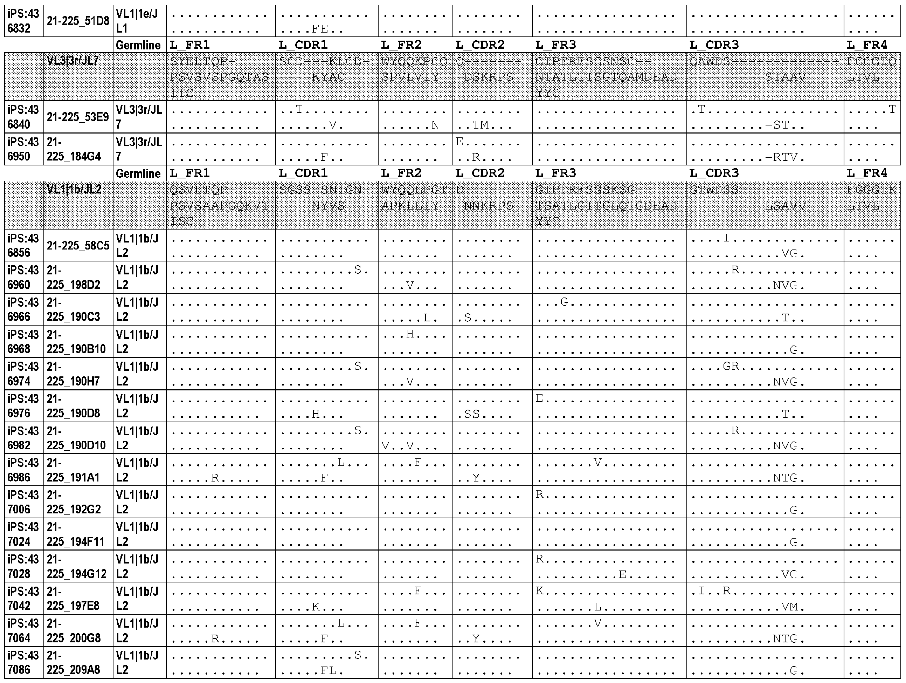

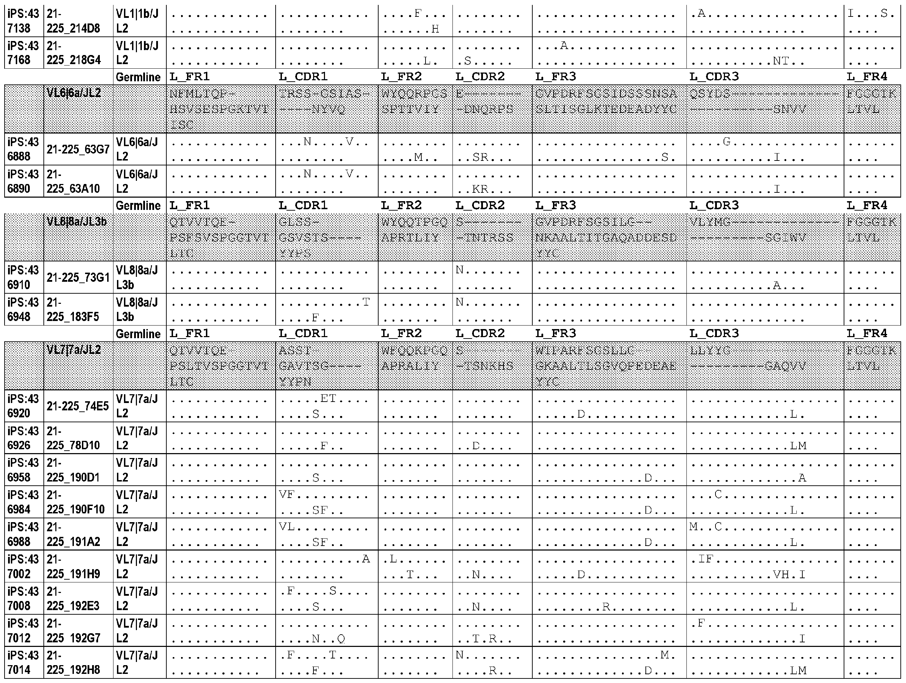

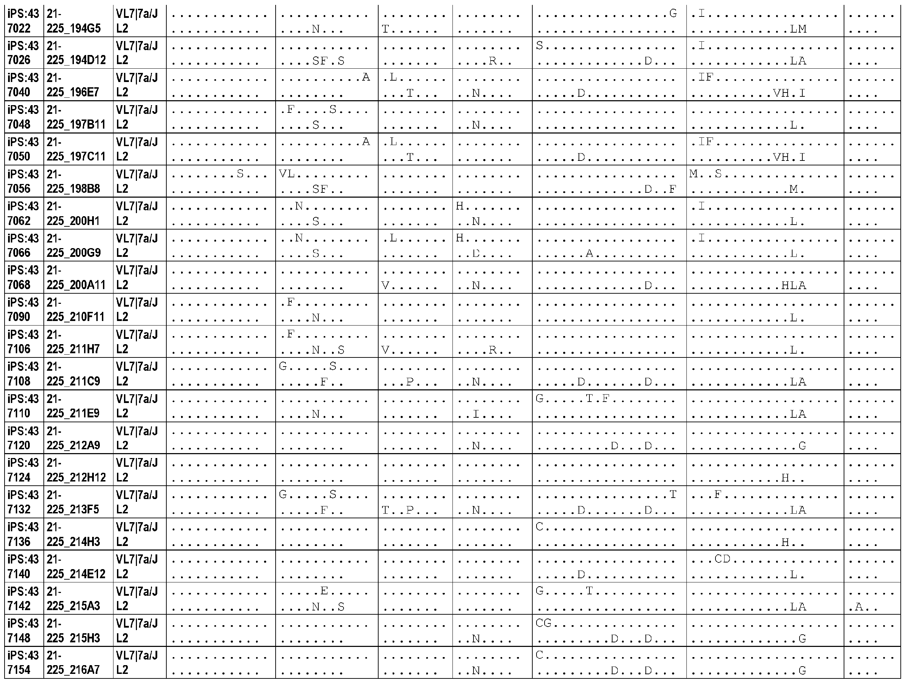

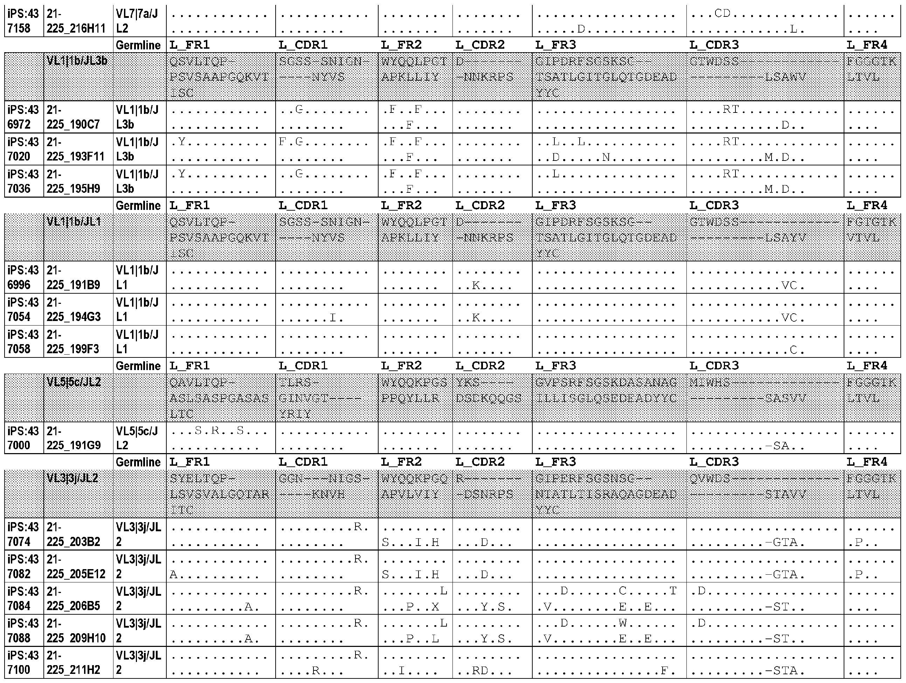

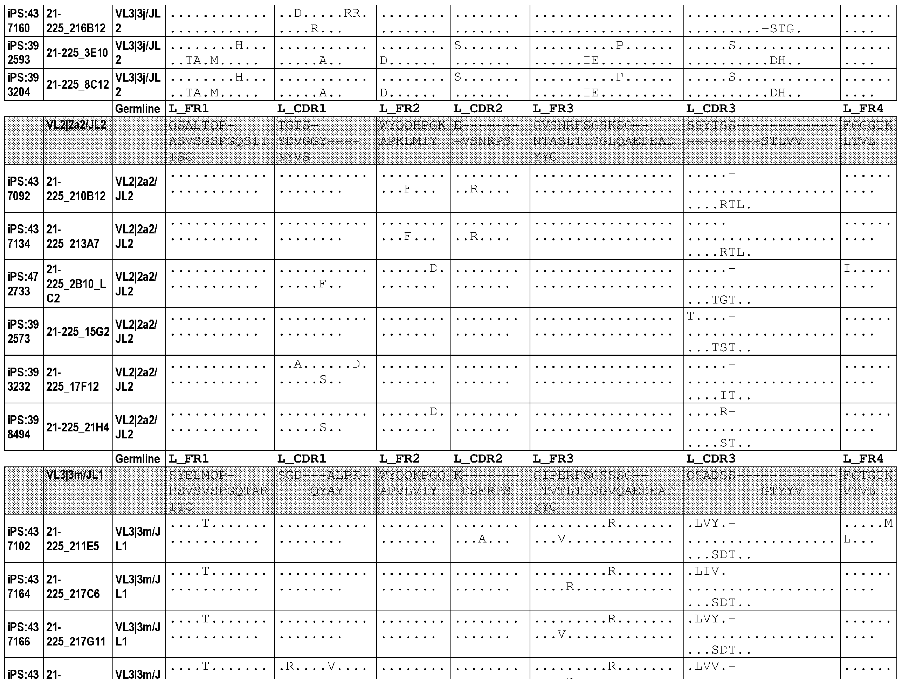

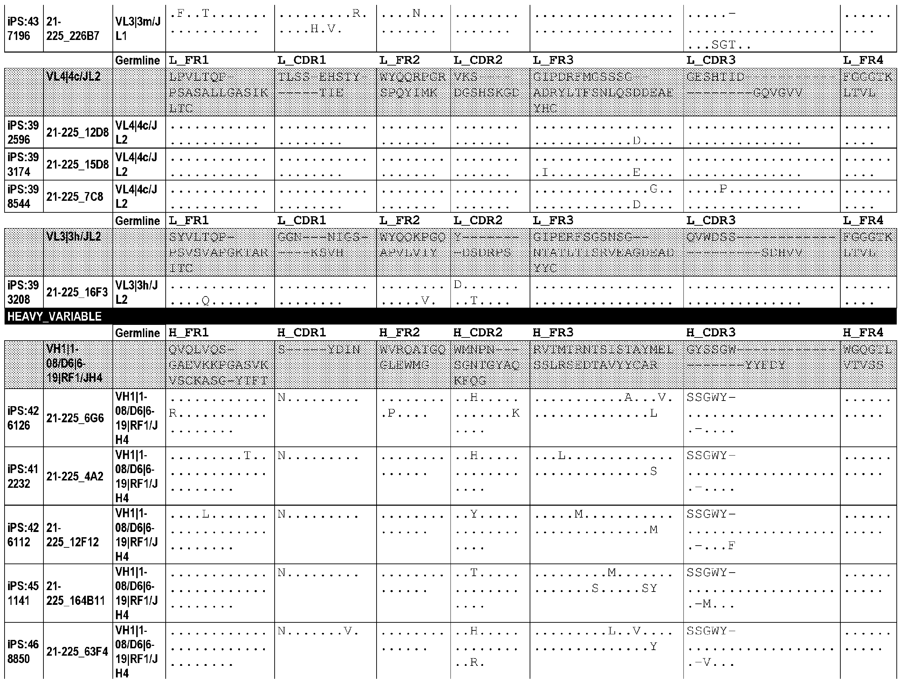

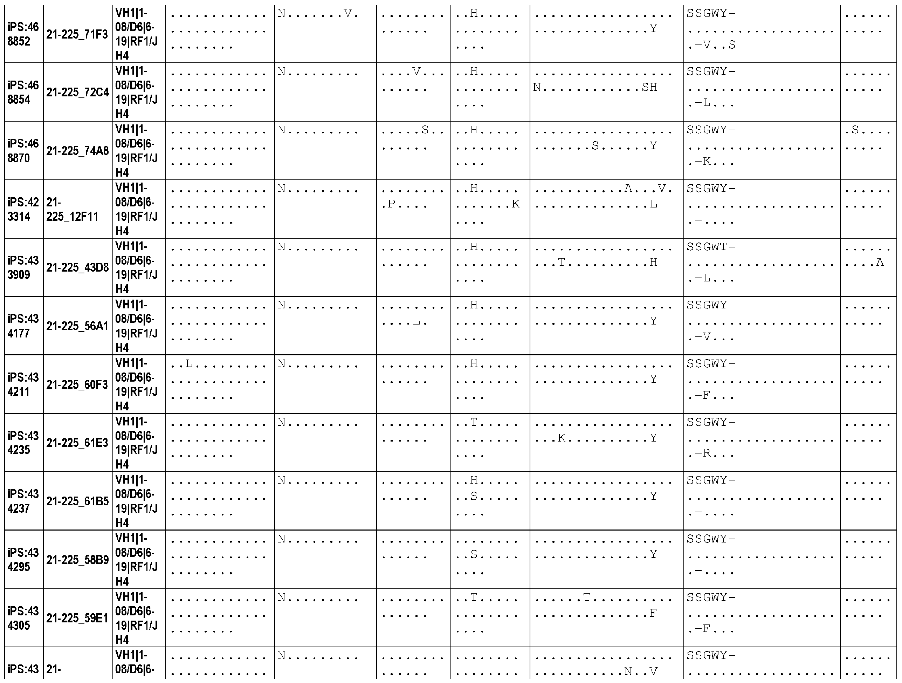

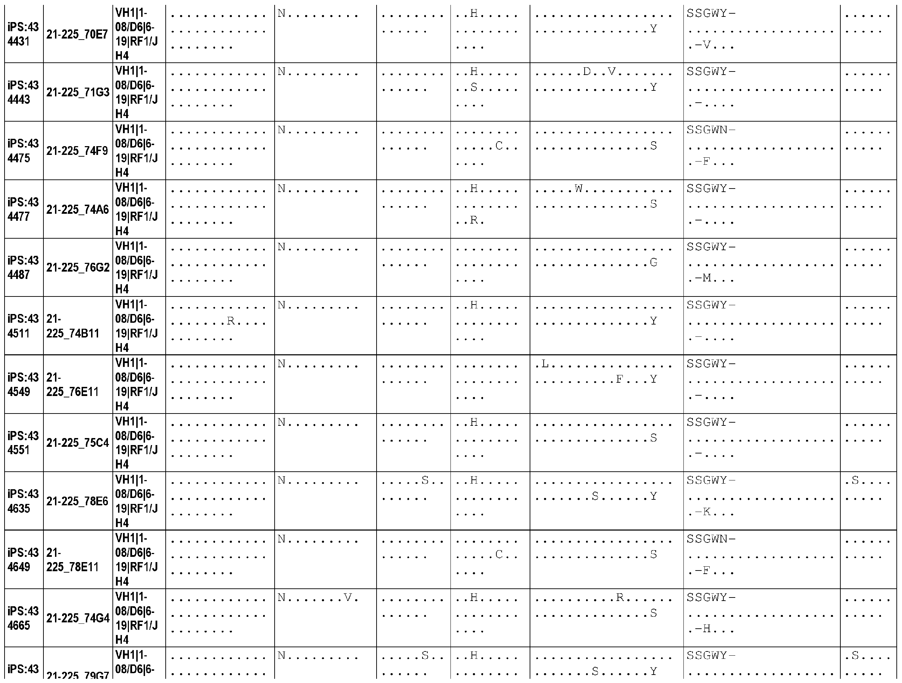

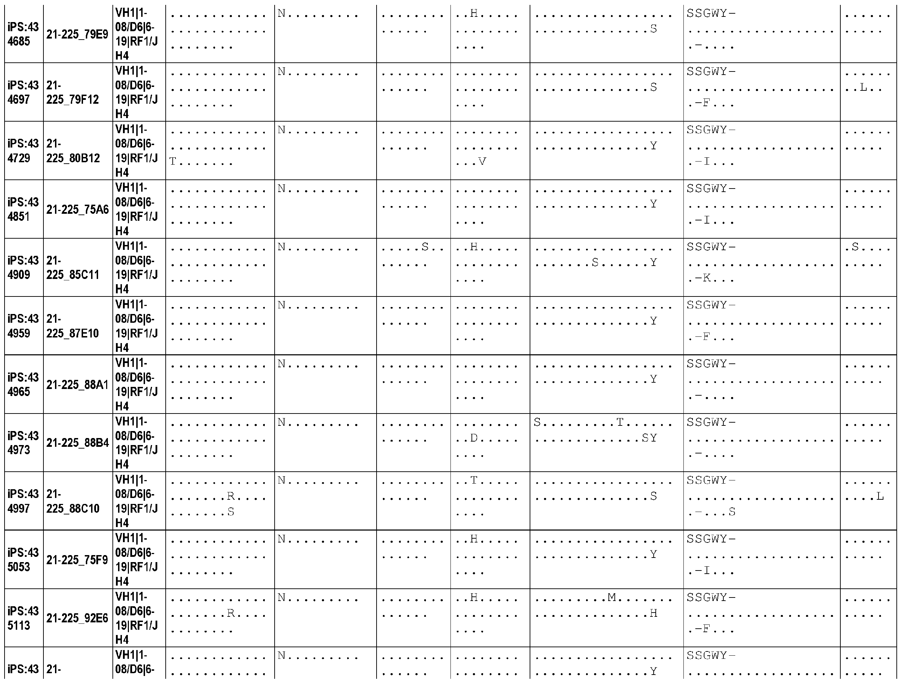

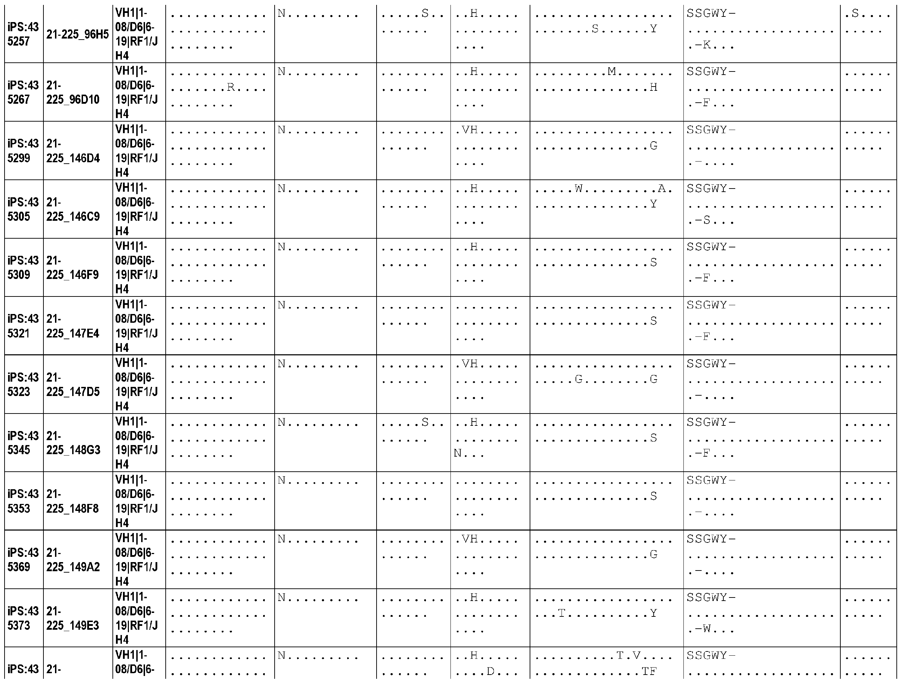

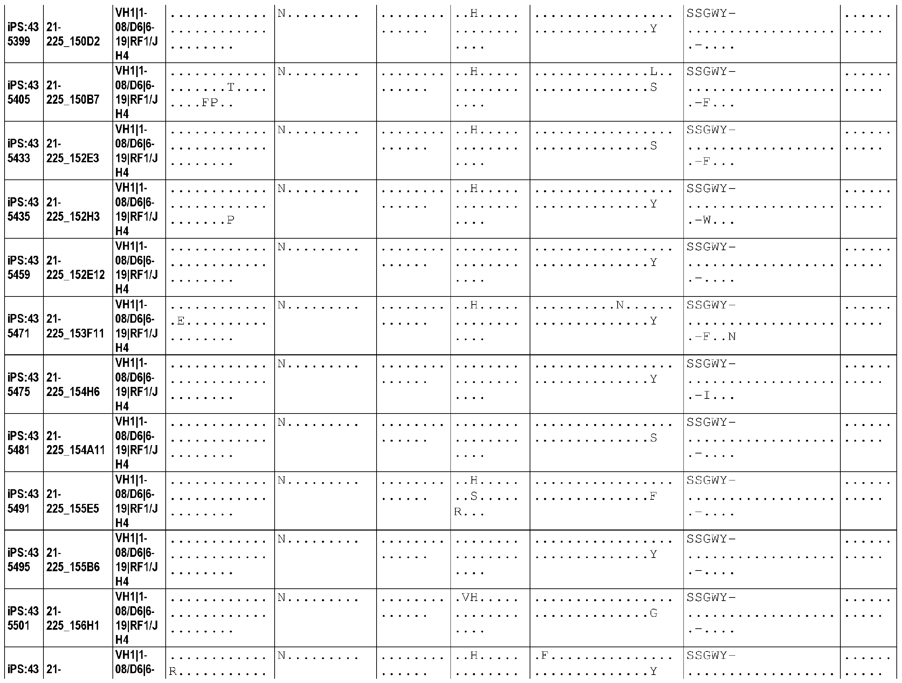

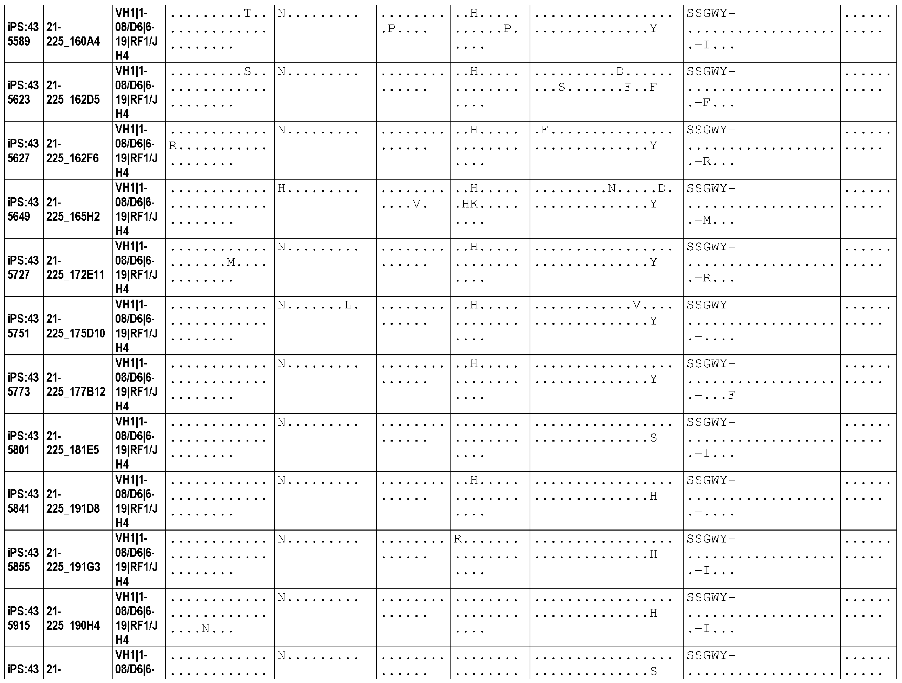

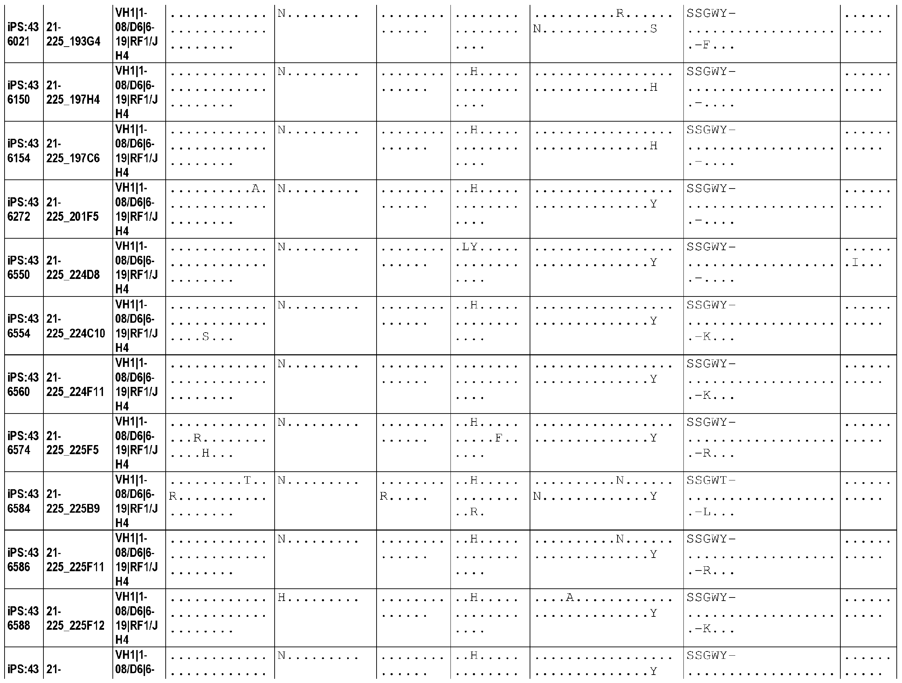

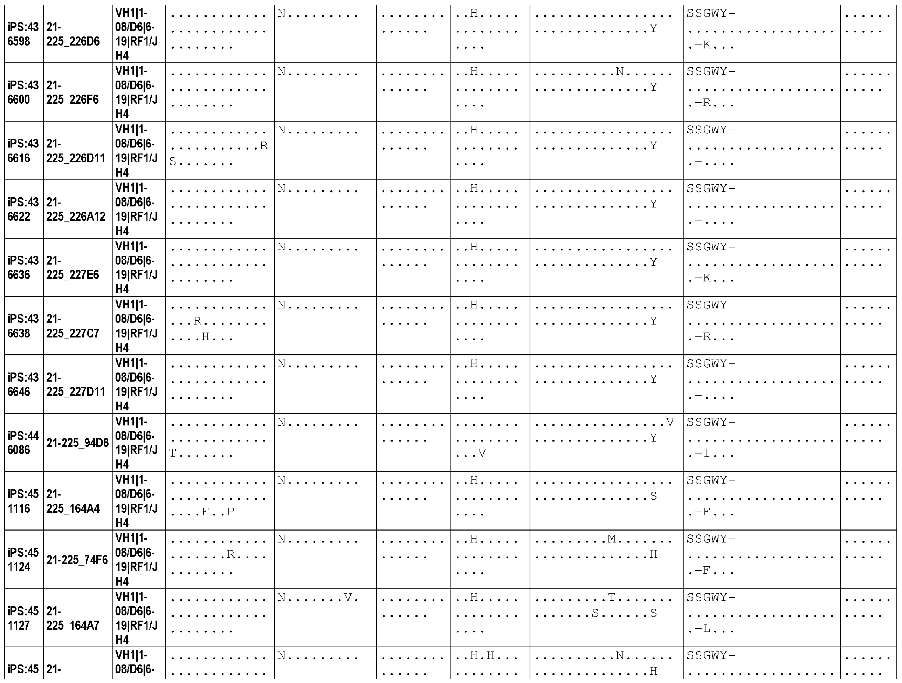

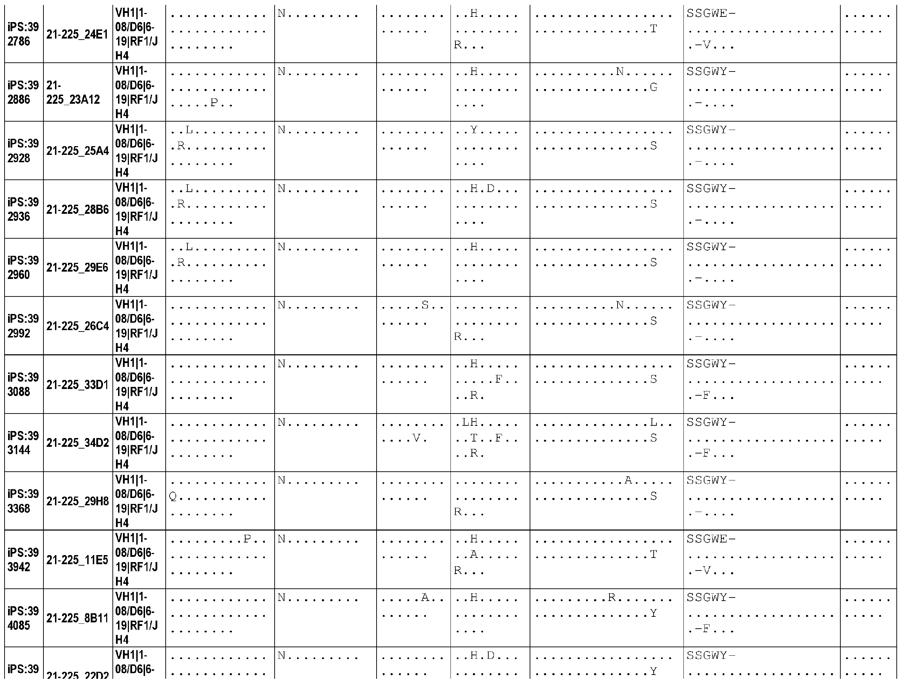

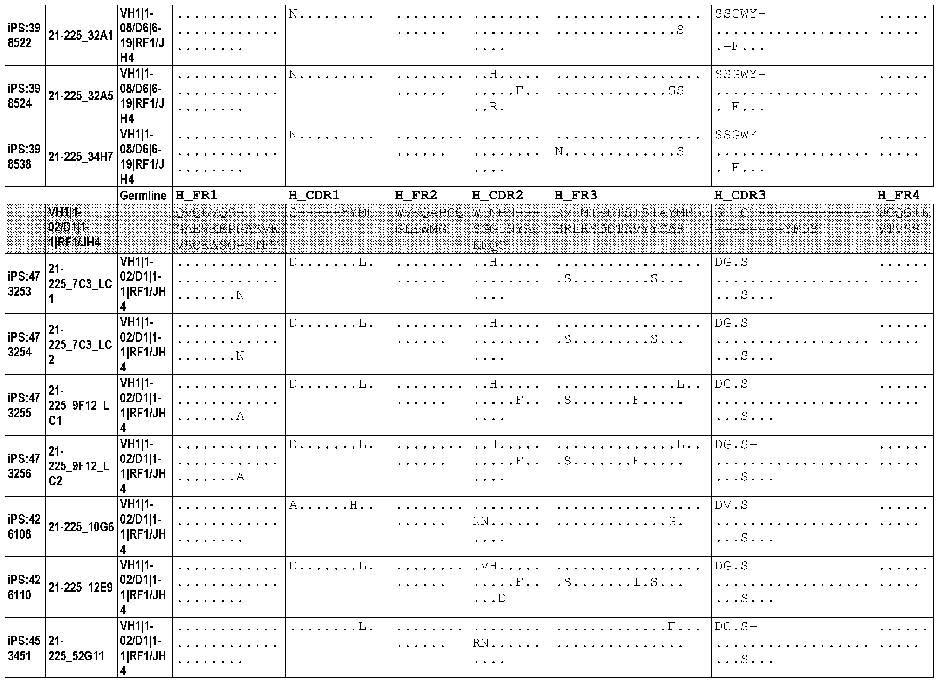

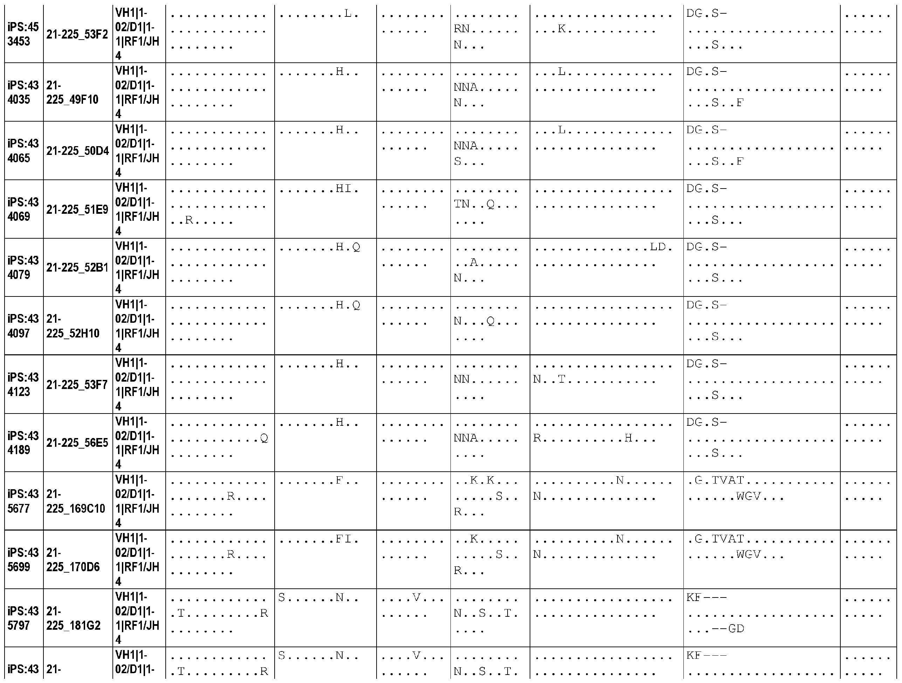

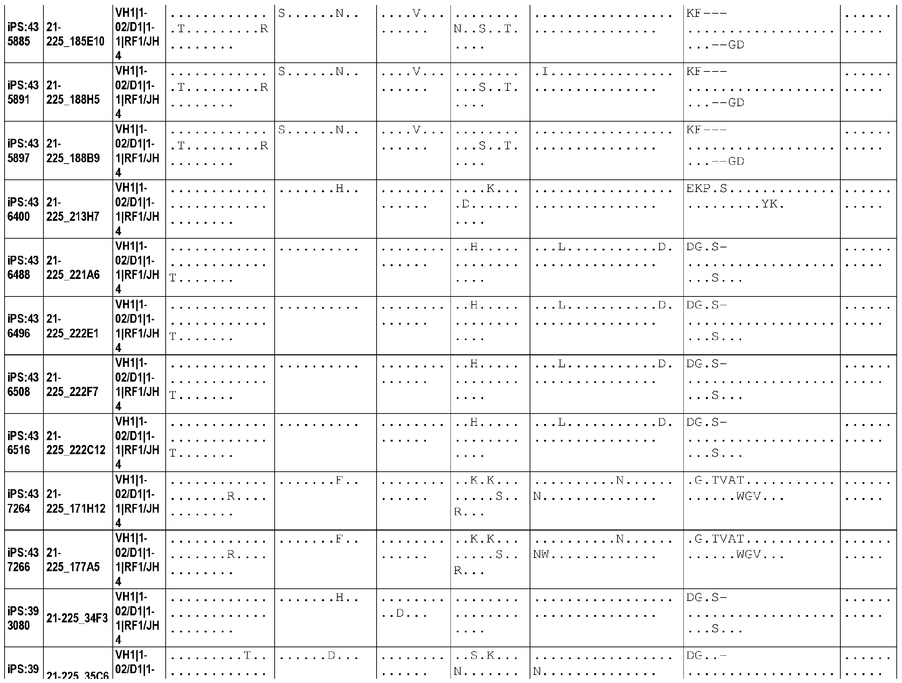

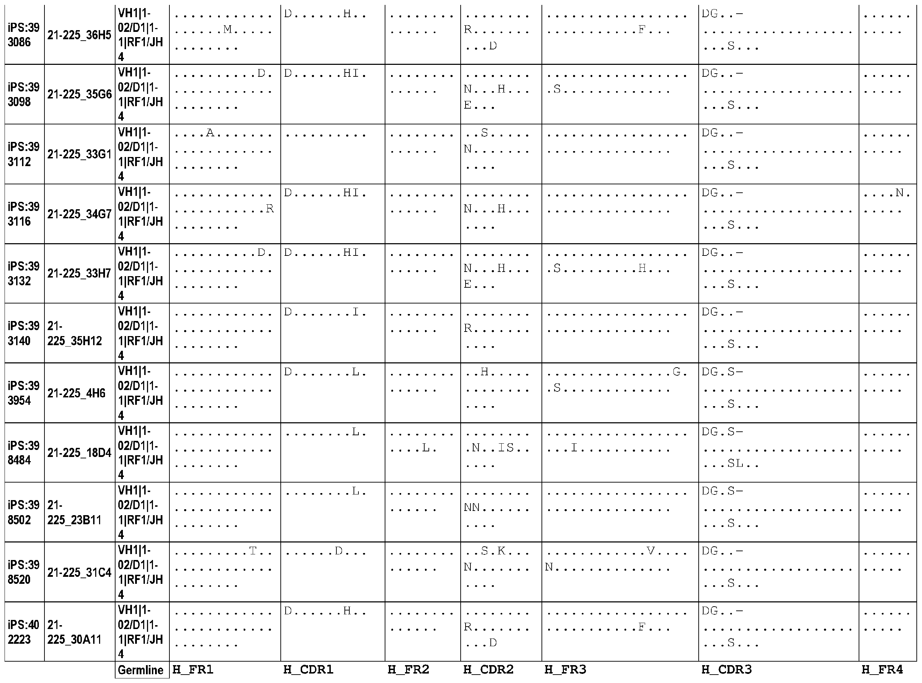

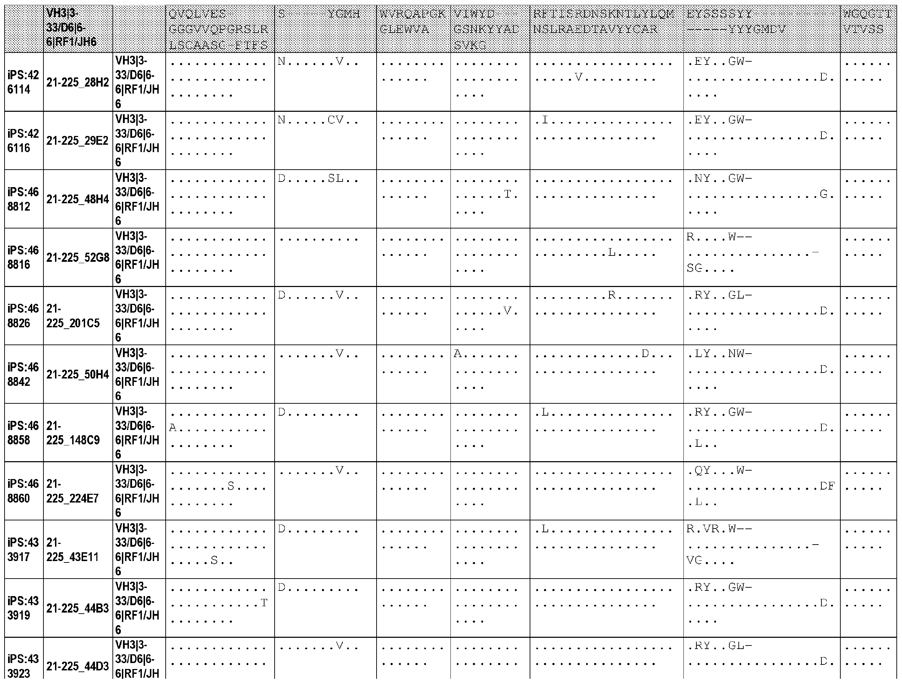

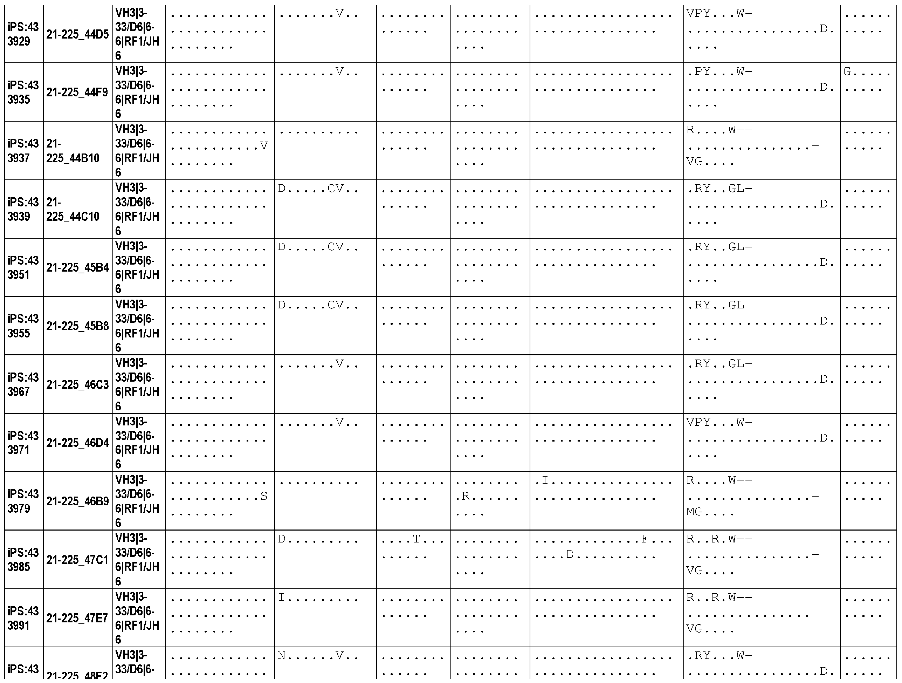

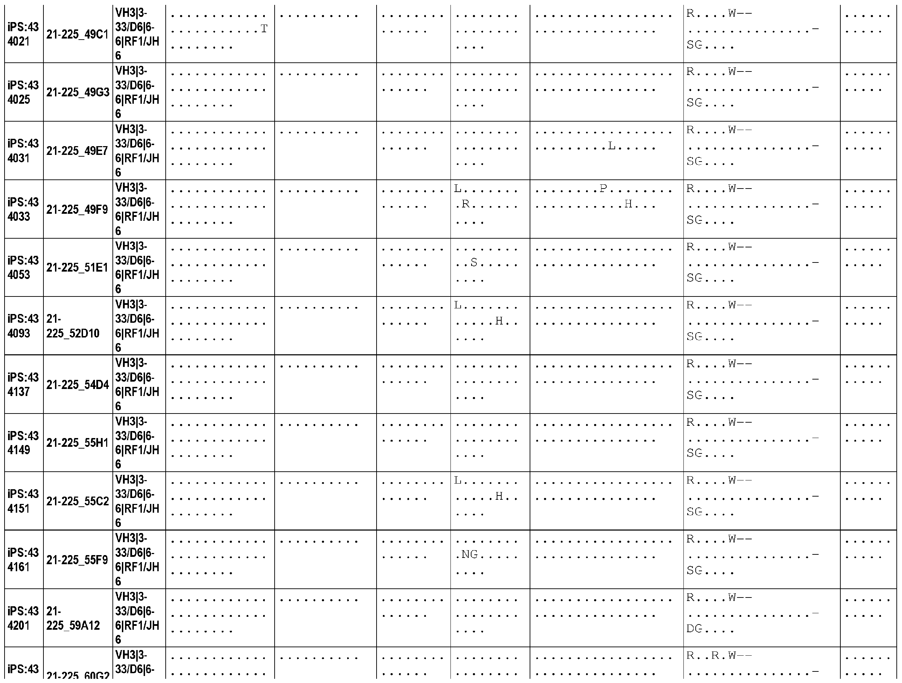

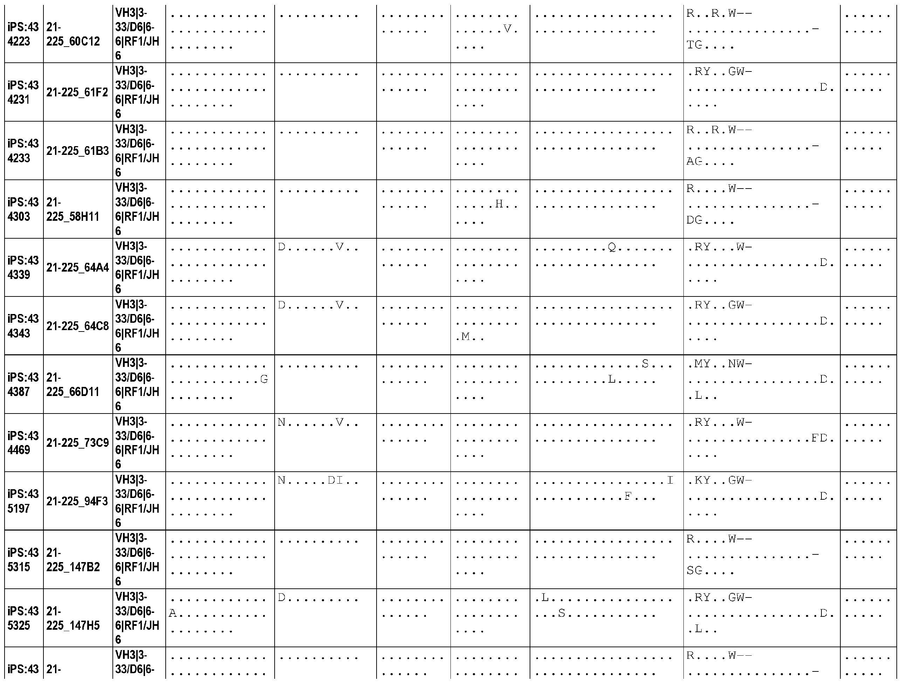

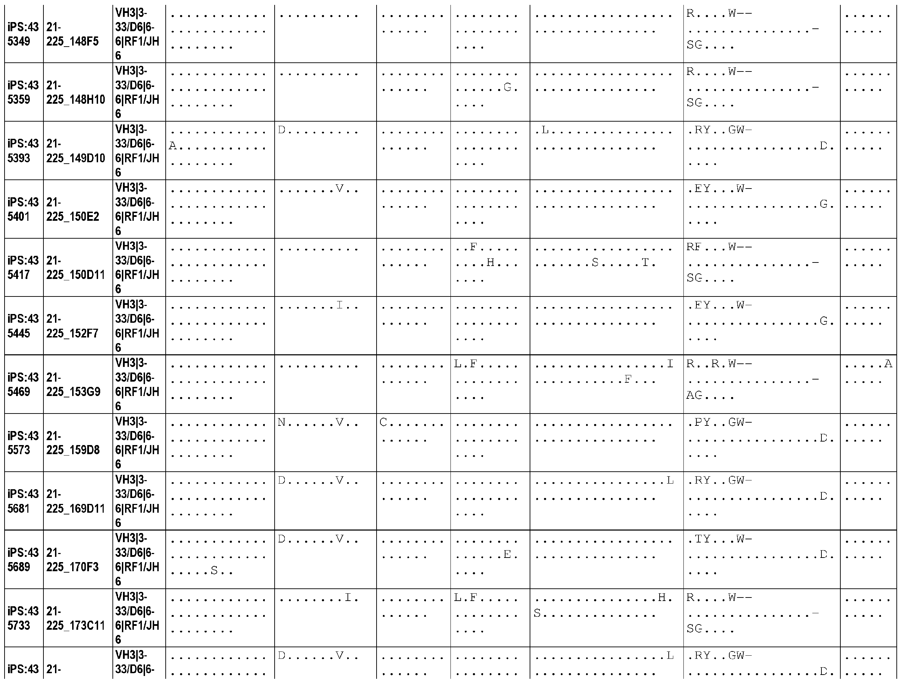

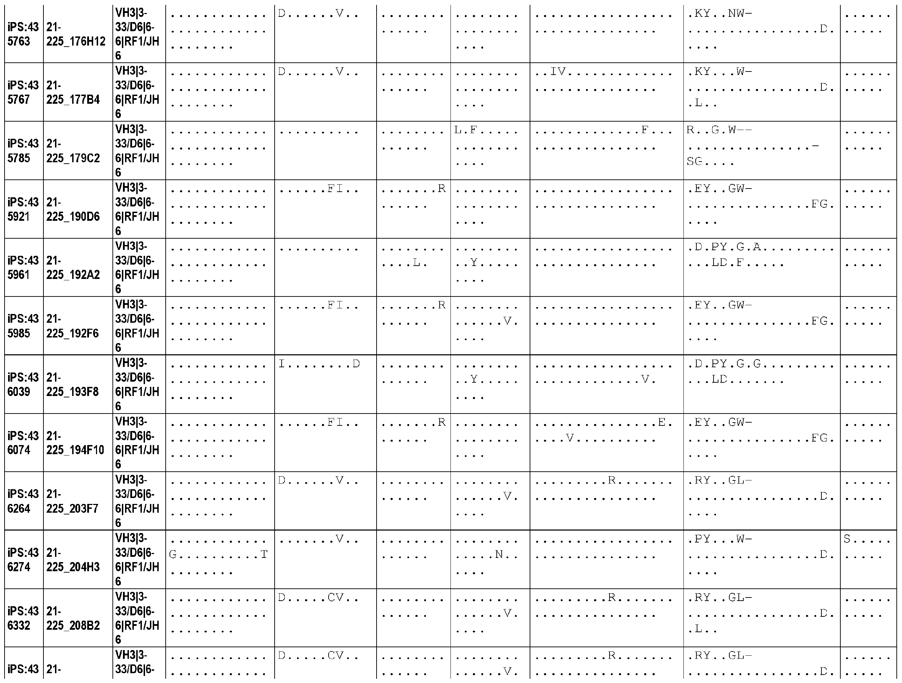

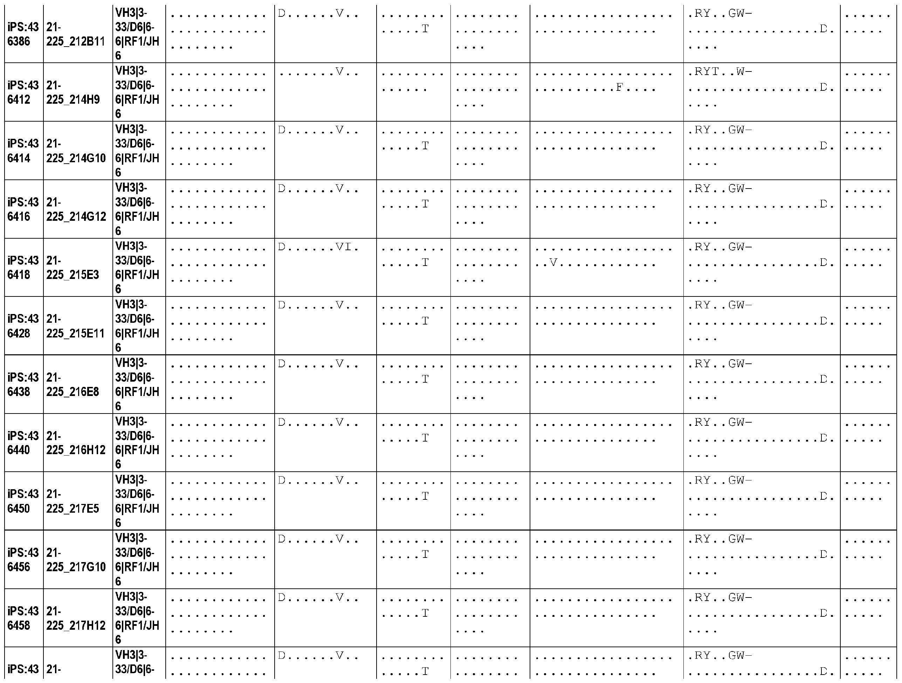

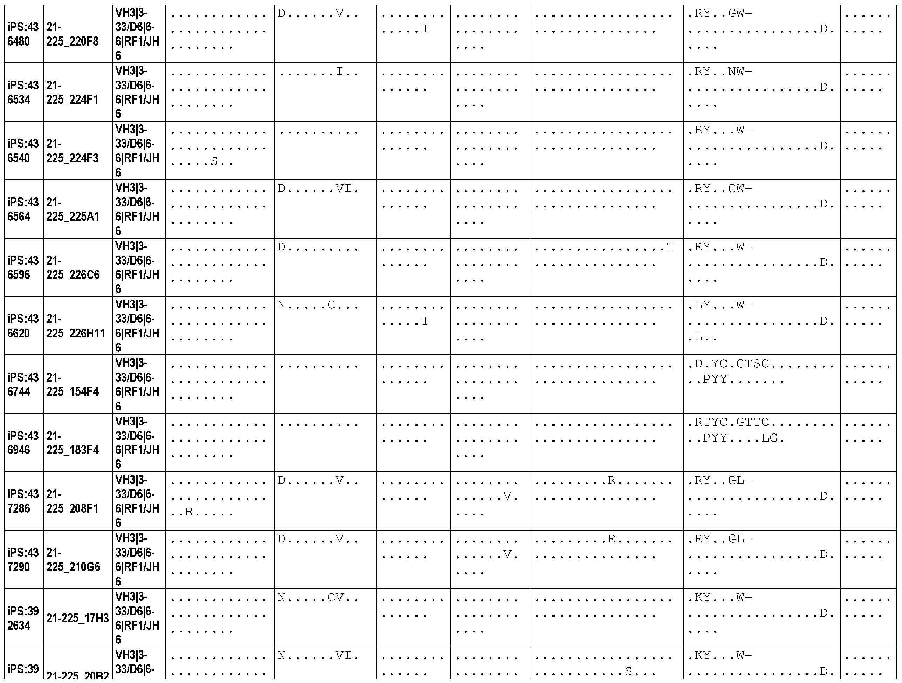

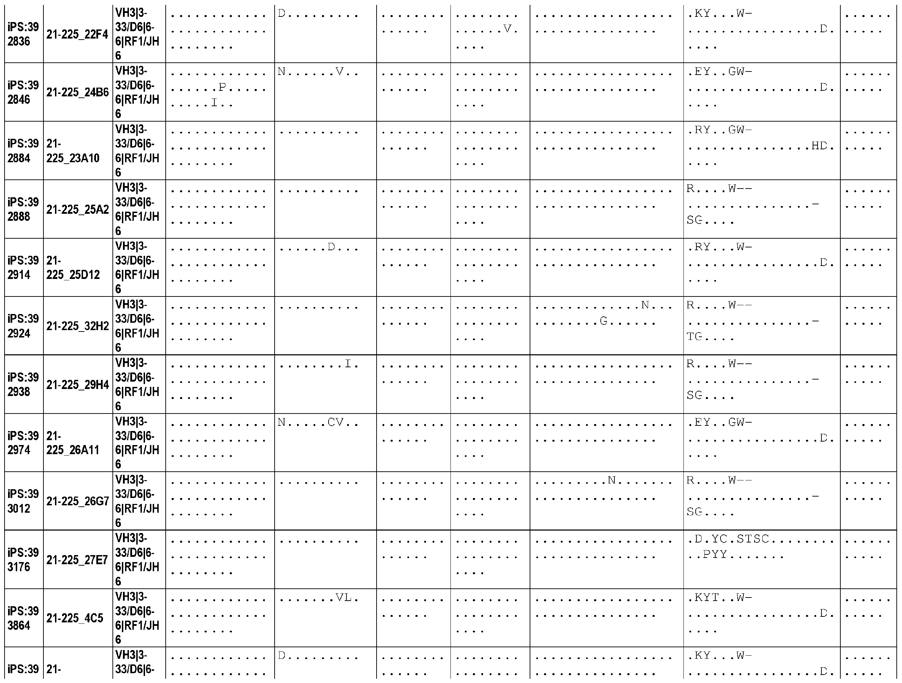

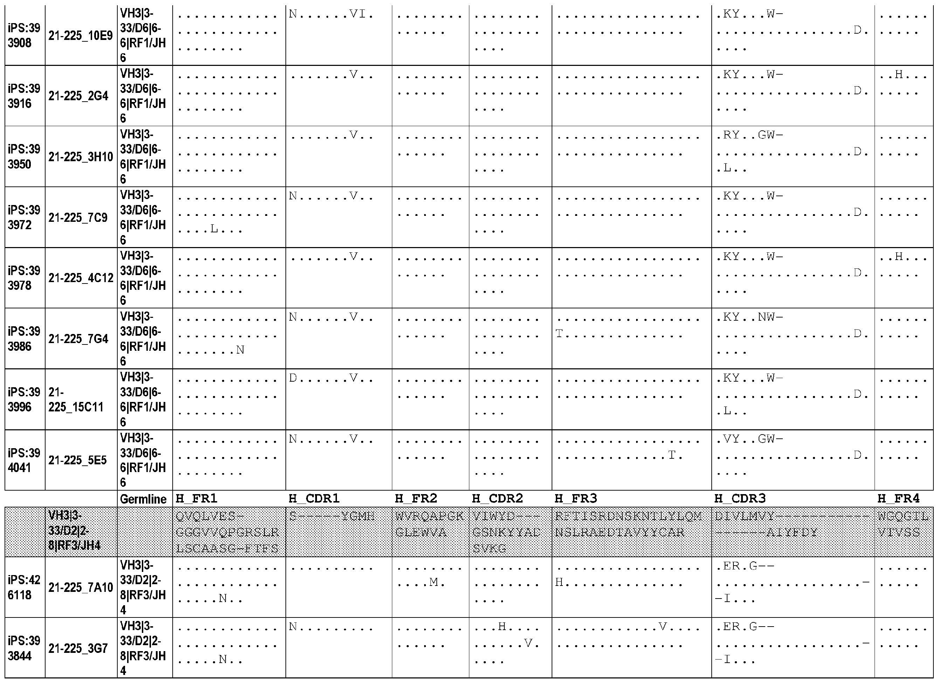

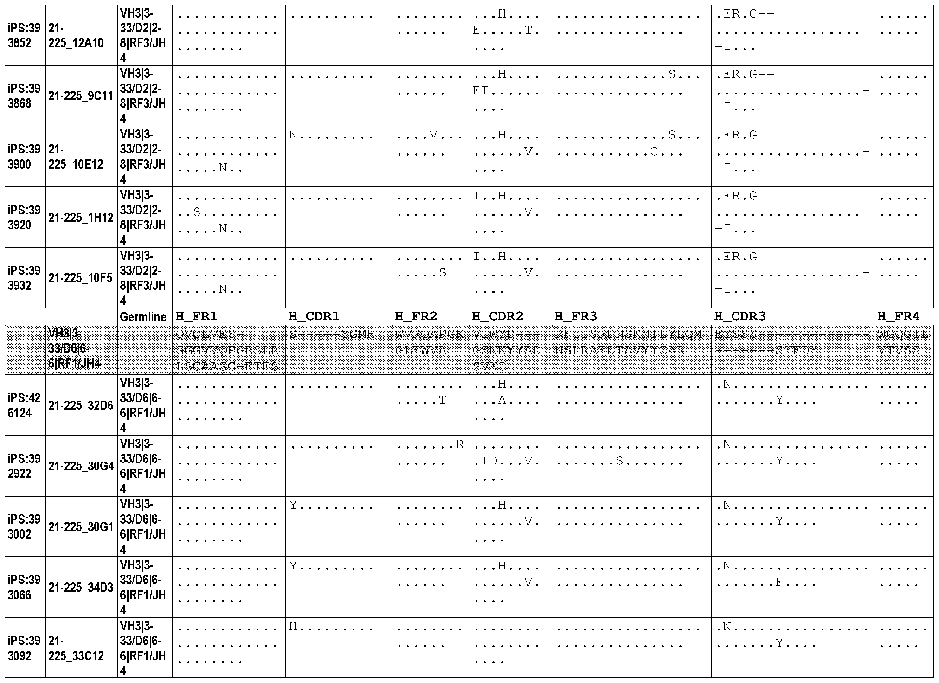

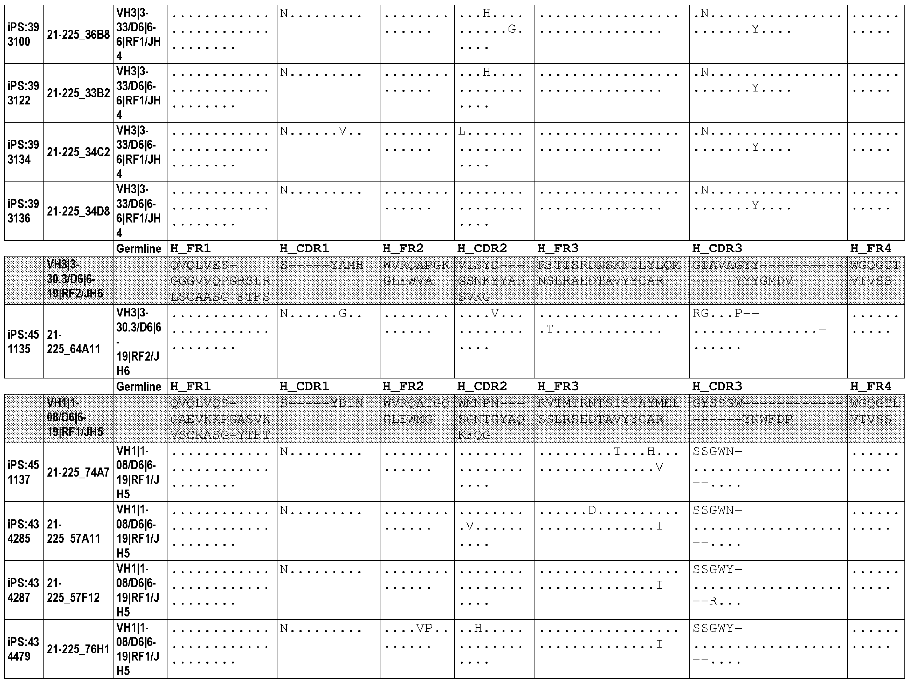

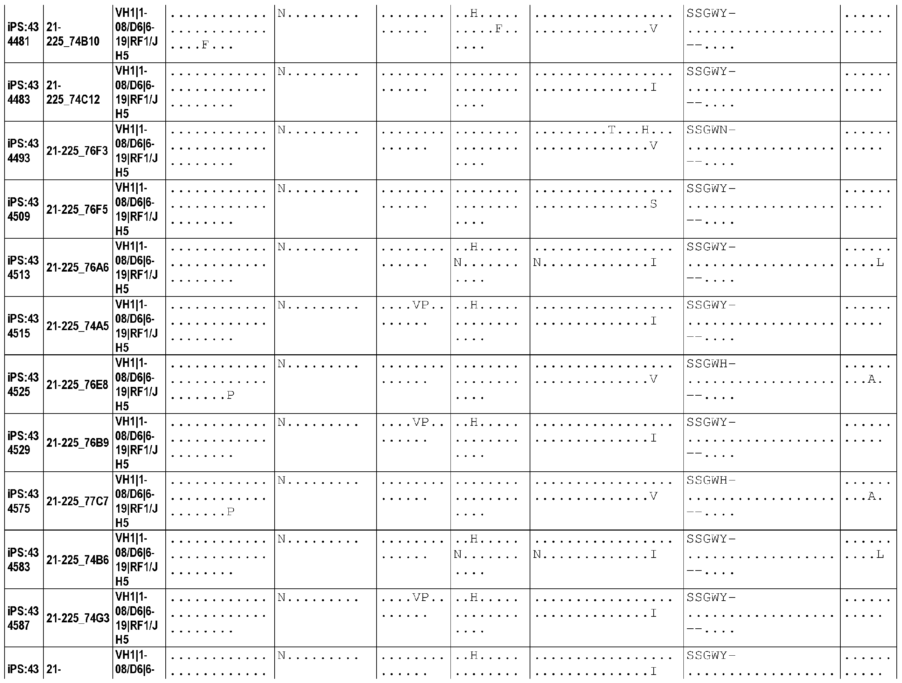

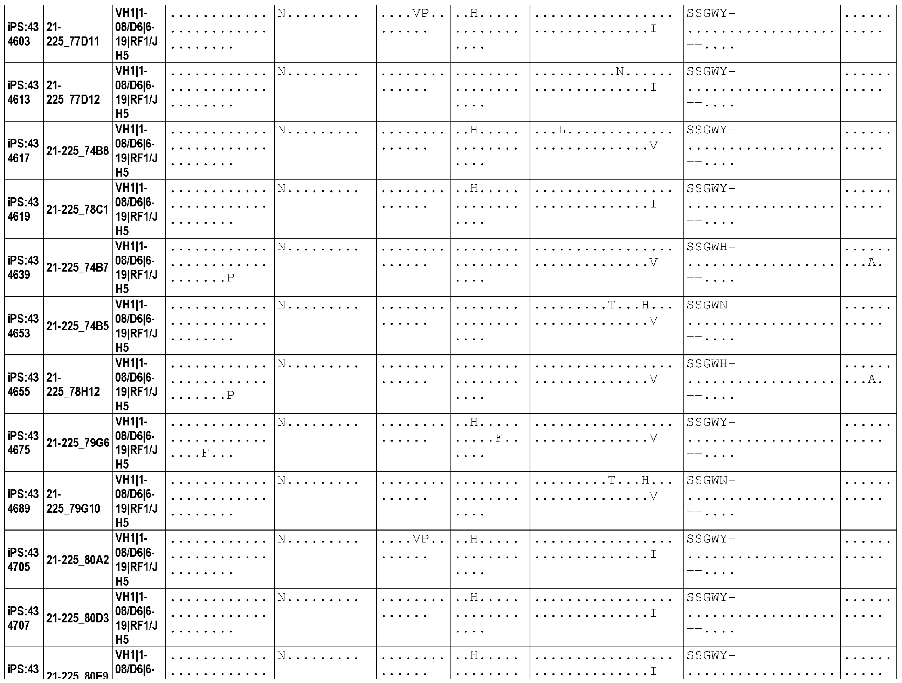

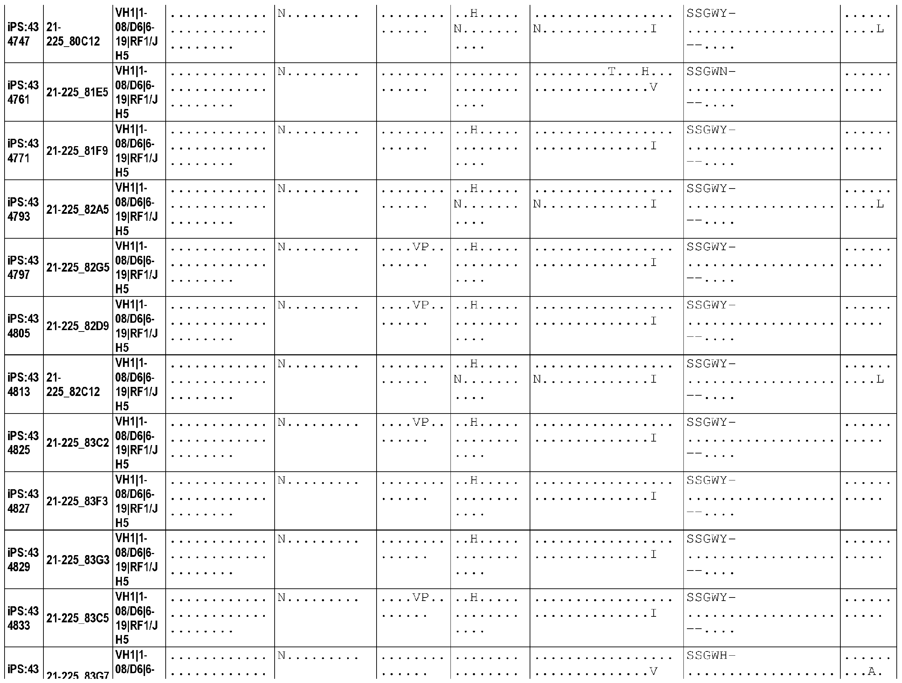

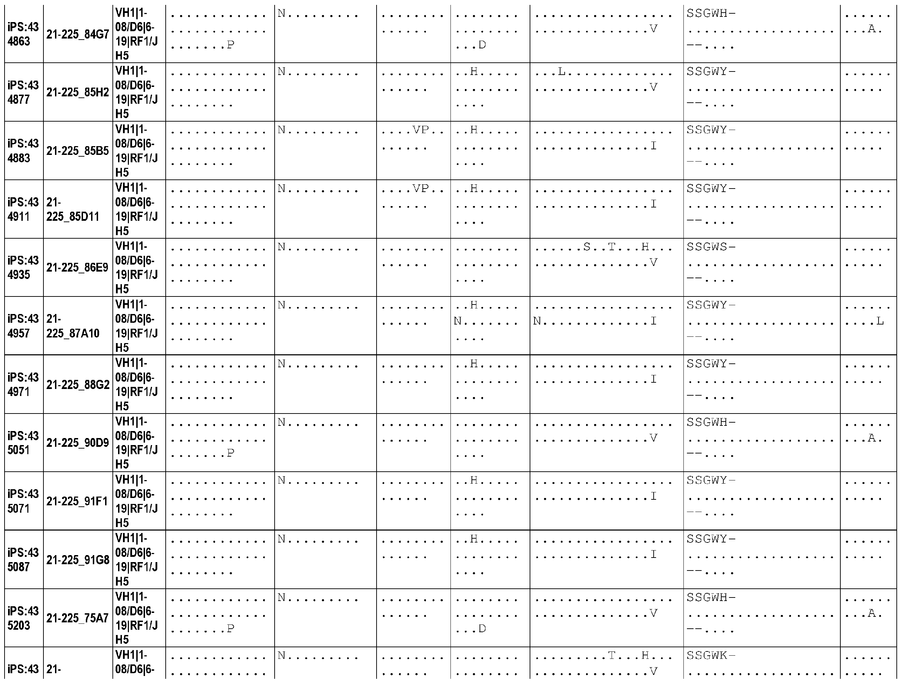

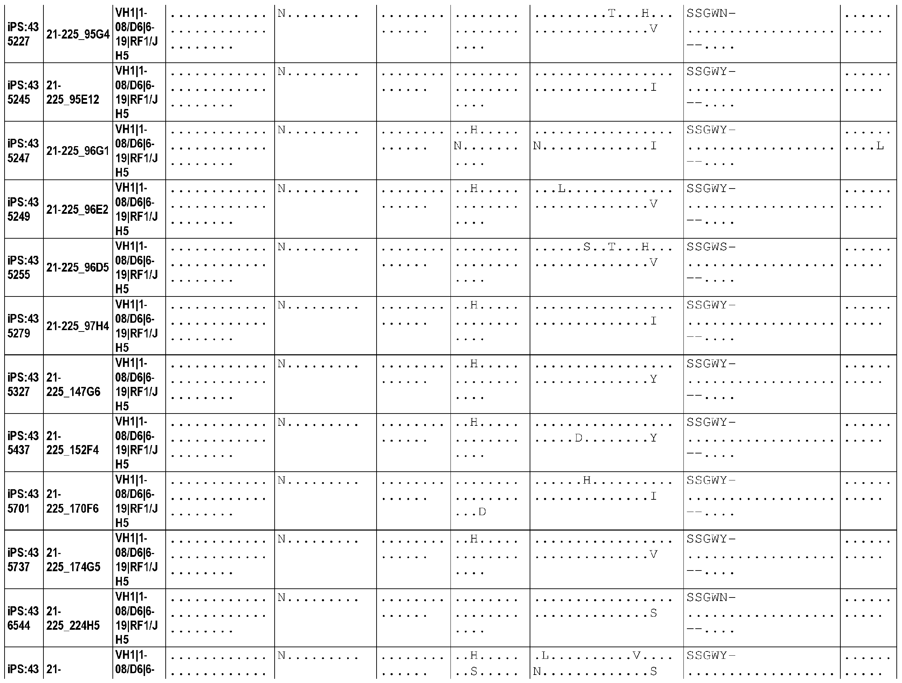

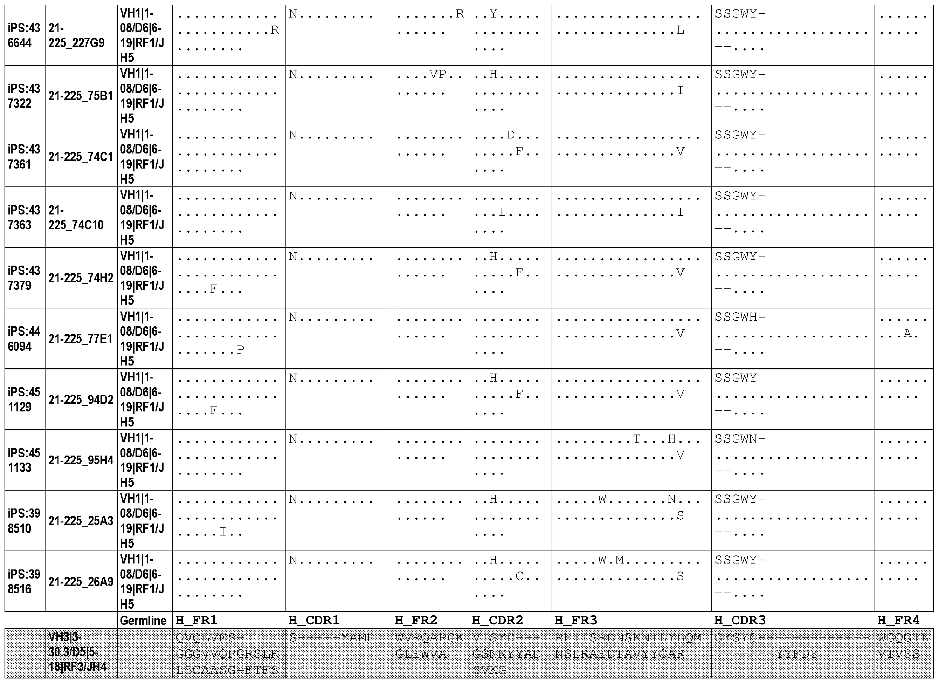

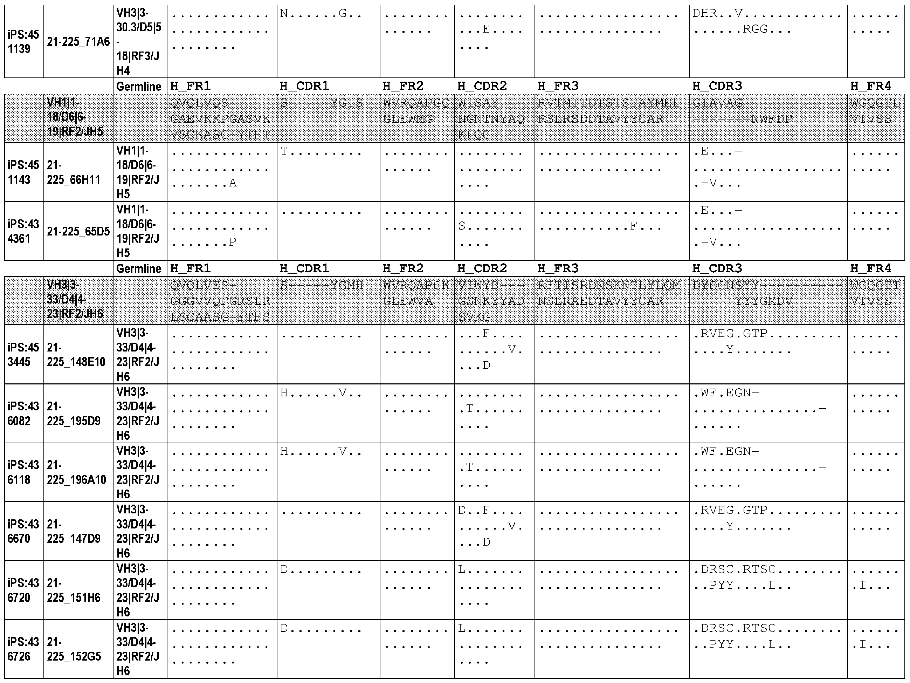

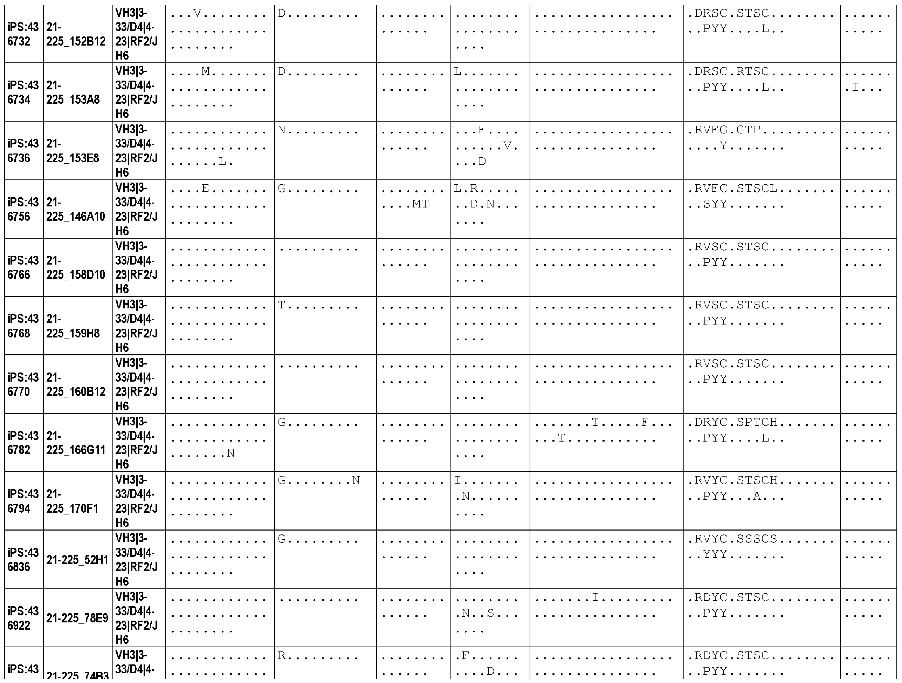

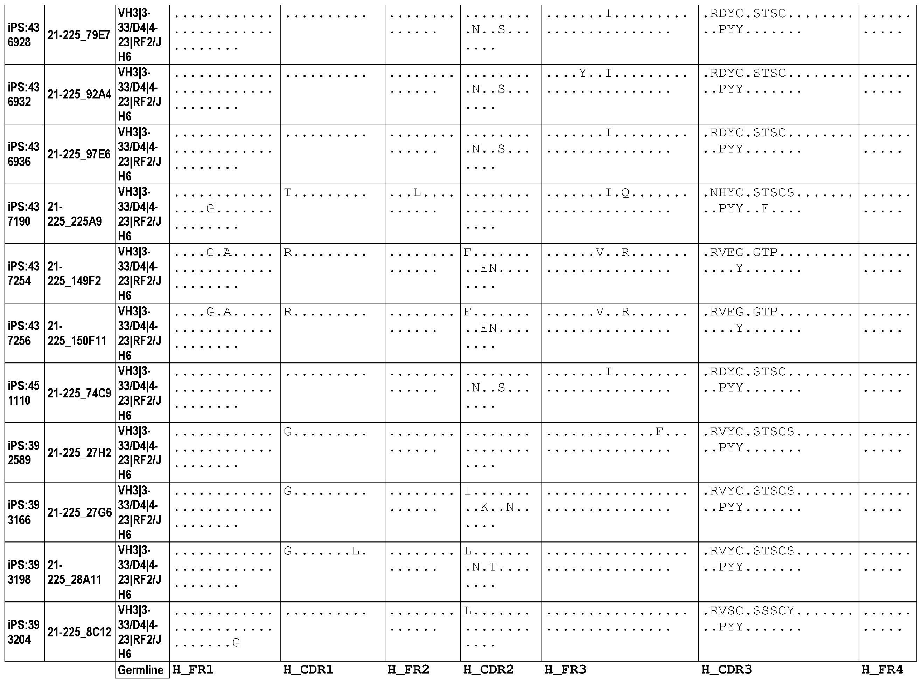

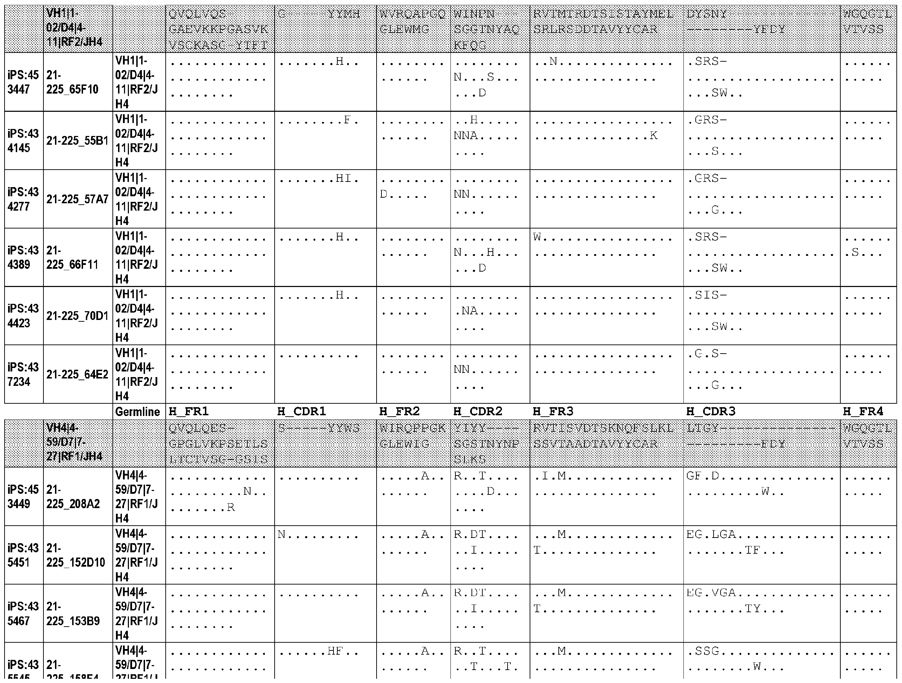

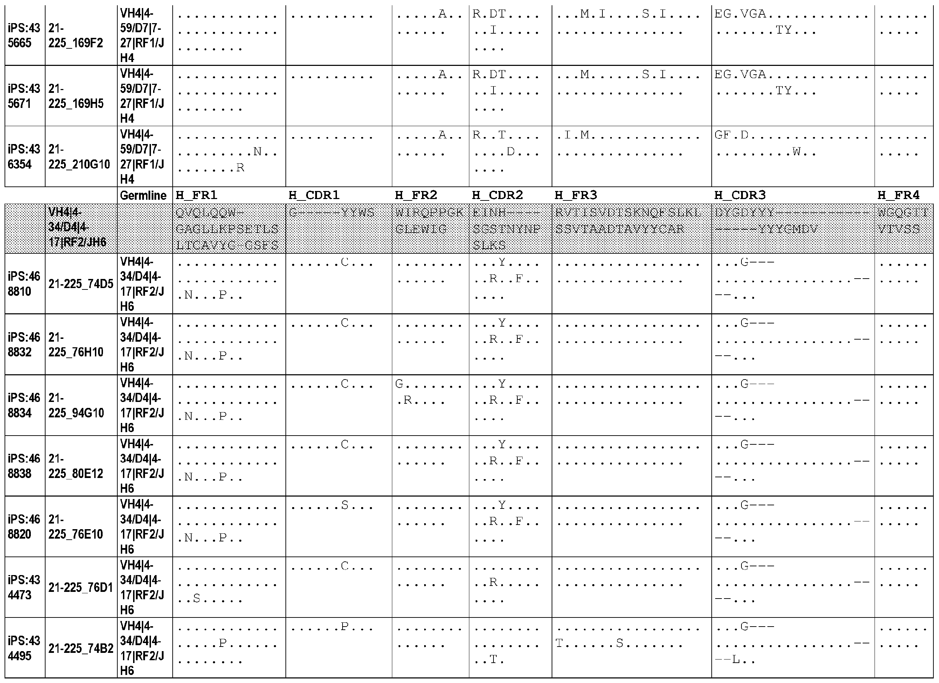

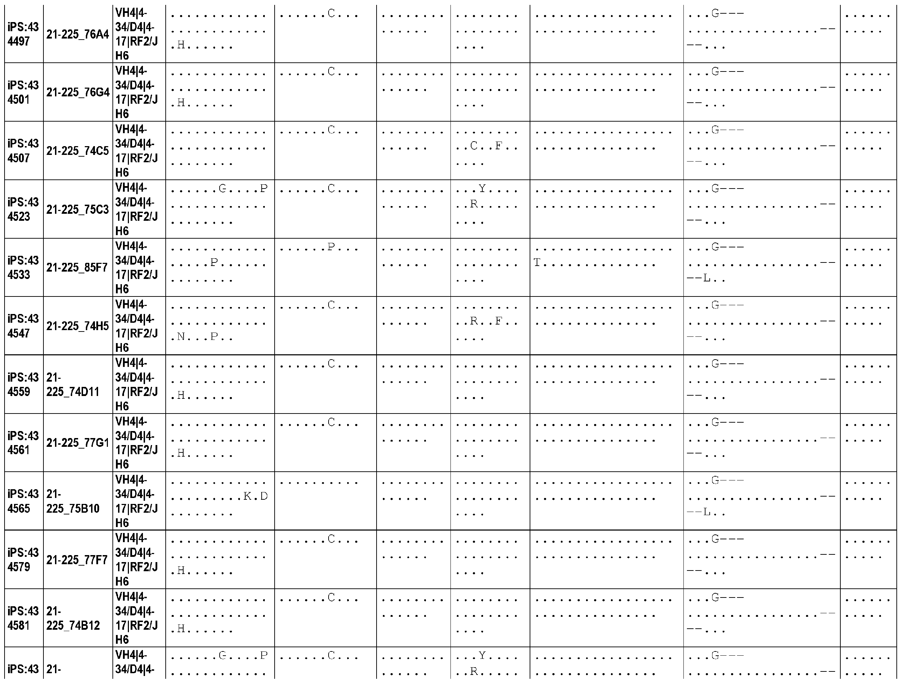

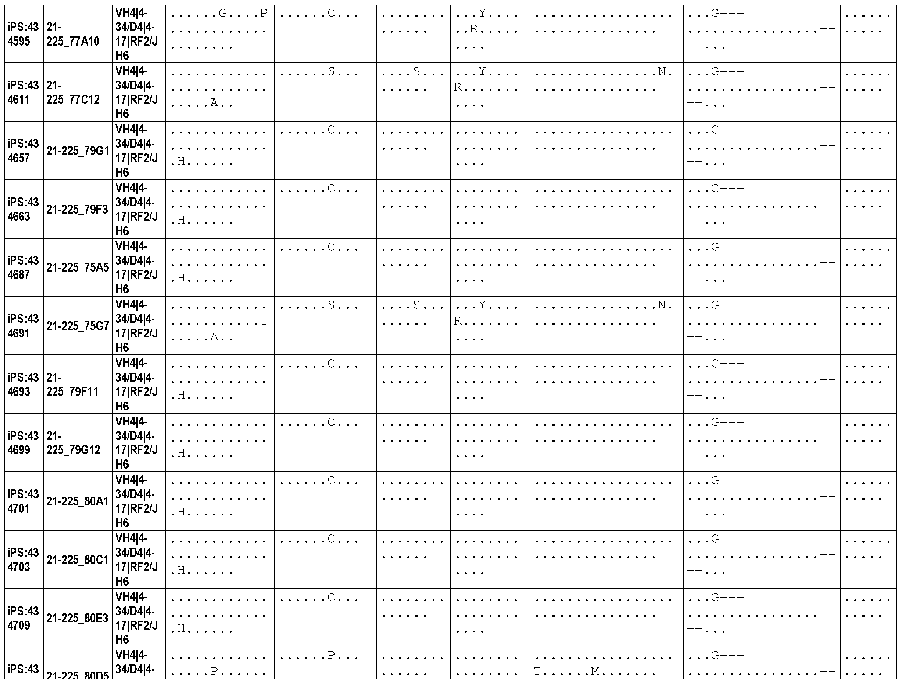

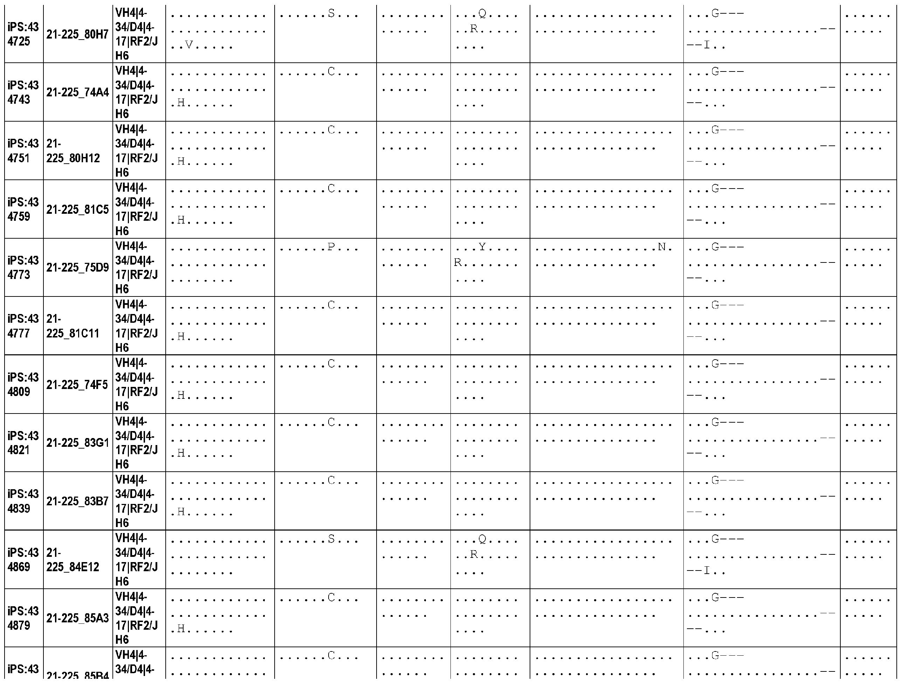

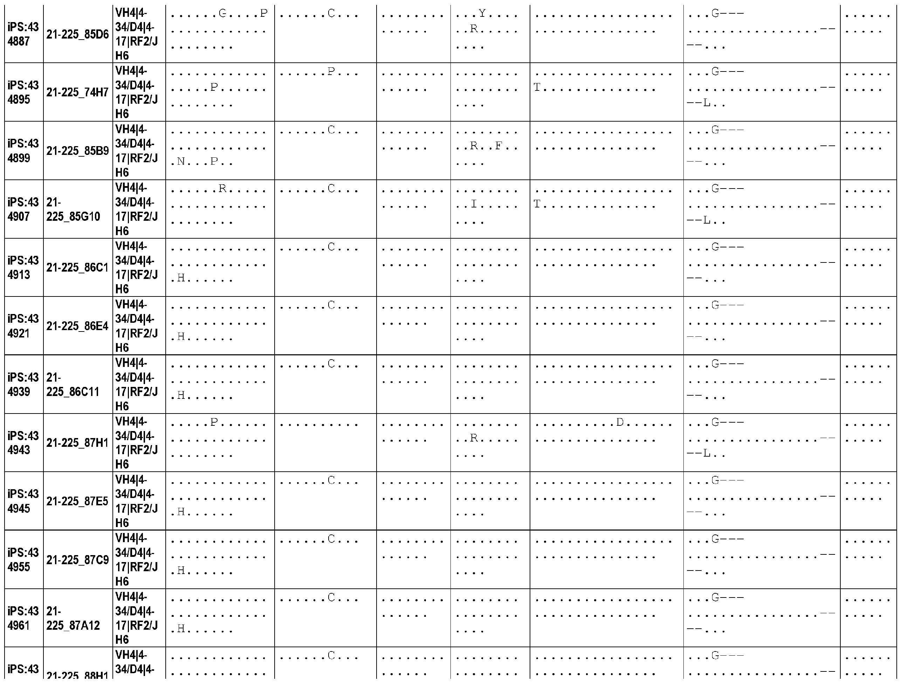

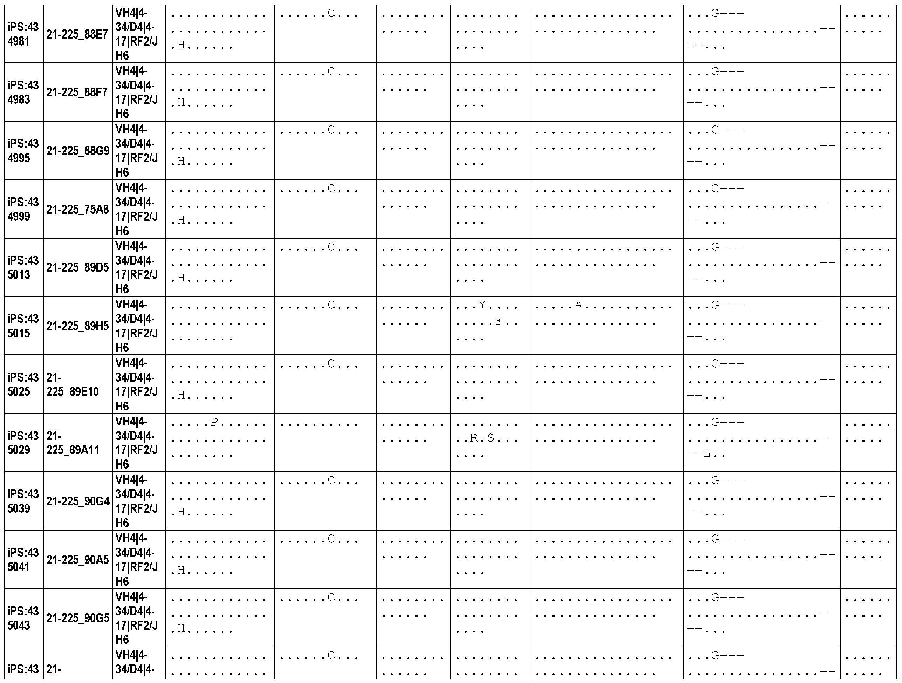

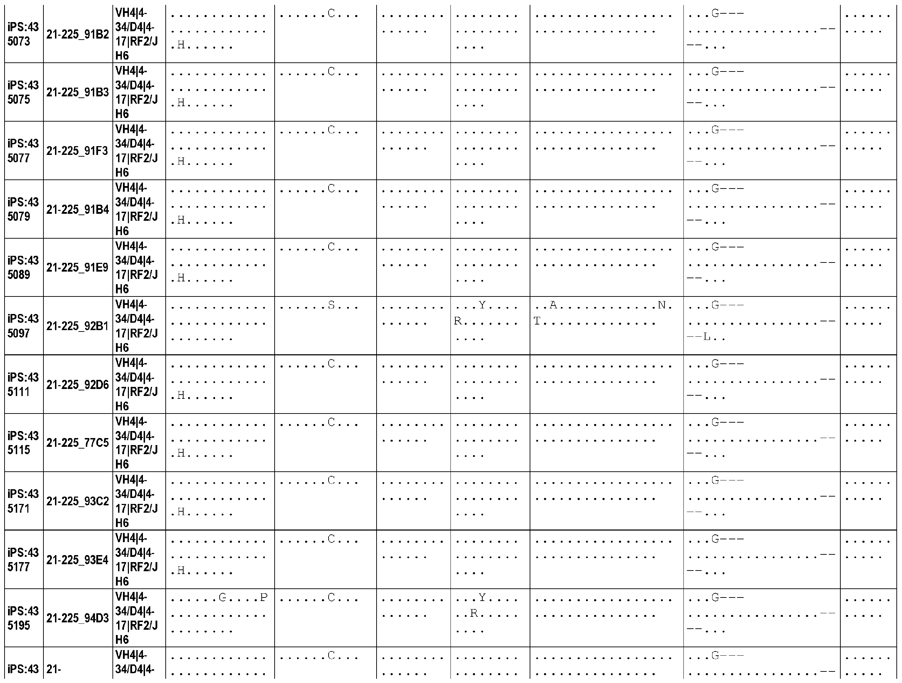

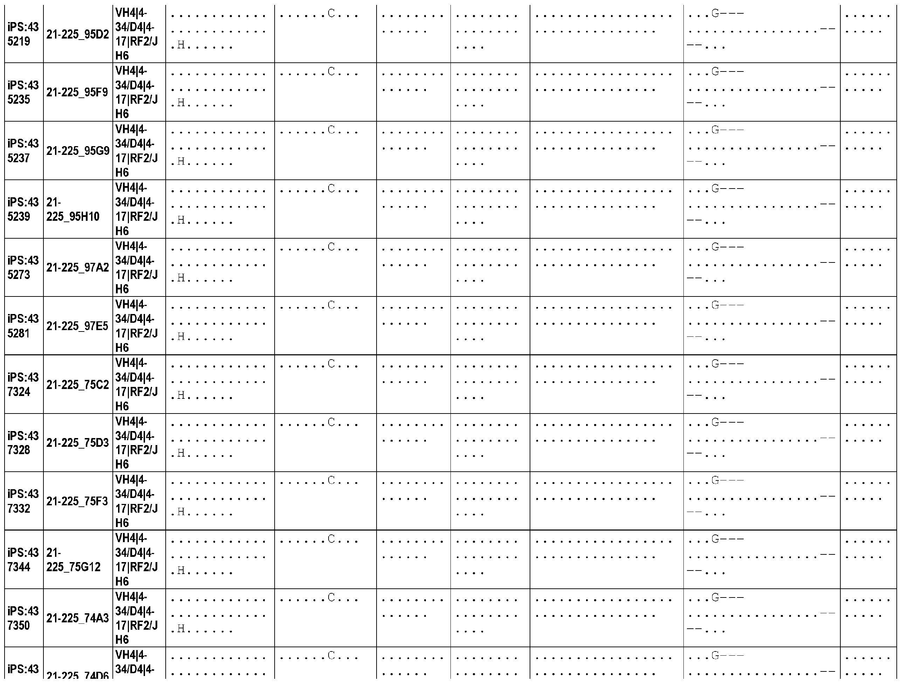

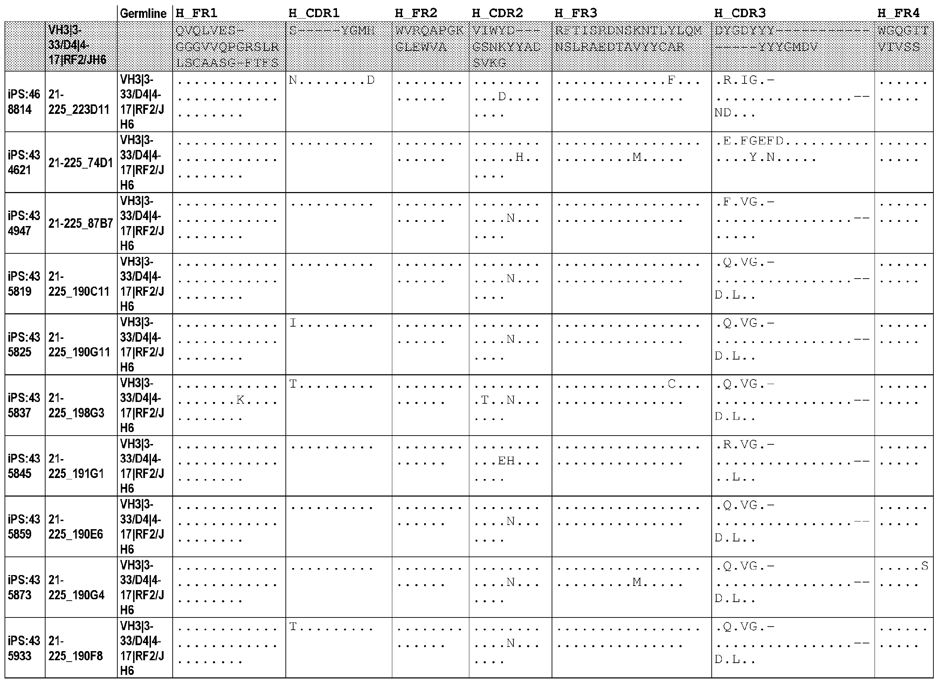

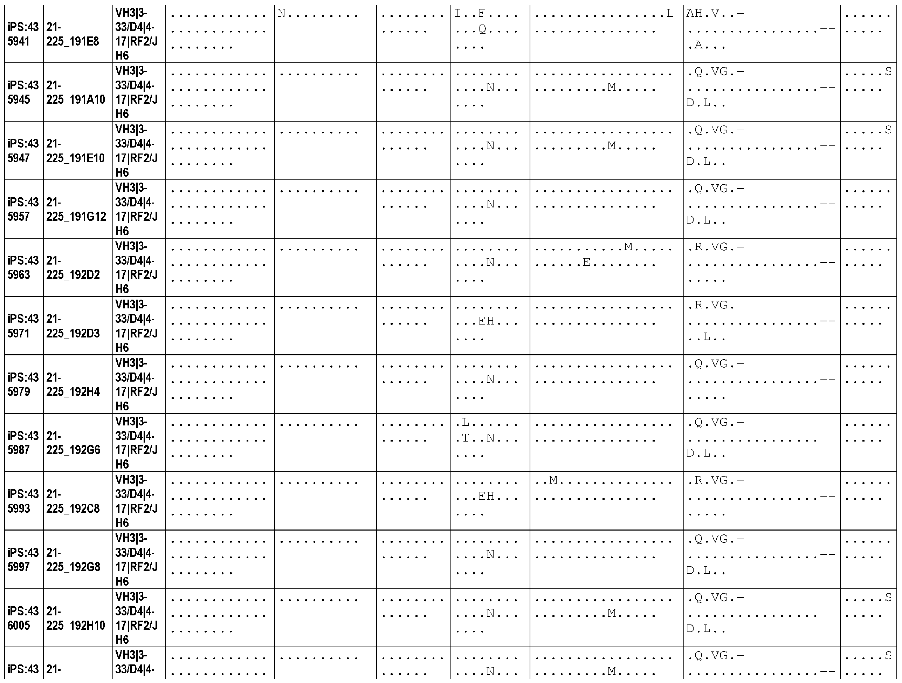

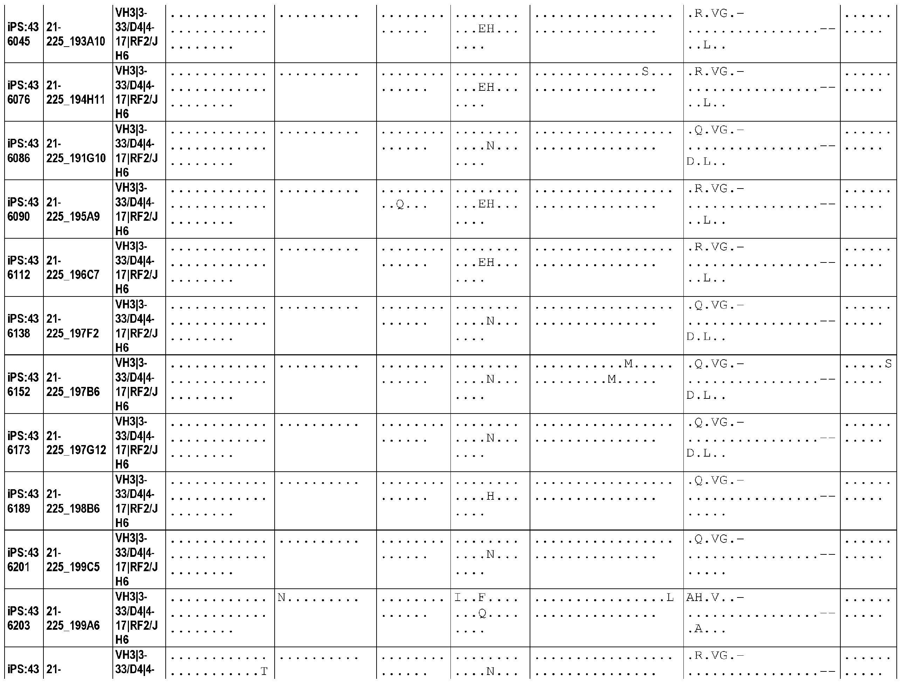

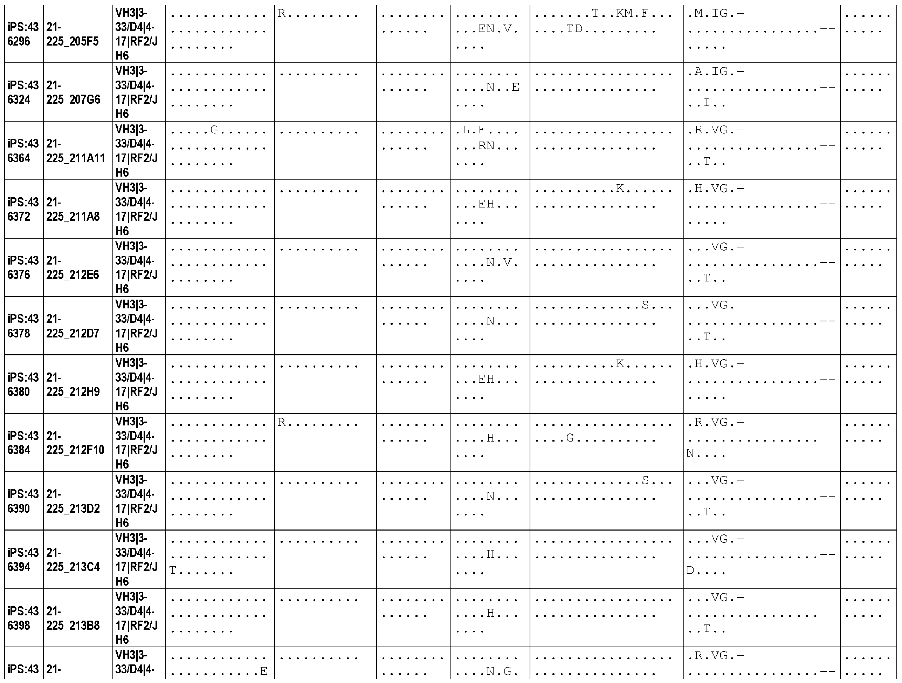

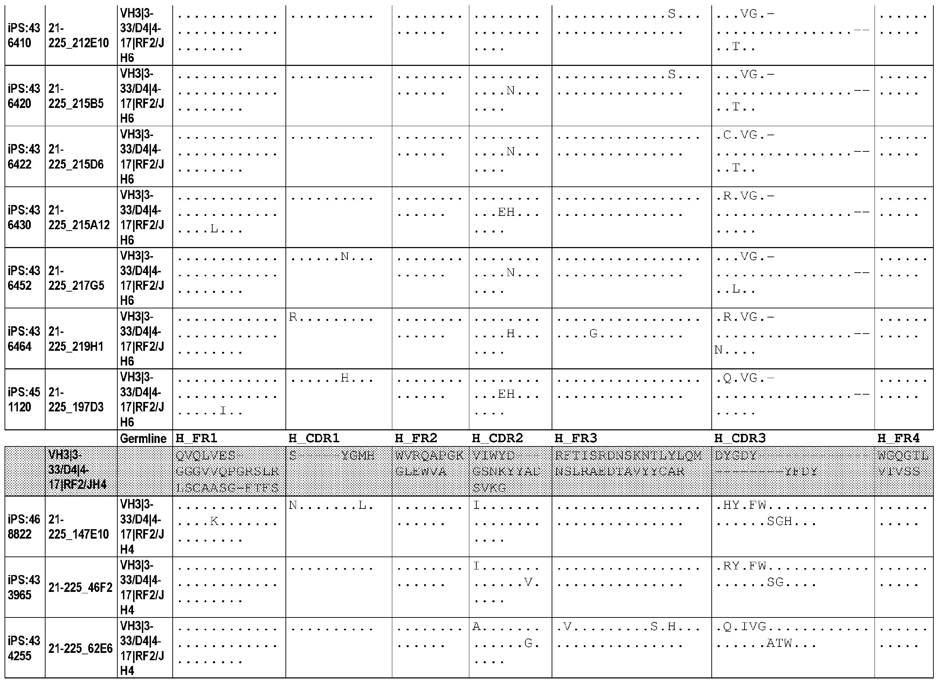

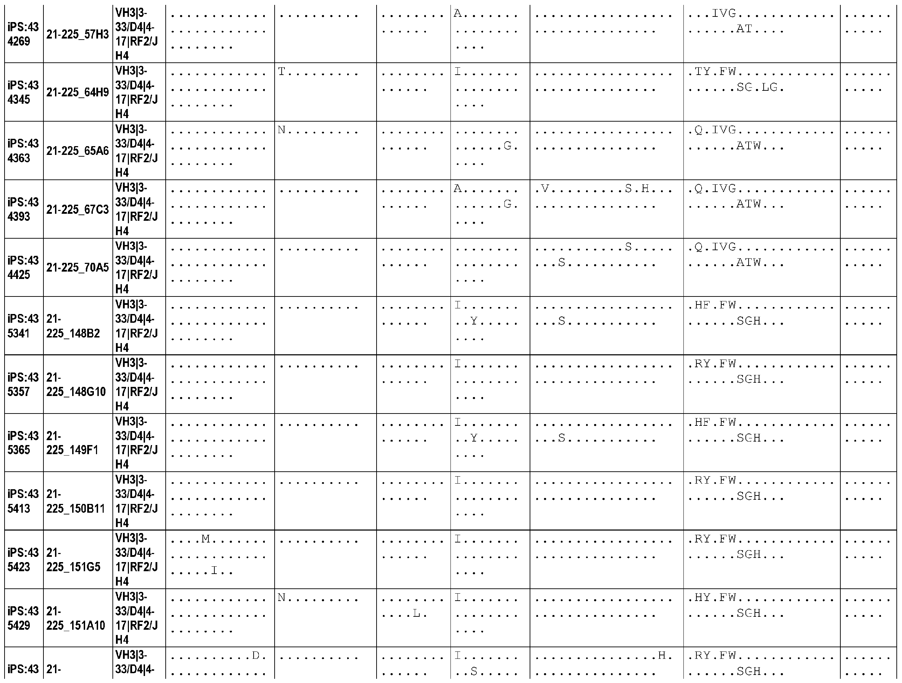

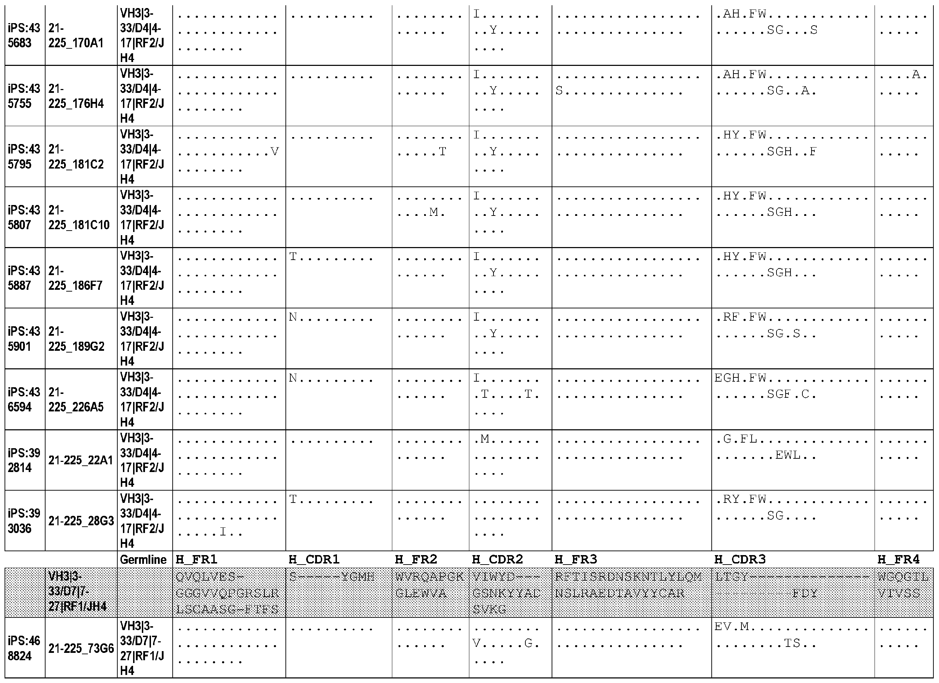

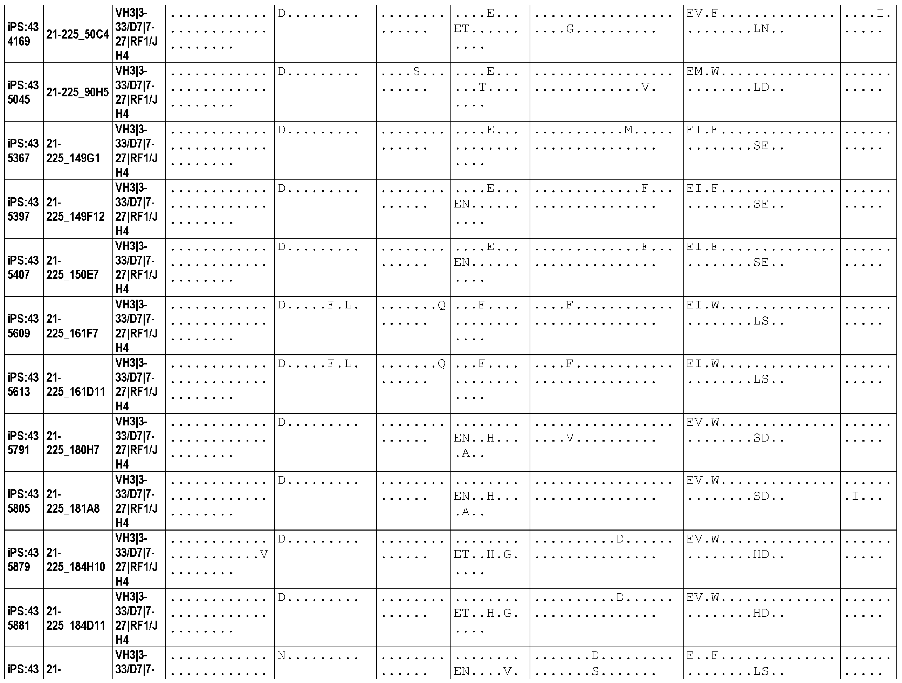

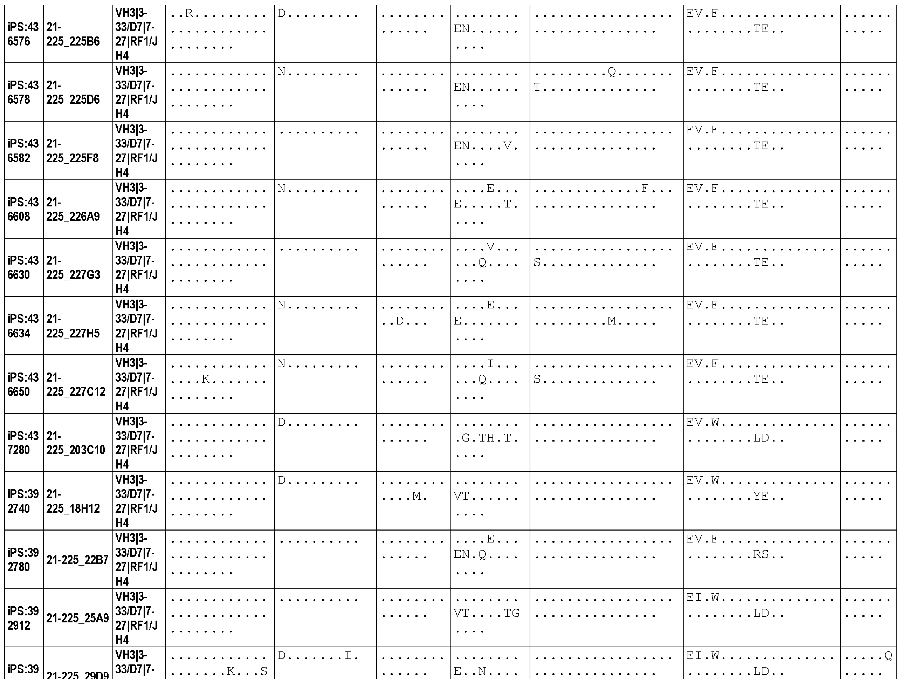

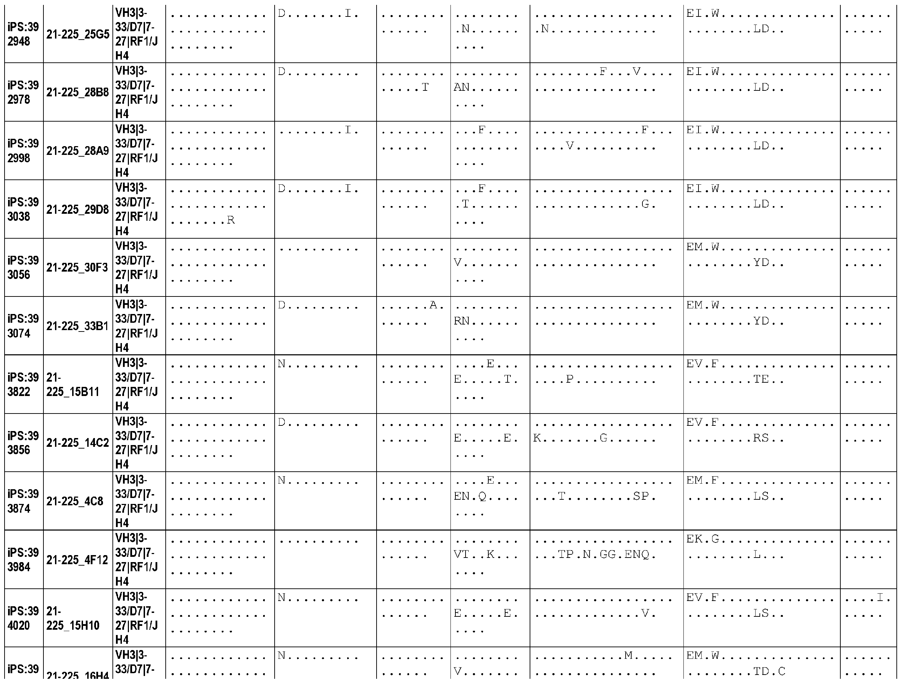

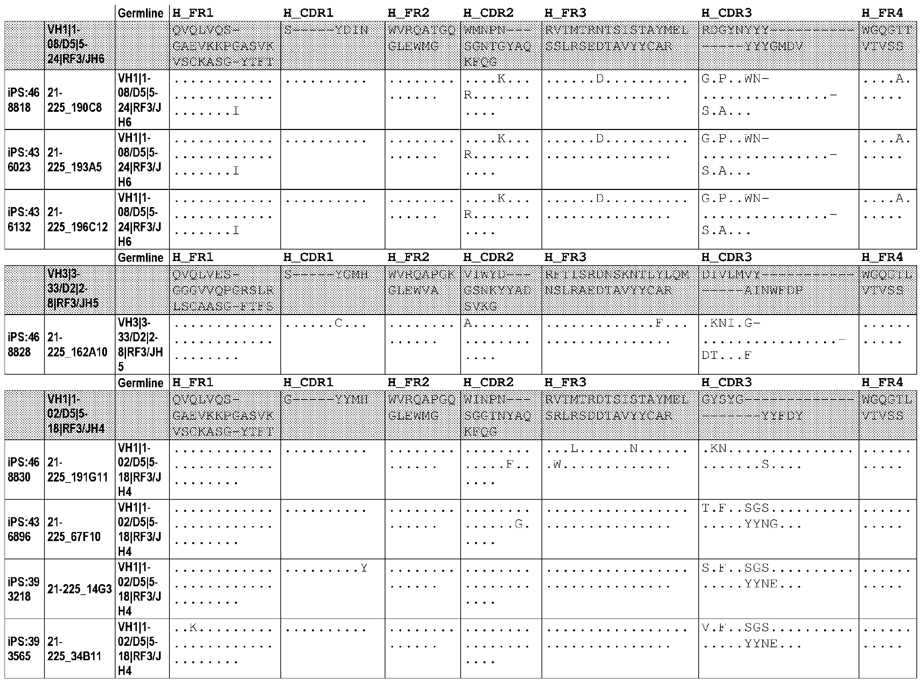

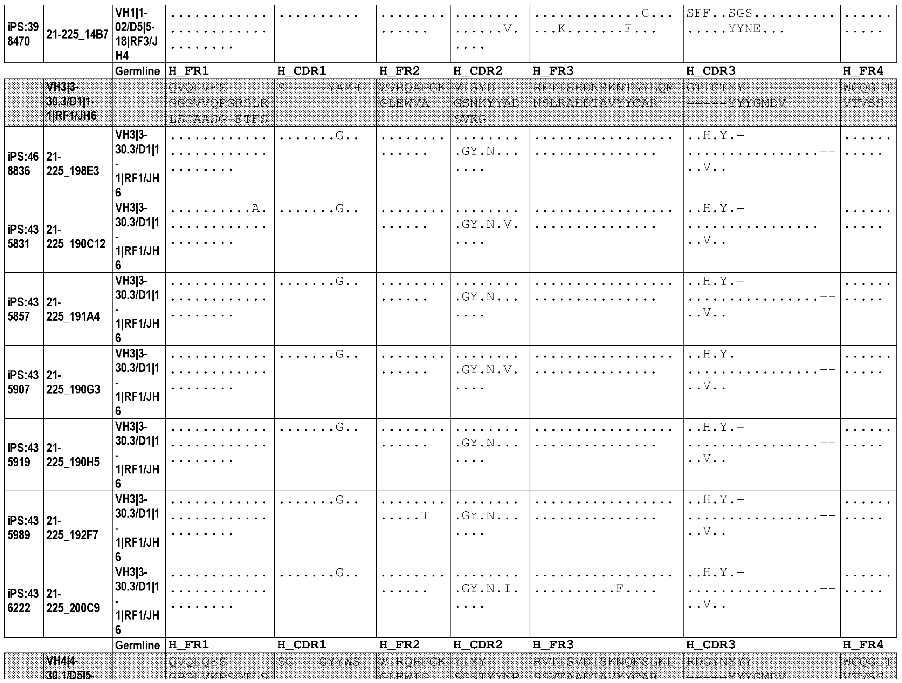

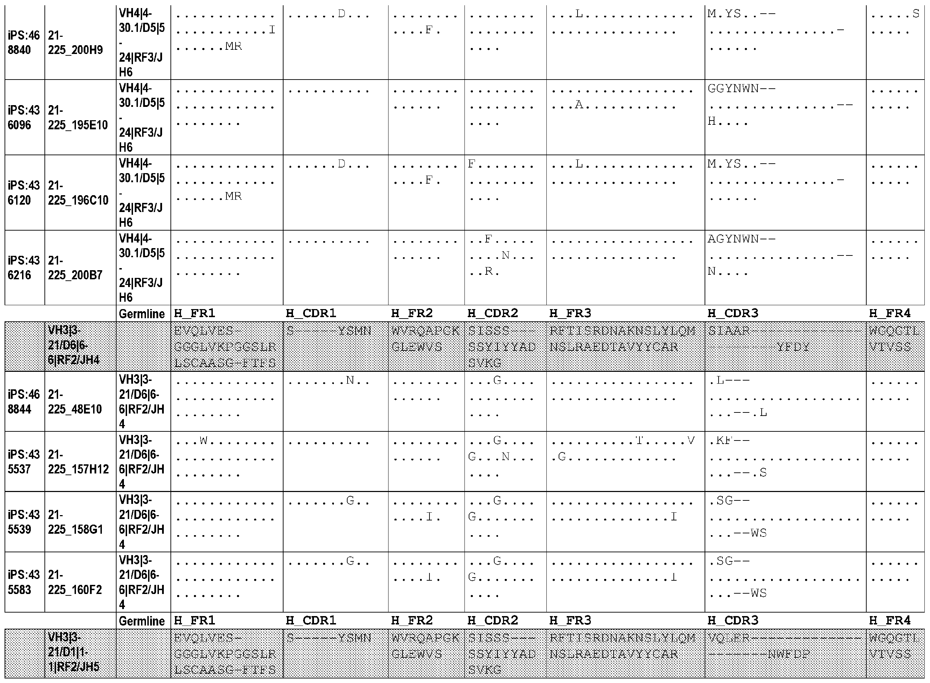

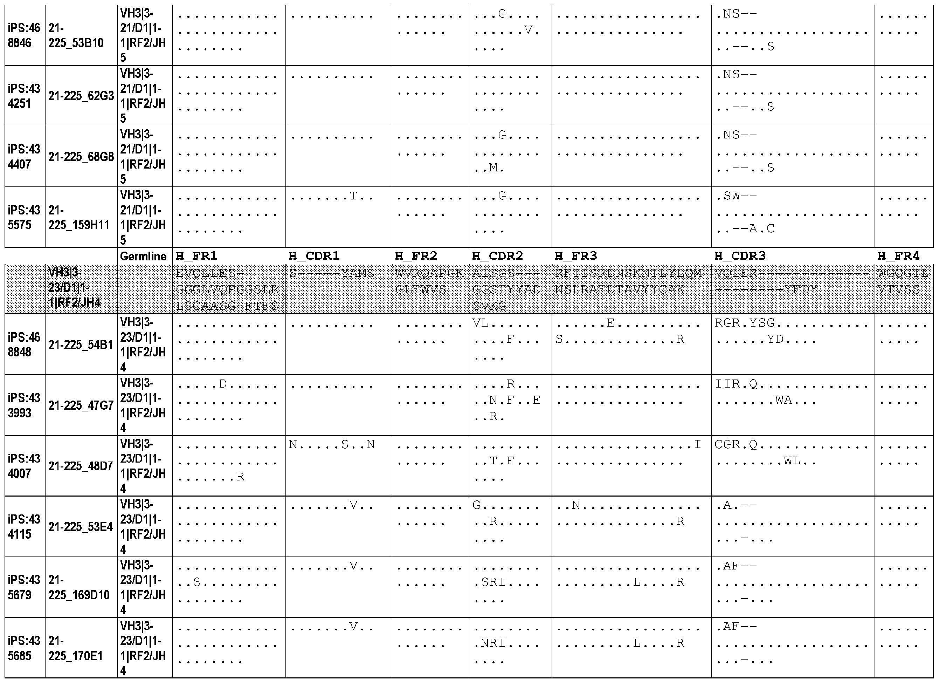

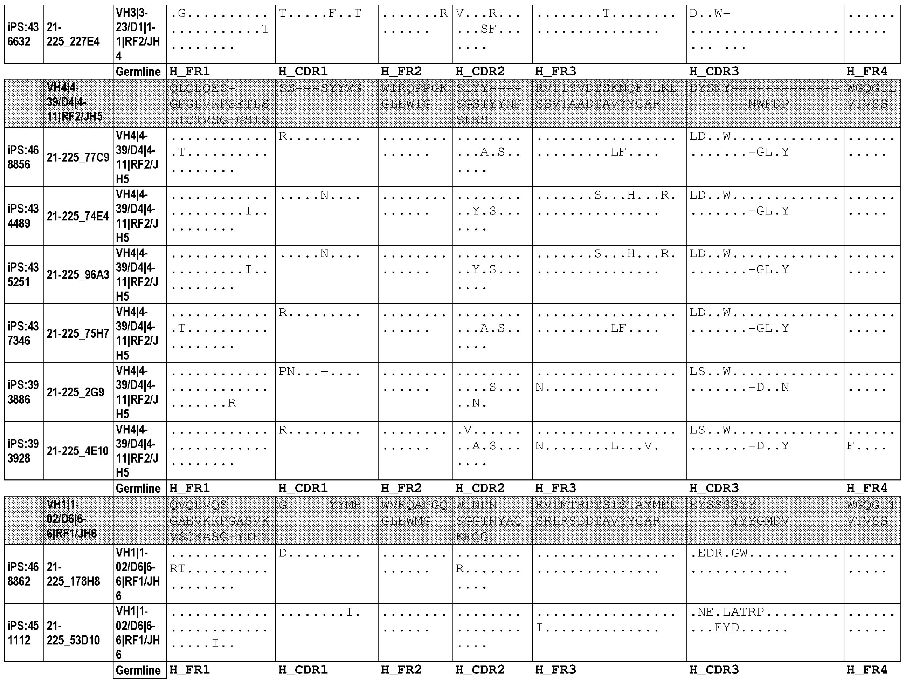

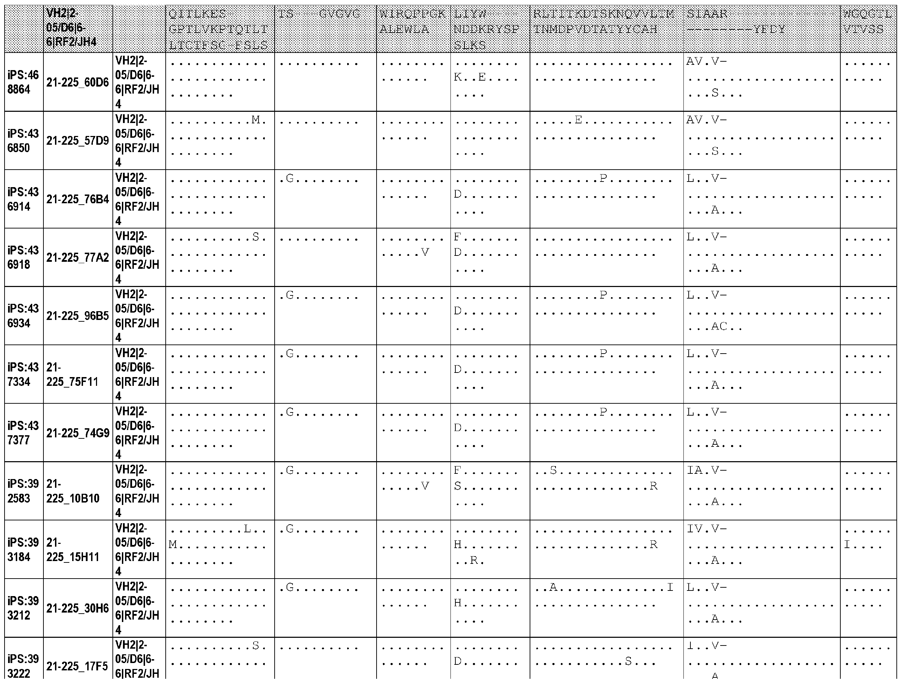

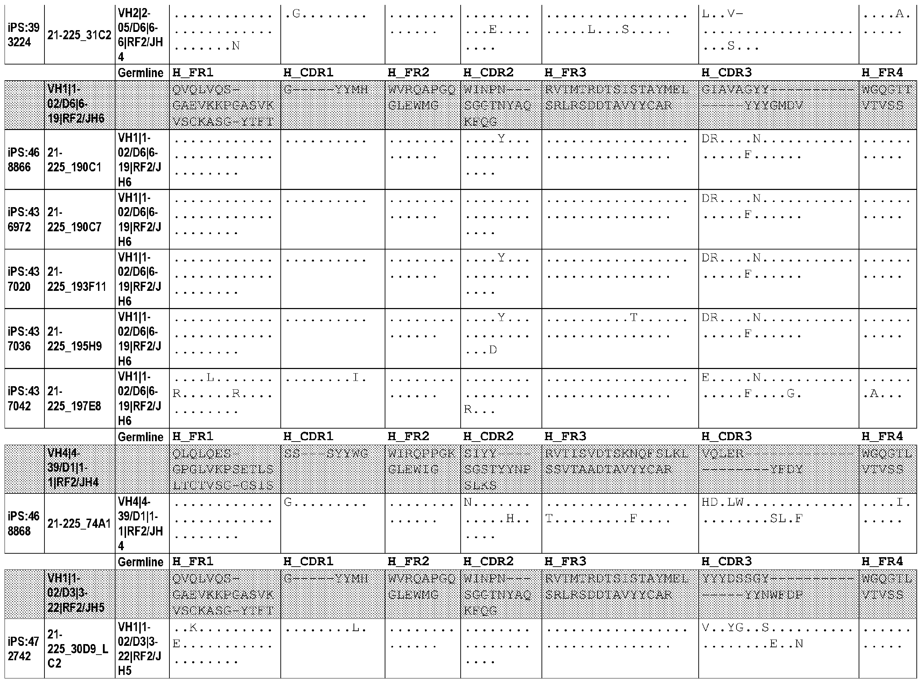

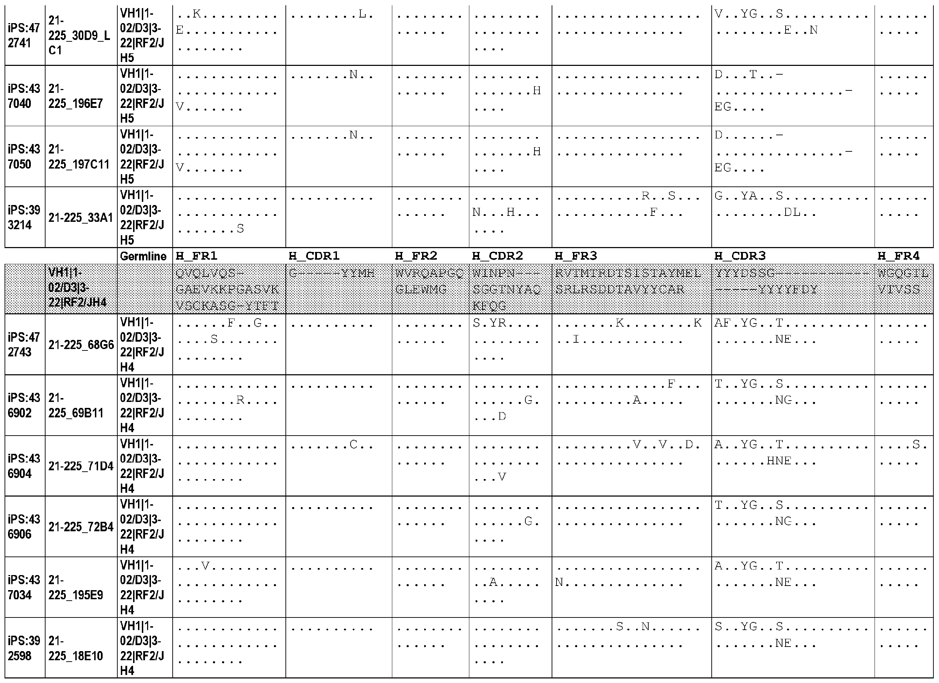

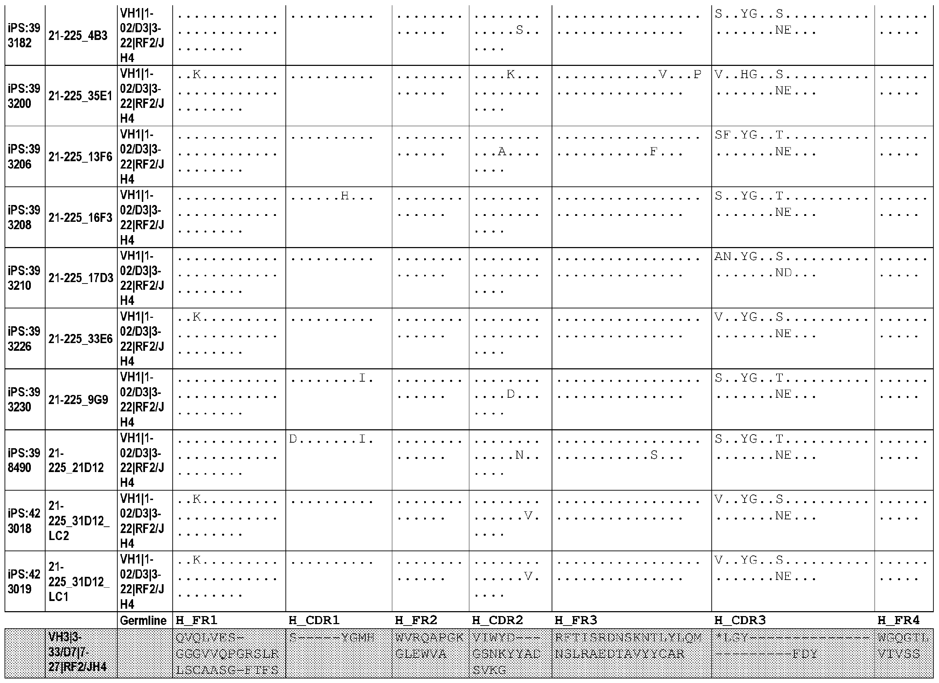

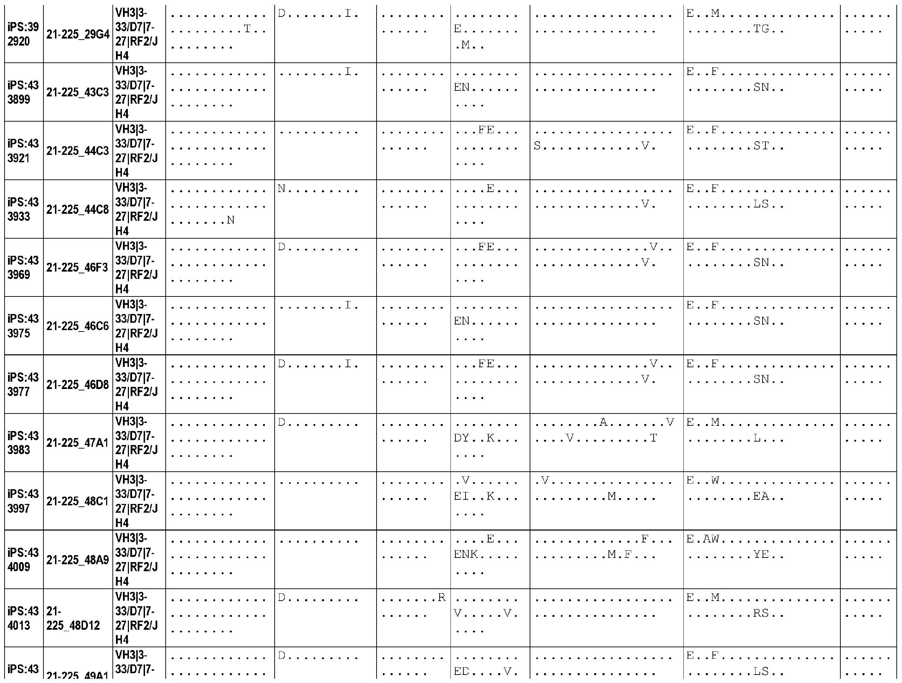

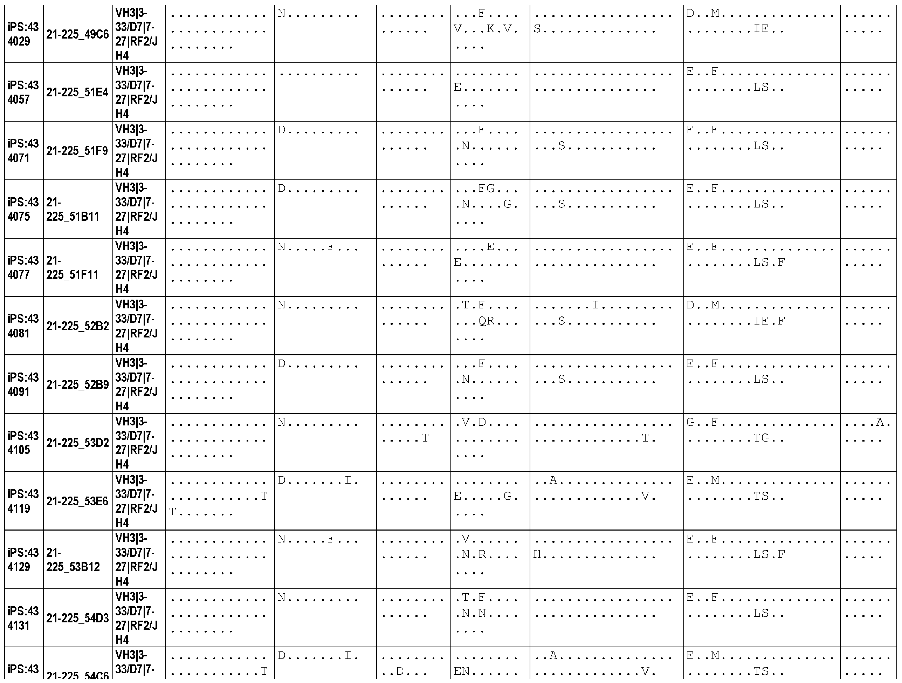

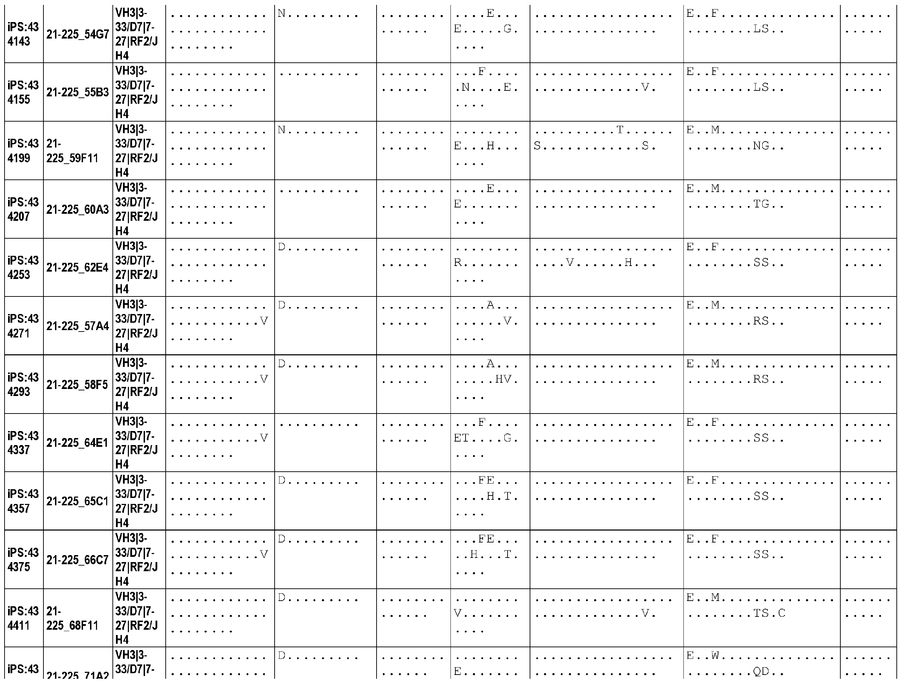

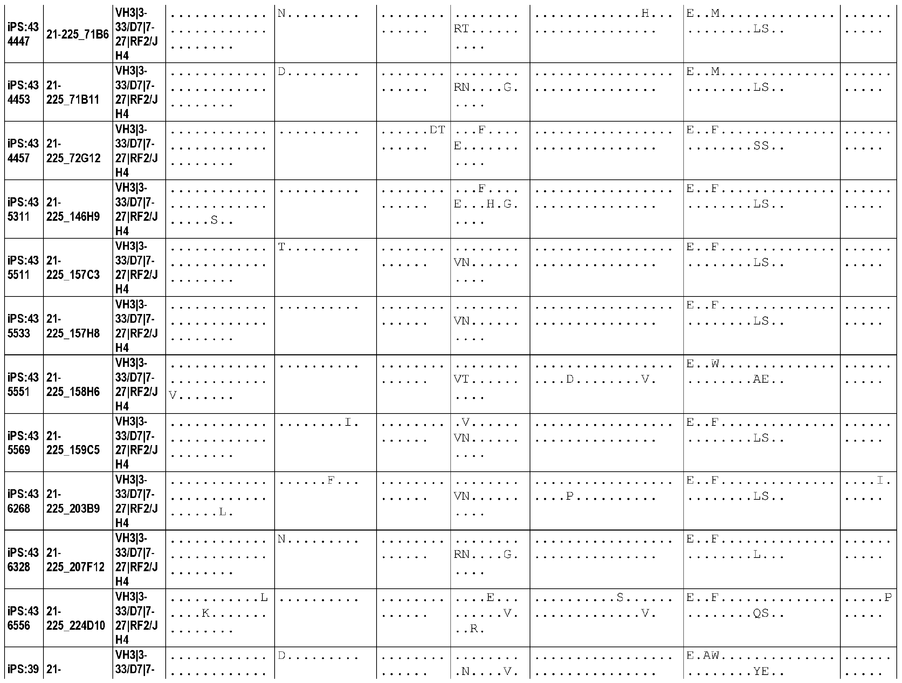

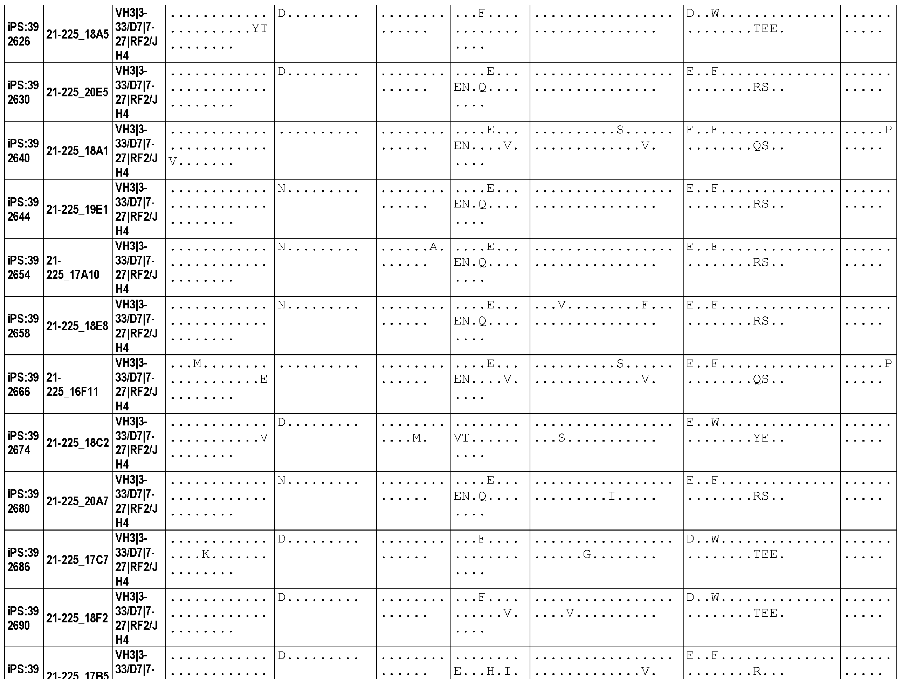

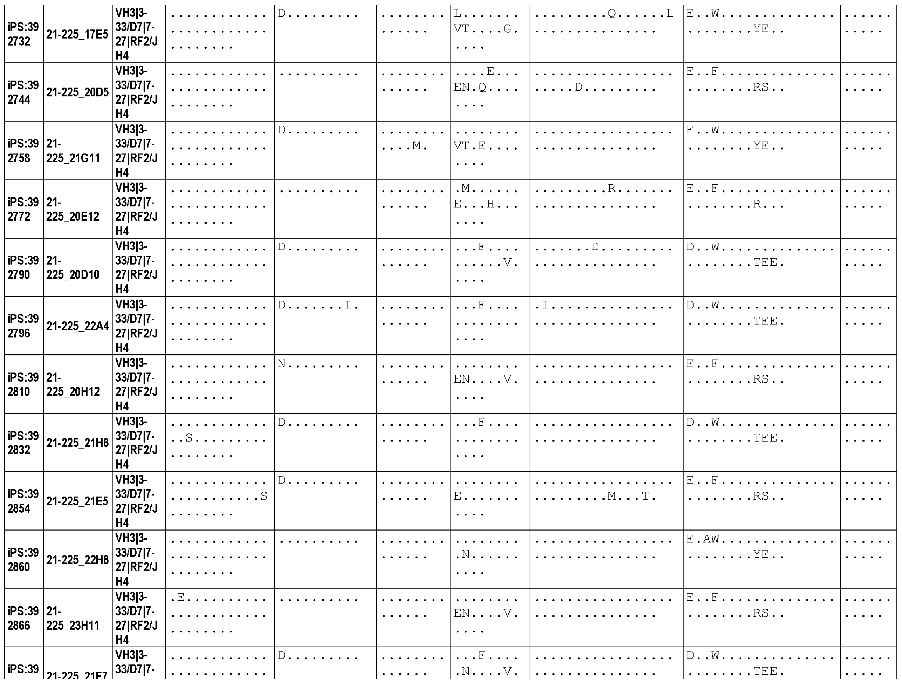

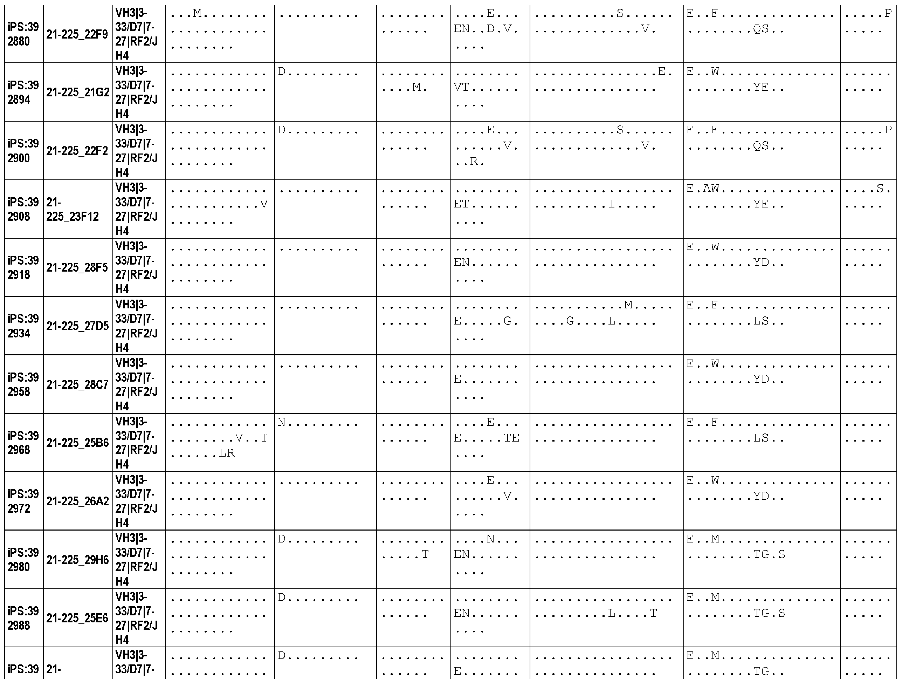

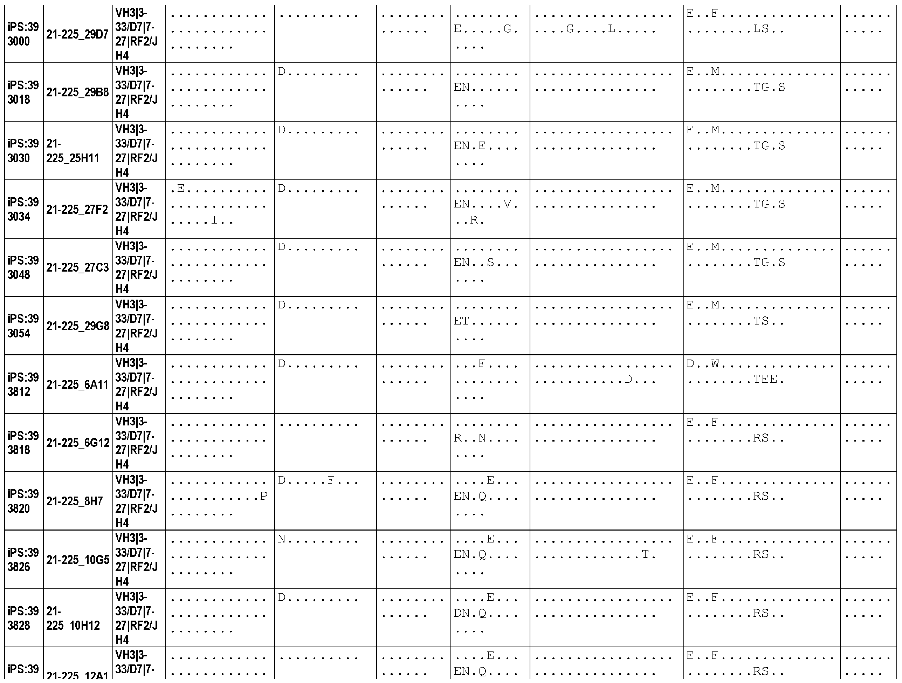

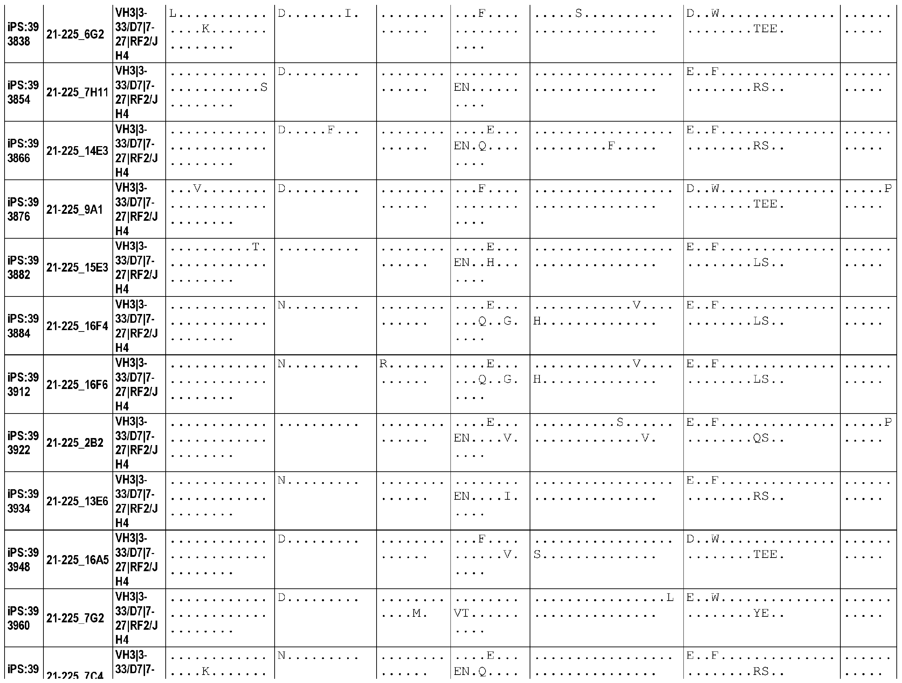

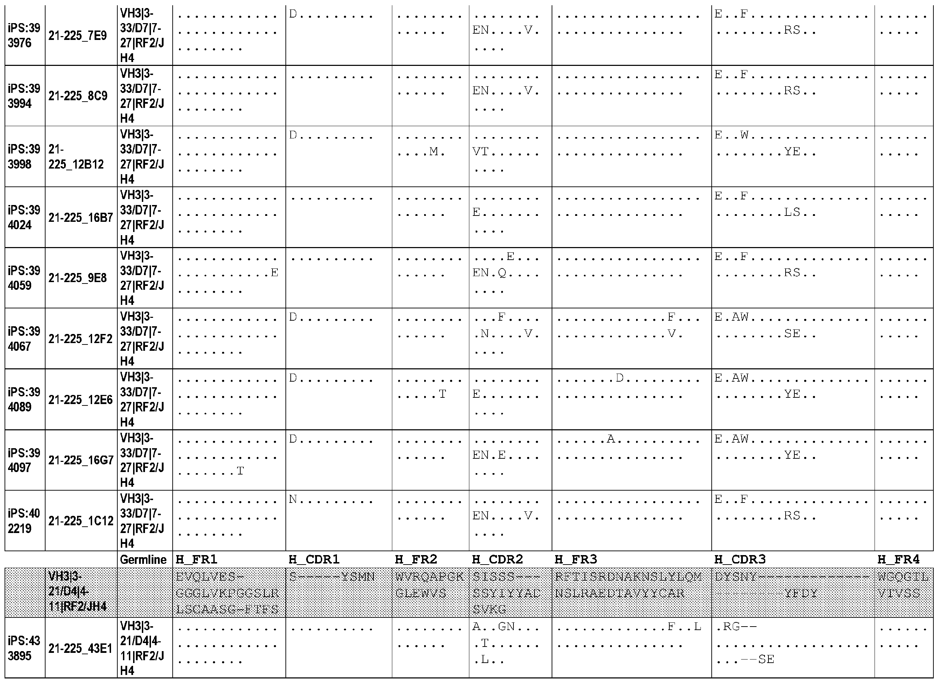

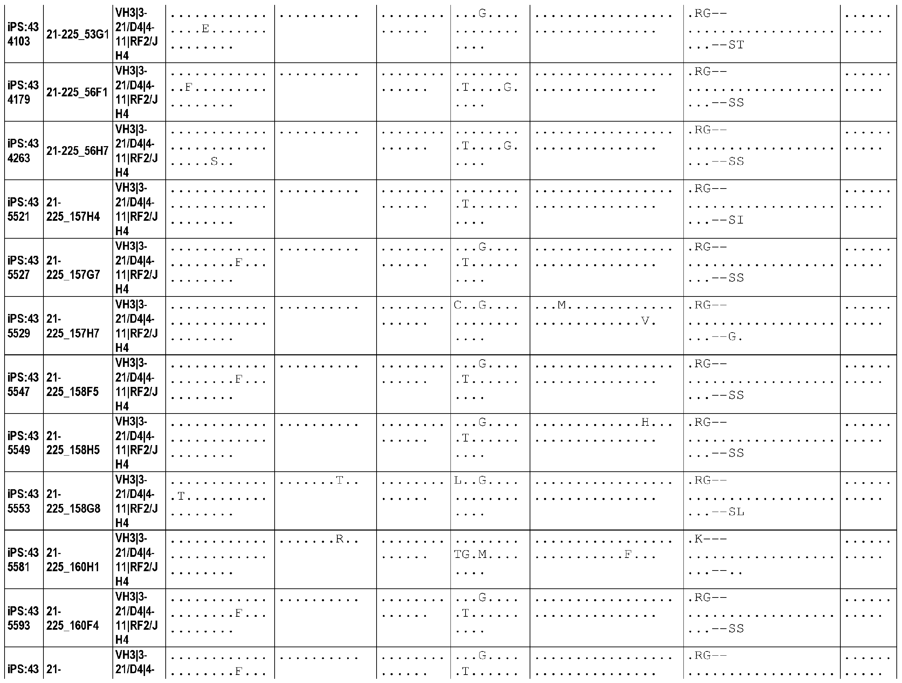

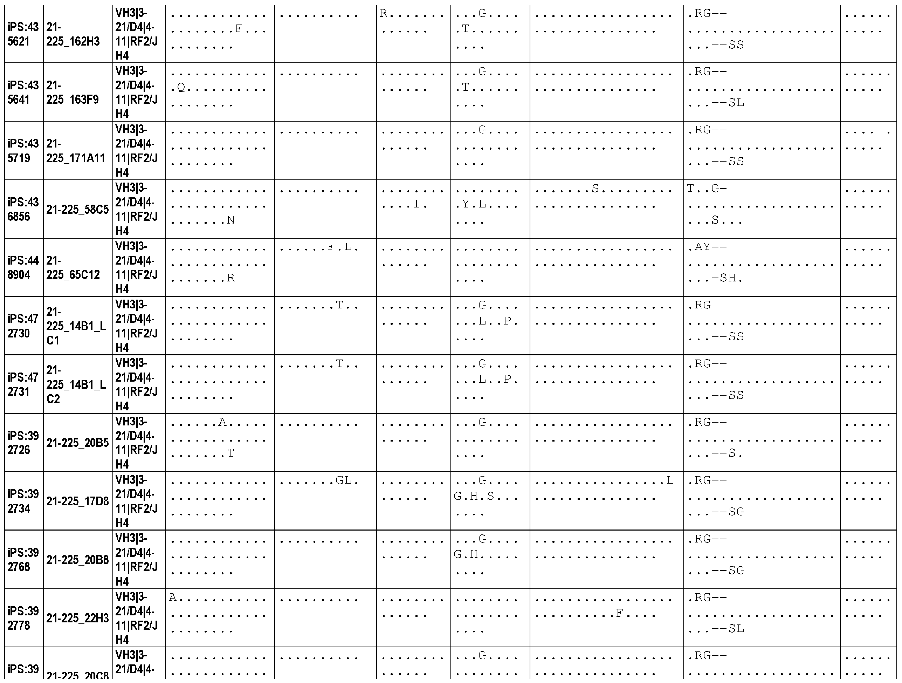

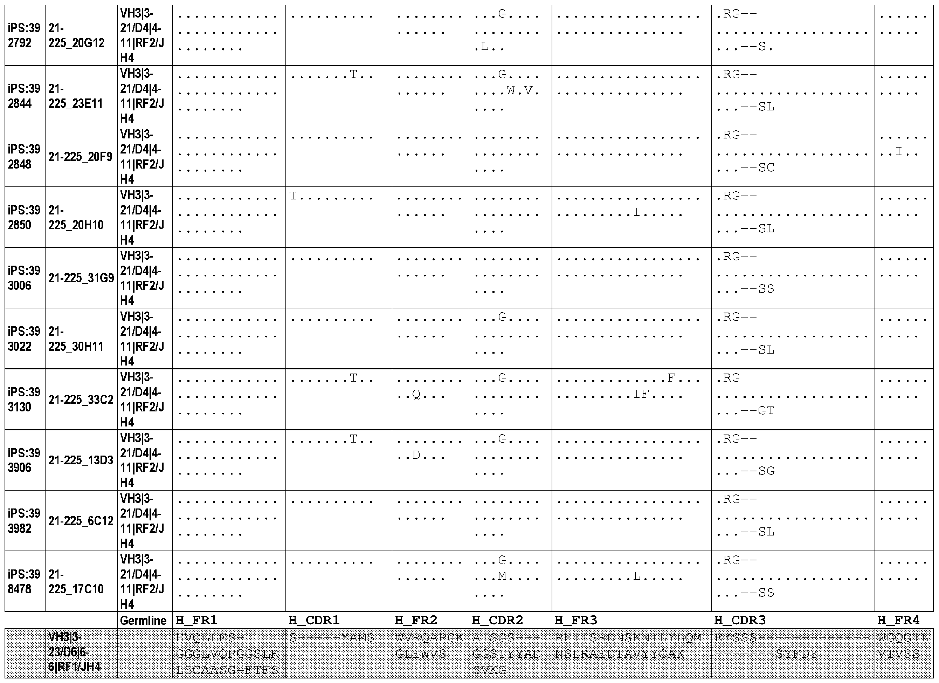

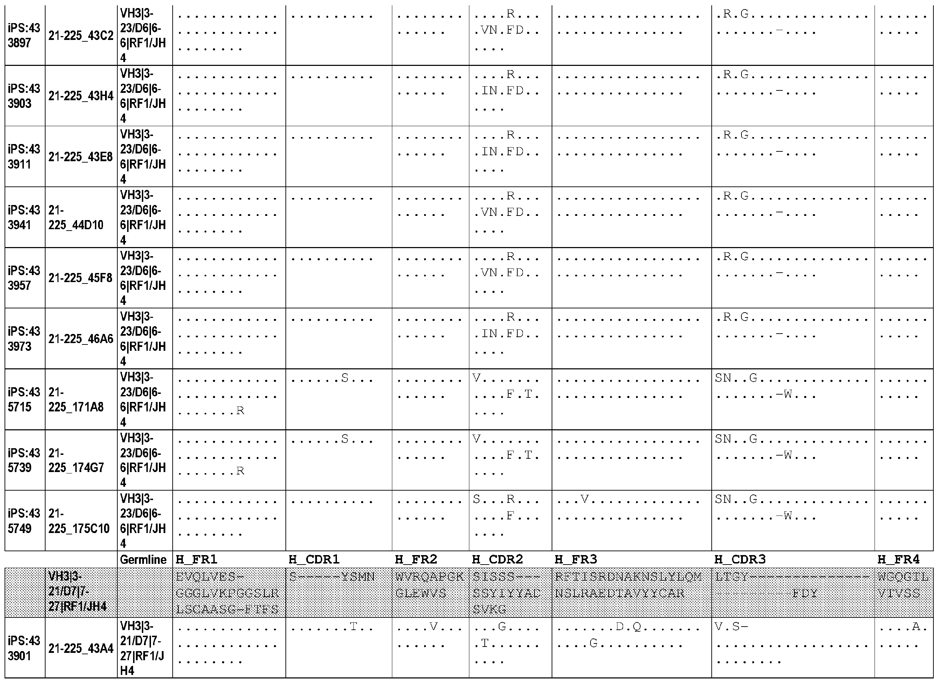

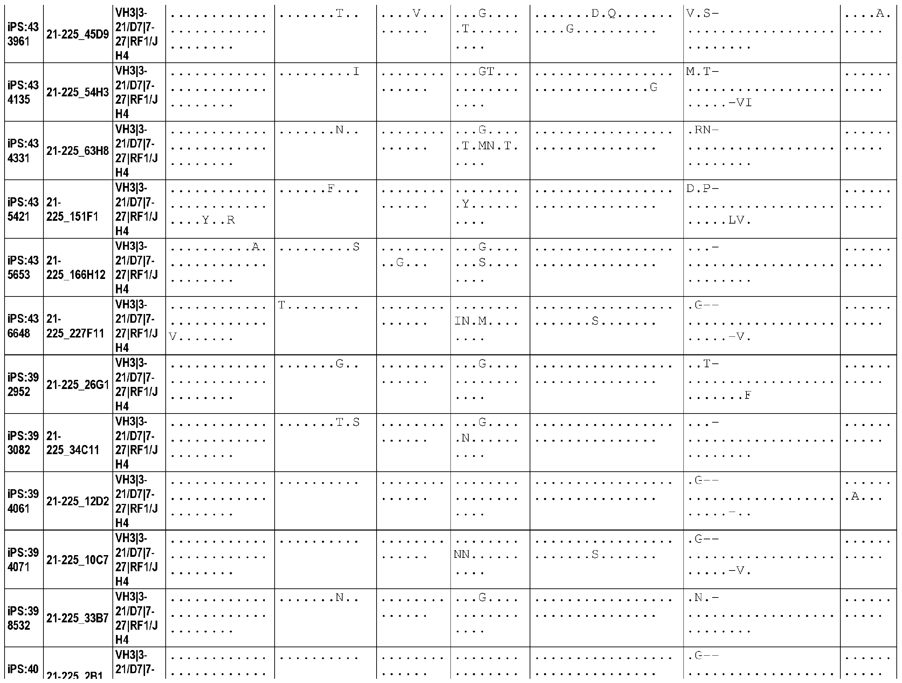

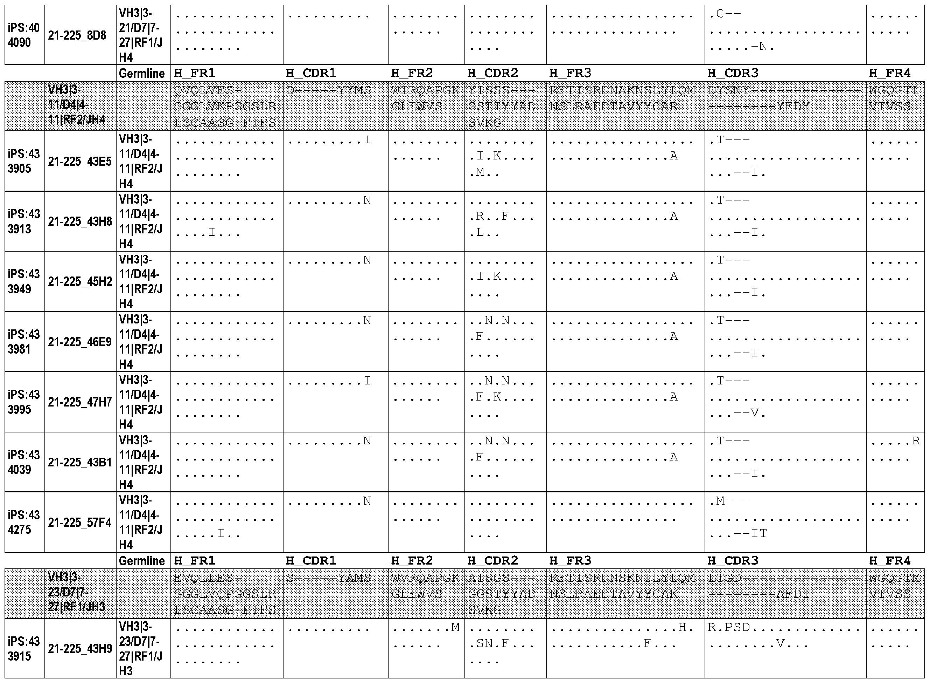

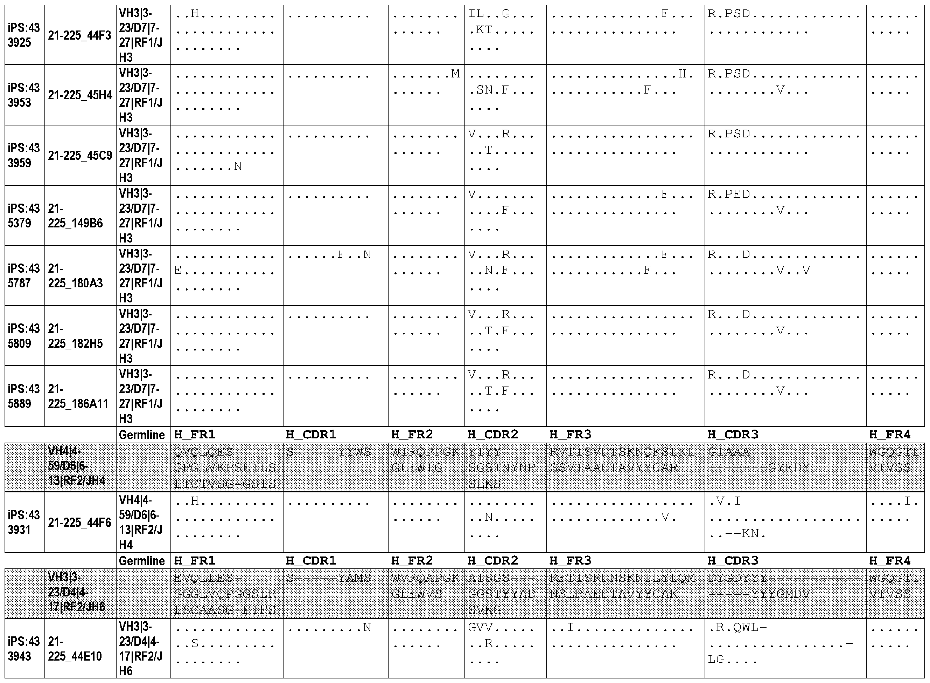

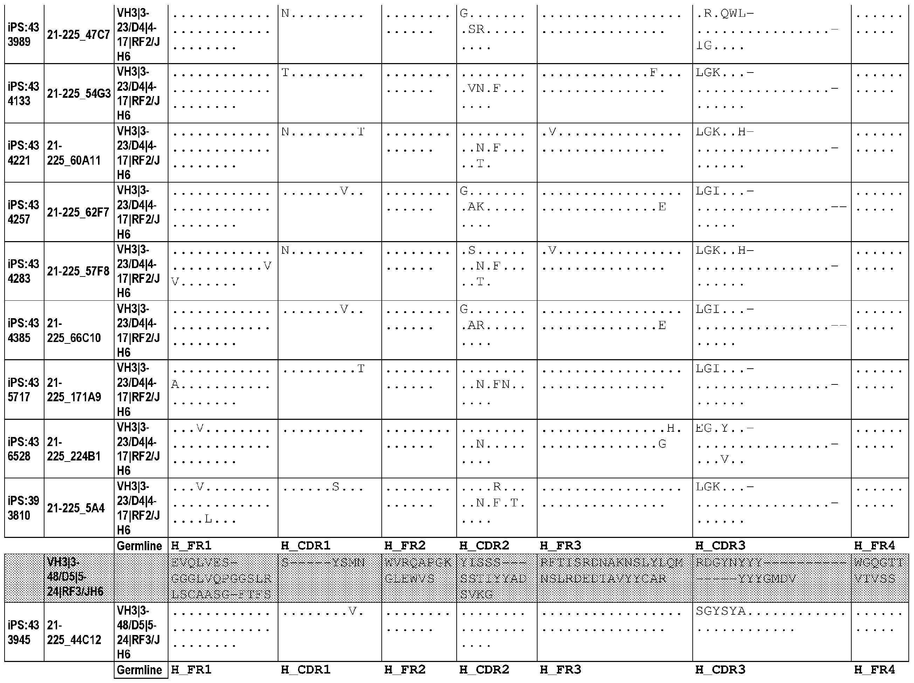

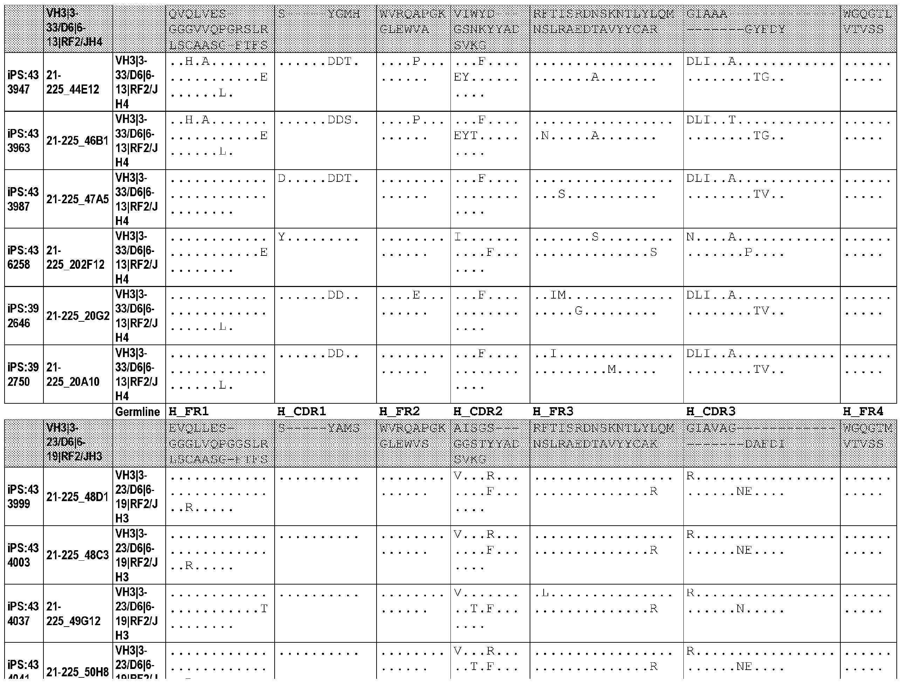

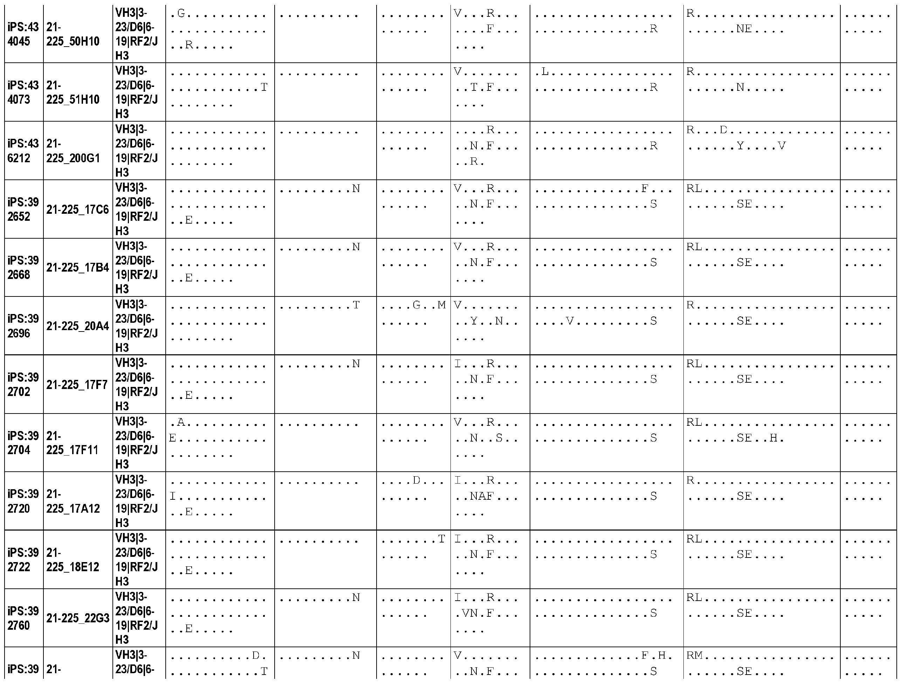

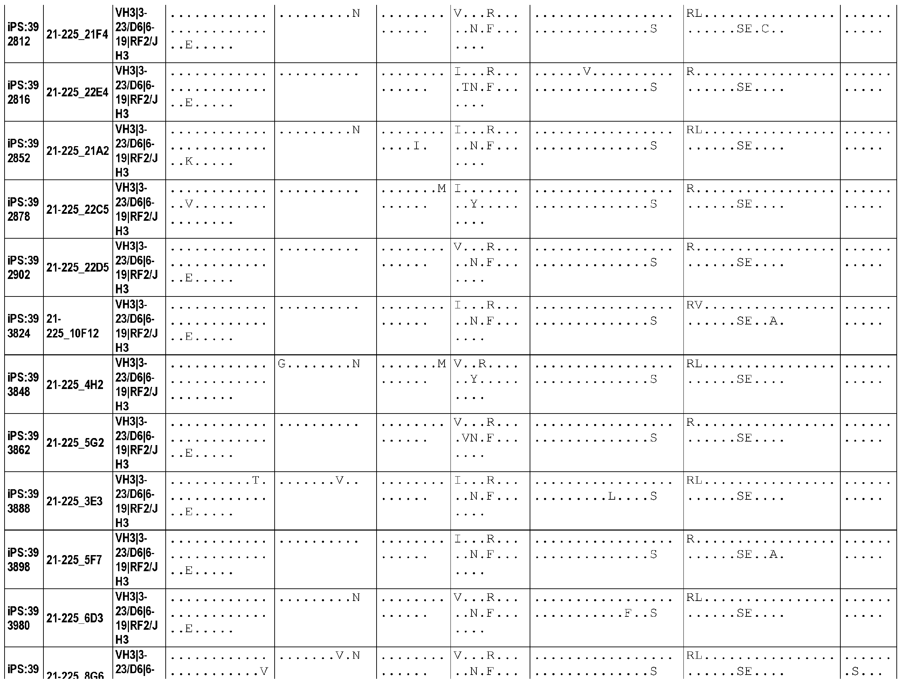

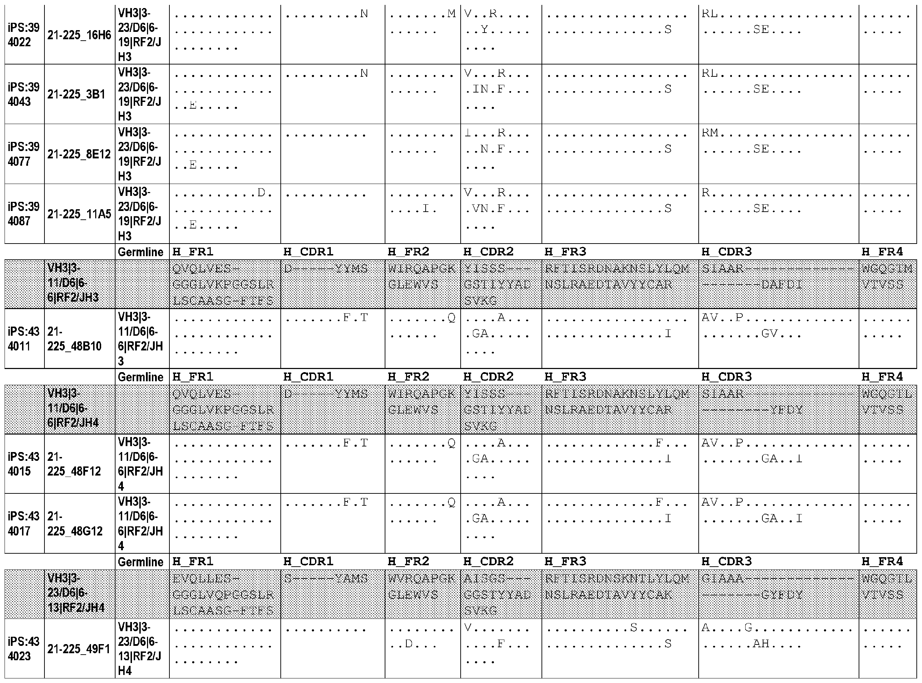

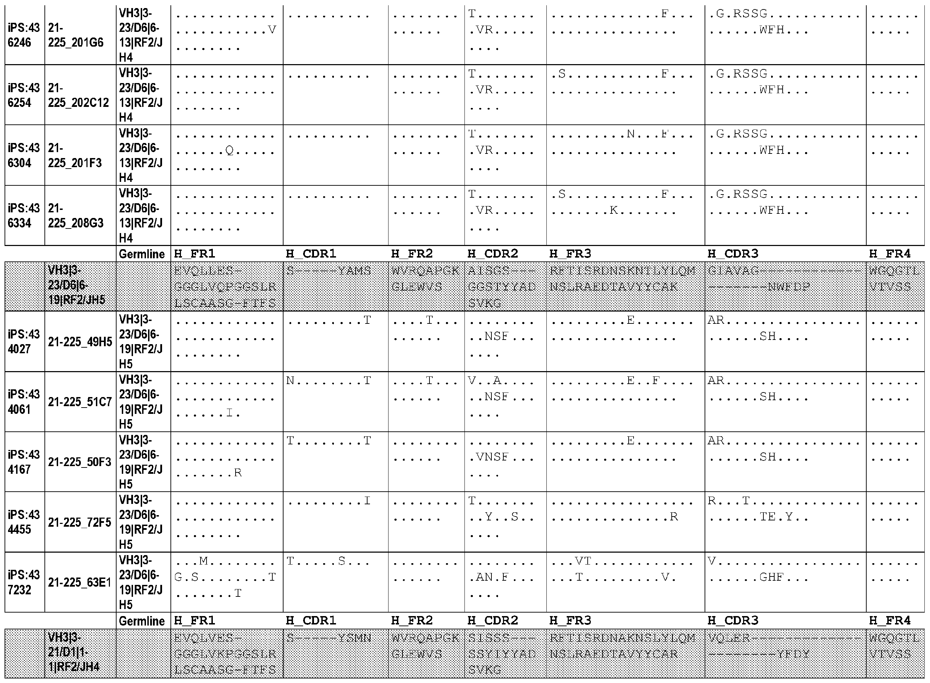

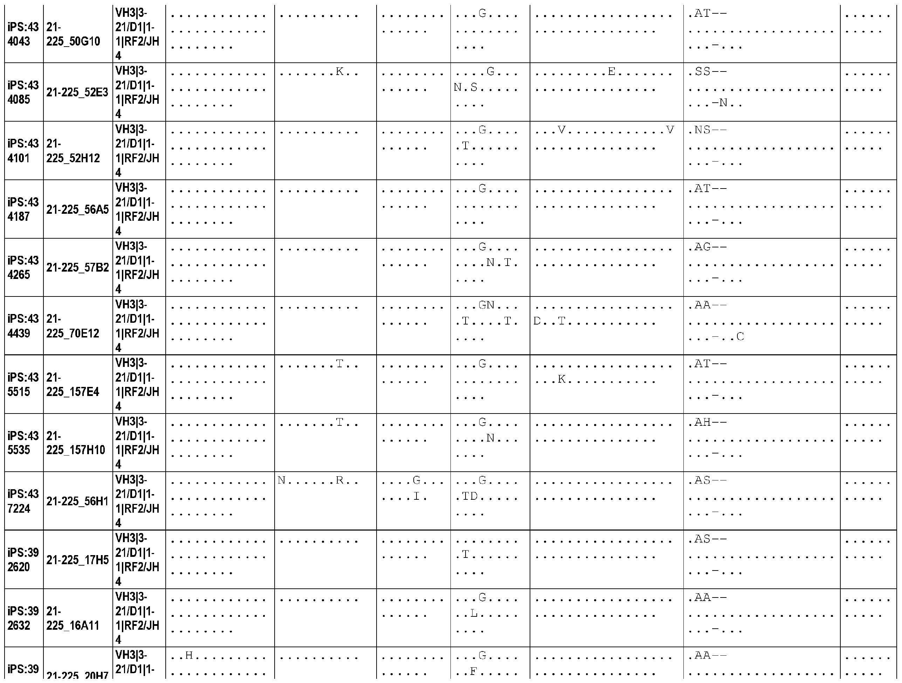

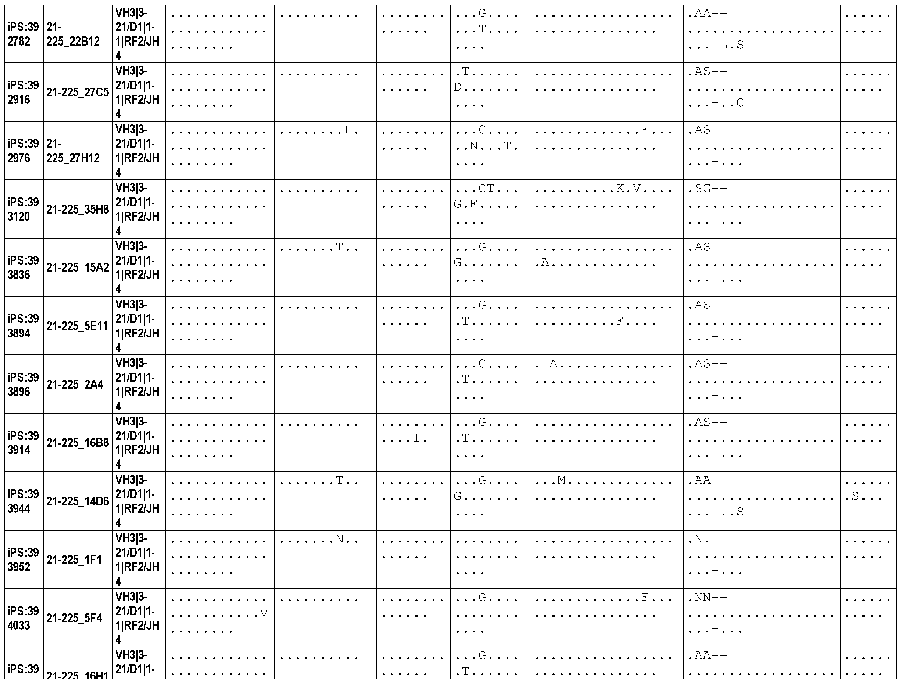

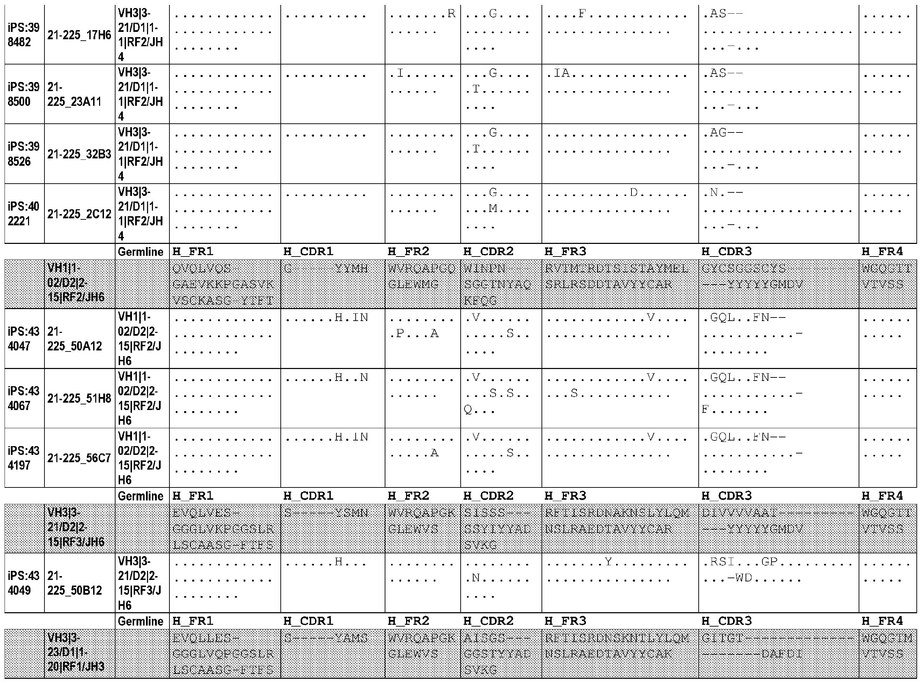

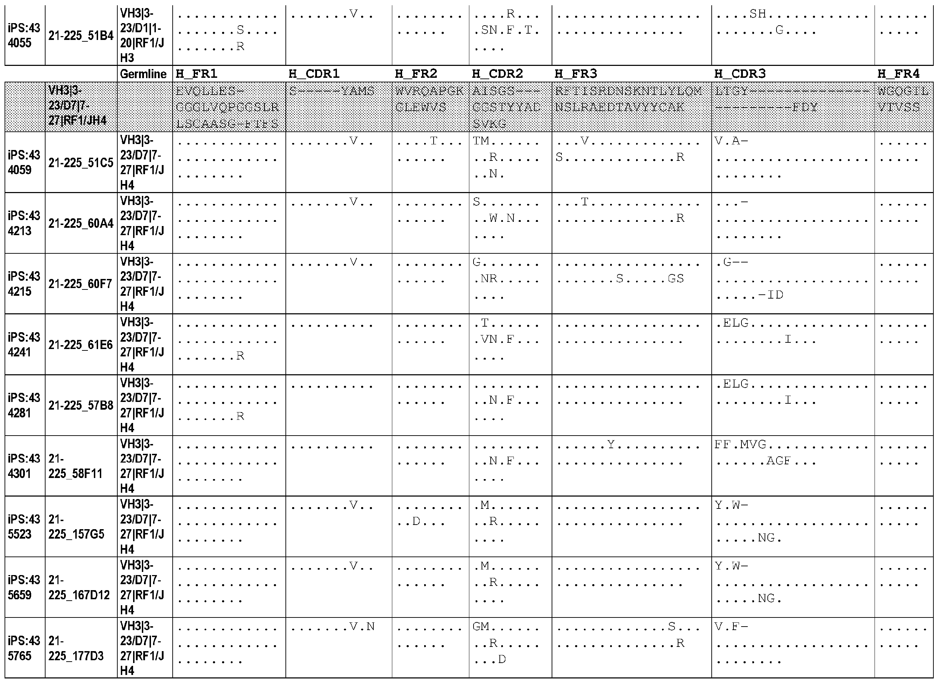

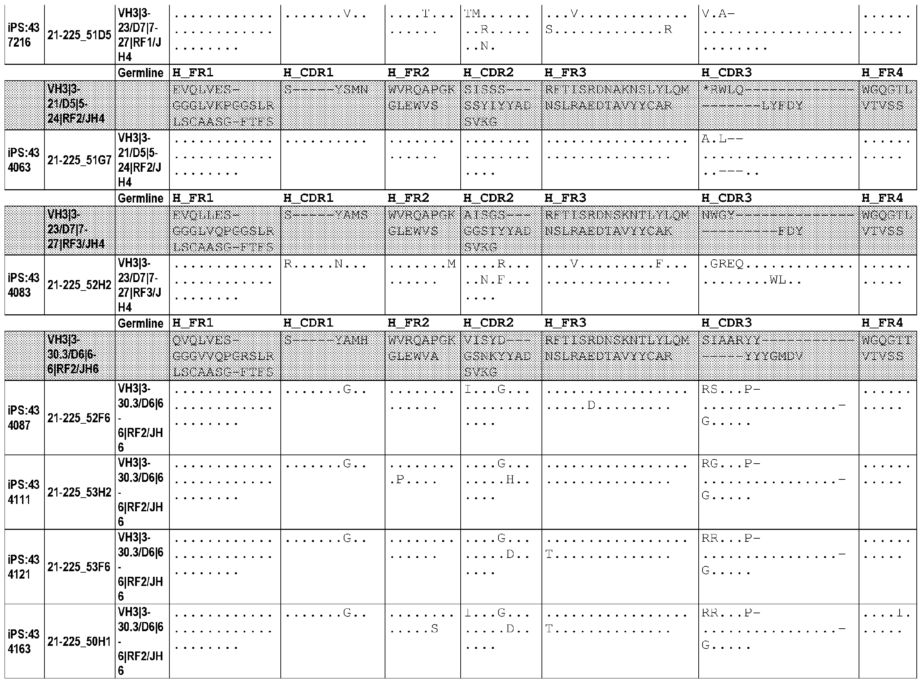

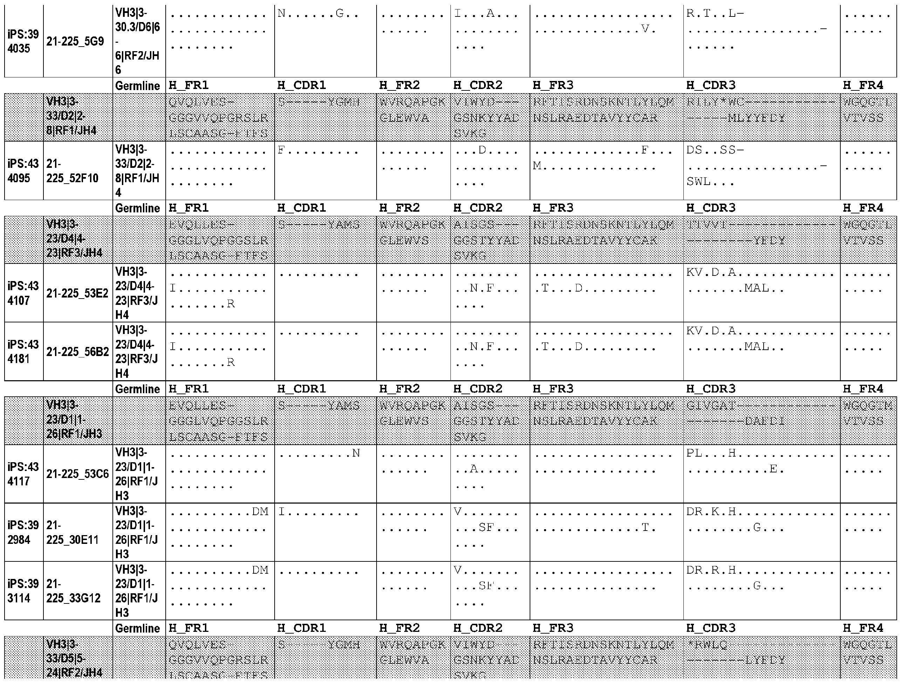

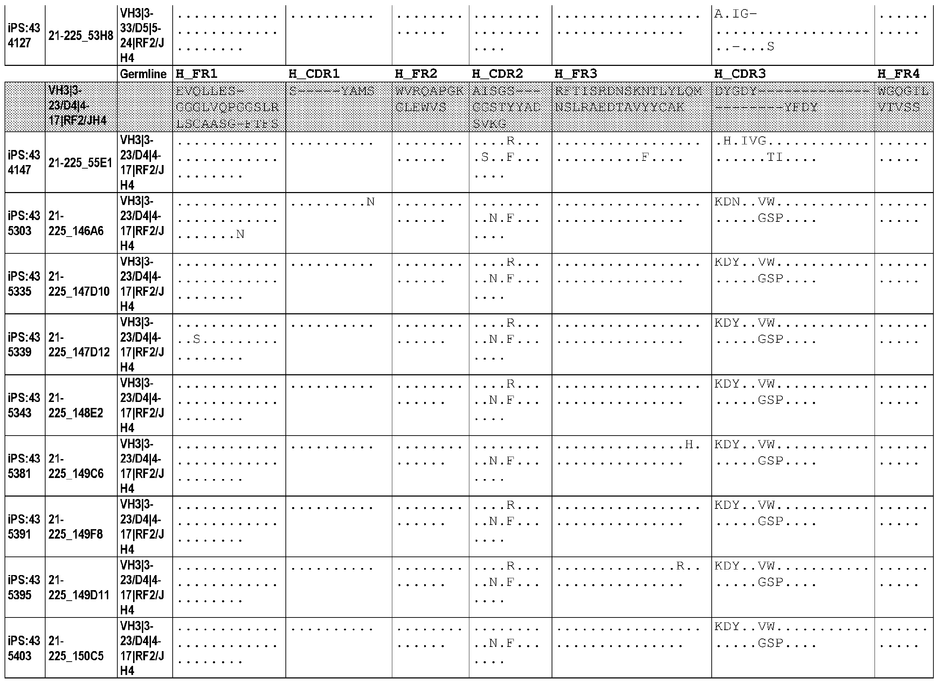

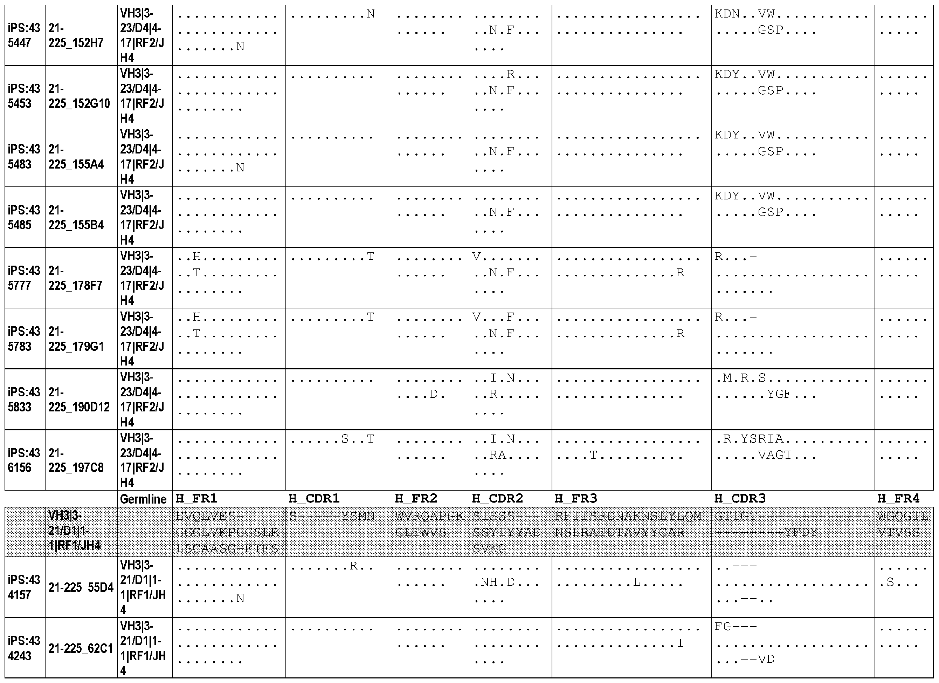

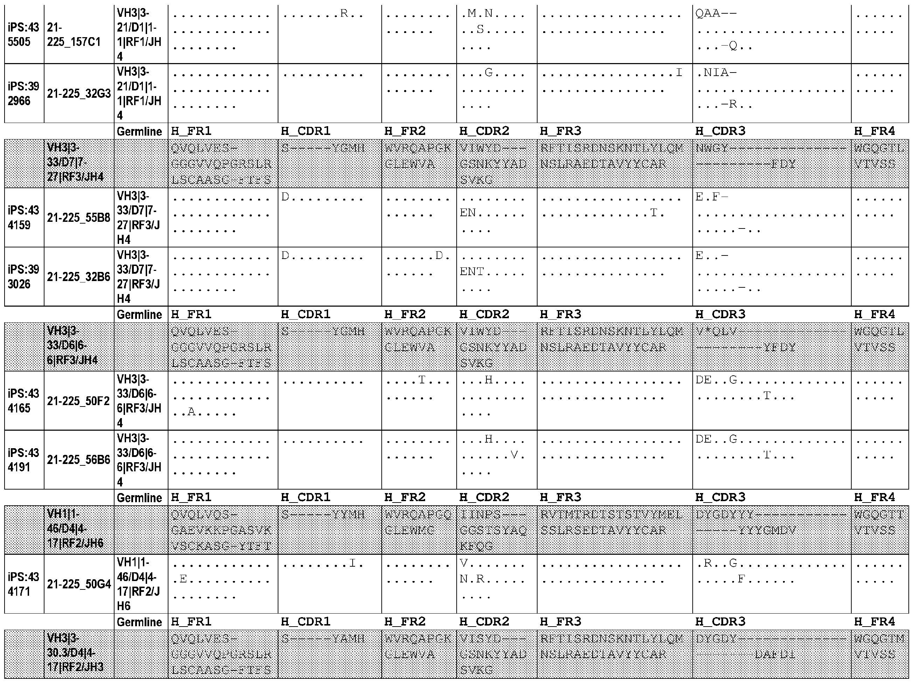

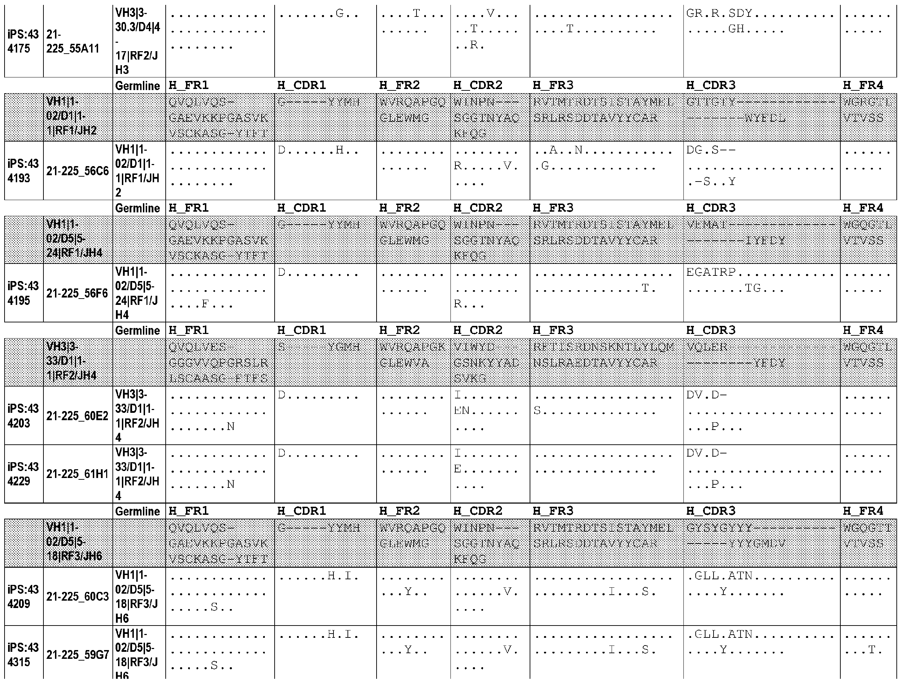

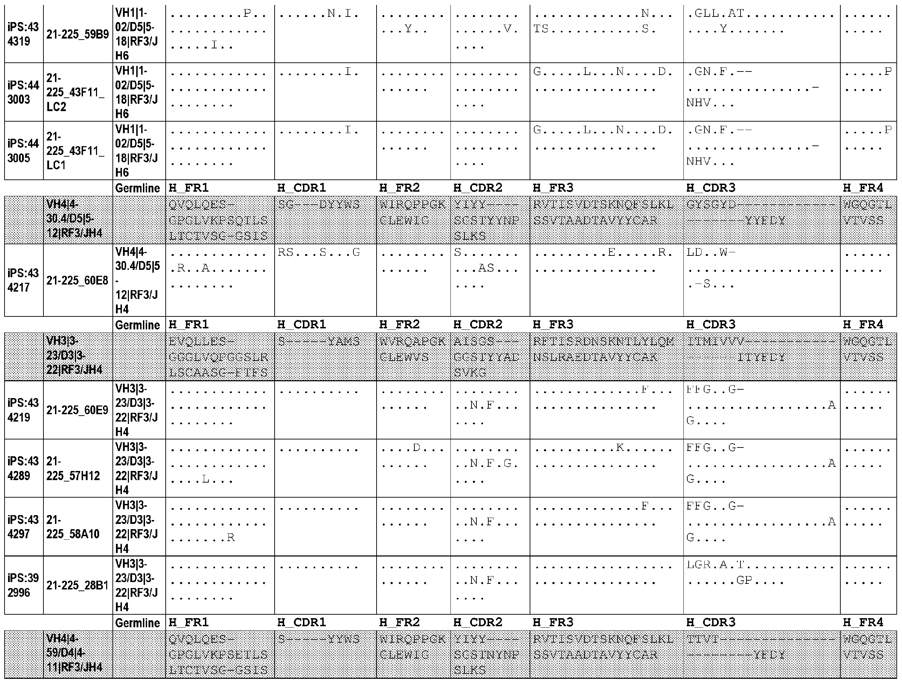

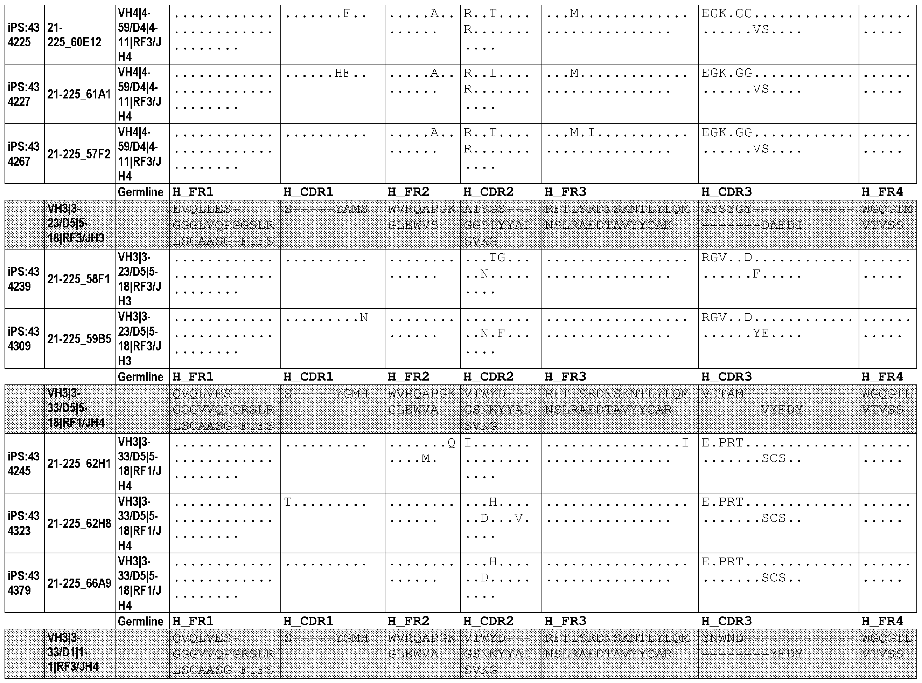

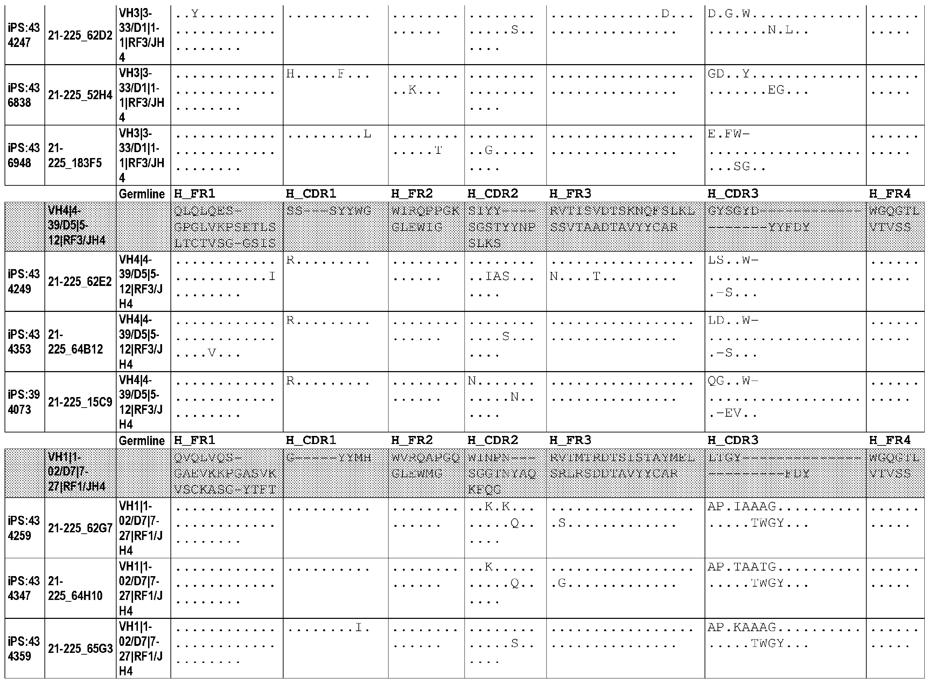

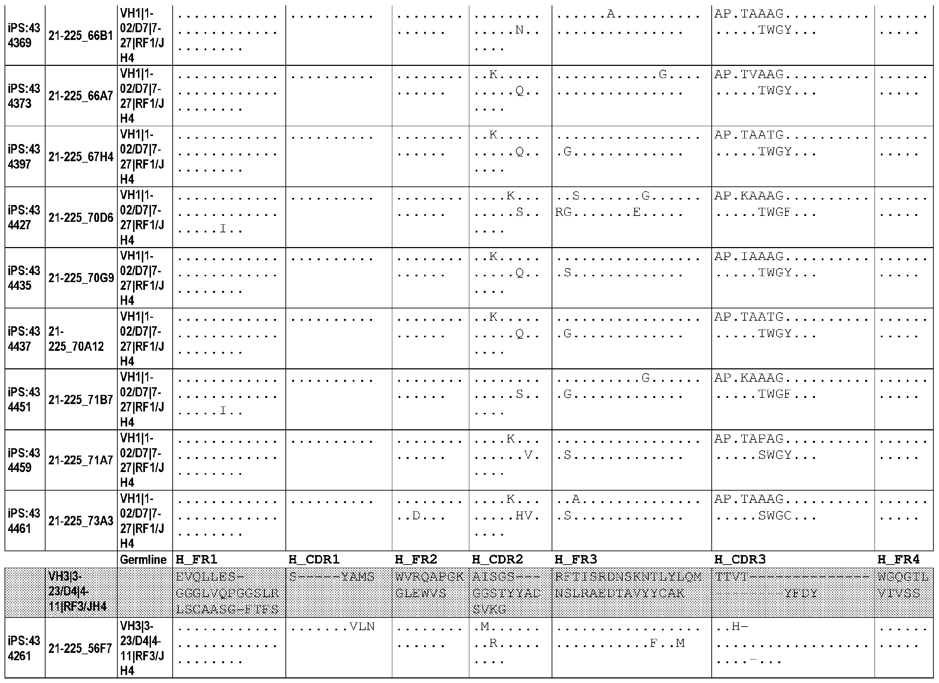

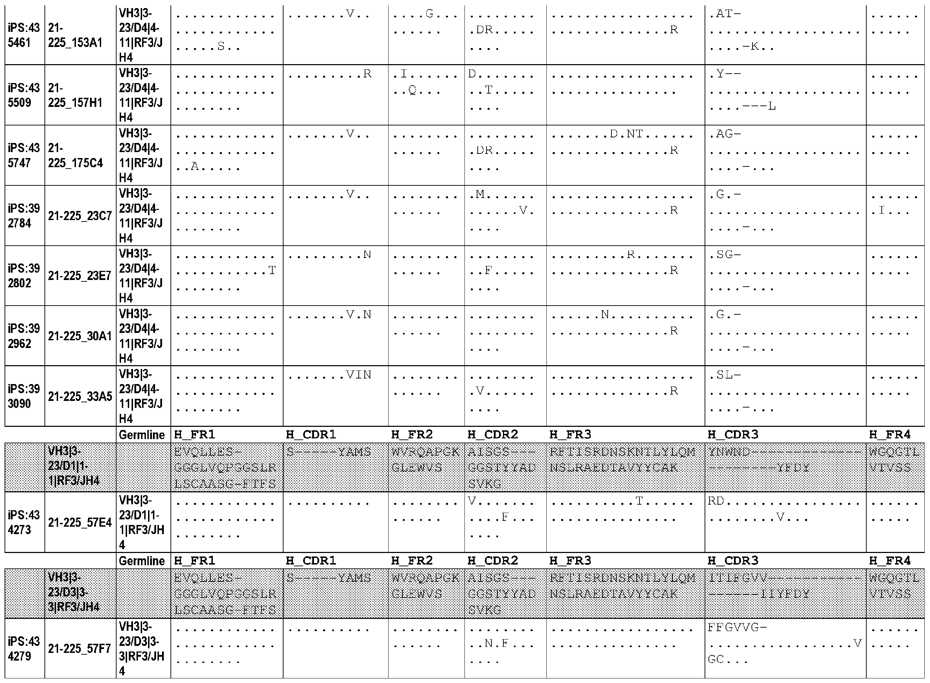

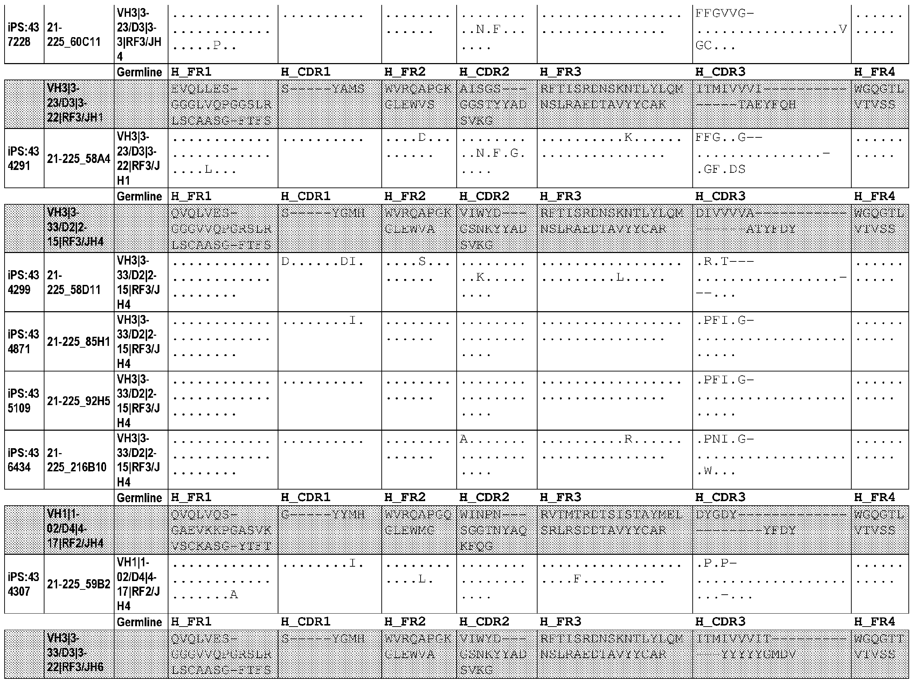

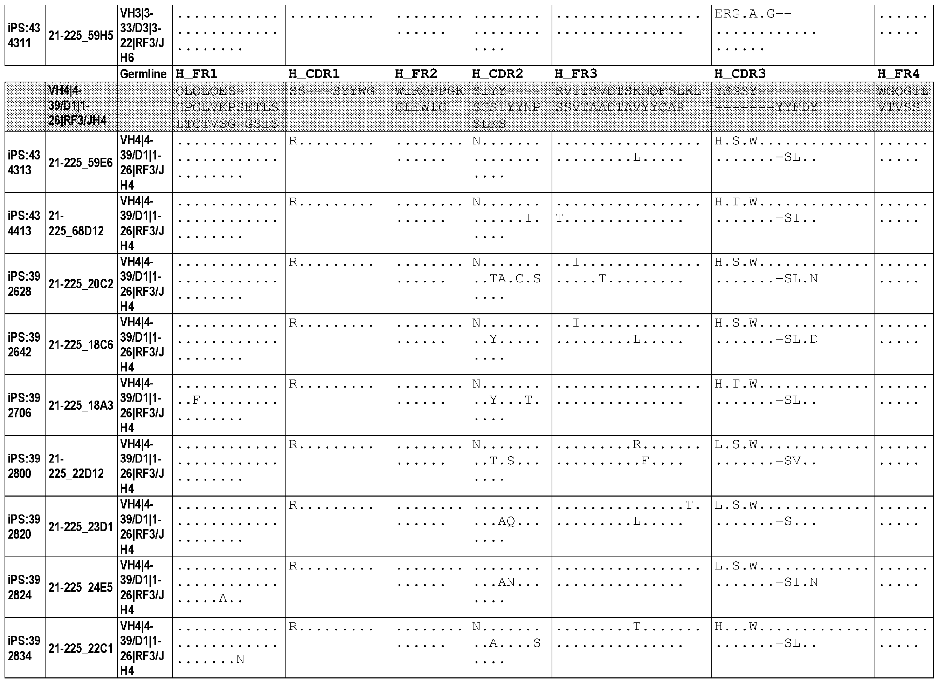

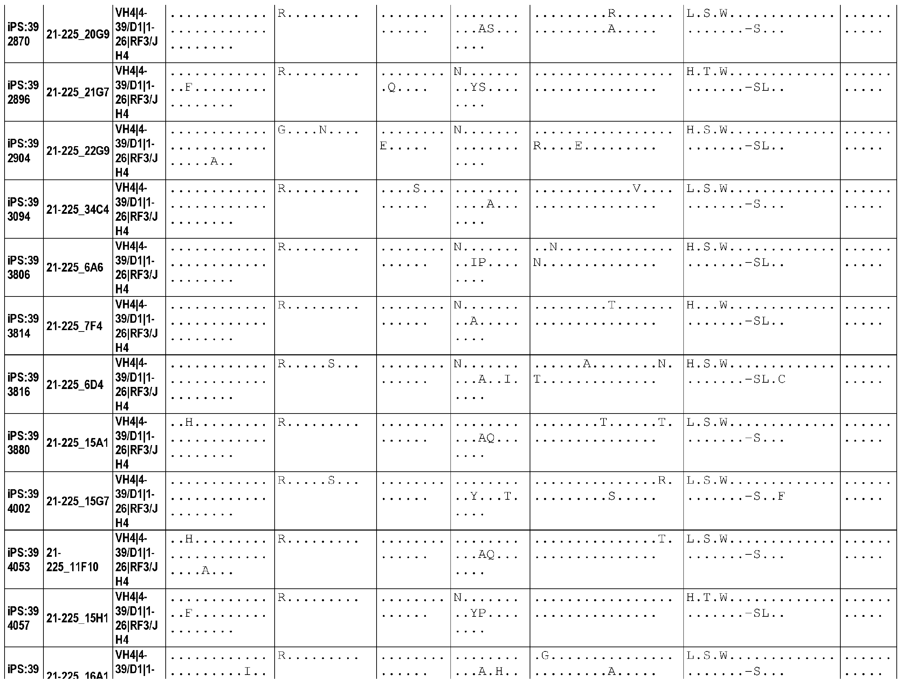

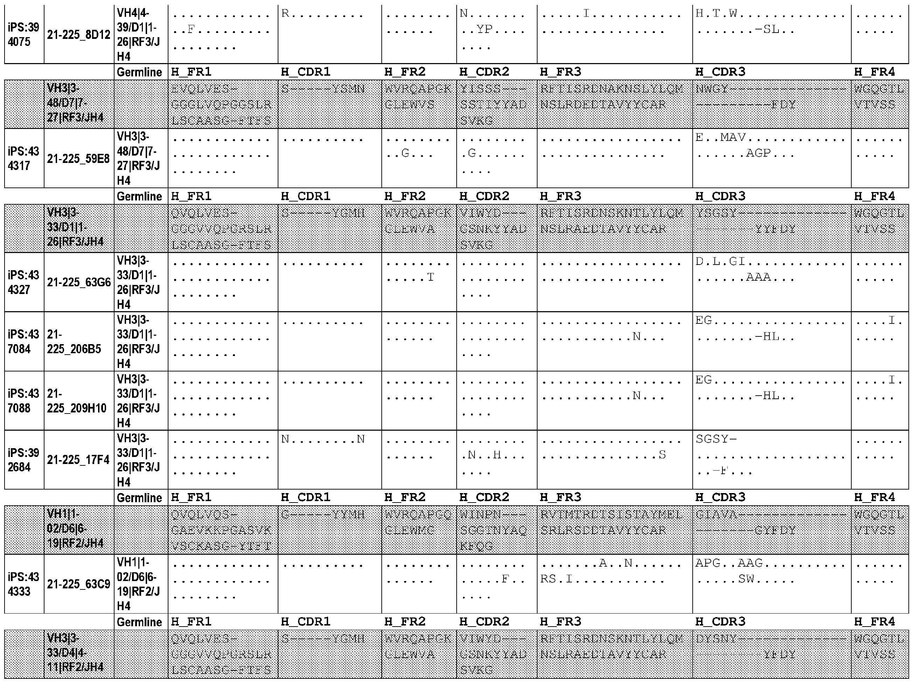

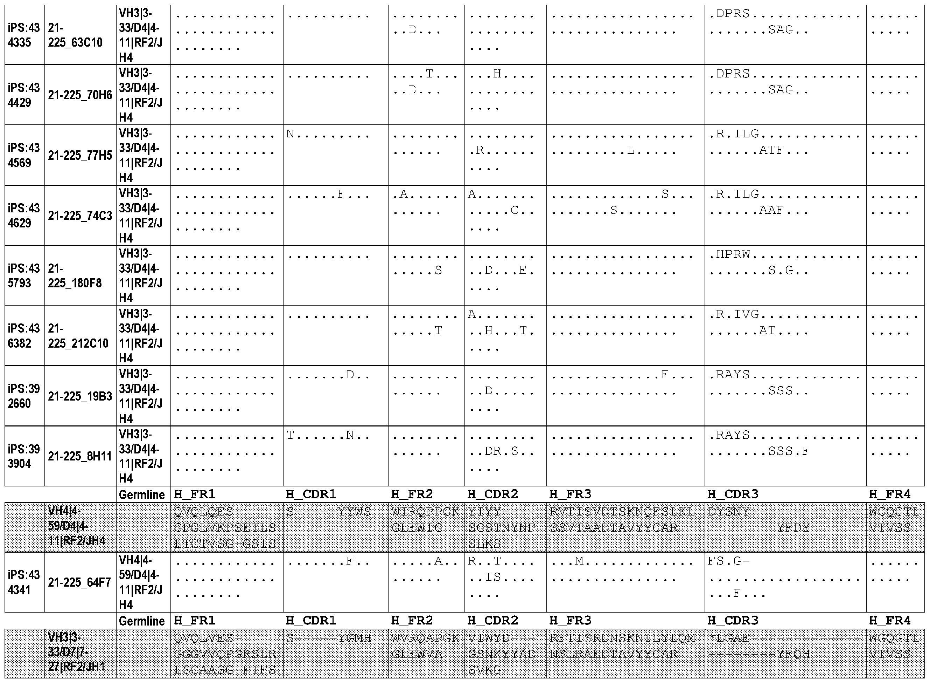

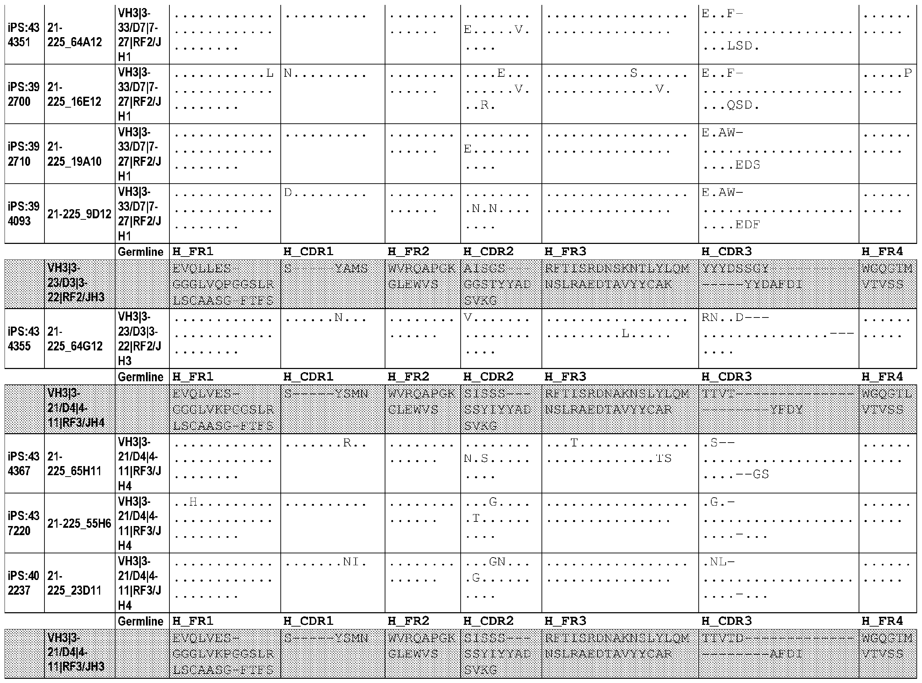

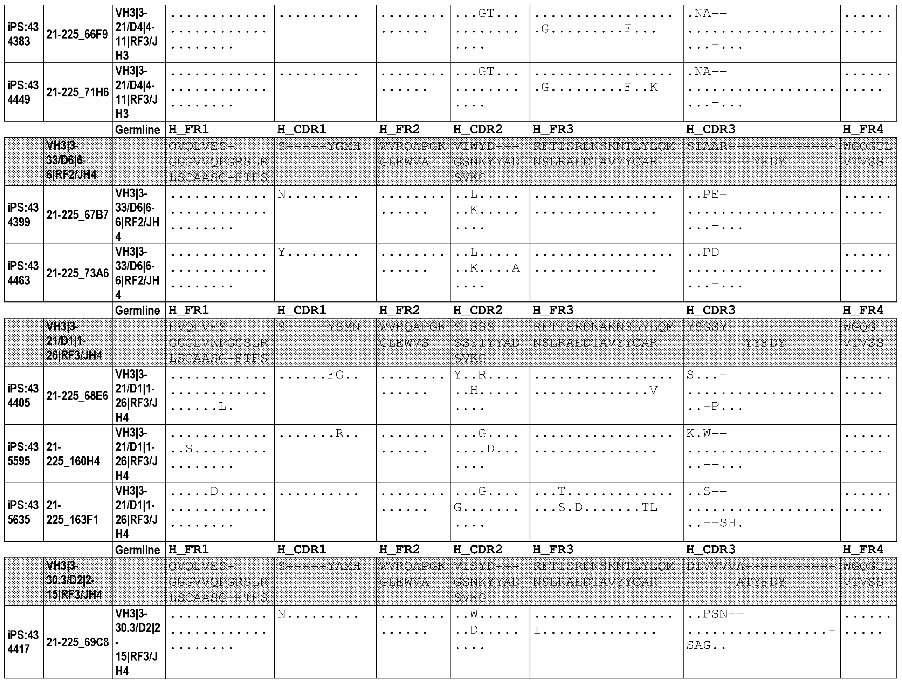

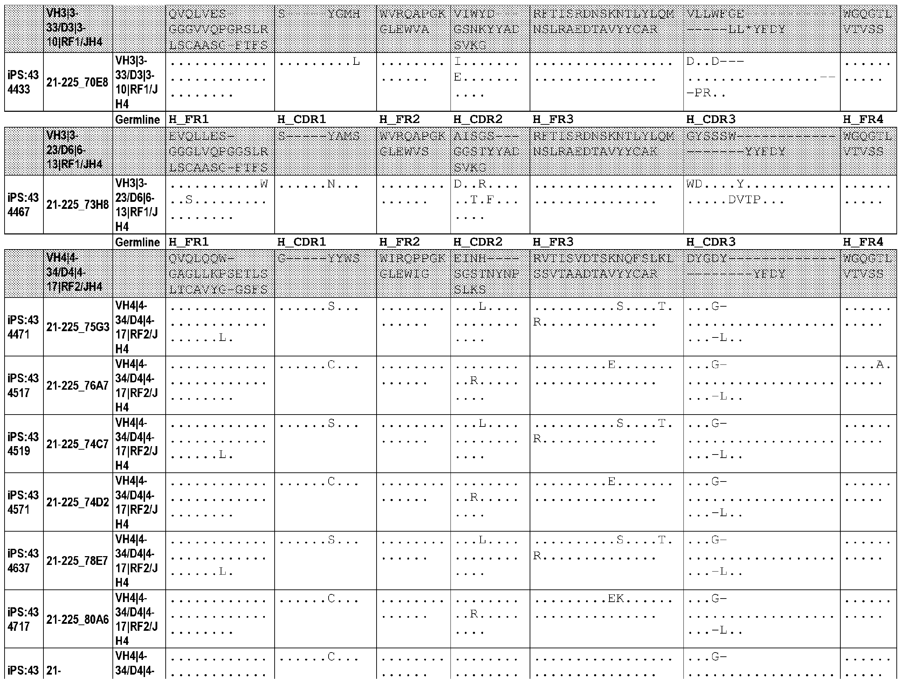

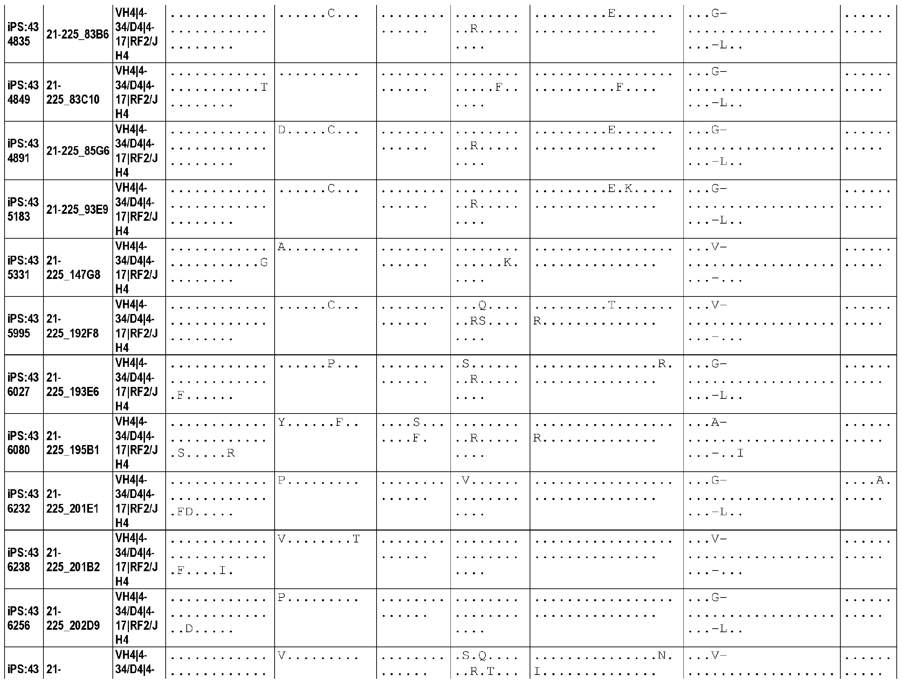

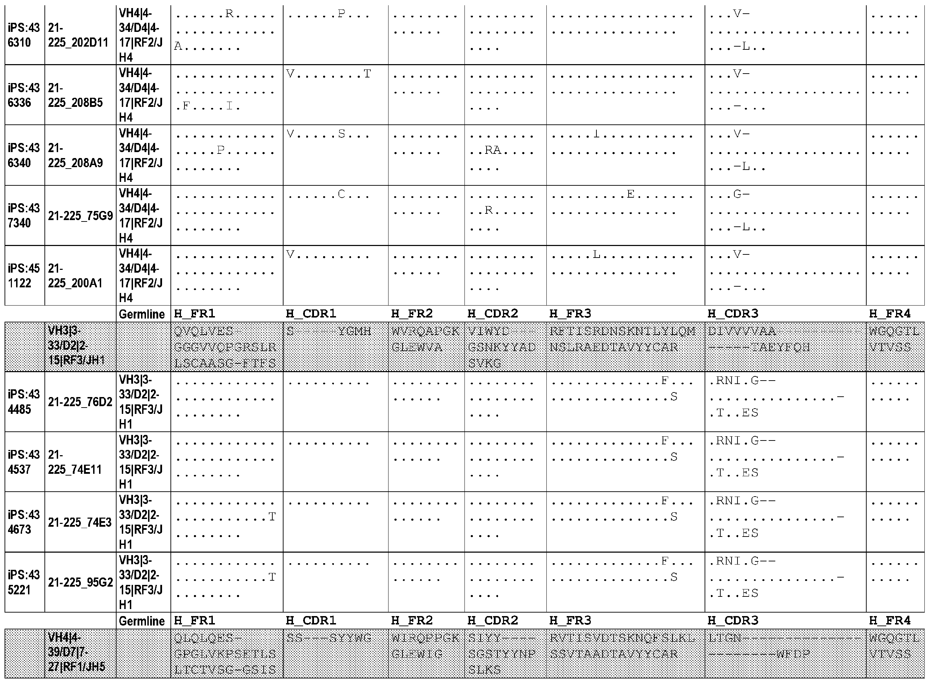

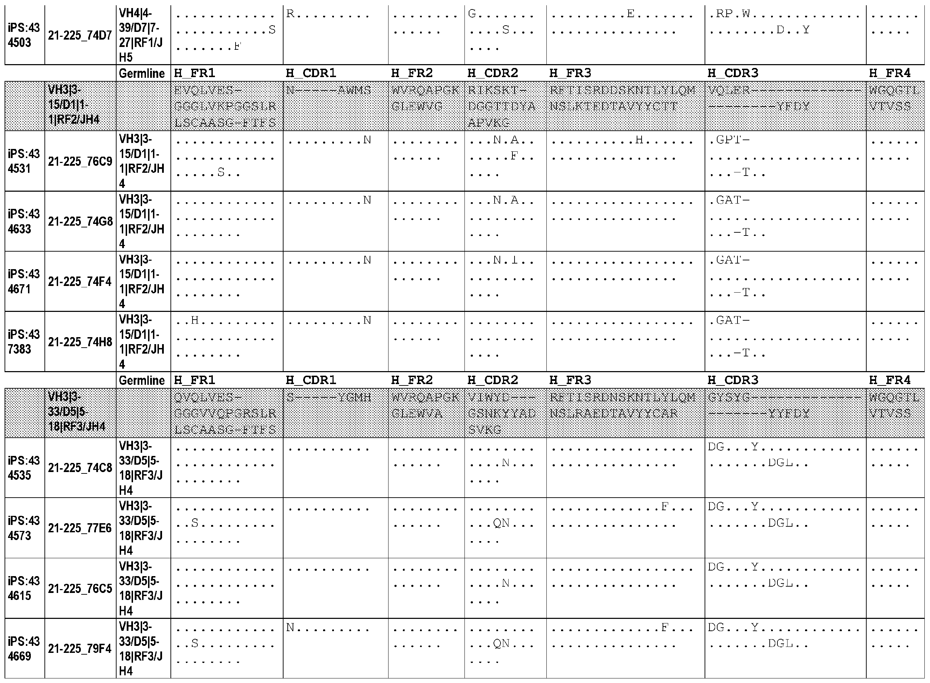

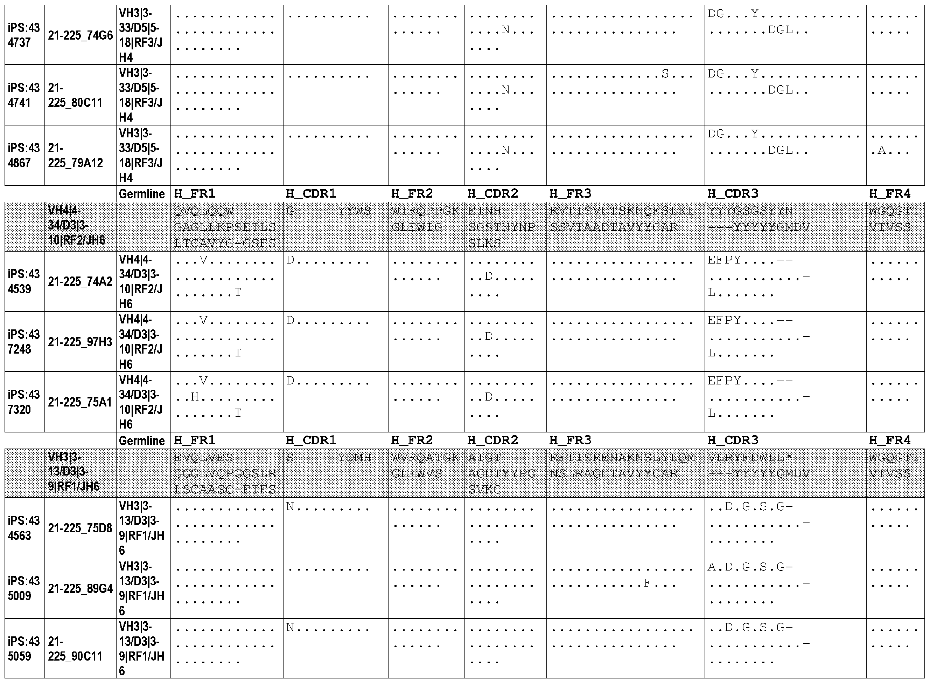

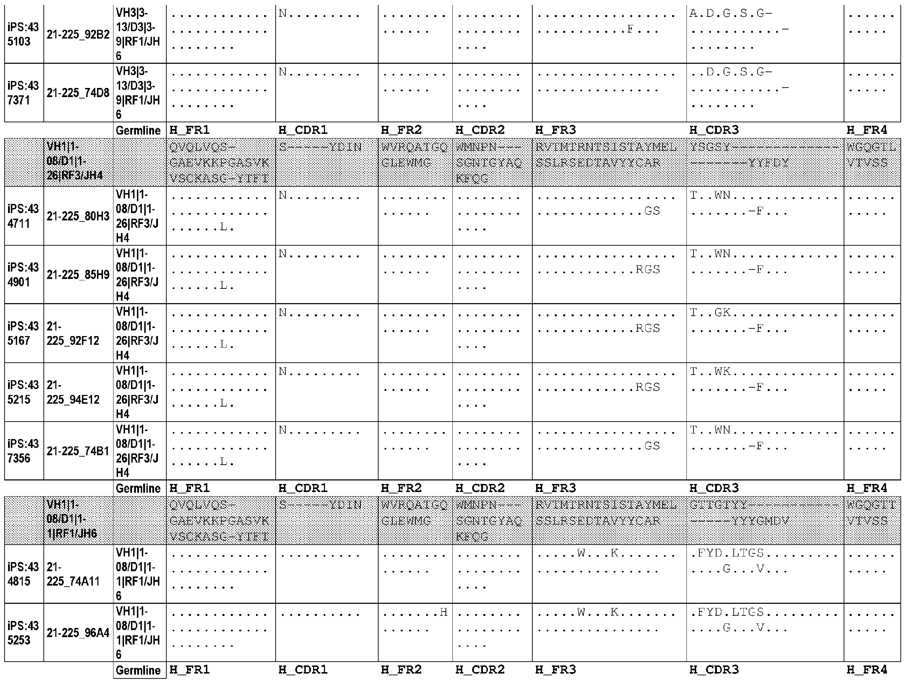

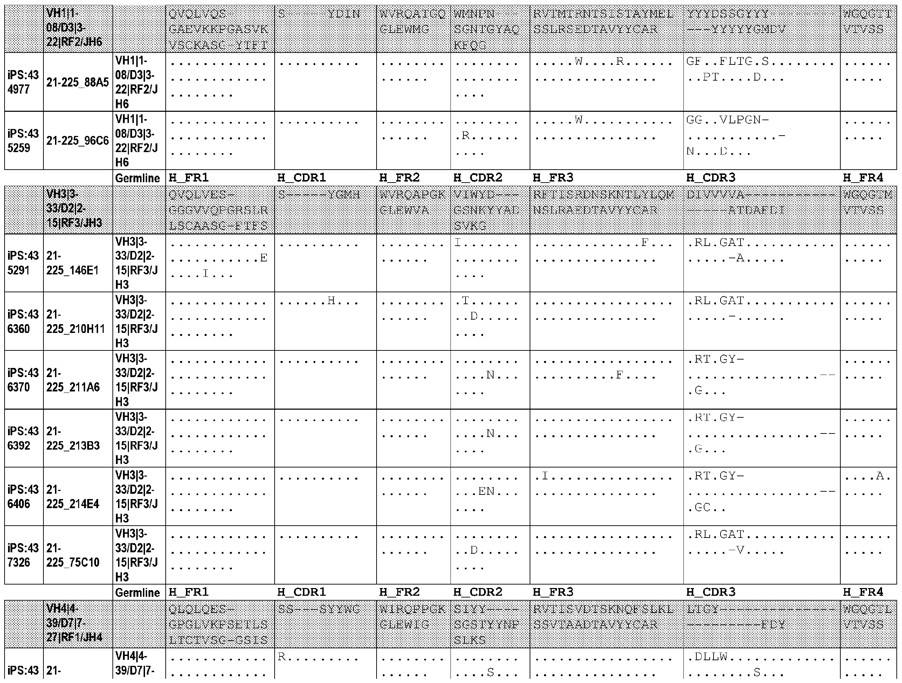

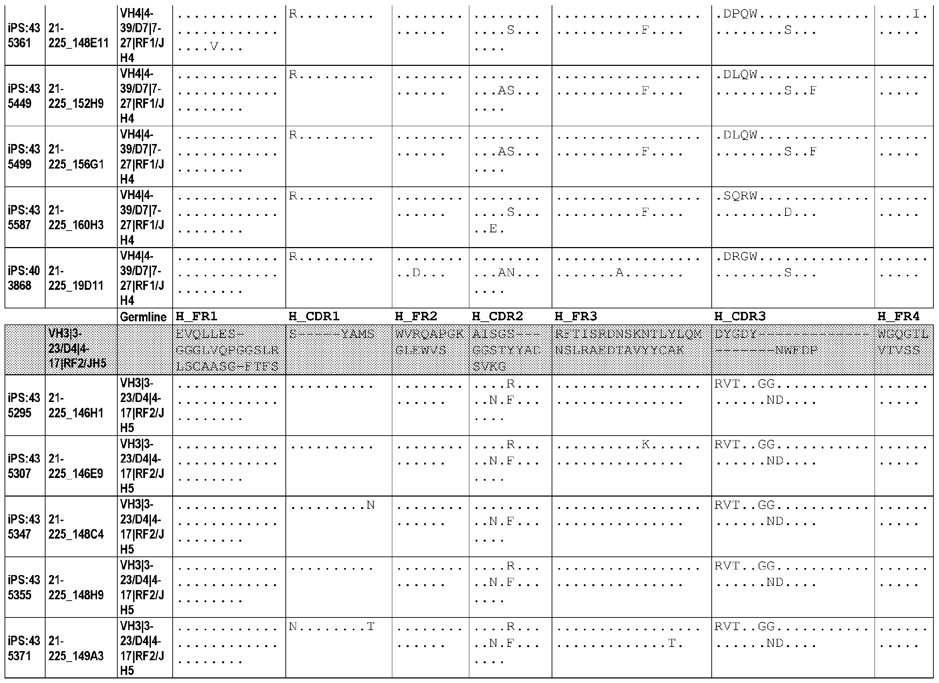

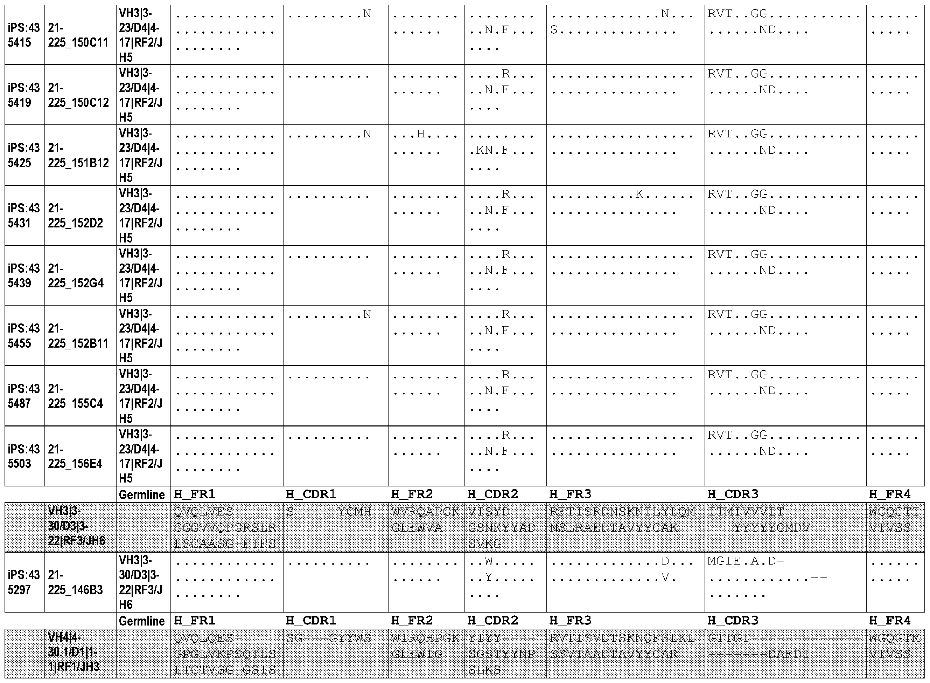

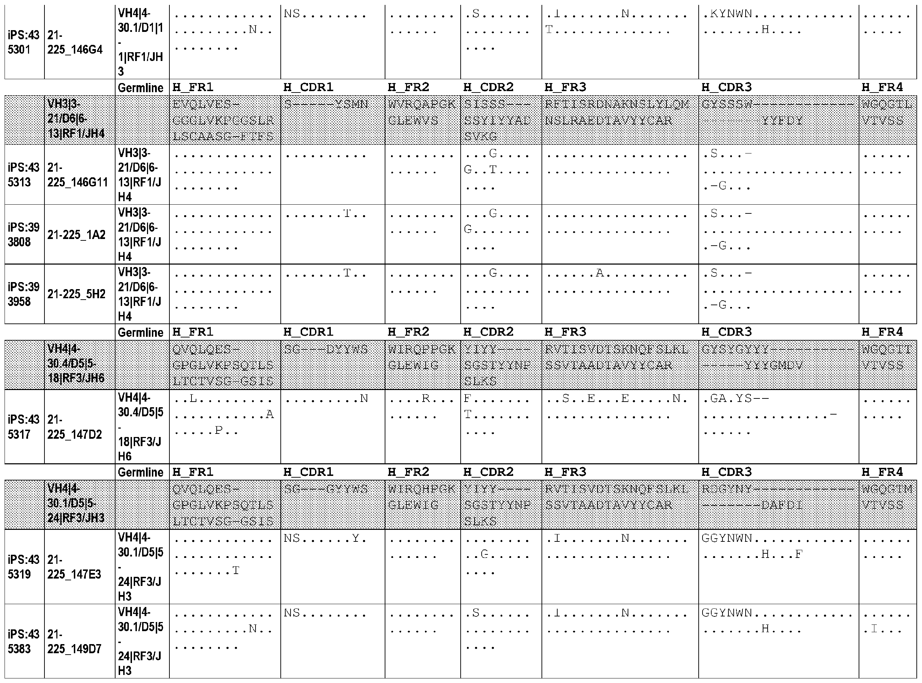

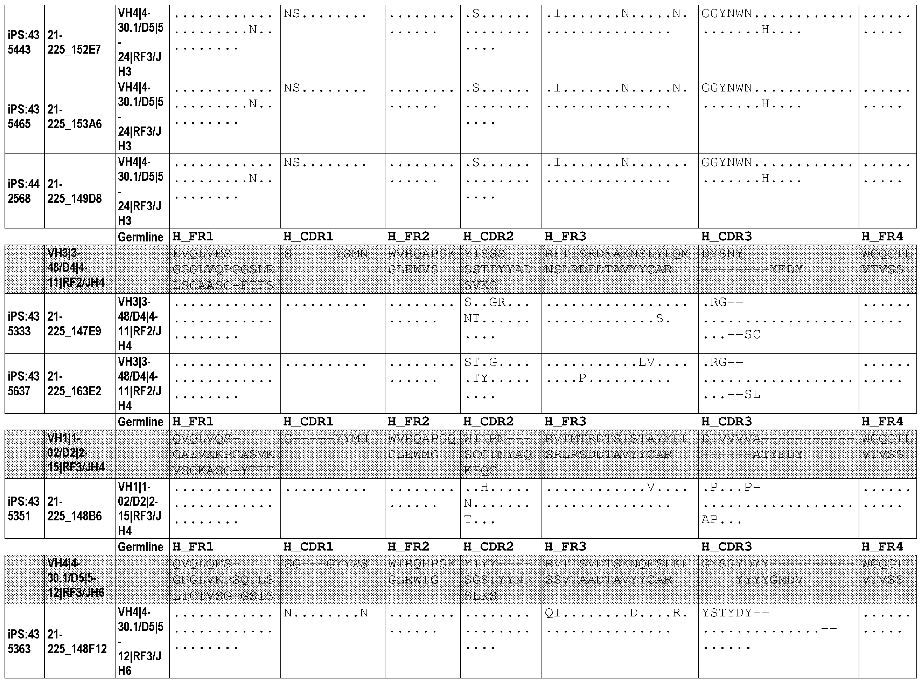

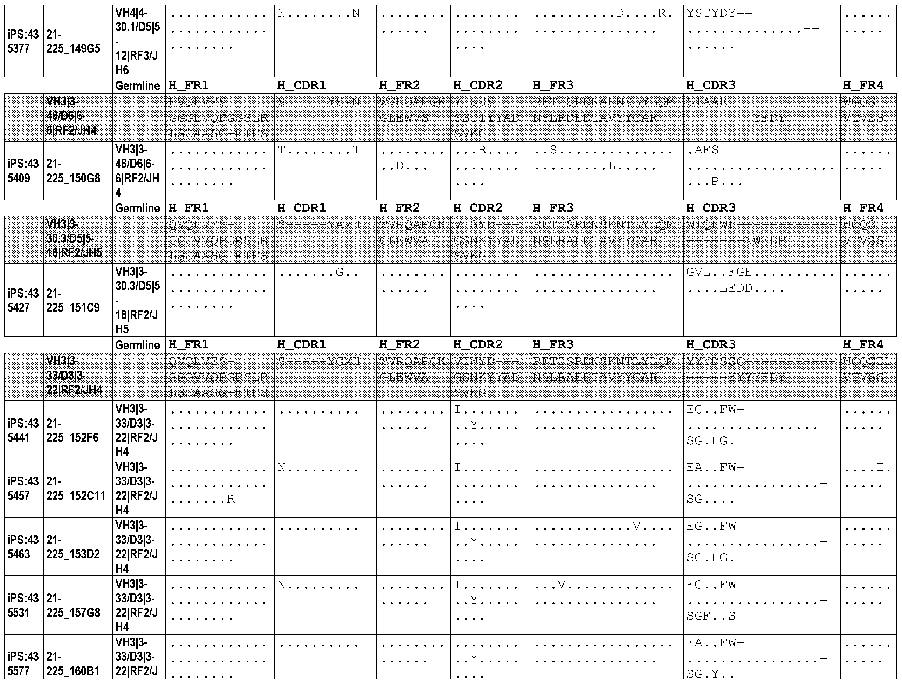

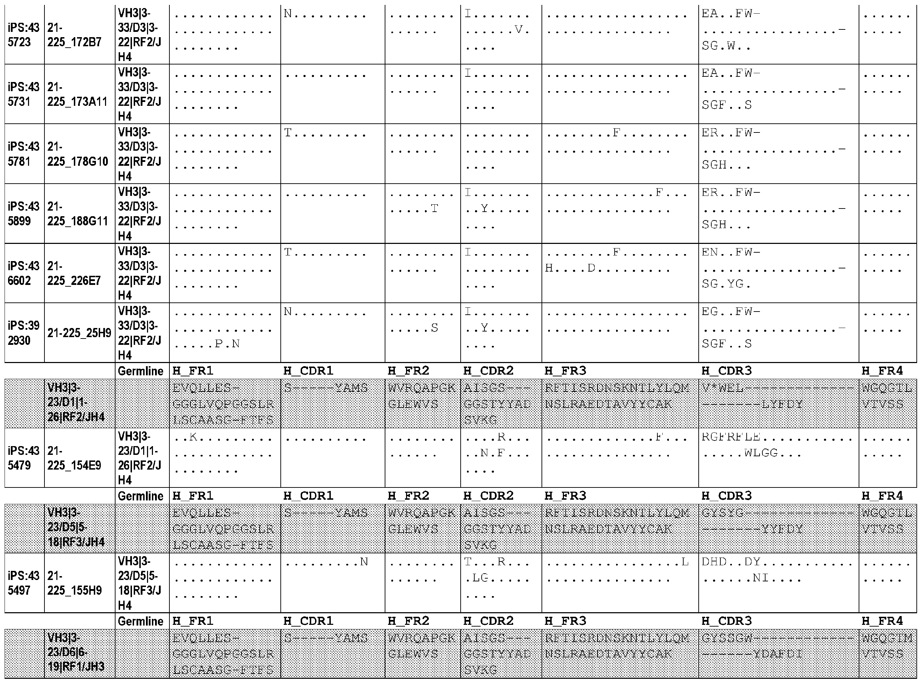

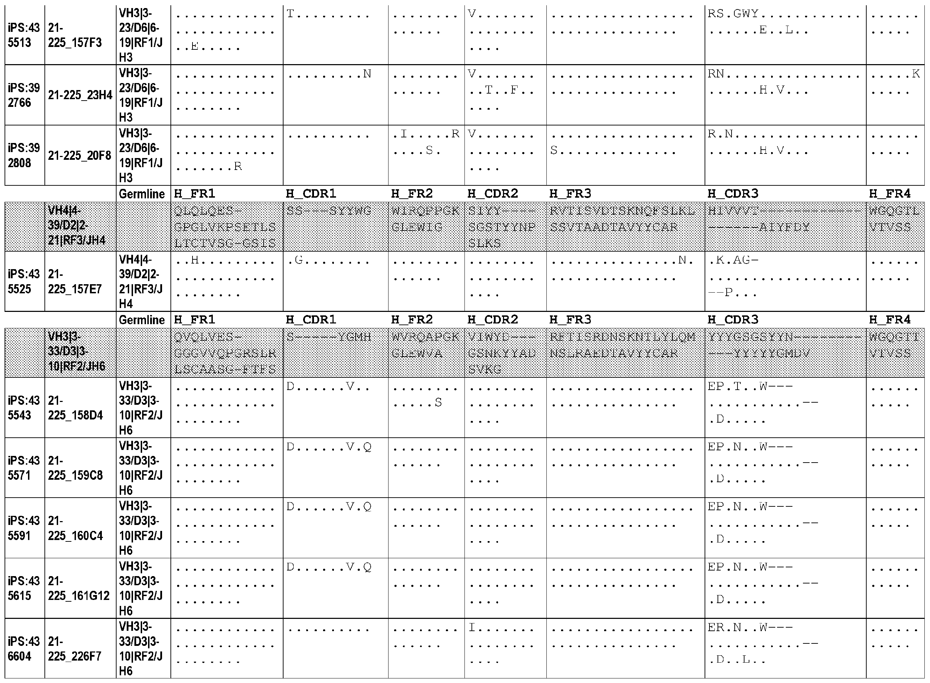

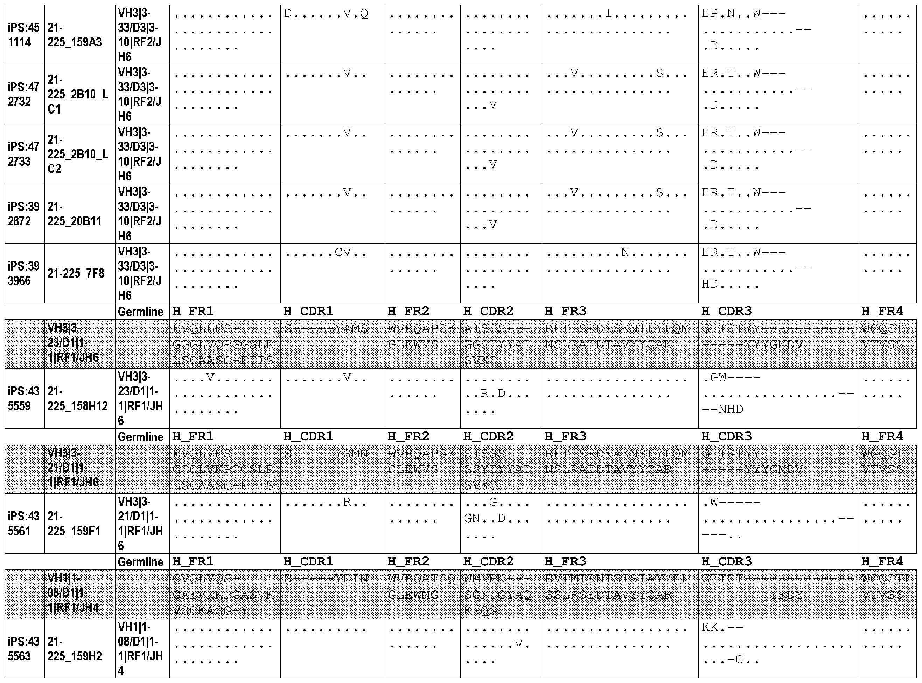

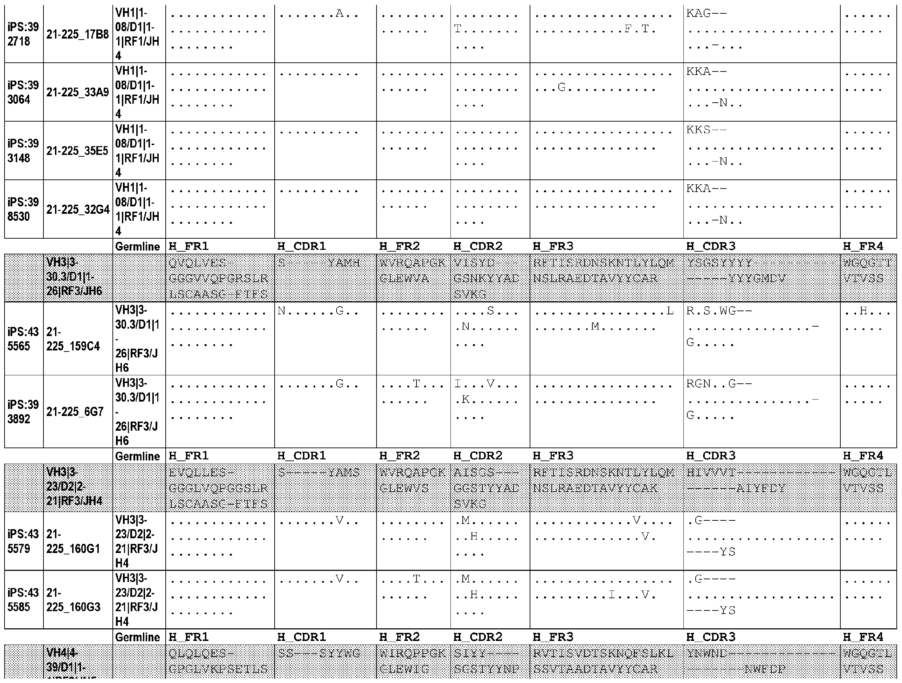

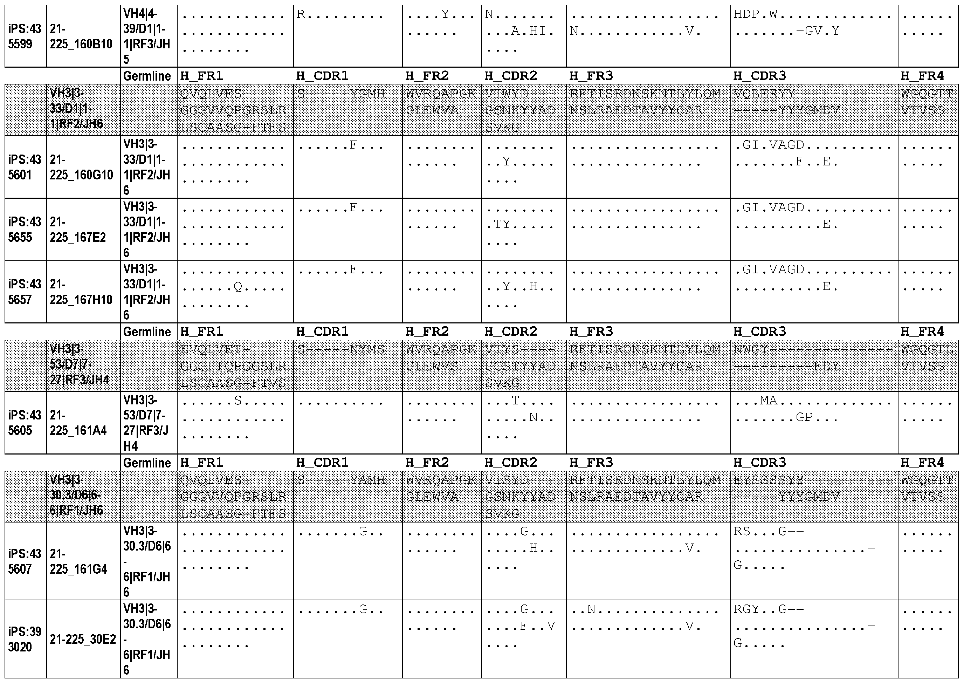

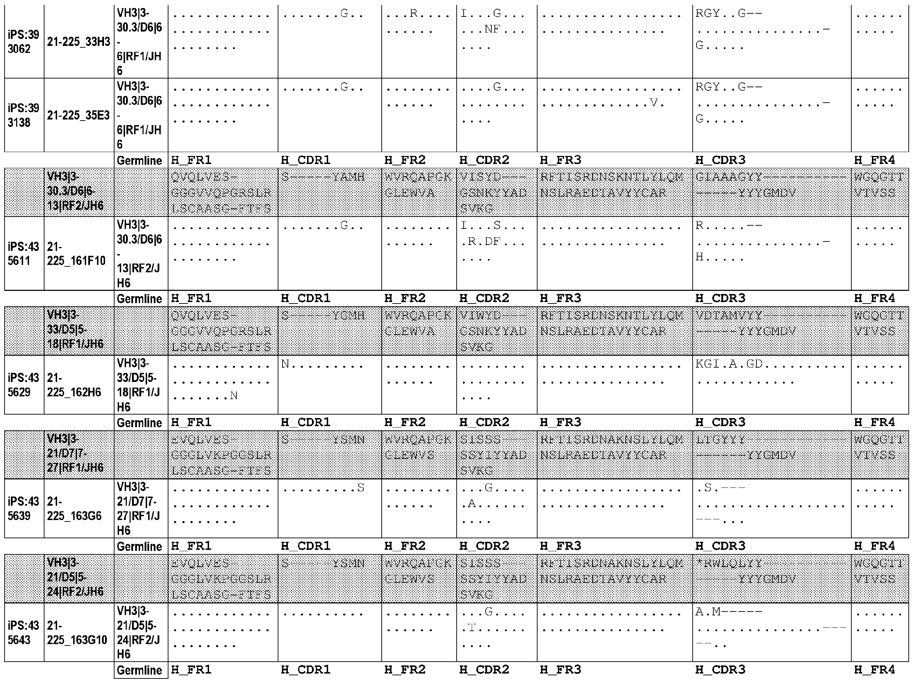

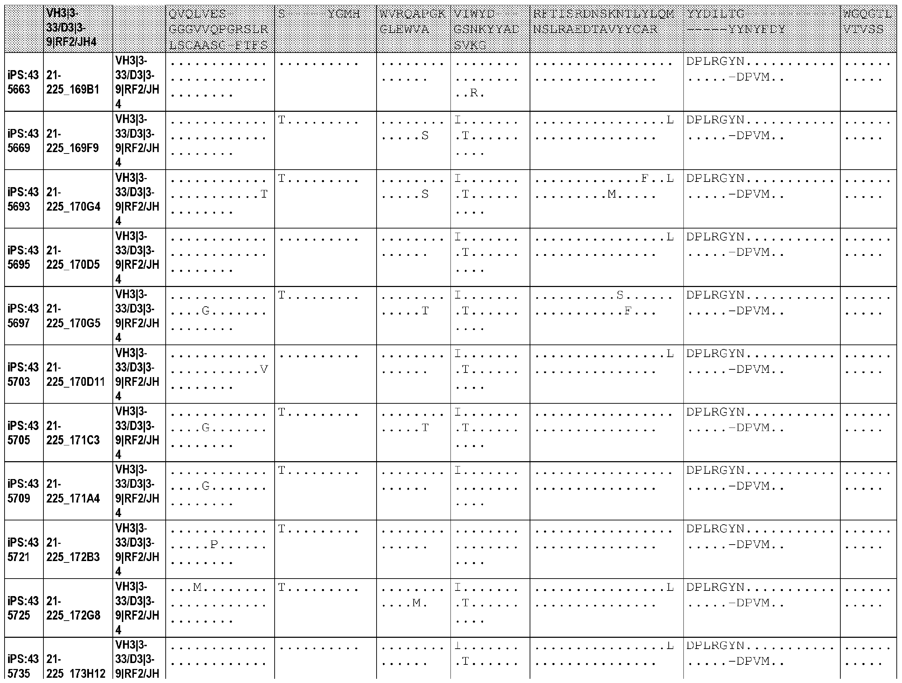

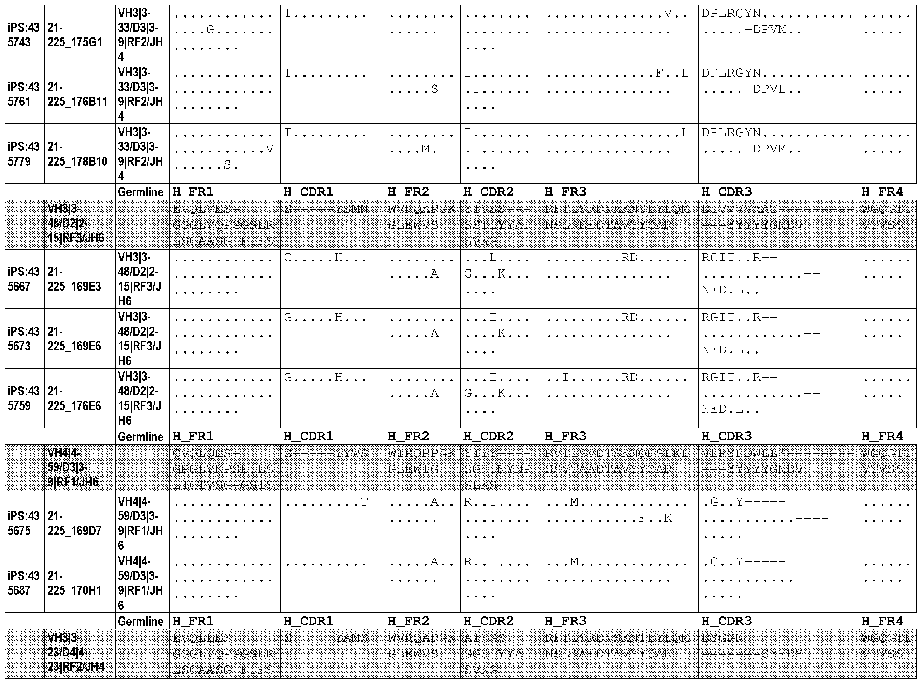

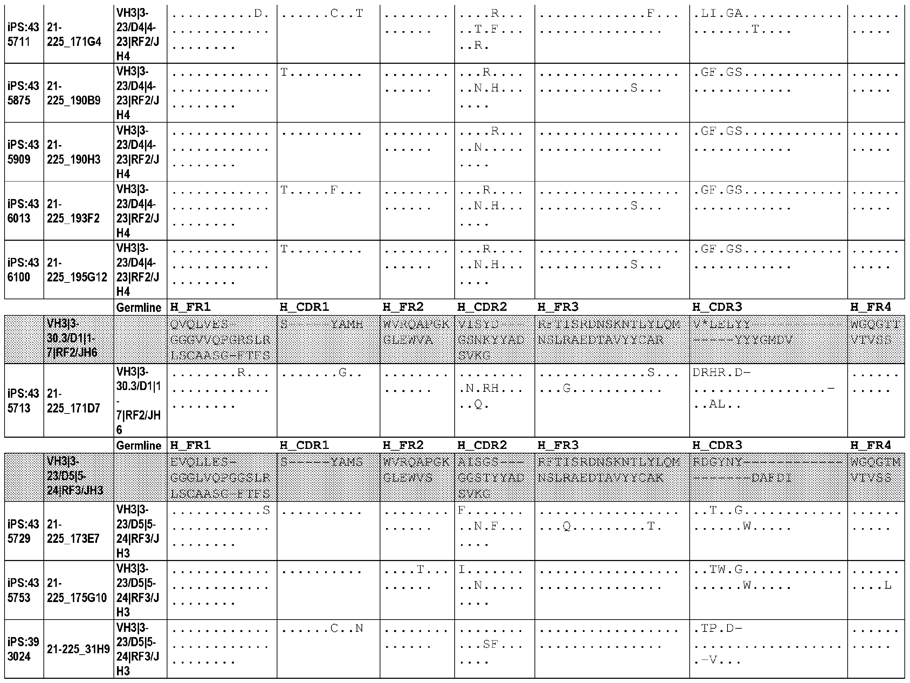

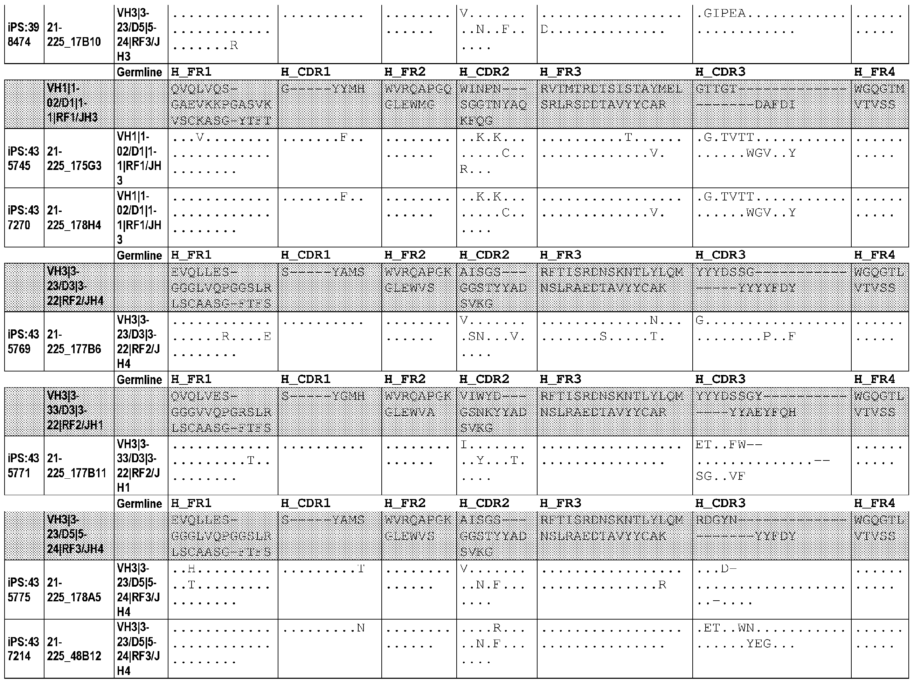

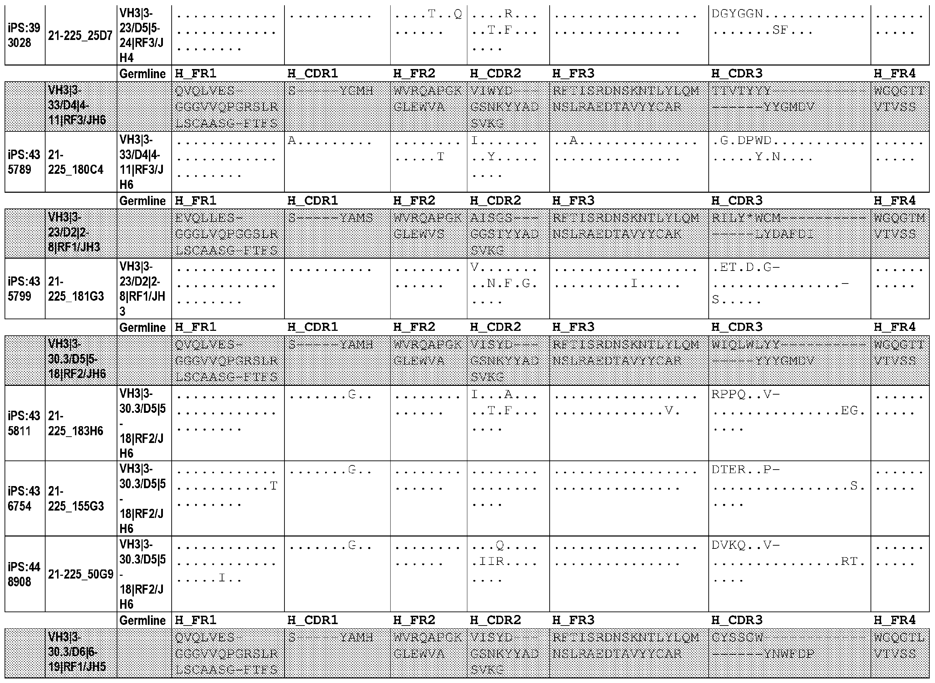

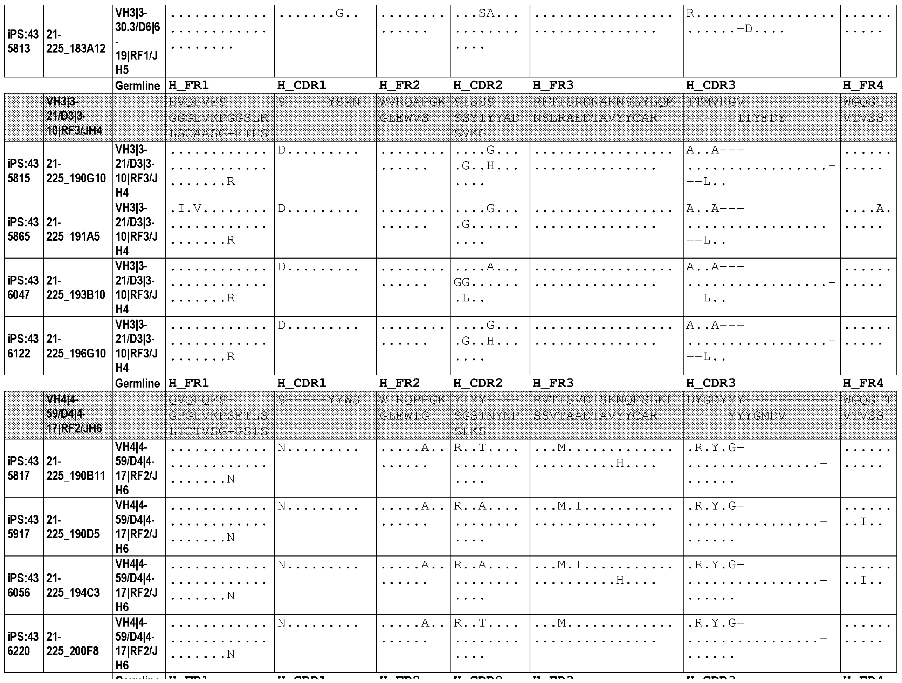

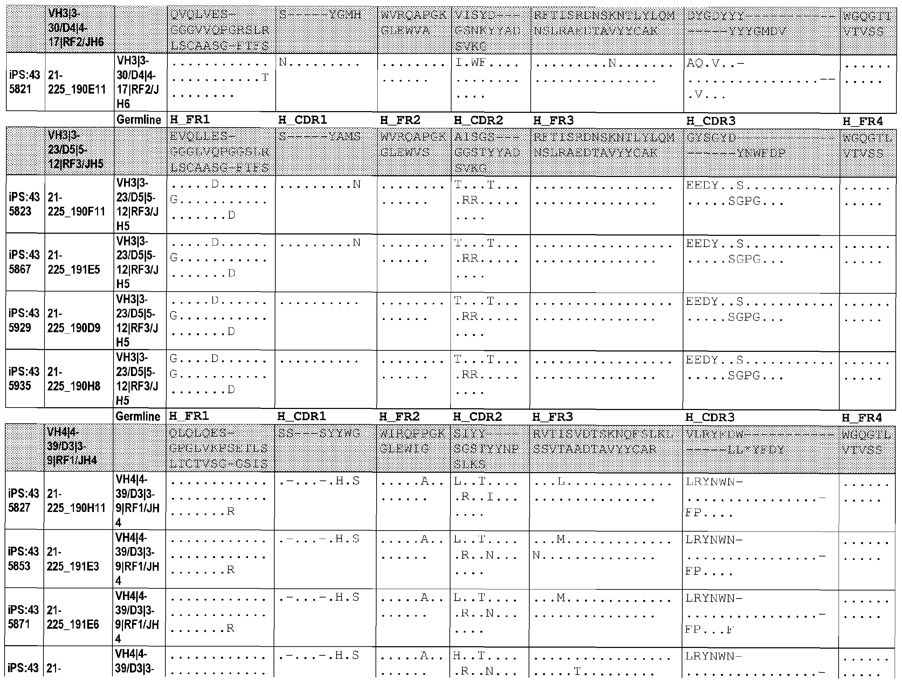

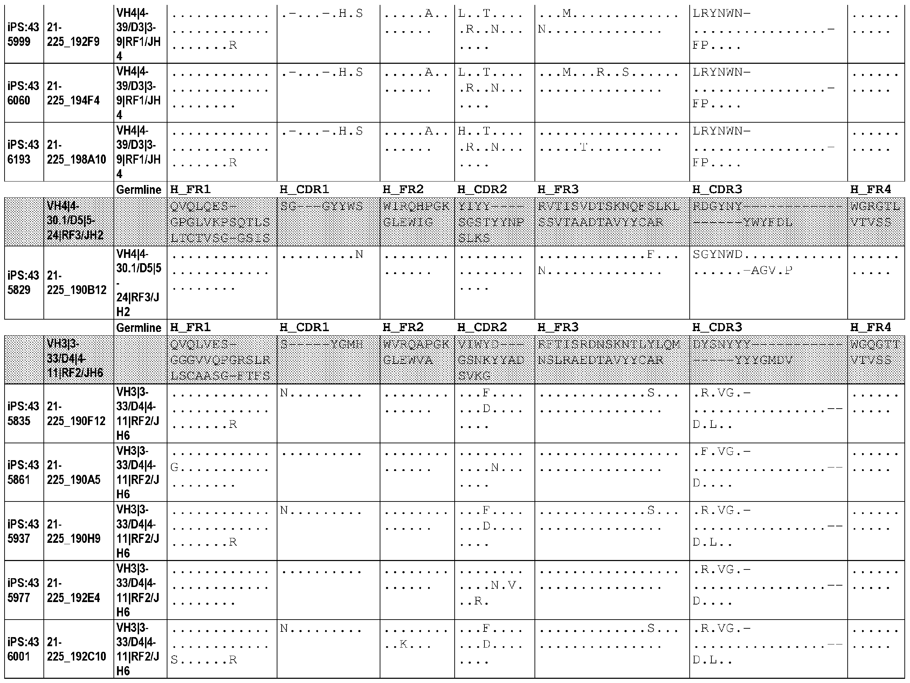

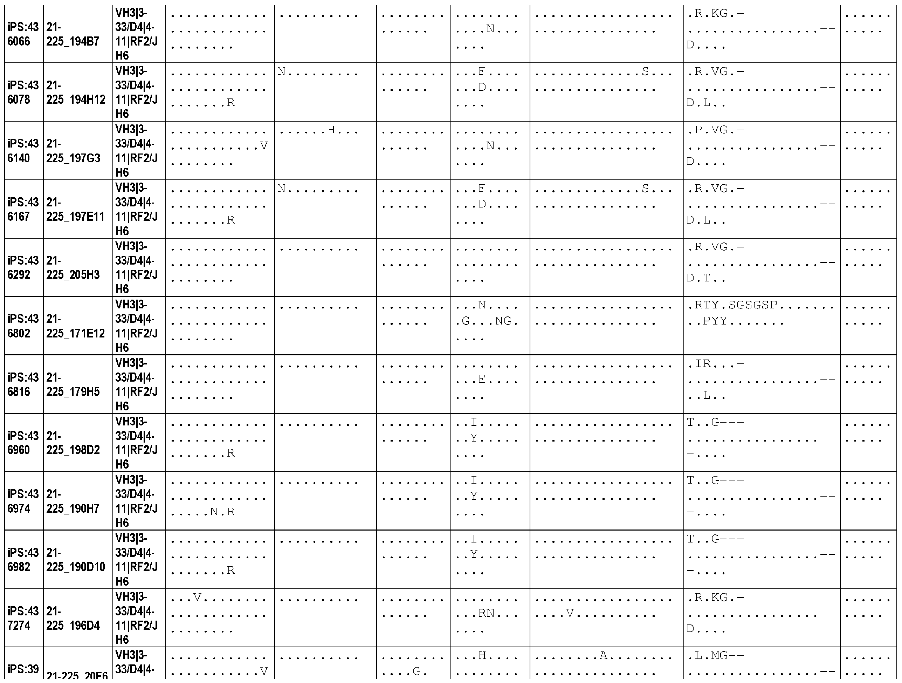

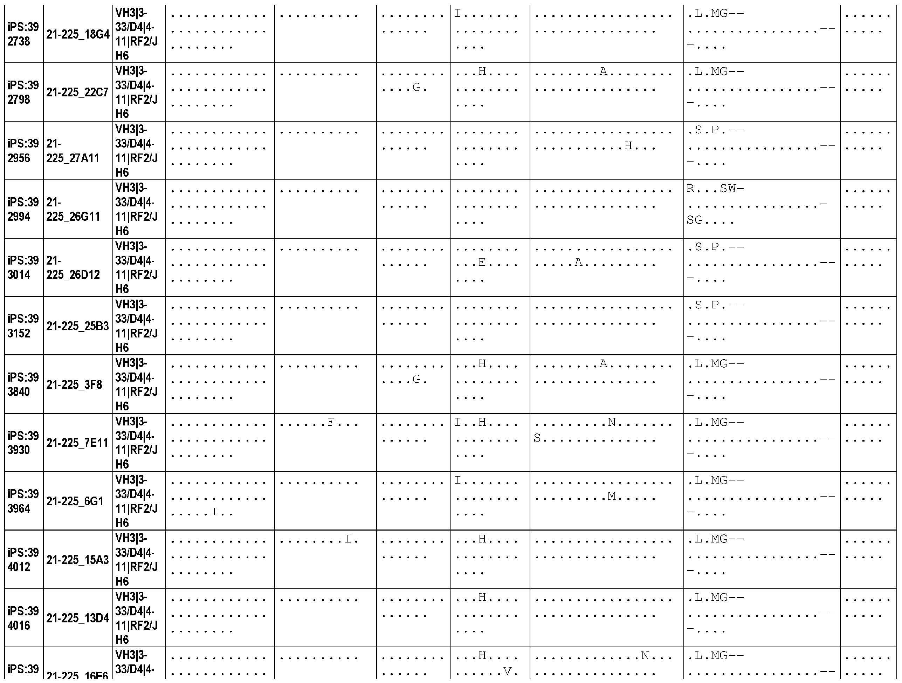

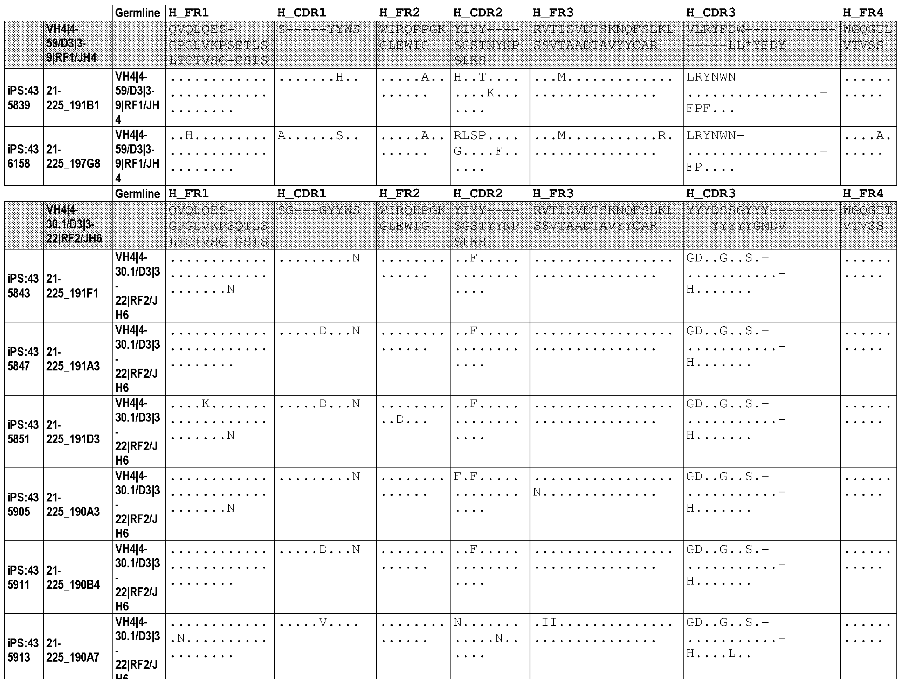

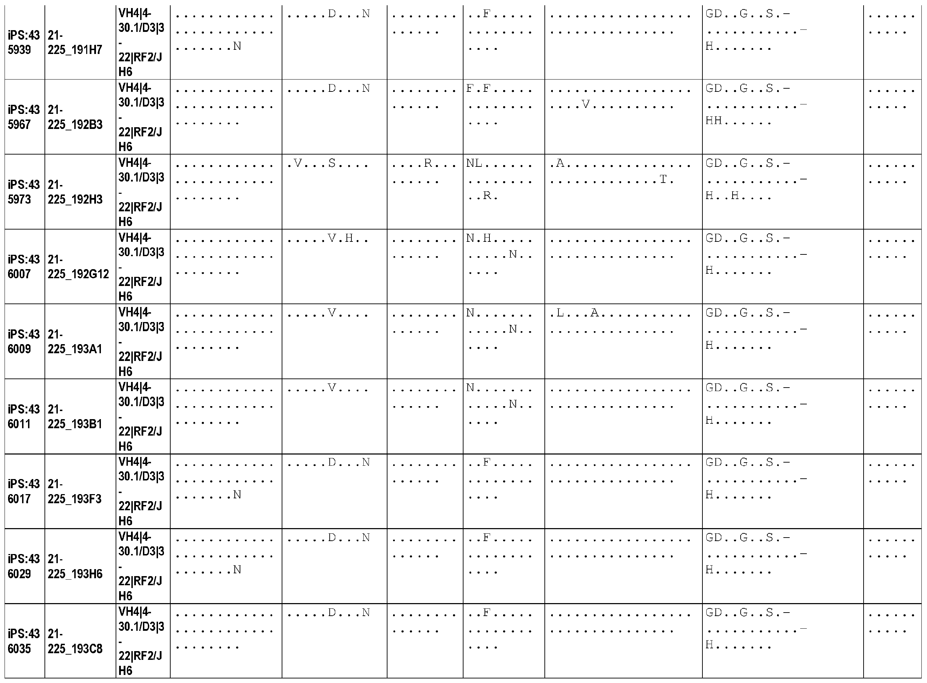

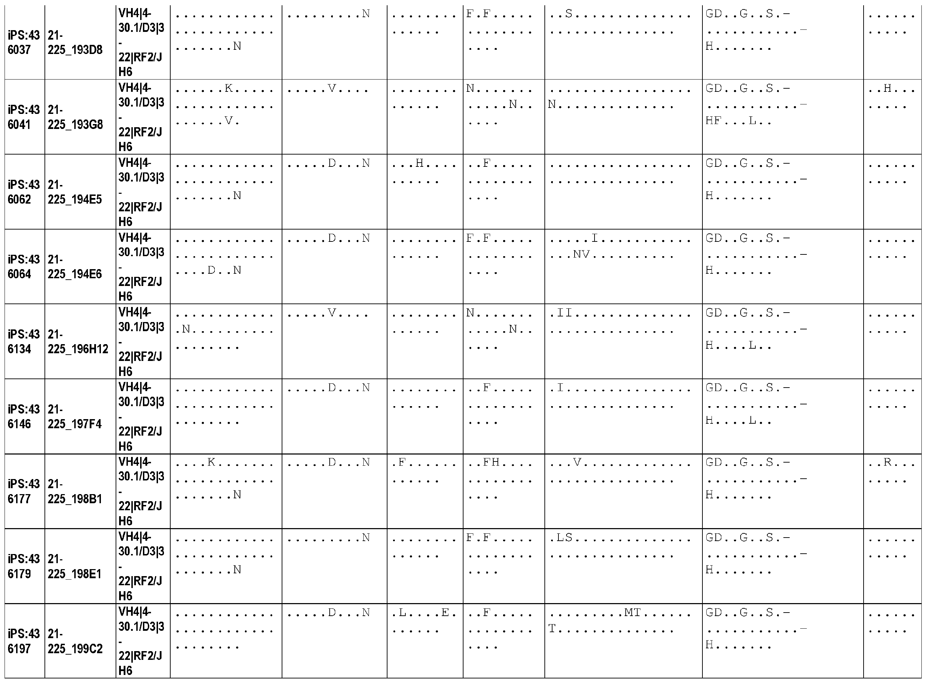

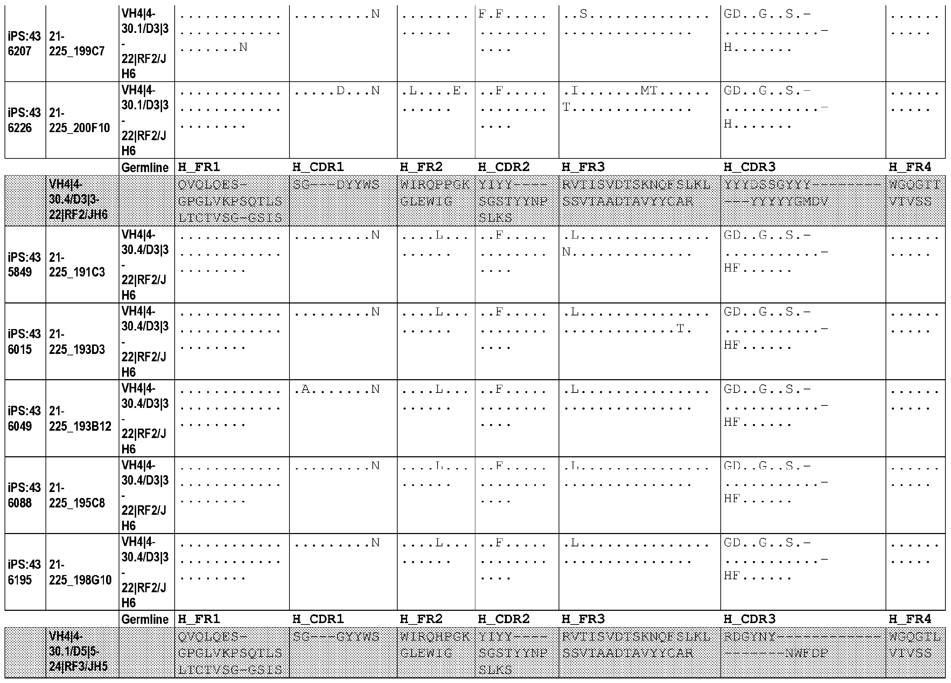

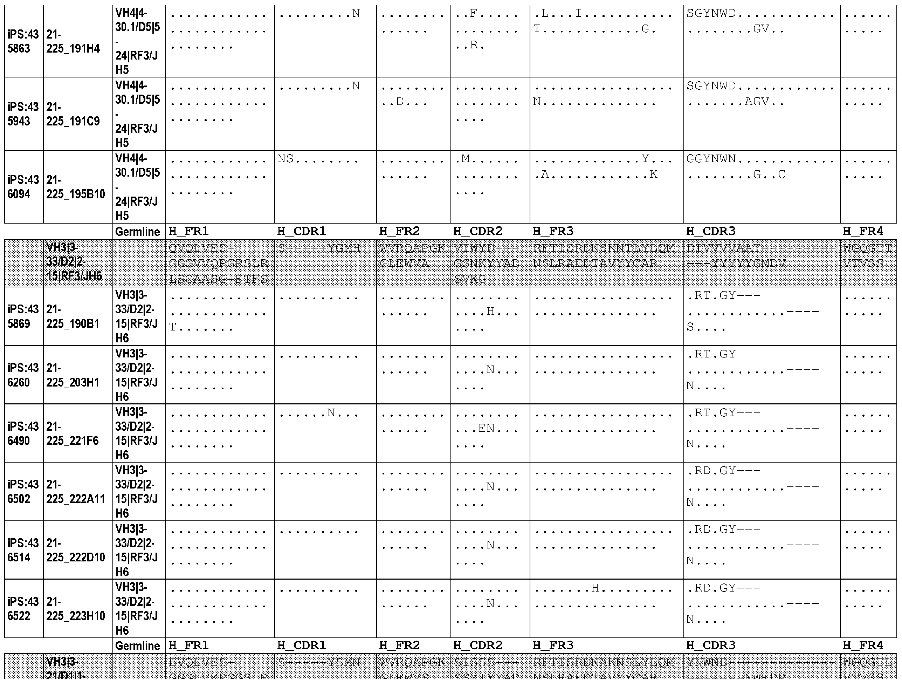

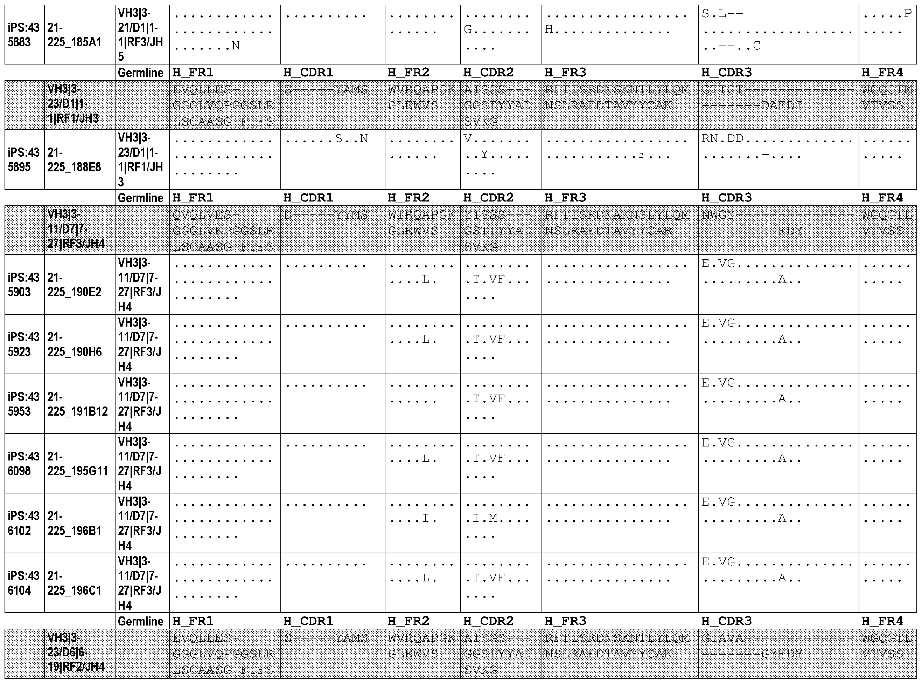

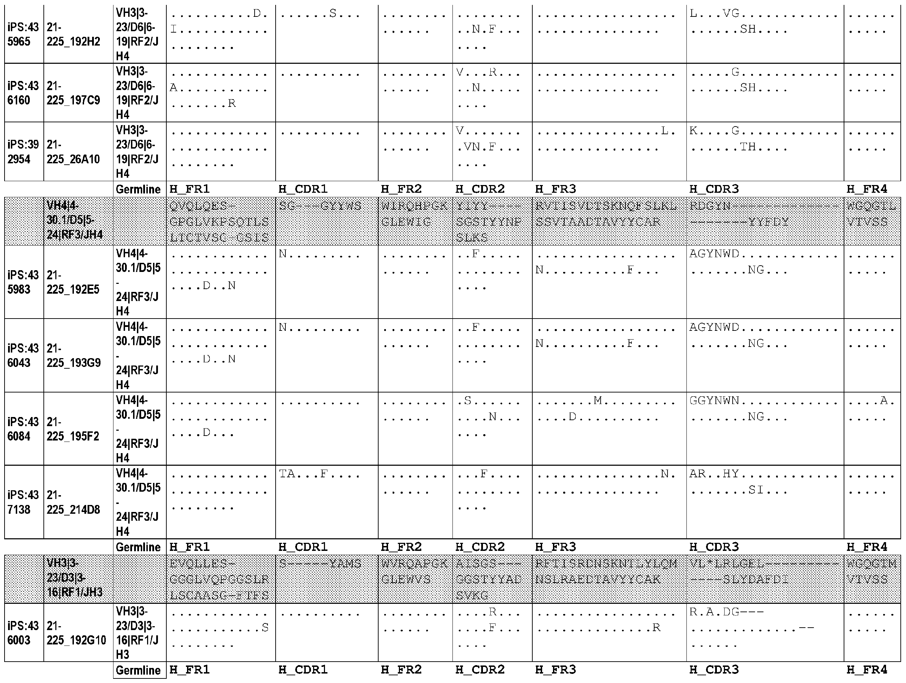

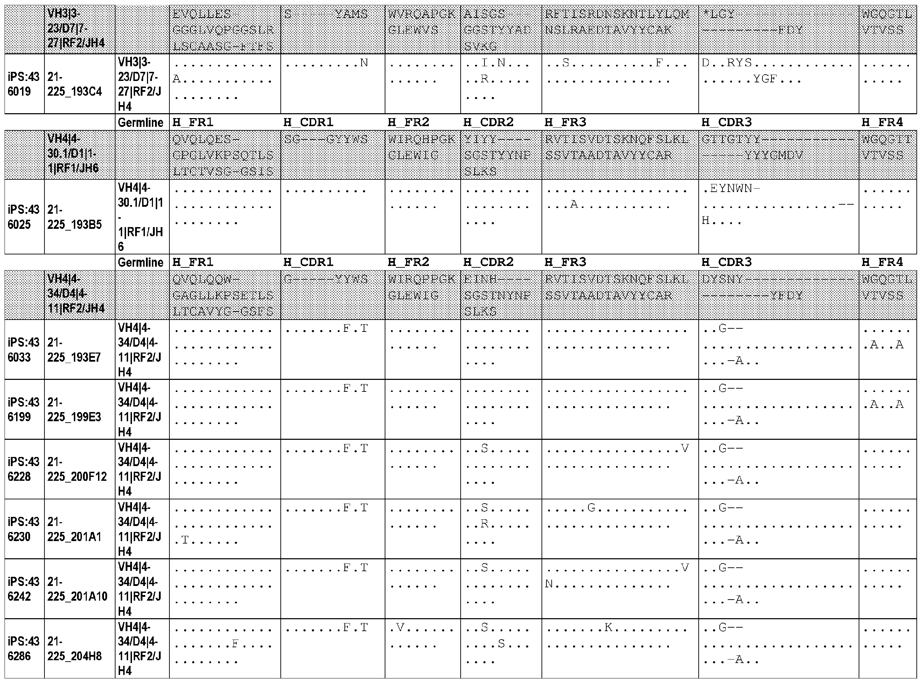

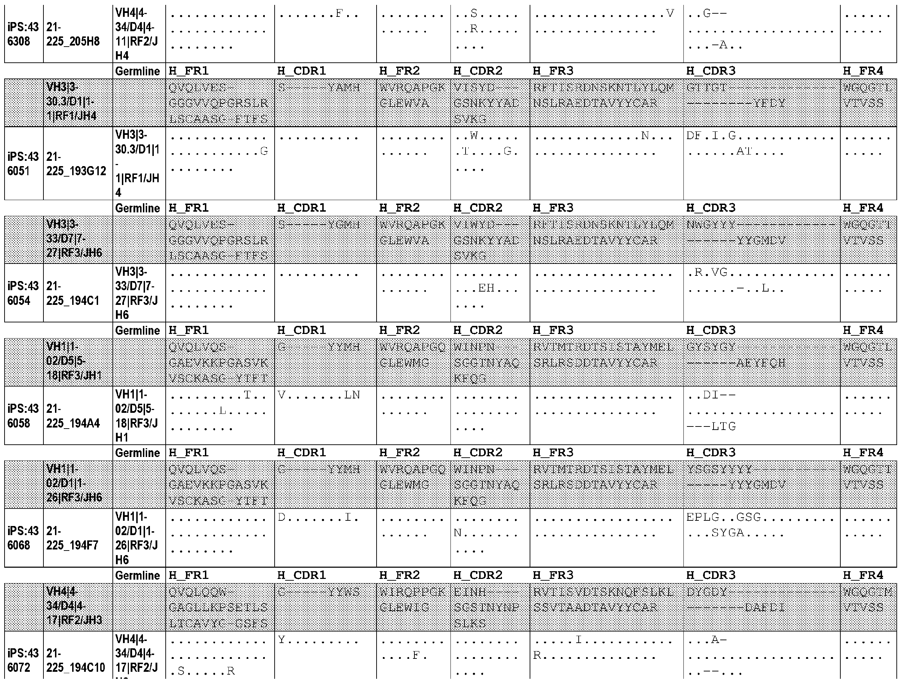

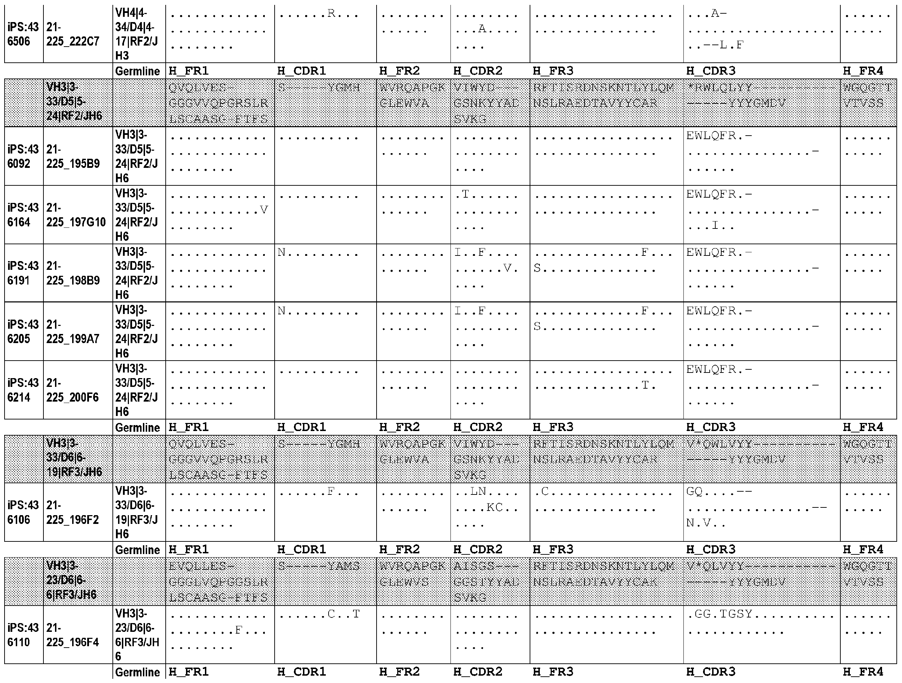

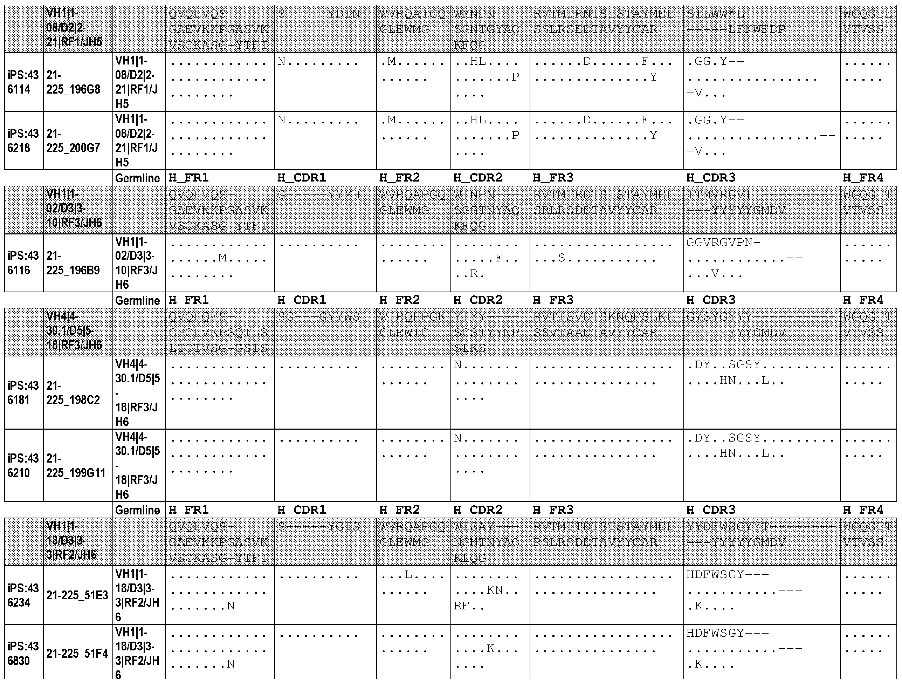

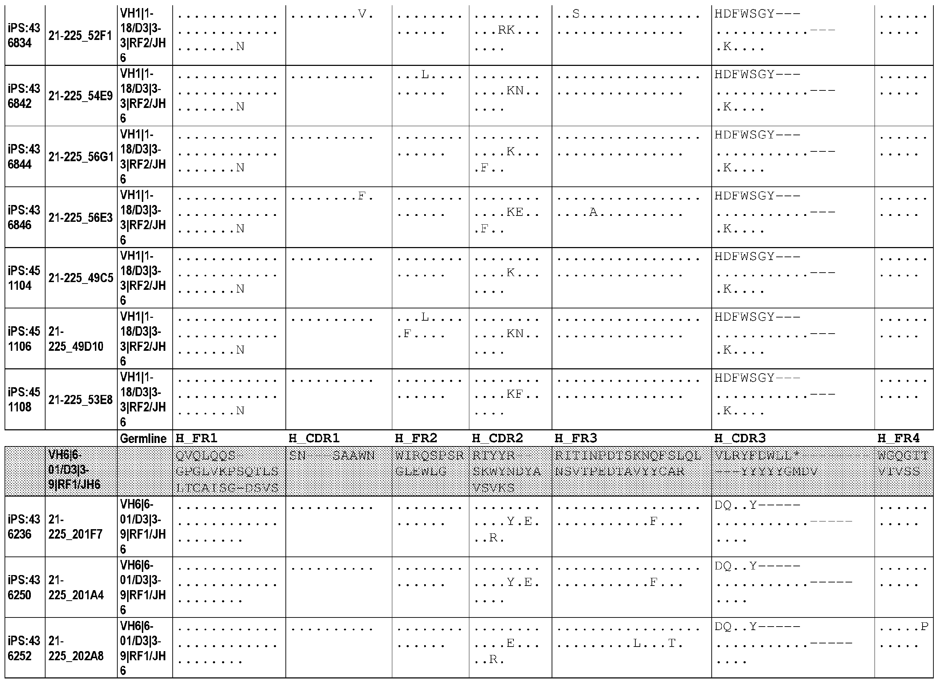

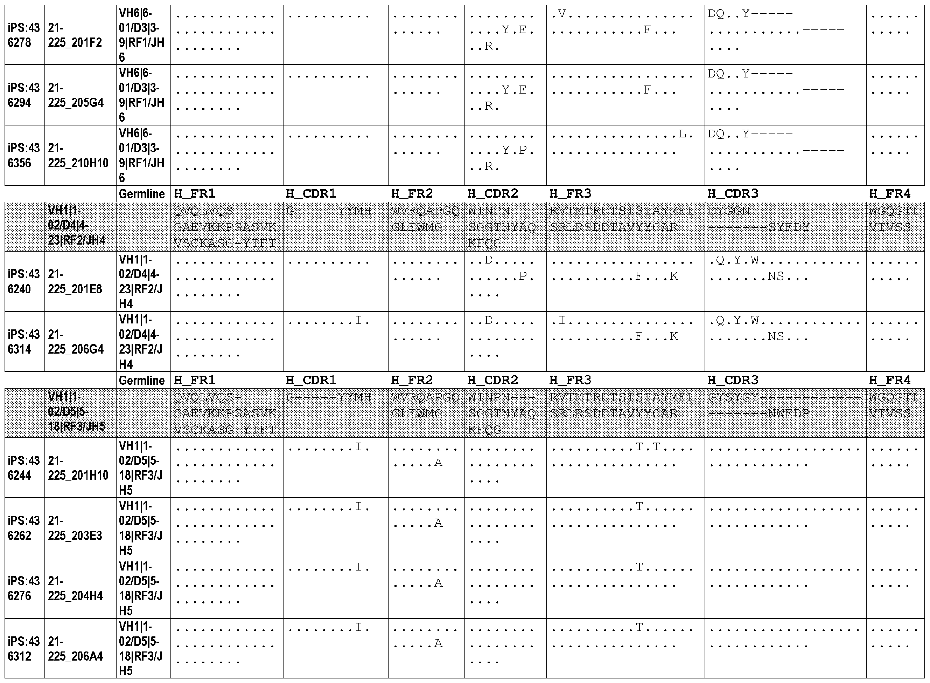

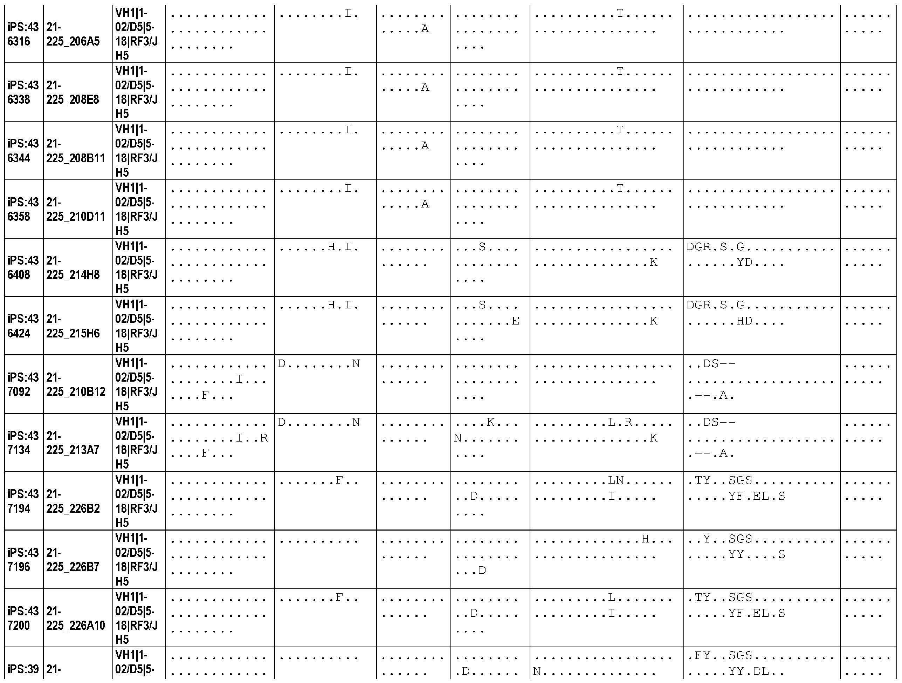

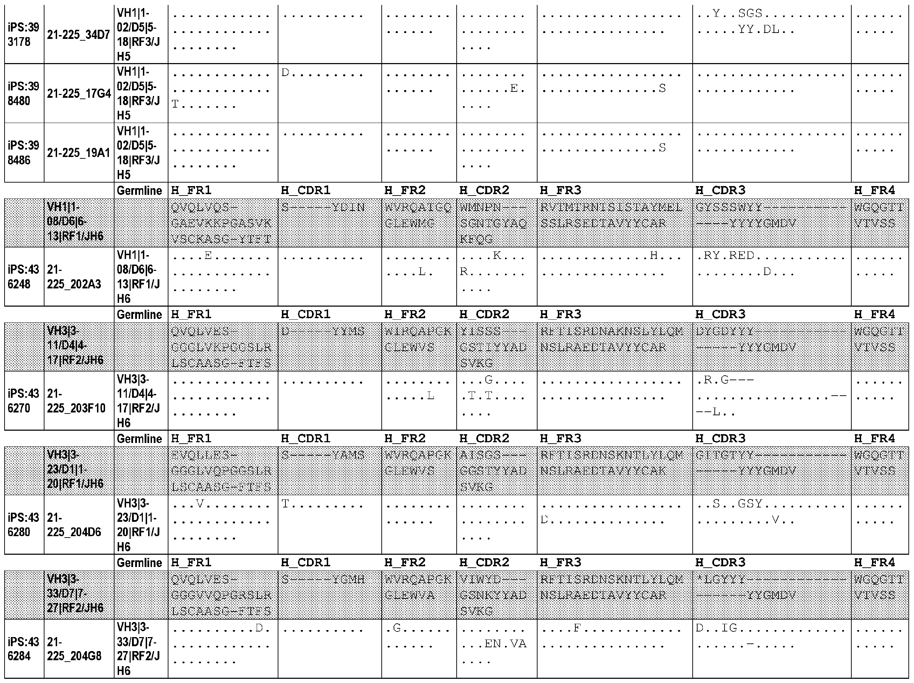

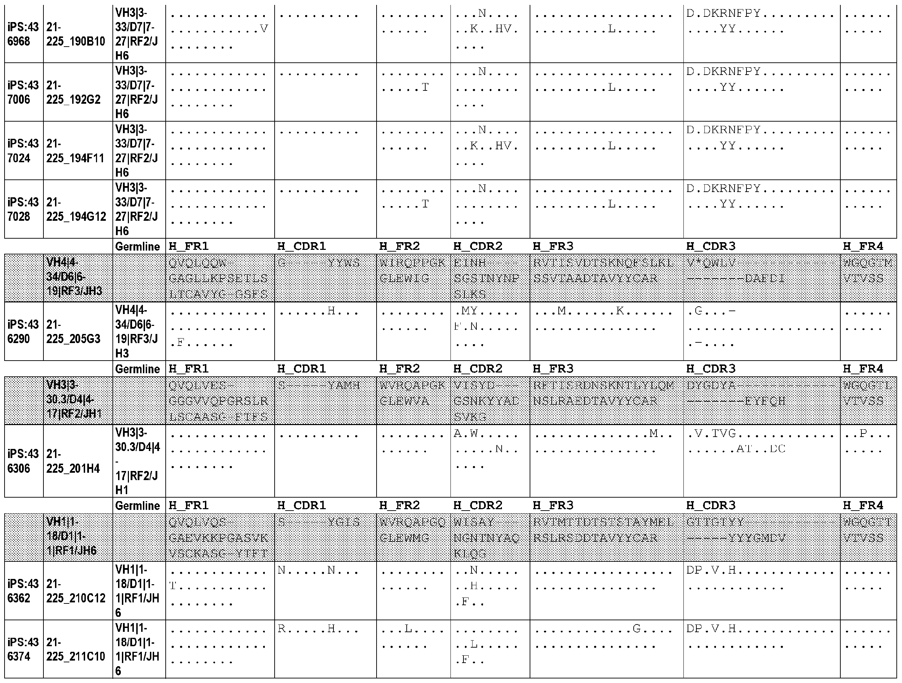

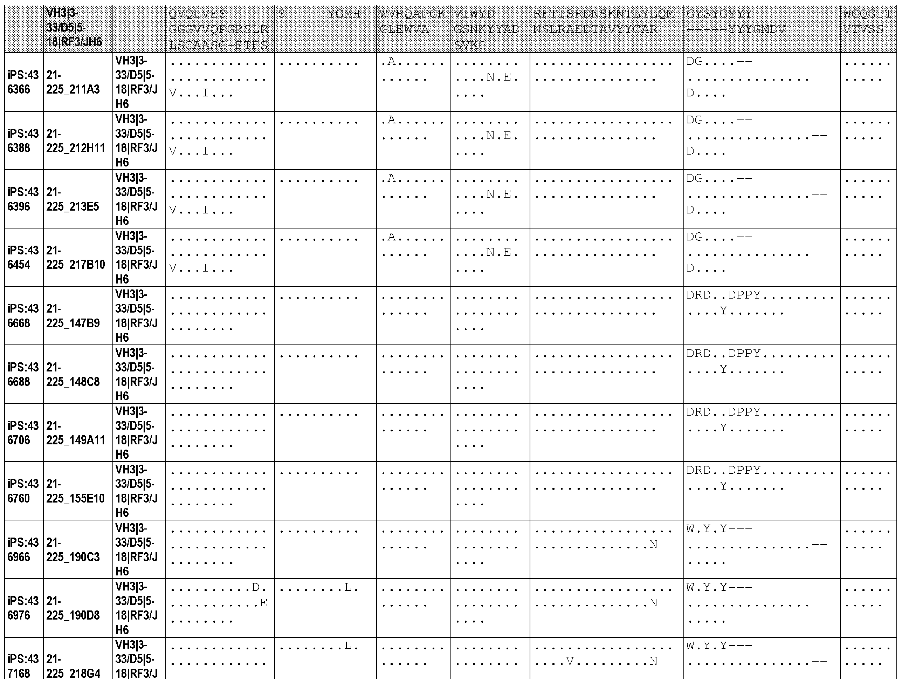

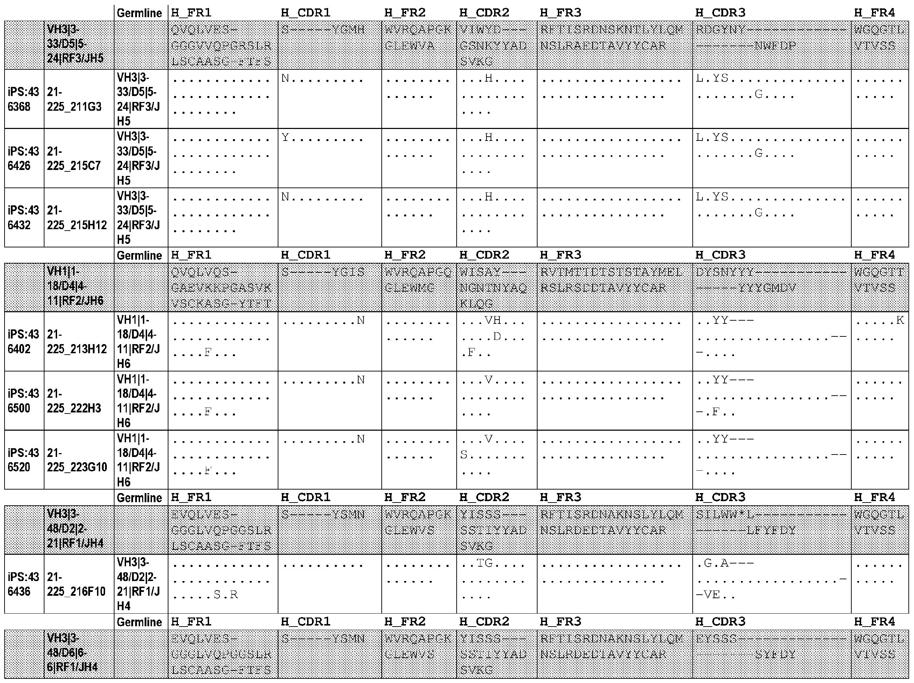

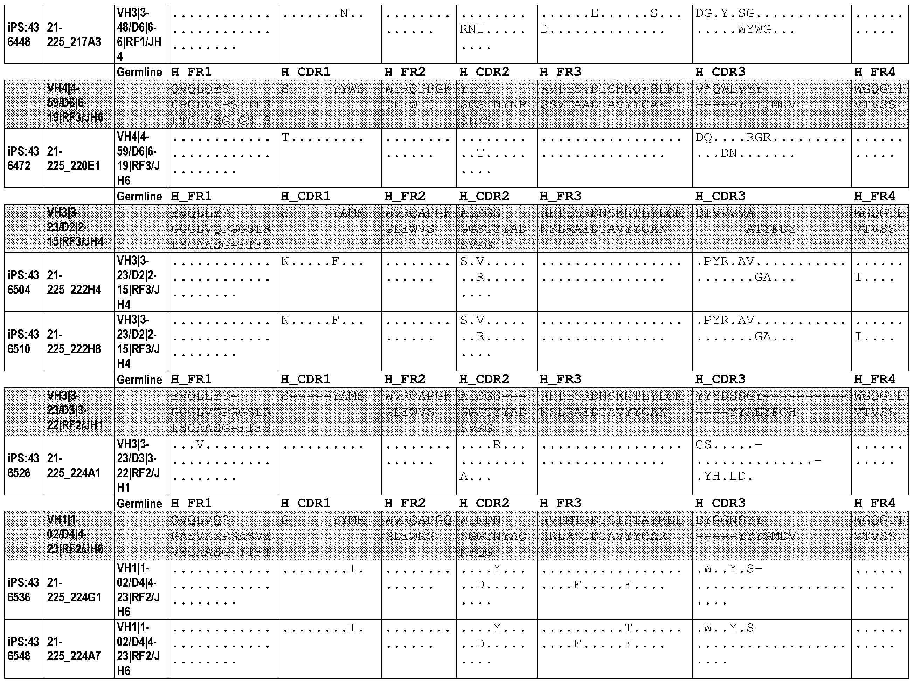

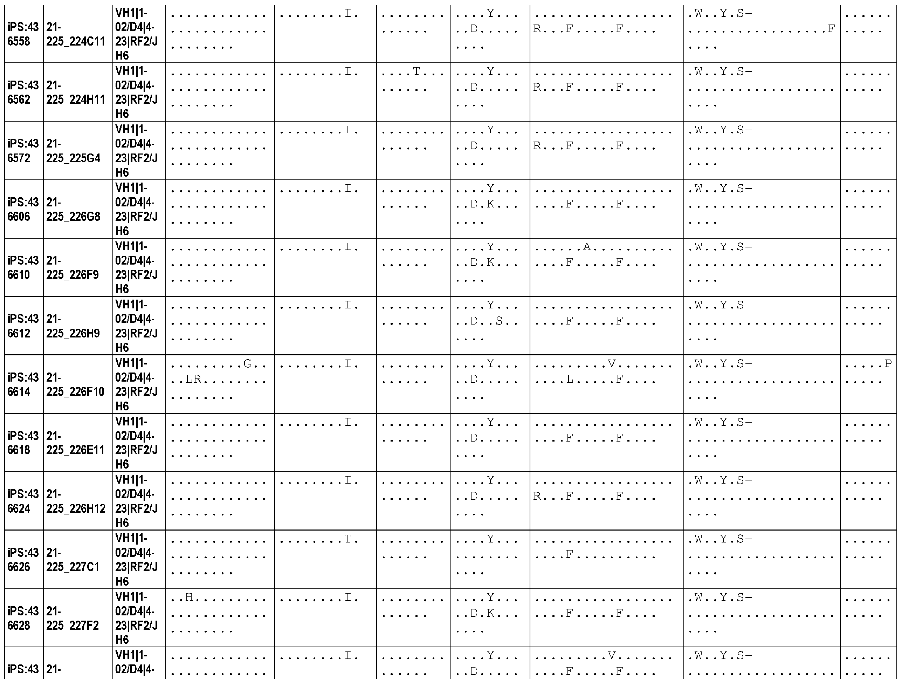

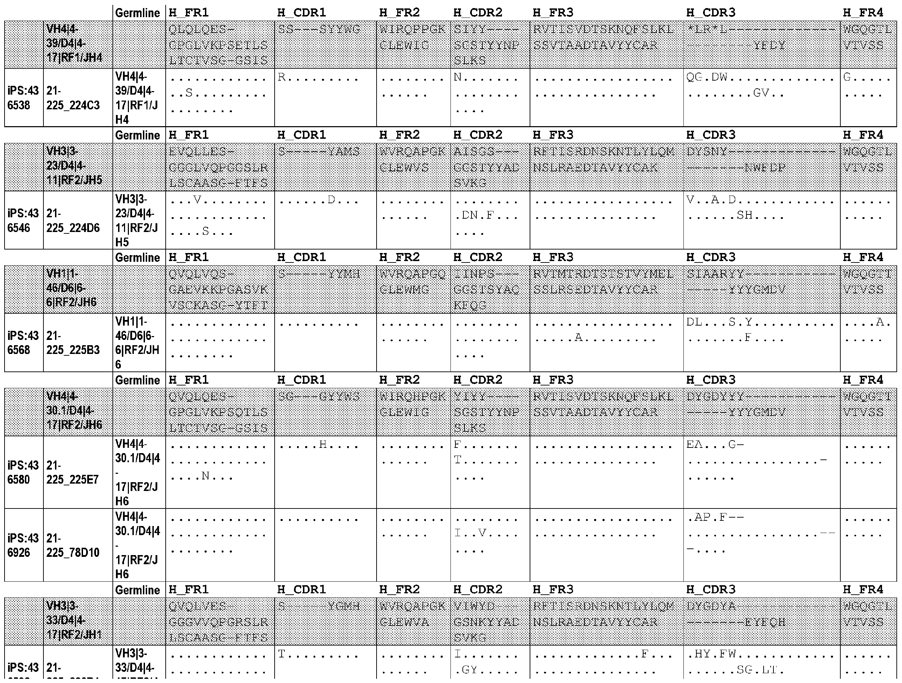

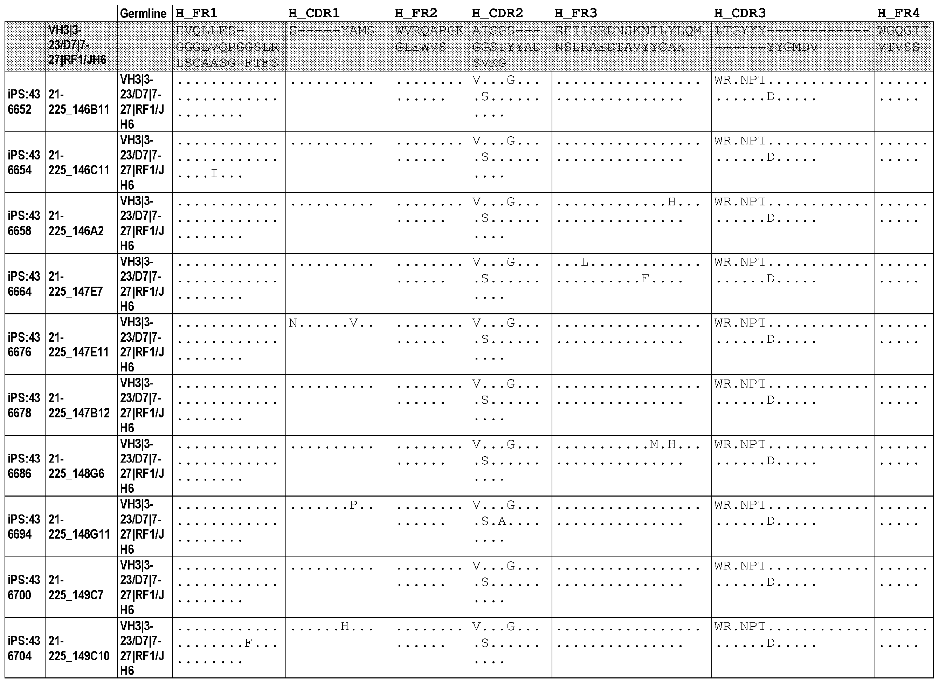

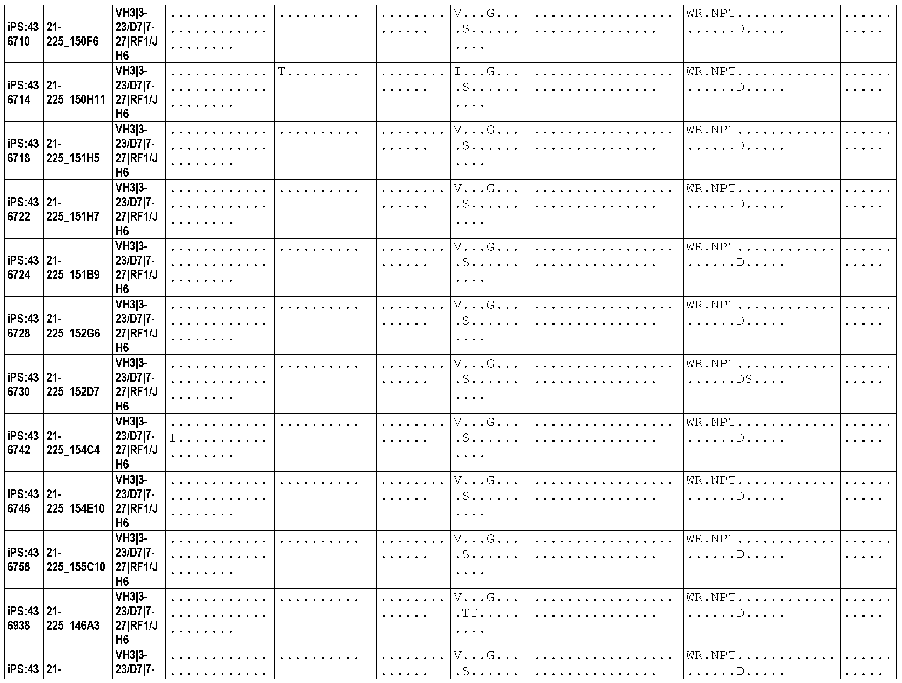

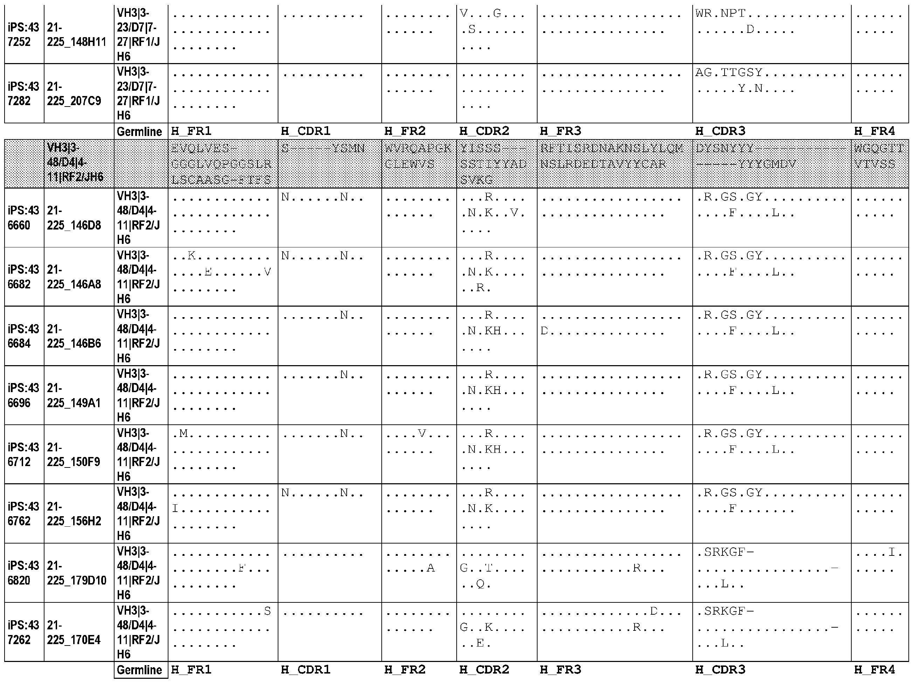

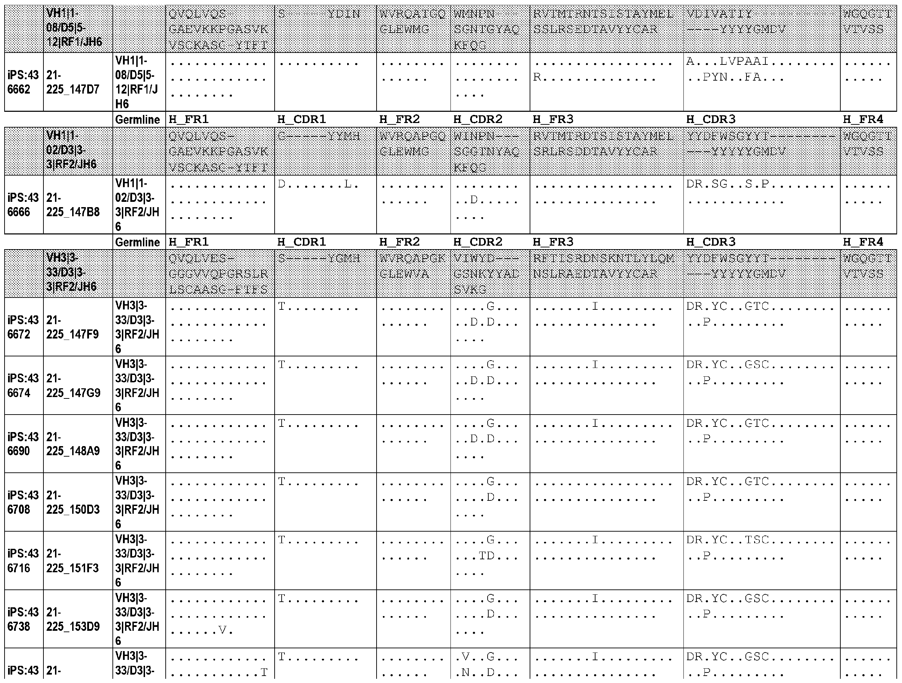

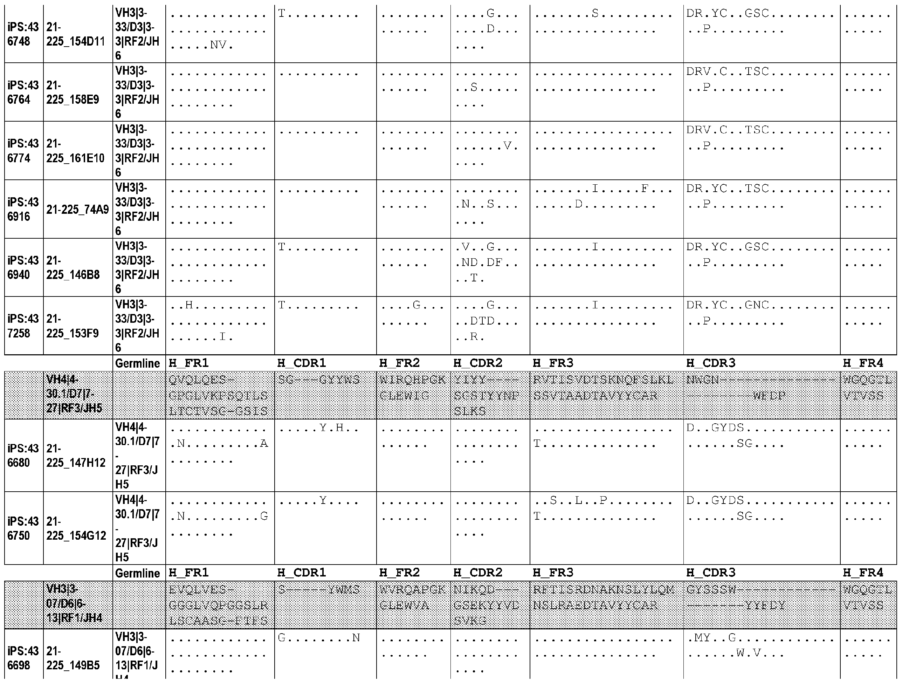

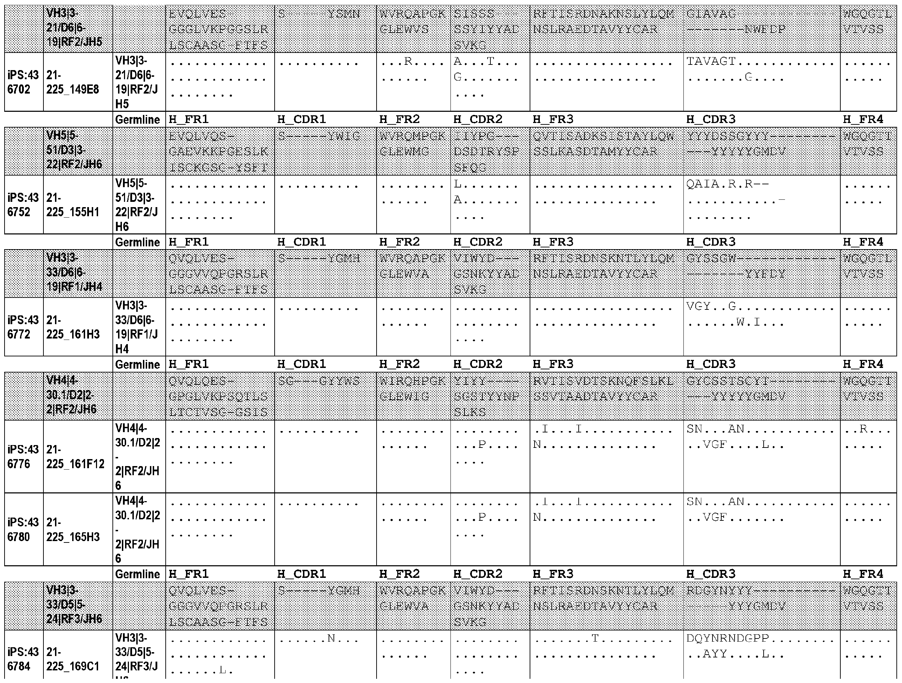

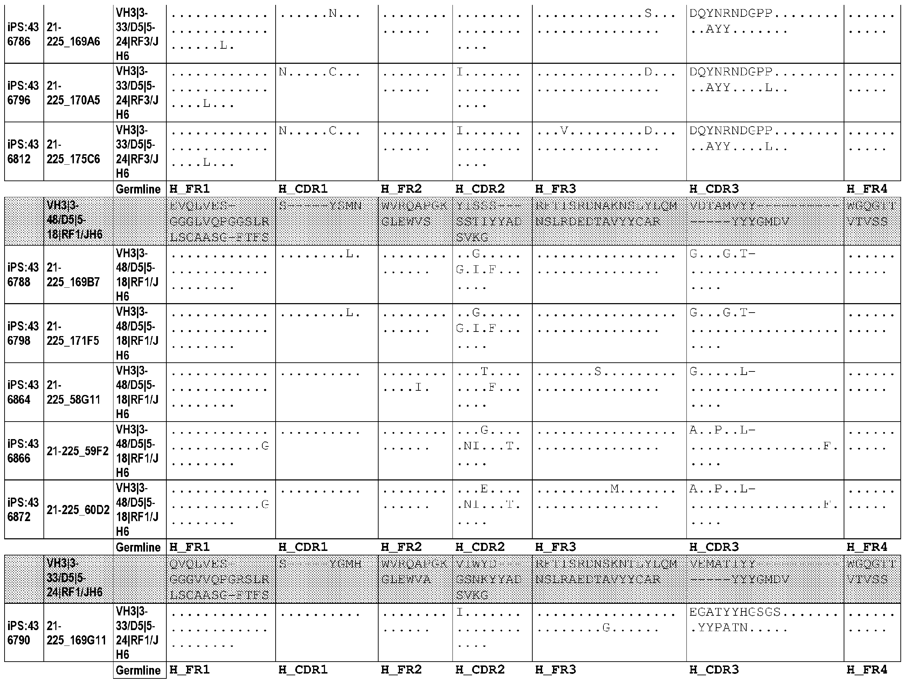

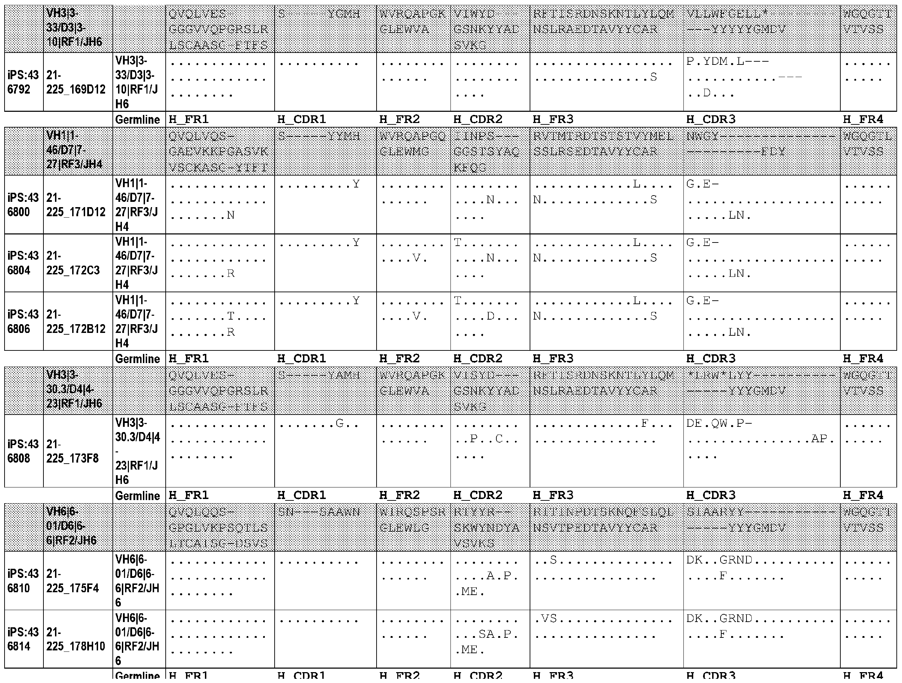

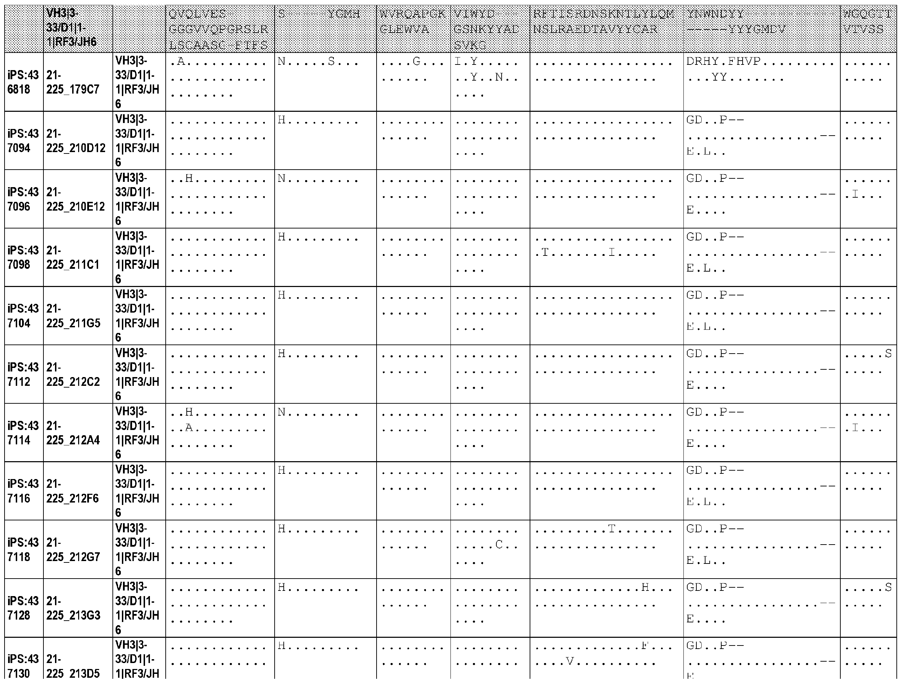

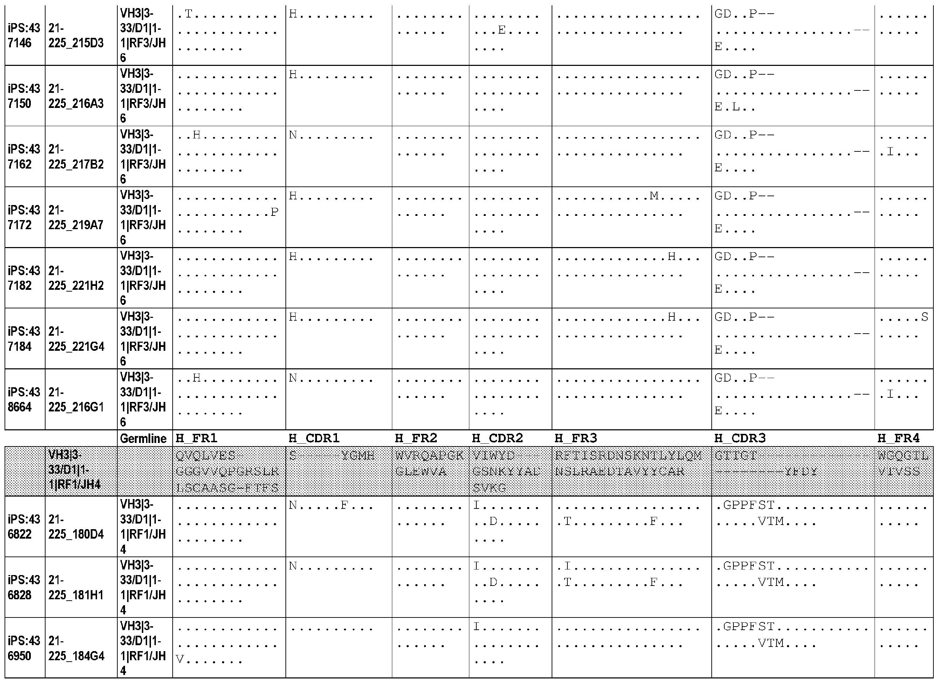

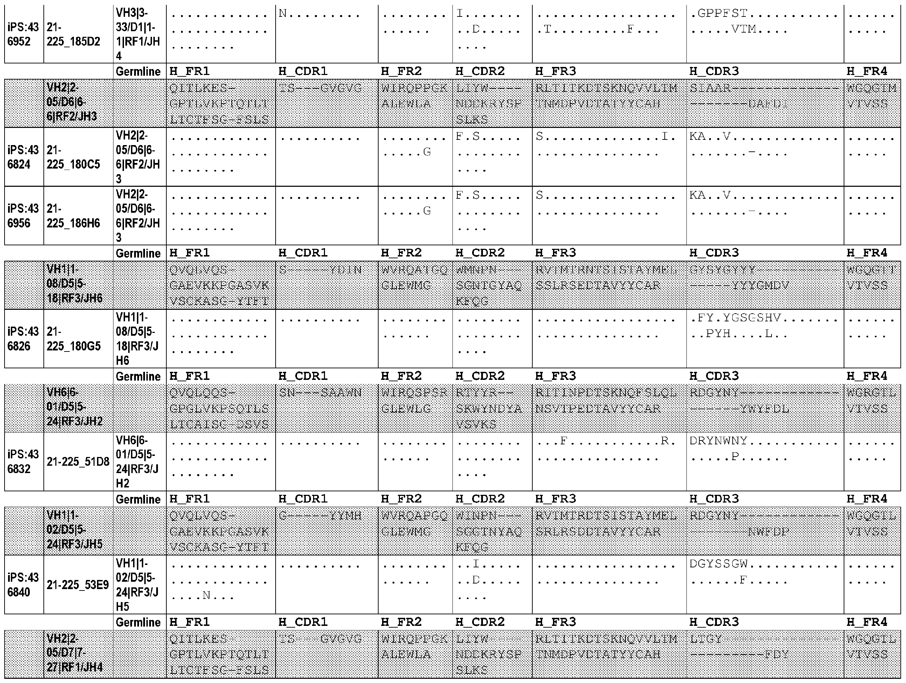

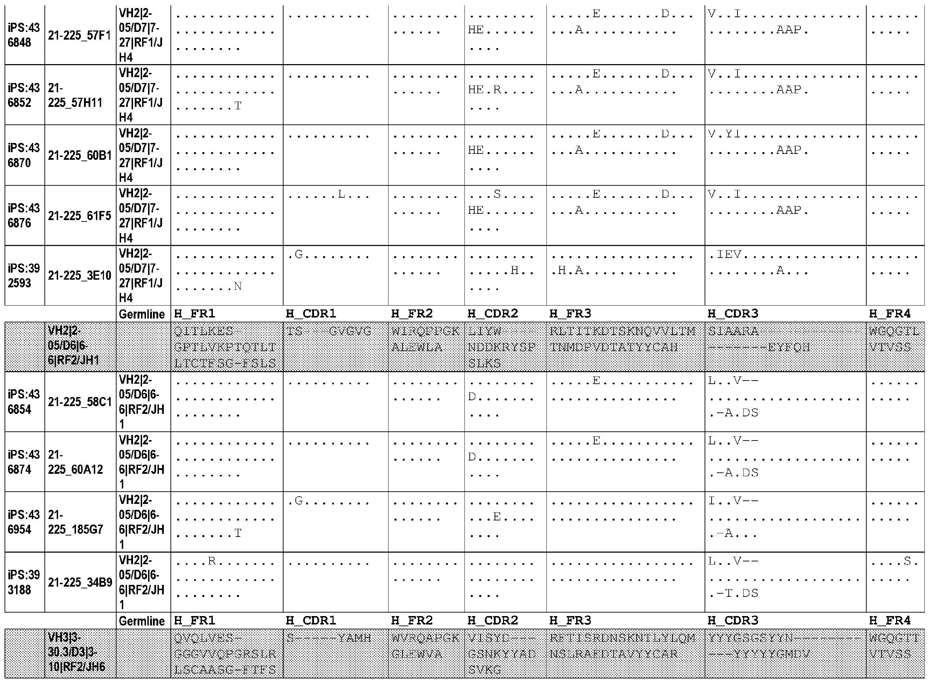

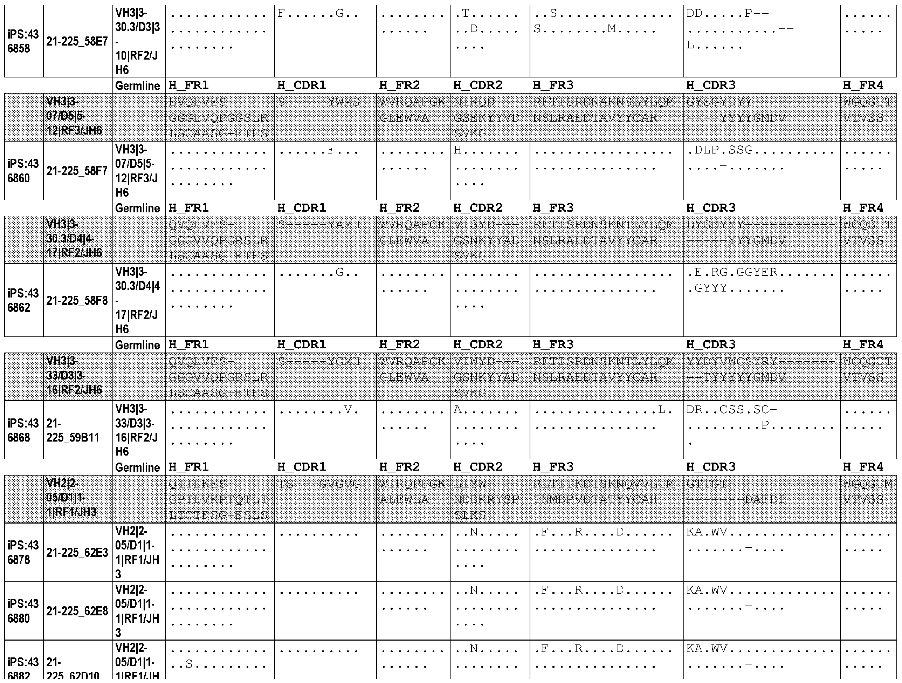

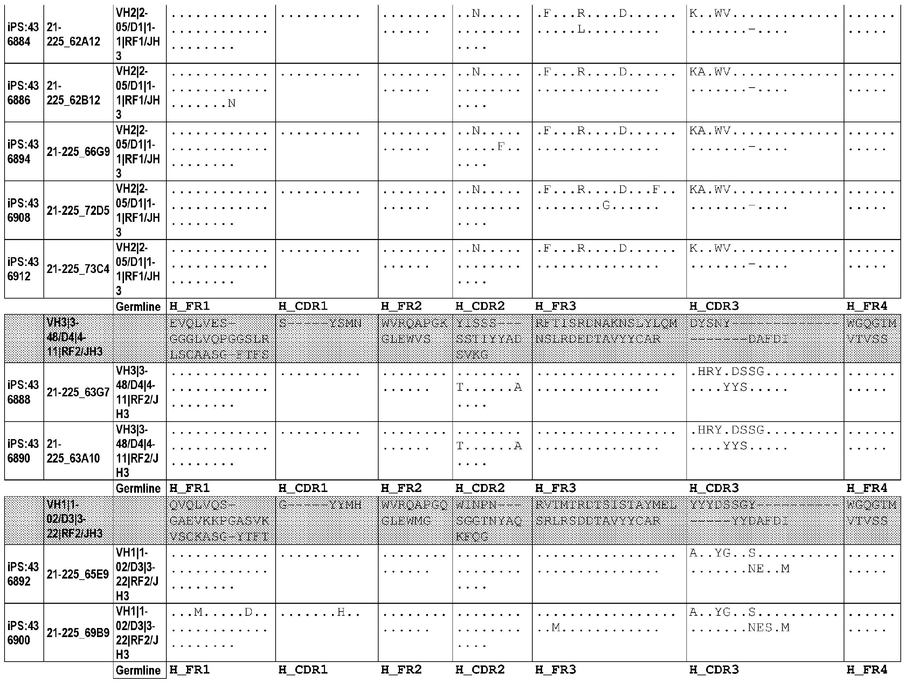

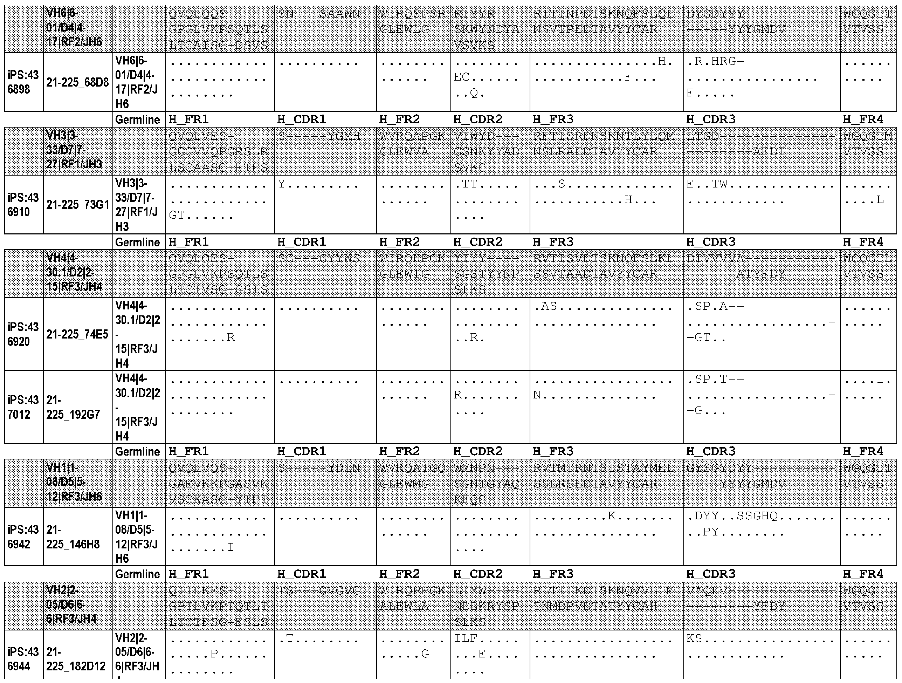

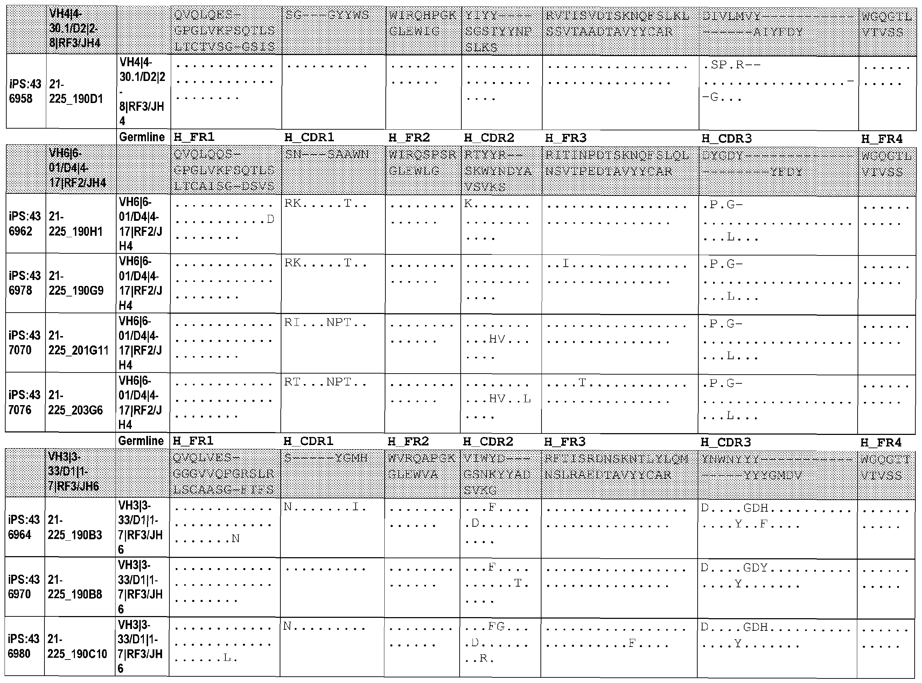

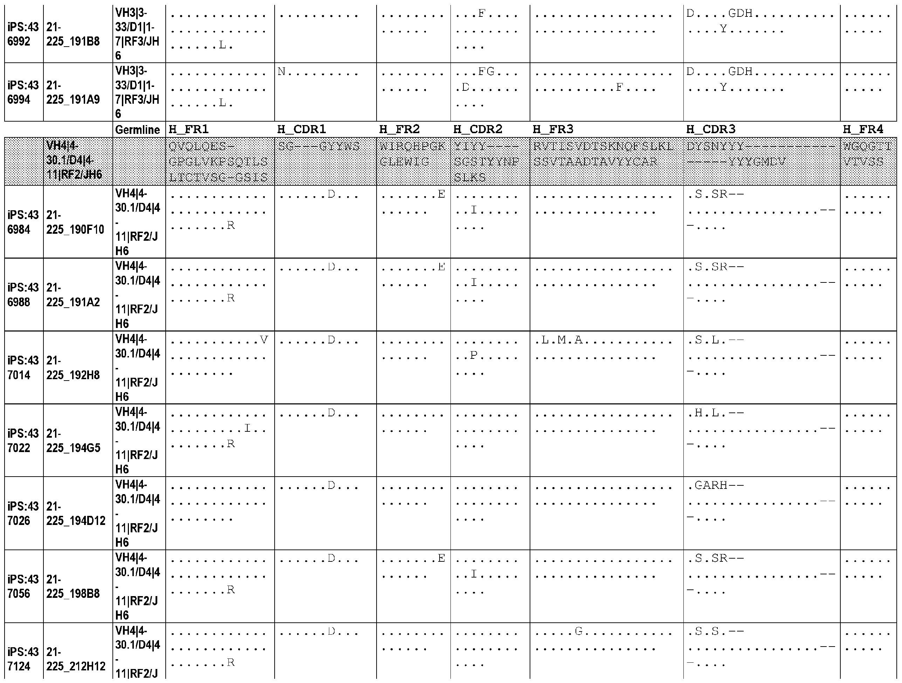

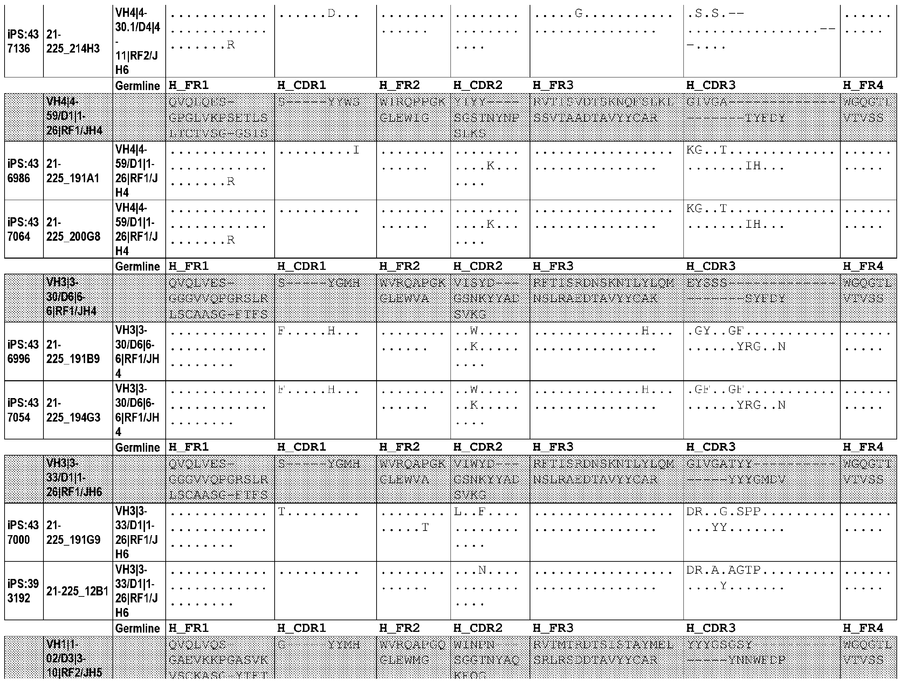

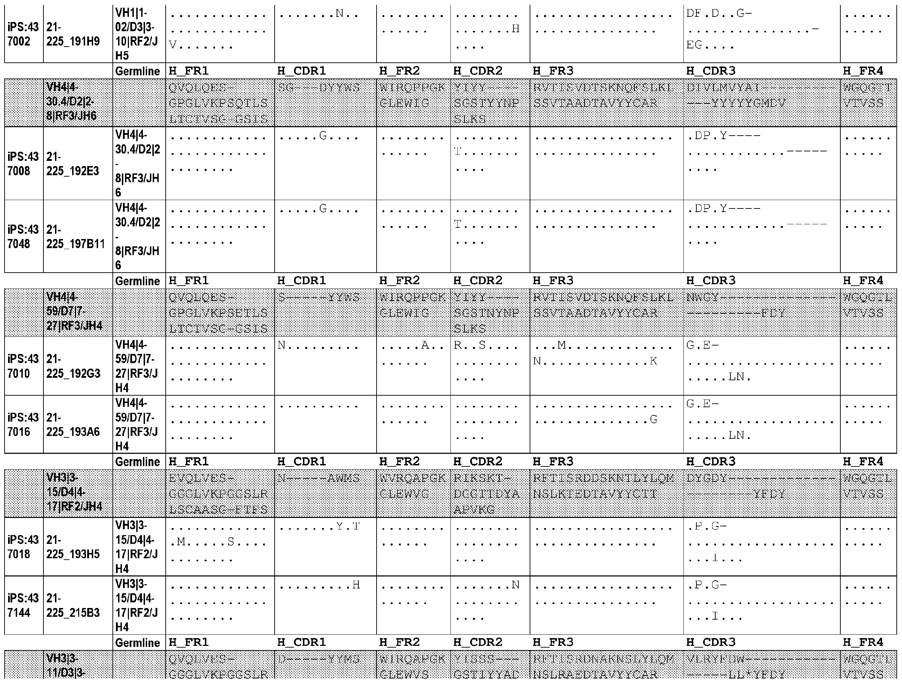

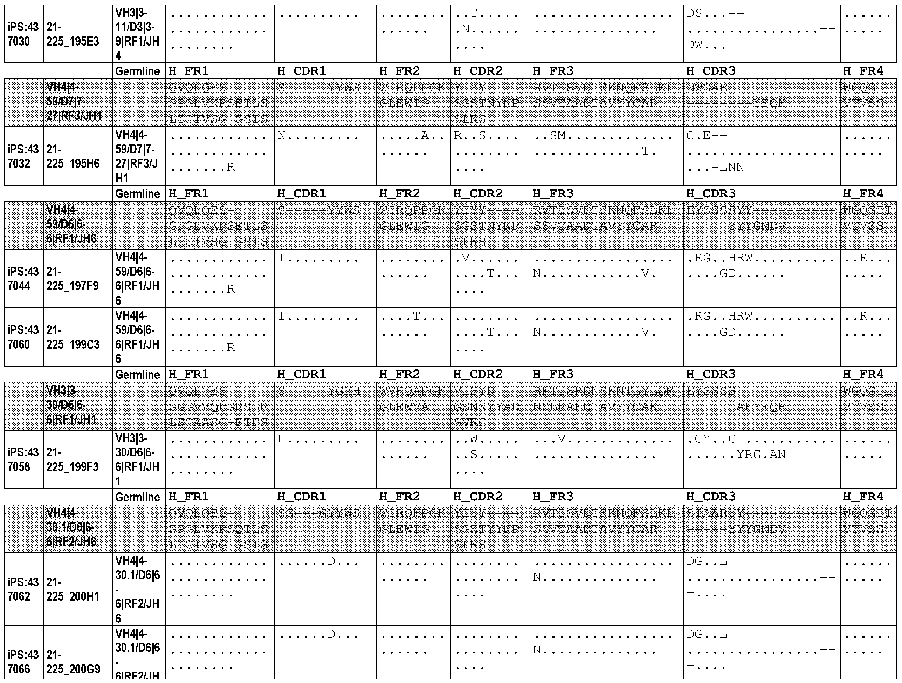

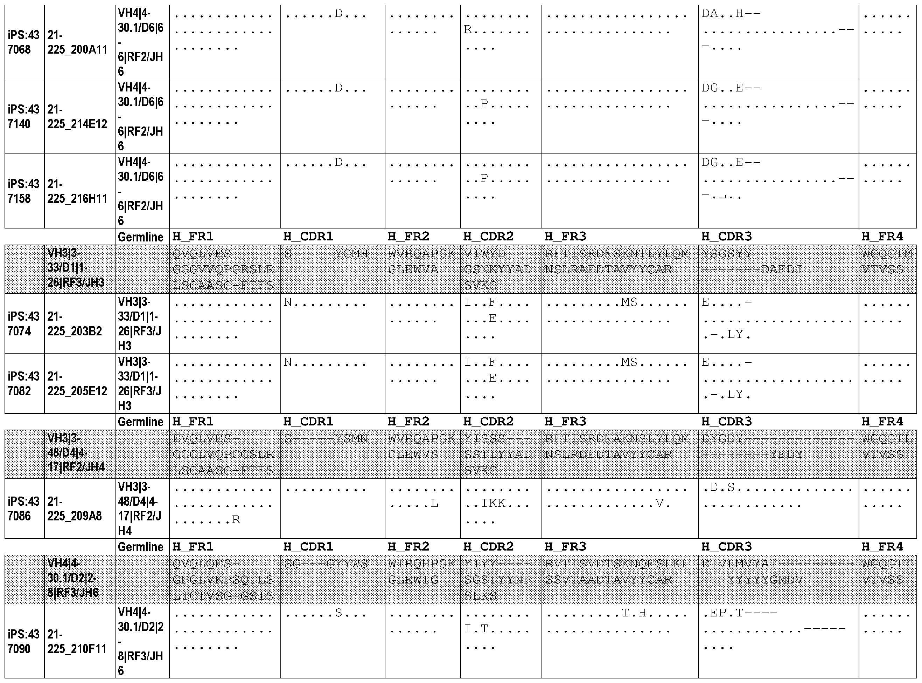

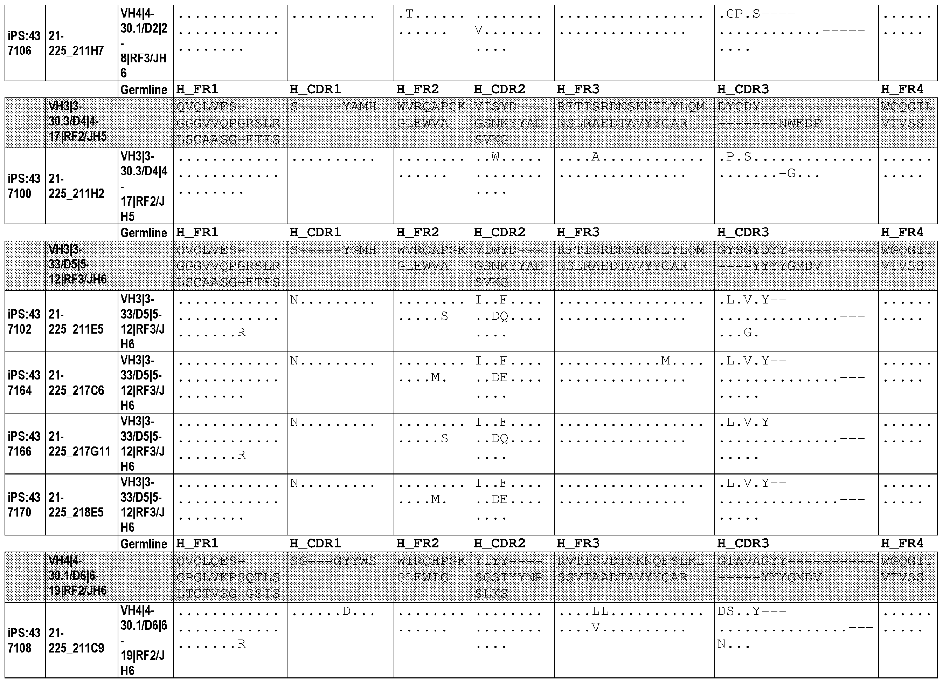

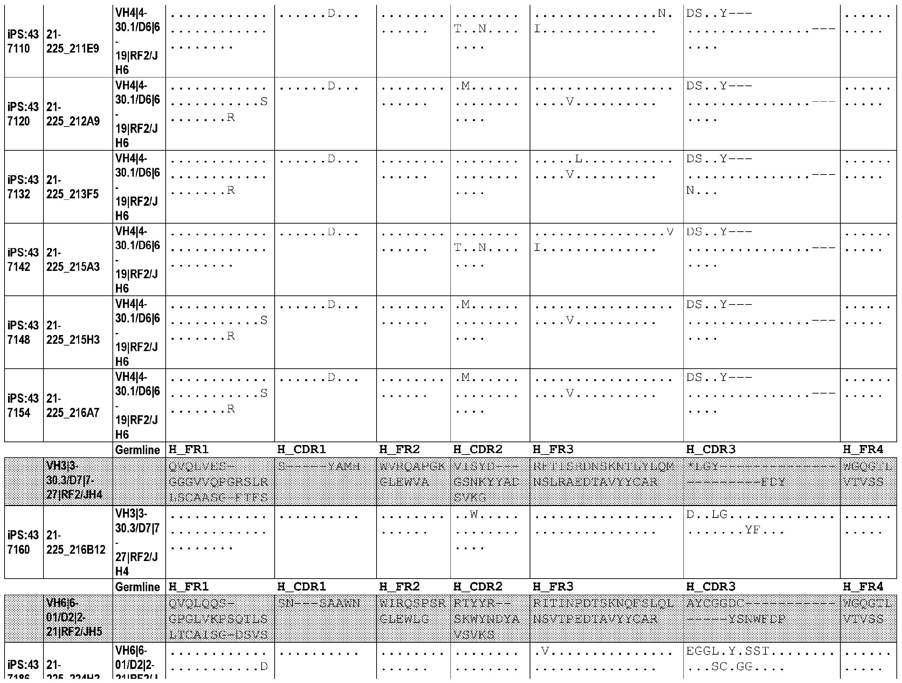

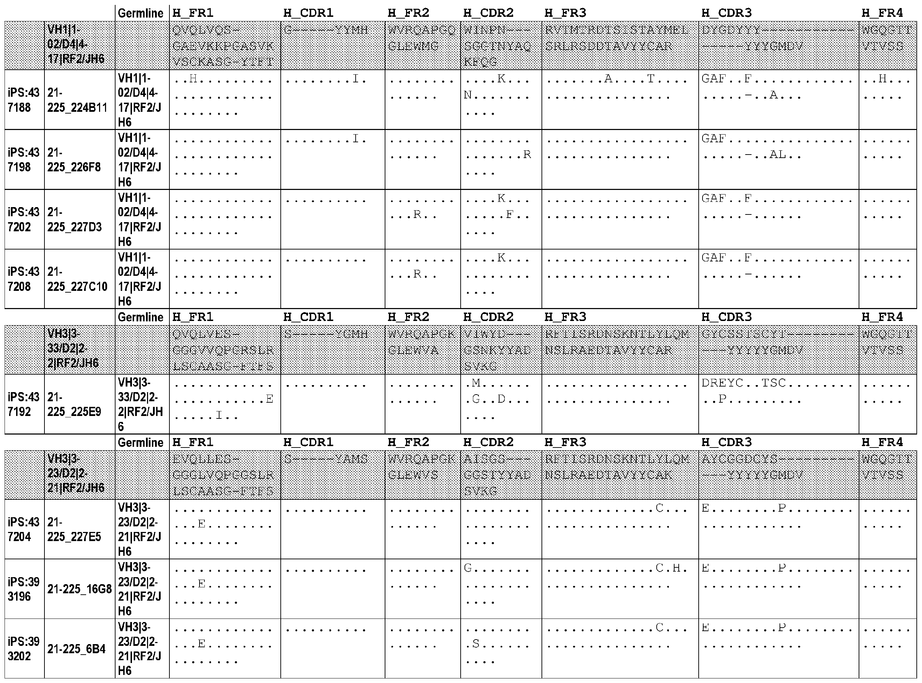

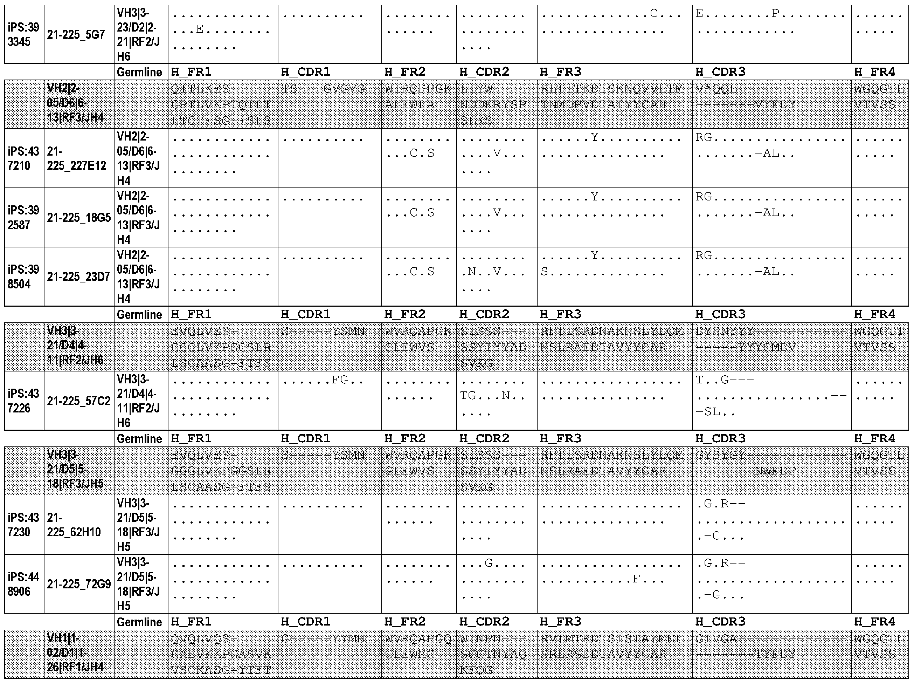

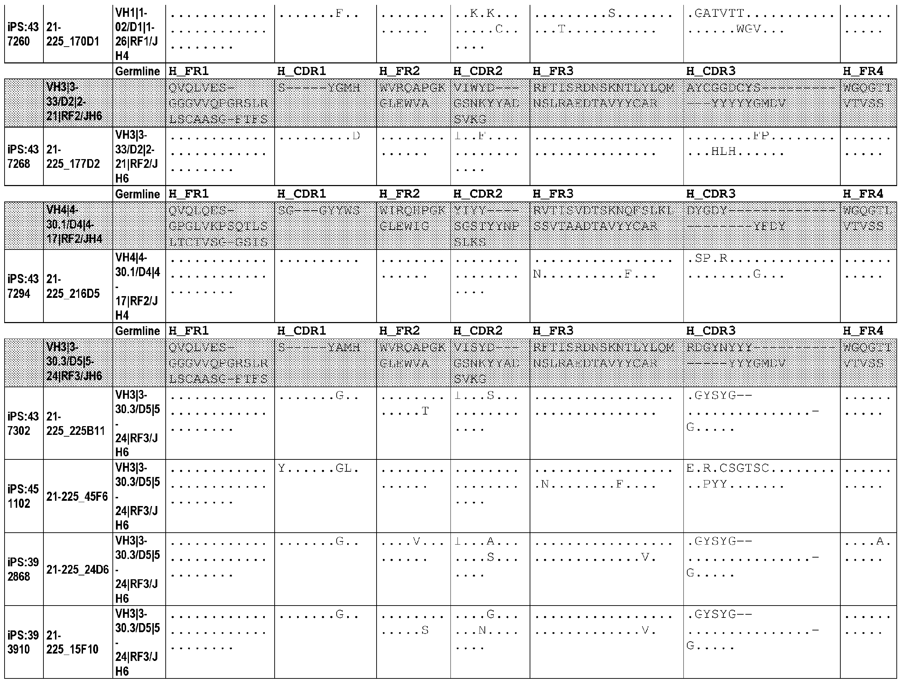

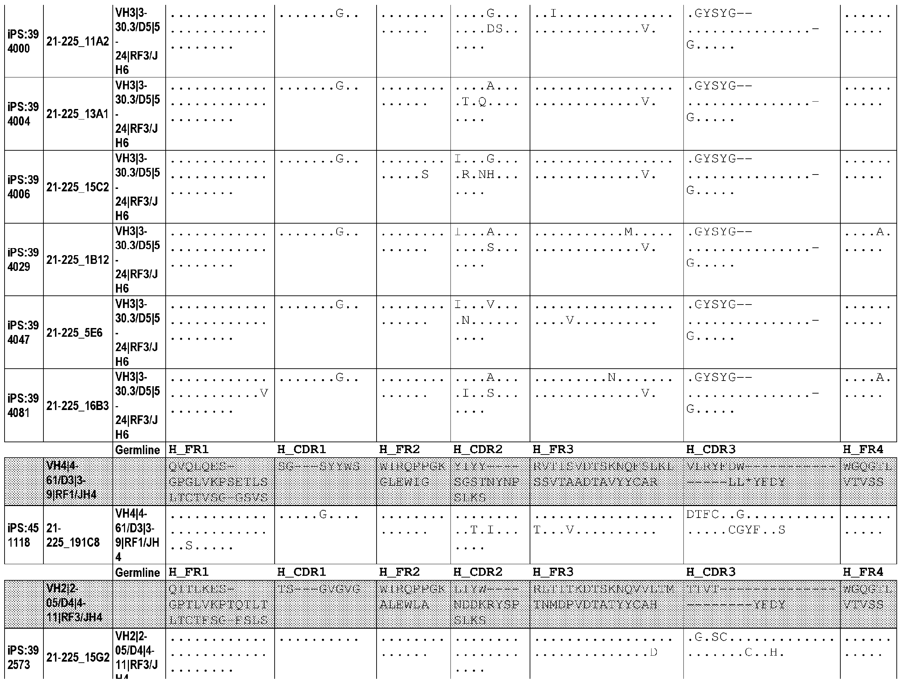

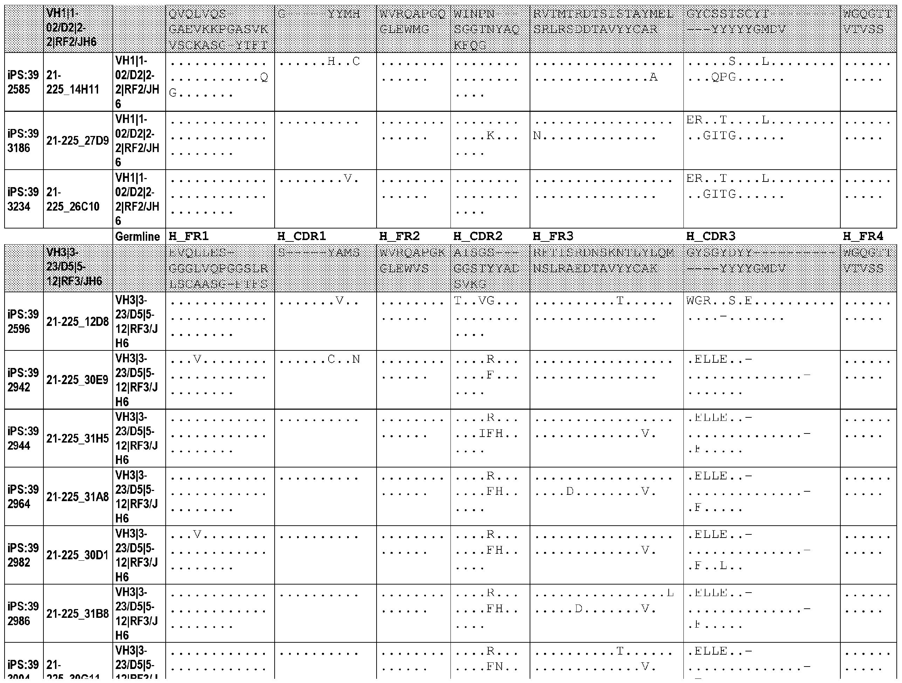

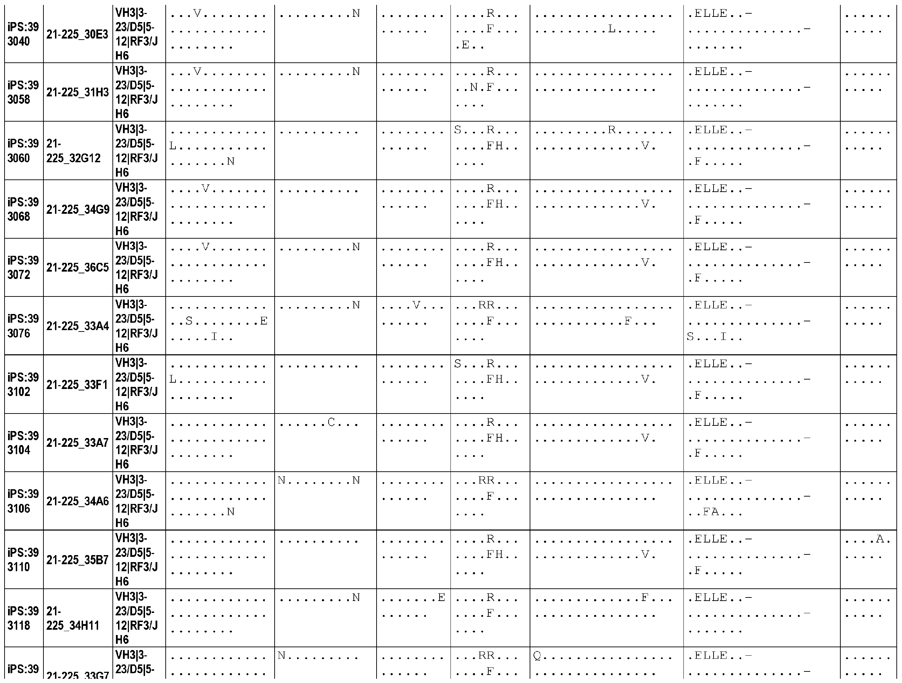

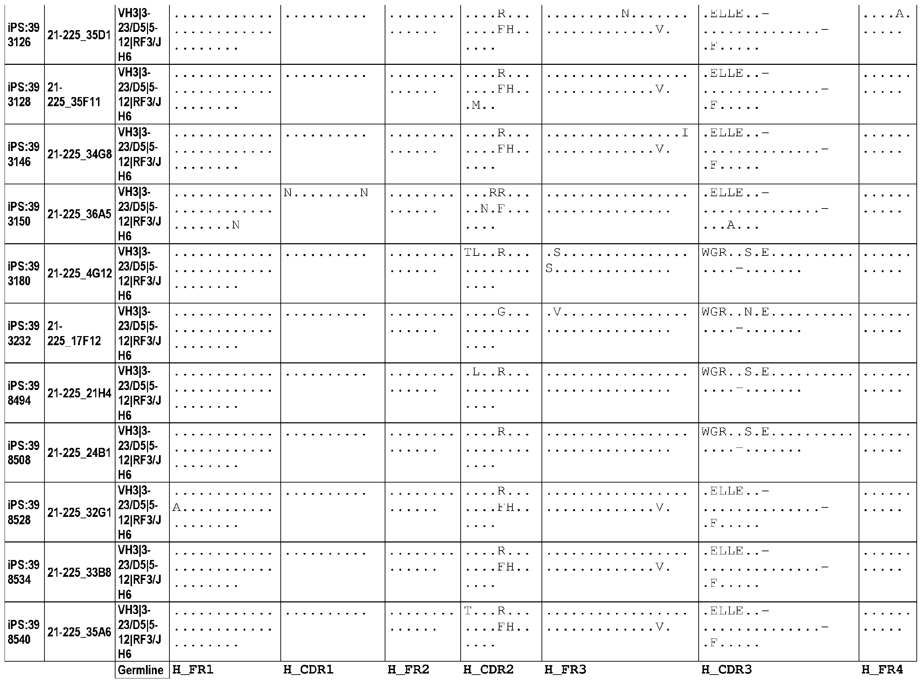

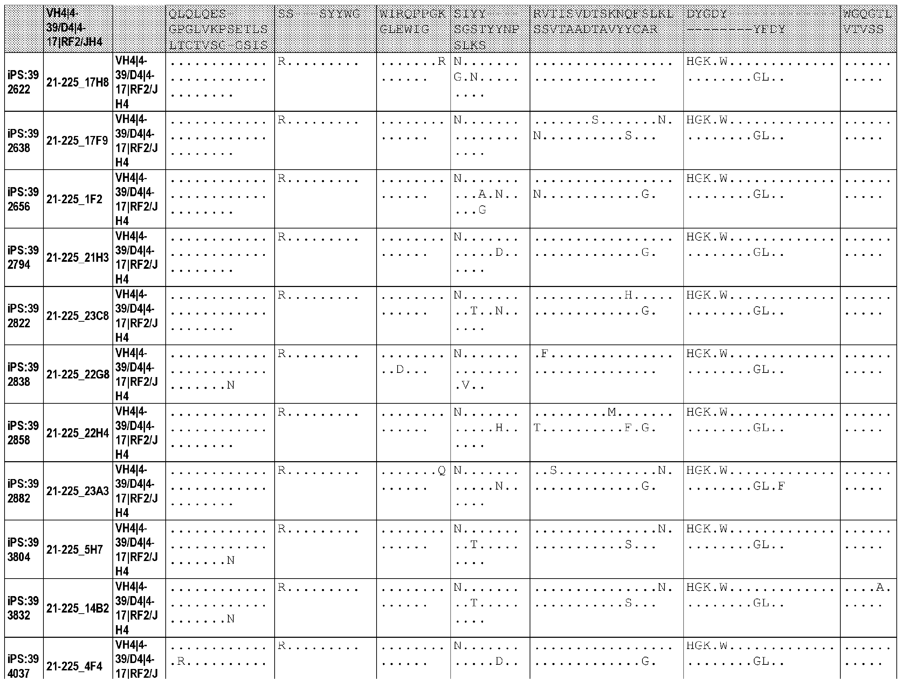

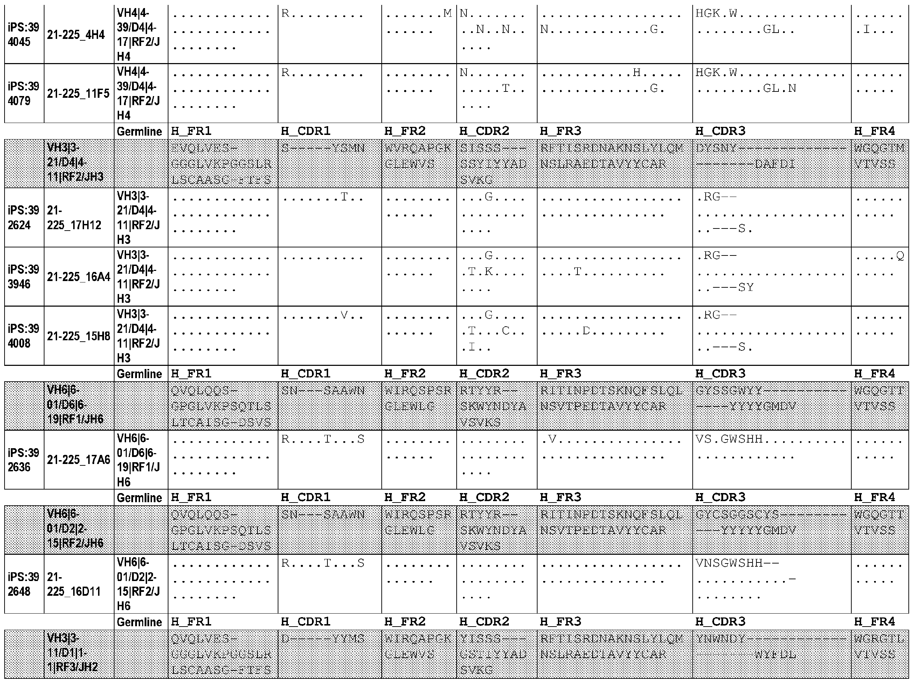

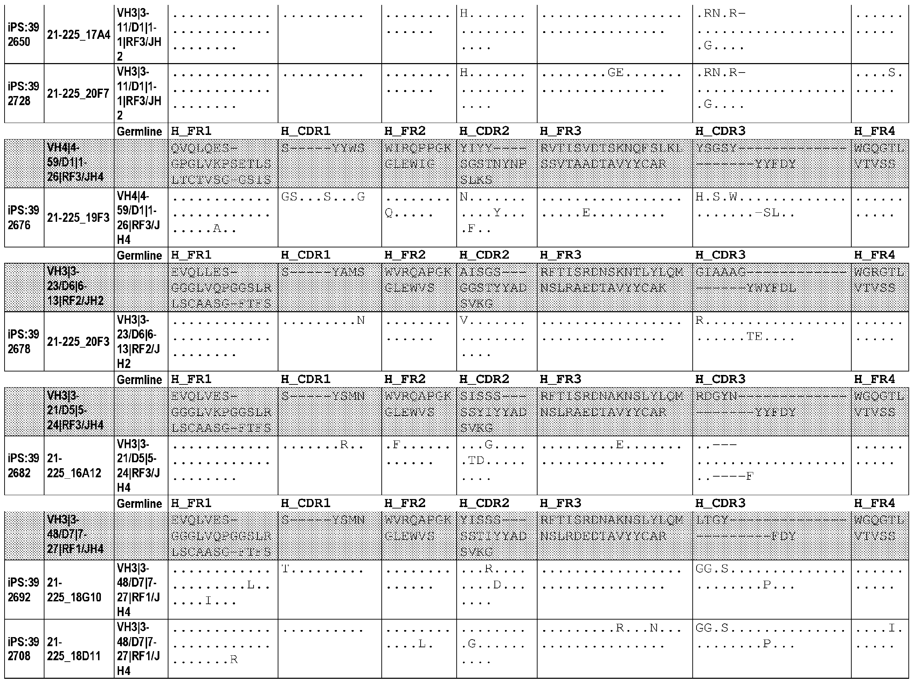

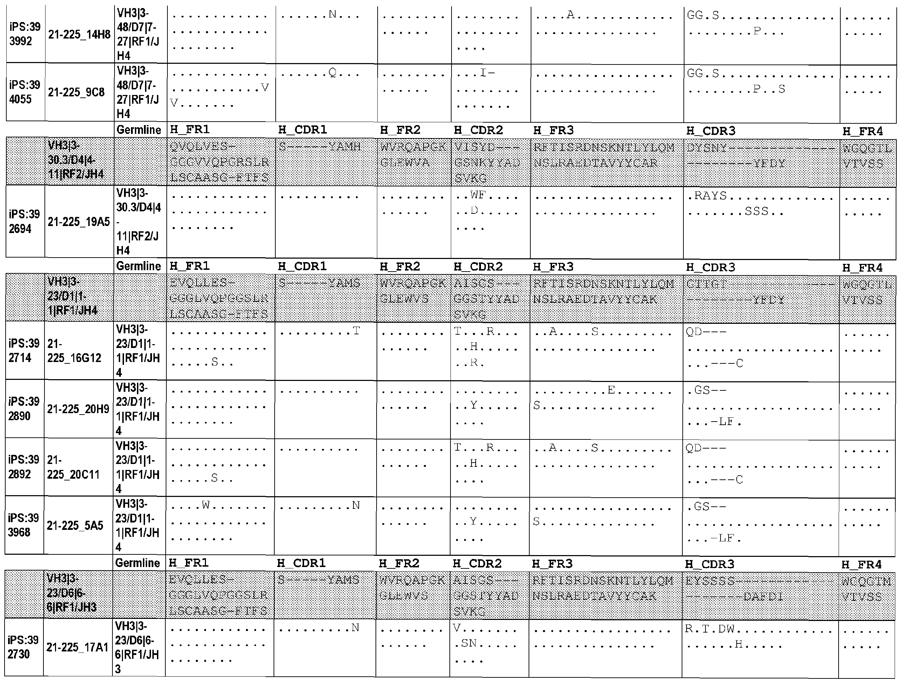

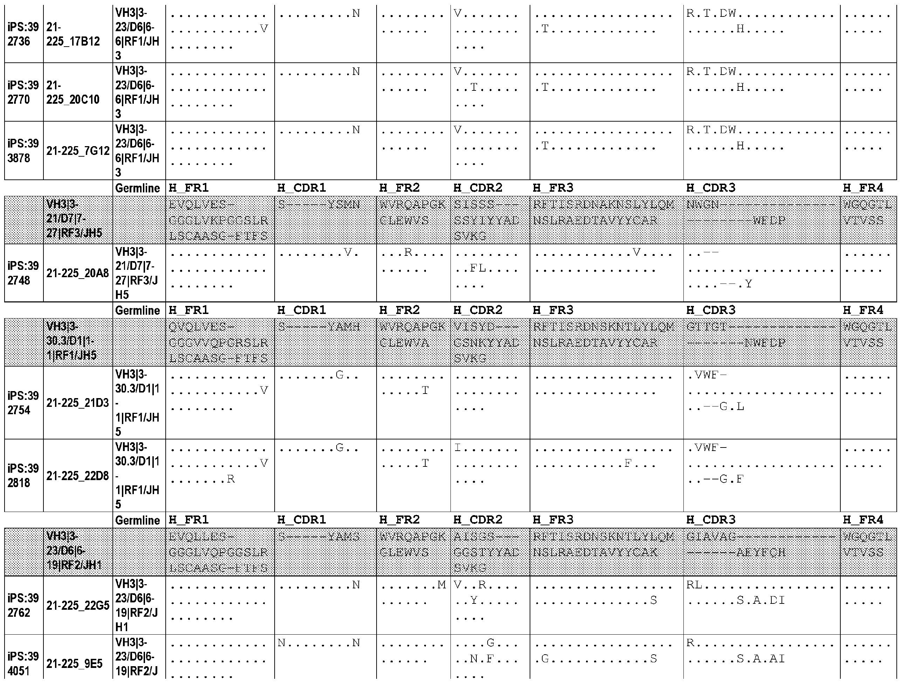

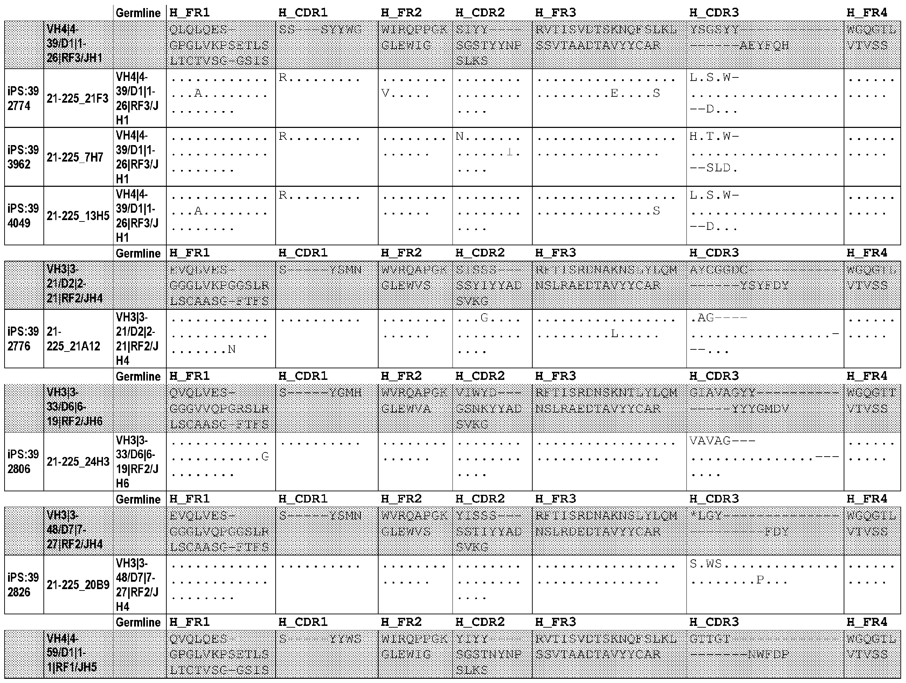

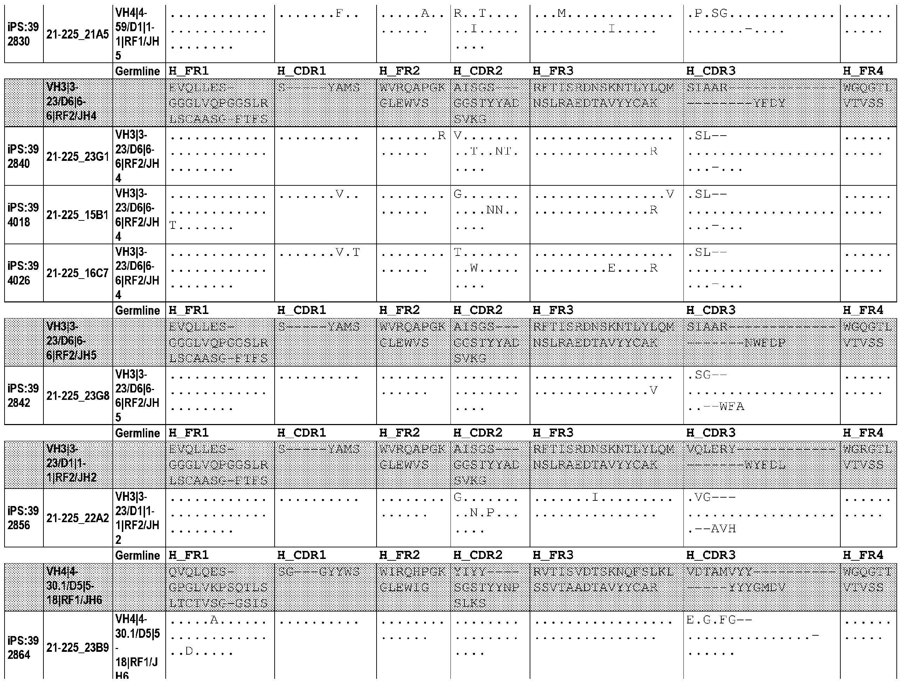

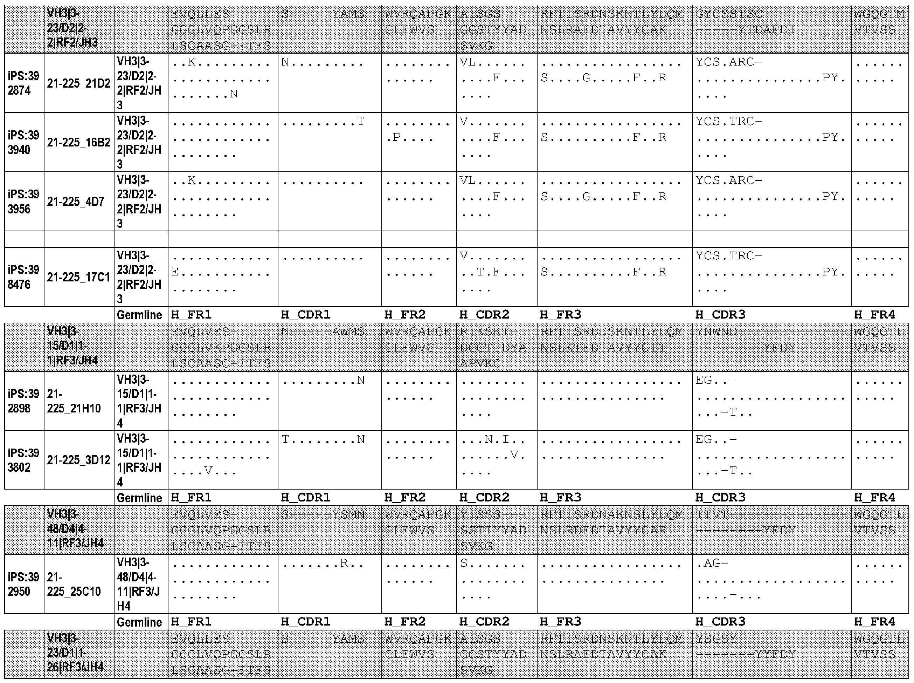

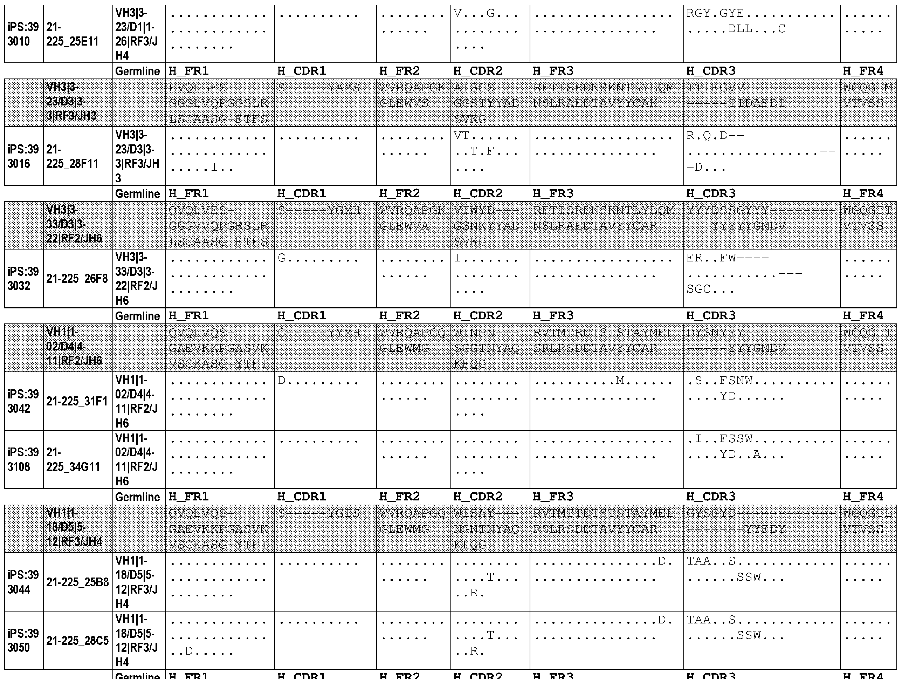

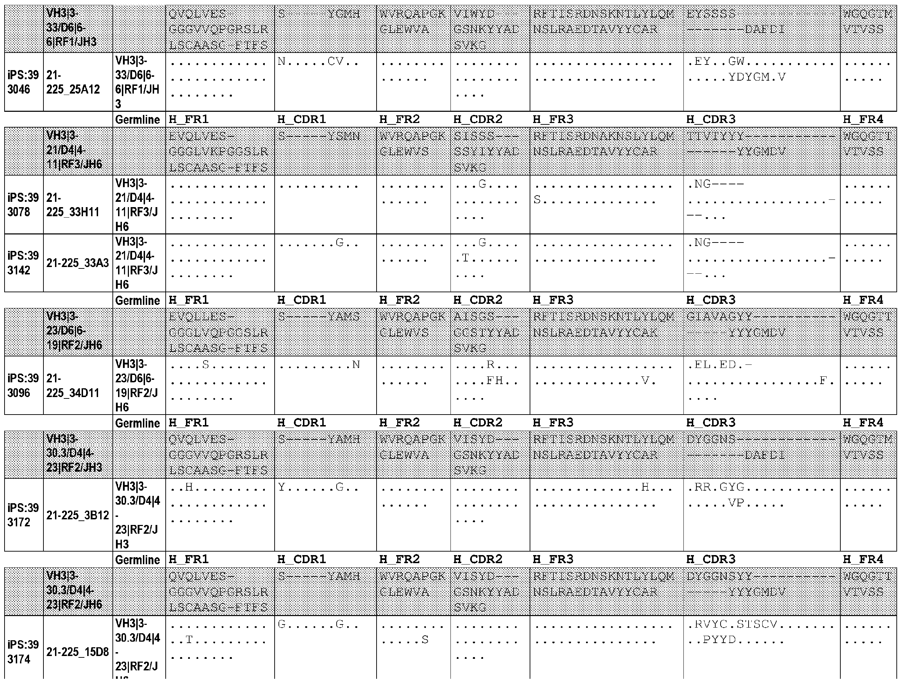

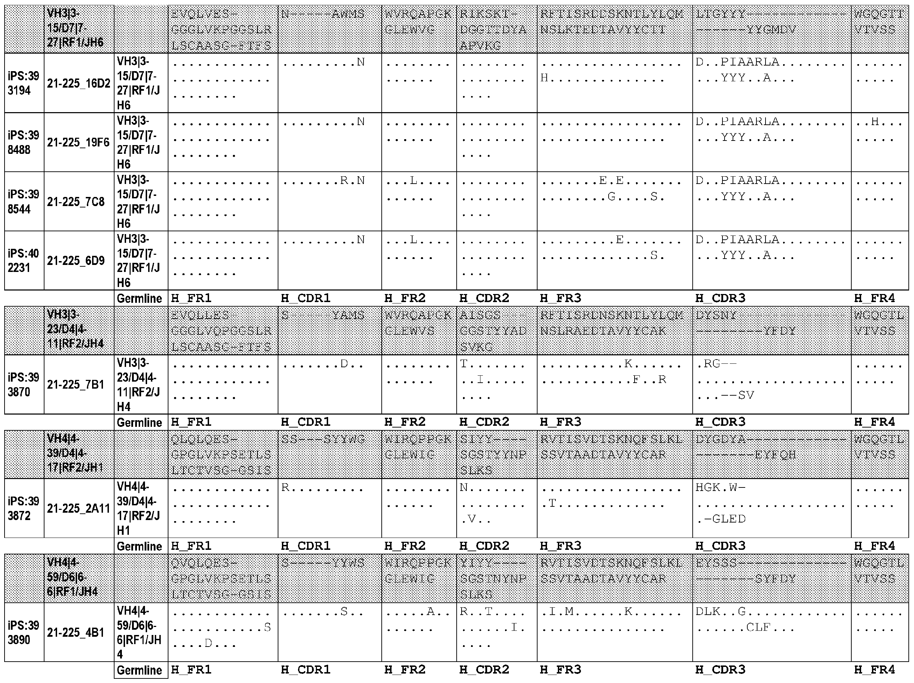

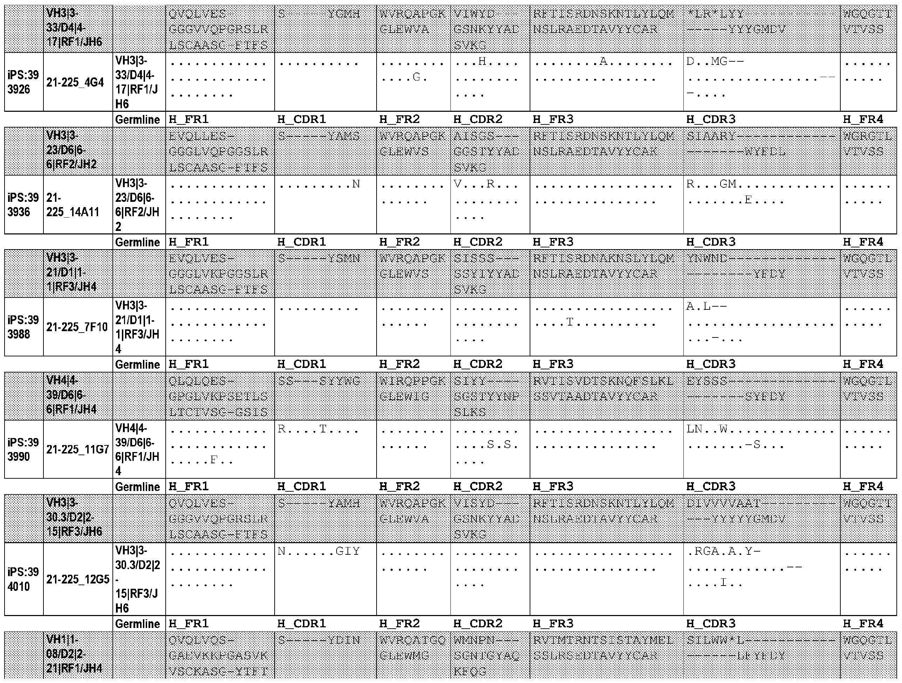

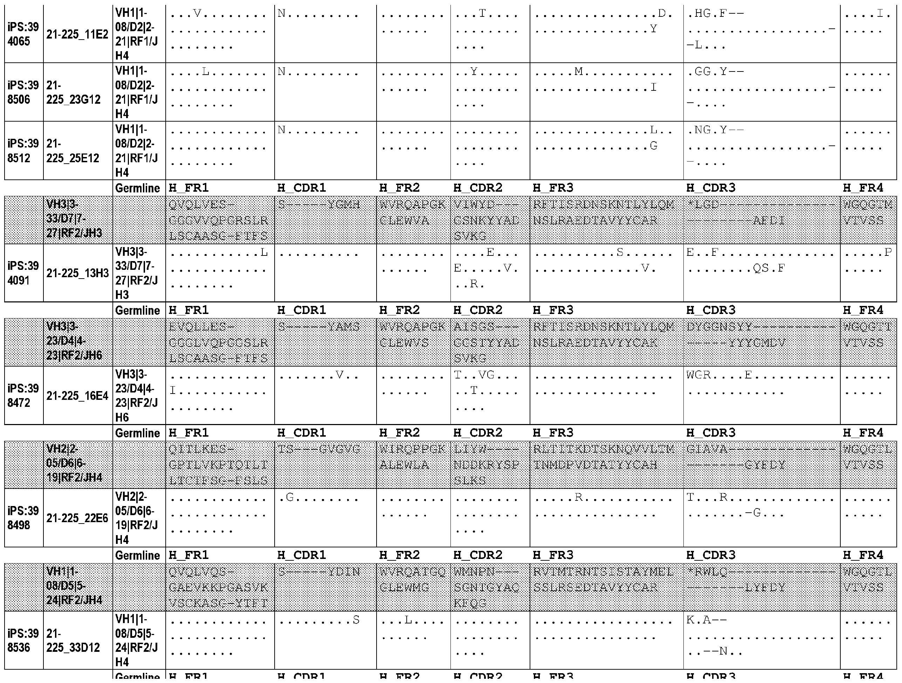

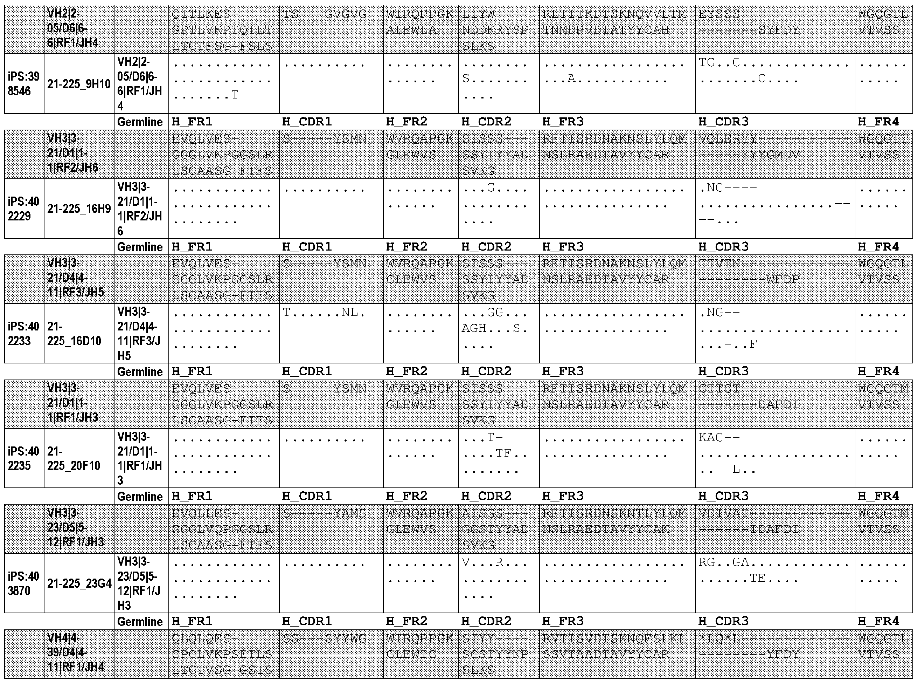

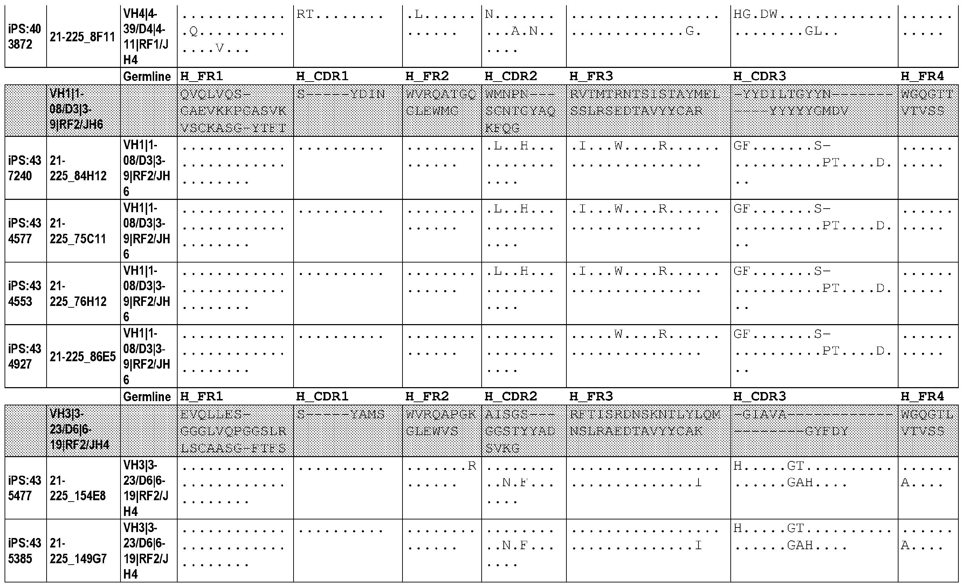

**Figure 52.** A table presenting a consensus protein alignment of light and heavy variable regions for certain antigen binding proteins (Table 5). An asterisk "*" denotes a stop codon. Sequences containing a stop codon are respresented as distinct sequences in the Sequence Listing, however, these sequences are related. Generally speaking, however, the amino acid sequences of the light and heavy chain variable domains in the consensus protein alignment presented in Table 5 are wrapped due to space issues, and unless stated otherwise, like in the case of sequences with one or more stop codons, should be understood to be single amino acid sequences.

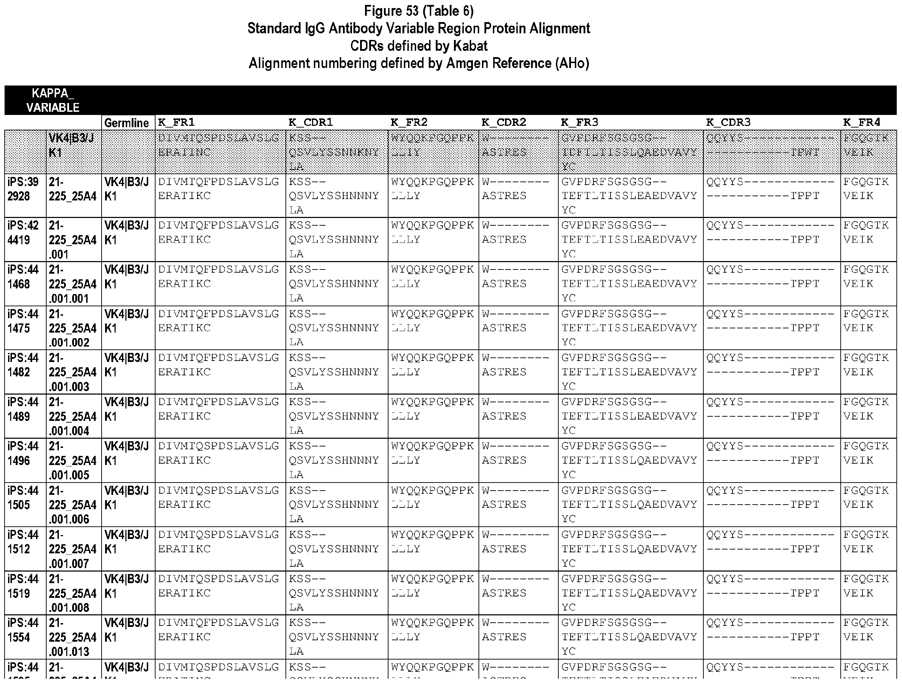

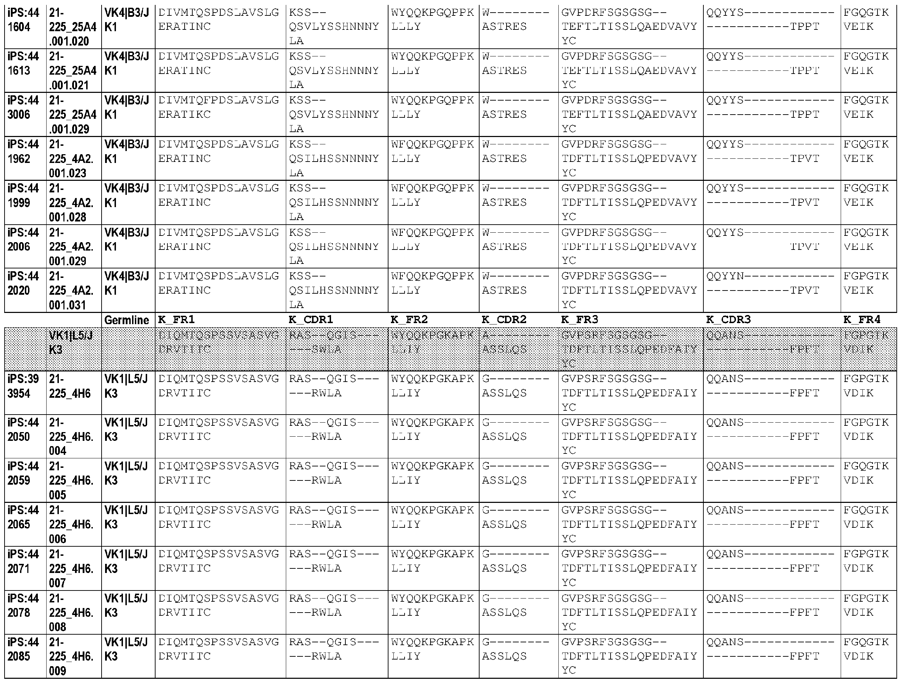

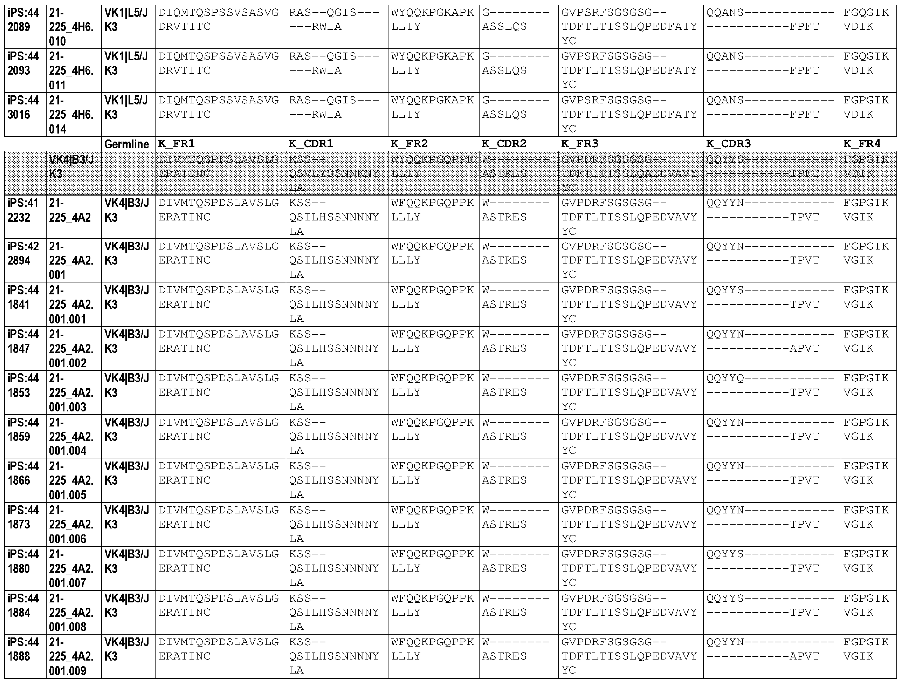

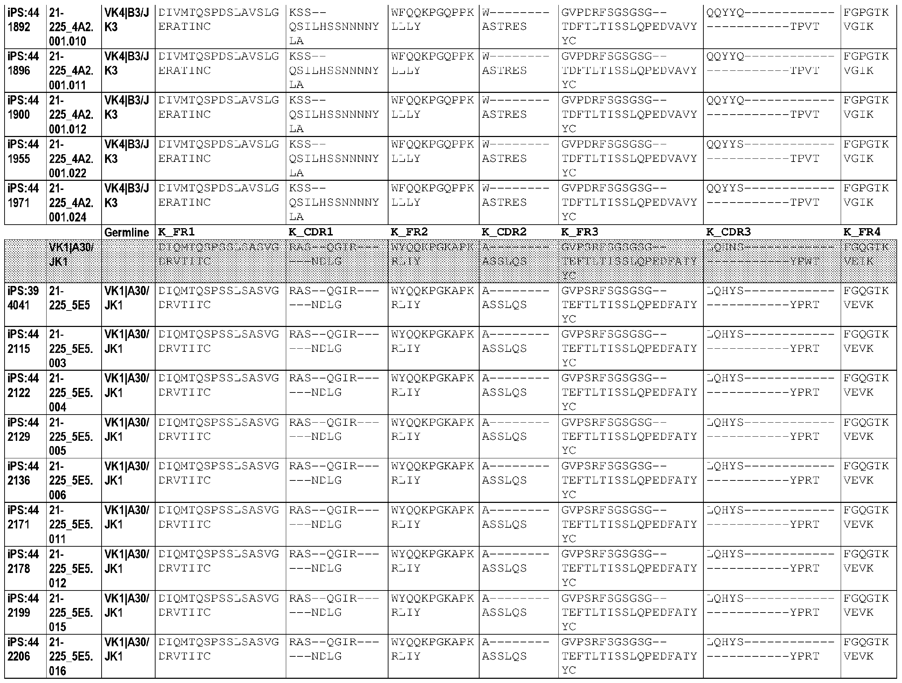

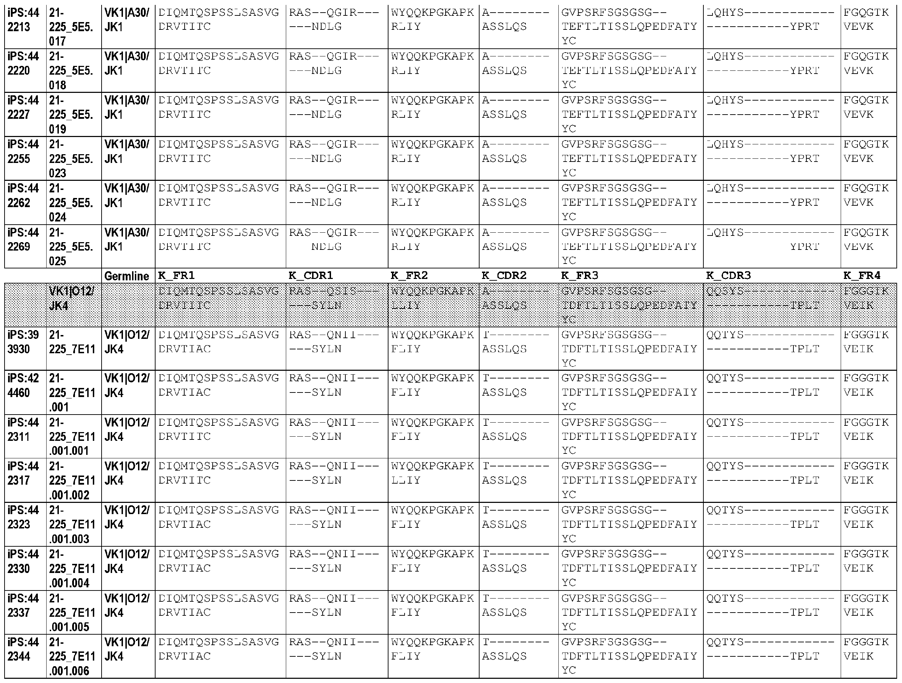

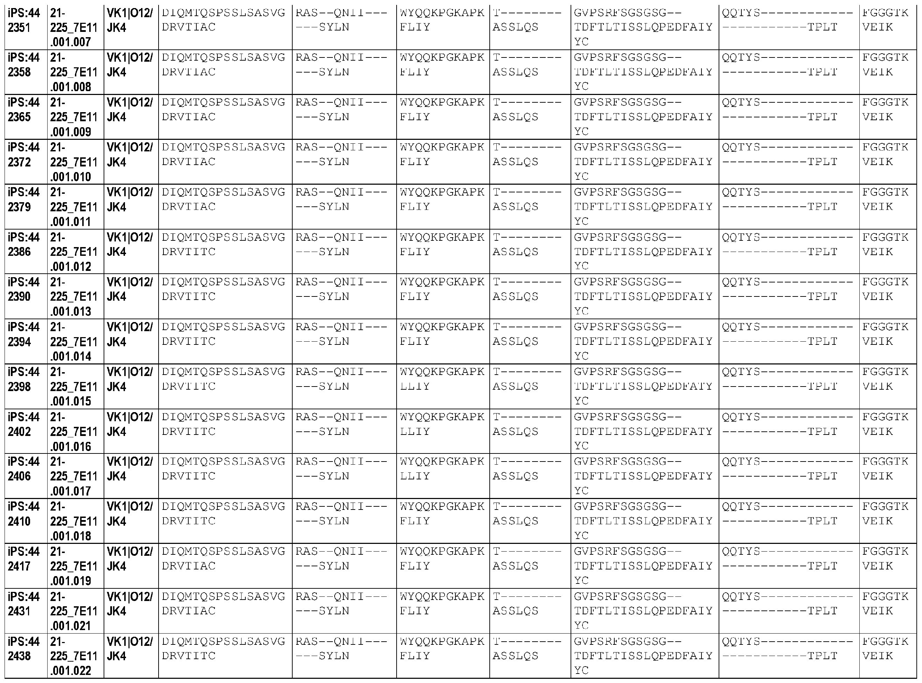

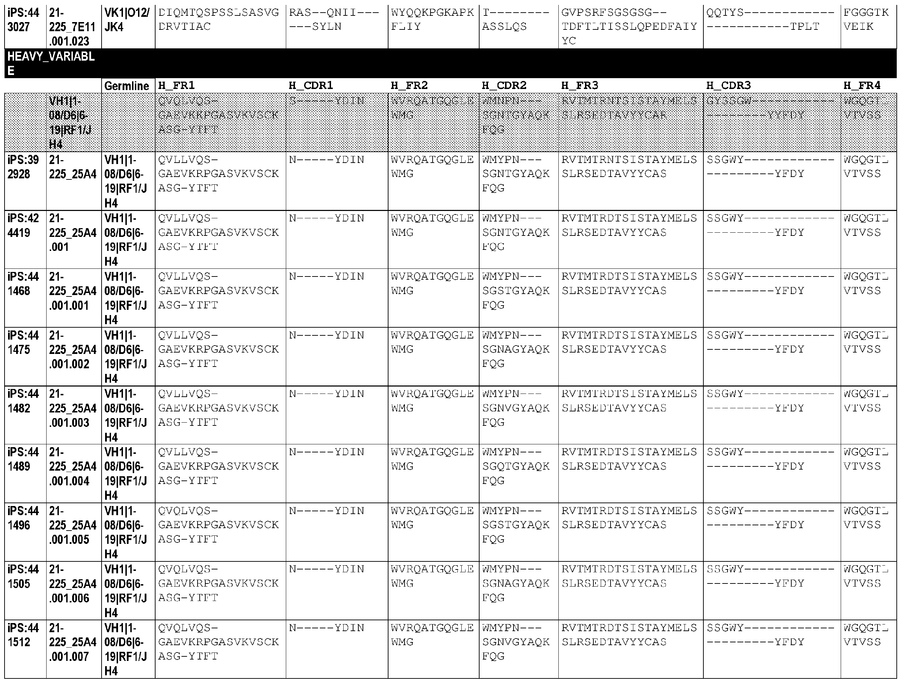

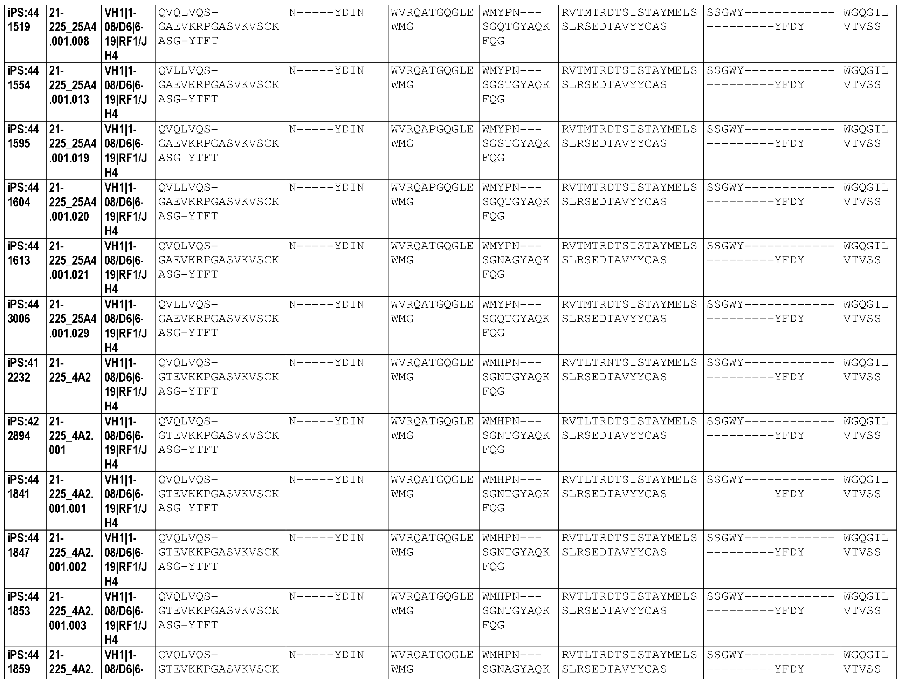

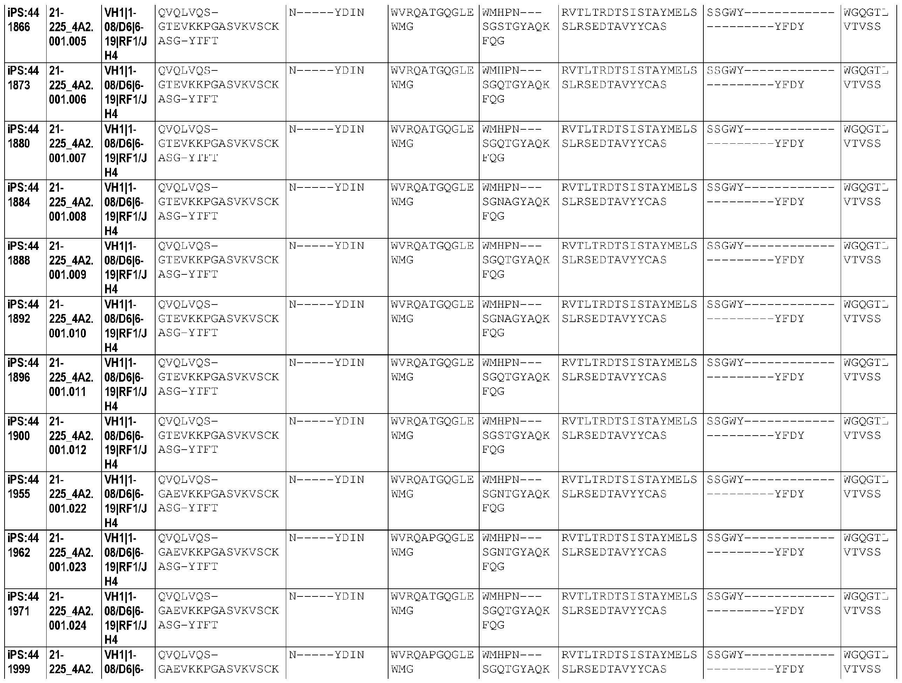

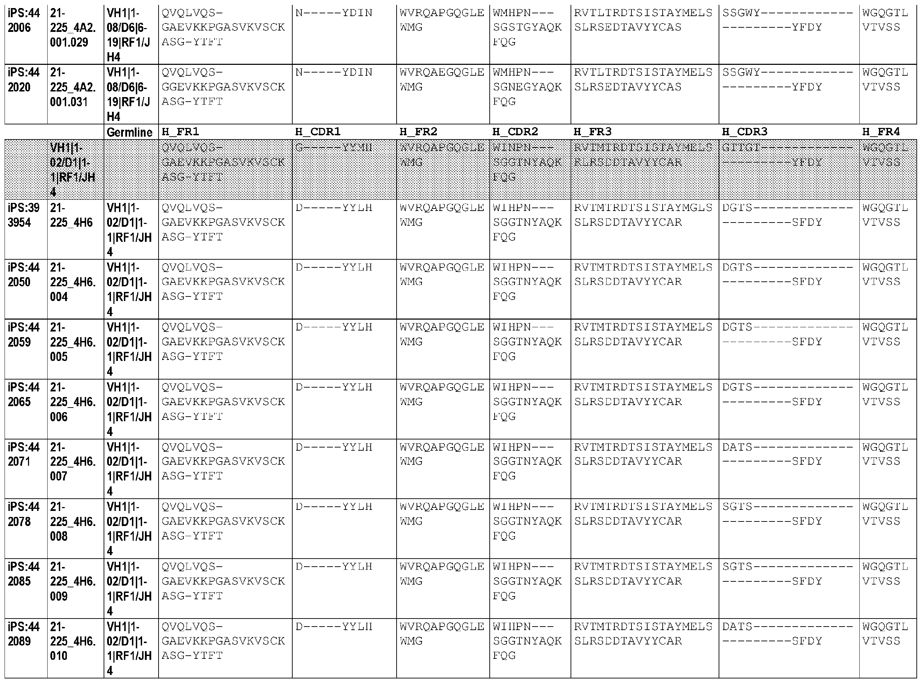

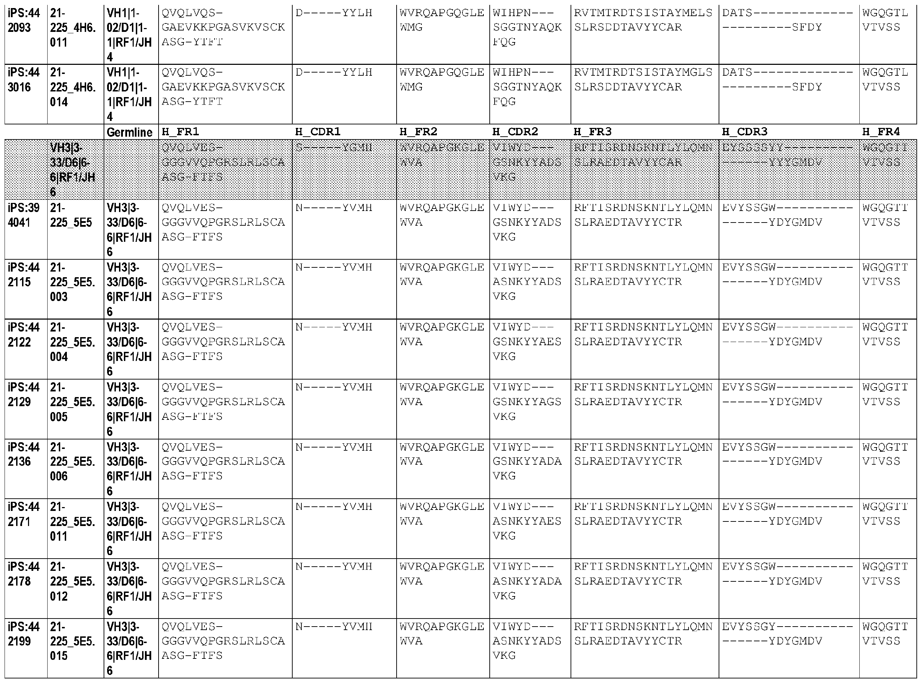

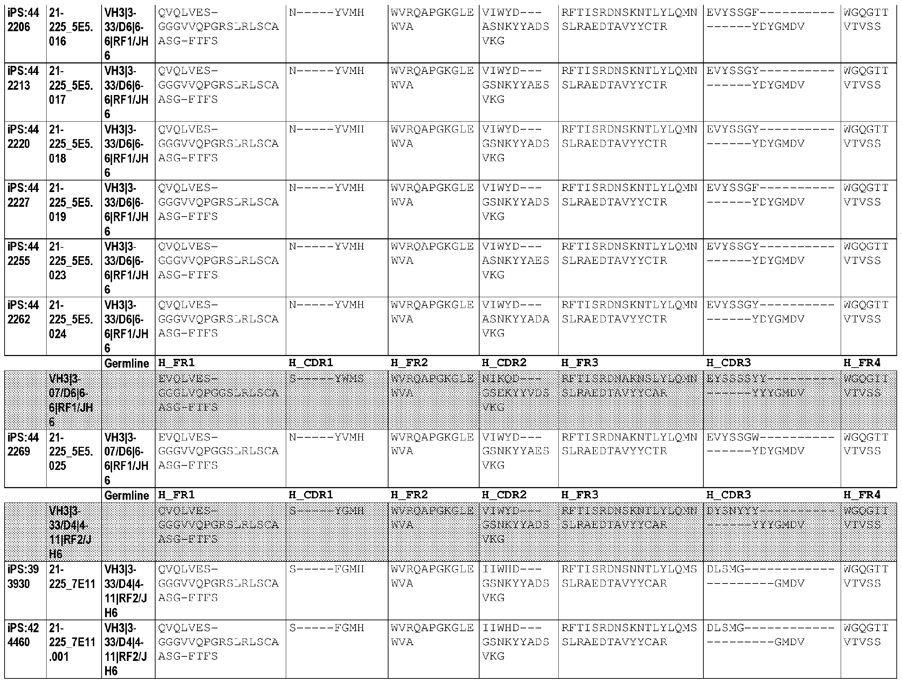

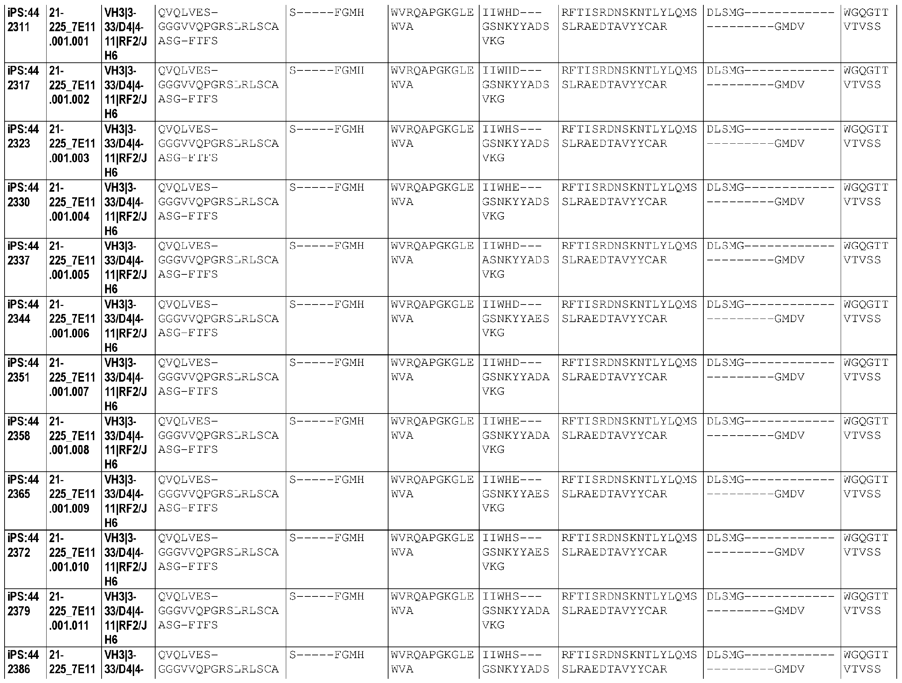

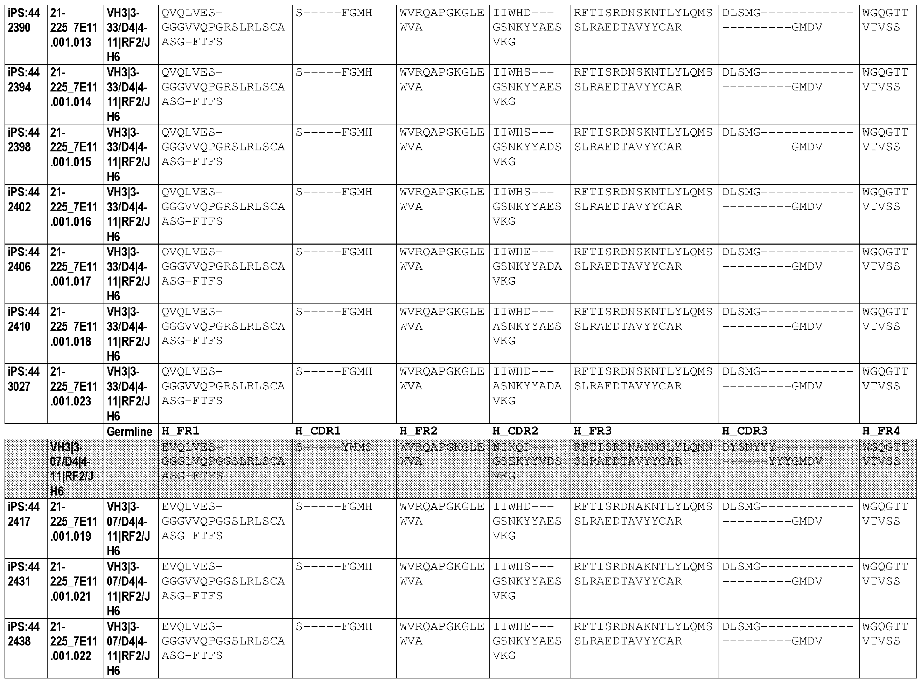

**Figure** 53. A table presenting a protein alignment of light and heavy variable regions for certain optimized antigen binding proteins (Table 6). The amino acid sequences of the light and heavy chain variable domains in the protein alignment presented in Table 6 are wrapped due to space issues, and unless stated otherwise, should be understood to be single amino acid sequences.

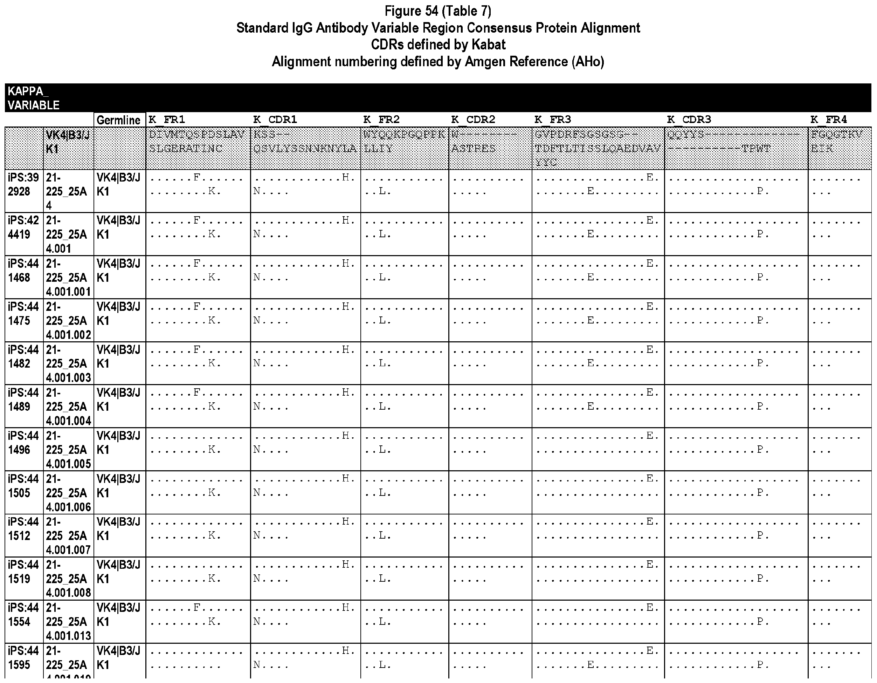

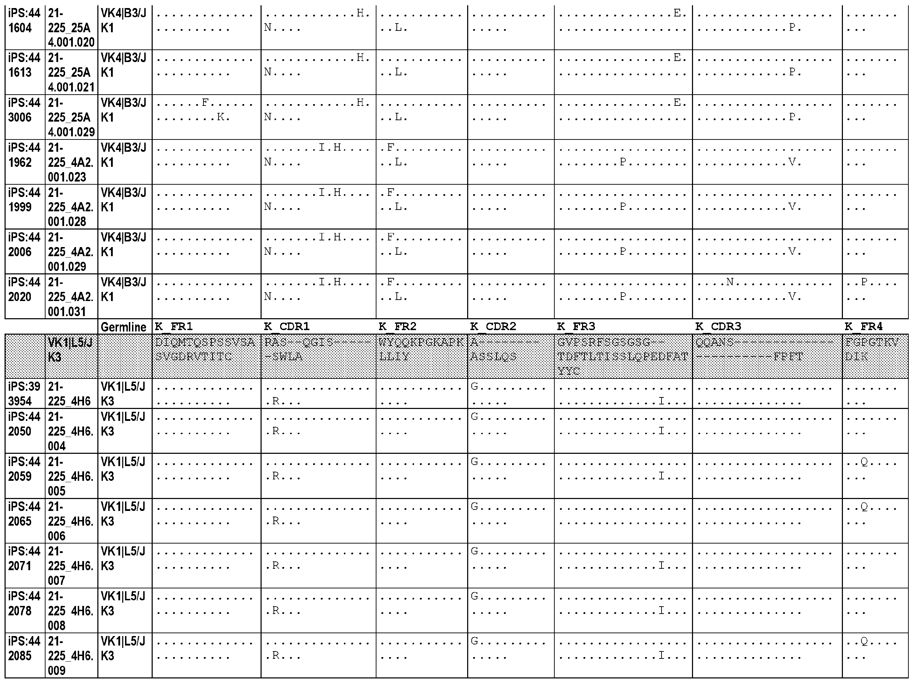

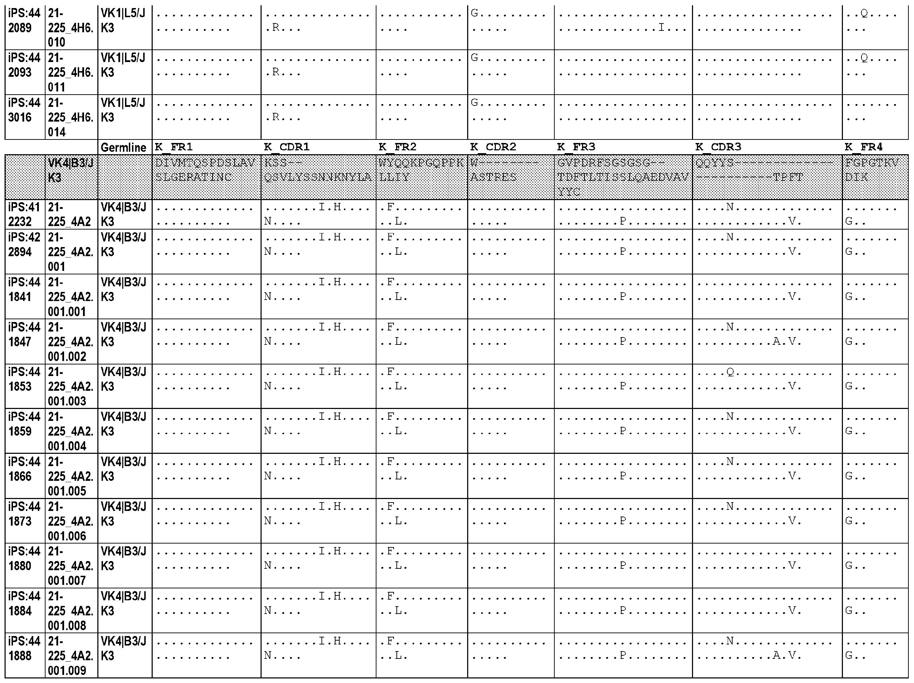

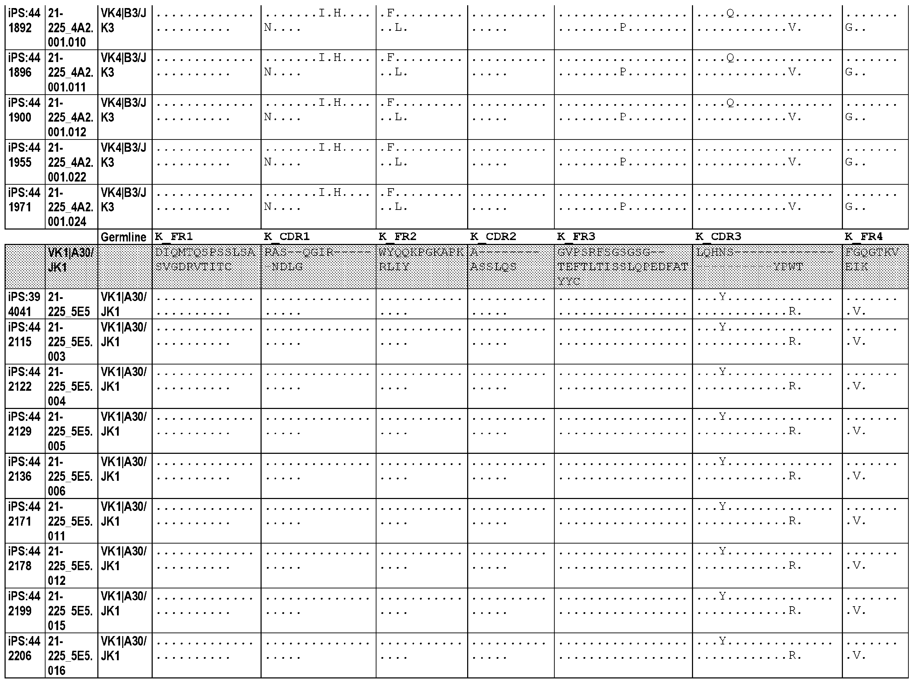

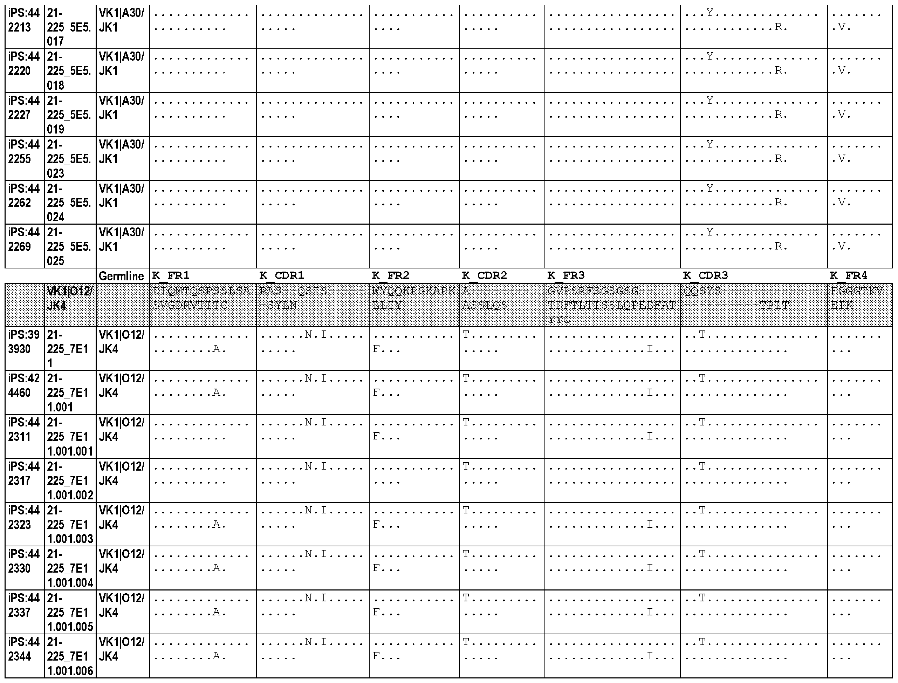

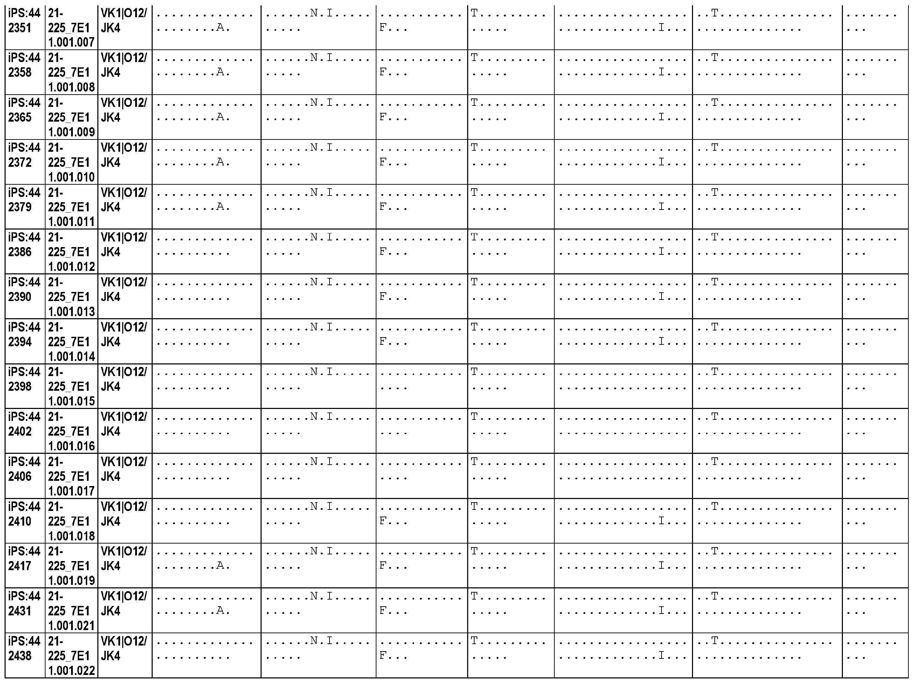

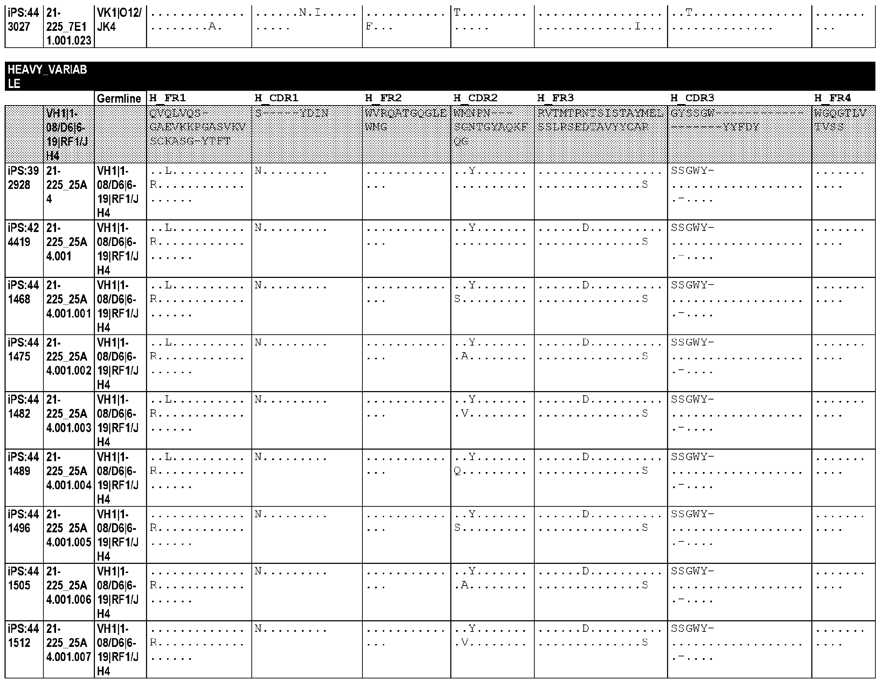

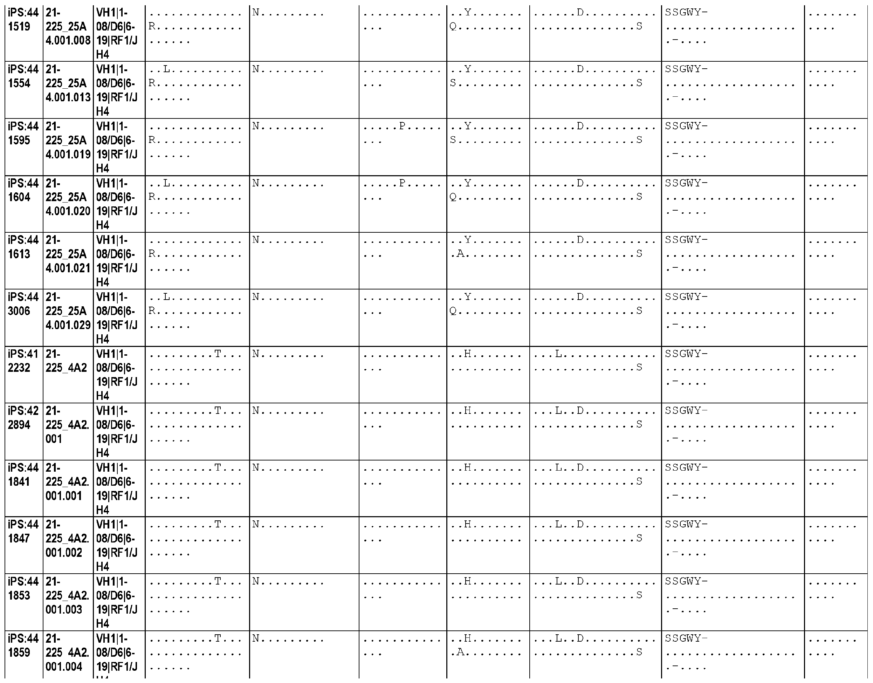

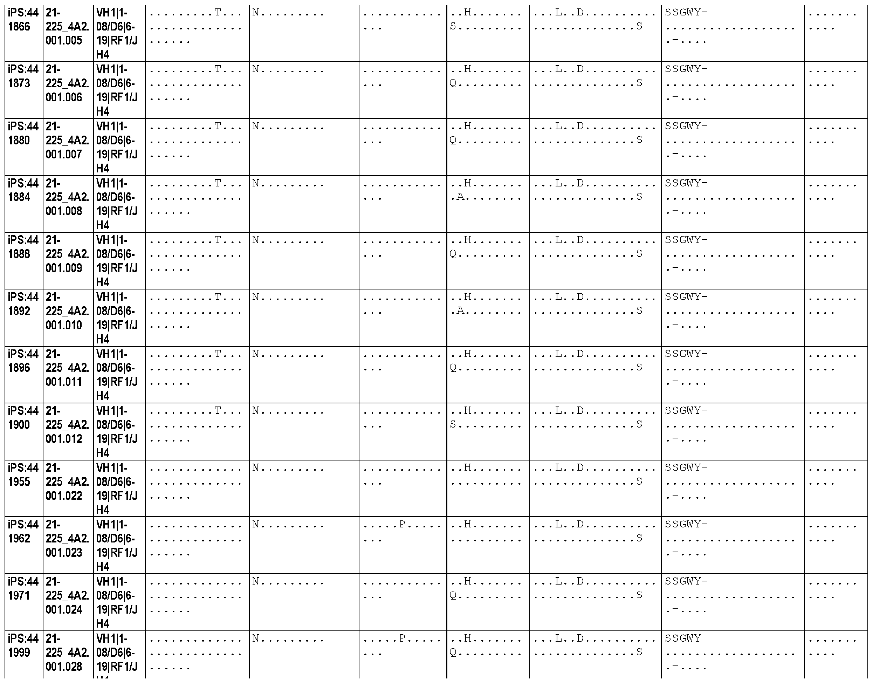

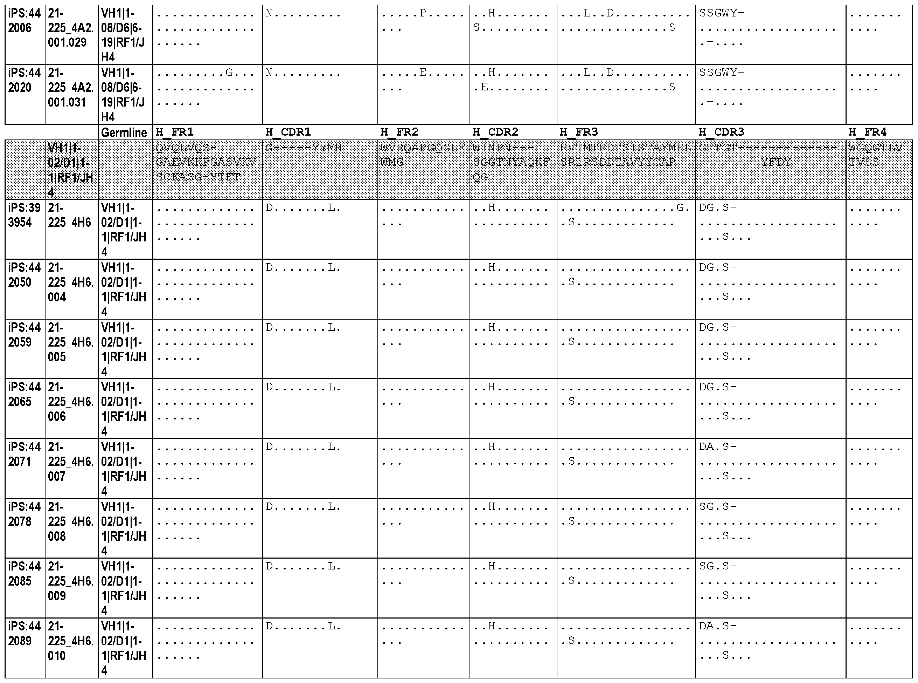

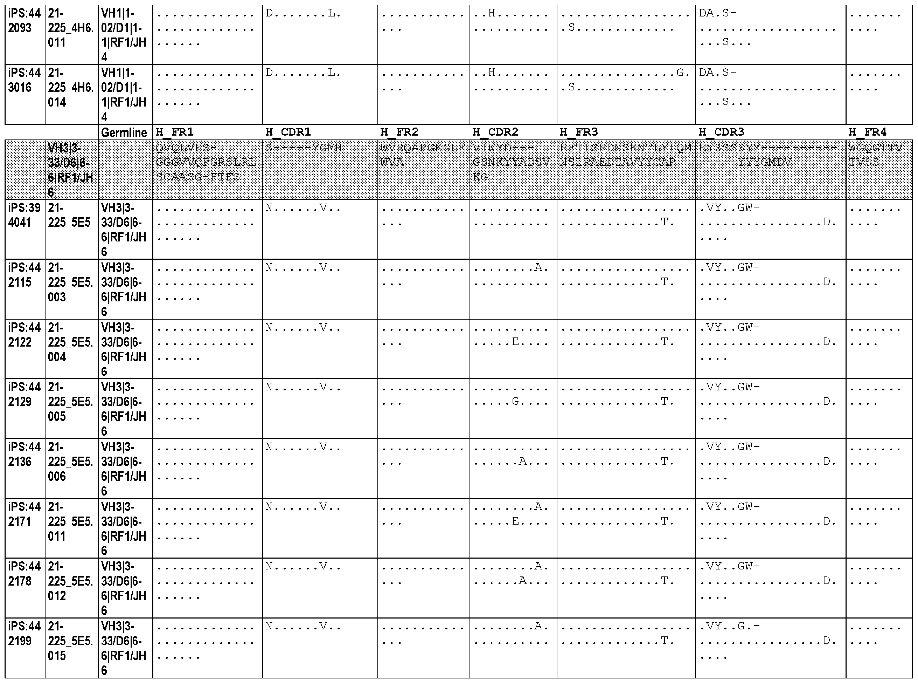

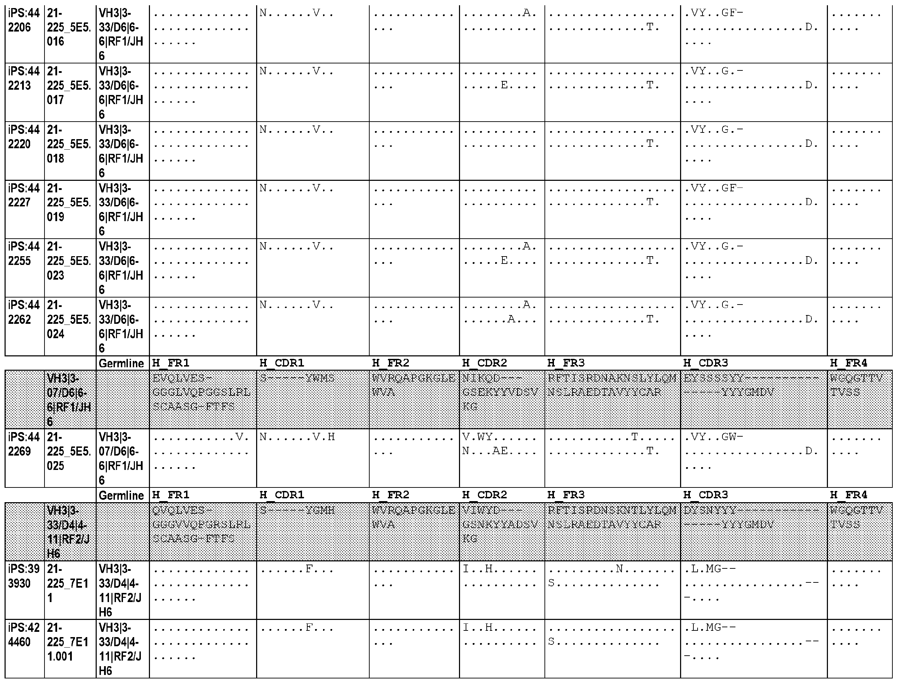

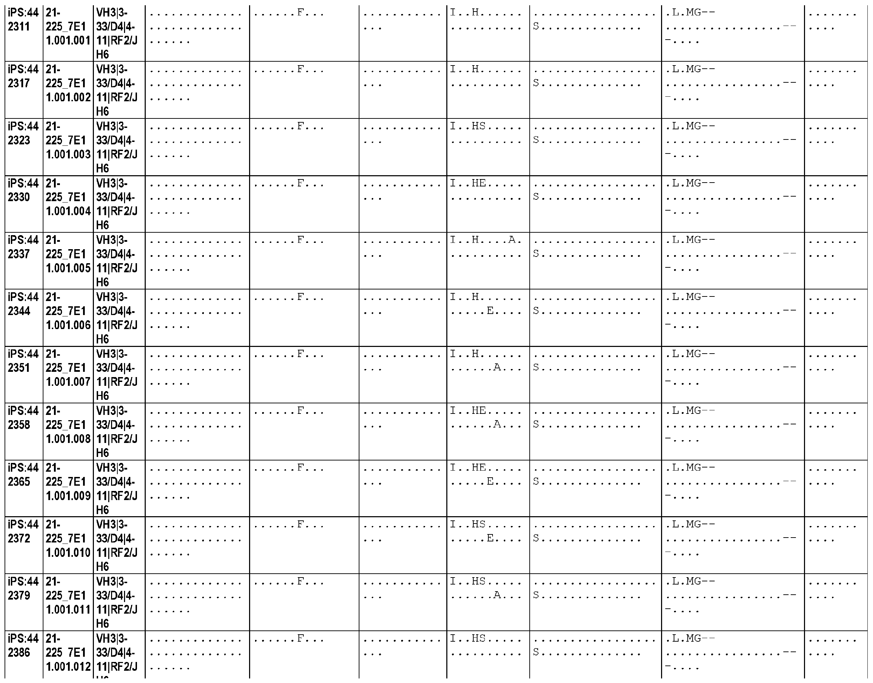

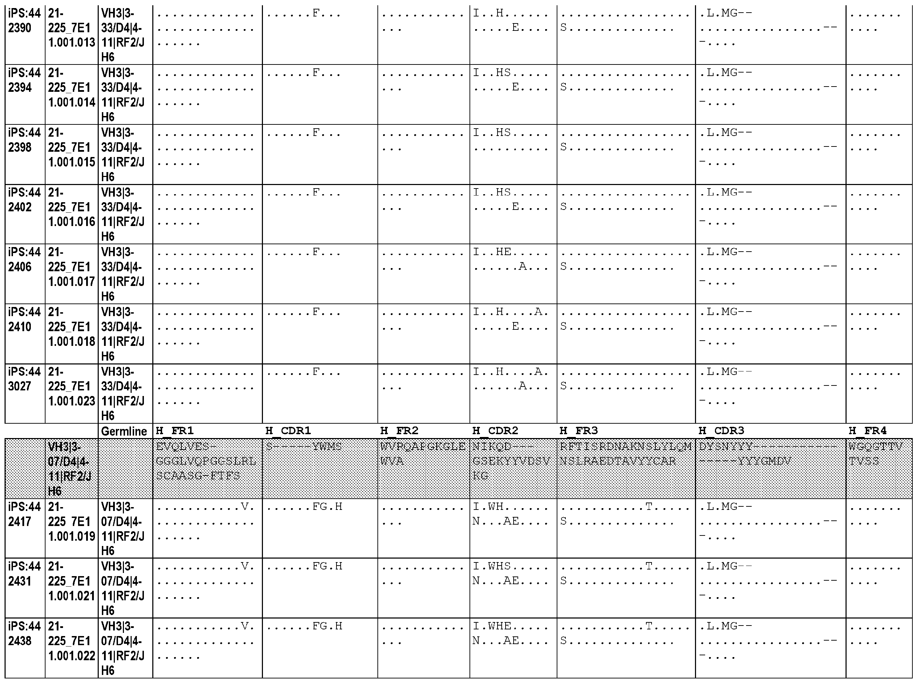

**Figure 54.** A table presenting a consensus protein alignment of light and heavy variable regions for certain optimized antigen binding proteins (Table 7). The amino acid sequences of the light and heavy chain variable domains in the consensus protein alignment presented in Table 7 are wrapped due to space issues, and unless stated otherwise, should be understood to be single amino acid sequences.

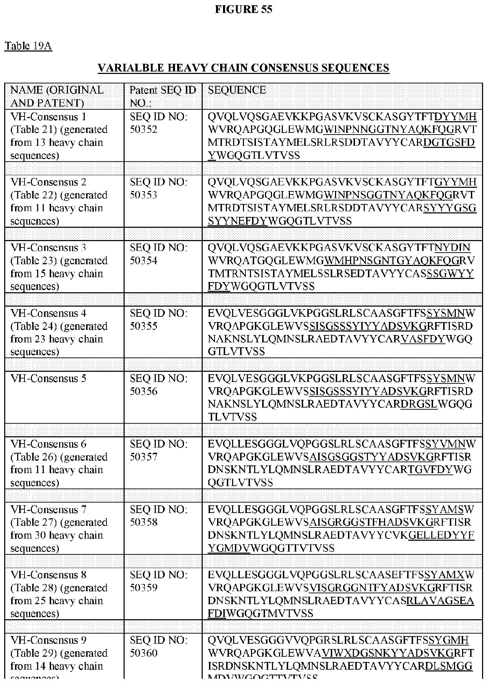

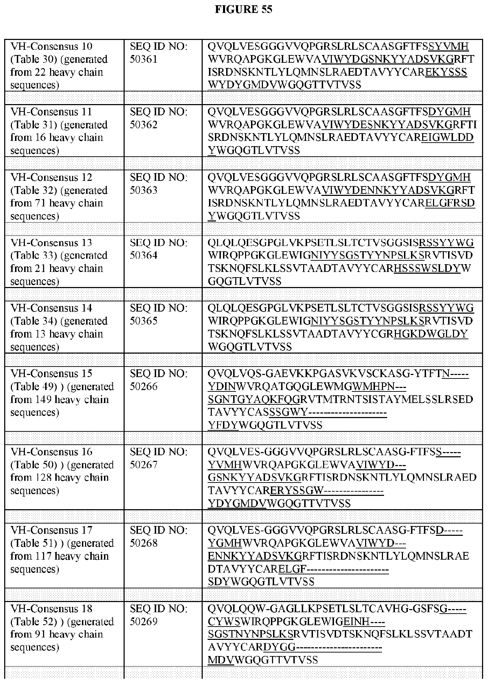

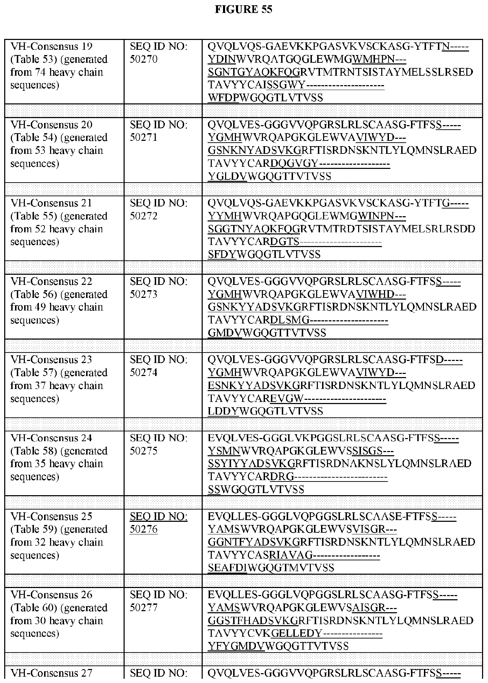

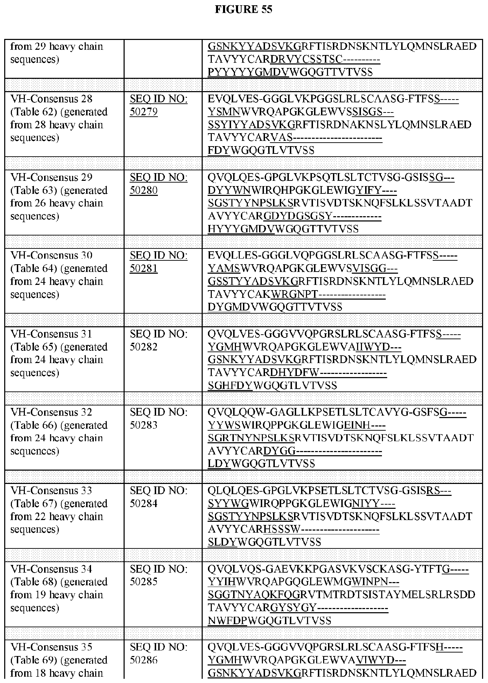

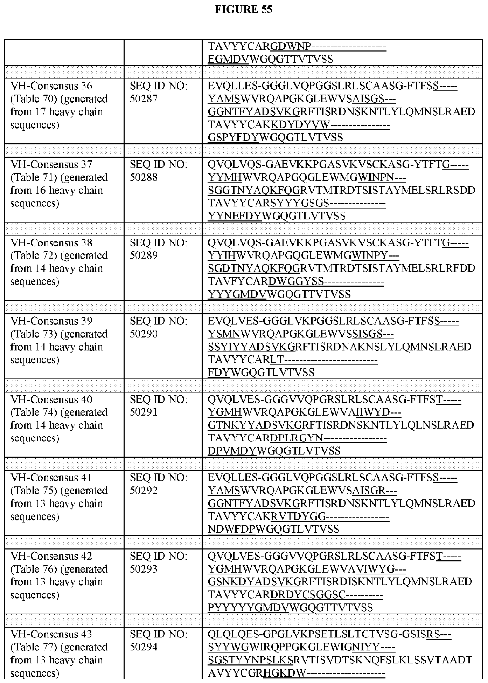

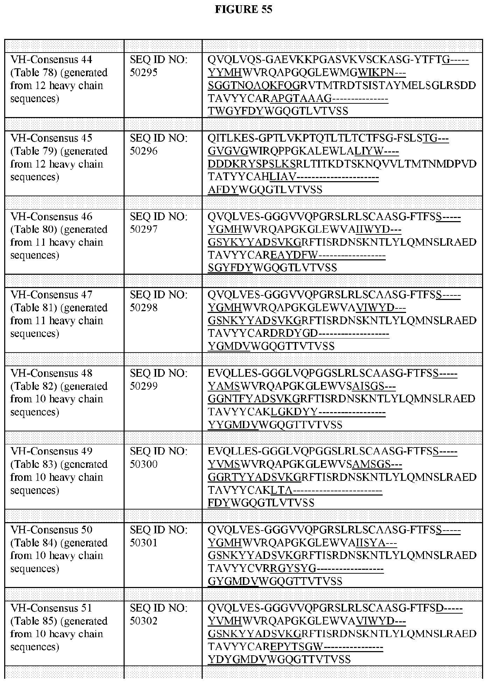

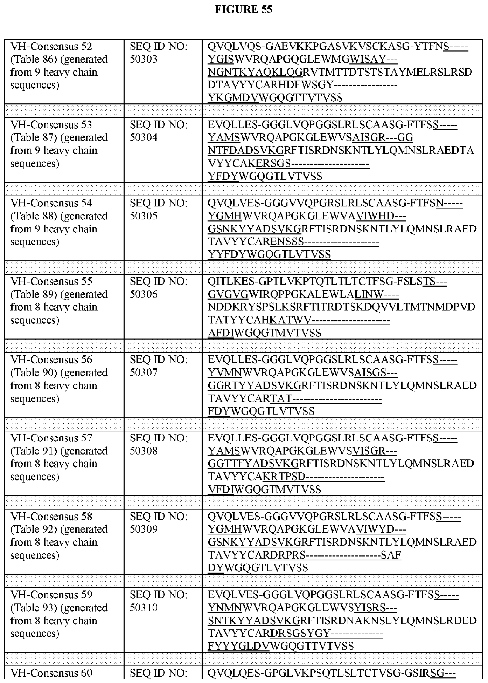

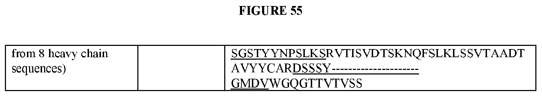

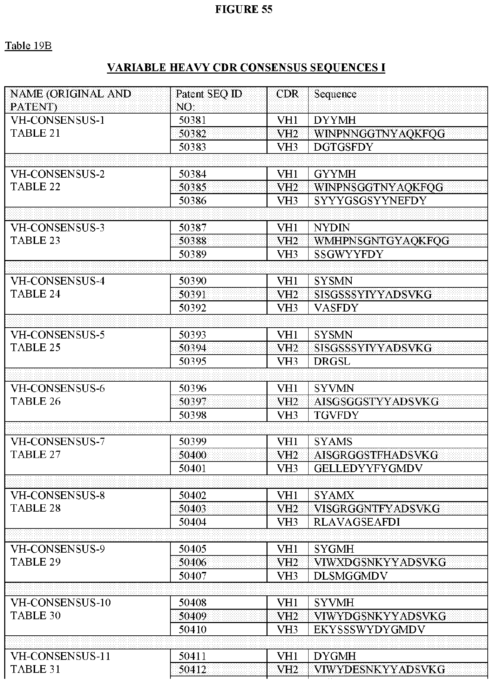

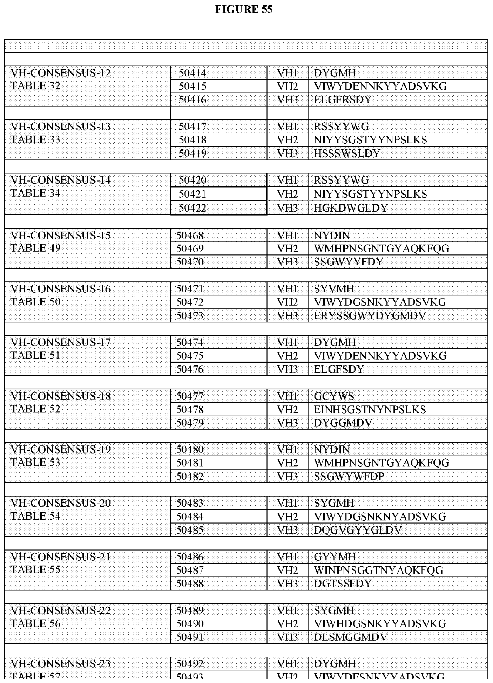

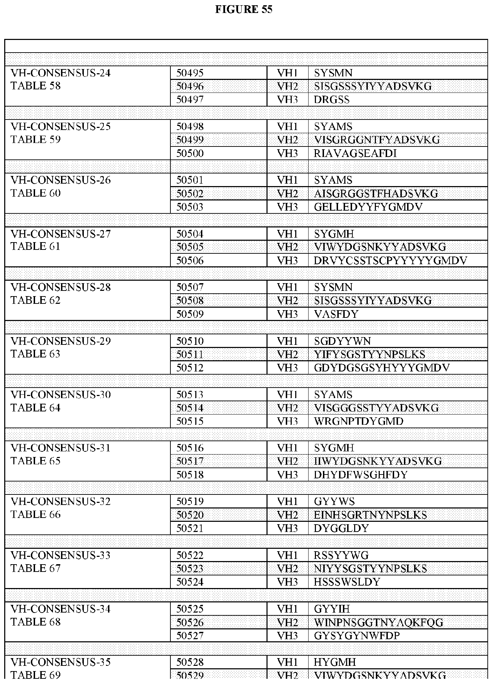

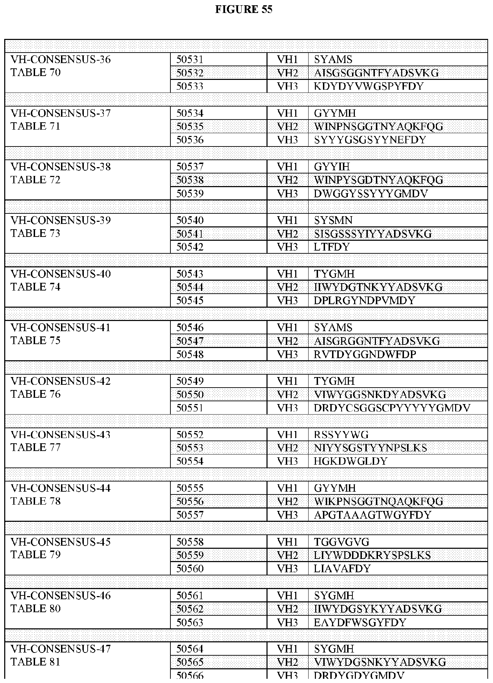

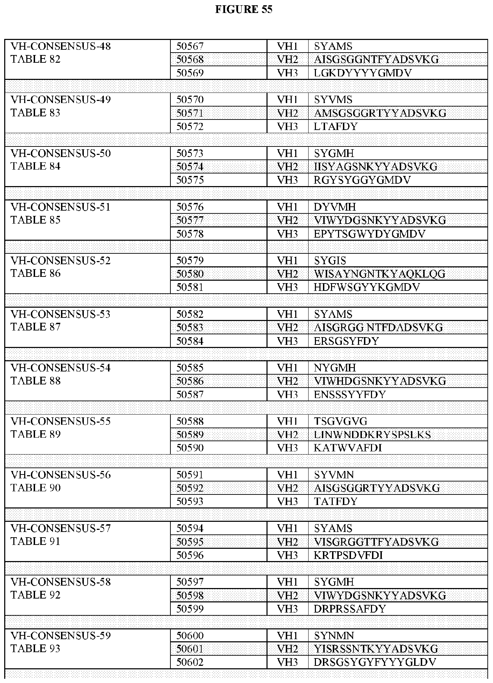

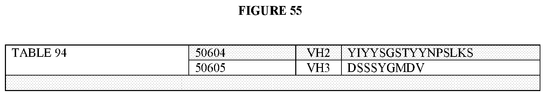

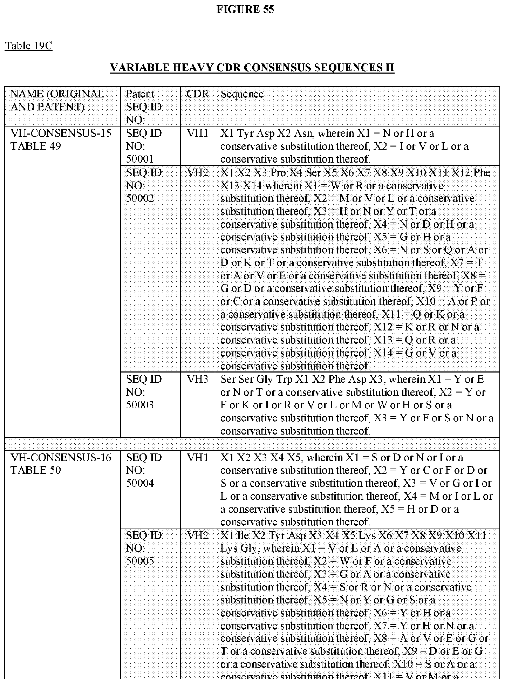

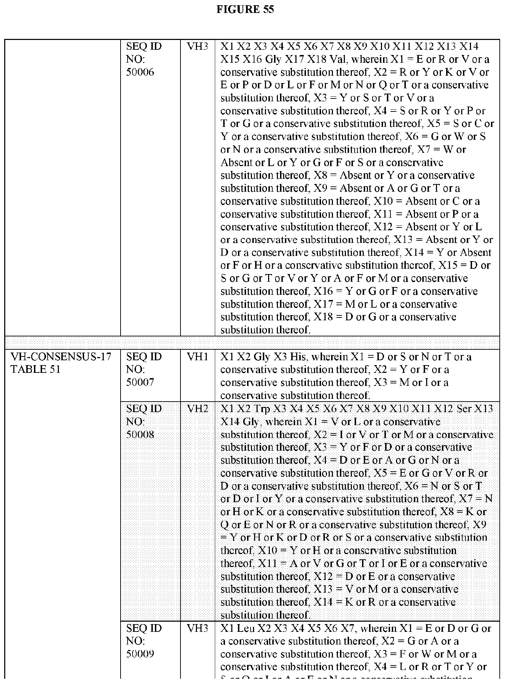

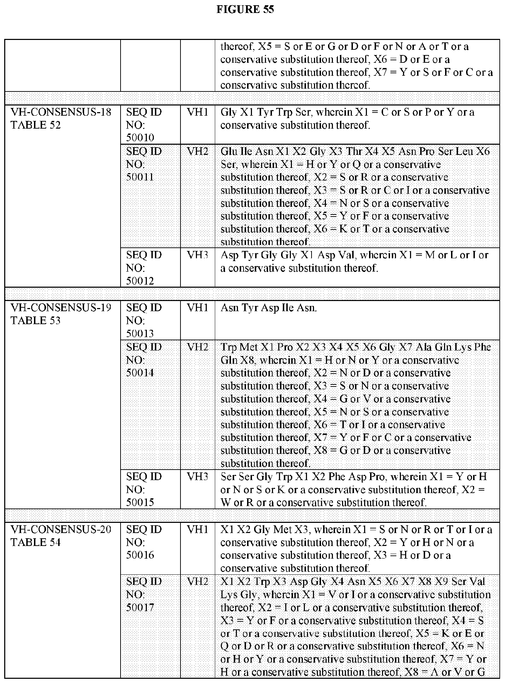

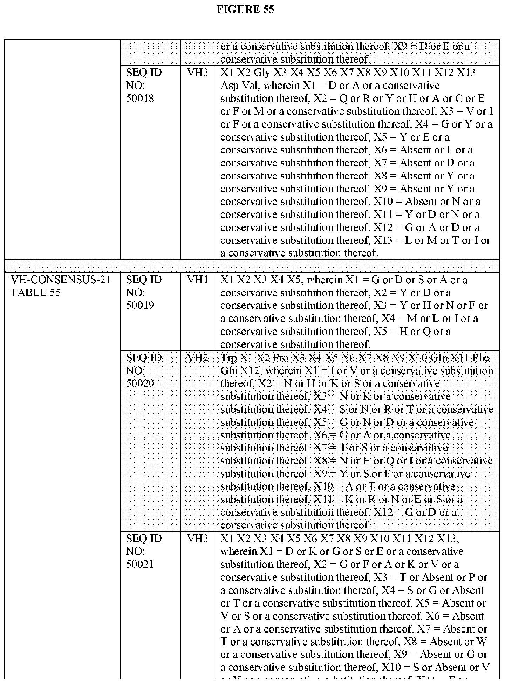

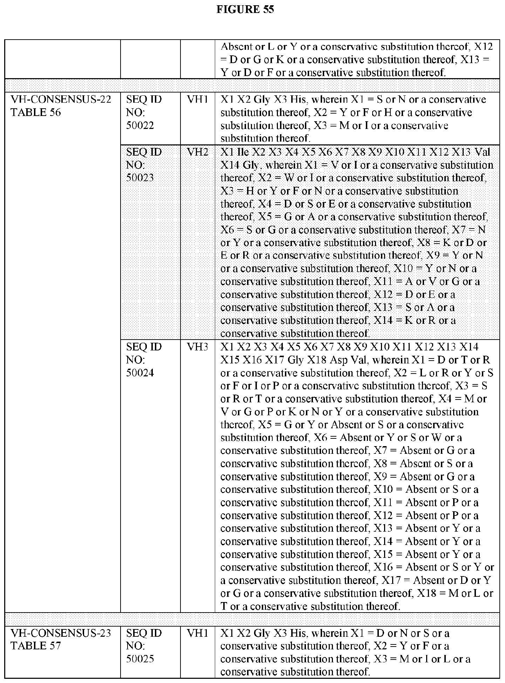

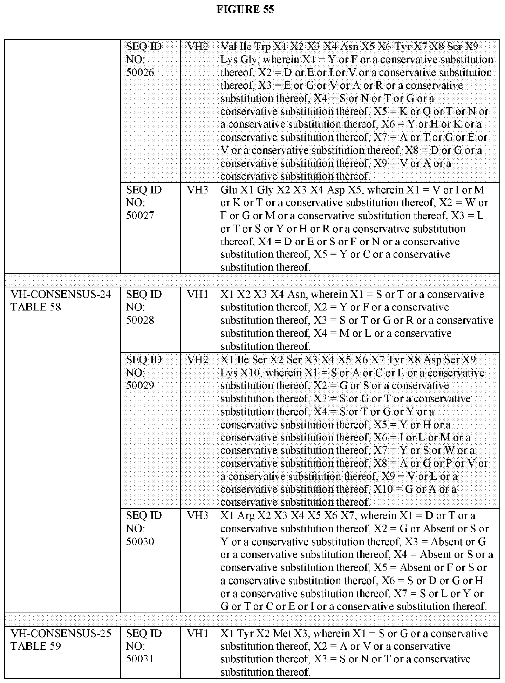

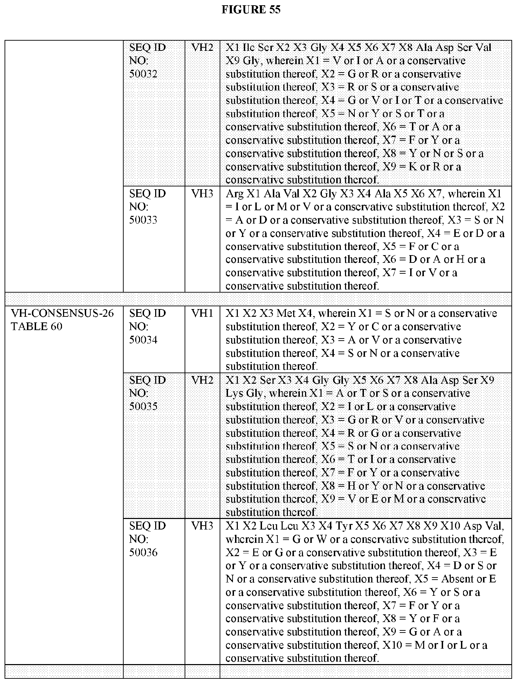

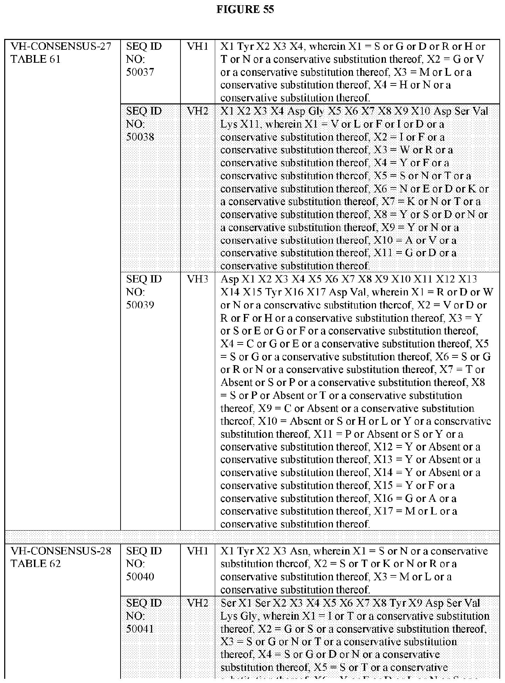

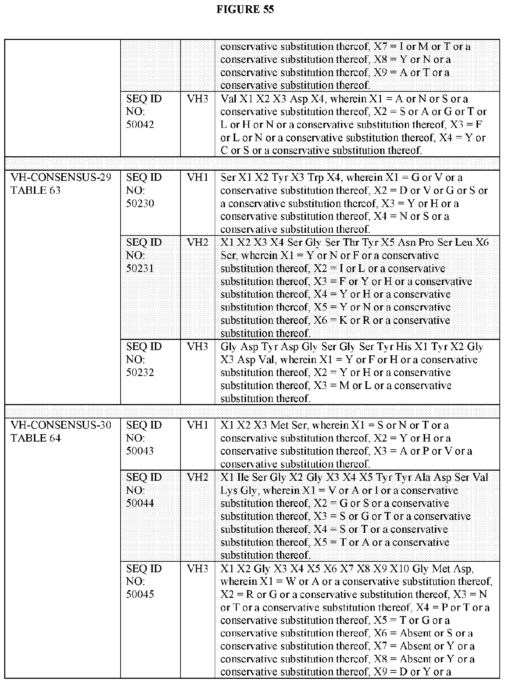

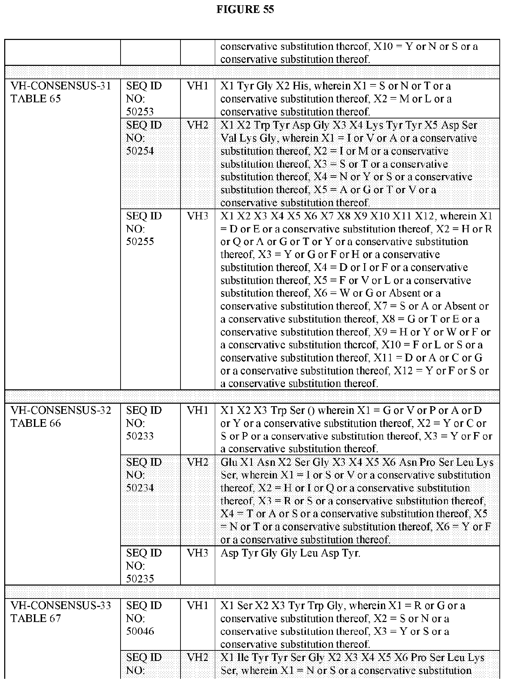

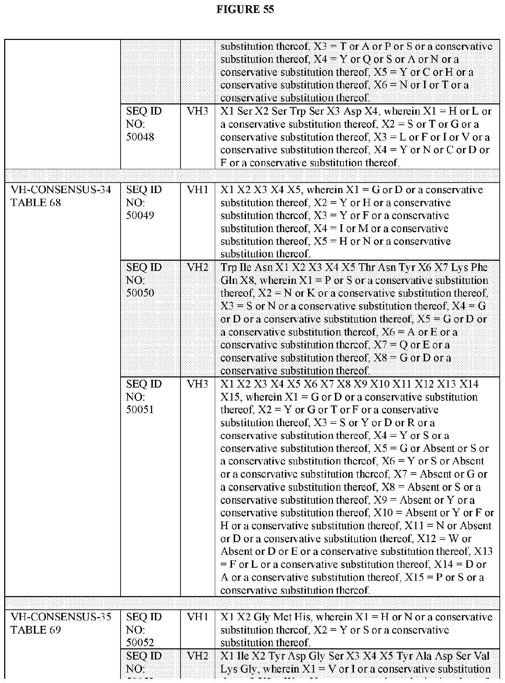

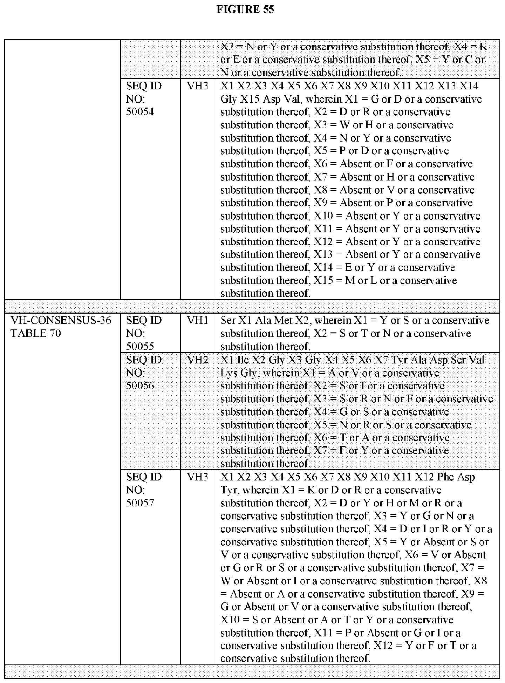

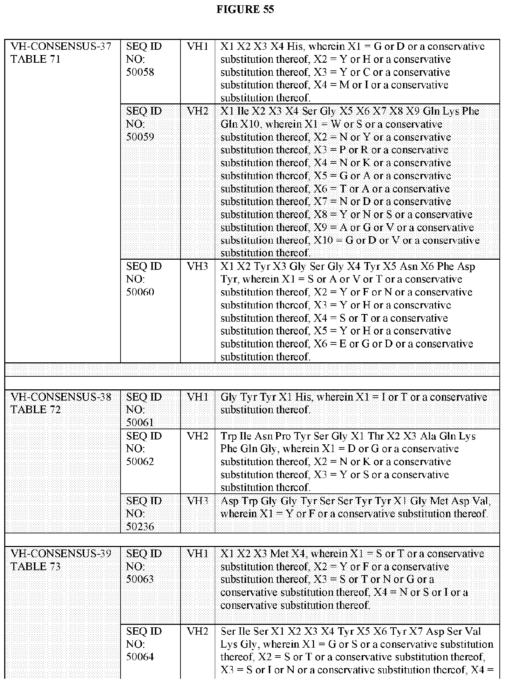

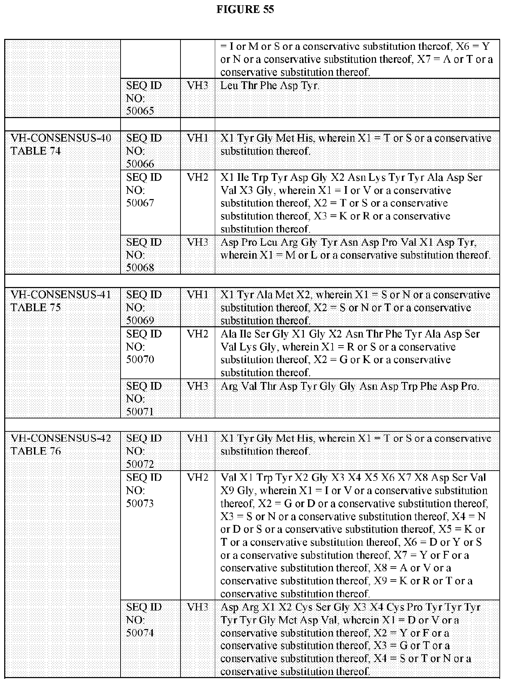

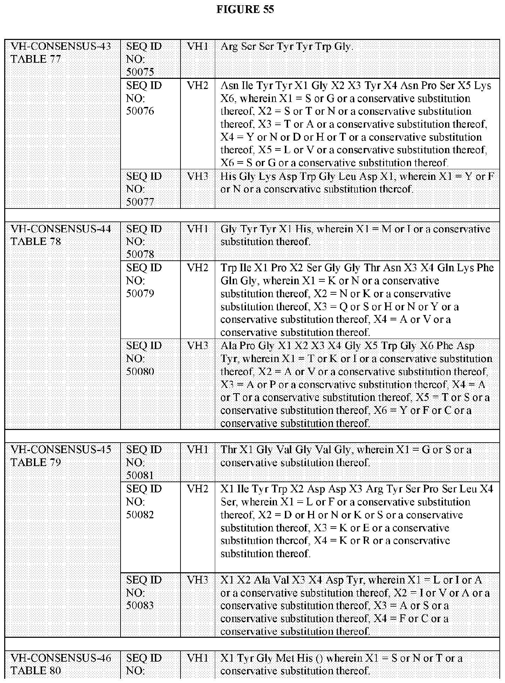

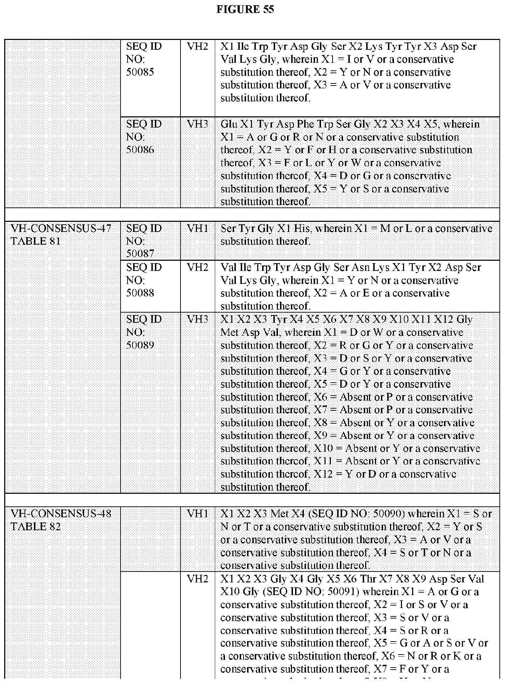

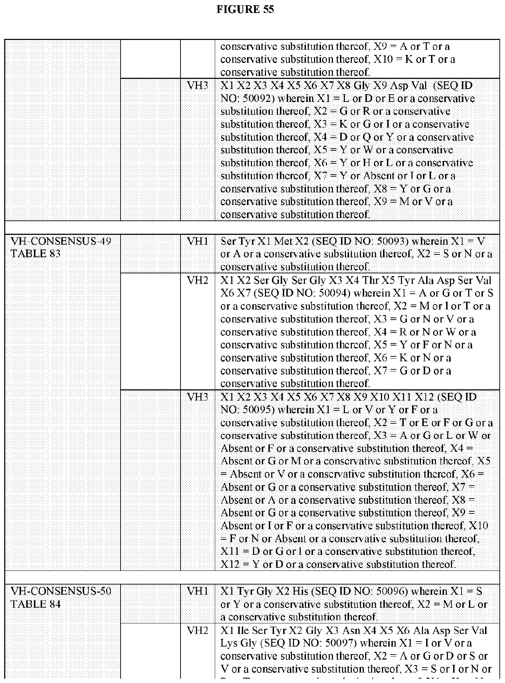

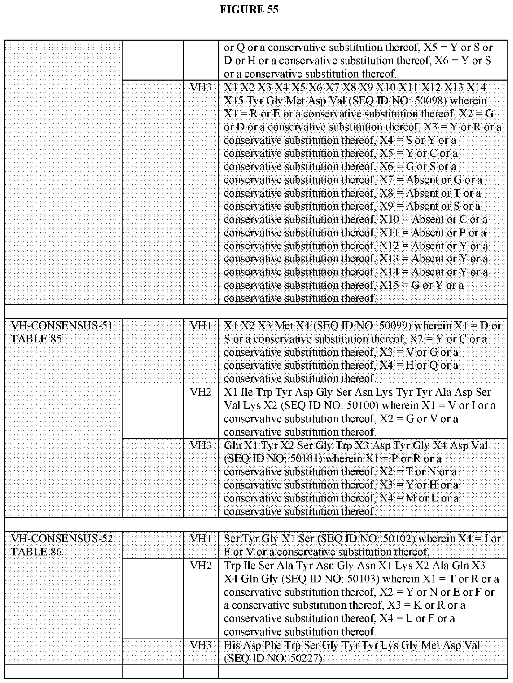

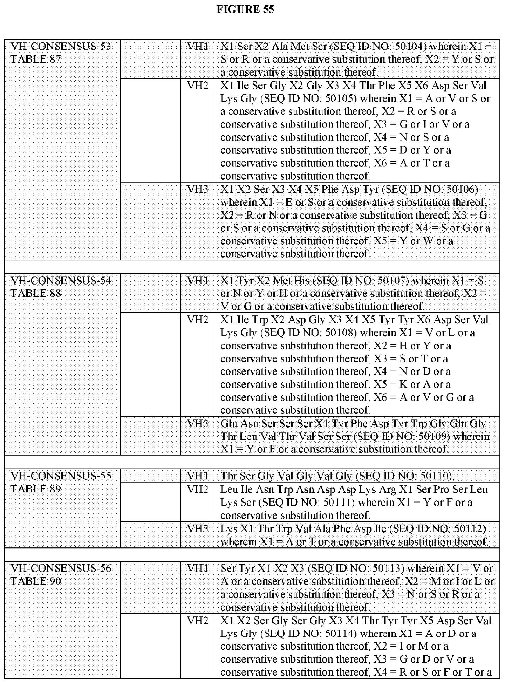

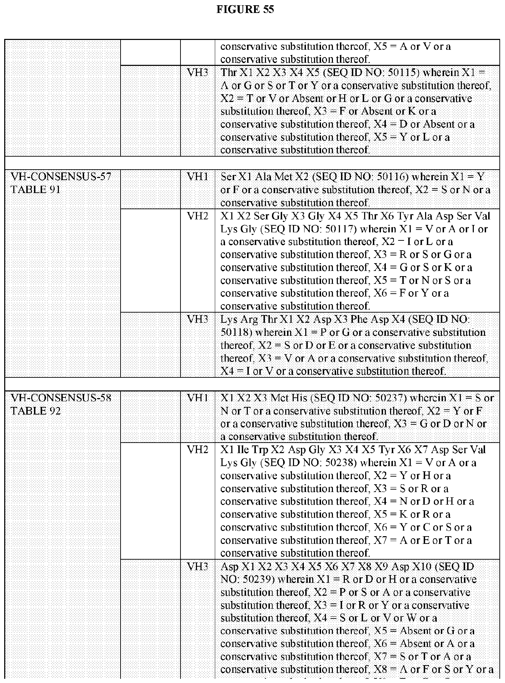

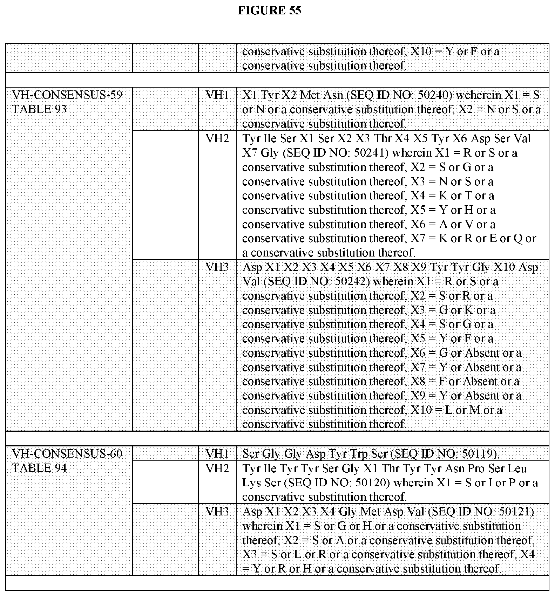

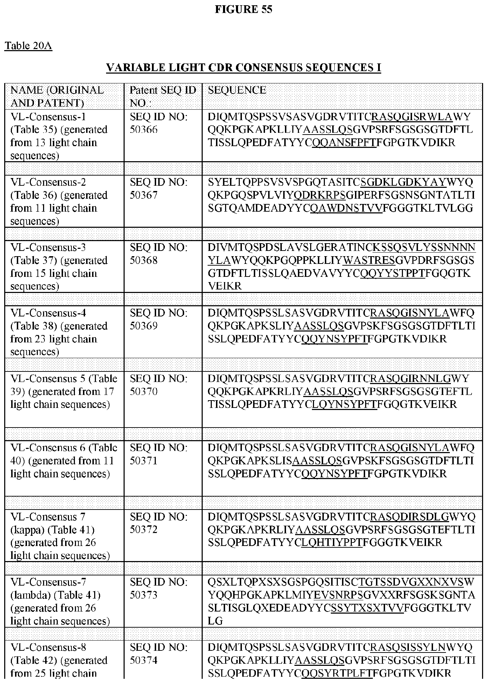

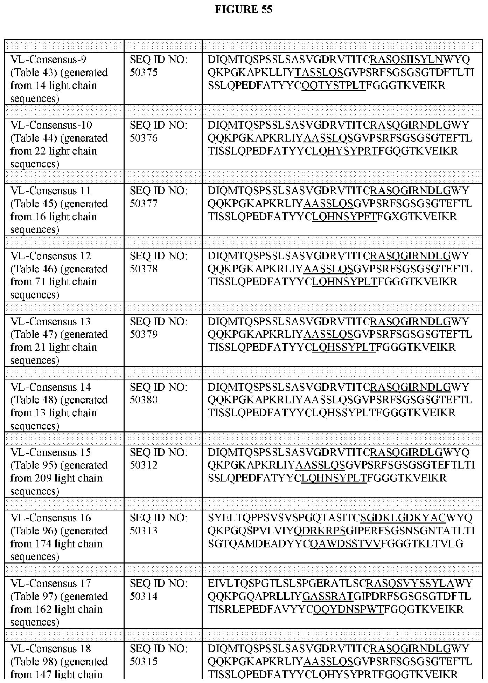

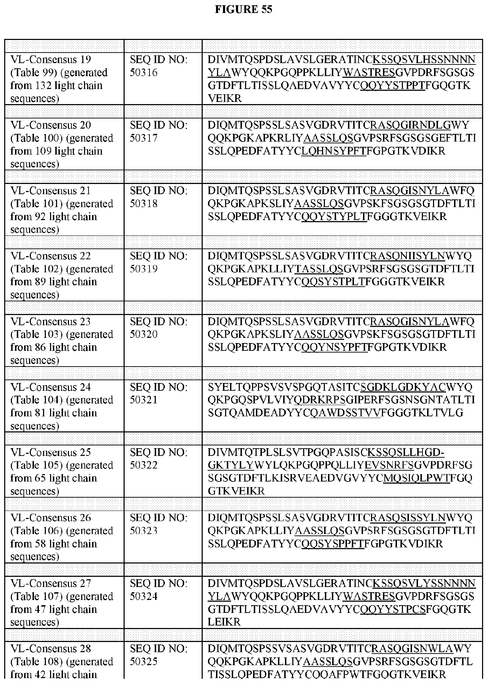

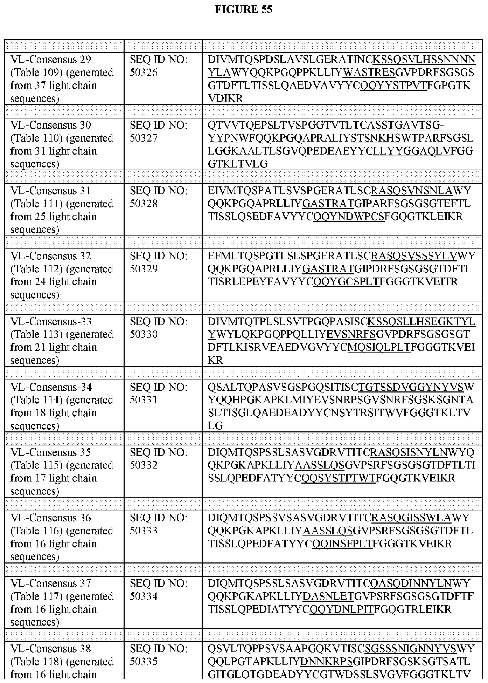

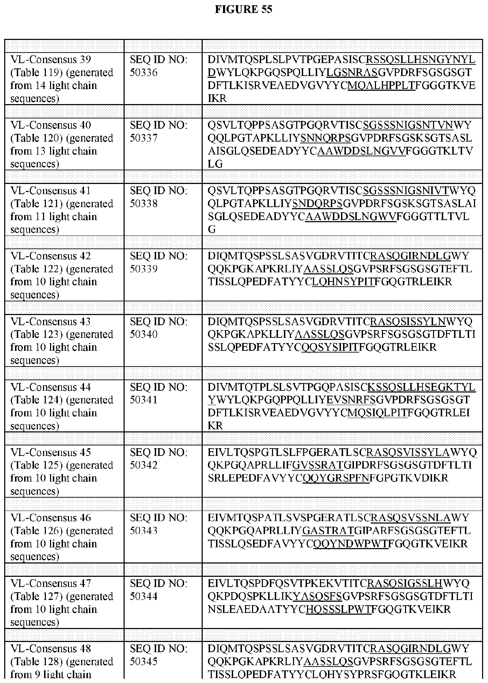

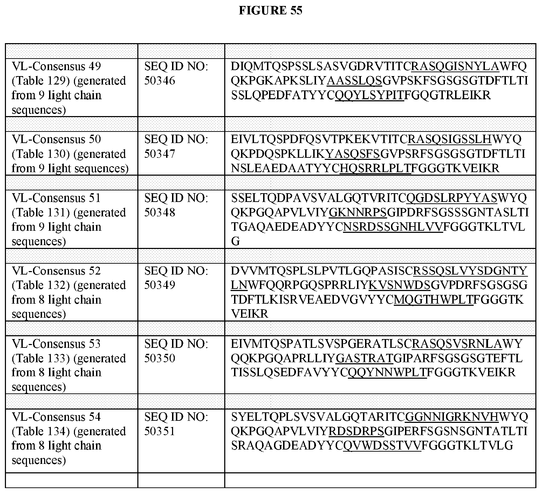

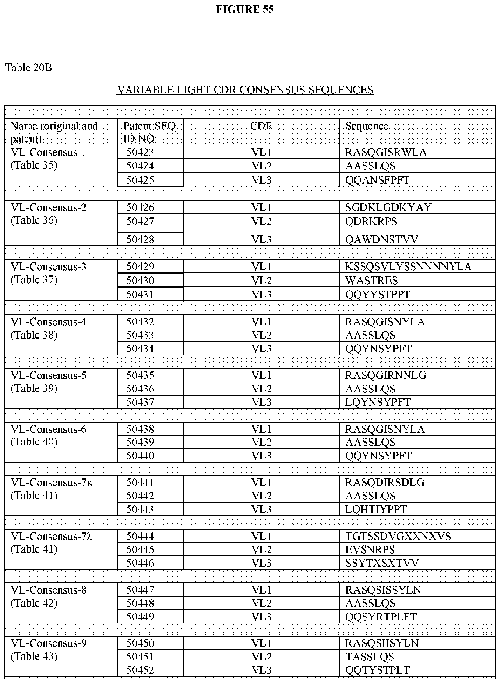

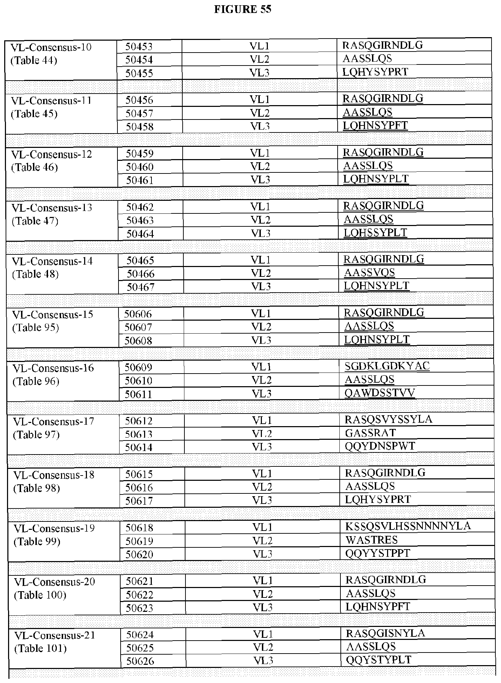

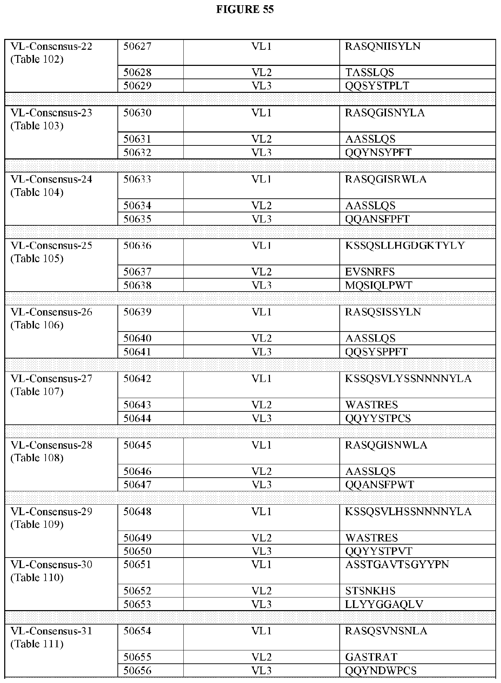

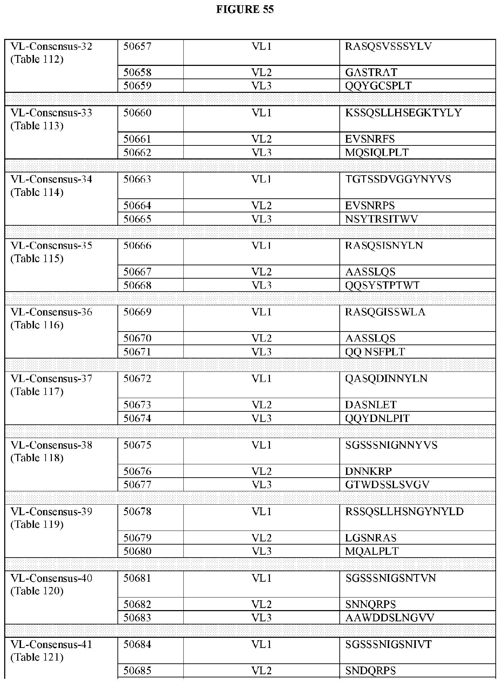

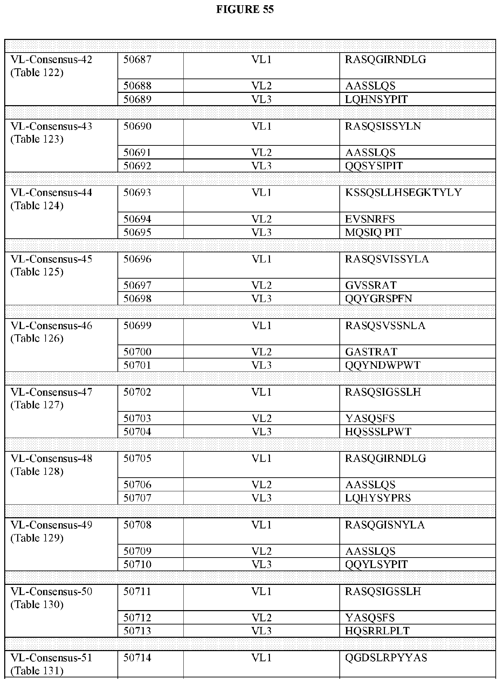

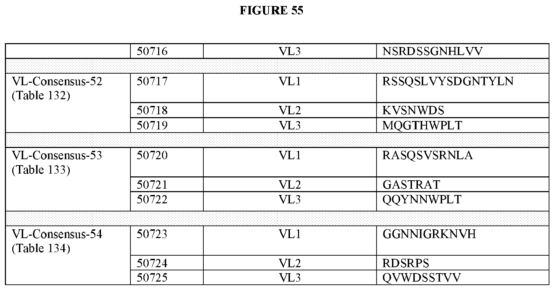

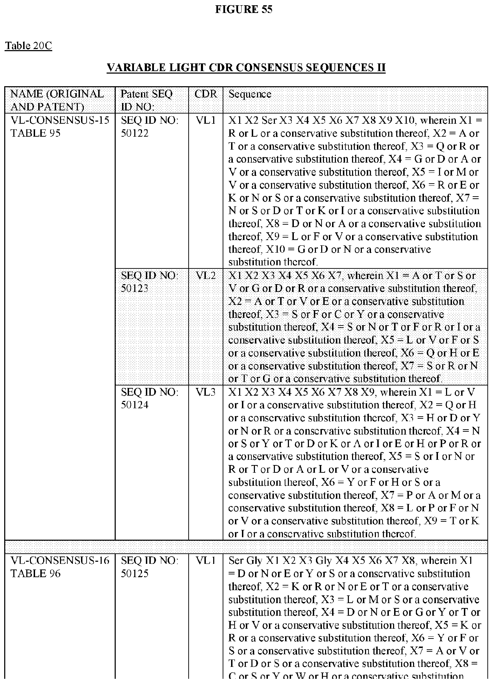

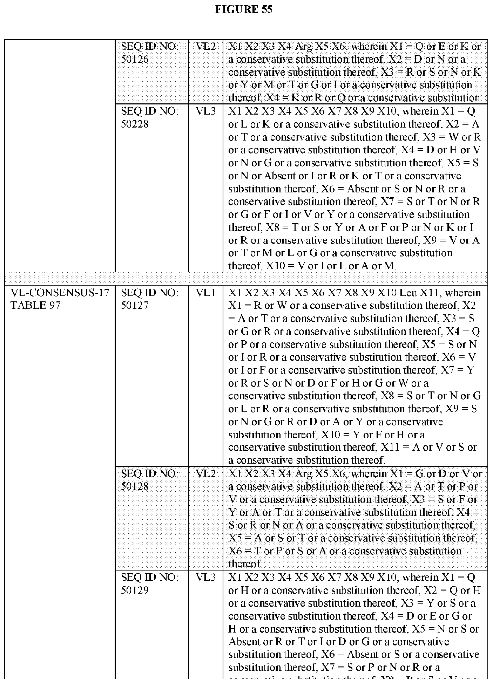

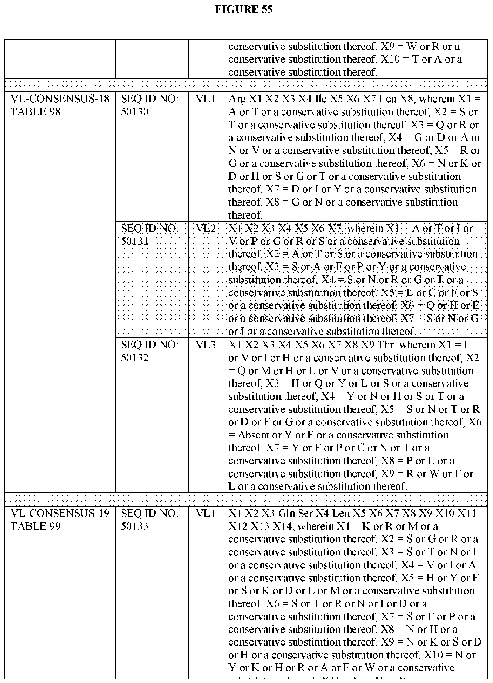

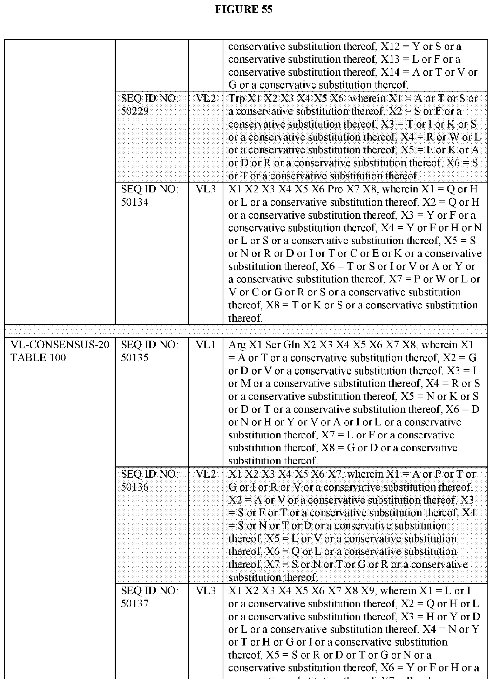

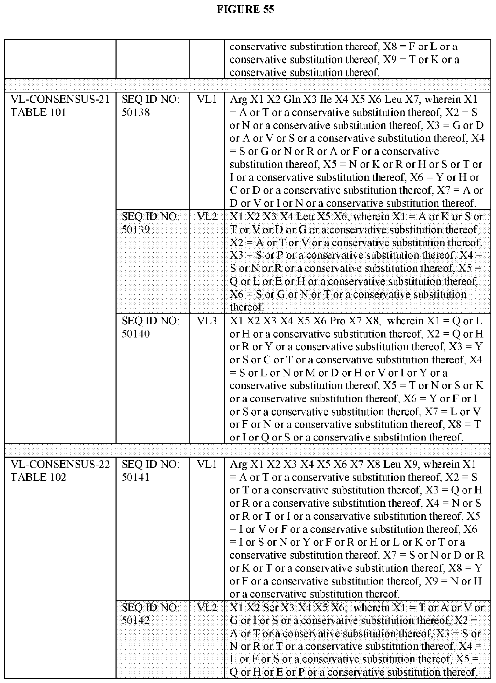

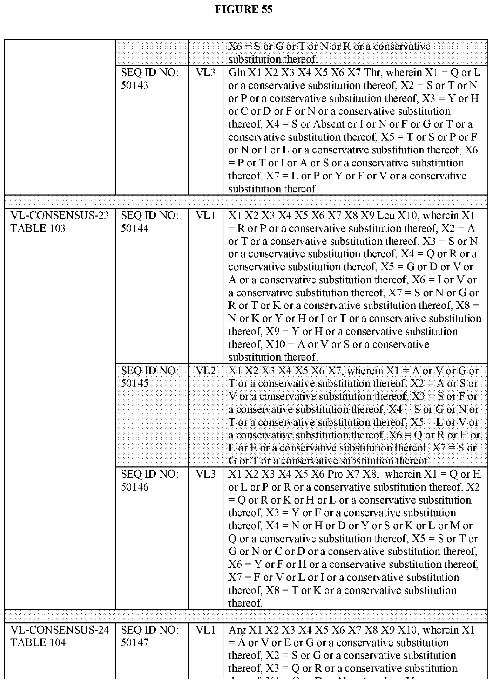

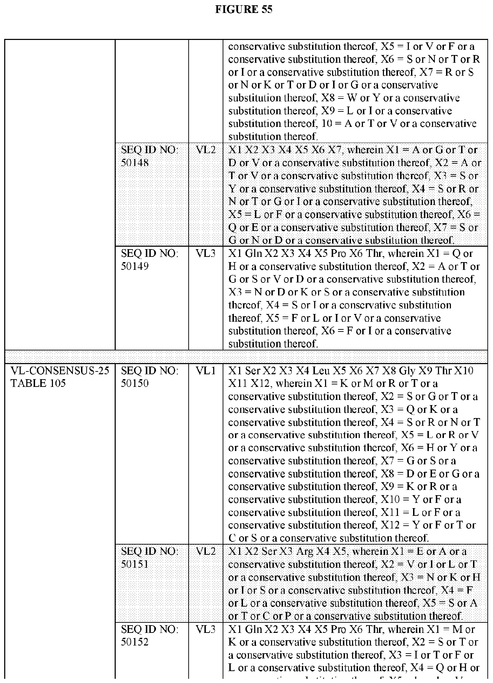

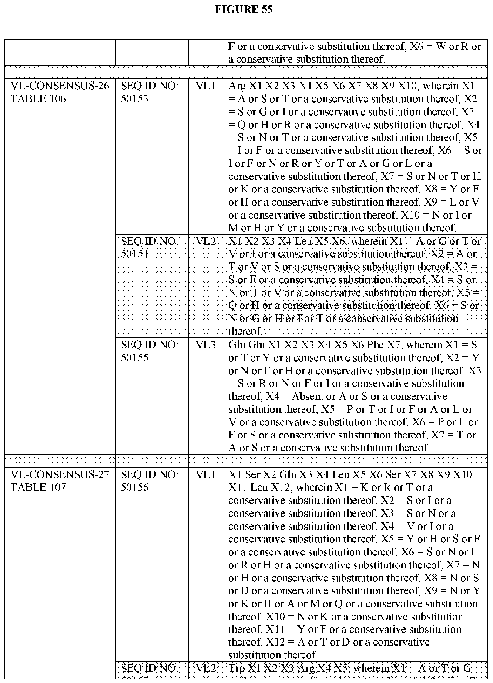

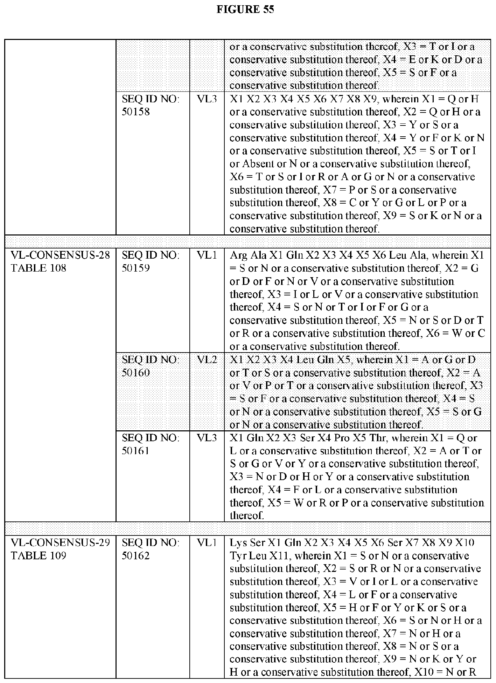

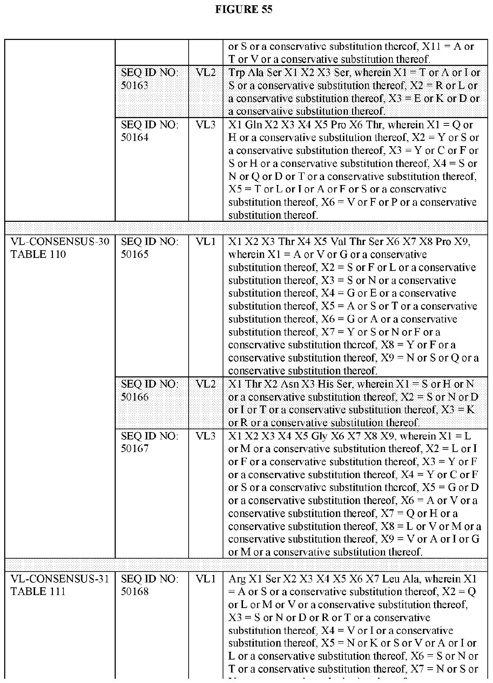

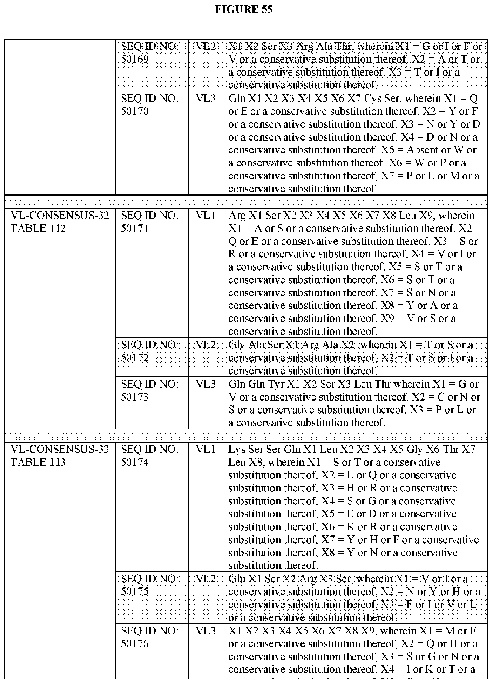

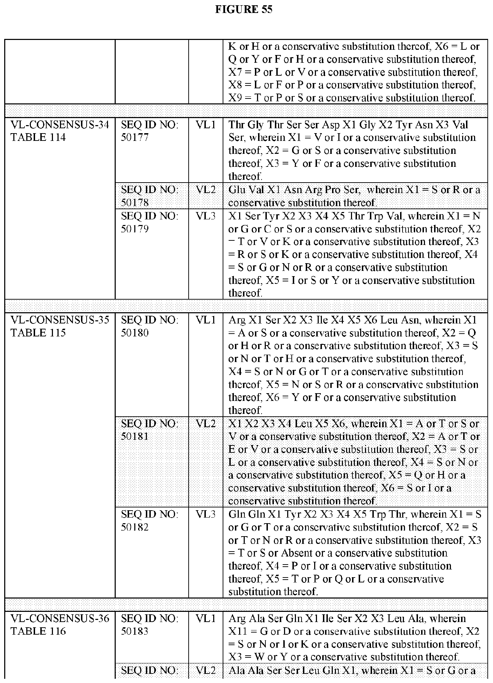

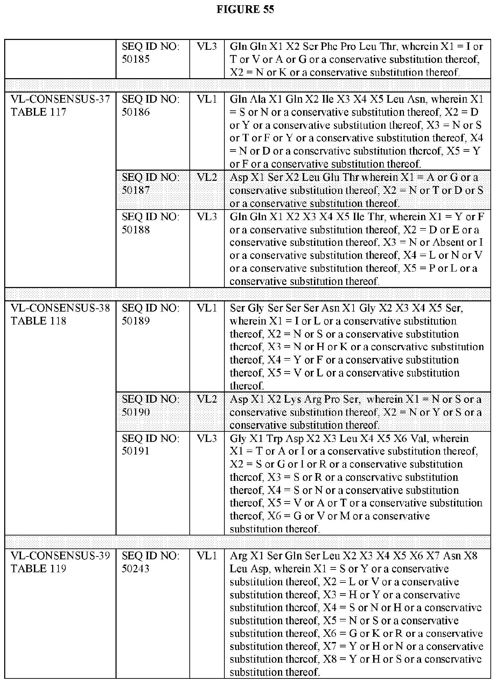

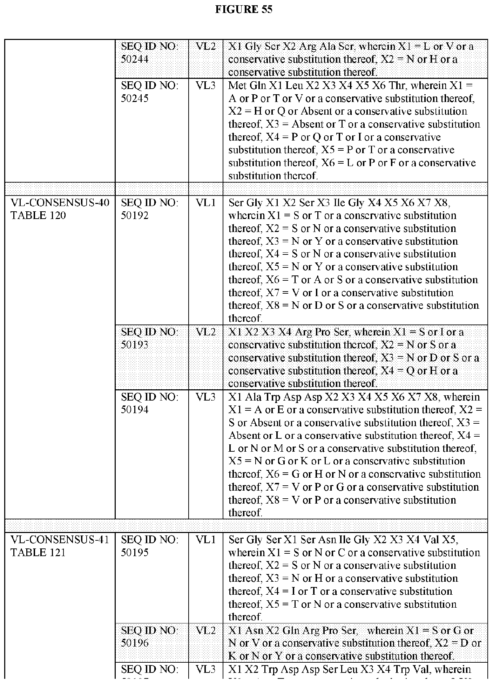

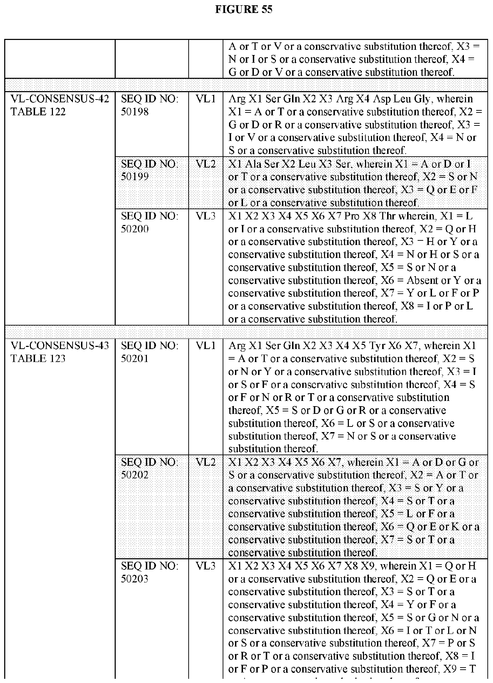

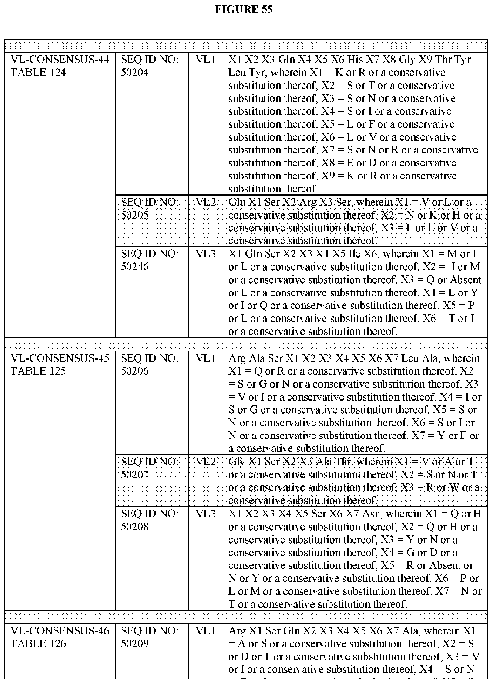

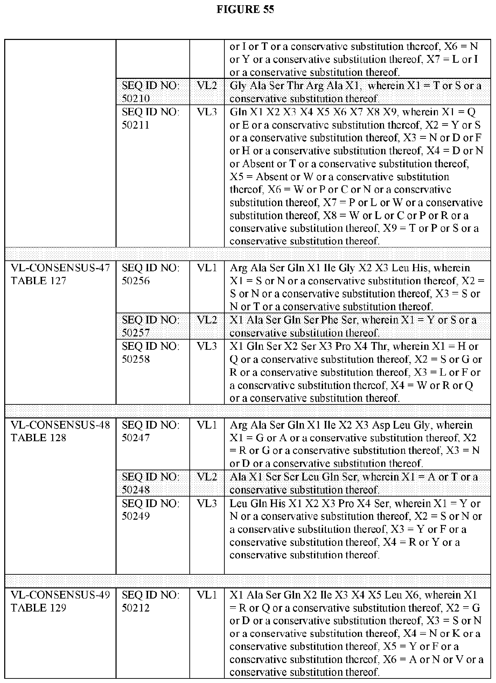

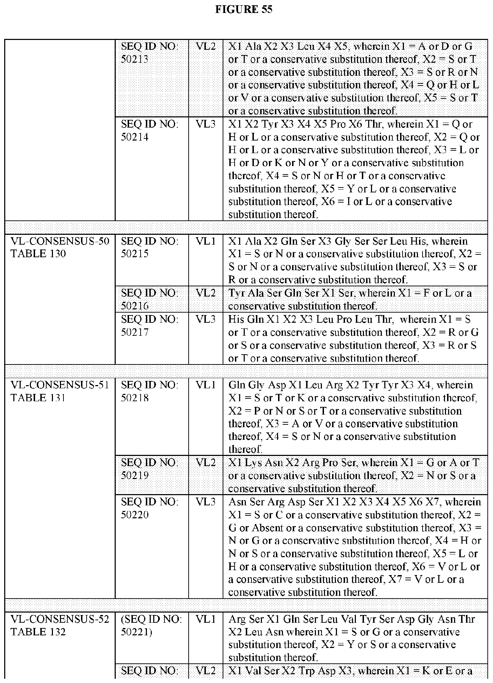

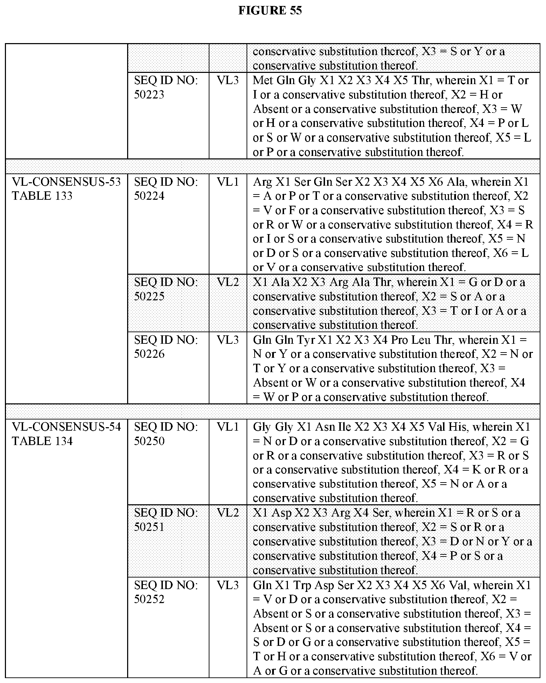

**Figure 55.** A group of tables presenting the consensus sequences of various heavy and light chain variable regions (Tables 19A and 20A, respectively), as well as the consensus sequences of CDRs of various heavy and light chain variable regions (Tables 19B and C and Tables 20B and 20C, respectively) for certain antigen binding proteins.

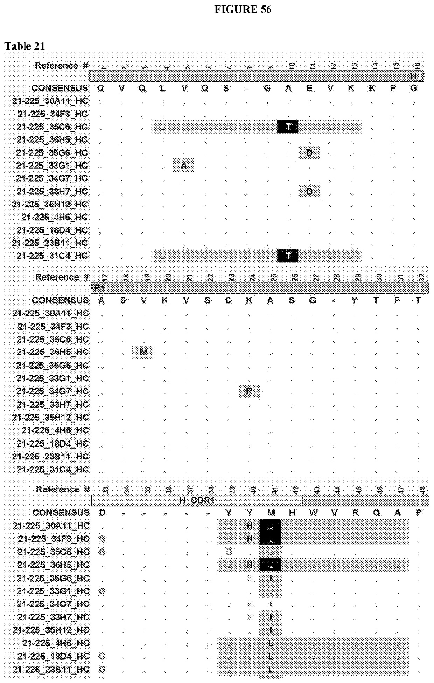

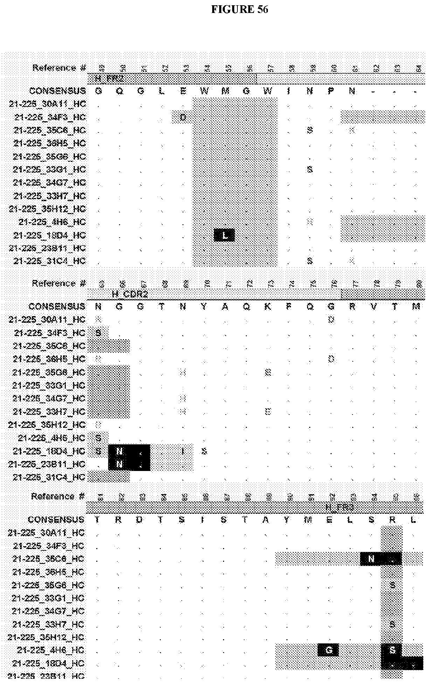

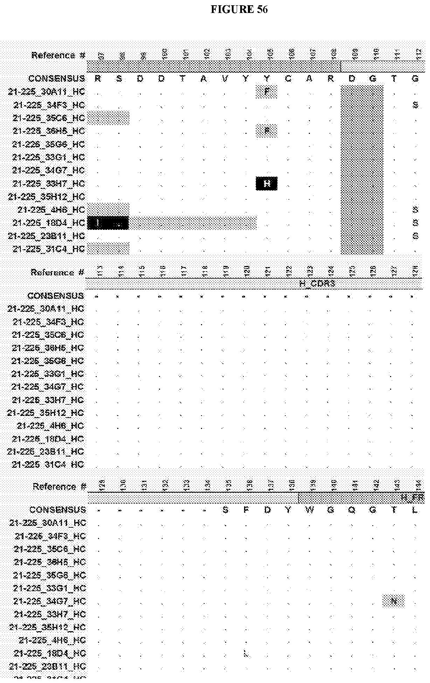

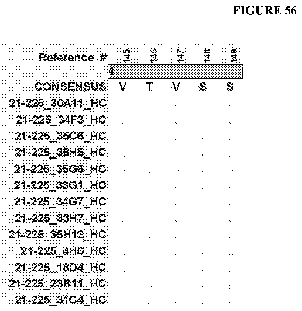

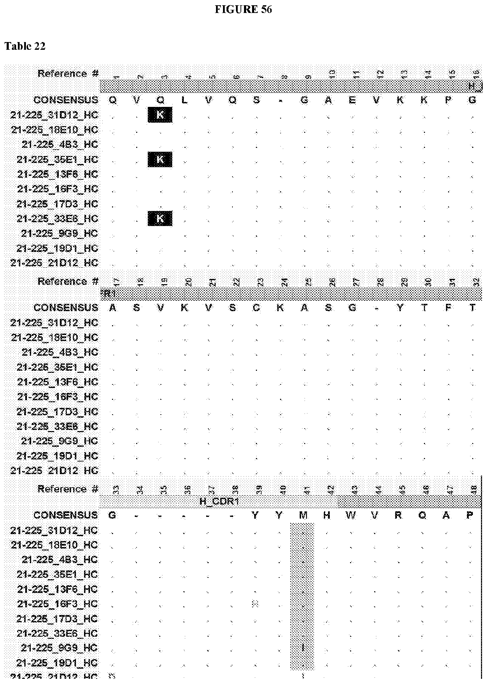

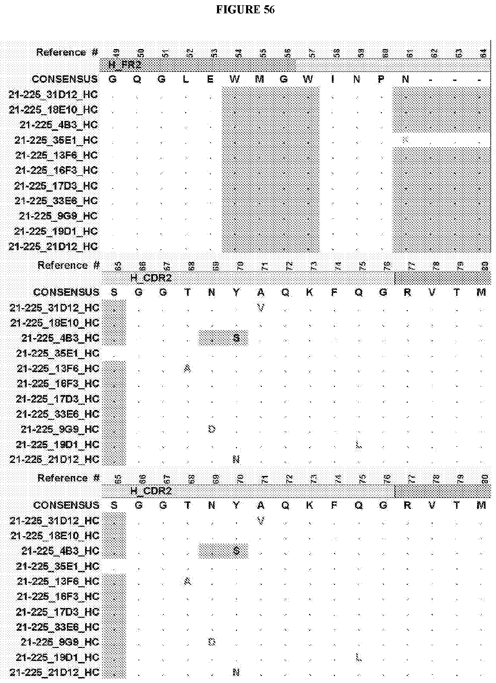

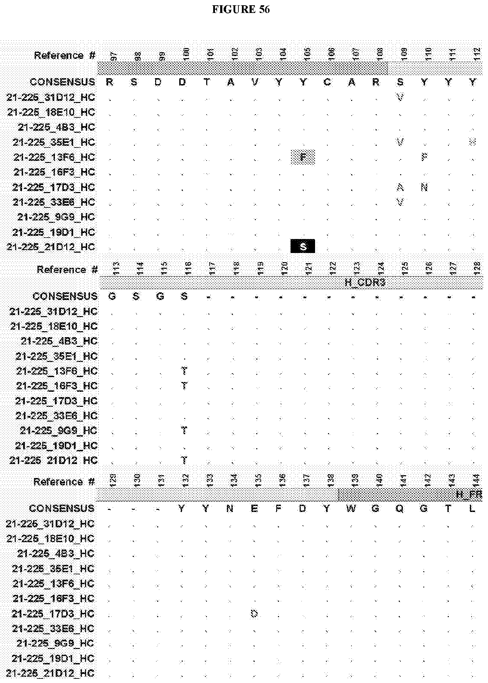

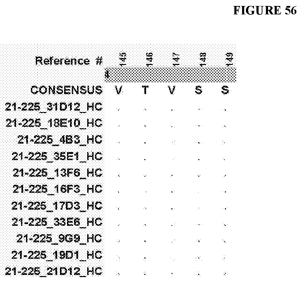

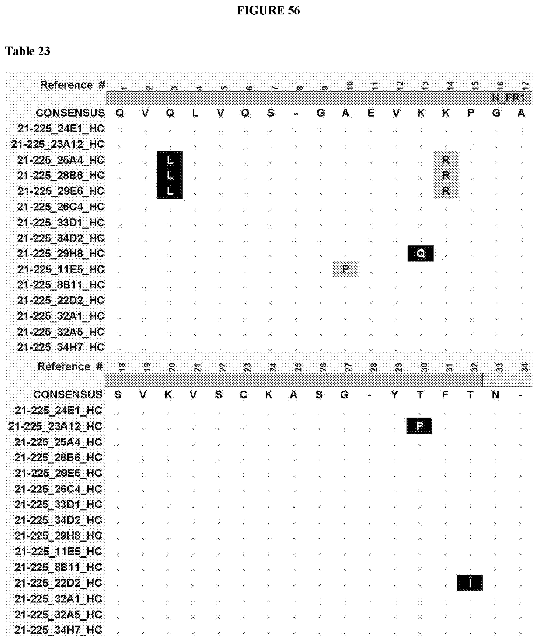

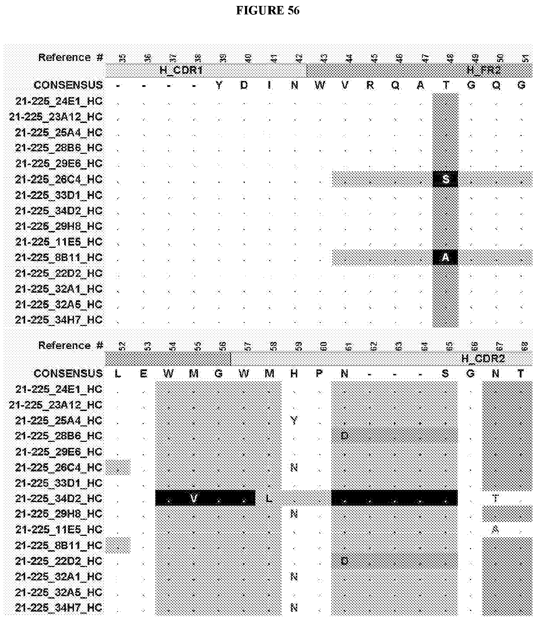

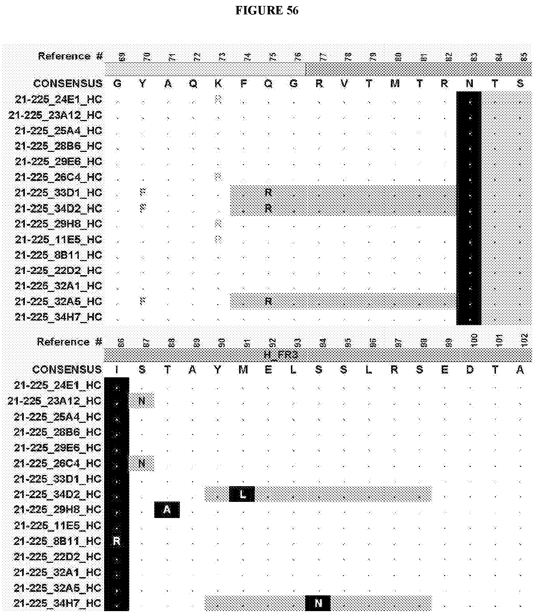

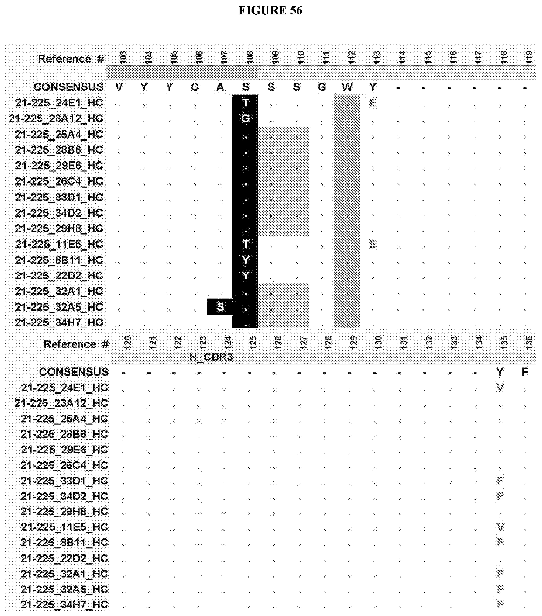

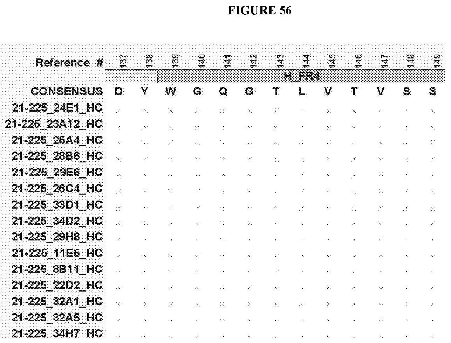

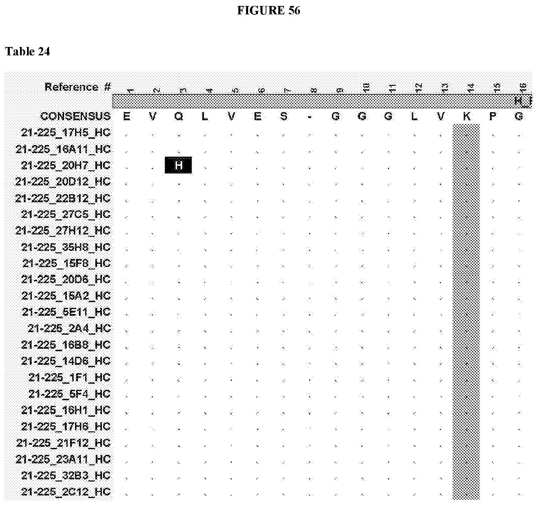

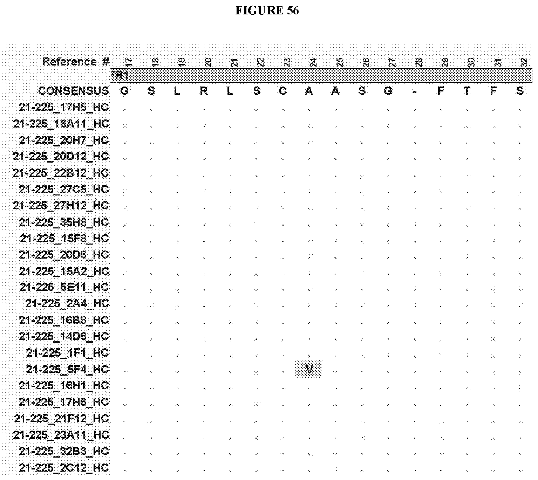

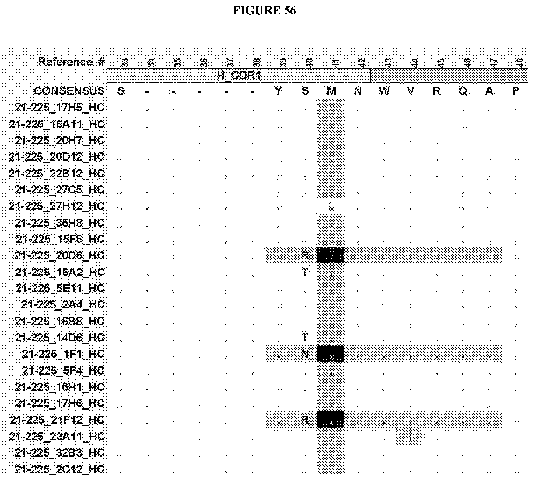

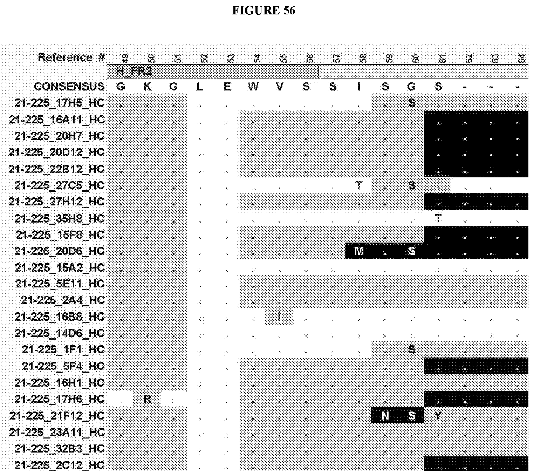

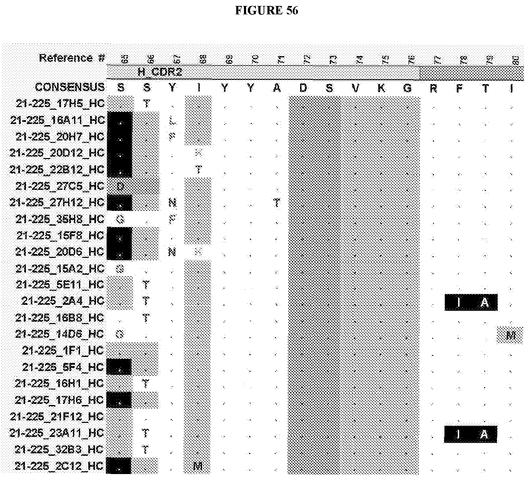

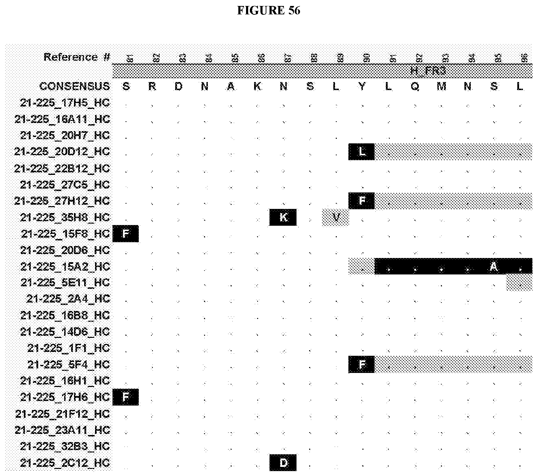

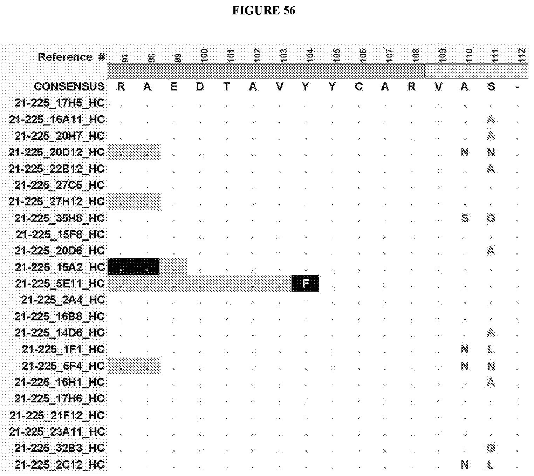

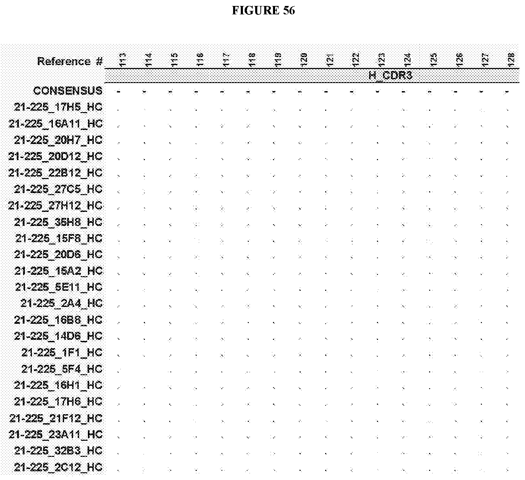

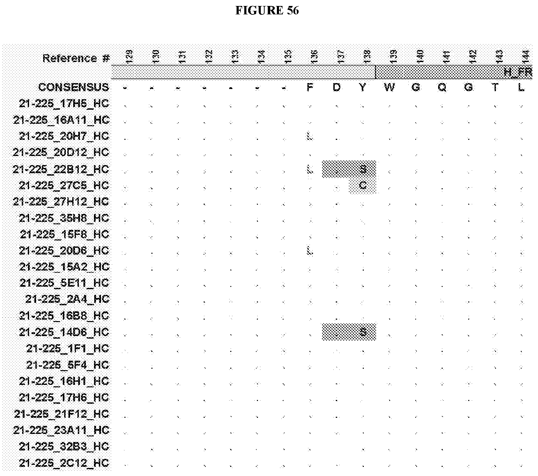

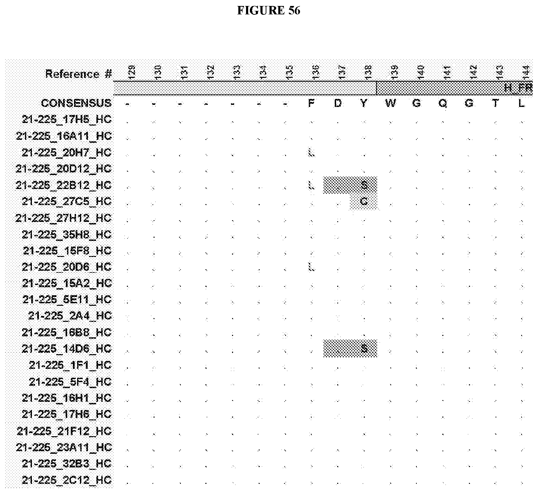

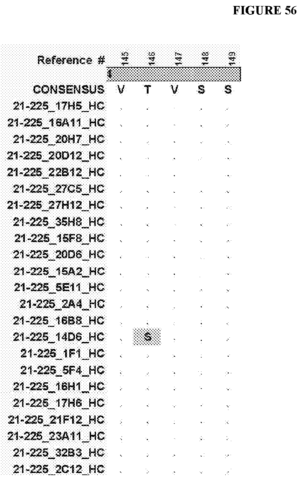

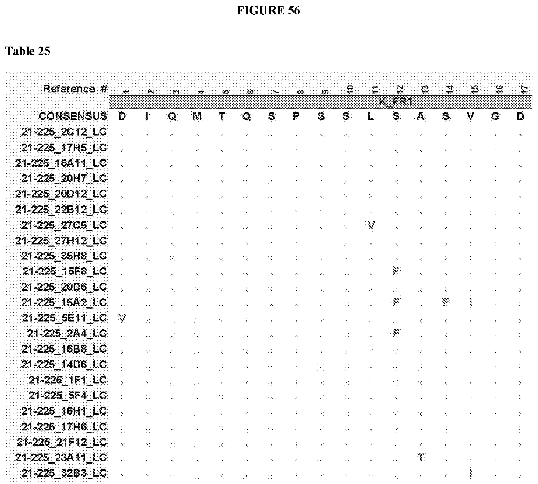

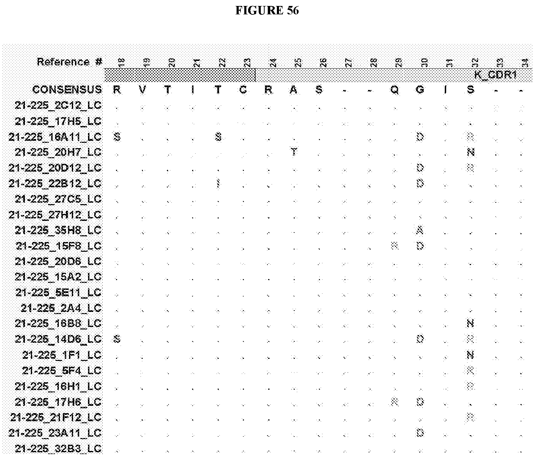

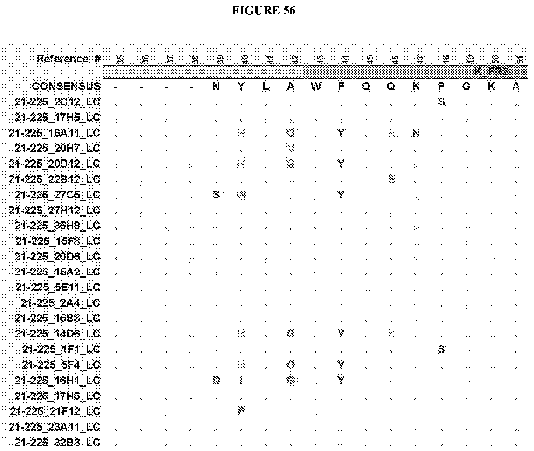

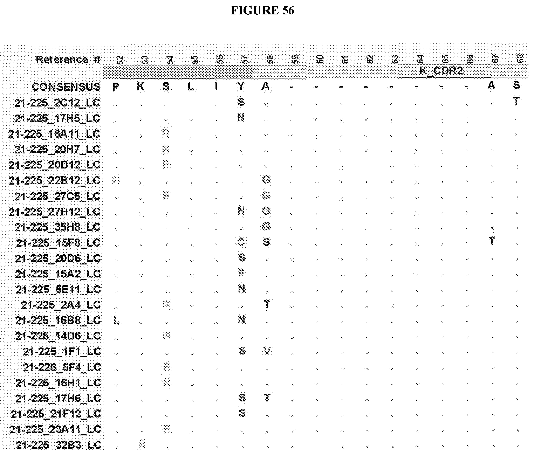

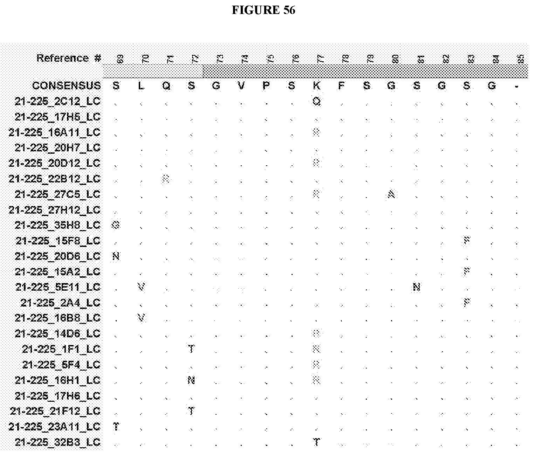

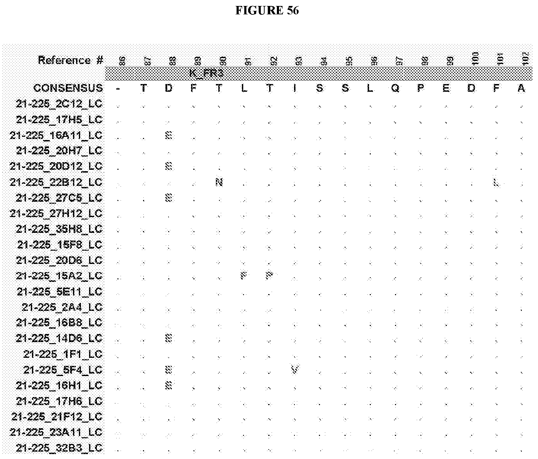

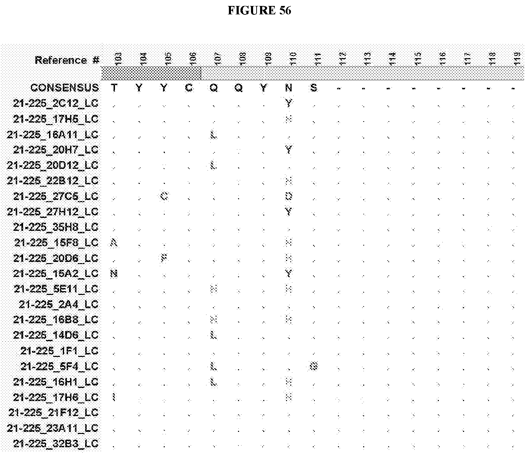

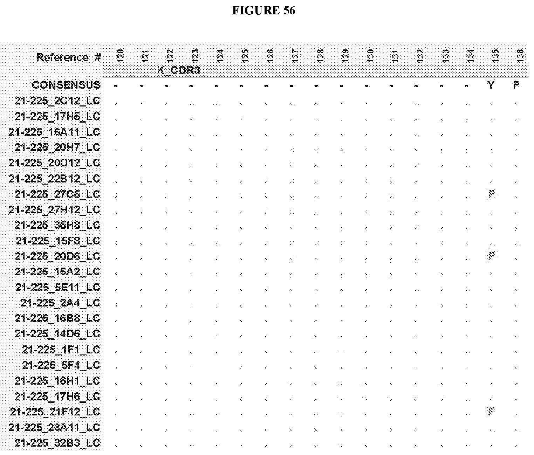

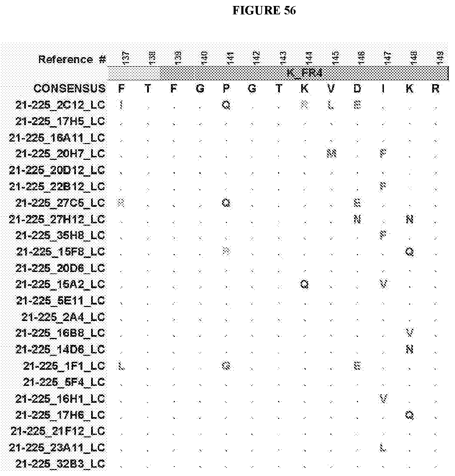

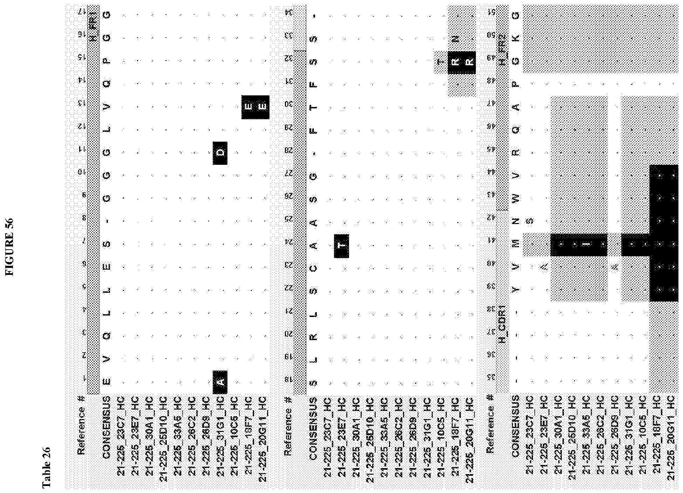

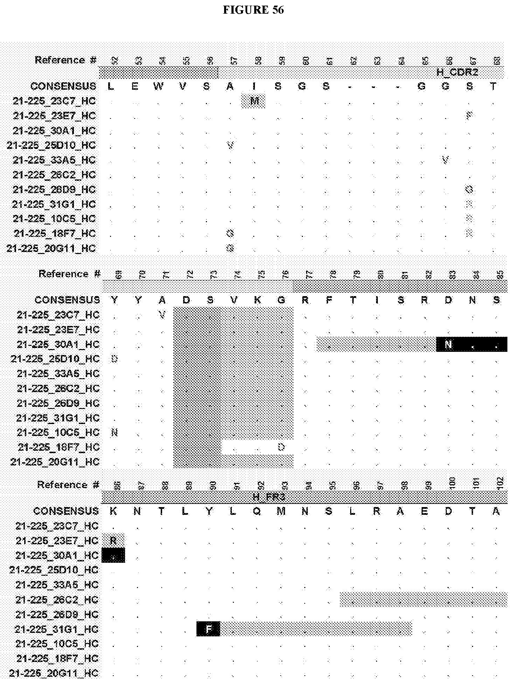

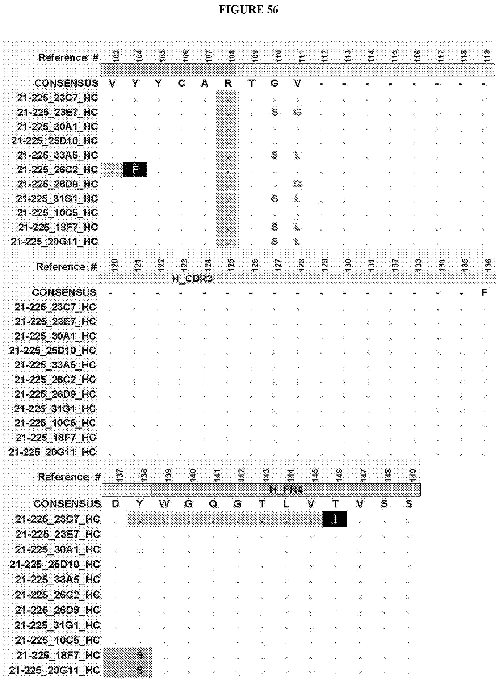

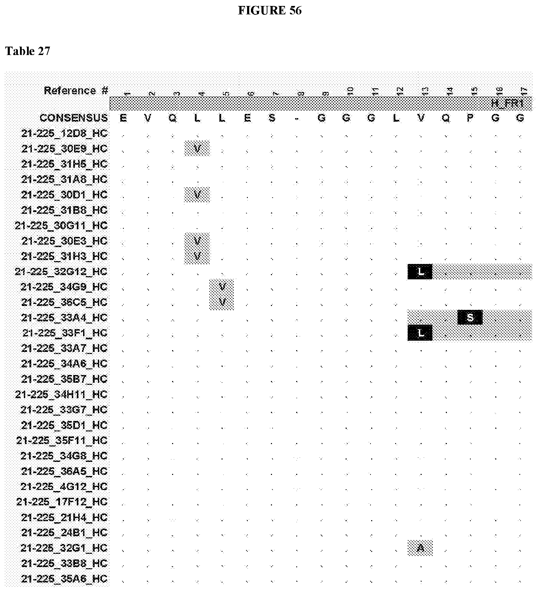

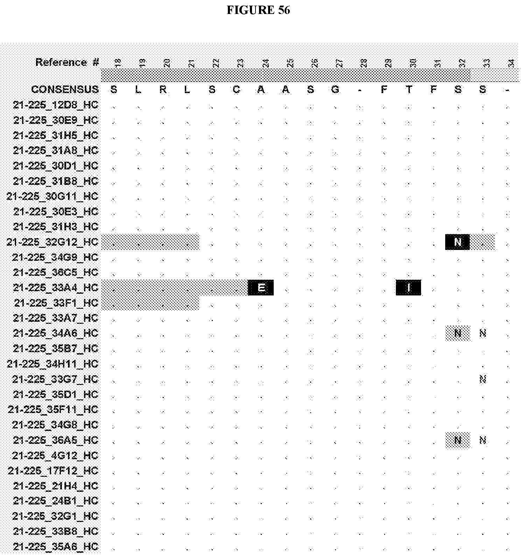

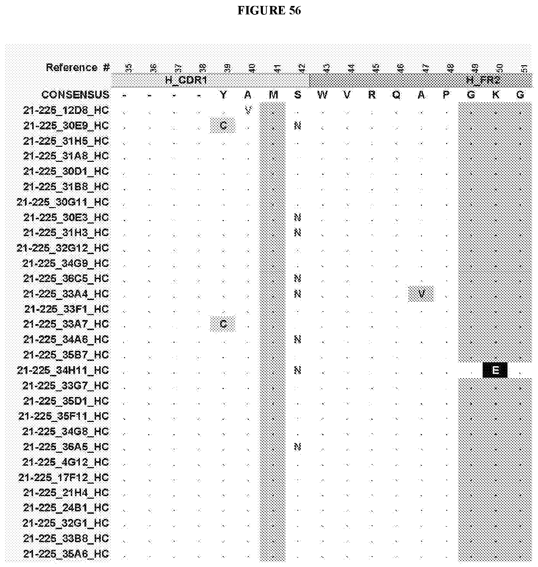

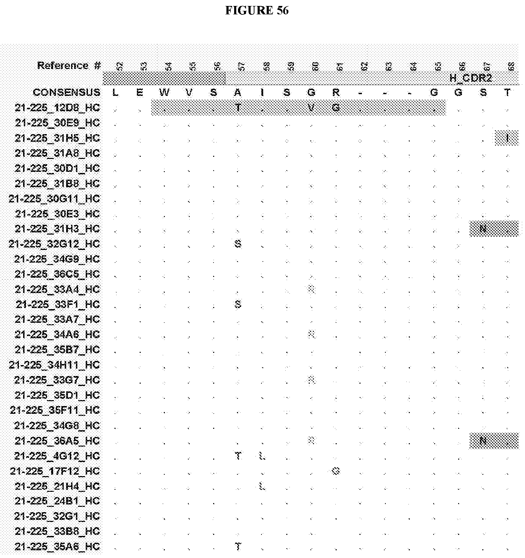

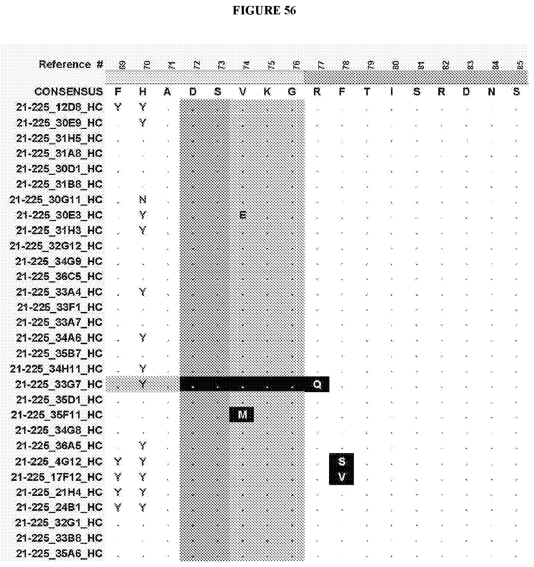

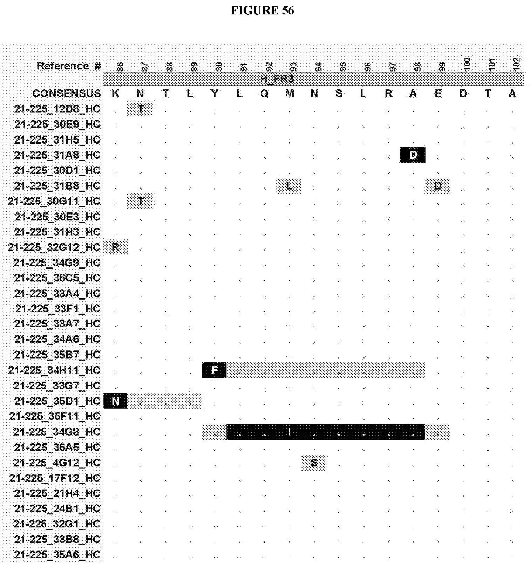

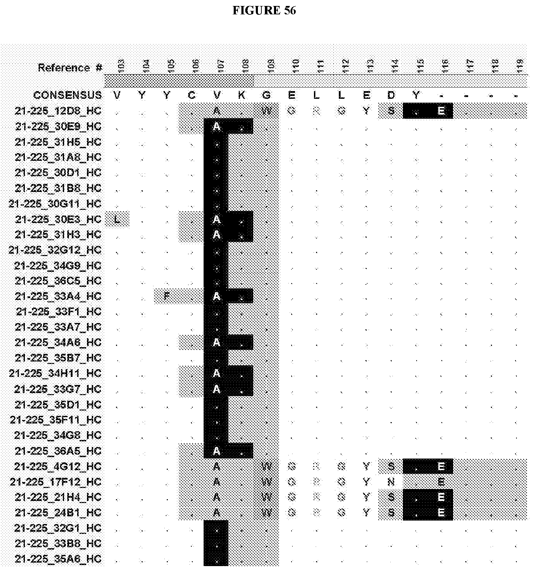

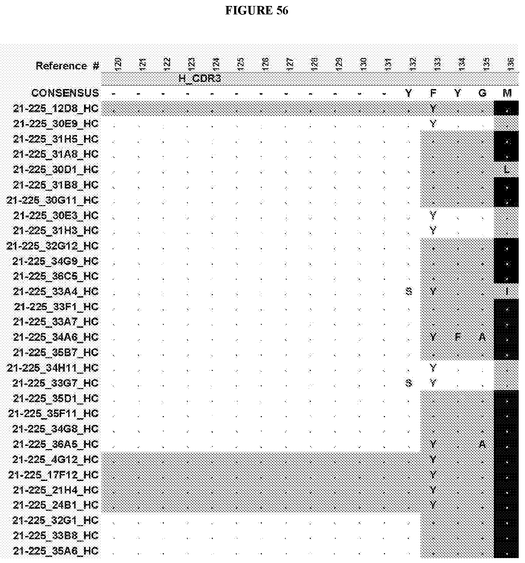

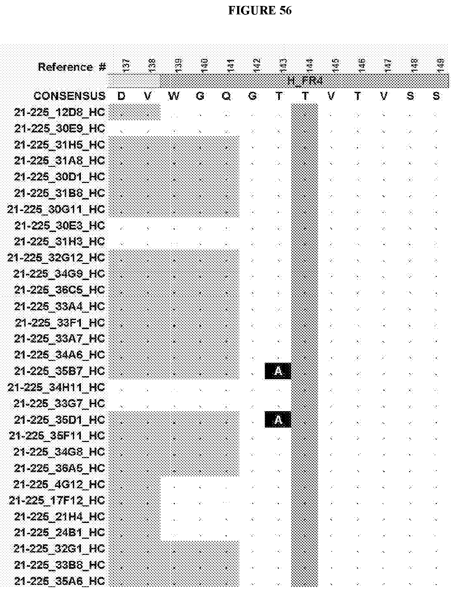

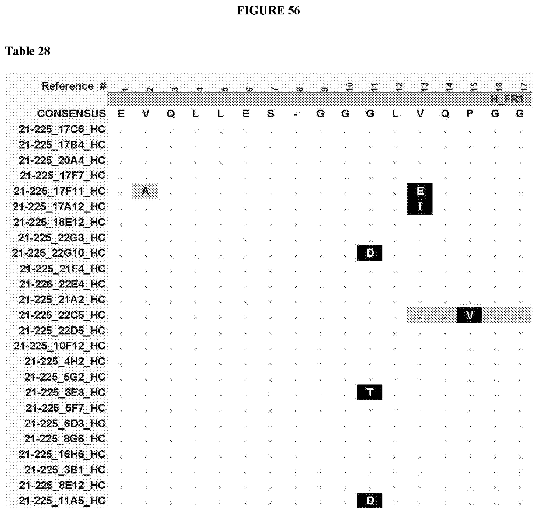

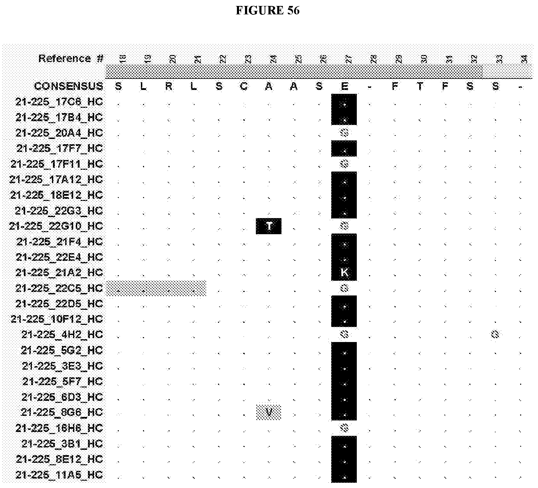

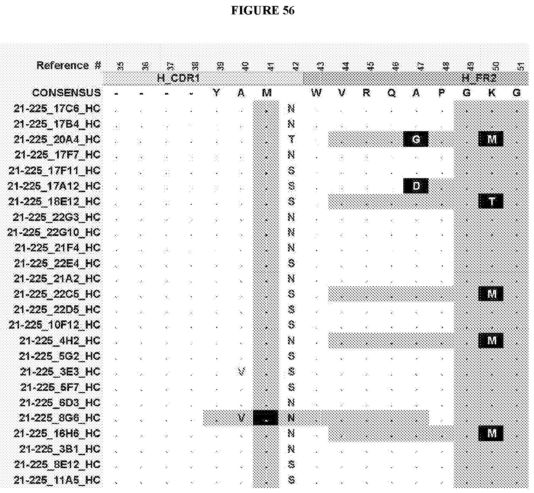

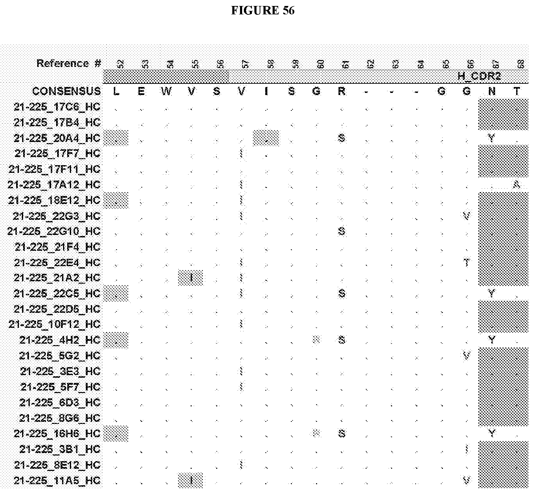

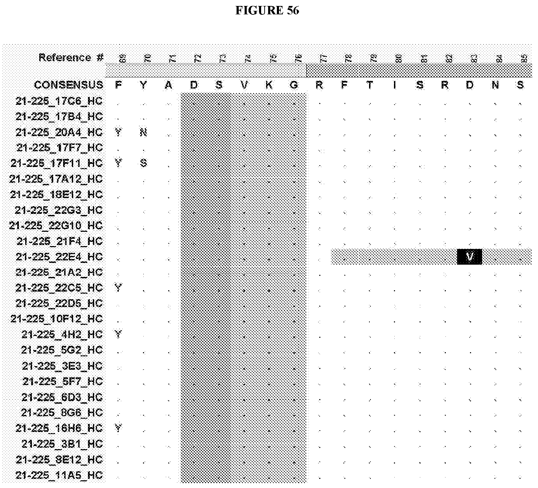

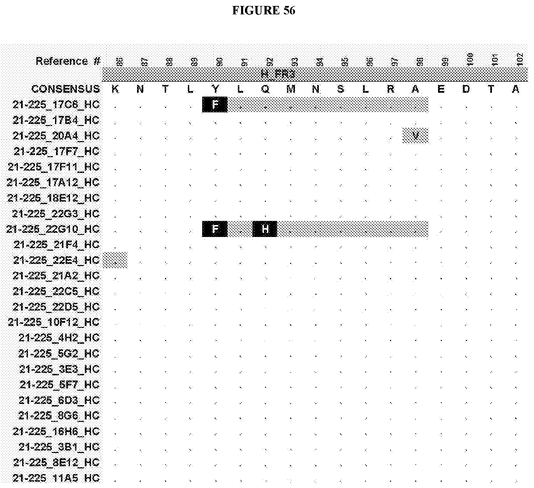

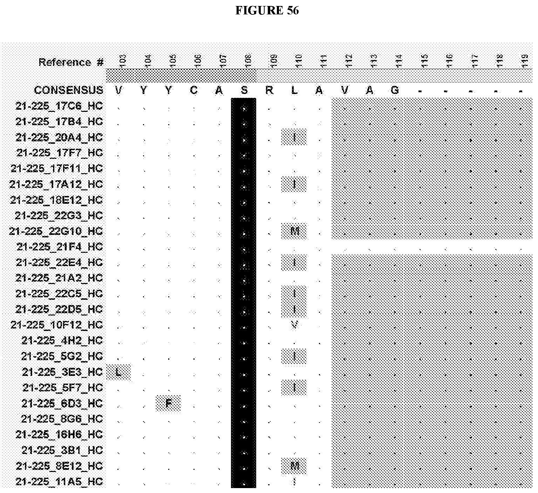

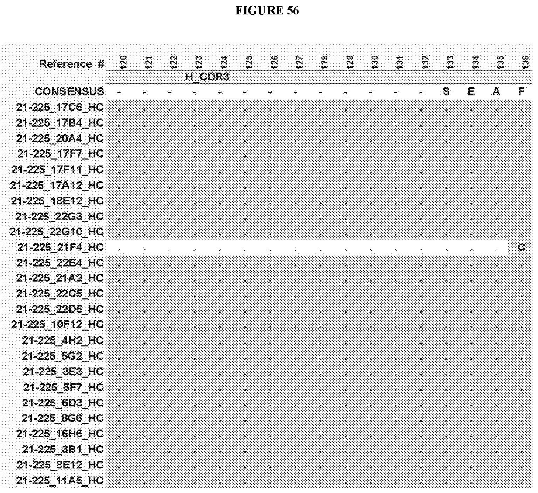

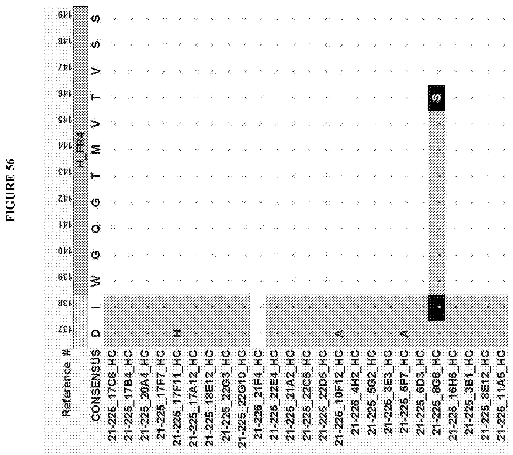

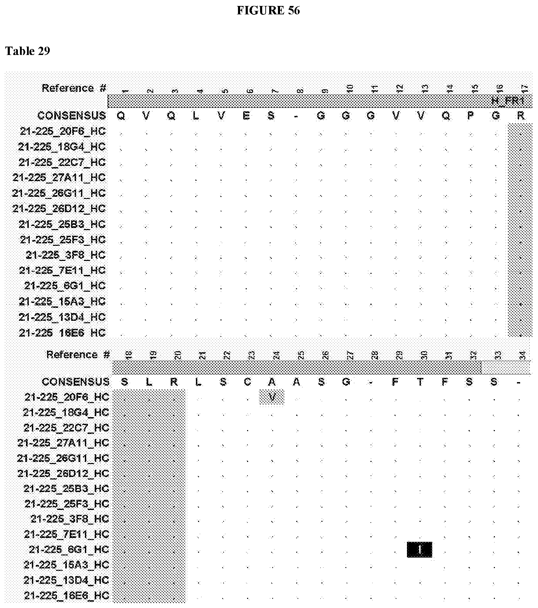

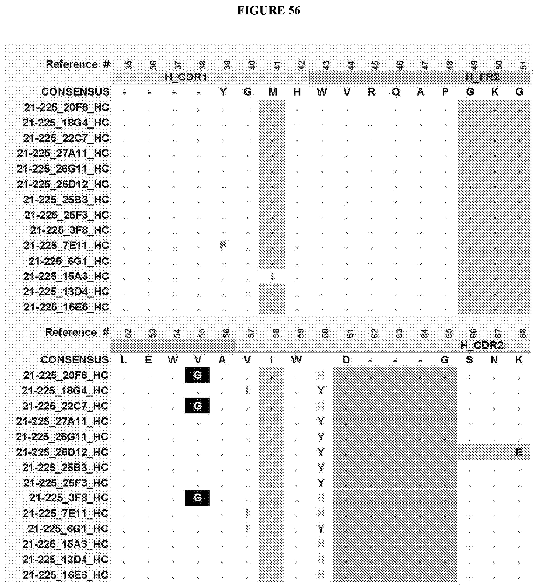

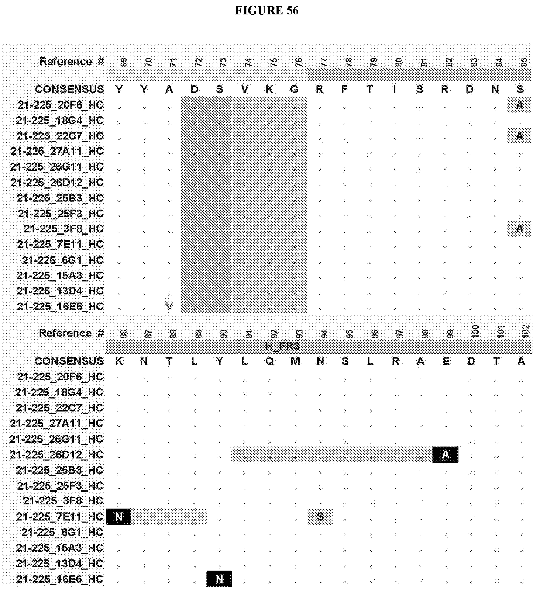

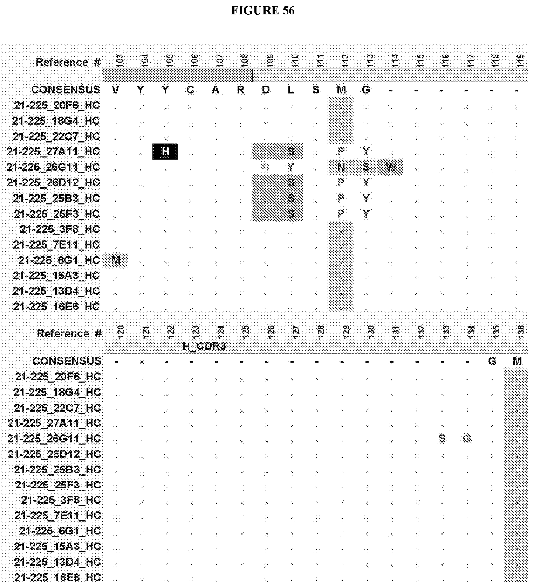

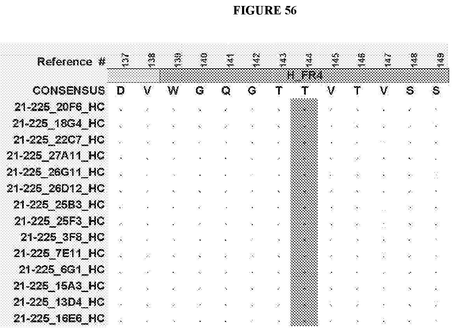

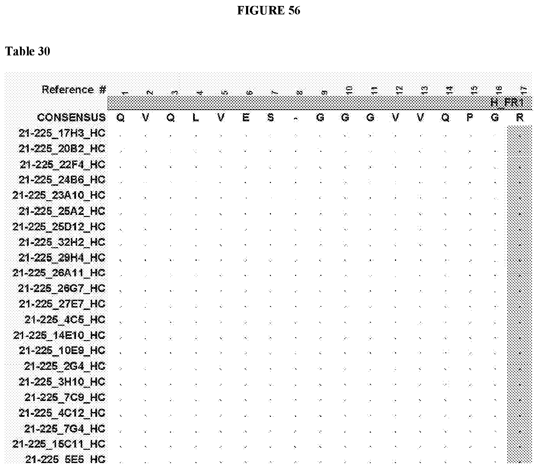

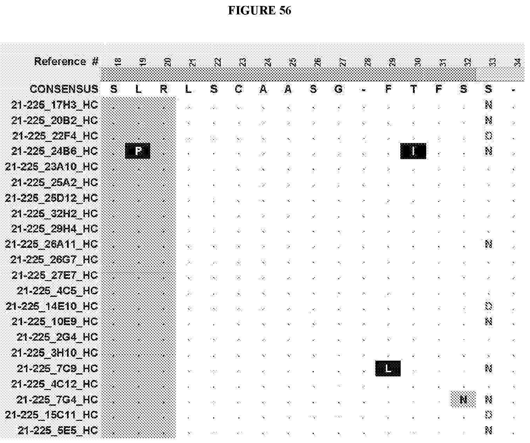

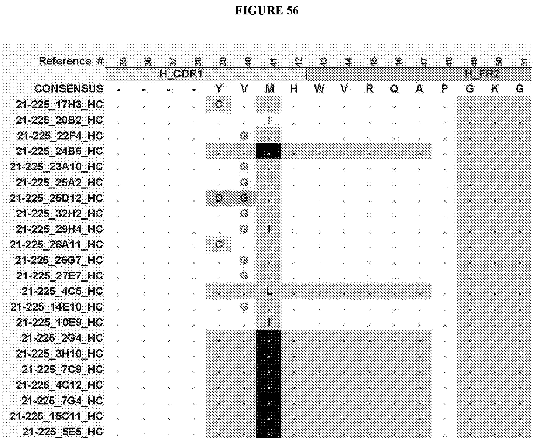

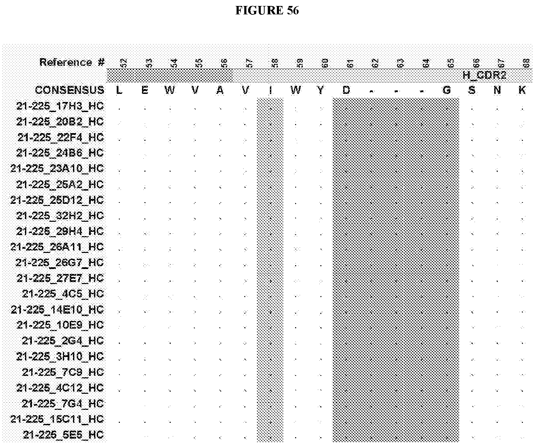

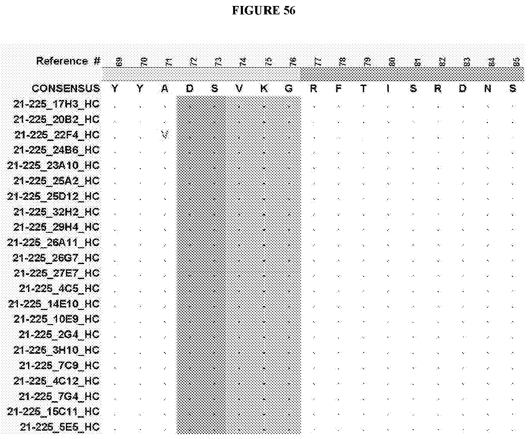

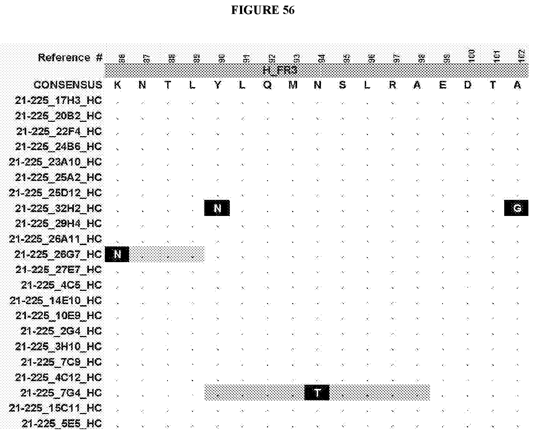

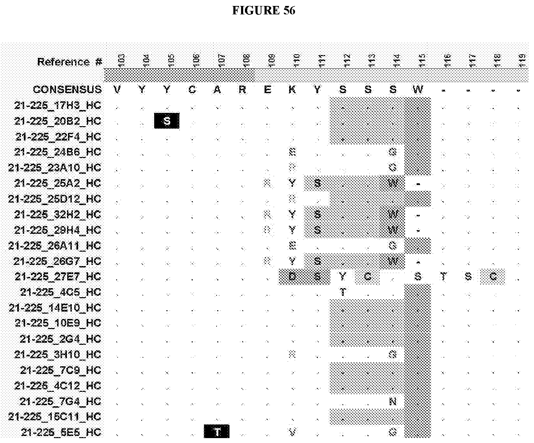

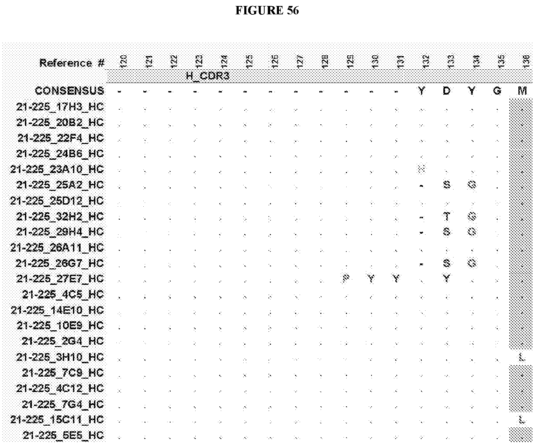

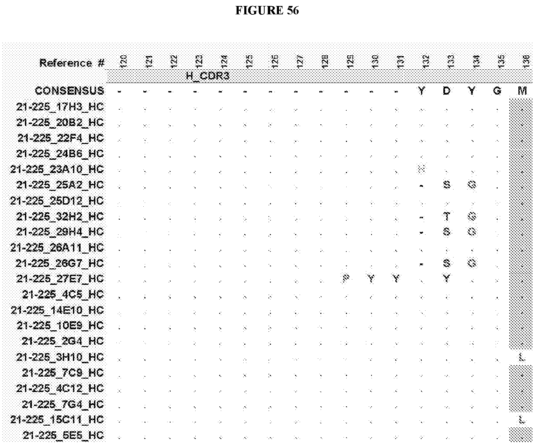

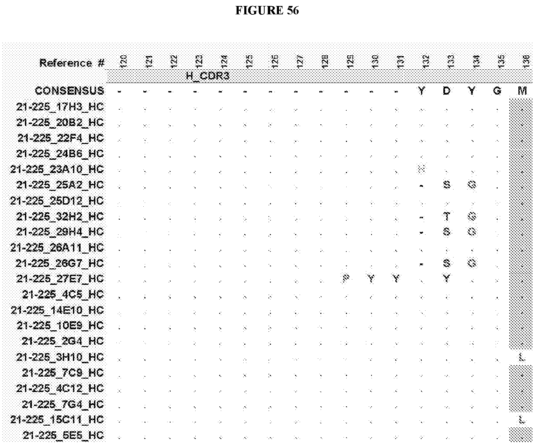

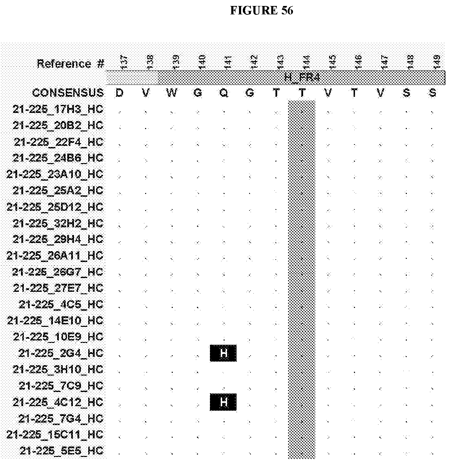

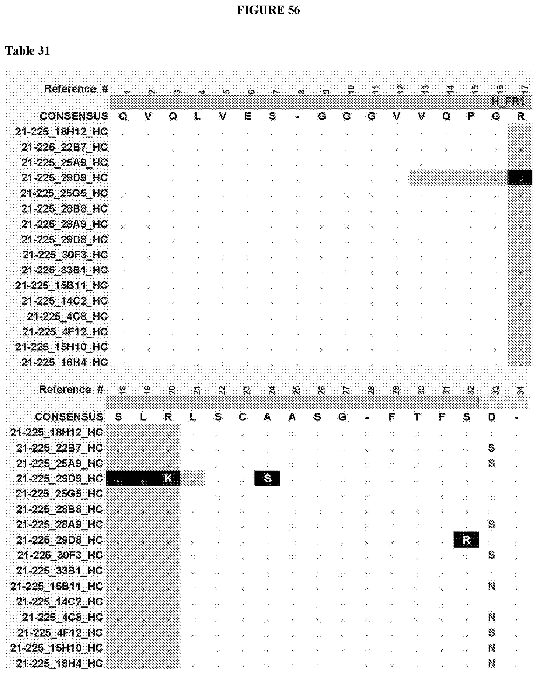

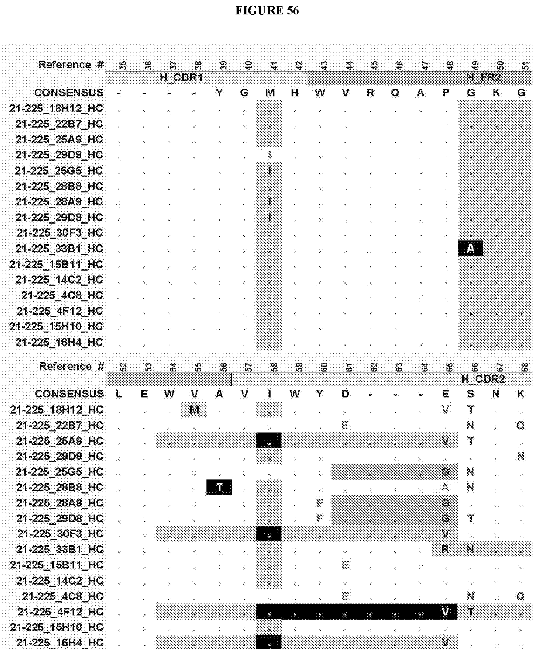

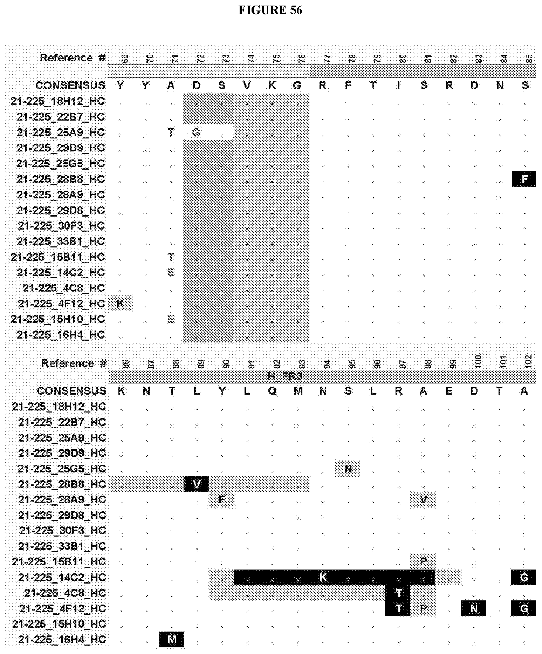

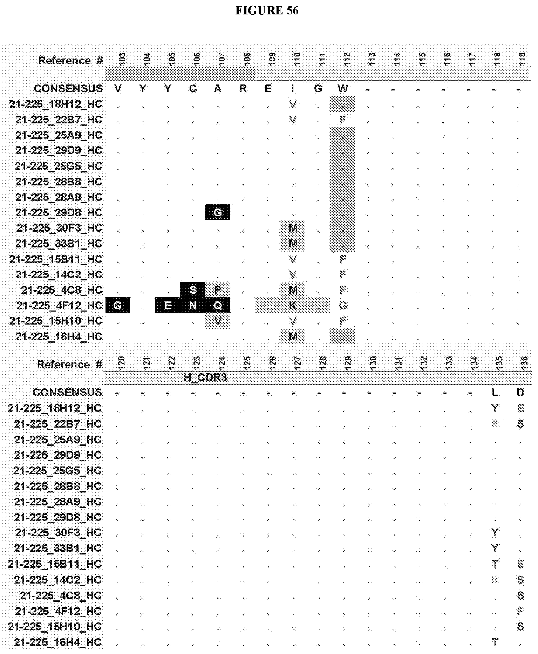

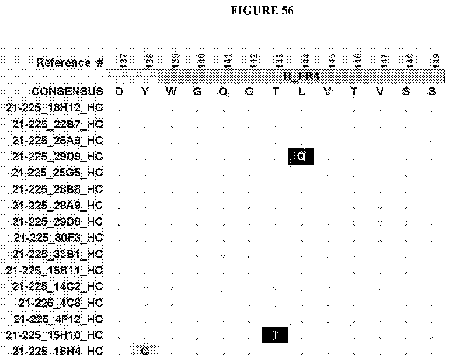

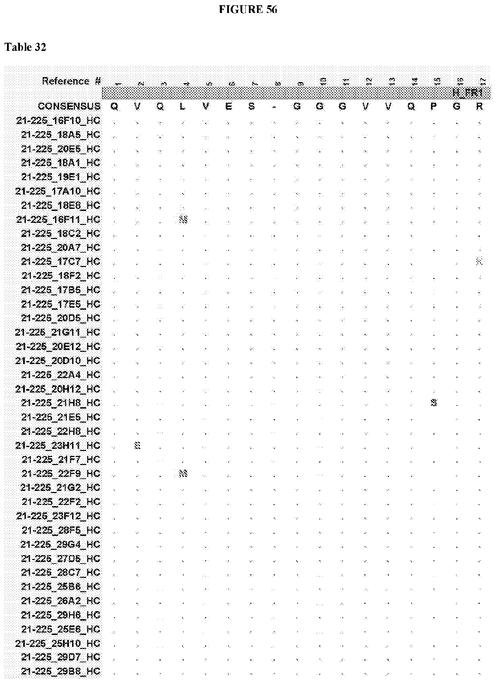

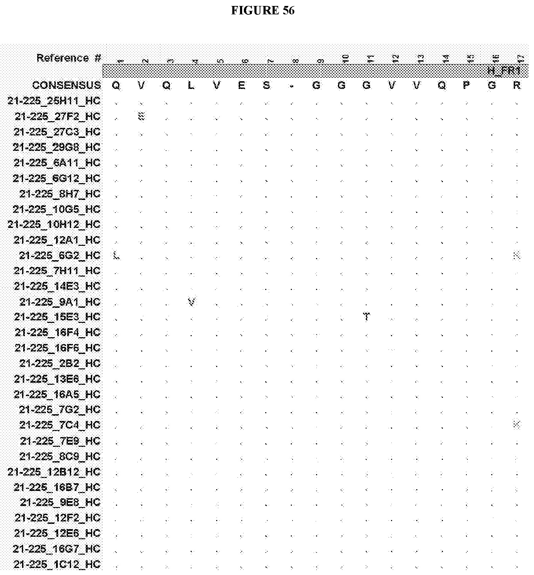

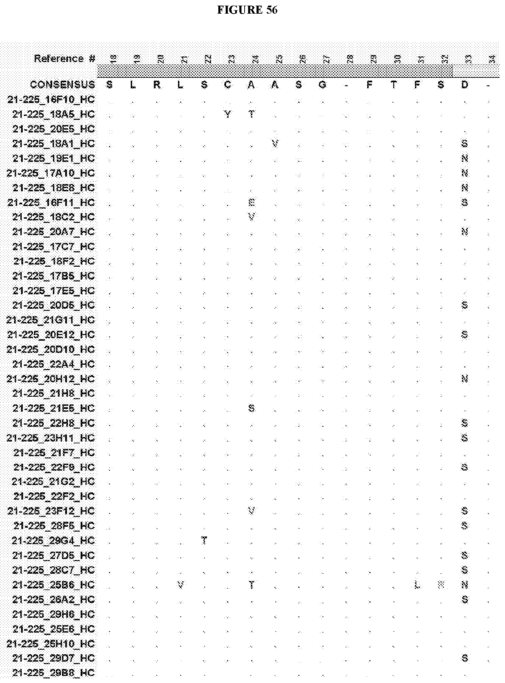

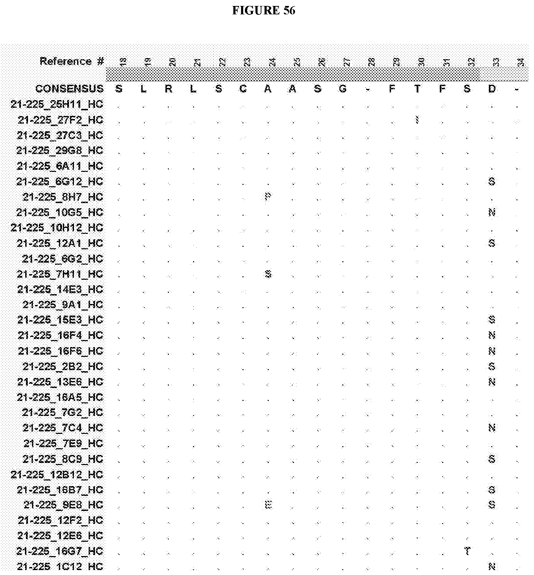

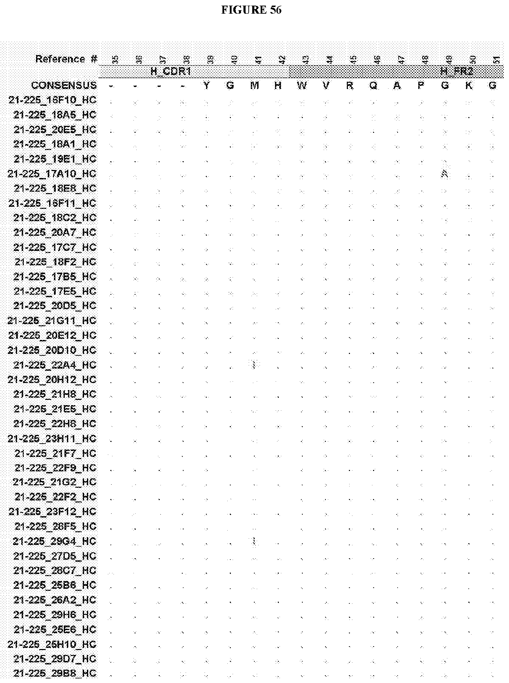

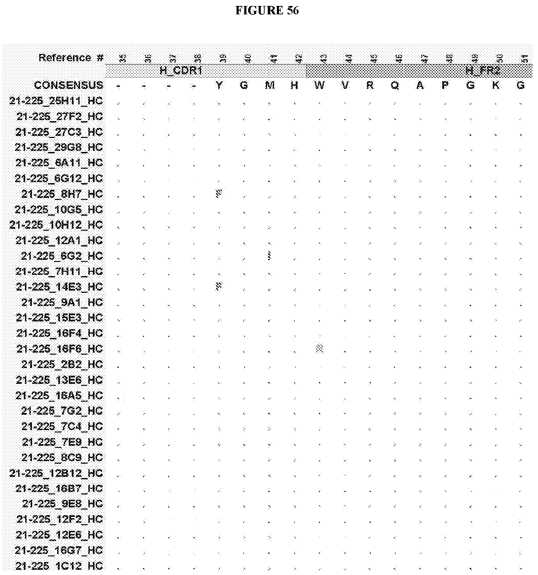

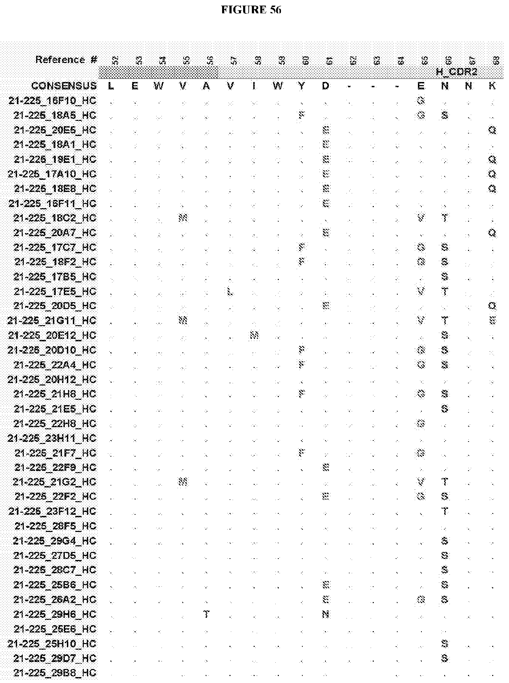

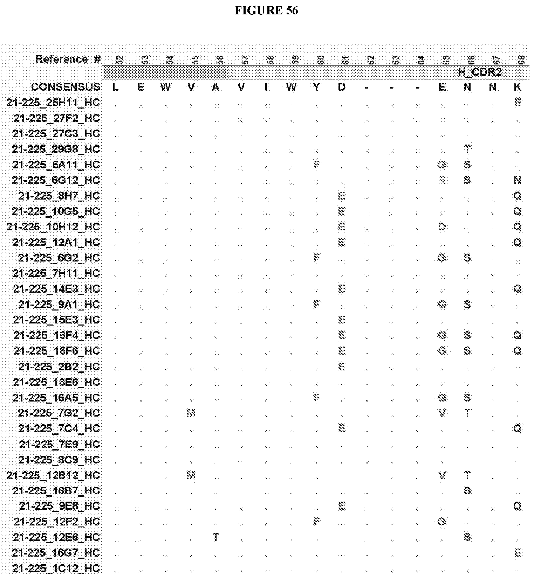

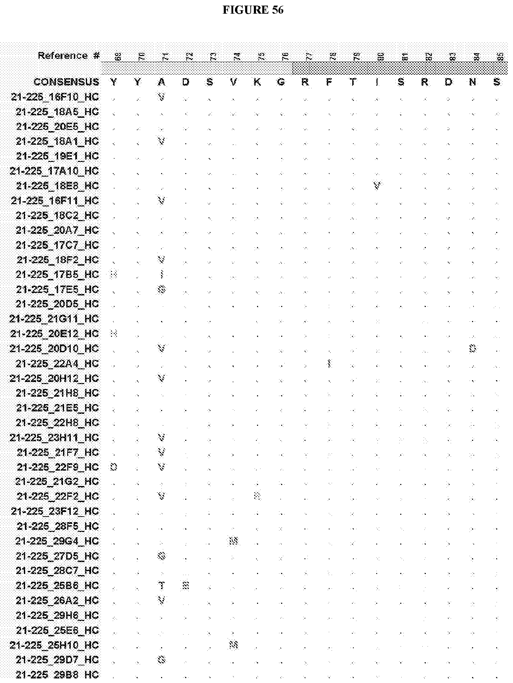

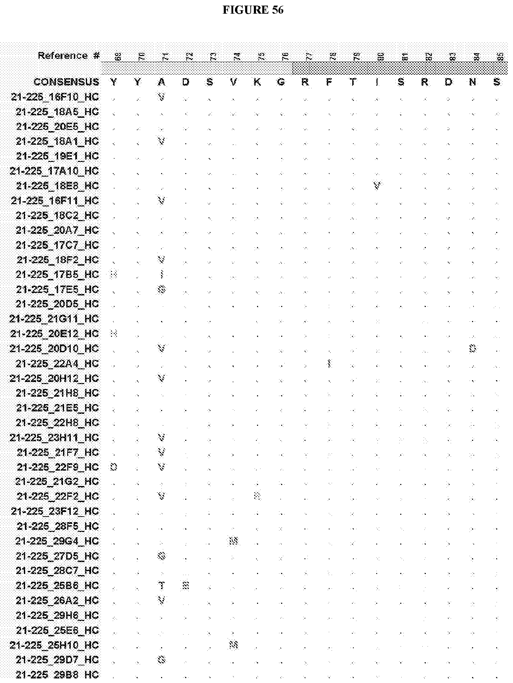

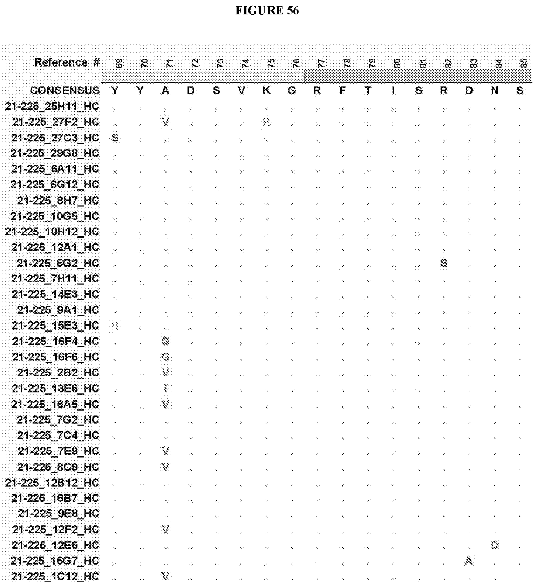

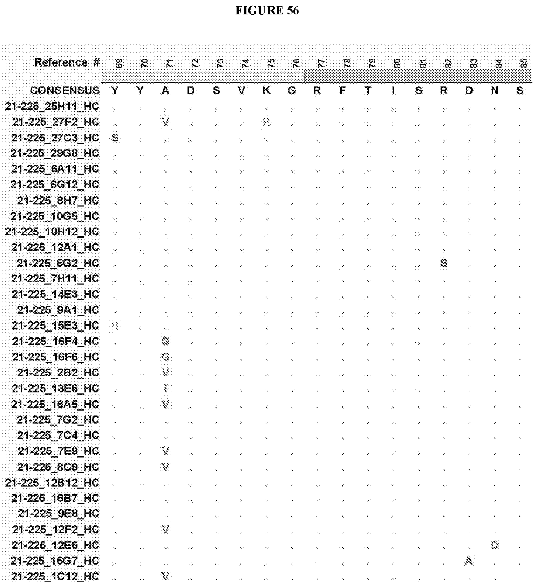

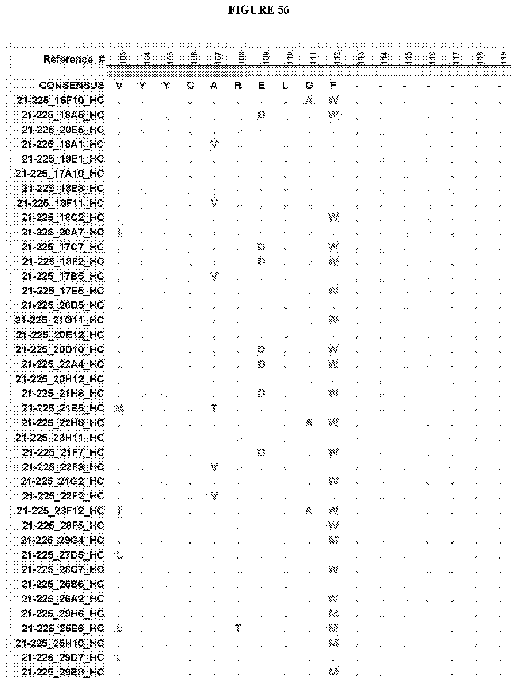

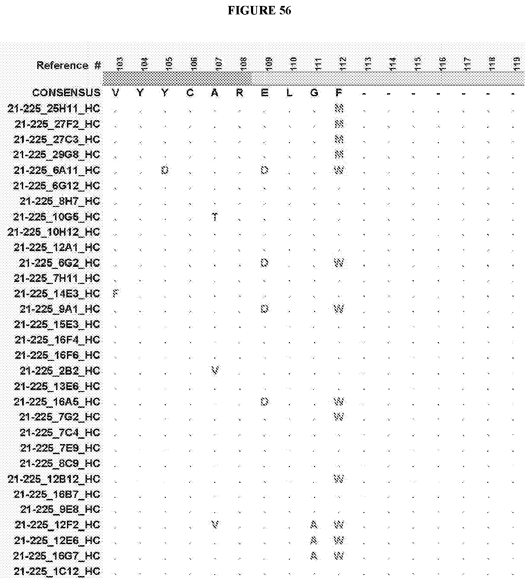

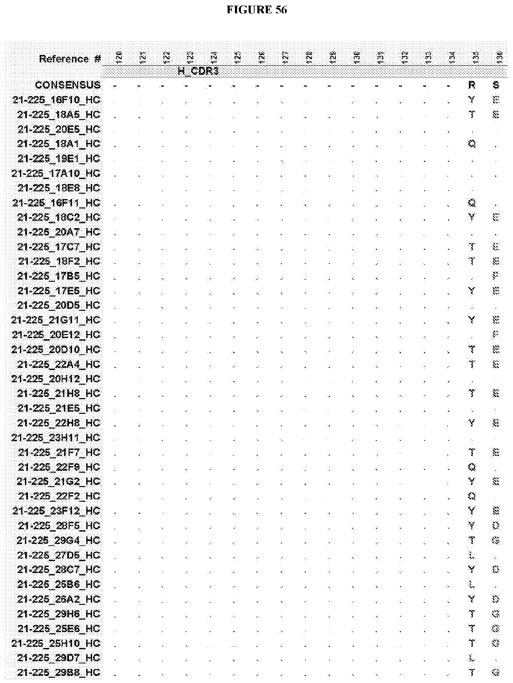

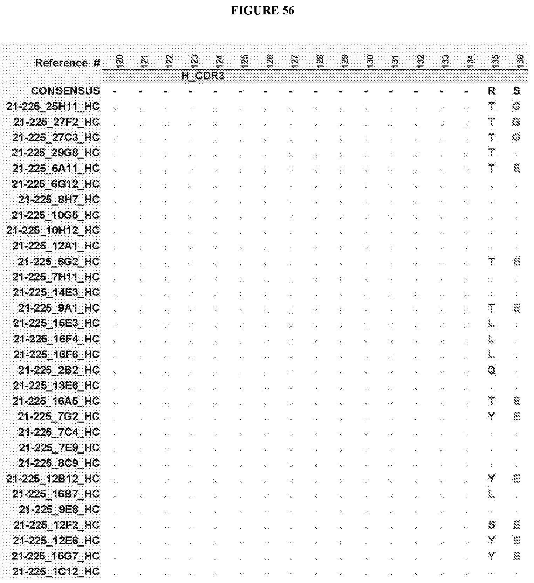

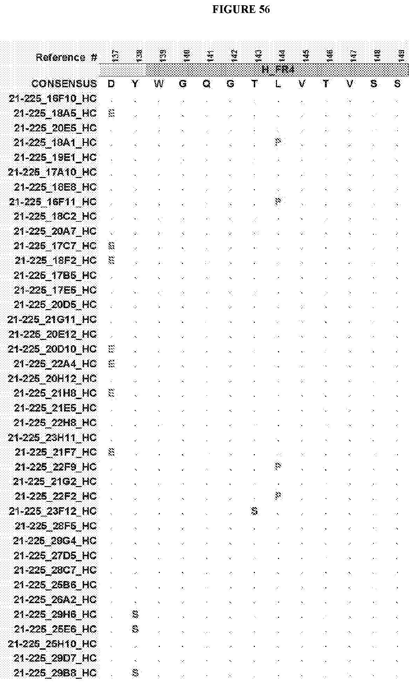

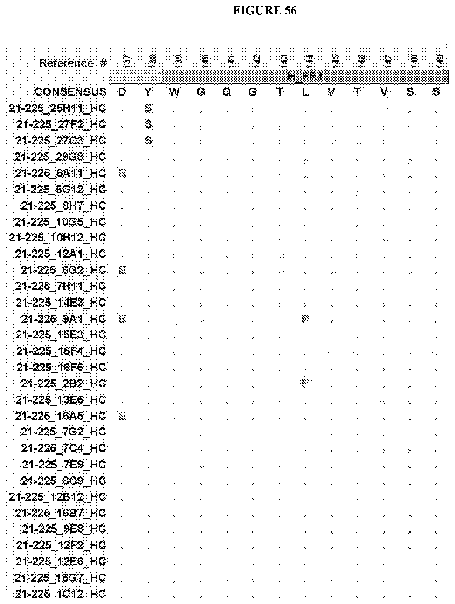

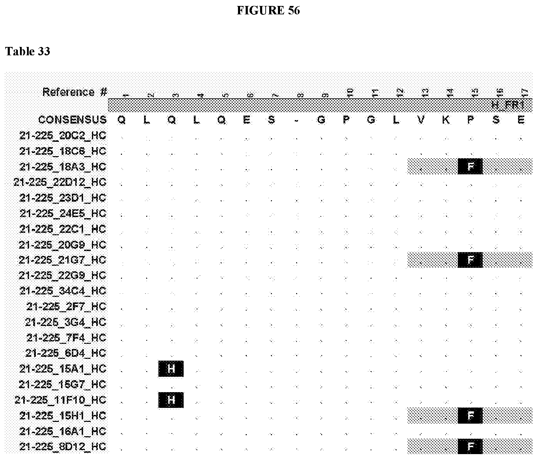

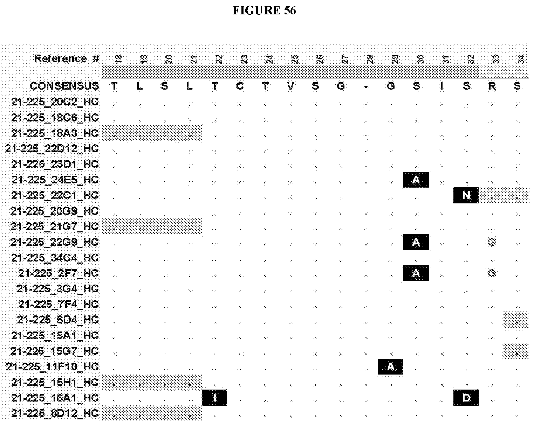

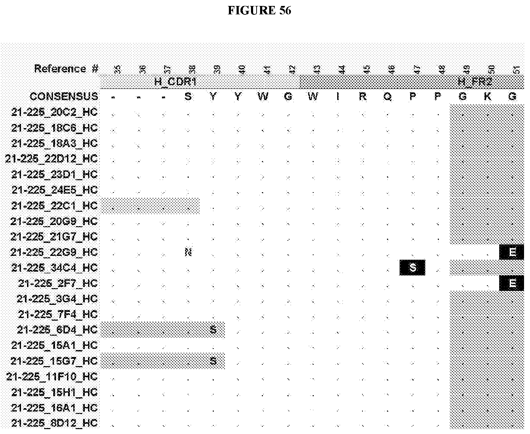

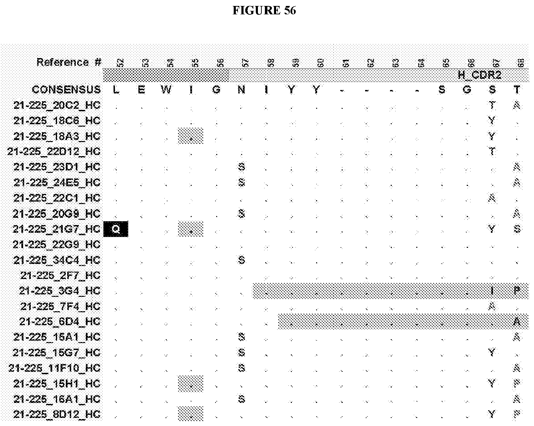

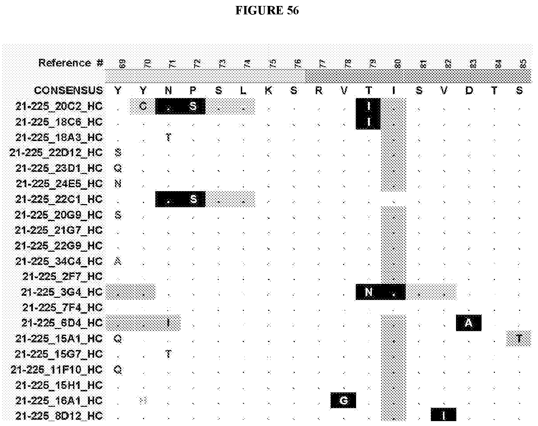

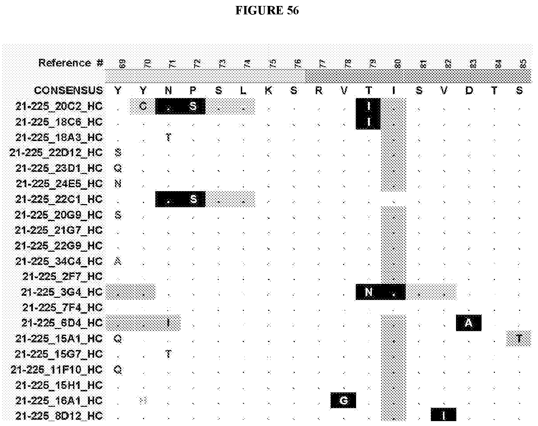

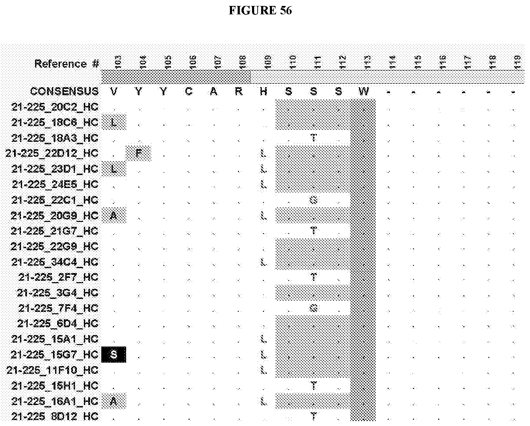

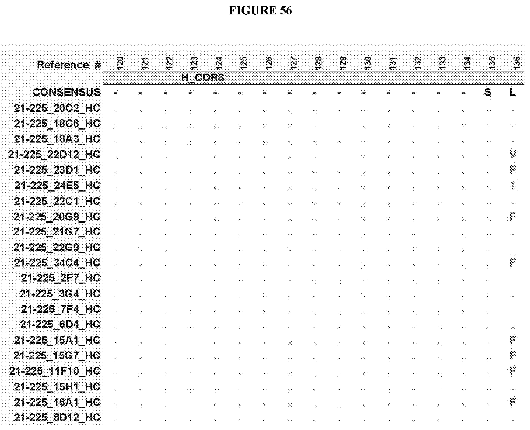

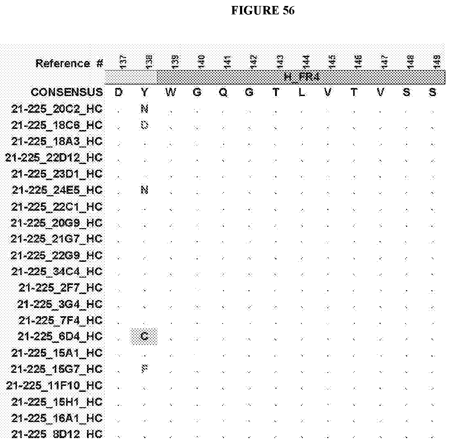

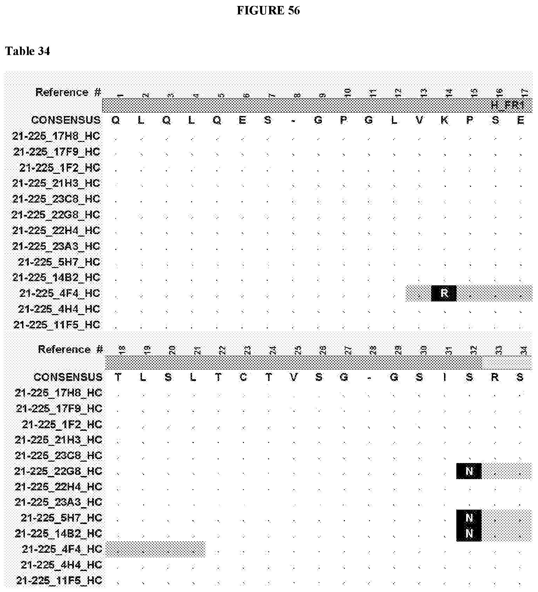

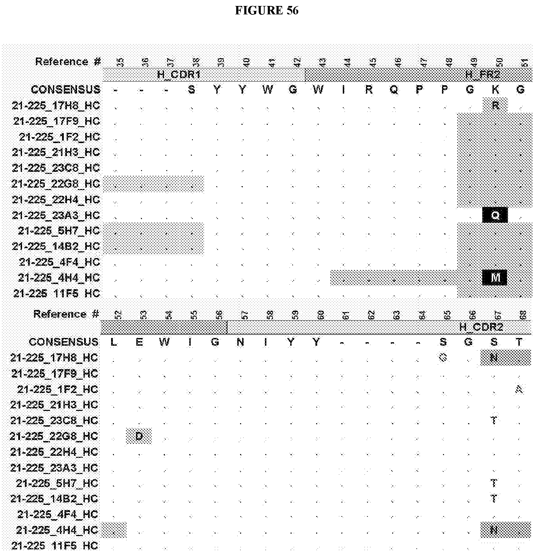

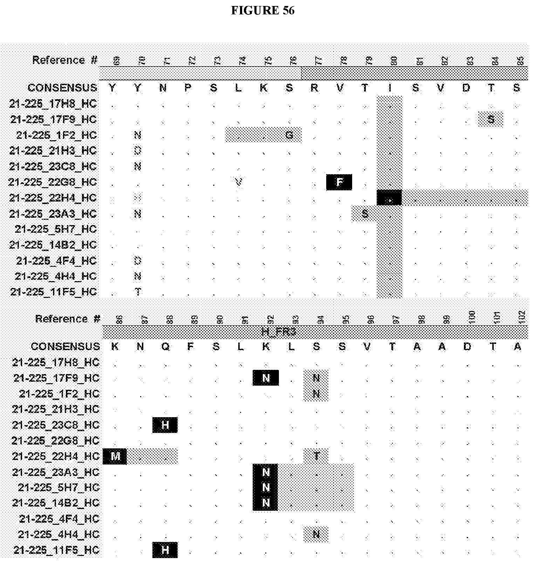

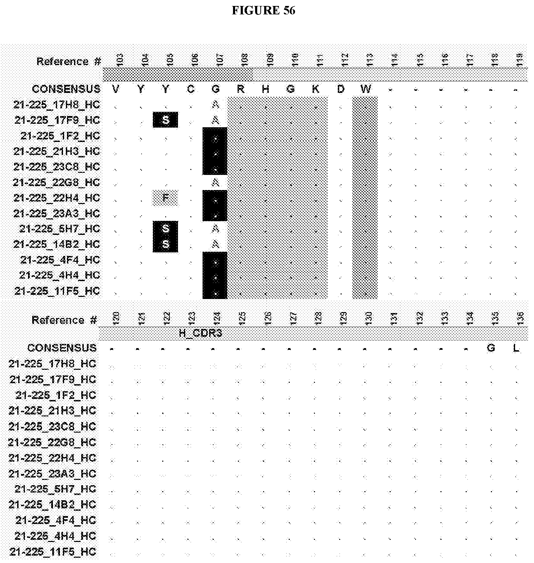

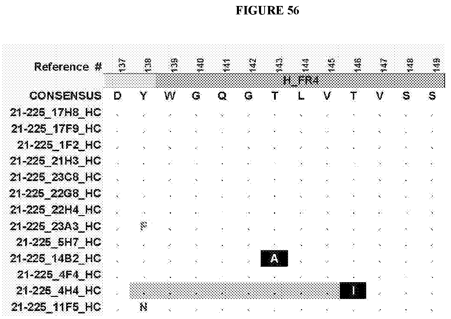

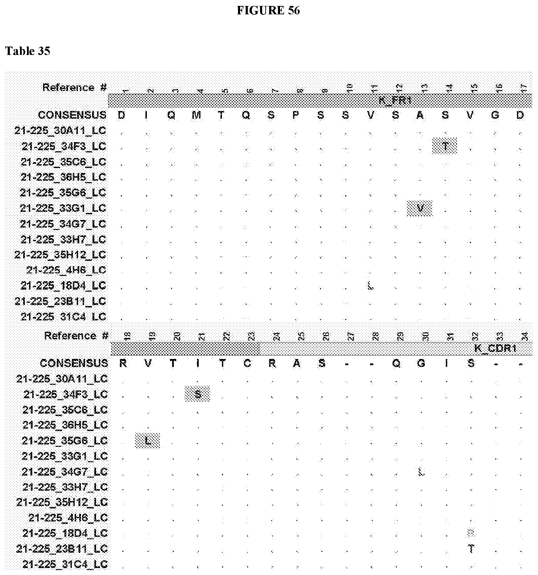

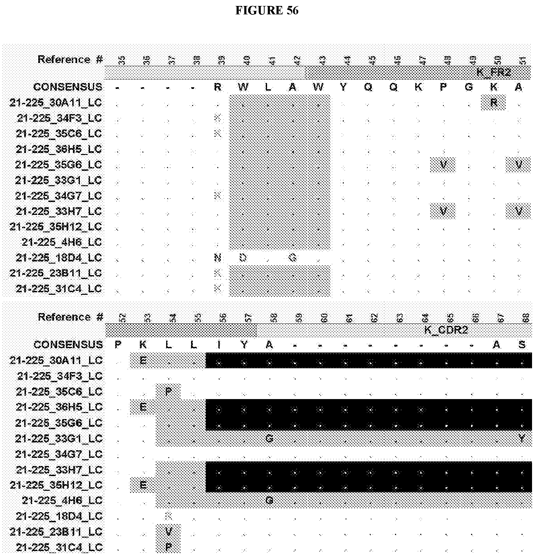

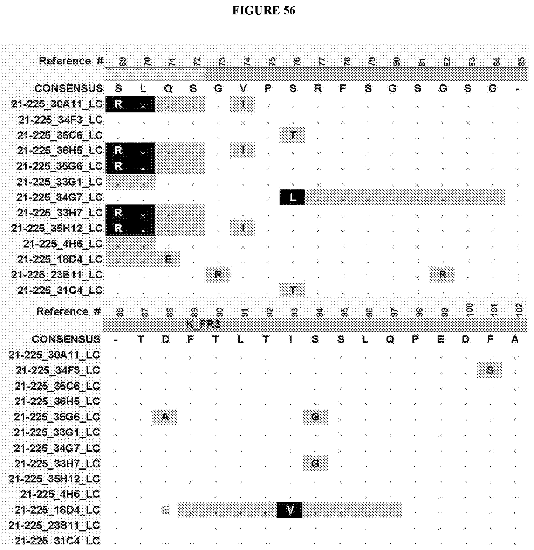

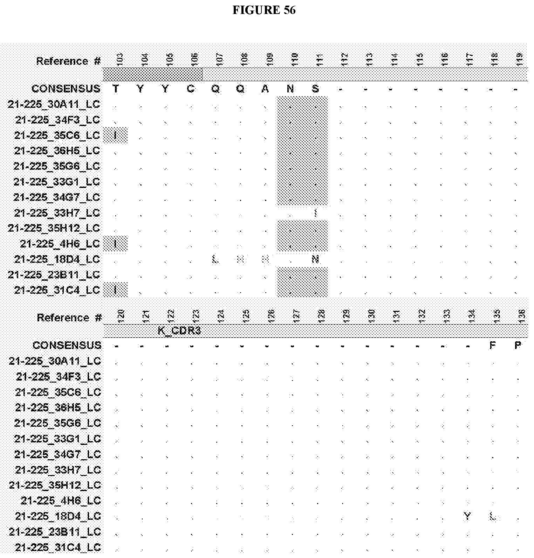

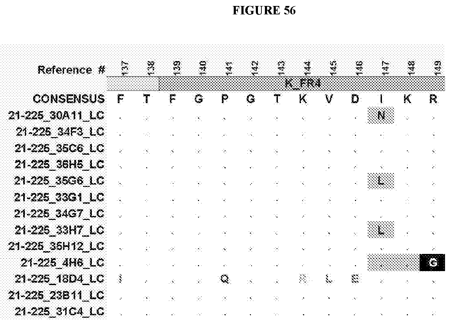

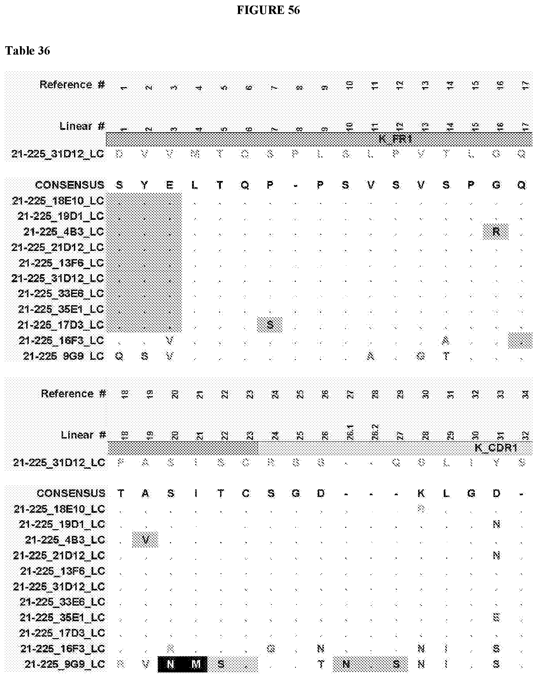

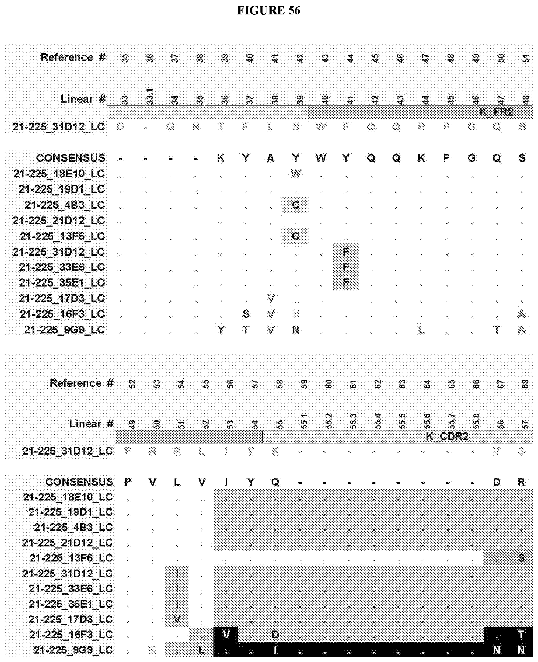

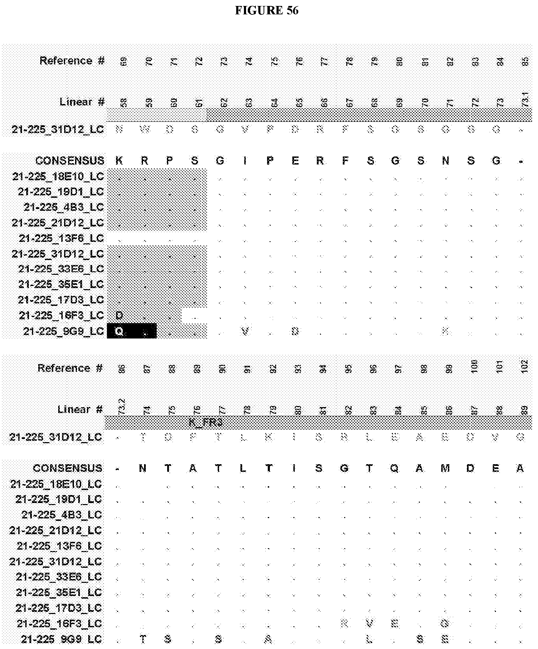

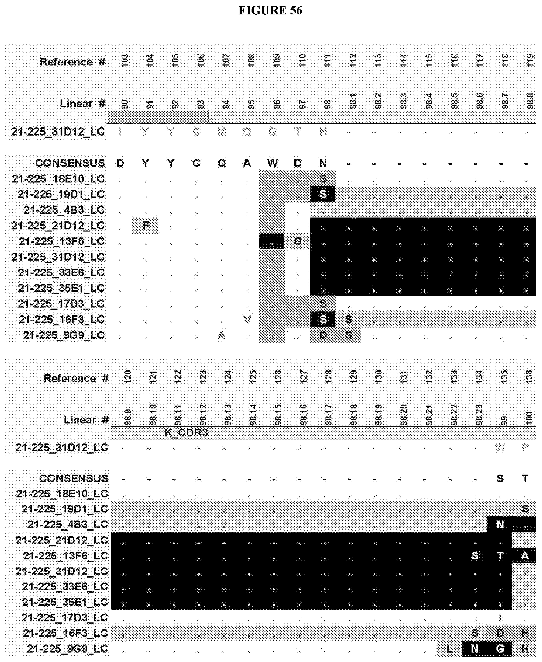

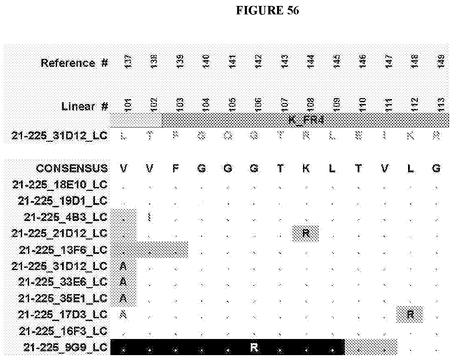

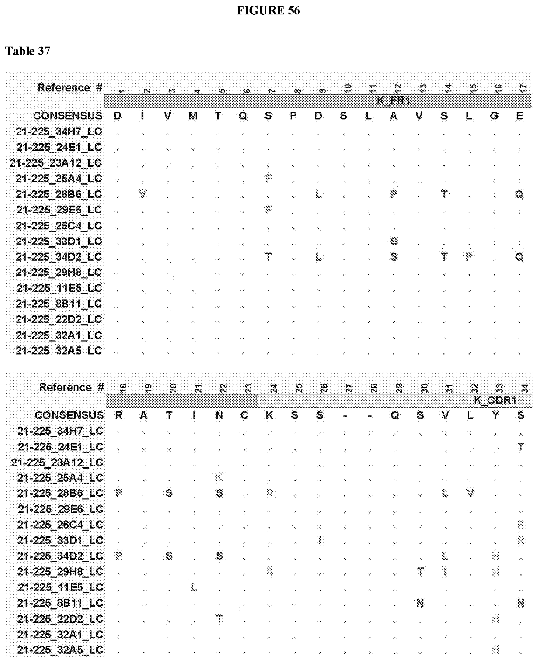

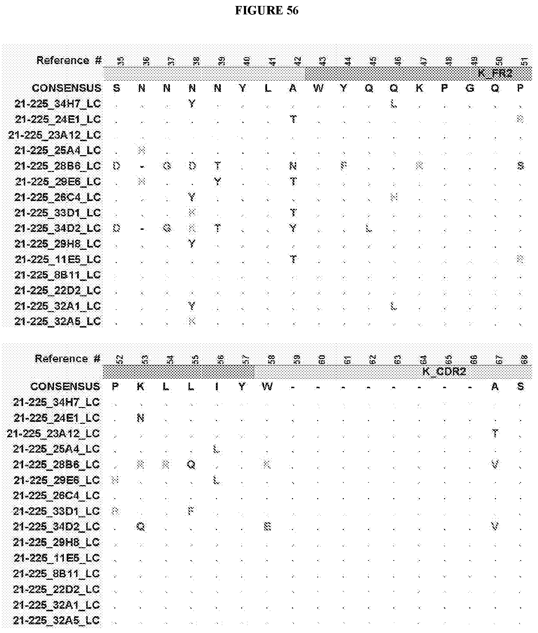

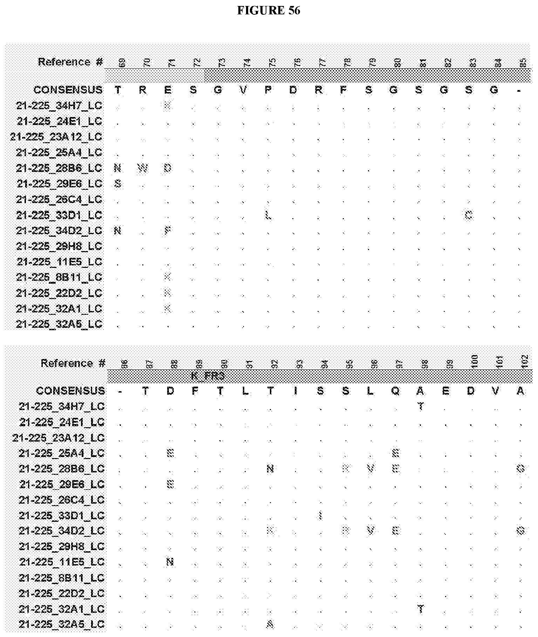

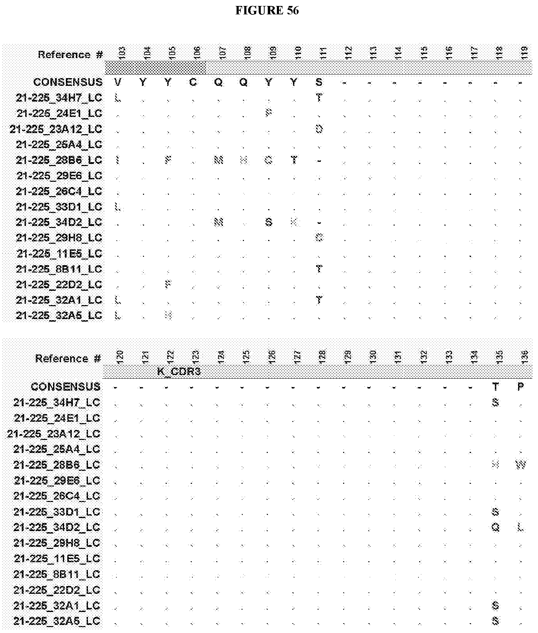

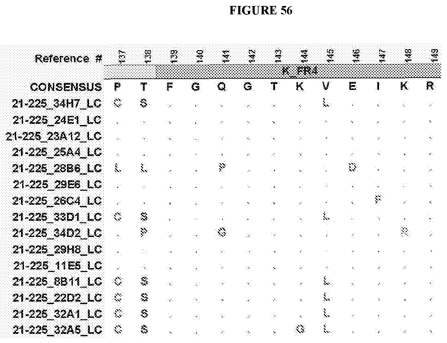

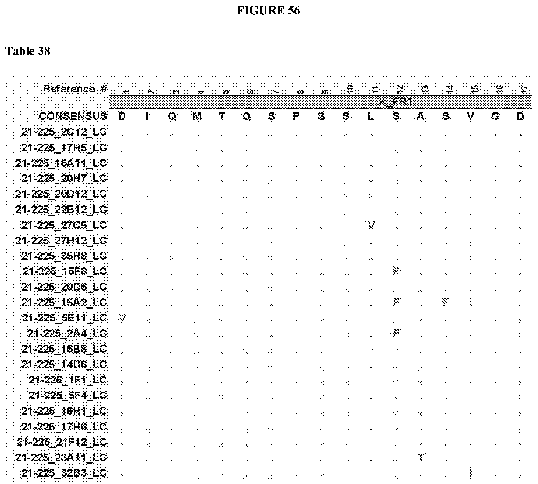

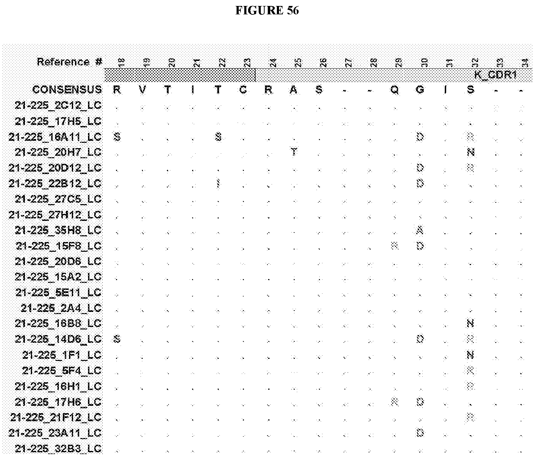

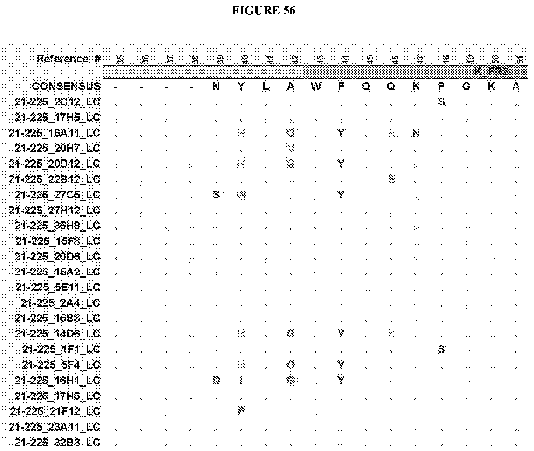

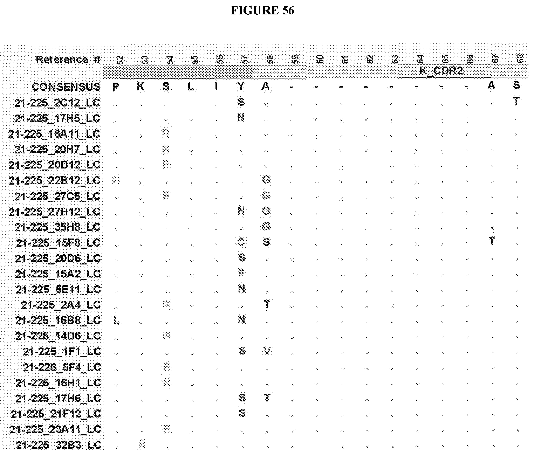

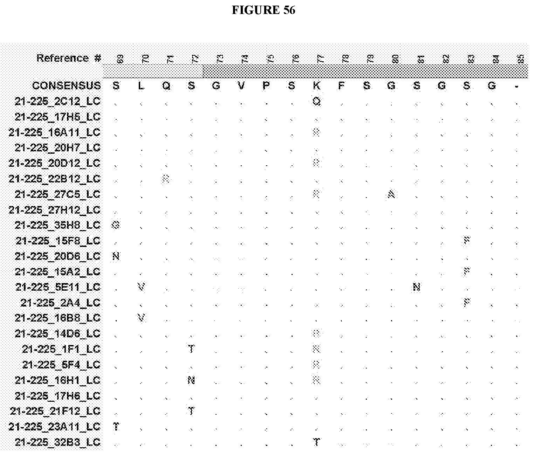

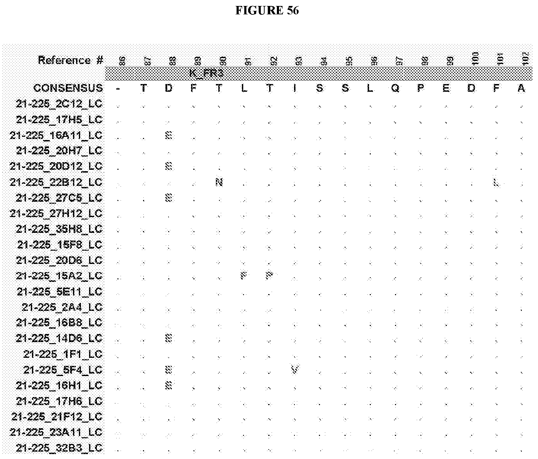

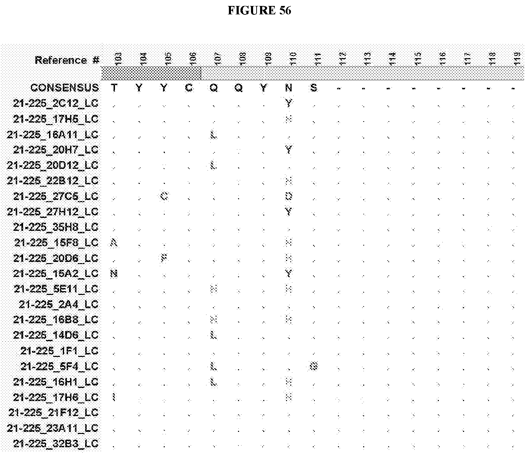

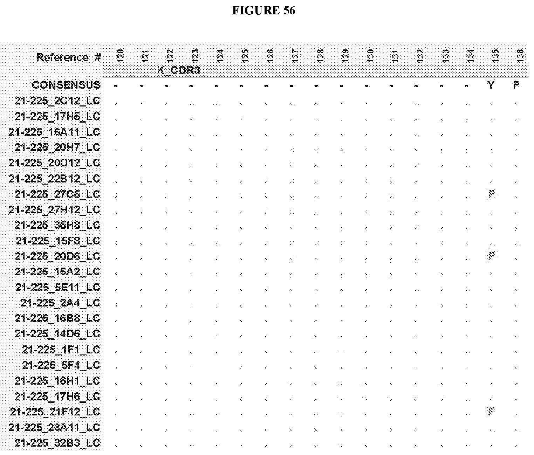

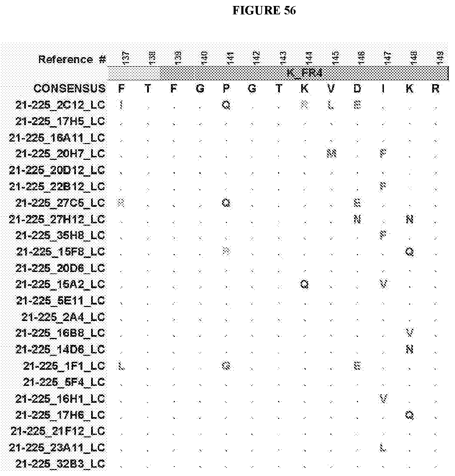

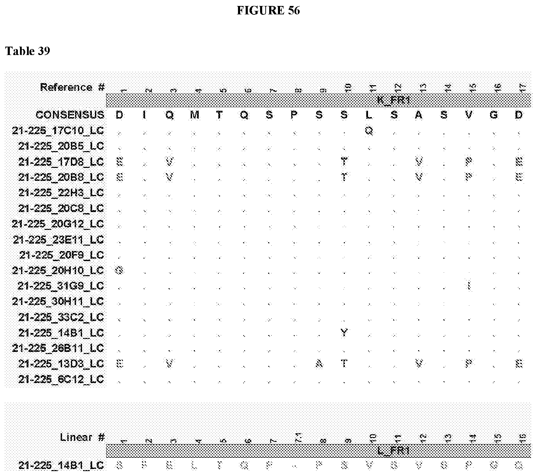

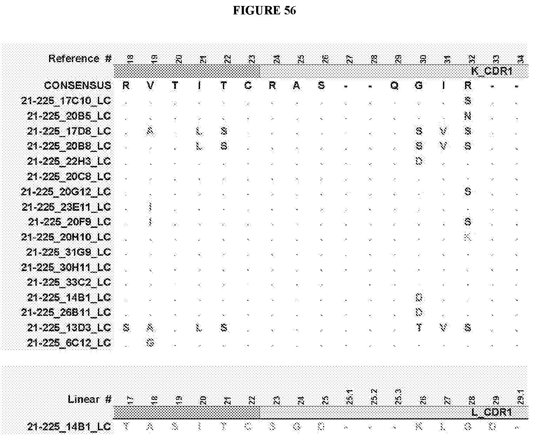

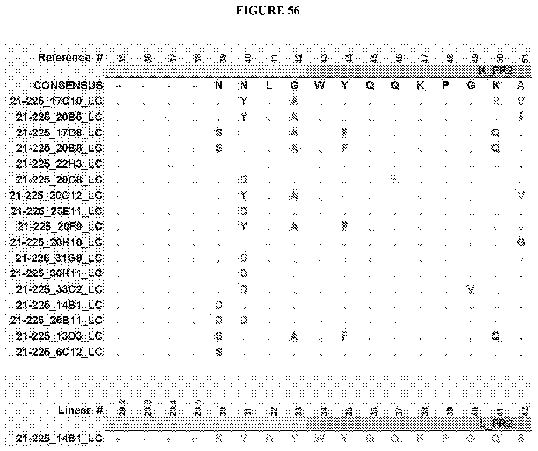

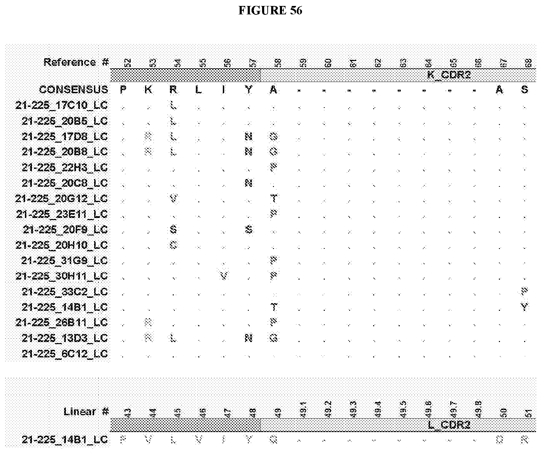

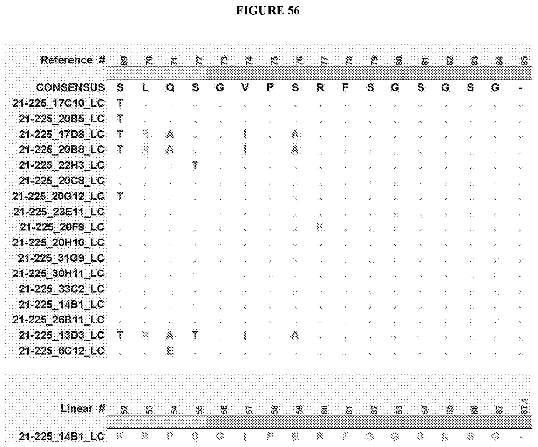

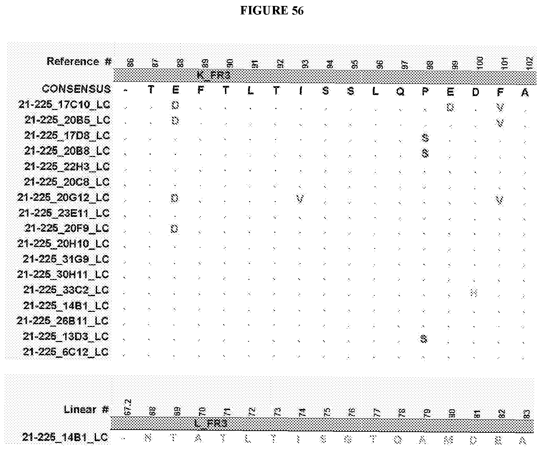

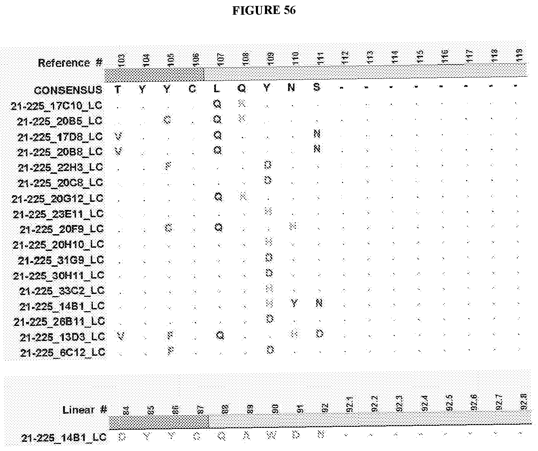

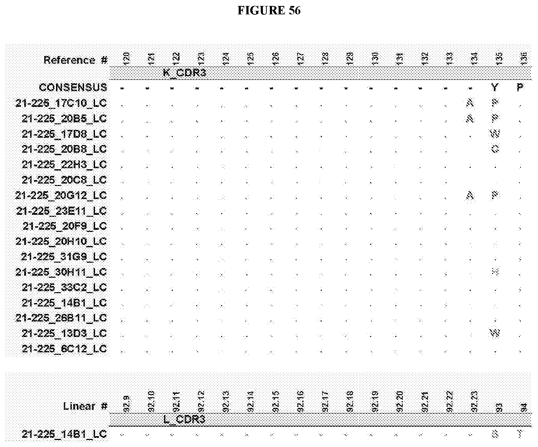

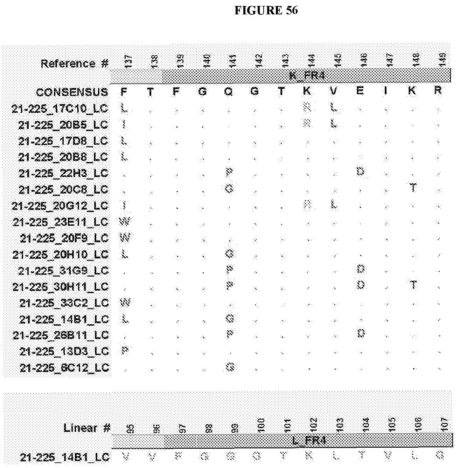

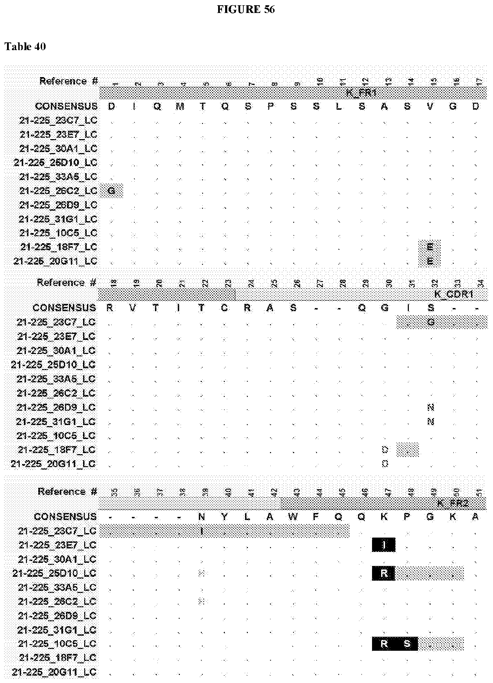

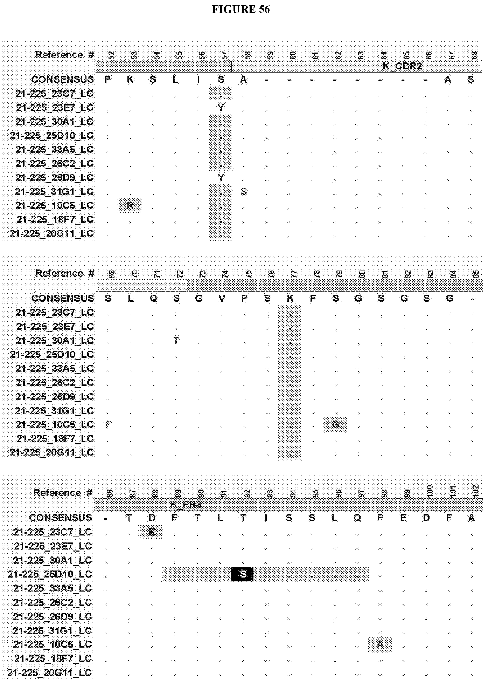

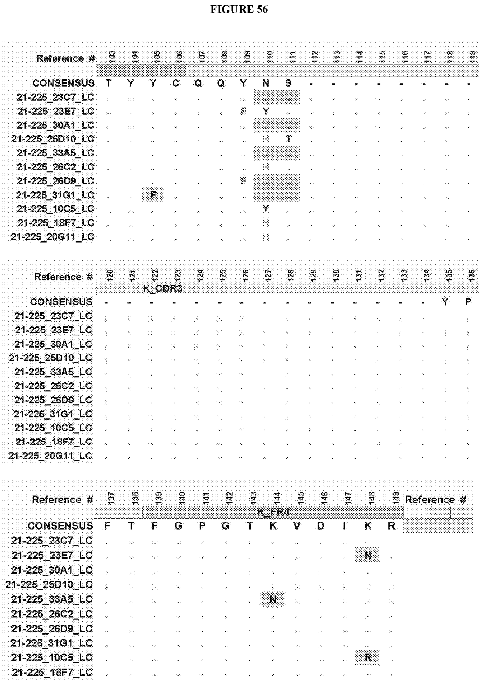

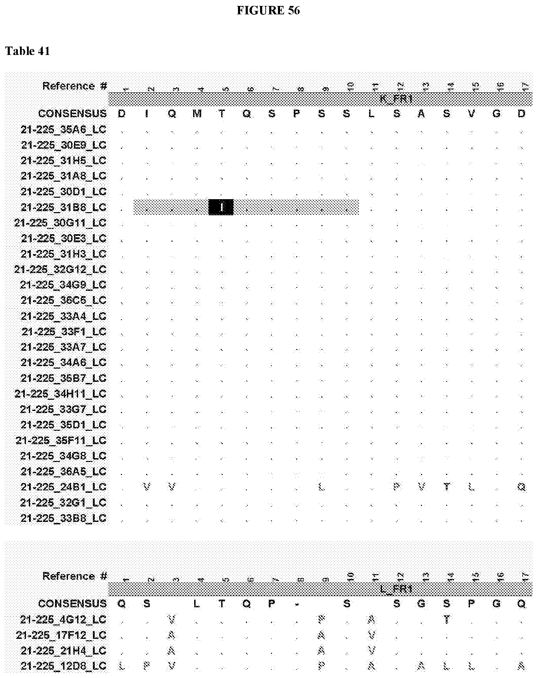

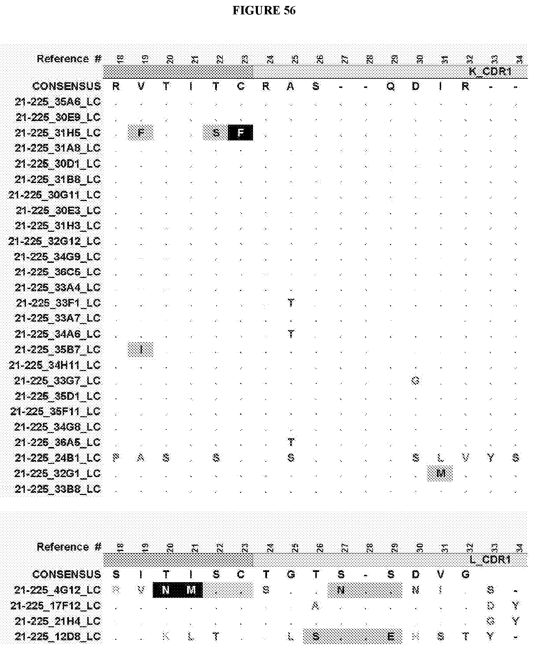

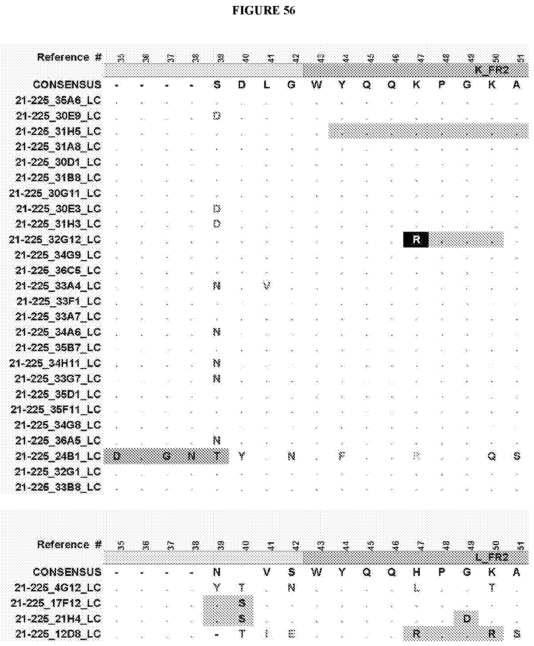

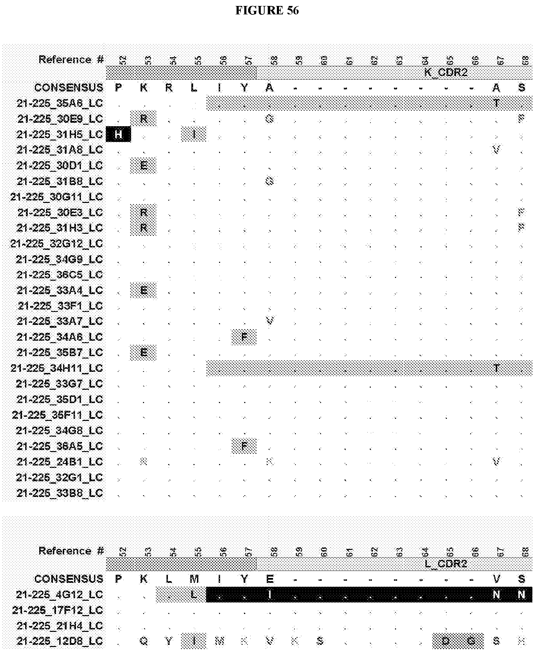

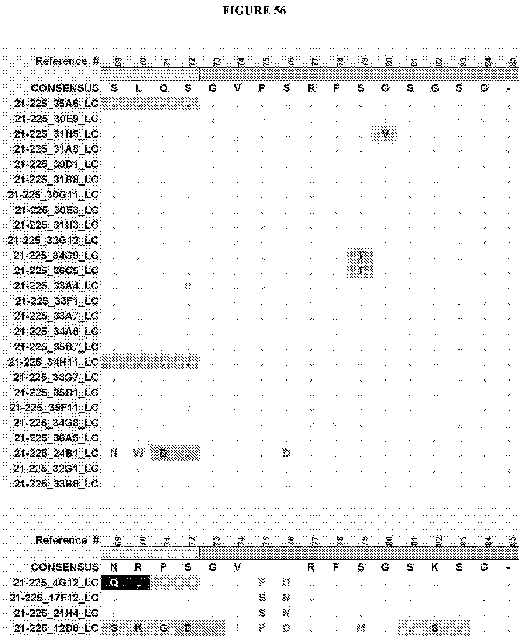

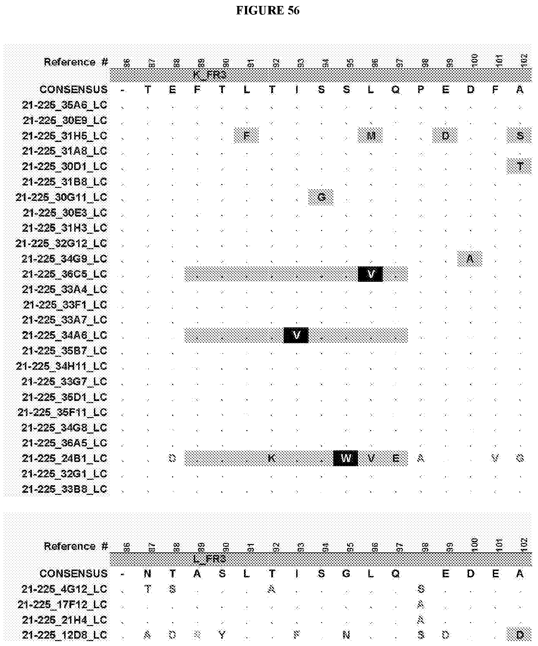

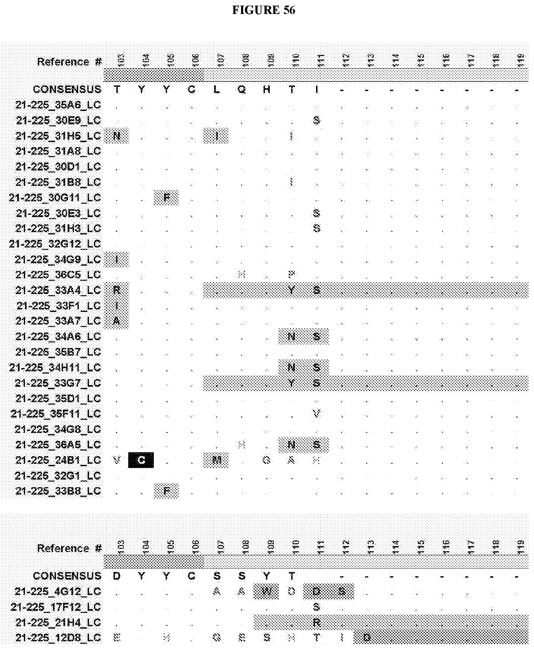

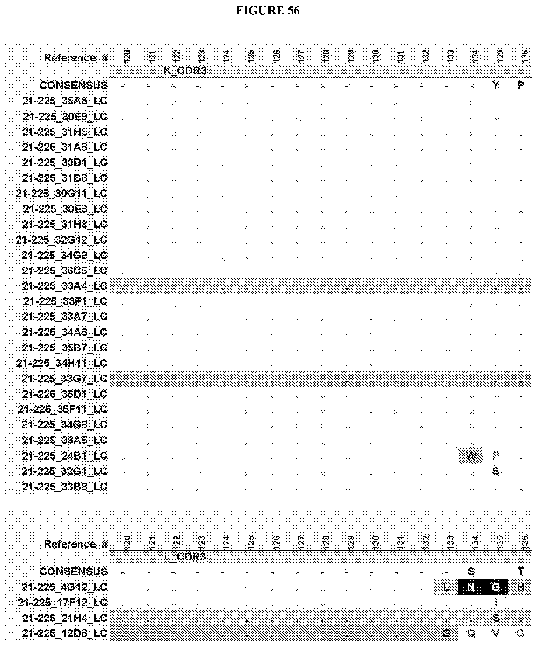

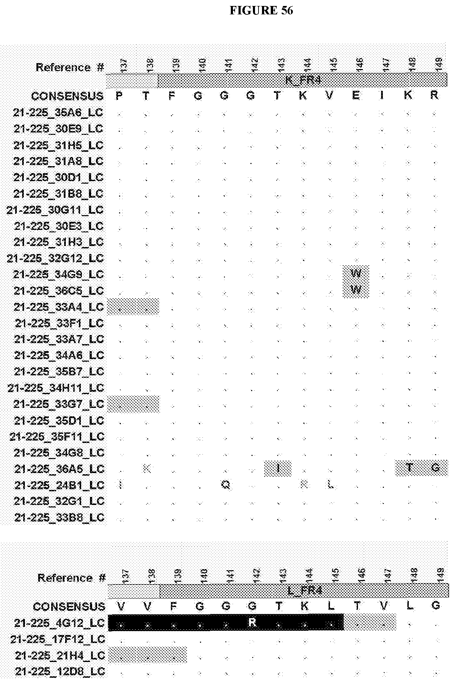

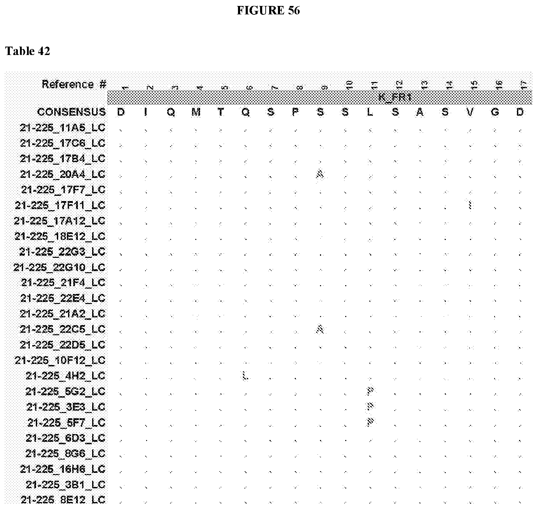

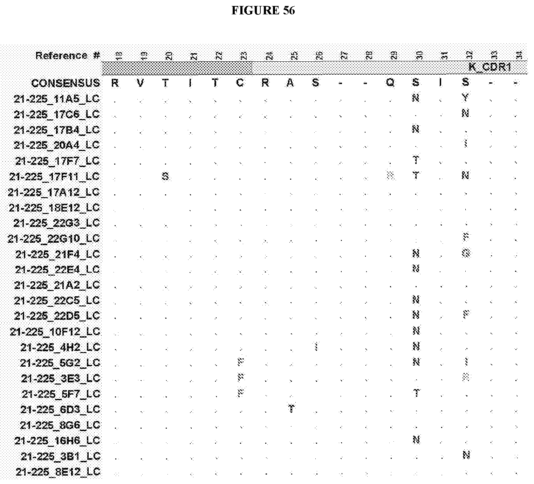

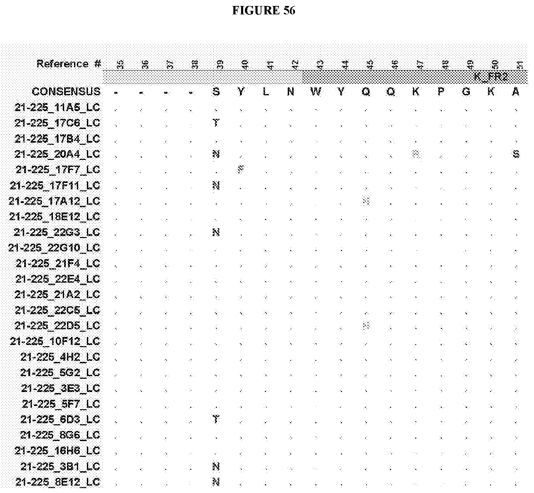

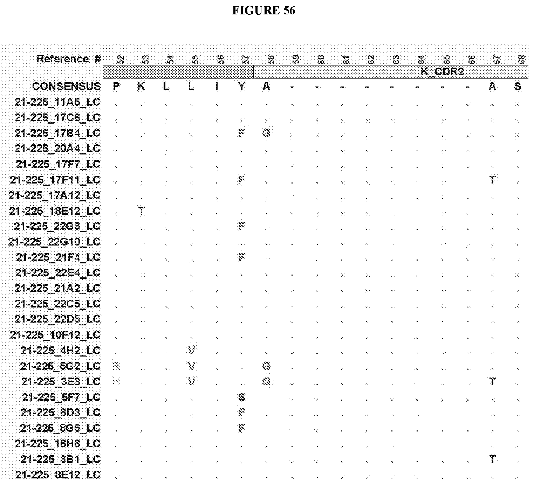

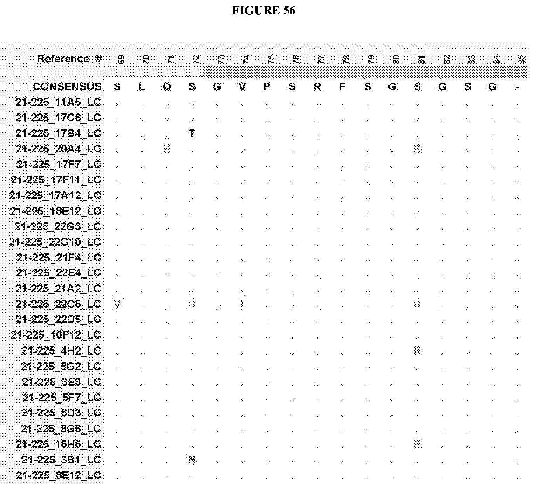

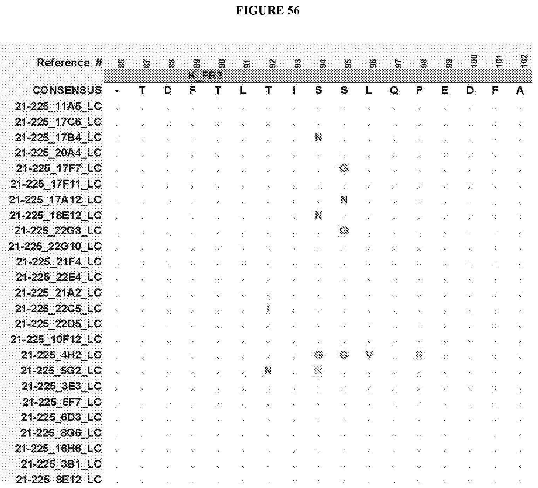

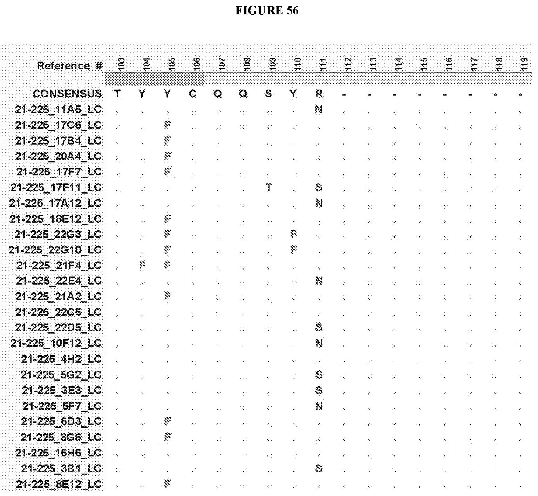

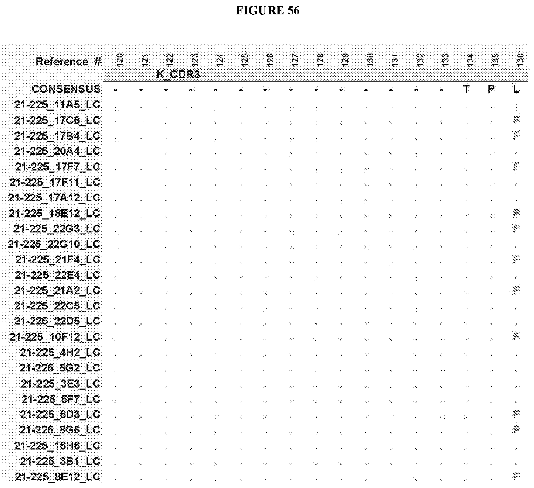

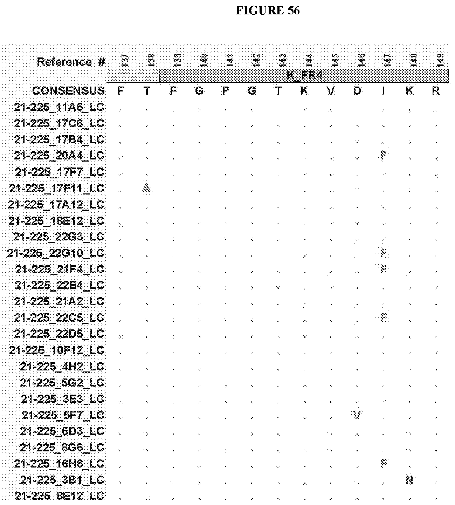

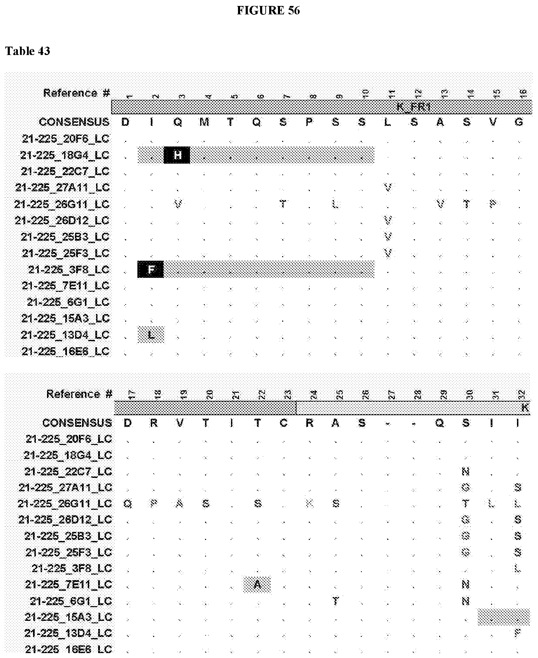

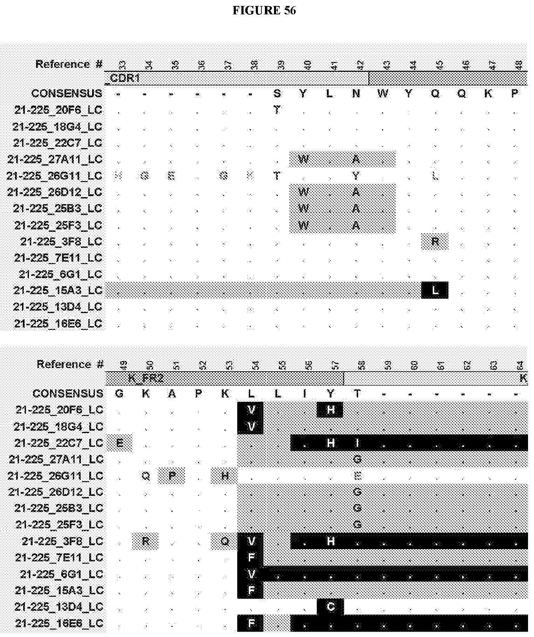

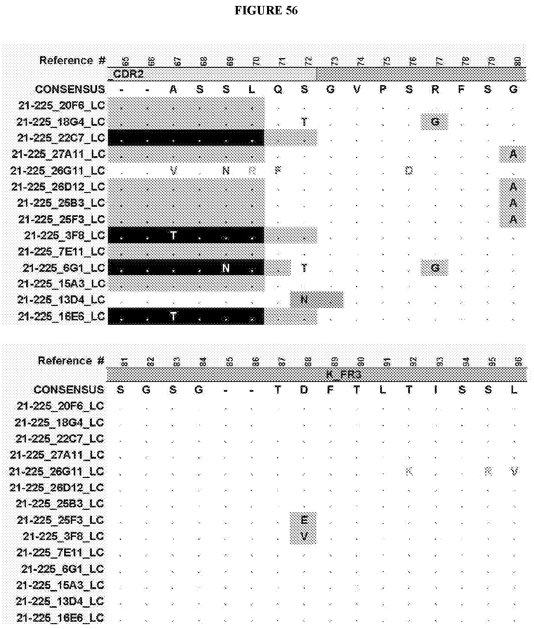

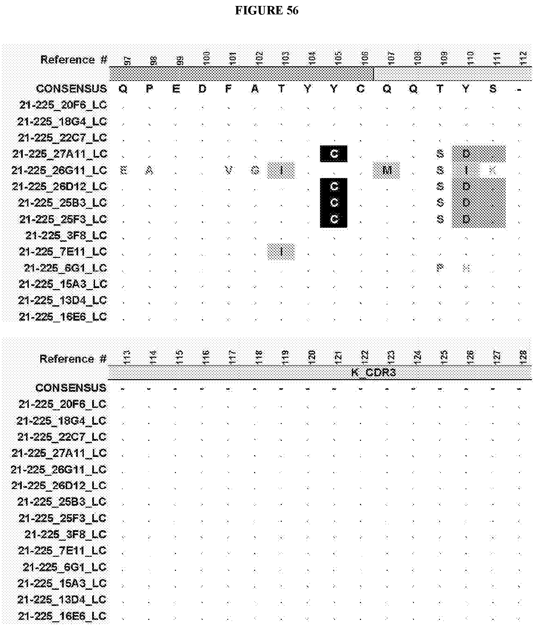

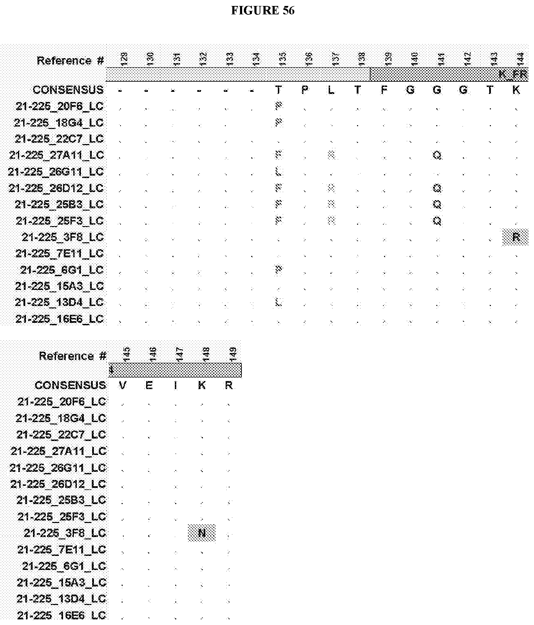

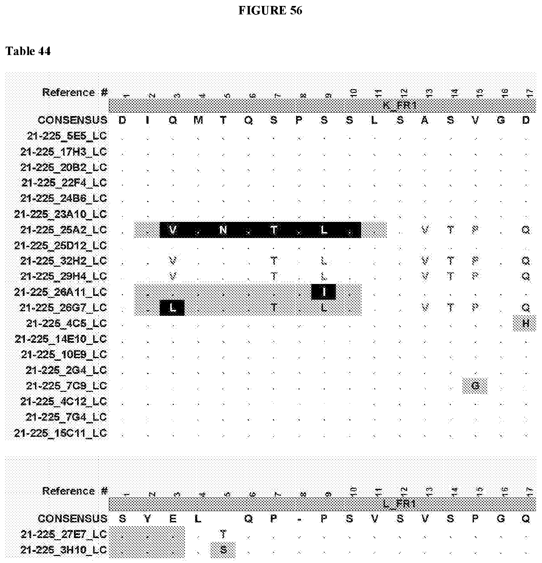

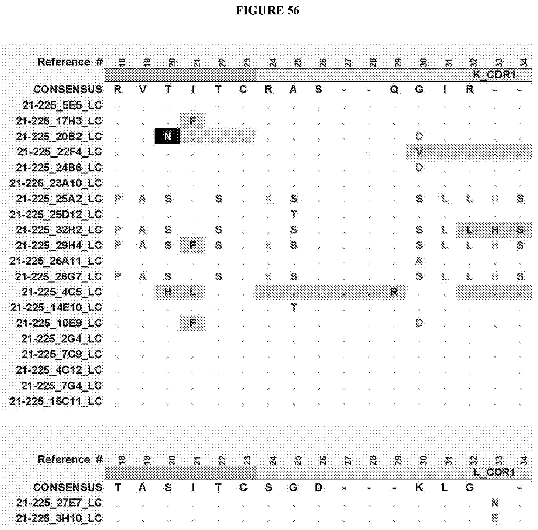

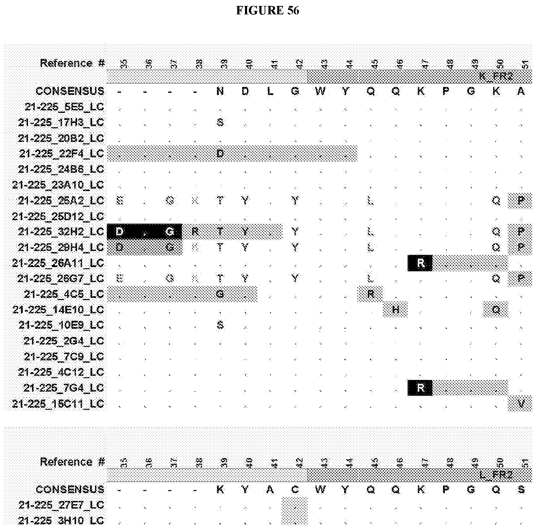

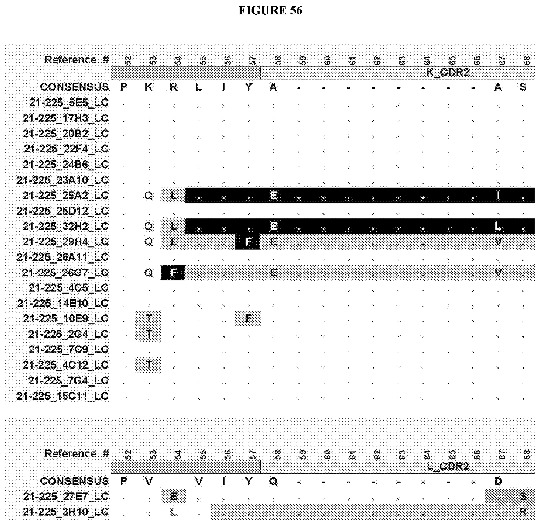

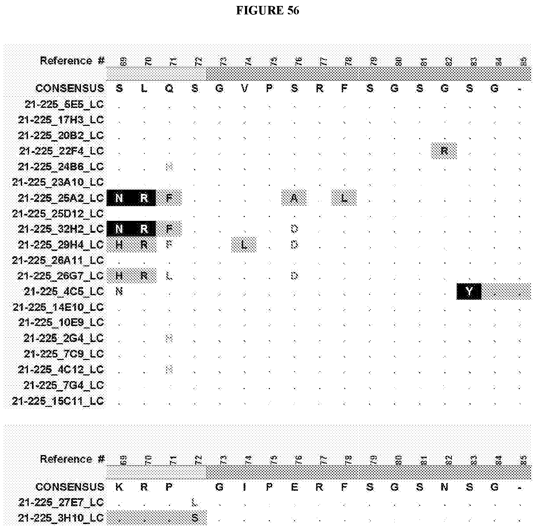

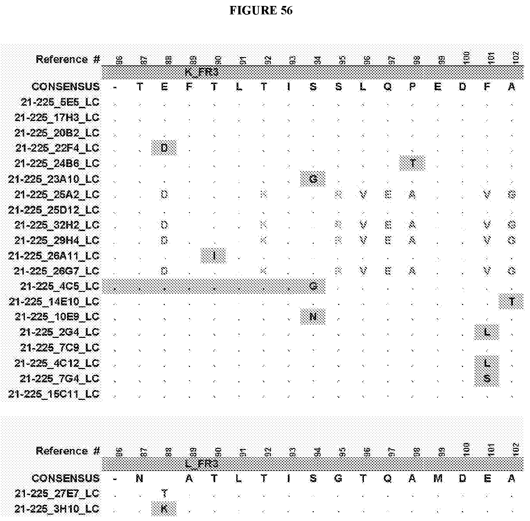

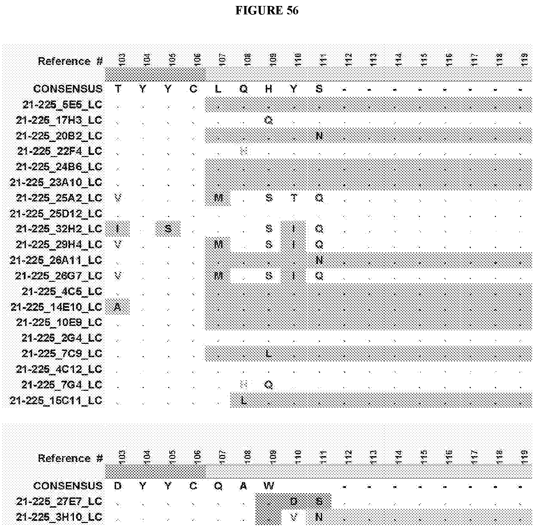

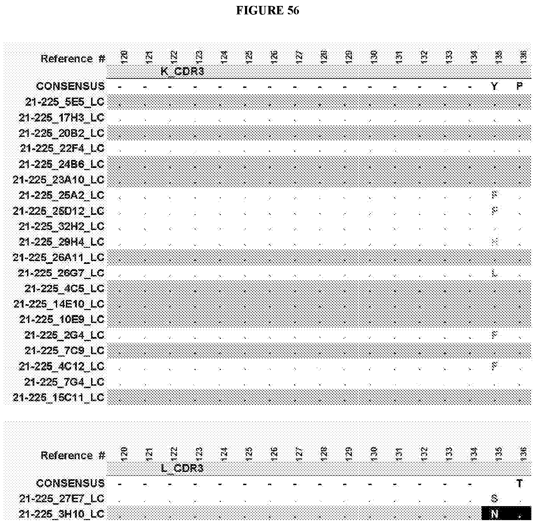

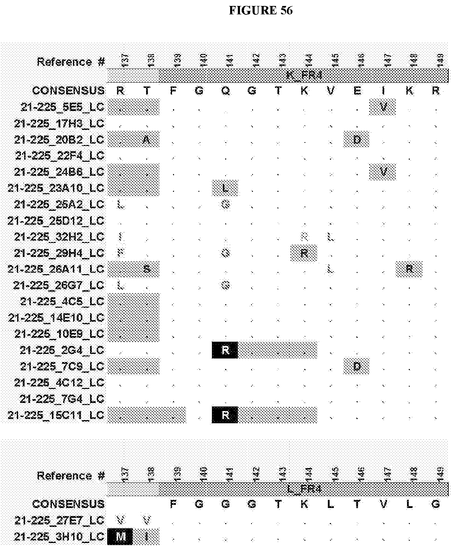

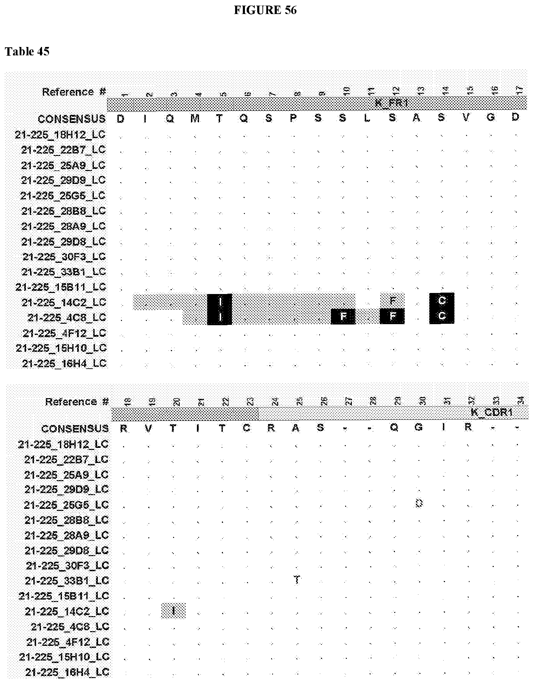

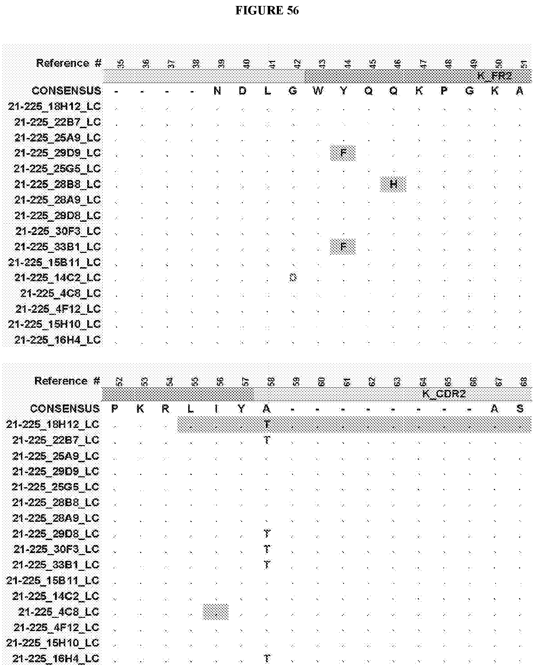

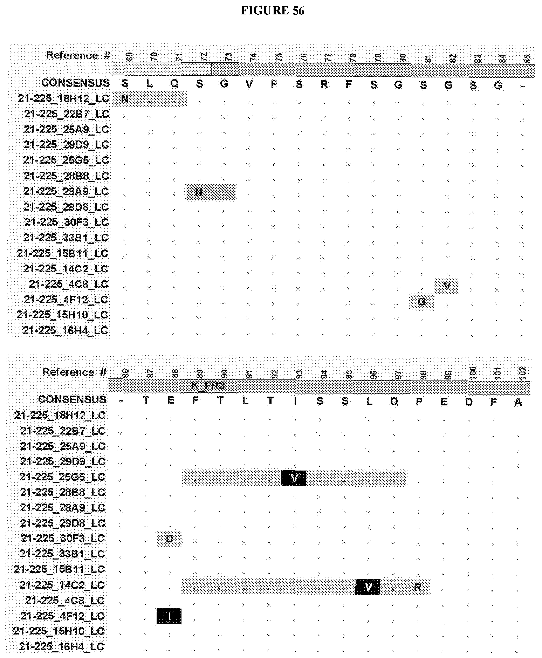

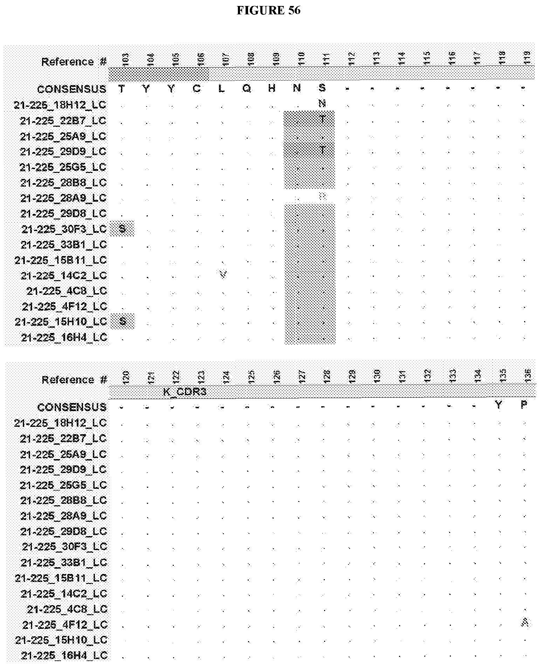

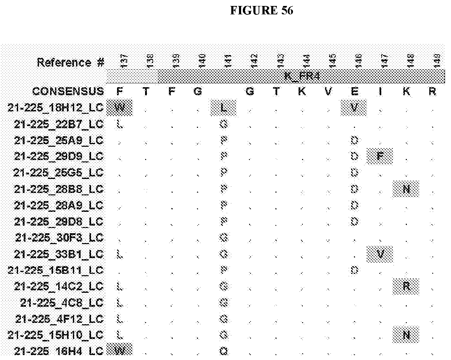

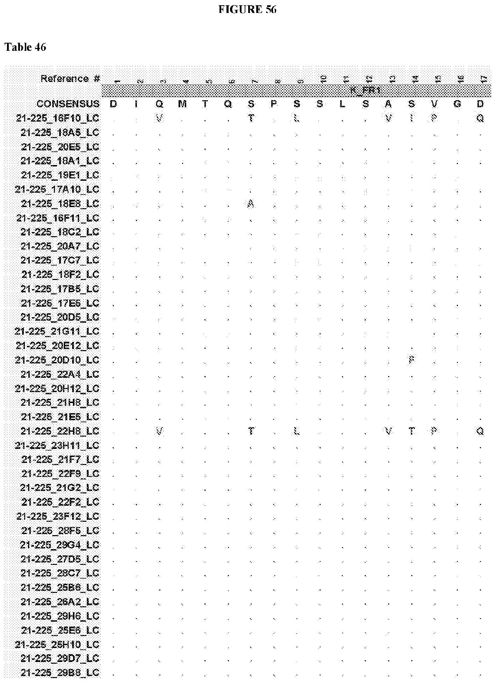

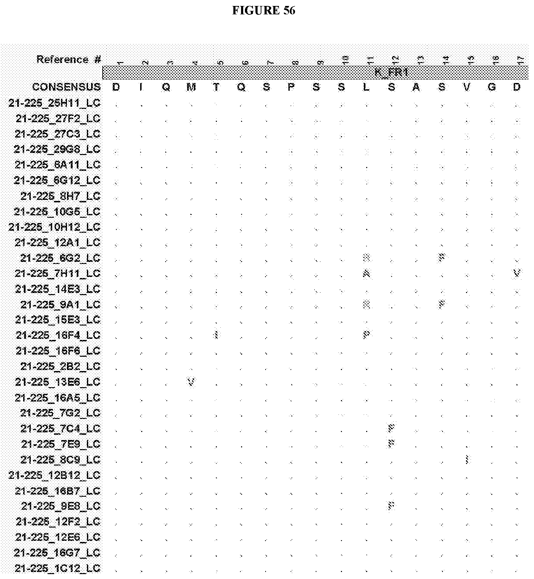

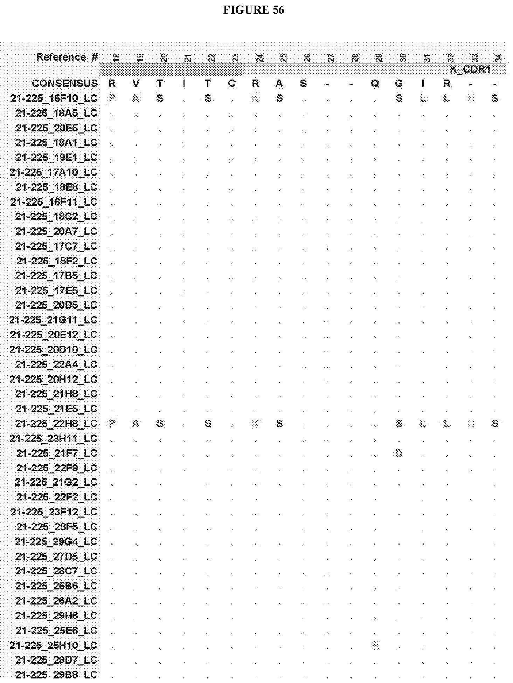

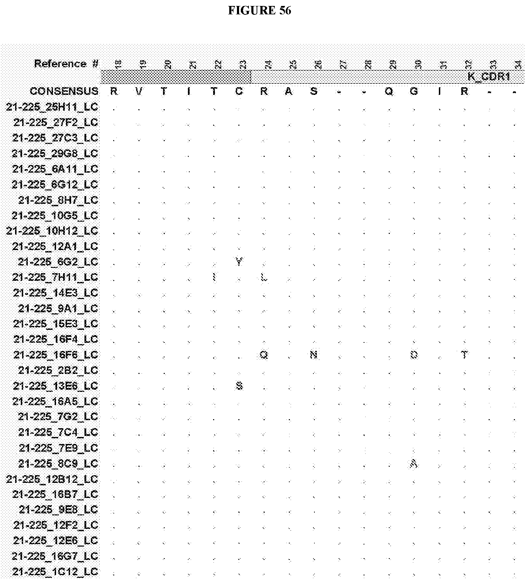

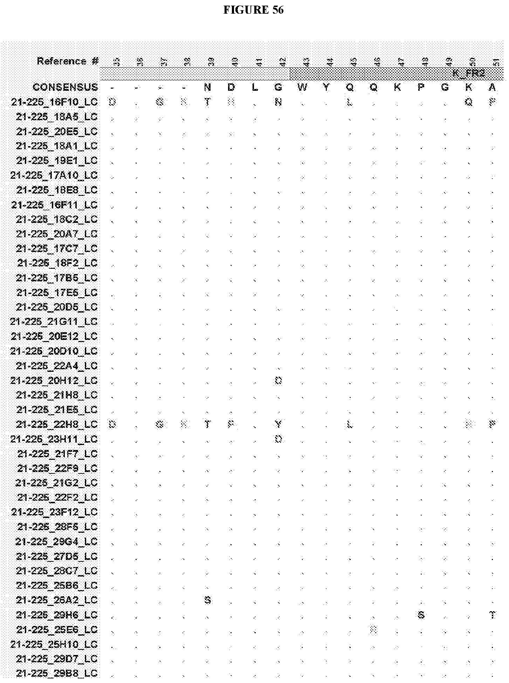

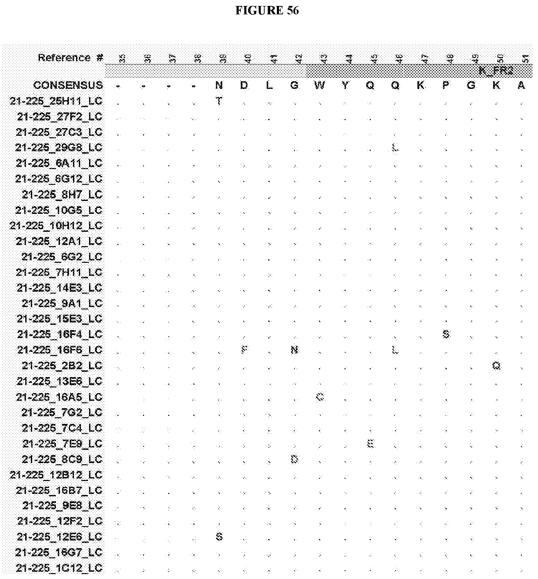

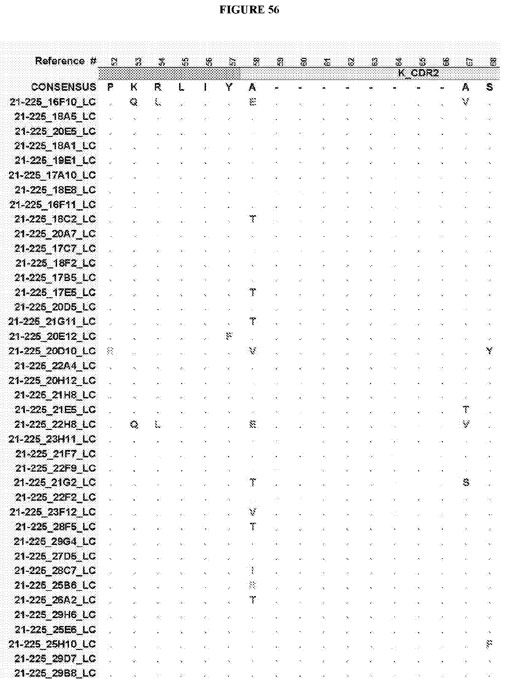

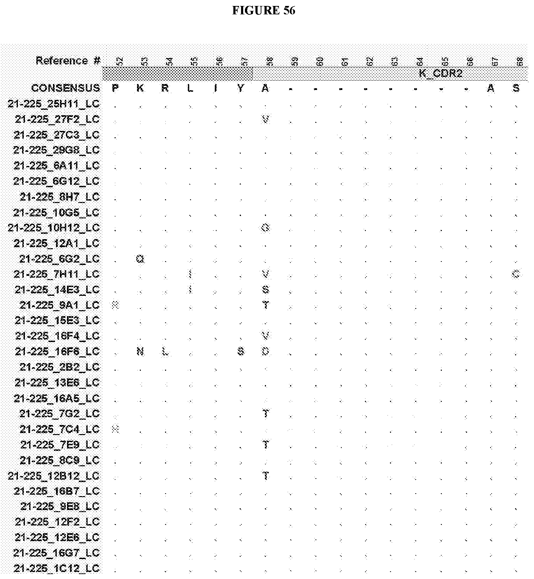

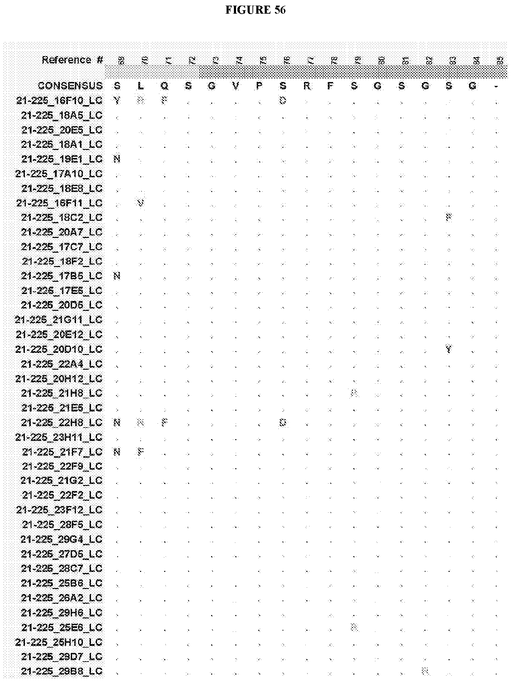

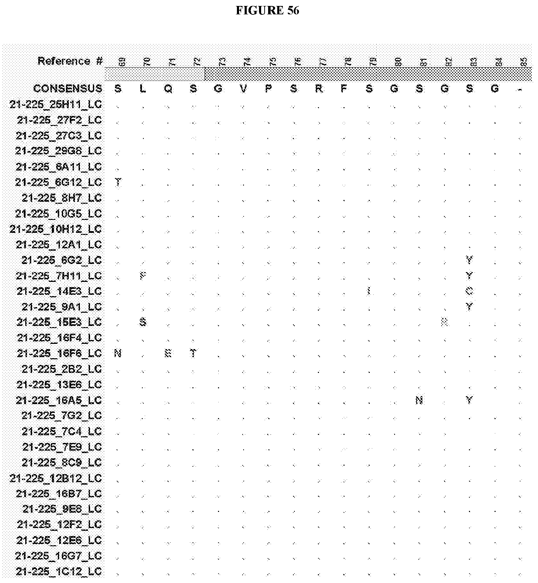

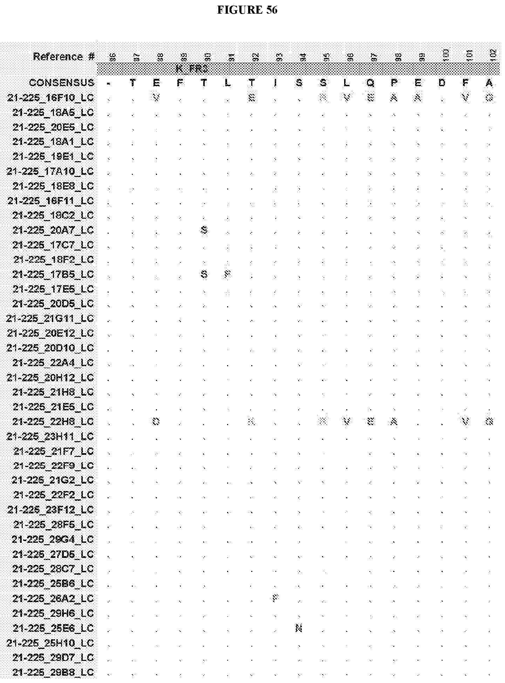

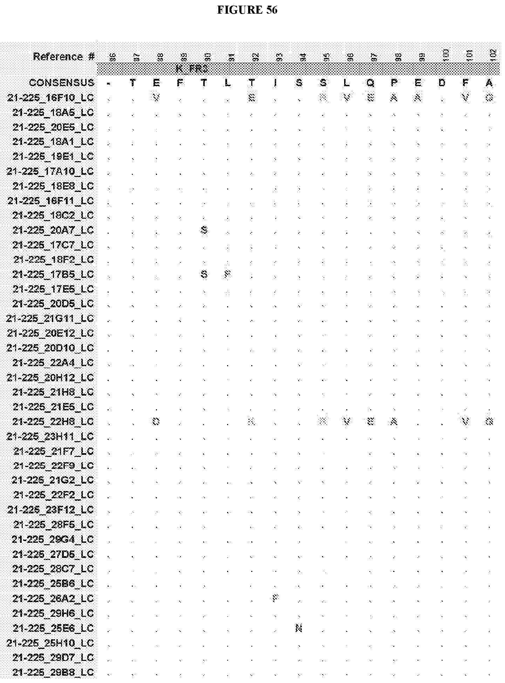

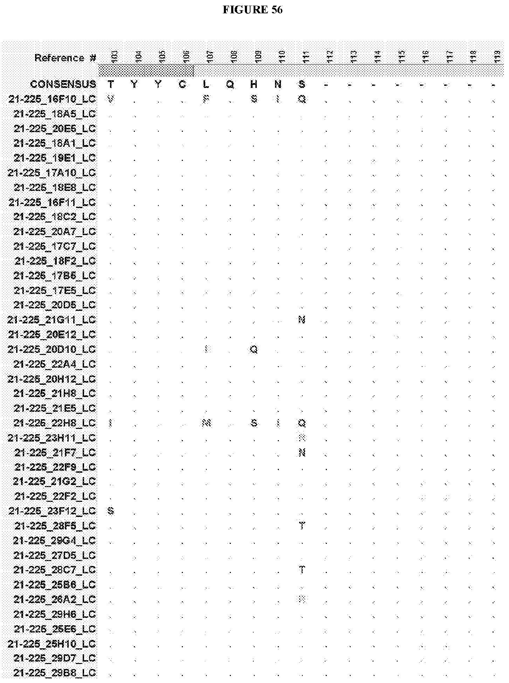

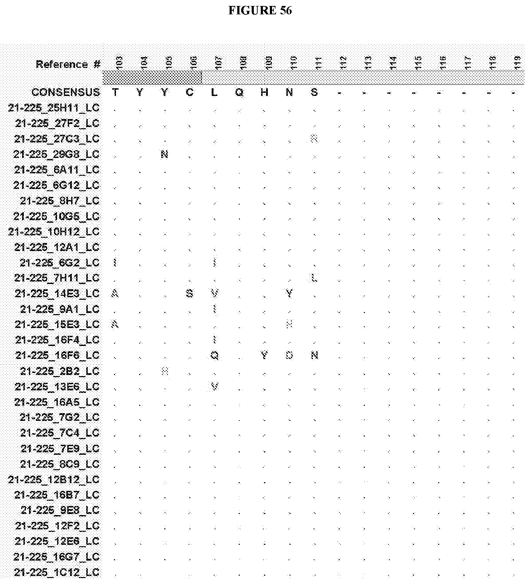

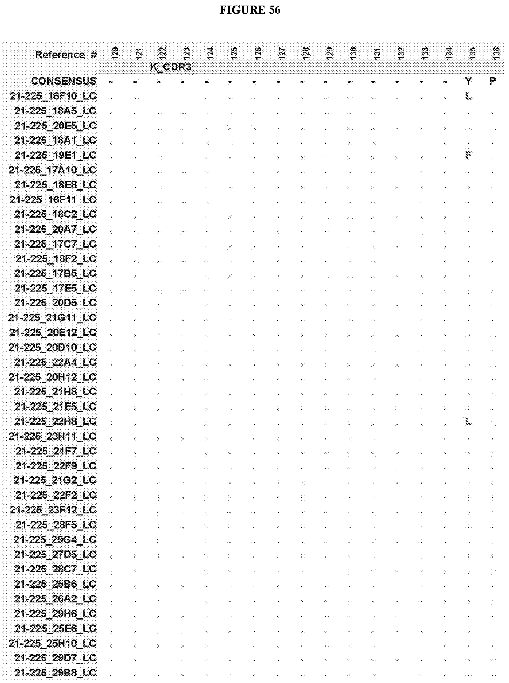

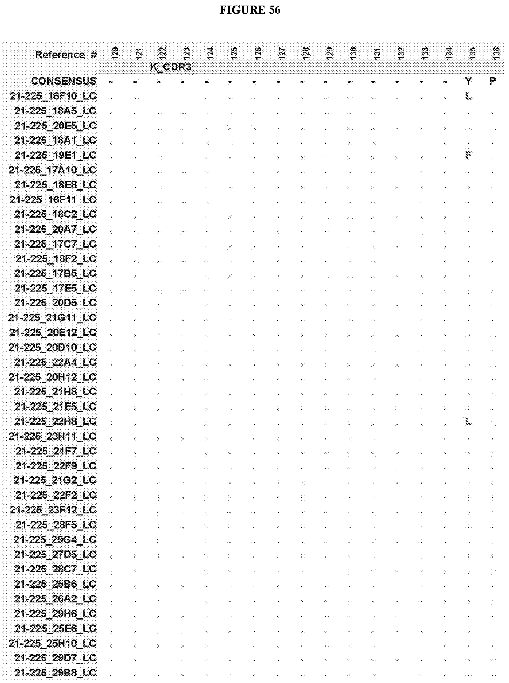

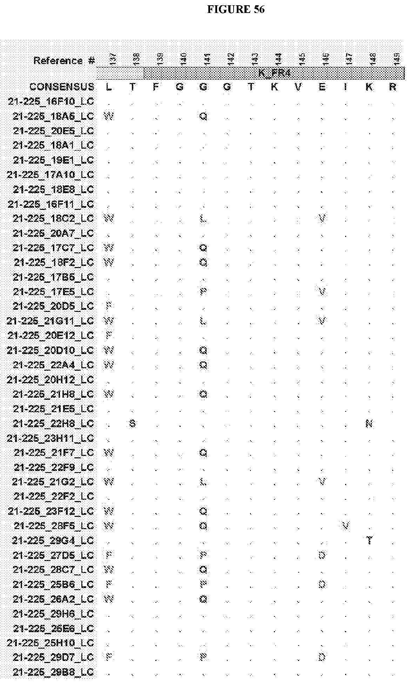

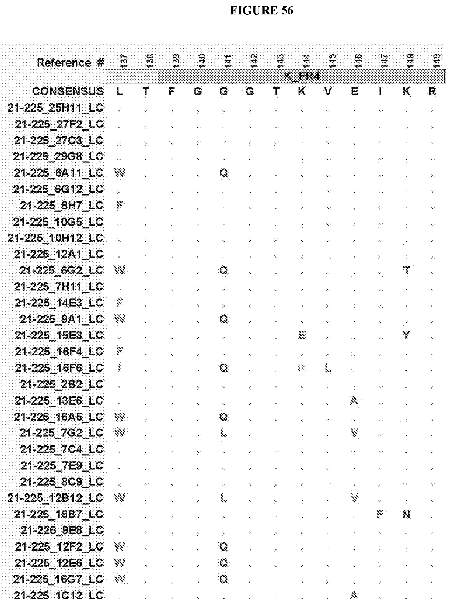

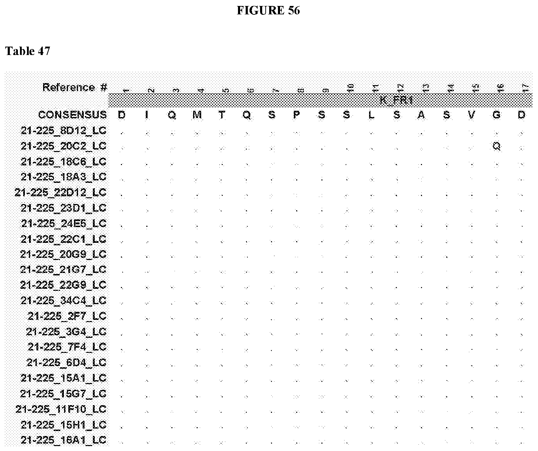

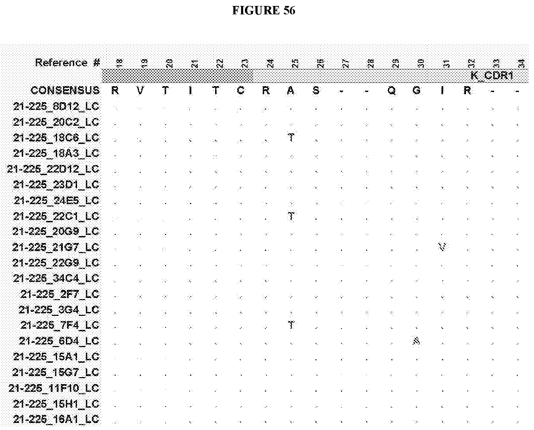

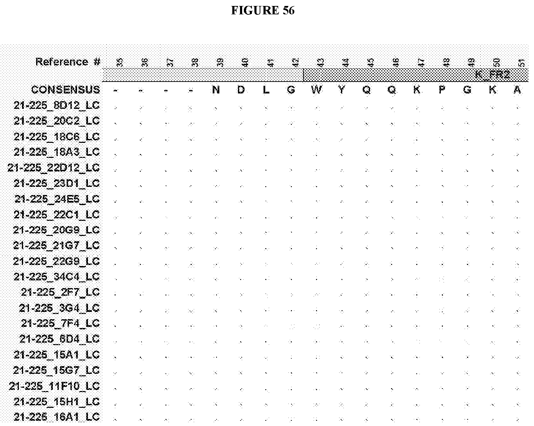

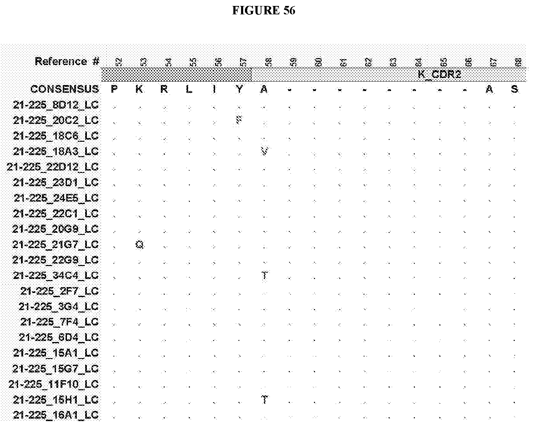

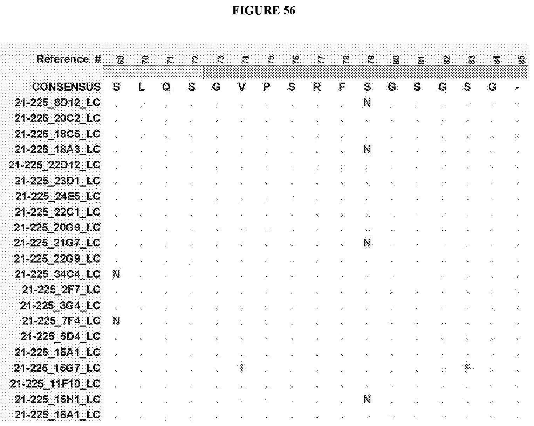

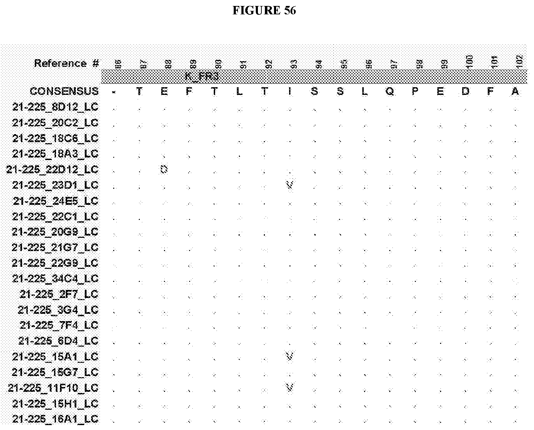

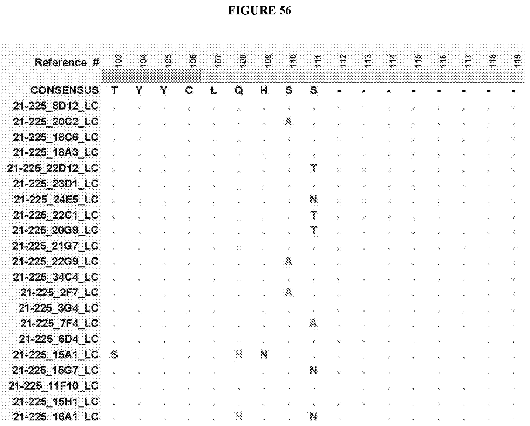

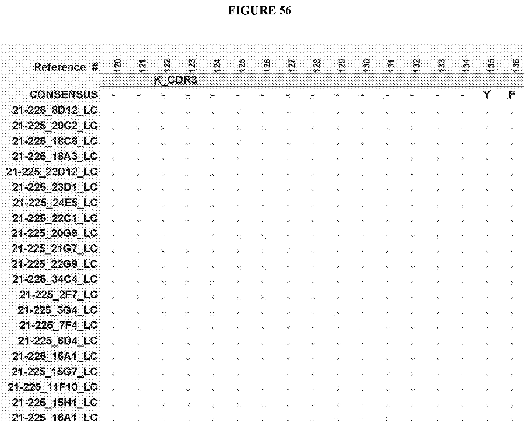

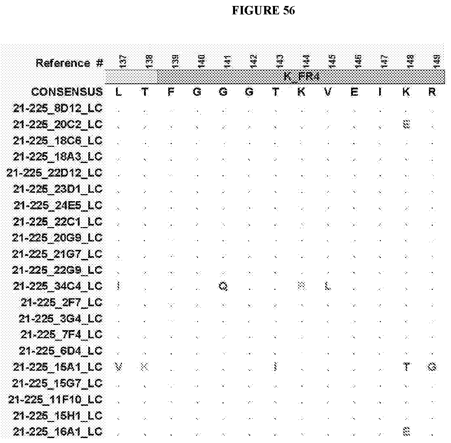

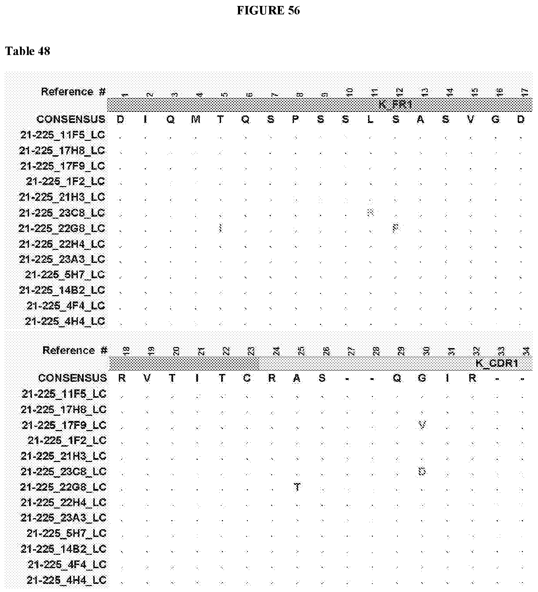

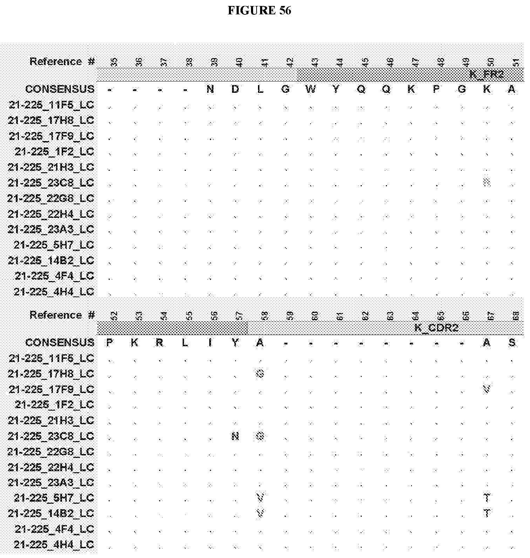

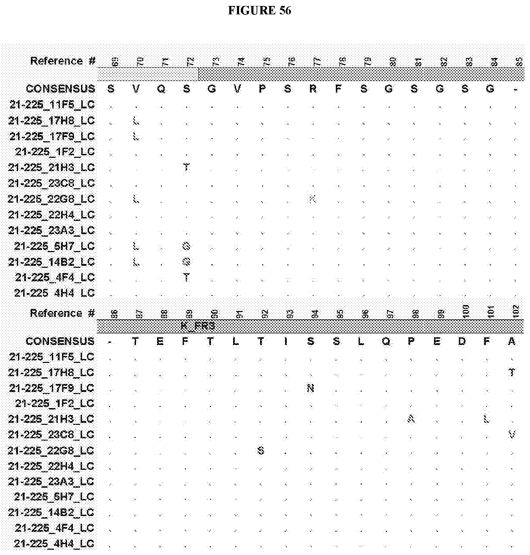

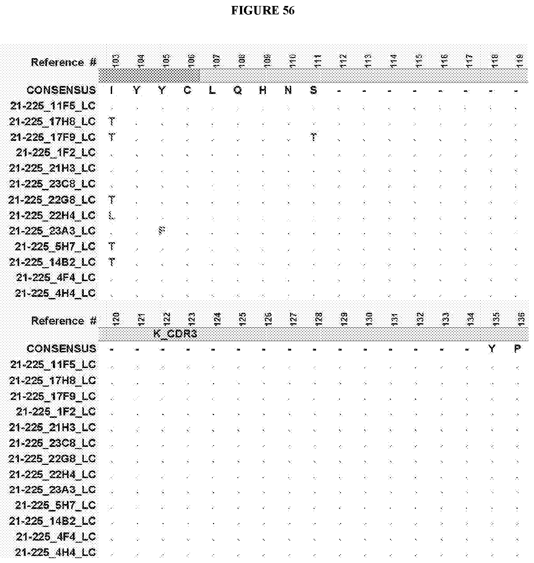

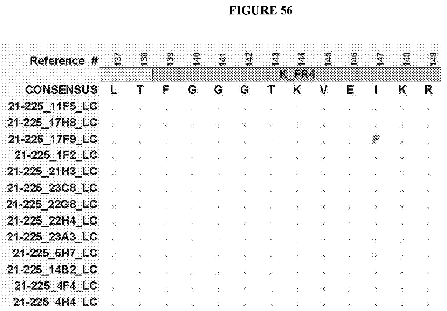

**Figure 56.** A group of tables presenting the detailed consensus protein alignment of various light and heavy chain variable regions for certain antigen binding proteins (Tables 21-48). The shading of amino acid residues in the consensus protein alignment presented in Tables 21-48 denote particular residues that one of ordinary skill in the art may wish to target for engineering.

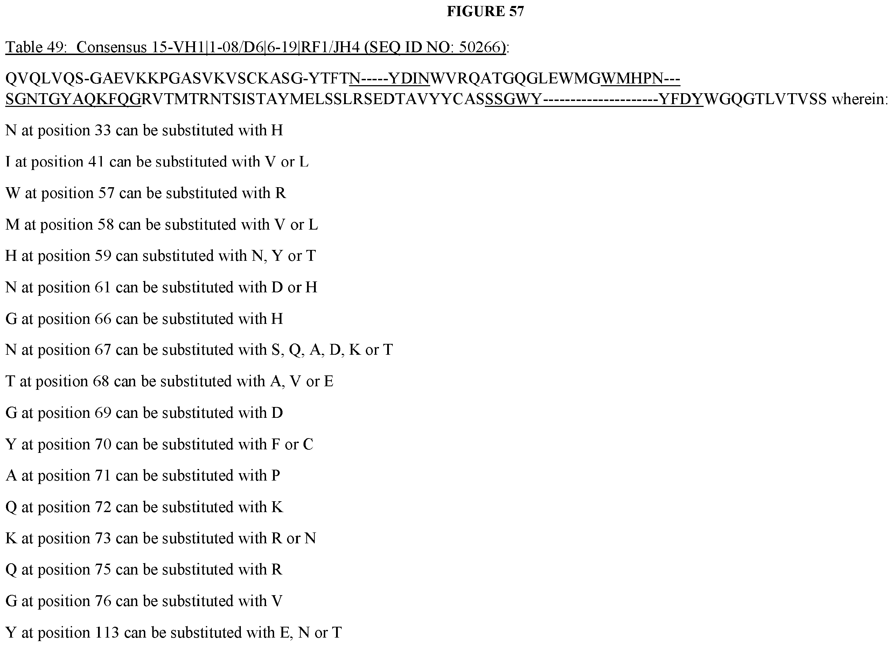

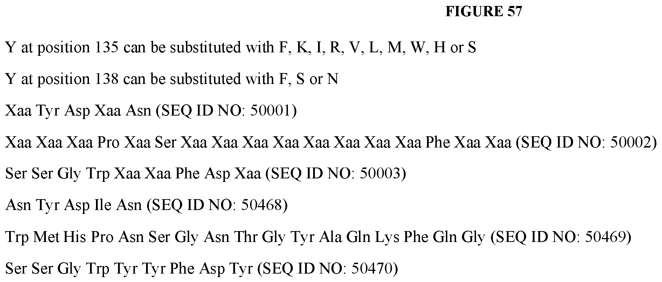

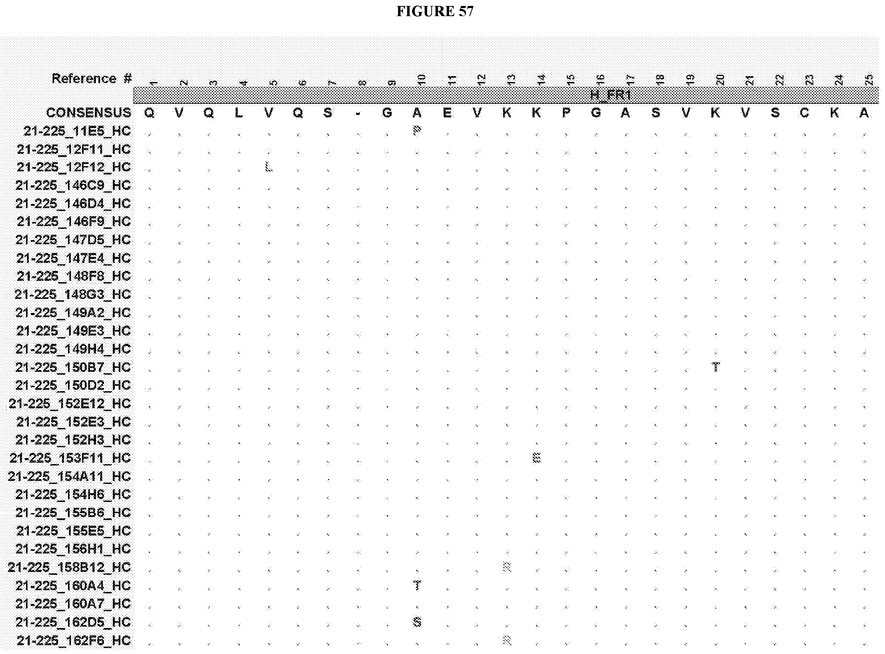

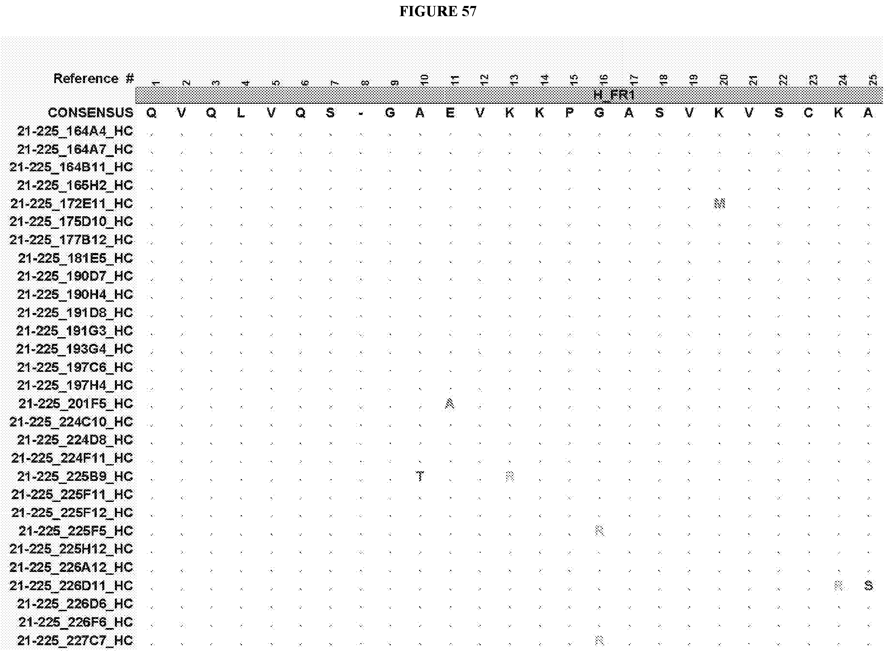

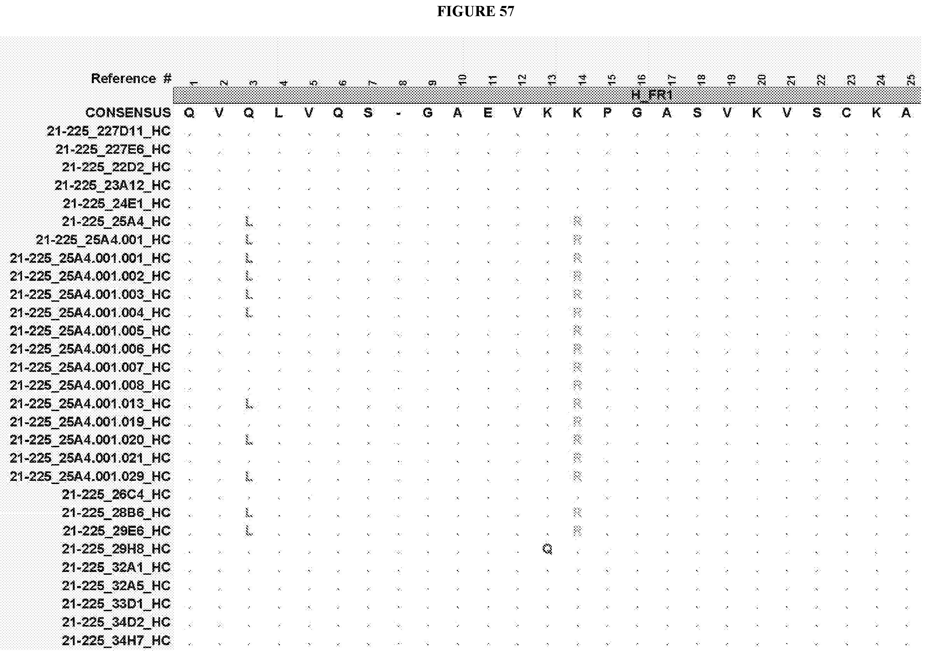

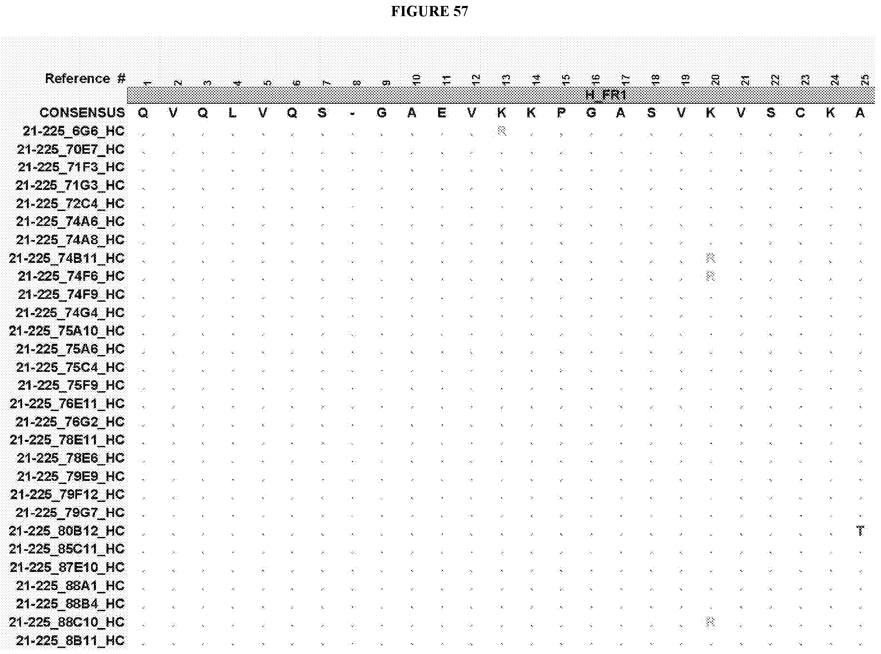

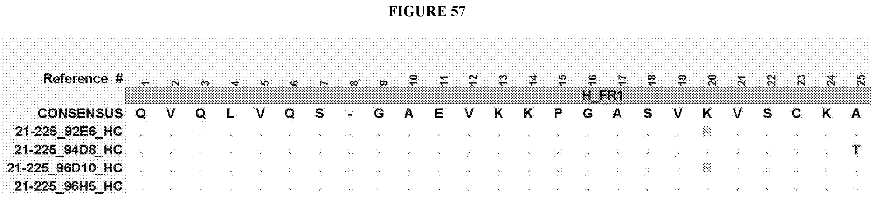

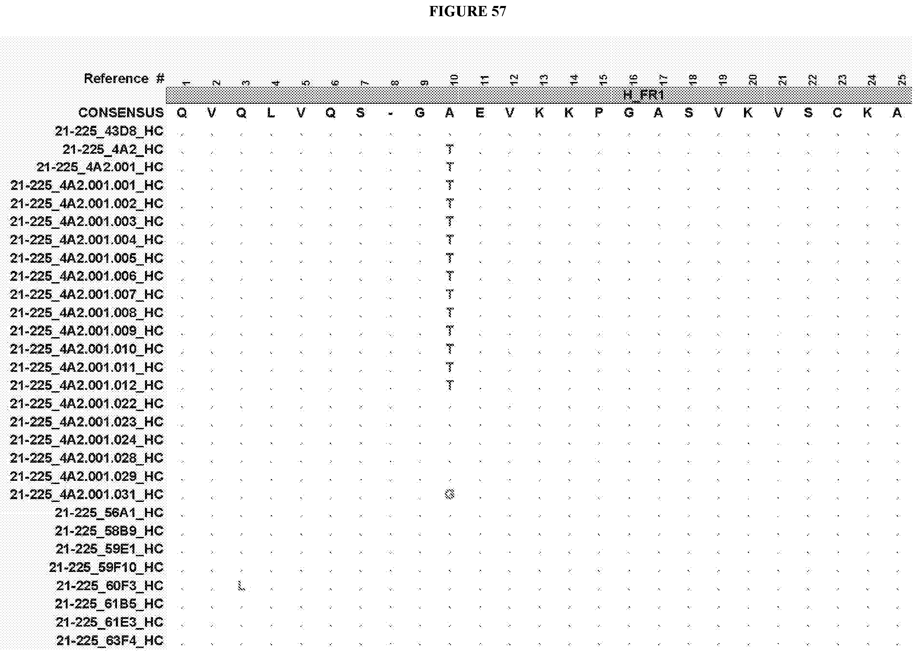

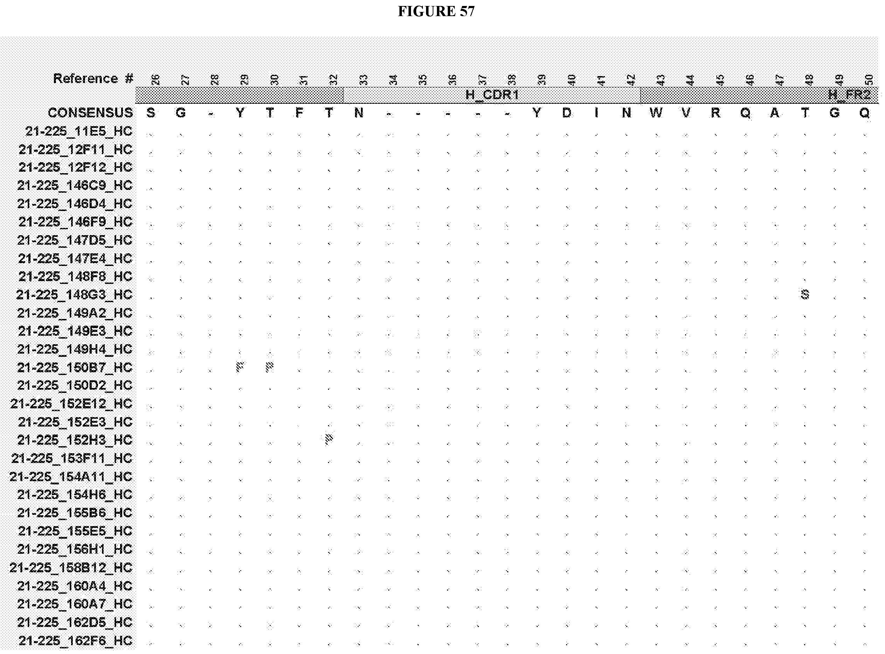

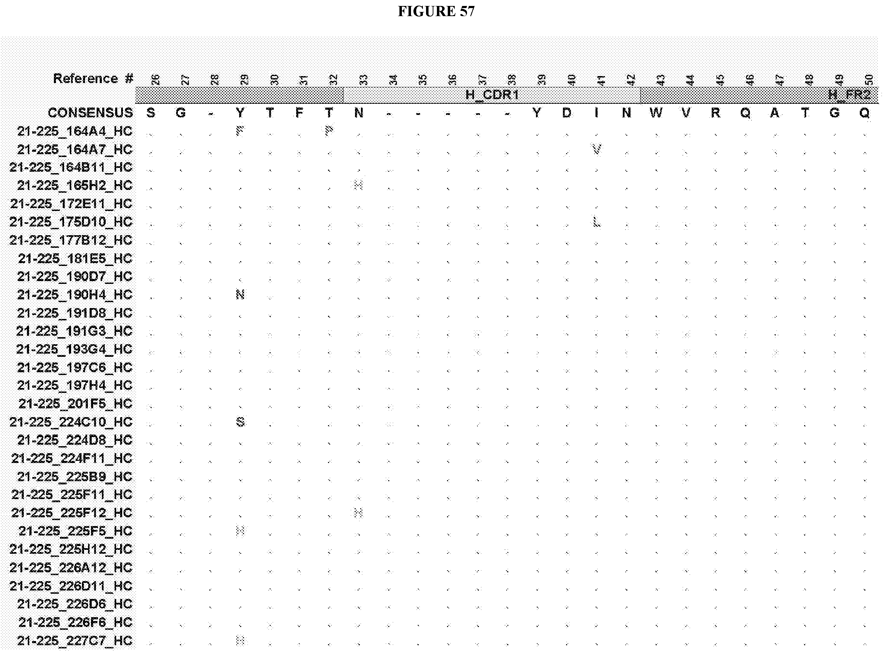

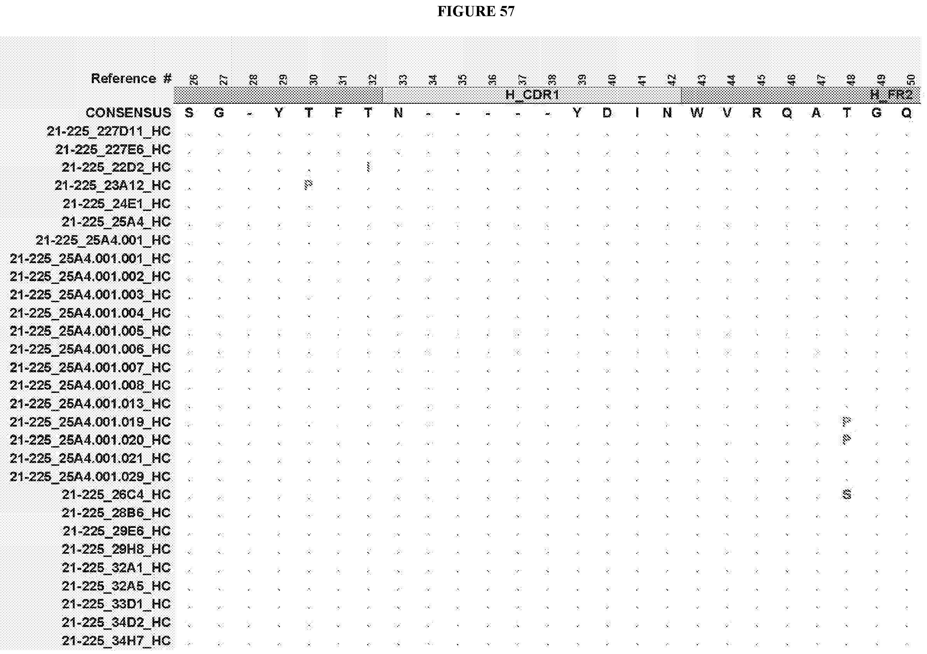

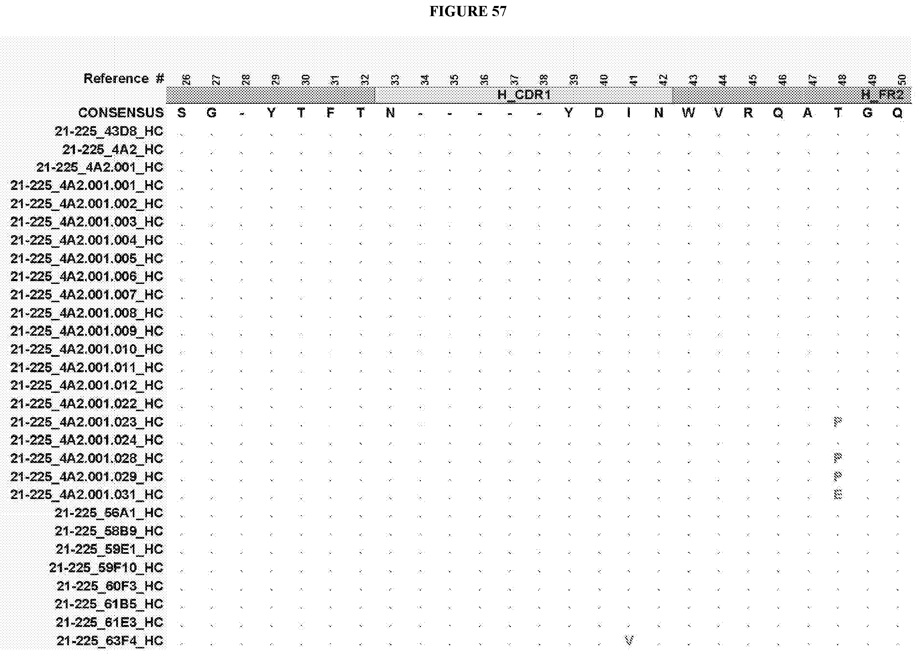

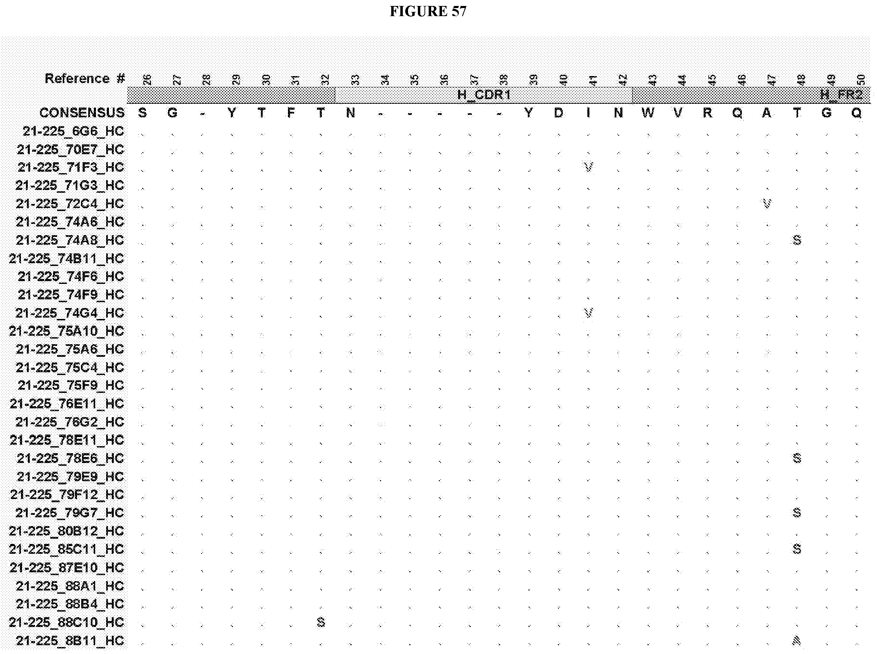

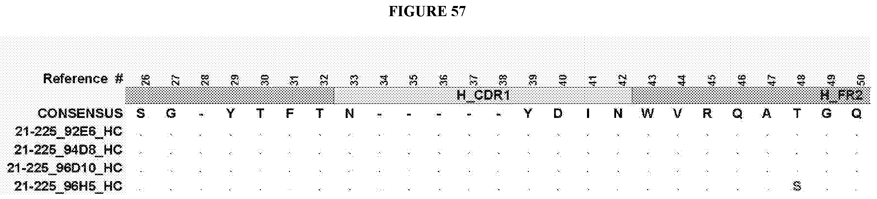

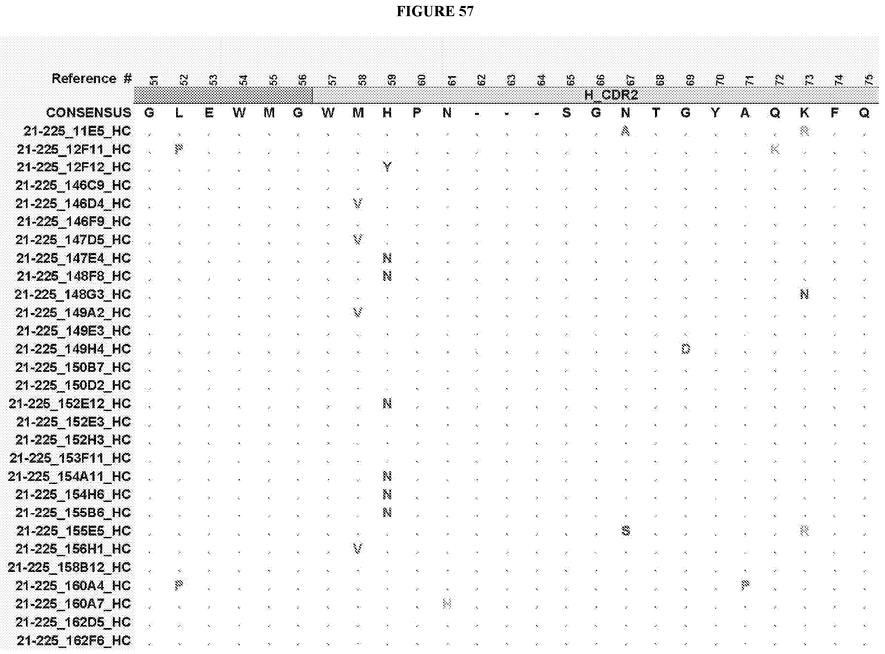

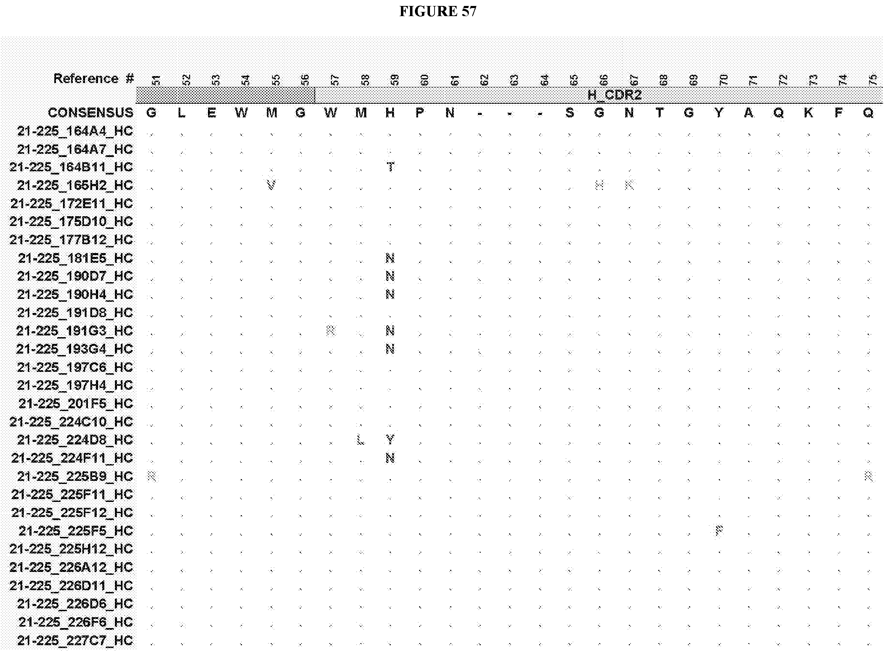

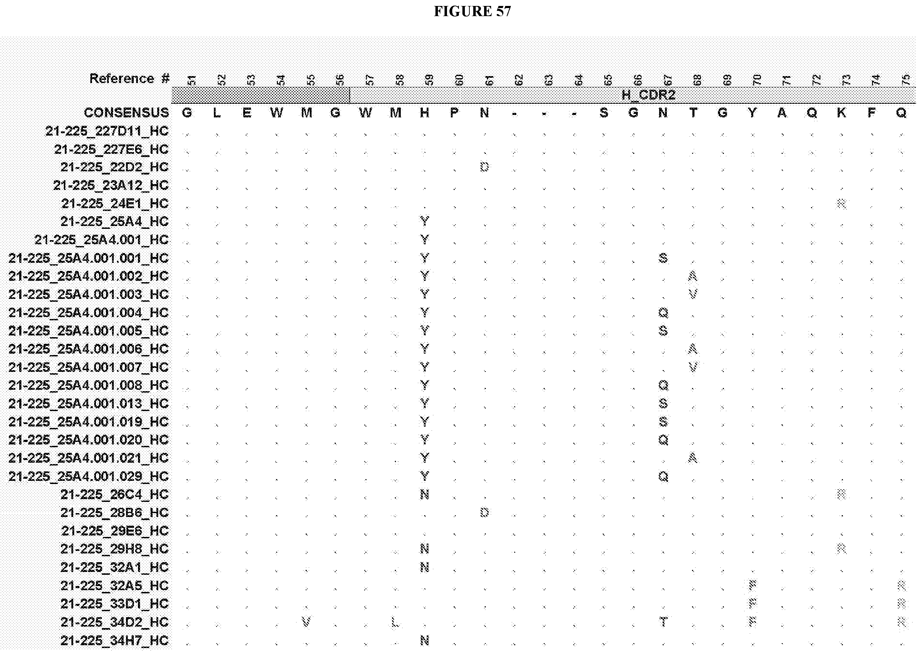

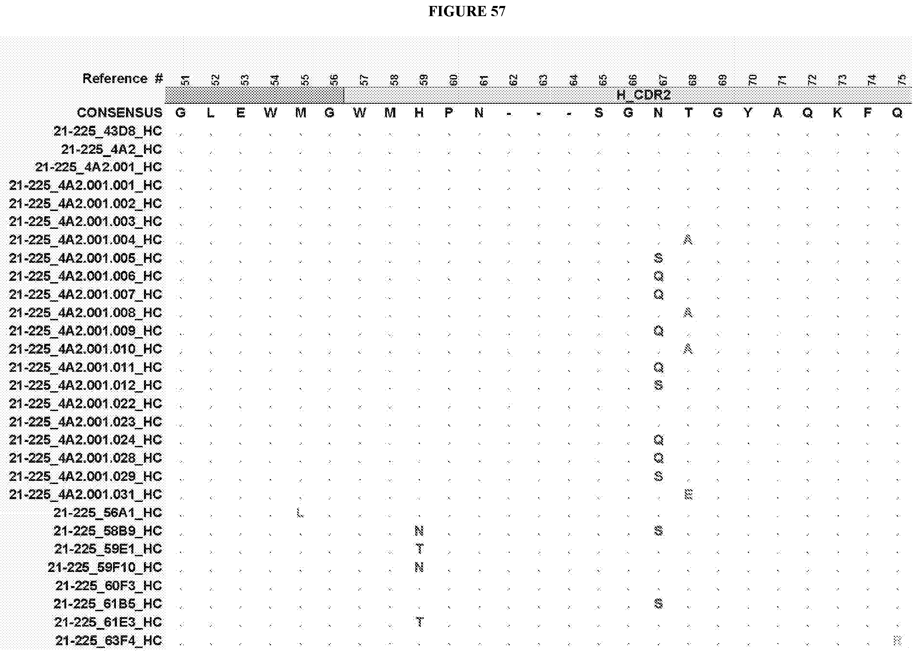

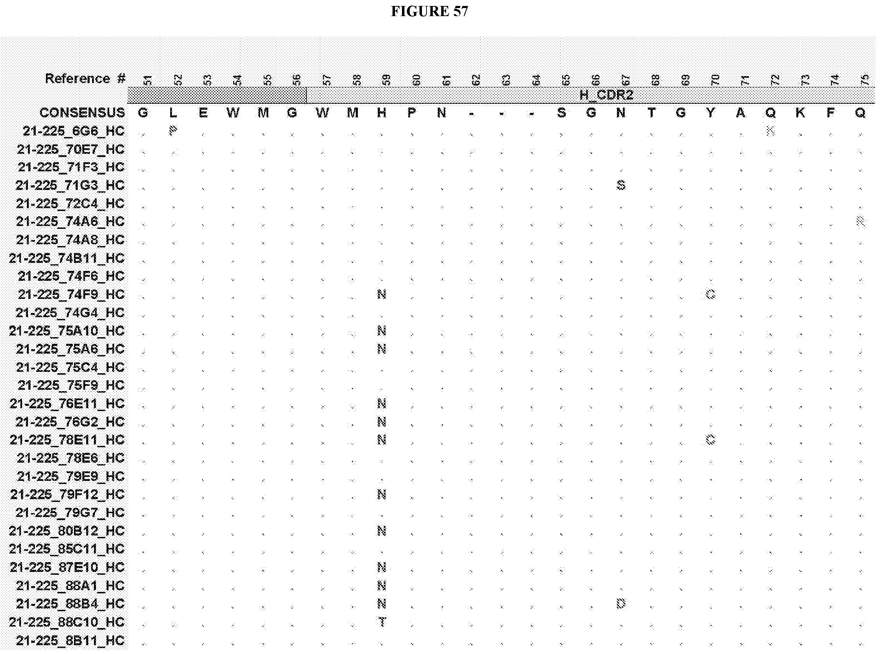

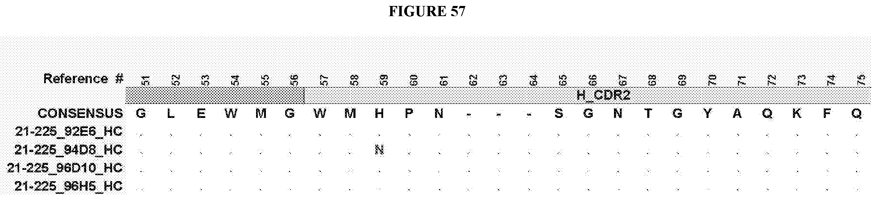

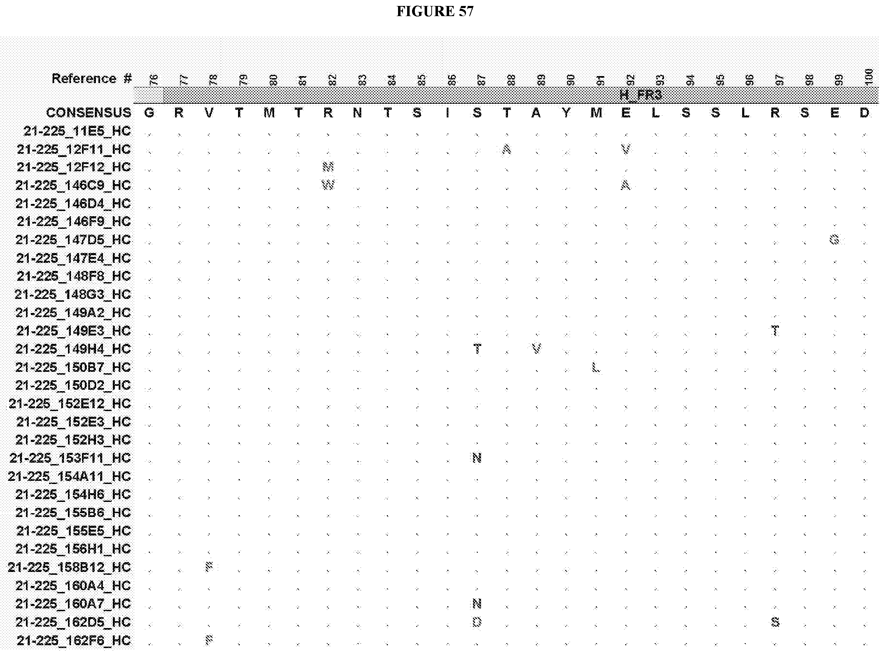

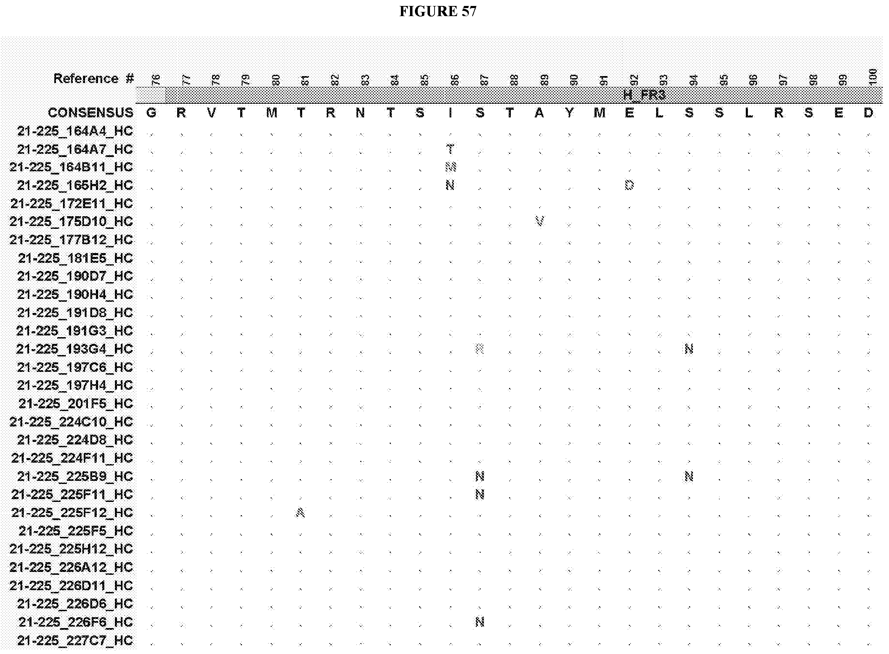

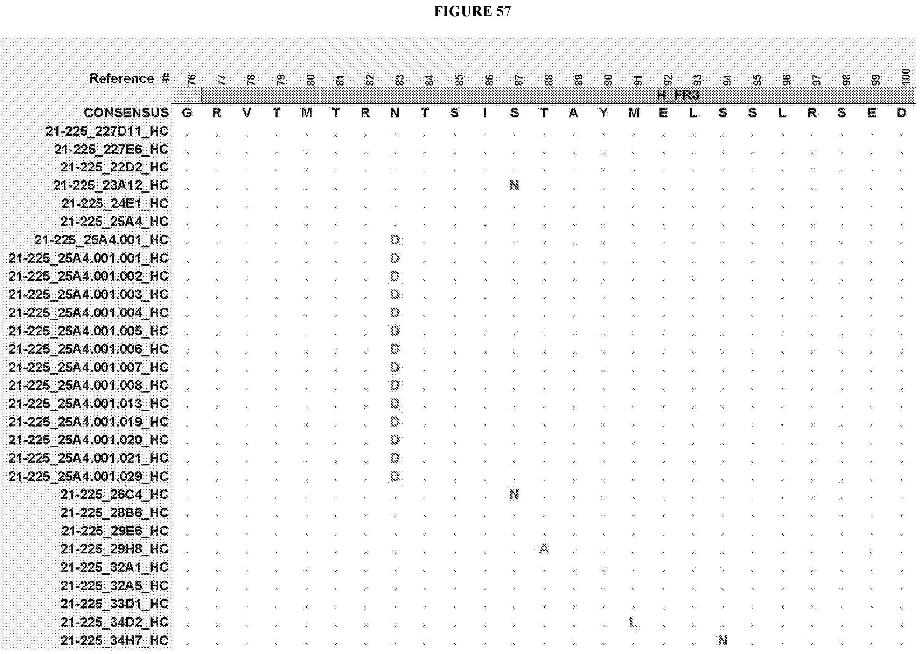

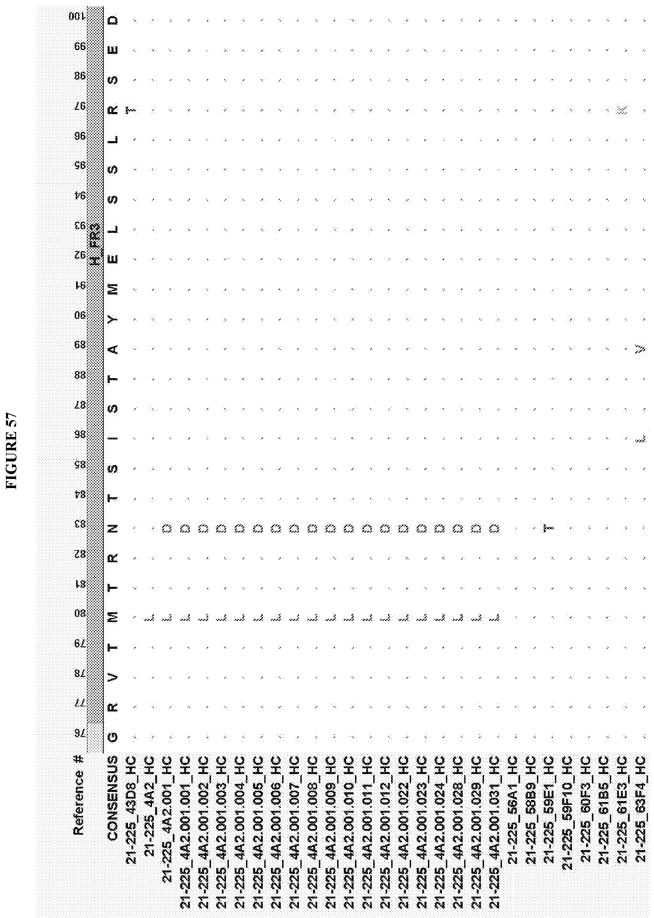

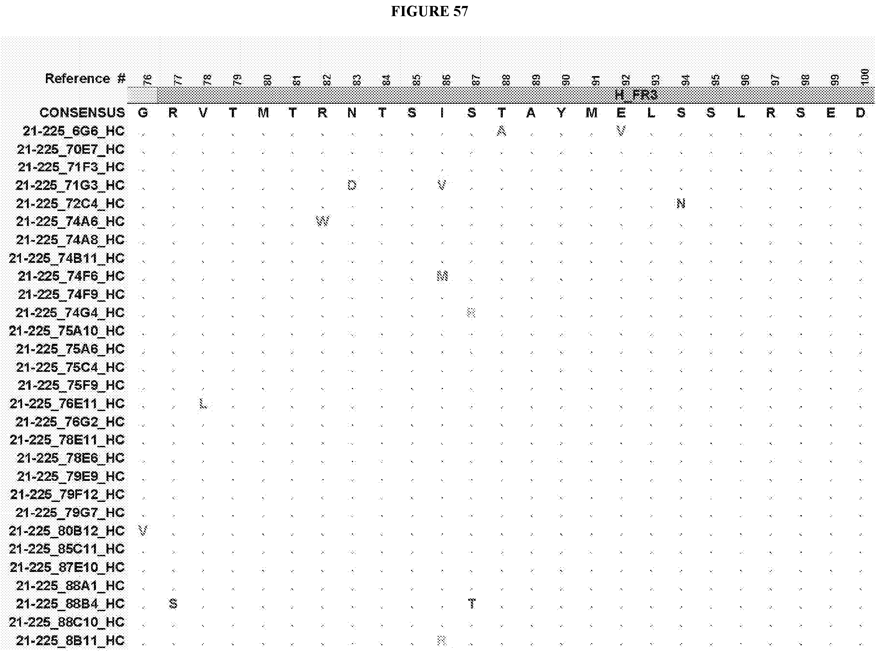

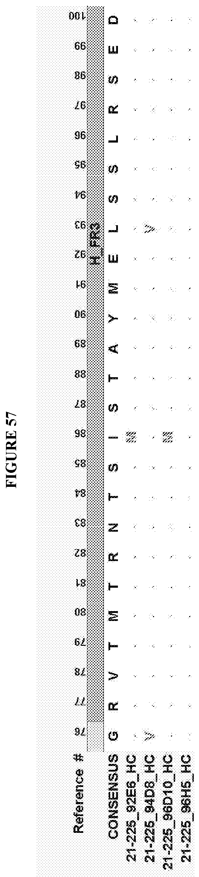

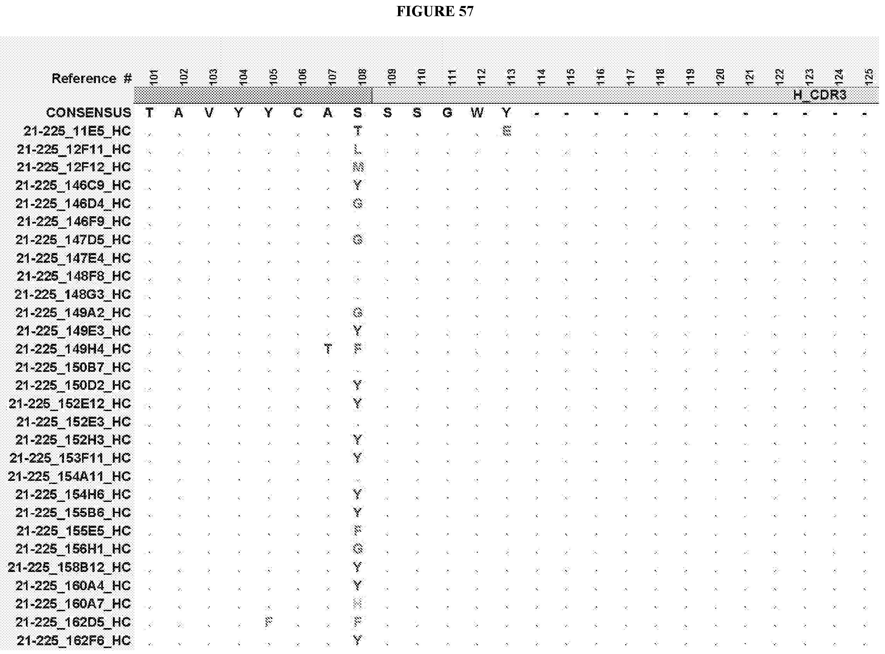

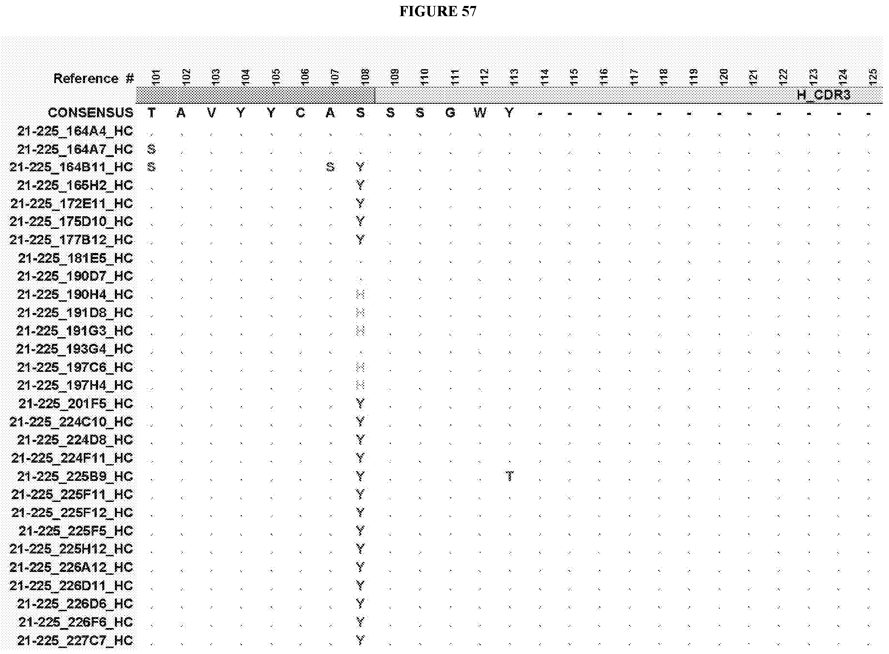

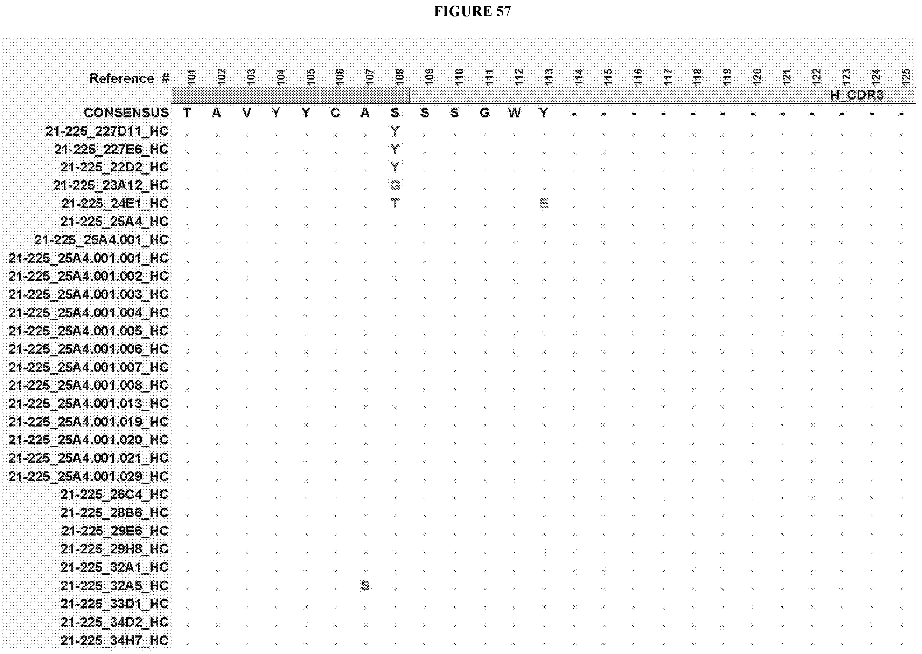

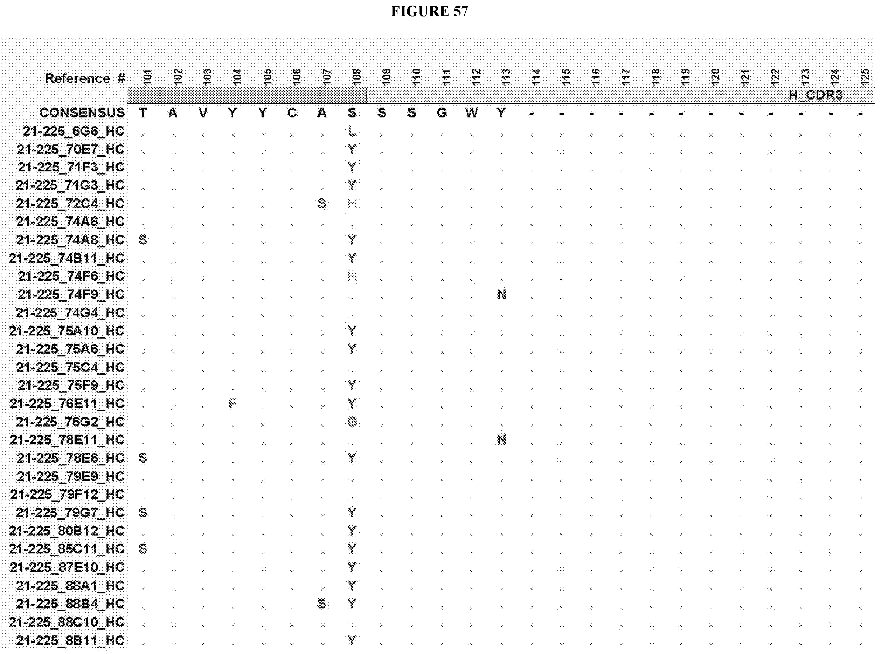

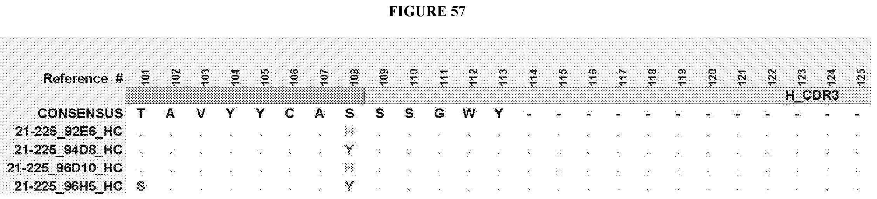

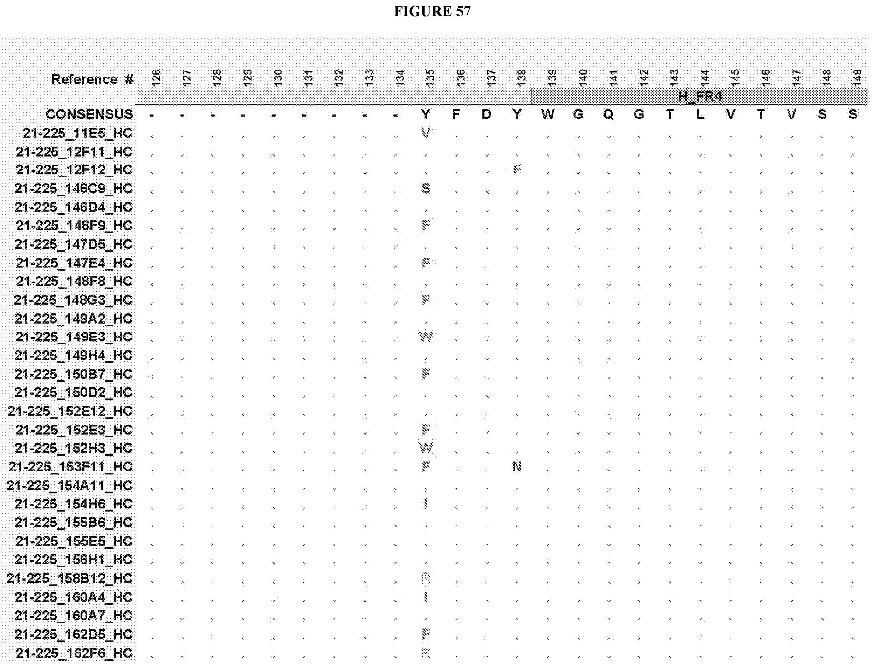

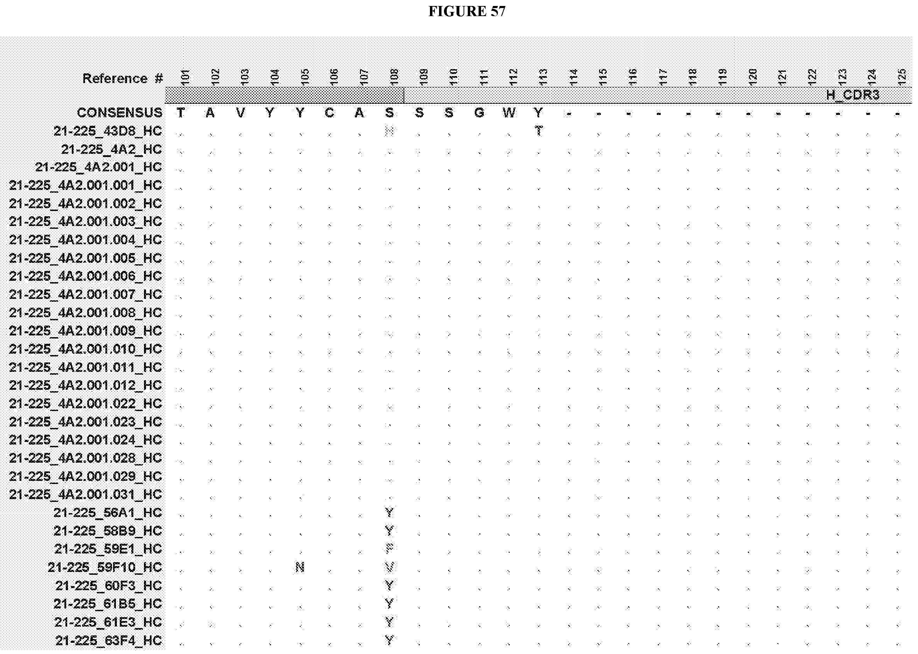

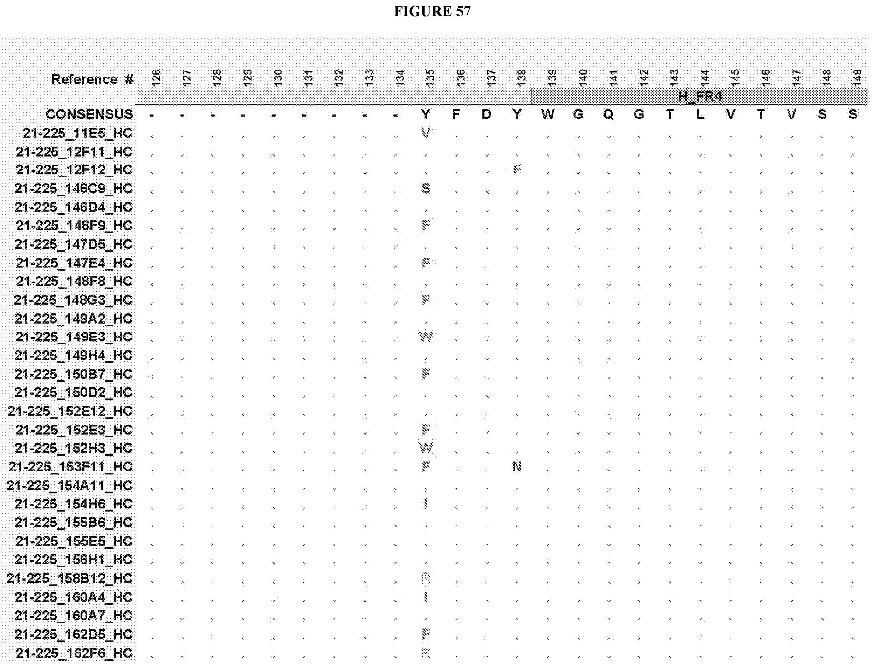

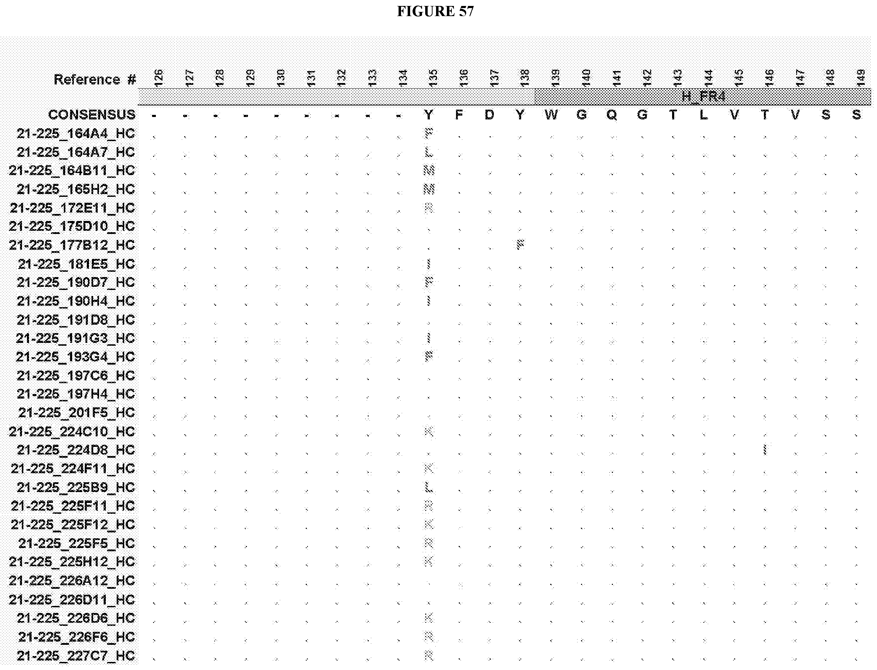

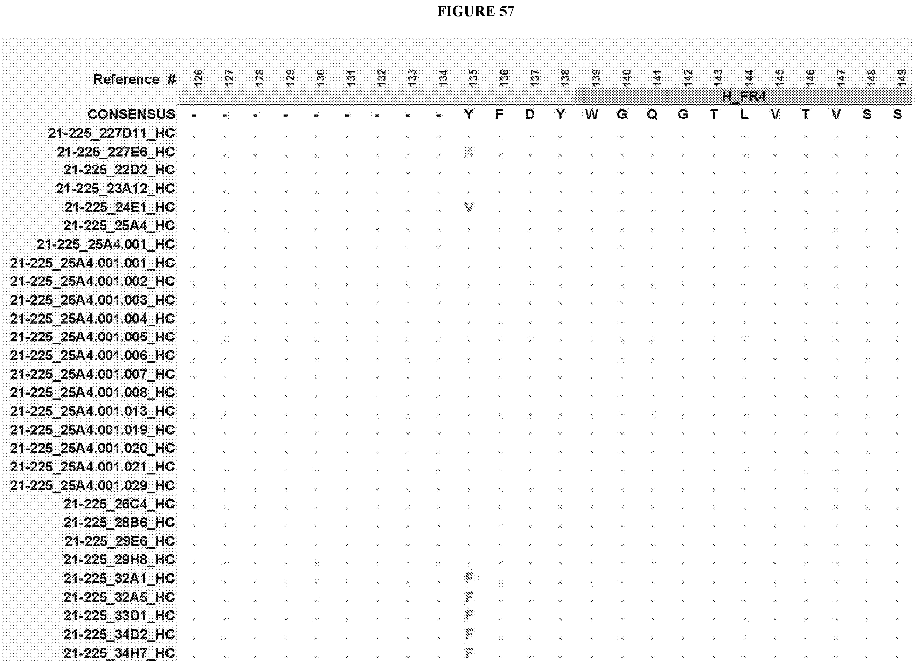

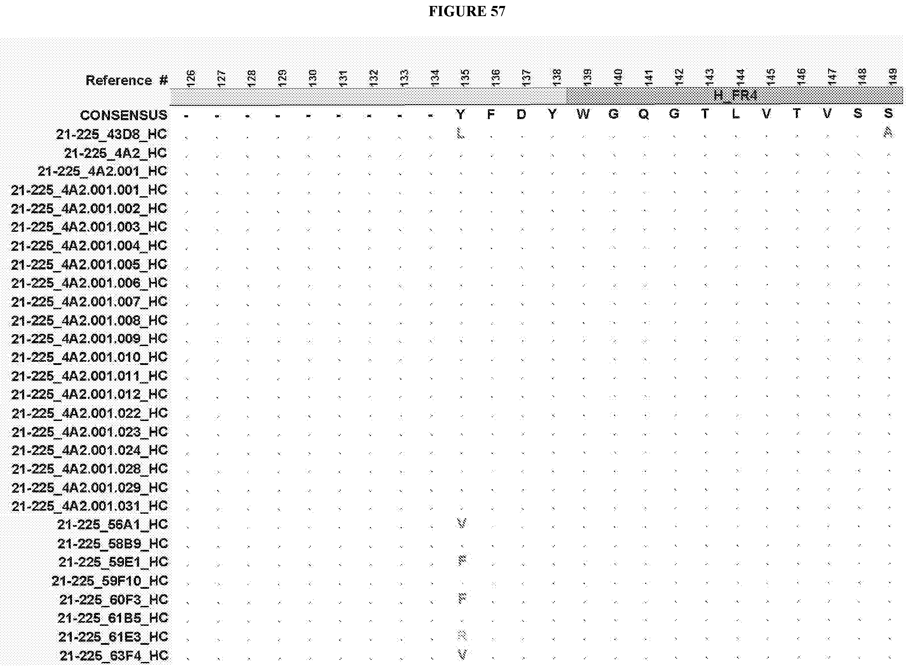

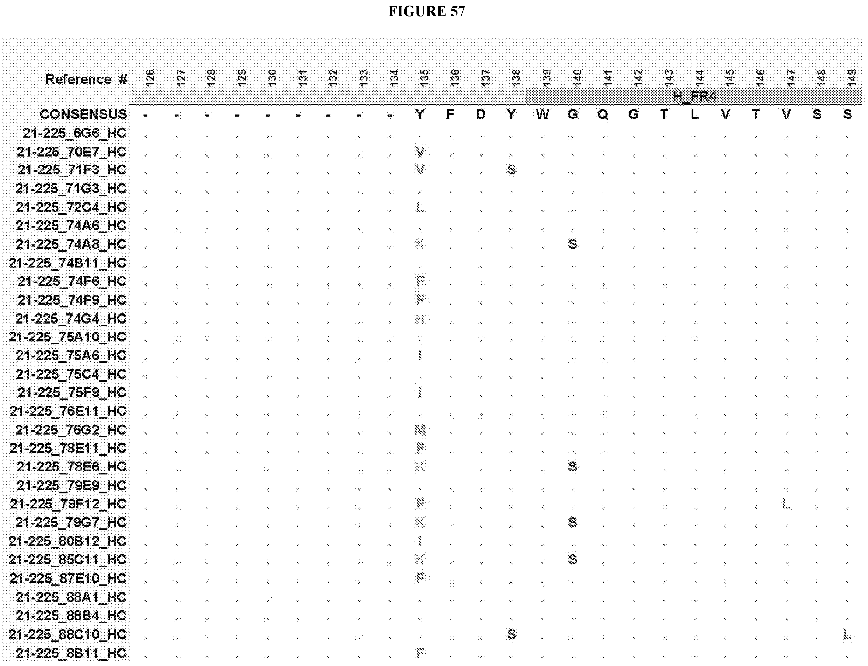

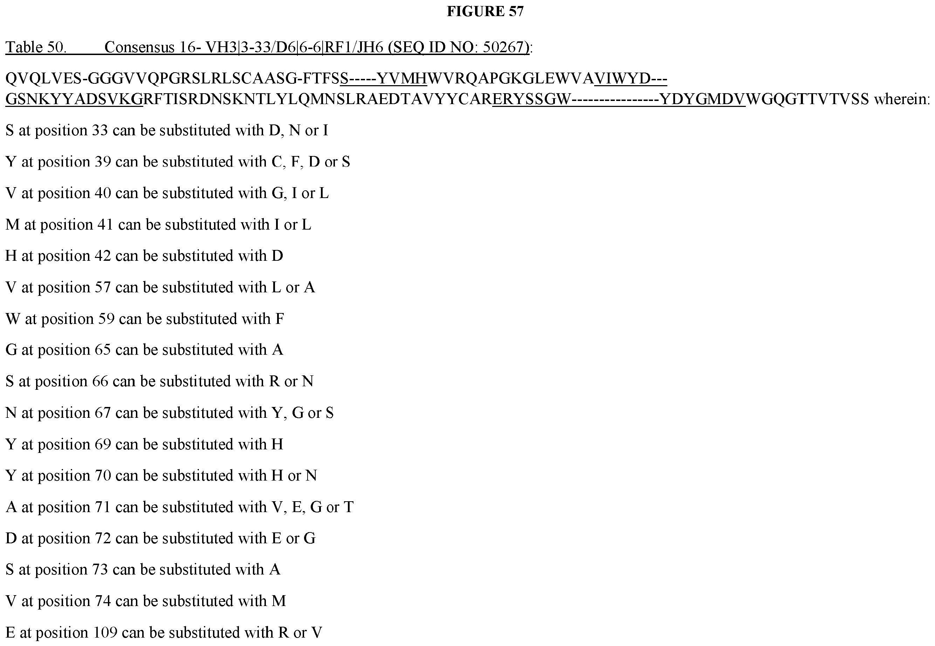

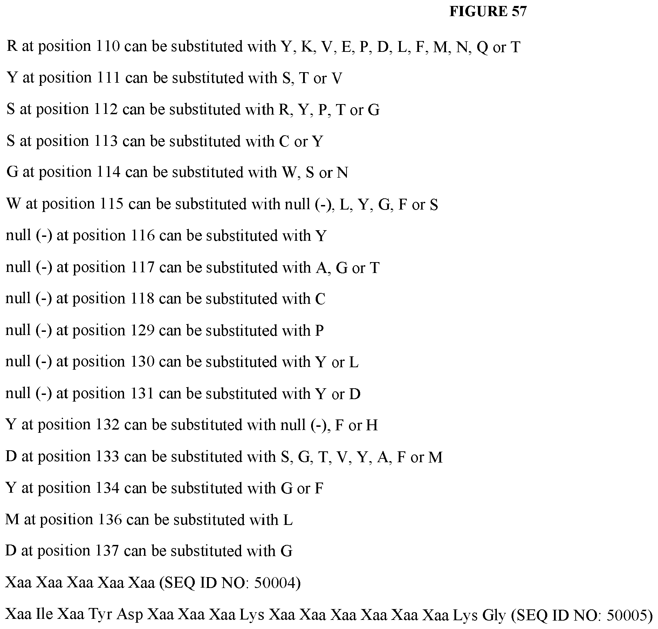

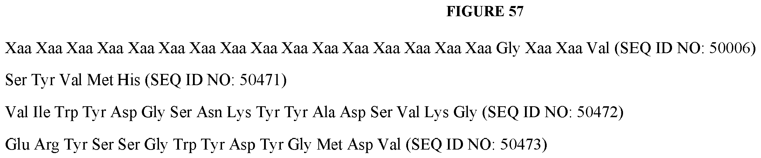

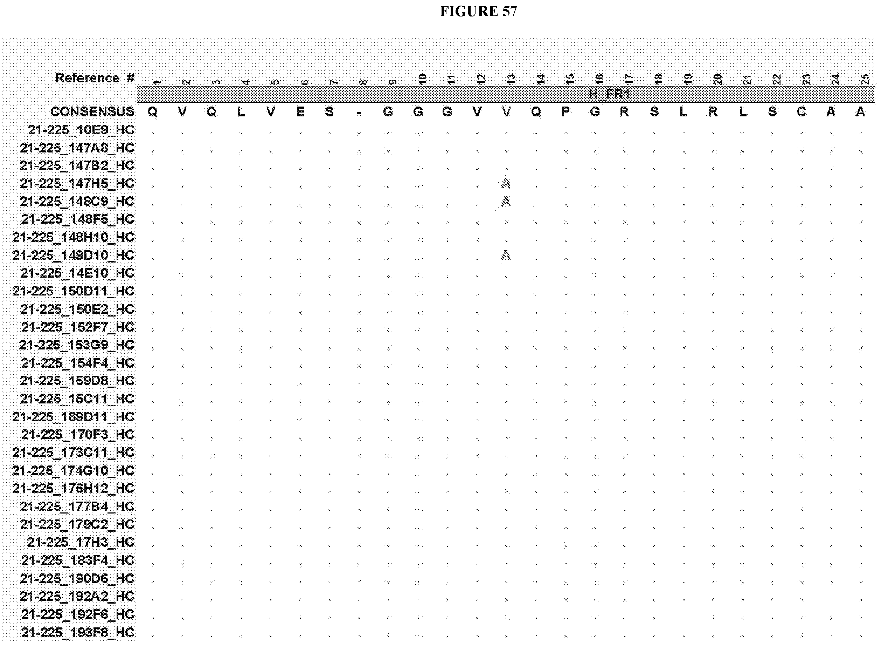

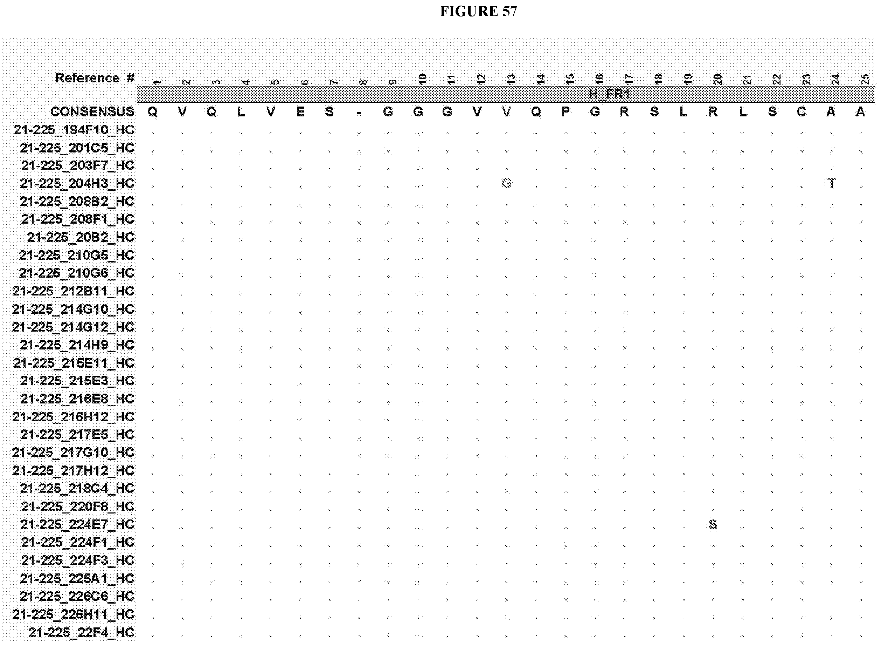

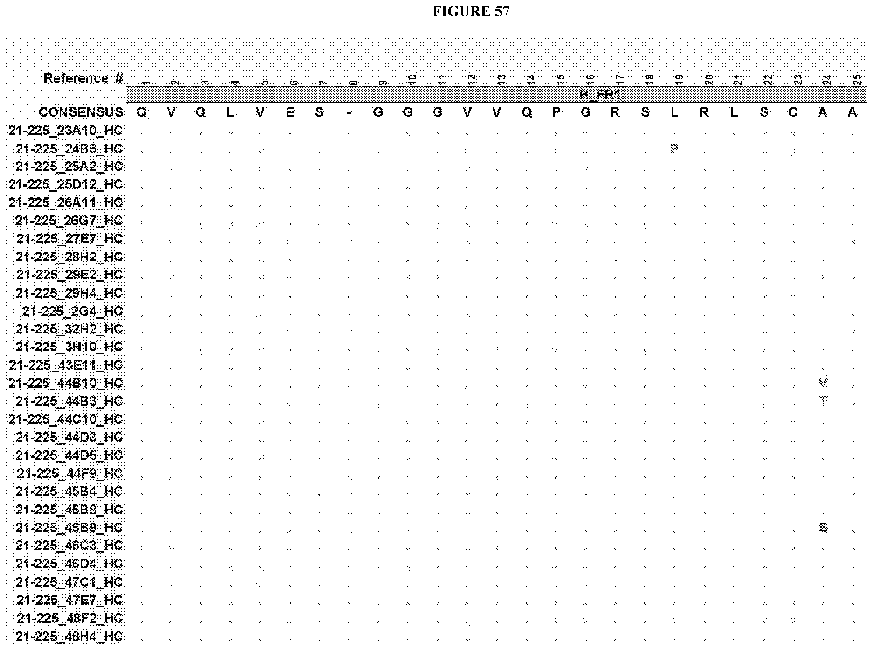

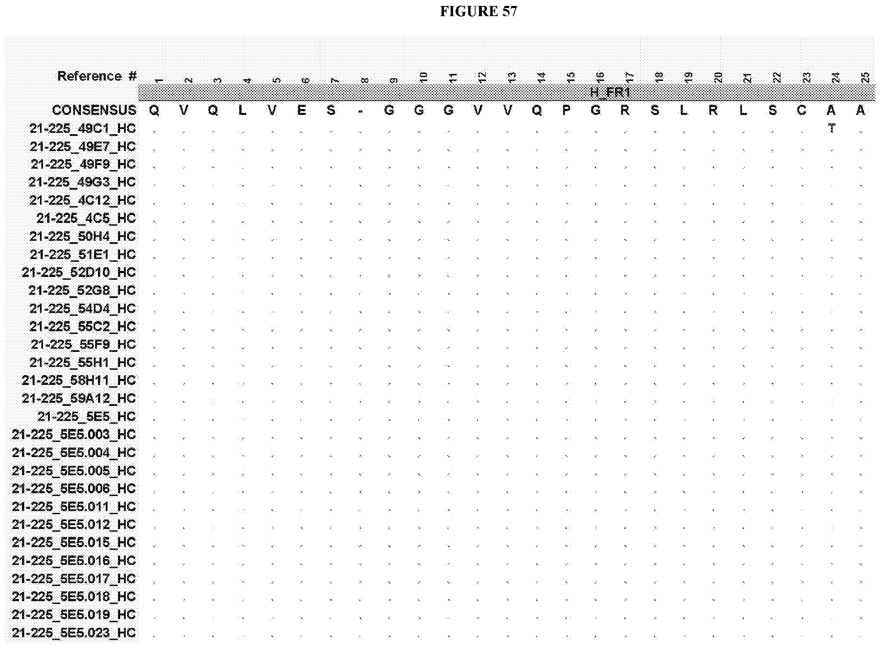

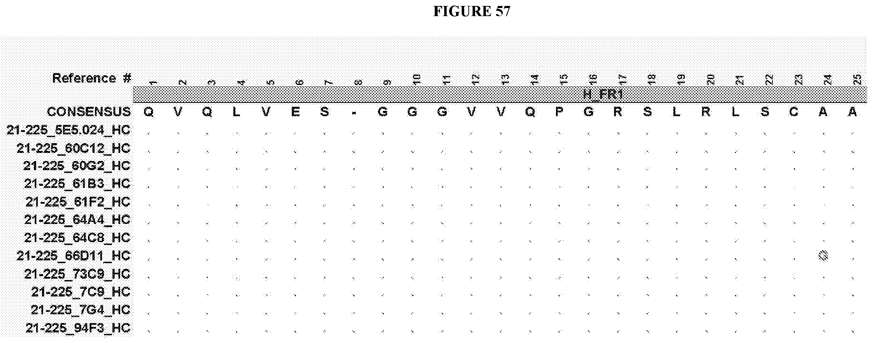

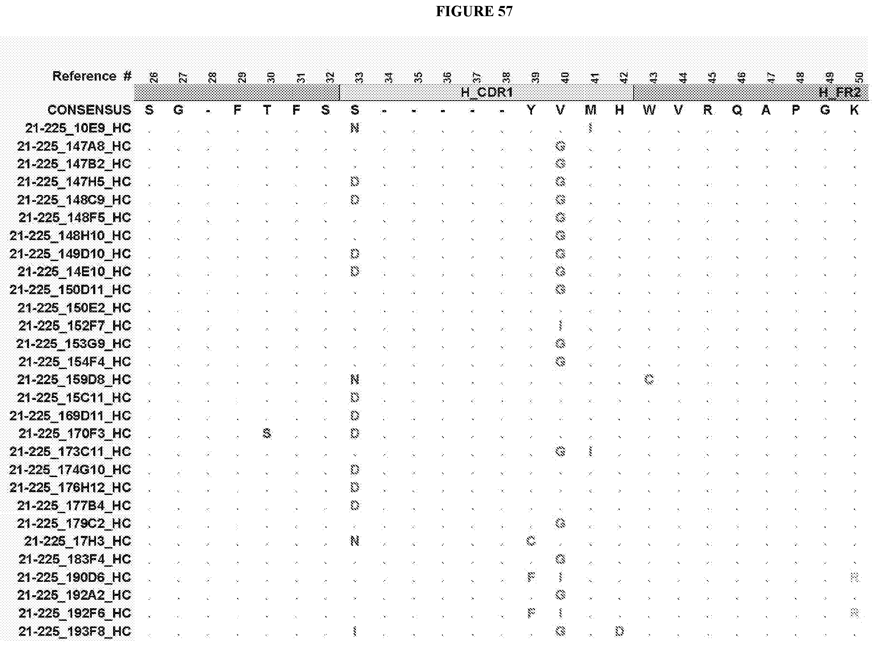

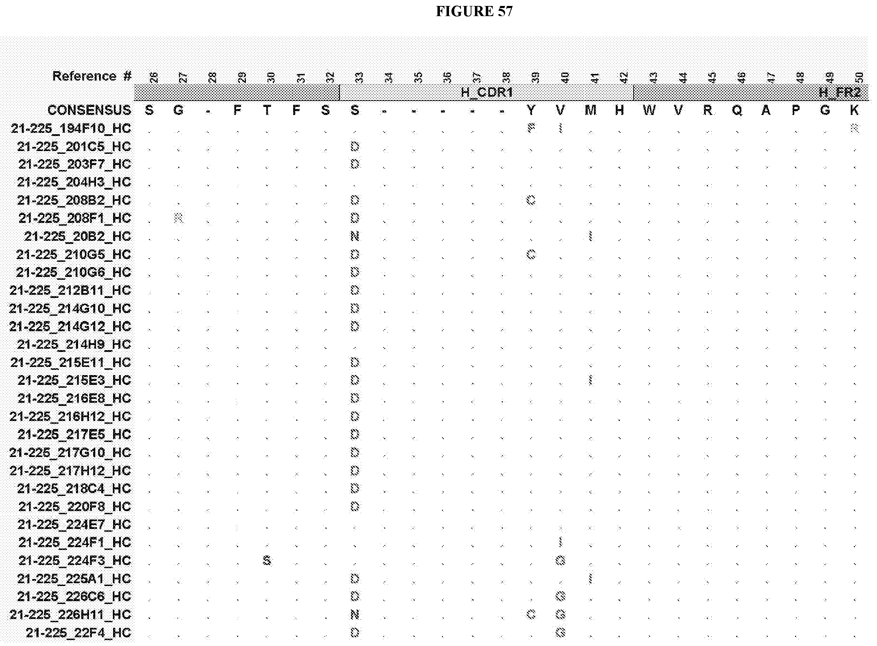

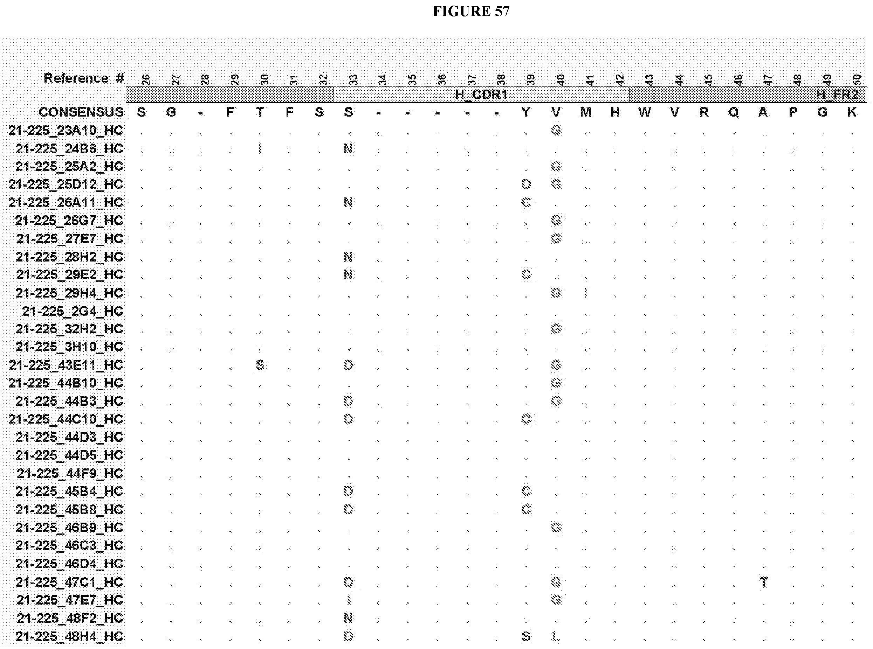

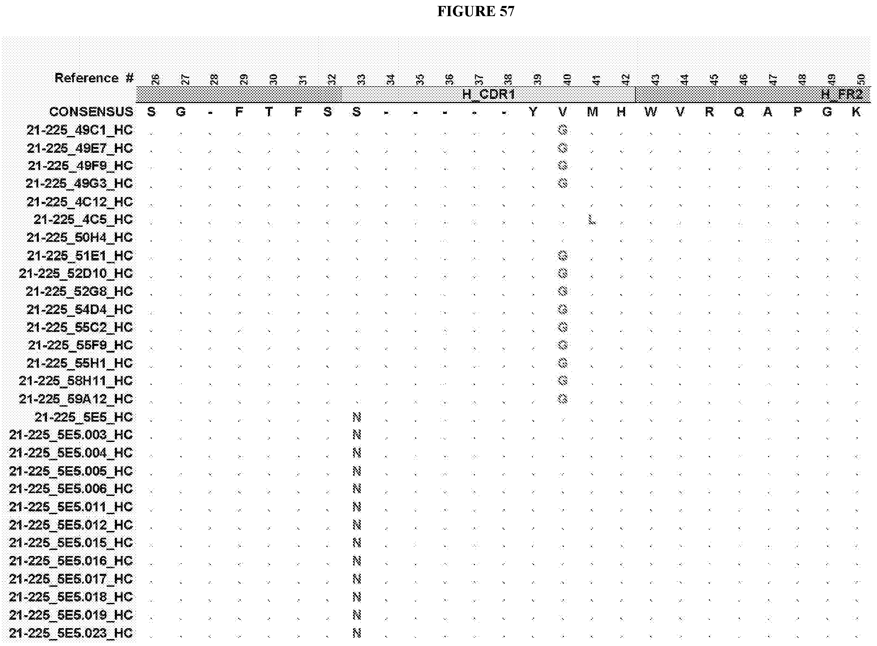

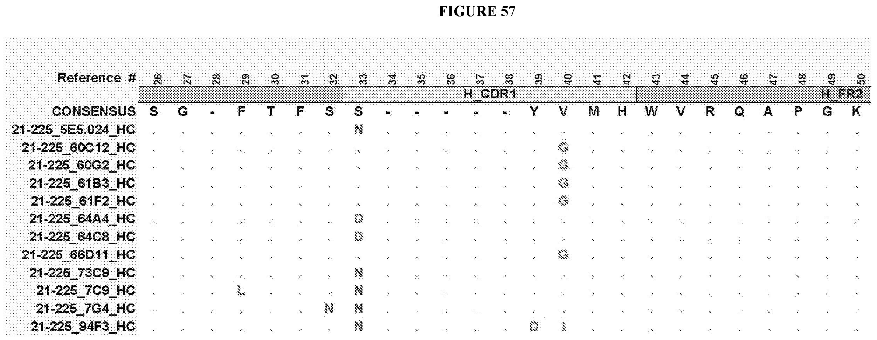

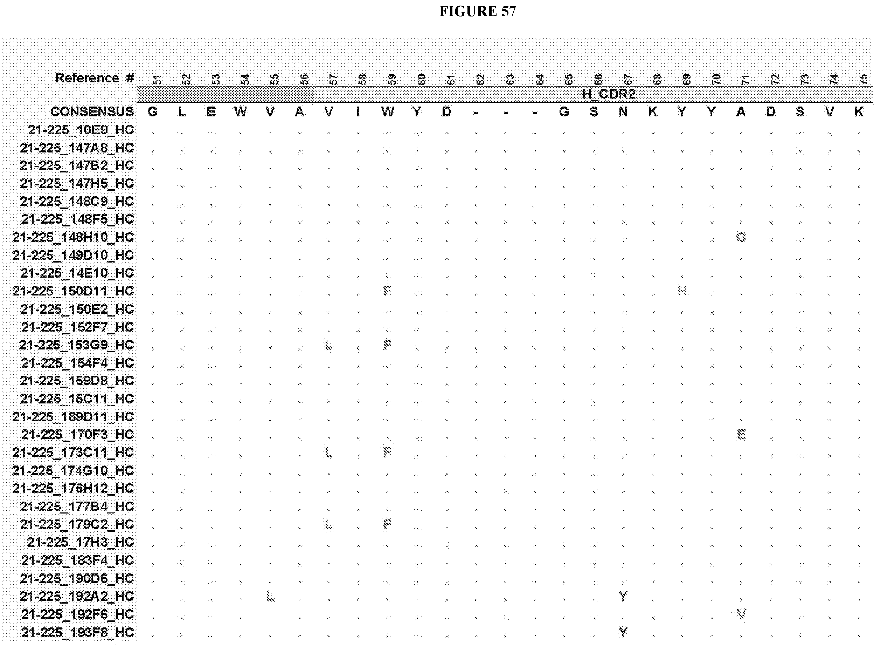

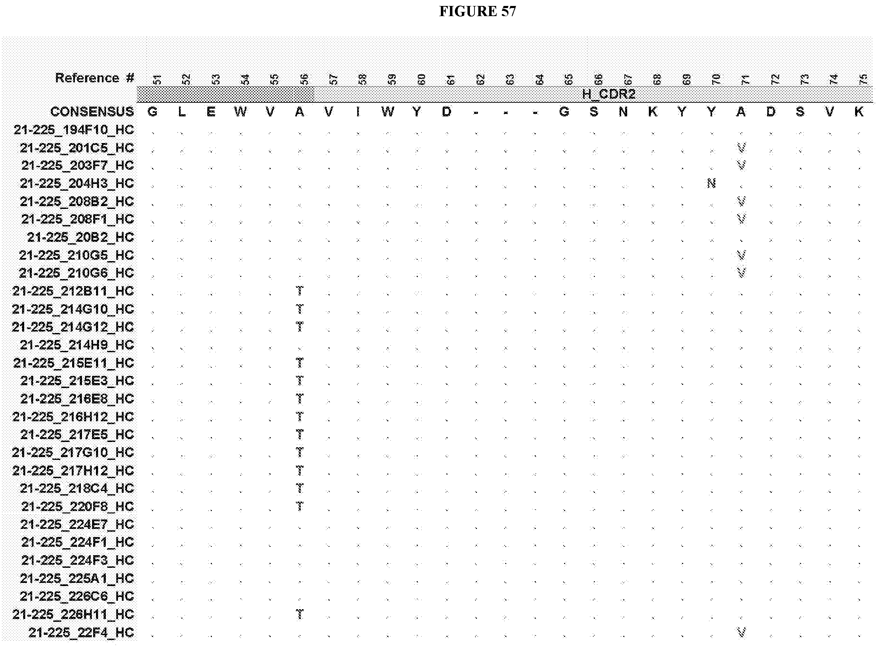

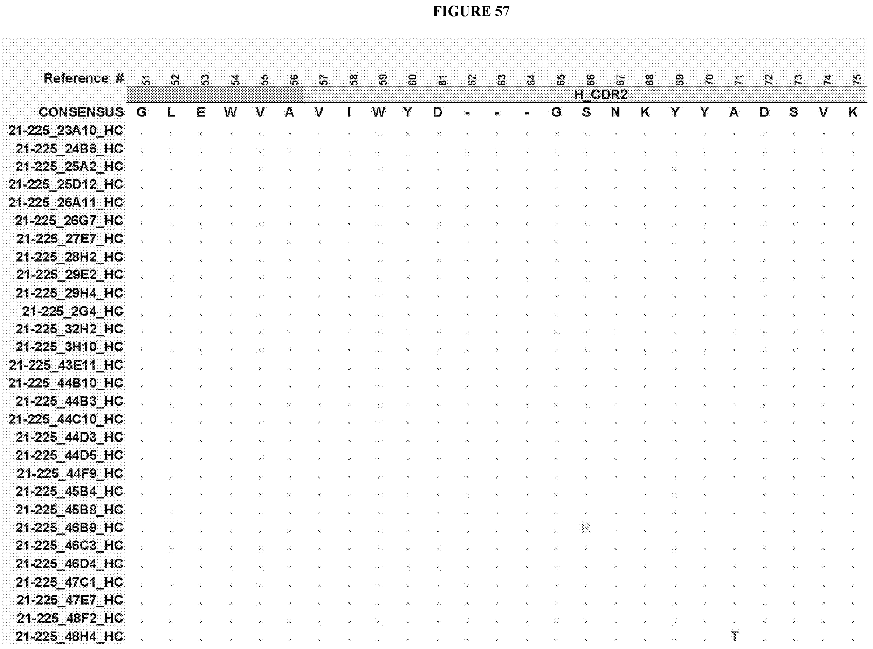

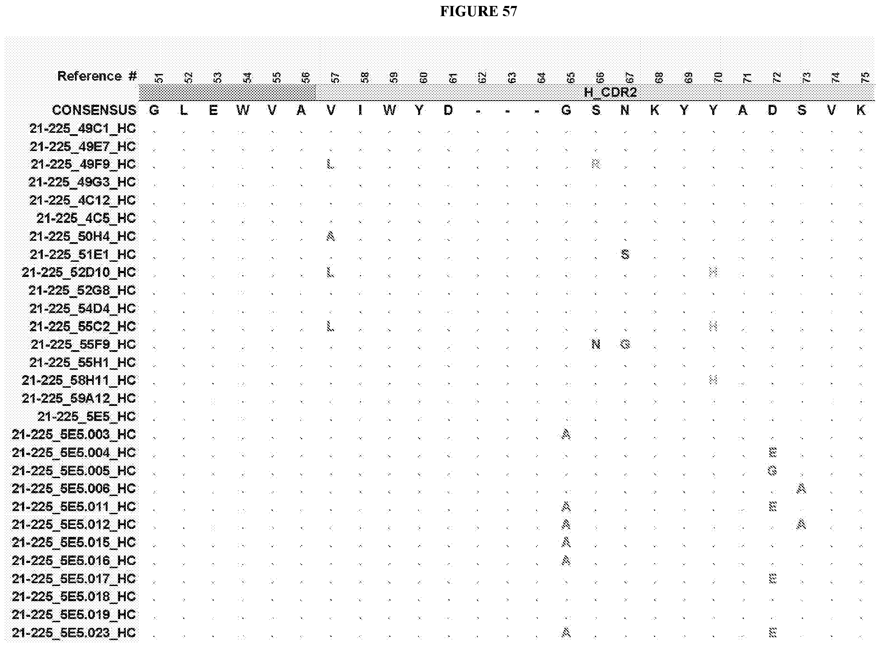

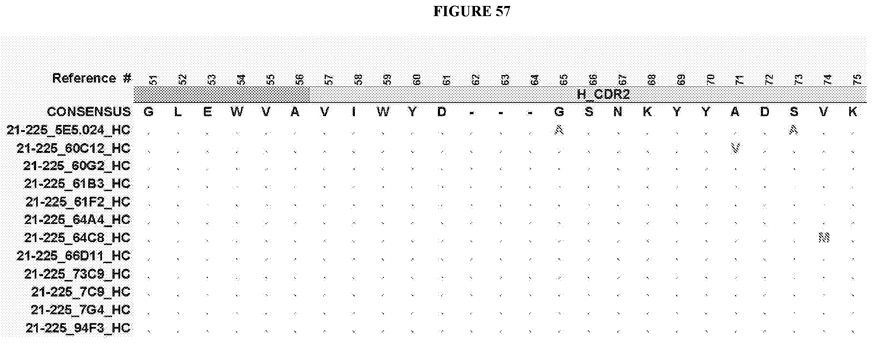

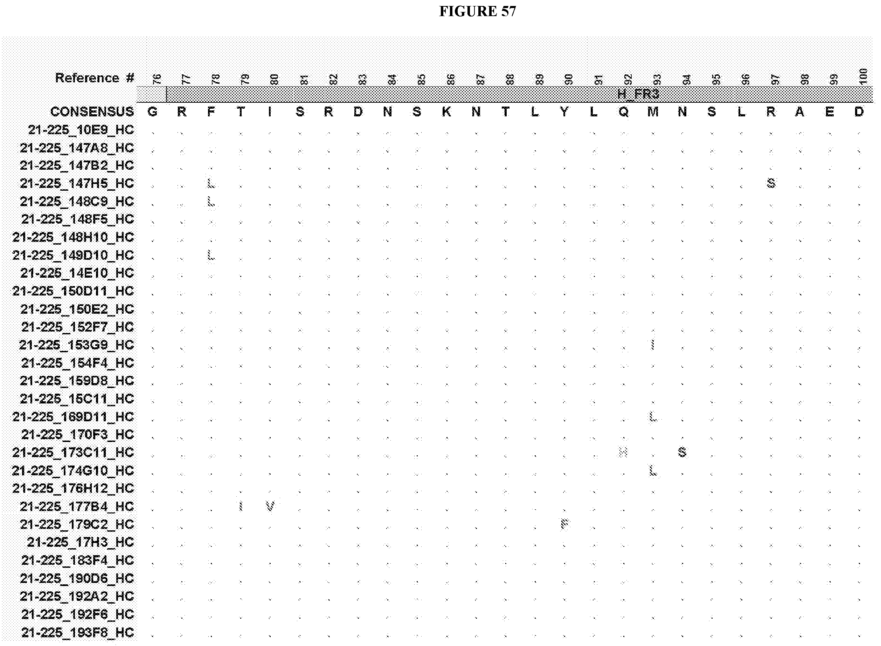

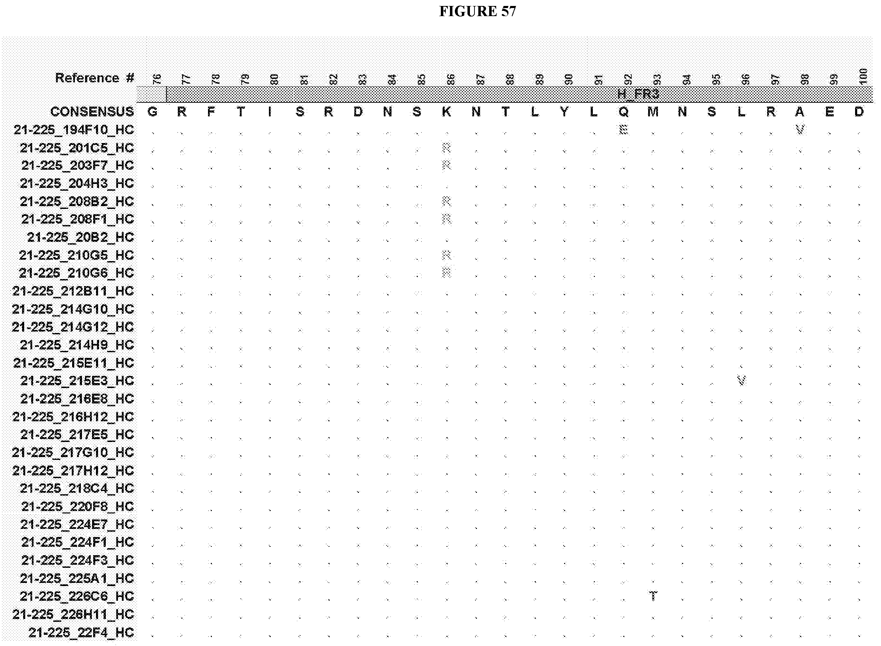

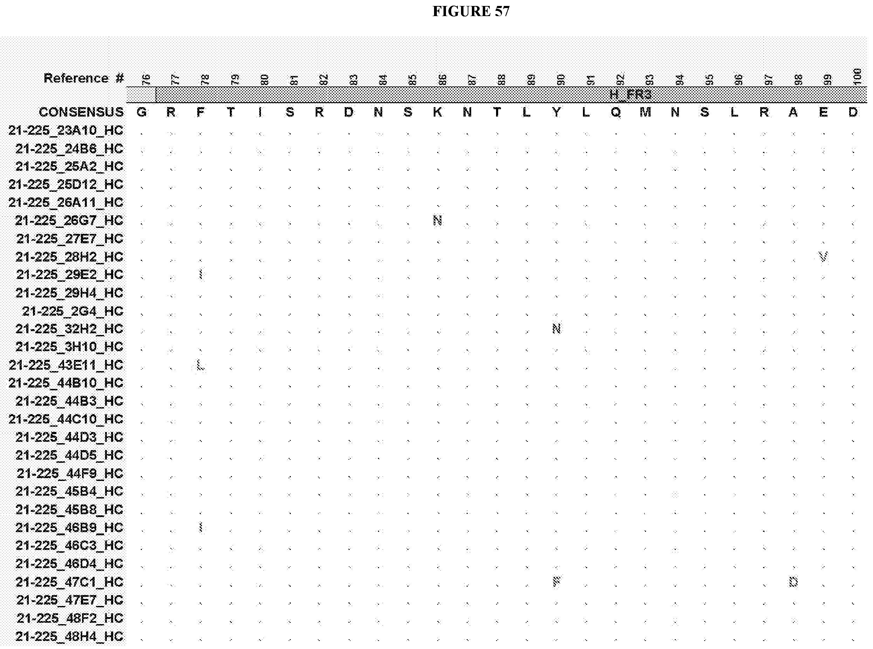

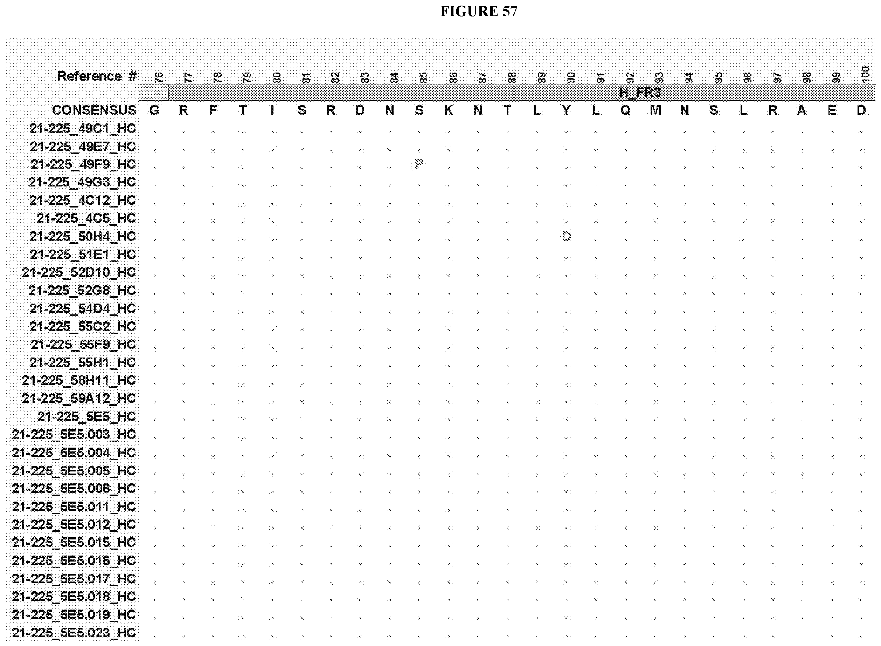

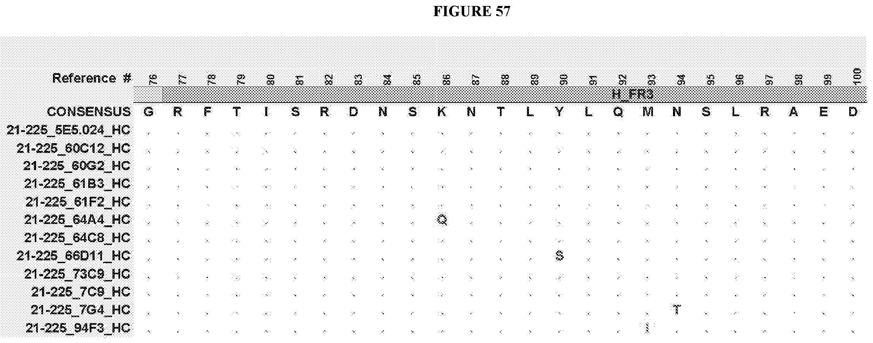

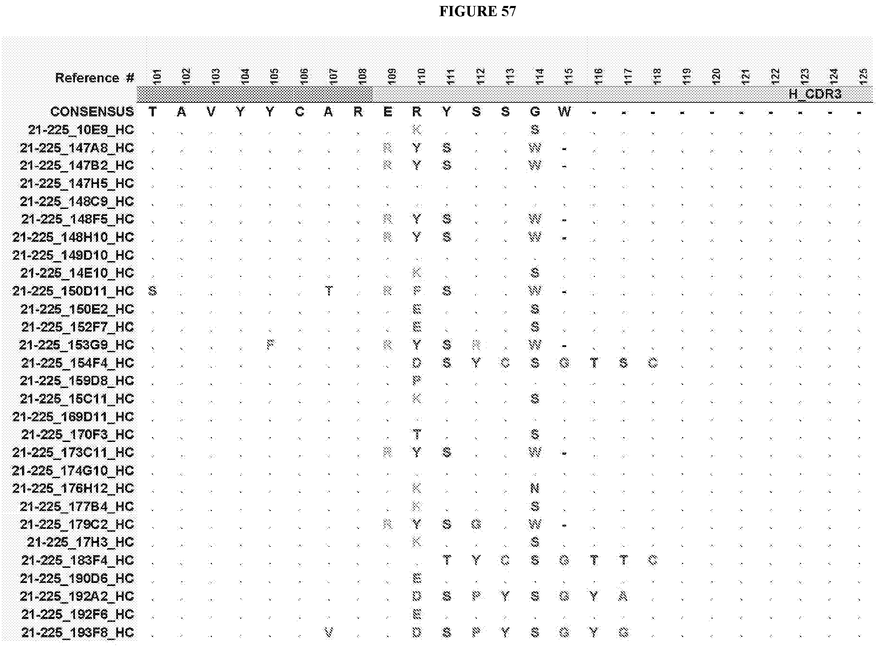

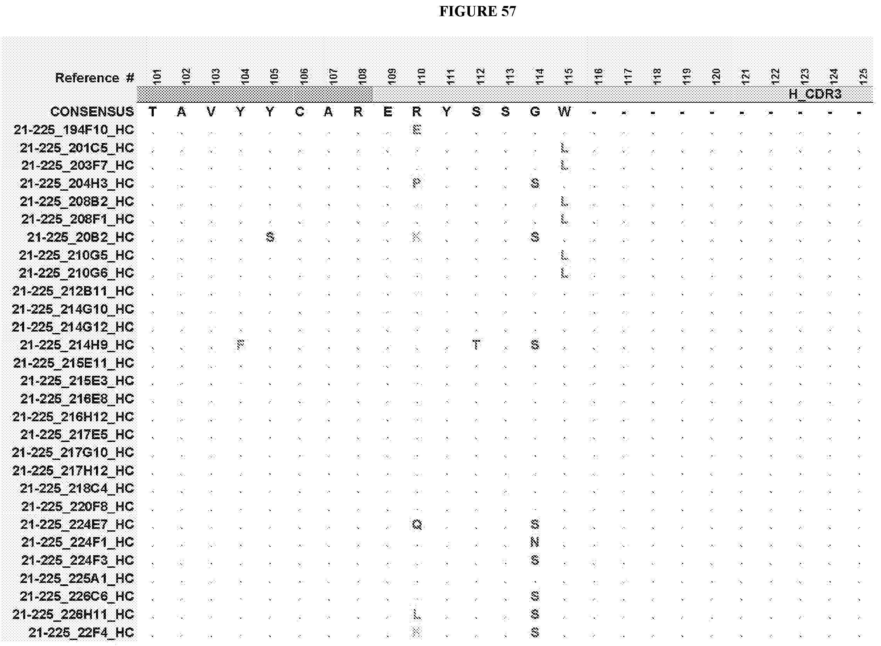

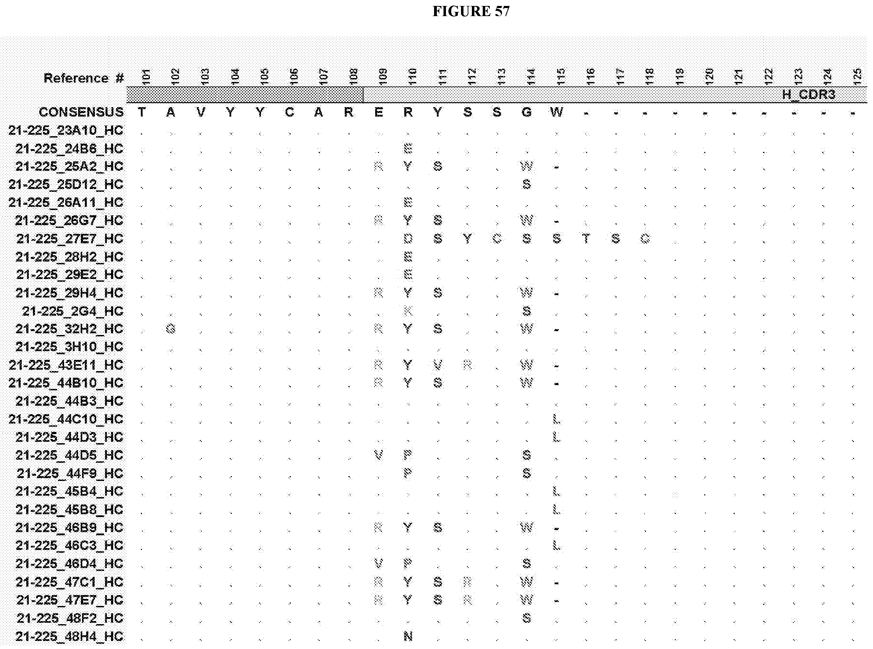

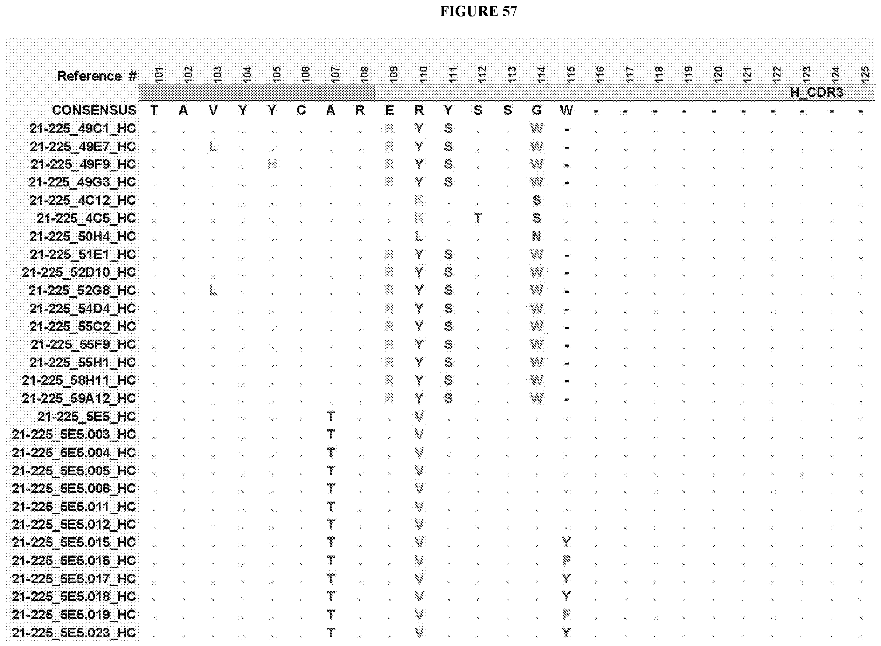

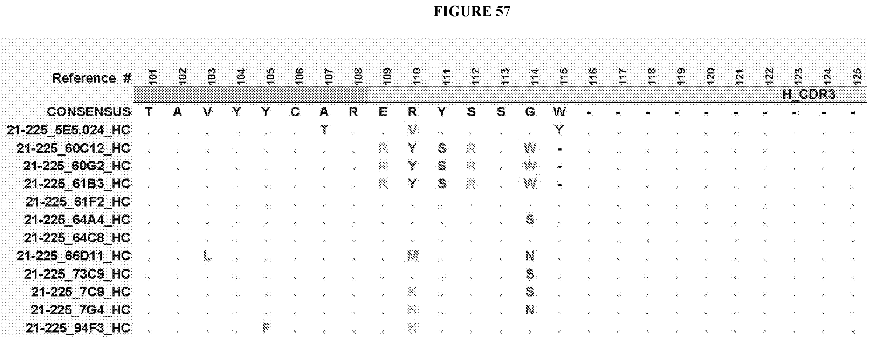

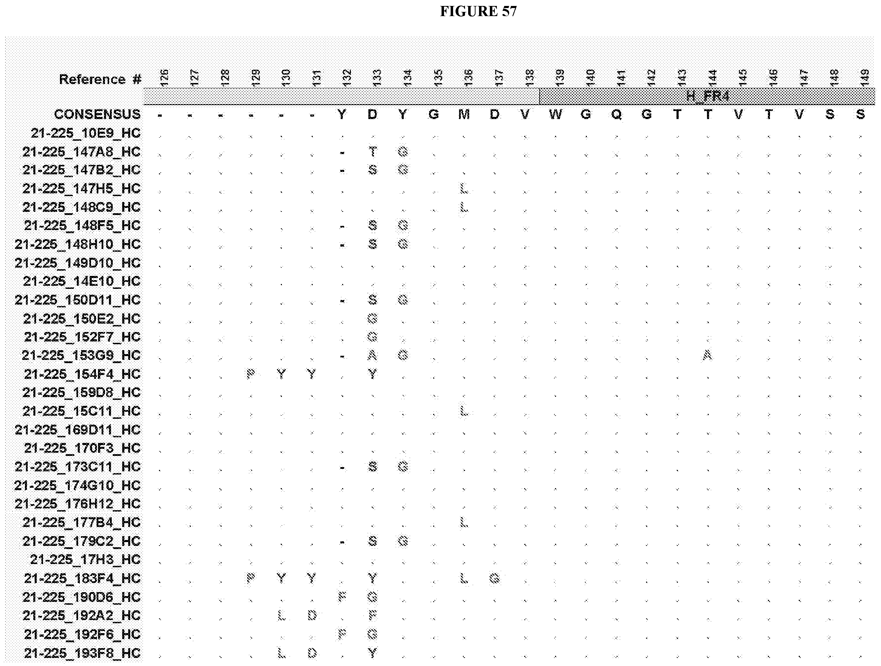

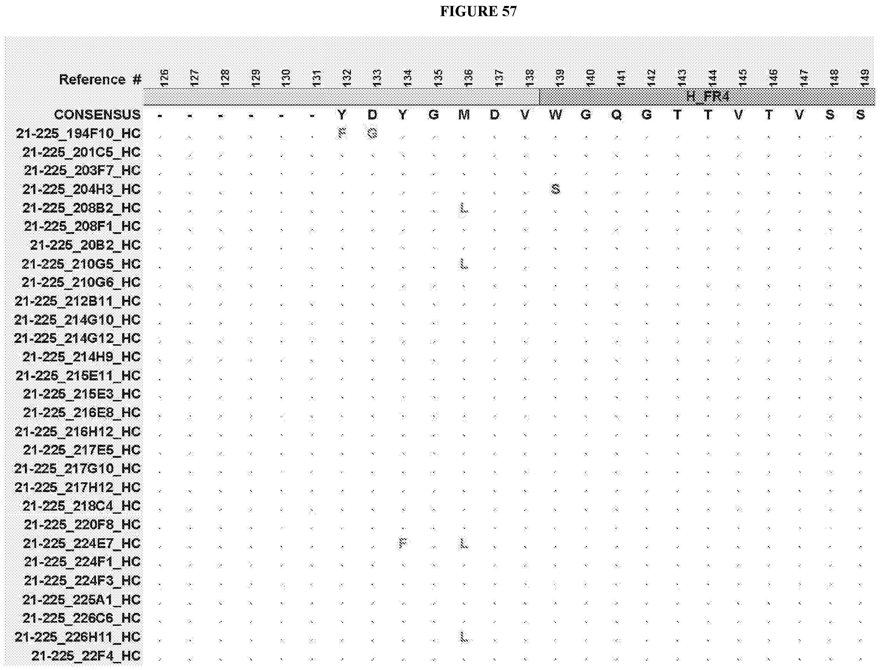

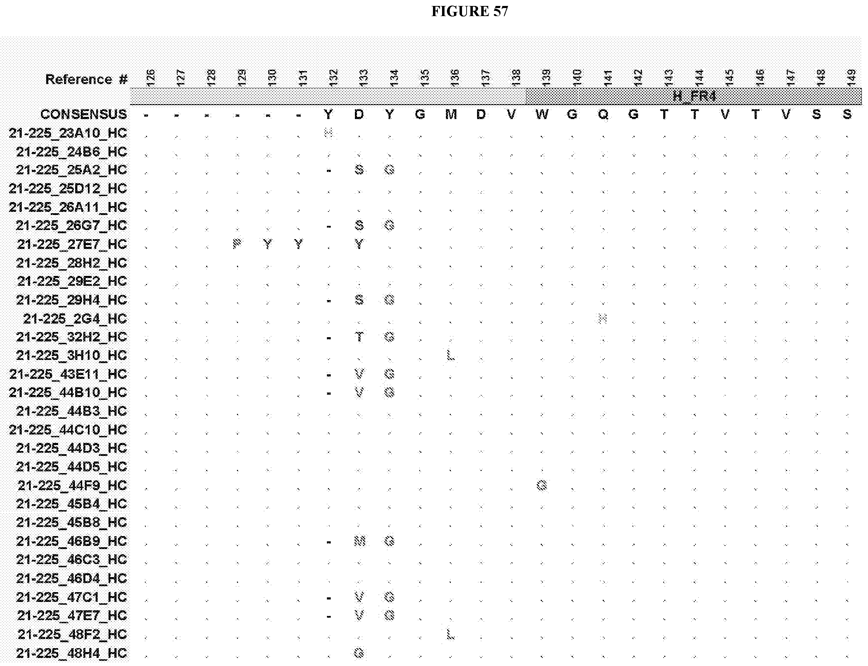

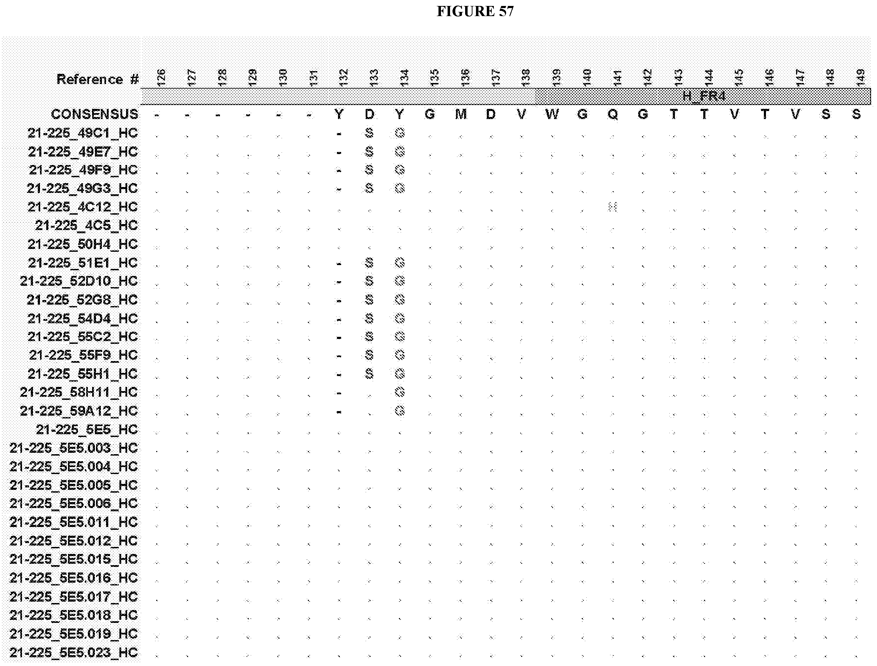

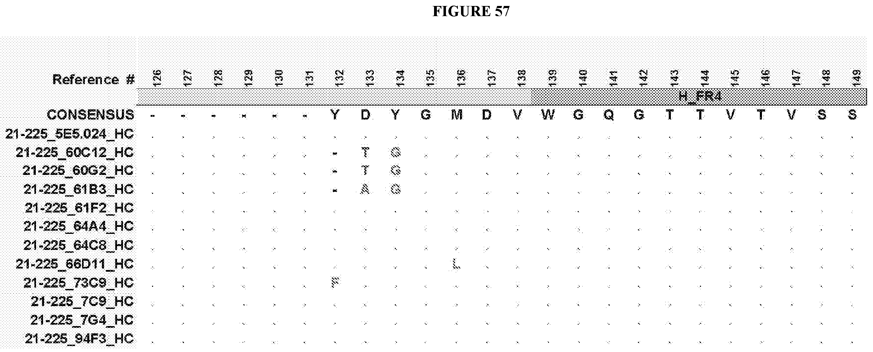

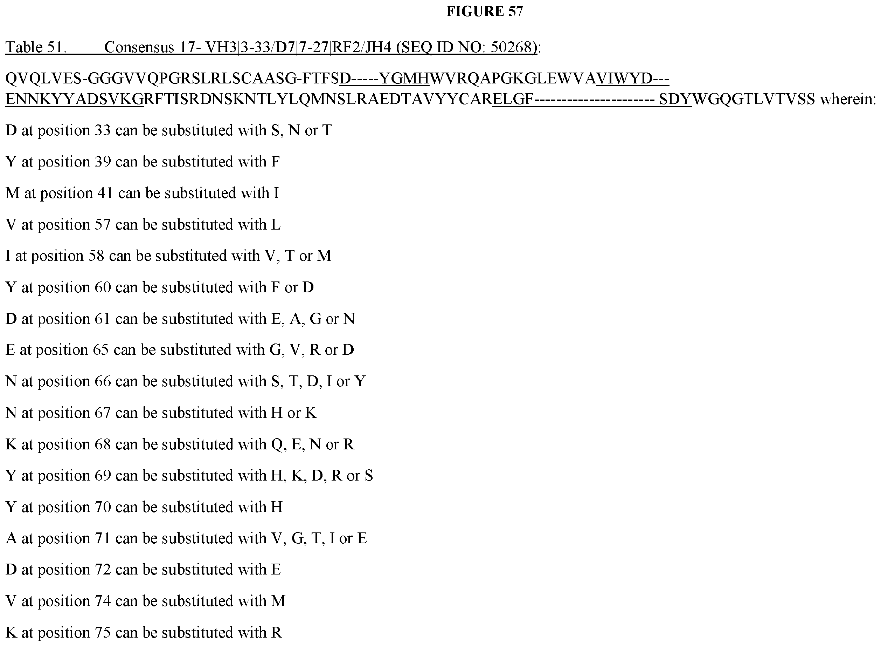

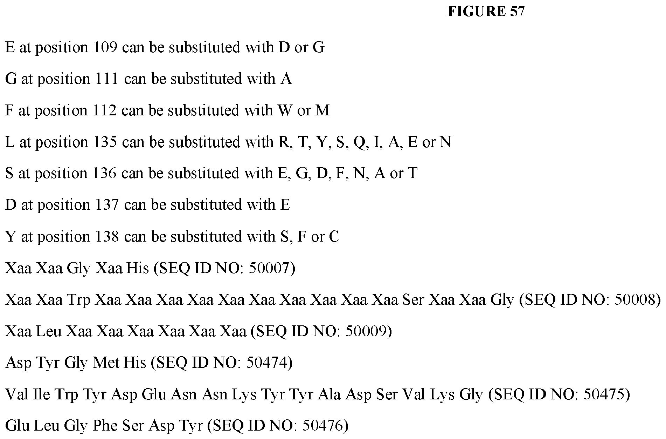

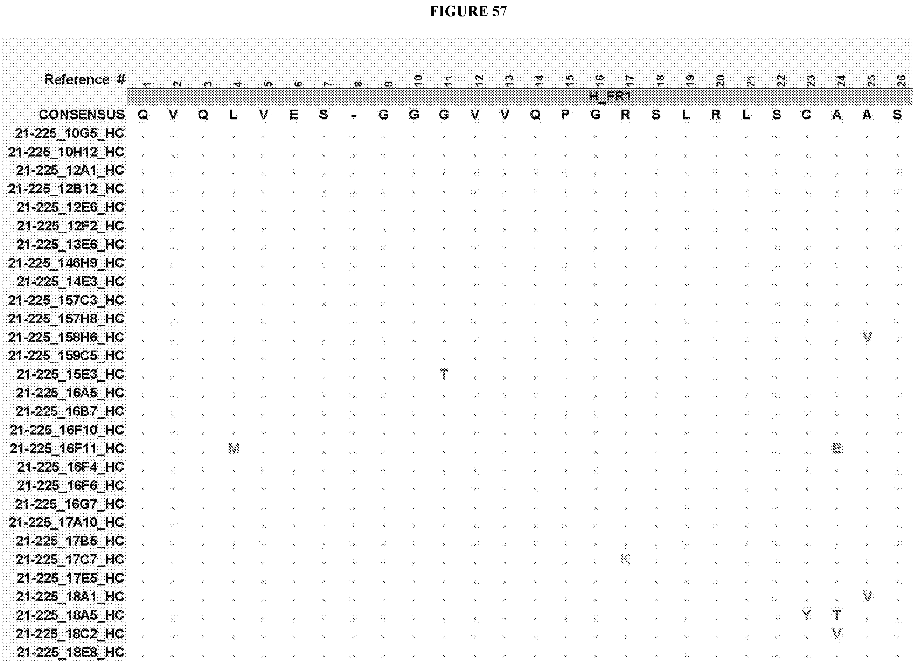

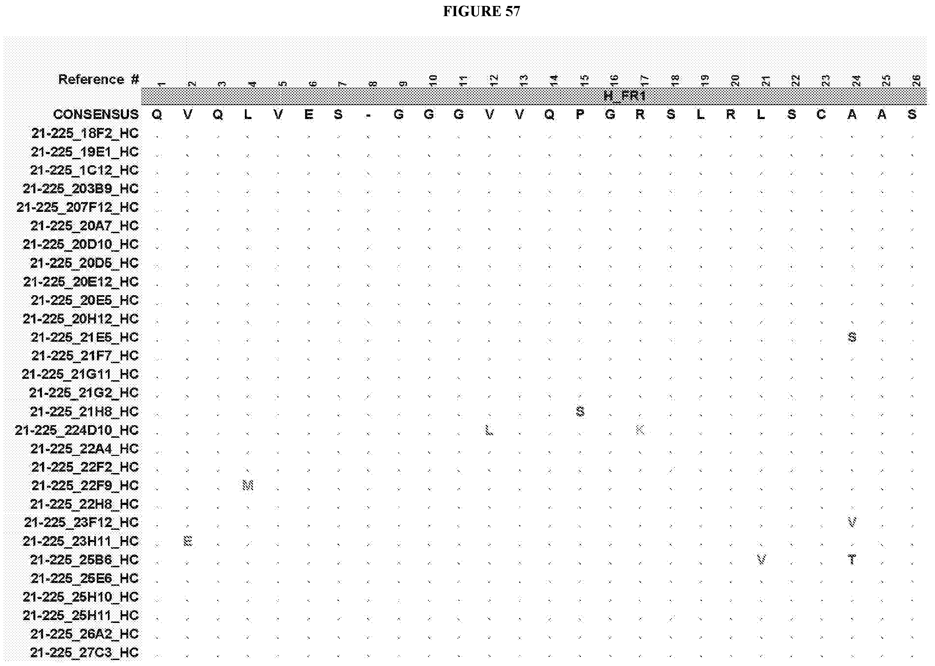

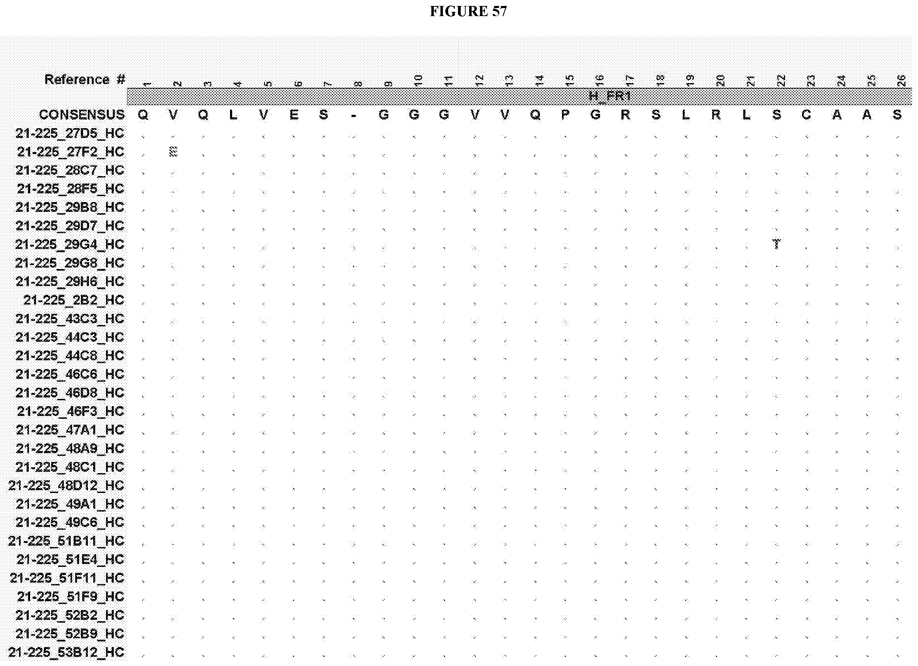

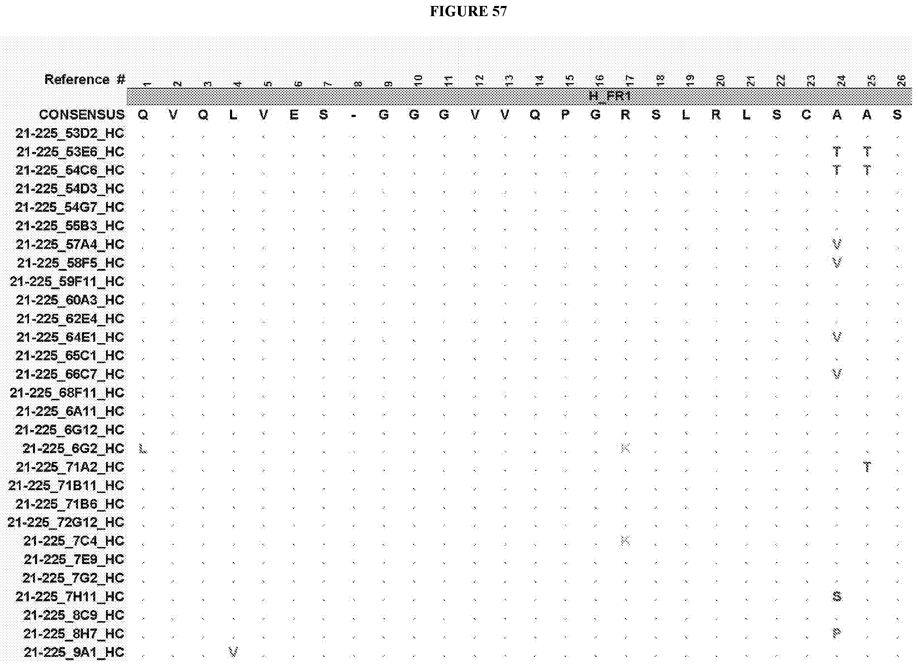

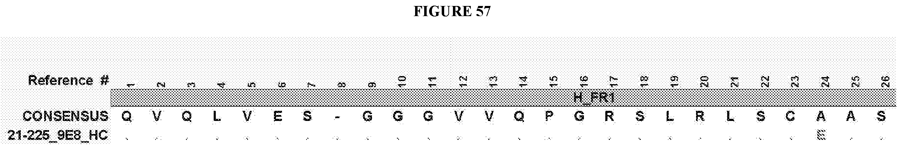

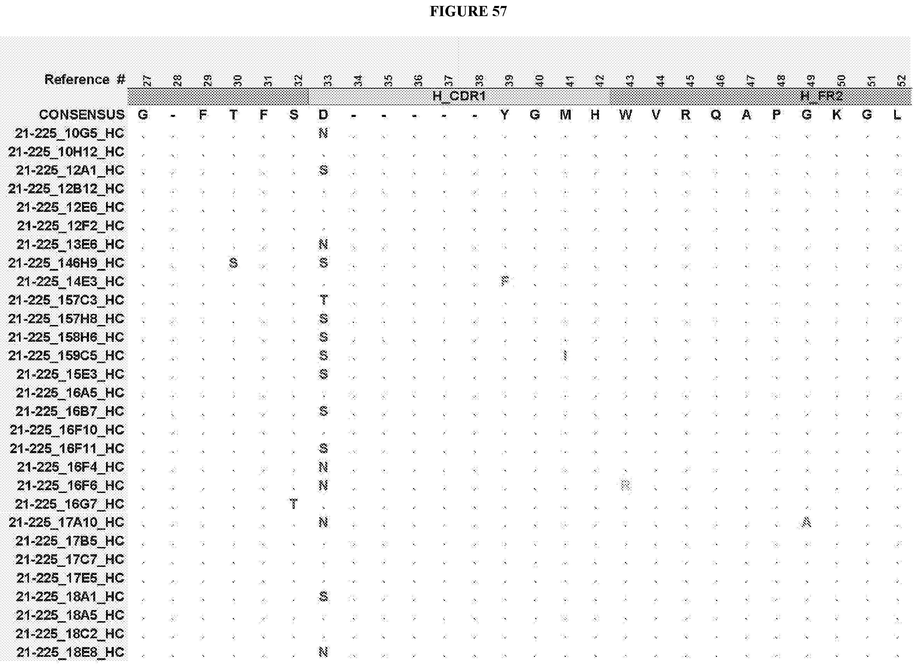

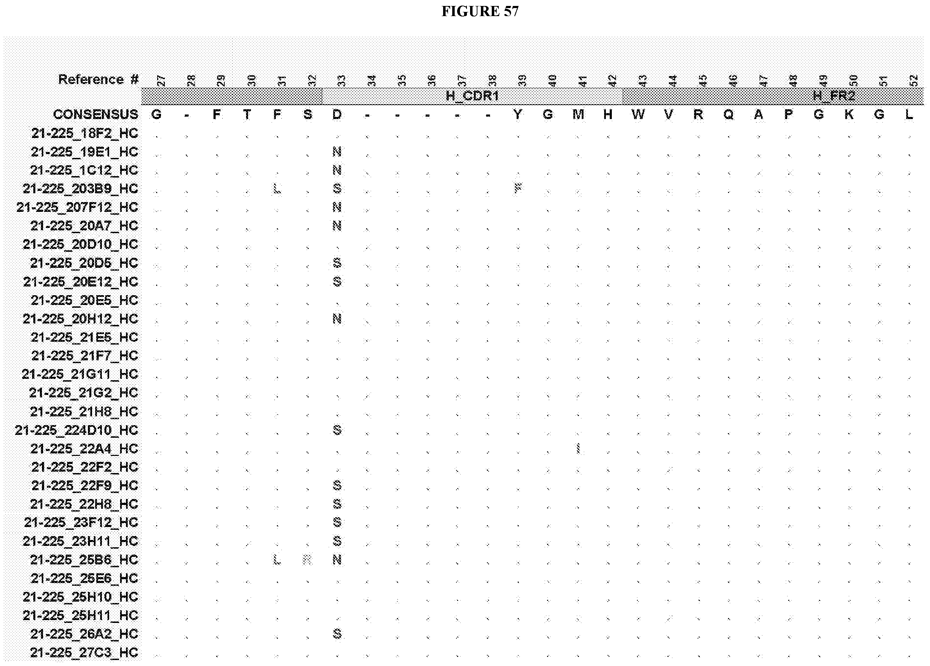

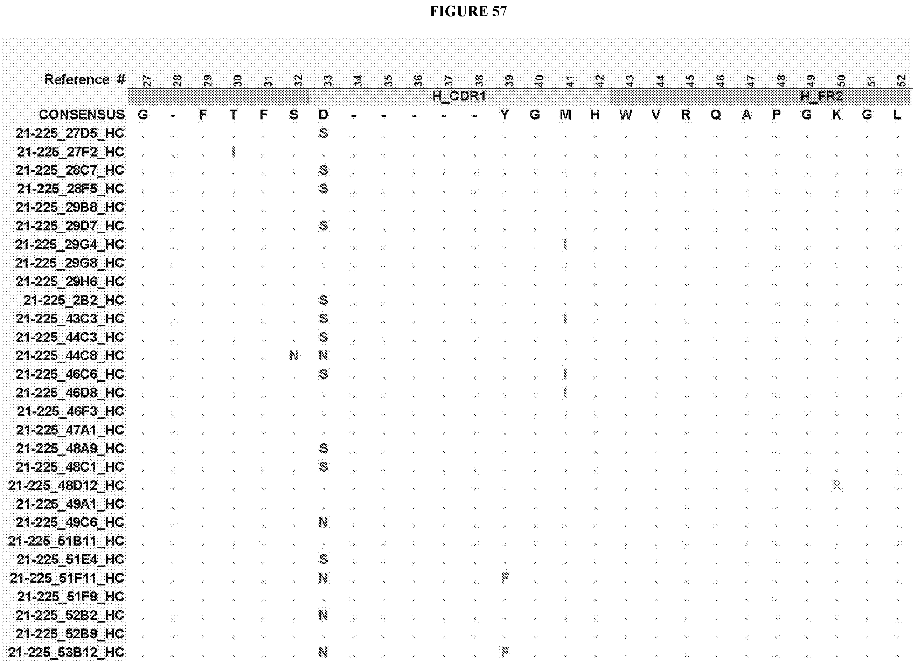

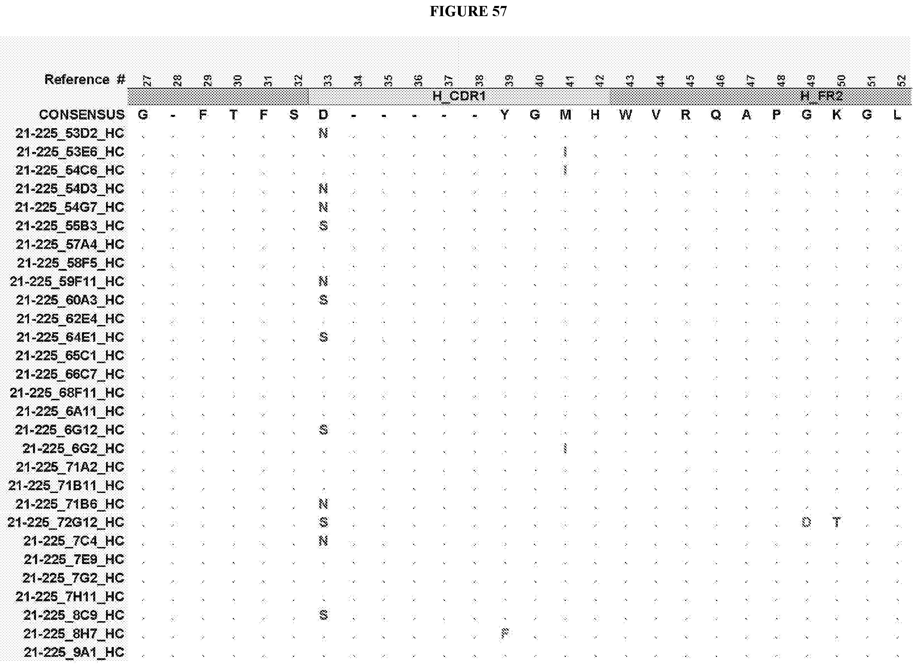

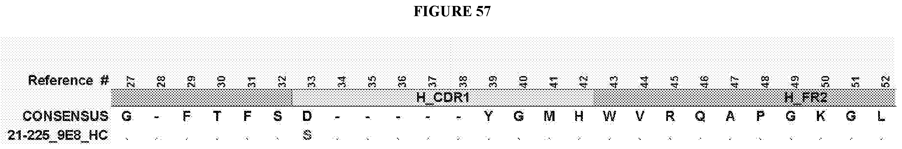

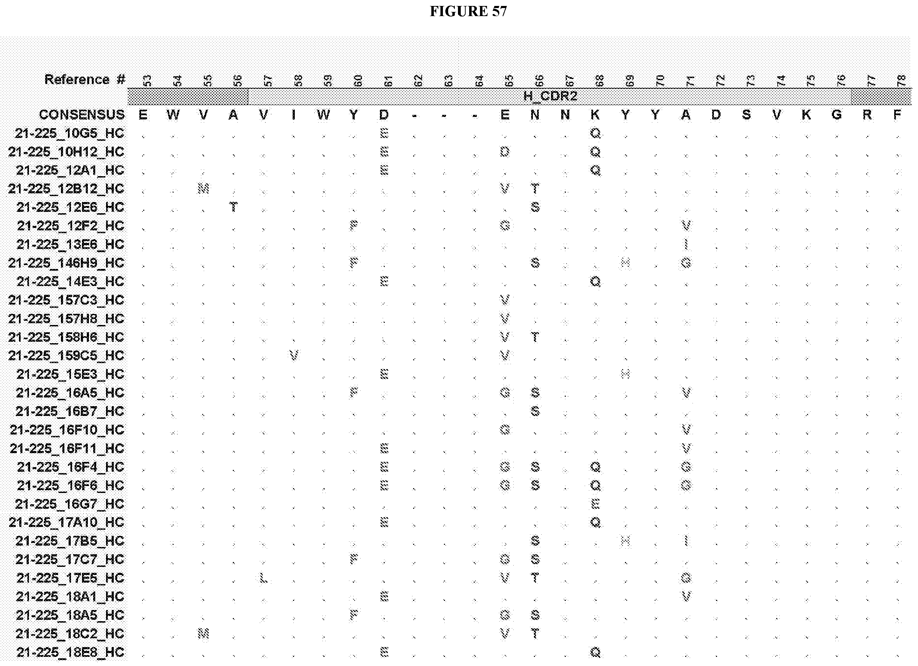

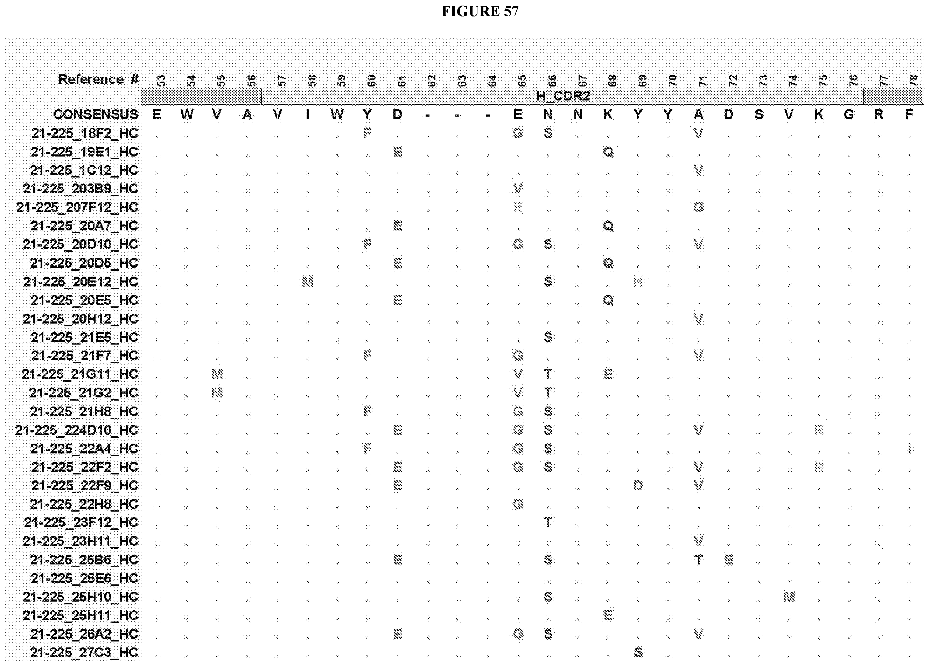

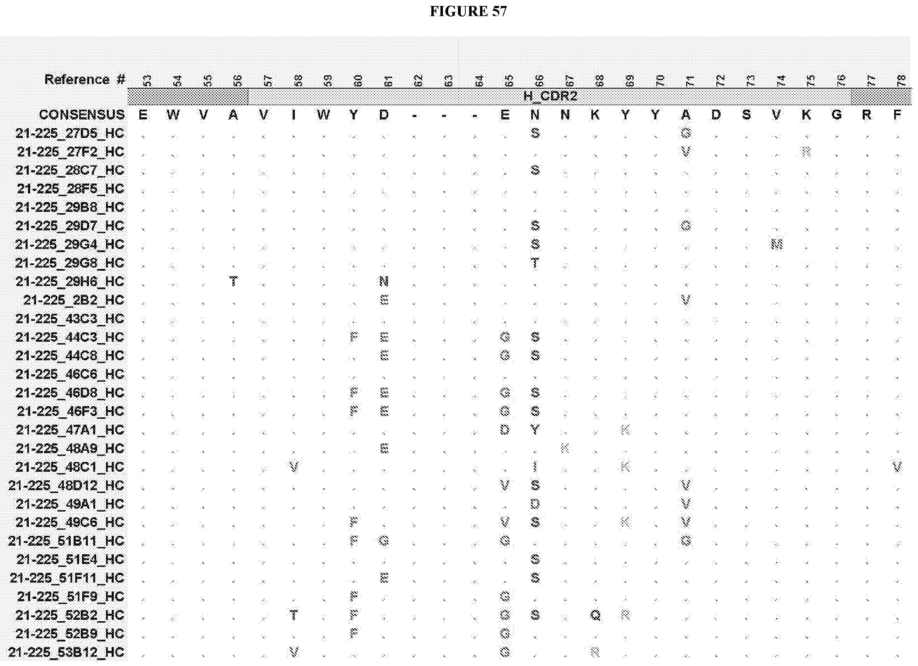

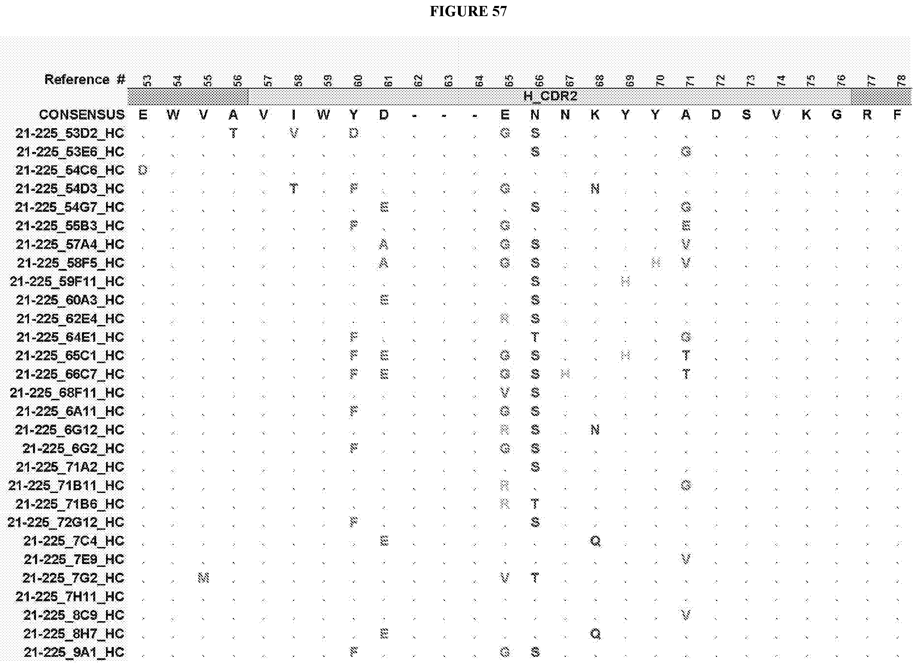

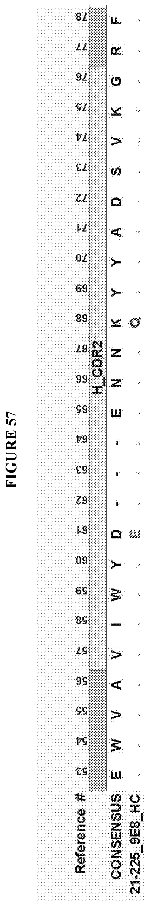

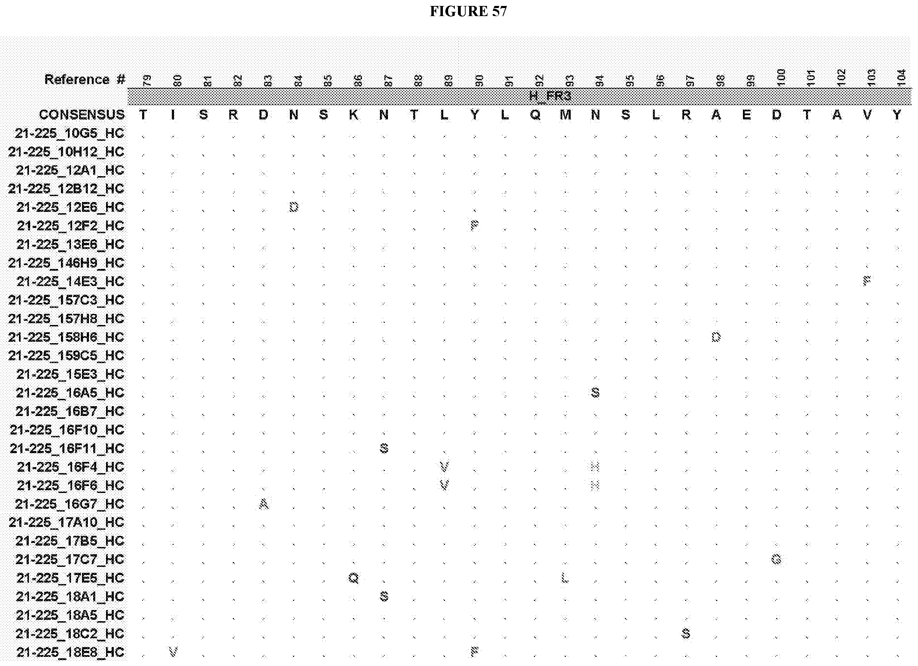

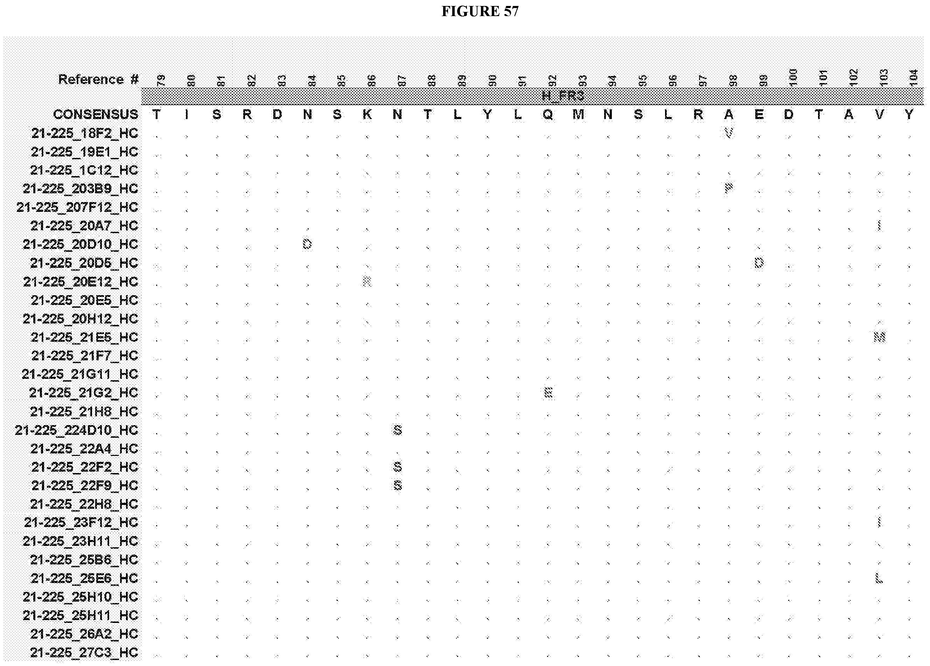

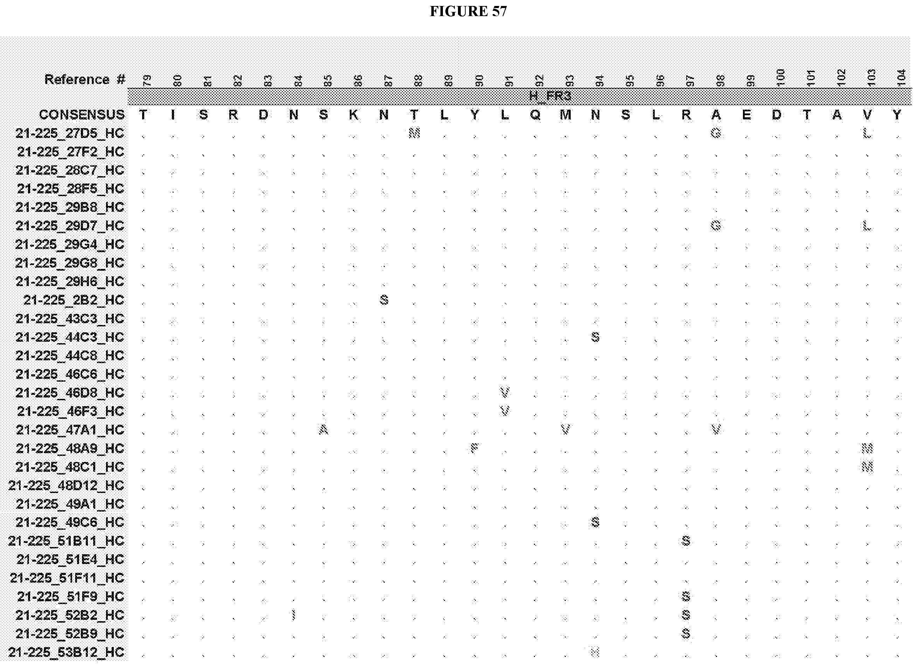

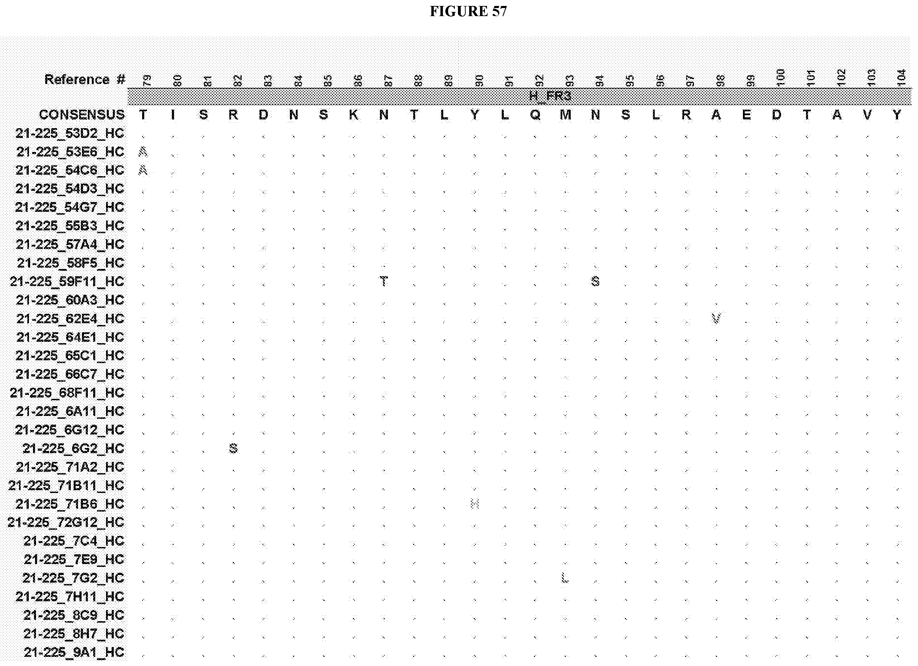

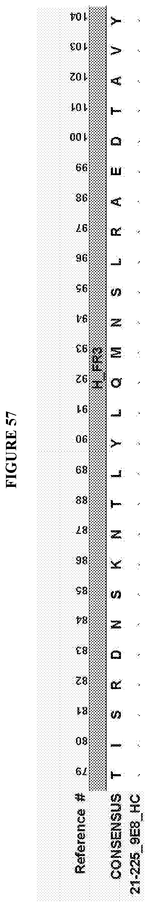

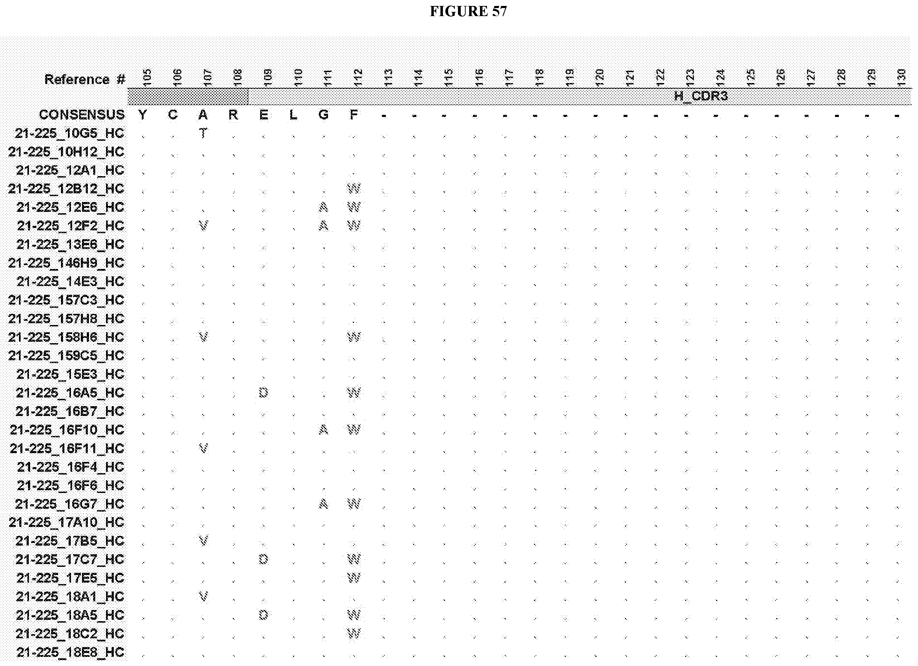

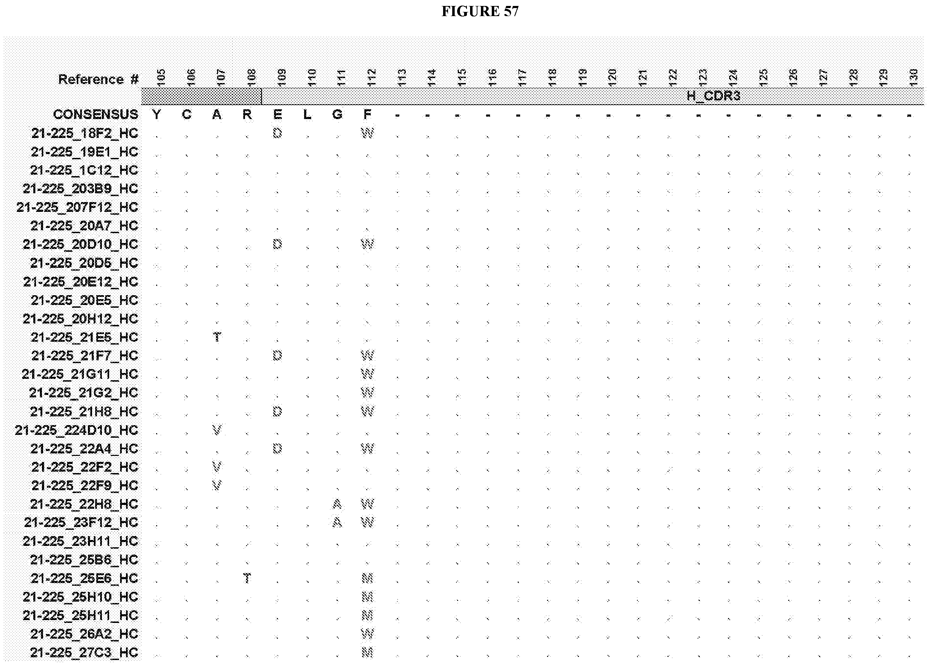

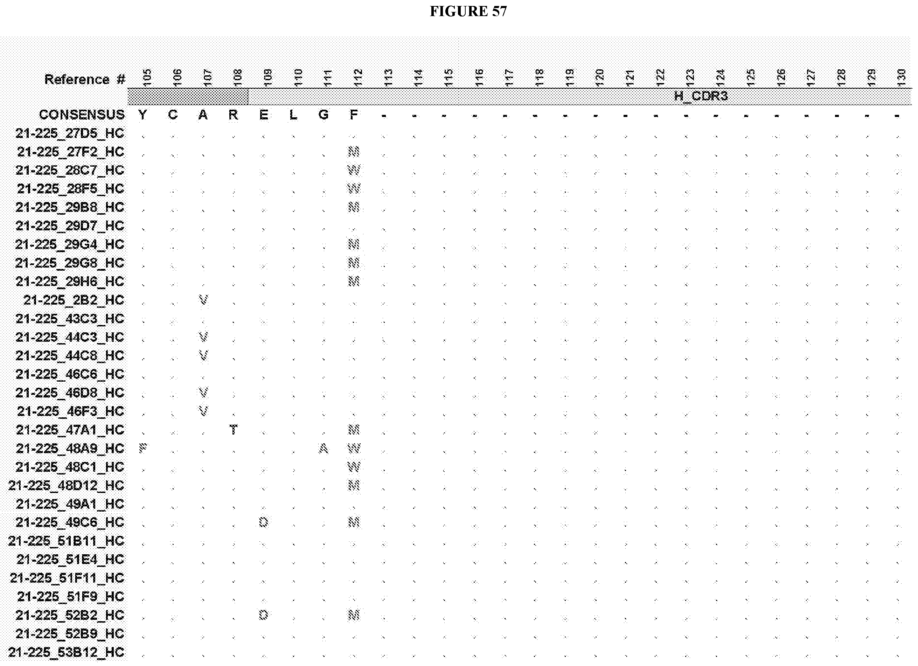

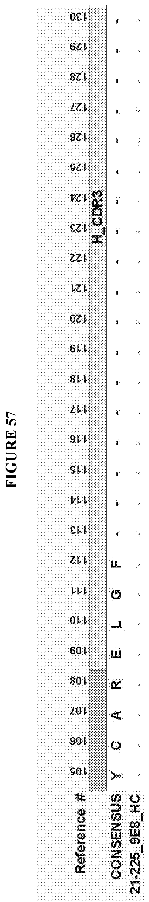

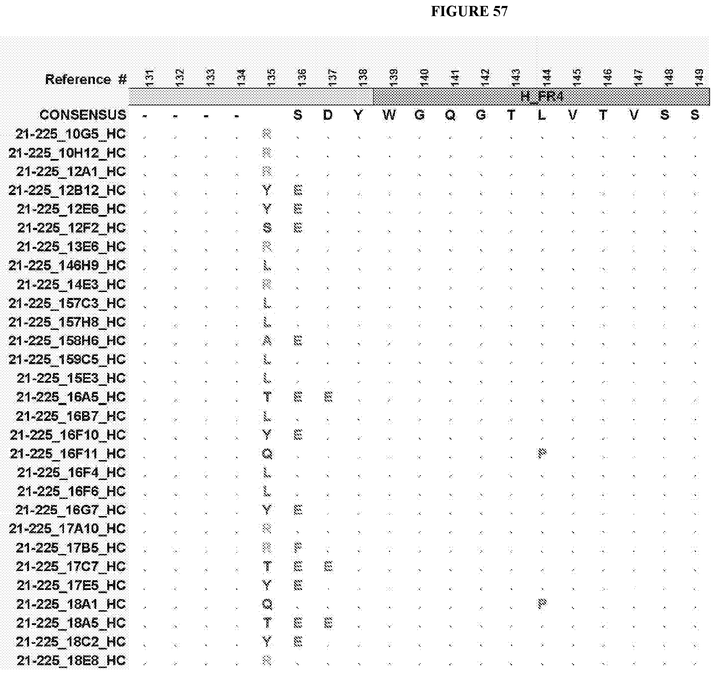

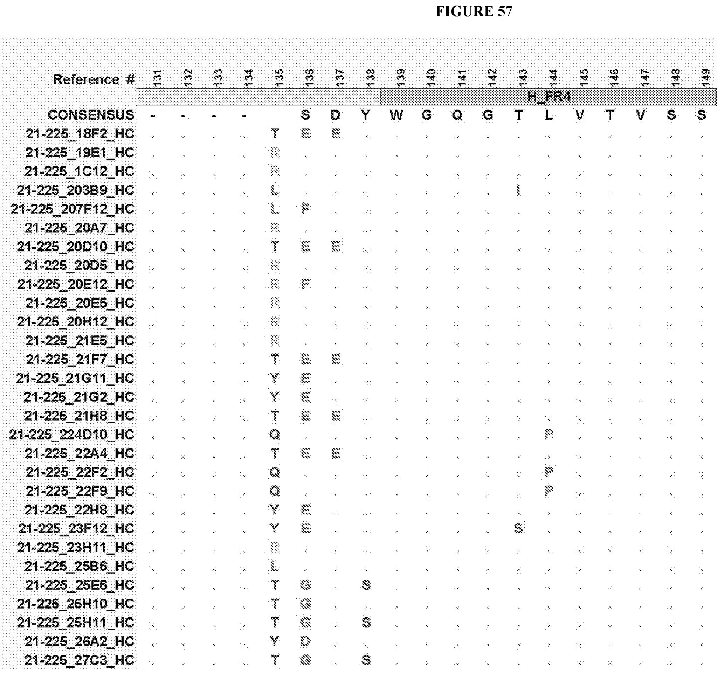

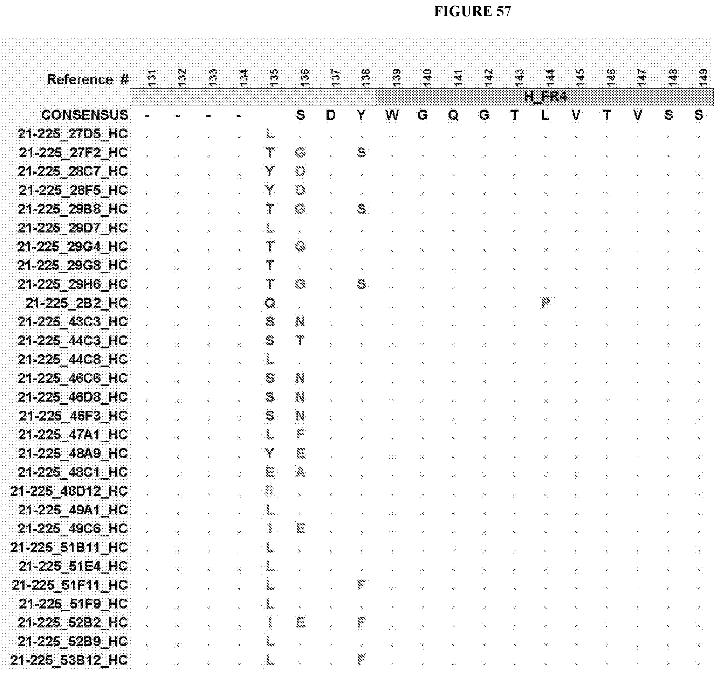

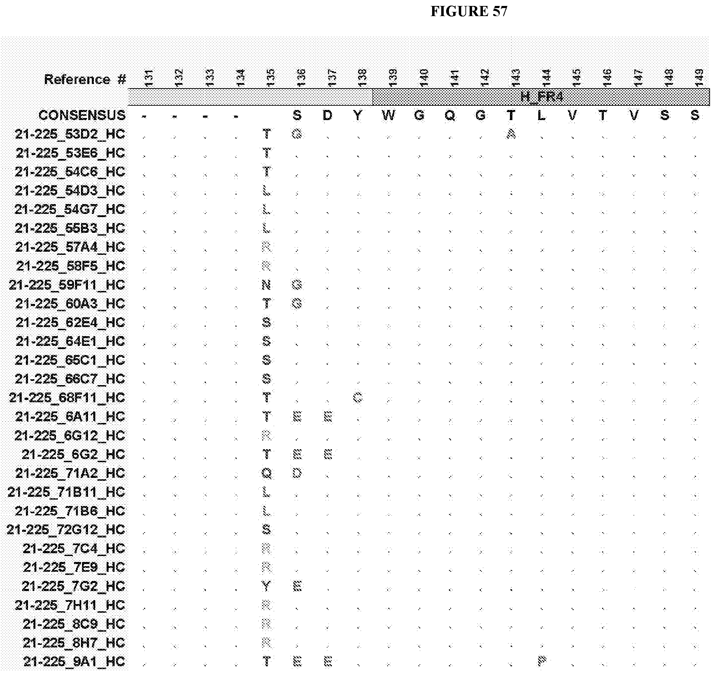

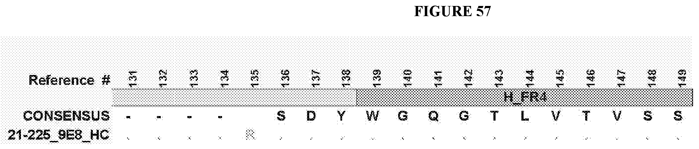

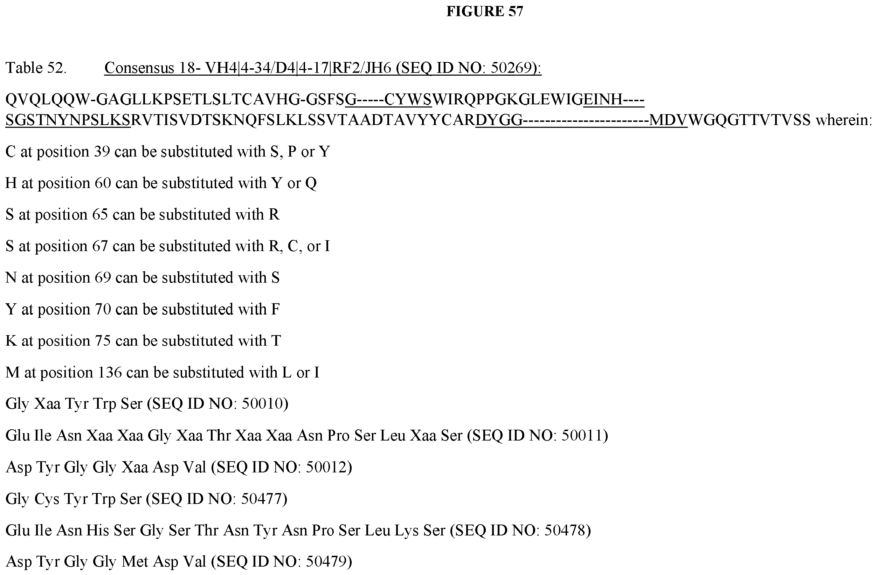

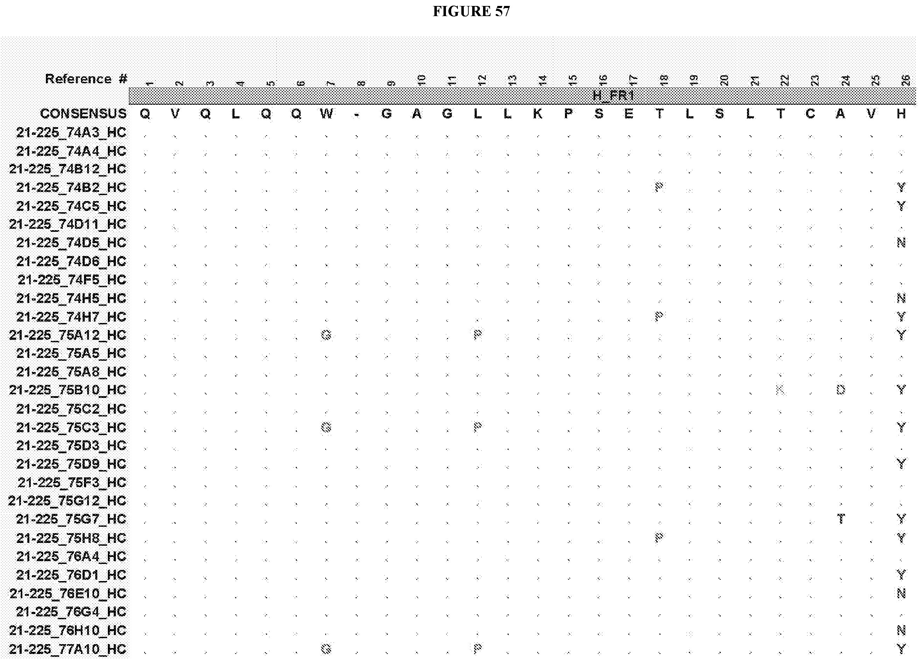

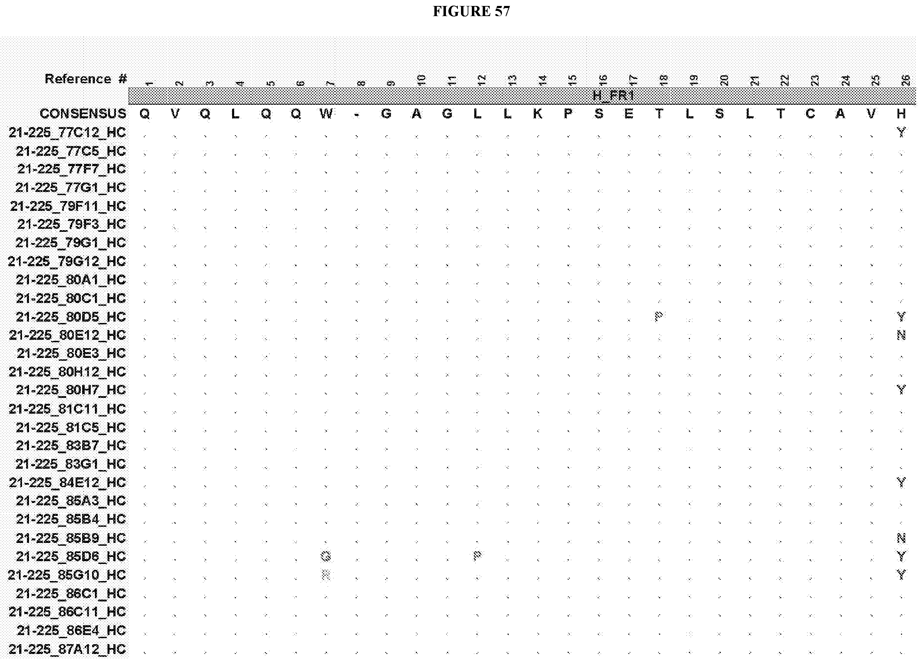

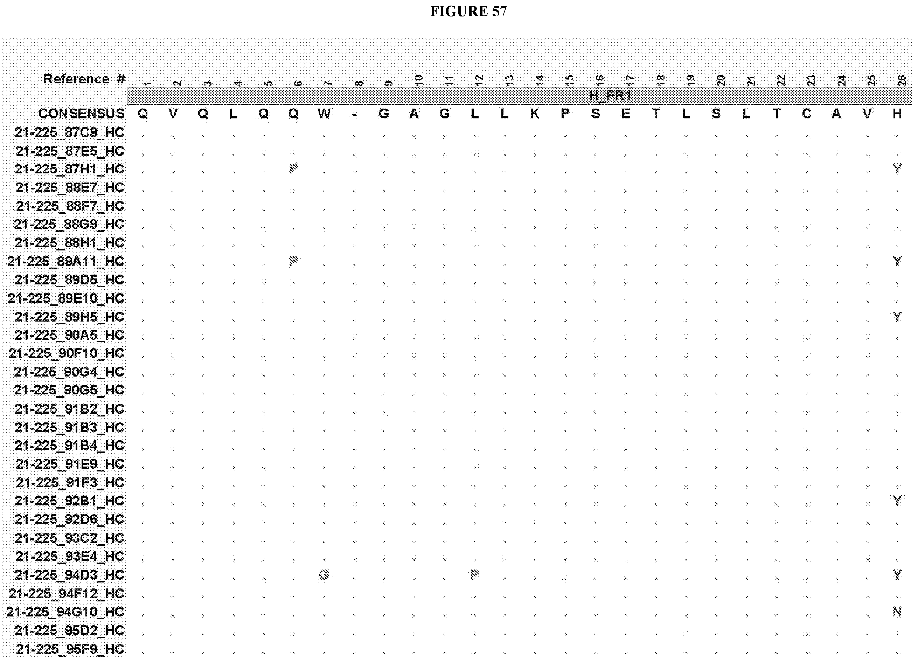

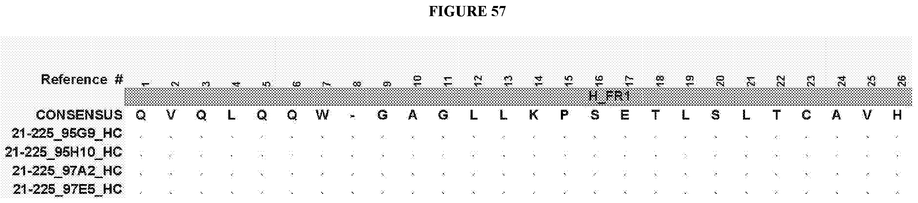

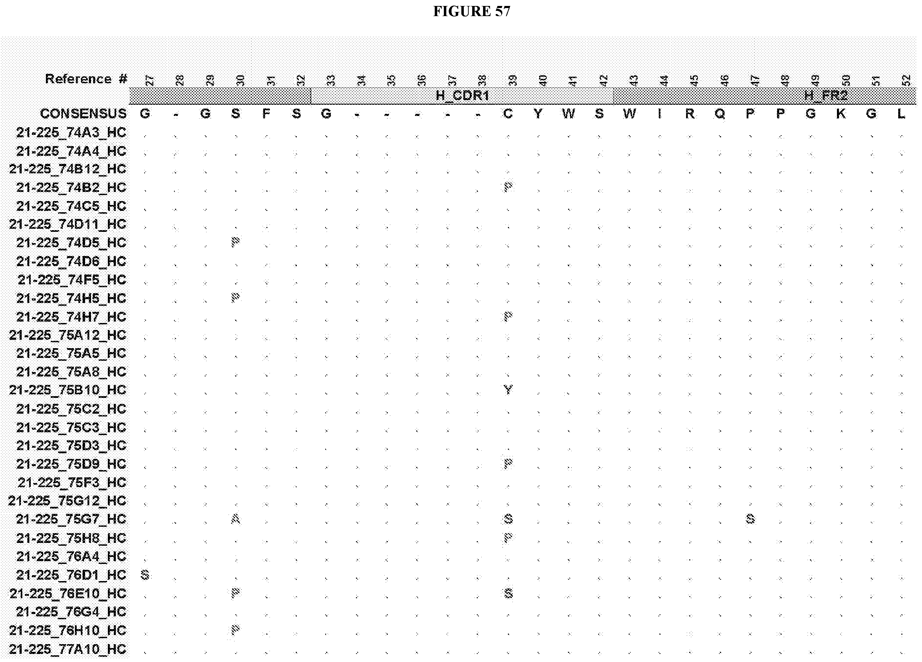

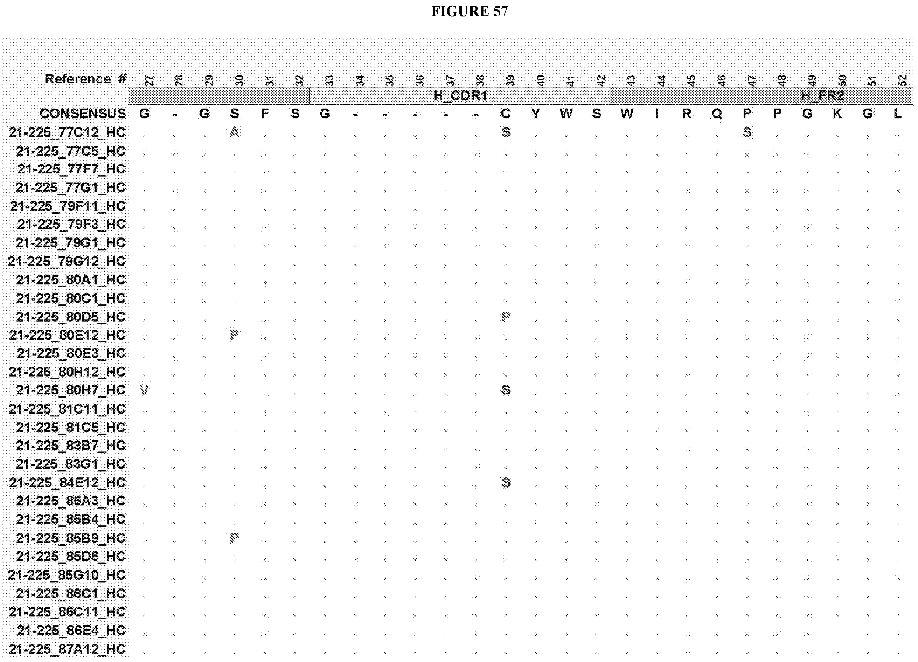

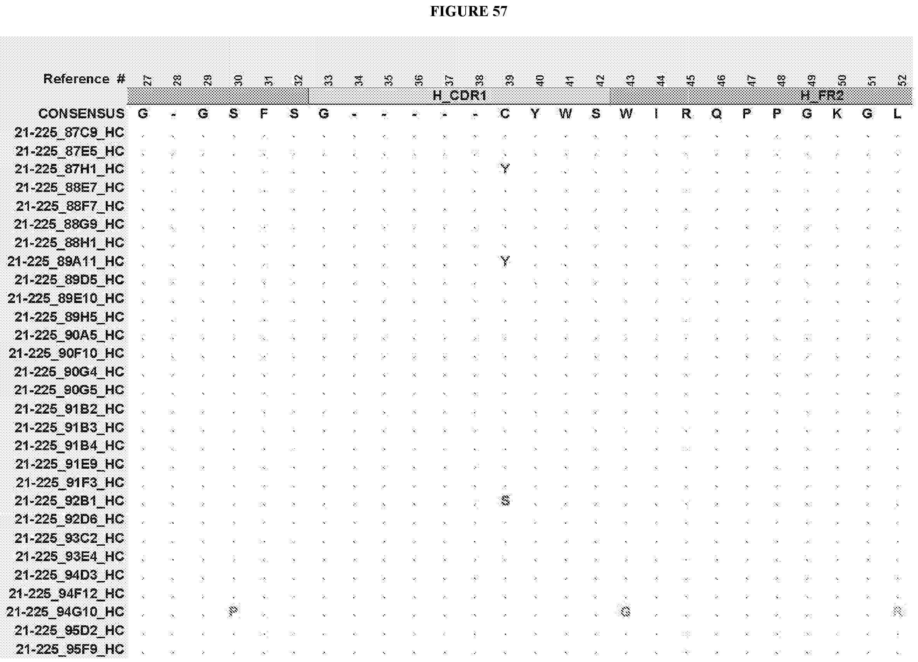

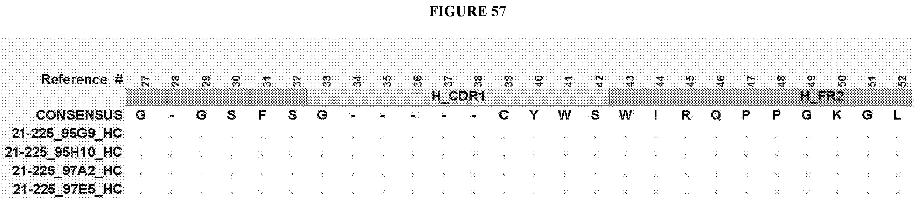

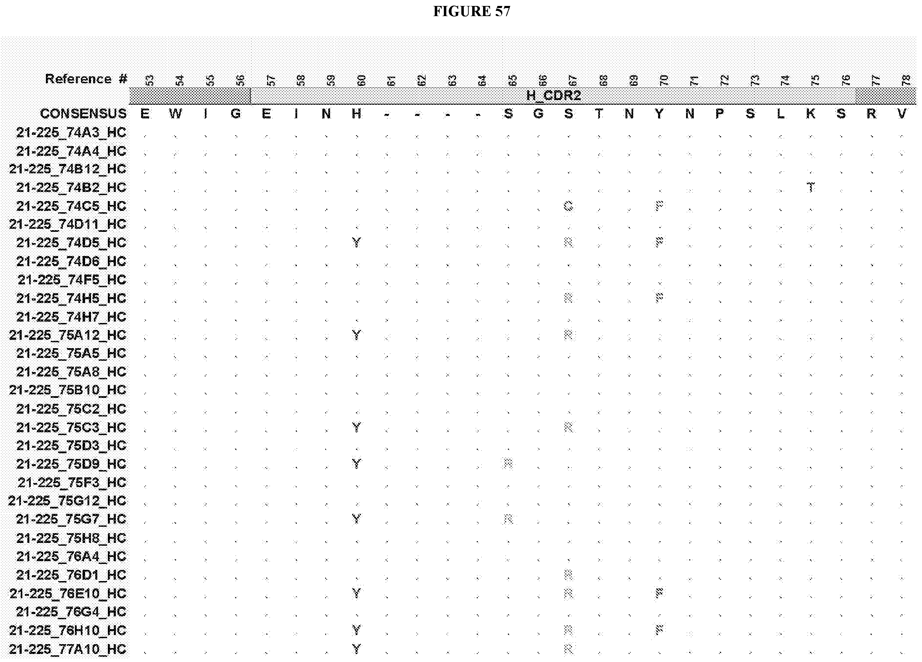

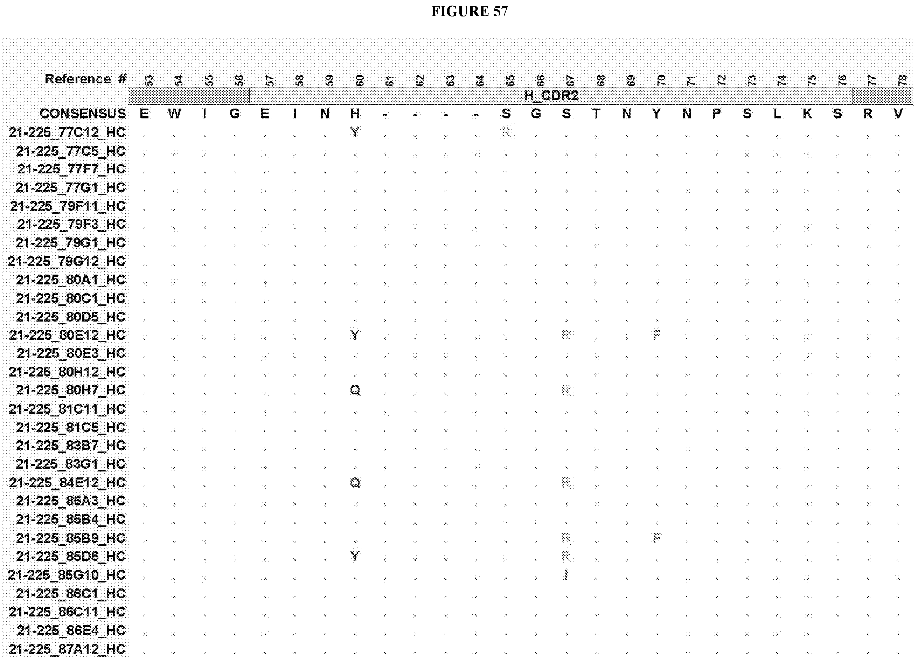

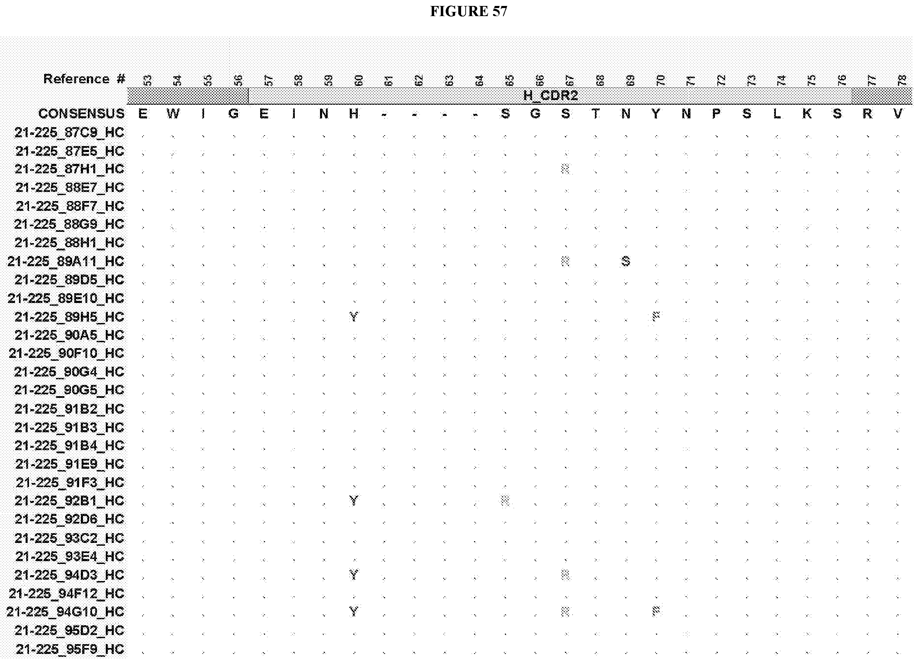

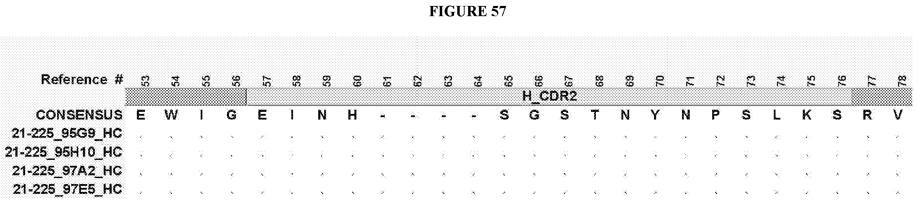

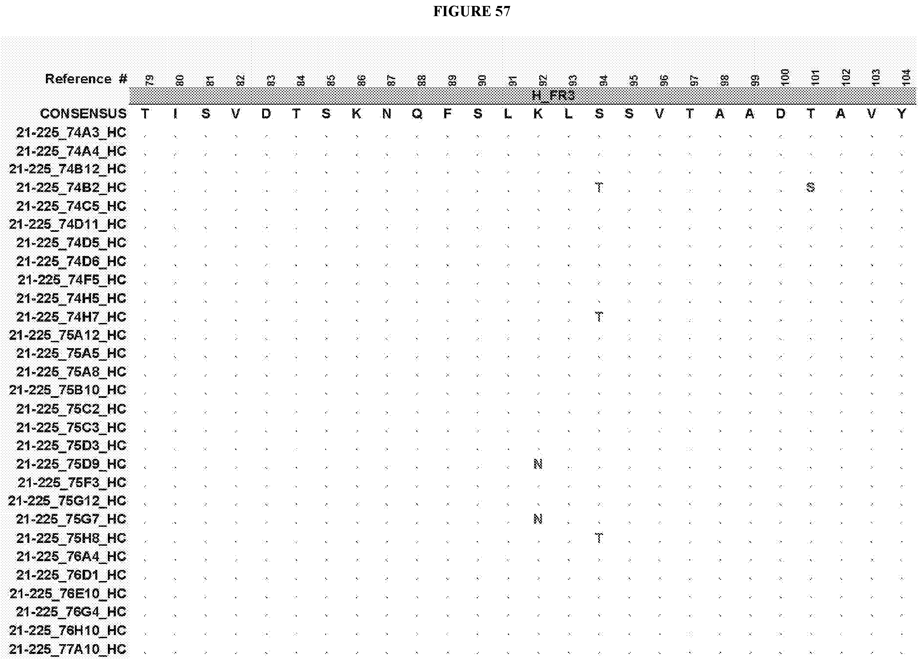

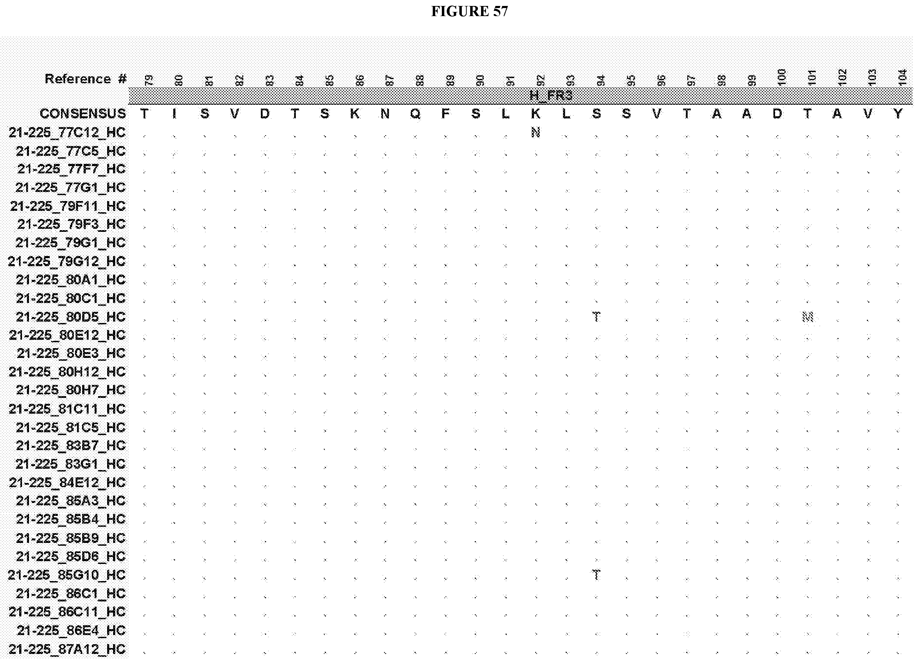

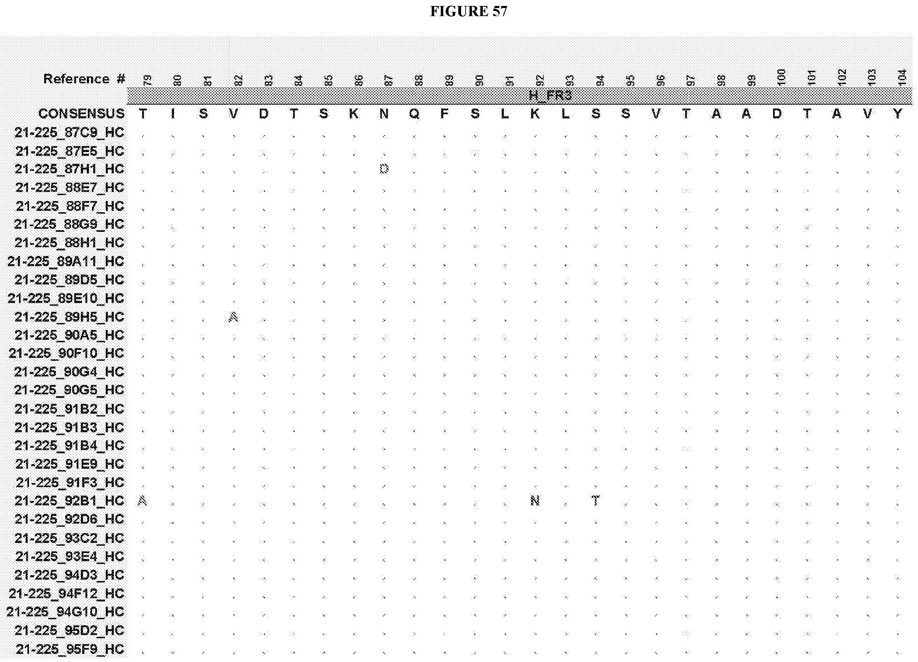

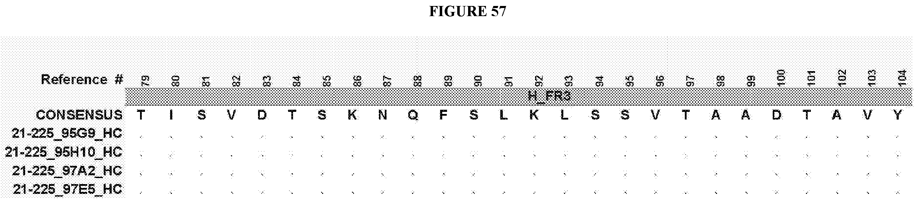

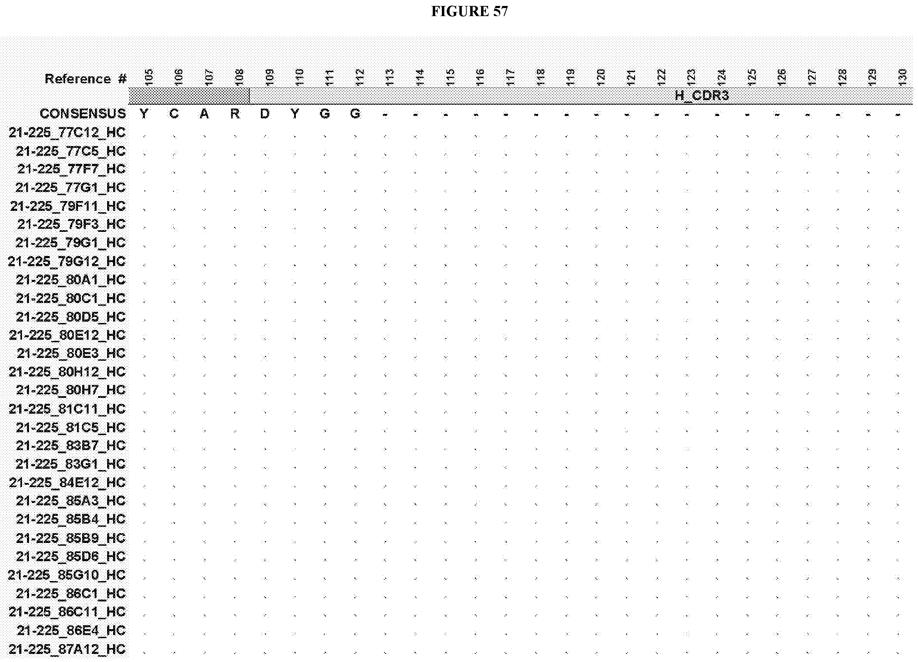

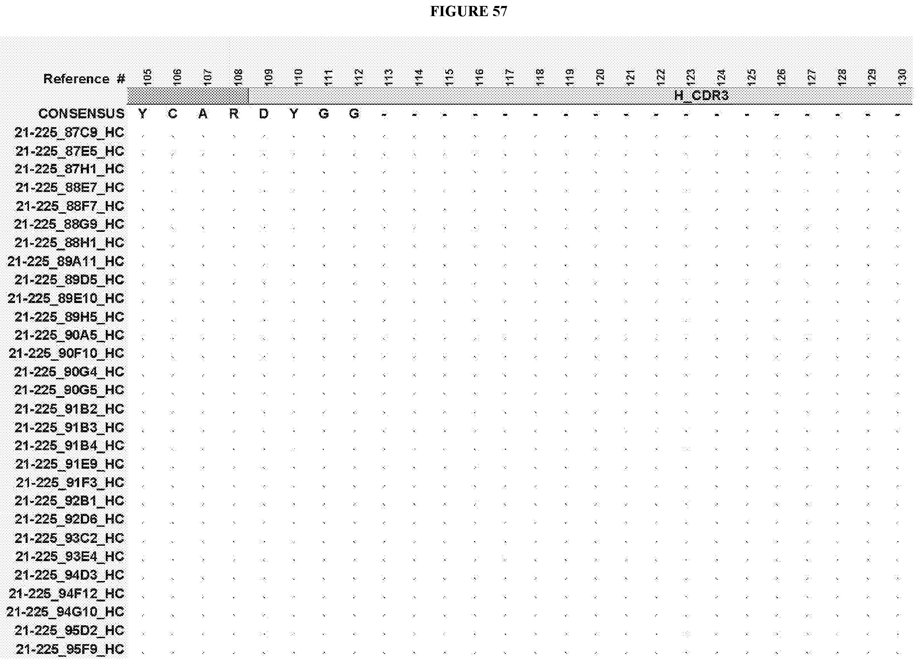

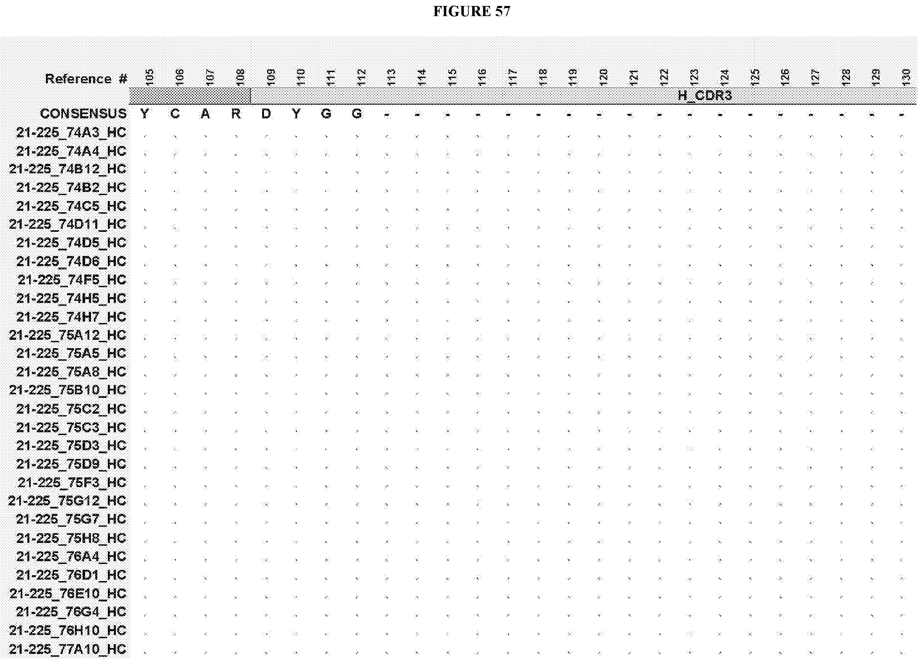

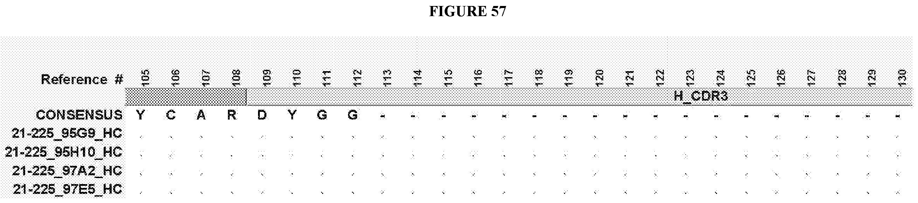

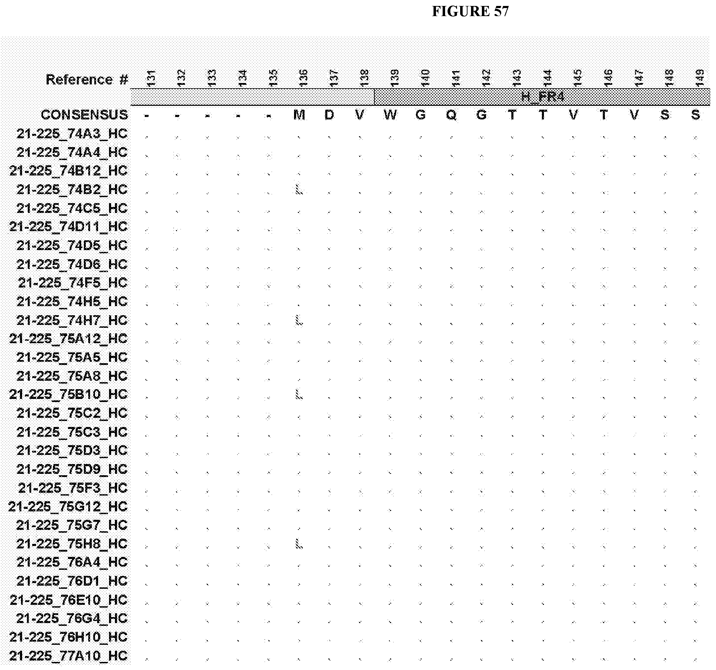

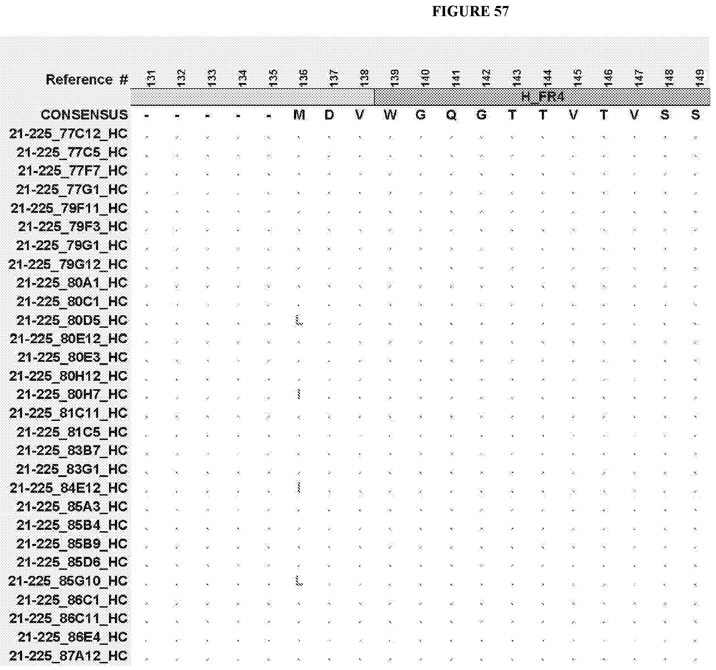

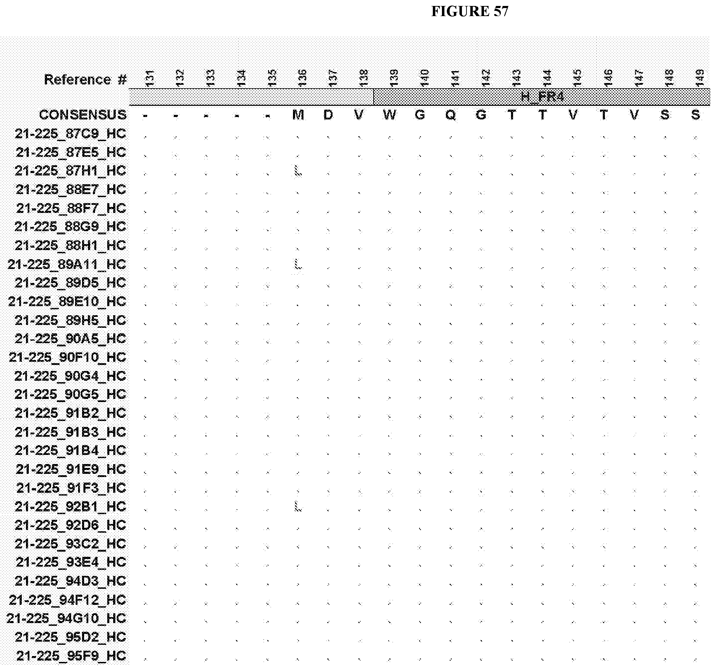

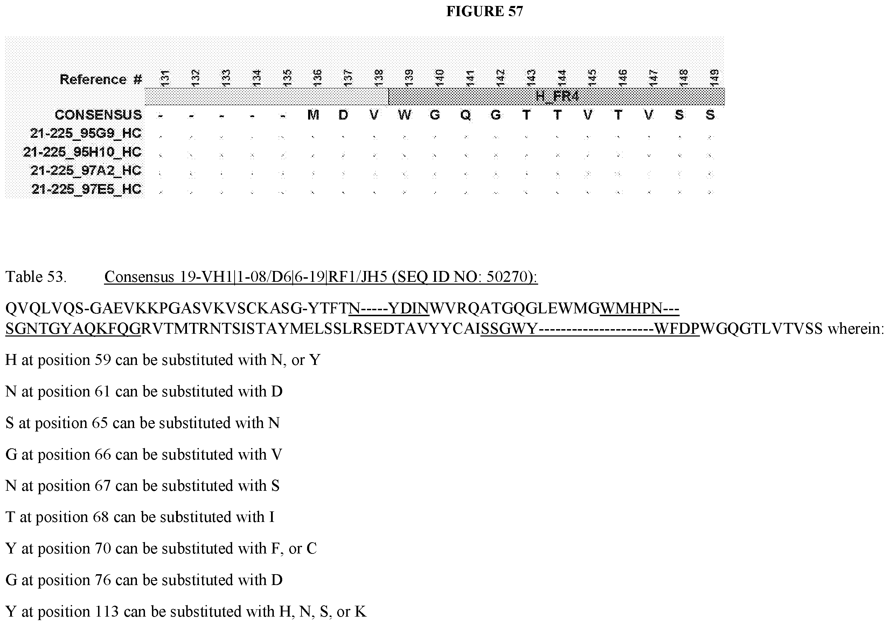

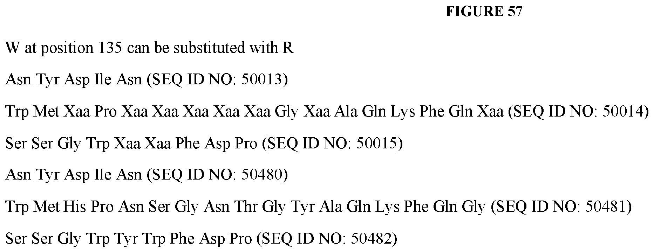

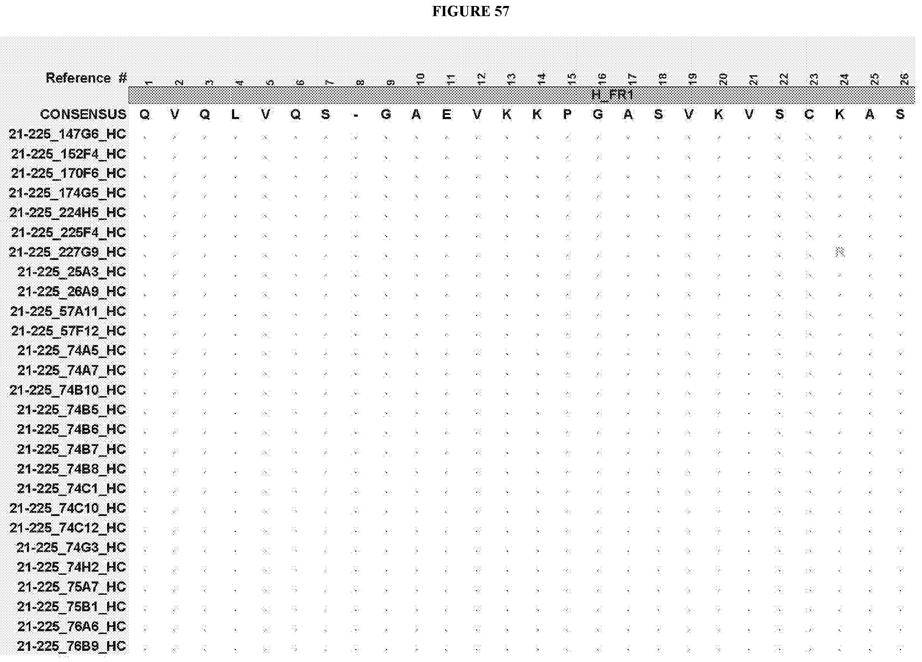

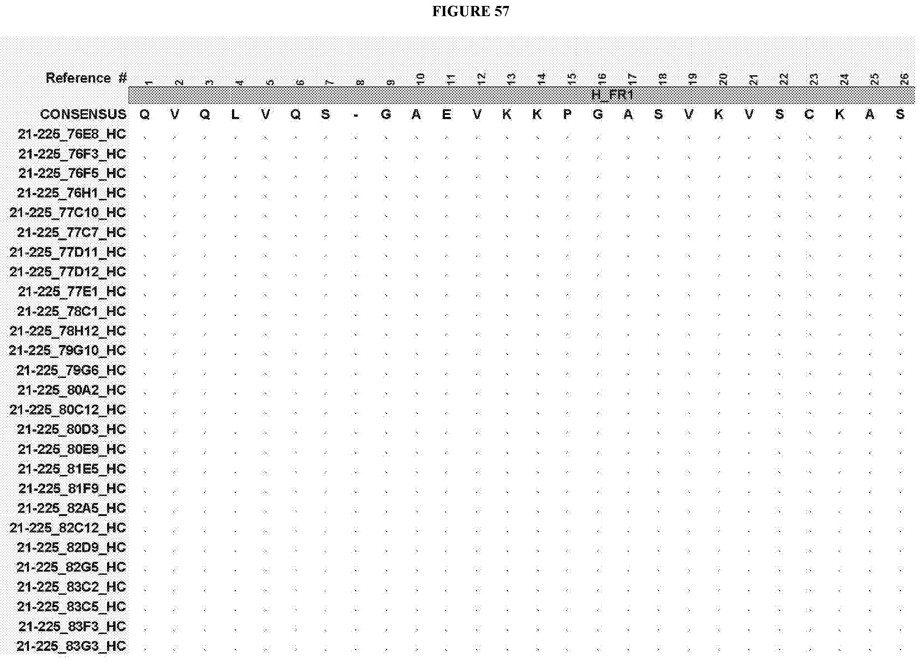

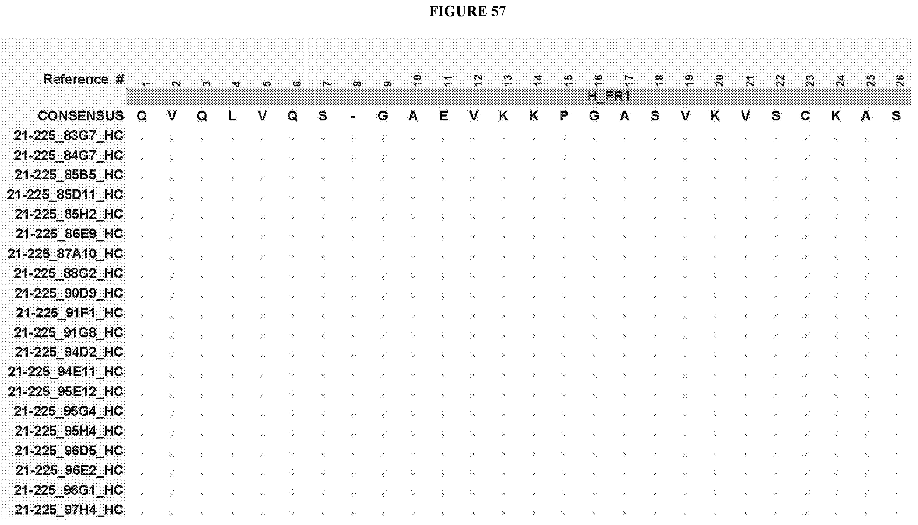

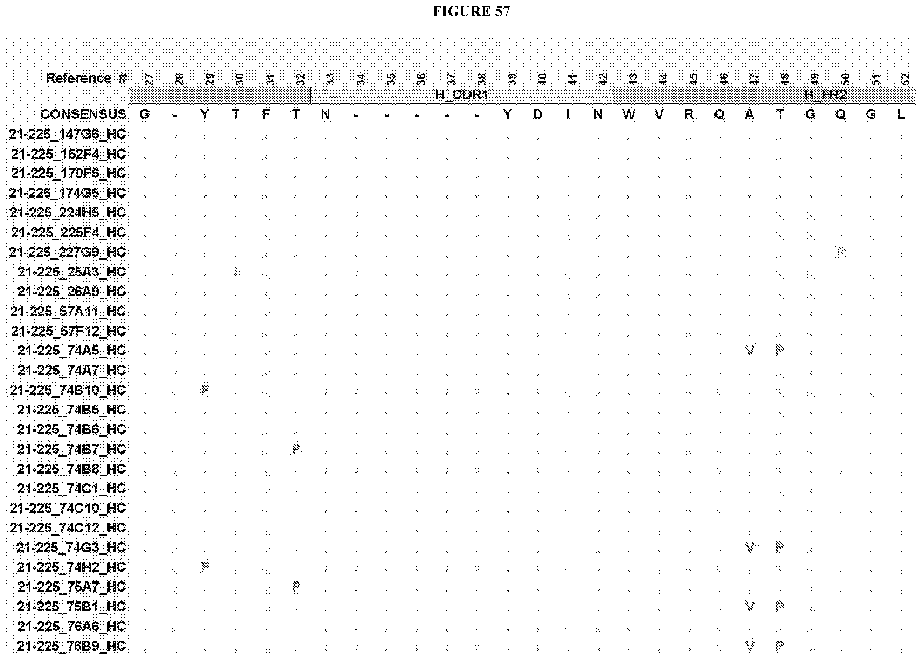

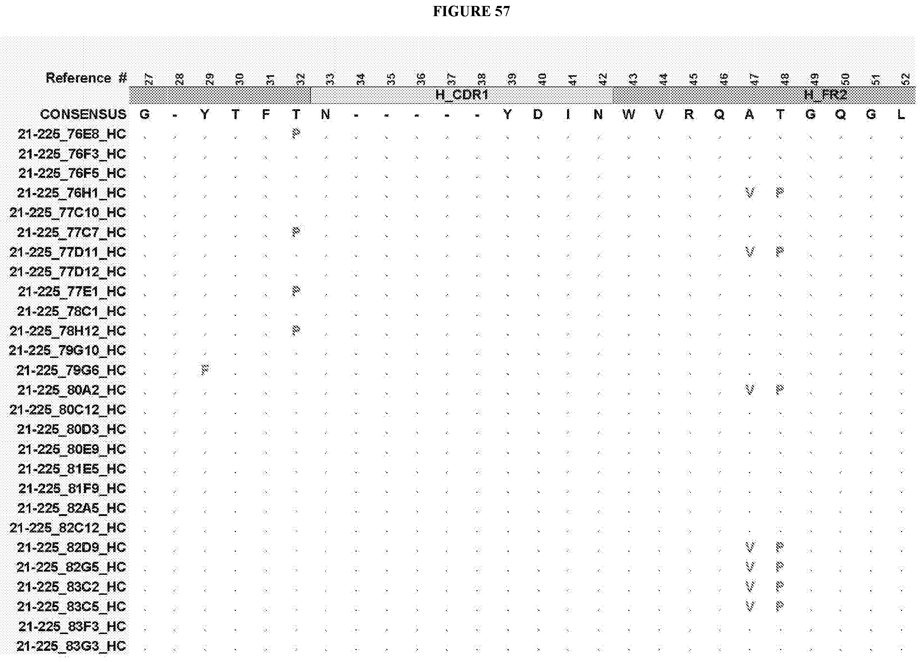

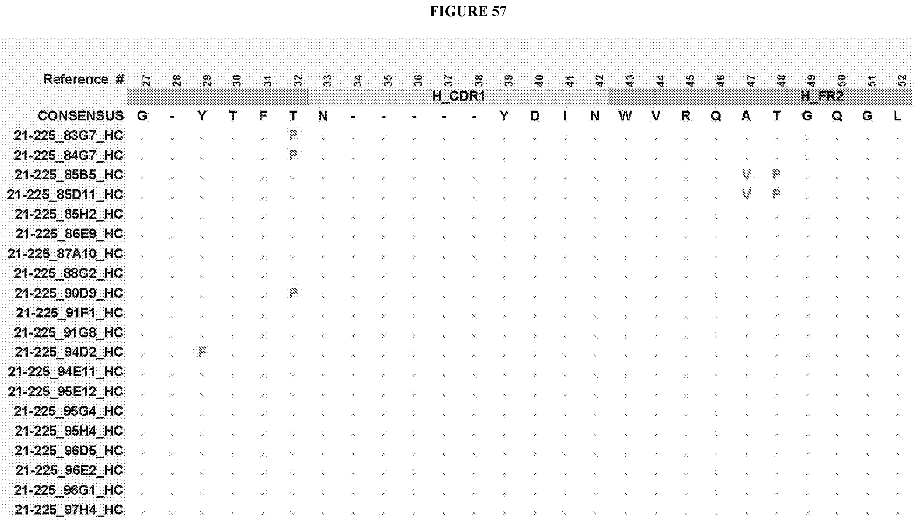

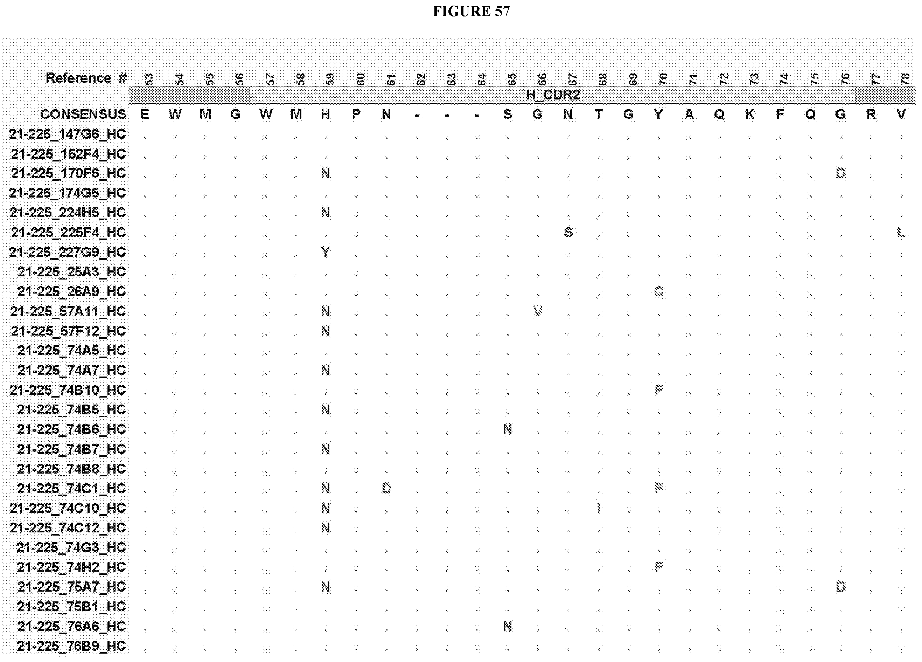

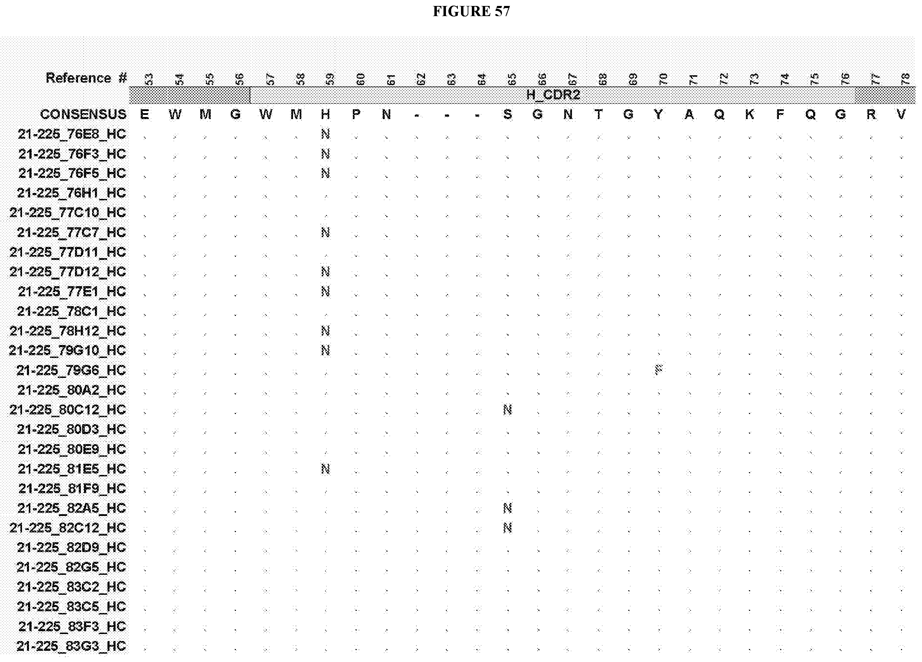

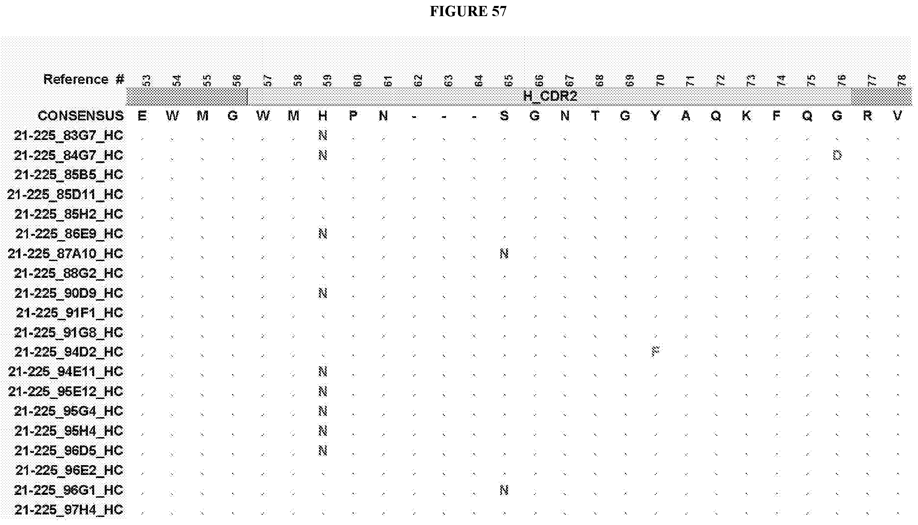

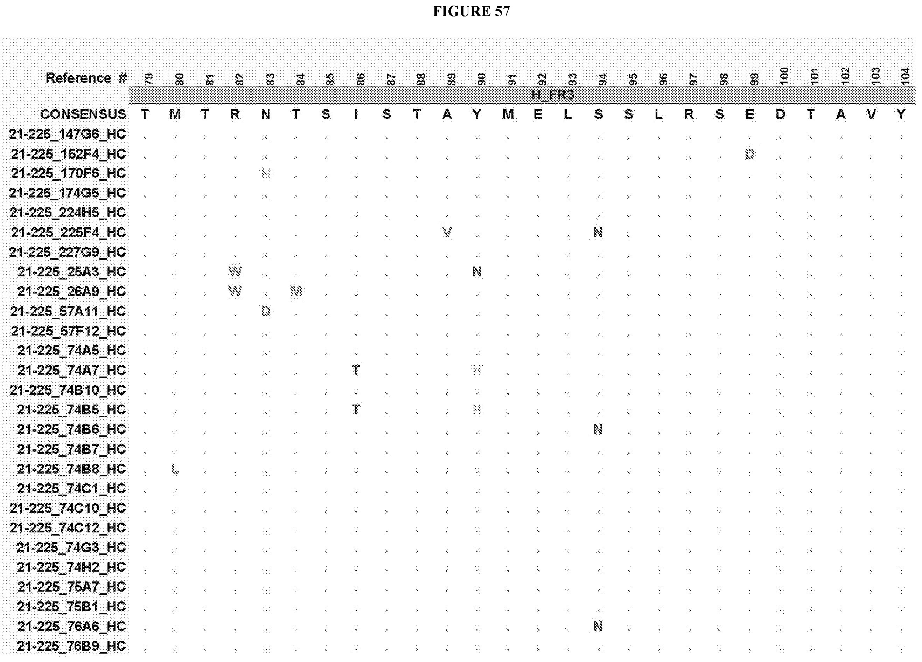

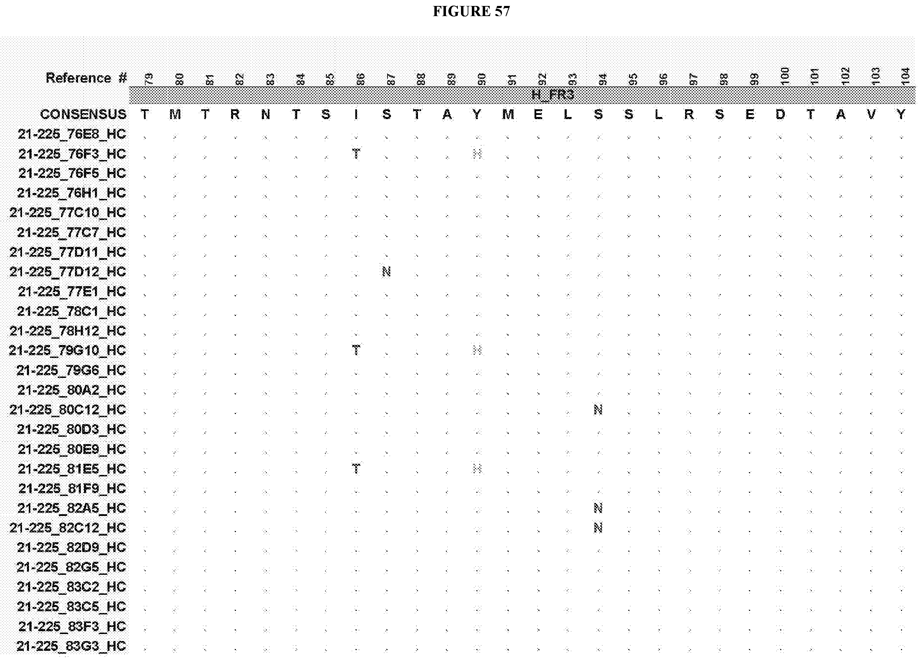

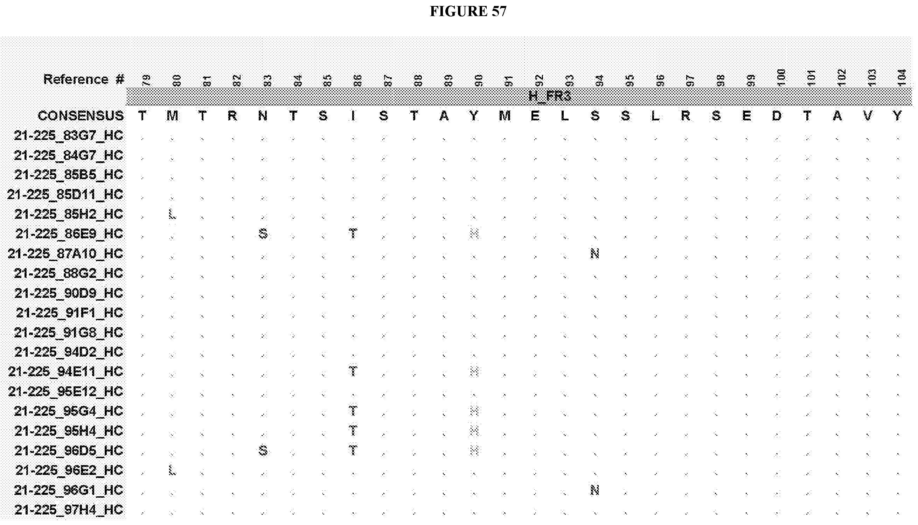

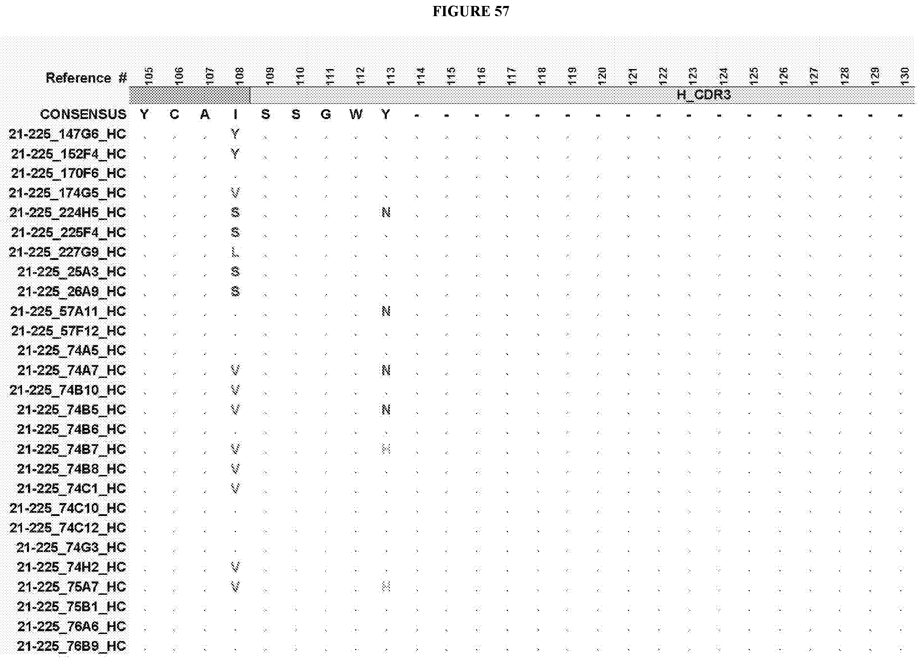

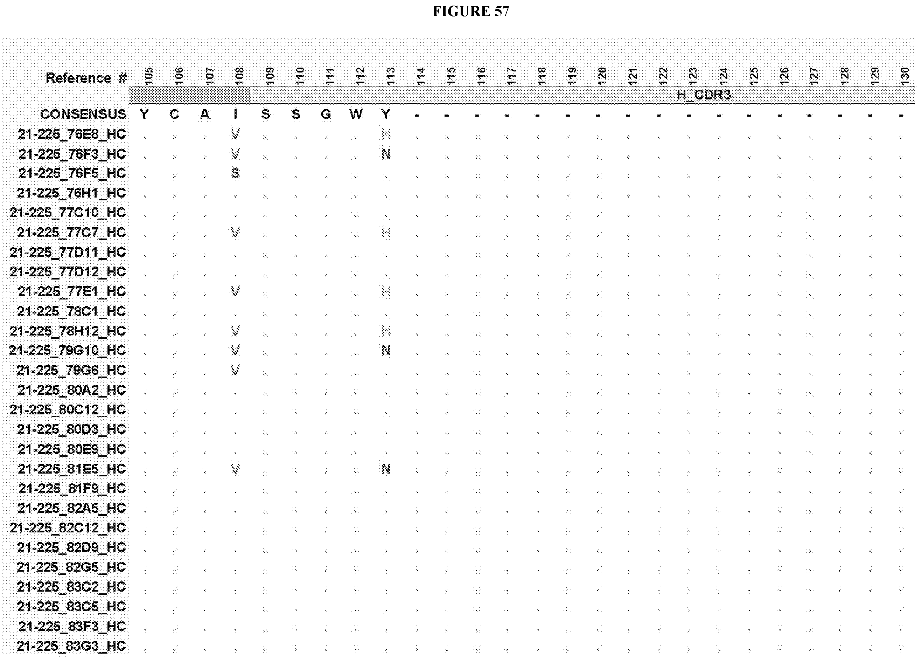

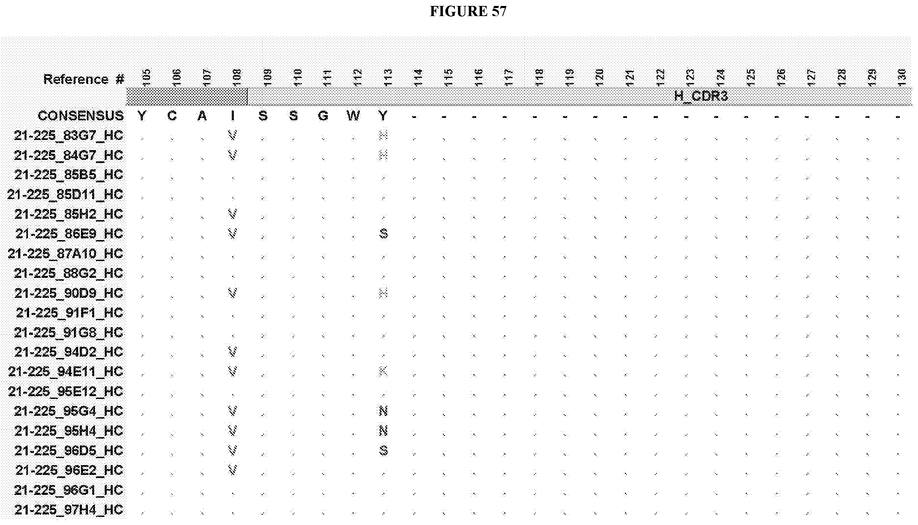

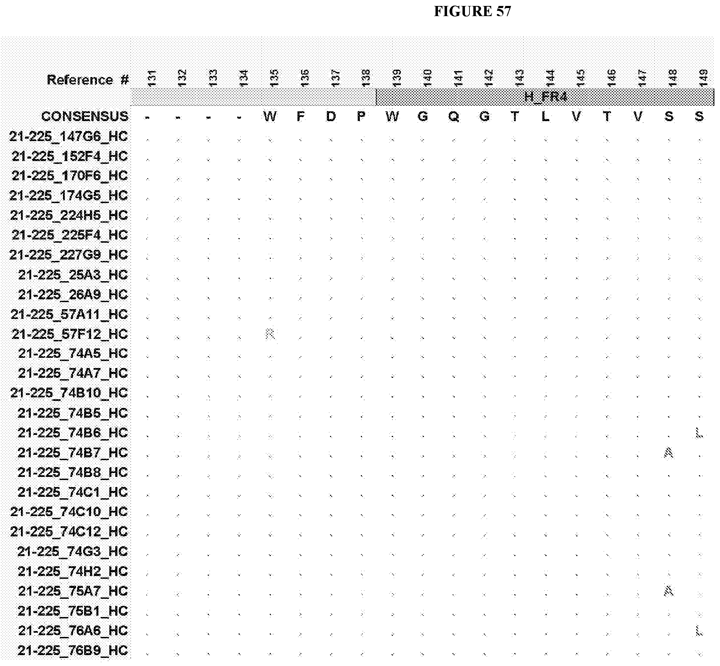

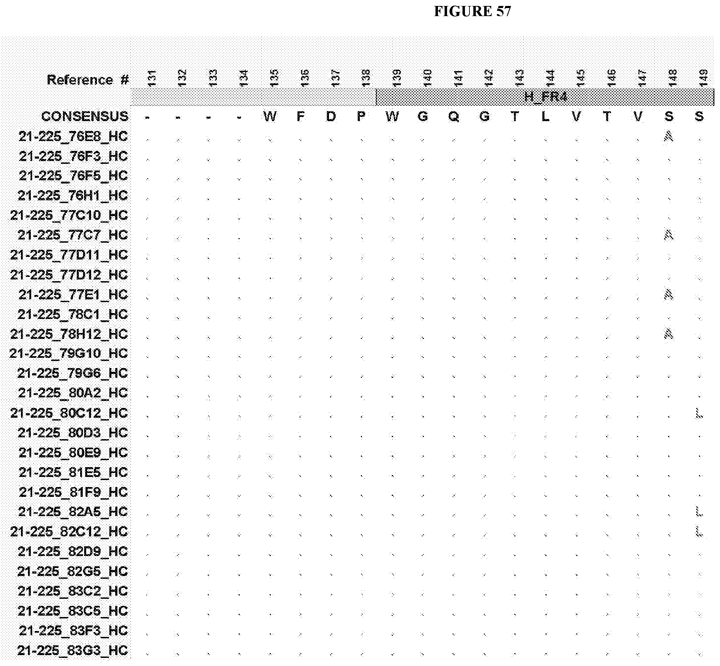

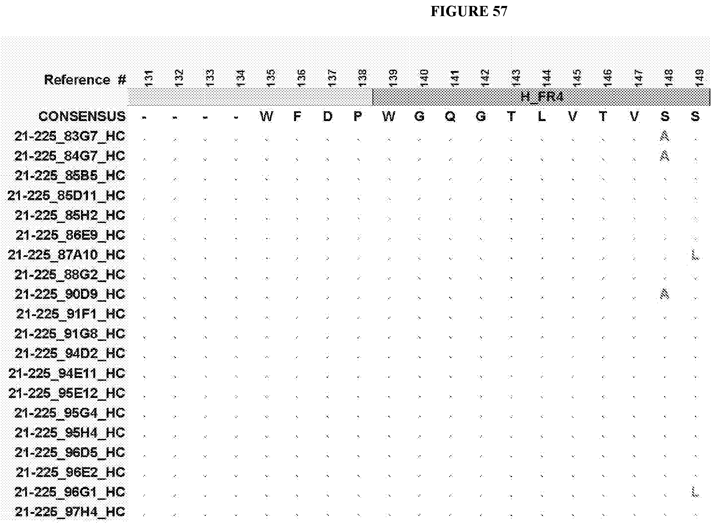

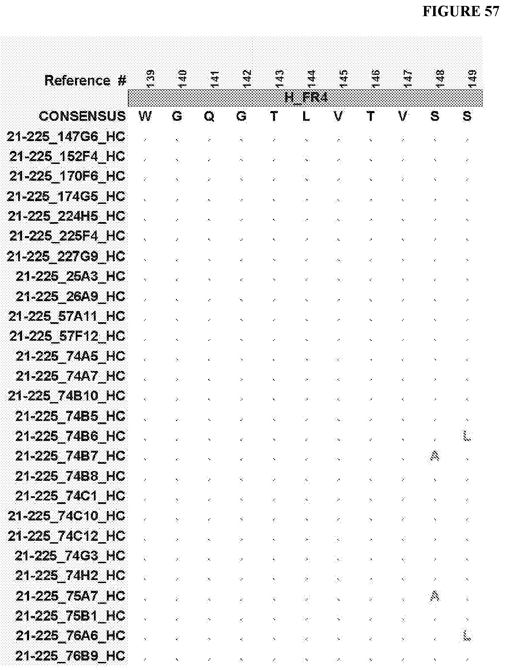

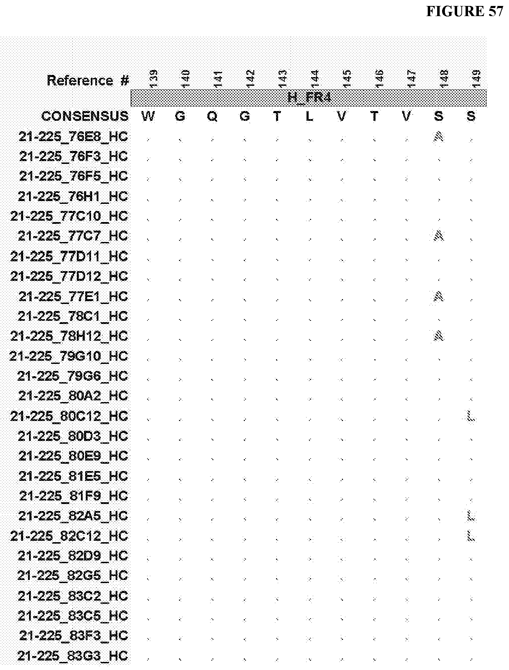

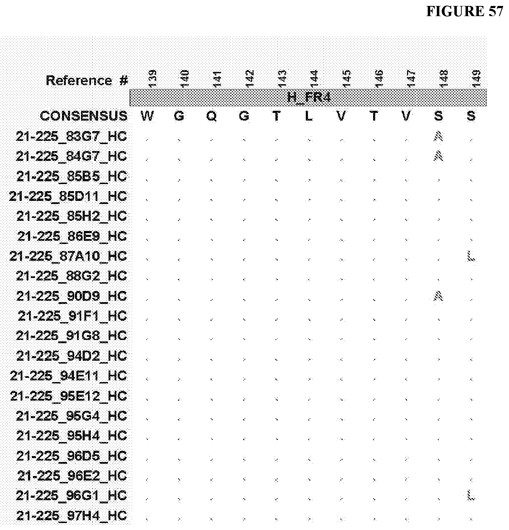

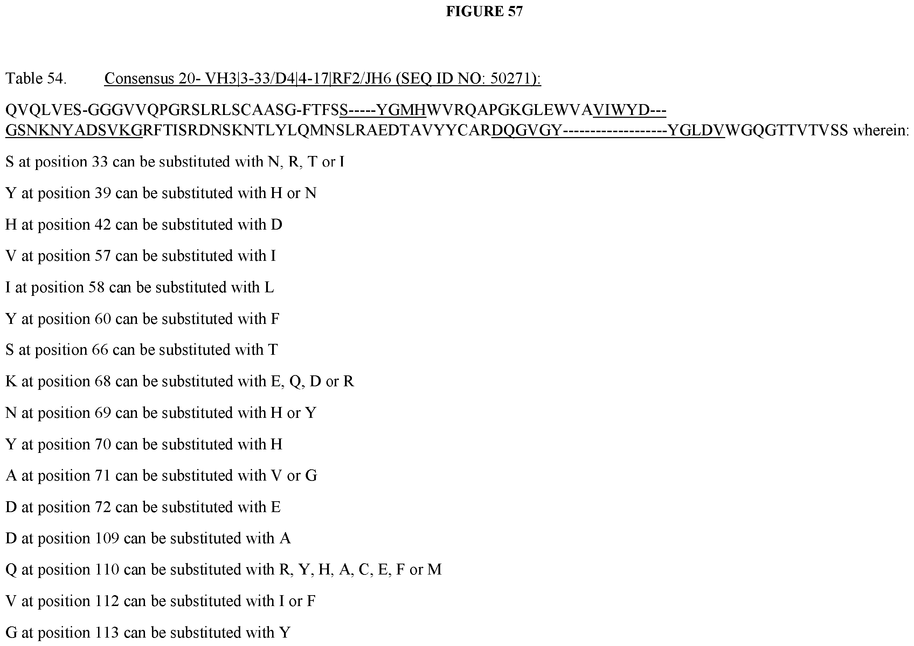

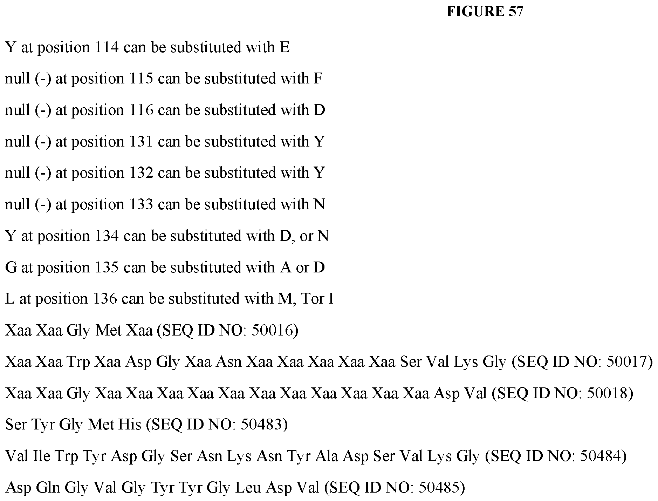

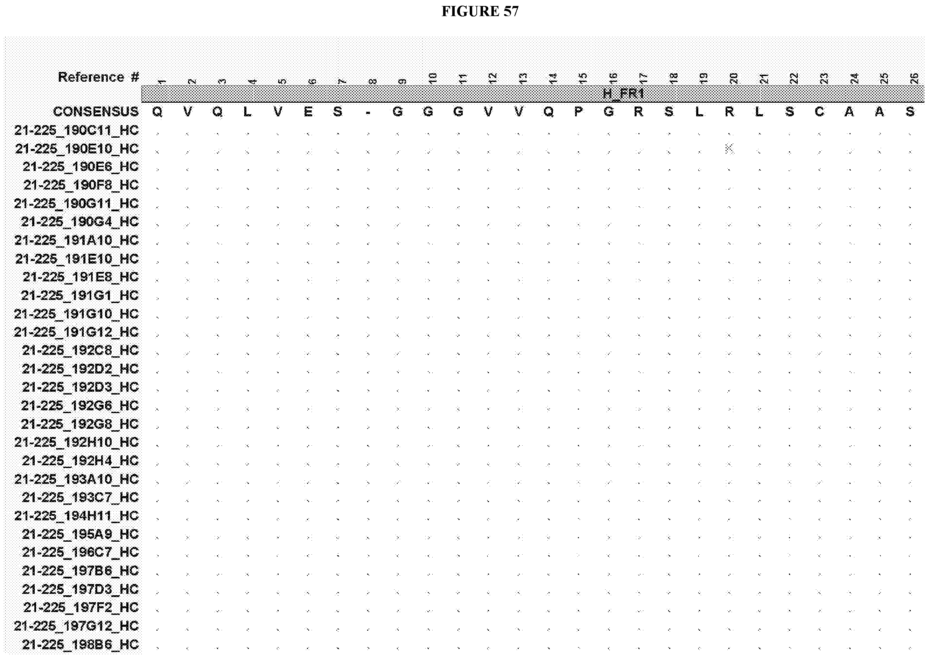

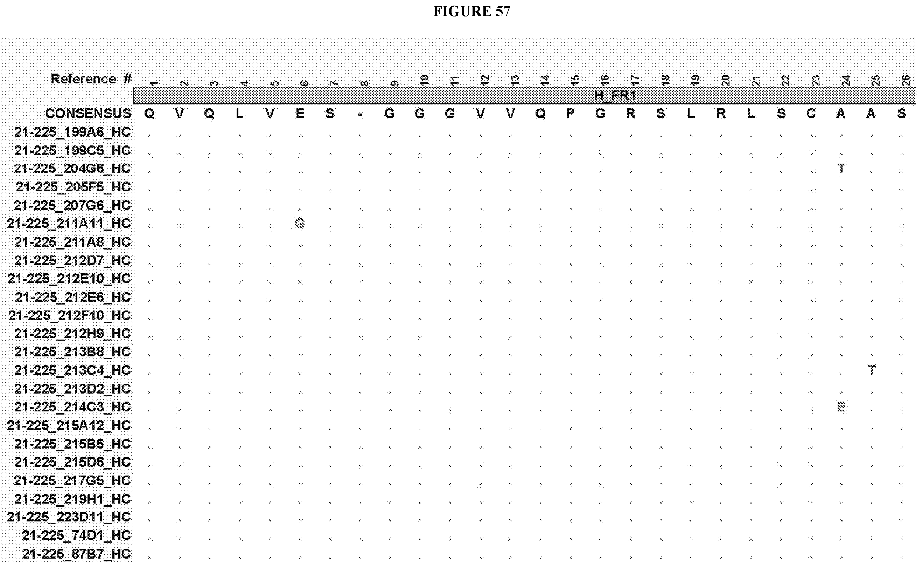

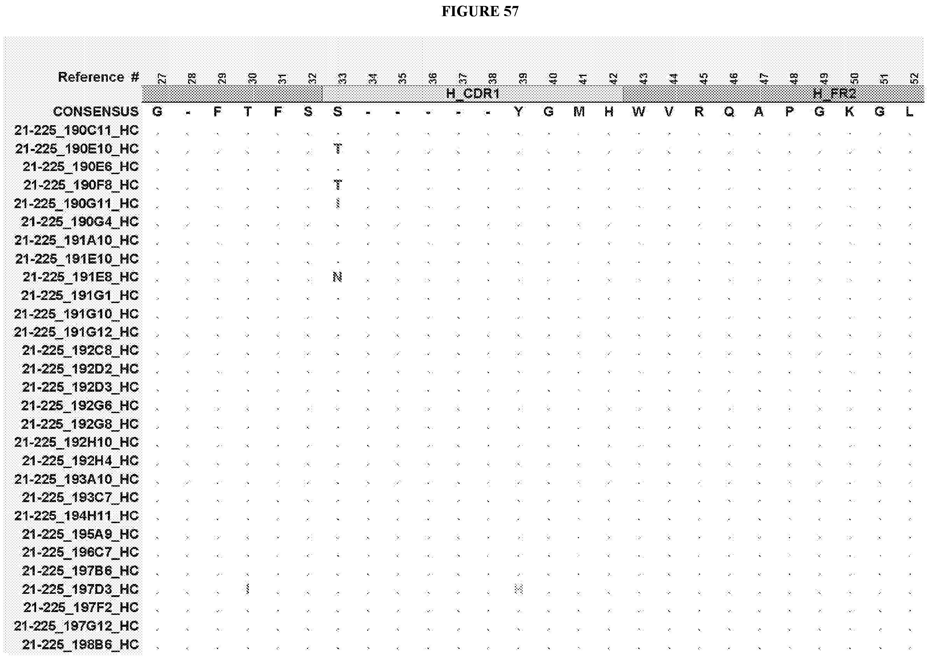

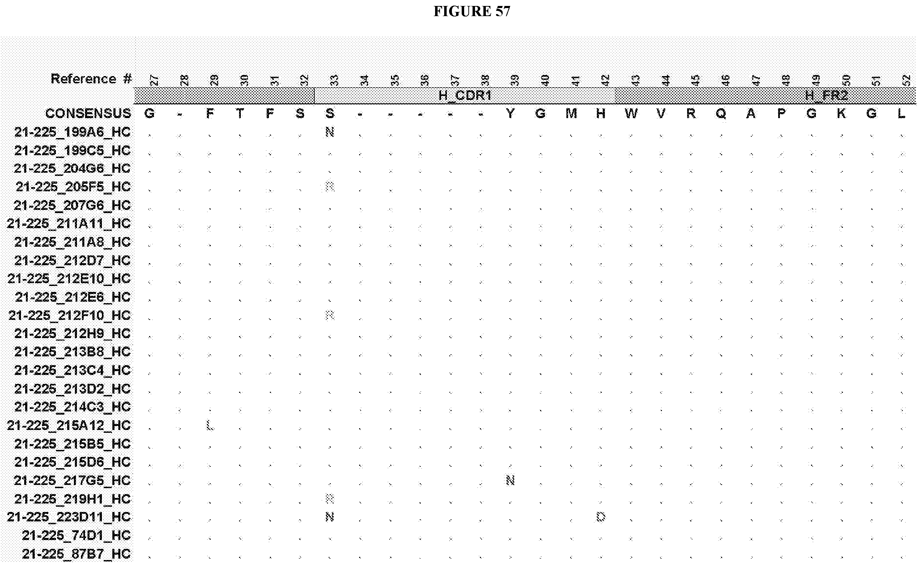

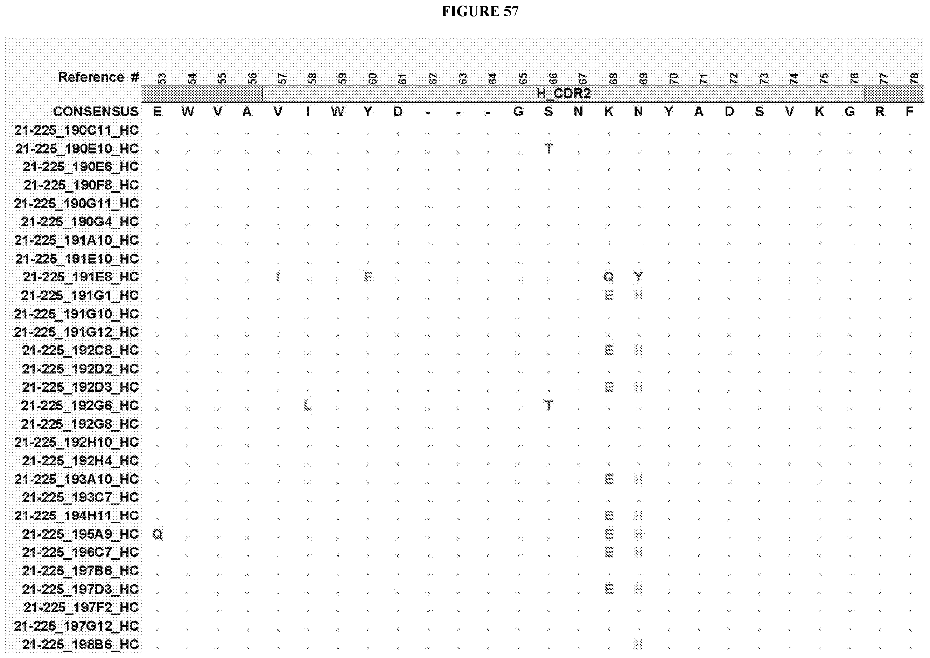

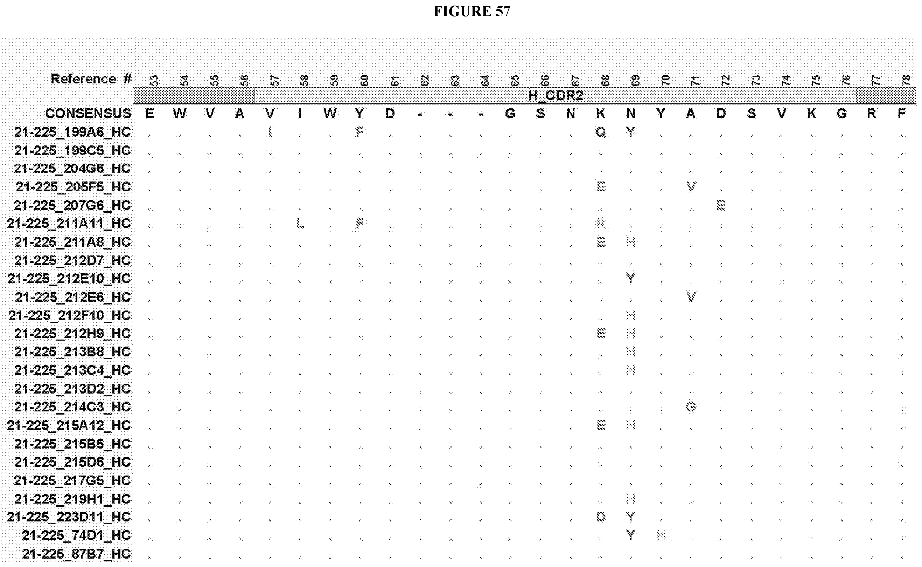

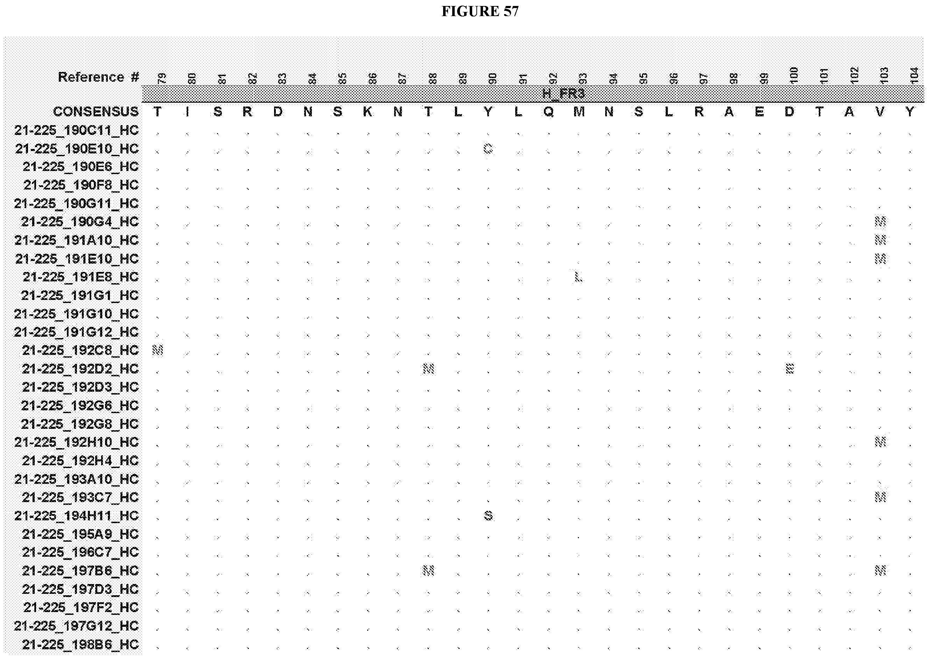

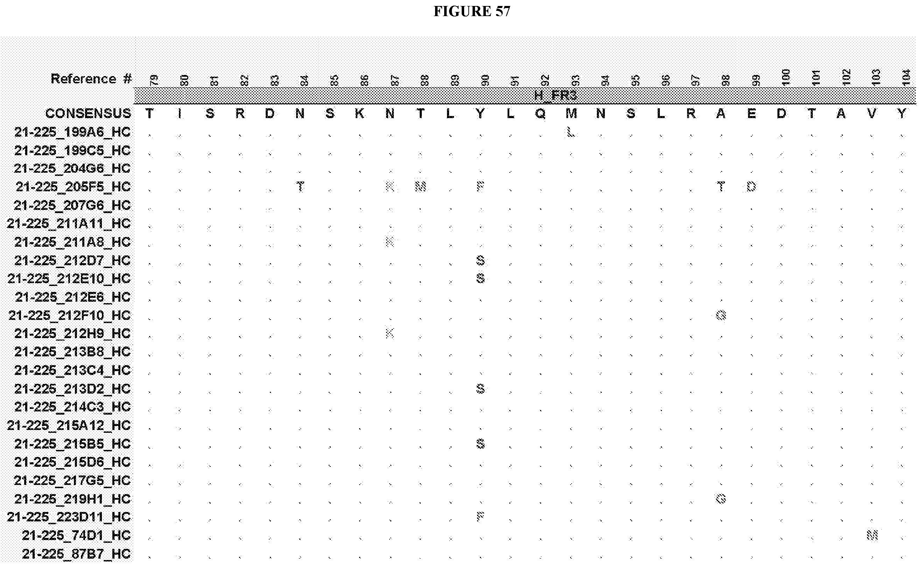

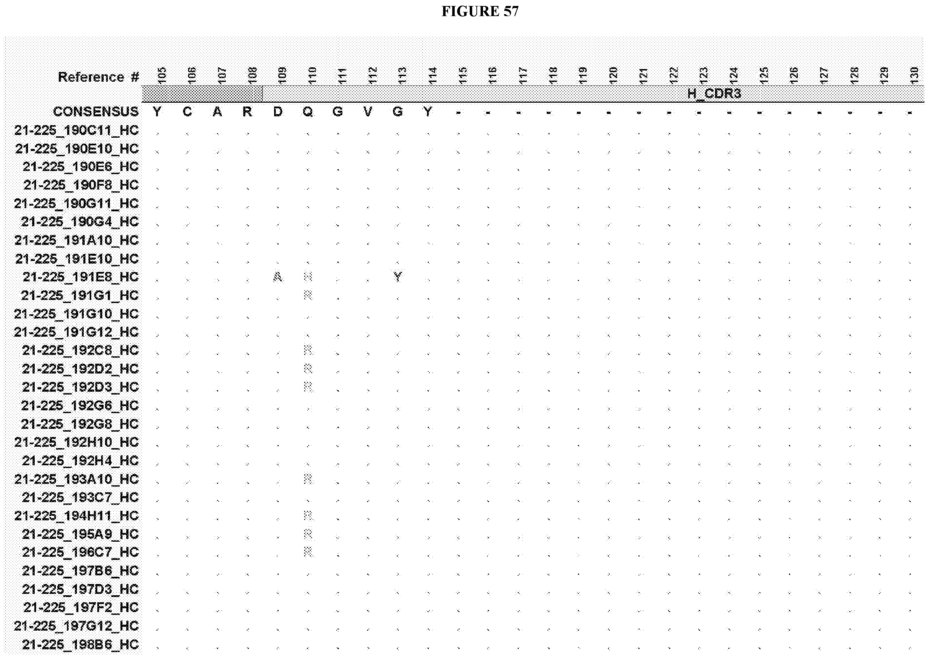

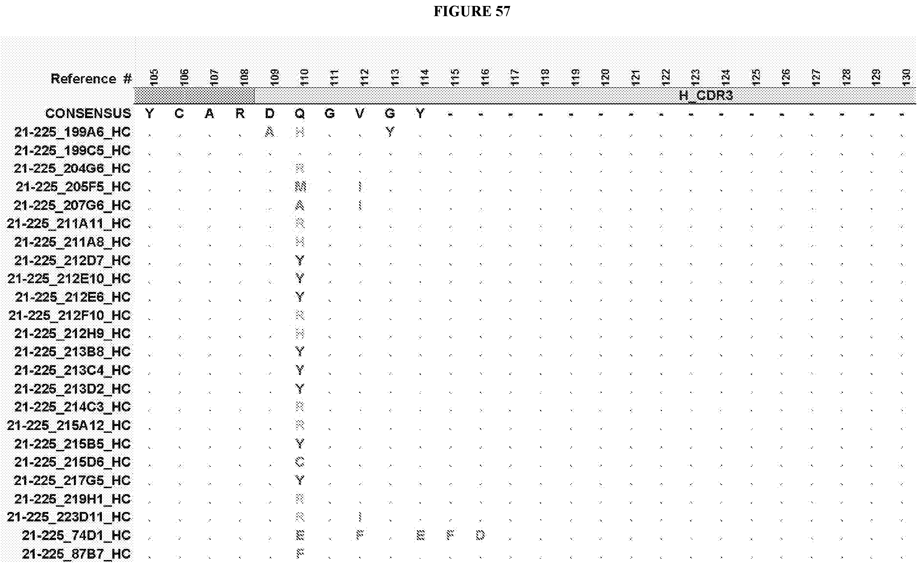

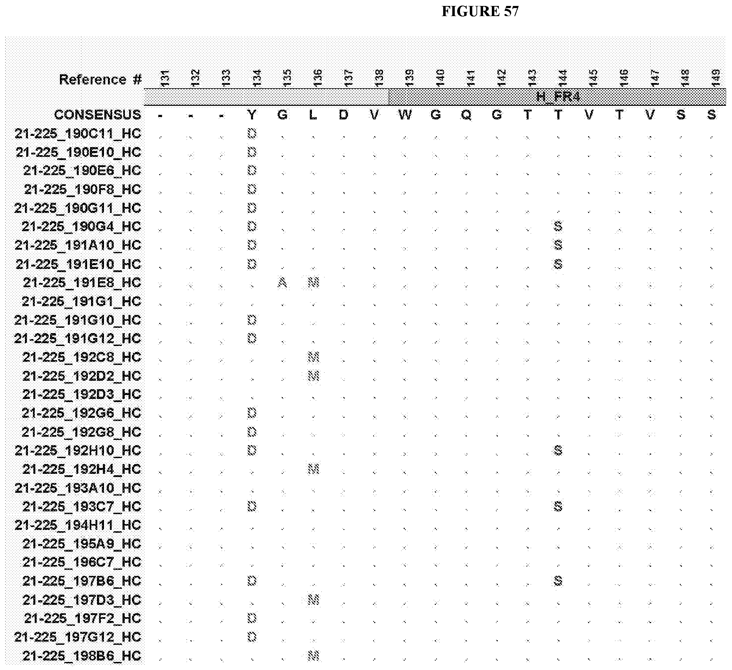

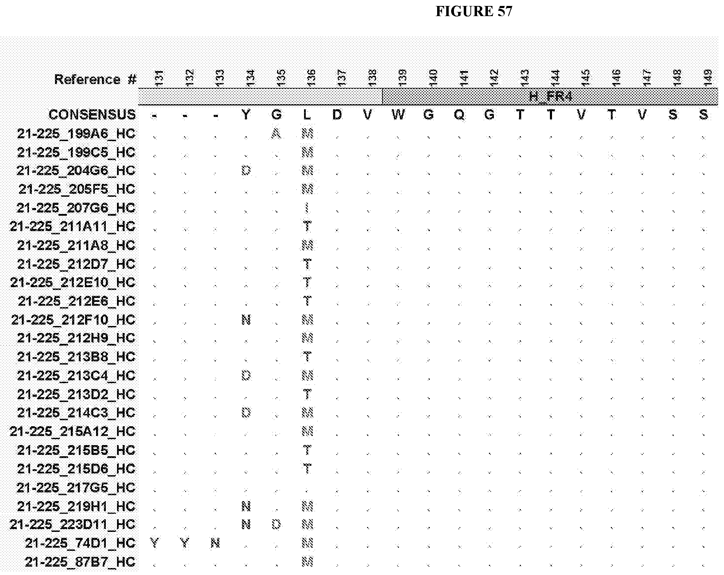

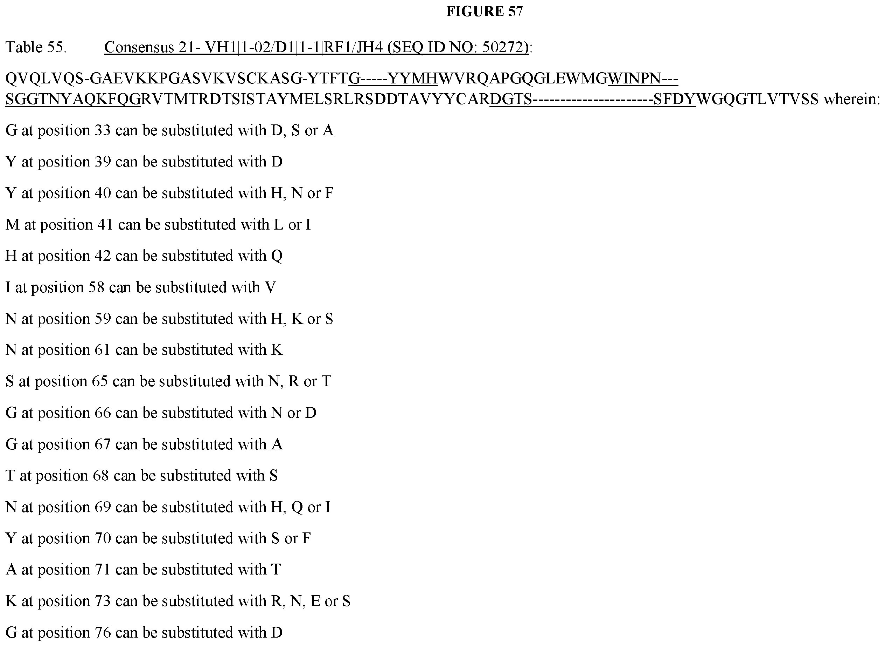

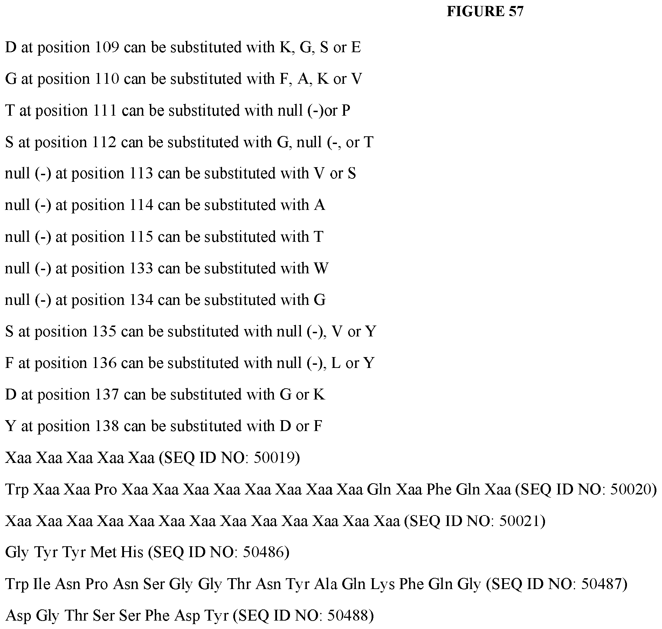

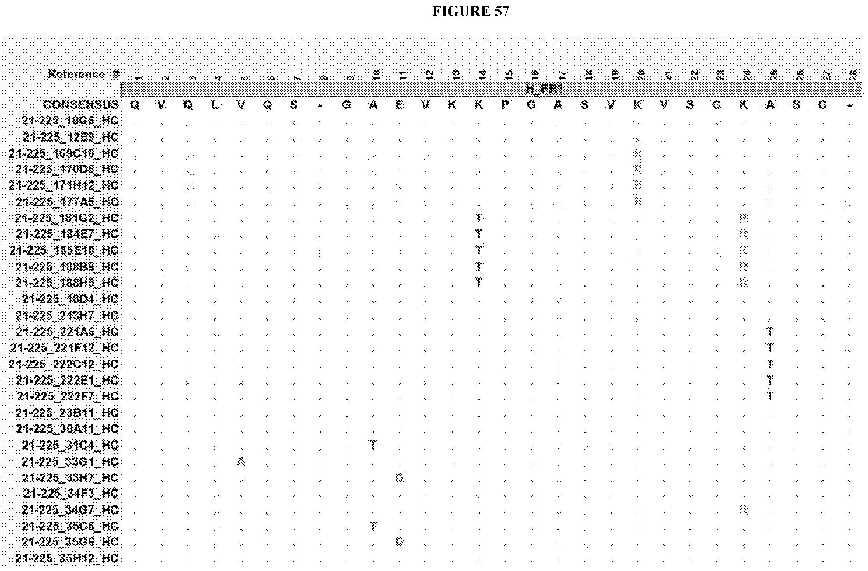

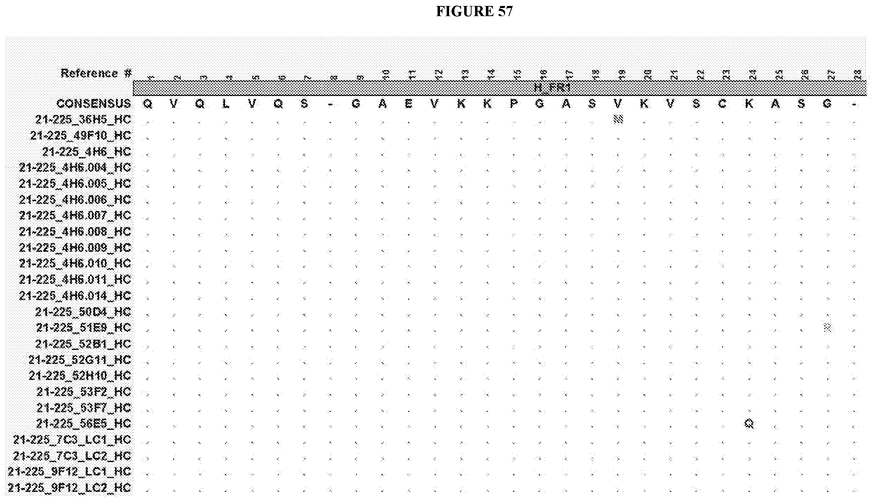

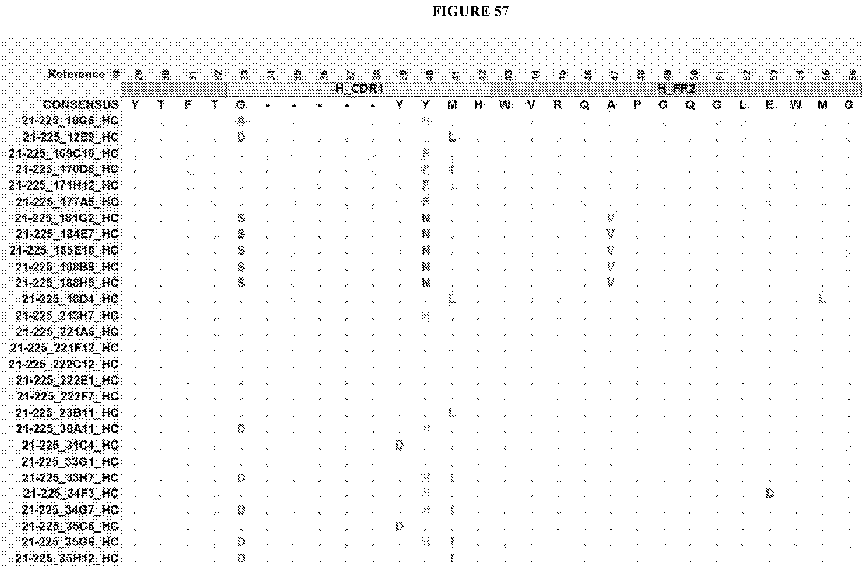

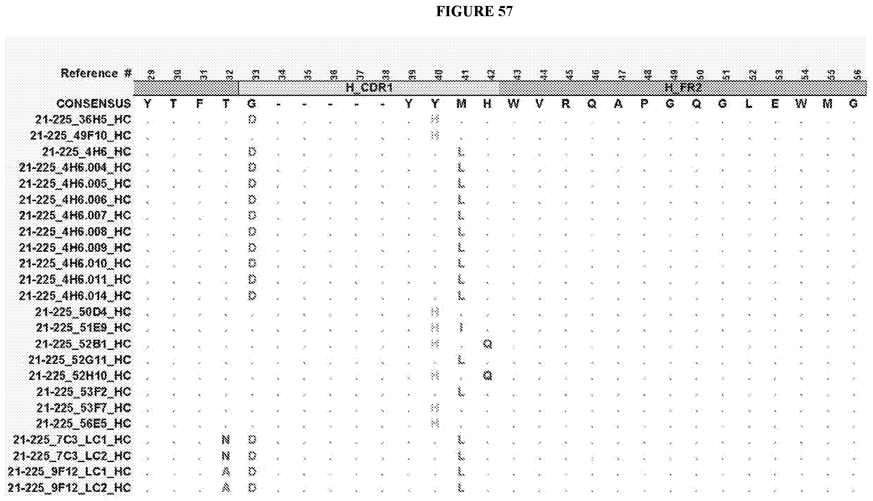

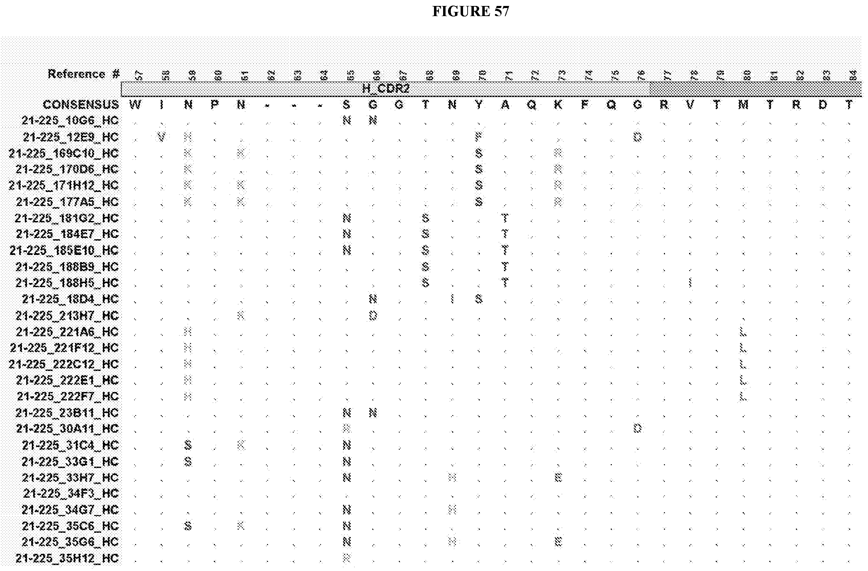

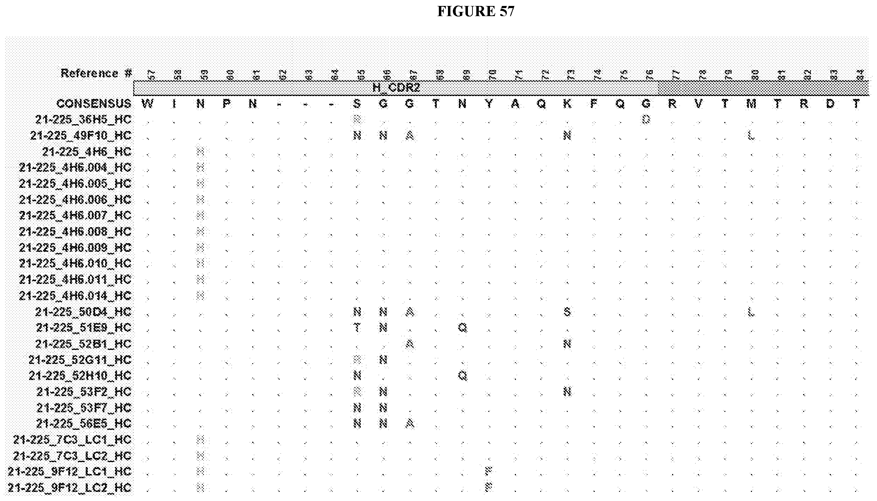

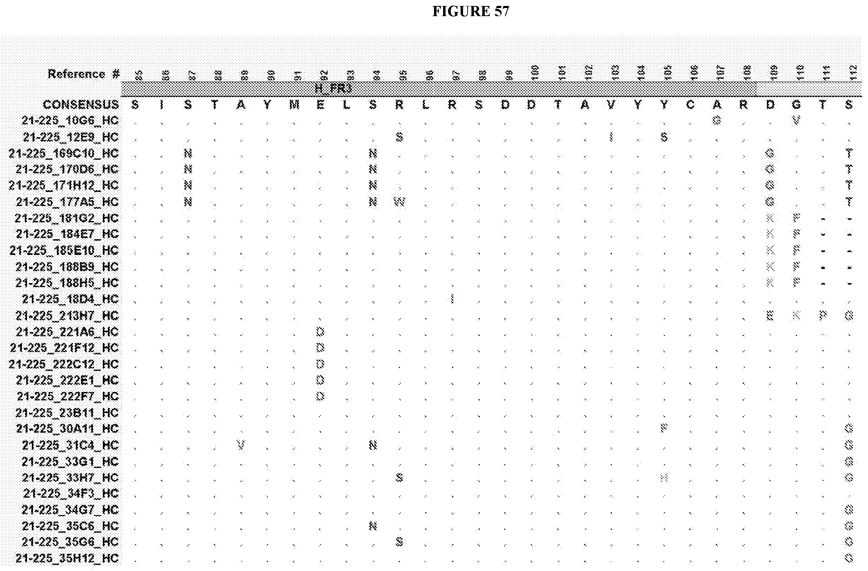

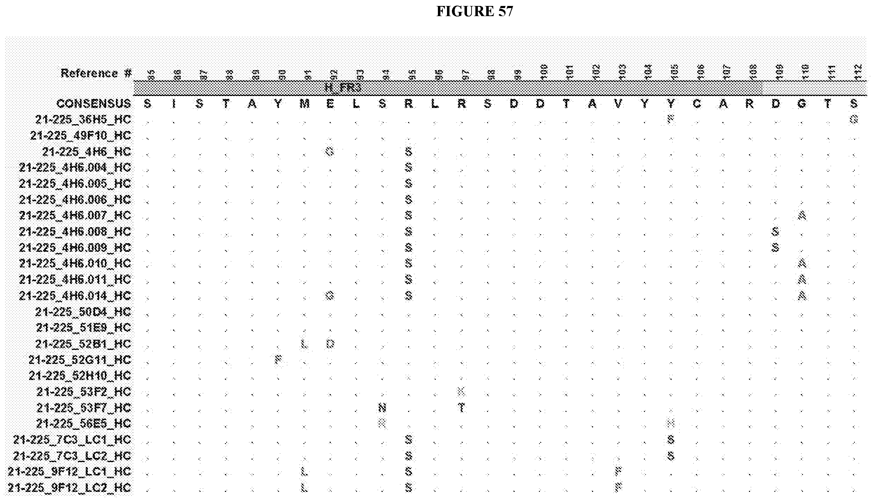

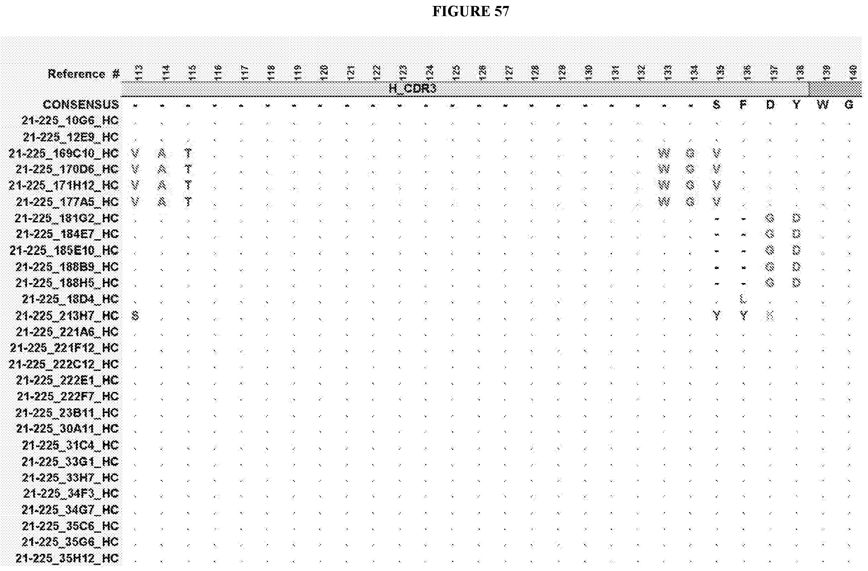

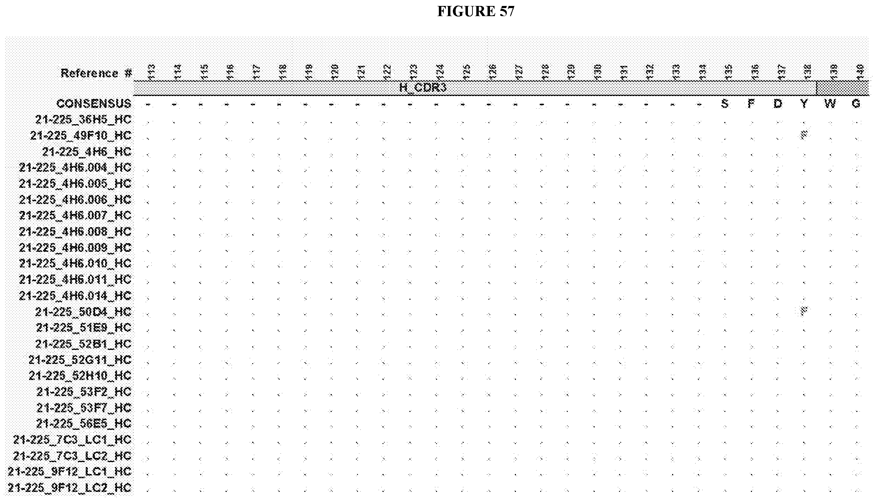

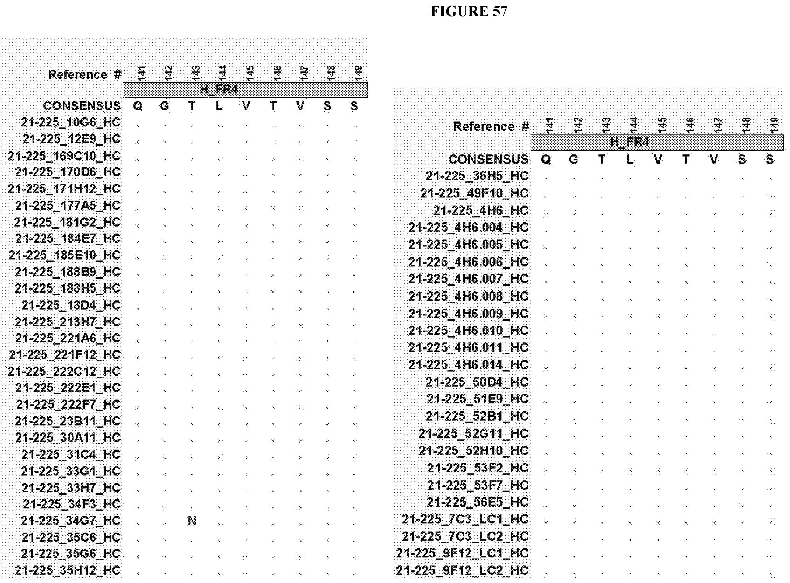

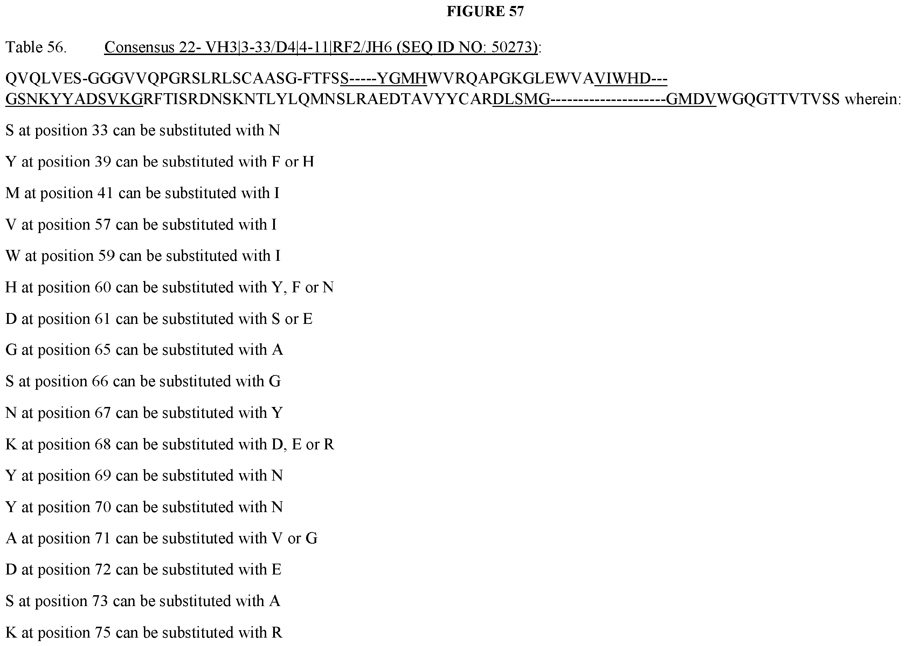

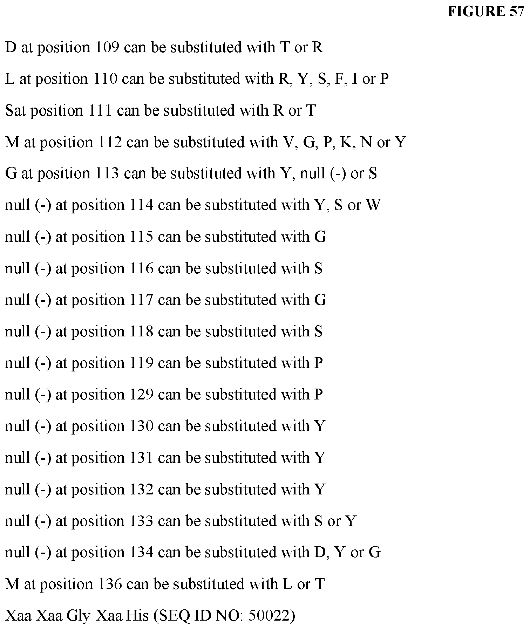

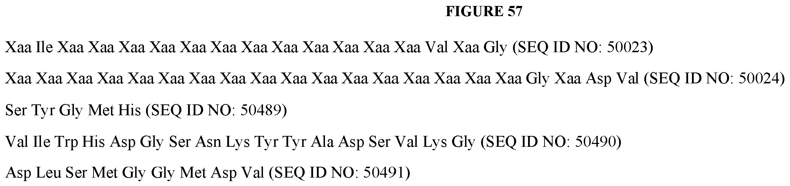

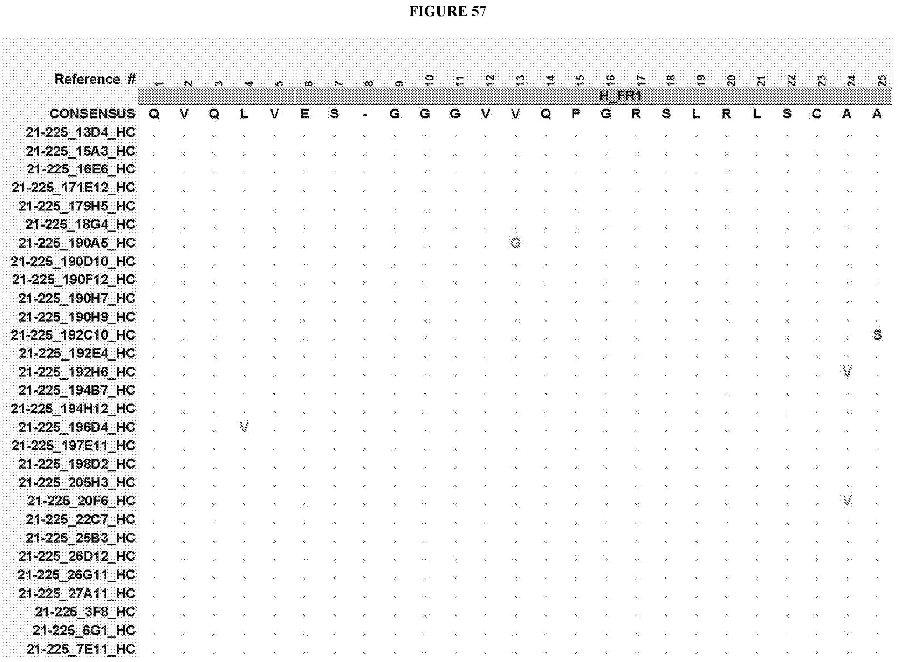

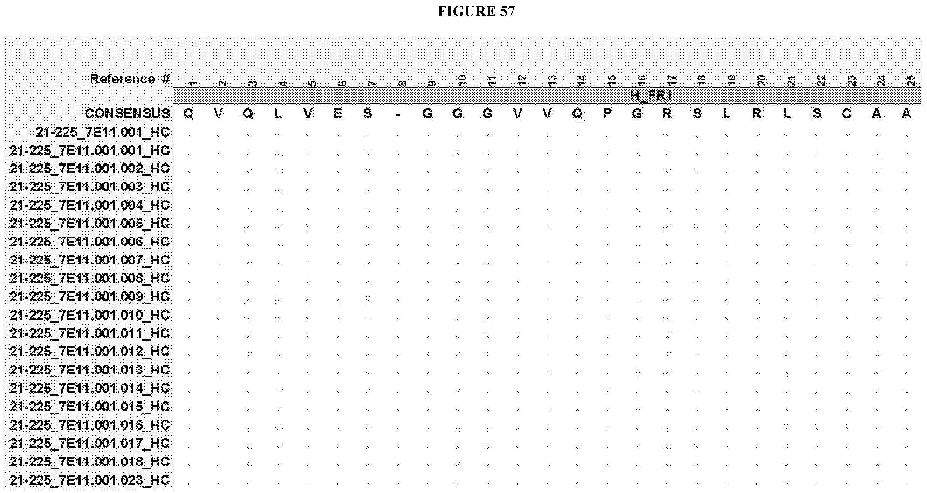

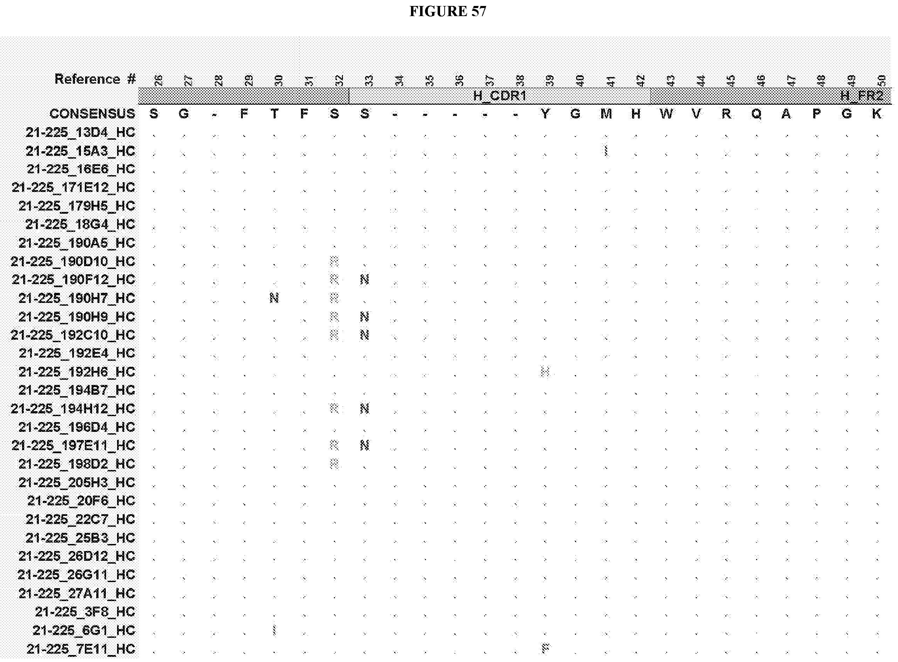

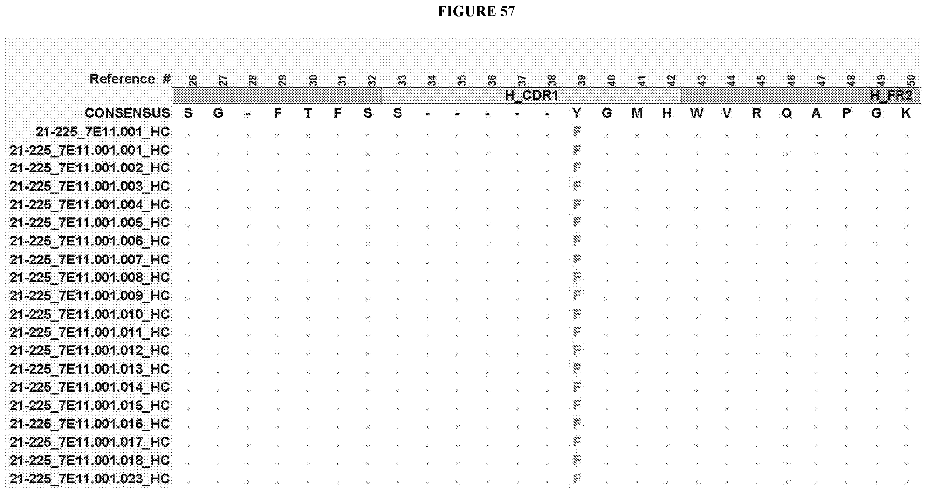

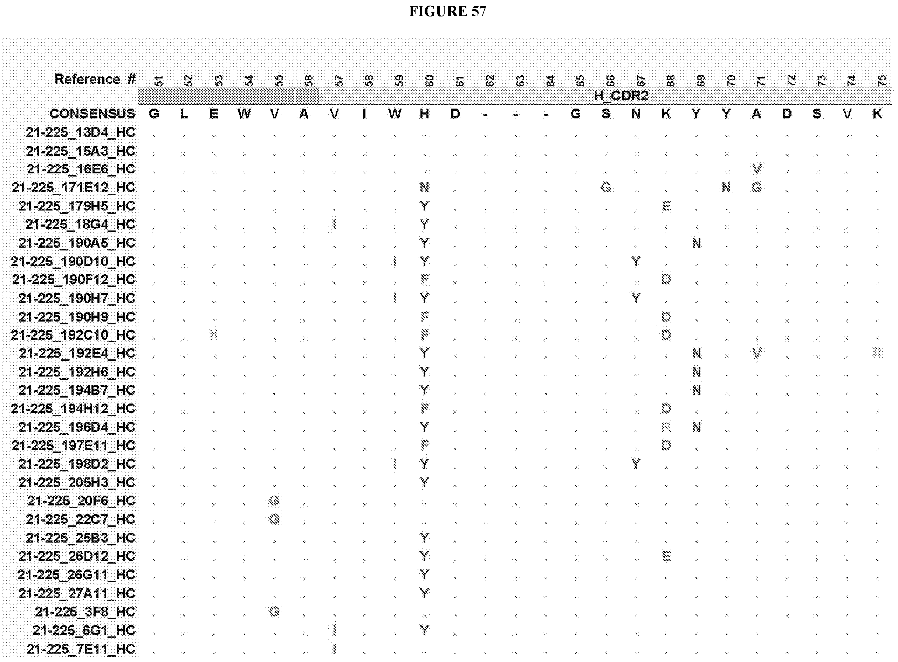

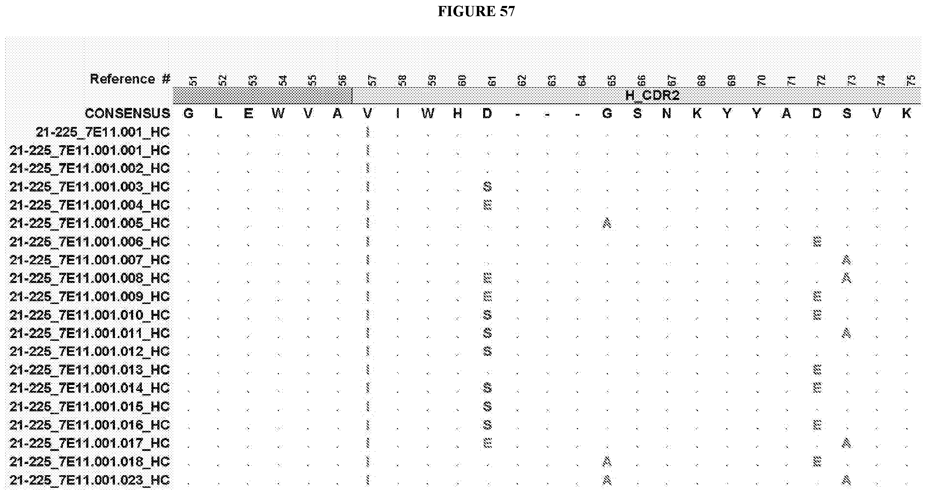

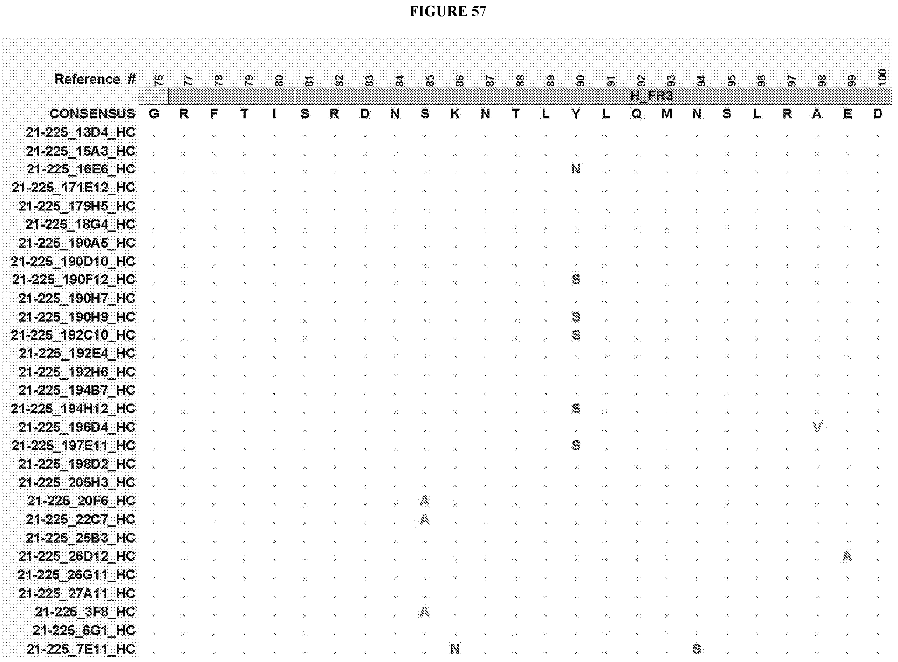

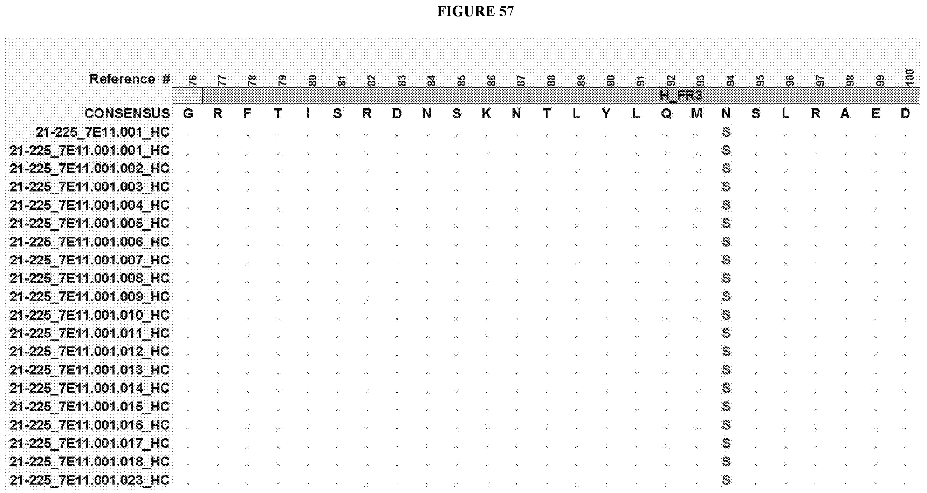

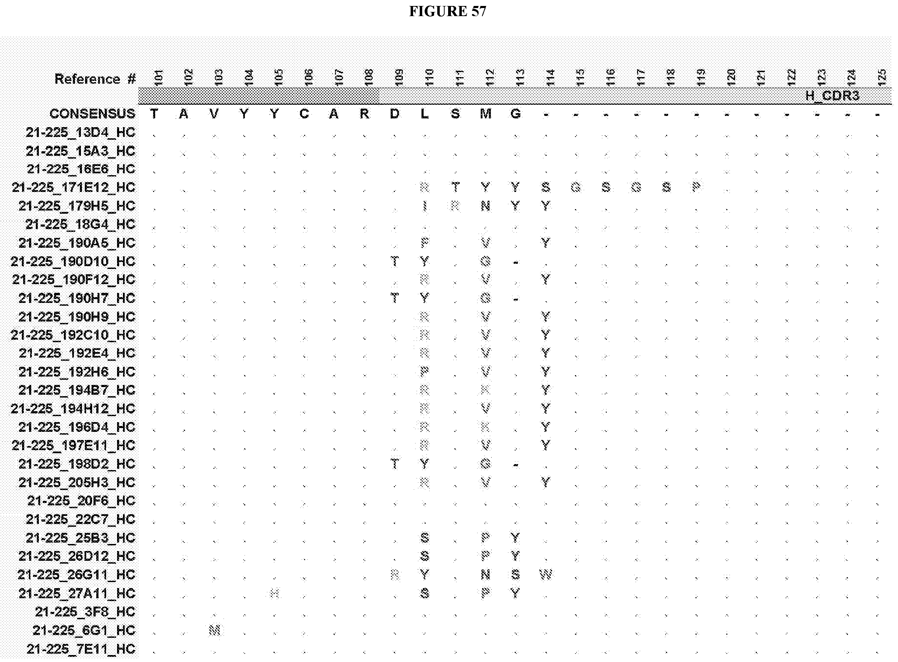

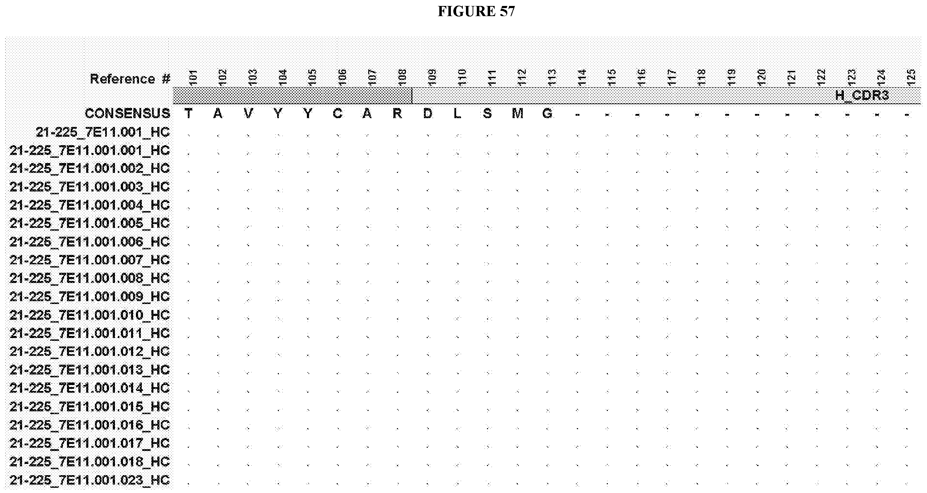

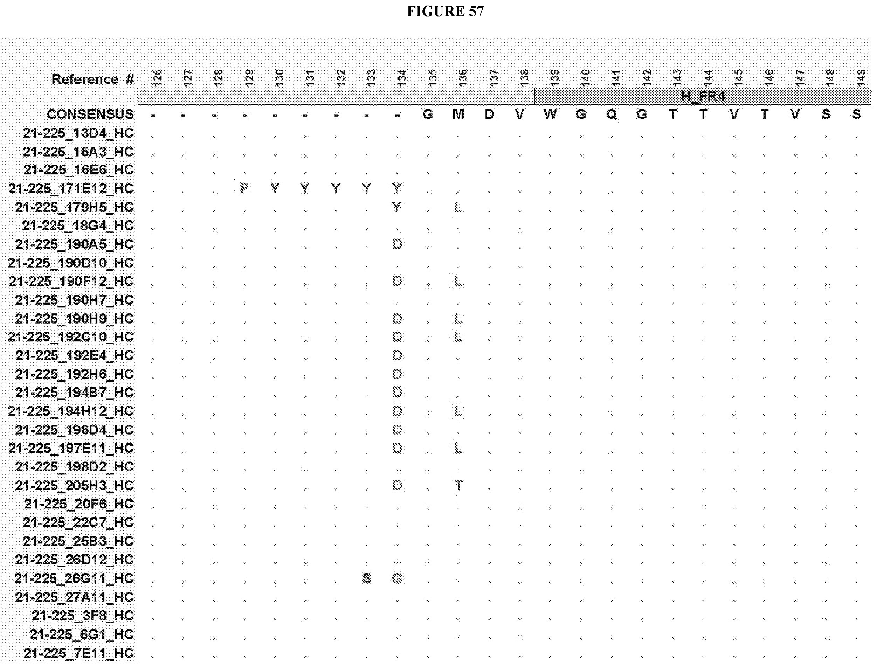

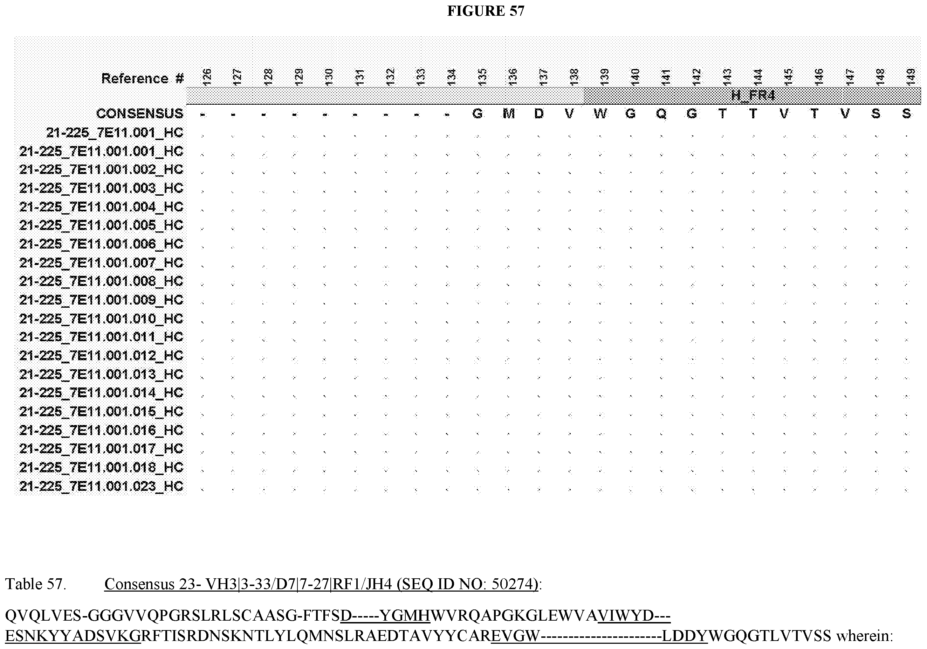

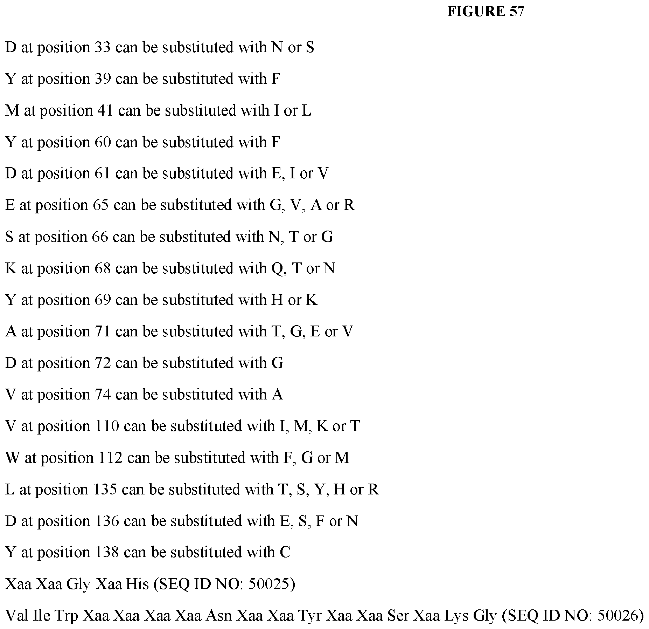

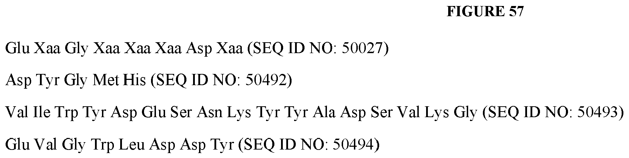

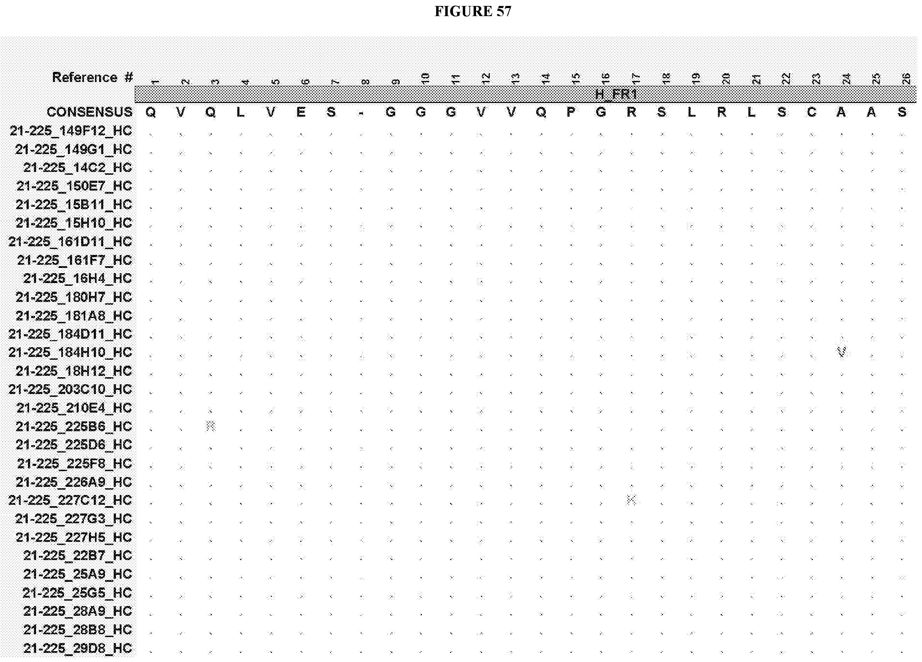

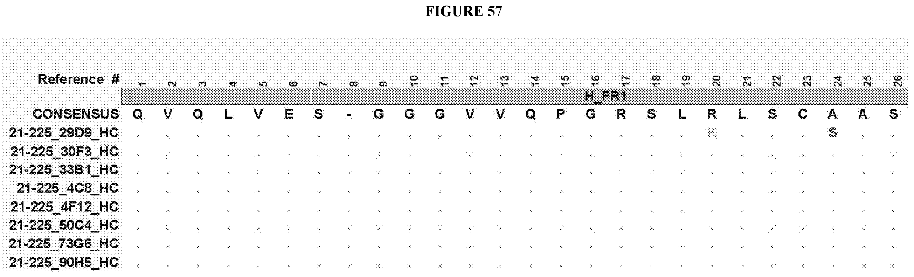

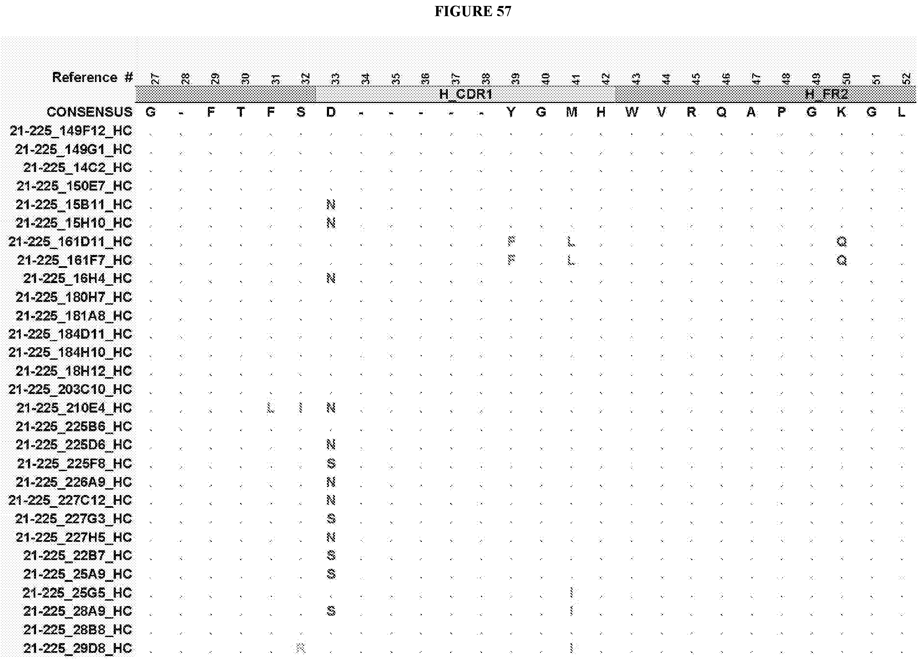

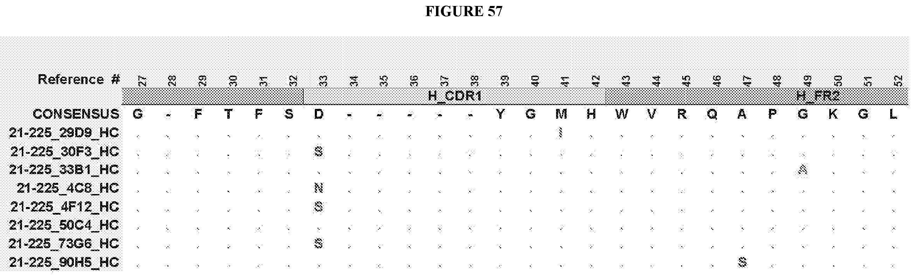

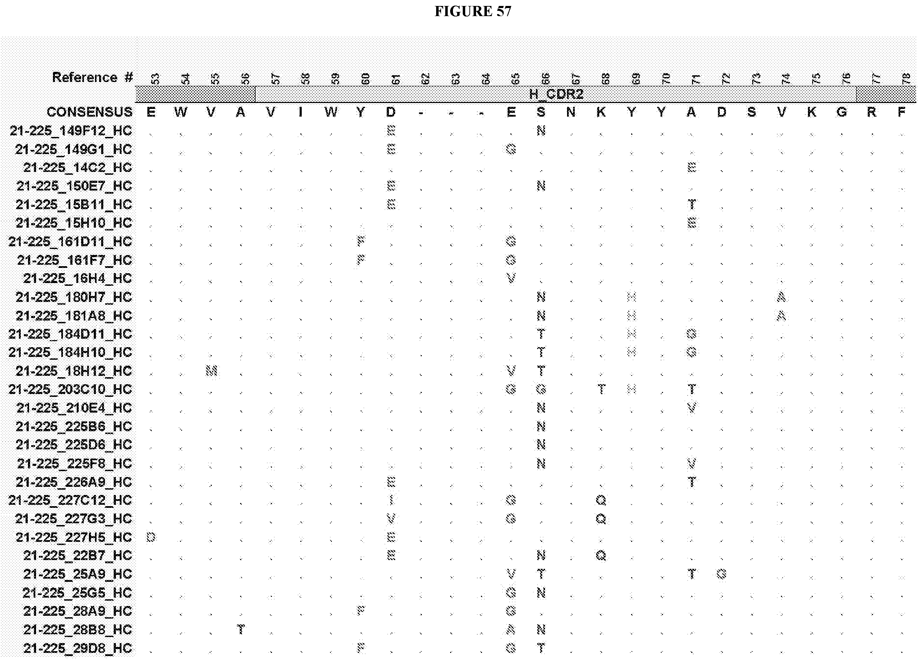

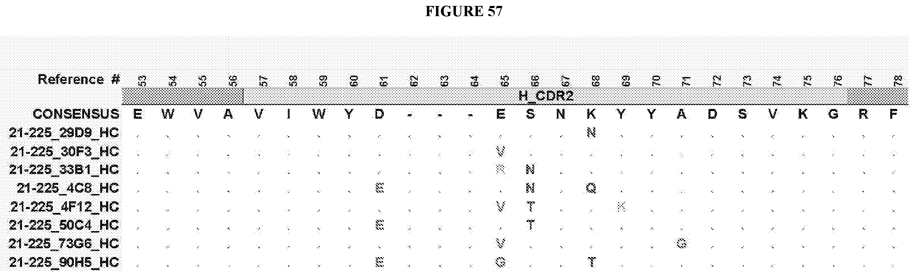

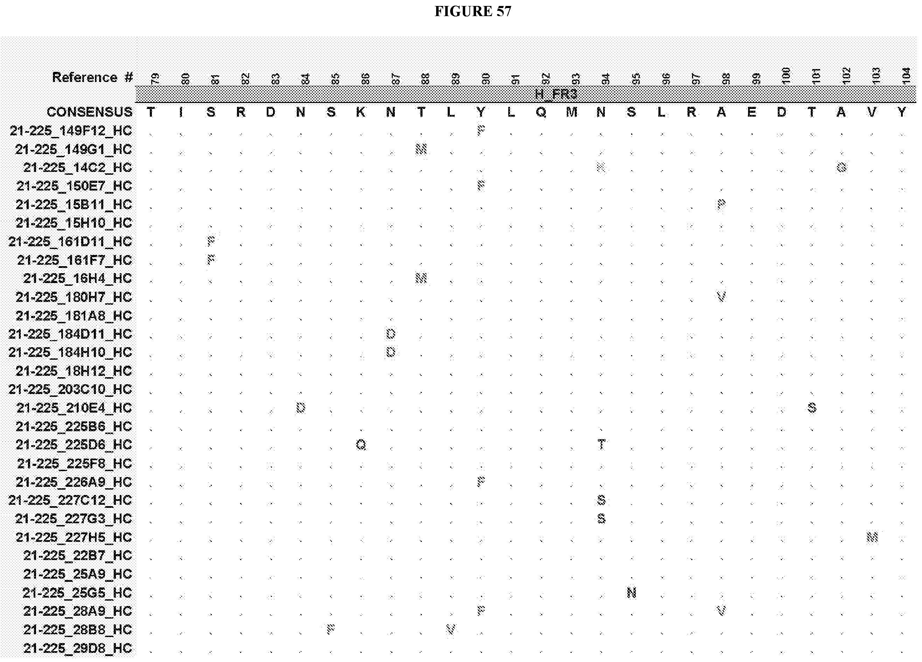

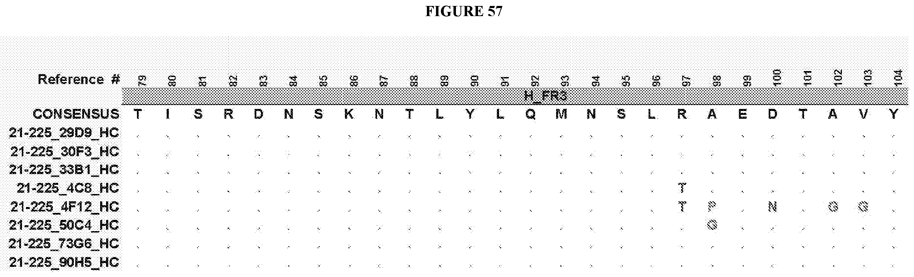

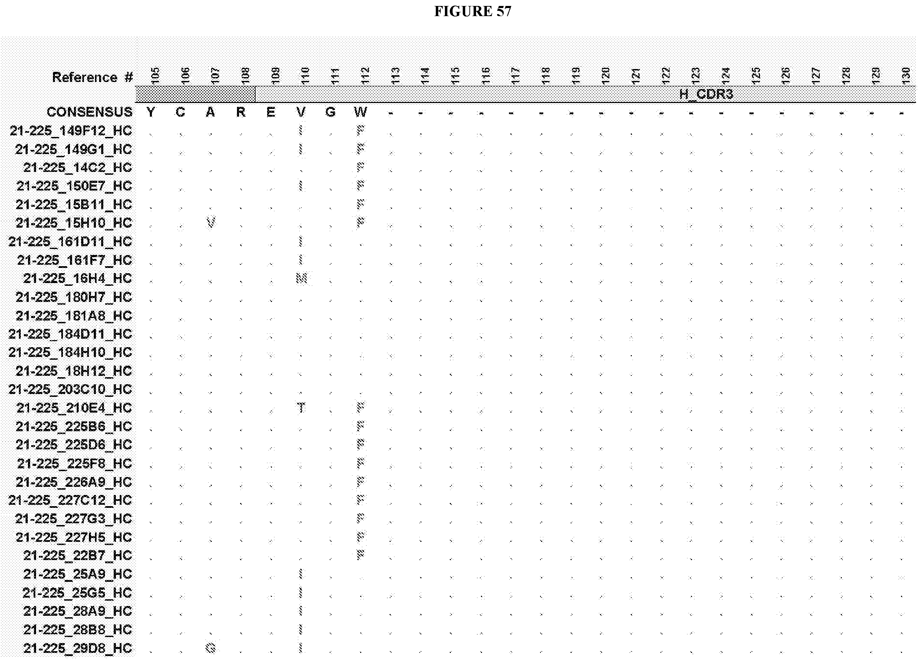

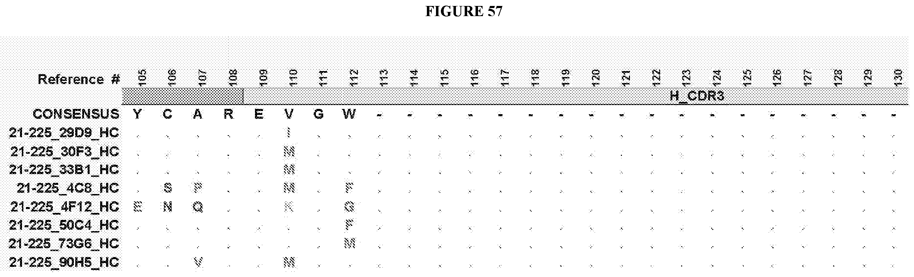

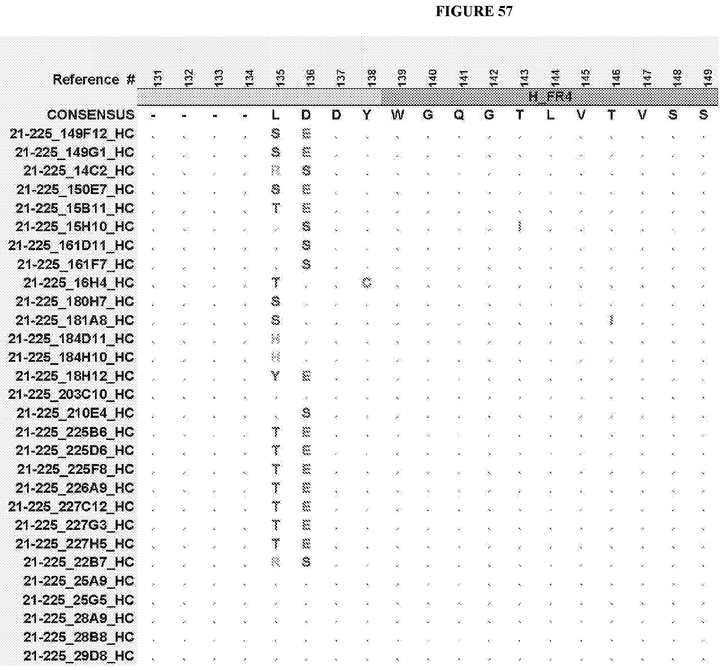

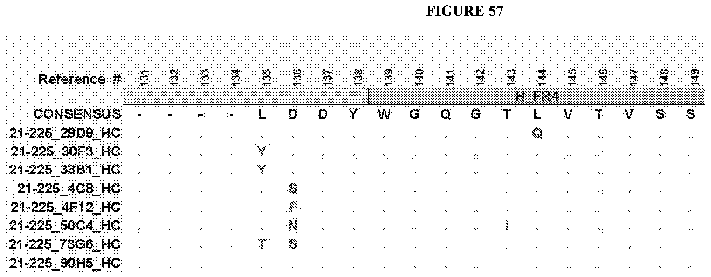

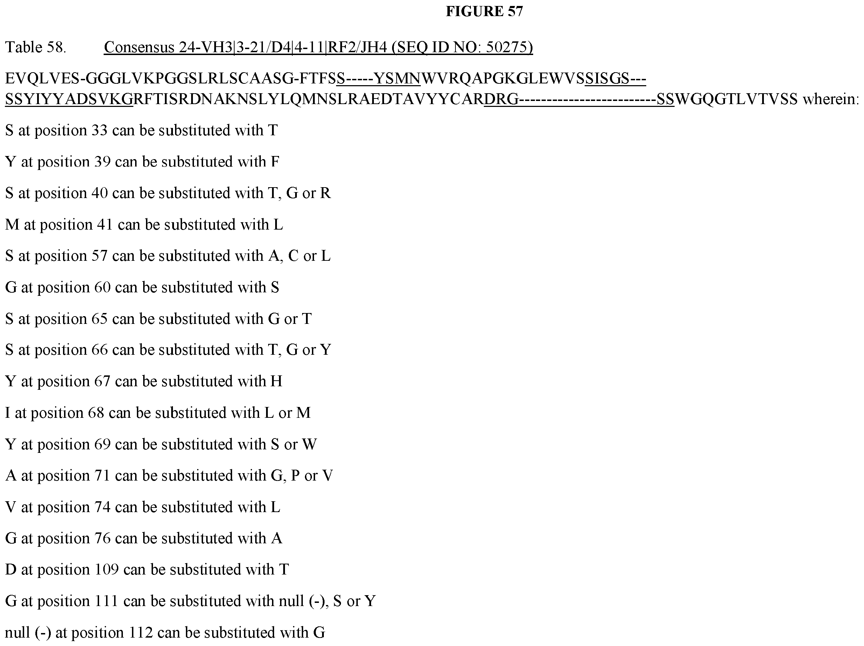

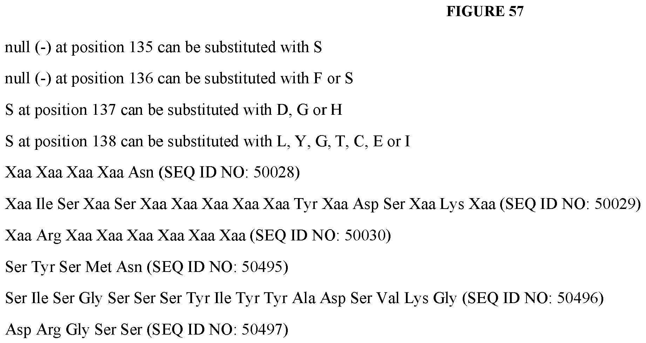

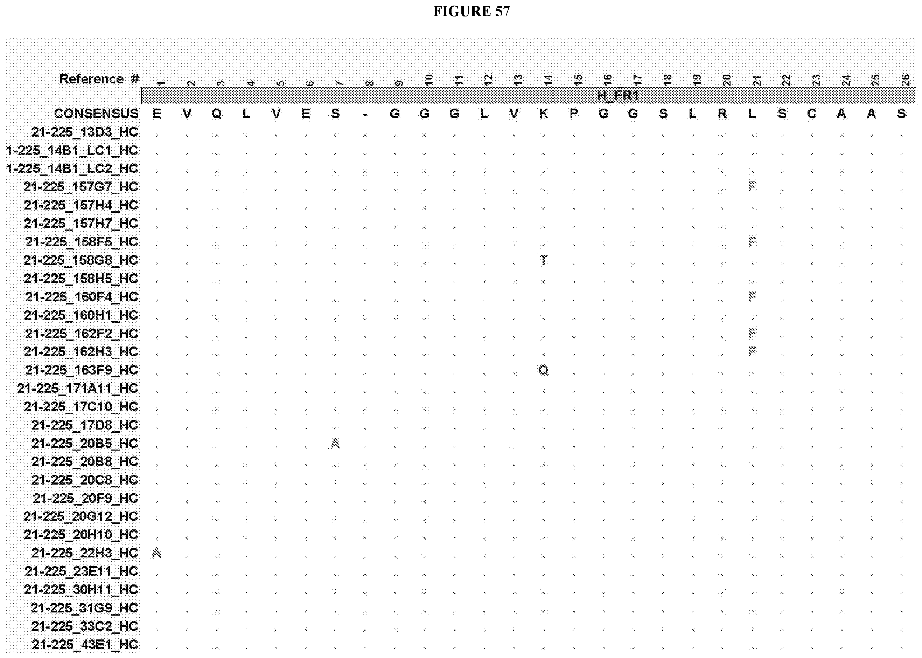

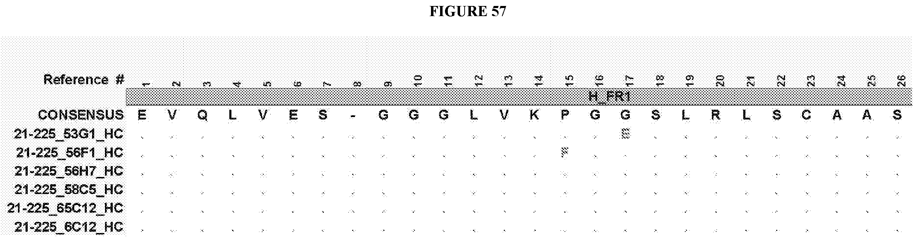

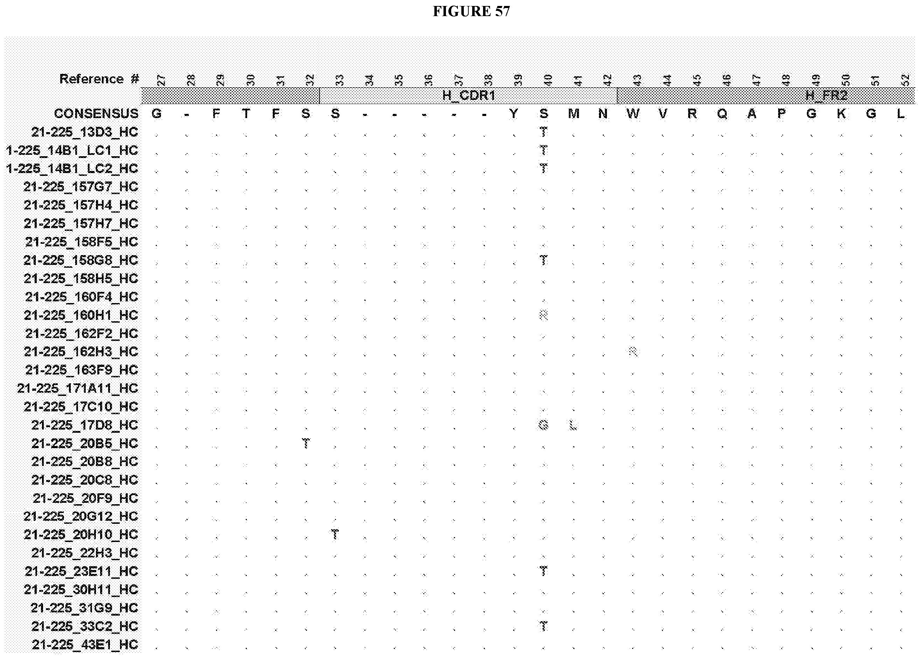

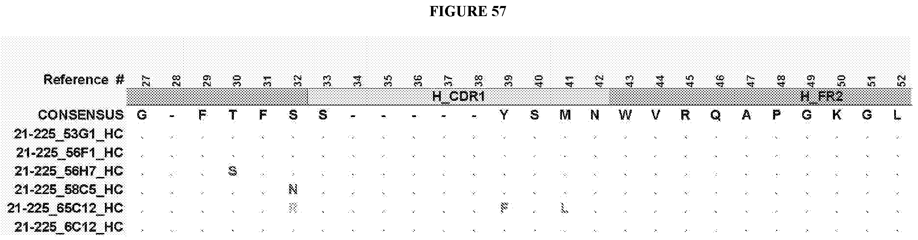

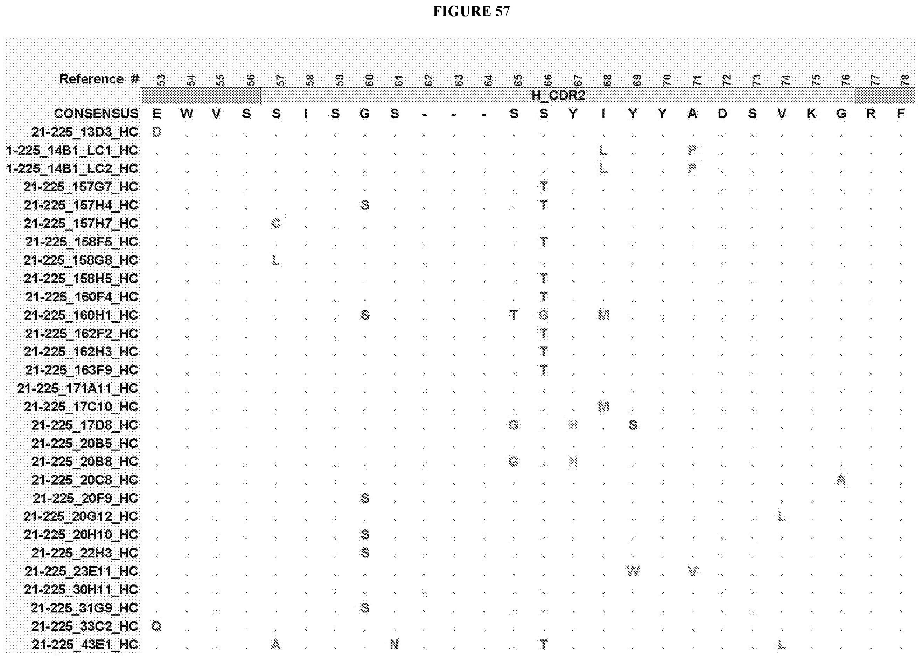

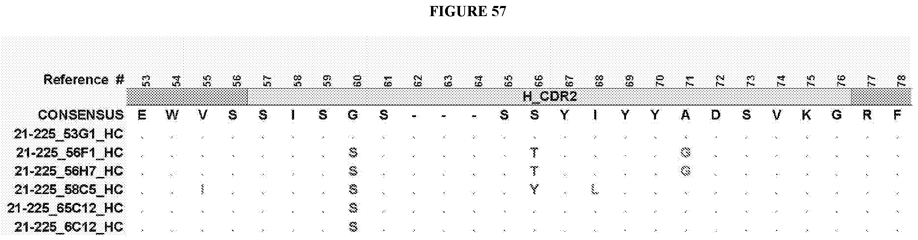

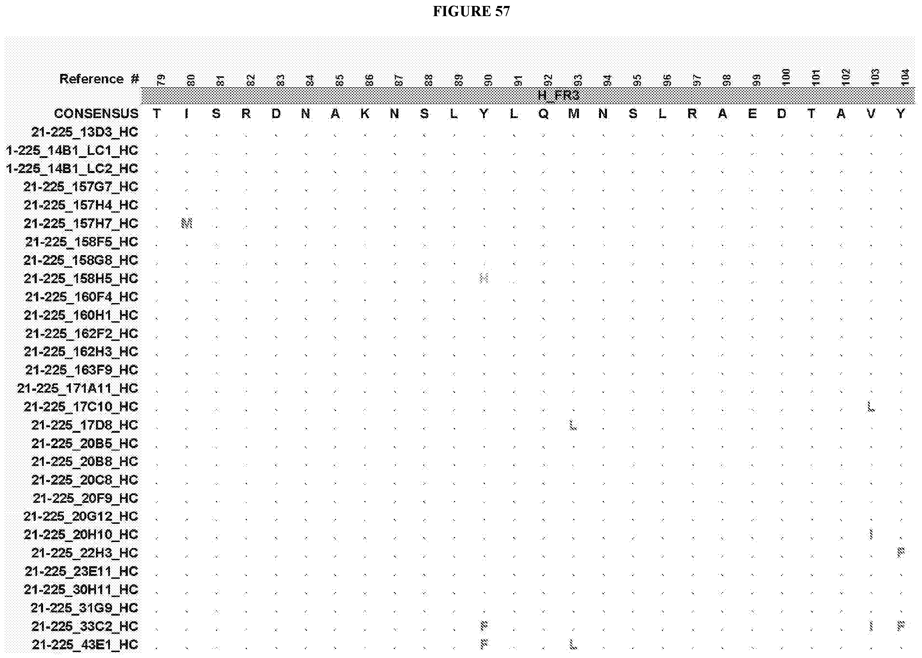

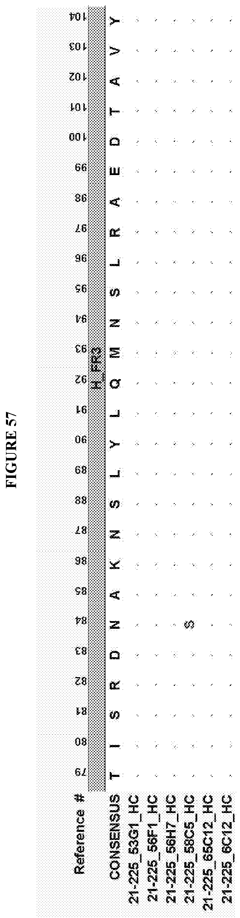

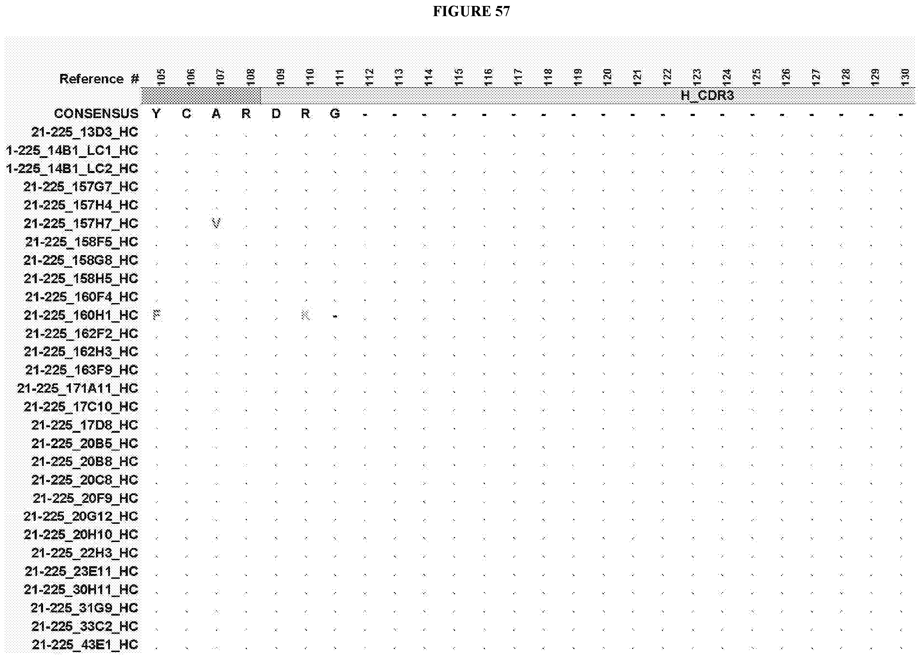

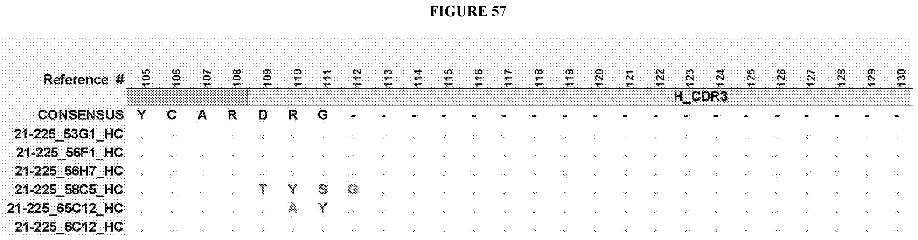

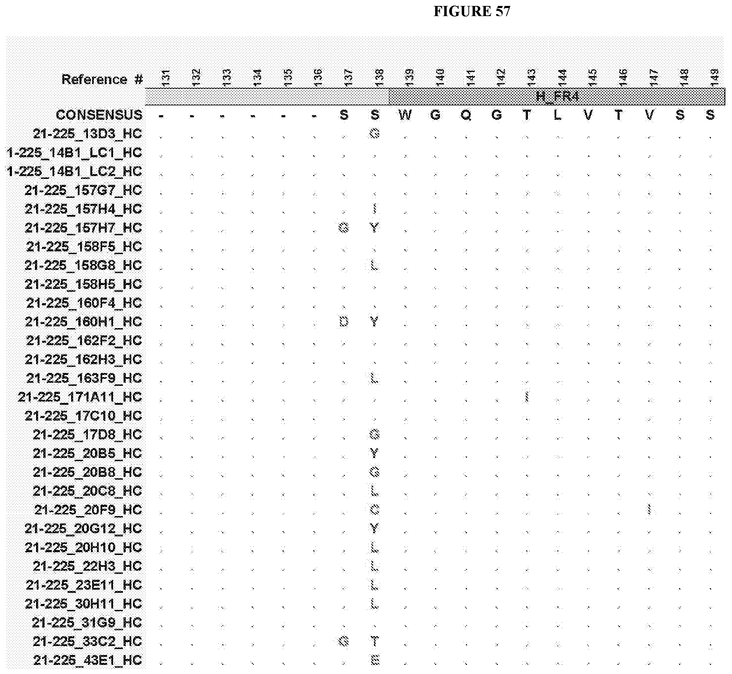

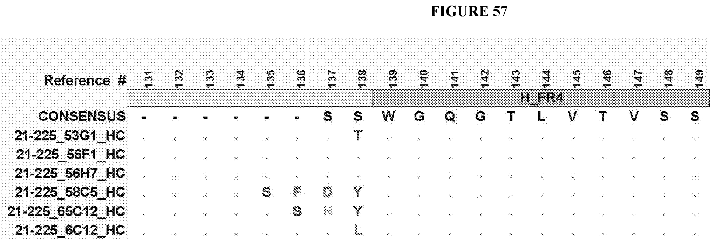

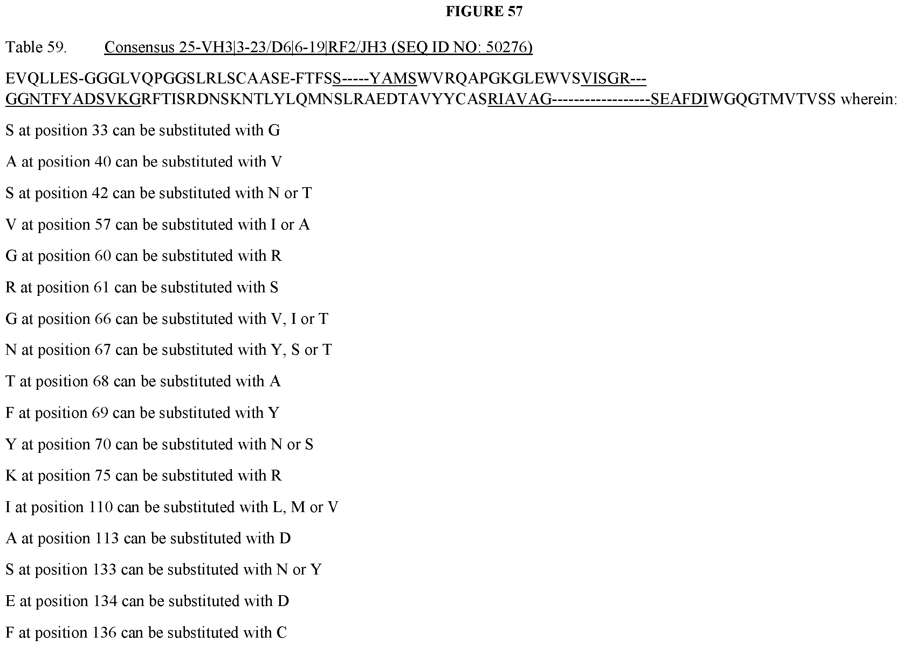

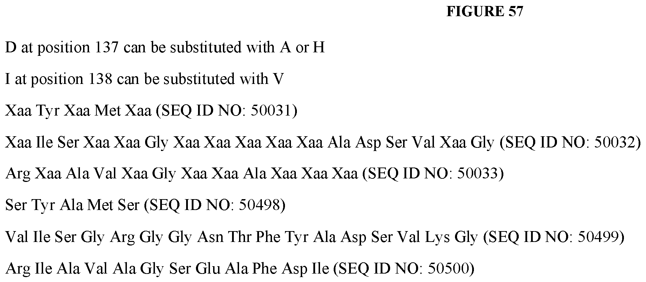

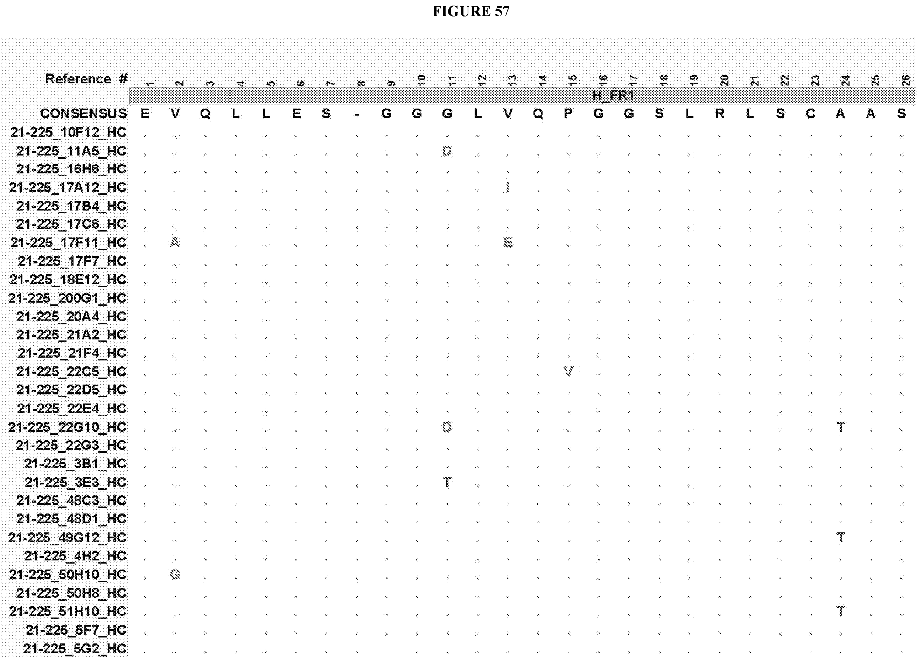

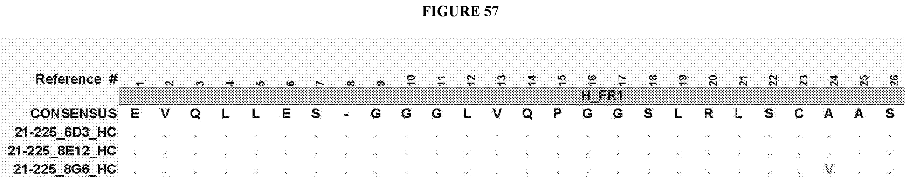

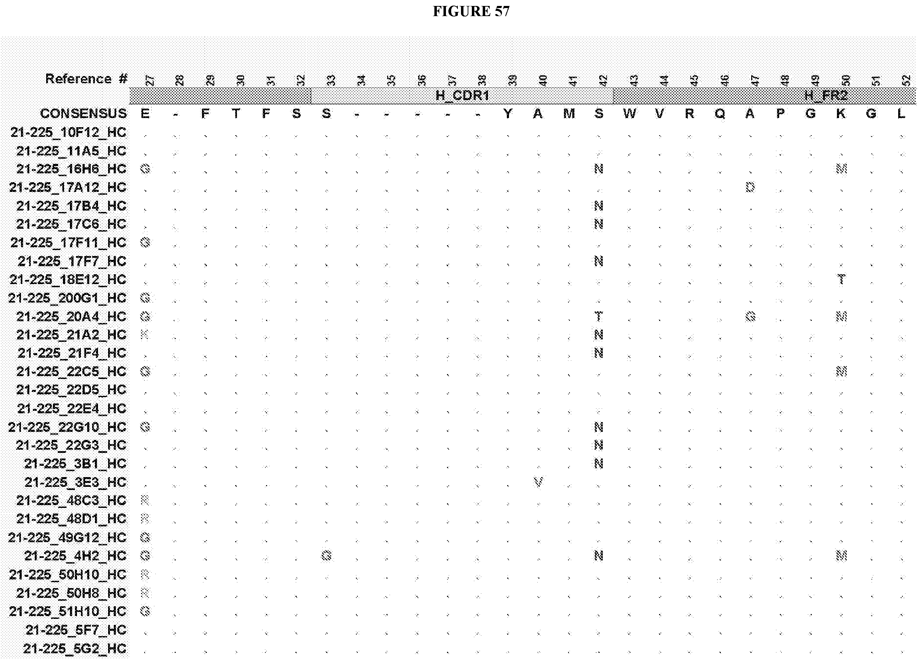

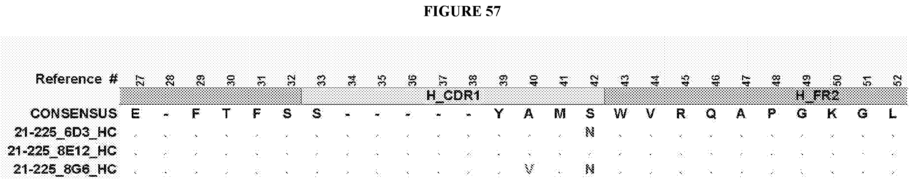

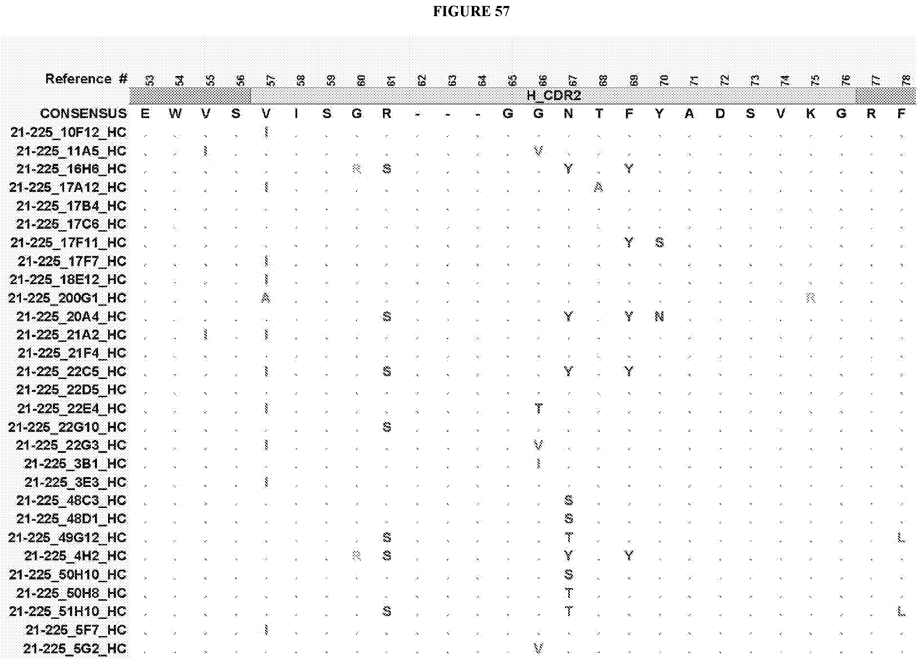

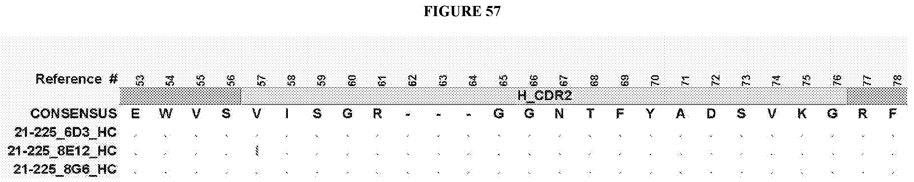

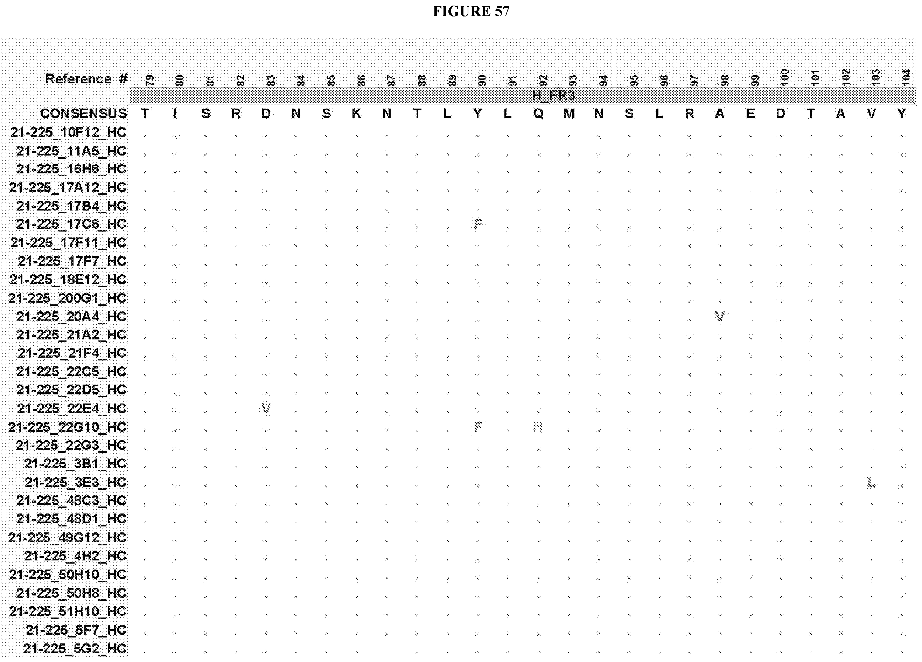

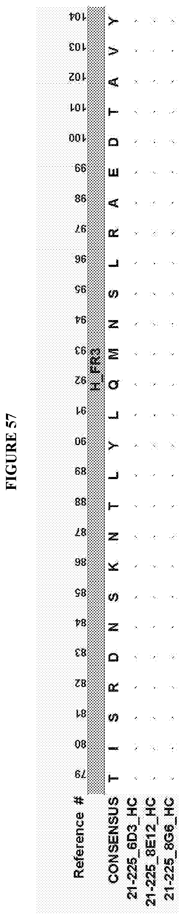

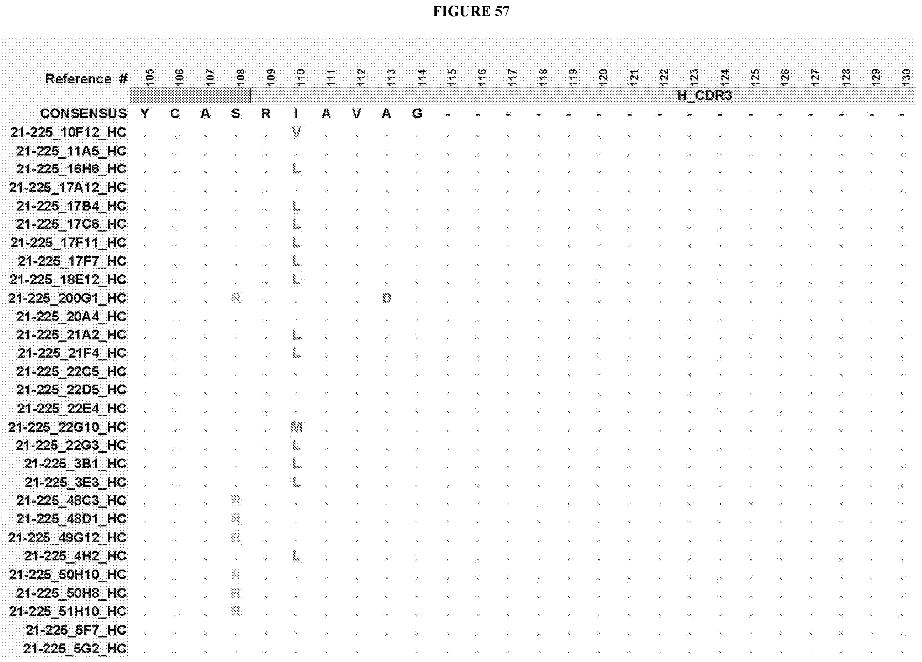

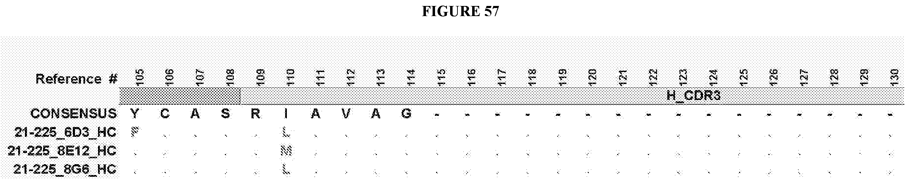

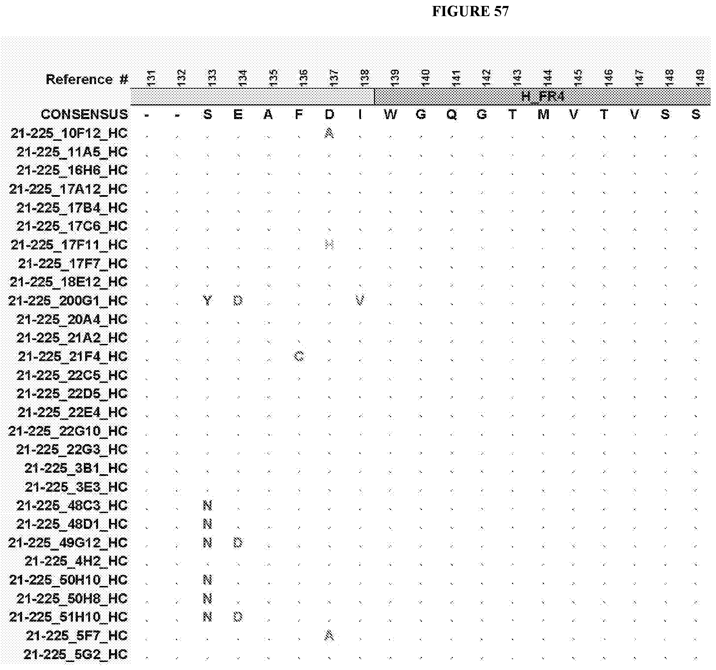

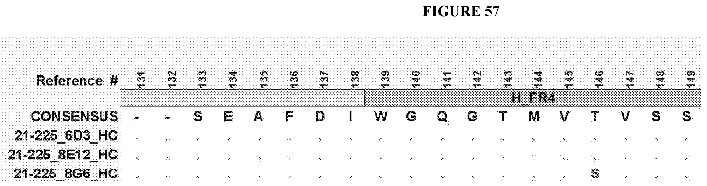

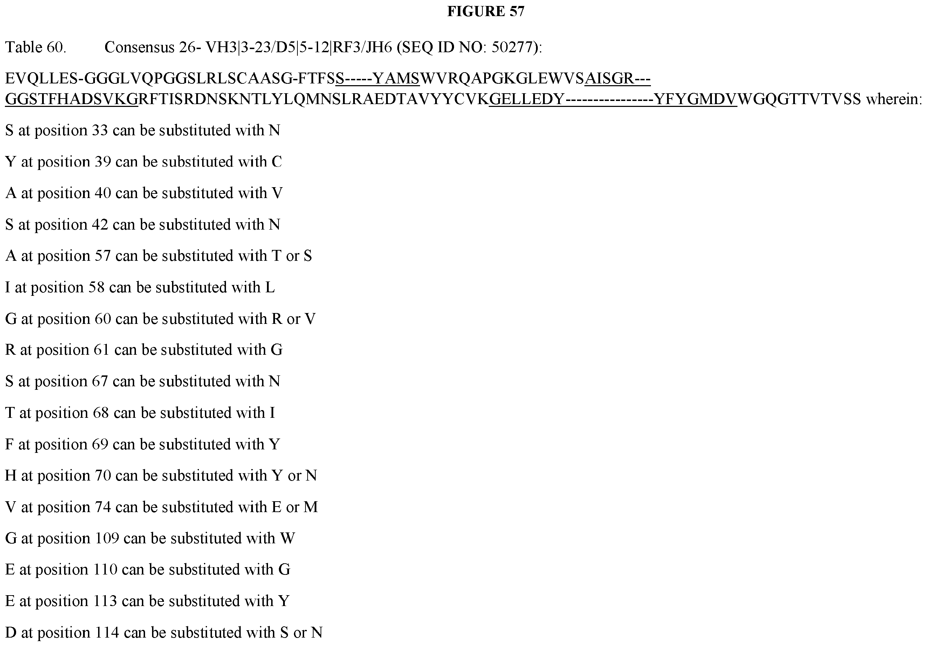

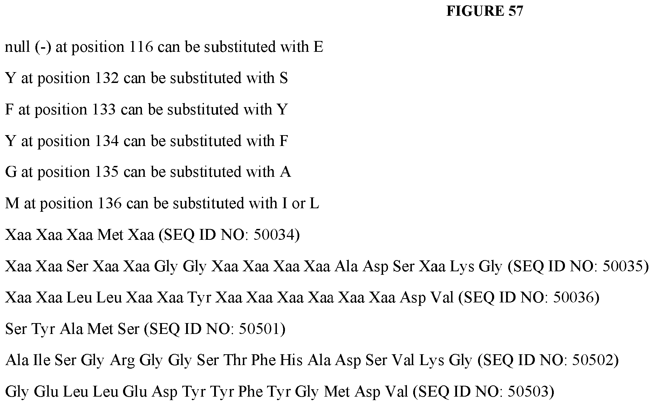

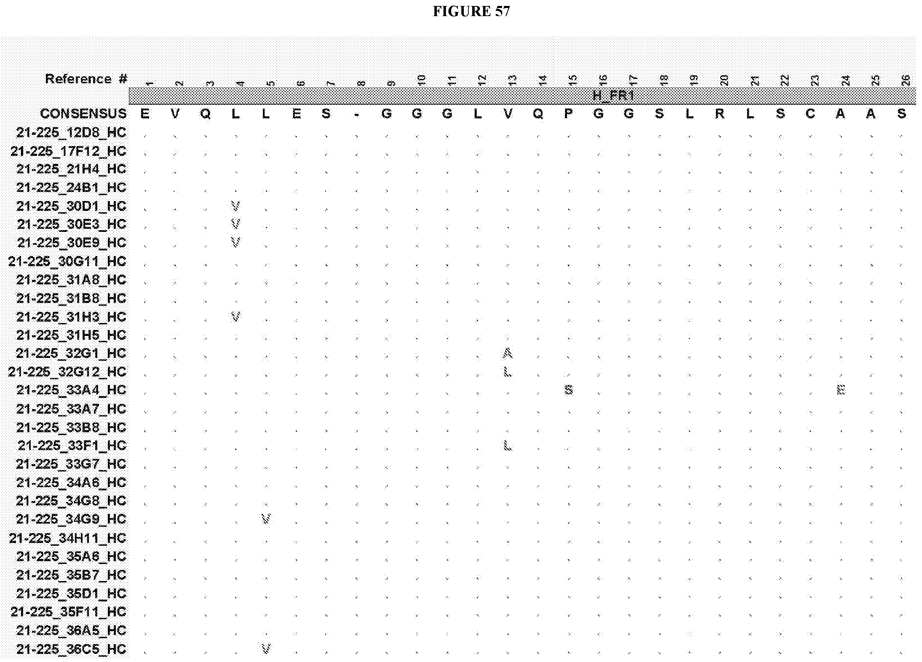

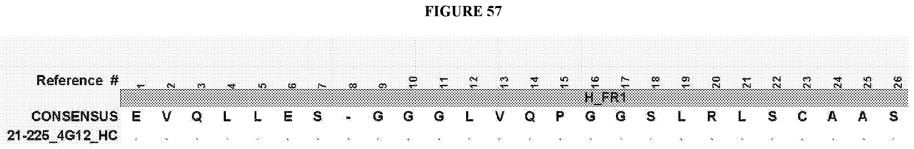

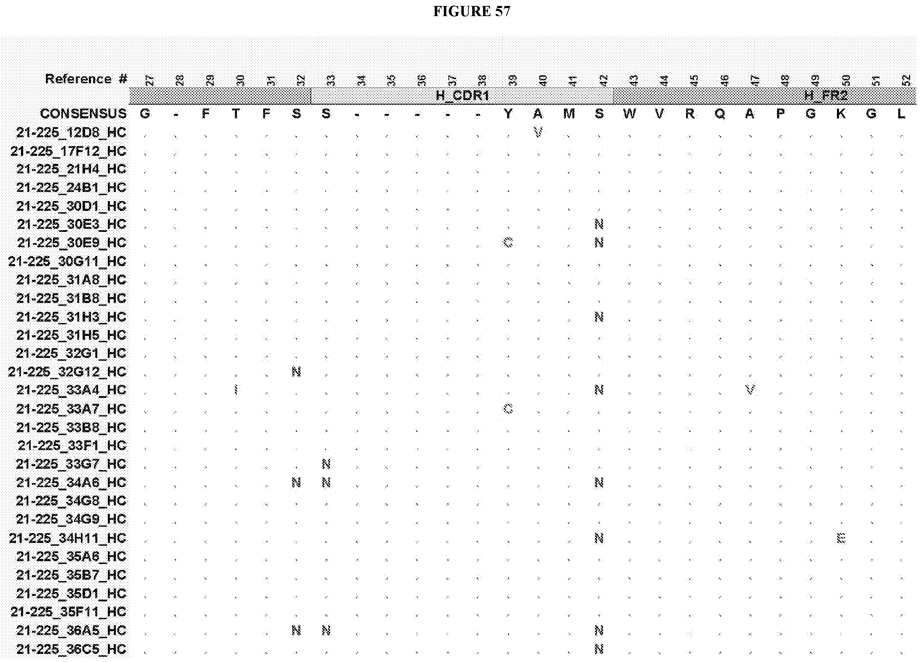

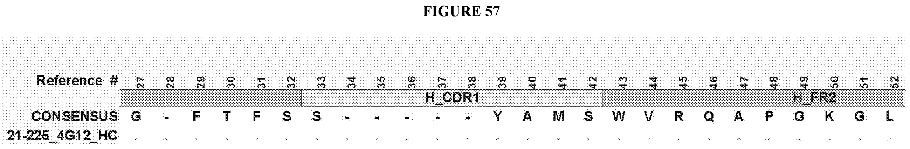

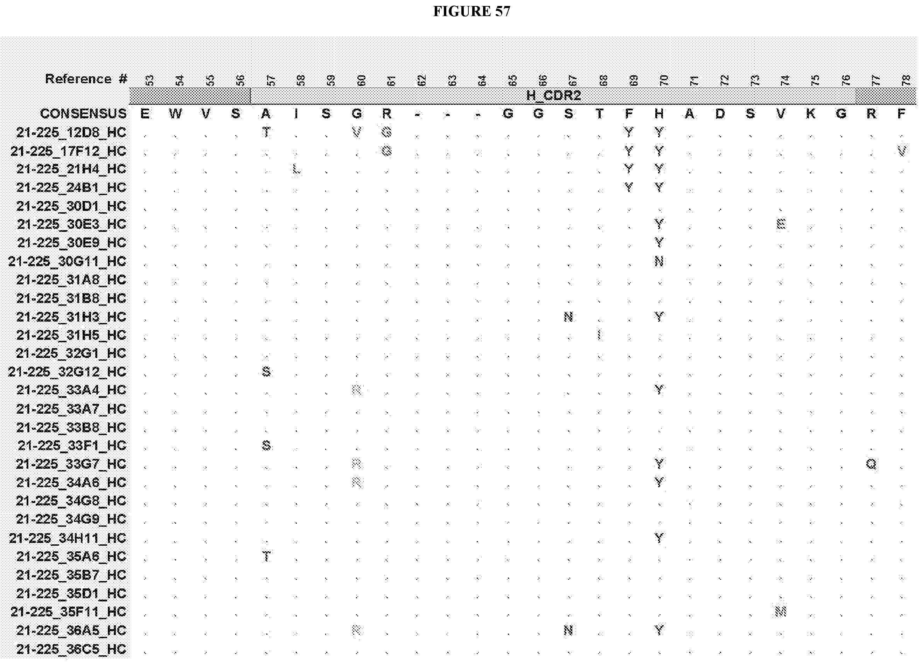

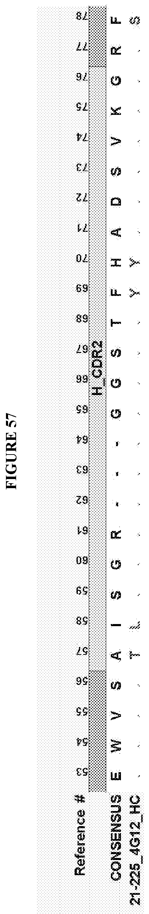

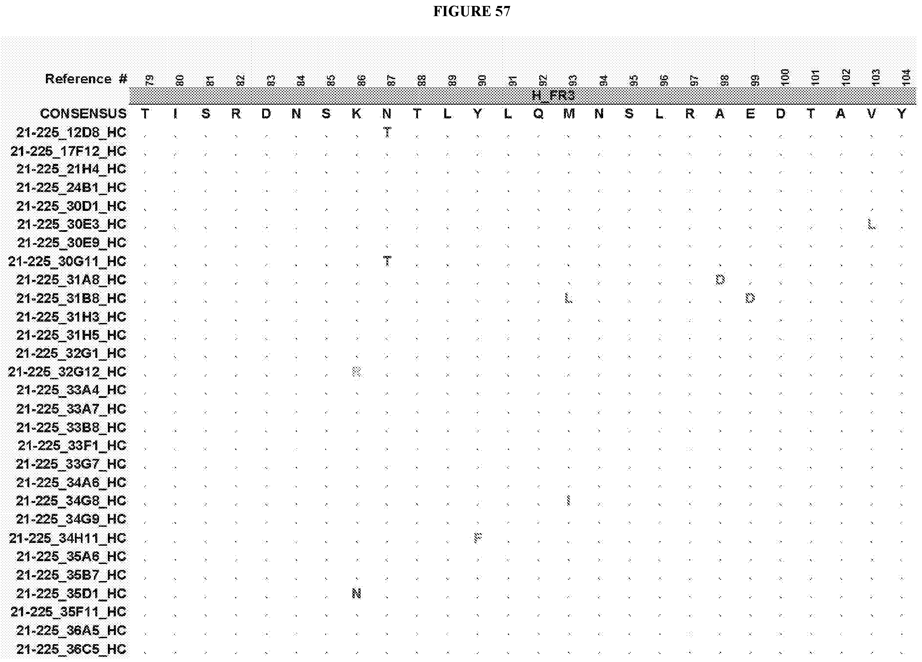

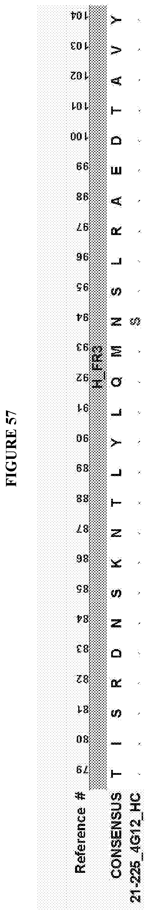

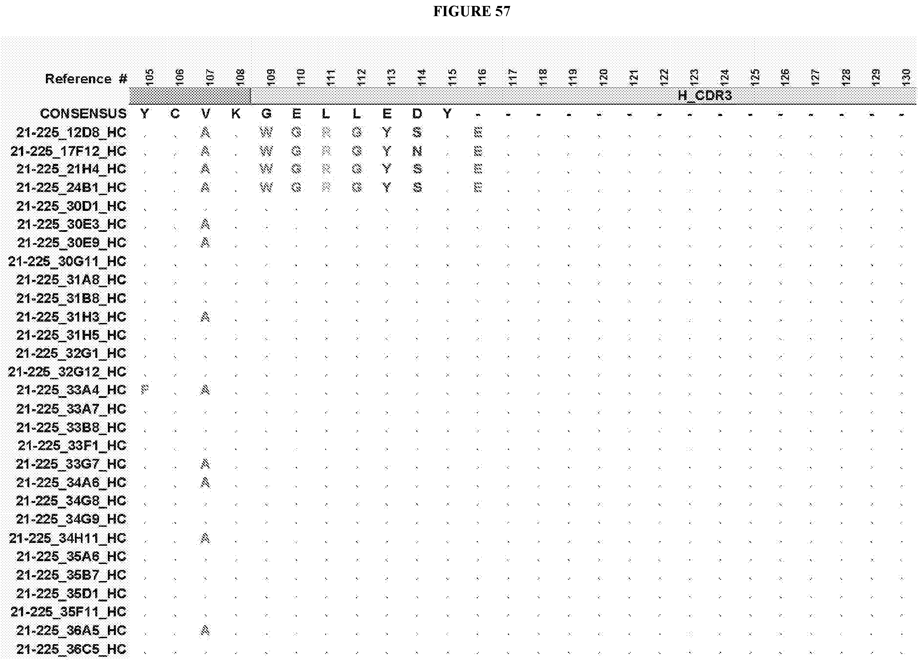

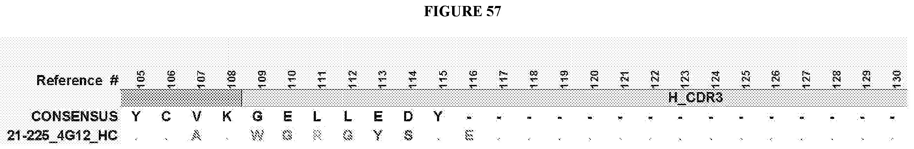

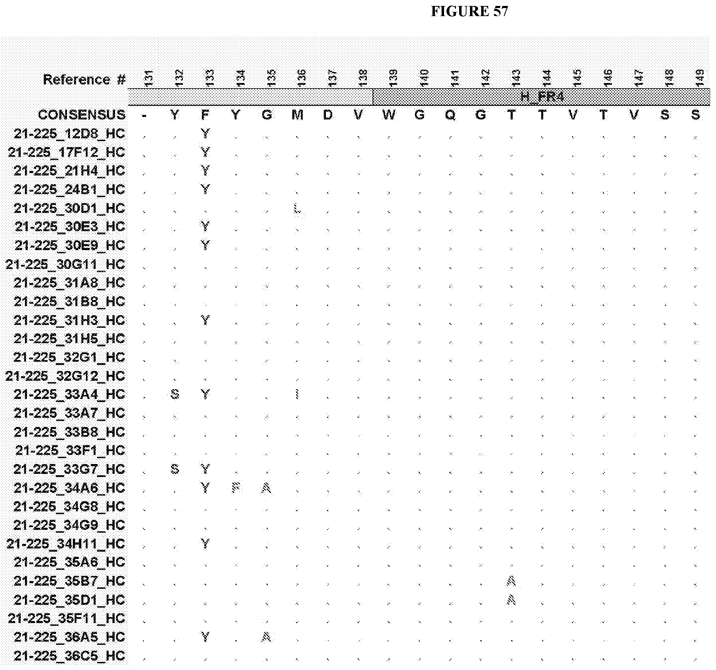

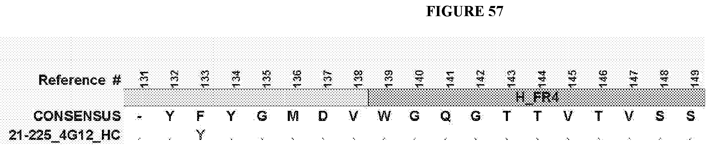

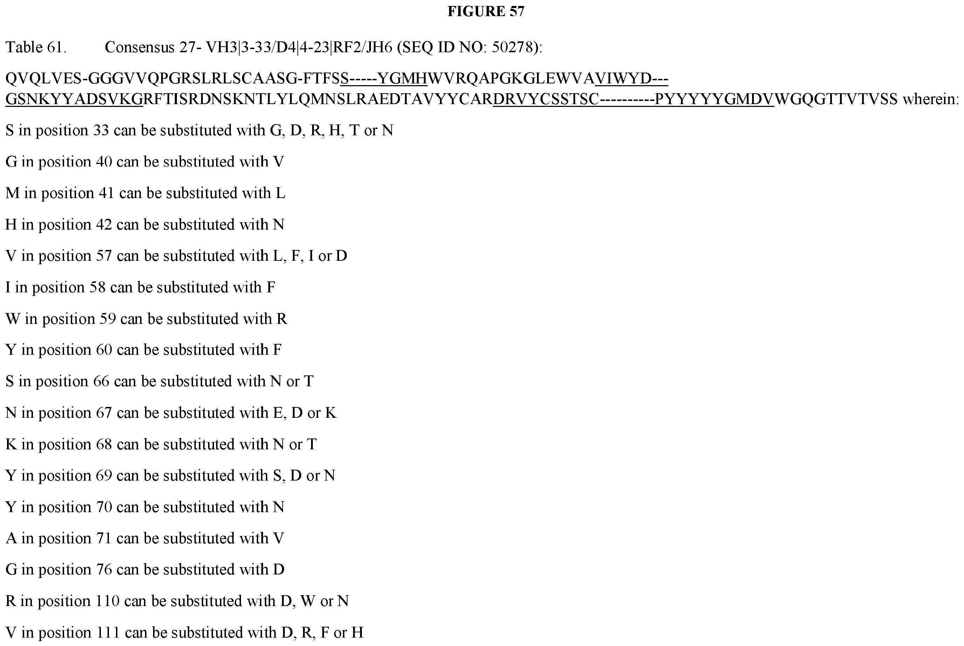

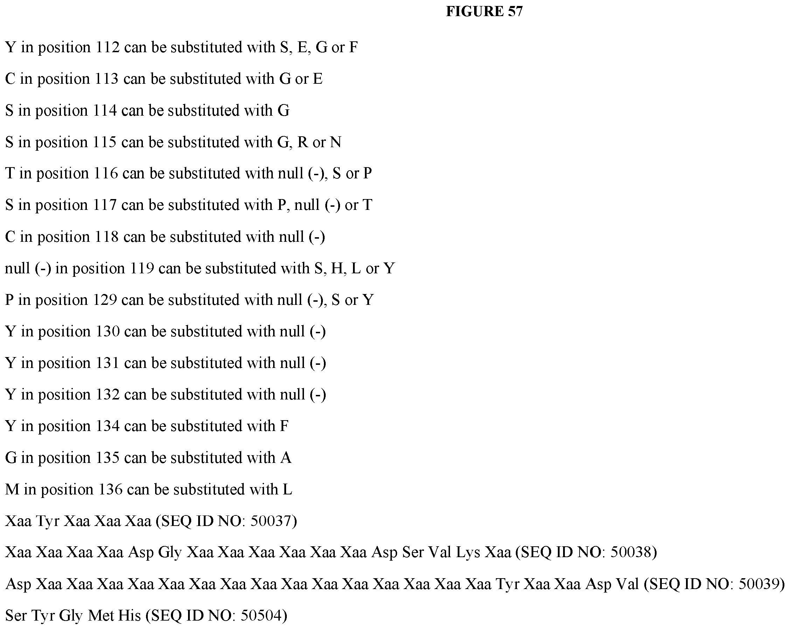

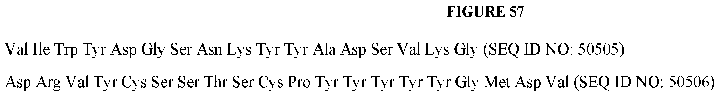

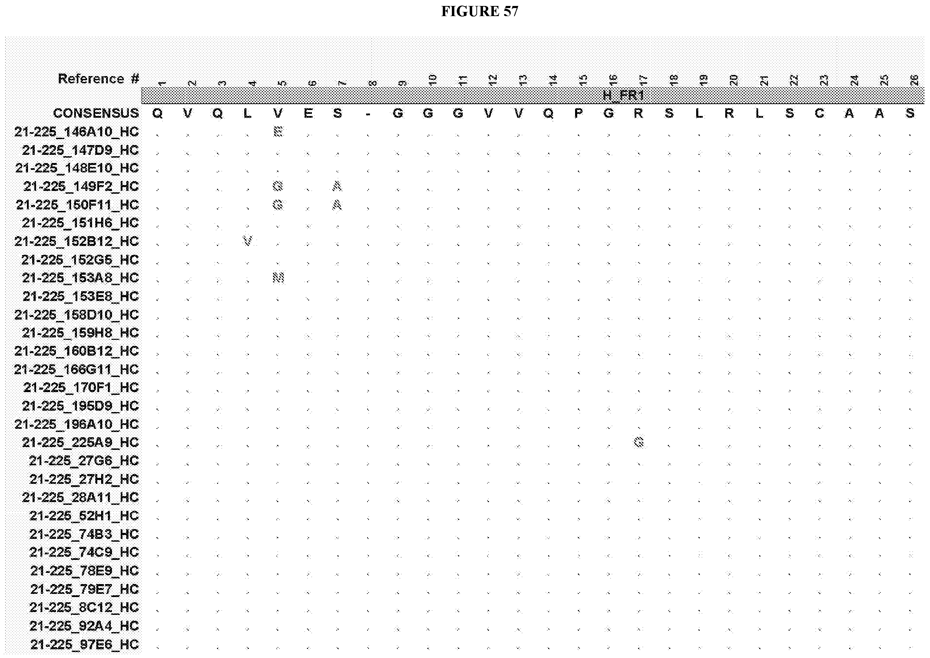

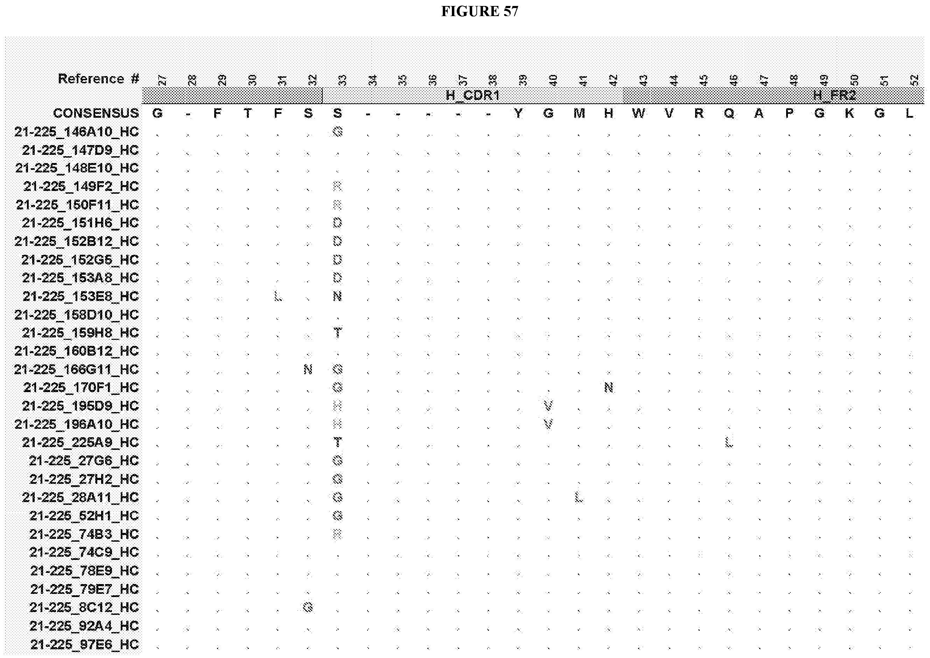

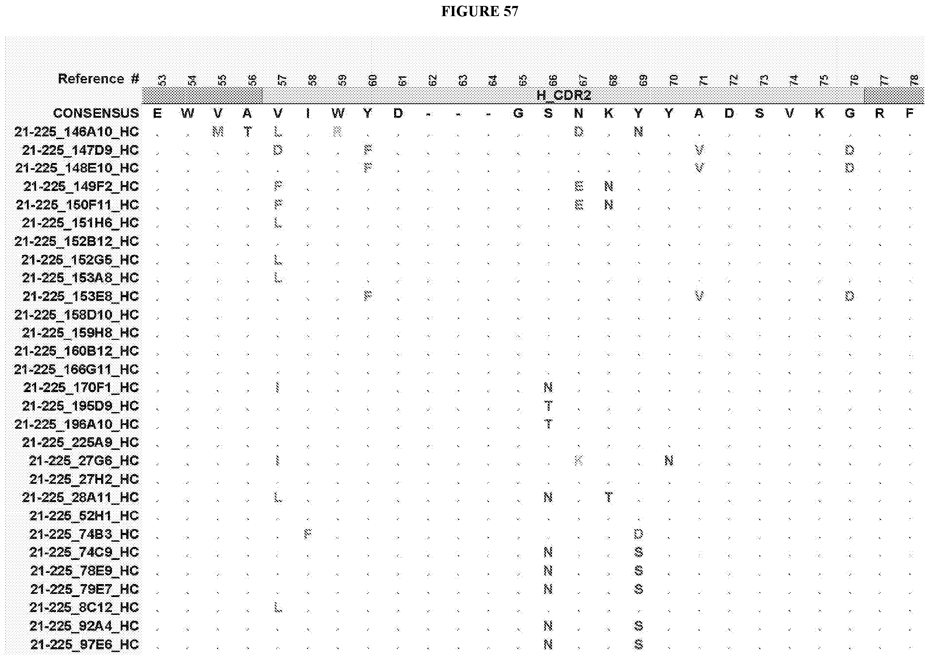

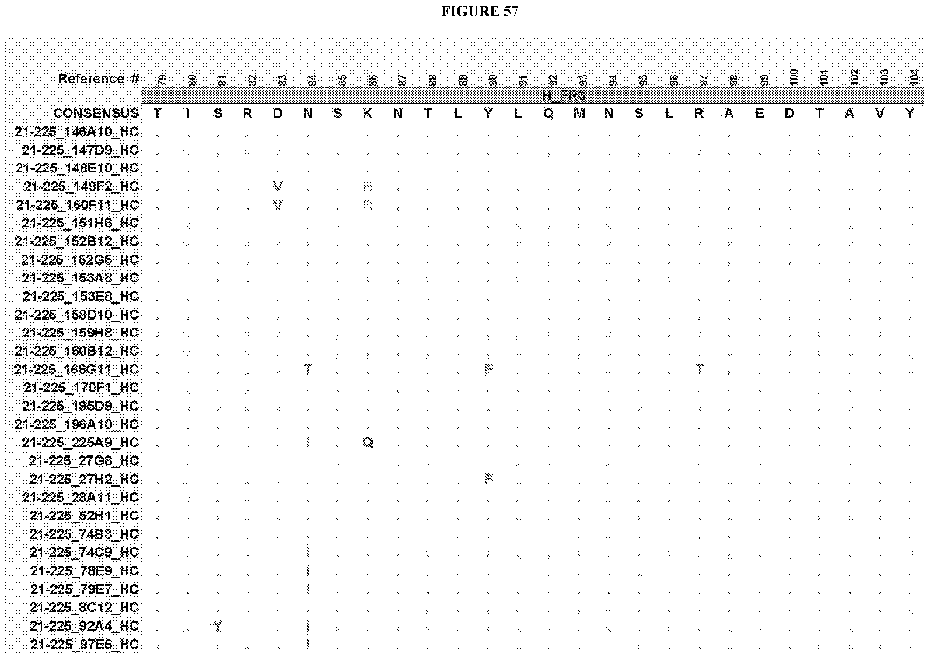

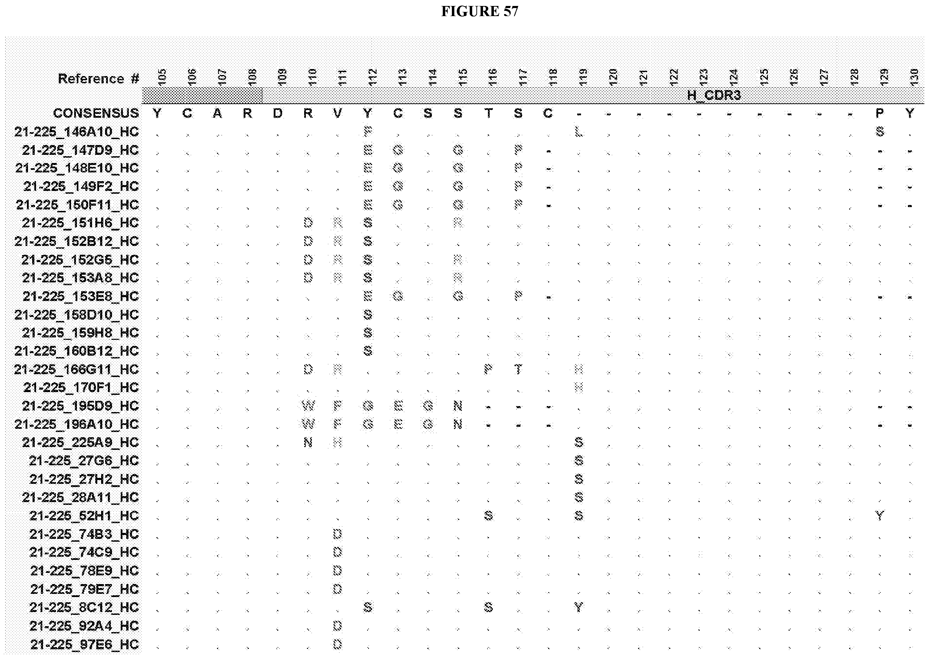

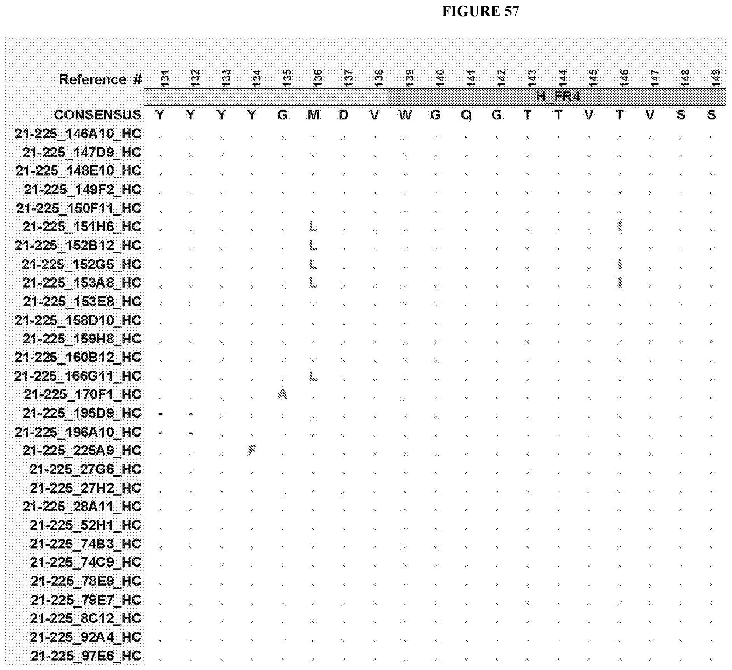

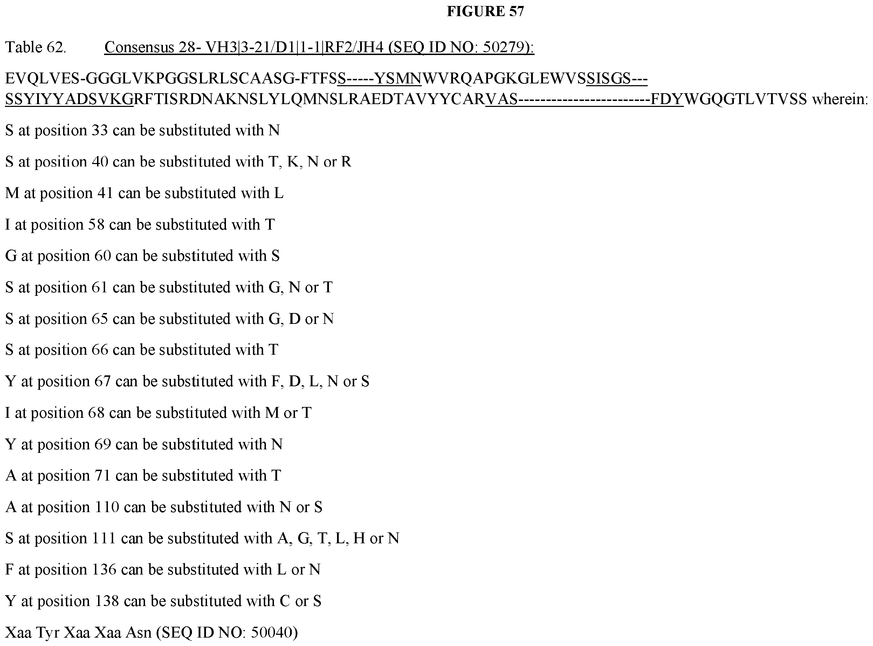

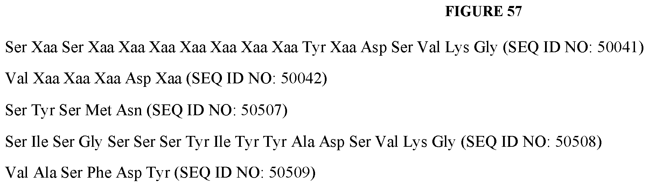

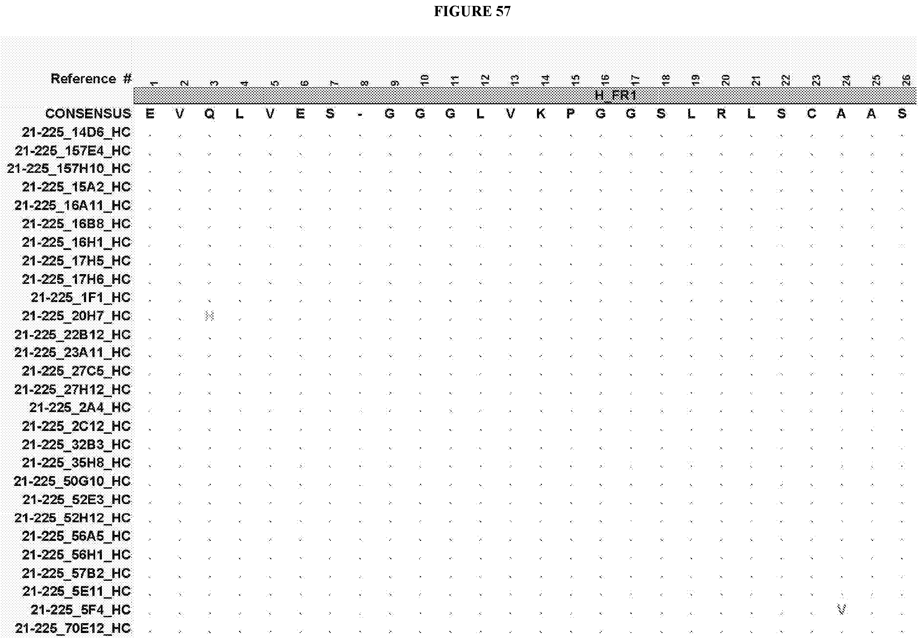

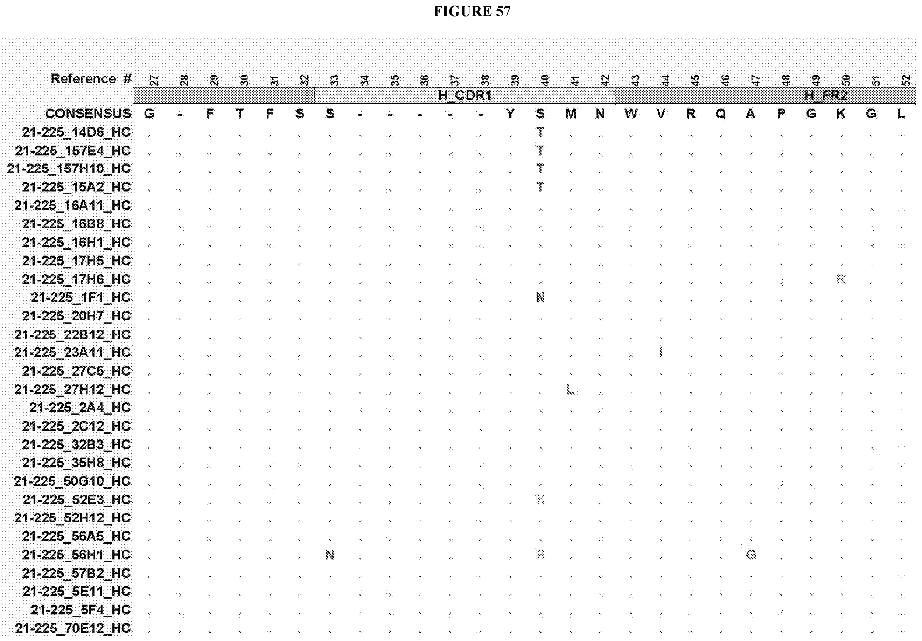

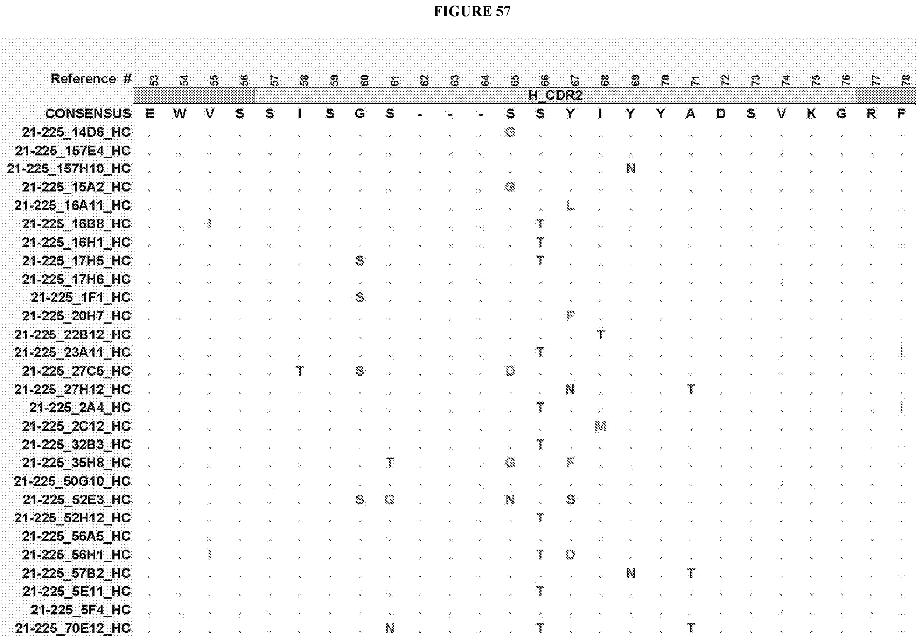

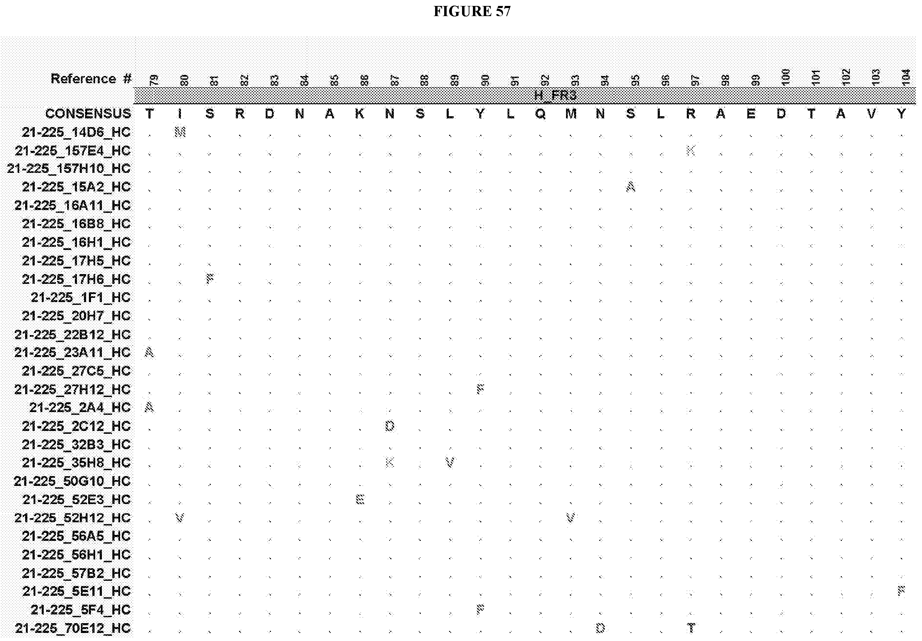

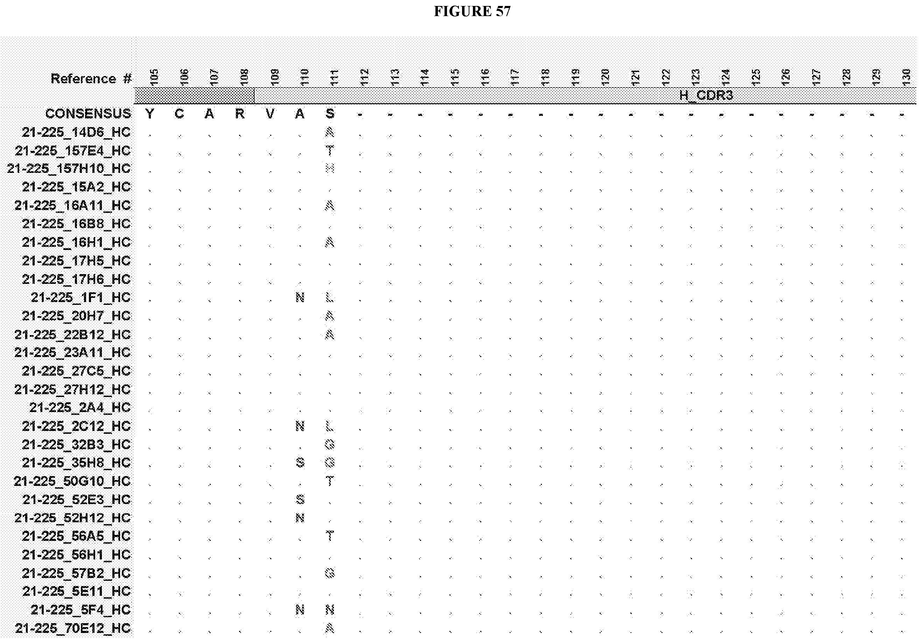

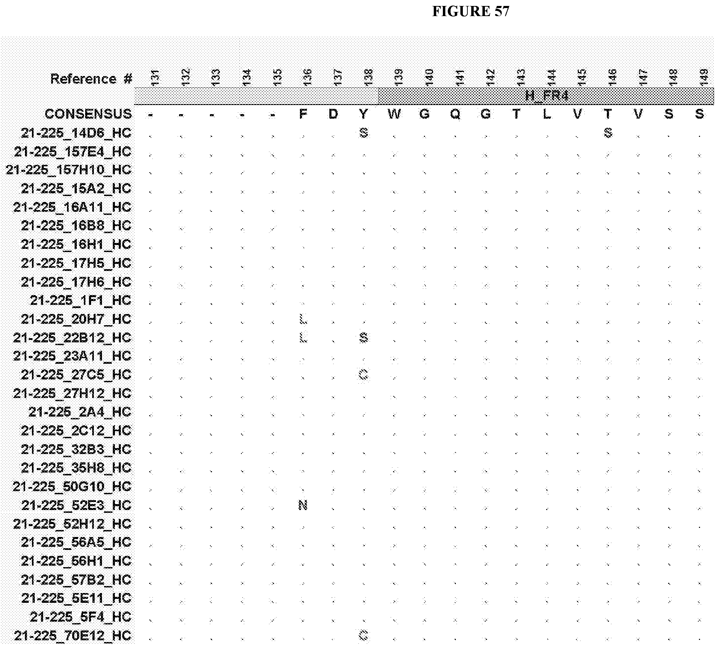

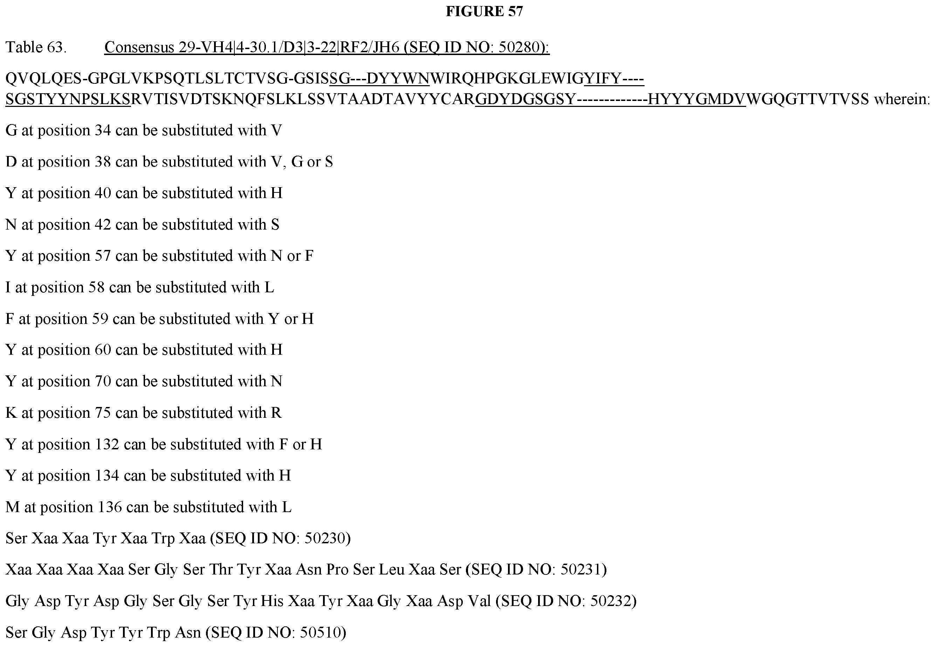

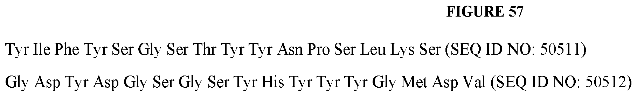

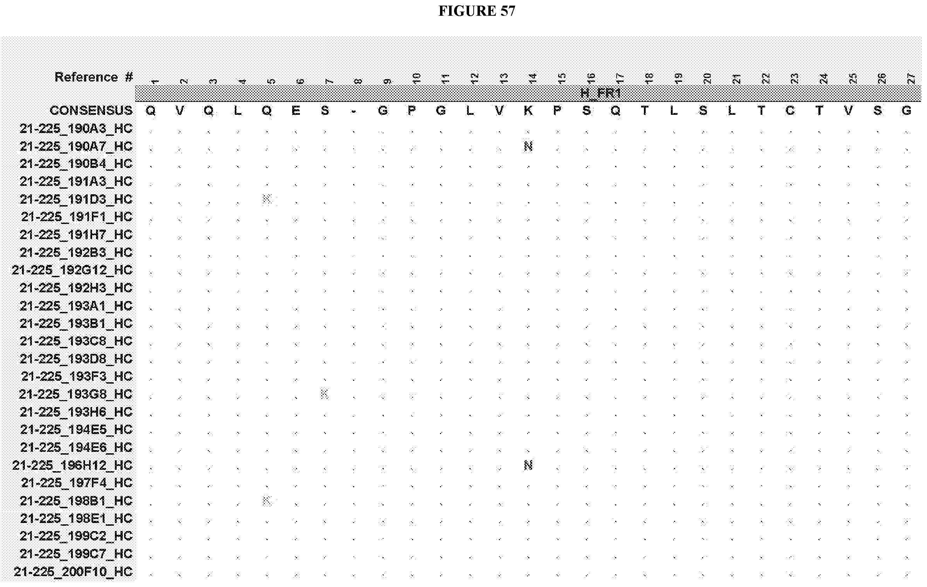

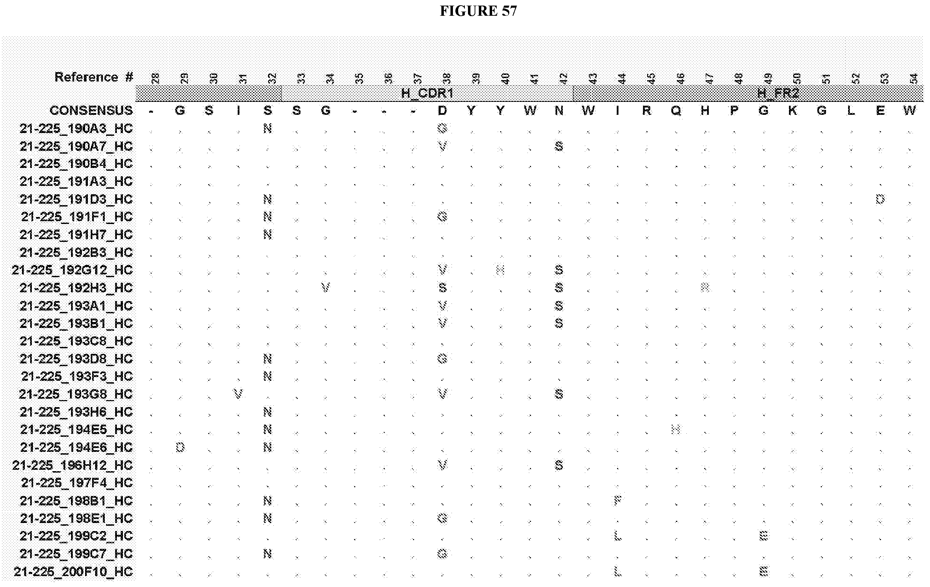

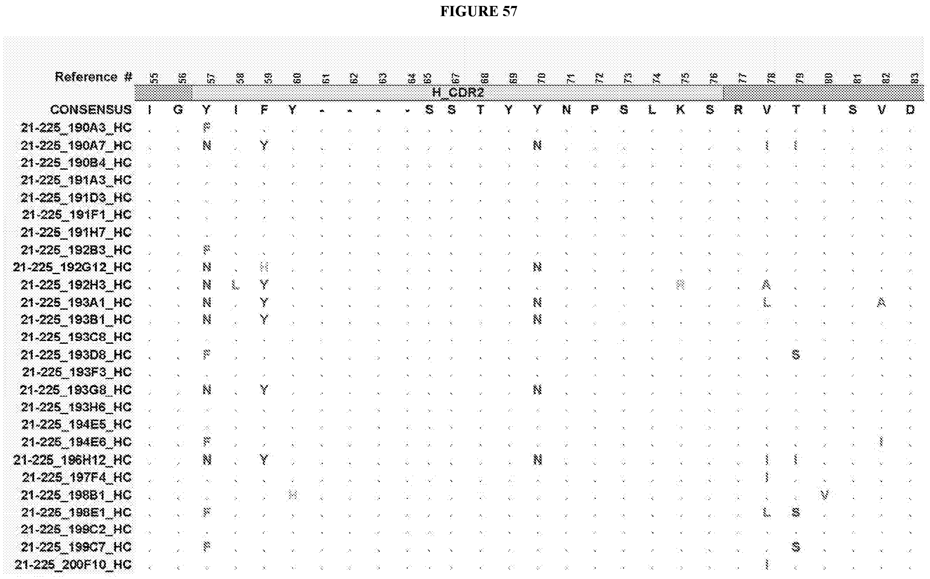

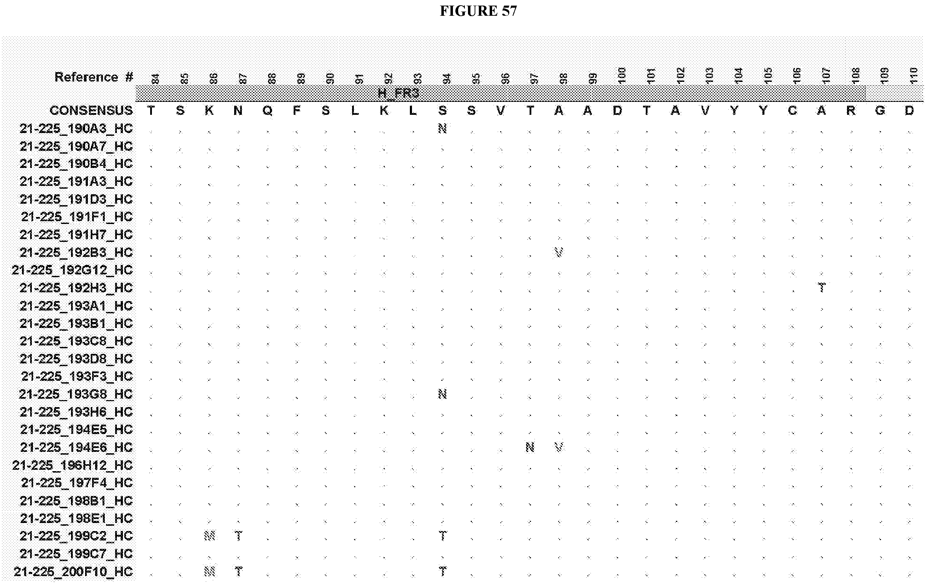

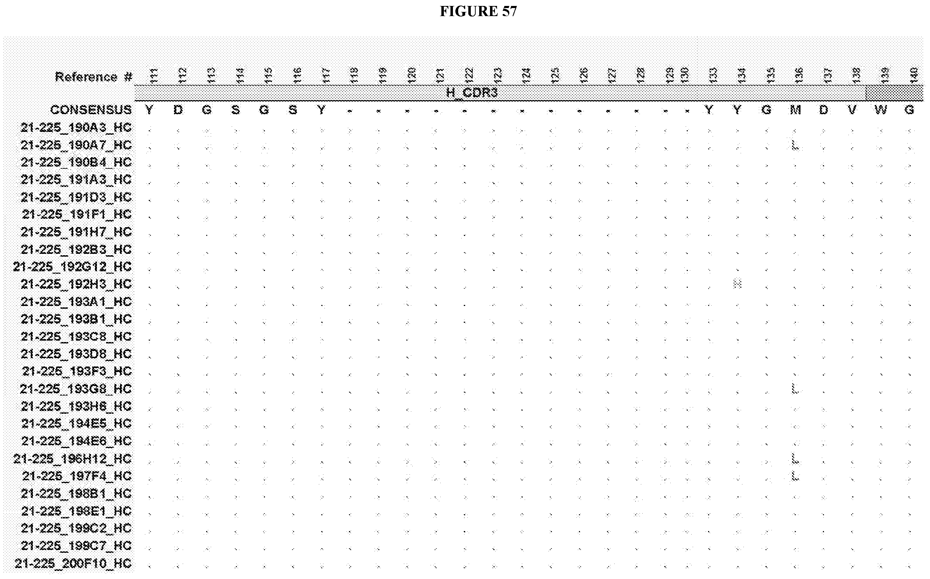

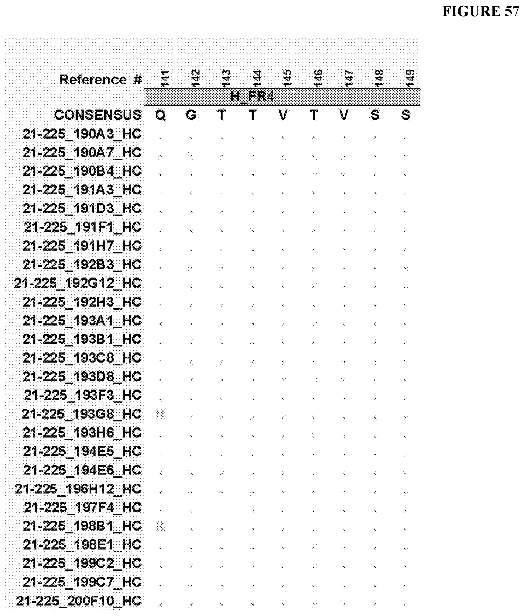

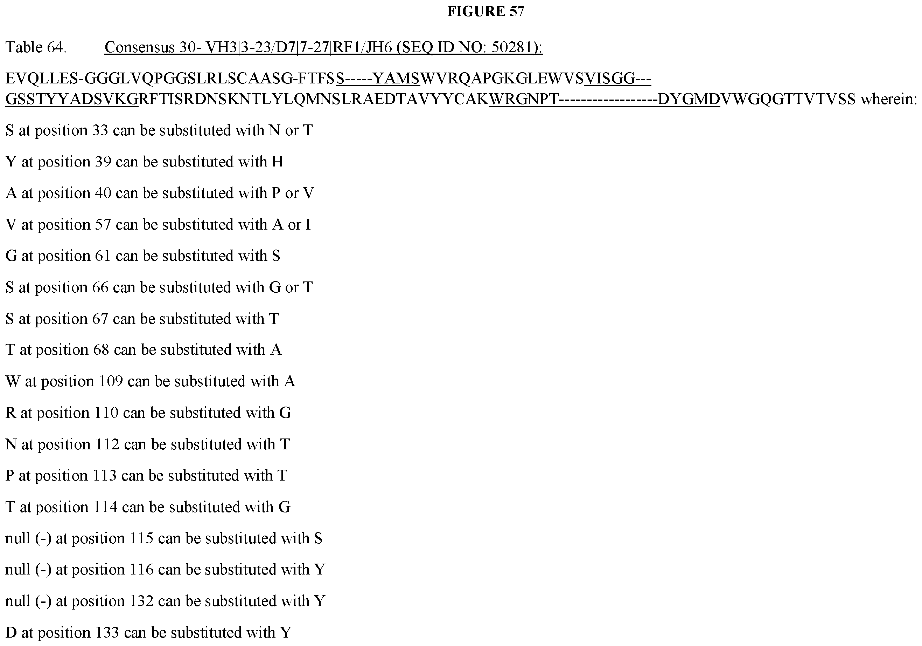

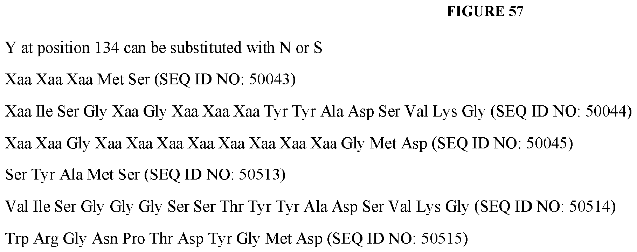

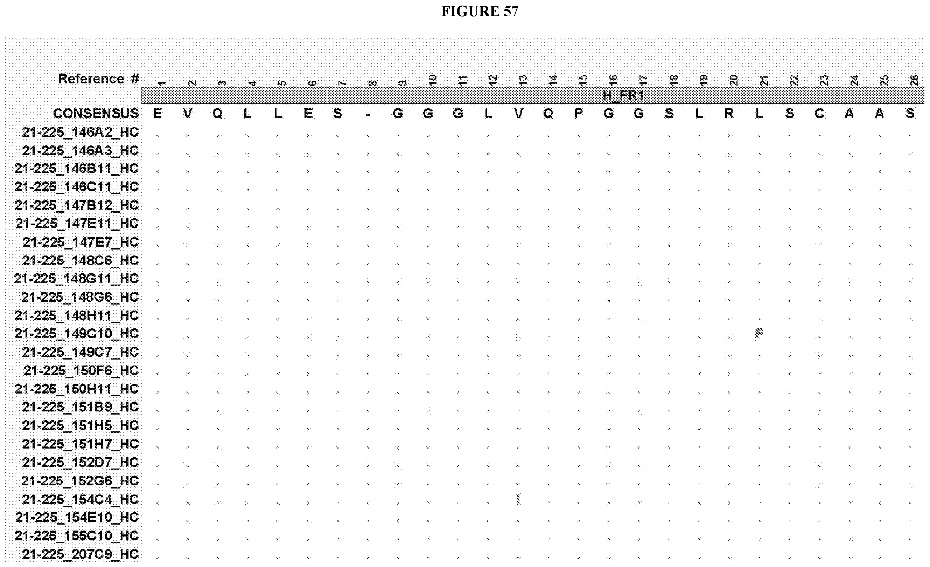

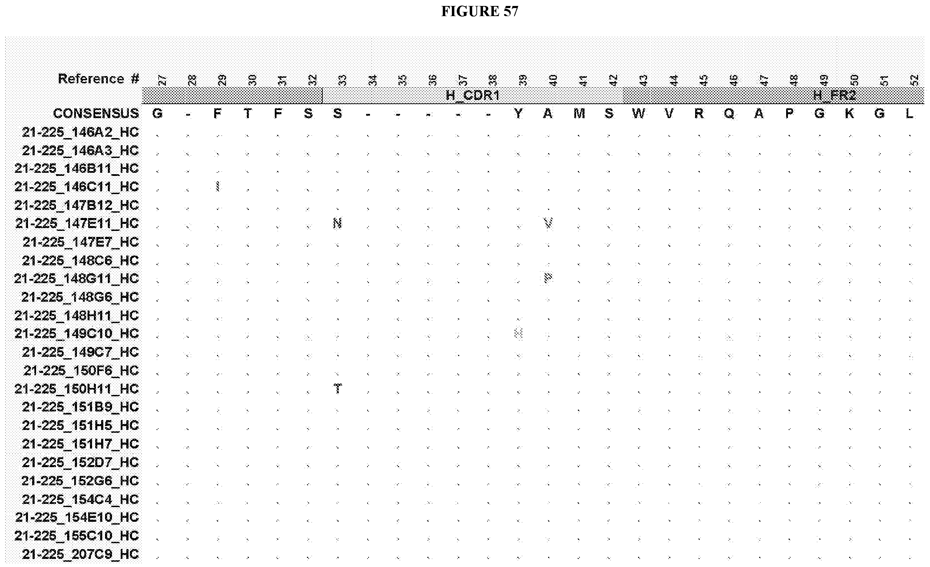

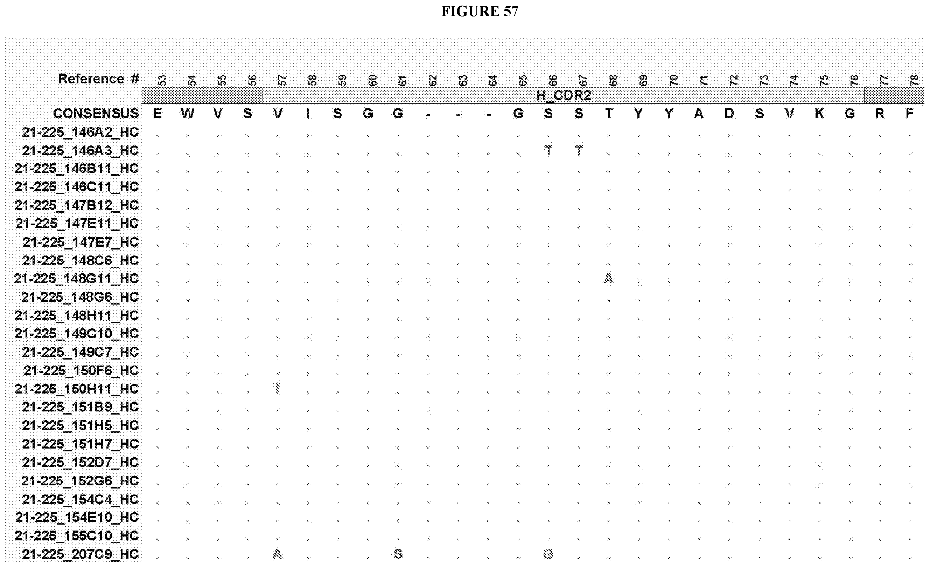

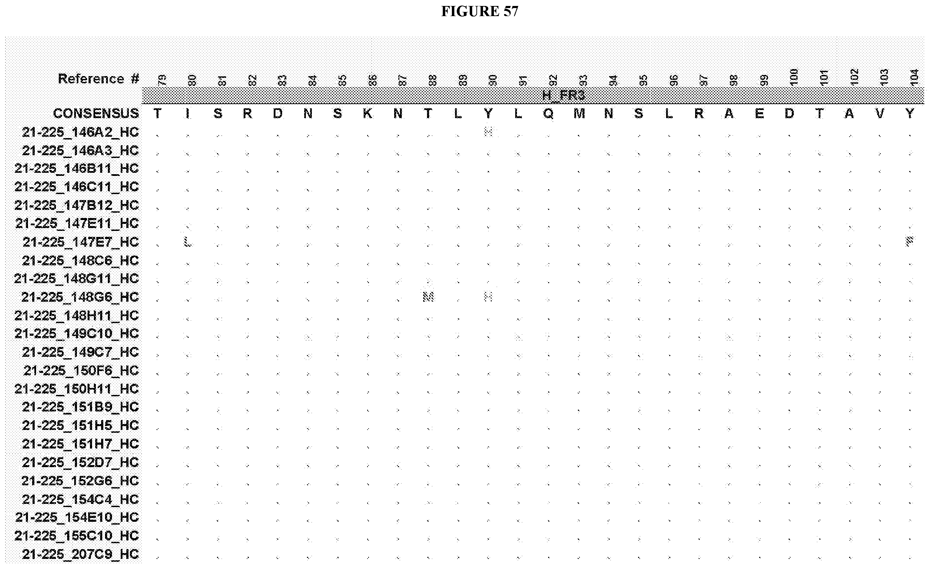

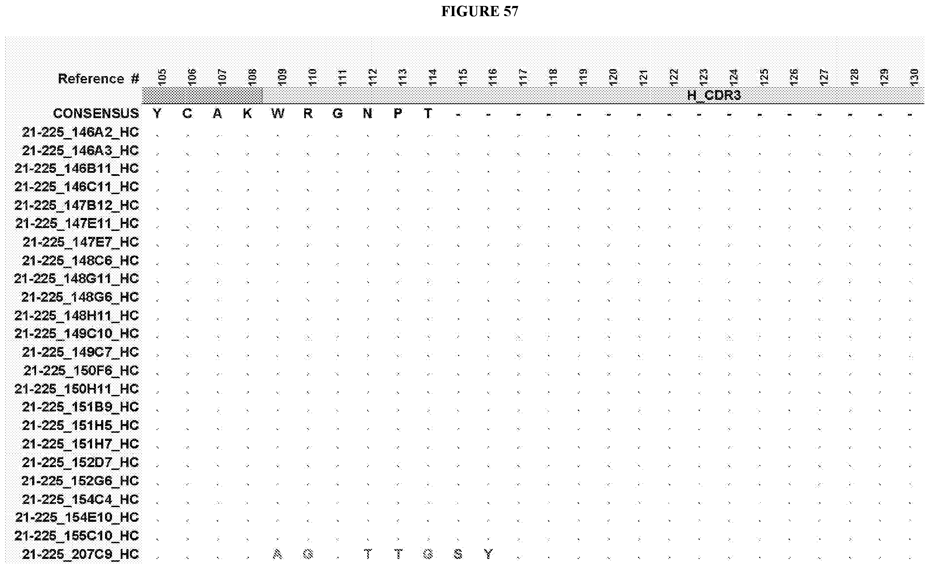

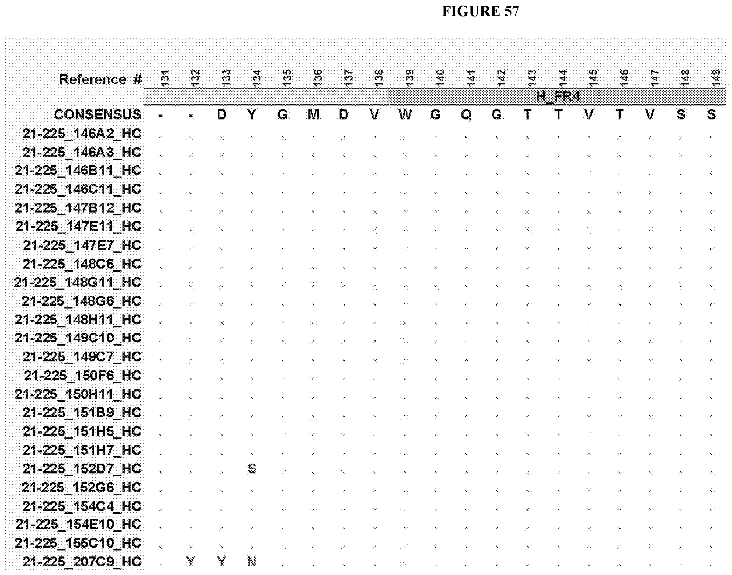

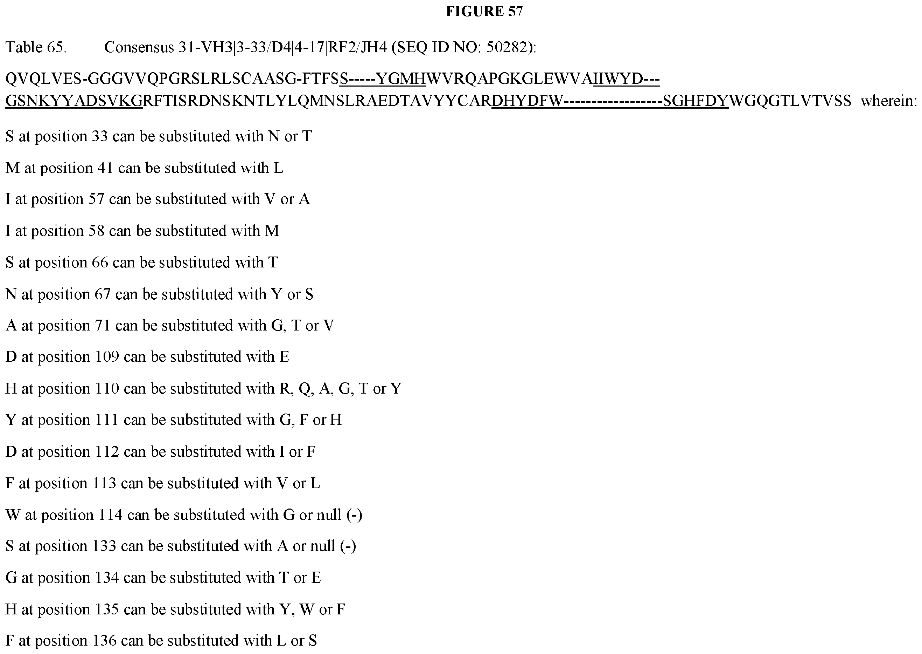

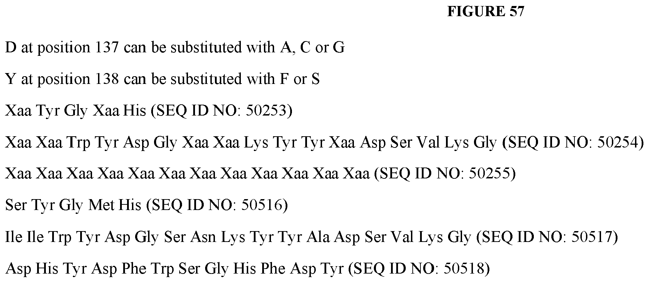

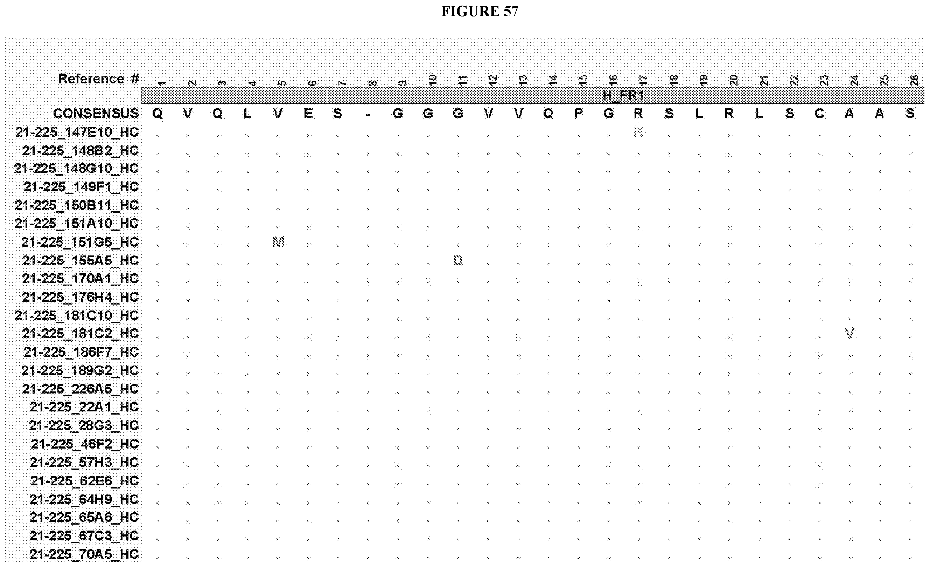

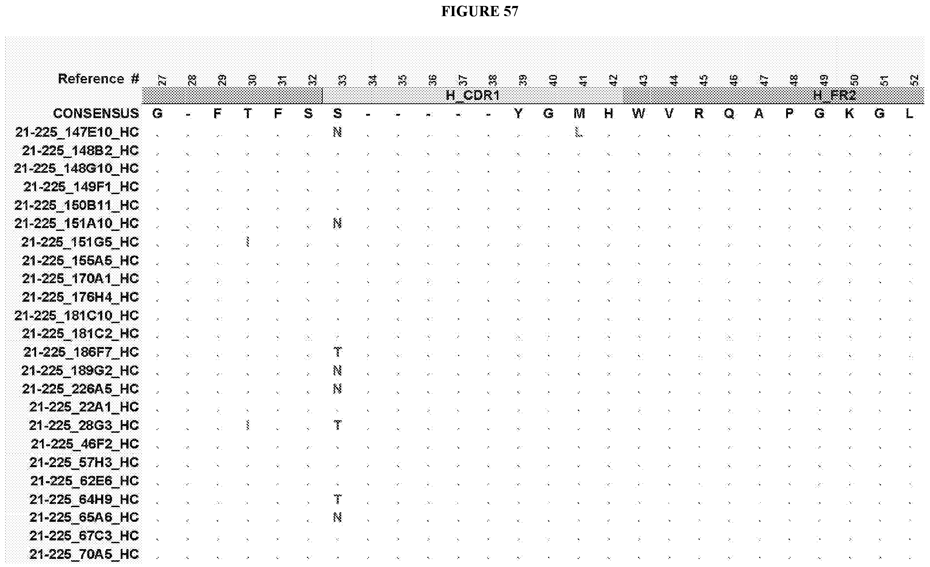

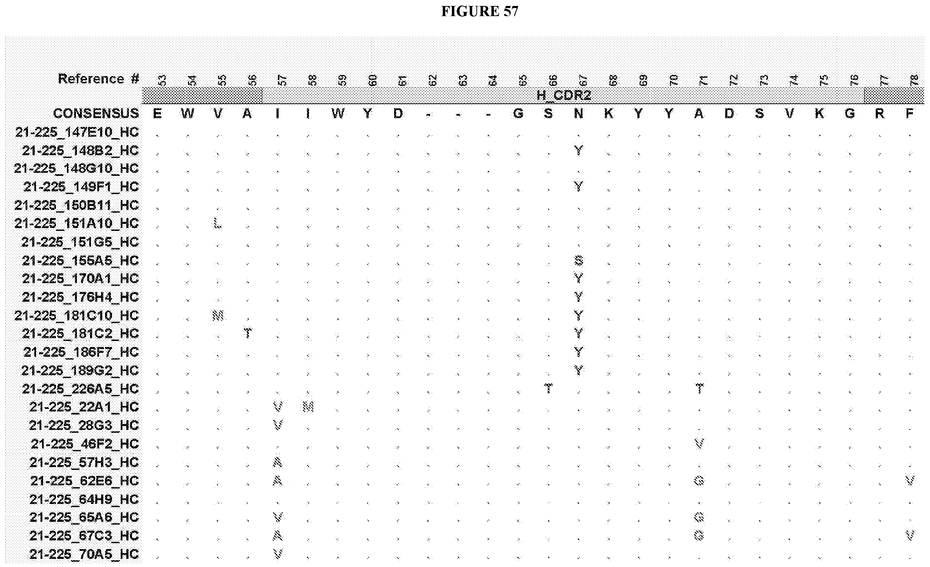

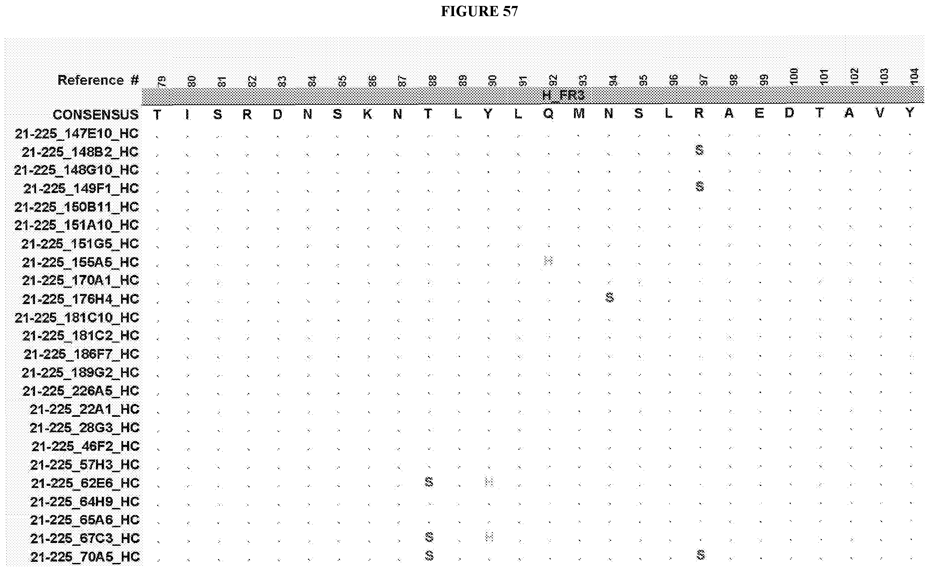

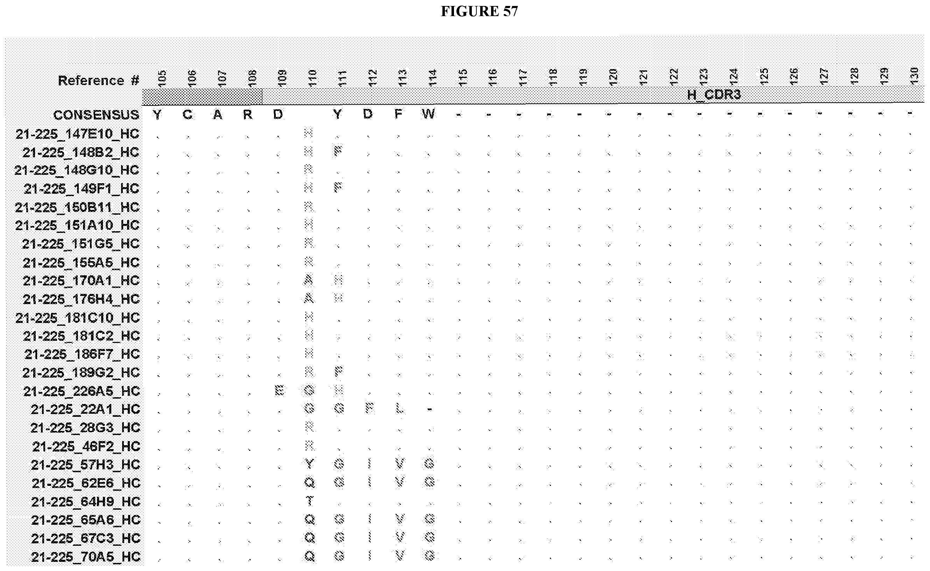

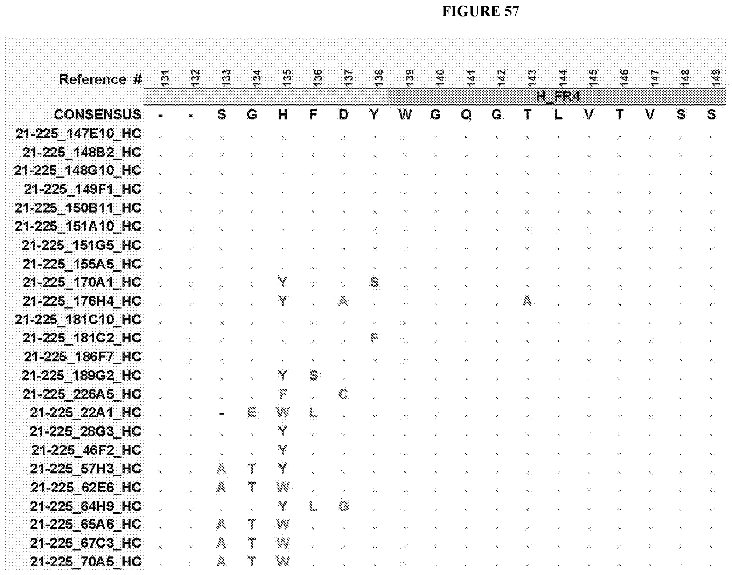

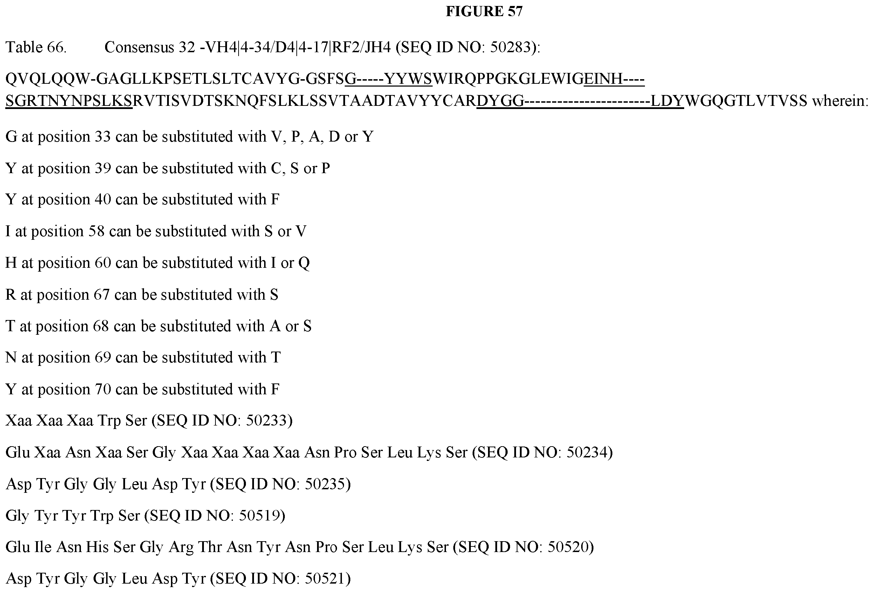

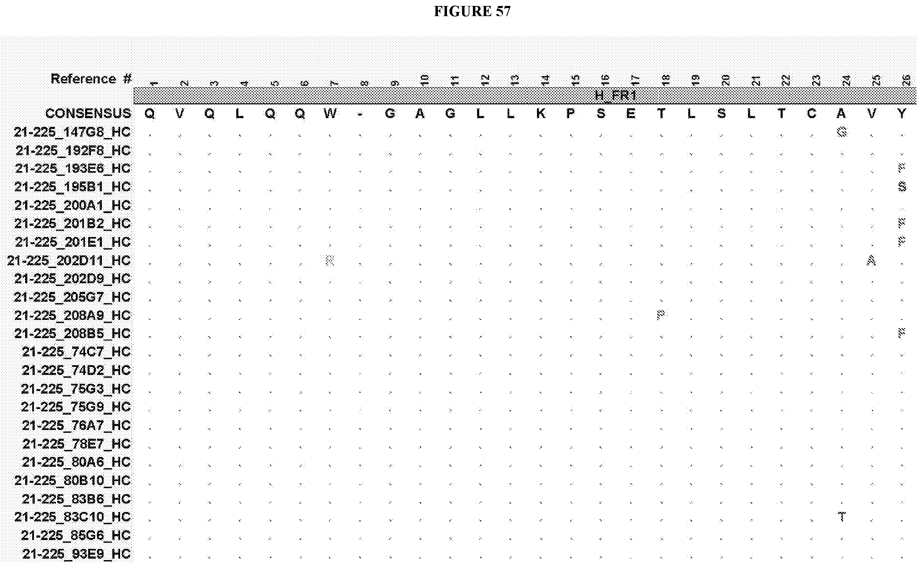

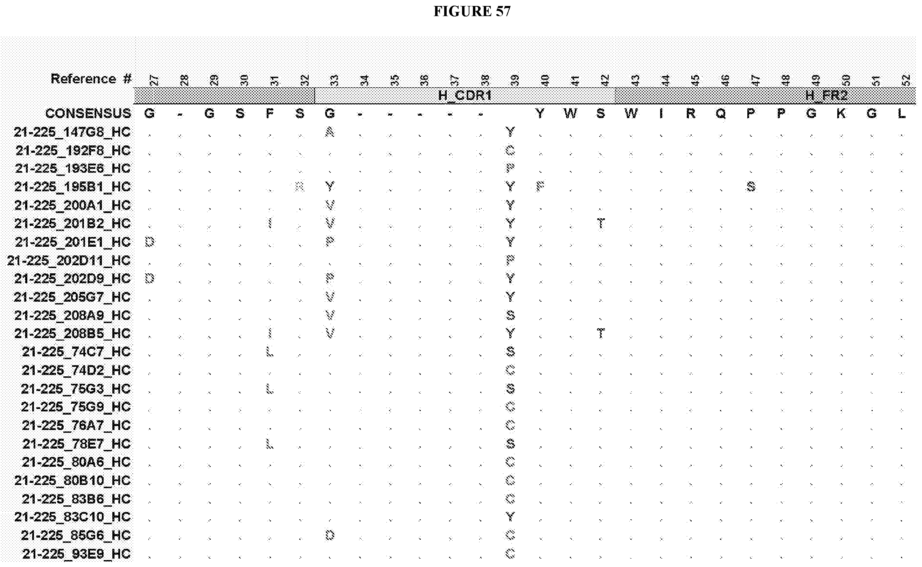

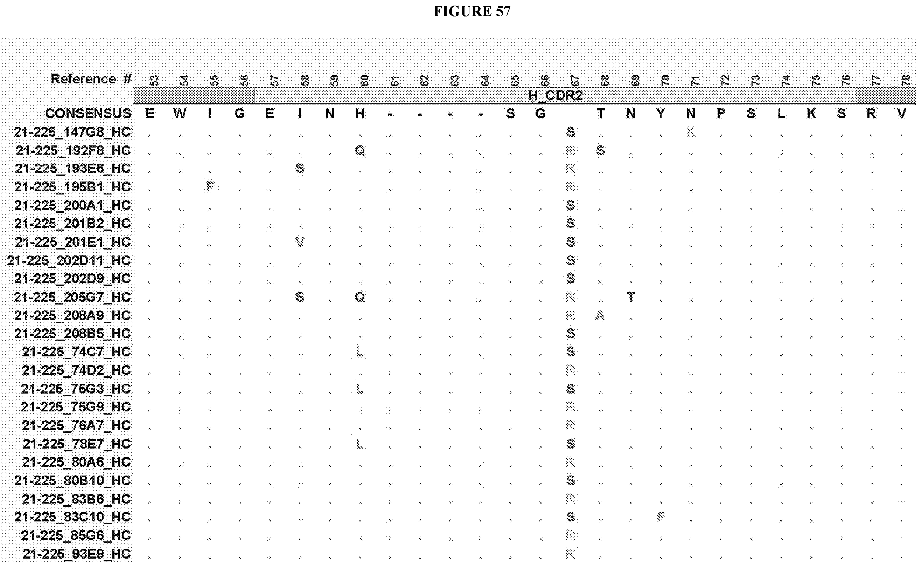

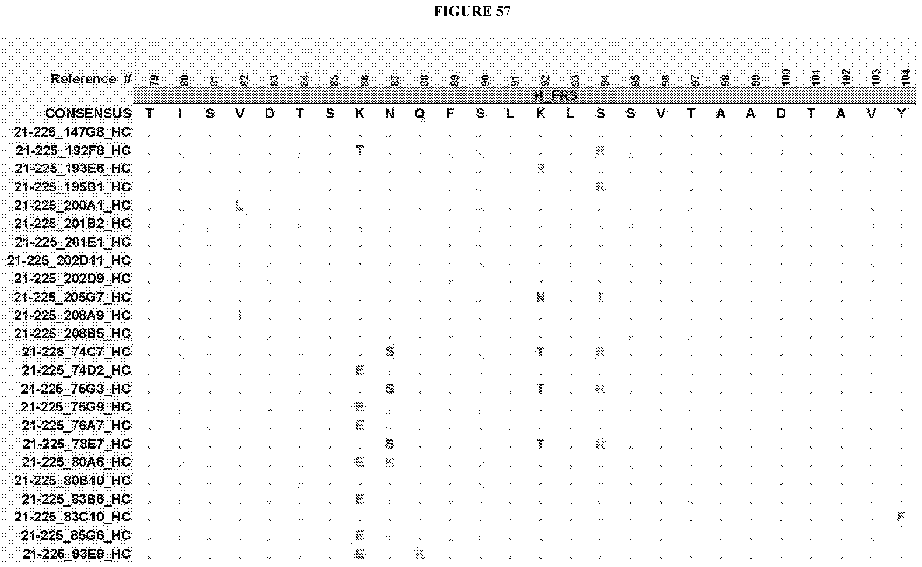

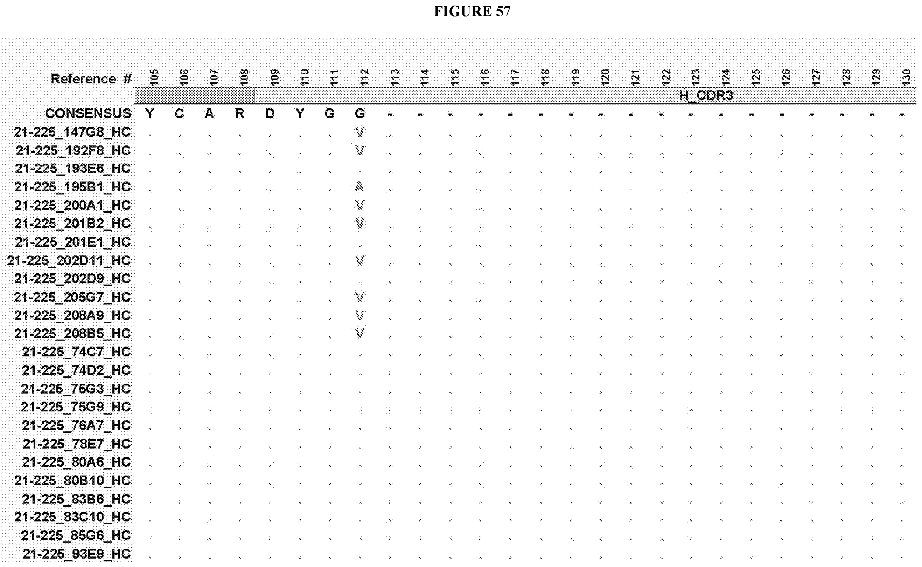

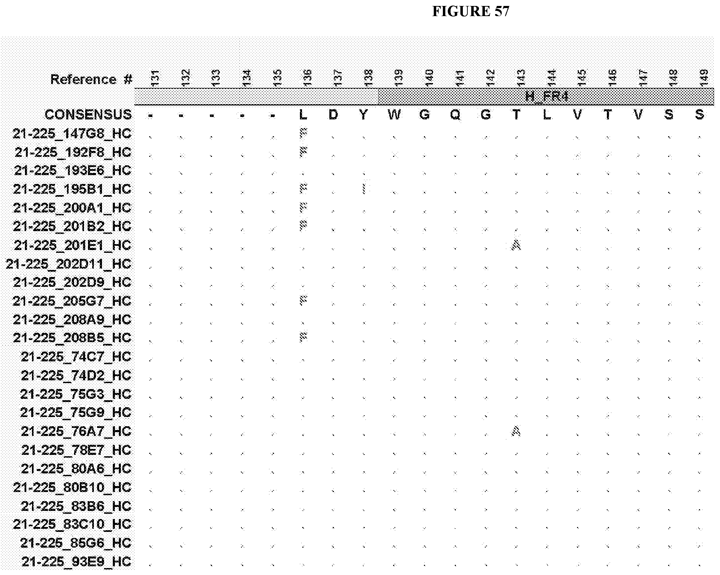

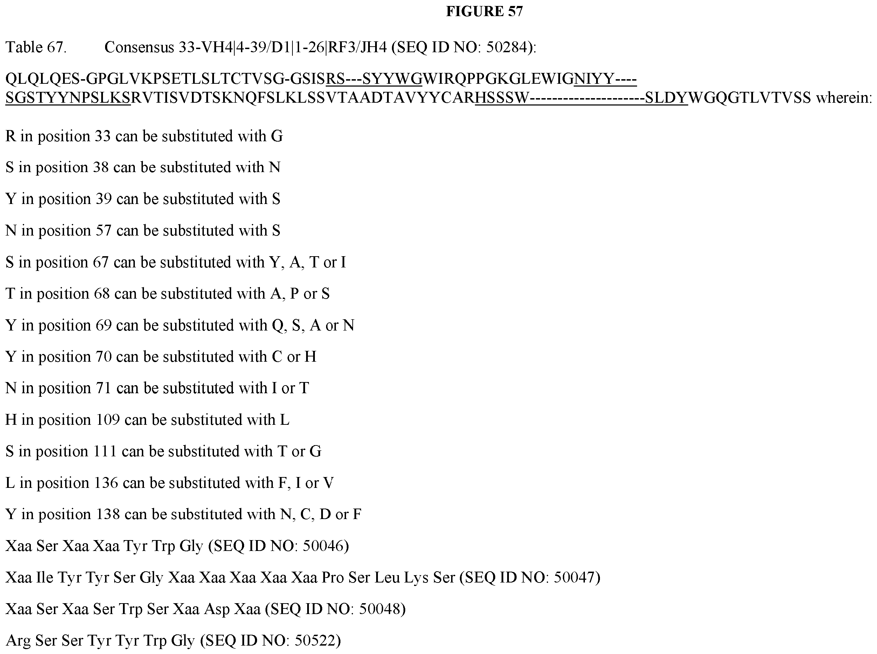

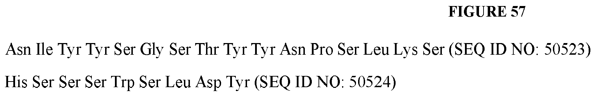

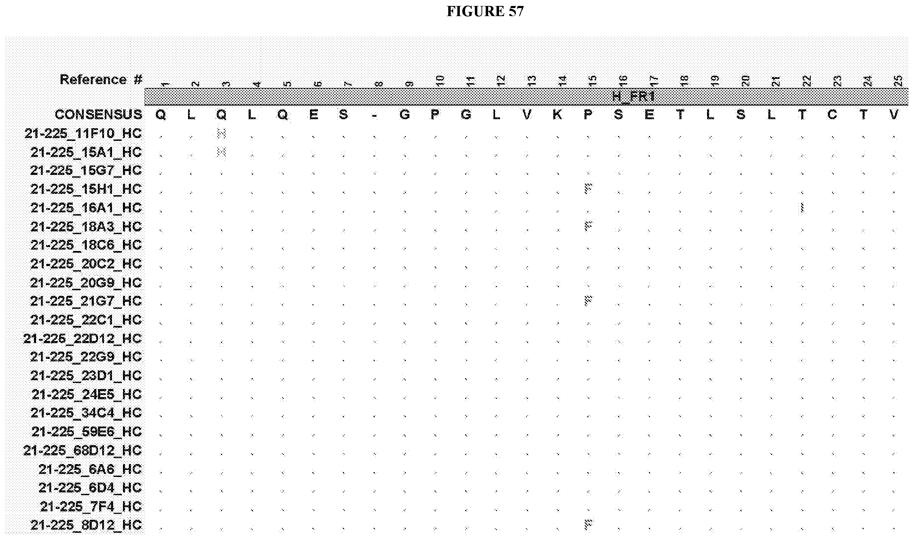

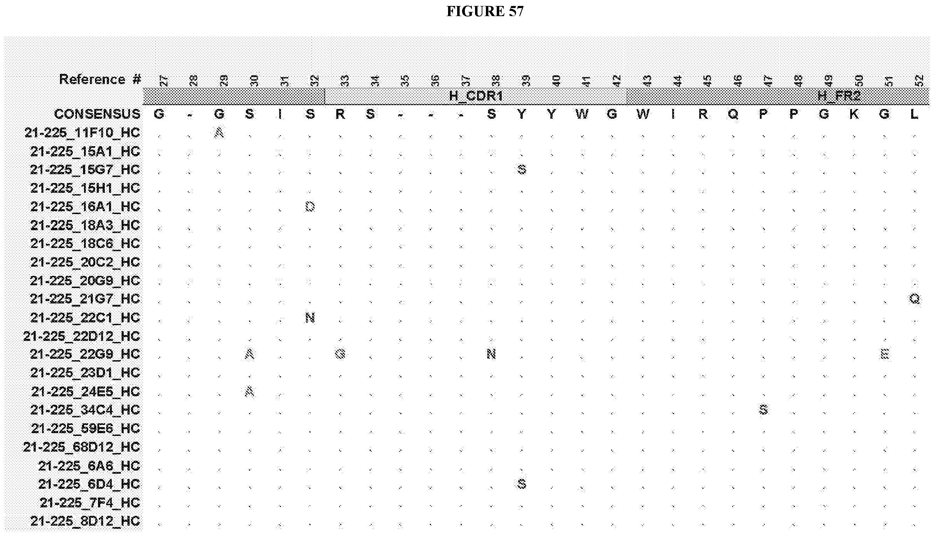

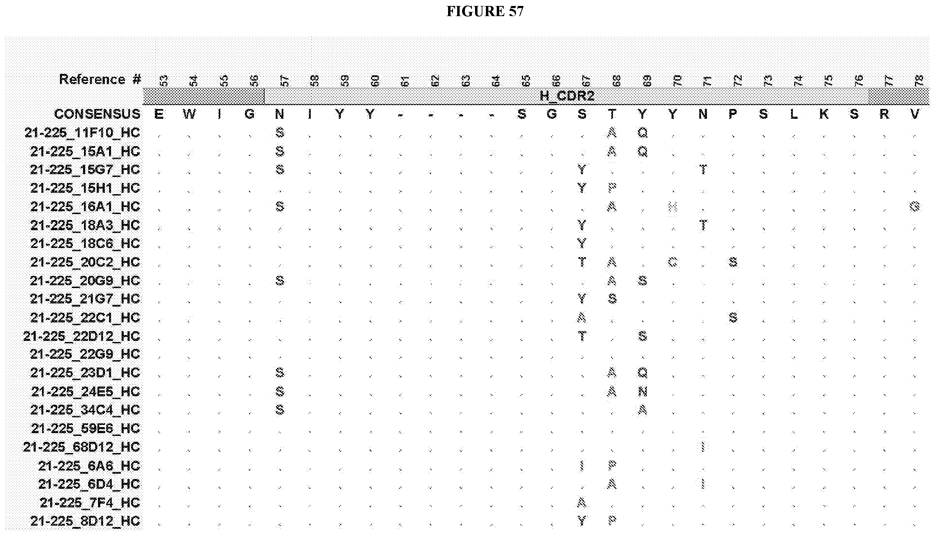

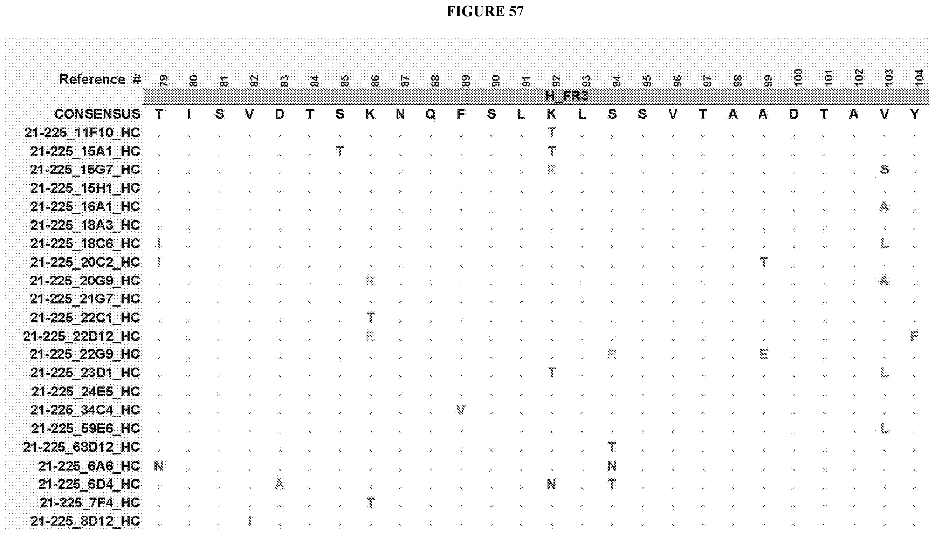

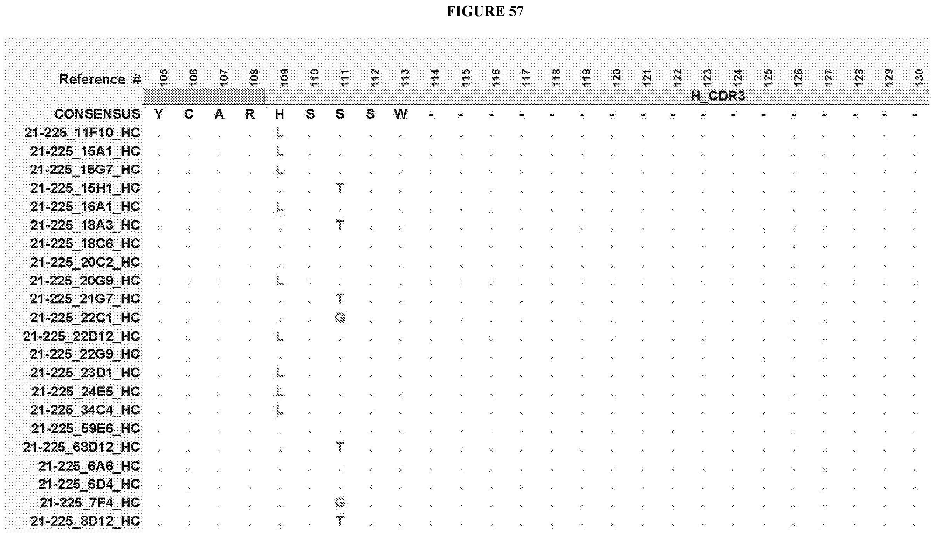

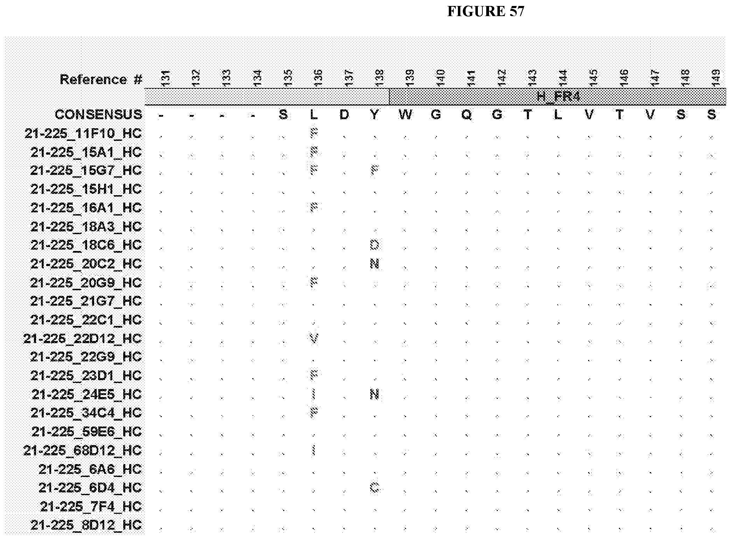

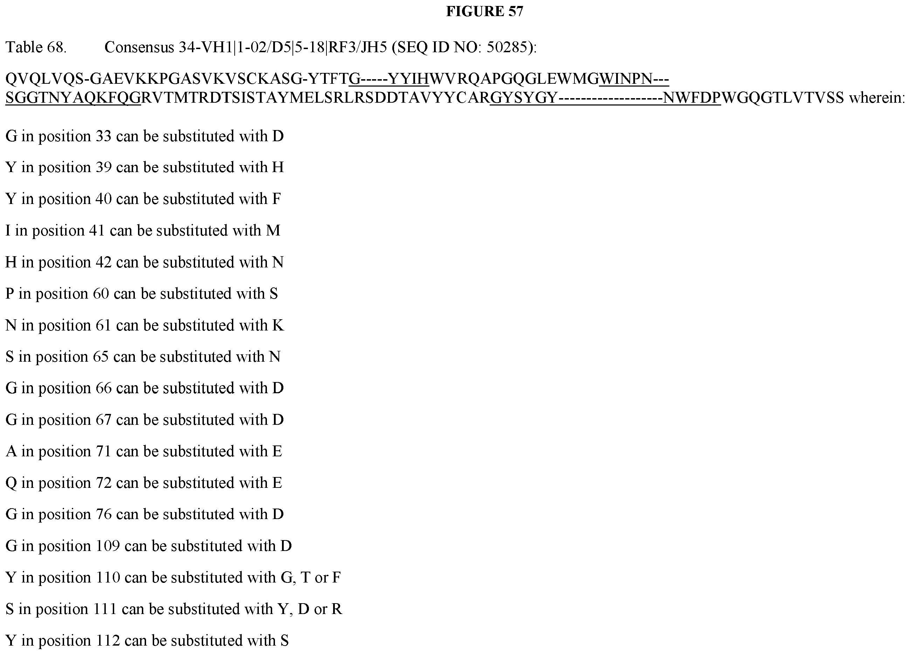

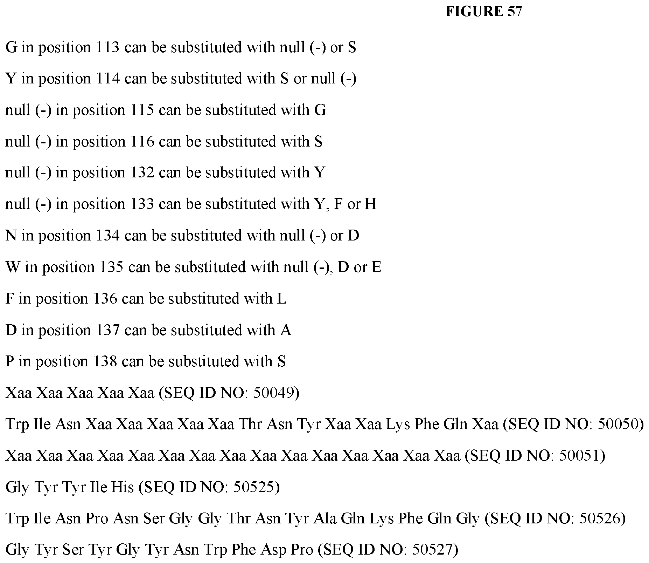

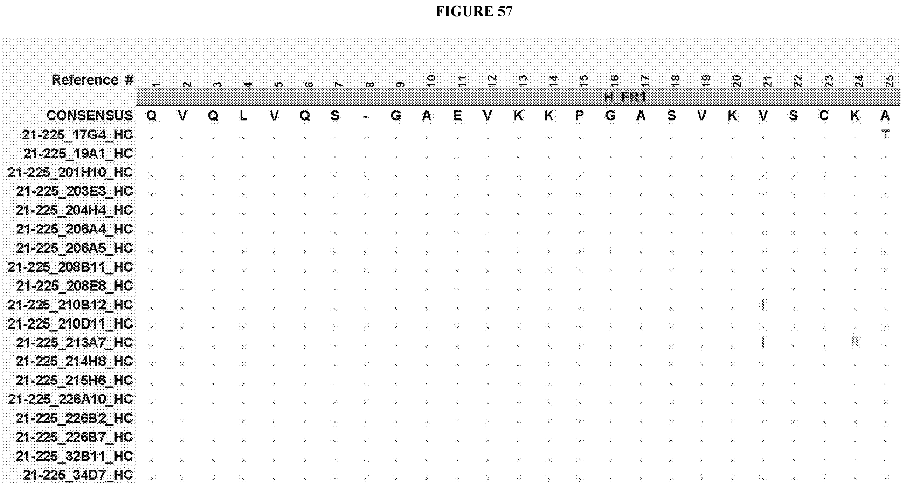

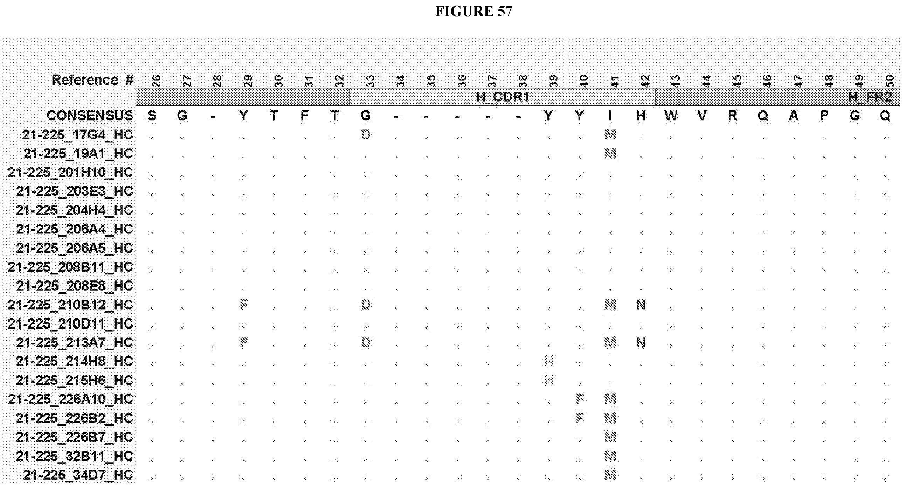

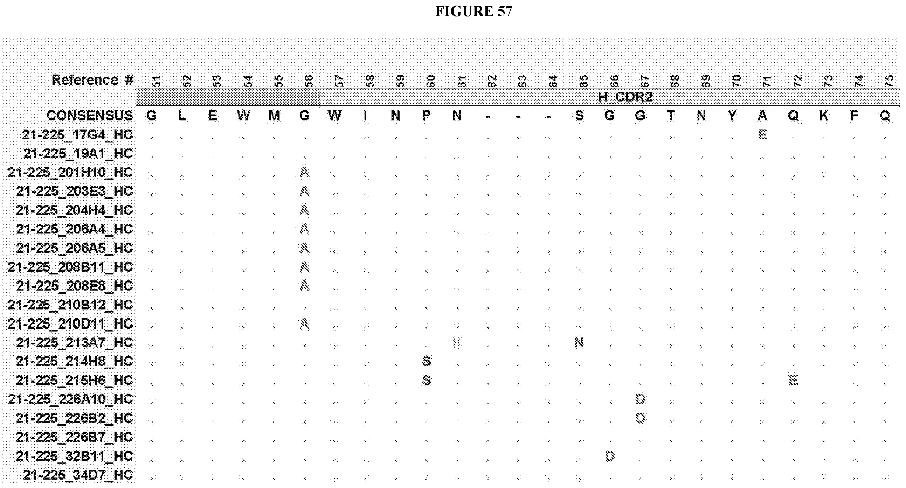

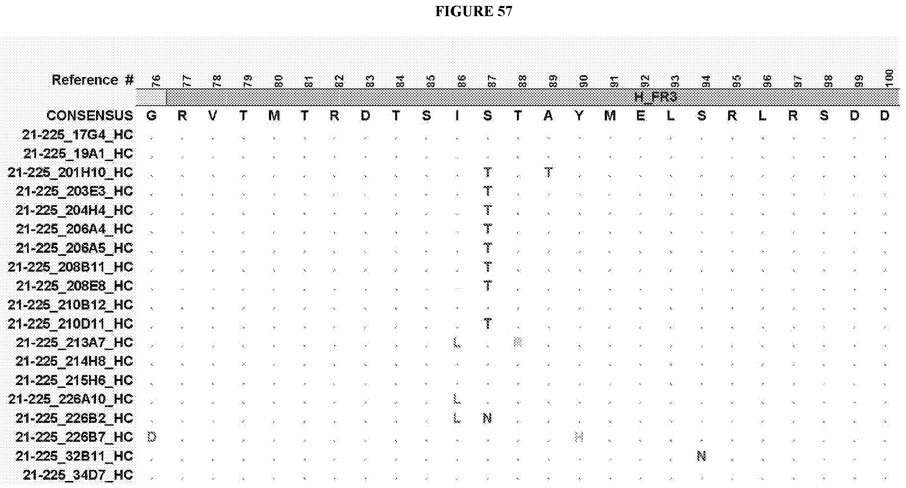

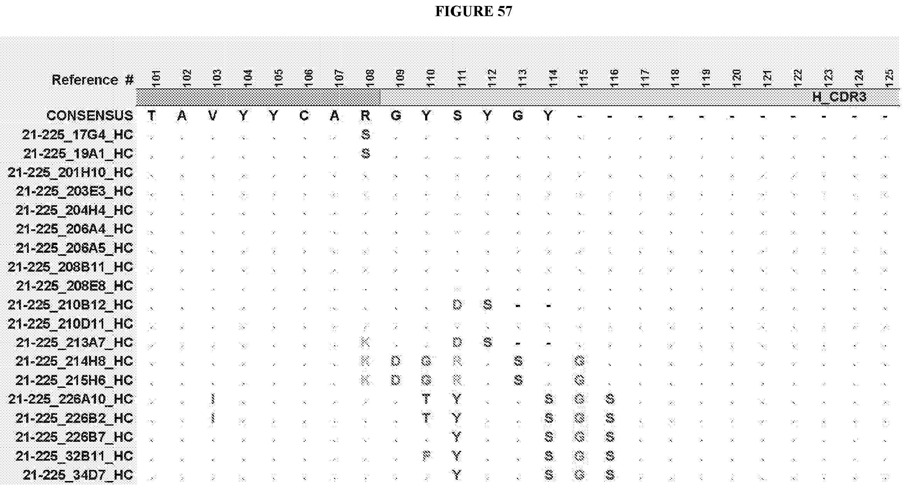

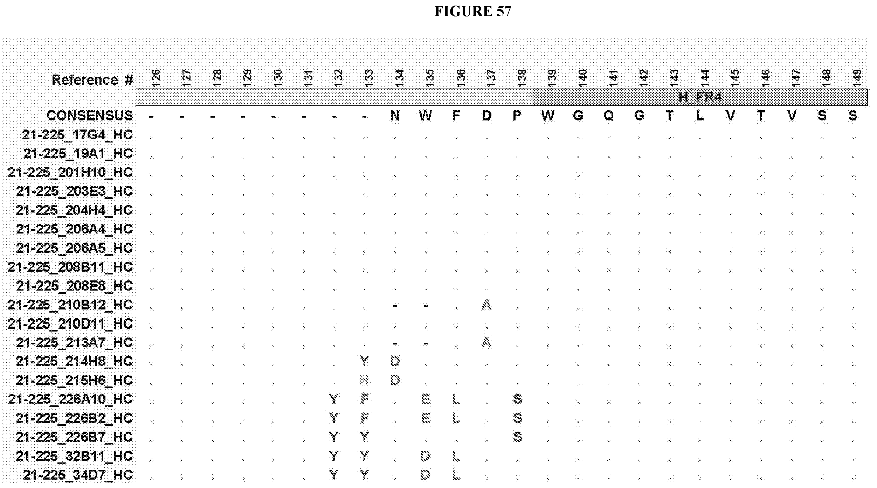

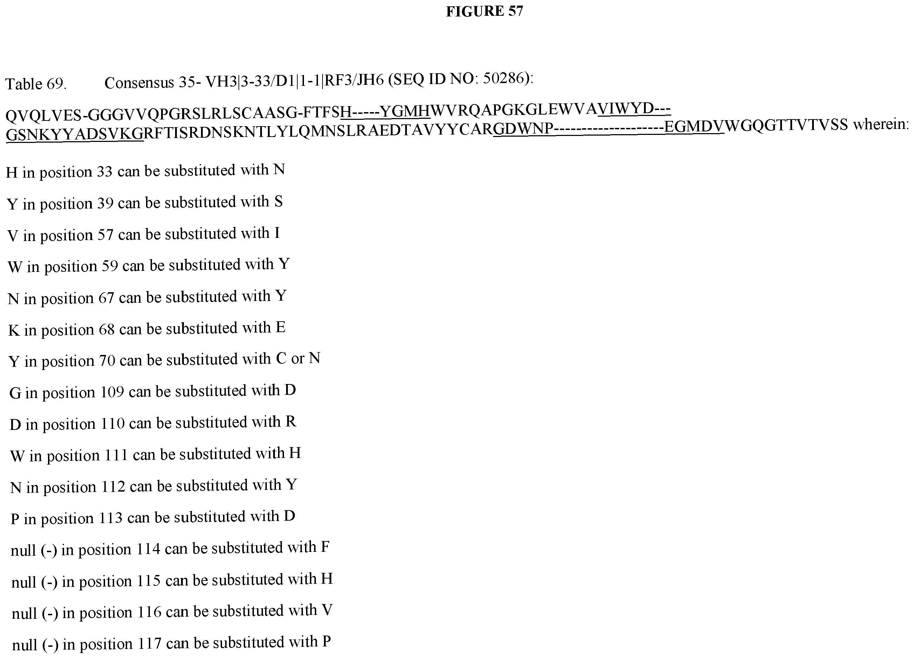

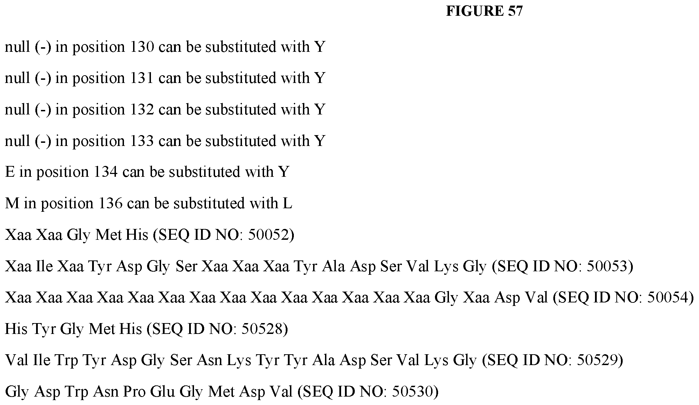

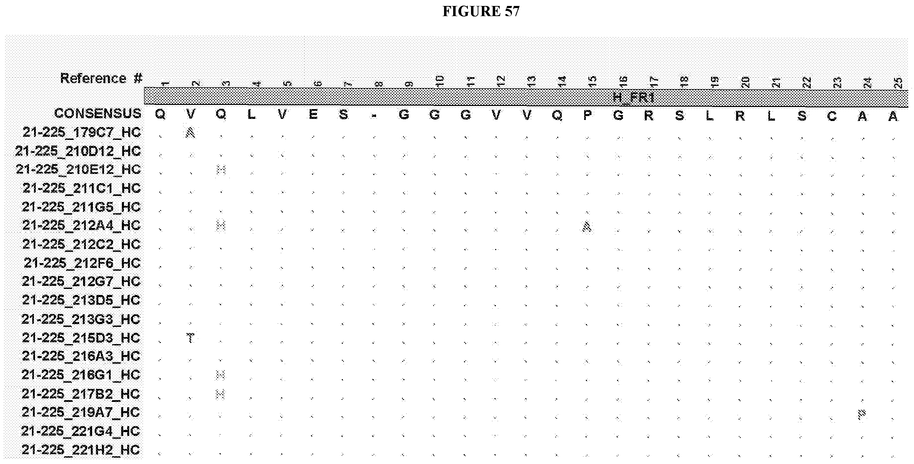

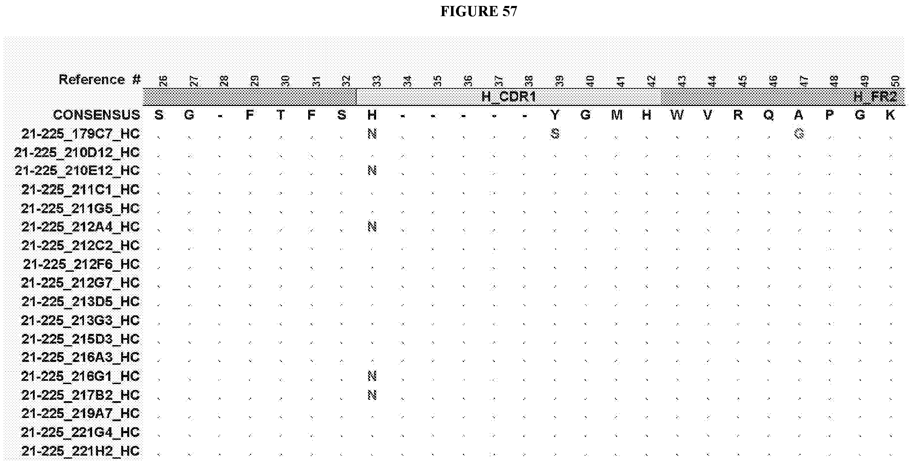

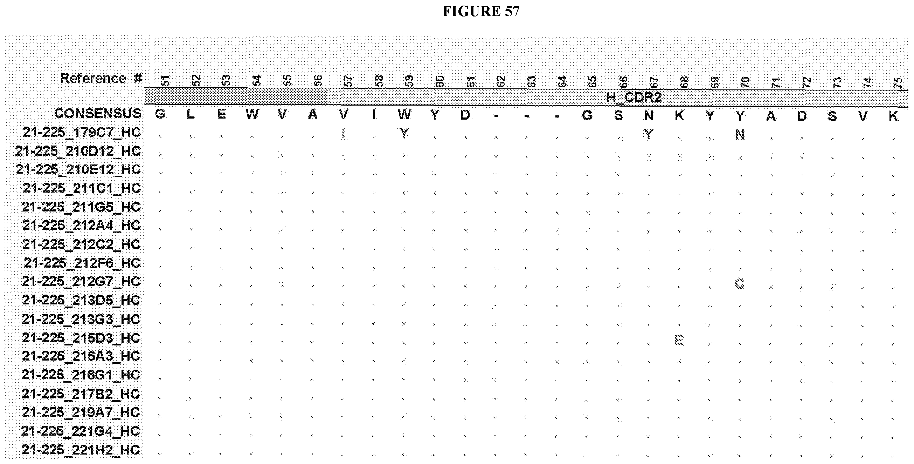

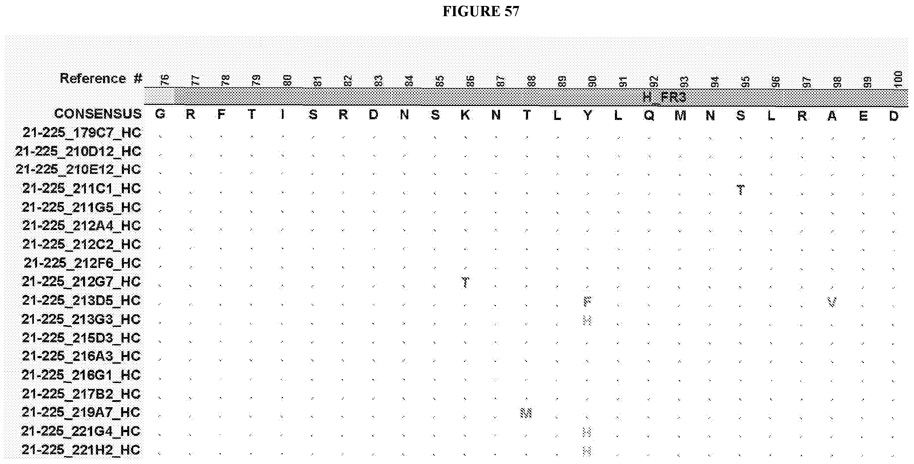

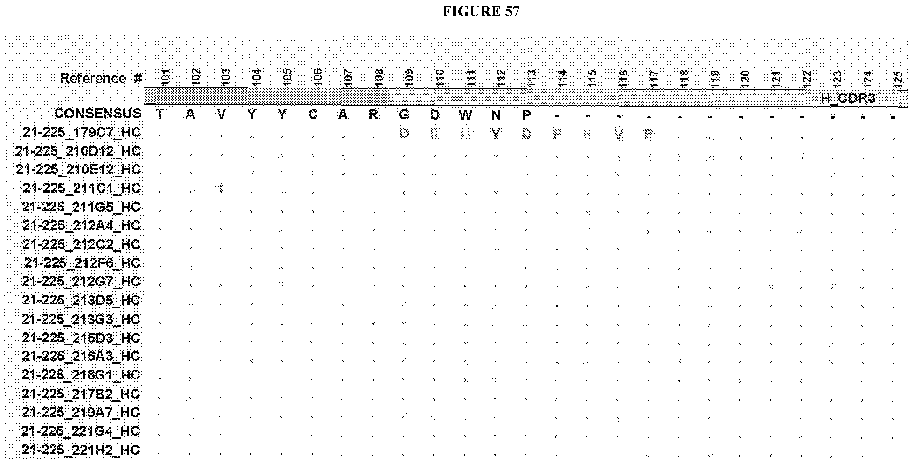

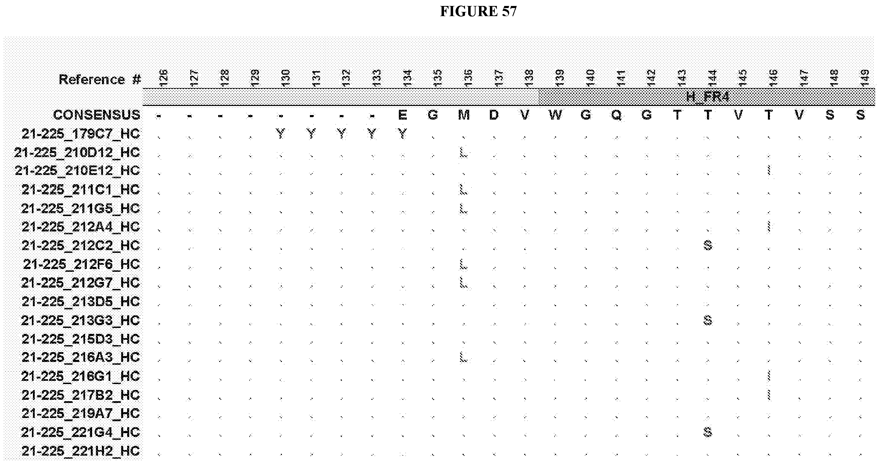

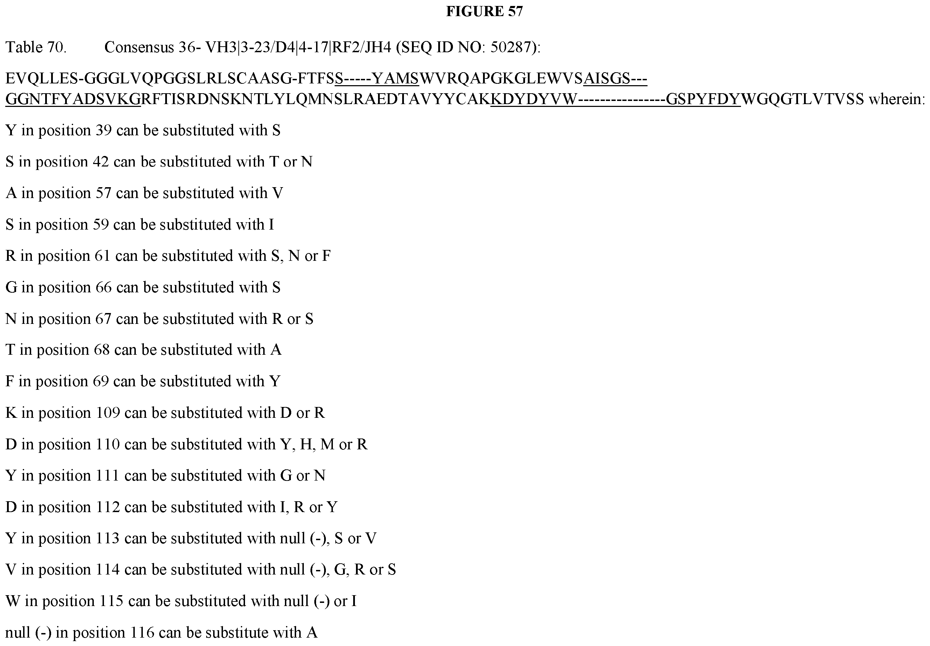

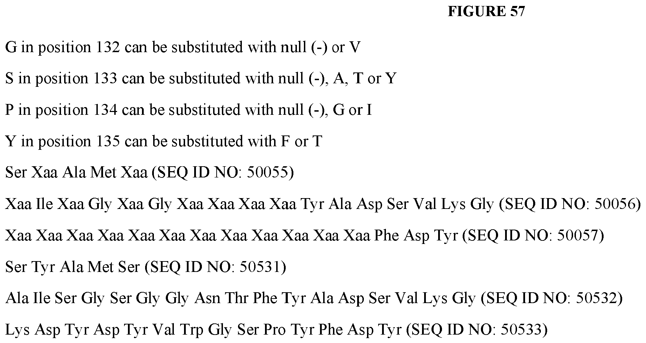

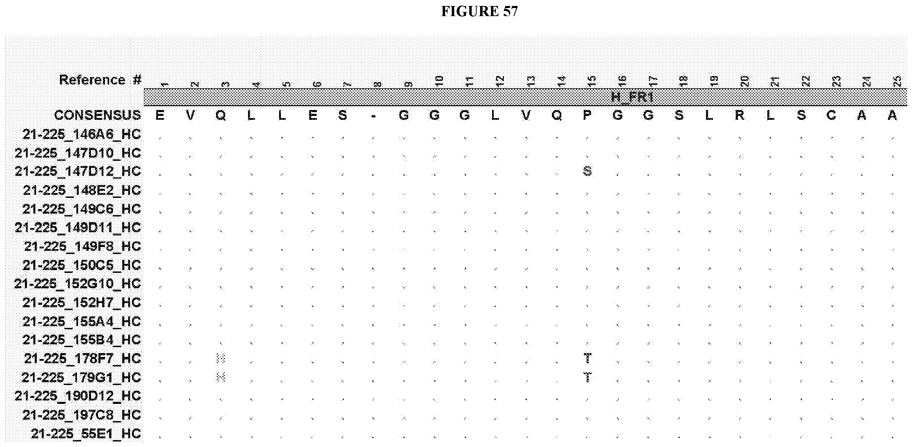

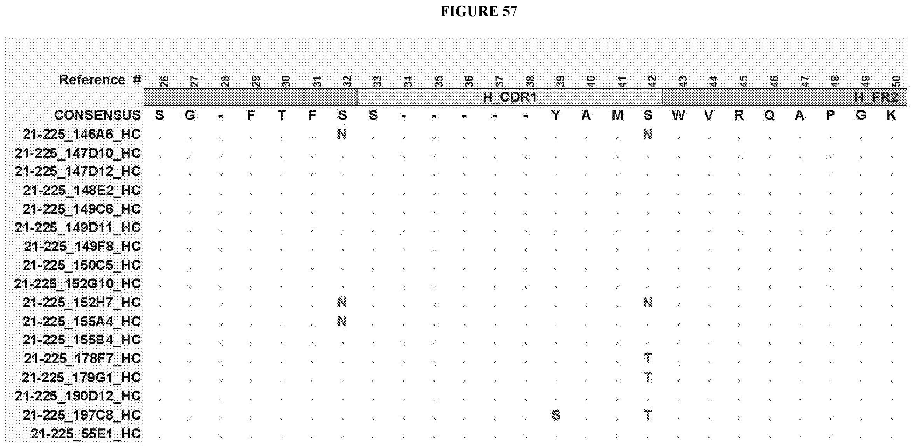

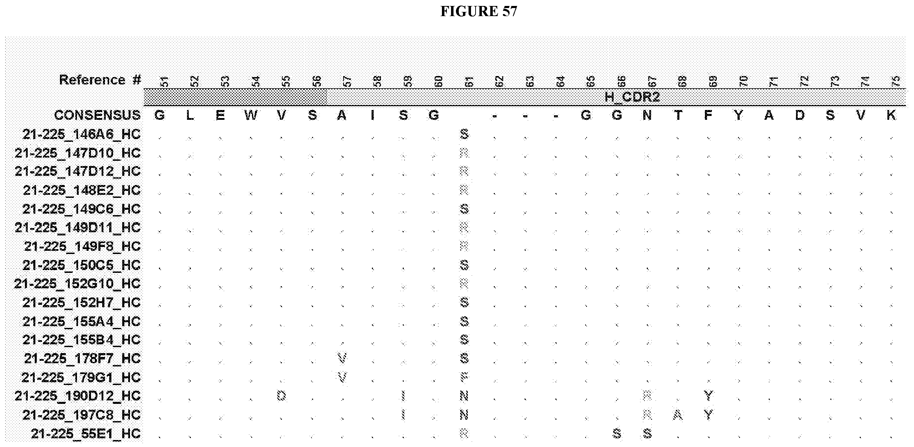

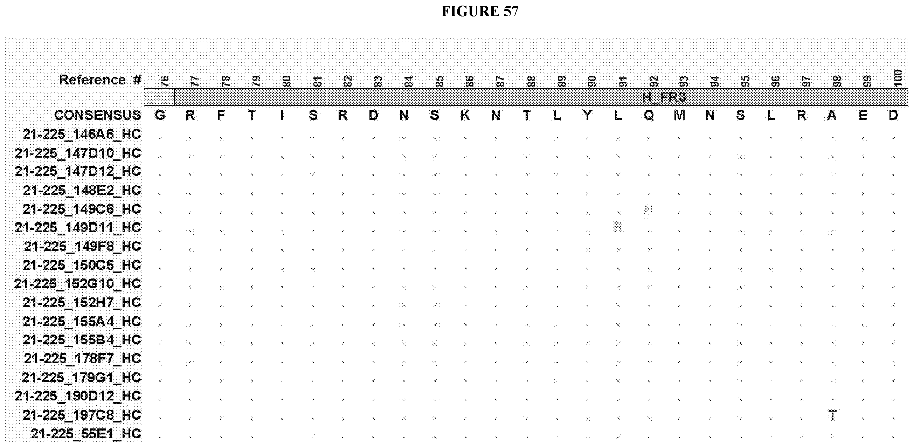

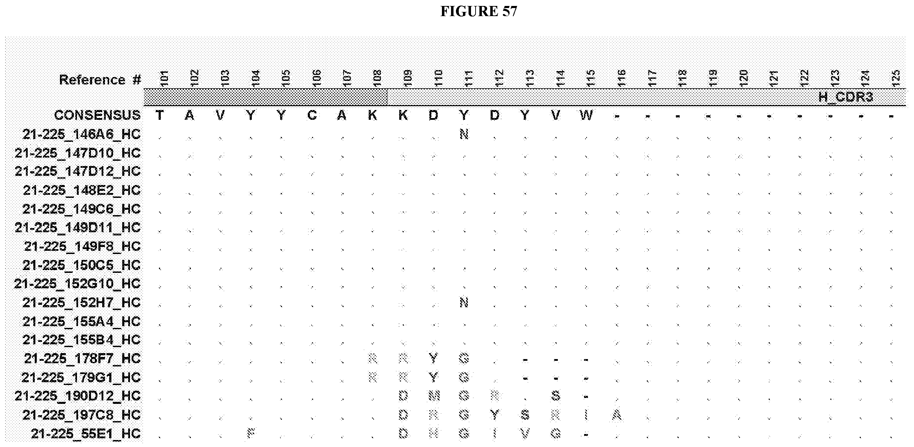

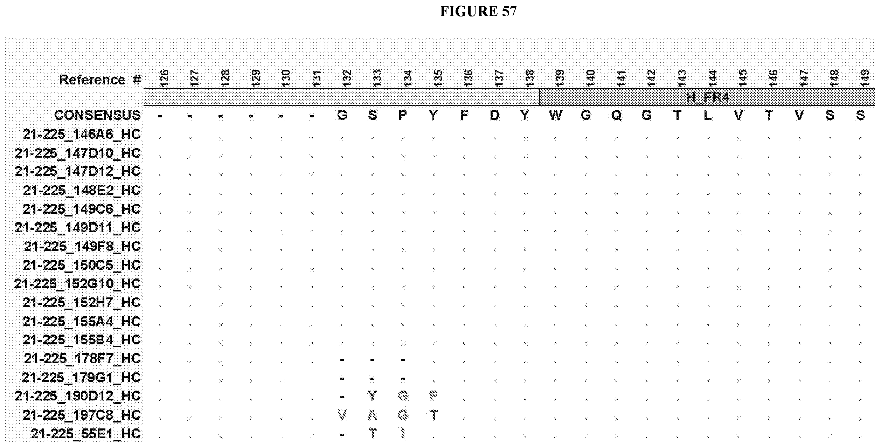

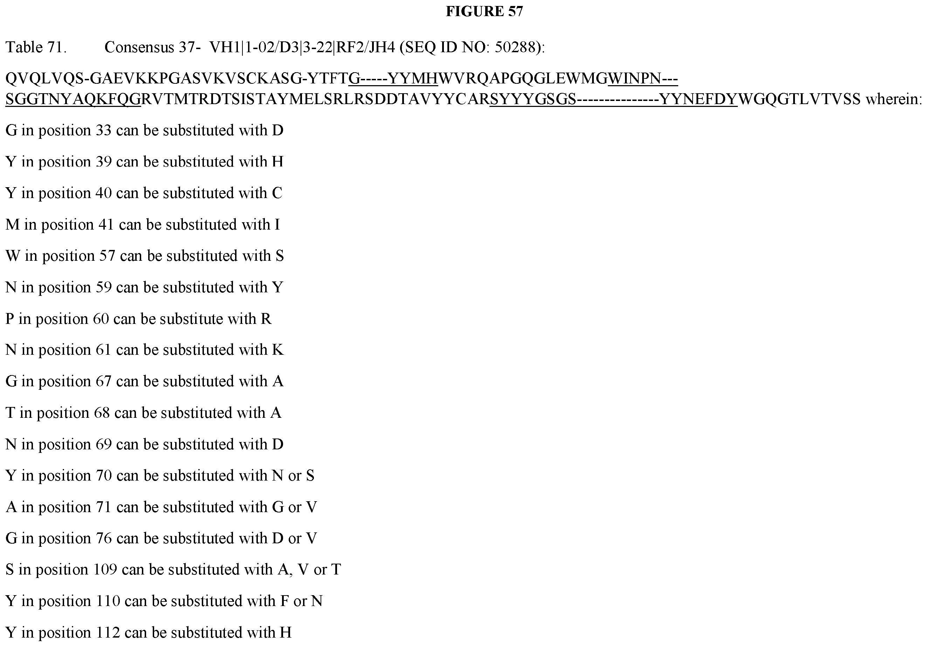

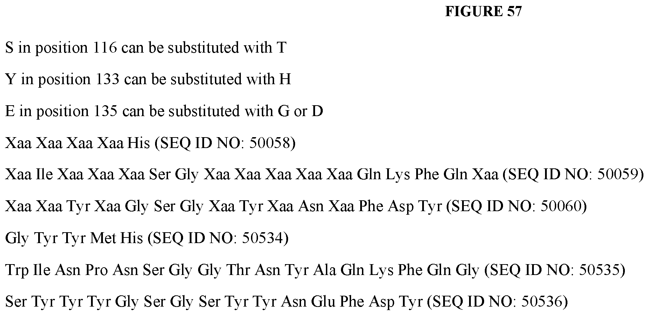

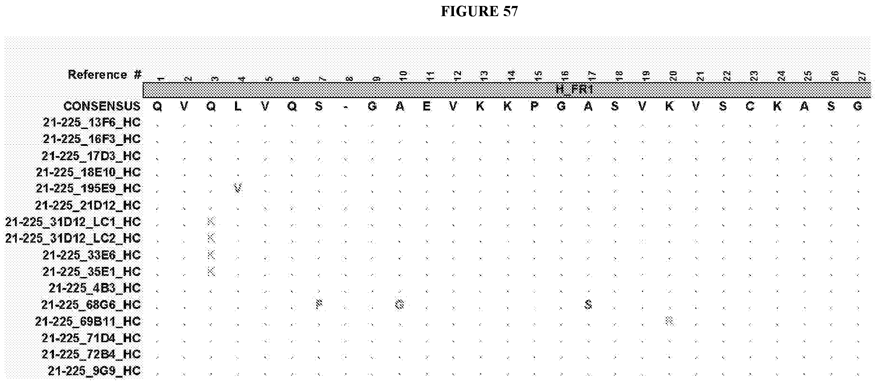

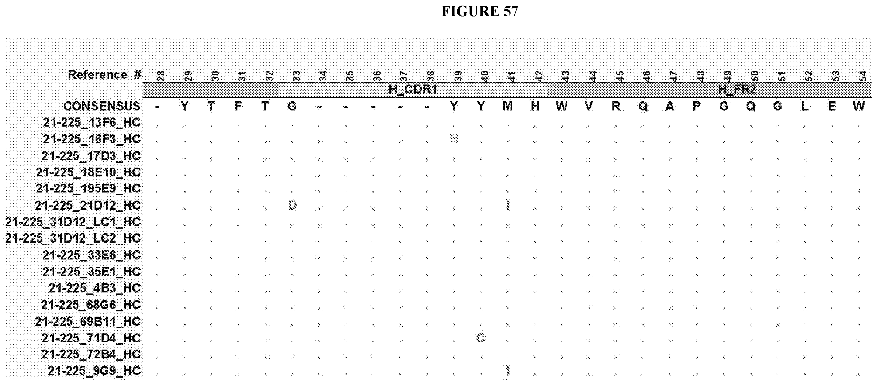

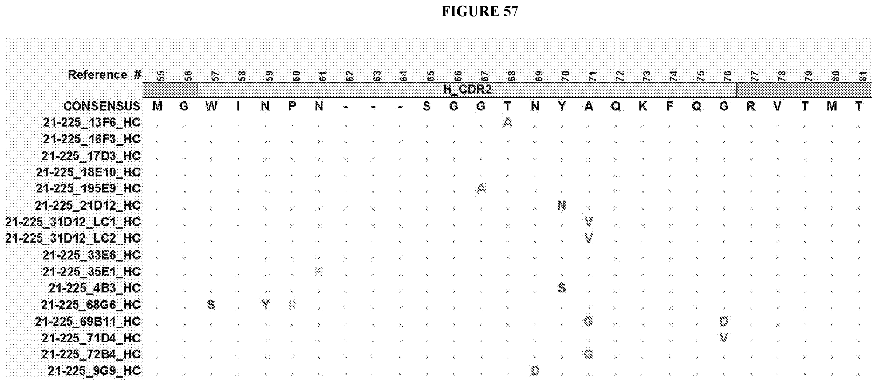

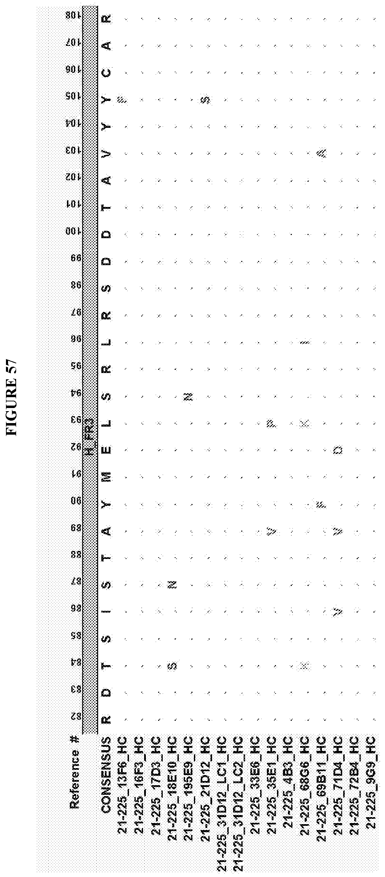

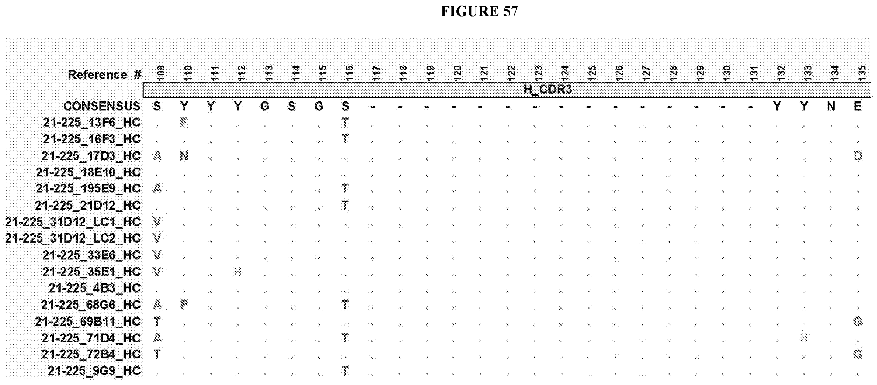

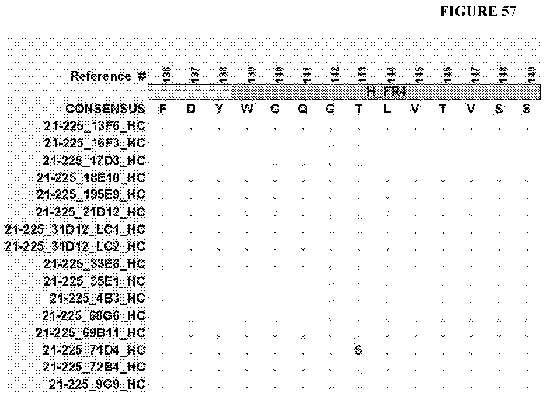

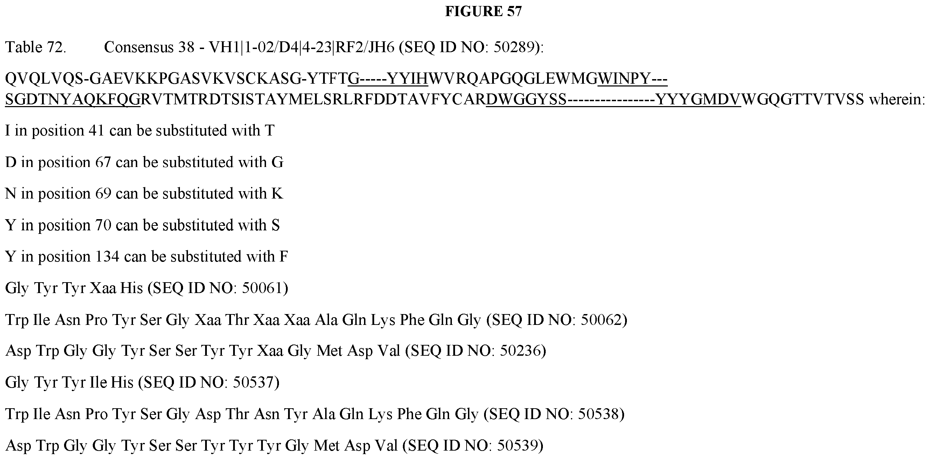

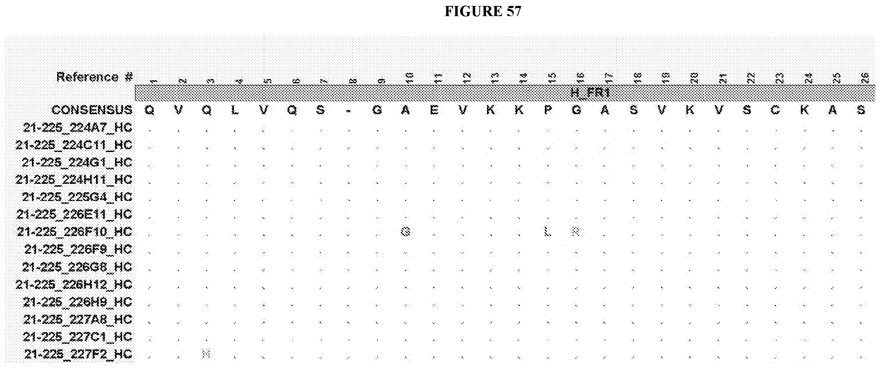

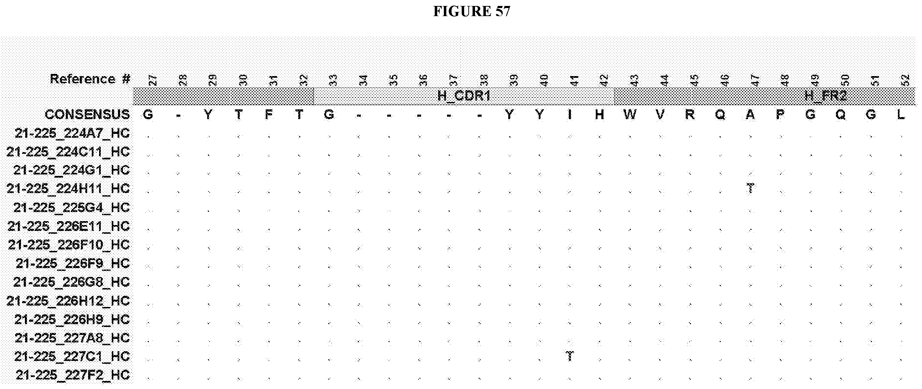

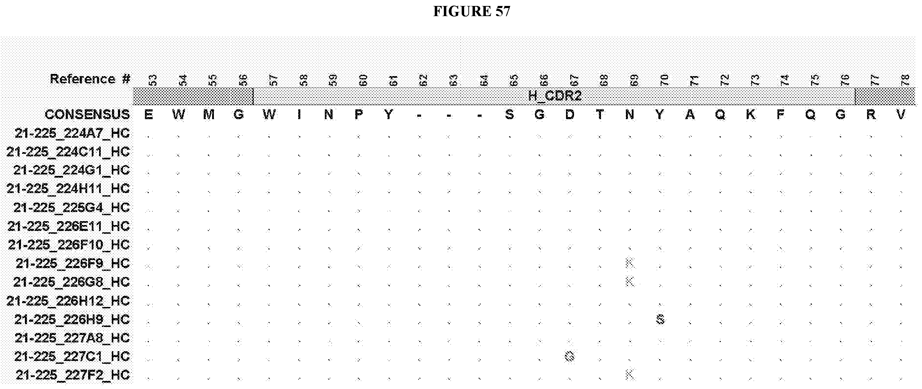

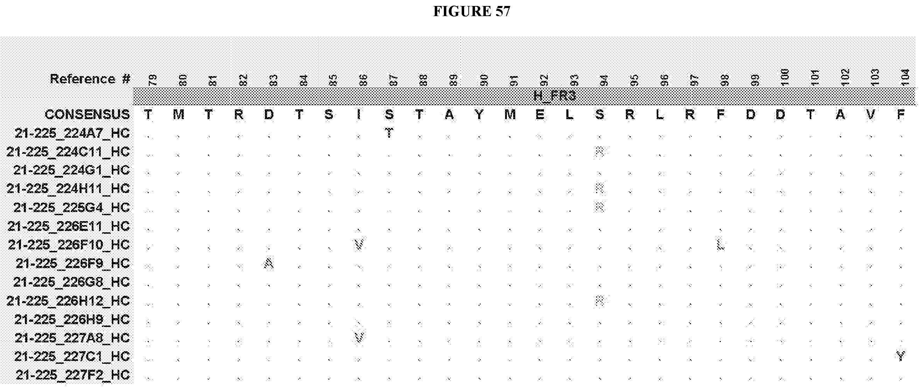

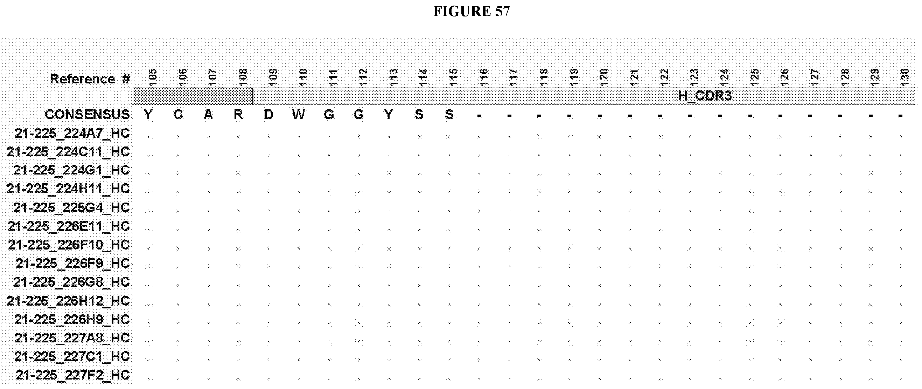

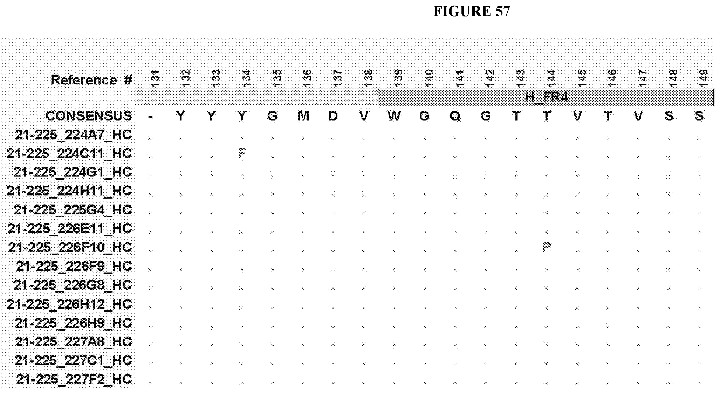

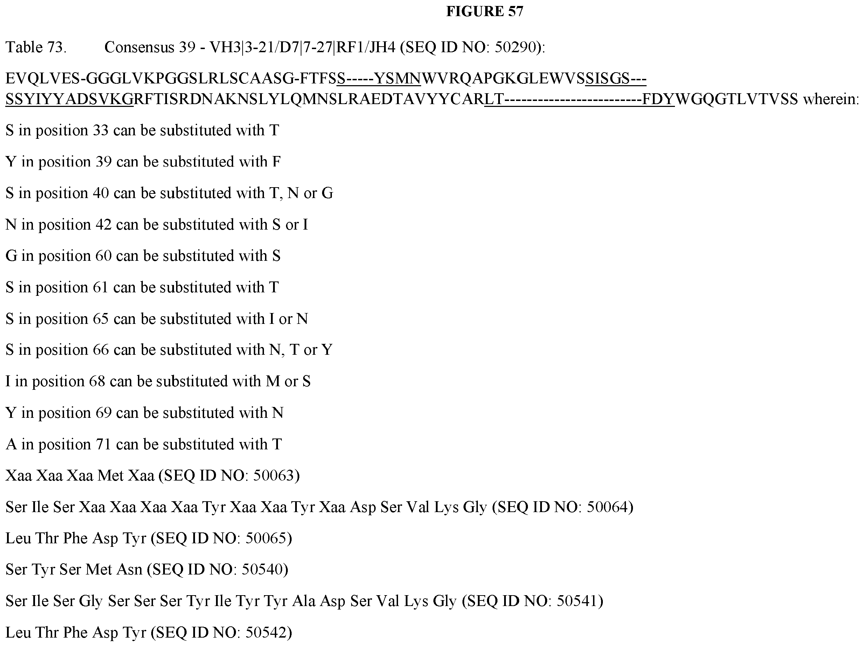

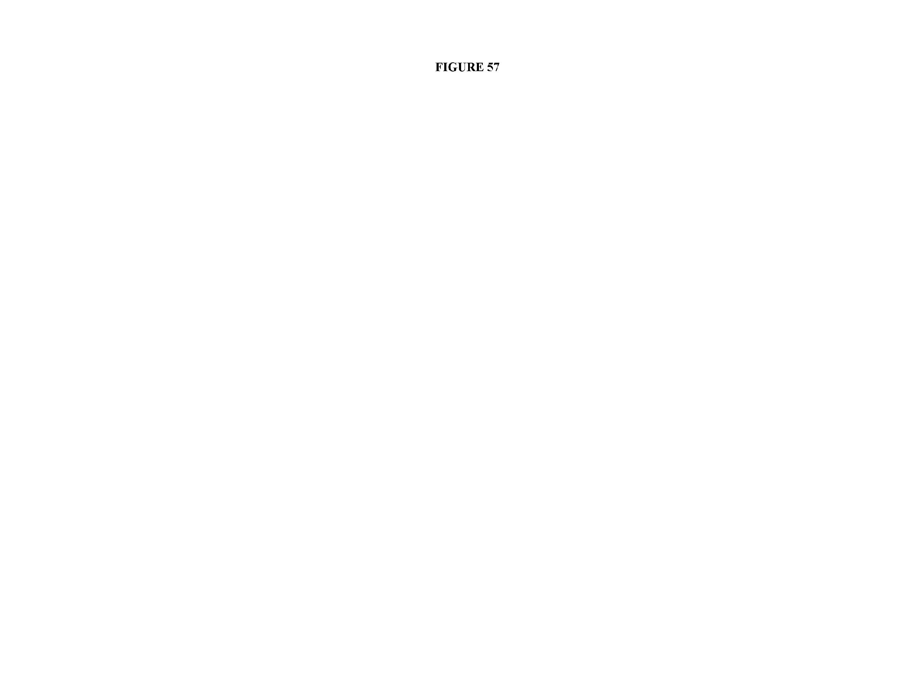

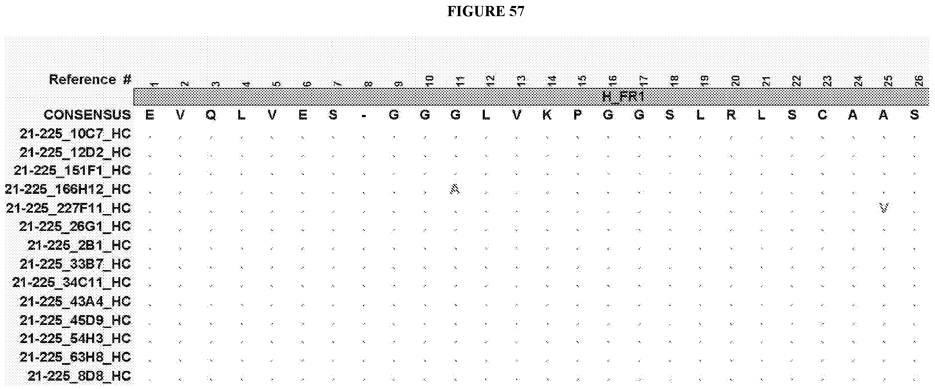

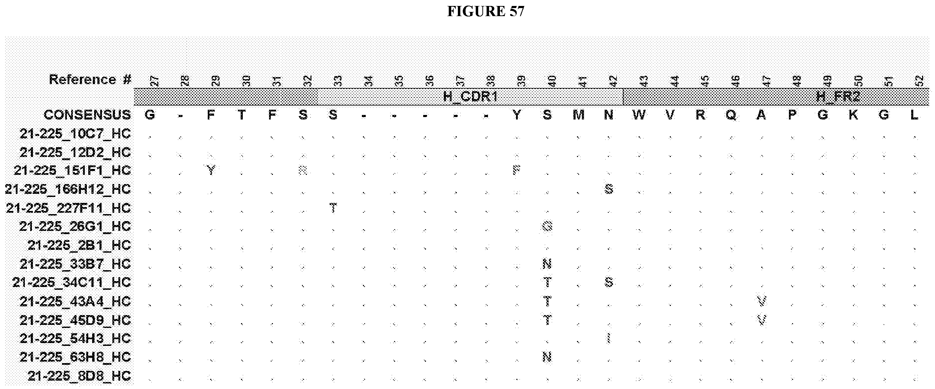

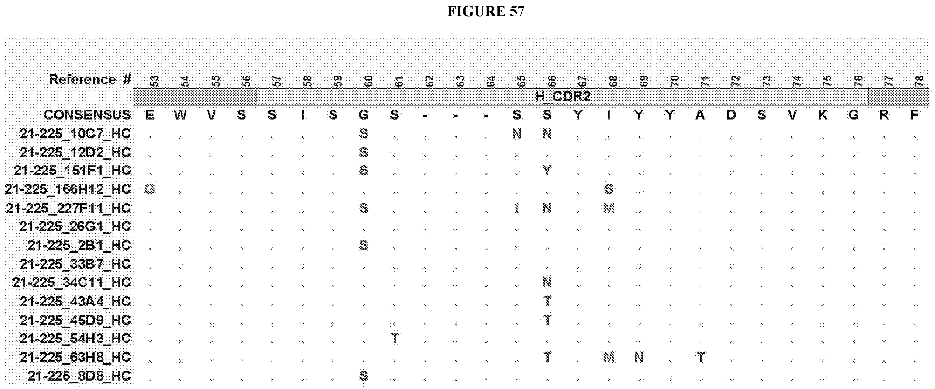

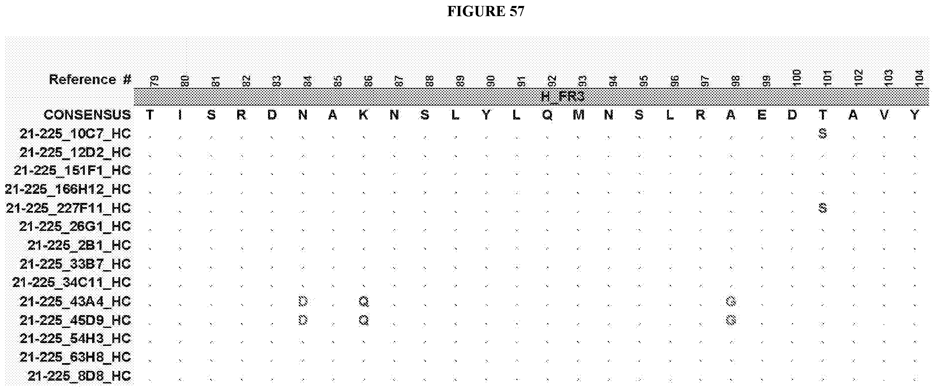

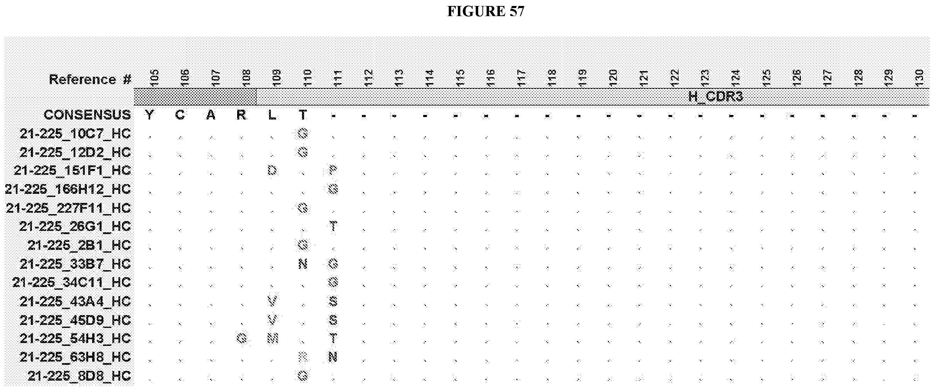

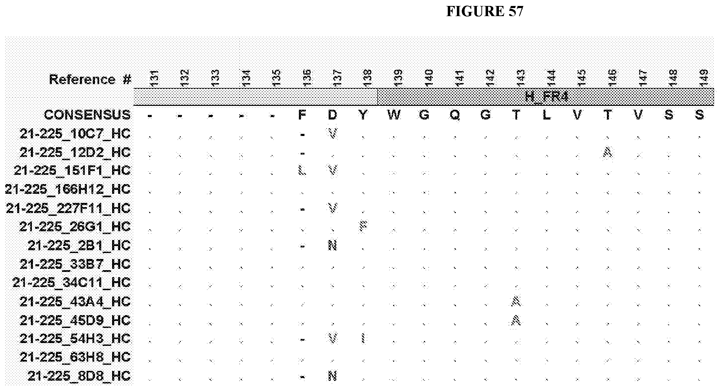

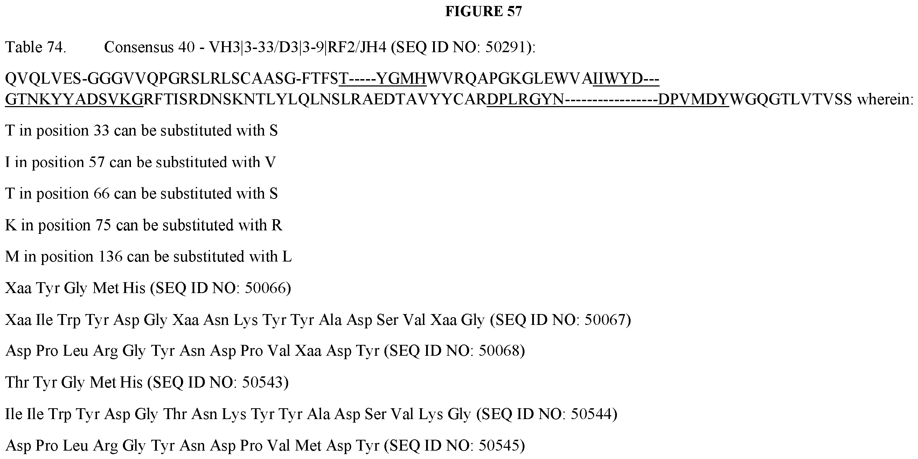

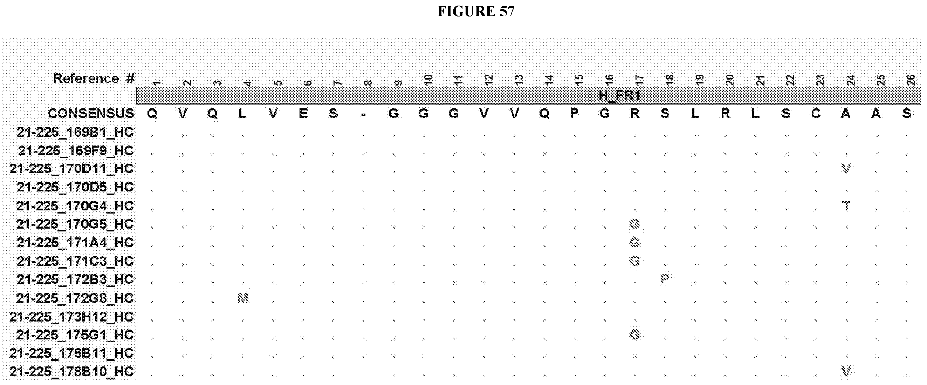

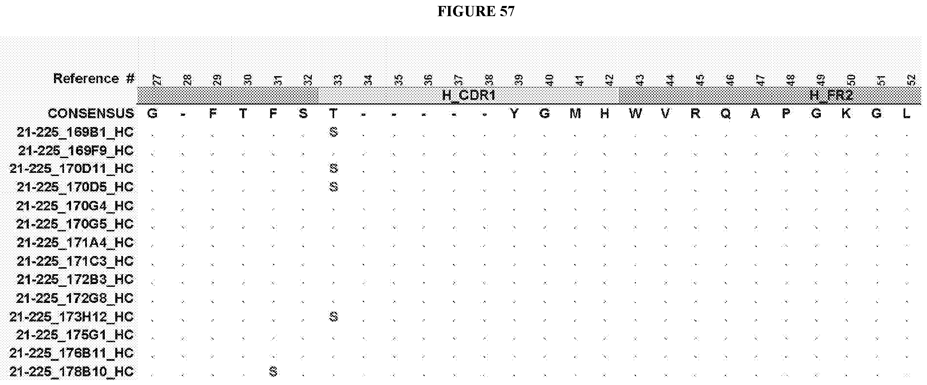

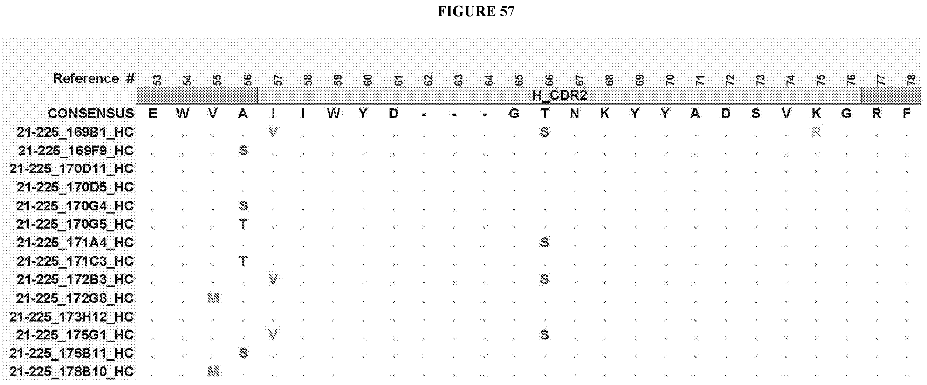

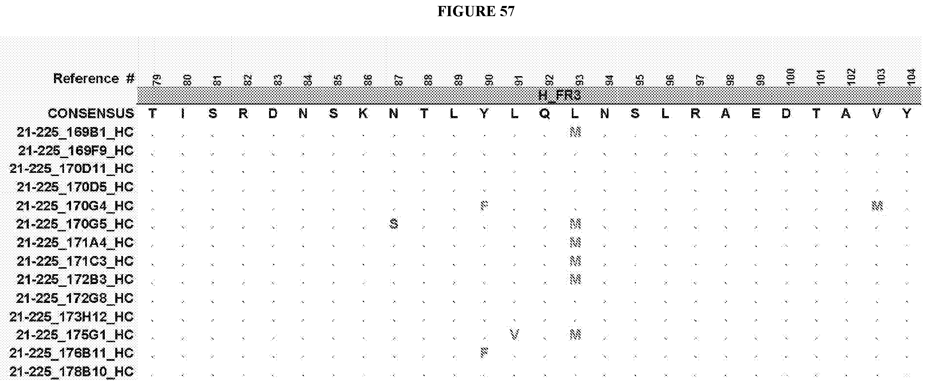

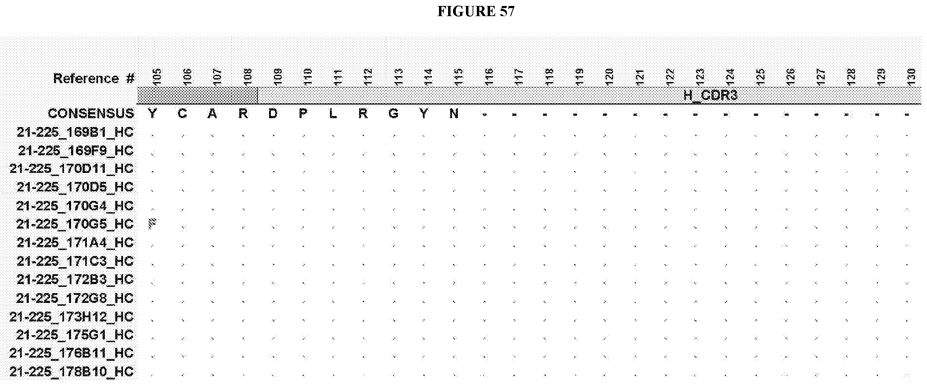

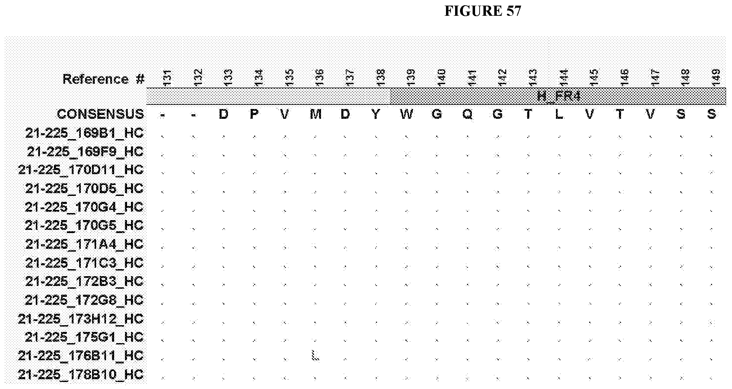

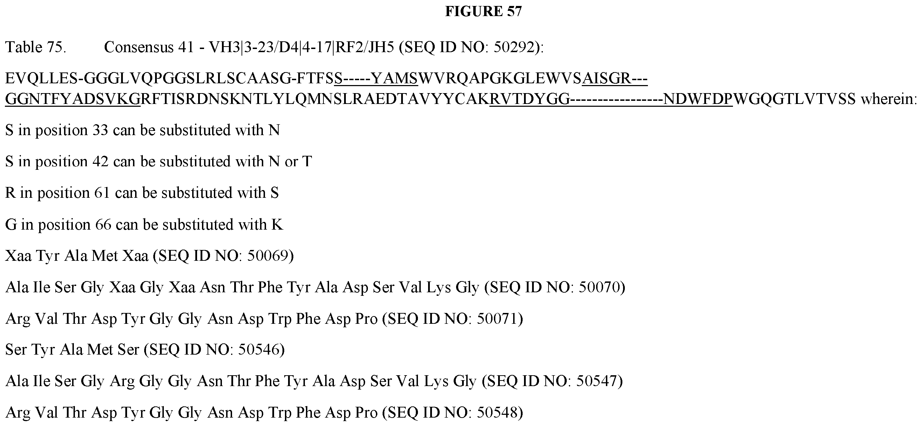

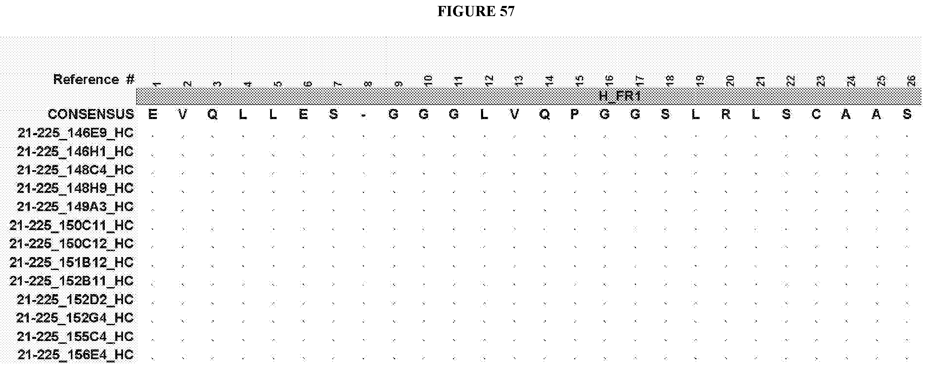

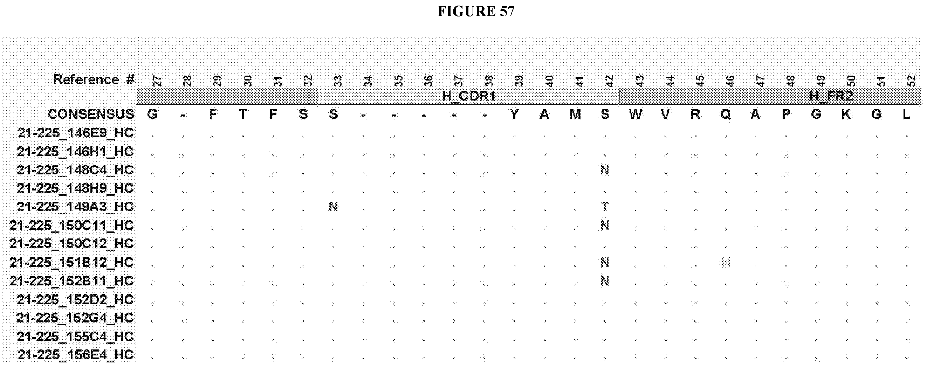

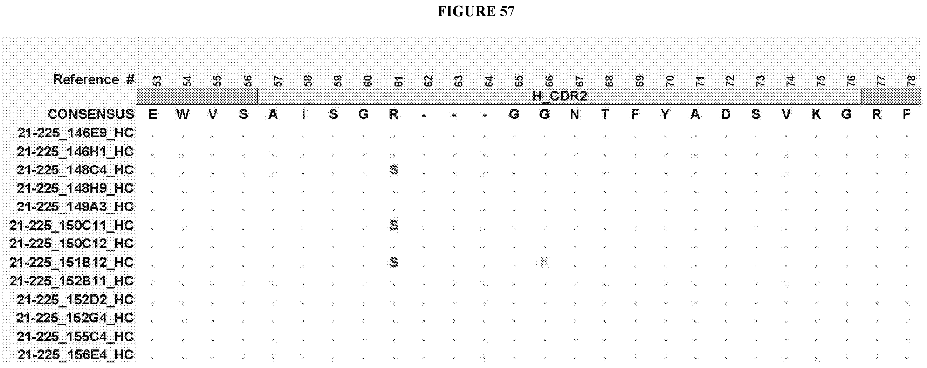

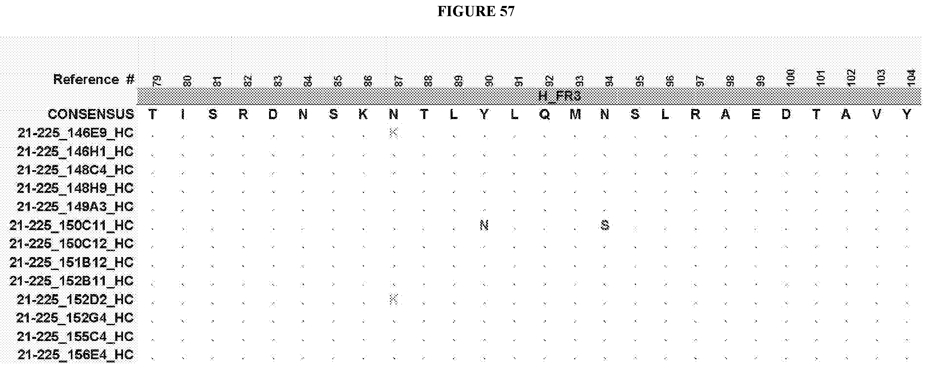

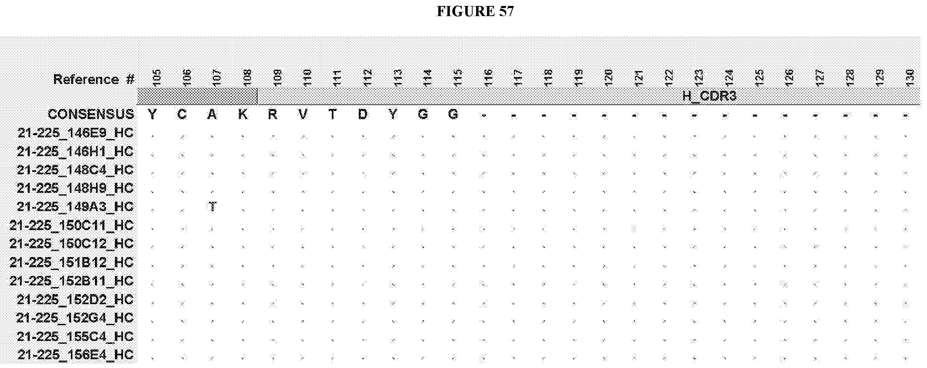

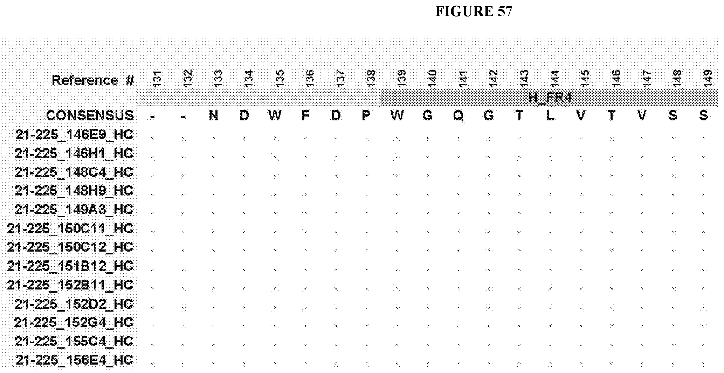

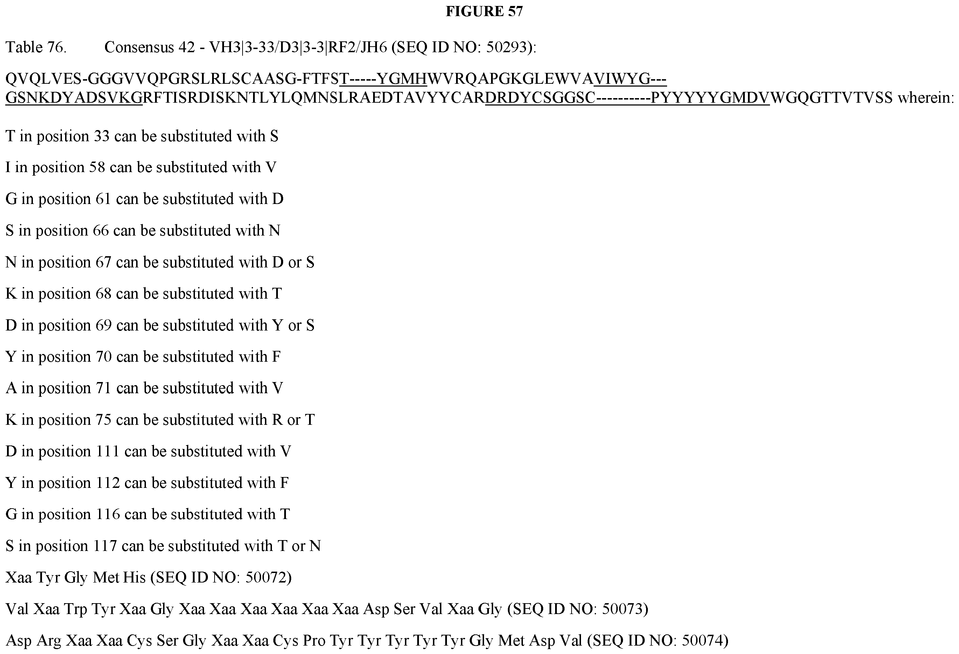

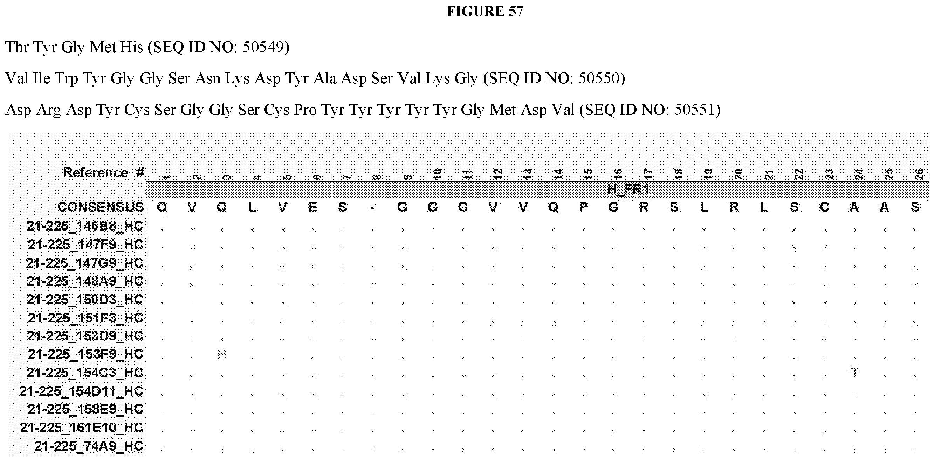

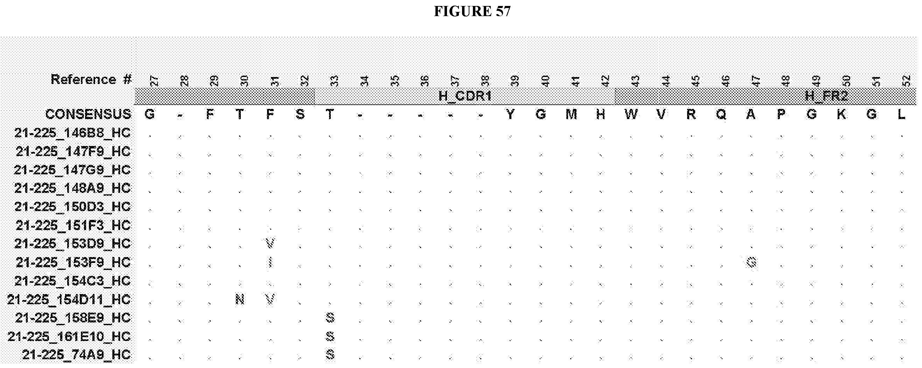

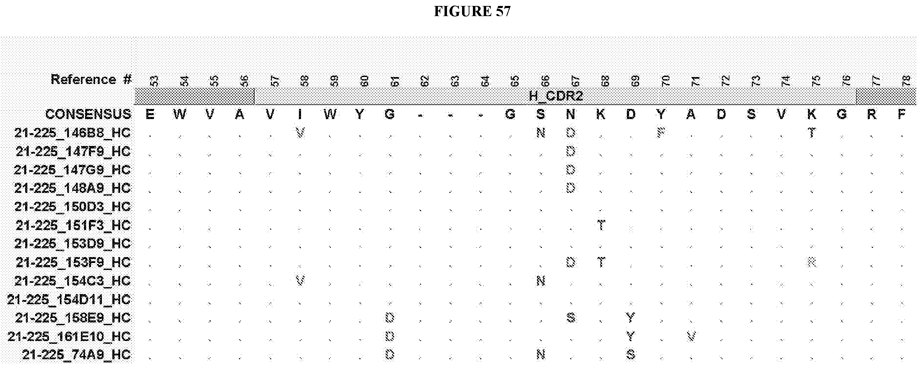

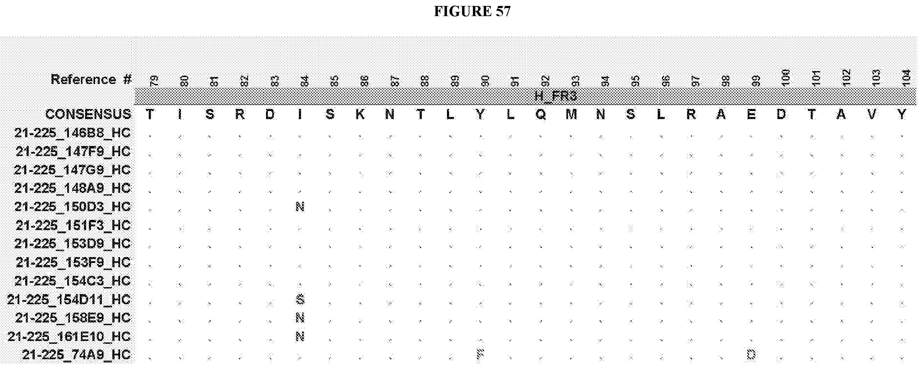

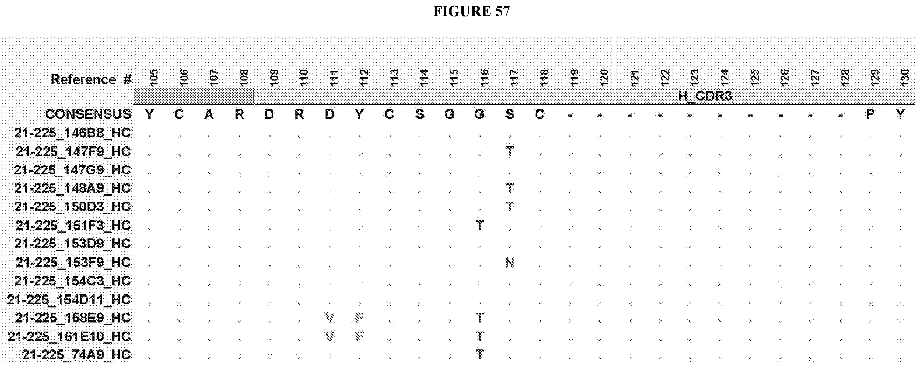

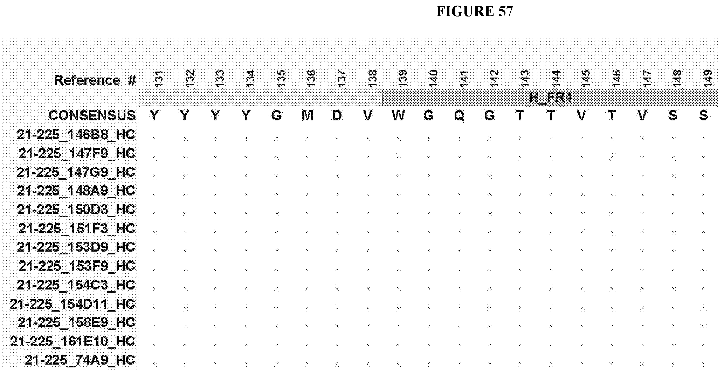

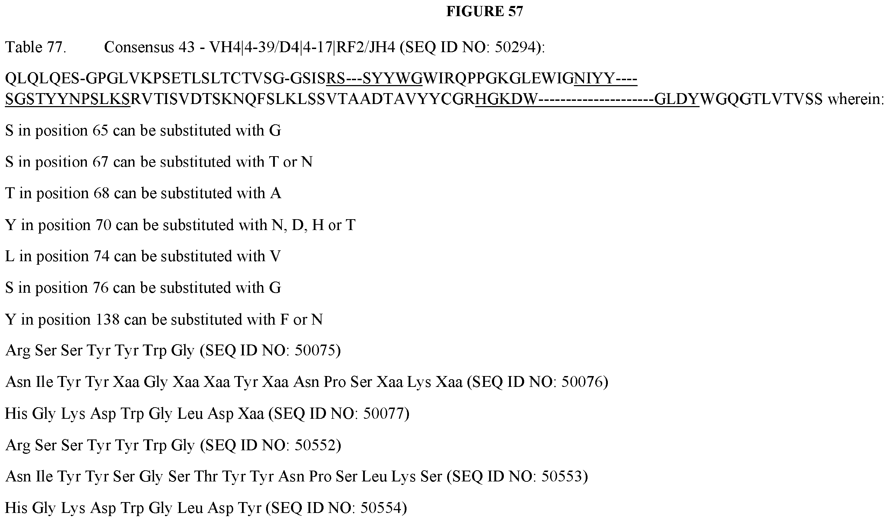

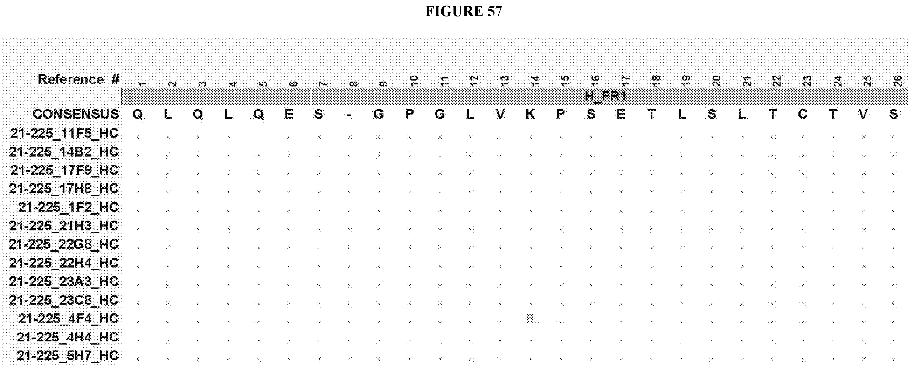

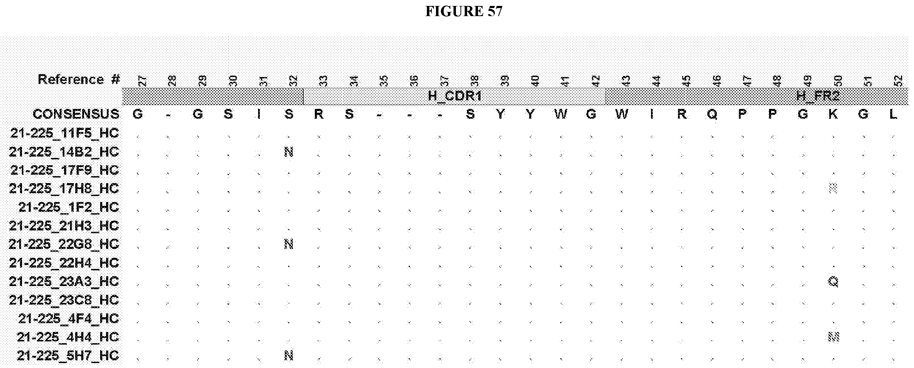

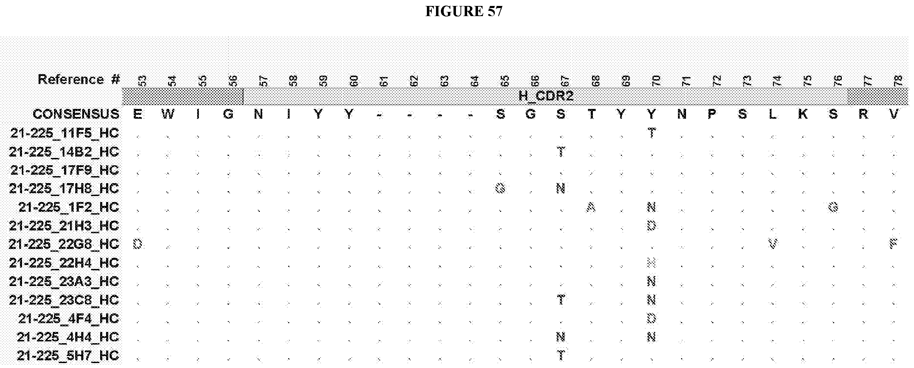

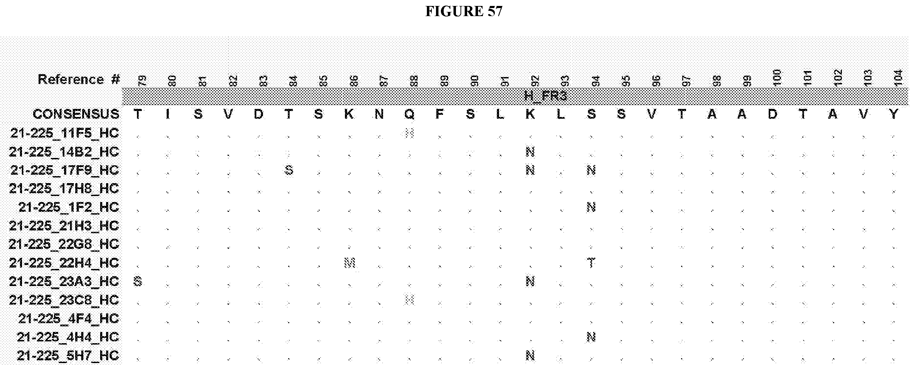

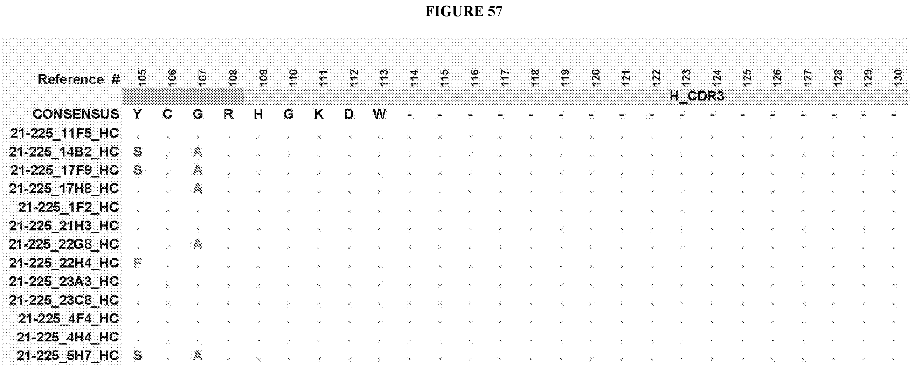

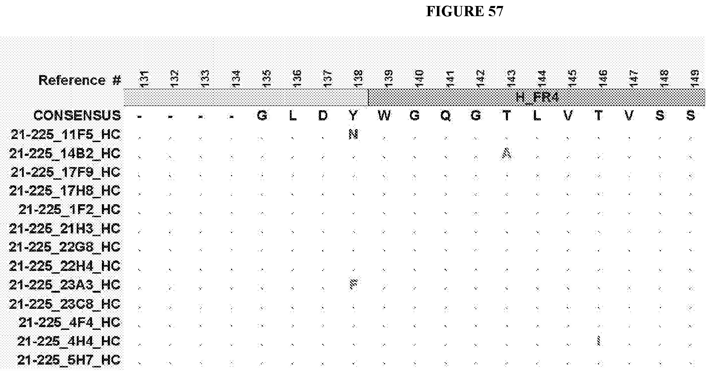

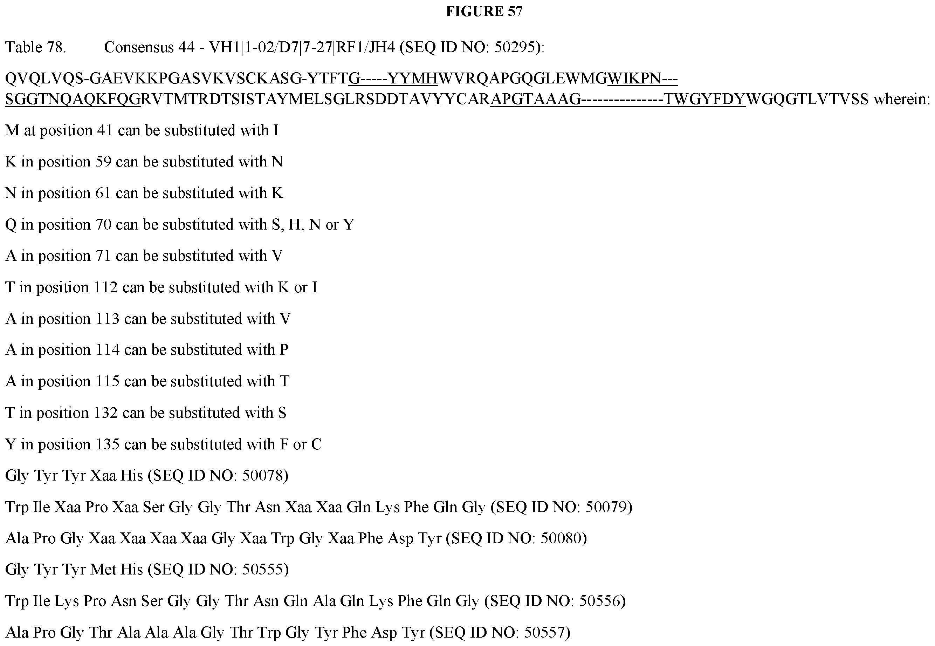

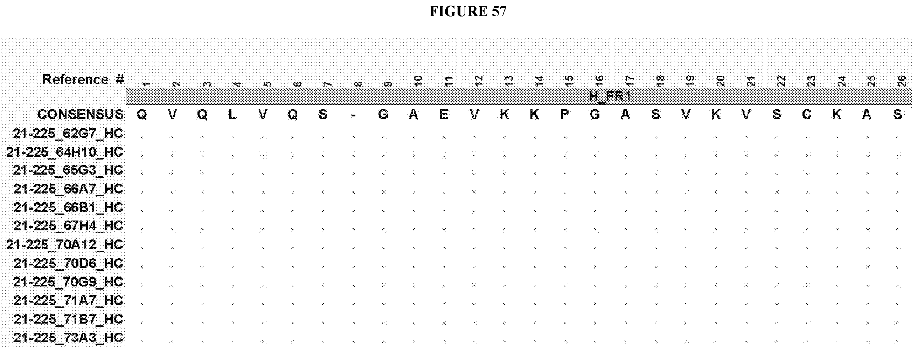

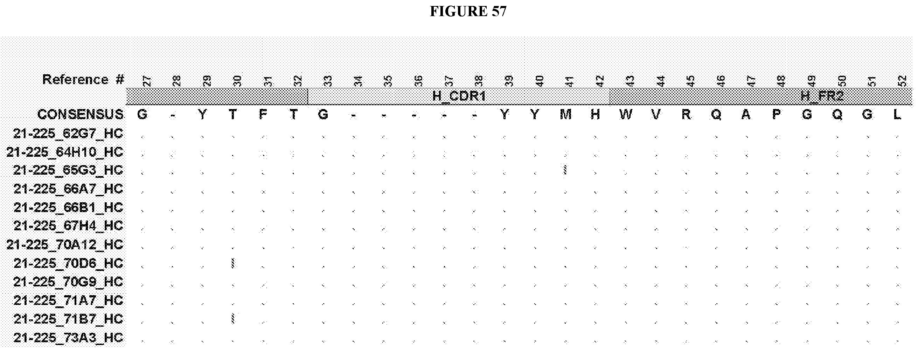

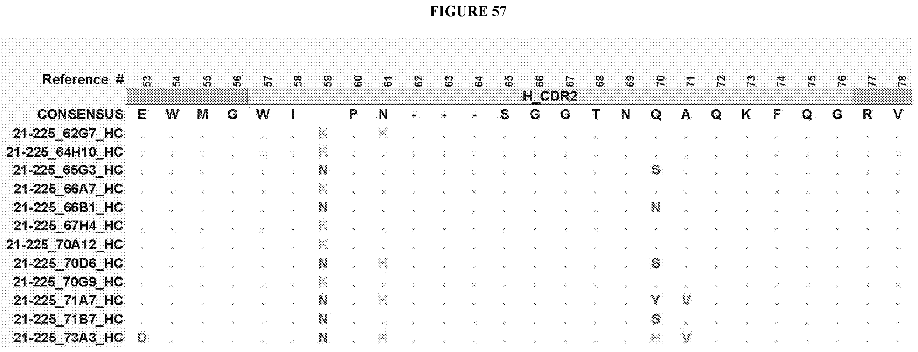

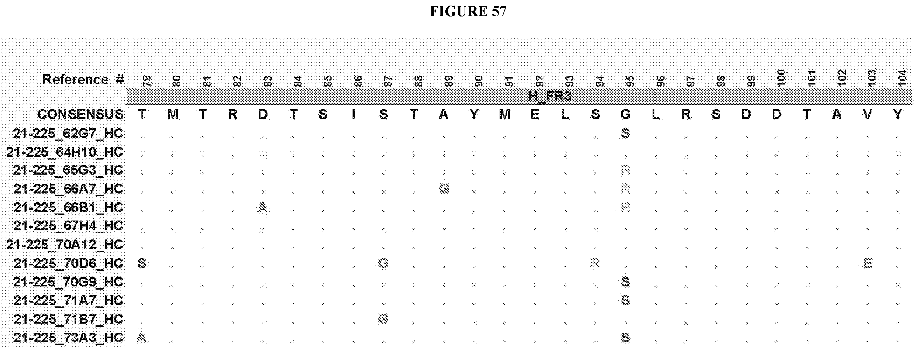

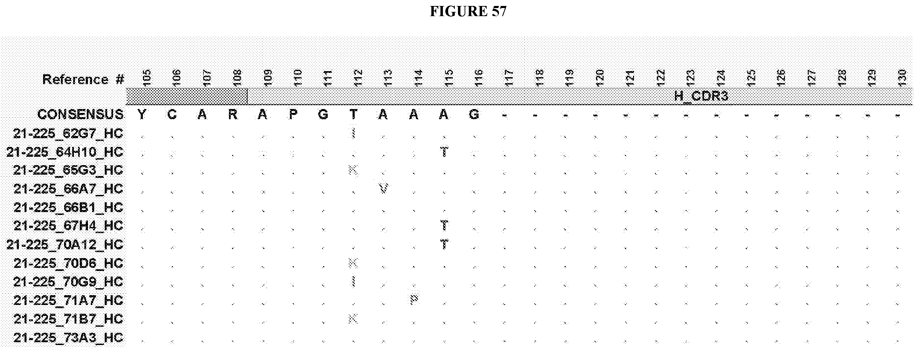

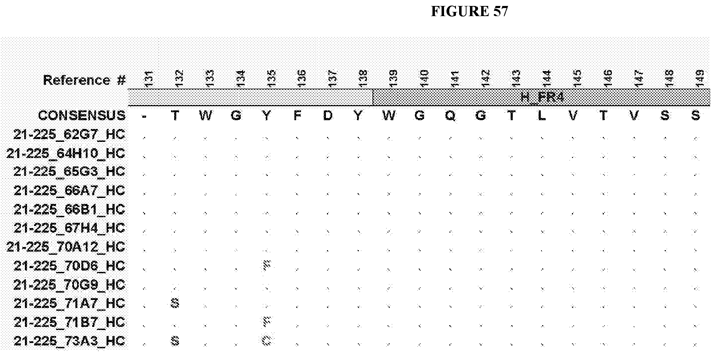

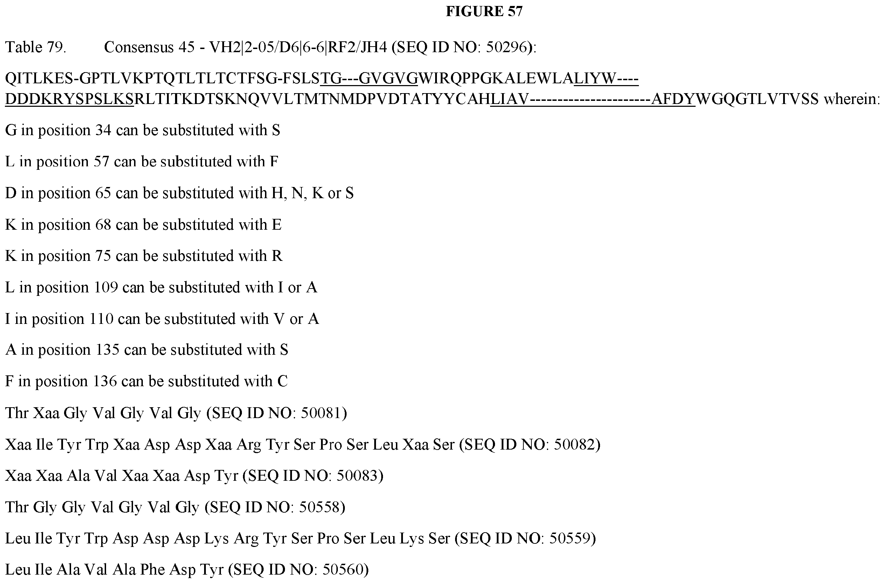

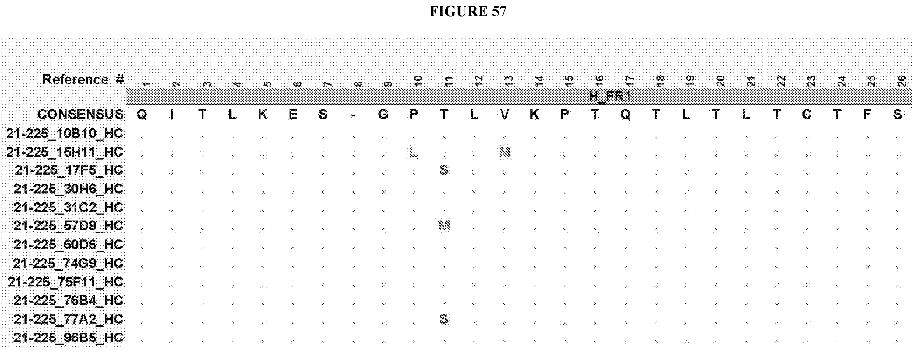

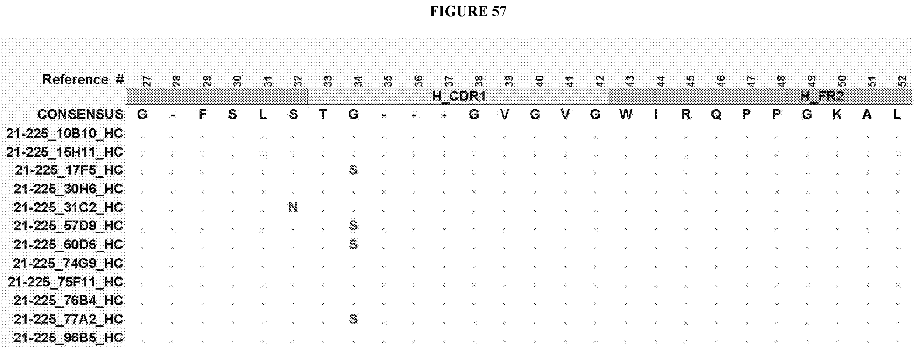

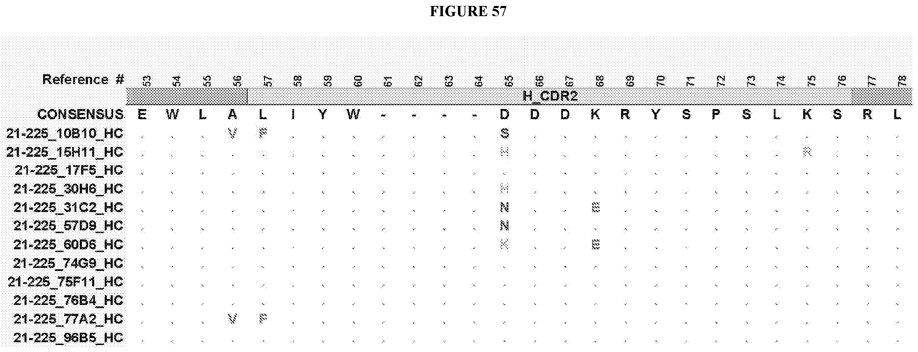

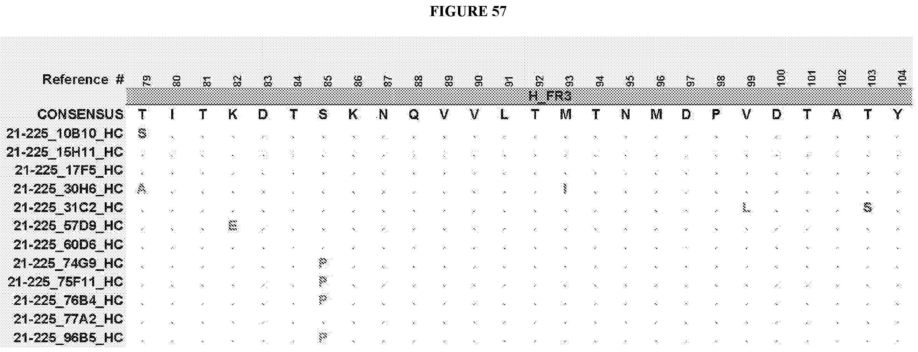

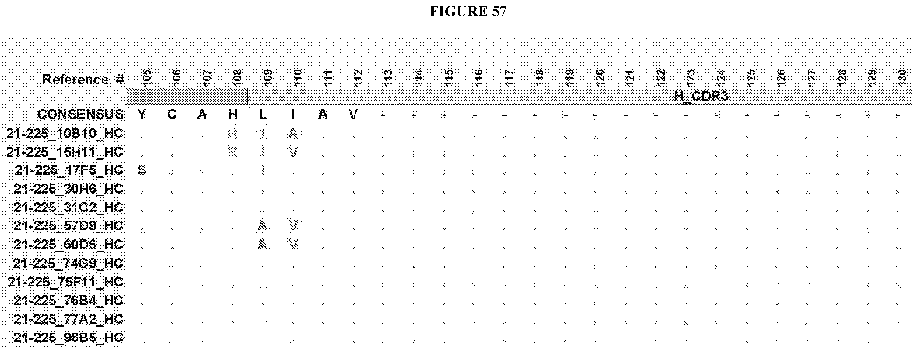

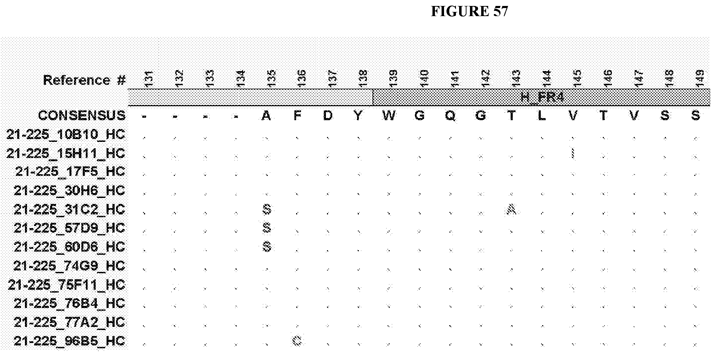

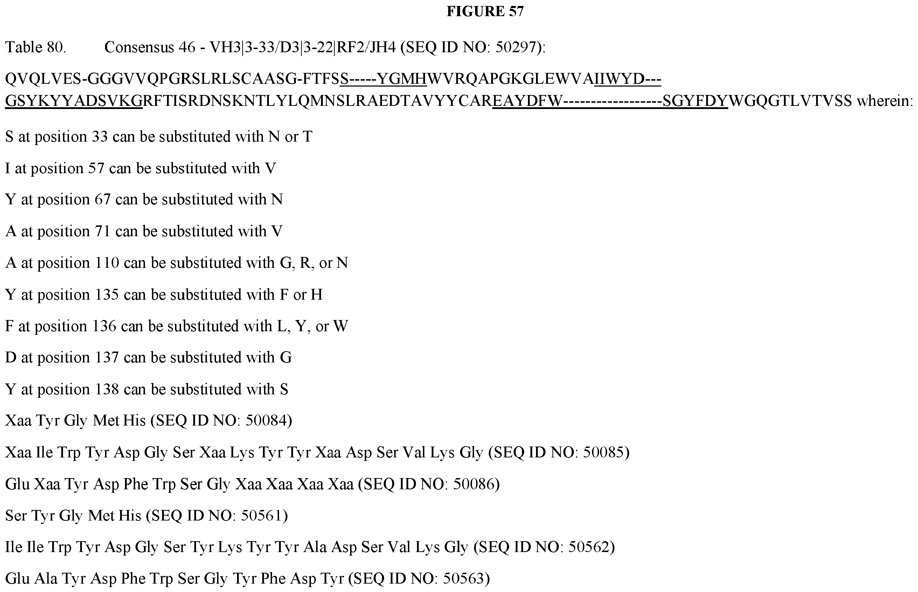

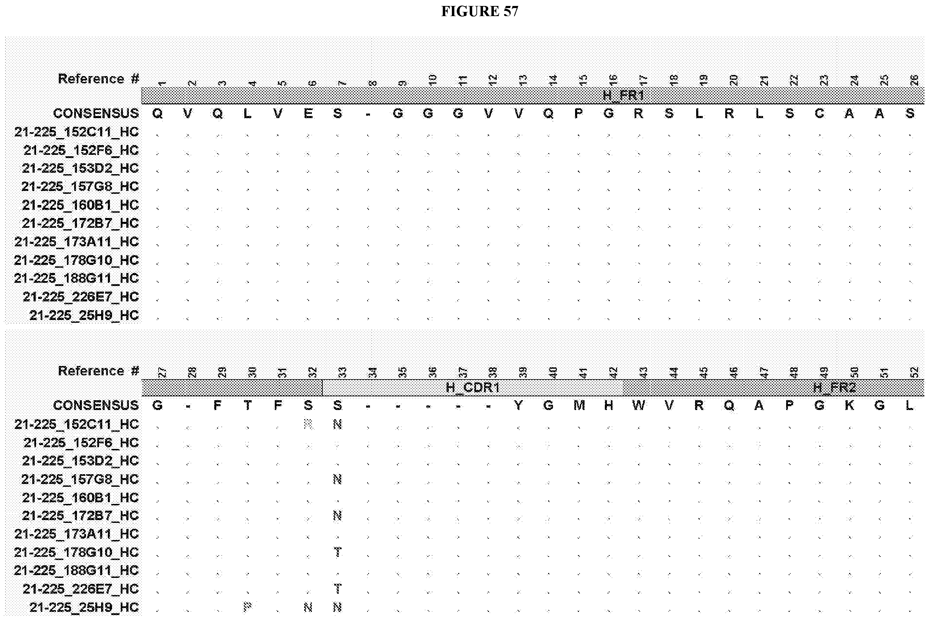

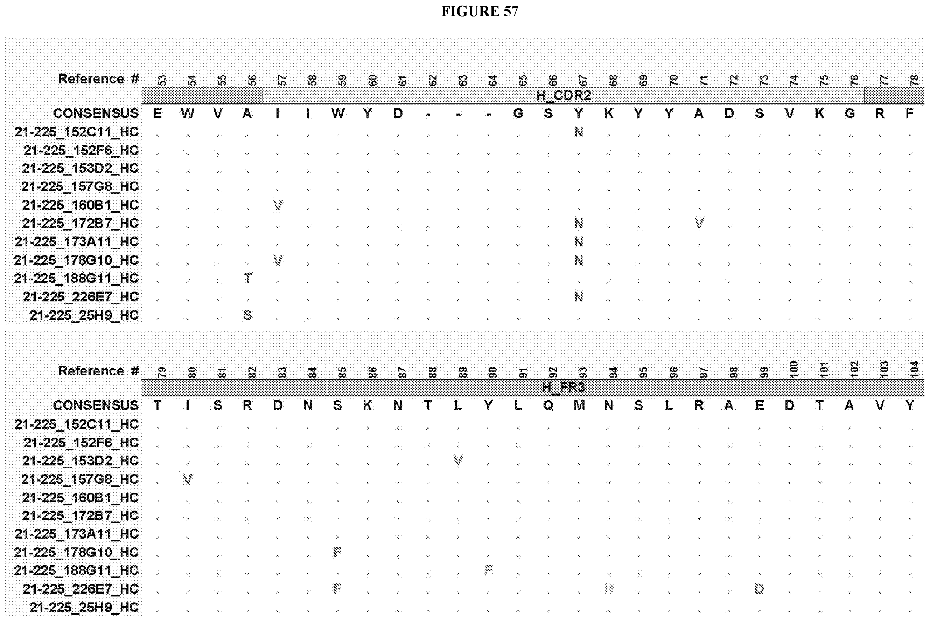

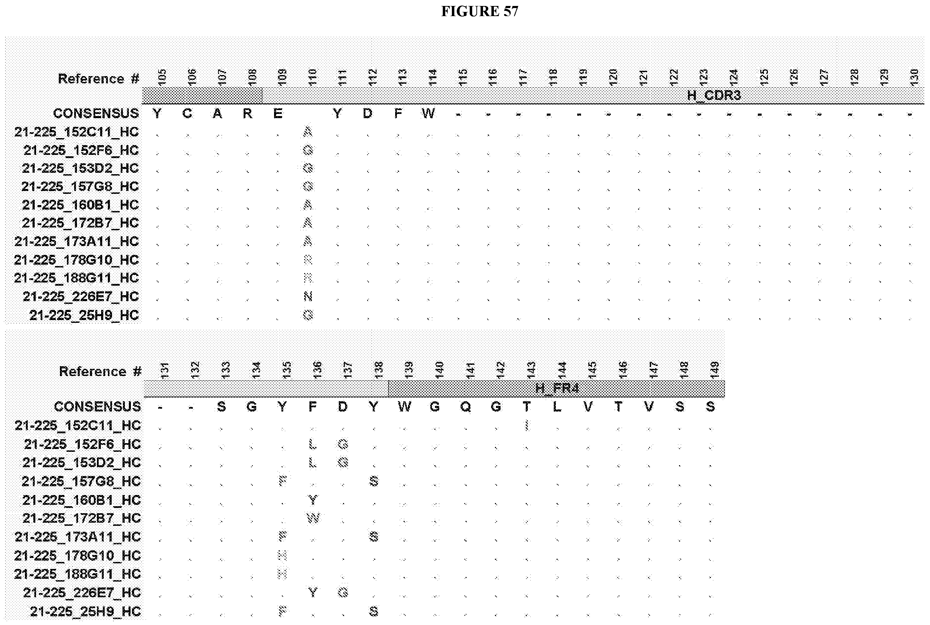

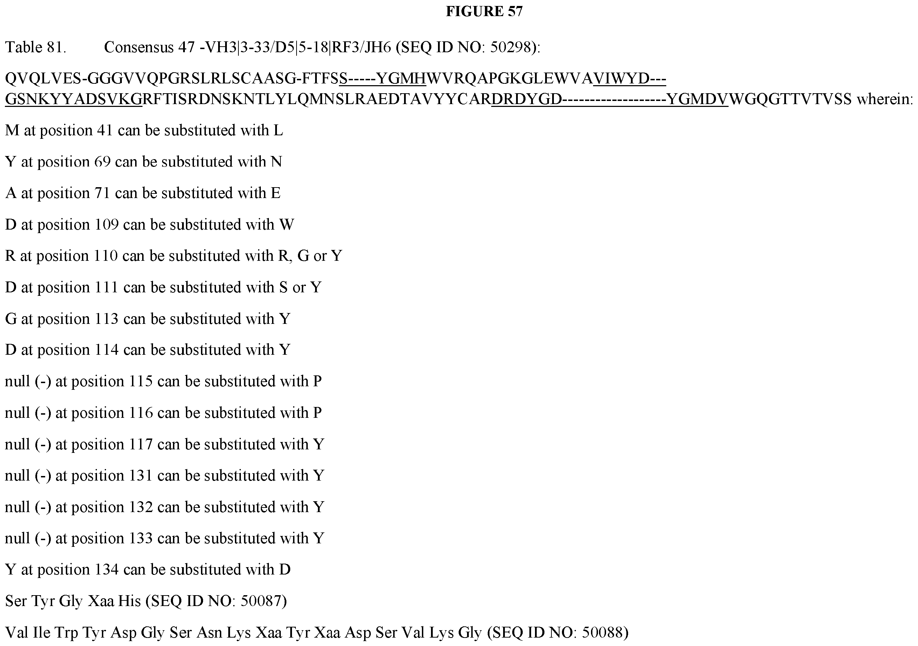

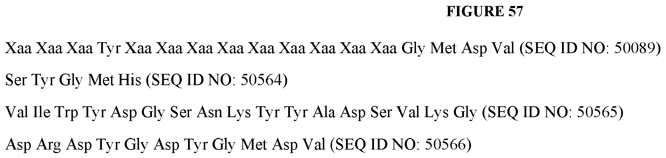

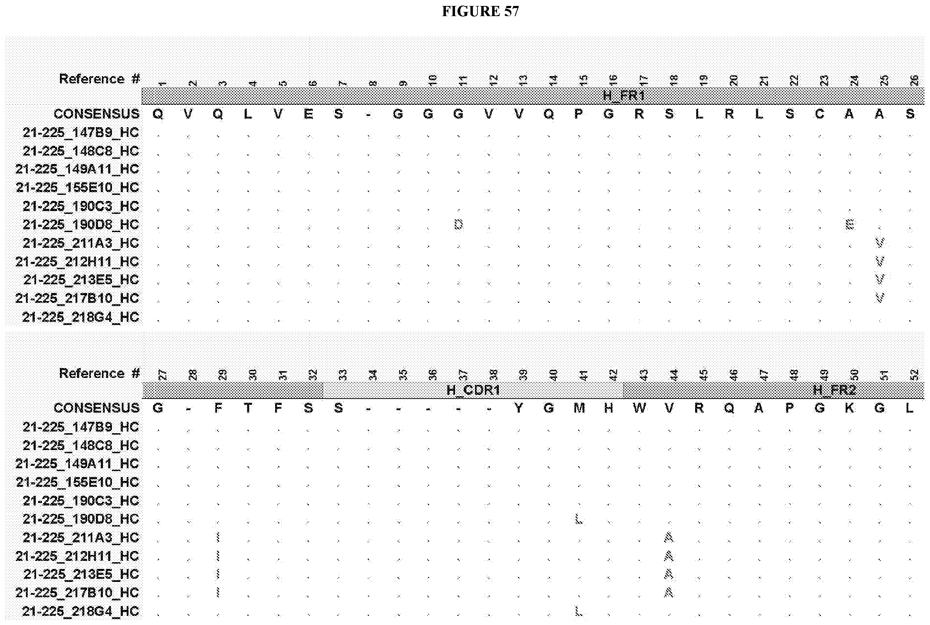

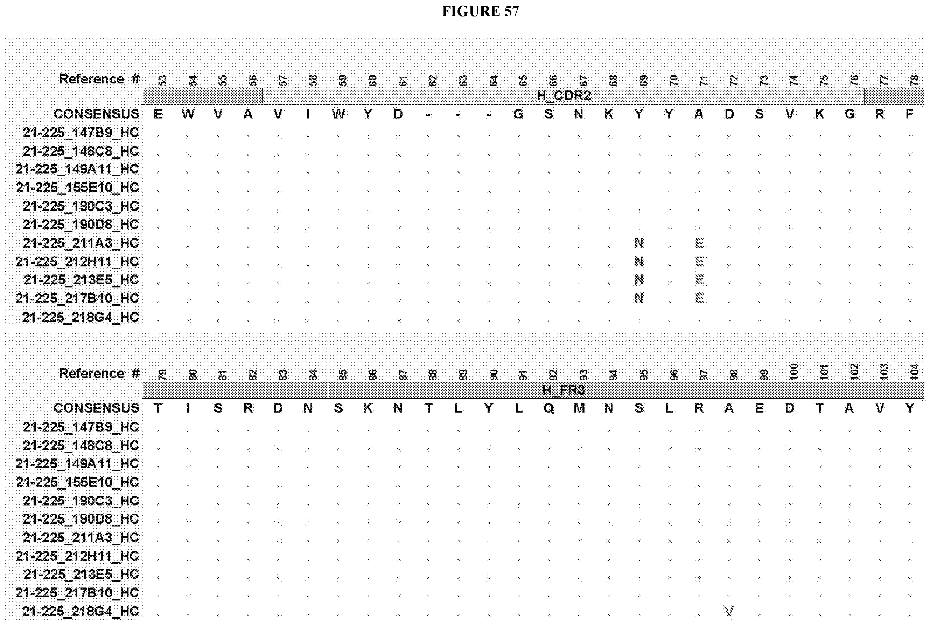

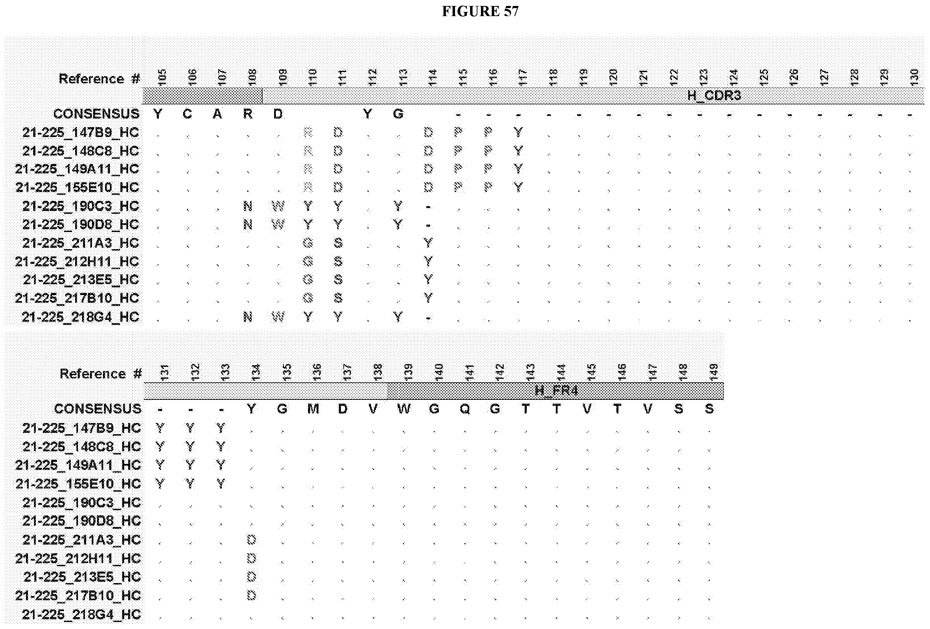

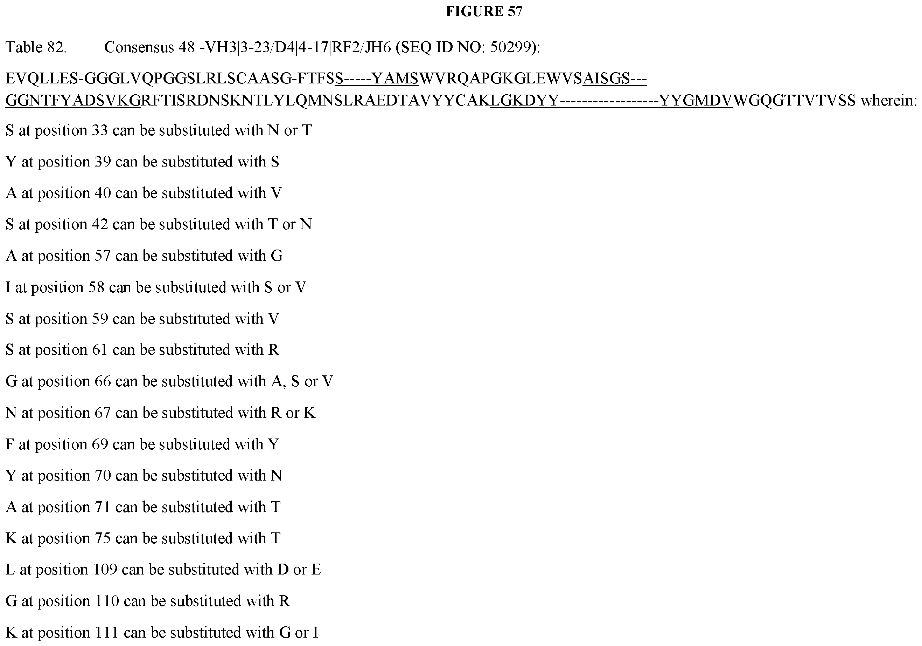

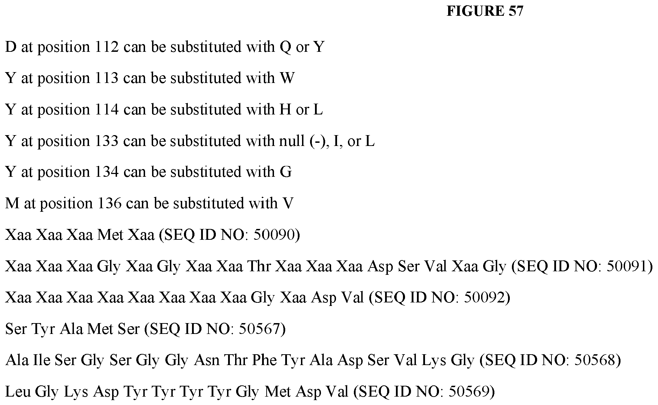

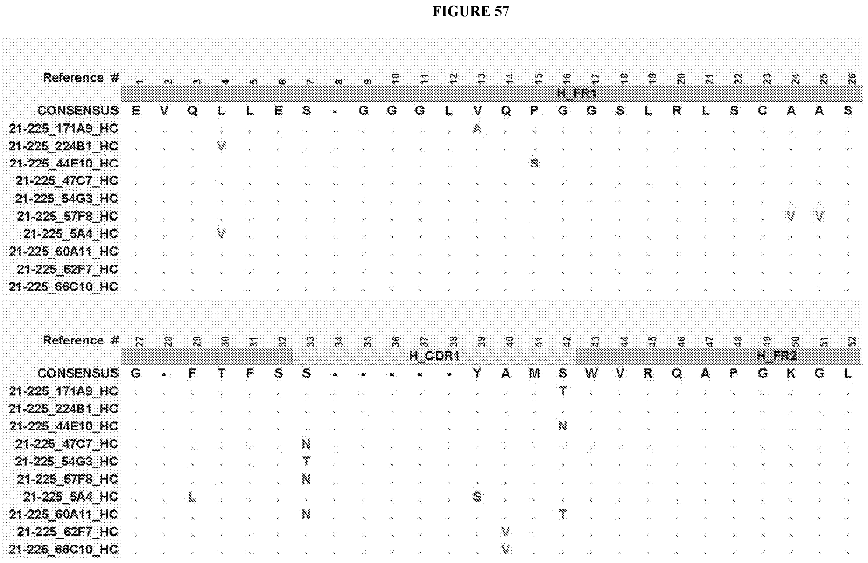

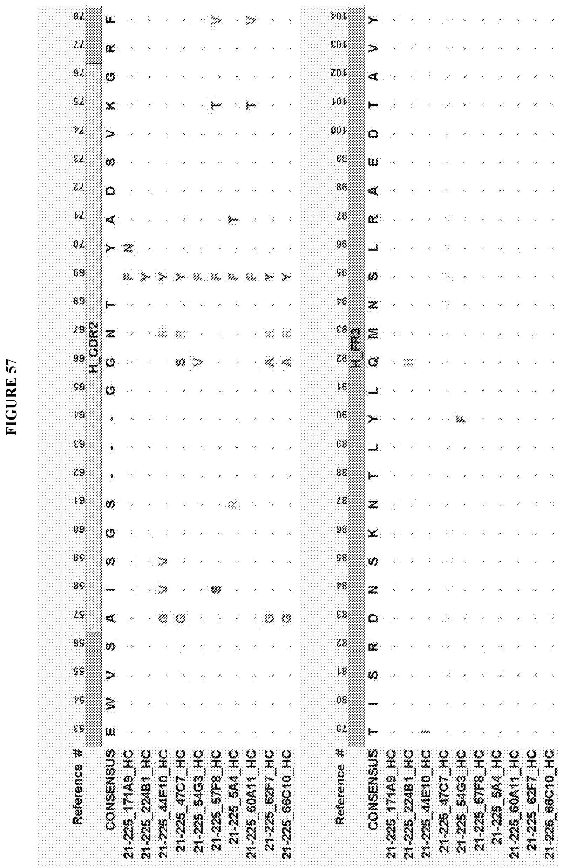

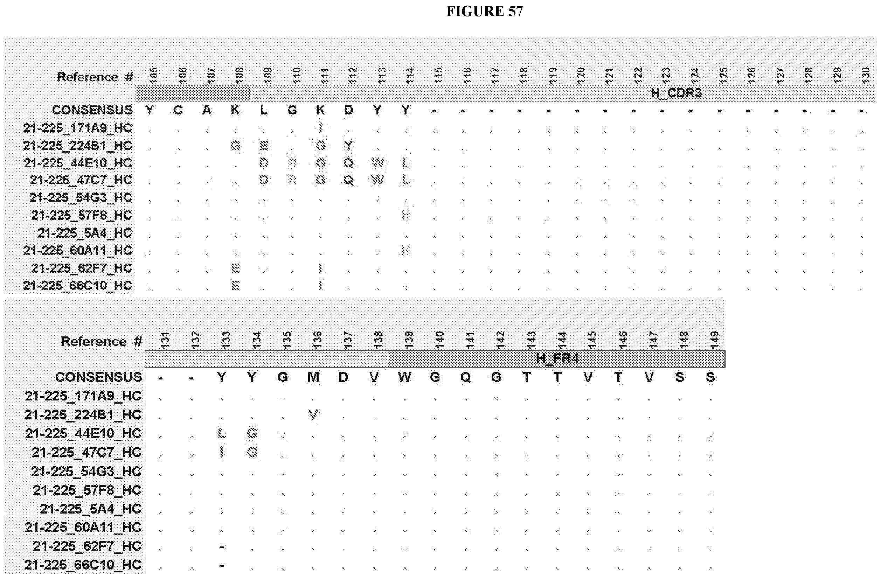

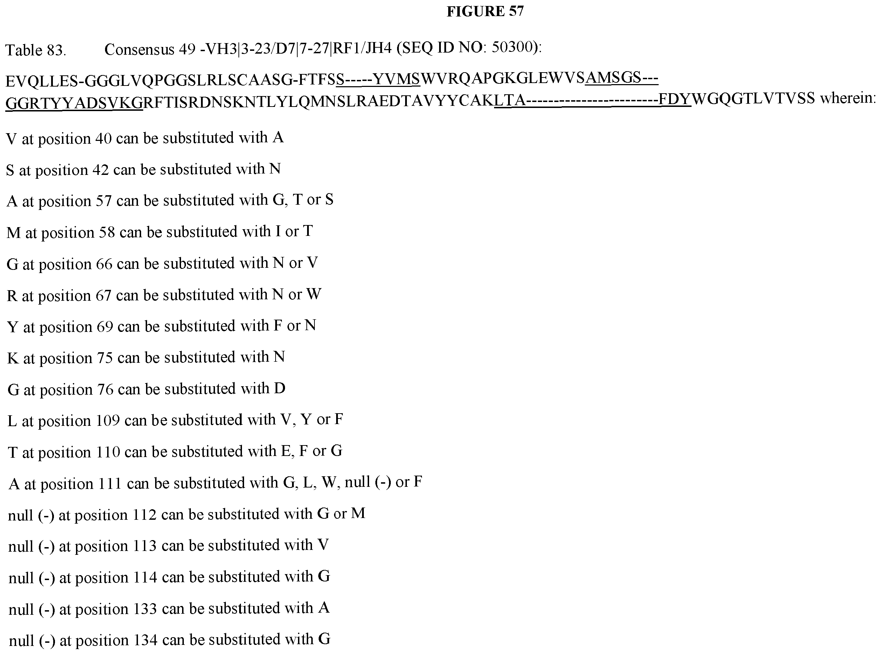

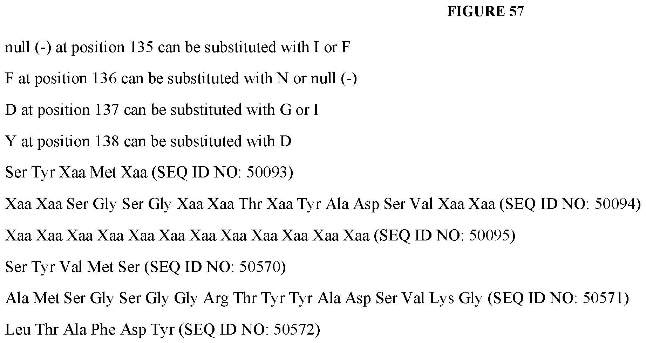

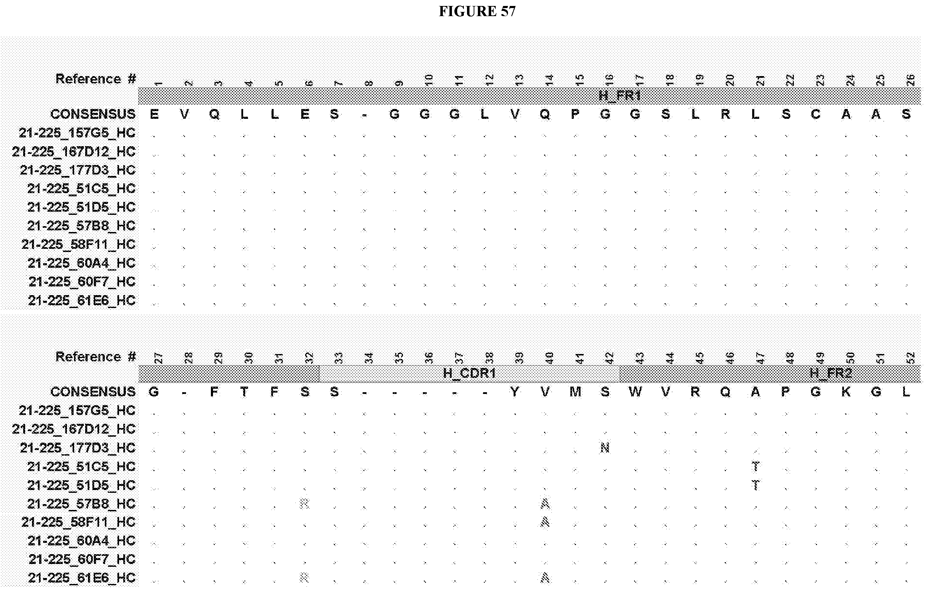

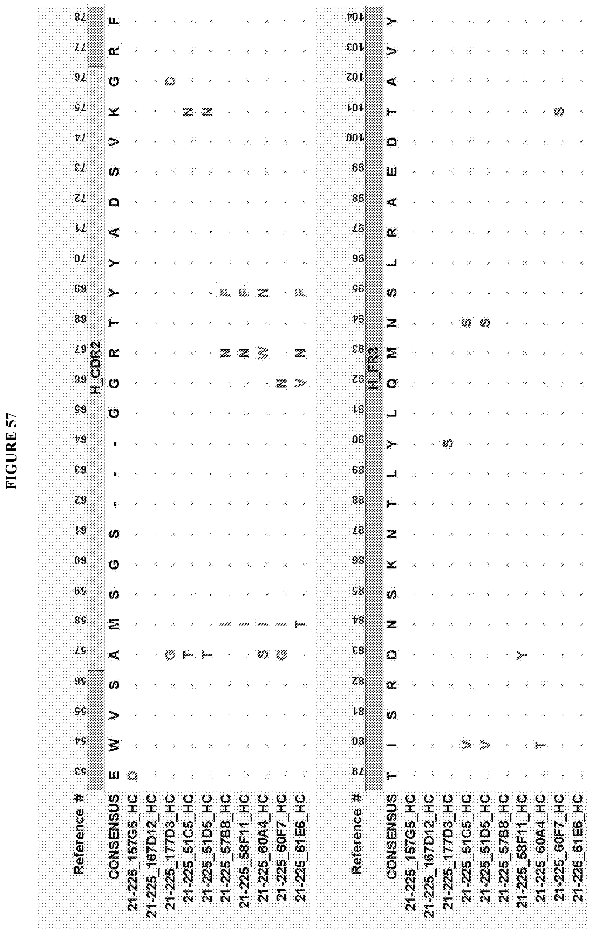

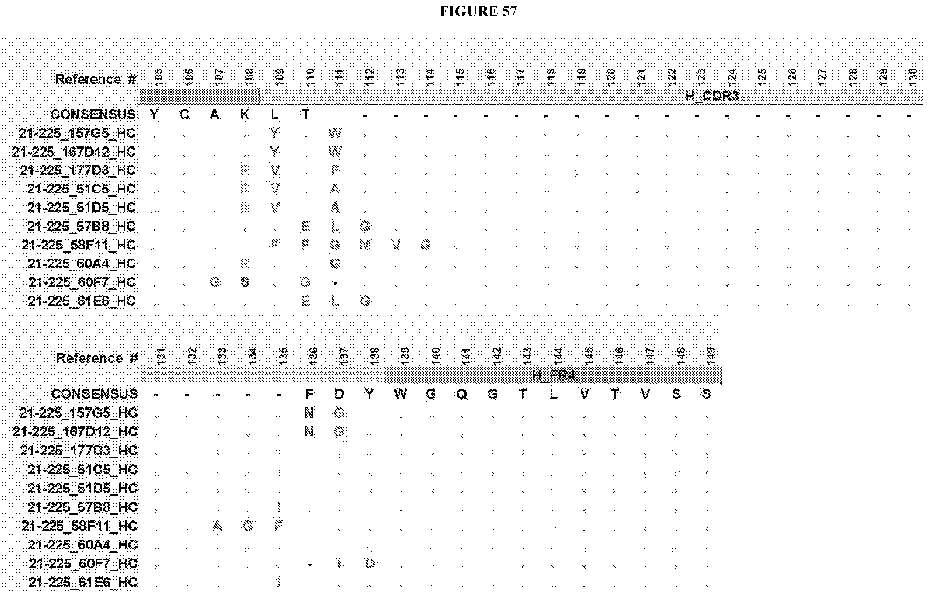

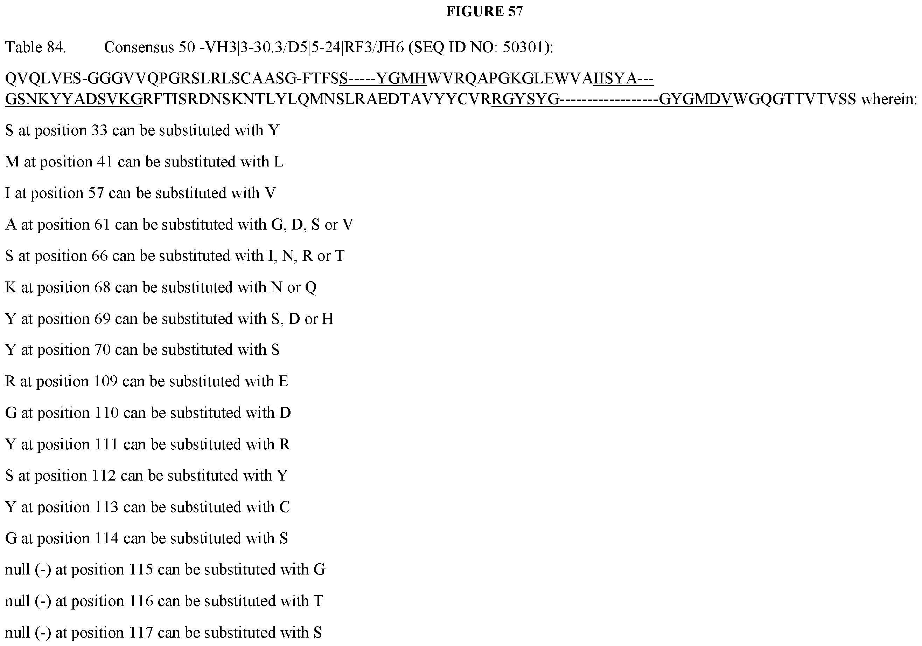

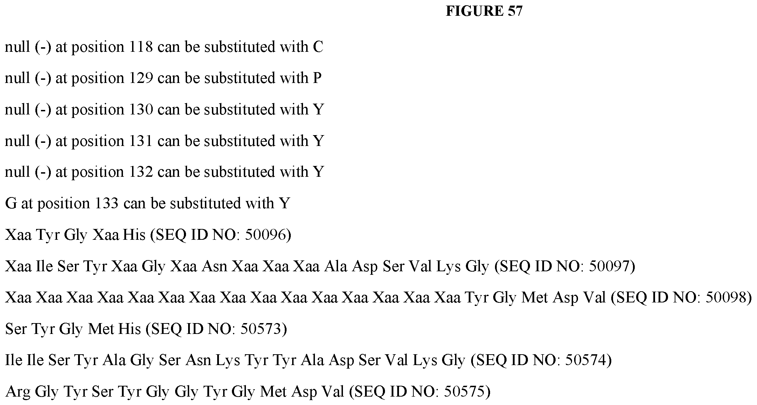

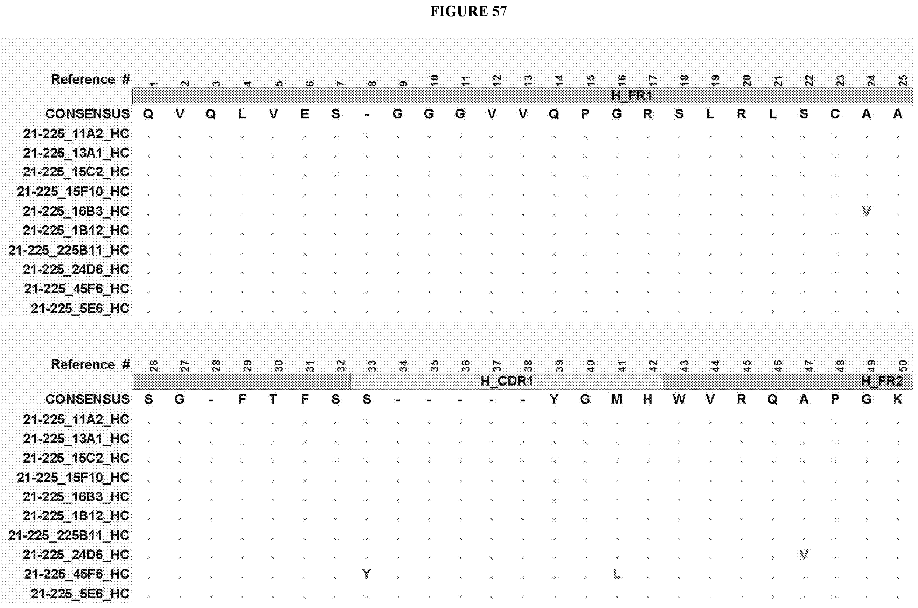

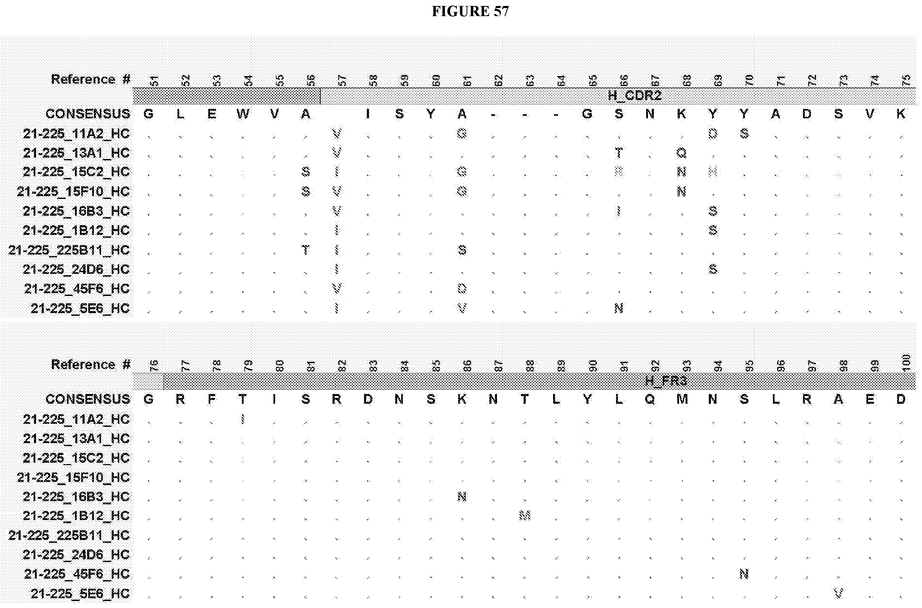

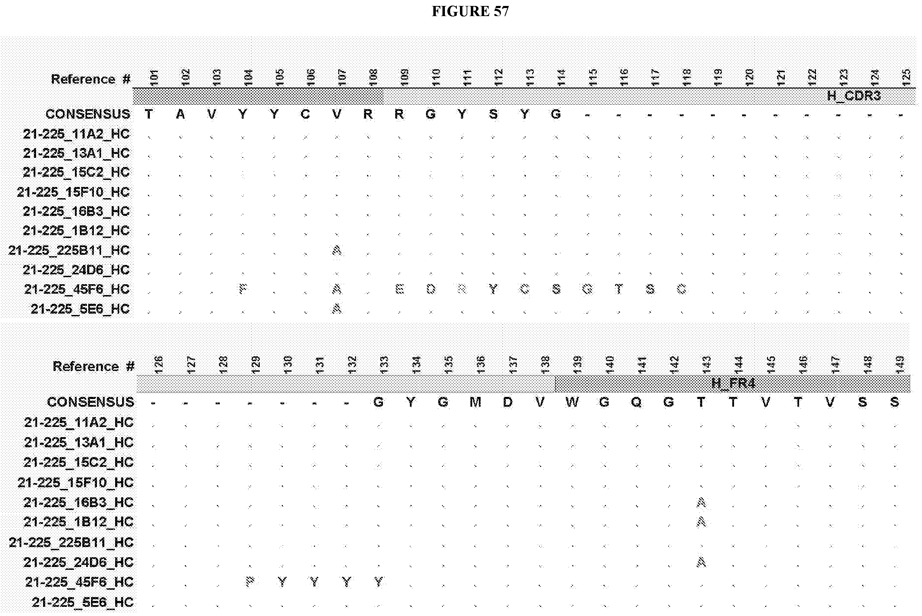

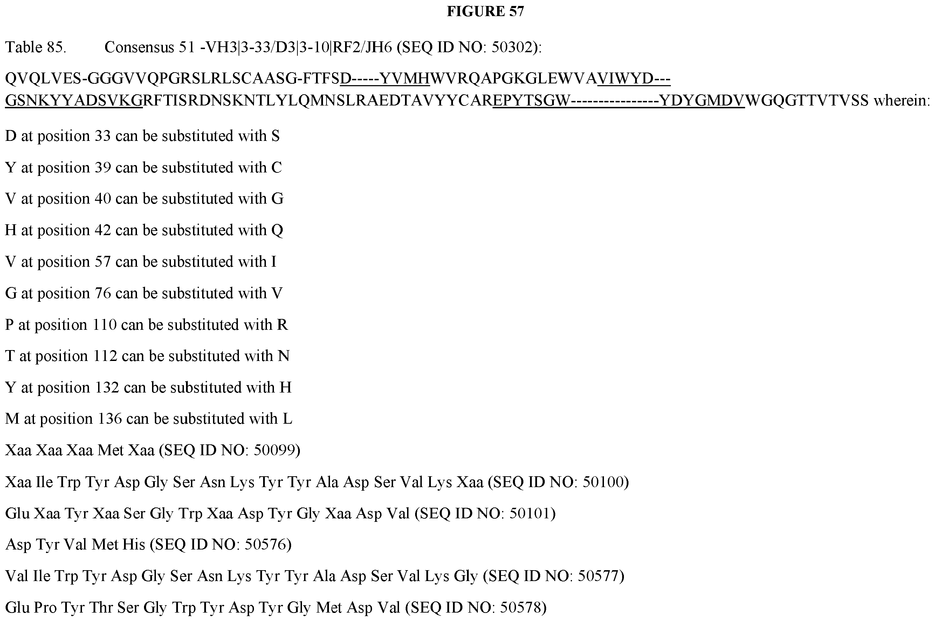

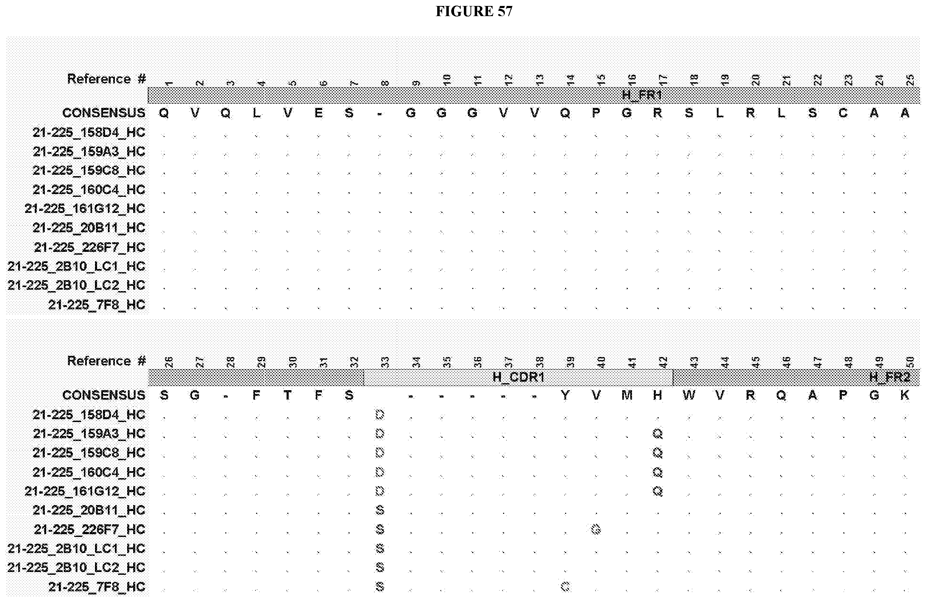

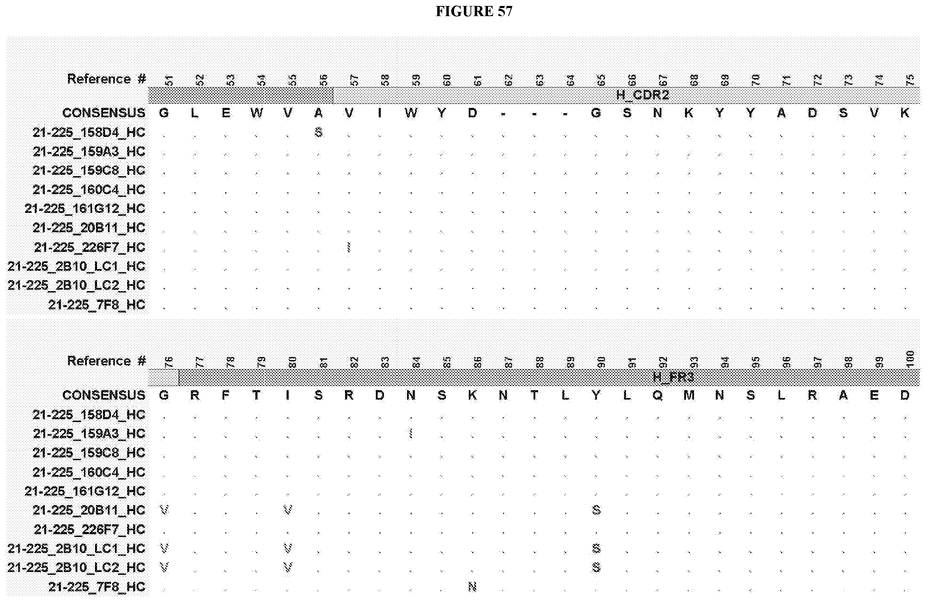

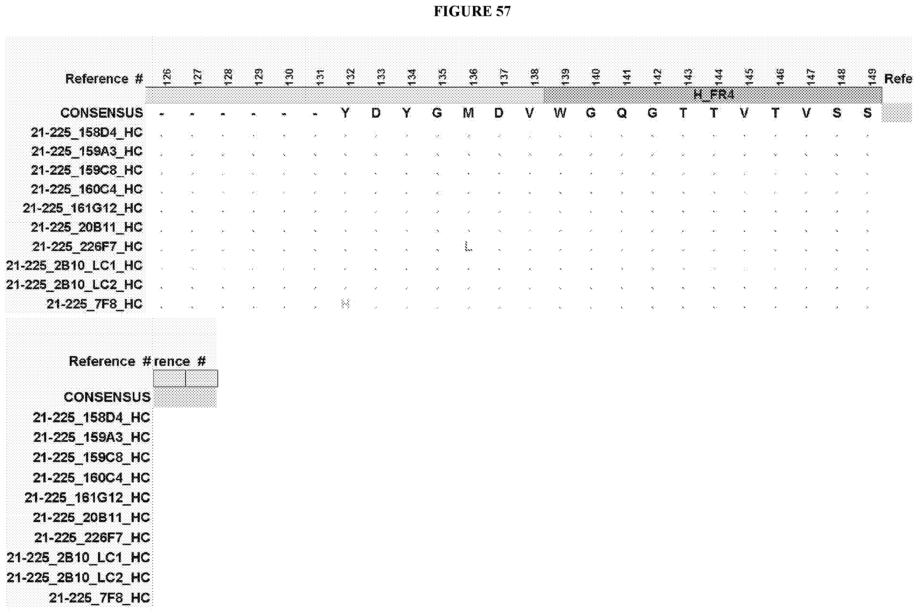

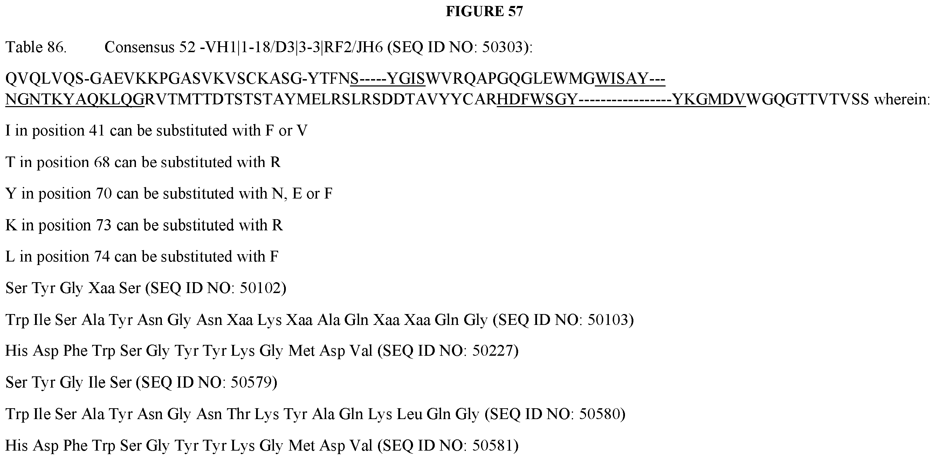

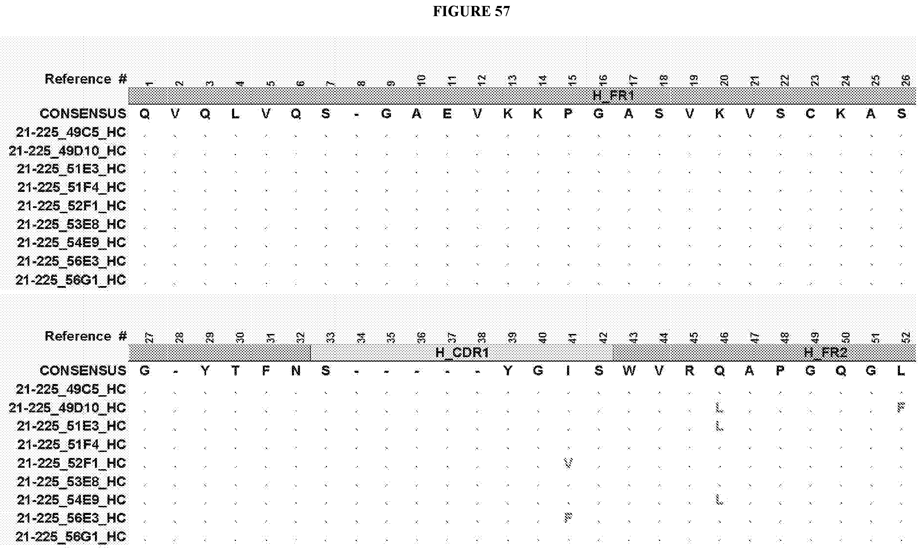

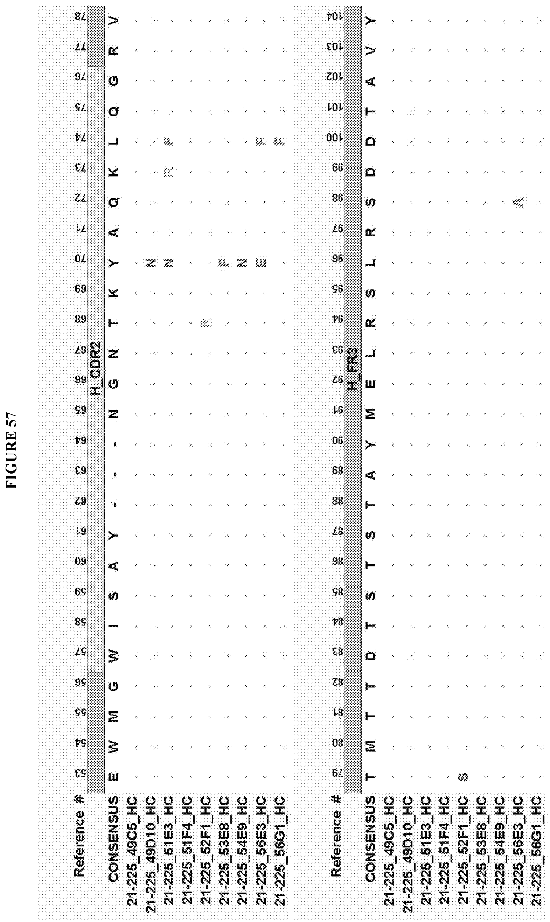

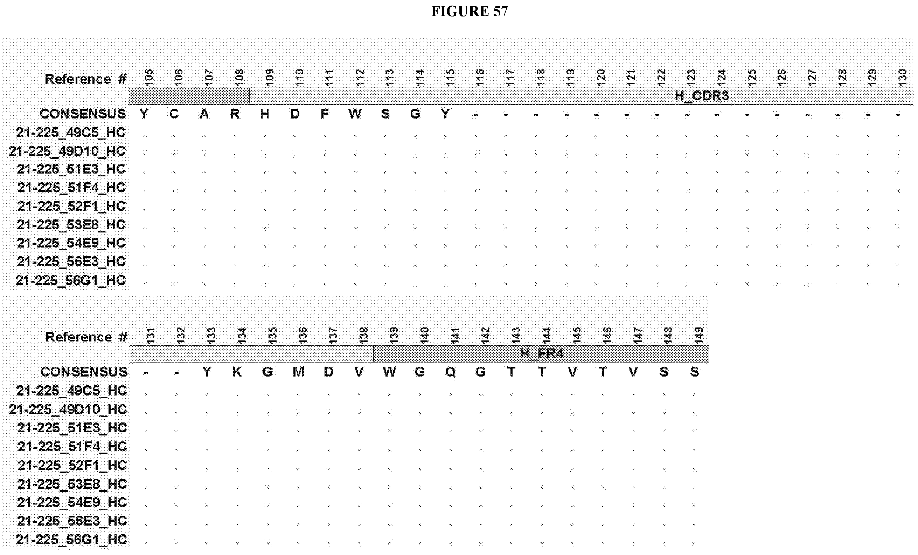

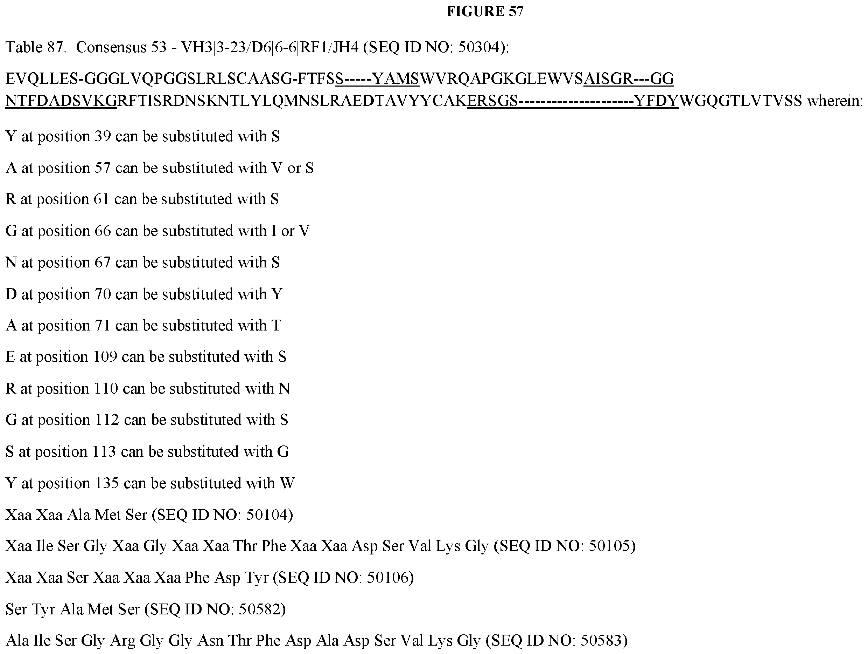

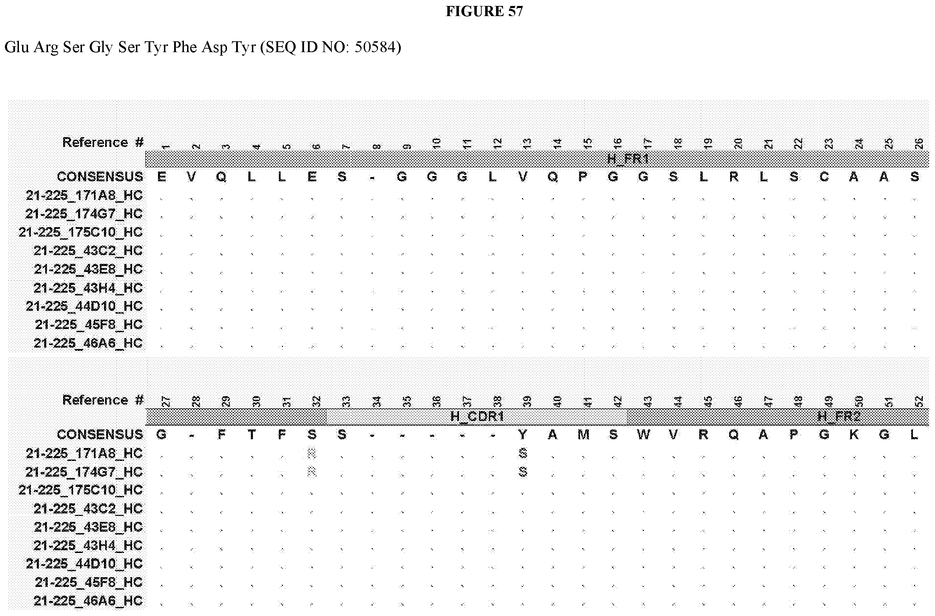

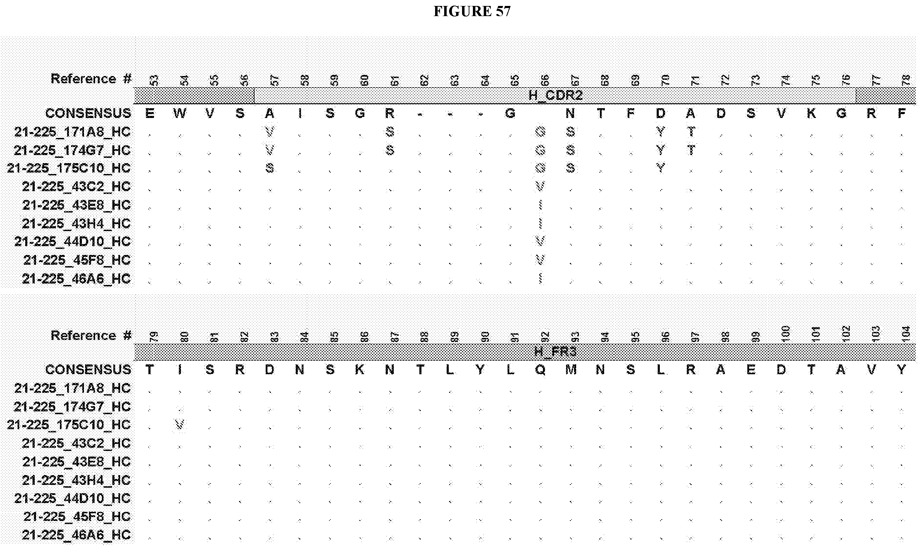

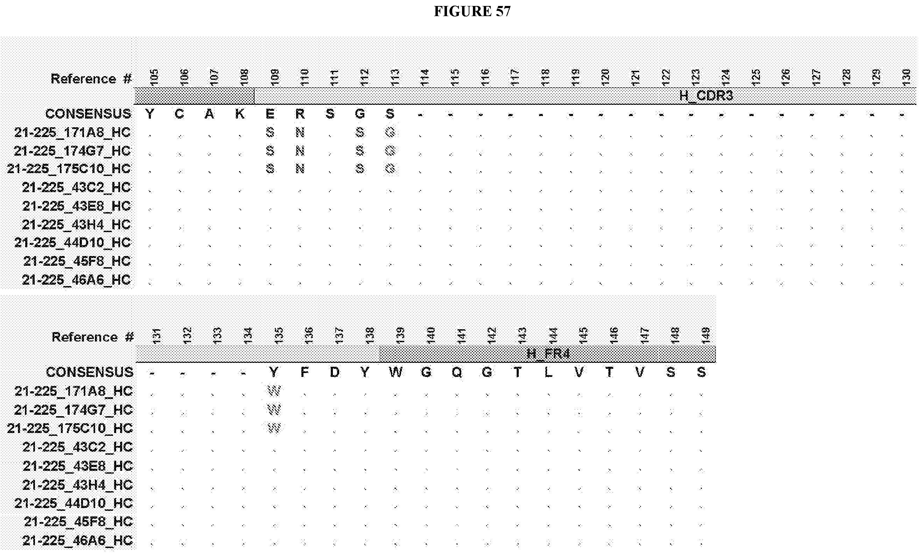

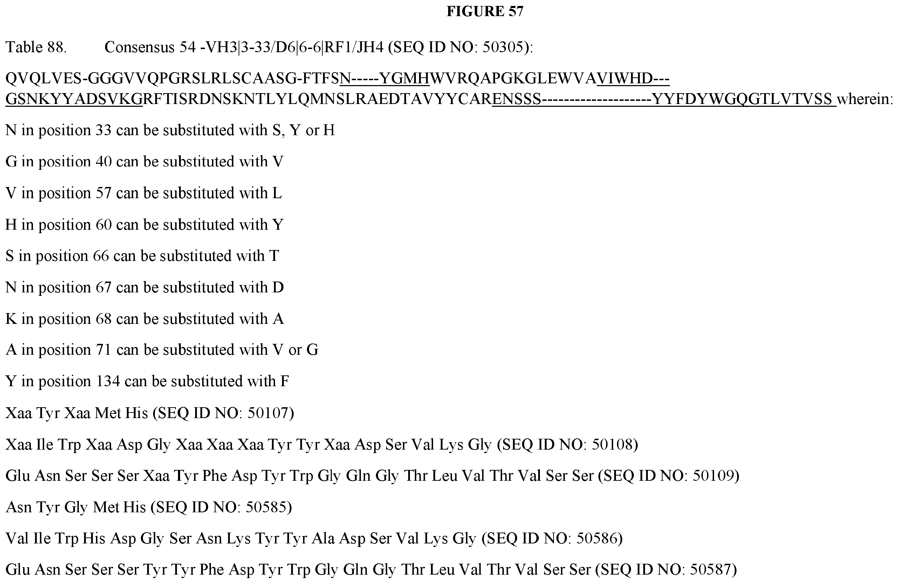

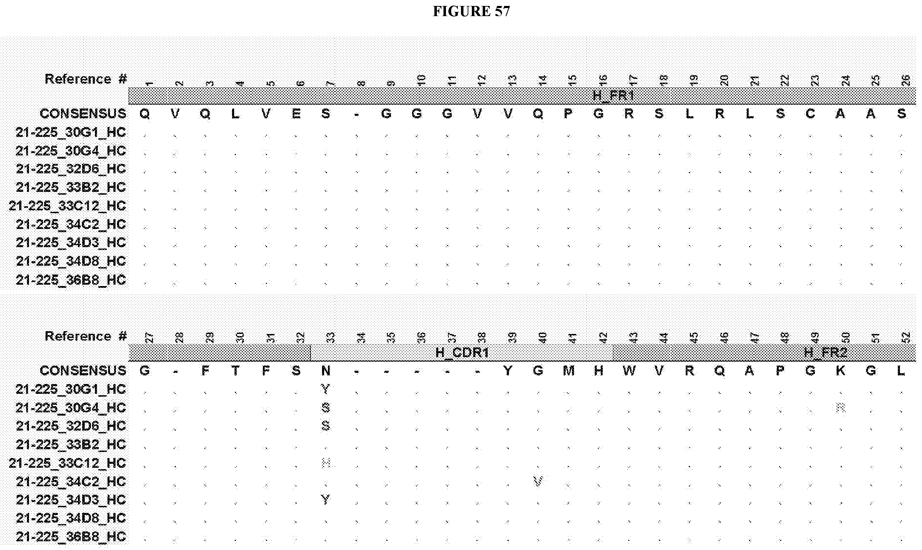

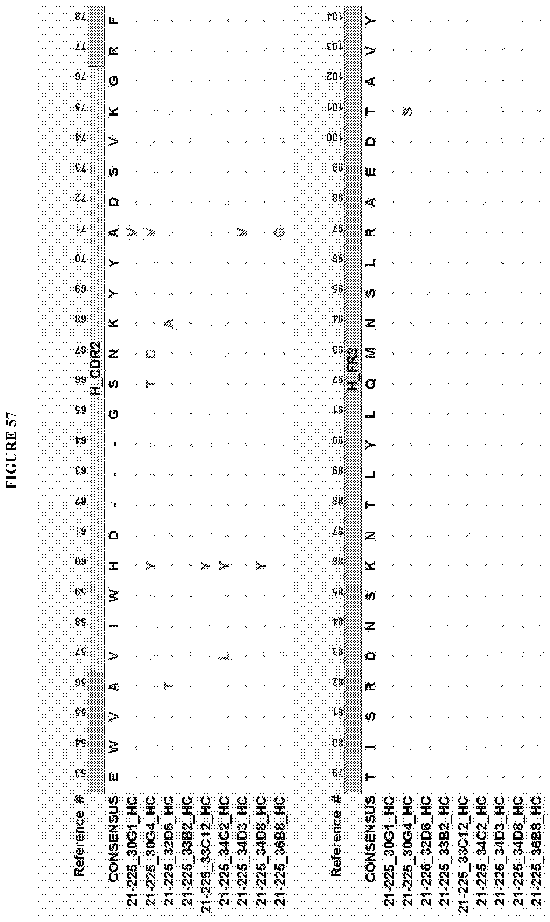

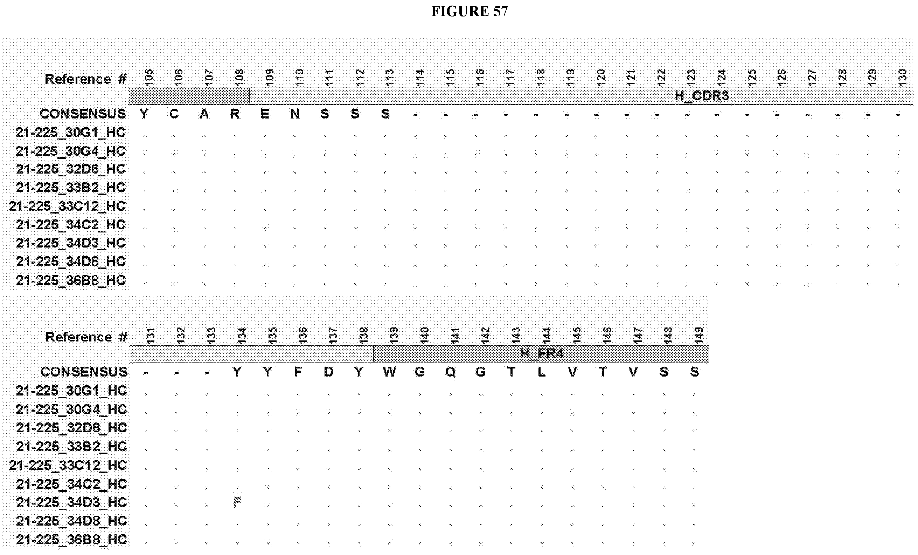

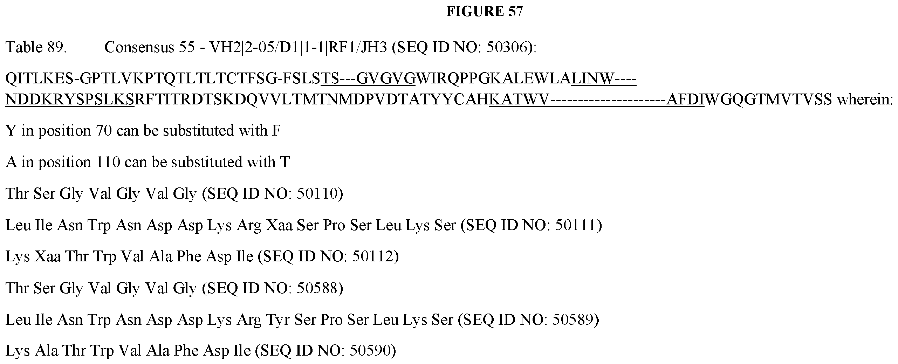

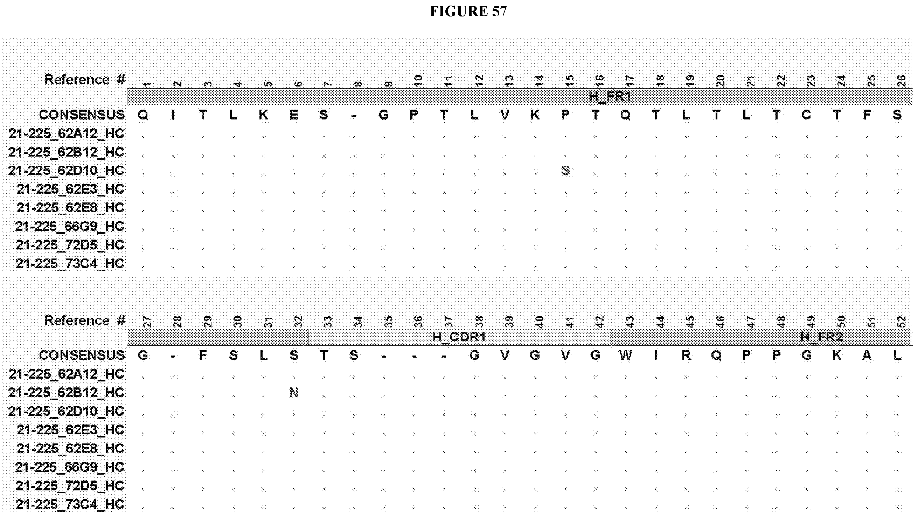

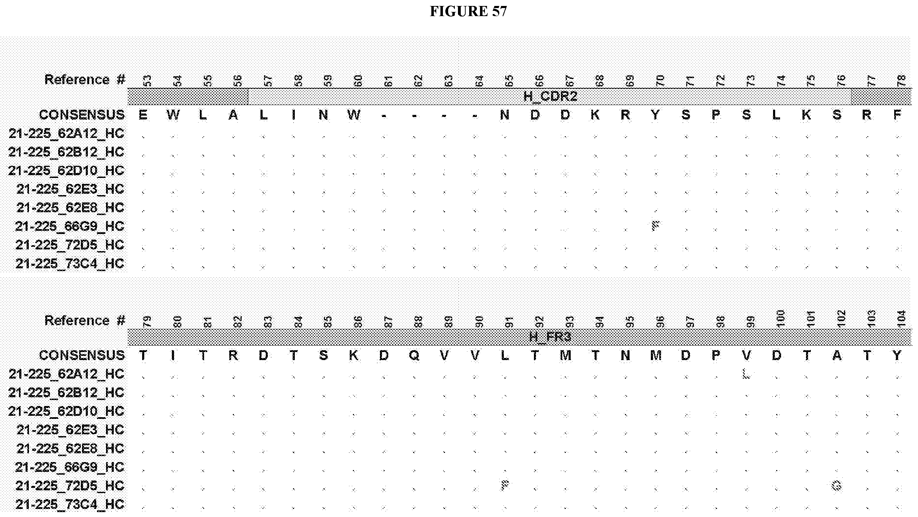

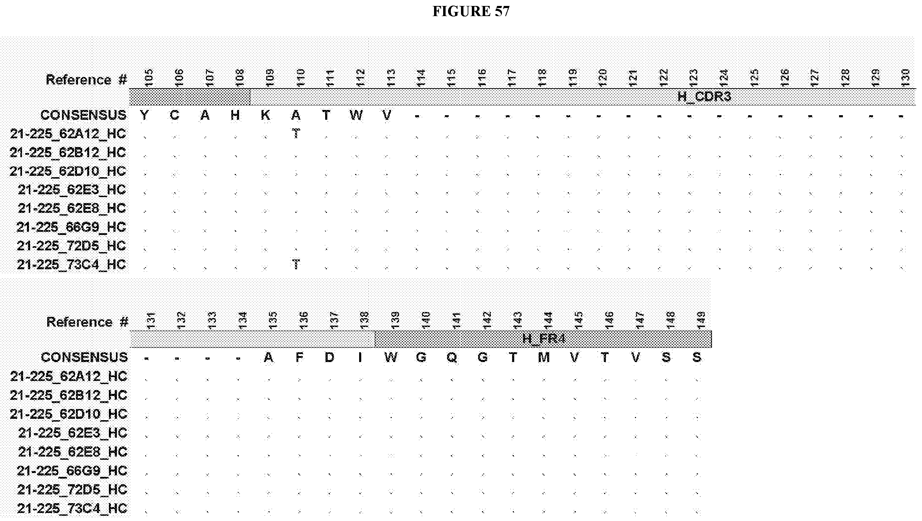

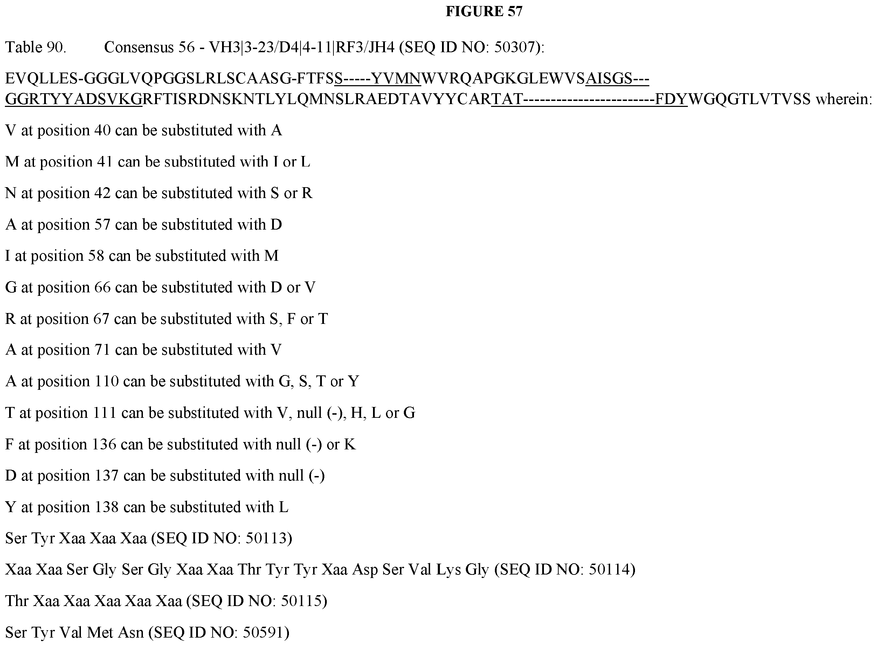

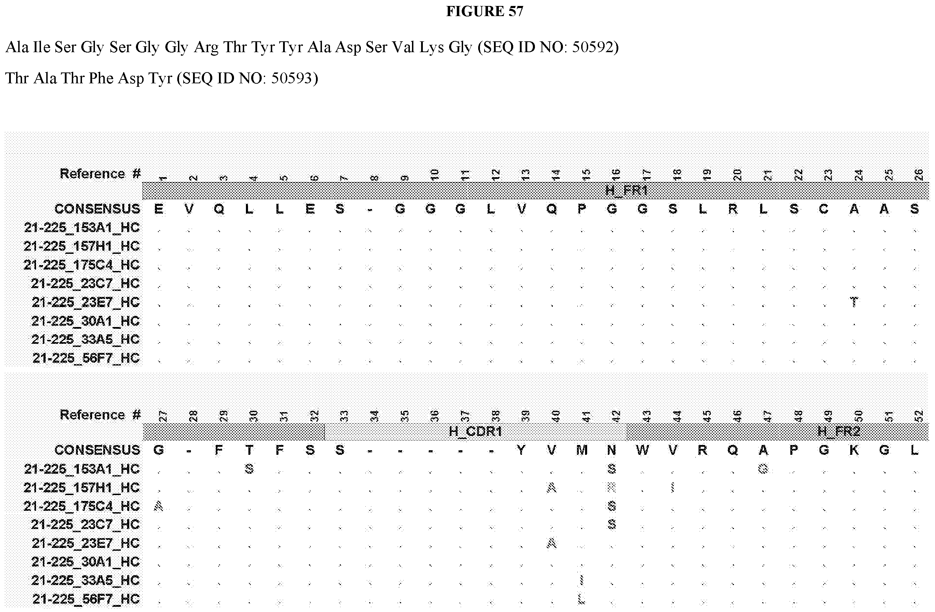

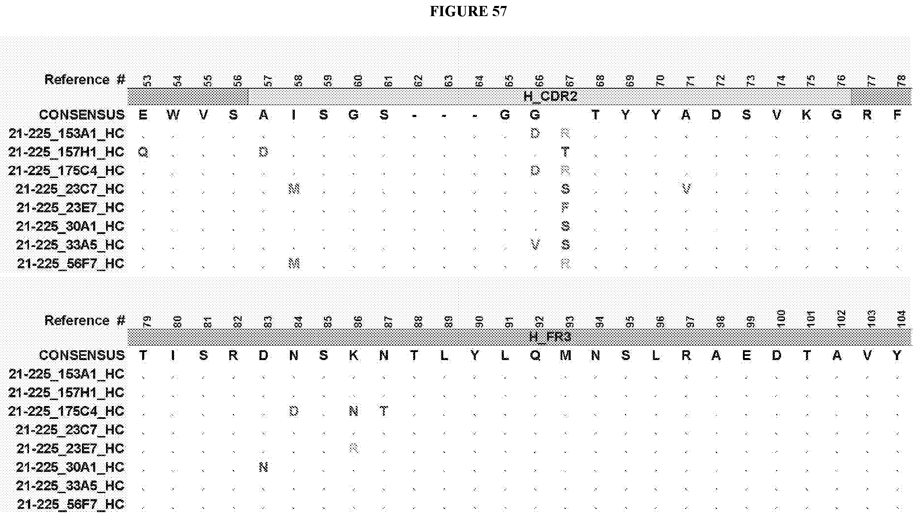

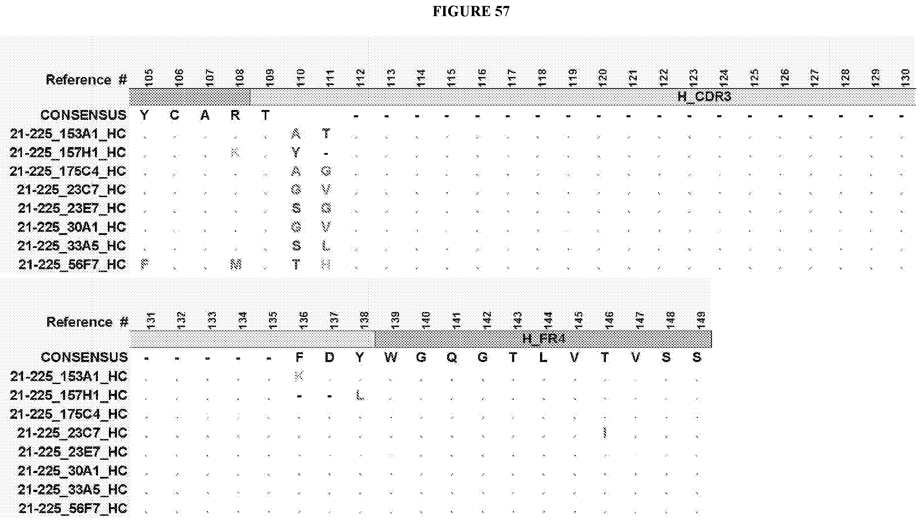

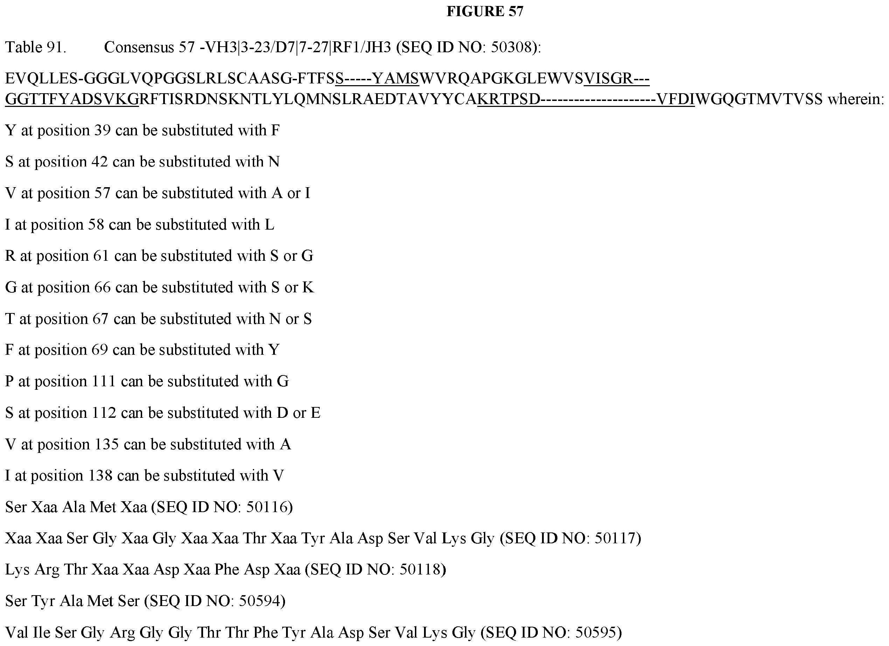

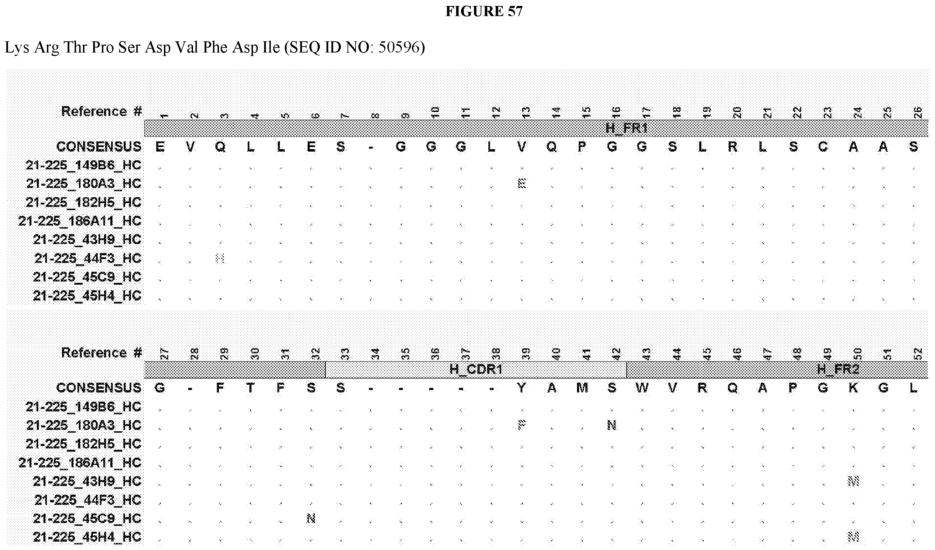

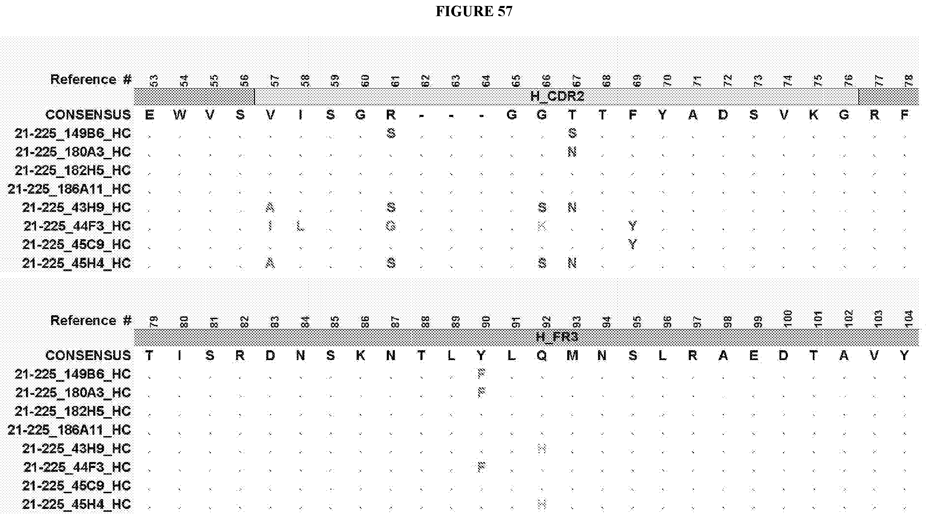

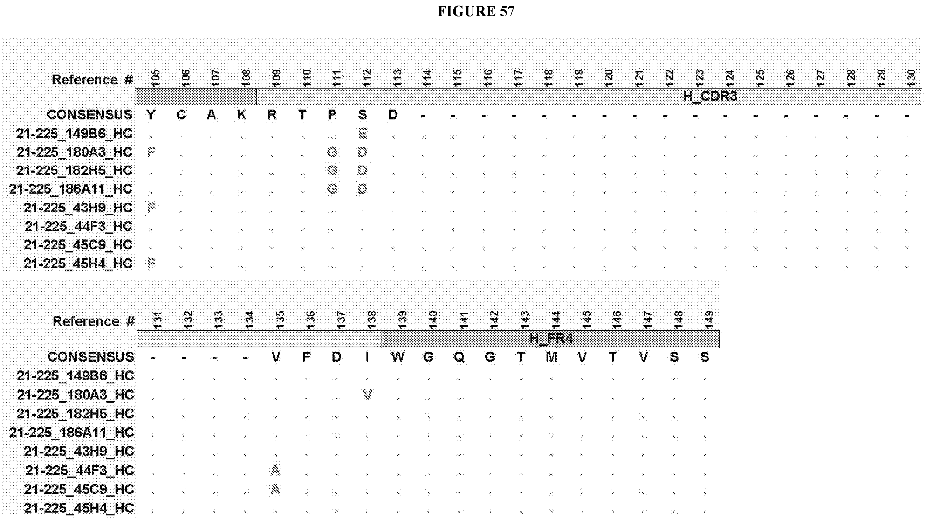

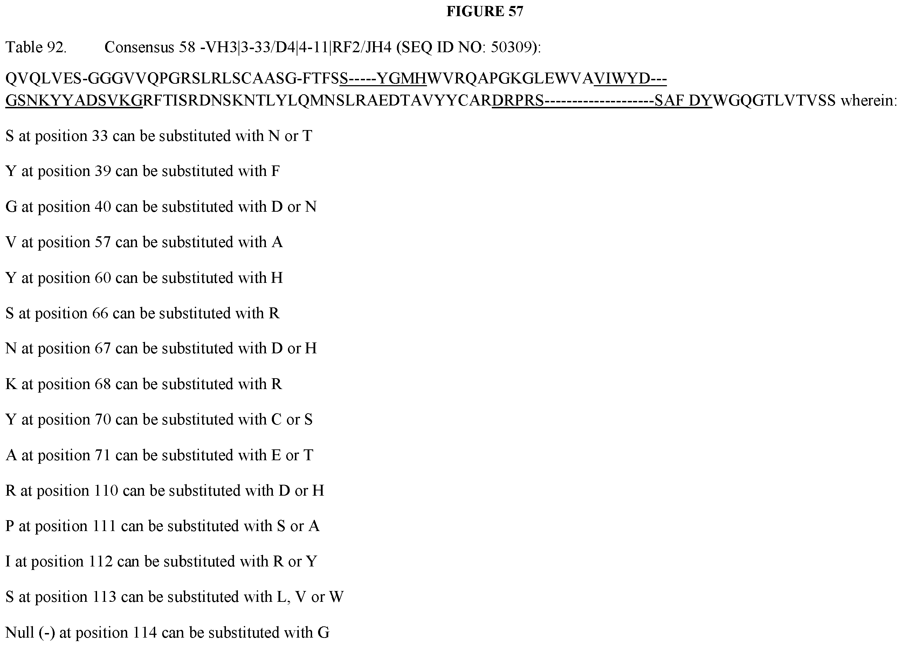

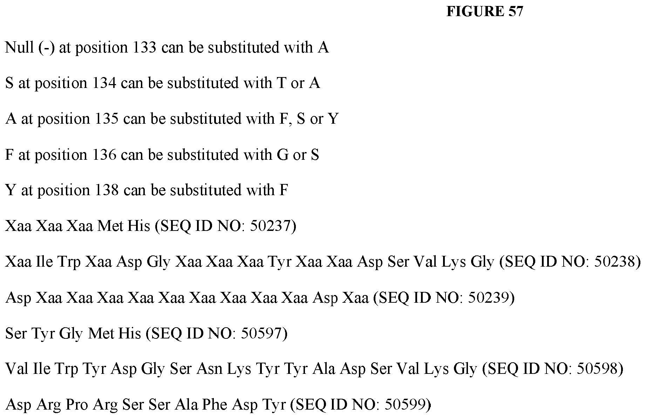

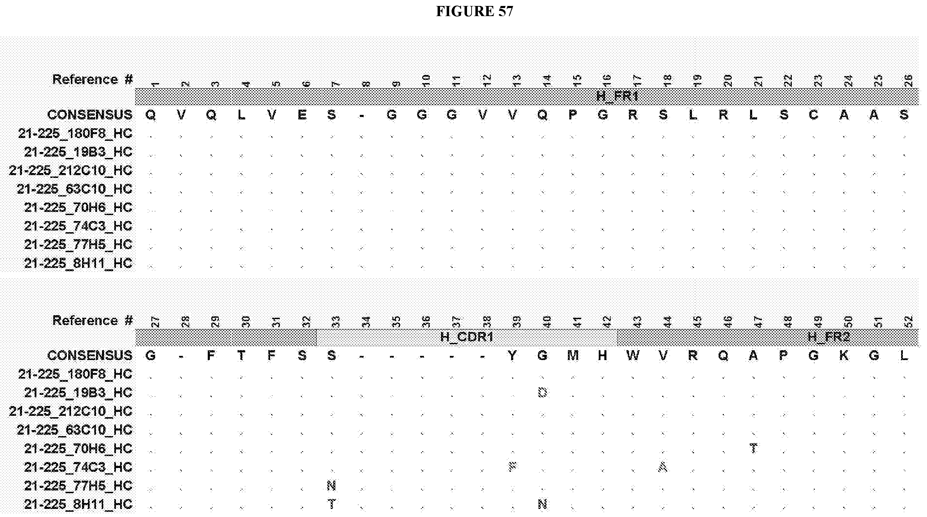

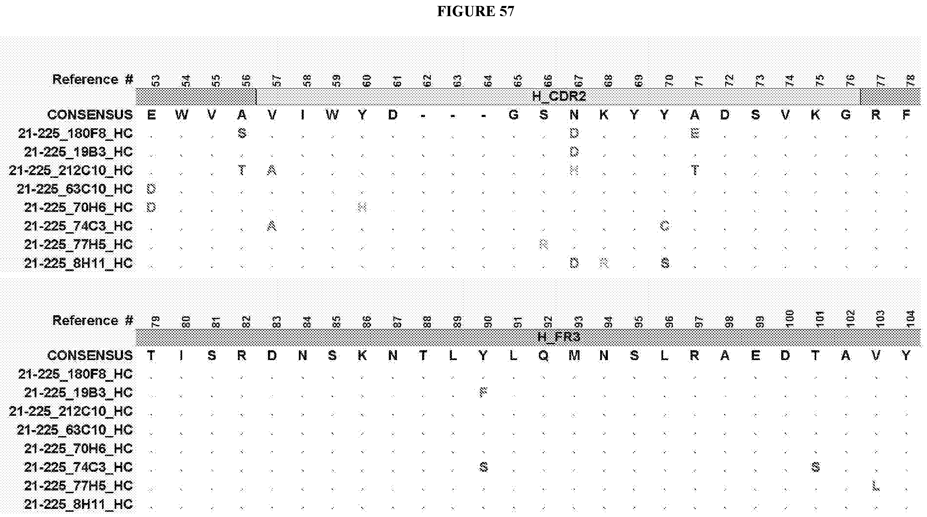

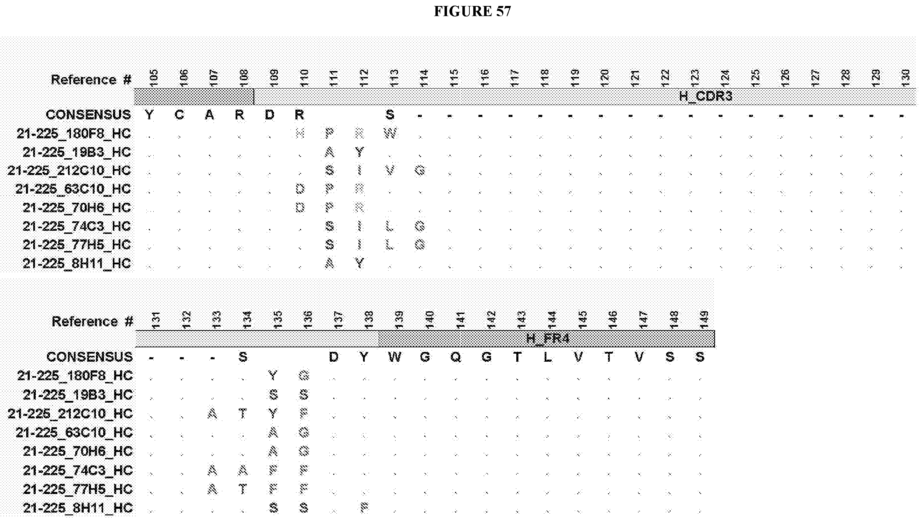

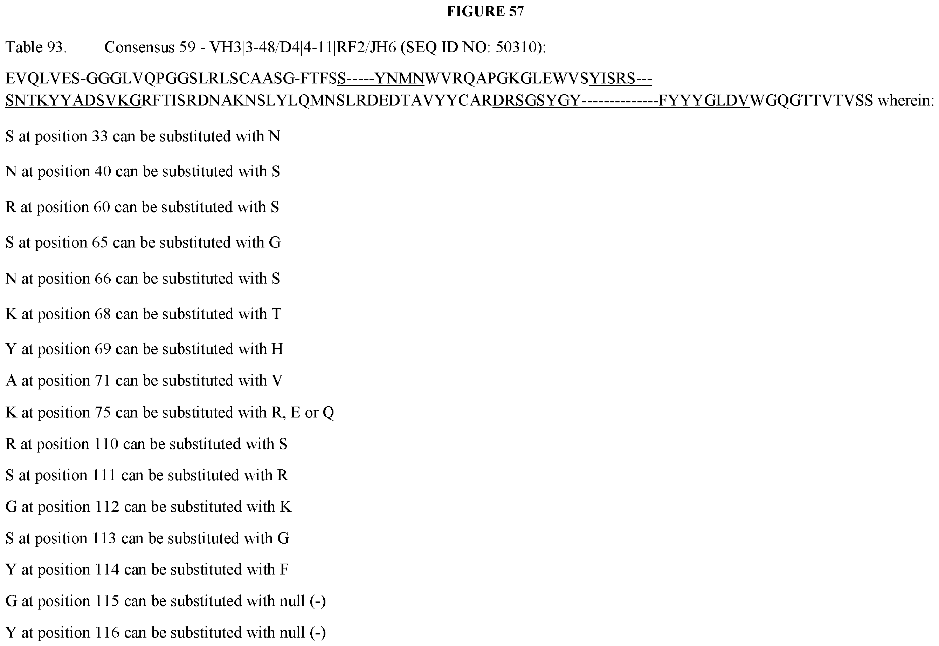

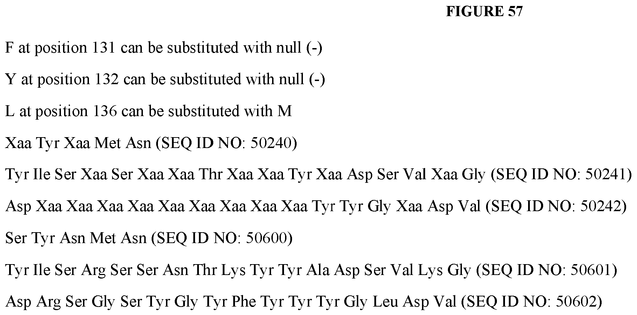

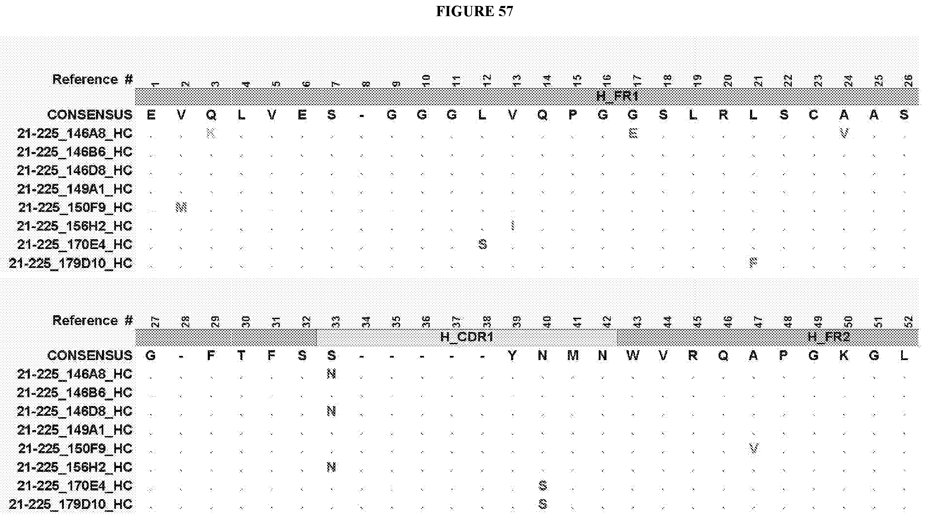

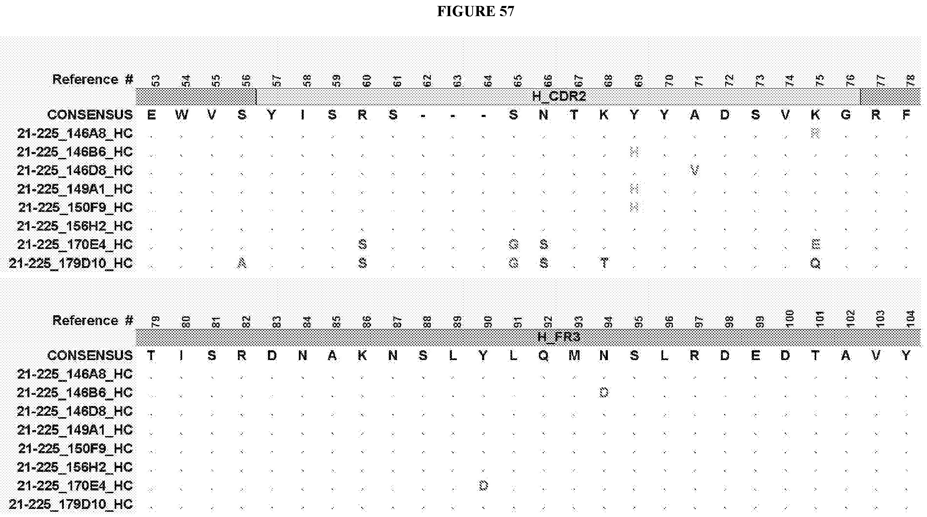

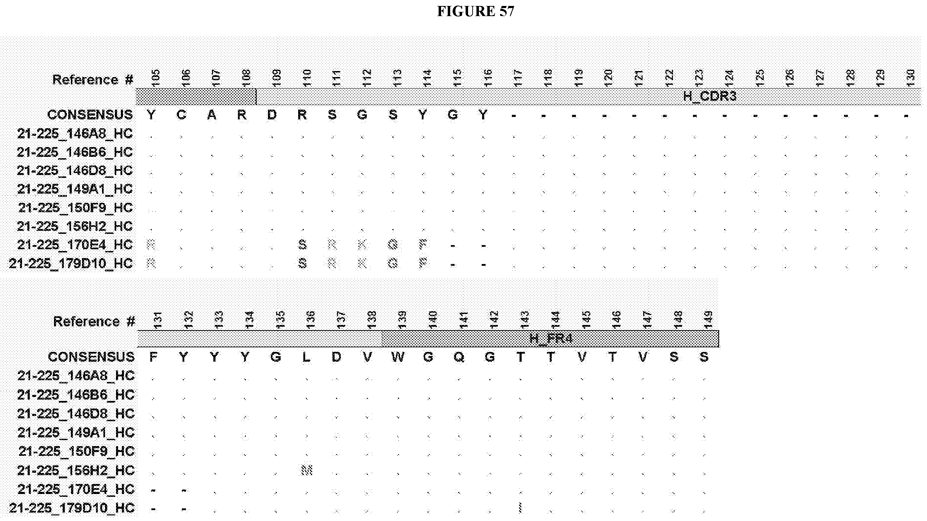

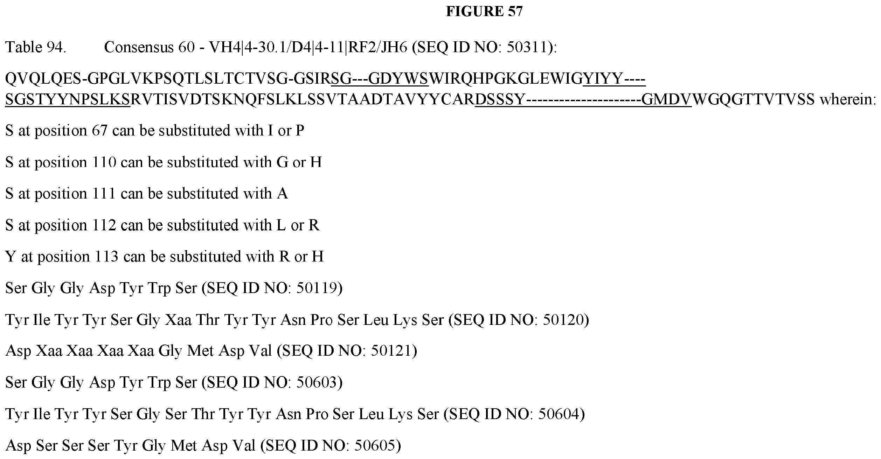

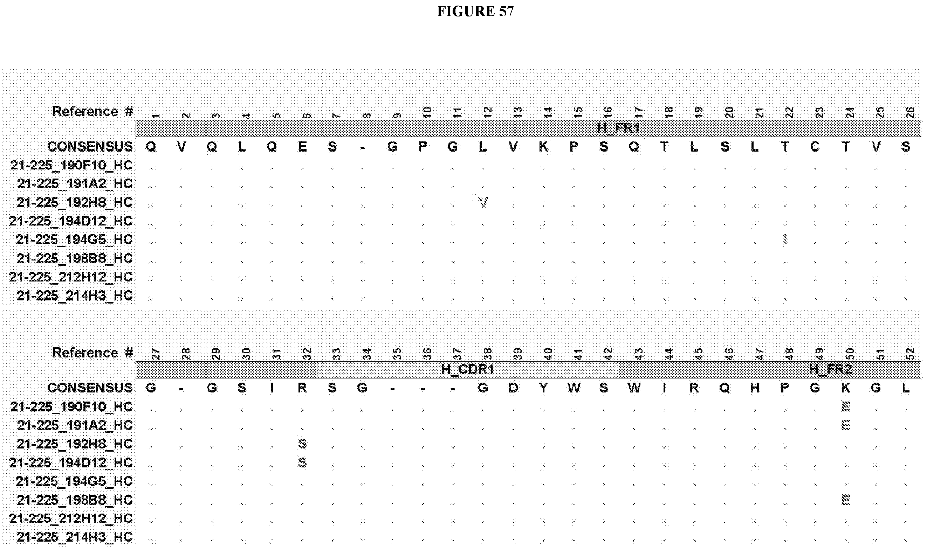

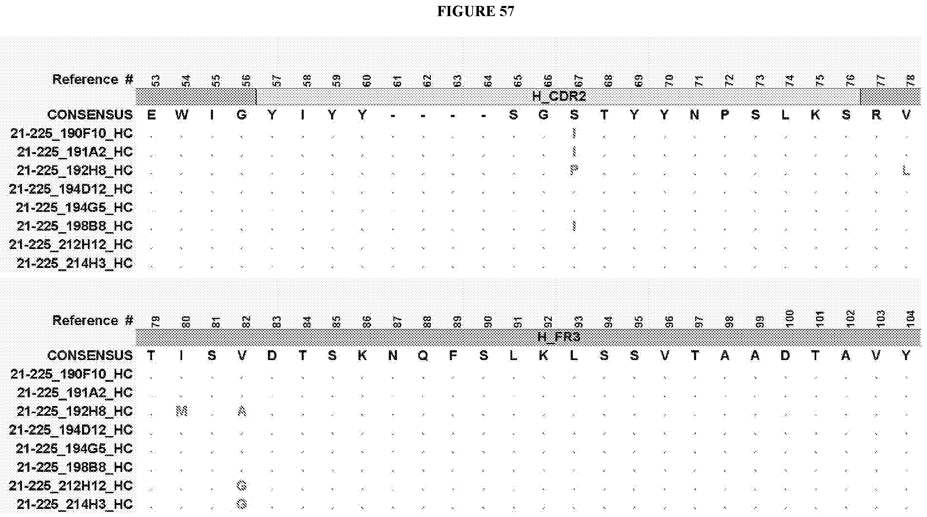

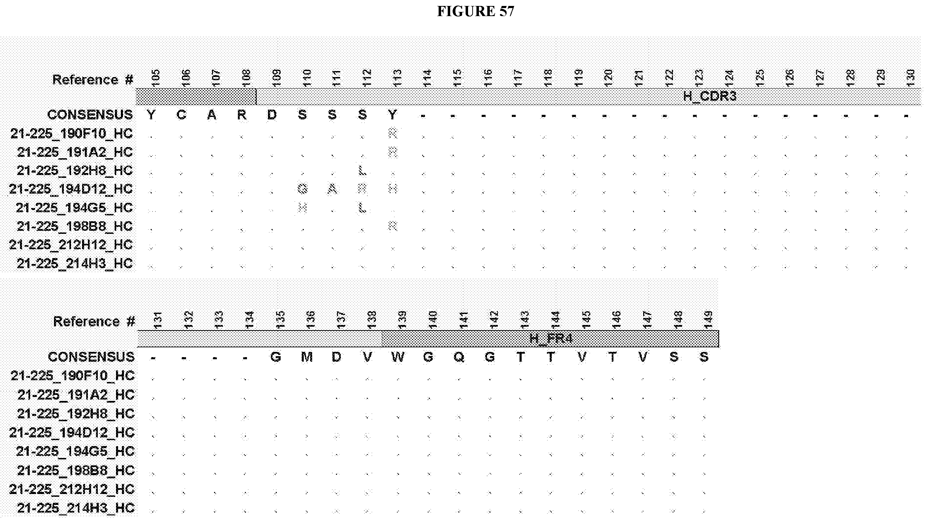

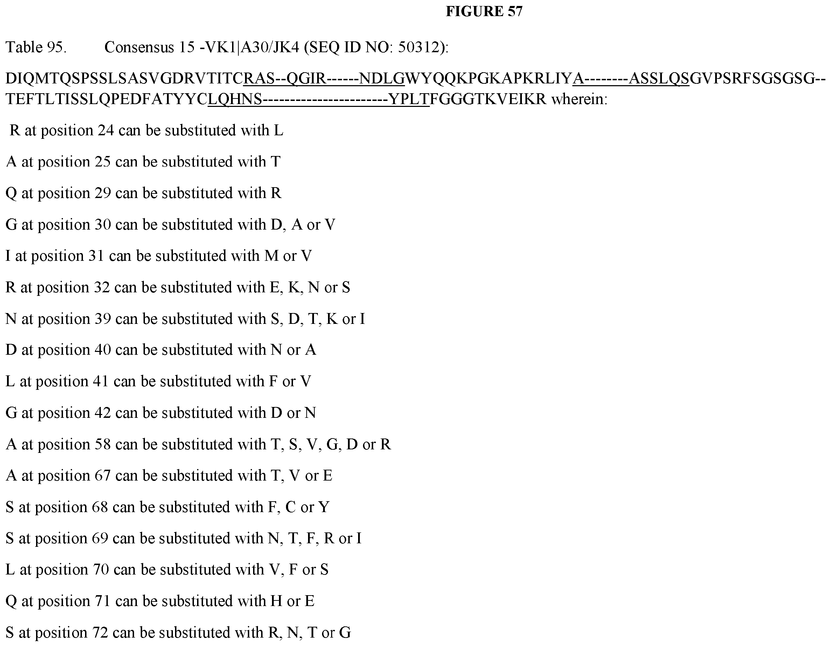

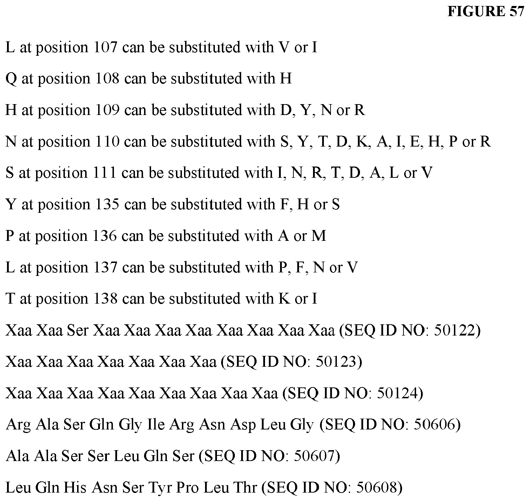

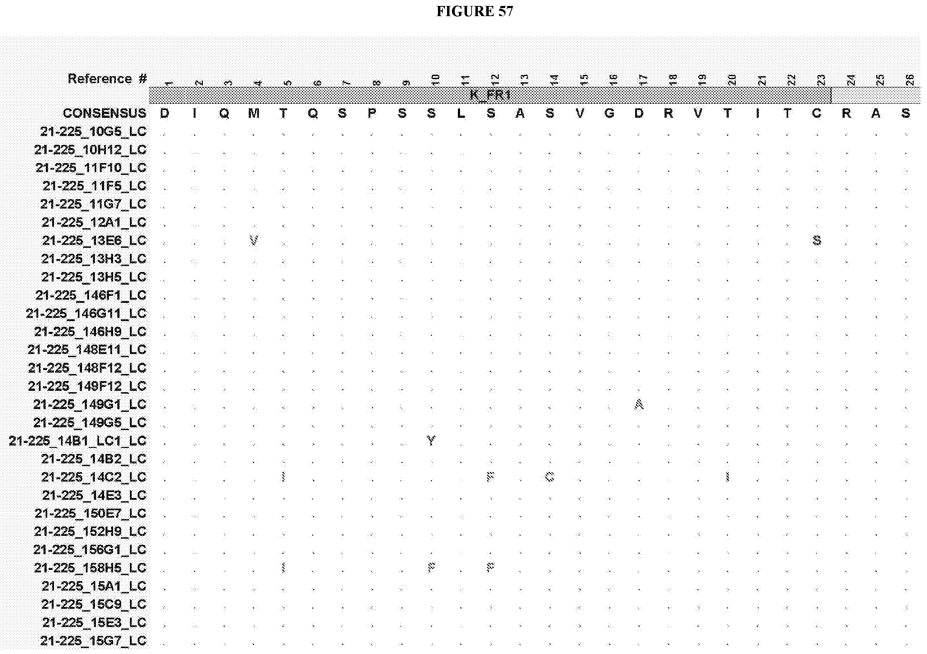

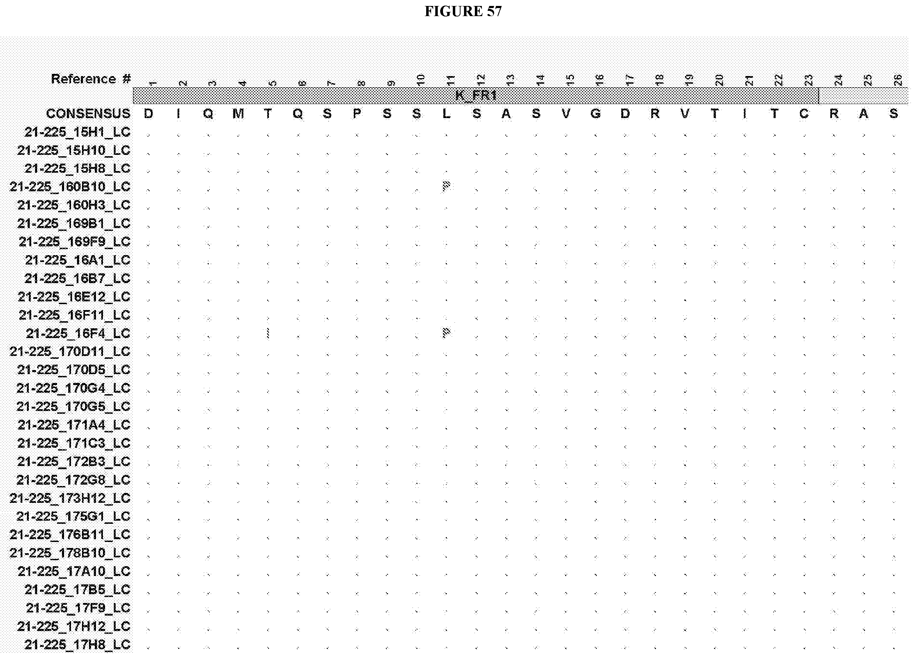

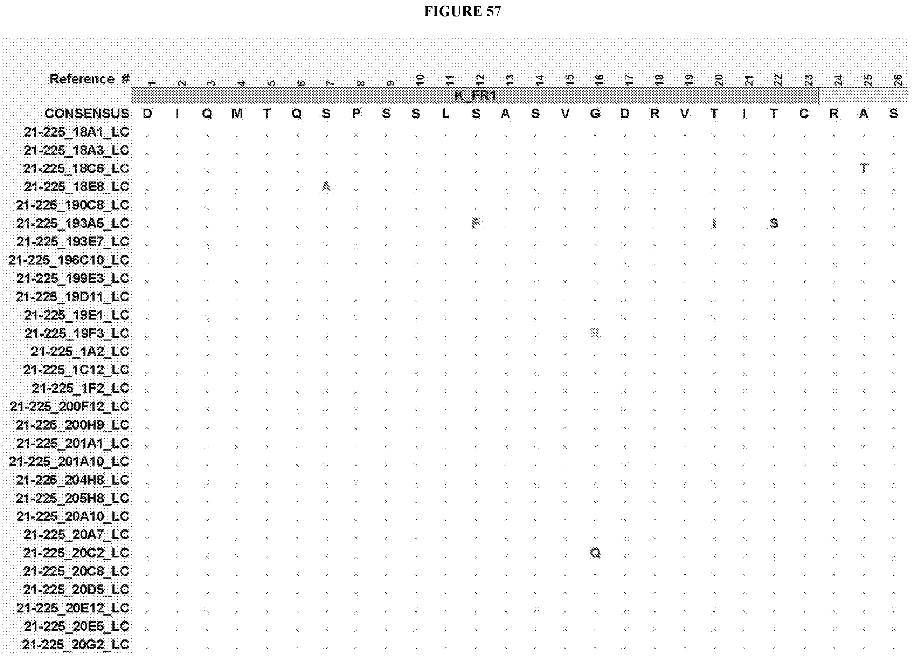

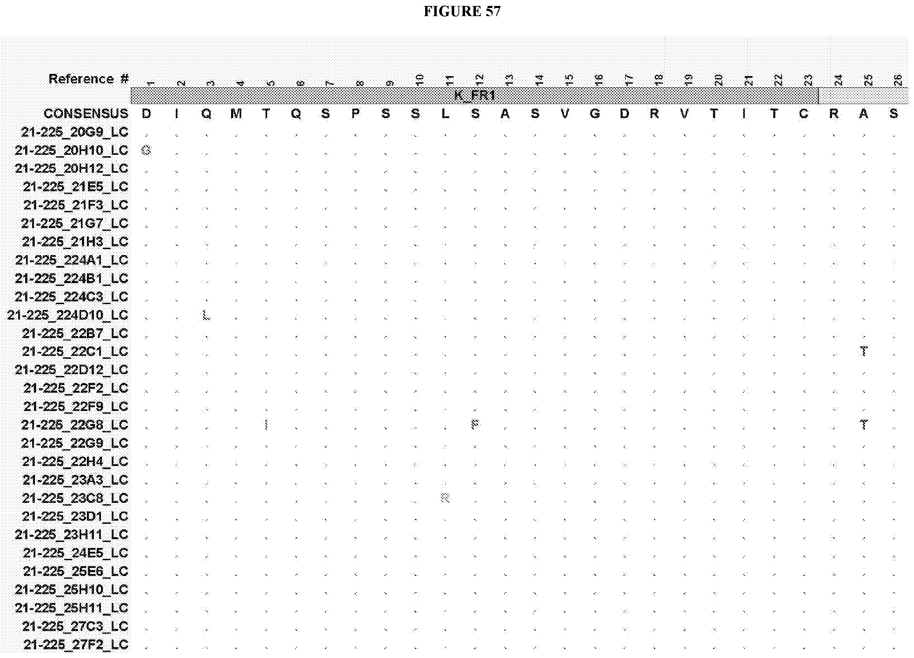

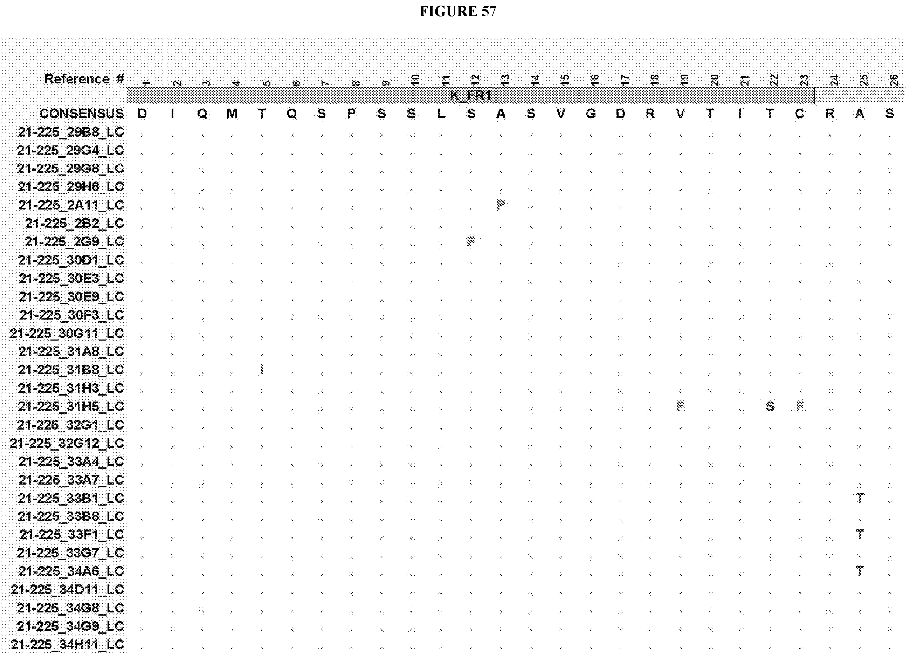

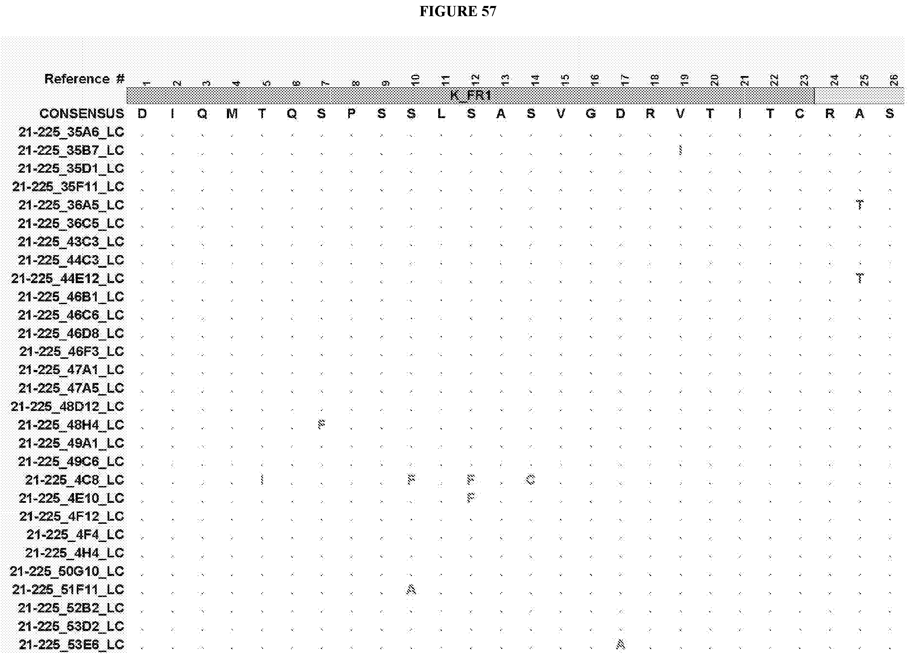

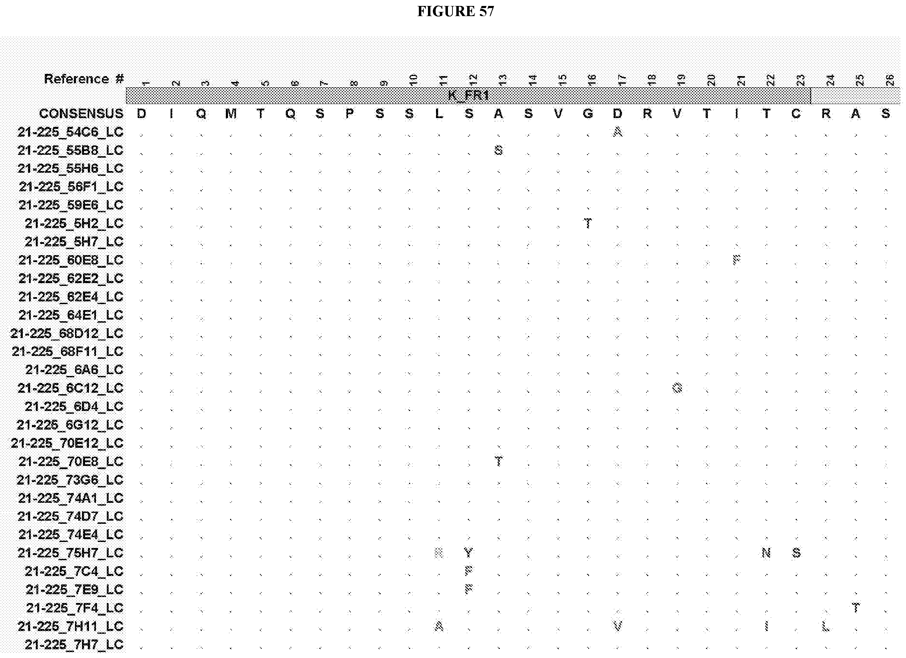

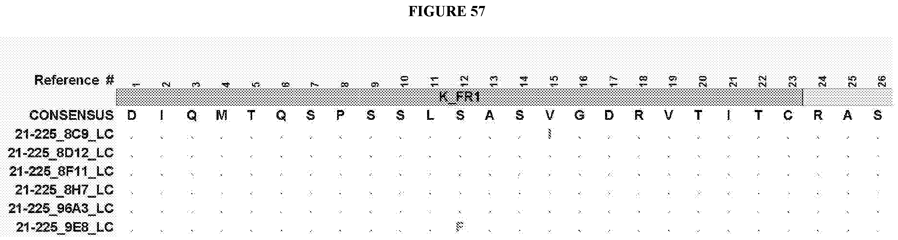

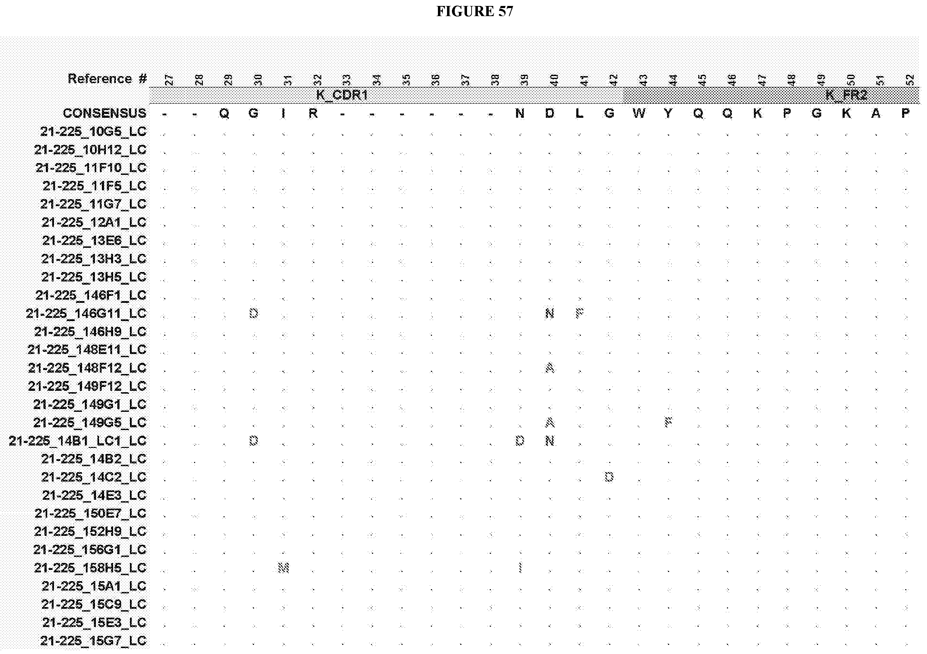

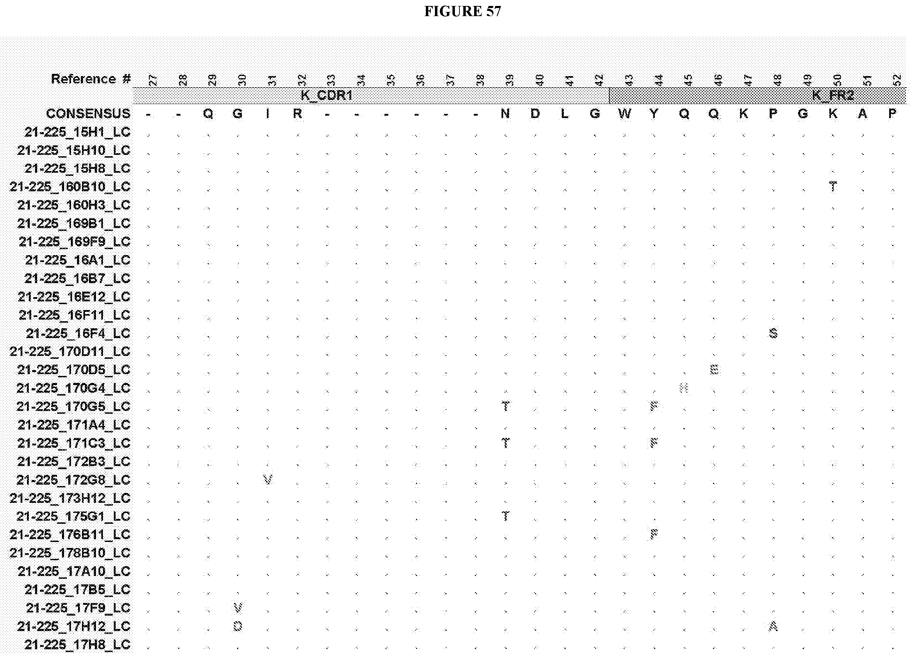

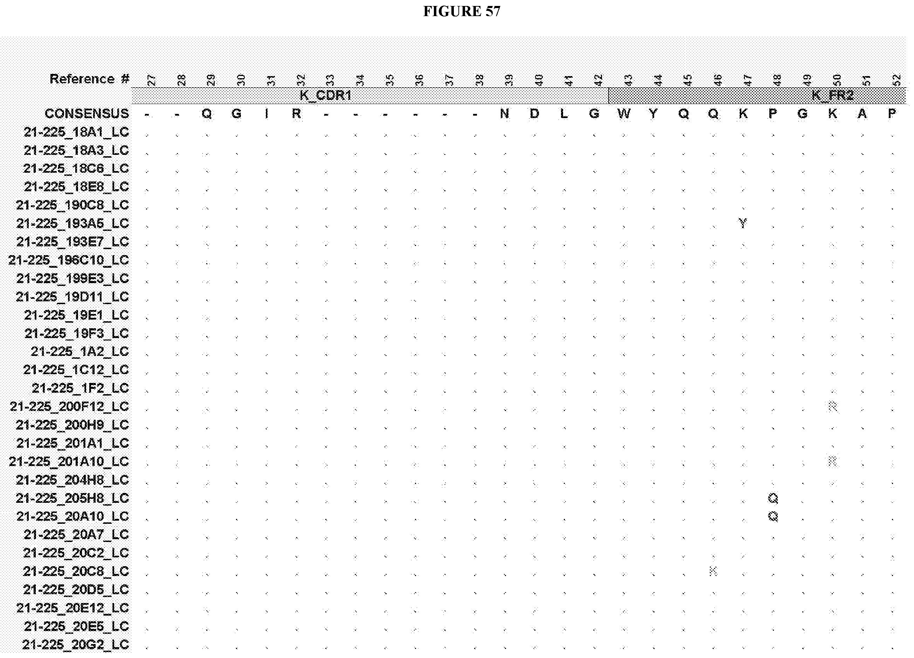

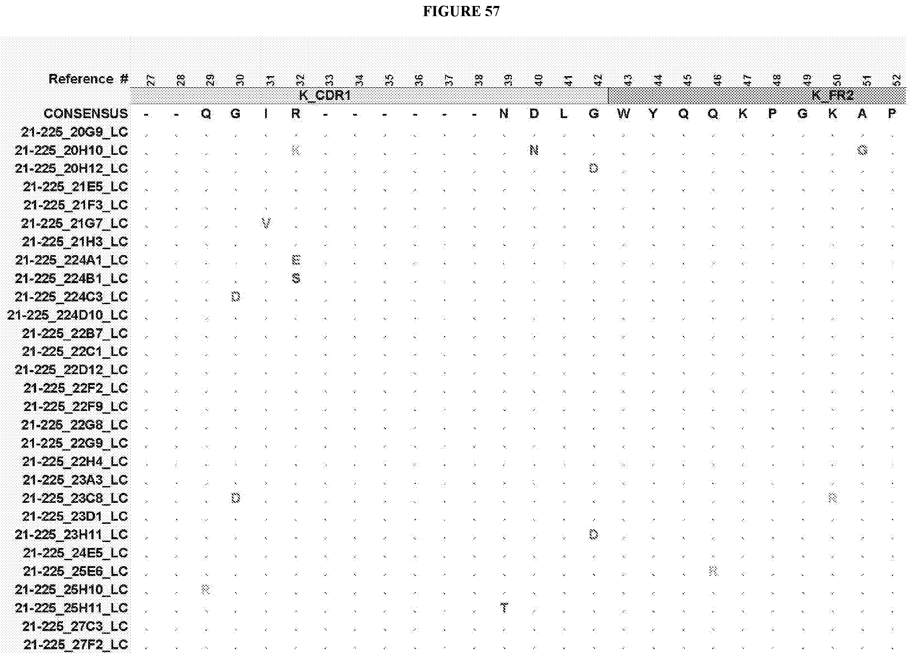

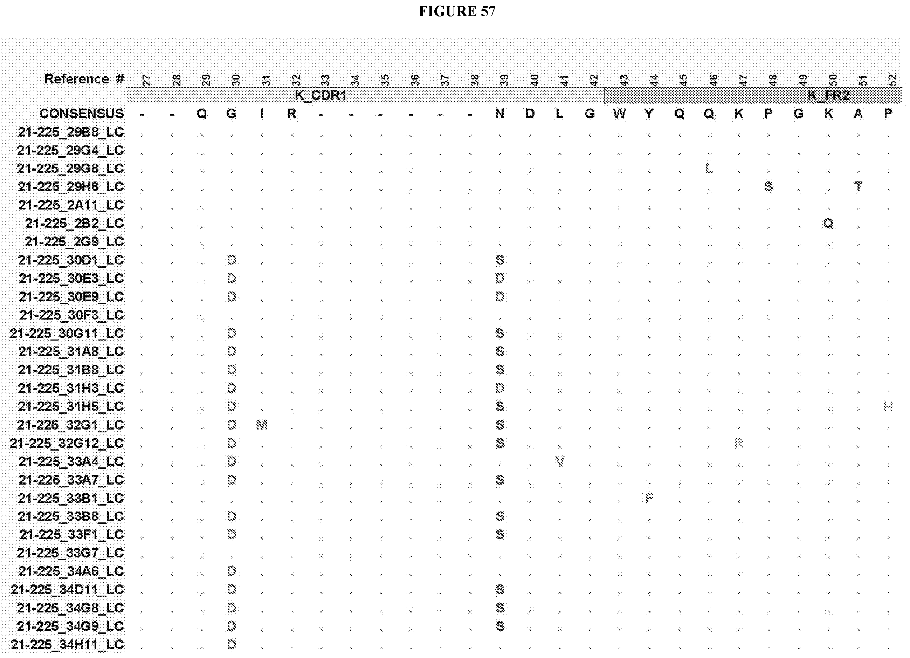

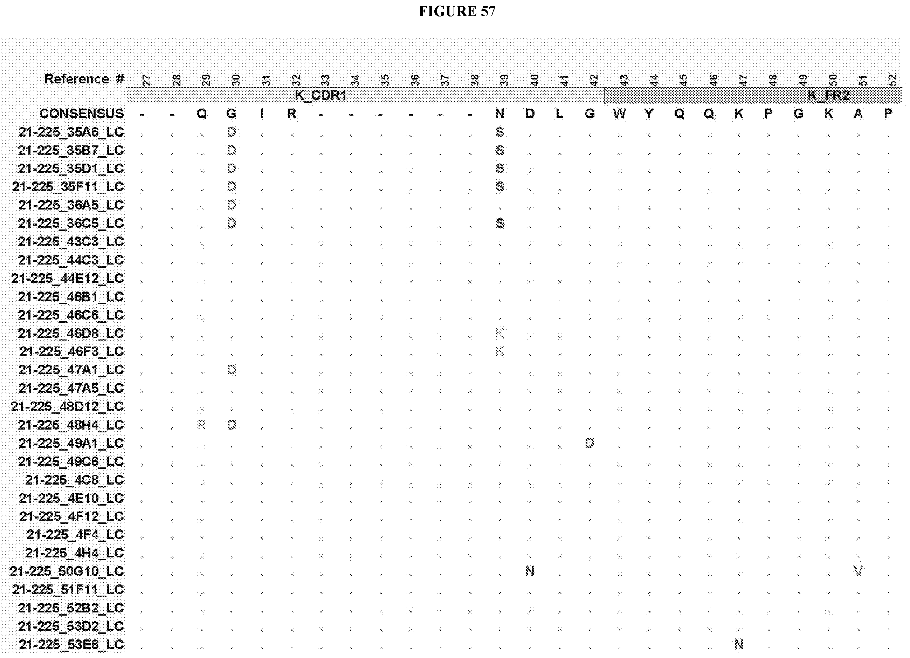

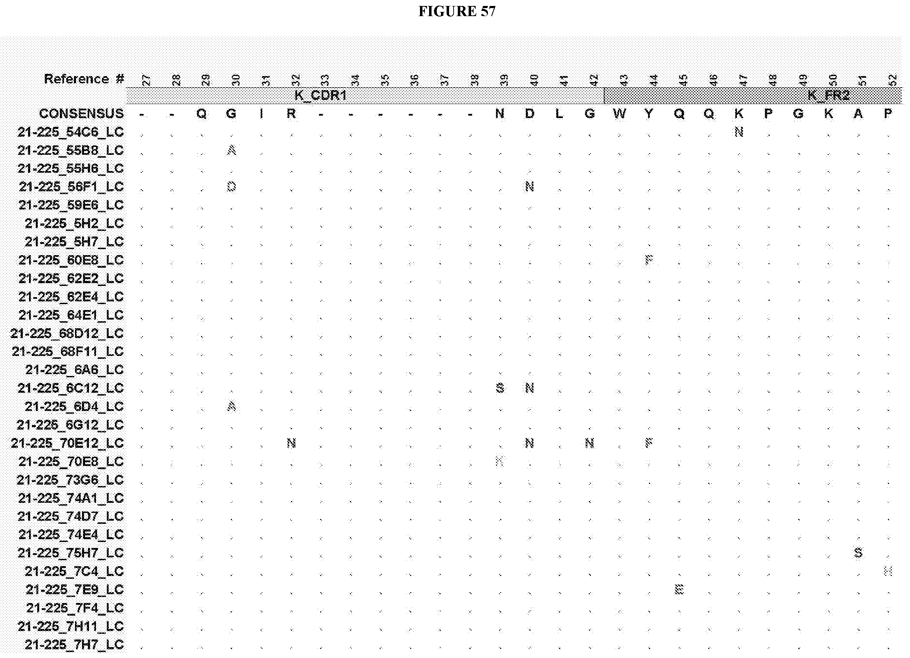

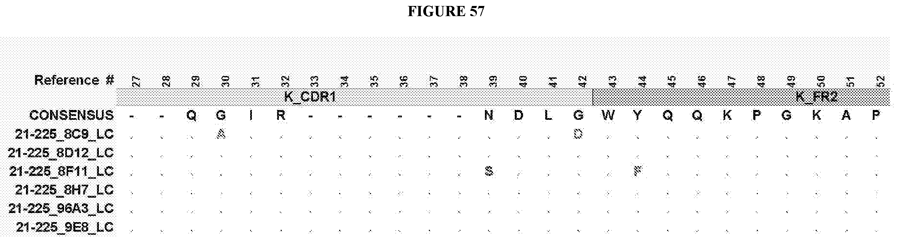

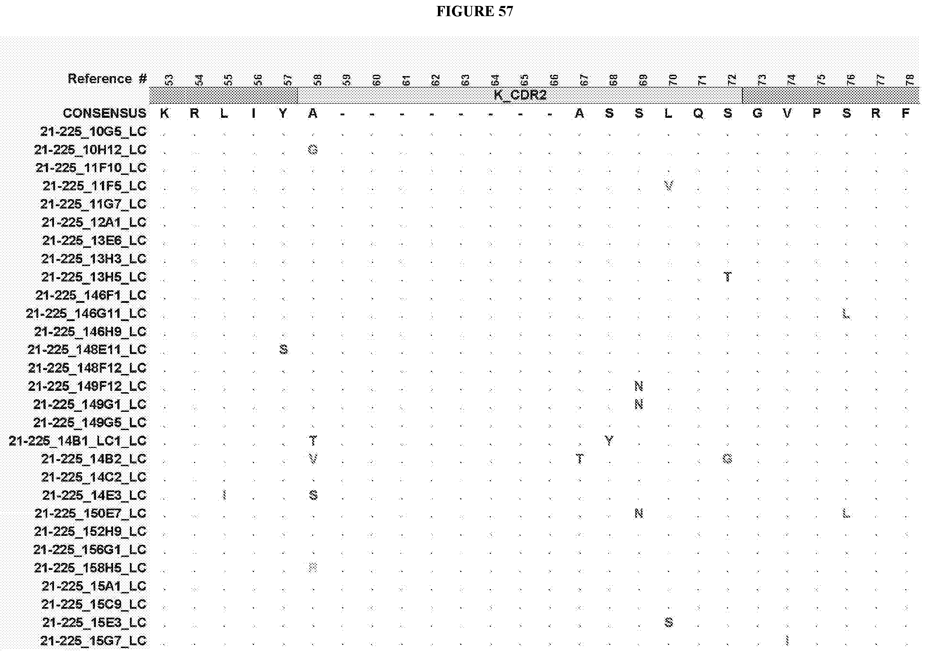

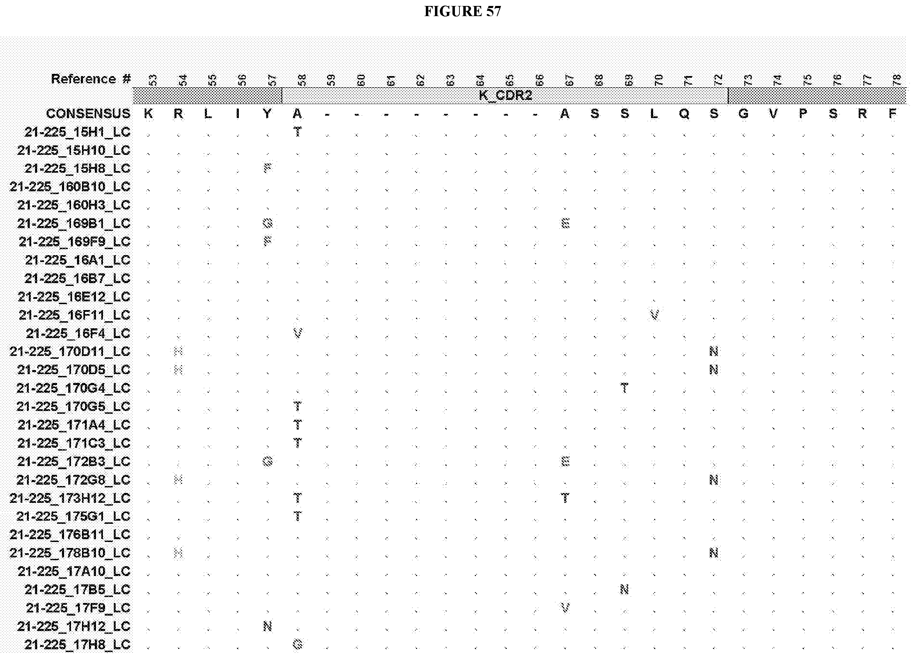

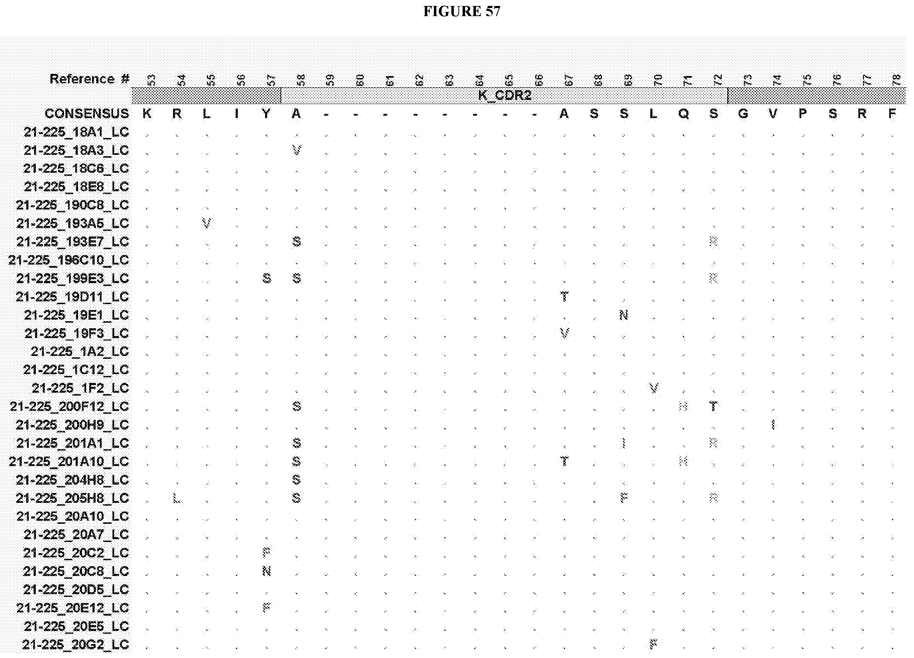

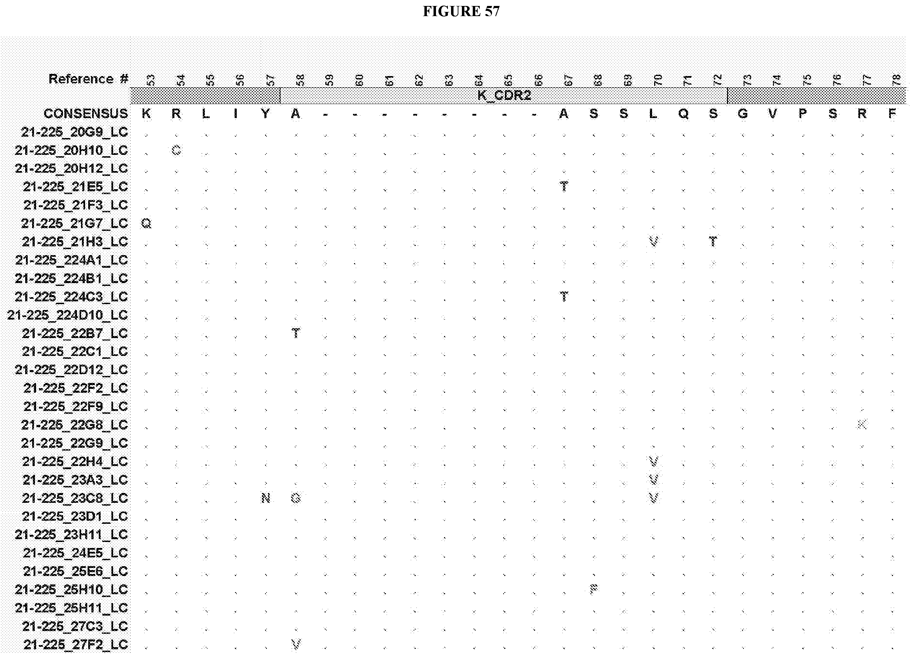

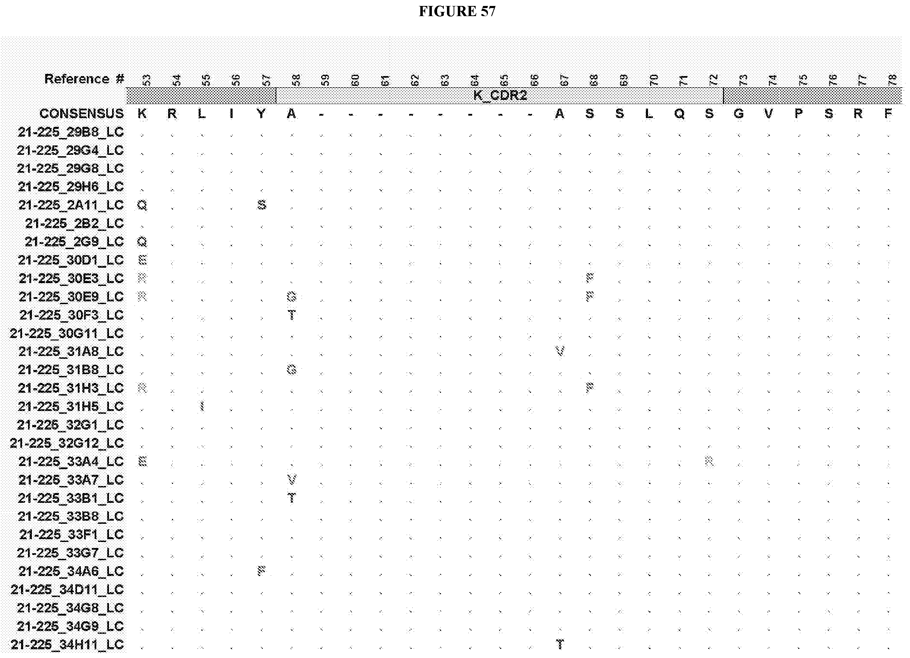

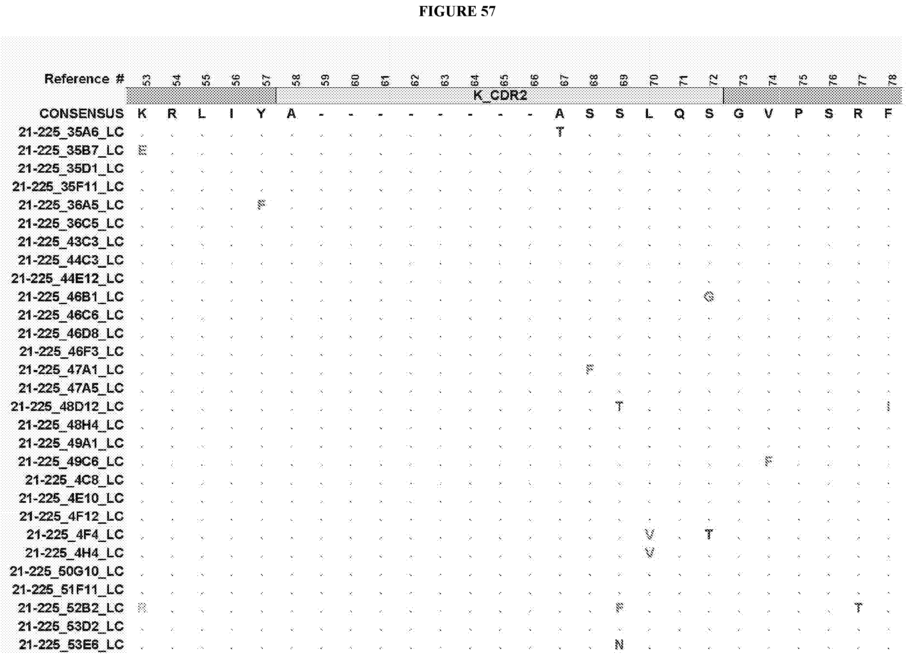

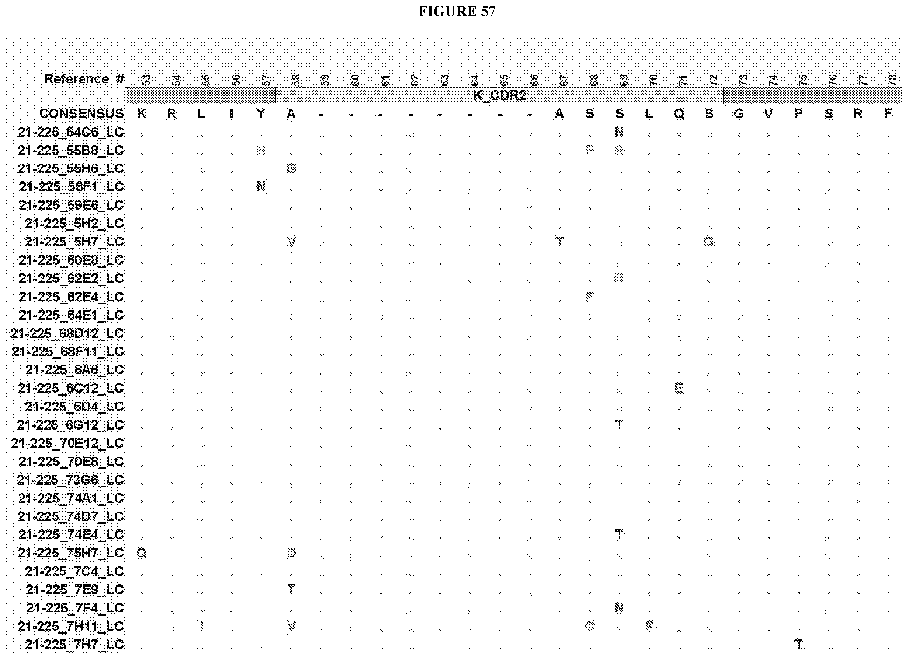

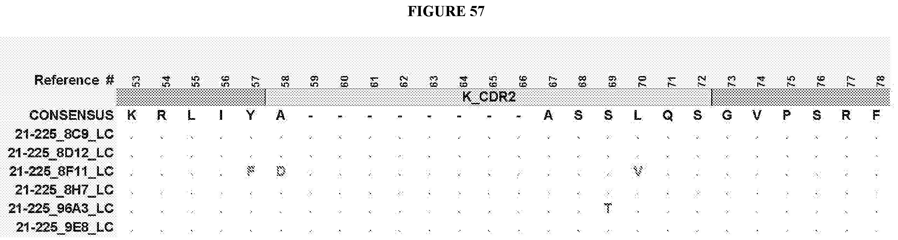

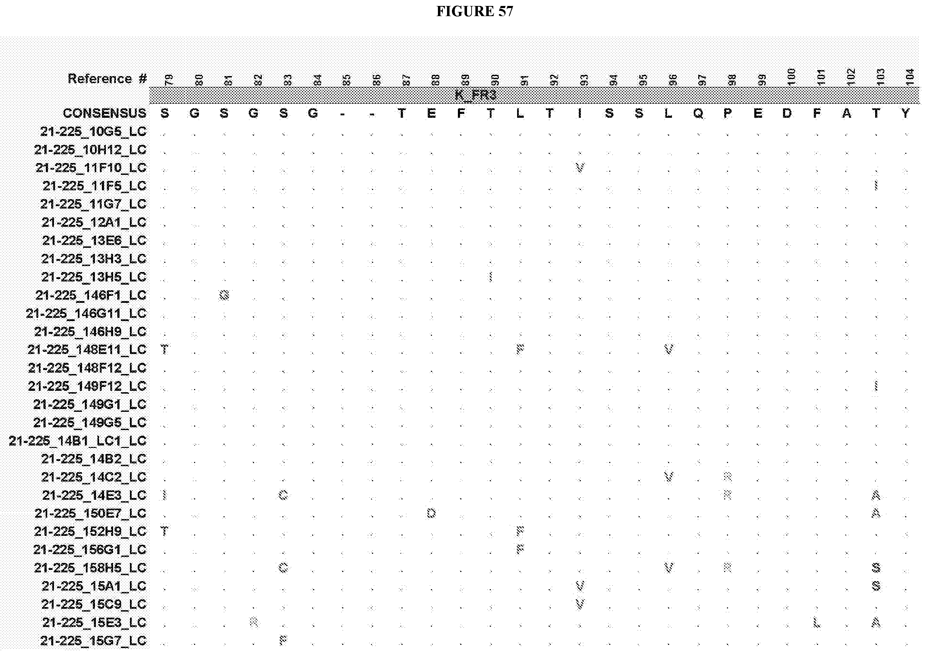

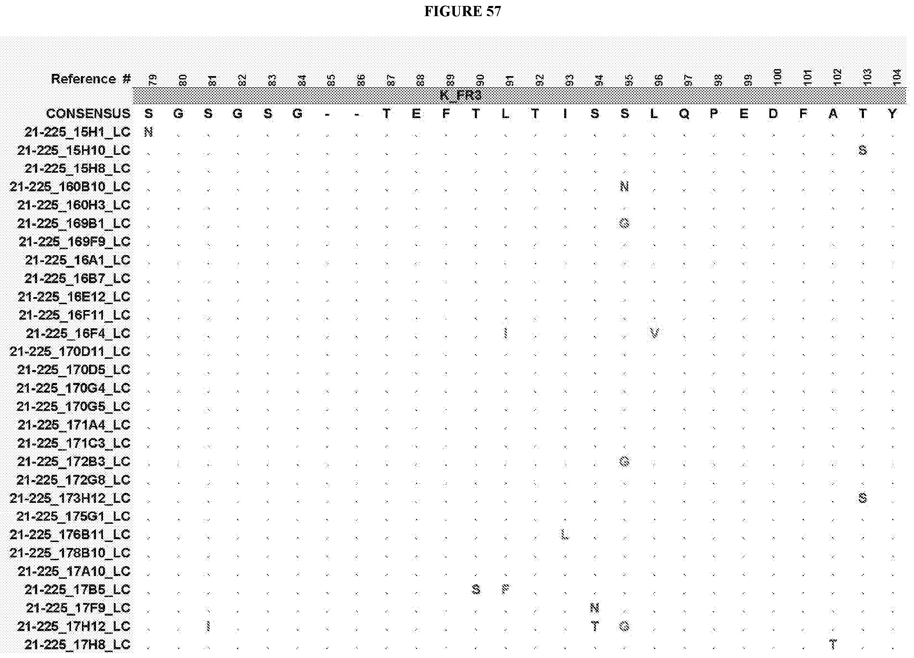

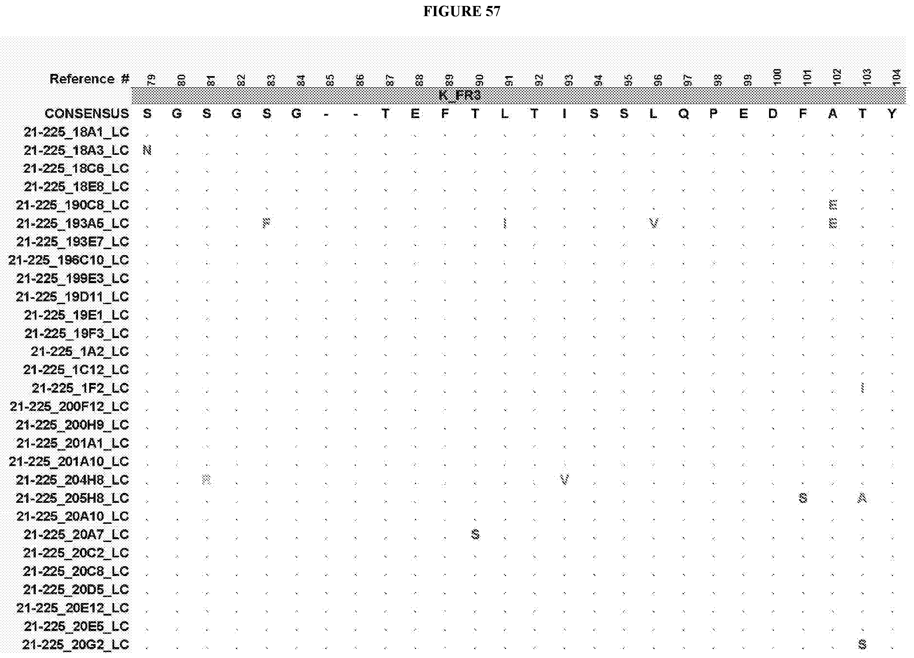

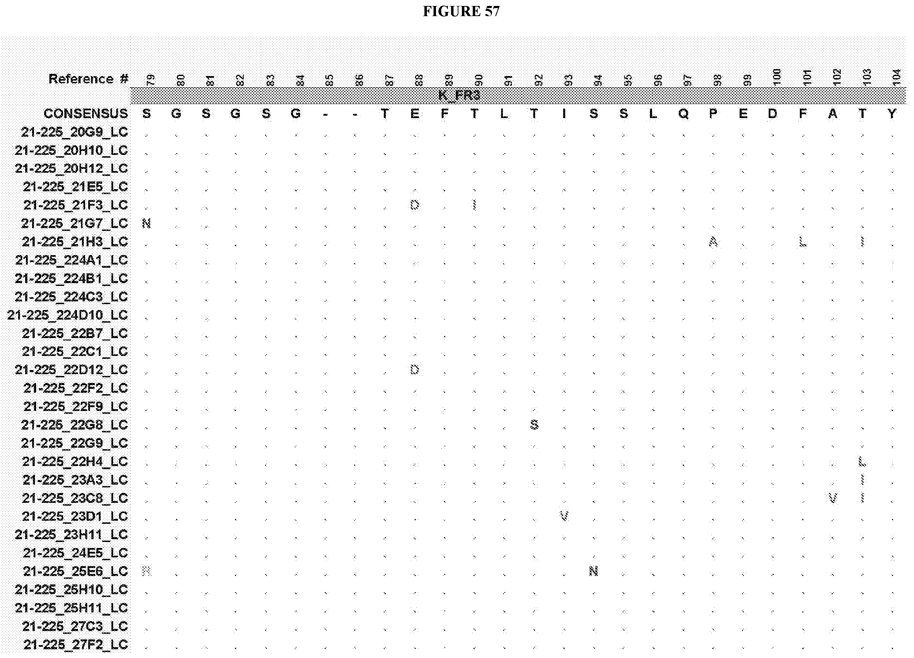

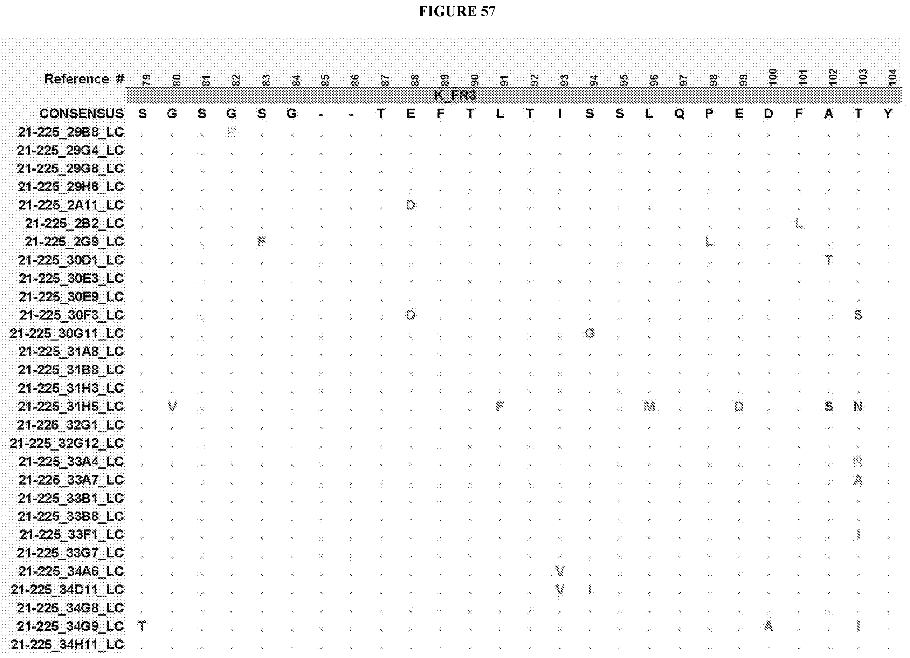

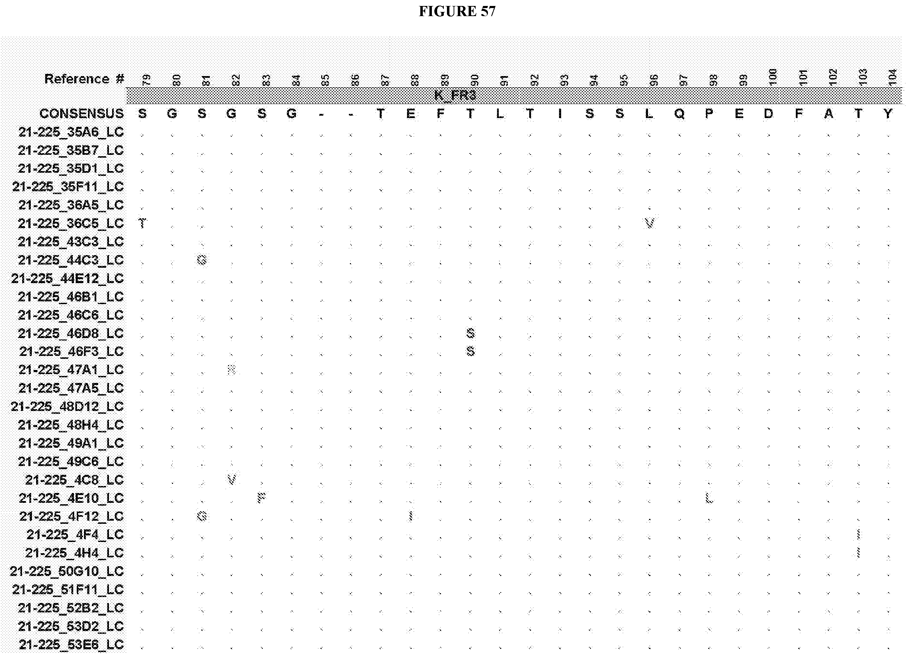

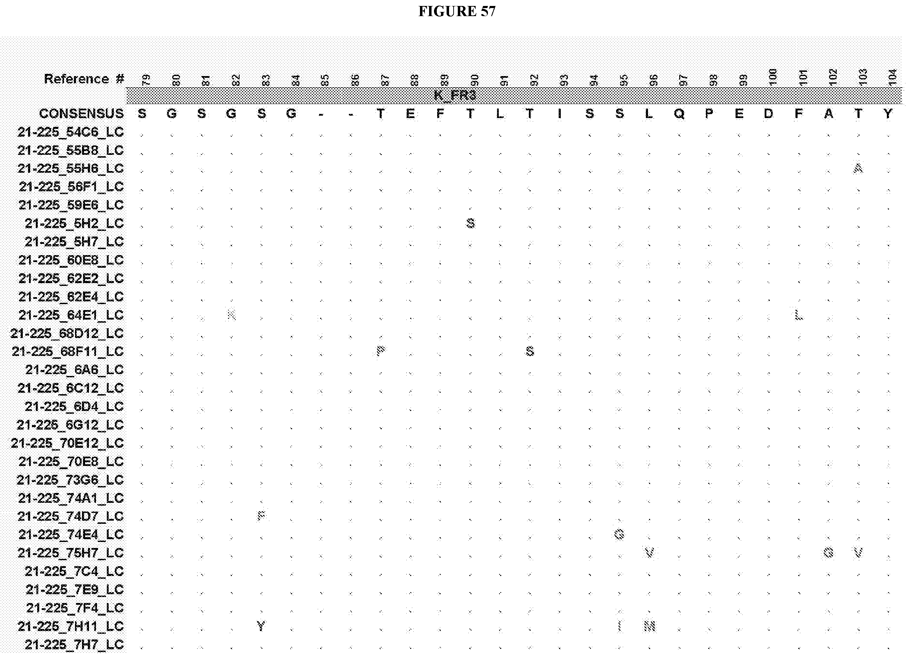

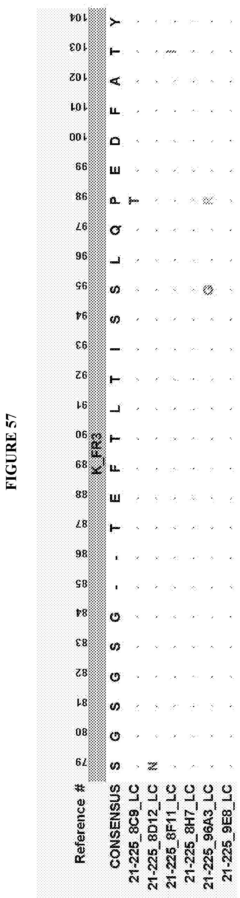

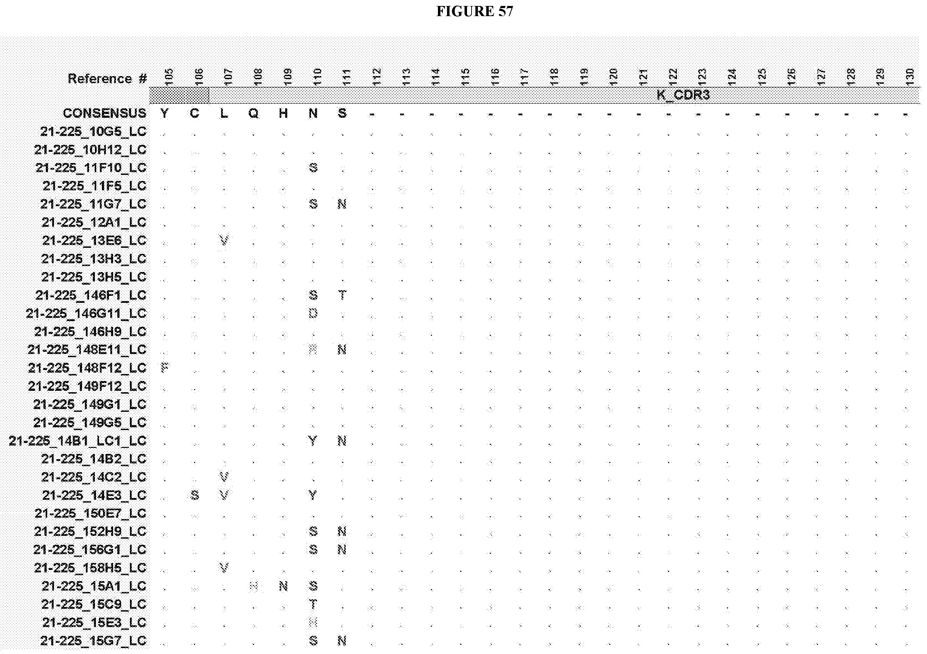

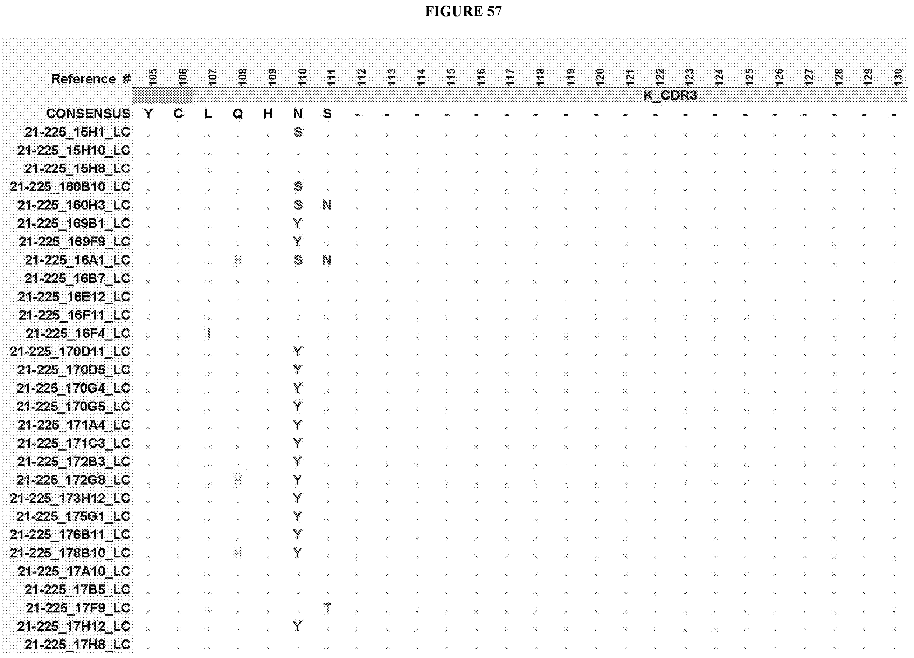

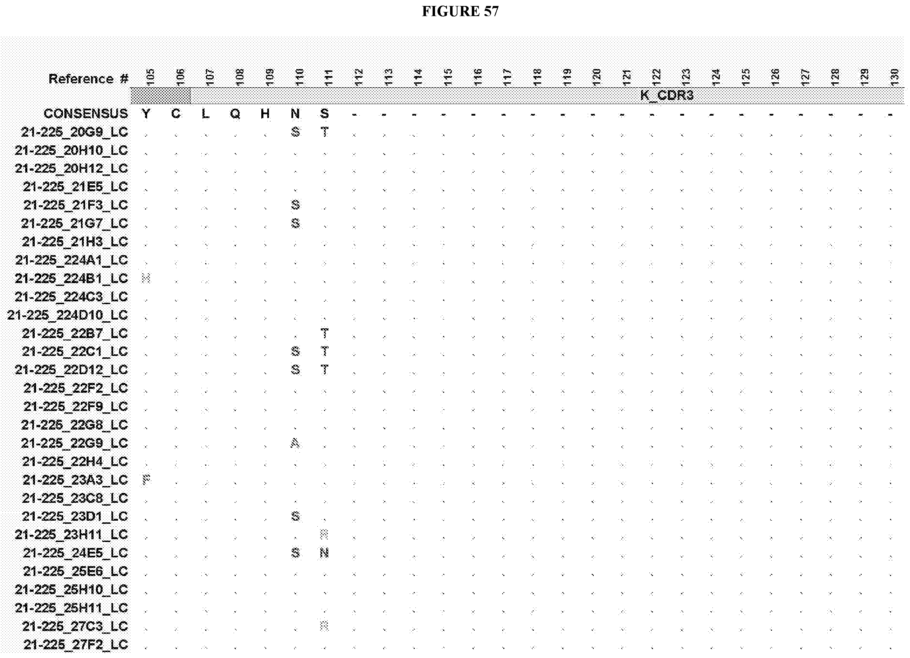

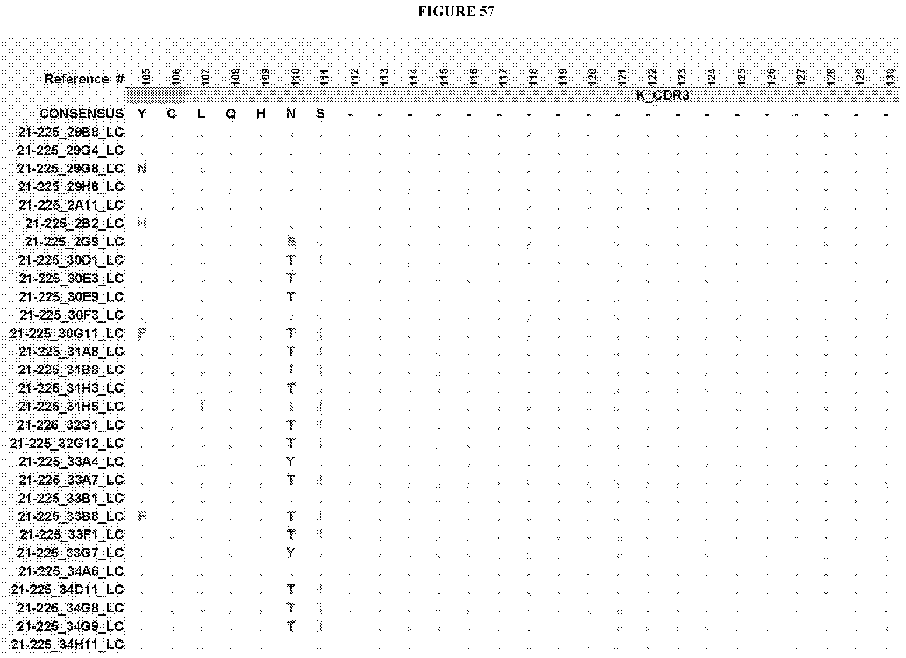

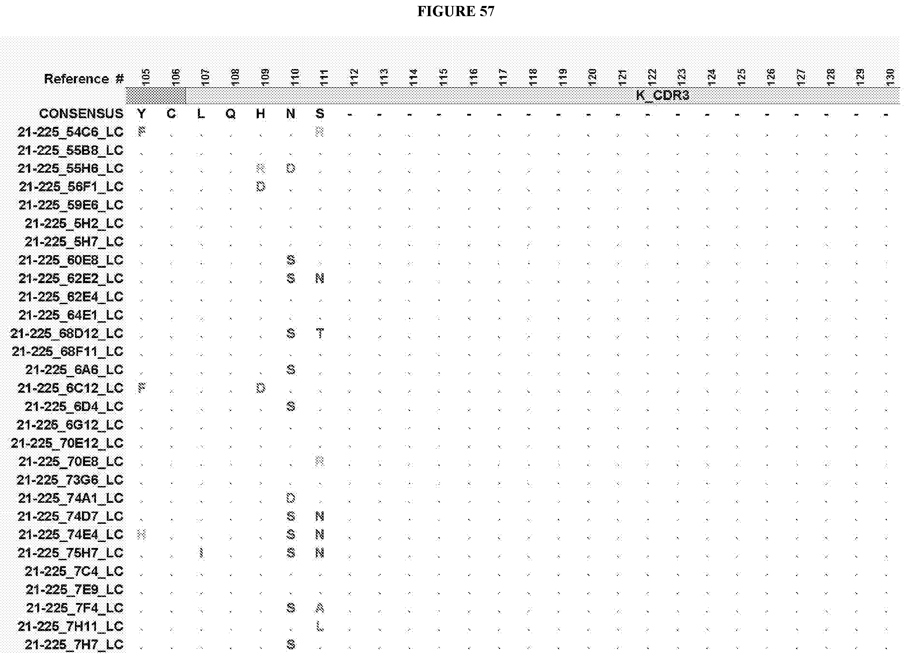

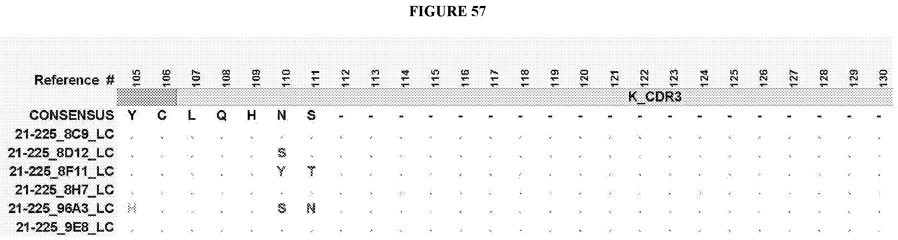

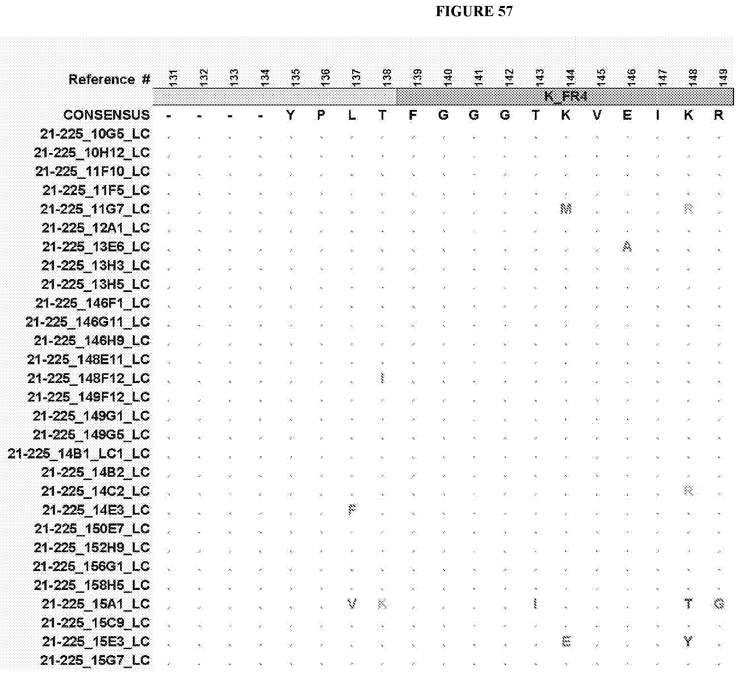

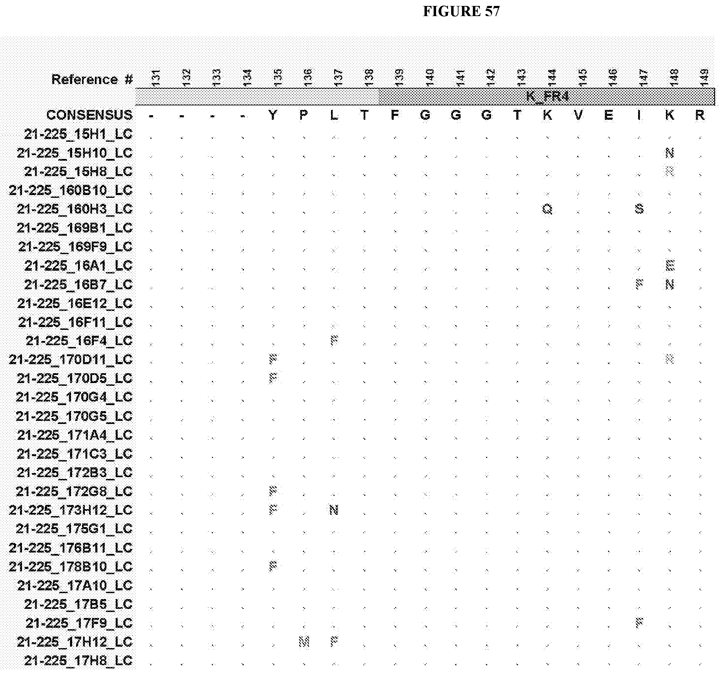

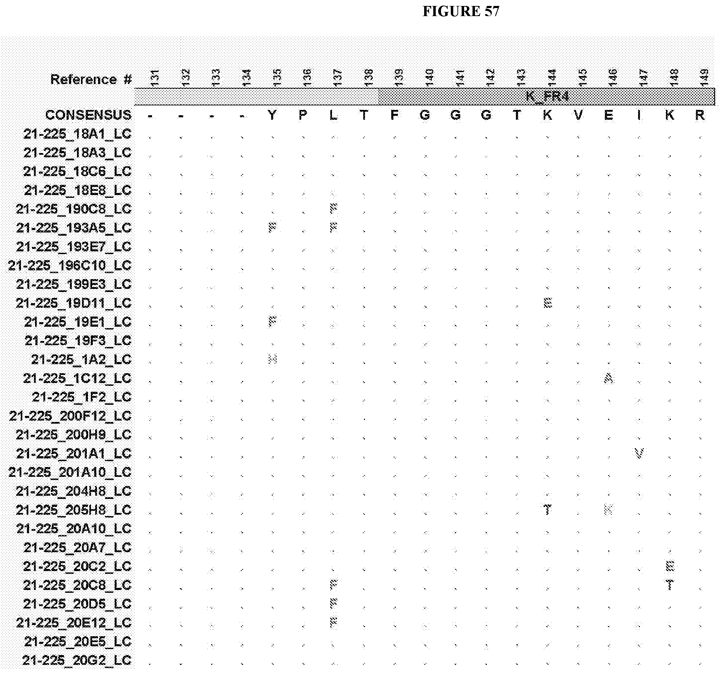

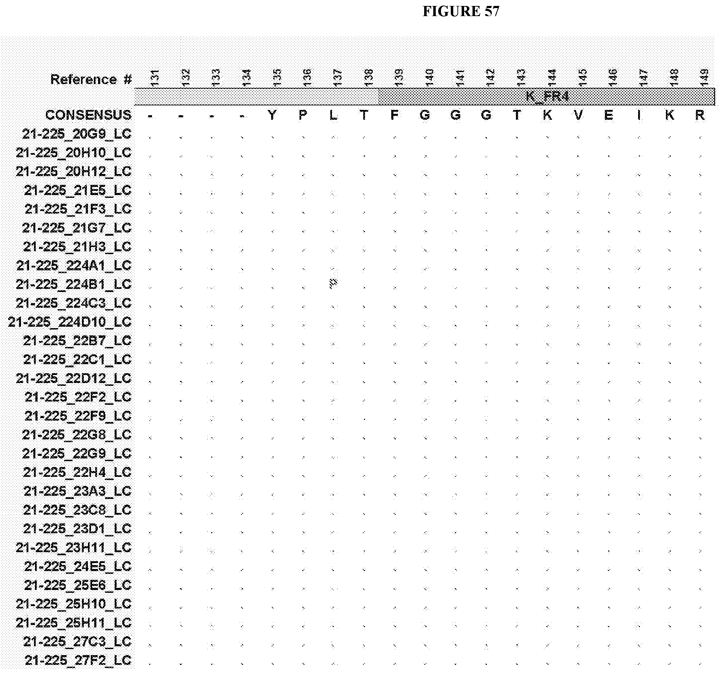

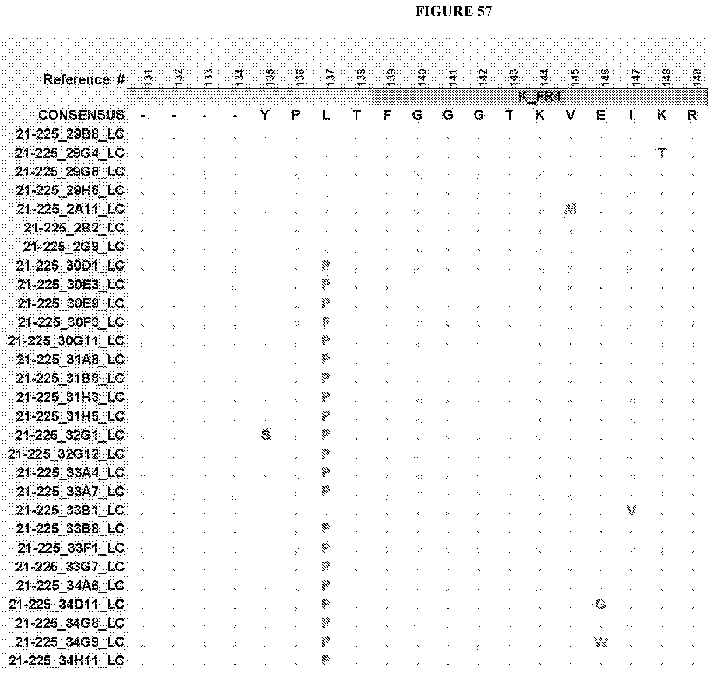

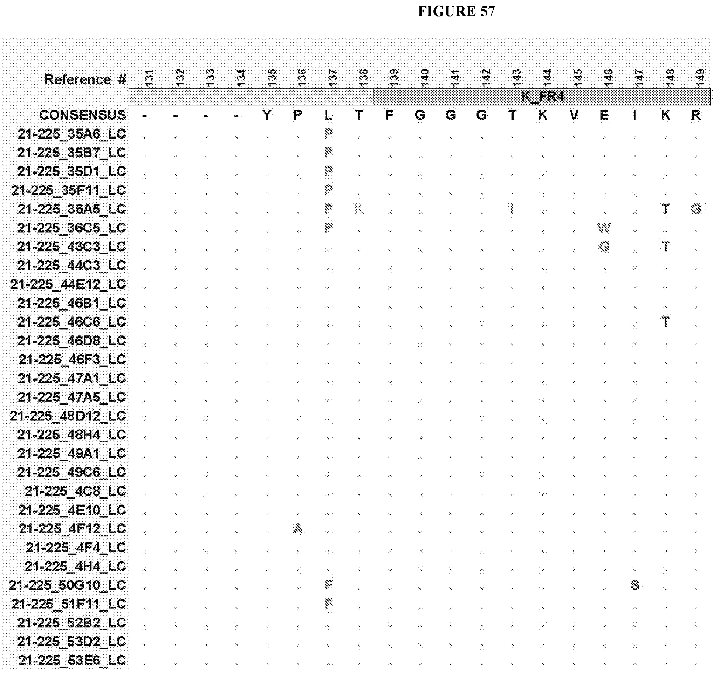

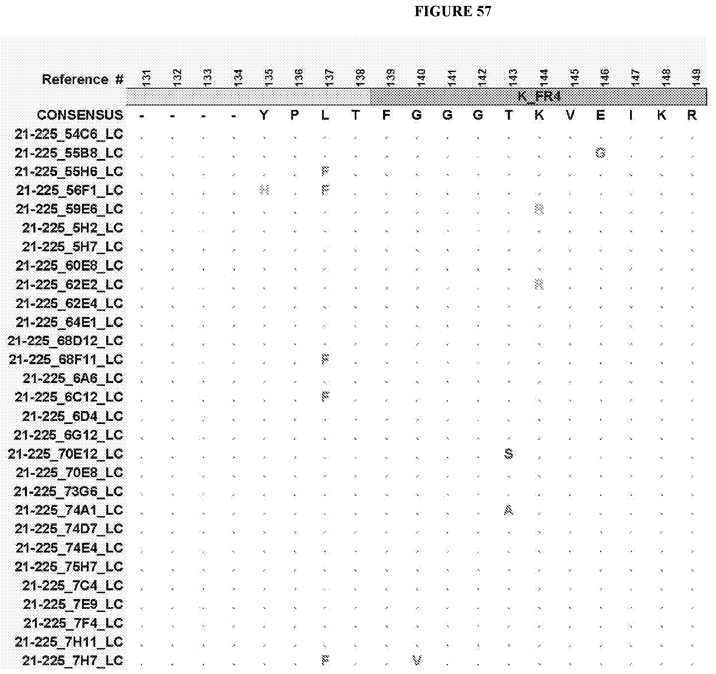

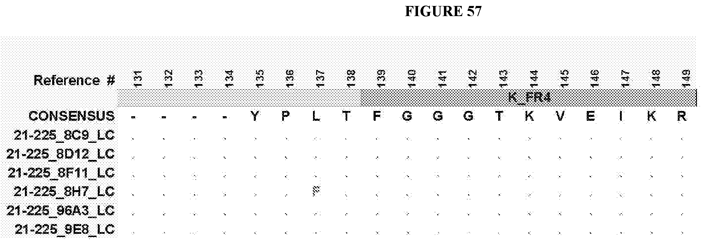

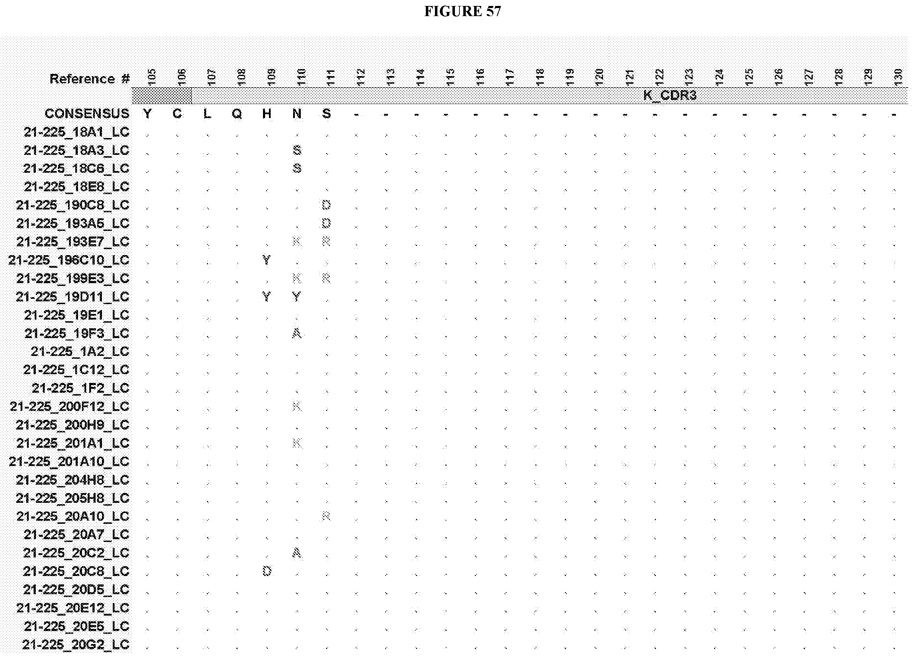

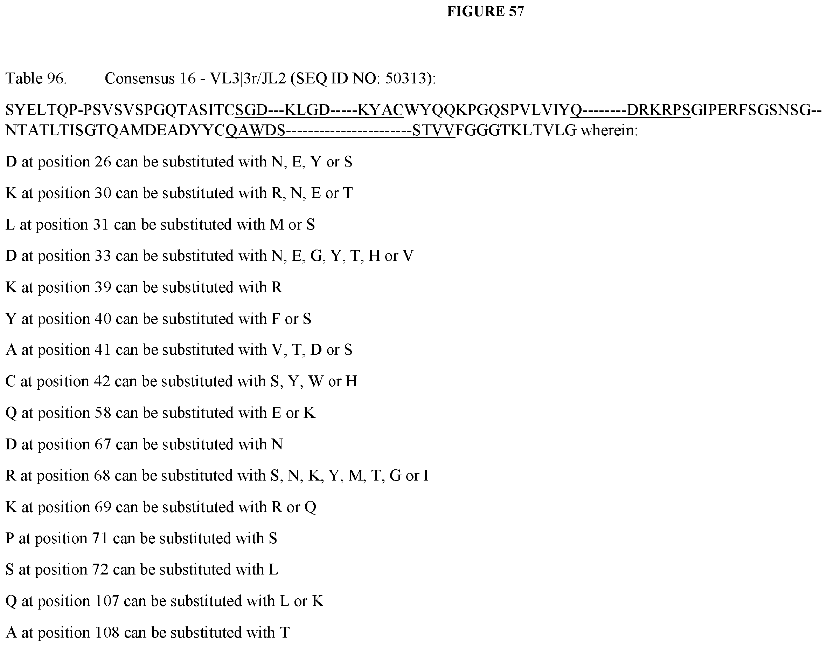

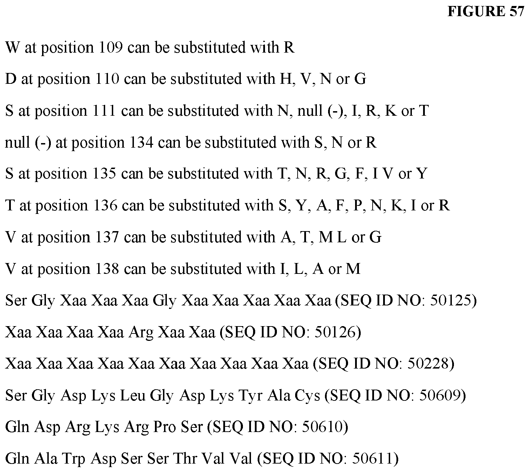

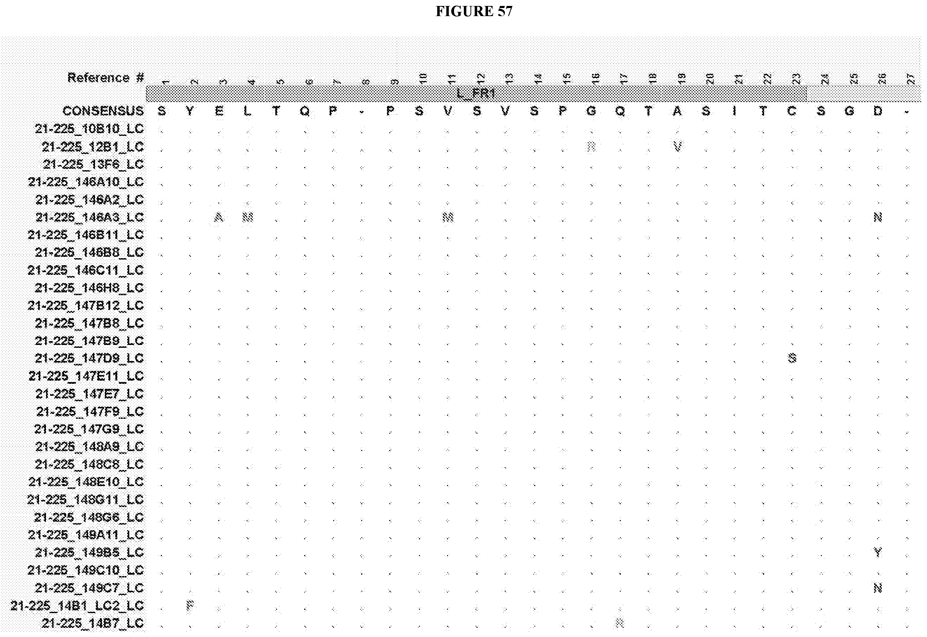

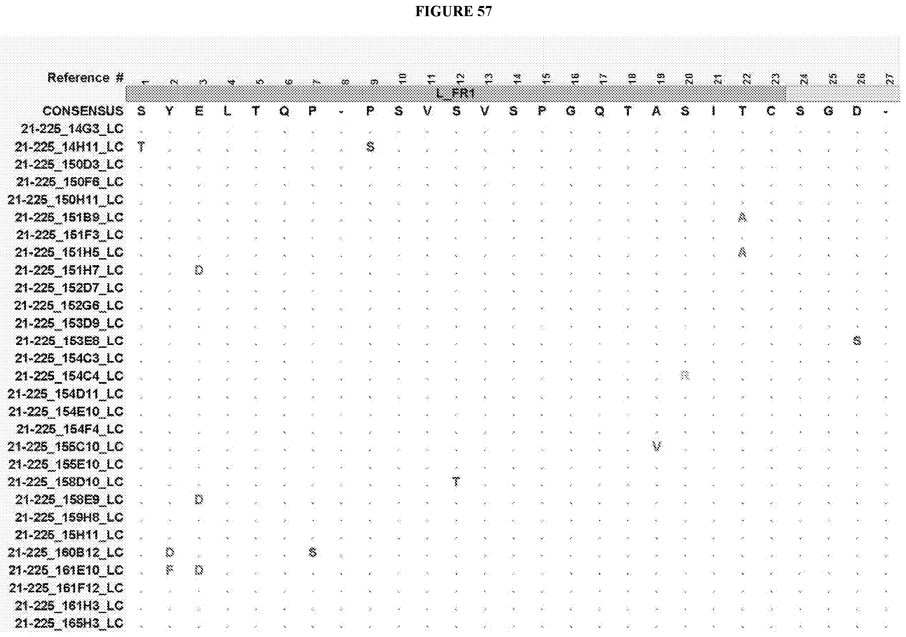

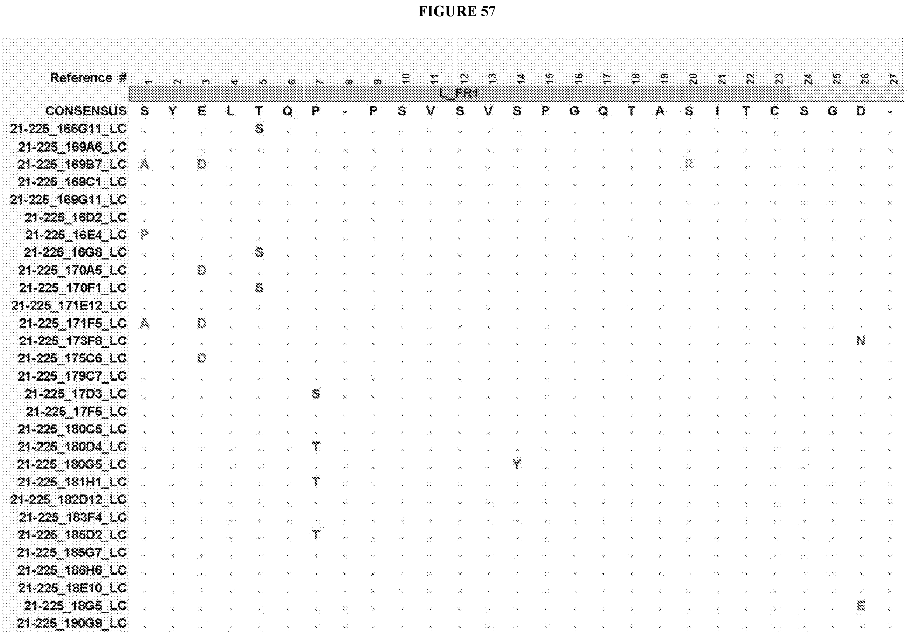

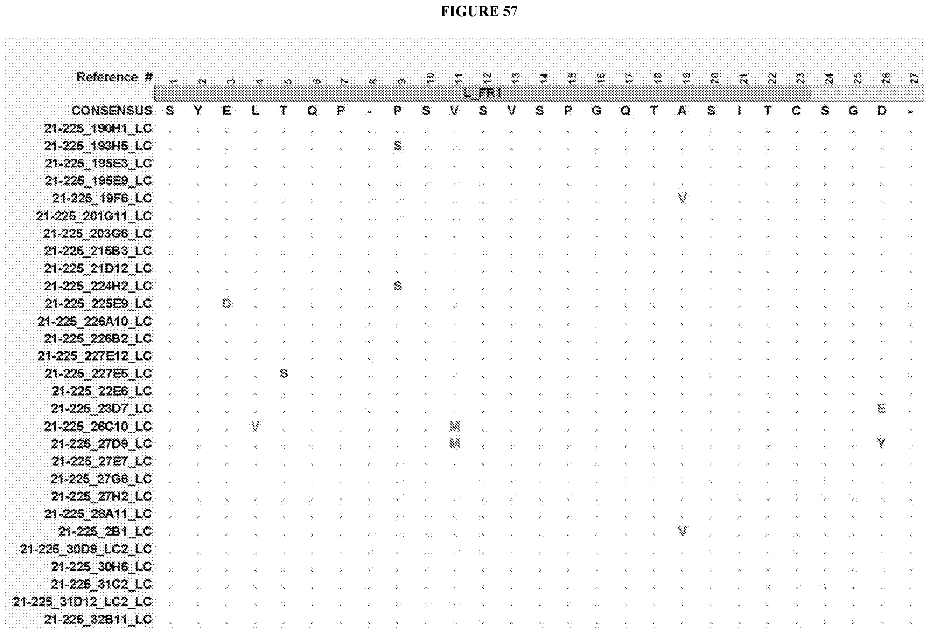

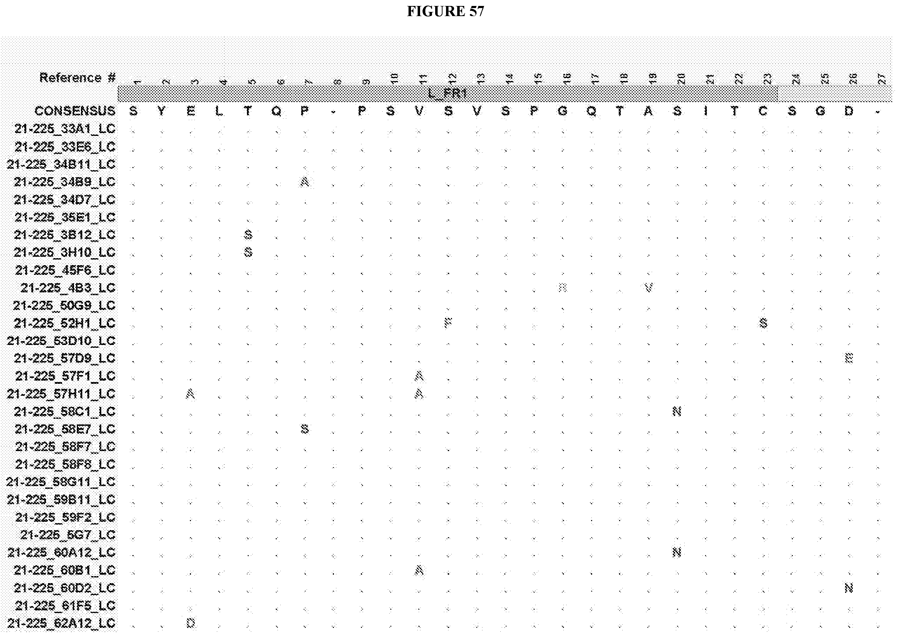

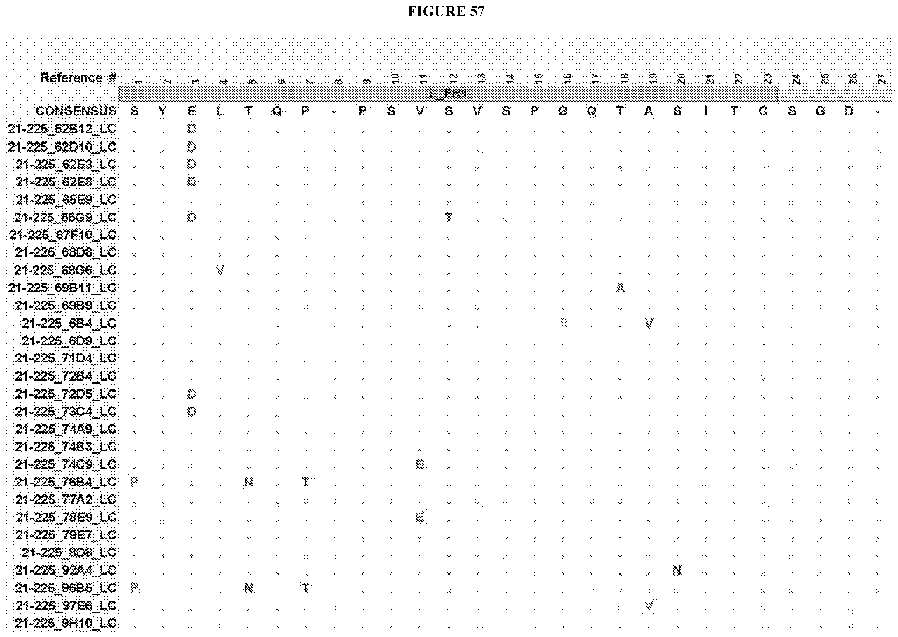

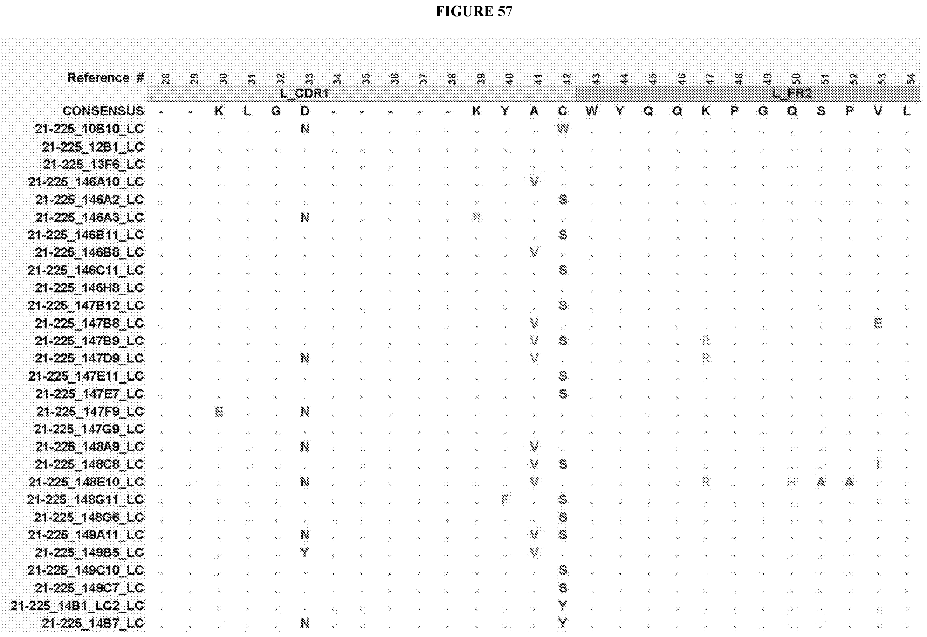

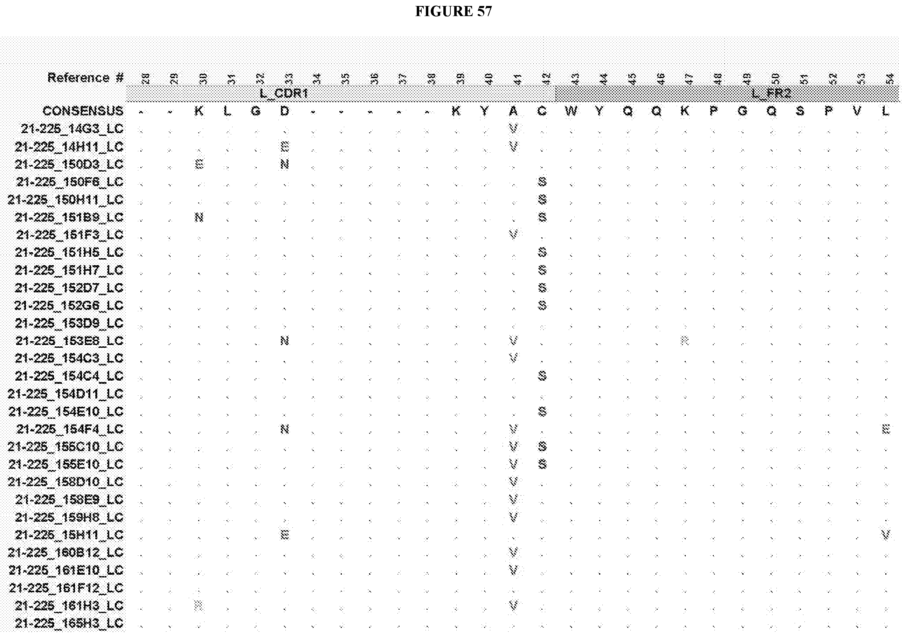

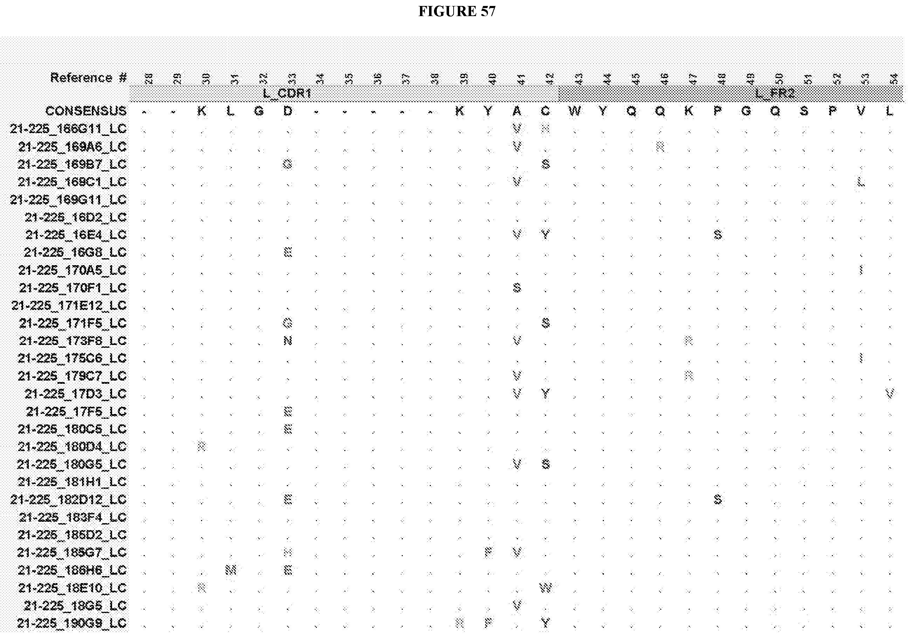

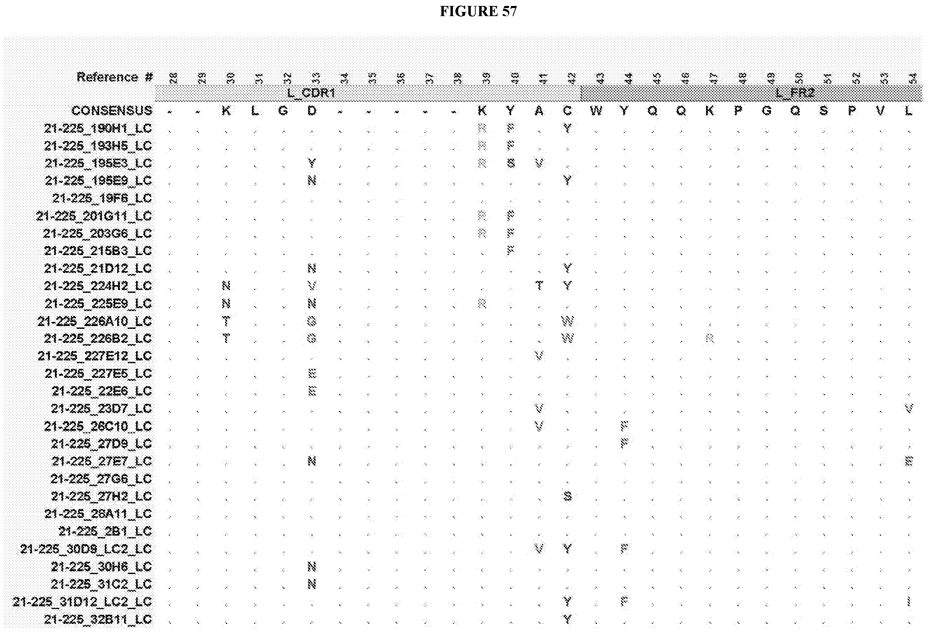

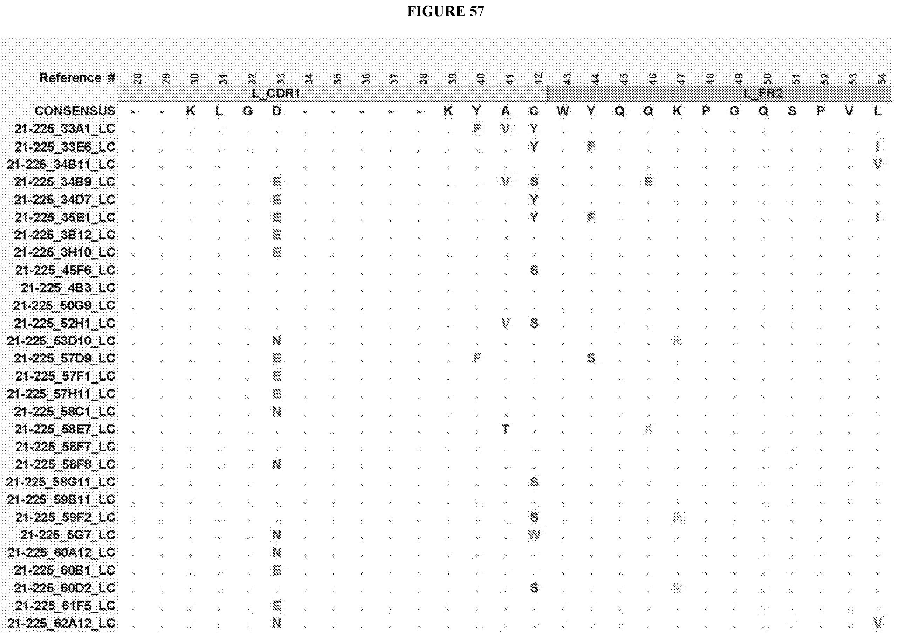

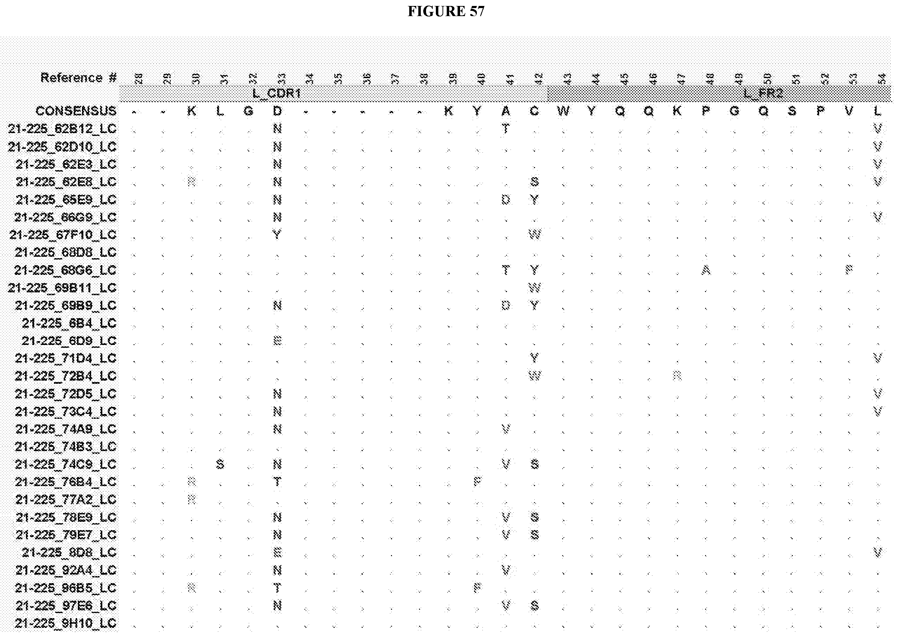

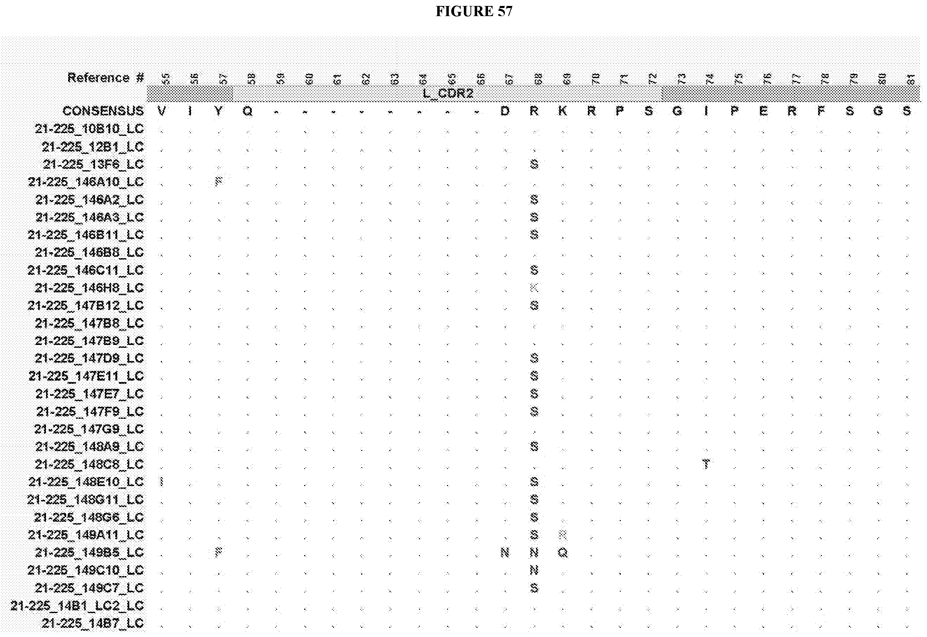

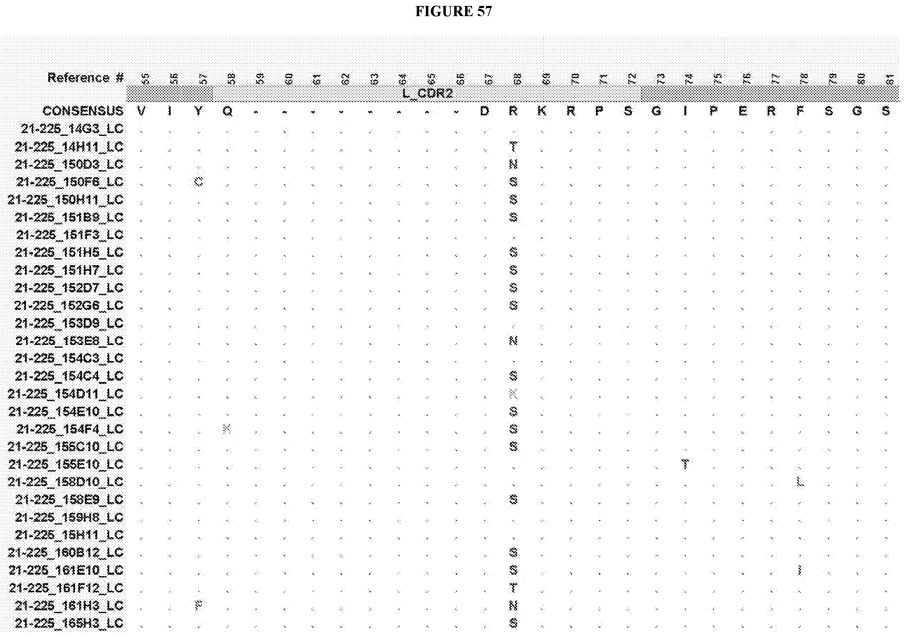

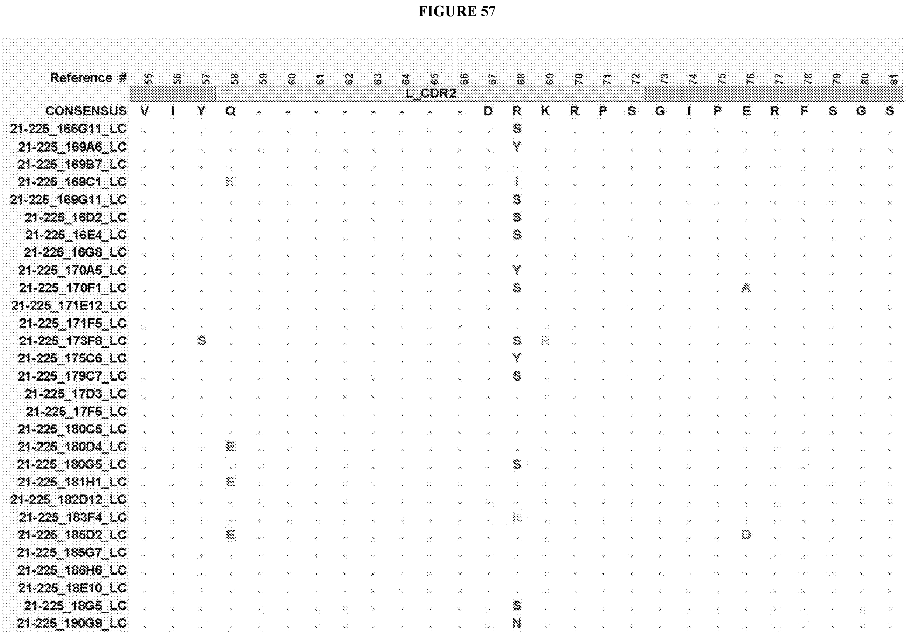

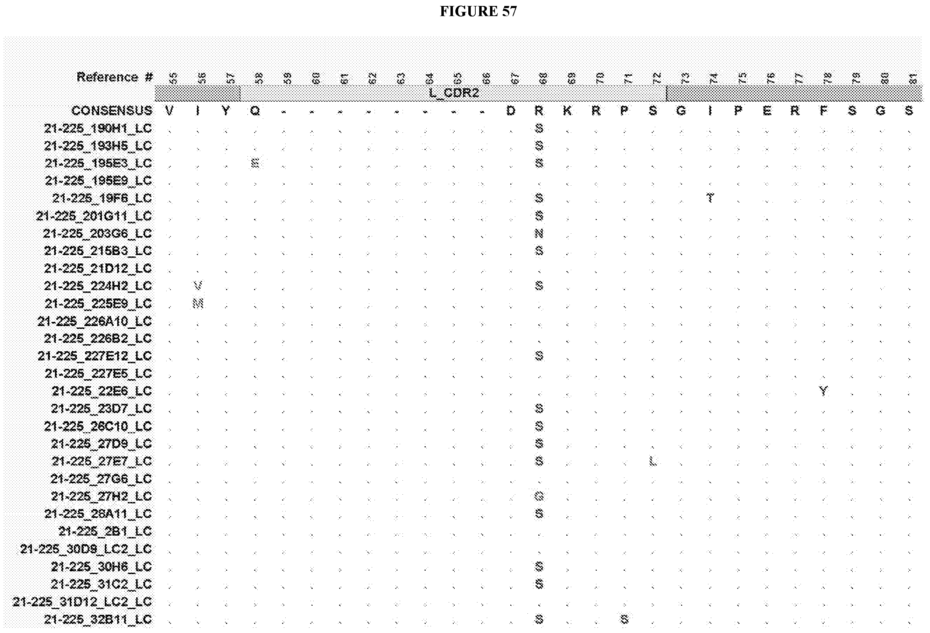

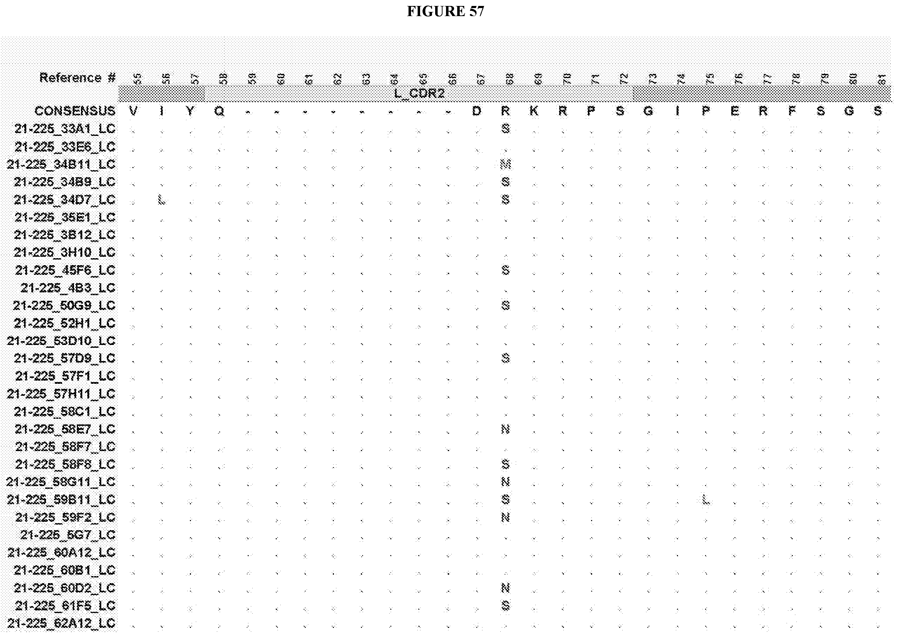

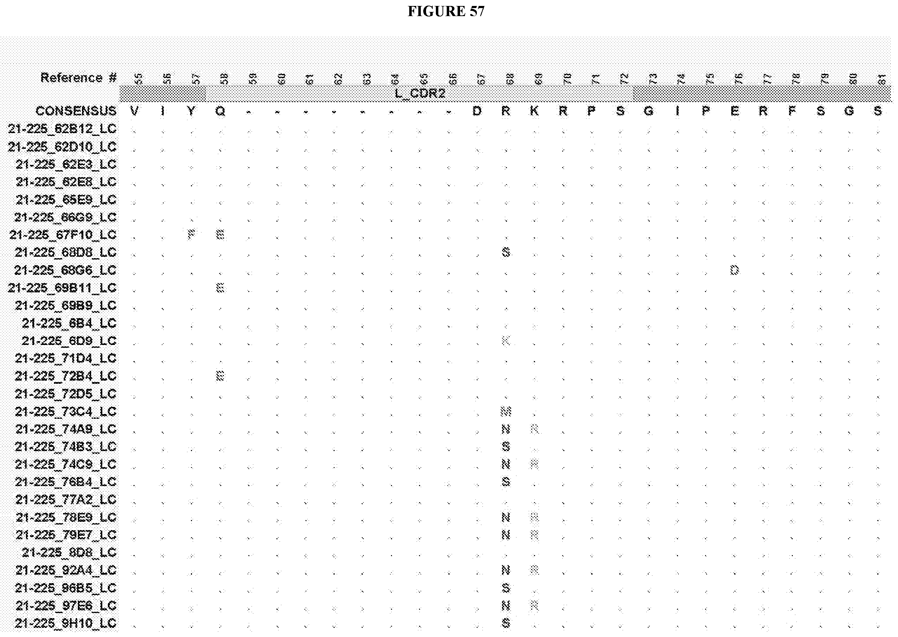

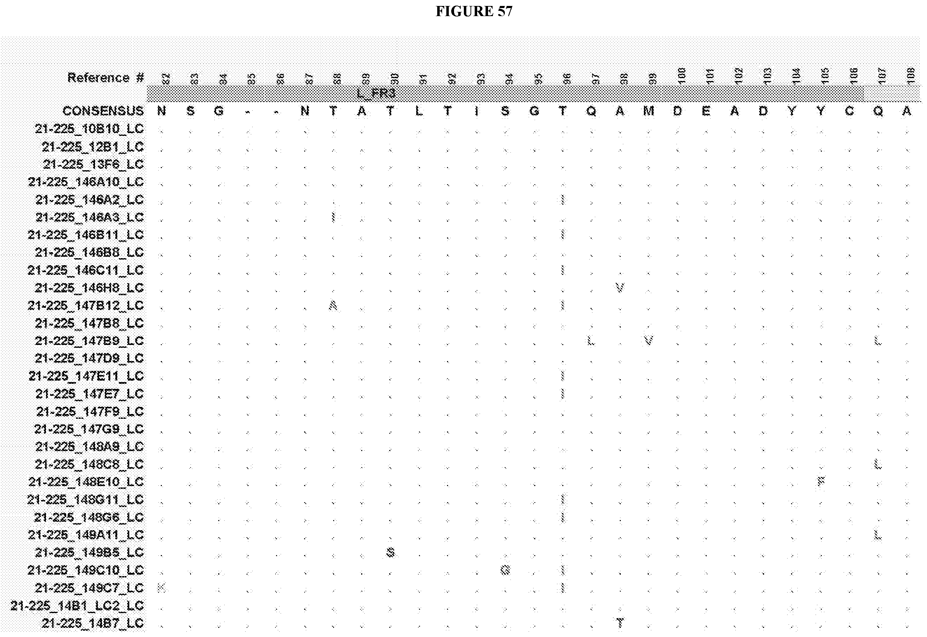

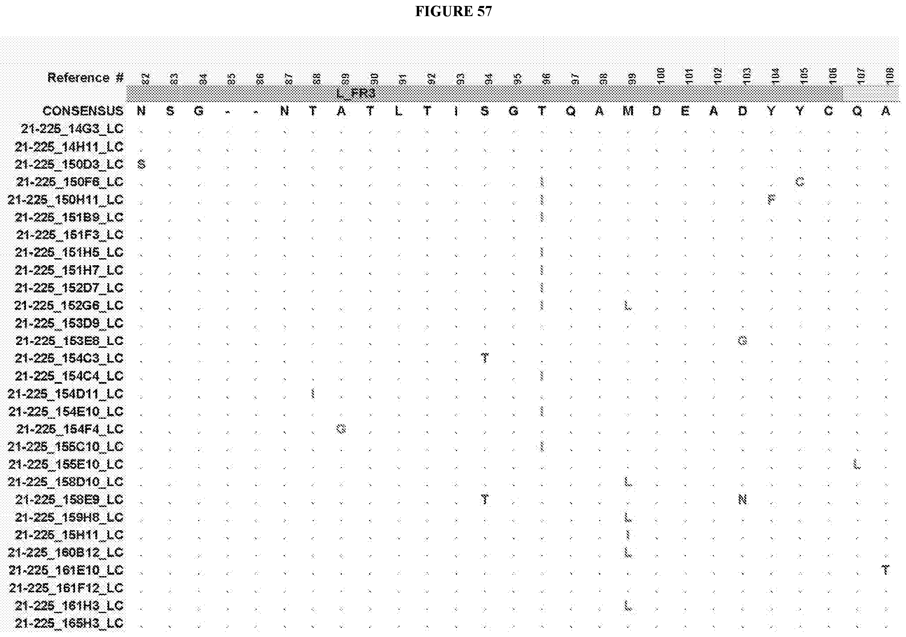

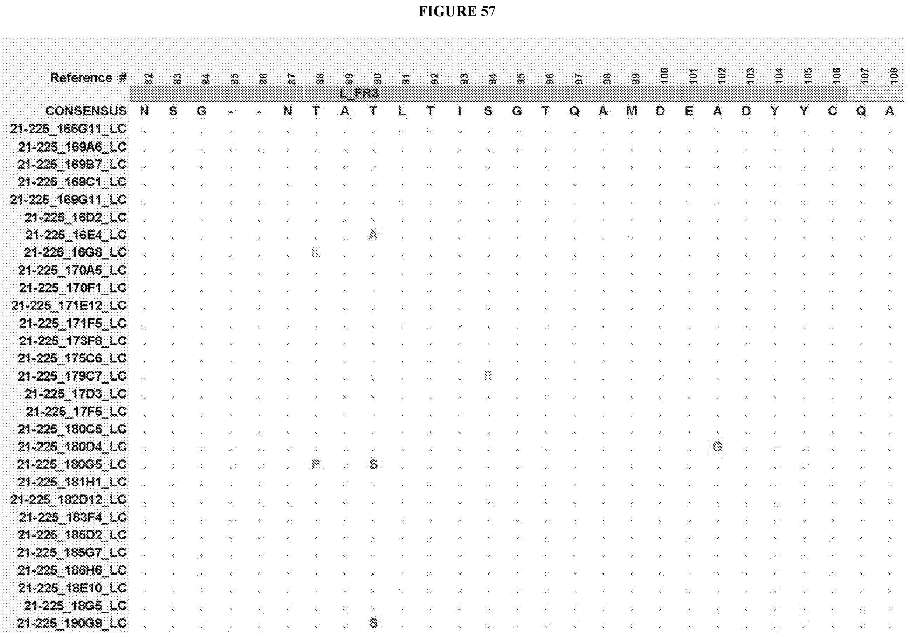

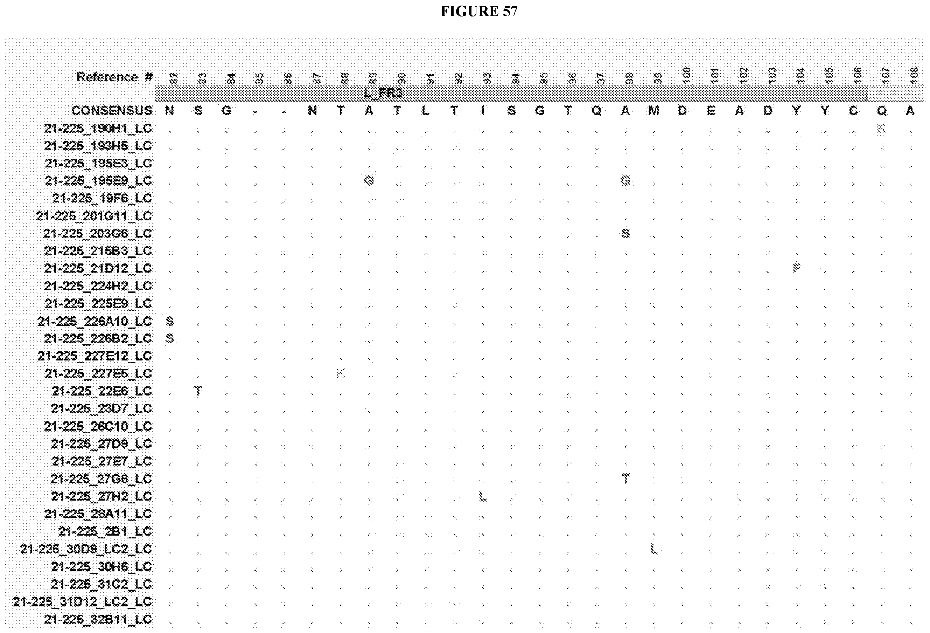

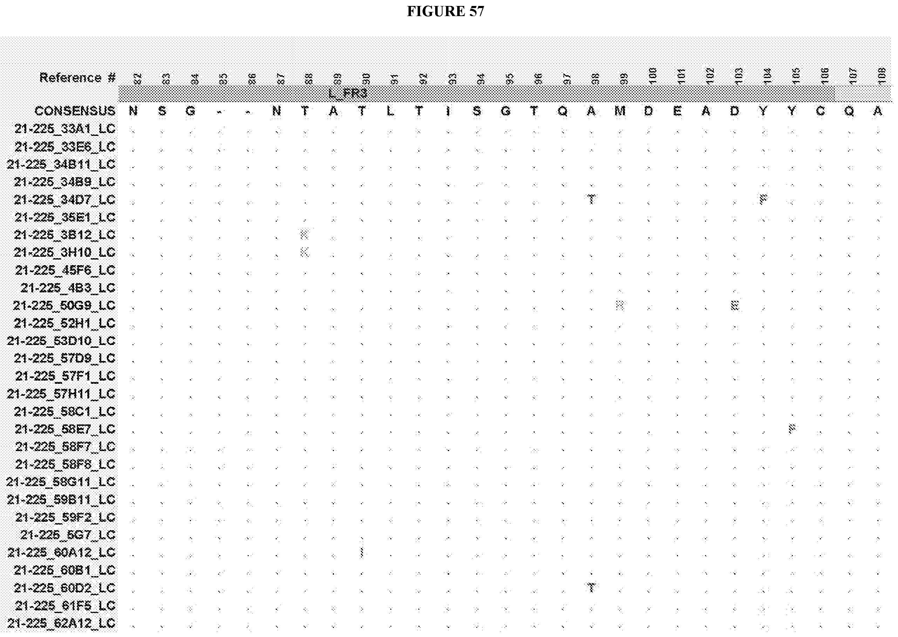

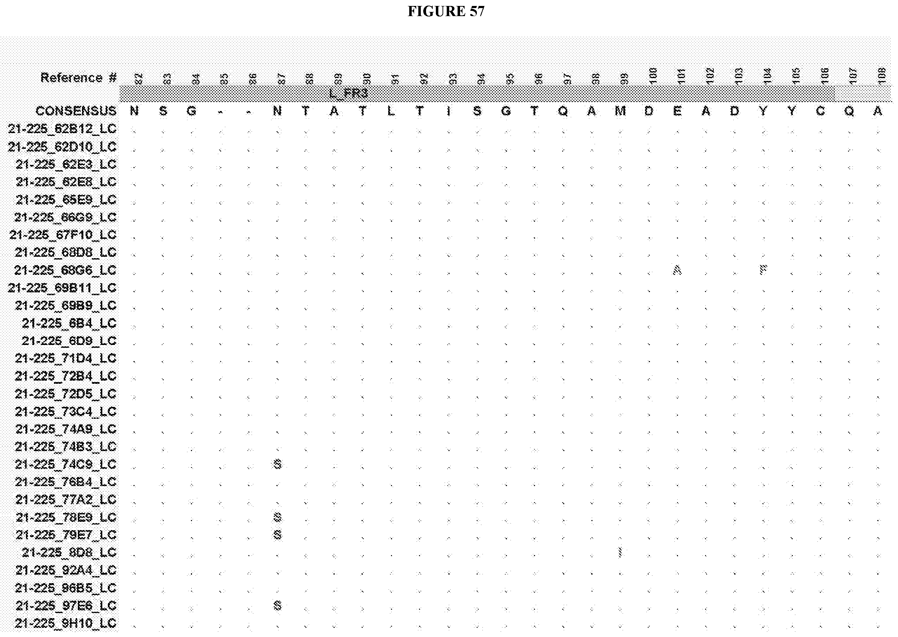

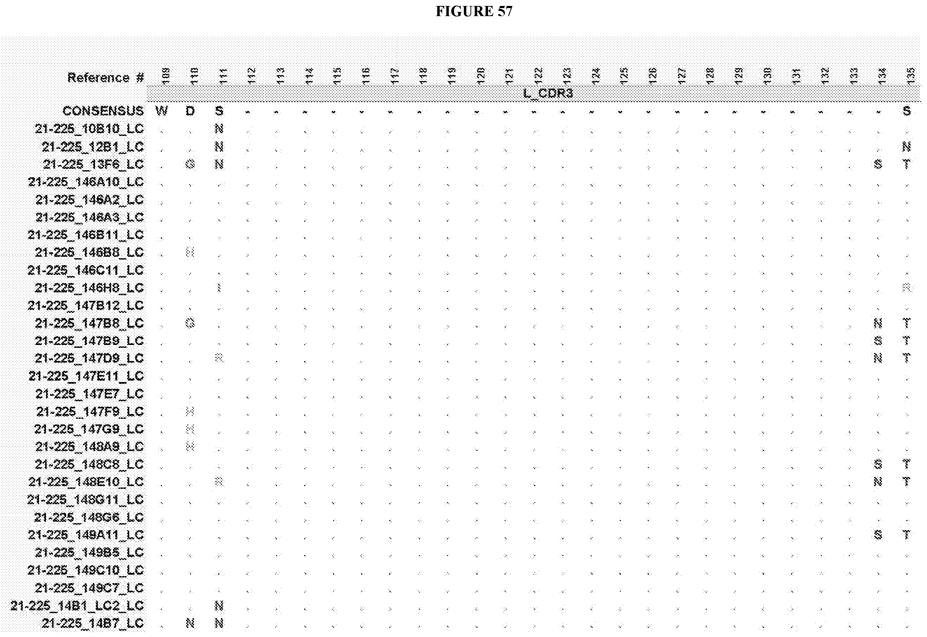

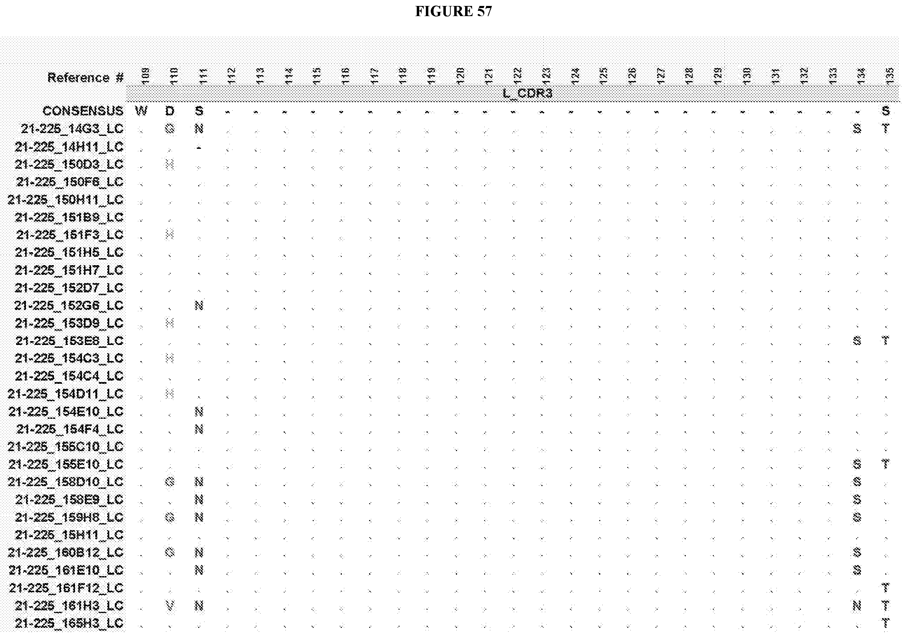

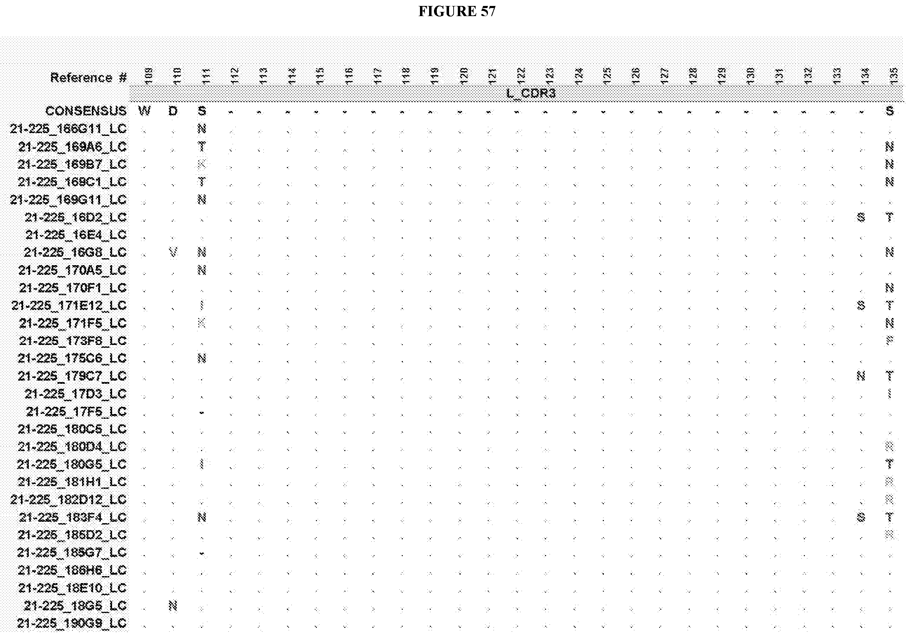

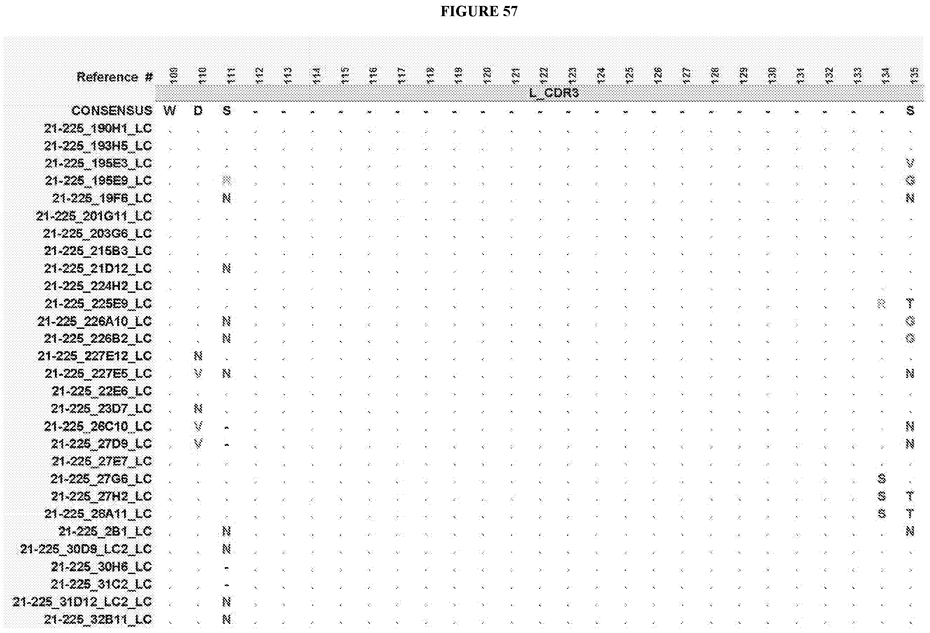

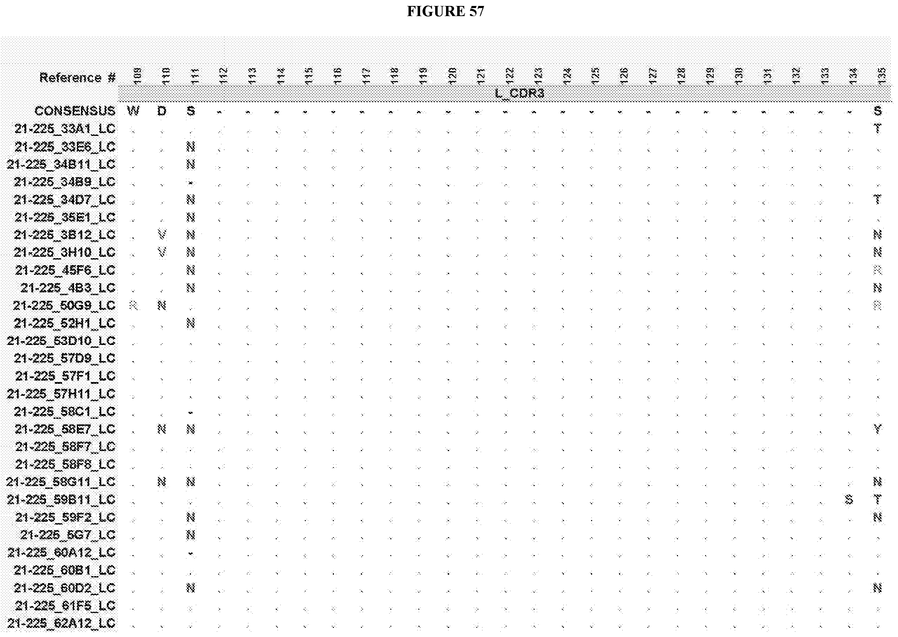

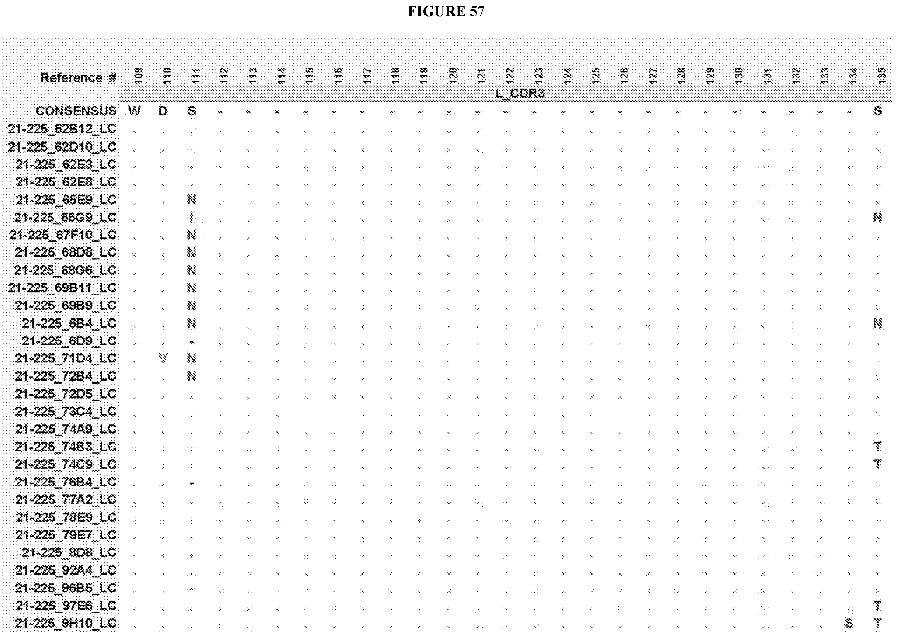

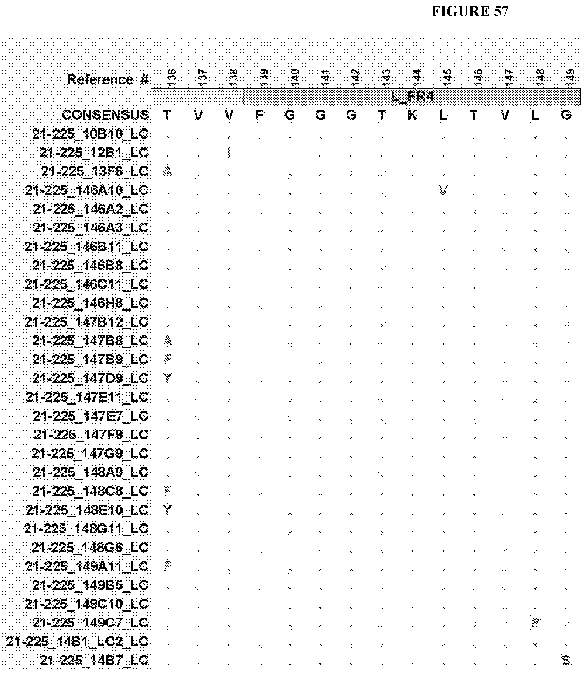

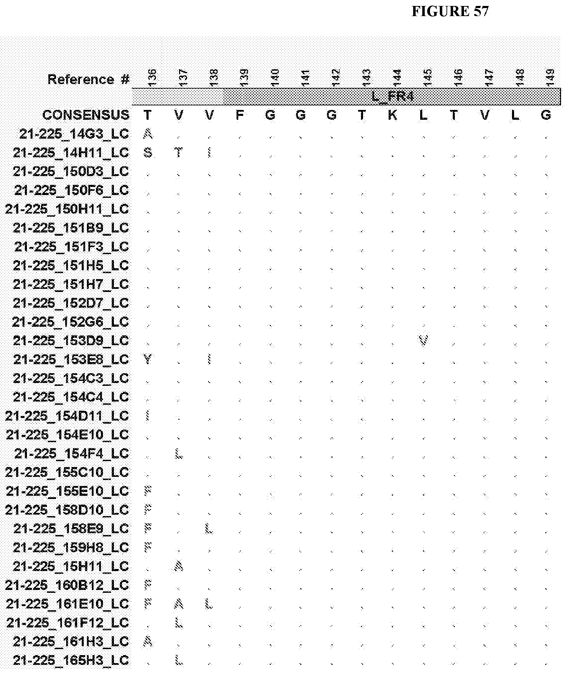

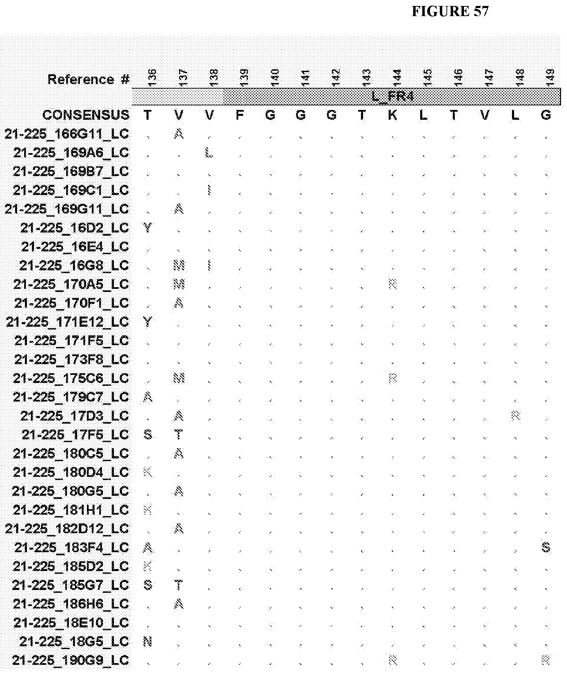

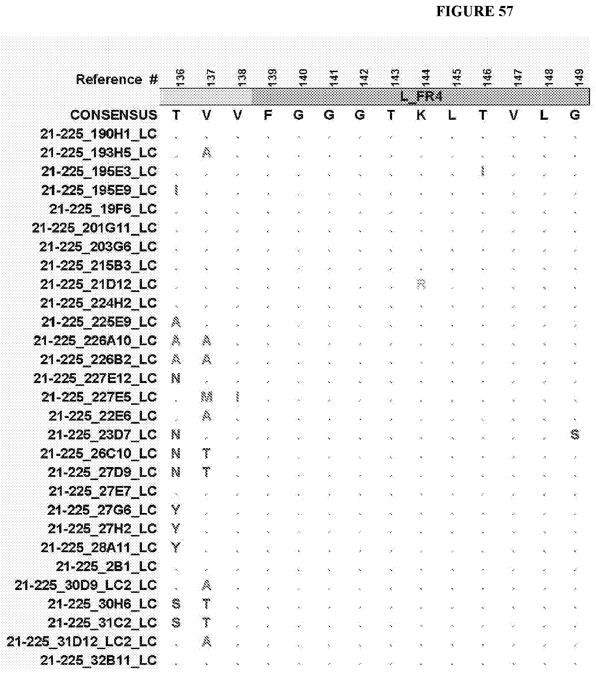

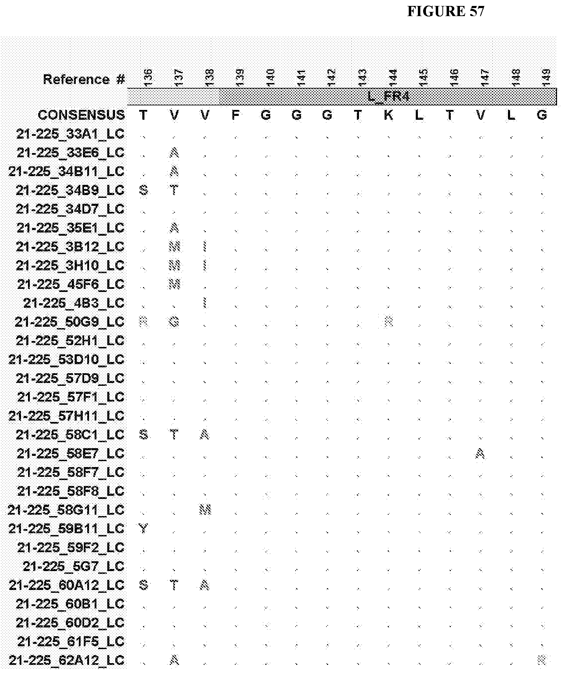

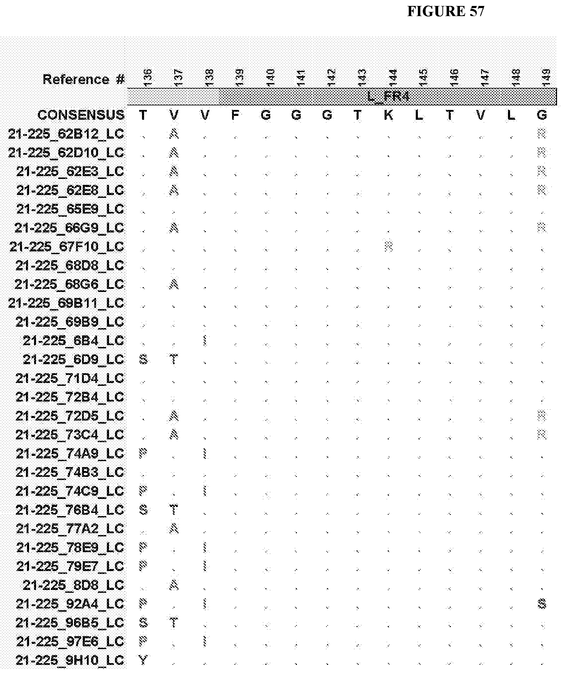

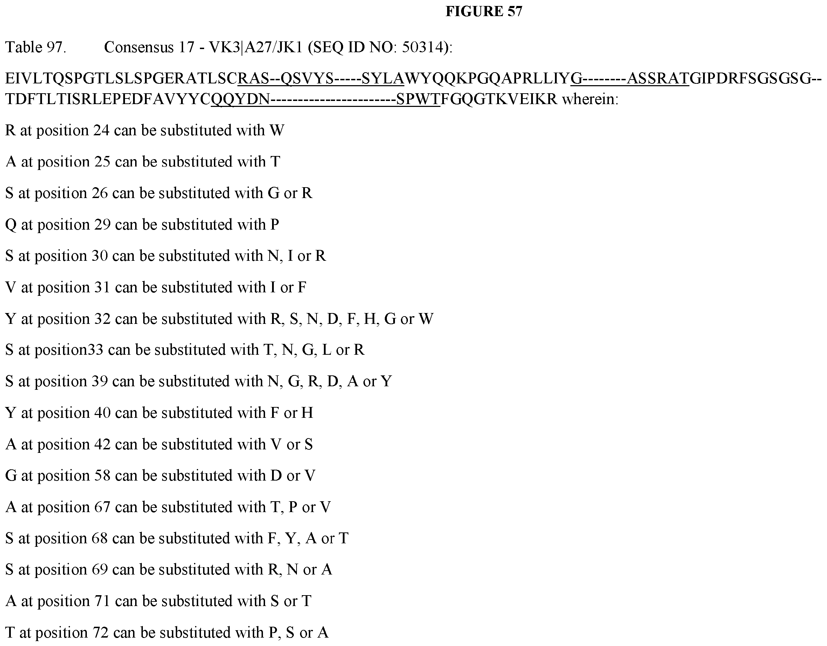

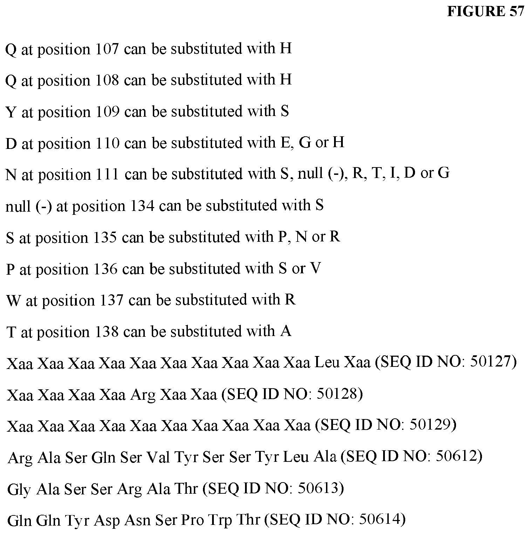

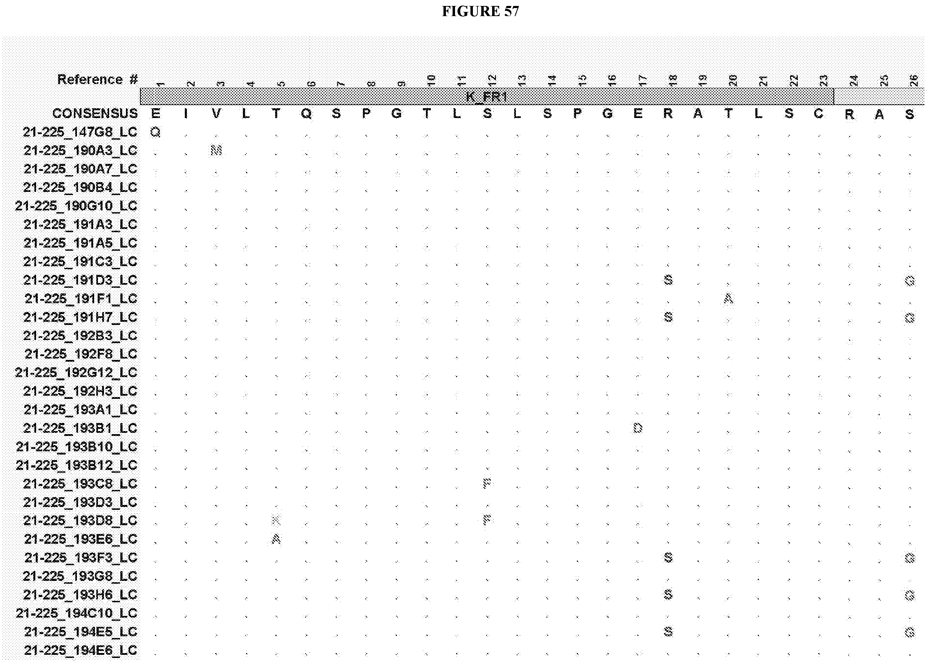

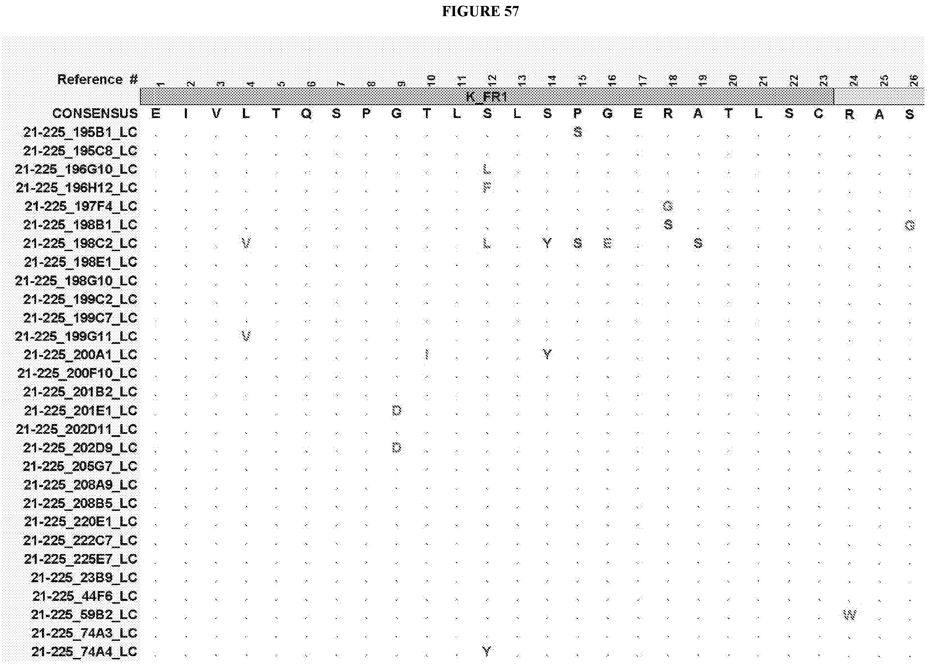

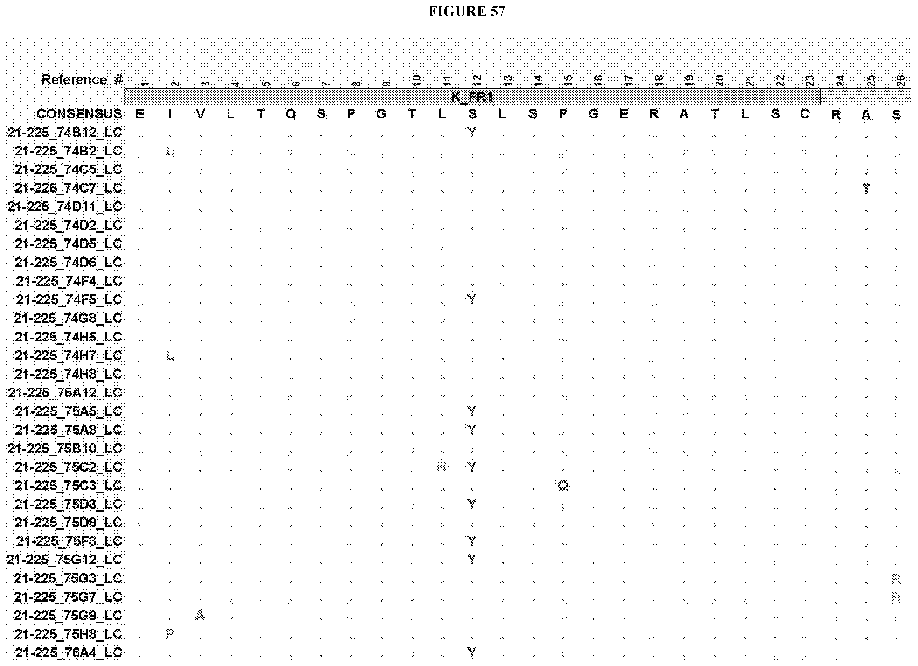

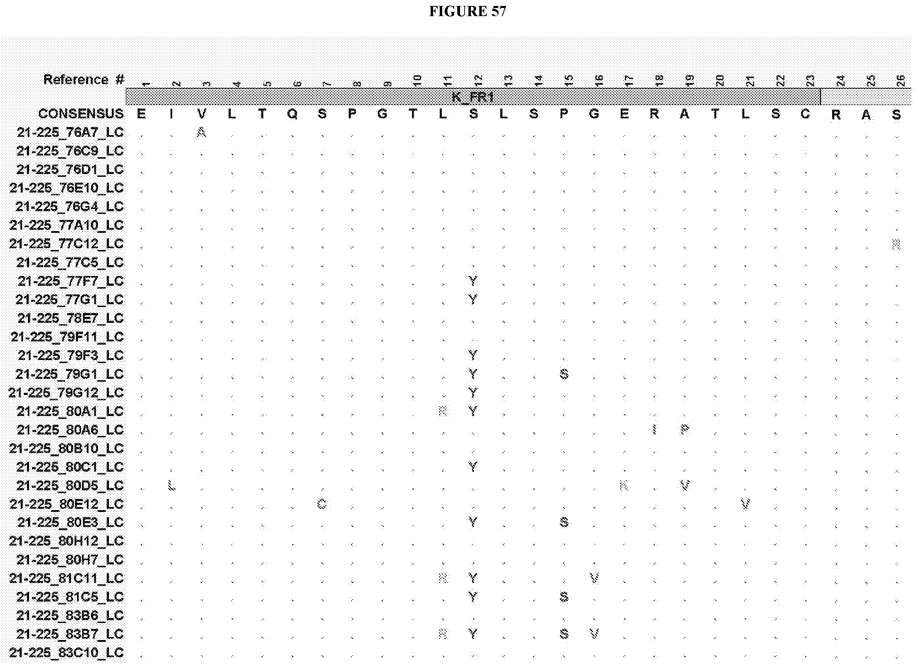

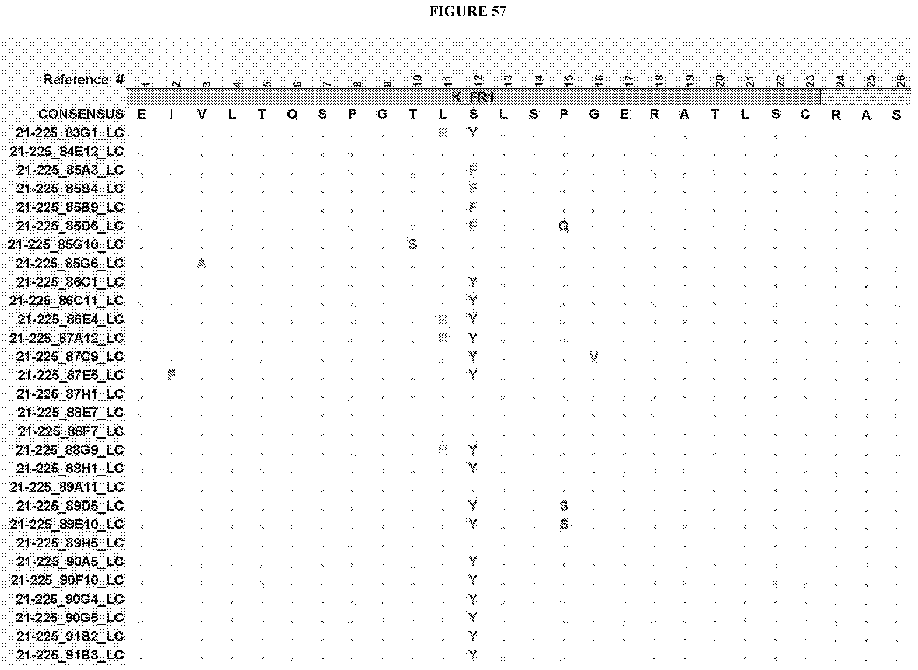

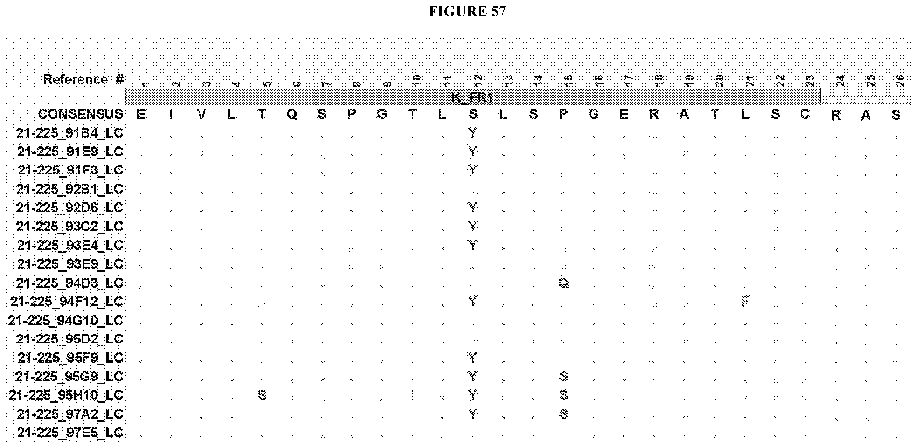

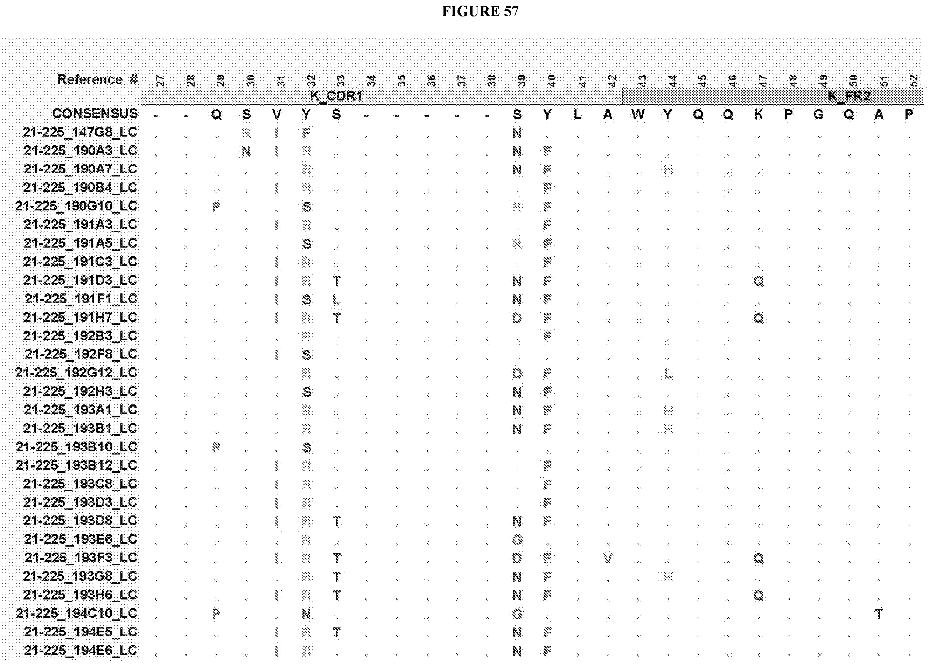

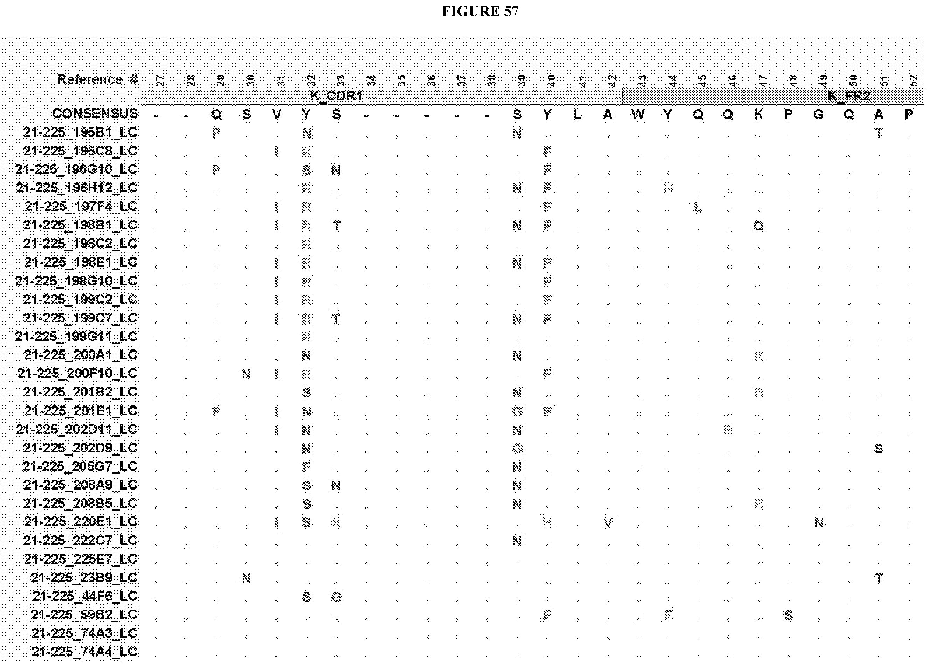

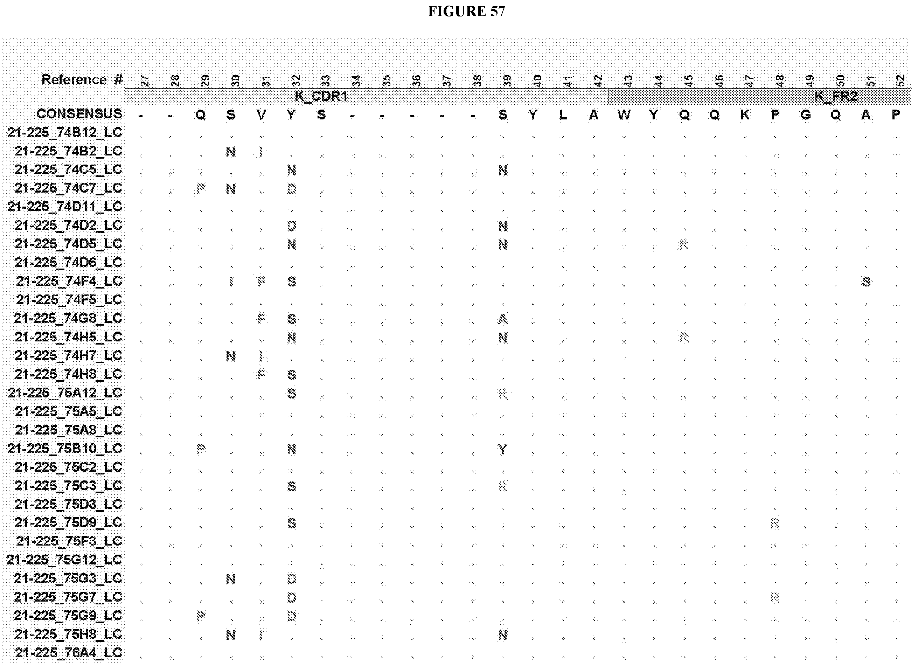

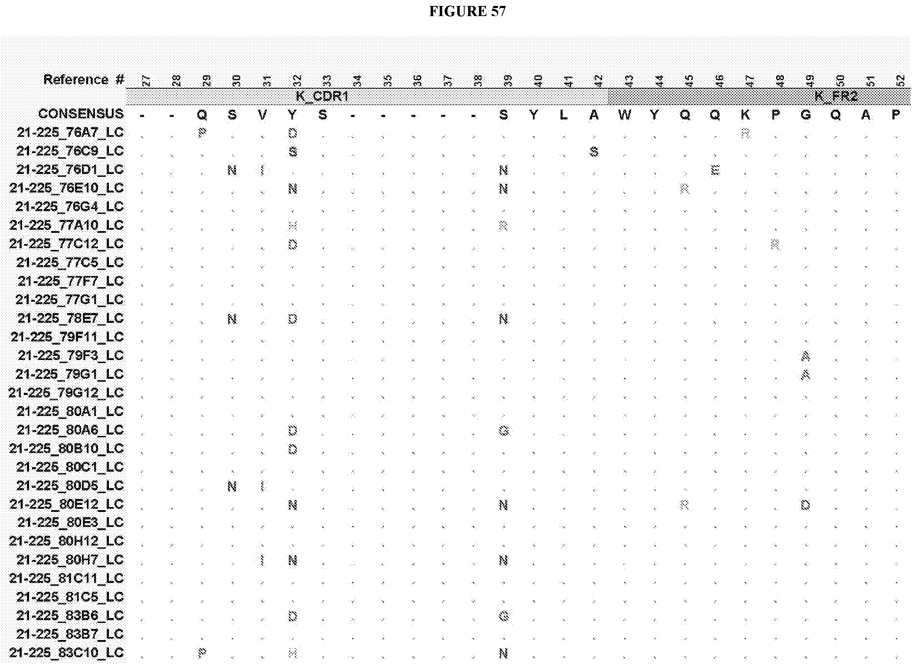

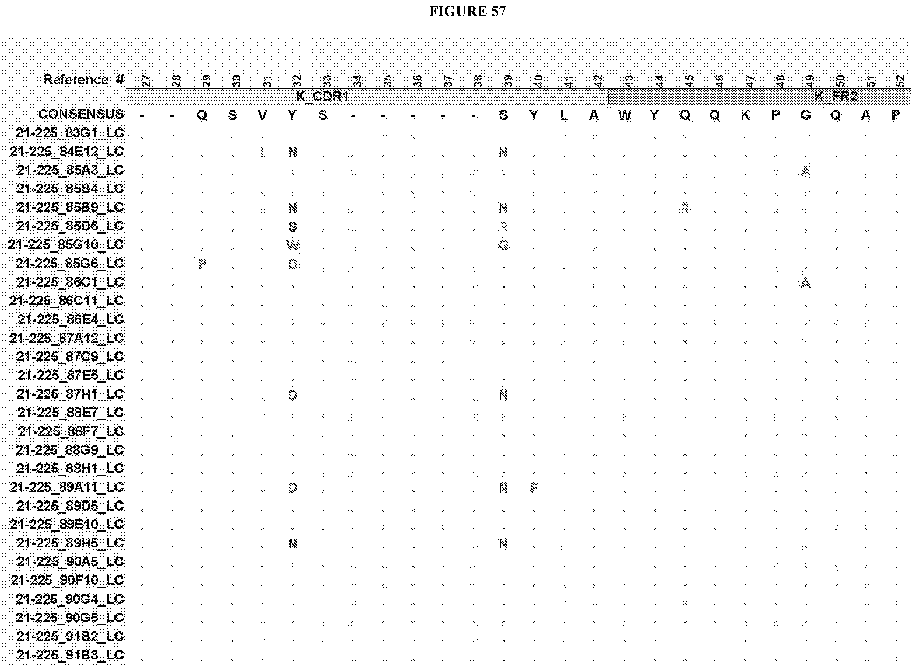

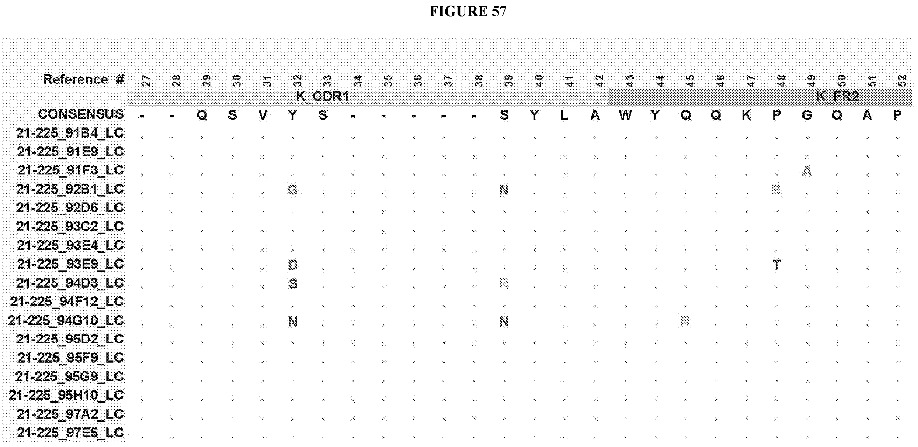

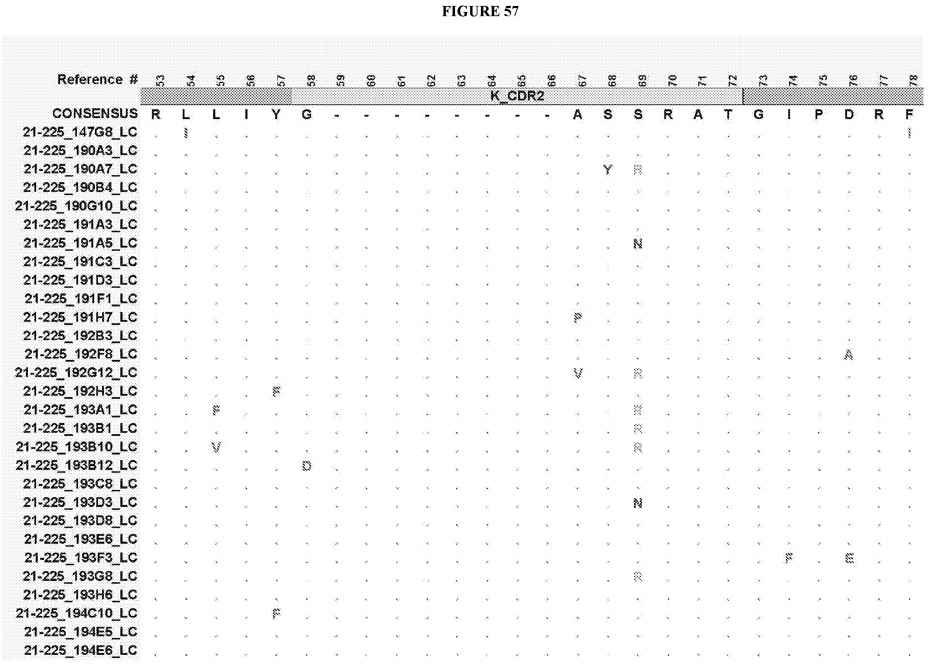

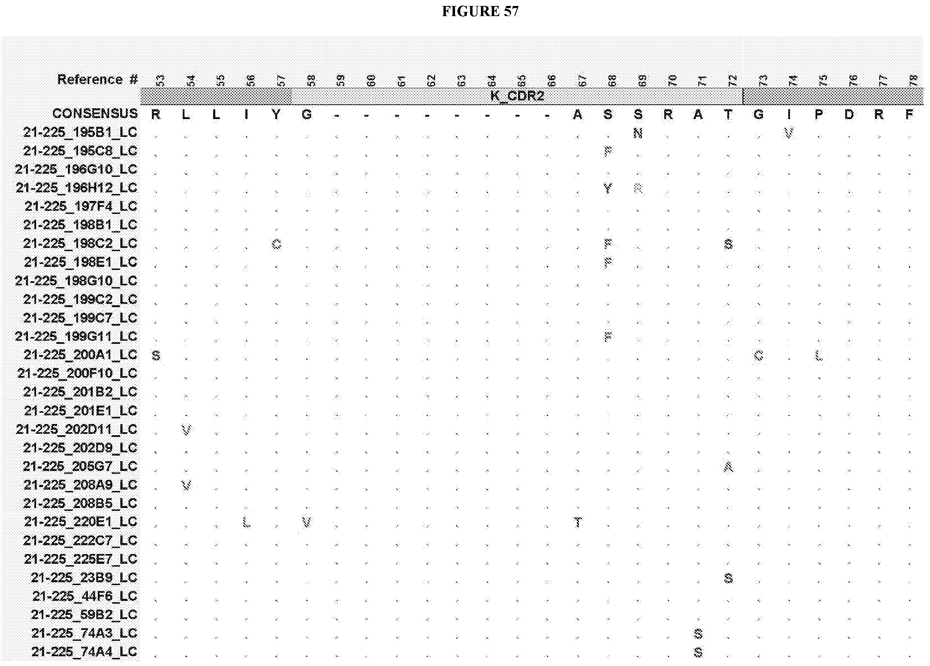

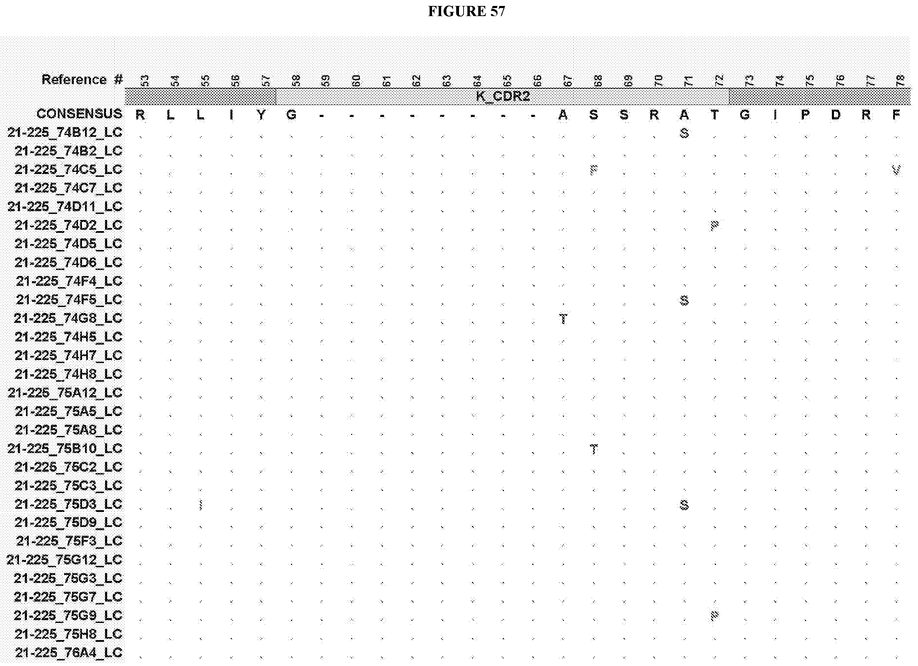

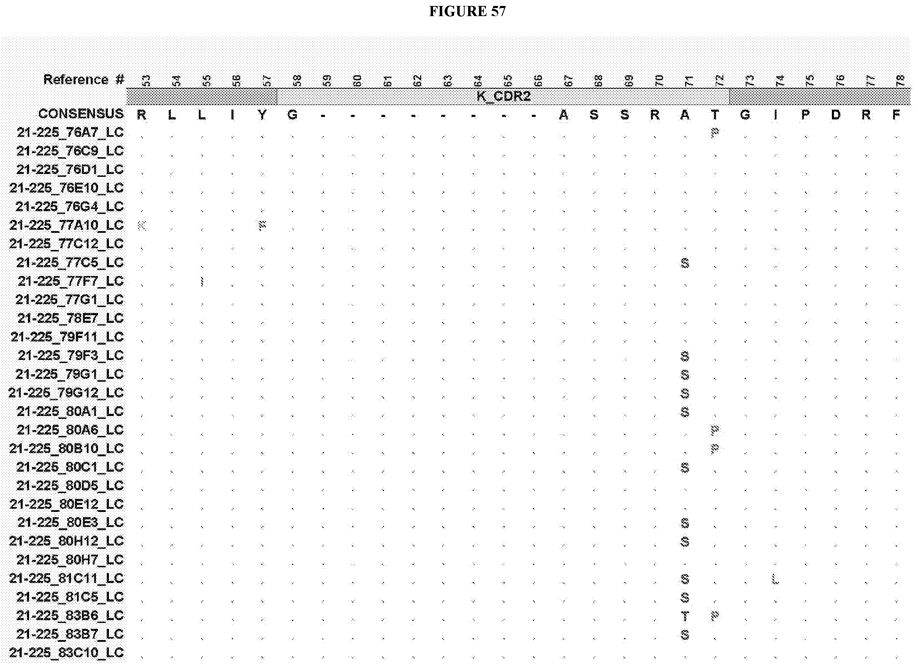

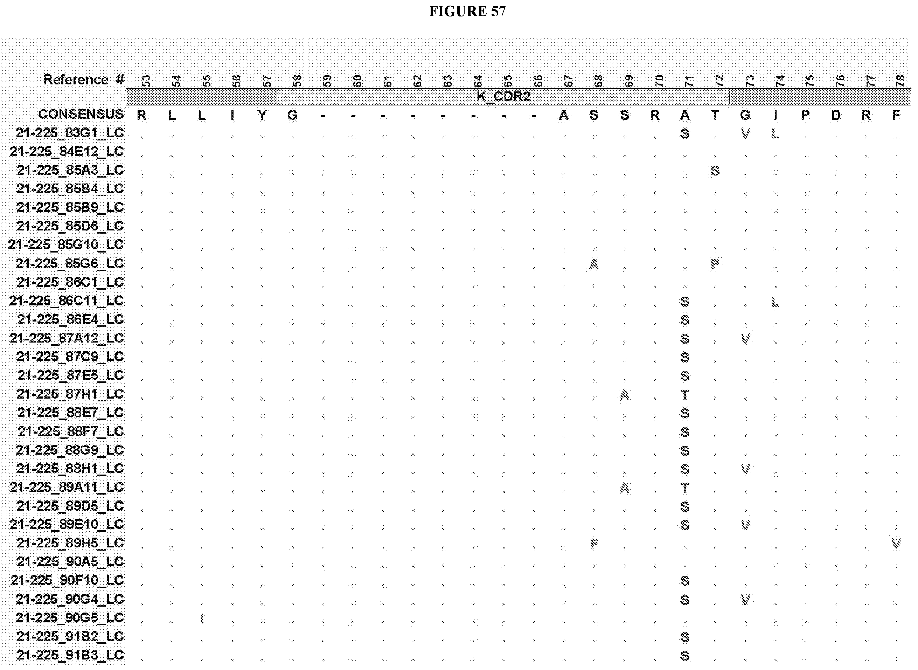

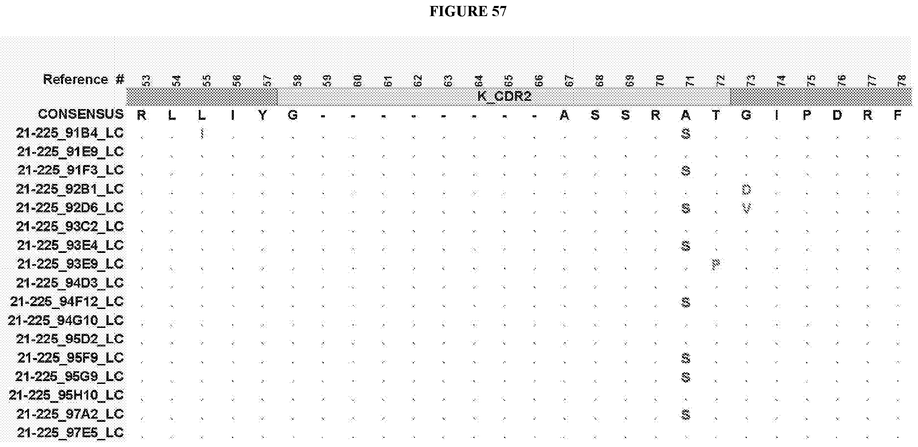

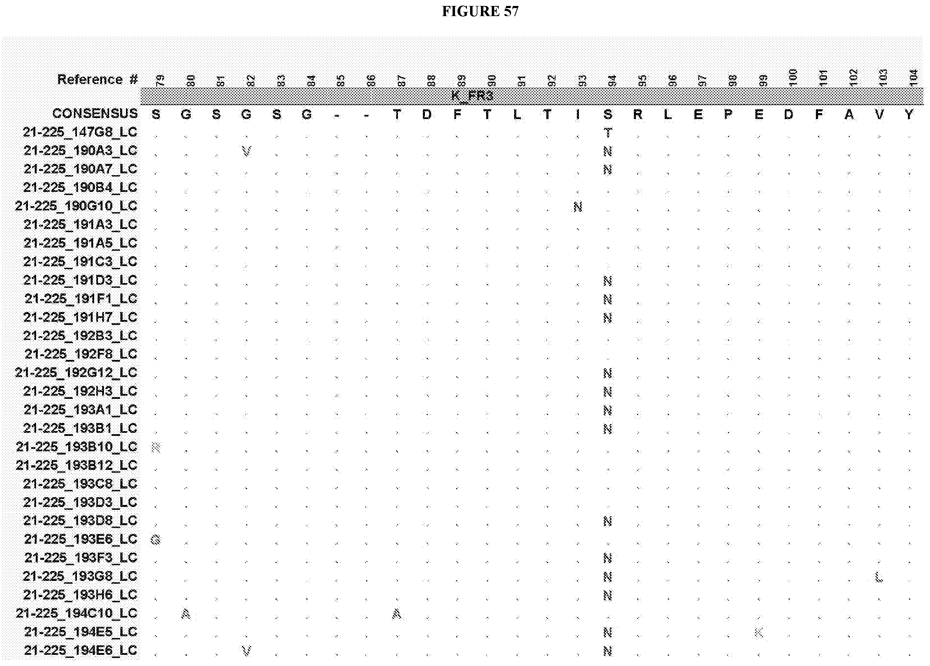

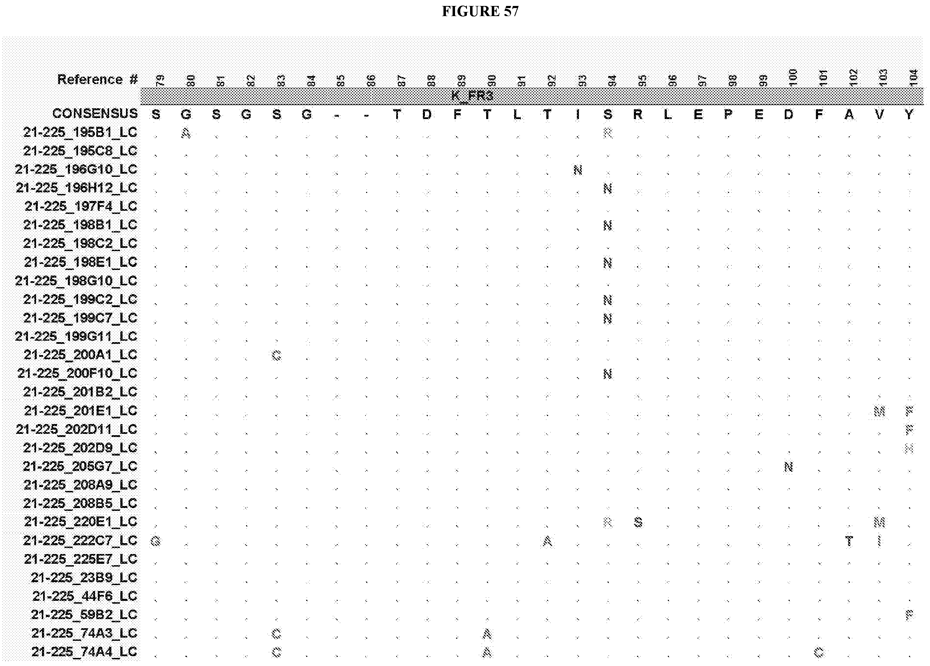

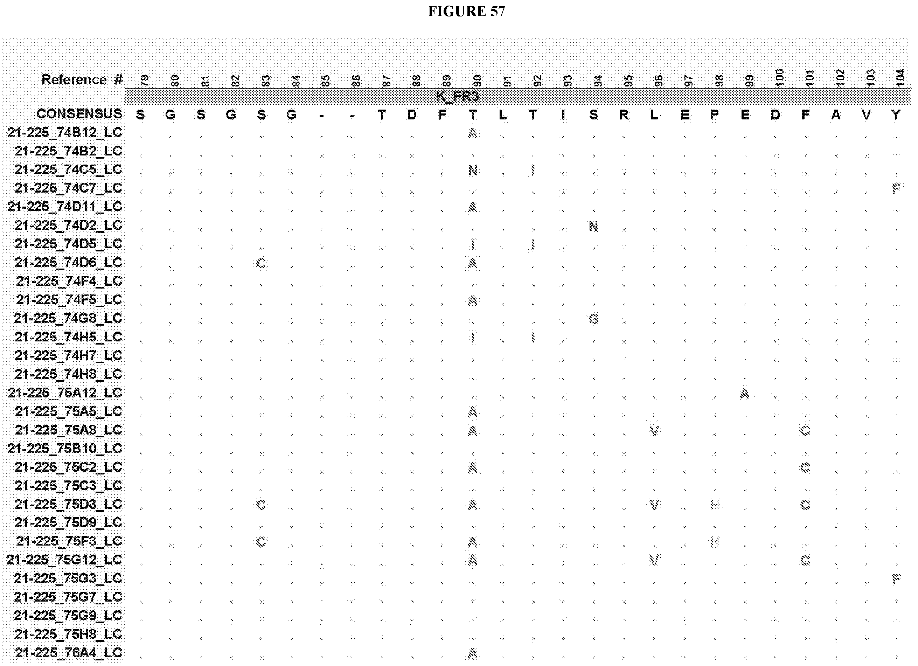

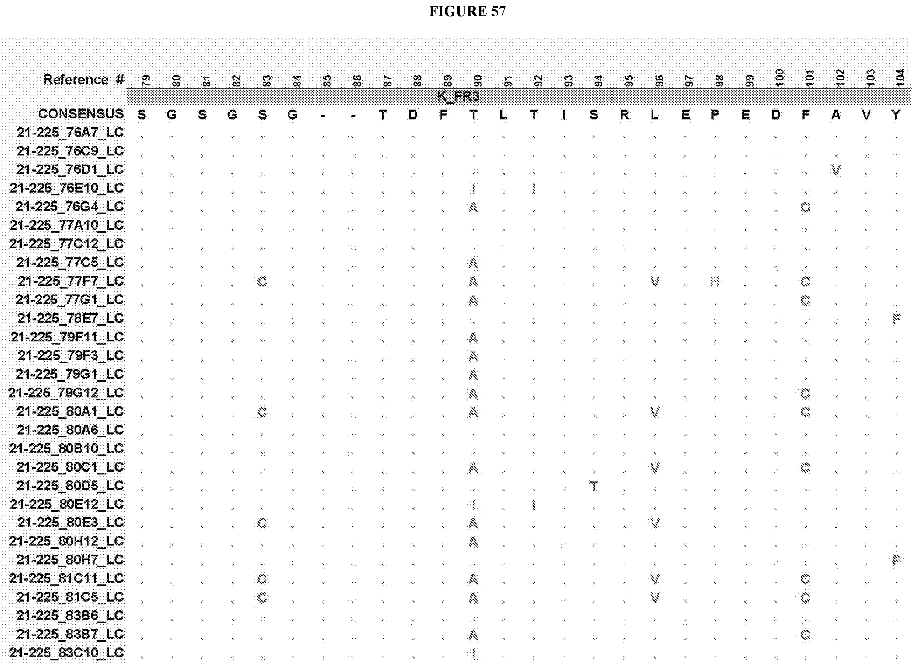

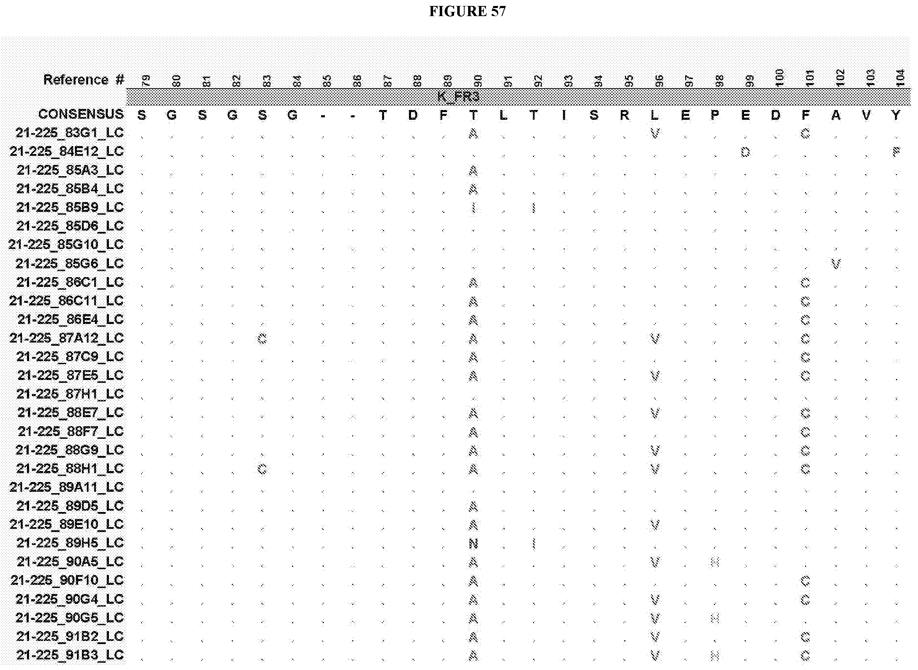

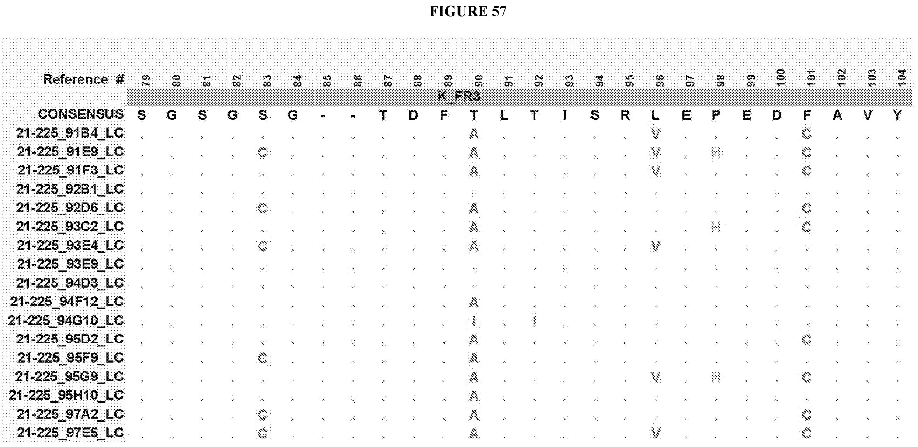

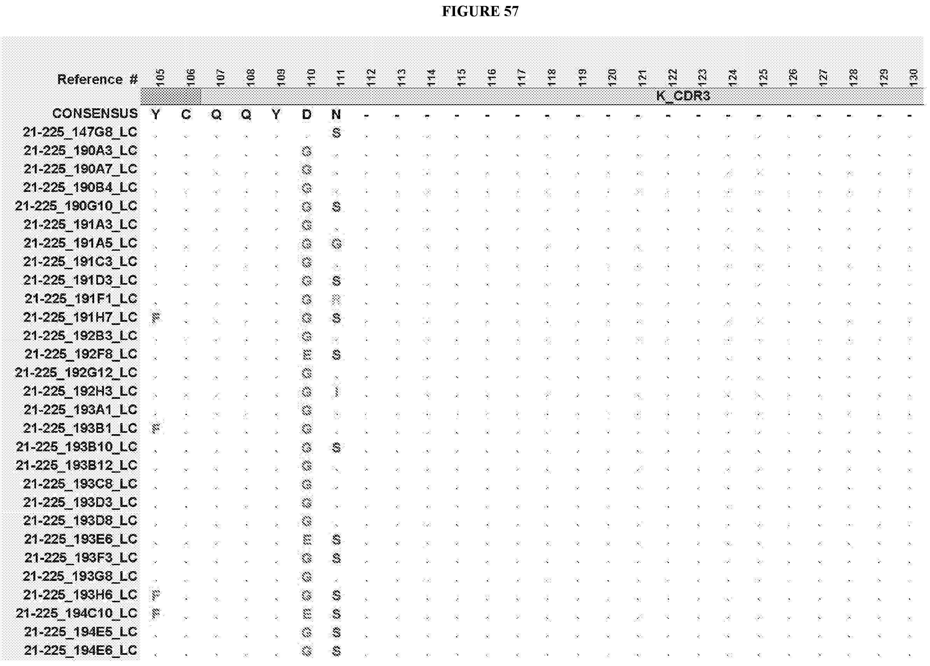

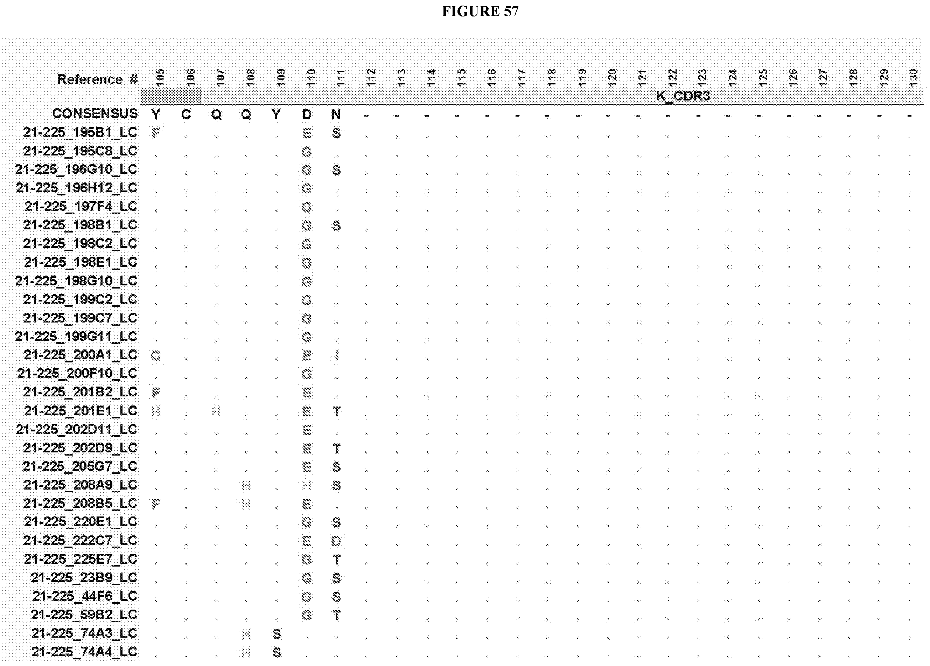

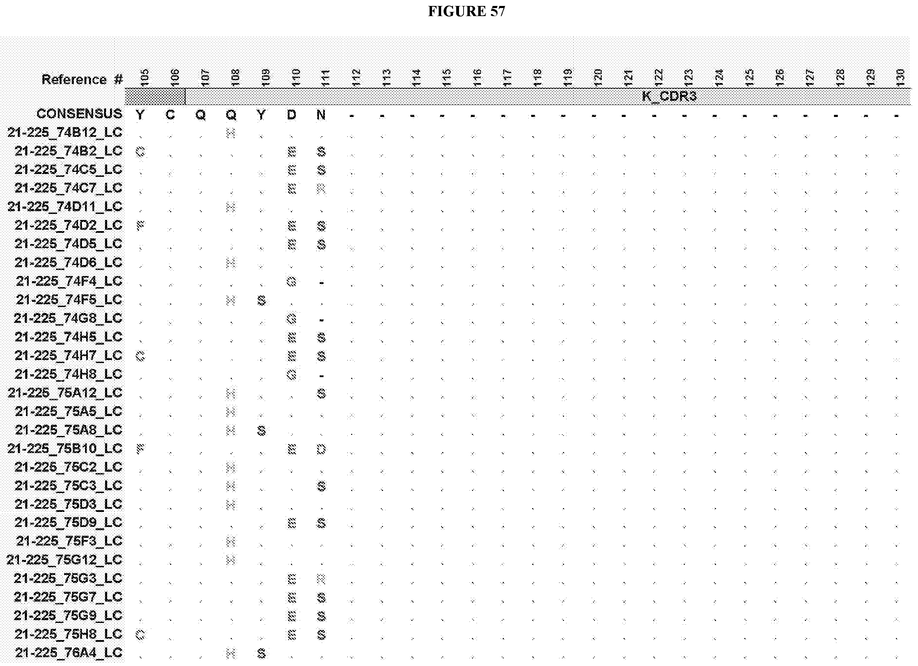

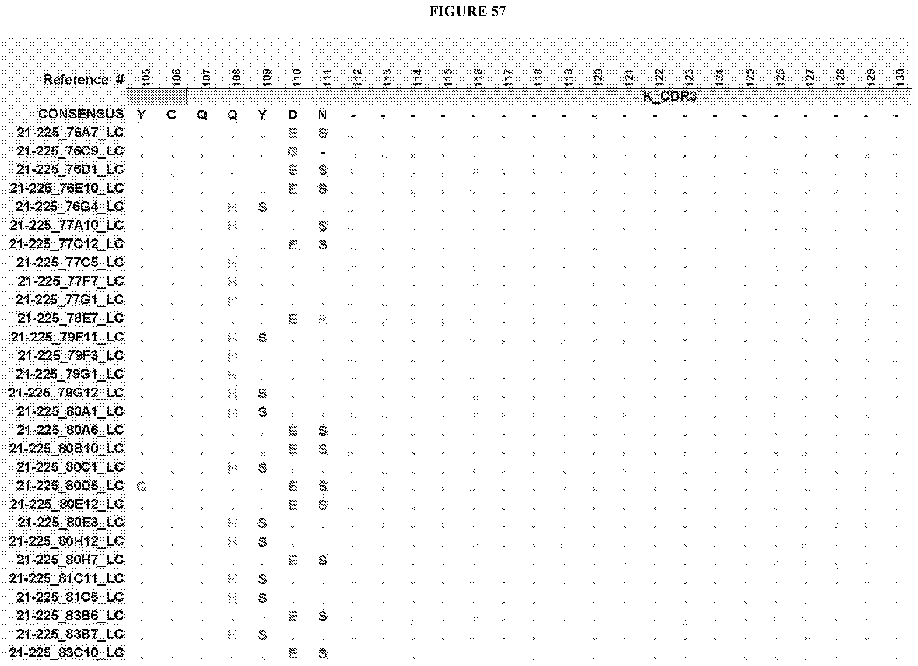

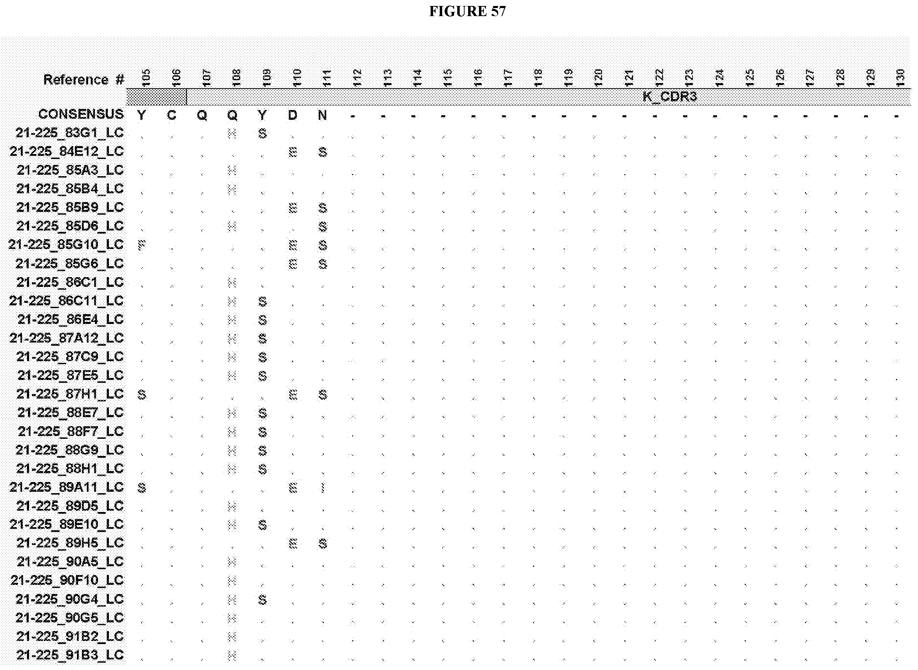

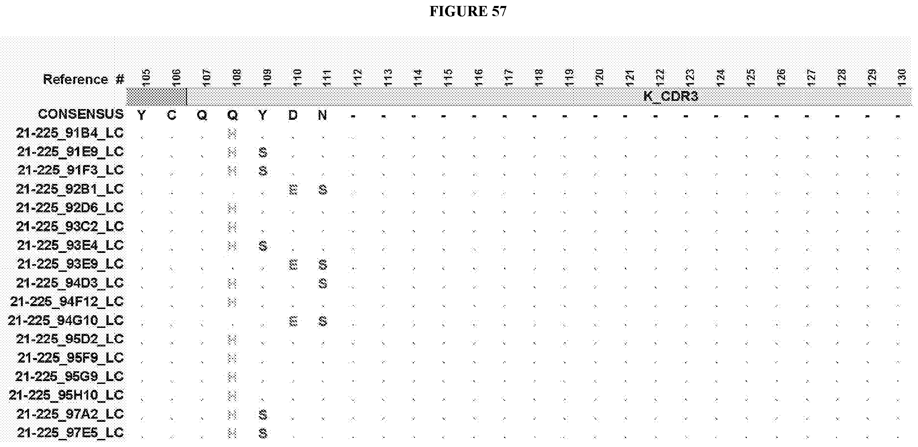

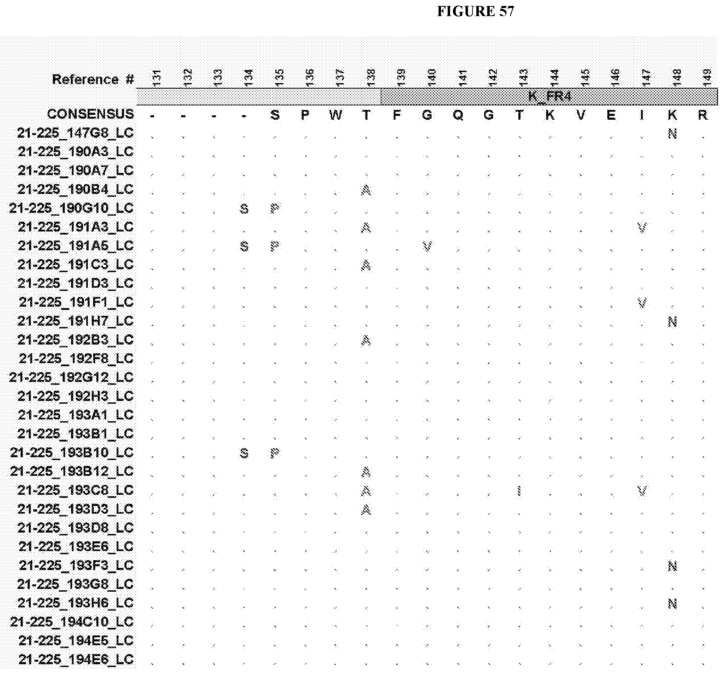

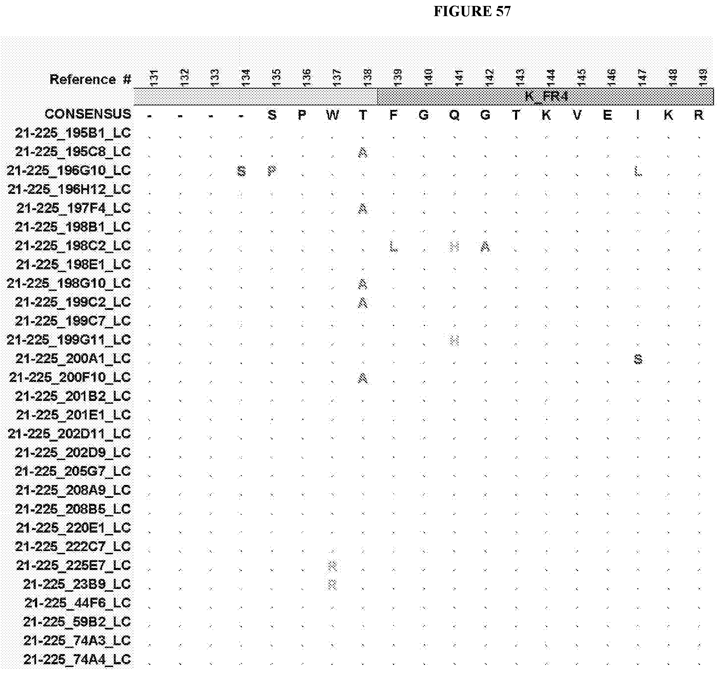

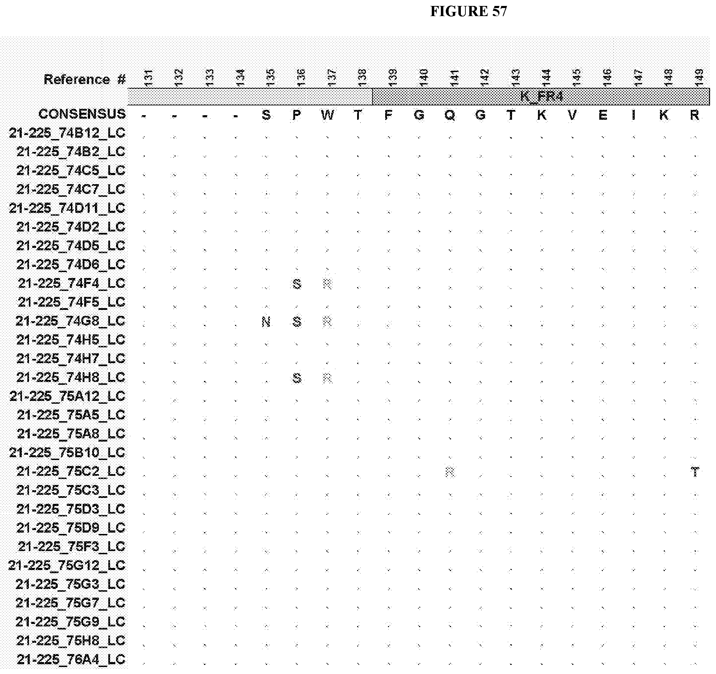

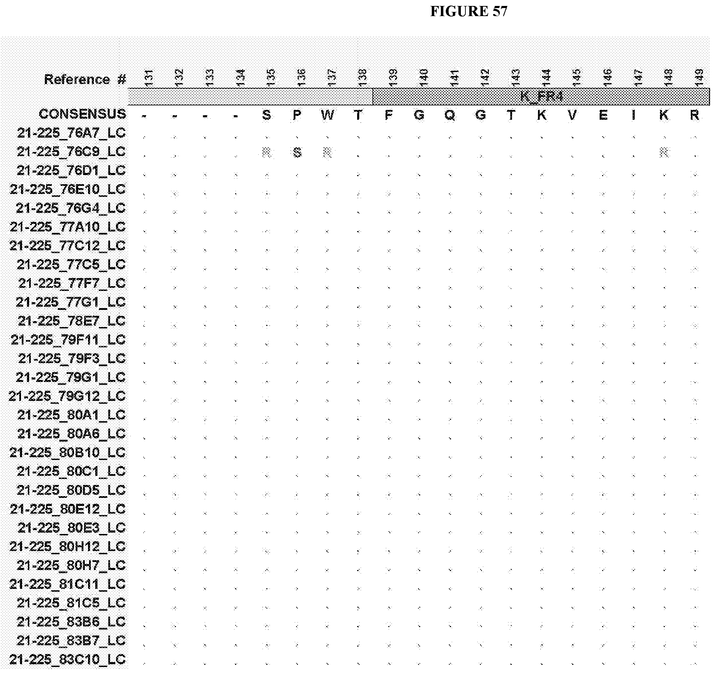

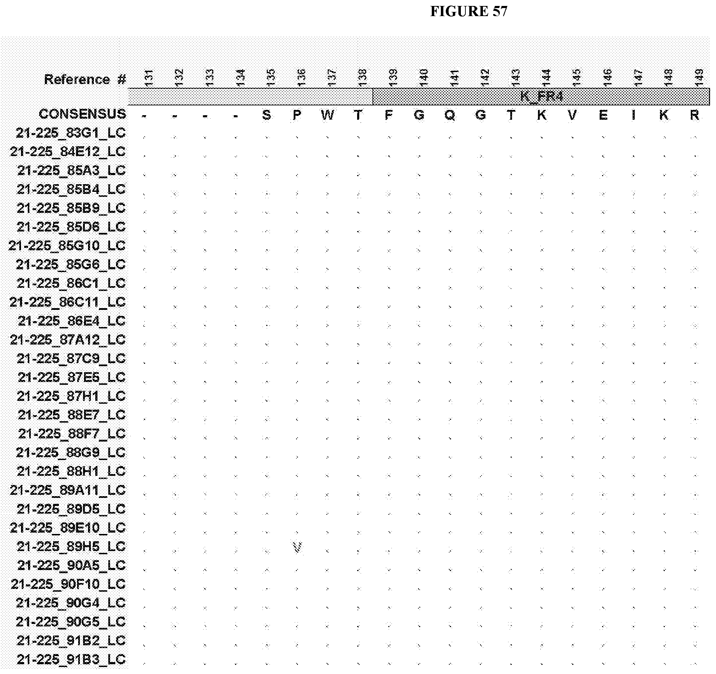

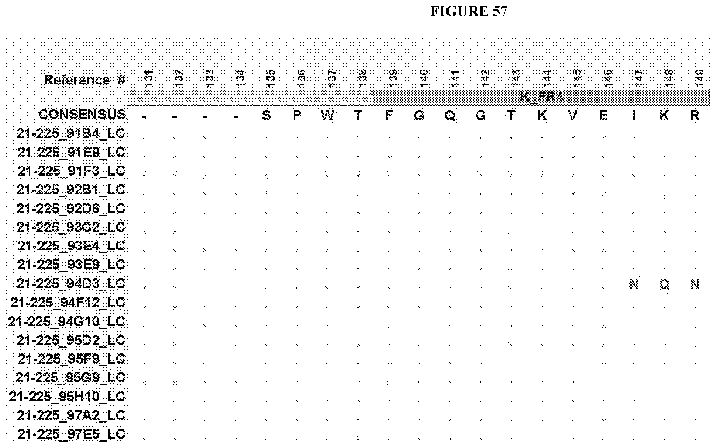

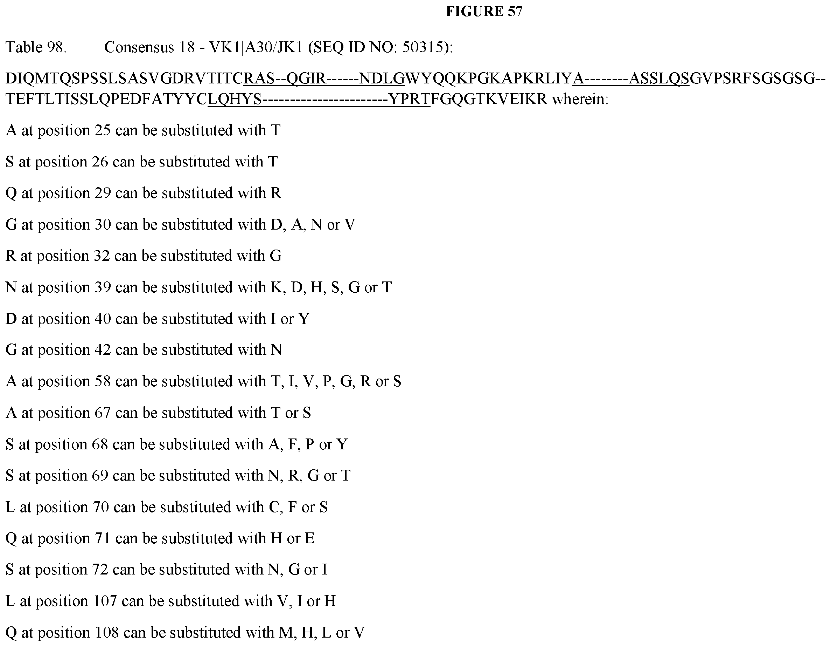

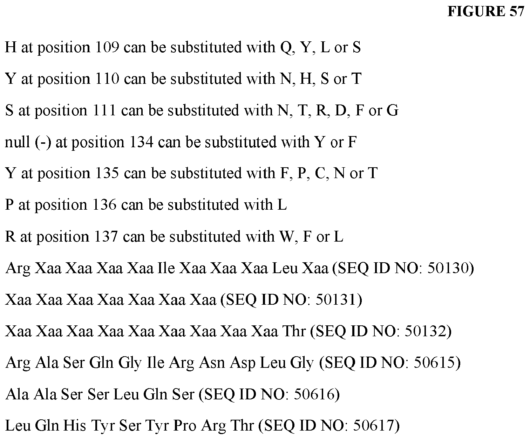

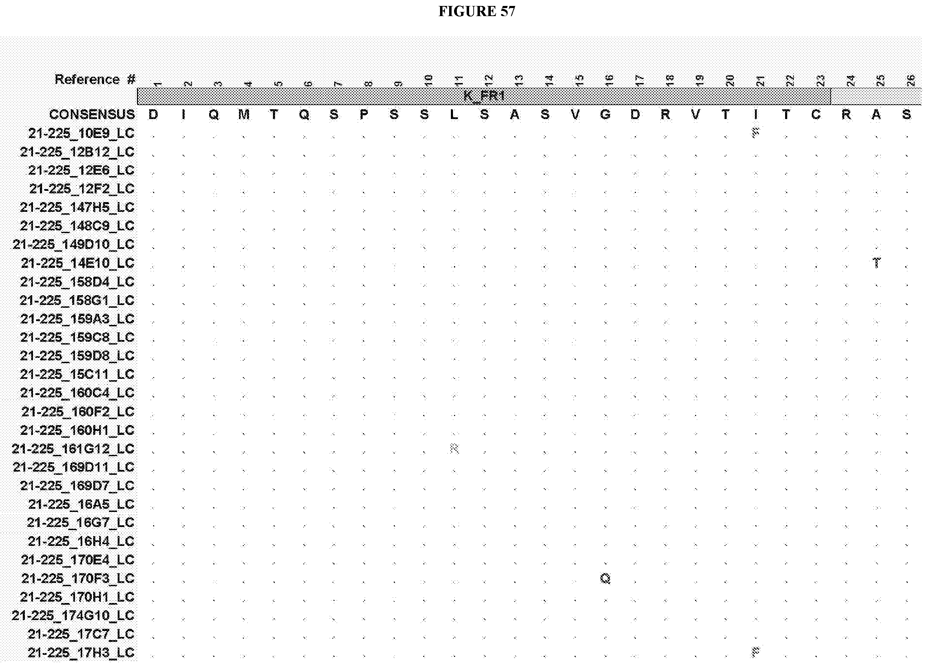

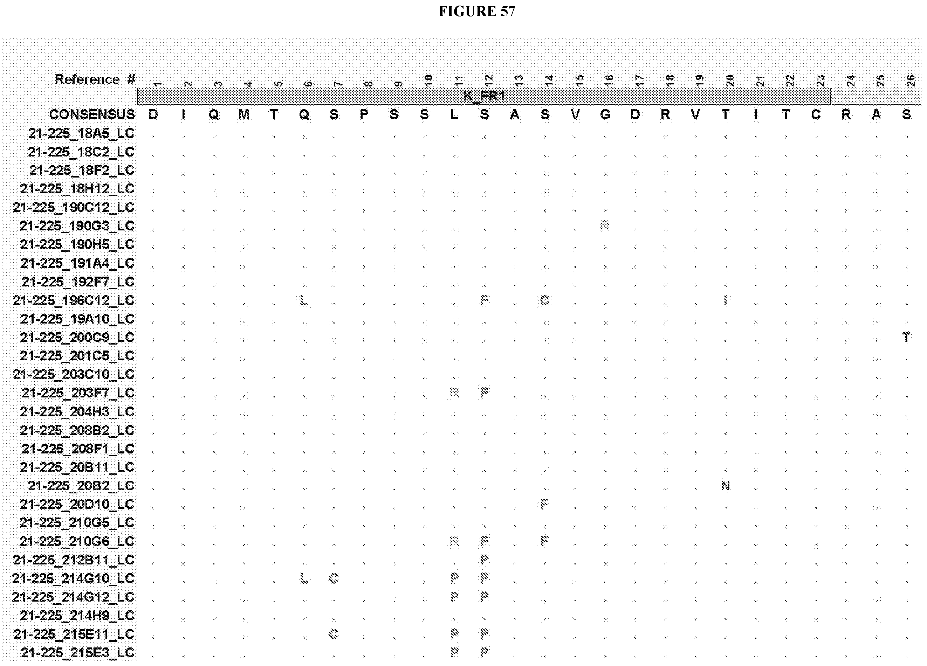

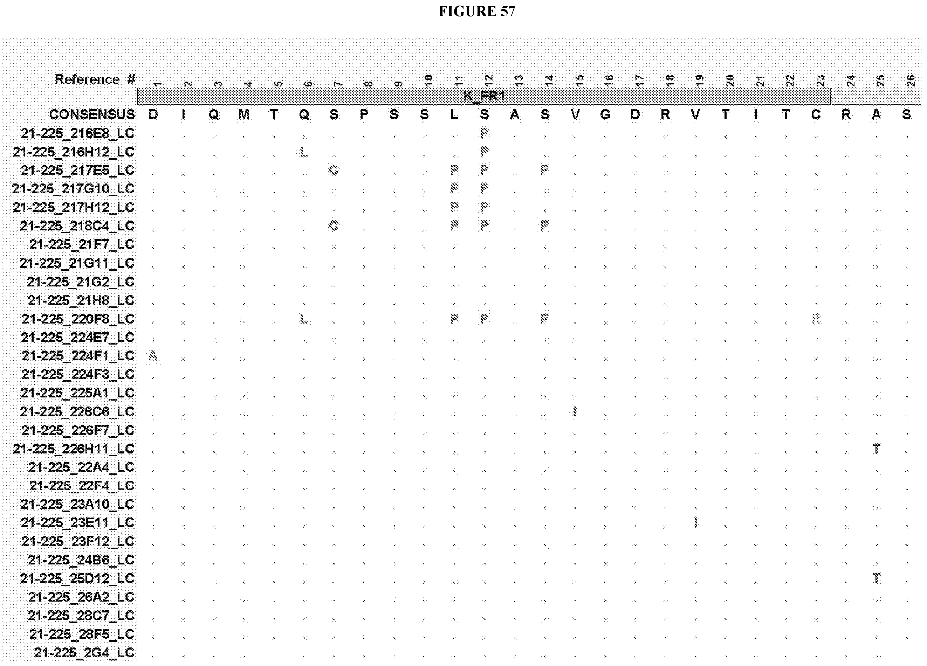

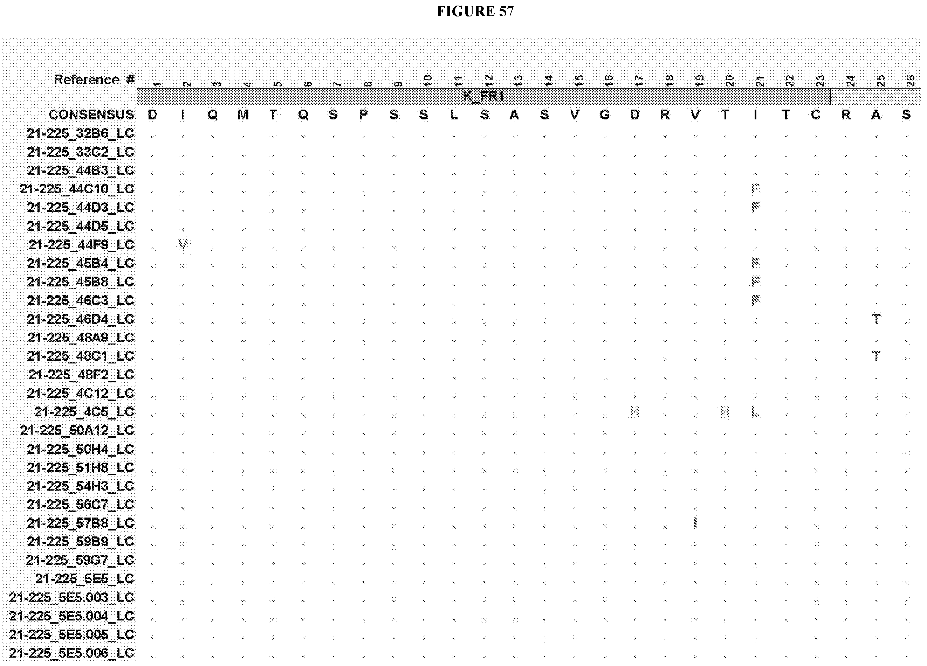

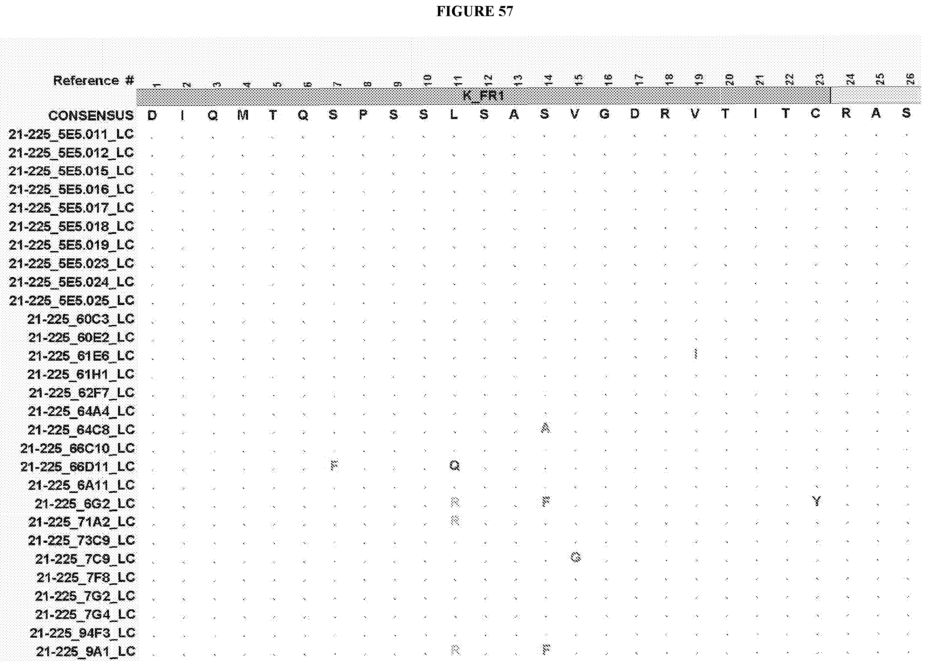

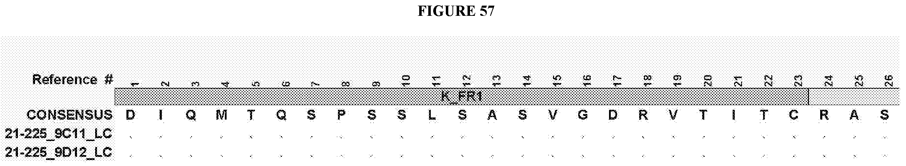

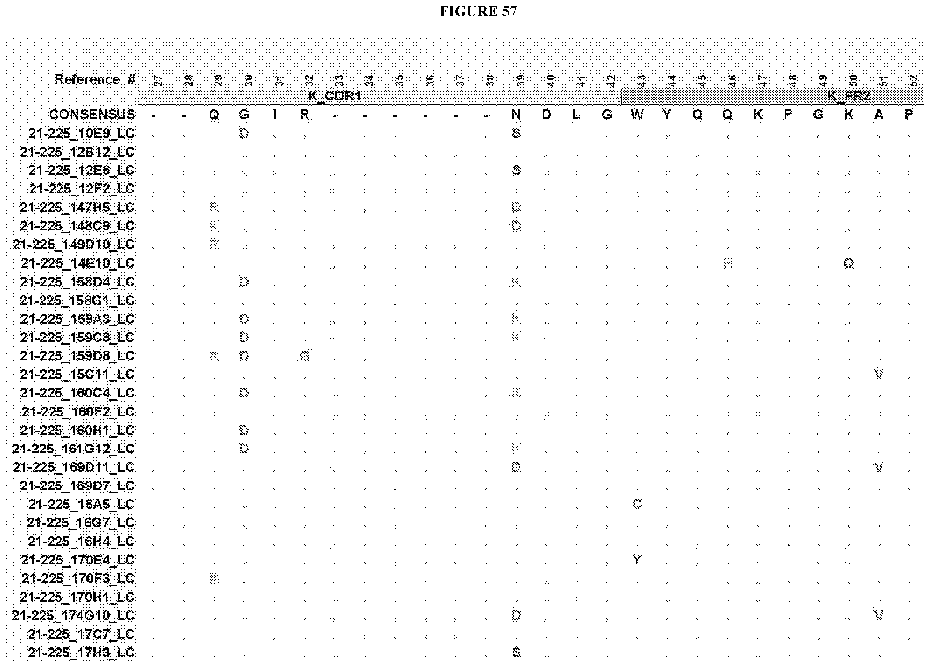

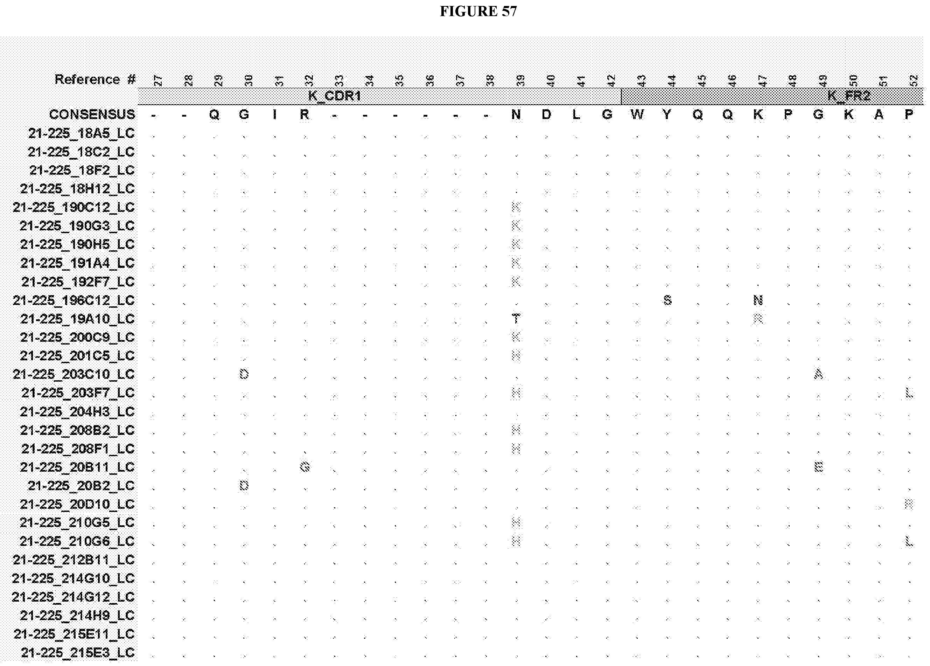

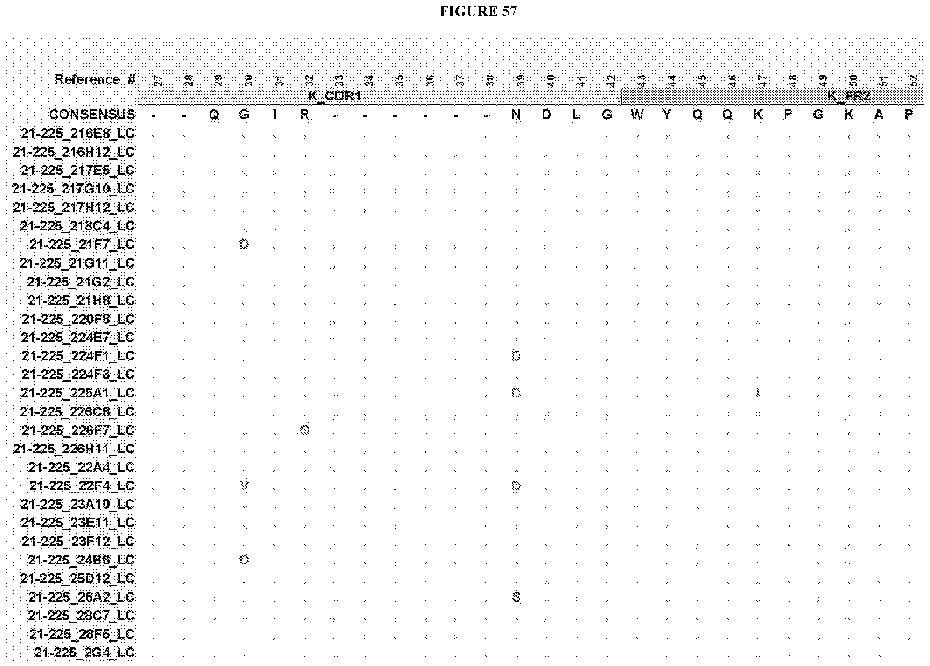

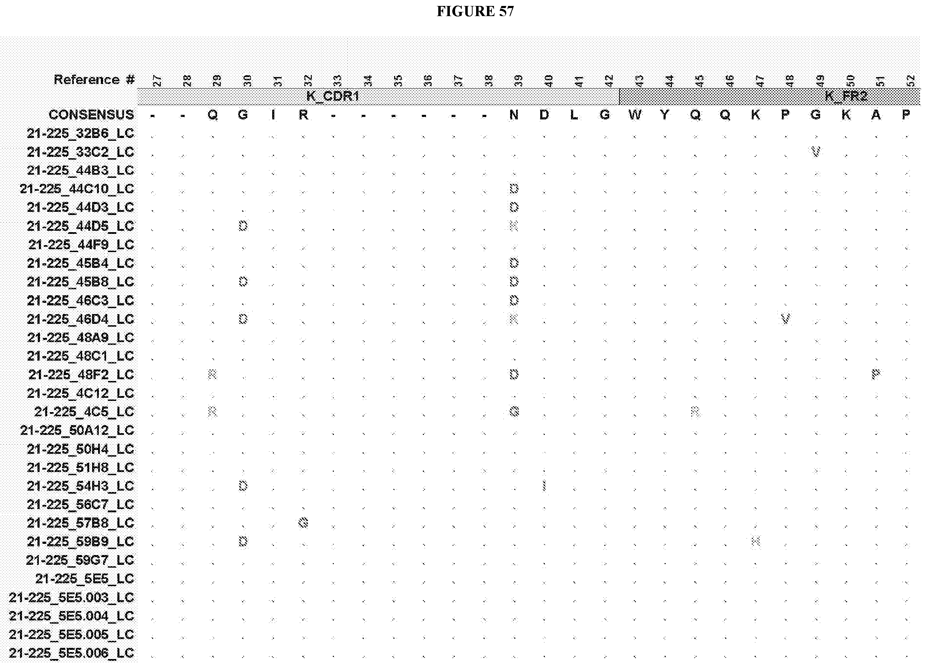

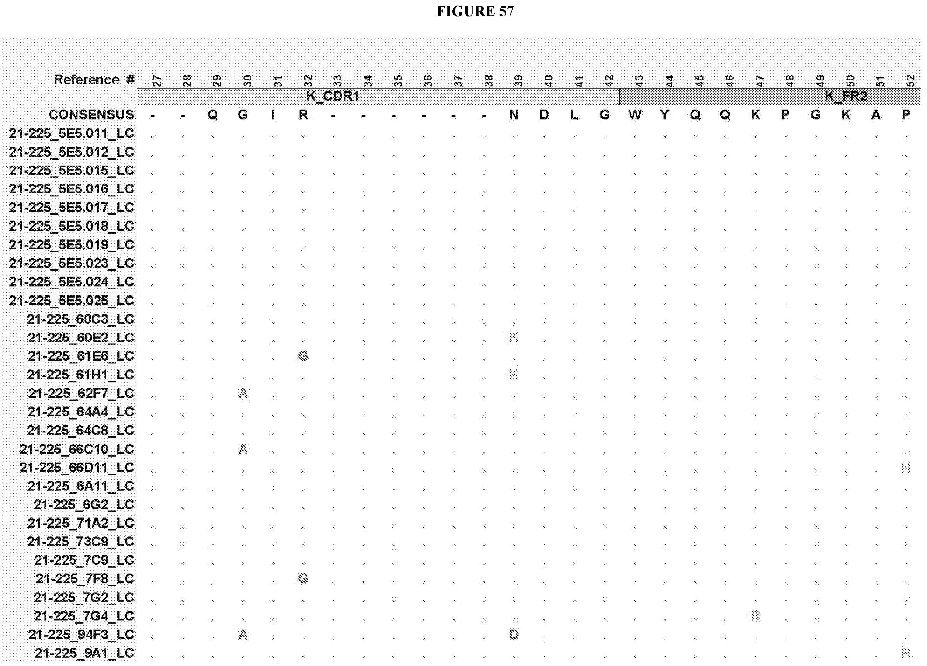

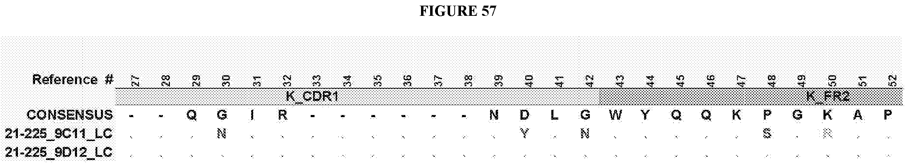

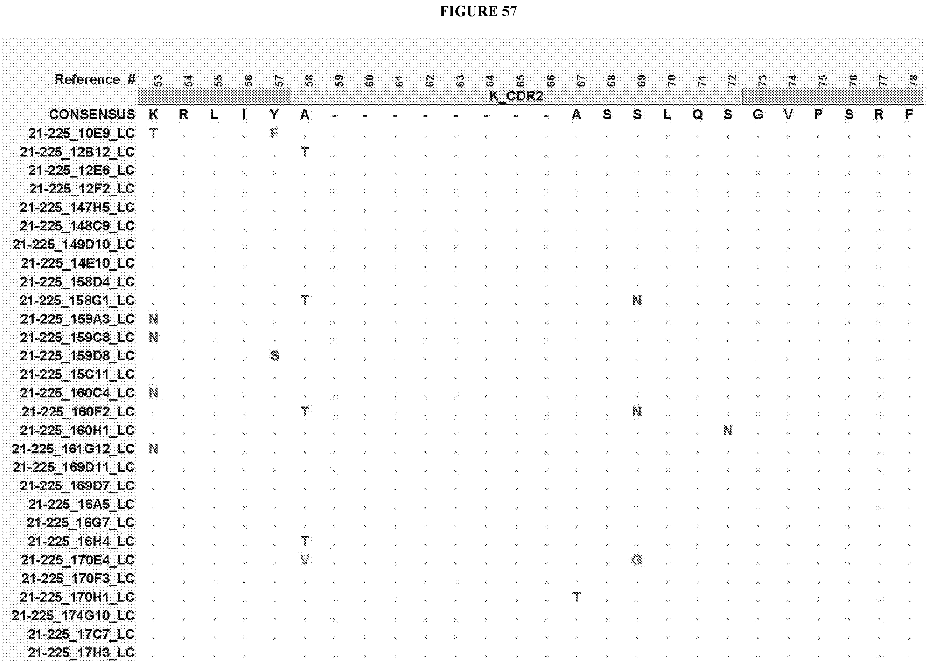

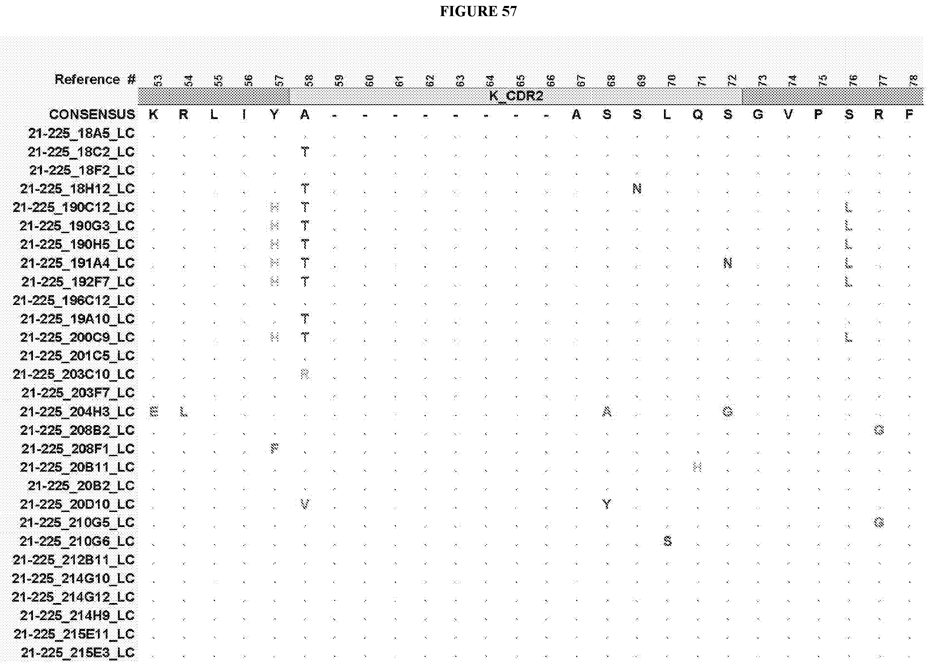

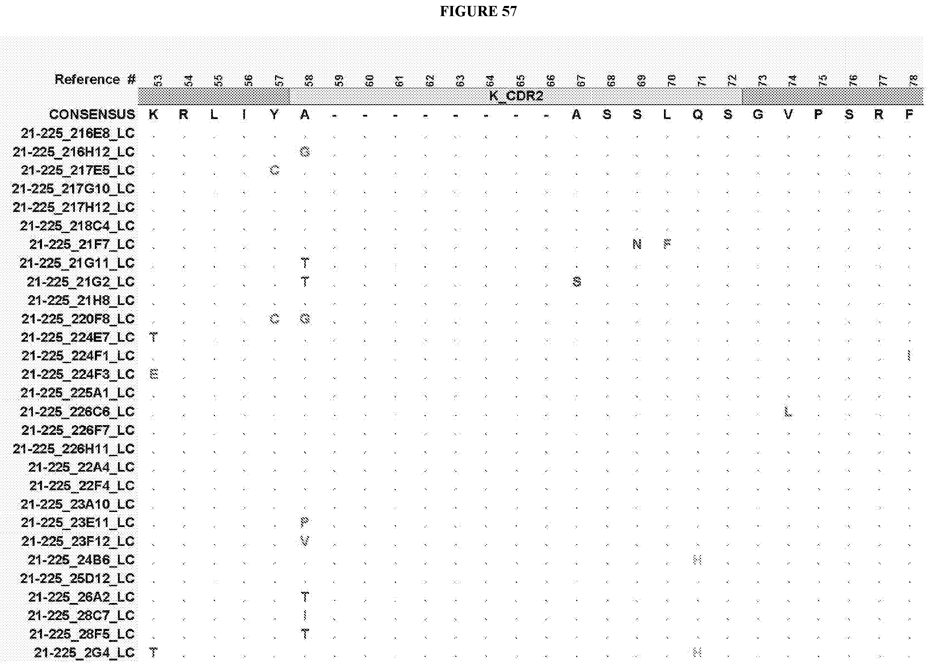

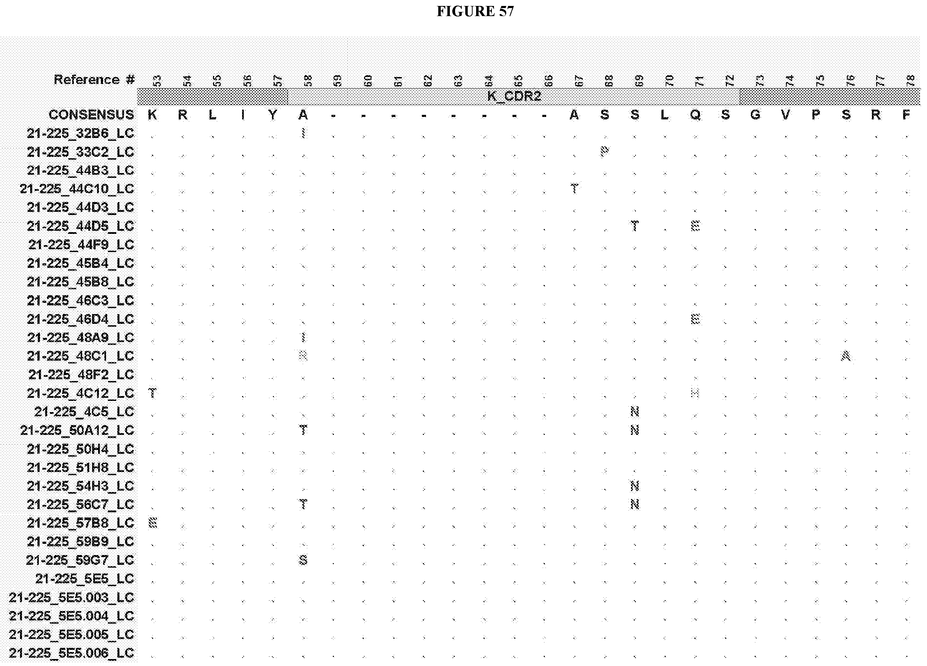

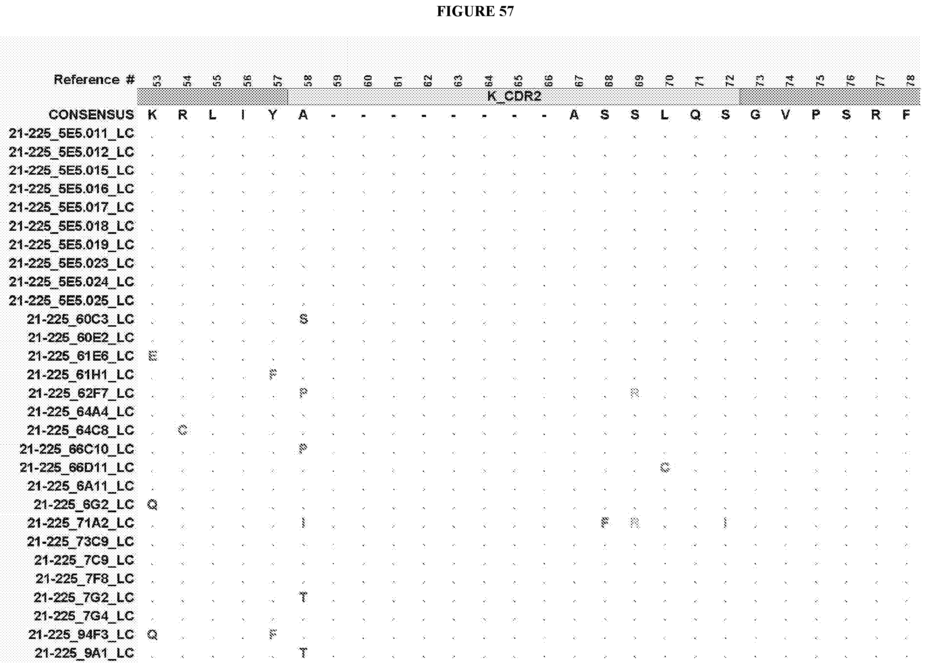

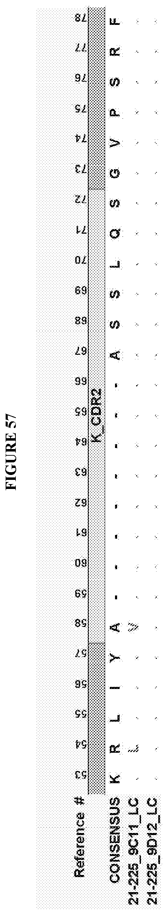

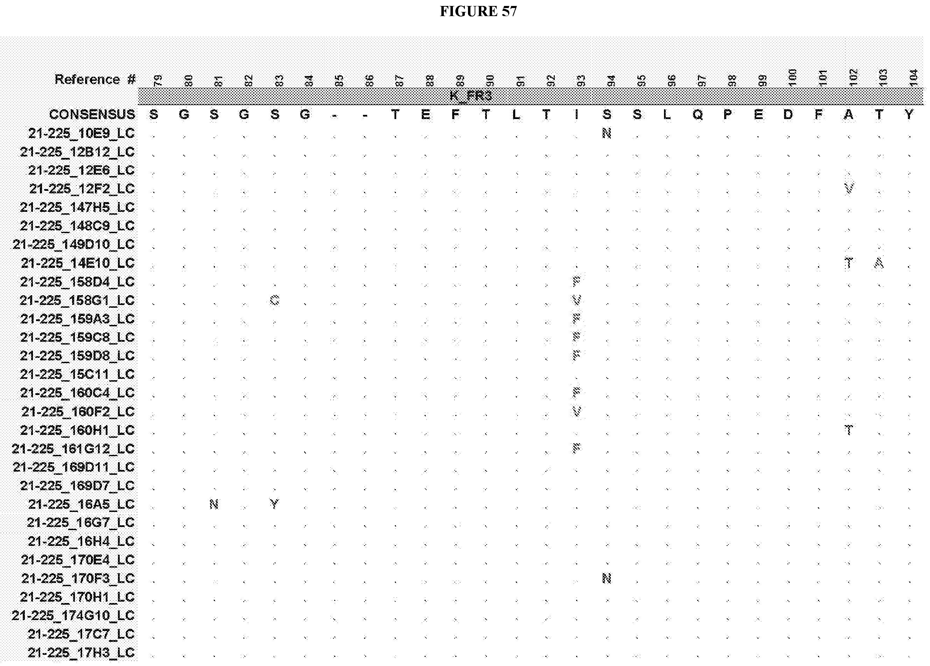

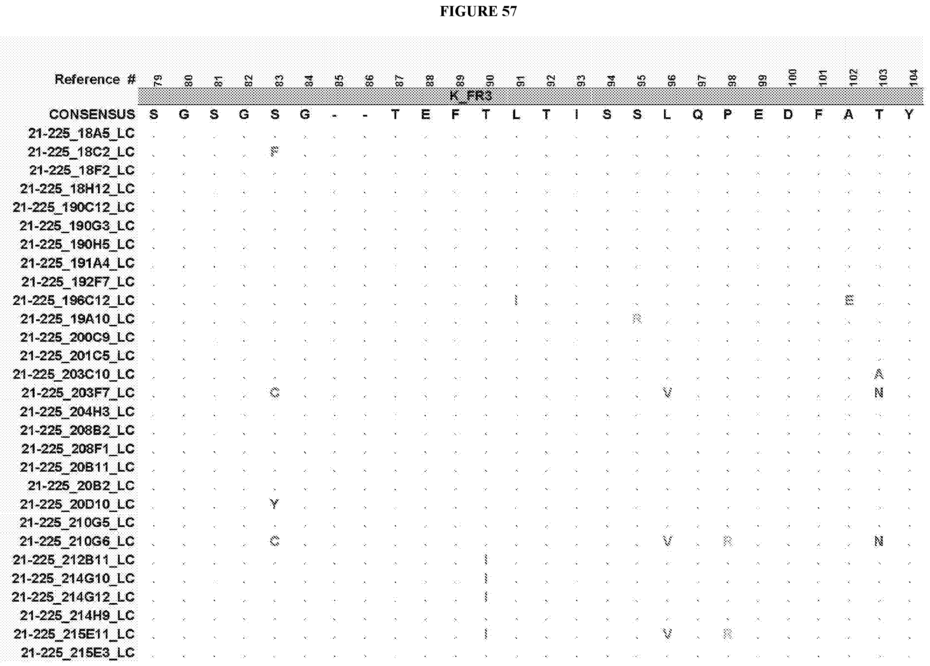

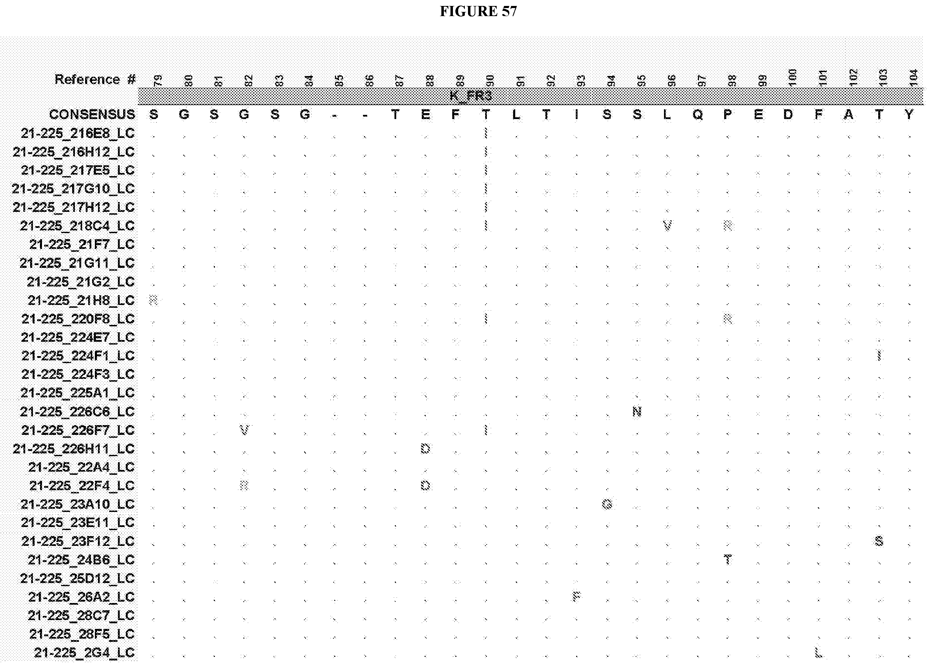

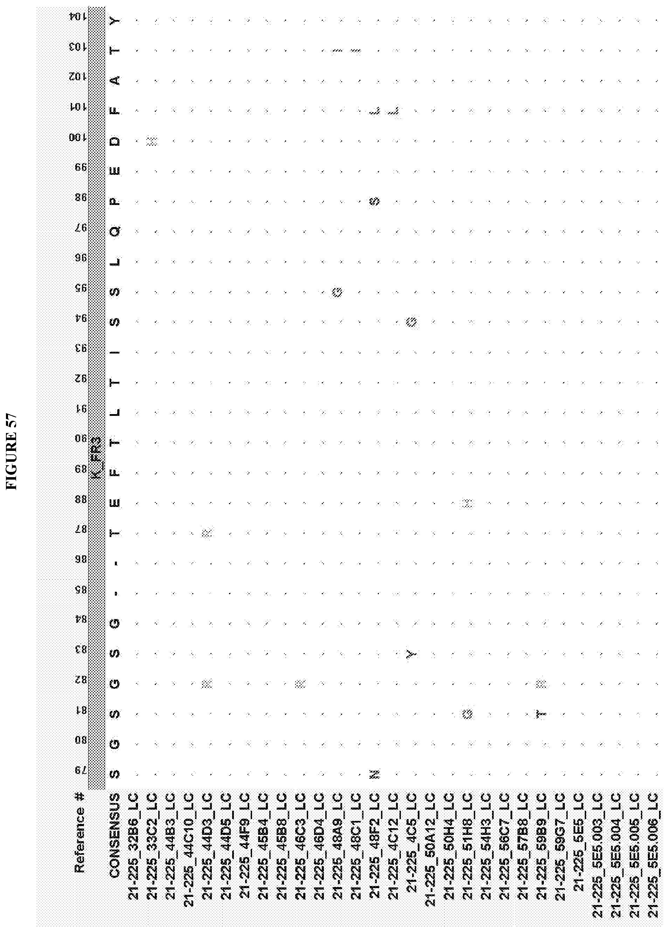

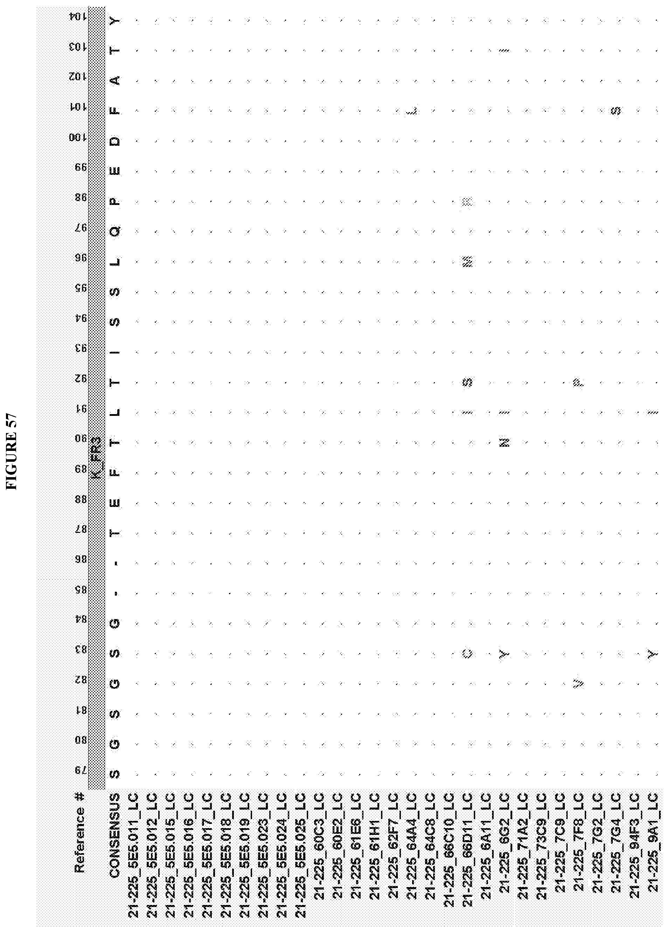

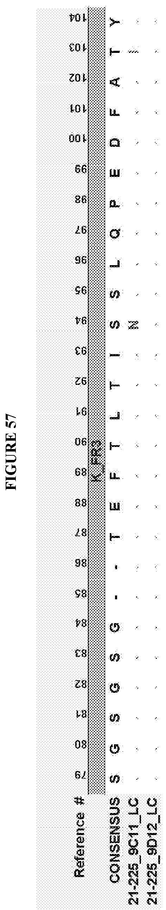

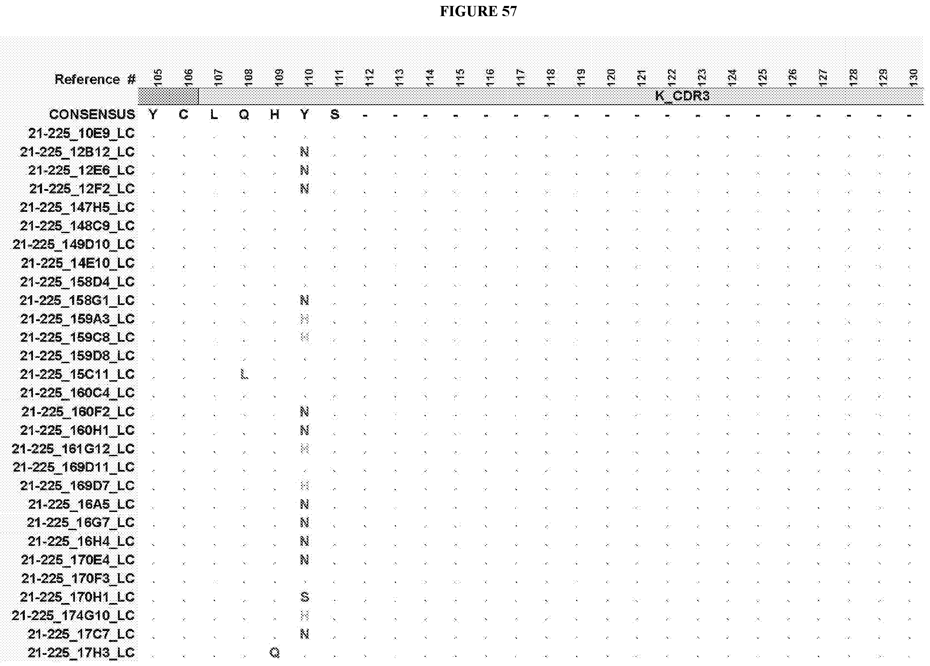

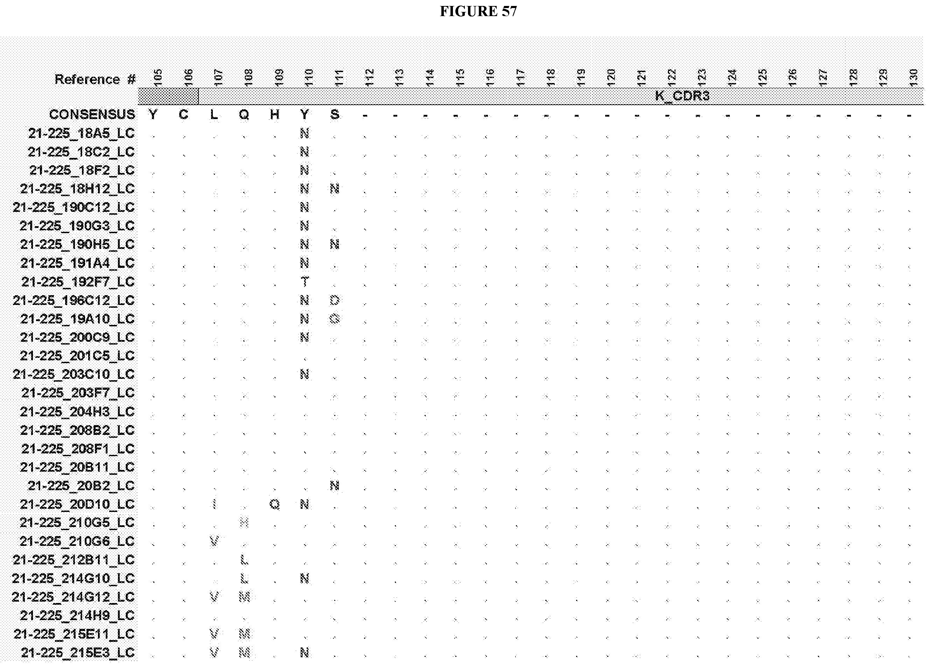

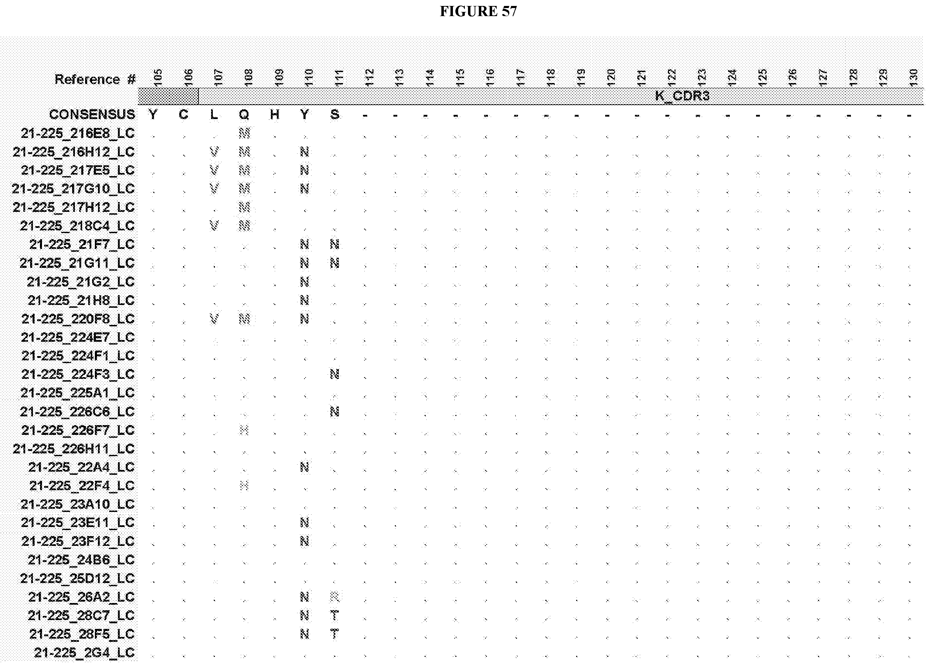

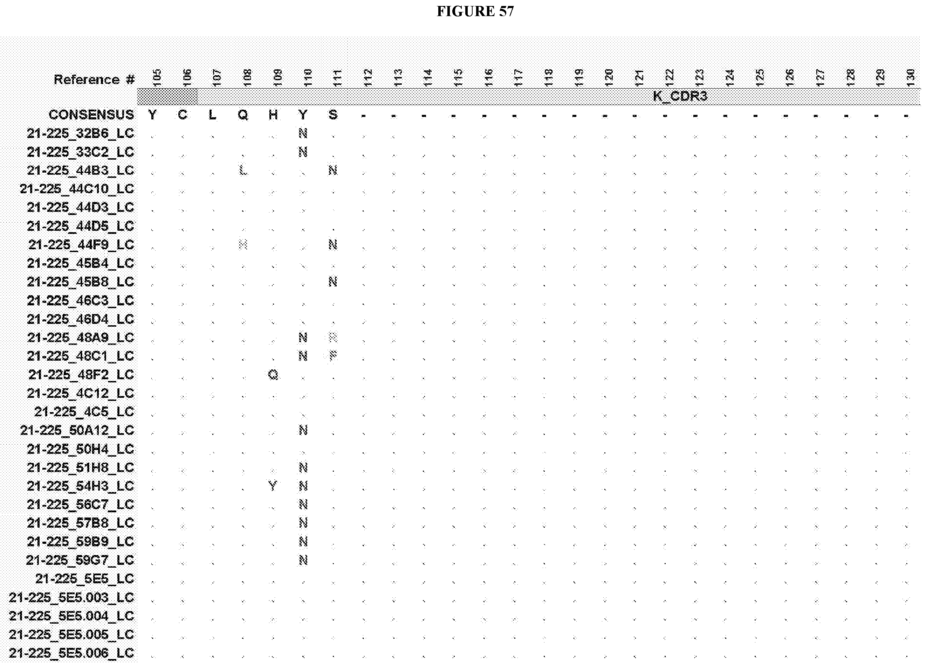

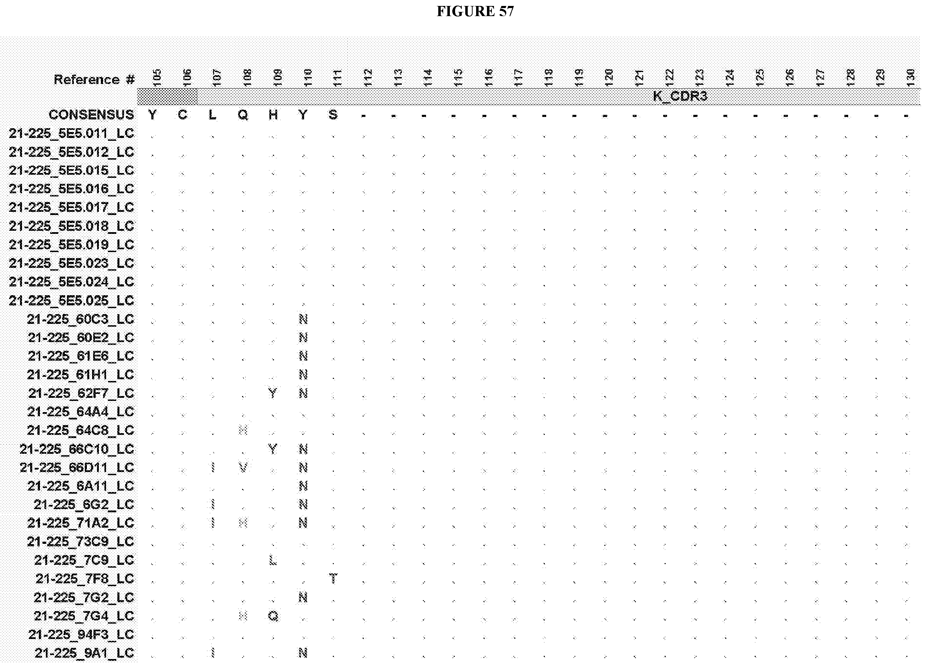

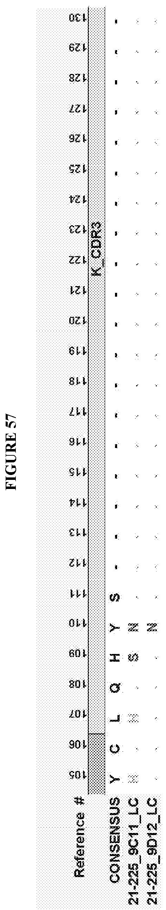

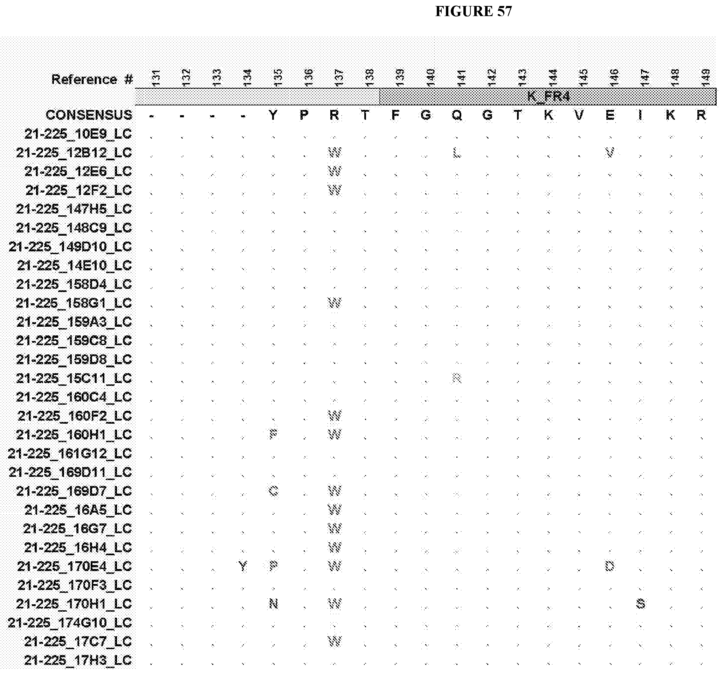

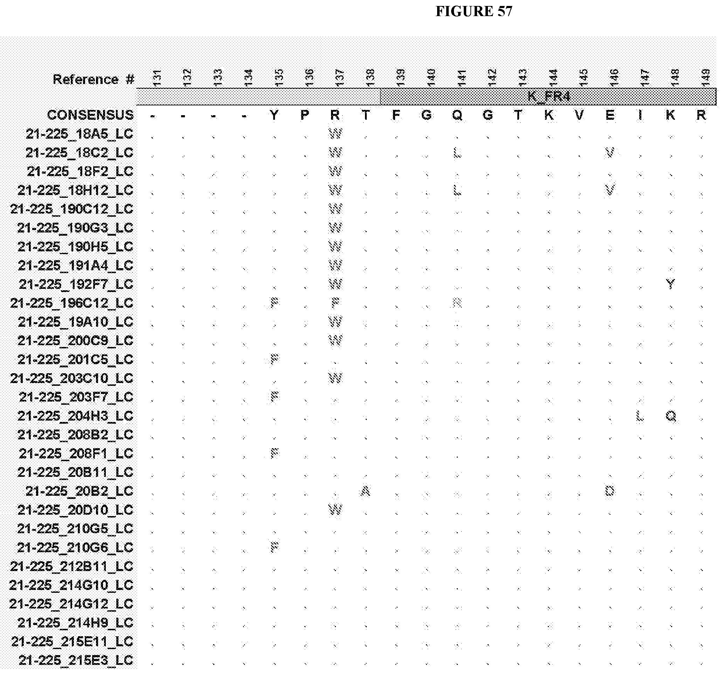

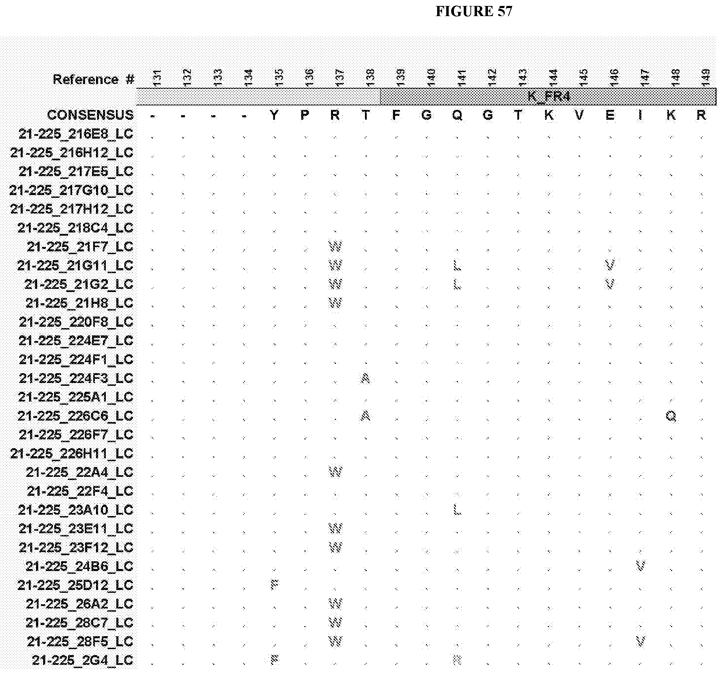

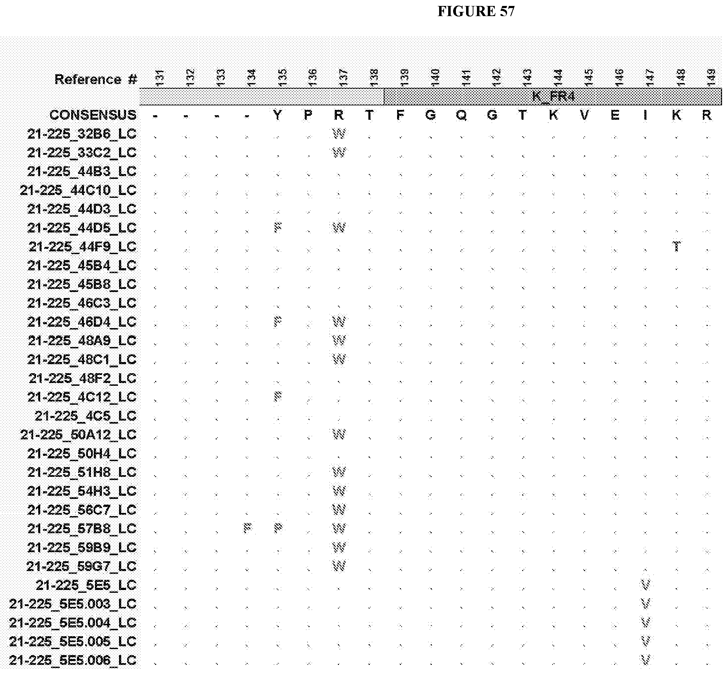

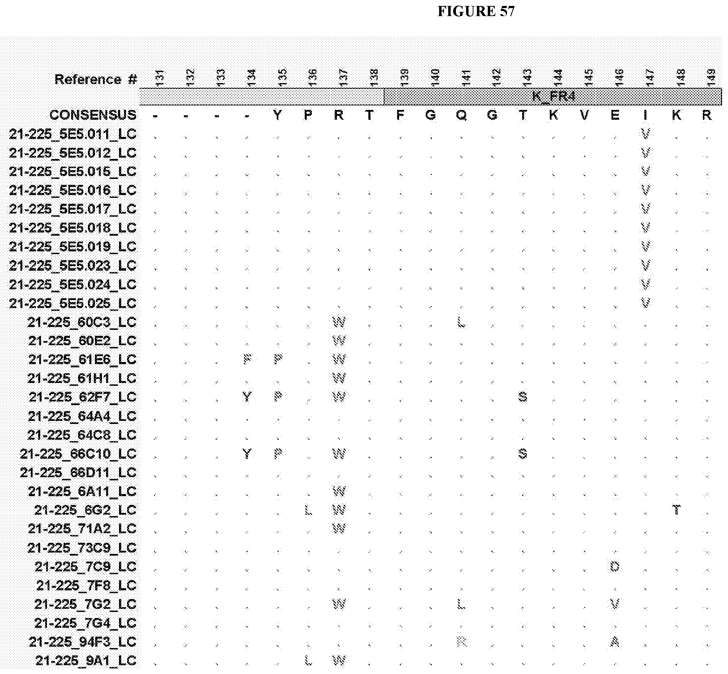

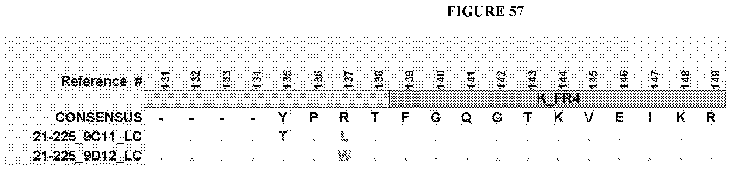

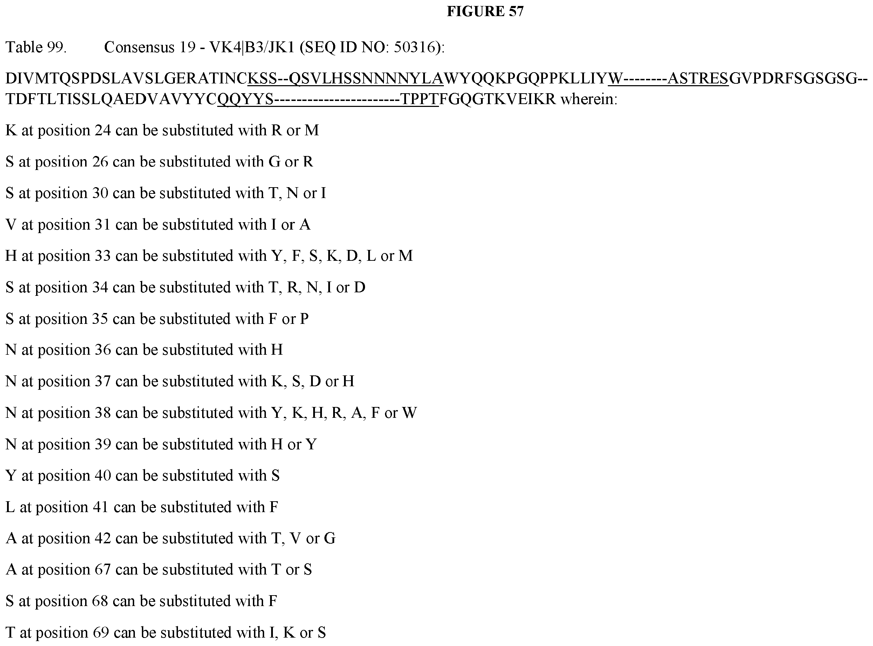

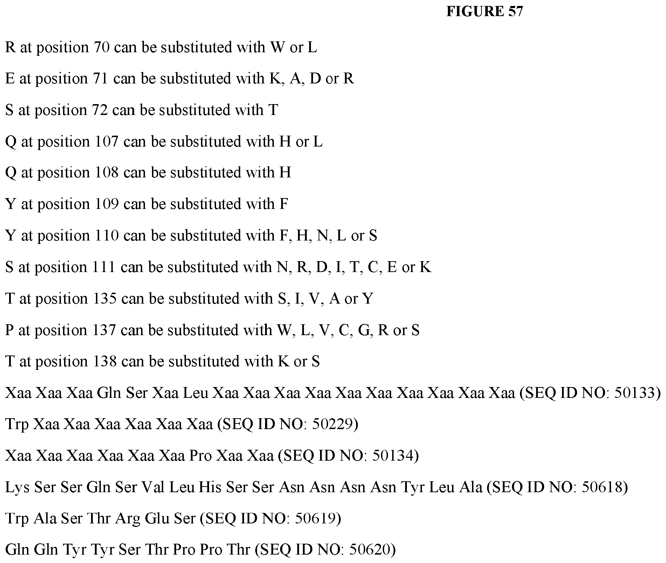

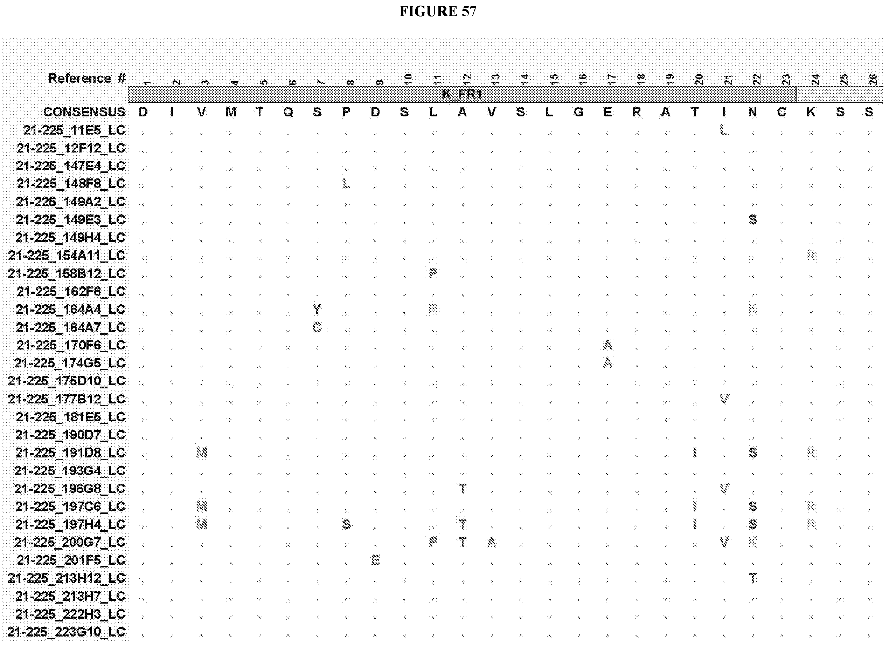

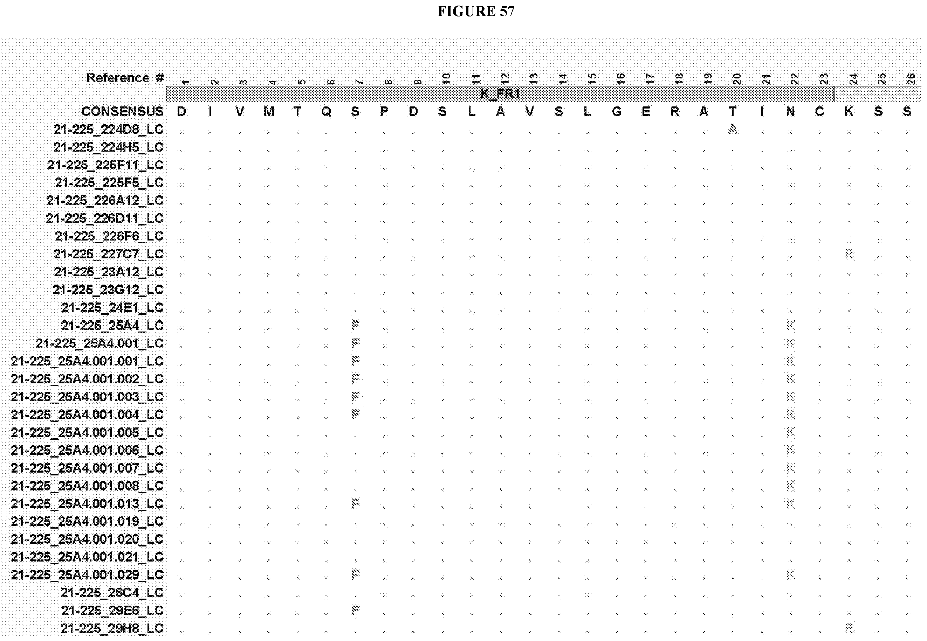

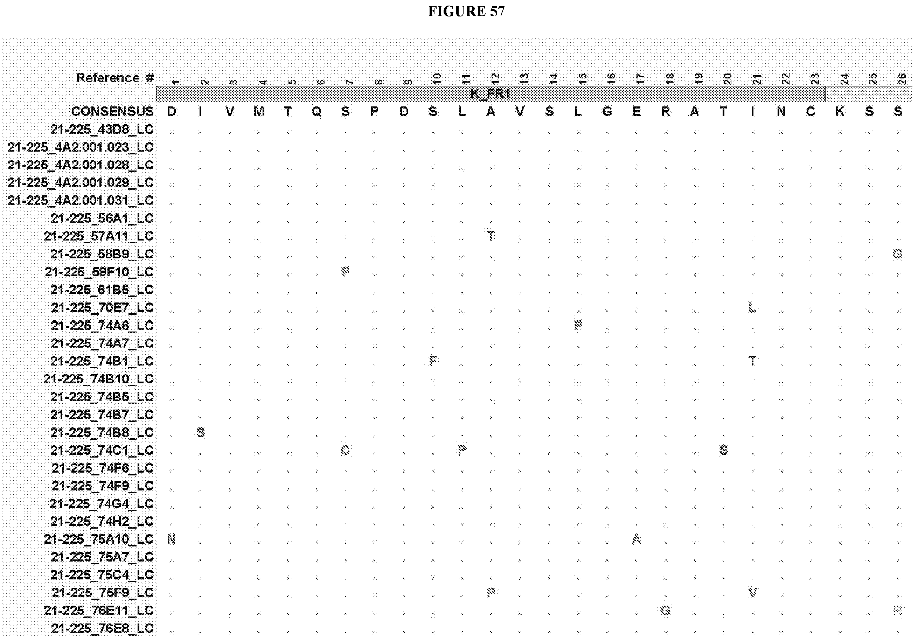

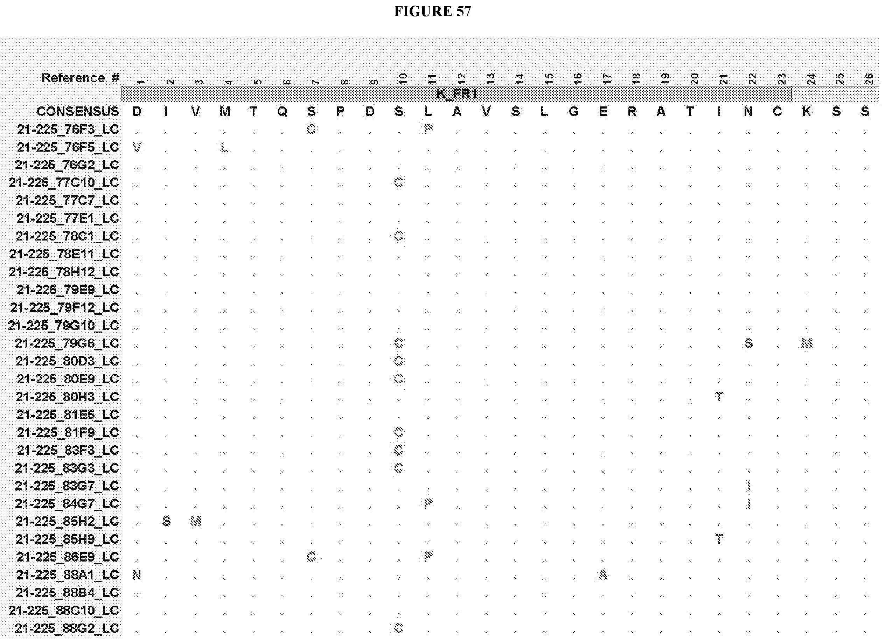

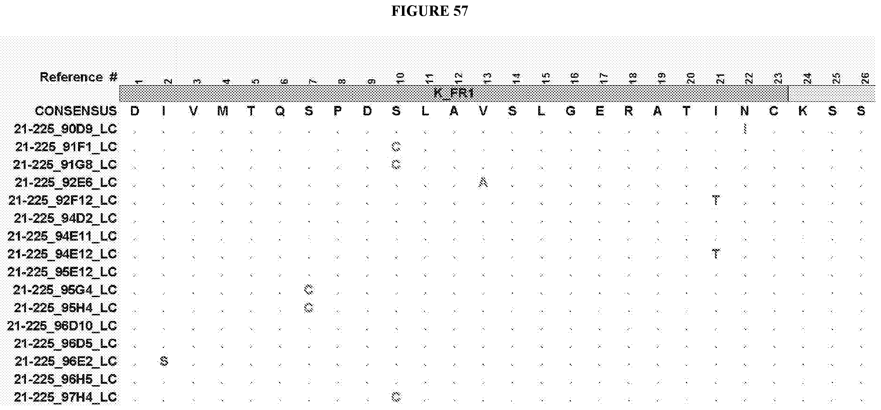

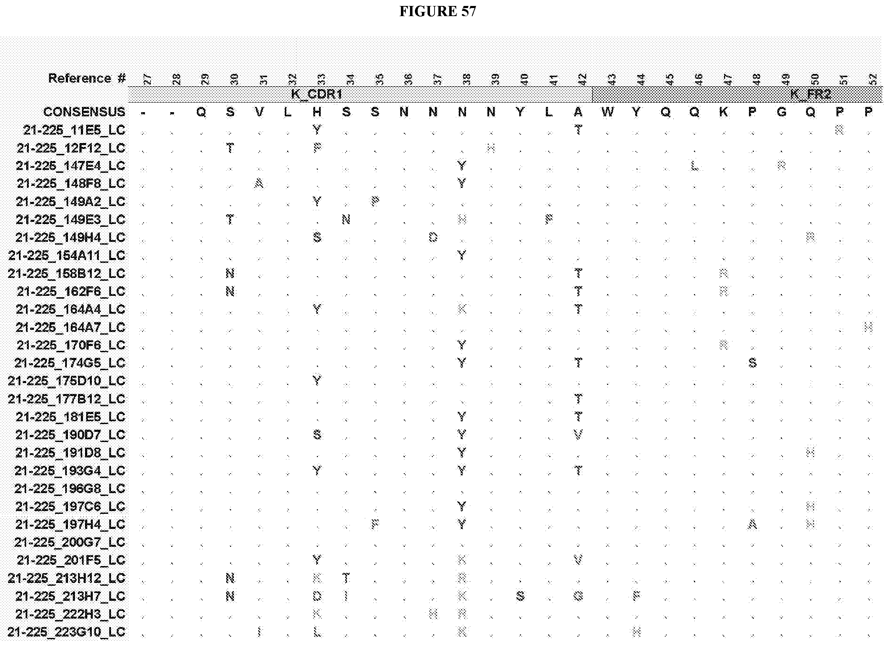

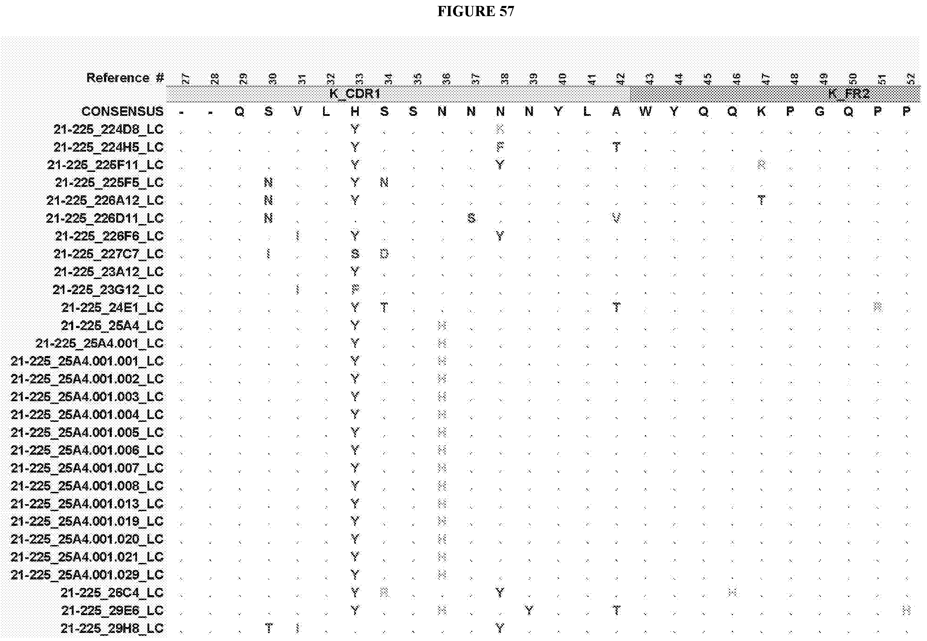

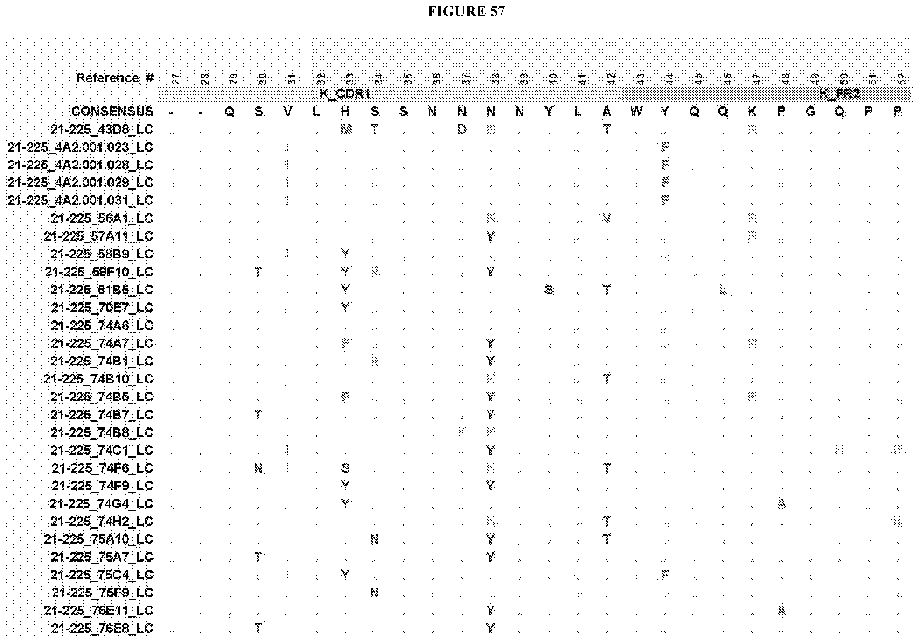

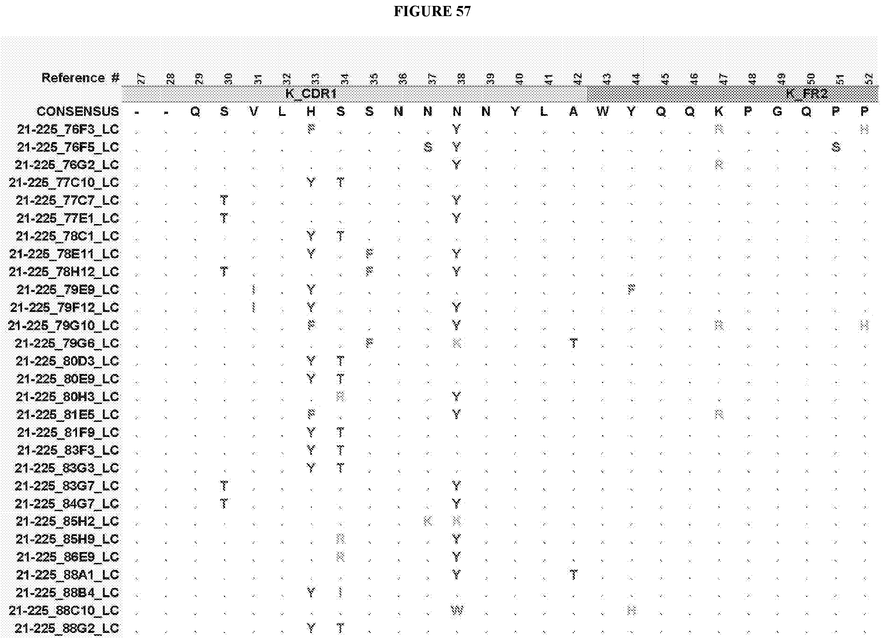

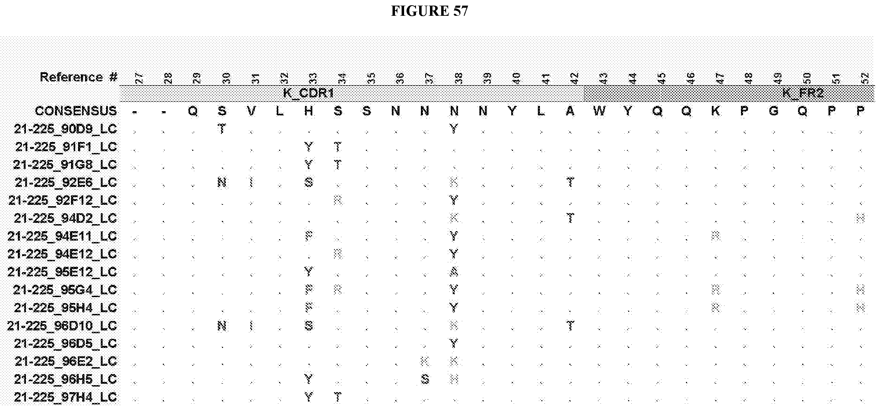

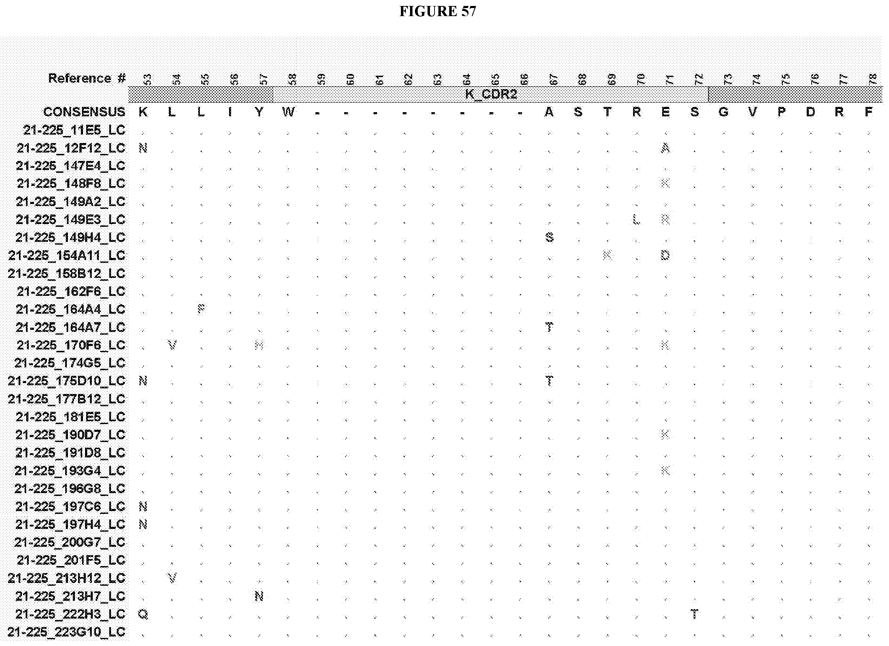

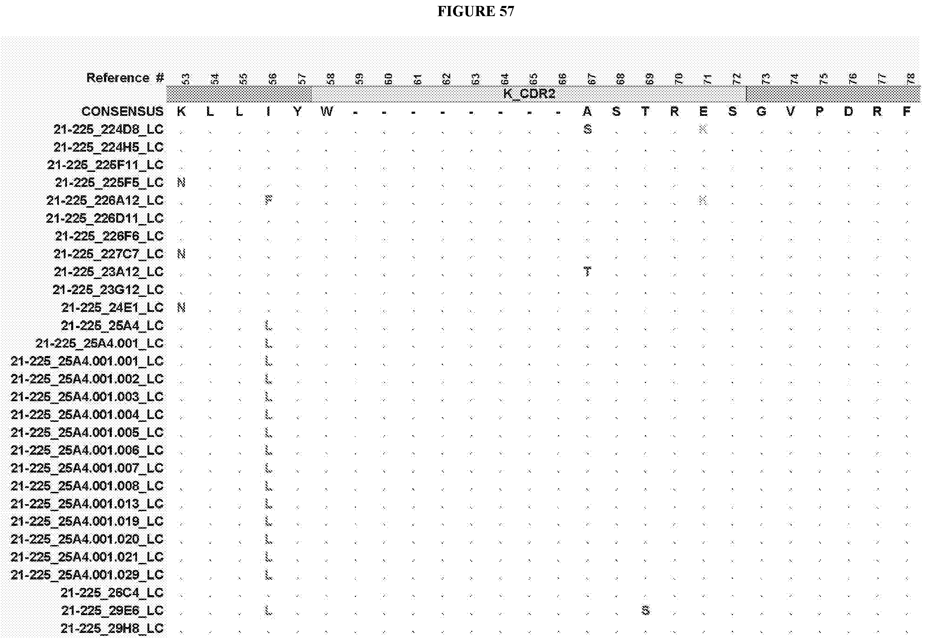

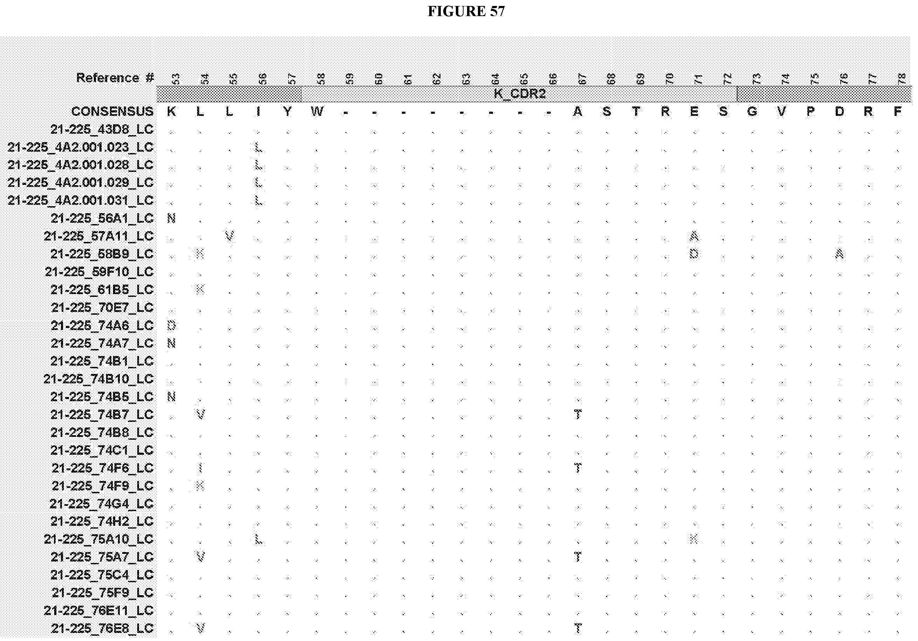

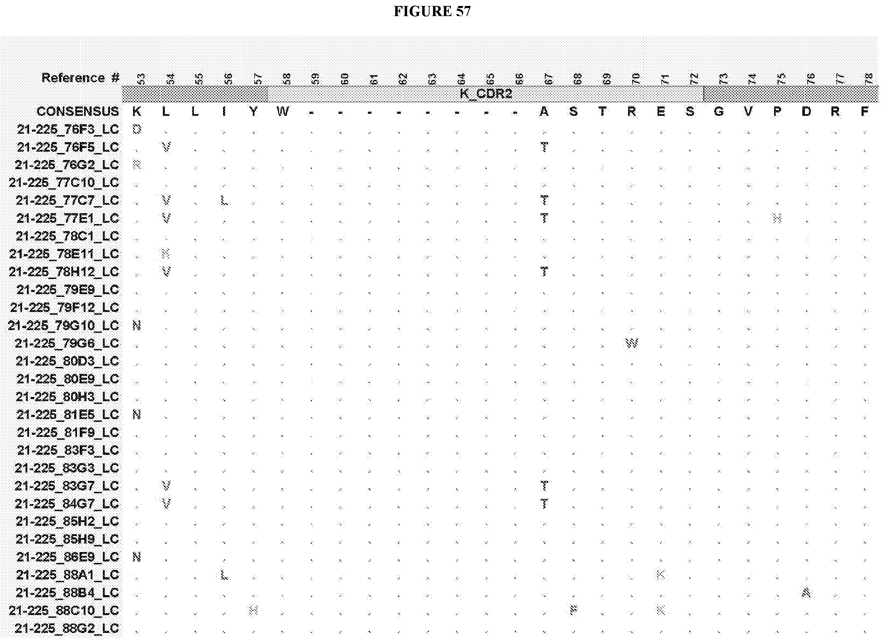

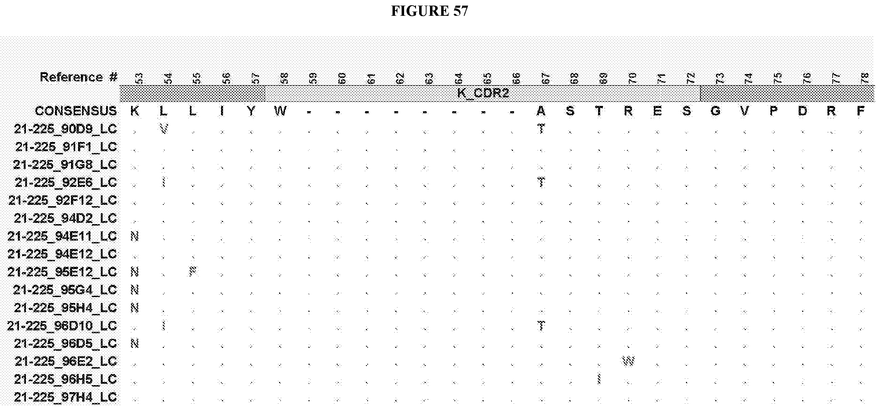

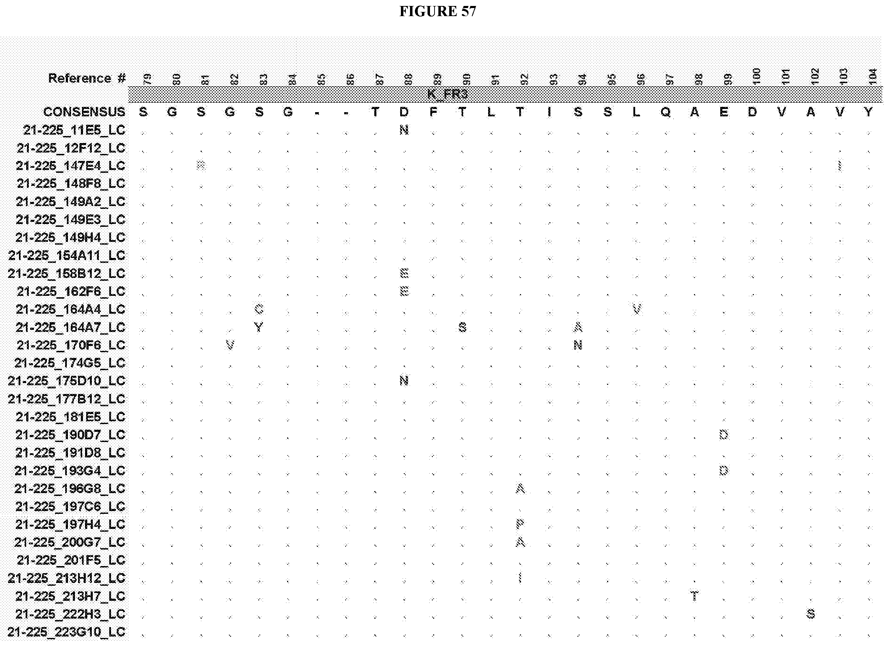

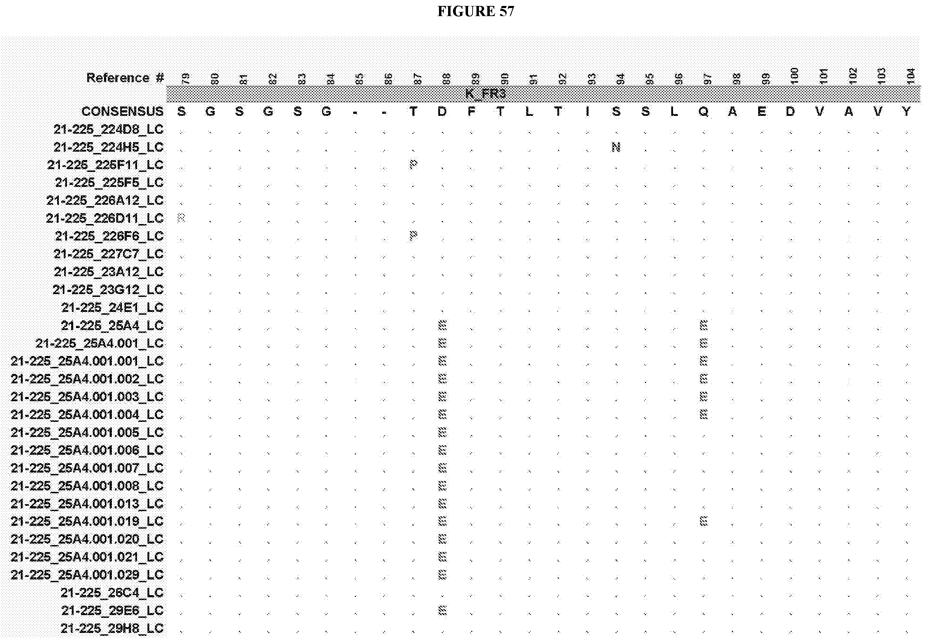

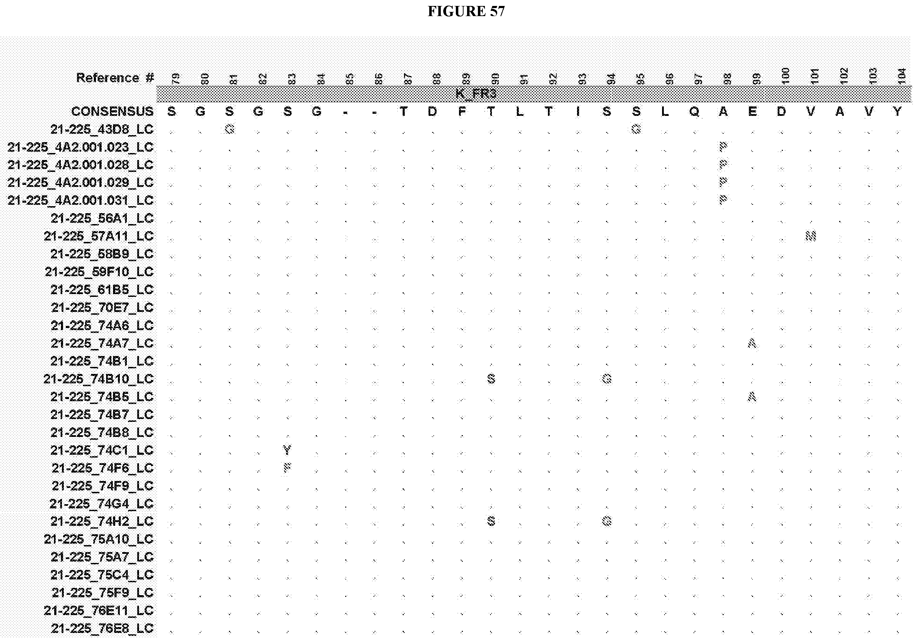

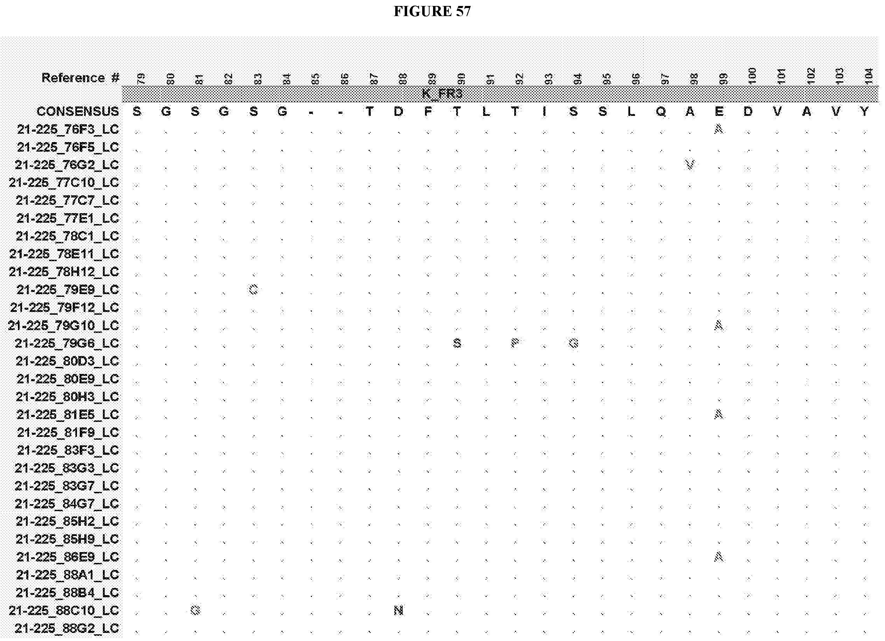

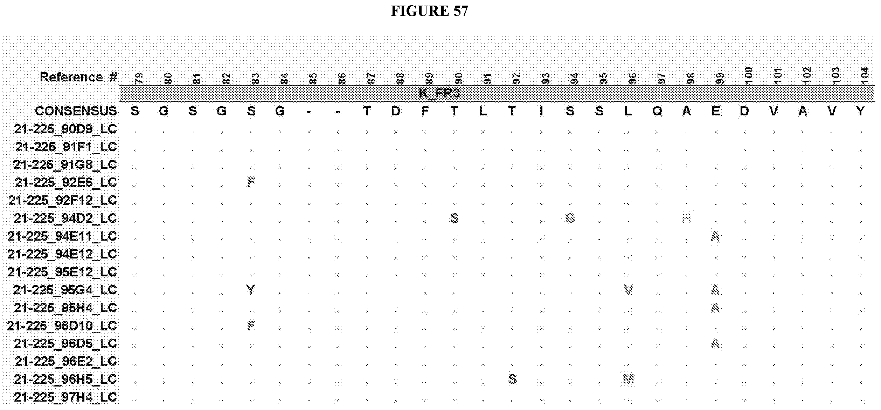

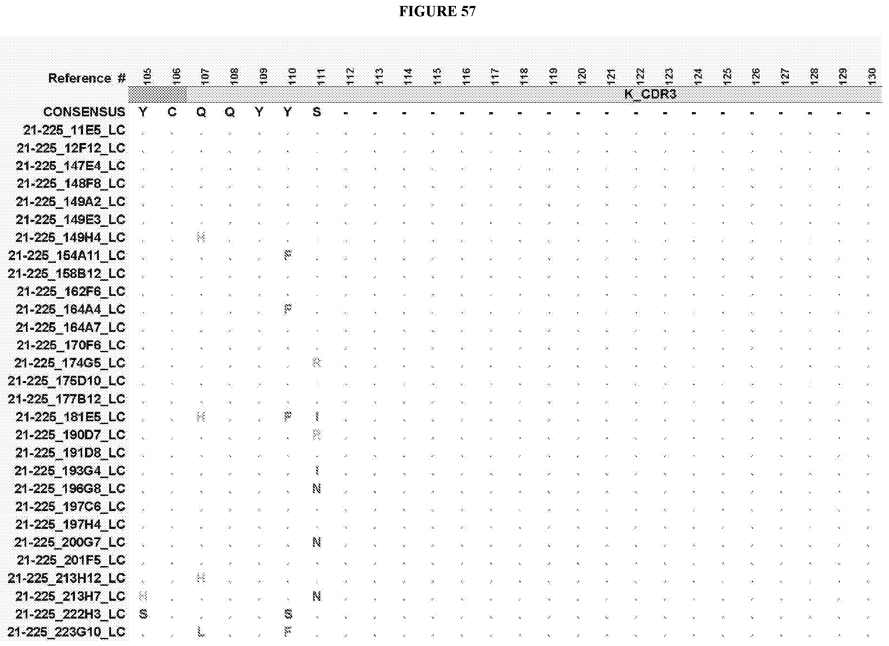

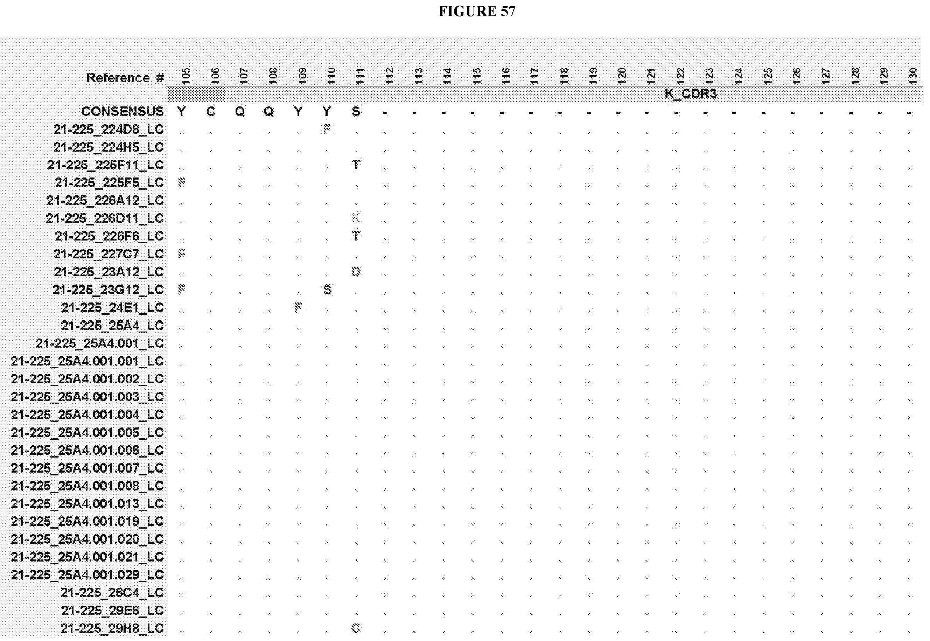

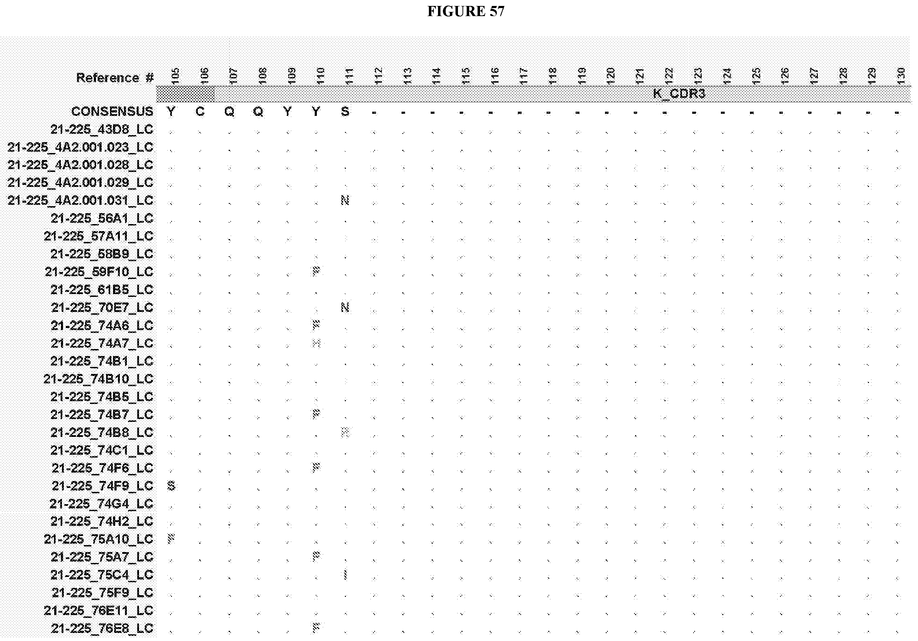

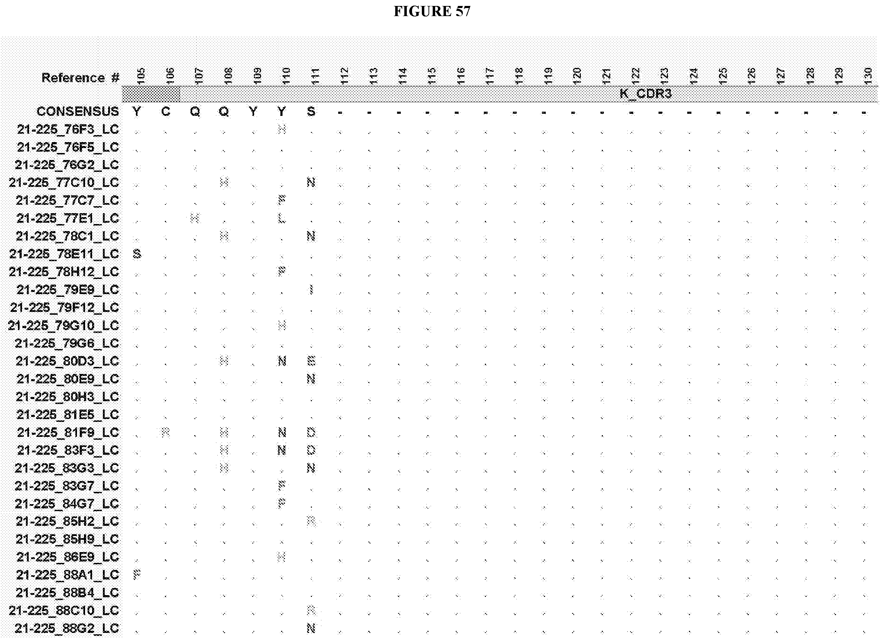

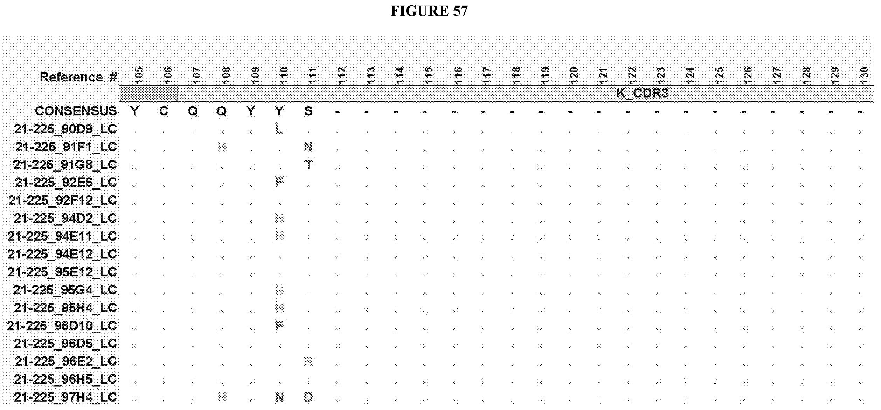

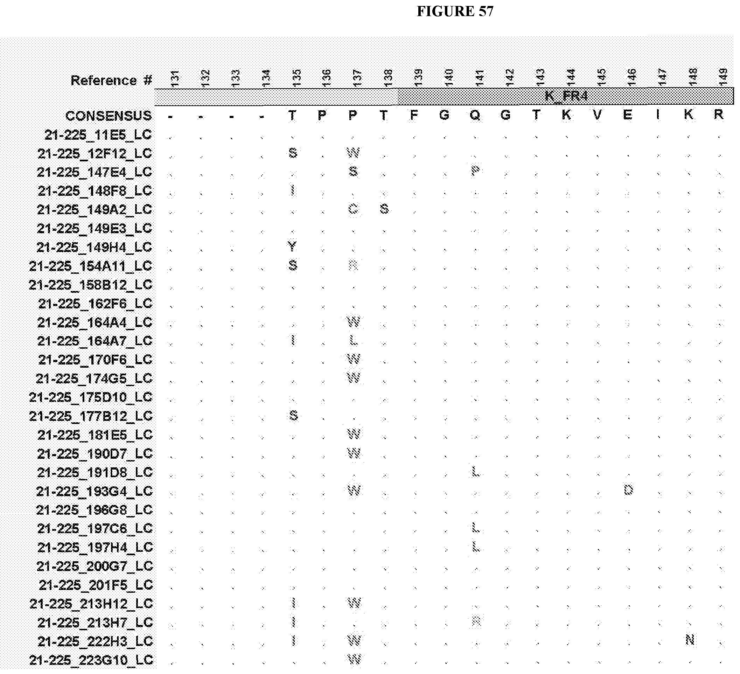

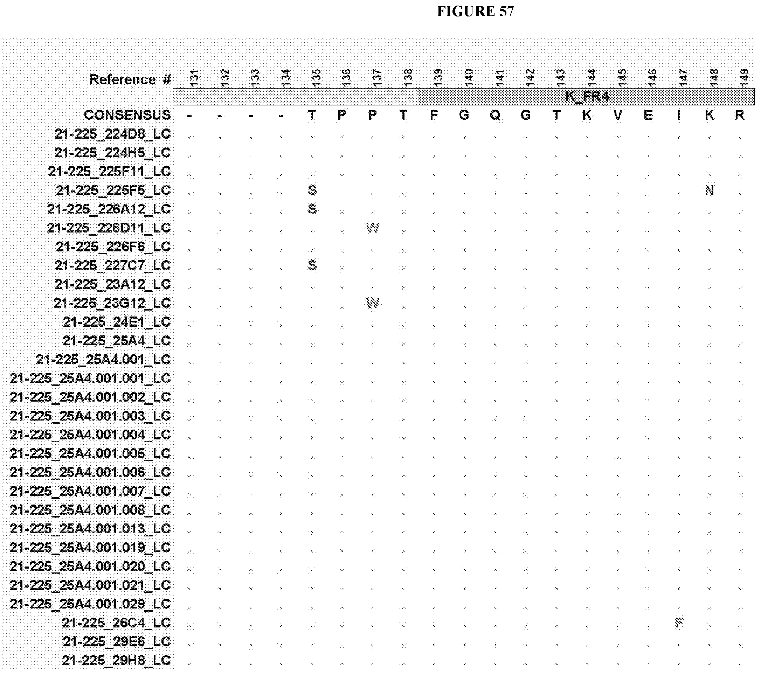

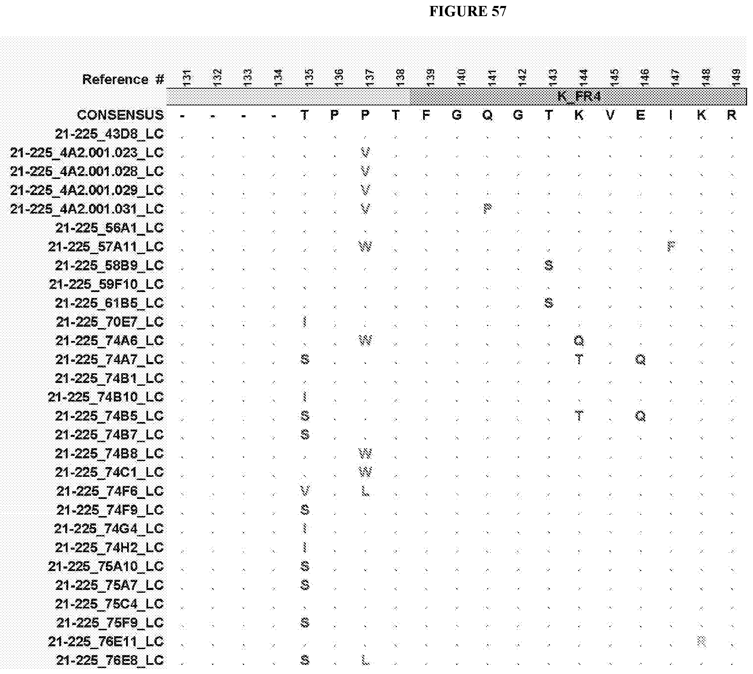

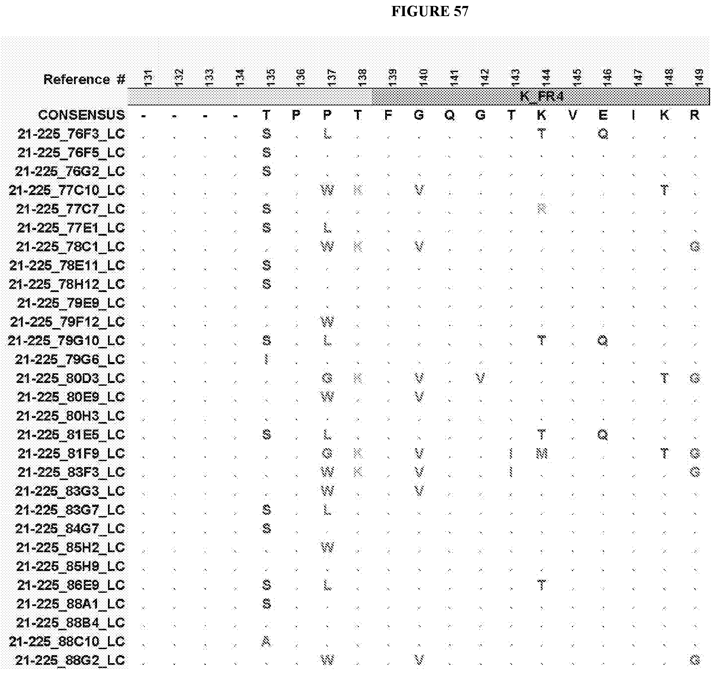

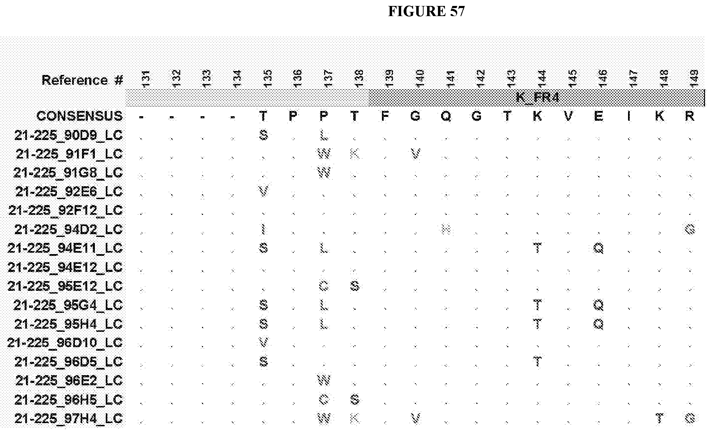

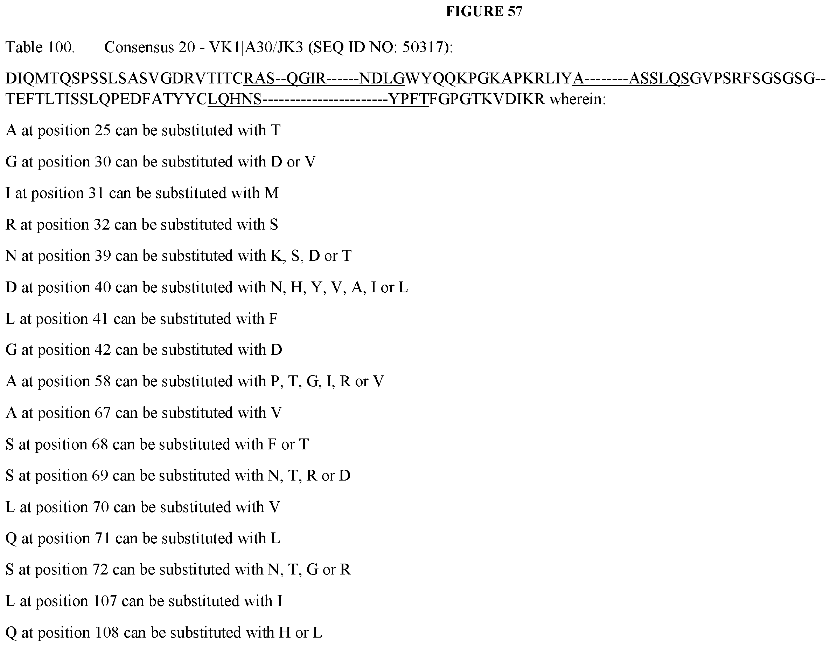

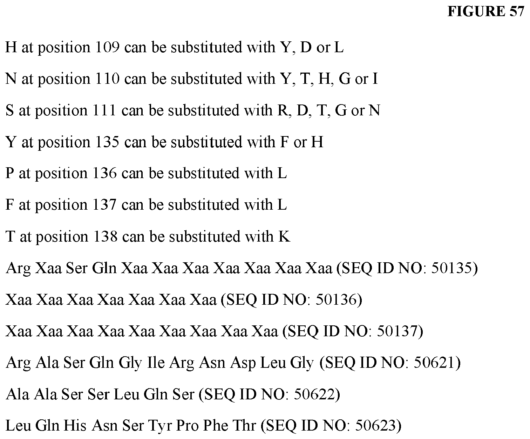

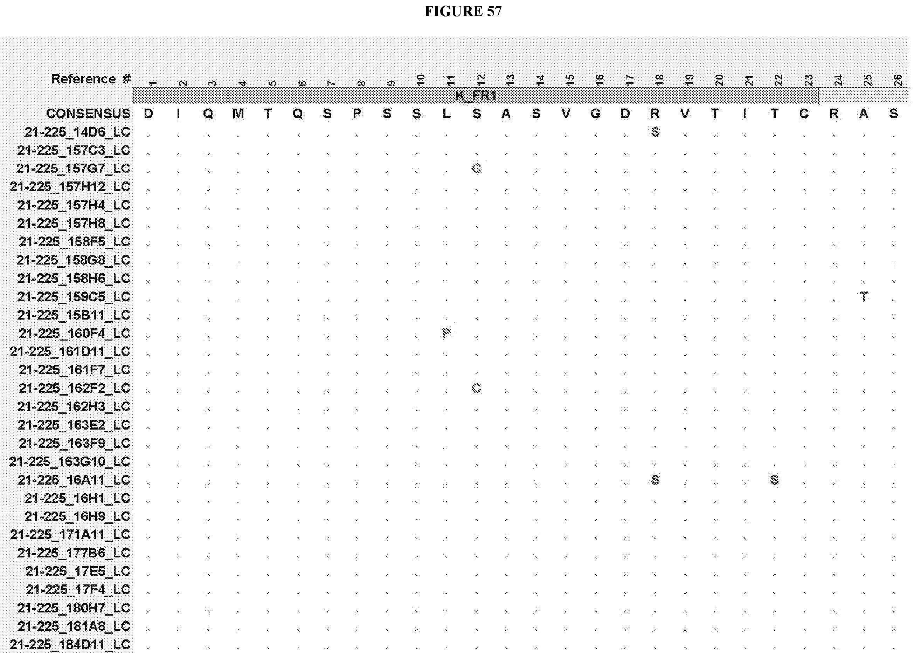

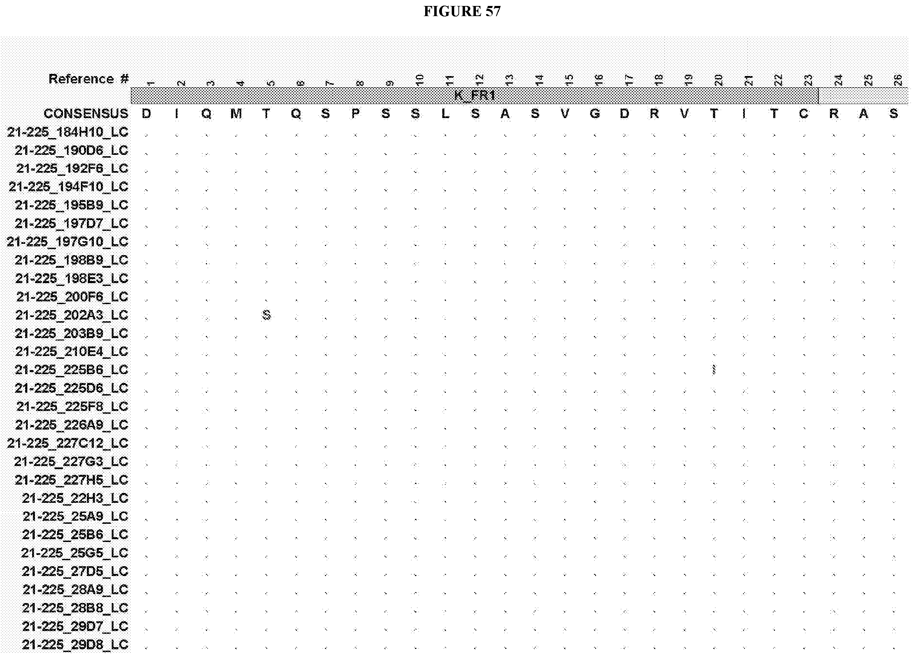

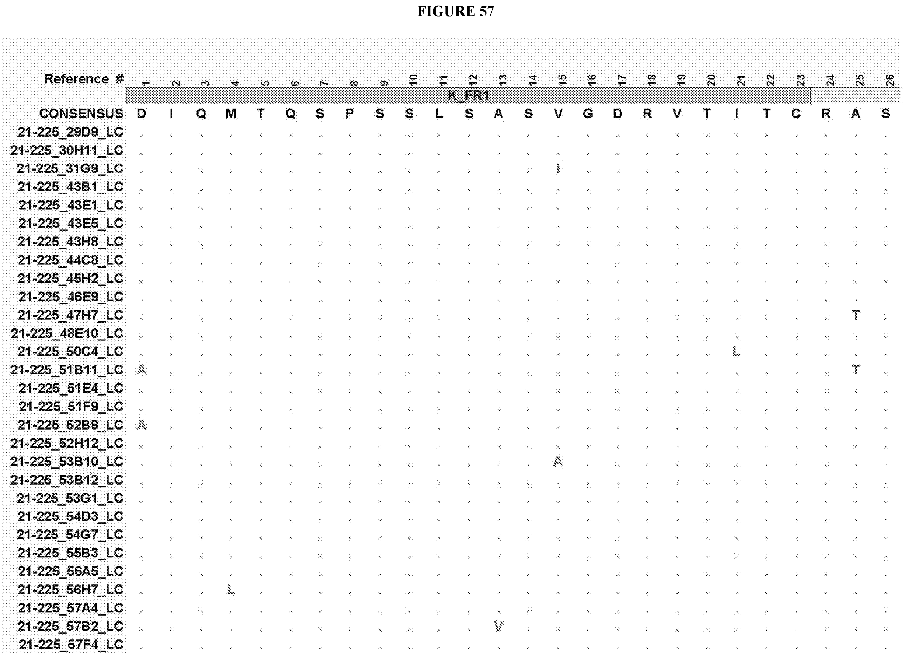

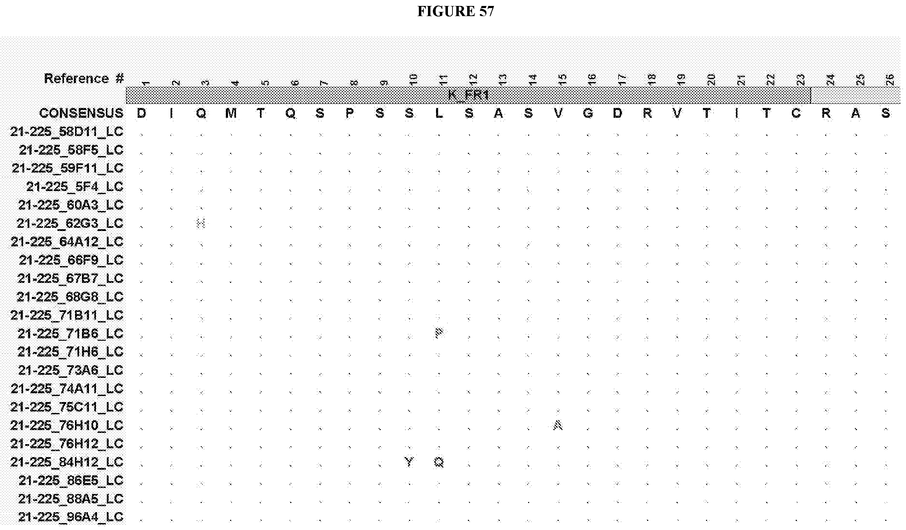

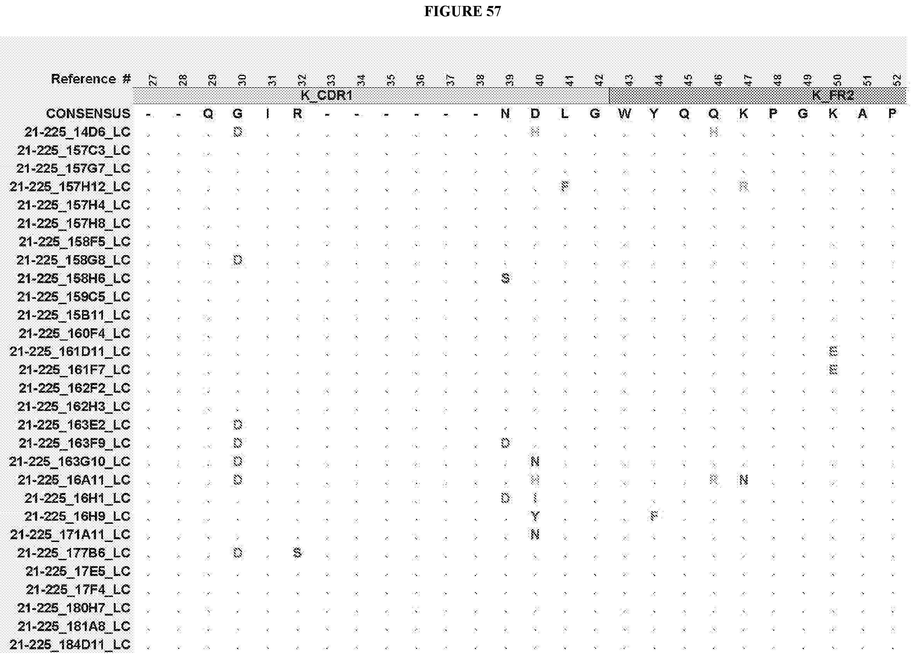

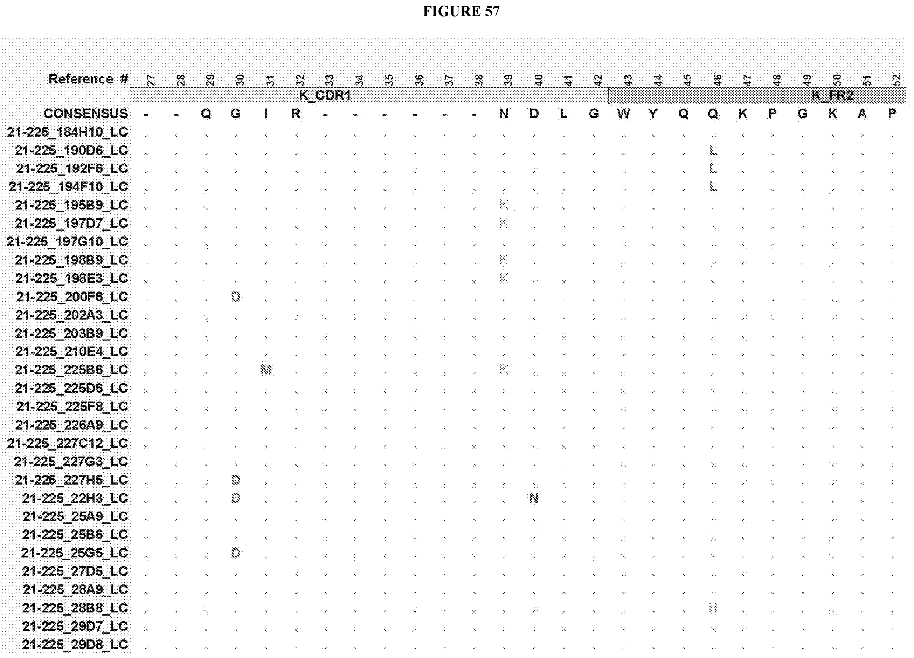

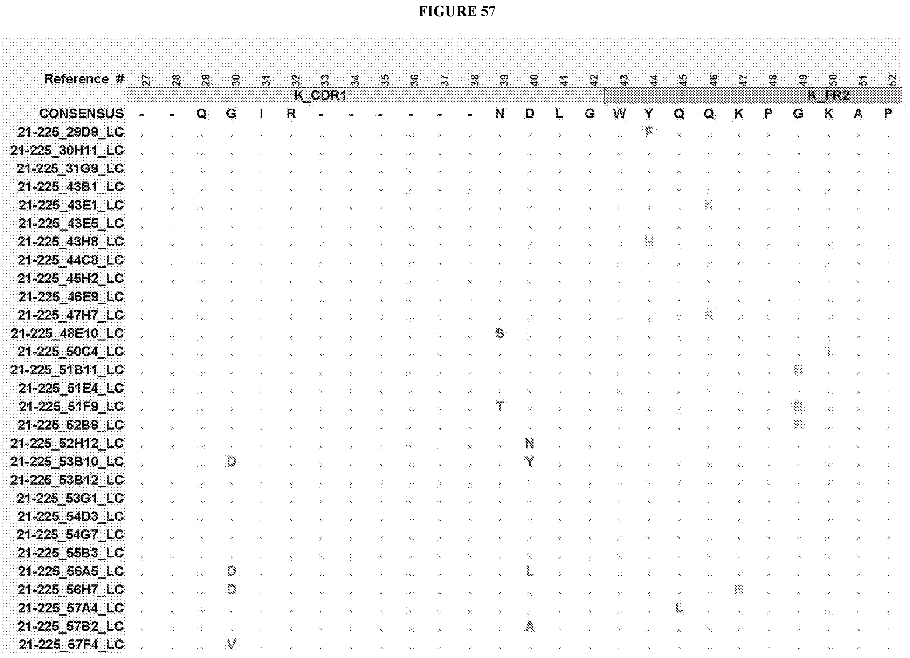

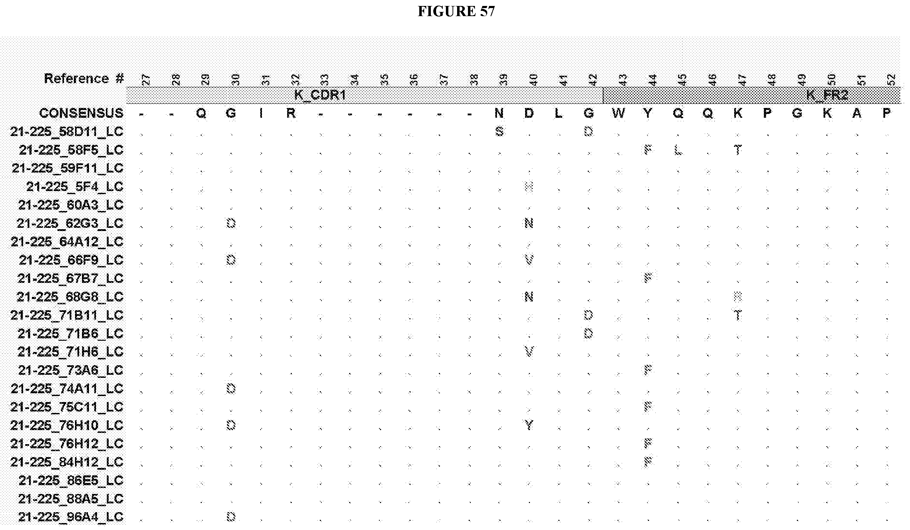

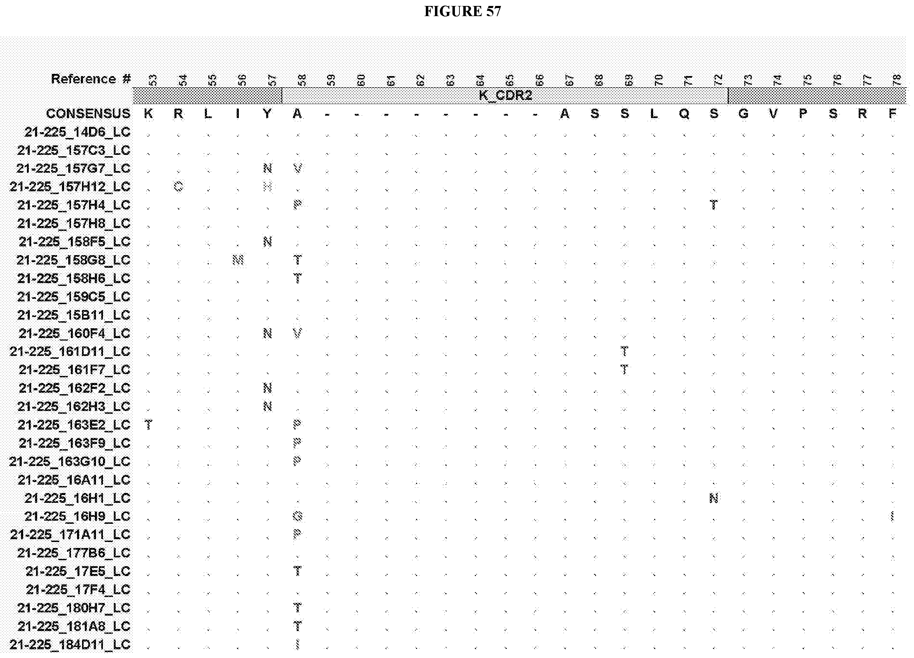

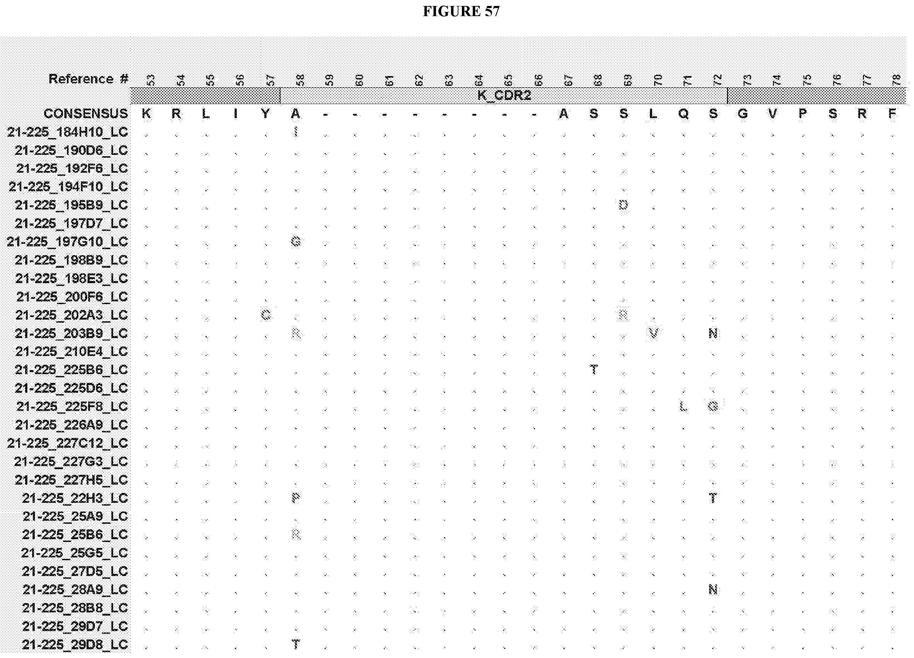

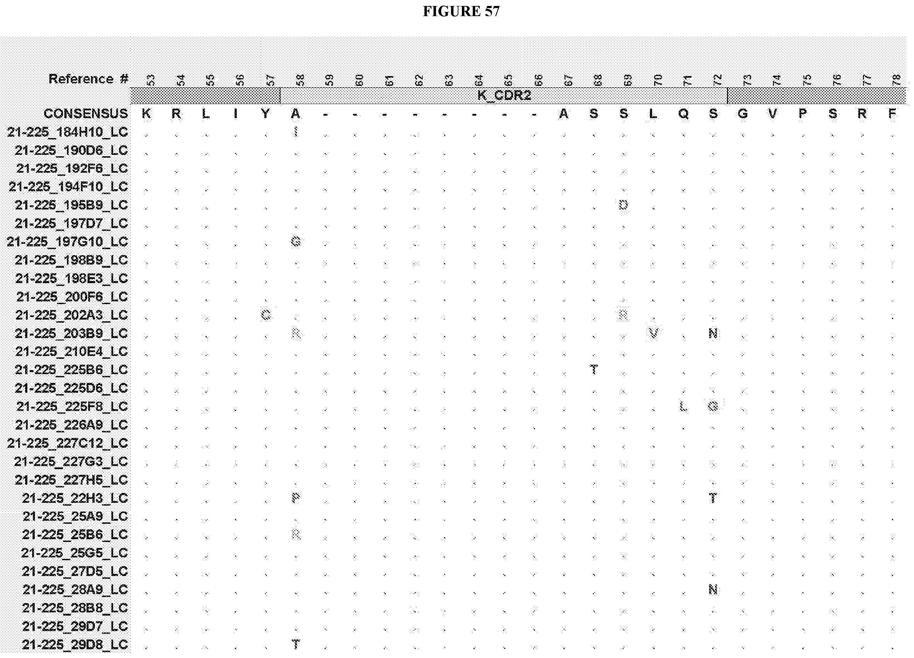

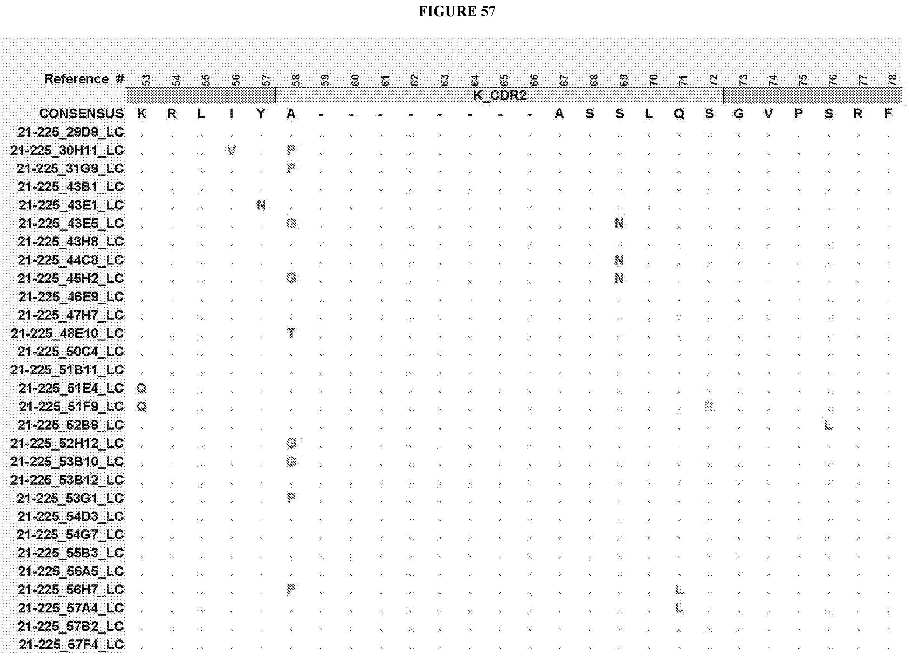

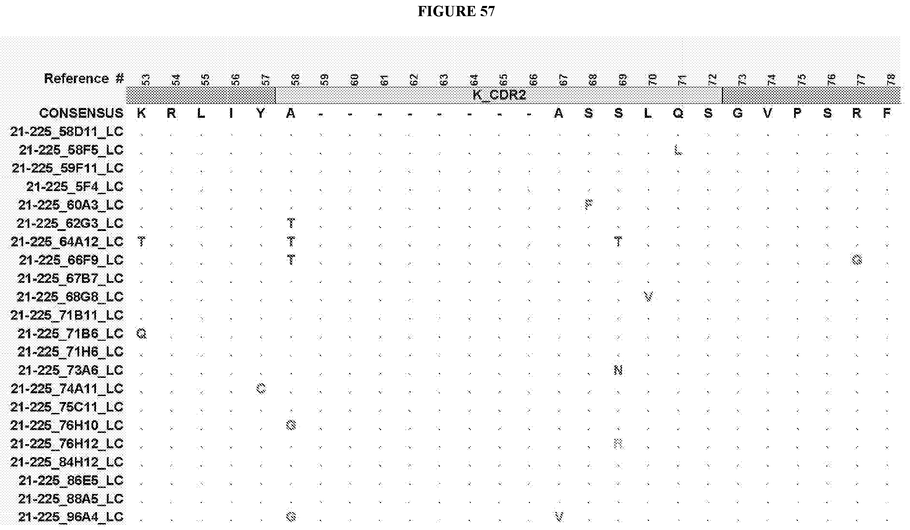

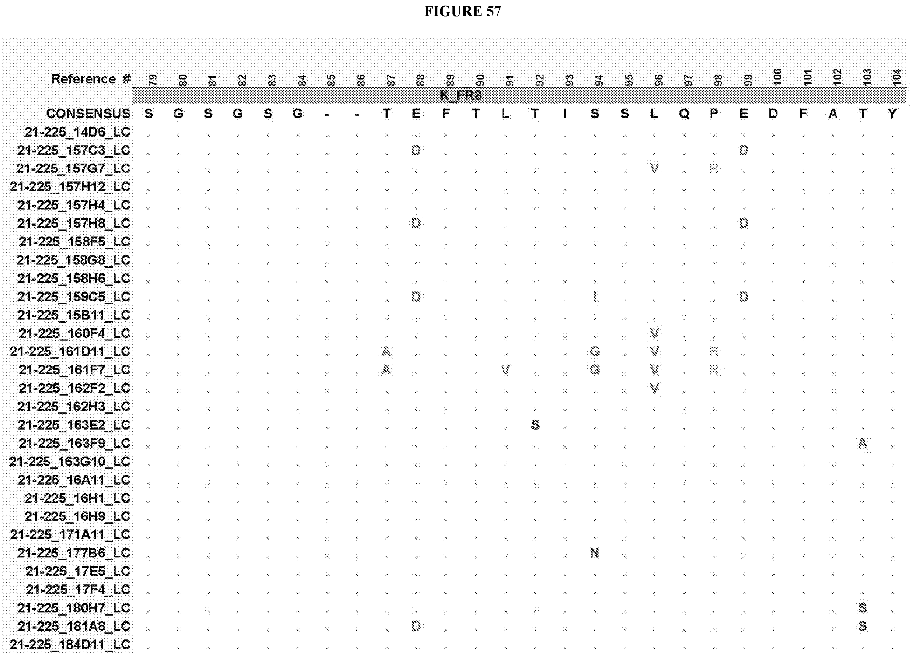

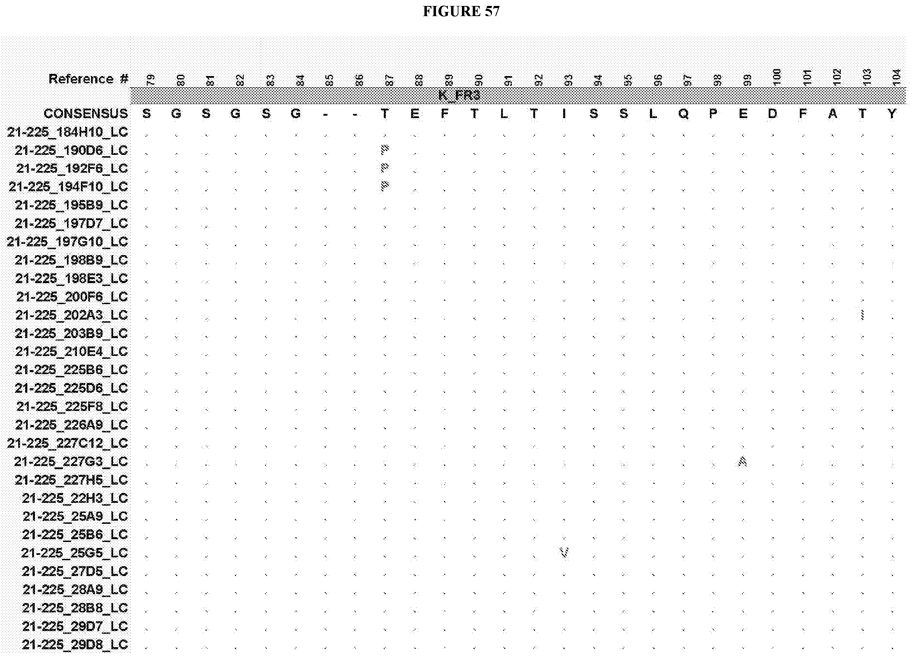

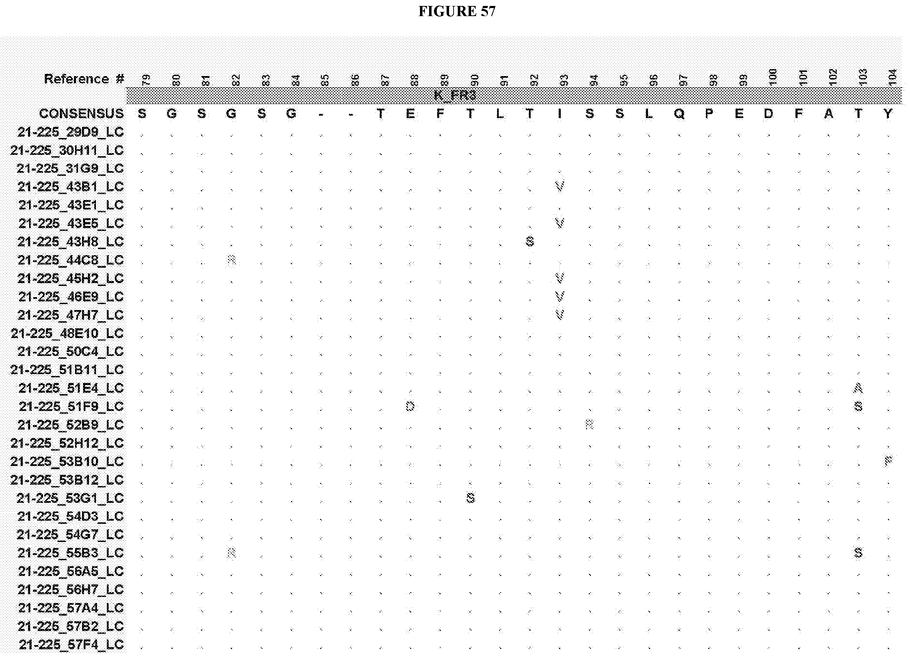

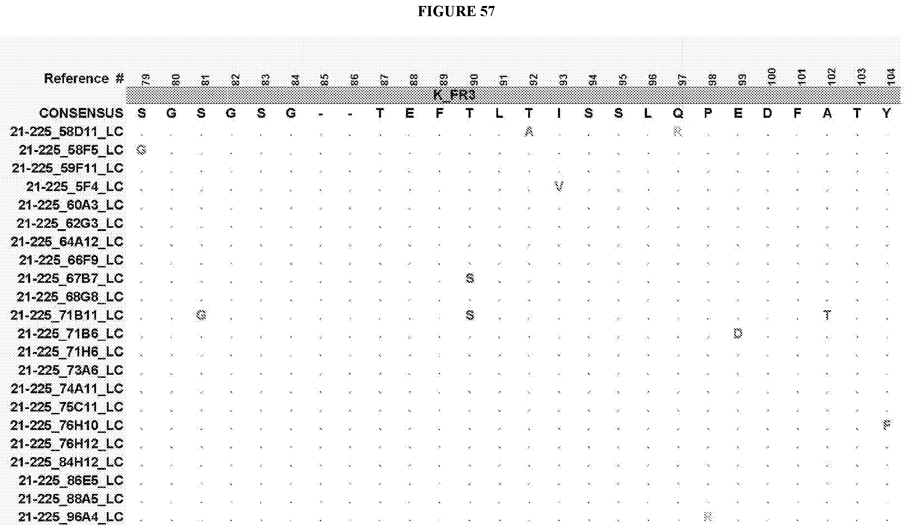

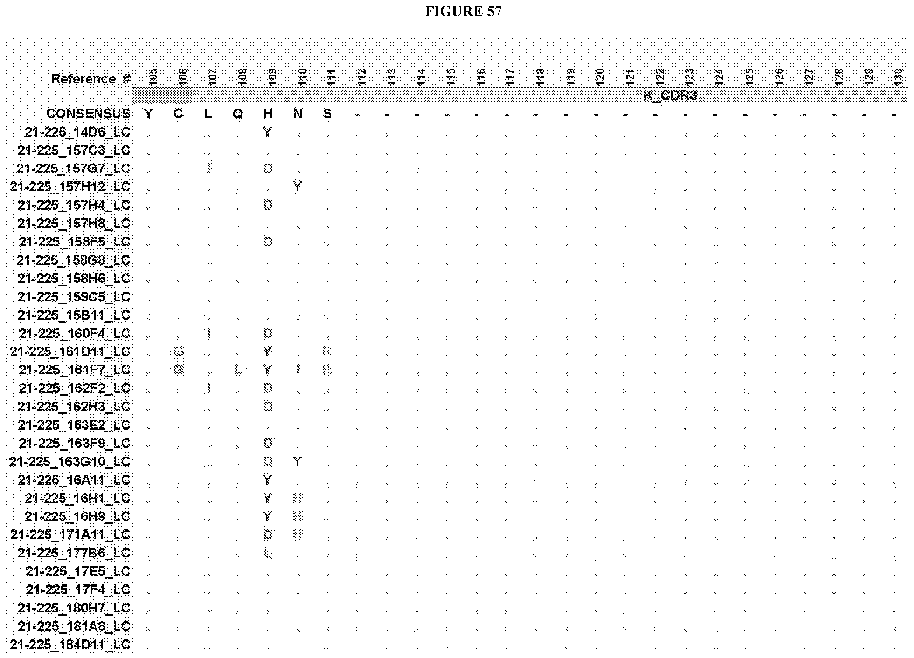

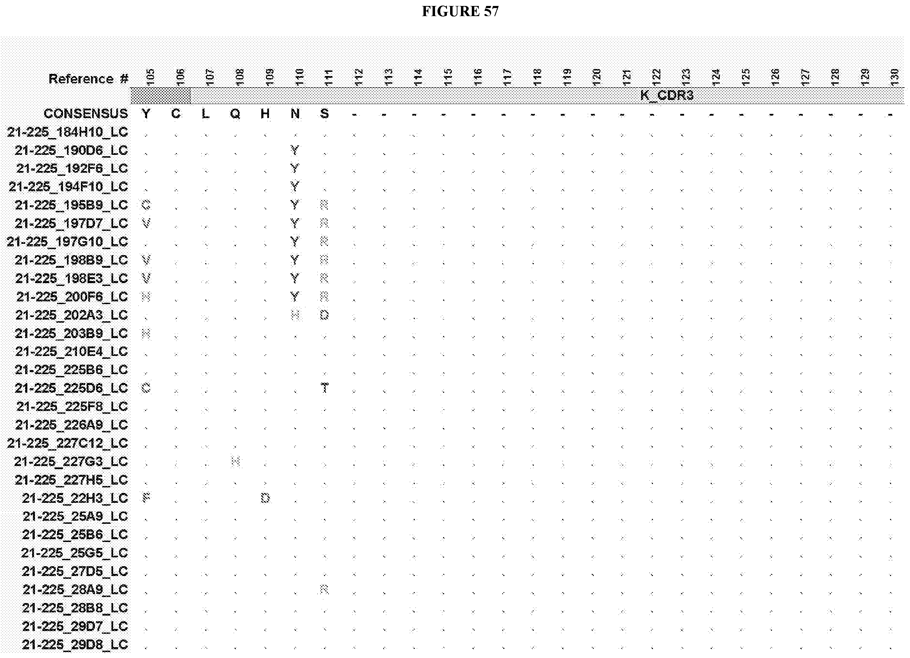

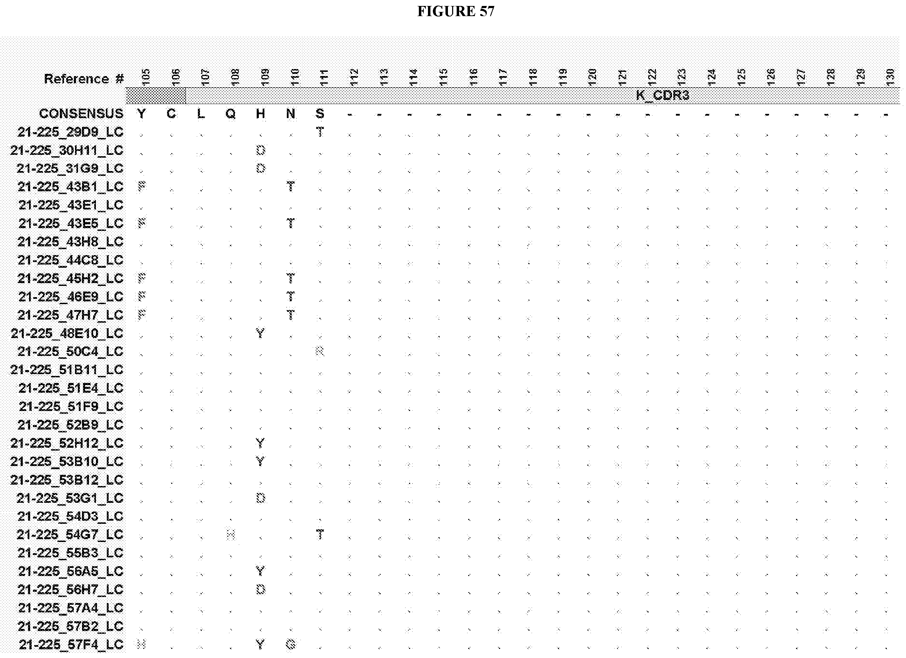

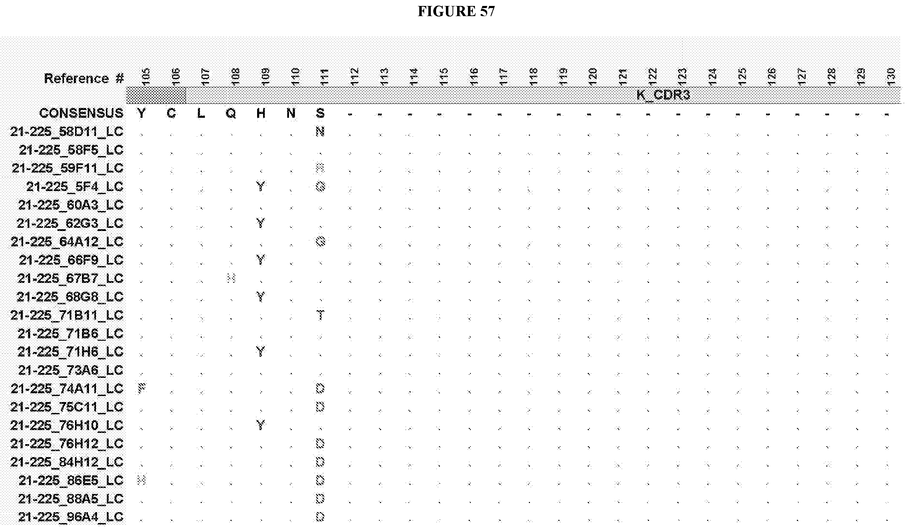

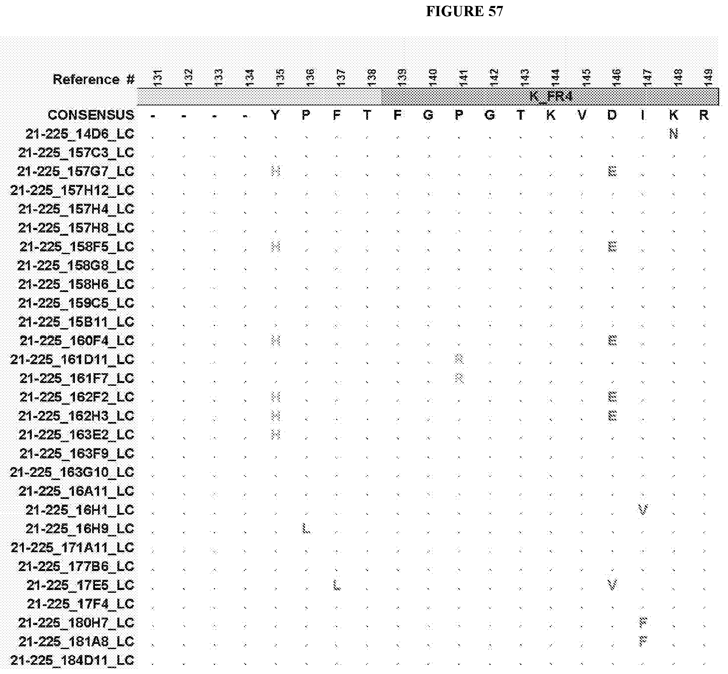

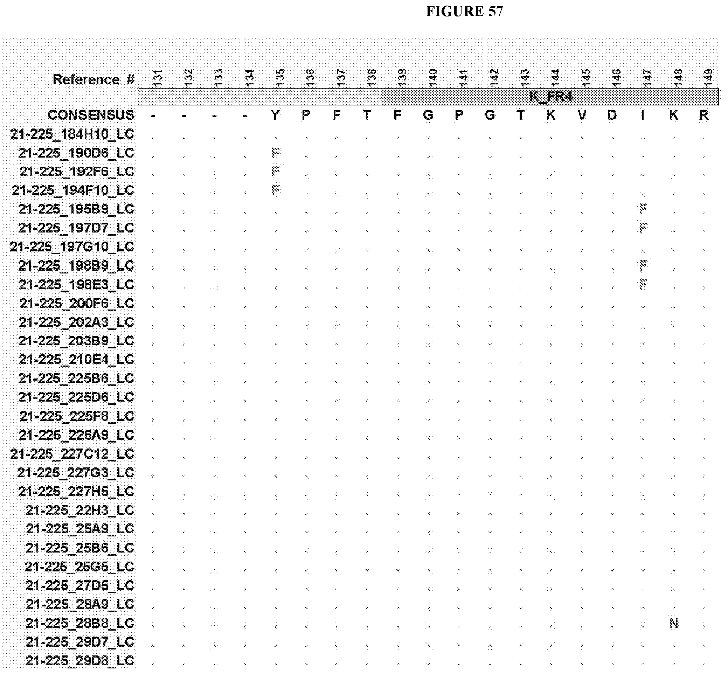

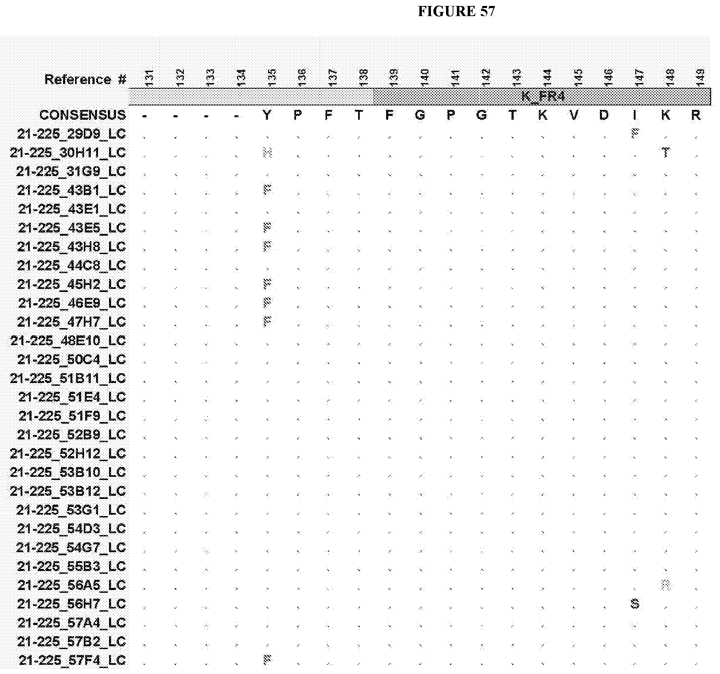

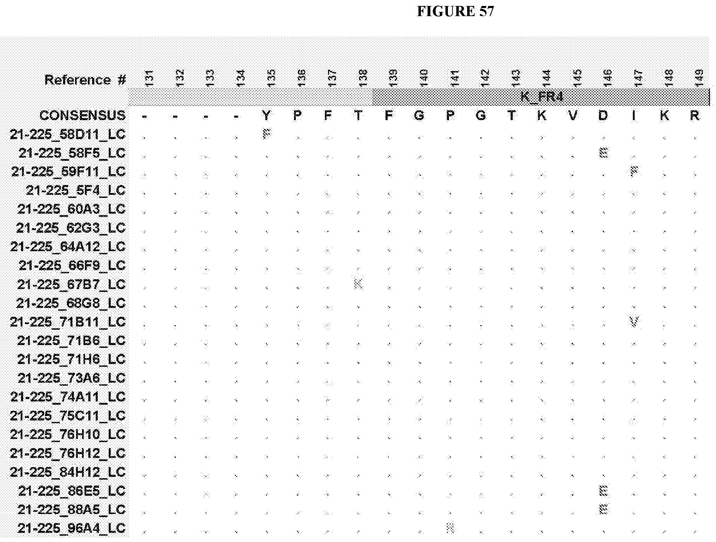

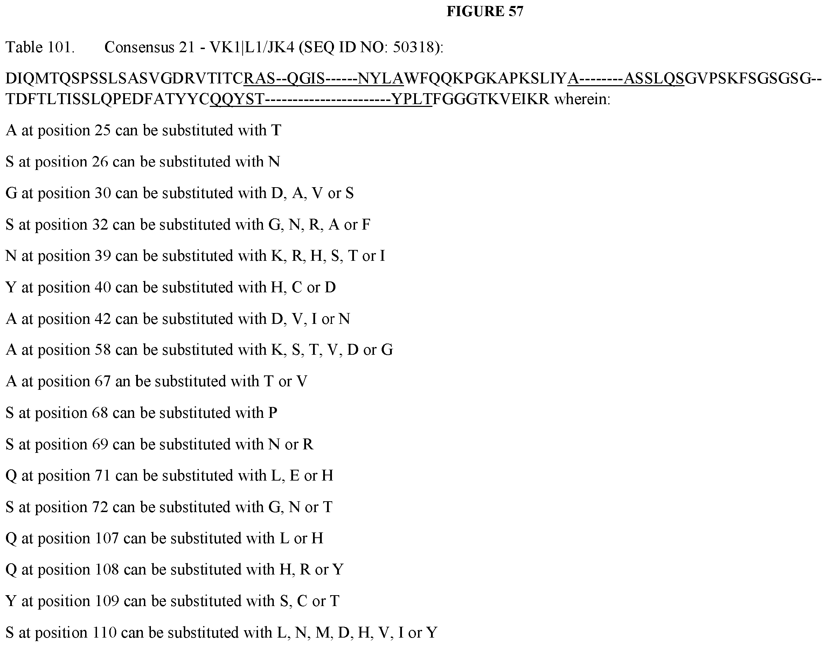

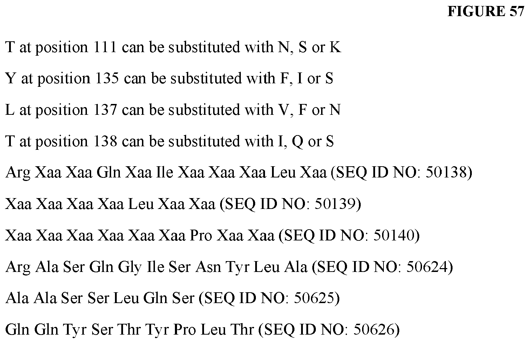

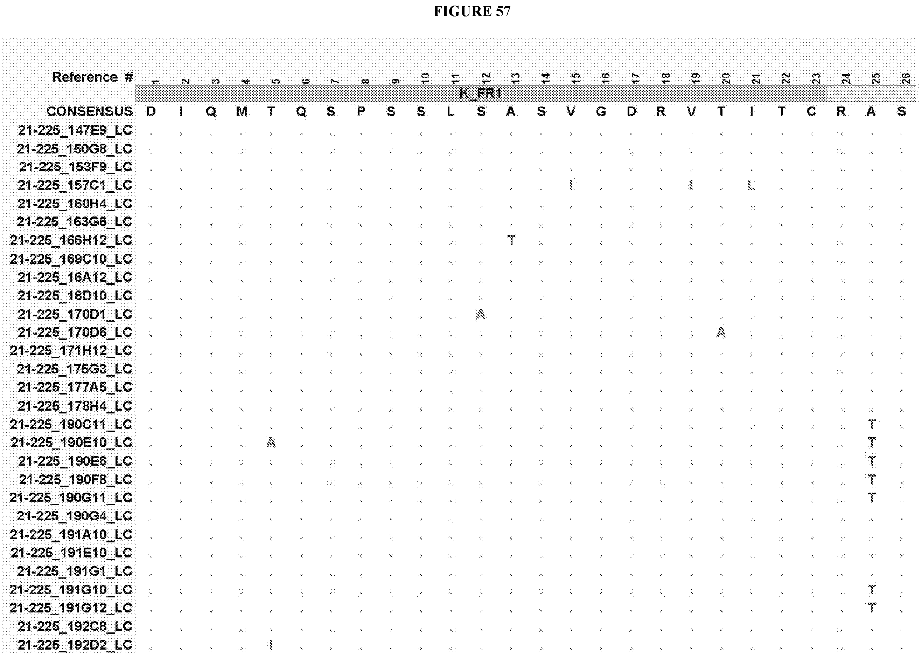

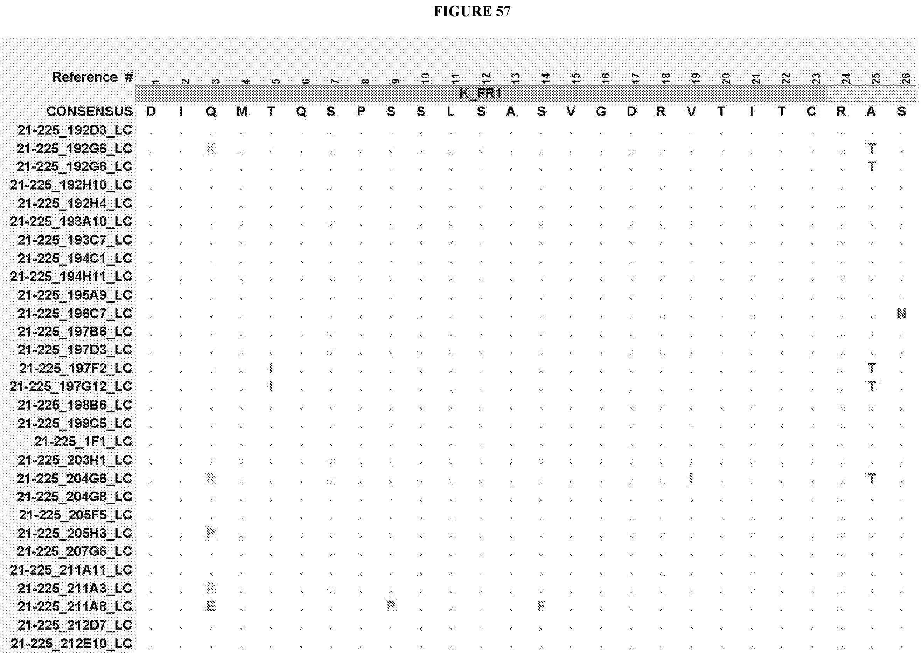

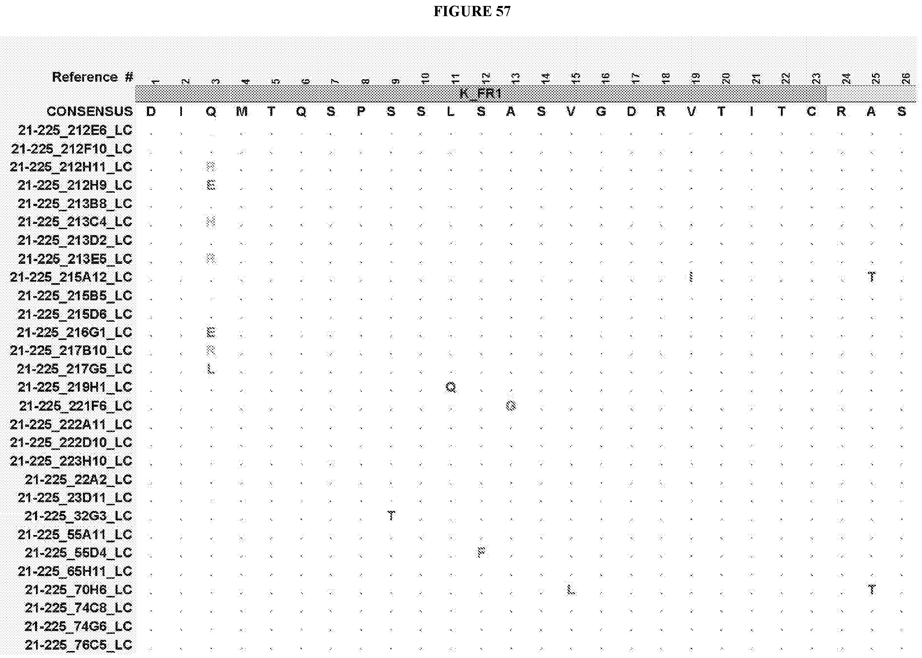

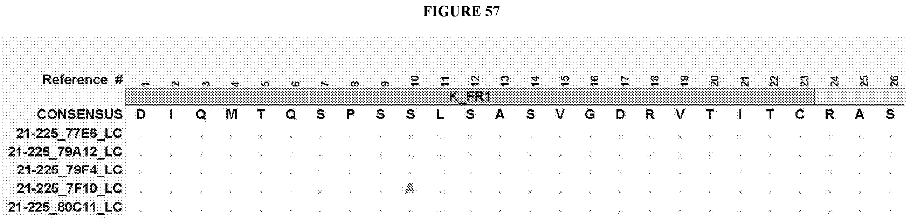

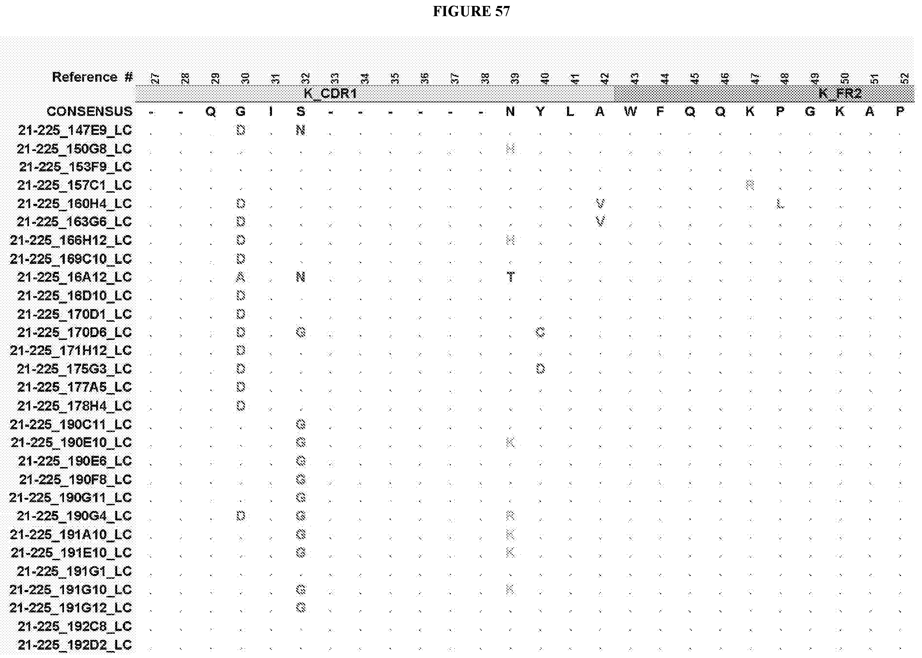

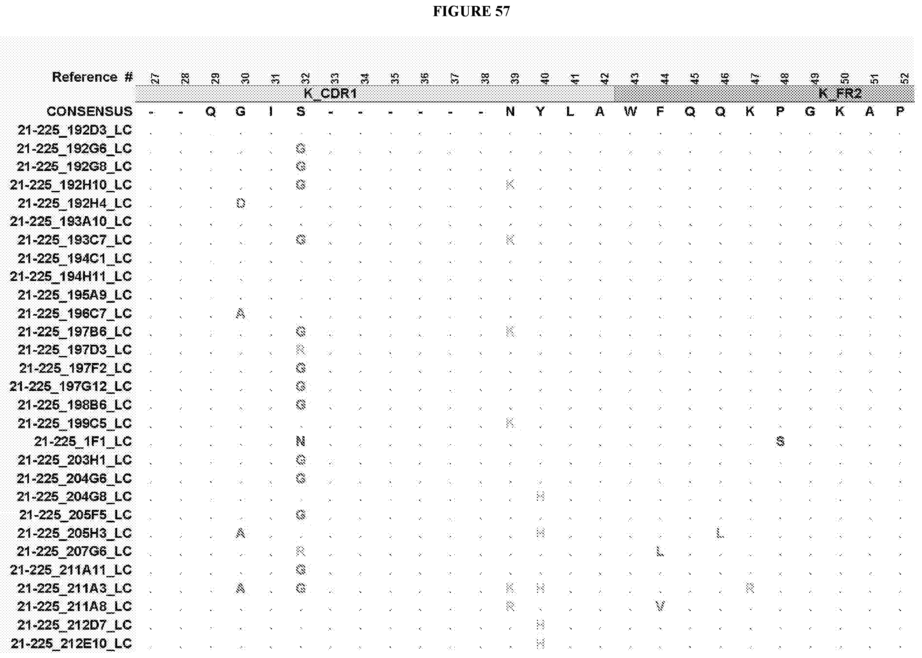

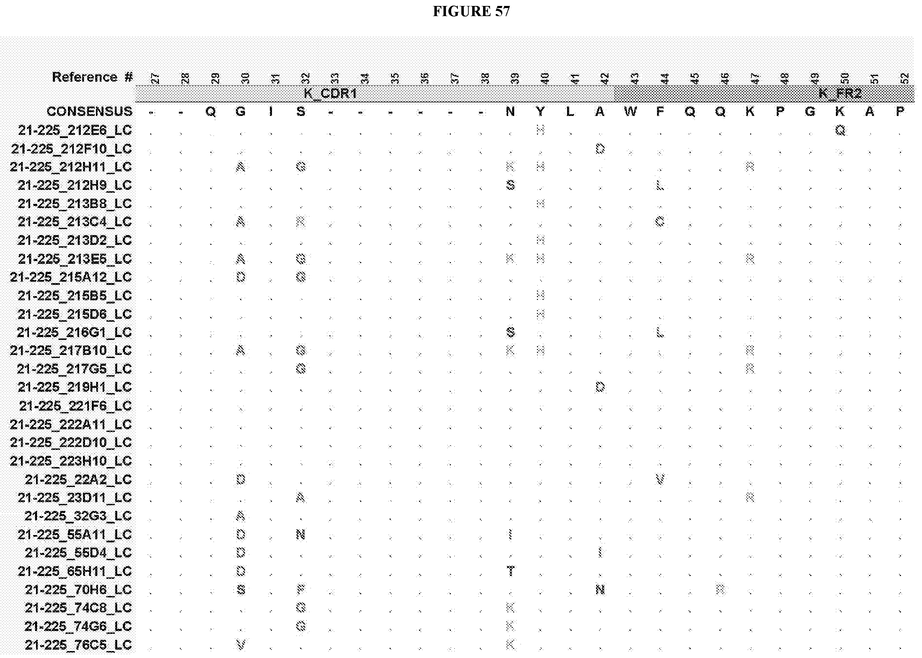

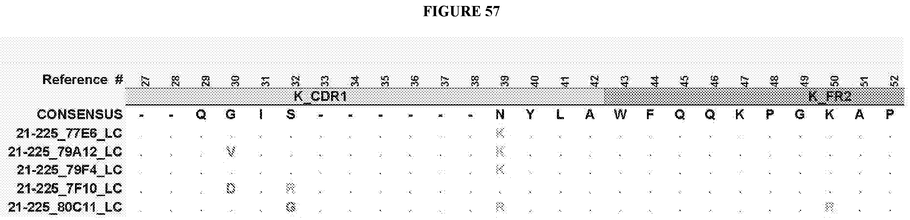

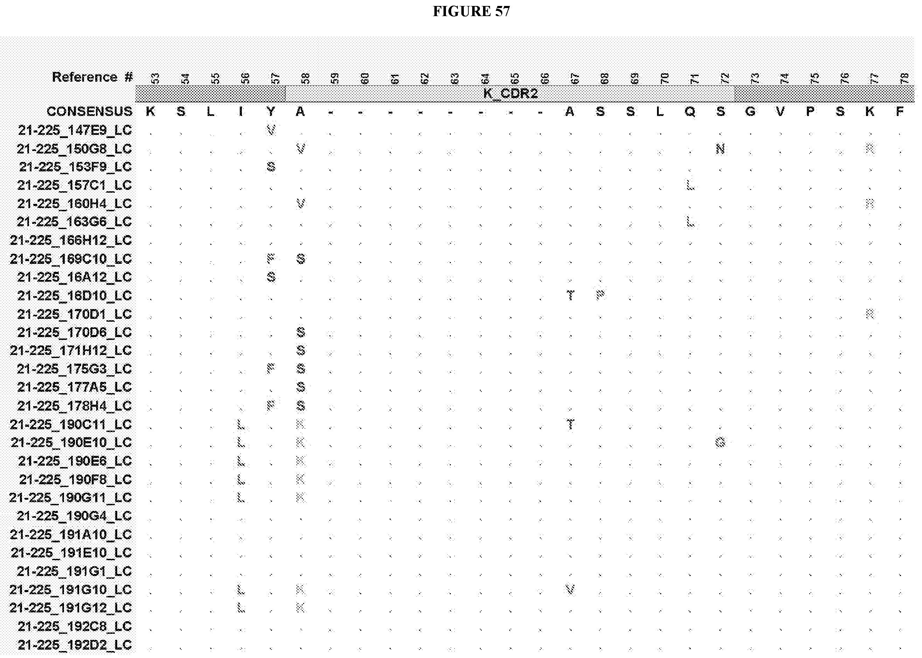

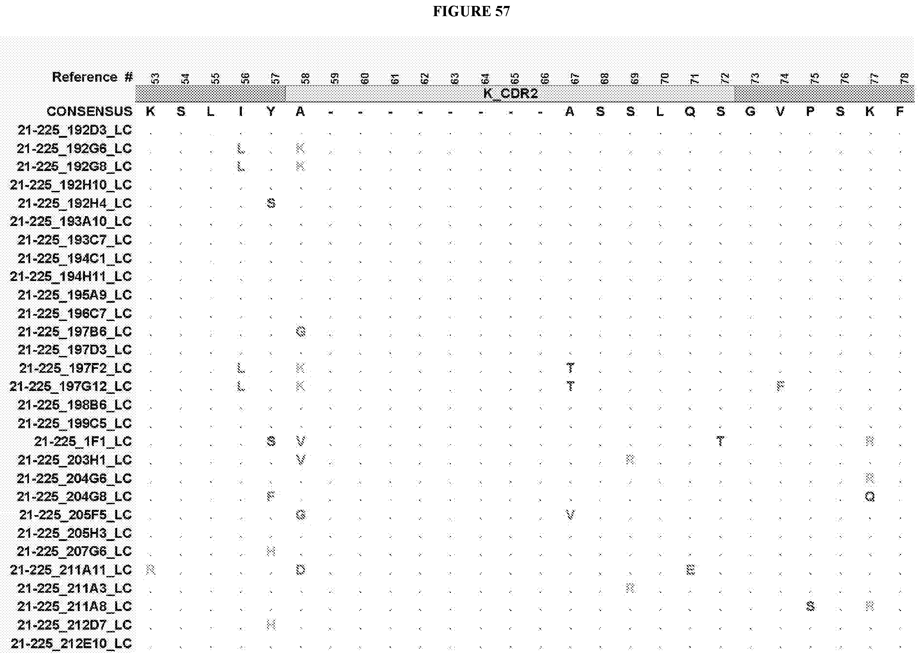

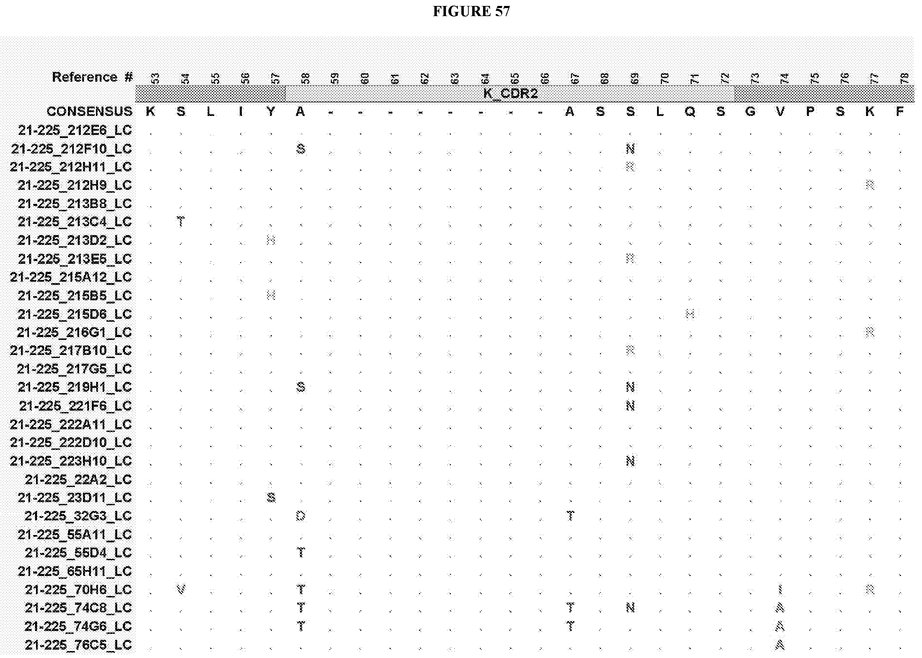

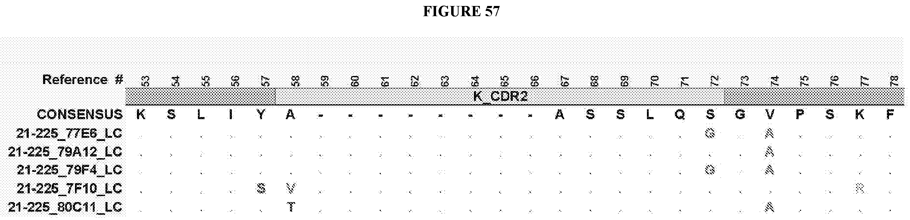

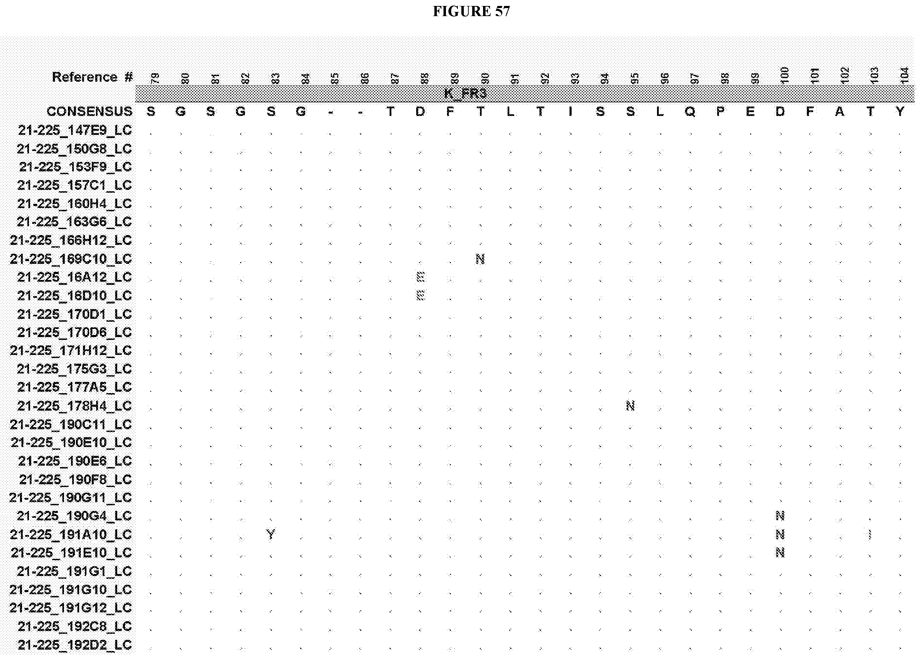

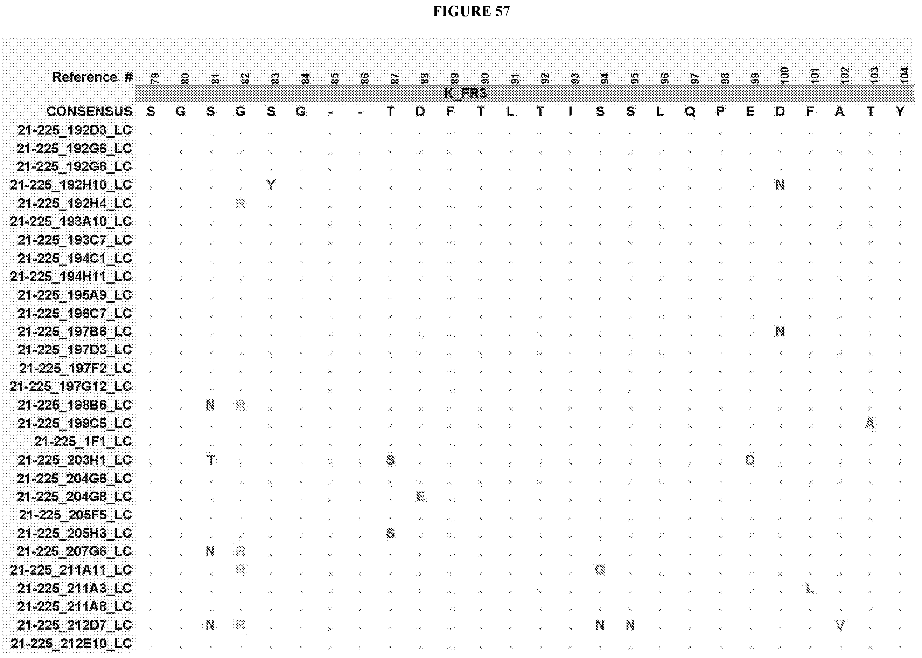

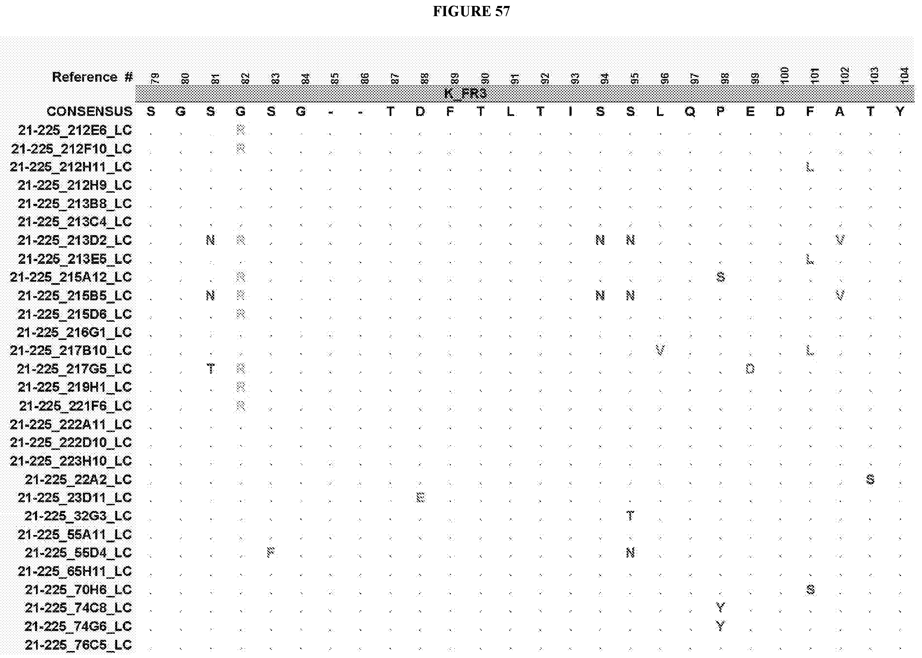

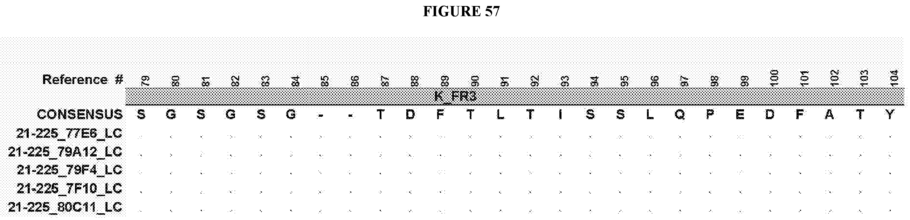

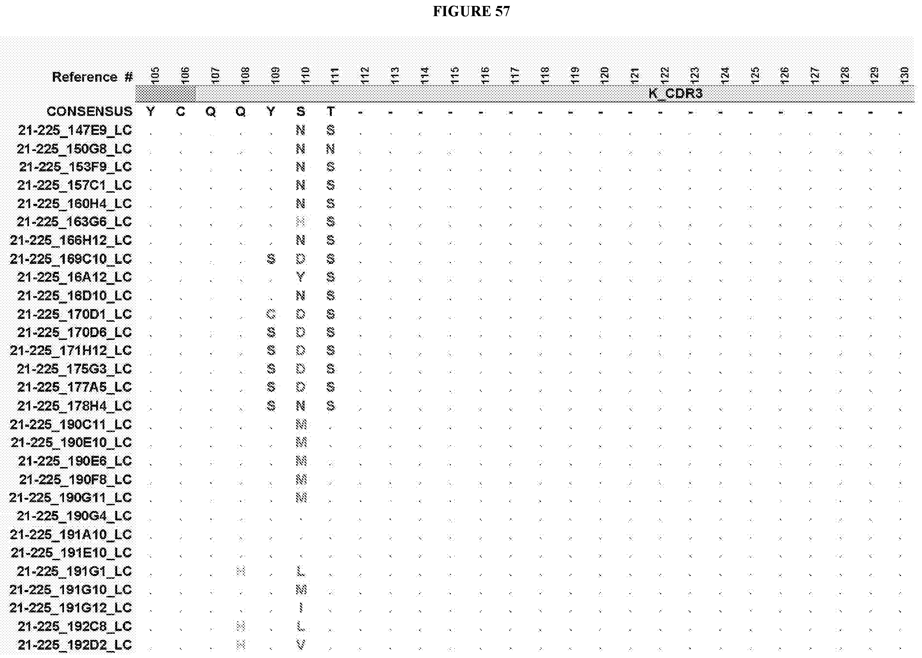

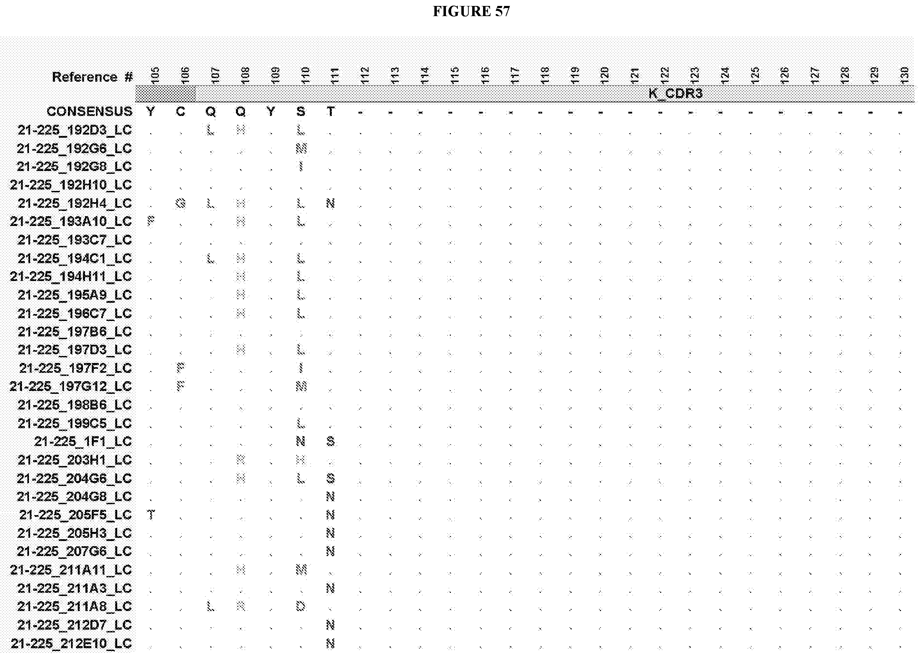

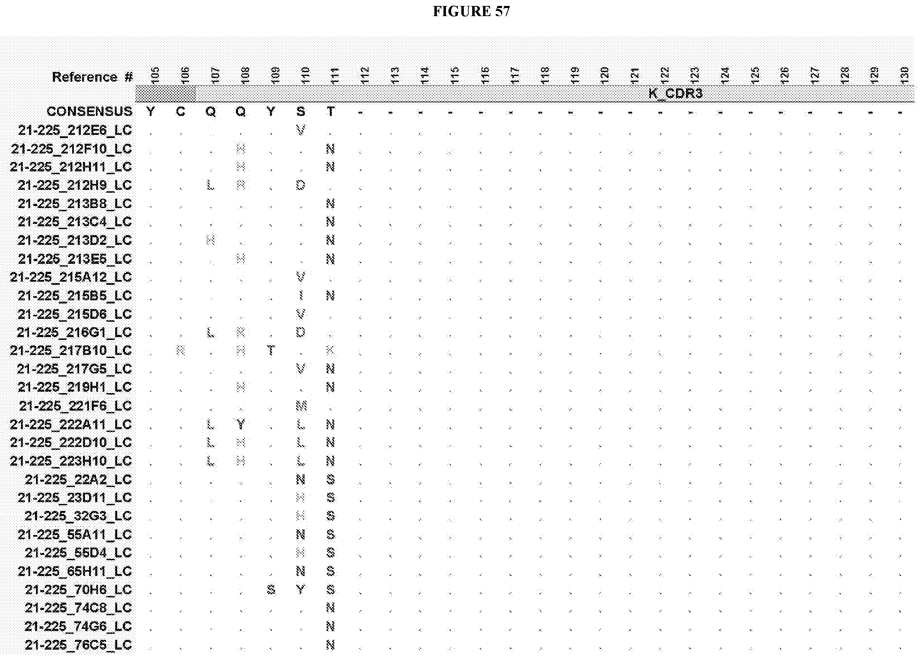

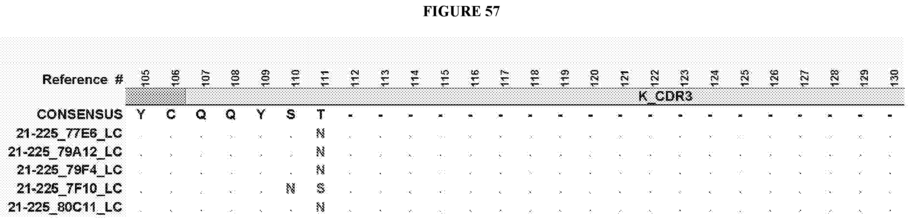

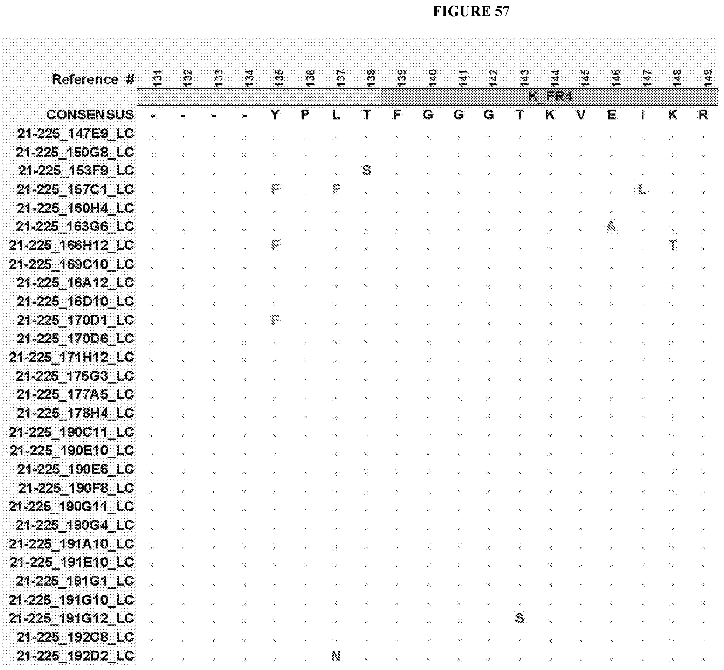

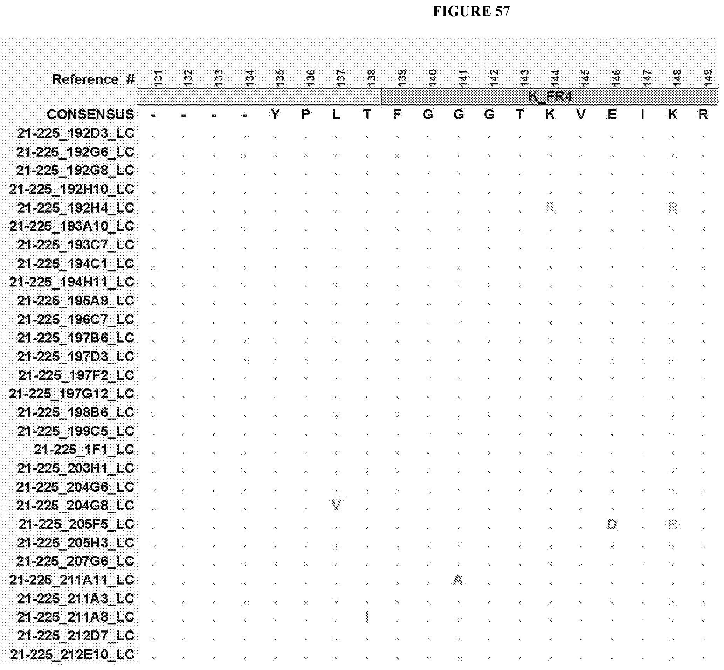

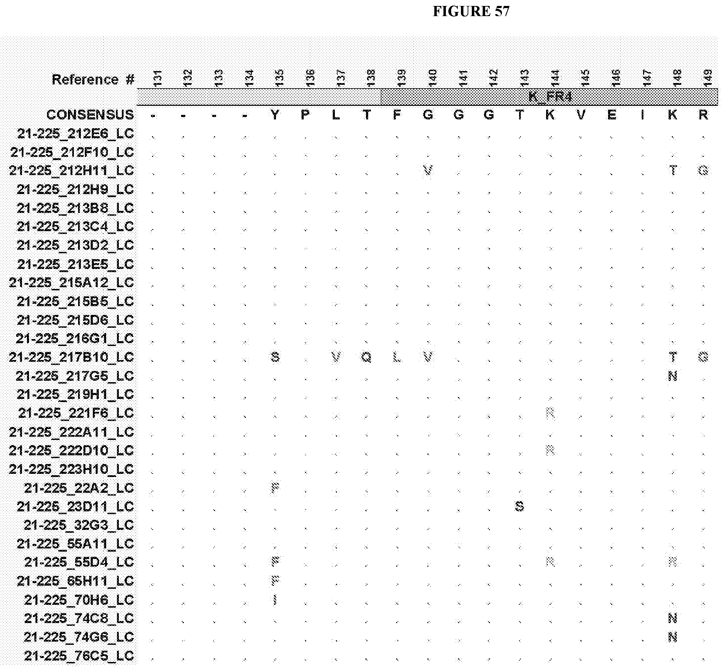

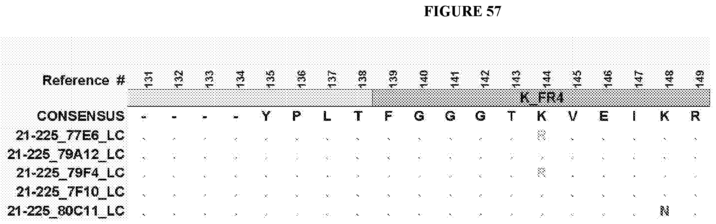

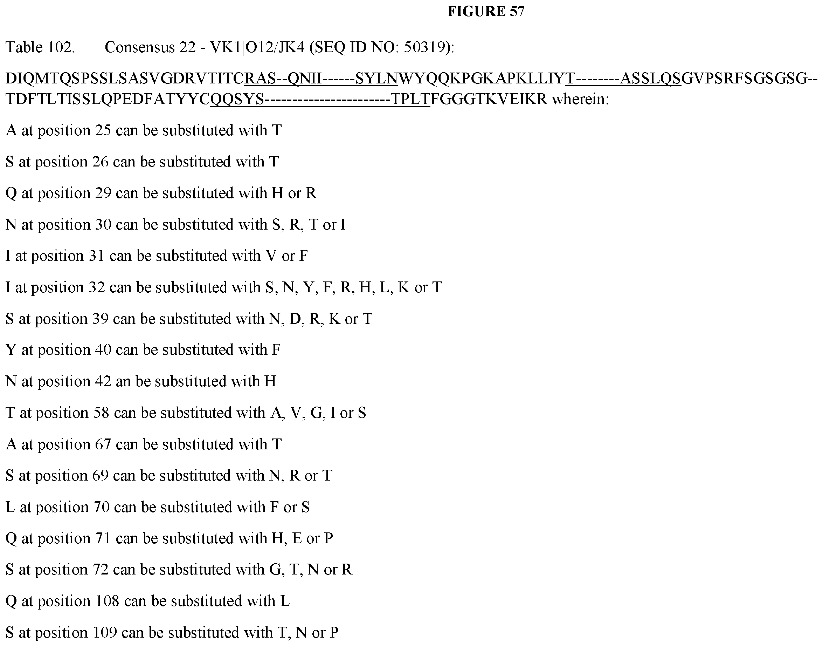

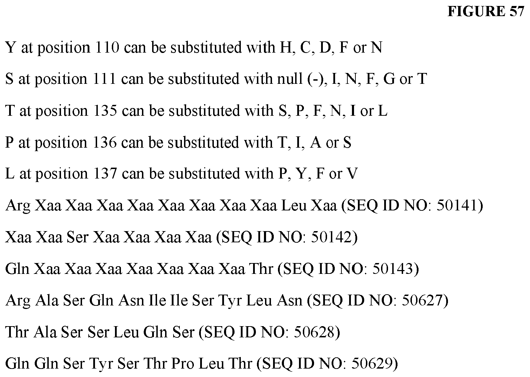

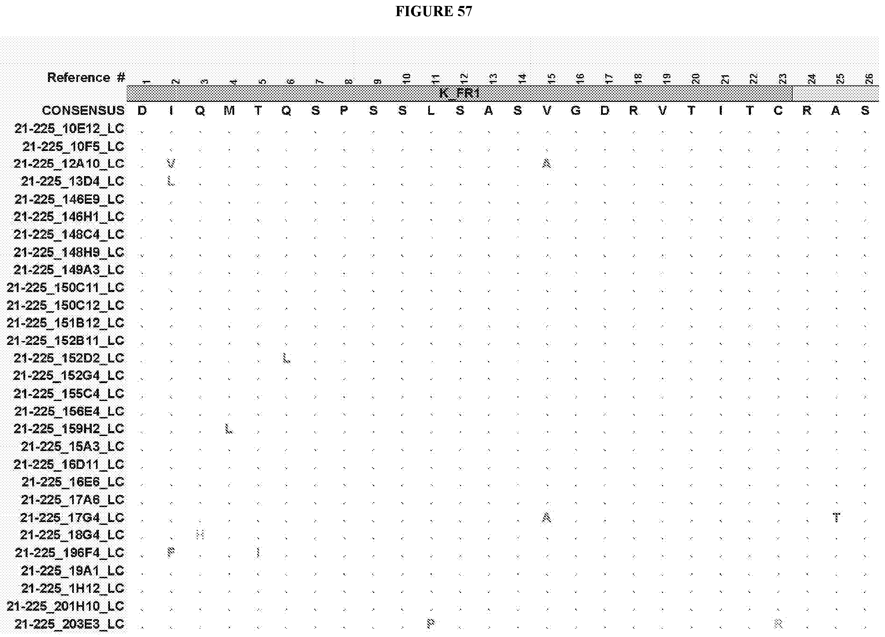

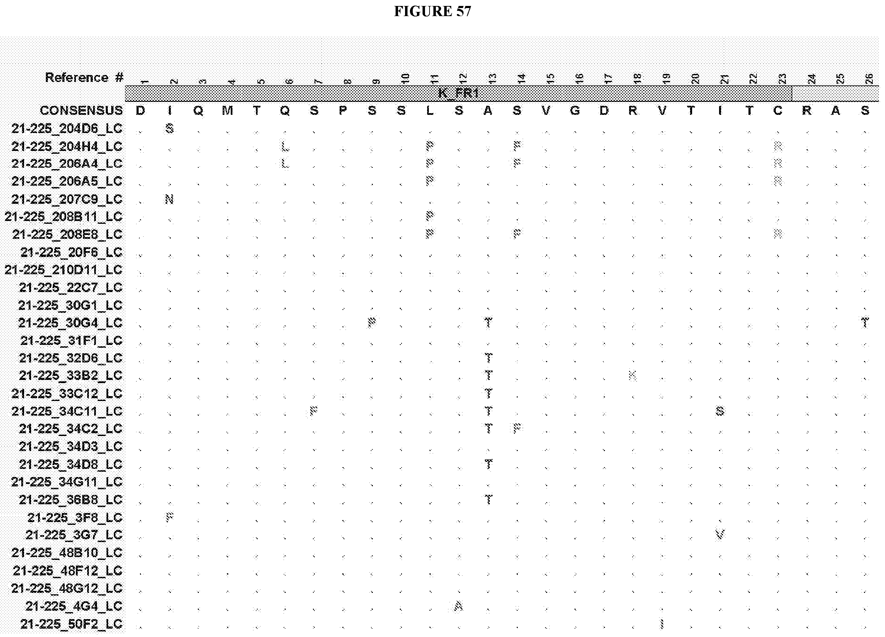

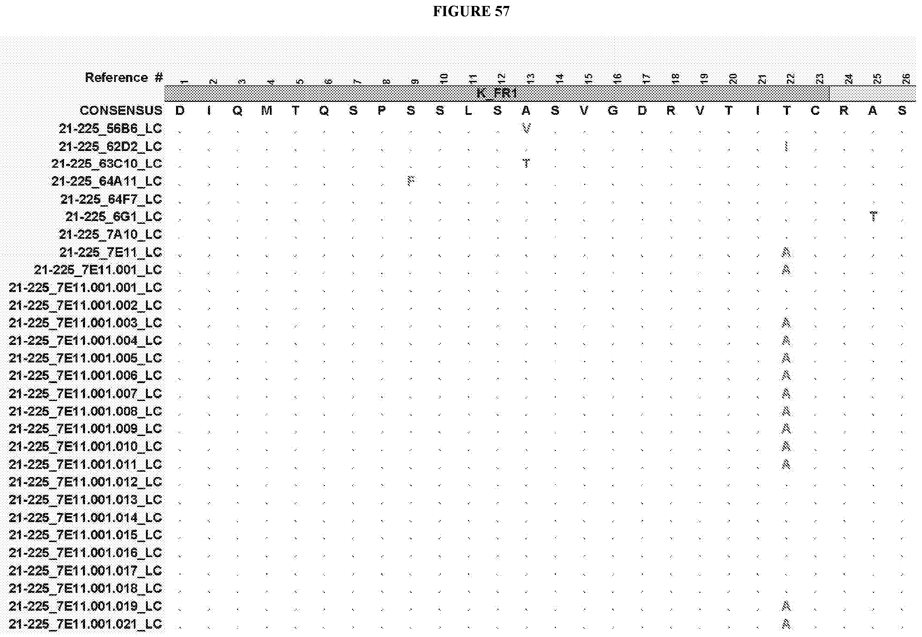

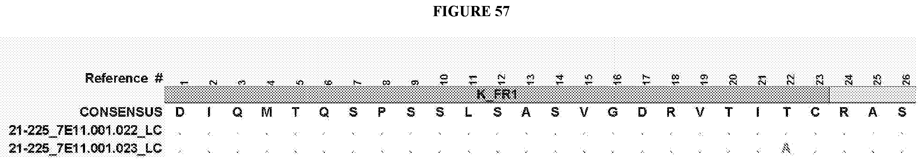

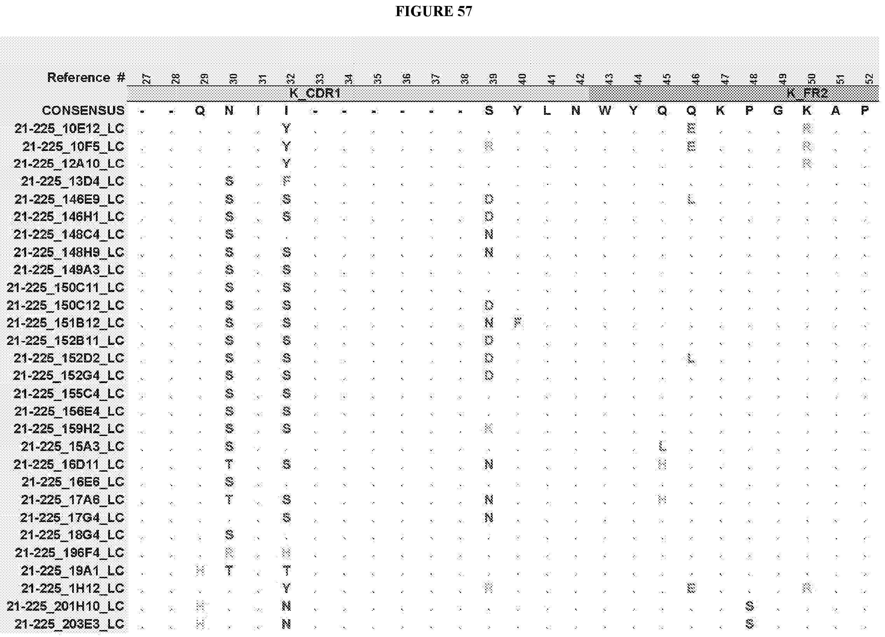

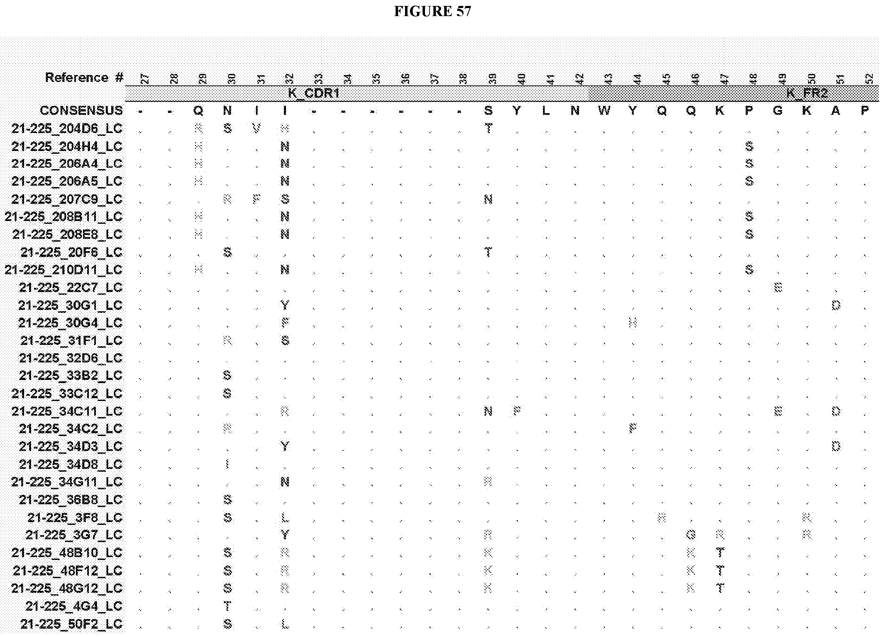

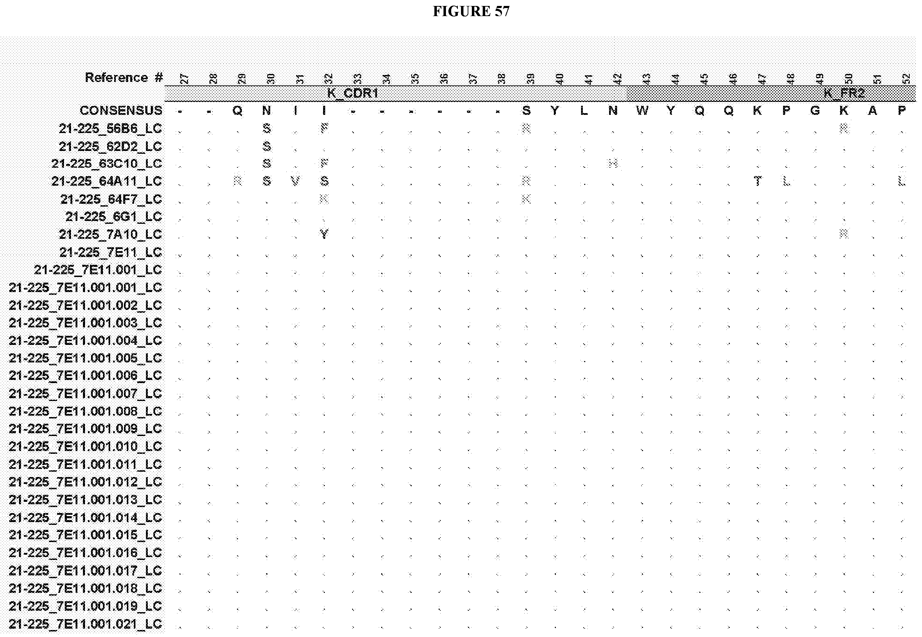

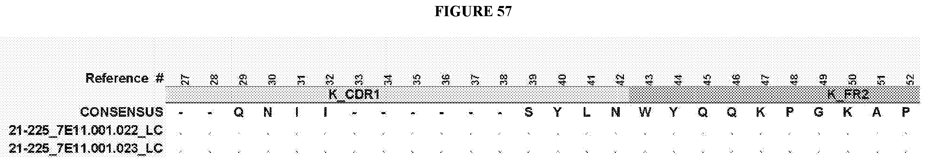

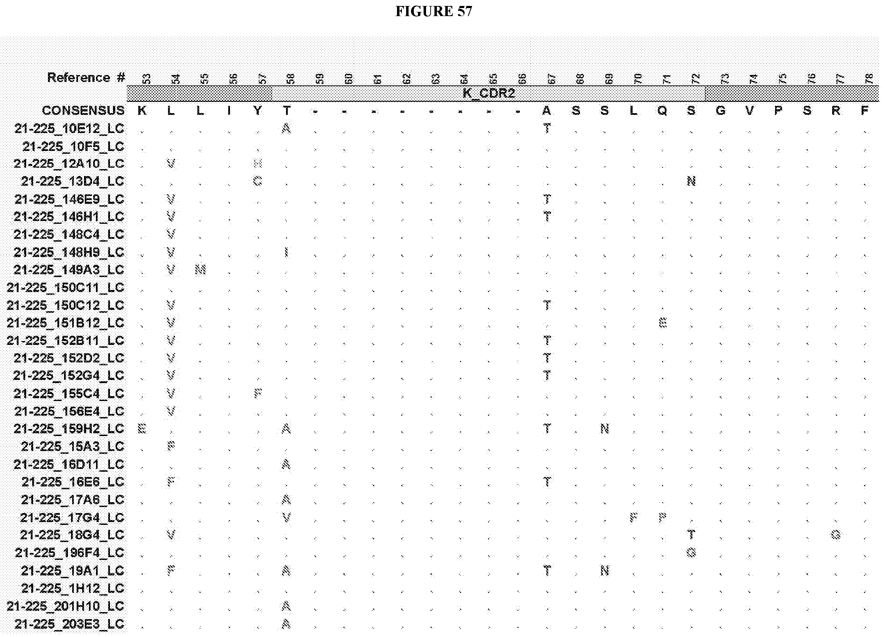

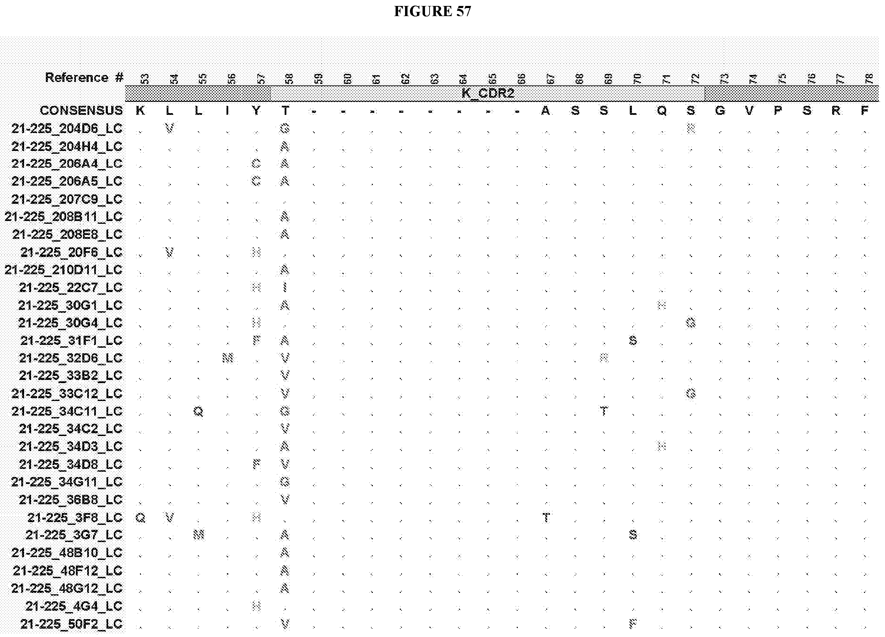

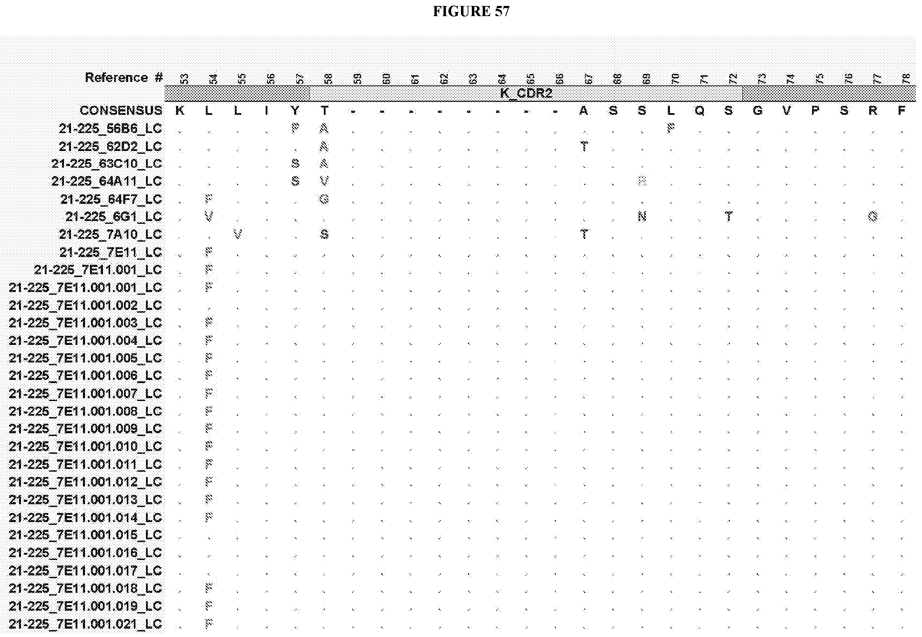

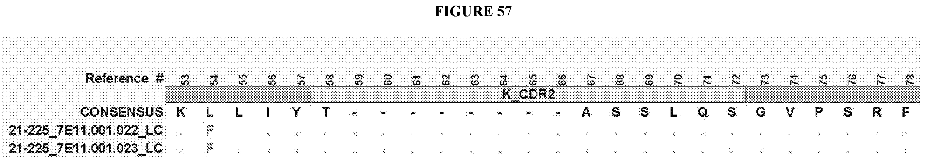

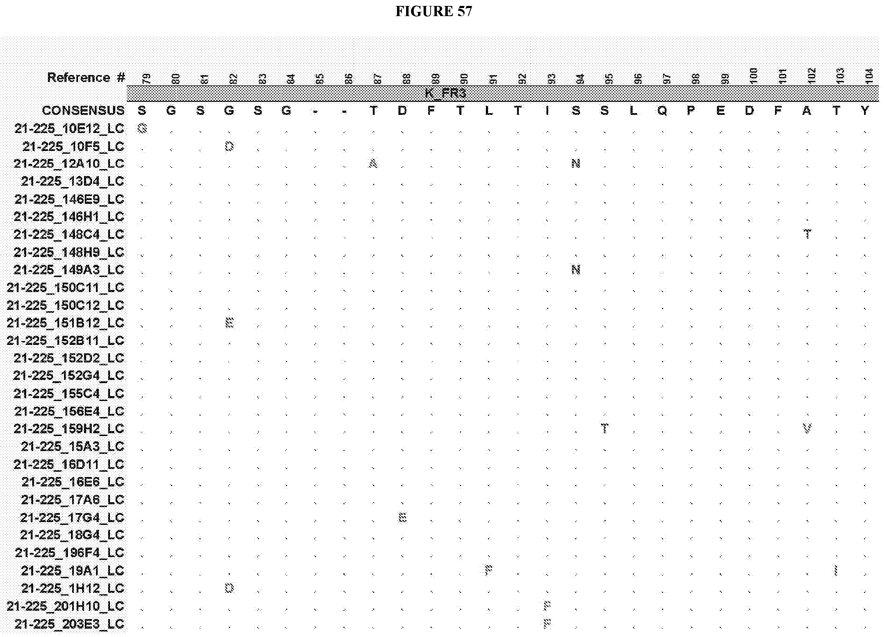

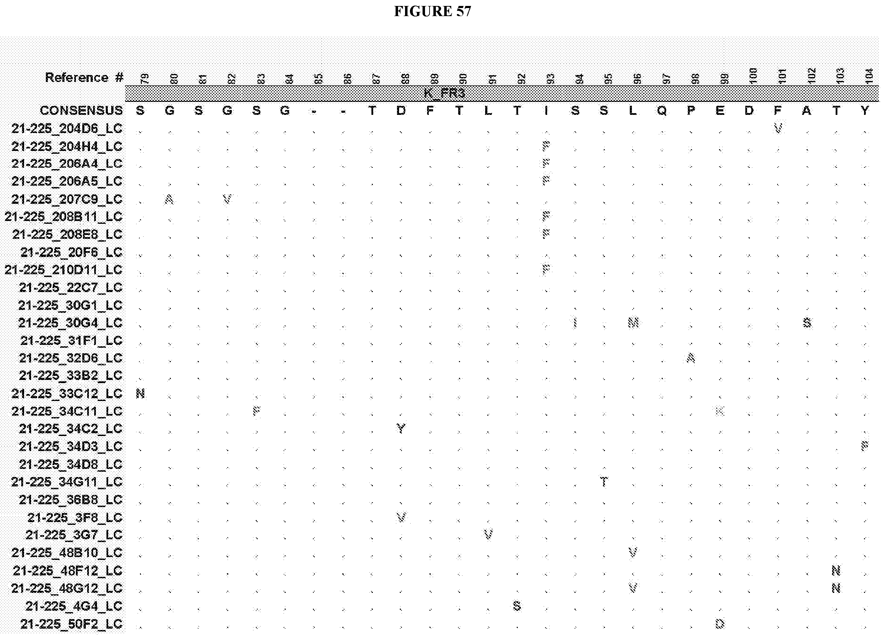

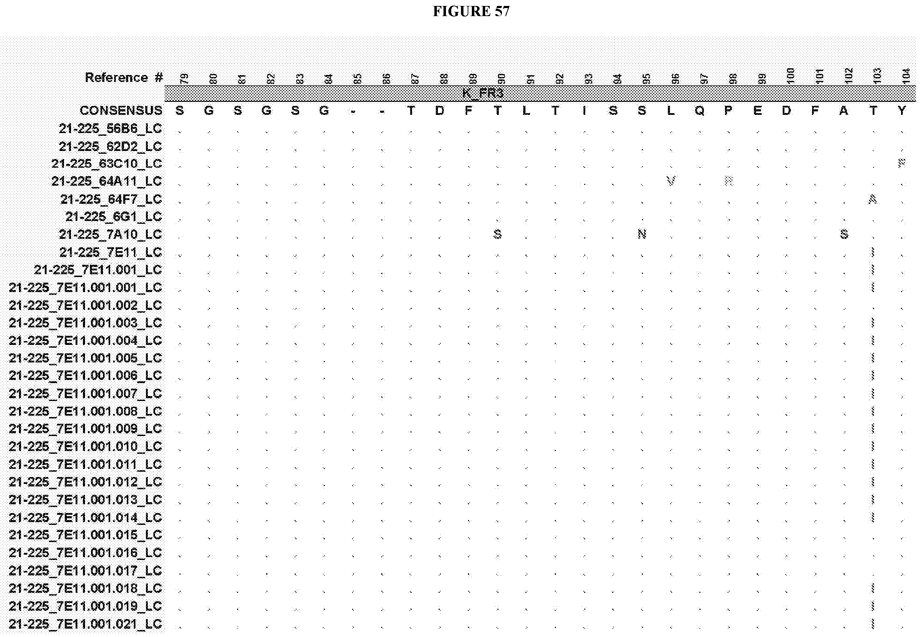

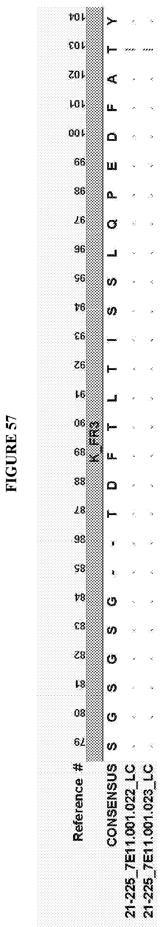

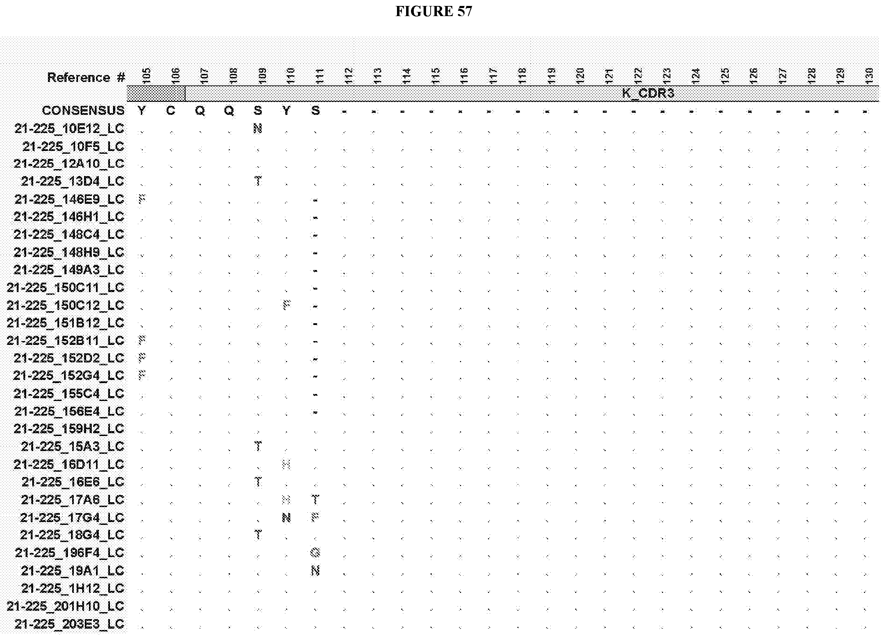

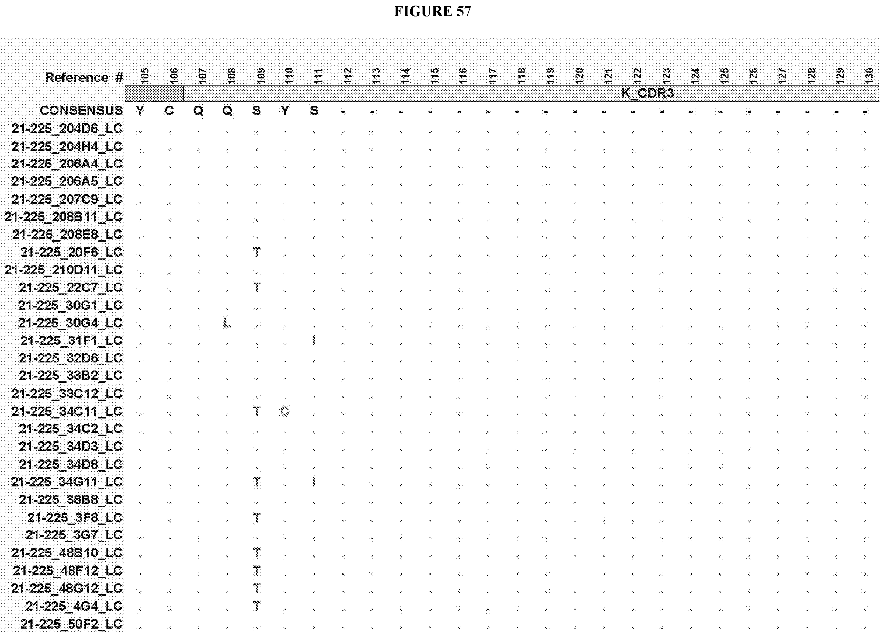

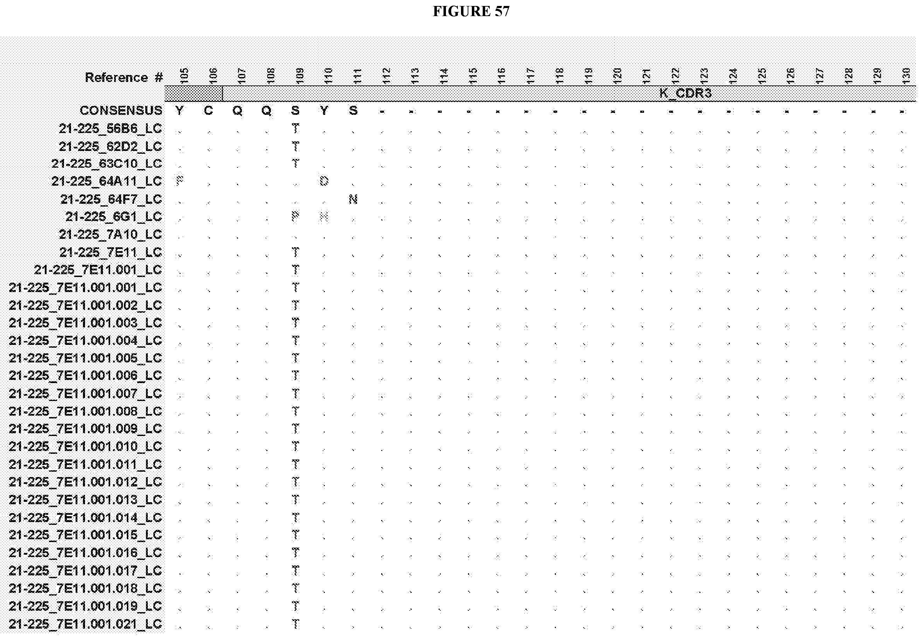

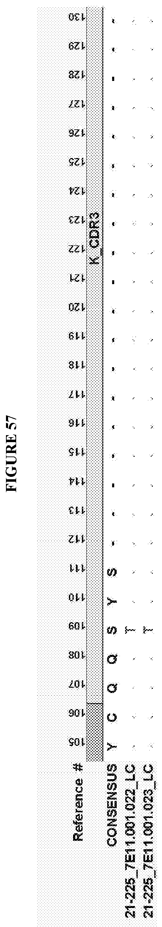

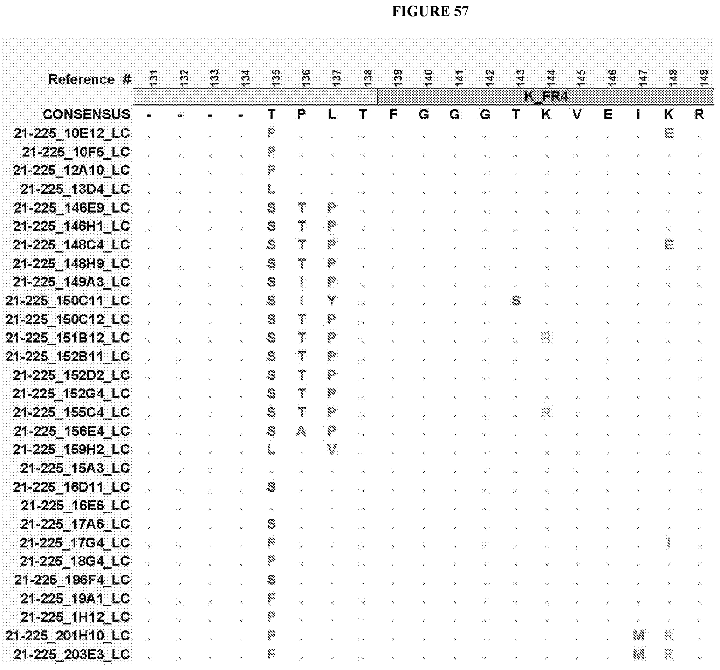

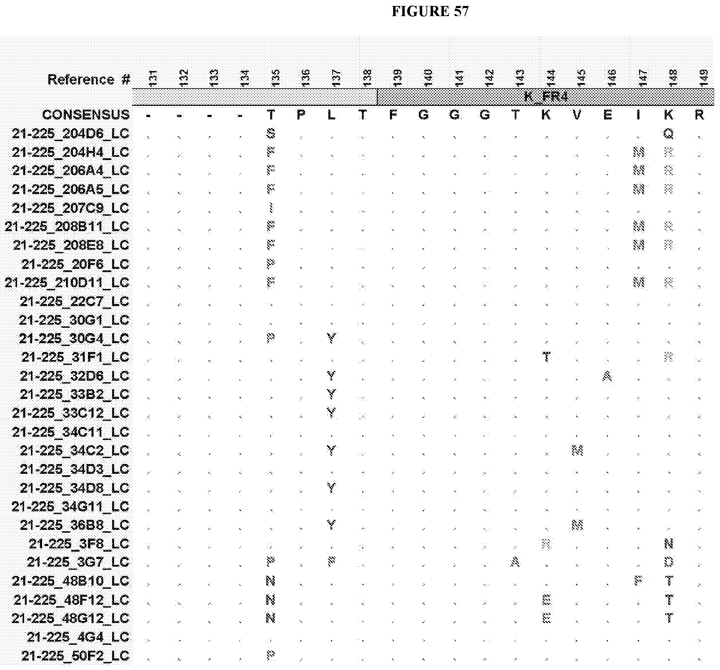

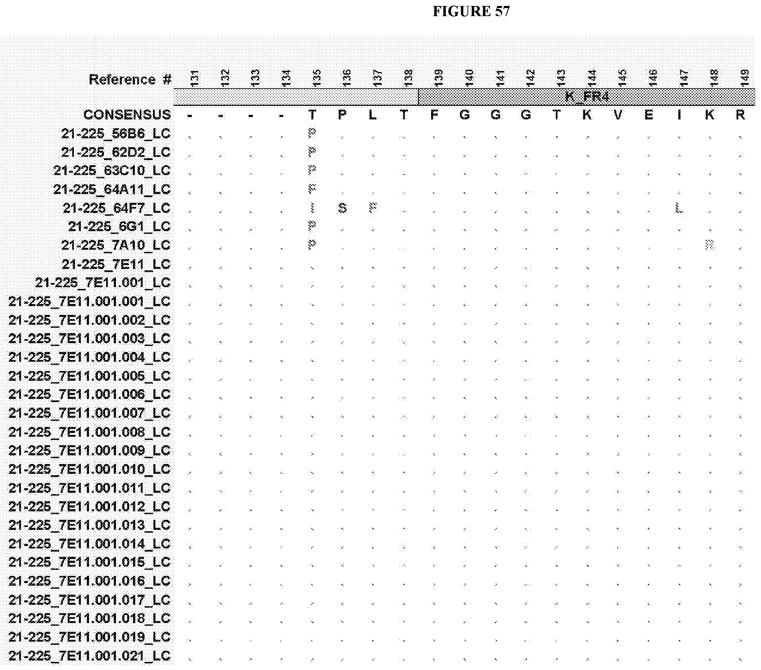

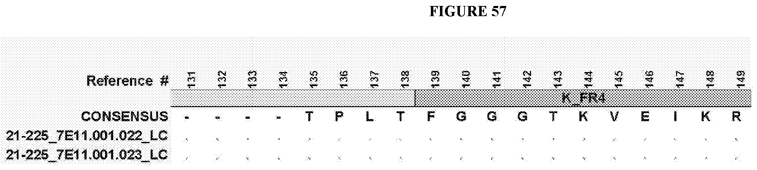

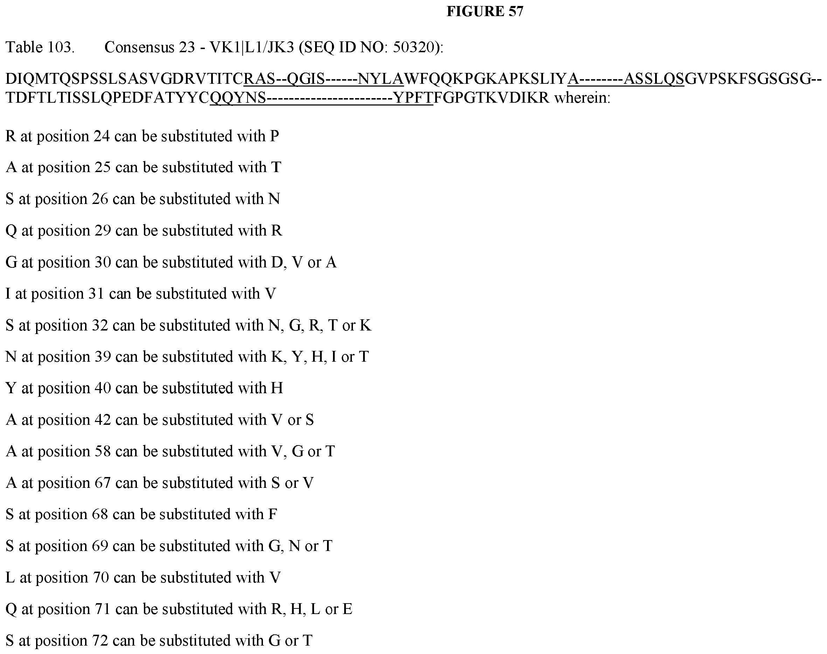

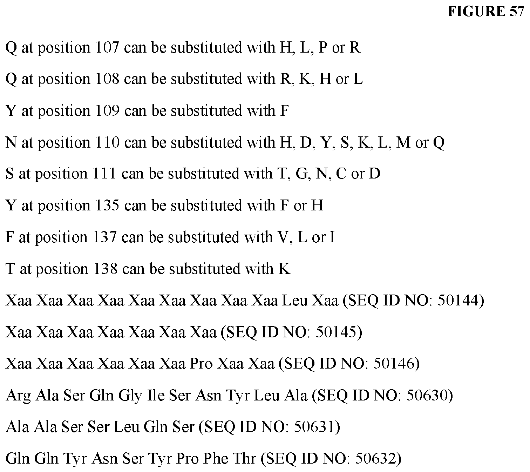

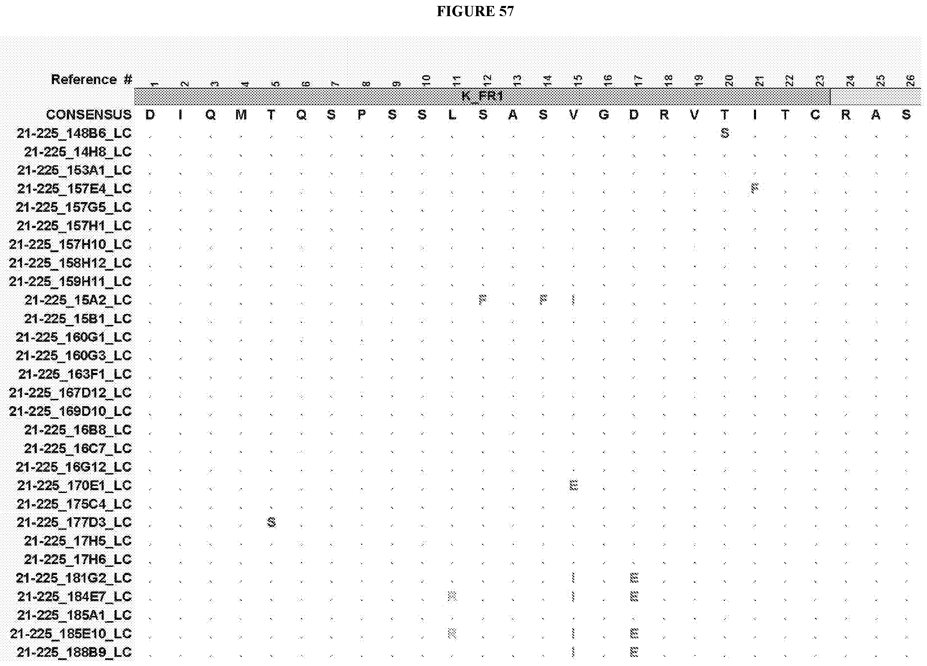

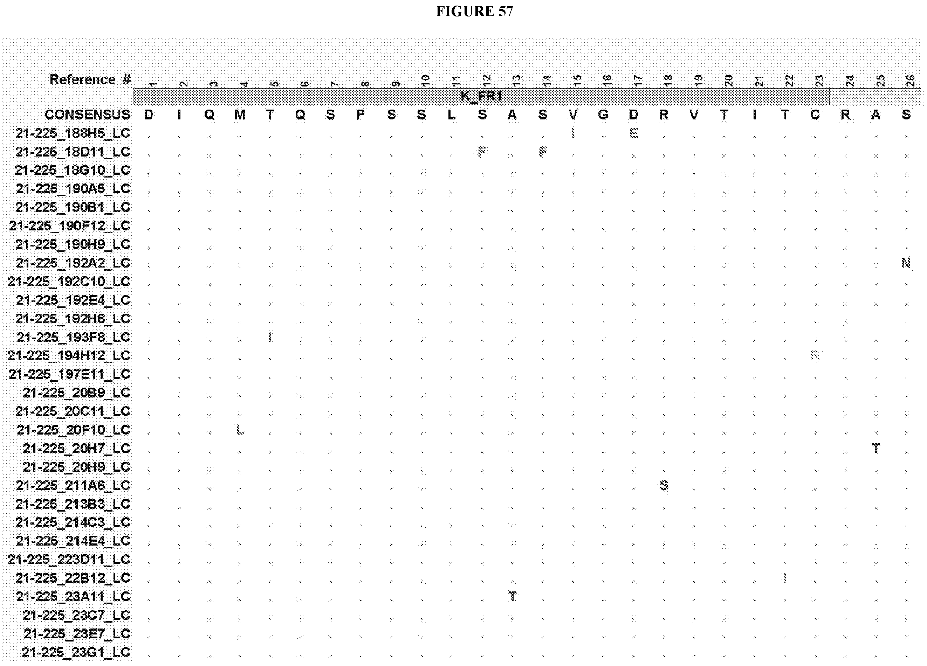

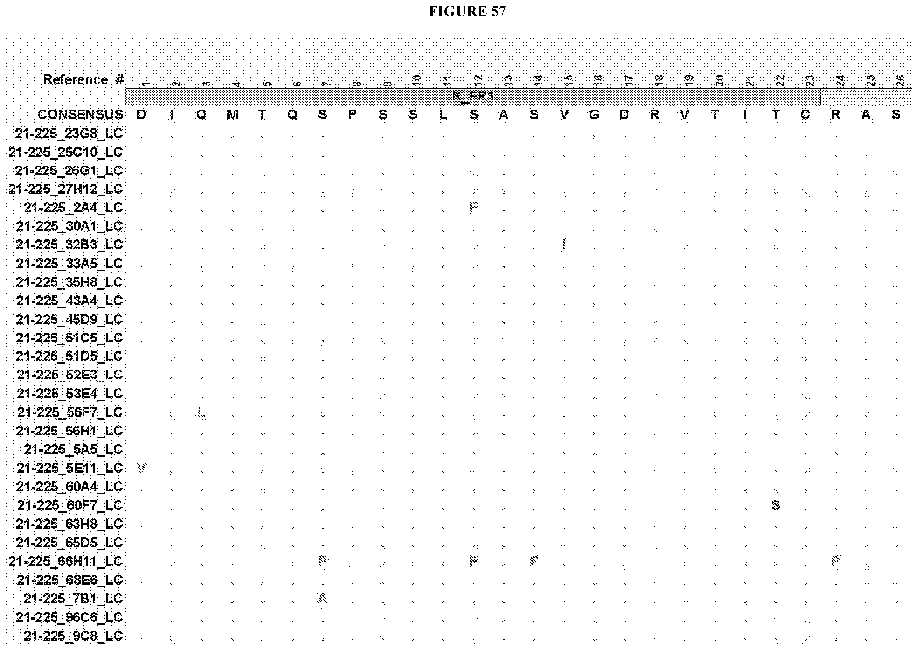

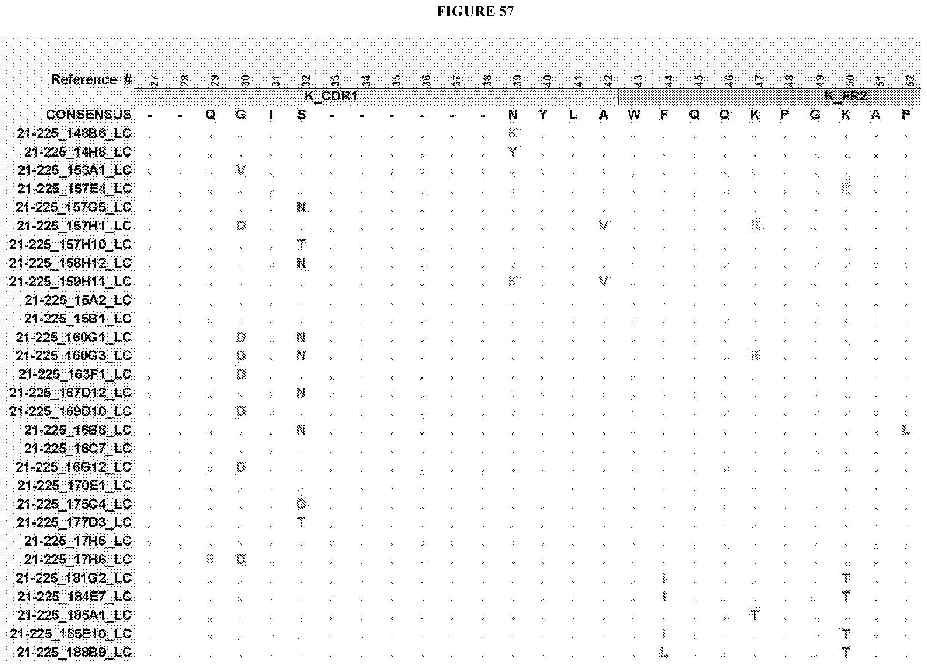

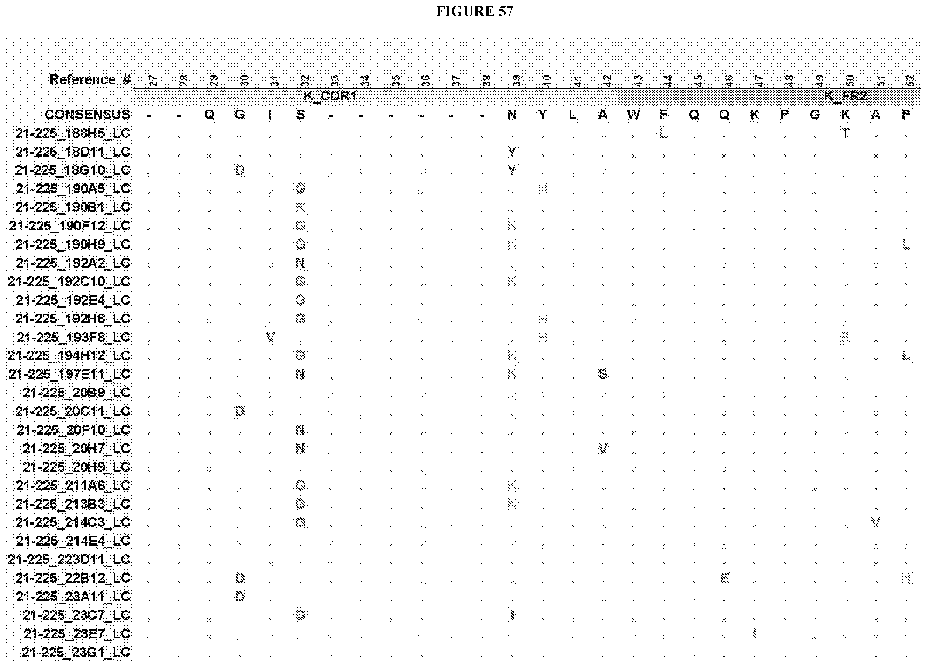

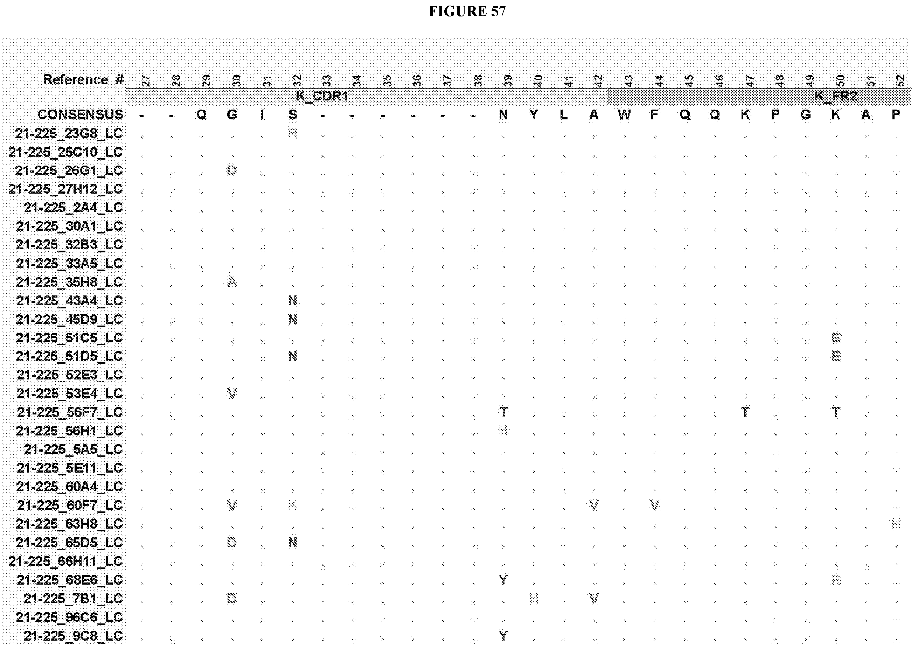

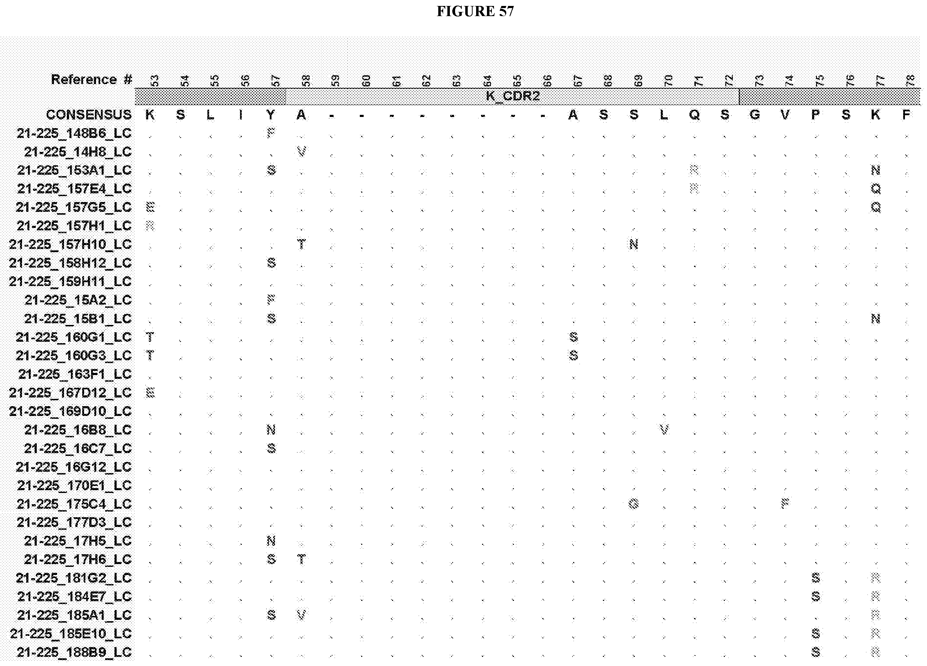

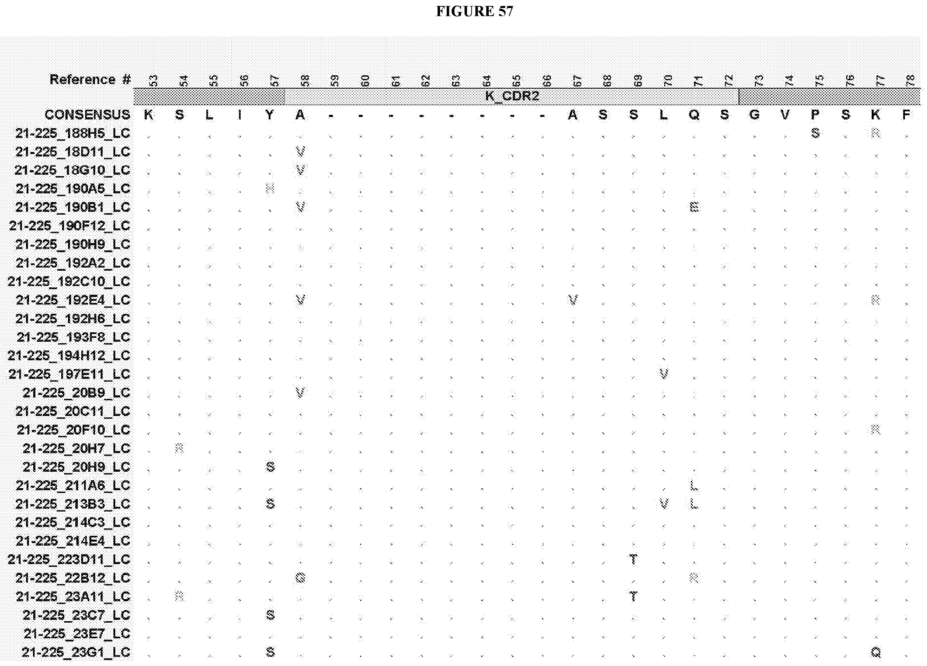

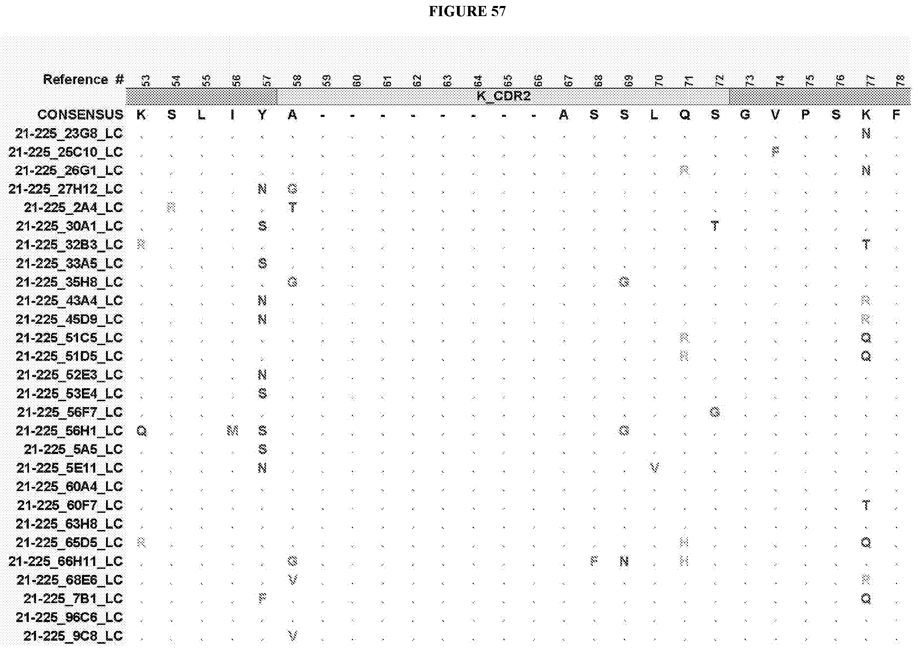

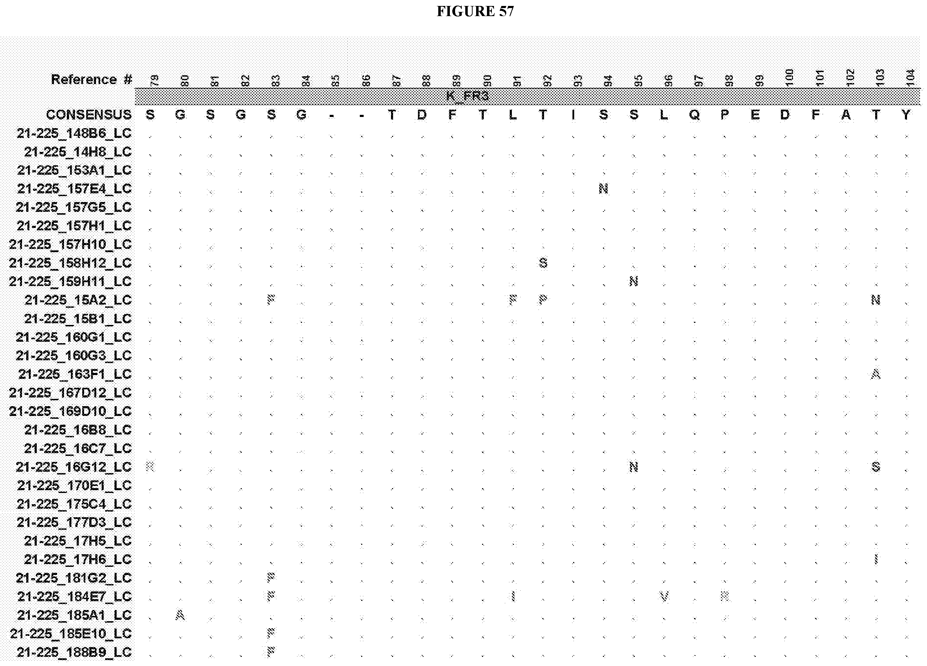

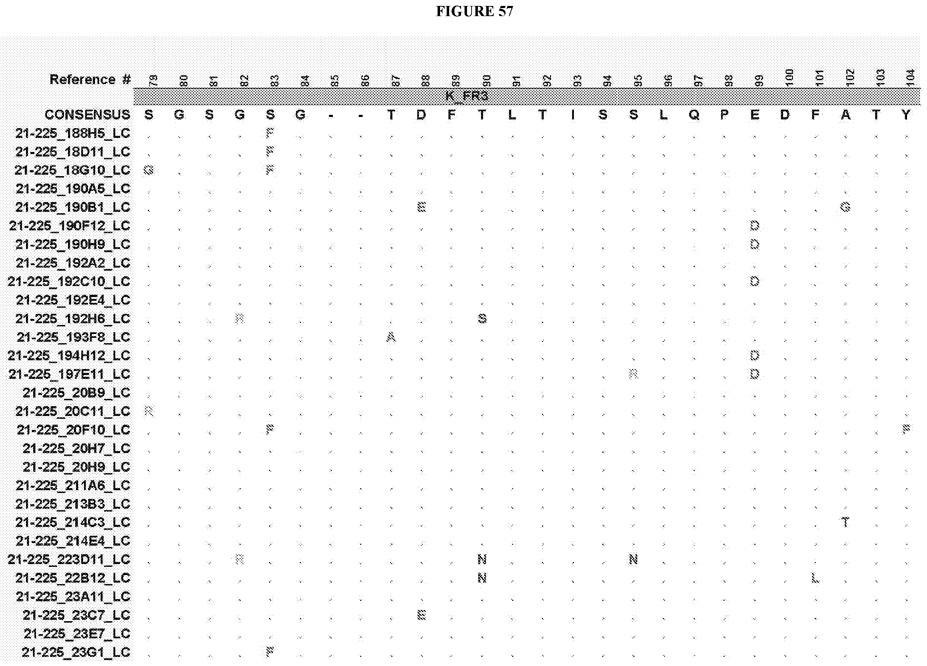

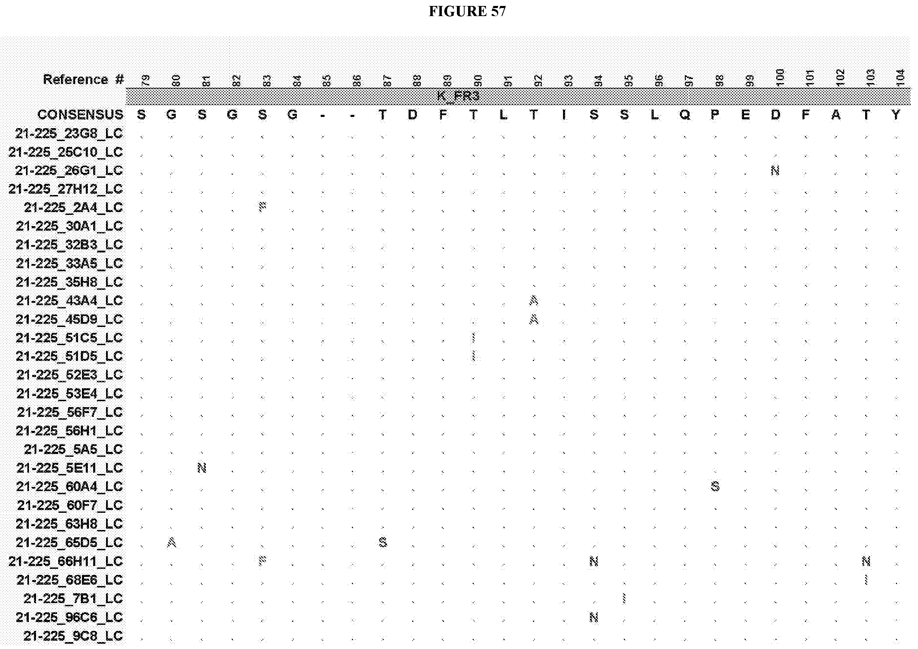

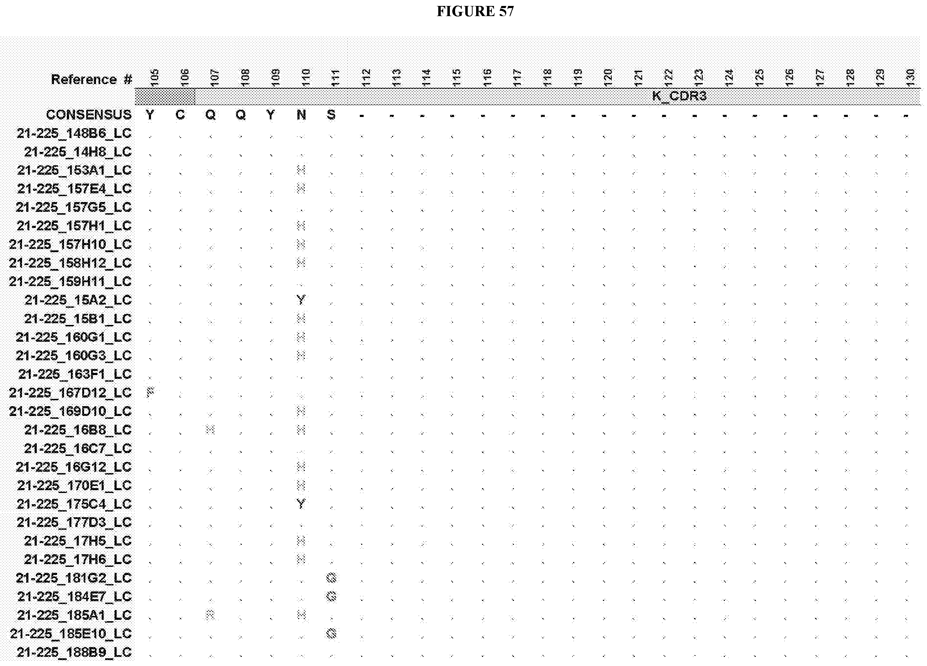

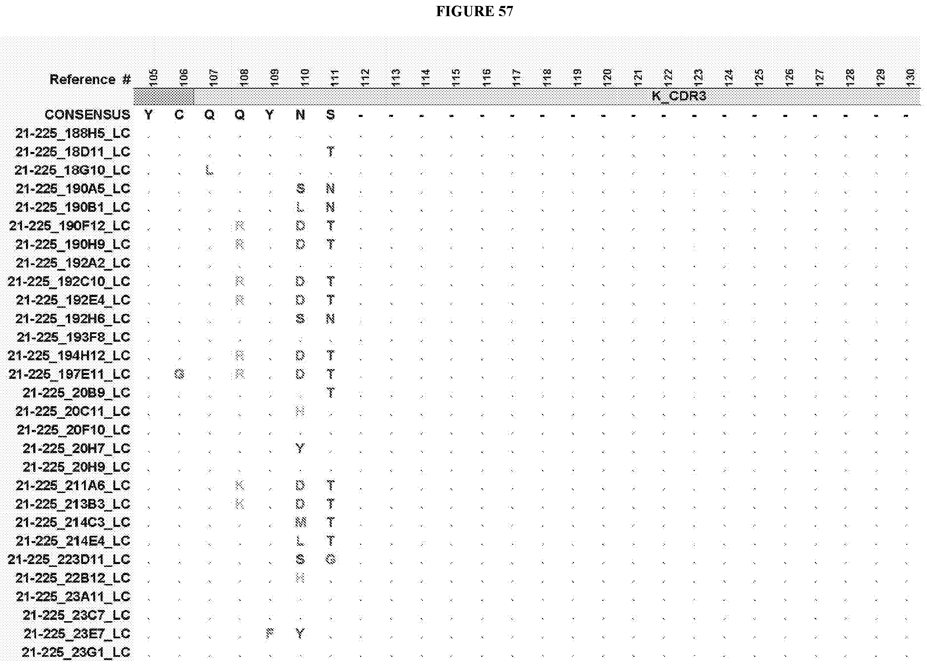

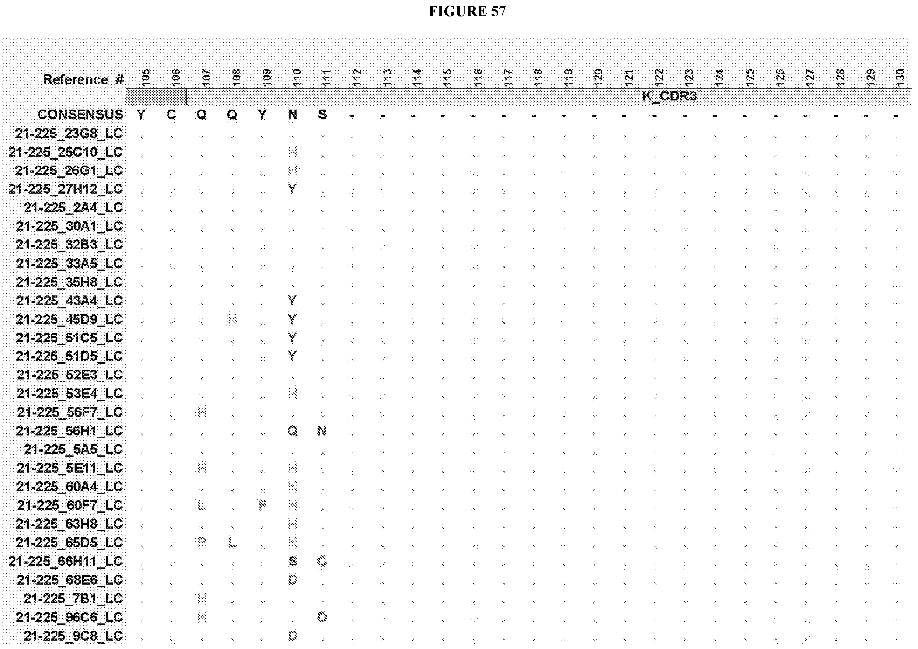

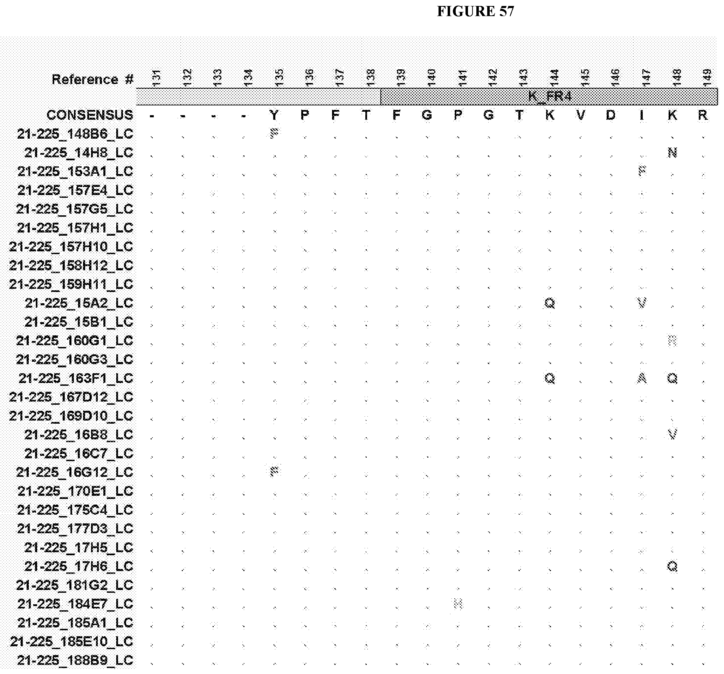

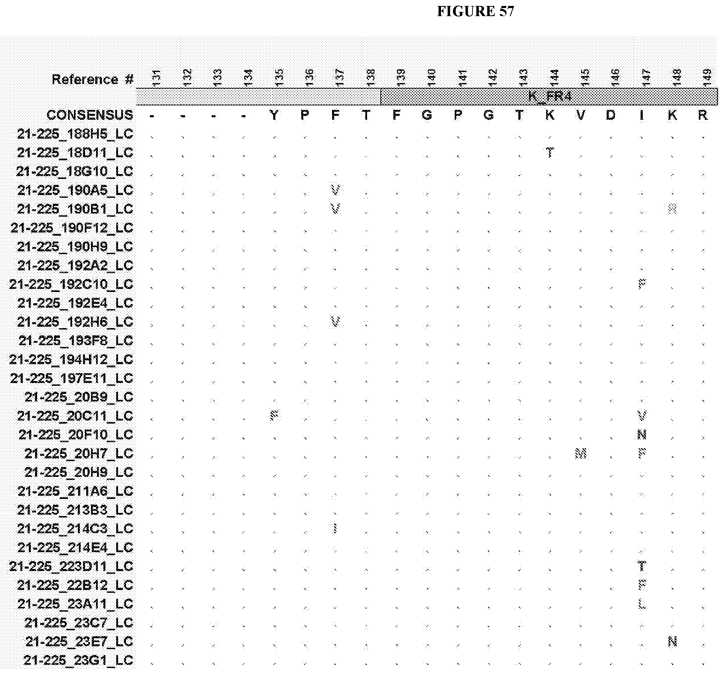

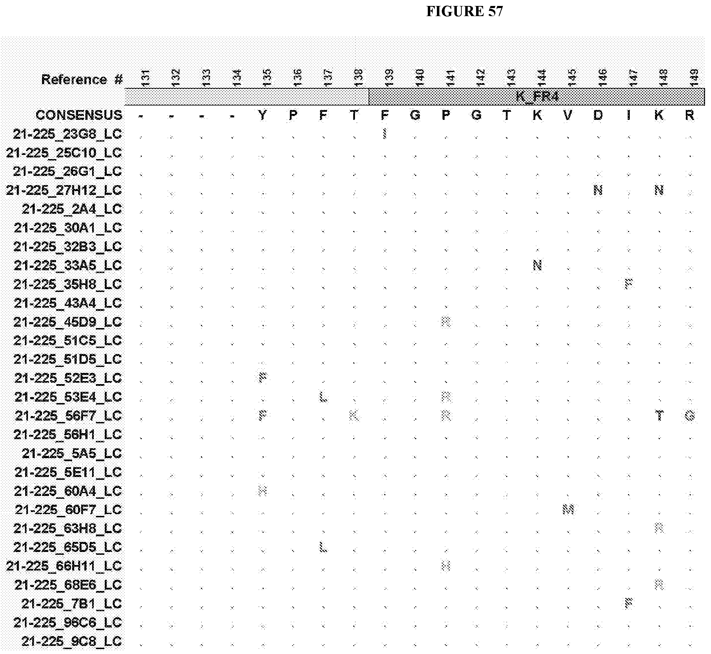

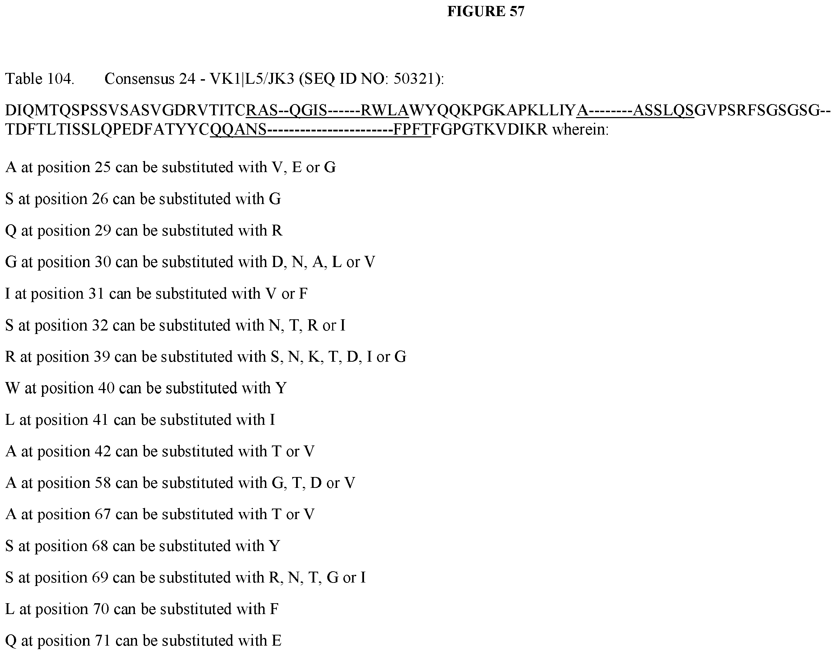

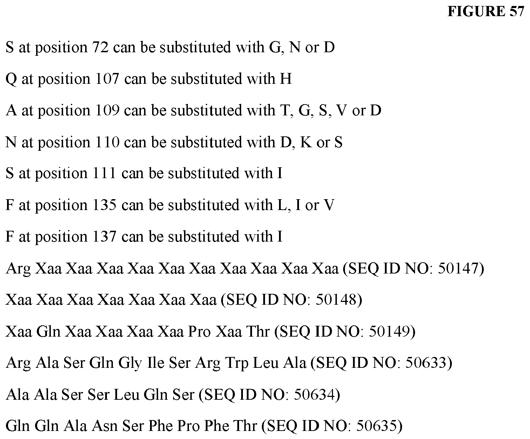

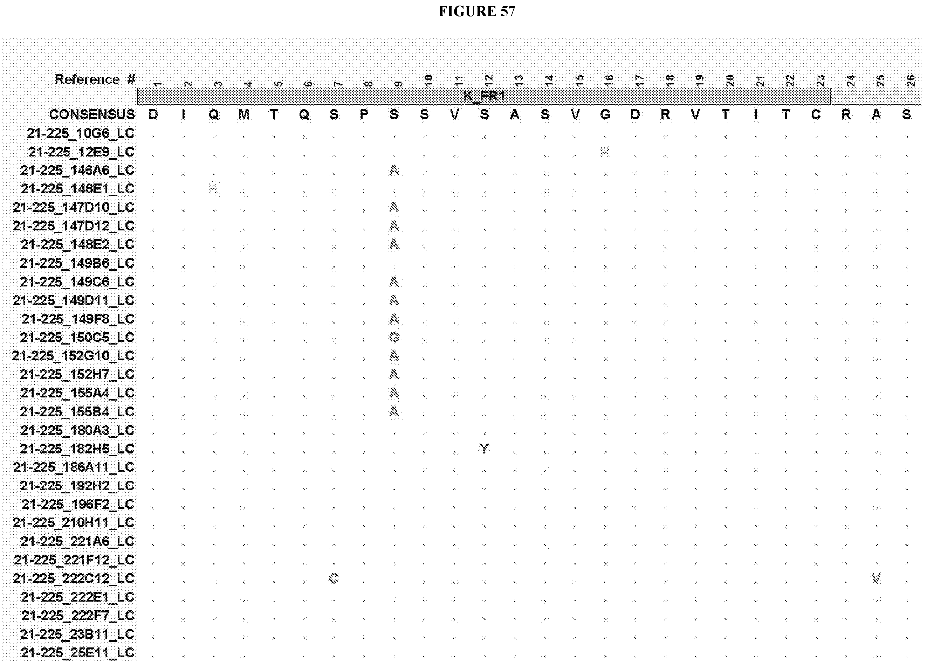

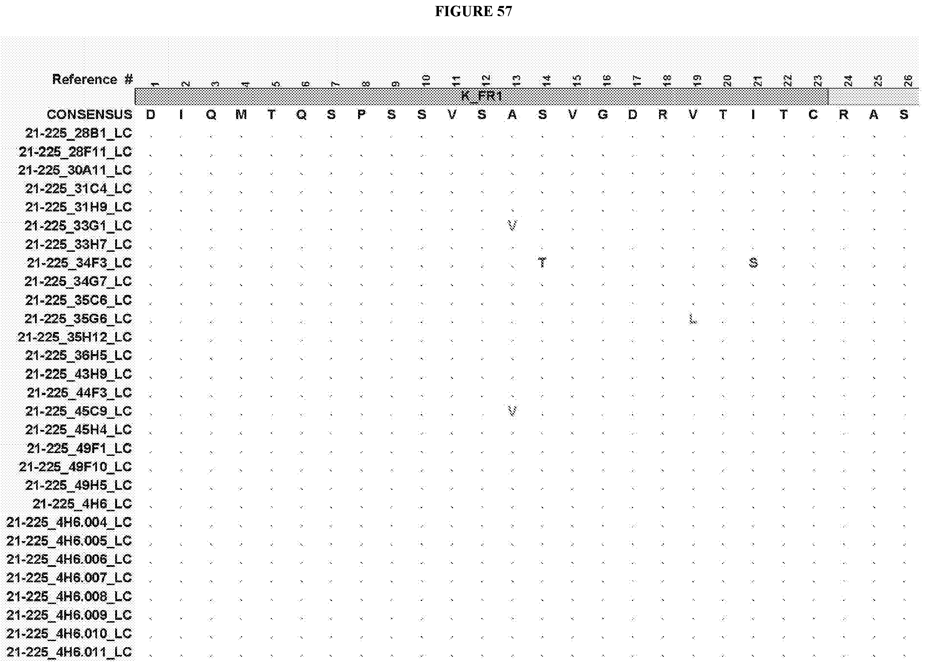

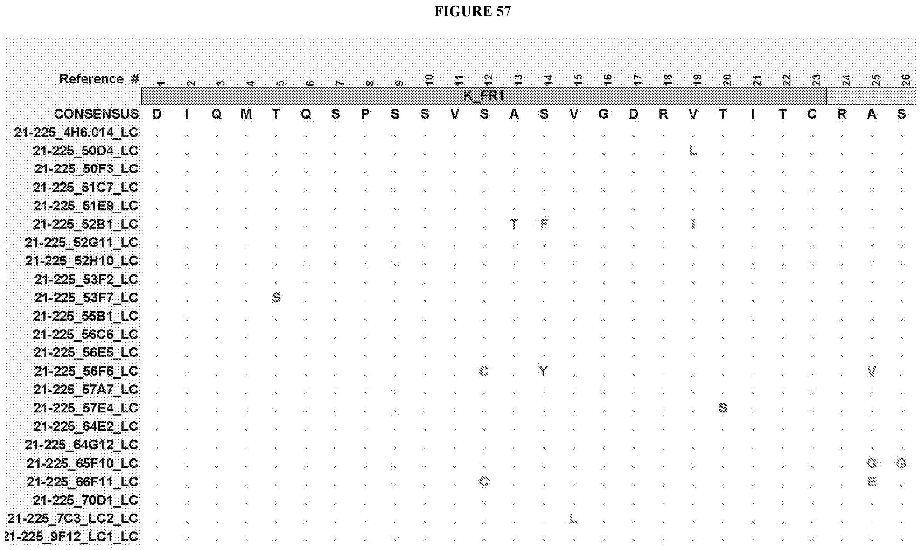

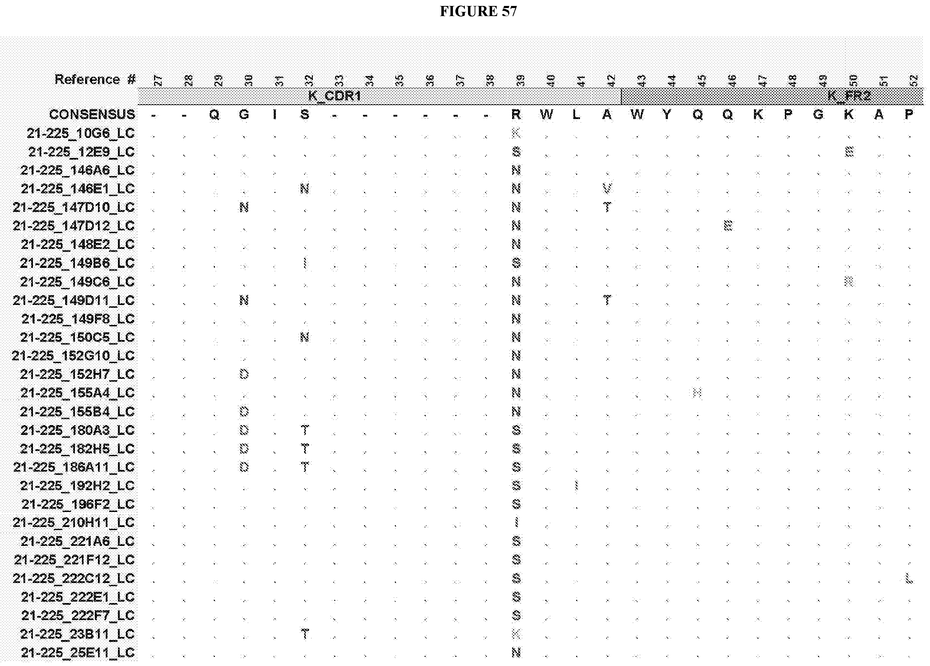

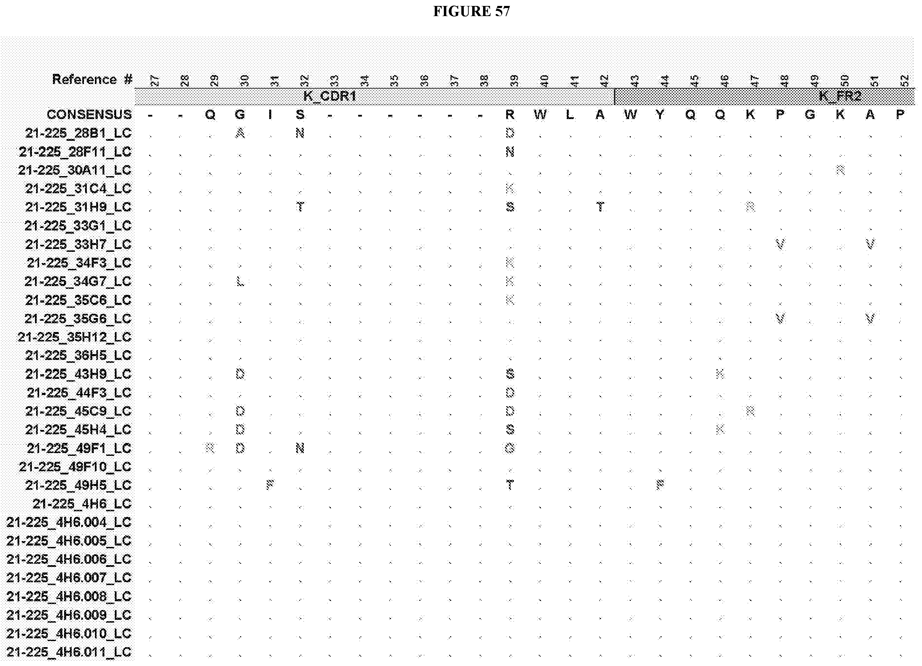

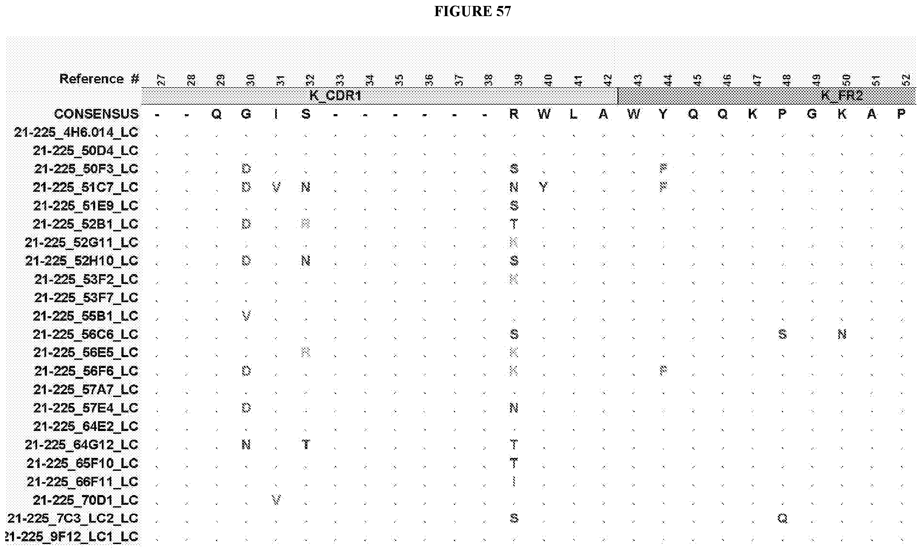

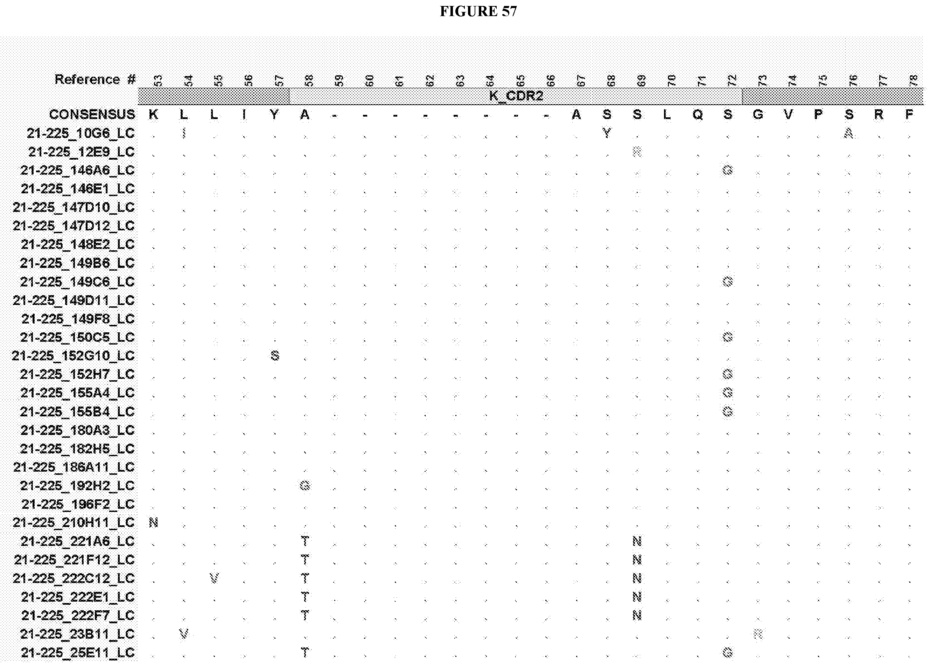

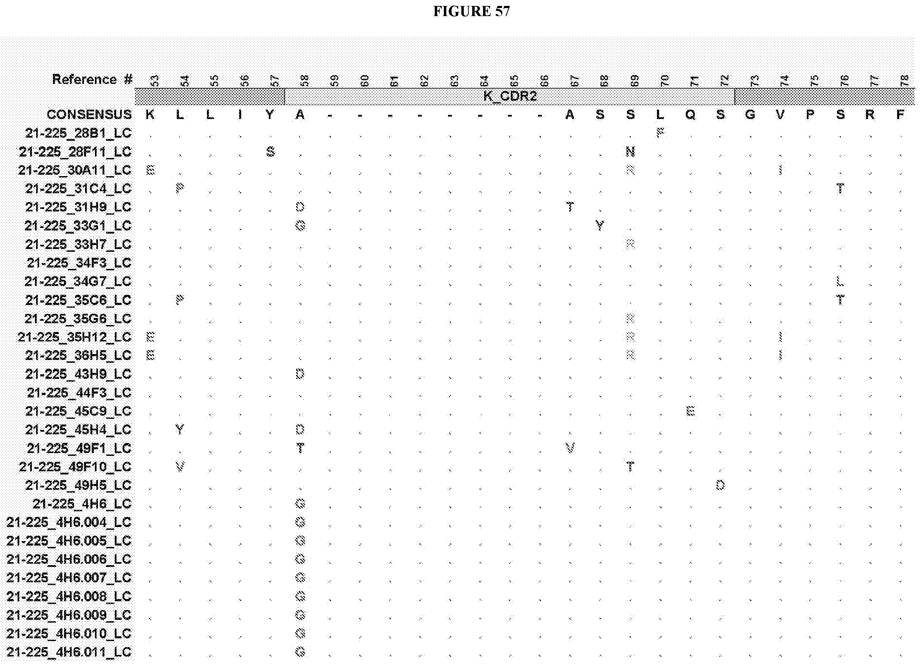

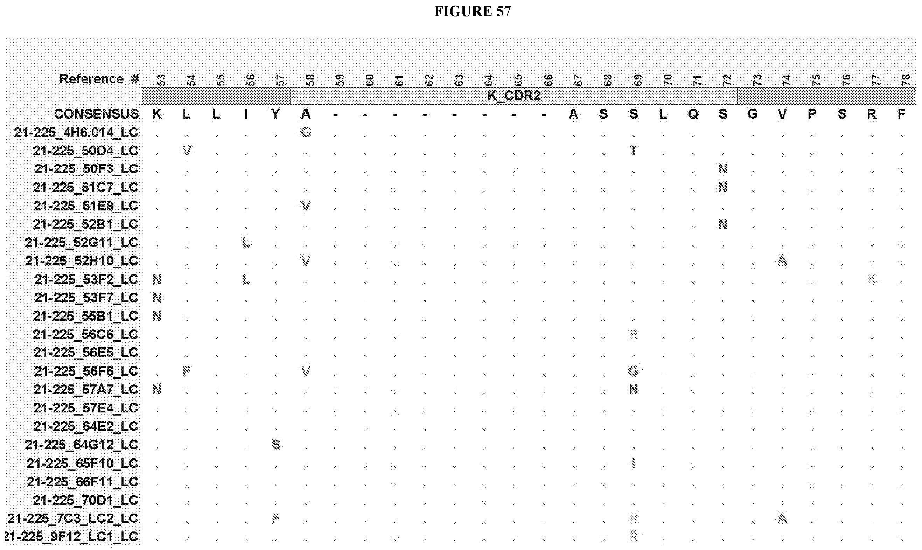

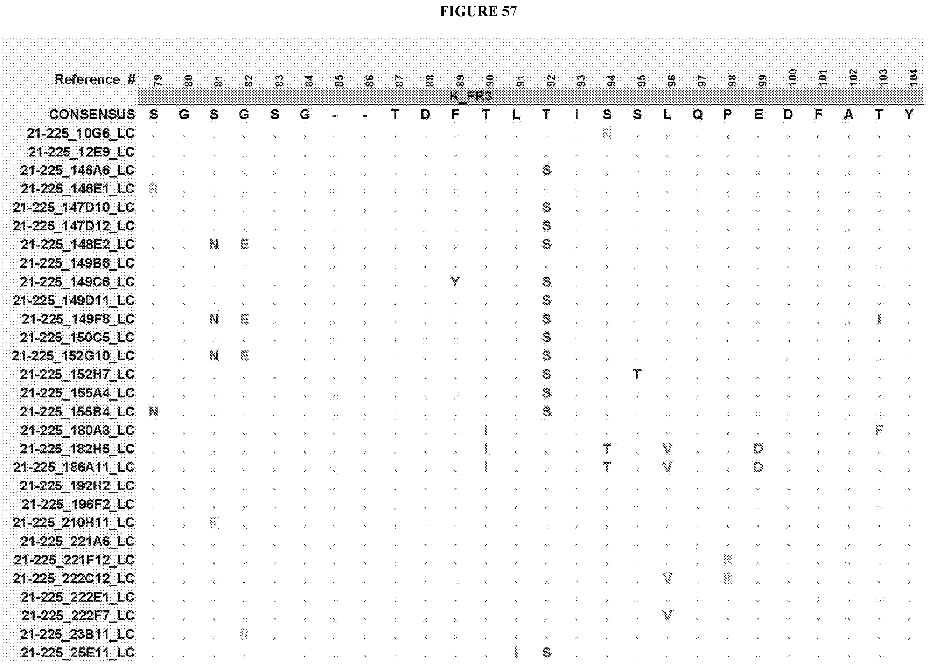

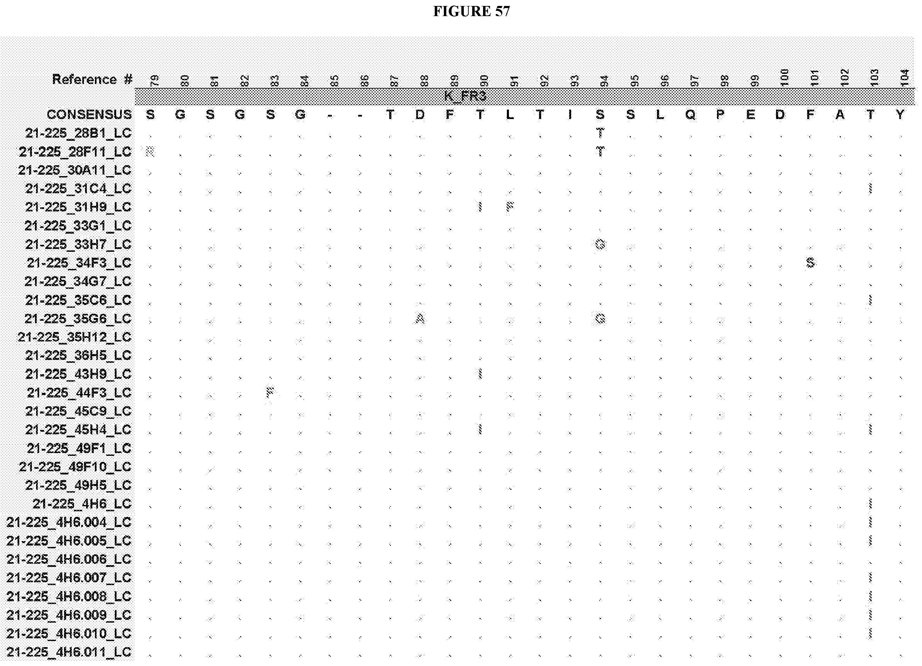

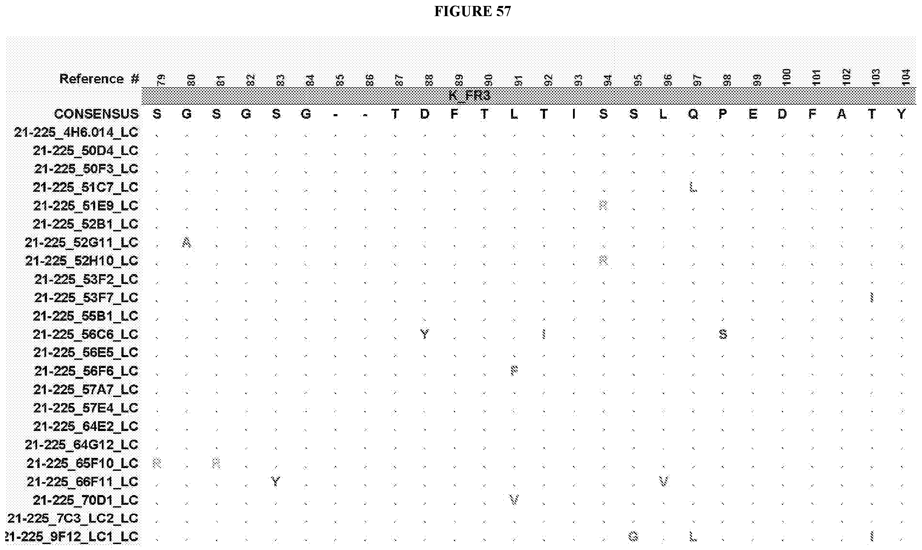

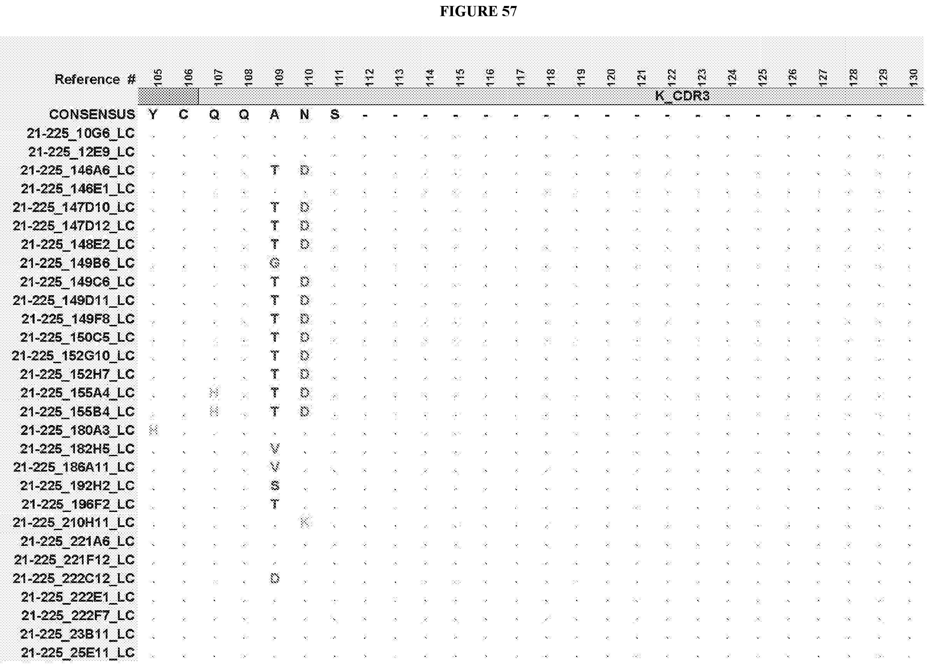

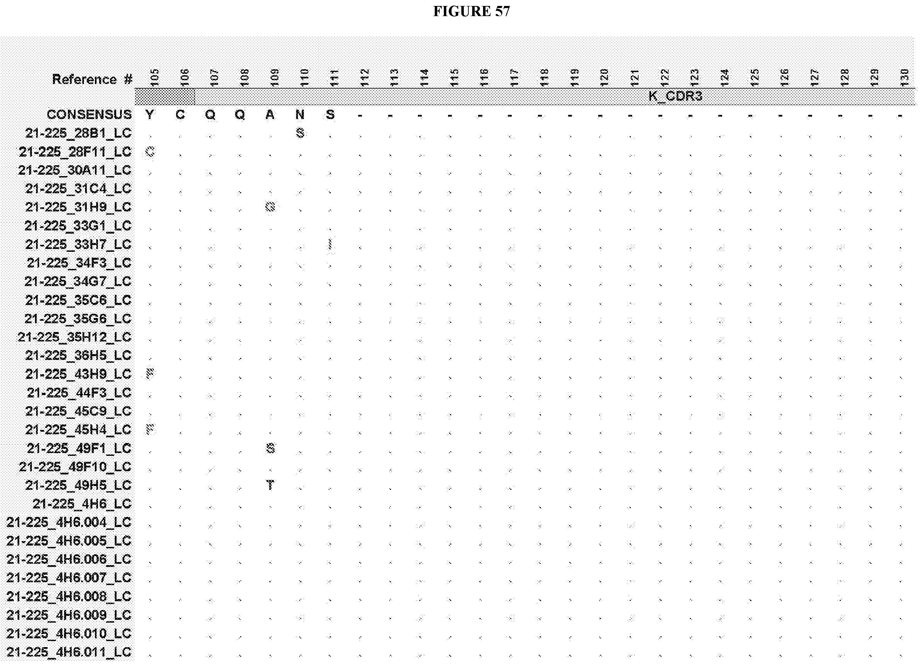

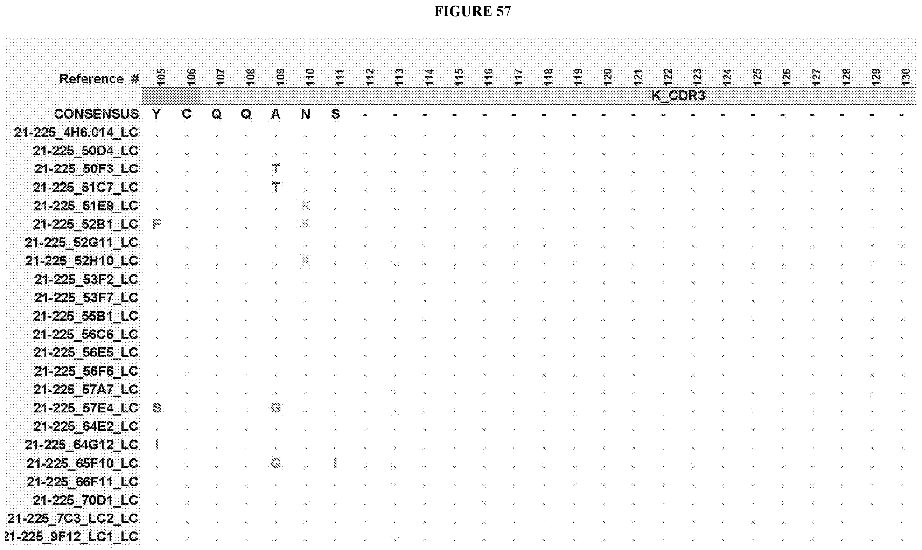

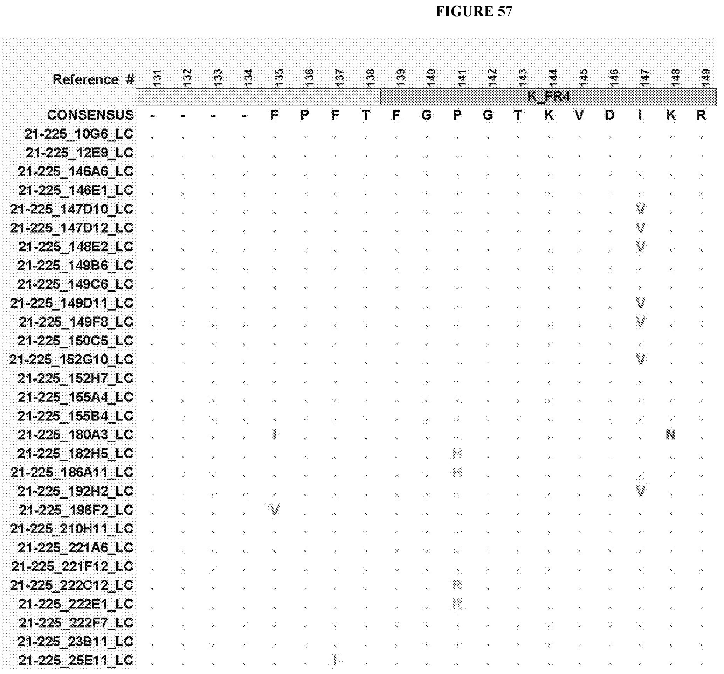

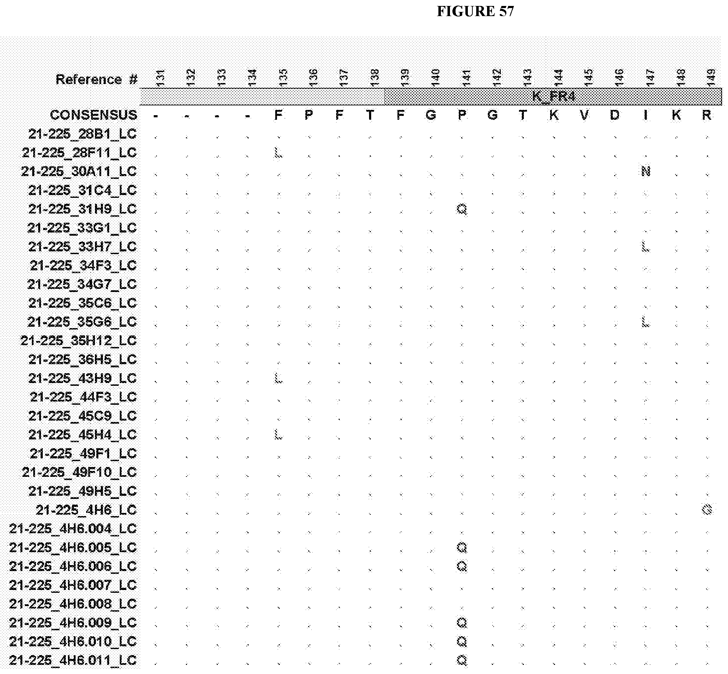

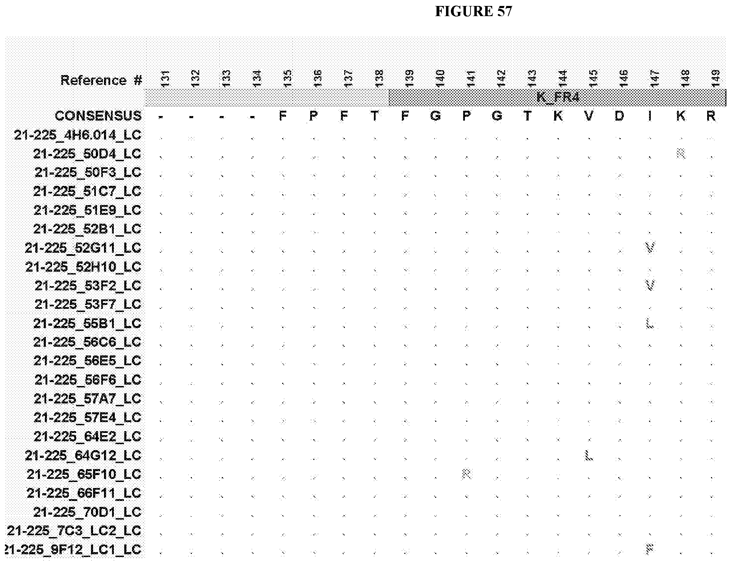

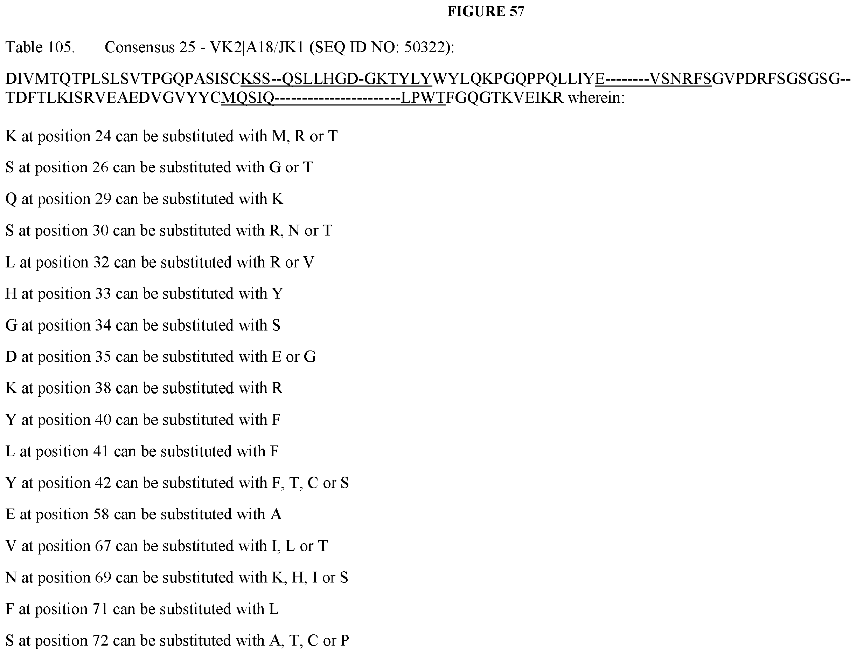

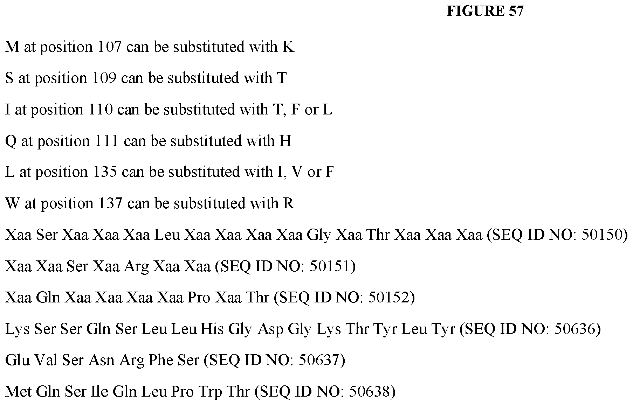

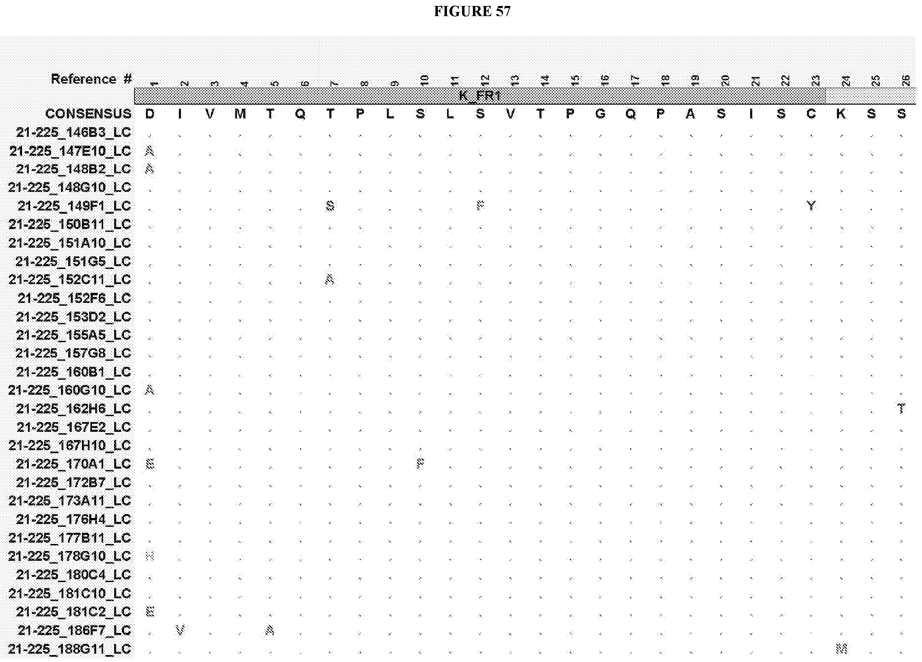

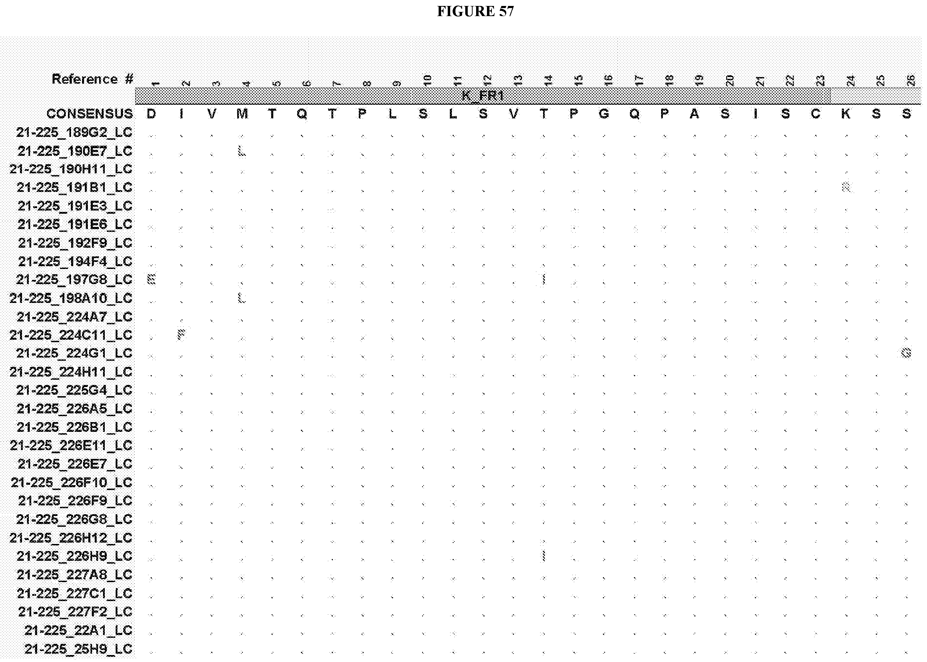

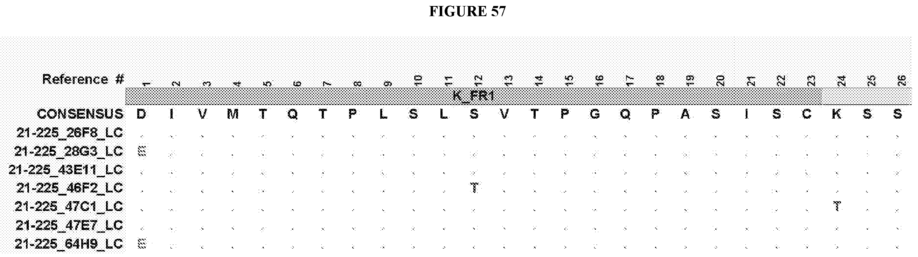

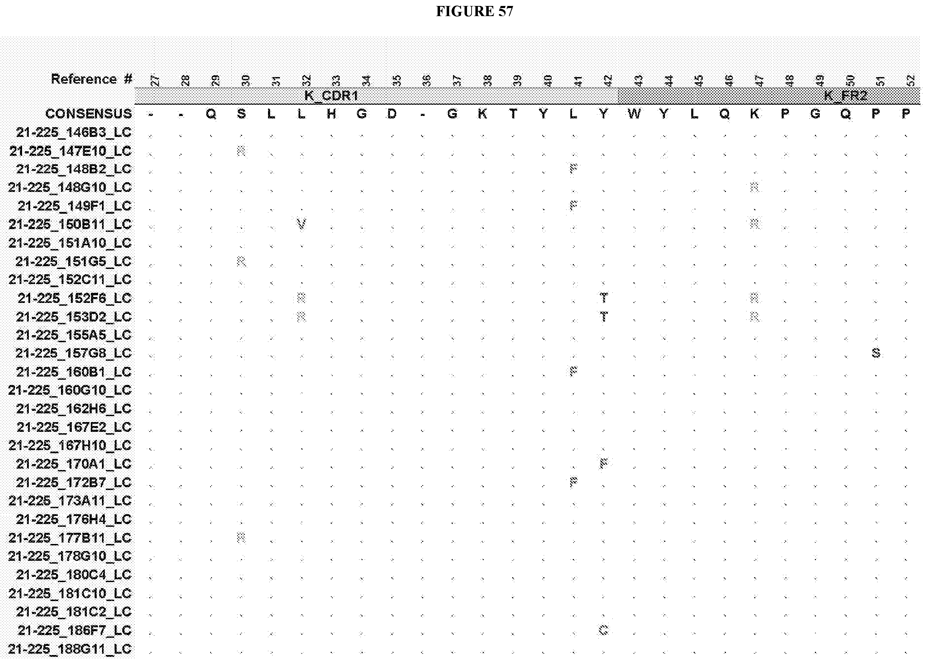

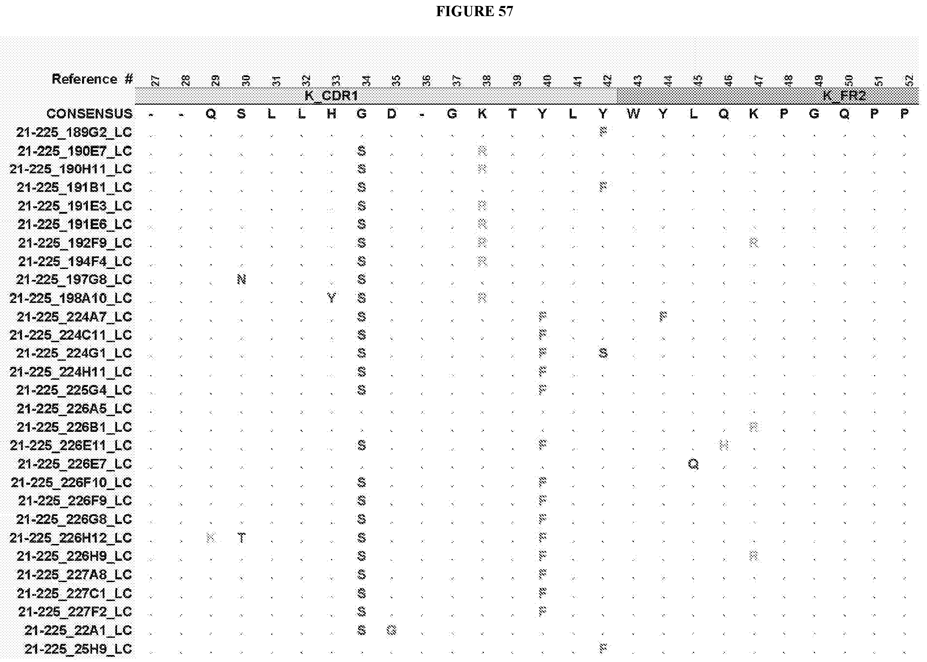

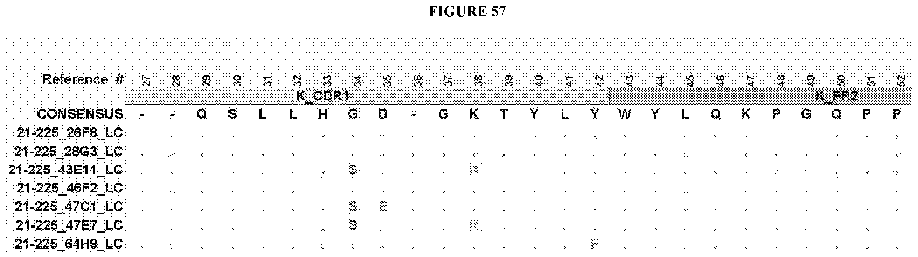

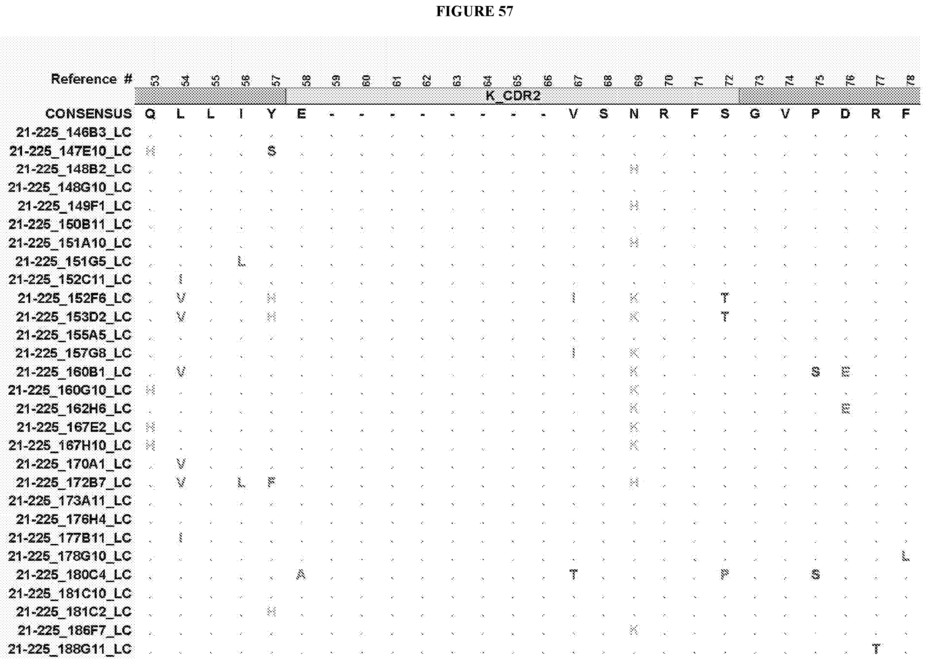

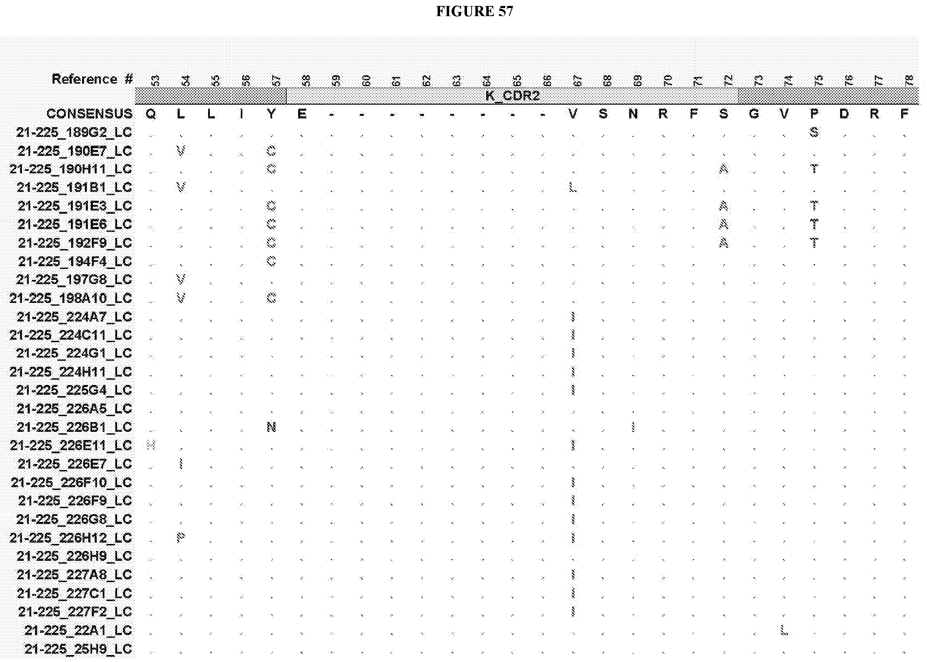

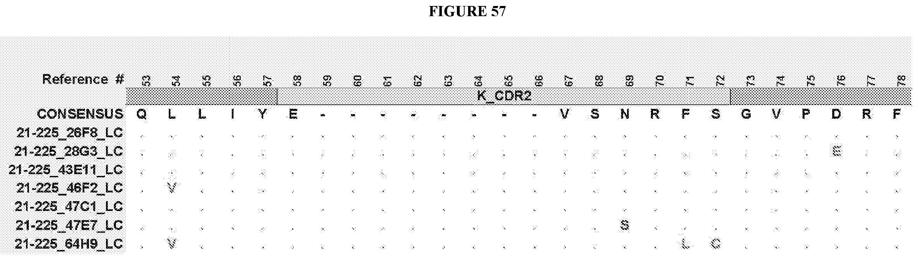

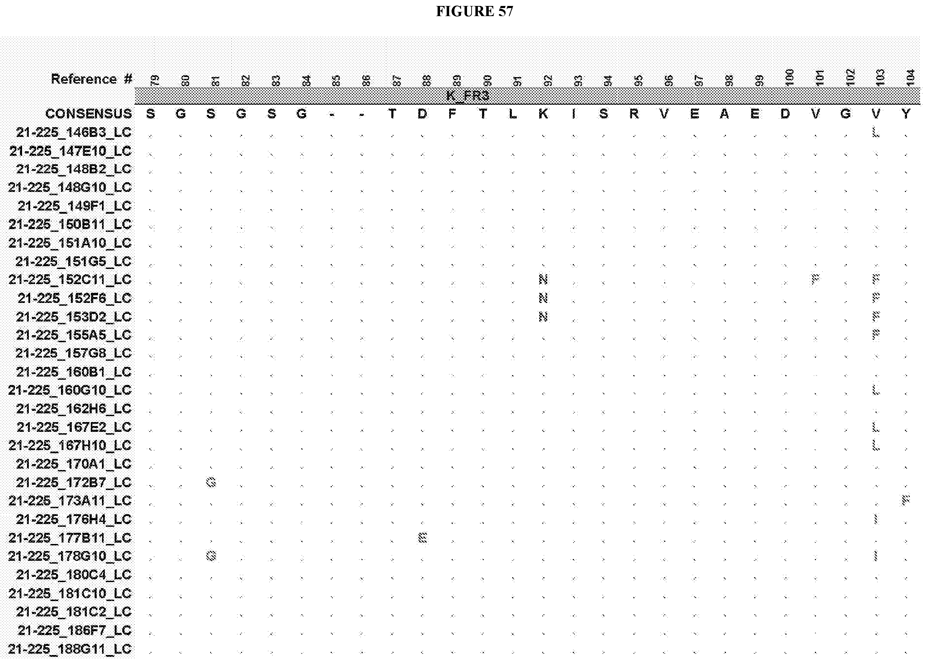

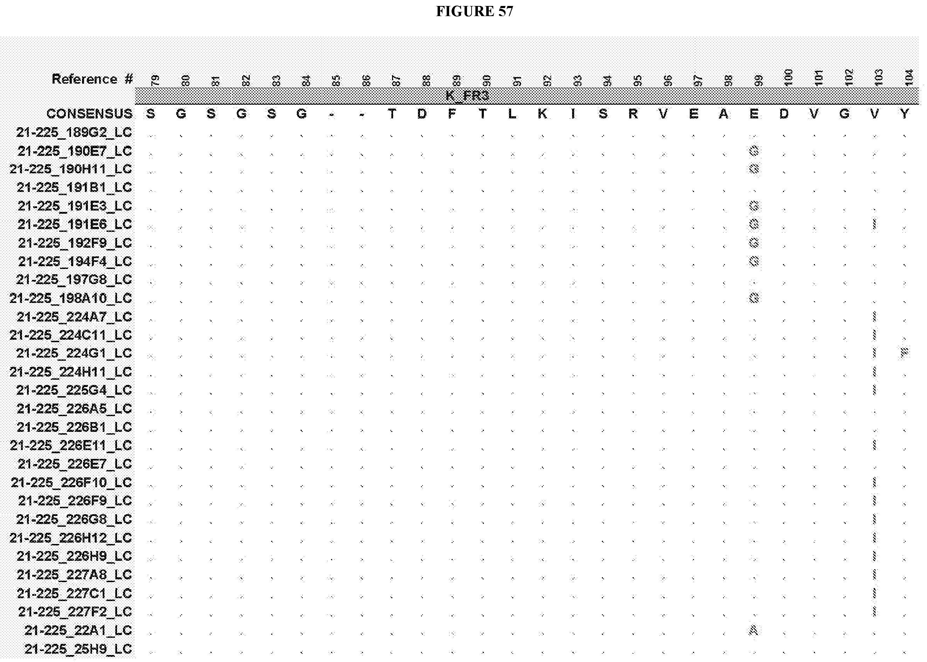

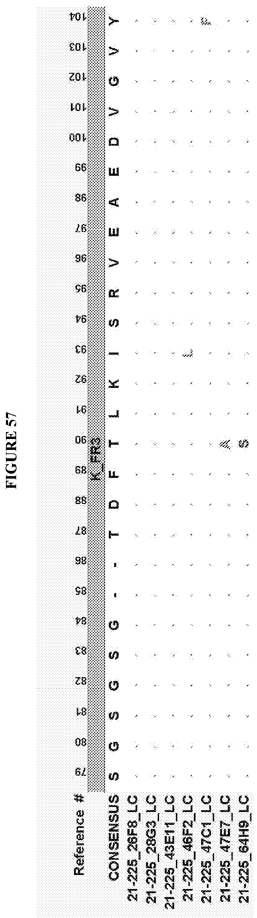

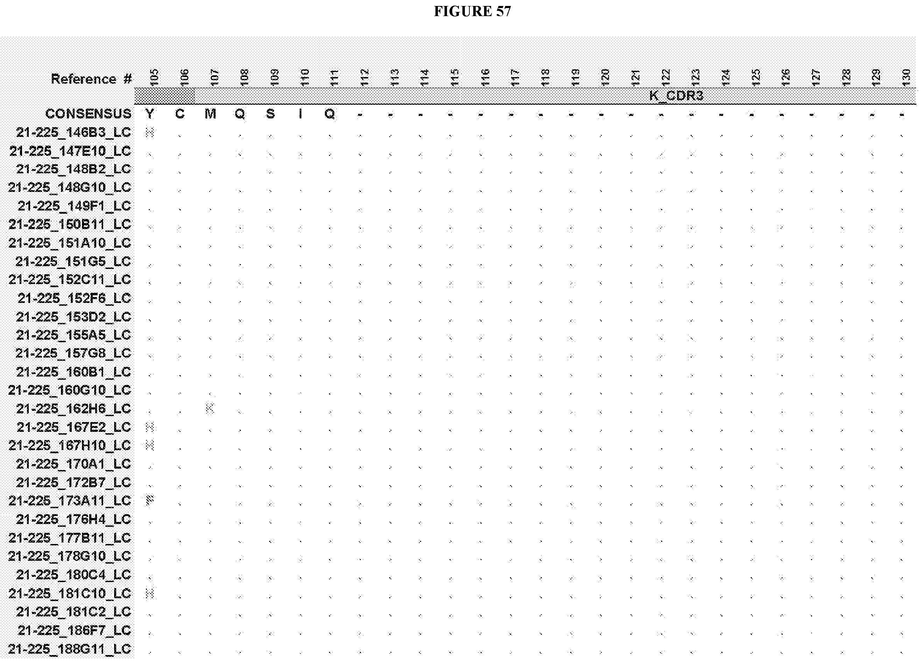

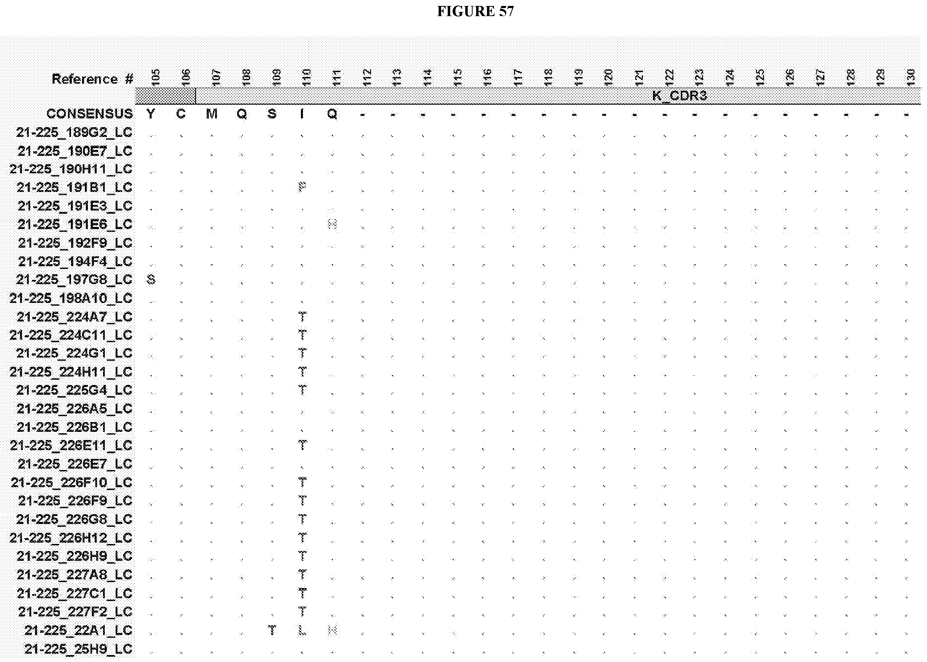

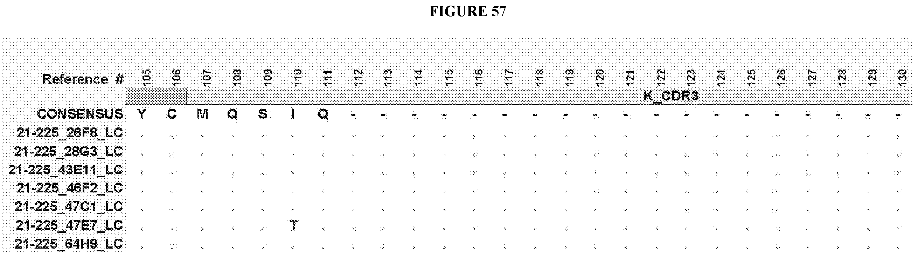

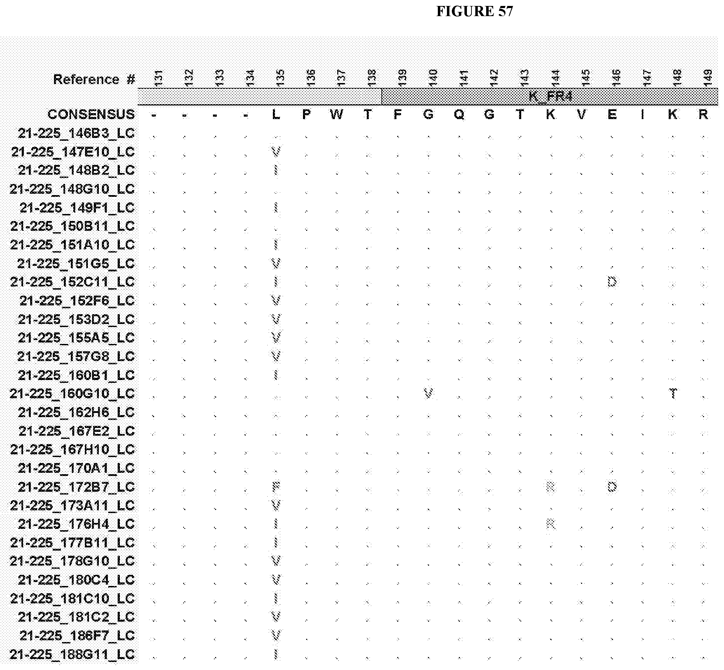

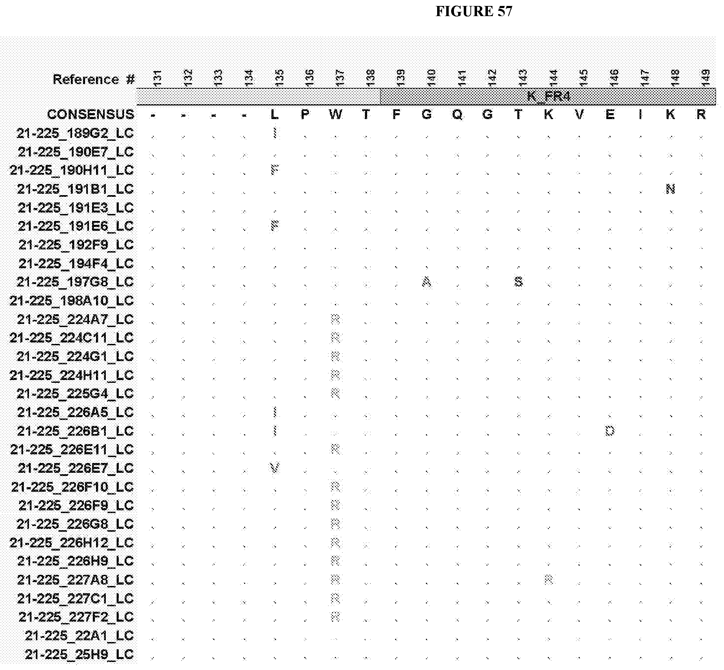

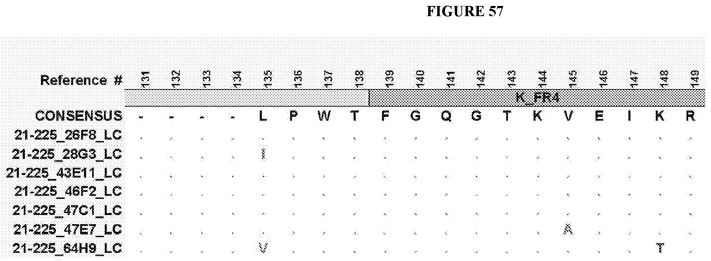

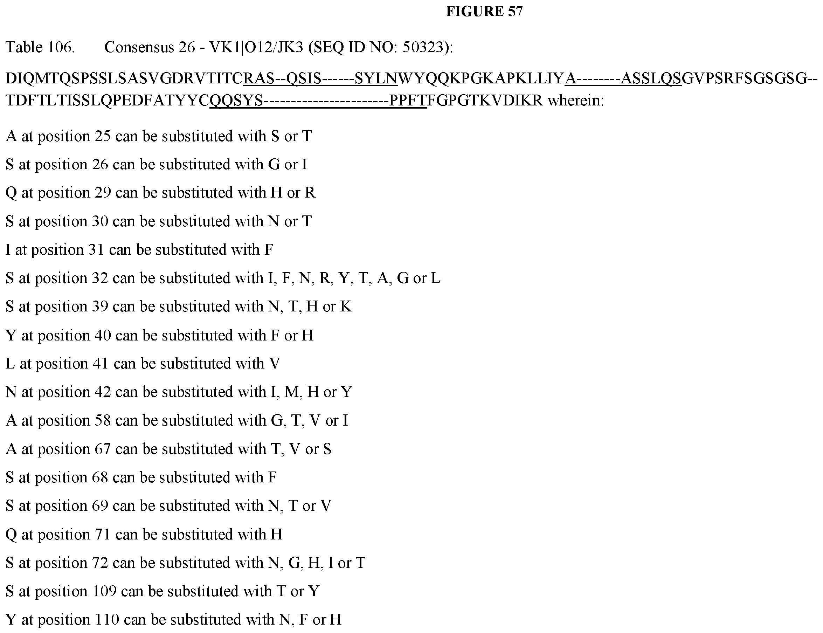

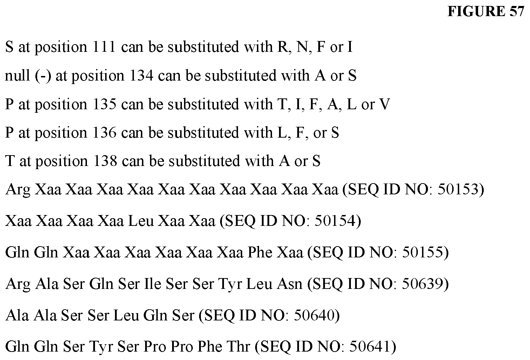

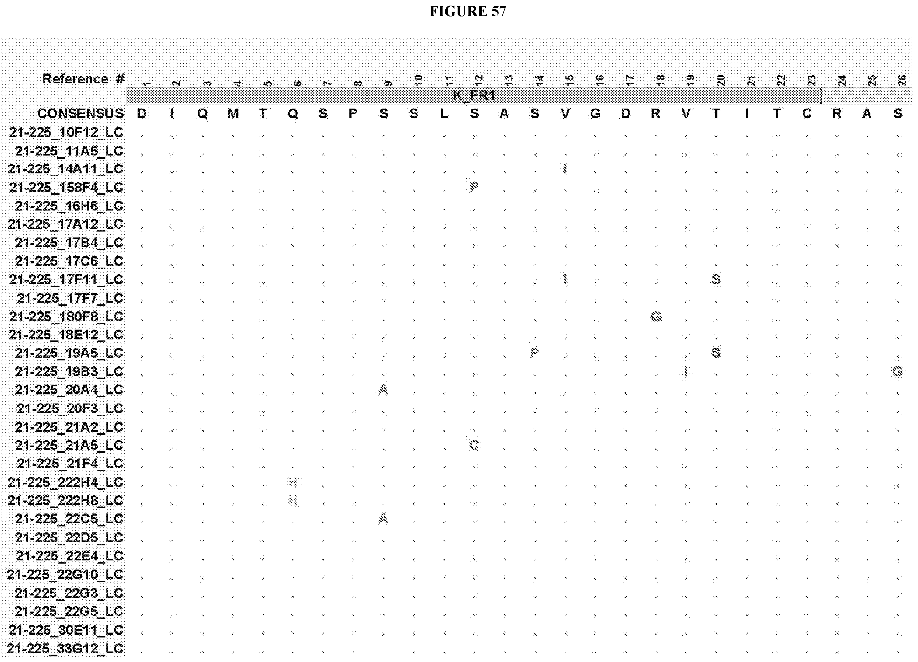

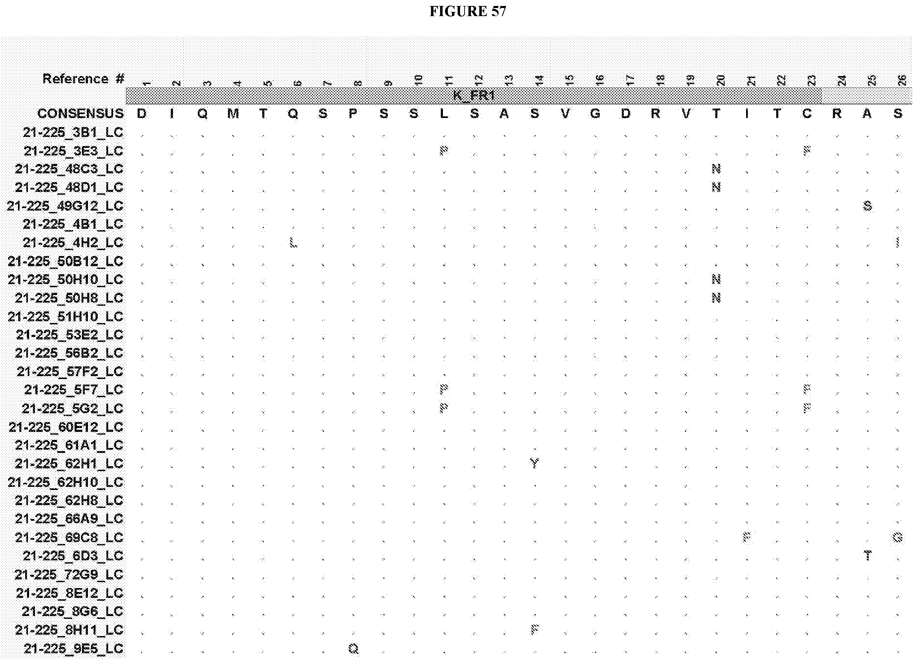

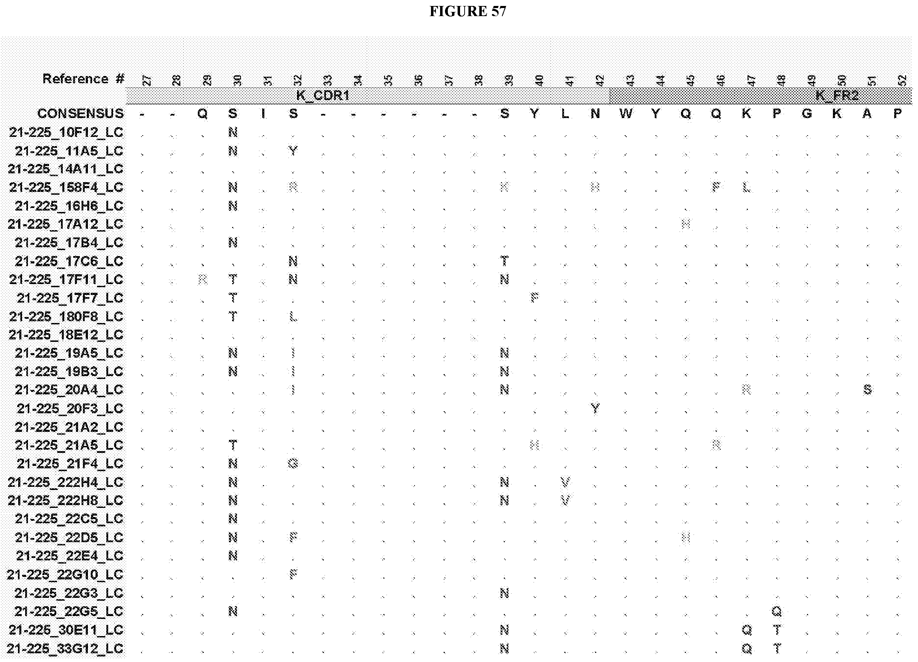

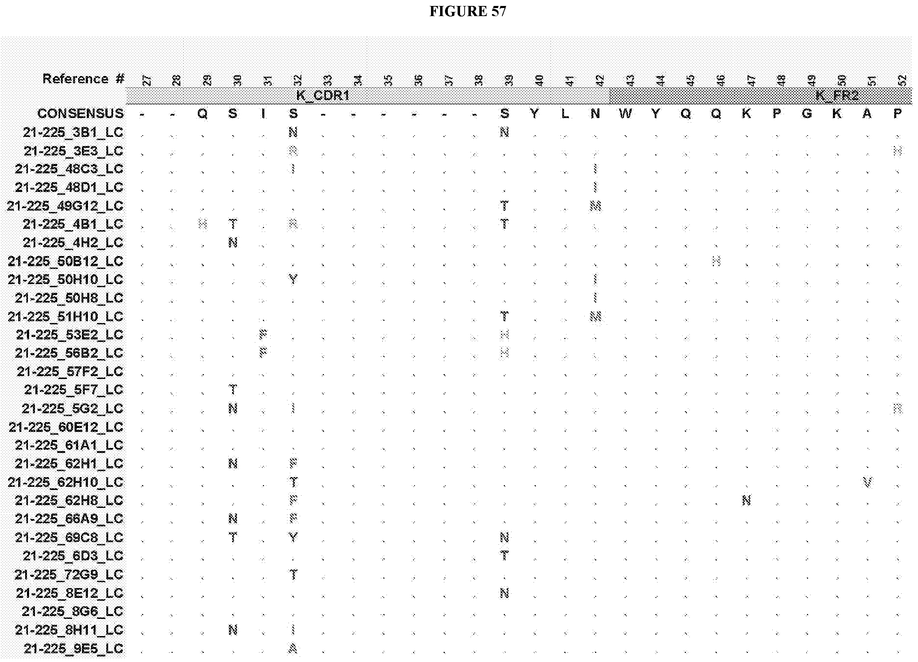

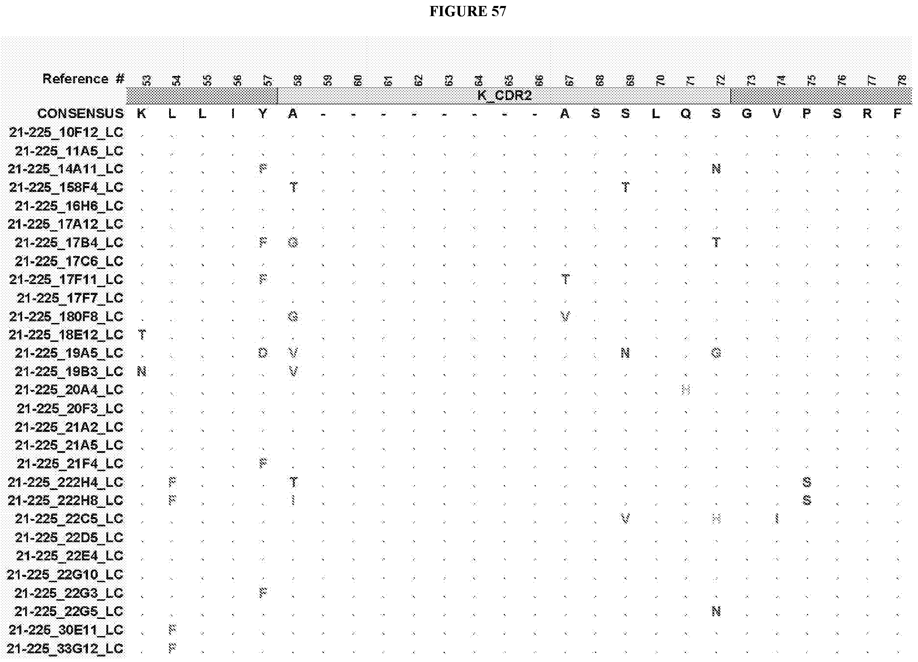

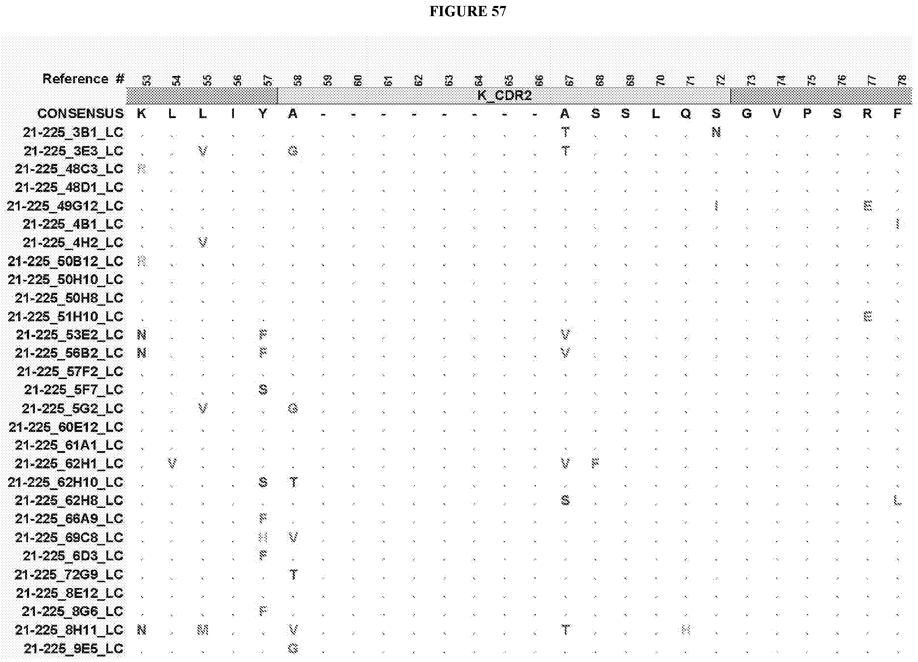

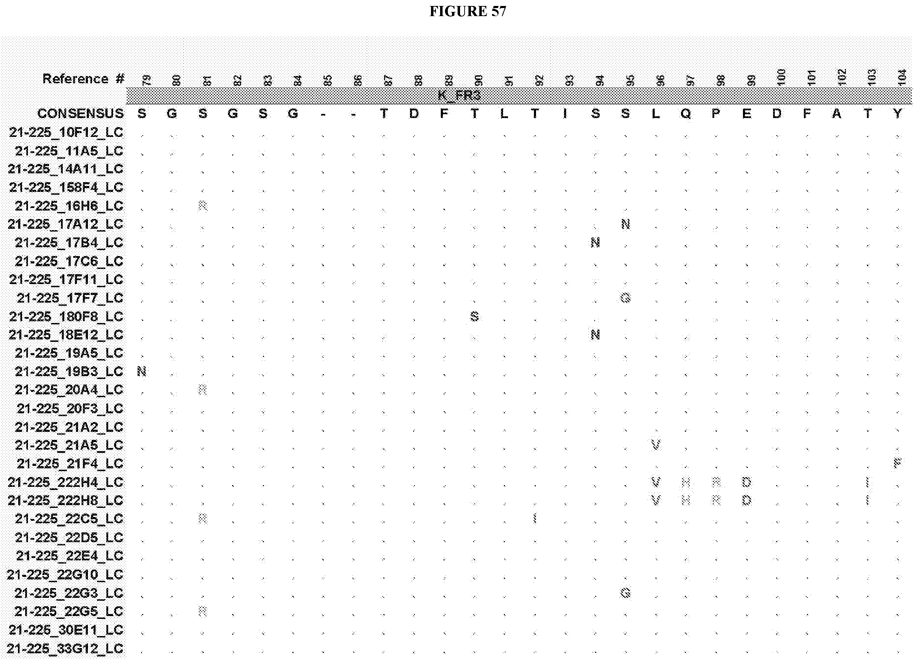

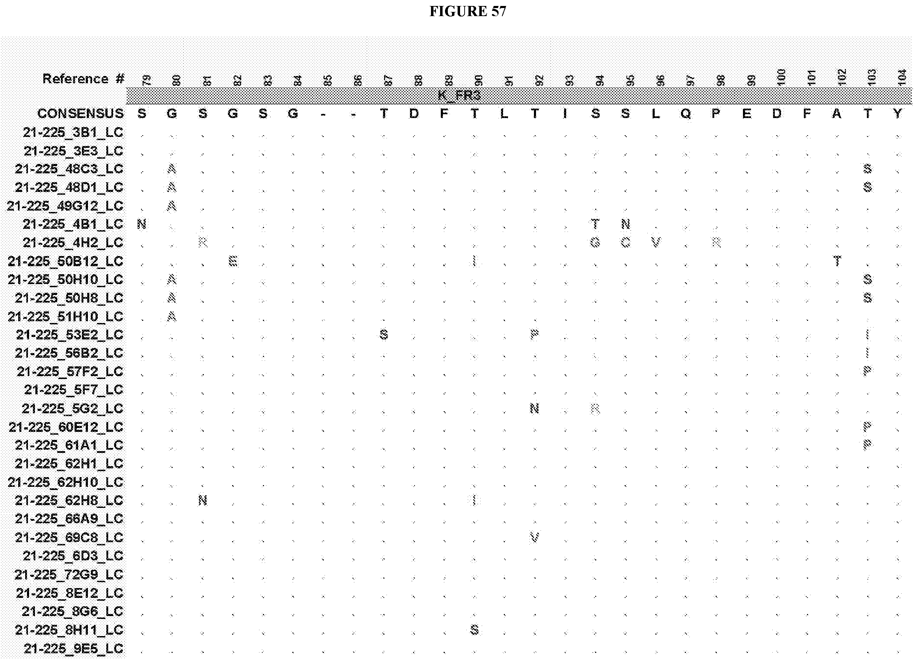

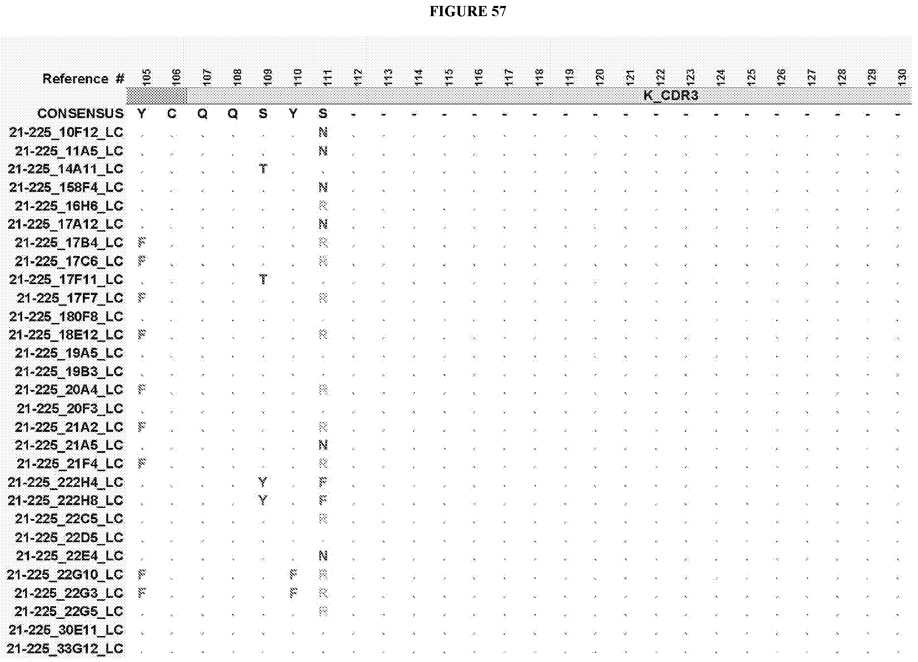

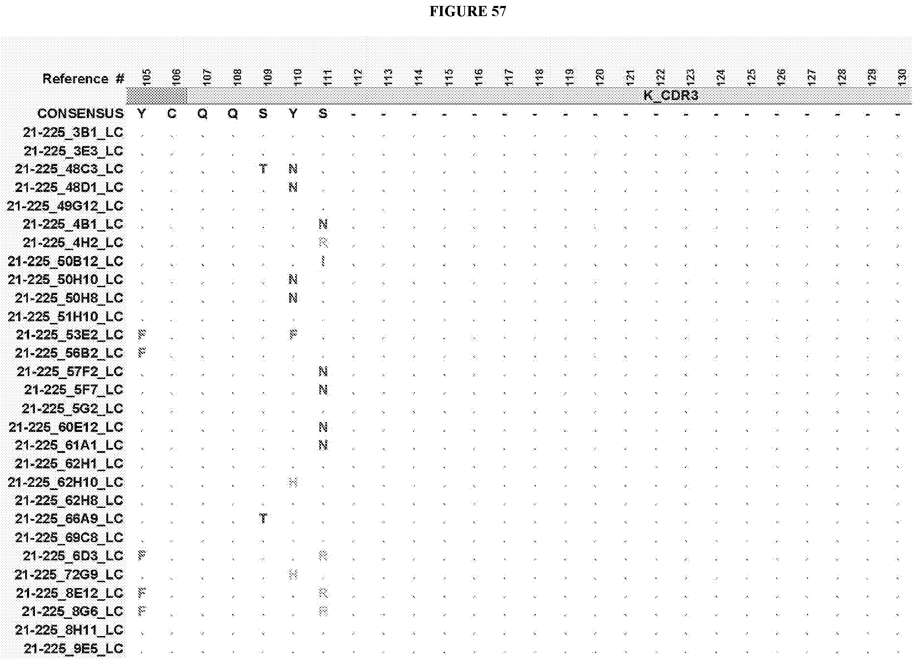

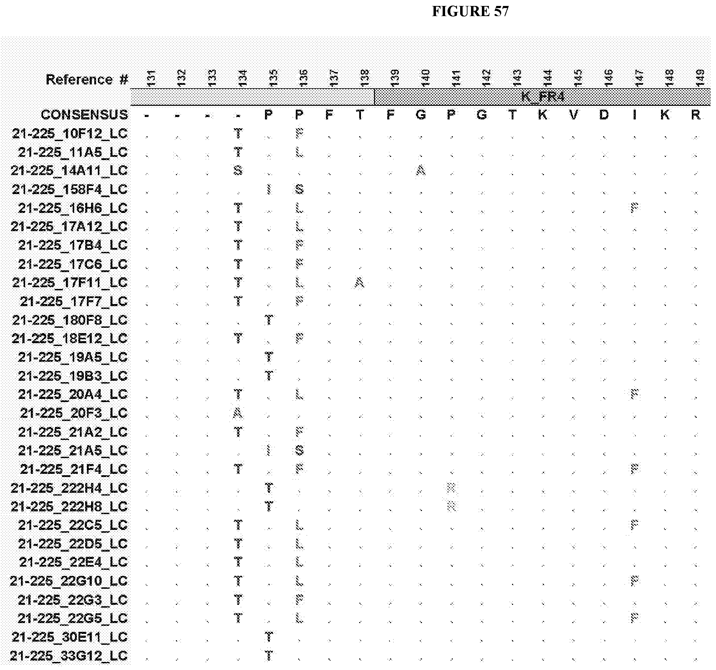

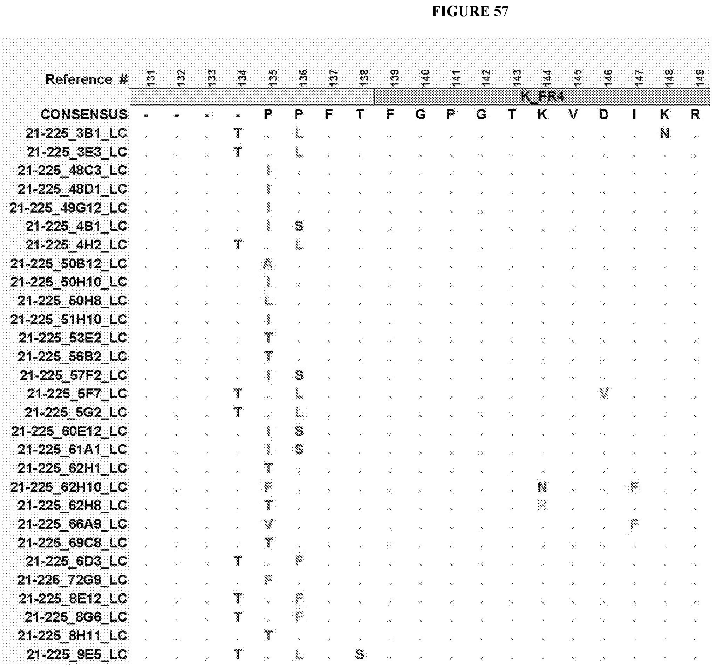

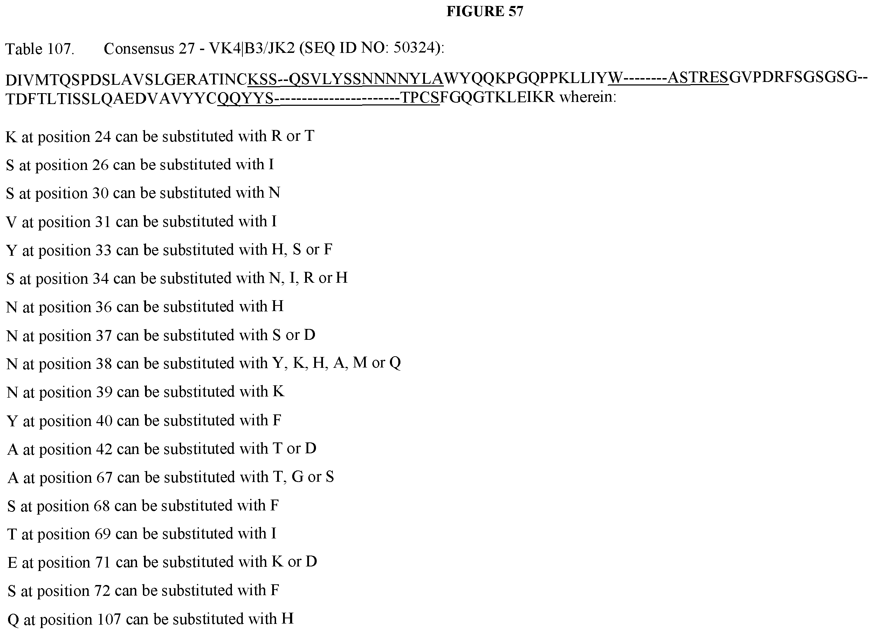

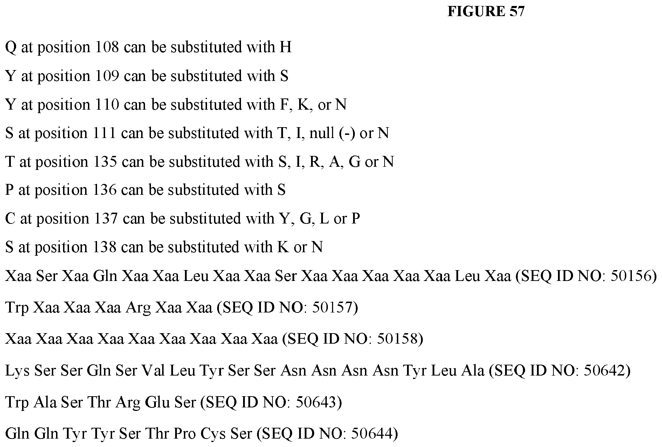

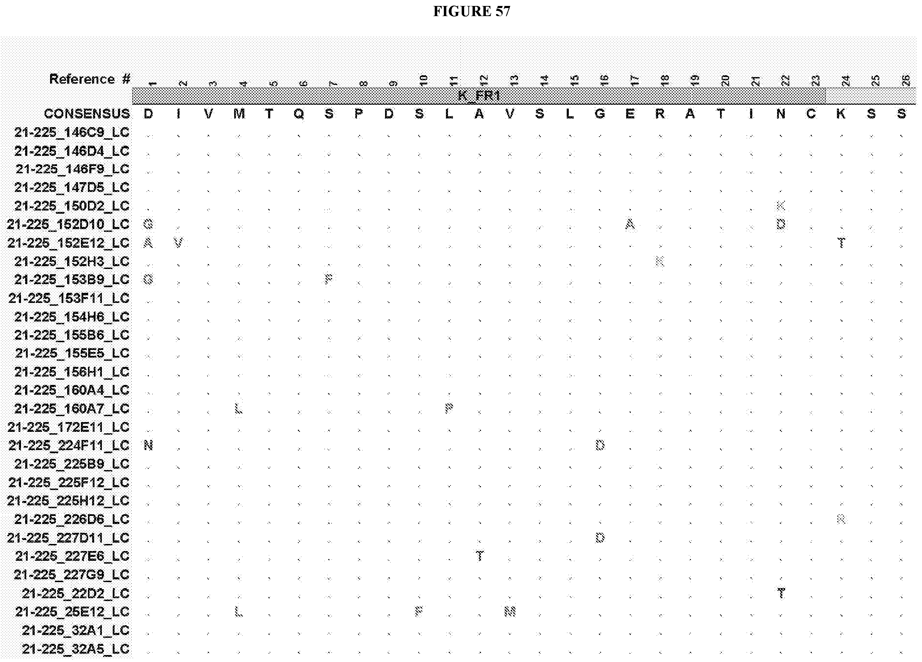

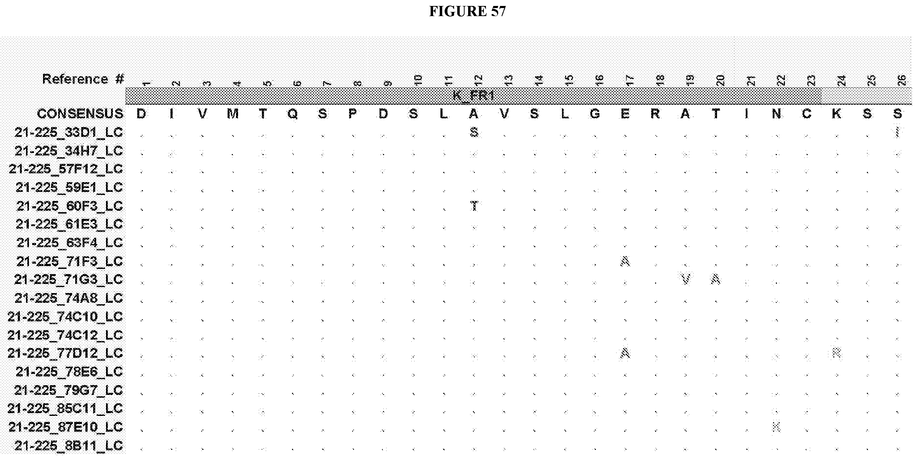

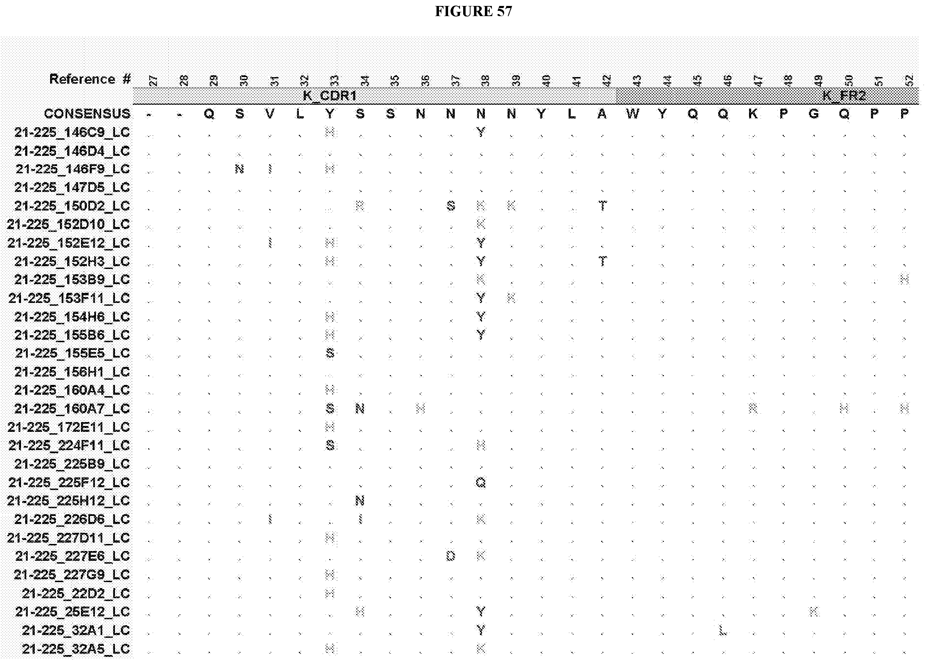

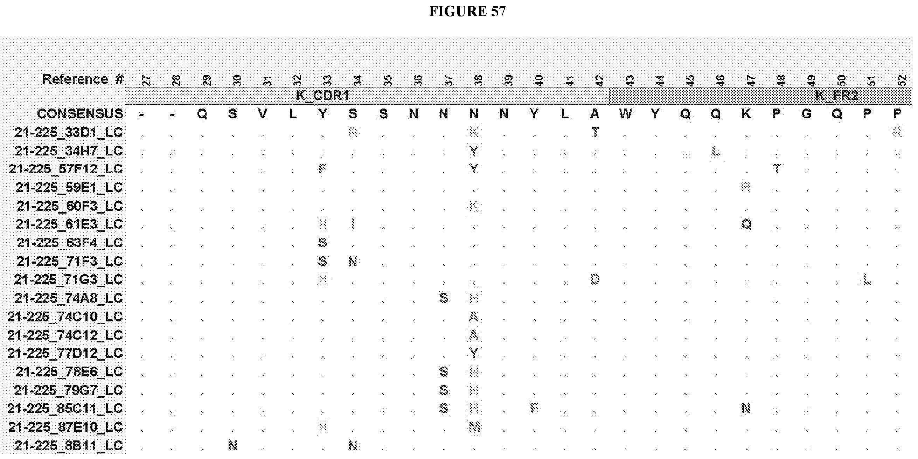

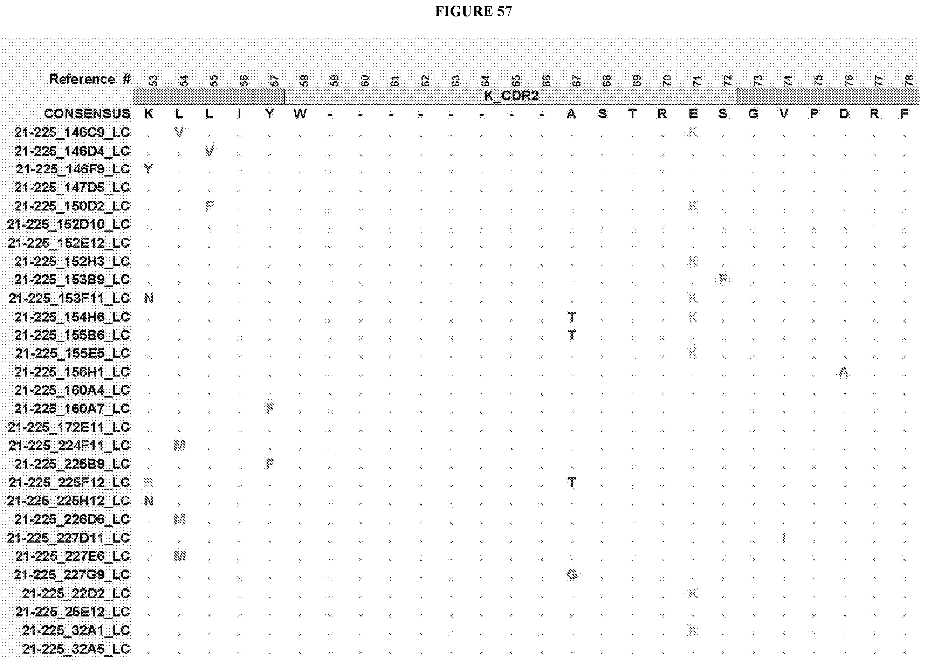

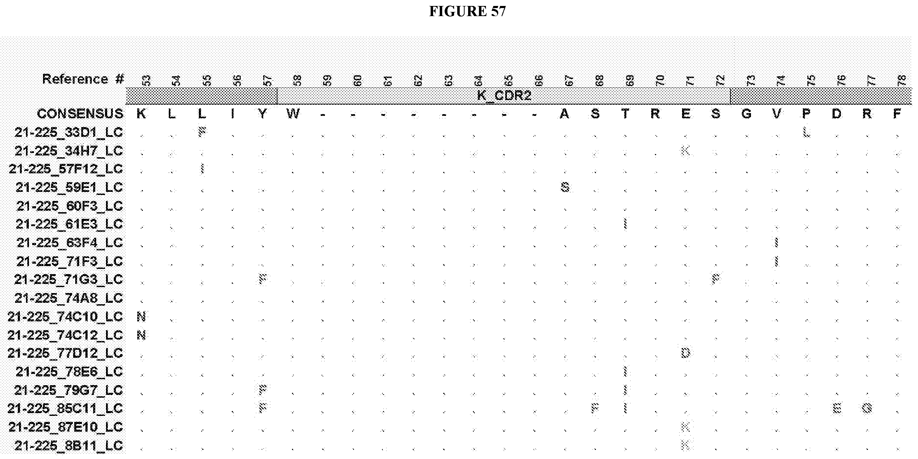

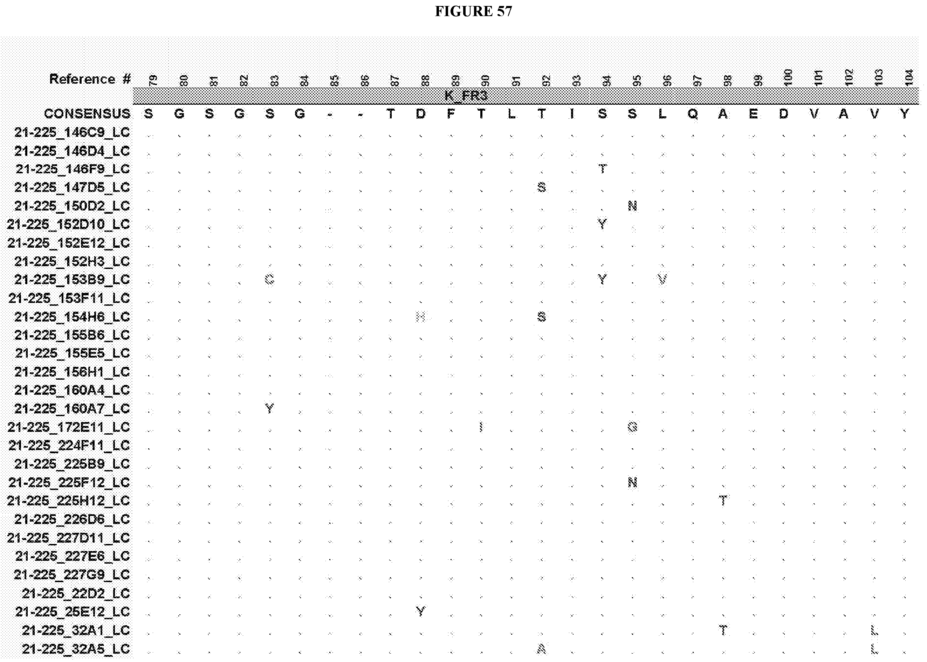

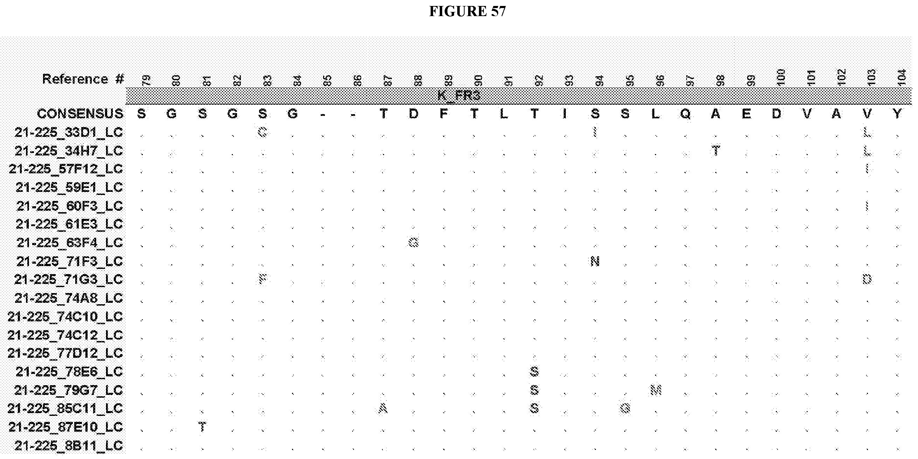

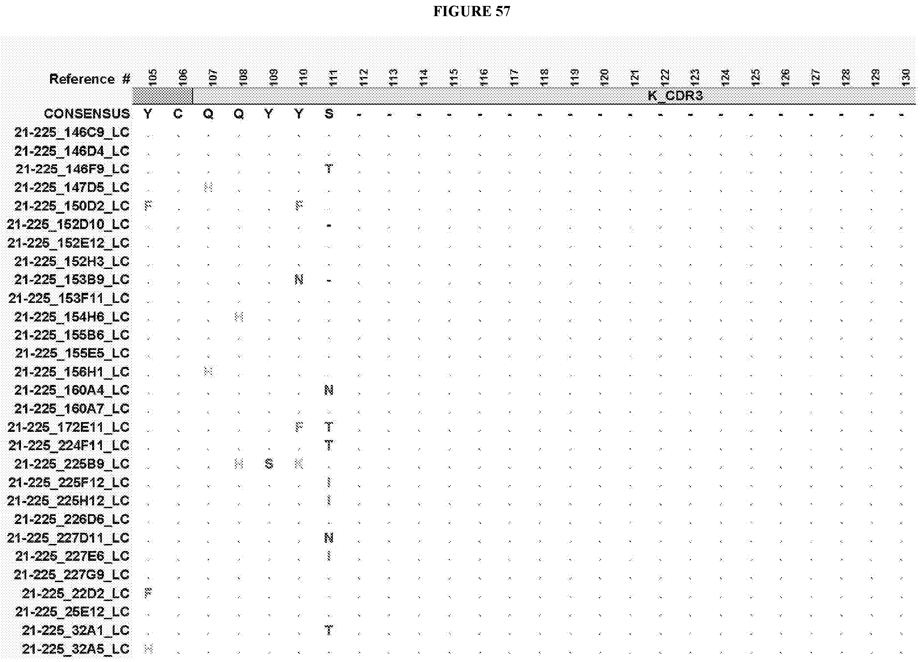

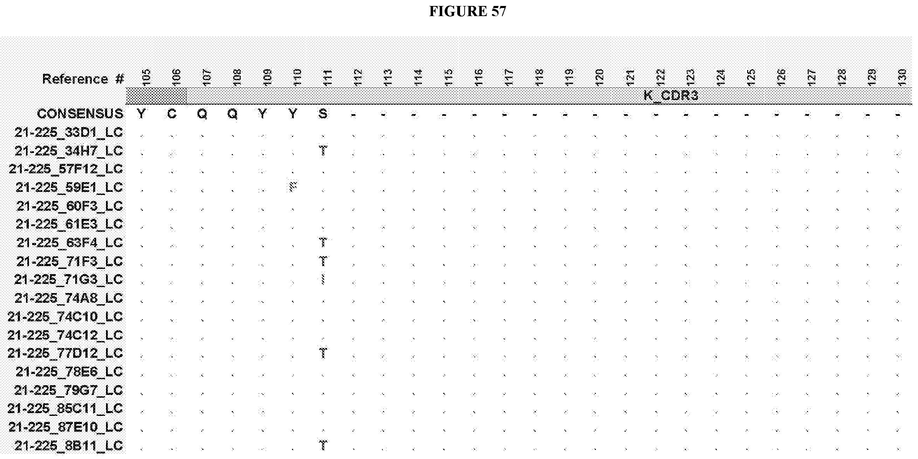

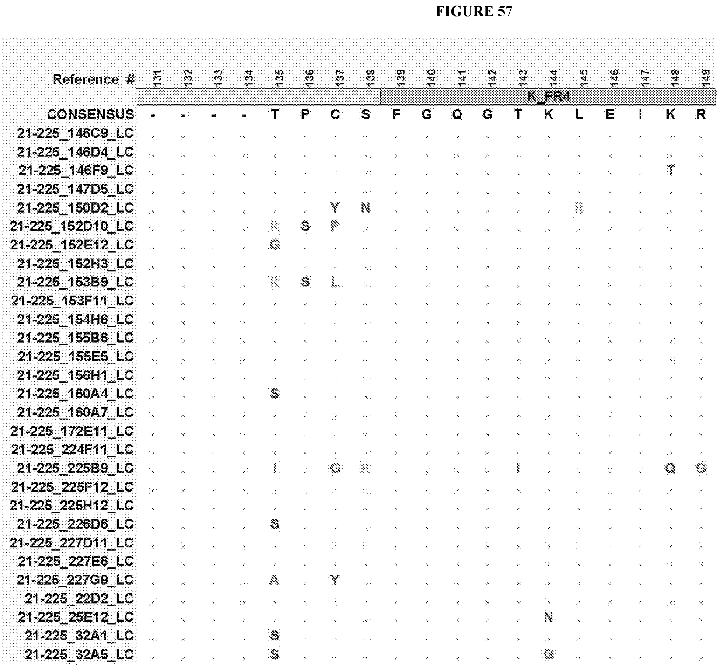

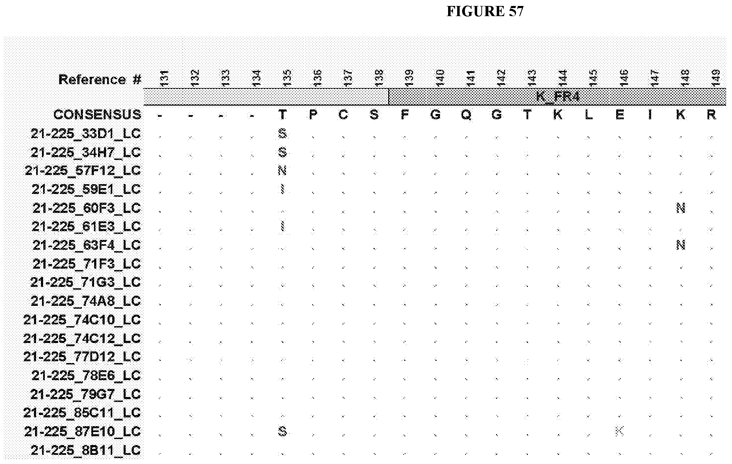

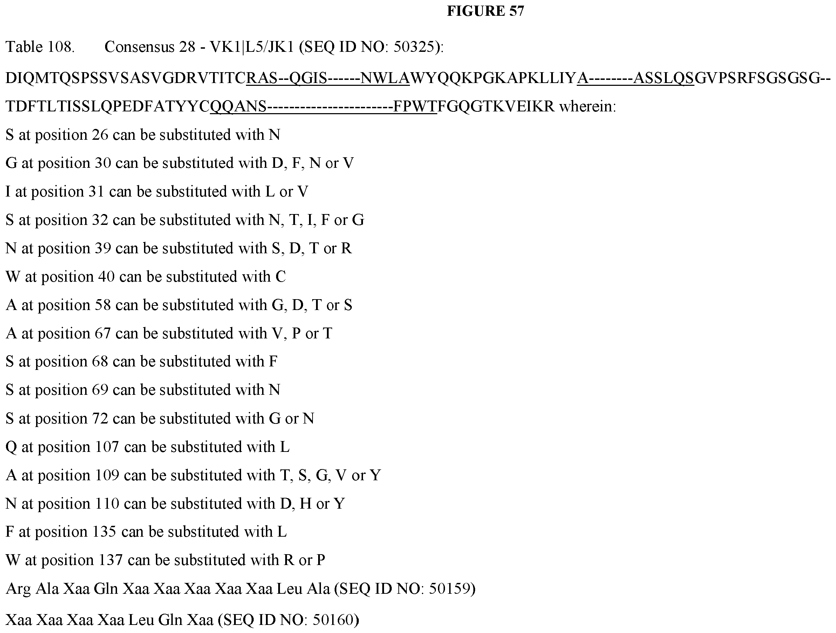

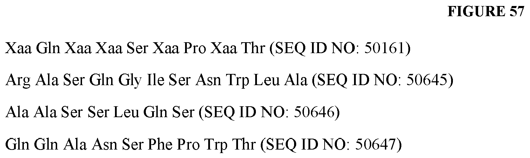

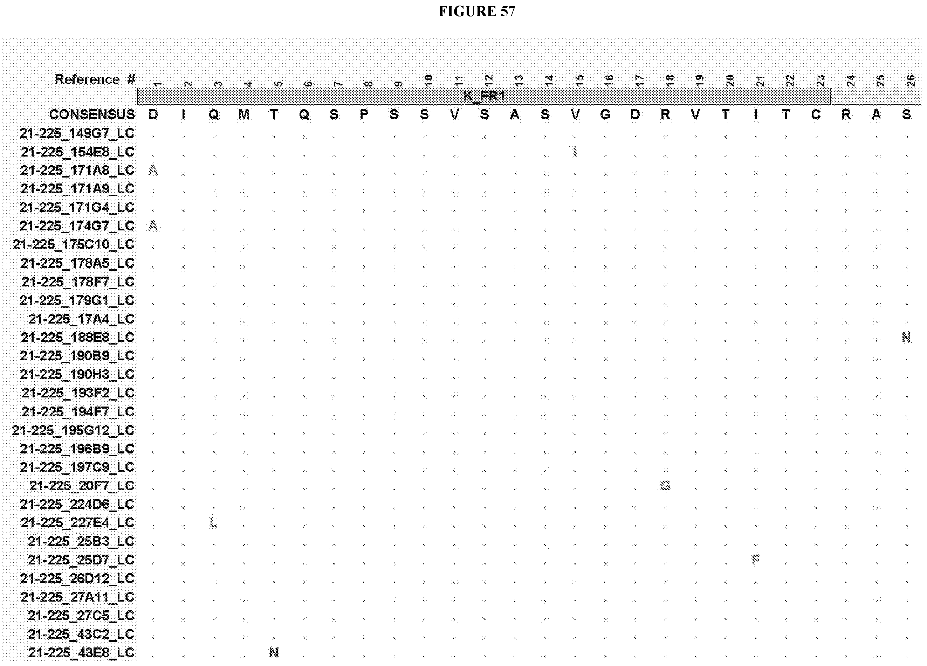

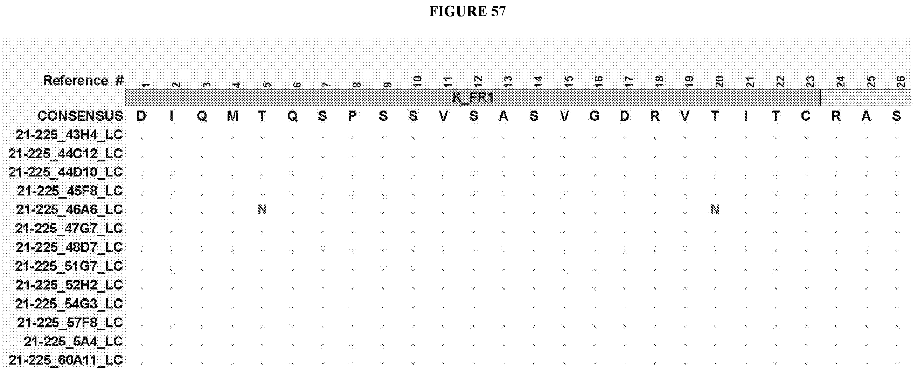

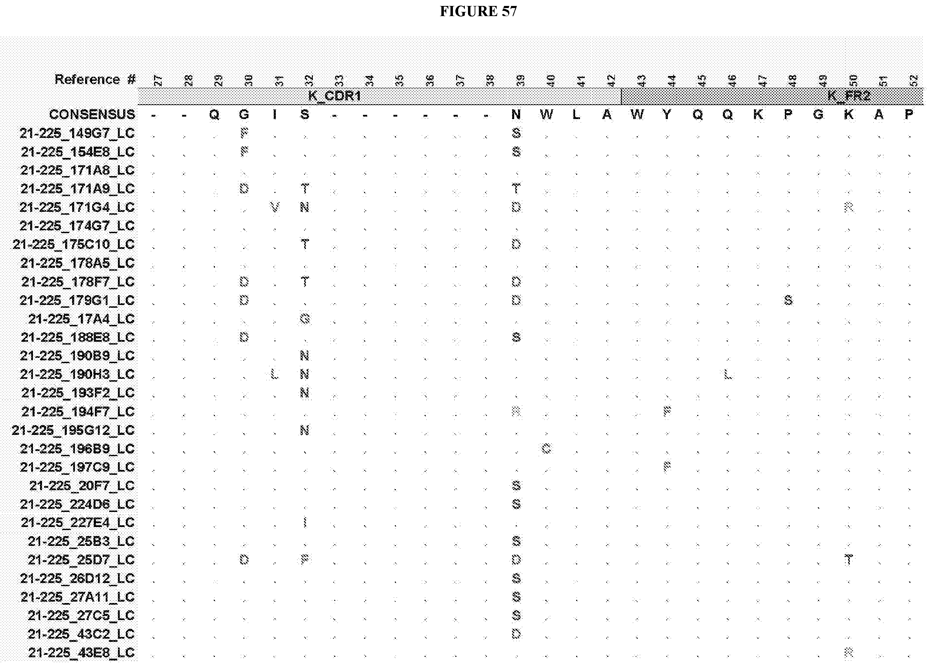

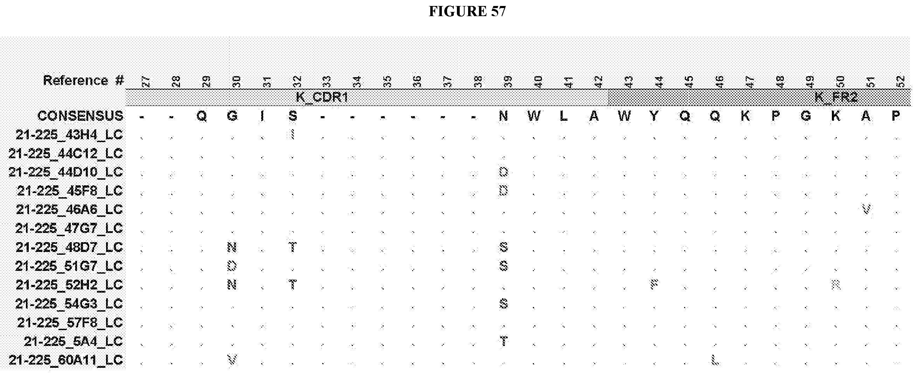

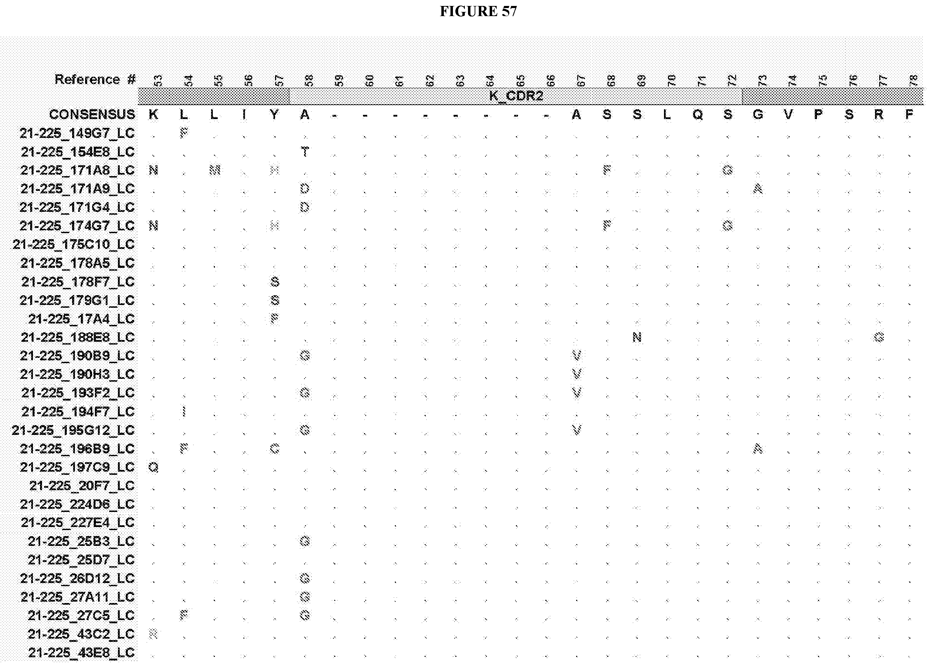

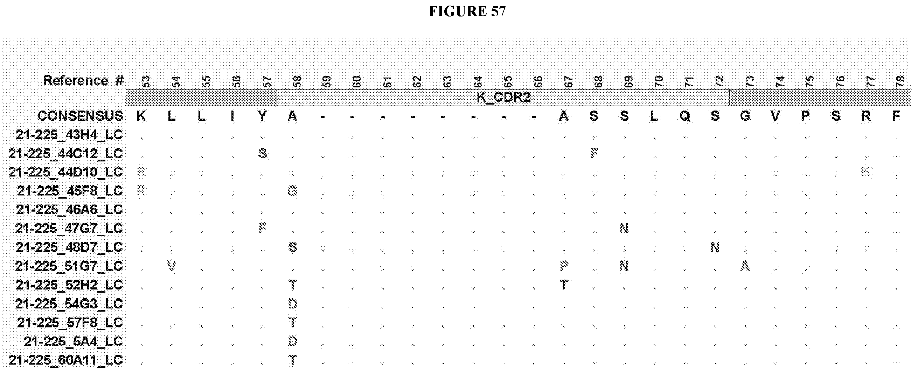

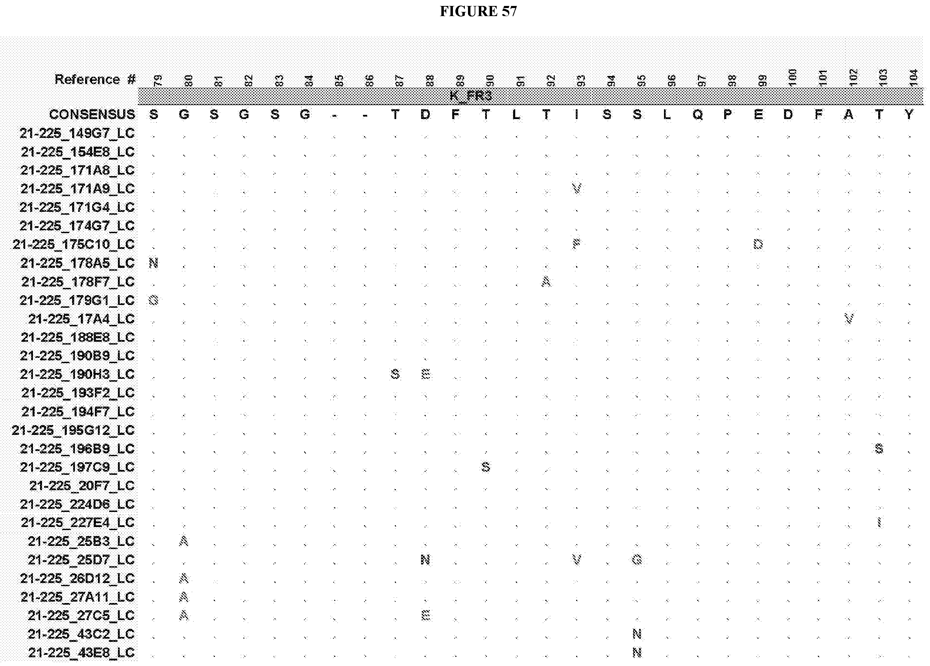

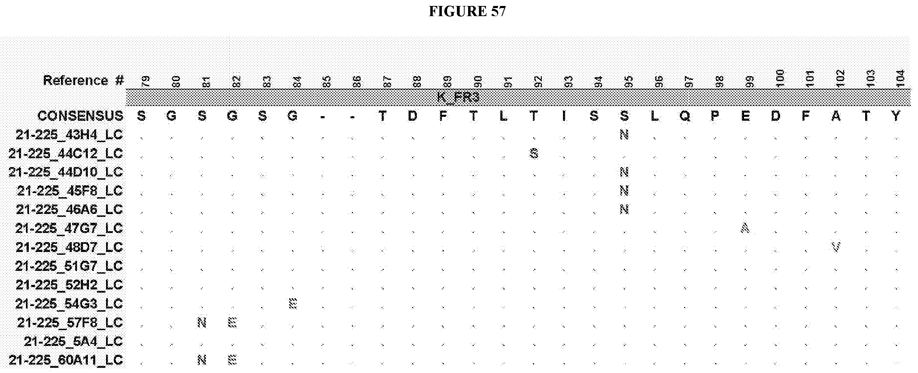

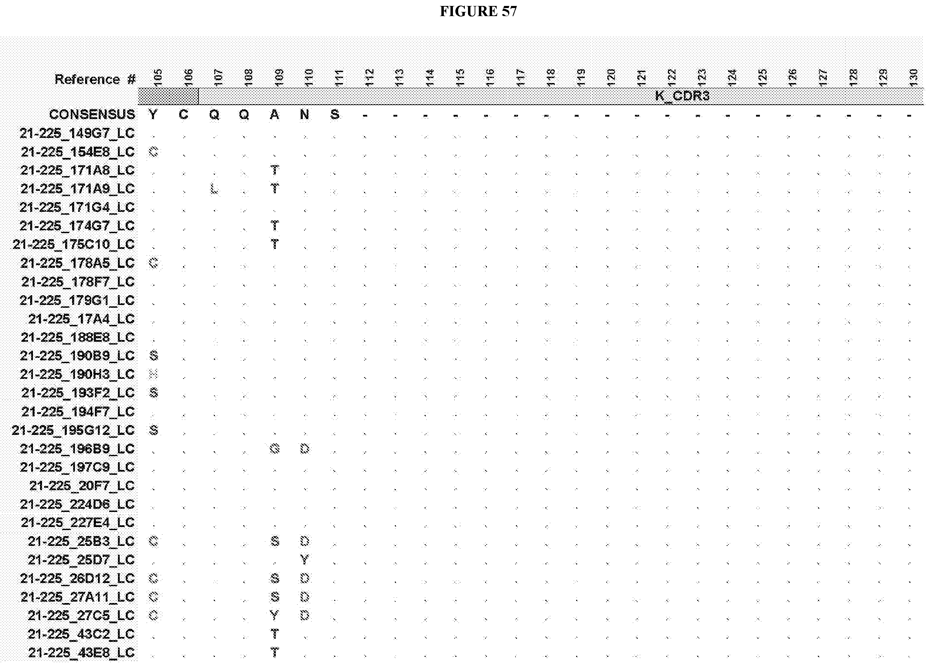

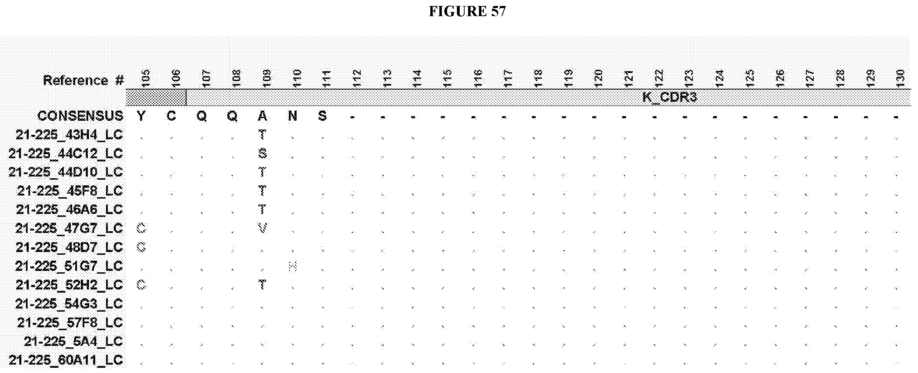

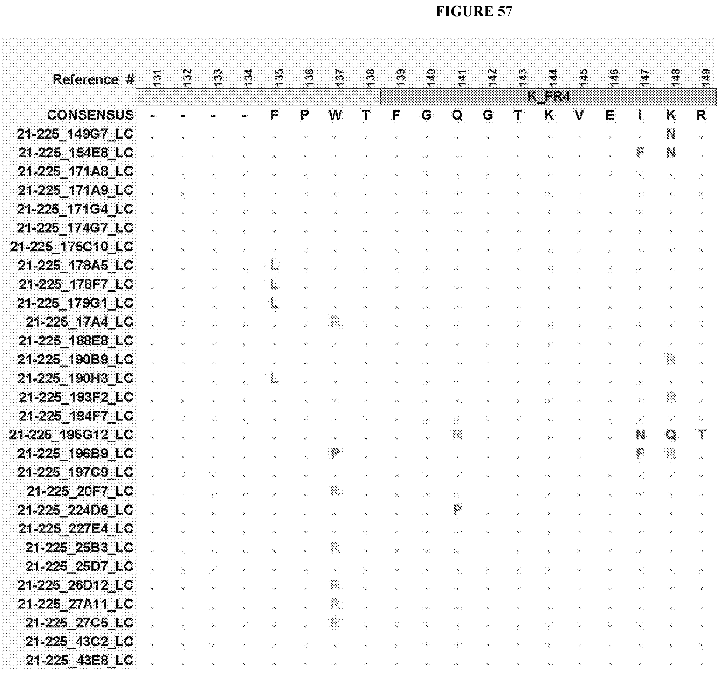

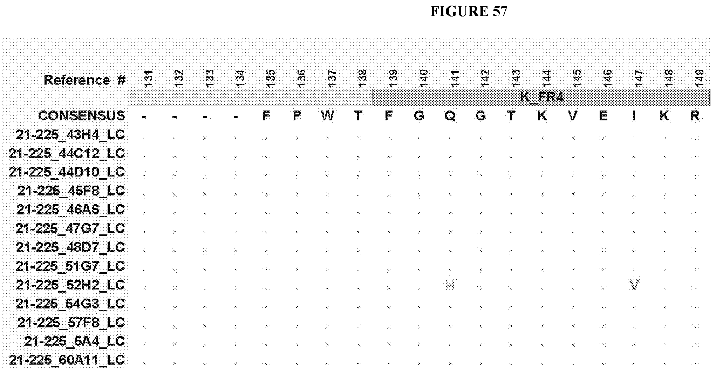

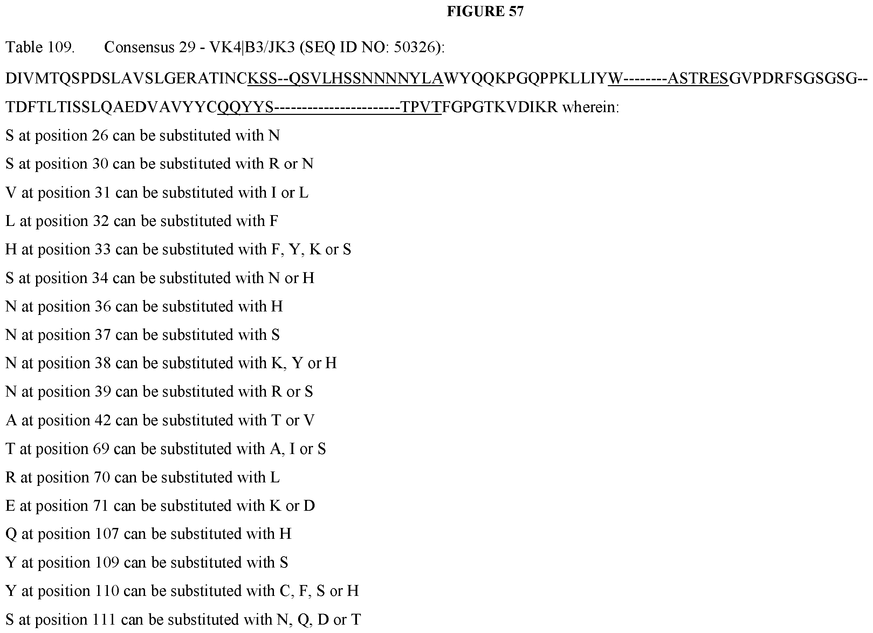

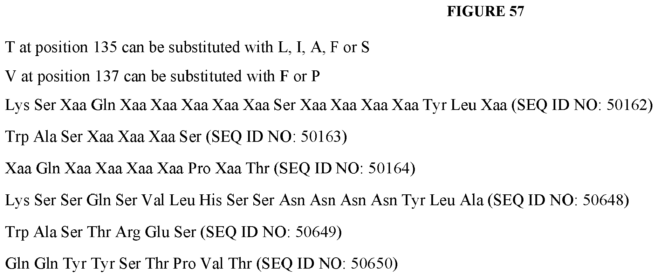

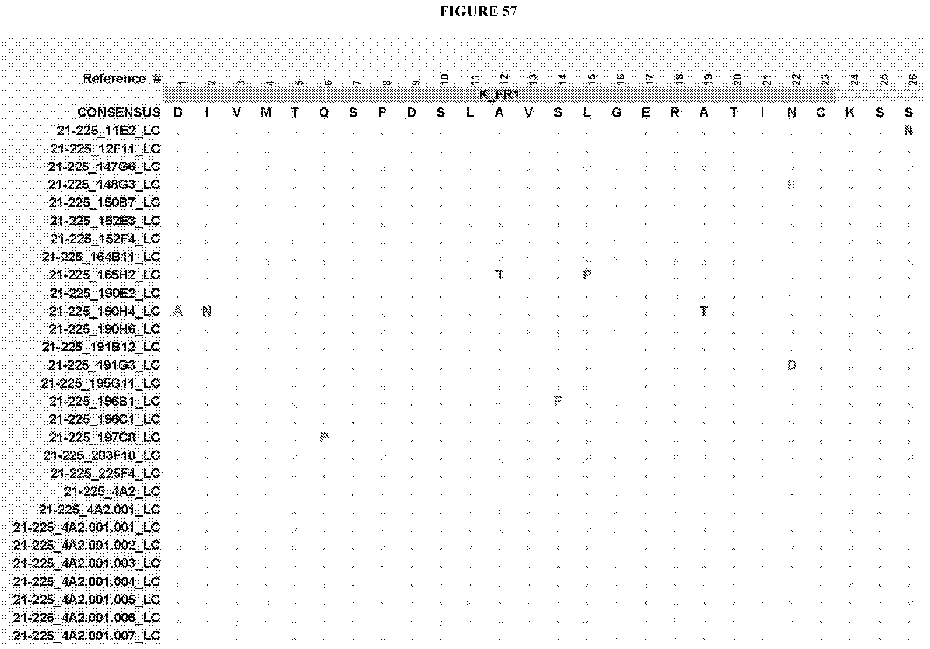

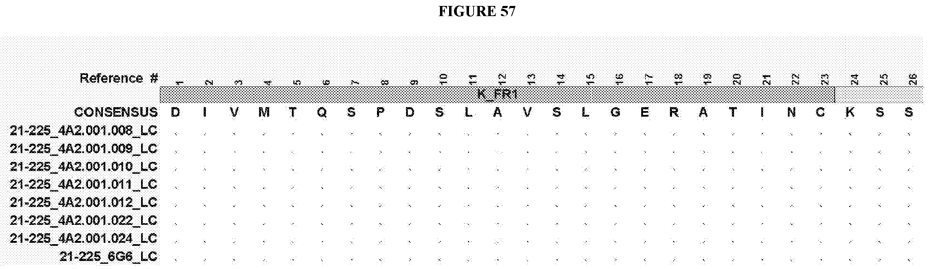

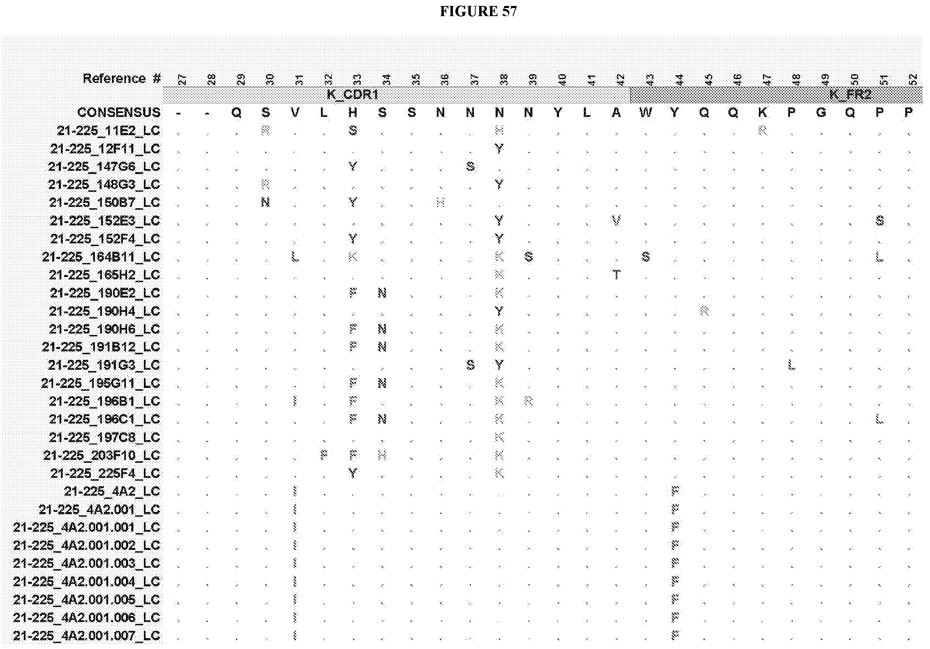

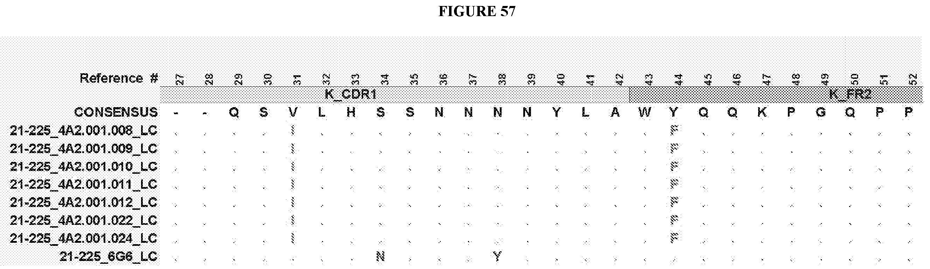

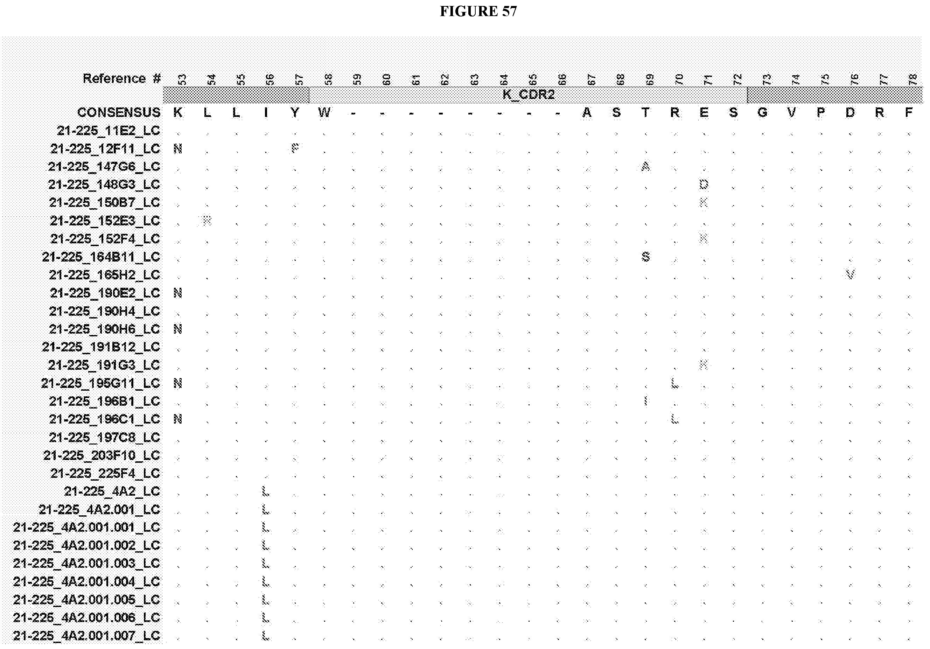

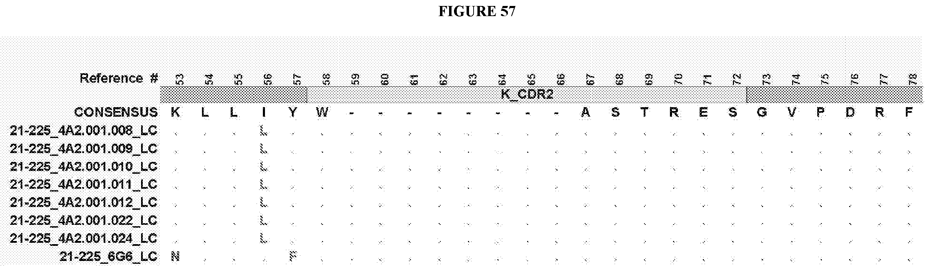

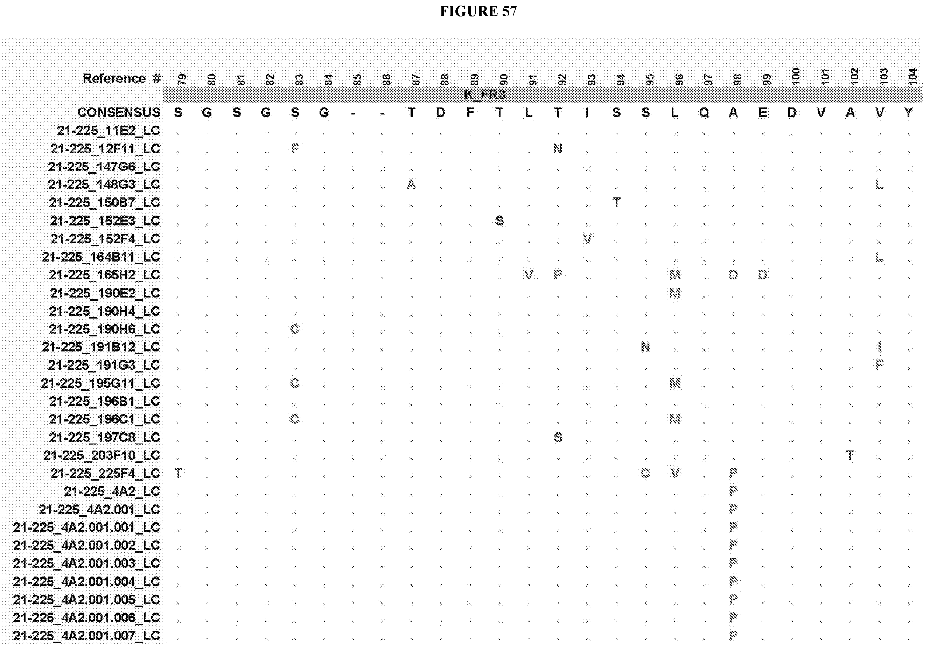

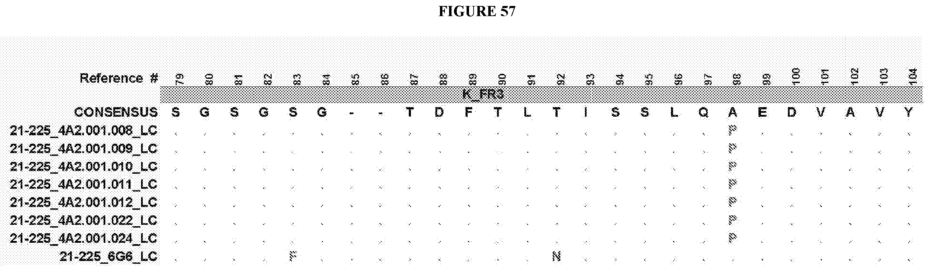

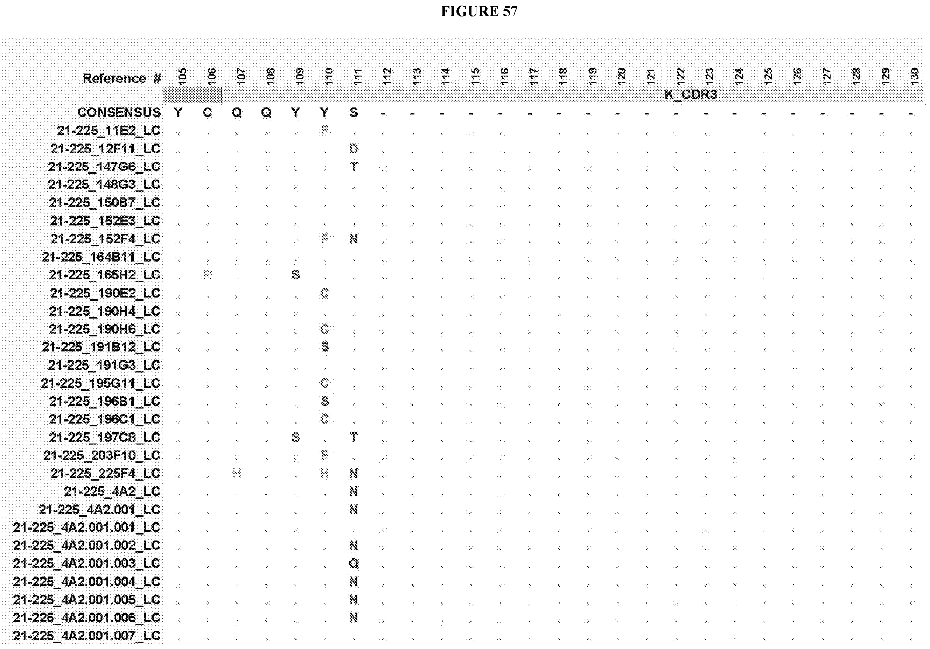

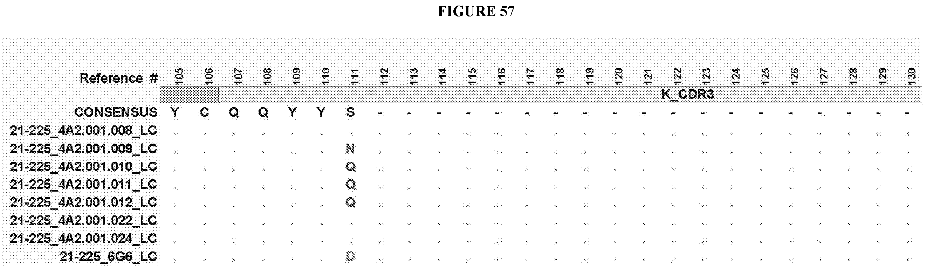

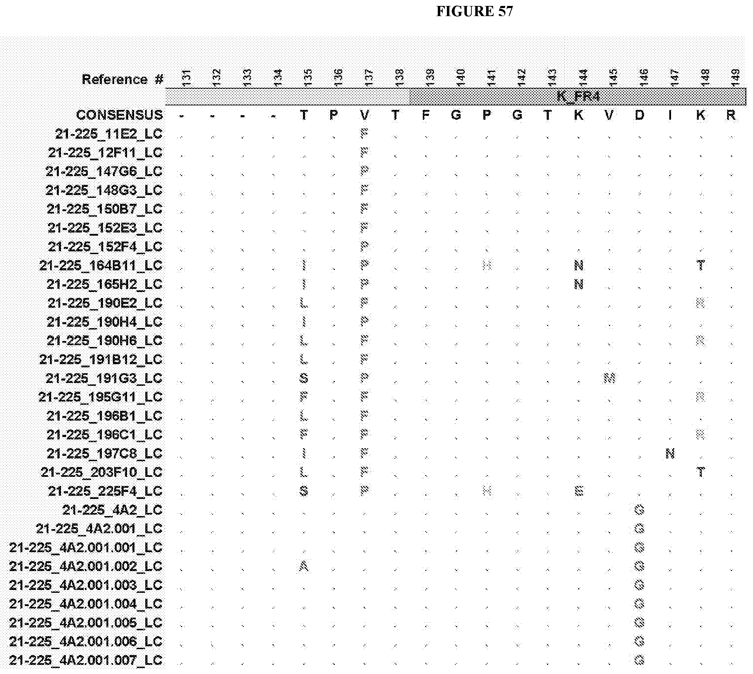

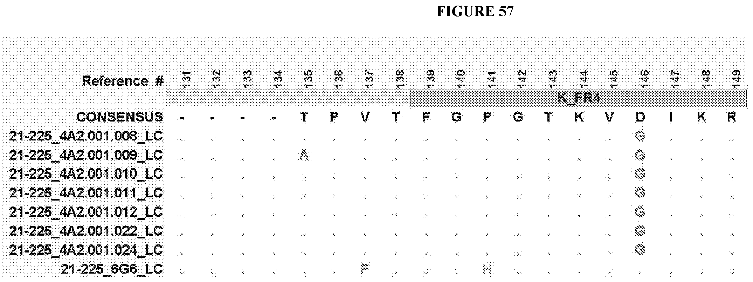

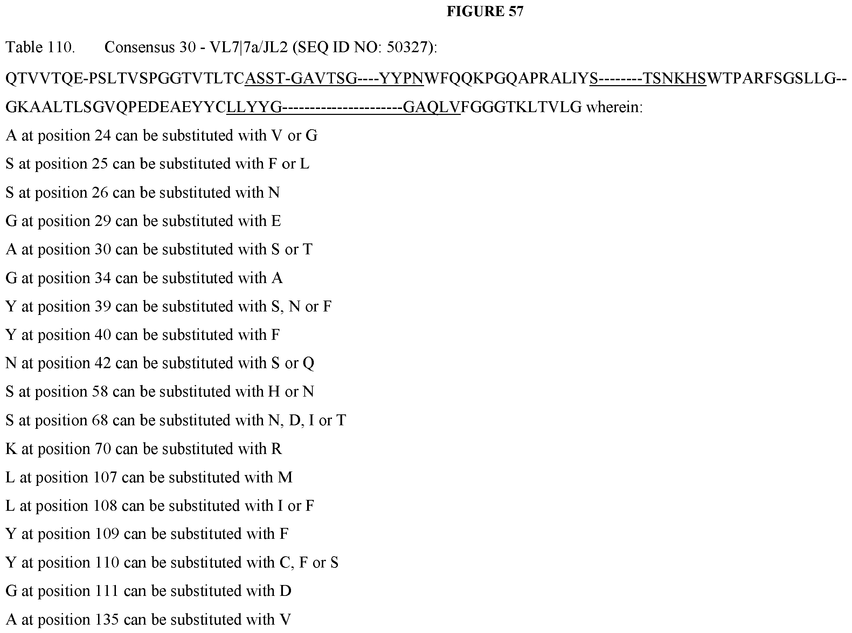

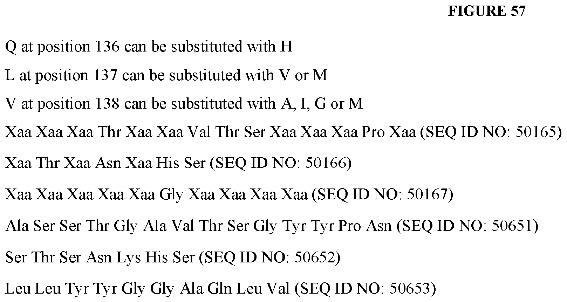

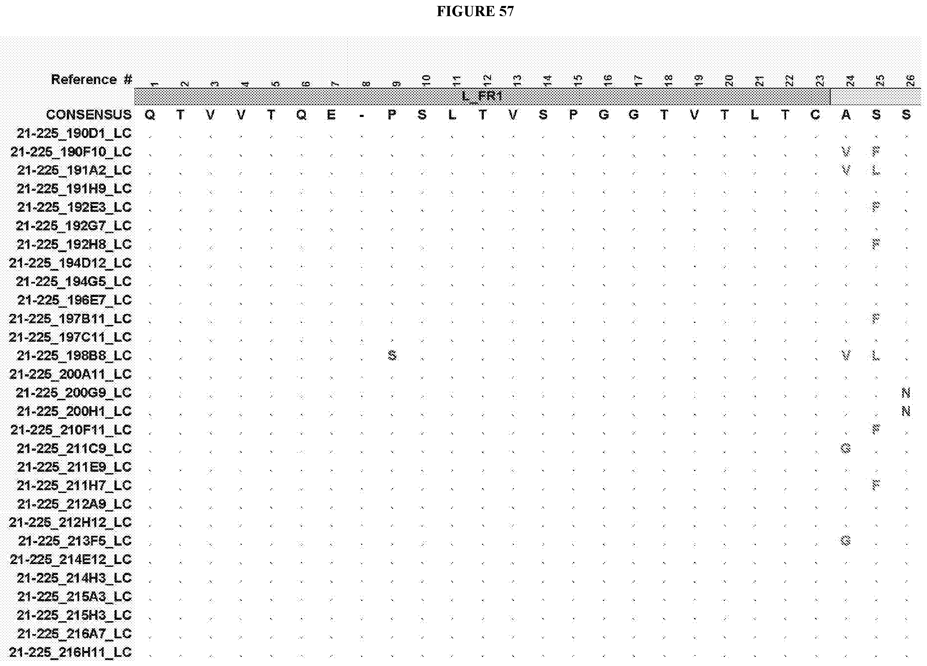

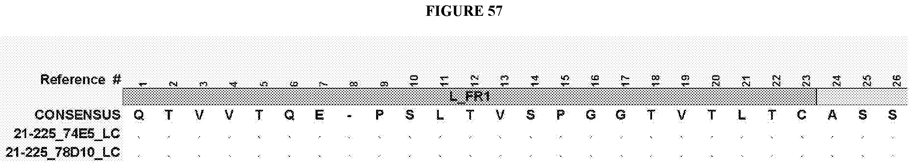

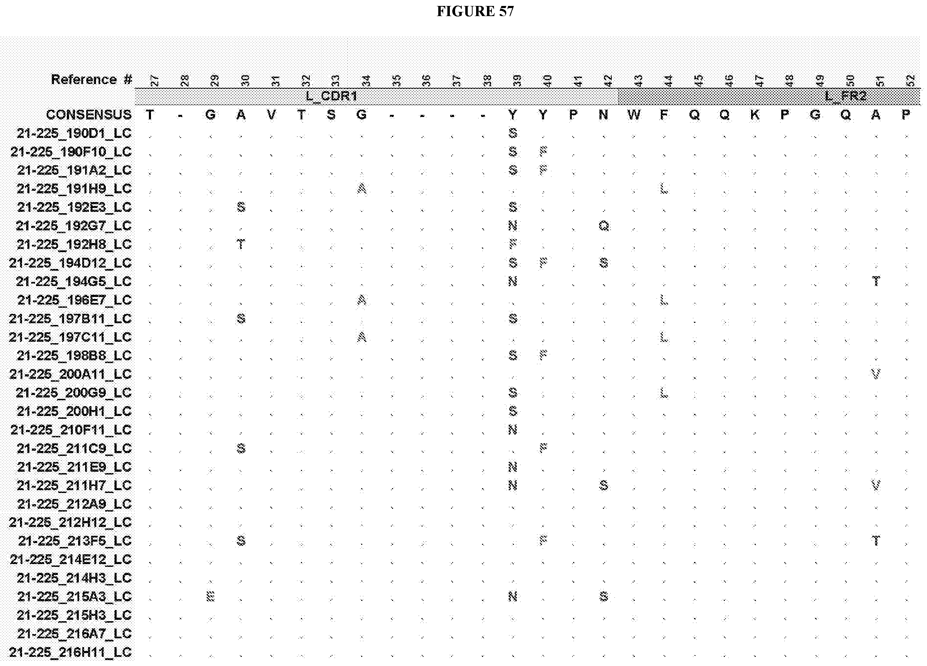

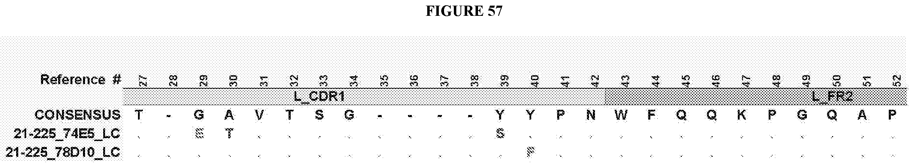

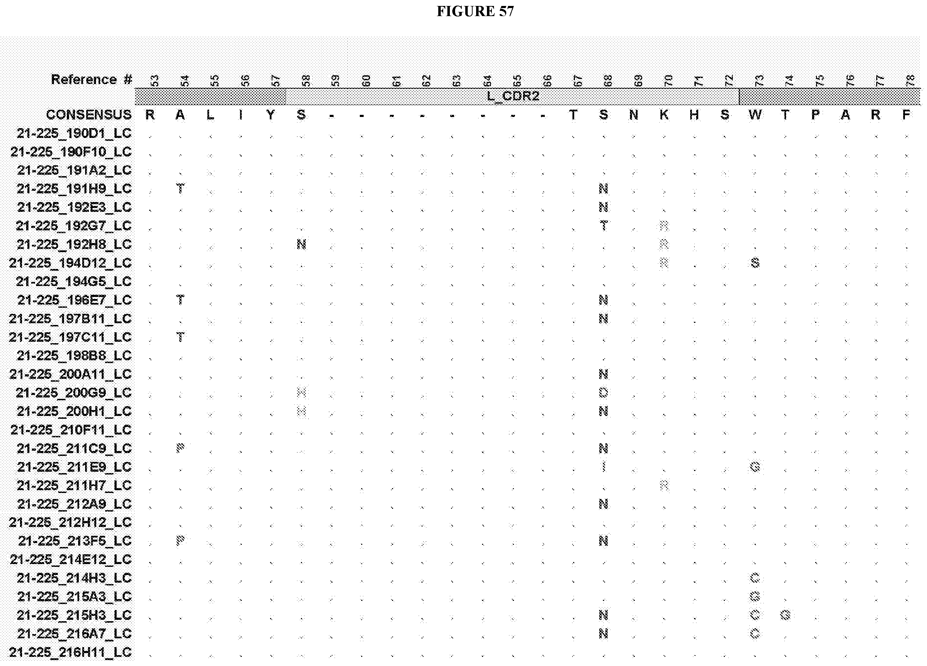

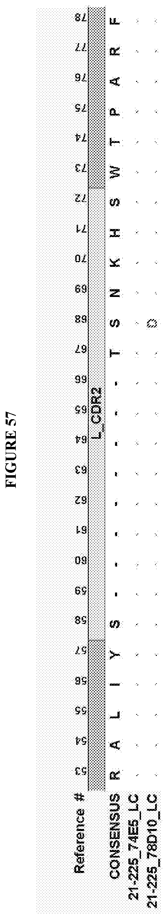

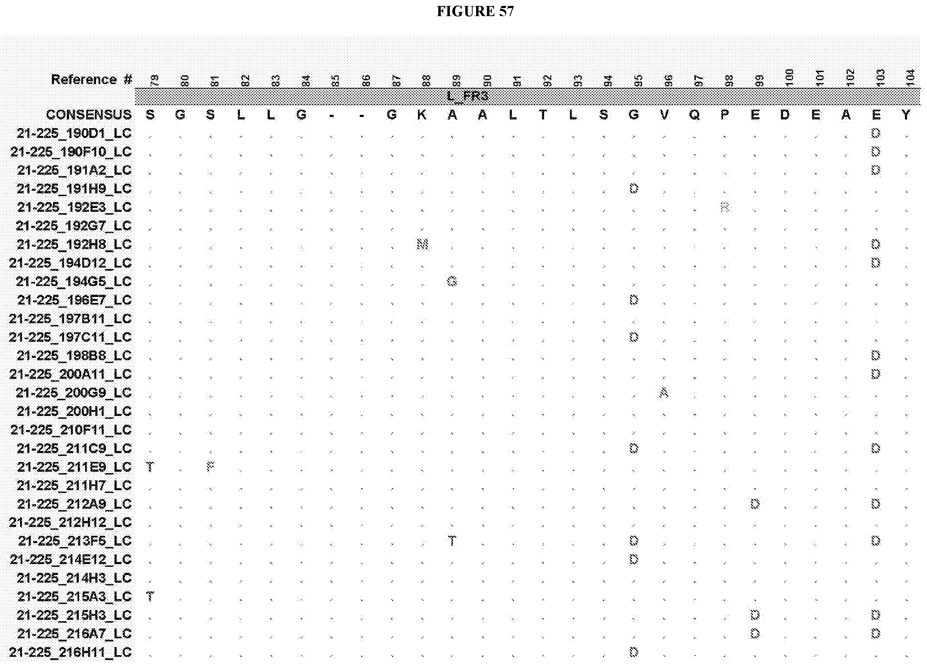

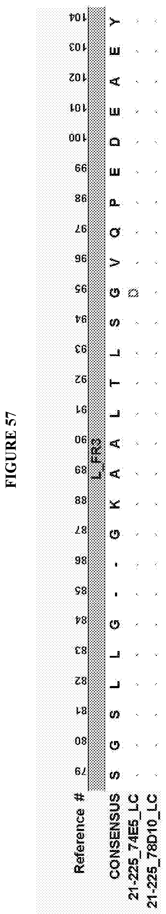

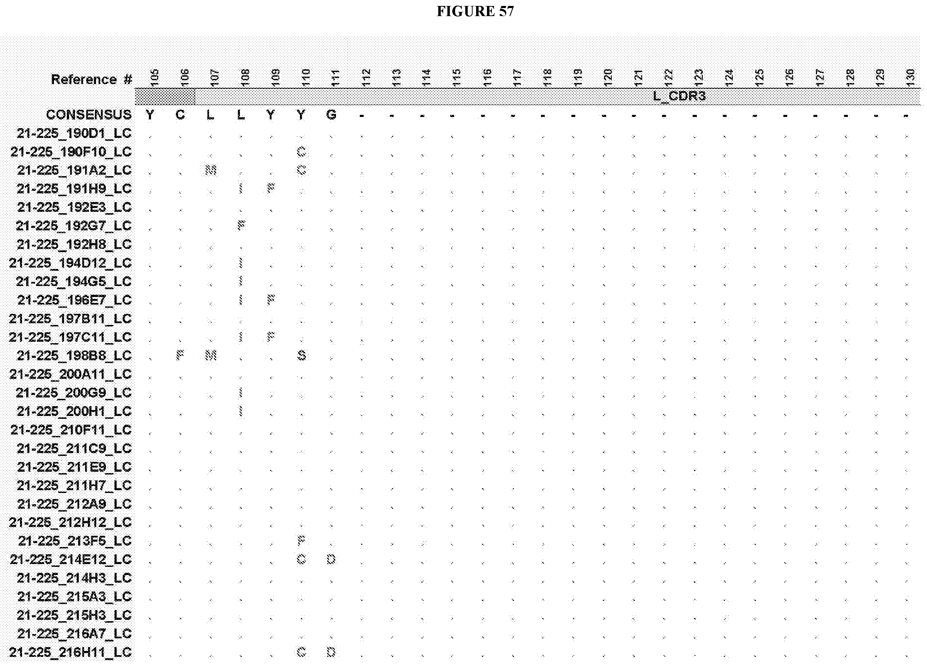

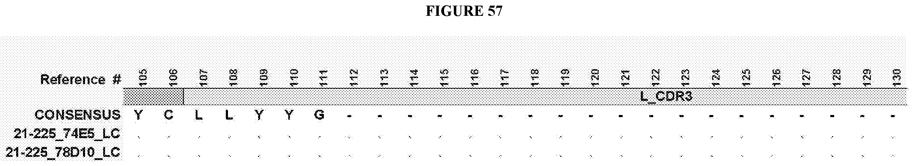

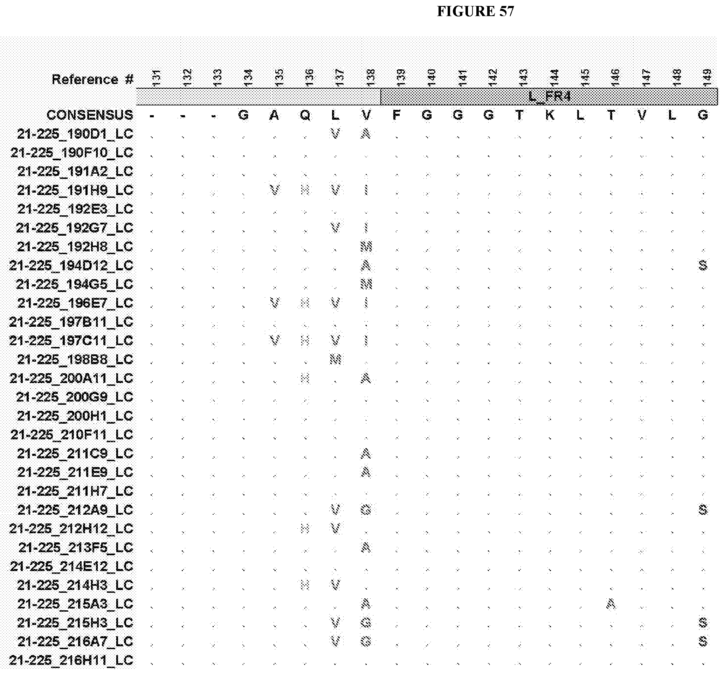

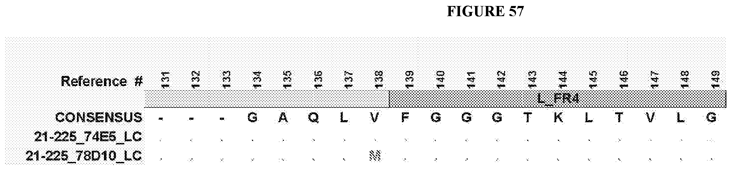

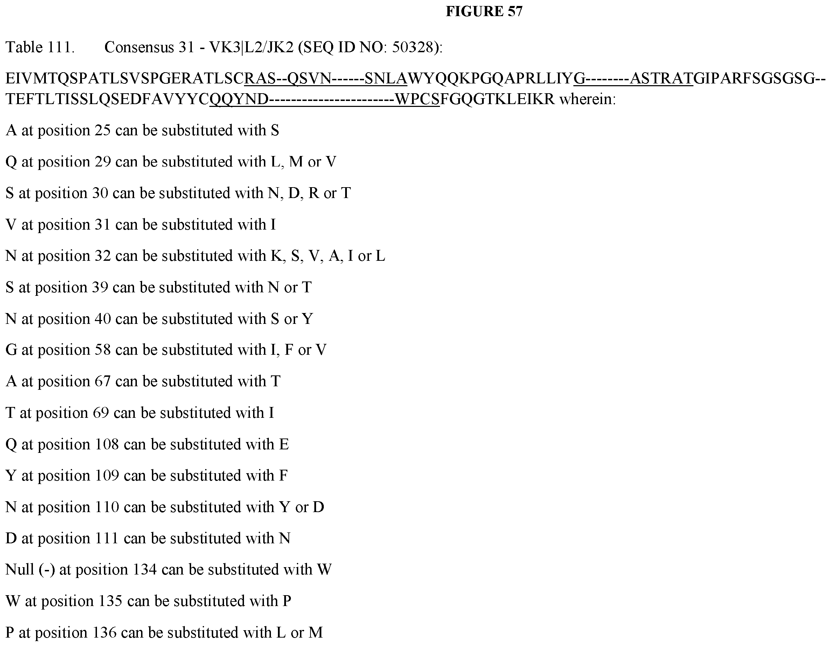

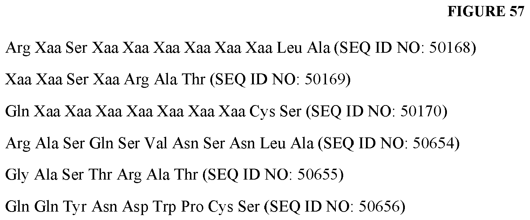

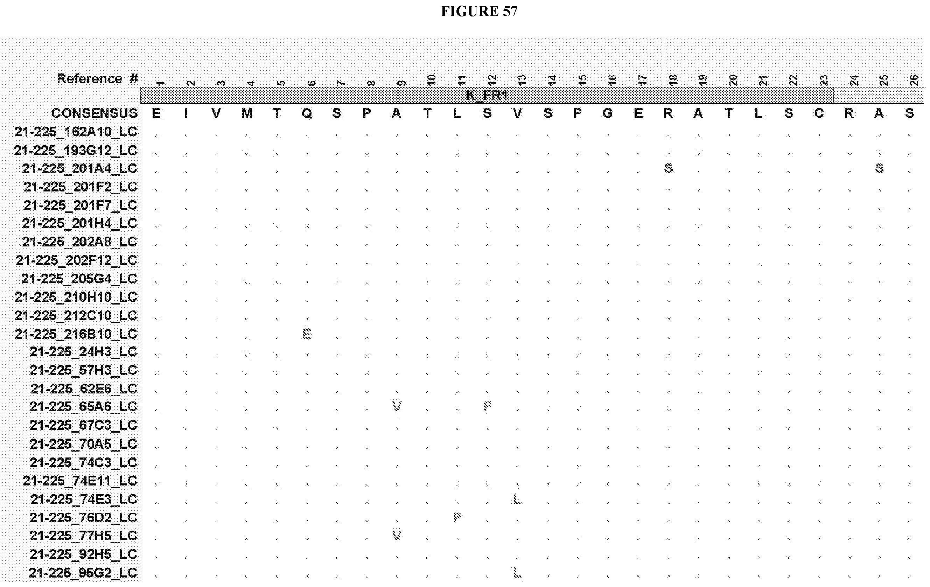

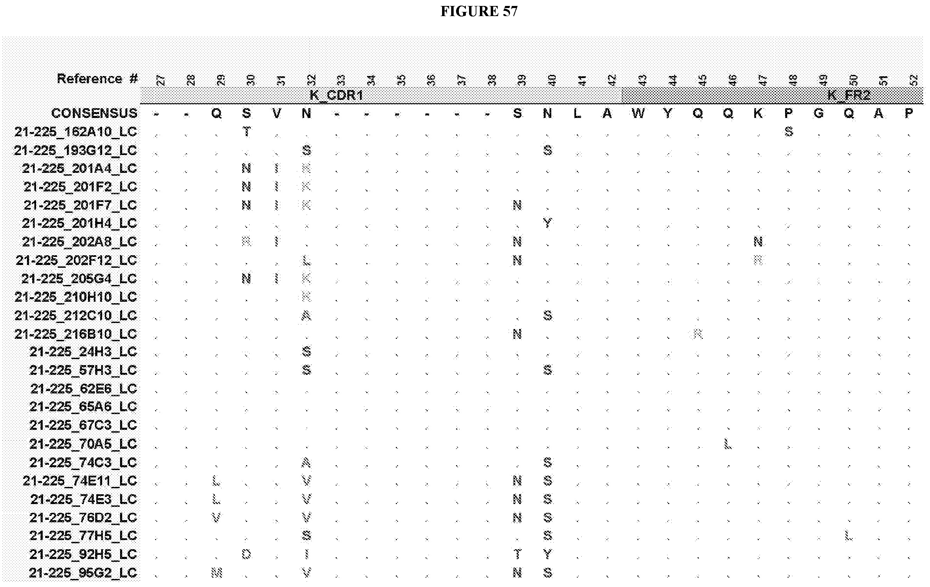

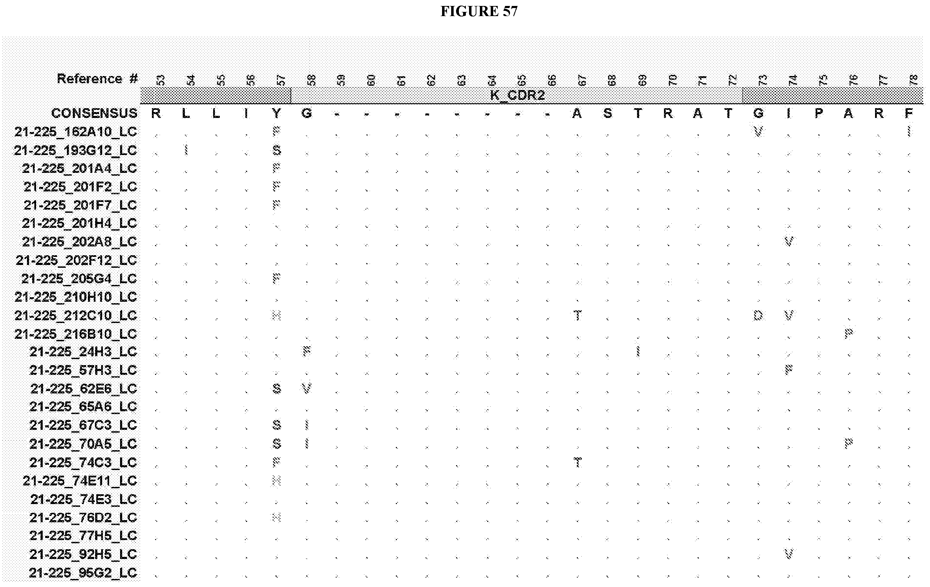

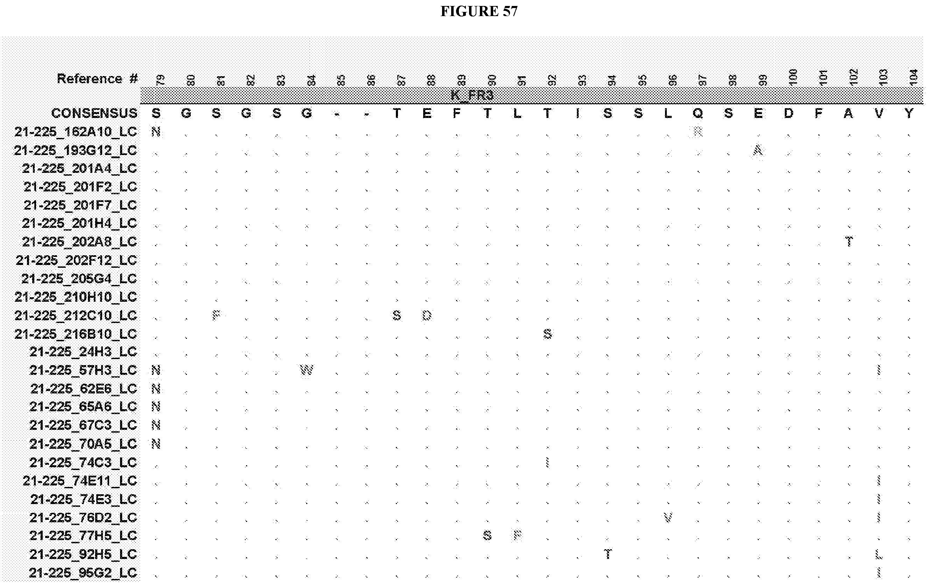

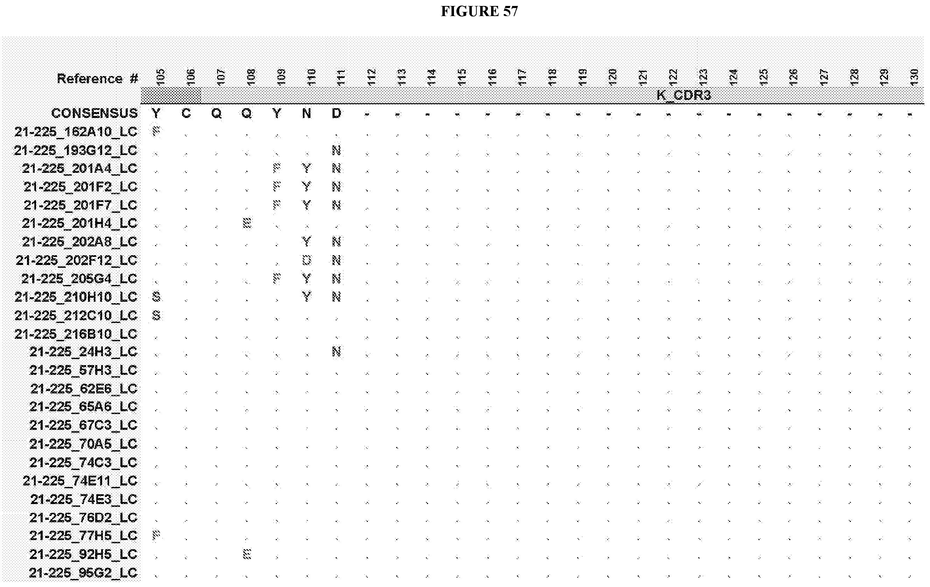

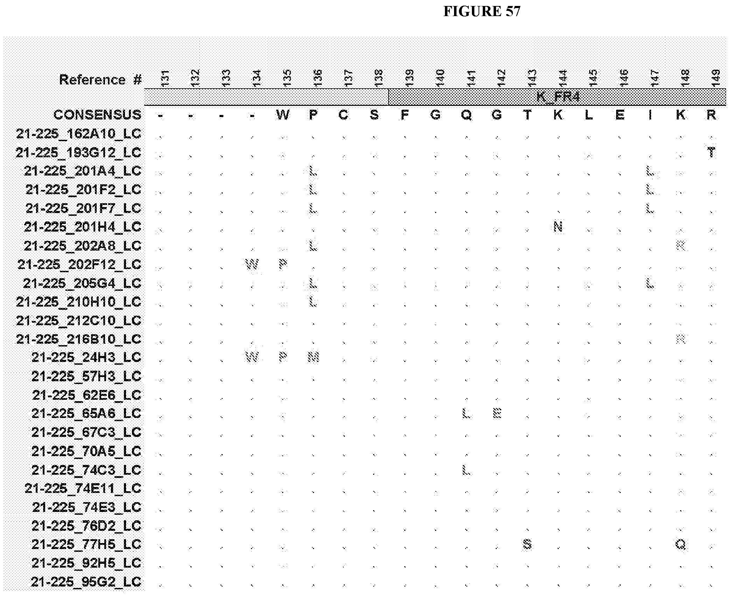

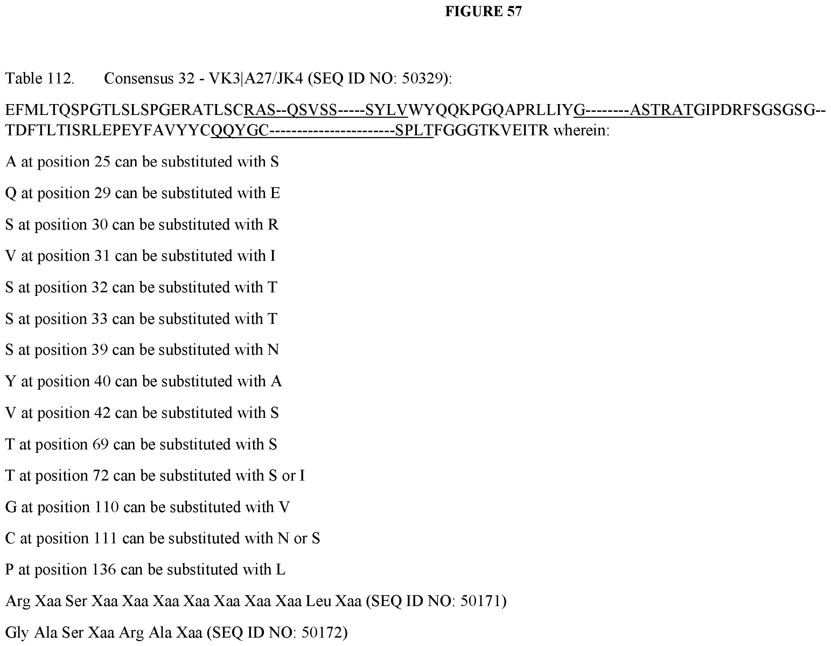

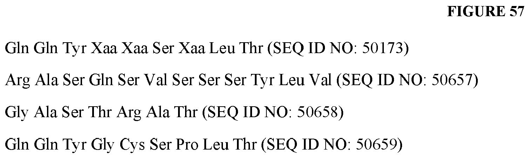

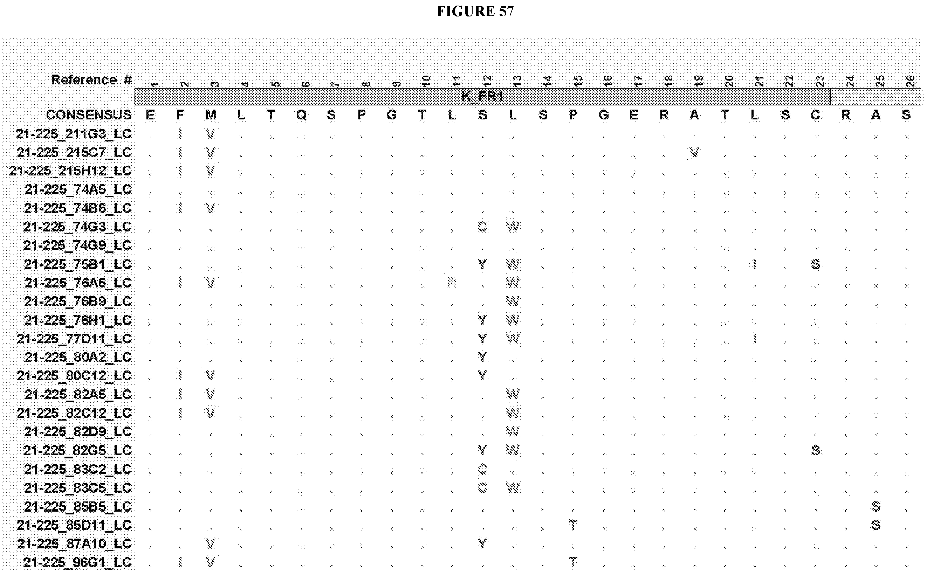

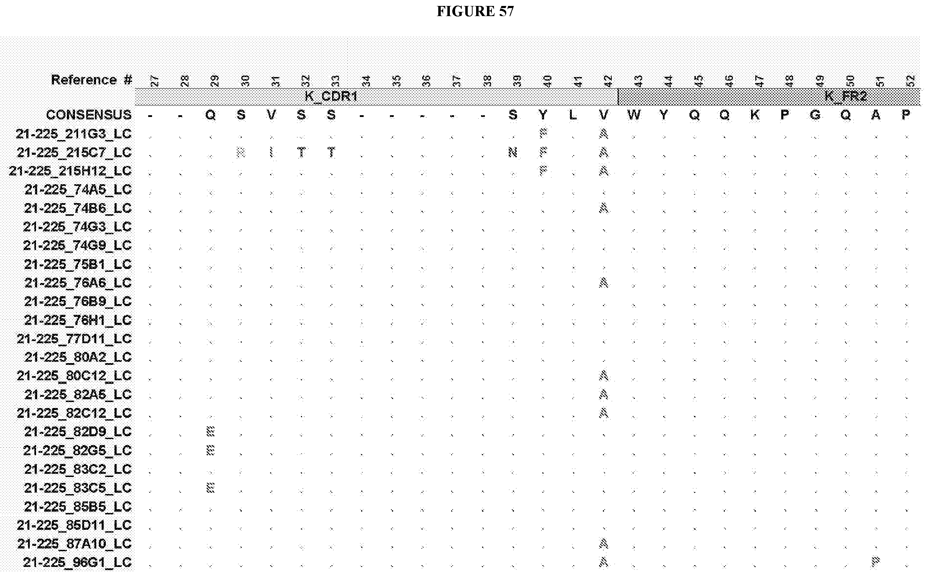

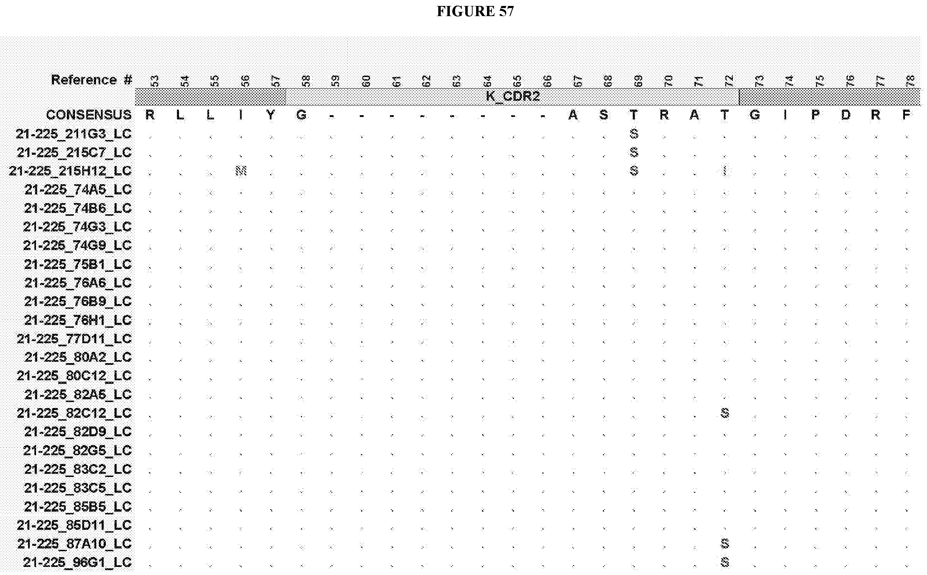

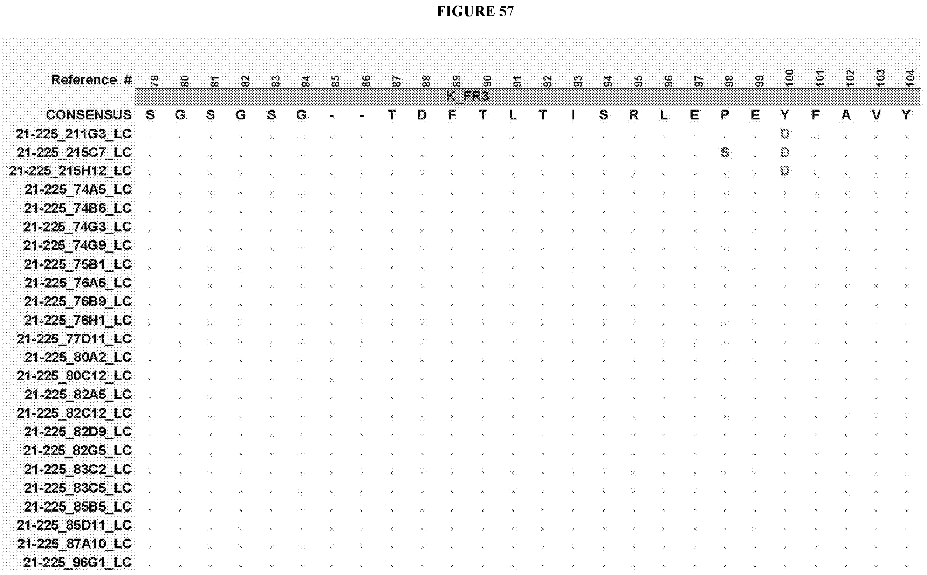

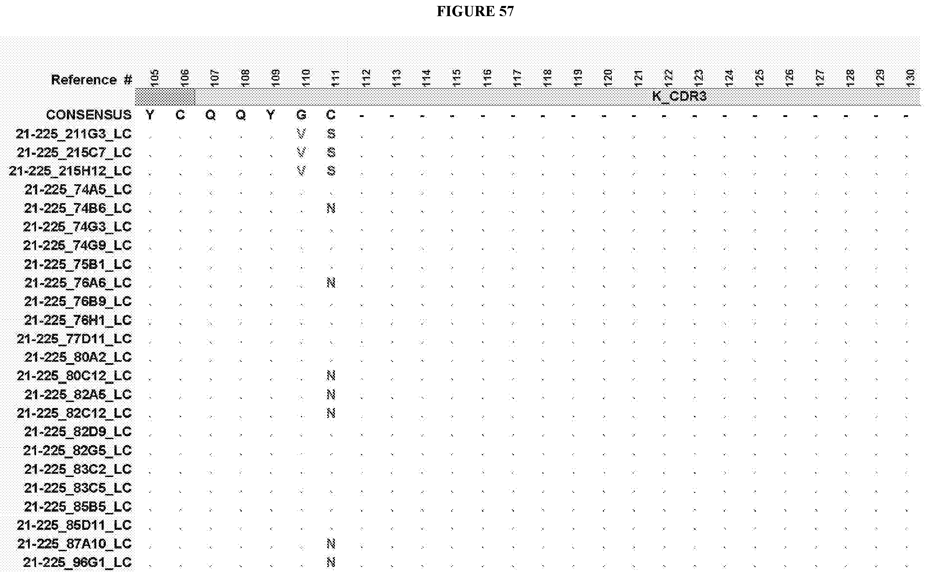

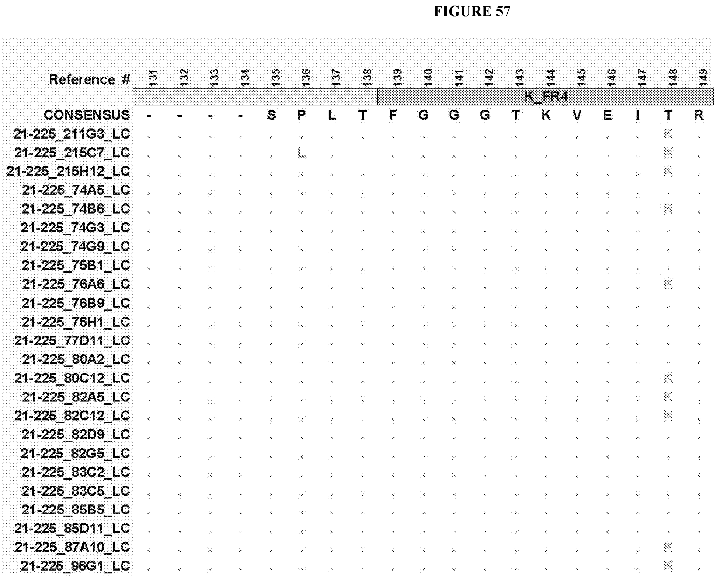

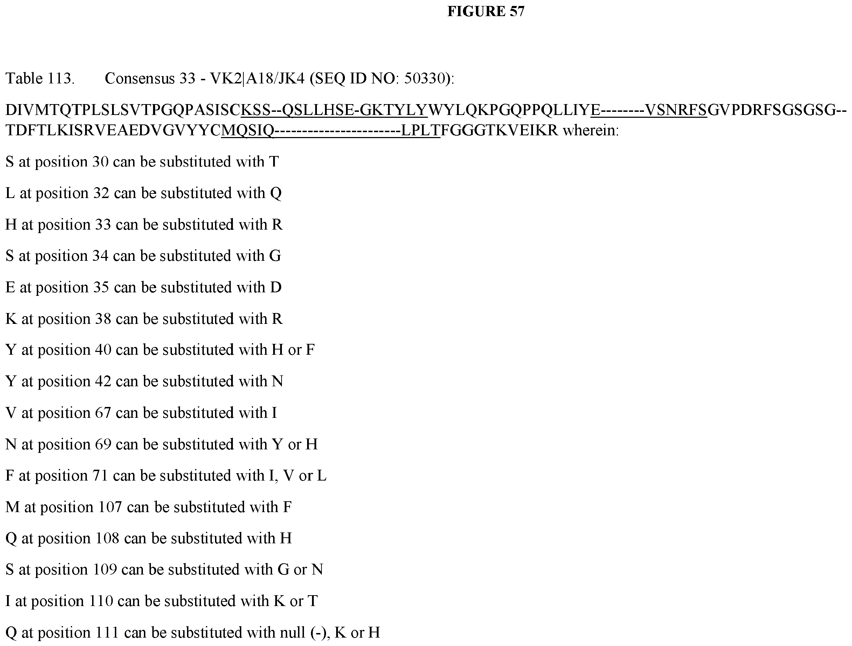

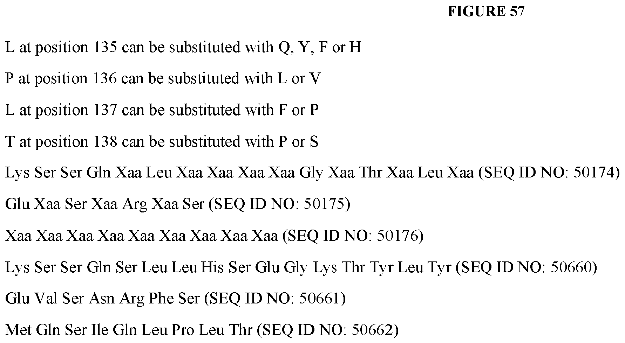

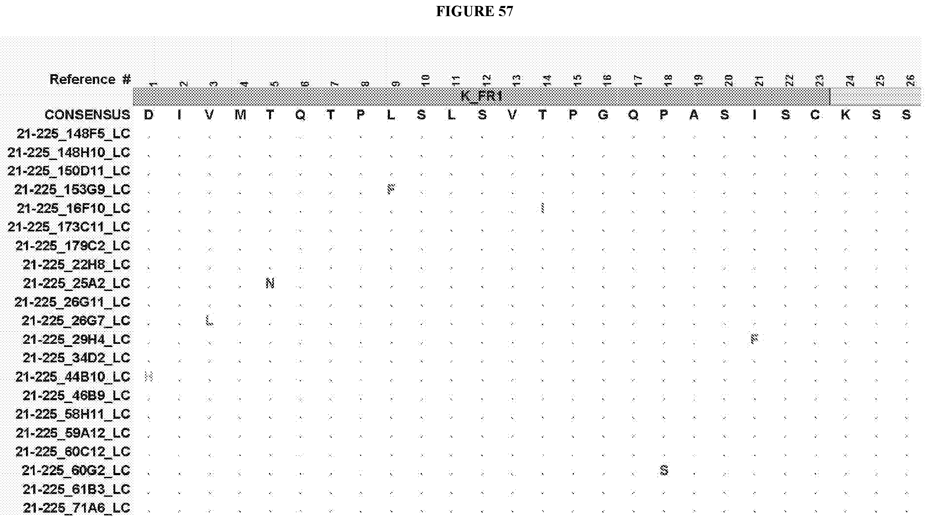

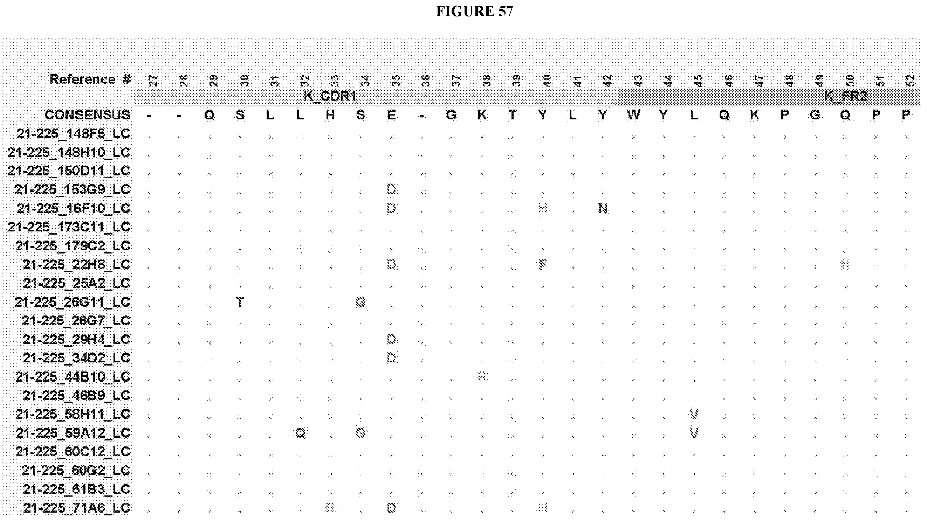

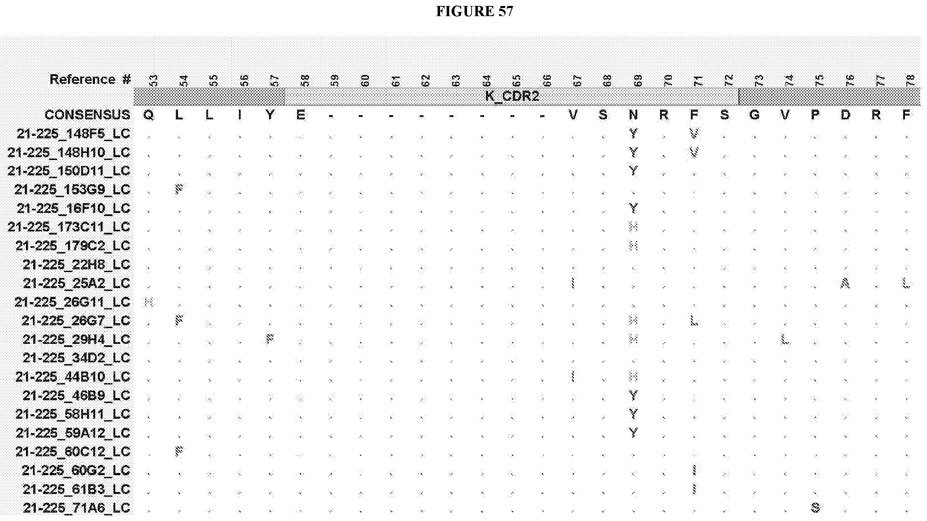

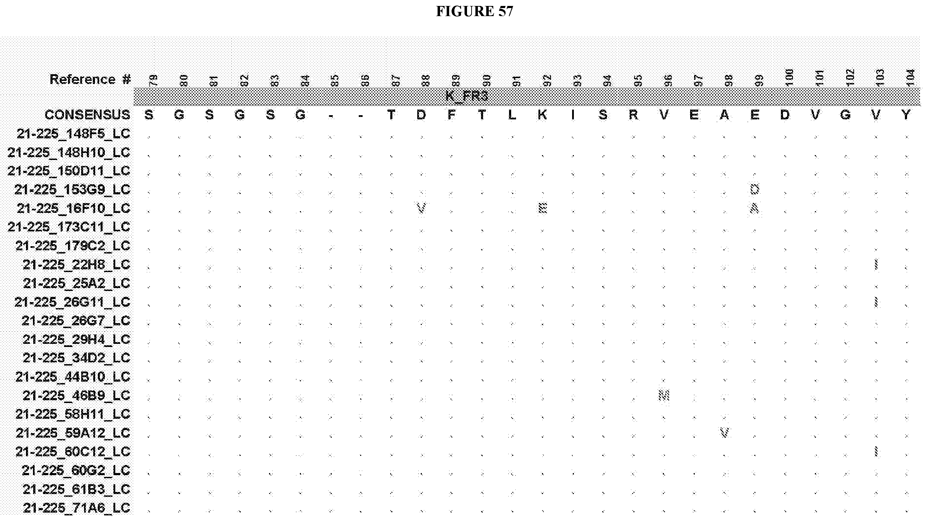

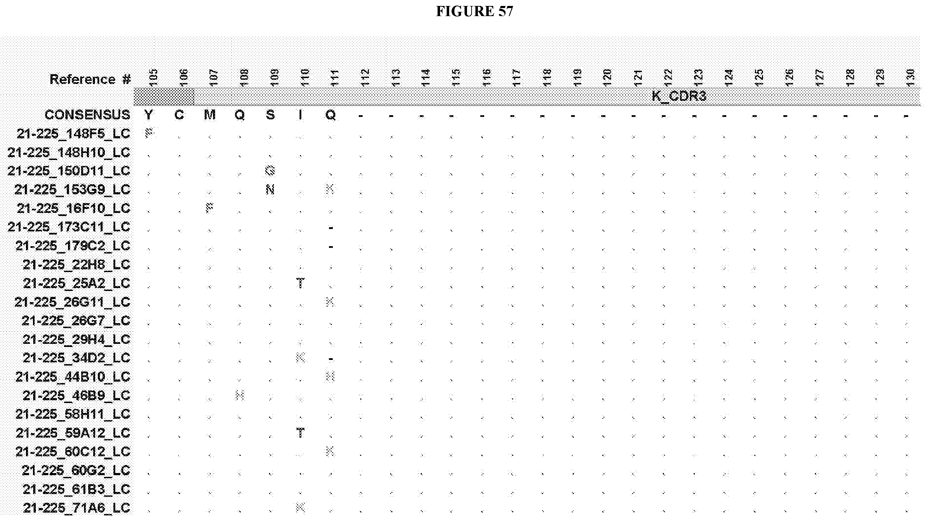

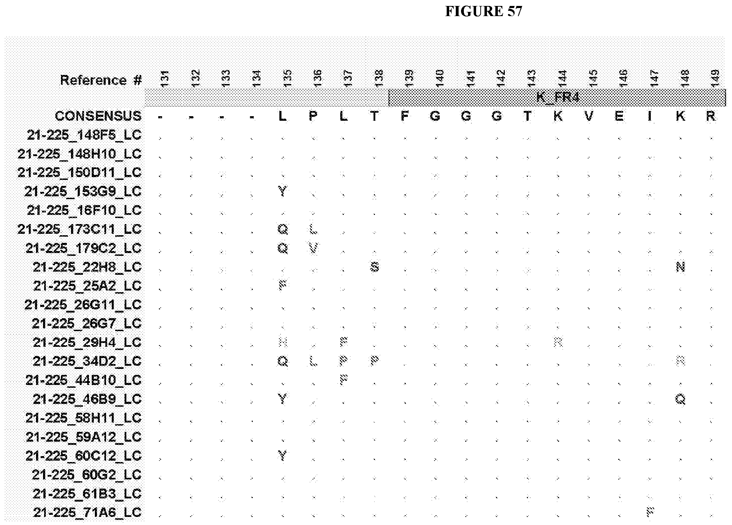

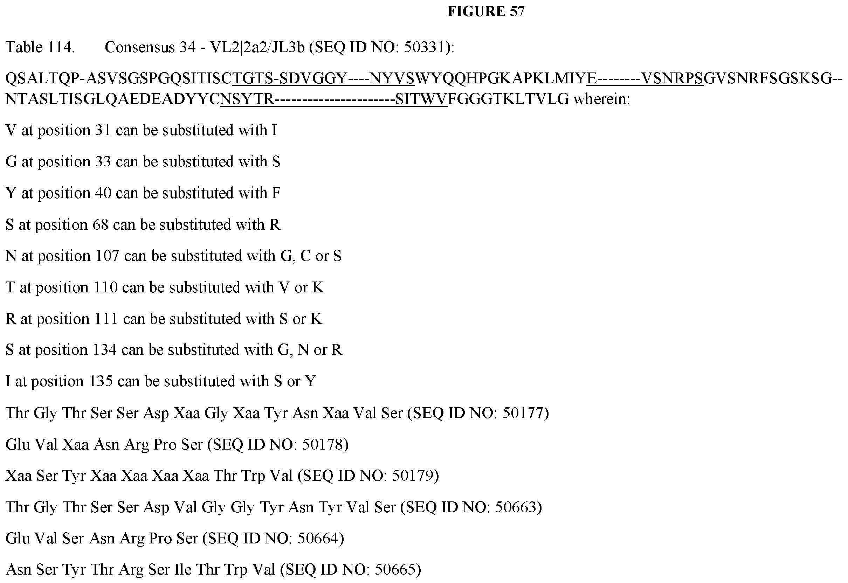

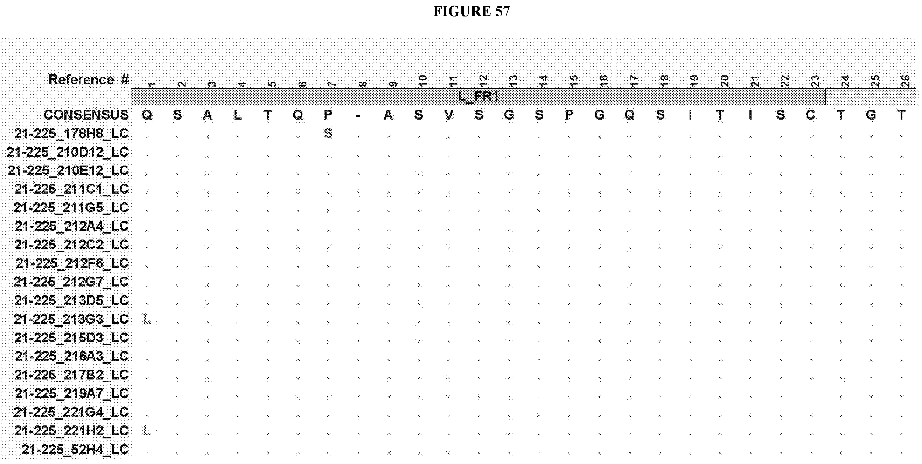

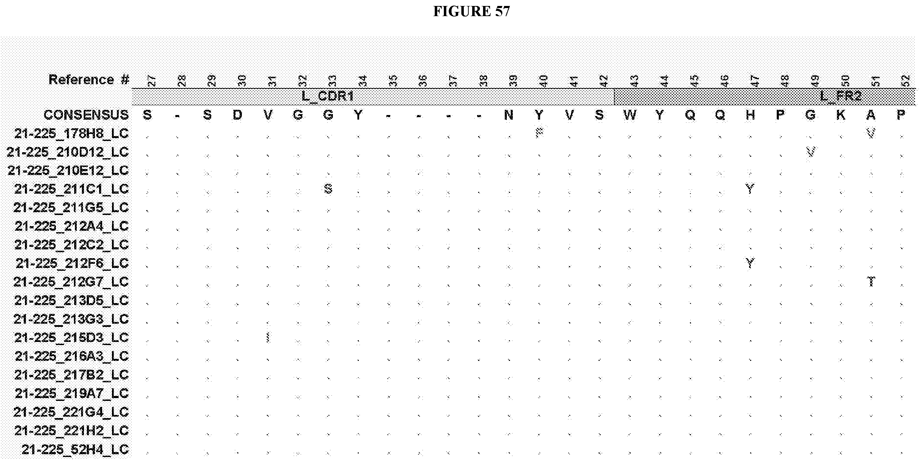

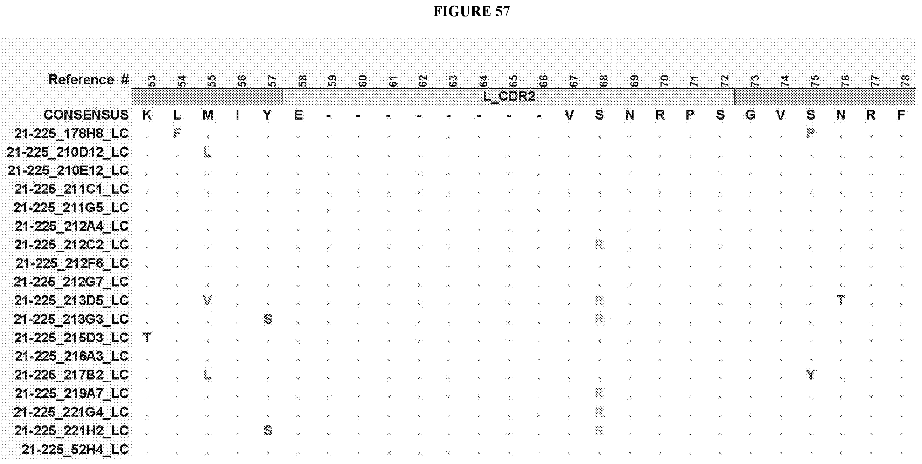

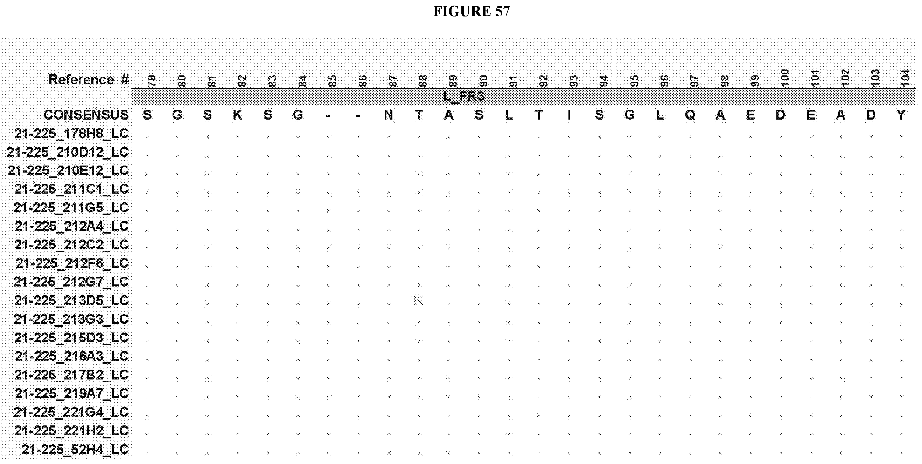

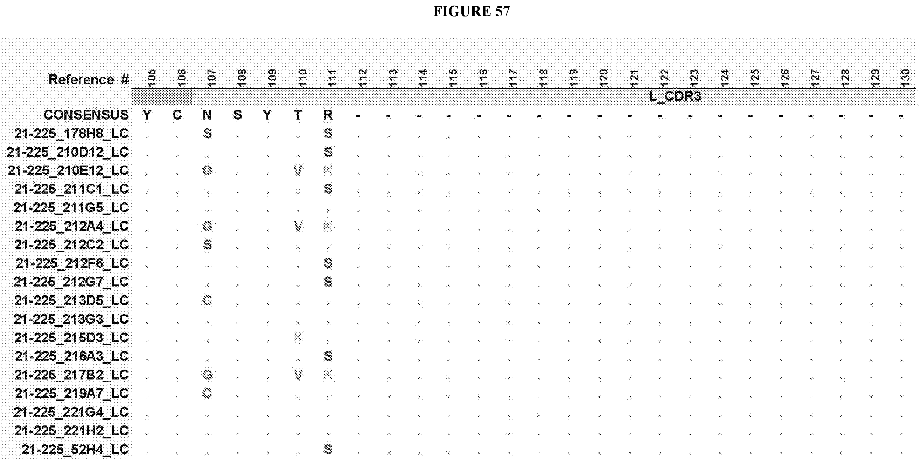

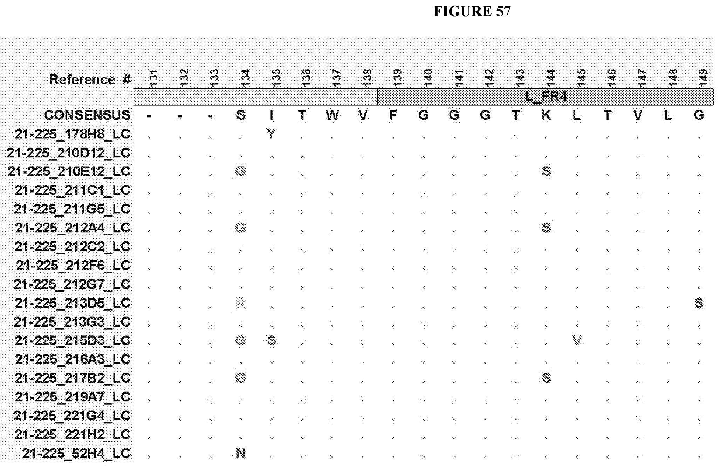

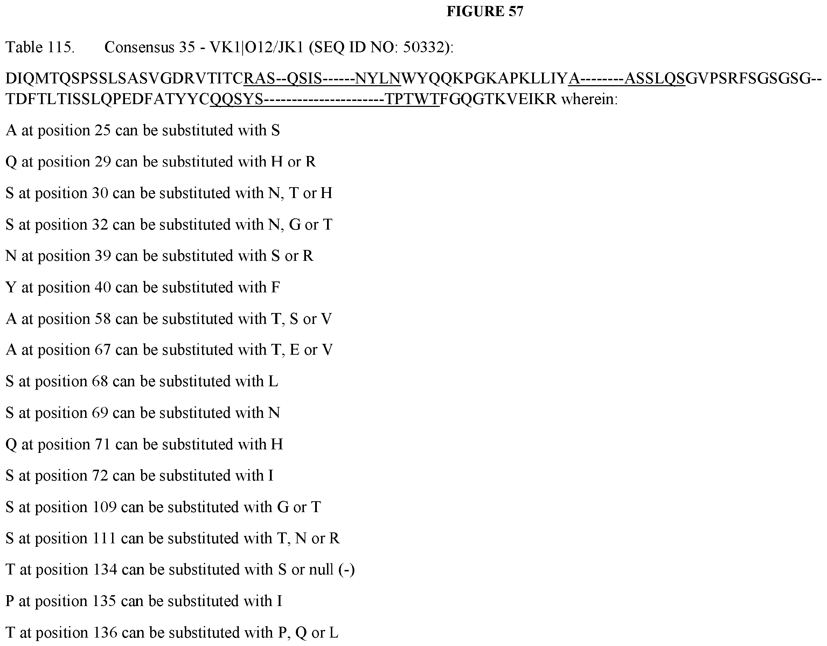

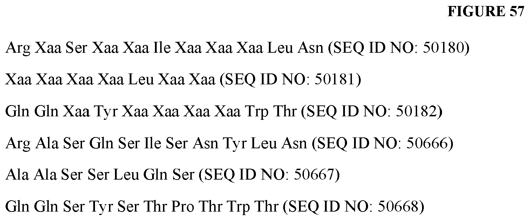

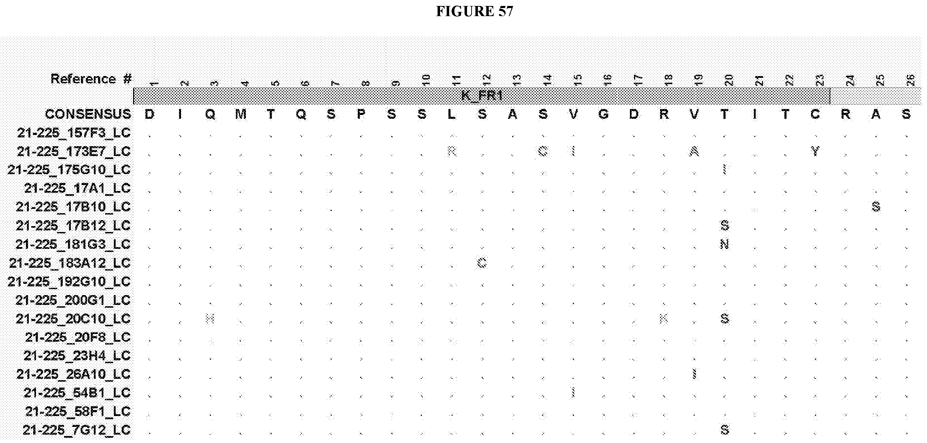

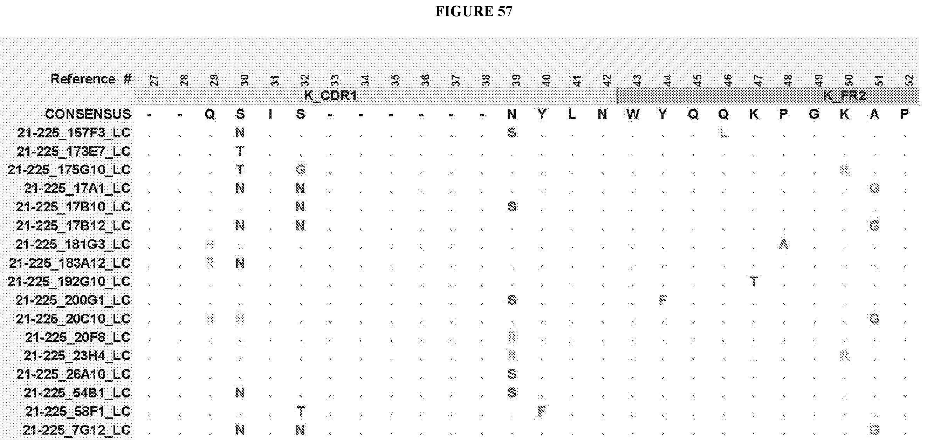

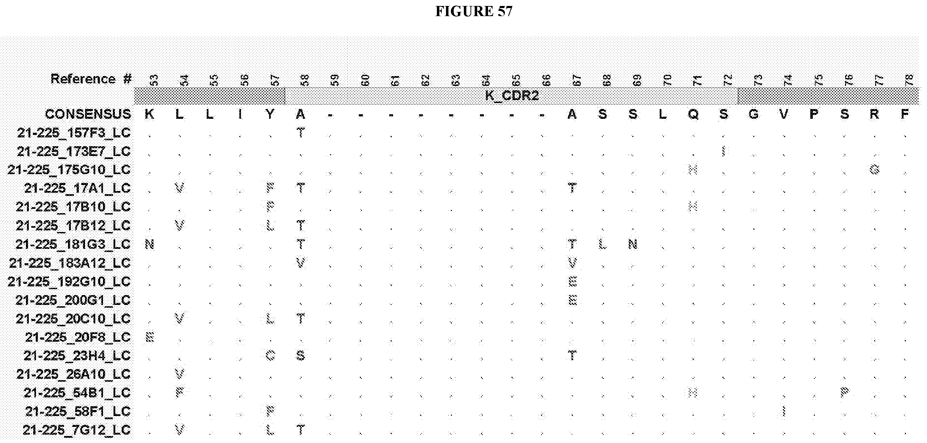

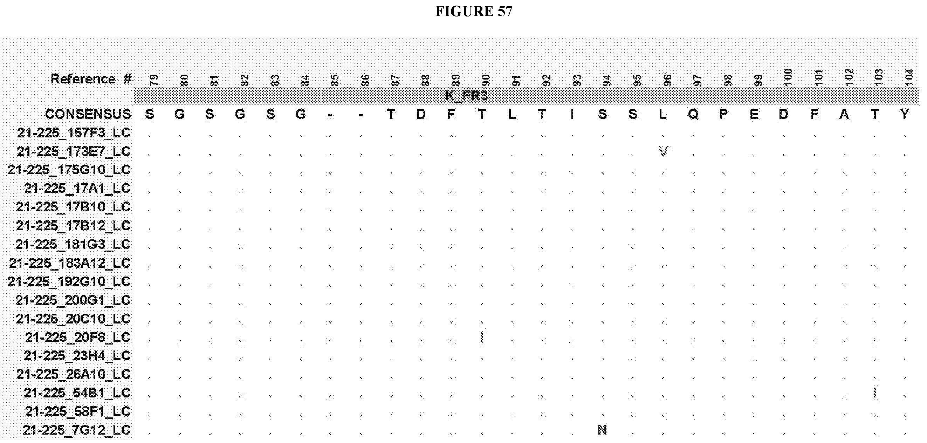

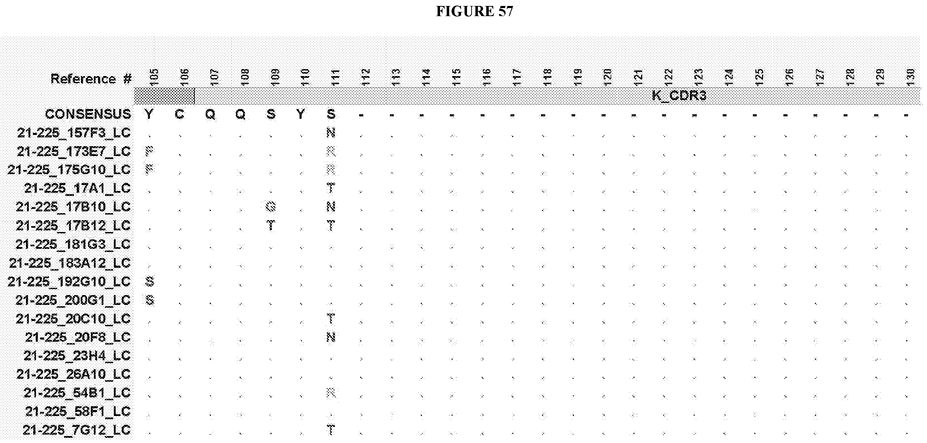

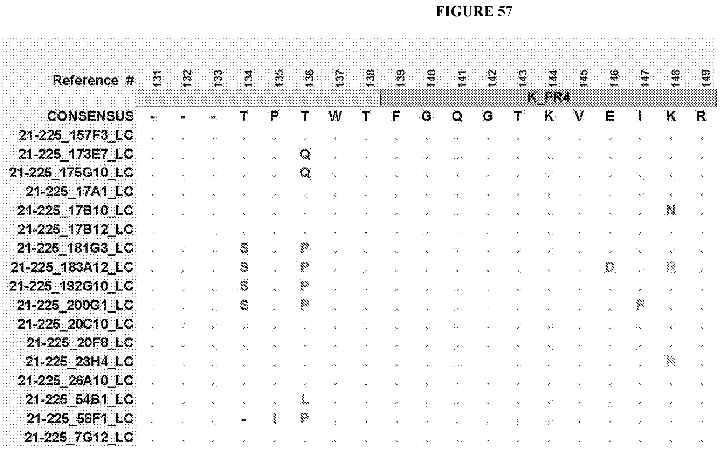

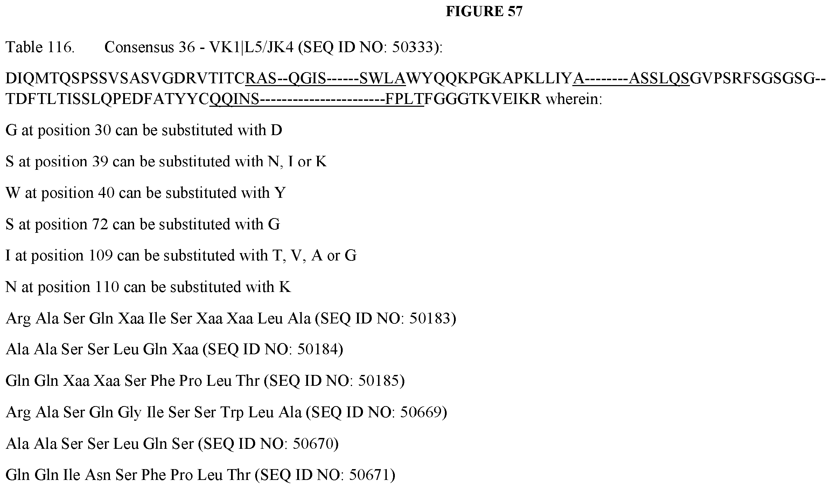

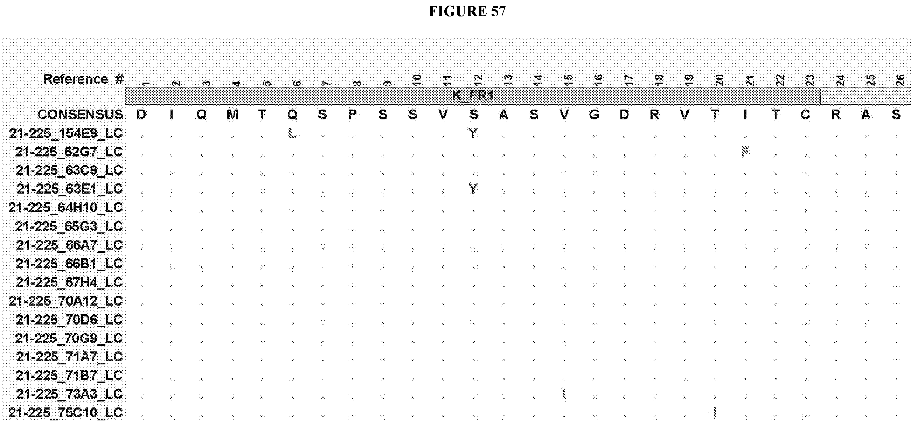

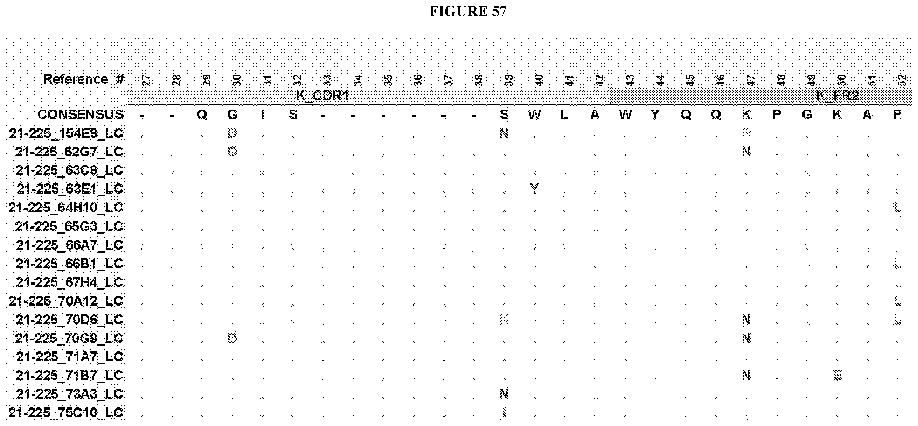

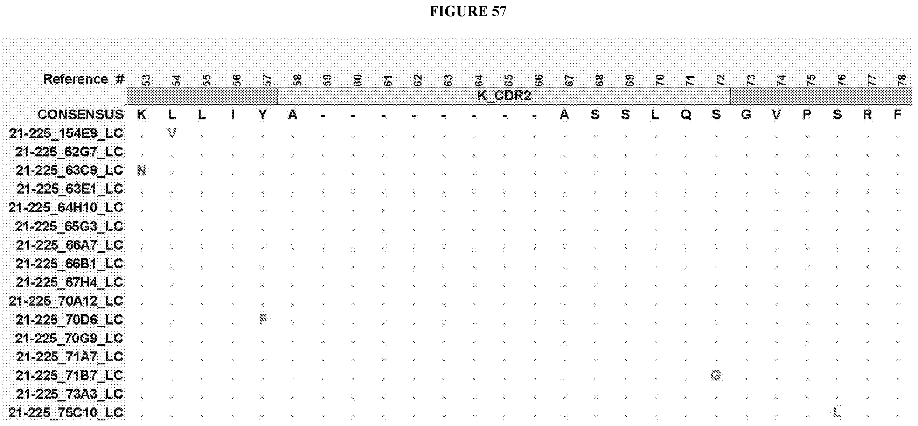

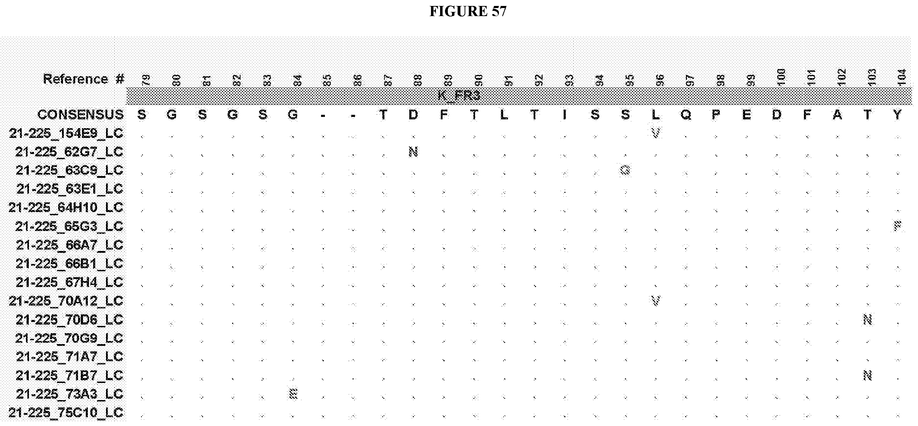

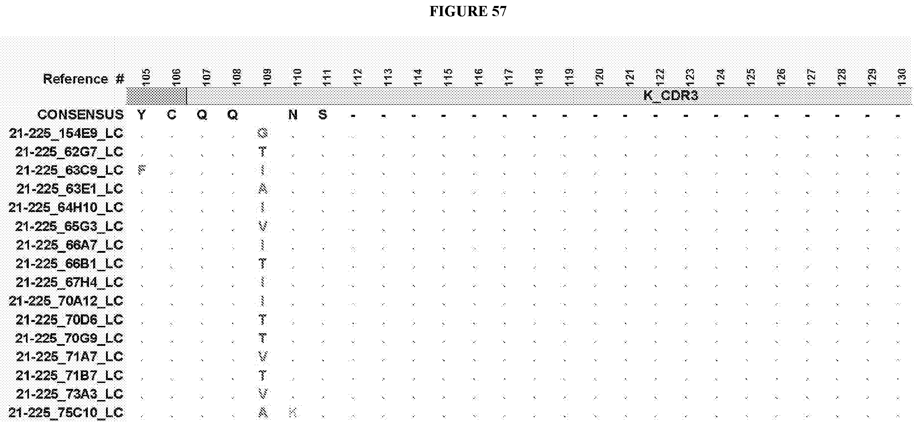

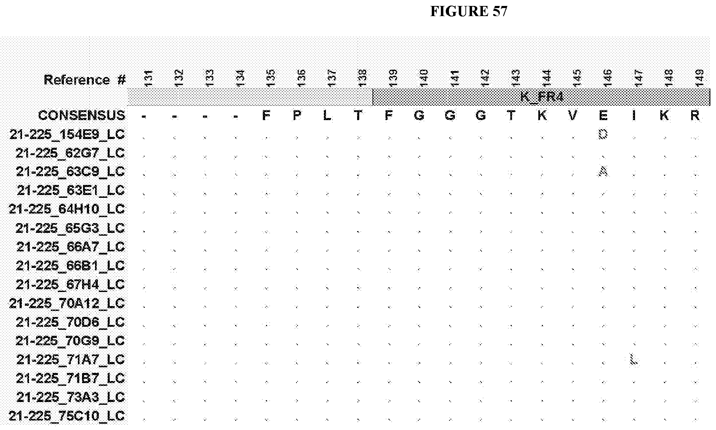

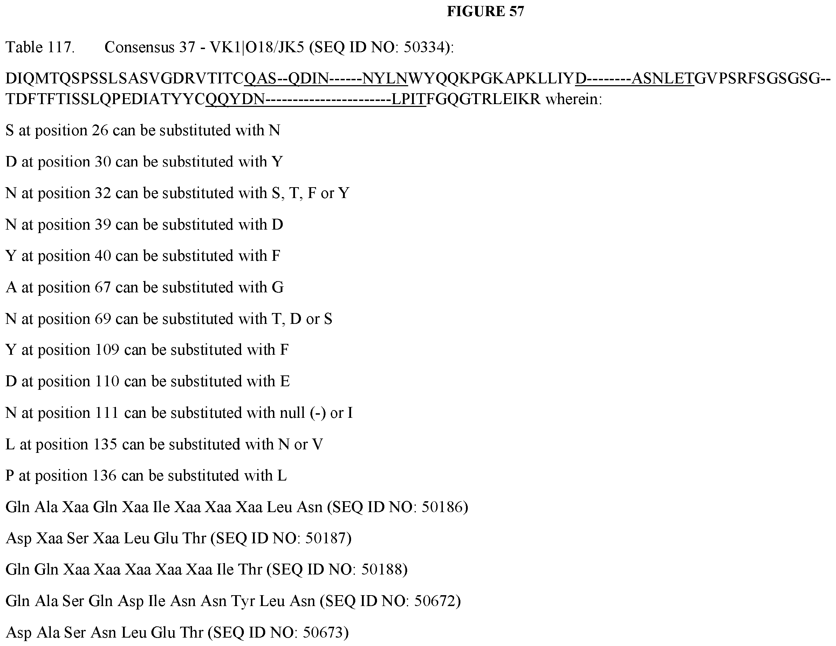

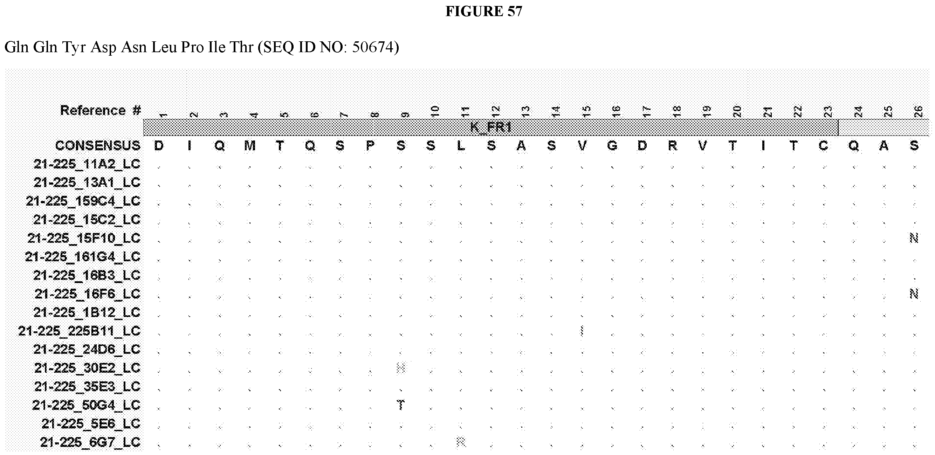

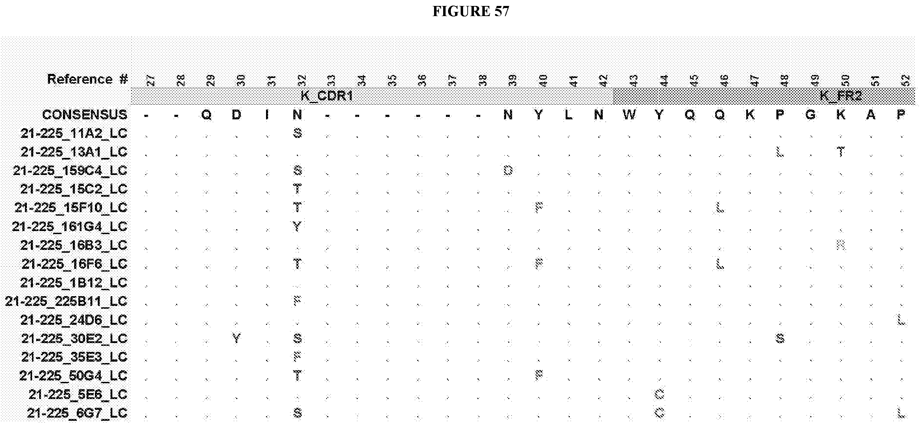

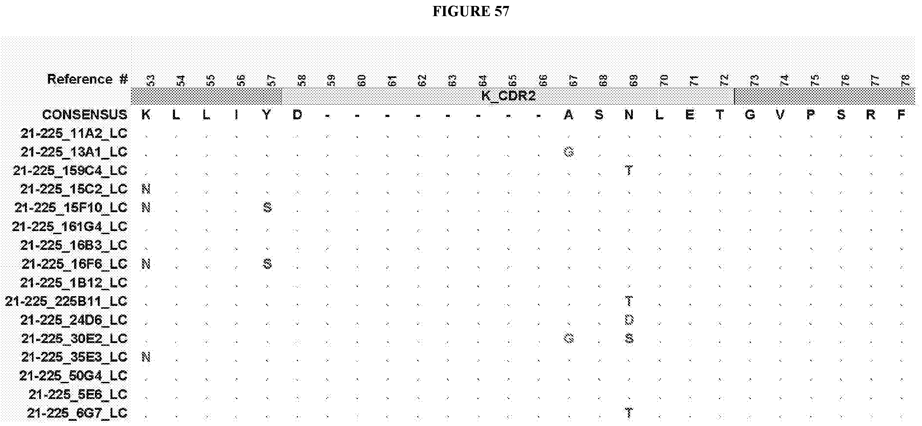

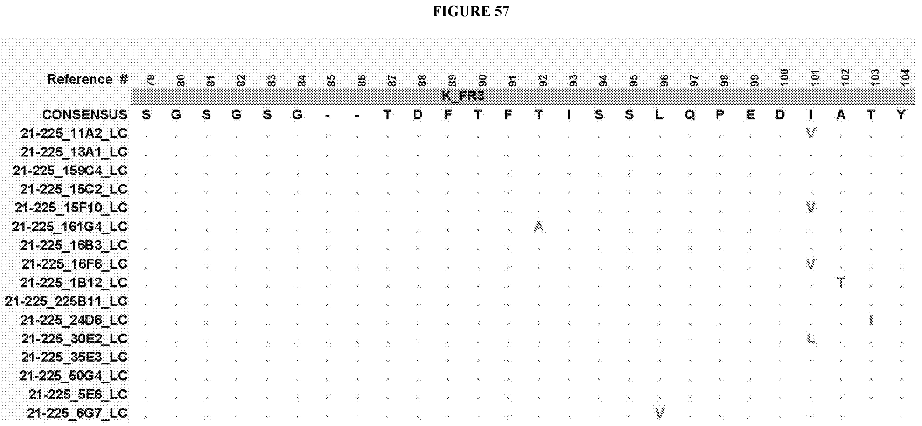

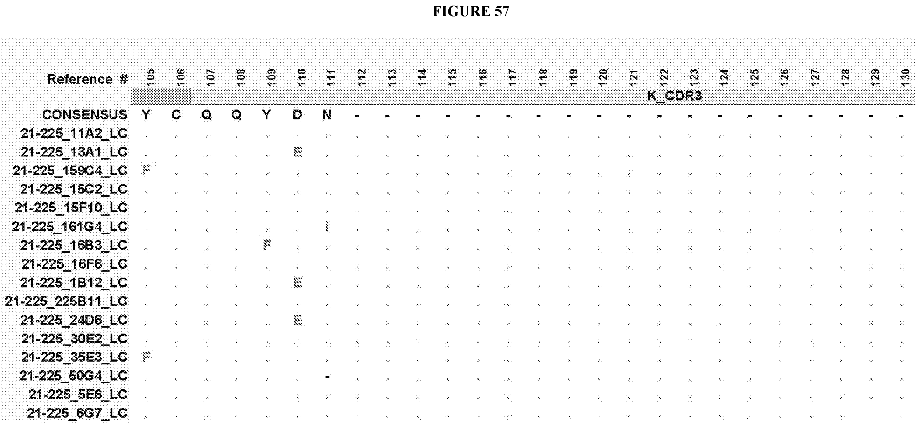

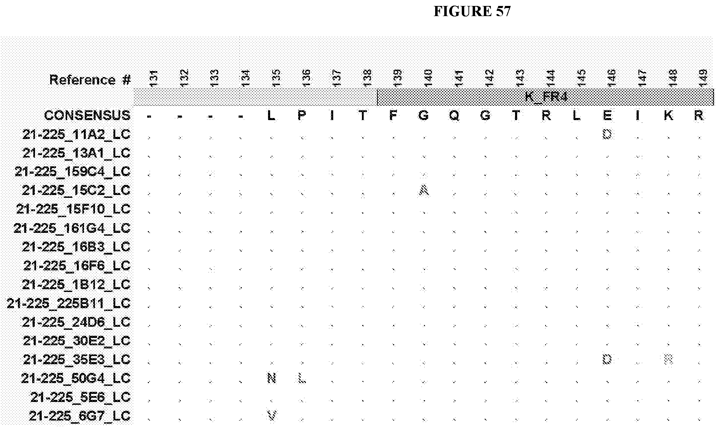

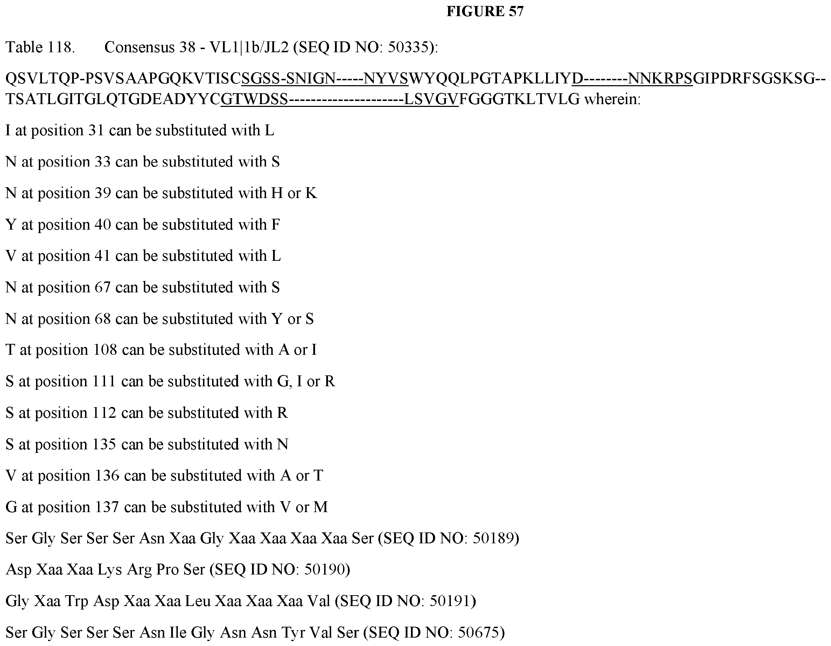

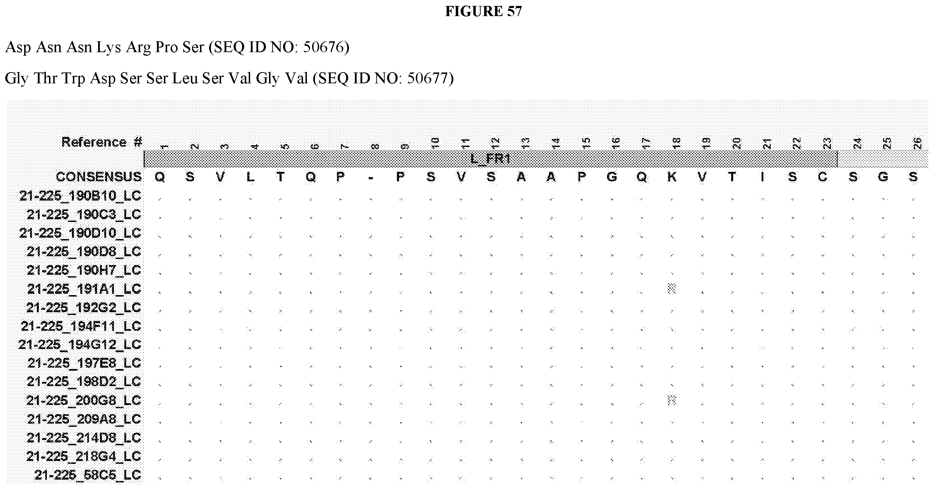

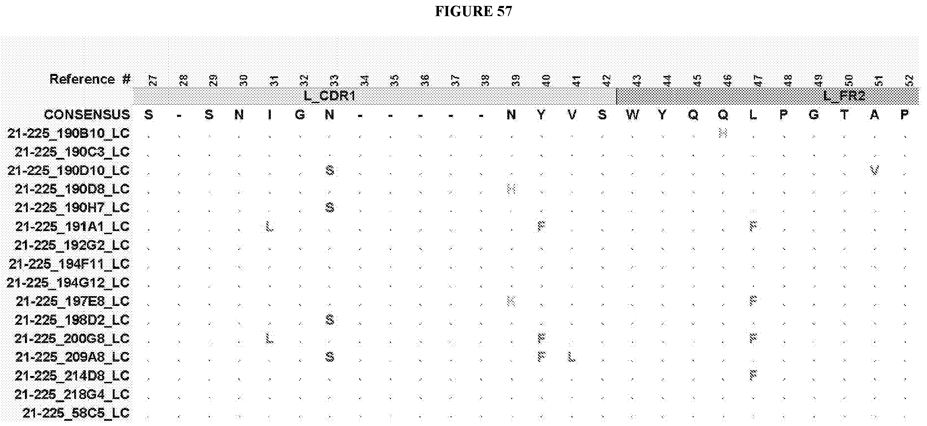

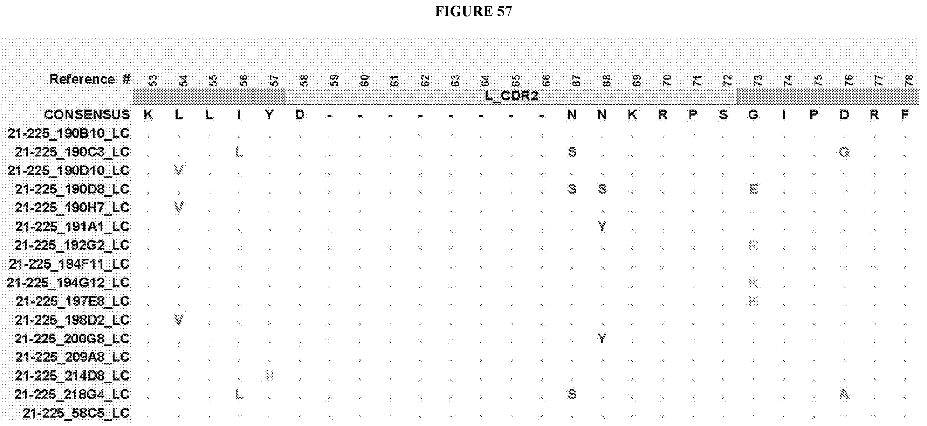

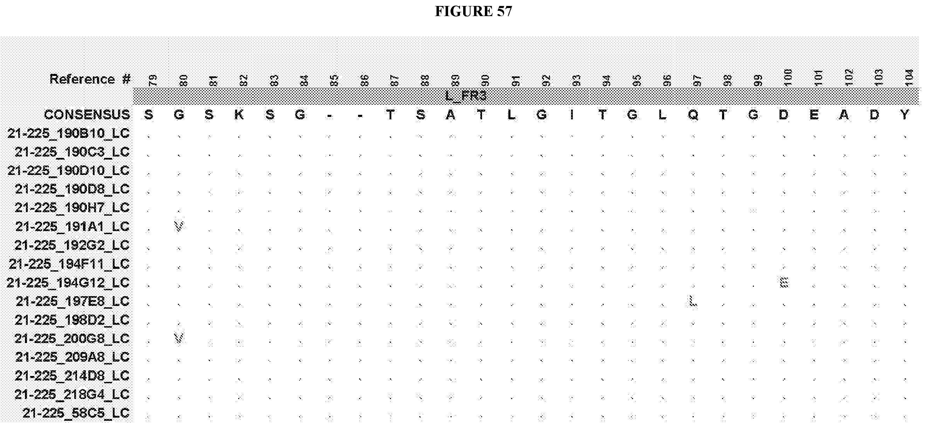

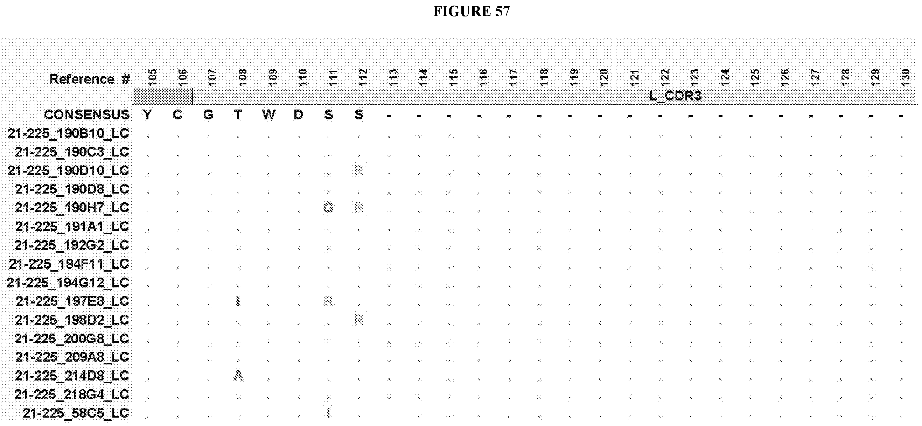

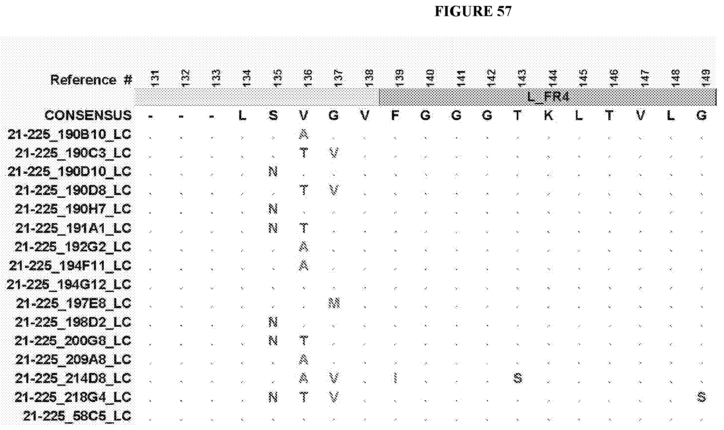

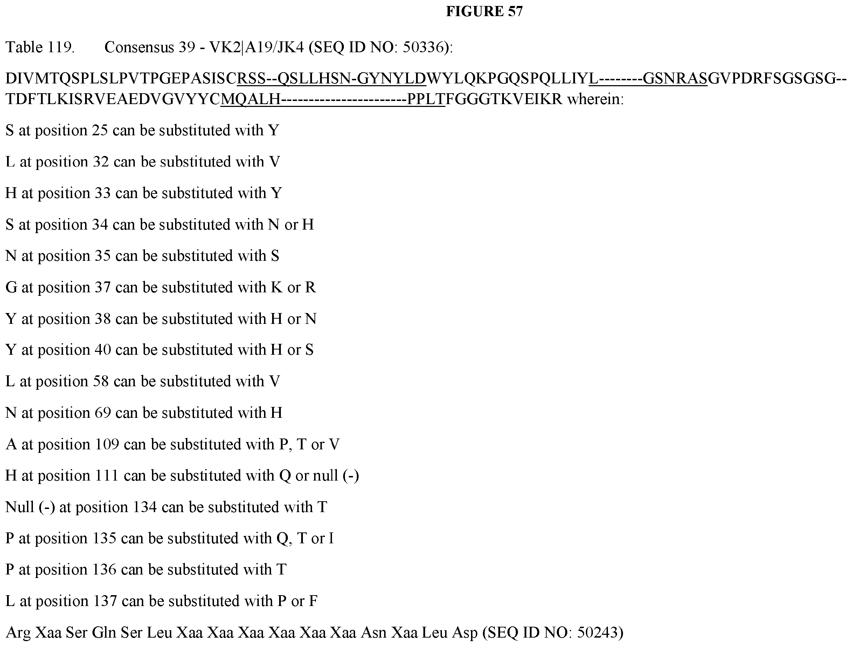

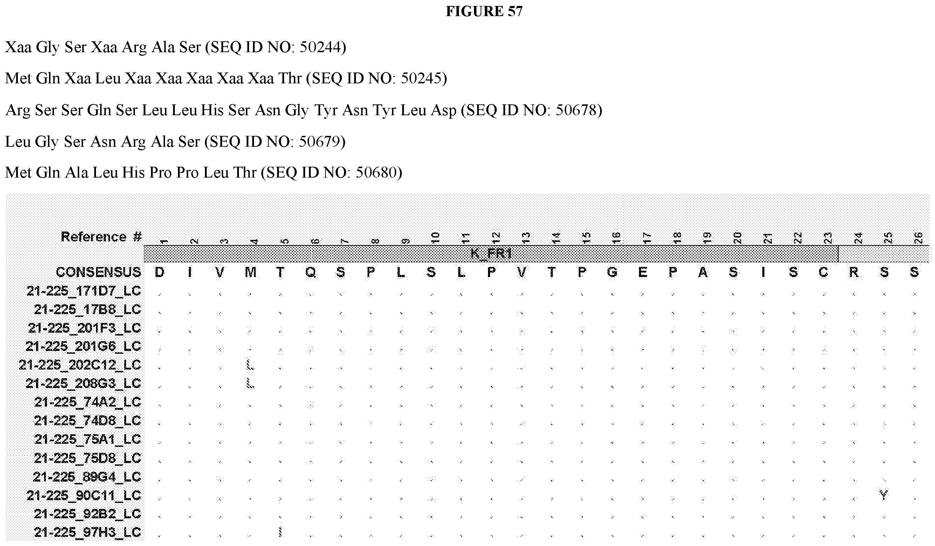

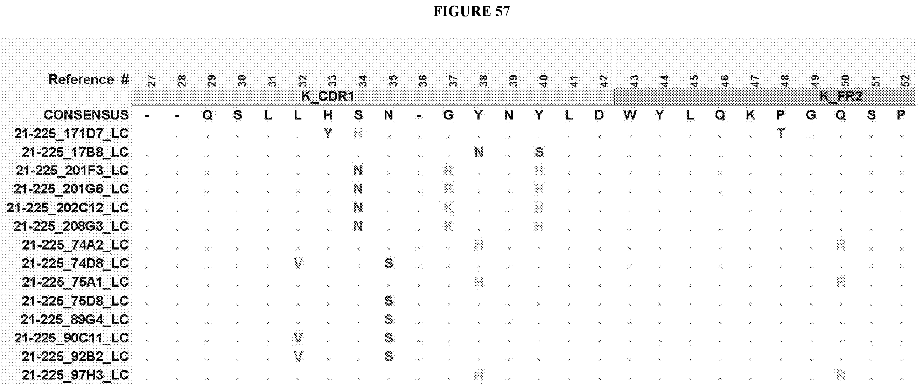

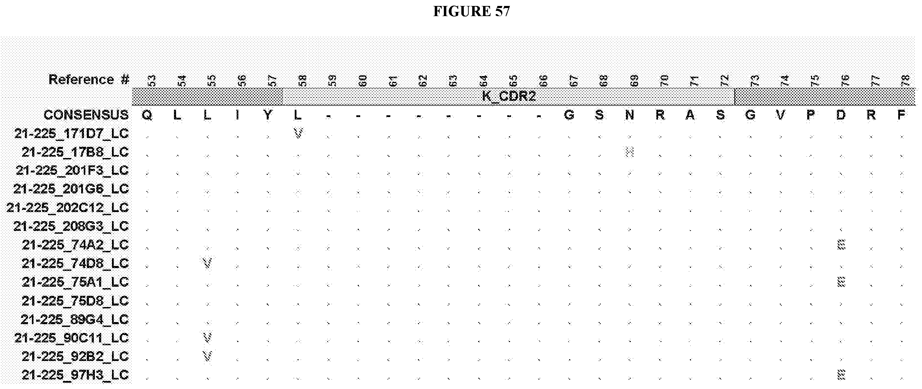

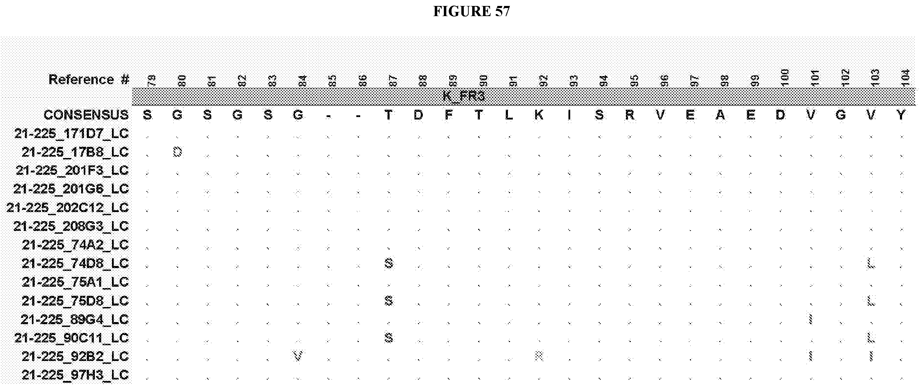

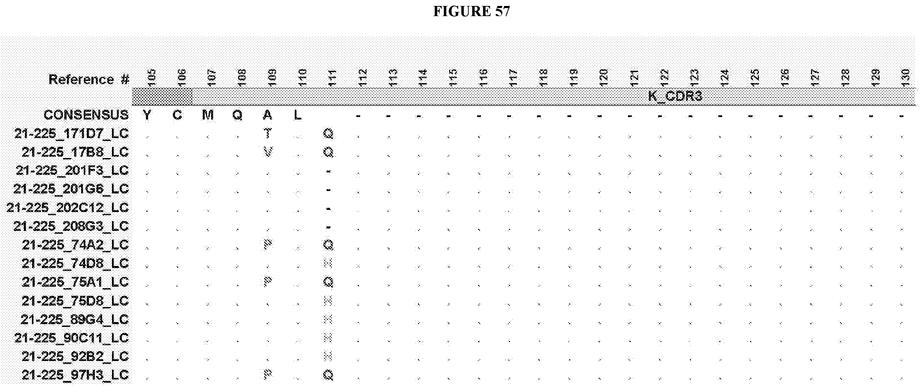

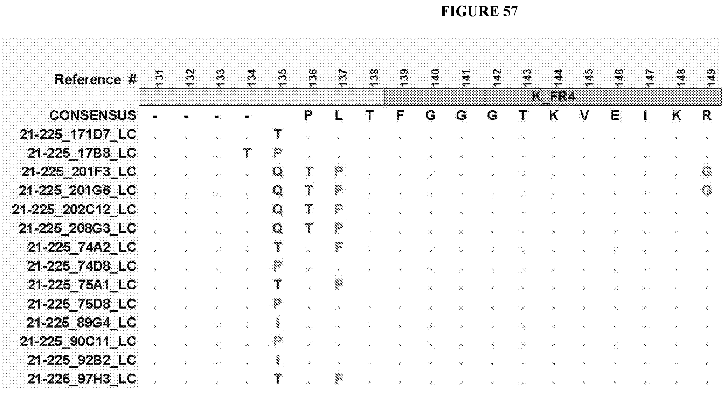

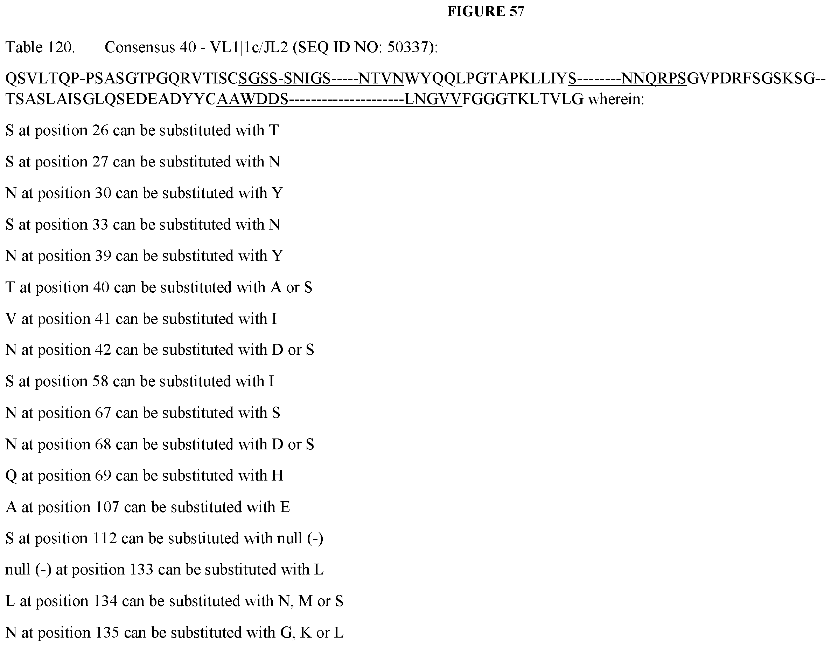

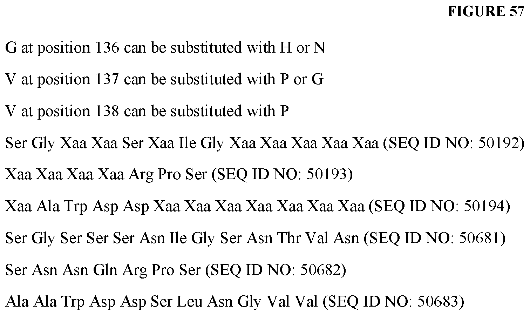

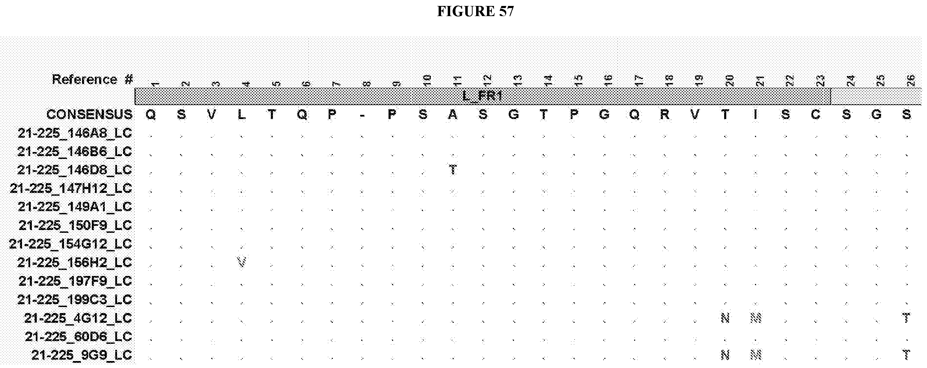

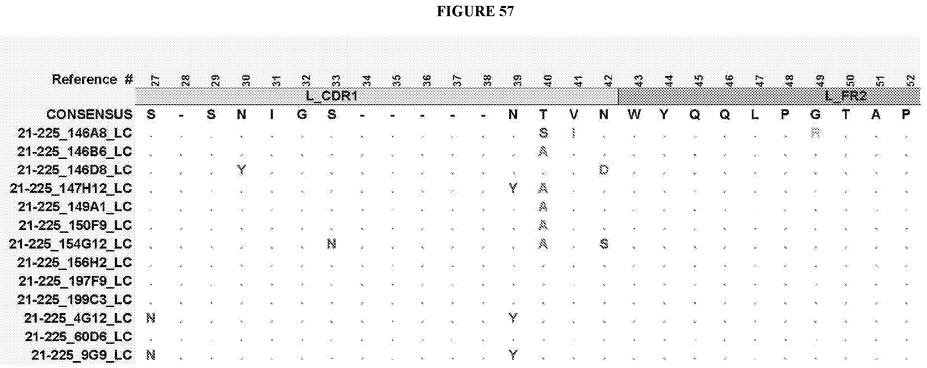

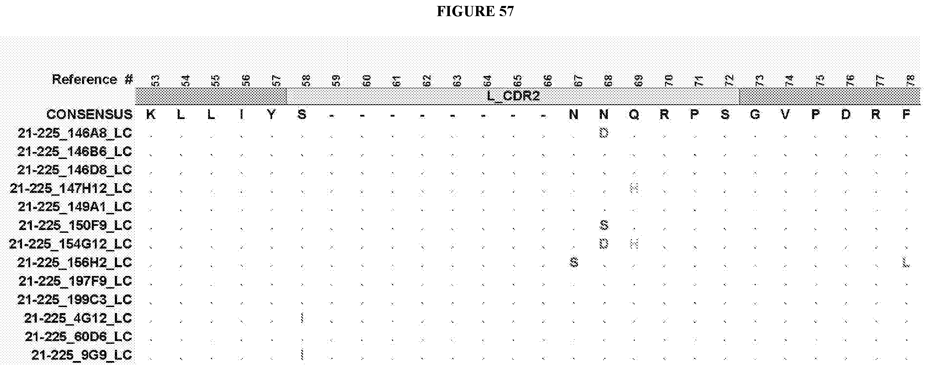

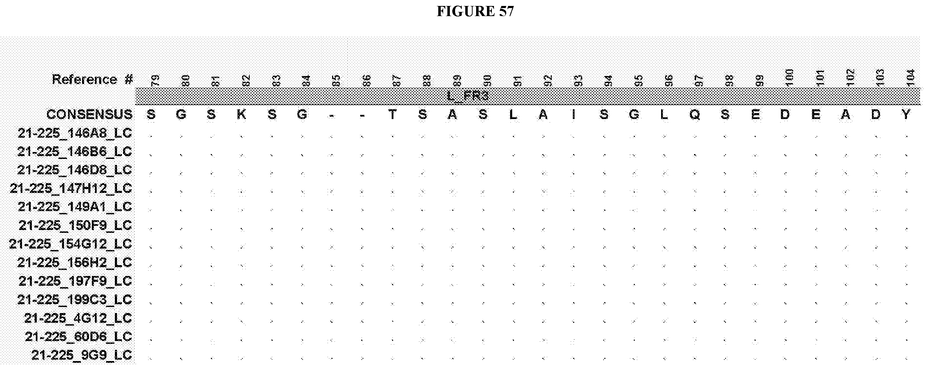

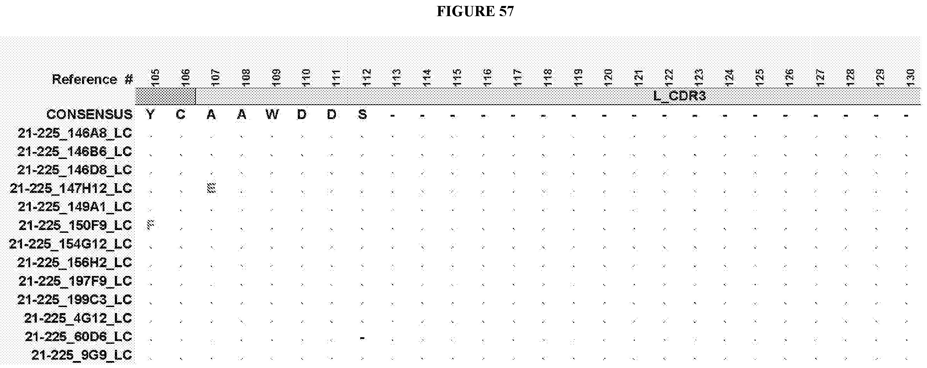

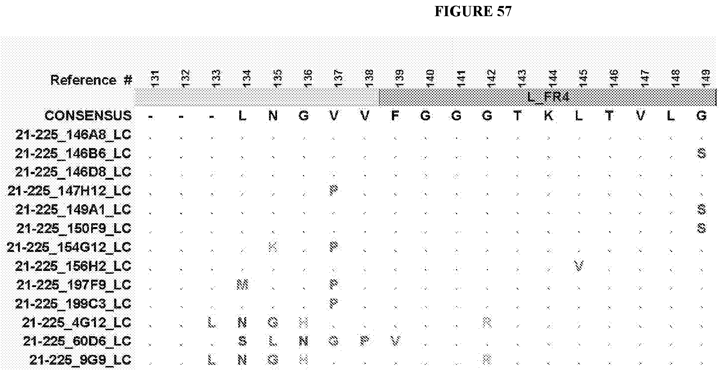

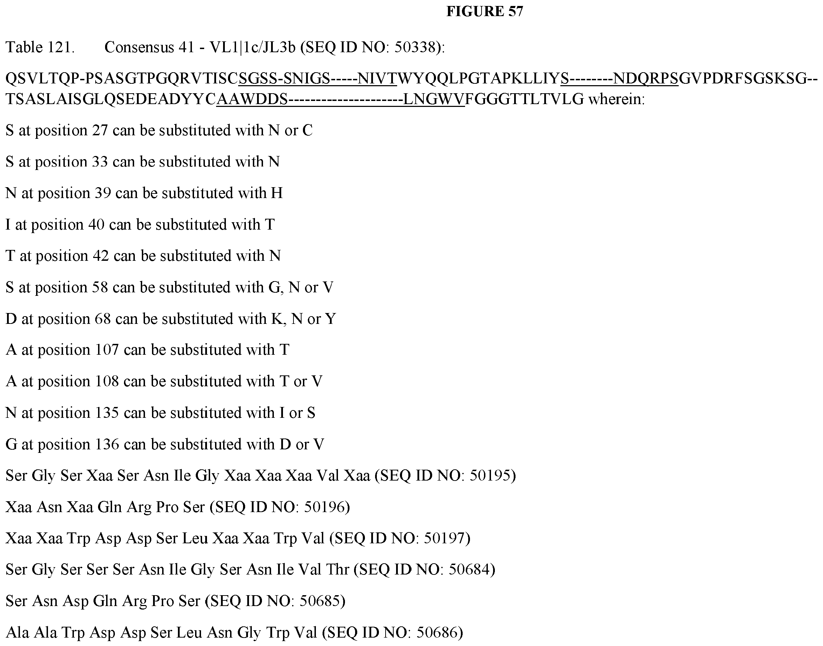

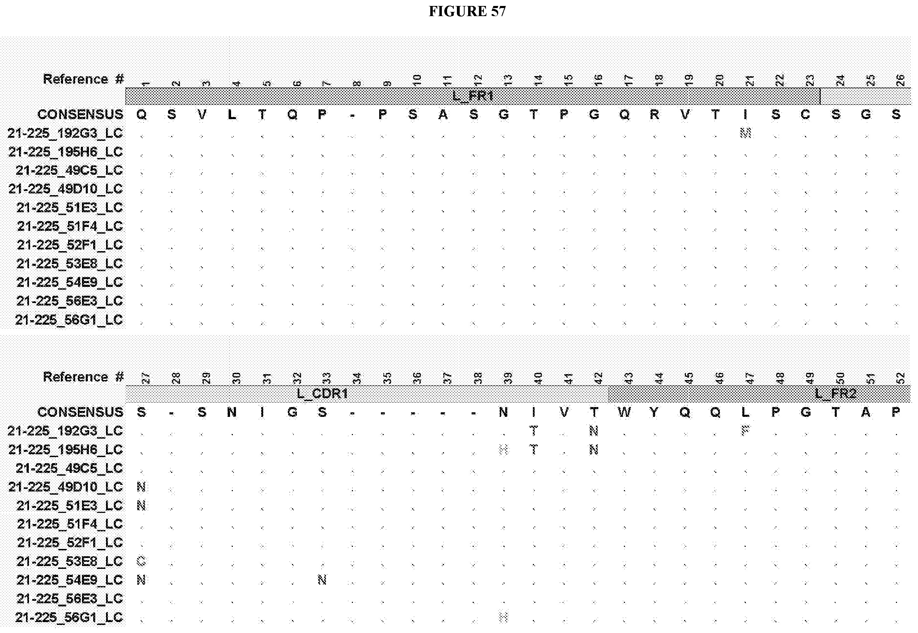

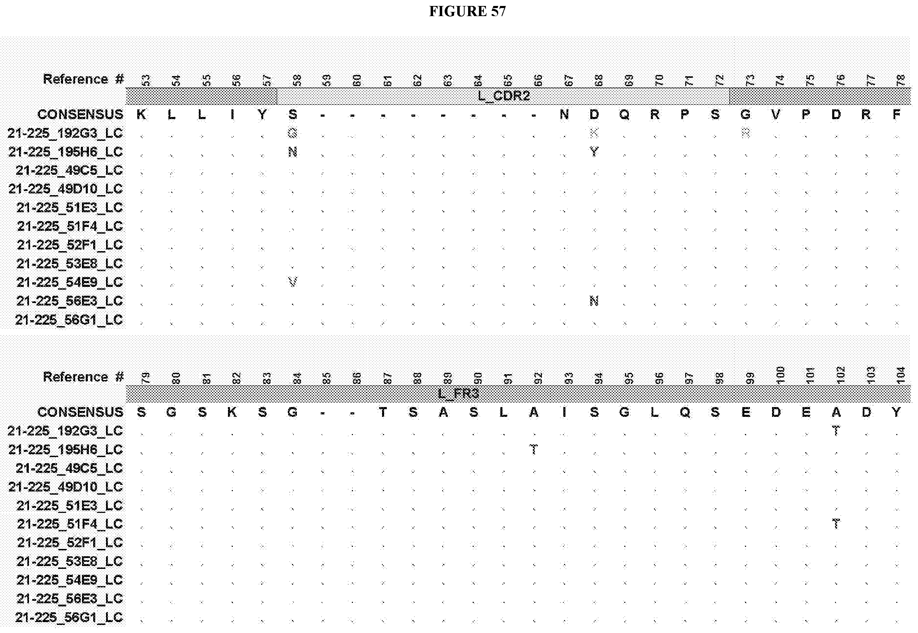

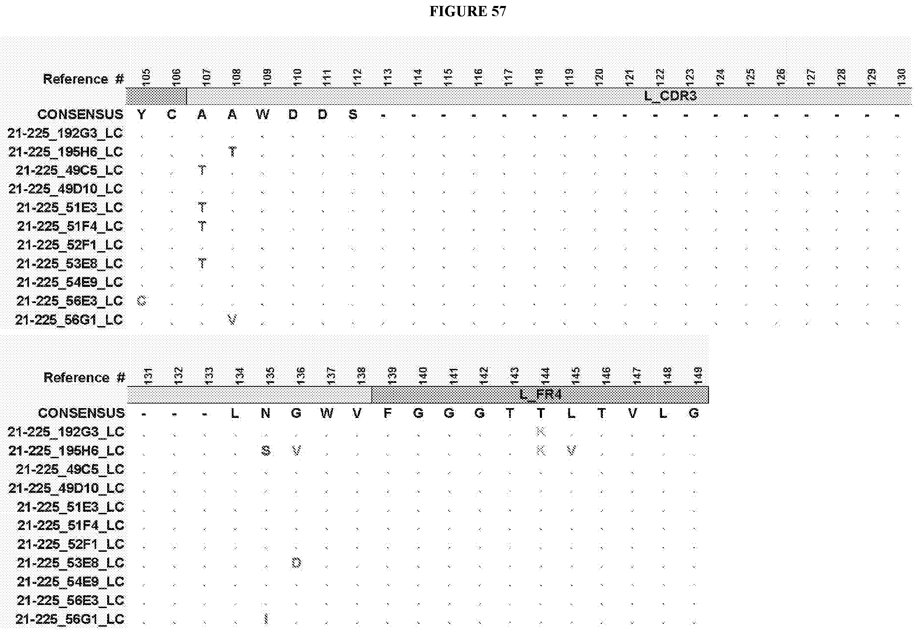

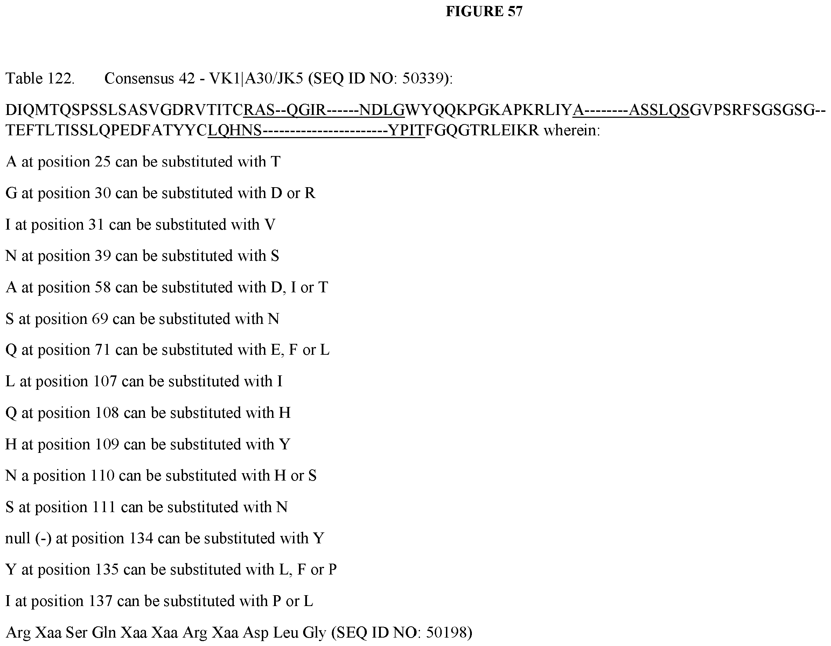

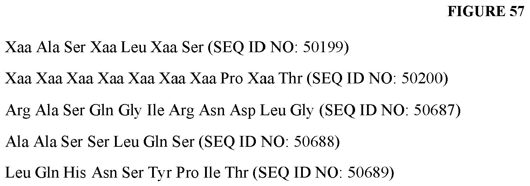

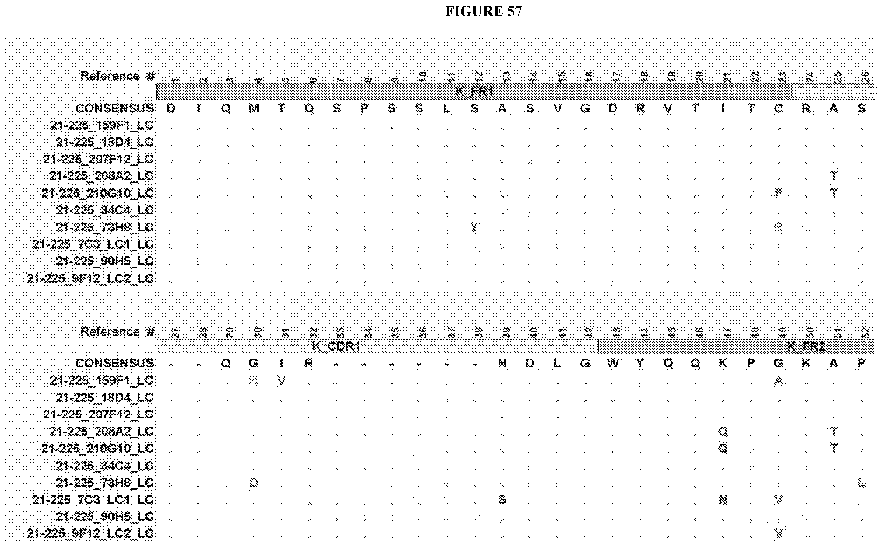

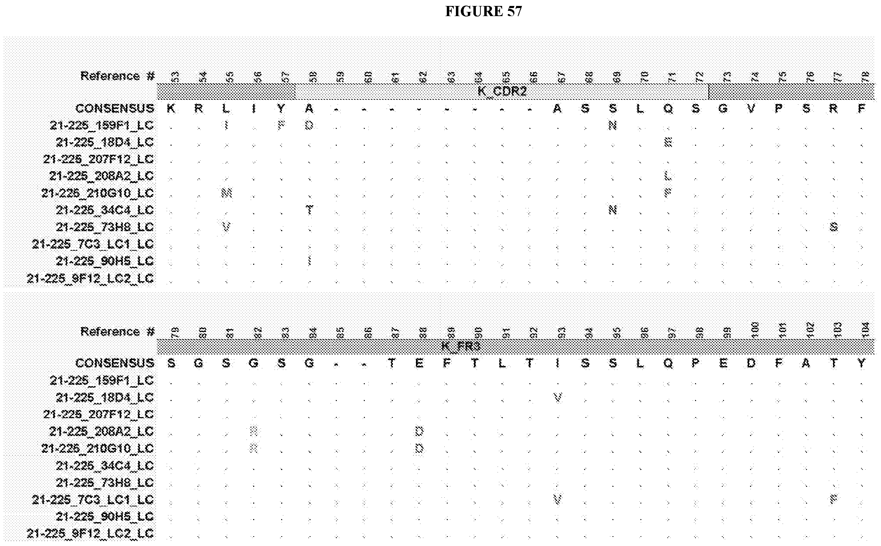

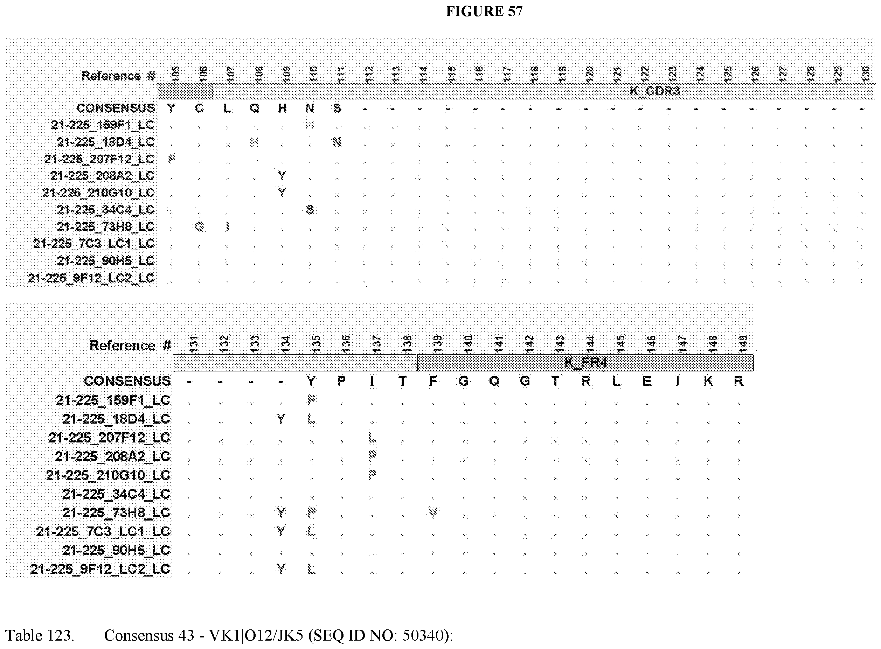

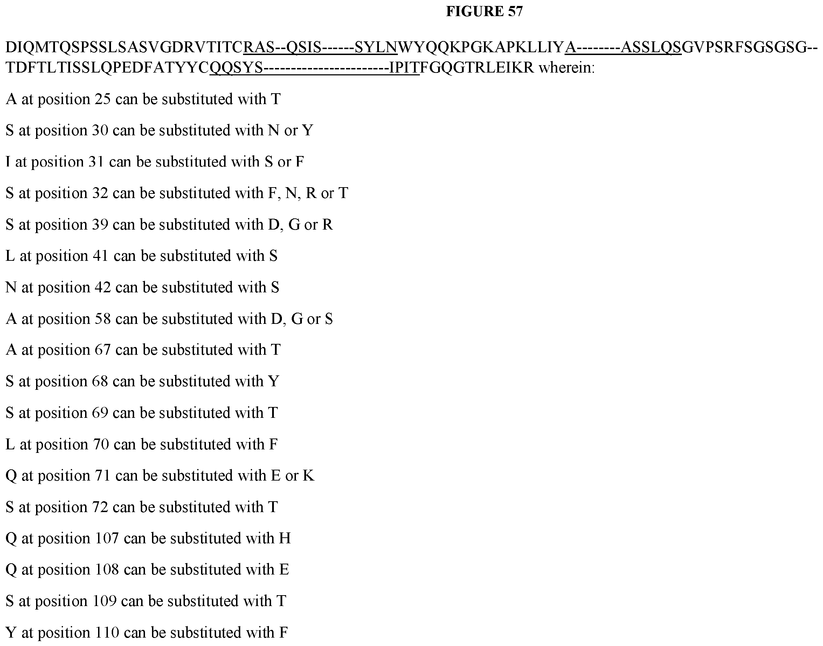

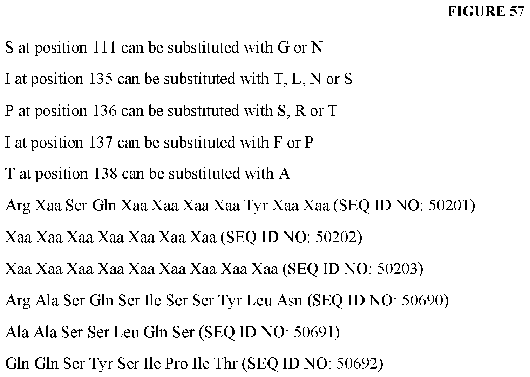

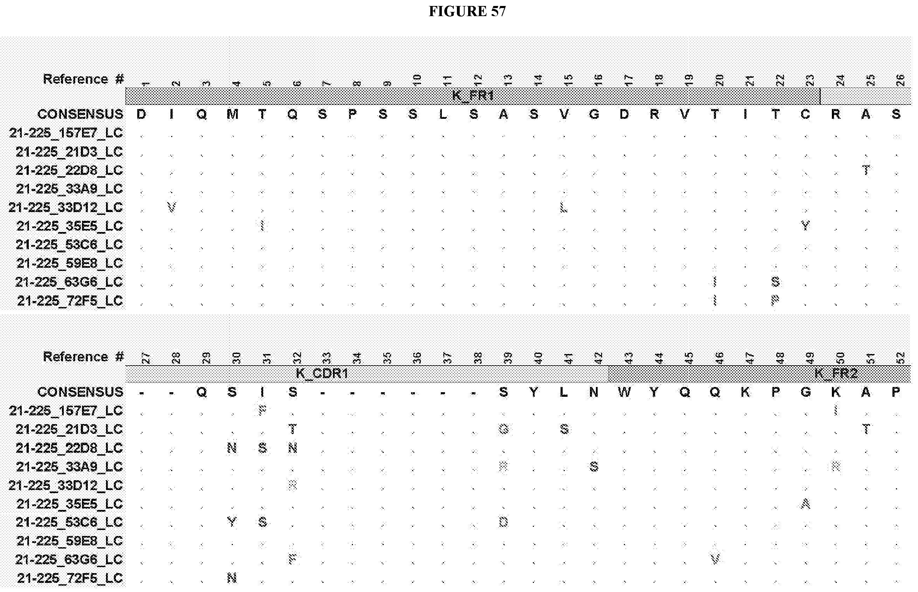

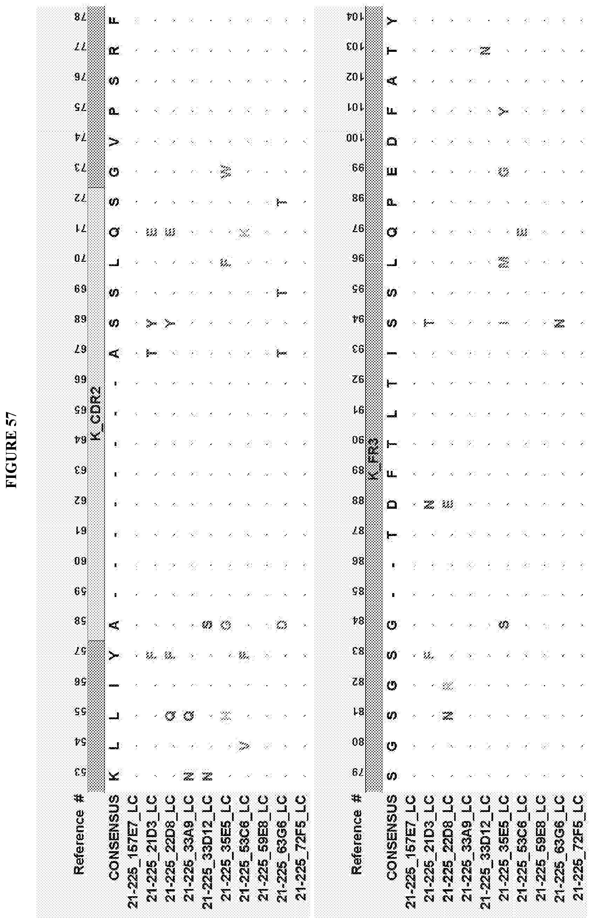

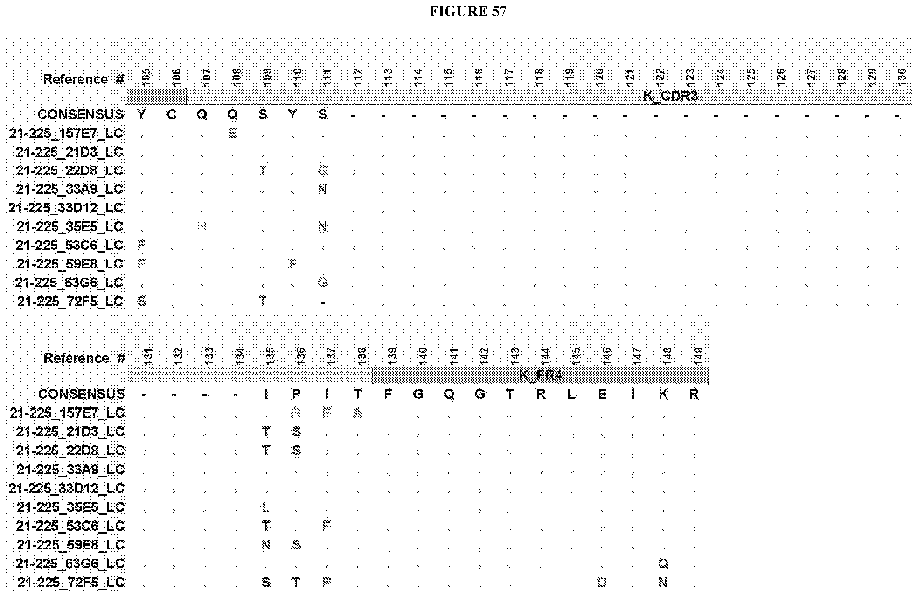

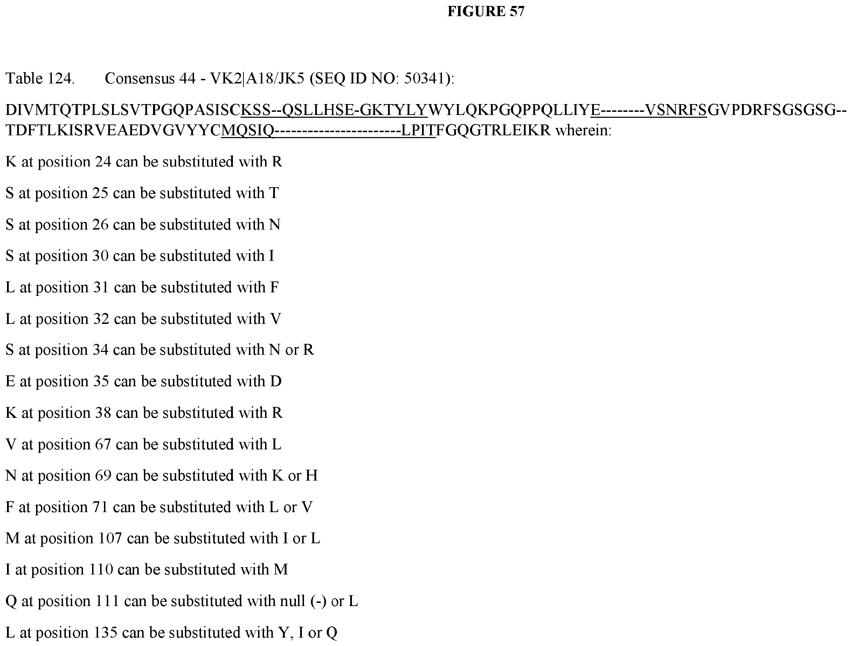

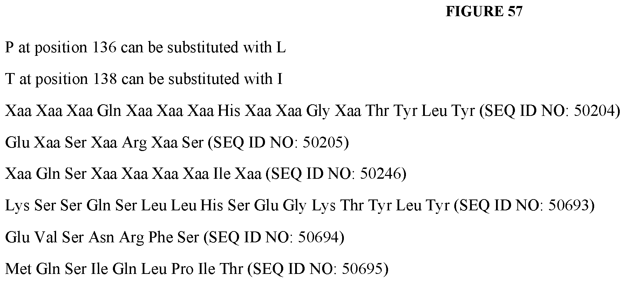

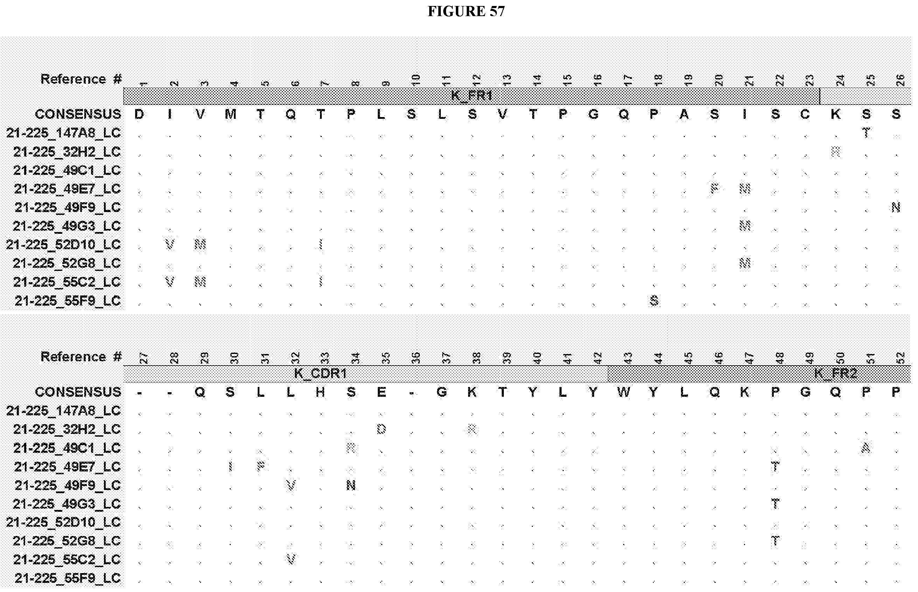

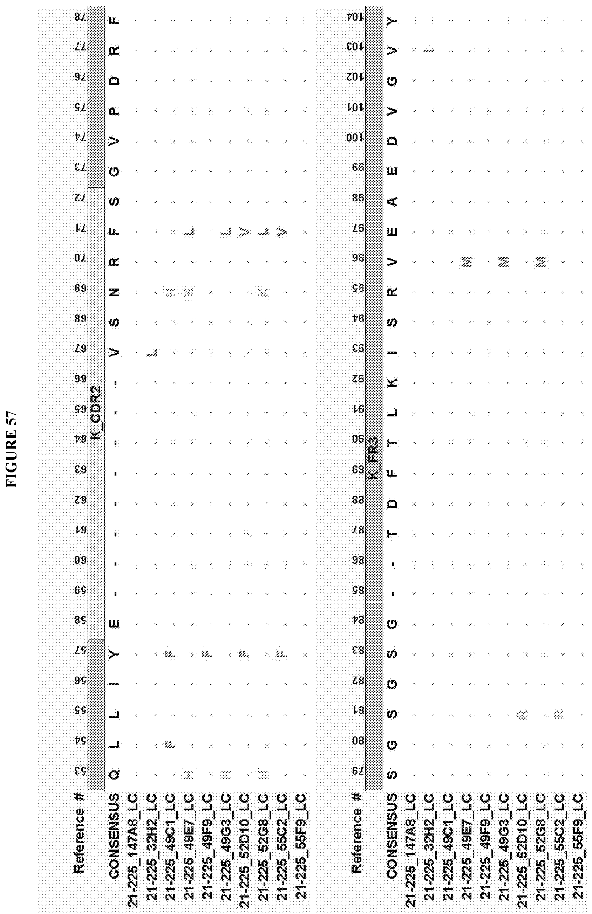

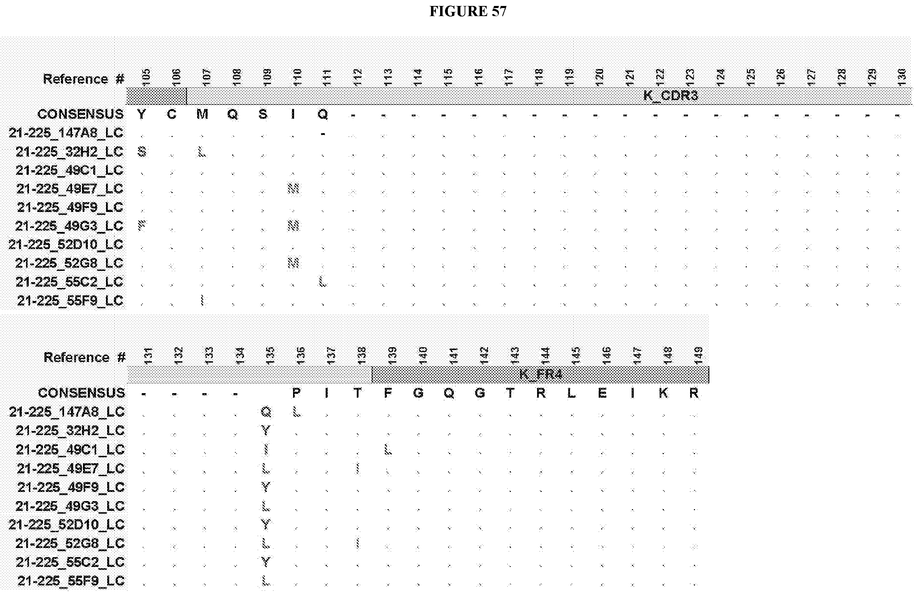

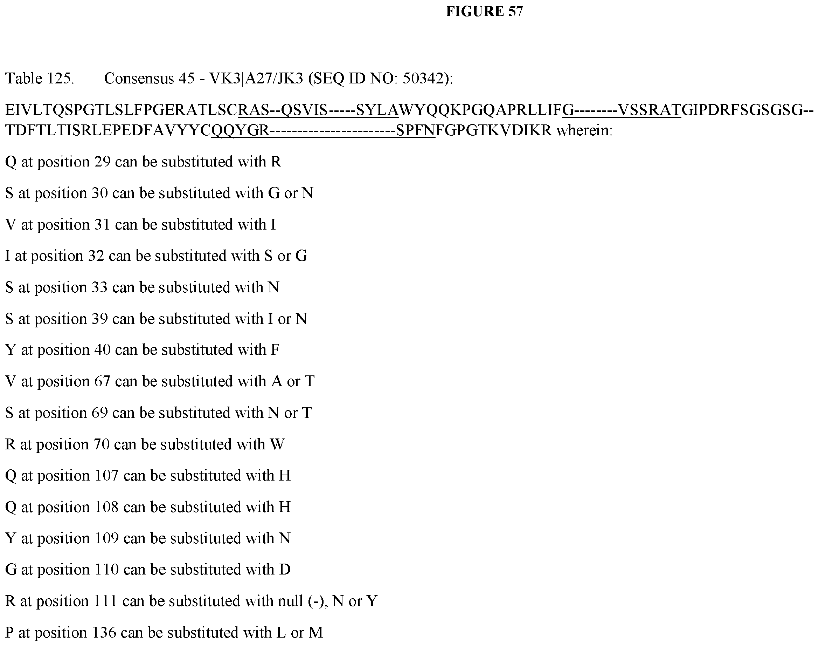

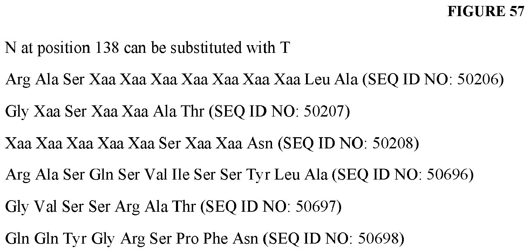

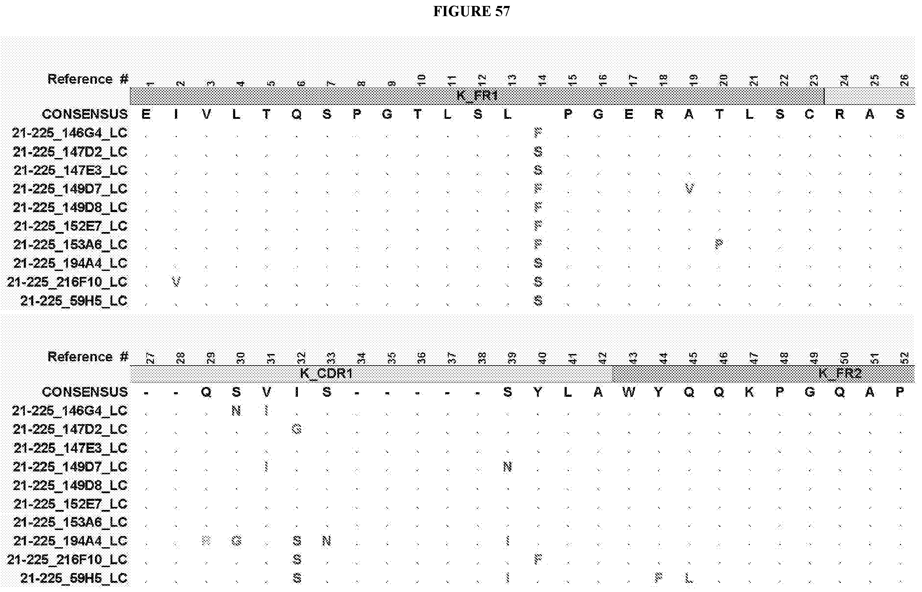

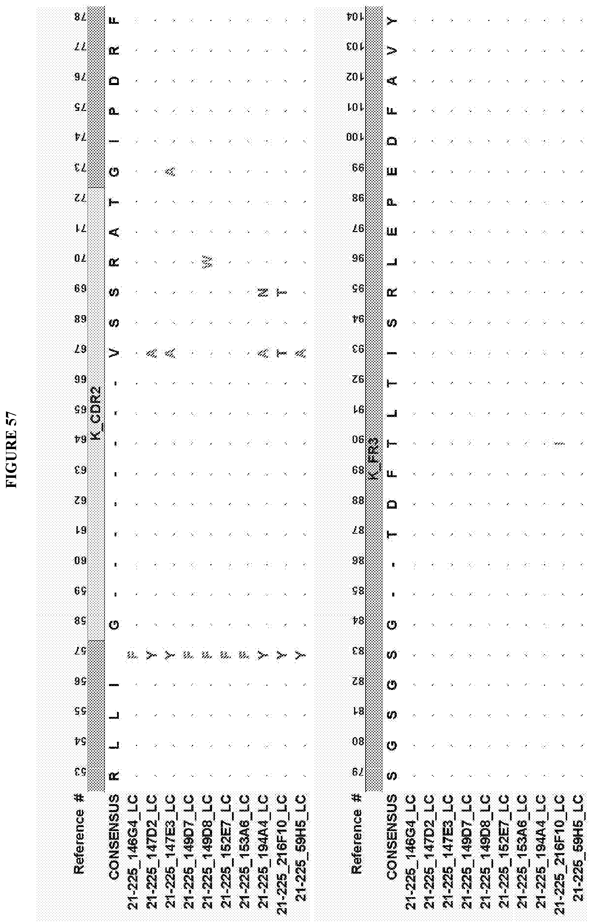

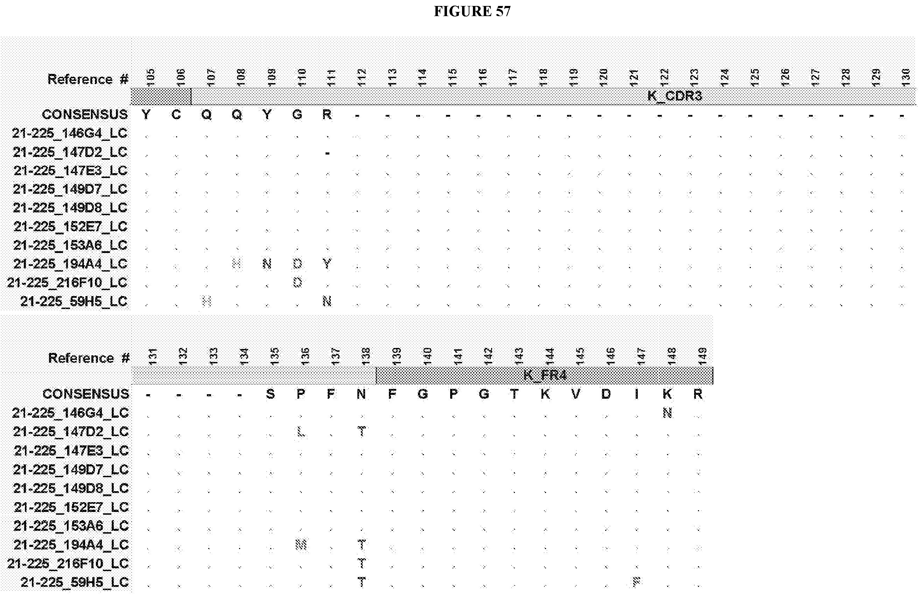

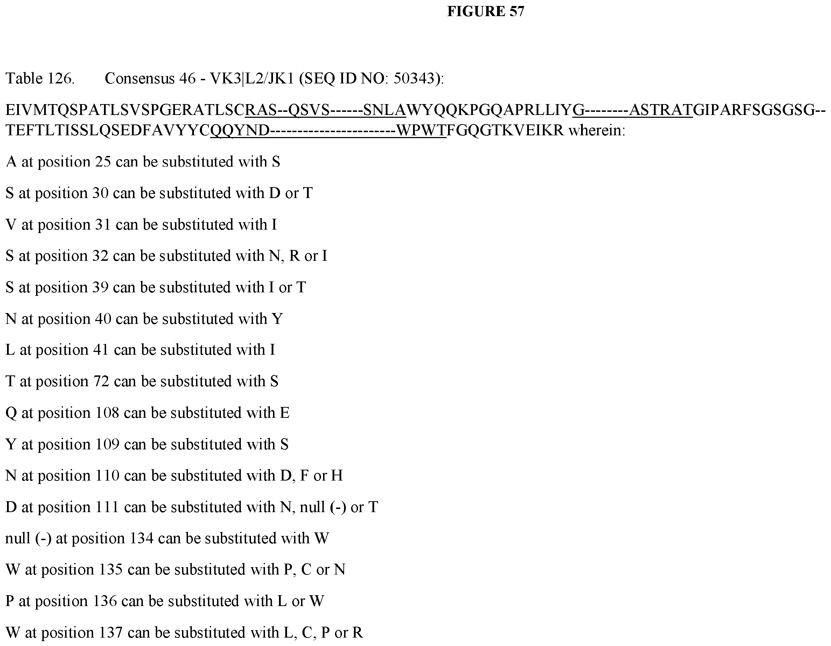

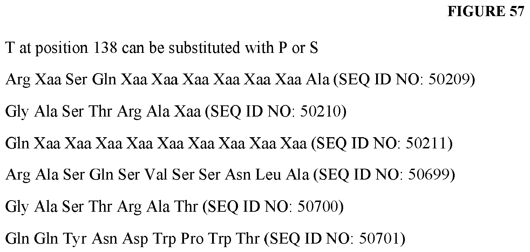

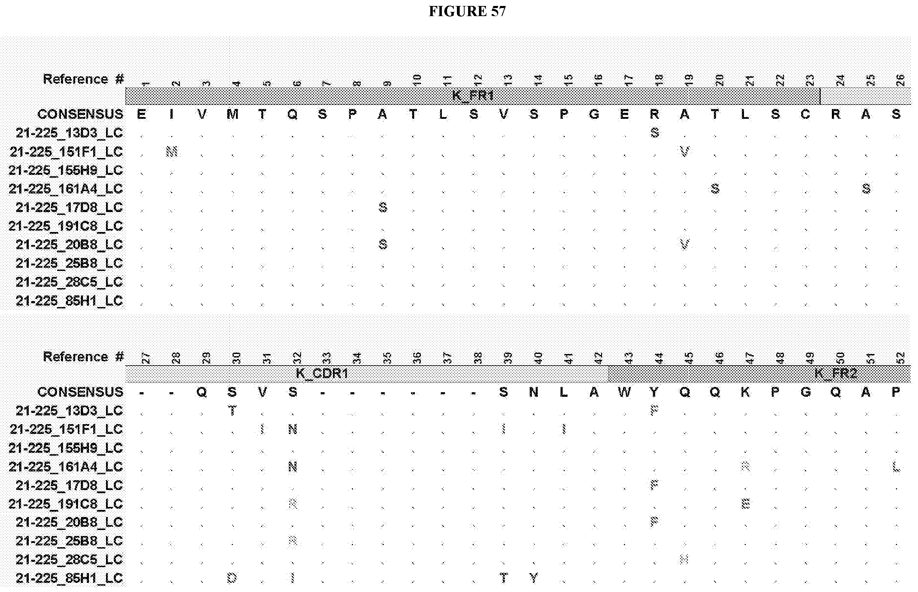

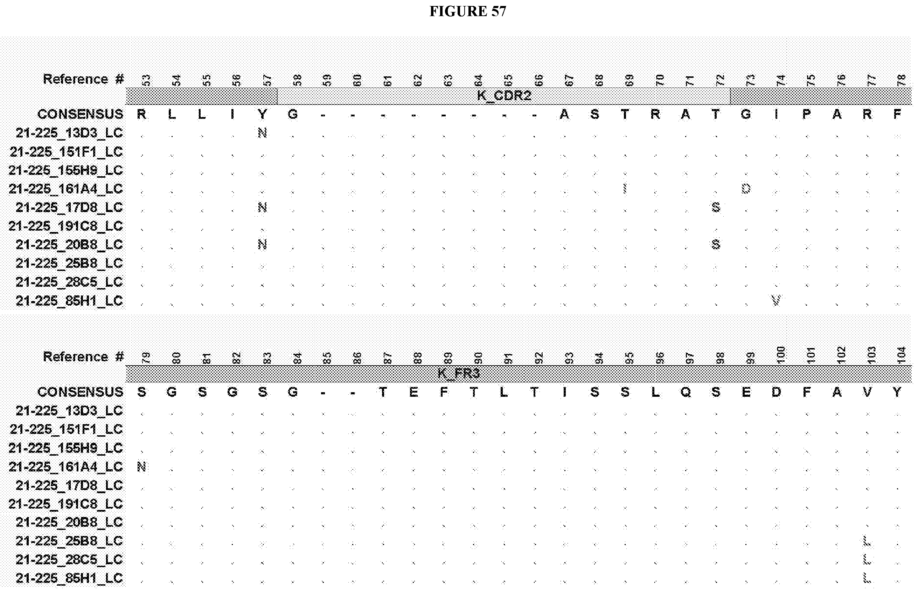

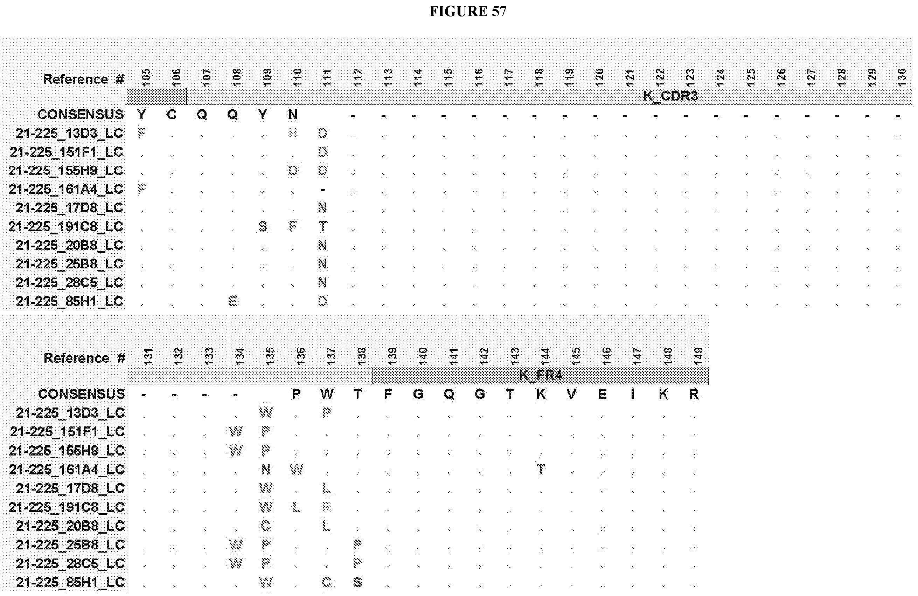

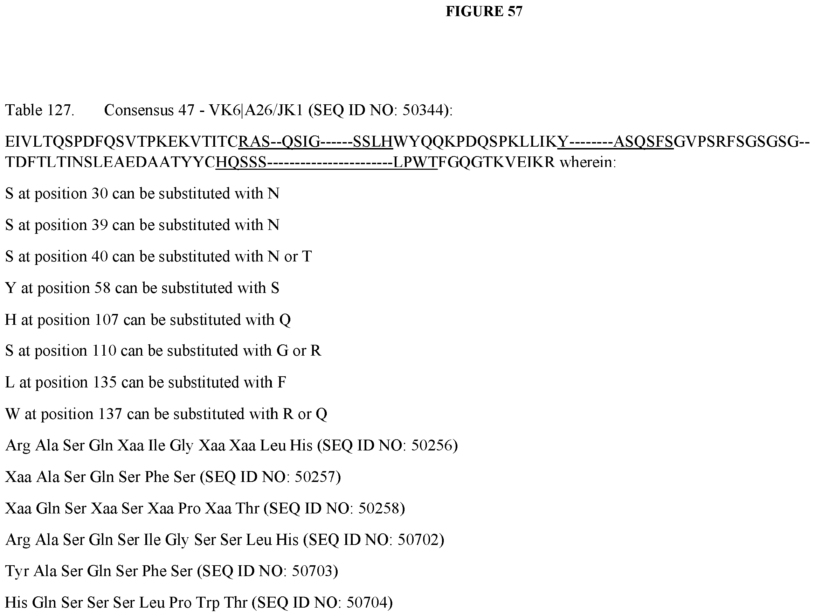

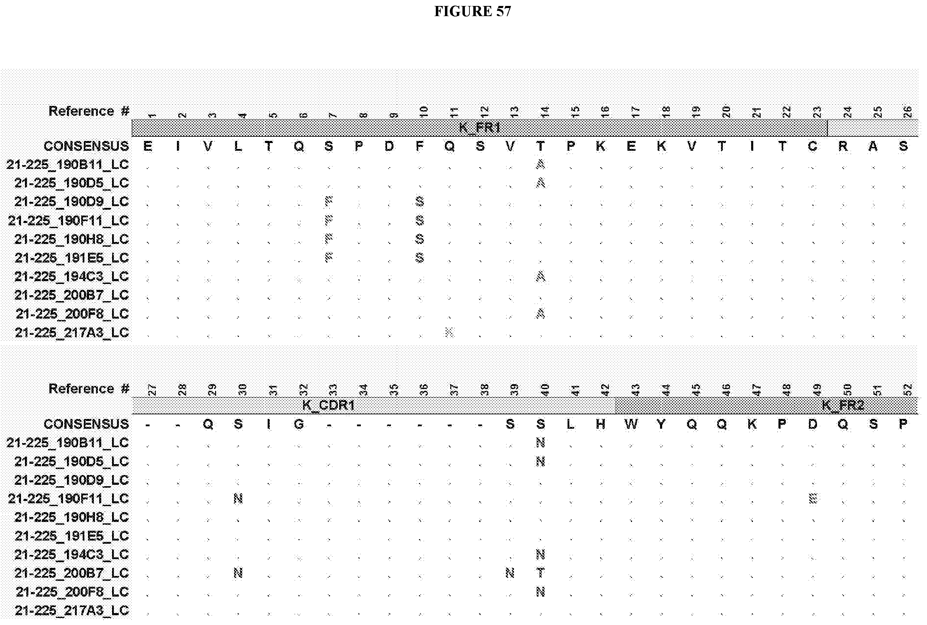

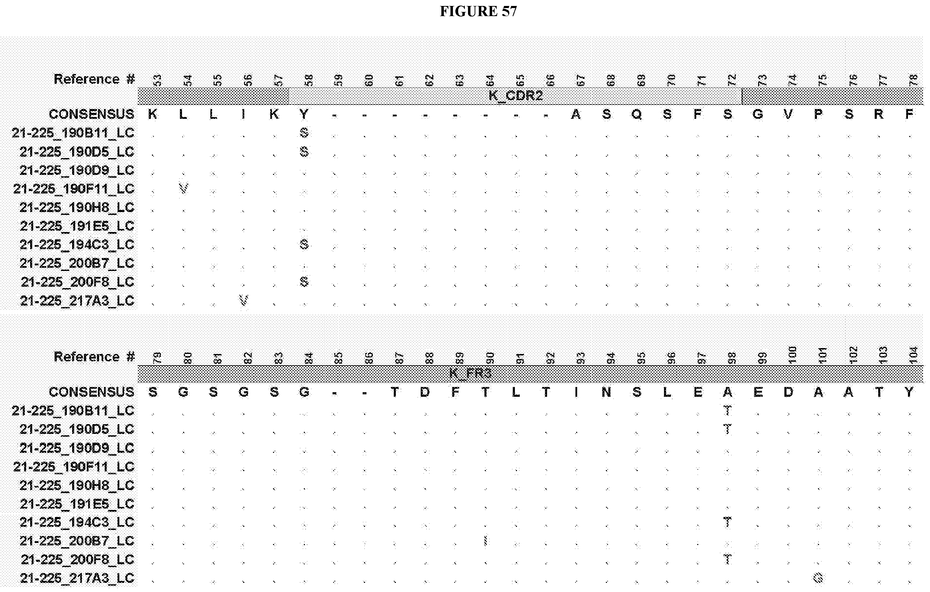

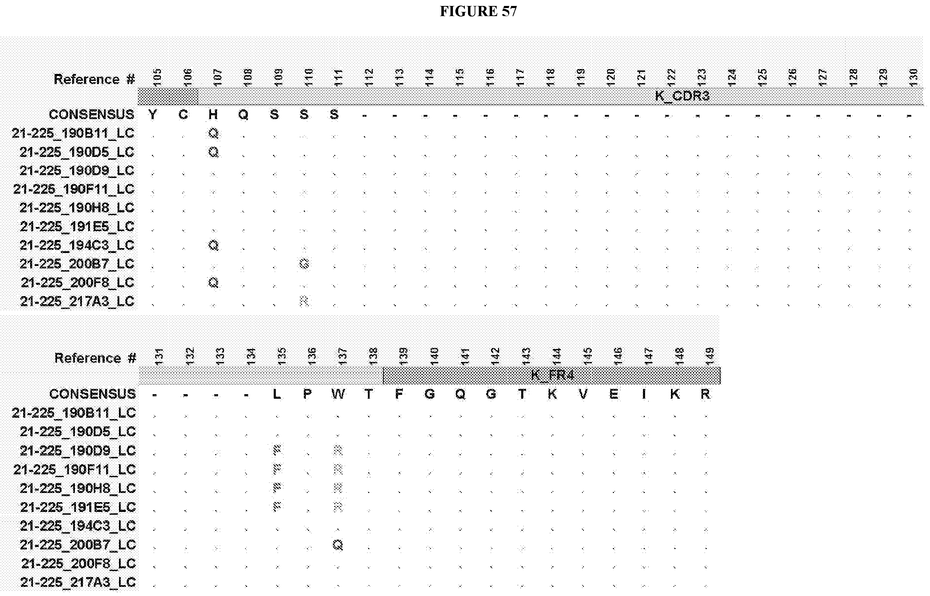

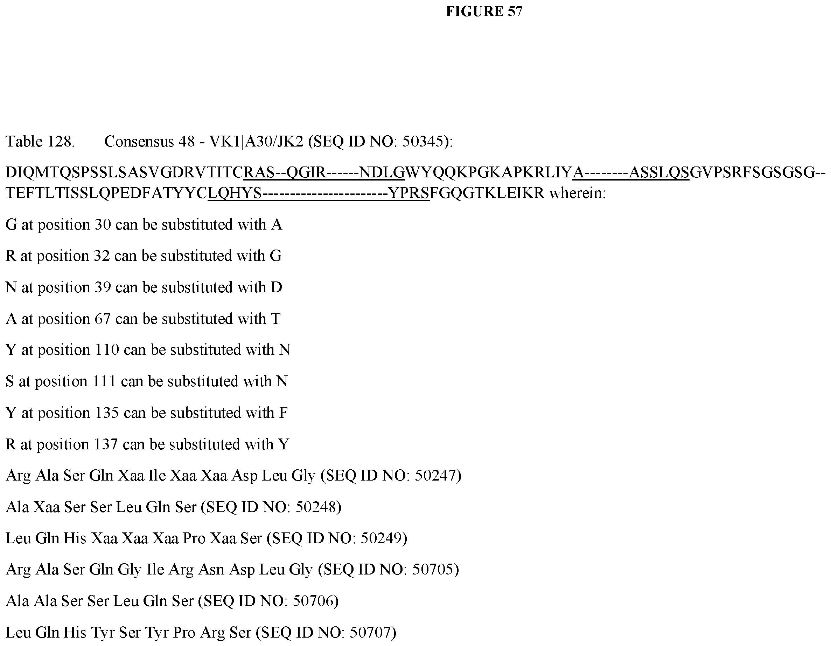

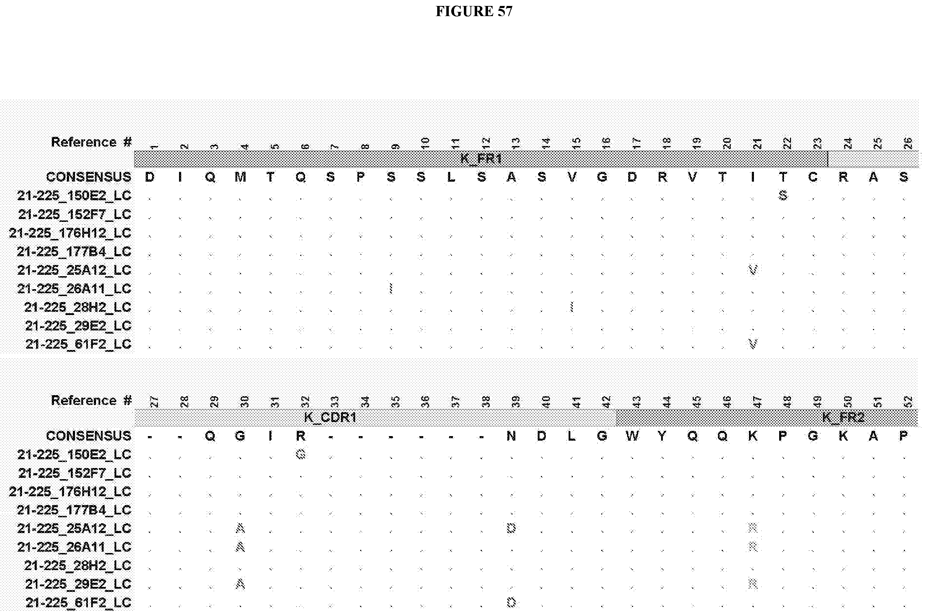

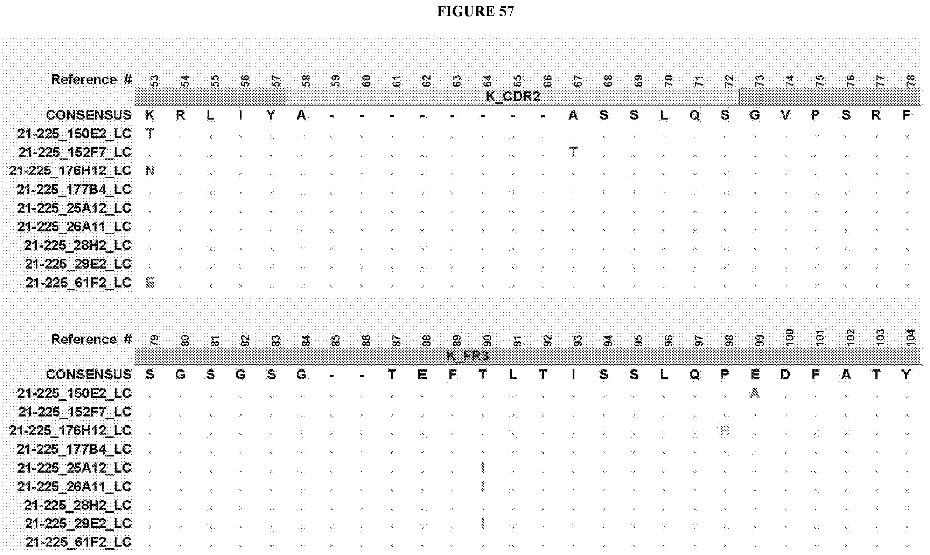

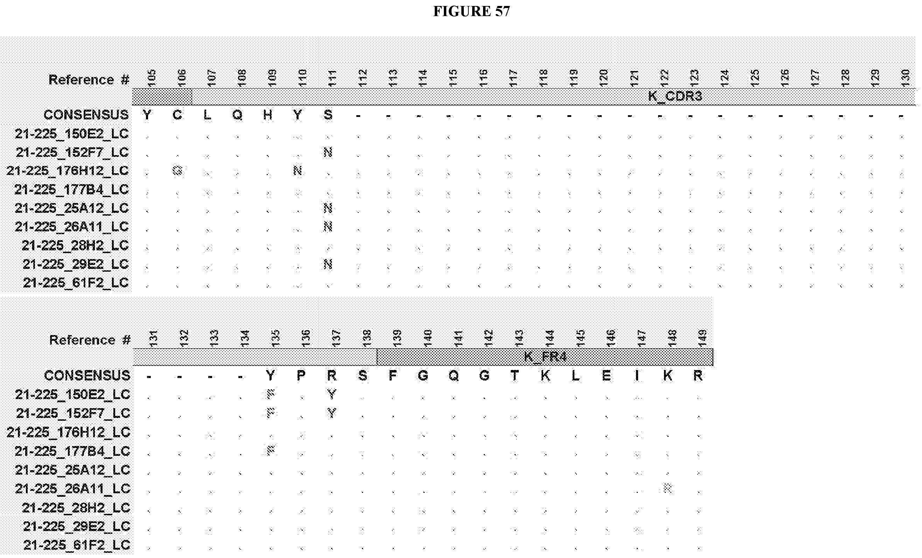

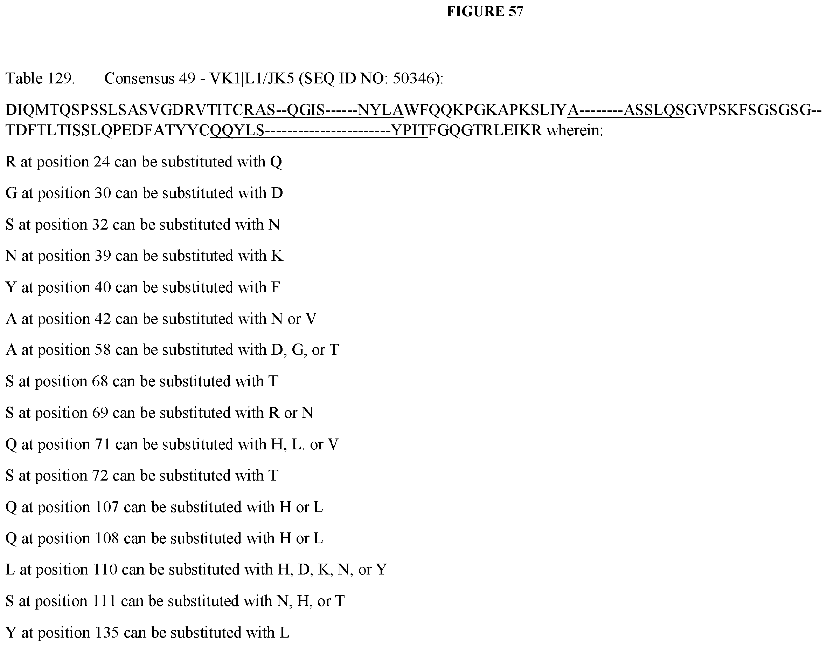

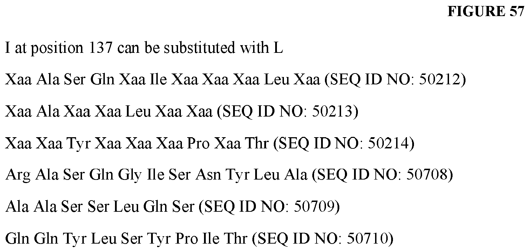

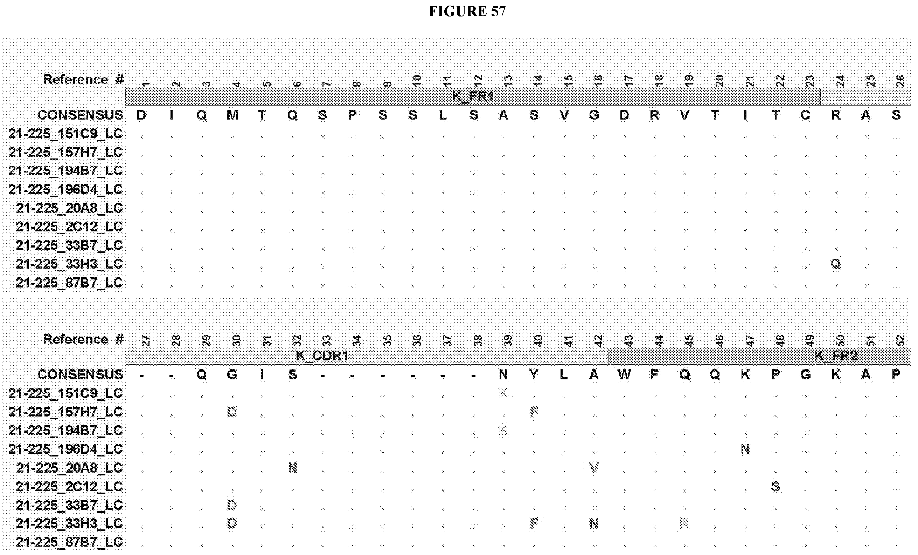

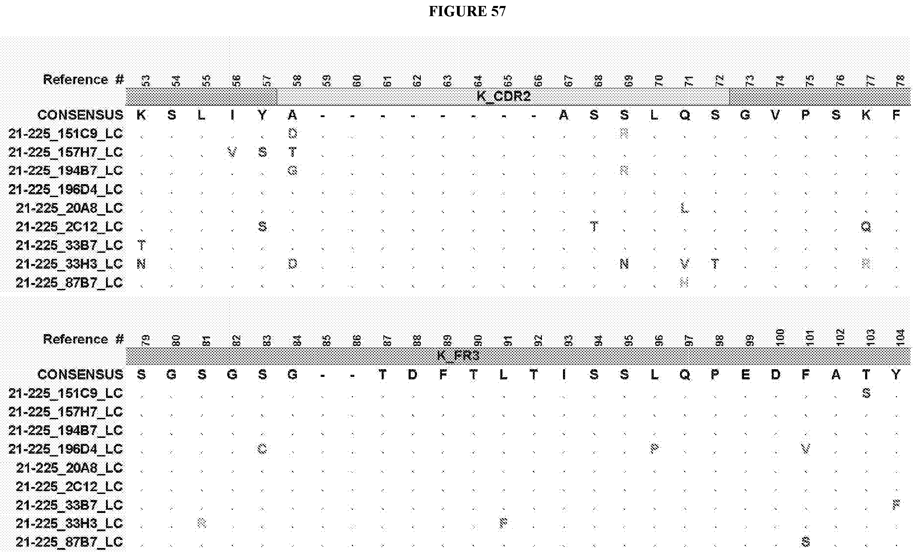

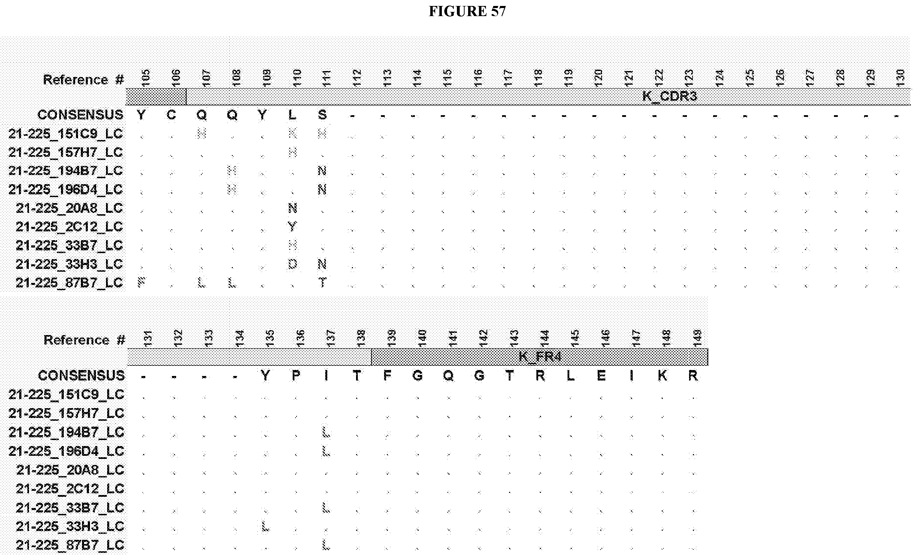

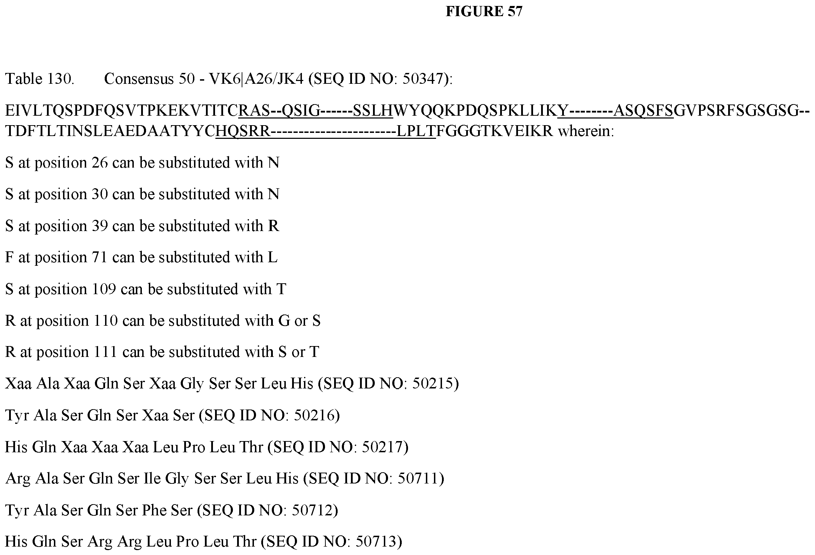

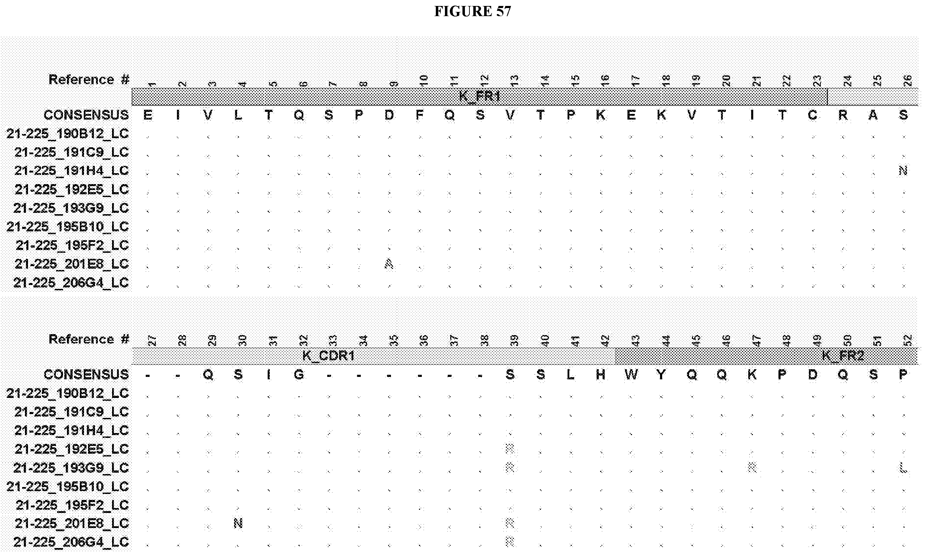

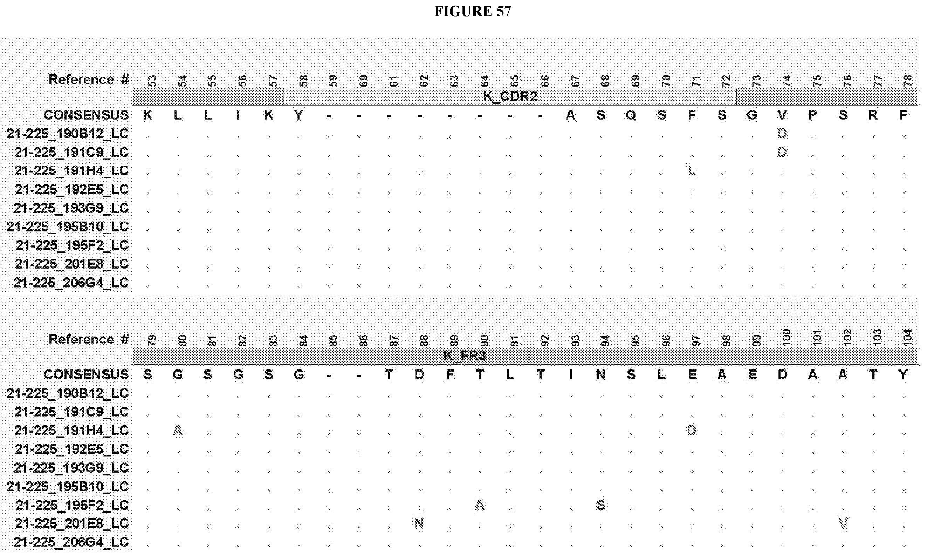

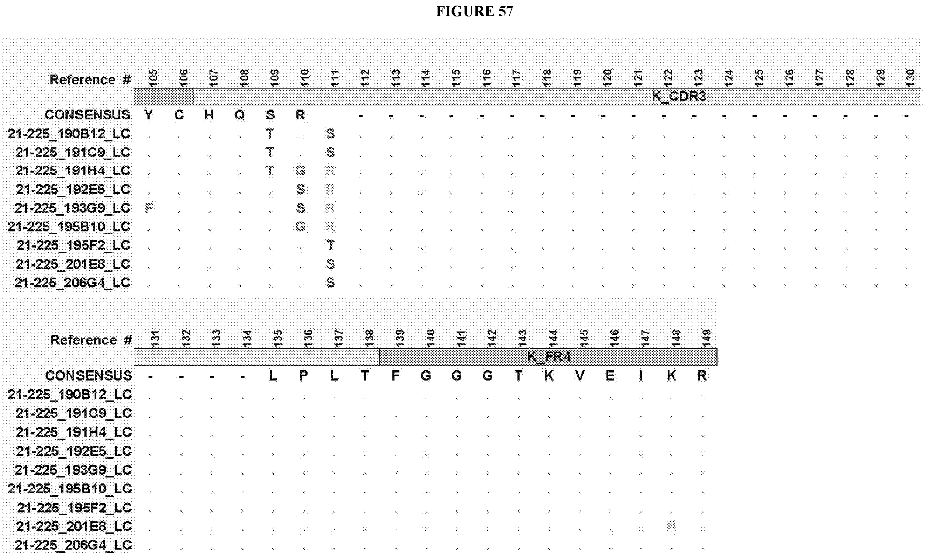

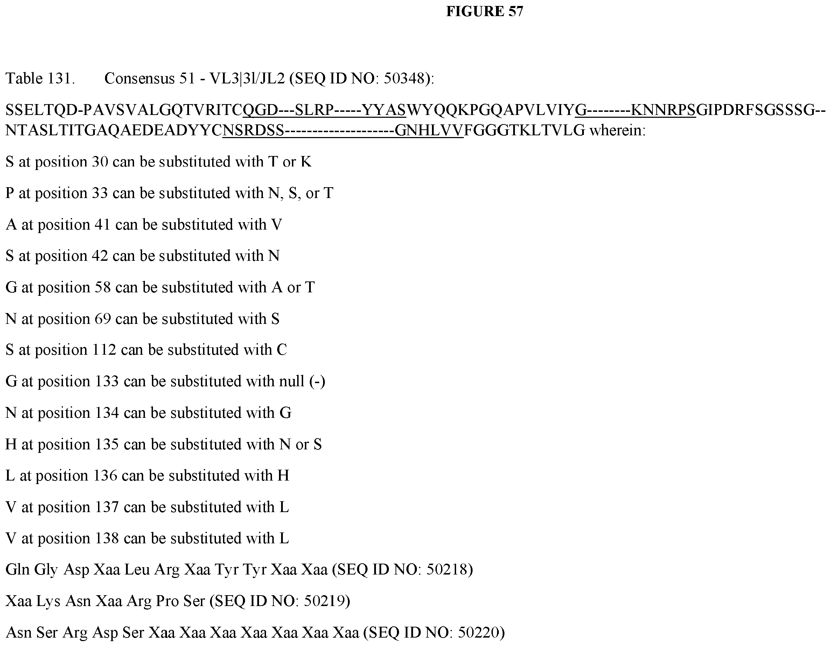

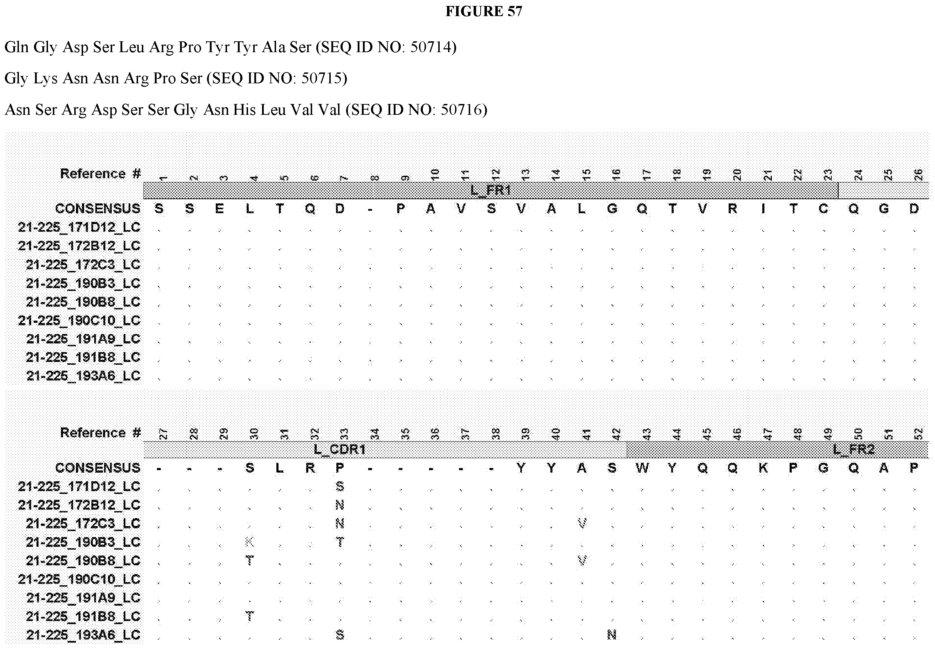

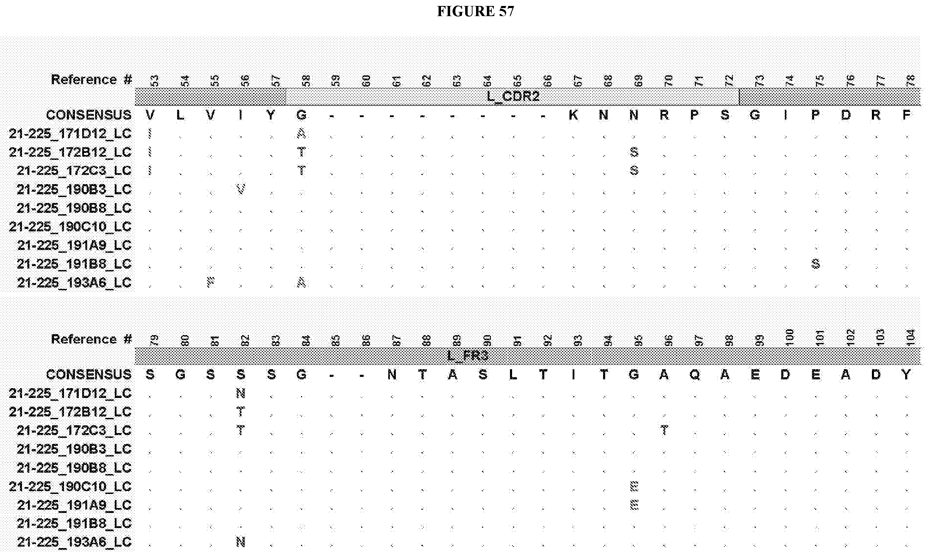

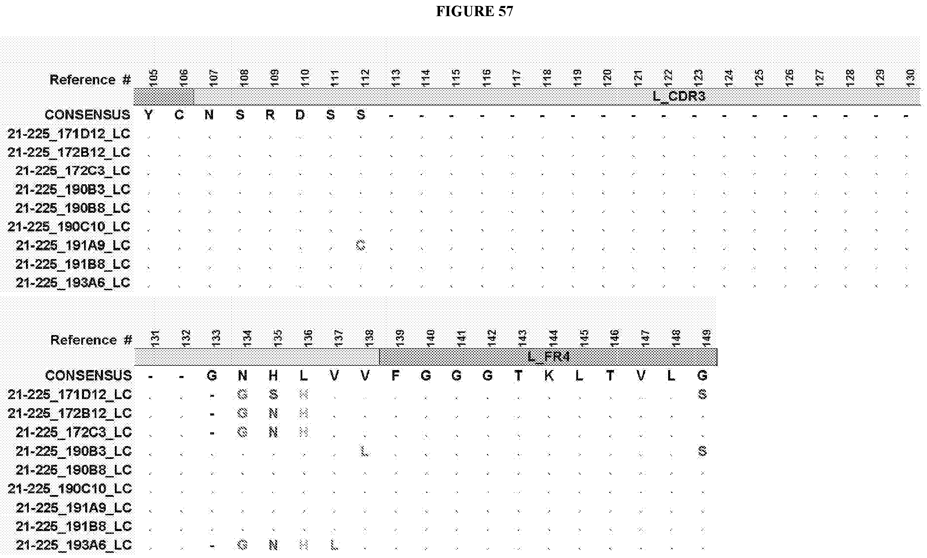

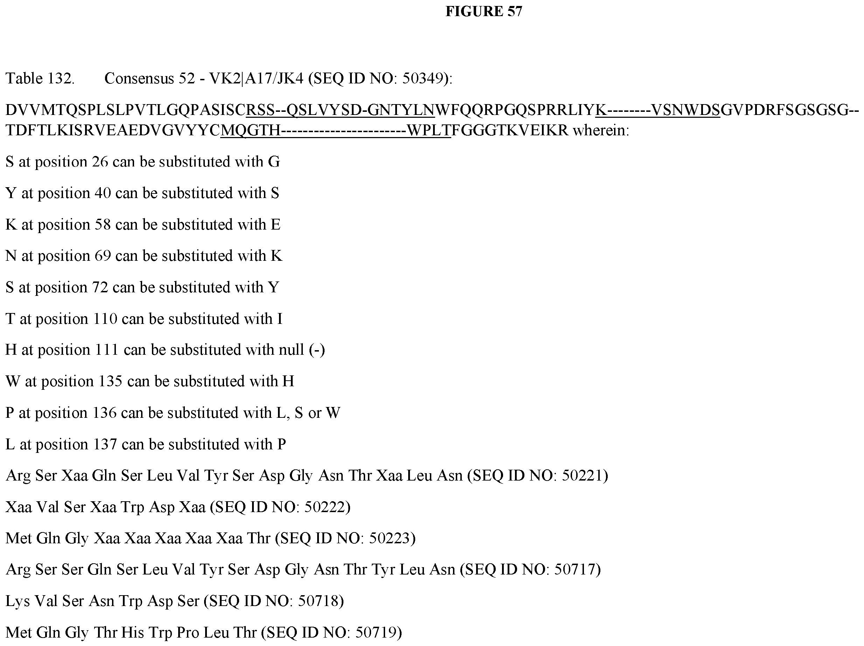

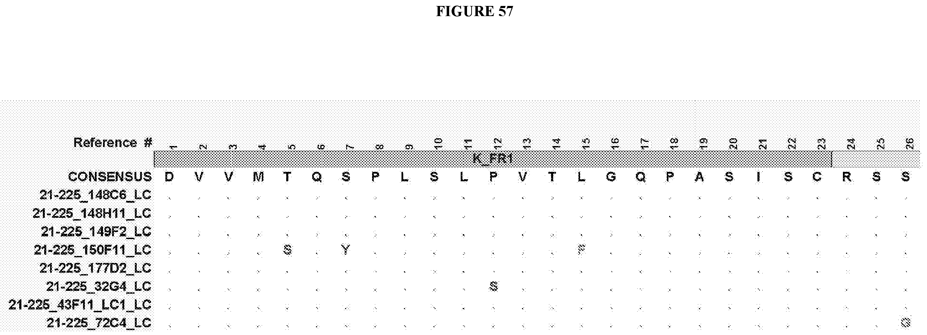

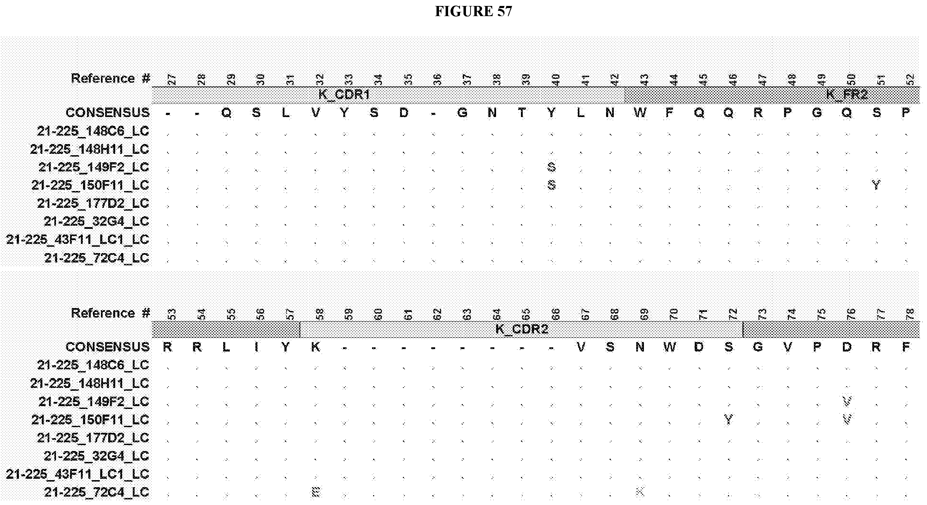

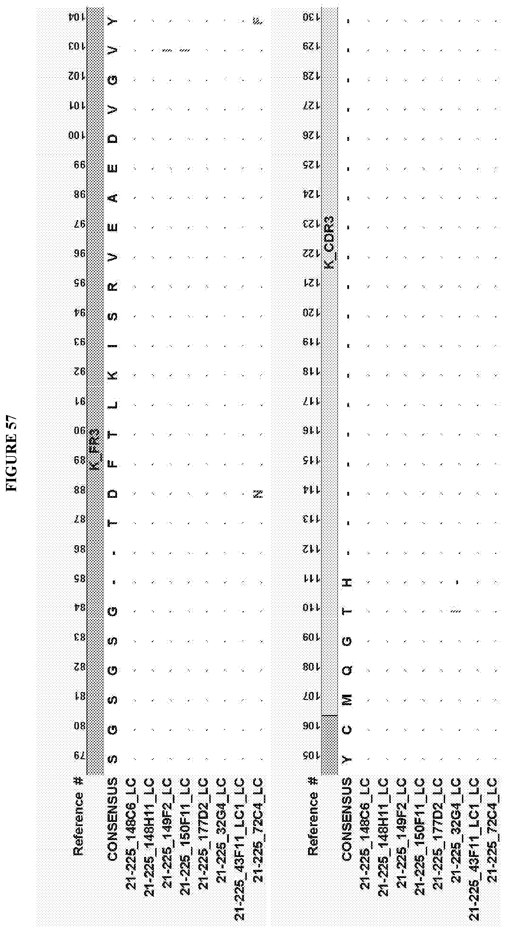

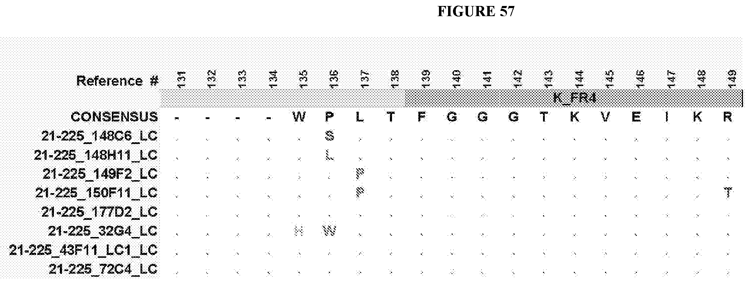

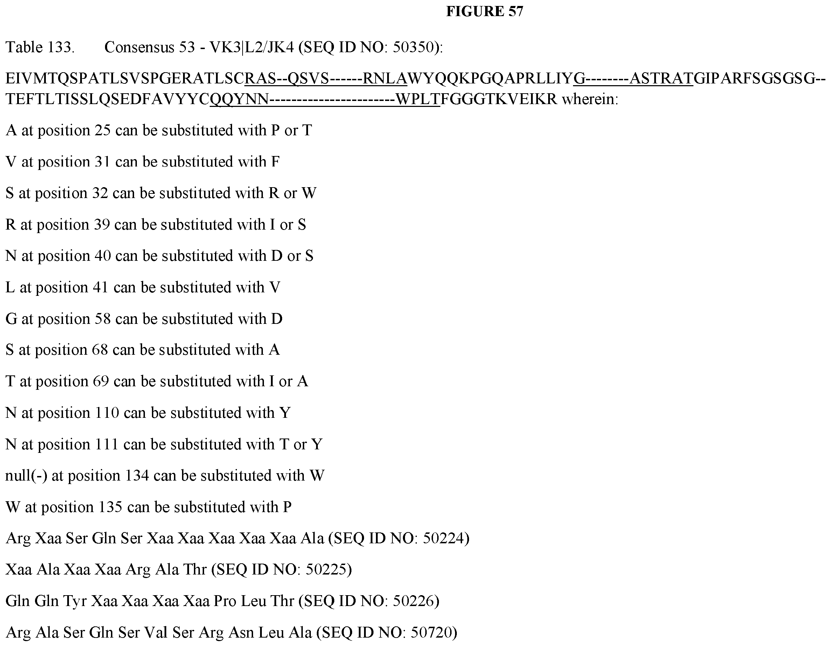

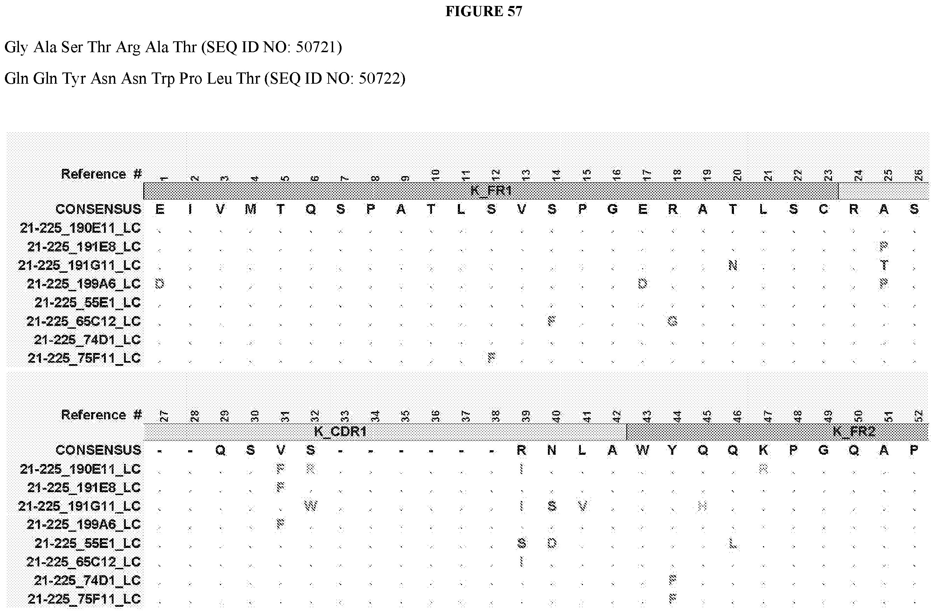

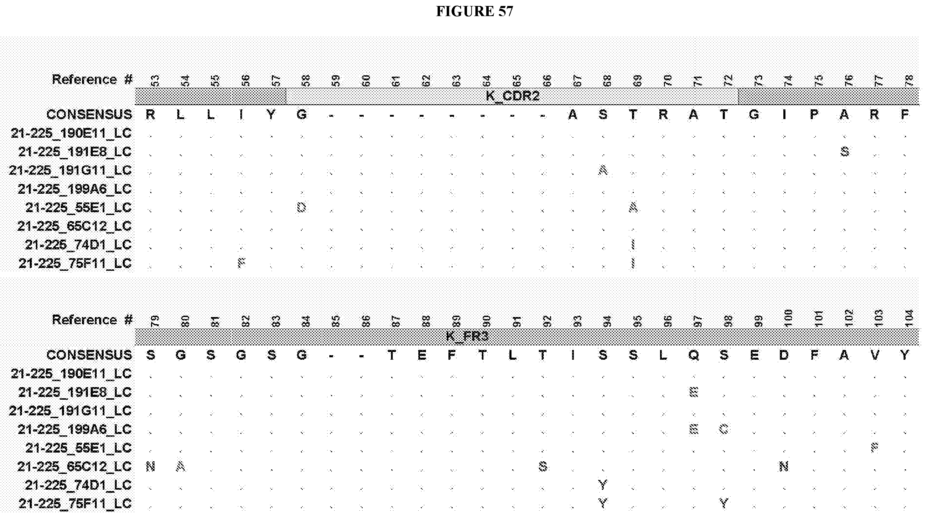

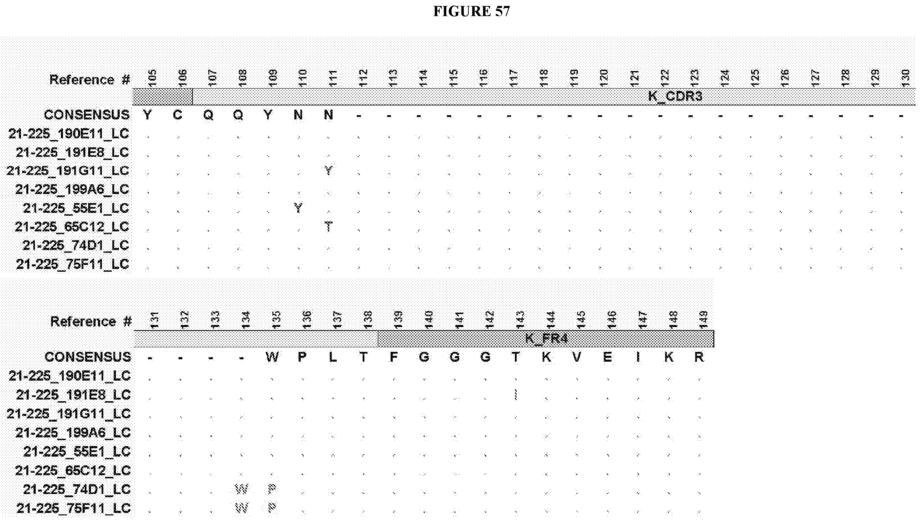

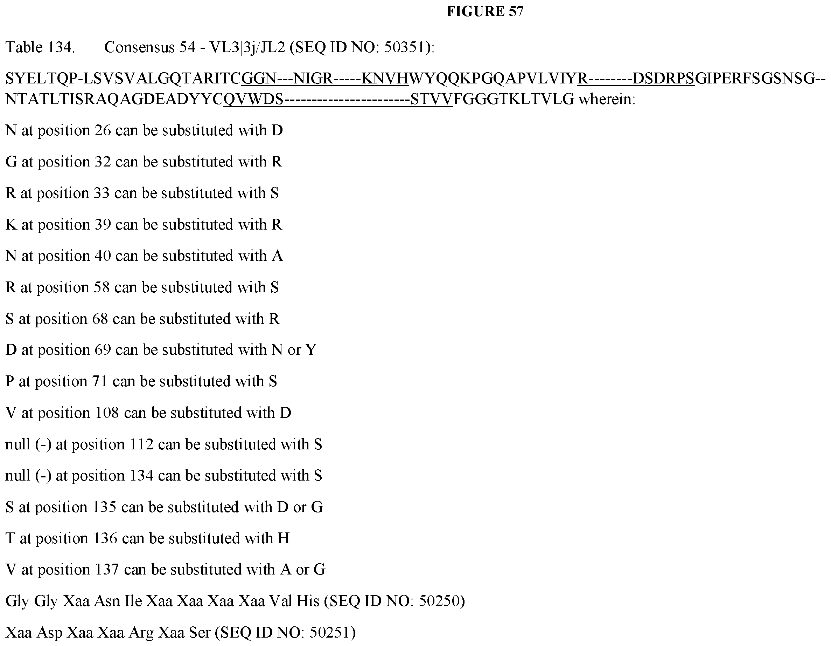

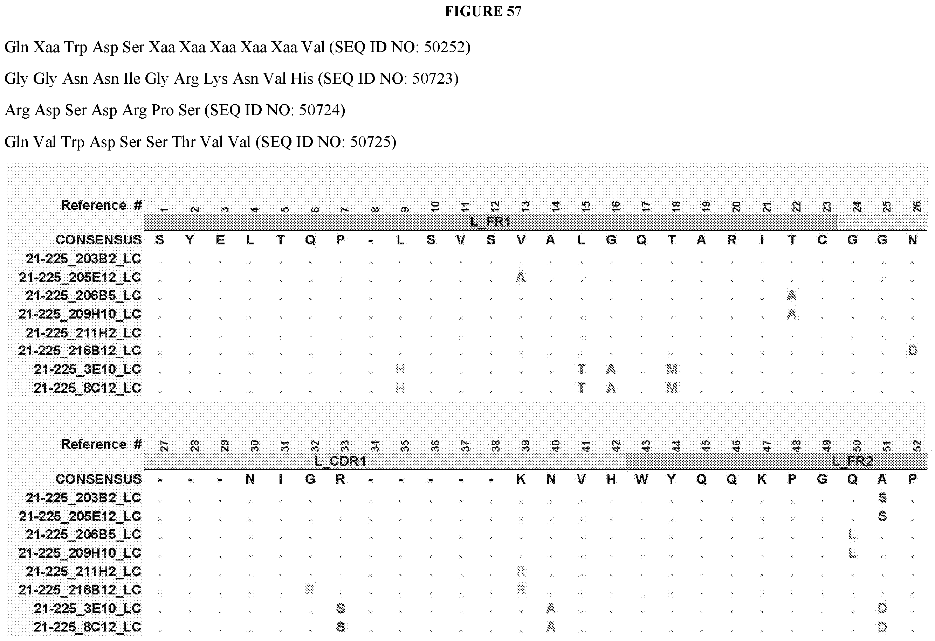

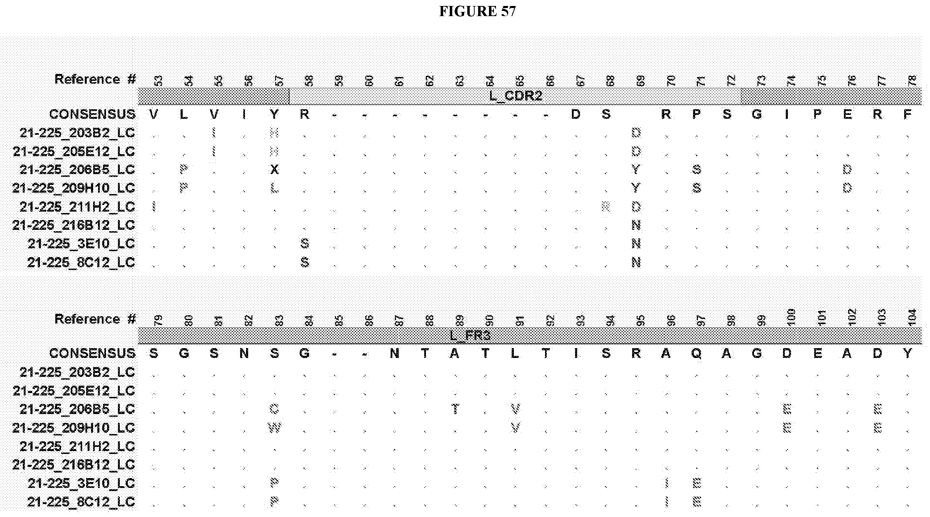

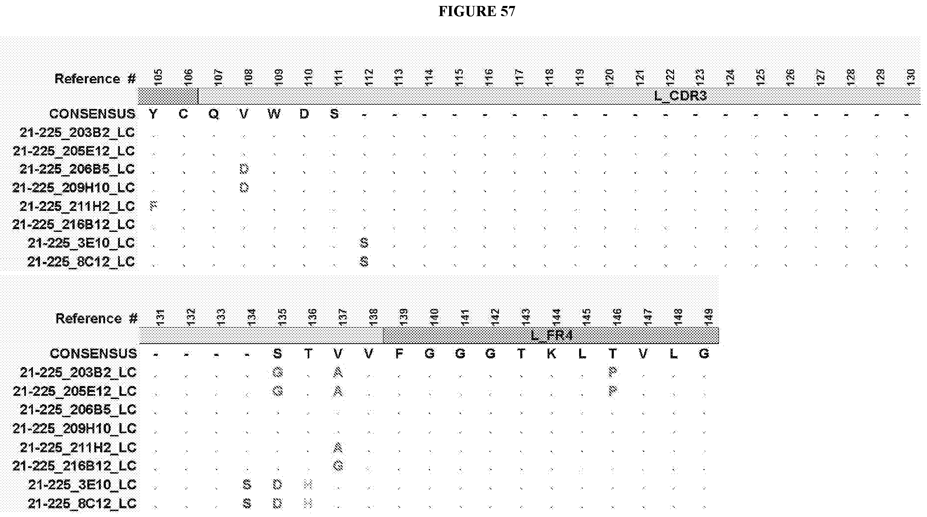

**Figure 57.** A group of tables presenting the consensus protein alignment of various light and heavy chain variable regions for certain antigen binding proteins (Tables 49-134).

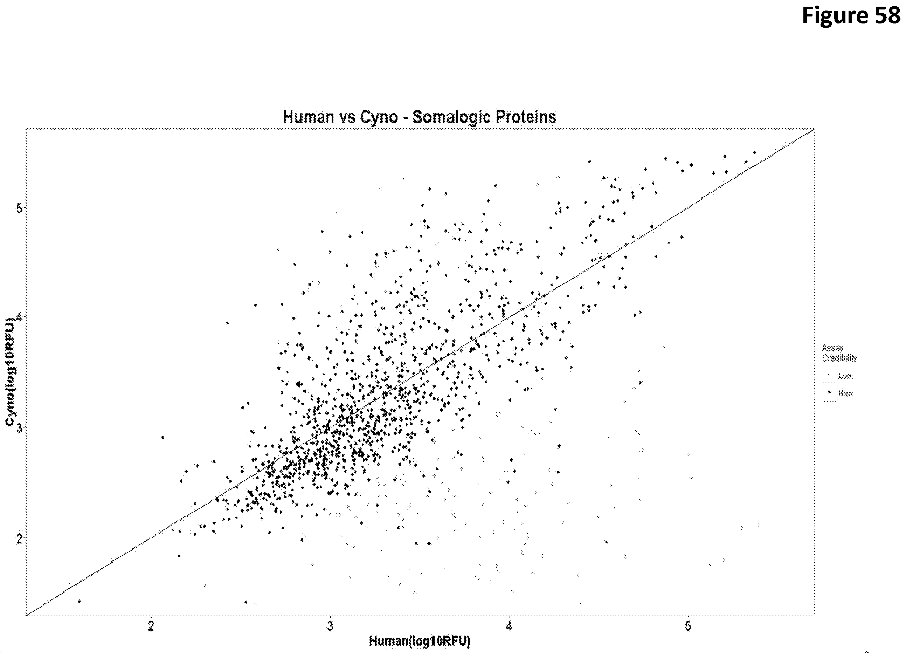

**Figure 58.** A graph depicting the credibility of protein measurements in cynomolgus monkey. Log10 RFU of mean protein levels in the two species are plotted and the ones with low credibility (light dots) and high credibility (darker dots) are marked.

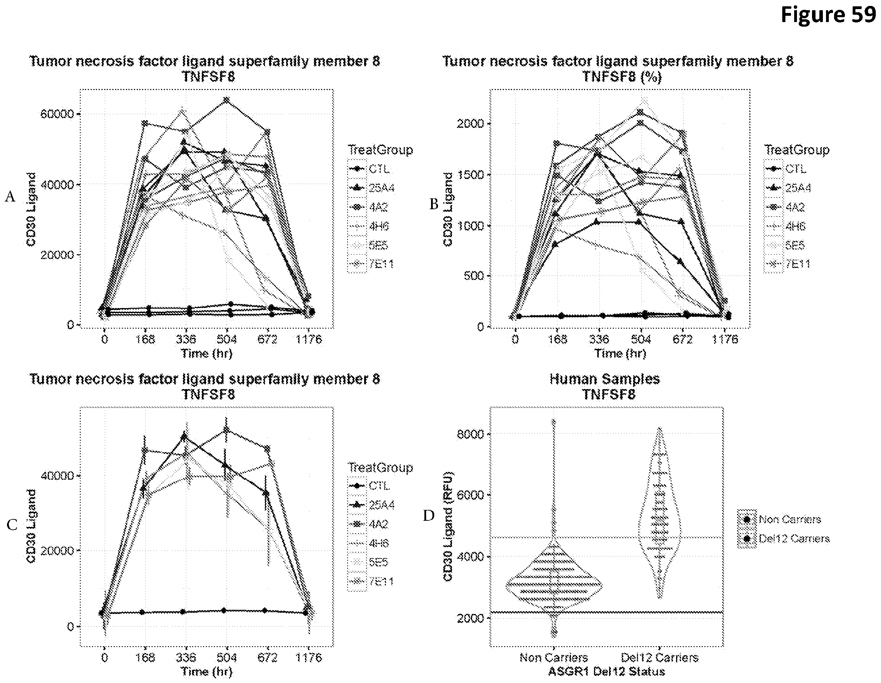

**Figure 59.** Serum protein analysis of cynomolgus monkey treated with anti- ASGR-1 antibodies. Panel A is a graph depicting TNFSF8 protein levels in individual animals of different treatment group across the time points. Panel B is a graph depicting normalized TNFSF8 protein levels (percent of time point 0) in individual animals of different treatment groups across the time points. Panel C is a graph depicting TNFSF8 protein levels in each treatment group (n=3, error bar represents the SEM), and Panel D is a graph depicting the distribution of TNFSF8 protein levels in human ASGR1 del12 carriers and non-carriers.

DETAILED DESCRIPTION OF THE VARIOUS EMBODIMENTS

As shown in Example 1 below, sequence variants in ASGR-1 (which resulted in either a faster degrading ASGR1 or a loss of function ASGR1 mutation) resulted in a lowering in the level of non-HDL cholesterol in humans. This in turn resulted in a decrease in the risk of coronary artery disease experienced by these people. As loss of function mutations in ASGR-1 resulted in both the lowering of non-HDL cholesterol and the lowering of coronary artery disease, antibodies and inhibitory RNA that effectively block ASGR can be used to lower the risk of coronary artery disease.

The present invention is directed to medical uses of interfering RNA of ASGR-1 and/or ASGR-2. In the present invention, the interfering RNA of ASGR-1 and/or ASGR-2 is used for treating or preventing a cardiovascular disease in a patient.

In this application, the use of the singular includes the plural unless specifically stated otherwise. In this application, the use of "or" means "and/or" unless stated otherwise. Furthermore, the use of the term "including", as well as other forms, such as "includes" and "included", is not limiting. Also, terms such as "element" or "component" encompass both elements and components comprising one unit and elements and components that comprise more than one subunit unless specifically stated otherwise. Also, the use of the term "portion" can include part of a moiety or the entire moiety.

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures used in connection with, and techniques of, cell and tissue culture, molecular biology, immunology, microbiology, genetics and protein and nucleic acid chemistry and hybridization described herein are those well-known and commonly used in the art. The methods and techniques described herein are generally performed according to conventional methods well known in the art and as described in various general and more specific references that are cited and discussed throughout the present specification unless otherwise indicated. See, e.g., Sambrook et al. Molecular Cloning: A Laboratory Manual, 2d ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual Cold Spring Harbor Laboratory Press, Cold Spring Harbor, N.Y. (1990). Enzymatic reactions and purification techniques are performed according to manufacturer's specifications, as commonly accomplished in the art or as described herein. The terminology used in connection with, and the laboratory procedures and techniques of, analytical chemistry, synthetic organic chemistry, and medicinal and pharmaceutical chemistry described herein are those well-known and commonly used in the art. Standard techniques can be used for chemical syntheses, chemical analyses, pharmaceutical preparation, formulation, and delivery, and treatment of patients.

Polynucleotide and polypeptide sequences are indicated using standard one- or three-letter abbreviations. Unless otherwise indicated, polypeptide sequences have their amino termini at the left and their carboxy termini at the right, and single-stranded nucleic acid sequences, and the top strand of double-stranded nucleic acid sequences, have their 5' termini at the left and their 3' termini at the right. A particular section of a polypeptide can be designated by amino acid residue number such as amino acids 1 to 50, or by the actual residue at that site such as asparagine to proline. A particular polypeptide or polynucleotide sequence also can be described by explaining how it differs from a reference sequence.

The following terms, unless otherwise indicated, shall be understood to have the following meanings:

The term "inhibitor" as used herein, is a compound that decreases the magnitude of at least one activity or function of a molecule compared to the magnitude of the activity or function observed in the absence of the inhibitor. In some instances, an inhibitor will substantially decrease the magnitude of at least one activity or function of a molecule compared to the magnitude of the activity or function observed in the absence of the inhibitor. In some instances, an inhibitor will completely diminish the magnitude of at least one activity or function of a molecule compared to the magnitude of the activity or function observed in the absence of the inhibitor. Certain exemplary inhibitors include, but are not limited to, proteins, peptides, antibodies, peptibodies, aptamers, antisense oligonucleotides, interfering RNA, carbohydrates or small organic molecules.

The term "isolated molecule" (where the molecule is, for example, a polypeptide, a polynucleotide, antigen binding protein or an antibody) is a molecule that by virtue of its origin or source of derivation (1) is not associated with naturally associated components that accompany it in its native state, (2) is substantially free of other molecules from the same species (3) is expressed by a cell from a different species, or (4) does not occur in nature. Thus, a molecule that is chemically synthesized, or expressed in a cellular system different from the cell from which it naturally originates, will be "isolated" from its naturally associated components. A molecule also may be rendered substantially free of naturally associated components by isolation, using purification techniques well known in the art. Molecule purity or homogeneity may be assayed by a number of means well known in the art. For example, the purity of a polypeptide sample may be assayed using polyacrylamide gel electrophoresis and staining of the gel to visualize the polypeptide using techniques well known in the art. For certain purposes, higher resolution may be provided by using HPLC or other means well known in the art for purification.

The terms "polynucleotide," "oligonucleotide" and "nucleic acid" are used interchangeably throughout and include DNA molecules (e.g., cDNA or genomic DNA), RNA molecules (e.g., mRNA), analogs of the DNA or RNA generated using nucleotide analogs (e.g., peptide nucleic acids and non-naturally occurring nucleotide analogs), and hybrids thereof. The nucleic acid molecule can be single-stranded or double-stranded. In one embodiment, the nucleic acid molecules comprise a contiguous open reading frame encoding an antibody, or a fragment, derivative, mutein, or variant thereof, as described herein.

A "vector" is a nucleic acid that can be used to introduce another nucleic acid linked to it into a cell. One type of vector is a "plasmid," which refers to a linear or circular double stranded DNA molecule into which additional nucleic acid segments can be ligated. Another type of vector is a viral vector (e.g., replication defective retroviruses, adenoviruses and adeno-associated viruses), wherein additional DNA segments can be introduced into the viral genome. Certain vectors are capable of autonomous replication in a host cell into which they are introduced (e.g., bacterial vectors comprising a bacterial origin of replication and episomal mammalian vectors). Other vectors (e.g., non-episomal mammalian vectors) are integrated into the genome of a host cell upon introduction into the host cell, and thereby are replicated along with the host genome. An "expression vector" is a type of vector that can direct the expression of a chosen polynucleotide.

A nucleotide sequence is "operably linked" to a regulatory sequence if the regulatory sequence affects the expression (e.g., the level, timing, or location of expression) of the nucleotide sequence. A "regulatory sequence" is a nucleic acid that affects the expression (e.g., the level, timing, or location of expression) of a nucleic acid to which it is operably linked. The regulatory sequence can, for example, exert its effects directly on the regulated nucleic acid, or through the action of one or more other molecules (e.g., polypeptides that bind to the regulatory sequence and/or the nucleic acid). Examples of regulatory sequences include promoters, enhancers and other expression control elements (e.g., polyadenylation signals). Further examples of regulatory sequences are described in, for example, Goeddel, 1990, Gene Expression Technology: Methods in Enzymology 185, Academic Press, San Diego, CA and Baron et al., 1995, Nucleic Acids Res. 23:3605-06.

A "host cell" is a cell that can be used to express a nucleic acid, e.g., a nucleic acid. A host cell can be a prokaryote, for example, E. coli, or it can be a eukaryote, for example, a single-celled eukaryote (e.g., a yeast or other fungus), a plant cell (e.g., a tobacco or tomato plant cell), an animal cell (e.g., a human cell, a monkey cell, a hamster cell, a rat cell, a mouse cell, or an insect cell) or a hybridoma. Typically, a host cell is a cultured cell that can be transformed or transfected with a polypeptide-encoding nucleic acid, which can then be expressed in the host cell. The phrase "recombinant host cell" can be used to denote a host cell that has been transformed or transfected with a nucleic acid to be expressed. A host cell also can be a cell that comprises the nucleic acid but does not express it at a desired level unless a regulatory sequence is introduced into the host cell such that it becomes operably linked with the nucleic acid. It is understood that the term host cell refers not only to the particular subject cell but to the progeny or potential progeny of such a cell. Because certain modifications may occur in succeeding generations due to, e.g., mutation or environmental influence, such progeny may not, in fact, be identical to the parent cell, but are still included within the scope of the term as used herein.

## ASGR

Genomic database analysis is one manner that allows for the discovery of associations between disease states and particular targets and/or pathways. For example, genetic analysis of patients with familial hypercholesterolemia resulted in the discovery of proprotein convertase subtilisin/kexin type 9 (PCSK9) being involved with regulating serum LDL cholesterol levels and risk of developing coronary artery disease, and ultimately, in the development of the recently approved Repatha®, an anti-hPCSK9 antibody, (see, e.g., Jackson et al., U.S. Patent No. 8,030,457). Advances in DNA sequencing technology provide the means to sequence the genomes of large numbers of individuals allowing for discovery of rare variants. deCODE Genetics (an Amgen company) has previously reported methods to analyze whole genomes of large numbers of Icelanders in order to search for associations between genetic variants and traits of interest. (Gudbjartsson et al., Nature Genetics; Vol. 47; 5; May 2015; p.435-444).

This methodology has now been applied in the search for novel genetic variants that affect cardiovascular disease, including cholesterol levels, and the risk for developing coronary artery disease and myocardial infarction (MI). The groundbreaking analysis performed has identified novel sequence variants of the Ashwell-Morell Receptor that are implicated in cardiovascular disease.

Whole-genome sequencing of the Icelandic population discovered a rare, 12 base pair deletion ("del12") in intron 4 of the ASGR-1 gene that is also present in other European ancestry populations. This deletion leads to a frameshift predicted to generate a truncated ASGR-1 receptor subunit that is lacking both the oligomerization and extracellular carbohydrate recognition domains (also known as "CRD," "carbohydrate binding domain" or "CBD") or may generate an unstable and rapidly degraded transcript (and therefore no protein) due to nonsense mediated decay. Whole-genome sequencing of the Icelandic population also discovered a second rare loss of function variant in the ASGR-1 gene; namely, a 4 base pair insertion in exon 7 (c.469-472dupAACT or "W158X"). This 4 base pair insertion in exon 7 causes a frameshift and introduces a premature stop codon at amino acid 158 out of the 291 amino acid full length protein (NP_001662.1:p.W158X). This variant is predicted to encode a protein lacking the carbohydrate recognition domain of the receptor or may generate an unstable and rapidly degraded transcript (and therefore no protein) due to nonsense mediated decay. Furthermore, the W158X variant effects all reported refseq transcripts of ASGR-1 regardless of tissue or cell type of expression. Without wishing to be bound by any particular hypothesis, the analysis indicates that del12 and W158X results in lower non-HDL cholesterol levels, protection against CAD and MI, leading to prolonged life. Additionally, the analysis indicates that del12 and W158X also associates with increased levels of circulating ALP and vitamin B12. Supporting this del12 and W158X association with increased levels of ALP are data from mice having a Y272C variant in ASGR-1, showing that these mice exhibit a phenotype of increased plasma ALP (Sabrautzki et al., Mamm. Genome, 23, 416- 430,2012). The Y272 position in mouse ASGR-1 corresponds to the Y273 position in human ASGR- 1 (see Figure 1A).

The Ashwell-Morell Receptor (AMR), originally named the hepatic asialoglycoprotein receptor, was one of the first cellular receptors to be isolated and identified. (Grewal, Methods in Enzymology, Volume 479, Chapter 13, 2010, pp.223-241). This receptor is also known as the Ashwell Receptor, the hepatic galactose/N-acetylgalactosamine (Gal-NAc) receptor, or the hepatic lectin receptor. However, this receptor is now more commonly known as "ASGPR," or simply "ASGR."

ASGR is a C-type lectin that is expressed on the surface of hepatocytes and is made up of 48 kDa major subunit(s) (ASGR-1) and 40 kDa minor subunit(s) (ASGR-2). (Roggenbuck et al., Autoimmune Highlights, 2012, 3:119-125). Functional variants of ASGR are formed by the oligomerization of the ASGR-1 and ASGR-2 subunits. (Grewal). The receptor complexes can comprise homo-oligomers and hetero-oligomers of the ASGR-1 and ASGR-2 subunits, with (ASGR- $1)_2$-$(ASGR-2)_1$ trimer being the most common form and having the highest affinity to substrate. (Grewal). Other identified forms of ASGR include $(ASGR-1)_2$, $(ASGR-1)_3$, $(ASGR-1)_2$-$(ASGR-2)_2$, $(ASGR-1)_3$-(ASGR-$2)_2$. (Grewal).

The polynucleotide and polypeptide sequences for several species of ASGR-1 and ASGR-2 are known. Table 1 presents sequences for human, mouse, rat, pig, dog and cynomolgus. Figures 1A, 1B and 2 present sequence alignments of various species of ASGR-1 and ASGR-2, and Figure 3 presents a sequence alignment between human ASGR-1 and human ASGR-2. ASGR-1, an asialoglycoprotein receptor, contains an N-term cytosolic domain, a transmembrane domain, a stalk region and a carbohydrate recognition domain (CRD) (alternatively known as the carbohydrate binding domain, or "CBD"). The carbohydrate recognition domain ("CRD") structure of ASGR-1 is reported in literature (M. Meier et al, JMB (2000)300, 857-865). The structure of ASGR-1 at a higher resolution than reported, and also when bound to various ligands (e.g., sugars including but not limited to lactose, galactose, and/or GalNAc or glycoproteins displaying such sugars including but not limited to fetuin, orosomucoid and/or alkaline phosphatase) is provided herein (see Example 10 and Figures 18-21 herein).

ASGR-1 is a single pass transmembrane protein and is the major subunit of ASGR. The galactose (Gal) or N-acetylgalactosamine (GalNAc) residues of glycoproteins are exposed by removal of sialic acid by sialidases, hence the term asialoglycoprotein for the ligands of ASGR. Although ASGR expression is detected in other tissues, liver is the predominant site of expression. A circulating form of the receptor, generated from ASGR-1 transcripts lacking exon two, has also been reported. (Liu J, Hu B, Yang Y, et al. A new splice variant of the major subunit of human asialoglycoprotein receptor encodes a secreted form in hepatocytes. PloS one 2010;5:e12934). The del12 and W158X variants are predicted to truncate both the membrane bound and the circulating form of the receptor, and as mentioned above, the W158X variant may generate an unstable and rapidly degraded transcript (and therefore no protein) due to nonsense mediated decay.

The primary reported function of ASGR is to bind and internalize glycoproteins in the circulation that contain terminal galactose or N-acetylgalactosamine residues (asialoglycoproteins), resulting in the clearance of these proteins from the circulation. (Roggenbuck). Reported endogenous ligands include components of the blood coagulation system, such as platelets and Von Willebrand Factor. (Grewal).

As used herein, the terms "ASGR, ASGR-1, and/or ASGR-2 function" or "ASGR, ASGR-1, and/or ASGR-2 activity" includes any biological effect of ASGR, ASGR-1 and/or ASGR- 2. In certain embodiments, ASGR function or activity includes the ability of ASGR to interact or bind to a ligand. In some embodiments, ASGR function or activity is represented by the ability of ASGR to interact or bind to sugars including but not limited to lactose, galactose, and/or GalNAc or glycoproteins displaying such sugars including but not limited to fetuin, orosomucoid and/or alkaline phosphatase. In some embodiments, ASGR function or activity includes any biological activity resulting from ASGR response.

Exemplary activities include, but are not limited to, clearance of asialoglycoproteins from the circulation; clearance of IgA from circulation; removal of apoptotic cells; clearance of low density lipoprotein (LDL) and/or the disposal of cellular fibronectin (Roggenbuck).

Given the location of ASGR on the surface of liver hepatocytes and its implication in hepatocyte entry by certain viruses (Roggenbuck), the receptor has become a target of convenience for therapeutics that require delivery to the liver and internalization into the cells. Examples of these uses include the targeted delivery of doxorubicin to hepatocellular carcinoma (Wei et al., Int J Nanomedicine, 2015, 10:5123-37), gene delivery to hepatocytes (D'Souza et al., J Control Release, 2015, 203:126-39), and targeted delivery of siRNA to hepatocytes (Rajeev et al., Chembiochem, 2015, 16(6):903-8). Methods of modulating platelet uptake by liver sinusoidal endothelial cells and of modulating thrombocytopenia via ASGR modulating compounds, such as siRNA, are disclosed in WO 2011/068798 A1.

Although the ASGR and its ability to mediate endocytosis and degradation of desialylated glycoproteins has been known for nearly 4 decades, the endogenous ligands and the physiological function of the receptor have been difficult to establish. (Weigel PH, Yik JH. Glycans as endocytosis signals: the cases of the asialoglycoprotein and hyaluronan/chondroitin sulfate receptors. Biochimica et biophysica acta 2002;1572:341-63). It has been reported that ASGR-1-/- mice (lacking any ASGR activity) thrive normally and do not accumulate desialylated glycoproteins in their circulation although they are unable to clear exogenously added asialoglycoproteins, suggesting that under normal physiological condition ASGR is not essential for homeostasis of circulating asialoglycoproteins. (Tozawa R, Ishibashi S, Osuga J, et al. Asialoglycoprotein receptor deficiency in mice lacking the major receptor subunit. Its obligate requirement for the stable expression of oligomeric receptor. The Journal of Biological Chemistry 2001;276:12624-8).

In contrast to the ASGR-1 knockout mice which lack an apparent phenotype, a clear physiological role for human ASGR-1 in cardiovascular disease, for example, but not limited to, the regulation of non-HDL levels and modulation of CAD and MI risk has been established herein. The association of del12 and W158X with increased levels of circulating ALP and vitamin B12 has also been demonstrated. Furthermore, as shown herein, disturbing one allele of ASGR-1 appears to have an overall beneficial effect as heterozygotes carriers of del12 live on average 1.5 years longer than non-carriers.

Surprisingly, the various embodiments provided herein demonstrate that the del12 variant and the W158 variant both have an effect on non-HDL levels that is opposite to their effect on ALP and vitamin B12 levels; decreasing non-HDL and increasing ALP and vitamin B12. While not wishing to be bound by any particular hypothesis, it is important to note that the common variant previously described that associates with ALP and LDL cholesterol also has opposing effects on these serum components; hence ASGR-1 may affect the level of these molecules through different mechanisms. It is unlikely that the ALP increase mediated by del12 or W158X reflects an underlying liver disease since other measures of liver function are not affected. Both ALP and the vitamin B12 transporter in the circulation, haptocorrin, are asialylated glycoproteins known to bind ASGR-1 and be cleared from the circulation by the receptor (Tuin A, Huizinga-Van der Vlag A, van Loenen- Weemaes AM, Meijer DK, Poelstra K. On the role and fate of LPS-dephosphorylating activity in the rat liver. American Journal of Physiology Gastrointestinal and Liver Physiology 2006;290:G377-85; Furger E, Fedosov SN, Lildballe DL, et al. Comparison of recombinant human haptocorrin expressed in human embryonic kidney cells and native haptocorrin. PloS one 2012;7:e37421; Burger RL, Schneider RJ, Mehlman CS, Allen RH. Human plasma R-type vitamin B12-binding proteins. II. The role of transcobalamin I, transcobalamin III, and the normal granulocyte vitamin B12-binding protein in the plasma transport of vitamin B12. The Journal of Biological Chemistry 1975;250:7707-13; Steirer LM, Park EI, Townsend RR, Baenziger JU. The asialoglycoprotein receptor regulates levels of plasma glycoproteins terminating with sialic acid alpha2,6-galactose. The Journal of Biological Chemistry 2009;284:3777-83). While not wishing to be bound by any particular hypothesis, the more likely reason for the increased levels of ALP and vitamin B12 in del12 carriers and in W158X carriers is decreased clearance of desialylated forms of these molecules from the circulation, due to reduced number of functional ASGR receptors in del12 carriers and in W158X carriers, suggesting a role for ASGR-1 in maintaining homeostasis of circulating ALP and vitamin B12.

While not wishing to be bound by any particular hypothesis, the decreased levels of non-HDL in del12 carriers and in W158X carriers in the face of reduced ASGR-1 function suggest that ASGR-1 affects non-HDL levels by mechanisms other than direct binding and endocytosis of cholesterol particles. In mice expressing a hypomorphic form of neuraminidase 1 (Neu1), a sialidase that cleaves the sialic acid residues thereby generating substrates for ASGR-1, the LDL receptor (LDLR) is sialylated and this form of the receptor was more stable and took up LDL cholesterol more avidly (LDL levels were decreased in these mice) than the asialylated form of the wild type LDLR (Yang A, Gyulay G, Mitchell M, White E, Trigatti BL Igdoura SA. Hypomorphic sialidase expression decreases serum cholesterol by downregulation of VLDL production in mice Journal of Lipid Research 2012;53:2573-2585). Both ASGR and LDLR are located in clathrin-coated pits on hepatocytes and ASGR may be capable of interacting with the asialylated form of the LDLR and blocking its activity.

Two novel rare variants in ASGR-1 have been identified herein that play a role in cardiovascular disease, including, but not limited to, lowering non-HDL levels and protecting against CAD and MI. These variants disrupt ASGR-1 protein function. Accordingly, the present invention is directed to medical uses of interfering RNA of ASGR-1 and/or ASGR-2, which inhibit ASGR-1 function and/or ASGR-2 function. Also disclosed, but not encompassed by the wording of the claims, are antigen binding proteins that inhibit ASGR function, ASGR-1 function and/or ASGR-2 function.

Interfering RNA

Examples of polynucleotides that can be used to target ASGR-1 and/or ASGR-2 sequences and reduce ASGR-1 and/or ASGR-2 expression include, but are not limited to, antisense oligonucleotides, and RNA interference (RNAi) agents, including short or small interfering RNA (siRNA), short hairpin RNA (shRNA), and microRNA (miRNA). See, for example, U.S. Patent Nos. 6,506,559; 8,394,628; 7,056,704; 7,078,196; 6,107,094; 5,898,031; 6,573,099; and European Patent No. 1,144,623. See also, for example, U.S. patent application publication nos. 2015/0259689; 2015/0197746; 2011/0092565; U.S. Patent Nos. 8,877,917; 8,507,455; and 7,579,451.

A composition for inhibiting the function or expression of a target polynucleotide sequence (e.g. ASGR-1 mRNA sequence, ASGR-2 mRNA sequence) in a mammalian cell, may comprises an agent that provides to a mammalian cell an at least partially double-stranded RNA molecule (e.g., an interfering RNA molecule). A double- stranded RNA molecule may include chemical modifications to ribonucleotides, including modifications to the ribose sugar, base, or backbone components of the ribonucleotides, such as those described herein or known in the art. Any such modifications, as used in a double-stranded RNA molecule (e.g. siRNA, shRNA, or the like), are encompassed by the term "double-stranded RNA" for the purposes of this disclosure. Thus, in general, the term "RNA" may also include RNA-DNA hybrids and polynucleotides comprising one or more modified nucleotides (e.g. nucleotides with modifications at the 2' position of the ribose ring), except where specified otherwise, e.g., where a 2'-OH group of ribose is required for a particular linkage.

In the present invention, interfering RNA of ASGR-1, and/or ASGR-2 is used for treating or preventing a cardiovascular disease in a patient. In some embodiments at least 10% of a partially double-stranded RNA molecule is double-stranded. Alternatively, the double stranded portion of these RNA molecules can be at least 30% of the length of the molecule. In another embodiment, the double stranded portion of these molecules can be at least 50% of the the length of the molecule. In still another embodiment, the double stranded portion of these molecules can be at least 70% of the length of the molecule. In another embodiment, the double stranded portion of these molecules can be at least 90% of the length of the molecule. In another embodiment, the molecule can be double stranded over its entire length. Alternatively, the double-stranded portion of these molecules can occur at either or both termini, or in some middle portion of the molecule, if the molecule is linear. Similarly, the double-stranded portion can be in any location if the molecule is circular. In certain embodiments of the present invention, the double-stranded portion of the RNA molecule becomes double-stranded only when the molecule is in the mammalian cell. In still other embodiment of this invention, the partially double-stranded molecule is an RNA/DNA hybrid, for example, a single strand containing RNA and DNA, prepared in vitro; or a duplex of two such single strands or portions thereof. In yet another embodiment, the RNA molecule, made in vivo or in vitro, is a duplex comprised of an RNA single strand and a DNA single strand. In some embodiments, the partially double-stranded RNA molecule comprises a polynucleotide sequence that is substantially homologous to the target polynucleotide sequence in order to effectively reduce or inhibit the function or expression thereof. The necessary homology may be suitably defined by use of a computer algorithm. As known in the art and discussed herein, "homology" or "identity" means the degree of sequence relatedness between two polypeptide or two polynucleotide sequences as determined by the identity of the match between two lengths of such sequences. Both identity and homology can be readily calculated by methods in the prior art [See also, e.g., COMPUTATIONAL MOLECULAR BIOLOGY, Lesk, A. M., ed., Oxford University Press, New York, (1988); BIOCOMPUTING: INFORMATICS AND GENOME PROJECTS, Smith, D. W., ed., Academic Press, New York, (1993); COMPUTER ANALYSIS OF SEQUENCE DATA, PART I, Griffin, A. M., and Griffin, H. G., eds., Humana Press, New Jersey, (1994); SEQUENCE ANALYSIS IN MOLECULAR BIOLOGY, von Heinje, G., Academic Press, (1987); and SEQUENCE ANALYSIS PRIMER, Gribskov, M. and Devereux, J., eds., M Stockton Press, New York, (1991)]. While there exist a number of methods to measure identity and homology between two polynucleotide sequences, the terms "identity", "similarity" and homology are well known to skilled artisans [H. Carillo and D. Lipton, SIAM J. Applied Math., 48:1073 (1988)]. Methods commonly employed to determine identity or homology between two sequences include, but are not limited to, those disclosed in Guide to Huge Computers, Martin J. Bishop, ed., Academic Press, San Diego, 1994, and H. Carillo and D. Lipton, SIAM J. Applied Math., 48:1073 (1988). Preferred methods to determine identity or homology are designed to give the largest match between the two sequences tested. Methods to determine identity and similarity are codified in computer programs. Preferred computer program to determine identity and homology between two sequences include, but are not limited to, the algorithm BESTFIT from the GCG program package [J. Devereux et al., Nucl. Acids Res., 12(1):387 (1984)], the related MACVECTOR program (Oxford), and the FASTA (Pearson) programs. For instance, searches for sequence similarities in databases between significant naturally occurring mammalian polynucleotide sequences and target polynucleotide sequences enable the design of suitable RNA molecules. The algorithm and/or the degree of homology necessary for any particular RNA molecule may be selected by one of skill in the art, depending on the identity of the target, and/or the closeness of homology of the target sequence to any naturally occurring mammalian sequence, which is desired to be left functioning normally.

In some embodiments, the interfering RNA for use for treating or preventing a cardiovascular disease in a patient comprises a double-stranded RNA molecule which comprises two antiparallel strands of contiguous nucleotides that are sufficiently complementary to each other to hybridize to form a duplex region. "Hybridize" or "hybridization" refers to the pairing of complementary polynucleotides, typically via hydrogen bonding (e.g. Watson- Crick, Hoogsteen or reversed Hoogsteen hydrogen bonding) between complementary bases in the two polynucleotides. The strand comprising a region having a sequence that is substantially complementary to a target sequence (e.g. target mRNA) is referred to as the "antisense strand." The "sense strand" refers to the strand that includes a region that is substantially complementary to a region of the antisense strand. In some embodiments, the sense strand may comprise a region that has a sequence that is substantially identical to the target sequence.

As used herein, a first sequence is "complementary" to a second sequence if a polynucleotide comprising the first sequence can hybridize to a polynucleotide comprising the second sequence to form a duplex region under certain conditions, such as physiological conditions. Other such conditions can include moderate or stringent hybridization conditions, which are known to those of skill in the art. A first sequence is considered to be fully complementary (100% complementary) to a second sequence if a polynucleotide comprising the first sequence base pairs with a polynucleotide comprising the second sequence over the entire length of one or both nucleotide sequences without any mismatches. A sequence is "substantially complementary" to a target sequence if the sequence is at least about 80%, 85%, 90%, 95%, 96%, 97%, 98%, 99% or 100% complementary to a target sequence. Percent complementarity can be calculated by dividing the number of bases in a first sequence that are complementary to bases at corresponding positions in a second or target sequence by the total length of the first sequence. A sequence may also be said to be substantially complementary to another sequence if there are no more than 5, 4, 3, or 2 mismatches over a 30 base pair duplex region when the two sequences are hybridized. Generally, if any nucleotide overhangs, as defined herein, are present, the sequence of such overhangs is not considered in determining the degree of complementarity between two sequences. By way of example, a sense strand of 21 nucleotides in length and an antisense strand of 21 nucleotides in length that hybridize to form a 19 base pair duplex region with a 2 nucleotide overhang at the 3' end of each strand would be considered to be fully complementary as the term is used herein.

In some embodiments, a region of the antisense strand comprises a sequence that is fully complementary to a region of the target RNA sequence (e.g. ASGR-1 and/or ASGR-2 mRNA). In such embodiments, the sense strand may comprise a sequence that is fully complementary to the sequence of the antisense strand. In other such embodiments, the sense strand may comprise a sequence that is substantially complementary to the sequence of the antisense strand, e.g. having 1, 2, 3, 4, or 5 mismatches in the duplex region formed by the sense and antisense strands. In certain embodiments, it is preferred that any mismatches occur within the terminal regions (e.g. within 6, 5, 4, 3, or 2 nucleotides of the 5' and/or 3' ends of the strands). In one embodiment, any mismatches in the duplex region formed from the sense and antisense strands occur within 6, 5, 4, 3, or 2 nucleotides of the 5' end of the antisense strand.

In certain embodiments, the sense strand and antisense strand of the double- stranded RNA may be two separate molecules that hybridize to form a duplex region, but are otherwise unconnected. Such double-stranded RNA molecules formed from two separate strands are referred to as "small interfering RNAs" or "short interfering RNAs" (siRNAs).

In other embodiments, the sense strand and the antisense strand that hybridize to form a duplex region may be part of a single RNA molecule, i.e. the sense and antisense strands are part of a self-complementary region of a single RNA molecule. In such cases, a single RNA molecule comprises a duplex region (also referred to as a stem region) and a loop region. The 3' end of the sense strand is connected to the 5' end of the antisense strand by a contiguous sequence of unpaired nucleotides, which will form the loop region. The loop region is typically of a sufficient length to allow the RNA molecule to fold back on itself such that the antisense strand can base pair with the sense strand to form the duplex or stem region. The loop region can comprise from about 3 to about 25, from about 5 to about 15, or from about 8 to about 12 unpaired nucleotides. Such RNA molecules with at least partially self-complementary regions are referred to as "short hairpin RNAs" (shRNAs). The length of a single, at least partially self-complementary RNA molecule can be from about 35 nucleotides to about 100 nucleotides, from about 45 nucleotides to about 85 nucleotides, or from about 50 to about 60 nucleotides and comprise a duplex region and loop region each having the lengths recited herein.

In some embodiments, the double-stranded RNA molecule comprises a sense strand and an antisense strand, wherein the antisense strand comprises a region having a sequence that is substantially or fully complementary to an ASGR-1 messenger RNA (mRNA) sequence and/or ASGR-2 mRNA sequence. As used herein, an "ASGR-1 mRNA sequence" or "ASGR-2 mRNA sequence" refers to any messenger RNA sequence, including splice variants, encoding an ASGR-1 protein or ASGR-2 protein, including ASGR-1 or ASGR-2 protein variants or isoforms from any species (e.g. mouse, rat, non-human primate, human).

The sense strand of the double-stranded RNA molecule typically comprises a sequence that is sufficiently complementary to the sequence of the antisense strand such that the two strands hybridize under physiological conditions to form a duplex region. A "duplex region" refers to the region in two complementary or substantially complementary polynucleotides that form base pairs with one another, either by Watson-Crick base pairing or other hydrogen bonding interaction, to create a duplex between the two polynucleotides. The duplex region of the RNA molecule should be of sufficient length to allow the RNA molecule to enter the RNA interference pathway, e.g. by engaging the Dicer enzyme and/or the RISC complex. For instance, in some embodiments, the duplex region is about 15 to about 30 base pairs in length. Other lengths for the duplex region within this range are also suitable, such as about 15 to about 28 base pairs, about 15 to about 26 base pairs, about 15 to about 24 base pairs, about 15 to about 22 base pairs, about17 to about 28 base pairs, about 17 to about 26 base pairs, about 17 to about 24 base pairs, about 17 to about 23 base pairs, about 17 to about 21 base pairs, about 19 to about 25 base pairs, about 19 to about 23 base pairs, or about 19 to about 21 base pairs. In one embodiment, the duplex region is about 17 to about 24 base pairs in length. In another embodiment, the duplex region is about 19 to about 21 base pairs in length.

For embodiments in which the sense strand and antisense strand are two separate molecules (e.g. RNAi agent is a siRNA), the sense strand and antisense strand need not be the same length as the length of the duplex region. For instance, one or both strands may be longer than the duplex region and have one or more unpaired nucleotides or mismatches flanking the duplex region. Thus, in some embodiments, the double-stranded RNA molecule comprises at least one nucleotide overhang. As used herein, a "nucleotide overhang" refers to the unpaired nucleotide or nucleotides that extend beyond the duplex region at the terminal ends of the strands. Nucleotide overhangs are typically created when the 3’ end of one strand extends beyond the 5’ end of the other strand or when the 5’ end of one strand extends beyond the 3’ end of the other strand. The length of a nucleotide overhang is generally between 1 and 6 nucleotides, 1 and 5 nucleotides, 1 and 4 nucleotides, 1 and 3 nucleotides, 2 and 6 nucleotides, 2 and 5 nucleotides, or 2 and 4 nucleotides. In some embodiments, the nucleotide overhang comprises 1, 2, 3, 4, 5, or 6 nucleotides. In one particular embodiment, the nucleotide overhang comprises 1 to 4 nucleotides. In certain embodiments, the nucleotide overhang comprises 2 nucleotides. The nucleotides in the overhang can be ribonucleotides, deoxyribonucleotides, or modified nucleotides as described herein.

The nucleotide overhang can be at the 5’ end or 3’ end of one or both strands. For example, in one embodiment, the double-stranded RNA molecule comprises a nucleotide overhang at the 5’ end and the 3’ end of the antisense strand. In another embodiment, the double-stranded RNA molecule comprises a nucleotide overhang at the 5’ end and the 3’ end of the sense strand. In some embodiments, the double-stranded RNA molecule comprises a nucleotide overhang at the 5’ end of the sense strand and the 5’ end of the antisense strand. In other embodiments, the double-stranded RNA molecule comprises a nucleotide overhang at the 3’ end of the sense strand and the 3’ end of the antisense strand.

The double-stranded RNA molecules may comprise a single nucleotide overhang at one end of the molecule and a blunt end at the other. A "blunt end" means that the sense strand and antisense strand are fully base-paired at the end of the molecule and there are no unpaired nucleotides that extend beyond the duplex region. In some embodiments, the double-stranded RNA molecule comprises a nucleotide overhang at the 3’ end of the sense strand and a blunt end at the 5’ end of the sense strand and 3’ end of the antisense strand. In other embodiments, the double-stranded RNA molecule comprises a nucleotide overhang at the 3’ end of the antisense strand and a blunt end at the 5’ end of the antisense strand and the 3’ end of the sense strand. In certain embodiments, the double- stranded RNA molecule comprises a blunt end at both ends of the double-stranded RNA molecule. In such embodiments, the sense strand and antisense strand have the same length and the duplex region is the same length as the sense and antisense strands (i.e. the molecule is double-stranded over its entire length).

The sense strand and antisense strand can each independently be about 15 to about 30 nucleotides in length, about 18 to about 28 nucleotides in length, about 19 to about 27 nucleotides in length, about 19 to about 25 nucleotides in length, about 19 to about 23 nucleotides in length, about 21 to about 25 nucleotides in length, or about 21 to about 23 nucleotides in length. In certain embodiments, the sense strand and antisense strand are each about 18, about 19, about 20, about 21, about 22, about 23, about 24, or about 25 nucleotides in length. In some embodiments, the sense strand and antisense strand have the same length but form a duplex region that is shorter than the strands such that the double-stranded RNA molecule has two nucleotide overhangs. For instance, in one embodiment, the double-stranded RNA molecule comprises (i) a sense strand and an antisense strand that are each 21 nucleotides in length, (ii) a duplex region that is 19 base pairs in length, and (iii) nucleotide overhangs of 2 unpaired nucleotides at both the 3’ end of the sense strand and the 3’ end of the antisense strand. In another embodiment, the double-stranded RNA molecule comprises (i) a sense strand and an antisense strand that are each 23 nucleotides in length, (ii) a duplex region that is 21 base pairs in length, and (iii) nucleotide overhangs of 2 unpaired nucleotides at both the 3’ end of the sense strand and the 3’ end of the antisense strand. In other embodiments, the sense strand and antisense strand have the same length and form a duplex region over their entire length such that there are no nucleotide overhangs on either end of the double-stranded molecule. In one such embodiment, the double-stranded RNA molecule is blunt ended and comprises (i) a sense strand and an antisense strand, each of which is 21 nucleotides in length, and (ii) a duplex region that is 21 base pairs in length. In another such embodiment, the double-stranded RNA molecule is blunt ended and comprises (i) a sense strand and an antisense strand, each of which is 23 nucleotides in length, and (ii) a duplex region that is 23 base pairs in length.

In other embodiments, the sense strand or the antisense strand is longer than the other strand and the two strands form a duplex region having a length equal to that of the shorter strand such that the double-stranded RNA molecule comprises at least one nucleotide overhang. For example, in one embodiment, the double-stranded RNA molecule comprises (i) a sense strand that is 19 nucleotides in length, (ii) an antisense strand that is 21 nucleotides in length, (iii) a duplex region of 19 base pairs in length, and (iv) a single nucleotide overhang of 2 unpaired nucleotides at the 3' end of the antisense strand. In another embodiment, the double-stranded RNA molecule comprises (i) a sense strand that is 21 nucleotides in length, (ii) an antisense strand that is 23 nucleotides in length, (iii) a duplex region of 21 base pairs in length, and (iv) a single nucleotide overhang of 2 unpaired nucleotides at the 3' end of the antisense strand.

Off-target toxicity is a constant concern in the development of pharmaceutical products. With interfering RNA agents, the potential exists for homology with certain endogenous polynucleotide sequences that could lead to unintended toxic effects in the patient receiving the interfering RNA. Accordingly, in some embodiments, the RNA molecule comprises a polynucleotide sequence that is also substantially non-homologous to any naturally occurring, normally functioning, and essential mammalian polynucleotide sequence, so that the RNA molecule does not adversely affect the function of any essential naturally occurring mammalian polynucleotide sequence, when used in the medical uses of this invention. Such naturally occurring functional mammalian polynucleotide sequences include mammalian sequences that encode desired proteins, as well as mammalian sequences that are non-coding, but that provide for essential regulatory sequences in a healthy mammal. Preferably, the RNA molecule useful in the medical uses of the invention must be sufficiently distinct in sequence from any mammalian polynucleotide sequence expressed in the target cells (e.g. liver cells) for which the function is intended to be undisturbed after any of the medical uses of this invention are performed. As described for determining the homology to the target sequence above, one of skill in the art may resort to the above-identified computer algorithms to define the essential lack of homology between the RNA molecule polynucleotide sequence and the normal mammalian sequences expressed in the target cells. For example, in a specific embodiment, the homology between the sequence of an RNAi agent and the selected normal sequence expressed in the target cells is less than the homologies of the formulae described above. In some embodiments, there is almost no homology at all between the sequence of an RNAi agent and any normal mammalian sequence.

The double-stranded RNA molecules used in the medical uses of the invention may comprise one or more modified nucleotides. A "modified nucleotide" refers to a nucleotide that has one or more chemical modifications to the nucleoside, nucleobase, pentose ring, or phosphate group. The double-stranded RNA molecules may comprise combinations of modified nucleotides, ribonucleotides, and deoxyribonucleotides. Incorporation of modified nucleotides into one or both strands of double-stranded RNA molecules can improve the in vivo stability of the RNA molecules, e.g., by reducing the molecules' susceptibility to nucleases and other degradation processes. The potency of double-stranded RNA molecules for reducing expression of the target gene can also be enhanced by incorporation of modified nucleotides.

In certain embodiments, the modified nucleotides have a modification of the ribose sugar. These sugar modifications can include modifications at the 2' and/or 5' position of the pentose ring. A 2'-modified nucleotide refers to a nucleotide having a pentose ring with a substituent at the 2' position other than H or OH. Such 2'-modifications include, but are not limited to, 2'-O-alkyl (e.g. O- $C_1$-$C_{10}$ or O-$C_1$-$C_{10}$ substituted alkyl), 2'-O-allyl (O-$CH_2CH=CH_2$), 2'-C-allyl, 2'-fluoro, 2'-O-methyl ($OCH_3$), 2'-O-methoxyethyl (O-$(CH_2)_2OCH_3$), 2'-$OCF_3$, 2'-O$(CH_2)_2SCH_3$, 2'-O-aminoalkyl, 2'-amino (e.g. $NH_2$), 2'-O-ethylamine, and 2'-azido. Modifications at the 5' position of the pentose ring include, but are not limited to, 5'-methyl (R or S); 5'-vinyl, and 5'-methoxy.

The double-stranded RNA molecules employed in the medical uses of the invention may also comprise one or more modified internucleotide linkages. As used herein, the term "modified internucleotide linkage" refers to an internucleotide linkage other than the natural 3' to 5' phosphodiester linkage. In some embodiments, the modified internucleotide linkage is a phosphorous- containing internucleotide linkage, such as a phosphotriester, aminoalkylphosphotriester, an alkylphosphonate (e.g. methylphosphonate, 3' -alkylene phosphonate), a phosphinate, a phosphoramidate (e.g. 3'-amino phosphoramidate and aminoalkylphosphoramidate), a phosphorothioate (P=S), a chiral phosphorothioate, a phosphorodithioate, a thionophosphoramidate, a thionoalkylphosphonate, a thionoalkylphosphotriester, and a boranophosphate. In one embodiment, a modified internucleotide linkage is a 2' to 5' phosphodiester linkage. In other embodiments, the modified internucleotide linkage is a non-phosphorous-containing internucleotide linkage and thus can be referred to as a modified internucleoside linkage. Such non-phosphorous-containing linkages include, but are not limited to, morpholino linkages (formed in part from the sugar portion of a nucleoside); siloxane linkages (-O-Si$(H)_2$-O-); sulfide, sulfoxide and sulfone linkages; formacetyl and thioformacetyl linkages; alkene containing backbones; sulfamate backbones; methylenemethylimino (-$CH_2$-N($CH_3$) -O-$CH_2$-) and methylenehydrazino linkages; sulfonate and sulfonamide linkages; amide linkages; and others having mixed N, O, S and $CH_2$ component parts. In one embodiment, the modified internucleoside linkage is a peptide-based linkage (e.g. aminoethylglycine) to create a peptide nucleic acid or PNA, such as those described in U.S. Patent Nos. 5,539,082; 5,714,331; and 5,719,262. Other suitable modified internucleotide and internucleoside linkages that may be employed in the double-stranded RNA molecules are described in U.S. Patent No. 6,693,187, U.S. Patent No. 9,181,551, U.S. Patent Publication No. 2016/0122761, and Deleavey and Damha, Chemistry and Biology, Vol. 19: 937-954, 2012.

Interfering RNA delivery

The interfering RNA compounds can be administered by any method suitable for administration of nucleic acid agents, such as a DNA vaccine or gene therapy vectors. These methods include gene guns, bio injectors, and skin patches as well as needle-free methods such as the micro particle DNA vaccine technology disclosed in U.S. Pat. No. 6,194,389, and the mammalian transdermal needle-free vaccination with powder-form vaccine as disclosed in U.S. Pat. No. 6,168,587. Additionally, intranasal delivery is possible, as described in, inter alia, Hamajima et al. (1998), Clin. Immunol. Immunopathol., 88(2), 205-10. Liposomes (e.g., as described in U.S. Pat. No. 6,472,375) and microencapsulation can also be used. Biodegradable targetable microparticle delivery systems can also be used (e.g., as described in U.S. Pat. No. 6,471,996).

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Such formulations can be prepared using standard techniques. The materials can also be obtained commercially from, for example, Alza Corporation and Nova Pharmaceuticals, Inc. Liposomal suspensions can also be used as pharmaceutically acceptable carriers. These can be prepared according to methods known to those skilled in the art, for example, as described in U.S. Pat. No. 4,522,811.

The interfering RNA molecule may be conjugated to one or more carbohydrate moieties to optimize one or more properties of the interfering RNA molecule. In many cases, the carbohydrate moiety will be attached to a modified subunit of the interfering RNA molecule or at the 5' or 3' end of one of strands of the interfering RNA molecule. E.g., the ribose sugar of one or more ribonucleotide subunits of an interfering RNA molecule can be replaced with another moiety, e.g., a non-carbohydrate (preferably cyclic) carrier to which is attached a carbohydrate moiety. A cyclic carrier may be a carbocyclic ring system, i.e., all ring atoms are carbon atoms, or a heterocyclic ring system, i.e., one or more ring atoms may be a heteroatom, e.g., nitrogen, oxygen, sulfur. The cyclic carrier may be a monocyclic ring system, or may contain two or more rings, e.g. fused rings. The cyclic carrier may be a fully saturated ring system, or it may contain one or more double bonds.

The carbohydrate moiety may be attached to the polynucleotide via a carrier. The carriers include (i) at least one "backbone attachment point," preferably two "backbone attachment points" and (ii) at least one "tethering attachment point." A "backbone attachment point" as used herein refers to a functional group, e.g. a hydroxyl group, or generally, a bond available for, and that is suitable for incorporation of the carrier into the backbone, e.g., the phosphate, or modified phosphate, e.g., sulfur containing, backbone, of a ribonucleic acid. A "tethering attachment point" (TAP) in some embodiments refers to a constituent ring atom of the cyclic carrier, e.g., a carbon atom or a heteroatom (distinct from an atom which provides a backbone attachment point), that connects a selected moiety. The moiety can be, e.g., a carbohydrate, e.g. monosaccharide, disaccharide, trisaccharide, tetrasaccharide, oligosaccharide and polysaccharide. Optionally, the selected moiety is connected by an intervening tether to the cyclic carrier. Thus, the cyclic carrier will often include a functional group, e.g., an amino group, or generally, provide a bond, that is suitable for incorporation or tethering of another chemical entity, e.g., a ligand to the constituent ring.

In some embodiments the interfering RNA molecule for use in the medical uses of the invention is conjugated to a carbohydrate moiety via a carrier, wherein the carrier can be cyclic group or acyclic group; in specific embodiments, the cyclic group is selected from pyrrolidinyl, pyrazolinyl, pyrazolidinyl, imidazolinyl, imidazolidinyl, piperidinyl, piperazinyl, [1,3]dioxolane, oxazolidinyl, isoxazolidinyl, morpholinyl, thiazolidinyl, isothiazolidinyl, quinoxalinyl, pyridazinonyl, tetrahydrofuryl and decalin; preferably, the acyclic group is selected from serinol backbone or diethanolamine backbone.

Targeting the interfering RNA

Given that ASGR, ASGR-1 and/or ASGR-2 is expressed on the surface of liver cells (e.g. hepatocytes), in certain embodiments, it is desirable to deliver the interfering RNA molecules to those liver cells so that the interfering effect can be exerted specifically within liver cells. Accordingly, in certain embodiments, the interfering RNA molecules are specifically targeted to liver cells using various methodologies known in the art and described herein. For example, in certain embodiments, antigen binding proteins (e.g. antibodies) or other targeting moieties disclosed herein below can be used to specifically target the interfering RNA molecules to the hepatocytes using various different receptors expressed on the surface of hepatocytes. In certain embodiments, the interfering RNA molecules are targeted to liver cells using the surface expressed ASGR, ASGR-1 and/or ASGR-2. In these embodiments, it is envisioned that this can result in a self-regulating system that reduces the amount of RNAi agent delivered to the liver cells as expression of ASGR, ASGR-1, and/or ASGR-2 is reduced due to the effect of the targeted interfering RNA.

A wide variety of targeting moieties can be coupled to the oligonucleotides. In some embodiments, the targeting moieties are coupled, e.g., covalently, either directly or indirectly via an intervening tether.

In some embodiments, a targeting moiety alters the distribution, targeting or lifetime of the molecule into which it is incorporated. In preferred embodiments a targeting moiety provides an enhanced affinity for a selected target, e.g., molecule, cell or cell type, compartment, receptor e.g., a cellular or organ compartment, tissue, organ or region of the body, as, e.g., compared to a species absent such a targeting moiety. Targeting moieties providing enhanced affinity for a selected target are also termed targeting moieties.

Some targeting moieties can have endosomolytic properties. The endosomolytic targeting moieties promote the lysis of the endosome and/or transport of the composition, or its components, from the endosome to the cytoplasm of the cell. The endosomolytic targeting moietymay be a polyanionic peptide or peptidomimetic which shows pH-dependent membrane activity and fusogenicity. In one embodiment, the endosomolytic targeting moiety assumes its active conformation at endosomal pH. The "active" conformation is that conformation in which the endosomolytic targeting moietypromotes lysis of the endosome and/or transport of the composition, or its components, from the endosome to the cytoplasm of the cell. Exemplary endosomolytic targeting moietiesinclude the GALA peptide (Subbarao et al., Biochemistry, 1987, 26: 2964-2972), the EALA peptide (Vogel et al., J. Am. Chem. Soc., 1996, 118: 1581-1586), and their derivatives (Turk et al., Biochem. Biophys. Acta, 2002, 1559: 56-68). In one embodiment, the endosomolytic component may contain a chemical group (e.g., an amino acid) which will undergo a change in charge or protonation in response to a change in pH. The endosomolytic component may be linear or branched.

In certain embodiments, targeting moieties can improve transport, hybridization, and specificity properties and may also improve nuclease resistance of the resultant natural or modified oligoribonucleotide, or a polymeric molecule comprising any combination of monomers described herein and/or natural or modified ribonucleotides.

In some embodiments, targeting moieties in general can include therapeutic modifiers, e.g., for enhancing uptake; diagnostic compounds or reporter groups e.g., for monitoring distribution; cross-linking agents; and nuclease-resistance conferring moieties. General examples include lipids, steroids, vitamins, sugars, proteins, peptides, polyamines, and peptide mimics.

Targeting moieties can include a naturally occurring substance, such as a protein (e.g., human serum albumin (I), low-density lipoprotein (LDL), high-density lipoprotein (HDL), or globulin); a carbohydrate (e.g., a dextran, pullulan, chitin, chitosan, inulin, cyclodextrin or hyaluronic acid); or a lipid. The targeting moiety may also be a recombinant or synthetic molecule, such as a synthetic polymer, e.g., a synthetic polyamino acid, an oligonucleotide (e.g. an aptamer). Examples of polyamino acids include polyamino acid is a polylysine (PLL), poly L-aspartic acid, poly L- glutamic acid, styrene-maleic acid anhydride copolymer, poly(L-lactide-co-glycolied) copolymer, divinyl ether-maleic anhydride copolymer, N-(2-hydroxypropyl)methacrylamide copolymer (HMPA), polyethylene glycol (PEG), polyvinyl alcohol (PVA), polyurethane, poly(2-ethylacryllic acid), N-isopropylacrylamide polymers, or polyphosphazine. Example of polyamines include: polyethylenimine, polylysine (PLL), spermine, spermidine, polyamine, pseudopeptide-polyamine, peptidomimetic polyamine, dendrimer polyamine, arginine, amidine, protamine, cationic lipid, cationic porphyrin, quaternary salt of a polyamine, or an alpha helical peptide.

Targeting moieties can also include other targeting groups, e.g., a cell or tissue targeting agent, e.g., a lectin, glycoprotein, lipid or protein, e.g., an antibody, that binds to a specified cell type such as a kidney cell. A targeting group can be a thyrotropin, melanotropin, lectin, glycoprotein, surfactant protein A, Mucin carbohydrate, multivalent lactose, multivalent galactose, N- acetyl-galactosamine, N-acetyl-gulucosamine multivalent mannose, multivalent fucose, glycosylated polyaminoacids, multivalent galactose, transferrin, bisphosphonate, polyglutamate, polyaspartate, a lipid, cholesterol, a steroid, bile acid, folate, vitamin B12, biotin, an RGD peptide, an RGD peptide mimetic or an aptamer.

Other examples of targeting moieties include dyes, intercalating agents (e.g. acridines), cross-linkers (e.g. psoralene, mitomycin C), porphyrins (TPPC4, texaphyrin, Sapphyrin), polycyclic aromatic hydrocarbons (e.g., phenazine, dihydrophenazine), artificial endonucleases or a chelator (e.g. EDTA), lipophilic molecules, e.g, cholesterol, cholic acid, 199ligonucle acetic acid, 1- pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine) and peptide conjugates (e.g., antennapedia peptide, Tat peptide), alkylating agents, phosphate, amino, mercapto, PEG (e.g., PEG-40K), MPEG, $[MPEG]_2$, polyamino, alkyl, substituted alkyl, radiolabeled markers, enzymes, haptens (e.g. biotin), transport/absorption facilitators (e.g., aspirin, vitamin E, folic acid), synthetic ribonucleases (e.g., imidazole, bisimidazole, histamine, imidazole clusters, acridine- imidazole conjugates, Eu3+ complexes of tetraazamacrocycles), dinitrophenyl, HRP, or AP.

Targeting moieties can be proteins, e.g., glycoproteins, or peptides, e.g., molecules having a specific affinity for a co-moiety, or antigen binding proteins, such as antibodies; e.g., an antibody, that binds to a specified cell type such as a liver hepatocyte. Targeting moieties may also include hormones and hormone receptors. They can also include non-peptidic species, such as lipids, lectins, carbohydrates, vitamins, cofactors, multivalent lactose, multivalent galactose, N-acetyl galactosamine, N-acetyl-gulucosamine multivalent mannose, multivalent fucose, or aptamers. The targeting moiety can be, for example, a lipopolysaccharide.

The targeting moiety can be a substance, e.g, a drug, which can increase the uptake of the interfering RNA molecule into the cell, for example, by disrupting the cell's cytoskeleton, e.g., by disrupting the cell's microtubules, microfilaments, and/or intermediate filaments. The drug can be, for example, taxon, vincristine, vinblastine, cytochalasin, nocodazole, japlakinolide, latrunculin A, phalloidin, swinholide A, indanocine, or myoservin.

The targeting moiety can increase the uptake of the interfering RNA molecule into the cell by activating an inflammatory response, for example. Exemplary targeting moieties that would have such an effect include tumor necrosis factor alpha (TNFalpha), interleukin-1 beta, or gamma interferon.

In one embodiment, the targeting moiety is a lipid or lipid-based molecule. Such a lipid or lipid-based molecule preferably binds a serum protein, e.g., human serum albumin (I). A serum protein binding targeting moiety, in certain embodiments, allows for distribution of the conjugate to a target tissue, e.g., a non-kidney target tissue of the body. For example, the target tissue can be the liver, including hepatocytes or parenchymal cells of the liver. Other molecules that can bind serum proteins can also be used as targeting moieties. For example, naproxen or aspirin can be used. A lipid or lipid-based targeting moiety can (a) increase resistance to degradation of the conjugate, (b) increase targeting or transport into a target cell or cell membrane, and/or (c) can be used to adjust binding to a serum protein.

A lipid based targeting moiety can be used to modulate, e.g., control the binding of the conjugate to a target tissue. For example, a lipid or lipid-based targeting moiety that binds to a serum protein more strongly will be less likely to be targeted to the kidney and therefore less likely to be cleared from the body. A lipid or lipid-based targeting moiety that binds to a serum protein less strongly can be used to target the conjugate to the kidney, if so desired.

In one embodiment, the lipid based targeting moiety binds human serum albumin. In a specific embodiment, it binds human serum albumin with a sufficient affinity such that the conjugate will be preferably distributed to a non-kidney tissue. In certain embodiments, it is preferred that the affinity not be so strong that the human serum albumin targeting moiety binding cannot be reversed.

In another preferred embodiment, the lipid based targeting moiety binds human serum albumin weakly or not at all, such that the conjugate will be preferably distributed to the kidney. Other moieties that target to kidney cells can also be used in place of or in addition to the lipid-based targeting moiety.

In another embodiment, the targeting moiety is for example a vitamin, e.g., a vitamin, which is taken up by a target cell, e.g., a proliferating cell. Exemplary vitamins include vitamin A, E, and K. Other exemplary vitamins include B vitamins, e.g., folic acid, B12, riboflavin, biotin, pyridoxal or other vitamins or nutrients taken up by cells. Also included are low density lipoprotein (LDL) and high-density lipoprotein (HDL).

In another embodiment, the targeting moiety is a cell-permeation agent, preferably a helical cell-permeation agent. In some embodiments, the agent is amphipathic. An exemplary agent is a peptide such as tat or antennopedia. If the agent is a peptide, it can be modified, including a peptidylmimetic, invertomers, non-peptide or pseudo-peptide linkages, and use of D-amino acids. The helical agent is preferably an alpha-helical agent, which preferably has a lipophilic and a lipophobic phase.

The targeting moiety can be a peptide or peptidomimetic. A peptidomimetic (also referred to herein as an oligopeptidomimetic) is a molecule capable of folding into a defined three- dimensional structure similar to a natural peptide. The peptide or peptidomimetic moiety can be about 5-50 amino acids long, e.g., about 5, 10, 15, 20, 25, 30, 35, 40, 45, or 50 amino acids long. A peptide or peptidomimetic can be, for example, a cell permeation peptide, cationic peptide, amphipathic peptide, or hydrophobic peptide (e.g., consisting primarily of Tyr, Trp or Phe). The peptide moiety can be a dendrimer peptide, constrained peptide or crosslinked peptide. In another alternative, the peptide moiety can include a hydrophobic membrane translocation sequence (MTS). An exemplary hydrophobic MTS-containing peptide is RFGF having the amino acid sequence AAVALLPAVLLALLAP. An RFGF analogue (e.g., amino acid sequence AALLPVLLAAP) containing a hydrophobic MTS can also be a targeting moiety. The peptide moiety can be a "delivery" peptide, which can carry large polar molecules including peptides, oligonucleotides, and protein across cell membranes. For example, sequences from the HIV Tat protein (GRKKRRQRRRPPQ) and the Drosophila Antennapedia protein (RQIKIWFQNRRMKWKK) have been found to be capable of functioning as delivery peptides. A peptide or peptidomimetic can be encoded by a random sequence of DNA, such as a peptide identified from a phage-display library, or one-bead-one-compound (OBOC) combinatorial library (Lam et al., Nature, 354:82-84, 1991). In some embodiments, the peptide or peptidomimetic tethered to an interfering RNA molecule via an incorporated monomer unit is a cell targeting peptide such as an arginine-glycine-aspartic acid (RGD)-peptide, or RGD mimic. A peptide moiety can range in length from about 5 amino acids to about 40 amino acids. The peptide moieties can have a structural modification, such as to increase stability or direct conformational properties. Any of the structural modifications described below can be utilized. An RGD peptide can facilitate targeting of an interfering RNA molecule to cells of a variety of other tissues, including the lung, kidney, spleen, or liver (Aoki et al., Cancer Gene Therapy 8:783-787, 2001). The RGD peptide can be linear or cyclic, and can be modified, e.g., glycosylated or methylated to facilitate targeting to specific tissues. For example, a glycosylated RGD peptide can deliver an interfering RNA molecule to a cell expressing $\alpha V\beta_3$ (Haubner et al., Jour. Nucl. Med., 42:326-336, 2001). Peptides that target markers enriched in proliferating cells can be used. E.g., RGD containing peptides and peptidomimetics can target cells, in particular cells that exhibit an integrin. Thus, one could use RGD peptides, cyclic peptides containing RGD, RGD peptides that include D-amino acids, as well as synthetic RGD mimics. In addition to RGD, one can use other moieties that target the integrin ligand.

A "cell permeation peptide" is capable of permeating a cell, e.g., a microbial cell, such as a bacterial or fungal cell, or a mammalian cell, such as a human cell. A microbial cell- permeating peptide can be, for example, an α-helical linear peptide (e.g., LL-37 or Ceropin P1), a disulfide bond-containing peptide (e.g., α-defensin, β-defensin or bactenecin), or a peptide containing only one or two dominating amino acids (e.g., PR-39 or indolicidin). A cell permeation peptide can also include a nuclear localization signal (NLS). For example, a cell permeation peptide can be a bipartite amphipathic peptide, such as MPG, which is derived from the fusion peptide domain of HIV- 1 gp41 and the NLS of SV40 large T antigen (Simeoni et al., Nucl. Acids Res. 31:2717-2724, 2003).

In one embodiment, a targeting peptide can be an amphipathic α-helical peptide. Exemplary amphipathic α-helical peptides include, but are not limited to, cecropins, lycotoxins, paradaxins, buforin, CPF, bombinin-like peptide (BLP), cathelicidins, ceratotoxins, S. clava peptides, hagfish intestinal antimicrobial peptides (HFIAPs), magainines, brevinins-2, dermaseptins, melittins, pleurocidin, H.sub.2A peptides, Xenopus peptides, esculentinis-1, and caerins.

Peptide and peptidomimetic targeting moietiesinclude those having naturally occurring or modified peptides, e.g., D or L peptides; α, β, or γ peptides; N-methyl peptides; azapeptides; peptides having one or more amide, i.e., peptide, linkages replaced with one or more urea, thiourea, carbamate, or sulfonyl urea linkages; or cyclic peptides.

The targeting moiety can be any moiety that is capable of targeting a specific receptor. Examples are: folate, GalNAc, galactose, mannose, mannose-6P, clusters of sugars such as GalNAc cluster, mannose cluster, galactose cluster, or an apatamer. A cluster is a combination of two or more sugar units. The targeting moieties also include integrin receptor moieties, chemokine receptor moieties, transferrin, biotin, serotonin receptor moieties, PSMA, endothelin, GCPII, somatostatin, LDL and HDL moieties. The targeting moieties can also be based on nucleic acid, e.g., an aptamer. The aptamer can be unmodified or have any combination of modifications disclosed herein.

Other exemplary endosomal release agents include imidazoles, poly or oligoimidazoles, PEIs, peptides, fusogenic peptides, polycaboxylates, polyacations, masked oligo or poly cations or anions, acetals, polyacetals, ketals/polyketyals, orthoesters, polymers with masked or unmasked cationic or anionic charges, dendrimers with masked or unmasked cationic or anionic charges.

Pharmacokinetic ("PK") modulators include lipophiles, bile acids, steroids, phospholipid analogues, peptides, protein binding agents, PEG, vitamins etc. Examplary PK modulators include, but are not limited to, cholesterol, fatty acids, cholic acid, lithocholic acid, dialkylglycerides, diacylglyceride, phospholipids, sphingolipids, naproxen, ibuprofen, vitamin E, biotin etc. Oligonucleotides that comprise a number of phosphorothioate linkages are also known to bind to serum protein, thus short oligonucleotides, e.g. oligonucleotides of about 5 bases, 10 bases, 15 bases or 20 bases, comprising multiple of phosphorothioate linkages in the backbaone are also amenable to the present invention as targeting moieties(e.g. as PK modulating moieties). In addition, aptamers that bind serum components (e.g. serum proteins) are also amenable to the present invention as PK modulating moieties.

When two or more targeting moieties are present, the targeting moieties can all have same properties, all have different properties or some targeting moieties have the same properties while others have different properties. For example, a targeting moiety can have targeting properties, have endosomolytic activity and/or have PK modulating properties. In certain embodiments, all the targeting moieties have different properties.

In some embodiments, a targeting moiety can be conjugated to nucleobases, sugar moieties, or internucleosidic linkages of nucleic acid molecules. Conjugation to purine nucleobases or derivatives thereof can occur at any position including, endocyclic and exocyclic atoms. In some embodiments, the 2-, 6-, 7-, or 8-positions of a purine nucleobase are attached to a conjugate moiety. Conjugation to pyrimidine nucleobases or derivatives thereof can also occur at any position. In some embodiments, the 2-, 5-, and 6-positions of a pyrimidine nucleobase can be substituted with a conjugate moiety. Conjugation to sugar moieties of nucleosides can occur at any carbon atom. Example carbon atoms of a sugar moiety that can be attached to a conjugate moiety include the 2', 3', and 5' carbon atoms. The 1' position can also be attached to a conjugate moiety, such as in an abasic residue. Internucleosidic linkages can also bear conjugate moieties. For phosphorus-containing linkages (e.g., phosphodiester, phosphorothioate, phosphorodithiotate, phosphoroamidate, and the like), the conjugate moiety can be attached directly to the phosphorus atom or to an O, N, or S atom bound to the phosphorus atom. For amine- or amide-containing internucleosidic linkages (e.g., PNA), the conjugate moiety can be attached to the nitrogen atom of the amine or amide or to an adjacent carbon atom.

It is envisioned that any suitable targeting moiety in the field of RNA interference may be used, although the targeting moiety is typically a carbohydrate e.g. monosaccharide (such as GalNAc), disaccharide, trisaccharide, tetrassaccharide, polysaccharide. Linkers that conjugate the targeting moiety to the nucleic acid include those discussed herein. For example, the targeting moiety can be one or more GalNAc derivatives attached through a bivalent or trivalent branched linker.

In certain embodiments, cleavable linking groups are utilized. A cleavable linking group is one which is sufficiently stable outside the cell, but which upon entry into a target cell is cleaved to release the two parts the linker is holding together. In one embodiment, the cleavable linking group is cleaved at least 10 times or more, and in some embodiments, at least 100 times faster in the target cell or under a first reference condition (which can, e.g., be selected to mimic or represent intracellular conditions) than in the blood of a subject, or under a second reference condition (which can, e.g., be selected to mimic or represent conditions found in the blood or serum).

Cleavable linking groups are susceptible to cleavage agents, e.g., pH, redox potential or the presence of degradative molecules. Generally, cleavage agents are more prevalent or found at higher levels or activities inside cells than in serum or blood. Examples of such degradative agents include: redox agents which are selected for particular substrates or which have no substrate specificity, including, e.g., oxidative or reductive enzymes or reductive agents such as mercaptans, present in cells, that can degrade a redox cleavable linking group by reduction; esterases; endosomes or agents that can create an acidic environment, e.g., those that result in a pH of five or lower; enzymes that can hydrolyze or degrade an acid cleavable linking group by acting as a general acid, peptidases (which can be substrate specific), and phosphatases.

A cleavable linkage group, such as a disulfide bond can be susceptible to pH. The pH of human serum is 7.4, while the average intracellular pH is slightly lower, ranging from about 7.1-7.3. Endosomes have a more acidic pH, in the range of 5.5-6.0, and lysosomes have an even more acidic pH at around 5.0. Some linkers will have a cleavable linking group that is cleaved at a preferred pH, thereby releasing the cationic lipid from the moiety inside the cell, or into the desired compartment of the cell.

A linker can include a cleavable linking group that is cleavable by a particular enzyme. The type of cleavable linking group incorporated into a linker can depend on the cell to be targeted. For example, liver targeting targeting moietiescan be linked to the cationic lipids through a linker that includes an ester group. Liver cells are rich in esterases, and therefore the linker will be cleaved more efficiently in liver cells than in cell types that are not esterase-rich. Other cell-types rich in esterases include cells of the lung, renal cortex, and testis. Linkers that contain peptide bonds can be used when targeting cell types rich in peptidases, such as liver cells and synoviocytes.

In general, the suitability of a candidate cleavable linking group can be evaluated by testing the ability of a degradative agent (or condition) to cleave the candidate linking group. It will also be desirable to also test the candidate cleavable linking group for the ability to resist cleavage in the blood or when in contact with other non-target tissue. Thus one can determine the relative susceptibility to cleavage between a first and a second condition, where the first is selected to be indicative of cleavage in a target cell and the second is selected to be indicative of cleavage in other tissues or biological fluids, e.g., blood or serum. The evaluations can be carried out in cell free systems, in cells, in cell culture, in organ or tissue culture, or in whole animals. It may be useful to make initial evaluations in cell-free or culture conditions and to confirm by further evaluations in whole animals. In some embodiments, useful candidate compounds are cleaved at least 2, 4, 10 or 100 times faster in the cell (or under in vitro conditions selected to mimic intracellular conditions) as compared to blood or serum (or under in vitro conditions selected to mimic extracellular conditions).

In some embodiments, redox cleavable linking groups are utilized. Redox cleavable linking groups are cleaved upon reduction or oxidation. An example of reductively cleavable linking group is a disulphide linking group (--S-S--). To determine if a candidate cleavable linking group is a suitable "reductively cleavable linking group," or for example is suitable for use with a particular interfering RNA molecule and particular targeting agent one can look to methods described herein. For example, a candidate can be evaluated by incubation with dithiothreitol (DTT), or other reducing agent using reagents know in the art, which mimic the rate of cleavage which would be observed in a cell, e.g., a target cell. The candidates can also be evaluated under conditions which are selected to mimic blood or serum conditions. In a specific embodiment, candidate compounds are cleaved by at most 10% in the blood. In some embodiments, useful candidate compounds are degraded at least 2, 4, 10 or 100 times faster in the cell (or under in vitro conditions selected to mimic intracellular conditions) as compared to blood (or under in vitro conditions selected to mimic extracellular conditions). The rate of cleavage of candidate compounds can be determined using standard enzyme kinetics assays under conditions chosen to mimic intracellular media and compared to conditions chosen to mimic extracellular media.

In yet some embodiments, phosphate-based cleavable linking groups are cleaved by agents that degrade or hydrolyze the phosphate group. An example of an agent that cleaves phosphate groups in cells are enzymes such as phosphatases in cells. Examples of phosphate-based linking groups are -O-P(O)(Ork)-O--, --O-P(S)(Ork)-O--, --O-P(S)(SRk)-O--, --S-P(O) (Ork)-O--, --O-P(O)(Ork)-S--, --S-P(O)(Ork)-S--, --O-P(S)(Ork)-S--, --S-P(S)(Ork)-O--, --O-P(O)(Rk)-O--, O-P(S)(Rk)-On, --S-P(O)(Rk)-O--, --S-P(S)(Rk)-O--, --S-P(O)(Rk)-S--, --O-P(S)(Rk)-S--. Specific embodiments include -O-P(O)(OH)-O--, --O-P(S)(OH)-O--, --O-P(S)(SH)-O--, --S- P(O)(OH)-O--, --O-P(O)(OH)-S--, --S-P(O)(OH)-S--, --O-P(S)(OH)-S--, --S-P(S)(OH)-O--, --O P(O)(H)-O--, --O-P(S)(H)-O--, --S-P(O)(H)-O--, --S-P(S)(H)-O--, --S-P(O)(H)-S--, --O-P(S)(H)- S--. Another specific embodiment is -O-P(O)(OH)-O--. These candidates can be evaluated using methods analogous to those described above.

In some embodiments, acid cleavable linking groups, which are linking groups that are cleaved under acidic conditions, are envisioned. In some embodiments acid cleavable linking groups are cleaved in an acidic environment with a pH of about 6.5 or lower (e.g., about 6.0, 5.5, 5.0, or lower), or by agents such as enzymes that can act as a general acid. In a cell, specific low pH organelles, such as endosomes and lysosomes can provide a cleaving environment for acid cleavable linking groups. Examples of acid cleavable linking groups include but are not limited to hydrazones, esters, and esters of amino acids. Acid cleavable groups can have the general formula -C==NN--, C(O)O, or -OC(O). A specific embodiment is when the carbon attached to the oxygen of the ester (the alkoxy group) is an aryl group, substituted alkyl group, or tertiary alkyl group such as dimethyl pentyl or t-butyl. These candidates can be evaluated using methods analogous to those described above.

In some embodiments, ester-based cleavable linking groups, which are cleaved by enzymes such as esterases and amidases in cells, are envisioned. Examples of ester-based cleavable linking groups include but are not limited to esters of alkylene, alkenylene and alkynylene groups. Ester cleavable linking groups have the general formula -C(O)O--, or -OC(O)--. These candidates can be evaluated using methods analogous to those described above.

In yet further embodiments, peptide-based cleavable linking groups, which are cleaved by enzymes such as peptidases and proteases in cells, are envisioned. Peptide-based cleavable linking groups are peptide bonds formed between amino acids to yield oligopeptides (e.g., dipeptides, tripeptides etc.) and polypeptides. Peptide-based cleavable groups do not include the amide group (- C(O)NH--). The amide group can be formed between any alkylene, alkenylene or alkynelene. A peptide bond is a special type of amide bond formed between amino acids to yield peptides and proteins. The peptide based cleavage group is generally limited to the peptide bond (i.e., the amide bond) formed between amino acids yielding peptides and proteins and does not include the entire amide functional group. Peptide-based cleavable linking groups have the general formula - $NHCHR^AC(O)NHCHR^BC(O)$--, where $R^A$ and $R^B$ are the R groups of the two adjacent amino acids. These candidates can be evaluated using methods analogous to those described above. As used herein, "carbohydrate" refers to a compound which is either a carbohydrate per se made up of one or more monosaccharide units having at least 6 carbon atoms (which may be linear, branched or cyclic) with an oxygen, nitrogen or sulfur atom bonded to each carbon atom; or a compound having as a part thereof a carbohydrate moiety made up of one or more monosaccharide units each having at least six carbon atoms (which may be linear, branched or cyclic), with an oxygen, nitrogen or sulfur atom bonded to each carbon atom. Representative carbohydrates include the sugars (mono-, di-, tri- and oligosaccharides containing from about 4-9 monosaccharide units), and polysaccharides such as starches, glycogen, cellulose and polysaccharide gums.

## Synthesis of interfering RNA

The interfering RNA molecules that can be employed in the medical uses of the present invention can readily be made using techniques known in the art, for example, using conventional RNA solid phase synthesis. See, for example, U.S. Patent No. 8,877,917. The polynucleotides of the double-stranded RNA molecules can be assembled on a suitable nucleic acid synthesizer utilizing standard nucleotide or nucleoside precursors (e.g. phosphoramidites). Automated nucleic acid synthesizers are sold commercially by several vendors, including DNA/RNA synthesizers from Applied Biosystems (Foster City, CA), MerMade synthesizers from BioAutomation (Irving, TX), and OligoPilot synthesizers from GE Healthcare Life Sciences (Pittsburgh, PA).

The 2' silyl protecting group can be used in conjunction with acid labile dimethoxytrityl (DMT) at the 5' position of ribonucleosides to synthesize oligonucleotides via phosphoramidite chemistry. Final deprotection conditions are known not to significantly degrade RNA products. All syntheses can be conducted in any automated or manual synthesizer on large, medium, or small scale. The syntheses may also be carried out in multiple well plates or glass slides.

The 2'-O-silyl group can be removed via exposure to fluoride ions, which can include any source of fluoride ion, e.g., those salts containing fluoride ion paired with inorganic counterions e.g., cesium fluoride and potassium fluoride or those salts containing fluoride ion paired with an organic counterion, e.g., a tetraalkylammonium fluoride. A crown ether catalyst can be utilized in combination with the inorganic fluoride in the deprotection reaction. Preferred fluoride ion source are tetrabutylammonium fluoride or aminehydrofluorides (e.g., combining aqueous HF with triethylamine in a dipolar aprotic solvent, e.g., dimethylformamide).

The choice of protecting groups for use on the phosphite triesters and phosphotriesters can alter the stability of the triesters towards fluoride. Methyl protection of the phosphotriester or phosphitetriester can stabilize the linkage against fluoride ions and improve process yields.

Since ribonucleosides have a reactive 2' hydroxyl substituent, it can be desirable to protect the reactive 2' position in RNA with a protecting group that is orthogonal to a 5'-O- dimethoxytrityl protecting group, e.g., one stable to treatment with acid. Silyl protecting groups meet this criterion and can be readily removed in a final fluroide deprotection step that can result in minimal RNA degradation.

Tetrazole catalysts can be used in the standard phosphoramidite coupling reaction. Preferred catalysts include e.g., tetrazole, S-ethyl-tetrazole, p-nitrophenyltetrazole.

See also, for example, Trufert et al., Tetrahedron, 52:3005, 1996; and Manoharan, "Oligonucleotide Conjugates in Antisense Technology," in Antisense Drug Technology, ed. S. T. Crooke, Marcel Dekker, Inc., 2001. The protected monomer compounds can be separated from a reaction mixture and further purified by a method such as column chromatography, high pressure liquid chromatography, or recrystallization. As can be appreciated by the skilled artisan, further methods of synthesizing the compounds of the formulae herein will be evident to those of ordinary skill in the art. Additionally, the various synthetic steps may be performed in an alternate sequence or order to give the desired compounds. Other synthetic chemistry transformations, protecting groups (e.g., for hydroxyl, amino, etc. present on the bases) and protecting group methodologies (protection and deprotection) useful in synthesizing the compounds described herein are known in the art and include, for example, those such as described in R. Larock, Comprehensive Organic Transformations, VCH Publishers (1989); T. W. Greene and P. G. M. Wuts, Protective Groups in Organic Synthesis, 2d. Ed., John Wiley and Sons (1991); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995), and subsequent editions thereof.

Medical Uses

Antigen binding proteins or antibodies binding to ASGR-1, and/or ASGR-2, or interfering RNA (e.g., siRNA or shRNA) of ASGR-1, and/or ASGR-2, as disclosed herein, may be used for treating a human subject (antigen binding proteins and antibodies are not encompassed by the wording of the claims) The antigen binding proteins may be monoclonal antibodies, such as human antibodies or humanized antibodies (not encompassed by the wording of the claims). As used herein the term "subject" refers to a mammal, including humans, and can be used interchangeably with the term "patient".

Given the results of the Icelandic study presented in the examples below, there need not be any particular further manipulation downstream in a host receiving a therapy involving administering the ASGR inhibitor (e.g., antigen binding proteins or antibodies or RNAi) to the host. That is, in some embodiments, the antibody (or RNAi) need simply be one or more of the antibodies (or RNAi) described herein, which binds to (and inhibits) ASGR (such as ASGR1), and be administered in an amount, and at a frequency sufficient to reduce the risk of cardiovascular disease, myocardial infarction, or other disorders provided herein.

In the present invention, interfering RNA (RNAi) of ASGR-1, and/or ASGR-2 is used for treating or preventing a cardiovascular disease in a patient. In some embodiments, the RNAi is administered in an amount sufficient to result in a lowering of non-HDL cholesterol. In some embodiments, the RNAi is administered in an amount sufficient to result in lowering LDL cholesterol. While not intended to be limiting unless expressed otherwise, below is a description of various embodiments through which ASGR can have an impact on various disorders, and thus, how the various RNAi provided herein (which can inhibit (e.g., reduce) ASGR function) can have an impact on the various disorders provided herein.

In some embodiments, the RNAi operates through ASGR's role in platelet clearance. Inhibiting (e.g., reducing) the receptor results in a reduction in clearance of old platelets. Such older platelets do not coagulate as well as new platelets and as a result, the blood is thinner. As a result, plaques can lessen and there can be a positive impact (e.g., stroke is lessened) for the subject.

In some embodiments, the RNAi binds to ASGR to alter inflammation. For example, reducing the ASGR-1 receptor results in a modification of the immune response. Normally, there can be an increase in proinflammatory cytokines. These proinflammatory cytokines are circulating in the native state (one where the ASGR1 receptor is not reduced). However, ALP (alkaline phosphatase) can have an anti inflammatory role thereby reducing inflammation and coagulopathy systemically. In some embodiments, the mechanism of action involves reducing ASGR1 which increases ALP and therefore reduces inflammation.

In some embodiments, and without intending to be limited by theory (unless expressed otherwise), the RNAi can reduce an activity due to ASGR interacting with one or more other molecules, either directly or indirectly. For example, various embodiments for various proteins are provided herein in Examples 18 and 19. As noted above, this selection of proteins can also be useful for determining the effectiveness of the RNAi and/or identification of a subject who can respond to the RNAi by monitoring one or more of these proteins as a Cardiovascular Disease marker. Thus, these markers are useful as markers and, without intending to be limited by theory, in some embodiments, one or more of the proteins disclosed below is the protein through which (directly or indirectly) ASGR1 modulation achieves its benefit for one or more of the disorders provided herein, including cardiovascular disease.

In addition to the marker proteins described in Examples 18 and 19 herein (which also allow for various mechanisms of action and monitoring the effectiveness of various ASGR inhibitors (e.g., antigen binding proteins or antibodies or RNAi) and dosage regimes), the following proteins of interest are those that interact with ASGR, and ASGR-1 in particular, directly by binding to them. Thus, these are additional interactions that can be inhibited (e.g., reduced) for various embodiments provided herein, by RNAi. While not intending to be limited by theory (unless explicitly stated otherwise), ASGR-1's binding to one or more of the following proteins can be inhibited (e.g., reduced) by using an ASGR-1 inhibitor (e.g., antigen binding protein or antibody or RNAi) provided herein that inhibits (e.g., reduces) the noted binding. While in some embodiments, the protein interactions are contemplated as resulting mechanisms of action that occur downstream from when ASGR levels are effectively reduced by RNAi, the following list is a list of proteins that directly bind to ASGR1, and thus whose direct binding to ASGR-1 can be inhibited (e.g., reduced) by one or more of the RNAi provided herein. In some embodiments, the RNAi inhibits (e.g., reduces) ASGR-1's binding to one or more of: Alpha-2-HS-glycoprotein (aka Fetuin A) (see Tozawa et al, J Biol Chem (2001) 276:12624- 12628); Asialoglycoprotein receptor 1 (see Stockert et al (1977) Science 197:667-668), Orosomucoid (aka alpha-1-acid glycoprotein) (see Tozawa et al, J Biol Chem (2001) 276:12624-12628), Alkaline phosphatase, (see Hardonk MJ, Scholtens HB. Histochemistry. 1980;69(3):289-97 and Scholtens HB, Meijer DK, Hardonk MJ. Liver. 1982 Mar;2(1):14-21), LDL and chylomicrons (Windler et al Biochem J (1991) 276:79-87), Fibronectin (see Rotundo et al Hepatology (1998) 28:475-485), and IgA (see Stockert et al PNAS (1982) 79:6229-6231). In some embodiments, the RNAi binds to ASGR and inhibits (e.g., reduces) ASGR's interaction with a molecule that has a terminal gal or galNAc, including, but not limited to protein ligands, synthetic polysaccharides, solid substrates, etc. In some embodiments, the RNAi inhibits (e.g., reduces) ASGR1 's ability to bind to an asialylated molecule. The present invention provides an interfering RNA of ASGR-1, and/or ASGR-2 for use for treating or preventing a cardiovascular disease in a patient In some embodiments, the cardiovascular disease is coronary artery disease or myocardial infarction. In some embodiments, the interfering RNA is an inhibitor of ASGR-1. In some embodiments, the interfering RNA Ais an inhibitor of ASGR-2. In some embodiments, the interfering RNA is an inhibitor of ASGR-1 and ASGR-2. In some embodiments, the relative risk reduction of a cardiovascular event is at least about 5%, at least about 10%, at least about 15%, at least about 20%, at least about 25%, at least about 30%, at least about 35%, at least about 40%, at least about 45%, at least about 50%, at least about 55%, at least about 60% in the patient. Some non-limiting examples of cardiovascular disease include atherosclerotic diseases, such as, for example, coronary heart disease, coronary artery disease, peripheral arterial disease, stroke (ischaemic and hemorrhagic), angina pectoris, cerebrovascular disease, acute coronary syndrome, and myocardial infarction. In some embodiments, the interfering RNA of ASGR-1, and/or ASGR-2 are used in reducing the risk of: nonfatal heart attacks, fatal and non-fatal strokes, certain types of heart surgery, hospitalization for heart failure, chest pain in patients with heart disease, and/or cardiovascular events because of established heart disease such as prior heart attack, prior heart surgery, and/or chest pain with evidence of clogged arteries. In some embodiments, the interfering RNA of ASGR-1, and/or ASGR-2 are used to reduce the risk of recurrent cardiovascular events.

In some embodiments, the invention provides medical uses of decreasing the risk of acquiring coronary artery disease or having an MI comprising administering to a patient in need thereof a therapeutically effective dose of an interfering RNA of ASGR-1, and/or ASGR-2 as described herein. In some embodiments, the interfering RNA is an inhibitor of ASGR-1. In some embodiments, the interfering RNA is an inhibitor of ASGR-2. In some embodiments, the interfering RNA is an inhibitor of ASGR-1 and ASGR-2. In some embodiments, the relative risk reduction of coronary artery disease or MI is at least about 5%, at least about 10%, at least about 15%, at least about 20%, at least about 25%, at least about 30%, at least about 35%, at least about 40%, at least about 45%, at least about 50%, at least about 55%, at least about 60% in the patient.

In some embodiments, the invention provides an interfering RNA of ASGR-1, and/or ASGR-2 for use for reducing blood LDL cholesterol levels in a patient. In some embodiments, the interfering RNA is an inhibitor of ASGR-1. In some embodiments, the interfering RNA is an inhibitor of ASGR-2. In some embodiments, the interfering RNA is an inhibitor of ASGR-1 and ASGR-2. In some embodiments, the blood LDL cholesterol level in the patient is reduced by at least about 15%, as compared to a predose level of blood LDL cholesterol in the patient. In some embodiments, the blood LDL cholesterol level of said patient is lowered by at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, or at least about 90% as compared to a predose level of blood LDL cholesterol in the patient.

In some embodiments, the invention provides an interfering RNA of ASGR-1, and/or ASGR-2 for use for reducing non-HDL cholesterol levels in a patient. In some embodiments, the interfering RNA is an inhibitor of ASGR-1. In some embodiments, the interfering RNA is an inhibitor of ASGR-2. In some embodiments, the interfering RNA is an inhibitor of ASGR-1 and ASGR-2. In some embodiments, the non-HDL cholesterol level in the patient is reduced by at least about 5%, as compared to a predose level of non-HDL cholesterol in the patient. In some embodiments the non-HDL cholesterol level of said patient is lowered by at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, or at least about 90% as compared to a predose level of non-HDL cholesterol in the patient.

In some embodiments, the invention provides an interfering RNA of ASGR-1, and/or ASGR-2 for use for increasing ALP levels in a patient In some embodiments, the interfering RNA is an inhibitor of ASGR-1. In some embodiments, the interfering RNA is an inhibitor of ASGR-2. In some embodiments, the interfering RNA is an inhibitor of ASGR-1 and ASGR-2. In some embodiments, the ALP level in the patient is increased by at least about 30%, as compared to a predose level of ALP in the patient. In some embodiments, the ALP level of said patient is increased by at least about at least about 35%, at least about 40%, at least about 45%, at least about 50%, at least about 55%, at least about 60%, at least about 65%, at least about 70%, at least about 75%, at least about 80%, at least about 85%, or at least about 90% as compared to a predose ALP level in the patient. In some embodiments, ALP levels are increased at least about 1.25x, 1.5x, 2x, 2.5x, 3x, 3.5x, 4x, 4.5x, and 5x over pretreatment.

In some embodiments, the interfering RNA are used for antagonizing ASGR-1 and/or ASGR-2 in a patient. In some embodiments, the interfering RNA is an inhibitor of ASGR-1. In some embodiments, the interfering RNA is an inhibitor of ASGR-2. In some embodiments, the interfering RNA is an inhibitor of ASGR-1 and ASGR-2.

In the present invention, an interfering RNA of ASGR-1, and/or ASGR-2 is used for treating or preventing a cardiovascular disease in a patient. In some embodiments, the interfering RNA is an inhibitor of ASGR-1. In some embodiments, the interfering RNA is an inhibitor of ASGR-2. In some embodiments, the interfering RNA is an inhibitor of ASGR-1 and ASGR-2. In some embodiments, the relative risk reduction of a cardiovascular event is at least about 5%, at least about 10%, at least about 15%, at least about 20%, at least about 25%, at least about 30%, at least about 35%, at least about 40%, at least about 45%, at least about 50%, at least about 55%, at least about 60% in the patient.

The term "treatment" encompasses alleviation of at least one symptom or other embodiment of a disorder, or reduction of disease severity, and the like. An antigen binding protein, in particular a human antibody, need not effect a complete cure, or eradicate every symptom or manifestation of a disease, to constitute a viable therapeutic agent. As is recognized in the pertinent field, drugs employed as therapeutic agents may reduce the severity of a given disease state, but need not abolish every manifestation of the disease to be regarded as useful therapeutic agents. Simply reducing the impact of a disease (for example, by reducing the number or severity of its symptoms, or by increasing the effectiveness of another treatment, or by producing another beneficial effect), or reducing the likelihood that the disease will occur or worsen in a subject, is sufficient. One embodiment of the invention is directed to a medical use comprising administering to a patient an interfering RNA in an amount and for a time sufficient to induce a sustained improvement over baseline of an indicator that reflects the severity of the particular disorder.

The term "prevention" encompasses prevention of at least one symptom or other embodiment of a disorder, and the like. A prophylactically administered treatment incorporating an antigen binding protein, in particular a human antibody according to the present invention, need not be completely effective in preventing the onset of a condition in order to constitute a viable prophylactic agent. Simply reducing the likelihood that the disease will occur or worsen in a subject, is sufficient.

The term "non-HDL cholesterol" encompasses all cholesterol-containing proatherogenic lipoproteins, including LDL cholesterol, very-low-density lipoprotein, intermediate- density lipoprotein, lipoprotein(a), and chylomicron. Non-HDL cholesterol levels are calculated by subtracting HDL cholesterol levels from total cholesterol levels.

As is understood in the pertinent field, pharmaceutical compositions comprising the antigen binding proteins and/or interfering RNA may be administered to a subject in a manner appropriate to the indication and the composition. Pharmaceutical compositions may comprise the human antibodies disclosed herein. Pharmaceutical compositions may comprise interfering RNA disclosed herein. Pharmaceutical compositions may be administered by any suitable technique, including but not limited to parenterally, topically, or by inhalation. If injected, the pharmaceutical composition can be administered, for example, via intra-articular, intravenous, intramuscular, intralesional, intraperitoneal or subcutaneous routes, by bolus injection, or continuous infusion. Delivery by inhalation includes, for example, nasal or oral inhalation, use of a nebulizer, inhalation of the antigen binding protein in aerosol form, and the like. Other alternatives include oral preparations including pills, syrups, or lozenges.

Advantageously, the antigen binding proteins or interfering RNA may be administered in the form of a composition comprising one or more additional components such as a physiologically acceptable carrier, excipient or diluent. Optionally, the composition additionally comprises one or more physiologically active agents. The composition may comprises one, two, three, four, five, or six physiologically active agents in addition to one or more antigen binding proteins (e.g, human antibodies) or interfering RNA.

Kits for use by medical practitioners are provided herein, including one or more antigen binding proteins or interfering RNA and a label or other instructions for use in treating any of the conditions discussed herein (not encompassed by the wording of the claims). The kit may includes a sterile preparation of one or more human antibodies, or one or more interfering RNA which may be in the form of a composition as disclosed herein, and may be in one or more vials.

Dosages and the frequency of administration may vary according to such factors as the route of administration, the particular antigen binding proteins or interfering RNA employed, the nature and severity of the disease to be treated, whether the condition is acute or chronic, and the size and general condition of the subject. Appropriate dosages can be determined by procedures known in the pertinent art, e.g. in clinical trials that may involve dose escalation studies.

An antigen binding protein, e.g., monoclonal antibodies, or interfering RNA may be administered, for example, once or more than once, e.g., at regular intervals over a period of time. For example, an antigen binding protein or interfering RNA is administered over a period of at least once a month or more, e.g., for one, two, or three months or even indefinitely. For treating chronic conditions, long-term treatment is generally most effective. However, for treating acute conditions, administration for shorter periods, e.g. from one to six weeks, may be sufficient. In general, the antigen binding protein or interfering RNA is administered until the patient manifests a medically relevant degree of improvement over baseline for the chosen indicator or indicators.

One example of therapeutic regimens provided herein comprise subcutaneous injection of an antigen binding protein or interfering RNA once a week, or once every two weeks, or once every month, once every other month, once every three months, once every six months or longer, at an appropriate dosage, to treat a condition in which it is desired to target cells expressing ASGR, ASGR-1 and/or ASGR-2. Weekly or monthly administration of antigen binding protein could be continued until a desired result is achieved, e.g., the subject's symptoms subside. Treatment may resume as needed, or, alternatively, maintenance doses may be administered.

In some embodiments, one or more of the markers in Tables 18.1, 18.2, 19.3, and 19.4 can be used to determine whether or not the amount of RNAi administered according to the medical use of the present invention is sufficient for its intended therapeutic application. In some embodiments, when one or more of the alterations in protein level, for the proteins outlined in one or more of Tables 18.1, 18.2, 19.3, and 19.4 changes in response to administering the RNAi, the RNAi is having an effect in the host. In some embodiments, the amount is sufficient when it alters the level of non-HDL cholesterol to a desired amount or reduces it by a desired amount. In some embodiments, the markers used can be one or more of those in one or more of Tiers 1, 2, 3, 4, and 5 of Table 19.4. In some embodiments, the markers used can be one or more of those in one or more of Tiers 1 and 5 of Table 19.4.

Combination therapies

Particular embodiments of the medical uses of the invention involve the use of at least one interfering RNA and one or more other therapeutics useful for treating or preventing cardiovascular disease. In one embodiment, interfering RNA is administered alone or in combination with other agents useful for treating the condition with which the patient is afflicted. Examples of such agents include both proteinaceous and non-proteinaceous drugs. When multiple therapeutics are co-administered, dosages may be adjusted accordingly, as is recognized in the pertinent art. "Co-administration" and combination therapy are not limited to simultaneous administration, but also include treatment regimens in which an antigen binding protein is administered at least once during a course of treatment that involves administering at least one other therapeutic agent to the patient. In certain embodiments, an interfering RNA is administered prior to the administration of at least one other therapeutic agent. In certain embodiments, an interfering RNA is administered concurrent with the administration of at least one other therapeutic agent. In certain embodiments, an interfering RNA is administered subsequent to the administration of at least one other therapeutic agent.

In one embodiment, the at least one interfering RNA is administered to a subject in combination with an anti-PCSK9 antibody (e.g., Repatha®', Praluent®, bococizumab). In another embodiment, the at least one interfering RNA is administered to a subject in combination with at least one other cholesterol-lowering (serum and/or total body cholesterol) agent. In some embodiments, the agents that increase the expression of LDLR, have been observed to increase serum HDL levels, lower LDL levels or lower triglyceride levels. Exemplary agents include, but are not limited to, statins (e.g., atorvastatin, cerivastatin, fluvastatin, lovastatin, mevastatin, pitavastatin, pravastatin, rosuvastatin, simvastatin), Nicotinic acid (Niacin) (NIACOR, NIASPAN (slow release niacin), SLO-NIACIN (slow release niacin)), Fibric acid (LOPID (Gemfibrozil), TRICOR (fenofibrate), Bile acid sequestrants (QUESTRAN (cholestyramine), colesevelam (WELCHOL), COLESTID (colestipol)), Cholesterol absorption inhibitors (ZETIA (ezetimibe)), combining nicotinic acid with statin (ADVICOR (LOVASTATIN and NIASPAN), combining a statin with an absorption inhibitor (VYTORIN (ZOCOR and ZETIA) and/or lipid modifying agents. In some embodiments, the at least one interfering RNA is combined with PPAR gamma agonsits, PPAR alpha/gamma agonists, squalene synthase inhibitors, CETP inhibitors, anti-hypertensives, anti-diabetic agents (such as sulphonyl ureas, insulin, GLP-1 analogs, DDPIV inhibitors), ApoB modulators, MTP inhibitoris and/or arteriosclerosis obliterans treatments. In some embodiments, the at least one interfering RNA is combined with an agent that increases the level of LDLR protein in a subject, such as statins, certain cytokines like oncostatin M, estrogen, and/or certain herbal ingredients such as berberine. In some embodiments, the at least one interfering RNA is combined with an agent that increases serum cholesterol levels in a subject (such as certain anti-psycotic agents, certain HIV protease inhibitors, dietary factors such as high fructose, sucrose, cholesterol or certain fatty acids and certain nuclear receptor agonists and antagonists for RXR, RAR, LXR, FXR). The combination of the two can allow for the undesirable side-effects of other agents to be mitigated by the antigen binding protein or interfering RNA.

Diagnostic Uses (not according to the claimed invention)

The antigen binding proteins disclosed herein may be useful for detecting the presence of ASGR, ASGR-1 and/or ASGR-2 in a biological sample. The term "detecting" as used herein encompasses quantitative or qualitative detection. A biological sample may comprises a cell or tissue. Such tissues may include tissues that express ASGR, ASGR-1 and/or ASGR-2 at higher levels relative to other tissues.

Disclosed herein is a method of detecting the presence of ASGR, ASGR-1 and/or ASGR-2 in a biological sample. The method may comprise contacting the biological sample with an antigen binding protein under conditions permissive for binding of an antigen binding protein to ASGR, ASGR-1 and/or ASGR-2, and detecting whether a complex is formed between the antigen binding protein and ASGR, ASGR-1 and/or ASGR-2.

Also disclosed herein is a method of diagnosing a disorder associated with increased or decreased expression of ASGR, ASGR-1 and/or ASGR-2. The method may comprises contacting a test cell with an antigen binding protein; determining the level of expression (either quantitatively or qualitatively) of ASGR, ASGR-1 and/or ASGR-2 by the test cell by detecting binding of the antigen binding protein to ASGR, ASGR-1 and/or ASGR-2; and comparing the level of expression of ASGR, ASGR-1 and/or ASGR-2 by the test cell with the level of expression of ASGR, ASGR-1 and/or ASGR-2 by a control cell (e.g., a normal cell of the same tissue origin as the test cell or a cell that expresses ASGR, ASGR-1 and/or ASGR-2 at levels comparable to such a normal cell), wherein a higher or lower level of expression of ASGR, ASGR-1 and/or ASGR-2 by the test cell as compared to the control cell indicates the presence of a disorder associated with increased or decreased expression of ASGR, ASGR-1 and/or ASGR-2. The test cell may be obtained from an individual suspected of having a disorder associated with increased or decreased expression of ASGR, ASGR-1 and/or ASGR-2.

A method of diagnosis or detection, such as those described above, may comprises detecting binding of an antigen binding protein to ASGR, ASGR-1 and/or ASGR-2 expressed on the surface of a cell or in a membrane preparation obtained from a cell expressing ASGR, ASGR-1 and/or ASGR-2 on its surface. The method may comprise contacting a cell with an antigen binding protein under conditions permissive for binding of an antigen binding protein to ASGR, ASGR-1 and/or ASGR-2, and detecting whether a complex is formed between the antigen binding protein and ASGR, ASGR-1 and/or ASGR-2 on the cell surface. An exemplary assay for detecting binding of an antigen binding protein to ASGR, ASGR-1 and/or ASGR-2 expressed on the surface of a cell is a "FACS" assay.

Certain other methods can be used to detect binding of antigen binding proteins disclosed herein to ASGR, ASGR-1 and/or ASGR-2. Such methods include, but are not limited to, antigen-binding assays that are well known in the art, such as western blots, radioimmunoassays, ELISA (enzyme linked immunosorbent assay), "sandwich" immunoassays, immunoprecipitation assays, fluorescent immunoassays, protein A immunoassays, and immunohistochemistry (IHC).

Antigen binding proteins disclosed herein may be labeled. Labels include, but are not limited to, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes $^{32}P$, $^{14}C$, $^{125}I$, $^{3}H$, and $^{131}I$, fluorophores such as rare earth chelates or fluorescein and its derivatives, rhodamine and its derivatives, dansyl, umbelliferone, luceriferases, e.g., firefly luciferase and bacterial luciferase (U.S. Pat. No. 4,737,456), luciferin, 2,3-dihydrophthalazinediones, horseradish peroxidase (HRP), alkaline phosphatase, beta-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

The antigen binding proteins disclosed herein may be immobilized on an insoluble matrix. Immobilization entails separating the antigen binding protein from any ASGR, ASGR-1 and/or ASGR-2 that remains free in solution. This conventionally is accomplished by either insolubilizing the antigen binding protein before the assay procedure, as by adsorption to a water-insoluble matrix or surface (see, e.g., Bennich et al., U.S. Pat. No. 3,720,760), or by covalent coupling (for example, using glutaraldehyde cross-linking), or by insolubilizing the antigen binding protein after formation of a complex between the antigen binding protein and ASGR, ASGR-1 and/or ASGR-2, e.g., by immunoprecipitation.

The invention having been described, the following examples are offered by way of illustration, and not limitation.

Numerous sequences have been provided herein. Where there is a discrepancy in the sequences, the sequences in the tables presented within the figures control, unless there is an indication otherwise. If there is any unintended difference between the same consensus sequences, the consensus sequences as provided in the figures (from the tables within the figures) will control (unless indicated otherwise). For any further descrepancies (rather than just alternative sequences) the sequences within Tables 1-7 will control, unless designated otherwise. The figures contain multiple sequences, sequence alignments and sequence components of various nucleic and amino acid sequences. The present specification references this information in terms of the designated tables and/or the designated figures. Either reference (via figure or table) can be used and either designation (figure or table) will indicate the alternative designation as well, where appropriate. Thus, FIG. 48 designates Table 1, FIG. 51 designates Table 4, FIG. 55 designates Tables 19A, 19B, 19C, 20A, 20B, and 20C, FIG. 57 designates Tables 49-134, and vice versa. As such, any discussion herein in regard to the above figures or tables is interchangeable with respect to the "table" or "figure" nomenclature.

## EXAMPLES

### Example 1- Identification of rare sequence variants that disrupt ASGR-1 function and lower non-HDL cholesterol and protect against coronary artery disease

The level of circulating non-high density lipoprotein (non-HDL) cholesterol is heritable and strongly correlated with the risk of coronary artery disease (CAD) and myocardial infraction (MI). Whole-genome sequencing offers the potential to search for rare sequence variants that have large effects on serum lipid levels and hence the risk of cardiovascular disease, such as CAD and MI.

METHODS

Study Participants:

Details of the population sample sets from Iceland, Denmark and The Netherlands, used to measure the various lipids traits (non-HDL cholesterol, HDL cholesterol, LDL cholesterol and triglycerides), alkaline phosphatase (ALP), ferritin, and vitamin B12, are outlined in Table 1.2. The dataset for ferritin is not shown. The coronary artery disease case-control sample sets that were a part of the study are outlined in Table 1.1.

Icelandic study population

Study participants were enrolled as part of various genetics programs at deCODE. Blood lipid levels (total cholesterol, non-high density lipoprotein cholesterol (non-HDL-C), low density lipoprotein cholesterol (LDL-C), high density lipoprotein cholesterol (HDL-C) and triglycerides), alkaline phosphatase and vitamin B12 levels were obtained from three of the largest laboratories in Iceland: 1) Landspitali - The National University Hospital of Iceland (LUH), Reykjavik (measurements performed between the years 1993 and 2012, hospitalized and ambulatory patients), 2) The Laboratory in Mjodd (RAM), Reykjavik (measurements performed between 2004 and 2012, ambulatory patients) and 3) Akureyri Hospital, The Regional Hospital in North Iceland, Akureyri (performed between 2004 and 2010, hospitalized and ambulatory patients). Information on the participants is outlined in Table 1.2. Lipid levels were adjusted for sex, year of birth and age at measurement, lipid lowering medication and measurement site, using the average of multiple measurements for an individual, and then normalized to a standard normal distribution using quantile normalization. To obtain effect estimates in mmol/L the estimates from the regression analysis were multiplied by the estimated standard deviation of lipid level in the population. Given their approximately log-normal distribution, triglyceride levels were log-transformed before adjustment and the corresponding effect estimates are presented as percentage change instead of units of mmol/L. The total number of individuals with non-HDL cholesterol, LDL cholesterol, HDL cholesterol and triglycerides in Iceland is shown in the Table 1.3 below. For each lipid, the number of chip-typed and directly imputed individuals and those with familial imputations is also shown.

Table 1.3: Lipid levels of Icelandic Study Participants

| | Non-HDL-C | LDL-C | HDL-C | Triglycerides |
|---|---|---|---|---|
| **Total number** | 119,146 | 53,841 | 119,514 | 80,111 |
| Direct imputation | 69,277 | 51,029 | 69,414 | 59,678 |
| Familial imputation | 49,869 | 2,812 | 50,100 | 20,433 |

The total number of Icelandic individuals with lipid values used in the study and the breakdown into those that were chip-typed and directly imputed (Direct imputation) and those that were first and second degree relatives of chip-typed individuals and had their genotypes inferred based on genealogy (Familial imputation).

Non-HDL cholesterol was obtained by subtracting HDL cholesterol from total cholesterol and measures the amount of cholesterol carried within all atherogenic lipoprotein particles (VLDL, IDL, LDL, chylomicrons and Lp(a)). The LDLcholesterol was calculated, using the Friedewald equation (for triglyceride levels < 4.00 mmol/L) (Friedewald, W. T., Levy, R. I. & Fredrickson, D. S. Estimation of the concentration of low-density lipoprotein cholesterol in plasma, without use of the preparative ultracentrifuge. Clin. Chem. 18, 499-502 (1972)). Total cholesterol and HDL-cholesterol values are a mixture of fasting and non-fasting values, whereas triglycerides are fasting values exclusively.

Coronary artery disease (CAD) was defined as a) individuals in the MONICA registry who suffered myocardial infarction (MI) before the age of 75 in Iceland between 1981 and 2002 and satisfied the MONICA criteria (Gudbjartsson, et al., Large-scale whole-genome sequencing of the Icelandic population. Nature genetics 2015), b) subjects with CAD discharge diagnoses (ICD 9 codes 410.*, 411.*, 412.*, 414.* or ICD 10 codes I20.0, I21.*, I22.*. I23.*, I24.*, I25.*) from LUH, c) subjects diagnosed with significant angiographic CAD (see defined below) identified from a nationwide clinical registry of coronary angiography and percutaneous coronary interventions at LUH between the years 1987 and 2012, d) subjects undergoing coronary artery bypass grafting (CABG) procedures at LUH between the years 2002 and 2011 or e) cause of death or contributing cause of death listed as MI or CAD (ICD 9 or 10 codes) on death registries between the years 1996 and 2009. Coronary angiograms in the nationwide registry were evaluated by an interventional cardiologist. Patients were considered to have significant angiographic CAD if one or more of the three major epicardial coronary vessels or the left main coronary artery was found to have at least 50% stenosis by visual estimation.

## Non-Icelandic study populations

Characteristics of the non-Icelandic sample sets are outlined in Table 1.1 and Table 1.2. All the studies outlined in Tables 1.1 and 1.2 were approved by appropriate bioethics and/or data protection authorities. For samples from the Nijmegen Biomedical Study, Netherlands, the lipid values (namely, total cholesterol, HDL-cholesterol and triglycerides) were all non-fasting values. For samples from the Danish Inter99 and Addition studies, the lipid values were all fasting values. All participating subjects donating biological samples signed informed consents. Personal identities of the phenotypes and biological samples were encrypted by a third party system provided by the Icelandic Data Protection Authority.

## Data Generation and Analysis

*Whole-genome sequencing, SNP calling, and imputation*

The Icelandic samples were genotyped using Illumina microarrays (Samani NJ et al., Genomewide association analysis of coronary artery disease. The New England journal of medicine 2007;357:443-53). The whole-genomes of 2,636 Icelanders were sequenced using the standard TruSeq methodology (Illumina) to a mean depth of at least 10X (median 20X)(Samani NJ et al., Genomewide association analysis of coronary artery disease. The New England journal of medicine 2007;357:443-53). For improved sequencing coverage of the GC-rich intron 4 in *ASGR-1* gene, the whole-genome sequence data generated for 738 Icelanders was analyzed using TruSeq PCR- free method from Illumina (mean depth of 30X). The del12 variant in intron 4 of *ASGR-1* was detected in this dataset.

*Single-Track assay SNP and microsatellite genotyping:*

We performed single SNP genotyping of rs186021206, using the Centaurus (Nanogen) platform (Gretarsdottir S, et al., Genome-wide association study identifies a sequence variant within the DAB2IP gene conferring susceptibility to abdominal aortic aneurysm. Nature genetics 2010;42:692). The del12 variant was genotyped using a PCR based method with the following primers: forward primer (NED labelled) 5'-TTCATCTTTCTTCCCACATTGC-3' (SEQ ID NO: 32600), reverse primer 5'-GGGCCTGAGAGAGACGTTCA-3' (SEQ ID NO: 32601). An internal size standard was added to the resulting PCR products and the fragments were separated and detected on an Applied Biosystems model 3730 sequencer, using in-house Allele Caller software.

*Statistical analyses:*

Associations between imputed genotypes and serum lipids (non-HDL cholesterol, HDL cholesterol, LDL cholesterol and triglycerides), ALP, ferritin and vitamin B12 levels in the Icelandic dataset were tested using a generalized linear regression, assuming an additive genetic model (Samani NJ et al., Genomewide association analysis of coronary artery disease. The New England journal of medicine 2007;357:443-53; and Olsen MH, et al., N-terminal pro-brain natriuretic peptide, but not high sensitivity C-reactive protein, improves cardiovascular risk prediction in the general population. European heart journal 2007;28:1374-81). For the Icelandic dataset, logistic regression was used to test for association between the del12 variant and coronary artery disease and myocardial infarction, treating the disease status as the response and the number of copies of del12 an individual carries as the explanatory variable. Coronary artery disease case-control association analysis for the non-Icelandic sample sets was done using the NEMO software (Jorgensen AB, et al., Loss-of-function mutations in APOC3 and risk of ischemic vascular disease. The New England journal of medicine 2014;371:32-41) assuming a multiplicative risk model. Results for the Icelandic and the non-Icelandic sample sets were combined using a Mantel-Haenszel fixed effects model. To estimate the effect of the del12 variant on myocardial infarction-free survival, Kaplan-Meier curves were estimated for survival to first myocardial infarction in heterozygous carriers and non-carriers (Hoogendoorn EH, et al., Thyroid function and prevalence of anti-thyroperoxidase antibodies in a population with borderline sufficient iodine intake: influences of age and sex. Clinical chemistry 2006;52:104-11) by dividing the corresponding chi-square statistic by 1.36 for non-HDL cholesterol, 1.57 for HDL cholesterol, 1.40 for triglycerides, 1.53 for ALP, 1.30 for vitamin B12, 1.71 for coronary artery disease and 1.48 for myocardial infarction.

To obtain a reliable imputation of the del12 variant, 3,799 Icelandic individuals were genotyped for the del12 variant and those genotypes were used as a training set for imputation of the del12 variant into the rest of the Icelandic population. The imputation information for del12 was 0.99.

The Icelandic samples were genotyped using Illumina microarrays as described above (Gudbartssoon, DF, et al., Large Scale whole-genome sequencing of the Icelandic population. Nature Genetics 2015). The whole-genomes of 2,636 Icelanders were sequenced using Illumina standard TruSeq methodology to a mean depth of at least 10X (median 20X) (Di Angelantonio E, et al., Major lipids, apolipoproteins, and risk of vascular disease. Jama 2009;302:1993-2000). A total of 35.5 million autosomal SNPs and INDEL's were identified using the Genome Analysis Toolkit version 2.3.9.

Information about haplotype sharing was used to improve variant genotyping, taking advantage of the fact that all sequenced individuals had also been chip-typed and long-range-phased. Variants were annotated using Ensembl release 72 and Variant Effect Predictor (VEP) version 2.8. Of the 35.5 million sequence variants found, 25.3 million variants passed the quality threshold and were imputed into 104,220 Icelanders who had been genotyped using Illumina chips. Additionally, using the Icelandic genealogy, genotype probabilities were calculated for 294,212 untyped individuals who are first and second degree relatives of the chip-typed individuals born after 1880 (Gudbartssoon, DF, et al., Large Scale whole-genome sequencing of the Icelandic population. Nature Genetics 2015). The informativeness of genotype imputation (imputation information) was estimated by the ratio of the variance of imputed expected allele counts and the variance of the actual allele counts:

$$\frac{Var(E(\theta|chip\ data))}{Var(\theta)},$$

where $\theta$ is the allele count. $Var(E(\theta|chipdata))$ was estimated by the observed variance of the imputed expected counts and $Var(\theta)$ was estimated by $p(1 - p)$, where $p$ is the allele frequency.

For improved sequencing coverage of the GC-rich intron 4 in *ASGR-1* gene, whole- genome sequence ("WGS") data generated for 738 Icelanders was analyzed using TruSeq PCR-free method from Illumina (mean depth of 30X). This PCR-free method gave much better coverage of GC- rich regions including the *ASGR-1* intron 4. The del12 variant in intron 4 of *ASGR-1* was detected in five individuals in this dataset.

To provide improved coverage of the associated region (1Mb centered on ASGR- 1), a new dataset was analyzed that included an additional 5,817 WGS individuals (on top of the 2,636 WGS Icelanders). These additional individuals were sequenced with either Illumina TruSeq PCR free or TrueSeq Nano methods. These Illumina TrueSeq methods give enhanced sequence coverage as compared to the standard Illumina TrueSeq method (median sequencing depth 32X). The identified sequence variants were imputed into 150,656 Icelandic chipped-typed individuals, and with the use of genealogy information, into primary and secondary relatives of chip-typed individuals that were un typed. In this expanded dataset, we identified another rare (0.027%), novel variant, W158X. The W158X variant is a four bp INDEL in exon 7 of ASGR-1 (NM_001671.4:c.469_472dupAACT) that causes frameshift and introduction of premature stop codon at amino acid 158 out of the 291 amino acid full length protein (NP_001662.1:p.Trp158X). A total of 345 individuals were Sanger-sequenced based on the imputation predicted carriers and non-carriers of c.469_472dupAACT. In this dataset, 79 c.469_472dupAACT carriers and 270 non-carriers were identified. This genotype data was then used to re-impute the variant into the Icelandic dataset. For non-HDL cholesterol, a larger sample set (n=136,261) was used in the association analysis outlined in Tables 1.4A and 1.4B.

Associations between imputed genotypes and serum lipids (non-HDL cholesterol, HDL cholesterol, LDL cholesterol and triglycerides), ALP and vitamin B12 levels in the Icelandic dataset were tested using a generalized linear regression, assuming an additive genetic model (Gudbjartsson DF, et al., Large-scale whole-genome sequencing of the Icelandic population. Nature genetics 2015; and Steinthorsdottir V, et al., Identification of low-frequency and rare sequence variants associated with elevated or reduced risk of type 2 diabetes. Nature genetics 2014;46:294-8). All measurements were adjusted for age, sex and measurement site, and average was taken over the available measurements after adjustment and inverse normal transformation. The lipid measurements were further adjusted for statin use. Removing individual known to take lipid lowering drugs in the Icelandic dataset did not alter the association with non-HDL cholesterol. The effect, in standardized units, changed from -0.29 (95% CI -0.38, -0.20; $P = 4.0 \times 10^{-11}$) to -0.30 (-0.39, -0.21; $P = 6.7 \times 10^{-11}$). This amounted to excluding 16,295 individuals, out of 119,146 individuals with non-HDL cholesterol information.

The lipid, ALP and vitamin B12 measurements from the Danish Inter99 study, ADDITION Denmark screening cohort, and the Nijmegen biomedical study, were adjusted and transformed in the same way and tested for association with allele count of del12 and rs186021206 using the linear regression implemented in the R software package. Results from the different populations were combined using the inverse variance fixed-effects method with METAL (Willer CJ, et al., METAL: fast and efficient meta-analysis of genomewide association scans. Bioinformatics 2010;26:2190-1). Effect estimates from the regression analysis are expressed in units of standard deviation (SD). To obtain effect estimates in mg/dL for non-HDL cholesterol, LDL cholesterol and HDL cholesterol, the estimates from the regression analysis were multiplied by the estimated SD of the population distributions. Triglyceride, ALP and vitamin B12 levels were log-transformed before adjustment as their distributions are approximately log-normal, and the corresponding effect estimates are presented as percentage change.

For the Icelandic dataset, logistic regression was used to test for association between the del12 variant and coronary artery disease and myocardial infarction, treating the disease status as the response and the number of copies of the deletion an individual carries as the explanatory variable. Other available individual characteristics that correlate with disease status were also included in the model as nuisance variables (Gudbjartsson DF, et al., Large-scale whole-genome sequencing of the Icelandic population. Nature genetics 2015). Coronary artery disease case-control association analysis for the non-Icelandic sample sets was done using the NEMO software (Gretarsdottir S, et al., The gene encoding phosphodiesterase 4D confers risk of ischemic stroke. Nature genetics 2003;35:131-8) assuming a multiplicative risk model. Results for the Icelandic and the non-Icelandic sample sets were combined using a Mantel-Haenszel fixed-effects model. Heterogeneity in the effect estimate was tested assuming that the estimated odds ratios for different groups follows a log-normal distribution using a likelihood ratio test with degrees of freedom equal to the number of groups compared minus one.

To estimate the effect of the del12 variant on myocardial infarction free survival, we estimated the Kaplan-Meier curves for survival to first myocardial infarction in heterozygous carriers and non-carriers stratified by sex and tested the difference in survival between carriers and non-carriers using the Cox proportional model. The analysis was performed using the survival library in the R software package. The survival analysis was based on 87,718 chip genotyped Icelanders and 44,655 Icelandic first and second degree relatives of chip typed individuals after restricting our analysis to those who lived to be at least 40 years old. Death was treated as a censoring event.

## Functional Characterization of the del12 Variant in ASGR-1

*cDNA preparation, amplification, Sanger sequencing and next generation sequencing:*

RNA was isolated from blood samples from carriers and non-carriers of del12. Following cDNA generation, the region between exon 3 and 5 in *ASGR-1* was PCR amplified and the identified PCR products (two for del12 carriers and one for non-carriers) were Sanger sequenced using standard methodology to determine the sequence difference between the identified cDNA products. To quantify the ratio between the two amplified cDNA PCR products, they were sequenced using Illumina MiSeq instrument coupled with the MiSeq v2 reagent kit.

*Western blot analysis:*

The wild type *ASGR-1* cDNA and *ASGR-1* cDNA with the 22bp deletion were transiently overexpressed in HeLa cells to determine if *ASGR-1* transcripts with the 22 bp deletion generated stable truncated ASGR-1 protein as evaluated by western blot analysis.

RNA was isolated from blood samples using a Qiagen RNA maxi kit. Concentration and quality of the RNA was determined using an Agilent 2100 Bioanalyzer (Agilent Technologies), all samples had RIN values over 7. Following cDNA generation, the region between exon 3 and 5 in *ASGR-1* was PCR amplified using the Advantage® 2 Polymerase kit (Clontech) with the forward primer, CACTCAGGTCCTTCTGCTGTTTC (SEQ ID NO: 32602) and the reverse primer, 5'-ACCTCGCCTCCTCCTGCT-3' (SEQ ID NO: 32603). The resulting products were resolved on agarose gel and the identified PCR products (two for del12 carriers and one for non- carriers) were Sanger sequenced using standard methodology to determine the sequence difference between the identified cDNA products. To quantitate the ratio between the two amplified cDNA PCR products, they were sequenced using Illumina MiSeq instrument coupled with the MiSeq v2 reagent kit.

## Transient overexpression of wild type and mutated ASGR-1 harbouring the 22bp deletion at the end of exon 4 in HeLa cells.

*Generation and cloning of wild type and mutated ASGR-1 cDNA:*

cDNA of ASGR-1 was obtained by PCR on human liver marathon ready cDNA (BD biosciences Clontech). The primers used were Forward 5 GCCAGCCCTATCATGACCAA 3 (SEQ ID NO: 32604) and Reverse 5'GCAGGTCGAGGCATTGAAGA'3 (SEQ ID NO: 32605). The resulting cDNA contained all exons including the start and stop codons of ASGR-1. PCR product was run on 1.6% Agarose gel and a band of the correct size was excised out and purified using QIAquick gel extraction kit (QIAGEN 28704) following the manufacturer's protocol. For cloning of ASGR-1 cDNA into pcDNA3.1/V5-His TOPO vector (Invitrogen K4800-01), 2μl of the gel extraction product was used and the manufacturer's protocol was followed resulting in pcDNA3.1_ASGR-1_WT. Transformed TOP10 chemically competent cells (Invitrogen C4040-10) were plated on LB plates containing 50μg/ml ampicillin. Colonies were expanded in 3ml LB medium containing 50μg/ml ampicillin. Plasmids were purified using QIAGEN plasmid mini kit (QIAGEN 12125) following the manufacturer's protocol. The plasmid sequence was confirmed by Sanger sequencing using the following sequencing primers: T7: 5'TAATACGACTCACTATAGGG'3 (SEQ ID NO: 32606), BGH: 5'TAGAAGGCACAGTCGAGG'3 (SEQ ID NO: 32607) and ASGR-1: 5'GAGGCAATGTGGGAAGAAAGATG'3 (SEQ ID NO: 32608)

*Introduction of 22bp deletion in ASGR-1:*

In order to generate a cDNA representative of the del12 carrier mRNA, targeted mutagenesis was performed. The Q5 Site-directed mutagenesis kit (New England BioLabs E0554S) and the pcDNA3.1_ASGR-1_WT plasmid was used as a template. In short, a PCR reaction was performed using the following primers 5'GAGGCAATGTGGGAAGAAAGATGAAGTCG'3 (SEQ ID NO: 32609) and 5'CTGGGCCTCCGTGCTCGC'3 (SEQ ID NO: 32610), resulting in a double stranded DNA fragment representing the entire pcDNA3.1_ASGR-1_WT plasmid lacking the 22bp at the end of exon 4. Following the manufacturers recommendation, 1uL of the PCR reaction was used in the KLD reaction (New England BioLabs E0554S) wherein the PCR fragment is phosphorylated, re-circularized and the non-mutated template plasmid is removed. Mutated plasmids were transformed into NEB 5-alpha Competent cells (New England BioLabs C2987H) and plated on LB plates containing 50μg/ml ampicillin. Colonies were expanded in 3ml LB medium containing 50μg/ml ampicillin. Plasmids were purified using QIAGEN plasmid mini kit (QIAGEN 12125) following the manufacturer's protocol. ASGR-1_22bp_del sequence was confirmed by Sanger sequencing.

*Expression of ASGR-1 in Cultured cells:*

Two days prior to transfection, 100,000 HeLa cells (Public Health England 93021013) were seeded into each well of a 6-well plate in 3mL of DMEM medium (11995-065, ThermoFisher) supplemented with 10% fetal calf serum (ThermoFisher 10500-064) and 50 units/mL penicillin and 50 ug/mL streptomycin (ThermoFisher 15070-063). Cells were incubated at 37°C and 5% $CO_2$ in a humidified incubator.

The day before transfection, media was replaced with the without antibiotics. On the day of transfection, for each transfected well, 2.5ug of plasmids containing ASGR-1_WT or ASGR-1_del22 cDNA were diluted in 125uL Opti-Mem medium (ThermoFisher 31985-047) and 5uL of P3000 reagent (ThermoFisher L3000-008). Next, 3.75uL Lipofectamine 3000 (ThermoFisher L3000-008) were mixed with 125uL of Opti-Mem. Subsequently, the diluted plasmid solution was mixed with the Lipofectamine 3000 solution at a 1:1 ratio and incubated at room temperature for 5 minutes before the addition of 250uL of the combined solution to each transfected well.

24 hours post transfection, the spent media was replaced with fresh without antibiotics. Selected wells were supplemented with 10uMMG132 (TOCRIS 1748) for 4.5 hours prior to harvesting of cells. 48 hours post transfection cells were harvested for analysis by washing wells 2x with PBS (ThermoFisher 14190-250) followed by an 8 minute incubation with 1mL of 0.5mM EDTA in PBS (ThermoFisher 15575-020). Next, the EDTA solution was aspirated and cells dislodged by pipetting of 2mL of fresh media. 3x6-wells were pooled for each experimental condition and cells were spun down at 300 × g for 5 minutes. The equivalent of 2x6-wells were lysed in 200uL of RIPA buffer for Western blot analysis. The remainder of cells were split in two and lysed in 300uL RLT buffer (Qiagen 74106) or 900uL Tissue and Cell lysis solution (Epicentre MTC096H) and snap frozen on dry ice for RNA and DNA extraction respectively. Three different transient expression experiments were done and all gave the same results.

*Quantitative PCR analysis:*

RNA was isolated from cells using the RNeasy Mini Kit (Qiagen 74106) according to manufacturer's recommendations, and concentration and quality was determined with Nanodrop 1000 spectrophotometer (Thermo Scientific). cDNA was synthesized using the High capacity cDNA reverse transcriptase kit (ThermoFisher). DNA was isolated from cells using the MasterPure DNA Purification Kit (Epicentre MCD85201) according to manufacturer's recommendation.

Analysis of gene expression and transfection efficiency was performed on total cDNA and DNA respectively, with real-time PCR on an ABI Prism 7900HT Sequence Detection System (ThermoFisher) using forward (AGACCTTCAGCATCTGGACAATG (SEQ ID NO: 32611)) and reverse (CGAGGTCCGGAGCAGAGA (SEQ ID NO: 32612)) primers and fluorescent labelled probe spanning exon junction 2-3 of the ASGR-1 gene (6FAM- CAGAAAAGGGCCACCTC-MGB (SEQ ID NO: 32613) (ThermoFisher). Human betaActin assay (ThermoFisher 4326315E) was run in parallel to verify normalization of input cDNA and DNA.

*Western blot analysis:*

Cells corresponding to two wells of a 6 well plate were lysed using 200μl of RIPA buffer with 1:100 Halt protease and phosphatase inhibitor cocktail (Thermo Scientific 78442). Lysates were kept on ice for 10 min with agitation followed by sonication for 20 sec (Branson 2510) and additional agitation on ice for 10 min. Lysates were spun down at 4°C for 15 min at 14,000 × g. Total protein amount of lysates was estimated using the Pierce BCA protein assay kit (Thermo Scientific 23227). Samples were prepared using Novex Bolt LDS sample buffer (4x) (Life technologies B0007) and Novex Bolt sample reducing agent (10x) (Life technologies B0009) and run on Novex Nupage 4- 12% Bis-Tris gel (Life technologies NP0335BOX). Total protein amount per lane was 24μg and PageRuler (Thermo scientific 26616) was used to estimate protein size. The gel was run at a constant of 200V for 50 min. Proteins were transferred to a nitrocellulose membrane (Life technologies IB23002) using iBlot2 (Life technologies). Membranes were allowed to dry and were then hydrated with MQ water before blotting. Membranes were blocked for 1 hour at room temperature using Odyssey blocking buffer PBS (Li-Cor 927-40000). Primary antibodies used were α-ASGR-1 (Sigma- Aldrich HPA011954) 1:500 (recognizes amino acid 1-41) and α-beta-actin (Abcam ab6276) 1:5000 incubated in blocking buffer with the addition of 0.1% Tween for 3 hours at room temperature. Secondary antibodies used were α-Rabbit 680RD (Li-Cor 926-68073) and α-Mouse 800CW (Li-Cor 926-32212) both 1:20,000 in PBST + 0.01% SDS for 1 hour at room temperature. After washing the membrane it was allowed to dry and then scanned using the Odyssey infrared imaging system (Li-Cor Biosciences).

**Other diseases and traits in deCODE database:**

The deCODE Genetics phenotype database contains medical information on diseases and traits obtained through collaboration with specialists in each field. This includes information on cardiovascular diseases (e.g., myocardial infarction, coronary arterial disease, peripheral arterial disease, atrial fibrillation, sick sinus syndrome and stroke), metabolic disorders (e.g., obesity, diabetes, and metabolic syndrome), psychiatric disorders (e.g., schizophrenia, bipolar disorder, anxiety and depression), addictions (e.g., nicotine, alcohol), inflammatory diseases (e.g., rheumatoid arthritis, lupus, and asthma), musculoskeletal disorders (e.g., osteoarthritis, osteoporosis), eye diseases (e.g., glaucoma), kidney diseases (e.g., kidney stones, kidney failure) and 29 types of cancer. Anthropometric measures have also been collected through several of these projects. Routinely measured traits from patient workups (e.g., sodium, potassium, bicarbonate, calcium, phosphate, creatinine, blood cell counts, hemoglobin, hematocrit, immunoglobulins, iron, vitamins, lipids, liver function tests and more) were obtained from the Landspitali University Hospital, Reykjavik, and the Icelandic Medical Center Laboratory in Mjodd (Laeknasetrid), Reykjavik. The number of independent and uncorrelated secondary traits tested for association with del12 amounts to 400.

**RESULTS**

**ASSOCIATION OF SEQUENCE VARIANTS WITH NON-HDL CHOLESTEROL LEVELS**

Sequence variants were first identified through whole-genome sequencing ("WSG") of 2,636 Icelanders to a median depth of 20x. These variants were imputed (assisted by long-range phased haplotypes) into the genomes of 104,220 Icelanders who had been genotyped using Illumina single nucleotide polymorphism (SNP) arrays. In addition, Icelandic genealogical information was used to calculate genotype probabilities for 294,212 close relatives to those genotyped. Using these data we screened for novel rare variants that associated with non-HDL cholesterol levels (n=1 19,146). A set of seven correlated (pairwise r2>0.7) rare non-coding SNPs on chromosome 17p13.1 associated with non-HDL cholesterol level. The seven variants span 80kb, including the asialoglycoprotein receptor 1 and 2 (ASGR-1 and ASGR-2) genes. The strongest association was represented by rs186021206 (minor allele frequency (MAF) = 0.43%) located downstream of ASGR-1 that associates with 8.9±1.5 mg/dl lowering of non-HDL cholesterol (P=1.4×10-9)(Table 1.4B).

The associated region was well covered by the whole-genome sequencing except for intron 4 of ASGR-1. This intron is 79 base pairs (bp) long and very GC rich. To explore this region further 738 individuals were whole genome sequenced with PCR-free sequencing (Illumina), that gave enhanced coverage of the intron and led to the identification of a 12bp deletion within the intron;NM_001671.4:c.284-36_283+33delCTGGGGCTGGGG here after referred to as del12. Following direct genotyping of del12 and imputation into the Icelandic dataset, we observed that del12 (MAF=0.41%) is highly correlated with rs186021206 (r2=0.86) and the six other correlated SNPs and associates even more strongly with lowering of non-HDL cholesterol levels (decrease of 10.2 ± 1.5 mg/dl, P=2.5×10-10) (Table 1.9A). Del12 also increases HDL cholesterol and decreases triglyceride (TG) levels, albeit to a much lesser degree than for non-HDL cholesterol (Tables 1.4A and 1.9B). None of the seven SNPs maintained a significant association with non-HDL cholesterol after adjusting for del12 indicating that del12 is sufficient to explain the non-HDL association.

To validate the del12 association with non-HDL cholesterol levels, del12 in samples from The Netherlands (Nijmegen Biomedical Study18) and Denmark (Danish Inter9919 and Danish Addition study20) were genotyped. Del12 associated with non-HDL cholesterol in each sample set with similar effect size as in Iceland (Table 1.2, Tables 1.4A and 1.4B and Table 1.9B). When all three datasets were combined with the Icelandic discovery data, it was established that del12 lowers non-HDL cholesterol by 11.6±1.5 mg/dl (P=1.0×10-16)(Table 1.9B).

To identify additional additional loss of function variants in ASGR-1, an extended dataset was screened based on sequence variants identified through whole-genome sequencing ("WSG") of an additional group of 5,817 WGS Icelanders on top of the 2,636 described above. In this dataset, a rare four bp insertion mutation was identified; namely, MAF=0.027%; NM_001671.4:c.469_472dupAACT. As mentioned throughout, this frameshift mutation introduces a premature stop codon at amino acid 158 out of the 291 amino acid full length protein (NP_001662.1:p.W158X). Potential carriers and non-carriers were directly genotyped using Sanger sequencing. Those genotypes were then used to re-impute p.W158X into 150,656 Icelandic chipped typed individuals and their first and second degree relatives. In this dataset, c.469_472dupAACT associates significantly with a decrease in non-HDL cholesterol (-21.6 mg/dL, 95% CI-34.2 to -9.6) and an increase in ALP (45.3% increase, 95% CI 20.4 to 68.2, $P=7.9\times10^{-6}$) (Table 1.8). The direction of the effects of c.469_472dupAACT and the effect sizes are similar to that of del12 (Table 1.8). Given that a single test was performed, these results provide a significant replication of the ASGR-1 loss of function effect on non-HDL and ALP. Furthermore, since W158X is not correlated with del12 (i.e. there was no overlap between individuals carrying W158X and del12), the W158X variant provides yet further proof that the loss of function in the ASGR-1 gene is responsible for the observed changes in non-HDL, Triglycerides, Alkaline Phosphatase, Ferritin and Vitamin B12 levels. For coronary artery disease, the odds ratio for W158X (c.469_472dupAACT) was 0.65 (95% CI 0.26 to 1.40; P=0.24). As mentioned above, the W158X (c.469_472dupAACT) variant is independent of del12 and none of the 79 carriers found in Iceland carried del12. The variant also appears to be specific to the Icelandic population as it is not detected in large population databases such as (Exome Aggregation Consortium (ExAC), Exome Variant Server (EVS), Genomes of the Netherlands (GoNL) and dbSNP.

DEL12 WITHIN INTRON 4 OF ASGR-1 CAUSES A SPLICING ERROR RESULTING IN A FRAMESHIFT

Since del12 is located in intron 4 of ASGR-1, we examined its effect on splicing between exons 4 and 5. The region between exon 3 and 5 in cDNA generated from blood samples from 12 non-carriers and 12 heterozygous carriers of del12 was PCR amplified (Figure 4). The PCR products were resolved by gel electrophoresis demonstrating a band of 239bp in non-carrier. In del12 carriers, however, a smaller 217bp band was noted in addition to the expected 239bp PCR product (Figure 4B). Upon Sanger sequencing of the cDNA products we identified in the 217bp cDNA fragment a 22bp deletion at the end of exon 4 (Figure 4C). The deletion of these 22bp from the ASGR- 1 transcript appears to be driven by a pseudo 5 '-splice site in exon 4 (Figure 4D). It causes a frameshift in carriers such that, if translated, the resulting protein would lack both the oligomerization and carbohydrate recognition domains. To quantify this splicing defect we used the Illumina TruSeq method for direct digital counting of sequencing reads that were generated by sequencing the two cDNA products found in del12 carriers. On average, $32 \pm 13\%$ of the total ASGR-1 transcripts were accounted for by the incorrectly spliced isoform (Figure 4E). This form could not be detected in non- carriers (Figure 4E). Together, these data identify ASGR-1 as the target gene for the non-HDL association at this locus and are consistent with the associated mutation, del12, disrupting the function of the ASGR-1 protein. ASGR-1 is the major subunit of the hepatic asioaloglycoprotein receptor (ASGR) known to recognize and mediate the endocytosis and degradation of a wide variety of desialylated glycoproteins that contain terminal galactose (Gal) or N-acetylgalactosamine (Gal- NAc) residues on their N-linked carbohydrate chains (Morell AG, Gregoriadis G, Scheinberg IH, Hickman J, Ashwell G. The role of sialic acid in determining the survival of glycoproteins in the circulation. The Journal of biological chemistry 1971;246:1461-7; Van Den Hamer CJ, Morell AG, Scheinberg IH, Hickman J, Ashwell G. Physical and chemical studies on ceruloplasmin. IX. The role of galactosyl residues in the clearance of ceruloplasmin from the circulation. The Journal of biological chemistry 1970;245:4397-402; Ashwell G, Harford J. Carbohydrate-specific receptors of the liver. Annual review of biochemistry 1982;51:531-54; Weigel PH. Galactosyl and N-acetylgalactosaminyl homeostasis: a function for mammalian asialoglycoprotein receptors. BioEssays: news and reviews in molecular, cellular and developmental biology 1994;16:519-24).

THE DEL12 VARIANT IN ASGR-1 AND RISK OF CORONARY ARTERY DISEASE

Given the effect of del12 on non-HDL cholesterol levels, its impact on risk of CAD in 33,090 cases and 236,254 controls from Iceland and 8,558 cases and 11,120 controls from the USA, the UK, New Zealand and Denmark was assessed. It was found that carriers of del12 have a lower risk of CAD than non-carriers (odds ratio 0.66; 95% confidence interval [CI] 0.55 to 0.79; $P=6.3\times10-6$) (Figure 5A). There was no evidence of heterogeneity across the eight study populations (Phet=0.96). Del12 also decreases risk of MI in Iceland (hazard ratio 0.64; 95% CI, 0.64 to 0.80; $P=8.5\times10-5$) (Figure 5B). In addition, del12 carriers have a 1.5 years longer lifespan than non-carriers (95% CI, 0.2 to 2.8 years; P=0.020).

There is a strong positive correlation between the effect of sequence variants on non-HDL cholesterol levels and risk of CAD (Haddad L, Day IN, Hunt S, Williams RR, Humphries SE, Hopkins PN. Evidence for a third genetic locus causing familial hypercholesterolemia. A non- LDLR, non-APOB kindred. Journal of lipid research 1999;40:1113-22; Timms KM, Wagner S, Samuels ME, et al. A mutation in PCSK9 causing autosomal-dominant hypercholesterolemia in a Utah pedigree. Human genetics 2004;114:349-53; Varret M, Rabes JP, Saint-Jore B, et al. A third major locus for autosomal dominant hypercholesterolemia maps to 1p34.1-p32. American journal of human genetics 1999;64:1378-87; Hunt SC, Hopkins PN, Bulka K, et al. Genetic localization to chromosome 1p32 of the third locus for familial hypercholesterolemia in a Utah kindred. Arterioscler Thromb Vasc Biol 2000;20:1089-93; Do R, Willer CJ, Schmidt EM, et al.

Common variants associated with plasma triglycerides and risk for coronary artery disease. Nature genetics 2013;45:1345-52) (Figure 6, Table 1.5). However, several published variants, deviate from the overall trend. For example, LPA and ANGPTL4 variants have a substantially greater effect on CAD than their non-HDL effects would predict while the effect of the APOE variants is weaker than predicted by the non-HDL effect. Del12 in ASGR-1 is another example of a variant whose effect on CAD is stronger than predicted by the effect non-HDL cholesterol effect (Figure 6, Table 1.5).

ASSOCIATION OF DEL12 WITH SERUM LEVELS OF ALP AND VITAMIN B12

To determine the overall effect of del12 in ASGR-1, its effect on a variety of human diseases and other traits in the Icelandic dataset was screened. A highly significant association of del12 with higher levels of circulating alkaline phosphatase (ALP) (33.6±2.8 U/L increase, P=3.6×10- 63) and vitamin B12 (58.4±8.3 pmol/L increase, P=3.1×10-12) was observed (Tables 8A and 8B and Table 18). An increase in ALP levels may reflect liver disease, however, there was no increase in del 12 carriers in serum gamma glutamyl transferase (GGT), bilirubin, alanine aminotransferase or other measures of liver function that commonly parallel changes in ALP in liver disease (Table 1.6).

The del12 association with higher levels of ALP and vitamin B12 in individuals from the Danish Inter99 study with comparable effect sizes (P=9.9×10-69 for ALP and P=9.9×10-14 for vitamin B12) was replicated (Table 1.10).

A common variant upstream of ASGR-1 (rs314253; MAF=35.1%) has been reported to associate modestly with both LDL cholesterol and ALP levels (Chambers JC, Zhang W, Sehmi J, et al. Genome-wide association study identifies loci influencing concentrations of liver enzymes in plasma. Nature genetics 2011;43:1131-8; Willer CJ, Schmidt EM, Sengupta S, et al. Discovery and refinement of loci associated with lipid levels. Nature genetics 2013;45:1274-83). This common variant association is replicated in the data of the present invention (strongest association for both ALP and non-HDL with the correlated rs56093546; MAF=21.6%) and that its associations with ALP and non-HDL are independent of the rare signal represented by del12 (r2<0.001, Table 1.5) as demonstrated. As for del12, this common variant has opposite effects on ALP and non-HDL; the allele that increases ALP decreases non-HDL (see Chambers; Willer) (Table 1.7).

**TABLE 1.1**

| Study | Design | Definition of CAD and MI cases | Ascertainment of controls | Reference |
|---|---|---|---|---|
| Iceland | Case/ control | CAD and MI cases were defined by: a) discharge diagnoses (ICD 9 codes 410.*, 411.*, 412.*, 414.* or ICD 10 codes 120.0, I21.*, I22.*. I23.*, I24.*, I25.*) from LUH, b) significant angiographic CAD (≥ 50% stenosis of the major coronary vessels), c) undergone coronary revascularisation (CABG) d) MI or CAD (ICD 9 or 10 codes) listed in death registries, or e) MI before the age of 75 from MONICA registry | Study participants from various deCODE genetics programs without known CVD. | Helgadottir A, Thorleifsson G, Manolescu A, et al. A common variant on chromosom e 9p21 affects the risk of myocardial infarction. Science (New York, NY) 2007;316:1 491-3. |

(continued)

| TABLE 1.1 | | | | |
|---|---|---|---|---|
| Study | Design | Definition of CAD and MI cases | Ascertainment of controls | Reference |
| UK 1 - Leicester MI Study | Case/ control | Cases included MI patients admitted to the coronary care units of the Leicester Royal Infirmary, Leicester and the Royal Hallamshire Hospital, Sheffield and satisfied the WHO criteria for acute MI. | Controls included adult visitors of individuals with non-cardiovascular disease from each hospital or individuals from three primary care practices located in the same geographical area. Individuals who reported a history of CAD were excluded. | Helgadottir A, Manolescu A, Thorleifsson G, et al. The gene encoding 5-lipoxygenas e activating protein confers risk of myocardial infarction and stroke. Nature genetics 2004;36: 23 3-9. |
| UK2 - BHF Family Heart Study | Case/ control | The British Heart Foundation Family Heart Study (BHF-FHS) CAD cases were index cases from families of European ancestry with a strong familial history of defined CAD recruited from throughout the United Kingdom. CAD was defined as a validated history of myocardial infarction or coronary revascularisation (PTCA or CABG) before the 66th birthday. | Controls were blood donors recruited by the United Kingdom Blood Service (UKBS) as part of the Wellcome Trust Case Control Consortium Study. | Genome- wide association study of 14,000 cases of seven common diseases and 3,000 shared controls. Nature 2007;447:6 61-78, and Samani NJ, Erdmann J, Hall AS, et al. Genomewid e association analysis of coronary artery disease. The New England journal of medicine 2007; 357:4 43-53. |
| Emory (Atlanta, Georgia, USA) | Case/ control | Cases were identified from subjects undergoing cardiac catheterization at the Emory University Hospital. CAD cases included those that had at least one significant stenosis (≥50%) in any of the major coronary arteries on angiography, or those without significant stenosis but had history of MI, CABG, or PCI. | Controls included individuals undergoing cardiac catheterization with no or minimal CAD (<20% stenosis) and had no prior history of MI or CAD. Additional controls were recruited from the Grady Memorial Hospitals and Clinical Registry in Neurology (CRIN) and included individuals with non-vascular neurological diseases (mainly Parkinson's and Alzheimer's diseases), their spouses, unrelated friends and community volunteers; excluding those with a known history of CAD. | Helgadottir A, et al. (2007) |

(continued)

| TABLE 1.1 | | | | |
|---|---|---|---|---|
| **Study** | **Design** | **Definition of CAD and MI cases** | **Ascertainment of controls** | **Reference** |
| Duke (Durham, North Carolina, USA) | Case/ control | Participants were enrolled at Duke University Medical Center through the cardiac catheterization laboratories. MI cases included those with self-reported history of MI (corroborated by review of medical records), or those who suffered an MI during the study follow-up period. | Controls included those with no history of MI prior or subsequent to the index cardiac catheterization and no PCI or CABG ejection fraction on left ventriculogram greater than 40%, and stenosis less than 50% on coronary angiography. | Helgadottir A, et al. (2007) |
| UPenn (Philadelphia, Pennsylvania, USA) | Case/ control | The study participants were enrolled at the University of Pennsylvania Medical Center and included subjects undergoing cardiac catheterization. CAD cases included those that had at least one significant stenosis (≥50%) in any of the major coronary arteries on angiography, or those without significant stenosis but had history of MI, CABG, or PCI. | Controls included individuals without significant luminal stenosis on coronary angiography (luminal stenosis less than 50%). | Helgadottir A, et al. (2007) |
| New Zealand | Case/ control | a) Significant angiographic CAD (≥ 50% stenosis of the major coronary vessels), b) CABG-procedures c) MI or CAD (ICD 9 or 10 codes) in a clinical registry. | Study participants without known CAD and ultrasound screened for carotid artery disease and abdominal aortic aneurysm, with ankle brachial index to exclude peripheral artery disease. | Gretarsdotti r S, Baas AF, Thorleifsson G, et al. Genome- wide association study identifies a sequence variant within the DAB2IP gene conferring susceptibilit y to abdominal aortic aneurysm. Nature genetics 2010;42: 69 2-7. |
| Denmark 1 (Gentofte cadlab) | Case/ control | Cases were identified from subject investigated by coronary artery angiography because of suspected ischemic heart disease, valvular heart disease or cardiomyopathy. CAD cases included those that had at least one significant stenosis (≥50%) in any of the major coronary arteries on angiography | Individuals in Monica10 and Inter99 studies without CAD diagnosis based on information from the Danish National Patient Registry and the Danish Register of Causes of Death. | |

(continued)

| TABLE 1.1 | | | | |
|---|---|---|---|---|
| **Study** | **Design** | **Definition of CAD and MI cases** | **Ascertainment of controls** | **Reference** |
| Denmark 2 (Monica10) | Case/ control | Monica10 is a population based study. Participants were recruited from the Danish Central Personal Register as random samples of the population in the southern part of the former Copenhagen County. Cardiovascular events were defined as first ever non-fatal or fatal CVD (ICD-8: 390-448/ICD- 10: I00-I79). Assessment of the cardiovascular endpoints was based on data from the Danish National Patient Registry and the Danish Register of Causes of Death. | Individuals in Monica10 and Inter99 studies without CAD diagnosis based on information from the Danish National Patient Registry and the Danish Register of Causes of Death. | Olsen MH, Hansen TW, Christensen MK, et al. N- terminal pro-brain natriuretic peptide, but not high sensitivity C-reactive protein, improves cardiovascu lar risk prediction in the general population. European heart journal 2007;28: 13 74-81. |
| Denmark 3 (Inter99) | Case/ control | The Inter99 study is a population-based randomized controlled trial (CT00289237, ClinicalTrials.gov) investigating the effects of lifestyle intervention on cardiovascular disease. Cardiovascular events were defined as first ever non-fatal or fatal CVD (ICD- 8: 390-448/ICD-10: 100- I79). Assessment of the cardiovascular endpoints was based on data from the Danish National Patient Registry and the Danish Register of Causes of Death. | Individuals in Monica10 and Inter99 studies without CAD diagnosis based on information from the Danish National Patient Registry and the Danish Register of Causes of Death. | 14. Jorg ensen AB, Frikke-Schmidt R, Nordestgaa rd BG, Tybjaerg- Hansen A. Loss-of-function mutations in APOC3 and risk of ischemic vascular disease. The New England journal of medicine 2014; 371:3 2-41. |
| Sweden | Case/ Control | Ischemic stroke patients from the clinic at Karolinska Univerity Hospital, Stockholm. The ischemic stroke diagnosis was based on clinical findings and brain imaging (CT or MRI). | Population-based controls, either healthy blood donors or healthy volunteers recruited at the Karolinska Hospital | Gretarsdotti r et al (2008) Traylor et al (2012) |
| South Germany | Case/ Control | Ischemic stroke patients recruited at the stroke unit of the Department of Neurology, Klinikum Grosshadern, University of Munich. Diagnoses were based on clinical findings and imaging evidence (either CT or MRI), and were clinically confirmed by neurologists. | Gender and age matched individuals without history of cardiovascular disease selected from the KORA S4 Study | Traylor etal (2008) Gschwendt ner et al (2009) Wichmann et al (2005) |

(continued)

| TABLE 1.1 | | | | |
|---|---|---|---|---|
| **Study** | **Design** | **Definition of CAD and MI cases** | **Ascertainment of controls** | **Reference** |
| West Germany | Case/ Control | Ischemic stroke patients recruited through hospitls participating in the regional Westphalian Stroke Register. Diagnoses were based on clinical findings and imaging evidence (either CT or MRI), and were clinically confirmed by neurologists. | Population controls with a self-reported history of stroke from the population based Dortmund Health Study | Traylor et al (2008) Berger et al (2007) |
| United Kingdom | Case/ Control | Ischemic stroke patients recruited through a cerebrovascular service clinic. All cases were phenotyped by one experienced stroke neurologist with review of original brain imaging with CT or MRI. | Community controls, age and gender matched and free of symptomatic cerebrovascular disease were recruited from the same geographic area as the patients. | Traylor et al (2008) Gschwendt ner et al (2009) |

**Table 1.2.** Characteristics of Participants in the Discovery and Replication Studies of the association of del12 Variant with Plasma Lipid, Alkaline Phosphatase, and Vitamin B12 levels

| **Trait[a]** | **Iceland** | **Nijmegen Biomedical Study (Netherlands)[c]** | **Inter99 study (Denmark)[d]** | **Addition Study (Denmark)[e]** |
|---|---|---|---|---|
| Ancestry | Caucasian | Caucasian | Caucasian | Caucasian |
| Nb | 194,958 | 5,645 | 7,633 | 9,689 |
| Mean age (SD), yrs | 58.2 (40.6-75.8) | 55.8 (38.0-73.6) | 48.5 (36.1-55.5) | 59.9 (53.1-66.7) |
| Gender, % female | 53.4% | 53.6% | 49.9% | 46.4% |
| Non-HDL cholesterol (SD), mg/dL | 154.7 (109.1-200.3) | 170.7 (129.4-212.0) | 161.6 (117.5-205.7) | 164.7 (124.0-205.4) |
| LDL cholesterol (SD), mg/dL | 133.0 (91.6-174.4) | 138.6 (102.2-175.0) | 137.2 (99.7-174.7) | 139.3 (101.9-176.1) |
| HDL cholesterol (SD), mg/dL | 54.7 (37.7-71.7) | 52.6 (39.2-66.0) | 54.2 (38.4-70.0) | 60.0 (43.6-76.4) |
| Total Cholesterol (SD), mg/dL | 208.0 (162.6-253.4) | 223.4 (180.9-265.9) | 215.8 (173.6-258.0) | 224.7 (183.9-265.5) |
| Triglycerides (SD), mg/dL | 133.6 (67.6-190.5) | 155.8 (94.5-256.8) | 105.8 (60.8-183.9) | 117.4 (73.5-187.3) |
| Alkaline phosphatase (SD), IU/l | 87.1 (53.5-141.7) | na | 41.3 (30.7-55.6) | na |
| Vitamin B12 (SD), pmol/l | 398 (256-618) | na | 398 (286-554) | na |

[a]The average values (where available) for each of the traits listed is shown (± one SD). [b]Number of individuals with measurements for at least one of the traits. [C]Wetzels et al (2007)[5], [d]Jörgensen et al (2003)[6], [e]Lauritzen etal (2000)[7].

| Table 1.4A. Association of del12 with Non-HDL Cholesterol, LDL Cholesterol, HDL Cholesterol, Triglyceride, ALP and Vitamin B12 in Iceland, Denmark and The Netherlands | | | | | |
|---|---|---|---|---|---|
| | **Study population (n)** | | **Effect (95% CI)[a]** | ***P* value** | **Population mean value[e] (± 1SD)** |
| | **Non-HDL cholesterol** | **del12 freq.** (%) | **mg/dL** | | **mg/dL** |
| Discovery | Iceland (119,146) | 0.41 | -13.6 (-17.7,-9.4) | $2.5\times10^{-10}$ | 154.7 (109.1-200.3) |
| Replication | Denmark A[b] (6,182) | 0.22 | -21.3 (-36.8,-5.9) | 0.0069 | 161.6 (117.5-205.7) |
| Replication | Denmark B[c] (9,656) | 0.32 | -22.2 (-32.8,-11.7) | $3.8\times10^{-5}$ | 164.7 (124.0-205.4) |
| Replication | The Netherlands[d] (5,537) | 0.50 | -17.0 (-28.3,-5.7) | 0.0032 | 170.7 (129.4-212.0) |
| | Combined | | -15.3 (-18.9,-11.7) | $1.0\times10^{-16}$ | |
| | **LDL cholesterol** | | **mg/dL** | | |
| Discovery | Iceland (53,841) | 0.41 | -9.5 (-14.0,-5.1) | $2.8\times10^{-5}$ | 133.0 (91.6-174.4 |
| Replication | Denmark A (6,098) | 0.22 | -22.1 (-35.5,-8.7) | 0.0012 | 137.2 (99.7-174.7) |
| Replication | Denmark B (8,080) | 0.32 | -19.0 (-29.2,-8.8) | 0.00026 | 139.3 (101.9-176.1) |
| Replication | The Netherlands (5,523) | 0.50 | -16.0 (-26.1,-6.0) | 0.0018 | 138.6 (102.2-175.0) |
| | Combined | | -12.5 (-16.2,-8.8) | $3.9\times10^{-11}$ | |
| | **HDL cholesterol** | | **mg/dL** | | **mg/dL** |
| Discovery | Iceland (119,514) | 0.41 | 2.4 (0.7,4.1) | 0.0058 | 54.7 (37.7-71.7) |
| Replication | Denmark A (6,182) | 0.22 | 4.6 (-0.8,9.9) | 0.096 | 54.2 (38.4-70.0) |
| Replication | Denmark B (9,656) | 0.32 | 2.4 (-1.8,6.7) | 0.26 | 60.0 (43.6-76.4) |
| Replication | The Netherlands (5,537) | 0.50 | 2.4 (-1.3,6.0) | 0.20 | 52.6 (39.2-66.0) |
| | Combined | | 2.5 (1.1,4.0) | 0.00039 | |
| | **Triglyceride** | | **% change** | | **mg/dL** |
| Discovery | Iceland (80,011) | 0.41 | -6.1 (-10.8,-1 .5) | 0.012 | 133.6 (67.6-190.5) |
| Replication | Denmark A (6,182) | 0.22 | -6.0 (-25.2,11.4) | 0.53 | 105.8 (60.8-183.9) |
| Replication | Denmark B (8,163) | 0.32 | -8.9 (-21.0,2.3) | 0.15 | 117.4 (73.5-187.3) |
| Replication | The Netherlands (5,537) | 0.50 | -4.4 (-17.9,8.2) | 0.52 | 155.8 (94.5-256.8) |
| | Combined | | -6.3 (-10.3,-2.3) | 0.0032 | |
| | **ALP** | | **% change** | | **U/L** |
| Discovery | Iceland (126,060) | 0.41 | 50.1 (42.9,57.2) | $3.6\times10^{-63}$ | 87.1 (53.5-141.7) |
| Replication | Denmark A[c] (5,829) | 0.22 | 29.1 (14.8,42.5) | $3.1\times10^{-6}$ | 41.3 (30.7-55.6) |
| | Combined | | 46.5 (40.1,52.7) | $5.6\times10^{-69}$ | |
| | **Vitamin B12** | | **% change** | | **pmol/L** |
| Discovery | Iceland (97,910) | 0.41 | 16.6 (11.5,21.5) | $3.1\times10^{-12}$ | 398 (256-618) |
| Replication | Denmark A[c] (5,826) | 0.22 | 18.6 (3.9,32.4) | 0.0053 | 398 (286-554) |
| | Combined | | 16.8 (12.0,21.5) | $8.3\times10^{-14}$ | |

[a]Effect estimates and 95% confidence intervals (95% CI) in mg/dL for the non-HDL cholesterol and HDL cholesterol and as percentage change for triglyceride, ALP and vitamin B12. [b]The Danish Inter99 study (Jorgensen et al. 2003). [c]The Danish Addition study (van den Donk et al. 2011). [d]The Nijmegen Biomedical Study (Hoogendoom et al. 2006). [e]For triglyceride, ALP and vitamin B12, the population mean and the SD are calculated for log-transformed values and transformed back to original units. To convert the values for non-HDL and HDL cholesterol to millimoles per liter, multiply by 0.02586. To convert triglyceride to mmol/L, multiply by 0.01129.

| Table 1.4B. Association of del12 and rs186021206 with Cholesterols, Triglyceride, Alkaline Phosphatase and Vitamin B12 Measurements in Iceland, Denmark and the Netherlands. | | | | | | | | |
|---|---|---|---|---|---|---|---|---|
| | | rs186021206 | | | | del12 | | |
| **Trait / Cohort (n)[a]** | | **Effect[b]** | **Effect(95% CI)[c]** | ***P*** | **$P_{adj}$[d]** | **Effect[b]** | **Effect(95% CI)[c]** | ***P*** |
| **Non-HDL cholesterol** | | **SD** | **mg/dL** | | | | **mg/dL** | |
| Iceland (119,146) | | -0.28 | -12.9 (-17.1,-8.7) | $1.4\times10^{-9}$ | 0.39 | -0.30 | -13.6 (-17.7,-9.4) | $2.5\times10^{-10}$ |
| Denmark (6,182) | A | -0.38 | -16.7 (-27.9,-5.4) | 0.0038 | 0.64 | -0.48 | -21.3 (-36.8,-5.9) | 0.0069 |
| Denmark (9,656) | B | -0.32 | -13.1 (-21.0,-5.3) | 0.0011 | 0.74 | -0.55 | -22.2 (-32.8,-11.7) | $3.8\times10^{-5}$ |
| The Netherlands (5,537) | | -0.23 | -9.7 (-19.9,0.5) | 0.062 | 0.19 | -0.41 | -17.0 (-28.3,-5.7) | 0.0032 |
| Combined | | -0.29 | -12.9 (-16.3,-9.6) | $2.0\times10^{-14}$ | 0.24 | -0.34 | -15.3 (-18.9,-11.7) | $1.0\times10^{-16}$ |
| **LDL cholesterol** | | | **mg/dL** | | | | **mg/dL** | |
| Iceland (53,841) | | -0.22 | -9.2 (-13.6,-4.7) | $5.5\times10^{-5}$ | 0.78 | -0.23 | -9.5 (-14.0,-5.1) | $2.8\times10^{-5}$ |
| Denmark (6,098) | A | -0.43 | -16.1 (-25.8,-6.3) | 0.0012 | 0.56 | -0.59 | -22.1 (-35.5,-8.7) | 0.0012 |
| Denmark (8,080) | B | -0.34 | -12.5 (-20.3,-4.7) | 0.0016 | 0.86 | -0.51 | -19.0 (-29.2,-8.8) | 0.00026 |
| The Netherlands (5,523) | | -0.36 | -13.2 (-22.3,-4.2) | 0.0041 | 0.81 | -0.44 | -16.0 (-26.1,-6.0) | 0.0018 |
| Combined | | -0.28 | -11.1 (-14.5,-7.8) | $1.0\times10^{-10}$ | 0.70 | -0.31 | -12.5 (-16.2,-8.8) | $3.9\times10^{-11}$ |
| **Total cholesterol** | | | **mg/dL** | | | | **mg/dL** | |
| Iceland (125,381) | | -0.22 | -9.9 (-14.0,-5.7) | $3.1\times10^{-6}$ | 0.78 | -0.23 | -10.5 (-14.7,-6.4) | $6.5\times10^{-7}$ |
| Denmark (6,182) | A | -0.32 | -13.5 (-24.2,-2.8) | 0.014 | 0.54 | -0.33 | -14.0 (-28.7,0.8) | 0.063 |
| Denmark (9,656) | B | -0.30 | -12.0 (-19.9,-4.2) | 0.0027 | 0.97 | -0.47 | -19.2 (-29.8,-8.6) | 0.00040 |
| The Netherlands (5,537) | | -0.21 | -9.0 (-19.5,1.5) | 0.0927 | 0.48 | -0.33 | -14.1 (-25.7,-2.5) | 0.018 |
| Combined | | -0.24 | -10.5 (-13.8,-7.2) | $5.1\times10^{-10}$ | 0.68 | -0.27 | -12.0 (-15.6,-8.5) | $5.6\times10^{-11}$ |
| **HDL cholesterol** | | | **mg/dL** | | | | **mg/dL** | |
| Iceland (119,514) | | 0.13 | 2.2 (0.5,3.9) | 0.011 | 0.0055 | 0.14 | 2.4 (0.7,4.1) | 0.0058 |
| Denmark (6,182) | A | 0.15 | 2.4 (-1.5,6.4) | 0.22 | 0.84 | 0.29 | 4.6 (-0.8,9.9) | 0.096 |
| Denmark (9,656) | B | 0.03 | 0.4 (-2.7,3.6) | 0.79 | 0.32 | 0.15 | 2.4 (-1.8,6.7) | 0.26 |
| The Netherlands (5,537) | | 0.02 | 0.2 (-3.1,3.5) | 0.9 | 0.043 | 0.18 | 2.4 (-1.3,6.0) | 0.20 |
| Combined | | 0.10 | 1.6 (0.4,2.9) | 0.01 | 0.001 | 0.15 | 2.5 (1.1,4.0) | 0.00039 |
| **Triglyceride** | | | **% change** | | | | **% change** | |
| Iceland (80,011) | | -0.11 | -5.4 (-10.1,-0.8) | 0.027 | 0.13 | -0.12 | -6.1 (-10.8,-1.5) | 0.012 |

(continued)

| Triglyceride | | | % change | | | | % change | |
|---|---|---|---|---|---|---|---|---|
| Denmark (6,182) | A | -0.26 | -13.4 (-26.1,-1.6) | 0.046 | 0.11 | -0.11 | -6.0 (-25.2,11.4) | 0.53 |
| Denmark (8,163) | B | -0.03 | -1.3 (-11.2,8.0) | 0.79 | 0.099 | -0.2 | -8.9 (-21.0,2.3) | 0.15 |
| The Netherlands (5,537) | | 0.13 | 6.5 (-7.0,19.1) | 0.32 | 0.0057 | -0.09 | -4.4 (-17.9,8.2) | 0.52 |
| Combined | | -0.09 | -4.2 (-7.9,-0.6) | 0.028 | 0.0066 | -0.13 | -6.3 (-10.3,-2.3) | 0.003 |
| **ALP** | | | **% change** | | | | **% change** | |
| Iceland (126,060) | | 0.82 | 48.9 (41.8,55.8) | $1.2\times10^{-61}$ | 0.10 | 0.84 | 50.1 (42.9,57.2) | $3.6\times10^{-63}$ |
| Denmark (6,035) | A | 0.70 | 23.0 (13.2,32.4) | $2.2\times10^{-7}$ | 0.092 | 0.86 | 29.1 (14.8,42.5) | $3.1\times10^{-6}$ |
| Combined | | 0.80 | 41.5 (35.9,47.0) | $1.9\times10^{-67}$ | 0.026 | 0.84 | 46.5 (40.1,52.7) | $5.6\times10^{-69}$ |
| **Vitamin B12** | | | **% change** | | | | **% change** | |
| Iceland (97,910) | | 0.33 | 15.8 (10.8,20.7) | $2.0\times10^{-11}$ | 0.15 | 0.35 | 16.6 (11.5,21.5) | $3.1\times10^{-12}$ |
| Denmark (6,032) | A | 0.49 | 17.6 (7.2,27.7) | 0.00027 | 0.011 | 0.52 | 18.6 (3.9,32.4) | 0.0053 |
| Combined | | 0.35 | 16.1 (11.6,20.6) | $4.3\times10^{-14}$ | 0.84 | 0.36 | 16.8 (12.0,21.5) | $8.3\times10^{-14}$ |

[a]Number of individuals with trait value and genotypes. [b]Effect estimates from the regression in units of standard deviations (SD) of the distributions of the adjusted values. [c]Effect estimates and 95% confidence intervals (95% CI) in mg/dL for the cholesterol, and as percentage change for triglyceride, ALP and vitamin B12. [d]*P*-values adjusted for the effect of del12. "The Netherlands", The Nijmegen Biomedical Study[15]; "Denmark A", The Danish Inter99 study[6]; "Denmark B", The Danish Addition study[16].

**Table 1.5. The association of published lipid variants with non-HDL cholesterol levels and coronary artery disease in Iceland.**

| Build 36 position | | | | Non-HDL (mg/dL) | | Coronary artery disease | | |
|---|---|---|---|---|---|---|---|---|
| Chr | Position (hg18) | MAF | Info | Effect | SE | OR | 95% | CI |
| 1 | 25,641,524 | 0.47184 | 0.996 | 0.7 | 0.2 | 0.99 | 0.97 | 1.02 |
| 1 | 55,278,235 | 0.01173 | 0.986 | -17.2 | 1.0 | 0.73 | 0.66 | 0.81 |
| 1 | 62,725,961 | 0.21814 | 0.996 | 1.6 | 0.3 | 1.01 | 0.98 | 1.03 |
| 1 | 62,906,518 | 0.33844 | 0.998 | -2.3 | 0.2 | 0.99 | 0.97 | 1.01 |
| 1 | 92,766,395 | 0.19052 | 0.999 | 0.8 | 0.3 | 0.99 | 0.97 | 1.02 |
| 1 | 109,620,053 | 0.20789 | 0.999 | 4.8 | 0.3 | 1.08 | 1.06 | 1.11 |
| 1 | 110,000,250 | 0.41287 | 0.995 | 1.0 | 0.2 | 1.01 | 0.99 | 1.03 |
| 1 | 149,225,460 | 0.15162 | 0.997 | -0.7 | 0.3 | 1.03 | 1.00 | 1.06 |
| 1 | 154,967,275 | 0.28892 | 0.998 | -0.5 | 0.2 | 0.99 | 0.97 | 1.02 |
| 1 | 219,036,651 | 0.28689 | 0.994 | 0.9 | 0.2 | 1.01 | 0.98 | 1.03 |
| 1 | 228,362,314 | 0.39128 | 0.999 | -1.1 | 0.2 | 0.99 | 0.97 | 1.01 |
| 1 | 232,915,962 | 0.4424 | 0.999 | 1.2 | 0.2 | 1.00 | 0.98 | 1.03 |
| 2 | 21,087,477 | 0.04518 | 0.999 | -6.1 | 0.5 | 0.94 | 0.89 | 0.99 |
| 2 | 21,117,405 | 0.3491 | 0.997 | 2.9 | 0.2 | 1.05 | 1.03 | 1.07 |
| 2 | 21,139,562 | 0.1408 | 0.999 | 4.3 | 0.3 | 1.08 | 1.04 | 1.11 |

(continued)

| Table 1.5. The association of published lipid variants with non-HDL cholesterol levels and coronary artery disease in Iceland. | | | | | | | | |
|---|---|---|---|---|---|---|---|---|
| Build 36 position | | | | Non-HDL (mg/dL) | | Coronary artery disease | | |
| Chr | Position (hg18) | MAF | Info | Effect | SE | OR | 95% | CI |
| 2 | 27,584,444 | 0.34466 | 0.998 | -1.8 | 0.2 | 1.00 | 0.98 | 1.03 |
| 2 | 27,584,716 | 0.20151 | 0.995 | -1.4 | 0.3 | 1.00 | 0.97 | 1.02 |
| 2 | 43,927,385 | 0.27892 | 0.999 | -2.6 | 0.2 | 0.95 | 0.93 | 0.98 |
| 2 | 43,953,086 | 0.19027 | 0.997 | -1.5 | 0.3 | 0.96 | 0.94 | 0.99 |
| 2 | 63,003,061 | 0.32014 | 0.997 | 0.9 | 0.2 | 1.02 | 1.00 | 1.05 |
| 2 | 118,293,189 | 0.07895 | 0.998 | -0.8 | 0.4 | 1.02 | 0.98 | 1.06 |
| 2 | 121,025,958 | 0.41077 | 0.994 | 0.6 | 0.2 | 1.03 | 1.01 | 1.06 |
| 2 | 169,538,401 | 0.37685 | 0.999 | -0.5 | 0.2 | 0.99 | 0.97 | 1.01 |
| 2 | 216,012,629 | 0.32322 | 0.998 | 0.8 | 0.2 | 0.95 | 0.93 | 0.97 |
| 3 | 12,271,469 | 0.3667 | 0.998 | -1.2 | 0.2 | 0.99 | 0.97 | 1.02 |
| 3 | 32,508,014 | 0.07924 | 0.997 | -1.6 | 0.4 | 0.98 | 0.94 | 1.02 |
| 3 | 133,691,893 | 0.11977 | 0.998 | -1.1 | 0.3 | 0.99 | 0.96 | 1.03 |
| 3 | 172,209,912 | 0.07646 | 0.999 | 0.8 | 0.4 | 1.08 | 1.04 | 1.12 |
| 4 | 3,442,937 | 0.40281 | 0.991 | 0.7 | 0.2 | 1.03 | 1.00 | 1.05 |
| 4 | 25,672,088 | 0.14802 | 0.993 | 0.9 | 0.3 | 1.04 | 1.01 | 1.07 |
| 4 | 88,249,285 | 0.40279 | 0.999 | 0.7 | 0.2 | 1.00 | 0.98 | 1.02 |
| 4 | 100,233,828 | 0.42298 | 0.998 | 0.5 | 0.2 | 1.01 | 0.99 | 1.03 |
| 5 | 74,661,243 | 0.35407 | 0.999 | 2.8 | 0.2 | 1.04 | 1.02 | 1.06 |
| 5 | 122,883,315 | 0.47211 | 0.995 | 0.5 | 0.2 | 1.00 | 0.98 | 1.02 |
| 5 | 156,322,875 | 0.35741 | 0.998 | 1.7 | 0.2 | 1.01 | 0.99 | 1.03 |
| 6 | 16,217,142 | 0.46163 | 0.995 | -0.8 | 0.2 | 0.99 | 0.97 | 1.01 |
| 6 | 26,201,120 | 0.06713 | 1.000 | -1.5 | 0.4 | 0.99 | 0.95 | 1.03 |
| 6 | 31,373,469 | 0.29084 | 0.993 | 0.8 | 0.2 | 1.02 | 1.00 | 1.04 |
| 6 | 43,865,874 | 0.47286 | 0.993 | 0.9 | 0.2 | 1.02 | 1.00 | 1.04 |
| 6 | 100,706,818 | 0.19956 | 0.998 | -1.0 | 0.3 | 1.00 | 0.97 | 1.02 |
| 6 | 116,444,196 | 0.40848 | 0.998 | -0.6 | 0.2 | 0.98 | 0.96 | 1.00 |
| 6 | 127,494,332 | 0.47183 | 0.999 | 0.9 | 0.2 | 1.01 | 0.99 | 1.03 |
| 6 | 139,873,450 | 0.42692 | 0.999 | -0.7 | 0.2 | 0.98 | 0.96 | 1.00 |
| 6 | 160,881,127 | 0.01773 | 1.000 | 4.0 | 0.8 | 1.31 | 1.21 | 1.41 |
| 6 | 160,930,108 | 0.06104 | 0.984 | 2.3 | 0.4 | 1.27 | 1.22 | 1.33 |
| 7 | 21,573,877 | 0.22512 | 0.992 | 1.5 | 0.3 | 1.00 | 0.98 | 1.02 |
| 7 | 25,958,351 | 0.14423 | 0.993 | 0.9 | 0.3 | 1.05 | 1.02 | 1.08 |
| 7 | 44,548,856 | 0.2013 | 0.990 | 2.0 | 0.3 | 1.02 | 1.00 | 1.05 |
| 7 | 44,567,220 | 0.42549 | 0.998 | -1.2 | 0.2 | 0.97 | 0.95 | 0.99 |
| 7 | 72,620,810 | 0.11552 | 0.998 | -0.9 | 0.3 | 1.02 | 0.99 | 1.06 |
| 7 | 72,697,942 | 0.46468 | 0.997 | 0.5 | 0.2 | 0.99 | 0.97 | 1.01 |
| 7 | 130,095,474 | 0.44163 | 0.998 | -0.5 | 0.2 | 0.96 | 0.94 | 0.98 |
| 8 | 9,221,641 | 0.07554 | 0.997 | 1.9 | 0.4 | 1.04 | 1.00 | 1.08 |
| 8 | 18,316,718 | 0.18705 | 0.996 | -1.3 | 0.3 | 0.96 | 0.94 | 0.99 |
| 8 | 19,888,502 | 0.08181 | 0.996 | -2.1 | 0.4 | 0.93 | 0.89 | 0.97 |
| 8 | 19,910,123 | 0.45471 | 0.996 | -1.0 | 0.2 | 0.96 | 0.94 | 0.98 |
| 8 | 55,584,167 | 0.24432 | 1.000 | 1.0 | 0.2 | 1.02 | 0.99 | 1.04 |
| 8 | 59,548,473 | 0.31037 | 0.998 | -1.4 | 0.2 | 0.99 | 0.97 | 1.01 |
| 8 | 116,733,072 | 0.26318 | 0.999 | -1.1 | 0.2 | 1.00 | 0.97 | 1.02 |
| 8 | 126,543,488 | 0.22755 | 0.997 | -1.9 | 0.3 | 0.96 | 0.94 | 0.99 |

(continued)

| Table 1.5. The association of published lipid variants with non-HDL cholesterol levels and coronary artery disease in Iceland. | | | | | | | | |
|---|---|---|---|---|---|---|---|---|
| Build 36 position | | | | Non-HDL (mg/dL) | | Coronary artery disease | | |
| Chr | Position (hg18) | MAF | Info | Effect | SE | OR | 95% | CI |
| 8 | 126,551,803 | 0.49199 | 0.999 | -2.3 | 0.2 | 0.95 | 0.93 | 0.97 |
| 8 | 145,094,645 | 0.385 | 0.990 | 0.7 | 0.2 | 0.98 | 0.96 | 1.00 |
| 9 | 2,630,759 | 0.09898 | 0.998 | -1.3 | 0.4 | 0.97 | 0.94 | 1.01 |
| 9 | 16,894,846 | 0.31865 | 0.998 | -0.5 | 0.2 | 0.97 | 0.95 | 0.99 |
| 9 | 106,704,122 | 0.25781 | 0.999 | -1.1 | 0.2 | 0.97 | 0.95 | 0.99 |
| 9 | 106,724,051 | 0.28833 | 0.997 | -0.6 | 0.2 | 0.99 | 0.97 | 1.02 |
| 9 | 135,122,694 | 0.38646 | 0.997 | -0.9 | 0.2 | 0.99 | 0.97 | 1.01 |
| 9 | 135,143,989 | 0.15248 | 0.995 | 1.0 | 0.3 | 1.05 | 1.02 | 1.08 |
| 10 | 94,829,632 | 0.42892 | 0.993 | -0.6 | 0.2 | 0.99 | 0.97 | 1.01 |
| 11 | 18,612,847 | 0.30731 | 0.998 | 0.8 | 0.2 | 1.02 | 1.00 | 1.04 |
| 11 | 61,305,450 | 0.27208 | 0.991 | 0.8 | 0.2 | 1.01 | 0.99 | 1.04 |
| 11 | 61,354,548 | 0.38782 | 0.998 | -1.1 | 0.2 | 1.00 | 0.98 | 1.02 |
| 11 | 116,144,314 | 0.06787 | 0.999 | -5.8 | 0.4 | 0.94 | 0.91 | 0.98 |
| 11 | 116,159,645 | 0.46743 | 0.999 | -0.5 | 0.2 | 0.97 | 0.95 | 0.99 |
| 11 | 116,206,564 | 0.00228 | 0.979 | -15.1 | 2.3 | 0.91 | 0.73 | 1.14 |
| 11 | 122,039,714 | 0.40275 | 0.996 | 0.6 | 0.2 | 1.01 | 0.99 | 1.03 |
| 11 | 125,749,162 | 0.10572 | 0.999 | 0.7 | 0.3 | 1.02 | 0.99 | 1.06 |
| 12 | 110,492,139 | 0.38236 | 0.999 | 0.8 | 0.2 | 0.94 | 0.92 | 0.96 |
| 12 | 110,794,963 | 0.2284 | 0.999 | 0.8 | 0.3 | 0.94 | 0.92 | 0.97 |
| 12 | 119,901,033 | 0.30901 | 0.994 | 0.9 | 0.2 | 1.03 | 1.01 | 1.05 |
| 13 | 31,851,388 | 0.44766 | 0.999 | -0.7 | 0.2 | 0.99 | 0.97 | 1.01 |
| 14 | 23,953,727 | 0.49889 | 0.995 | 0.8 | 0.2 | 0.98 | 0.96 | 1.01 |
| 15 | 56,518,445 | 0.19278 | 0.999 | -0.6 | 0.3 | 0.99 | 0.97 | 1.02 |
| 16 | 55,542,640 | 0.38939 | 0.991 | -1.8 | 0.2 | 0.97 | 0.95 | 0.99 |
| 16 | 55,572,592 | 0.06047 | 0.997 | 2.9 | 0.5 | 1.04 | 1.00 | 1.09 |
| 16 | 66,485,543 | 0.10432 | 1.000 | -0.8 | 0.3 | 0.97 | 0.94 | 1.01 |
| 16 | 70,665,594 | 0.14755 | 0.997 | 1.3 | 0.3 | 1.03 | 1.00 | 1.06 |
| 17 | 7,032,374 | 0.35058 | 0.996 | -1.0 | 0.2 | 0.98 | 0.96 | 1.00 |
| 17 | 8,101,874 | 0.49481 | 0.998 | -0.4 | 0.2 | 0.96 | 0.94 | 0.98 |
| 17 | 39,281,652 | 0.03364 | 0.989 | 1.3 | 0.6 | 1.08 | 1.02 | 1.15 |
| 17 | 42,746,803 | 0.28266 | 0.998 | 0.6 | 0.2 | 1.02 | 1.00 | 1.04 |
| 17 | 64,394,061 | 0.32561 | 0.995 | 0.5 | 0.2 | 1.03 | 1.01 | 1.05 |
| 18 | 45,363,953 | 0.01171 | 0.999 | 4.8 | 1.0 | 1.00 | 0.91 | 1.09 |
| 19 | 8,335,323 | 0.02392 | 0.965 | -4.7 | 0.7 | 0.80 | 0.74 | 0.86 |
| 19 | 11,063,306 | 0.0888 | 0.995 | -6.8 | 0.4 | 0.89 | 0.86 | 0.92 |
| 19 | 11,088,602 | 0.45236 | 0.997 | 1.4 | 0.2 | 1.02 | 1.00 | 1.04 |
| 19 | 19,268,718 | 0.07838 | 0.997 | -3.8 | 0.4 | 0.96 | 0.92 | 1.00 |
| 19 | 50,103,781 | 0.16819 | 0.980 | 8.4 | 0.3 | 1.05 | 1.02 | 1.08 |
| 19 | 50,103,919 | 0.05236 | 0.968 | -16.9 | 0.5 | 0.83 | 0.79 | 0.87 |
| 19 | 53,898,229 | 0.39118 | 0.997 | 1.1 | 0.2 | 1.00 | 0.98 | 1.03 |
| 19 | 57,016,028 | 0.27115 | 0.999 | 0.6 | 0.2 | 1.03 | 1.01 | 1.06 |
| 19 | 59,489,660 | 0.21613 | 0.990 | -0.6 | 0.3 | 0.99 | 0.96 | 1.02 |
| 20 | 12,910,718 | 0.45731 | 0.998 | 0.4 | 0.2 | 1.00 | 0.98 | 1.03 |
| 20 | 17,793,921 | 0.15541 | 0.991 | 0.8 | 0.3 | 0.98 | 0.95 | 1.01 |
| 20 | 38,613,850 | 0.34358 | 0.997 | -1.1 | 0.2 | 0.98 | 0.96 | 1.00 |

(continued)

| Table 1.5. The association of published lipid variants with non-HDL cholesterol levels and coronary artery disease in Iceland. | | | | | | | | |
|---|---|---|---|---|---|---|---|---|
| Build 36 position | | | | Non-HDL (mg/dL) | | Coronary artery disease | | |
| Chr | Position (hg18) | MAF | Info | Effect | SE | OR | 95% | CI |
| 20 | 39,157,752 | 0.45945 | 0.997 | 1.1 | 0.2 | 0.99 | 0.97 | 1.01 |
| 20 | 42,475,778 | 0.04599 | 0.993 | -1.3 | 0.5 | 0.98 | 0.93 | 1.03 |
| 20 | 44,018,827 | 0.21978 | 0.998 | 1.3 | 0.3 | 0.98 | 0.96 | 1.01 |

Shown are the build 36 positions (hg18), minor allele frequency (MAF), imputation information, the non-HDL effect in mg/dL and the standard error of the estimate (SE), and the OR for coronary artery disease and 95% CI for the minor allele.

| Table 1.6. Association of del12 with various measures of liver function in Iceland | | | | | |
|---|---|---|---|---|---|
| Phenotype | n[a] | Effect[b] | Effect (95% CI)[c] | *P* | Mean (± 1 SD)[d] |
| | | | % change | | |
| Alanine Transaminase | 144,402 | 0.087 | 5.8 (-0.4,12.2) | 0.065 | 28.7 (15.0-54.8 ) units/L |
| Alkaline Phosphatase | 126,060 | 0.840 | 50.1 (42.9,57.2) | $3.6\times10^{-63}$ | 87.1 (53.5-141.7) units/L |
| Aspartate Transaminase | 144,931 | 0.072 | 4.1 (-2.9,11.4) | 0.095 | 28.1 (14.2-55.6) units/L |
| Bilirubin | 94,805 | 0.054 | 3.7 (-2.6,10.4) | 0.25 | 9.1 (4.6-18.0) μm/L |
| Gamma Glutamyl Transpeptidase | 138,844 | 0.113 | 10.3 (1.7,19.2) | 0.015 | 30.9 (13.1-72.9 )units/L |
| | | | g/L | | |
| Albumin | 78,555 | -0.109 | -0.72 (-1.37,0.06) | 0.033 | 39.5 (33.0-46.0) g/L |

[a]Number of individuals used in the association analysis for each of the traits. [b]Effect estimate, in units of standard deviation, from regression of adjusted trait values on the expected genotype count of del12.

[c]effect estimates and 95% CI in original units. For traits with log-normal distribution the effects are presented as percentage change with 95% CI. [d]Mean trait values, ± one SD, in the Icelandic population. For traits with log-normal distribution the mean and SD is calculated for log-transformed trait values and transformed back to original units.

**Table 1.7. Common Variants at the *ASGR-1* Locus Associated with Non-HDL Cholesterol and Alkaline Phosphatase in Iceland**

| | rs314253 | rs56093546 | del12 |
|---|---|---|---|
| **Chromosome position** | 17:7032374 | 17:7004539 | 17:7020979 |
| **MAF (%)** | 35.06 | 21.63 | 0.43 |
| **Effect[a] on non-HDL cholesterol (P value)** | -0.03 ($5.9\times10^{-6}$) | -0.04 ($2.0\times10^{-6}$) | -0.30 ($2.5\times10^{-10}$) |
| **Adjusted for rs314253 (P)** | - | 0,022 | $7.9\times10^{-11}$ |
| **Adjusted for rs56093546 (P)** | 0,0068 | - | $7.2\times10^{-11}$ |
| **Adjusted for del12 (P)** | $6.4\times10^{-7}$ | $1.7\times10^{-6}$ | - |
| **Effect[a] on ALP (P value)** | 0.050 ($3.9\times10^{-21}$) | 0.068 ($7.4\times10^{-28}$) | 0.82 ($3.6\times10^{-63}$) |
| **Adjusted for rs314253 (P)** | - | $5.7\times10^{-12}$ | $4.1\times10^{-66}$ |
| **Adjusted for rs56093546 (P)** | 0,000042 | - | $2.0\times10^{-66}$ |
| **Adjusted for del12 (P)** | $4.2\times10^{-24}$ | $4.0\times10^{-31}$ | |
| **$r^2$, D' (relative to rs314253)** | - | 0.29, 0.76 | 0.001, 0.60 |
| **$r^2$, D' (relative to rs56093546)** | 0.29, 0.76 | - | 0.001, 1.00 |

[a]Effect estimates from the regression in units of standard deviations of the distributions of the adjusted values.

The association of rs314253 with LDL cholesterol was reported in Willer et al 2013 and with ALP in Chambers et al., 2011.

**Table 1.8. Association of p.w158X and del12 with Cholesterols, Triglyceride, Alkaline Phosphatase, Vitamin B12 and CAD in an extended Icelandic dataset**

| | p.W158X | | | del12 | | |
|---|---|---|---|---|---|---|
| Trait / (n)[a] | Effect[b] | Effect (95% CI)[c] | *P* | Effect" | Effect (95% CI)[c] | *P* |
| **Non-HDL cholesterol** | **SD** | **mg/dL** | | **SD** | **mg/dL** | |
| (136,261) | -0.45 | -21.6 (-34.2,- 9.6) | 0.00057 | -0.29 | -13.3 (-17.2,-9.3) | $4.0\times10^{-11}$ |
| **LDL cholesterol** | | **mg/dL** | | | **mg/dL** | |
| (53,932) | -0.38 | -15.9 (- 32.7,0.9) | 0.064 | -0.23 | -9.7 (-14.1,-5.1) | $2.8x10^{-5}$ |
| **Total cholesterol** | | **mg/dL** | | | **mg/dL** | |
| (131,879) | -0.30 | -13.5 (- 29.3,2.2) | 0.091 | -0.23 | -10.4 (-14.2,-6.5) | $1.4\times10^{-7}$ |
| **HDL cholesterol** | | **mg/dL** | | | **mg/dL** | |
| (124,437) | 0.14 | 2.4 (-3.9,8.7) | 0.45 | 0.15 | 2.5 (1.0,4.0) | 0.0016 |
| **Triglyceride** | | % change | | | % change | |
| (82,569) | -0.17 | -8.4 (- 25.5,7.2) | 0.33 | -0.12 | -6.0 (-10.4,-1.8) | 0.0075 |
| **ALP** | | % change | | | % change | |
| (131,966) | 0.77 | 45.3 (20.4,68.2) | $7.9\times10^{-6}$ | 0.80 | 47.7 (2.2,87.1) | $5.6\times10^{-76}$ |
| **Vitamin B12** | | % change | | | % change | |
| (102,624) | 0.26 | 15.6 (- 4.3,34.0) | 0.084 | 0.33 | 17.5 (3.1,30.9) | $5.6\times10^{-16}$ |
| **CAD** | | **OR** | ***P*** | | **OR** | ***P*** |
| (35,134/275,567) | | 0.61 (0.26,1.40) | 0.24 | | 0.66 (0.54,0.81) | $4.5\times10^{-5}$ |

[a]Number of individuals with trait value and genotypes. [b]Effect estimates from the regression in units of standard deviations (SD) of the distributions of the adjusted values. [c] Effect estimates and 95% confidence intervals (95% CI) in mg/dL for the cholesterols, and as percentage change for triglyceride, ALP and vitamin B12. [d]*P*-values adjusted for the effect of del12. This analysis was done on an updated Icelandic dataset that includes 8,453 WGS individuals and imputation into 150,656 Icelandic individuals. For none-HDL cholesterol association analysis an updated sample set was used that contained 136,261 Icelanders.

**Table 1.9A. Correlation and conditional analysis for del12 and the seven other SNPs that show the strongest association at 17p13.1 with non-HDL cholesterol in Iceland**

| | | | | | | non-HDL | | | |
|---|---|---|---|---|---|---|---|---|---|
| Variant | Pos | EA | OA | EA.freq (%) | $r^{2d}$ | Effect[c] | *P* | $P_{adjdel12}$[a] | $P_{adjSNP}$[b] |
| chr17:6930020:5 | 6930020 | T | C | 0.39 | 0.85 | -0.243 | $5.2\times10^{-7}$ | 0.10 | 2.8E-05 |
| rs188743906 | 6931736 | T | C | 0.39 | 0.85 | -0.243 | $5.2\times10^{-7}$ | 0.18 | 2.9E-05 |
| rs150983647 | 6942021 | T | C | 0.44 | 0.76 | -0.232 | $5.3\times10^{-7}$ | 0.39 | 7.6E-05 |
| chr17:6944653:5 | 6944653 | A | G | 0.39 | 0.85 | -0.242 | $5.9\times10^{-7}$ | 0.10 | 2.3E-05 |
| rs146261845 | 6952978 | T | C | 0.40 | 0.75 | -0.259 | $1.1\times10^{-7}$ | 0.88 | 0.00053 |
| chr17:6961021:5 | 6961021 | C | T | 0.39 | 0.85 | -0.250 | $2.2\times10^{-7}$ | 0.18 | 0.00010 |
| rs186021206 | 7010136 | A | G | 0.43 | 0.86 | -0.283 | $1.4\times10^{-9}$ | 0.39 | 0.067 |
| del12 | **7020979** | del12 | | 0.41 | | -0.297 | $2.5\times10^{-10}$ | - | - |

[a]*P*-value for correlation between the SNP and the trait, tested conditional on the association of the trait with del12. [b]*P*-value for the correlation between the trait and del12, tested conditional on the association of the trait with the SNP. [c]Effect estimated in units of standardized trait values. [d]Correlation $r^2$ between del12 and sequencing genotypes of the SNPs in 2,128 Icelandic individuals. Shown are the build 36 positions (hg18).

**Table 1.9B. Association of del12 with Non-HDL Cholesterol, HDL Cholesterol and Triglyceride Measurements, in Iceland, Denmark and the Netherlands**

| | Study population (η) | Change[a] ? SE | *P* value | Mean value[b] in non-carriers (SD) |
|---|---|---|---|---|
| | **Non-HDL cholesterol** | **mg/dl** | | **mg/dl** |
| Discovery | Iceland (119,146) | -10.4 ± 1.5 | $2.5\times10^{-10}$ | 156.8 (38.2) |
| Replication | The Netherlandsc (5,156) | -15.4 ± 5.4 | 0.0032 | 170.7 (41.3) |
| Replication | Denmark A[d] (5,968) | -17.4 ± 8.1 | 0.0069 | 158.3 (42.9) |
| Replication | Denmark B[e] (8,822) | -21.6 ± 5.4 | $3.8\times10^{-5}$ | 164.5 (40.5) |
| | Combined | -11.6 ± 1.5 | $1.0\times10^{-16}$ | |
| | **HDL cholesterol** | **mg/dl** | | **mg/dl** |
| Discovery | Iceland (119,514) | 0±0.4 | 0.0058 | 55.2 (15.8) |
| Replication | The Netherlands (5,537) | 2.7±1.5 | 0.20 | 52.2 (13.1) |
| Replication | Denmark A (6,182) | 1.2±2.7 | 0.096 | 55.2 (15.4) |
| Replication | Denmark B (9,656) | 1.5±1.2 | 0.26 | 59.9 (16.2) |
| | Combined | 0±0.4 | 0.00039 | |
| | **Triglyceride - mg/dl** | **mg/dl** | | **mg/dl** |
| Discovery | Iceland (80,011) | -1.2±1.5 | 0.012 | 130.9 (75.2) |
| Replication | The Netherlands (5,537) | -0.4±5.8 | 0.52 | 176.9 (121.2) |
| Replication | Denmark A (6,182) | 8.1±6.9 | 0.53 | 116.8 (84.0) |
| Replication | Denmark B (8,163) | -3.512.3 | 0.15 | 131.8 (118.5) |
| | Combined | -1.5±1.2 | 0.0030 | |

[a]Effect size, ± standard error, represents the difference in mean values between heterozygote carriers and non-carriers of the variants after adjusting for age, sex and, for Iceland, site and statin use. [b]Calculated based on unadjusted values. [c]The Nijmegen Biomedical Study (Wetzels et al. 2007). [d]The Danish Inter99 study (Jørgensen et al. 2003). [e]The Danish Addition study (Lauritzen et al. 2000). To convert the values for non-HDL cholesterol to millimoles per liter, multiply by 0.02586.

**Table 1.10. Association of del12 with Alkaline Phosphatase and Vitamin B12 Serum Measurements in Iceland and Denmark**

| | Study population (n) | Change[a] ± SE | *P* value | Mean value[b] in non- carriers (SD) |
|---|---|---|---|---|
| | **ALP** | **U/L** | | **U/L** |
| Discovery | Iceland (126,060) | + 33.6 ± 2.8 | $3.6\times10^{-63}$ | 92.8 (64.0) |
| Replication | Denmark A (5,829) | + 15.8 ± 2.6 | $1.7\times10^{-6}$ | 42.9 (13.5) |
| | Combined | + 24.1 ± 1.9 | $9.9\times10^{-69}$ | |
| | **Vitamin B12** | **pmol/L** | | **pmol/L** |
| Discovery | Iceland (97,910) | +58.4 ± 8.3 | $3.1\times10^{-12}$ | 439.0 (171.0) |
| Replication | Denmark A (5,826) | +75.9 ± 29.2 | 0.0069 | 420.0 (146.0) |
| | Combined | +59.7 ± 7.9 | $9.9\times10^{-14}$ | |

[a]Effect size, ± standard error, represents the difference in mean values between heterozygote carriers and non-carriers of the variants after adjusting for age, sex and, for Iceland, site and statin use. [b]Calculated based on unadjusted values. The Nijmegen Biomedical Study (Wetzels et al. 2007). [d]The Danish Inter99 study (Jørgensen et al. 2003). [e]The Danish Addition study (Lauritzen et al. 2000). To convert the values for non-HDL cholesterol to millimoles per liter, multiply by 0.02586.

## Example 2-ALP data from ASGR-1 Knockout mice

ASGR-1 KO mice (strain B6.129S4-*ASGR-1*$^{tm1Sau}$/SaubJxmJ) were obtained from Jackson Labs and maintained on a chow diet. Serum was collected from male and female animals after a 4hr fast and tested in an Olympus AU640 Clinical Chemistry Analyzer. Compared to wild- type mice, serum ALP is elevated in ASGR-1 knockout mice (*, $p < 0.05$; ****, $p < 0.0001$, one-way ANOVA with Dunnett test). Levels of alanine transaminase (ALT) and aspartate transaminase (AST) were not significantly different between the groups. These data are summarized in Figure 7 herein. WT=wild-type; HE=heterozygous; HO=homozygous.

**Example 3-RNAi**

**Material and Methods**

**siRNA Constructs**

Table 3.1:

| Vendor | Vendor catalog# | Primary Gene Target | Target Sequence | SEQ ID NO: | matched control | SEQ ID NO: |
|---|---|---|---|---|---|---|
| Fisher/ Ambion | S1662 | hASGR-1 | ACUUCACAGC GAGCACGGA | 32614 | ACUUCACACGC AGCACGGA | 32632 |
| GE/ Dharmacon | D-011215-01 | hASGR-2 | GCCAAGGACU UUCAAGAUA | 32615 | GCCAAGGAGAA UCAAGAUA | 32633 |
| GE/ Dharmacon | D-011215-03 | hASGR-2 | UGACGGAGGU CCAGGCAAU | 32616 | UGACGGAGCAG CAGGCAAU | 32634 |
| GE/ Dharmacon | D-011215-04 | hASGR-2 | AGUGAUGGCU CUUGGAAAU | 32617 | AGUGAUGGGAG UUGGAAAU | 32635 |
| Fisher/ Ambion | S1665 | hASGR-2 | GACUAUAGGC ACAACUACA | 32618 | GACUAUAGCGU CAACUACA | 32636 |
| Fisher/ Ambion | S194296 | hASGR-2 | CUGUGUGACUG GGUCCCAA | 32619 | CUGUGUGAGAC GGUCCCAA | 32637 |
| Fisher/ Ambion | S194297 | hASGR-2 | CACCUCUGGCU AACCCAUA | 32620 | CACCUCUGCGAA ACCCAUA | 32638 |
| GE/ Dharmacon | D-042958-01 | mASGR-1 | GAGACAGGCUU CCAGAAUU | 32621 | GAGACAGGGAA CCAGAAUU | 32639 |
| GE/ Dharmacon | D-042958-04 | mASGR-1 | UGAAGUUAGUG GAGUCGAA | 32622 | UGAAGUUACAC GAGUCGAA | 32640 |

(continued)

| Vendor | Vendor catalog# | Primary Gene Target | Target Sequence | SEQ ID NO: | matched control | SEQ ID NO: |
|---|---|---|---|---|---|---|
| Fisher/ Ambion | S62656 | mASGR-1 | AGAUCACUCCA GUUUGCUA | 32623 | AGAUCACUGGU GUUUGCUA | 32641 |
| Qiagen | S10273579 6 | mASGR-1 | CCAUCAUGACA AAGGAUUA | 32624 | CCAUCAUGUGU AAGGAUUA | 32642 |
| GE/ Dharmacon | D-061966-01 | mASGR-2 | GGAUGGAACU GAUUAUAGA | 32625 | GGAUGGAAGAC AUUAUAGA | 32643 |
| GE/ Dharmacon | D-061966-02 | mASGR-2 | GGAAUUGGGCC UUCACUCA | 32626 | GGAAUUGGCGG UUCACUCA | 32644 |
| GE/ Dharmacon | D-061966-03 | mASGR-2 | GACGGAACAUC ACCCACUA | 32627 | GACGGAACUAG ACCCACUA | 32645 |
| GE/ Dharmacon | D-061966-04 | mASGR-2 | GGAUAGGUCUU ACCGACAG | 32628 | GGAUAGGUGAA ACCGACAG | 32646 |
| GE/ Dharmacon | S62659 | mASGR-2 | GCAGGAUCCU AGGAUAGAA | 32629 | GCAGGAUCGAU GGAUAGAA | 32647 |
| Fisher/ Ambion | S62660 | mASGR-2 | ACAUUGCUCU UUCACCUGA | 32630 | ACAUUGCUGAA UCACCUGA | 32648 |
| Fisher/ Ambion | S62661 | mASGR-2 | GAAGAGUUUC GGACCCUGA | 32631 | GAAGAGUUAGC GACCCUGA | 32649 |

## Expression analysis

RNA was isolated from the HepG2, CHOs stable cell lines, or liver tissues treated with scrambled siRNA, matched control siRNA or siRNAs against hASGR-1, hASGR-2, mASGR-1 or mASGR-2 using the Qiacube and standard Qiagen RNA isolation protocol. The RNA was DNase treated using the RQ1 DNase kit (Promega). Quantitative PCR was performed according to the manufacturer's protocol on the Quantstudio 7 using the indicated primer probe set (hASGR-1: Hs01005019_ml; hASGR-2: Hs00910102_m1; mASGR-1: Mm01245581_ml, mASGR- 2:Mm00431863_ml) from Applied Biosystems. 50ng RNA/well was used and normalized with 18S internal control.

## siRNA transfection

Cells were transfected with 10nM indicated scrambled siRNA, matched control siRNA or siRNAs against hASGR-1, hASGR-2, mASGR-1 or mASGR-2 siRNA for 3-4 days, using Lipofectamine RNAIMAX (Thermo Scientific) following manufacturer's RNAi reverse transfection protocol. Transfection was done in 96 well Screenstar microplates (Greiner bio-one) for internalization assay as well as in 96 well clear tissue culture plates (Corning) for QPCR and Western blotting.

## Western Blotting

Cells were lysed in RIPA buffer containing inhibitors 3-4 days after siRNA transfection. Cell lysates were passed through a 21 gauge syringe five times and then centrifuged at 13000rpm at 4°C for 15mins. Supernatants were collected and protein concentrations were determined. If needed, 30ug of protein was deglycosylated using the deglycosylation kit (Genzyme). 10ug-30ug of total protein was loaded in each well. The gel was transferred onto a nitrocellulose membrane and the membrane was blocked with 5% blocking buffer for 1hr at RT. Membrane was then probed with anti-mASGR-1 (1:1000, R &D), hASGR-1 (1:1000, ProteinTech), hASGR-2 (1:1000, Abeam), anti-flag (1:5000, Sigma), anti-his (1:1000, Cell signaling) and mouse anti β-actin (1:5000, Thermo Fisher or Cell signaling) o/n at 4°C. The membrane was further probed with anti- mouse and anti-rat secondary antibodies to detection the indicated bands.

## Ligand Internalization assay

CHO stable cell lines were treated with scrambled siRNA, matched control siRNA or siRNAs against hASGR-1, hASGR-2, mASGR-1 or mASGR-2 siRNA for 3-4 days and plated in 96-well plate. Biotin-GalNAc-PAA was incubated and streptavidin-Alexa488 was further added to cells. Draq5 was used to counterstain cells (for both cytoplasm and nuclei). Cells were scanned with Operetta Image System and data analyzed by Columbus.

## Animal Study

All animal housing conditions and research protocols were approved by the Amgen Institutional Animal Care and Use Committee (IACUC). Mice were housed in a specified-pathogen free, AAALAC, Intl-accredited facility in ventilated microisolators. Procedures and housing rooms are positively pressured and regulated on a 12:12 dark:light cycle. All animals received reverse- osmosis purified water ad libitum via an automatic watering system. 10-12 week old C57BL/6J animals (The Jackson Laboratory) were singly housed and were fed standard chow (2020× Teklad global soy protein-free extruded rodent diet; Harlan).

siRNAs modified for in vivo studies were formulated with Invivofectamine 3.0 (Thermo Scientific) following the manufacturer's protocol. In brief, siRNAs were pre-mixed with complex buffer (provided by manufacturer) and Invivofectamine 3.0, and then incubated at 50°C for 30 minute and further diluted by PBS before injection.

Mice were i.v. injected with buffer, indicated siRNA and matched control siRNA at 1-2 mg/kg body weight in 0.25 ml buffer at indicated time. Liver total RNA from harvested animals was processed for qPCR analysis.

Data from these studies is provided in Figures 8-17 herein.

## Example 4- Y272C mutant data

Stable pools of Chinese hamster ovary (CHO) cells expressing C-terminal FLAG epitope-tagged murine wild-type or Y272C ASGR-1 were generated by established methods using puromycin selection. Cell surface expression of ASGR-1 was confirmed by FACS using anti-FLAG antibody both during selection process and at the time of the experiment. Ligand binding was assessed by FACS using β-GalNAc-PAA-biotin (Glycotech Corporation) and streptavidin-phycoerythrin (PE). Briefly, ligand was added to 100ul cells ($1\times10^6$cells) in Dulbeco's Modified Eagle Medium (DMEM) without phenol red plus 2% bovine serum albumin (BSA) and incubated on ice for 60 minutes. Cells were then washed 3x with DMEM without phenol red plus 2% BSA. Streptavidin-PE was then added at 1μg/ml for 20 minutes on ice followed by 3 more washes in DMEM without phenol red plus 2% BSA, at which point the cells were resuspended in 0.5ml DMEM without phenol red plus 2% BSA and 5ul of 0.1mM SyTOx Blue viability dye and analyzed on a BD LSR II (BD Biosciences). Data are presented as Median Fluorescence Intensity as shown in Table 4.1, below.

**Table 4.1**

| ASGR-1 Y272C has reduced ligand binding compared to wild-type ASGR-1 | | | | | |
|---|---|---|---|---|---|
| | **β-GalNAc-PAA-biotin, ug/ml** | | | | **Anti-FLAG antibody** |
| | **0** | **0.1** | **0.3** | | |
| Parental | 5.23 | 5.52 | 5.57 | | 7.3 |
| WT | 4.87 | 763.51 | 1394.86 | | 3959.65 |

(continued)

| ASGR-1 Y272C has reduced ligand binding compared to wild-type ASGR-1 | | | | | |
|---|---|---|---|---|---|
| | β-GalNAc-PAA-biotin, ug/ml | | | | Anti-FLAG antibody |
| | 0 | 0.1 | 0.3 | | |
| Y272C | 5.28 | 5.47 | 6.10 | | 973.38 |

## Example 5-generation of antibodies

### Molecular Cloning of ASGR-1 and ASGR-2 Sequences

For production of recombinant ASGR-1 and ASGR-2 vectors, cDNA sequences were synthesized, obtained from a commercial source or compiled from RNA sequencing data (Amgen). Human, mouse and rat ASGR cDNA clones were from obtained commercially (OriGene Technologies, Inc.). All other ASGR cDNAs were synthesized (Integrated DNA Technologies, Inc.). GenBank accession numbers are as follows: human ASGR-1 (NM_001671.4), human ASGR-2 (NM_080913.3), mouse ASGR-1 (BC022106.1), mouse ASGR-2 (BC011197.1), rat ASGR-1 (NM_012503), rat ASGR-2 (NM_017189), pig ASGR-1 (NM_001244458), pig ASGR-2 (XM_005669199), dog ASGR-1 (XM_546579), dog ASGR-2 (XM_003434599), cynomologus monkey ASGR-1 (XP_005582755). Since the NCBI entry for cynomologus ASGR-2 was a partial amino acid sequence (NCBI protein accession # EHH57653), the complete nucleotide sequence was compiled through the analysis of the cyno genome (genome build Macaca_fascicularis_5.0; GenBank accession number GCA_000364345.1; Washington University) and RNA sequencing data (Amgen) from cyno liver, heart and skin tissue. For transient or stable mammalian expression, cDNAs were cloned into pTT5 (National Research Council of Canada), pSLX235a (SureTech) or pJiF1 (Boyce Lab, Massachusetts General Hospital, US Patent: 7,192,933). For individual recombinant protein production in mammalian cells, most sequences were tagged at their C-termini with a 6xHis purification tag. For complexes of huASGR-1 and huASGR-2, huASGR-2 was expressed without the 6xHis tag. For recombinant expression in E. coli, sequences were cloned into pET21a (Novagen, EMD Millipore). The amino acid sequences of the resultant ASGR proteins are shown in Table 1.

### Expression and Purification of Recombinant Proteins

### Generation of Stable CHO-S Cell Pools for Recombinant Protein Expression

CHO-S (Invitrogen, Carlsbad, California) cells were transfected with the pSLX235a vector encoding ASGR-1 or ASGR-2 using Lipfectamine LTX according to the manufacturer's recommendations (ThermoFisher Scientific). Stable pools were selected using 10ug/ml puromycin (single selections) or 10ug/ml puromycin and 400ug/ml hygromycin (double selections) and by culturing the cells in fresh media every 2 days. Stable pools were then used for recombinant protein production.

### Recombinant Protein Production and Purification from CHO-S Cell Stable Pools

Cells from the selected stable pools were expanded in growth medium. When sufficient cell numbers had been obtained, cultures were seeded in 2L conical flasks in a volume of 1L of growth medium at a viable cell density of $8\times10^5$ cells/ml. Cells were then cultured in suspension at 37°C, in 5% $CO_2$ for three days, after which the temperature was dropped to 31°C for the final 7 days of production. Centrifugation was used to pellet the cells, and the resulting supernatant was filtered to generate conditioned medium.

Individual recombinant proteins were purified via the 6xHis tag using Ni-Excel resin (GE Healthcare). Briefly, 1.4L of conditioned medium was loaded onto 3× 5ml Ni-Excel Hi-trap columns and then washed with 10 column volumes of wash buffer (25mM HEPES, pH7.6, 250mM NaCl, 1mM CaCh, 50mM imidazole). Protein was eluted from the columns with 7 column volumes of elution buffer (25mM HEPES, pH7.6, 250mM NaCl, 1mM $CaCl_2$, 400mM imidazole). The eluted fractions were loaded onto a HiLoad Superdex 200 column via 2× 10ml injections and eluted with 25mM HEPES, pH 7.6; 150mM NaCl, 1mM CaCh. The final fractions were collected based on their expected molecular weight. The identity of the proteins in each eluted peak was confirmed by LC-TOF-MS after deglycosylation (with N-glycanase, O-glycanase and sialidase) and reduction. ASGR-1/ASGR-2 complexes were purified by pre-incubating the ASGR-1-6xHis Tag conditioned medium with ASGR-2-no 6xHis Tag conditioned medium. These conditions permitted association of both proteins giving a complex that could be purified via the standard two-step Ni-Excel/SEC method.

## Recombinant Protein Production and Purification from E. coli

E. coli codon optimized sequences were cloned into the pET21a expression plasmid. Plasmids were transformed into E.coli strain BL21(DE3) Star (ThermoFisher Scientific Inc.) and individual clones were selected using carbenicillin. For expression, cells were grown in 1L TB growth medium (supplemented with carbenicillin) in a 4L flask at 37°C with shaking. When an optical density of 2 was achieved, protein expression was induced by the addition of 1mM IPTG (final concentration). After 4 hours of induction at 37°C, the cell paste was harvested by centrifugation (recovering between 7 and 14 g cell paste/ L culture). Protein localization into the insoluble fraction was confirmed by SDS-PAGE.

Inclusion bodies were recovered from the cell paste and solubilized in 6M guanidinium containing 10mM DTT. Successful protein refolding was established by screening a matrix of 32 conditions that included a variety of buffers, pHs, denaturants, stabilizing agents and reducing agents. The refolding procedure was initiated by rapidly diluting the dissolved inclusion bodies at a ratio of 1:15 into the appropriate refold buffer, maintaining approximately 1mg of protein per condition. The samples were then incubated at 4°C for 60 hours. The resulting batches were analysed by SDS-PAGE and Ion Exchange chromatography to identify the optimal refolding conditions. For the ASGR-1 CBD (148-291), the final refold conditions were: pH 9.5, 2.5M urea, 20% glycerol, 4mM cysteine and 4mM cystamine.

## Generation of anti-ASGR Immune Responses

### Mouse Strains

Fully human antibodies to human ASGR were generated by immunizing XENOMOUSE® transgenic mice (U.S. Pat. Nos. 6,114,598; 6,162,963;6,833,268; 7,049,426; 7,064,244; Green et al., 1994, Nature Genetics 7:13-21; Mendez et al., 1997, Nature Genetics 15:146-156; Green and Jakobovitis, 1998, J. Ex. Med, 188:483-495; Kellerman and Green, Current Opinion in Biotechnology 13, 593-597, 2002). Animals from the XMG2-K, XMG2-KL, XMG4-K and XMG4-KL XENOMOUSE® strains were used for all immunizations.

Mouse anti-human ASGR antibodies were generated by immunizing BALB/c, C57BL/6 and CD-1 mice (Charles River Laboratories, San Diego, California) as well as B6.129S4- *ASGR-1*$^{tm1Sau}$/SaubJxmJ (ASGR-1 KO mice) and C57BL6 x129 F1 mice (Jackson Laboratory, Sacramento, CA).

Fully human, heavy chain only antibodies (HCAbs) were generated by immunizing the VH4 and 8V3 strains of transgenic Harbour mice (Janssens et al. 2006, PNAS 103:15130-15135; Harbour Biologics, Rotterdam, Netherlands). Rat anti-mouse ASGR antibodies were generated using Brown Norway Rats (Charles River Laboratories, San Diego, California).

## Immunizations

Multiple immunogens and routes of immunization were used to generate anti- human ASGR immune responses. For genetic immunizations, mice were immunized 12-14 times over 6-8 weeks using the Helios Gene Gun system according to the manufacturer's instructions (BioRad, Hercules, California). Briefly, expression vectors encoding wild type human or mouse ASGR-1 (or both huASGR-1+huASGR-2, muASGR-1+muASGR-2) were coated onto gold beads (BioRad, Hercules, California) and delivered to the epidermis of a shaved mouse or rat abdomen. For cell-based immunizations, mice and rats were immunized with CHO-s cells (Invitrogen, Carlsbad, California) or 293-6E cells (National Resource Council of Canada) transiently transfected with expression vectors encoding human or mouse ASGR-1 (or both huASGR-1+huASGR-2, muASGR-1+muASGR-2). Animals were immunized with cells mixed with Alum prepared from aluminum potassium sulfate (EMD Chemicals Inc., Gibbstown, NJ) and CpG-ODN (Eurofins MWG Operon LLC, Huntsville, AL) 10 times over 6 weeks using a protocol that alternated between sub-cutaneous and intraperitoneal injections. The initial boost was comprised of $4\times10^6$ cells while subsequent boosts contained $2\times10^6$ cells. For soluble protein immunizations, mice were immunized with a variety of human ASGR recombinant proteins representing the complete extracellular domain (ECD), the carbohydrate binding domain (CBD) or the complex of ASGR-1 and ASGR-2 ECDs (Table 5.1). Animals were immunized with recombinant protein (or recombinant protein conjugated to KLH using standard methods) mixed with Alum and CpG-ODN, Complete Freund's Adjuvant (Sigma), or MPL+ Adjuvant (Sigma) 10 times over 4-6 weeks using sub-cutaneous injections. The initial boost was comprised of 10μg while subsequent boosts contained 5-10μg. Human ASGR-1-specific serum titers were monitored by live- cell FACS analysis on an Accuri flow cytometer (BD Biosciences). Animals with the highest antigen- specific serum titers were sacrificed and used for hybridoma generation (Kohler and Milstein, 1975).

Table 5.1. Soluble, Recombinant Protein Antigens Used for Immunizations

| Recombinant Protein Immunogen | Source |
|---|---|
| huASGR-1 (Cat#:C428) ECD-KLH conjugate | Novoprotein |
| huASGR-1 (64-291) ECD-KLH conjugate | Amgen |
| huASGR-1 (64-291) ECD | Amgen |
| huASGR-1 (154-291) CBD | Amgen |
| huASGR-1(64-291)/huASGR-2 (61-287) ECD Complex | Amgen |
| huASGR-1(64-291)/huASGR-2 (61-287) ECD Complex-KLH conjugate | Amgen |
| muASGR-1 (63-284) | Amgen |

**Preparation of Monoclonal Antibodies**

**Hybridoma Generation**

Animals exhibiting suitable serum titers were identified and lymphocytes were obtained from spleen and/or draining lymphnodes. Pooled lymphocytes (from each immunization cohort) were dissociated from lymphoid tissue by grinding in a suitable medium (for example, Dulbecco's Modified Eagle Medium (DMEM); Invitrogen, Carlsbad, CA). B cells were selected and/or expanded using standard methods, and fused with a suitable fusion partner using techniques that were known in the art.

**Antigen Enrichment of Hybridoma Pools**

Fused hybridoma pools from each immune tissue harvest were used as a source of material for FACS-based enrichments using a variety of probes. To enrich for hybridomas expressing antibodies specific to native (full length, on-cell) human, cyno, mouse, rat, dog, or pig ASGR-1 (and native human ASGR-2) membranes were prepared from 293T cells transiently expressing the relevant ASGR cDNA construct. 24 hours after transfection using 293-fectin (ThermoFisher Scientific Inc.), cells were biotinylated with E-Z link NHS-LC-LC- Biotin according to the manufacturer's recommendation (ThermoFisher Scientific Inc.). After biotinylation, cells were homogenized with a needle and syringe to form membrane fragments and referred to as "membrane preps". The biotinylated membrane preps were then used to detect hybridomas expressing surface antibodies specific to the target of interest via standard biotin-streptavidin chemistry. To enrich for hybridomas capable of binding to the recombinant ASGR-1 ECD or CBD, soluble, 6xHis-tagged ASGR-1 proteins were used (Amgen).

To enrich hybridoma pools for the antigen of interest, they were first incubated with the appropriate membrane prep or soluble probe. For soluble forms of ASGR-1, the recombinant protein probes were added to the hybridomas and allowed to bind. Excess probe was then washed away and the antigen-specific hybridomas were identified by simultaneous detection of surface IgG (with an Alexa 488 conjugated secondary antibody (Jackson ImmunoResearch) (Gt anti-mouse Fc for wild type mouse hybridomas and Gt anti-human Fc for transgenic mouse hybridomas)) and the soluble ASGR-1 probe via its 6xHis tag (using an Amgen-derived anti-6xHis monoclonal antibody conjugated to Alexa 647 via an Alexa 647 labeling kit (ThermoFisher Scientific Inc). Hybridomas expressing surface IgG and binding antigen were detected by FACS analysis on an Accuri flow cytometer. Dual positive events were sorted as single cells into 384-well plates on a FACS Aria cell sorter (BD Biosciences). For native forms of ASGR-1, biotinylated membrane preps were prepared as described from 293T cells transiently expressing the appropriate antigen. After washing away unbound probe, dual positive hybridomas expressing cell surface IgG and binding antigen were detected using an Alexa 488 conjugated secondary antibody (to detect IgG) and streptavidin conjugated to Alexa 647 (Jackson ImmunoResearch) to detect antigen. These events were sorted as single cells into 384-well plates on a FACS Aria cell sorter. After several days of culture, the hybridoma supernatants containing monoclonal antibodies were collected and used in the screening assays described in the examples below.

**Example 6: Identification of ASGR-1 Specific Antibodies**

The following Table 6.1 summarizes the approximate numbers of antibodies assayed:

**Table 6.1. Summary of the identification and selection of huASGR-1 binding, ligand blocking antibodies.**

| ASGR-1 Screen | Number of Antibodies |
|---|---|
| huASGR-1 Binders | 15731 |
| huASGR-1-Ligand Blockers (>60%) | 5306 |
| Sequences Unique huASGR-1-Ligand Blockers | 2603 (disclosed in Table 3) |
| huASGR-1-Ligand Blockers (>50%) | 172 (disclosed in Table 3) |

**Example 6-A: Initial Selection of ASGR-1 Specific Binding Antibodies**

Hybridoma supernatants (monoclonal antibodies) were screened for binding to human ASGR-1 transiently expressed on Human Embryonic Kidney (HEK) 293 cells using the Cell Insight™ High Content Imaging Platform (ThermoFisher Scientific). Human ASGR-1 was transiently expressed on host HEK 293 cells by transfection using human ASGR-1 DNA, Gibco™ Opti-MEM® media and 293Fectin™ reagents following the protocol set out by the manufacturer. Transfected HEK 293 cells expressing the human ASGR-1, hybridoma supernatant or control samples, Alexa Fluor® 488 IgG Fc fragment-specific detection antibody and Hoechst 33342 stain were mixed and incubated for 3 hours at room temperature. Samples were then washed and analyzed on the CellInsight™ system. Supernatants were counter-screened against HEK 293 cells transfected with empty parental vector (referred to as mock). Analysis was done using irrelevant IgG antibody supernatant sample signal; hybridoma supernatant samples showing two times or greater signal over irrelevant IgG antibody sample were considered to be exhibiting ASGR-1-specific binding profiles and selected for further characterization. See Table 6.1.

**Example 6-B: Identification of ASGR-1 Receptor-Ligand Blocking Antibodies**

ASGR-1-binding hybridoma supernatants were tested for their ability to block ASGR-1 from binding ligand. Competitive binding assays were performed on the antigen specific hybridoma supernatant samples using FACS on either HEK 293 cells transiently expressing human ASGR-1 or CHO-S cells stably expressing Human ASGR-1 as follows. HEK 293 cells or CHO-S cells expressing human ASGR-1 were mixed with the antibody sample (hybridoma supernatants specific for ASGR-1) and incubated for 1 hour at 4°C, and then washed twice. Cells with bound sample were then incubated with precomplexed β-GalNAc-PAA-Biotin (GlycoTech, Gaithersburg, Maryland)/Alexa Fluor® 647- Streptavidin for 45 minutes at 4°C. The concentration of β-GalNAc- PAA-Biotin was used at the binding EC50 concentration on the specific cell line. The concentration of Alexa Fluor® 647 Streptavidin was used at a 2:1 molar ratio to β-GalNAc-PAA-Biotin. The 7- AAD cell viability stain was then added and the cells incubated for a further 15 minutes at 4°C, washed twice and resuspended in FACS buffer. Where tolerated by cell viability, FACS buffer supplemented with 1mM Calcium Chloride was used in all steps. Samples were analyzed using a BD Accuri™ Flow Cytometer and an Intellicyt HyperCyt Autosampler. Analysis was done using irrelevant (non-ASGR- 1 specific) IgG antibody supernatant control signal on both mock transfected HEK 293 cells and Human ASGR-1 transfected HEK 293 cells to determine maximum and minimum β-GalNAc-PAA- Biotin binding signal. Using these maximum and minimum binding signals, the % β-GalNAc-PAA- Biotin binding inhibition was determined. ASGR-1 antibodies having the ability to reduce ligand binding ≥60% were identified (Table 6.1), and sequenced using methods available to those skilled in the art. The sequences of unique ASGR-1-specific, ligand blocking antibodies are displayed in Table 2-7 herein.

The unique ASGR-1-specific, ligand blocking antibodies were then tested for their ability to block the GalNAc ligand under more stringent conditions using a single, known antibody concentration (5ug/ml). The receptor-ligand blocking assays were performed using 293T cells transiently expressing ASGR-1 or CHOs cells that had been stably transfected with ASGR-1. ASGR- 1 antibodies having the ability to reduce ligand binding ≥50% were identified. See Table 6.1.

**Example 7: Antibody Characterization Assays**

**A. ASGR-1 Species Cross Reactivity, ASGR-2 Selectivity Assays and Hepatoma (HEPG2) Binding Assays**

Human ASGR-1-specific, ligand competing antibody samples were tested for binding to ASGR-1 from other species (cynomologus monkey ASGR-1, mouse ASGR-1, rat ASGR- 1, dog ASGR-1, and pig ASGR-1) as well as to human ASGR-2 in FACS binding assays at normalized antibody concentrations. For cell-based assays, HEK 293 cells expressing the appropriate antigen of interest were mixed with antibody sample or controls, incubated for 1 hour at 4°C, and then washed twice. Cells with bound antibody were then incubated with Alexa Fluor® 647 IgG Fc fragment- specific detection antibody and 7-AAD viability stain for 15 minutes at 4°C, washed once and resuspended in FACS buffer. Samples were analyzed using a BD Accuri™ Flow Cytometer and an Intellicyt HyperCyt Autosampler. As a negative control, supernatants and controls were also screened against HEK 293 cells transfected with empty parental vector. Analysis was done using irrelevant (non-ASGR-1 specific) IgG antibody supernatant sample signal; hybridoma supernatant samples showing at least two times the signal over irrelevant IgG antibody sample were considered to be exhibiting ASGR-1- species specific binding profiles. For membrane-prep binding assays, ASGR-1 species specific membrane preps were used to coat LumAvidin® microspheres (beads) and tested for binding to selected hybridoma supernatants or controls. Briefly, ASGR-1 species specific membrane preps were incubated with streptavidin-coated LumAvidin® beads for 45 minutes in the dark at room temperature and washed twice. Beads were resuspeneded in FACS buffer containing Stabilguard®. Antigen-bound beads were then incubated with normalized antibody sample for 1 hour in the dark at room temperature, washed twice, incubated with Alexa Fluor® 488 IgG Fc fragment-specific detection antibody for 15 minutes in the dark at room temperature, washed once and finally resuspended in FACS buffer. Samples were analyzed using an Intellicyt iQue™ Screener Platform. FACS buffer supplemented with 1mM Calcium Chloride was used in all steps. As a negative control, supernatants and controls were also screened against a non-ASGR-1 antigen membrane prep coated on the LumAvidin® beads. Analysis was done using irrelevant (non-ASGR-1 specific) IgG antibody supernatant sample signal; hybridoma supernatant samples showing at least two times the signal over irrelevant IgG antibody sample were considered to be exhibiting specific binding profiles. See Table 7.1.

Human ASGR-1-specific, ligand competing hybridoma supernatant samples were screened for binding to the human hepatocellular carcinoma cell line HepG2 (ATCC HB-8065) at normalized antibody concentrations. For FACS binding assays, HepG2 cells were mixed with normalized antibody samples or controls, incubated for 1 hour at 4°C, and washed twice. Cells with bound antibody were then incubated with Alexa Fluor® 647 IgG Fc fragment-specific detection antibody and 7-AAD viability stain for 15 minutes at 4°C, washed once and resuspended in FACS buffer. Samples were analyzed using a BD Accuri™ Flow Cytometer and an Intellicyt HyperCyt Autosampler. For high content imaging binding assays, HepG2 cells were mixed with normalized antibody samples or controls, incubated for 1 hour at room temperature and washed twice. Cells with bound antibody were then incubated with Alexa Fluor® 488 IgG Fc fragment-specific detection antibody and Hoechst 33342 stain for 30 minutes at room temperature, washed twice and analyzed on the CellInsight™ system. Where tolerated by cell viability, FACS buffer supplemented with 1mM Calcium Chloride was used in all steps. Analysis was done using irrelevant (non-ASGR-1 specific) IgG antibody supernatant sample signal; hybridoma supernatant samples showing two times or greater signal over irrelevant IgG antibody sample were considered to be exhibiting HepG2 ASGR-1 specific binding profiles. See Table 7.1.

Table 7.1. Summary of the binding specificities of the selected human ASGR-1 binding antibodies.

| | **Binding Data Summary** | | | | | | | |
|---|---|---|---|---|---|---|---|---|
| **mAb** | **Human ASGR-1** | **Cyno ASGR- 1** | **Mouse ASGR- 1** | **Rat ASGR- 1** | **Dog ASGR- 1** | **Pig ASGR- 1** | **HEPG2 Cells** | **Human ASGR- 2** |
| 25A4 | Y | Y | N | Y | N | Y | Y | N |
| 26C4 | Y | Y | N | Y | N | Y | Y | N |
| 29H8 | Y | Y | N | Y | N | Y | Y | N |
| 4A2 | Y | Y | N | Y | N | Y | Y | N |
| 4H6 | Y | Y | Y | Y | N | Y | Y | N |
| 56E5 | Y | Y | N | N | N | Y | Y | N |
| 7F4 | Y | Y | N | no data | Y | Y | Y | Y |
| 7G4 | Y | Y | N | N | N | Y | Y | N |
| 48B12 | Y | Y | N | N | N | Y | Y | N |
| 184E7 | Y | Y | Y | Y | Y | Y | Y | N |
| 194A4 | Y | Y | N | Y | Y | Y | Y | N |
| 4B1 | Y | Y | Y | Y | Y | Y | Y | N |
| 72G9 | Y | Y | Y | Y | Y | Y | Y | N |

(continued)

| | Binding Data Summary | | | | | | | |
|---|---|---|---|---|---|---|---|---|
| mAb | Human ASGR-1 | Cyno ASGR- 1 | Mouse ASGR- 1 | Rat ASGR- 1 | Dog ASGR- 1 | Pig ASGR- 1 | HEPG2 Cells | Human ASGR- 2 |
| 190F8 | Y | Y | N | N | Y | Y | Y | N |
| 191G1 | Y | Y | N | N | Y | Y | Y | N |
| 191G10 | Y | Y | N | N | Y | Y | Y | N |
| 194C1 | Y | Y | N | N | Y | Y | Y | N |
| 197G3 | Y | Y | N | N | Y | Y | Y | N |
| 198G3 | Y | Y | N | N | Y | Y | Y | N |
| 75G3 | Y | Y | N | N | Y | Y | Y | N |
| 218G4 | Y | Y | N | N | Y | Y | Y | N |
| 193E7 | Y | Y | N | N | Y | N | Y | N |
| 198D2 | Y | Y | N | Y | N | Y | Y | N |
| 202A3 | Y | Y | N | N | Y | Y | Y | N |
| 7E11 | Y | Y | N | N | N | Y | Y | N |
| 22G5 | Y | Y | N | N | N | N | Y | N |
| 5E5 | Y | Y | N | Y | N | N | Y | N |
| 54E9 | Y | Y | N | N | Y | N | Y | N |
| 6G7 | Y | Y | N | Y | N | N | Y | Y |
| 176H4 | Y | Y | N | N | Y | Y | Y | N |
| 194C10 | Y | Y | N | N | Y | Y | Y | N |
| 12D2 | Y | Y | Y | Y | Y | Y | Y | N |

**B. Relative Binding Affinities for ASGR-Specific mAbs**

To assess antibody and antigen interaction strength (relative binding affinity), ASGR-1 specific, ligand competing antibody hybridoma supernatants were tested in a limiting antigen binding assay. Titrated amounts of recombinant, soluble ASGR-1 biotinylated protein was incubated with streptavidin-coated LumAvidin Beads® for 45 minutes in the dark at room temperature and washed twice. Beads were resuspeneded in FACS buffer containing Stabilguard® and 0.05% Sodium Azide. Antigen-bound beads were then incubated with normalized hybridoma supernatant sample or controls for 18 hours in the dark at room temperature, washed twice, incubated with Alexa Fluor® 488 IgG fragment-specific detection antibody for 15 minutes in the dark at room temperature, washed once and finally resuspended in FACS buffer. Samples were analyzed using an Intellicyt iQue™ Screener Platform. FACS buffer supplemented with 1mM Calcium Chloride was used in all steps. Analysis was done using irrelevant (non-ASGR-1 specific) IgG antibody supernatant sample signal; hybridoma supernatant samples showing at least two times or greater signal over irrelevant IgG antibody sample were considered to be exhibiting ASGR-1 specific binding profiles. In this assay method, the antibody binding signal correlates with antibody affinity. Antibody binding data for a representative antigen coating concentration that fell in the linear range of the instrument signal detection is shown in Table 7.2. The degree of antibody binding to the target (ASGR-1) correlates with the measured fluorescent intensity and thus allows a relative comparison of affinities across the panel.

Table 7.2 Limited Antigen Binding Assay to Assess Relative Affinities of selected mAbs

| mAb | Soluble ASGR-1 2.5 ng/mL (FACS Geomean) | mAb | Soluble ASGR-1 2.5 ng/mL (FACS Geomean) | mAb | Soluble ASGR-1 2.5 ng/mL (FACS Geomean) | mAb | Soluble ASGR-1 2.5 ng/mL (FACS Geomean) |
|---|---|---|---|---|---|---|---|
| 25A4 | 17952 | 48B12 | 26989 | 194C1 | 16937 | 7E11 | 4662 |
| 26C4 | 12007 | 184E7 | 40198 | 197G3 | 17708 | 22G5 | 1078 |
| 29H8 | 12179 | 194A4 | 38934 | 198G3 | 25969 | 5E5 | 3278 |
| 4A2 | 16604 | 4B1 | 10060 | 75G3 | 35840 | 54E9 | 6487 |
| 4H6 | 2990 | 72G9 | 34014 | 218G4 | 15105 | 6G7 | 2290 |
| 56E5 | 22648 | 190F8 | 13899 | 193E7 | 18315 | 176H4 | 29444 |
| 7F4 | 4910 | 191G1 | 9546 | 198D2 | 1872 | 194C10 | 21854 |
| 7G4 | 6795 | 191G10 | 24154 | 202A3 | 2152 | 12D2 | 105 |

### C. pH and Calcium Sensitivity

This Example characterizes ASGR-1 antibodies based on the effect of pH and/or calcium on their ability to bind the target. For this example, a label-free, kinetic antibody-ASGR-1 binding assay was employed to assess the sensitivity of the antibodies to changes in pH and calcium. Briefly, the ASGR-1 -specific, ligand-competing antibodies were first immobilized and then allowed to bind recombinant, soluble huASGR-1 under physiological conditions (ie. pH 7.4, 1mM CaCl2). The amount of binding was determined and set to 100%. In order to determine if the antibody- ASGR-1 interaction was sensitive to changes in pH or Ca, the assay buffer was then changed to conditions lacking calcium, a reduced pH (pH 5.6) or both lacking calcium and reduced pH (pH 5.6), and dissociation of ASGR-1 from the mAbs monitored. The amount of ASGR-1 remaining bound under each condition was assessed and expressed as a percent of the starting signal. If a >10% difference in ASGR-1 binding signal was calculated (when compared to that measured under physiological conditions), a particular antibody was classified as being sensitive to that condition. Using this method, the selected antibodies were classified into 5 categories:

1. affected by the removal of calcium
2. unaffected by the removal of calcium or drop in pH
3. affected when both calcium is removed and pH is dropped
4. affected by calcium removal, pH drop and both combined
5. affected by the drop in pH

The relative dissociation of ASGR-1 from antibodies was measured using a label- free assay on an OctetHTX instrument (Fortebio). Antibody samples were captured on anti-HuFc kinetic biosensors (ForteBio cat # 18-5064) at 5 ug/mL in assay buffer (10 mM Tris, 0.1 % Triton, 150 mM NaCl, 1 mg/mL BSA, 1 mM CaCl2, pH7.4) for three minutes. A one minute baseline stabilization step was performed in assay buffer. Soluble ASGR-1 (Amgen) at 6 ug/ml in assay buffer was added and association to the antibodies was monitored for two minutes. Subsequent dissociation of ASGR-1 from the antibodies was performed by incubating the ASGR-1-mAb complexes for 10 minutes under each of the following conditions:

| | |
|---|---|
| pH 7.4 + calcium | 10mM Tris, 0.1%Triton, 150mM NaCl, 1mg/mL BSA, pH7.4, 1mM CaCl2 |
| pH 7.4 - calcium | 10mM Tris, 0.1%Triton, 150mM NaCl, 1mg/mL BSA, pH7.4 |
| pH 5.6 + calcium | 10mM Tris, 0.1%Triton, 150mM NaCl, 1mg/mL BSA, pH5.6, 1mM CaCl2 |
| pH 5.6 - calcium | 10mM Tris, 0.1%Triton, 150mM NaCl, 1mg/mL BSA, pH5.6 |

The binding signal at the end of the 2 minute association phase for each dissociation experiment was set to 100% and used to represent the maximal level of ASGR-1 binding. After 1 minute of dissociation, the percentage of ASGR-1 remaining bound was calculated. The lower the percent remaining at a given time point indicates increased levels of dissociation in response to the test conditions (ie. different pH and/or calcium concentrations). The change in the percentage of ASGR-1 remaining bound in response to each test condition relative to the percent remaining in the control conditions (ie. pH 7.4 + calcium) was determined. Cut-offs for an antibody to be categorized as being sensitive to a particular condition were set to >10% (ie. if >10% of the ASGR-1 dissociates from the antibody under a particular test condition compared to control condition, it was deemed sensitive to that condition). The analysis was done using the 1 minute dissociation time point (except for mAb 149A1 which was binned based on the 4 minute dissociation time point). Using this analysis, the ASGR-1-binding, receptor-ligand blocking antibodies were separated into groups according to their dissociation profiles in response to pH and calcium (Table 7.3). Antibodies belonging to each category were observed.

Table 7.3. pH and Calcium Sensitivity of ASGR-1-mAb Interactions

| **pH and Calcium Sensitivity Determination (% Change Compared to pH 7.4 + Calcium)** | | | | | | | |
|---|---|---|---|---|---|---|---|
| **mAb** | **pH 7.4 minus Calcium** | **pH 5.6 plus Calcium** | **pH 5.6 minus Calcium** | **Calcium sensitive** | **pH sensitive** | **pH and calcium sensitive** | **pH bin** |
| 10G6 | 7% | 4% | 15% | N | N | Y | 3 |
| 148E10 | 7% | 19% | 33% | N | Y | Y | 5 |
| 154F4 | 10% | 41% | 67% | N | Y | Y | 5 |
| 159H8 | 6% | 10% | 26% | N | Y | Y | 5 |
| 160B12 | 6% | 8% | 22% | N | N | Y | 3 |
| 175D10 | 4% | -3% | 2% | N | N | N | 2 |
| 177D2 | 3% | 2% | 10% | N | N | Y | 3 |
| 25A4 | 2% | -3% | -1% | N | N | N | 2 |
| 26C4 | 3% | 2% | 2% | N | N | N | 2 |
| 27E7 | 20% | 35% | 46% | Y | Y | Y | 4 |
| 29E2 | 5% | 25% | 38% | N | Y | Y | 5 |
| 29H8 | 2% | -2% | 2% | N | N | N | 2 |
| 31D12 | 10% | 27% | 34% | Y | Y | Y | 4 |
| 32D6 | 26% | 33% | 55% | Y | Y | Y | 4 |
| 45B4 | 4% | 10% | 23% | N | Y | Y | 5 |
| 49F10 | 4% | -2% | 8% | N | N | N | 2 |
| 4A2 | 1% | -3% | 1% | N | N | N | 2 |
| 4B3 | 12% | 33% | 45% | Y | Y | Y | 4 |
| 4H6 | 5% | -1% | 2% | N | N | N | 2 |
| 50D4 | 6% | 0% | 9% | N | N | N | 2 |
| 50G9 | 37% | 62% | 44% | Y | Y | Y | 4 |
| 51E9 | 3% | -5% | 2% | N | N | N | 2 |
| 52G11 | 15% | 1% | 13% | Y | N | Y | 1 |
| 52H1 | 5% | -1% | 10% | N | N | N | 2 |
| 53F2 | 15% | 1% | 13% | Y | N | Y | 1 |
| 53F7 | 9% | 3% | 13% | N | N | Y | 3 |
| 55B1 | 5% | -2% | 4% | N | N | N | 2 |
| 56E5 | 1% | -6% | -1% | N | N | N | 2 |

(continued)

| pH and Calcium Sensitivity Determination (% Change Compared to pH 7.4 + Calcium) | | | | | | | |
|---|---|---|---|---|---|---|---|
| mAb | pH 7.4 minus Calcium | pH 5.6 plus Calcium | pH 5.6 minus Calcium | Calcium sensitive | pH sensitive | pH and calcium sensitive | pH bin |
| 57A7 | 13% | 13% | 29% | Y | Y | Y | 4 |
| 58G11 | 38% | 12% | 51% | Y | Y | Y | 4 |
| 59F2 | 48% | 52% | 74% | Y | Y | Y | 4 |
| 5E5 | 7% | 18% | 42% | N | Y | Y | 5 |
| 60D2 | 20% | 42% | 49% | Y | Y | Y | 4 |
| 60E8 | 3% | 11% | 18% | N | Y | Y | 5 |
| 63A10 | 8% | 3% | 47% | N | N | Y | 3 |
| 63G7 | 20% | 15% | 59% | Y | Y | Y | 4 |
| 64B12 | 6% | 6% | 7% | N | N | N | 2 |
| 65F10 | 25% | 18% | 37% | Y | Y | Y | 4 |
| 68G6 | 22% | 39% | 47% | Y | Y | Y | 4 |
| 6D9 | 14% | 25% | 42% | Y | Y | Y | 4 |
| 6G6 | 1% | -3% | 0% | N | N | N | 2 |
| 70D1 | 17% | 12% | 29% | Y | Y | Y | 4 |
| 7E11 | 9% | 5% | 14% | N | N | Y | 3 |
| 7F4 | 4% | 6% | 9% | N | N | N | 2 |
| 7G4 | 2% | 1% | 7% | N | N | N | 2 |
| 9G9 | 25% | 38% | 55% | Y | Y | Y | 4 |
| 65E9 | 22% | 30% | 35% | Y | Y | Y | 4 |
| 72B4 | 32% | 26% | 43% | Y | Y | Y | 4 |
| 147D10 | 13% | 4% | 11% | Y | N | Y | 1 |
| 149D11 | 11% | 3% | 11% | Y | N | Y | 1 |
| 149F8 | 1% | -8% | -1% | N | N | N | 2 |
| 22G5 | 40% | 35% | No Data | Y | Y | No Data | 4* |
| 48B12 | 4% | -6% | 0% | N | N | N | 2 |
| 52H2 | 26% | 11% | 32% | Y | Y | Y | 4 |
| 6G7 | 8% | 4% | 16% | N | N | Y | 3 |
| 64G12 | 24% | 10% | 24% | Y | N | Y | 1 |
| 72F5 | 64% | 20% | 30% | Y | Y | Y | 4 |
| 147E9 | 5% | -4% | 20% | N | N | Y | 3 |
| 184E7 | 1% | -9% | -3% | N | N | N | 2 |
| 194A4 | -1% | -7% | -3% | N | N | N | 2 |
| 208A2 | -4% | -10% | -5% | N | N | N | 2 |
| 210G10 | -3% | -10% | -5% | N | N | N | 2 |
| 4B1 | 6% | -5% | -2% | N | N | N | 2 |

(continued)

| pH and Calcium Sensitivity Determination (% Change Compared to pH 7.4 + Calcium) | | | | | | | |
|---|---|---|---|---|---|---|---|
| mAb | pH 7.4 minus Calcium | pH 5.6 plus Calcium | pH 5.6 minus Calcium | Calcium sensitive | pH sensitive | pH and calcium sensitive | pH bin |
| 62H10 | 13% | -2% | 14% | Y | N | Y | 1 |
| 72G9 | 1% | -7% | -1% | N | N | N | 2 |
| 148H10 | 45% | 10% | 47% | Y | N | Y | 1 |
| 173C11 | 17% | 0% | 29% | Y | N | Y | 1 |
| 179C2 | 25% | 0% | 45% | Y | N | Y | 1 |
| 47C1 | 13% | -1% | 10% | Y | N | Y | 1 |
| 49C1 | 72% | 23% | 64% | Y | Y | Y | 4 |
| 60C12 | 14% | -3% | 12% | Y | N | Y | 1 |
| 60G2 | 36% | 7% | 31% | Y | N | Y | 1 |
| 65D5 | 34% | 7% | 61% | Y | N | Y | 1 |
| 66H11 | 81% | 36% | 52% | Y | Y | Y | 4 |
| 73G1 | 100% | 33% | 62% | Y | Y | Y | 4 |
| 51E3 | 65% | 16% | 42% | Y | Y | Y | 4 |
| 53E8 | 68% | 20% | 64% | Y | Y | Y | 4 |
| 54E9 | 79% | 24% | 75% | Y | Y | Y | 4 |
| 56E3 | 75% | 21% | 16% | Y | Y | Y | 4 |
| 190C11 | -1% | -6% | -6% | N | N | N | 2 |
| 190E6 | -1% | -12% | -6% | N | N | N | 2 |
| 190F12 | -1% | -6% | -6% | N | N | N | 2 |
| 190F8 | -1% | -5% | -5% | N | N | N | 2 |
| 190G11 | -2% | -8% | -5% | N | N | N | 2 |
| 190H9 | -1% | -6% | -7% | N | N | N | 2 |
| 191A10 | 0% | -5% | -5% | N | N | N | 2 |
| 191G1 | -10% | -15% | -11% | N | N | N | 2 |
| 191G10 | 0% | -5% | -5% | N | N | N | 2 |
| 191G12 | -2% | -5% | -6% | N | N | N | 2 |
| 192C10 | -1% | -6% | -6% | N | N | N | 2 |
| 192C8 | -9% | -14% | -14% | N | N | N | 2 |
| 192E4 | -2% | -9% | -8% | N | N | N | 2 |
| 192G6 | -1% | -6% | -6% | N | N | N | 2 |
| 192G8 | -1% | -5% | -6% | N | N | N | 2 |
| 192H10 | 0% | -5% | -4% | N | N | N | 2 |
| 193C7 | -1% | -8% | -8% | N | N | N | 2 |
| 194B7 | 1% | -4% | -4% | N | N | N | 2 |
| 194C1 | -7% | -12% | -8% | N | N | N | 2 |

(continued)

| pH and Calcium Sensitivity Determination (% Change Compared to pH 7.4 + Calcium) | | | | | | | |
|---|---|---|---|---|---|---|---|
| mAb | pH 7.4 minus Calcium | pH 5.6 plus Calcium | pH 5.6 minus Calcium | Calcium sensitive | pH sensitive | pH and calcium sensitive | pH bin |
| 196C7 | -8% | -12% | -12% | N | N | N | 2 |
| 197B6 | -1% | -8% | -7% | N | N | N | 2 |
| 197E11 | -1% | -5% | -4% | N | N | N | 2 |
| 197F2 | 0% | -6% | -6% | N | N | N | 2 |
| 197G3 | 2% | -3% | -3% | N | N | N | 2 |
| 198G3 | -1% | -4% | -4% | N | N | N | 2 |
| 213B3 | -1% | -7% | -3% | N | N | N | 2 |
| 219H1 | 2% | -3% | 1% | N | N | N | 2 |
| 74C8 | 1% | -7% | -3% | N | N | N | 2 |
| 74G6 | 1% | -9% | -4% | N | N | N | 2 |
| 75G3 | -1% | -1% | 2% | N | N | N | 2 |
| 74B2 | 8% | -9% | -5% | N | N | N | 2 |
| 74H7 | 1% | -2% | 1% | N | N | N | 2 |
| 85F7 | 2% | -2% | 2% | N | N | N | 2 |
| 198B9 | 3% | 2% | 11% | N | N | Y | 3 |
| 199A7 | 1% | 1% | 10% | N | N | Y | 3 |
| 218G4 | 1% | -4% | 0% | N | N | N | 2 |
| 146A8 | 2% | -9% | 25% | N | N | Y | 3 |
| 146B6 | 2% | -5% | 13% | N | N | Y | 3 |
| 149A1 | 2% | -7% | 9% | N | N | Y | 3* |
| 172B12 | -14% | -27% | -13% | N | N | N | 2 |
| 172C3 | -9% | -26% | 0% | N | N | N | 2 |
| 193E7 | -9% | -9% | -4% | N | N | N | 2 |
| 199E3 | -5% | -4% | -4% | N | N | N | 2 |
| 226F9 | 100% | 51% | 77% | Y | Y | Y | 4 |
| 227C1 | 100% | 54% | 73% | Y | Y | Y | 4 |
| 227F2 | 80% | 50% | 100% | Y | Y | Y | 4 |
| 65C12 | 13% | 0% | 23% | Y | N | Y | 1 |
| 176H4 | 2% | -4% | 26% | N | N | Y | 3 |
| 194C10 | 2% | 10% | 16% | N | Y | Y | 5 |
| 191E10 | -1% | -9% | -9% | N | N | N | 2 |
| 196F4 | -8% | -5% | -6% | N | N | N | 2 |
| 198D2 | -8% | -30% | -28% | N | N | N | 2 |
| 202A3 | -21% | -22% | -23% | N | N | N | 2 |
| 204G6 | -5% | -11% | -10% | N | N | N | 2 |

(continued)

| pH and Calcium Sensitivity Determination (% Change Compared to pH 7.4 + Calcium) | | | | | | | |
|---|---|---|---|---|---|---|---|
| **mAb** | **pH 7.4 minus Calcium** | **pH 5.6 plus Calcium** | **pH 5.6 minus Calcium** | **Calcium sensitive** | **pH sensitive** | **pH and calcium sensitive** | **pH bin** |
| 224G1 | 77% | 41% | 65% | Y | Y | Y | 4 |
| 52D10 | 21% | 3% | 45% | Y | N | Y | 1 |
| 64E2 | 48% | 29% | 49% | Y | Y | Y | 4 |
| *No actual data; bin predicted on the totality of information regarding the antibody. | | | | | | | |

**D. Relative Epitope Binning/Profiling**

A common way to characterize epitopes is through competition experiments. Antibodies that compete with each other can be thought of as binding the same or overlapping site on the target. This example describes a method of determining competition for binding to hASGR-1 and the results of the method when applied to a number of antibodies described herein.

Binning experiments can be conducted in a number of ways, and the method employed may have an effect on the assay results. Common to these methods is that ASGR-1 is typically bound by one reference antibody and probed by another. If the reference antibody prevents the binding of the probe antibody then the antibodies are said to be in the same bin. The order in which the antibodies are employed is important. If antibody A is employed as the reference antibody and blocks the binding of antibody B the converse is not always true: antibody B used as the reference antibody will not necessarily block antibody A. There are a number of factors in play here: the binding of an antibody can cause conformational changes in the target which prevent the binding of the second antibody, or epitopes which overlap but do not completely occlude each other may allow for the second antibody to still have enough high-affinity interactions with the target to allow binding. In general, if competition is observed in either order the antibodies are said to bin together, and if both antibodies can block each other then it is likely that the epitopes overlap more completely.

For this example, a modified antibody-antibody competition assay was used to determine the relative epitope binning profiles of the ASGR-1 specific, ligand blocking antibodies in a high throughput manner. Briefly, individual antibodies were tested for their ability to compete for binding with a panel of reference antibodies chosen based on their different binding characterstics (eg. species cross reactivity, HEPG2 binding, etc.) and primary sequences. The pattern of competition/binding of each test antibody with the reference antibody panel was then determined and compared to those produced from the other test antibodies. The degree of correlation between the individual test antibody competition/binding profiles was then compared. Antibodies that showed similar competition/binding profiles were binned (grouped) together (eg. Binning Profile A, B, etc.).

Biotinylated recombinant soluble human ASGR-1 protein was coupled to streptavidin coated, uniquely barcoded LumAvidin Beads® (LumAvidin Microspheres, Cat#:L101- LXXX-01; Luminex Corp., Austin,Texas, U.S.A.) for 45 minutes in the dark at room temperature and washed twice. The reference antibody hybridoma supernatant samples were incubated with the antigen-coated beads for 1 hour in the dark at room temperature and washed three times. Beads were resuspended in FACS buffer containing Stabilguard®. The antigen-coated, reference antibody-bound beads were pooled and then divided into individual sample wells containing a normalized (2.5ug/ml) test antibody (hybridoma supernatant) sample (or negative control), incubated for 1 hour in the dark at room temperature and washed twice. The samples were then incubated with Alexa Fluor® 488 IgG fragment-specific detection antibody for 15 minutes in the dark at room temperature, washed once and resuspended in FACS buffer. FACS buffer supplemented with 1mM Calcium Chloride was used in all steps. Samples were analyzed using an Intellicyt iQue™ Screener Platform.

To determine the antibody competition/binding profiles of the individual test antibodies, the reference-only antibody binding signal was subtracted from the reference plus test antibody signal for each competition/binding reaction (ie. across the entire reference antibody set). An individual antibody binding profile was defined as the collection of net binding values for each competition/binding reaction. The degree of similarity between individual profiles was then assessed by calculating the coefficient of determination between each of the test antibody profiles. Test antibodies showing high degrees of similarity ($R^2 \geq 0.8$) to each other were then grouped into common binning profiles. Separate binning profiles were only defined if there were two or more samples with a high degree of correlation. If individual unique antibody binning profiles were observed (ie. they displayed a low degree of similarity to other test antibody binding profiles), the bin was classified as unknown. Using this method, the ASGR-1-binding, receptor-ligand blocking antibodies were sub- divided into 14 unique binning profiles (A, B, C, D, E, L, M, N, O, P, Q, R, T and unknown) (Table 7.4).

Antibodies that displayed a unique binning profile (as defined above) but shared a relatively high degree of similarity to another profile ($R^2$ = 0.6-0.8) were categorized as a sub-bin (ie. A.1, A.2, etc.) of that profile.

Table 7.4 Relative Epitope Binning/Profiling of ASGR-1 Specific Receptor-Ligand Blocking mAbs

| mAb | Epitope BIN | mAb | Epitope BIN | mAb | Epitope BIN | mAb | Epitope BIN |
|---|---|---|---|---|---|---|---|
| 10G6 | A | 52H1 | A | 9C11 | A.3 | 60G2 | E |
| 11E2 | A | 53F2 | A | 12B12 | B | 65D5 | E |
| 11F5 | A | 53F7 | A | 147D10 | B | 66H11 | E |
| 12E9 | A | 55B1 | A | 149D11 | B | 71A6 | E |
| 12F11 | A | 56E5 | A | 149F8 | B | 73G1 | E |
| 12F12 | A | 57A7 | A | 151B9 | B | 49C5 | E.1 |
| 13F6 | A | 58G11 | A | 175F4 | B | 49D10 | E.1 |
| 148E10 | A | 59F2 | A | 22G5 | B | 51E3 | E.1 |
| 154F4 | A | 5E5 | A | 48B12 | B | 51F4 | E.1 |
| 159H8 | A | 60D2 | A | 52H2 | B | 53E8 | E.1 |
| 160B12 | A | 60E8 | A | 6G7 | B | 54E9 | E.1 |

| | | | | | | | |
|---|---|---|---|---|---|---|---|
| 175D10 | A | 63A10 | A | 7G2 | B | 56E3 | E.1 |
| 177D2 | A | 63G7 | A | 64G12 | B.1 | 56G1 | E.1 |
| 25A4 | A | 64B12 | A | 72F5 | B.1 | 190C11 | L |
| 25D12 | A | 65F10 | A | 147E9 | C | 190E6 | L |
| 26C4 | A | 68G6 | A | 184E7 | C | 190F12 | L |
| 27E7 | A | 6A6 | A | 194A4 | C | 190F8 | L |
| 28H2 | A | 6D4 | A | 208A2 | C | 190G11 | L |
| 29E2 | A | 6D9 | A | 210G10 | C | 190H9 | L |
| 29E6 | A | 6G6 | A | 4B1 | C | 191A10 | L |
| 29H8 | A | 70D1 | A | 60E12 | C | 191G1 | L |
| 31D12 | A | 7A10 | A | 61A1 | C | 191G10 | L |
| 32D6 | A | 7C3 | A | 62H10 | C | 191G12 | L |
| 3G7 | A | 7E11 | A | 63H8 | C | 192C10 | L |
| 45B4 | A | 7F4 | A | 72G9 | C | 192C8 | L |
| 49F10 | A | 7F8 | A | 8D8 | D.1 | 192E4 | L |
| 4A2 | A | 7G4 | A | 12D2 | E | 192G6 | L |
| 4B3 | A | 8D12 | A | 148H10 | E | 192G8 | L |
| 4H6 | A | 9F12 | A | 173C11 | E | 192H10 | L |
| 50D4 | A | 9G9 | A | 179C2 | E | 193C7 | L |
| 50G9 | A | 65E9 | A.1 | 47C1 | E | 194B7 | L |
| 51E9 | A | 72B4 | A.1 | 49C1 | E | 194C1 | L |
| 52G11 | A | 7H7 | A.2 | 60C12 | E | 196C7 | L |
| 197B6 | L | 197F2 | L | 198G3 | L | 219H1 | L |
| 197E11 | L | 197G3 | L | 213B3 | L | 74C8 | L |
| 74G6 | L | 74H7 | M.1 | 218G4 | O | 172B12 | Q |
| 75G3 | M | 85F7 | M.1 | 146A8 | P | 172C3 | Q |
| 89A11 | M | 198B9 | N | 146B6 | P | 193E7 | Q |
| 74B2 | M.1 | 199A7 | N | 149A1 | P | 199E3 | Q |
| 226F9 | Q | 227F2 | Q | 176H4 | R | | |
| 227C1 | Q | 65C12 | Q | 194C10 | T | | |

### E. Epitope Mapping - Arginine/Glutamic Acid Mutational Profiling

This Example characterizes ASGR-1 antibodies based on the effect of mutagenesis of ASGR-1 on their ability to bind the target. Previous data indicated that the ASGR-1 CBD is primarily responsible for antibody binding for the panel of antibodies. As such, only the ASGR-1 CBD was considered structurally in the context of the full length ASGR-1 in the design of mutation sites.

Arginine/Glutamic acid mutational mapping was used to characterize epitopes bound by human ASGR-1-specific, ligand blocking antibodies. Briefly, 144 individual point mutations were made across the CBD domain of human ASGR-1 protein (SEQ ID NO:5) starting at position 148. Ninety-one constructs, representing surface residues (modelled using the ASGR-1 crystal structure in the PyMOL Molecular Graphics System (Version 1.8; Schrödinger, LLC.)) and therefore potentially accessible for antibody binding, were selected for these assays. Mutant hASGR- 1 variants were constructed such that non-arginine residues were changed to arginine and where wild type arginine residues were mutated to glutamic acid. Each mutant hASGR-1 sequence was then cloned into a mammalian expression vector and used to transiently transfect CHOs cells. The ability of human ASGR-1-specific, ligand competing antibodies to bind to the mutant hASGR-1 proteins was assessed by FACS as described above.

Antibodies were tested for binding to the individual mutant and wild type ASGR-1 constructs using normalized antibody concentrations (5ug/ml). CHO-S cells transiently expressing the appropriate mutated or non-mutated antigen of interest were mixed with antibody sample or controls, incubated for 1 hour at 4°C, and then washed twice. Cells with bound antibody were then incubated with Alexa Fluor® 647 IgG Fc fragment-specific detection antibody and 7-AAD viability stain for 15 minutes at 4°C, washed once and resuspended in FACS buffer. Samples were analyzed using a BD Accuri™ Flow Cytometer and an Intellicyt HyperCyt Autosampler. As a negative control, supernatants and controls were also screened against CHO-S cells transfected with empty parental vector (referred to as mock). In order to exclude mutants that were poorly expressed or produced mis- folded antigen, only constructs that yeilded a binding data average of at least 25% or greater compared to the average binding observed on wildtype hASGR-1 was used for further analysis. Because mutant hASGR-1 expression levels varied relative to each other, sample binding data for each construct was normalized for expression by dividing the binding data from an antibody not affected by the mutations (e.g., 65C12) by the binding values of each test antibody on a given mutant construct. Also, because the antibody binding affinities varied amongst the samples, the expression corrected data (above) was further normalized by comparing test antibody binding on each mutant construct to wild type hASGR- 1. Identification of specific mutations that affected test antibody binding was performed by an interquartile range (IQR) analysis to determine statistical outliers. A mutation was identified as a "hit" if the calculated values were ≥ 3x the IQR (above the 3$^{rd}$ quartile/upper fence) for a given mutant construct. Although IQR analysis was used here to determine signifance and identify hits, one skilled in the art will recognize that a number of methods could be employed in order to normalize the data (eg. using epitope-tagged constructs or other ASGR-1-binding antibodies directed against non-CBD epitopes). Any statistically significant reduction in antibody binding signal to a mutant construct (compared to that determined for binding to wild type ASGR-1) determined by these methods could be used for hit identification.

For illustrative purposes, Table 7.5 shows the IQR analysis with a single mutant construct (i.e., H203).

Table 7.5. IQR analysis (representative data for construct H203)

| | FACS Binding Geomean | | Expression Normalization to mAb 65C12 | | Antibody Binding Normalization to wt ASGR1 Binding | | > Q3 + 3xIQR Gating | |
|---|---|---|---|---|---|---|---|---|
| mAb | wt ASGR1 | H203 | wt ASGR1 | H203 | wt ASGR1 | H203 | wt ASGR1 | H203 |
| 4A2 | 41104 | 18946 | 1.3644 | 1.3597 | 1.0000 | 0.9966 | 1.0000 | 0.9966 |
| 7E11 | 45453 | 14714 | 1.2338 | 1.7509 | 1.0000 | 1.4191 | 1.0000 | 1.4191 |
| 56E5 | 42617 | 20345 | 1.3159 | 1.2662 | 1.0000 | 0.9622 | 1.0000 | 0.9622 |
| 7G4 | 48526 | 18542 | 1.1557 | 1.3893 | 1.0000 | 1.2022 | 1.0000 | 1.2022 |
| 53F7 | 43474 | 18081 | 1.2900 | 1.4248 | 1.0000 | 1.1045 | 1.0000 | 1.1045 |
| 10G6 | 43059 | 18213 | 1.3024 | 1.4145 | 1.0000 | 1.0860 | 1.0000 | 1.0860 |
| 26C4 | 45991 | 13484 | 1.2194 | 1.9105 | 1.0000 | 1.5668 | 1.0000 | 1.5668 |
| 6G6 | 47628 | 20505 | 1.1775 | 1.2564 | 1.0000 | 1.0670 | 1.0000 | 1.0670 |
| 29H8 | 40927 | 13217 | 1.3702 | 1.9491 | 1.0000 | 1.4225 | 1.0000 | 1.4225 |
| 25A4 | 55579 | 20036 | 1.0090 | 1.2858 | 1.0000 | 1.2743 | 1.0000 | 1.2743 |
| 32D6 | 36128 | 13465 | 1.5522 | 1.9132 | 1.0000 | 1.2325 | 1.0000 | 1.2325 |
| 198D2 | 16882 | 7138 | 3.3219 | 3.6090 | 1.0000 | 1.0864 | 1.0000 | 1.0864 |
| 4B3 | 35561 | 1696 | 1.5770 | 15.1900 | 1.0000 | 9.6323 | 1.0000 | 9.6323 |
| 50G9 | 37326 | 1506 | 1.5024 | 17.1095 | 1.0000 | 11.3879 | 1.0000 | 11.3879 |
| 60D2 | 29631 | 1368 | 1.8926 | 18.8256 | 1.0000 | 9.9467 | 1.0000 | 9.9467 |
| 59F2 | 27915 | 1346 | 2.0089 | 19.1372 | 1.0000 | 9.5260 | 1.0000 | 9.5260 |
| 60E8 | 38653 | 1518 | 1.4509 | 16.9692 | 1.0000 | 11.6960 | 1.0000 | 11.6960 |
| 65E9 | 29613 | 1471 | 1.8938 | 17.5097 | 1.0000 | 9.2460 | 1.0000 | 9.2460 |
| 5E5 | 40651 | 12616 | 1.3796 | 2.0420 | 1.0000 | 1.4802 | 1.0000 | 1.4802 |
| 29E2 | 25781 | 15058 | 2.1752 | 1.7108 | 1.0000 | 0.7865 | 1.0000 | 0.7865 |
| 45B4 | 30350 | 14012 | 1.8478 | 1.8385 | 1.0000 | 0.9950 | 1.0000 | 0.9950 |
| 6G7 | 38643 | 15089 | 1.4512 | 1.7073 | 1.0000 | 1.1764 | 1.0000 | 1.1764 |
| 72F5 | 27993 | 10499 | 2.0034 | 2.4537 | 1.0000 | 1.2248 | 1.0000 | 1.2248 |
| 22G5 | 45048 | 15060 | 1.2449 | 1.7105 | 1.0000 | 1.3740 | 1.0000 | 1.3740 |
| 48B12 | 52493 | 20467 | 1.0683 | 1.2587 | 1.0000 | 1.1782 | 1.0000 | 1.1782 |
| 151B9 | 23527 | 9738 | 2.3837 | 2.6454 | 1.0000 | 1.1098 | 1.0000 | 1.1098 |
| 52H2 | 47957 | 18609 | 1.1694 | 1.3843 | 1.0000 | 1.1838 | 1.0000 | 1.1838 |
| 149D11 | 23601 | 8866 | 2.3761 | 2.9055 | 1.0000 | 1.2228 | 1.0000 | 1.2228 |
| 175F4 | 33619 | 14804 | 1.6681 | 1.7401 | 1.0000 | 1.0432 | 1.0000 | 1.0432 |
| 147E9 | 40166 | 21513 | 1.3962 | 1.1975 | 1.0000 | 0.8577 | 1.0000 | 0.8577 |
| 61A1 | 39965 | 20142 | 1.4032 | 1.2790 | 1.0000 | 0.9115 | 1.0000 | 0.9115 |

| | | | | | | | | |
|---|---|---|---|---|---|---|---|---|
| 184E7 | 42704 | 18354 | 1.3132 | 1.4036 | 1.0000 | 1.0688 | 1.0000 | 1.0688 |
| 72G9 | 36507 | 18778 | 1.5361 | 1.3719 | 1.0000 | 0.8931 | 1.0000 | 0.8931 |
| 194A4 | 16291 | 12149 | 3.4424 | 2.1204 | 1.0000 | 0.6160 | 1.0000 | 0.6160 |
| 60C12 | 31286 | 19812 | 1.7925 | 1.3003 | 1.0000 | 0.7254 | 1.0000 | 0.7254 |
| 173C11 | 28526 | 13861 | 1.9659 | 1.8586 | 1.0000 | 0.9454 | 1.0000 | 0.9454 |
| 56E3 | 33876 | 20425 | 1.6555 | 1.2613 | 1.0000 | 0.7619 | 1.0000 | 0.7619 |
| 54E9 | 38589 | 15344 | 1.4533 | 1.6789 | 1.0000 | 1.1552 | 1.0000 | 1.1552 |
| 65D5 | 41007 | 20291 | 1.3676 | 1.2696 | 1.0000 | 0.9283 | 1.0000 | 0.9283 |
| 190F8 | 36503 | 15073 | 1.5363 | 1.7091 | 1.0000 | 1.1125 | 1.0000 | 1.1125 |
| 198G3 | 21467 | 13143 | 2.6124 | 1.9600 | 1.0000 | 0.7503 | 1.0000 | 0.7503 |
| 191G10 | 33829 | 17045 | 1.6578 | 1.5114 | 1.0000 | 0.9117 | 1.0000 | 0.9117 |
| 202A3 | 24848 | 12497 | 2.2570 | 2.0614 | 1.0000 | 0.9134 | 1.0000 | 0.9134 |
| 194C1 | 20860 | 11044 | 2.6884 | 2.3325 | 1.0000 | 0.8676 | 1.0000 | 0.8676 |
| 176H4 | 33506 | 10237 | 1.6737 | 2.5166 | 1.0000 | 1.5036 | 1.0000 | 1.5036 |
| 197G3 | 13308 | 3503 | 4.2141 | 7.3547 | 1.0000 | 1.7453 | 1.0000 | 1.7453 |
| 191G1 | 25298 | 10876 | 2.2168 | 2.3687 | 1.0000 | 1.0685 | 1.0000 | 1.0685 |
| 213B3 | 15070 | 12846 | 3.7212 | 2.0054 | 1.0000 | 0.5389 | 1.0000 | 0.5389 |
| 218G4 | 12212 | 7933 | 4.5923 | 3.2472 | 1.0000 | 0.7071 | 1.0000 | 0.7071 |
| 75G3 | 37223 | 14472 | 1.5066 | 1.7801 | 1.0000 | 1.1815 | 1.0000 | 1.1815 |
| 194C10 | 28138 | 13217 | 1.9930 | 1.9491 | 1.0000 | 0.9780 | 1.0000 | 0.9780 |
| 85F7 | 32968 | 16509 | 1.7010 | 1.5605 | 1.0000 | 0.9174 | 1.0000 | 0.9174 |
| 199A7 | 17005 | 9455 | 3.2978 | 2.7247 | 1.0000 | 0.8262 | 1.0000 | 0.8262 |
| 146B6 | 24138 | 14412 | 2.3233 | 1.7875 | 1.0000 | 0.7694 | 1.0000 | 0.7694 |
| 193E7 | 35508 | 13783 | 1.5794 | 1.8691 | 1.0000 | 1.1835 | 1.0000 | 1.1835 |
| 65C12 | 56080 | 25761 | 1.0000 | 1.0000 | 1.0000 | 1.0000 | 1.0000 | 1.0000 |

The grayed values for antibodies 4B3, 50G9, 60D2, 59F2, 60E8, and 65E9 represent the statistically significant hits (i.e., ≥ 3x the IQR) whose binding was affected by mutations H203.

A summary of the hASGR-1 residues important for binding of the representative antibodies is shown in Table 7.6. In addition, this analysis revealed that the mutation of some ASGR- 1 residues had more dramatic effects on a given antibody binding than others. This likely reflects the relative contribution or importance that these residues have in mediating interactions with specific test antibodies. The degree by which each mutation impacted the ability of a test antibody to bind was determined by calculating the magnitude of an individual binding data point above the upper gate determined by each IQR analysis. The relative impact of each mutation on the binding of a given test antibody was then ranked using this method and displayed as a heatmap in Table 7.6. Dark grey coloring indicates the data point deviated dramatically from the upper gate (ie. a large effect on antibody binding), while light grey/white indicates the data point was very close to the cut offs (ie. 3x the IQR)(Table 7.6). When aligned with the relative epitope profiling bin assignments (Example 7D above), this analysis reveals a set of core ASGR-1 amino acid positions that, when mutated, disrupt test antibody binding. As such, these positions are likely part of the ASGR-1 epitopes bound by the selected antibodies. These amino acid residues either directly contact or are involved in the interaction with the antibody, or are in close enough proximity that, when mutated, interfere with antibody binding. Amino acid positions identified as statistically significant hits, but barely made the cut offs, and map to surface ASGR-1 locations distinct from the main epitope bins (Figure 47) may represent residues that, when mutated, disrupt the conformation of ASGR-1 such that an antibody that binds to a distinct epitope is affected (ie. an indirect effect). mAb 197G3 is an example of an antibody displaying a range of binding sensitivities in this assay, yet the most important residues (R274 and R271) can be identified by rank ordering them as described.

Table 7.6. ASGR-1 residues identified as hits via Arg/Glu scanning mutagenesis

++++ -

| mAb | Relative Epitope Profiling Bin | ASGR1 CBD Mutations That Reduced Antibody Binding Signal (3xIQR Cutoff) | | | | | | | | | | | | | | | | | | | |
|---|---|---|---|---|---|---|---|---|---|---|---|---|---|---|---|---|---|---|---|---|---|
| 4A2 | A | W195 | | | | | | | | | | | | | | | | | | | |
| 7E11 | A | W195 | | | | | | | | | | | | | | | | | | | |
| 56E5 | A | W195 | | | | | | | | | | | | | | | | | | | |
| 7G4 | A | W195 | R263 | | | | | | | | | | | | | | | | | | |
| 53F7 | A | W195 | K199 | E196 | | | | | | | | | | | | | | | | | |
| 10G6 | A | W195 | E196 | | | | | | | | | | | | | | | | | | |
| 26C4 | A | W195 | P207 | | | | | | | | | | | | | | | | | | |
| 6G6 | A | | | | | | | | | | | | | | | | | | | | |
| 29H8 | A | W195 | P207 | | | | | | | | | | | | | | | | | | |
| 25A4 | A | | | | | | | | | | | | | | | | | | | | |
| 32D6 | A | W195 | K199 | | | | | | | | | | | | | | | | | | |
| 198D2 | unknown | W195 | E196 | H204 | | | | | | | | | | | | | | | | | |
| 4B3 | A | H203 | H204 | | | | | | | | | | | | | | | | | | |
| 50G9 | A | H203 | H204 | | | | | | | | | | | | | | | | | | |
| 60D2 | A | H203 | H204 | | | | | | | | | | | | | | | | | | |
| 59F2 | A | H203 | H204 | | | | | | | | | | | | | | | | | | |
| 60E8 | A | H203 | H204 | P220 | G251 | | | | | | | | | | | | | | | | |
| 65E9 | A.1 | H203 | H204 | | | | | | | | | | | | | | | | | | |
| 5E5 | A | K199 | W195 | R263 | | | | | | | | | | | | | | | | | |
| 29E2 | A | K199 | R263 | | | | | | | | | | | | | | | | | | |
| 45B4 | A | K199 | R263 | | | | | | | | | | | | | | | | | | |
| 6G7 | B | L184 | | | | | | | | | | | | | | | | | | | |
| 72F5 | B.1 | L184 | | | | | | | | | | | | | | | | | | | |
| 22G5 | B | L184 | N265 | P220 | H215 | G251 | G248 | R183 | G246 | | | | | | | | | | | | |
| 48B12 | B | L184 | | | | | | | | | | | | | | | | | | | |
| 151B9 | B | L184 | | | | | | | | | | | | | | | | | | | |
| 52H2 | B | L184 | P238 | H247 | G251 | P220 | | | | | | | | | | | | | | | |
| 149D11 | B | R170 | L184 | S171 | | | | | | | | | | | | | | | | | |
| 175F4 | B | R183 | | | | | | | | | | | | | | | | | | | |
| 147E9 | C | D243 | P241 | D242 | G251 | E253 | Y245 | | | | | | | | | | | | | | |
| 61A1 | C | D243 | P241 | Y245 | D242 | E253 | G251 | | | | | | | | | | | | | | |
| 184E7 | C | D243 | E253 | P241 | | | | | | | | | | | | | | | | | |
| 72G9 | C | D243 | Y245 | P241 | D242 | E253 | G251 | | | | | | | | | | | | | | |
| 194A4 | C | D260 | | | | | | | | | | | | | | | | | | | |
| 60C12 | E | R237 | D260 | R263 | | | | | | | | | | | | | | | | | |
| 173C11 | E | R237 | D260 | T259 | R263 | | | | | | | | | | | | | | | | |
| 56E3 | E.1 | R237 | T259 | R263 | N265 | D260 | P241 | R170 | | | | | | | | | | | | | |
| 54E9 | E.1 | R237 | T259 | N265 | R263 | D260 | P241 | E239 | | | | | | | | | | | | | |
| 65D5 | E | D260 | R237 | R263 | T259 | | | | | | | | | | | | | | | | |
| 190F8 | L | R271 | R274 | G172 | P272 | V208 | | | | | | | | | | | | | | | |
| 198G3 | L | R271 | R274 | G172 | | | | | | | | | | | | | | | | | |
| 191G10 | L | R271 | G172 | R274 | | | | | | | | | | | | | | | | | |
| 202A3 | unknown | R271 | G172 | N209 | | | | | | | | | | | | | | | | | |
| 194C1 | L | R274 | R271 | P272 | G172 | V208 | R170 | | | | | | | | | | | | | | |
| 176H4 | R | R271 | P272 | N265 | G172 | P241 | L249 | H247 | D242 | | | | | | | | | | | | |
| 197G3 | L | R274 | R271 | R170 | G172 | D243 | G248 | D216 | P272 | S171 | Q270 | L249 | E196 | D260 | H215 | D225 | D228 | G251 | E280 | P207 | H204 |
| 191G1 | L | R274 | P238 | R271 | P272 | G172 | V208 | | | | | | | | | | | | | | |
| 213B3 | L | P238 | R271 | R274 | P272 | G172 | | | | | | | | | | | | | | | |
| 218G4 | O | R274 | P238 | G172 | R271 | | | | | | | | | | | | | | | | |
| 75G3 | M | R274 | R170 | G172 | V208 | | | | | | | | | | | | | | | | |
| 194C10 | T | R274 | R170 | G172 | V208 | | | | | | | | | | | | | | | | |
| 85F7 | M.1 | R274 | R170 | V208 | G172 | | | | | | | | | | | | | | | | |
| 199A7 | N | H215 | R170 | R183 | Q270 | | | | | | | | | | | | | | | | |
| 146B6 | P | T259 | N265 | P241 | | | | | | | | | | | | | | | | | |
| 193E7 | Q | R263 | P207 | | | | | | | | | | | | | | | | | | |
| 65C12 | Q | | | | | | | | | | | | | | | | | | | | |

In order to compare the mutational hit patterns of the individual test antibodies with each other, the coefficient of determination between the test antibodies was determined. The expression and antibody binding normalized data set was used to generate binding profiles for each test antibody across the mutant panel. The resulting profiles for each individual test antibody were then compared for their degree of similarity to all of the other test antibodies. The coefficient of determination ($R^2$) for each combination was determined and converted into a heat map in order to visualize the resulting patterns (Figure 46). For simplicity, a representative antibody from each unique mutational profile (Reference Antibody) is shown in Figure 46. This analysis revealed 7 predominant hit patterns or mutational clusters.. Test antibodies affected by the 7 predominant mutational clusters correspond to those from competition/binding binning profiles A, B, C, E and L (3 distinct hit/mutational clusters of bin A antibodies and 1 distinct hit/mutational cluster of bin B, bin C, bin E and bin L antibodies). The remaining antibodies, categorized as displaying separate binning profiles (compared to bins A, B, C, E and L), are affected by distinct mutations in ASGR-1, but also include residues that partially overlap with test antibodies belonging to the predominant bins.

This data indicates the selected antibodies bind to epitopes that partially overlap with the 7 predominant epitope regions. The residues important for the binding of antibodies belonging to the 7 predominant epitope regions were then mapped onto a computer representation of the surface of the ASGR-1 structure using the PyMOL Molecular Graphics System (Version 1.8; Schrödinger, LLC.) (Figure 47). A residue on the surface of ASGR-1 was considered part of the same epitope region if at least one antibody from a distinct binning profile (ie. A, B, C, E and L) was identified as being sensitive to mutation. For example, the predominant epitope region for antibodies belonging to binning profile C includes hASGR-1 residues P241, D242, D243, Y245, G251 and E253 (SEQ ID NO:5). The binding of antibody 147E9 is affected by mutation of all of these residues, while antibody 184E7 is only disrupted by mutation of P241, D243 and E253. Thus, the predominant epitope region of ASGR-1 bound by antibodies belonging to binning profile C is defined as including one or more of (but not limited to) P241, D242, D243, Y245, G251 and E253 (SEQ ID NO:5). Also, note that the antibody 194A4 was classified as belonging to Bin C as determined in Example 7D, however, the results of this arginine/glutamic acid mutational profiling (as well as the results from crystal structure analysis of the ASGR-1 CBD/194A4 complex described in Example 10H) suggests that the relative epitope profiling may have been inaccurate.

Antibodies belonging to binning profile A were further sub-divided into 3 distinct mutational clusters. These clusters mapped to ASGR-1 surface positions that overlap with, or are in extremely close physical proximity to, each other consistent with a common binning profile. Antibodies that displayed binning profiles distinct from the 5 major bins (i.e., A, B, C, E and L) also showed distinct patterns of mutations that affected their binding (Figure 46). Some binning profiles (R, O, M, M.1 and T) share significant overlap with antibodies from binning profile L, and can be considered sub-bins of this profile. Taken together, this data indicates that antibodies capable of blocking ASGR-1-ligand interactions bind to 5 major epitope regions. In addition, blocking antibodies were identified that bind to partially overlapping epitopes of these major regions.

**Example 8: ASGR Internalization Assay**

To determine whether the antibodies bind and also prevent internalization of ASGR-1 into cells expressing ASGR-1, an in vitro internalization assay is performed of various antibody samples.

<u>Human ASGR-1 Internalization Cellular Imaging Assay Protocol</u>

Reagents:

U2OS (Human Osteosarcoma) cell line
McCoy's 5A Medium: Gibco, #16600-082
MEM NEAA (100X): Gibco, #11140-050
Penicillin-Streptomycin (10,000U/ml, 100X) Gibco, #15140-122
L-Glutamine (100X): Gibco, #25030-081
Fetal Bovine Serum: Gibco, #16000-044
DPBS (without Ca and Mg): Gibco, #14190-136
DPBS (with Ca and Mg): Gibco, #14040-133
Cell Dissociation Buffer: Gibco, #13151-014
1Liter Filter: Corning, #430517
Hepes Buffer (1M): Gibco, #15630-080
BacMam Virus - huASGR-1: GS: SNAP26f
β-GalNAc-PAA-Biotin: GlycoTech, #01-011
SNAP-Surface Alexa Fluor 546: New England Biolabs, #S9132S
Streptavidin-Alexa Fluor 633: Life Technologies, #S21375
Hoechst 33342: Invitrogen, #H3570
Pitstop2: abeam Biochemical, #ab120687
Pitstop2 - negative control: abcam Biochemical, #ab120688
Paraformaldehyde (8% Aqueous Solution): Electron Microscopy Sciences, #157-8-100
Imaging plate - 96 well Optical Bottom: Thermo Scientific Nunc, #165305
Operetta High Content Imager: Perkin Elmer

U2OS complete growth medium:

McCoy's 5A with 10%FBS, 1XMEM NEAA, 1XL-Glutamine, and 1XPenicillin-Streptomycin Medium was filtered before use on cells

U2OS cell plating and culturing:

U2OS cells were grown to 75-85% confluence in T175 before plating into a 96 well plate.

1. The U2OS culture medium was aspirated off the cells in the T175 flask

2. Cells were washed with 10mls of DPBS and aspirated off

3. 3mls of Cell Dissociation Buffer was added to the cells and incubated for 5 minutes inside a cell incubator (37°C, 5%CO2) to detach the cells from the T175 flask.

4. The detached cells were diluted with 7mls of the growth medium

5. 1ml of cells were used to count the number of cells available to plate

6. The cells were diluted in growth medium to give a final concentration of 28,000 cells/well and BacMam virus (huASGR-1: GS: SNAP26f) was also added to the cells at this time with the desired concentration (MOI).

7. The cells were mixed together with the BacMam virus for 1-2 minutes and then plated on the 96 well imaging plate at a volume of 100ul/well.

8. The plate was placed inside an incubator (37°C, 5%CO2) for 16-20 hours before treatment.

Treatment of Cells (16-20 hours incubation)

1. The next day, the medium on the 96 well plate was dumped out and washed once with DPBS.

2. McCoy's 5A Medium plus 10mM of Hepes buffer (assay buffer) was added to the cells (100ul) for 1 hour inside the incubator.

3. After the 1 hour incubation, the medium was dumped out and washed once with DPBS containing Ca and Mg.

4. Pitstop2 and Pitstop2 negative control were prepared in assay buffer at 20uM.

5. Volume of 100ul per well of the inhibitors were added to the U2OS cells for 15 minutes inside the incubator.

6. GalNAc-biotin (100nM) and streptavidin-Alexa633 (100nM) were pre-mixed in assay buffer and incubated for 10 minutes at room temperature.

7. SNAP-Surface Alexa Fluor 546 (2.5uM) was prepared in assay buffer.

8. After the 15 minutes incubation, both GalNAc-biotin-streptavidin-Alexa633 and SNAP- Surface Alexa Fluor 546 were directly added (10ul) to the medium containing Pitstop2 inhibitors for 30 minutes inside the incubator.

9. After the 30 minutes incubation, medium was dumped out and the cells were washed once with DPBS.

10. The cells were fixed by adding 50ul of 4% Paraformaldehyde (8% paraformaldehyde was diluted with DPBS) containing Hoechst dye (1:5000 dilution) to the cells for 10 minutes at room temperature.

11. After 10 minutes incubation, the cells were washed twice with DPBS and 100ul of DPBS was added to each well.

12. The plate was imaged on the Operetta instrument with three channels measuring the different fluorescence dyes.

1) Hoechst was measured using filters in the range of excitation: 360-400nm and emission: 410- 480nm

2) GalNAc-biotin-streptavidin-Alexa633 was measured using filters in the range of excitation: 600-630nm and emission: 640-680nm

3) SNAP-Surface Alexa Fluor 546 was measured using filters in the range of excitation: 520- 550nm and emission: 560-630

13. Harmony 3.5 software (Perkin Elmer) was used to identify and quantify internalized spots for fluorescence dyes added in the assay.

This internalization assay can be performed to assay the antigen binding proteins disclsoed herein to determine how much they reduce or inhibit internalization of ASGR, ASGR-1, and/or ASGR-2.

**Example 9: Additional ligand blocking assays**

Preparation of desialylated protein ligands (asialofetuin and orosomucoid)

A. Asialofetuin

Bovine fetuin (AHSG) was obtained commercially (Sigma) and purified using a CaptoQ Impres (GE Healthcare Life Sciences) matrix. Briefly, the material was loaded in 25mM TRIS pH 7.9 at up to 17mg/ml resin, resolved in 20mM BisTRIS (pH6.5) with a gradient of sodium chloride. The main peak was gradient pooled (~0.15M NaCl final) and resolved on a SuperDex200 SEC (GE Healthcare Life Sciences) in Hepes-buffered saline (pH 7.9). The purified AHSG was then concentrated and incubated with Innolink Biotin 354S (EMD Millipore) according to the manufacturer's instructions. The biotinylated protein was then desalted by gel filtration and concentrated once again.

The purified, biotinylated protein was subsequently desialylated by incubation with C. perfringens neuraminidase (Sigma; 1 unit/10mg protein for 12 hours at 37oC in 50mM sodium phosphate, 9mM HEPES, 0.12M NaCl, pH6). The resulting material was harvested and digested for an additional 3 hours with A. ureafaciens neuraminidase (QAbio; 0.5 units/10mg protein at 37oC). The digested sample was diluted 3 fold with 20mM HEPES containing 0.15M NaCl (pH 7.5) (HBS) to neutral pH and applied to a monomeric Avidin agarose (Pierce) HR16/10 column, run at 60cm/hour. The loaded column was held for 15 minutes then washed with four column volumes of HBS. The biotinylated, desialylated protein was finally eluted with three column volumes of HBS containing 2mM Biotin plus an additional two column volumes of 0.1M Glycine-HCl (pH 2.8), which was immediately neutralized during collection with 50mM TRIS Base). Protein-containing fractions from both types of elutions were identified, pooled, concentrated, dialyzed extensively against 10mM HEPES, 0.14M NaCl (pH 7.5), re-concentrated and finally filtered sterilized. The purified lots were then analyzed by SDS-PAGE and mass spectrometry prior to use in the described assays.

B. Orosomucoid

Bovine orosomucoid (AGP) was obtained commercially (Sigma) and purified over SuperDex200 resin equilibrated in HBS (pH7.9) by size exclusion chromatography. The front of the main AGP peak was combined from 3 individual runs to generate hyperglycosylated AGP, with the remainder of the main peaks (from the 3 combined runs) to generate hypoglycosylated AGP. For biotinylation, the purified AGP was concentrated to 5mg/ml and incubated with Innolink Biotin 354S as described. The biotinylated protein was then desalted by gel filtration and concentrated.

After biotinylation, the protein was desialylated by incubating it for 18 hours at 37oC with one unit of C. perfringens neuraminidase (Sigma) per 10mg protein in 50mM sodium phosphate, 9mM HEPES, 0.12M NaCl (pH6). The resulting material was harvested and digested for an additional 6 hours at 37oC with 0.5 units A. ureafaciens neuraminidase (QAbio) per 10mg protein. The sample was diluted 3 fold with HBS to achieve a neutral pH and applied to a monomeric Avidin agarose (Pierce) HR16/10 column, run at 60cm/hour. The loaded column was held for 15 minutes and then washed with four column volumes of HBS. The biotinylated, desialylated protein was subsequently eluted with three column volumes of HBS containing 2mM Biotin, plus two column volumes 0.1M Glycine-HCl (pH 2.8), which was immediately neutralized during collection with 50mM TRIS Base. Protein-containing fractions from both types of elutions were identified, pooled, concentrated, dialyzed extensively against 10mM HEPES, 0.14M NaCl (pH 7.5), re-concentrated and finally filtered sterilized. The purified lots were then analyzed by SDS-PAGE and mass spectrometry prior to use in the described assays.

These ligands can be used in additional ligand binding assays to determine antigen binding protein inhibition of ligand binding to ASGR, ASGR-1 and/or ASGR-2.

## Example 10: Crystal Structure Analysis of Interaction between Ligands and ASGR-1 and Antibodies and ASGR-1

### A. Crystal Structures of ASGR-1 Carbohydrate Binding Domain with Ligand Bound

#### Introduction

The crystal structure of ligand free ASGR-1 CBD (carbohydrate binding domain) has been previously described (1). Protein expression of ASGR-1 CBD (SEQ ID NO:5), purification and crystallization was performed similar to the published method, however the structures described here differ from the published crystal structure. Analysis of these structures shows extra N- and C- terminal amino acids compared to the published structure, how various ligands interact with the ASGR-1 carbohydrate binding domain, and possible selectivity determinants between ASGR- 1/ASGR-2 for various saccharides.

#### Results

**Lactose binds in the carbohydrate binding pocket of ASGR-1**

Protein crystals of the ASGR-1/Lactose complex were grown and the crystal structure was determined at 2.05 Å. Although a method similar to that of the published structure was followed, clear electron density is present for the lactose disaccharide in the carbohydrate binding pocket. See Figures 18A and 18BIn this structure, the galactose ring of the lactose disaccharide sits on top of the calcium ion at the carbohydrate binding domain and forms the majority of the contacts with the ASGR-1 protein. Hydrogen bonds are formed between lactose and ASGR-1 amino acids Q240, D242, E253, and N265. Additionally, van der Waals interactions are formed with at least W244 (SEQ ID NO:5). See Figure 18C.

Analysis of the crystal structure identifies specific amino acids involved in the interaction between ASGR-1 and lactose. Interacting with at least these amino acids by an alternate molecule can completely or partially affect the interaction between ASGR-1 and lactose.

ASGR-1 / Lactose analysis (Distances below were calculated with PyMOL):

Amino acids with at least one non-hydrogen atom 4.5 Å or less to the bound lactose molecule were identified and include: Q240, D242, W244, E253, N265, D266, D267 (SEQ ID NO:5).

Amino acids with at least one non-hydrogen atom 5 Å or less to the bound lactose molecule were identified and include: Q240, D242, W244, E253, N265, D266, D267 (SEQ ID NO:5).

Amino acids with at least one non-hydrogen atom 5-8 Å from the bound lactose molecule were identified and include: N209, R237, P238, E239, P241, D243, Y245, G246, H247, G252, C255, H257, T259, D260, V268, R271, Y273 (SEQ ID NO:5).

**Galactose binds in the carbohydrate binding pocket of ASGR-1 similar to lactose**

Protein crystals of the ASGR-1/Galactose complex were grown and the crystal structure was determined at 2.4 Å. Although a method similar to that of the published structure was followed, clear electron density is present for the galactose saccharide in the carbohydrate binding domain. See Figure 19A and 19B.

In this structure, galactose sits on top of the calcium ion at the carbohydrate binding site and forms contacts with the ASGR-1 protein. Hydrogen bonds are formed between galactose and ASGR-1 amino acids Q240, D242, E253, and N265 (SEQ ID NO:5). Additionally, van der Waals interactions are formed with at least W244. See Figure 19C.

Analysis of the crystal structure identifies specific amino acids involved in the interaction between ASGR-1 and galactose. Interacting with at least these amino acids by an alternate molecule may completely or partially affect the interaction between ASGR-1 and galactose. Distances below were calculated with PyMOL.

ASGR-1 / Galactose analysis (Distances below were calculated with PyMOL):

Amino acids with at least one non-hydrogen atom 4.5 Å or less to the bound galactose molecule were identified and include: R237, Q240, D242, W244, E253, N265, D266, D267 (SEQ ID NO:5). Amino acids with at least one non-hydrogen atom 5 Å or less to the bound lactose molecule were identified and include: R237, Q240, D242, W244, E253, N265, D266, D267 (SEQ ID NO:5).

Amino acids with at least one non-hydrogen atom 5-8 Å from the bound lactose molecule were identified and include: N209, P238, E239, P241, D243, Y245, G246, H247, G252, C255, H257, T259, V268, R271, Y273 (SEQ ID NO:5).

When comparing the ASGR-1/Lactose and ASGR-1/Galactose structures, the galactose rings of each saccharide superimpose very well. One difference in the proteins in the two structures is the conformation of R237, an amino acid in close proximity to the carbohydrate binding site. In the superimposition shown in Figure 20, the ASGR-1/Lactose structure is shown in white and the ASGR-1/Galactose structure is shown in black.

**N-acetyl-D-galactosamine (GalNAc) binds in the carbohydrate binding pocket of ASGR-1 similar to galactose, buts forms additional interactions**

Protein crystals of the ASGR-1/GalNAc complex were grown and the crystal structure was determined at 2.2 Å. Although a method similar to that of the published structure was followed, clear electron density is present for the GalNAc saccharide in the carbohydrate binding pocket. See Figure 21A and Figure 21B.

In this structure, GalNAc sits on top of the calcium ion at the carbohydrate binding site and forms contacts with the ASGR-1 protein. Hydrogen bonds are formed between GalNAc and ASGR-1 amino acids Q240, D242, E253, and N265. Additionally, van der Waals interactions are formed with at least W244. In this structure, R237 is in a similar conformation as observed in the galactose complex. However, in this case hydrogen bonds are formed between R237 and the acetyl of GalNAc. These additional interactions with R237 help explain both the observed tighter binding of GalNAc (than galactose) to ASGR-1, and the tighter binding to GalNAc to ASGR-1 (than ASGR- 2, in which this amino acid is Ala rather than Arg). See Figure 21C

**ASGR-1 / GalNAc analysis (Distances were calculated with PyMOL);**

Analysis of the crystal structure identifies specific amino acids involved in the interaction between ASGR-1 and GalNAc. Interacting with at least one of these amino acids by an alternate molecule may completely or partially inhibit the interaction between ASGR-1 and GalNAc.

Amino acids with at least one atom 4.5 Å or less to the bound GalNAc molecule were identified and include: N209, R237, Q240, D242, W244, E253, H257, T259, N265, D266, D267, Y273 (SEQ ID NO:5). Amino acids with at least one non-hydrogen atom 5 Å or less to the bound lactose molecule were identified and include: N209, R237, Q240, D242, W244, E253, H257, T259, N265, D266, D267, Y273 (SEQ ID NO:5).

Amino acids with at least one non-hydrogen atom 5-8 Å from the bound lactose molecule were identified and include: P238, E239, P241, D243, Y245, G246, H247, G252, C255, F258, D260, R263, W264, V268, R271 (SEQ ID NO:5).

The coordinates for the ASGR-1 CBD/GalNAc crystal structure complex are presented in Table 10.1.

**Methods**

ASGR-1 expression and purification

For all chrystallography experiments in Example 12, Human ASGR-1 CBD protein (SEQ ID NO:5) was expressed in E.coli and refolded and purified.

ASGR-1 crystallization

Purified human ASGR-1 CBD (148-291) protein was concentrated to 8-12 mg/ml. ASGR-1/carbohydrate complex crystals grow in 0.1 M sodium cacodylate pH 6.8, 0.08 M ammonium sulfate, 21-23% PEG 8000 in the presence of 20 mM ligand (lactose, galactose or GalNAc).

Data collection and structure determination

Datasets for ASGR-1 CBD complexes were collected on a Rigaku FR-E X-ray source (ASGR-1/Lactose and ASGR-1/Galactose) or at Berkeley Advanced Light Source beamline 5.0.2 (ASGR-1/GalNAc). All datasets were processed with iMosflm(2) and scaled with AIMLESS(3) from the CCP4 program suite(4).

ASGR-1/Lactose crystals grow in the C2 space group with unit cell dimensions a=113.5, b=32.3, c=40.4 Å, β=92.3° with one complex molecule per asymmetric unit, and diffract to 2.05 Å resolution. The ASGR-1 structure was solved by molecular replacement with the program PHASER(5) using the published ASGR-1 structure(1) as the starting search model. The structure was improved with multiple rounds of model building with Coot(6) and refinement with PHENIX(7). The refined structure has R=18.9 and $R_{free}$=24.4.

ASGR-1/Galactose crystals grow in the C2 space group with unit cell dimensions a=113.1, b=32.7, c=40.7 Å, β=91.6° with one complex molecule per asymmetric unit, and diffract to 2.4 Å resolution. The ASGR-1/Lactose structure was used as the starting molecule for molecular replacement, and model building and refinement were performed as described for the ASGR-1/Lactose complex to R=15.8 and $R_{free}$=22.9.

ASGR-1/GalNAc crystals grow in the C2 space group with unit cell dimensions a=112.7, b=32.3, c=40.5 Å, β=91.7° with one complex molecule per asymmetric unit, and diffract to 2.2 Å resolution. The ASGR-1/Lactose structure was used as the starting molecule for molecular replacement, and model building and refinement were performed as described for the ASGR-1/Lactose complex to R=16.5 and $R_{free}$=23.0.

Structure analysis and distance calculations were performed with the program PyMOL(8).

**References**

1. Meier, M., Bider, M. D., Malashkevich, V. N., Spiess, M., and Burkhard, P. (2000) Crystal structure of the carbohydrate recognition domain of the H1 subunit of the asialoglycoprotein receptor. Journal of molecular biology 300, 857-865
2. Battye, T. G., Kontogiannis, L., Johnson, O., Powell, H. R., and Leslie, A. G. (2011) iMOSFLM: a new graphical interface for diffraction-image processing with MOSFLM. Acta crystallographica 67, 271-281
3. Evans, P. (2006) Scaling and assessment of data quality. Acta crystallographica 62, 72-82
4. CCP4. (1994) The CCP4 suite: programs for protein crystallography. Acta crystallographica 50, 760-763
5. McCoy, A. J., Grosse-Kunstleve, R. W., Adams, P. D., Winn, M. D., Storoni, L. C., and Read, R. J. (2007) Phaser crystallographic software. Journal of applied crystallography 40, 658-674
6. Emsley, P., Lohkamp, B., Scott, W. G., and Cowtan, K. (2010) Features and development of Coot. Acta crystallographica 66, 486-501
7. Adams, P. D., Afonine, P. V., Bunkoczi, G., Chen, V. B., Davis, I. W., Echols, N., Headd, J. J., Hung, L. W., Kapral, G. J., Grosse-Kunstleve, R. W., McCoy, A. J., Moriarty, N. W., Oeffner, R., Read, R. J., Richardson, D. C., Richardson, J. S., Terwilliger, T. C., and Zwart, P. H. (2010) PHENIX: a comprehensive Python-based system for macromolecular structure solution. Acta crystallographica 66, 213-221
8. DeLano, W. L. (2002) The PyMOL Molecular Graphics System. Palo Alto


**B. Crystal Structure of ASGR-1 Carbohydrate Binding Domain (CBD) with 5E5**

The present example presents the crystal structure of the ASGR-1 CBD bound to the Fab fragment of 5E5, determined to 1.95 Å resolution (the conditions for which are described in the below). This structure, depicted in FIG. 22A&B, shows that when 5E5 binds to/interacts with ASGR-1, a conformational rearrangement of the carbohydrate binding loop occurs, impairing the carbohydrate binding loop from binding to/interacting with ligand (i.e., carbohydrates). This demonstrates that the 5E5 Fab indirectly inhibits the ASGR-1 CBD/Ligand binding.

The depicted structure also allows one to identify specific core ASGR amino acid residues for the interaction interface of 5E5 with ASGR-1. This was defined as residues that are within 5 Å of the 5E5 protein. The core residues are as follows: H161, E162, W195, E196, Q198, K199, F200, Q202, H203, H204, G232, F233, K234, N235, W236, R237, P238, D261, G262, R263 (SEQ ID NO:5).

The structures were also used to identify boundary ASGR-1 amino acid residues for the interaction interface with 5E5. These residues were ASGR-1 residues that were from 5-8 Å of the 5E5 protein. The boundary residues are as follows: V159, E160, R163, T193, S194, E197, V201, 1205, G206, P207, Y229, E230, T231, E239, F258, T259, D260, W264 (SEQ ID NO:5).

Specific core 5E5 amino acid residues of the interaction interface with ASGR-1 were defined as 5E5 residues that are within 5 Å of the ASGR-1 protein. The core 5E5 Heavy Chain residues include: S30, N31, W52, Y53, D54, S56, N57, Y59, Y101, S102, S103, G104, W105, Y106, D107; and the core 5E5 Light Chain residues include: 5E5 Light Chain: Q27, R30, D32, H91, Y92, S93, Y94.

Boundary 5E5 amino acid residues of the interaction interface with ASGR-1 were defined as 5E5 residues that are 5-8 Å from the ASGR-1 protein. The boundary 5E5 Heavy Chain residues include: Y32, V33, V50, G55, K58, N74, E99, V100, Y108; and the boundary 5E5 Light Chain residues include: I2, G28, 129, L33, Q90, P95, R96.

**Methods**

**Expression and purification of protein samples**

The 5E5 Fab fragment was generated by cleaving the 5E5 mAb with caspase 3. Post caspase cleavage, the Fab was isolated by purification on a MonoS ion exchange column. Ni Sepharose Excel subtraction was then performed to ensure the Fc domain was removed from the sample.

5E5 mAb Heavy Chain (SEQ ID NO: 32695):

QVQLVESGGGVVQPGRSLRLSCAASGFTFSNYVMHWVRQAPGKGLEWVAVIWYDGSNKY YADSVKGRFTISRDNSKNTLYLQMNSLRAEDTAVYYCTREVYSSGWYDYGMDVWGQGTT VTVSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCGSDEVDGGDKTHTCPP CPAPELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQVYT LPPSREEMTKNQVSLTCLVKGFYPSDIAVEWESNGQPENNYKTTPPVLDSDGSFFLYSKLTV DKSRWQQGNVFSCSVMHEALHNHYTQKSLSLSPGHHHHHH

5E5 mAb Light Chain (SEQ ID NO:32696):

DIQMTQSPSSLSASVGDRVTITCRASQGIRNDLGWYQQKPGKAPKRLIYAASSLQSGVPSRF SGSGSGTEFTLTISSLQPEDFATYYCLQHYSYPRTFGQGTKVEVKRTVAAPSVFIFPPSDEQL KSGTASVVCLLNNFYPREAKVQWKVDNALQSGNSQESVTEQDSKDSTYSLSSTLTLSKADY EKHKVYACEVTHQGLSSPVTKSFNRGEC

5E5 Fab Heavy Chain (Post Cleavage) (SEQ ID NO:32697):

QVQLVESGGGVVQPGRSLRLSCAASGFTFSNYVMHWVRQAPGKGLEWVAVIWYDGSNKY YADSVKGRFTISRDNSKNTLYLQMNSLRAEDTAVYYCTREVYSSGWYDYGMDVWGQGTT

VTVSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCGSDEVD

5E5 Fab Light Chain (Post Cleavage) (SEQ ID NO:32698):

DIQMTQSPSSLSASVGDRVTITCRASQGIRNDLGWYQQKPGKAPKRLIYAASSLQSGVPSRF SGSGSGTEFTLTISSLQPEDFATYYCLQHYSYPRTFGQGTKVEVKRTVAAPSVFIFPPSDEQL KSGTASVVCLLNNFYPREAKVQWKVDNALQSGNSQESVTEQDSKDSTYSLSSTLTLSKADY EKHKVYACEVTHQGLSSPVTKSFNRGEC

Complex formation and crystallization

The ASGR-1 CBD / 5E5 Fab complex was made by mixing a molar excess of ASGR-1 CBD with 5E5 Fab. The complex was separated from excess ASGR-1 by purification on a size exclusion chromatography column. The ASGR-1 CBD / 5E5 Fab complex was concentrated to 10 mg/ml and crystallizes in 0.1 M Tris pH 8.5, 12% PEG 4000.

Data collection and structure determination

The dataset for the ASGR-1 CBD / 5E5 Fab complex crystal was collected on beamline 5.0.2 at the Berkeley synchrotron and processed with Mosflm[1]/Aimless[2].

ASGR-1 CBD / 5E5 Fab complex crystals grow in the $P2_1$ space group with unit cell dimensions a=62.93, b=41.75, c=118.89 Å and $\beta$=97.16 with one complex molecule per asymmetric unit, and diffract to 1.95 Å resolution. The ASGR-1 CBD / 5E5 Fab complex structure was solved by molecular replacement with the program Molrep[2]. The structure was improved with multiple rounds of model building with Coot[3] and refinement with Phenix[4], to a final R=25.9 / $R_{free}$=30.5. While the electron density for the ASGR-1 CBD and 5E5 Fab variable domain (along with the corresponding interface) is quite good, the electron density for the 5E5 constant domain is poor (most likely due to poor packing within the crystal lattice). This likely explains the higher $R/R_{free}$ observed from this structure refinement.

Core interaction interface amino acids were determined as being all amino acid residues with at least one non-hydrogen atom less than or equal to 5 Å from the partner protein. 5 Å was chosen as the core region cutoff distance to allow for atoms within a van der Waals radius plus a possible water-mediated hydrogen bond. Boundary interaction interface amino acids were determined as all amino acid residues with at least one non-hydrogen atom less than or equal to 8 Å from the partner protein but not included in the core interaction list. Less than or equal to 8 Å was chosen as the boundary region cutoff distance to allow for the length of an extended arginine amino acid. Amino acids that met these distance criteria were calculated with the program PyMOL[5].

**References**

1. Battye, T.G., Kontogiannis, L., Johnson, O., Powell, H.R. & Leslie, A.G. iMOSFLM: a new graphical interface for diffraction-image processing with MOSFLM. Acta Crystallogr D Biol Crystallogr 67, 271-81 (2011).
2. CCP4. The CCP4 suite: programs for protein crystallography. Acta Crystallogr D Biol Crystallogr 50, 760-3 (1994).
3. Emsley, P., Lohkamp, B., Scott, W.G. & Cowtan, K. Features and development of Coot. Acta Crystallogr D Biol Crystallogr 66, 486-501 (2010).
4. Adams, P.D. et al. PHENIX: a comprehensive Python-based system for macromolecular structure solution. Acta Crystallogr D Biol Crystallogr 66, 213-21 (2010).
5. DeLano, W.L. The PyMOL Molecular Graphics System. (Palo Alto, 2002).


**C. Crystal Structure of ASGR-1 Carbohydrate Binding Domain (CBD) with 22G5**

The present example presents the crystal structure of the ASGR-1 CBD bound to the Fab fragment of 22G5, determined to 2.1 Å resolution (the conditions of which are described above in B). This structure, depicted in FIG. 23A&B, shows that when 22G5 binds to/interacts with ASGR- 1, a conformational rearrangement of the carbohydrate binding loop occurs, impairing the carbohydrate binding loop from binding to/interacting with ligand (i.e., carbohydrates). This demonstrates that the 22G5 Fab indirectly inhibits the ASGR-1 CBD/Ligand binding.

The depicted structure also allows one to identify specific core ASGR amino acid residues for the interaction interface of 22G5 with ASGR-1. This was defined as residues that are within 5 Å of the 22G5 protein. The core residues are as follows: W167, S171, G172, K173, A174, A176, D177, N180, Y181, R183, L184, E185, D186, Q270, P272, W275 (SEQ ID NO:5).

The structures were also used to identify boundary ASGR-1 amino acid residues for the interaction interface with 22G5. These residues were ASGR-1 residues that were from 5-8 Å of the 22G5 protein. The boundary residues are as follows: P155, N157, W158, F168, S169, R170, W175, A178, D179, C182, A187, W211, C269, R271, Y273, R274, C277, T279 (SEQ ID NO:5).

Specific core 22G5 amino acid residues of the interaction interface with ASGR-1 were defined as 22G5 residues that are within 5 Å of the ASGR-1 protein. The core 22G5 Heavy Chain residues include: A33, V50, 151, S52, R53, S54, G55, G56, Y57, Y59, R99, A101, A103, G104, E106; and the core 22G5 Light Chain residues include: 22G5 Light Chain: Y32, S91, Y92, R93, Thr94, Pro95, F97.

Boundary 22G5 amino acid residues of the interaction interface with ASGR-1 were defined as 22G5 residues that are 5-8 Å from the ASGR-1 protein. The boundary 22G5 Heavy Chain residues include: S30, S31, Y32, M34, N35, W47, S49, T58, R72, N74, L100, V102, S105; and the boundary 22G5 Light Chain residues include: I2, Q27, N28, NAG100, I29, S30, S31, Q90, L96.

**Methods:**

The same methods were followed as described above in Example 10B except for the following changes:

The 22G5 Fab fragment was generated by cleaving the 22G5-IgG4 mAb with papain;

The ASGR-1 CBD / 22G5 Fab complex was concentrated to 8 mg/ml and crystallized in 0.1 Bis-Tris pH 6.5, 0.2 sodium malonate, 20% PEG 3350;

The dataset was processed with XDS/Aimless;

ASGR-1 CBD / 22G5 Fab complex crystals grow in the P212121 space group with unit cell dimensions a=46.04, b=80.34, c=169.14 Å with one complex molecule per asymmetric unit, and diffract to 2.1 Å resolution; and

The structure was improved with multiple rounds of model building with Coot3 and refinement with Phenix4, to a final R=17.8 / Rfree=22.5.

**D. Crystal Structure of ASGR-1 Carbohydrate Binding Domain (CBD) with 4A2**

The present example presents the crystal structure of the ASGR-1 CBD bound to the Fab fragment of 4A2, determined to 2.15 Å resolution (the conditions of which are described above in section B of this Example). This structure, depicted in Figures 24, 25 and 26, shows that when 4A2 binds to/interacts with ASGR-1, a conformational rearrangement of the carbohydrate binding loop occurs, impairing the carbohydrate binding loop from binding to/interacting with ligand (i.e., carbohydrates). This demonstrates that the 4A2 Fab indirectly inhibits the ASGR-1 CBD/Ligand binding.

The depicted structure also allows one to identify specific core ASGR amino acid residues for the interaction interface of 4A2 with ASGR-1. This was defined as residues that are within 5 Å of the 4A2 protein. The core residues are as follows: R170, W195, E196, K199, Q202, H203, H204, 1205, G206, P207, V208, F233, K234, N235, W236, P238, D260, D261, G262, R263, R274 (SEQ ID NO:5).

The structures were also used to identify boundary ASGR-1 amino acid residues for the interaction interface with 4A2. These residues were ASGR-1 residues that were from 5-8 Å of the 4A2 protein. The boundary residues are as follows: N157, V159, F168, S169, S171, S194, Q198, F200, V201, T210, R237, E239, Q240, F258, T259, W264 (SEQ ID NO:5).

Specific core 4A2 amino acid residues of the interaction interface with ASGR-1 were defined as 4A2 residues that are within 5 Å of the ASGR-1 protein. The core 4A2 Heavy Chain residues include: T28, F29, T30, N31, Y32, D33, W50, H52, S55, N57, S99, S100, G101, W102, Y103; and the core 4A2 Light Chain residues include: 4A2 Light Chain: H31, S33, N34, N36, Y38, W56, Y97, Y98.

Boundary 4A2 amino acid residues of the interaction interface with ASGR-1 were defined as 4A2 residues that are 5-8 Å from the ASGR-1 protein. The boundary 4A2 Heavy Chain residues include: Y27, 134, N35, W47, M51, P53, N54, G56, T58, G59, Y104, D106; and the boundary 4A2 Light Chain residues include: I29, S32, N35, N37, Y55, T59, Q96, N99, T100.

The coordinates for the ASGR-1 CBD/4A2 crystal structure complex are presented in Table 10.2.

**Methods:**

The same methods were followed as described above in part B of this Example except for the following changes:

1. For this antibody only, a double stop codon was inserted at the end of CH1 domain that allowed for expression of a 4A2 Fab. The Fab purification was carried out via an affinity and a cation exchanger column. The final sequence of 4A2 Fab is:

Heavy Chain (SEQ ID NO:32650):

QVQLVQSGTEVKKPGASVKVSCKASGYTFTNYDINWVRQATGQGLEWMGWMHPN
SGNTGYAQKFQGRVTLTRDTSISTAYMELSSLRSEDTAVYYCASSSGWYYFDYWGQ
GTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCGSD
EVDGGD

Light Chain (SEQ ID NO:32651):

DIVMTQSPDSLAVSLGERATINCKSSQSILHSSNNNNYLAWFQQKPGQPPKLL
LYWASTRESGVPDRFSGSGSGTDFTLTISSLQPEDVAVYYCQQYYNTPVTFGP

GTKVGIKRTVAAPSVFIFPPSDEQLKSGTASVVCLLNNFYPREAKVQWKVDN
ALQSGNSQESVTEQDSKDSTYSLSSTLTLSKADYEKHKVYACEVTHQGLSSP
VTKSFNRGEC

1. The ASGR-1 CBD / 4A2 Fab complex was concentrated to 20 mg/ml and crystallized in 0.2 M Tri-Lithium citrate and 20% PEG3350;
2. The ASGR-1 CBD / 4A2 Fab complex crystals grow in the $P2_12_12_1$ space group with unit cell dimensions a=63.42, b=76.37, c=156.67 Å with one complex molecule per asymmetric unit, and diffract to 2.15 Å resolution;

and
3. The structure was improved with multiple rounds of model building with Coot[3] and refinement with Phenix[4], to a final R=17.9 / $R_{free}$=21.8.

## Crystal Structure of ASGR-1 Carbohydrate Binding Domain (CBD) with 7E11

The present example presents the crystal structure of the ASGR-1 CBD bound to the Fab fragment of 7E11, determined to 2.0 Å resolution (the conditions of which are described above in section B of this Example). This structure, depicted in FIGS. 27 and 28, shows that when 7E11 binds to/interacts with ASGR-1, a conformational rearrangement of the carbohydrate binding loop occurs, impairing the carbohydrate binding loop from binding to/interacting with ligand (i.e., carbohydrates). This demonstrates that the 7E11 Fab indirectly inhibits the ASGR-1 CBD/Ligand binding.

The depicted structure also allows one to identify specific core ASGR amino acid residues for the interaction interface of 7E11 with ASGR-1. This was defined as residues that are within 5 Å of the 7E11 protein. The core residues are as follows: H161, S194, W195, E196, Q198, K199, F200, Q202, H203, F233, K234, N235, W236, R237, P238, R263 (SEQ ID NO:5).

The structures were also used to identify boundary ASGR-1 amino acid residues for the interaction interface with 7E11. These residues were ASGR-1 residues that were from 5-8 Å of the 7E11 protein. The boundary residues are as follows: E160, E162, V192, T193, E197, V201, H204, Y229, E230, T231, G232, E239, Q240, P241, D261, G262, W264 (SEQ ID NO:5).

Specific core 7E11 amino acid residues of the interaction interface with ASGR-1 were defined as 7E11 residues that are within 5 Å of the ASGR-1 protein. The core 7E11 Heavy Chain residues include: S30, S31, 150, W52, H53, S56, N57, Y59, S01, M102, G103; and the core 7E11 Light Chain residues include: I30, Y32, T91, Y92, S93, T94, I96.

Boundary 7E11 amino acid residues of the interaction interface with ASGR-1 were defined as 7E11 residues that are 5-8 Å from the ASGR-1 protein. The boundary 7E11 Heavy Chain residues include: T28, F29, F32, G33, H35, W47, 151, D54, K58, D99, L100, G104; and the boundary 7E11 Light Chain residues include: I2, Q27, N28, 129, S31, L33, N34, T50, S67, Q89, Q90, P95.

## Methods:

The same methods were followed as described above in part B of this example except for the following changes: The 7E11 Fab fragment was generated by cleaving the 7E11 mAb with caspase 3:

**7E11 mAb Heavy Chain (SEQ ID NO:32652):**

QVQLVESGGGVVQPGRSLRLSCAASGFTFSSFGMHWVRQAPGKGLEWVAII
WHDGSNKYYADSVKGRFTISRDNSNNTLYLQMSSLRAEDTAVYYCARDLS
MGGMDVWGQGTTVTVSSASTKGPSVFPLAPSSKSTSGGTAALGCLVKDYFP
EPVTVSWNSGALTSGVHTFPAVLQSSGLYSLSSVVTVPSSSLGTQTYICNVNH
KPSNTKVDKKVEPKSCGSDEVDGGDKTHTCPPCPAPELLGGPSVFLFPPKPKD
TLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLP
PSREEMTKNQVSLTCLVKGFYPSDIAVEWESNGQPENNYKTTPPVLDSDGSF
FLYSKLTVDKSRWQQGNVFSCSVMHEALHNHYTQKSLSLSPGHHHHHH

**7E11 mAb Light Chain (SEQ ID NO:32653):**

DIQMTQSPSSLSASVGDRVTIACRASQNIISYLNWYQQKPGKAPKFLIYTASSL
QSGVPSRFSGSGSGTDFTLTISSLQPEDFAIYYCQQTYSTPLTFGGGTKVEIKRT
VAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGE
C

**7E11 Fab Heavy Chain (Post-Cleavage) (SEQ ID NO:32654):**

QVQLVESGGGVVQPGRSLRLSCAASGFTFSSFGMHWVRQAPGKGLEWVAII
WHDGSNKYYADSVKGRFTISRDNSNNTLYLQMSSLRAEDTAVYYCARDLS
MGGMDVWGQGTTVTVSSASTKGPSVFPLAPSSKSTSGGTAALGCLVKDYFP
EPVTVSWNSGALTSGVHTFPAVLQSSGLYSLSSVVTVPSSSLGTQTYICNVNH
KPSNTKVDKKVEPKSCGSDEVD

**7E11 Fab Light Chain (Post-Cleavage) (SEQ ID NO:32655):**

DIQMTQSPSSLSASVGDRVTIACRASQNIISYLNWYQQKPGKAPKFLIYTASSL
QSGVPSRFSGSGSGTDFTLTISSLQPEDFAIYYCQQTYSTPLTFGGGTKVEIKRT
VAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGE
C

1. The ASGR-1 CBD / 7E11 Fab complex was concentrated to 20 mg/ml and crystallized in 0.2 M Potassium Phosphate monobasic and 20% PEG3350;
2. The ASGR-1 CBD / 7E11 Fab complex crystals grow in the P6222 space group with unit cell dimensions a=105.75, b=105.75, c=193.75 Å and $\gamma$=120.0 ° with one complex molecule per asymmetric unit, and diffract to 2.0 Å resolution;
3. The dataset was processed with XDS/CCP4;
4. The ASGR-1 CBD / 7E11 Fab complex structure was solved by molecular replacement with the program Phaser; and
5. The structure was improved with multiple rounds of model building with Coot[3] and refinement with Phenix[4], to a final R=21.4 / $R_{free}$=26.9.

**E. Crystal Structure of ASGR-1 Carbohydrate Binding Domain (CBD) with 4H6**

The present example presents the crystal structure of the ASGR-1 CBD bound to the Fab fragment of 4H6, determined to 2.6 Å resolution (the conditions of which are described above in section B of this Example). This structure, depicted in FIGS. 29 and 30, shows that when 4H6 binds to/interacts with ASGR-1, a conformational rearrangement of the carbohydrate binding loop occurs, impairing the carbohydrate binding loop from binding to/interacting with ligand (i.e., carbohydrates). This demonstrates that the 4H6 Fab indirectly inhibits the ASGR-1 CBD/Ligand binding.
The depicted structure also allows one to identify specific core ASGR amino acid residues for the interaction interface of 4H6 with ASGR-1. This was defined as residues that are within 5 Å of the 4H6 protein. The core residues are as follows: H161, E162, T193, S194, W195, E196, K199, Q202, T231, G232, F233, K234, N235, P238, D261, R263 (SEQ ID NO:5).
The structures were also used to identify boundary ASGR-1 amino acid residues for the interaction interface with 4H6. These residues were ASGR-1 residues that were from 5-8 Å of the 4H6 protein. The boundary residues are as follows: R163, V192, E197, Q198, H203, P207, D228, E230, W236, R237, D260, G262, W264 (SEQ ID NO:5).

Specific core 4H6 amino acid residues of the interaction interface with ASGR-1 were defined as 4H6 residues that are within 5 Å of the ASGR-1 protein. The core 4H6 Heavy Chain residues include: Y33, H35, W50, H52, S55, G57, T58, N59, D99, G100, T101, S102; and the core 4H6 Light Chain residues include: Q27, W32, A91, N92, S93, F94, F96.

Boundary 4H6 amino acid residues of the interaction interface with ASGR-1 were defined as 4H6 residues that are 5-8 Å from the ASGR-1 protein. The boundary 4H6 Heavy Chain residues include: D31, Y32, L34, W47, I51, N54, G56, Y60, Q65, S103, F104; and the boundary 4H6 Light Chain residues include: D1, I2, G28, 129, S30, R31, Y49, G50, Q89, Q90, P95.

**Methods:**

The same methods were followed as described above in part B of this example except for the following changes:

1. The 4H6 Fab fragment was generated by cleaving the 4H6 mAb with caspase 3.

**4H6 mAb Heavy Chain (SEQ ID NO:32656):**

QVQLVQSGAEVKKPGASVKVSCKASGYTFTDYYLHWVRQAPGQGLEWMGWIHPN
SGGTNYAQKFQGRVTMTRDTSISTAYMGLSSLRSDDTAVYYCARDGTSSFDYWGQ
GTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCGSD
EVDGGDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKA
LPAPIEKTISKAKGQPREPQVYTLPPSREEMTKNQVSLTCLVKGFYPSDIAVEWESNG
QPENNYKTTPPVLDSDGSFFLYSKLTVDKSRWQQGNVFSCSVMHEALHNHYTQKSL
SLSPGHHHHHH

**4H6 mAb Light Chain (SEQ ID NO:32657):**

DIQMTQSPSSVSASVGDRVTITCRASQGISRWLAWYQQKPGKAPKLLIYGASSLQSG
VPSRFSGSGSGTDFTLTISSLQPEDFAIYYCQQANSFPFTFGPGTKVDIKGTVAAPSVFI
FPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC

**4H6 Fab Heavy Chain (Post-Cleavage) (SEQ ID NO:32658):**

QVQLVQSGAEVKKPGASVKVSCKASGYTFTDYYLHWVRQAPGQGLEWMGWIHPN
SGGTNYAQKFQGRVTMTRDTSISTAYMGLSSLRSDDTAVYYCARDGTSSFDYWGQ
GTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCGSD
EVD

**4H6 Fab Light Chain (Post-Cleavage) (SEQ ID NO:32659):**

DIQMTQSPSSVSASVGDRVTITCRASQGISRWLAWYQQKPGKAPKLLIYGASSLQSG
VPSRFSGSGSGTDFTLTISSLQPEDFAIYYCQQANSFPFTFGPGTKVDIKGTVAAPSVFI
FPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC

2. The ASGR-1 CBD / 4H6 Fab complex was concentrated to 20 mg/ml and crystallized in 0.2M Sodium fluoride, 0.1 M Bis Tris propane pH8.5, 20% PEG3350;
3. The dataset was collected on beamline ID22 at the APS synchrotron and processed with HKL2000/CCP4;
4. The ASGR-1 CBD / 4H6 Fab complex crystals grow in the $P12_11$ space group with unit cell dimensions a=57.20, b=43.58, c=131.65 Å and β=90.7° with one complex molecule per asymmetric unit, and diffract to 2.6 Å resolution;

5. The ASGR-1 CBD / 4H6 Fab complex structure was solved by molecular replacement with the program Phaser; and
6. The structure was improved with multiple rounds of model building with Coot[3] and refinement with Phenix[4], to a final R=17.9 / $R_{free}$=22.5.

**F. Crystal Structure of ASGR-1 Carbohydrate Binding Domain (CBD) with 72G9**

The present example presents the crystal structure of the ASGR-1 CBD bound to the Fab fragment of 72G9, determined to 2.55 Å resolution (the conditions of which are described above in section B of this Example). This structure, depicted in FIGS. 31 and 32A and 32B, shows that when 72G9 binds to/interacts **with** ASGR-1, the CDR H2 loop of the Fab fragment appears to directly block the ligand (i.e., carbohydrate) binding/interacting to ASGR-1 CBD. This demonstrates that the 72G9 Fab directly inhibits the ASGR-1 CBD/Ligand binding.
The depicted structure also allows one to identify specific core ASGR amino acid residues for the interaction interface of 72G9 with ASGR-1. This was defined as residues that are within 5 Å of the 72G9 protein. The core residues are as follows: D216, Q217, N218, G219, P220, W221, Y229, E230, K234, W236, E239, Q240, P241, D242, D243, W244, Y245, G246, L249, G250, G251, G252, D254, Q270 ((SEQ ID NO:5).
The structures were also used to identify boundary ASGR-1 amino acid residues for the interaction interface with 72G9. These residues were ASGR-1 residues that were from 5-8 Å of the 72G9 protein. The boundary residues are as follows: H215, K222, T231, G232, R237, P238, H247, G248, E253, C255, D266, V268, C269 (SEQ ID NO:5).
Specific core 72G9 amino acid residues of the interaction interface with ASGR-1 were defined as 72G9 residues that are within 5 Å of the ASGR-1 protein. The core 72G9 Heavy Chain residues include: G26, F27, T28, S30, S31, Y32, S33, S52, G53, S54, S56, Y57, Y59, R98, G100, S101, R102; and the core 72G9 Light Chain residues include: Y32, Y49, T50, Q55, S91, H92, S93, F94, F96.
Boundary 72G9 amino acid residues of the interaction interface with ASGR-1 were defined as 72G9 residues that are 5-8 Å from the ASGR-1 protein. The boundary 72G9 Heavy Chain residues include: V2, F29, N35, S50, T51, S55, 158, R72, G99, G103, F104, D105 ; and the boundary 72G9 Light Chain residues include: S28, 129, T30, N33, L46, S53, L54, S56, Q89, Q90, P95.

**Methods:**

The same methods were followed as described above in part B of this example except for the following changes:

1. The 72G9 Fab fragment was generated by cleaving the 72G9 mAb with caspase 3.

**72G9 mAb Heavy Chain (SEQ ID NO:32660):**

EVQLVESGGGLVKPGGSLRLSCAASGFTFSSYSMNWVRQAPGKGLEWVSSISGSSSY
IYYADSVKGRFTISRDNAKNSLYLQMNSLRAEDTAVYFCARGGSRGFDPWGQGTLV
TVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCGSDEVDGG
DKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPI
EKTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSP
GHHHHHH

**72G9 mAb Light Chain (SEQ ID NO:32661):**

DIQMTQSPSSLSASVGDRVTITCRASQSITSYLNWYQQKPGKAPKLLIYTASSLQSGV
PSRFSGSGSGTDFTLTISSLQPEDFATYYCQQSHSFPFTFGPGTKVDIKRTVAAPSVFIF
PPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC

**72G9 Fab Heavy Chain (Post-Cleavage) (SEQ ID NO:32662):**

EVQLVESGGGLVKPGGSLRLSCAASGFTFSSYSMNWVRQAPGKGLEWVSSISGSSSY IYYADSVKGRFTISRDNAKNSLYLQMNSLRAEDTAVYFCARGGSRGFDPWGQGTLV TVSSASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCGSDEVD

**72G9 Fab Light Chain (Post-Cleavage) (SEQ ID NO:32663):**

DIQMTQSPSSLSASVGDRVTITCRASQSITSYLNWYQQKPGKAPKLLIYTASSLQSGV PSRFSGSGSGTDFTLTISSLQPEDFATYYCQQSHSFPFTFGPGTKVDIKRTVAAPSVFIF PPSDEQLKSGTASVVCLLNNFYPREAKVQWKVDNALQSGNSQESVTEQDSKDSTYS LSSTLTLSKADYEKHKVYACEVTHQGLSSPVTKSFNRGEC

2. The 72G9 Fab/ASGR-1 CBD complex was concentrated to 0.2 M Magnesium Sulfate heptahydrate, 20% PEG3350;

3. The ASGR-1 CBD / 72G9 Fab complex crystals grew in the $P2_1$ space group with unit cell dimensions a=100.98, b=64.95, c=100.68 Å and β=96.43° with one complex molecule per asymmetric unit, and diffract to 2.55 Å resolution;

4. The dataset was processed with XDS/CCP4;

5. The ASGR-1 CBD / 72G9 Fab complex structure was solved by molecular replacement with the program Phaser; and

6. The structure was improved with multiple rounds of model building with Coot[3] and refinement with Phenix[4], to a final R=20.4 / $R_{free}$=23.4.

**G. Crystal Structure of ASGR-1 Carbohydrate Binding Domain (CBD) with 194A4**

The present example presents the crystal structure of the ASGR-1 CBD bound to the Fab fragment of 194A4, determined to 2.6 Å resolution (the conditions of which are described above in section B of this Example). This structure, depicted in FIGS. 33 and 34, shows that when 194A4 binds to/interacts with ASGR-1, a conformational rearrangement of the carbohydrate binding loop occurs, impairing the carbohydrate binding loop from binding to/interacting with ligand (i.e., carbohydrates). This demonstrates that the 194A4 Fab indirectly inhibits the ASGR-1 CBD/Ligand binding.

The depicted structure also allows one to identify specific core ASGR amino acid residues for the interaction interface of 194A4 with ASGR-1. This was defined as residues that are within 5 Å of the 194A4 protein. The core residues are as follows: T193, S194, W195, E196, P220, W221, G226, T227, D228, Y229, E230, T231, G232, F233, K234, N235, W236, R237, P238, E239, G252 (SEQ ID NO:5).

The structures were also used to identify boundary ASGR-1 amino acid residues for the interaction interface with 194A4. These residues were ASGR-1 residues that were from 5-8 Å of the 194A4 protein. The boundary residues are as follows: H161, E162, V191, V192, E197, Q198, D216, G219, K222, W223, D225, R263, W264 (SEQ ID NO:5).

Specific core 194A4 amino acid residues of the interaction interface with ASGR-1 were defined as 194A4 residues that are within 5 Å of the ASGR-1 protein. The core 194A4 Heavy Chain residues include: V31, Y32, Y33, W50, N52, S55, G57, R98, G99, Y100, D101, I 102, T204; and the core 194A4 Light Chain residues include: V29, S30, I32, Y33, L47, Y50, R55, A56, T57, Y94.

Boundary 194A4 amino acid residues of the interaction interface with ASGR-1 were defined as 194A4 residues that are 5-8 Å from the ASGR-1 protein. The boundary 194A4 Heavy Chain residues include: V2, Y27, T30, L34, N35, P53, N54, G56, T58, N59, A97, L103, G105; and the boundary 194A4 Light Chain residues include: G28, N31, L48, I49, G51, N54, G58, 159, S68, G69, D93, S95.

**Methods:**

The same methods were followed as described above in part B of this example except for the following changes:

1. The 194A4 Fab fragment was generated by cleaving the 194A4 mAb with caspase 3.

**194A4 mAb Heavy Chain (SEQ ID NO:32664):**

QVQLVQSGTEVKKPGASLKVSCKASGYTFTVYYLNWVRQAPGQGLEWMGWINPN
SGGTNYAQKFQGRVTMTRDTSISTAYMELSRLRSDDTAVYYCARGYDILTGWGQG
TLVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCGSDE
VDGGDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKA
LPAPIEKTISKAKGQPREPQVYTLPPSREEMTKNQVSLTCLVKGFYPSDIAVEWESNG
QPENNYKTTPPVLDSDGSFFLYSKLTVDKSRWQQGNVFSCSVMHEALHNHYTQKSLSLSPG
HHHHHH

**194A4 mAb Light Chain (SEQ ID NO:32665):**

EIVLTQSPGTLSLSPGERATLSCRASRGVSNIYLAWYQQKPGQAPRLLIYGASNRATG
IPDRFSGSGSGTDFTLTISRLEPEDFAVYYCQHNDYSMFTFGPGTKVDIKRTVAAPSV
FIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC

**194A4 Fab Heavy Chain (Post-Cleavage) (SEQ ID NO:32666):**

QVQLVQSGTEVKKPGASLKVSCKASGYTFTVYYLNWVRQAPGQGLEWMGWINPN
SGGTNYAQKFQGRVTMTRDTSISTAYMELSRLRSDDTAVYYCARGYDILTGWGQG
TLVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCGSDE
VD

**194A4 Fab Light Chain (Post-Cleavage) (SEQ ID NO:32667):**

EIVLTQSPGTLSLSPGERATLSCRASRGVSNIYLAWYQQKPGQAPRLLIYGASNRATG
IPDRFSGSGSGTDFTLTISRLEPEDFAVYYCQHNDYSMFTFGPGTKVDIKRTVAAPSV
FIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC

2. The 194A4 Fab/ASGR-1 CBD complex was concentrated to 13.1 mg/mL and crystallized with 0.2 M Sodium chloride, 0.1M MES pH6.0, 20% PEG2000 MME;
3. The dataset was processed with XDS/CCP4;
4. The 194A4 Fab/ASGR-1 CBD complex crystals grow in the $P2_12_12_1$ space group with unit cell dimensions a=52.23, b=66.40, c=177.75 Å with one complex molecule per asymmetric unit, and diffract to 2.6 Å resolution;
5. The ASGR-1 CBD / 194A4 Fab complex structure was solved by molecular replacement with the program Phaser; and
6. The structure was improved with multiple rounds of model building with Coot[3] and refinement with Phenix[4], to a final R=20.1 / $R_{free}$=24.6.

**H. Crystal Structure of ASGR-1 Carbohydrate Binding Domain (CBD) with 54E9**

The present example presents the crystal structure of the ASGR-1 CBD bound to the Fab fragment of 54E9, determined to 2.6 Å resolution (the conditions of which are described above in section B of this Example). This structure, depicted in FIG. 35 and FIG. 36A and FIG. 36B, shows that when 54E9 binds to/interacts with ASGR-1, the CDR H3 loop of the Fab fragment appears to directly block the ligand (i.e., carbohydrate) from binding/interacting to ASGR-1 CBD. This demonstrates that the 54E9 Fab directly inhibits the ASGR-1 CBD/Ligand binding.

The depicted structure also allows one to identify specific core ASGR amino acid residues for the interaction interface of 54E9 with ASGR-1. This was defined as residues that are within 5 Å of the 54E9 protein. The core residues are as follows: W195, N209, N235, R237, P238, E239, Q240, D242, H257, T259, D260, D261, R263, N265, D267, R271, Y273 (SEQ ID NO:5).

The structures were also used to identify boundary ASGR-1 amino acid residues for the interaction interface with 54E9. These residues were ASGR-1 residues that were from 5-8 Å of the 54E9 protein. The boundary residues are as follows: Q198, Q202, P207, V208, F233, W236, D243, E253, F258, G262, W264, D266 (SEQ ID NO:5).

Specific core 54E9 amino acid residues of the interaction interface with ASGR-1 were defined as 54E9 residues that are within 5 Å of the ASGR-1 protein. The core 54E9 Heavy Chain residues include: N30, S31, Y32, S52, Y54, N55, K59, R98, D100, F101, W102, S103, G104, Y105, K107, D110; and the core 54E9 Light Chain residues include: none.

Boundary 54E9 amino acid residues of the interaction interface with ASGR-1 were defined as 54E9 residues that are 5-8 Å from the ASGR-1 protein. The boundary 54E9 Heavy Chain residues include: V2, Y27, T28, F29, G33, W50, A53, G56, N57, H99, Y106, G108; and the boundary 54E9 Light Chain residues include: N31, Y50, V51, Q54.

**Methods:**

The same methods were followed as described above in part B of this example except for the following changes:

1. The 54E9 Fab fragment was generated by cleaving the 54E9 mAb with caspase 3.

**54E9 mAb Heavy Chain (SEQ ID NO:32668):**

QVQLVQSGAEVKKPGASVKVSCKASGYTFNSYGISWVRLAPGQGLEWMGWISAYN
GNTKNAQKLQGRVTMTTDTSTSTAYMELRSLRSDDTAVYYCARHDFWSGYYKGM
DVWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEP
KSCGSDEVDGGDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSDIAV
EWESNGQPENNYKTTPPVLDSDGSFFLYSKLTVDKSRWQQGNVFSCSVMHEALHN
HYTQKSLSLSPGHHHHHH

**54E9 mAb Light Chain (SEQ ID NO: 32669):**

QSVLTQPPSASGTPGQRVTISCSGSNSNIGNNIVTWYQQLPGTAPKLLIYVNDQRPSG
VPDRFSGSKSGTSASLAISGLQSEDEADYYCAAWDDSLNGWVFGGGTTLTVLGQPK

AAPSVTLFPPSSEELQANKATLVCLISDFYPGAVTVAWKADSSPVKAGVETTTPSKQ
SNNKYAASSYLSLTPEQWKSHRSYSCQVTHEGSTVEKTVAPTECS

**54E9 Fab Heavy Chain (Post-Cleavage) (SEQ ID NO:32670):**

QVQLVQSGAEVKKPGASVKVSCKASGYTFNSYGISWVRLAPGQGLEWMGWISAYN
GNTKNAQKLQGRVTMTTDTSTSTAYMELRSLRSDDTAVYYCARHDFWSGYYKGM
DVWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEP
KSCGSDEVD

54E9 **Fab Light Chain (Post-Cleavage) (SEQ ID NO:32671):**

QSVLTQPPSASGTPGQRVTISCSGSNSNIGNNIVTWYQQLPGTAPKLLIYVNDQRPSG
VPDRFSGSKSGTSASLAISGLQSEDEADYYCAAWDDSLNGWVFGGGTTLTVLGQPK
AAPSVTLFPPSSEELQANKATLVCLISDFYPGAVTVAWKADSSPVKAGVETTTPSKQ
SNNKYAASSYLSLTPEQWKSHRSYSCQVTHEGSTVEKTVAPTECS

1. The 54E9 Fab/ASGR-1 CBD complex was concentrated to 14.8 mg/mL and crystallized with 0.2 M Magnesium Chloride hexahydrate, 20% PEG3350;
2. The dataset was processed with XDS/CCP4;
3. The 54E9 Fab/ASGR-1 CBD complex crystals grow in the I2 space group with unit cell dimensions a=64.66, b=41.65, c=224.59 Å and β=97.60° with one complex molecule per asymmetric unit, and diffract to 2.6 Å resolution;
4. The 54E9 Fab/ASGR-1 CBD complex structure was solved by molecular replacement with the program Phaser; and
5. The structure was improved with multiple rounds of model building with Coot[3] and refinement with Phenix[4], to a final R=19.1 / $R_{free}$=25.9.

**I. Crystal Structure of ASGR-1 Carbohydrate Binding Domain (CBD) with 218G4**

The present example presents the crystal structure of the ASGR-1 CBD bound to the Fab fragment of 218G4, determined to 2.4 Å resolution (the conditions of which are described above in section B of this Example). This structure, depicted in Figures 37 and 38, shows that when 218G4 binds to/interacts with ASGR-1, it impairs its ability to bind to ligand (e.g., carbohydrate). This demonstrates that the 218G4 Fab directly inhibits the ASGR-1 CBD/Ligand binding.
The depicted structure also allows one to identify specific core ASGR amino acid residues for the interaction interface of 218G4 with ASGR-1. This was defined as residues that are within 5 Å of the 218G4 protein. The core residues are as follows: R170, S171, G172, A174, H204, 1205, G206, P207, V208, N209, H257, D260, N265, D267, Q270, R271, P272, Y273, R274 (SEQ ID NO:5).
The structures were also used to identify boundary ASGR-1 amino acid residues for the interaction interface with 218G4. These residues were ASGR-1 residues that were from 5-8 Å of the 218G4 protein. The boundary residues are as follows: W167, F168, S169, K173, W175, D177, Y181, Q202, H203, T210, W211, R237, F258, T259, D261, D266, V268, C269, W275 (SEQ ID NO:5).
Specific core 218G4 amino acid residues of the interaction interface with ASGR-1 were defined as 218G4 residues that are within 5 Å of the ASGR-1 protein. The core 218G4 Heavy Chain residues include: Q1, V2, F27, S30, S31, Y32, Y53, D54, W99, Y100, Y101, Y102; and the core 218G4 Light Chain residues include: Y33, Y50, D51, N53, K54, S57.
Boundary 218G4 amino acid residues of the interaction interface with ASGR-1 were defined as 218G4 residues that are 5-8 Å from the ASGR-1 protein. The boundary 218G4 Heavy Chain residues include: G26, T28, F29, G33, W52, G55, R72, N74, N98, Y103, Y104, D107, V108; and the boundary 218G4 Light Chain residues include: V34, S52, R55, P56, G58, G65.
The coordinates for the ASGR-1 CBD/GalNAc crystal structure complex are presented in Table 10.3.

**Methods:**

The same methods were followed as described above in part B of this example except for the following changes:

1. The 218G4 Fab fragment was generated by cleaving the 218G4 mAb with caspase 3.

**218G4 mAb Heavy Chain (SEQ ID NO:32672):**

QVQLVESGGGVVQPGRSLRLSCAASGFTFSSYGLHWVRQAPGKGLEWVAVIWYDGSNKY
YADSVKGRFTISRDNSKNTLYLQMNSLRVEDTAVYYCANWYYYYYGMDVWGQGTTVTV
SSASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCGSDEVDGGDKTHTCPPCPA
PELLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYSKLTVDK
SRWQQGNVFSCSVMHEALHNHYTQKSLSLSPGHHHHHH

**218G4 mAb Light Chain (SEQ ID NO: 32673):**

QSVLTQPPSVSAAPGQKVTISCSGSSSNIGNNYVSWYQQLPGTAPKLLLYDSNKRPSGIPARF SGSKSGTSATLGITGLQTGDEADYYCGTWDSSLNTVVFGGGTKLTVLSQPKAAPSVTLFPPS SEELQANKATLVCLISDFYPGAVTVAWKADSSPVKAGVETTTPSKQSNNKYAASSYLSLTP EQWKSHRSYSCQVTHEGSTVEKTVAPTECS

**218G4 Fab Heavy Chain (Post-Cleavage)(SEQ ID NO: 32674):**

QVQLVESGGGVVQPGRSLRLSCAASGFTFSSYGLHWVRQAPGKGLEWVAVIWYDGSNKY YADSVKGRFTISRDNSKNTLYLQMNSLRVEDTAVYYCANWYYYYYGMDVWGQGTTVTV SSASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCGSDEVD

**218G4 Fab Light Chain (Post-Cleavage):**

Same sequence as 218G4 mAb Light chain

1. The 218G4 Fab/ASGR-1 CBD complex was concentrated to 16.4 mg/mL and crystallized with 0.1M Tris pH8 and 1.6M Lithium Sulfate;
2. The dataset was collected from a single crystal on beamline ID22 at the Argonne National Laboratory and processed with XDS/CCP4;
3. The 218G4 Fab/ASGR-1 CBD complex crystals grow in the C222 space group with unit cell dimensions a=137.24, b=245.26, c=118.91 Å with two complex molecules per asymmetric unit and diffract to 2.6 Å resolution;
4. The 218G4 Fab/ASGR-1 CBD complex structure was solved by molecular replacement with the program Phaser; and
5. The structure was improved with multiple rounds of model building with Coot[3] and refinement with Phenix[4], to a final $R_{factor}$=18.4/ $R_{free}$=21.6.

**J. Crystal Structure of ASGR-1 Carbohydrate Binding Domain (CBD) with 176H4**

The present example presents the crystal structure of the ASGR-1 CBD bound to the Fab fragment of 176H5, determined to 2.3 Å resolution (the conditions of which are described above in section B of this Example). This structure, depicted in Figures 39 and 40, show that when 176H4 binds to/interacts with ASGR-1, it appears to block ligand (e.g., carbohydrate) binding by ASGR-1 CBD, with the paratope of the 176H4 antibody located directly on top of the carbohydrate binding pocket. This demonstrates that the 174H4 Fab directly inhibits the ASGR-1 CBD/Ligand binding.

The depicted structure also allows one to identify specific core ASGR amino acid residues for the interaction interface of 176H4 with ASGR-1. This was defined as residues that are within 5 Å of the 176H4 protein. The core residues are as follows: R170, S171, G172, K173, A174, D177, P207, V208, N209, R237, Q240, W244, G246, H247, G248, L249, E253, H257, T259, D260, N265, D267, Q270, R271, P272, Y273, R274 (SEQ ID NO:5).

The structures were also used to identify boundary ASGR-1 amino acid residues for the interaction interface with 176H4. These residues were ASGR-1 residues that were from 5-8 Å of the 176H4 protein. The boundary residues are as follows: S169, W175, A176, A178, T210, W211, W236, P238, E239, D242, Y245, G250, G251, F258, D261, G262, R263, W264, D266, V268, C269, W275 (SEQ ID NO:5).

Specific core 176H4 amino acid residues of the interaction interface with ASGR-1 were defined as 176H4 residues that are within 5 Å of the ASGR-1 protein. The core 176H4 Heavy Chain residues include: S31, W52, Y53, D54, Y57, Y59, D102, F103, W104; and the core 176H4 Light Chain residues include: H31, G32, D33, G34, K35, Y37, 197, Q98, I99.

Boundary 176H4 amino acid residues of the interaction interface with ASGR-1 were defined as 176H4 residues that are 5-8 Å from the ASGR-1 protein. The boundary 176H4 Heavy Chain residues include: T28, S30, Y32, G33, W47, 150, 151, S56, K58, Y60, K65, D99, H101, S105, G106; and the boundary 176H4 Light Chain residues include: I2, Q27, S28, L29, L30, T36, E55, Q95, S96, P100, W101.

**Methods:**

The same methods were followed as described above in part B of this example except for the following changes:

1. The 176H4 Fab fragment was generated by cleaving the 176H4 mAb with caspase 3.

**176H4 mAb Heavy Chain (SEQ ID NO:32675):**

QVQLVESGGGVVQPGRSLRLSCAASGFTFSSYGMHWVRQAPGKGLEWVAIIWYDG
SYKYYADSVKGRFTISRDNSKNTLYLQMSSLRAEDTAVYYCARDAHDFWSGYFAY
WGQGALVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSC
GSDEVDGGDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYKCKV
SNKALPAPIEKTISKAKGQPREPQVYTLPPSREEMTKNQVSLTCLVKGFYPSDIAVEW
ESNGQPENNYKTTPPVLDSDGSFFLYSKLTVDKSRWQQGNVFSCSVMHEALHNHYT
QKSLSLSPGHHHHHH

**176H4 mAb Light Chain (SEQ ID NO: 32676:**

DIVMTQTPLSLSVTPGQPASISCKSSQSLLHGDGKTYLYWYLQKPGQPPQLLIYEVSN
RFSGVPDRFSGSGSGTDFTLKISRVEAEDVGIYYCMQSIQIPWTFGQGTRVEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC

**176H4 Fab Heavy Chain (Post-Cleavage)(SEQ ID NO:32677):**

QVQLVESGGGVVQPGRSLRLSCAASGFTFSSYGMHWVRQAPGKGLEWVAIIWYDG
SYKYYADSVKGRFTISRDNSKNTLYLQMSSLRAEDTAVYYCARDAHDFWSGYFAY
WGQGALVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSC
GSDEVD

176H4 Fab Light Chain (Post-Cleavage):
Same sequence as 176H4 mAb Light chain

1. The 176H4 Fab/ASGR-1 CBD complex was concentrated to 14.9 mg/mL and crystallized 1with 0.2 M Sodium Nitrate, 20% PEG3350;
2. The dataset was collected from a single crystal on beamline ID22 at the Argonne National Laboratory and processed with XDS/CCP4;
3. The 176H4 Fab/ASGR-1 CBD complex crystals grow in the 1121 space group with unit cell dimensions a=68.31, b=126.31, c=134.13 Å and β=101.6° with two complex molecules per asymmetric unit, and diffract to 2.3 Å resolution;
4. The 176H4 Fab/ASGR-1 CBD complex structure was solved by molecular replacement with the program Phaser; and
5. The structure was improved with multiple rounds of model building with Coot[3] and refinement with Phenix[4], to a final $R_{factor}$=17.9 / $R_{free}$=23.3.

**K. Crystal Structure of ASGR-1 Carbohydrate Binding Domain (CBD) with 194C10**

The present example presents the crystal structure of the ASGR-1 CBD bound to the Fab fragment of 194C10, determined to 2.6 Å resolution (the conditions of which are described above in section B of this Example). This structure, depicted in Figures 41 and 42, shows that when 194C10 binds to/interacts with ASGR-1, it likely induces a conformational rearrangement of the carbohydrate binding loop, impairing ASGR-1 CBD from binding to ligand (e.g., carbohydrate), as well as possibly blocking the ligand (e.g., carbohydrate) binding by ASGR-1 CBD, with the paratope of the 194C10 Fab.

These data indicate that the 174H4 Fab may directly and/or indirectly inhibit the ASGR-1 CBD/Ligand binding.

The depicted structure also allows one to identify specific core ASGR amino acid residues for the interaction interface of 194C10 with ASGR-1. This was defined as residues that are within 5 Å of the 194C10 protein. The core residues are as follows: N157, R170, S171, G172, Q202, H203, H204, 1205, G206, P207, V208, N209, T210, D260, R271, P272, Y273, R274 (SEQ ID NO:5).

The structures were also used to identify boundary ASGR-1 amino acid residues for the interaction interface with 194C10. These residues were ASGR-1 residues that were from 5-8 Å of the 194C10 protein. The boundary residues are as follows: V156, W158, V159, H161, W167, F168, S169, K173, K199, F200, V201, W211, R237, H257, F258, T259, D261, D267, V268, Q270, W275 (SEQ ID NO:5).

Specific core 194C10 amino acid residues of the interaction interface with ASGR- 1 were defined as 194C10 residues that are within 5 Å of the ASGR-1 protein. The core 194C10 Heavy Chain residues include: R30, Y31, Y33, E50, S54, S56, N58, D98, Y99, G100; and the core 194C10 Light Chain residues include: N30, S31, Y33, F50, S54, S68, Y92, E93, W97.

Boundary 194C10 amino acid residues of the interaction interface with ASGR-1 were defined as 194C10 residues that are 5-8 Å from the ASGR-1 protein. The boundary 194C10 Heavy Chain residues include: S28, Y32, W34, S35, W47, G49, I51, S52, H53, G55, T57, R97, A101, F102, D103; and the boundary 194C10 Light Chain residues include: S28, V29, G32, L47, G51, A52, S53, R55, A56, G69, Q90, Q91, S94, S95.

The coordinates for the ASGR-1 CBD/GalNAc crystal structure complex are presented in Table 10.4.

**Methods:**

The same methods were followed as described above in part B of this example except for the following changes:

1. 194C10 Fab fragment was generated by cleaving the 194C10 mAb with caspase 3.

194C10 mAb Heavy Chain (SEQ ID NO:32678):

QVQLQQWGAGLLKPSETLSLTCAVSGGSFRYYYWSWIRQPPGKGLEWFGEI
NHAGSTNYNPSLKSRVTISIDTSKNQFSLKLRSVTAADTAVYYCARDYGAFDIWGQG
TMVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCG

194C10 mAb Light Chain (SEQ ID NO:32679):

EIVLTQSPGTLSLSPGERATLSCRASPSVNSGYLAWYQQKPGQTPRLLIFGASSRATGI
PDRFSASGSGADFTLTISRLEPEDFAVYFCQQYESSPWTFGQGTKVEIKRTVAAPSVFI
FPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC*

194C10 Fab Heavy Chain (Post-Cleavage)(SEQ ID NO:32680):

QVQLQQWGAGLLKPSETLSLTCAVSGGSFRYYYWSWIRQPPGKGLEWFGEINHAGS
TNYNPSLKSRVTISIDTSKNQFSLKLRSVTAADTAVYYCARDYGAFDIWGQGTMVT
VSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCGSDEVD

194C10 Fab Light Chain (Post-Cleavage):

Same sequence as 194C10 mAb Light chain

1. The 194C10 Fab/ASGR-1 CBD complex was concentrated to 13.6 mg/mL and crystallized with 0.2 M Ammonium Sulfate, 0.1 M Tris pH7.5, 20% PEG5000MME;
2. The dataset was collected from a single crystal on beamline ID22 at the Argonne National Laboratory and processed with XDS/CCP4;
3. The 194C10 Fab/ASGR-1 CBD complex crystals grow in the $P12_11$ space group with unit cell dimensions a=65.62, b=130.44, c=85.93 Å and β=111.6 ° with two complex molecules per asymmetric unit, and diffract to 2.6 Å resolution.;
4. The 194C10 Fab/ASGR-1 CBD complex structure was solved by molecular replacement with the program Phaser; and
5.The structure was improved with multiple rounds of model building with Coot[3] and refinement with Phenix[4], to a final $R_{factor}$=17.1 / $R_{free}$=22.8.

**L. Interaction between GalNAc, ASGR-1 and certain Antibodies**

The structure of the 72G9/ASGR-1 complex (Item G above) was overlaid on the ASGR-1/ligand (GalNac) structure (Item A above) and the result of this combination is depicted in Figure 31B. The structure of the 54E9/ASGR-1 complex (Item I above) was also overlaid on the ASGR-1/ligand (GalNac) structure (Item A above) and the result of this combination is depicted in Figure 35B. The structure of the 218G4/ASGR-1 complex (Item J above) was overlaid on the ASGR- 1/ligand (GalNac) structure (Item A above) and the result of this combination is depicted in Figure 38. The structure of the 176H4/ASGR-1 complex (Item K above) was overlaid on the ASGR-1/ligand (GalNac) structure (Item A above) and the result of this combination is depicted in Figure 40. These figures demonstrate areas on ASGR-1 which can be usefully targeted to inhibit ASGR-1 interaction with a ligand, e.g., GalNac. These figures show that 72G9, 54E9, 218G4 and 176H4 directly interact with a subset of amino acid residues that are specifically involved in binding to the ligand (e.g., GalNAc).

As noted above, analysis of the crystal structures identified specific amino acids involved in the interaction between ASGR-1 and the partner proteins (the core and boundary regions of the interface on the ASGR-1 surface) and the spatial requirements of these partner proteins to interact with ASGR-1. The structures suggest ways to inhibit the interaction between ASGR-1 and a ligand, GalNAc. First, as noted above, binding an agent to ASGR-1 where it shares residues in common with the binding site of a ligand such as GalNAc would inhibit the interaction between ASGR-1 and the ligand. Second, an agent that binds outside of the residues in common can sterically interfere with the ligand that are either N- or C-terminal to the ligand to prevent the interaction between ASGR-1 and a ligand.

The residues that are involved in both ligand binding and are close to the areas where the above noted antigen binding proteins bind may be especially useful for manipulating ASGR-1 binding to ligand. For example, amino acid residues from interfaces in common in both the core region and boundary region for the different binding partners are listed in Table 10.5 below.

Table 10.5

| Parameters | Amino acid position(s) |
|---|---|
| 72G9 /GalNAc both under 5 Å | Q240, D242, W244 |
| 72G9 under 5 Å / GalNAc 5-8 Å | E239, P241, D243, Y245, G246, G252 |
| 72G9 at 5-8 Å / GalNAc under 5 Å | R237, E253 |
| 72G9 / GalNAc both at 5-8 Å | P238, H247, C255, V268 |
| 54E9 / GalNAc both under 5 Å | N209, R237, Q240, D242, H257, T259, N265, D267, Y273 |
| 54E9 under 5 Å / GalNAc 5-8 Å | P238, E239, D260, R263, R271 |
| 54E9 at 5-8 Å / GalNAc under 5 Å | E253, D266 |
| 54E9 / GalNAc both at 5-8 Å | D243, F258, W264 |
| 218G4 / GalNAc both under 5 Å | N209, H257, N265, D267, Y273 |
| 218G4 under 5 Å / GalNAc 5-8 Å | D260, R271 |
| 218G4 at 5-8 Å / GalNAc under 5 Å | R237, T259, D266 |
| 218G4 / GalNAc both at 5-8 Å | F258, V268 |
| 176H4 / GalNAc both under 5 Å | N209, R237, Q240, W244, E253, H257, T259, N265, D267, Y273 |
| 176H4 under 5 Å / GalNAc 5-8 Å | G246, H247, D260, R271 |
| 176H4 at 5-8 Å / GalNAc under 5 Å | D266 |
| 176H4 / GalNAc both at 5-8 Å | P238, E239, Y245, F258, R263, W264, V268 |

As will be appreciated by one of skill in the art, the antigen binding proteins may bind to and/or block at least one of the above noted residues.

Antigen binding proteins and molecules that interact with the relevant areas or residues of the structure of ASGR-1 (including those areas or residues within 15, 15-8, 8, 8-5, 5, or fewer angstroms from where ligands, such as GalNAc, or the antibodies, interact with ASGR-1) depicted in the figures (e.g., Figures 19-42) and/or their corresponding positions on the structures from the coordinates are also contemplated.

## Example 11: Determination of the Binding Affinity of ASGR-1 Specific Antibodies

To quantitate the binding affinity of specific antibodies for ASGR-1 (either purified from hybridoma supernatants or made recombinantly), association and dissociation rates can be measured using a ForteBio Octet instrument. The antibodies were covalently coupled to AR2Gtips to load levels close to 2 nm and then bound to the soluble human ASGR-1 carbohydrate binding domain (CBD; amino acid residues 154-281; N-terminal 6xHis tag) in a 3-fold serial dilution series starting typically at 30 nM with either 3-point or 6-point dilution series. Experimental kinetic results were globally fit to a 1:1 binding model in order to determine the association and dissociation rate constants as well as the equilibrium dissociation constant. Association and dissociation times were chosen to ensure that curvature was present during association curves and measured dissociation levels dropped at least 5% from starting levels. All Octet buffers contained 10 mM Tris (pH7.5), 150 mM NaCl, 1 mM $CaCl_2$, 0.10 mg/ml BSA and 0.13% Triton X-100. Octet assays were run at 27°C. Because this assay only measures binding to the ASGR-1 CBD, antibodies that recognize epitopes partially or entirely outside the CBD and/or recognize ASGR-1 in the context of a native ASGR complex, for example, as could occur on cell membranes, may not score as positive in this assay. Data provided for representative antibodies in TABLE 11.1.

**Table 11.1:**

| Ab name | Octet binding $K_D$ (nM) | Ab name | Octet binding $K_D$ (nM) |
|---|---|---|---|
| 4H6 | 4.8 | 194A4 | 0.7 |
| 4B1 | >30 | 194C1 | 1.3 |
| 4A2 | 0.06 | 194C10 | 4 |
| 5E5 | 7.6 | 197G3 | 0.8 |
| 6G7 | 2.0 | 198D2 | >30 |
| 7G4 | 0.9 | 198G3 | 0.04 |
| 7F4 | 1.2 | 202A3 | >30 |
| 7E11 | 1.6 | 218G4 | 2.6 |
| 12D2 | >30 | 4A2.001 | 0.06 |
| 22G5 | 1.4 | 4A2.001.003 | 0.04 |
| 25A4 | 0.03 | 4A2.001.004 | 0.03 |

| | | | |
|---|---|---|---|
| 26C4 | 0.4 | 4A2.001.005 | 0.02 |
| 29H8 | 1.0 | 4A2.001.010 | 0.04 |
| 48B12 | 0.3 | 4A2.001.012 | 0.04 |
| 54E9 | >30 | 25A4.001 | 0.06 |
| 56E5 | 0.5 | 25A4.001.021 | 0.04 |
| 72G9 | 0.5 | 4H6.009 | 0.28 |
| 75G3 | 1.0 | 7E11.001 | 0.71 |
| 176H4 | 0.8 | 7E11.001.005 | 0.42 |
| 184E7 | 0.3 | 7E11.001.007 | 0.62 |
| 190F8 | 0.6 | 5E5.016 | 1.46 |
| 191G1 | 2.4 | 5E5.019 | 1.80 |
| 191G10 | 0.5 | 5E5.005 | 2.00 |
| 193E7 | 3.5 | | |

**Example 12: CHO-S:huASGR-1 Cell Binding Assay**

CHO-S stable high-expressing cell line were developed for both human ASGR-1 as well as mouse ASGR-1. A typical 384 well plate multiplex flow cytometry-based cell binding method is described as followed: Parental CHO-S cells and CHO-S:huASGR-1 cells were respectively labeled using a CellTrace CFSE Cell Proliferation Kit (ThermoFisher Catalog #C34554) and CellTrace Violet Cell Proliferation Kit (ThermoFisher Catalog#C34557) CHO-S:muASGR-1 were not labeled. 20 ul of cells at 4C were added to duplicate wells of the 384 well plate. The cells were equally mixed from all three cell lines (30K cells/well). Then 20 ul of the ASGR-1 antibodies (either purified from hybridoma supernatants or made recombinantly) were added in an 11-point dose response using a 1:2 fold serial dilution starting at 100 nM.. The cells and antibodies were incubated for 30 min at 4°C and then spun down and washed twice with FACS buffer containing 1mM CaCl2.

30 ul of anti-huIgG-APC secondary antibodies were then added at a 1:1000 dilution ) for 30 min at 4°C and then washed once with the same buffer. 60ul of PI (1:1000) was added and then the cells were read by a core flow cytometry facility. The cells were gated first for live cells, then for single cells and finally for the cell dyes to separate the mixed cells into the three different cell populations. Histograms of signal vs count representing the binding profile of each antibody at each antibody concentration were automatically analyzed for the median of the binding signal and then a binding graph was made with log 10 antibody concentration in nM on the X axis with standard deviation of the median signals from the duplicate wells on the Y-axis. The binding curves were fit with a standard four parameter sigmoidal binding curve and EC50's reported for all graphs with full curves. Data provided for representative antibodies in TABLE 12.1.

**Table 12.1:**

| Ab name | Cell binding EC50 (nM) | Ab name | Cell binding EC50 (nM) |
|---|---|---|---|
| 4H6 | 1.70 | 56E5 | 1.1 |
| 4B1 | 4.1 | 72G9 | 0.41 |
| 4A2 | 0.82 | 75G3 | 1 |
| 4A2.001 | 1.8 | 176H4 | 1 |
| 5E5 | 3.80 | 184E7 | 1 |
| 6G7 | 0.6 | 190F8 | 9 |
| 7G4 | 0.69 | 191G1 | 0.16 |
| 7F4 | 5.40 | 191G10 | 0.31 |
| 7E11 | 1.40 | 193E7 | 0.13 |
| 7E11.001 | 3.2 | 194A4 | 25 |
| 12D2 | 3.2 | 194C1 | 0.11 |
| 22G5 | 7.2 | 194C10 | 0.56 |
| 25A4 | 1.6 | 197G3 | 0.25 |
| 25A4.001 | 1.2 | 198D2 | 0.14 |
| 26C4 | 11 | 198G3 | 0.21 |
| 29H8 | 1.9 | 202A3 | 0.8 |
| 48B12 | 38 | 218G4 | 2.2 |
| 54E9 | 5 | | |

For human ASGR-2, CHO-S stable cells expressing C-terminal His-tagged human ASGR-2 were resuspended in cold flow buffer (10mM Tris, pH 7.5, 137mM NaCl, 1mM CaCl2 and 2%fetal bovine serum) and 1.5×10e6 cells per well were added to a 96-well, v-bottom plate in a volume of 80 ul. 80 ul of antibody at 400 nM was then added to each well. After incubation on ice for 30 min, the cells were centrifuged at 1400 rpm for 3 min and then washed twice in cold flow buffer. The cells were then resuspended in 120 ul of anti-human IgG-APC (diluted 1:1000 in flow buffer) and incubated on ice for 30 minutes, centrifuged and washed twice as before, and resuspended in 200ul cold flow buffer, and then analyzed on a BD-LSR II flow cytometer. Data provided for antibody 7F4 in Figure 43.

**Example 13: CHO-S:huASGR-1 Ligand Blocking Assay**

All ASGR-1 antibodies that bound either human or mouse ASGR-1 stable CHO-S cells were then tested for ligand blocking using both a protein ligand and a synthetic sugar ligand. The method in brief is as follows: first, 20 ul of either CHO-Shuman or mouse ASGR-1 cells were added to wells of a 384 well plate (30k cells/well) followed by spin and discarding the supernatant. Second, 10 ul of the antibodies (either purified from hybridoma supernatants or made recombinantly) were added in duplicate to the cells in a dilution series (200 nM top concentration, 1:2 serial dilution, 11 point curve) and were incubated for 30 min at 4°C. Third, 10 ul of the minimally biotinylated ligands were added at 2x their binding EC05, so that the wells contained a final 20 ul volume with Ab starting at 100 nM and the ligand at their EC50. After 30 min incubation at 4°C, the plate was spun and washed twice with FACS buffer + 1 mM CaCl2 followed by the detection streptavidin-AF647 at 1:1000 dilution. After 30min at 4°C, the cells were spun and washed once and then 60 ul PI added at 1:1000 dilution and the plates delivered to a core flow cytometry facility. The plates were read and processed similarly to the cell binding method except the signal now represents an inhibition curve and typically decreases a function of increasing antibody concentration. IC50 nM potency and % Inhibition were reported. The desialylated, biotinylated asialofetuin (see Example 9A) and biotinylated GALNAc- PAA (Fisher #NC9024754) were used as ligands with measured binding EC50s of 10.7 and 5.4 nM. Differences in the ability of antibodies to block these two ligands could occur as a result of differences in, for example, avidity stemming from differences in the number and/or orientation of the ASGR binding terminal sugar residues of each ligand, steric hindrance between antibody and each ligand, and/or changes in the conformation of ASGR induced by antibody binding that selectively alters the binding of each ligand. Data provided for representative antibodies in TABLE 13.1.

**Table 13.1:**

| | Ligand Blocking | | | |
|---|---|---|---|---|
| | bn-GalNAc-PAA | | bn-asialofetuin | |
| Ab name | IC50 (nM) | % Inhibition | IC50 (nM) | % Inhibition |
| 4H6 | 8.1 | 20% | 12 | 85% |
| 4B1 | 42 | 36% | 64 | 75% |
| 4A2 | 54 | 70% | 11 | 99% |
| 4A2.001 | 28 | 75 | 12 | 99 |
| 5E5 | >200 | 0% | 16 | 95% |
| 6G7 | >200 | 0% | 11 | 99% |
| 7G4 | 20 | -30% | 14 | 96% |
| 7F4 | 0.24 | 30% | 2.6 | 99% |
| 7E11 | 40 | 37% | 13 | 99% |
| 7E11.001 | >100 | 50 | 13 | 99 |
| 12D2 | 2.1 | 10% | 10 | 20% |
| 22G5 | 11 | 93% | 3.4 | 99% |
| 25A4 | 40 | 77% | 11 | 99% |
| 25A4.001 | 31 | 68 | 8.1 | 99 |
| 26C4 | 36 | 83% | 6.6 | 99% |
| 29H8 | 17 | 99% | 7 | 99% |
| 48B12 | 86 | 94% | 19 | 99% |
| 54E9 | 100 | 19% | 50 | 75% |
| 56E5 | 45 | 99% | 23 | 99% |
| 72G9 | 24 | 20% | 53 | 20% |
| 75G3 | 115 | 99% | 29 | 99% |
| 176H4 | 73 | 79% | 59 | 99% |
| 184E7 | 10 | 99% | 23 | 99% |
| 190F8 | 44 | 83% | 34 | 98% |
| 191G1 | 62 | 78% | 24 | 99% |
| 191G10 | 56 | 99% | 27 | 99% |
| 193E7 | 33 | 60% | 30 | 99% |
| 194A4 | 48 | 60% | 57 | 99% |
| 194C1 | 72 | 89% | 34 | 99% |
| 194C10 | 87 | 99% | 30 | 99% |
| 197G3 | 15 | 74% | 29 | 90% |
| 198D2 | 55 | 99% | 22 | 99% |
| 198G3 | 5 | 81% | 26 | 99% |
| 202A3 | 32 | 96% | 16 | 98% |
| 218G4 | 71 | 99% | 28 | 99% |

**Example 14: ASGR-1 Specific Antibody Optimization (Chemical Degradation Site Engineering):**

Variable domain sequence motifs having a high risk of sidechain degradation were engineered out of ASGR-1 specific antibodies. See for example, ASGR-1 specific antibody sequences in Tables 6 and 7.

Certain high risk motifs included: (1) CDR 'NG' and 'NT' sequences prone to asparagine deamidation, (2) CDR 'DG,' 'DH', 'DS,' and 'DT' sequences prone to aspartic acid isomerization, (3) and CDR3 tryptophans prone to oxidation. Bioinformatics and structural analyses were used to identify substitutions likely to retain binding affinity to the ASGR-1 CBD. Typically, substitution identities were derived from germline sequences or from sequence- related ASGR-1 CBD-binding mAbs. These substitutions were then modeled into a homology model of the unbound mAb using the software MOE (CCG)[1] to predict structural fitness. For cases in which the bioinformatics or structural analyses did not provide a clear substitution identity, residue types chemically similar to the parent residue were identified.

Variable domain sequence motifs violating multiple sequence alignment-based pair-wise residue covariance trends[2] were also engineered out of ASGR-1 specific antibodies. Substitution identities for covariance violators were identified using a hybrid bioinformatics/structural approach similar to that used to remediate degradation sites.

1. Molecular Operating Environment (MOE), 2013.08; Chemical Computing Group, Inc., 1010 Sherbooke St. West, Suit #910, Montreal, QC, Canada, H3A 2R7, 2016.
2. Kannan, G. Method of Correlated Mutational Analysis to Improve Therapeutic Antibodies. US Patent Application PCT/US2012/028596 filed March 9, 2012.

**Example 15: Epitope Mapping Using Peptide Arrays**

Custom peptide microarrays were obtained commercially (PEPperPRINT GmbH). For epitope mapping using linear arrays, the antigen (ASGR-1) was translated into 291 different overlapping 15 amino acid (aa) peptides printed in duplicate (582 peptide spots per array copy). For epitope mapping using cyclized arrays, the antigen (ASGR-1) was translated into 888 different overlapping 7 aa, 10 aa and 13 aa peptides printed in duplicate (1,776 peptide spots per array copy). Peptide cyclization was accomplished using N- to C-terminal thioether formation with and without an additional scaffold for varying conformational restriction. Each PEPperCHIP® Peptide array is framed by Flag (DYKDDDDKAS) and HA (YPYDVPDYAG) control peptides. Assay buffer was PBS-T (PBS, pH7.4, 0.05% Tween 20), blocking buffer was Rockland Blocking Buffer (Rockland Immunochemicals), staining buffer was assay buffer + 10% Rockland Blocking buffer. Secondary antibody was goat anti-human IRDye680LT (Li-Cor). Control antibodies were anti-FLAG M2 DyLight800, anti-HA DyLight680. Arrays were scanned on Li-Cor Odyssey with an offset of 0.65mm, 21 um resolution.

Array staining and detection was per manufacturer's instructions. Briefly, arrays were pre-stained with secondary antibodies for 30 minutes, washed and scanned to detect background binding. Arrays were then stained with commercially available primary antibodies overnight, followed by washing and 30 minute incubation with labeled secondary antibodies. Arrays were scanned to detect binding of anti-ASGR-1 antibodies. Finally, arrays were stained with control antibodies for 45 minutes prior to washing and scanning to detect control peptides.

Antigen binding proteins with desired binding properties can be identified using this assay.

Example 16: ***In vivo* studies**

RNAi constructs that reduce expression of ASGR-1 and/or ASGR-2 and/or antigen binding proteins, such as monoclonal antibodies, that inhibit ligand binding to ASGR, ASGR-1, and/or ASGR-2 *in vitro* can be administered *in vivo* to a relevant animal model and levels and/or activity of endogenous blood proteins like alkaline phosphatase measured. In addition, the clearance of exogenously administered ASGR ligands (for example asialoglycoproteins, certain non-asialylated proteins, synthetic ligands, etc.) can be inhibited by pre-treatment with RNAi or a co- or pre- administered antibody.

Additionally, physiologic effects of the antigen binding proteins or RNAi can be evaluated in relevant animal models of cardiovascular disease using readouts including blood pressure, primary and secondary hemostasis, heart function and morphology, endothelial function, LDL cholesterol levels, non-HDL cholesterol levels, inflammation, and atherosclerosis.

**Example 17: Effect of ASGR-1 antibody 4A2 on serum LDL cholesterol and alkaline phosphatase in normal and obese cynomolgus monkeys**

The purpose of the study was to evaluate the LDL cholesterol (LDL-C)-lowering activity of anti-ASGR inhibitors. In general, cynomolgus monkeys do not have high levels of total cholesterol, HDL-C or LDL-C. Therefore, both normal and dyslipidemic models were utilized in this example. In the dyslipidemic model, monkeys were selected if their LDL levels were at least 100mg/dL (normal is 40-60mg/dL), and if there body mass index was over 41 kg/$m^2$ (normal is below 35 kg/$m^2$). Animals that met these criteria on standard diet were classified as spontaneously obese dyslipidemic. Other animals were fed a high-fat diet (HFD; 4.15 kcal/gm, 32% fat) prior to inclusion in the study and were classified as HFD obese dyslipidemic.

Naive male spontaneous obese dyslipidemic and HFD obese dyslipidemic cynomolgous monkeys were given a single subcutaneous injection of anti-ASGR-1 antibody 4A2.001 (IgG1z-SEFL2) (10 mg/kg in 10 mM sodium acetate, 9% sucrose, 0.01% polysorbate-80, pH 5.2). Naive male and female normal cynomolgus monkeys were given a single intravenous injection of anti- ASGR-1 antibody 4A2.001 (IgG1z-SEFL2)(100 mg/kg in 10 mM sodium acetate, 9% sucrose, 0.01% polysorbate-80, pH 5.2). Blood was collected from overnight fasted animals to monitor LDL-C and alkaline phosphatase (ALP) levels post-antibody injection. Blood was collected 70, 118, 190 and 268 hours post-injection (dyslipidemic models) and at 0.05, 0.25, 0.5, 1, 4, 8, 24, 48, 72, 168, 240, 336, 504, 672, 840, 1008, and 1176 hours post-injection (normal). LDL-C decrease (%) and ALP increase (%) were the main endpoints of the study and were measured on Roche C311 and C501 chemistry analyzers. Baseline levels of LDL-C and ALP were established from blood collected 7 days prior to antibody administration.

Dyslipidemic model:

| | |
|---|---|
| Species: | *Macaca fascicularis* |
| Weight Range: | >7.0 kg |
| BMI Range: | >41 kg/$m^2$ |
| Age range: | 12-17 years |
| Time on HFD: | 6 months |
| Source: | KBI monkey colony |
| Number and Sex: | 3 male spontaneous obese monkeys and 3 male HFD induced obese monkeys (BMI>41, LDL>80 mg/dL)). Animals were selected from a larger pool based on similar baseline LDL and ALP levels |

Normal model:

| | |
|---|---|
| Species: | *Macaca fascicularis* |
| Weight Range: | 2.6-4.2 kg |
| Age range: | 2.5-4 years |
| Number and Sex: | 2 male and 1 female fed normal laboratory diet |

Data for this study is provided in Figure 44 (dyslipidemic model) and Figure 45 (normal model).

**Example 18: Proteomic Profiling of Serum Samples from Human ASGR1 Carriers and Controls**

Introduction

As described above in Example 1, ASGR1 loss-of-function (LOF) was found to be associated with a beneficial phenotype (protected from coronary artery disease, lower LDL cholesterol and longer life span) in human. [1]. To understand the mechanism of action underlying this association and find potential biomarkers, proteomic measurement of human serum samples were performed and compared to changes in circulating protein levels between the ASGR1 LOF variant carriers and controls.

**Materials and methods**

Sample collection and Proteomic Profiling

A total of 333 human serum samples were acquired from the deCODE Icelandic population study, including 100 ASGR1 del12 heterozygous carriers (cases group) and 233 non- carriers (controls group). The Case/Control Groups are well matched by sex, age and collection time/ freezer storage time. 150ul serum samples were shipped to SomaLogic Inc, where 1310 proteins were measured by the SOMAscan Assay 1.3k. The 1310 proteins were SOMAmer® Reagents Generated to Human Proteins, the complete list of tested proteins are summarized in the SOMAscan Assay 1.3K Content, Rev 1 (Effective: 9/21/2015).

The SOMAscan assay measured serum protein concentration using a Slow Off-rate Modified DNA Aptamer (SOMAmer)-based capture array[2]. Each of the 1310 proteins is bond by its respective fluorescently labeled SOMAmer in the assay and their concentrations are reflected by the respective SOMAmer's relative fluorescence units (RFU).

## Data analysis

2 Samples were removed due to low volume that did not meet Somascan requirements and 13 samples were removed because they had been treated with EDTA. The RFU data of each measured protein was log transformed, then centered and scaled to calculate standardized RFU values for this protein. Principle components (PCs) were derived from 1310 standardized RFU values by principle components analysis. An outlier removal based on Hotellings T2 distribution of PC1 and PC2 was applied and excluded another 8 samples from further analysis.

After QC, the remaining 93 ASGR1 Del12 heterozygous Carriers (cases group) and 217 samples without the Del12 allele (controls group) and their standardized RFU values of each protein were analyzed by a linear model adjusting for Age, Sex, FreezerTime and the first 10 PCs,

$$Yi = \beta 0 + \beta 1Gi + \beta 2AGEi + \beta 3SEXi + \beta 4FTi + \beta 5PC1i + \cdots + \beta 15PC10i + \varepsilon i$$

where *Yi* is the standardized RFU value for the *i th* sample for a particular protein, *Gi* is the Del12 genotype the *i th* sample and $\beta 1$ capture the estimates of the mean difference between human samples with Del12 and without Del12. Since 1310 tests were performed for the proteins on Somascan platform, we calculated the significant threshold by Bonferroni method ($0.05/1310=3.82\times10^{-5}$) assuming these are independent tests. However, the Bonferroni correction is likely too stringent because proteins are often correlated with each other therefore these tests are not independent. Thus a realistic threshold of significance ($5.19\times10^{-5}$) was obtained by performing 100,000 permutations using the method by Sham and Purcell 2014[3].

## Results and Discussion

Using the permutation threshold, 41 Proteins were identified to have significant serum levels between human ASGR1 del12 carriers and non-carriers ($P<5.19\times10^{-5}$). Of those, 26 show significant increase in the carriers (Table 18.1) and 15 decrease significantly in the carriers (Table 18.2). These changes are likely to mediate the beneficial effects resulting from ASGR1 loss of function seen in the del12 carriers. The levels of these proteins in blood can serve as biomarkers for ASGR1 loss of function and be used to assess ASGR1-targeted therapy during drug development.

**Table 18.1. Proteins with significant increase in serum of ASGR1 del12 carriers.**

| p value | Estimate (SD) | Gene | Full Name |
|---|---|---|---|
| 3.71E-54 | 1.34 | TNFSF8 | Tumor necrosis factor ligand superfamily member 8 |
| 1.33E-52 | 1.45 | CD 163 | Scavenger receptor cysteine-rich type 1 protein M130 |
| 2.07E-25 | 1.09 | CSFIR | Macrophage colony-stimulating factor 1 receptor |
| 1.44E-24 | 1.16 | LYVE1 | Lymphatic vessel endothelial hyaluronic acid receptor 1 |
| 1.03E-22 | 0.65 | IL6ST | Interleukin-6 receptor subunit beta |
| 4.56E-15 | 0.67 | IL18BP | Interleukin-18-binding protein |
| 1.16E-12 | 0.74 | CD300C | CMRF35-like molecule 6 |
| 2.47E-12 | 0.59 | TYRO3 | Tyrosine-protein kinase receptor TYRO3 |
| 8.85E-12 | 0.80 | LRP8 | Low-density lipoprotein receptor-related protein 8 |
| 1.76E-09 | 0.66 | IL1RL1 | Interleukin-1 receptor-like 1 |
| 2.62E-09 | 0.61 | ISLR2 | Immunoglobulin superfamily containing leucine-rich repeat protein 2 |
| 4.01E-09 | 0.55 | SIGLEC7 | Sialic acid-binding Ig-like lectin 7 |

(continued)

| p value | Estimate (SD) | Gene | Full Name |
|---|---|---|---|
| 4.47E-09 | 0.48 | NRXN3 | Neurexin-3-beta |
| 1.03E-07 | 0.58 | PLAU | Urokinase-type plasminogen activator |
| 2.96E-07 | 0.37 | CD55 | Complement decay-accelerating factor |
| 8.27E-07 | 0.53 | CD48 | CD48 antigen |
| 1.22E-06 | 0.31 | TNFRSF21 | Tumor necrosis factor receptor superfamily member 21 |
| 1.62E-06 | 0.36 | MRC2 | C-type mannose receptor 2 |
| 3.82E-06 | 0.57 | KLK13 | Kallikrein-13 |
| 4.95E-06 | 0.33 | IGFIR | Insulin-like growth factor 1 receptor |
| 1.46E-05 | 0.45 | ANGPT2 | Angiopoietin-2 |
| 2.02E-05 | 0.39 | CNTN4 | Contactin-4 |
| 2.57E-05 | 0.47 | FCGR3B | Low affinity immunoglobulin gamma Fc region receptor III-B |
| 2.93E-05 | 0.38 | C1S | Complement C1s subcomponent |
| 3.92E-05 | 0.40 | LY9 | T-lymphocyte surface antigen Ly-9 |
| 4.48E-05 | 0.46 | CD200R1 | Cell surface glycoprotein CD200 receptor 1 |

**Table 18.2. Proteins with significant decrease in serum of ASGR1 del12 carriers.**

| p value | Estimate (SD) | Gene | Target Full Name |
|---|---|---|---|
| 1.08E-09 | -0.52 | CD93 | Complement component C1q receptor |
| 6.32E-09 | -0.50 | IDS | Iduronate 2-sulfatase |
| 1.56E-07 | -0.34 | RGMB | RGM domain family member B |
| 2.91E-07 | -0.44 | TGFBI | Transforming growth factor-beta-induced protein ig- h3 |
| 5.56E-07 | -0.48 | LUM | Lumican |
| 6.67E-07 | -0.46 | MMP2 | 72 kDa type IV collagenase |
| 1.36E-06 | -0.38 | FLRT2 | Leucine-rich repeat transmembrane protein FLRT2 |
| 2.18E-06 | -0.48 | AHSG | Alpha-2-HS-glycoprotein |
| 2.44E-06 | -0.37 | CSH1 CSH2 | Chorionic somatomammotropin hormone |
| 3. 16E-06 | -0.54 | ESM1 | Endothelial cell-specific molecule 1 |
| 1.36E-05 | -0.52 | AFM | Afamin |
| 1.67E-05 | -0.48 | TNFRSF17 | Tumor necrosis factor receptor superfamily member 17 |
| 2.68E-05 | -0.46 | OMD | Osteomodulin |
| 4.69E-05 | -0.23 | GDI2 | Rab GDP dissociation inhibitor beta |
| 5.09E-05 | -0.45 | SPOCK2 | Testican-2 |

**References**

1 See also, Nioi, P. et al. Variant ASGR1 Associated with a Reduced Risk of Coronary Artery Disease. The New England journal of medicine 374, 2131-2141, doi:10.1056/NEJMoa1508419 (2016).

2 Gold, L. et al. Aptamer-based multiplexed proteomic technology for biomarker discovery. PLoS One 5, e15004, doi:10.1371/journal.pone.0015004 (2010).
3 Sham, P. C. & Purcell, S. M. Statistical power and significance testing in large-scale genetic studies. Nature reviews. Genetics 15, 335-346, doi:10.1038/nrg3706 (2014).

## Example 19: Proteomic Profiling of Serum Samples from ASGR1 Cyno PK-PD study

Introduction

As decribed above in Example 1, ASGR1 loss-of-function (LOF) was found to be associated with a beneficial phenotype (protected from coronary artery disease, lower LDL cholesterol and longer life span) in human[1]. Certain ASGR-1 antigen binding proteins disclosed herein were found to mimic the LOF effects, and can be useful in the treatment of coronary artery disease. In brief, cynomolgus monkeys were treated with certain ASGR-1 specific, ligand blocking antibodies in order to study the PK-PD profile of these antibodies. Moreover, a dose-dependent elevation of alkaline phosphatase (ALP) levels was observed in the Ab-treated cynos, which resembles the ALP elevation seen in human ASGR1 LOF carriers. In addition to ALP, proteomic profiling in human serum identified 41 proteins that potentially underlie the beneficial effects caused by ASGR1 LOF as described above in Example 18. To compare effects of anti-ASGR1 antibody treatment with the human ASGR1 LOF and identify comparable signatures in cynomolgus monkey, proteomic measurement of the serum samples from this study was conducted. The list of proteins with altered levels in the antibody-treated animals is compared to the ones identified in human LOF carriers.

### Materials and methods

Sample selection and Proteomic Profiling

6 animal groups with 3 animals in each group were selected for proteomic profiling. The 6 groups include 5 antibody-treated groups (mAb1/25A4, mAb2/4A2, mAb3/7E11, mAb4/SES and mAb8/4H6) and a vehicle control group (mAb6). The animals were dosed once at 100mg/kg. Serum samples from time points 0, 168, 336, 504, 672 and 1176 hours were collected for each animal (Table 19.1 & 19.2). The only exception is group mAb8/4H6, where time point 1008 hour is used instead of 1176 hour. 120ul serum samples were shipped to SomaLogic Inc, where 1310 proteins (see table 18.0) were measured by the SOMAscan Assay 1.3k.

The SOMAscan assay measures serum protein concentration using a Slow Off-rate Modified DNA Aptamer (SOMAmer)-based capture array[2]. Each of the 1310 proteins is bond by its respective fluorescently labeled SOMAmer in the assay and their concentrations are reflected by the respective SOMAmer's relative fluorescence units (RFU).

**Table 19.1. Serum sample selection.**

| | | Time points | | | | | |
|---|---|---|---|---|---|---|---|
| Animal group | Animal Number | D0 | D8 | D15 | D22 | D29 | D50 |
| | | 0 hr | 168 hr | 336 hr | 504 hr | 672 hr | 1176 hr |
| 25A4 | 701, 702, 703 | √ | √ | √ | √ | √ | √ |
| 4A2 | 704, 705, 706 | √ | √ | √ | √ | √ | √ |
| 7E11 | 707,708,711 | √ | √ | √ | √ | √ | √ |
| 5E5 | 709,710,712 | √ | √ | √ | √ | √ | √ |
| SEFL2-control | 716,717,718 | √ | √ | √ | √ | √ | √ |
| 4H6 | 204, 205, 206 | √ | √ | √ | √ | √ | √* |

* 4H6 was collected at D43 (1008 hr).

**Table 19.2. List of all sample groups by treatment and time points.**

| Sample group | Treatment (*e.g.*, drug, vehicle, *etc.*) | Time point | # of Samples in Group | Subject ID |
|---|---|---|---|---|
| 25A4_D0 | mAb1 | 0 Hr | 3 | 701, 702, 703 |
| 25A4_D8 | mAb1 | 168 Hr | 3 | 701, 702, 703 |
| 25A4_D15 | mAb1 | 336 Hr | 3 | 701, 702, 703 |
| 25A4_D22 | mAb1 | 504 Hr | 3 | 701, 702, 703 |
| 25A4_D29 | mAb1 | 672 Hr | 3 | 701, 702, 703 |
| 25A4_D50 | mAb1 | 1176 Hr | 3 | 701, 702, 703 |
| 4A2_D0 | mAb2 | 0 Hr | 3 | 704, 705, 706 |
| 4A2_D8 | mAb2 | 168 Hr | 3 | 704, 705, 706 |
| 4A2_D15 | mAb2 | 336 Hr | 3 | 704, 705, 706 |
| 4A2_D22 | mAb2 | 504 Hr | 3 | 704, 705, 706 |
| 4A2_D29 | mAb2 | 672 Hr | 3 | 704, 705, 706 |
| 4A2_D50 | mAb2 | 1176 Hr | 3 | 704, 705, 706 |
| 7E11_D0 | mAb3 | 0 Hr | 3 | 707, 708, 711 |
| 7E11_D8 | mAb3 | 168 Hr | 3 | 707, 708, 711 |
| 7E11_D15 | mAb3 | 336 Hr | 3 | 707, 708, 711 |
| 7E11_D22 | mAb3 | 504 Hr | 3 | 707, 708, 711 |
| 7E11_D29 | mAb3 | 672 Hr | 3 | 707, 708, 711 |
| 7E11_D50 | mAb3 | 1176 Hr | 3 | 707, 708, 711 |

(continued)

| Sample group | Treatment (*e.g.*, drug, vehicle, *etc.*) | Time point | # of Samples in Group | Subject ID |
|---|---|---|---|---|
| 5E5_D0 | mAb4 | 0 Hr | 3 | 709, 710, 712 |
| 5E5_D8 | mAb4 | 168 Hr | 3 | 709, 710, 712 |
| 5E5_D15 | mAb4 | 336 Hr | 3 | 709, 710, 712 |
| 5E5_D22 | mAb4 | 504 Hr | 3 | 709, 710, 712 |
| 5E5_D29 | mAb4 | 672 Hr | 3 | 709, 710, 712 |
| 5E5_D50 | mAb4 | 1176 Hr | 3 | 709, 710, 712 |
| CTL_D0 | mAb6 | 0 Hr | 3 | 716, 717, 718 |
| CTL_D8 | mAb6 | 168 Hr | 3 | 716, 717, 718 |
| CTL_D15 | mAb6 | 336 Hr | 3 | 716, 717, 718 |
| CTL_D22 | mAb6 | 504 Hr | 3 | 716, 717, 718 |
| CTL_D29 | mAb6 | 672 Hr | 3 | 716, 717, 718 |
| CTL_D50 | mAb6 | 1176 Hr | 3 | 716, 717, 718 |
| 4H6_D0 | mAb8 | 0 Hr | 3 | 204, 205, 206 |
| 4H6_D8 | mAb8 | 168 Hr | 3 | 204, 205, 206 |
| 4H6_D15 | mAb8 | 336 Hr | 3 | 204, 205, 206 |
| 4H6_D22 | mAb8 | 504 Hr | 3 | 204, 205, 206 |
| 4H6_D29 | mAb8 | 672 Hr | 3 | 204, 205, 206 |
| 4H6_D43 | mAb8 | 1008 Hr | 3 | 204, 205, 206 |

Data analysis

As the SOMAscan assay was developed for humans, some proteins in cynomolgus monkey may not be recognized by the SOMAmer reagents. As a result, SOMAscan measurements of these proteins would have low credibility and may not reflect the true protein levels. A simple criterion was defined to determine the credibility of the measurements, assuming the serum levels of a given protein are in relatively close range in human and cynomolgus monkey. The mean and range of each protein level in human are calculated based on the 217 human control samples from the human proteomic study described in Example 18. The mean and range of each protein level in cynomolgus monkey are calculated based on a total of 48 samples including measurements of all time points for the SEFL-2 control group and the pre-treatment (D0) and washout period (D50) measurements of all the other groups. A protein measurement would be assigned low credibility if (1) its range in cynomolgus monkey is not overlapping with human; and (2) there is a 5 fold difference between the mean level of this protein in human and cynomolgus monkey. A total of 162 proteins were determined as low-credibility by these criteria and were excluded (Figure 58, which depicts a summary of the credibility of protein measurements in cynomolgus monkey). In FIG. 58, log10 RFU of mean protein levels in the two species are plotted and the ones with low credibility (light shading) and high credibility (black) are marked.

One sample in the 4H6 group was removed due to low volume that did not meet the requirements for the SOMAscan assay. No outliers were found in the principle components analysis. A linear mixed model adjusting for potential confounding factors was used to test whether the ASGR1 antibody treatment changes each protein level differently from the control group over time points,

$$\boldsymbol{Y_{ti} = \beta_0 + \beta_1 \mathrm{TREATGROUP}_i + \beta_2 \mathrm{TIME}_{ti} + \beta_3 (\mathrm{TREATGROUP}_i)(\mathrm{TIME}_{ti}) + \beta_4 COV_{ti} + \cdots + \beta_{m+4} COV_{ti} + b_{0i} + \varepsilon_{ti}}$$

which is determined by the p-value for $\beta_3$ (i.e., treatment by time interaction; mean difference in slopes between treatment conditions). The random effect $b_{01}$ captures individual animal heterogeneity. The TREATGROUP is coded as (25A4=4A2=7E11=SES=4H6=1; SELF-2=0) and TIME is coded as (D8=D15=D22=D29=1; D0=D50=0) to test for the ASGR1 antibodies effect after treatment comparing to pre-treatment and washout period. Since multiple tests were performed for the proteins on SOMAscan platform, a Bonferroni corrected significant threshold ($5\times10^{-5}$) was used.

## Results and discussion

33 proteins were identified to have significant serum level changes after ASGR1 antibody treatment (Table 19.3; $P<5\times10^{-5}$). Interestingly, all the 33 proteins show increased levels (1.36-10.18 fold) after ASGR1 antibody treatment.

**Table 19.3. Proteins with significant changes after ASGR1 antibody treatment in Cynomolgus monkey.**

| P-value | Estimated Fold Change | Gene | Full Name |
|---|---|---|---|
| 1.87E-13 | 10.18 | TNFSF8 | Tumor necrosis factor ligand superfamily member 8 |
| 1.01E-06 | 8.56 | ASGR1 | Asialoglycoprotein receptor 1 |
| 1.35E-10 | 3.93 | ADGRE2 | Adhesion G protein-coupled receptor E2 |
| 2.74E-11 | 2.86 | CD86 | T-lymphocyte activation antigen CD86 |
| 1.46E-11 | 2.81 | TNFRSF21 | Tumor necrosis factor receptor superfamily member 21 |
| 7.48E-10 | 2.57 | LICAM | Neural cell adhesion molecule L1 |
| 6.09E-12 | 2.42 | PLXNC1 | Plexin-C1 |
| 1.22E-07 | 2.11 | MRC2 | C-type mannose receptor 2 |
| 1.18E-06 | 2.10 | AMIGO2 | Amphoterin-induced protein 2 |
| 2.28E-11 | 2.02 | ANGPT2 | Angiopoietin-2 |
| 6.68E-09 | 1.99 | INSR | Insulin receptor |
| 1.02E-10 | 1.93 | IL17RA | Interleukin-17 receptor A |
| 7.12E-12 | 1.90 | NRXN3 | Neurexin-3-beta |
| 5.95E-06 | 1.85 | GPNMB | Transmembrane glycoprotein NMB |
| 2.03E-06 | 1.74 | IGFIR | Insulin-like growth factor 1 receptor |
| 3.91E-09 | 1.73 | PLAUR | Urokinase plasminogen activator surface receptor |
| 3.58E-09 | 1.69 | FGFR1 | Fibroblast growth factor receptor 1 |
| 1.26E-06 | 1.60 | LRP8 | Low-density lipoprotein receptor-related protein 8 |
| 3.87E-09 | 1.55 | LYPD3 | Ly6/PLAUR domain-containing protein 3 |
| 3.17E-06 | 1.55 | GRN | Granulins |
| 4.27E-05 | 1.54 | CNTN4 | Contactin-4 |
| 4.59E-07 | 1.54 | KDR | Vascular endothelial growth factor receptor 2 |
| 4.99E-06 | 1.53 | IL12RB2 | Interleukin-12 receptor subunit beta-2 |
| 5.85E-06 | 1.52 | ROBO3 | Roundabout homolog 3 |
| 1.44E-06 | 1.50 | ALCAM | CD166 antigen |
| 3.83E-05 | 1.46 | TYROS | Tyrosine-protein kinase receptor TYROS |
| 3.09E-05 | 1.45 | CADM1 | Cell adhesion molecule 1 |
| 1.53E-08 | 1.44 | JAG1 | Protein jagged-1 |
| 2.58E-09 | 1.43 | ISLR2 | Immunoglobulin superfamily containing leucine- rich repeat protein 2 |
| 3.11E-OS | 1.39 | SET | Protein SET |

(continued)

| P-value | Estimated Fold Change | Gene | Full Name |
|---|---|---|---|
| 4.64E-05 | 1.38 | IL20RA | Interleukin-20 receptor subunit alpha |
| 2.15E-06 | 1.36 | KLRK1 | NKG2-D type II integral membrane protein |
| 2.39E-05 | 1.36 | GFRA2 | GDNF family receptor alpha-2 |

To compare results from this study with the human proteomic study, a list of proteins made by the 33 proteins in Table 19.3 and the top 41 proteins identified in human was compiled. This results in a list of 64 proteins total. The estimates of protein level change and p-value of the changes in the studies were compared (Table 19.4). Based on concordance of change in the cyno (in response to ASGR1 antibody treatment) and human (in response to ASGR1 LOF) studies, the proteins are classified into 5 tiers. Tier 1 includes 10 proteins that pass the stringent Bonferroni corrected significance level ($p<5\times10^{-5}$) in both studies with the same direction of changes. The number of proteins supported by strong evidence in both studies are much higher than the number one would expect by chance ($p=1.58\times10^{-8}$; Fisher's exact test). It indicates that ASGR1 Ab treatment can induce a serum protein levels change in cyno that is similar to the effect of del12 LOF variant in Human. Therefore, these proteins are the core biomarkers. For example, the strongest biomarker TNFSF8 had more than 10 fold increase after ASGR1 Antibody treatment (Figures 59A-59D, which depict the results of serum protein levels of TNFSF8 in cyno and human studies).

Tier 2 contains 12 proteins with strong evidence ($p<5\times10^{-5}$) in the cyno study and suggestive evidence ($p<0.05$) in human with the same direction of changes. Both Tier 1 and 2 proteins have increased levels in both studies. Tier 3 includes 11 proteins that are found significant only in the cyno study but not human. These proteins are likely to be biomarkers specific for the drug modality or for cynomolgus monkeys. For example, the soluble secreted form of ASGR1 increased more than 10 fold after antibody treatment but no significant difference was observed in human between the ASGR1 del12 carriers and non-carriers. Tier 4 contains 17 proteins with significant evidence ($p<5\times10^{-5}$) in the human study but not supported by the cyno study. Majority of the proteins in Tier 4 has decrease levels in human del12 carriers. This observation may indicate a difference between antibody treatment and constitutive gene LOF. It could also possibly be due to species difference or simply caused by lower statistical power in the cyno study.

Lastly, there are 14 proteins with significant changes in human classified as Tier 5 because they were excluded in the cyno study due to the low credibility of their SOMAmer reagents.

In summary, the two studies show high degree of concordance between the antibody treatment in cynomolgus monkey and ASGR1 LOF in humans, with 10 proteins (Tier 1) showing very significant changes in the same direction in both studies. The ASGR-1 antibody treatment is working well as a way of mimicking the effects of ASGR1 LOF in humans and can be useful in the treatment of coronary artery disease.

**Table 19.4. Five tiers of protein biomarkers and comparison of the estimates of protein level change and p-value between the two studies.**

| Target Full Name | Gene | human | | Cyno | | Tier |
|---|---|---|---|---|---|---|
| | | Estimate (SD) | P-value | Estimate log2FC | P-value | |
| Tumor necrosis factor ligand superfamily member 8 | TNFSF8 | 1.34 | 3.7E-54 | 3.35 | 1.87E-13 | 1 |
| Tumor necrosis factor receptor superfamily member 21 | TNFRSF21 | 0.31 | 1.2E-06 | 1.49 | 1.46E-11 | 1 |
| C-type mannose receptor 2 | MRC2 | 0.36 | 1.6E-06 | 1.08 | 1.22E-07 | 1 |
| Angiopoietin-2 | ANGPT2 | 0.45 | 1.5E-05 | 1.01 | 2.28E-11 | 1 |
| Neurexin-3-beta | NRXN3 | 0.48 | 4.5E-09 | 0.93 | 7.12E-12 | 1 |
| Insulin-like growth factor 1 receptor | IGF1R | 0.33 | 5.0E-06 | 0.80 | 2.03E-06 | 1 |

(continued)

| Target Full Name | Gene | human | | Cyno | | Tier |
|---|---|---|---|---|---|---|
| | | Estimate (SD) | P-value | Estimate log2FC | P-value | |
| Low-density lipoprotein receptor-related protein 8 | LRP8 | 0.80 | 8.9E-12 | 0.68 | 1.26E-06 | 1 |
| Contactin-4 | CNTN4 | 0.39 | 2.0E-05 | 0.63 | 4.27E-05 | 1 |
| Tyrosine-protein kinase receptor TYR03 | TYR03 | 0.59 | 2.5E-12 | 0.55 | 3.83E-05 | 1 |
| Immunoglobulin superfamily containing leucine- rich repeat protein 2 | ISLR2 | 0.61 | 2.6E-09 | 0.52 | 2.58E-09 | 1 |
| T-lymphocyte activation antigen CD86 | CD86 | 0.39 | 2.1E-03 | 1.52 | 2.74E-11 | 2 |
| Neural cell adhesion molecule Ll | LICAM | 0.30 | 5.5E-03 | 1.36 | 7.48E-10 | 2 |
| Plexin-Cl | PLXNC1 | 0.40 | 1.0E-04 | 1.28 | 6.09E-12 | 2 |
| Amphoterin-induced protein 2 | AMIGO2 | 0.44 | 1.9E-04 | 1.07 | 1.18E-06 | 2 |
| Interleukin-17 receptor A | IL17RA | 0.29 | 0.03 | 0.95 | 1.02E-10 | 2 |
| Urokinase plasminogen activator surface receptor | PLAUR | 0.35 | 3.3E-04 | 0.79 | 3.91E-09 | 2 |
| Fibroblast growth factor receptor 1 | FGFR1 | 0.30 | 2.3E-03 | 0.75 | 3.58E-09 | 2 |
| Granulins | GRN | 0.27 | 5.7E-03 | 0.63 | 3.17E-06 | 2 |
| CD166 antigen | ALCAM | 0.20 | 9.1E-03 | 0.58 | 1.44E-06 | 2 |
| Protein jagged-1 | JAG1 | 0.17 | 0.01 | 0.53 | 1.53E-08 | 2 |
| Protein SET | SET | 0.28 | 2.1E-03 | 0.47 | 3.11E-05 | 2 |
| GDNF family receptor alpha-2 | GFRA2 | 0.39 | 9.2E-05 | 0.44 | 2.39E-05 | 2 |
| Asialoglycoprotein receptor 1 | ASGR1 | 0.00 | 0.99 | 3.10 | 1.01E-06 | 3 |
| Adhesion G protein-coupled receptor E2 | ADGRE2 | 0.04 | 0.70 | 1.97 | 1.35E-10 | 3 |
| Insulin receptor | INSR | 0.20 | 0.06 | 1.00 | 6.68E-09 | 3 |
| Transmembrane glycoprotein NMB | GPNMB | -0.22 | 0.01 | 0.89 | 5.95E-06 | 3 |
| Ly6/PLAUR domain-containing protein 3 | LYPD3 | -0.06 | 0.26 | 0.63 | 3.87E-09 | 3 |
| Vascular endothelial growth factor receptor 2 | KDR | 0.19 | 0.09 | 0.63 | 4.59E-07 | 3 |
| Interleukin-12 receptor subunit beta-2 | IL12RB2 | 0.11 | 0.38 | 0.61 | 4.99E-06 | 3 |
| Roundabout homolog 3 | ROBO3 | 0.08 | 0.55 | 0.61 | 5.85E-06 | 3 |

(continued)

| Target Full Name | Gene | human | | Cyno | | Tier |
|---|---|---|---|---|---|---|
| | | Estimate (SD) | P-value | Estimate log2FC | P-value | |
| Cell adhesion molecule 1 | CADM1 | -0.17 | 0.02 | 0.53 | 3.09E-05 | 3 |
| Interleukin-20 receptor subunit alpha | IL20RA | 0.05 | 0.69 | 0.47 | 4.64E-05 | 3 |
| NKG2-D type II integral membrane protein | KLRK1 | -0.14 | 0.25 | 0.44 | 2.15E-06 | 3 |
| Lymphatic vessel endothelial hyaluronic acid receptor 1 | LYVE1 | 1.16 | 1.44E-24 | 0.00 | 0.96 | 4 |
| CMRF35-like molecule 6 | CD300C | 0.74 | 1.16E-12 | 0.03 | 0.39 | 4 |
| Interleukin-1 receptor-like 1 | IL1RL1 | 0.66 | 1.76E-09 | 0.75 | 0.10 | 4 |
| Kallikrein-13 | KLK13 | 0.57 | 3.82E-06 | 0.08 | 0.42 | 4 |
| CD48 antigen | CD48 | 0.53 | 8.27E-07 | -0.06 | 0.81 | 4 |
| Rab GDP dissociation inhibitor beta | GDI2 | -0.23 | 4.69E-05 | -0.27 | 0.09 | 4 |
| Chorionic somatomammotropin hormone | CSH1 CSH2 | -0.37 | 2.44E-06 | 0.19 | 9.4E-03 | 4 |
| Leucine-rich repeat transmembrane protein FLRT2 | FLRT2 | -0.38 | 1.36E-06 | 0.30 | 0.03 | 4 |
| Transforming growth factor-beta-induced protein ig-h3 | TGFBI | -0.44 | 2.91E-07 | 0.13 | 0.46 | 4 |
| Testican-2 | SPOCK2 | -0.45 | 5.09E-05 | 0.10 | 0.96 | 4 |
| 72 kDa type IV collagenase | MMP2 | -0.46 | 6.67E-07 | 0.13 | 0.48 | 4 |
| Osteomodulin | OMD | -0.46 | 2.68E-05 | 0.12 | 0.78 | 4 |
| Alpha-2 HS -glycoprotein | AHSG | -0.48 | 2.18E-06 | 0.00 | 0.97 | 4 |
| Iduronate 2-sulfatase | IDS | -0.50 | 6.32E-09 | 0.05 | 0.33 | 4 |
| Complement component Clq receptor | CD93 | -0.52 | 1.08E-09 | 0.19 | 0.15 | 4 |
| Afamin | AFM | -0.52 | 1.36E-05 | 0.02 | 0.94 | 4 |
| Endothelial cell-specific molecule 1 | ESM1 | -0.54 | 3.16E-06 | 0.09 | 0.56 | 4 |
| Scavenger receptor cysteine-rich type 1 protein M130 | CD163 | 1.45 | 1.33E-52 | NA | NA | 5 |
| Macrophage colony-stimulating factor 1 receptor | CSF1R | 1.09 | 2.07E-25 | NA | NA | 5 |
| Interleukin-18-binding protein | IL18BP | 0.67 | 4.56E-15 | NA | NA | 5 |
| Interleukin-6 receptor subunit beta | IL6ST | 0.65 | 1.03E-22 | NA | NA | 5 |

(continued)

| Target Full Name | Gene | human | | Cyno | | Tier |
|---|---|---|---|---|---|---|
| | | Estimate (SD) | P-value | Estimate log2FC | P-value | |
| Urokinase-type plasminogen activator | PLAU | 0.58 | 1.03E-07 | NA | NA | 5 |
| Sialic acid-binding Ig-like lectin 7 | SIGLEC7 | 0.55 | 4.01E-09 | NA | NA | 5 |
| Low affinity immunoglobulin gamma Fc region receptor III-B | FCGR3B | 0.47 | 2.57E-05 | NA | NA | 5 |
| Cell surface glycoprotein CD200 receptor 1 | CD200R1 | 0.46 | 4.48E-05 | NA | NA | 5 |
| T-lymphocyte surface antigen Ly-9 | LY9 | 0.40 | 3.92E-05 | NA | NA | 5 |
| Complement C1s subcomponent | C1S | 0.38 | 2.93E-05 | NA | NA | 5 |
| Complement decay-accelerating factor | CD55 | 0.37 | 2.96E-07 | NA | NA | 5 |
| RGM domain family member B | RGMB | -0.34 | 1.56E-07 | NA | NA | 5 |
| Lumican | LUM | -0.48 | 5.56E-07 | NA | NA | 5 |
| Tumor necrosis factor receptor superfamily member 17 | TNFRSF17 | -0.48 | 1.67E-05 | NA | NA | 5 |

**References**

1 See also, Nioi, P. et al. Variant ASGR1 Associated with a Reduced Risk of Coronary Artery Disease. The New England journal of medicine 374, 2131-2141, doi:10.1056/NEJMoa1508419 (2016).

2 Gold, L. et al. Aptamer-based multiplexed proteomic technology for biomarker discovery. PLoS One 5, el 5004, doi:10.1371/journal.pone.0015004 (2010).

**Example 20: Method of reducing a risk of cardiovascular disease**

A subject at risk of cardiovascular disease is identified. One or more antibodies as provided herein (see Example 7, as well as Tables A, B and C) and/or RNAi constructs that reduce expression of ASGR-1 and/or ASGR-2 (as outlined in Example 3), are administered to a subject at risk of cardiovascular disease. The antibody and/or RNAi construct reduces the level of expression of ASGR, ASGR-1 and/or ASGR-2. Subsequent rounds of antibodies and/or RNAi are administered to the subject. One or more of the markers in Example 19 (e.g., Tier 1) is monitored to make certain that an adequate amount of the antibody and/or RNAi construct is administered and is functioning as desired. The risk that the subject will experience cardio vascular disease is decreased.

Additionally, as a further option, physiologic effects of the antibody and/or RNAi can be evaluated in relevant animal models of cardiovascular disease using readouts including blood pressure, primary and secondary hemostasis, heart function and morphology, endothelial function, LDL cholesterol levels, non-HDL cholesterol levels, inflammation, and/or atherosclerosis.

**Example 21: Method of reducing a risk of myocardial infarction or coronary artery disease**

A subject at risk of a myocardial infarction or coronary artery disease is identified. One or more antibodies as provided herein (see Example 7, as well as Tables A, B and C) and/or RNAi constructs that reduce expression of ASGR-1 and/or ASGR-2 (as outlined in Example 3), are administered to a subject at risk of a myocardial infarction or coronary artery disease. The antibody and/or RNAi construct reduces the level of expression of ASGR, ASGR-1 and/or ASGR-2. Subsequent rounds of antibodies and/or RNAi are administered to the subject. One or more of the markers in Example 19 (e.g., Tier 1) is monitored to make certain that an adequate amount of the antibody and/or RNAi construct is administered and is functioning as desired. The risk that the subject will experience a myocardial infarction or coronary artery disease is decreased.

Additionally, as a further option, physiologic effects of the antibody and/or RNAi can be evaluated in relevant animal models of myocardial infarction or coronary artery disease using readouts including blood pressure, primary and secondary hemostasis, heart function and morphology, endothelial function, LDL cholesterol levels, non-HDL cholesterol levels, inflammation, and/or atherosclerosis.

## Example 22: Method of reducing LDL cholesterol

A subject having a LDL cholesterol level to be lowered is identified. One or more antibodies as provided herein (see Example 7, as well as Tables A, B and C) and/or RNAi constructs that reduce expression of ASGR-1 and/or ASGR-2 (as outlined in Example 3), are administered to the subject. The antibody and/or RNAi construct reduces the level of expression of ASGR, ASGR-1 and/or ASGR-2. Subsequent rounds of antibodies and/or RNAi are administered to the subject. One or more of the markers in Example 19 (e.g., tier 1) is monitored to make certain that an adequate amount of the antibody and/or RNAi construct is administered and is functioning as desired. The level of LDL cholesterol in the subject is thereby reduced.

## Example 23: Method of reducing non-HDL cholesterol

A subject having a non-HDL cholesterol level to be lowered is identified. One or more antibodies as provided herein (see Example 7, as well as Tables A, B and C) and/or RNAi constructs that reduce expression of ASGR-1 and/or ASGR-2 (as outlined in Example 3), are administered to the subject. The antibody and/or RNAi construct reduces the level of expression of ASGR, ASGR-1 and/or ASGR-2. Subsequent rounds of antibodies and/or RNAi are administered to the subject. One or more of the markers in Example 19 (e.g., Tier 1) is monitored to make certain that an adequate amount of the antibody and/or RNAi construct is administered and is functioning as desired. The level of non-HDL cholesterol in the subject is thereby reduced.

## Example 24; Method of increasing ALP levels

One or more antibodies as provided herein (see Example 7, as well as Tables A, B, and C) and/or RNAi constructs that reduce expression of ASGR-1 and/or ASGR-2 (as outlined in Example 3), are administered to the subject. The antibody and/or RNAi construct reduces the level of expression of ASGR, ASGR-1 and/or ASGR-2. Subsequent rounds of antibodies and/or RNAi are administered to the subject. One or more of the markers in Example 19 (e.g., Tier 1) is monitored to make certain that an adequate amount of the antibody and/or RNAi construct is administered and is functioning as desired. The level of ALP in the subject is thereby increased.

## Example 25: Method of Monitoring the Effectiveness of an ASGR-1 Therapy

One or more antibodies as provided herein (see Example 7, as well as Tables A, B and C) and/or RNAi constructs that reduce expression of ASGR-1 and/or ASGR-2 (as outlined in Example 3), are administered to the subject. One or more of the markers in Example 19 is monitored to make certain that an adequate amount of the antibody and/or RNAi construct is administered and is functioning as desired. When the marker level changes in a similar manner to those changes noted in Example 19 (e.g., Tier 1), it is evidence that the amount of the one or more antibody and/or RNAi is effective. Additionally, as a further option, the effectiveness of this biochemical change can be observed by its physiologic effects from the antibody and/or RNAi, which can be evaluated using readouts including blood pressure, primary and secondary hemostasis, heart function and morphology, endothelial function, LDL cholesterol levels, non-HDL cholesterol levels, inflammation, and/or atherosclerosis.

**TABLE 15: Antibody Constant Regions**

| Designation | SEQ ID NO: | CL Domain Amino Acid Sequence |
|---|---|---|
| CL-1 | 32681 | GQPKANPTVTLFPPSSEELQANKATLVCLISDFYPGAVTVAWKADGSP VKAGVETTKPSKQSNNKYAASSYLSLTPEQWKSHRSYSCQVTHEGSTV EKTVAPTECS |

(continued)

| Designation | SEQ ID NO: | CL Domain Amino Acid Sequence |
|---|---|---|
| CL-2 | 32682 | GQPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS |
| CL-2.1 | 32683 | QPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS |
| CL-3 | 32684 | GQPKAAPSVTLFPPSSEELQANKATLVCLISDFYPGAVTVAWKADSSPVKAGVETTTPSKQSNNKYAASSYLSLTPEQWKSHKSYSCQVTHEGSTVEKTVAPTECS |
| CL-7 | 32685 | GQPKAAPSVTLFPPSSEELQANKATLVCLVSDFYPGAVTVAWKADGSPVKVGVETTKPSKQSNNKYAASSYLSLTPEQWKSHRSYSCRVTHEGSTVEKTVAPAECS |
| Human kappa v1 | 32686 | TVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| Human kappa v2 | 32687 | RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |

**TABLE 15: Antibody Constant Regions**

| Ig isotype | SEQ ID NO: | Heavy Chain Constant Region Amino Acid Sequence |
|---|---|---|
| Human IgGlz | 32688 | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| Human IgGlza | 32689 | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| Ig isotype | SEQ ID NO: | Heavy Chain Constant Region Amino Acid Sequence |
|---|---|---|
| Human IgGlf | 32690 | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| Human IgG1fa | 32691 | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| Human IgG2 | 32692 | ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERKCCVECPPCPAPPVAGPSVFLFPPKPKDTLMISRTPEVTCVVVDVSHEDPEVQFNWYVDGVEVHNAKTKPREEQFNSTFRVVSVLTVVHQDWLNGKEYKCKVSNKGLPAPIEKTISKTKGQPREPQVYTLPPSREEMTKNQVSLTCLVKGFYPSDIAVEWESNGQPENNYKTTPPMLDSDGSFFLYSKLTVDKSRWQQGNVFSCSVMHEALHNHYTQKSLSLSPGK |
| Human IgG4 | 32693 | ASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPSCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGK |
| Human IgG1-SEFL2 | 32694 | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPCEEQYGSTYRCVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |

**Table 10.1**

| | | | | | | | | | | | |
|---|---|---|---|---|---|---|---|---|---|---|---|
| ATOM | 1 | O | THR | A | 151 | -35.000 | -25.802 | 13.973 | 1.00 | 41.82 | O |
| ATOM | 2 | N | THR | A | 151 | -34.909 | -27.403 | 11.804 | 1.00 | 40.99 | N |
| ATOM | 3 | CA | THR | A | 151 | -34.274 | -27.888 | 13.020 | 1.00 | 40.74 | C |
| ATOM | 4 | C | THR | A | 151 | -34.232 | -26.755 | 14.051 | 1.00 | 40.85 | C |
| ATOM | 5 | CB | THR | A | 151 | -32.835 | -28.381 | 12.758 | 1.00 | 37.81 | C |
| ATOM | 6 | OG1 | THR | A | 151 | -32.738 | -28.919 | 11.438 | 1.00 | 46.22 | O |

(continued)

| | | | | | | | | | | | |
|---|---|---|---|---|---|---|---|---|---|---|---|
| ATOM | 7 | CG2 | THR | A | 151 | -32.470 | -29.491 | 13.725 | 1.00 | 44.52 | C |
| ATOM | 8 | O | CYS | A | 152 | -30.928 | -26.774 | 16.222 | 1.00 | 27.69 | O |
| ATOM | 9 | N | CYS | A | 152 | -33.315 | -26.861 | 15.005 | 1.00 | 43.00 | N |
| ATOM | 10 | CA | CYS | A | 152 | -33.119 | -25.852 | 16.027 | 1.00 | 32.33 | C |
| ATOM | 11 | C | CYS | A | 152 | -31.621 | -25.772 | 16.312 | 1.00 | 30.92 | C |
| ATOM | 12 | CB | CYS | A | 152 | -33.900 | -26.213 | 17.289 | 1.00 | 36.97 | C |
| ATOM | 13 | SG | CYS | A | 152 | -34.435 | -24.804 | 18.287 | 1.00 | 47.04 | S |
| ATOM | 14 | N | CYS | A | 153 | -31.104 | -24.590 | 16.620 | 1.00 | 26.78 | N |
| ATOM | 15 | CA | CYS | A | 153 | -29.716 | -24.493 | 17.055 | 1.00 | 24.74 | C |
| ATOM | 16 | C | CYS | A | 153 | -29.577 | -25.058 | 18.464 | 1.00 | 26.56 | C |
| ATOM | 17 | O | CYS | A | 153 | -30.538 | -25.026 | 19.235 | 1.00 | 25.88 | O |
| ATOM | 18 | CB | CYS | A | 153 | -29.243 | -23.040 | 17.017 | 1.00 | 21.46 | C |
| ATOM | 19 | SG | CYS | A | 153 | -29.368 | -22.304 | 15.376 | 1.00 | 33.20 | S |
| ATOM | 20 | N | PRO | A | 154 | -28.379 | -25.571 | 18.813 | 1.00 | 26.96 | N |
| ATOM | 21 | CA | PRO | A | 154 | -28.146 | -26.019 | 20.190 | 1.00 | 20.79 | C |
| ATOM | 22 | C | PRO | A | 154 | -28.236 | -24.848 | 21.163 | 1.00 | 22.57 | C |
| ATOM | 23 | O | PRO | A | 154 | -28.081 | -23.710 | 20.737 | 1.00 | 22.50 | O |
| ATOM | 24 | CB | PRO | A | 154 | -26.715 | -26.585 | 20.147 | 1.00 | 20.99 | C |
| ATOM | 25 | CG | PRO | A | 154 | -26.432 | -26.822 | 18.709 | 1.00 | 21.42 | C |
| ATOM | 26 | CD | PRO | A | 154 | -27.183 | -25.760 | 17.974 | 1.00 | 19.94 | C |
| ATOM | 27 | N | VAL | A | 155 | -28.499 | -25.124 | 22.438 | 1.00 | 21.58 | N |
| ATOM | 28 | CA | VAL | A | 155 | -28.490 | -24.090 | 23.462 | 1.00 | 20.72 | C |
| ATOM | 29 | C | VAL | A | 155 | -27.187 | -23.287 | 23.412 | 1.00 | 25.13 | C |
| ATOM | 30 | O | VAL | A | 155 | -26.109 | -23.865 | 23.218 | 1.00 | 27.20 | O |
| ATOM | 31 | CB | VAL | A | 155 | -28.664 | -24.704 | 24.884 | 1.00 | 23.97 | C |
| ATOM | 32 | CG1 | VAL | A | 155 | -28.384 | -23.669 | 25.957 | 1.00 | 27.90 | C |
| ATOM | 33 | CG2 | VAL | A | 155 | -30.062 | -25.267 | 25.061 | 1.00 | 25.53 | C |
| ATOM | 34 | N | ASN | A | 156 | -27.299 | -21.968 | 23.586 | 1.00 | 20.81 | N |
| ATOM | 35 | CA | ASN | A | 156 | -26.158 | -21.050 | 23.634 | 1.00 | 24.68 | C |
| ATOM | 36 | C | ASN | A | 156 | -25.568 | -20.824 | 22.253 | 1.00 | 25.74 | C |
| ATOM | 37 | O | ASN | A | 156 | -24.518 | -20.206 | 22.106 | 1.00 | 25.52 | O |
| ATOM | 38 | CB | ASN | A | 156 | -25.070 | -21.552 | 24.603 | 1.00 | 30.21 | C |
| ATOM | 39 | CG | ASN | A | 156 | -25.565 | -21.628 | 26.041 | 1.00 | 38.75 | C |
| ATOM | 40 | OD1 | ASN | A | 156 | -26.494 | -20.908 | 26.430 | 1.00 | 35.88 | O |
| ATOM | 41 | ND2 | ASN | A | 156 | -24.953 | -22.502 | 26.835 | 1.00 | 33.84 | N |
| ATOM | 42 | N | TRP | A | 157 | -26.247 | -21.340 | 21.237 | 1.00 | 24.43 | N |
| ATOM | 43 | CA | TRP | A | 157 | -25.928 | -20.981 | 19.866 | 1.00 | 23.93 | C |
| ATOM | 44 | C | TRP | A | 157 | -26.920 | -19.930 | 19.389 | 1.00 | 26.16 | C |
| ATOM | 45 | O | TRP | A | 157 | -28.032 | -19.841 | 19.904 | 1.00 | 28.51 | O |
| ATOM | 46 | CB | TRP | A | 157 | -25.965 | -22.202 | 18.965 | 1.00 | 22.94 | C |
| ATOM | 47 | CG | TRP | A | 157 | -24.818 | -23.129 | 19.174 | 1.00 | 24.76 | C |
| ATOM | 48 | CD1 | TRP | A | 157 | -24.459 | -23.752 | 20.336 | 1.00 | 20.94 | C |
| ATOM | 49 | CD2 | TRP | A | 157 | -23.887 | -23.562 | 18.180 | 1.00 | 19.03 | C |
| ATOM | 50 | NE1 | TRP | A | 157 | -23.355 | -24.540 | 20.126 | 1.00 | 23.79 | N |
| ATOM | 51 | CE2 | TRP | A | 157 | -22.983 | -24.440 | 18.809 | 1.00 | 21.53 | C |
| ATOM | 52 | CE3 | TRP | A | 157 | -23.717 | -23.273 | 16.828 | 1.00 | 15.83 | C |
| ATOM | 53 | CZ2 | TRP | A | 157 | -21.927 | -25.043 | 18.124 | 1.00 | 16.56 | C |
| ATOM | 54 | CZ3 | TRP | A | 157 | -22.667 | -23.866 | 16.152 | 1.00 | 18.56 | C |
| ATOM | 55 | CH2 | TRP | A | 157 | -21.790 | -24.746 | 16.798 | 1.00 | 13.77 | C |
| ATOM | 56 | N | VAL | A | 158 | -26.520 | -19.120 | 18.420 | 1.00 | 25.61 | N |
| ATOM | 57 | CA | VAL | A | 158 | -27.394 | -18.058 | 17.951 | 1.00 | 23.85 | C |

(continued)

| | | | | | | | | | | | |
|---|---|---|---|---|---|---|---|---|---|---|---|
| ATOM | 58 | C | VAL | A | 158 | -27.857 | -18.367 | 16.536 | 1.00 | 26.75 | C |
| ATOM | 59 | O | VAL | A | 158 | -27.048 | -18.589 | 15.642 | 1.00 | 24.47 | O |
| ATOM | 60 | CB | VAL | A | 158 | -26.698 | -16.690 | 17.998 | 1.00 | 26.10 | C |
| ATOM | 61 | CG1 | VAL | A | 158 | -27.691 | -15.587 | 17.690 | 1.00 | 21.15 | C |
| ATOM | 62 | CG2 | VAL | A | 158 | -26.076 | -16.469 | 19.368 | 1.00 | 27.52 | C |
| ATOM | 63 | N | GLU | A | 159 | -29.170 | -18.411 | 16.351 | 1.00 | 30.02 | N |
| ATOM | 64 | CA | GLU | A | 159 | -29.751 | -18.659 | 15.043 | 1.00 | 28.56 | C |
| ATOM | 65 | C | GLU | A | 159 | -29.824 | -17.359 | 14.272 | 1.00 | 25.78 | C |
| ATOM | 66 | O | GLU | A | 159 | -30.140 | -16.313 | 14.840 | 1.00 | 24.18 | O |
| ATOM | 67 | CB | GLU | A | 159 | -31.145 | -19.279 | 15.182 | 1.00 | 24.88 | C |
| ATOM | 68 | CG | GLU | A | 159 | -31.764 | -19.742 | 13.875 | 1.00 | 35.35 | C |
| ATOM | 69 | CD | GLU | A | 159 | -33.213 | -20.190 | 14.052 | 1.00 | 41.67 | C |
| ATOM | 70 | OE1 | GLU | A | 159 | -34.123 | -19.392 | 13.733 | 1.00 | 44.61 | O |
| ATOM | 71 | OE2 | GLU | A | 159 | -33.441 | -21.333 | 14.516 | 1.00 | 36.73 | O |
| ATOM | 72 | N | HIS | A | 160 | -29.542 | -17.432 | 12.977 | 1.00 | 25.27 | N |
| ATOM | 73 | CA | HIS | A | 160 | -29.577 | -16.257 | 12.118 | 1.00 | 31.03 | C |
| ATOM | 74 | C | HIS | A | 160 | -29.525 | -16.672 | 10.656 | 1.00 | 30.42 | C |
| ATOM | 75 | O | HIS | A | 160 | -28.530 | -17.261 | 10.205 | 1.00 | 26.33 | O |
| ATOM | 76 | CB | HIS | A | 160 | -28.411 | -15.317 | 12.426 | 1.00 | 29.99 | C |
| ATOM | 77 | CG | HIS | A | 160 | -28.320 | -14.150 | 11.493 | 1.00 | 28.06 | C |
| ATOM | 78 | ND1 | HIS | A | 160 | -29.043 | -12.991 | 11.679 | 1.00 | 33.48 | N |
| ATOM | 79 | CD2 | HIS | A | 160 | -27.605 | -13.970 | 10.359 | 1.00 | 29.69 | C |
| ATOM | 80 | CE1 | HIS | A | 160 | -28.770 | -12.143 | 10.703 | 1.00 | 30.20 | C |
| ATOM | 81 | NE2 | HIS | A | 160 | -27.903 | -12.713 | 9.887 | 1.00 | 29.37 | N |
| ATOM | 82 | N | GLU | A | 161 | -30.593 | -16.361 | 9.925 | 1.00 | 29.26 | N |
| ATOM | 83 | CA | GLU | A | 161 | -30.696 | -16.730 | 8.520 | 1.00 | 30.40 | C |
| ATOM | 84 | C | GLU | A | 161 | -30.349 | -18.188 | 8.278 | 1.00 | 30.86 | C |
| ATOM | 85 | O | GLU | A | 161 | -29.548 | -18.493 | 7.393 | 1.00 | 34.60 | O |
| ATOM | 86 | CB | GLU | A | 161 | -29.788 | -15.852 | 7.659 | 1.00 | 35.54 | C |
| ATOM | 87 | CG | GLU | A | 161 | -30.197 | -14.386 | 7.604 | 1.00 | 41.66 | C |
| ATOM | 88 | CD | GLU | A | 161 | -31.526 | -14.165 | 6.901 | 1.00 | 42.46 | C |
| ATOM | 89 | OE1 | GLU | A | 161 | -32.027 | -15.108 | 6.252 | 1.00 | 47.99 | O |
| ATOM | 90 | OE2 | GLU | A | 161 | -32.070 | -13.043 | 7.001 | 1.00 | 40.82 | O |
| ATOM | 91 | N | ARG | A | 162 | -30.928 | -19.068 | 9.092 | 1.00 | 24.36 | N |
| ATOM | 92 | CA | ARG | A | 162 | -30.792 | -20.516 | 8.931 | 1.00 | 31.86 | C |
| ATOM | 93 | C | ARG | A | 162 | -29.374 | -21.045 | 9.202 | 1.00 | 30.42 | C |
| ATOM | 94 | O | ARG | A | 162 | -29.030 | -22.166 | 8.814 | 1.00 | 31.05 | O |
| ATOM | 95 | CB | ARG | A | 162 | -31.250 | -20.930 | 7.528 | 1.00 | 38.14 | C |
| ATOM | 96 | CG | ARG | A | 162 | -32.267 | -22.051 | 7.540 | 1.00 | 48.13 | C |
| ATOM | 97 | CD | ARG | A | 162 | -33.076 | -22.071 | 6.261 | 1.00 | 59.39 | C |
| ATOM | 98 | NE | ARG | A | 162 | -33.517 | -23.423 | 5.921 | 1.00 | 73.66 | N |
| ATOM | 99 | CZ | ARG | A | 162 | -34.365 | -23.702 | 4.937 | 1.00 | 64.55 | C |
| ATOM | 100 | NH1 | ARG | A | 162 | -34.866 | -22.720 | 4.200 | 1.00 | 53.99 | N |
| ATOM | 101 | NH2 | ARG | A | 162 | -34.711 | -24.960 | 4.694 | 1.00 | 63.33 | N |
| ATOM | 102 | N | SER | A | 163 | -28.556 | -20.240 | 9.869 | 1.00 | 24.82 | N |
| ATOM | 103 | CA | SER | A | 163 | -27.287 | -20.728 | 10.379 | 1.00 | 27.06 | C |
| ATOM | 104 | C | SER | A | 163 | -27.270 | -20.611 | 11.897 | 1.00 | 28.98 | C |
| ATOM | 105 | O | SER | A | 163 | -27.914 | -19.730 | 12.474 | 1.00 | 28.00 | O |
| ATOM | 106 | CB | SER | A | 163 | -26.110 | -19.963 | 9.768 | 1.00 | 26.42 | C |
| ATOM | 107 | OG | SER | A | 163 | -25.629 | -20.605 | 8.596 | 1.00 | 27.29 | O |
| ATOM | 108 | N | CYS | A | 164 | -26.548 | -21.522 | 12.537 | 1.00 | 26.62 | N |

(continued)

| | | | | | | | | | | | |
|---|---|---|---|---|---|---|---|---|---|---|---|
| ATOM | 109 | CA | CYS | A | 164 | -26.342 | -21.469 | 13.975 | 1.00 | 23.71 | C |
| ATOM | 110 | C | CYS | A | 164 | -24.920 | -20.996 | 14.249 | 1.00 | 23.70 | C |
| ATOM | 111 | O | CYS | A | 164 | -23.980 | -21.412 | 13.579 | 1.00 | 20.95 | O |
| ATOM | 112 | CB | CYS | A | 164 | -26.591 | -22.838 | 14.601 | 1.00 | 25.60 | C |
| ATOM | 113 | SG | CYS | A | 164 | -28.228 | -23.515 | 14.210 | 1.00 | 29.69 | S |
| ATOM | 114 | N | TYR | A | 165 | -24.766 | -20.116 | 15.227 | 1.00 | 23.47 | N |
| ATOM | 115 | CA | TYR | A | 165 | -23.469 | -19.539 | 15.507 | 1.00 | 21.97 | C |
| ATOM | 116 | C | TYR | A | 165 | -23.139 | -19.708 | 16.970 | 1.00 | 22.33 | C |
| ATOM | 117 | O | TYR | A | 165 | -23.970 | -19.473 | 17.835 | 1.00 | 21.92 | O |
| ATOM | 118 | CB | TYR | A | 165 | -23.434 | -18.055 | 15.141 | 1.00 | 17.23 | C |
| ATOM | 119 | CG | TYR | A | 165 | -23.665 | -17.761 | 13.679 | 1.00 | 18.76 | C |
| ATOM | 120 | CD1 | TYR | A | 165 | -24.949 | -17.615 | 13.174 | 1.00 | 22.13 | C |
| ATOM | 121 | CD2 | TYR | A | 165 | -22.601 | -17.602 | 12.813 | 1.00 | 21.90 | C |
| ATOM | 122 | CE1 | TYR | A | 165 | -25.169 | -17.327 | 11.833 | 1.00 | 25.39 | C |
| ATOM | 123 | CE2 | TYR | A | 165 | -22.808 | -17.317 | 11.470 | 1.00 | 23.30 | C |
| ATOM | 124 | CZ | TYR | A | 165 | -24.096 | -17.177 | 10.990 | 1.00 | 24.28 | C |
| ATOM | 125 | OH | TYR | A | 165 | -24.295 | -16.900 | 9.663 | 1.00 | 25.37 | O |
| ATOM | 126 | N | TRP | A | 166 | -21.913 | -20.111 | 17.243 | 1.00 | 17.81 | N |
| ATOM | 127 | CA | TRP | A | 166 | -21.436 | -20.129 | 18.601 | 1.00 | 20.06 | C |
| ATOM | 128 | C | TRP | A | 166 | -20.213 | -19.213 | 18.680 | 1.00 | 18.56 | C |
| ATOM | 129 | O | TRP | A | 166 | -19.289 | -19.307 | 17.870 | 1.00 | 18.84 | O |
| ATOM | 130 | CB | TRP | A | 166 | -21.117 | -21.554 | 19.040 | 1.00 | 18.95 | C |
| ATOM | 131 | CG | TRP | A | 166 | -20.709 | -21.665 | 20.486 | 1.00 | 22.40 | C |
| ATOM | 132 | CD1 | TRP | A | 166 | -21.533 | -21.745 | 21.563 | 1.00 | 21.92 | C |
| ATOM | 133 | CD2 | TRP | A | 166 | -19.369 | -21.697 | 21.001 | 1.00 | 22.78 | C |
| ATOM | 134 | NE1 | TRP | A | 166 | -20.797 | -21.831 | 22.717 | 1.00 | 26.27 | N |
| ATOM | 135 | CE2 | TRP | A | 166 | -19.464 | -21.805 | 22.400 | 1.00 | 22.51 | C |
| ATOM | 136 | CE3 | TRP | A | 166 | -18.098 | -21.648 | 20.408 | 1.00 | 20.00 | C |
| ATOM | 137 | CZ2 | TRP | A | 166 | -18.343 | -21.869 | 23.221 | 1.00 | 19.21 | C |
| ATOM | 138 | CZ3 | TRP | A | 166 | -16.989 | -21.716 | 21.214 | 1.00 | 19.92 | C |
| ATOM | 139 | CH2 | TRP | A | 166 | -17.116 | -21.820 | 22.616 | 1.00 | 20.77 | C |
| ATOM | 140 | N | PHE | A | 167 | -20.233 | -18.308 | 19.648 | 1.00 | 20.52 | N |
| ATOM | 141 | CA | PHE | A | 167 | -19.189 | -17.304 | 19.803 | 1.00 | 17.05 | C |
| ATOM | 142 | C | PHE | A | 167 | -18.367 | -17.589 | 21.039 | 1.00 | 23.11 | C |
| ATOM | 143 | O | PHE | A | 167 | -18.847 | -17.397 | 22.149 | 1.00 | 20.92 | O |
| ATOM | 144 | CB | PHE | A | 167 | -19.794 | -15.905 | 19.894 | 1.00 | 17.09 | C |
| ATOM | 145 | CG | PHE | A | 167 | -20.649 | -15.533 | 18.712 | 1.00 | 19.67 | C |
| ATOM | 146 | CD1 | PHE | A | 167 | -22.012 | -15.806 | 18.710 | 1.00 | 20.06 | C |
| ATOM | 147 | CD2 | PHE | A | 167 | -20.087 | -14.907 | 17.598 | 1.00 | 18.66 | C |
| ATOM | 148 | CE1 | PHE | A | 167 | -22.807 | -15.457 | 17.615 | 1.00 | 21.81 | C |
| ATOM | 149 | CE2 | PHE | A | 167 | -20.860 | -14.555 | 16.501 | 1.00 | 14.19 | C |
| ATOM | 150 | CZ | PHE | A | 167 | -22.230 | -14.836 | 16.502 | 1.00 | 20.28 | C |
| ATOM | 151 | N | SER | A | 168 | -17.127 | -18.039 | 20.853 | 1.00 | 20.07 | N |
| ATOM | 152 | CA | SER | A | 168 | -16.296 | -18.360 | 22.000 | 1.00 | 19.88 | C |
| ATOM | 153 | C | SER | A | 168 | -16.073 | -17.121 | 22.847 | 1.00 | 23.65 | C |
| ATOM | 154 | O | SER | A | 168 | -16.105 | -15.992 | 22.331 | 1.00 | 22.61 | O |
| ATOM | 155 | CB | SER | A | 168 | -14.950 | -18.952 | 21.570 | 1.00 | 13.34 | C |
| ATOM | 156 | OG | SER | A | 168 | -14 .018 | -17.940 | 21.262 | 1.00 | 16.40 | O |
| ATOM | 157 | N | ARG | A | 169 | -15.877 | -17.339 | 24.149 | 1.00 | 18.01 | N |
| ATOM | 158 | CA | ARG | A | 169 | -15.443 | -16.284 | 25.038 | 1.00 | 22.03 | C |
| ATOM | 159 | C | ARG | A | 169 | -14.033 | -16.614 | 25.568 | 1.00 | 21.36 | C |

(continued)

| | | | | | | | | | | | |
|---|---|---|---|---|---|---|---|---|---|---|---|
| ATOM | 160 | O | ARG | A | 169 | -13.617 | -16.124 | 26.615 | 1.00 | 24.92 | O |
| ATOM | 161 | CB | ARG | A | 169 | -16.447 | -16.082 | 26.182 | 1.00 | 26.09 | C |
| ATOM | 162 | CG | ARG | A | 169 | -17 . 858 | -15.584 | 25.733 | 1.00 | 33.24 | C |
| ATOM | 163 | CD | ARG | A | 169 | -17.799 | -14.586 | 24.532 | 1.00 | 35.48 | C |
| ATOM | 164 | NE | ARG | A | 169 | -19.120 | -14.199 | 24.007 | 1.00 | 41.60 | N |
| ATOM | 165 | CZ | ARG | A | 169 | -19.315 | -13.401 | 22.953 | 1.00 | 38.23 | C |
| ATOM | 166 | NH1 | ARG | A | 169 | -18.279 | -12.905 | 22.281 | 1.00 | 28.72 | N |
| ATOM | 167 | NH2 | ARG | A | 169 | -20.550 | -13.100 | 22.559 | 1.00 | 36.96 | N |
| ATOM | 168 | N | SER | A | 170 | -13.303 | -17.438 | 24.823 | 1.00 | 16.23 | N |
| ATOM | 169 | CA | SER | A | 170 | -11.877 | -17.660 | 25.065 | 1.00 | 16.00 | C |
| ATOM | 170 | C | SER | A | 170 | -11.094 | -17.698 | 23.742 | 1.00 | 17.67 | C |
| ATOM | 171 | O | SER | A | 170 | -11.662 | -17.537 | 22.663 | 1.00 | 16.96 | O |
| ATOM | 172 | CB | SER | A | 170 | -11.652 | -18.958 | 25.851 | 1.00 | 17.57 | C |
| ATOM | 173 | OG | SER | A | 170 | -12.101 | -20.084 | 25.121 | 1.00 | 18.85 | O |
| ATOM | 174 | N | GLY | A | 171 | -9.783 | -17.916 | 23.824 | 1.00 | 19.23 | N |
| ATOM | 175 | CA | GLY | A | 171 | -8.947 | -17.854 | 22.645 | 1.00 | 12.77 | C |
| ATOM | 176 | C | GLY | A | 171 | -8.169 | -19.115 | 22.334 | 1.00 | 16.65 | C |
| ATOM | 177 | O | GLY | A | 171 | -7.701 | -19.820 | 23.236 | 1.00 | 16.99 | O |
| ATOM | 178 | N | LYS | A | 172 | -8.037 | -19.389 | 21.039 | 1.00 | 15.85 | N |
| ATOM | 179 | CA | LYS | A | 172 | -7.313 | -20.542 | 20.533 | 1.00 | 13.77 | C |
| ATOM | 180 | C | LYS | A | 172 | -6.509 | -20.164 | 19.309 | 1.00 | 17.02 | C |
| ATOM | 181 | O | LYS | A | 172 | -6.873 | -19.224 | 18.576 | 1.00 | 17.20 | O |
| ATOM | 182 | CB | LYS | A | 172 | -8.262 | -21. 678 | 20.148 | 1.00 | 16.06 | C |
| ATOM | 183 | CG | LYS | A | 172 | -8.818 | -22.506 | 21.281 | 1.00 | 17.04 | C |
| ATOM | 184 | CD | LYS | A | 172 | -9.638 | -23.666 | 20.687 | 1.00 | 17.13 | C |
| ATOM | 185 | CE | LYS | A | 172 | -10.400 | -24.441 | 21.766 | 1.00 | 16.06 | C |
| ATOM | 186 | NZ | LYS | A | 172 | -9.439 | -25.070 | 22.711 | 1.00 | 20.45 | N |
| ATOM | 187 | N | ALA | A | 173 | -5.427 | -20.902 | 19.074 | 1.00 | 13.08 | N |
| ATOM | 188 | CA | ALA | A | 173 | -4.750 | -20.834 | 17.792 | 1.00 | 14.02 | C |
| ATOM | 189 | C | ALA | A | 173 | -5.758 | -21.289 | 16.754 | 1.00 | 16.04 | C |
| ATOM | 190 | O | ALA | A | 173 | -6.675 | -22.056 | 17.064 | 1.00 | 15.29 | O |
| ATOM | 191 | CB | ALA | A | 173 | -3.482 | -21.720 | 17.766 | 1.00 | 14.17 | C |
| ATOM | 192 | N | TRP | A | 174 | -5.591 | -20.827 | 15.522 | 1.00 | 16.89 | N |
| ATOM | 193 | CA | TRP | A | 174 | -6.571 | -21. 098 | 14.492 | 1.00 | 15.29 | C |
| ATOM | 194 | C | TRP | A | 174 | -6.839 | -22.598 | 14.329 | 1.00 | 17.24 | C |
| ATOM | 195 | O | TRP | A | 174 | -8.000 | -23.030 | 14.334 | 1.00 | 17.21 | O |
| ATOM | 196 | CB | TRP | A | 174 | -6.111 | -20.486 | 13.185 | 1.00 | 15.83 | C |
| ATOM | 197 | CG | TRP | A | 174 | -7.133 | -20.523 | 12.123 | 1.00 | 17.80 | C |
| ATOM | 198 | CD1 | TRP | A | 174 | -8.015 | -19.532 | 11.795 | 1.00 | 14.69 | C |
| ATOM | 199 | CD2 | TRP | A | 174 | -7.375 | -21.598 | 11.217 | 1.00 | 17.89 | C |
| ATOM | 200 | NE1 | TRP | A | 174 | -8.784 | -19.926 | 10.732 | 1.00 | 13.89 | N |
| ATOM | 201 | CE2 | TRP | A | 174 | -8.416 | -21.193 | 10.361 | 1.00 | 17.17 | C |
| ATOM | 202 | CE3 | TRP | A | 174 | -6.803 | -22.865 | 11.036 | 1.00 | 20.13 | C |
| ATOM | 203 | CZ2 | TRP | A | 174 | -8.911 | -22.013 | 9.353 | 1.00 | 17.46 | C |
| ATOM | 204 | CZ3 | TRP | A | 174 | -7.294 | -23.675 | 10.039 | 1.00 | 18.74 | C |
| ATOM | 205 | CH2 | TRP | A | 174 | -8.338 | -23.246 | 9.208 | 1.00 | 21.31 | C |
| ATOM | 206 | N | ALA | A | 175 | -5.781 | -23.395 | 14.229 | 1.00 | 14.70 | N |
| ATOM | 207 | CA | ALA | A | 175 | -5.950 | -24.829 | 13.999 | 1.00 | 16.88 | C |
| ATOM | 208 | C | ALA | A | 175 | -6.707 | -25.488 | 15.138 | 1.00 | 18.55 | C |
| ATOM | 209 | O | ALA | A | 175 | -7.444 | -26.448 | 14.921 | 1.00 | 18.63 | O |
| ATOM | 210 | CB | ALA | A | 175 | -4.595 | -25.512 | 13.803 | 1.00 | 20.43 | C |

(continued)

| | | | | | | | | | | | |
|---|---|---|---|---|---|---|---|---|---|---|---|
| ATOM | 211 | N | ASP | A | 176 | -6.530 | -24.982 | 16.355 | 1.00 | 15.66 | N |
| ATOM | 212 | CA | ASP | A | 176 | -7.267 | -25.535 | 17.479 | 1.00 | 14.07 | C |
| ATOM | 213 | C | ASP | A | 176 | -8.717 | -25.047 | 17.471 | 1.00 | 17.62 | C |
| ATOM | 214 | O | ASP | A | 176 | -9.621 | -25.790 | 17.834 | 1.00 | 19.92 | O |
| ATOM | 215 | CB | ASP | A | 176 | -6.566 | -25.191 | 18.784 | 1.00 | 17.82 | C |
| ATOM | 216 | CG | ASP | A | 176 | -5.169 | -25.805 | 18.865 | 1.00 | 32.45 | C |
| ATOM | 217 | OD2 | ASP | A | 176 | -4.231 | -25.127 | 19.349 | 1.00 | 29.94 | O |
| ATOM | 218 | OD1 | ASP | A | 176 | -5.003 | -26.959 | 18.405 | 1.00 | 34.43 | O |
| ATOM | 219 | N | ALA | A | 177 | -8.951 | -23.811 | 17.036 | 1.00 | 15.88 | N |
| ATOM | 220 | CA | ALA | A | 177 | -10.324 | -23.342 | 16.867 | 1.00 | 15.36 | C |
| ATOM | 221 | C | ALA | A | 177 | -11.000 | -24.134 | 15.765 | 1.00 | 14.26 | C |
| ATOM | 222 | O | ALA | A | 177 | -12.176 | -24.472 | 15.856 | 1.00 | 17.76 | O |
| ATOM | 223 | CB | ALA | A | 177 | -10.355 | -21.866 | 16.550 | 1.00 | 12.43 | C |
| ATOM | 224 | N | ASP | A | 178 | -10.250 | -24.417 | 14.710 | 1.00 | 17.91 | N |
| ATOM | 225 | CA | ASP | A | 178 | -10.776 | -25.168 | 13.575 | 1.00 | 18.45 | C |
| ATOM | 226 | C | ASP | A | 178 | -11.241 | -26.540 | 14.045 | 1.00 | 20.72 | C |
| ATOM | 227 | O | ASP | A | 178 | -12.390 | -26.930 | 13.819 | 1.00 | 17.75 | O |
| ATOM | 228 | CB | ASP | A | 178 | -9.714 | -25.301 | 12.493 | 1.00 | 18.31 | C |
| ATOM | 229 | CG | ASP | A | 178 | -10.161 | -26.169 | 11.346 | 1.00 | 24.63 | C |
| ATOM | 230 | OD1 | ASP | A | 178 | -11.279 | -25.949 | 10.829 | 1.00 | 26.01 | O |
| ATOM | 231 | OD2 | ASP | A | 178 | -9.388 | -27.067 | 10.960 | 1.00 | 28.35 | O |
| ATOM | 232 | N | ASN | A | 179 | -10.343 | -27.251 | 14.729 | 1.00 | 18.35 | N |
| ATOM | 233 | CA | ASN | A | 179 | -10.668 | -28.541 | 15.329 | 1.00 | 20.73 | C |
| ATOM | 234 | C | ASN | A | 179 | -11.839 | -28.466 | 16.310 | 1.00 | 21.76 | C |
| ATOM | 235 | O | ASN | A | 179 | -12.689 | -29.359 | 16.343 | 1.00 | 22.85 | O |
| ATOM | 236 | CB | ASN | A | 179 | -9.443 | -29.130 | 16.037 | 1.00 | 19.26 | C |
| ATOM | 237 | CG | ASN | A | 179 | -9.756 | -30.435 | 16.752 | 1.00 | 24.98 | C |
| ATOM | 238 | OD1 | ASN | A | 179 | -9.955 | -30.455 | 17.961 | 1.00 | 27.04 | O |
| ATOM | 239 | ND2 | ASN | A | 179 | -9.814 | -31.528 | 16.002 | 1.00 | 24.23 | N |
| ATOM | 240 | N | TYR | A | 180 | -11.897 | -27.407 | 17.110 | 1.00 | 17.86 | N |
| ATOM | 241 | CA | TYR | A | 180 | -13.001 | -27.265 | 18.054 | 1.00 | 16.79 | C |
| ATOM | 242 | C | TYR | A | 180 | -14.342 | -27.266 | 17.313 | 1.00 | 19.82 | C |
| ATOM | 243 | O | TYR | A | 180 | -15.287 | -27.948 | 17.709 | 1.00 | 19.73 | O |
| ATOM | 244 | CB | TYR | A | 180 | -12.861 | -25.985 | 18.891 | 1.00 | 16.59 | C |
| ATOM | 245 | CG | TYR | A | 180 | -14.056 | -25.719 | 19.810 | 1.00 | 19.48 | C |
| ATOM | 246 | CD2 | TYR | A | 180 | -13.996 | -26.024 | 21.162 | 1.00 | 24.26 | C |
| ATOM | 247 | CD1 | TYR | A | 180 | -15.245 | -25.156 | 19.316 | 1.00 | 18.40 | C |
| ATOM | 248 | CE2 | TYR | A | 180 | -15.089 | -25.789 | 22.008 | 1.00 | 20.67 | C |
| ATOM | 249 | CE1 | TYR | A | 180 | -16.328 | -24.922 | 20.135 | 1.00 | 16.82 | C |
| ATOM | 250 | CZ | TYR | A | 180 | -16.243 | -25.239 | 21.483 | 1.00 | 23.60 | C |
| ATOM | 251 | OH | TYR | A | 180 | -17.312 | -25.009 | 22.311 | 1.00 | 27.99 | O |
| ATOM | 252 | N | CYS | A | 181 | -14.416 | -26.480 | 16.247 | 1.00 | 21.68 | N |
| ATOM | 253 | CA | CYS | A | 181 | -15.634 | -26.361 | 15.468 | 1.00 | 21.18 | C |
| ATOM | 254 | C | CYS | A | 181 | -16.005 | -27.691 | 14.816 | 1.00 | 22.39 | C |
| ATOM | 255 | O | CYS | A | 181 | -17.173 | -28.054 | 14.738 | 1.00 | 17.23 | O |
| ATOM | 256 | CB | CYS | A | 181 | -15.479 | -25.268 | 14.414 | 1.00 | 17.00 | C |
| ATOM | 257 | SG | CYS | A | 181 | -15.347 | -23.612 | 15.108 | 1.00 | 21.92 | S |
| ATOM | 258 | N | ARG | A | 182 | -15.008 | -28.435 | 14.366 | 1.00 | 22.70 | N |
| ATOM | 259 | CA | ARG | A | 182 | -15.309 | -29.699 | 13.720 | 1.00 | 25.40 | C |
| ATOM | 260 | C | ARG | A | 182 | -15.881 | -30.687 | 14.737 | 1.00 | 22.32 | C |
| ATOM | 261 | O | ARG | A | 182 | -16.756 | -31.489 | 14.417 | 1.00 | 21.62 | O |

(continued)

| | | | | | | | | | | | |
|---|---|---|---|---|---|---|---|---|---|---|---|
| ATOM | 262 | CB | ARG | A | 182 | -14.064 | -30.255 | 13.031 | 1.00 | 22.43 | C |
| ATOM | 263 | CG | ARG | A | 182 | -13.757 | -29.535 | 11.727 | 1.00 | 27.35 | C |
| ATOM | 264 | CD | ARG | A | 182 | -12.390 | -29.887 | 11.188 | 1.00 | 27.56 | C |
| ATOM | 265 | NE | ARG | A | 182 | -11.981 | -28.956 | 10.139 | 1.00 | 34.68 | N |
| ATOM | 266 | CZ | ARG | A | 182 | -12.311 | -29.080 | 8.851 | 1.00 | 37.24 | C |
| ATOM | 267 | NH1 | ARG | A | 182 | -13.062 | -30.100 | 8.446 | 1.00 | 30.09 | N |
| ATOM | 268 | NH2 | ARG | A | 182 | -11.900 | -28.176 | 7.966 | 1.00 | 33.00 | N |
| ATOM | 269 | N | LEU | A | 183 | -15.415 | -30.609 | 15.975 | 1.00 | 22.21 | N |
| ATOM | 270 | CA | LEU | A | 183 | -15.858 | -31.565 | 16.980 | 1.00 | 20.10 | C |
| ATOM | 271 | C | LEU | A | 183 | -17.289 | -31.265 | 17.408 | 1.00 | 22.94 | C |
| ATOM | 272 | O | LEU | A | 183 | -17.907 | -32.076 | 18.096 | 1.00 | 23.53 | O |
| ATOM | 273 | CB | LEU | A | 183 | -14.918 | -31.577 | 18.182 | 1.00 | 19.03 | C |
| ATOM | 274 | CG | LEU | A | 183 | -13.826 | -32.651 | 18.153 | 1.00 | 24.47 | C |
| ATOM | 275 | CD1 | LEU | A | 183 | -13.119 | -32.696 | 16.809 | 1.00 | 24.47 | C |
| ATOM | 276 | CD2 | LEU | A | 183 | -12.816 | -32.407 | 19.258 | 1.00 | 24.08 | C |
| ATOM | 277 | N | GLU | A | 184 | -17.809 | -30.113 | 16.977 | 1.00 | 23.63 | N |
| ATOM | 278 | CA | GLU | A | 184 | -19.222 | -29.762 | 17.156 | 1.00 | 23.88 | C |
| ATOM | 279 | C | GLU | A | 184 | -20.075 | -29.971 | 15.910 | 1.00 | 22.40 | C |
| ATOM | 280 | O | GLU | A | 184 | -21.110 | -29.330 | 15.791 | 1.00 | 22.93 | O |
| ATOM | 281 | CB | GLU | A | 184 | -19.372 | -28.292 | 17.560 | 1.00 | 23.75 | C |
| ATOM | 282 | CG | GLU | A | 184 | -18.635 | -27.890 | 18.806 | 1.00 | 26.06 | C |
| ATOM | 283 | CD | GLU | A | 184 | -19.139 | -28.631 | 20.019 | 1.00 | 33.11 | C |
| ATOM | 284 | OE1 | GLU | A | 184 | -20.363 | -28.913 | 20.075 | 1.00 | 34.32 | O |
| ATOM | 285 | OE2 | GLU | A | 184 | -18.305 | -28.946 | 20.899 | 1.00 | 34.75 | O |
| ATOM | 286 | N | ASP | A | 185 | -19.651 | -30.834 | 14.989 | 1.00 | 21.89 | N |
| ATOM | 287 | CA | ASP | A | 185 | -20.270 | -30.908 | 13.664 | 1.00 | 25.50 | C |
| ATOM | 288 | C | ASP | A | 185 | -20.452 | -29.523 | 13.066 | 1.00 | 22.06 | C |
| ATOM | 289 | O | ASP | A | 185 | -21.511 | -29.206 | 12.547 | 1.00 | 21.39 | O |
| ATOM | 290 | CB | ASP | A | 185 | -21.637 | -31.606 | 13.701 | 1.00 | 40.42 | C |
| ATOM | 291 | CG | ASP | A | 185 | -21.531 | -33.104 | 13.910 | 1.00 | 51.64 | C |
| ATOM | 292 | OD1 | ASP | A | 185 | -20.401 | -33.647 | 13.831 | 1.00 | 55.89 | O |
| ATOM | 293 | OD2 | ASP | A | 185 | -22.589 | -33.745 | 14.116 | 1.00 | 52.07 | O |
| ATOM | 294 | N | ALA | A | 186 | -19.419 | -28.693 | 13.152 | 1.00 | 25.31 | N |
| ATOM | 295 | CA | ALA | A | 186 | -19.521 | -27.315 | 12.702 | 1.00 | 21.25 | C |
| ATOM | 296 | C | ALA | A | 186 | -18.262 | -26.920 | 11.989 | 1.00 | 18.66 | C |
| ATOM | 297 | O | ALA | A | 186 | -17.353 | -27.723 | 11.805 | 1.00 | 20.79 | O |
| ATOM | 298 | CB | ALA | A | 186 | -19.779 | -26.377 | 13.882 | 1.00 | 18.33 | C |
| ATOM | 299 | N | HIS | A | 187 | -18.199 | -25.658 | 11.603 | 1.00 | 18.96 | N |
| ATOM | 300 | CA | HIS | A | 187 | -17.012 | -25.135 | 10.969 | 1.00 | 17.14 | C |
| ATOM | 301 | C | HIS | A | 187 | -16.810 | -23.710 | 11.439 | 1.00 | 17.01 | C |
| ATOM | 302 | O | HIS | A | 187 | -17.776 | -23.033 | 11.778 | 1.00 | 19.70 | O |
| ATOM | 303 | CB | HIS | A | 187 | -17.156 | -25.191 | 9.450 | 1.00 | 15.59 | C |
| ATOM | 304 | CG | HIS | A | 187 | -18.323 | -24.414 | 8.940 | 1.00 | 15.47 | C |
| ATOM | 305 | ND1 | HIS | A | 187 | -18.281 | -23.048 | 8.761 | 1.00 | 15.92 | N |
| ATOM | 306 | CD2 | HIS | A | 187 | -19.578 | -24.800 | 8.606 | 1.00 | 18.70 | C |
| ATOM | 307 | CE1 | HIS | A | 187 | -19.456 | -22.628 | 8.323 | 1.00 | 18.66 | C |
| ATOM | 308 | NE2 | HIS | A | 187 | -20.261 | -23.671 | 8.223 | 1.00 | 21.90 | N |
| ATOM | 309 | N | LEU | A | 188 | -15.560 | -23.264 | 11.480 | 1.00 | 16.40 | N |
| ATOM | 310 | CA | LEU | A | 188 | -15.256 | -21.859 | 11.698 | 1.00 | 14.33 | C |
| ATOM | 311 | C | LEU | A | 188 | -16.058 | -20.996 | 10.738 | 1.00 | 16.82 | C |
| ATOM | 312 | O | LEU | A | 188 | -16.141 | -21.289 | 9.527 | 1.00 | 15.26 | O |

(continued)

| | | | | | | | | | | | |
|---|---|---|---|---|---|---|---|---|---|---|---|
| ATOM | 313 | CB | LEU | A | 188 | -13.761 | -21.602 | 11.512 | 1.00 | 13.49 | C |
| ATOM | 314 | CG | LEU | A | 188 | -12.853 | -21.922 | 12.696 | 1.00 | 16.50 | C |
| ATOM | 315 | CD1 | LEU | A | 188 | -11.390 | -21.816 | 12.290 | 1.00 | 18.24 | C |
| ATOM | 316 | CD2 | LEU | A | 188 | -13.163 | -20.975 | 13.841 | 1.00 | 15.22 | C |
| ATOM | 317 | N | VAL | A | 189 | -16.613 | -19.915 | 11.271 | 1.00 | 15.19 | N |
| ATOM | 318 | CA | VAL | A | 189 | -17.596 | -19.127 | 10.543 | 1.00 | 13.95 | C |
| ATOM | 319 | C | VAL | A | 189 | -17.130 | -18.784 | 9.129 | 1.00 | 14.59 | C |
| ATOM | 320 | O | VAL | A | 189 | -15.970 | -18.432 | 8.894 | 1.00 | 13.02 | O |
| ATOM | 321 | CB | VAL | A | 189 | -17.957 | -17.836 | 11.316 | 1.00 | 15.19 | C |
| ATOM | 322 | CG1 | VAL | A | 189 | -16.788 | -16.866 | 11.361 | 1.00 | 13.58 | C |
| ATOM | 323 | CG2 | VAL | A | 189 | -19.191 | -17.168 | 10.700 | 1.00 | 19.68 | C |
| ATOM | 324 | N | VAL | A | 190 | -18.041 | -18.966 | 8.183 | 1.00 | 16.77 | N |
| ATOM | 325 | CA | VAL | A | 190 | -17 .814 | -18.617 | 6.786 | 1.00 | 18.83 | C |
| ATOM | 326 | C | VAL | A | 190 | -18.720 | -17.429 | 6.469 | 1.00 | 18.58 | C |
| ATOM | 327 | O | VAL | A | 190 | -19.925 | -17.526 | 6.604 | 1.00 | 17.97 | O |
| ATOM | 328 | CB | VAL | A | 190 | -18.113 | -19.808 | 5.843 | 1.00 | 19.02 | C |
| ATOM | 329 | CG1 | VAL | A | 190 | -18.145 | -19.357 | 4.367 | 1.00 | 16.14 | C |
| ATOM | 330 | CG2 | VAL | A | 190 | -17.092 | -20.917 | 6.052 | 1.00 | 17.67 | C |
| ATOM | 331 | N | VAL | A | 191 | -18.146 | -16.297 | 6.086 | 1.00 | 16.96 | N |
| ATOM | 332 | CA | VAL | A | 191 | -18.956 | -15.094 | 5.909 | 1.00 | 16.61 | C |
| ATOM | 333 | C | VAL | A | 191 | -19.163 | -14.813 | 4.424 | 1.00 | 21.27 | C |
| ATOM | 334 | O | VAL | A | 191 | -18.196 | -14.541 | 3.705 | 1.00 | 19.05 | O |
| ATOM | 335 | CB | VAL | A | 191 | -18.302 | -13.887 | 6.589 | 1.00 | 19.68 | C |
| ATOM | 336 | CG1 | VAL | A | 191 | -19.161 | -12.637 | 6.414 | 1.00 | 16.81 | C |
| ATOM | 337 | CG2 | VAL | A | 191 | -18.073 | -14.187 | 8.076 | 1.00 | 17.57 | C |
| ATOM | 338 | N | THR | A | 192 | -20.410 | -14.895 | 3.960 | 1.00 | 17.29 | N |
| ATOM | 339 | CA | THR | A | 192 | -20.686 | -14.767 | 2.523 | 1.00 | 21.54 | C |
| ATOM | 340 | C | THR | A | 192 | -21.558 | -13.571 | 2.143 | 1.00 | 21.36 | C |
| ATOM | 341 | O | THR | A | 192 | -21.854 | -13.372 | 0.976 | 1.00 | 21.17 | O |
| ATOM | 342 | CB | THR | A | 192 | -21.344 | -16.046 | 1.963 | 1.00 | 19.51 | C |
| ATOM | 343 | OG1 | THR | A | 192 | -22.486 | -16.392 | 2.755 | 1.00 | 23.52 | O |
| ATOM | 344 | CG2 | THR | A | 192 | -20.354 | -17.193 | 2.007 | 1.00 | 19.60 | C |
| ATOM | 345 | N | SER | A | 193 | -21.938 | -12.744 | 3.107 | 1.00 | 18.59 | N |
| ATOM | 346 | CA | SER | A | 193 | -22.678 | -11.549 | 2.755 | 1.00 | 21.89 | C |
| ATOM | 347 | C | SER | A | 193 | -22.432 | -10.451 | 3.763 | 1.00 | 26.64 | C |
| ATOM | 348 | O | SER | A | 193 | -21.913 | -10.693 | 4.856 | 1.00 | 19.28 | O |
| ATOM | 349 | CB | SER | A | 193 | -24.172 | -11.847 | 2.681 | 1.00 | 23.78 | C |
| ATOM | 350 | OG | SER | A | 193 | -24.655 | -12.141 | 3.986 | 1.00 | 23.32 | O |
| ATOM | 351 | N | TRP | A | 194 | -22.825 | -9.241 | 3.378 | 1.00 | 27.24 | N |
| ATOM | 352 | CA | TRP | A | 194 | -22.794 | -8.087 | 4.260 | 1.00 | 24.77 | C |
| ATOM | 353 | C | TRP | A | 194 | -23.633 | -8.331 | 5.504 | 1.00 | 25.57 | C |
| ATOM | 354 | O | TRP | A | 194 | -23.199 | -8.061 | 6.624 | 1.00 | 26.59 | O |
| ATOM | 355 | CB | TRP | A | 194 | -23.295 | -6.845 | 3.519 | 1.00 | 28.68 | C |
| ATOM | 356 | CG | TRP | A | 194 | -23.125 | -5.572 | 4.283 | 1.00 | 38.36 | C |
| ATOM | 357 | CD1 | TRP | A | 194 | -24.116 | -4.747 | 4.741 | 1.00 | 37.90 | C |
| ATOM | 358 | CD2 | TRP | A | 194 | -21.884 | -4.976 | 4.696 | 1.00 | 44.56 | C |
| ATOM | 359 | NE1 | TRP | A | 194 | -23.569 | -3.668 | 5.400 | 1.00 | 43.45 | N |
| ATOM | 360 | CE2 | TRP | A | 194 | -22.202 | -3.785 | 5.388 | 1.00 | 46.98 | C |
| ATOM | 361 | CE3 | TRP | A | 194 | -20.538 | -5.334 | 4.546 | 1.00 | 36.22 | C |
| ATOM | 362 | CZ2 | TRP | A | 194 | -21.219 | -2.949 | 5.932 | 1.00 | 52.52 | C |
| ATOM | 363 | CZ3 | TRP | A | 194 | -19.560 | -4.503 | 5.089 | 1.00 | 42.44 | C |

(continued)

| | | | | | | | | | | | |
|---|---|---|---|---|---|---|---|---|---|---|---|
| ATOM | 364 | CH2 | TRP | A | 194 | -19.908 | -3.325 | 5.773 | 1.00 | 54.05 | C |
| ATOM | 365 | N | GLU | A | 195 | -24.848 | -8.824 | 5.293 | 1.00 | 26.92 | N |
| ATOM | 366 | CA | GLU | A | 195 | -25.764 | -9.112 | 6.385 | 1.00 | 23.47 | C |
| ATOM | 367 | C | GLU | A | 195 | -25.105 | -9.996 | 7.433 | 1.00 | 23.12 | C |
| ATOM | 368 | O | GLU | A | 195 | -25.175 | -9.715 | 8.632 | 1.00 | 28.18 | O |
| ATOM | 369 | CB | GLU | A | 195 | -27.037 | -9.781 | 5.846 | 1.00 | 30.26 | C |
| ATOM | 370 | CG | GLU | A | 195 | -27.820 | -10.627 | 6.876 | 1.00 | 40.78 | C |
| ATOM | 371 | CD | GLU | A | 195 | -27.676 | -12.144 | 6.673 | 1.00 | 40.62 | C |
| ATOM | 372 | OE1 | GLU | A | 195 | -27.945 | -12.645 | 5.559 | 1.00 | 43.62 | O |
| ATOM | 373 | OE2 | GLU | A | 195 | -27.294 | -12.841 | 7.634 | 1.00 | 42.10 | O |
| ATOM | 374 | N | GLU | A | 196 | -24.454 | -11.059 | 6.975 | 1.00 | 20.16 | N |
| ATOM | 375 | CA | GLU | A | 196 | -23.824 | -12.005 | 7.884 | 1.00 | 25.02 | C |
| ATOM | 376 | C | GLU | A | 196 | -22.610 | -11.407 | 8.600 | 1.00 | 21.89 | C |
| ATOM | 377 | O | GLU | A | 196 | -22.377 | -11.689 | 9.775 | 1.00 | 18.44 | O |
| ATOM | 378 | CB | GLU | A | 196 | -23.416 | -13.265 | 7.136 | 1.00 | 20.96 | C |
| ATOM | 379 | CG | GLU | A | 196 | -22.822 | -14.312 | 8.049 | 1.00 | 22.24 | C |
| ATOM | 380 | CD | GLU | A | 196 | -22.589 | -15.613 | 7.337 | 1.00 | 22.15 | C |
| ATOM | 381 | OE1 | GLU | A | 196 | -22.492 | -15.604 | 6.093 | 1.00 | 19.92 | O |
| ATOM | 382 | OE2 | GLU | A | 196 | -22.506 | -16.645 | 8.028 | 1.00 | 24.74 | O |
| ATOM | 383 | N | GLN | A | 197 | -21.851 | -10.584 | 7.881 | 1.00 | 18.28 | N |
| ATOM | 384 | CA | GLN | A | 197 | -20.751 | -9.817 | 8.456 | 1.00 | 18.47 | C |
| ATOM | 385 | C | GLN | A | 197 | -21.217 | -8.914 | 9.600 | 1.00 | 24.91 | C |
| ATOM | 386 | O | GLN | A | 197 | -20.639 | -8.924 | 10.694 | 1.00 | 18.25 | O |
| ATOM | 387 | CB | GLN | A | 197 | -20.084 | -8.973 | 7.373 | 1.00 | 18.68 | C |
| ATOM | 388 | CG | GLN | A | 197 | -19.167 | -7.888 | 7.890 | 1.00 | 23.19 | C |
| ATOM | 389 | CD | GLN | A | 197 | -17.832 | -8.428 | 8.348 | 1.00 | 19.88 | C |
| ATOM | 390 | OE1 | GLN | A | 197 | -17.290 | -9.361 | 7.758 | 1.00 | 19.94 | O |
| ATOM | 391 | NE2 | GLN | A | 197 | -17.289 | -7.840 | 9.406 | 1.00 | 20.73 | N |
| ATOM | 392 | N | LYS | A | 198 | -22.253 | -8.118 | 9.334 | 1.00 | 26.27 | N |
| ATOM | 393 | CA | LYS | A | 198 | -22.775 | -7.197 | 10.330 | 1.00 | 20.44 | C |
| ATOM | 394 | C | LYS | A | 198 | -23.308 | -7.995 | 11.495 | 1.00 | 18.19 | C |
| ATOM | 395 | O | LYS | A | 198 | -23.199 | -7.578 | 12.637 | 1.00 | 20.09 | O |
| ATOM | 396 | CB | LYS | A | 198 | -23.886 | -6.312 | 9.757 | 1.00 | 26.97 | C |
| ATOM | 397 | CG | LYS | A | 198 | -23.441 | -5.226 | 8.806 | 1.00 | 32.65 | C |
| ATOM | 398 | CD | LYS | A | 198 | -24.652 | -4.387 | 8.368 | 1.00 | 47.94 | C |
| ATOM | 399 | CE | LYS | A | 198 | -25.404 | -3.782 | 9.568 | 1.00 | 44 . 86 | C |
| ATOM | 400 | NZ | LYS | A | 198 | -26.475 | -2.822 | 9.154 | 1.00 | 46.87 | N |
| ATOM | 401 | N | PHE | A | 199 | -23.903 | -9.142 | 11.196 | 1.00 | 16.78 | N |
| ATOM | 402 | CA | PHE | A | 199 | -24.438 | -9.981 | 12.248 | 1.00 | 18.71 | C |
| ATOM | 403 | C | PHE | A | 199 | -23.319 | -10.480 | 13.161 | 1.00 | 19.39 | C |
| ATOM | 404 | O | PHE | A | 199 | -23.448 | -10.481 | 14.383 | 1.00 | 19.46 | O |
| ATOM | 405 | CB | PHE | A | 199 | -25.204 | -11.163 | 11.669 | 1.00 | 15.44 | C |
| ATOM | 406 | CG | PHE | A | 199 | -25.498 | -12.218 | 12.675 | 1.00 | 18.84 | C |
| ATOM | 407 | CD2 | PHE | A | 199 | -24.739 | -13.374 | 12.732 | 1.00 | 22.90 | C |
| ATOM | 408 | CD1 | PHE | A | 199 | -26.510 | -12.045 | 13.596 | 1.00 | 23.16 | C |
| ATOM | 409 | CE2 | PHE | A | 199 | -24.998 | -14.339 | 13.686 | 1.00 | 25.42 | C |
| ATOM | 410 | CE1 | PHE | A | 199 | -26.775 | -13.011 | 14.557 | 1.00 | 24.25 | C |
| ATOM | 411 | CZ | PHE | A | 199 | -26.023 | -14.155 | 14.603 | 1.00 | 20.45 | C |
| ATOM | 412 | N | VAL | A | 200 | -22.220 | -10.923 | 12.568 | 1.00 | 20.88 | N |
| ATOM | 413 | CA | VAL | A | 200 | -21.127 | -11.461 | 13.366 | 1.00 | 19.97 | C |
| ATOM | 414 | C | VAL | A | 200 | -20.361 | -10.342 | 14.096 | 1.00 | 19.76 | C |

(continued)

| | | | | | | | | | | | |
|---|---|---|---|---|---|---|---|---|---|---|---|
| ATOM | 415 | O | VAL | A | 200 | -20.037 | -10.494 | 15.267 | 1.00 | 18.57 | O |
| ATOM | 416 | CB | VAL | A | 200 | -20.201 | -12.301 | 12.496 | 1.00 | 20.92 | C |
| ATOM | 417 | CG1 | VAL | A | 200 | -18.898 | -12.658 | 13.243 | 1.00 | 18.59 | C |
| ATOM | 418 | CG2 | VAL | A | 200 | -20.942 | -13.551 | 12.046 | 1.00 | 16.54 | C |
| ATOM | 419 | N | GLN | A | 201 | -20.126 | -9.211 | 13.430 | 1.00 | 19.23 | N |
| ATOM | 420 | CA | GLN | A | 201 | -19.564 | -8.024 | 14.096 | 1.00 | 22.96 | C |
| ATOM | 421 | C | GLN | A | 201 | -20.288 | -7.714 | 15.403 | 1.00 | 25.56 | C |
| ATOM | 422 | O | GLN | A | 201 | -19.649 | -7.493 | 16.437 | 1.00 | 22.68 | O |
| ATOM | 423 | CB | GLN | A | 201 | -19.648 | -6.781 | 13.199 | 1.00 | 23.94 | C |
| ATOM | 424 | CG | GLN | A | 201 | -18.493 | -6.549 | 12.235 | 1.00 | 24.31 | C |
| ATOM | 425 | CD | GLN | A | 201 | -18.865 | -5.570 | 11.096 | 1.00 | 31.70 | C |
| ATOM | 426 | OE1 | GLN | A | 201 | -18.274 | -5.595 | 10.007 | 1.00 | 25.36 | O |
| ATOM | 427 | NE2 | GLN | A | 201 | -19.856 | -4.718 | 11.349 | 1.00 | 28.05 | N |
| ATOM | 428 | N | HIS | A | 202 | -21.625 | -7.687 | 15.349 | 1.00 | 19.95 | N |
| ATOM | 429 | CA | HIS | A | 202 | -22.415 | -7.330 | 16.514 | 1.00 | 20.60 | C |
| ATOM | 430 | C | HIS | A | 202 | -22.126 | -8.228 | 17.715 | 1.00 | 23.45 | C |
| ATOM | 431 | O | HIS | A | 202 | -21.915 | -7.742 | 18.822 | 1.00 | 26.55 | O |
| ATOM | 432 | CB | HIS | A | 202 | -23.906 | -7.382 | 16.207 | 1.00 | 22.12 | C |
| ATOM | 433 | CG | HIS | A | 202 | -24.758 | -7.236 | 17.425 | 1.00 | 21.72 | C |
| ATOM | 434 | ND1 | HIS | A | 202 | -25.123 | -6.010 | 17.931 | 1.00 | 25.57 | N |
| ATOM | 435 | CD2 | HIS | A | 202 | -25.276 | -8.160 | 18.267 | 1.00 | 25.38 | C |
| ATOM | 436 | CE1 | HIS | A | 202 | -25.846 | -6.183 | 19.022 | 1.00 | 23.46 | C |
| ATOM | 437 | NE2 | HIS | A | 202 | -25.958 | -7.481 | 19.244 | 1.00 | 23.52 | N |
| ATOM | 438 | N | HIS | A | 203 | -22.128 | -9.537 | 17.495 | 1.00 | 22.74 | N |
| ATOM | 439 | CA | HIS | A | 203 | -21.920 | -10.478 | 18.584 | 1.00 | 21.81 | C |
| ATOM | 440 | C | HIS | A | 203 | -20.469 | -10.633 | 19.043 | 1.00 | 21.12 | C |
| ATOM | 441 | O | HIS | A | 203 | -20.243 | -11.066 | 20.168 | 1.00 | 31.14 | O |
| ATOM | 442 | CB | HIS | A | 203 | -22.455 | -11.849 | 18.201 | 1.00 | 19.70 | C |
| ATOM | 443 | CG | HIS | A | 203 | -23.947 | -11.937 | 18.209 | 1.00 | 27.48 | C |
| ATOM | 444 | ND1 | HIS | A | 203 | -24.669 | -12.220 | 19.349 | 1.00 | 27.80 | N |
| ATOM | 445 | CD2 | HIS | A | 203 | -24.855 | -11.767 | 17.220 | 1.00 | 22.28 | C |
| ATOM | 446 | CE1 | HIS | A | 203 | -25.958 | -12.223 | 19.061 | 1.00 | 21. 84 | C |
| ATOM | 447 | NE2 | HIS | A | 203 | -26.097 | -11.945 | 17.777 | 1.00 | 20.85 | N |
| ATOM | 448 | N | ILE | A | 204 | -19.487 | -10.319 | 18.206 | 1.00 | 19.01 | N |
| ATOM | 449 | CA | ILE | A | 204 | -18.102 | -10.491 | 18.664 | 1.00 | 24.65 | C |
| ATOM | 450 | C | ILE | A | 204 | -17.506 | -9.213 | 19.247 | 1.00 | 23.37 | C |
| ATOM | 451 | O | ILE | A | 204 | -16.502 | -9.262 | 19.956 | 1.00 | 29.46 | O |
| ATOM | 452 | CB | ILE | A | 204 | -17.156 | -10.992 | 17.550 | 1.00 | 19.60 | C |
| ATOM | 453 | CG1 | ILE | A | 204 | -17.032 | -9.970 | 16.421 | 1.00 | 22.94 | C |
| ATOM | 454 | CG2 | ILE | A | 204 | -17.581 | -12.351 | 17.037 | 1.00 | 18.69 | C |
| ATOM | 455 | CD1 | ILE | A | 204 | -15.981 | -10.357 | 15.400 | 1.00 | 18.85 | C |
| ATOM | 456 | N | GLY | A | 205 | -18.118 | -8.075 | 18.954 | 1.00 | 21.26 | N |
| ATOM | 457 | CA | GLY | A | 205 | -17.663 | -6.811 | 19.504 | 1.00 | 22.73 | C |
| ATOM | 458 | C | GLY | A | 205 | -16.273 | -6.438 | 19.025 | 1.00 | 24.53 | C |
| ATOM | 459 | O | GLY | A | 205 | -15.782 | -6.990 | 18.041 | 1.00 | 29.29 | O |
| ATOM | 460 | N | PRO | A | 206 | -15.614 | -5.508 | 19.729 | 1.00 | 27.53 | N |
| ATOM | 461 | CA | PRO | A | 206 | -14.313 | -5.000 | 19.264 | 1.00 | 25.17 | C |
| ATOM | 462 | C | PRO | A | 206 | -13.141 | -5.982 | 19.496 | 1.00 | 23.26 | C |
| ATOM | 463 | O | PRO | A | 206 | -12.078 | -5.584 | 19.979 | 1.00 | 27.76 | O |
| ATOM | 464 | CB | PRO | A | 206 | -14.137 | -3.729 | 20.092 | 1.00 | 24.03 | C |
| ATOM | 465 | CG | PRO | A | 206 | -14.809 | -4.066 | 21.394 | 1.00 | 21.52 | C |

(continued)

| | | | | | | | | | | | |
|---|---|---|---|---|---|---|---|---|---|---|---|
| ATOM | 466 | CD | PRO | A | 206 | -16.016 | -4.911 | 21.018 | 1.00 | 22.26 | C |
| ATOM | 467 | N | VAL | A | 207 | -13.325 | -7.240 | 19.114 | 1.00 | 21.78 | N |
| ATOM | 468 | CA | VAL | A | 207 | -12.381 | -8.298 | 19.464 | 1.00 | 22.06 | C |
| ATOM | 469 | C | VAL | A | 207 | -11.844 | -9.063 | 18.248 | 1.00 | 21.75 | C |
| ATOM | 470 | O | VAL | A | 207 | -12.602 | -9.464 | 17.361 | 1.00 | 16.31 | O |
| ATOM | 471 | CB | VAL | A | 207 | -13.044 | -9.308 | 20.425 | 1.00 | 18.92 | C |
| ATOM | 472 | CG1 | VAL | A | 207 | -12.057 | -10.360 | 20.865 | 1.00 | 16.73 | C |
| ATOM | 473 | CG2 | VAL | A | 207 | -13.637 | -8.587 | 21.612 | 1.00 | 15.45 | C |
| ATOM | 474 | N | ASN | A | 208 | -10.532 | -9.277 | 18.220 | 1.00 | 20.49 | N |
| ATOM | 475 | CA | ASN | A | 208 | -9.922 | -10.105 | 17.191 | 1.00 | 18.16 | C |
| ATOM | 476 | C | ASN | A | 208 | -10.499 | -11.507 | 17.256 | 1.00 | 15.84 | C |
| ATOM | 477 | O | ASN | A | 208 | -10.423 | -12.147 | 18.294 | 1.00 | 16.63 | O |
| ATOM | 478 | CB | ASN | A | 208 | -8.413 | -10.156 | 17.367 | 1.00 | 16.49 | C |
| ATOM | 479 | CG | ASN | A | 208 | -7.741 | -8.839 | 17.055 | 1.00 | 18.40 | C |
| ATOM | 480 | OD1 | ASN | A | 208 | -6.915 | -8.354 | 17.828 | 1.00 | 21.56 | O |
| ATOM | 481 | ND2 | ASN | A | 208 | -8.067 | -8.269 | 15.912 | 1.00 | 15.53 | N |
| ATOM | 482 | N | THR | A | 209 | -11.073 | -11. 982 | 16.157 | 1.00 | 14.76 | N |
| ATOM | 483 | CA | THR | A | 209 | -11.804 | -13.251 | 16.157 | 1.00 | 13.30 | C |
| ATOM | 484 | C | THR | A | 209 | -11.524 | -14.065 | 14.892 | 1.00 | 14.80 | C |
| ATOM | 485 | O | THR | A | 209 | -11.708 | -13.581 | 13.778 | 1.00 | 13.11 | O |
| ATOM | 486 | CB | THR | A | 209 | -13.330 | -13.001 | 16.288 | 1.00 | 15.05 | C |
| ATOM | 487 | OG1 | THR | A | 209 | -13.573 | -12.186 | 17.436 | 1.00 | 17.23 | O |
| ATOM | 488 | CG2 | THR | A | 209 | -14.102 | -14.310 | 16.450 | 1.00 | 12.52 | C |
| ATOM | 489 | N | TRP | A | 210 | -11.075 | -15.300 | 15.058 | 1.00 | 12.28 | N |
| ATOM | 490 | CA | TRP | A | 210 | -10.804 | -16.146 | 13.905 | 1.00 | 13.62 | C |
| ATOM | 491 | C | TRP | A | 210 | -12.057 | -16.440 | 13.089 | 1.00 | 15.28 | C |
| ATOM | 492 | O | TRP | A | 210 | -13.124 | -16.679 | 13.649 | 1.00 | 14.02 | O |
| ATOM | 493 | CB | TRP | A | 210 | -10.197 | -17.482 | 14.330 | 1.00 | 13.45 | C |
| ATOM | 494 | CG | TRP | A | 210 | -8.783 | -17.472 | 14.852 | 1.00 | 12.79 | C |
| ATOM | 495 | CD1 | TRP | A | 210 | -8.352 | -18.038 | 16.003 | 1.00 | 14.29 | C |
| ATOM | 496 | CD2 | TRP | A | 210 | -7.619 | -16.907 | 14.223 | 1.00 | 12.57 | C |
| ATOM | 497 | NE1 | TRP | A | 210 | -6.992 | -17.868 | 16.141 | 1.00 | 14.57 | N |
| ATOM | 498 | CE2 | TRP | A | 210 | -6.521 | -17.177 | 15.057 | 1.00 | 12.81 | C |
| ATOM | 499 | CE3 | TRP | A | 210 | -7.401 | -16.202 | 13.033 | 1.00 | 13.91 | C |
| ATOM | 500 | CZ2 | TRP | A | 210 | -5.225 | -16.755 | 14.754 | 1.00 | 15.48 | C |
| ATOM | 501 | CZ3 | TRP | A | 210 | -6.107 | -15.791 | 12.729 | 1.00 | 15.74 | C |
| ATOM | 502 | CH2 | TRP | A | 210 | -5.038 | -16.076 | 13.582 | 1.00 | 14.80 | C |
| ATOM | 503 | N | MET | A | 211 | -11.917 | -16.443 | 11.763 | 1.00 | 14.46 | N |
| ATOM | 504 | CA | MET | A | 211 | -12.972 | -16.939 | 10.897 | 1.00 | 13.94 | C |
| ATOM | 505 | C | MET | A | 211 | -12.389 | -18.101 | 10.107 | 1.00 | 18.15 | C |
| ATOM | 506 | O | MET | A | 211 | -11.183 | -18.368 | 10.203 | 1.00 | 14.39 | O |
| ATOM | 507 | CB | MET | A | 211 | -13.511 | -15.843 | 9.973 | 1.00 | 14.29 | C |
| ATOM | 508 | CG | MET | A | 211 | -12.639 | -15.552 | 8.758 | 1.00 | 15.21 | C |
| ATOM | 509 | SD | MET | A | 211 | -13.121 | -14.063 | 7.849 | 1.00 | 15.99 | S |
| ATOM | 510 | CE | MET | A | 211 | -12.617 | -12.803 | 9.002 | 1.00 | 13.06 | C |
| ATOM | 511 | N | GLY | A | 212 | -13.234 | -18.800 | 9.348 | 1.00 | 16.19 | N |
| ATOM | 512 | CA | GLY | A | 212 | -12.811 | -20.001 | 8.645 | 1.00 | 14.73 | C |
| ATOM | 513 | C | GLY | A | 212 | -12.179 | -19.746 | 7.292 | 1.00 | 14.61 | C |
| ATOM | 514 | O | GLY | A | 212 | -12.524 | -20.367 | 6.306 | 1.00 | 14.25 | O |
| ATOM | 515 | N | LEU | A | 213 | -11.231 | -18.824 | 7.260 | 1.00 | 15.62 | N |
| ATOM | 516 | CA | LEU | A | 213 | -10.589 | -18.426 | 6.023 | 1.00 | 13.96 | C |

(continued)

| | | | | | | | | | | | |
|---|---|---|---|---|---|---|---|---|---|---|---|
| ATOM | 517 | C | LEU | A | 213 | -9.080 | -18.461 | 6.261 | 1.00 | 16.13 | C |
| ATOM | 518 | O | LEU | A | 213 | -8.604 | -17.950 | 7.283 | 1.00 | 13.92 | O |
| ATOM | 519 | CB | LEU | A | 213 | -11.069 | -17.041 | 5.609 | 1.00 | 12.59 | C |
| ATOM | 520 | CG | LEU | A | 213 | -10.425 | -16.377 | 4.404 | 1.00 | 13.49 | C |
| ATOM | 521 | CD1 | LEU | A | 213 | -10.715 | -17.173 | 3.139 | 1.00 | 12.34 | C |
| ATOM | 522 | CD2 | LEU | A | 213 | -10.899 | -14.942 | 4.283 | 1.00 | 13.03 | C |
| ATOM | 523 | N | HIS | A | 214 | -8.348 | -19.107 | 5.356 | 1.00 | 14.35 | N |
| ATOM | 524 | CA | HIS | A | 214 | -6.894 | -19.291 | 5.509 | 1.00 | 16.41 | C |
| ATOM | 525 | C | HIS | A | 214 | -6.244 | -19.539 | 4.160 | 1.00 | 18.20 | C |
| ATOM | 526 | O | HIS | A | 214 | -6.940 | -19.874 | 3.207 | 1.00 | 16.31 | O |
| ATOM | 527 | CB | HIS | A | 214 | -6.577 | -20.464 | 6.440 | 1.00 | 13.36 | C |
| ATOM | 528 | CG | HIS | A | 214 | -6.949 | -21.797 | 5.874 | 1.00 | 17.43 | C |
| ATOM | 529 | ND1 | HIS | A | 214 | -6.016 | -22.687 | 5.391 | 1.00 | 23.48 | N |
| ATOM | 530 | CD2 | HIS | A | 214 | -8.158 | -22.382 | 5.690 | 1.00 | 19.34 | C |
| ATOM | 531 | CE1 | HIS | A | 214 | -6.630 | -23.772 | 4.949 | 1.00 | 21.50 | C |
| ATOM | 532 | NE2 | HIS | A | 214 | -7.930 | -23.613 | 5.122 | 1.00 | 22.11 | N |
| ATOM | 533 | N | ASP | A | 215 | -4.920 | -19.376 | 4.079 | 1.00 | 18.22 | N |
| ATOM | 534 | CA | ASP | A | 215 | -4.188 | -19.750 | 2.867 | 1.00 | 17.30 | C |
| ATOM | 535 | C | ASP | A | 215 | -2.925 | -20.539 | 3.240 | 1.00 | 19.55 | C |
| ATOM | 536 | O | ASP | A | 215 | -1.834 | -20.316 | 2.712 | 1.00 | 19.31 | O |
| ATOM | 537 | CB | ASP | A | 215 | -3.849 | -18.504 | 2.029 | 1.00 | 14.78 | C |
| ATOM | 538 | CG | ASP | A | 215 | -2.730 | -17.641 | 2.638 | 1.00 | 17.69 | C |
| ATOM | 539 | OD1 | ASP | A | 215 | -2.483 | -17.688 | 3.869 | 1.00 | 15.15 | O |
| ATOM | 540 | OD2 | ASP | A | 215 | -2.095 | -16.899 | 1.866 | 1.00 | 17.44 | O |
| ATOM | 541 | N | GLN | A | 216 | -3.078 | -21.470 | 4.167 | 1.00 | 21.06 | N |
| ATOM | 542 | CA | GLN | A | 216 | -1.934 | -22.227 | 4.657 | 1.00 | 24.33 | C |
| ATOM | 543 | C | GLN | A | 216 | -1.340 | -23.160 | 3.607 | 1.00 | 21.68 | C |
| ATOM | 544 | O | GLN | A | 216 | -0.150 | -23.452 | 3.649 | 1.00 | 24.43 | O |
| ATOM | 545 | CB | GLN | A | 216 | -2.333 | -23.029 | 5.886 | 1.00 | 23.90 | C |
| ATOM | 546 | CG | GLN | A | 216 | -2.684 | -22.189 | 7.079 | 1.00 | 20.99 | C |
| ATOM | 547 | CD | GLN | A | 216 | -3.202 | -23.036 | 8.215 | 1.00 | 30.17 | C |
| ATOM | 548 | OE1 | GLN | A | 216 | -4.266 | -23.648 | 8.110 | 1.00 | 26.71 | O |
| ATOM | 549 | NE2 | GLN | A | 216 | -2.440 | -23.098 | 9.305 | 1.00 | 40.70 | N |
| ATOM | 550 | N | ASN | A | 217 | -2.162 | -23.621 | 2.668 | 1.00 | 23.32 | N |
| ATOM | 551 | CA | ASN | A | 217 | -1.685 | -24.532 | 1.629 | 1.00 | 20.04 | C |
| ATOM | 552 | C | ASN | A | 217 | -1.612 | -23.852 | 0.283 | 1.00 | 24.86 | C |
| ATOM | 553 | O | ASN | A | 217 | -1.540 | -24.510 | -0.756 | 1.00 | 27.21 | O |
| ATOM | 554 | CB | ASN | A | 217 | -2.588 | -25.759 | 1.517 | 1.00 | 28.11 | C |
| ATOM | 555 | CG | ASN | A | 217 | -2.888 | -26.378 | 2.852 | 1.00 | 32.30 | C |
| ATOM | 556 | OD1 | ASN | A | 217 | -4.055 | -26.547 | 3.218 | 1.00 | 38.88 | O |
| ATOM | 557 | ND2 | ASN | A | 217 | -1.841 | -26.715 | 3.600 | 1.00 | 27.42 | N |
| ATOM | 558 | N | GLY | A | 218 | -1.658 | -22.529 | 0.295 | 1.00 | 22.21 | N |
| ATOM | 559 | CA | GLY | A | 218 | -1.460 | -21.777 | -0.918 | 1.00 | 20.14 | C |
| ATOM | 560 | C | GLY | A | 218 | -2.428 | -20.632 | -1.068 | 1.00 | 22.88 | C |
| ATOM | 561 | O | GLY | A | 218 | -2.165 | -19.536 | -0.571 | 1.00 | 23.78 | O |
| ATOM | 562 | N | PRO | A | 219 | -3.534 | -20.871 | -1.791 | 1.00 | 26.59 | N |
| ATOM | 563 | CA | PRO | A | 219 | -4.537 | -19.838 | -2.062 | 1.00 | 23.67 | C |
| ATOM | 564 | C | PRO | A | 219 | -5.541 | -19.712 | -0.933 | 1.00 | 20.80 | C |
| ATOM | 565 | O | PRO | A | 219 | -5.700 | -20.647 | -0.143 | 1.00 | 21.41 | O |
| ATOM | 566 | CB | PRO | A | 219 | -5.216 | -20.342 | -3.341 | 1.00 | 21.68 | C |
| ATOM | 567 | CG | PRO | A | 219 | -5.134 | -21.823 | -3.228 | 1.00 | 22.20 | C |

(continued)

| | | | | | | | | | | | |
|---|---|---|---|---|---|---|---|---|---|---|---|
| ATOM | 568 | CD | PRO | A | 219 | -3.775 | -22.087 | -2.592 | 1.00 | 27.98 | C |
| ATOM | 569 | N | TRP | A | 220 | -6.210 | -18.564 | -0.867 | 1.00 | 19.23 | N |
| ATOM | 570 | CA | TRP | A | 220 | -7.281 | -18.357 | 0.093 | 1.00 | 20.89 | C |
| ATOM | 571 | C | TRP | A | 220 | -8.394 | -19.370 | -0.119 | 1.00 | 18.11 | C |
| ATOM | 572 | O | TRP | A | 220 | -8.890 | -19.549 | -1.237 | 1.00 | 16.24 | O |
| ATOM | 573 | CB | TRP | A | 220 | -7.818 | -16.924 | 0.000 | 1.00 | 20.98 | C |
| ATOM | 574 | CG | TRP | A | 220 | -6.855 | -15.955 | 0.591 | 1.00 | 19.44 | C |
| ATOM | 575 | CD1 | TRP | A | 220 | -6.071 | -15.058 | -0.079 | 1.00 | 22.05 | C |
| ATOM | 576 | CD2 | TRP | A | 220 | -6.519 | -15.825 | 1.977 | 1.00 | 16.53 | C |
| ATOM | 577 | NE1 | TRP | A | 220 | -5.286 | -14.361 | 0.811 | 1.00 | 14.72 | N |
| ATOM | 578 | CE2 | TRP | A | 220 | -5.539 | -14.815 | 2.077 | 1.00 | 13.97 | C |
| ATOM | 579 | CE3 | TRP | A | 220 | -6.958 | -16.456 | 3.143 | 1.00 | 12.82 | C |
| ATOM | 580 | CZ2 | TRP | A | 220 | -4.997 | -14.422 | 3.295 | 1.00 | 16.02 | C |
| ATOM | 581 | CZ3 | TRP | A | 220 | -6.422 | -16.062 | 4.347 | 1.00 | 15.01 | C |
| ATOM | 582 | CH2 | TRP | A | 220 | -5.449 | -15.060 | 4.418 | 1.00 | 18.72 | C |
| ATOM | 583 | N | LYS | A | 221 | -8.744 | -20.038 | 0.979 | 1.00 | 18.43 | N |
| ATOM | 584 | CA | LYS | A | 221 | -9.784 | -21.059 | 1.030 | 1.00 | 20.12 | C |
| ATOM | 585 | C | LYS | A | 221 | -10.651 | -20.866 | 2.269 | 1.00 | 17.14 | C |
| ATOM | 586 | O | LYS | A | 221 | -10.133 | -20.648 | 3.354 | 1.00 | 17.85 | O |
| ATOM | 587 | CB | LYS | A | 221 | -9.169 | -22.463 | 1.059 | 1.00 | 22.71 | C |
| ATOM | 588 | CG | LYS | A | 221 | -8.599 | -22.928 | -0.263 | 1.00 | 30.31 | C |
| ATOM | 589 | CD | LYS | A | 221 | -7.862 | -24.250 | -0.113 | 1.00 | 39.91 | C |
| ATOM | 590 | CE | LYS | A | 221 | -7.419 | -24.776 | -1.471 | 1.00 | 44.01 | C |
| ATOM | 591 | NZ | LYS | A | 221 | -6.601 | -26.016 | -1.336 | 1.00 | 58.69 | N |
| ATOM | 592 | N | TRP | A | 222 | -11.965 | -20.938 | 2.096 | 1.00 | 20.05 | N |
| ATOM | 593 | CA | TRP | A | 222 | -12.893 | -21.059 | 3.220 | 1.00 | 18.07 | C |
| ATOM | 594 | C | TRP | A | 222 | -12.973 | -22.520 | 3.658 | 1.00 | 15.25 | C |
| ATOM | 595 | O | TRP | A | 222 | -12.883 | -23.404 | 2.821 | 1.00 | 22.45 | O |
| ATOM | 596 | CB | TRP | A | 222 | -14.280 | -20.553 | 2.827 | 1.00 | 18.47 | C |
| ATOM | 597 | CG | TRP | A | 222 | -14.383 | -19.074 | 2.639 | 1.00 | 15.11 | C |
| ATOM | 598 | CD1 | TRP | A | 222 | -14.384 | -18.397 | 1.458 | 1.00 | 15.59 | C |
| ATOM | 599 | CD2 | TRP | A | 222 | -14.536 | -18.090 | 3.667 | 1.00 | 12.30 | C |
| ATOM | 600 | NE1 | TRP | A | 222 | -14.525 | -17.045 | 1.686 | 1.00 | 17.01 | N |
| ATOM | 601 | CE2 | TRP | A | 222 | -14.621 | -16.834 | 3.037 | 1.00 | 14.83 | C |
| ATOM | 602 | CE3 | TRP | A | 222 | -14.607 | -18.148 | 5.058 | 1.00 | 15.63 | C |
| ATOM | 603 | CZ2 | TRP | A | 222 | -14.774 | -15.651 | 3.749 | 1.00 | 14.19 | C |
| ATOM | 604 | CZ3 | TRP | A | 222 | -14.750 | -16.975 | 5.761 | 1.00 | 14 . 82 | C |
| ATOM | 605 | CH2 | TRP | A | 222 | -14 . 833 | -15.741 | 5.103 | 1.00 | 16.93 | C |
| ATOM | 606 | N | VAL | A | 223 | -13.160 | -22.781 | 4.948 | 1.00 | 18.67 | N |
| ATOM | 607 | CA | VAL | A | 223 | -13.104 | -24.154 | 5.453 | 1.00 | 21.37 | C |
| ATOM | 608 | C | VAL | A | 223 | -14.214 | -25.092 | 4.955 | 1.00 | 19.54 | C |
| ATOM | 609 | O | VAL | A | 223 | -14.002 | -26.293 | 4.883 | 1.00 | 26.83 | O |
| ATOM | 610 | CB | VAL | A | 223 | -13.127 | -24.195 | 7.000 | 1.00 | 20.27 | C |
| ATOM | 611 | CG1 | VAL | A | 223 | -11.823 | -23.684 | 7.550 | 1.00 | 16.93 | C |
| ATOM | 612 | CG2 | VAL | A | 223 | -14.323 | -23.424 | 7.546 | 1.00 | 13.22 | C |
| ATOM | 613 | N | ASP | A | 224 | -15.391 | -24.575 | 4.627 | 1.00 | 20.49 | N |
| ATOM | 614 | CA | ASP | A | 224 | -16.455 | -25.453 | 4.129 | 1.00 | 28.05 | C |
| ATOM | 615 | C | ASP | A | 224 | -16.450 | -25.610 | 2.592 | 1.00 | 31.47 | C |
| ATOM | 616 | O | ASP | A | 224 | -17.344 | -26.231 | 2.017 | 1.00 | 34.41 | O |
| ATOM | 617 | CB | ASP | A | 224 | -17 . 832 | -24.956 | 4.606 | 1.00 | 26.17 | C |
| ATOM | 618 | CG | ASP | A | 224 | -18.313 | -23.720 | 3.861 | 1.00 | 30.40 | C |

(continued)

| | | | | | | | | | | | |
|---|---|---|---|---|---|---|---|---|---|---|---|
| ATOM | 619 | OD1 | ASP | A | 224 | -17.480 | -22.971 | 3.288 | 1.00 | 27.86 | O |
| ATOM | 620 | OD2 | ASP | A | 224 | -19.548 | -23.486 | 3.866 | 1.00 | 41.83 | O |
| ATOM | 621 | N | GLY | A | 225 | -15.442 | -25.060 | 1.930 | 1.00 | 26.37 | N |
| ATOM | 622 | CA | GLY | A | 225 | -15.325 | -25.234 | 0.495 | 1.00 | 24.35 | C |
| ATOM | 623 | C | GLY | A | 225 | -15.828 | -24.060 | -0.325 | 1.00 | 26.11 | C |
| ATOM | 624 | O | GLY | A | 225 | -15.605 | -24.021 | -1.532 | 1.00 | 30.45 | O |
| ATOM | 625 | N | THR | A | 226 | -16.512 | -23.114 | 0.322 | 1.00 | 28.38 | N |
| ATOM | 626 | CA | THR | A | 226 | -16.991 | -21.894 | -0.346 | 1.00 | 26.57 | C |
| ATOM | 627 | C | THR | A | 226 | -15.853 | -21.219 | -1.107 | 1.00 | 27.23 | C |
| ATOM | 628 | O | THR | A | 226 | -14.732 | -21.105 | -0.602 | 1.00 | 25.68 | O |
| ATOM | 629 | CB | THR | A | 226 | -17.587 | -20.886 | 0.669 | 1.00 | 26.04 | C |
| ATOM | 630 | OG1 | THR | A | 226 | -18.644 | -21.511 | 1.408 | 1.00 | 31.78 | O |
| ATOM | 631 | CG2 | THR | A | 226 | -18.109 | -19.649 | -0.025 | 1.00 | 22.83 | C |
| ATOM | 632 | N | ASP | A | 227 | -16.131 | -20.791 | -2.331 | 1.00 | 25.74 | N |
| ATOM | 633 | CA | ASP | A | 227 | -15.101 | -20.198 | -3.157 | 1.00 | 24.58 | C |
| ATOM | 634 | C | ASP | A | 227 | -14.769 | -18.774 | -2.724 | 1.00 | 27.29 | C |
| ATOM | 635 | O | ASP | A | 227 | -15.658 | -17.943 | -2.523 | 1.00 | 23.84 | O |
| ATOM | 636 | CB | ASP | A | 227 | -15.523 | -20.199 | -4.625 | 1.00 | 31.00 | C |
| ATOM | 637 | CG | ASP | A | 227 | -14.712 | -19.226 | -5.447 | 1.00 | 33.32 | C |
| ATOM | 638 | OD1 | ASP | A | 227 | -13.509 | -19.502 | -5.653 | 1.00 | 36.87 | O |
| ATOM | 639 | OD2 | ASP | A | 227 | -15.261 | -18.175 | -5.849 | 1.00 | 32.69 | O |
| ATOM | 640 | N | TYR | A | 228 | -13.480 | -18.485 | -2.609 | 1.00 | 25.74 | N |
| ATOM | 641 | CA | TYR | A | 228 | -13.055 | -17.195 | -2.090 | 1.00 | 22.35 | C |
| ATOM | 642 | C | TYR | A | 228 | -13.186 | -16.074 | -3.109 | 1.00 | 22.96 | C |
| ATOM | 643 | O | TYR | A | 228 | -13.706 | -15.006 | -2.779 | 1.00 | 21.88 | O |
| ATOM | 644 | CB | TYR | A | 228 | -11.610 | -17.272 | -1.590 | 1.00 | 17.43 | C |
| ATOM | 645 | CG | TYR | A | 228 | -10.989 | -15.928 | -1.261 | 1.00 | 18.06 | C |
| ATOM | 646 | CD2 | TYR | A | 228 | -10.176 | -15.269 | -2.181 | 1.00 | 19.30 | C |
| ATOM | 647 | CD1 | TYR | A | 228 | -11.208 | -15.316 | -0.019 | 1.00 | 18.53 | C |
| ATOM | 648 | CE2 | TYR | A | 228 | -9.602 | -14.021 | -1.886 | 1.00 | 18.76 | C |
| ATOM | 649 | CE1 | TYR | A | 228 | -10.630 | -14 . 081 | 0.293 | 1.00 | 15.99 | C |
| ATOM | 650 | CZ | TYR | A | 228 | -9.826 | -13.436 | -0.643 | 1.00 | 20.11 | C |
| ATOM | 651 | OH | TYR | A | 228 | -9.245 | -12.212 | -0.342 | 1.00 | 16.25 | O |
| ATOM | 652 | N | GLU | A | 229 | -12.717 | -16.293 | -4.339 | 1.00 | 24.84 | N |
| ATOM | 653 | CA | GLU | A | 229 | -12.560 | -15.153 | -5.244 | 1.00 | 26.92 | C |
| ATOM | 654 | C | GLU | A | 229 | -13.890 | -14.532 | -5.653 | 1.00 | 27.92 | C |
| ATOM | 655 | O | GLU | A | 229 | -13.958 | -13.314 | -5.816 | 1.00 | 23.76 | O |
| ATOM | 656 | CB | GLU | A | 229 | -11.744 | -15.529 | -6.482 | 1.00 | 31.64 | C |
| ATOM | 657 | CG | GLU | A | 229 | -12.229 | -16.734 | -7.265 | 1.00 | 42.79 | C |
| ATOM | 658 | CD | GLU | A | 229 | -11.112 | -17.346 | -8.115 | 1.00 | 43.21 | C |
| ATOM | 659 | OE1 | GLU | A | 229 | -10.142 | -16.627 | -8.448 | 1.00 | 39.47 | O |
| ATOM | 660 | OE2 | GLU | A | 229 | -11.196 | -18.552 | -8.440 | 1.00 | 52.24 | O |
| ATOM | 661 | N | THR | A | 230 | -14.945 | -15.341 | -5.782 | 1.00 | 26.03 | N |
| ATOM | 662 | CA | THR | A | 230 | -16.276 | -14.802 | -6.087 | 1.00 | 26.83 | C |
| ATOM | 663 | C | THR | A | 230 | -17.120 | -14.519 | -4.847 | 1.00 | 30.75 | C |
| ATOM | 664 | O | THR | A | 230 | -18.233 | -14.006 | -4.945 | 1.00 | 30.18 | O |
| ATOM | 665 | CB | THR | A | 230 | -17.090 | -15.754 | -6.977 | 1.00 | 27.80 | C |
| ATOM | 666 | OG1 | THR | A | 230 | -17.113 | -17.052 | -6.378 | 1.00 | 25.69 | O |
| ATOM | 667 | CG2 | THR | A | 230 | -16.472 | -15.855 | -8.362 | 1.00 | 25.73 | C |
| ATOM | 668 | N | GLY | A | 231 | -16.610 | -14.872 | -3.678 | 1.00 | 25.26 | N |
| ATOM | 669 | CA | GLY | A | 231 | -17.362 | -14.653 | -2.463 | 1.00 | 27.26 | C |

(continued)

| | | | | | | | | | | | |
|---|---|---|---|---|---|---|---|---|---|---|---|
| ATOM | 670 | C | GLY | A | 231 | -17.154 | -13.274 | -1.878 | 1.00 | 26.63 | C |
| ATOM | 671 | O | GLY | A | 231 | -16.290 | -12.519 | -2.321 | 1.00 | 25.15 | O |
| ATOM | 672 | N | PHE | A | 232 | -17.973 | -12.959 | -0.882 | 1.00 | 23.85 | N |
| ATOM | 673 | CA | PHE | A | 232 | -17 . 839 | -11.763 | -0.056 | 1.00 | 25.36 | C |
| ATOM | 674 | C | PHE | A | 232 | -16.433 | -11.627 | 0.530 | 1.00 | 22.51 | C |
| ATOM | 675 | O | PHE | A | 232 | -15.815 | -12.623 | 0.898 | 1.00 | 24.64 | O |
| ATOM | 676 | CB | PHE | A | 232 | -18.875 | -11.820 | 1.062 | 1.00 | 23.46 | C |
| ATOM | 677 | CG | PHE | A | 232 | -18.859 | -10.645 | 1.971 | 1.00 | 24.65 | C |
| ATOM | 678 | CD1 | PHE | A | 232 | -19.395 | -9.429 | 1.564 | 1.00 | 19.49 | C |
| ATOM | 679 | CD2 | PHE | A | 232 | -18.354 | -10.760 | 3.254 | 1.00 | 17.94 | C |
| ATOM | 680 | CE1 | PHE | A | 232 | -19.391 | -8.337 | 2.414 | 1.00 | 21.52 | C |
| ATOM | 681 | CE2 | PHE | A | 232 | -18.350 | -9.671 | 4.102 | 1.00 | 20.16 | C |
| ATOM | 682 | CZ | PHE | A | 232 | -18.870 | -8.457 | 3.678 | 1.00 | 22.04 | C |
| ATOM | 683 | N | LYS | A | 233 | -15.923 | -10.400 | 0.576 | 1.00 | 24.28 | N |
| ATOM | 684 | CA | LYS | A | 233 | -14.642 | -10.099 | 1.224 | 1.00 | 22.92 | C |
| ATOM | 685 | C | LYS | A | 233 | -14.782 | -8.799 | 1.978 | 1.00 | 22.84 | C |
| ATOM | 686 | O | LYS | A | 233 | -15.471 | -7.891 | 1.516 | 1.00 | 22.56 | O |
| ATOM | 687 | CB | LYS | A | 233 | -13.495 | -9.991 | 0.212 | 1.00 | 17.41 | C |
| ATOM | 688 | CG | LYS | A | 233 | -13.227 | -11.274 | -0.560 | 1.00 | 20.00 | C |
| ATOM | 689 | CD | LYS | A | 233 | -12.285 | -11.032 | -1.710 | 1.00 | 21.13 | C |
| ATOM | 690 | CE | LYS | A | 233 | -12.419 | -12.105 | -2.767 | 1.00 | 26.26 | C |
| ATOM | 691 | NZ | LYS | A | 233 | -13.753 | -12.082 | -3.453 | 1.00 | 23.06 | N |
| ATOM | 692 | N | ASN | A | 234 | -14.132 | -8.705 | 3.134 | 1.00 | 17.57 | N |
| ATOM | 693 | CA | ASN | A | 234 | -14.165 | -7.467 | 3.910 | 1.00 | 21.38 | C |
| ATOM | 694 | C | ASN | A | 234 | -12.791 | -7.112 | 4.486 | 1.00 | 19.87 | C |
| ATOM | 695 | O | ASN | A | 234 | -12.683 | -6.685 | 5.629 | 1.00 | 23.24 | O |
| ATOM | 696 | CB | ASN | A | 234 | -15.212 | -7.571 | 5.033 | 1.00 | 18.47 | C |
| ATOM | 697 | CG | ASN | A | 234 | -15.554 | -6.222 | 5.643 | 1.00 | 19.07 | C |
| ATOM | 698 | OD1 | ASN | A | 234 | -15.762 | -6.106 | 6.848 | 1.00 | 22.84 | O |
| ATOM | 699 | ND2 | ASN | A | 234 | -15.579 | -5.191 | 4.817 | 1.00 | 21.10 | N |
| ATOM | 700 | N | TRP | A | 235 | -11.742 | -7.311 | 3.697 | 1.00 | 19.16 | N |
| ATOM | 701 | CA | TRP | A | 235 | -10.390 | -6.975 | 4.135 | 1.00 | 22.32 | C |
| ATOM | 702 | C | TRP | A | 235 | -10.264 | -5.498 | 4.467 | 1.00 | 20.90 | C |
| ATOM | 703 | O | TRP | A | 235 | -10.730 | -4.642 | 3.719 | 1.00 | 27.44 | O |
| ATOM | 704 | CB | TRP | A | 235 | -9.354 | -7.319 | 3.063 | 1.00 | 20.49 | C |
| ATOM | 705 | CG | TRP | A | 235 | -9.281 | -8.753 | 2.684 | 1.00 | 20.76 | C |
| ATOM | 706 | CD1 | TRP | A | 235 | -9.712 | -9.314 | 1.523 | 1.00 | 19.71 | C |
| ATOM | 707 | CD2 | TRP | A | 235 | -8.736 | -9.816 | 3.467 | 1.00 | 18.48 | C |
| ATOM | 708 | NE1 | TRP | A | 235 | -9.475 | -10.663 | 1.532 | 1.00 | 18.33 | N |
| ATOM | 709 | CE2 | TRP | A | 235 | -8.869 | -10.998 | 2.713 | 1.00 | 17.93 | C |
| ATOM | 710 | CE3 | TRP | A | 235 | -8.135 | -9.883 | 4.730 | 1.00 | 17.04 | C |
| ATOM | 711 | CZ2 | TRP | A | 235 | -8.427 | -12.236 | 3.180 | 1.00 | 18.19 | C |
| ATOM | 712 | CZ3 | TRP | A | 235 | -7.703 | -11.102 | 5.193 | 1.00 | 14.22 | C |
| ATOM | 713 | CH2 | TRP | A | 235 | -7.855 | -12.269 | 4.423 | 1.00 | 16.87 | C |
| ATOM | 714 | N | ARG | A | 236 | -9.630 | -5.198 | 5.585 | 1.00 | 23.85 | N |
| ATOM | 715 | CA | ARG | A | 236 | -9.169 | -3.842 | 5.826 | 1.00 | 25.84 | C |
| ATOM | 716 | C | ARG | A | 236 | -8.260 | -3.466 | 4.669 | 1.00 | 25.73 | C |
| ATOM | 717 | O | ARG | A | 236 | -7.521 | -4.318 | 4.166 | 1.00 | 27.18 | O |
| ATOM | 718 | CB | ARG | A | 236 | -8.428 | -3.743 | 7.159 | 1.00 | 24.94 | C |
| ATOM | 719 | CG | ARG | A | 236 | -8.108 | -2.312 | 7.586 | 1.00 | 32.25 | C |
| ATOM | 720 | CD | ARG | A | 236 | -7.436 | -2.288 | 8.947 | 1.00 | 30.42 | C |

(continued)

| | | | | | | | | | | | |
|---|---|---|---|---|---|---|---|---|---|---|---|
| ATOM | 721 | NE | ARG | A | 236 | -6.217 | -3.084 | 8.934 | 1.00 | 38.54 | N |
| ATOM | 722 | CZ | ARG | A | 236 | -5.711 | -3.700 | 10.000 | 1.00 | 39.09 | C |
| ATOM | 723 | NH1 | ARG | A | 236 | -6.327 | -3.608 | 11.176 | 1.00 | 39.28 | N |
| ATOM | 724 | NH2 | ARG | A | 236 | -4.588 | -4.409 | 9.889 | 1.00 | 27.72 | N |
| ATOM | 725 | N | PRO | A | 237 | -8.333 | -2.210 | 4.212 | 1.00 | 25.65 | N |
| ATOM | 726 | CA | PRO | A | 237 | -7.412 | -1.747 | 3.167 | 1.00 | 31.88 | C |
| ATOM | 727 | C | PRO | A | 237 | -5.951 | -2.048 | 3.497 | 1.00 | 27.32 | C |
| ATOM | 728 | O | PRO | A | 237 | -5.531 | -1.839 | 4.638 | 1.00 | 28.86 | O |
| ATOM | 729 | CB | PRO | A | 237 | -7.678 | -0.242 | 3.122 | 1.00 | 33.60 | C |
| ATOM | 730 | CG | PRO | A | 237 | -9.149 | -0.156 | 3.436 | 1.00 | 32.49 | C |
| ATOM | 731 | CD | PRO | A | 237 | -9.367 | -1.203 | 4.515 | 1.00 | 29.17 | C |
| ATOM | 732 | N | GLU | A | 238 | -5.231 | -2.563 | 2.501 | 1.00 | 21.38 | N |
| ATOM | 733 | CA | GLU | A | 238 | -3.843 | -3.022 | 2.608 | 1.00 | 23.04 | C |
| ATOM | 734 | C | GLU | A | 238 | -3.741 | -4.424 | 3.231 | 1.00 | 19.36 | C |
| ATOM | 735 | O | GLU | A | 238 | -2.648 | -4.901 | 3.526 | 1.00 | 20.85 | O |
| ATOM | 736 | CB | GLU | A | 238 | -2.978 | -2.022 | 3.397 | 1.00 | 26.89 | C |
| ATOM | 737 | CG | GLU | A | 238 | -2.828 | -0.651 | 2.740 | 1.00 | 31.80 | C |
| ATOM | 738 | CD | GLU | A | 238 | -2.337 | 0.424 | 3.713 | 1.00 | 51.15 | C |
| ATOM | 739 | OE1 | GLU | A | 238 | -2.205 | 0.128 | 4.926 | 1.00 | 50.89 | O |
| ATOM | 740 | OE2 | GLU | A | 238 | -2.101 | 1.573 | 3.269 | 1.00 | 56.05 | O |
| ATOM | 741 | N | GLN | A | 239 | -4.872 | -5.089 | 3.427 | 1.00 | 20.11 | N |
| ATOM | 742 | CA | GLN | A | 239 | -4.840 | -6.495 | 3.825 | 1.00 | 21.60 | C |
| ATOM | 743 | C | GLN | A | 239 | -5.501 | -7.317 | 2.736 | 1.00 | 19.53 | C |
| ATOM | 744 | O | GLN | A | 239 | -6.332 | -6.792 | 2.011 | 1.00 | 21.20 | O |
| ATOM | 745 | CB | GLN | A | 239 | -5.543 | -6.726 | 5.166 | 1.00 | 17.31 | C |
| ATOM | 746 | CG | GLN | A | 239 | -5.020 | -5.893 | 6.310 | 1.00 | 21.03 | C |
| ATOM | 747 | CD | GLN | A | 239 | -3.565 | -6.156 | 6.618 | 1.00 | 18.65 | C |
| ATOM | 748 | OE1 | GLN | A | 239 | -3.045 | -7.232 | 6.343 | 1.00 | 17.67 | O |
| ATOM | 749 | NE2 | GLN | A | 239 | -2.897 | -5.165 | 7.184 | 1.00 | 18.18 | N |
| ATOM | 750 | N | PRO | A | 240 | -5.132 | -8.601 | 2.601 | 1.00 | 16.46 | N |
| ATOM | 751 | CA | PRO | A | 240 | -4.108 | -9.345 | 3.337 | 1.00 | 20.08 | C |
| ATOM | 752 | C | PRO | A | 240 | -2.696 | -8.938 | 2.901 | 1.00 | 19.97 | C |
| ATOM | 753 | O | PRO | A | 240 | -2.486 | -8.679 | 1.708 | 1.00 | 19.33 | O |
| ATOM | 754 | CB | PRO | A | 240 | -4.412 | -10.806 | 2.966 | 1.00 | 15.73 | C |
| ATOM | 755 | CG | PRO | A | 240 | -4.999 | -10.714 | 1.622 | 1.00 | 20.40 | C |
| ATOM | 756 | CD | PRO | A | 240 | -5.783 | -9.438 | 1.580 | 1.00 | 18.17 | C |
| ATOM | 757 | N | ASP | A | 241 | -1.744 | -8.882 | 3.830 | 1.00 | 16.04 | N |
| ATOM | 758 | CA | ASP | A | 241 | -0.399 | -8.446 | 3.472 | 1.00 | 12.88 | C |
| ATOM | 759 | C | ASP | A | 241 | 0.663 | -9.491 | 3.732 | 1.00 | 12.48 | C |
| ATOM | 760 | O | ASP | A | 241 | 1.842 | -9.234 | 3.490 | 1.00 | 15.24 | O |
| ATOM | 761 | CB | ASP | A | 241 | -0.035 | -7.164 | 4.212 | 1.00 | 15.52 | C |
| ATOM | 762 | CG | ASP | A | 241 | -0.053 | -7.332 | 5.733 | 1.00 | 19.38 | C |
| ATOM | 763 | OD1 | ASP | A | 241 | -0.451 | -8.416 | 6.231 | 1.00 | 19.33 | O |
| ATOM | 764 | OD2 | ASP | A | 241 | 0.293 | -6.358 | 6.430 | 1.00 | 17.43 | O |
| ATOM | 765 | N | ASP | A | 242 | 0.249 | -10.652 | 4.237 | 1.00 | 17.41 | N |
| ATOM | 766 | CA | ASP | A | 242 | 1.166 | -11.774 | 4.527 | 1.00 | 16.54 | C |
| ATOM | 767 | C | ASP | A | 242 | 2.465 | -11.313 | 5.190 | 1.00 | 16.20 | C |
| ATOM | 768 | O | ASP | A | 242 | 3.572 | -11.552 | 4.700 | 1.00 | 18.85 | O |
| ATOM | 769 | CB | ASP | A | 242 | 1.487 | -12.530 | 3.261 | 1.00 | 16.74 | C |
| ATOM | 770 | CG | ASP | A | 242 | 2.053 | -13.888 | 3.538 | 1.00 | 21.40 | C |
| ATOM | 771 | OD1 | ASP | A | 242 | 1.481 | -14.576 | 4.413 | 1.00 | 17.68 | O |

(continued)

| | | | | | | | | | | | |
|---|---|---|---|---|---|---|---|---|---|---|---|
| ATOM | 772 | OD2 | ASP | A | 242 | 3.066 | -14.253 | 2.887 | 1.00 | 16.85 | O |
| ATOM | 773 | N | TRP | A | 243 | 2.301 | -10.617 | 6.299 | 1.00 | 15.83 | N |
| ATOM | 774 | CA | TRP | A | 243 | 3.382 | -9.929 | 6.960 | 1.00 | 16.80 | C |
| ATOM | 775 | C | TRP | A | 243 | 4.390 | -10.913 | 7.545 | 1.00 | 16.03 | C |
| ATOM | 776 | O | TRP | A | 243 | 4.020 | -11.905 | 8.153 | 1.00 | 15.06 | O |
| ATOM | 777 | CB | TRP | A | 243 | 2.799 | -9.041 | 8.043 | 1.00 | 19.08 | C |
| ATOM | 778 | CG | TRP | A | 243 | 3.727 | -8.029 | 8.570 | 1.00 | 24.75 | C |
| ATOM | 779 | CD1 | TRP | A | 243 | 4.591 | -7.248 | 7.857 | 1.00 | 21.85 | C |
| ATOM | 780 | CD2 | TRP | A | 243 | 3.888 | -7.665 | 9.942 | 1.00 | 19.00 | C |
| ATOM | 781 | NE1 | TRP | A | 243 | 5.274 | -6.417 | 8.707 | 1.00 | 20.11 | N |
| ATOM | 782 | CE2 | TRP | A | 243 | 4.858 | -6.653 | 9.993 | 1.00 | 17.79 | C |
| ATOM | 783 | CE3 | TRP | A | 243 | 3.296 | -8.092 | 11.133 | 1.00 | 19.98 | C |
| ATOM | 784 | CZ2 | TRP | A | 243 | 5.259 | -6.067 | 11.189 | 1.00 | 19.26 | C |
| ATOM | 785 | CZ3 | TRP | A | 243 | 3.697 | -7.508 | 12.321 | 1.00 | 19.32 | C |
| ATOM | 786 | CH2 | TRP | A | 243 | 4.666 | -6.508 | 12.338 | 1.00 | 14.64 | C |
| ATOM | 787 | N | TYR | A | 244 | 5.671 | -10.643 | 7.350 | 1.00 | 19.42 | N |
| ATOM | 788 | CA | TYR | A | 244 | 6.694 | -11.486 | 7.945 | 1.00 | 20.57 | C |
| ATOM | 789 | C | TYR | A | 244 | 7.295 | -10.760 | 9.140 | 1.00 | 19.06 | C |
| ATOM | 790 | O | TYR | A | 244 | 8.022 | -11.353 | 9.930 | 1.00 | 17.76 | O |
| ATOM | 791 | CB | TYR | A | 244 | 7.784 | -11.833 | 6.932 | 1.00 | 18.60 | C |
| ATOM | 792 | CG | TYR | A | 244 | 7.501 | -13.035 | 6.056 | 1.00 | 24.25 | C |
| ATOM | 793 | CD1 | TYR | A | 244 | 6.568 | -12.970 | 5.020 | 1.00 | 17.33 | C |
| ATOM | 794 | CD2 | TYR | A | 244 | 8.189 | -14.228 | 6.244 | 1.00 | 24.36 | C |
| ATOM | 795 | CE1 | TYR | A | 244 | 6.321 | -14.070 | 4.203 | 1.00 | 18.22 | C |
| ATOM | 796 | CE2 | TYR | A | 244 | 7.945 | -15.342 | 5.436 | 1.00 | 21.75 | C |
| ATOM | 797 | CZ | TYR | A | 244 | 7.016 | -15.257 | 4.415 | 1.00 | 19.74 | C |
| ATOM | 798 | OH | TYR | A | 244 | 6.776 | -16.367 | 3.620 | 1.00 | 14.89 | O |
| ATOM | 799 | N | GLY | A | 245 | 6.958 | -9.478 | 9.265 | 1.00 | 19.02 | N |
| ATOM | 800 | CA | GLY | A | 245 | 7.658 | -8.568 | 10.152 | 1.00 | 20.36 | C |
| ATOM | 801 | C | GLY | A | 245 | 7.367 | -8.768 | 11.621 | 1.00 | 19.87 | C |
| ATOM | 802 | O | GLY | A | 245 | 7.941 | -8.086 | 12.475 | 1.00 | 21.29 | O |
| ATOM | 803 | N | HIS | A | 246 | 6.463 | -9.696 | 11.910 | 1.00 | 19.33 | N |
| ATOM | 804 | CA | HIS | A | 246 | 6.150 | -10.062 | 13.277 | 1.00 | 17.48 | C |
| ATOM | 805 | C | HIS | A | 246 | 7.284 | -10.899 | 13.844 | 1.00 | 18.02 | C |
| ATOM | 806 | O | HIS | A | 246 | 7.418 | -11.015 | 15.053 | 1.00 | 22.65 | O |
| ATOM | 807 | CB | HIS | A | 246 | 4.825 | -10.823 | 13.340 | 1.00 | 15.31 | C |
| ATOM | 808 | CG | HIS | A | 246 | 4.719 | -11.907 | 12.316 | 1.00 | 19.52 | C |
| ATOM | 809 | ND1 | HIS | A | 246 | 5.326 | -13.134 | 12.467 | 1.00 | 16.40 | N |
| ATOM | 810 | CD2 | HIS | A | 246 | 4.101 | -11.937 | 11.112 | 1.00 | 16.23 | C |
| ATOM | 811 | CE1 | HIS | A | 246 | 5.085 | -13.875 | 11.400 | 1.00 | 17.63 | C |
| ATOM | 812 | NE2 | HIS | A | 246 | 4.343 | -13.172 | 10.564 | 1.00 | 16.33 | N |
| ATOM | 813 | N | GLY | A | 247 | 8.098 | -11.482 | 12.968 | 1.00 | 18.78 | N |
| ATOM | 814 | CA | GLY | A | 247 | 9.295 | -12.193 | 13.394 | 1.00 | 17.32 | C |
| ATOM | 815 | C | GLY | A | 247 | 9.030 | -13.544 | 14.022 | 1.00 | 19.13 | C |
| ATOM | 816 | O | GLY | A | 247 | 9.904 | -14.116 | 14.677 | 1.00 | 21.71 | O |
| ATOM | 817 | N | LEU | A | 248 | 7.828 | -14.074 | 13.811 | 1.00 | 20.58 | N |
| ATOM | 818 | CA | LEU | A | 248 | 7.427 | -15.330 | 14.447 | 1.00 | 17.12 | C |
| ATOM | 819 | C | LEU | A | 248 | 7.676 | -16.527 | 13.537 | 1.00 | 14.44 | C |
| ATOM | 820 | O | LEU | A | 248 | 7.656 | -17.670 | 13.977 | 1.00 | 17.33 | O |
| ATOM | 821 | CB | LEU | A | 248 | 5.951 | -15.270 | 14.852 | 1.00 | 19.74 | C |
| ATOM | 822 | CG | LEU | A | 248 | 5.635 | -14.119 | 15.804 | 1.00 | 17.23 | C |

(continued)

| | | | | | | | | | | | |
|---|---|---|---|---|---|---|---|---|---|---|---|
| ATOM | 823 | CD1 | LEU | A | 248 | 4.193 | -14.202 | 16.254 | 1.00 | 17.05 | C |
| ATOM | 824 | CD2 | LEU | A | 248 | 6.599 | -14.120 | 17.007 | 1.00 | 16.08 | C |
| ATOM | 825 | N | GLY | A | 249 | 7.920 | -16.261 | 12.263 | 1.00 | 17.53 | N |
| ATOM | 826 | CA | GLY | A | 249 | 8.159 | -17.332 | 11.315 | 1.00 | 16.74 | C |
| ATOM | 827 | C | GLY | A | 249 | 7.032 | -17.381 | 10.316 | 1.00 | 15.59 | C |
| ATOM | 828 | O | GLY | A | 249 | 5.874 | -17.344 | 10.708 | 1.00 | 17.37 | O |
| ATOM | 829 | N | GLY | A | 250 | 7.370 | -17.451 | 9.029 | 1.00 | 14.92 | N |
| ATOM | 830 | CA | GLY | A | 250 | 6.370 | -17.435 | 7.982 | 1.00 | 19.03 | C |
| ATOM | 831 | C | GLY | A | 250 | 5.672 | -16.090 | 7.891 | 1.00 | 20.35 | C |
| ATOM | 832 | O | GLY | A | 250 | 6.054 | -15.119 | 8.549 | 1.00 | 18.82 | O |
| ATOM | 833 | N | GLY | A | 251 | 4.646 | -16.022 | 7.057 | 1.00 | 21.60 | N |
| ATOM | 834 | CA | GLY | A | 251 | 3.910 | -14.787 | 6.880 | 1.00 | 17.04 | C |
| ATOM | 835 | C | GLY | A | 251 | 2.691 | -14.851 | 7.752 | 1.00 | 16.59 | C |
| ATOM | 836 | O | GLY | A | 251 | 2.778 | -15.115 | 8.952 | 1.00 | 22.55 | O |
| ATOM | 837 | N | GLU | A | 252 | 1.539 | -14.636 | 7.137 | 1.00 | 16.53 | N |
| ATOM | 838 | CA | GLU | A | 252 | 0.272 | -14.664 | 7.846 | 1.00 | 15.39 | C |
| ATOM | 839 | C | GLU | A | 252 | -0.760 | -15.400 | 7.016 | 1.00 | 17.17 | C |
| ATOM | 840 | O | GLU | A | 252 | -1.021 | -15.027 | 5.864 | 1.00 | 14.07 | O |
| ATOM | 841 | CB | GLU | A | 252 | -0.201 | -13.253 | 8.143 | 1.00 | 15.30 | C |
| ATOM | 842 | CG | GLU | A | 252 | 0.748 | -12.448 | 8.970 | 1.00 | 16.44 | C |
| ATOM | 843 | CD | GLU | A | 252 | 0.242 | -11.045 | 9.189 | 1.00 | 18.48 | C |
| ATOM | 844 | OE1 | GLU | A | 252 | 0.261 | -10.579 | 10.340 | 1.00 | 16.20 | O |
| ATOM | 845 | OE2 | GLU | A | 252 | -0.159 | -10.400 | 8.200 | 1.00 | 15.99 | O |
| ATOM | 846 | N | ASP | A | 253 | -1.355 | -16.437 | 7.590 | 1.00 | 15.10 | N |
| ATOM | 847 | CA | ASP | A | 253 | -2.163 | -17.337 | 6.787 | 1.00 | 16.04 | C |
| ATOM | 848 | C | ASP | A | 253 | -3.600 | -17.499 | 7.243 | 1.00 | 15.46 | C |
| ATOM | 849 | O | ASP | A | 253 | -4.310 | -18.320 | 6.713 | 1.00 | 17.45 | O |
| ATOM | 850 | CB | ASP | A | 253 | -1.519 | -18.719 | 6.745 | 1.00 | 15.26 | C |
| ATOM | 851 | CG | ASP | A | 253 | -0.090 | -18.683 | 6.258 | 1.00 | 18.84 | C |
| ATOM | 852 | OD1 | ASP | A | 253 | 0.337 | -17.664 | 5.670 | 1.00 | 19.76 | O |
| ATOM | 853 | OD2 | ASP | A | 253 | 0.605 | -19.703 | 6.423 | 1.00 | 24.71 | O |
| ATOM | 854 | N | CYS | A | 254 | -4.029 | -16.745 | 8.238 | 1.00 | 15.58 | N |
| ATOM | 855 | CA | CYS | A | 254 | -5.321 | -17.012 | 8.818 | 1.00 | 13.38 | C |
| ATOM | 856 | C | CYS | A | 254 | -6.083 | -15.715 | 9.012 | 1.00 | 17.24 | C |
| ATOM | 857 | O | CYS | A | 254 | -5.577 | -14.778 | 9.643 | 1.00 | 17.90 | O |
| ATOM | 858 | CB | CYS | A | 254 | -5.161 | -17.762 | 10.147 | 1.00 | 17.64 | C |
| ATOM | 859 | SG | CYS | A | 254 | -4.659 | -19.487 | 9.965 | 1.00 | 15.82 | S |
| ATOM | 860 | N | ALA | A | 255 | -7.298 | -15.672 | 8.474 | 1.00 | 10.34 | N |
| ATOM | 861 | CA | ALA | A | 255 | -8.134 | -14.474 | 8.555 | 1.00 | 15.36 | C |
| ATOM | 862 | C | ALA | A | 255 | -8.863 | -14.334 | 9.886 | 1.00 | 16.38 | C |
| ATOM | 863 | O | ALA | A | 255 | -9.420 | -15.293 | 10.424 | 1.00 | 15.65 | O |
| ATOM | 864 | CB | ALA | A | 255 | -9.153 | -14.460 | 7.430 | 1.00 | 13.09 | C |
| ATOM | 865 | N | HIS | A | 256 | -8.886 | -13.116 | 10.401 | 1.00 | 15.41 | N |
| ATOM | 866 | CA | HIS | A | 256 | -9.712 | -12.834 | 11.552 | 1.00 | 16.09 | C |
| ATOM | 867 | C | HIS | A | 256 | -10.427 | -11.512 | 11.367 | 1.00 | 14.49 | C |
| ATOM | 868 | O | HIS | A | 256 | -9.986 | -10.664 | 10.599 | 1.00 | 17.03 | O |
| ATOM | 869 | CB | HIS | A | 256 | -8.874 | -12.816 | 12.841 | 1.00 | 11.61 | C |
| ATOM | 870 | CG | HIS | A | 256 | -7.844 | -11.733 | 12.885 | 1.00 | 15.00 | C |
| ATOM | 871 | ND1 | HIS | A | 256 | -7.979 | -10.609 | 13.673 | 1.00 | 13.69 | N |
| ATOM | 872 | CD2 | HIS | A | 256 | -6.657 | -11.605 | 12.245 | 1.00 | 13.08 | C |
| ATOM | 873 | CE1 | HIS | A | 256 | -6.915 | -9.840 | 13.522 | 1.00 | 15.34 | C |

(continued)

| | | | | | | | | | | | |
|---|---|---|---|---|---|---|---|---|---|---|---|
| ATOM | 874 | NE2 | HIS | A | 256 | -6.098 | -10.423 | 12.661 | 1.00 | 15.12 | N |
| ATOM | 875 | N | PHE | A | 257 | -11.543 | -11.357 | 12.065 | 1.00 | 16.75 | N |
| ATOM | 876 | CA | PHE | A | 257 | -12.147 | -10.057 | 12.229 | 1.00 | 14.34 | C |
| ATOM | 877 | C | PHE | A | 257 | -11.246 | -9.225 | 13.119 | 1.00 | 16.09 | C |
| ATOM | 878 | O | PHE | A | 257 | -10.752 | -9.703 | 14.142 | 1.00 | 16.99 | O |
| ATOM | 879 | CB | PHE | A | 257 | -13.541 | -10.159 | 12.844 | 1.00 | 13.50 | C |
| ATOM | 880 | CG | PHE | A | 257 | -14.430 | -11.166 | 12.176 | 1.00 | 21.06 | C |
| ATOM | 881 | CD2 | PHE | A | 257 | -14.462 | -12.477 | 12.618 | 1.00 | 16.97 | C |
| ATOM | 882 | CD1 | PHE | A | 257 | -15.233 | -10.803 | 11.098 | 1.00 | 18.50 | C |
| ATOM | 883 | CE2 | PHE | A | 257 | -15.277 | -13.410 | 12.003 | 1.00 | 17.90 | C |
| ATOM | 884 | CE1 | PHE | A | 257 | -16.049 | -11.729 | 10.487 | 1.00 | 16.37 | C |
| ATOM | 885 | CZ | PHE | A | 257 | -16.066 | -13.029 | 10.928 | 1.00 | 14.28 | C |
| ATOM | 886 | N | THR | A | 258 | -11.039 | -7.979 | 12.734 | 1.00 | 15.18 | N |
| ATOM | 887 | CA | THR | A | 258 | -10.337 | -7.053 | 13.597 | 1.00 | 19.93 | C |
| ATOM | 888 | C | THR | A | 258 | -11.351 | -6.356 | 14.465 | 1.00 | 22.34 | C |
| ATOM | 889 | O | THR | A | 258 | -12.557 | -6.616 | 14.356 | 1.00 | 28.29 | O |
| ATOM | 890 | CB | THR | A | 258 | -9.553 | -6.014 | 12.814 | 1.00 | 18.33 | C |
| ATOM | 891 | OG1 | THR | A | 258 | -10.468 | -5.213 | 12.058 | 1.00 | 17.14 | O |
| ATOM | 892 | CG2 | THR | A | 258 | -8.569 | -6.692 | 11.876 | 1.00 | 19.40 | C |
| ATOM | 893 | N | ASP | A | 259 | -10.869 | -5.459 | 15.316 | 1.00 | 20.10 | N |
| ATOM | 894 | CA | ASP | A | 259 | -11.735 | -4.749 | 16.250 | 1.00 | 23.59 | C |
| ATOM | 895 | C | ASP | A | 259 | -12.909 | -4.037 | 15.572 | 1.00 | 23.64 | C |
| ATOM | 896 | O | ASP | A | 259 | -13.984 | -3.947 | 16.148 | 1.00 | 24.14 | O |
| ATOM | 897 | CB | ASP | A | 259 | -10.911 | -3.747 | 17.079 | 1.00 | 22.86 | C |
| ATOM | 898 | CG | ASP | A | 259 | -10.205 | -2.685 | 16.223 | 1.00 | 30.45 | C |
| ATOM | 899 | OD1 | ASP | A | 259 | -10.086 | -2.851 | 14.987 | 1.00 | 32.11 | O |
| ATOM | 900 | OD2 | ASP | A | 259 | -9.736 | -1.679 | 16.808 | 1.00 | 36.17 | O |
| ATOM | 901 | N | ASP | A | 260 | -12.710 | -3.542 | 14.351 | 1.00 | 27.68 | N |
| ATOM | 902 | CA | ASP | A | 260 | -13.762 | -2.778 | 13.673 | 1.00 | 24.53 | C |
| ATOM | 903 | C | ASP | A | 260 | -14.544 | -3.664 | 12.716 | 1.00 | 23.69 | C |
| ATOM | 904 | O | ASP | A | 260 | -15.474 | -3.208 | 12.055 | 1.00 | 27.59 | O |
| ATOM | 905 | CB | ASP | A | 260 | -13.177 | -1.551 | 12.934 | 1.00 | 23.65 | C |
| ATOM | 906 | CG | ASP | A | 260 | -12.279 | -1.920 | 11.738 | 1.00 | 31.18 | C |
| ATOM | 907 | OD1 | ASP | A | 260 | -12.578 | -2.896 | 11.008 | 1.00 | 25.05 | O |
| ATOM | 908 | OD2 | ASP | A | 260 | -11.267 | -1.206 | 11.514 | 1.00 | 30.59 | O |
| ATOM | 909 | N | GLY | A | 261 | -14.138 | -4.927 | 12.617 | 1.00 | 24.03 | N |
| ATOM | 910 | CA | GLY | A | 261 | -14.898 | -5.908 | 11 . 863 | 1.00 | 18.76 | C |
| ATOM | 911 | C | GLY | A | 261 | -14.236 | -6.262 | 10.550 | 1.00 | 17.99 | C |
| ATOM | 912 | O | GLY | A | 261 | -14.459 | -7.331 | 10.004 | 1.00 | 13.89 | O |
| ATOM | 913 | N | ARG | A | 262 | -13.392 | -5.373 | 10.048 | 1.00 | 19.30 | N |
| ATOM | 914 | CA | ARG | A | 262 | -12.709 | -5.649 | 8.791 | 1.00 | 19.10 | C |
| ATOM | 915 | C | ARG | A | 262 | -11.659 | -6.749 | 8.989 | 1.00 | 19.94 | C |
| ATOM | 916 | O | ARG | A | 262 | -11.216 | -7.016 | 10.120 | 1.00 | 12.26 | O |
| ATOM | 917 | CB | ARG | A | 262 | -12.115 | -4.356 | 8.232 | 1.00 | 20.66 | C |
| ATOM | 918 | CG | ARG | A | 262 | -13.219 | -3.351 | 7 .897 | 1.00 | 25.30 | C |
| ATOM | 919 | CD | ARG | A | 262 | -12.667 | -2.017 | 7.453 | 1.00 | 28.34 | C |
| ATOM | 920 | NE | ARG | A | 262 | -11.765 | -1.459 | 8.449 | 1.00 | 25.73 | N |
| ATOM | 921 | CZ | ARG | A | 262 | -11.035 | -0.367 | 8.254 | 1.00 | 29.19 | C |
| ATOM | 922 | NH1 | ARG | A | 262 | -11.114 | 0.278 | 7.100 | 1.00 | 24.96 | N |
| ATOM | 923 | NH2 | ARG | A | 262 | -10.229 | 0.079 | 9.208 | 1.00 | 29.61 | N |
| ATOM | 924 | N | TRP | A | 263 | -11.307 | -7.432 | 7.903 | 1.00 | 14.26 | N |

(continued)

| | | | | | | | | | | | |
|---|---|---|---|---|---|---|---|---|---|---|---|
| ATOM | 925 | CA | TRP | A | 263 | -10.517 | -8.648 | 8.041 | 1.00 | 16.50 | C |
| ATOM | 926 | C | TRP | A | 263 | -9.043 | -8.337 | 8.000 | 1.00 | 15.53 | C |
| ATOM | 927 | O | TRP | A | 263 | -8.627 | -7.337 | 7.443 | 1.00 | 16.65 | O |
| ATOM | 928 | CB | TRP | A | 263 | -10.851 | -9.661 | 6.946 | 1.00 | 13.91 | C |
| ATOM | 929 | CG | TRP | A | 263 | -12.290 | -10.013 | 6.862 | 1.00 | 16.25 | C |
| ATOM | 930 | CD1 | TRP | A | 263 | -13.273 | -9.643 | 7.721 | 1.00 | 13.40 | C |
| ATOM | 931 | CD2 | TRP | A | 263 | -12.918 | -10.804 | 5.846 | 1.00 | 17.03 | C |
| ATOM | 932 | NE1 | TRP | A | 263 | -14.473 | -10.156 | 7.314 | 1.00 | 15.19 | N |
| ATOM | 933 | CE2 | TRP | A | 263 | -14.284 | -10.870 | 6.160 | 1.00 | 14.98 | C |
| ATOM | 934 | CE3 | TRP | A | 263 | -12.456 | -11.455 | 4.697 | 1.00 | 13.09 | C |
| ATOM | 935 | CZ2 | TRP | A | 263 | -15.196 | -11.577 | 5.378 | 1.00 | 19.78 | C |
| ATOM | 936 | CZ3 | TRP | A | 263 | -13.360 | -12.154 | 3.922 | 1.00 | 16.68 | C |
| ATOM | 937 | CH2 | TRP | A | 263 | -14.716 | -12.206 | 4.261 | 1.00 | 16.08 | C |
| ATOM | 938 | N | ASN | A | 264 | -8.262 | -9.226 | 8.588 | 1.00 | 15.51 | N |
| ATOM | 939 | CA | ASN | A | 264 | -6.816 | -9.162 | 8.521 | 1.00 | 14.14 | C |
| ATOM | 940 | C | ASN | A | 264 | -6.313 | -10.573 | 8.398 | 1.00 | 14.75 | C |
| ATOM | 941 | O | ASN | A | 264 | -6.994 | -11.503 | 8.817 | 1.00 | 18.31 | O |
| ATOM | 942 | CB | ASN | A | 264 | -6.239 | -8.490 | 9.768 | 1.00 | 17.34 | C |
| ATOM | 943 | CG | ASN | A | 264 | -4.721 | -8.499 | 9.794 | 1.00 | 18.04 | C |
| ATOM | 944 | OD1 | ASN | A | 264 | -4.070 | -8.128 | 8.820 | 1.00 | 13.84 | O |
| ATOM | 945 | ND2 | ASN | A | 264 | -4.151 | -8.917 | 10.923 | 1.00 | 16.66 | N |
| ATOM | 946 | N | ASP | A | 265 | -5.146 | -10.757 | 7.795 | 1.00 | 18.48 | N |
| ATOM | 947 | CA | ASP | A | 265 | -4.527 | -12.072 | 7.800 | 1.00 | 18.19 | C |
| ATOM | 948 | C | ASP | A | 265 | -3.499 | -11.991 | 8.902 | 1.00 | 17.54 | C |
| ATOM | 949 | O | ASP | A | 265 | -2.811 | -10.980 | 9.030 | 1.00 | 18.38 | O |
| ATOM | 950 | CB | ASP | A | 265 | -3.912 | -12.455 | 6.438 | 1.00 | 15.50 | C |
| ATOM | 951 | CG | ASP | A | 265 | -2.875 | -11.458 | 5.943 | 1.00 | 16.55 | C |
| ATOM | 952 | OD1 | ASP | A | 265 | -2.920 | -10.277 | 6.358 | 1.00 | 15.74 | O |
| ATOM | 953 | OD2 | ASP | A | 265 | -2.022 | -11.858 | 5.106 | 1.00 | 16.21 | O |
| ATOM | 954 | N | ASP | A | 266 | -3.449 | -13.013 | 9.745 | 1.00 | 15.99 | N |
| ATOM | 955 | CA | ASP | A | 266 | -2.527 | -12.977 | 10.867 | 1.00 | 16.72 | C |
| ATOM | 956 | C | ASP | A | 266 | -1.843 | -14.321 | 11.085 | 1.00 | 19.73 | C |
| ATOM | 957 | O | ASP | A | 266 | -2.100 | -15.292 | 10.371 | 1.00 | 18.63 | O |
| ATOM | 958 | CB | ASP | A | 266 | -3.244 | -12.542 | 12.139 | 1.00 | 15.67 | C |
| ATOM | 959 | CG | ASP | A | 266 | -2.356 | -11.743 | 13.041 | 1.00 | 20.35 | C |
| ATOM | 960 | OD2 | ASP | A | 266 | -2.877 | -10.827 | 13.717 | 1.00 | 20.40 | O |
| ATOM | 961 | OD1 | ASP | A | 266 | -1.130 | -12.024 | 13.055 | 1.00 | 18.05 | O |
| ATOM | 962 | N | VAL | A | 267 | -0.951 | -14.365 | 12.069 | 1.00 | 21.31 | N |
| ATOM | 963 | CA | VAL | A | 267 | -0.236 | -15.588 | 12.357 | 1.00 | 20.03 | C |
| ATOM | 964 | C | VAL | A | 267 | -1.206 | -16.604 | 12.957 | 1.00 | 17.18 | C |
| ATOM | 965 | O | VAL | A | 267 | -1.904 | -16.301 | 13.923 | 1.00 | 19.78 | O |
| ATOM | 966 | CB | VAL | A | 267 | 0.935 | -15.327 | 13.302 | 1.00 | 18.16 | C |
| ATOM | 967 | CG1 | VAL | A | 267 | 1.670 | -16.609 | 13.576 | 1.00 | 19.68 | C |
| ATOM | 968 | CG2 | VAL | A | 267 | 1.862 | -14.301 | 12.685 | 1.00 | 15.95 | C |
| ATOM | 969 | N | CYS | A | 268 | -1.246 | -17.803 | 12.383 | 1.00 | 12.37 | N |
| ATOM | 970 | CA | CYS | A | 268 | -2.213 | -18.818 | 12.785 | 1.00 | 14.68 | C |
| ATOM | 971 | C | CYS | A | 268 | -2.031 | -19.284 | 14.238 | 1.00 | 14.79 | C |
| ATOM | 972 | O | CYS | A | 268 | -2.960 | -19.800 | 14.855 | 1.00 | 17.64 | O |
| ATOM | 973 | CB | CYS | A | 268 | -2.140 | -20.010 | 11 . 832 | 1.00 | 15.66 | C |
| ATOM | 974 | SG | CYS | A | 268 | -2.628 | -19.567 | 10.101 | 1.00 | 27.47 | S |
| ATOM | 975 | N | GLN | A | 269 | -0.851 | -19.069 | 14.786 | 1.00 | 13.72 | N |

(continued)

| | | | | | | | | | | | |
|---|---|---|---|---|---|---|---|---|---|---|---|
| ATOM | 976 | CA | GLN | A | 269 | -0.559 | -19.493 | 16.149 | 1.00 | 16.48 | C |
| ATOM | 977 | C | GLN | A | 269 | -1.136 | -18.565 | 17.190 | 1.00 | 15.79 | C |
| ATOM | 978 | O | GLN | A | 269 | -1.204 | -18.920 | 18.376 | 1.00 | 17.70 | O |
| ATOM | 979 | CB | GLN | A | 269 | 0.934 | -19.562 | 16.397 | 1.00 | 17.79 | C |
| ATOM | 980 | CG | GLN | A | 269 | 1.752 | -20.417 | 15.507 | 1.00 | 20.66 | C |
| ATOM | 981 | CD | GLN | A | 269 | 3.240 | -20.205 | 15.825 | 1.00 | 31.48 | C |
| ATOM | 982 | OE1 | GLN | A | 269 | 3.870 | -19.269 | 15.315 | 1.00 | 28.23 | O |
| ATOM | 983 | NE2 | GLN | A | 269 | 3.786 | -21.039 | 16.708 | 1.00 | 24.46 | N |
| ATOM | 9B4 | N | ARG | A | 270 | -1.501 | -17.358 | 16.781 | 1.00 | 13.72 | N |
| ATOM | 985 | CA | ARG | A | 270 | -2.043 | -16.420 | 17.759 | 1.00 | 14.78 | C |
| ATOM | 986 | C | ARG | A | 270 | -3.318 | -16.974 | 18.307 | 1.00 | 13.79 | C |
| ATOM | 987 | O | ARG | A | 270 | -4.116 | -17.542 | 17.558 | 1.00 | 16.01 | O |
| ATOM | 988 | CB | ARG | A | 270 | -2.301 | -15.045 | 17.162 | 1.00 | 13.63 | C |
| ATOM | 989 | CG | ARG | A | 270 | -1.059 | -14.369 | 16.672 | 1.00 | 17.92 | C |
| ATOM | 990 | CD | ARG | A | 270 | -1.316 | -12.904 | 16.442 | 1.00 | 15.26 | C |
| ATOM | 991 | NE | ARG | A | 270 | -0.240 | -12.295 | 15.678 | 1.00 | 18.21 | N |
| ATOM | 992 | CZ | ARG | A | 270 | 0.907 | -11.880 | 16.206 | 1.00 | 17.13 | C |
| ATOM | 993 | NH1 | ARG | A | 270 | 1.137 | -12.008 | 17.513 | 1.00 | 15.71 | N |
| ATOM | 994 | NH2 | ARG | A | 270 | 1.814 | -11.320 | 15.426 | 1.00 | 12.96 | N |
| ATOM | 995 | N | PRO | A | 271 | -3.503 | -16.833 | 19.623 | 1.00 | 14.36 | N |
| ATOM | 996 | CA | PRO | A | 271 | -4.769 | -17.166 | 20.253 | 1.00 | 12.34 | C |
| ATOM | 997 | C | PRO | A | 271 | -5.760 | -15.999 | 20.077 | 1.00 | 13.26 | C |
| ATOM | 998 | O | PRO | A | 271 | -5.535 | -14.874 | 20.507 | 1.00 | 14.69 | O |
| ATOM | 999 | CB | PRO | A | 271 | -4.381 | -17.391 | 21.727 | 1.00 | 15.38 | C |
| ATOM | 1000 | CG | PRO | A | 271 | -3.151 | -16.556 | 21.928 | 1.00 | 15.88 | C |
| ATOM | 1001 | CD | PRO | A | 271 | -2.502 | -16.336 | 20.593 | 1.00 | 15.31 | C |
| ATOM | 1002 | N | TYR | A | 272 | -6.855 | -16.285 | 19.400 | 1.00 | 13.29 | N |
| ATOM | 1003 | CA | TYR | A | 272 | -7.918 | -15.315 | 19.193 | 1.00 | 16.14 | C |
| ATOM | 1004 | C | TYR | A | 272 | -9.201 | -16.027 | 19.549 | 1.00 | 13.34 | C |
| ATOM | 1005 | O | TYR | A | 272 | -9.224 | -17.253 | 19.601 | 1.00 | 12.02 | O |
| ATOM | 1006 | CB | TYR | A | 272 | -7.982 | -14.830 | 17.736 | 1.00 | 11.47 | C |
| ATOM | 1007 | CG | TYR | A | 272 | -6.967 | -13.776 | 17.310 | 1.00 | 13.69 | C |
| ATOM | 1008 | CD1 | TYR | A | 272 | -6.439 | -12.872 | 18.213 | 1.00 | 9.93 | C |
| ATOM | 1009 | CD2 | TYR | A | 272 | -6.574 | -13.673 | 15.969 | 1.00 | 12.95 | C |
| ATOM | 1010 | CE1 | TYR | A | 272 | -5.538 | -11.914 | 17.807 | 1.00 | 11.19 | C |
| ATOM | 1011 | CE2 | TYR | A | 272 | -5.684 | -12.723 | 15.561 | 1.00 | 9.94 | C |
| ATOM | 1012 | CZ | TYR | A | 272 | -5.164 | -11.845 | 16.484 | 1.00 | 14.00 | C |
| ATOM | 1013 | OH | TYR | A | 272 | -4.257 | -10.893 | 16.080 | 1.00 | 17.41 | O |
| ATOM | 1014 | N | ARG | A | 273 | -10.271 | -15.272 | 19.765 | 1.00 | 12.95 | N |
| ATOM | 1015 | CA | ARG | A | 273 | -11.572 | -15.885 | 19.870 | 1.00 | 12.35 | C |
| ATOM | 1016 | C | ARG | A | 273 | -11.927 | -16.389 | 18.493 | 1.00 | 14.06 | C |
| ATOM | 1017 | O | ARG | A | 273 | -11.229 | -16.088 | 17.517 | 1.00 | 14.38 | O |
| ATOM | 1018 | CB | ARG | A | 273 | -12.611 | -14.905 | 20.397 | 1.00 | 14.33 | C |
| ATOM | 1019 | CG | ARG | A | 273 | -12.364 | -14.520 | 21.841 | 1.00 | 12.78 | C |
| ATOM | 1020 | CD | ARG | A | 273 | -13.449 | -13.645 | 22.401 | 1.00 | 17.64 | C |
| ATOM | 1021 | NE | ARG | A | 273 | -13.276 | -13.515 | 23.840 | 1.00 | 21.61 | N |
| ATOM | 1022 | CZ | ARG | A | 273 | -14.015 | -12.742 | 24.625 | 1.00 | 24.75 | C |
| ATOM | 1023 | NH1 | ARG | A | 273 | -14.997 | -12.010 | 24.109 | 1.00 | 30.00 | N |
| ATOM | 1024 | NH2 | ARG | A | 273 | -13.763 | -12.702 | 25.930 | 1.00 | 20.72 | N |
| ATOM | 1025 | N | TRP | A | 274 | -12.986 | -17.183 | 18.411 | 1.00 | 13.15 | N |
| ATOM | 1026 | CA | TRP | A | 274 | -13.420 | -17.706 | 17.130 | 1.00 | 13.83 | C |

(continued)

| | | | | | | | | | | | |
|---|---|---|---|---|---|---|---|---|---|---|---|
| ATOM | 1027 | C | TRP | A | 274 | -14.926 | -17.845 | 17.166 | 1.00 | 15.15 | C |
| ATOM | 1028 | O | TRP | A | 274 | -15.525 | -17.760 | 18.227 | 1.00 | 14.10 | O |
| ATOM | 1029 | CB | TRP | A | 274 | -12.758 | -19.043 | 16.833 | 1.00 | 12.63 | C |
| ATOM | 1030 | CG | TRP | A | 274 | -13.207 | -20.135 | 17.726 | 1.00 | 16.63 | C |
| ATOM | 1031 | CD1 | TRP | A | 274 | -14.106 | -21.107 | 17.428 | 1.00 | 18.08 | C |
| ATOM | 1032 | CD2 | TRP | A | 274 | -12.780 | -20.377 | 19.080 | 1.00 | 14.16 | C |
| ATOM | 1033 | NE1 | TRP | A | 274 | -14.265 | -21.951 | 18.507 | 1.00 | 15.30 | N |
| ATOM | 1034 | CE2 | TRP | A | 274 | -13.463 | -21.521 | 19.533 | 1.00 | 15.80 | C |
| ATOM | 1035 | CE3 | TRP | A | 274 | -11.874 | -19.744 | 19.944 | 1.00 | 16.39 | C |
| ATOM | 1036 | CZ2 | TRP | A | 274 | -13.282 | -22.046 | 20.822 | 1.00 | 16.41 | C |
| ATOM | 1037 | CZ3 | TRP | A | 274 | -11.700 | -20.261 | 21.226 | 1.00 | 17.93 | C |
| ATOM | 1038 | CH2 | TRP | A | 274 | -12.407 | -21.401 | 21.651 | 1.00 | 14 . 81 | C |
| ATOM | 1039 | N | VAL | A | 275 | -15.532 | -18.025 | 16.003 | 1.00 | 13.91 | N |
| ATOM | 1040 | CA | VAL | A | 275 | -16.954 | -18.282 | 15.911 | 1.00 | 14.90 | C |
| ATOM | 1041 | C | VAL | A | 275 | -17.157 | -19.604 | 15.214 | 1.00 | 17.68 | C |
| ATOM | 1042 | O | VAL | A | 275 | -16.570 | -19.843 | 14.160 | 1.00 | 16.82 | O |
| ATOM | 1043 | CB | VAL | A | 275 | -17.690 | -17.199 | 15.126 | 1.00 | 15.04 | C |
| ATOM | 1044 | CG1 | VAL | A | 275 | -19.194 | -17.473 | 15.122 | 1.00 | 14.67 | C |
| ATOM | 1045 | CG2 | VAL | A | 275 | -17.359 | -15.825 | 15.676 | 1.00 | 12.68 | C |
| ATOM | 1046 | N | CYS | A | 276 | -17.993 | -20.463 | 15.791 | 1.00 | 18.92 | N |
| ATOM | 1047 | CA | CYS | A | 276 | -18.396 | -21.676 | 15.095 | 1.00 | 20.03 | C |
| ATOM | 1048 | C | CYS | A | 276 | -19.725 | -21.444 | 14.397 | 1.00 | 19.45 | C |
| ATOM | 1049 | O | CYS | A | 276 | -20.581 | -20.714 | 14.896 | 1.00 | 17.29 | O |
| ATOM | 1050 | CB | CYS | A | 276 | -18.493 | -22.859 | 16.055 | 1.00 | 22.59 | C |
| ATOM | 1051 | SG | CYS | A | 276 | -16.895 | -23.523 | 16.446 | 1.00 | 31.78 | S |
| ATOM | 1052 | N | GLU | A | 277 | -19.865 | -22.050 | 13.225 | 1.00 | 18.94 | N |
| ATOM | 1053 | CA | GLU | A | 277 | -21.077 | -21.941 | 12.437 | 1.00 | 21.40 | C |
| ATOM | 1054 | C | GLU | A | 277 | -21.492 | -23.299 | 11.919 | 1.00 | 19.98 | C |
| ATOM | 1055 | O | GLU | A | 277 | -20.668 | -24.086 | 11.467 | 1.00 | 20.59 | O |
| ATOM | 1056 | CB | GLU | A | 277 | -20.887 | -20.988 | 11.263 | 1.00 | 17.46 | C |
| ATOM | 1057 | CG | GLU | A | 277 | -22.187 | -20.673 | 10.536 | 1.00 | 21.39 | C |
| ATOM | 1058 | CD | GLU | A | 277 | -21.972 | -20.063 | 9.154 | 1.00 | 22.43 | C |
| ATOM | 1059 | OE1 | GLU | A | 277 | -20.817 | -20.032 | 8.682 | 1.00 | 21.58 | O |
| ATOM | 1060 | OE2 | GLU | A | 277 | -22.962 | -19.607 | 8.540 | 1.00 | 22.39 | O |
| ATOM | 1061 | N | THR | A | 278 | -22.778 | -23.572 | 11.998 | 1.00 | 19.21 | N |
| ATOM | 1062 | CA | THR | A | 278 | -23.351 | -24.710 | 11.309 | 1.00 | 24.97 | C |
| ATOM | 1063 | C | THR | A | 278 | -24.612 | -24.250 | 10.581 | 1.00 | 25.29 | C |
| ATOM | 1064 | O | THR | A | 278 | -25.166 | -23.196 | 10.887 | 1.00 | 24.46 | O |
| ATOM | 1065 | CB | THR | A | 278 | -23.688 | -25.846 | 12.267 | 1.00 | 25.96 | C |
| ATOM | 1066 | OG1 | THR | A | 278 | -23.799 | -27.063 | 11.527 | 1.00 | 36.51 | O |
| ATOM | 1067 | CG2 | THR | A | 278 | -24.996 | -25.566 | 12.973 | 1.00 | 23.69 | C |
| ATOM | 1068 | N | GLU | A | 279 | -25.065 | -25.033 | 9.613 | 1.00 | 29.02 | N |
| ATOM | 1069 | CA | GLU | A | 279 | -26.228 | -24.630 | 8.841 | 1.00 | 31.70 | C |
| ATOM | 1070 | C | GLU | A | 279 | -27.438 | -25.498 | 9.167 | 1.00 | 29.56 | C |
| ATOM | 1071 | O | GLU | A | 279 | -27.299 | -26.687 | 9.406 | 1.00 | 29.11 | O |
| ATOM | 1072 | CB | GLU | A | 279 | -25.911 | -24.678 | 7.347 | 1.00 | 28.64 | C |
| ATOM | 1073 | CG | GLU | A | 279 | -24.969 | -23.568 | 6.885 | 1.00 | 27.57 | C |
| ATOM | 1074 | CD | GLU | A | 279 | -23.497 | -23.939 | 7.011 | 1.00 | 31.65 | C |
| ATOM | 1075 | OE1 | GLU | A | 279 | -22.662 | -23.026 | 7.182 | 1.00 | 30.86 | O |
| ATOM | 1076 | OE2 | GLU | A | 279 | -23.166 | -25.139 | 6.923 | 1.00 | 32.59 | O |
| ATOM | 1077 | N | LEU | A | 280 | -28.617 | -24.885 | 9.219 | 1.00 | 34.32 | N |

(continued)

| | | | | | | | | | | | |
|---|---|---|---|---|---|---|---|---|---|---|---|
| ATOM | 1078 | CA | LEU | A | 280 | -29.855 | -25.649 | 9.304 | 1.00 | 35.91 | C |
| ATOM | 1079 | C | LEU | A | 280 | -30.265 | -26.069 | 7.893 | 1.00 | 40.23 | C |
| ATOM | 1080 | O | LEU | A | 280 | -30.735 | -27.187 | 7.666 | 1.00 | 43.65 | O |
| ATOM | 1081 | CB | LEU | A | 280 | -30.974 | -24.837 | 9.959 | 1.00 | 37.40 | C |
| ATOM | 1082 | CG | LEU | A | 280 | -30.811 | -24.359 | 11.398 | 1.00 | 33.17 | C |
| ATOM | 1083 | CD1 | LEU | A | 280 | -32.064 | -23.616 | 11.839 | 1.00 | 39.13 | C |
| ATOM | 1084 | CD2 | LEU | A | 280 | -30.516 | -25.518 | 12.322 | 1.00 | 36.44 | C |
| ATOM | 1085 | OXT | LEU | A | 280 | -30.126 | -25.285 | 6.942 | 1.00 | 35.92 | O |
| HETATM | 1086 | CA | CA | | A1001 | -0.390 | -15.928 | 3.667 | 1.00 | 18.60 | Ca |
| HETATM | 1087 | CA | CA | | A1002 | -1.936 | -8.931 | 8.005 | 1.00 | 15.61 | Ca |
| HETATM | 1088 | CA | CA | | A1003 | -22.072 | -18.548 | 6.675 | 1.00 | 20.87 | Ca |
| HETATM | 1089 | CL | CL | | A1004 | -0.586 | -12.976 | 20.217 | 1.00 | 15.82 | Cl |
| HETATM | 1090 | CL | CL | | A1005 | -16.511 | -11.093 | 27.288 | 1.00 | 51.09 | C1 |
| TER | | | | | | | | | | | |
| HETATM | 1091 | C1 | NGA | D | 1 | -0.855 | -5.202 | 11.893 | 1.00 | 29.65 | C |
| HETATM | 1092 | C2 | NGA | D | 1 | -1.637 | -6.424 | 11.455 | 1.00 | 25.74 | C |
| HETATM | 1093 | C3 | NGA | D | 1 | -0.719 | -7.433 | 10.877 | 1.00 | 19.12 | C |
| HETATM | 1094 | C4 | NGA | D | 1 | 0.140 | -6.858 | 9.782 | 1.00 | 26.89 | C |
| HETATM | 1095 | C5 | NGA | D | 1 | 0.800 | -5.564 | 10.227 | 1.00 | 27.00 | C |
| HETATM | 1096 | C6 | NGA | D | 1 | 1.564 | -4.950 | 9.107 | 1.00 | 23.07 | C |
| HETATM | 1097 | C7 | NGA | D | 1 | -3.674 | -6.716 | 12.908 | 1.00 | 23.79 | C |
| HETATM | 1098 | CB | NGA | D | 1 | -4.345 | -7.353 | 14.124 | 1.00 | 17.91 | C |
| HETATM | 1099 | N2 | NGA | D | 1 | -2.306 | -7.017 | 12.612 | 1.00 | 24.92 | N |
| HETATM | 1100 | 01 | NGA | D | 1 | -1.657 | -4.257 | 12.478 | 1.00 | 40.15 | O |
| HETATM | 1101 | 03 | NGA | D | 1 | -1.485 | -8.575 | 10.399 | 1.00 | 17.51 | O |
| HETATM | 1102 | 04 | NGA | D | 1 | -0.627 | -6.578 | 8.599 | 1.00 | 18.77 | O |
| HETATM | 1103 | 05 | NGA | D | 1 | -0.199 | -4.606 | 10.704 | 1.00 | 21.93 | O |
| HETATM | 1104 | 06 | NGA | D | 1 | 2.201 | -3.801 | 9.619 | 1.00 | 23.93 | O |
| HETATM | 1105 | 07 | NGA | D | 1 | -4.290 | -5.954 | 12.181 | 1.00 | 26.58 | O |
| TER | | | | | | | | | | | |
| END | | | | | | | | | | | |

**Table 10.2**

| | | | | | | | | | | | |
|---|---|---|---|---|---|---|---|---|---|---|---|
| ATOM | 1 | N | GLN | A | 1 | 4.287 | -11.302 | 31.298 | 1.00 | 52.13 | N |
| ATOM | 2 | CA | GLN | A | 1 | 2.840 | -11.140 | 31.183 | 1.00 | 68.91 | C |
| ATOM | 3 | C | GLN | A | 1 | 2.320 | -11.699 | 29.861 | 1.00 | 58.31 | C |
| ATOM | 4 | O | GLN | A | 1 | 3.071 | -11.816 | 28.894 | 1.00 | 49.26 | O |
| ATOM | 5 | CB | GLN | A | 1 | 2.457 | -9.664 | 31.320 | 1.00 | 75.74 | C |
| ATOM | 6 | CG | GLN | A | 1 | 2.710 | -9.088 | 32.707 | 1.00 | 88.73 | C |
| ATOM | 7 | CD | GLN | A | 1 | 1.879 | -9.771 | 33.783 | 1.00 | 100.20 | C |
| ATOM | 8 | NE2 | GLN | A | 1 | 2.448 | -9.903 | 34.978 | 1.00 | 94.67 | N |
| ATOM | 9 | OE1 | GLN | A | 1 | 0.742 | -10.179 | 33.539 | 1.00 | 98.51 | O |
| ATOM | 10 | N | VAL | A | 2 | 1.035 | -12.043 | 29.824 | 1.00 | 56.43 | N |
| ATOM | 11 | CA | VAL | A | 2 | 0.425 | -12.599 | 28.617 | 1.00 | 47.45 | C |
| ATOM | 12 | C | VAL | A | 2 | 0.210 | -11.533 | 27.541 | 1.00 | 49.76 | C |
| ATOM | 13 | O | VAL | A | 2 | -0.422 | -10.506 | 27.790 | 1.00 | 52.22 | O |
| ATOM | 14 | CB | VAL | A | 2 | -0.928 | -13.275 | 28.926 | 1.00 | 51.59 | C |
| ATOM | 15 | CG1 | VAL | A | 2 | -1.627 | -13.682 | 27.636 | 1.00 | 51.80 | C |
| ATOM | 16 | CG2 | VAL | A | 2 | -0.727 | -14.481 | 29.825 | 1.00 | 44.15 | C |
| ATOM | 17 | N | GLN | A | 3 | 0.740 | -11.785 | 26.348 | 1.00 | 39.57 | N |
| ATOM | 18 | CA | GLN | A | 3 | 0.582 | -10.876 | 25.219 | 1.00 | 41.94 | C |
| ATOM | 19 | C | GLN | A | 3 | 0.085 | -11.615 | 23.981 | 1.00 | 38.69 | C |
| ATOM | 20 | O | GLN | A | 3 | 0.586 | -12.691 | 23.649 | 1.00 | 39.97 | O |
| ATOM | 21 | CB | GLN | A | 3 | 1.903 | -10.176 | 24.891 | 1.00 | 40.78 | C |
| ATOM | 22 | CG | GLN | A | 3 | 2.450 | -9.275 | 25.980 | 1.00 | 61.11 | C |
| ATOM | 23 | CD | GLN | A | 3 | 3.756 | -8.612 | 25.570 | 1.00 | 71.25 | C |
| ATOM | 24 | NE2 | GLN | A | 3 | 4.265 | -7.724 | 26.417 | 1.00 | 63.18 | N |
| ATOM | 25 | OE1 | GLN | A | 3 | 4.299 | -8.899 | 24.502 | 1.00 | 74.87 | O |
| ATOM | 26 | N | LEU | A | 4 | -0.896 | -11.035 | 23.298 | 1.00 | 33.31 | N |
| ATOM | 27 | CA | LEU | A | 4 | -1.361 | -11.577 | 22.025 | 1.00 | 32.23 | C |
| ATOM | 28 | C | LEU | A | 4 | -1.077 | -10.581 | 20.903 | 1.00 | 34.08 | C |
| ATOM | 29 | O | LEU | A | 4 | -1.523 | -9.436 | 20.954 | 1.00 | 35.79 | O |
| ATOM | 30 | CB | LEU | A | 4 | -2.854 | -11.904 | 22.076 | 1.00 | 24.96 | C |
| ATOM | 31 | CG | LEU | A | 4 | -3.339 | -12.906 | 23.123 | 1.00 | 35.14 | C |
| ATOM | 32 | CD1 | LEU | A | 4 | -4 . 849 | -13.045 | 23.036 | 1.00 | 33.63 | C |
| ATOM | 33 | CD2 | LEU | A | 4 | -2.665 | -14.259 | 22.950 | 1.00 | 36.57 | C |
| ATOM | 34 | N | VAL | A | 5 | -0.333 | -11.023 | 19.894 | 1.00 | 34.68 | N |

(continued)

| | | | | | | | | | | | |
|---|---|---|---|---|---|---|---|---|---|---|---|
| ATOM | 35 | CA | VAL | A | 5 | 0.072 | -10.150 | 18.794 | 1.00 | 31.10 | C |
| ATOM | 36 | C | VAL | A | 5 | -0.418 | -10.708 | 17.466 | 1.00 | 27.37 | C |
| ATOM | 37 | O | VAL | A | 5 | -0.029 | -11.804 | 17.061 | 1.00 | 33.78 | O |
| ATOM | 38 | CB | VAL | A | 5 | 1.603 | -9.971 | 18.747 | 1.00 | 33.23 | C |
| ATOM | 39 | CG1 | VAL | A | 5 | 2.000 | -9.098 | 17.564 | 1.00 | 39.33 | C |
| ATOM | 40 | CG2 | VAL | A | 5 | 2.107 | -9.365 | 20.052 | 1.00 | 30.20 | C |
| ATOM | 41 | N | GLN | A | 6 | -1.280 | -9.951 | 16.792 | 1.00 | 27.57 | N |
| ATOM | 42 | CA | GLN | A | 6 | -1.882 | -10.411 | 15.545 | 1.00 | 31.03 | C |
| ATOM | 43 | C | GLN | A | 6 | -1.155 | -9.849 | 14.333 | 1.00 | 32.02 | C |
| ATOM | 44 | O | GLN | A | 6 | -0.447 | -8.847 | 14.431 | 1.00 | 31.18 | O |
| ATOM | 45 | CB | GLN | A | 6 | -3.362 | -10.026 | 15.492 | 1.00 | 23.97 | C |
| ATOM | 46 | CG | GLN | A | 6 | -4.154 | -10.551 | 16.674 | 1.00 | 31.59 | C |
| ATOM | 47 | CD | GLN | A | 6 | -5.613 | -10.158 | 16.634 | 1.00 | 31.89 | C |
| ATOM | 48 | NE2 | GLN | A | 6 | -6.203 | -10.163 | 15.438 | 1.00 | 30.10 | N |
| ATOM | 49 | OE1 | GLN | A | 6 | -6.210 | -9.863 | 17.669 | 1.00 | 28.34 | O |
| ATOM | 50 | N | SER | A | 7 | -1.343 | -10.498 | 13.190 | 1.00 | 28.18 | N |
| ATOM | 51 | CA | SER | A | 7 | -0.769 | -10.018 | 11.941 | 1.00 | 37.96 | C |
| ATOM | 52 | C | SER | A | 7 | -1.529 | -8.784 | 11.456 | 1.00 | 36.31 | C |
| ATOM | 53 | O | SER | A | 7 | -2.504 | -8.356 | 12.088 | 1.00 | 29.94 | O |
| ATOM | 54 | CB | SER | A | 7 | -0.782 | -11.125 | 10.884 | 1.00 | 31.07 | C |
| ATOM | 55 | OG | SER | A | 7 | -2.003 | -11.840 | 10.917 | 1.00 | 34.72 | O |
| ATOM | 56 | N | GLY | A | 8 | -1.084 | -8.220 | 10.336 | 1.00 | 37.68 | N |
| ATOM | 57 | CA | GLY | A | 8 | -1.579 | -6.932 | 9.880 | 1.00 | 34.93 | C |
| ATOM | 58 | C | GLY | A | 8 | -2.778 | -6.994 | 8.957 | 1.00 | 39.24 | C |
| ATOM | 59 | O | GLY | A | 8 | -3.278 | -8.071 | 8.641 | 1.00 | 43.54 | O |
| ATOM | 60 | N | THR | A | 9 | -3.221 | -5.818 | 8.519 | 1.00 | 33.12 | N |
| ATOM | 61 | CA | THR | A | 9 | -4.417 | -5.660 | 7.698 | 1.00 | 34.90 | C |
| ATOM | 62 | C | THR | A | 9 | -4.411 | -6.499 | 6.424 | 1.00 | 34.53 | C |
| ATOM | 63 | O | THR | A | 9 | -3.369 | -6.699 | 5.809 | 1.00 | 36.17 | O |
| ATOM | 64 | CB | THR | A | 9 | -4.612 | -4.186 | 7.290 | 1.00 | 34.99 | C |
| ATOM | 65 | OG1 | THR | A | 9 | -4.305 | -3.333 | 8.400 | 1.00 | 54.03 | O |
| ATOM | 66 | CG2 | THR | A | 9 | -6.032 | -3.948 | 6.877 | 1.00 | 25.09 | C |
| ATOM | 67 | N | GLU | A | 10 | -5.591 | -6.977 | 6.042 | 1.00 | 38.28 | N |
| ATOM | 68 | CA | GLU | A | 10 | -5.770 | -7.781 | 4.840 | 1.00 | 36.11 | C |

(continued)

| | | | | | | | | | | | |
|---|---|---|---|---|---|---|---|---|---|---|---|
| ATOM | 69 | C | GLU | A | 10 | -6.846 | -7.183 | 3.942 | 1.00 | 42.38 | C |
| ATOM | 70 | O | GLU | A | 10 | -7.825 | -6.614 | 4.433 | 1.00 | 36.65 | O |
| ATOM | 71 | CB | GLU | A | 10 | -6.156 | -9.217 | 5.204 | 1.00 | 41.15 | C |
| ATOM | 72 | CG | GLU | A | 10 | -5.177 | -9.938 | 6.112 | 1.00 | 45.12 | C |
| ATOM | 73 | CD | GLU | A | 10 | -4.128 | -10.719 | 5.342 | 1.00 | 54.57 | C |
| ATOM | 74 | OE1 | GLU | A | 10 | -3.998 | -10.501 | 4.118 | 1.00 | 60.79 | O |
| ATOM | 75 | OE2 | GLU | A | 10 | -3.438 | -11.556 | 5.963 | 1.00 | 51.03 | O |
| ATOM | 76 | N | VAL | A | 11 | -6.659 | -7.314 | 2.630 | 1.00 | 33.01 | N |
| ATOM | 77 | CA | VAL | A | 11 | -7.690 | -6.970 | 1.654 | 1.00 | 35.41 | C |
| ATOM | 78 | C | VAL | A | 11 | -7.886 | -8.152 | 0.709 | 1.00 | 37.75 | C |
| ATOM | 79 | O | VAL | A | 11 | -6.920 | -8.678 | 0.153 | 1.00 | 40.66 | O |
| ATOM | 80 | CB | VAL | A | 11 | -7.331 | -5.718 | 0.826 | 1.00 | 41.44 | C |
| ATOM | 81 | CG1 | VAL | A | 11 | -8.587 | -5.137 | 0.181 | 1.00 | 34.49 | C |
| ATOM | 82 | CG2 | VAL | A | 11 | -6.642 | -4.681 | 1.692 | 1.00 | 43.75 | C |
| ATOM | 83 | N | LYS | A | 12 | -9.133 | -8.572 | 0.535 | 1.00 | 35.34 | N |
| ATOM | 84 | CA | LYS | A | 12 | -9.430 | -9.745 | -0.276 | 1.00 | 34.88 | C |
| ATOM | 85 | C | LYS | A | 12 | -10.619 | -9.497 | -1.196 | 1.00 | 41.54 | C |
| ATOM | 86 | O | LYS | A | 12 | -11.503 | -8.701 | -0.881 | 1.00 | 37.51 | O |
| ATOM | 87 | CB | LYS | A | 12 | -9.716 | -10.958 | 0.618 | 1.00 | 37.30 | C |
| ATOM | 88 | CG | LYS | A | 12 | -8.570 | -11.375 | 1.529 | 1.00 | 31.97 | C |
| ATOM | 89 | CD | LYS | A | 12 | -7.396 | -11.926 | 0.737 | 1.00 | 39.11 | C |
| ATOM | 90 | CE | LYS | A | 12 | -6.276 | -12.372 | 1.664 | 1.00 | 40.25 | C |
| ATOM | 91 | NZ | LYS | A | 12 | -5.084 | -12.848 | 0.910 | 1.00 | 43.21 | N |
| ATOM | 92 | N | LYS | A | 13 | -10.641 | -10.186 | -2.332 | 1.00 | 40.85 | N |
| ATOM | 93 | CA | LYS | A | 13 | -11.800 | -10.147 | -3.217 | 1.00 | 46.07 | C |
| ATOM | 94 | C | LYS | A | 13 | -12.873 | -11.095 | -2.687 | 1.00 | 41.63 | C |
| ATOM | 95 | O | LYS | A | 13 | -12.557 | -12.058 | -1.991 | 1.00 | 42.80 | O |
| ATOM | 96 | CB | LYS | A | 13 | -11.401 | -10.525 | -4.646 | 1.00 | 44.49 | C |
| ATOM | 97 | CG | LYS | A | 13 | -10.368 | -9.597 | -5.262 | 1.00 | 51.17 | C |
| ATOM | 98 | CD | LYS | A | 13 | -10.037 | -10.015 | -6.686 | 1.00 | 69.57 | C |
| ATOM | 99 | CE | LYS | A | 13 | -9.093 | -9.026 | -7.351 | 1.00 | 76.15 | C |
| ATOM | 100 | NZ | LYS | A | 13 | -8.805 | -9.405 | -8.763 | 1.00 | 81.80 | N |
| ATOM | 101 | N | PRO | A | 14 | -14.149 | -10.815 | -2.992 | 1.00 | 40.61 | N |
| ATOM | 102 | CA | PRO | A | 14 | -15.210 | -11.741 | -2.581 | 1.00 | 42.09 | C |

(continued)

| | | | | | | | | | | | |
|---|---|---|---|---|---|---|---|---|---|---|---|
| ATOM | 103 | C | PRO | A | 14 | -14.959 | -13.147 | -3.124 | 1.00 | 43.49 | C |
| ATOM | 104 | O | PRO | A | 14 | -14.532 | -13.286 | -4.268 | 1.00 | 43.97 | O |
| ATOM | 105 | CB | PRO | A | 14 | -16.474 | -11.125 | -3.191 | 1.00 | 41.78 | C |
| ATOM | 106 | CG | PRO | A | 14 | -16.153 | -9.669 | -3.327 | 1.00 | 42.84 | C |
| ATOM | 107 | CD | PRO | A | 14 | -14.683 | -9.605 | -3.641 | 1.00 | 40.32 | C |
| ATOM | 108 | N | GLY | A | 15 | -15.191 | -14.166 | -2.302 | 1.00 | 44.15 | N |
| ATOM | 109 | CA | GLY | A | 15 | -14.989 | -15.542 | -2.716 | 1.00 | 39.13 | C |
| ATOM | 110 | C | GLY | A | 15 | -13.611 | -16.090 | -2.391 | 1.00 | 48.08 | C |
| ATOM | 111 | O | GLY | A | 15 | -13.385 | -17.297 | -2.469 | 1.00 | 45.50 | O |
| ATOM | 112 | N | ALA | A | 16 | -12.683 | -15.208 | -2.028 | 1.00 | 41.49 | N |
| ATOM | 113 | CA | ALA | A | 16 | -11.334 | -15.636 | -1.665 | 1.00 | 40.38 | C |
| ATOM | 114 | C | ALA | A | 16 | -11.263 | -16.095 | -0.209 | 1.00 | 44.65 | C |
| ATOM | 115 | O | ALA | A | 16 | -12.281 | -16.184 | 0.478 | 1.00 | 39.46 | O |
| ATOM | 116 | CB | ALA | A | 16 | -10.340 | -14.512 | -1.908 | 1.00 | 42.69 | C |
| ATOM | 117 | N | SER | A | 17 | -10.052 | -16.383 | 0.257 | 1.00 | 41.27 | N |
| ATOM | 118 | C | SER | A | 17 | -8.822 | -15.906 | 2.328 | 1.00 | 41.94 | C |
| ATOM | 119 | O | SER | A | 17 | -7.925 | -15.363 | 1.688 | 1.00 | 46.41 | O |
| ATOM | 120 | CA | ASER | A | 17 | -9.841 | -16.800 | 1.638 | 0.70 | 41.50 | C |
| ATOM | 121 | CB | ASER | A | 17 | -9.382 | -18.258 | 1.699 | 0.70 | 42.17 | C |
| ATOM | 122 | OG | ASER | A | 17 | -10.402 | -19.130 | 1.247 | 0.70 | 48.56 | O |
| ATOM | 123 | CA | BSER | A | 17 | -9.844 | -16.800 | 1.638 | 0.30 | 41.51 | C |
| ATOM | 124 | CB | BSER | A | 17 | -9.387 | -18.259 | 1.703 | 0.30 | 42.20 | C |
| ATOM | 125 | OG | BSER | A | 17 | -8.050 | -18.394 | 1.253 | 0.30 | 38.89 | O |
| ATOM | 126 | N | VAL | A | 18 | -8.963 | -15.760 | 3.639 | 1.00 | 40.15 | N |
| ATOM | 127 | CA | VAL | A | 18 | -8.023 | -14.966 | 4.414 | 1.00 | 37.97 | C |
| ATOM | 128 | C | VAL | A | 18 | -7.539 | -15.771 | 5.615 | 1.00 | 36.78 | C |
| ATOM | 129 | O | VAL | A | 18 | -8.313 | -16.499 | 6.237 | 1.00 | 43.11 | O |
| ATOM | 130 | CB | VAL | A | 18 | -8.657 | -13.636 | 4.879 | 1.00 | 39.97 | C |
| ATOM | 131 | CG1 | VAL | A | 18 | -9.911 | -13.893 | 5.706 | 1.00 | 34.36 | C |
| ATOM | 132 | CG2 | VAL | A | 18 | -7.651 | -12.802 | 5.661 | 1.00 | 41.62 | C |
| ATOM | 133 | N | LYS | A | 19 | -6.251 | -15.664 | 5.921 | 1.00 | 39.92 | N |
| ATOM | 134 | CA | LYS | A | 19 | -5.706 | -16.304 | 7.110 | 1.00 | 40.61 | C |
| ATOM | 135 | C | LYS | A | 19 | -5.015 | -15.279 | 8.012 | 1.00 | 41.01 | C |
| ATOM | 136 | O | LYS | A | 19 | -4.067 | -14.610 | 7.599 | 1.00 | 42.89 | O |

(continued)

| | | | | | | | | | | | |
|---|---|---|---|---|---|---|---|---|---|---|---|
| ATOM | 137 | CB | LYS | A | 19 | -4.737 | -17.424 | 6.731 | 1.00 | 38.22 | C |
| ATOM | 138 | CG | LYS | A | 19 | -4.105 | -18.105 | 7.932 | 1.00 | 35.23 | C |
| ATOM | 139 | CD | LYS | A | 19 | -3.153 | -19.219 | 7.527 | 1.00 | 37.78 | C |
| ATOM | 140 | CE | LYS | A | 19 | -3.892 | -20.532 | 7.324 | 1.00 | 52.29 | C |
| ATOM | 141 | NZ | LYS | A | 19 | -2.953 | -21.693 | 7.310 | 1.00 | 57.68 | N |
| ATOM | 142 | N | VAL | A | 20 | -5.511 | -15.165 | 9.241 | 1.00 | 38.03 | N |
| ATOM | 143 | CA | VAL | A | 20 | -4.982 | -14.234 | 10.235 | 1.00 | 34.63 | C |
| ATOM | 144 | C | VAL | A | 20 | -4.240 | -15.005 | 11.332 | 1.00 | 39.64 | C |
| ATOM | 145 | O | VAL | A | 20 | -4.658 | -16.099 | 11.720 | 1.00 | 36.81 | O |
| ATOM | 146 | CB | VAL | A | 20 | -6.115 | -13.388 | 10.867 | 1.00 | 33.08 | C |
| ATOM | 147 | CG1 | VAL | A | 20 | -5.553 | -12.359 | 11.839 | 1.00 | 39.43 | C |
| ATOM | 148 | CG2 | VAL | A | 20 | -6.941 | -12.707 | 9.784 | 1.00 | 38.82 | C |
| ATOM | 149 | N | SER | A | 21 | -3.144 | -14.439 | 11 . 832 | 1.00 | 30.21 | N |
| ATOM | 150 | CA | SER | A | 21 | -2.338 | -15.124 | 12.838 | 1.00 | 32.22 | C |
| ATOM | 151 | C | SER | A | 21 | -2.354 | -14.384 | 14.168 | 1.00 | 34.12 | C |
| ATOM | 152 | O | SER | A | 21 | -2.566 | -13.176 | 14.218 | 1.00 | 32.94 | O |
| ATOM | 153 | CB | SER | A | 21 | -0.892 | -15.289 | 12.354 | 1.00 | 32.38 | C |
| ATOM | 154 | OG | SER | A | 21 | -0.205 | -14.046 | 12.353 | 1.00 | 35.31 | O |
| ATOM | 155 | N | CYS | A | 22 | -2.116 | -15.123 | 15.244 | 1.00 | 36.35 | N |
| ATOM | 156 | CA | CYS | A | 22 | -2.109 | -14.565 | 16.587 | 1.00 | 33.43 | C |
| ATOM | 157 | C | CYS | A | 22 | -0.985 | -15.204 | 17.401 | 1.00 | 38.78 | C |
| ATOM | 158 | O | CYS | A | 22 | -1.059 | -16.380 | 17.757 | 1.00 | 39.38 | O |
| ATOM | 159 | CB | CYS | A | 22 | -3.468 | -14.791 | 17.260 | 1.00 | 38.36 | C |
| ATOM | 160 | SG | CYS | A | 22 | -3.592 | -14.210 | 18.969 | 1.00 | 47.87 | S |
| ATOM | 161 | N | LYS | A | 23 | 0.064 | -14.440 | 17.681 | 1.00 | 37.86 | N |
| ATOM | 162 | CA | LYS | A | 23 | 1.207 | -14.980 | 18.410 | 1.00 | 40.02 | C |
| ATOM | 163 | C | LYS | A | 23 | 1.019 | -14 . 817 | 19.912 | 1.00 | 36.84 | C |
| ATOM | 164 | O | LYS | A | 23 | 0.925 | -13.697 | 20.415 | 1.00 | 38.22 | O |
| ATOM | 165 | CB | LYS | A | 23 | 2.506 | -14.303 | 17.966 | 1.00 | 40.53 | C |
| ATOM | 166 | CG | LYS | A | 23 | 3.763 | -15.022 | 18.446 | 1.00 | 49.89 | C |
| ATOM | 167 | CD | LYS | A | 23 | 5.015 | -14.206 | 18.167 | 1.00 | 49.82 | C |
| ATOM | 168 | CE | LYS | A | 23 | 6.195 | -15.104 | 17.823 | 1.00 | 63.34 | C |
| ATOM | 169 | NZ | LYS | A | 23 | 6.392 | -16.183 | 18.827 | 1.00 | 59.88 | N |
| ATOM | 170 | N | ALA | A | 24 | 0.960 | -15.940 | 20.621 | 1.00 | 39.67 | N |

(continued)

| | | | | | | | | | | | |
|---|---|---|---|---|---|---|---|---|---|---|---|
| ATOM | 171 | CA | ALA | A | 24 | 0.756 | -15.929 | 22.066 | 1.00 | 44.74 | C |
| ATOM | 172 | C | ALA | A | 24 | 2.070 | -16.103 | 22.819 | 1.00 | 52.29 | C |
| ATOM | 173 | O | ALA | A | 24 | 2.952 | -16.844 | 22.390 | 1.00 | 57.40 | O |
| ATOM | 174 | CB | ALA | A | 24 | -0.227 | -17.014 | 22.468 | 1.00 | 43.19 | C |
| ATOM | 175 | N | SER | A | 25 | 2.193 | -15.416 | 23.949 | 1.00 | 49.70 | N |
| ATOM | 176 | CA | SER | A | 25 | 3.390 | -15.511 | 24.771 | 1.00 | 51.40 | C |
| ATOM | 177 | C | SER | A | 25 | 3.108 | -15.073 | 26.206 | 1.00 | 52.18 | C |
| ATOM | 178 | O | SER | A | 25 | 2.195 | -14.282 | 26.449 | 1.00 | 44.33 | O |
| ATOM | 179 | CB | SER | A | 25 | 4.513 | -14.665 | 24.171 | 1.00 | 54.47 | C |
| ATOM | 180 | OG | SER | A | 25 | 4.108 | -13.314 | 24.022 | 1.00 | 61.15 | O |
| ATOM | 181 | N | GLY | A | 26 | 3.887 | -15.597 | 27.149 | 1.00 | 43.62 | N |
| ATOM | 182 | CA | GLY | A | 26 | 3.797 | -15.182 | 28.539 | 1.00 | 42.96 | C |
| ATOM | 183 | C | GLY | A | 26 | 2.914 | -16.042 | 29.429 | 1.00 | 47.24 | C |
| ATOM | 184 | O | GLY | A | 26 | 2.711 | -15.718 | 30.599 | 1.00 | 47.28 | O |
| ATOM | 185 | N | TYR | A | 27 | 2.388 | -17.136 | 28.889 | 1.00 | 41.19 | N |
| ATOM | 186 | CA | TYR | A | 27 | 1.504 | -18.006 | 29.657 | 1.00 | 39.91 | C |
| ATOM | 187 | C | TYR | A | 27 | 2.259 | -18.809 | 30.704 | 1.00 | 53.27 | C |
| ATOM | 188 | O | TYR | A | 27 | 3.307 | -19.393 | 30.423 | 1.00 | 48.93 | O |
| ATOM | 189 | CB | TYR | A | 27 | 0.740 | -18.956 | 28.733 | 1.00 | 48.01 | C |
| ATOM | 190 | CG | TYR | A | 27 | -0.376 | -18.280 | 27.978 | 1.00 | 52.64 | C |
| ATOM | 191 | CD2 | TYR | A | 27 | -0.151 | -17.727 | 26.726 | 1.00 | 45.16 | C |
| ATOM | 192 | CD1 | TYR | A | 27 | -1.655 | -18.180 | 28.526 | 1.00 | 47.65 | C |
| ATOM | 193 | CE2 | TYR | A | 27 | -1.163 | -17.099 | 26.032 | 1.00 | 50.12 | C |
| ATOM | 194 | CE1 | TYR | A | 27 | -2.678 | -17.555 | 27.837 | 1.00 | 41.89 | C |
| ATOM | 195 | CZ | TYR | A | 27 | -2.422 | -17.015 | 26.590 | 1.00 | 49.26 | C |
| ATOM | 196 | OH | TYR | A | 27 | -3.417 | -16.385 | 25.887 | 1.00 | 50.17 | O |
| ATOM | 197 | N | THR | A | 28 | 1.705 | -18.844 | 31.910 | 1.00 | 55.54 | N |
| ATOM | 198 | CA | THR | A | 28 | 2.345 | -19.511 | 33.035 | 1.00 | 52.97 | C |
| ATOM | 199 | C | THR | A | 28 | 1.695 | -20.867 | 33.330 | 1.00 | 58.74 | C |
| ATOM | 200 | O | THR | A | 28 | 2.080 | -21.560 | 34.275 | 1.00 | 53.17 | O |
| ATOM | 201 | CB | THR | A | 28 | 2.301 | -18.622 | 34.297 | 1.00 | 45.57 | C |
| ATOM | 202 | CG2 | THR | A | 28 | 0.870 | -18.237 | 34.629 | 1.00 | 50.36 | C |
| ATOM | 203 | OG1 | THR | A | 28 | 2.873 | -19.326 | 35.405 | 1.00 | 80.42 | O |
| ATOM | 204 | O | PHE | A | 29 | -0.347 | -22.422 | 30.294 | 1.00 | 42.50 | O |

(continued)

| | | | | | | | | | | | |
|---|---|---|---|---|---|---|---|---|---|---|---|
| ATOM | 205 | N | PHE | A | 29 | 0.711 | -21.242 | 32.516 | 1.00 | 52.37 | N |
| ATOM | 206 | CA | PHE | A | 29 | 0.048 | -22.536 | 32.661 | 1.00 | 40.27 | C |
| ATOM | 207 | C | PHE | A | 29 | -0.288 | -23.133 | 31.297 | 1.00 | 41.00 | C |
| ATOM | 208 | CB | PHE | A | 29 | -1.216 | -22.409 | 33.519 | 1.00 | 44.95 | C |
| ATOM | 209 | CG | PHE | A | 29 | -2.147 | -21.312 | 33.081 | 1.00 | 42.97 | C |
| ATOM | 210 | CD2 | PHE | A | 29 | -2.146 | -20.087 | 33.728 | 1.00 | 44.83 | C |
| ATOM | 211 | CD1 | PHE | A | 29 | -3.034 | -21.512 | 32.034 | 1.00 | 41.74 | C |
| ATOM | 212 | CE2 | PHE | A | 29 | -3.005 | -19.077 | 33.334 | 1.00 | 49.55 | C |
| ATOM | 213 | CE1 | PHE | A | 29 | -3.893 | -20.508 | 31.635 | 1.00 | 43.96 | C |
| ATOM | 214 | CZ | PHE | A | 29 | -3.879 | -19.287 | 32.286 | 1.00 | 50.75 | C |
| ATOM | 215 | O | THR | A | 30 | -1.919 | -25.101 | 28.021 | 1.00 | 42.53 | O |
| ATOM | 216 | N | THR | A | 30 | -0.530 | -24.439 | 31.272 | 1.00 | 40.36 | N |
| ATOM | 217 | CA | THR | A | 30 | -0.586 | -25.179 | 30.016 | 1.00 | 42.39 | C |
| ATOM | 218 | C | THR | A | 30 | -1.914 | -25.074 | 29.255 | 1.00 | 42.49 | C |
| ATOM | 219 | CB | THR | A | 30 | -0.281 | -26.682 | 30.251 | 1.00 | 43.93 | C |
| ATOM | 220 | OG1 | THR | A | 30 | -1.295 | -27.254 | 31.083 | 1.00 | 62.78 | O |
| ATOM | 221 | CG2 | THR | A | 30 | 1.071 | -26.857 | 30.923 | 1.00 | 45.80 | C |
| ATOM | 222 | O | ASN | A | 31 | -5.415 | -23.080 | 30.210 | 1.00 | 32.62 | O |
| ATOM | 223 | N | ASN | A | 31 | -3.035 | -24.959 | 29.965 | 1.00 | 33.58 | N |
| ATOM | 224 | CA | ASN | A | 31 | -4.334 | -25.021 | 29.288 | 1.00 | 29.92 | C |
| ATOM | 225 | C | ASN | A | 31 | -5.085 | -23.699 | 29.201 | 1.00 | 35.69 | C |
| ATOM | 226 | CB | ASN | A | 31 | -5.230 | -26.055 | 29.963 | 1.00 | 36.07 | C |
| ATOM | 227 | CG | ASN | A | 31 | -4.646 | -27.449 | 29.903 | 1.00 | 37.13 | C |
| ATOM | 228 | OD1 | ASN | A | 31 | -4.449 | -28.006 | 28.821 | 1.00 | 32.81 | O |
| ATOM | 229 | ND2 | ASN | A | 31 | -4.361 | -28.018 | 31.066 | 1.00 | 28.91 | N |
| ATOM | 230 | N | TYR | A | 32 | -5.354 | -23.289 | 27.968 | 1.00 | 31.28 | N |
| ATOM | 231 | CA | TYR | A | 32 | -6.159 | -22.112 | 27.677 | 1.00 | 34.89 | C |
| ATOM | 232 | C | TYR | A | 32 | -6.695 | -22.261 | 26.258 | 1.00 | 37.67 | C |
| ATOM | 233 | O | TYR | A | 32 | -6.197 | -23.085 | 25.491 | 1.00 | 29.11 | O |
| ATOM | 234 | CB | TYR | A | 32 | -5.339 | -20.825 | 27.826 | 1.00 | 30.61 | C |
| ATOM | 235 | CG | TYR | A | 32 | -4.090 | -20.799 | 26.970 | 1.00 | 42.67 | C |
| ATOM | 236 | CD1 | TYR | A | 32 | -2.900 | -21.355 | 27.425 | 1.00 | 39.54 | C |
| ATOM | 237 | CD2 | TYR | A | 32 | -4.101 | -20.225 | 25.704 | 1.00 | 38.42 | C |
| ATOM | 238 | CE1 | TYR | A | 32 | -1.758 | -21.341 | 26.643 | 1.00 | 41.78 | C |

(continued)

| | | | | | | | | | | |
|---|---|---|---|---|---|---|---|---|---|---|
| ATOM | 239 | CE2 | TYR | A | 32 | -2.962 | -20.206 | 24.921 | 1.00 | 42.06 | C |
| ATOM | 240 | CZ | TYR | A | 32 | -1.797 | -20.765 | 25.393 | 1.00 | 47.23 | C |
| ATOM | 241 | OH | TYR | A | 32 | -0.666 | -20.741 | 24.609 | 1.00 | 56.16 | O |
| ATOM | 242 | N | ASP | A | 33 | -7.716 | -21.482 | 25.919 | 1.00 | 30.49 | N |
| ATOM | 243 | CA | ASP | A | 33 | -8.271 | -21.484 | 24.570 | 1.00 | 29.65 | C |
| ATOM | 244 | C | ASP | A | 33 | -7.998 | -20.161 | 23.883 | 1.00 | 31.10 | C |
| ATOM | 245 | O | ASP | A | 33 | -8.062 | -19.113 | 24.513 | 1.00 | 28.34 | O |
| ATOM | 246 | CB | ASP | A | 33 | -9.779 | -21.734 | 24.591 | 1.00 | 29.08 | C |
| ATOM | 247 | CG | ASP | A | 33 | -10.139 | -23.124 | 25.082 | 1.00 | 34.81 | C |
| ATOM | 248 | OD1 | ASP | A | 33 | -9.427 | -24.090 | 24.729 | 1.00 | 31.12 | O |
| ATOM | 249 | OD2 | ASP | A | 33 | -11.145 | -23.247 | 25.814 | 1.00 | 30.55 | O |
| ATOM | 250 | N | ILE | A | 34 | -7.685 | -20.204 | 22.593 | 1.00 | 28.80 | N |
| ATOM | 251 | CA | ILE | A | 34 | -7.698 | -18.989 | 21.801 | 1.00 | 29.45 | C |
| ATOM | 252 | C | ILE | A | 34 | -9.070 | -18.897 | 21.155 | 1.00 | 28.08 | C |
| ATOM | 253 | O | ILE | A | 34 | -9.518 | -19.838 | 20.500 | 1.00 | 32.08 | O |
| ATOM | 254 | CB | ILE | A | 34 | -6.587 | -18.954 | 20.729 | 1.00 | 34.02 | C |
| ATOM | 255 | CG1 | ILE | A | 34 | -5.301 | -18.380 | 21.315 | 1.00 | 40.10 | C |
| ATOM | 256 | CG2 | ILE | A | 34 | -6.981 | -18.048 | 19.581 | 1.00 | 33.89 | C |
| ATOM | 257 | CD1 | ILE | A | 34 | -4.555 | -19.327 | 22.160 | 1.00 | 37.52 | C |
| ATOM | 258 | N | ASN | A | 35 | -9.743 | -17.774 | 21.381 | 1.00 | 23.45 | N |
| ATOM | 259 | CA | ASN | A | 35 | -11.044 | -17.502 | 20.786 | 1.00 | 28.14 | C |
| ATOM | 260 | C | ASN | A | 35 | -10.911 | -16.447 | 19.693 | 1.00 | 30.81 | C |
| ATOM | 261 | O | ASN | A | 35 | -10.013 | -15.607 | 19.733 | 1.00 | 33.76 | O |
| ATOM | 262 | CB | ASN | A | 35 | -12.045 | -17.023 | 21.849 | 1.00 | 30.44 | C |
| ATOM | 263 | CG | ASN | A | 35 | -12.218 | -18.015 | 22.989 | 1.00 | 30.22 | C |
| ATOM | 264 | OD1 | ASN | A | 35 | -13.183 | -18.779 | 23.018 | 1.00 | 29.29 | O |
| ATOM | 265 | ND2 | ASN | A | 35 | -11.293 | -17.995 | 23.943 | 1.00 | 26.20 | N |
| ATOM | 266 | N | TRP | A | 36 | -11.802 | -16.482 | 18.715 | 1.00 | 28.47 | N |
| ATOM | 267 | CA | TRP | A | 36 | -11.807 | -15.447 | 17.697 | 1.00 | 32.59 | C |
| ATOM | 268 | C | TRP | A | 36 | -13.139 | -14.720 | 17.718 | 1.00 | 32.65 | C |
| ATOM | 269 | O | TRP | A | 36 | -14.202 | -15.337 | 17.703 | 1.00 | 30.35 | O |
| ATOM | 270 | CB | TRP | A | 36 | -11.509 | -16.038 | 16.318 | 1.00 | 30.51 | C |
| ATOM | 271 | CG | TRP | A | 36 | -10.106 | -16.553 | 16.242 | 1.00 | 27.75 | C |
| ATOM | 272 | CD1 | TRP | A | 36 | -9.665 | -17.781 | 16.637 | 1.00 | 28.80 | C |

(continued)

| | | | | | | | | | | | |
|---|---|---|---|---|---|---|---|---|---|---|---|
| ATOM | 273 | CD2 | TRP | A | 36 | -8.951 | -15.840 | 15.779 | 1.00 | 33.27 | C |
| ATOM | 274 | CE2 | TRP | A | 36 | -7 . 847 | -16.707 | 15.906 | 1.00 | 31.39 | C |
| ATOM | 275 | CE3 | TRP | A | 36 | -8.745 | -14.557 | 15.260 | 1.00 | 31.98 | C |
| ATOM | 276 | NE1 | TRP | A | 36 | -8.309 | -17.884 | 16.434 | 1.00 | 36.72 | N |
| ATOM | 277 | CZ2 | TRP | A | 36 | -6.554 | -16.334 | 15.535 | 1.00 | 38.10 | C |
| ATOM | 278 | CZ3 | TRP | A | 36 | -7.457 | -14.186 | 14 .890 | 1.00 | 30.43 | C |
| ATOM | 279 | CH2 | TRP | A | 36 | -6.380 | -15.071 | 15.030 | 1.00 | 33.42 | C |
| ATOM | 280 | N | VAL | A | 37 | -13.053 | -13.397 | 17.790 | 1.00 | 27.11 | N |
| ATOM | 281 | CA | VAL | A | 37 | -14.207 | -12.528 | 17.936 | 1.00 | 30.57 | C |
| ATOM | 282 | C | VAL | A | 37 | -14.076 | -11.410 | 16.914 | 1.00 | 33.89 | C |
| ATOM | 283 | O | VAL | A | 37 | -13.032 | -10.759 | 16.846 | 1.00 | 33.58 | O |
| ATOM | 284 | CB | VAL | A | 37 | -14.301 | -11.929 | 19.370 | 1.00 | 25.89 | C |
| ATOM | 285 | CG1 | VAL | A | 37 | -15.467 | -10.957 | 19.478 | 1.00 | 27.13 | C |
| ATOM | 286 | CG2 | VAL | A | 37 | -14.423 | -13.031 | 20.417 | 1.00 | 26.86 | C |
| ATOM | 287 | N | ARG | A | 38 | -15.116 | -11.188 | 16.115 | 1.00 | 29.31 | N |
| ATOM | 288 | CA | ARG | A | 38 | -15.054 | -10.150 | 15.088 | 1.00 | 28.87 | C |
| ATOM | 289 | C | ARG | A | 38 | -16.002 | -8.995 | 15.395 | 1.00 | 32.02 | C |
| ATOM | 290 | O | ARG | A | 38 | -17.005 | -9.163 | 16.089 | 1.00 | 30.64 | O |
| ATOM | 291 | CB | ARG | A | 38 | -15.367 | -10.732 | 13.708 | 1.00 | 35.52 | C |
| ATOM | 292 | CG | ARG | A | 38 | -16.652 | -11.529 | 13.656 | 1.00 | 39.93 | C |
| ATOM | 293 | CD | ARG | A | 38 | -17.550 | -11.096 | 12.506 | 1.00 | 43.60 | C |
| ATOM | 294 | NE | ARG | A | 38 | -17.164 | -11.681 | 11.229 | 1.00 | 44.71 | N |
| ATOM | 295 | CZ | ARG | A | 38 | -18.023 | -12.119 | 10.311 | 1.00 | 45.96 | C |
| ATOM | 296 | NH1 | ARG | A | 38 | -19.329 | -12.055 | 10.529 | 1.00 | 39.60 | N |
| ATOM | 297 | NH2 | ARG | A | 38 | -17.572 | -12.626 | 9.170 | 1.00 | 33.94 | N |
| ATOM | 298 | N | GLN | A | 39 | -15.669 | -7.821 | 14 . 871 | 1.00 | 30.63 | N |
| ATOM | 299 | CA | GLN | A | 39 | -16.452 | -6.618 | 15.103 | 1.00 | 28.53 | C |
| ATOM | 300 | C | GLN | A | 39 | -16.568 | -5.795 | 13.825 | 1.00 | 31.84 | C |
| ATOM | 301 | O | GLN | A | 39 | -15.569 | -5.314 | 13.294 | 1.00 | 29.51 | O |
| ATOM | 302 | CB | GLN | A | 39 | -15.819 | -5.775 | 16.219 | 1.00 | 30.14 | C |
| ATOM | 303 | CG | GLN | A | 39 | -16.516 | -4.444 | 16.478 | 1.00 | 28.25 | C |
| ATOM | 304 | CD | GLN | A | 39 | -16.024 | -3.781 | 17.751 | 1.00 | 39.30 | C |
| ATOM | 305 | NE2 | GLN | A | 39 | -16.896 | -3.689 | 18.751 | 1.00 | 30.37 | N |
| ATOM | 306 | OE1 | GLN | A | 39 | -14.870 | -3.368 | 17.839 | 1.00 | 39.08 | O |

(continued)

| | | | | | | | | | | | |
|---|---|---|---|---|---|---|---|---|---|---|---|
| ATOM | 307 | N | ALA | A | 40 | -17.788 | -5.637 | 13.330 | 1.00 | 37.04 | N |
| ATOM | 308 | CA | ALA | A | 40 | -18.016 | -4.815 | 12.149 | 1.00 | 44.35 | C |
| ATOM | 309 | C | ALA | A | 40 | -18.259 | -3.363 | 12.548 | 1.00 | 49.15 | C |
| ATOM | 310 | O | ALA | A | 40 | -18.880 | -3.094 | 13.579 | 1.00 | 49.51 | O |
| ATOM | 311 | CB | ALA | A | 40 | -19.190 | -5.352 | 11.341 | 1.00 | 36.53 | C |
| ATOM | 312 | N | THR | A | 41 | -17.768 | -2.444 | 11.715 | 1.00 | 55.43 | N |
| ATOM | 313 | CA | THR | A | 41 | -17.923 | -0.987 | 11.865 | 1.00 | 45.13 | C |
| ATOM | 314 | C | THR | A | 41 | -17.917 | -0.449 | 13.304 | 1.00 | 61.05 | C |
| ATOM | 315 | O | THR | A | 41 | -18.779 | 0.345 | 13.688 | 1.00 | 60.70 | O |
| ATOM | 316 | CB | THR | A | 41 | -19.224 | -0.480 | 11.157 | 1.00 | 56.54 | C |
| ATOM | 317 | OG1 | THR | A | 41 | -19.398 | 0.918 | 11.416 | 1.00 | 74.65 | O |
| ATOM | 318 | CG2 | THR | A | 41 | -20.475 | -1.233 | 11.611 | 1.00 | 50.35 | C |
| ATOM | 319 | N | GLY | A | 42 | -16.931 | -0.880 | 14.088 | 1.00 | 60.31 | N |
| ATOM | 320 | CA | GLY | A | 42 | -16.677 | -0.311 | 15.402 | 1.00 | 50.20 | C |
| ATOM | 321 | C | GLY | A | 42 | -17.674 | -0.603 | 16.515 | 1.00 | 58.90 | C |
| ATOM | 322 | O | GLY | A | 42 | -17.496 | -0.125 | 17.638 | 1.00 | 64.22 | O |
| ATOM | 323 | N | GLN | A | 43 | -18.718 | -1.377 | 16.225 | 1.00 | 61.17 | N |
| ATOM | 324 | CA | GLN | A | 43 | -19.700 | -1.716 | 17.255 | 1.00 | 58.73 | C |
| ATOM | 325 | C | GLN | A | 43 | -20.368 | -3.072 | 17.007 | 1.00 | 47.27 | C |
| ATOM | 326 | O | GLN | A | 43 | -20.811 | -3.369 | 15.897 | 1.00 | 54.96 | O |
| ATOM | 327 | CB | GLN | A | 43 | -20.761 | -0.613 | 17.362 | 1.00 | 57.68 | C |
| ATOM | 328 | CG | GLN | A | 43 | -21.613 | -0.690 | 18.627 | 1.00 | 55.99 | C |
| ATOM | 329 | CD | GLN | A | 43 | -22.130 | 0.669 | 19.075 | 1.00 | 60.55 | C |
| ATOM | 330 | NE2 | GLN | A | 43 | -21.668 | 1.727 | 18.416 | 1.00 | 61.26 | N |
| ATOM | 331 | OE1 | GLN | A | 43 | -22.929 | 0.765 | 20.007 | 1.00 | 62.03 | O |
| ATOM | 332 | N | GLY | A | 44 | -20.438 | -3.885 | 18.059 | 1.00 | 52.11 | N |
| ATOM | 333 | CA | GLY | A | 44 | -21.017 | -5.215 | 17.978 | 1.00 | 38.87 | C |
| ATOM | 334 | C | GLY | A | 44 | -19.946 | -6.290 | 17.933 | 1.00 | 40.89 | C |
| ATOM | 335 | O | GLY | A | 44 | -19.033 | -6.225 | 17.115 | 1.00 | 48.89 | O |
| ATOM | 336 | N | LEU | A | 45 | -20.051 | -7.281 | 18.811 | 1.00 | 28.02 | N |
| ATOM | 337 | CA | LEU | A | 45 | -19.065 | -8.355 | 18.862 | 1.00 | 32.48 | C |
| ATOM | 338 | C | LEU | A | 45 | -19.726 | -9.703 | 18.639 | 1.00 | 29.29 | C |
| ATOM | 339 | O | LEU | A | 45 | -20.768 | -9.993 | 19.226 | 1.00 | 35.37 | O |
| ATOM | 340 | CB | LEU | A | 45 | -18.331 | -8.345 | 20.209 | 1.00 | 30.66 | C |

(continued)

| | | | | | | | | | | | |
|---|---|---|---|---|---|---|---|---|---|---|---|
| ATOM | 341 | CG | LEU | A | 45 | -17.553 | -7.072 | 20.561 | 1.00 | 31.07 | C |
| ATOM | 342 | CD1 | LEU | A | 45 | -17.379 | -6.937 | 22.068 | 1.00 | 32.58 | C |
| ATOM | 343 | CD2 | LEU | A | 45 | -16.198 | -7.075 | 19.873 | 1.00 | 29.40 | C |
| ATOM | 344 | N | GLU | A | 46 | -19.140 | -10.533 | 17.785 | 1.00 | 29.17 | N |
| ATOM | 345 | CA | GLU | A | 46 | -19.639 | -11.895 | 17.673 | 1.00 | 35.62 | C |
| ATOM | 346 | C | GLU | A | 46 | -18.513 | -12.920 | 17.699 | 1.00 | 35.22 | C |
| ATOM | 347 | O | GLU | A | 46 | -17.496 | -12.798 | 17.009 | 1.00 | 32.17 | O |
| ATOM | 348 | CB | GLU | A | 46 | -20.511 | -12.072 | 16.421 | 1.00 | 37.90 | C |
| ATOM | 349 | CG | GLU | A | 46 | -19.828 | -11.914 | 15.092 | 1.00 | 47.11 | C |
| ATOM | 350 | CD | GLU | A | 46 | -20.765 | -12.211 | 13.925 | 1.00 | 55.52 | C |
| ATOM | 351 | OE1 | GLU | A | 46 | -21.675 | -13.052 | 14.087 | 1.00 | 54.51 | O |
| ATOM | 352 | OE2 | GLU | A | 46 | -20.601 | -11.595 | 12.850 | 1.00 | 58.10 | O |
| ATOM | 353 | N | TRP | A | 47 | -18.732 | -13.926 | 18.536 | 1.00 | 32.28 | N |
| ATOM | 354 | CA | TRP | A | 47 | -17.800 | -15.012 | 18.788 | 1.00 | 36.07 | C |
| ATOM | 355 | C | TRP | A | 47 | -17 . 838 | -16.016 | 17.641 | 1.00 | 34.08 | C |
| ATOM | 356 | O | TRP | A | 47 | -18.910 | -16.392 | 17.169 | 1.00 | 29.79 | O |
| ATOM | 357 | CB | TRP | A | 47 | -18.166 | -15.664 | 20.121 | 1.00 | 25.80 | C |
| ATOM | 358 | CG | TRP | A | 47 | -17.311 | -16.791 | 20.594 | 1.00 | 33.81 | C |
| ATOM | 359 | CD1 | TRP | A | 47 | -16.096 | -16.703 | 21.210 | 1.00 | 32.06 | C |
| ATOM | 360 | CD2 | TRP | A | 47 | -17.644 | -18.182 | 20.562 | 1.00 | 33.38 | C |
| ATOM | 361 | CE2 | TRP | A | 47 | -16.572 | -18.882 | 21.149 | 1.00 | 34.18 | C |
| ATOM | 362 | CE3 | TRP | A | 47 | -18.740 | -18.904 | 20.079 | 1.00 | 35.50 | C |
| ATOM | 363 | NE1 | TRP | A | 47 | -15.638 | -17.957 | 21.536 | 1.00 | 29.56 | N |
| ATOM | 364 | CZ2 | TRP | A | 47 | -16.565 | -20.271 | 21.268 | 1.00 | 31.56 | C |
| ATOM | 365 | CZ3 | TRP | A | 47 | -18.729 | -20.282 | 20.196 | 1.00 | 42.35 | C |
| ATOM | 366 | CH2 | TRP | A | 47 | -17.647 | -20.950 | 20.787 | 1.00 | 34.97 | C |
| ATOM | 367 | N | MET | A | 48 | -16.668 | -16.437 | 17.179 | 1.00 | 32.09 | N |
| ATOM | 368 | CA | MET | A | 48 | -16.596 | -17.326 | 16.028 | 1.00 | 30.89 | C |
| ATOM | 369 | C | MET | A | 48 | -16.261 | -18.755 | 16.429 | 1.00 | 29.94 | C |
| ATOM | 370 | O | MET | A | 48 | -16.653 | -19.705 | 15.757 | 1.00 | 33.43 | O |
| ATOM | 371 | CB | MET | A | 48 | -15.558 | -16.823 | 15.028 | 1.00 | 28.48 | C |
| ATOM | 372 | CG | MET | A | 48 | -15.790 | -15.409 | 14.538 | 1.00 | 33.13 | C |
| ATOM | 373 | SD | MET | A | 48 | -14.488 | -14.924 | 13.396 | 1.00 | 37.93 | S |
| ATOM | 374 | CE | MET | A | 48 | -14.665 | -16.165 | 12.119 | 1.00 | 39.94 | C |

(continued)

| | | | | | | | | | | | |
|---|---|---|---|---|---|---|---|---|---|---|---|
| ATOM | 375 | N | GLY | A | 49 | -15.511 | -18.908 | 17.511 | 1.00 | 33.42 | N |
| ATOM | 376 | CA | GLY | A | 49 | -15.128 | -20.234 | 17.955 | 1.00 | 33.05 | C |
| ATOM | 377 | C | GLY | A | 49 | -13.878 | -20.244 | 18.806 | 1.00 | 34.29 | C |
| ATOM | 378 | O | GLY | A | 49 | -13.256 | -19.202 | 19.031 | 1.00 | 34.24 | O |
| ATOM | 379 | N | TRP | A | 50 | -13.509 | -21.429 | 19.279 | 1.00 | 30.14 | N |
| ATOM | 380 | CA | TRP | A | 50 | -12.368 | -21.562 | 20.176 | 1.00 | 27.60 | C |
| ATOM | 381 | C | TRP | A | 50 | -11.393 | -22.619 | 19.679 | 1.00 | 36.00 | C |
| ATOM | 382 | O | TRP | A | 50 | -11.758 | -23.513 | 18.915 | 1.00 | 26.22 | O |
| ATOM | 383 | CB | TRP | A | 50 | -12.833 | -21.906 | 21.595 | 1.00 | 27.32 | C |
| ATOM | 384 | CG | TRP | A | 50 | -13.525 | -23.240 | 21.714 | 1.00 | 31.33 | C |
| ATOM | 385 | CD1 | TRP | A | 50 | -14.867 | -23.479 | 21.627 | 1.00 | 31.09 | C |
| ATOM | 386 | CD2 | TRP | A | 50 | -12.907 | -24.513 | 21.952 | 1.00 | 32.23 | C |
| ATOM | 387 | CE2 | TRP | A | 50 | -13.936 | -25.476 | 21.994 | 1.00 | 36.04 | C |
| ATOM | 388 | CE3 | TRP | A | 50 | -11.583 | -24.932 | 22.133 | 1.00 | 35.42 | C |
| ATOM | 389 | NE1 | TRP | A | 50 | -15.122 | -24.819 | 21.791 | 1.00 | 30.64 | N |
| ATOM | 390 | CZ2 | TRP | A | 50 | -13.684 | -26.834 | 22.208 | 1.00 | 34.17 | C |
| ATOM | 391 | CZ3 | TRP | A | 50 | -11.334 | -26.282 | 22.347 | 1.00 | 33.78 | C |
| ATOM | 392 | CH2 | TRP | A | 50 | -12.379 | -27.215 | 22.381 | 1.00 | 34.68 | C |
| ATOM | 393 | N | MET | A | 51 | -10.147 | -22.507 | 20.120 | 1.00 | 29.16 | N |
| ATOM | 394 | CA | MET | A | 51 | -9.148 | -23.507 | 19.807 | 1.00 | 28.07 | C |
| ATOM | 395 | C | MET | A | 51 | -8.191 | -23.679 | 20.975 | 1.00 | 31.55 | C |
| ATOM | 396 | O | MET | A | 51 | -7.728 | -22.696 | 21.553 | 1.00 | 33.21 | O |
| ATOM | 397 | CB | MET | A | 51 | -8.374 | -23.130 | 18.549 | 1.00 | 31.45 | C |
| ATOM | 398 | CG | MET | A | 51 | -7.420 | -24.221 | 18.093 | 1.00 | 37.56 | C |
| ATOM | 399 | SD | MET | A | 51 | -5.818 | -23.586 | 17.594 | 1.00 | 57.24 | S |
| ATOM | 400 | CE | MET | A | 51 | -5.222 | -22.920 | 19.142 | 1.00 | 46.36 | C |
| ATOM | 401 | N | HIS | A | 52 | -7.902 | -24.935 | 21.305 | 1.00 | 27.88 | N |
| ATOM | 402 | CA | HIS | A | 52 | -6.991 | -25.294 | 22.391 | 1.00 | 35.15 | C |
| ATOM | 403 | C | HIS | A | 52 | -5.615 | -25.621 | 21.816 | 1.00 | 35.88 | C |
| ATOM | 404 | O | HIS | A | 52 | -5.449 | -26.650 | 21.169 | 1.00 | 39.53 | O |
| ATOM | 405 | CB | HIS | A | 52 | -7.557 | -26.491 | 23.173 | 1.00 | 31.19 | C |
| ATOM | 406 | CG | HIS | A | 52 | -6.737 | -26.899 | 24.362 | 1.00 | 32.88 | C |
| ATOM | 407 | CD2 | HIS | A | 52 | -6.353 | -26.213 | 25.462 | 1.00 | 35.83 | C |
| ATOM | 408 | ND1 | HIS | A | 52 | -6.248 | -28.180 | 24.520 | 1.00 | 33.47 | N |

(continued)

| | | | | | | | | | | | |
|---|---|---|---|---|---|---|---|---|---|---|---|
| ATOM | 409 | CE1 | HIS | A | 52 | -5.585 | -28.258 | 25.660 | 1.00 | 32.93 | C |
| ATOM | 410 | NE2 | HIS | A | 52 | -5.631 | -27.080 | 26.252 | 1.00 | 33.96 | N |
| ATOM | 411 | N | PRO | A | 53 | -4.629 | -24.737 | 22.035 | 1.00 | 36.74 | N |
| ATOM | 412 | CA | PRO | A | 53 | -3.284 | -24.901 | 21.465 | 1.00 | 41.48 | C |
| ATOM | 413 | C | PRO | A | 53 | -2.619 | -26.232 | 21.802 | 1.00 | 43.82 | C |
| ATOM | 414 | O | PRO | A | 53 | -1.959 | -26.811 | 20.941 | 1.00 | 42.27 | O |
| ATOM | 415 | CB | PRO | A | 53 | -2.497 | -23.744 | 22.088 | 1.00 | 37.52 | C |
| ATOM | 416 | CG | PRO | A | 53 | -3.521 | -22.710 | 22.372 | 1.00 | 42.21 | C |
| ATOM | 417 | CD | PRO | A | 53 | -4.768 | -23.456 | 22.751 | 1.00 | 37.85 | C |
| ATOM | 418 | N | ASN | A | 54 | -2.795 | -26.709 | 23.029 | 1.00 | 40.74 | N |
| ATOM | 419 | CA | ASN | A | 54 | -2.088 | -27.903 | 23.482 | 1.00 | 40.42 | C |
| ATOM | 420 | C | ASN | A | 54 | -2.575 | -29.192 | 22.814 | 1.00 | 44.96 | C |
| ATOM | 421 | O | ASN | A | 54 | -1.848 | -30.184 | 22.781 | 1.00 | 53.00 | O |
| ATOM | 422 | CB | ASN | A | 54 | -2.201 | -28.035 | 25.003 | 1.00 | 41.59 | C |
| ATOM | 423 | CG | ASN | A | 54 | -0.988 | -28.704 | 25.620 | 1.00 | 48.60 | C |
| ATOM | 424 | ND2 | ASN | A | 54 | -1.220 | -29.565 | 26.607 | 1.00 | 49.53 | N |
| ATOM | 425 | OD1 | ASN | A | 54 | 0.146 | -28.456 | 25.208 | 1.00 | 54.90 | O |
| ATOM | 426 | N | SER | A | 55 | -3.792 | -29.179 | 22.276 | 1.00 | 41.51 | N |
| ATOM | 427 | CA | SER | A | 55 | -4.354 | -30.372 | 21.641 | 1.00 | 39.42 | C |
| ATOM | 428 | C | SER | A | 55 | -4.800 | -30.137 | 20.196 | 1.00 | 45.89 | C |
| ATOM | 429 | O | SER | A | 55 | -4.958 | -31.083 | 19.428 | 1.00 | 45.29 | O |
| ATOM | 430 | CB | SER | A | 55 | -5.544 | -30.886 | 22.444 | 1.00 | 40.90 | C |
| ATOM | 431 | OG | SER | A | 55 | -6.661 | -30.030 | 22.269 | 1.00 | 41.20 | O |
| ATOM | 432 | N | GLY | A | 56 | -5.020 | -28.878 | 19.831 | 1.00 | 39.03 | N |
| ATOM | 433 | CA | GLY | A | 56 | -5.491 | -28.550 | 18.497 | 1.00 | 34.31 | C |
| ATOM | 434 | C | GLY | A | 56 | -6.992 | -28.707 | 18.348 | 1.00 | 33.47 | C |
| ATOM | 435 | O | GLY | A | 56 | -7.551 | -28.402 | 17.298 | 1.00 | 35.63 | O |
| ATOM | 436 | N | ASN | A | 57 | -7.649 | -29.185 | 19.401 | 1.00 | 32.07 | N |
| ATOM | 437 | CA | ASN | A | 57 | -9.099 | -29.323 | 19.384 | 1.00 | 35.35 | C |
| ATOM | 438 | C | ASN | A | 57 | -9.792 | -27.972 | 19.257 | 1.00 | 35.99 | C |
| ATOM | 439 | O | ASN | A | 57 | -9.298 | -26.956 | 19.753 | 1.00 | 31.96 | O |
| ATOM | 440 | CB | ASN | A | 57 | -9.585 | -30.042 | 20.638 | 1.00 | 36.70 | C |
| ATOM | 441 | CG | ASN | A | 57 | -9.149 | -31.490 | 20.679 | 1.00 | 51.73 | C |
| ATOM | 442 | OD1 | ASN | A | 57 | -8.193 | -31.882 | 20.005 | 1.00 | 51.72 | O |

(continued)

| | | | | | | | | | | | |
|---|---|---|---|---|---|---|---|---|---|---|---|
| ATOM | 443 | ND2 | ASN | A | 57 | -9.851 | -32.297 | 21.467 | 1.00 | 40.02 | N |
| ATOM | 444 | N | THR | A | 58 | -10.938 | -27.966 | 18.585 | 1.00 | 32.70 | N |
| ATOM | 445 | CA | THR | A | 58 | -11.657 | -26.728 | 18.315 | 1.00 | 34.35 | C |
| ATOM | 446 | C | THR | A | 58 | -13.149 | -26.874 | 18.543 | 1.00 | 36.07 | C |
| ATOM | 447 | O | THR | A | 58 | -13.664 | -27.985 | 18.682 | 1.00 | 35.59 | O |
| ATOM | 448 | CB | THR | A | 58 | -11.463 | -26.251 | 16.859 | 1.00 | 35.50 | C |
| ATOM | 449 | CG2 | THR | A | 58 | -9.986 | -26.209 | 16.474 | 1.00 | 29.82 | C |
| ATOM | 450 | OG1 | THR | A | 58 | -12.167 | -27.132 | 15.976 | 1.00 | 34.89 | O |
| ATOM | 451 | N | GLY | A | 59 | -13.833 | -25.735 | 18.563 | 1.00 | 34.36 | N |
| ATOM | 452 | CA | GLY | A | 59 | -15.283 | -25.682 | 18.542 | 1.00 | 31.26 | C |
| ATOM | 453 | C | GLY | A | 59 | -15.712 | -24.404 | 17.843 | 1.00 | 36.02 | C |
| ATOM | 454 | O | GLY | A | 59 | -15.268 | -23.319 | 18.214 | 1.00 | 34.77 | O |
| ATOM | 455 | N | TYR | A | 60 | -16.561 | -24.528 | 16.826 | 1.00 | 38.85 | N |
| ATOM | 456 | CA | TYR | A | 60 | -17.004 | -23.374 | 16.045 | 1.00 | 36.79 | C |
| ATOM | 457 | C | TYR | A | 60 | -18.467 | -23.041 | 16.310 | 1.00 | 43.76 | C |
| ATOM | 458 | O | TYR | A | 60 | -19.269 | -23.933 | 16.581 | 1.00 | 40.31 | O |
| ATOM | 459 | CB | TYR | A | 60 | -16.818 | -23.630 | 14.546 | 1.00 | 40.99 | C |
| ATOM | 460 | CG | TYR | A | 60 | -15.446 | -24.123 | 14.157 | 1.00 | 40.11 | C |
| ATOM | 461 | CD1 | TYR | A | 60 | -14.305 | -23.583 | 14.729 | 1.00 | 37.87 | C |
| ATOM | 462 | CD2 | TYR | A | 60 | -15.292 | -25.132 | 13.213 | 1.00 | 40.09 | C |
| ATOM | 463 | CE1 | TYR | A | 60 | -13.050 | -24.031 | 14.374 | 1.00 | 39.91 | C |
| ATOM | 464 | CE2 | TYR | A | 60 | -14.037 | -25.587 | 12.851 | 1.00 | 40.87 | C |
| ATOM | 465 | CZ | TYR | A | 60 | -12.920 | -25.030 | 13.436 | 1.00 | 42.40 | C |
| ATOM | 466 | OH | TYR | A | 60 | -11.666 | -25.467 | 13.091 | 1.00 | 41.60 | O |
| ATOM | 467 | N | ALA | A | 61 | -18.816 | -21.760 | 16.223 | 1.00 | 32.46 | N |
| ATOM | 468 | CA | ALA | A | 61 | -20.219 | -21.370 | 16.223 | 1.00 | 39.25 | C |
| ATOM | 469 | C | ALA | A | 61 | -20.873 | -21.880 | 14.940 | 1.00 | 40.91 | C |
| ATOM | 470 | O | ALA | A | 61 | -20.228 | -21.916 | 13.891 | 1.00 | 43.24 | O |
| ATOM | 471 | CB | ALA | A | 61 | -20.363 | -19.856 | 16.343 | 1.00 | 34.62 | C |
| ATOM | 472 | N | GLN | A | 62 | -22.140 | -22.280 | 15.030 | 1.00 | 45.76 | N |
| ATOM | 473 | CA | GLN | A | 62 | -22.878 | -22.825 | 13.887 | 1.00 | 53.88 | C |
| ATOM | 474 | C | GLN | A | 62 | -22.795 | -21.937 | 12.646 | 1.00 | 47.20 | C |
| ATOM | 475 | O | GLN | A | 62 | -22.716 | -22.431 | 11.522 | 1.00 | 48.71 | O |
| ATOM | 476 | CB | GLN | A | 62 | -24.349 | -23.042 | 14.258 | 1.00 | 52.36 | C |

(continued)

| | | | | | | | | | | | |
|---|---|---|---|---|---|---|---|---|---|---|---|
| ATOM | 477 | CG | GLN | A | 62 | -24.596 | -24.209 | 15.206 | 1.00 | 79.57 | C |
| ATOM | 478 | CD | GLN | A | 62 | -24.422 | -25.562 | 14.534 | 1.00 | 86.89 | C |
| ATOM | 479 | NE2 | GLN | A | 62 | -24.222 | -26.603 | 15.339 | 1.00 | 75.39 | N |
| ATOM | 480 | OE1 | GLN | A | 62 | -24.468 | -25.670 | 13.306 | 1.00 | 81.01 | O |
| ATOM | 481 | N | LYS | A | 63 | -22.804 | -20.626 | 12.865 | 1.00 | 48.15 | N |
| ATOM | 482 | CA | LYS | A | 63 | -22.788 | -19.644 | 11.787 | 1.00 | 42.61 | C |
| ATOM | 483 | C | LYS | A | 63 | -21.524 | -19.723 | 10.919 | 1.00 | 43.90 | C |
| ATOM | 484 | O | LYS | A | 63 | -21.540 | -19.325 | 9.756 | 1.00 | 41.77 | O |
| ATOM | 485 | CB | LYS | A | 63 | -22.936 | -18.235 | 12.377 | 1.00 | 45.82 | C |
| ATOM | 486 | CG | LYS | A | 63 | -23.079 | -17.120 | 11.353 | 1.00 | 50.01 | C |
| ATOM | 487 | CD | LYS | A | 63 | -23.154 | -15.753 | 12.024 | 1.00 | 52.82 | C |
| ATOM | 488 | CE | LYS | A | 63 | -23.046 | -14.631 | 10.996 | 1.00 | 50.15 | C |
| ATOM | 489 | NZ | LYS | A | 63 | -23.127 | -13.283 | 11.624 | 1.00 | 61.30 | N |
| ATOM | 490 | N | PHE | A | 64 | -20.435 | -20.246 | 11.478 | 1.00 | 41.78 | N |
| ATOM | 491 | CA | PHE | A | 64 | -19.156 | -20.267 | 10.768 | 1.00 | 39.68 | C |
| ATOM | 492 | C | PHE | A | 64 | -18.656 | -21.675 | 10.456 | 1.00 | 44.13 | C |
| ATOM | 493 | O | PHE | A | 64 | -17.611 | -21.836 | 9.821 | 1.00 | 46.68 | O |
| ATOM | 494 | CB | PHE | A | 64 | -18.094 | -19.513 | 11.574 | 1.00 | 35.08 | C |
| ATOM | 495 | CG | PHE | A | 64 | -18.454 | -18.086 | 11.848 | 1.00 | 41.60 | C |
| ATOM | 496 | CD1 | PHE | A | 64 | -18.254 | -17.109 | 10.885 | 1.00 | 42.81 | C |
| ATOM | 497 | CD2 | PHE | A | 64 | -19.008 | -17.720 | 13.062 | 1.00 | 34.34 | C |
| ATOM | 498 | CE1 | PHE | A | 64 | -18.593 | -15.790 | 11.135 | 1.00 | 34.56 | C |
| ATOM | 499 | CE2 | PHE | A | 64 | -19.350 | -16.405 | 13.313 | 1.00 | 40.22 | C |
| ATOM | 500 | CZ | PHE | A | 64 | -19.142 | -15.441 | 12.348 | 1.00 | 32.48 | C |
| ATOM | 501 | N | GLN | A | 65 | -19.389 | -22.689 | 10.907 | 1.00 | 49.73 | N |
| ATOM | 502 | CA | GLN | A | 65 | -19.066 | -24.069 | 10.552 | 1.00 | 46.28 | C |
| ATOM | 503 | C | GLN | A | 65 | -19.116 | -24.231 | 9.040 | 1.00 | 44.61 | C |
| ATOM | 504 | O | GLN | A | 65 | -20.120 | -23.905 | 8.408 | 1.00 | 45.36 | O |
| ATOM | 505 | CB | GLN | A | 65 | -20.024 | -25.051 | 11.229 | 1.00 | 47.60 | C |
| ATOM | 506 | CG | GLN | A | 65 | -19.573 | -25.483 | 12.619 | 1.00 | 59.40 | C |
| ATOM | 507 | CD | GLN | A | 65 | -20.692 | -26.096 | 13.442 | 1.00 | 69.19 | C |
| ATOM | 508 | NE2 | GLN | A | 65 | -20.460 | -26.234 | 14.745 | 1.00 | 55.87 | N |
| ATOM | 509 | OE1 | GLN | A | 65 | -21.754 | -26.434 | 12.916 | 1.00 | 71.81 | O |
| ATOM | 510 | N | GLY | A | 66 | -18.019 | -24.711 | 8.464 | 1.00 | 39.34 | N |

(continued)

| | | | | | | | | | | | |
|---|---|---|---|---|---|---|---|---|---|---|---|
| ATOM | 511 | CA | GLY | A | 66 | -17.919 | -24.856 | 7.026 | 1.00 | 46.10 | C |
| ATOM | 512 | C | GLY | A | 66 | -17.130 | -23.748 | 6.349 | 1.00 | 56.88 | C |
| ATOM | 513 | O | GLY | A | 66 | -16.677 | -23.912 | 5.214 | 1.00 | 58.19 | O |
| ATOM | 514 | N | ARG | A | 67 | -16.966 | -22.617 | 7.031 | 1.00 | 44.44 | N |
| ATOM | 515 | CA | ARG | A | 67 | -16.192 | -21.511 | 6.470 | 1.00 | 43.73 | C |
| ATOM | 516 | C | ARG | A | 67 | -14.935 | -21.213 | 7.280 | 1.00 | 45.38 | C |
| ATOM | 517 | O | ARG | A | 67 | -13.937 | -20.745 | 6.732 | 1.00 | 45.99 | O |
| ATOM | 518 | CB | ARG | A | 67 | -17.045 | -20.244 | 6.368 | 1.00 | 47.99 | C |
| ATOM | 519 | CG | ARG | A | 67 | -18.207 | -20.359 | 5.406 | 1.00 | 47.03 | C |
| ATOM | 520 | CD | ARG | A | 67 | -18.570 | -19.011 | 4.793 | 1.00 | 47.09 | C |
| ATOM | 521 | NE | ARG | A | 67 | -19.006 | -18.032 | 5.783 | 1.00 | 50.09 | N |
| ATOM | 522 | CZ | ARG | A | 67 | -18.526 | -16.794 | 5.866 | 1.00 | 49.71 | C |
| ATOM | 523 | NH1 | ARG | A | 67 | -17.590 | -16.383 | 5.020 | 1.00 | 46.73 | N |
| ATOM | 524 | NH2 | ARG | A | 67 | -18.983 | -15.964 | 6.794 | 1.00 | 47.52 | N |
| ATOM | 525 | N | VAL | A | 68 | -14.979 | -21.480 | 8.582 | 1.00 | 39.50 | N |
| ATOM | 526 | CA | VAL | A | 68 | -13.848 | -21.157 | 9.443 | 1.00 | 35.99 | C |
| ATOM | 527 | C | VAL | A | 68 | -12.994 | -22.390 | 9.737 | 1.00 | 39.26 | C |
| ATOM | 528 | O | VAL | A | 68 | -13.491 | -23.514 | 9.795 | 1.00 | 40.40 | O |
| ATOM | 529 | CB | VAL | A | 68 | -14.314 | -20.511 | 10.777 | 1.00 | 43.31 | C |
| ATOM | 530 | CG1 | VAL | A | 68 | -14.925 | -21.554 | 11.709 | 1.00 | 44.00 | C |
| ATOM | 531 | CG2 | VAL | A | 68 | -13.155 | -19.793 | 11.461 | 1.00 | 36.66 | C |
| ATOM | 532 | N | THR | A | 69 | -11.694 | -22.167 | 9.893 | 1.00 | 34.83 | N |
| ATOM | 533 | CA | THR | A | 69 | -10.772 | -23.220 | 10.282 | 1.00 | 37.94 | C |
| ATOM | 534 | C | THR | A | 69 | -9.749 | -22.651 | 11.247 | 1.00 | 36.48 | C |
| ATOM | 535 | O | THR | A | 69 | -9.020 | -21.722 | 10.905 | 1.00 | 39.14 | O |
| ATOM | 536 | CB | THR | A | 69 | -10.049 | -23.833 | 9.069 | 1.00 | 38.15 | C |
| ATOM | 537 | CG2 | THR | A | 69 | -9.167 | -24.998 | 9.508 | 1.00 | 36.65 | C |
| ATOM | 538 | OG1 | THR | A | 69 | -11.015 | -24.297 | 8.120 | 1.00 | 40.71 | O |
| ATOM | 539 | N | LEU | A | 70 | -9.705 | -23.202 | 12.454 | 1.00 | 32.50 | N |
| ATOM | 540 | CA | LEU | A | 70 | -8.759 | -22.746 | 13.463 | 1.00 | 32.88 | C |
| ATOM | 541 | C | LEU | A | 70 | -7.647 | -23.769 | 13.633 | 1.00 | 41.47 | C |
| ATOM | 542 | O | LEU | A | 70 | -7.911 | -24.942 | 13.900 | 1.00 | 41.36 | O |
| ATOM | 543 | CB | LEU | A | 70 | -9.465 | -22.501 | 14.797 | 1.00 | 32.92 | C |
| ATOM | 544 | CG | LEU | A | 70 | -10.682 | -21.573 | 14.763 | 1.00 | 41.37 | C |

(continued)

| | | | | | | | | | | | |
|---|---|---|---|---|---|---|---|---|---|---|---|
| ATOM | 545 | CD1 | LEU | A | 70 | -11.203 | -21.301 | 16.178 | 1.00 | 36.02 | C |
| ATOM | 546 | CD2 | LEU | A | 70 | -10.360 | -20.271 | 14.047 | 1.00 | 32.82 | C |
| ATOM | 547 | N | THR | A | 71 | -6.405 | -23.324 | 13.465 | 1.00 | 33.46 | N |
| ATOM | 548 | CA | THR | A | 71 | -5.250 | -24.204 | 13.587 | 1.00 | 35.36 | C |
| ATOM | 549 | C | THR | A | 71 | -4.166 | -23.523 | 14.409 | 1.00 | 39.68 | C |
| ATOM | 550 | O | THR | A | 71 | -4.299 | -22.357 | 14.775 | 1.00 | 38.29 | O |
| ATOM | 551 | CB | THR | A | 71 | -4.684 | -24.607 | 12.203 | 1.00 | 40.98 | C |
| ATOM | 552 | CG2 | THR | A | 71 | -5.679 | -25.475 | 11.451 | 1.00 | 37.98 | C |
| ATOM | 553 | OG1 | THR | A | 71 | -4.413 | -23.432 | 11.427 | 1.00 | 44.28 | O |
| ATOM | 554 | N | ARG | A | 72 | -3.097 | -24.250 | 14.713 | 1.00 | 41.13 | N |
| ATOM | 555 | CA | ARG | A | 72 | -1.996 | -23.659 | 15.460 | 1.00 | 41.98 | C |
| ATOM | 556 | C | ARG | A | 72 | -0.644 | -24.252 | 15.077 | 1.00 | 44.70 | C |
| ATOM | 557 | O | ARG | A | 72 | -0.566 | -25.292 | 14.429 | 1.00 | 41.56 | O |
| ATOM | 558 | CB | ARG | A | 72 | -2.231 | -23.811 | 16.970 | 1.00 | 44.74 | C |
| ATOM | 559 | CG | ARG | A | 72 | -2.599 | -25.216 | 17.439 | 1.00 | 46.36 | C |
| ATOM | 560 | CD | ARG | A | 72 | -1.366 | -26.056 | 17.691 | 1.00 | 52.55 | C |
| ATOM | 561 | NE | ARG | A | 72 | -1.653 | -27.299 | 18.401 | 1.00 | 45.44 | N |
| ATOM | 562 | CZ | ARG | A | 72 | -2.105 | -28.406 | 17.824 | 1.00 | 51.24 | C |
| ATOM | 563 | NH1 | ARG | A | 72 | -2.353 | -28.429 | 16.522 | 1.00 | 53.24 | N |
| ATOM | 564 | NH2 | ARG | A | 72 | -2.319 | -29.492 | 18.553 | 1.00 | 55.87 | N |
| ATOM | 565 | N | ASP | A | 73 | 0.413 | -23.563 | 15.488 | 1.00 | 44.00 | N |
| ATOM | 566 | CA | ASP | A | 73 | 1.779 | -24.033 | 15.331 | 1.00 | 45.95 | C |
| ATOM | 567 | C | ASP | A | 73 | 2.510 | -23.807 | 16.652 | 1.00 | 45.15 | C |
| ATOM | 568 | O | ASP | A | 73 | 3.010 | -22.710 | 16.916 | 1.00 | 46.41 | O |
| ATOM | 569 | CB | ASP | A | 73 | 2.473 | -23.305 | 14.175 | 1.00 | 52.61 | C |
| ATOM | 570 | CG | ASP | A | 73 | 3.922 | -23.728 | 13.995 | 1.00 | 58.41 | C |
| ATOM | 571 | OD1 | ASP | A | 73 | 4.345 | -24.730 | 14.609 | 1.00 | 57.08 | O |
| ATOM | 572 | OD2 | ASP | A | 73 | 4.639 | -23.059 | 13.220 | 1.00 | 60.43 | O |
| ATOM | 573 | N | THR | A | 74 | 2.560 | -24.847 | 17.480 | 1.00 | 46.17 | N |
| ATOM | 574 | CA | THR | A | 74 | 3.082 | -24.726 | 18.841 | 1.00 | 48.47 | C |
| ATOM | 575 | C | THR | A | 74 | 4.559 | -24.346 | 18.884 | 1.00 | 46.89 | C |
| ATOM | 576 | O | THR | A | 74 | 5.012 | -23.723 | 19.842 | 1.00 | 53.90 | O |
| ATOM | 577 | CB | THR | A | 74 | 2.888 | -26.035 | 19.636 | 1.00 | 54.20 | C |
| ATOM | 578 | CG2 | THR | A | 74 | 1.439 | -26.185 | 20.072 | 1.00 | 48.42 | C |

(continued)

| | | | | | | | | | | | |
|---|---|---|---|---|---|---|---|---|---|---|---|
| ATOM | 579 | OG1 | THR | A | 74 | 3.250 | -27.155 | 18.817 | 1.00 | 60.06 | O |
| ATOM | 580 | N | SER | A | 75 | 5.303 | -24.710 | 17 . 845 | 1.00 | 51.70 | N |
| ATOM | 581 | CA | SER | A | 75 | 6.737 | -24.436 | 17.801 | 1.00 | 54.91 | C |
| ATOM | 582 | C | SER | A | 75 | 7.036 | -22.937 | 17 . 815 | 1.00 | 55.26 | C |
| ATOM | 583 | O | SER | A | 75 | 8.114 | -22.515 | 18.237 | 1.00 | 49.86 | O |
| ATOM | 584 | CB | SER | A | 75 | 7.365 | -25.088 | 16.568 | 1.00 | 55.81 | C |
| ATOM | 585 | OG | SER | A | 75 | 6.713 | -24.668 | 15.382 | 1.00 | 60.43 | O |
| ATOM | 586 | N | ILE | A | 76 | 6.077 | -22.136 | 17.359 | 1.00 | 52.63 | N |
| ATOM | 587 | CA | ILE | A | 76 | 6.224 | -20.684 | 17.386 | 1.00 | 47.56 | C |
| ATOM | 588 | C | ILE | A | 76 | 5.090 | -20.021 | 18.170 | 1.00 | 50.56 | C |
| ATOM | 589 | O | ILE | A | 76 | 4.920 | -18.802 | 18.109 | 1.00 | 52.59 | O |
| ATOM | 590 | CB | ILE | A | 76 | 6.271 | -20.093 | 15.962 | 1.00 | 50.46 | C |
| ATOM | 591 | CG1 | ILE | A | 76 | 5.040 | -20.521 | 15.163 | 1.00 | 47.49 | C |
| ATOM | 592 | CG2 | ILE | A | 76 | 7.536 | -20.531 | 15.248 | 1.00 | 46.69 | C |
| ATOM | 593 | CD1 | ILE | A | 76 | 4.922 | -19.840 | 13.817 | 1.00 | 48.75 | C |
| ATOM | 594 | N | SER | A | 77 | 4.324 | -20.834 | 18.896 | 1.00 | 49.81 | N |
| ATOM | 595 | CA | SER | A | 77 | 3.229 | -20.357 | 19.744 | 1.00 | 45.00 | C |
| ATOM | 596 | C | SER | A | 77 | 2.246 | -19.470 | 18.988 | 1.00 | 46.19 | C |
| ATOM | 597 | O | SER | A | 77 | 1.894 | -18.387 | 19.456 | 1.00 | 40.30 | O |
| ATOM | 598 | CB | SER | A | 77 | 3.781 | -19.591 | 20.951 | 1.00 | 43.85 | C |
| ATOM | 599 | OG | SER | A | 77 | 4.716 | -20.370 | 21.673 | 1.00 | 63.87 | O |
| ATOM | 600 | N | THR | A | 78 | 1.800 | -19.925 | 17.B22 | 1.00 | 39.04 | N |
| ATOM | 601 | CA | THR | A | 78 | 0.956 | -19.090 | 16.979 | 1.00 | 37.69 | C |
| ATOM | 602 | C | THR | A | 78 | -0.337 | -19.788 | 16.575 | 1.00 | 37.97 | C |
| ATOM | 603 | O | THR | A | 78 | -0.325 | -20.930 | 16.115 | 1.00 | 39.48 | O |
| ATOM | 604 | CB | THR | A | 78 | 1.718 | -18.641 | 15.712 | 1.00 | 38.64 | C |
| ATOM | 605 | CG2 | THR | A | 78 | 0.810 | -17.840 | 14.787 | 1.00 | 38.64 | C |
| ATOM | 606 | OG1 | THR | A | 78 | 2.828 | -17.822 | 16.094 | 1.00 | 35.01 | O |
| ATOM | 607 | N | ALA | A | 79 | -1.454 | -19.093 | 16.765 | 1.00 | 33.81 | N |
| ATOM | 608 | CA | ALA | A | 79 | -2.756 | -19.592 | 16.336 | 1.00 | 34.16 | C |
| ATOM | 609 | C | ALA | A | 79 | -3.165 | -18.934 | 15.023 | 1.00 | 34.47 | C |
| ATOM | 610 | O | ALA | A | 79 | -2.748 | -17.815 | 14.720 | 1.00 | 33.95 | O |
| ATOM | 611 | CB | ALA | A | 79 | -3.805 | -19.340 | 17.403 | 1.00 | 24.34 | C |
| ATOM | 612 | N | TYR | A | 80 | -3.990 | -19.631 | 14.252 | 1.00 | 31.73 | N |

(continued)

| | | | | | | | | | | | |
|---|---|---|---|---|---|---|---|---|---|---|---|
| ATOM | 613 | CA | TYR | A | 80 | -4.432 | -19.135 | 12.958 | 1.00 | 37.57 | C |
| ATOM | 614 | C | TYR | A | 80 | -5.942 | -19.198 | 12.810 | 1.00 | 39.18 | C |
| ATOM | 615 | O | TYR | A | 80 | -6.598 | -20.132 | 13.278 | 1.00 | 37.02 | O |
| ATOM | 616 | CB | TYR | A | 80 | -3.786 | -19.929 | 11.819 | 1.00 | 36.79 | C |
| ATOM | 617 | CG | TYR | A | 80 | -2.281 | -19.862 | 11.797 | 1.00 | 40.36 | C |
| ATOM | 618 | CD1 | TYR | A | 80 | -1.621 | -18.821 | 11.158 | 1.00 | 37.20 | C |
| ATOM | 619 | CD2 | TYR | A | 80 | -1.517 | -20.847 | 12.413 | 1.00 | 38.74 | C |
| ATOM | 620 | CE1 | TYR | A | 80 | -0.238 | -18.761 | 11.135 | 1.00 | 37.53 | C |
| ATOM | 621 | CE2 | TYR | A | 80 | -0.140 | -20.797 | 12.395 | 1.00 | 37.97 | C |
| ATOM | 622 | CZ | TYR | A | 80 | 0.497 | -19.755 | 11.756 | 1.00 | 43.48 | C |
| ATOM | 623 | OH | TYR | A | 80 | 1.872 | -19.708 | 11.742 | 1.00 | 41.36 | O |
| ATOM | 624 | N | MET | A | 81 | -6.486 | -18.195 | 12.141 | 1.00 | 31.97 | N |
| ATOM | 625 | CA | MET | A | 81 | -7.895 | -18.185 | 11.803 | 1.00 | 37.08 | C |
| ATOM | 626 | C | MET | A | 81 | -7.999 | -18.062 | 10.297 | 1.00 | 34.14 | C |
| ATOM | 627 | O | MET | A | 81 | -7.505 | -17.096 | 9.717 | 1.00 | 38.01 | O |
| ATOM | 628 | CB | MET | A | 81 | -8.619 | -17.036 | 12.509 | 1.00 | 39.39 | C |
| ATOM | 629 | CG | MET | A | 81 | -10.137 | -17.099 | 12.445 | 1.00 | 43.17 | C |
| ATOM | 630 | SD | MET | A | 81 | -10.835 | -16.468 | 10.907 | 1.00 | 56.54 | S |
| ATOM | 631 | CE | MET | A | 81 | -10.224 | -14.782 | 10.930 | 1.00 | 40.20 | C |
| ATOM | 632 | N | GLU | A | 82 | -8.606 | -19.054 | 9.659 | 1.00 | 36.27 | N |
| ATOM | 633 | CA | GLU | A | 82 | -8.825 | -18.989 | 8.222 | 1.00 | 37.25 | C |
| ATOM | 634 | C | GLU | A | 82 | -10.310 | -18.963 | 7 .908 | 1.00 | 38.89 | C |
| ATOM | 635 | O | GLU | A | 82 | -11.064 | -19.831 | 8.343 | 1.00 | 42.10 | O |
| ATOM | 636 | CB | GLU | A | 82 | -8.159 | -20.161 | 7.504 | 1.00 | 34.97 | C |
| ATOM | 637 | CG | GLU | A | 82 | -8.406 | -20.149 | 6.003 | 1.00 | 48.29 | C |
| ATOM | 638 | CD | GLU | A | 82 | -7.374 | -20.946 | 5.228 | 1.00 | 61.14 | C |
| ATOM | 639 | OE1 | GLU | A | 82 | -6.617 | -21.717 | 5.858 | 1.00 | 70.53 | O |
| ATOM | 640 | OE2 | GLU | A | 82 | -7.313 | -20.793 | 3.989 | 1.00 | 62.98 | O |
| ATOM | 641 | N | LEU | A | 83 | -10.726 | -17.946 | 7.161 | 1.00 | 41.14 | N |
| ATOM | 642 | CA | LEU | A | 83 | -12.118 | -17.807 | 6.766 | 1.00 | 40.75 | C |
| ATOM | 643 | C | LEU | A | 83 | -12.204 | -17.828 | 5.246 | 1.00 | 42.77 | C |
| ATOM | 644 | O | LEU | A | 83 | -11.572 | -17.017 | 4.569 | 1.00 | 46.85 | O |
| ATOM | 645 | CB | LEU | A | 83 | -12.716 | -16.519 | 7.336 | 1.00 | 38.47 | C |
| ATOM | 646 | CG | LEU | A | 83 | -14.233 | -16.359 | 7.216 | 1.00 | 41.96 | C |

(continued)

| | | | | | | | | | | | |
|---|---|---|---|---|---|---|---|---|---|---|---|
| ATOM | 647 | CD1 | LEU | A | 83 | -14.956 | -17.499 | 7.919 | 1.00 | 40.57 | C |
| ATOM | 648 | CD2 | LEU | A | 83 | -14.677 | -15.015 | 7.776 | 1.00 | 38.68 | C |
| ATOM | 649 | N | SER | A | 84 | -12.974 | -18.769 | 4.711 | 1.00 | 41.64 | N |
| ATOM | 650 | CA | SER | A | 84 | -13.079 | -18.936 | 3.267 | 1.00 | 42.87 | C |
| ATOM | 651 | C | SER | A | 84 | -14.426 | -18.442 | 2.740 | 1.00 | 44.92 | C |
| ATOM | 652 | O | SER | A | 84 | -15.304 | -18.067 | 3.522 | 1.00 | 44.37 | O |
| ATOM | 653 | CB | SER | A | 84 | -12.868 | -20.402 | 2.888 | 1.00 | 39.46 | C |
| ATOM | 654 | OG | SER | A | 84 | -13.903 | -21.211 | 3.416 | 1.00 | 44.27 | O |
| ATOM | 655 | N | SER | A | 85 | -14.572 | -18.454 | 1.414 | 1.00 | 47.43 | N |
| ATOM | 656 | CA | SER | A | 85 | -15.769 | -17.960 | 0.732 | 1.00 | 41.86 | C |
| ATOM | 657 | C | SER | A | 85 | -16.189 | -16.591 | 1.245 | 1.00 | 43.45 | C |
| ATOM | 658 | O | SER | A | 85 | -17.337 | -16.392 | 1.652 | 1.00 | 44.70 | O |
| ATOM | 659 | CB | SER | A | 85 | -16.922 | -18.952 | 0.882 | 1.00 | 45.07 | C |
| ATOM | 660 | OG | SER | A | 85 | -16.674 | -20.125 | 0.128 | 1.00 | 51.48 | O |
| ATOM | 661 | N | LEU | A | 86 | -15.249 | -15.650 | 1.223 | 1.00 | 43.44 | N |
| ATOM | 662 | CA | LEU | A | 86 | -15.457 | -14.338 | 1.826 | 1.00 | 41.57 | C |
| ATOM | 663 | C | LEU | A | 86 | -16.526 | -13.526 | 1.104 | 1.00 | 46.59 | C |
| ATOM | 664 | O | LEU | A | 86 | -16.551 | -13.456 | -0.128 | 1.00 | 40.39 | O |
| ATOM | 665 | CB | LEU | A | 86 | -14.142 | -13.557 | 1.858 | 1.00 | 40.33 | C |
| ATOM | 666 | CG | LEU | A | 86 | -13.076 | -14.119 | 2.800 | 1.00 | 41.08 | C |
| ATOM | 667 | CD1 | LEU | A | 86 | -11.732 | -13.444 | 2.573 | 1.00 | 39.92 | C |
| ATOM | 668 | CD2 | LEU | A | 86 | -13.527 | -13.966 | 4.241 | 1.00 | 33.71 | C |
| ATOM | 669 | N | ARG | A | 87 | -17.420 | -12.933 | 1.888 | 1.00 | 37.30 | N |
| ATOM | 670 | CA | ARG | A | 87 | -18.411 | -12.002 | 1.371 | 1.00 | 42.71 | C |
| ATOM | 671 | C | ARG | A | 87 | -18.086 | -10.616 | 1.905 | 1.00 | 44.74 | C |
| ATOM | 672 | O | ARG | A | 87 | -17.304 | -10.483 | 2.846 | 1.00 | 42.11 | O |
| ATOM | 673 | CB | ARG | A | 87 | -19.828 | -12.418 | 1.774 | 1.00 | 47.73 | C |
| ATOM | 674 | CG | ARG | A | 87 | -20.231 | -13.806 | 1.298 | 1.00 | 57.87 | C |
| ATOM | 675 | CD | ARG | A | 87 | -21.702 | -14.088 | 1.577 | 1.00 | 78.50 | C |
| ATOM | 676 | NE | ARG | A | 87 | -22.154 | -15.318 | 0.928 | 1.00 | 98.72 | N |
| ATOM | 677 | CZ | ARG | A | 87 | -23.391 | -15.800 | 1.009 | 1.00 | 102.44 | C |
| ATOM | 678 | NH1 | ARG | A | 87 | -24.311 | -15.158 | 1.716 | 1.00 | 100.96 | N |
| ATOM | 679 | NH2 | ARG | A | 87 | -23.708 | -16.927 | 0.384 | 1.00 | 94.73 | N |
| ATOM | 680 | N | SER | A | 88 | -18.682 | -9.586 | 1.315 | 1.00 | 48.46 | N |

(continued)

| | | | | | | | | | | | |
|---|---|---|---|---|---|---|---|---|---|---|---|
| ATOM | 681 | CA | SER | A | 88 | -18.426 | -8.221 | 1.759 | 1.00 | 47.83 | C |
| ATOM | 682 | C | SER | A | 88 | -18.871 | -8.035 | 3.212 | 1.00 | 43.27 | C |
| ATOM | 683 | O | SER | A | 88 | -18.315 | -7.210 | 3.935 | 1.00 | 45.67 | O |
| ATOM | 684 | CB | SER | A | 88 | -19.126 | -7.209 | 0.843 | 1.00 | 46.62 | C |
| ATOM | 685 | OG | SER | A | 88 | -20.530 | -7.399 | 0.826 | 1.00 | 56.66 | O |
| ATOM | 686 | N | GLU | A | 89 | -19.857 | -8.823 | 3.638 | 1.00 | 43.65 | N |
| ATOM | 687 | CA | GLU | A | 89 | -20.347 | -8.780 | 5.012 | 1.00 | 42.47 | C |
| ATOM | 688 | C | GLU | A | 89 | -19.332 | -9.323 | 6.015 | 1.00 | 42.20 | C |
| ATOM | 689 | O | GLU | A | 89 | -19.508 | -9.163 | 7.224 | 1.00 | 44.31 | O |
| ATOM | 690 | CB | GLU | A | 89 | -21.658 | -9.562 | 5.146 | 1.00 | 48.07 | C |
| ATOM | 691 | CG | GLU | A | 89 | -22.881 | -8.844 | 4.594 | 1.00 | 69.24 | C |
| ATOM | 692 | CD | GLU | A | 89 | -22.970 | -8.904 | 3.079 | 1.00 | 77.53 | C |
| ATOM | 693 | OE1 | GLU | A | 89 | -22.301 | -9.773 | 2.476 | 1.00 | 65.90 | O |
| ATOM | 694 | OE2 | GLU | A | 89 | -23.710 | -8.081 | 2.493 | 1.00 | 76.65 | O |
| ATOM | 695 | N | ASP | A | 90 | -18.279 | -9.969 | 5.519 | 1.00 | 36.36 | N |
| ATOM | 696 | CA | ASP | A | 90 | -17.213 | -10.463 | 6.388 | 1.00 | 34.38 | C |
| ATOM | 697 | C | ASP | A | 90 | -16.197 | -9.364 | 6.709 | 1.00 | 39.43 | C |
| ATOM | 698 | O | ASP | A | 90 | -15.250 | -9.589 | 7.463 | 1.00 | 38.18 | O |
| ATOM | 699 | CB | ASP | A | 90 | -16.494 | -11.658 | 5.751 | 1.00 | 38.88 | C |
| ATOM | 700 | CG | ASP | A | 90 | -17.361 | -12.903 | 5.681 | 1.00 | 42.31 | C |
| ATOM | 701 | OD1 | ASP | A | 90 | -18.157 | -13.146 | 6.616 | 1.00 | 37.56 | O |
| ATOM | 702 | OD2 | ASP | A | 90 | -17.237 | -13.646 | 4.683 | 1.00 | 47.30 | O |
| ATOM | 703 | N | THR | A | 91 | -16.389 | -8.183 | 6.127 | 1.00 | 34.63 | N |
| ATOM | 704 | CA | THR | A | 91 | -15.525 | -7.042 | 6.418 | 1.00 | 35.67 | C |
| ATOM | 705 | C | THR | A | 91 | -15.661 | -6.663 | 7.887 | 1.00 | 34.77 | C |
| ATOM | 706 | O | THR | A | 91 | -16.743 | -6.285 | 8.340 | 1.00 | 34.99 | O |
| ATOM | 707 | CB | THR | A | 91 | -15.858 | -5.818 | 5.532 | 1.00 | 37.50 | C |
| ATOM | 708 | CG2 | THR | A | 91 | -15.075 | -4.591 | 5.994 | 1.00 | 34.40 | C |
| ATOM | 709 | OG1 | THR | A | 91 | -15.529 | -6.104 | 4.164 | 1.00 | 40.03 | O |
| ATOM | 710 | N | ALA | A | 92 | -14.562 | -6.772 | 8.626 | 1.00 | 29.62 | N |
| ATOM | 711 | CA | ALA | A | 92 | -14.588 | -6.570 | 10.070 | 1.00 | 32.67 | C |
| ATOM | 712 | C | ALA | A | 92 | -13.190 | -6.544 | 10.665 | 1.00 | 27.73 | C |
| ATOM | 713 | O | ALA | A | 92 | -12.209 | -6.882 | 10.001 | 1.00 | 29.29 | O |
| ATOM | 714 | CB | ALA | A | 92 | -15.416 | -7.671 | 10.746 | 1.00 | 33.43 | C |

(continued)

| | | | | | | | | | | | |
|---|---|---|---|---|---|---|---|---|---|---|---|
| ATOM | 715 | N | VAL | A | 93 | -13.112 | -6.143 | 11.929 | 1.00 | 30.17 | N |
| ATOM | 716 | CA | VAL | A | 93 | -11.897 | -6.308 | 12.704 | 1.00 | 26.67 | C |
| ATOM | 717 | C | VAL | A | 93 | -11.959 | -7.652 | 13.424 | 1.00 | 29.85 | C |
| ATOM | 718 | O | VAL | A | 93 | -12.911 | -7.934 | 14.154 | 1.00 | 32.54 | O |
| ATOM | 719 | CB | VAL | A | 93 | -11.705 | -5.169 | 13.726 | 1.00 | 31. 84 | C |
| ATOM | 720 | CG1 | VAL | A | 93 | -10.539 | -5.475 | 14.653 | 1.00 | 24.37 | C |
| ATOM | 721 | CG2 | VAL | A | 93 | -11.487 | -3.842 | 13.009 | 1.00 | 25.26 | C |
| ATOM | 722 | N | TYR | A | 94 | -10.948 | -8.484 | 13.207 | 1.00 | 26.52 | N |
| ATOM | 723 | CA | TYR | A | 94 | -10.911 | -9.806 | 13.816 | 1.00 | 31.35 | C |
| ATOM | 724 | C | TYR | A | 94 | -9.961 | -9.826 | 15.005 | 1.00 | 33.59 | C |
| ATOM | 725 | O | TYR | A | 94 | -8.769 | -9.546 | 14.867 | 1.00 | 31.79 | O |
| ATOM | 726 | CB | TYR | A | 94 | -10.518 | -10.861 | 12.774 | 1.00 | 25.00 | C |
| ATOM | 727 | CG | TYR | A | 94 | -11.629 | -11.105 | 11.781 | 1.00 | 27.22 | C |
| ATOM | 728 | CD1 | TYR | A | 94 | -11.863 | -10.216 | 10.739 | 1.00 | 30.50 | C |
| ATOM | 729 | CD2 | TYR | A | 94 | -12.469 | -12.207 | 11.906 | 1.00 | 23.97 | C |
| ATOM | 730 | CE1 | TYR | A | 94 | -12.893 | -10.423 | 9.842 | 1.00 | 27.95 | C |
| ATOM | 731 | CE2 | TYR | A | 94 | -13.492 | -12.423 | 11.022 | 1.00 | 26.58 | C |
| ATOM | 732 | CZ | TYR | A | 94 | -13.703 | -11.527 | 9.989 | 1.00 | 32.87 | C |
| ATOM | 733 | OH | TYR | A | 94 | -14.730 | -11.739 | 9.104 | 1.00 | 30.47 | O |
| ATOM | 734 | N | TYR | A | 95 | -10.511 | -10.135 | 16.177 | 1.00 | 24.85 | N |
| ATOM | 735 | CA | TYR | A | 95 | -9.731 | -10.215 | 17.406 | 1.00 | 28.45 | C |
| ATOM | 736 | C | TYR | A | 95 | -9.474 | -11.661 | 17 .805 | 1.00 | 27.62 | C |
| ATOM | 737 | O | TYR | A | 95 | -10.356 | -12.508 | 17.688 | 1.00 | 28.27 | O |
| ATOM | 738 | CB | TYR | A | 95 | -10.450 | -9.512 | 18.564 | 1.00 | 24.54 | C |
| ATOM | 739 | CG | TYR | A | 95 | -10.626 | -8.019 | 18.419 | 1.00 | 26.81 | C |
| ATOM | 740 | CD1 | TYR | A | 95 | -9.580 | -7.148 | 18.692 | 1.00 | 27.26 | C |
| ATOM | 741 | CD2 | TYR | A | 95 | -11.847 | -7.481 | 18.036 | 1.00 | 28.58 | C |
| ATOM | 742 | CE1 | TYR | A | 95 | -9.737 | -5.778 | 18.571 | 1.00 | 27.06 | C |
| ATOM | 743 | CE2 | TYR | A | 95 | -12.017 | -6.113 | 17.913 | 1.00 | 27.13 | C |
| ATOM | 744 | CZ | TYR | A | 95 | -10.960 | -5.269 | 18.184 | 1.00 | 27.27 | C |
| ATOM | 745 | OH | TYR | A | 95 | -11.125 | -3.911 | 18.060 | 1.00 | 31.79 | O |
| ATOM | 746 | N | CYS | A | 96 | -8.274 | -11.945 | 18.289 | 1.00 | 23.92 | N |
| ATOM | 747 | CA | CYS | A | 96 | -8.073 | -13.182 | 19.025 | 1.00 | 30.30 | C |
| ATOM | 748 | C | CYS | A | 96 | -8.115 | -12.829 | 20.505 | 1.00 | 27.04 | C |

(continued)

| | | | | | | | | | | | |
|---|---|---|---|---|---|---|---|---|---|---|---|
| ATOM | 749 | O | CYS | A | 96 | -7.708 | -11.736 | 20.899 | 1.00 | 28.05 | O |
| ATOM | 750 | CB | CYS | A | 96 | -6.758 | -13.877 | 18.645 | 1.00 | 33.84 | C |
| ATOM | 751 | SG | CYS | A | 96 | -5.246 | -13.010 | 19.099 | 1.00 | 48.82 | S |
| ATOM | 752 | N | ALA | A | 97 | -8.634 | -13.742 | 21.316 | 1.00 | 28.58 | N |
| ATOM | 753 | CA | ALA | A | 97 | -8.783 | -13.497 | 22.743 | 1.00 | 26.57 | C |
| ATOM | 754 | C | ALA | A | 97 | -8.768 | -14.816 | 23.507 | 1.00 | 32.11 | C |
| ATOM | 755 | O | ALA | A | 97 | -9.409 | -15.780 | 23.091 | 1.00 | 30.87 | O |
| ATOM | 756 | CB | ALA | A | 97 | -10.065 | -12.736 | 23.016 | 1.00 | 26.61 | C |
| ATOM | 757 | N | SER | A | 98 | -8.042 | -14.864 | 24.621 | 1.00 | 27.25 | N |
| ATOM | 758 | CA | SER | A | 98 | -7.888 | -16.121 | 25.348 | 1.00 | 32.20 | C |
| ATOM | 759 | C | SER | A | 98 | -8.839 | -16.275 | 26.532 | 1.00 | 29.23 | C |
| ATOM | 760 | O | SER | A | 98 | -9.249 | -15.294 | 27.153 | 1.00 | 25.23 | O |
| ATOM | 761 | CB | SER | A | 98 | -6.453 | -16.280 | 25.845 | 1.00 | 33.45 | C |
| ATOM | 762 | OG | SER | A | 98 | -6.155 | -15.337 | 26.856 | 1.00 | 45.41 | O |
| ATOM | 763 | N | SER | A | 99 | -9.169 | -17.528 | 26.833 | 1.00 | 29.87 | N |
| ATOM | 764 | CA | SER | A | 99 | -9.971 | -17.885 | 28.001 | 1.00 | 30.05 | C |
| ATOM | 765 | C | SER | A | 99 | -9.348 | -19.099 | 28.689 | 1.00 | 35.19 | C |
| ATOM | 766 | O | SER | A | 99 | -8.528 | -19.794 | 28.086 | 1.00 | 30.60 | O |
| ATOM | 767 | CB | SER | A | 99 | -11.417 | -18.171 | 27.596 | 1.00 | 26.53 | C |
| ATOM | 768 | OG | SER | A | 99 | -11.485 | -19.129 | 26.552 | 1.00 | 29.62 | O |
| ATOM | 769 | N | SER | A | 100 | -9.738 | -19.355 | 29.938 | 1.00 | 33.90 | N |
| ATOM | 770 | CA | SER | A | 100 | -9.099 | -20.396 | 30.749 | 1.00 | 39.84 | C |
| ATOM | 771 | C | SER | A | 100 | -10.060 | -21.427 | 31.338 | 1.00 | 33.23 | C |
| ATOM | 772 | O | SER | A | 100 | -9.932 | -21.793 | 32.506 | 1.00 | 40.18 | O |
| ATOM | 773 | CB | SER | A | 100 | -8.319 | -19.758 | 31.900 | 1.00 | 32.53 | C |
| ATOM | 774 | OG | SER | A | 100 | -7.194 | -19.048 | 31.424 | 1.00 | 49.78 | O |
| ATOM | 775 | O | GLY | A | 101 | -14.315 | -23.137 | 30.676 | 1.00 | 40.43 | O |
| ATOM | 776 | N | GLY | A | 101 | -11.017 | -21.899 | 30.547 | 1.00 | 31.28 | N |
| ATOM | 777 | CA | GLY | A | 101 | -11.974 | -22.868 | 31.048 | 1.00 | 28.36 | C |
| ATOM | 778 | C | GLY | A | 101 | -13.406 | -22.374 | 30.999 | 1.00 | 32.57 | C |
| ATOM | 779 | O | TRP | A | 102 | -13.530 | -18.918 | 29.800 | 1.00 | 32.97 | O |
| ATOM | 780 | N | TRP | A | 102 | -13.617 | -21.110 | 31.357 | 1.00 | 23.41 | N |
| ATOM | 781 | CA | TRP | A | 102 | -14 . 879 | -20.435 | 31.059 | 1.00 | 26.91 | C |
| ATOM | 782 | C | TRP | A | 102 | -14.626 | -19.471 | 29.916 | 1.00 | 30.14 | C |

(continued)

| | | | | | | | | | | | |
|---|---|---|---|---|---|---|---|---|---|---|---|
| ATOM | 783 | CB | TRP | A | 102 | -15.432 | -19.683 | 32.271 | 1.00 | 27.64 | C |
| ATOM | 784 | CG | TRP | A | 102 | -15.721 | -20.553 | 33.447 | 1.00 | 28.49 | C |
| ATOM | 785 | CD1 | TRP | A | 102 | -16.761 | -21.428 | 33.587 | 1.00 | 25.22 | C |
| ATOM | 786 | CD2 | TRP | A | 102 | -14.972 | -20.618 | 34.664 | 1.00 | 25.37 | C |
| ATOM | 787 | NE1 | TRP | A | 102 | -16.701 | -22.038 | 34.818 | 1.00 | 26.75 | N |
| ATOM | 788 | CE2 | TRP | A | 102 | -15.611 | -21.559 | 35.498 | 1.00 | 23.51 | C |
| ATOM | 789 | CE3 | TRP | A | 102 | -13.819 | -19.977 | 35.128 | 1.00 | 23.78 | C |
| ATOM | 790 | CZ2 | TRP | A | 102 | -15.134 | -21.874 | 36.772 | 1.00 | 27.12 | C |
| ATOM | 791 | CZ3 | TRP | A | 102 | -13.346 | -20.291 | 36.396 | 1.00 | 31. 84 | C |
| ATOM | 792 | CH2 | TRP | A | 102 | -14.003 | -21.232 | 37.202 | 1.00 | 24.60 | C |
| ATOM | 793 | O | TYR | A | 103 | -16.560 | -16.374 | 27.434 | 1.00 | 35.87 | O |
| ATOM | 794 | N | TYR | A | 103 | -15.628 | -19.268 | 29.071 | 1.00 | 23.62 | N |
| ATOM | 795 | CA | TYR | A | 103 | -15.441 | -18.444 | 27.884 | 1.00 | 33.88 | C |
| ATOM | 796 | C | TYR | A | 103 | -15.723 | -16.960 | 28.117 | 1.00 | 34.03 | C |
| ATOM | 797 | CB | TYR | A | 103 | -16.321 | -18.949 | 26.742 | 1.00 | 27.85 | C |
| ATOM | 798 | CG | TYR | A | 103 | -15.988 | -20.342 | 26.265 | 1.00 | 32.69 | C |
| ATOM | 799 | CD1 | TYR | A | 103 | -14.804 | -20.601 | 25.588 | 1.00 | 31.32 | C |
| ATOM | 800 | CD2 | TYR | A | 103 | -16.866 | -21.396 | 26.478 | 1.00 | 31.92 | C |
| ATOM | 801 | CE1 | TYR | A | 103 | -14.498 | -21.879 | 25.143 | 1.00 | 32.07 | C |
| ATOM | 802 | CE2 | TYR | A | 103 | -16.571 | -22.673 | 26.036 | 1.00 | 31.82 | C |
| ATOM | 803 | CZ | TYR | A | 103 | -15.385 | -22.908 | 25.370 | 1.00 | 34.61 | C |
| ATOM | 804 | OH | TYR | A | 103 | -15.090 | -24.176 | 24.931 | 1.00 | 37.00 | O |
| ATOM | 805 | N | TYR | A | 104 | -15.035 | -16.359 | 29.085 | 1.00 | 30.47 | N |
| ATOM | 806 | CA | TYR | A | 104 | -14.929 | -14.904 | 29.133 | 1.00 | 34.52 | C |
| ATOM | 807 | C | TYR | A | 104 | -13.452 | -14.557 | 28.940 | 1.00 | 37.81 | C |
| ATOM | 808 | O | TYR | A | 104 | -12.571 | -15.311 | 29.359 | 1.00 | 33.63 | O |
| ATOM | 809 | CB | TYR | A | 104 | -15.488 | -14.328 | 30.439 | 1.00 | 28.01 | C |
| ATOM | 810 | CG | TYR | A | 104 | -14.762 | -14.755 | 31.690 | 1.00 | 29.48 | C |
| ATOM | 811 | CD2 | TYR | A | 104 | -15.209 | -15.837 | 32.442 | 1.00 | 31.05 | C |
| ATOM | 812 | CD1 | TYR | A | 104 | -13.639 | -14.068 | 32.132 | 1.00 | 33.84 | C |
| ATOM | 813 | CE2 | TYR | A | 104 | -14.547 | -16.227 | 33.600 | 1.00 | 31.77 | C |
| ATOM | 814 | CE1 | TYR | A | 104 | -12.968 | -14.452 | 33.282 | 1.00 | 38.93 | C |
| ATOM | 815 | CZ | TYR | A | 104 | -13.424 | -15.530 | 34.009 | 1.00 | 35.52 | C |
| ATOM | 816 | OH | TYR | A | 104 | -12.751 | -15.905 | 35.147 | 1.00 | 47. 88 | O |

(continued)

| | | | | | | | | | | | |
|---|---|---|---|---|---|---|---|---|---|---|---|
| ATOM | 817 | N | PHE | A | 105 | -13.180 | -13.423 | 28.308 | 1.00 | 34.39 | N |
| ATOM | 818 | CA | PHE | A | 105 | -11.862 | -13.201 | 27.718 | 1.00 | 31.85 | C |
| ATOM | 819 | C | PHE | A | 105 | -11.046 | -12.124 | 28.419 | 1.00 | 31.30 | C |
| ATOM | 820 | O | PHE | A | 105 | -11.367 | -10.938 | 28.347 | 1.00 | 33.02 | O |
| ATOM | 821 | CB | PHE | A | 105 | -12.046 | -12.872 | 26.240 | 1.00 | 27.43 | C |
| ATOM | 822 | CG | PHE | A | 105 | -13.093 | -13.719 | 25.583 | 1.00 | 32.14 | C |
| ATOM | 823 | CD1 | PHE | A | 105 | -12.894 | -15.083 | 25.423 | 1.00 | 32.34 | C |
| ATOM | 824 | CD2 | PHE | A | 105 | -14.292 | -13.168 | 25.166 | 1.00 | 33.77 | C |
| ATOM | 825 | CE1 | PHE | A | 105 | -13.866 | -15.879 | 24.840 | 1.00 | 28.51 | C |
| ATOM | 826 | CE2 | PHE | A | 105 | -15.267 | -13.959 | 24.577 | 1.00 | 36.93 | C |
| ATOM | 827 | CZ | PHE | A | 105 | -15.052 | -15.317 | 24.413 | 1.00 | 31.65 | C |
| ATOM | 828 | N | ASP | A | 106 | -9.981 | -12.543 | 29.097 | 1.00 | 31.31 | N |
| ATOM | 829 | CA | ASP | A | 106 | -9.205 | -11.614 | 29.914 | 1.00 | 33.51 | C |
| ATOM | 830 | C | ASP | A | 106 | -8.015 | -11.014 | 29.167 | 1.00 | 32.69 | C |
| ATOM | 831 | O | ASP | A | 106 | -7.485 | -9.983 | 29.578 | 1.00 | 38.29 | O |
| ATOM | 832 | CB | ASP | A | 106 | -8.734 | -12.298 | 31.206 | 1.00 | 37.27 | C |
| ATOM | 833 | CG | ASP | A | 106 | -8.078 | -13.645 | 30.962 | 1.00 | 44.11 | C |
| ATOM | 834 | OD1 | ASP | A | 106 | -8.298 | -14.243 | 29.886 | 1.00 | 44.95 | O |
| ATOM | 835 | OD2 | ASP | A | 106 | -7.347 | -14.114 | 31.860 | 1.00 | 39.24 | O |
| ATOM | 836 | N | TYR | A | 107 | -7.604 | -11.645 | 28.069 | 1.00 | 33.81 | N |
| ATOM | 837 | CA | TYR | A | 107 | -6.550 | -11.085 | 27.220 | 1.00 | 31.46 | C |
| ATOM | 838 | C | TYR | A | 107 | -6.967 | -11.061 | 25.752 | 1.00 | 27.21 | C |
| ATOM | 839 | O | TYR | A | 107 | -7.565 | -12.015 | 25.256 | 1.00 | 27.70 | O |
| ATOM | 840 | CB | TYR | A | 107 | -5.250 | -11.870 | 27.388 | 1.00 | 34.62 | C |
| ATOM | 841 | CG | TYR | A | 107 | -4.719 | -11.830 | 28.802 | 1.00 | 39.20 | C |
| ATOM | 842 | CD1 | TYR | A | 107 | -4.065 | -10.705 | 29.284 | 1.00 | 44.79 | C |
| ATOM | 843 | CD2 | TYR | A | 107 | -4.882 | -12.912 | 29.659 | 1.00 | 42.77 | C |
| ATOM | 844 | CE1 | TYR | A | 107 | -3.584 | -10.658 | 30.583 | 1.00 | 57.22 | C |
| ATOM | 845 | CE2 | TYR | A | 107 | -4.403 | -12.875 | 30.957 | 1.00 | 50.96 | C |
| ATOM | 846 | CZ | TYR | A | 107 | -3.758 | -11.746 | 31.414 | 1.00 | 48.15 | C |
| ATOM | 847 | OH | TYR | A | 107 | -3.279 | -11.702 | 32.703 | 1.00 | 71.00 | O |
| ATOM | 848 | N | TRP | A | 108 | -6.638 | -9.967 | 25.066 | 1.00 | 27.81 | N |
| ATOM | 849 | CA | TRP | A | 108 | -7.055 | -9.741 | 23.679 | 1.00 | 21.18 | C |
| ATOM | 850 | C | TRP | A | 108 | -5.892 | -9.324 | 22.782 | 1.00 | 26.32 | C |

(continued)

| | | | | | | | | | | |
|---|---|---|---|---|---|---|---|---|---|---|
| ATOM | 851 | O | TRP | A | 108 | -4.984 | -8.615 | 23.218 | 1.00 | 29.92 | O |
| ATOM | 852 | CB | TRP | A | 108 | -8.132 | -8.654 | 23.610 | 1.00 | 21.90 | C |
| ATOM | 853 | CG | TRP | A | 108 | -9.444 | -9.007 | 24.233 | 1.00 | 27.98 | C |
| ATOM | 854 | CD1 | TRP | A | 108 | -9.700 | -9.207 | 25.560 | 1.00 | 27.08 | C |
| ATOM | 855 | CD2 | TRP | A | 108 | -10.693 | -9.171 | 23.554 | 1.00 | 27.01 | C |
| ATOM | 856 | CE2 | TRP | A | 108 | -11.663 | -9.484 | 24.530 | 1.00 | 25.58 | C |
| ATOM | 857 | CE3 | TRP | A | 108 | -11.085 | -9.093 | 22.212 | 1.00 | 25.64 | C |
| ATOM | 858 | NE1 | TRP | A | 108 | -11.032 | -9.500 | 25.746 | 1.00 | 24.70 | N |
| ATOM | 859 | CZ2 | TRP | A | 108 | -12.999 | -9.715 | 24.208 | 1.00 | 25.47 | C |
| ATOM | 860 | CZ3 | TRP | A | 108 | -12.414 | -9.322 | 21.893 | 1.00 | 32.29 | C |
| ATOM | 861 | CH2 | TRP | A | 108 | -13.355 | -9.630 | 22.889 | 1.00 | 32.24 | C |
| ATOM | 862 | N | GLY | A | 109 | -5.928 | -9.748 | 21.523 | 1.00 | 27.02 | N |
| ATOM | 863 | CA | GLY | A | 109 | -5.002 | -9.227 | 20.531 | 1.00 | 28.74 | C |
| ATOM | 864 | C | GLY | A | 109 | -5.415 | -7 . 812 | 20.159 | 1.00 | 31. 88 | C |
| ATOM | 865 | O | GLY | A | 109 | -6.476 | -7.346 | 20.584 | 1.00 | 26.79 | O |
| ATOM | 866 | N | GLN | A | 110 | -4.595 | -7.120 | 19.373 | 1.00 | 28.50 | N |
| ATOM | 867 | CA | GLN | A | 110 | -4.878 | -5.721 | 19.055 | 1.00 | 29.47 | C |
| ATOM | 868 | C | GLN | A | 110 | -5.904 | -5.611 | 17.928 | 1.00 | 30.21 | C |
| ATOM | 869 | O | GLN | A | 110 | -6.432 | -4.532 | 17.659 | 1.00 | 36.00 | O |
| ATOM | 870 | CB | GLN | A | 110 | -3.591 | -4.966 | 18.683 | 1.00 | 23.57 | C |
| ATOM | 871 | CG | GLN | A | 110 | -3.125 | -5.156 | 17.239 | 1.00 | 29.23 | C |
| ATOM | 872 | CD | GLN | A | 110 | -2.309 | -6.419 | 17.032 | 1.00 | 33.10 | C |
| ATOM | 873 | NE2 | GLN | A | 110 | -1.568 | -6.467 | 15.929 | 1.00 | 31.73 | N |
| ATOM | 874 | OE1 | GLN | A | 110 | -2.343 | -7.342 | 17.850 | 1.00 | 35.23 | O |
| ATOM | 875 | N | GLY | A | 111 | -6.192 | -6.733 | 17.277 | 1.00 | 31.62 | N |
| ATOM | 876 | CA | GLY | A | 111 | -7.188 | -6.760 | 16.225 | 1.00 | 31.32 | C |
| ATOM | 877 | C | GLY | A | 111 | -6.604 | -6.675 | 14.831 | 1.00 | 34.22 | C |
| ATOM | 878 | O | GLY | A | 111 | -5.561 | -6.059 | 14.612 | 1.00 | 31.13 | O |
| ATOM | 879 | N | THR | A | 112 | -7.291 | -7.299 | 13.882 | 1.00 | 29.50 | N |
| ATOM | 880 | CA | THR | A | 112 | -6.858 | -7.300 | 12.494 | 1.00 | 30.89 | C |
| ATOM | 881 | C | THR | A | 112 | -8.008 | -6.901 | 11.587 | 1.00 | 30.69 | C |
| ATOM | 882 | O | THR | A | 112 | -9.035 | -7.577 | 11.551 | 1.00 | 28.68 | O |
| ATOM | 883 | CB | THR | A | 112 | -6.334 | -8.683 | 12.064 | 1.00 | 34.70 | C |
| ATOM | 884 | CG2 | THR | A | 112 | -5.960 | -8.683 | 10.584 | 1.00 | 33.17 | C |

(continued)

| | | | | | | | | | | | |
|---|---|---|---|---|---|---|---|---|---|---|---|
| ATOM | 885 | OG1 | THR | A | 112 | -5.182 | -9.025 | 12.846 | 1.00 | 39.75 | O |
| ATOM | 886 | N | LEU | A | 113 | -7 . 842 | -5.804 | 10.859 | 1.00 | 28.77 | N |
| ATOM | 887 | CA | LEU | A | 113 | -8.868 | -5.372 | 9.917 | 1.00 | 26.18 | C |
| ATOM | 888 | C | LEU | A | 113 | -8.801 | -6.203 | 8.635 | 1.00 | 32.80 | C |
| ATOM | 889 | O | LEU | A | 113 | -7.769 | -6.263 | 7.968 | 1.00 | 31.11 | O |
| ATOM | 890 | CB | LEU | A | 113 | -8.721 | -3.883 | 9.587 | 1.00 | 26.67 | C |
| ATOM | 891 | CG | LEU | A | 113 | -9.680 | -3.339 | 8.518 | 1.00 | 32.26 | C |
| ATOM | 892 | CD1 | LEU | A | 113 | -11.139 | -3.581 | 8.899 | 1.00 | 29.02 | C |
| ATOM | 893 | CD2 | LEU | A | 113 | -9.437 | -1.855 | 8.262 | 1.00 | 32.24 | C |
| ATOM | 894 | N | VAL | A | 114 | -9.904 | -6.859 | 8.305 | 1.00 | 28.44 | N |
| ATOM | 895 | CA | VAL | A | 114 | -10.009 | -7.566 | 7.036 | 1.00 | 30.93 | C |
| ATOM | 896 | C | VAL | A | 114 | -11.076 | -6.899 | 6.188 | 1.00 | 30.62 | C |
| ATOM | 897 | O | VAL | A | 114 | -12.233 | -6.788 | 6.601 | 1.00 | 29.64 | O |
| ATOM | 898 | CB | VAL | A | 114 | -10.353 | -9.058 | 7.225 | 1.00 | 31.72 | C |
| ATOM | 899 | CG1 | VAL | A | 114 | -10.535 | -9.740 | 5.869 | 1.00 | 33.36 | C |
| ATOM | 900 | CG2 | VAL | A | 114 | -9.265 | -9.750 | 8.042 | 1.00 | 32.90 | C |
| ATOM | 901 | N | THR | A | 115 | -10.676 | -6.436 | 5.009 | 1.00 | 35.29 | N |
| ATOM | 902 | CA | THR | A | 115 | -11.604 | -5.788 | 4.100 | 1.00 | 34.33 | C |
| ATOM | 903 | C | THR | A | 115 | -11.863 | -6.700 | 2.912 | 1.00 | 36.39 | C |
| ATOM | 904 | O | THR | A | 115 | -10.929 | -7.156 | 2.251 | 1.00 | 35.90 | O |
| ATOM | 905 | CB | THR | A | 115 | -11.068 | -4.426 | 3.610 | 1.00 | 36.40 | C |
| ATOM | 906 | CG2 | THR | A | 115 | -12.038 | -3.785 | 2.628 | 1.00 | 34.46 | C |
| ATOM | 907 | OG1 | THR | A | 115 | -10.890 | -3.552 | 4.730 | 1.00 | 37.23 | O |
| ATOM | 908 | N | VAL | A | 116 | -13.136 | -6.976 | 2.657 | 1.00 | 39.45 | N |
| ATOM | 909 | CA | VAL | A | 116 | -13.527 | -7.775 | 1.504 | 1.00 | 34.84 | C |
| ATOM | 910 | C | VAL | A | 116 | -14.212 | -6.875 | 0.490 | 1.00 | 38.13 | C |
| ATOM | 911 | O | VAL | A | 116 | -15.289 | -6.339 | 0.755 | 1.00 | 35.45 | O |
| ATOM | 912 | CB | VAL | A | 116 | -14.475 | -8.932 | 1.886 | 1.00 | 39.24 | C |
| ATOM | 913 | CG1 | VAL | A | 116 | -14.679 | -9.864 | 0.693 | 1.00 | 39.66 | C |
| ATOM | 914 | CG2 | VAL | A | 116 | -13.924 | -9.705 | 3.075 | 1.00 | 38.07 | C |
| ATOM | 915 | N | SER | A | 117 | -13.589 | -6.706 | -0.671 | 1.00 | 34.44 | N |
| ATOM | 916 | CA | SER | A | 117 | -14.126 | -5.795 | -1.671 | 1.00 | 40.89 | C |
| ATOM | 917 | C | SER | A | 117 | -13.687 | -6.131 | -3.091 | 1.00 | 40.89 | C |
| ATOM | 918 | O | SER | A | 117 | -12.598 | -6.661 | -3.313 | 1.00 | 38.45 | O |

(continued)

| | | | | | | | | | | | |
|---|---|---|---|---|---|---|---|---|---|---|---|
| ATOM | 919 | CB | SER | A | 117 | -13.716 | -4.359 | -1.345 | 1.00 | 34.47 | C |
| ATOM | 920 | OG | SER | A | 117 | -14.224 | -3.460 | -2.318 | 1.00 | 43.36 | O |
| ATOM | 921 | O | SER | A | 118 | -12.636 | -4.911 | -6.922 | 1.00 | 43.31 | O |
| ATOM | 922 | N | SER | A | 118 | -14.546 | -5.795 | -4.047 | 1.00 | 42.01 | N |
| ATOM | 923 | CA | SER | A | 118 | -14.232 | -5.940 | -5.462 | 1.00 | 52.20 | C |
| ATOM | 924 | C | SER | A | 118 | -13.391 | -4.770 | -5.963 | 1.00 | 47.46 | C |
| ATOM | 925 | CB | SER | A | 118 | -15.516 | -6.046 | -6.287 | 1.00 | 44.49 | C |
| ATOM | 926 | OG | SER | A | 118 | -16.364 | -7.057 | -5.772 | 1.00 | 64.31 | O |
| ATOM | 927 | O | ALA | A | 119 | -10.794 | -3.378 | -5.023 | 1.00 | 46.60 | O |
| ATOM | 928 | N | ALA | A | 119 | -13.530 | -3.617 | -5.311 | 1.00 | 44.58 | N |
| ATOM | 929 | CA | ALA | A | 119 | -12.862 | -2.393 | -5.753 | 1.00 | 40.93 | C |
| ATOM | 930 | C | ALA | A | 119 | -11.342 | -2.520 | -5.714 | 1.00 | 45.33 | C |
| ATOM | 931 | CB | ALA | A | 119 | -13.312 | -1.211 | -4.908 | 1.00 | 36.58 | C |
| ATOM | 932 | N | SER | A | 120 | -10.666 | -1.656 | -6.462 | 1.00 | 46.47 | N |
| ATOM | 933 | CA | SER | A | 120 | -9.215 | -1.709 | -6.568 | 1.00 | 45.38 | C |
| ATOM | 934 | C | SER | A | 120 | -8.519 | -1.011 | -5.404 | 1.00 | 40.94 | C |
| ATOM | 935 | O | SER | A | 120 | -8.988 | 0.011 | -4.907 | 1.00 | 41.57 | O |
| ATOM | 936 | CB | SER | A | 120 | -8.765 | -1.087 | -7.888 | 1.00 | 48.55 | C |
| ATOM | 937 | OG | SER | A | 120 | -9.353 | -1.760 | -8.986 | 1.00 | 60.18 | O |
| ATOM | 938 | N | THR | A | 121 | -7.396 | -1.579 | -4.977 | 1.00 | 34.74 | N |
| ATOM | 939 | CA | THR | A | 121 | -6.548 | -0.974 | -3.960 | 1.00 | 32.02 | C |
| ATOM | 940 | C | THR | A | 121 | -5.820 | 0.246 | -4.520 | 1.00 | 36.76 | C |
| ATOM | 941 | O | THR | A | 121 | -5.170 | 0.163 | -5.560 | 1.00 | 38.97 | O |
| ATOM | 942 | CB | THR | A | 121 | -5.515 | -1.984 | -3.424 | 1.00 | 34.58 | C |
| ATOM | 943 | CG2 | THR | A | 121 | -4.467 | -1.287 | -2.564 | 1.00 | 34.08 | C |
| ATOM | 944 | OG1 | THR | A | 121 | -6.183 | -2.982 | -2.643 | 1.00 | 35.30 | O |
| ATOM | 945 | N | LYS | A | 122 | -5.925 | 1.374 | -3.822 | 1.00 | 38.80 | N |
| ATOM | 946 | CA | LYS | A | 122 | -5.311 | 2.619 | -4.276 | 1.00 | 33.51 | C |
| ATOM | 947 | C | LYS | A | 122 | -4.612 | 3.348 | -3.137 | 1.00 | 34.96 | C |
| ATOM | 948 | O | LYS | A | 122 | -5.217 | 3.618 | -2.103 | 1.00 | 36.45 | O |
| ATOM | 949 | CB | LYS | A | 122 | -6.364 | 3.533 | -4.907 | 1.00 | 31.35 | C |
| ATOM | 950 | CG | LYS | A | 122 | -5.799 | 4.818 | -5.474 | 1.00 | 41.47 | C |
| ATOM | 951 | CD | LYS | A | 122 | -6.894 | 5.726 | -6.013 | 1.00 | 44.52 | C |
| ATOM | 952 | CE | LYS | A | 122 | -6.300 | 6.975 | -6.653 | 1.00 | 45.34 | C |

(continued)

| | | | | | | | | | | |
|---|---|---|---|---|---|---|---|---|---|---|
| ATOM | 953 | NZ | LYS | A | 122 | -5.380 | 7.695 | -5.723 | 1.00 | 52.67 | N |
| ATOM | 954 | N | GLY | A | 123 | -3.336 | 3.665 | -3.328 | 1.00 | 34.17 | N |
| ATOM | 955 | CA | GLY | A | 123 | -2.581 | 4.418 | -2.344 | 1.00 | 31.92 | C |
| ATOM | 956 | C | GLY | A | 123 | -2.957 | 5.889 | -2.359 | 1.00 | 38.36 | C |
| ATOM | 957 | O | GLY | A | 123 | -3.423 | 6.403 | -3.375 | 1.00 | 39.10 | O |
| ATOM | 958 | N | PRO | A | 124 | -2.747 | 6.578 | -1.228 | 1.00 | 35.34 | N |
| ATOM | 959 | CA | PRO | A | 124 | -3.181 | 7.963 | -1.024 | 1.00 | 33.85 | C |
| ATOM | 960 | C | PRO | A | 124 | -2.245 | 9.021 | -1.600 | 1.00 | 38.95 | C |
| ATOM | 961 | O | PRO | A | 124 | -1.036 | 8.809 | -1.689 | 1.00 | 33.76 | O |
| ATOM | 962 | CB | PRO | A | 124 | -3.209 | 8.084 | 0.497 | 1.00 | 31.61 | C |
| ATOM | 963 | CG | PRO | A | 124 | -2.100 | 7.184 | 0.944 | 1.00 | 31.80 | C |
| ATOM | 964 | CD | PRO | A | 124 | -2.095 | 6.024 | -0.026 | 1.00 | 35.65 | C |
| ATOM | 965 | N | SER | A | 125 | -2.817 | 10.160 | -1.974 | 1.00 | 31.51 | N |
| ATOM | 966 | CA | SER | A | 125 | -2.038 | 11.366 | -2.216 | 1.00 | 34.17 | C |
| ATOM | 967 | C | SER | A | 125 | -2.015 | 12.176 | -0.926 | 1.00 | 32.77 | C |
| ATOM | 968 | O | SER | A | 125 | -3.041 | 12.315 | -0.261 | 1.00 | 38.58 | O |
| ATOM | 969 | CB | SER | A | 125 | -2.631 | 12.189 | -3.362 | 1.00 | 40.17 | C |
| ATOM | 970 | OG | SER | A | 125 | -2.812 | 11.397 | -4.524 | 1.00 | 45.30 | O |
| ATOM | 971 | N | VAL | A | 126 | -0.851 | 12.695 | -0.557 | 1.00 | 28.64 | N |
| ATOM | 972 | CA | VAL | A | 126 | -0.734 | 13.466 | 0.674 | 1.00 | 28.98 | C |
| ATOM | 973 | C | VAL | A | 126 | -0.444 | 14.927 | 0.363 | 1.00 | 33.93 | C |
| ATOM | 974 | O | VAL | A | 126 | 0.556 | 15.244 | -0.279 | 1.00 | 37.76 | O |
| ATOM | 975 | CB | VAL | A | 126 | 0.367 | 12.905 | 1.593 | 1.00 | 28.89 | C |
| ATOM | 976 | CG1 | VAL | A | 126 | 0.374 | 13.641 | 2.930 | 1.00 | 28.32 | C |
| ATOM | 977 | CG2 | VAL | A | 126 | 0.169 | 11.413 | 1.797 | 1.00 | 24.48 | C |
| ATOM | 978 | N | PHE | A | 127 | -1.325 | 15.809 | 0.825 | 1.00 | 29.15 | N |
| ATOM | 979 | CA | PHE | A | 127 | -1.212 | 17.236 | 0.557 | 1.00 | 31.17 | C |
| ATOM | 980 | C | PHE | A | 127 | -1.034 | 18.028 | 1.850 | 1.00 | 38.80 | C |
| ATOM | 981 | O | PHE | A | 127 | -1.578 | 17.657 | 2.892 | 1.00 | 31.26 | O |
| ATOM | 982 | CB | PHE | A | 127 | -2.448 | 17.739 | -0.198 | 1.00 | 29.74 | C |
| ATOM | 983 | CG | PHE | A | 127 | -2.709 | 17.013 | -1.484 | 1.00 | 32.93 | C |
| ATOM | 984 | CD1 | PHE | A | 127 | -1.798 | 17.076 | -2.529 | 1.00 | 36.91 | C |
| ATOM | 985 | CD2 | PHE | A | 127 | -3.869 | 16.275 | -1.657 | 1.00 | 33.66 | C |
| ATOM | 986 | CE1 | PHE | A | 127 | -2.033 | 16.407 | -3.718 | 1.00 | 30.94 | C |

(continued)

| | | | | | | | | | | | |
|---|---|---|---|---|---|---|---|---|---|---|---|
| ATOM | 987 | CE2 | PHE | A | 127 | -4.114 | 15.605 | -2.848 | 1.00 | 38.29 | C |
| ATOM | 988 | CZ | PHE | A | 127 | -3.194 | 15.670 | -3.878 | 1.00 | 33.93 | C |
| ATOM | 989 | N | PRO | A | 128 | -0.278 | 19.134 | 1.785 | 1.00 | 38.69 | N |
| ATOM | 990 | CA | PRO | A | 128 | -0.029 | 19.931 | 2.990 | 1.00 | 31.44 | C |
| ATOM | 991 | C | PRO | A | 128 | -1.230 | 20.772 | 3.410 | 1.00 | 34.99 | C |
| ATOM | 992 | O | PRO | A | 128 | -1.930 | 21.318 | 2.562 | 1.00 | 36.42 | O |
| ATOM | 993 | CB | PRO | A | 128 | 1.136 | 20.832 | 2.575 | 1.00 | 35.21 | C |
| ATOM | 994 | CG | PRO | A | 128 | 0.966 | 20.997 | 1.104 | 1.00 | 31.99 | C |
| ATOM | 995 | CD | PRO | A | 128 | 0.395 | 19.694 | 0.598 | 1.00 | 31.14 | C |
| ATOM | 996 | N | LEU | A | 129 | -1.470 | 20.845 | 4.714 | 1.00 | 31. 68 | N |
| ATOM | 997 | CA | LEU | A | 129 | -2.354 | 21.853 | 5.280 | 1.00 | 32.81 | C |
| ATOM | 998 | C | LEU | A | 129 | -1.451 | 22.903 | 5.908 | 1.00 | 29.54 | C |
| ATOM | 999 | O | LEU | A | 129 | -1.054 | 22.777 | 7.063 | 1.00 | 28.79 | O |
| ATOM | 1000 | CB | LEU | A | 129 | -3.309 | 21.249 | 6.314 | 1.00 | 28.35 | C |
| ATOM | 1001 | CG | LEU | A | 129 | -4.272 | 20.181 | 5.787 | 1.00 | 32.12 | C |
| ATOM | 1002 | CD1 | LEU | A | 129 | -5.104 | 19.577 | 6.917 | 1.00 | 27.87 | C |
| ATOM | 1003 | CD2 | LEU | A | 129 | -5.170 | 20.762 | 4.700 | 1.00 | 27.99 | C |
| ATOM | 1004 | N | ALA | A | 130 | -1.106 | 23.923 | 5.130 | 1.00 | 30.89 | N |
| ATOM | 1005 | CA | ALA | A | 130 | -0.044 | 24. 853 | 5.509 | 1.00 | 38.77 | C |
| ATOM | 1006 | C | ALA | A | 130 | -0.484 | 25.820 | 6.595 | 1.00 | 37.50 | C |
| ATOM | 1007 | O | ALA | A | 130 | -1.576 | 26.378 | 6.524 | 1.00 | 41.37 | O |
| ATOM | 1008 | CB | ALA | A | 130 | 0.445 | 25.624 | 4.286 | 1.00 | 37.41 | C |
| ATOM | 1009 | N | PRO | A | 131 | 0.381 | 26.031 | 7.599 | 1.00 | 45.74 | N |
| ATOM | 1010 | CA | PRO | A | 131 | 0.059 | 26.928 | 8.714 | 1.00 | 46.02 | C |
| ATOM | 1011 | C | PRO | A | 131 | -0.111 | 28.370 | 8.245 | 1.00 | 52.71 | C |
| ATOM | 1012 | O | PRO | A | 131 | 0.650 | 28.845 | 7.397 | 1.00 | 47.91 | O |
| ATOM | 1013 | CB | PRO | A | 131 | 1.264 | 26.778 | 9.647 | 1.00 | 40.81 | C |
| ATOM | 1014 | CG | PRO | A | 131 | 2.376 | 26.354 | 8.764 | 1.00 | 45.92 | C |
| ATOM | 1015 | CD | PRO | A | 131 | 1.751 | 25.499 | 7.694 | 1.00 | 43.34 | C |
| ATOM | 1016 | N | SER | A | 132 | -1.117 | 29.043 | 8.793 | 1.00 | 55.56 | N |
| ATOM | 1017 | CA | SER | A | 132 | -1.473 | 30.396 | 8.380 | 1.00 | 71.89 | C |
| ATOM | 1018 | C | SER | A | 132 | -0.309 | 31.371 | 8.517 | 1.00 | 74.46 | C |
| ATOM | 1019 | O | SER | A | 132 | 0.408 | 31.364 | 9.518 | 1.00 | 79.24 | O |
| ATOM | 1020 | CB | SER | A | 132 | -2.670 | 30.896 | 9.198 | 1.00 | 76.95 | C |

(continued)

| | | | | | | | | | | | |
|---|---|---|---|---|---|---|---|---|---|---|---|
| ATOM | 1021 | OG | SER | A | 132 | -3.053 | 32.204 | 8.811 | 1.00 | 77.97 | O |
| ATOM | 1022 | N | SER | A | 133 | -0.120 | 32.201 | 7.496 | 1.00 | 80.68 | N |
| ATOM | 1023 | CA | SER | A | 133 | 0.838 | 33.296 | 7.575 | 1.00 | 89.81 | C |
| ATOM | 1024 | C | SER | A | 133 | 0.309 | 34.331 | 8.560 | 1.00 | 95.47 | C |
| ATOM | 1025 | O | SER | A | 133 | 1.072 | 35.087 | 9.166 | 1.00 | 92.23 | O |
| ATOM | 1026 | CB | SER | A | 133 | 1.075 | 33.922 | 6.198 | 1.00 | 81.20 | C |
| ATOM | 1027 | OG | SER | A | 133 | -0.144 | 34.340 | 5.609 | 1.00 | 74. 80 | O |
| ATOM | 1028 | N | LYS | A | 134 | -1.012 | 34.343 | 8.717 | 1.00 | 93.18 | N |
| ATOM | 1029 | CA | LYS | A | 134 | -1.678 | 35.224 | 9.665 | 1.00 | 90.77 | C |
| ATOM | 1030 | C | LYS | A | 134 | -1.923 | 34.525 | 11.001 | 1.00 | 94.50 | C |
| ATOM | 1031 | O | LYS | A | 134 | -2.896 | 34.825 | 11.696 | 1.00 | 94.00 | O |
| ATOM | 1032 | CB | LYS | A | 134 | -3.003 | 35.729 | 9.089 | 1.00 | 86.16 | C |
| ATOM | 1033 | CG | LYS | A | 134 | -2.852 | 36.635 | 7.879 | 1.00 | 83.75 | C |
| ATOM | 1034 | CD | LYS | A | 134 | -4.135 | 37.409 | 7.614 | 1.00 | 91.56 | C |
| ATOM | 1035 | CE | LYS | A | 134 | -3.926 | 38.492 | 6.567 | 1.00 | 95.47 | C |
| ATOM | 1036 | NZ | LYS | A | 134 | -5.110 | 39.389 | 6.446 | 1.00 | 88.66 | N |
| ATOM | 1037 | N | SER | A | 135 | -1.045 | 33.588 | 11.353 | 1.00 | 96.11 | N |
| ATOM | 1038 | CA | SER | A | 135 | -1.111 | 32.936 | 12.658 | 1.00 | 97.73 | C |
| ATOM | 1039 | C | SER | A | 135 | -0.885 | 33.973 | 13.753 | 1.00 | 102.54 | C |
| ATOM | 1040 | O | SER | A | 135 | -0.148 | 34.941 | 13.555 | 1.00 | 102.52 | O |
| ATOM | 1041 | CB | SER | A | 135 | -0.077 | 31.810 | 12.768 | 1.00 | 83.08 | C |
| ATOM | 1042 | OG | SER | A | 135 | -0.335 | 30.775 | 11.834 | 1.00 | 78.24 | O |
| ATOM | 1043 | N | THR | A | 136 | -1.524 | 33.775 | 14.901 | 1.00 | 102.47 | N |
| ATOM | 1044 | CA | THR | A | 136 | -1.399 | 34.717 | 16.009 | 1.00 | 105.40 | C |
| ATOM | 1045 | C | THR | A | 136 | 0.030 | 34.741 | 16.550 | 1.00 | 104.11 | C |
| ATOM | 1046 | O | THR | A | 136 | 0.518 | 33.744 | 17.085 | 1.00 | 99.44 | O |
| ATOM | 1047 | CB | THR | A | 136 | -2.377 | 34.379 | 17.154 | 1.00 | 99.90 | C |
| ATOM | 1048 | CG2 | THR | A | 136 | -3.817 | 34.548 | 16.692 | 1.00 | 100.80 | C |
| ATOM | 1049 | OG1 | THR | A | 136 | -2.173 | 33.027 | 17.582 | 1.00 | 97.52 | O |
| ATOM | 1050 | N | SER | A | 137 | 0.696 | 35.883 | 16.395 | 1.00 | 105.35 | N |
| ATOM | 1051 | CA | SER | A | 137 | 2.071 | 36.049 | 16.858 | 1.00 | 104.45 | C |
| ATOM | 1052 | C | SER | A | 137 | 2.168 | 35.866 | 18.370 | 1.00 | 102.60 | C |
| ATOM | 1053 | O | SER | A | 137 | 1.517 | 36.580 | 19.135 | 1.00 | 98.58 | O |
| ATOM | 1054 | CB | SER | A | 137 | 2.610 | 37.424 | 16.456 | 1.00 | 105.78 | C |

(continued)

| | | | | | | | | | | | |
|---|---|---|---|---|---|---|---|---|---|---|---|
| ATOM | 1055 | OG | SER | A | 137 | 2.624 | 37.578 | 15.047 | 1.00 | 96.80 | O |
| ATOM | 1056 | N | GLY | A | 138 | 2.981 | 34.902 | 18.790 | 1.00 | 97.35 | N |
| ATOM | 1057 | CA | GLY | A | 138 | 3.115 | 34.571 | 20.197 | 1.00 | 98.58 | C |
| ATOM | 1058 | C | GLY | A | 138 | 1.978 | 33.692 | 20.685 | 1.00 | 99.72 | C |
| ATOM | 1059 | O | GLY | A | 138 | 1.816 | 33.480 | 21.888 | 1.00 | 98.83 | O |
| ATOM | 1060 | N | GLY | A | 139 | 1.188 | 33.180 | 19.744 | 1.00 | 93.81 | N |
| ATOM | 1061 | CA | GLY | A | 139 | 0.044 | 32.348 | 20.066 | 1.00 | 77.07 | C |
| ATOM | 1062 | C | GLY | A | 139 | 0.181 | 30.926 | 19.557 | 1.00 | 70.80 | C |
| ATOM | 1063 | O | GLY | A | 139 | 1.247 | 30.316 | 19.662 | 1.00 | 65.42 | O |
| ATOM | 1064 | N | THR | A | 140 | -0.904 | 30.398 | 18.998 | 1.00 | 65.95 | N |
| ATOM | 1065 | CA | THR | A | 140 | -0.941 | 29.012 | 18.541 | 1.00 | 60.59 | C |
| ATOM | 1066 | C | THR | A | 140 | -1.281 | 28.906 | 17.053 | 1.00 | 54.28 | C |
| ATOM | 1067 | O | THR | A | 140 | -2.238 | 29.520 | 16.580 | 1.00 | 54.81 | O |
| ATOM | 1068 | CB | THR | A | 140 | -1.963 | 28.189 | 19.360 | 1.00 | 57.65 | C |
| ATOM | 1069 | CG2 | THR | A | 140 | -2.218 | 26.832 | 18.719 | 1.00 | 45.39 | C |
| ATOM | 1070 | OG1 | THR | A | 140 | -1.463 | 27.993 | 20.689 | 1.00 | 65.11 | O |
| ATOM | 1071 | N | ALA | A | 141 | -0.488 | 28.129 | 16.321 | 1.00 | 41.19 | N |
| ATOM | 1072 | CA | ALA | A | 141 | -0.767 | 27.862 | 14.914 | 1.00 | 47. 82 | C |
| ATOM | 1073 | C | ALA | A | 141 | -1.193 | 26.410 | 14.708 | 1.00 | 39.86 | C |
| ATOM | 1074 | O | ALA | A | 141 | -0.881 | 25.541 | 15.521 | 1.00 | 42.63 | O |
| ATOM | 1075 | CB | ALA | A | 141 | 0.447 | 28.181 | 14.059 | 1.00 | 33.31 | C |
| ATOM | 1076 | N | ALA | A | 142 | -1.911 | 26.151 | 13.621 | 1.00 | 35.54 | N |
| ATOM | 1077 | CA | ALA | A | 142 | -2.279 | 24.787 | 13.265 | 1.00 | 32.56 | C |
| ATOM | 1078 | C | ALA | A | 142 | -1.747 | 24.446 | 11.885 | 1.00 | 30.50 | C |
| ATOM | 1079 | O | ALA | A | 142 | -1.761 | 25.276 | 10.978 | 1.00 | 39.38 | O |
| ATOM | 1080 | CB | ALA | A | 142 | -3.791 | 24.600 | 13.317 | 1.00 | 33.11 | C |
| ATOM | 1081 | N | LEU | A | 143 | -1.267 | 23.220 | 11.735 | 1.00 | 29.03 | N |
| ATOM | 1082 | CA | LEU | A | 143 | -0.827 | 22.726 | 10.447 | 1.00 | 27.15 | C |
| ATOM | 1083 | C | LEU | A | 143 | -1.214 | 21.263 | 10.343 | 1.00 | 29.85 | C |
| ATOM | 1084 | O | LEU | A | 143 | -1.577 | 20.643 | 11.340 | 1.00 | 30.37 | O |
| ATOM | 1085 | CB | LEU | A | 143 | 0.681 | 22.922 | 10.267 | 1.00 | 31.63 | C |
| ATOM | 1086 | CG | LEU | A | 143 | 1.648 | 22.236 | 11.232 | 1.00 | 36.90 | C |
| ATOM | 1087 | CD1 | LEU | A | 143 | 2.145 | 20.915 | 10.665 | 1.00 | 35.87 | C |
| ATOM | 1088 | CD2 | LEU | A | 143 | 2.817 | 23.154 | 11.551 | 1.00 | 37.42 | C |

(continued)

| | | | | | | | | | | | |
|---|---|---|---|---|---|---|---|---|---|---|---|
| ATOM | 1089 | N | GLY | A | 144 | -1.155 | 20.708 | 9.140 | 1.00 | 25.86 | N |
| ATOM | 1090 | CA | GLY | A | 144 | -1.542 | 19.327 | 8.968 | 1.00 | 28.52 | C |
| ATOM | 1091 | C | GLY | A | 144 | -1.254 | 18.715 | 7.615 | 1.00 | 24.81 | C |
| ATOM | 1092 | O | GLY | A | 144 | -0.577 | 19.300 | 6.774 | 1.00 | 28.21 | O |
| ATOM | 1093 | N | CYS | A | 145 | -1.779 | 17.511 | 7.428 | 1.00 | 23.50 | N |
| ATOM | 1094 | CA | CYS | A | 145 | -1.680 | 16.777 | 6.178 | 1.00 | 26.22 | C |
| ATOM | 1095 | C | CYS | A | 145 | -3.059 | 16.307 | 5.759 | 1.00 | 29.48 | C |
| ATOM | 1096 | O | CYS | A | 145 | -3.828 | 15.820 | 6.587 | 1.00 | 28.29 | O |
| ATOM | 1097 | CB | CYS | A | 145 | -0.745 | 15.567 | 6.317 | 1.00 | 32.48 | C |
| ATOM | 1098 | SG | CYS | A | 145 | 1.014 | 15.968 | 6.295 | 1.00 | 53.28 | S |
| ATOM | 1099 | N | LEU | A | 146 | -3.364 | 16.447 | 4.475 | 1.00 | 31.34 | N |
| ATOM | 1100 | CA | LEU | A | 146 | -4.579 | 15.883 | 3.911 | 1.00 | 25.63 | C |
| ATOM | 1101 | C | LEU | A | 146 | -4.235 | 14.603 | 3.157 | 1.00 | 30.29 | C |
| ATOM | 1102 | O | LEU | A | 146 | -3.490 | 14.627 | 2.177 | 1.00 | 34.41 | O |
| ATOM | 1103 | CB | LEU | A | 146 | -5.265 | 16.895 | 2.991 | 1.00 | 30.65 | C |
| ATOM | 1104 | CG | LEU | A | 146 | -6.575 | 16.493 | 2.311 | 1.00 | 36.27 | C |
| ATOM | 1105 | CD1 | LEU | A | 146 | -7.664 | 16.170 | 3.329 | 1.00 | 29.24 | C |
| ATOM | 1106 | CD2 | LEU | A | 146 | -7.027 | 17.607 | 1.378 | 1.00 | 36.45 | C |
| ATOM | 1107 | N | VAL | A | 147 | -4.772 | 13.485 | 3.628 | 1.00 | 30.39 | N |
| ATOM | 1108 | CA | VAL | A | 147 | -4.504 | 12.182 | 3.033 | 1.00 | 27.86 | C |
| ATOM | 1109 | C | VAL | A | 147 | -5.700 | 11.750 | 2.184 | 1.00 | 27.94 | C |
| ATOM | 1110 | O | VAL | A | 147 | -6.718 | 11.321 | 2.711 | 1.00 | 33.43 | O |
| ATOM | 1111 | CB | VAL | A | 147 | -4.209 | 11.143 | 4.123 | 1.00 | 30.05 | C |
| ATOM | 1112 | CG1 | VAL | A | 147 | -3.807 | 9.811 | 3.509 | 1.00 | 31.17 | C |
| ATOM | 1113 | CG2 | VAL | A | 147 | -3.116 | 11.661 | 5.053 | 1.00 | 29.02 | C |
| ATOM | 1114 | N | LYS | A | 148 | -5.579 | 11.870 | 0.866 | 1.00 | 32.05 | N |
| ATOM | 1115 | CA | LYS | A | 148 | -6.760 | 11.820 | 0.010 | 1.00 | 38.00 | C |
| ATOM | 1116 | C | LYS | A | 148 | -6.740 | 10.696 | -1.026 | 1.00 | 35.47 | C |
| ATOM | 1117 | O | LYS | A | 148 | -5.685 | 10.328 | -1.539 | 1.00 | 38.98 | O |
| ATOM | 1118 | CB | LYS | A | 148 | -6.935 | 13.172 | -0.697 | 1.00 | 39.17 | C |
| ATOM | 1119 | CG | LYS | A | 148 | -8.280 | 13.343 | -1.383 | 1.00 | 47.97 | C |
| ATOM | 1120 | CD | LYS | A | 148 | -8.527 | 14.785 | -1.779 | 1.00 | 41.88 | C |
| ATOM | 1121 | CE | LYS | A | 148 | -9.901 | 14.951 | -2.417 | 1.00 | 51.38 | C |
| ATOM | 1122 | NZ | LYS | A | 148 | -10.035 | 14.191 | -3.688 | 1.00 | 52.46 | N |

(continued)

| | | | | | | | | | | | |
|---|---|---|---|---|---|---|---|---|---|---|---|
| ATOM | 1123 | N | ASP | A | 149 | -7.929 | 10.160 | -1.309 | 1.00 | 39.61 | N |
| ATOM | 1124 | CA | ASP | A | 149 | -8.162 | 9.206 | -2.398 | 1.00 | 36.49 | C |
| ATOM | 1125 | C | ASP | A | 149 | -7.423 | 7. 886 | -2.225 | 1.00 | 40.38 | C |
| ATOM | 1126 | O | ASP | A | 149 | -6.620 | 7.499 | -3.076 | 1.00 | 38.28 | O |
| ATOM | 1127 | CB | ASP | A | 149 | -7.779 | 9.829 | -3.748 | 1.00 | 35.81 | C |
| ATOM | 1128 | CG | ASP | A | 149 | -8.690 | 10.975 | -4.139 | 1.00 | 44.23 | C |
| ATOM | 1129 | OD1 | ASP | A | 149 | -9.834 | 11.014 | -3.642 | 1.00 | 45.39 | O |
| ATOM | 1130 | OD2 | ASP | A | 149 | -8.265 | 11.835 | -4.940 | 1.00 | 51.78 | O |
| ATOM | 1131 | N | TYR | A | 150 | -7.702 | 7.185 | -1.133 | 1.00 | 28.61 | N |
| ATOM | 1132 | CA | TYR | A | 150 | -7.115 | 5.872 | -0.950 | 1.00 | 31. 03 | C |
| ATOM | 1133 | C | TYR | A | 150 | -8.197 | 4. 827 | -0.728 | 1.00 | 38.37 | C |
| ATOM | 1134 | O | TYR | A | 150 | -9.354 | 5.159 | -0.456 | 1.00 | 36.04 | O |
| ATOM | 1135 | CB | TYR | A | 150 | -6.113 | 5.872 | 0.212 | 1.00 | 29.48 | C |
| ATOM | 1136 | CG | TYR | A | 150 | -6.713 | 6.143 | 1.571 | 1.00 | 32.63 | C |
| ATOM | 1137 | GD1 | TYR | A | 150 | -6.837 | 7.439 | 2.051 | 1.00 | 31.16 | C |
| ATOM | 1138 | CD2 | TYR | A | 150 | -7.145 | 5.100 | 2.380 | 1.00 | 34.56 | C |
| ATOM | 1139 | CE1 | TYR | A | 150 | -7.381 | 7.689 | 3.295 | 1.00 | 26.37 | C |
| ATOM | 1140 | CE2 | TYR | A | 150 | -7.691 | 5.340 | 3.625 | 1.00 | 30.49 | C |
| ATOM | 1141 | CZ | TYR | A | 150 | -7.804 | 6.633 | 4.079 | 1.00 | 32.92 | C |
| ATOM | 1142 | OH | TYR | A | 150 | -8.347 | 6.865 | 5.322 | 1.00 | 33.53 | O |
| ATOM | 1143 | N | PHE | A | 151 | -7.807 | 3.565 | -0.866 | 1.00 | 30.04 | N |
| ATOM | 1144 | CA | PHE | A | 151 | -8.699 | 2.437 | -0.650 | 1.00 | 32.42 | C |
| ATOM | 1145 | C | PHE | A | 151 | -7.861 | 1.173 | -0.518 | 1.00 | 32.34 | C |
| ATOM | 1146 | O | PHE | A | 151 | -6.895 | 0.992 | -1.258 | 1.00 | 38.41 | O |
| ATOM | 1147 | CB | PHE | A | 151 | -9.708 | 2.298 | -1.799 | 1.00 | 32.75 | C |
| ATOM | 1148 | CG | PHE | A | 151 | -10.788 | 1.287 | -1.535 | 1.00 | 35.46 | C |
| ATOM | 1149 | CD2 | PHE | A | 151 | -11.978 | 1.669 | -0.936 | 1.00 | 38.75 | C |
| ATOM | 1150 | CD1 | PHE | A | 151 | -10.605 | -0.047 | -1.863 | 1.00 | 36.37 | C |
| ATOM | 1151 | CE2 | PHE | A | 151 | -12.969 | 0.743 | -0.676 | 1.00 | 37.03 | C |
| ATOM | 1152 | CE1 | PHE | A | 151 | -11.595 | -0.981 | -1.605 | 1.00 | 36.99 | C |
| ATOM | 1153 | CZ | PHE | A | 151 | -12.777 | -0.584 | -1.013 | 1.00 | 38.87 | C |
| ATOM | 1154 | N | PRO | A | 152 | -8.207 | 0.307 | 0.446 | 1.00 | 36.93 | N |
| ATOM | 1155 | CA | PRO | A | 152 | -9.241 | 0.535 | 1.456 | 1.00 | 33.39 | C |
| ATOM | 1156 | C | PRO | A | 152 | -8.638 | 1.119 | 2.726 | 1.00 | 36.06 | C |

(continued)

| | | | | | | | | | | | |
|---|---|---|---|---|---|---|---|---|---|---|---|
| ATOM | 1157 | O | PRO | A | 152 | -7.493 | 1.579 | 2.706 | 1.00 | 30.72 | O |
| ATOM | 1158 | CB | PRO | A | 152 | -9.775 | -0.869 | 1.707 | 1.00 | 35.39 | C |
| ATOM | 1159 | CG | PRO | A | 152 | -8.534 | -1.711 | 1.609 | 1.00 | 34.66 | C |
| ATOM | 1160 | CD | PRO | A | 152 | -7.652 | -1.056 | 0.552 | 1.00 | 34.24 | C |
| ATOM | 1161 | N | GLU | A | 153 | -9.400 | 1.095 | 3.816 | 1.00 | 31.54 | N |
| ATOM | 1162 | CA | GLU | A | 153 | -8.855 | 1.413 | 5.132 | 1.00 | 35.20 | C |
| ATOM | 1163 | C | GLU | A | 153 | -7.853 | 0.323 | 5.528 | 1.00 | 30.90 | C |
| ATOM | 1164 | O | GLU | A | 153 | -7.918 | -0.790 | 5.012 | 1.00 | 30.88 | O |
| ATOM | 1165 | CB | GLU | A | 153 | -9.979 | 1.531 | 6.170 | 1.00 | 34.19 | C |
| ATOM | 1166 | CG | GLU | A | 153 | -10.884 | 2.735 | 5.978 | 1.00 | 30.47 | C |
| ATOM | 1167 | CD | GLU | A | 153 | -10.607 | 3.838 | 6.980 | 1.00 | 49.19 | C |
| ATOM | 1168 | OE1 | GLU | A | 153 | -11.550 | 4.226 | 7.705 | 1.00 | 56.79 | O |
| ATOM | 1169 | OE2 | GLU | A | 153 | -9.452 | 4.323 | 7.042 | 1.00 | 53.78 | O |
| ATOM | 1170 | N | PRO | A | 154 | -6.923 | 0.634 | 6.444 | 1.00 | 31.40 | N |
| ATOM | 1171 | CA | PRO | A | 154 | -6.719 | 1.915 | 7.118 | 1.00 | 33.58 | C |
| ATOM | 1172 | C | PRO | A | 154 | -5.436 | 2.624 | 6.692 | 1.00 | 34.09 | C |
| ATOM | 1173 | O | PRO | A | 154 | -4.576 | 2.024 | 6.047 | 1.00 | 33.03 | O |
| ATOM | 1174 | CB | PRO | A | 154 | -6.614 | 1.494 | 8.576 | 1.00 | 27.86 | C |
| ATOM | 1175 | CG | PRO | A | 154 | -5.808 | 0.210 | 8.476 | 1.00 | 30.94 | C |
| ATOM | 1176 | CD | PRO | A | 154 | -6.178 | -0.435 | 7.133 | 1.00 | 33.22 | C |
| ATOM | 1177 | N | VAL | A | 155 | -5.306 | 3.892 | 7.059 | 1.00 | 34.38 | N |
| ATOM | 1178 | CA | VAL | A | 155 | -4.006 | 4.540 | 7.013 | 1.00 | 35.85 | C |
| ATOM | 1179 | C | VAL | A | 155 | -3.648 | 4.982 | 8.420 | 1.00 | 35.04 | C |
| ATOM | 1180 | O | VAL | A | 155 | -4.525 | 5.310 | 9.219 | 1.00 | 34.51 | O |
| ATOM | 1181 | CB | VAL | A | 155 | -3.966 | 5.755 | 6.060 | 1.00 | 36.38 | C |
| ATOM | 1182 | CG1 | VAL | A | 155 | -4.367 | 5.351 | 4.656 | 1.00 | 37.19 | C |
| ATOM | 1183 | CG2 | VAL | A | 155 | -4.850 | 6.870 | 6.572 | 1.00 | 41.10 | C |
| ATOM | 1184 | N | THR | A | 156 | -2.358 | 4.966 | 8.730 | 1.00 | 32.78 | N |
| ATOM | 1185 | CA | THR | A | 156 | -1.887 | 5.503 | 9.994 | 1.00 | 29.21 | C |
| ATOM | 1186 | C | THR | A | 156 | -1.074 | 6.759 | 9.733 | 1.00 | 35.34 | C |
| ATOM | 1187 | O | THR | A | 156 | -0.448 | 6.916 | 8.675 | 1.00 | 29.04 | O |
| ATOM | 1188 | CB | THR | A | 156 | -1.034 | 4.487 | 10.785 | 1.00 | 34.00 | C |
| ATOM | 1189 | CG2 | THR | A | 156 | -1.843 | 3.230 | 11.088 | 1.00 | 33.80 | C |
| ATOM | 1190 | OG1 | THR | A | 156 | 0.132 | 4.140 | 10.028 | 1.00 | 35.62 | O |

(continued)

| | | | | | | | | | | | |
|---|---|---|---|---|---|---|---|---|---|---|---|
| ATOM | 1191 | N | VAL | A | 157 | -1.099 | 7.665 | 10.700 | 1.00 | 27.89 | N |
| ATOM | 1192 | CA | VAL | A | 157 | -0.375 | 8.916 | 10.582 | 1.00 | 33.47 | C |
| ATOM | 1193 | C | VAL | A | 157 | 0.370 | 9.197 | 11.867 | 1.00 | 28.46 | C |
| ATOM | 1194 | O | VAL | A | 157 | -0.216 | 9.168 | 12.949 | 1.00 | 36.97 | O |
| ATOM | 1195 | CB | VAL | A | 157 | -1.319 | 10.100 | 10.276 | 1.00 | 28.34 | C |
| ATOM | 1196 | CG1 | VAL | A | 157 | -0.523 | 11.395 | 10.154 | 1.00 | 28.04 | C |
| ATOM | 1197 | CG2 | VAL | A | 157 | -2.120 | 9.833 | 9.011 | 1.00 | 26.05 | C |
| ATOM | 1198 | N | SER | A | 158 | 1.665 | 9.455 | 11.752 | 1.00 | 30.73 | N |
| ATOM | 1199 | CA | SER | A | 158 | 2.429 | 9.964 | 12.880 | 1.00 | 36.51 | C |
| ATOM | 1200 | C | SER | A | 158 | 3.087 | 11.267 | 12.461 | 1.00 | 32.08 | C |
| ATOM | 1201 | O | SER | A | 158 | 3.162 | 11.571 | 11.270 | 1.00 | 30.98 | O |
| ATOM | 1202 | CB | SER | A | 158 | 3.476 | 8.951 | 13.347 | 1.00 | 35.17 | C |
| ATOM | 1203 | OG | SER | A | 158 | 4.538 | 8.848 | 12.419 | 1.00 | 40.81 | O |
| ATOM | 1204 | N | TRP | A | 159 | 3.546 | 12.040 | 13.440 | 1.00 | 27.85 | N |
| ATOM | 1205 | CA | TRP | A | 159 | 4.263 | 13.276 | 13.166 | 1.00 | 30.67 | C |
| ATOM | 1206 | C | TRP | A | 159 | 5.684 | 13.207 | 13.707 | 1.00 | 31.39 | C |
| ATOM | 1207 | O | TRP | A | 159 | 5.900 | 12.792 | 14.845 | 1.00 | 30.84 | O |
| ATOM | 1208 | CB | TRP | A | 159 | 3.519 | 14.470 | 13.761 | 1.00 | 28.15 | C |
| ATOM | 1209 | CG | TRP | A | 159 | 2.278 | 14.795 | 13.004 | 1.00 | 35.31 | C |
| ATOM | 1210 | CD1 | TRP | A | 159 | 1.049 | 14.237 | 13.170 | 1.00 | 31.91 | C |
| ATOM | 1211 | CD2 | TRP | A | 159 | 2.149 | 15.745 | 11.942 | 1.00 | 33.46 | C |
| ATOM | 1212 | CE2 | TRP | A | 159 | 0.807 | 15.716 | 11.518 | 1.00 | 32.34 | C |
| ATOM | 1213 | CE3 | TRP | A | 159 | 3.038 | 16.620 | 11.310 | 1.00 | 33.99 | C |
| ATOM | 1214 | NE1 | TRP | A | 159 | 0.157 | 14.784 | 12.283 | 1.00 | 31.36 | N |
| ATOM | 1215 | CZ2 | TRP | A | 159 | 0.331 | 16.526 | 10.489 | 1.00 | 30.53 | C |
| ATOM | 1216 | CZ3 | TRP | A | 159 | 2.565 | 17.423 | 10.289 | 1.00 | 30.89 | C |
| ATOM | 1217 | CH2 | TRP | A | 159 | 1.223 | 17.372 | 9.890 | 1.00 | 33.28 | C |
| ATOM | 1218 | N | ASN | A | 160 | 6.639 | 13.617 | 12.875 | 1.00 | 26.79 | N |
| ATOM | 1219 | CA | ASN | A | 160 | 8.061 | 13.584 | 13.208 | 1.00 | 28.03 | C |
| ATOM | 1220 | C | ASN | A | 160 | 8.491 | 12.236 | 13.778 | 1.00 | 33.44 | C |
| ATOM | 1221 | O | ASN | A | 160 | 9.200 | 12.171 | 14.784 | 1.00 | 32.52 | O |
| ATOM | 1222 | CB | ASN | A | 160 | 8.405 | 14.708 | 14.185 | 1.00 | 27.50 | C |
| ATOM | 1223 | CG | ASN | A | 160 | 8.269 | 16.078 | 13.559 | 1.00 | 30.24 | C |
| ATOM | 1224 | ND2 | ASN | A | 160 | 8.292 | 17.119 | 14.385 | 1.00 | 34.83 | N |

(continued)

| | | | | | | | | | | | |
|---|---|---|---|---|---|---|---|---|---|---|---|
| ATOM | 1225 | OD1 | ASN | A | 160 | 8.153 | 16.199 | 12.342 | 1.00 | 37.06 | O |
| ATOM | 1226 | N | SER | A | 161 | 8.026 | 11.168 | 13.135 | 1.00 | 32.54 | N |
| ATOM | 1227 | CA | SER | A | 161 | 8.394 | 9.801 | 13.492 | 1.00 | 38.63 | C |
| ATOM | 1228 | C | SER | A | 161 | 7.988 | 9.425 | 14.916 | 1.00 | 37.84 | C |
| ATOM | 1229 | O | SER | A | 161 | 8.601 | 8.556 | 15.530 | 1.00 | 36.85 | O |
| ATOM | 1230 | CB | SER | A | 161 | 9.901 | 9.598 | 13.310 | 1.00 | 33.00 | C |
| ATOM | 1231 | OG | SER | A | 161 | 10.292 | 9.901 | 11.983 | 1.00 | 36.60 | O |
| ATOM | 1232 | N | GLY | A | 162 | 6.951 | 10.074 | 15.434 | 1.00 | 33.46 | N |
| ATOM | 1233 | CA | GLY | A | 162 | 6.456 | 9.771 | 16.764 | 1.00 | 33.72 | C |
| ATOM | 1234 | C | GLY | A | 162 | 7.005 | 10.686 | 17 . 843 | 1.00 | 35.30 | C |
| ATOM | 1235 | O | GLY | A | 162 | 6.639 | 10.568 | 19.011 | 1.00 | 42.69 | O |
| ATOM | 1236 | N | ALA | A | 163 | 7.885 | 11.602 | 17.457 | 1.00 | 37.22 | N |
| ATOM | 1237 | CA | ALA | A | 163 | 8.439 | 12.561 | 18.405 | 1.00 | 36.88 | C |
| ATOM | 1238 | C | ALA | A | 163 | 7.428 | 13.661 | 18.725 | 1.00 | 41.59 | C |
| ATOM | 1239 | O | ALA | A | 163 | 7.524 | 14.318 | 19.762 | 1.00 | 40.49 | O |
| ATOM | 1240 | CB | ALA | A | 163 | 9.725 | 13.165 | 17.865 | 1.00 | 30.59 | C |
| ATOM | 1241 | N | LEU | A | 164 | 6.460 | 13.858 | 17.833 | 1.00 | 36.52 | N |
| ATOM | 1242 | CA | LEU | A | 164 | 5.423 | 14.863 | 18.052 | 1.00 | 35.71 | C |
| ATOM | 1243 | C | LEU | A | 164 | 4.089 | 14.180 | 18.333 | 1.00 | 36.12 | C |
| ATOM | 1244 | O | LEU | A | 164 | 3.517 | 13.513 | 17.468 | 1.00 | 35.37 | O |
| ATOM | 1245 | CB | LEU | A | 164 | 5.316 | 15.802 | 16.849 | 1.00 | 30.79 | C |
| ATOM | 1246 | CG | LEU | A | 164 | 4.328 | 16.965 | 16.961 | 1.00 | 36.59 | C |
| ATOM | 1247 | CD1 | LEU | A | 164 | 4.537 | 17.756 | 18.256 | 1.00 | 36.35 | C |
| ATOM | 1248 | CD2 | LEU | A | 164 | 4.450 | 17. 875 | 15.752 | 1.00 | 33.18 | C |
| ATOM | 1249 | N | THR | A | 165 | 3.601 | 14.359 | 19.555 | 1.00 | 33.05 | N |
| ATOM | 1250 | CA | THR | A | 165 | 2.442 | 13.623 | 20.048 | 1.00 | 39.48 | C |
| ATOM | 1251 | C | THR | A | 165 | 1.347 | 14.555 | 20.562 | 1.00 | 39.43 | C |
| ATOM | 1252 | O | THR | A | 165 | 0.164 | 14.353 | 20.285 | 1.00 | 39.07 | O |
| ATOM | 1253 | CB | THR | A | 165 | 2.864 | 12.646 | 21.174 | 1.00 | 34.92 | C |
| ATOM | 1254 | CG2 | THR | A | 165 | 1.693 | 12.309 | 22.089 | 1.00 | 52.65 | C |
| ATOM | 1255 | OG1 | THR | A | 165 | 3.367 | 11.442 | 20.588 | 1.00 | 50.23 | O |
| ATOM | 1256 | N | SER | A | 166 | 1.750 | 15.577 | 21.308 | 1.00 | 33.38 | N |
| ATOM | 1257 | CA | SER | A | 166 | 0.804 | 16.495 | 21.930 | 1.00 | 35.48 | C |
| ATOM | 1258 | C | SER | A | 166 | 0.202 | 17.452 | 20.915 | 1.00 | 34.11 | C |

(continued)

| | | | | | | | | | | |
|---|---|---|---|---|---|---|---|---|---|---|
| ATOM | 1259 | O | SER | A | 166 | 0.920 | 18.061 | 20.123 | 1.00 | 37.38 | O |
| ATOM | 1260 | CB | SER | A | 166 | 1.487 | 17.285 | 23.047 | 1.00 | 37.23 | C |
| ATOM | 1261 | OG | SER | A | 166 | 2.094 | 16.406 | 23.982 | 1.00 | 58.12 | O |
| ATOM | 1262 | N | GLY | A | 167 | -1.120 | 17.583 | 20.945 | 1.00 | 36.94 | N |
| ATOM | 1263 | CA | GLY | A | 167 | -1.809 | 18.532 | 20.090 | 1.00 | 35.05 | C |
| ATOM | 1264 | C | GLY | A | 167 | -2.196 | 17.943 | 18.750 | 1.00 | 38.60 | C |
| ATOM | 1265 | O | GLY | A | 167 | -2.701 | 18.650 | 17 . 879 | 1.00 | 38.31 | O |
| ATOM | 1266 | N | VAL | A | 168 | -1.965 | 16.643 | 18.586 | 1.00 | 35.11 | N |
| ATOM | 1267 | CA | VAL | A | 168 | -2.262 | 15.968 | 17.328 | 1.00 | 34.52 | C |
| ATOM | 1268 | C | VAL | A | 168 | -3.690 | 15.438 | 17.297 | 1.00 | 33.72 | C |
| ATOM | 1269 | O | VAL | A | 168 | -4.145 | 14.801 | 18.242 | 1.00 | 33.06 | O |
| ATOM | 1270 | CB | VAL | A | 168 | -1.295 | 14.792 | 17.074 | 1.00 | 34.33 | C |
| ATOM | 1271 | CG1 | VAL | A | 168 | -1.662 | 14.059 | 15.783 | 1.00 | 32.11 | C |
| ATOM | 1272 | CG2 | VAL | A | 168 | 0.141 | 15.285 | 17.027 | 1.00 | 30.55 | C |
| ATOM | 1273 | N | HIS | A | 169 | -4.392 | 15.712 | 16.203 | 1.00 | 34.59 | N |
| ATOM | 1274 | CA | HIS | A | 169 | -5.693 | 15.108 | 15.953 | 1.00 | 33.15 | C |
| ATOM | 1275 | C | HIS | A | 169 | -5.701 | 14.463 | 14.577 | 1.00 | 34.43 | C |
| ATOM | 1276 | O | HIS | A | 169 | -5.556 | 15.147 | 13.561 | 1.00 | 32.61 | O |
| ATOM | 1277 | CB | HIS | A | 169 | -6.822 | 16.140 | 16.039 | 1.00 | 33.41 | C |
| ATOM | 1278 | CG | HIS | A | 169 | -7.036 | 16.702 | 17.409 | 1.00 | 38.03 | C |
| ATOM | 1279 | CD2 | HIS | A | 169 | -6.891 | 17.961 | 17.887 | 1.00 | 35.39 | C |
| ATOM | 1280 | ND1 | HIS | A | 169 | -7.482 | 15.940 | 18.467 | 1.00 | 45.83 | N |
| ATOM | 1281 | CE1 | HIS | A | 169 | -7.592 | 16.702 | 19.541 | 1.00 | 37.15 | C |
| ATOM | 1282 | NE2 | HIS | A | 169 | -7.240 | 17.933 | 19.215 | 1.00 | 39.39 | N |
| ATOM | 1283 | N | THR | A | 170 | -5.857 | 13.145 | 14.551 | 1.00 | 33.36 | N |
| ATOM | 1284 | CA | THR | A | 170 | -6.059 | 12.424 | 13.304 | 1.00 | 31.63 | C |
| ATOM | 1285 | C | THR | A | 170 | -7.525 | 12.030 | 13.217 | 1.00 | 32.95 | C |
| ATOM | 1286 | O | THR | A | 170 | -8.032 | 11.288 | 14.056 | 1.00 | 37.08 | O |
| ATOM | 1287 | CB | THR | A | 170 | -5.168 | 11.178 | 13.200 | 1.00 | 35.35 | C |
| ATOM | 1288 | CG2 | THR | A | 170 | -5.437 | 10.439 | 11.895 | 1.00 | 29.64 | C |
| ATOM | 1289 | OG1 | THR | A | 170 | -3.792 | 11.576 | 13.246 | 1.00 | 35.66 | O |
| ATOM | 1290 | N | PHE | A | 171 | -8.202 | 12.553 | 12.204 | 1.00 | 30.34 | N |
| ATOM | 1291 | CA | PHE | A | 171 | -9.641 | 12.398 | 12.076 | 1.00 | 30.60 | C |
| ATOM | 1292 | C | PHE | A | 171 | -10.036 | 11.082 | 11.422 | 1.00 | 29.79 | C |

(continued)

| | | | | | | | | | | |
|---|---|---|---|---|---|---|---|---|---|---|
| ATOM | 1293 | O | PHE | A | 171 | -9.297 | 10.551 | 10.597 | 1.00 | 31.35 | O |
| ATOM | 1294 | CB | PHE | A | 171 | -10.214 | 13.574 | 11.286 | 1.00 | 23.31 | C |
| ATOM | 1295 | CG | PHE | A | 171 | -10.141 | 14.874 | 12.024 | 1.00 | 25.50 | C |
| ATOM | 1296 | CD2 | PHE | A | 171 | -11.197 | 15.295 | 12.810 | 1.00 | 29.91 | C |
| ATOM | 1297 | CD1 | PHE | A | 171 | -9.004 | 15.662 | 11.957 | 1.00 | 31.55 | C |
| ATOM | 1298 | CE2 | PHE | A | 171 | -11.133 | 16.488 | 13.502 | 1.00 | 30.68 | C |
| ATOM | 1299 | CE1 | PHE | A | 171 | -8.931 | 16.856 | 12.647 | 1.00 | 36.15 | C |
| ATOM | 1300 | CZ | PHE | A | 171 | -10.001 | 17.269 | 13.422 | 1.00 | 30.59 | C |
| ATOM | 1301 | N | PRO | A | 172 | -11.204 | 10.546 | 11.805 | 1.00 | 35.35 | N |
| ATOM | 1302 | CA | PRO | A | 172 | -11.781 | 9.388 | 11.117 | 1.00 | 36.50 | C |
| ATOM | 1303 | C | PRO | A | 172 | -11.928 | 9.670 | 9.627 | 1.00 | 36.86 | C |
| ATOM | 1304 | O | PRO | A | 172 | -12.265 | 10.794 | 9.253 | 1.00 | 33.48 | O |
| ATOM | 1305 | CB | PRO | A | 172 | -13.151 | 9.225 | 11.784 | 1.00 | 32.84 | C |
| ATOM | 1306 | CG | PRO | A | 172 | -12.979 | 9.826 | 13.137 | 1.00 | 36.13 | C |
| ATOM | 1307 | CD | PRO | A | 172 | -12.018 | 10.968 | 12.961 | 1.00 | 33.84 | C |
| ATOM | 1308 | N | ALA | A | 173 | -11.661 | 8.675 | 8.791 | 1.00 | 32.35 | N |
| ATOM | 1309 | CA | ALA | A | 173 | -11.783 | 8.850 | 7.349 | 1.00 | 35.00 | C |
| ATOM | 1310 | C | ALA | A | 173 | -13.237 | 9.021 | 6.930 | 1.00 | 34.52 | C |
| ATOM | 1311 | O | ALA | A | 173 | -14.132 | 8.415 | 7.516 | 1.00 | 37.71 | O |
| ATOM | 1312 | CB | ALA | A | 173 | -11.168 | 7.666 | 6.618 | 1.00 | 34.54 | C |
| ATOM | 1313 | N | VAL | A | 174 | -13.473 | 9.849 | 5.920 | 1.00 | 33.54 | N |
| ATOM | 1314 | CA | VAL | A | 174 | -14.792 | 9.909 | 5.307 | 1.00 | 33.28 | C |
| ATOM | 1315 | C | VAL | A | 174 | -14.737 | 9.250 | 3.939 | 1.00 | 35.09 | C |
| ATOM | 1316 | O | VAL | A | 174 | -13.734 | 9.348 | 3.223 | 1.00 | 34.87 | O |
| ATOM | 1317 | CB | VAL | A | 174 | -15.320 | 11.360 | 5.171 | 1.00 | 32.47 | C |
| ATOM | 1318 | CG1 | VAL | A | 174 | -15.378 | 12.032 | 6.534 | 1.00 | 40.37 | C |
| ATOM | 1319 | CG2 | VAL | A | 174 | -14.469 | 12.168 | 4.196 | 1.00 | 37.12 | C |
| ATOM | 1320 | N | LEU | A | 175 | -15.809 | 8.550 | 3.591 | 1.00 | 40.76 | N |
| ATOM | 1321 | CA | LEU | A | 175 | -15.921 | 7.932 | 2.280 | 1.00 | 38.53 | C |
| ATOM | 1322 | C | LEU | A | 175 | -16.501 | 8.941 | 1.300 | 1.00 | 37.30 | C |
| ATOM | 1323 | O | LEU | A | 175 | -17.605 | 9.447 | 1.494 | 1.00 | 43.25 | O |
| ATOM | 1324 | CB | LEU | A | 175 | -16.788 | 6.669 | 2.344 | 1.00 | 37.54 | C |
| ATOM | 1325 | CG | LEU | A | 175 | -16.905 | 5.864 | 1.047 | 1.00 | 40.58 | C |
| ATOM | 1326 | CD1 | LEU | A | 175 | -15.529 | 5.519 | 0.504 | 1.00 | 37.07 | C |

(continued)

| | | | | | | | | | | | |
|---|---|---|---|---|---|---|---|---|---|---|---|
| ATOM | 1327 | CD2 | LEU | A | 175 | -17.733 | 4.598 | 1.258 | 1.00 | 43.41 | C |
| ATOM | 1328 | N | GLN | A | 176 | -15.744 | 9.239 | 0.252 | 1.00 | 44.15 | N |
| ATOM | 1329 | CA | GLN | A | 176 | -16.163 | 10.211 | -0.746 | 1.00 | 37.45 | C |
| ATOM | 1330 | C | GLN | A | 176 | -17.095 | 9.569 | -1.769 | 1.00 | 44.09 | C |
| ATOM | 1331 | O | GLN | A | 176 | -17.298 | 8.356 | -1.756 | 1.00 | 44.31 | O |
| ATOM | 1332 | CB | GLN | A | 176 | -14.941 | 10.815 | -1.439 | 1.00 | 39.61 | C |
| ATOM | 1333 | CG | GLN | A | 176 | -13.940 | 11.448 | -0.480 | 1.00 | 41.98 | C |
| ATOM | 1334 | CD | GLN | A | 176 | -12.634 | 11.818 | -1.160 | 1.00 | 45.48 | C |
| ATOM | 1335 | NE2 | GLN | A | 176 | -11.917 | 10.813 | -1.655 | 1.00 | 33.19 | N |
| ATOM | 1336 | OE1 | GLN | A | 176 | -12.270 | 12.993 | -1.231 | 1.00 | 52. 08 | O |
| ATOM | 1337 | N | SER | A | 177 | -17.655 | 10.390 | -2.655 | 1.00 | 43.50 | N |
| ATOM | 1338 | CA | SER | A | 177 | -18.571 | 9.913 | -3.690 | 1.00 | 44.39 | C |
| ATOM | 1339 | C | SER | A | 177 | -17.886 | 8.978 | -4.679 | 1.00 | 42.75 | C |
| ATOM | 1340 | O | SER | A | 177 | -18.534 | 8.157 | -5.323 | 1.00 | 48.60 | O |
| ATOM | 1341 | CB | SER | A | 177 | -19.180 | 11.096 | -4.443 | 1.00 | 49.14 | C |
| ATOM | 1342 | OG | SER | A | 177 | -19.961 | 11.895 | -3.575 | 1.00 | 52.77 | O |
| ATOM | 1343 | N | SER | A | 178 | -16.571 | 9.116 | -4.793 | 1.00 | 39.08 | N |
| ATOM | 1344 | CA | SER | A | 178 | -15.780 | 8.312 | -5.714 | 1.00 | 38.13 | C |
| ATOM | 1345 | C | SER | A | 178 | -15.542 | 6.890 | -5.215 | 1.00 | 39.53 | C |
| ATOM | 1346 | O | SER | A | 178 | -14.972 | 6.064 | -5.930 | 1.00 | 46.28 | O |
| ATOM | 1347 | CB | SER | A | 178 | -14.435 | 8.991 | -5.957 | 1.00 | 43.23 | C |
| ATOM | 1348 | OG | SER | A | 178 | -13.792 | 9.255 | -4.720 | 1.00 | 49.71 | O |
| ATOM | 1349 | N | GLY | A | 179 | -15.960 | 6.608 | -3.985 | 1.00 | 41.09 | N |
| ATOM | 1350 | CA | GLY | A | 179 | -15.696 | 5.317 | -3.379 | 1.00 | 35.99 | C |
| ATOM | 1351 | C | GLY | A | 179 | -14.316 | 5.259 | -2.746 | 1.00 | 41.20 | C |
| ATOM | 1352 | O | GLY | A | 179 | -13.882 | 4.210 | -2.266 | 1.00 | 37.34 | O |
| ATOM | 1353 | N | LEU | A | 180 | -13.623 | 6.393 | -2.742 | 1.00 | 37.81 | N |
| ATOM | 1354 | CA | LEU | A | 180 | -12.302 | 6.482 | -2.127 | 1.00 | 39.78 | C |
| ATOM | 1355 | C | LEU | A | 180 | -12.370 | 7.247 | -0.808 | 1.00 | 38.81 | C |
| ATOM | 1356 | O | LEU | A | 180 | -13.165 | 8.179 | -0.659 | 1.00 | 34.97 | O |
| ATOM | 1357 | CB | LEU | A | 180 | -11.310 | 7.152 | -3.082 | 1.00 | 38.31 | C |
| ATOM | 1358 | CG | LEU | A | 180 | -11.181 | 6.507 | -4.470 | 1.00 | 38.11 | C |
| ATOM | 1359 | CD1 | LEU | A | 180 | -10.351 | 7.381 | -5.408 | 1.00 | 33.95 | C |
| ATOM | 1360 | CD2 | LEU | A | 180 | -10.577 | 5.119 | -4.359 | 1.00 | 35.65 | C |

(continued)

| | | | | | | | | | | | |
|---|---|---|---|---|---|---|---|---|---|---|---|
| ATOM | 1361 | N | TYR | A | 181 | -11.534 | 6.849 | 0.147 | 1.00 | 33.32 | N |
| ATOM | 1362 | CA | TYR | A | 181 | -11.492 | 7.494 | 1.457 | 1.00 | 34.27 | C |
| ATOM | 1363 | C | TYR | A | 181 | -10.628 | 8.746 | 1.466 | 1.00 | 36.24 | C |
| ATOM | 1364 | O | TYR | A | 181 | -9.805 | 8.964 | 0.573 | 1.00 | 31.81 | O |
| ATOM | 1365 | CB | TYR | A | 181 | -10.969 | 6.527 | 2.524 | 1.00 | 29.72 | C |
| ATOM | 1366 | CG | TYR | A | 181 | -11.916 | 5.396 | 2.837 | 1.00 | 35.89 | C |
| ATOM | 1367 | CD1 | TYR | A | 181 | -12.985 | 5.582 | 3.707 | 1.00 | 33.01 | C |
| ATOM | 1368 | CD2 | TYR | A | 181 | -11.743 | 4.140 | 2.267 | 1.00 | 31.28 | C |
| ATOM | 1369 | CE1 | TYR | A | 181 | -13.859 | 4.547 | 4.002 | 1.00 | 34.04 | C |
| ATOM | 1370 | CE2 | TYR | A | 181 | -12.614 | 3.095 | 2.560 | 1.00 | 35.99 | C |
| ATOM | 1371 | CZ | TYR | A | 181 | -13.669 | 3.306 | 3.427 | 1.00 | 35.87 | C |
| ATOM | 1372 | OH | TYR | A | 181 | -14.539 | 2.278 | 3.721 | 1.00 | 42.84 | O |
| ATOM | 1373 | N | SER | A | 182 | -10.815 | 9.555 | 2.503 | 1.00 | 33.72 | N |
| ATOM | 1374 | CA | SER | A | 182 | -9.993 | 10.731 | 2.727 | 1.00 | 30.09 | C |
| ATOM | 1375 | C | SER | A | 182 | -9.965 | 11.071 | 4.214 | 1.00 | 35.24 | C |
| ATOM | 1376 | O | SER | A | 182 | -10.975 | 10.941 | 4.907 | 1.00 | 35.29 | O |
| ATOM | 1377 | CB | SER | A | 182 | -10.521 | 11.913 | 1.917 | 1.00 | 35.11 | C |
| ATOM | 1378 | OG | SER | A | 182 | -9.824 | 13.099 | 2.239 | 1.00 | 44.24 | O |
| ATOM | 1379 | N | LEU | A | 183 | -8.809 | 11.488 | 4.713 | 1.00 | 24.60 | N |
| ATOM | 1380 | CA | LEU | A | 183 | -8.745 | 11.992 | 6.077 | 1.00 | 33.42 | C |
| ATOM | 1381 | C | LEU | A | 183 | -7.713 | 13.095 | 6.223 | 1.00 | 28.32 | C |
| ATOM | 1382 | O | LEU | A | 183 | -6.855 | 13.293 | 5.365 | 1.00 | 35.46 | O |
| ATOM | 1383 | CB | LEU | A | 183 | -8.455 | 10.858 | 7.075 | 1.00 | 32.60 | C |
| ATOM | 1384 | CG | LEU | A | 183 | -7.132 | 10.092 | 7.186 | 1.00 | 38.46 | C |
| ATOM | 1385 | CD1 | LEU | A | 183 | -6.003 | 10.910 | 7.815 | 1.00 | 32.89 | C |
| ATOM | 1386 | CD2 | LEU | A | 183 | -7.377 | 8.845 | 8.013 | 1.00 | 35.96 | C |
| ATOM | 1387 | N | SER | A | 184 | -7.801 | 13.812 | 7.329 | 1.00 | 28.07 | N |
| ATOM | 1388 | CA | SER | A | 184 | -6.803 | 14 . 806 | 7.644 | 1.00 | 30.87 | C |
| ATOM | 1389 | C | SER | A | 184 | -6.202 | 14.512 | 9.009 | 1.00 | 36.19 | C |
| ATOM | 1390 | O | SER | A | 184 | -6.846 | 13.908 | 9.869 | 1.00 | 32.31 | O |
| ATOM | 1391 | CB | SER | A | 184 | -7.411 | 16.198 | 7.619 | 1.00 | 27.57 | C |
| ATOM | 1392 | OG | SER | A | 184 | -8.437 | 16.292 | 8.586 | 1.00 | 37.02 | O |
| ATOM | 1393 | N | SER | A | 185 | -4.958 | 14.930 | 9.190 | 1.00 | 29.68 | N |
| ATOM | 1394 | CA | SER | A | 185 | -4.309 | 14.883 | 10.487 | 1.00 | 27.58 | C |

(continued)

| | | | | | | | | | | | |
|---|---|---|---|---|---|---|---|---|---|---|---|
| ATOM | 1395 | C | SER | A | 185 | -3.733 | 16.254 | 10.747 | 1.00 | 29.16 | C |
| ATOM | 1396 | O | SER | A | 185 | -3.023 | 16.799 | 9.908 | 1.00 | 34.94 | O |
| ATOM | 1397 | CB | SER | A | 185 | -3.211 | 13.822 | 10.533 | 1.00 | 29.89 | C |
| ATOM | 1398 | OG | SER | A | 185 | -2.506 | 13.889 | 11.762 | 1.00 | 33.01 | O |
| ATOM | 1399 | N | VAL | A | 186 | -4.051 | 16.825 | 11.898 | 1.00 | 31. 07 | N |
| ATOM | 1400 | CA | VAL | A | 186 | -3.573 | 18.158 | 12.209 | 1.00 | 32.17 | C |
| ATOM | 1401 | C | VAL | A | 186 | -2.863 | 18.166 | 13.545 | 1.00 | 30.53 | C |
| ATOM | 1402 | O | VAL | A | 186 | -3.002 | 17.244 | 14.347 | 1.00 | 29.86 | O |
| ATOM | 1403 | CB | VAL | A | 186 | -4.719 | 19.179 | 12.231 | 1.00 | 30.64 | C |
| ATOM | 1404 | CG1 | VAL | A | 186 | -5.443 | 19.180 | 10.890 | 1.00 | 27.44 | C |
| ATOM | 1405 | CG2 | VAL | A | 186 | -5.684 | 18.868 | 13.369 | 1.00 | 31.93 | C |
| ATOM | 1406 | N | VAL | A | 187 | -2.082 | 19.211 | 13.765 | 1.00 | 28.24 | N |
| ATOM | 1407 | CA | VAL | A | 187 | -1.402 | 19.397 | 15.028 | 1.00 | 29.57 | C |
| ATOM | 1408 | C | VAL | A | 187 | -1.341 | 20.894 | 15.298 | 1.00 | 33.56 | C |
| ATOM | 1409 | O | VAL | A | 187 | -1.156 | 21.689 | 14.377 | 1.00 | 33.65 | O |
| ATOM | 1410 | CB | VAL | A | 187 | 0.013 | 18.764 | 15.013 | 1.00 | 32.42 | C |
| ATOM | 1411 | CG1 | VAL | A | 187 | 0.854 | 19.335 | 13.875 | 1.00 | 35.03 | C |
| ATOM | 1412 | CG2 | VAL | A | 187 | 0.712 | 18.946 | 16.361 | 1.00 | 32.60 | C |
| ATOM | 1413 | N | THR | A | 188 | -1.553 | 21.282 | 16.549 | 1.00 | 31.74 | N |
| ATOM | 1414 | CA | THR | A | 188 | -1.405 | 22.674 | 16.943 | 1.00 | 36.08 | C |
| ATOM | 1415 | C | THR | A | 188 | -0.048 | 22.862 | 17.608 | 1.00 | 40.20 | C |
| ATOM | 1416 | O | THR | A | 188 | 0.332 | 22.092 | 18.488 | 1.00 | 42.98 | O |
| ATOM | 1417 | CB | THR | A | 188 | -2.526 | 23.122 | 17.898 | 1.00 | 35.80 | C |
| ATOM | 1418 | CG2 | THR | A | 188 | -3.810 | 23.379 | 17.124 | 1.00 | 36.31 | C |
| ATOM | 1419 | OG1 | THR | A | 188 | -2.765 | 22.093 | 18.865 | 1.00 | 43.98 | O |
| ATOM | 1420 | N | VAL | A | 189 | 0.685 | 23.878 | 17.166 | 1.00 | 34.48 | N |
| ATOM | 1421 | CA | VAL | A | 189 | 2.034 | 24.144 | 17.655 | 1.00 | 36.93 | C |
| ATOM | 1422 | C | VAL | A | 189 | 2.205 | 25.642 | 17.918 | 1.00 | 45.71 | C |
| ATOM | 1423 | O | VAL | A | 189 | 1.428 | 26.453 | 17.409 | 1.00 | 48.89 | O |
| ATOM | 1424 | CB | VAL | A | 189 | 3.105 | 23.673 | 16.645 | 1.00 | 39.16 | C |
| ATOM | 1425 | CG1 | VAL | A | 189 | 2.983 | 22.178 | 16.378 | 1.00 | 36.29 | C |
| ATOM | 1426 | CG2 | VAL | A | 189 | 2.999 | 24.470 | 15.354 | 1.00 | 30.72 | C |
| ATOM | 1427 | N | PRO | A | 190 | 3.216 | 26.018 | 18.719 | 1.00 | 45.89 | N |
| ATOM | 1428 | CA | PRO | A | 190 | 3.473 | 27.449 | 18.936 | 1.00 | 50.63 | C |

(continued)

| | | | | | | | | | | | |
|---|---|---|---|---|---|---|---|---|---|---|---|
| ATOM | 1429 | C | PRO | A | 190 | 3.852 | 28.173 | 17.644 | 1.00 | 48.93 | C |
| ATOM | 1430 | O | PRO | A | 190 | 4.637 | 27.643 | 16.858 | 1.00 | 44.98 | O |
| ATOM | 1431 | CB | PRO | A | 190 | 4.645 | 27.450 | 19.924 | 1.00 | 48.82 | C |
| ATOM | 1432 | CG | PRO | A | 190 | 4.553 | 26.131 | 20.620 | 1.00 | 46.27 | C |
| ATOM | 1433 | CD | PRO | A | 190 | 4.067 | 25.170 | 19.574 | 1.00 | 42.43 | C |
| ATOM | 1434 | N | SER | A | 191 | 3.297 | 29.364 | 17.433 | 1.00 | 48.29 | N |
| ATOM | 1435 | CA | SER | A | 191 | 3.581 | 30.147 | 16.231 | 1.00 | 50.08 | C |
| ATOM | 1436 | C | SER | A | 191 | 5.068 | 30.440 | 16.074 | 1.00 | 50.39 | C |
| ATOM | 1437 | O | SER | A | 191 | 5.585 | 30.495 | 14.959 | 1.00 | 44.82 | O |
| ATOM | 1438 | CB | SER | A | 191 | 2.802 | 31.463 | 16.249 | 1.00 | 57.90 | C |
| ATOM | 1439 | OG | SER | A | 191 | 1.415 | 31.239 | 16.078 | 1.00 | 74.28 | O |
| ATOM | 1440 | N | SER | A | 192 | 5.753 | 30.618 | 17.200 | 1.00 | 46.04 | N |
| ATOM | 1441 | CA | SER | A | 192 | 7.169 | 30.965 | 17.193 | 1.00 | 48.53 | C |
| ATOM | 1442 | C | SER | A | 192 | 8.062 | 29.808 | 16.739 | 1.00 | 53.82 | C |
| ATOM | 1443 | O | SER | A | 192 | 9.241 | 30.009 | 16.445 | 1.00 | 58.88 | O |
| ATOM | 1444 | CB | SER | A | 192 | 7.598 | 31.447 | 18.582 | 1.00 | 52.00 | C |
| ATOM | 1445 | OG | SER | A | 192 | 7.184 | 30.541 | 19.592 | 1.00 | 49.57 | O |
| ATOM | 1446 | N | SER | A | 193 | 7.507 | 28.601 | 16.671 | 1.00 | 54.17 | N |
| ATOM | 1447 | CA | SER | A | 193 | 8.299 | 27.443 | 16.260 | 1.00 | 50.73 | C |
| ATOM | 1448 | C | SER | A | 193 | 8.291 | 27.260 | 14.741 | 1.00 | 50.16 | C |
| ATOM | 1449 | O | SER | A | 193 | 9.128 | 26.541 | 14.197 | 1.00 | 53.54 | O |
| ATOM | 1450 | CB | SER | A | 193 | 7.795 | 26.168 | 16.944 | 1.00 | 44.93 | C |
| ATOM | 1451 | OG | SER | A | 193 | 6.626 | 25.670 | 16.317 | 1.00 | 46.77 | O |
| ATOM | 1452 | N | LEU | A | 194 | 7.349 | 27.907 | 14.060 | 1.00 | 45.24 | N |
| ATOM | 1453 | CA | LEU | A | 194 | 7.292 | 27.842 | 12.602 | 1.00 | 46.01 | C |
| ATOM | 1454 | C | LEU | A | 194 | 8.520 | 28.519 | 12.007 | 1.00 | 55.53 | C |
| ATOM | 1455 | O | LEU | A | 194 | 8.857 | 29.643 | 12.376 | 1.00 | 62.86 | O |
| ATOM | 1456 | CB | LEU | A | 194 | 6.021 | 28.502 | 12.073 | 1.00 | 39.49 | C |
| ATOM | 1457 | CG | LEU | A | 194 | 4.678 | 28.001 | 12.599 | 1.00 | 46.53 | C |
| ATOM | 1458 | CD1 | LEU | A | 194 | 3.550 | 28.840 | 12.024 | 1.00 | 43.35 | C |
| ATOM | 1459 | CD2 | LEU | A | 194 | 4.476 | 26.535 | 12.261 | 1.00 | 44.15 | C |
| ATOM | 1460 | N | GLY | A | 195 | 9.195 | 27.832 | 11.091 | 1.00 | 59.38 | N |
| ATOM | 1461 | CA | GLY | A | 195 | 10.422 | 28.355 | 10.519 | 1.00 | 62.95 | C |
| ATOM | 1462 | C | GLY | A | 195 | 11.653 | 27.847 | 11.246 | 1.00 | 59.76 | C |

(continued)

| | | | | | | | | | | | |
|---|---|---|---|---|---|---|---|---|---|---|---|
| ATOM | 1463 | O | GLY | A | 195 | 12.729 | 27.733 | 10.660 | 1.00 | 64.18 | O |
| ATOM | 1464 | N | THR | A | 196 | 11.493 | 27.540 | 12.529 | 1.00 | 59.45 | N |
| ATOM | 1465 | CA | THR | A | 196 | 12.570 | 26.958 | 13.320 | 1.00 | 58.51 | C |
| ATOM | 1466 | C | THR | A | 196 | 12.448 | 25.434 | 13.387 | 1.00 | 60.43 | C |
| ATOM | 1467 | O | THR | A | 196 | 13.414 | 24.713 | 13.138 | 1.00 | 61.86 | O |
| ATOM | 1468 | CB | THR | A | 196 | 12.583 | 27.522 | 14.754 | 1.00 | 61.27 | C |
| ATOM | 1469 | CG2 | THR | A | 196 | 13.706 | 26.891 | 15.568 | 1.00 | 57.43 | C |
| ATOM | 1470 | OG1 | THR | A | 196 | 12.767 | 28.942 | 14.708 | 1.00 | 68.83 | O |
| ATOM | 1471 | N | GLN | A | 197 | 11.254 | 24.954 | 13.725 | 1.00 | 49.07 | N |
| ATOM | 1472 | CA | GLN | A | 197 | 11.009 | 23.525 | 13.894 | 1.00 | 41.26 | C |
| ATOM | 1473 | C | GLN | A | 197 | 10.473 | 22.872 | 12.619 | 1.00 | 42.46 | C |
| ATOM | 1474 | O | GLN | A | 197 | 9.577 | 23.400 | 11.963 | 1.00 | 40.55 | O |
| ATOM | 1475 | CB | GLN | A | 197 | 10.030 | 23.290 | 15.049 | 1.00 | 44.12 | C |
| ATOM | 1476 | CG | GLN | A | 197 | 9.629 | 21.833 | 15.242 | 1.00 | 47.27 | C |
| ATOM | 1477 | CD | GLN | A | 197 | 10.769 | 20.968 | 15.751 | 1.00 | 57.04 | C |
| ATOM | 1478 | NE2 | GLN | A | 197 | 10.939 | 19.790 | 15.150 | 1.00 | 43.31 | N |
| ATOM | 1479 | OE1 | GLN | A | 197 | 11.490 | 21.354 | 16.673 | 1.00 | 51.73 | O |
| ATOM | 1480 | N | THR | A | 198 | 11.034 | 21.719 | 12.273 | 1.00 | 41.32 | N |
| ATOM | 1481 | CA | THR | A | 198 | 10.577 | 20.958 | 11.121 | 1.00 | 35.03 | C |
| ATOM | 1482 | C | THR | A | 198 | 9.365 | 20.113 | 11.502 | 1.00 | 36.05 | C |
| ATOM | 1483 | O | THR | A | 198 | 9.359 | 19.468 | 12.550 | 1.00 | 35.98 | O |
| ATOM | 1484 | CB | THR | A | 198 | 11.705 | 20.058 | 10.565 | 1.00 | 33.49 | C |
| ATOM | 1485 | CG2 | THR | A | 198 | 11.153 | 19.010 | 9.620 | 1.00 | 33.33 | C |
| ATOM | 1486 | OG1 | THR | A | 198 | 12.644 | 20.870 | 9.848 | 1.00 | 47.14 | O |
| ATOM | 1487 | N | TYR | A | 199 | 8.334 | 20.141 | 10.660 | 1.00 | 34.82 | N |
| ATOM | 1488 | CA | TYR | A | 199 | 7.135 | 19.338 | 10.882 | 1.00 | 33.85 | C |
| ATOM | 1489 | C | TYR | A | 199 | 6.877 | 18.409 | 9.704 | 1.00 | 31.69 | C |
| ATOM | 1490 | O | TYR | A | 199 | 6.644 | 18.859 | 8.582 | 1.00 | 34.22 | O |
| ATOM | 1491 | CB | TYR | A | 199 | 5.924 | 20.238 | 11.129 | 1.00 | 28.42 | c |
| ATOM | 1492 | CG | TYR | A | 199 | 6.043 | 21.030 | 12.407 | 1.00 | 33.72 | c |
| ATOM | 1493 | CD1 | TYR | A | 199 | 5.935 | 20.405 | 13.640 | 1.00 | 27.74 | c |
| ATOM | 1494 | CD2 | TYR | A | 199 | 6.288 | 22.398 | 12.385 | 1.00 | 27.12 | c |
| ATOM | 1495 | CE1 | TYR | A | 199 | 6.058 | 21.120 | 14.822 | 1.00 | 37.15 | c |
| ATOM | 1496 | CE2 | TYR | A | 199 | 6.412 | 23.122 | 13.561 | 1.00 | 35.98 | c |

(continued)

| | | | | | | | | | | | |
|---|---|---|---|---|---|---|---|---|---|---|---|
| ATOM | 1497 | CZ | TYR | A | 199 | 6.294 | 22.477 | 14.776 | 1.00 | 33.49 | c |
| ATOM | 1498 | OH | TYR | A | 199 | 6.414 | 23.184 | 15.948 | 1.00 | 40.19 | O |
| ATOM | 1499 | N | ILE | A | 200 | 6.935 | 17.108 | 9.969 | 1.00 | 30.26 | N |
| ATOM | 1500 | CA | ILE | A | 200 | 6.768 | 16.097 | 8.933 | 1.00 | 31.27 | C |
| ATOM | 1501 | C | ILE | A | 200 | 5.710 | 15.089 | 9.347 | 1.00 | 33.53 | C |
| ATOM | 1502 | O | ILE | A | 200 | 5.768 | 14.546 | 10.452 | 1.00 | 32.98 | O |
| ATOM | 1503 | CB | ILE | A | 200 | 8.093 | 15.352 | 8.644 | 1.00 | 32.27 | C |
| ATOM | 1504 | CG1 | ILE | A | 200 | 9.164 | 16.333 | 8.160 | 1.00 | 37.70 | C |
| ATOM | 1505 | CG2 | ILE | A | 200 | 7.880 | 14.236 | 7.619 | 1.00 | 26.84 | C |
| ATOM | 1506 | CD1 | ILE | A | 200 | 10.495 | 15.689 | 7.860 | 1.00 | 36.87 | C |
| ATOM | 1507 | N | CYS | A | 201 | 4.739 | 14 . 842 | 8.470 | 1.00 | 28.73 | N |
| ATOM | 1508 | CA | CYS | A | 201 | 3.747 | 13.812 | 8.746 | 1.00 | 31.40 | C |
| ATOM | 1509 | C | CYS | A | 201 | 4.129 | 12.523 | 8.032 | 1.00 | 31.76 | C |
| ATOM | 1510 | O | CYS | A | 201 | 4.442 | 12.518 | 6.837 | 1.00 | 30.36 | O |
| ATOM | 1511 | CB | CYS | A | 201 | 2.338 | 14.264 | 8.341 | 1.00 | 29.40 | C |
| ATOM | 1512 | SG | CYS | A | 201 | 1.974 | 14.198 | 6.581 | 1.00 | 46.24 | S |
| ATOM | 1513 | N | ASN | A | 202 | 4.123 | 11.434 | 8.788 | 1.00 | 32.10 | N |
| ATOM | 1514 | CA | ASN | A | 202 | 4.479 | 10.126 | 8.262 | 1.00 | 32.09 | C |
| ATOM | 1515 | C | ASN | A | 202 | 3.216 | 9.332 | 7.990 | 1.00 | 30.31 | C |
| ATOM | 1516 | O | ASN | A | 202 | 2.476 | 8.990 | 8.908 | 1.00 | 34.67 | O |
| ATOM | 1517 | CB | ASN | A | 202 | 5.384 | 9.380 | 9.243 | 1.00 | 31.62 | C |
| ATOM | 1518 | CG | ASN | A | 202 | 6.445 | 10.276 | 9.849 | 1.00 | 34.57 | C |
| ATOM | 1519 | ND2 | ASN | A | 202 | 7.425 | 10.661 | 9.041 | 1.00 | 31.78 | N |
| ATOM | 1520 | OD1 | ASN | A | 202 | 6.382 | 10.621 | 11.030 | 1.00 | 37.73 | O |
| ATOM | 1521 | N | VAL | A | 203 | 2.958 | 9.057 | 6.722 | 1.00 | 31.29 | N |
| ATOM | 1522 | CA | VAL | A | 203 | 1.730 | 8.384 | 6.341 | 1.00 | 29.99 | C |
| ATOM | 1523 | C | VAL | A | 203 | 2.037 | 6.982 | 5.860 | 1.00 | 29.95 | C |
| ATOM | 1524 | O | VAL | A | 203 | 2.962 | 6.775 | 5.079 | 1.00 | 32.94 | O |
| ATOM | 1525 | CB | VAL | A | 203 | 0.981 | 9.163 | 5.243 | 1.00 | 35.04 | C |
| ATOM | 1526 | CG1 | VAL | A | 203 | -0.314 | 8.459 | 4.876 | 1.00 | 28.65 | C |
| ATOM | 1527 | CG2 | VAL | A | 203 | 0.710 | 10.592 | 5.707 | 1.00 | 26.66 | C |
| ATOM | 1528 | N | ASN | A | 204 | 1.264 | 6.019 | 6.345 | 1.00 | 29.35 | N |
| ATOM | 1529 | CA | ASN | A | 204 | 1.449 | 4.631 | 5.961 | 1.00 | 31.31 | C |
| ATOM | 1530 | C | ASN | A | 204 | 0.127 | 4.003 | 5.525 | 1.00 | 33.24 | C |

(continued)

| | | | | | | | | | | | |
|---|---|---|---|---|---|---|---|---|---|---|---|
| ATOM | 1531 | O | ASN | A | 204 | -0.888 | 4.112 | 6.214 | 1.00 | 34.37 | O |
| ATOM | 1532 | CB | ASN | A | 204 | 2.073 | 3.843 | 7.117 | 1.00 | 37.45 | C |
| ATOM | 1533 | CG | ASN | A | 204 | 2.259 | 2.373 | 6.789 | 1.00 | 54.72 | C |
| ATOM | 1534 | ND2 | ASN | A | 204 | 3.039 | 2.091 | 5.754 | 1.00 | 50.83 | N |
| ATOM | 1535 | OD1 | ASN | A | 204 | 1.698 | 1.501 | 7.455 | 1.00 | 73.44 | O |
| ATOM | 1536 | N | HIS | A | 205 | 0.141 | 3.373 | 4.358 | 1.00 | 31.18 | N |
| ATOM | 1537 | CA | HIS | A | 205 | -1.028 | 2.675 | 3.844 | 1.00 | 37.56 | C |
| ATOM | 1538 | C | HIS | A | 205 | -0.605 | 1.286 | 3.413 | 1.00 | 34.89 | C |
| ATOM | 1539 | O | HIS | A | 205 | -0.256 | 1.068 | 2.253 | 1.00 | 36.52 | O |
| ATOM | 1540 | CB | HIS | A | 205 | -1.658 | 3.433 | 2.675 | 1.00 | 27.77 | C |
| ATOM | 1541 | CG | HIS | A | 205 | -2.917 | 2.803 | 2.155 | 1.00 | 35.56 | C |
| ATOM | 1542 | CD2 | HIS | A | 205 | -4.104 | 2.562 | 2.753 | 1.00 | 33.13 | C |
| ATOM | 1543 | ND1 | HIS | A | 205 | -3.037 | 2.344 | 0.859 | 1.00 | 35.44 | N |
| ATOM | 1544 | CE1 | HIS | A | 205 | -4.248 | 1.846 | 0.686 | 1.00 | 33.94 | C |
| ATOM | 1545 | NE2 | HIS | A | 205 | -4.919 | 1.967 | 1.815 | 1.00 | 29.56 | N |
| ATOM | 1546 | N | LYS | A | 206 | -0.623 | 0.353 | 4.358 | 1.00 | 34.52 | N |
| ATOM | 1547 | CA | LYS | A | 206 | -0.155 | -1. 008 | 4.104 | 1.00 | 41.43 | C |
| ATOM | 1548 | C | LYS | A | 206 | -0.833 | -1.754 | 2.942 | 1.00 | 35.74 | C |
| ATOM | 1549 | O | LYS | A | 206 | -0.155 | -2.493 | 2.231 | 1.00 | 39.67 | O |
| ATOM | 1550 | CB | LYS | A | 206 | -0.277 | -1.846 | 5.379 | 1.00 | 47.00 | C |
| ATOM | 1551 | CG | LYS | A | 206 | 0.830 | -1.575 | 6.386 | 1.00 | 57.13 | C |
| ATOM | 1552 | CD | LYS | A | 206 | 0.895 | -2.659 | 7.452 | 1.00 | 81.12 | C |
| ATOM | 1553 | CE | LYS | A | 206 | 2.103 | -2.473 | 8.361 | 1.00 | 81.79 | C |
| ATOM | 1554 | NZ | LYS | A | 206 | 2.106 | -1.138 | 9.024 | 1.00 | 63.36 | N |
| ATOM | 1555 | N | PRO | A | 207 | -2.158 | -1.589 | 2.748 | 1.00 | 37.38 | N |
| ATOM | 1556 | CA | PRO | A | 207 | -2.759 | -2.328 | 1.624 | 1.00 | 32.27 | C |
| ATOM | 1557 | C | PRO | A | 207 | -2.137 | -2.030 | 0.252 | 1.00 | 38.84 | C |
| ATOM | 1558 | O | PRO | A | 207 | -2.064 | -2.930 | -0.578 | 1.00 | 37.23 | O |
| ATOM | 1559 | CB | PRO | A | 207 | -4.218 | -1.876 | 1.655 | 1.00 | 34.91 | C |
| ATOM | 1560 | CG | PRO | A | 207 | -4.471 | -1.559 | 3.085 | 1.00 | 40.39 | C |
| ATOM | 1561 | CD | PRO | A | 207 | -3.192 | -0.955 | 3.590 | 1.00 | 32.89 | C |
| ATOM | 1562 | N | SER | A | 208 | -1.696 | -0.797 | 0.020 | 1.00 | 38.69 | N |
| ATOM | 1563 | CA | SER | A | 208 | -1.064 | -0.446 | -1.250 | 1.00 | 34.52 | C |
| ATOM | 1564 | C | SER | A | 208 | 0.454 | -0.414 | -1.115 | 1.00 | 40.84 | C |

(continued)

| | | | | | | | | | | | |
|---|---|---|---|---|---|---|---|---|---|---|---|
| ATOM | 1565 | O | SER | A | 208 | 1.157 | -0.044 | -2.056 | 1.00 | 41.36 | O |
| ATOM | 1566 | CB | SER | A | 208 | -1.565 | 0.911 | -1.751 | 1.00 | 37.36 | C |
| ATOM | 1567 | OG | SER | A | 208 | -1.111 | 1.961 | -0.912 | 1.00 | 32.83 | O |
| ATOM | 1568 | N | ASN | A | 209 | 0.942 | -0.792 | 0.065 | 1.00 | 38.90 | N |
| ATOM | 1569 | CA | ASN | A | 209 | 2.367 | -0.729 | 0.389 | 1.00 | 42.99 | C |
| ATOM | 1570 | C | ASN | A | 209 | 2.941 | 0.680 | 0.177 | 1.00 | 43.61 | C |
| ATOM | 1571 | O | ASN | A | 209 | 4.051 | 0.841 | -0.327 | 1.00 | 47.92 | O |
| ATOM | 1572 | CB | ASN | A | 209 | 3.147 | -1.756 | -0.441 | 1.00 | 48.09 | C |
| ATOM | 1573 | CG | ASN | A | 209 | 4.502 | -2.086 | 0.159 | 1.00 | 71.77 | C |
| ATOM | 1574 | ND2 | ASN | A | 209 | 5.502 | -2.260 | -0.700 | 1.00 | 76.92 | N |
| ATOM | 1575 | OD1 | ASN | A | 209 | 4.649 | -2.177 | 1.379 | 1.00 | 76.40 | O |
| ATOM | 1576 | N | THR | A | 210 | 2.174 | 1.692 | 0.575 | 1.00 | 40.46 | N |
| ATOM | 1577 | CA | THR | A | 210 | 2.546 | 3.090 | 0.371 | 1.00 | 38.21 | C |
| ATOM | 1578 | C | THR | A | 210 | 3.020 | 3.766 | 1.656 | 1.00 | 38.99 | C |
| ATOM | 1579 | O | THR | A | 210 | 2.320 | 3.746 | 2.670 | 1.00 | 38.40 | O |
| ATOM | 1580 | CB | THR | A | 210 | 1.361 | 3.901 | -0.200 | 1.00 | 38.96 | C |
| ATOM | 1581 | CG2 | THR | A | 210 | 1.720 | 5.376 | -0.316 | 1.00 | 37.56 | C |
| ATOM | 1582 | OG1 | THR | A | 210 | 1.006 | 3.395 | -1.494 | 1.00 | 42.32 | O |
| ATOM | 1583 | N | LYS | A | 211 | 4.214 | 4.353 | 1.607 | 1.00 | 32.28 | N |
| ATOM | 1584 | CA | LYS | A | 211 | 4.716 | 5.195 | 2.693 | 1.00 | 35.66 | C |
| ATOM | 1585 | C | LYS | A | 211 | 5.072 | 6.573 | 2.155 | 1.00 | 33.89 | C |
| ATOM | 1586 | O | LYS | A | 211 | 5.786 | 6.698 | 1.158 | 1.00 | 32.27 | O |
| ATOM | 1587 | CB | LYS | A | 211 | 5.940 | 4.569 | 3.370 | 1.00 | 36.77 | C |
| ATOM | 1588 | CG | LYS | A | 211 | 5.624 | 3.421 | 4.316 | 1.00 | 45.64 | C |
| ATOM | 1589 | CD | LYS | A | 211 | 6.864 | 2.974 | 5.086 | 1.00 | 52.89 | C |
| ATOM | 1590 | CE | LYS | A | 211 | 7.970 | 2.513 | 4.143 | 1.00 | 58.66 | C |
| ATOM | 1591 | NZ | LYS | A | 211 | 9.223 | 2.152 | 4.870 | 1.00 | 61. 89 | N |
| ATOM | 1592 | N | VAL | A | 212 | 4.564 | 7.608 | 2.811 | 1.00 | 27.70 | N |
| ATOM | 1593 | CA | VAL | A | 212 | 4.861 | 8.978 | 2.416 | 1.00 | 34.14 | C |
| ATOM | 1594 | C | VAL | A | 212 | 5.294 | 9.800 | 3.623 | 1.00 | 33.04 | C |
| ATOM | 1595 | O | VAL | A | 212 | 4.657 | 9.748 | 4.677 | 1.00 | 34.67 | O |
| ATOM | 1596 | CB | VAL | A | 212 | 3.640 | 9.659 | 1.741 | 1.00 | 28.22 | C |
| ATOM | 1597 | CG1 | VAL | A | 212 | 3.944 | 11.111 | 1.424 | 1.00 | 26.60 | C |
| ATOM | 1598 | CG2 | VAL | A | 212 | 3.236 | 8.911 | 0.475 | 1.00 | 33.62 | C |

(continued)

| | | | | | | | | | | | |
|---|---|---|---|---|---|---|---|---|---|---|---|
| ATOM | 1599 | N | ASP | A | 213 | 6.387 | 10.544 | 3.469 | 1.00 | 31.96 | N |
| ATOM | 1600 | CA | ASP | A | 213 | 6.802 | 11.535 | 4.460 | 1.00 | 31.89 | C |
| ATOM | 1601 | C | ASP | A | 213 | 6.622 | 12.935 | 3.886 | 1.00 | 34.24 | C |
| ATOM | 1602 | O | ASP | A | 213 | 7.286 | 13.306 | 2.918 | 1.00 | 32.65 | O |
| ATOM | 1603 | CB | ASP | A | 213 | 8.258 | 11.323 | 4.883 | 1.00 | 35.66 | C |
| ATOM | 1604 | CG | ASP | A | 213 | 8.479 | 9.985 | 5.572 | 1.00 | 47.44 | C |
| ATOM | 1605 | OD1 | ASP | A | 213 | 7.659 | 9.616 | 6.445 | 1.00 | 42.96 | O |
| ATOM | 1606 | OD2 | ASP | A | 213 | 9.475 | 9.302 | 5.238 | 1.00 | 43.88 | O |
| ATOM | 1607 | N | LYS | A | 214 | 5.728 | 13.708 | 4.492 | 1.00 | 33.17 | N |
| ATOM | 1608 | CA | LYS | A | 214 | 5.361 | 15.016 | 3.967 | 1.00 | 30.01 | C |
| ATOM | 1609 | C | LYS | A | 214 | 5.791 | 16.138 | 4.906 | 1.00 | 30.73 | C |
| ATOM | 1610 | O | LYS | A | 214 | 5.254 | 16.286 | 6.004 | 1.00 | 29.64 | O |
| ATOM | 1611 | CB | LYS | A | 214 | 3.849 | 15.082 | 3.727 | 1.00 | 28.75 | C |
| ATOM | 1612 | CG | LYS | A | 214 | 3.356 | 16.407 | 3.174 | 1.00 | 34.59 | C |
| ATOM | 1613 | CD | LYS | A | 214 | 3.879 | 16.624 | 1.767 | 1.00 | 33.64 | C |
| ATOM | 1614 | CE | LYS | A | 214 | 3.468 | 17.978 | 1.222 | 1.00 | 44.70 | C |
| ATOM | 1615 | NZ | LYS | A | 214 | 4.102 | 18.251 | -0.100 | 1.00 | 46.18 | N |
| ATOM | 1616 | N | LYS | A | 215 | 6.760 | 16.932 | 4.469 | 1.00 | 31.30 | N |
| ATOM | 1617 | CA | LYS | A | 215 | 7.181 | 18.086 | 5.247 | 1.00 | 29.47 | C |
| ATOM | 1618 | C | LYS | A | 215 | 6.197 | 19.230 | 5.031 | 1.00 | 34.25 | C |
| ATOM | 1619 | O | LYS | A | 215 | 5.887 | 19.587 | 3.895 | 1.00 | 30.95 | O |
| ATOM | 1620 | CB | LYS | A | 215 | 8.597 | 18.521 | 4.870 | 1.00 | 32.87 | C |
| ATOM | 1621 | CG | LYS | A | 215 | 9.076 | 19.731 | 5.657 | 1.00 | 32.92 | C |
| ATOM | 1622 | CD | LYS | A | 215 | 10.523 | 20.071 | 5.351 | 1.00 | 42.60 | C |
| ATOM | 1623 | CE | LYS | A | 215 | 10.950 | 21.339 | 6.087 | 1.00 | 41.79 | C |
| ATOM | 1624 | NZ | LYS | A | 215 | 12.411 | 21.587 | 5.969 | 1.00 | 52.69 | N |
| ATOM | 1625 | N | VAL | A | 216 | 5.697 | 19.798 | 6.121 | 1.00 | 27.91 | N |
| ATOM | 1626 | CA | VAL | A | 216 | 4.736 | 20.883 | 6.011 | 1.00 | 31.50 | C |
| ATOM | 1627 | C | VAL | A | 216 | 5.346 | 22.189 | 6.512 | 1.00 | 35.76 | C |
| ATOM | 1628 | O | VAL | A | 216 | 5.803 | 22.282 | 7.654 | 1.00 | 31.10 | O |
| ATOM | 1629 | CB | VAL | A | 216 | 3.443 | 20.567 | 6.782 | 1.00 | 32.04 | C |
| ATOM | 1630 | CG1 | VAL | A | 216 | 2.447 | 21.716 | 6.650 | 1.00 | 29.56 | C |
| ATOM | 1631 | CG2 | VAL | A | 216 | 2.833 | 19.265 | 6.271 | 1.00 | 28.39 | C |
| ATOM | 1632 | N | GLU | A | 217 | 5.357 | 23.187 | 5.634 | 1.00 | 39.92 | N |

(continued)

| | | | | | | | | | | |
|---|---|---|---|---|---|---|---|---|---|---|
| ATOM | 1633 | CA | GLU | A | 217 | 5.937 | 24.497 | 5.924 | 1.00 | 46.47 | C |
| ATOM | 1634 | C | GLU | A | 217 | 4.917 | 25.604 | 5.714 | 1.00 | 44 . 08 | C |
| ATOM | 1635 | O | GLU | A | 217 | 3.980 | 25.443 | 4.933 | 1.00 | 41.16 | O |
| ATOM | 1636 | CB | GLU | A | 217 | 7.149 | 24.764 | 5.026 | 1.00 | 44.32 | C |
| ATOM | 1637 | CG | GLU | A | 217 | 8.396 | 23.976 | 5.365 | 1.00 | 58.93 | C |
| ATOM | 1638 | CD | GLU | A | 217 | 9.501 | 24.188 | 4.344 | 1.00 | 64.35 | C |
| ATOM | 1639 | OE1 | GLU | A | 217 | 10.678 | 24.300 | 4.750 | 1.00 | 75.54 | O |
| ATOM | 1640 | OE2 | GLU | A | 217 | 9.192 | 24.239 | 3.135 | 1.00 | 64.80 | O |
| ATOM | 1641 | N | PRO | A | 218 | 5.099 | 26.740 | 6.406 | 1.00 | 48.25 | N |
| ATOM | 1642 | CA | PRO | A | 218 | 4.315 | 27.925 | 6.039 | 1.00 | 50.69 | C |
| ATOM | 1643 | C | PRO | A | 218 | 4.667 | 28.339 | 4.615 | 1.00 | 57.06 | C |
| ATOM | 1644 | O | PRO | A | 218 | 5.822 | 28.177 | 4.218 | 1.00 | 55.63 | O |
| ATOM | 1645 | CB | PRO | A | 218 | 4.757 | 28.979 | 7.060 | 1.00 | 50.18 | C |
| ATOM | 1646 | CG | PRO | A | 218 | 6.087 | 28.500 | 7.561 | 1.00 | 50.76 | C |
| ATOM | 1647 | CD | PRO | A | 218 | 6.027 | 27.005 | 7.521 | 1.00 | 42.82 | C |
| ATOM | 1648 | N | LYS | A | 219 | 3.703 | 28.834 | 3.846 | 1.00 | 55.24 | N |
| ATOM | 1649 | CA | LYS | A | 219 | 3.998 | 29.214 | 2.468 | 1.00 | 72.58 | C |
| ATOM | 1650 | C | LYS | A | 219 | 4.426 | 30.679 | 2.392 | 1.00 | 77.04 | C |
| ATOM | 1651 | O | LYS | A | 219 | 3.946 | 31.522 | 3.156 | 1.00 | 71.74 | O |
| ATOM | 1652 | CB | LYS | A | 219 | 2.796 | 28.952 | 1.553 | 1.00 | 66.67 | C |
| ATOM | 1653 | CG | LYS | A | 219 | 3.147 | 29.002 | 0.068 | 1.00 | 73.26 | C |
| ATOM | 1654 | CD | LYS | A | 219 | 2.190 | 28.182 | -0.790 | 1.00 | 78.60 | C |
| ATOM | 1655 | CE | LYS | A | 219 | 0.862 | 28.890 | -1.001 | 1.00 | 76.20 | C |
| ATOM | 1656 | NZ | LYS | A | 219 | 0.010 | 28.168 | -1.992 | 1.00 | 70.66 | N |
| ATOM | 1657 | N | SER | A | 220 | 5.346 | 30.969 | 1.477 | 1.00 | 78.80 | N |
| ATOM | 1658 | CA | SER | A | 220 | 5.870 | 32.319 | 1.306 | 1.00 | 86.14 | C |
| ATOM | 1659 | C | SER | A | 220 | 5.245 | 33.004 | 0.096 | 1.00 | 76.66 | C |
| ATOM | 1660 | O | SER | A | 220 | 4.778 | 32.339 | -0.829 | 1.00 | 76.17 | O |
| ATOM | 1661 | CB | SER | A | 220 | 7.394 | 32.284 | 1.164 | 1.00 | 88.61 | C |
| ATOM | 1662 | OG | SER | A | 220 | 7.787 | 31.375 | 0.150 | 1.00 | 79.58 | O |
| TER | | | | | | | | | | | |
| ATOM | 1663 | N | ASP | B | 1 | -30.071 | -19.770 | 21.044 | 1.00 | 55.86 | N |
| ATOM | 1664 | CA | ASP | B | 1 | -28.959 | -18.907 | 21.429 | 1.00 | 51.10 | C |
| ATOM | 1665 | C | ASP | B | 1 | -29.427 | -17.792 | 22.359 | 1.00 | 49.42 | C |

(continued)

| | | | | | | | | | | | |
|---|---|---|---|---|---|---|---|---|---|---|---|
| ATOM | 1666 | O | ASP | B | 1 | -30.475 | -17.185 | 22.139 | 1.00 | 43.10 | O |
| ATOM | 1667 | CB | ASP | B | 1 | -28.287 | -18.316 | 20.190 | 1.00 | 44.13 | C |
| ATOM | 1668 | CG | ASP | B | 1 | -27.652 | -19.377 | 19.308 | 1.00 | 61.47 | C |
| ATOM | 1669 | OD2 | ASP | B | 1 | -26.859 | -19.009 | 18.415 | 1.00 | 60.15 | O |
| ATOM | 1670 | OD1 | ASP | B | 1 | -27.951 | -20.577 | 19.503 | 1.00 | 63.76 | O |
| ATOM | 1671 | N | ILE | B | 2 | -28.649 | -17.533 | 23.404 | 1.00 | 43.87 | N |
| ATOM | 1672 | CA | ILE | B | 2 | -29.004 | -16.506 | 24.372 | 1.00 | 42.91 | C |
| ATOM | 1673 | C | ILE | B | 2 | -28.769 | -15.115 | 23.794 | 1.00 | 45.05 | C |
| ATOM | 1674 | O | ILE | B | 2 | -27.663 | -14.787 | 23.359 | 1.00 | 46.40 | O |
| ATOM | 1675 | CB | ILE | B | 2 | -28.207 | -16.666 | 25.675 | 1.00 | 39.78 | C |
| ATOM | 1676 | CG1 | ILE | B | 2 | -28.476 | -18.041 | 26.288 | 1.00 | 37.36 | C |
| ATOM | 1677 | CG2 | ILE | B | 2 | -28.563 | -15.558 | 26.659 | 1.00 | 35.94 | C |
| ATOM | 1678 | CD1 | ILE | B | 2 | -27.665 | -18.321 | 27.537 | 1.00 | 32.11 | C |
| ATOM | 1679 | N | VAL | B | 3 | -29.824 | -14.306 | 23.783 | 1.00 | 41.92 | N |
| ATOM | 1680 | CA | VAL | B | 3 | -29.759 | -12.953 | 23.249 | 1.00 | 38.55 | C |
| ATOM | 1681 | C | VAL | B | 3 | -29.539 | -11.943 | 24.372 | 1.00 | 39.90 | C |
| ATOM | 1682 | O | VAL | B | 3 | -30.258 | -11.945 | 25.372 | 1.00 | 40.51 | O |
| ATOM | 1683 | CB | VAL | B | 3 | -31.042 | -12.599 | 22.476 | 1.00 | 39.53 | C |
| ATOM | 1684 | CG1 | VAL | B | 3 | -30.987 | -11.169 | 21.965 | 1.00 | 34.86 | C |
| ATOM | 1685 | CG2 | VAL | B | 3 | -31.241 | -13.570 | 21.324 | 1.00 | 38.27 | C |
| ATOM | 1686 | N | MET | B | 4 | -28.528 | -11.094 | 24.214 | 1.00 | 35.54 | N |
| ATOM | 1687 | CA | MET | B | 4 | -28.248 | -10.063 | 25.208 | 1.00 | 37.31 | C |
| ATOM | 1688 | C | MET | B | 4 | -28.677 | -8.695 | 24.687 | 1.00 | 34.89 | C |
| ATOM | 1689 | O | MET | B | 4 | -28.362 | -8.327 | 23.559 | 1.00 | 35.92 | O |
| ATOM | 1690 | CB | MET | B | 4 | -26.760 | -10.043 | 25.570 | 1.00 | 29.00 | C |
| ATOM | 1691 | CG | MET | B | 4 | -26.173 | -11.410 | 25.886 | 1.00 | 32.64 | C |
| ATOM | 1692 | SD | MET | B | 4 | -26.854 | -12.116 | 27.401 | 1.00 | 36.18 | S |
| ATOM | 1693 | CE | MET | B | 4 | -26.247 | -10.945 | 28.614 | 1.00 | 29.96 | C |
| ATOM | 1694 | N | THR | B | 5 | -29.405 | -7.953 | 25.510 | 1.00 | 36.75 | N |
| ATOM | 1695 | CA | THR | B | 5 | -29.788 | -6.591 | 25.170 | 1.00 | 36.17 | C |
| ATOM | 1696 | C | THR | B | 5 | -29.273 | -5.640 | 26.238 | 1.00 | 38.85 | C |
| ATOM | 1697 | O | THR | B | 5 | -29.080 | -6.029 | 27.392 | 1.00 | 40.10 | O |
| ATOM | 1698 | CB | THR | B | 5 | -31.315 | -6.436 | 25.040 | 1.00 | 33.68 | C |
| ATOM | 1699 | CG2 | THR | B | 5 | -31.853 | -7.309 | 23.910 | 1.00 | 30.80 | C |

(continued)

| | | | | | | | | | | | |
|---|---|---|---|---|---|---|---|---|---|---|---|
| ATOM | 1700 | OG1 | THR | B | 5 | -31.938 | -6.816 | 26.270 | 1.00 | 36.62 | O |
| ATOM | 1701 | N | GLN | B | 6 | -29.038 | -4.394 | 25.848 | 1.00 | 33.36 | N |
| ATOM | 1702 | CA | GLN | B | 6 | -28.602 | -3.376 | 26.788 | 1.00 | 33.06 | C |
| ATOM | 1703 | C | GLN | B | 6 | -29.438 | -2.119 | 26.620 | 1.00 | 31. 88 | C |
| ATOM | 1704 | O | GLN | B | 6 | -30.040 | -1.899 | 25.571 | 1.00 | 35.73 | O |
| ATOM | 1705 | CB | GLN | B | 6 | -27.116 | -3.048 | 26.599 | 1.00 | 33.60 | C |
| ATOM | 1706 | CG | GLN | B | 6 | -26.169 | -4.214 | 26.870 | 1.00 | 36.92 | C |
| ATOM | 1707 | CD | GLN | B | 6 | -24.721 | -3.883 | 26.533 | 1.00 | 38.24 | C |
| ATOM | 1708 | NE2 | GLN | B | 6 | -24.272 | -2.702 | 26.941 | 1.00 | 30.52 | N |
| ATOM | 1709 | OE1 | GLN | B | 6 | -24.017 | -4.682 | 25.915 | 1.00 | 36.39 | O |
| ATOM | 1710 | N | SER | B | 7 | -29.470 | -1.302 | 27.665 | 1.00 | 32.38 | N |
| ATOM | 1711 | CA | SER | B | 7 | -30.103 | 0.006 | 27.606 | 1.00 | 37.23 | C |
| ATOM | 1712 | C | SER | B | 7 | -29.416 | 0.933 | 28.601 | 1.00 | 36.04 | C |
| ATOM | 1713 | O | SER | B | 7 | -28.976 | 0.485 | 29.662 | 1.00 | 39.38 | O |
| ATOM | 1714 | CB | SER | B | 7 | -31.602 | -0.096 | 27.901 | 1.00 | 37.67 | C |
| ATOM | 1715 | OG | SER | B | 7 | -31.826 | -0.591 | 29.208 | 1.00 | 50.24 | O |
| ATOM | 1716 | N | PRO | B | B | -29.296 | 2.226 | 28.258 | 1.00 | 38.41 | N |
| ATOM | 1717 | CA | PRO | B | B | -29.712 | 2.835 | 26.989 | 1.00 | 38.28 | C |
| ATOM | 1718 | C | PRO | B | B | -28.708 | 2.568 | 25.872 | 1.00 | 44.42 | C |
| ATOM | 1719 | O | PRO | B | B | -27.687 | 1.924 | 26.117 | 1.00 | 41.31 | O |
| ATOM | 1720 | CB | PRO | B | B | -29.766 | 4.326 | 27.325 | 1.00 | 41.32 | C |
| ATOM | 1721 | CG | PRO | B | B | -28.725 | 4.492 | 28.383 | 1.00 | 37.24 | C |
| ATOM | 1722 | CD | PRO | B | B | -28.754 | 3.226 | 29.197 | 1.00 | 31.36 | C |
| ATOM | 1723 | N | ASP | B | 9 | -28.994 | 3.049 | 24.667 | 1.00 | 42.66 | N |
| ATOM | 1724 | CA | ASP | B | 9 | -28.037 | 2.952 | 23.570 | 1.00 | 44.47 | C |
| ATOM | 1725 | C | ASP | B | 9 | -26.850 | 3.863 | 23.842 | 1.00 | 46.92 | C |
| ATOM | 1726 | O | ASP | B | 9 | -25.699 | 3.503 | 23.588 | 1.00 | 38.54 | O |
| ATOM | 1727 | CB | ASP | B | 9 | -28.687 | 3.322 | 22.236 | 1.00 | 44.64 | C |
| ATOM | 1728 | CG | ASP | B | 9 | -29.773 | 2.349 | 21.827 | 1.00 | 70.41 | C |
| ATOM | 1729 | OD1 | ASP | B | 9 | -29.730 | 1.181 | 22.272 | 1.00 | 74.55 | O |
| ATOM | 1730 | OD2 | ASP | B | 9 | -30.671 | 2.753 | 21.057 | 1.00 | 80.56 | O |
| ATOM | 1731 | N | SER | B | 10 | -27.150 | 5.044 | 24.371 | 1.00 | 42.57 | N |
| ATOM | 1732 | CA | SER | B | 10 | -26.146 | 6.061 | 24.628 | 1.00 | 39.96 | C |
| ATOM | 1733 | C | SER | B | 10 | -26.395 | 6.721 | 25.978 | 1.00 | 42.60 | C |

(continued)

| | | | | | | | | | | | |
|---|---|---|---|---|---|---|---|---|---|---|---|
| ATOM | 1734 | O | SER | B | 10 | -27.522 | 6.742 | 26.475 | 1.00 | 43.39 | O |
| ATOM | 1735 | CB | SER | B | 10 | -26.149 | 7.110 | 23.515 | 1.00 | 29.15 | C |
| ATOM | 1736 | OG | SER | B | 10 | -25.012 | 7.943 | 23.611 | 1.00 | 55.37 | O |
| ATOM | 1737 | N | LEU | B | 11 | -25.342 | 7.286 | 26.553 | 1.00 | 38.86 | N |
| ATOM | 1738 | CA | LEU | B | 11 | -25.384 | 7.756 | 27.927 | 1.00 | 39.26 | C |
| ATOM | 1739 | C | LEU | B | 11 | -24.310 | 8.819 | 28.132 | 1.00 | 33.06 | C |
| ATOM | 1740 | O | LEU | B | 11 | -23.172 | 8.636 | 27.709 | 1.00 | 43.59 | O |
| ATOM | 1741 | CB | LEU | B | 11 | -25.186 | 6.564 | 28.875 | 1.00 | 46.93 | C |
| ATOM | 1742 | CG | LEU | B | 11 | -25.354 | 6.653 | 30.391 | 1.00 | 40.86 | C |
| ATOM | 1743 | CD1 | LEU | B | 11 | -24.122 | 7.245 | 31.054 | 1.00 | 49.46 | C |
| ATOM | 1744 | CD2 | LEU | B | 11 | -26.602 | 7.452 | 30.735 | 1.00 | 58.72 | C |
| ATOM | 1745 | N | ALA | B | 12 | -24.668 | 9.930 | 28.768 | 1.00 | 34.90 | N |
| ATOM | 1746 | CA | ALA | B | 12 | -23.695 | 10.979 | 29.078 | 1.00 | 32.04 | C |
| ATOM | 1747 | C | ALA | B | 12 | -23.942 | 11.565 | 30.460 | 1.00 | 35.38 | C |
| ATOM | 1748 | O | ALA | B | 12 | -25.070 | 11.894 | 30.813 | 1.00 | 40.40 | O |
| ATOM | 1749 | CB | ALA | B | 12 | -23.733 | 12.076 | 28.027 | 1.00 | 39.73 | C |
| ATOM | 1750 | N | VAL | B | 13 | -22.874 | 11.709 | 31.235 | 1.00 | 38.02 | N |
| ATOM | 1751 | CA | VAL | B | 13 | -22.987 | 12.114 | 32.629 | 1.00 | 32.52 | C |
| ATOM | 1752 | C | VAL | B | 13 | -21.794 | 12.994 | 33.009 | 1.00 | 41.29 | C |
| ATOM | 1753 | O | VAL | B | 13 | -20.707 | 12.841 | 32.450 | 1.00 | 40.66 | O |
| ATOM | 1754 | CB | VAL | B | 13 | -23.098 | 10.856 | 33.537 | 1.00 | 47.79 | C |
| ATOM | 1755 | CG1 | VAL | B | 13 | -22.187 | 10.934 | 34.752 | 1.00 | 44.21 | C |
| ATOM | 1756 | CG2 | VAL | B | 13 | -24.548 | 10.619 | 33.939 | 1.00 | 43.75 | C |
| ATOM | 1757 | N | SER | B | 14 | -21.999 | 13.933 | 33.931 | 1.00 | 41.23 | N |
| ATOM | 1758 | CA | SER | B | 14 | -20.938 | 14.857 | 34.333 | 1.00 | 36.91 | C |
| ATOM | 1759 | C | SER | B | 14 | -19.862 | 14.158 | 35.162 | 1.00 | 39.93 | C |
| ATOM | 1760 | O | SER | B | 14 | -20.125 | 13.118 | 35.766 | 1.00 | 42.44 | O |
| ATOM | 1761 | CB | SER | B | 14 | -21.528 | 16.030 | 35.121 | 1.00 | 46.50 | C |
| ATOM | 1762 | OG | SER | B | 14 | -22.570 | 16.660 | 34.393 | 1.00 | 52.97 | O |
| ATOM | 1763 | N | LEU | B | 15 | -18.656 | 14.729 | 35.182 | 1.00 | 35.81 | N |
| ATOM | 1764 | CA | LEU | B | 15 | -17.549 | 14.188 | 35.975 | 1.00 | 41.19 | C |
| ATOM | 1765 | C | LEU | B | 15 | -17.949 | 13.897 | 37.417 | 1.00 | 46.73 | C |
| ATOM | 1766 | O | LEU | B | 15 | -18.711 | 14.651 | 38.022 | 1.00 | 44.31 | O |
| ATOM | 1767 | CB | LEU | B | 15 | -16.356 | 15.148 | 35.990 | 1.00 | 42.78 | C |

(continued)

| | | | | | | | | | | |
|---|---|---|---|---|---|---|---|---|---|---|
| ATOM | 1768 | CG | LEU | B | 15 | -15.368 | 15.192 | 34.825 | 1.00 | 60.55 | C |
| ATOM | 1769 | CD1 | LEU | B | 15 | -15.877 | 16.103 | 33.716 | 1.00 | 58.51 | C |
| ATOM | 1770 | CD2 | LEU | B | 15 | -13.996 | 15. 648 | 35.316 | 1.00 | 59.55 | C |
| ATOM | 1771 | N | GLY | B | 16 | -17.431 | 12. 800 | 37.960 | 1.00 | 41.51 | N |
| ATOM | 1772 | CA | GLY | B | 16 | -17.639 | 12.466 | 39.355 | 1.00 | 38.32 | C |
| ATOM | 1773 | C | GLY | B | 16 | -19.048 | 12.012 | 39.675 | 1.00 | 37.62 | C |
| ATOM | 1774 | O | GLY | B | 16 | -19.362 | 11.730 | 40.829 | 1.00 | 45.46 | O |
| ATOM | 1775 | N | GLU | B | 17 | -19.902 | 11.938 | 38.661 | 1.00 | 40.92 | N |
| ATOM | 1776 | CA | GLU | B | 17 | -21.273 | 11.496 | 38.876 | 1.00 | 41.65 | C |
| ATOM | 1777 | C | GLU | B | 17 | -21.458 | 10.032 | 38.491 | 1.00 | 45.71 | C |
| ATOM | 1778 | O | GLU | B | 17 | -20.568 | 9.404 | 37.912 | 1.00 | 38.54 | O |
| ATOM | 1779 | CB | GLU | B | 17 | -22.256 | 12.378 | 38.104 | 1.00 | 42.09 | C |
| ATOM | 1780 | CG | GLU | B | 17 | -22.365 | 13.791 | 38.659 | 1.00 | 48.23 | C |
| ATOM | 1781 | CD | GLU | B | 17 | -23.478 | 14.596 | 38.011 | 1.00 | 66.55 | C |
| ATOM | 1782 | OE1 | GLU | B | 17 | -23.909 | 14.234 | 36.894 | 1.00 | 71.11 | O |
| ATOM | 1783 | OE2 | GLU | B | 17 | -23.923 | 15.591 | 38.624 | 1.00 | 74.03 | O |
| ATOM | 1784 | N | ARG | B | 18 | -22.627 | 9.500 | 38.823 | 1.00 | 45.65 | N |
| ATOM | 1785 | CA | ARG | B | 18 | -22.899 | 8.077 | 38.701 | 1.00 | 45.58 | C |
| ATOM | 1786 | C | ARG | B | 18 | -23.508 | 7.729 | 37.346 | 1.00 | 40.92 | C |
| ATOM | 1787 | O | ARG | B | 18 | -24.379 | 8.436 | 36.843 | 1.00 | 38.35 | O |
| ATOM | 1788 | CB | ARG | B | 18 | -23.826 | 7.638 | 39.838 | 1.00 | 43.55 | C |
| ATOM | 1789 | CG | ARG | B | 18 | -24.162 | 6.165 | 39.879 | 1.00 | 54.20 | C |
| ATOM | 1790 | CD | ARG | B | 18 | -24.969 | 5.853 | 41.132 | 1.00 | 66.41 | C |
| ATOM | 1791 | NE | ARG | B | 18 | -25.394 | 4.459 | 41.181 | 1.00 | 84.38 | N |
| ATOM | 1792 | CZ | ARG | B | 18 | -26.076 | 3.921 | 42.186 | 1.00 | 83.62 | C |
| ATOM | 1793 | NH1 | ARG | B | 18 | -26.412 | 4.662 | 43.234 | 1.00 | 81.35 | N |
| ATOM | 1794 | NH2 | ARG | B | 18 | -26.420 | 2.640 | 42.142 | 1.00 | 85.37 | N |
| ATOM | 1795 | N | ALA | B | 19 | -23.030 | 6.643 | 36.751 | 1.00 | 35.20 | N |
| ATOM | 1796 | CA | ALA | B | 19 | -23.584 | 6.161 | 35.491 | 1.00 | 34.46 | C |
| ATOM | 1797 | C | ALA | B | 19 | -23.980 | 4.696 | 35.617 | 1.00 | 39.57 | C |
| ATOM | 1798 | O | ALA | B | 19 | -23.276 | 3.904 | 36.247 | 1.00 | 38.45 | O |
| ATOM | 1799 | CB | ALA | B | 19 | -22.588 | 6.351 | 34.356 | 1.00 | 36.85 | C |
| ATOM | 1800 | N | THR | B | 20 | -25.105 | 4.344 | 35.004 | 1.00 | 33.06 | N |
| ATOM | 1801 | CA | THR | B | 20 | -25.654 | 3.001 | 35.107 | 1.00 | 35.98 | C |

(continued)

| | | | | | | | | | | | |
|---|---|---|---|---|---|---|---|---|---|---|---|
| ATOM | 1802 | C | THR | B | 20 | -26.066 | 2.452 | 33.742 | 1.00 | 38.75 | C |
| ATOM | 1803 | O | THR | B | 20 | -26.762 | 3.118 | 32.979 | 1.00 | 37.55 | O |
| ATOM | 1804 | CB | THR | B | 20 | -26.866 | 2.987 | 36.062 | 1.00 | 38.02 | C |
| ATOM | 1805 | CG2 | THR | B | 20 | -27.718 | 1.757 | 35.842 | 1.00 | 44.22 | C |
| ATOM | 1806 | OG1 | THR | B | 20 | -26.398 | 3.008 | 37.419 | 1.00 | 45.86 | O |
| ATOM | 1807 | N | ILE | B | 21 | -25.622 | 1.235 | 33.441 | 1.00 | 37.58 | N |
| ATOM | 1808 | CA | ILE | B | 21 | -25.973 | 0.559 | 32.196 | 1.00 | 35.14 | C |
| ATOM | 1809 | C | ILE | B | 21 | -26.661 | -0.765 | 32.507 | 1.00 | 37.66 | C |
| ATOM | 1810 | O | ILE | B | 21 | -26.193 | -1.527 | 33.350 | 1.00 | 32.81 | O |
| ATOM | 1811 | CB | ILE | B | 21 | -24.733 | 0.284 | 31.326 | 1.00 | 38.29 | C |
| ATOM | 1812 | CG1 | ILE | B | 21 | -23.964 | 1.573 | 31.057 | 1.00 | 37.46 | C |
| ATOM | 1813 | CG2 | ILE | B | 21 | -25.127 | -0.395 | 30.018 | 1.00 | 45.60 | C |
| ATOM | 1814 | CD1 | ILE | B | 21 | -22.717 | 1.348 | 30.238 | 1.00 | 41.15 | C |
| ATOM | 1815 | N | ASN | B | 22 | -27.761 | -1.046 | 31.819 | 1.00 | 30.24 | N |
| ATOM | 1816 | CA | ASN | B | 22 | -28.521 | -2.260 | 32.084 | 1.00 | 37.10 | C |
| ATOM | 1817 | C | ASN | B | 22 | -28.270 | -3.358 | 31.050 | 1.00 | 40.28 | C |
| ATOM | 1818 | O | ASN | B | 22 | -28.041 | -3.081 | 29.870 | 1.00 | 35.19 | O |
| ATOM | 1819 | CB | ASN | B | 22 | -30.013 | -1.932 | 32.157 | 1.00 | 36.20 | C |
| ATOM | 1820 | CG | ASN | B | 22 | -30.308 | -0.788 | 33.121 | 1.00 | 60.60 | C |
| ATOM | 1821 | OD1 | ASN | B | 22 | -30.309 | -0.974 | 34.341 | 1.00 | 57.11 | O |
| ATOM | 1822 | ND2 | ASN | B | 22 | -30.547 | 0.407 | 32.576 | 1.00 | 55.17 | N |
| ATOM | 1823 | N | CYS | B | 23 | -28.315 | -4.604 | 31.509 | 1.00 | 36.70 | N |
| ATOM | 1824 | CA | CYS | B | 23 | -28.121 | -5.762 | 30.647 | 1.00 | 34.30 | C |
| ATOM | 1825 | C | CYS | B | 23 | -29.231 | -6.791 | 30.872 | 1.00 | 38.13 | C |
| ATOM | 1826 | O | CYS | B | 23 | -29.570 | -7.107 | 32.012 | 1.00 | 40.38 | O |
| ATOM | 1827 | CB | CYS | B | 23 | -26.748 | -6.391 | 30.913 | 1.00 | 37.80 | C |
| ATOM | 1828 | SG | CYS | B | 23 | -26.222 | -7.657 | 29.735 | 1.00 | 53.67 | S |
| ATOM | 1829 | N | LYS | B | 24 | -29.794 | -7.312 | 29.786 | 1.00 | 33.98 | N |
| ATOM | 1830 | CA | LYS | B | 24 | -30.813 | -8.356 | 29.871 | 1.00 | 36.97 | C |
| ATOM | 1831 | C | LYS | B | 24 | -30.440 | -9.564 | 29.024 | 1.00 | 42.52 | C |
| ATOM | 1832 | O | LYS | B | 24 | -29.979 | -9.419 | 27.890 | 1.00 | 38.82 | O |
| ATOM | 1833 | CB | LYS | B | 24 | -32.181 | -7 . 831 | 29.425 | 1.00 | 43.72 | C |
| ATOM | 1834 | CG | LYS | B | 24 | -33.084 | -7.356 | 30.550 | 1.00 | 55.35 | C |
| ATOM | 1835 | CD | LYS | B | 24 | -34.508 | -7.143 | 30.045 | 1.00 | 61.25 | C |

(continued)

| | | | | | | | | | | | |
|---|---|---|---|---|---|---|---|---|---|---|---|
| ATOM | 1836 | CE | LYS | B | 24 | -35.433 | -6.673 | 31.155 | 1.00 | 65.56 | C |
| ATOM | 1837 | NZ | LYS | B | 24 | -35.055 | -5.323 | 31.656 | 1.00 | 61.27 | N |
| ATOM | 1838 | N | SER | B | 25 | -30.644 | -10.755 | 29.580 | 1.00 | 37.96 | N |
| ATOM | 1839 | CA | SER | B | 25 | -30.438 | -11.991 | 28.835 | 1.00 | 41.49 | C |
| ATOM | 1840 | C | SER | B | 25 | -31.782 | -12.646 | 28.540 | 1.00 | 40.87 | C |
| ATOM | 1841 | O | SER | B | 25 | -32.677 | -12.640 | 29.379 | 1.00 | 42.24 | O |
| ATOM | 1842 | CB | SER | B | 25 | -29.524 | -12.949 | 29.603 | 1.00 | 33.20 | C |
| ATOM | 1843 | OG | SER | B | 25 | -29.974 | -13.149 | 30.931 | 1.00 | 36.26 | O |
| ATOM | 1844 | N | SER | B | 26 | -31.915 | -13.209 | 27.342 | 1.00 | 38.29 | N |
| ATOM | 1845 | CA | SER | B | 26 | -33.176 | -13.790 | 26.895 | 1.00 | 38.35 | C |
| ATOM | 1846 | C | SER | B | 26 | -33.605 | -14.975 | 27.759 | 1.00 | 43.66 | C |
| ATOM | 1847 | O | SER | B | 26 | -34.760 | -15.394 | 27.720 | 1.00 | 40.54 | O |
| ATOM | 1848 | CB | SER | B | 26 | -33.067 | -14.224 | 25.434 | 1.00 | 35.59 | C |
| ATOM | 1849 | OG | SER | B | 26 | -32.037 | -15.182 | 25.272 | 1.00 | 38.41 | O |
| ATOM | 1850 | N | GLN | B | 27 | -32.666 | -15.519 | 28.527 | 1.00 | 42.85 | N |
| ATOM | 1851 | CA | GLN | B | 27 | -32.979 | -16.560 | 29.498 | 1.00 | 39.30 | C |
| ATOM | 1852 | C | GLN | B | 27 | -32.004 | -16.465 | 30.660 | 1.00 | 43.02 | C |
| ATOM | 1853 | O | GLN | B | 27 | -31.011 | -15.745 | 30.581 | 1.00 | 38.87 | O |
| ATOM | 1854 | CB | GLN | B | 27 | -32.926 | -17.946 | 28.858 | 1.00 | 40.88 | C |
| ATOM | 1855 | CG | GLN | B | 27 | -31.538 | -18.387 | 28.447 | 1.00 | 44.20 | C |
| ATOM | 1856 | CD | GLN | B | 27 | -31.549 | -19.707 | 27.705 | 1.00 | 51.33 | C |
| ATOM | 1857 | NE2 | GLN | B | 27 | -31.158 | -20.776 | 28.393 | 1.00 | 42.28 | N |
| ATOM | 1858 | OE1 | GLN | B | 27 | -31.906 | -19.769 | 26.525 | 1.00 | 47. 83 | O |
| ATOM | 1859 | N | SER | B | 28 | -32.291 | -17.187 | 31.738 | 1.00 | 40.20 | N |
| ATOM | 1860 | CA | SER | B | 28 | -31.473 | -17.109 | 32.941 | 1.00 | 36.91 | C |
| ATOM | 1861 | C | SER | B | 28 | -30.065 | -17.631 | 32.699 | 1.00 | 41.10 | C |
| ATOM | 1862 | O | SER | B | 28 | -29.871 | -18.659 | 32.048 | 1.00 | 36.62 | O |
| ATOM | 1863 | CB | SER | B | 28 | -32.120 | -17.889 | 34.087 | 1.00 | 41.70 | C |
| ATOM | 1864 | OG | SER | B | 28 | -31.399 | -17.700 | 35.294 | 1.00 | 42.03 | O |
| ATOM | 1865 | N | ILE | B | 29 | -29.084 | -16.906 | 33.227 | 1.00 | 36.07 | N |
| ATOM | 1866 | CA | ILE | B | 29 | -27.692 | -17.320 | 33.149 | 1.00 | 34.98 | C |
| ATOM | 1867 | C | ILE | B | 29 | -27.143 | -17.474 | 34.564 | 1.00 | 36.30 | C |
| ATOM | 1868 | O | ILE | B | 29 | -25.934 | -17.400 | 34.795 | 1.00 | 34.45 | O |
| ATOM | 1869 | CB | ILE | B | 29 | -26.846 | -16.316 | 32.344 | 1.00 | 31.71 | C |

(continued)

| | | | | | | | | | | | |
|---|---|---|---|---|---|---|---|---|---|---|---|
| ATOM | 1870 | CG1 | ILE | B | 29 | -26.947 | -14.916 | 32.949 | 1.00 | 29.95 | C |
| ATOM | 1871 | CG2 | ILE | B | 29 | -27.310 | -16.276 | 30.898 | 1.00 | 29.61 | C |
| ATOM | 1872 | CD1 | ILE | B | 29 | -26.198 | -13.855 | 32.145 | 1.00 | 31.42 | C |
| ATOM | 1873 | O | LEU | B | 30 | -28.655 | -20.230 | 36.655 | 1.00 | 44.52 | O |
| ATOM | 1874 | N | LEU | B | 30 | -28.058 | -17.672 | 35.508 | 1.00 | 33.91 | N |
| ATOM | 1875 | CA | LEU | B | 30 | -27.709 | -18.044 | 36.873 | 1.00 | 39.31 | C |
| ATOM | 1876 | C | LEU | B | 30 | -27.673 | -19.563 | 36.977 | 1.00 | 38.06 | C |
| ATOM | 1877 | CB | LEU | B | 30 | -28.716 | -17.465 | 37.872 | 1.00 | 37.18 | C |
| ATOM | 1878 | CG | LEU | B | 30 | -28.589 | -17.932 | 39.327 | 1.00 | 39.11 | C |
| ATOM | 1879 | CD2 | LEU | B | 30 | -29.827 | -17.558 | 40.128 | 1.00 | 36.35 | C |
| ATOM | 1880 | CD1 | LEU | B | 30 | -27.331 | -17.378 | 39.987 | 1.00 | 36.12 | C |
| ATOM | 1881 | O | HIS | B | 31 | -26.681 | -21.536 | 39.965 | 1.00 | 38.61 | O |
| ATOM | 1882 | N | HIS | B | 31 | -26.546 | -20.117 | 37.411 | 1.00 | 33.48 | N |
| ATOM | 1883 | CA | HIS | B | 31 | -26.464 | -21.562 | 37.580 | 1.00 | 38.93 | C |
| ATOM | 1884 | C | HIS | B | 31 | -27.113 | -21.968 | 38.897 | 1.00 | 39.33 | C |
| ATOM | 1885 | CB | HIS | B | 31 | -25.018 | -22.046 | 37.533 | 1.00 | 38.09 | C |
| ATOM | 1886 | CG | HIS | B | 31 | -24.889 | -23.538 | 37.446 | 1.00 | 39.11 | C |
| ATOM | 1887 | ND1 | HIS | B | 31 | -24.604 | -24.190 | 36.272 | 1.00 | 42.93 | N |
| ATOM | 1888 | CD2 | HIS | B | 31 | -25.028 | -24.496 | 38.394 | 1.00 | 37.40 | C |
| ATOM | 1889 | CE1 | HIS | B | 31 | -24.560 | -25.497 | 36.497 | 1.00 | 40.76 | C |
| ATOM | 1890 | NE2 | HIS | B | 31 | -24.816 | -25.705 | 37.772 | 1.00 | 41.52 | N |
| ATOM | 1891 | O | SER | B | 32 | -28.568 | -23.745 | 42.260 | 1.00 | 57.25 | O |
| ATOM | 1892 | N | SER | B | 32 | -28.140 | -22.809 | 38.812 | 1.00 | 43.81 | N |
| ATOM | 1893 | CA | SER | B | 32 | -28.969 | -23.149 | 39.972 | 1.00 | 49.61 | C |
| ATOM | 1894 | C | SER | B | 32 | -28.209 | -23.866 | 41.089 | 1.00 | 43.17 | C |
| ATOM | 1895 | CB | SER | B | 32 | -30.159 | -24.004 | 39.530 | 1.00 | 47.25 | C |
| ATOM | 1896 | OG | SER | B | 32 | -29.731 | -25.115 | 38.758 | 1.00 | 56.93 | O |
| ATOM | 1897 | O | SER | B | 33 | -25.191 | -24.612 | 43.655 | 1.00 | 47.19 | O |
| ATOM | 1898 | N | SER | B | 33 | -27.155 | -24.596 | 40.737 | 1.00 | 40.34 | N |
| ATOM | 1899 | CA | SER | B | 33 | -26.407 | -25.373 | 41.729 | 1.00 | 50.61 | C |
| ATOM | 1900 | C | SER | B | 33 | -25.300 | -24.578 | 42.428 | 1.00 | 46.45 | C |
| ATOM | 1901 | CB | SER | B | 33 | -25.807 | -26.621 | 41.079 | 1.00 | 46.34 | C |
| ATOM | 1902 | OG | SER | B | 33 | -26.825 | -27.469 | 40.581 | 1.00 | 54.71 | O |
| ATOM | 1903 | O | ASN | B | 34 | -22.973 | -20.977 | 43.235 | 1.00 | 37.27 | O |

(continued)

| | | | | | | | | | | | |
|---|---|---|---|---|---|---|---|---|---|---|---|
| ATOM | 1904 | N | ASN | B | 34 | -24.470 | -23.875 | 41.661 | 1.00 | 42.95 | N |
| ATOM | 1905 | CA | ASN | B | 34 | -23.365 | -23.137 | 42.269 | 1.00 | 42.26 | C |
| ATOM | 1906 | C | ASN | B | 34 | -23.735 | -21.686 | 42.578 | 1.00 | 34.30 | C |
| ATOM | 1907 | CB | ASN | B | 34 | -22.112 | -23.195 | 41.378 | 1.00 | 39.81 | C |
| ATOM | 1908 | CG | ASN | B | 34 | -22.306 | -22.519 | 40.027 | 1.00 | 38.01 | C |
| ATOM | 1909 | OD1 | ASN | B | 34 | -22.552 | -21.313 | 39.949 | 1.00 | 35.29 | O |
| ATOM | 1910 | ND2 | ASN | B | 34 | -22.163 | -23.292 | 38.952 | 1.00 | 32.57 | N |
| ATOM | 1911 | O | ASN | B | 35 | -24.573 | -17.694 | 42.495 | 1.00 | 38.12 | O |
| ATOM | 1912 | N | ASN | B | 35 | -24.900 | -21.256 | 42.095 | 1.00 | 36.64 | N |
| ATOM | 1913 | CA | ASN | B | 35 | -25.455 | -19.936 | 42.414 | 1.00 | 41.38 | C |
| ATOM | 1914 | C | ASN | B | 35 | -24.612 | -18.762 | 41.888 | 1.00 | 38.67 | C |
| ATOM | 1915 | CB | ASN | B | 35 | -25.650 | -19.805 | 43.936 | 1.00 | 38.07 | C |
| ATOM | 1916 | CG | ASN | B | 35 | -26.644 | -18.719 | 44.315 | 1.00 | 52.73 | C |
| ATOM | 1917 | OD1 | ASN | B | 35 | -27.611 | -18.462 | 43.594 | 1.00 | 51.71 | O |
| ATOM | 1918 | ND2 | ASN | B | 35 | -26.403 | -18.069 | 45.452 | 1.00 | 46.79 | N |
| ATOM | 1919 | O | ASN | B | 36 | -24.385 | -18.267 | 38.068 | 1.00 | 37.24 | O |
| ATOM | 1920 | N | ASN | B | 36 | -23.947 | -18.955 | 40.752 | 1.00 | 32.67 | N |
| ATOM | 1921 | CA | ASN | B | 36 | -23.207 | -17.863 | 40.120 | 1.00 | 35.30 | C |
| ATOM | 1922 | C | ASN | B | 36 | -23.838 | -17.439 | 38.801 | 1.00 | 30.07 | C |
| ATOM | 1923 | CB | ASN | B | 36 | -21.743 | -18.252 | 39.888 | 1.00 | 30.99 | C |
| ATOM | 1924 | CG | ASN | B | 36 | -20.922 | -18.234 | 41.165 | 1.00 | 39.65 | C |
| ATOM | 1925 | OD1 | ASN | B | 36 | -20.340 | -17.210 | 41.526 | 1.00 | 36.59 | O |
| ATOM | 1926 | ND2 | ASN | B | 36 | -20.870 | -19.369 | 41.854 | 1.00 | 34.78 | N |
| ATOM | 1927 | O | ASN | B | 37 | -22.048 | -14.928 | 36.474 | 1.00 | 26.67 | O |
| ATOM | 1928 | N | ASN | B | 37 | -23.761 | -16.144 | 38.510 | 1.00 | 28.56 | N |
| ATOM | 1929 | CA | ASN | B | 37 | -24.222 | -15.603 | 37.237 | 1.00 | 32.35 | C |
| ATOM | 1930 | C | ASN | B | 37 | -23.090 | -15.540 | 36.217 | 1.00 | 35.69 | C |
| ATOM | 1931 | CB | ASN | B | 37 | -24.817 | -14.214 | 37.437 | 1.00 | 33.21 | C |
| ATOM | 1932 | CG | ASN | B | 37 | -25.996 | -14.219 | 38.386 | 1.00 | 35.83 | C |
| ATOM | 1933 | OD1 | ASN | B | 37 | -25.854 | -13.922 | 39.573 | 1.00 | 39.01 | O |
| ATOM | 1934 | ND2 | ASN | B | 37 | -27.166 | -14.566 | 37.868 | 1.00 | 28.90 | N |
| ATOM | 1935 | N | TYR | B | 38 | -23.306 | -16.156 | 35.059 | 1.00 | 26.80 | N |
| ATOM | 1936 | CA | TYR | B | 38 | -22.251 | -16.313 | 34.063 | 1.00 | 31.80 | C |
| ATOM | 1937 | C | TYR | B | 38 | -22.234 | -15.119 | 33.118 | 1.00 | 29.97 | C |

(continued)

| | | | | | | | | | | | |
|---|---|---|---|---|---|---|---|---|---|---|---|
| ATOM | 1938 | O | TYR | B | 38 | -22.505 | -15.241 | 31.924 | 1.00 | 29.53 | O |
| ATOM | 1939 | CB | TYR | B | 38 | -22.431 | -17.628 | 33.295 | 1.00 | 28.25 | C |
| ATOM | 1940 | CG | TYR | B | 38 | -22.072 | -18.859 | 34.115 | 1.00 | 31.67 | C |
| ATOM | 1941 | CD2 | TYR | B | 38 | -21.149 | -19.792 | 33.649 | 1.00 | 32.47 | C |
| ATOM | 1942 | CD1 | TYR | B | 38 | -22.656 | -19.083 | 35.358 | 1.00 | 26.38 | C |
| ATOM | 1943 | CE2 | TYR | B | 38 | -20.816 | -20.912 | 34.406 | 1.00 | 31.96 | C |
| ATOM | 1944 | CE1 | TYR | B | 38 | -22.327 | -20.190 | 36.118 | 1.00 | 30.74 | C |
| ATOM | 1945 | CZ | TYR | B | 38 | -21.414 | -21.102 | 35.639 | 1.00 | 32.95 | C |
| ATOM | 1946 | OH | TYR | B | 38 | -21.096 | -22.200 | 36.403 | 1.00 | 33.05 | O |
| ATOM | 1947 | N | LEU | B | 39 | -21.905 | -13.963 | 33.680 | 1.00 | 29.02 | N |
| ATOM | 1948 | CA | LEU | B | 39 | -21.932 | -12.703 | 32.957 | 1.00 | 26.70 | C |
| ATOM | 1949 | C | LEU | B | 39 | -20.623 | -11.945 | 33.112 | 1.00 | 25.80 | C |
| ATOM | 1950 | O | LEU | B | 39 | -20.043 | -11.912 | 34.202 | 1.00 | 29.17 | O |
| ATOM | 1951 | CB | LEU | B | 39 | -23.082 | -11.829 | 33.457 | 1.00 | 30.90 | C |
| ATOM | 1952 | CG | LEU | B | 39 | -23.219 | -10.510 | 32.698 | 1.00 | 33.97 | C |
| ATOM | 1953 | CD1 | LEU | B | 39 | -24.064 | -10.714 | 31.458 | 1.00 | 27.79 | C |
| ATOM | 1954 | CD2 | LEU | B | 39 | -23.785 | -9.419 | 33.577 | 1.00 | 33.15 | C |
| ATOM | 1955 | N | ALA | B | 40 | -20.168 | -11.330 | 32.025 | 1.00 | 28.13 | N |
| ATOM | 1956 | CA | ALA | B | 40 | -18.989 | -10.470 | 32.068 | 1.00 | 25.92 | C |
| ATOM | 1957 | C | ALA | B | 40 | -19.287 | -9.078 | 31.501 | 1.00 | 26.60 | C |
| ATOM | 1958 | O | ALA | B | 40 | -20.162 | -8.917 | 30.648 | 1.00 | 25.73 | O |
| ATOM | 1959 | CB | ALA | B | 40 | -17 . 836 | -11.111 | 31.309 | 1.00 | 26.93 | C |
| ATOM | 1960 | N | TRP | B | 41 | -18.556 | - 8.079 | 31.986 | 1.00 | 26.55 | N |
| ATOM | 1961 | CA | TRP | B | 41 | -18.651 | -6.720 | 31.455 | 1.00 | 30.35 | C |
| ATOM | 1962 | C | TRP | B | 41 | -17.325 | -6.293 | 30.832 | 1.00 | 29.83 | C |
| ATOM | 1963 | O | TRP | B | 41 | -16.261 | -6.507 | 31.415 | 1.00 | 26.54 | O |
| ATOM | 1964 | CB | TRP | B | 41 | -19.042 | -5.723 | 32.549 | 1.00 | 25.43 | C |
| ATOM | 1965 | CG | TRP | B | 41 | -20.484 | -5.762 | 32.956 | 1.00 | 30.44 | C |
| ATOM | 1966 | CD1 | TRP | B | 41 | -21.014 | -6.405 | 34.039 | 1.00 | 30.19 | C |
| ATOM | 1967 | CD2 | TRP | B | 41 | -21.581 | -5.116 | 32.297 | 1.00 | 28.45 | C |
| ATOM | 1968 | CE2 | TRP | B | 41 | -22.747 | -5.418 | 33.032 | 1.00 | 31.50 | C |
| ATOM | 1969 | CE3 | TRP | B | 41 | -21.692 | -4.318 | 31.154 | 1.00 | 33.61 | C |
| ATOM | 1970 | NE1 | TRP | B | 41 | -22.372 | -6.204 | 34.090 | 1.00 | 28.08 | N |
| ATOM | 1971 | CZ2 | TRP | B | 41 | -24.008 | -4.946 | 32.664 | 1.00 | 32.35 | C |

(continued)

| | | | | | | | | | | | |
|---|---|---|---|---|---|---|---|---|---|---|---|
| ATOM | 1972 | CZ3 | TRP | B | 41 | -22.947 | -3.846 | 30.787 | 1.00 | 31.41 | C |
| ATOM | 1973 | CH2 | TRP | B | 41 | -24.087 | -4.163 | 31.541 | 1.00 | 36.76 | C |
| ATOM | 1974 | N | PHE | B | 42 | -17.400 | -5.679 | 29.655 | 1.00 | 25.32 | N |
| ATOM | 1975 | CA | PHE | B | 42 | -16.220 | -5.212 | 28.939 | 1.00 | 27.15 | C |
| ATOM | 1976 | C | PHE | B | 42 | -16.271 | -3.715 | 28.680 | 1.00 | 31.54 | C |
| ATOM | 1977 | O | PHE | B | 42 | -17.338 | -3.154 | 28.431 | 1.00 | 31.28 | O |
| ATOM | 1978 | CB | PHE | B | 42 | -16.073 | -5.944 | 27.601 | 1.00 | 23.42 | C |
| ATOM | 1979 | CG | PHE | B | 42 | -15.772 | -7.405 | 27.739 | 1.00 | 26.14 | C |
| ATOM | 1980 | CD1 | PHE | B | 42 | -14.462 | -7.847 | 27.845 | 1.00 | 24.13 | C |
| ATOM | 1981 | CD2 | PHE | B | 42 | -16.794 | -8.337 | 27.758 | 1.00 | 29.43 | C |
| ATOM | 1982 | CE1 | PHE | B | 42 | -14.181 | -9.195 | 27.976 | 1.00 | 28.57 | C |
| ATOM | 1983 | CE2 | PHE | B | 42 | -16.518 | -9.690 | 27.887 | 1.00 | 26.26 | C |
| ATOM | 1984 | CZ | PHE | B | 42 | -15.212 | -10.116 | 27.997 | 1.00 | 25.92 | C |
| ATOM | 1985 | N | GLN | B | 43 | -15.109 | -3.076 | 28.723 | 1.00 | 27.61 | N |
| ATOM | 1986 | CA | GLN | B | 43 | -14.976 | -1.691 | 28.289 | 1.00 | 26.82 | C |
| ATOM | 1987 | C | GLN | B | 43 | -14.148 | -1.643 | 27.004 | 1.00 | 32.12 | C |
| ATOM | 1988 | O | GLN | B | 43 | -13.062 | -2.221 | 26.945 | 1.00 | 29.12 | O |
| ATOM | 1989 | CB | GLN | B | 43 | -14.317 | -0.842 | 29.375 | 1.00 | 23.34 | C |
| ATOM | 1990 | CG | GLN | B | 43 | -14.190 | 0.629 | 29.022 | 1.00 | 24.31 | C |
| ATOM | 1991 | CD | GLN | B | 43 | -13.172 | 1.345 | 29.891 | 1.00 | 29.32 | C |
| ATOM | 1992 | NE2 | GLN | B | 43 | -13.650 | 2.131 | 30.851 | 1.00 | 31.37 | N |
| ATOM | 1993 | OE1 | GLN | B | 43 | -11.970 | 1.192 | 29.701 | 1.00 | 31.73 | O |
| ATOM | 1994 | N | GLN | B | 44 | -14.656 | -0.977 | 25.972 | 1.00 | 20.12 | N |
| ATOM | 1995 | CA | GLN | B | 44 | -13.869 | -0.810 | 24.754 | 1.00 | 26.13 | C |
| ATOM | 1996 | C | GLN | B | 44 | -13.601 | 0.662 | 24.464 | 1.00 | 26.14 | C |
| ATOM | 1997 | O | GLN | B | 44 | -14.477 | 1.377 | 23.977 | 1.00 | 29.23 | O |
| ATOM | 1998 | CB | GLN | B | 44 | -14.559 | -1.470 | 23.556 | 1.00 | 24.46 | C |
| ATOM | 1999 | CG | GLN | B | 44 | -13.755 | -1.359 | 22.263 | 1.00 | 26.38 | C |
| ATOM | 2000 | CD | GLN | B | 44 | -14.303 | -2.228 | 21.137 | 1.00 | 28.28 | C |
| ATOM | 2001 | NE2 | GLN | B | 44 | -13.407 | -2.827 | 20.355 | 1.00 | 28.01 | N |
| ATOM | 2002 | OE1 | GLN | B | 44 | -15.512 | -2.363 | 20.977 | 1.00 | 28.62 | O |
| ATOM | 2003 | N | LYS | B | 45 | -12.388 | 1.103 | 24.786 | 1.00 | 27.16 | N |
| ATOM | 2004 | CA | LYS | B | 45 | -11.913 | 2.448 | 24.466 | 1.00 | 35.42 | C |
| ATOM | 2005 | C | LYS | B | 45 | -11.696 | 2.581 | 22.963 | 1.00 | 35.66 | C |

(continued)

| | | | | | | | | | | | |
|---|---|---|---|---|---|---|---|---|---|---|---|
| ATOM | 2006 | O | LYS | B | 45 | -11.493 | 1.577 | 22.281 | 1.00 | 35.60 | O |
| ATOM | 2007 | CB | LYS | B | 45 | -10.602 | 2.752 | 25.205 | 1.00 | 33.99 | C |
| ATOM | 2008 | CG | LYS | B | 45 | -10.694 | 2.701 | 26.707 | 1.00 | 42.68 | C |
| ATOM | 2009 | CD | LYS | B | 45 | -9.353 | 3.040 | 27.351 | 1.00 | 41.29 | C |
| ATOM | 2010 | CE | LYS | B | 45 | -8.230 | 2.156 | 26.828 | 1.00 | 41.00 | C |
| ATOM | 2011 | NZ | LYS | B | 45 | -7.078 | 2.115 | 27.785 | 1.00 | 34.96 | N |
| ATOM | 2012 | N | PRO | B | 46 | -11.726 | 3.820 | 22.440 | 1.00 | 38.26 | N |
| ATOM | 2013 | CA | PRO | B | 46 | -11.508 | 4.031 | 21.001 | 1.00 | 37.25 | C |
| ATOM | 2014 | C | PRO | B | 46 | -10.173 | 3.470 | 20.504 | 1.00 | 36.36 | C |
| ATOM | 2015 | O | PRO | B | 46 | -9.125 | 3.762 | 21.087 | 1.00 | 35.60 | O |
| ATOM | 2016 | CB | PRO | B | 46 | -11.537 | 5.559 | 20.865 | 1.00 | 41.28 | C |
| ATOM | 2017 | CG | PRO | B | 46 | -12.362 | 6.021 | 22.009 | 1.00 | 39.45 | C |
| ATOM | 2018 | CD | PRO | B | 46 | -12.046 | 5.080 | 23.138 | 1.00 | 35.28 | C |
| ATOM | 2019 | N | GLY | B | 47 | -10.225 | 2.664 | 19.447 | 1.00 | 34.42 | N |
| ATOM | 2020 | CA | GLY | B | 47 | -9.034 | 2.100 | 18.835 | 1.00 | 31.31 | C |
| ATOM | 2021 | C | GLY | B | 47 | -8.501 | 0.849 | 19.517 | 1.00 | 42.85 | C |
| ATOM | 2022 | O | GLY | B | 47 | -7.507 | 0.270 | 19.074 | 1.00 | 30.21 | O |
| ATOM | 2023 | N | GLN | B | 48 | -9.163 | 0.425 | 20.589 | 1.00 | 29.62 | N |
| ATOM | 2024 | CA | GLN | B | 48 | -8.662 | -0.672 | 21.411 | 1.00 | 33.47 | C |
| ATOM | 2025 | C | GLN | B | 48 | -9.588 | -1.887 | 21.411 | 1.00 | 33.23 | C |
| ATOM | 2026 | O | GLN | B | 48 | -10.787 | -1.757 | 21.171 | 1.00 | 26.94 | O |
| ATOM | 2027 | CB | GLN | B | 48 | -8.451 | -0.187 | 22.850 | 1.00 | 32.23 | C |
| ATOM | 2028 | CG | GLN | B | 48 | -7.391 | 0.893 | 22.990 | 1.00 | 33.96 | C |
| ATOM | 2029 | CD | GLN | B | 48 | -6.011 | 0.404 | 22.590 | 1.00 | 43.63 | C |
| ATOM | 2030 | OE1 | GLN | B | 48 | -5.276 | 1.095 | 21.885 | 1.00 | 42.98 | O |
| ATOM | 2031 | NE2 | GLN | B | 48 | -5.652 | -0.796 | 23.042 | 1.00 | 39.16 | N |
| ATOM | 2032 | N | PRO | B | 49 | -9.032 | -3.077 | 21.691 | 1.00 | 30.89 | N |
| ATOM | 2033 | CA | PRO | B | 49 | -9.885 | -4.246 | 21.918 | 1.00 | 26.27 | C |
| ATOM | 2034 | C | PRO | B | 49 | -10.687 | -4.069 | 23.203 | 1.00 | 27.88 | C |
| ATOM | 2035 | O | PRO | B | 49 | -10.290 | -3.264 | 24.045 | 1.00 | 27.50 | O |
| ATOM | 2036 | CB | PRO | B | 49 | -8.883 | -5.396 | 22.049 | 1.00 | 26.15 | C |
| ATOM | 2037 | CG | PRO | B | 49 | -7.629 | -4.740 | 22.538 | 1.00 | 28.99 | C |
| ATOM | 2038 | CD | PRO | B | 49 | -7.602 | -3.394 | 21.865 | 1.00 | 32.59 | C |
| ATOM | 2039 | N | PRO | B | 50 | -11.803 | -4.799 | 23.351 | 1.00 | 25.78 | N |

(continued)

| | | | | | | | | | | | |
|---|---|---|---|---|---|---|---|---|---|---|---|
| ATOM | 2040 | CA | PRO | B | 50 | -12.539 | -4.746 | 24.621 | 1.00 | 23.59 | C |
| ATOM | 2041 | C | PRO | B | 50 | -11.625 | -5.113 | 25.793 | 1.00 | 30.73 | C |
| ATOM | 2042 | O | PRO | B | 50 | -10.661 | -5.859 | 25.608 | 1.00 | 26.96 | O |
| ATOM | 2043 | CB | PRO | B | 50 | -13.643 | -5.791 | 24.438 | 1.00 | 24.85 | C |
| ATOM | 2044 | CG | PRO | B | 50 | -13.817 | -5.908 | 22.949 | 1.00 | 25.66 | C |
| ATOM | 2045 | CD | PRO | B | 50 | -12.441 | -5.694 | 22.370 | 1.00 | 25.19 | C |
| ATOM | 2046 | N | LYS | B | 51 | -11.909 | -4.574 | 26.972 | 1.00 | 27.75 | N |
| ATOM | 2047 | CA | LYS | B | 51 | -11.131 | -4.899 | 28.158 | 1.00 | 25.94 | C |
| ATOM | 2048 | C | LYS | B | 51 | -12.052 | -5.420 | 29.251 | 1.00 | 30.23 | C |
| ATOM | 2049 | O | LYS | B | 51 | -13.083 | -4 . 818 | 29.547 | 1.00 | 25.89 | O |
| ATOM | 2050 | CB | LYS | B | 51 | -10.351 | -3.680 | 28.654 | 1.00 | 29.25 | C |
| ATOM | 2051 | CG | LYS | B | 51 | -9.163 | -4.045 | 29.535 | 1.00 | 32.02 | C |
| ATOM | 2052 | CD | LYS | B | 51 | -9.469 | -3.859 | 31.001 | 1.00 | 37.80 | C |
| ATOM | 2053 | CE | LYS | B | 51 | -8.409 | -4.533 | 31.869 | 1.00 | 37.22 | C |
| ATOM | 2054 | NZ | LYS | B | 51 | -8.655 | -5.999 | 32.014 | 1.00 | 34.08 | N |
| ATOM | 2055 | N | LEU | B | 52 | -11.680 | -6.547 | 29.845 | 1.00 | 24.57 | N |
| ATOM | 2056 | CA | LEU | B | 52 | -12.514 | -7.177 | 30.860 | 1.00 | 28.11 | C |
| ATOM | 2057 | C | LEU | B | 52 | -12.555 | -6.335 | 32.130 | 1.00 | 24.97 | C |
| ATOM | 2058 | O | LEU | B | 52 | -11.514 | -5.962 | 32.663 | 1.00 | 30.29 | O |
| ATOM | 2059 | CB | LEU | B | 52 | -11.998 | -8.583 | 31.172 | 1.00 | 26.30 | C |
| ATOM | 2060 | CG | LEU | B | 52 | -12.775 | -9.420 | 32.190 | 1.00 | 32.39 | c |
| ATOM | 2061 | CD1 | LEU | B | 52 | -14.222 | -9.597 | 31.751 | 1.00 | 25.02 | c |
| ATOM | 2062 | CD2 | LEU | B | 52 | -12.099 | -10.772 | 32.378 | 1.00 | 30.83 | C |
| ATOM | 2063 | N | LEU | B | 53 | -13.760 | -6.030 | 32.600 | 1.00 | 27.61 | N |
| ATOM | 2064 | CA | LEU | B | 53 | -13.938 | -5.297 | 33.854 | 1.00 | 30.52 | C |
| ATOM | 2065 | C | LEU | B | 53 | -14.434 | -6.219 | 34.956 | 1.00 | 31.29 | C |
| ATOM | 2066 | O | LEU | B | 53 | -13.913 | -6.226 | 36.068 | 1.00 | 30.02 | O |
| ATOM | 2067 | CB | LEU | B | 53 | -14.936 | -4.153 | 33.688 | 1.00 | 25.25 | C |
| ATOM | 2068 | CG | LEU | B | 53 | -14.711 | -3.080 | 32.627 | 1.00 | 33.70 | C |
| ATOM | 2069 | CD1 | LEU | B | 53 | -15.917 | -2.149 | 32.611 | 1.00 | 26.80 | C |
| ATOM | 2070 | CD2 | LEU | B | 53 | -13.432 | -2.308 | 32.907 | 1.00 | 32.50 | C |
| ATOM | 2071 | N | LEU | B | 54 | -15.460 | -6.993 | 34.631 | 1.00 | 28.48 | N |
| ATOM | 2072 | CA | LEU | B | 54 | -16.169 | -7.782 | 35.623 | 1.00 | 30.79 | C |
| ATOM | 2073 | C | LEU | B | 54 | -16.516 | -9.152 | 35.071 | 1.00 | 28.54 | C |

(continued)

| | | | | | | | | | | | |
|---|---|---|---|---|---|---|---|---|---|---|---|
| ATOM | 2074 | O | LEU | B | 54 | -16.791 | -9.294 | 33.883 | 1.00 | 26.65 | O |
| ATOM | 2075 | CB | LEU | B | 54 | -17.448 | -7.055 | 36.059 | 1.00 | 31.42 | C |
| ATOM | 2076 | CG | LEU | B | 54 | -17.289 | -5.722 | 36.798 | 1.00 | 33.30 | C |
| ATOM | 2077 | CD1 | LEU | B | 54 | -18.529 | -4.870 | 36.615 | 1.00 | 32.48 | C |
| ATOM | 2078 | CD2 | LEU | B | 54 | -17.052 | -5.971 | 38.268 | 1.00 | 34.07 | C |
| ATOM | 2079 | N | TYR | B | 55 | -16.502 | -10.158 | 35.938 | 1.00 | 29.20 | N |
| ATOM | 2080 | CA | TYR | B | 55 | -17.005 | -11.475 | 35.580 | 1.00 | 29.74 | C |
| ATOM | 2081 | C | TYR | B | 55 | -17.755 | -12.043 | 36.791 | 1.00 | 32.63 | C |
| ATOM | 2082 | O | TYR | B | 55 | -17.726 | -11.449 | 37.872 | 1.00 | 31.32 | O |
| ATOM | 2083 | CB | TYR | B | 55 | -15.869 | -12.393 | 35.101 | 1.00 | 28.86 | C |
| ATOM | 2084 | CG | TYR | B | 55 | -14.714 | -12.558 | 36.065 | 1.00 | 30.07 | C |
| ATOM | 2085 | CD1 | TYR | B | 55 | -13.746 | -11.572 | 36.204 | 1.00 | 31.15 | C |
| ATOM | 2086 | CD2 | TYR | B | 55 | -14.578 | -13.717 | 36.814 | 1.00 | 30.26 | C |
| ATOM | 2087 | CE1 | TYR | B | 55 | -12.686 | -11.728 | 37.088 | 1.00 | 34.78 | C |
| ATOM | 2088 | CE2 | TYR | B | 55 | -13.525 | -13.886 | 37.695 | 1.00 | 33.93 | C |
| ATOM | 2089 | CZ | TYR | B | 55 | -12.582 | -12.889 | 37.828 | 1.00 | 36.44 | C |
| ATOM | 2090 | OH | TYR | B | 55 | -11.534 | -13.059 | 38.705 | 1.00 | 37.42 | O |
| ATOM | 2091 | N | TRP | B | 56 | -18.448 | -13.165 | 36.599 | 1.00 | 28.09 | N |
| ATOM | 2092 | CA | TRP | B | 56 | -19.439 | -13.657 | 37.566 | 1.00 | 26.67 | C |
| ATOM | 2093 | C | TRP | B | 56 | -20.387 | -12.528 | 37.984 | 1.00 | 27.89 | C |
| ATOM | 2094 | O | TRP | B | 56 | -20.767 | -12.420 | 39.151 | 1.00 | 24.63 | O |
| ATOM | 2095 | CB | TRP | B | 56 | -18.769 | -14.276 | 38.799 | 1.00 | 22.01 | C |
| ATOM | 2096 | CG | TRP | B | 56 | -17.799 | -15.367 | 38.462 | 1.00 | 27.09 | C |
| ATOM | 2097 | CD1 | TRP | B | 56 | -16.475 | -15.402 | 38.777 | 1.00 | 26.84 | C |
| ATOM | 2098 | CD2 | TRP | B | 56 | -18.068 | -16.569 | 37.720 | 1.00 | 23.97 | C |
| ATOM | 2099 | NE1 | TRP | B | 56 | -15.901 | -16.551 | 38.286 | 1.00 | 28.86 | N |
| ATOM | 2100 | CE2 | TRP | B | 56 | -16.857 | -17.284 | 37.633 | 1.00 | 25.06 | C |
| ATOM | 2101 | CE3 | TRP | B | 56 | -19.214 | -17.112 | 37.131 | 1.00 | 23.47 | C |
| ATOM | 2102 | CZ2 | TRP | B | 56 | -16.758 | -18.514 | 36.983 | 1.00 | 28.72 | C |
| ATOM | 2103 | CZ3 | TRP | B | 56 | -19.117 | -18.334 | 36.486 | 1.00 | 28.66 | C |
| ATOM | 2104 | CH2 | TRP | B | 56 | -17.896 | -19.021 | 36.414 | 1.00 | 29.03 | C |
| ATOM | 2105 | N | ALA | B | 57 | -20.734 | -11.686 | 37.009 | 1.00 | 26.41 | N |
| ATOM | 2106 | CA | ALA | B | 57 | -21.626 | -10.531 | 37.167 | 1.00 | 29.78 | C |
| ATOM | 2107 | C | ALA | B | 57 | -21.083 | -9.421 | 38.079 | 1.00 | 31.22 | C |

(continued)

| | | | | | | | | | | | |
|---|---|---|---|---|---|---|---|---|---|---|---|
| ATOM | 2108 | O | ALA | B | 57 | -21.331 | -8.242 | 37.817 | 1.00 | 35.13 | O |
| ATOM | 2109 | CB | ALA | B | 57 | -23.008 | -10.985 | 37.667 | 1.00 | 27.19 | C |
| ATOM | 2110 | N | SER | B | 58 | -20.344 | -9.767 | 39.132 | 1.00 | 32.27 | N |
| ATOM | 2111 | CA | SER | B | 58 | -19.992 | -8.748 | 40.125 | 1.00 | 31.56 | C |
| ATOM | 2112 | C | SER | B | 58 | -18.563 | -8.741 | 40.672 | 1.00 | 32.07 | C |
| ATOM | 2113 | O | SER | B | 58 | -18.251 | -7.901 | 41.517 | 1.00 | 37.30 | O |
| ATOM | 2114 | CB | SER | B | 58 | -20.955 | -8.849 | 41.315 | 1.00 | 36.25 | C |
| ATOM | 2115 | OG | SER | B | 58 | -20.817 | -10.091 | 41.985 | 1.00 | 37.37 | O |
| ATOM | 2116 | N | THR | B | 59 | -17.687 | -9.640 | 40.229 | 1.00 | 31.17 | N |
| ATOM | 2117 | CA | THR | B | 59 | -16.319 | -9.596 | 40.759 | 1.00 | 29.77 | C |
| ATOM | 2118 | C | THR | B | 59 | -15.365 | -8.910 | 39.770 | 1.00 | 30.97 | C |
| ATOM | 2119 | O | THR | B | 59 | -15.367 | -9.201 | 38.575 | 1.00 | 31.74 | O |
| ATOM | 2120 | CB | THR | B | 59 | -15.786 | -11.014 | 41.142 | 1.00 | 36.77 | C |
| ATOM | 2121 | OG1 | THR | B | 59 | -14.721 | -11.403 | 40.269 | 1.00 | 41.80 | O |
| ATOM | 2122 | CG2 | THR | B | 59 | -16.890 | -12.053 | 41.102 | 1.00 | 28.98 | C |
| ATOM | 2123 | N | ARG | B | 60 | -14.565 | -7.978 | 40.284 | 1.00 | 32.62 | N |
| ATOM | 2124 | CA | ARG | B | 60 | -13.693 | -7.159 | 39.449 | 1.00 | 36.43 | C |
| ATOM | 2125 | C | ARG | B | 60 | -12.441 | -7.902 | 39.022 | 1.00 | 35.37 | C |
| ATOM | 2126 | O | ARG | B | 60 | -11.819 | -8.588 | 39.825 | 1.00 | 33.08 | O |
| ATOM | 2127 | CB | ARG | B | 60 | -13.278 | -5.877 | 40.182 | 1.00 | 33.09 | C |
| ATOM | 2128 | CG | ARG | B | 60 | -14.342 | -4.796 | 40.212 | 1.00 | 39.07 | C |
| ATOM | 2129 | CD | ARG | B | 60 | -13.752 | -3.436 | 40.558 | 1.00 | 37.50 | C |
| ATOM | 2130 | NE | ARG | B | 60 | -12.854 | -3.495 | 41.707 | 1.00 | 43.68 | N |
| ATOM | 2131 | CZ | ARG | B | 60 | -13.259 | -3.551 | 42.971 | 1.00 | 51.03 | C |
| ATOM | 2132 | NH1 | ARG | B | 60 | -14.555 | -3.560 | 43.254 | 1.00 | 53.34 | N |
| ATOM | 2133 | NH2 | ARG | B | 60 | -12.368 | -3.603 | 43.952 | 1.00 | 52.19 | N |
| ATOM | 2134 | N | GLU | B | 61 | -12.061 | -7.747 | 37.759 | 1.00 | 30.40 | N |
| ATOM | 2135 | CA | GLU | B | 61 | -10.764 | -8.235 | 37.310 | 1.00 | 35.70 | C |
| ATOM | 2136 | C | GLU | B | 61 | -9.659 | -7.523 | 38.077 | 1.00 | 35.51 | C |
| ATOM | 2137 | O | GLU | B | 61 | -9.863 | -6.424 | 38.598 | 1.00 | 36.52 | O |
| ATOM | 2138 | CB | GLU | B | 61 | -10.582 | -8.023 | 35.805 | 1.00 | 30.95 | C |
| ATOM | 2139 | CG | GLU | B | 61 | -10.406 | -9.307 | 35.015 | 1.00 | 51.01 | C |
| ATOM | 2140 | CD | GLU | B | 61 | -9.084 | -10.015 | 35.278 | 1.00 | 47.16 | C |
| ATOM | 2141 | OE1 | GLU | B | 61 | -8.884 | -10.535 | 36.397 | 1.00 | 57.21 | O |

(continued)

| | | | | | | | | | | |
|---|---|---|---|---|---|---|---|---|---|---|
| ATOM | 2142 | OE2 | GLU | B | 61 | -8.245 | -10.063 | 34.354 | 1.00 | 55.45 | O |
| ATOM | 2143 | N | SER | B | 62 | -8.491 | -8.148 | 38.143 | 1.00 | 36.53 | N |
| ATOM | 2144 | CA | SER | B | 62 | -7.352 | -7.559 | 38.838 | 1.00 | 37.86 | C |
| ATOM | 2145 | C | SER | B | 62 | -6.959 | -6.222 | 38.211 | 1.00 | 35.62 | C |
| ATOM | 2146 | O | SER | B | 62 | -6.919 | -6.086 | 36.992 | 1.00 | 34.27 | O |
| ATOM | 2147 | CB | SER | B | 62 | -6.166 | -8.527 | 38.822 | 1.00 | 40.55 | C |
| ATOM | 2148 | OG | SER | B | 62 | -5.113 | -8.060 | 39.646 | 1.00 | 56.20 | O |
| ATOM | 2149 | N | GLY | B | 63 | -6.690 | -5.228 | 39.049 | 1.00 | 32.40 | N |
| ATOM | 2150 | CA | GLY | B | 63 | -6.280 | -3.924 | 38.561 | 1.00 | 32.97 | C |
| ATOM | 2151 | C | GLY | B | 63 | -7.413 | -2.971 | 38.213 | 1.00 | 38.33 | C |
| ATOM | 2152 | O | GLY | B | 63 | -7.194 | -1.767 | 38.099 | 1.00 | 40.50 | O |
| ATOM | 2153 | N | VAL | B | 64 | -8.624 | -3.498 | 38.045 | 1.00 | 31.70 | N |
| ATOM | 2154 | CA | VAL | B | 64 | -9.780 | -2.664 | 37.711 | 1.00 | 30.42 | C |
| ATOM | 2155 | C | VAL | B | 64 | -10.188 | -1.782 | 38.894 | 1.00 | 34.52 | C |
| ATOM | 2156 | O | VAL | B | 64 | -10.378 | -2.279 | 40.004 | 1.00 | 36.24 | O |
| ATOM | 2157 | CB | VAL | B | 64 | -10.988 | -3.525 | 37.269 | 1.00 | 33.44 | C |
| ATOM | 2158 | CG1 | VAL | B | 64 | -12.241 | -2.668 | 37.101 | 1.00 | 31.47 | C |
| ATOM | 2159 | CG2 | VAL | B | 64 | -10.668 | -4.271 | 35.985 | 1.00 | 30.34 | C |
| ATOM | 2160 | N | PRO | B | 65 | -10.326 | -0.466 | 38.655 | 1.00 | 35.36 | N |
| ATOM | 2161 | CA | PRO | B | 65 | -10.693 | 0.500 | 39.701 | 1.00 | 33.81 | C |
| ATOM | 2162 | C | PRO | B | 65 | -12.039 | 0.170 | 40.344 | 1.00 | 36.63 | C |
| ATOM | 2163 | O | PRO | B | 65 | -12.940 | -0.301 | 39.649 | 1.00 | 30.63 | O |
| ATOM | 2164 | CB | PRO | B | 65 | -10.765 | 1.831 | 38.946 | 1.00 | 31.15 | C |
| ATOM | 2165 | CG | PRO | B | 65 | -9.947 | 1.623 | 37.710 | 1.00 | 35.40 | C |
| ATOM | 2166 | CD | PRO | B | 65 | -10.131 | 0.182 | 37.347 | 1.00 | 33.17 | C |
| ATOM | 2167 | N | ASP | B | 66 | -12.177 | 0.421 | 41.645 | 1.00 | 33.02 | N |
| ATOM | 2168 | CA | ASP | B | 66 | -13.408 | 0.069 | 42.357 | 1.00 | 39.73 | C |
| ATOM | 2169 | C | ASP | B | 66 | -14.599 | 0.965 | 41.998 | 1.00 | 39.77 | C |
| ATOM | 2170 | O | ASP | B | 66 | -15.703 | 0.752 | 42.499 | 1.00 | 35.02 | O |
| ATOM | 2171 | CB | ASP | B | 66 | -13.180 | 0.093 | 43.874 | 1.00 | 38.87 | C |
| ATOM | 2172 | CG | ASP | B | 66 | -12.527 | 1.374 | 44.354 | 1.00 | 46.60 | C |
| ATOM | 2173 | OD1 | ASP | B | 66 | -12.535 | 2.376 | 43.608 | 1.00 | 57.95 | O |
| ATOM | 2174 | OD2 | ASP | B | 66 | -12.004 | 1.377 | 45.489 | 1.00 | 64.41 | O |
| ATOM | 2175 | N | ARG | B | 67 | -14.376 | 1.953 | 41.129 | 1.00 | 35.35 | N |

(continued)

| | | | | | | | | | | | |
|---|---|---|---|---|---|---|---|---|---|---|---|
| ATOM | 2176 | CA | ARG | B | 67 | -15.470 | 2.752 | 40.569 | 1.00 | 31.85 | C |
| ATOM | 2177 | C | ARG | B | 67 | -16.456 | 1.880 | 39.807 | 1.00 | 34.69 | C |
| ATOM | 2178 | O | ARG | B | 67 | -17.638 | 2.209 | 39.698 | 1.00 | 35.56 | O |
| ATOM | 2179 | CB | ARG | B | 67 | -14.947 | 3.828 | 39.614 | 1.00 | 39.18 | C |
| ATOM | 2180 | CG | ARG | B | 67 | -13.752 | 4.611 | 40.095 | 1.00 | 51. 83 | C |
| ATOM | 2181 | CD | ARG | B | 67 | -13.589 | 5.861 | 39.242 | 1.00 | 57.84 | C |
| ATOM | 2182 | NE | ARG | B | 67 | -13.490 | 5.570 | 37.810 | 1.00 | 38.84 | N |
| ATOM | 2183 | CZ | ARG | B | 67 | -12.335 | 5.389 | 37.179 | 1.00 | 42.20 | C |
| ATOM | 2184 | NH1 | ARG | B | 67 | -11.197 | 5.458 | 37.859 | 1.00 | 41.09 | N |
| ATOM | 2185 | NH2 | ARG | B | 67 | -12.314 | 5.140 | 35.878 | 1.00 | 37.06 | N |
| ATOM | 2186 | N | PHE | B | 68 | -15.948 | 0.785 | 39.245 | 1.00 | 28.82 | N |
| ATOM | 2187 | CA | PHE | B | 68 | -16.759 | -0.122 | 38.443 | 1.00 | 36.23 | C |
| ATOM | 2188 | C | PHE | B | 68 | -17.314 | -1.232 | 39.308 | 1.00 | 34.50 | C |
| ATOM | 2189 | O | PHE | B | 68 | -16.564 | -1.931 | 39.995 | 1.00 | 31.34 | O |
| ATOM | 2190 | CB | PHE | B | 68 | -15.941 | -0.727 | 37.297 | 1.00 | 30.38 | C |
| ATOM | 2191 | CG | PHE | B | 68 | -15.401 | 0.287 | 36.337 | 1.00 | 34.41 | C |
| ATOM | 2192 | CD1 | PHE | B | 68 | -16.127 | 0.651 | 35.215 | 1.00 | 31.80 | C |
| ATOM | 2193 | CD2 | PHE | B | 68 | -14.163 | 0.874 | 36.551 | 1.00 | 34.95 | C |
| ATOM | 2194 | CE1 | PHE | B | 68 | -15.629 | 1.586 | 34.328 | 1.00 | 29.96 | C |
| ATOM | 2195 | CE2 | PHE | B | 68 | -13.661 | 1.807 | 35.666 | 1.00 | 35.32 | C |
| ATOM | 2196 | CZ | PHE | B | 68 | -14.396 | 2.163 | 34.554 | 1.00 | 31.86 | C |
| ATOM | 2197 | N | SER | B | 69 | -18.627 | -1.408 | 39.275 | 1.00 | 30.82 | N |
| ATOM | 2198 | CA | SER | B | 69 | -19.228 | -2.482 | 40.044 | 1.00 | 30.92 | C |
| ATOM | 2199 | C | SER | B | 69 | -20.364 | -3.114 | 39.268 | 1.00 | 34.45 | C |
| ATOM | 2200 | O | SER | B | 69 | -20.998 | -2.468 | 38.430 | 1.00 | 35.02 | O |
| ATOM | 2201 | CB | SER | B | 69 | -19.724 | -1.970 | 41.397 | 1.00 | 32.49 | c |
| ATOM | 2202 | OG | SER | B | 69 | -20.797 | -1.062 | 41.230 | 1.00 | 37.05 | O |
| ATOM | 2203 | N | GLY | B | 70 | -20.610 | -4.387 | 39.550 | 1.00 | 30.93 | N |
| ATOM | 2204 | CA | GLY | B | 70 | -21.673 | -5.116 | 38.896 | 1.00 | 28.03 | C |
| ATOM | 2205 | C | GLY | B | 70 | -22.689 | -5.620 | 39.898 | 1.00 | 38.68 | C |
| ATOM | 2206 | O | GLY | B | 70 | -22.361 | -5.887 | 41.056 | 1.00 | 36.13 | O |
| ATOM | 2207 | N | SER | B | 71 | -23.932 | -5.737 | 39.449 | 1.00 | 35.97 | N |
| ATOM | 2208 | CA | SER | B | 71 | -24.996 | -6.282 | 40.276 | 1.00 | 32.81 | C |
| ATOM | 2209 | C | SER | B | 71 | -26.038 | -6.927 | 39.380 | 1.00 | 36.09 | C |

(continued)

| | | | | | | | | | | | |
|---|---|---|---|---|---|---|---|---|---|---|---|
| ATOM | 2210 | O | SER | B | 71 | -25.980 | -6.798 | 38.157 | 1.00 | 36.12 | O |
| ATOM | 2211 | CB | SER | B | 71 | -25.632 | -5.193 | 41.136 | 1.00 | 38.54 | C |
| ATOM | 2212 | OG | SER | B | 71 | -26.284 | -4.232 | 40.322 | 1.00 | 42.67 | O |
| ATOM | 2213 | N | GLY | B | 72 | -26.996 | -7.612 | 39.992 | 1.00 | 35.53 | N |
| ATOM | 2214 | CA | GLY | B | 72 | -28.054 | -8.256 | 39.242 | 1.00 | 32.26 | C |
| ATOM | 2215 | C | GLY | B | 72 | -28.025 | -9.760 | 39.411 | 1.00 | 33.08 | C |
| ATOM | 2216 | O | GLY | B | 72 | -27.109 | -10.312 | 40.020 | 1.00 | 37.03 | O |
| ATOM | 2217 | N | SER | B | 73 | -29.038 | -10.420 | 38.869 | 1.00 | 35.95 | N |
| ATOM | 2218 | CA | SER | B | 73 | -29.166 | -11.861 | 38.993 | 1.00 | 39.04 | C |
| ATOM | 2219 | C | SER | B | 73 | -30.132 | -12.395 | 37.946 | 1.00 | 40.07 | C |
| ATOM | 2220 | O | SER | B | 73 | -31.042 | -11.691 | 37.512 | 1.00 | 40.54 | O |
| ATOM | 2221 | CB | SER | B | 73 | -29.643 | -12.235 | 40.398 | 1.00 | 44.78 | C |
| ATOM | 2222 | OG | SER | B | 73 | -29.753 | -13.638 | 40.538 | 1.00 | 50.73 | O |
| ATOM | 2223 | N | GLY | B | 74 | -29.920 | -13.638 | 37.531 | 1.00 | 41. 84 | N |
| ATOM | 2224 | CA | GLY | B | 74 | -30.816 | -14.291 | 36.597 | 1.00 | 37.63 | C |
| ATOM | 2225 | C | GLY | B | 74 | -30.776 | -13.743 | 35.182 | 1.00 | 41.67 | C |
| ATOM | 2226 | O | GLY | B | 74 | -29.999 | -14.211 | 34.347 | 1.00 | 37.99 | O |
| ATOM | 2227 | N | THR | B | 75 | -31.628 | -12.761 | 34.907 | 1.00 | 34.80 | N |
| ATOM | 2228 | CA | THR | B | 75 | -31.767 | -12.239 | 33.553 | 1.00 | 38.56 | C |
| ATOM | 2229 | C | THR | B | 75 | -31.515 | -10.733 | 33.475 | 1.00 | 38.78 | C |
| ATOM | 2230 | O | THR | B | 75 | -31.439 | -10.173 | 32.384 | 1.00 | 44.01 | O |
| ATOM | 2231 | CB | THR | B | 75 | -33.176 | -12.534 | 32.980 | 1.00 | 38.87 | C |
| ATOM | 2232 | CG2 | THR | B | 75 | -33.477 | -14.025 | 33.021 | 1.00 | 37.18 | C |
| ATOM | 2233 | OG1 | THR | B | 75 | -34.163 | -11.838 | 33.749 | 1.00 | 46.98 | O |
| ATOM | 2234 | N | ASP | B | 76 | -31.389 | -10.083 | 34.629 | 1.00 | 37.55 | N |
| ATOM | 2235 | CA | ASP | B | 76 | -31.244 | -8.628 | 34.678 | 1.00 | 38.55 | C |
| ATOM | 2236 | C | ASP | B | 76 | -29.989 | -8.211 | 35.414 | 1.00 | 30.72 | C |
| ATOM | 2237 | O | ASP | B | 76 | -29.767 | -8.605 | 36.557 | 1.00 | 39.41 | O |
| ATOM | 2238 | CB | ASP | B | 76 | -32.463 | -7.978 | 35.340 | 1.00 | 39.46 | C |
| ATOM | 2239 | CG | ASP | B | 76 | -33.567 | -7.680 | 34.352 | 1.00 | 54.19 | C |
| ATOM | 2240 | OD2 | ASP | B | 76 | -34.444 | -8.548 | 34.149 | 1.00 | 61.43 | O |
| ATOM | 2241 | OD1 | ASP | B | 76 | -33.553 | -6.575 | 33.770 | 1.00 | 68.17 | O |
| ATOM | 2242 | N | PHE | B | 77 | -29.173 | -7.397 | 34.756 | 1.00 | 32.93 | N |
| ATOM | 2243 | CA | PHE | B | 77 | -27.890 | -6.999 | 35.314 | 1.00 | 34.16 | C |

(continued)

| | | | | | | | | | | | |
|---|---|---|---|---|---|---|---|---|---|---|---|
| ATOM | 2244 | C | PHE | B | 77 | -27.608 | -5.540 | 35.021 | 1.00 | 28.32 | C |
| ATOM | 2245 | O | PHE | B | 77 | -28.054 | -5.005 | 34.010 | 1.00 | 34.43 | O |
| ATOM | 2246 | CB | PHE | B | 77 | -26.769 | -7.870 | 34.748 | 1.00 | 33.65 | C |
| ATOM | 2247 | CG | PHE | B | 77 | -27.008 | -9.340 | 34.908 | 1.00 | 31.73 | C |
| ATOM | 2248 | CD1 | PHE | B | 77 | -26.584 | -10.004 | 36.050 | 1.00 | 34.33 | C |
| ATOM | 2249 | CD2 | PHE | B | 77 | -27.663 | -10.061 | 33.924 | 1.00 | 30.60 | C |
| ATOM | 2250 | CE1 | PHE | B | 77 | -26.807 | -11.363 | 36.204 | 1.00 | 28.94 | C |
| ATOM | 2251 | CE2 | PHE | B | 77 | -27.892 | -11.420 | 34.078 | 1.00 | 32.18 | C |
| ATOM | 2252 | CZ | PHE | B | 77 | -27.463 | -12.067 | 35.215 | 1.00 | 28.31 | C |
| ATOM | 2253 | N | THR | B | 78 | -26.864 | -4 . 894 | 35.909 | 1.00 | 33.91 | N |
| ATOM | 2254 | CA | THR | B | 78 | -26.454 | -3.520 | 35.673 | 1.00 | 36.80 | C |
| ATOM | 2255 | C | THR | B | 78 | -24.972 | -3.332 | 35.956 | 1.00 | 35.53 | C |
| ATOM | 2256 | O | THR | B | 78 | -24.429 | -3.893 | 36.909 | 1.00 | 33.42 | O |
| ATOM | 2257 | CB | THR | B | 78 | -27.250 | -2.514 | 36.536 | 1.00 | 36.13 | C |
| ATOM | 2258 | CG2 | THR | B | 78 | -28.744 | -2.790 | 36.465 | 1.00 | 35.39 | C |
| ATOM | 2259 | OG1 | THR | B | 78 | -26.815 | -2.600 | 37.896 | 1.00 | 52.08 | O |
| ATOM | 2260 | N | LEU | B | 79 | -24.321 | -2.546 | 35.108 | 1.00 | 33.37 | N |
| ATOM | 2261 | CA | LEU | B | 79 | -22.978 | -2.072 | 35.382 | 1.00 | 32.04 | C |
| ATOM | 2262 | C | LEU | B | 79 | -23.082 | -0.689 | 36.000 | 1.00 | 35.99 | C |
| ATOM | 2263 | O | LEU | B | 79 | -23.805 | 0.166 | 35.494 | 1.00 | 30.23 | O |
| ATOM | 2264 | CB | LEU | B | 79 | -22.138 | -2.030 | 34.105 | 1.00 | 32.75 | C |
| ATOM | 2265 | CG | LEU | B | 79 | -20.814 | -1.269 | 34.215 | 1.00 | 31.00 | C |
| ATOM | 2266 | CD1 | LEU | B | 79 | -19.875 | -1.948 | 35.189 | 1.00 | 30.67 | C |
| ATOM | 2267 | CD2 | LEU | B | 79 | -20.151 | -1.129 | 32.853 | 1.00 | 29.83 | C |
| ATOM | 2268 | N | THR | B | 80 | -22.379 | -0.467 | 37.102 | 1.00 | 37.73 | N |
| ATOM | 2269 | CA | THR | B | 80 | -22.390 | 0.852 | 37.711 | 1.00 | 37.01 | C |
| ATOM | 2270 | C | THR | B | 80 | -20.999 | 1.468 | 37.726 | 1.00 | 37.89 | C |
| ATOM | 2271 | O | THR | B | 80 | -20.044 | 0.876 | 38.229 | 1.00 | 33.52 | O |
| ATOM | 2272 | CB | THR | B | 80 | -22.938 | 0.816 | 39.152 | 1.00 | 47.22 | C |
| ATOM | 2273 | CG2 | THR | B | 80 | -22.815 | 2.194 | 39.802 | 1.00 | 42.48 | C |
| ATOM | 2274 | OG1 | THR | B | 80 | -24.316 | 0.427 | 39.130 | 1.00 | 44.17 | O |
| ATOM | 2275 | N | ILE | B | 81 | -20.893 | 2.659 | 37.152 | 1.00 | 36.66 | N |
| ATOM | 2276 | CA | ILE | B | 81 | -19.699 | 3.464 | 37.321 | 1.00 | 35.94 | C |
| ATOM | 2277 | C | ILE | B | 81 | -20.013 | 4.576 | 38.308 | 1.00 | 41.22 | C |

(continued)

| | | | | | | | | | | | |
|---|---|---|---|---|---|---|---|---|---|---|---|
| ATOM | 2278 | O | ILE | B | 81 | -20.729 | 5.527 | 37.984 | 1.00 | 39.45 | O |
| ATOM | 2279 | CB | ILE | B | 81 | -19.206 | 4. 068 | 35.997 | 1.00 | 29.74 | C |
| ATOM | 2280 | CG1 | ILE | B | 81 | -19.077 | 2.986 | 34.927 | 1.00 | 31.37 | C |
| ATOM | 2281 | CG2 | ILE | B | 81 | -17 . 875 | 4.784 | 36.204 | 1.00 | 37.10 | C |
| ATOM | 2282 | CD1 | ILE | B | 81 | -18.674 | 3.527 | 33.563 | 1.00 | 36.05 | C |
| ATOM | 2283 | N | SER | B | 82 | -19.506 | 4.442 | 39.525 | 1.00 | 36.61 | N |
| ATOM | 2284 | CA | SER | B | 82 | -19.621 | 5.521 | 40.489 | 1.00 | 47.92 | C |
| ATOM | 2285 | C | SER | B | 82 | -18.478 | 6.491 | 40.221 | 1.00 | 54.74 | C |
| ATOM | 2286 | O | SER | B | 82 | -17.328 | 6.069 | 40.079 | 1.00 | 68.40 | O |
| ATOM | 2287 | CB | SER | B | 82 | -19.571 | 4.991 | 41.921 | 1.00 | 38.83 | C |
| ATOM | 2288 | OG | SER | B | 82 | -18.240 | 4.674 | 42.287 | 1.00 | 41.65 | O |
| ATOM | 2289 | N | SER | B | 83 | -18.803 | 7.776 | 40.124 | 1.00 | 52.26 | N |
| ATOM | 2290 | CA | SER | B | 83 | -17.818 | 8.825 | 39.853 | 1.00 | 47.12 | C |
| ATOM | 2291 | C | SER | B | 83 | -17.144 | 8.663 | 38.481 | 1.00 | 47.93 | C |
| ATOM | 2292 | O | SER | B | 83 | -15.999 | 8.217 | 38.381 | 1.00 | 37.39 | O |
| ATOM | 2293 | CB | SER | B | 83 | -16.763 | 8.864 | 40.962 | 1.00 | 42.74 | C |
| ATOM | 2294 | OG | SER | B | 83 | -15.929 | 10.003 | 40.831 | 1.00 | 61.34 | O |
| ATOM | 2295 | N | LEU | B | 84 | -17.862 | 9.054 | 37.432 | 1.00 | 39.91 | N |
| ATOM | 2296 | CA | LEU | B | 84 | -17.373 | 8.932 | 36.061 | 1.00 | 40.35 | C |
| ATOM | 2297 | C | LEU | B | 84 | -16.120 | 9.777 | 35.825 | 1.00 | 43.92 | C |
| ATOM | 2298 | O | LEU | B | 84 | -16.079 | 10.954 | 36.188 | 1.00 | 32.91 | O |
| ATOM | 2299 | CB | LEU | B | 84 | -18.470 | 9.334 | 35.072 | 1.00 | 36.00 | C |
| ATOM | 2300 | CG | LEU | B | 84 | -18.281 | 8.902 | 33.616 | 1.00 | 40.11 | C |
| ATOM | 2301 | CD1 | LEU | B | 84 | -18.455 | 7.396 | 33.472 | 1.00 | 34.46 | C |
| ATOM | 2302 | CD2 | LEU | B | 84 | -19.246 | 9.639 | 32.702 | 1.00 | 39.38 | C |
| ATOM | 2303 | N | GLN | B | 85 | -15.103 | 9.166 | 35.221 | 1.00 | 35.37 | N |
| ATOM | 2304 | CA | GLN | B | 85 | -13.839 | 9.844 | 34.940 | 1.00 | 35.03 | C |
| ATOM | 2305 | C | GLN | B | 85 | -13.682 | 10.088 | 33.432 | 1.00 | 35.85 | C |
| ATOM | 2306 | O | GLN | B | 85 | -14.311 | 9.400 | 32.627 | 1.00 | 30.69 | O |
| ATOM | 2307 | CB | GLN | B | 85 | -12.665 | 9.023 | 35.484 | 1.00 | 33.69 | C |
| ATOM | 2308 | CG | GLN | B | 85 | -12.719 | 8.782 | 36.990 | 1.00 | 39.42 | C |
| ATOM | 2309 | CD | GLN | B | 85 | -12.651 | 10.069 | 37.795 | 1.00 | 43.01 | C |
| ATOM | 2310 | NE2 | GLN | B | 85 | -13.732 | 10.386 | 38.500 | 1.00 | 38.02 | N |
| ATOM | 2311 | OE1 | GLN | B | 85 | -11.640 | 10.772 | 37.778 | 1.00 | 40.65 | O |

(continued)

| | | | | | | | | | | | |
|---|---|---|---|---|---|---|---|---|---|---|---|
| ATOM | 2312 | N | PRO | B | 86 | -12.857 | 11.082 | 33.046 | 1.00 | 33.28 | N |
| ATOM | 2313 | CA | PRO | B | 86 | -12.643 | 11.413 | 31.631 | 1.00 | 31.64 | C |
| ATOM | 2314 | C | PRO | B | 86 | -12.220 | 10.209 | 30.792 | 1.00 | 35.32 | C |
| ATOM | 2315 | O | PRO | B | 86 | -12.635 | 10.093 | 29.641 | 1.00 | 30.64 | O |
| ATOM | 2316 | CB | PRO | B | 86 | -11.520 | 12.456 | 31.681 | 1.00 | 34.31 | C |
| ATOM | 2317 | CG | PRO | B | 86 | -11.690 | 13.108 | 33.001 | 1.00 | 42.02 | C |
| ATOM | 2318 | CD | PRO | B | 86 | -12.146 | 12.020 | 33.936 | 1.00 | 35.55 | C |
| ATOM | 2319 | N | GLU | B | 87 | -11.423 | 9.316 | 31.373 | 1.00 | 32.17 | N |
| ATOM | 2320 | CA | GLU | B | 87 | -10.921 | 8.161 | 30.637 | 1.00 | 31.69 | C |
| ATOM | 2321 | C | GLU | B | 87 | -11.975 | 7.063 | 30.470 | 1.00 | 31.13 | C |
| ATOM | 2322 | O | GLU | B | 87 | -11.744 | 6.086 | 29.761 | 1.00 | 31. 02 | O |
| ATOM | 2323 | CB | GLU | B | 87 | -9.681 | 7.583 | 31.327 | 1.00 | 32.83 | C |
| ATOM | 2324 | CG | GLU | B | 87 | -9.941 | 6.983 | 32.703 | 1.00 | 34.70 | C |
| ATOM | 2325 | CD | GLU | B | 87 | -9.628 | 7.950 | 33.834 | 1.00 | 47.47 | C |
| ATOM | 2326 | OE1 | GLU | B | 87 | -9.890 | 9.166 | 33.681 | 1.00 | 39.21 | O |
| ATOM | 2327 | OE2 | GLU | B | 87 | -9.111 | 7.486 | 34 . 876 | 1.00 | 49.29 | O |
| ATOM | 2328 | N | ASP | B | 88 | -13.131 | 7.227 | 31.108 | 1.00 | 28.67 | N |
| ATOM | 2329 | CA | ASP | B | 88 | -14.175 | 6.210 | 31.053 | 1.00 | 29.26 | C |
| ATOM | 2330 | C | ASP | B | 88 | -15.032 | 6.315 | 29.787 | 1.00 | 31.41 | C |
| ATOM | 2331 | O | ASP | B | 88 | -15.961 | 5.532 | 29.602 | 1.00 | 31. 09 | O |
| ATOM | 2332 | CB | ASP | B | 88 | -15.070 | 6.293 | 32.295 | 1.00 | 32.59 | C |
| ATOM | 2333 | CG | ASP | B | 88 | -14.287 | 6.153 | 33.595 | 1.00 | 39.45 | C |
| ATOM | 2334 | OD1 | ASP | B | 88 | -13.166 | 5.597 | 33.565 | 1.00 | 34.75 | O |
| ATOM | 2335 | OD2 | ASP | B | 88 | -14.794 | 6.595 | 34.650 | 1.00 | 40.00 | O |
| ATOM | 2336 | N | VAL | B | 89 | -14.725 | 7.276 | 28.920 | 1.00 | 27.74 | N |
| ATOM | 2337 | CA | VAL | B | 89 | -15.437 | 7.395 | 27.648 | 1.00 | 29.97 | C |
| ATOM | 2338 | C | VAL | B | 89 | -15.162 | 6.169 | 26.783 | 1.00 | 26.75 | C |
| ATOM | 2339 | O | VAL | B | 89 | -14.020 | 5.928 | 26.391 | 1.00 | 32.52 | O |
| ATOM | 2340 | CB | VAL | B | 89 | -15.029 | 8.662 | 26.862 | 1.00 | 36.47 | C |
| ATOM | 2341 | CG1 | VAL | B | 89 | -15.785 | 8.732 | 25.538 | 1.00 | 36.88 | C |
| ATOM | 2342 | CG2 | VAL | B | 89 | -15.281 | 9.908 | 27.681 | 1.00 | 41.88 | C |
| ATOM | 2343 | N | ALA | B | 90 | -16.209 | 5.410 | 26.479 | 1.00 | 25.10 | N |
| ATOM | 2344 | CA | ALA | B | 90 | -16.063 | 4.149 | 25.764 | 1.00 | 28.51 | C |
| ATOM | 2345 | C | ALA | B | 90 | -17.410 | 3.508 | 25.491 | 1.00 | 30.70 | C |

(continued)

| | | | | | | | | | | | |
|---|---|---|---|---|---|---|---|---|---|---|---|
| ATOM | 2346 | O | ALA | B | 90 | -18.448 | 3.995 | 25.943 | 1.00 | 27.64 | O |
| ATOM | 2347 | CB | ALA | B | 90 | -15.185 | 3.178 | 26.561 | 1.00 | 30.46 | C |
| ATOM | 2348 | N | VAL | B | 91 | -17.377 | 2.401 | 24.755 | 1.00 | 29.92 | N |
| ATOM | 2349 | CA | VAL | B | 91 | -18.539 | 1.542 | 24.606 | 1.00 | 28.27 | C |
| ATOM | 2350 | C | VAL | B | 91 | -18.425 | 0.364 | 25.573 | 1.00 | 33.48 | C |
| ATOM | 2351 | O | VAL | B | 91 | -17.368 | -0.265 | 25.684 | 1.00 | 25.89 | O |
| ATOM | 2352 | CB | VAL | B | 91 | -18.683 | 1.024 | 23.172 | 1.00 | 28.97 | C |
| ATOM | 2353 | CG1 | VAL | B | 91 | -19.940 | 0.178 | 23.045 | 1.00 | 28.55 | C |
| ATOM | 2354 | CG2 | VAL | B | 91 | -18.723 | 2.194 | 22.196 | 1.00 | 29.32 | C |
| ATOM | 2355 | N | TYR | B | 92 | -19.511 | 0.077 | 26.281 | 1.00 | 29.51 | N |
| ATOM | 2356 | CA | TYR | B | 92 | -19.509 | -0.993 | 27.267 | 1.00 | 29.47 | C |
| ATOM | 2357 | C | TYR | B | 92 | -20.379 | -2.148 | 26.793 | 1.00 | 33.18 | C |
| ATOM | 2358 | O | TYR | B | 92 | -21.477 | -1.945 | 26.267 | 1.00 | 29.29 | O |
| ATOM | 2359 | CB | TYR | B | 92 | -19.978 | -0.472 | 28.630 | 1.00 | 23.56 | C |
| ATOM | 2360 | CG | TYR | B | 92 | -19.010 | 0.510 | 29.254 | 1.00 | 27.95 | C |
| ATOM | 2361 | CD2 | TYR | B | 92 | -18.114 | 0.104 | 30.236 | 1.00 | 31.49 | C |
| ATOM | 2362 | CD1 | TYR | B | 92 | -18.978 | 1.841 | 28.848 | 1.00 | 28.41 | C |
| ATOM | 2363 | CE2 | TYR | B | 92 | -17.222 | 0.995 | 30.808 | 1.00 | 26.89 | C |
| ATOM | 2364 | CE1 | TYR | B | 92 | -18.086 | 2.740 | 29.411 | 1.00 | 25.28 | C |
| ATOM | 2365 | CZ | TYR | B | 92 | -17.213 | 2.309 | 30.387 | 1.00 | 27.09 | C |
| ATOM | 2366 | OH | TYR | B | 92 | -16.325 | 3.190 | 30.940 | 1.00 | 27.92 | O |
| ATOM | 2367 | N | TYR | B | 93 | -19.863 | -3.361 | 26.962 | 1.00 | 27.67 | N |
| ATOM | 2368 | CA | TYR | B | 93 | -20.548 | -4.569 | 26.524 | 1.00 | 26.66 | C |
| ATOM | 2369 | C | TYR | B | 93 | -20.725 | -5.548 | 27.673 | 1.00 | 29.56 | C |
| ATOM | 2370 | O | TYR | B | 93 | -19.802 | -5.759 | 28.458 | 1.00 | 27.75 | O |
| ATOM | 2371 | CB | TYR | B | 93 | -19.765 | -5.270 | 25.412 | 1.00 | 25.28 | C |
| ATOM | 2372 | CG | TYR | B | 93 | -19.615 | -4.503 | 24.125 | 1.00 | 28.54 | C |
| ATOM | 2373 | CD2 | TYR | B | 93 | -20.529 | -4.662 | 23.090 | 1.00 | 26.88 | C |
| ATOM | 2374 | CD1 | TYR | B | 93 | -18.540 | -3.644 | 23.928 | 1.00 | 27.13 | C |
| ATOM | 2375 | CE2 | TYR | B | 93 | -20.386 | -3.973 | 21.899 | 1.00 | 32.89 | C |
| ATOM | 2376 | CE1 | TYR | B | 93 | -18.385 | -2.955 | 22.743 | 1.00 | 29.51 | C |
| ATOM | 2377 | CZ | TYR | B | 93 | -19.309 | -3.123 | 21.731 | 1.00 | 32.69 | C |
| ATOM | 2378 | OH | TYR | B | 93 | -19.161 | -2.436 | 20.550 | 1.00 | 33.36 | O |
| ATOM | 2379 | N | CYS | B | 94 | -21.901 | -6.157 | 27.762 | 1.00 | 25.68 | N |

(continued)

| | | | | | | | | | | | |
|---|---|---|---|---|---|---|---|---|---|---|---|
| ATOM | 2380 | CA | CYS | B | 94 | -22.079 | -7.300 | 28.644 | 1.00 | 25.53 | C |
| ATOM | 2381 | C | CYS | B | 94 | -21.899 | -8.557 | 27.810 | 1.00 | 28.07 | C |
| ATOM | 2382 | O | CYS | B | 94 | -22.013 | -8.516 | 26.585 | 1.00 | 28.25 | O |
| ATOM | 2383 | CB | CYS | B | 94 | -23.450 | -7.282 | 29.329 | 1.00 | 30.94 | C |
| ATOM | 2384 | SG | CYS | B | 94 | -24.873 | -7.383 | 28.210 | 1.00 | 35.78 | S |
| ATOM | 2385 | N | GLN | B | 95 | -21.606 | -9.671 | 28.468 | 1.00 | 25.81 | N |
| ATOM | 2386 | CA | GLN | B | 95 | -21.373 - | -10.920 | 27.758 | 1.00 | 28.41 | C |
| ATOM | 2387 | C | GLN | B | 95 | -21.819 - | -12.093 | 28.612 | 1.00 | 24.10 | C |
| ATOM | 2388 | O | GLN | B | 95 | -21.476 - | -12.169 | 29.789 | 1.00 | 33.33 | O |
| ATOM | 2389 | CB | GLN | B | 95 | -19.891 - | -11.065 | 27.391 | 1.00 | 24.57 | C |
| ATOM | 2390 | CG | GLN | B | 95 | -19.574 - | -12.277 | 26.523 | 1.00 | 27.70 | C |
| ATOM | 2391 | CD | GLN | B | 95 | -18.497 - | -13.168 | 27.121 | 1.00 | 30.66 | C |
| ATOM | 2392 | NE2 | GLN | B | 95 | -18.756 - | -14.468 | 27.147 | 1.00 | 41.67 | N |
| ATOM | 2393 | OE1 | GLN | B | 95 | -17.452 - | -12.695 | 27.557 | 1.00 | 36.39 | O |
| ATOM | 2394 | N | GLN | B | 96 | -22.584 - | -13.008 | 28.031 | 1.00 | 30.99 | N |
| ATOM | 2395 | CA | GLN | B | 96 | -22.926 - | -14.232 | 28.748 | 1.00 | 28.28 | C |
| ATOM | 2396 | C | GLN | B | 96 | -21.986 - | -15.347 | 28.313 | 1.00 | 31.50 | C |
| ATOM | 2397 | O | GLN | B | 96 | -21.612 - | -15.447 | 27.141 | 1.00 | 27.92 | O |
| ATOM | 2398 | CB | GLN | B | 96 | -24.392 - | -14.630 | 28.517 | 1.00 | 24.83 | C |
| ATOM | 2399 | CG | GLN | B | 96 | -24.737 - | -15.108 | 27.104 | 1.00 | 28.68 | C |
| ATOM | 2400 | CD | GLN | B | 96 | -24.439 - | -16.586 | 26.876 | 1.00 | 32.36 | C |
| ATOM | 2401 | NE2 | GLN | B | 96 | -24.396 - | -16.992 | 25.611 | 1.00 | 32.40 | N |
| ATOM | 2402 | OE1 | GLN | B | 96 | -24.241 - | -17.348 | 27.826 | 1.00 | 32.56 | O |
| ATOM | 2403 | N | TYR | B | 97 | -21.592 - | -16.182 | 29.261 | 1.00 | 26.53 | N |
| ATOM | 2404 | CA | TYR | B | 97 | -20.838 - | -17.370 | 28.918 | 1.00 | 30.75 | C |
| ATOM | 2405 | C | TYR | B | 97 | -21.465 - | -18.571 | 29.608 | 1.00 | 31.37 | C |
| ATOM | 2406 | O | TYR | B | 97 | -20.778 - | -19.519 | 29.989 | 1.00 | 28.22 | O |
| ATOM | 2407 | CB | TYR | B | 97 | -19.367 - | -17.209 | 29.294 | 1.00 | 27.16 | C |
| ATOM | 2408 | CG | TYR | B | 97 | -19.121 - | -16.745 | 30.714 | 1.00 | 30.11 | C |
| ATOM | 2409 | CD2 | TYR | B | 97 | -18.893 - | -17.661 | 31.737 | 1.00 | 25.04 | C |
| ATOM | 2410 | CD1 | TYR | B | 97 | -19.093 - | -15.390 | 31.026 | 1.00 | 26.12 | C |
| ATOM | 2411 | CE2 | TYR | B | 97 | -18.650 - | -17.239 | 33.033 | 1.00 | 26.05 | C |
| ATOM | 2412 | CE1 | TYR | B | 97 | -18.856 | -14.960 | 32.316 | 1.00 | 30.26 | C |
| ATOM | 2413 | CZ | TYR | B | 97 | -18.634 | -15.887 | 33.314 | 1.00 | 28.89 | C |

(continued)

| | | | | | | | | | | |
|---|---|---|---|---|---|---|---|---|---|---|
| ATOM | 2414 | OH | TYR | B | 97 | -18.396 | -15.458 | 34.597 | 1.00 | 29.84 | O |
| ATOM | 2415 | N | TYR | B | 98 | -22.784 | -18.510 | 29.762 | 1.00 | 37.70 | N |
| ATOM | 2416 | CA | TYR | B | 98 | -23.553 | -19.601 | 30.349 | 1.00 | 32.36 | C |
| ATOM | 2417 | C | TYR | B | 98 | -23.579 | -20.801 | 29.410 | 1.00 | 37.08 | C |
| ATOM | 2418 | O | TYR | B | 98 | -23.446 | -21.942 | 29.849 | 1.00 | 37.43 | O |
| ATOM | 2419 | CB | TYR | B | 98 | -24.981 | -19.153 | 30.672 | 1.00 | 32.63 | C |
| ATOM | 2420 | CG | TYR | B | 98 | -25.769 | -20.191 | 31.440 | 1.00 | 40.80 | C |
| ATOM | 2421 | CD1 | TYR | B | 98 | -25.434 | -20.511 | 32.750 | 1.00 | 39.01 | C |
| ATOM | 2422 | CD2 | TYR | B | 98 | -26.844 | -20.853 | 30.857 | 1.00 | 44.09 | C |
| ATOM | 2423 | CE1 | TYR | B | 98 | -26.146 | -21.462 | 33.461 | 1.00 | 46.24 | C |
| ATOM | 2424 | CE2 | TYR | B | 98 | -27.566 | -21.807 | 31.563 | 1.00 | 46.44 | C |
| ATOM | 2425 | CZ | TYR | B | 98 | -27.209 | -22.107 | 32.864 | 1.00 | 48.82 | C |
| ATOM | 2426 | OH | TYR | B | 98 | -27.913 | -23.052 | 33.574 | 1.00 | 61. 88 | O |
| ATOM | 2427 | N | ASN | B | 99 | -23.758 | -20.542 | 28.117 | 1.00 | 36.19 | N |
| ATOM | 2428 | CA | ASN | B | 99 | -23.611 | -21.596 | 27.119 | 1.00 | 44.15 | C |
| ATOM | 2429 | C | ASN | B | 99 | -23.116 | -21.074 | 25.778 | 1.00 | 42.06 | C |
| ATOM | 2430 | O | ASN | B | 99 | -23.118 | -19.868 | 25.521 | 1.00 | 43.16 | O |
| ATOM | 2431 | CB | ASN | B | 99 | -24.927 | -22.361 | 26.924 | 1.00 | 49.30 | C |
| ATOM | 2432 | CG | ASN | B | 99 | -26.102 | -21.455 | 26.600 | 1.00 | 51.38 | C |
| ATOM | 2433 | OD1 | ASN | B | 99 | -27.134 | -21.508 | 27.271 | 1.00 | 68.12 | O |
| ATOM | 2434 | ND2 | ASN | B | 99 | -25.959 | -20.628 | 25.568 | 1.00 | 49.12 | N |
| ATOM | 2435 | N | THR | B | 100 | -22.700 | -22.001 | 24.924 | 1.00 | 38.31 | N |
| ATOM | 2436 | CA | THR | B | 100 | -22.195 | -21.657 | 23.607 | 1.00 | 37.71 | C |
| ATOM | 2437 | C | THR | B | 100 | -23.339 | -21.679 | 22.601 | 1.00 | 36.72 | C |
| ATOM | 2438 | O | THR | B | 100 | -24.243 | -22.506 | 22.713 | 1.00 | 45.21 | O |
| ATOM | 2439 | CB | THR | B | 100 | -21.076 | -22.618 | 23.173 | 1.00 | 40.81 | C |
| ATOM | 2440 | OG1 | THR | B | 100 | -21.540 | -23.966 | 23.286 | 1.00 | 41.71 | O |
| ATOM | 2441 | CG2 | THR | B | 100 | -19.855 | -22.442 | 24.069 | 1.00 | 34.33 | C |
| ATOM | 2442 | N | PRO | B | 101 | -23.313 | -20.765 | 21.617 | 1.00 | 37.91 | N |
| ATOM | 2443 | CA | PRO | B | 101 | -22.253 | -19.773 | 21.404 | 1.00 | 35.04 | C |
| ATOM | 2444 | C | PRO | B | 101 | -22.295 | -18.613 | 22.395 | 1.00 | 38.94 | C |
| ATOM | 2445 | O | PRO | B | 101 | -23.368 | -18.135 | 22.774 | 1.00 | 33.27 | O |
| ATOM | 2446 | CB | PRO | B | 101 | -22.526 | -19.273 | 19.983 | 1.00 | 36.11 | C |
| ATOM | 2447 | CG | PRO | B | 101 | -24.004 | -19.394 | 19.839 | 1.00 | 36.49 | C |

(continued)

| | | | | | | | | | | | |
|---|---|---|---|---|---|---|---|---|---|---|---|
| ATOM | 2448 | CD | PRO | B | 101 | -24.388 | -20.638 | 20.615 | 1.00 | 42.27 | C |
| ATOM | 2449 | N | VAL | B | 102 | -21.111 | -18.192 | 22.823 | 1.00 | 35.40 | N |
| ATOM | 2450 | CA | VAL | B | 102 | -20.943 | -16.980 | 23.610 | 1.00 | 34.45 | C |
| ATOM | 2451 | C | VAL | B | 102 | -21.531 | -15.794 | 22.858 | 1.00 | 35.11 | C |
| ATOM | 2452 | O | VAL | B | 102 | -21.300 | -15.643 | 21.659 | 1.00 | 30.87 | O |
| ATOM | 2453 | CB | VAL | B | 102 | -19.454 | -16.735 | 23.911 | 1.00 | 38.34 | C |
| ATOM | 2454 | CG1 | VAL | B | 102 | -19.155 | -15.250 | 24.058 | 1.00 | 41.39 | C |
| ATOM | 2455 | CG2 | VAL | B | 102 | -19.046 | -17.509 | 25.142 | 1.00 | 38.42 | C |
| ATOM | 2456 | N | THR | B | 103 | -22.307 | -14.966 | 23.549 | 1.00 | 28.91 | N |
| ATOM | 2457 | CA | THR | B | 103 | -22.929 | -13.817 | 22.901 | 1.00 | 32.04 | C |
| ATOM | 2458 | C | THR | B | 103 | -22.740 | -12.532 | 23.711 | 1.00 | 33.94 | C |
| ATOM | 2459 | O | THR | B | 103 | -22.715 | -12.554 | 24.945 | 1.00 | 30.64 | O |
| ATOM | 2460 | CB | THR | B | 103 | -24.437 | -14.054 | 22.659 | 1.00 | 33.42 | C |
| ATOM | 2461 | CG2 | THR | B | 103 | -24.658 | -15.178 | 21.636 | 1.00 | 30.57 | C |
| ATOM | 2462 | OG1 | THR | B | 103 | -25.068 | -14.415 | 23.890 | 1.00 | 35.52 | O |
| ATOM | 2463 | N | PHE | B | 104 | -22.594 | -11.419 | 22.996 | 1.00 | 27.76 | N |
| ATOM | 2464 | CA | PHE | B | 104 | -22.429 | -10.102 | 23.604 | 1.00 | 32.55 | C |
| ATOM | 2465 | C | PHE | B | 104 | -23.693 | -9.274 | 23.430 | 1.00 | 34.05 | C |
| ATOM | 2466 | O | PHE | B | 104 | -24.481 | -9.520 | 22.519 | 1.00 | 32.37 | O |
| ATOM | 2467 | CB | PHE | B | 104 | -21.257 | -9.342 | 22.971 | 1.00 | 30.42 | C |
| ATOM | 2468 | CG | PHE | B | 104 | -19.915 | -9.986 | 23.175 | 1.00 | 30.59 | C |
| ATOM | 2469 | CD1 | PHE | B | 104 | -19.531 | -11.079 | 22.417 | 1.00 | 27.18 | C |
| ATOM | 2470 | CD2 | PHE | B | 104 | -19.019 | -9.467 | 24.094 | 1.00 | 30.39 | C |
| ATOM | 2471 | CE1 | PHE | B | 104 | -18.289 | -11.660 | 22.590 | 1.00 | 31.59 | C |
| ATOM | 2472 | CE2 | PHE | B | 104 | -17.775 | -10.044 | 24.274 | 1.00 | 31.20 | C |
| ATOM | 2473 | CZ | PHE | B | 104 | -17.411 | -11.144 | 23.519 | 1.00 | 33.50 | C |
| ATOM | 2474 | N | GLY | B | 105 | -23.873 | -8.278 | 24.291 | 1.00 | 34.23 | N |
| ATOM | 2475 | CA | GLY | B | 105 | -24.878 | -7.258 | 24.057 | 1.00 | 26.27 | C |
| ATOM | 2476 | C | GLY | B | 105 | -24.415 | -6.334 | 22.941 | 1.00 | 34.89 | C |
| ATOM | 2477 | O | GLY | B | 105 | -23.264 | -6.413 | 22.509 | 1.00 | 31.29 | O |
| ATOM | 2478 | N | PRO | B | 106 | -25.307 | -5.447 | 22.470 | 1.00 | 35.38 | N |
| ATOM | 2479 | CA | PRO | B | 106 | -25.028 | -4.559 | 21.336 | 1.00 | 33.90 | C |
| ATOM | 2480 | C | PRO | B | 106 | -24.100 | -3.399 | 21.689 | 1.00 | 32.12 | C |
| ATOM | 2481 | O | PRO | B | 106 | -23.610 | -2.715 | 20.794 | 1.00 | 34.27 | O |

(continued)

| | | | | | | | | | | | |
|---|---|---|---|---|---|---|---|---|---|---|---|
| ATOM | 2482 | CB | PRO | B | 106 | -26.417 | -4.037 | 20.956 | 1.00 | 29.01 | C |
| ATOM | 2483 | CG | PRO | B | 106 | -27.173 | -4.053 | 22.248 | 1.00 | 37.44 | C |
| ATOM | 2484 | CD | PRO | B | 106 | -26.676 | -5.267 | 22.989 | 1.00 | 33.03 | C |
| ATOM | 2485 | N | GLY | B | 107 | -23.874 | -3.177 | 22.978 | 1.00 | 34.23 | N |
| ATOM | 2486 | CA | GLY | B | 107 | -22.984 | -2.120 | 23.415 | 1.00 | 34.20 | C |
| ATOM | 2487 | C | GLY | B | 107 | -23.701 | -0.843 | 23.807 | 1.00 | 34.29 | C |
| ATOM | 2488 | O | GLY | B | 107 | -24.725 | -0.489 | 23.230 | 1.00 | 37.38 | O |
| ATOM | 2489 | N | THR | B | 108 | -23.149 | -0.148 | 24.795 | 1.00 | 33.98 | N |
| ATOM | 2490 | CA | THR | B | 108 | -23.695 | 1.121 | 25.256 | 1.00 | 35.86 | C |
| ATOM | 2491 | C | THR | B | 108 | -22.617 | 2.190 | 25.231 | 1.00 | 33.06 | C |
| ATOM | 2492 | O | THR | B | 108 | -21.586 | 2.039 | 25.886 | 1.00 | 33.05 | O |
| ATOM | 2493 | CB | THR | B | 108 | -24.251 | 1.016 | 26.690 | 1.00 | 31.62 | C |
| ATOM | 2494 | CG2 | THR | B | 108 | -24.619 | 2.395 | 27.221 | 1.00 | 36.86 | C |
| ATOM | 2495 | OG1 | THR | B | 108 | -25.406 | 0.174 | 26.702 | 1.00 | 36.12 | O |
| ATOM | 2496 | N | LYS | B | 109 | -22.847 | 3.271 | 24.493 | 1.00 | 28.00 | N |
| ATOM | 2497 | CA | LYS | B | 109 | -21.850 | 4.337 | 24.418 | 1.00 | 33.62 | C |
| ATOM | 2498 | C | LYS | B | 109 | -21.956 | 5.254 | 25.634 | 1.00 | 38.15 | C |
| ATOM | 2499 | O | LYS | B | 109 | -23.026 | 5.786 | 25.931 | 1.00 | 41.81 | O |
| ATOM | 2500 | CB | LYS | B | 109 | -22.008 | 5.142 | 23.124 | 1.00 | 36.39 | C |
| ATOM | 2501 | CG | LYS | B | 109 | -22.625 | 4.343 | 21.980 | 1.00 | 56.96 | C |
| ATOM | 2502 | CD | LYS | B | 109 | -21.807 | 4.421 | 20.696 | 1.00 | 56.13 | C |
| ATOM | 2503 | CE | LYS | B | 109 | -21.884 | 5.792 | 20.054 | 1.00 | 62. 08 | C |
| ATOM | 2504 | NZ | LYS | B | 109 | -21.138 | 5.822 | 18.763 | 1.00 | 74. 82 | N |
| ATOM | 2505 | N | VAL | B | 110 | -20.848 | 5.420 | 26.348 | 1.00 | 32.23 | N |
| ATOM | 2506 | CA | VAL | B | 110 | -20.804 | 6.345 | 27.474 | 1.00 | 29.73 | C |
| ATOM | 2507 | C | VAL | B | 110 | -19.884 | 7.524 | 27.185 | 1.00 | 32.33 | C |
| ATOM | 2508 | O | VAL | B | 110 | -18.699 | 7.340 | 26.904 | 1.00 | 33. 08 | O |
| ATOM | 2509 | CB | VAL | B | 110 | -20.328 | 5.659 | 28.765 | 1.00 | 31.47 | C |
| ATOM | 2510 | CG1 | VAL | B | 110 | -20.223 | 6.678 | 29.886 | 1.00 | 29.96 | C |
| ATOM | 2511 | CG2 | VAL | B | 110 | -21.270 | 4.525 | 29.142 | 1.00 | 34.90 | C |
| ATOM | 2512 | N | GLY | B | 111 | -20.440 | 8.730 | 27.249 | 1.00 | 29.76 | N |
| ATOM | 2513 | CA | GLY | B | 111 | -19.666 | 9.945 | 27.076 | 1.00 | 29.88 | C |
| ATOM | 2514 | C | GLY | B | 111 | -19.749 | 10.757 | 28.351 | 1.00 | 39.68 | C |
| ATOM | 2515 | O | GLY | B | 111 | -20.459 | 10.374 | 29.280 | 1.00 | 39.37 | O |

(continued)

| | | | | | | | | | | | |
|---|---|---|---|---|---|---|---|---|---|---|---|
| ATOM | 2516 | N | ILE | B | 112 | -19.030 | 11.872 | 28.416 | 1.00 | 31.76 | N |
| ATOM | 2517 | CA | ILE | B | 112 | -19.082 | 12.697 | 29.613 | 1.00 | 36.49 | C |
| ATOM | 2518 | C | ILE | B | 112 | -19.508 | 14.132 | 29.295 | 1.00 | 41.86 | C |
| ATOM | 2519 | O | ILE | B | 112 | -19.221 | 14.665 | 28.216 | 1.00 | 33.11 | O |
| ATOM | 2520 | CB | ILE | B | 112 | -17.720 | 12.704 | 30.366 | 1.00 | 36.62 | C |
| ATOM | 2521 | CG1 | ILE | B | 112 | -16.745 | 13.711 | 29.766 | 1.00 | 42.34 | C |
| ATOM | 2522 | CG2 | ILE | B | 112 | -17.105 | 11.311 | 30.403 | 1.00 | 53.62 | C |
| ATOM | 2523 | CD1 | ILE | B | 112 | -16.711 | 15.042 | 30.519 | 1.00 | 54.93 | C |
| ATOM | 2524 | O | LYS | B | 113 | -19.210 | 16.754 | 32.044 | 1.00 | 44.16 | O |
| ATOM | 2525 | N | LYS | B | 113 | -20.201 | 14.744 | 30.251 | 1.00 | 37.92 | N |
| ATOM | 2526 | CA | LYS | B | 113 | -20.582 | 16.147 | 30.170 | 1.00 | 39.34 | C |
| ATOM | 2527 | C | LYS | B | 113 | -19.560 | 17.015 | 30.892 | 1.00 | 43.78 | C |
| ATOM | 2528 | CB | LYS | B | 113 | -21.965 | 16.381 | 30.778 | 1.00 | 41.17 | C |
| ATOM | 2529 | CG | LYS | B | 113 | -23.120 | 15.740 | 30.040 | 1.00 | 40.99 | C |
| ATOM | 2530 | CD | LYS | B | 113 | -24.434 | 16.062 | 30.750 | 1.00 | 46.55 | C |
| ATOM | 2531 | CE | LYS | B | 113 | -25.614 | 15.365 | 30.094 | 1.00 | 50.82 | C |
| ATOM | 2532 | NZ | LYS | B | 113 | -26.868 | 15.545 | 30.882 | 1.00 | 66.34 | N |
| ATOM | 2533 | O | ARG | B | 114 | -19.706 | 20.614 | 29.907 | 1.00 | 33.63 | O |
| ATOM | 2534 | N | ARG | B | 114 | -19.080 | 18.045 | 30.208 | 1.00 | 37.75 | N |
| ATOM | 2535 | CA | ARG | B | 114 | -18.179 | 19.009 | 30.821 | 1.00 | 32.69 | C |
| ATOM | 2536 | C | ARG | B | 114 | -18.661 | 20.423 | 30.526 | 1.00 | 32.37 | C |
| ATOM | 2537 | CB | ARG | B | 114 | -16.747 | 18.815 | 30.316 | 1.00 | 29.43 | C |
| ATOM | 2538 | CG | ARG | B | 114 | -16.605 | 18.850 | 28.801 | 1.00 | 32.96 | C |
| ATOM | 2539 | CD | ARG | B | 114 | -15.228 | 19.372 | 28.400 | 1.00 | 29.78 | C |
| ATOM | 2540 | NE | ARG | B | 114 | -15.053 | 20.768 | 28.793 | 1.00 | 30.75 | N |
| ATOM | 2541 | CZ | ARG | B | 114 | -13.893 | 21.418 | 28.775 | 1.00 | 35.05 | C |
| ATOM | 2542 | NH1 | ARG | B | 114 | -13.841 | 22.691 | 29.144 | 1.00 | 37.02 | N |
| ATOM | 2543 | NH2 | ARG | B | 114 | -12.784 | 20.801 | 28.392 | 1.00 | 37.36 | N |
| ATOM | 2544 | O | THR | B | 115 | -17.390 | 22.466 | 28.475 | 1.00 | 30.86 | O |
| ATOM | 2545 | N | THR | B | 115 | -17.898 | 21.413 | 30.968 | 1.00 | 34.13 | N |
| ATOM | 2546 | CA | THR | B | 115 | -18.244 | 22.800 | 30.701 | 1.00 | 37.77 | C |
| ATOM | 2547 | C | THR | B | 115 | -18.146 | 23.108 | 29.207 | 1.00 | 38.67 | C |
| ATOM | 2548 | CB | THR | B | 115 | -17.336 | 23.763 | 31.484 | 1.00 | 35.59 | C |
| ATOM | 2549 | OG1 | THR | B | 115 | -15.972 | 23.545 | 31.106 | 1.00 | 34.24 | O |

(continued)

| | | | | | | | | | | | |
|---|---|---|---|---|---|---|---|---|---|---|---|
| ATOM | 2550 | CG2 | THR | B | 115 | -17.485 | 23.531 | 32.986 | 1.00 | 28.14 | C |
| ATOM | 2551 | N | VAL | B | 116 | -18.932 | 24.080 | 28.762 | 1.00 | 39.57 | N |
| ATOM | 2552 | CA | VAL | B | 116 | -18.884 | 24.545 | 27.383 | 1.00 | 35.06 | C |
| ATOM | 2553 | C | VAL | B | 116 | -17.491 | 25.075 | 27.047 | 1.00 | 35.92 | C |
| ATOM | 2554 | O | VAL | B | 116 | -16.872 | 25.772 | 27.855 | 1.00 | 32.43 | O |
| ATOM | 2555 | CB | VAL | B | 116 | -19.938 | 25.649 | 27.133 | 1.00 | 35.82 | c |
| ATOM | 2556 | CG1 | VAL | B | 116 | -19.734 | 26.295 | 25.778 | 1.00 | 33.41 | c |
| ATOM | 2557 | CG2 | VAL | B | 116 | -21.347 | 25.082 | 27.253 | 1.00 | 29.35 | C |
| ATOM | 2558 | N | ALA | B | 117 | -16.991 | 24.725 | 25.865 | 1.00 | 29.09 | N |
| ATOM | 2559 | CA | ALA | B | 117 | -15.719 | 25.259 | 25.387 | 1.00 | 27.87 | C |
| ATOM | 2560 | C | ALA | B | 117 | -15.829 | 25.618 | 23.913 | 1.00 | 34.19 | C |
| ATOM | 2561 | O | ALA | B | 117 | -16.203 | 24.779 | 23.088 | 1.00 | 29.90 | O |
| ATOM | 2562 | CB | ALA | B | 117 | -14.589 | 24.258 | 25.615 | 1.00 | 30.76 | C |
| ATOM | 2563 | N | ALA | B | 118 | -15.522 | 26.870 | 23.588 | 1.00 | 32.82 | N |
| ATOM | 2564 | CA | ALA | B | 118 | -15.615 | 27.347 | 22.212 | 1.00 | 34.29 | C |
| ATOM | 2565 | C | ALA | B | 118 | -14.474 | 26.788 | 21.370 | 1.00 | 29.81 | C |
| ATOM | 2566 | O | ALA | B | 118 | -13.357 | 26.625 | 21.858 | 1.00 | 31.85 | O |
| ATOM | 2567 | CB | ALA | B | 118 | -15.614 | 28.877 | 22.173 | 1.00 | 29.49 | C |
| ATOM | 2568 | N | PRO | B | 119 | -14.753 | 26.481 | 20.097 | 1.00 | 31.49 | N |
| ATOM | 2569 | CA | PRO | B | 119 | -13.680 | 25.979 | 19.237 | 1.00 | 32.80 | C |
| ATOM | 2570 | C | PRO | B | 119 | -12.745 | 27.089 | 18.790 | 1.00 | 34.11 | C |
| ATOM | 2571 | O | PRO | B | 119 | -13.190 | 28.218 | 18.601 | 1.00 | 30.89 | O |
| ATOM | 2572 | CB | PRO | B | 119 | -14.436 | 25.404 | 18.039 | 1.00 | 29.59 | C |
| ATOM | 2573 | CG | PRO | B | 119 | -15.668 | 26.247 | 17.957 | 1.00 | 33.03 | C |
| ATOM | 2574 | CD | PRO | B | 119 | -16.043 | 26.559 | 19.386 | 1.00 | 30.58 | C |
| ATOM | 2575 | N | SER | B | 120 | -11.467 | 26.769 | 18.635 | 1.00 | 29.42 | N |
| ATOM | 2576 | CA | SER | B | 120 | -10.567 | 27.624 | 17.878 | 1.00 | 36.74 | C |
| ATOM | 2577 | C | SER | B | 120 | -10.661 | 27.181 | 16.418 | 1.00 | 34.59 | C |
| ATOM | 2578 | O | SER | B | 120 | -10.687 | 25.983 | 16.131 | 1.00 | 33.48 | O |
| ATOM | 2579 | CB | SER | B | 120 | -9.135 | 27.531 | 18.407 | 1.00 | 31.92 | C |
| ATOM | 2580 | OG | SER | B | 120 | -8.627 | 26.219 | 18.245 | 1.00 | 50.83 | O |
| ATOM | 2581 | N | VAL | B | 121 | -10.734 | 28.139 | 15.499 | 1.00 | 34.03 | N |
| ATOM | 2582 | CA | VAL | B | 121 | -11.015 | 27.831 | 14.096 | 1.00 | 29.08 | C |
| ATOM | 2583 | C | VAL | B | 121 | -9.845 | 28.195 | 13.179 | 1.00 | 31.62 | C |

(continued)

| | | | | | | | | | | | |
|---|---|---|---|---|---|---|---|---|---|---|---|
| ATOM | 2584 | O | VAL | B | 121 | -9.239 | 29.257 | 13.314 | 1.00 | 33.72 | O |
| ATOM | 2585 | CB | VAL | B | 121 | -12.300 | 28.560 | 13.616 | 1.00 | 30.04 | C |
| ATOM | 2586 | CG1 | VAL | B | 121 | -12.663 | 28.158 | 12.193 | 1.00 | 30.45 | C |
| ATOM | 2587 | CG2 | VAL | B | 121 | -13.460 | 28.259 | 14.551 | 1.00 | 31.72 | C |
| ATOM | 2588 | N | PHE | B | 122 | -9.534 | 27.295 | 12.251 | 1.00 | 27.86 | N |
| ATOM | 2589 | CA | PHE | B | 122 | -8.463 | 27.498 | 11.283 | 1.00 | 29.57 | C |
| ATOM | 2590 | C | PHE | B | 122 | -8.935 | 27.084 | 9.890 | 1.00 | 39.92 | C |
| ATOM | 2591 | O | PHE | B | 122 | -9.622 | 26.068 | 9.734 | 1.00 | 34.52 | O |
| ATOM | 2592 | CB | PHE | B | 122 | -7.215 | 26.689 | 11.663 | 1.00 | 36.36 | C |
| ATOM | 2593 | CG | PHE | B | 122 | -6.747 | 26.906 | 13.078 | 1.00 | 32.19 | C |
| ATOM | 2594 | CD1 | PHE | B | 122 | -7.271 | 26.158 | 14.117 | 1.00 | 36.97 | C |
| ATOM | 2595 | CD2 | PHE | B | 122 | -5.770 | 27 . 847 | 13.362 | 1.00 | 34.40 | C |
| ATOM | 2596 | CE1 | PHE | B | 122 | -6.836 | 26.352 | 15.419 | 1.00 | 41.91 | C |
| ATOM | 2597 | CE2 | PHE | B | 122 | -5.330 | 28.044 | 14.661 | 1.00 | 36.84 | C |
| ATOM | 2598 | CZ | PHE | B | 122 | -5.864 | 27.294 | 15.689 | 1.00 | 37.49 | C |
| ATOM | 2599 | N | ILE | B | 123 | -8.566 | 27.862 | 8.879 | 1.00 | 28.07 | N |
| ATOM | 2600 | CA | ILE | B | 123 | -8.915 | 27.528 | 7.505 | 1.00 | 32.41 | C |
| ATOM | 2601 | C | ILE | B | 123 | -7.636 | 27.277 | 6.701 | 1.00 | 32.00 | C |
| ATOM | 2602 | O | ILE | B | 123 | -6.622 | 27.942 | 6.907 | 1.00 | 31.75 | O |
| ATOM | 2603 | CB | ILE | B | 123 | -9.777 | 28.646 | 6.846 | 1.00 | 28.87 | C |
| ATOM | 2604 | CG1 | ILE | B | 123 | -10.347 | 28.183 | 5.502 | 1.00 | 32.47 | C |
| ATOM | 2605 | CG2 | ILE | B | 123 | -8.985 | 29.938 | 6.700 | 1.00 | 28.48 | C |
| ATOM | 2606 | CD1 | ILE | B | 123 | -11.358 | 29.154 | 4.896 | 1.00 | 31.95 | C |
| ATOM | 2607 | N | PHE | B | 124 | -7.679 | 26.291 | 5.811 | 1.00 | 31.26 | N |
| ATOM | 2608 | CA | PHE | B | 124 | -6.514 | 25.928 | 5.009 | 1.00 | 30.58 | C |
| ATOM | 2609 | C | PHE | B | 124 | -6.854 | 25.944 | 3.523 | 1.00 | 35.12 | C |
| ATOM | 2610 | O | PHE | B | 124 | -7.774 | 25.250 | 3.089 | 1.00 | 33.03 | O |
| ATOM | 2611 | CB | PHE | B | 124 | -5.992 | 24.540 | 5.390 | 1.00 | 32.40 | C |
| ATOM | 2612 | CG | PHE | B | 124 | -5.566 | 24.410 | 6.825 | 1.00 | 32.10 | C |
| ATOM | 2613 | CD1 | PHE | B | 124 | -4.314 | 24.842 | 7.232 | 1.00 | 31.26 | C |
| ATOM | 2614 | CD2 | PHE | B | 124 | -6.407 | 23.820 | 7.759 | 1.00 | 31. 84 | C |
| ATOM | 2615 | CE1 | PHE | B | 124 | -3.913 | 24.711 | 8.549 | 1.00 | 34.33 | C |
| ATOM | 2616 | CE2 | PHE | B | 124 | -6.015 | 23.684 | 9.076 | 1.00 | 34.28 | C |
| ATOM | 2617 | CZ | PHE | B | 124 | -4.765 | 24.133 | 9.474 | 1.00 | 35.30 | C |

(continued)

| | | | | | | | | | | | |
|---|---|---|---|---|---|---|---|---|---|---|---|
| ATOM | 2618 | N | PRO | B | 125 | -6.107 | 26.731 | 2.734 | 1.00 | 37.67 | N |
| ATOM | 2619 | CA | PRO | B | 125 | -6.296 | 26.753 | 1.278 | 1.00 | 33.34 | C |
| ATOM | 2620 | C | PRO | B | 125 | -5.816 | 25.454 | 0.646 | 1.00 | 32.58 | C |
| ATOM | 2621 | O | PRO | B | 125 | -4.996 | 24.766 | 1.247 | 1.00 | 29.20 | O |
| ATOM | 2622 | CB | PRO | B | 125 | -5.422 | 27.930 | 0.816 | 1.00 | 33.45 | C |
| ATOM | 2623 | CG | PRO | B | 125 | -5.036 | 28.671 | 2.074 | 1.00 | 39.04 | C |
| ATOM | 2624 | CD | PRO | B | 125 | -5.049 | 27.658 | 3.171 | 1.00 | 32.49 | C |
| ATOM | 2625 | N | PRO | B | 126 | -6.307 | 25.126 | -0.557 | 1.00 | 31.25 | N |
| ATOM | 2626 | CA | PRO | B | 126 | -5.736 | 23.974 | -1.263 | 1.00 | 36.87 | C |
| ATOM | 2627 | C | PRO | B | 126 | -4.265 | 24.213 | -1.615 | 1.00 | 37.25 | C |
| ATOM | 2628 | O | PRO | B | 126 | -3.844 | 25.360 | -1.778 | 1.00 | 32.38 | O |
| ATOM | 2629 | CB | PRO | B | 126 | -6.599 | 23.868 | -2.527 | 1.00 | 31.74 | C |
| ATOM | 2630 | CG | PRO | B | 126 | -7.137 | 25.243 | -2.728 | 1.00 | 32.69 | C |
| ATOM | 2631 | CD | PRO | B | 126 | -7.351 | 25.800 | -1.348 | 1.00 | 31.78 | C |
| ATOM | 2632 | N | SER | B | 127 | -3.491 | 23.139 | -1.709 | 1.00 | 33.45 | N |
| ATOM | 2633 | CA | SER | B | 127 | -2.082 | 23.251 | -2.055 | 1.00 | 37.63 | C |
| ATOM | 2634 | C | SER | B | 127 | -1.931 | 23.368 | -3.565 | 1.00 | 41.08 | C |
| ATOM | 2635 | O | SER | B | 127 | -2.812 | 22.939 | -4.317 | 1.00 | 38.62 | O |
| ATOM | 2636 | CB | SER | B | 127 | -1.299 | 22.043 | -1.542 | 1.00 | 33.64 | C |
| ATOM | 2637 | OG | SER | B | 127 | -1.647 | 20.874 | -2.267 | 1.00 | 31.28 | O |
| ATOM | 2638 | N | ASP | B | 128 | -0.814 | 23.938 | -4.008 | 1.00 | 40.33 | N |
| ATOM | 2639 | CA | ASP | B | 128 | -0.544 | 24.044 | -5.437 | 1.00 | 42.97 | C |
| ATOM | 2640 | C | ASP | B | 128 | -0.439 | 22.664 | -6.066 | 1.00 | 36.76 | C |
| ATOM | 2641 | O | ASP | B | 128 | -0.783 | 22.481 | -7.232 | 1.00 | 42.27 | O |
| ATOM | 2642 | CB | ASP | B | 128 | 0.737 | 24.841 | -5.695 | 1.00 | 46.63 | C |
| ATOM | 2643 | CG | ASP | B | 128 | 0.570 | 26.322 | -5.406 | 1.00 | 59.28 | C |
| ATOM | 2644 | OD1 | ASP | B | 128 | -0.562 | 26.834 | -5.546 | 1.00 | 59.20 | O |
| ATOM | 2645 | OD2 | ASP | B | 128 | 1.570 | 26.974 | -5.040 | 1.00 | 62.56 | O |
| ATOM | 2646 | N | GLU | B | 129 | 0.022 | 21.690 | -5.286 | 1.00 | 37.90 | N |
| ATOM | 2647 | CA | GLU | B | 129 | 0.240 | 20.347 | -5.809 | 1.00 | 41.75 | C |
| ATOM | 2648 | C | GLU | B | 129 | -1.075 | 19.666 | -6.183 | 1.00 | 42.49 | C |
| ATOM | 2649 | O | GLU | B | 129 | -1.157 | 18.982 | -7.202 | 1.00 | 42.15 | O |
| ATOM | 2650 | CB | GLU | B | 129 | 1.008 | 19.489 | -4.800 | 1.00 | 38.29 | C |
| ATOM | 2651 | CG | GLU | B | 129 | 1.521 | 18.181 | -5.394 | 1.00 | 55.48 | C |

(continued)

| | | | | | | | | | | |
|---|---|---|---|---|---|---|---|---|---|---|
| ATOM | 2652 | CD | GLU | B | 129 | 2.471 | 17.435 | -4.469 | 1.00 | 75.53 | C |
| ATOM | 2653 | OE1 | GLU | B | 129 | 3.644 | 17.234 | -4.857 | 1.00 | 78.69 | O |
| ATOM | 2654 | OE2 | GLU | B | 129 | 2.044 | 17.043 | -3.360 | 1.00 | 72.25 | O |
| ATOM | 2655 | N | GLN | B | 130 | -2.104 | 19.857 | -5.365 | 1.00 | 37.24 | N |
| ATOM | 2656 | CA | GLN | B | 130 | -3.403 | 19.259 | -5.654 | 1.00 | 34.81 | C |
| ATOM | 2657 | C | GLN | B | 130 | -4.093 | 19.947 | -6.829 | 1.00 | 35.05 | C |
| ATOM | 2658 | O | GLN | B | 130 | -4.774 | 19.297 | -7.622 | 1.00 | 36.82 | O |
| ATOM | 2659 | CB | GLN | B | 130 | -4.312 | 19.312 | -4.427 | 1.00 | 32.75 | C |
| ATOM | 2660 | CG | GLN | B | 130 | -5.631 | 18.597 | -4.645 | 1.00 | 30.49 | C |
| ATOM | 2661 | CD | GLN | B | 130 | -6.626 | 18.848 | -3.534 | 1.00 | 38.30 | C |
| ATOM | 2662 | NE2 | GLN | B | 130 | -7.643 | 18.000 | -3.456 | 1.00 | 35.08 | N |
| ATOM | 2663 | OE1 | GLN | B | 130 | -6.485 | 19.793 | -2.754 | 1.00 | 31.99 | O |
| ATOM | 2664 | N | LEU | B | 131 | -3.930 | 21.265 | -6.924 | 1.00 | 34.78 | N |
| ATOM | 2665 | CA | LEU | B | 131 | -4.512 | 22.036 | -8.019 | 1.00 | 46.01 | C |
| ATOM | 2666 | C | LEU | B | 131 | -4.044 | 21.506 | -9.369 | 1.00 | 40.30 | C |
| ATOM | 2667 | O | LEU | B | 131 | -4.800 | 21.497 | -10.337 | 1.00 | 49.24 | O |
| ATOM | 2668 | CB | LEU | B | 131 | -4.160 | 23.521 | -7.884 | 1.00 | 39.51 | C |
| ATOM | 2669 | CG | LEU | B | 131 | -4.884 | 24.279 | -6.773 | 1.00 | 41.36 | C |
| ATOM | 2670 | CD1 | LEU | B | 131 | -4.458 | 25.736 | -6.738 | 1.00 | 42.24 | C |
| ATOM | 2671 | CD2 | LEU | B | 131 | -6.385 | 24.168 | -6.956 | 1.00 | 37.48 | C |
| ATOM | 2672 | N | LYS | B | 132 | -2.798 | 21. 048 | -9.414 | 1.00 | 43.36 | N |
| ATOM | 2673 | CA | LYS | B | 132 | -2.214 | 20.462 | -10.616 | 1.00 | 47.83 | C |
| ATOM | 2674 | C | LYS | B | 132 | -2.933 | 19.197 | -11.086 | 1.00 | 46.86 | C |
| ATOM | 2675 | O | LYS | B | 132 | -2.715 | 18.740 | -12.206 | 1.00 | 54.52 | O |
| ATOM | 2676 | CB | LYS | B | 132 | -0.737 | 20.146 | -10.373 | 1.00 | 49.58 | C |
| ATOM | 2677 | CG | LYS | B | 132 | 0.229 | 21.216 | -10.868 | 1.00 | 56.15 | C |
| ATOM | 2678 | CD | LYS | B | 132 | 1.614 | 21.060 | -10.241 | 1.00 | 64.07 | C |
| ATOM | 2679 | CE | LYS | B | 132 | 2.009 | 19.593 | -10.066 | 1.00 | 66.31 | C |
| ATOM | 2680 | NZ | LYS | B | 132 | 2.086 | 18.843 | -11.352 | 1.00 | 66.61 | N |
| ATOM | 2681 | N | SER | B | 133 | -3.776 | 18.626 | -10.232 | 1.00 | 47.49 | N |
| ATOM | 2682 | CA | SER | B | 133 | -4.497 | 17.403 | -10.578 | 1.00 | 38.87 | C |
| ATOM | 2683 | C | SER | B | 133 | -5.963 | 17.680 | -10.922 | 1.00 | 45.85 | C |
| ATOM | 2684 | O | SER | B | 133 | -6.718 | 16.757 | -11.226 | 1.00 | 45.58 | O |
| ATOM | 2685 | CB | SER | B | 133 | -4.415 | 16.389 | -9.433 | 1.00 | 48.92 | C |

(continued)

| | | | | | | | | | | | |
|---|---|---|---|---|---|---|---|---|---|---|---|
| ATOM | 2686 | OG | SER | B | 133 | -5.127 | 16.848 | -8.293 | 1.00 | 52.02 | O |
| ATOM | 2687 | N | GLY | B | 134 | -6.365 | 18.947 | -10.858 | 1.00 | 39.38 | N |
| ATOM | 2688 | CA | GLY | B | 134 | -7.692 | 19.349 | -11.294 | 1.00 | 38.72 | C |
| ATOM | 2689 | C | GLY | B | 134 | -8.737 | 19.497 | -10.202 | 1.00 | 40.64 | C |
| ATOM | 2690 | O | GLY | B | 134 | -9.904 | 19.767 | -10.488 | 1.00 | 47.67 | O |
| ATOM | 2691 | N | THR | B | 135 | -8.320 | 19.335 | -8.951 | 1.00 | 44.09 | N |
| ATOM | 2692 | CA | THR | B | 135 | -9.239 | 19.382 | -7.816 | 1.00 | 41.60 | C |
| ATOM | 2693 | C | THR | B | 135 | -8.700 | 20.294 | -6.716 | 1.00 | 38.79 | C |
| ATOM | 2694 | O | THR | B | 135 | -7.490 | 20.386 | -6.515 | 1.00 | 39.11 | O |
| ATOM | 2695 | CB | THR | B | 135 | -9.485 | 17.965 | -7.246 | 1.00 | 42.58 | C |
| ATOM | 2696 | CG2 | THR | B | 135 | -10.545 | 17.982 | -6.153 | 1.00 | 36.34 | C |
| ATOM | 2697 | OG1 | THR | B | 135 | -9.910 | 17.092 | -8.300 | 1.00 | 49.29 | O |
| ATOM | 2698 | N | ALA | B | 136 | -9.599 | 20.979 | -6.016 | 1.00 | 39.56 | N |
| ATOM | 2699 | CA | ALA | B | 136 | -9.218 | 21.845 | -4.907 | 1.00 | 31.14 | C |
| ATOM | 2700 | C | ALA | B | 136 | -9.980 | 21.476 | -3.637 | 1.00 | 41.19 | C |
| ATOM | 2701 | O | ALA | B | 136 | -11.211 | 21.514 | -3.609 | 1.00 | 38.90 | O |
| ATOM | 2702 | CB | ALA | B | 136 | -9.465 | 23.300 | -5.257 | 1.00 | 30.89 | C |
| ATOM | 2703 | N | SER | B | 137 | -9.243 | 21.121 | -2.588 | 1.00 | 32.83 | N |
| ATOM | 2704 | CA | SER | B | 137 | -9.844 | 20.823 | -1.295 | 1.00 | 29.16 | C |
| ATOM | 2705 | C | SER | B | 137 | -9.551 | 21.947 | -0.312 | 1.00 | 31.90 | C |
| ATOM | 2706 | O | SER | B | 137 | -8.391 | 22.273 | -0.060 | 1.00 | 32.52 | O |
| ATOM | 2707 | CB | SER | B | 137 | -9.325 | 19.493 | -0.742 | 1.00 | 31.20 | C |
| ATOM | 2708 | OG | SER | B | 137 | -9.665 | 18.411 | -1.588 | 1.00 | 35.83 | O |
| ATOM | 2709 | N | VAL | B | 138 | -10.605 | 22.549 | 0.227 | 1.00 | 29.38 | N |
| ATOM | 2710 | CA | VAL | B | 138 | -10.455 | 23.578 | 1.250 | 1.00 | 28.61 | C |
| ATOM | 2711 | C | VAL | B | 138 | -10.856 | 22.986 | 2.596 | 1.00 | 28.33 | C |
| ATOM | 2712 | O | VAL | B | 138 | -11.896 | 22.332 | 2.708 | 1.00 | 28.89 | O |
| ATOM | 2713 | CB | VAL | B | 138 | -11.304 | 24.821 | 0.943 | 1.00 | 32.25 | C |
| ATOM | 2714 | CG1 | VAL | B | 138 | -10.880 | 25.981 | 1.825 | 1.00 | 32.25 | C |
| ATOM | 2715 | CG2 | VAL | B | 138 | -11.171 | 25.197 | -0.524 | 1.00 | 34.85 | C |
| ATOM | 2716 | N | VAL | B | 139 | -10.031 | 23.208 | 3.615 | 1.00 | 27.05 | N |
| ATOM | 2717 | CA | VAL | B | 139 | -10.241 | 22.558 | 4.906 | 1.00 | 30.66 | C |
| ATOM | 2718 | C | VAL | B | 139 | -10.472 | 23.556 | 6.033 | 1.00 | 28.68 | C |
| ATOM | 2719 | O | VAL | B | 139 | -9.741 | 24.537 | 6.173 | 1.00 | 29.77 | O |

(continued)

| | | | | | | | | | | |
|---|---|---|---|---|---|---|---|---|---|---|
| ATOM | 2720 | CB | VAL | B | 139 | -9.042 | 21.646 | 5.267 | 1.00 | 31.42 | C |
| ATOM | 2721 | CG1 | VAL | B | 139 | -9.199 | 21.067 | 6.665 | 1.00 | 30.03 | C |
| ATOM | 2722 | CG2 | VAL | B | 139 | -8.904 | 20.529 | 4.239 | 1.00 | 29.56 | C |
| ATOM | 2723 | N | CYS | B | 140 | -11.507 | 23.300 | 6.826 | 1.00 | 31.41 | N |
| ATOM | 2724 | CA | CYS | B | 140 | -11.792 | 24.088 | 8.015 | 1.00 | 30.57 | C |
| ATOM | 2725 | C | CYS | B | 140 | -11.634 | 23.207 | 9.245 | 1.00 | 36.50 | C |
| ATOM | 2726 | O | CYS | B | 140 | -12.198 | 22.113 | 9.306 | 1.00 | 29.35 | O |
| ATOM | 2727 | CB | CYS | B | 140 | -13.205 | 24.672 | 7.960 | 1.00 | 32.78 | C |
| ATOM | 2728 | SG | CYS | B | 140 | -13.561 | 25.937 | 9.212 | 1.00 | 47.42 | S |
| ATOM | 2729 | N | LEU | B | 141 | -10.871 | 23.686 | 10.222 | 1.00 | 34.12 | N |
| ATOM | 2730 | CA | LEU | B | 141 | -10.629 | 22.931 | 11.444 | 1.00 | 30.81 | C |
| ATOM | 2731 | C | LEU | B | 141 | -11.230 | 23.619 | 12.672 | 1.00 | 33.78 | C |
| ATOM | 2732 | O | LEU | B | 141 | -11.006 | 24.809 | 12.904 | 1.00 | 32.21 | O |
| ATOM | 2733 | CB | LEU | B | 141 | -9.126 | 22.721 | 11.649 | 1.00 | 30.25 | C |
| ATOM | 2734 | CG | LEU | B | 141 | -8.728 | 22.199 | 13.032 | 1.00 | 33.72 | C |
| ATOM | 2735 | CD1 | LEU | B | 141 | -9.143 | 20.737 | 13.205 | 1.00 | 28.38 | C |
| ATOM | 2736 | CD2 | LEU | B | 141 | -7.234 | 22.383 | 13.280 | 1.00 | 35.34 | C |
| ATOM | 2737 | N | LEU | B | 142 | -12.006 | 22.862 | 13.442 | 1.00 | 25.85 | N |
| ATOM | 2738 | CA | LEU | B | 142 | -12.538 | 23.331 | 14.721 | 1.00 | 29.63 | C |
| ATOM | 2739 | C | LEU | B | 142 | -11.865 | 22.526 | 15.822 | 1.00 | 31.52 | C |
| ATOM | 2740 | O | LEU | B | 142 | -11.966 | 21.300 | 15.848 | 1.00 | 32.17 | O |
| ATOM | 2741 | CB | LEU | B | 142 | -14.062 | 23.177 | 14.796 | 1.00 | 28.07 | C |
| ATOM | 2742 | CG | LEU | B | 142 | -14.992 | 24.140 | 14.044 | 1.00 | 29.90 | C |
| ATOM | 2743 | CD1 | LEU | B | 142 | -14.697 | 24.189 | 12.550 | 1.00 | 36.79 | C |
| ATOM | 2744 | CD2 | LEU | B | 142 | -16.440 | 23.731 | 14.274 | 1.00 | 27.06 | C |
| ATOM | 2745 | N | ASN | B | 143 | -11.174 | 23.209 | 16.723 | 1.00 | 25.29 | N |
| ATOM | 2746 | CA | ASN | B | 143 | -10.291 | 22.521 | 17 . 648 | 1.00 | 31. 88 | C |
| ATOM | 2747 | C | ASN | B | 143 | -10.754 | 22.593 | 19.100 | 1.00 | 34.33 | C |
| ATOM | 2748 | O | ASN | B | 143 | -11.042 | 23.673 | 19.618 | 1.00 | 32.56 | O |
| ATOM | 2749 | CB | ASN | B | 143 | -8.876 | 23. 089 | 17.512 | 1.00 | 32.86 | C |
| ATOM | 2750 | CG | ASN | B | 143 | -7.813 | 22.134 | 18.016 | 1.00 | 43.50 | C |
| ATOM | 2751 | ND2 | ASN | B | 143 | -7.037 | 22.574 | 18.997 | 1.00 | 47.72 | N |
| ATOM | 2752 | OD1 | ASN | B | 143 | -7.683 | 21.018 | 17.518 | 1.00 | 44.96 | O |
| ATOM | 2753 | N | ASN | B | 144 | -10.840 | 21.421 | 19.733 | 1.00 | 33.53 | N |

(continued)

| | | | | | | | | | | | |
|---|---|---|---|---|---|---|---|---|---|---|---|
| ATOM | 2754 | CA | ASN | B | 144 | -11.100 | 21.284 | 21.171 | 1.00 | 31.24 | C |
| ATOM | 2755 | C | ASN | B | 144 | -12.330 | 22.023 | 21.688 | 1.00 | 29.06 | C |
| ATOM | 2756 | O | ASN | B | 144 | -12.211 | 22.953 | 22.482 | 1.00 | 33.00 | O |
| ATOM | 2757 | CB | ASN | B | 144 | -9.873 | 21.743 | 21.962 | 1.00 | 30.14 | C |
| ATOM | 2758 | CG | ASN | B | 144 | -8.626 | 20.965 | 21.598 | 1.00 | 37.05 | C |
| ATOM | 2759 | ND2 | ASN | B | 144 | -7.468 | 21.575 | 21.802 | 1.00 | 43.00 | N |
| ATOM | 2760 | OD1 | ASN | B | 144 | -8.704 | 19.827 | 21.134 | 1.00 | 38.36 | O |
| ATOM | 2761 | N | PHE | B | 145 | -13.513 | 21.602 | 21.258 | 1.00 | 31.89 | N |
| ATOM | 2762 | CA | PHE | B | 145 | -14.732 | 22.260 | 21.706 | 1.00 | 29.94 | C |
| ATOM | 2763 | C | PHE | B | 145 | -15.672 | 21.295 | 22.416 | 1.00 | 34.88 | C |
| ATOM | 2764 | O | PHE | B | 145 | -15.533 | 20.074 | 22.317 | 1.00 | 26.87 | O |
| ATOM | 2765 | CB | PHE | B | 145 | -15.455 | 22.936 | 20.529 | 1.00 | 26.54 | C |
| ATOM | 2766 | CG | PHE | B | 145 | -15.846 | 21.994 | 19.418 | 1.00 | 27.20 | C |
| ATOM | 2767 | CD1 | PHE | B | 145 | -14.961 | 21.705 | 18.392 | 1.00 | 25.82 | C |
| ATOM | 2768 | CD2 | PHE | B | 145 | -17.102 | 21.413 | 19.392 | 1.00 | 29.78 | C |
| ATOM | 2769 | CE1 | PHE | B | 145 | -15.316 | 20.839 | 17.368 | 1.00 | 27.58 | c |
| ATOM | 2770 | CE2 | PHE | B | 145 | -17.467 | 20.551 | 18.372 | 1.00 | 29.41 | c |
| ATOM | 2771 | CZ | PHE | B | 145 | -16.569 | 20.263 | 17.356 | 1.00 | 27.09 | C |
| ATOM | 2772 | N | TYR | B | 146 | -16.619 | 21.871 | 23.149 | 1.00 | 28.10 | N |
| ATOM | 2773 | CA | TYR | B | 146 | -17.698 | 21.125 | 23.778 | 1.00 | 30.54 | C |
| ATOM | 2774 | C | TYR | B | 146 | -18.886 | 22.071 | 23.962 | 1.00 | 29.20 | C |
| ATOM | 2775 | O | TYR | B | 146 | -18.702 | 23.217 | 24.364 | 1.00 | 28.71 | O |
| ATOM | 2776 | CB | TYR | B | 146 | -17.262 | 20.527 | 25.128 | 1.00 | 31.96 | C |
| ATOM | 2777 | CG | TYR | B | 146 | -18.319 | 19.621 | 25.711 | 1.00 | 34.78 | C |
| ATOM | 2778 | CD2 | TYR | B | 146 | -18.334 | 18.262 | 25.418 | 1.00 | 31.97 | C |
| ATOM | 2779 | CD1 | TYR | B | 146 | -19.334 | 20.131 | 26.515 | 1.00 | 31.84 | C |
| ATOM | 2780 | CE2 | TYR | B | 146 | -19.319 | 17.432 | 25.925 | 1.00 | 32.92 | C |
| ATOM | 2781 | CE1 | TYR | B | 146 | -20.324 | 19.311 | 27.022 | 1.00 | 32.17 | C |
| ATOM | 2782 | CZ | TYR | B | 146 | -20.310 | 17.965 | 26.725 | 1.00 | 34.30 | C |
| ATOM | 2783 | OH | TYR | B | 146 | -21.291 | 17.149 | 27.228 | 1.00 | 42.60 | O |
| ATOM | 2784 | N | PRO | B | 147 | -20.112 | 21.593 | 23.695 | 1.00 | 25.21 | N |
| ATOM | 2785 | CA | PRO | B | 147 | -20.474 | 20.225 | 23.311 | 1.00 | 31.43 | C |
| ATOM | 2786 | C | PRO | B | 147 | -20.233 | 19.921 | 21.838 | 1.00 | 34.10 | C |
| ATOM | 2787 | O | PRO | B | 147 | -19.696 | 20.751 | 21.103 | 1.00 | 33.70 | O |

(continued)

| | | | | | | | | | | | |
|---|---|---|---|---|---|---|---|---|---|---|---|
| ATOM | 2788 | CB | PRO | B | 147 | -21.967 | 20.159 | 23.643 | 1.00 | 32.07 | C |
| ATOM | 2789 | CG | PRO | B | 147 | -22.446 | 21.552 | 23.429 | 1.00 | 27.75 | C |
| ATOM | 2790 | CD | PRO | B | 147 | -21.301 | 22.453 | 23.836 | 1.00 | 31.95 | C |
| ATOM | 2791 | N | ARG | B | 148 | -20.646 | 18.725 | 21.434 | 1.00 | 34.40 | N |
| ATOM | 2792 | CA | ARG | B | 148 | -20.383 | 18.168 | 20.110 | 1.00 | 32.34 | C |
| ATOM | 2793 | C | ARG | B | 148 | -21.069 | 18.932 | 18.975 | 1.00 | 32.84 | C |
| ATOM | 2794 | O | ARG | B | 148 | -20.505 | 19.087 | 17 .892 | 1.00 | 29.63 | O |
| ATOM | 2795 | CB | ARG | B | 148 | -20.823 | 16.700 | 20.101 | 1.00 | 35.79 | C |
| ATOM | 2796 | CG | ARG | B | 148 | -20.923 | 16.051 | 18.744 | 1.00 | 41.03 | C |
| ATOM | 2797 | CD | ARG | B | 148 | -19.703 | 15.208 | 18.453 | 1.00 | 47.90 | C |
| ATOM | 2798 | NE | ARG | B | 148 | -20.044 | 13.980 | 17.738 | 1.00 | 56.46 | N |
| ATOM | 2799 | CZ | ARG | B | 148 | -20.405 | 13.932 | 16.458 | 1.00 | 56.76 | C |
| ATOM | 2800 | NH1 | ARG | B | 148 | -20.485 | 15.048 | 15.740 | 1.00 | 54.31 | N |
| ATOM | 2801 | NH2 | ARG | B | 148 | -20.688 | 12.767 | 15.893 | 1.00 | 51.59 | N |
| ATOM | 2802 | N | GLU | B | 149 | -22.284 | 19.404 | 19.230 | 1.00 | 36.40 | N |
| ATOM | 2803 | CA | GLU | B | 149 | -23.068 | 20.115 | 18.224 | 1.00 | 39.72 | C |
| ATOM | 2804 | C | GLU | B | 149 | -22.387 | 21.403 | 17.779 | 1.00 | 39.89 | C |
| ATOM | 2805 | O | GLU | B | 149 | -22.182 | 22.319 | 18.577 | 1.00 | 39.53 | O |
| ATOM | 2806 | CB | GLU | B | 149 | -24.473 | 20.431 | 18.755 | 1.00 | 34.93 | C |
| ATOM | 2807 | CG | GLU | B | 149 | -25.369 | 19.209 | 18.956 | 1.00 | 45.72 | C |
| ATOM | 2808 | CD | GLU | B | 149 | -24.967 | 18.363 | 20.158 | 1.00 | 54.80 | C |
| ATOM | 2809 | OE1 | GLU | B | 149 | -24.402 | 18.920 | 21.125 | 1.00 | 48.46 | O |
| ATOM | 2810 | OE2 | GLU | B | 149 | -25.211 | 17.138 | 20.130 | 1.00 | 62.82 | O |
| ATOM | 2811 | N | ALA | B | 150 | -22.042 | 21.462 | 16.498 | 1.00 | 35.98 | N |
| ATOM | 2812 | CA | ALA | B | 150 | -21.432 | 22.649 | 15.914 | 1.00 | 37.14 | C |
| ATOM | 2813 | C | ALA | B | 150 | -21.867 | 22.779 | 14.465 | 1.00 | 43.97 | C |
| ATOM | 2814 | O | ALA | B | 150 | -22.094 | 21.778 | 13.786 | 1.00 | 46.01 | O |
| ATOM | 2815 | CB | ALA | B | 150 | -19.914 | 22.586 | 16.014 | 1.00 | 32.49 | C |
| ATOM | 2816 | N | LYS | B | 151 | -21.987 | 24.012 | 13.992 | 1.00 | 39.41 | N |
| ATOM | 2817 | CA | LYS | B | 151 | -22.417 | 24.246 | 12.624 | 1.00 | 43.48 | C |
| ATOM | 2818 | C | LYS | B | 151 | -21.319 | 24.913 | 11.807 | 1.00 | 39.53 | C |
| ATOM | 2819 | O | LYS | B | 151 | -20.799 | 25.966 | 12.179 | 1.00 | 36.87 | O |
| ATOM | 2820 | CB | LYS | B | 151 | -23.687 | 25.099 | 12.596 | 1.00 | 43.88 | C |
| ATOM | 2821 | CG | LYS | B | 151 | -24.256 | 25.301 | 11.196 | 1.00 | 55.40 | C |

(continued)

| | | | | | | | | | | |
|---|---|---|---|---|---|---|---|---|---|---|
| ATOM | 2822 | CD | LYS | B | 151 | -25.547 | 26.104 | 11.223 | 1.00 | 53.19 | C |
| ATOM | 2823 | CE | LYS | B | 151 | -26.147 | 26.222 | 9.830 | 1.00 | 70.97 | C |
| ATOM | 2824 | NZ | LYS | B | 151 | -27.463 | 26.923 | 9.839 | 1.00 | 84.38 | N |
| ATOM | 2825 | N | VAL | B | 152 | -20.968 | 24.282 | 10.694 | 1.00 | 36.81 | N |
| ATOM | 2826 | CA | VAL | B | 152 | -19.990 | 24.837 | 9.774 | 1.00 | 37.97 | C |
| ATOM | 2827 | C | VAL | B | 152 | -20.652 | 25.168 | 8.446 | 1.00 | 36.38 | C |
| ATOM | 2828 | O | VAL | B | 152 | -21.221 | 24.292 | 7.790 | 1.00 | 36.25 | O |
| ATOM | 2829 | CB | VAL | B | 152 | -18.820 | 23.867 | 9.535 | 1.00 | 39.90 | C |
| ATOM | 2830 | CG1 | VAL | B | 152 | -17.884 | 24.420 | 8.470 | 1.00 | 35.36 | C |
| ATOM | 2831 | CG2 | VAL | B | 152 | -18.078 | 23.614 | 10.834 | 1.00 | 39.55 | C |
| ATOM | 2832 | N | GLN | B | 153 | -20.590 | 26.437 | 8.060 | 1.00 | 32.64 | N |
| ATOM | 2833 | CA | GLN | B | 153 | -21.118 | 26.864 | 6.771 | 1.00 | 34.66 | C |
| ATOM | 2834 | C | GLN | B | 153 | -19.999 | 27.378 | 5.884 | 1.00 | 38.08 | C |
| ATOM | 2835 | O | GLN | B | 153 | -19.290 | 28.319 | 6.244 | 1.00 | 38.02 | O |
| ATOM | 2836 | CB | GLN | B | 153 | -22.187 | 27.946 | 6.946 | 1.00 | 37.83 | C |
| ATOM | 2837 | CG | GLN | B | 153 | -23.590 | 27.404 | 7.164 | 1.00 | 60.26 | C |
| ATOM | 2838 | CD | GLN | B | 153 | -24.596 | 28.498 | 7.470 | 1.00 | 67.71 | C |
| ATOM | 2839 | NE2 | GLN | B | 153 | -25.857 | 28.257 | 7.123 | 1.00 | 56.86 | N |
| ATOM | 2840 | OE1 | GLN | B | 153 | -24.244 | 29.548 | 8.009 | 1.00 | 70.19 | O |
| ATOM | 2841 | N | TRP | B | 154 | -19.840 | 26.747 | 4.727 | 1.00 | 31.58 | N |
| ATOM | 2842 | CA | TRP | B | 154 | -18.864 | 27.189 | 3.747 | 1.00 | 27.44 | C |
| ATOM | 2843 | C | TRP | B | 154 | -19.449 | 28.300 | 2.884 | 1.00 | 33.80 | C |
| ATOM | 2844 | O | TRP | B | 154 | -20.592 | 28.209 | 2.436 | 1.00 | 30.68 | O |
| ATOM | 2845 | CB | TRP | B | 154 | -18.415 | 26.022 | 2.869 | 1.00 | 25.14 | C |
| ATOM | 2846 | CG | TRP | B | 154 | -17.449 | 25.089 | 3.532 | 1.00 | 34.77 | C |
| ATOM | 2847 | CD1 | TRP | B | 154 | -17.729 | 23.870 | 4.080 | 1.00 | 29.48 | C |
| ATOM | 2848 | CD2 | TRP | B | 154 | -16.042 | 25.295 | 3.709 | 1.00 | 33.52 | C |
| ATOM | 2849 | CE2 | TRP | B | 154 | -15.534 | 24.159 | 4.369 | 1.00 | 36.13 | C |
| ATOM | 2850 | CE3 | TRP | B | 154 | -15.163 | 26.328 | 3.370 | 1.00 | 28.50 | C |
| ATOM | 2851 | NE1 | TRP | B | 154 | -16.584 | 23.304 | 4.582 | 1.00 | 32.42 | N |
| ATOM | 2852 | CZ2 | TRP | B | 154 | -14.186 | 24.028 | 4.697 | 1.00 | 35.02 | C |
| ATOM | 2853 | CZ3 | TRP | B | 154 | -13.827 | 26.199 | 3.699 | 1.00 | 32.42 | C |
| ATOM | 2854 | CH2 | TRP | B | 154 | -13.350 | 25.056 | 4.355 | 1.00 | 33.88 | C |
| ATOM | 2855 | N | LYS | B | 155 | -18.661 | 29.343 | 2.652 | 1.00 | 26.76 | N |

(continued)

| | | | | | | | | | | | |
|---|---|---|---|---|---|---|---|---|---|---|---|
| ATOM | 2856 | CA | LYS | B | 155 | -19.089 | 30.458 | 1.818 | 1.00 | 34.70 | C |
| ATOM | 2857 | C | LYS | B | 155 | -18.018 | 30.803 | 0.795 | 1.00 | 35.14 | C |
| ATOM | 2858 | O | LYS | B | 155 | -16.869 | 31.063 | 1.149 | 1.00 | 34.32 | O |
| ATOM | 2859 | CB | LYS | B | 155 | -19.415 | 31.685 | 2.671 | 1.00 | 31. 08 | C |
| ATOM | 2860 | CG | LYS | B | 155 | -20.658 | 31.527 | 3.525 | 1.00 | 39.61 | C |
| ATOM | 2861 | CD | LYS | B | 155 | -20.861 | 32.722 | 4.439 | 1.00 | 45.78 | C |
| ATOM | 2862 | CE | LYS | B | 155 | -22.086 | 32.531 | 5.322 | 1.00 | 52.43 | C |
| ATOM | 2863 | NZ | LYS | B | 155 | -22.240 | 33.644 | 6.300 | 1.00 | 64.93 | N |
| ATOM | 2864 | N | VAL | B | 156 | -18.404 | 30.787 | -0.474 | 1.00 | 33.51 | N |
| ATOM | 2865 | CA | VAL | B | 156 | -17.512 | 31.163 | -1.562 | 1.00 | 34.85 | C |
| ATOM | 2866 | C | VAL | B | 156 | -18.043 | 32.430 | -2.235 | 1.00 | 37.05 | C |
| ATOM | 2867 | O | VAL | B | 156 | -19.085 | 32.398 | -2.889 | 1.00 | 36.43 | O |
| ATOM | 2868 | CB | VAL | B | 156 | -17.378 | 30.035 | -2.590 | 1.00 | 37.60 | C |
| ATOM | 2869 | CG1 | VAL | B | 156 | -16.452 | 30.450 | -3.701 | 1.00 | 36.02 | C |
| ATOM | 2870 | CG2 | VAL | B | 156 | -16.863 | 28.772 | -1.921 | 1.00 | 31.56 | C |
| ATOM | 2871 | N | ASP | B | 157 | -17.321 | 33.536 | -2.060 | 1.00 | 38.87 | N |
| ATOM | 2872 | CA | ASP | B | 157 | -17.785 | 34.866 | -2.468 | 1.00 | 41.38 | C |
| ATOM | 2873 | C | ASP | B | 157 | -19.196 | 35.139 | -1.951 | 1.00 | 44.37 | C |
| ATOM | 2874 | O | ASP | B | 157 | -20.058 | 35.627 | -2.684 | 1.00 | 49.49 | O |
| ATOM | 2875 | CB | ASP | B | 157 | -17.733 | 35.023 | -3.991 | 1.00 | 42.34 | C |
| ATOM | 2876 | CG | ASP | B | 157 | -16.326 | 35.296 | -4.499 | 1.00 | 44.58 | C |
| ATOM | 2877 | OD1 | ASP | B | 157 | -15.517 | 35.866 | -3.734 | 1.00 | 49.40 | O |
| ATOM | 2878 | OD2 | ASP | B | 157 | -16.029 | 34.942 | -5.659 | 1.00 | 49.33 | O |
| ATOM | 2879 | N | ASN | B | 158 | -19.405 | 34.809 | -0.678 | 1.00 | 38.79 | N |
| ATOM | 2880 | CA | ASN | B | 158 | -20.688 | 34.958 | 0.016 | 1.00 | 46.07 | C |
| ATOM | 2881 | C | ASN | B | 158 | -21.827 | 34.101 | -0.535 | 1.00 | 42.73 | C |
| ATOM | 2882 | O | ASN | B | 158 | -22.992 | 34.335 | -0.218 | 1.00 | 49.48 | O |
| ATOM | 2883 | CB | ASN | B | 158 | -21.115 | 36.426 | 0.033 | 1.00 | 47.14 | C |
| ATOM | 2884 | CG | ASN | B | 158 | -20.716 | 37.122 | 1.313 | 1.00 | 64.92 | C |
| ATOM | 2885 | ND2 | ASN | B | 158 | -20.238 | 38.356 | 1.196 | 1.00 | 70.04 | N |
| ATOM | 2886 | OD1 | ASN | B | 158 | -20.825 | 36.549 | 2.400 | 1.00 | 67.22 | O |
| ATOM | 2887 | N | ALA | B | 159 | -21.497 | 33.100 | -1.341 | 1.00 | 37.66 | N |
| ATOM | 2888 | CA | ALA | B | 159 | -22.492 | 32.110 | -1.731 | 1.00 | 37.19 | C |
| ATOM | 2889 | C | ALA | B | 159 | -22.368 | 30.887 | -0.823 | 1.00 | 39.41 | C |

(continued)

| | | | | | | | | | | | |
|---|---|---|---|---|---|---|---|---|---|---|---|
| ATOM | 2890 | O | ALA | B | 159 | -21.298 | 30.285 | -0.720 | 1.00 | 32.50 | O |
| ATOM | 2891 | CB | ALA | B | 159 | -22.330 | 31.717 | -3.191 | 1.00 | 30.09 | C |
| ATOM | 2892 | N | LEU | B | 160 | -23.464 | 30.537 | -0.159 | 1.00 | 34.32 | N |
| ATOM | 2893 | CA | LEU | B | 160 | -23.496 | 29.376 | 0.720 | 1.00 | 29.40 | C |
| ATOM | 2894 | C | LEU | B | 160 | -23.360 | 28. 083 | -0.073 | 1.00 | 33.09 | C |
| ATOM | 2895 | O | LEU | B | 160 | -24.083 | 27.858 | -1.044 | 1.00 | 36.96 | O |
| ATOM | 2896 | CB | LEU | B | 160 | -24.790 | 29.348 | 1.540 | 1.00 | 37.86 | C |
| ATOM | 2897 | CG | LEU | B | 160 | -24.960 | 30.336 | 2.701 | 1.00 | 54.68 | C |
| ATOM | 2898 | CD1 | LEU | B | 160 | -25.190 | 31.767 | 2.222 | 1.00 | 48.85 | C |
| ATOM | 2899 | CD2 | LEU | B | 160 | -26.100 | 29.883 | 3.608 | 1.00 | 60.01 | C |
| ATOM | 2900 | N | GLN | B | 161 | -22.427 | 27.238 | 0.347 | 1.00 | 30.58 | N |
| ATOM | 2901 | CA | GLN | B | 161 | -22.206 | 25.943 | -0.284 | 1.00 | 30.22 | C |
| ATOM | 2902 | C | GLN | B | 161 | -22.919 | 24.850 | 0.499 | 1.00 | 35.29 | C |
| ATOM | 2903 | O | GLN | B | 161 | -22.868 | 24.821 | 1.727 | 1.00 | 38.99 | O |
| ATOM | 2904 | CB | GLN | B | 161 | -20.710 | 25.633 | -0.371 | 1.00 | 31.23 | C |
| ATOM | 2905 | CG | GLN | B | 161 | -19.880 | 26.761 | -0.958 | 1.00 | 30.86 | C |
| ATOM | 2906 | CD | GLN | B | 161 | -20.221 | 27.015 | -2.407 | 1.00 | 32.74 | C |
| ATOM | 2907 | NE2 | GLN | B | 161 | -20.715 | 28.210 | -2.698 | 1.00 | 30.42 | N |
| ATOM | 2908 | OE1 | GLN | B | 161 | -20.062 | 26.138 | -3.252 | 1.00 | 34.09 | O |
| ATOM | 2909 | N | SER | B | 162 | -23.588 | 23.955 | -0.215 | 1.00 | 27.98 | N |
| ATOM | 2910 | CA | SER | B | 162 | -24.234 | 22.815 | 0.416 | 1.00 | 38.77 | C |
| ATOM | 2911 | C | SER | B | 162 | -24.111 | 21.602 | -0.496 | 1.00 | 34.56 | C |
| ATOM | 2912 | O | SER | B | 162 | -24.271 | 21.717 | -1.713 | 1.00 | 33.35 | O |
| ATOM | 2913 | CB | SER | B | 162 | -25.703 | 23.126 | 0.723 | 1.00 | 31.67 | C |
| ATOM | 2914 | OG | SER | B | 162 | -26.329 | 22.040 | 1.385 | 1.00 | 49.66 | O |
| ATOM | 2915 | N | GLY | B | 163 | -23.800 | 20.449 | 0.090 | 1.00 | 33.25 | N |
| ATOM | 2916 | CA | GLY | B | 163 | -23.739 | 19.206 | -0.661 | 1.00 | 28.14 | C |
| ATOM | 2917 | C | GLY | B | 163 | -22.356 | 18.880 | -1.192 | 1.00 | 32.51 | C |
| ATOM | 2918 | O | GLY | B | 163 | -22.101 | 17.758 | -1.620 | 1.00 | 33.73 | O |
| ATOM | 2919 | N | ASN | B | 164 | -21.457 | 19.859 | -1.164 | 1.00 | 30.22 | N |
| ATOM | 2920 | CA | ASN | B | 164 | -20.114 | 19.664 | -1.694 | 1.00 | 31.34 | C |
| ATOM | 2921 | C | ASN | B | 164 | -19.043 | 19.693 | -0.605 | 1.00 | 35.48 | C |
| ATOM | 2922 | O | ASN | B | 164 | -17.881 | 19.990 | -0.876 | 1.00 | 31.32 | O |
| ATOM | 2923 | CB | ASN | B | 164 | -19.809 | 20.722 | -2.767 | 1.00 | 30.74 | C |

(continued)

| | | | | | | | | | | | |
|---|---|---|---|---|---|---|---|---|---|---|---|
| ATOM | 2924 | CG | ASN | B | 164 | -19.951 | 22.147 | -2.248 | 1.00 | 33.60 | C |
| ATOM | 2925 | ND2 | ASN | B | 164 | -19.572 | 23.116 | -3.073 | 1.00 | 33.79 | N |
| ATOM | 2926 | OD1 | ASN | B | 164 | -20.405 | 22.374 | -1.127 | 1.00 | 32.68 | O |
| ATOM | 2927 | N | SER | B | 165 | -19.435 | 19.387 | 0.628 | 1.00 | 35.40 | N |
| ATOM | 2928 | CA | SER | B | 165 | -18.470 | 19.275 | 1.714 | 1.00 | 35.79 | C |
| ATOM | 2929 | C | SER | B | 165 | -18.750 | 18.042 | 2.561 | 1.00 | 32.53 | C |
| ATOM | 2930 | O | SER | B | 165 | -19.849 | 17.490 | 2.521 | 1.00 | 29.82 | O |
| ATOM | 2931 | CB | SER | B | 165 | -18.478 | 20.530 | 2.593 | 1.00 | 32.78 | C |
| ATOM | 2932 | OG | SER | B | 165 | -19.639 | 20.592 | 3.401 | 1.00 | 33.05 | O |
| ATOM | 2933 | N | GLN | B | 166 | -17.742 | 17.606 | 3.311 | 1.00 | 30.86 | N |
| ATOM | 2934 | CA | GLN | B | 166 | -17.896 | 16.486 | 4.236 | 1.00 | 32.40 | C |
| ATOM | 2935 | C | GLN | B | 166 | -17.194 | 16.792 | 5.553 | 1.00 | 32.23 | C |
| ATOM | 2936 | O | GLN | B | 166 | -16.181 | 17.493 | 5.575 | 1.00 | 33.90 | O |
| ATOM | 2937 | CB | GLN | B | 166 | -17.346 | 15.189 | 3.634 | 1.00 | 26.55 | C |
| ATOM | 2938 | CG | GLN | B | 166 | -18.110 | 14.694 | 2.418 | 1.00 | 37.55 | C |
| ATOM | 2939 | CD | GLN | B | 166 | -17.656 | 13.318 | 1.960 | 1.00 | 45.88 | C |
| ATOM | 2940 | NE2 | GLN | B | 166 | -16.458 | 13.247 | 1.393 | 1.00 | 37.75 | N |
| ATOM | 2941 | OE1 | GLN | B | 166 | -18.378 | 12.332 | 2.110 | 1.00 | 44.50 | O |
| ATOM | 2942 | N | GLU | B | 167 | -17.741 | 16.260 | 6.642 | 1.00 | 26.95 | N |
| ATOM | 2943 | CA | GLU | B | 167 | -17.217 | 16.491 | 7.985 | 1.00 | 34.35 | C |
| ATOM | 2944 | C | GLU | B | 167 | -16.789 | 15.199 | 8.666 | 1.00 | 31.65 | C |
| ATOM | 2945 | O | GLU | B | 167 | -17.351 | 14.136 | 8.416 | 1.00 | 31.31 | O |
| ATOM | 2946 | CB | GLU | B | 167 | -18.263 | 17.166 | 8.868 | 1.00 | 33.65 | C |
| ATOM | 2947 | CG | GLU | B | 167 | -18.526 | 18.617 | 8.585 | 1.00 | 50.67 | C |
| ATOM | 2948 | CD | GLU | B | 167 | -19.553 | 19.191 | 9.544 | 1.00 | 59.29 | C |
| ATOM | 2949 | OE1 | GLU | B | 167 | -20.018 | 18.431 | 10.425 | 1.00 | 49.27 | O |
| ATOM | 2950 | OE2 | GLU | B | 167 | -19.895 | 20.388 | 9.418 | 1.00 | 59.18 | O |
| ATOM | 2951 | N | SER | B | 168 | -15.815 | 15.317 | 9.556 | 1.00 | 28.62 | N |
| ATOM | 2952 | CA | SER | B | 168 | -15.393 | 14.216 | 10.407 | 1.00 | 32.03 | C |
| ATOM | 2953 | C | SER | B | 168 | -15.096 | 14.753 | 11.802 | 1.00 | 31. 07 | C |
| ATOM | 2954 | O | SER | B | 168 | -14.499 | 15.819 | 11.945 | 1.00 | 28.49 | O |
| ATOM | 2955 | CB | SER | B | 168 | -14.165 | 13.522 | 9.817 | 1.00 | 34.82 | C |
| ATOM | 2956 | OG | SER | B | 168 | -13.566 | 12.666 | 10.762 | 1.00 | 40.29 | O |
| ATOM | 2957 | N AVAL | AVAL | B | 169 | -15.514 | 14.013 | 12.828 | 0.89 | 30.56 | N |

(continued)

| | | | | | | | | | | | |
|---|---|---|---|---|---|---|---|---|---|---|---|
| ATOM | 2958 | CA | AVAL | B | 169 | -15.342 | 14.433 | 14.221 | 0.89 | 30.43 | C |
| ATOM | 2959 | C AVAL | AVAL | B | 169 | -14.558 | 13.389 | 15.017 | 0.89 | 31.75 | C |
| ATOM | 2960 | O | AVAL | B | 169 | -14.796 | 12.193 | 14.870 | 0.89 | 33.51 | O |
| ATOM | 2961 | CB | AVAL | B | 169 | -16.708 | 14.663 | 14.910 | 0.89 | 32.02 | C |
| ATOM | 2962 | CG1AVAL | AVAL | B | 169 | -16.518 | 15.215 | 16.317 | 0.89 | 33.65 | C |
| ATOM | 2963 | CG2AVAL | AVAL | B | 169 | -17.578 | 15.591 | 14.079 | 0.89 | 31.73 | C |
| ATOM | 2964 | N | BVAL | B | 169 | -15.523 | 14.018 | 12.822 | 0.11 | 30.45 | N |
| ATOM | 2965 | CA | BVAL | B | 169 | -15.292 | 14.424 | 14.200 | 0.11 | 31.12 | C |
| ATOM | 2966 | C | BVAL | B | 169 | -14.467 | 13.380 | 14.937 | 0.11 | 31.77 | C |
| ATOM | 2967 | O | BVAL | B | 169 | -14.578 | 12.184 | 14.669 | 0.11 | 33.10 | O |
| ATOM | 2968 | CB | BVAL | B | 169 | -16.616 | 14. 648 | 14.953 | 0.11 | 32.66 | C |
| ATOM | 2969 | CG1BVAL | BVAL | B | 169 | -17.352 | 15.847 | 14.385 | 0.11 | 32.73 | C |
| ATOM | 2970 | CG2BVAL | BVAL | B | 169 | -17.482 | 13.403 | 14.878 | 0.11 | 33.85 | C |
| ATOM | 2971 | N | THR | B | 170 | -13.633 | 13.837 | 15.862 | 1.00 | 29.38 | N |
| ATOM | 2972 | CA | THR | B | 170 | -12.847 | 12.921 | 16.682 | 1.00 | 36.76 | C |
| ATOM | 2973 | C | THR | B | 170 | -13.703 | 12.346 | 17 .805 | 1.00 | 33.01 | C |
| ATOM | 2974 | O | THR | B | 170 | -14.762 | 12.885 | 18.133 | 1.00 | 30.78 | O |
| ATOM | 2975 | CB | THR | B | 170 | -11.620 | 13.605 | 17.308 | 1.00 | 30.27 | C |
| ATOM | 2976 | CG2 | THR | B | 170 | -10.719 | 14.211 | 16.235 | 1.00 | 34.58 | C |
| ATOM | 2977 | OG1 | THR | B | 170 | -12.057 | 14.633 | 18.199 | 1.00 | 31.73 | O |
| ATOM | 2978 | N | GLU | B | 171 | -13.243 | 11.248 | 18.390 | 1.00 | 34.68 | N |
| ATOM | 2979 | CA | GLU | B | 171 | -13.843 | 10.746 | 19.618 | 1.00 | 35.38 | C |
| ATOM | 2980 | C | GLU | B | 171 | -13.613 | 11.768 | 20.720 | 1.00 | 37.95 | C |
| ATOM | 2981 | O | GLU | B | 171 | -12.689 | 12.584 | 20.639 | 1.00 | 34.11 | O |
| ATOM | 2982 | CB | GLU | B | 171 | -13.247 | 9.391 | 20.011 | 1.00 | 35.96 | c |
| ATOM | 2983 | CG | GLU | B | 171 | -13.457 | 8.290 | 18.984 | 1.00 | 47.50 | c |
| ATOM | 2984 | CD | GLU | B | 171 | -14.850 | 7.683 | 19.038 | 1.00 | 65.62 | c |
| ATOM | 2985 | OE1 | GLU | B | 171 | -15.595 | 7.969 | 20.002 | 1.00 | 65.84 | O |
| ATOM | 2986 | OE2 | GLU | B | 171 | -15.198 | 6.912 | 18.116 | 1.00 | 70.74 | O |
| ATOM | 2987 | N | GLN | B | 172 | -14.456 | 11.734 | 21.743 | 1.00 | 34.37 | N |
| ATOM | 2988 | CA | GLN | B | 172 | -14.272 | 12.609 | 22.885 | 1.00 | 27.96 | C |
| ATOM | 2989 | C | GLN | B | 172 | -12.897 | 12.347 | 23.509 | 1.00 | 29.48 | C |
| ATOM | 2990 | O | GLN | B | 172 | -12.544 | 11.210 | 23.785 | 1.00 | 33.27 | O |
| ATOM | 2991 | CB | GLN | B | 172 | -15.399 | 12.399 | 23.899 | 1.00 | 31.65 | C |

(continued)

| | | | | | | | | | | | |
|---|---|---|---|---|---|---|---|---|---|---|---|
| ATOM | 2992 | CG | GLN | B | 172 | -15.483 | 13.474 | 24.959 | 1.00 | 34.27 | C |
| ATOM | 2993 | CD | GLN | B | 172 | -16.804 | 13.457 | 25.706 | 1.00 | 37.13 | C |
| ATOM | 2994 | NE2 | GLN | B | 172 | -17.166 | 14.594 | 26.286 | 1.00 | 32.91 | N |
| ATOM | 2995 | OE1 | GLN | B | 172 | -17.496 | 12.437 | 25.755 | 1.00 | 34.99 | O |
| ATOM | 2996 | N | ASP | B | 173 | -12.120 | 13.408 | 23.700 | 1.00 | 30.64 | N |
| ATOM | 2997 | CA | ASP | B | 173 | -10.766 | 13.296 | 24.231 | 1.00 | 32.58 | C |
| ATOM | 2998 | C | ASP | B | 173 | -10.749 | 12.684 | 25.636 | 1.00 | 36.32 | C |
| ATOM | 2999 | O | ASP | B | 173 | -11.537 | 13.069 | 26.502 | 1.00 | 30.09 | O |
| ATOM | 3000 | CB | ASP | B | 173 | -10.097 | 14.671 | 24.246 | 1.00 | 32.10 | C |
| ATOM | 3001 | CG | ASP | B | 173 | -8.613 | 14.593 | 24.538 | 1.00 | 36.21 | C |
| ATOM | 3002 | OD2 | ASP | B | 173 | -8.222 | 14.802 | 25.708 | 1.00 | 36.85 | O |
| ATOM | 3003 | OD1 | ASP | B | 173 | -7.836 | 14.324 | 23.595 | 1.00 | 37.22 | O |
| ATOM | 3004 | N | SER | B | 174 | -9.844 | 11.737 | 25.863 | 1.00 | 32.20 | N |
| ATOM | 3005 | CA | SER | B | 174 | -9.818 | 11.015 | 27.134 | 1.00 | 38.98 | C |
| ATOM | 3006 | C | SER | B | 174 | -9.243 | 11.855 | 28.275 | 1.00 | 32.61 | C |
| ATOM | 3007 | O | SER | B | 174 | -9.301 | 11.447 | 29.432 | 1.00 | 42.30 | O |
| ATOM | 3008 | CB | SER | B | 174 | -9.017 | 9.717 | 26.998 | 1.00 | 36.54 | C |
| ATOM | 3009 | OG | SER | B | 174 | -7.630 | 9.986 | 26.883 | 1.00 | 46.81 | O |
| ATOM | 3010 | N | LYS | B | 175 | -8.691 | 13.020 | 27.954 | 1.00 | 35.47 | N |
| ATOM | 3011 | CA | LYS | B | 175 | -8.142 | 13.907 | 28.981 | 1.00 | 33.77 | C |
| ATOM | 3012 | C | LYS | B | 175 | -9.040 | 15.113 | 29.278 | 1.00 | 36.43 | C |
| ATOM | 3013 | O | LYS | B | 175 | -9.479 | 15.287 | 30.410 | 1.00 | 30.79 | O |
| ATOM | 3014 | CB | LYS | B | 175 | -6.749 | 14.404 | 28.581 | 1.00 | 36.54 | C |
| ATOM | 3015 | CG | LYS | B | 175 | -5.717 | 13.303 | 28.387 | 1.00 | 47.29 | C |
| ATOM | 3016 | CD | LYS | B | 175 | -4.339 | 13. 895 | 28.117 | 1.00 | 54.56 | C |
| ATOM | 3017 | CE | LYS | B | 175 | -3.317 | 12.811 | 27 . 821 | 1.00 | 53.35 | C |
| ATOM | 3018 | NZ | LYS | B | 175 | -1.931 | 13.358 | 27.809 | 1.00 | 53.83 | N |
| ATOM | 3019 | N | ASP | B | 176 | -9.306 | 15.950 | 28.275 | 1.00 | 35.92 | N |
| ATOM | 3020 | CA | ASP | B | 176 | -10.070 | 17.176 | 28.522 | 1.00 | 33.42 | C |
| ATOM | 3021 | C | ASP | B | 176 | -11.526 | 17.072 | 28.069 | 1.00 | 33.69 | C |
| ATOM | 3022 | O | ASP | B | 176 | -12.293 | 18.025 | 28.205 | 1.00 | 32.10 | O |
| ATOM | 3023 | CB | ASP | B | 176 | -9.391 | 18.387 | 27.857 | 1.00 | 37.93 | C |
| ATOM | 3024 | CG | ASP | B | 176 | -9.374 | 18.315 | 26.326 | 1.00 | 44. 81 | C |
| ATOM | 3025 | OD1 | ASP | B | 176 | -10.135 | 17.530 | 25.718 | 1.00 | 39.09 | O |

(continued)

| | | | | | | | | | | | |
|---|---|---|---|---|---|---|---|---|---|---|---|
| ATOM | 3026 | OD2 | ASP | B | 176 | -8.591 | 19.081 | 25.721 | 1.00 | 49.31 | O |
| ATOM | 3027 | N | SER | B | 177 | -11.879 | 15.923 | 27.501 | 1.00 | 31.41 | N |
| ATOM | 3028 | CA | SER | B | 177 | -13.259 | 15.601 | 27.145 | 1.00 | 32.38 | C |
| ATOM | 3029 | C | SER | B | 177 | -13.869 | 16.487 | 26.062 | 1.00 | 31.53 | C |
| ATOM | 3030 | O | SER | B | 177 | -15.087 | 16.651 | 26.017 | 1.00 | 29.23 | O |
| ATOM | 3031 | CB | SER | B | 177 | -14.142 | 15.665 | 28.390 | 1.00 | 29.13 | C |
| ATOM | 3032 | OG | SER | B | 177 | -13.689 | 14.767 | 29.386 | 1.00 | 32.14 | O |
| ATOM | 3033 | N | THR | B | 178 | -13.031 | 17.045 | 25.192 | 1.00 | 32.85 | N |
| ATOM | 3034 | CA | THR | B | 178 | -13.521 | 17 . 873 | 24.091 | 1.00 | 31.34 | C |
| ATOM | 3035 | C | THR | B | 178 | -13.603 | 17.102 | 22.779 | 1.00 | 29.53 | C |
| ATOM | 3036 | O | THR | B | 178 | -13.145 | 15.960 | 22.680 | 1.00 | 31.68 | O |
| ATOM | 3037 | CB | THR | B | 178 | -12.627 | 19.108 | 23.865 | 1.00 | 30.04 | C |
| ATOM | 3038 | CG2 | THR | B | 178 | -12.576 | 19.979 | 25.117 | 1.00 | 31.87 | C |
| ATOM | 3039 | OG1 | THR | B | 178 | -11.302 | 18.683 | 23.526 | 1.00 | 33.96 | O |
| ATOM | 3040 | N | TYR | B | 179 | -14.199 | 17.738 | 21.776 | 1.00 | 26.97 | N |
| ATOM | 3041 | CA | TYR | B | 179 | -14.198 | 17.225 | 20.411 | 1.00 | 27.62 | C |
| ATOM | 3042 | C | TYR | B | 179 | -13.395 | 18.139 | 19.503 | 1.00 | 28.49 | C |
| ATOM | 3043 | O | TYR | B | 179 | -13.193 | 19.316 | 19.809 | 1.00 | 27.60 | O |
| ATOM | 3044 | CB | TYR | B | 179 | -15.620 | 17.110 | 19.862 | 1.00 | 23.93 | C |
| ATOM | 3045 | CG | TYR | B | 179 | -16.540 | 16.224 | 20.659 | 1.00 | 32.03 | C |
| ATOM | 3046 | CD2 | TYR | B | 179 | -16.701 | 14.884 | 20.326 | 1.00 | 29.16 | C |
| ATOM | 3047 | CD1 | TYR | B | 179 | -17.266 | 16.729 | 21.735 | 1.00 | 27.55 | C |
| ATOM | 3048 | CE2 | TYR | B | 179 | -17.549 | 14.065 | 21.046 | 1.00 | 36.83 | C |
| ATOM | 3049 | CE1 | TYR | B | 179 | -18.118 | 15.915 | 22.467 | 1.00 | 35.32 | C |
| ATOM | 3050 | CZ | TYR | B | 179 | -18.254 | 14.584 | 22.118 | 1.00 | 36.45 | C |
| ATOM | 3051 | OH | TYR | B | 179 | -19.096 | 13.768 | 22.837 | 1.00 | 41.00 | O |
| ATOM | 3052 | N | SER | B | 180 | -12.945 | 17.597 | 18.379 | 1.00 | 27.52 | N |
| ATOM | 3053 | CA | SER | B | 180 | -12.437 | 18.418 | 17.288 | 1.00 | 29.79 | C |
| ATOM | 3054 | C | SER | B | 180 | -13.143 | 18.002 | 16.000 | 1.00 | 28.83 | C |
| ATOM | 3055 | O | SER | B | 180 | -13.591 | 16.862 | 15.875 | 1.00 | 26.78 | O |
| ATOM | 3056 | CB | SER | B | 180 | -10.921 | 18.287 | 17.150 | 1.00 | 34.04 | C |
| ATOM | 3057 | OG | SER | B | 180 | -10.255 | 18.889 | 18.246 | 1.00 | 36.05 | O |
| ATOM | 3058 | N | LEU | B | 181 | -13.252 | 18.928 | 15.053 | 1.00 | 27.50 | N |
| ATOM | 3059 | CA | LEU | B | 181 | -13.984 | 18.670 | 13.819 | 1.00 | 29.24 | C |

(continued)

| | | | | | | | | | | | |
|---|---|---|---|---|---|---|---|---|---|---|---|
| ATOM | 3060 | C | LEU | B | 181 | -13.206 | 19.150 | 12.595 | 1.00 | 34.49 | C |
| ATOM | 3061 | O | LEU | B | 181 | -12.583 | 20.214 | 12.610 | 1.00 | 29.64 | O |
| ATOM | 3062 | CB | LEU | B | 181 | -15.367 | 19.337 | 13.866 | 1.00 | 31.76 | C |
| ATOM | 3063 | CG | LEU | B | 181 | -16.315 | 19.197 | 12.662 | 1.00 | 33.92 | C |
| ATOM | 3064 | CD1 | LEU | B | 181 | -17.770 | 19.237 | 13.115 | 1.00 | 33.67 | C |
| ATOM | 3065 | CD2 | LEU | B | 181 | -16.071 | 20.275 | 11.620 | 1.00 | 27.36 | C |
| ATOM | 3066 | N | SER | B | 182 | -13.266 | 18.355 | 11.534 | 1.00 | 30.45 | N |
| ATOM | 3067 | CA | SER | B | 182 | -12.622 | 18.683 | 10.273 | 1.00 | 32.39 | C |
| ATOM | 3068 | C | SER | B | 182 | -13.653 | 18.706 | 9.157 | 1.00 | 34.50 | C |
| ATOM | 3069 | O | SER | B | 182 | -14.346 | 17.715 | 8.935 | 1.00 | 32.11 | O |
| ATOM | 3070 | CB | SER | B | 182 | -11.521 | 17.671 | 9.953 | 1.00 | 35.13 | C |
| ATOM | 3071 | OG | SER | B | 182 | -10.952 | 17.929 | 8.684 | 1.00 | 35.29 | O |
| ATOM | 3072 | N | SER | B | 183 | -13.769 | 19.841 | 8.473 | 1.00 | 32.48 | N |
| ATOM | 3073 | CA | SER | B | 183 | -14.666 | 19.946 | 7.327 | 1.00 | 31.00 | C |
| ATOM | 3074 | C | SER | B | 183 | -13.877 | 20.240 | 6.058 | 1.00 | 37.54 | C |
| ATOM | 3075 | O | SER | B | 183 | -13.014 | 21.119 | 6.041 | 1.00 | 34.24 | O |
| ATOM | 3076 | CB | SER | B | 183 | -15.720 | 21. 028 | 7.548 | 1.00 | 28.88 | C |
| ATOM | 3077 | OG | SER | B | 183 | -16.568 | 21.127 | 6.415 | 1.00 | 34.00 | O |
| ATOM | 3078 | N | THR | B | 184 | -14.185 | 19.495 | 5.002 | 1.00 | 29.33 | N |
| ATOM | 3079 | CA | THR | B | 184 | -13.490 | 19.618 | 3.733 | 1.00 | 32.43 | C |
| ATOM | 3080 | C | THR | B | 184 | -14.449 | 20.002 | 2.609 | 1.00 | 33.53 | C |
| ATOM | 3081 | O | THR | B | 184 | -15.375 | 19.258 | 2.296 | 1.00 | 30.24 | O |
| ATOM | 3082 | CB | THR | B | 184 | -12.778 | 18.303 | 3.347 | 1.00 | 30.27 | C |
| ATOM | 3083 | CG2 | THR | B | 184 | -12.075 | 18.448 | 1.993 | 1.00 | 34.53 | C |
| ATOM | 3084 | OG1 | THR | B | 184 | -11.813 | 17.966 | 4.348 | 1.00 | 43.02 | O |
| ATOM | 3085 | N | LEU | B | 185 | -14.212 | 21.164 | 2.013 | 1.00 | 29.43 | N |
| ATOM | 3086 | CA | LEU | B | 185 | -14.950 | 21.610 | 0.838 | 1.00 | 26.42 | C |
| ATOM | 3087 | C | LEU | B | 185 | -14.195 | 21.216 | -0.428 | 1.00 | 31.67 | C |
| ATOM | 3088 | O | LEU | B | 185 | -13.021 | 21.549 | -0.587 | 1.00 | 32.58 | O |
| ATOM | 3089 | CB | LEU | B | 185 | -15.162 | 23.123 | 0.882 | 1.00 | 26.19 | C |
| ATOM | 3090 | CG | LEU | B | 185 | -15.801 | 23.777 | -0.346 | 1.00 | 33.72 | C |
| ATOM | 3091 | CD1 | LEU | B | 185 | -17.290 | 23.451 | -0.426 | 1.00 | 29.32 | C |
| ATOM | 3092 | CD2 | LEU | B | 185 | -15.570 | 25.285 | -0.336 | 1.00 | 29.64 | C |
| ATOM | 3093 | N | THR | B | 186 | -14.862 | 20.503 | -1.330 | 1.00 | 27.07 | N |

(continued)

| | | | | | | | | | | | |
|---|---|---|---|---|---|---|---|---|---|---|---|
| ATOM | 3094 | CA | THR | B | 186 | -14.200 | 20.043 | -2.544 | 1.00 | 32.13 | C |
| ATOM | 3095 | C | THR | B | 186 | -14.797 | 20.676 | -3.794 | 1.00 | 34.19 | C |
| ATOM | 3096 | O | THR | B | 186 | -15.999 | 20.569 | -4.046 | 1.00 | 32.51 | O |
| ATOM | 3097 | CB | THR | B | 186 | -14.265 | 18.512 | -2.672 | 1.00 | 35.70 | C |
| ATOM | 3098 | CG2 | THR | B | 186 | -13.532 | 18.054 | -3.919 | 1.00 | 33.31 | C |
| ATOM | 3099 | OG1 | THR | B | 186 | -13.652 | 17.913 | -1.522 | 1.00 | 40.62 | O |
| ATOM | 3100 | N | LEU | B | 187 | -13.941 | 21.346 | -4.562 | 1.00 | 35.11 | N |
| ATOM | 3101 | CA | LEU | B | 187 | -14.315 | 21.945 | -5.843 | 1.00 | 35.77 | C |
| ATOM | 3102 | C | LEU | B | 187 | -13.339 | 21.525 | -6.932 | 1.00 | 37.88 | C |
| ATOM | 3103 | O | LEU | B | 187 | -12.206 | 21.145 | -6.642 | 1.00 | 36.56 | O |
| ATOM | 3104 | CB | LEU | B | 187 | -14.333 | 23.471 | -5.755 | 1.00 | 30.60 | C |
| ATOM | 3105 | CG | LEU | B | 187 | -15.286 | 24.143 | -4.775 | 1.00 | 36.33 | C |
| ATOM | 3106 | CD1 | LEU | B | 187 | -15.031 | 25.641 | -4.767 | 1.00 | 43.76 | C |
| ATOM | 3107 | CD2 | LEU | B | 187 | -16.729 | 23.835 | -5.147 | 1.00 | 50.34 | C |
| ATOM | 3108 | N | SER | B | 188 | -13.767 | 21.609 | -8.187 | 1.00 | 38.12 | N |
| ATOM | 3109 | CA | SER | B | 188 | -12.839 | 21.434 | -9.297 | 1.00 | 37.37 | C |
| ATOM | 3110 | C | SER | B | 188 | -11.888 | 22.627 | -9.343 | 1.00 | 37.79 | C |
| ATOM | 3111 | O | SER | B | 188 | -12.169 | 23.681 | -8.761 | 1.00 | 33.69 | O |
| ATOM | 3112 | CB | SER | B | 188 | -13.584 | 21.297 | -10.625 | 1.00 | 36.22 | C |
| ATOM | 3113 | OG | SER | B | 188 | -14.208 | 22.519 | -10.973 | 1.00 | 39.78 | O |
| ATOM | 3114 | N | LYS | B | 189 | -10.765 | 22.460 | -10.031 | 1.00 | 38.51 | N |
| ATOM | 3115 | CA | LYS | B | 189 | -9.809 | 23.546 | -10.193 | 1.00 | 37.38 | C |
| ATOM | 3116 | C | LYS | B | 189 | -10.460 | 24.721 | -10.915 | 1.00 | 37.61 | C |
| ATOM | 3117 | O | LYS | B | 189 | -10.331 | 25.869 | -10.491 | 1.00 | 42.81 | O |
| ATOM | 3118 | CB | LYS | B | 189 | -8.576 | 23.064 | -10.959 | 1.00 | 43.42 | C |
| ATOM | 3119 | CG | LYS | B | 189 | -7.574 | 24.158 | -11.293 | 1.00 | 46.58 | C |
| ATOM | 3120 | CD | LYS | B | 189 | -6.453 | 23.615 | -12.166 | 1.00 | 47.94 | C |
| ATOM | 3121 | CE | LYS | B | 189 | -5.477 | 24.704 | -12.561 | 1.00 | 50.50 | C |
| ATOM | 3122 | NZ | LYS | B | 189 | -4.369 | 24.160 | -13.395 | 1.00 | 67.88 | N |
| ATOM | 3123 | N | ALA | B | 190 | -11.175 | 24.415 | -11.995 | 1.00 | 37.86 | N |
| ATOM | 3124 | CA | ALA | B | 190 | -11.862 | 25.429 | -12.791 | 1.00 | 38.31 | C |
| ATOM | 3125 | C | ALA | B | 190 | -12.825 | 26.252 | -11.947 | 1.00 | 41.13 | C |
| ATOM | 3126 | O | ALA | B | 190 | -12.859 | 27.477 | -12.057 | 1.00 | 45.90 | O |
| ATOM | 3127 | CB | ALA | B | 190 | -12.602 | 24.777 | -13.953 | 1.00 | 30.47 | C |

(continued)

| | | | | | | | | | | | |
|---|---|---|---|---|---|---|---|---|---|---|---|
| ATOM | 3128 | N | ASP | B | 191 | -13.602 | 25.582 | -11.100 | 1.00 | 38.92 | N |
| ATOM | 3129 | CA | ASP | B | 191 | -14.536 | 26.287 | -10.226 | 1.00 | 37.31 | C |
| ATOM | 3130 | C | ASP | B | 191 | -13.796 | 27.103 | -9.175 | 1.00 | 35.97 | C |
| ATOM | 3131 | O | ASP | B | 191 | -14.211 | 28.214 | -8.831 | 1.00 | 35.16 | O |
| ATOM | 3132 | CB | ASP | B | 191 | -15.497 | 25.308 | -9.542 | 1.00 | 40.65 | C |
| ATOM | 3133 | CG | ASP | B | 191 | -16.675 | 24.932 | -10.425 | 1.00 | 54.48 | C |
| ATOM | 3134 | OD1 | ASP | B | 191 | -16.840 | 25.554 | -11.498 | 1.00 | 48.85 | O |
| ATOM | 3135 | OD2 | ASP | B | 191 | -17.445 | 24.024 | -10.039 | 1.00 | 50.23 | O |
| ATOM | 3136 | N | TYR | B | 192 | -12.700 | 26.549 | -8.667 | 1.00 | 35.50 | N |
| ATOM | 3137 | CA | TYR | B | 192 | -11.948 | 27.193 | -7.595 | 1.00 | 34.33 | C |
| ATOM | 3138 | C | TYR | B | 192 | -11.381 | 28.543 | -8.022 | 1.00 | 37.59 | C |
| ATOM | 3139 | O | TYR | B | 192 | -11.395 | 29.505 | -7.256 | 1.00 | 35.83 | O |
| ATOM | 3140 | CB | TYR | B | 192 | -10.814 | 26.288 | -7.115 | 1.00 | 34.12 | C |
| ATOM | 3141 | CG | TYR | B | 192 | -9.932 | 26.940 | -6.076 | 1.00 | 36.45 | C |
| ATOM | 3142 | CD1 | TYR | B | 192 | -10.429 | 27.251 | -4.815 | 1.00 | 34.80 | C |
| ATOM | 3143 | CD2 | TYR | B | 192 | -8.606 | 27.243 | -6.350 | 1.00 | 32.17 | C |
| ATOM | 3144 | CE1 | TYR | B | 192 | -9.632 | 27.849 | -3.857 | 1.00 | 32.72 | C |
| ATOM | 3145 | CE2 | TYR | B | 192 | -7.797 | 27.838 | -5.394 | 1.00 | 34.13 | C |
| ATOM | 3146 | CZ | TYR | B | 192 | -8.319 | 28.142 | -4.152 | 1.00 | 31.36 | C |
| ATOM | 3147 | OH | TYR | B | 192 | -7.526 | 28.732 | -3.197 | 1.00 | 35.96 | O |
| ATOM | 3148 | N | GLU | B | 193 | -10.895 | 28.609 | -9.255 | 1.00 | 39.80 | N |
| ATOM | 3149 | CA | GLU | B | 193 | -10.237 | 29.811 | -9.763 | 1.00 | 43.18 | C |
| ATOM | 3150 | C | GLU | B | 193 | -11.213 | 30.900 | -10.210 | 1.00 | 39.55 | C |
| ATOM | 3151 | O | GLU | B | 193 | -10.799 | 32.022 | -10.496 | 1.00 | 44.13 | O |
| ATOM | 3152 | CB | GLU | B | 193 | -9.311 | 29.440 | -10.921 | 1.00 | 44.18 | C |
| ATOM | 3153 | CG | GLU | B | 193 | -8.172 | 28.528 | -10.515 | 1.00 | 46.87 | C |
| ATOM | 3154 | CD | GLU | B | 193 | -7.393 | 28.010 | -11.704 | 1.00 | 66.53 | C |
| ATOM | 3155 | OE1 | GLU | B | 193 | -7.965 | 27.968 | -12.815 | 1.00 | 70.34 | O |
| ATOM | 3156 | OE2 | GLU | B | 193 | -6.210 | 27.648 | -11.529 | 1.00 | 73.19 | O |
| ATOM | 3157 | N | LYS | B | 194 | -12.502 | 30.575 | -10.261 | 1.00 | 41.15 | N |
| ATOM | 3158 | CA | LYS | B | 194 | -13.522 | 31.554 | -10.634 | 1.00 | 39.86 | C |
| ATOM | 3159 | C | LYS | B | 194 | -13.955 | 32.429 | -9.461 | 1.00 | 44.25 | C |
| ATOM | 3160 | O | LYS | B | 194 | -14.816 | 33.295 | -9.615 | 1.00 | 42.88 | O |
| ATOM | 3161 | CB | LYS | B | 194 | -14.754 | 30.855 | -11.214 | 1.00 | 43.14 | C |

(continued)

| | | | | | | | | | | | |
|---|---|---|---|---|---|---|---|---|---|---|---|
| ATOM | 3162 | CG | LYS | B | 194 | -14.506 | 30.110 | -12.507 | 1.00 | 50.46 | C |
| ATOM | 3163 | CD | LYS | B | 194 | -15.768 | 29.418 | -12.991 | 1.00 | 54.94 | C |
| ATOM | 3164 | CE | LYS | B | 194 | -15.503 | 28.612 | -14.255 | 1.00 | 65.50 | C |
| ATOM | 3165 | NZ | LYS | B | 194 | -14.922 | 29.454 | -15.339 | 1.00 | 69.52 | N |
| ATOM | 3166 | N | HIS | B | 195 | -13.373 | 32.204 | -8.288 | 1.00 | 42.19 | N |
| ATOM | 3167 | CA | HIS | B | 195 | -13.799 | 32.939 | -7.099 | 1.00 | 42.19 | C |
| ATOM | 3168 | C | HIS | B | 195 | -12.625 | 33.455 | -6.285 | 1.00 | 36.87 | C |
| ATOM | 3169 | O | HIS | B | 195 | -11.498 | 32.976 | -6.423 | 1.00 | 37.36 | O |
| ATOM | 3170 | CB | HIS | B | 195 | -14.687 | 32.061 | -6.214 | 1.00 | 38.63 | C |
| ATOM | 3171 | CG | HIS | B | 195 | -15.894 | 31.518 | -6.914 | 1.00 | 41.06 | C |
| ATOM | 3172 | CD2 | HIS | B | 195 | -16.180 | 30.270 | -7.356 | 1.00 | 44.21 | C |
| ATOM | 3173 | ND1 | HIS | B | 195 | -16.987 | 32.296 | -7.224 | 1.00 | 43.75 | N |
| ATOM | 3174 | CE1 | HIS | B | 195 | -17 .894 | 31.554 | -7.834 | 1.00 | 44.35 | C |
| ATOM | 3175 | NE2 | HIS | B | 195 | -17.430 | 30.321 | -7.926 | 1.00 | 44.01 | N |
| ATOM | 3176 | N | LYS | B | 196 | -12.908 | 34.424 | -5.420 | 1.00 | 38.25 | N |
| ATOM | 3177 | CA | LYS | B | 196 | -11.876 | 35.084 | -4.639 | 1.00 | 35.29 | C |
| ATOM | 3178 | C | LYS | B | 196 | -11.915 | 34.716 | -3.152 | 1.00 | 39.07 | C |
| ATOM | 3179 | O | LYS | B | 196 | -10.942 | 34.190 | -2.615 | 1.00 | 39.45 | O |
| ATOM | 3180 | CB | LYS | B | 196 | -11.998 | 36.602 | -4.793 | 1.00 | 41.25 | C |
| ATOM | 3181 | CG | LYS | B | 196 | -10.931 | 37.391 | -4.047 | 1.00 | 43.11 | C |
| ATOM | 3182 | CD | LYS | B | 196 | -11.215 | 38.884 | -4.099 | 1.00 | 57.11 | C |
| ATOM | 3183 | CE | LYS | B | 196 | -10.103 | 39.686 | -3.447 | 1.00 | 60.58 | C |
| ATOM | 3184 | NZ | LYS | B | 196 | -10.341 | 41.150 | -3.566 | 1.00 | 75.28 | N |
| ATOM | 3185 | N | VAL | B | 197 | -13.032 | 35.002 | -2.488 | 1.00 | 33.64 | N |
| ATOM | 3186 | CA | VAL | B | 197 | -13.108 | 34.851 | -1.037 | 1.00 | 41.13 | C |
| ATOM | 3187 | C | VAL | B | 197 | -13.640 | 33.478 | -0.620 | 1.00 | 39.79 | C |
| ATOM | 3188 | O | VAL | B | 197 | -14.758 | 33.098 | -0.973 | 1.00 | 35.93 | O |
| ATOM | 3189 | CB | VAL | B | 197 | -13.993 | 35.943 | -0.400 | 1.00 | 39.79 | C |
| ATOM | 3190 | CG1 | VAL | B | 197 | -13.964 | 35.829 | 1.117 | 1.00 | 33.80 | C |
| ATOM | 3191 | CG2 | VAL | B | 197 | -13.532 | 37.330 | -0.834 | 1.00 | 36.75 | C |
| ATOM | 3192 | N | TYR | B | 198 | -12.827 | 32.743 | 0.134 | 1.00 | 36.80 | N |
| ATOM | 3193 | CA | TYR | B | 198 | -13.223 | 31.442 | 0.662 | 1.00 | 31.41 | C |
| ATOM | 3194 | C | TYR | B | 198 | -13.289 | 31.518 | 2.173 | 1.00 | 34.47 | C |
| ATOM | 3195 | O | TYR | B | 198 | -12.324 | 31. 909 | 2.821 | 1.00 | 35.65 | O |

(continued)

| | | | | | | | | | | |
|---|---|---|---|---|---|---|---|---|---|---|
| ATOM | 3196 | CB | TYR | B | 198 | -12.252 | 30.347 | 0.210 | 1.00 | 29.88 | c |
| ATOM | 3197 | CG | TYR | B | 198 | -12.394 | 30.041 | -1.259 | 1.00 | 32.49 | c |
| ATOM | 3198 | CD1 | TYR | B | 198 | -11.774 | 30.838 | -2.219 | 1.00 | 31.45 | c |
| ATOM | 3199 | CD2 | TYR | B | 198 | -13.179 | 28.978 | -1.694 | 1.00 | 30.07 | c |
| ATOM | 3200 | CE1 | TYR | B | 198 | -11.919 | 30.573 | -3.572 | 1.00 | 35.86 | c |
| ATOM | 3201 | CE2 | TYR | B | 198 | -13.327 | 28.706 | -3.043 | 1.00 | 31.84 | c |
| ATOM | 3202 | CZ | TYR | B | 198 | -12.695 | 29.509 | -3.975 | 1.00 | 31.78 | c |
| ATOM | 3203 | OH | TYR | B | 198 | -12.842 | 29.245 | -5.313 | 1.00 | 37.12 | O |
| ATOM | 3204 | N | ALA | B | 199 | -14.435 | 31. 148 | 2.731 | 1.00 | 31.33 | N |
| ATOM | 3205 | CA | ALA | B | 199 | -14.672 | 31.330 | 4.155 | 1.00 | 35.87 | C |
| ATOM | 3206 | C | ALA | B | 199 | -15.414 | 30.150 | 4.755 | 1.00 | 37.75 | C |
| ATOM | 3207 | O | ALA | B | 199 | -16.236 | 29.517 | 4.090 | 1.00 | 29.26 | O |
| ATOM | 3208 | CB | ALA | B | 199 | -15.454 | 32.614 | 4.394 | 1.00 | 28.89 | C |
| ATOM | 3209 | N | CYS | B | 200 | -15.114 | 29.844 | 6.012 | 1.00 | 29.79 | N |
| ATOM | 3210 | CA | CYS | B | 200 | -15.967 | 28.940 | 6.764 | 1.00 | 30.62 | C |
| ATOM | 3211 | C | CYS | B | 200 | -16.481 | 29.695 | 7.980 | 1.00 | 34.82 | C |
| ATOM | 3212 | O | CYS | B | 200 | -15.727 | 30.376 | 8.673 | 1.00 | 35.07 | O |
| ATOM | 3213 | CB | CYS | B | 200 | -15.232 | 27.651 | 7.157 | 1.00 | 39.10 | C |
| ATOM | 3214 | SG | CYS | B | 200 | -13.954 | 27.783 | 8.432 | 1.00 | 54.20 | S |
| ATOM | 3215 | N | GLU | B | 201 | -17.785 | 29.598 | 8.205 | 1.00 | 34.80 | N |
| ATOM | 3216 | CA | GLU | B | 201 | -18.438 | 30.312 | 9.289 | 1.00 | 35.26 | C |
| ATOM | 3217 | C | GLU | B | 201 | -18.905 | 29.313 | 10.338 | 1.00 | 33.11 | C |
| ATOM | 3218 | O | GLU | B | 201 | -19.670 | 28.395 | 10.036 | 1.00 | 30.25 | O |
| ATOM | 3219 | CB | GLU | B | 201 | -19.619 | 31.134 | 8.764 | 1.00 | 31.34 | C |
| ATOM | 3220 | CG | GLU | B | 201 | -20.333 | 31. 938 | 9.838 | 1.00 | 46.76 | C |
| ATOM | 3221 | CD | GLU | B | 201 | -21.725 | 32.382 | 9.423 | 1.00 | 57.59 | C |
| ATOM | 3222 | OE1 | GLU | B | 201 | -22.650 | 31.538 | 9.431 | 1.00 | 59.83 | O |
| ATOM | 3223 | OE2 | GLU | B | 201 | -21.893 | 33.575 | 9.093 | 1.00 | 62.46 | O |
| ATOM | 3224 | N | VAL | B | 202 | -18.450 | 29.490 | 11.572 | 1.00 | 33.14 | N |
| ATOM | 3225 | CA | VAL | B | 202 | -18.715 | 28.498 | 12.604 | 1.00 | 34.14 | C |
| ATOM | 3226 | C | VAL | B | 202 | -19.683 | 28.996 | 13.674 | 1.00 | 34.64 | C |
| ATOM | 3227 | O | VAL | B | 202 | -19.512 | 30.079 | 14.236 | 1.00 | 32.69 | O |
| ATOM | 3228 | CB | VAL | B | 202 | -17.408 | 28.046 | 13.276 | 1.00 | 32.83 | C |
| ATOM | 3229 | CG1 | VAL | B | 202 | -17.699 | 27.136 | 14.458 | 1.00 | 27.65 | C |

(continued)

| | | | | | | | | | | | |
|---|---|---|---|---|---|---|---|---|---|---|---|
| ATOM | 3230 | CG2 | VAL | B | 202 | -16.513 | 27.355 | 12.258 | 1.00 | 31.56 | C |
| ATOM | 3231 | N | THR | B | 203 | -20.702 | 28.185 | 13.942 | 1.00 | 30.80 | N |
| ATOM | 3232 | CA | THR | B | 203 | -21.670 | 28.455 | 14.997 | 1.00 | 34.07 | C |
| ATOM | 3233 | C | THR | B | 203 | -21.556 | 27.394 | 16.091 | 1.00 | 36.01 | C |
| ATOM | 3234 | O | THR | B | 203 | -21.518 | 26.196 | 15.802 | 1.00 | 34.64 | O |
| ATOM | 3235 | CB | THR | B | 203 | -23.111 | 28.477 | 14.444 | 1.00 | 36.63 | C |
| ATOM | 3236 | CG2 | THR | B | 203 | -24.102 | 28.883 | 15.526 | 1.00 | 36.15 | C |
| ATOM | 3237 | OG1 | THR | B | 203 | -23.190 | 29.403 | 13.356 | 1.00 | 45.86 | O |
| ATOM | 3238 | N | HIS | B | 204 | -21.499 | 27.841 | 17.343 | 1.00 | 32.76 | N |
| ATOM | 3239 | CA | HIS | B | 204 | -21.356 | 26.946 | 18.487 | 1.00 | 34.42 | C |
| ATOM | 3240 | C | HIS | B | 204 | -21.850 | 27.620 | 19.764 | 1.00 | 33.50 | C |
| ATOM | 3241 | O | HIS | B | 204 | -21.750 | 28.837 | 19.909 | 1.00 | 34.28 | O |
| ATOM | 3242 | CB | HIS | B | 204 | -19.895 | 26.510 | 18.656 | 1.00 | 29.00 | C |
| ATOM | 3243 | CG | HIS | B | 204 | -19.689 | 25.483 | 19.728 | 1.00 | 38.33 | C |
| ATOM | 3244 | CD2 | HIS | B | 204 | -19.847 | 24.138 | 19.717 | 1.00 | 32. 84 | C |
| ATOM | 3245 | ND1 | HIS | B | 204 | -19.266 | 25.810 | 20.999 | 1.00 | 29.49 | N |
| ATOM | 3246 | CE1 | HIS | B | 204 | -19.177 | 24.710 | 21.726 | 1.00 | 34.17 | C |
| ATOM | 3247 | NE2 | HIS | B | 204 | -19.522 | 23.681 | 20.973 | 1.00 | 33.34 | N |
| ATOM | 3248 | N | GLN | B | 205 | -22.367 | 26.816 | 20.687 | 1.00 | 33.90 | N |
| ATOM | 3249 | CA | GLN | B | 205 | -22.892 | 27.307 | 21.961 | 1.00 | 33.84 | C |
| ATOM | 3250 | C | GLN | B | 205 | -21.905 | 28.198 | 22.725 | 1.00 | 36.35 | C |
| ATOM | 3251 | O | GLN | B | 205 | -22.313 | 29.126 | 23.425 | 1.00 | 40.21 | O |
| ATOM | 3252 | CB | GLN | B | 205 | -23.309 | 26.121 | 22.835 | 1.00 | 33.40 | C |
| ATOM | 3253 | CG | GLN | B | 205 | -23.981 | 26.507 | 24.136 | 1.00 | 49.24 | C |
| ATOM | 3254 | CD | GLN | B | 205 | -24.475 | 25.304 | 24.914 | 1.00 | 50.37 | C |
| ATOM | 3255 | NE2 | GLN | B | 205 | -24.852 | 25.527 | 26.168 | 1.00 | 57.58 | N |
| ATOM | 3256 | OE1 | GLN | B | 205 | -24.518 | 24.186 | 24.396 | 1.00 | 53.77 | O |
| ATOM | 3257 | N | GLY | B | 206 | -20.610 | 27.931 | 22.578 | 1.00 | 30.24 | N |
| ATOM | 3258 | CA | GLY | B | 206 | -19.587 | 28.728 | 23.237 | 1.00 | 32.96 | C |
| ATOM | 3259 | C | GLY | B | 206 | -19.239 | 30.036 | 22.542 | 1.00 | 34.80 | C |
| ATOM | 3260 | O | GLY | B | 206 | -18.466 | 30.834 | 23.064 | 1.00 | 30.49 | O |
| ATOM | 3261 | N | LEU | B | 207 | -19.806 | 30.260 | 21.363 | 1.00 | 38.67 | N |
| ATOM | 3262 | CA | LEU | B | 207 | -19.553 | 31.492 | 20.619 | 1.00 | 41.75 | C |
| ATOM | 3263 | C | LEU | B | 207 | -20.765 | 32.412 | 20.674 | 1.00 | 40.10 | C |

(continued)

| | | | | | | | | | | |
|---|---|---|---|---|---|---|---|---|---|---|
| ATOM | 3264 | O | LEU | B | 207 | -21.848 | 32.050 | 20.210 | 1.00 | 42.41 | O |
| ATOM | 3265 | CB | LEU | B | 207 | -19.204 | 31.180 | 19.165 | 1.00 | 34.75 | c |
| ATOM | 3266 | CG | LEU | B | 207 | -17.957 | 30.336 | 18.917 | 1.00 | 35.04 | c |
| ATOM | 3267 | CD1 | LEU | B | 207 | -17.871 | 29.946 | 17.444 | 1.00 | 32.97 | c |
| ATOM | 3268 | CD2 | LEU | B | 207 | -16.709 | 31. 087 | 19.362 | 1.00 | 34.44 | c |
| ATOM | 3269 | N | SER | B | 208 | -20.582 | 33.600 | 21.239 | 1.00 | 41.65 | N |
| ATOM | 3270 | CA | SER | B | 208 | -21.685 | 34.551 | 21.366 | 1.00 | 50.19 | C |
| ATOM | 3271 | C | SER | B | 208 | -22.189 | 34.991 | 19.989 | 1.00 | 43.51 | C |
| ATOM | 3272 | O | SER | B | 208 | -23.367 | 35.307 | 19.824 | 1.00 | 51. 88 | O |
| ATOM | 3273 | CB | SER | B | 208 | -21.260 | 35.758 | 22.207 | 1.00 | 41.21 | C |
| ATOM | 3274 | OG | SER | B | 208 | -19.985 | 36.239 | 21.815 | 1.00 | 63.38 | O |
| ATOM | 3275 | N | SER | B | 209 | -21.296 | 34.995 | 19.002 | 1.00 | 40.06 | N |
| ATOM | 3276 | CA | SER | B | 209 | -21.682 | 35.228 | 17.613 | 1.00 | 44.47 | C |
| ATOM | 3277 | C | SER | B | 209 | -20.812 | 34.364 | 16.695 | 1.00 | 38.33 | C |
| ATOM | 3278 | O | SER | B | 209 | -19.696 | 34.005 | 17.067 | 1.00 | 43.33 | O |
| ATOM | 3279 | CB | SER | B | 209 | -21.557 | 36.715 | 17.253 | 1.00 | 45.07 | C |
| ATOM | 3280 | OG | SER | B | 209 | -20.203 | 37.102 | 17.120 | 1.00 | 49.50 | O |
| ATOM | 3281 | N | PRO | B | 210 | -21.323 | 34.017 | 15.499 | 1.00 | 44.65 | N |
| ATOM | 3282 | CA | PRO | B | 210 | -20.580 | 33.149 | 14.574 | 1.00 | 38.77 | C |
| ATOM | 3283 | C | PRO | B | 210 | -19.176 | 33.661 | 14.264 | 1.00 | 41.23 | C |
| ATOM | 3284 | O | PRO | B | 210 | -18.966 | 34.867 | 14.141 | 1.00 | 45.61 | O |
| ATOM | 3285 | CB | PRO | B | 210 | -21.450 | 33.156 | 13.315 | 1.00 | 39.30 | C |
| ATOM | 3286 | CG | PRO | B | 210 | -22.826 | 33.399 | 13.826 | 1.00 | 43.89 | C |
| ATOM | 3287 | CD | PRO | B | 210 | -22.658 | 34.357 | 14.974 | 1.00 | 40.75 | C |
| ATOM | 3288 | N | VAL | B | 211 | -18.224 | 32.741 | 14.155 | 1.00 | 39.40 | N |
| ATOM | 3289 | CA | VAL | B | 211 | -16.834 | 33.090 | 13.883 | 1.00 | 39.75 | C |
| ATOM | 3290 | C | VAL | B | 211 | -16.475 | 32.703 | 12.452 | 1.00 | 40.69 | C |
| ATOM | 3291 | O | VAL | B | 211 | -16.747 | 31.582 | 12.020 | 1.00 | 36.87 | O |
| ATOM | 3292 | CB | VAL | B | 211 | -15.877 | 32.398 | 14.880 | 1.00 | 38.99 | C |
| ATOM | 3293 | CG1 | VAL | B | 211 | -14.429 | 32.537 | 14.436 | 1.00 | 34.79 | C |
| ATOM | 3294 | CG2 | VAL | B | 211 | -16.071 | 32.973 | 16.272 | 1.00 | 40.49 | C |
| ATOM | 3295 | N | THR | B | 212 | -15.880 | 33.639 | 11.716 | 1.00 | 35.72 | N |
| ATOM | 3296 | CA | THR | B | 212 | -15.550 | 33.409 | 10.314 | 1.00 | 39.63 | C |
| ATOM | 3297 | C | THR | B | 212 | -14.055 | 33.500 | 10.046 | 1.00 | 35.76 | C |

(continued)

| | | | | | | | | | | | |
|---|---|---|---|---|---|---|---|---|---|---|---|
| ATOM | 3298 | O | THR | B | 212 | -13.424 | 34.518 | 10.323 | 1.00 | 42.94 | O |
| ATOM | 3299 | CB | THR | B | 212 | -16.272 | 34.414 | 9.391 | 1.00 | 38.85 | C |
| ATOM | 3300 | CG2 | THR | B | 212 | -15.814 | 34.238 | 7.953 | 1.00 | 37.83 | C |
| ATOM | 3301 | OG1 | THR | B | 212 | -17.687 | 34.196 | 9.461 | 1.00 | 46.70 | O |
| ATOM | 3302 | N | LYS | B | 213 | -13.491 | 32.424 | 9.513 | 1.00 | 33.60 | N |
| ATOM | 3303 | CA | LYS | B | 213 | -12.120 | 32.451 | 9.029 | 1.00 | 36.19 | C |
| ATOM | 3304 | C | LYS | B | 213 | -12.155 | 32.386 | 7.514 | 1.00 | 38.31 | C |
| ATOM | 3305 | O | LYS | B | 213 | -12.964 | 31.659 | 6.938 | 1.00 | 40.76 | O |
| ATOM | 3306 | CB | LYS | B | 213 | -11.298 | 31.295 | 9.602 | 1.00 | 35.13 | C |
| ATOM | 3307 | CG | LYS | B | 213 | -11.186 | 31.301 | 11.120 | 1.00 | 41.13 | C |
| ATOM | 3308 | CD | LYS | B | 213 | -10.462 | 32.537 | 11.635 | 1.00 | 36.48 | C |
| ATOM | 3309 | CE | LYS | B | 213 | -10.432 | 32.550 | 13.161 | 1.00 | 36.86 | C |
| ATOM | 3310 | NZ | LYS | B | 213 | -9.699 | 33.727 | 13.703 | 1.00 | 51. 87 | N |
| ATOM | 3311 | N | SER | B | 214 | -11.283 | 33.149 | 6.869 | 1.00 | 36.97 | N |
| ATOM | 3312 | CA | SER | B | 214 | -11.301 | 33.233 | 5.420 | 1.00 | 35.10 | C |
| ATOM | 3313 | C | SER | B | 214 | -9.934 | 33.547 | 4.827 | 1.00 | 37.64 | C |
| ATOM | 3314 | O | SER | B | 214 | -9.021 | 33.986 | 5.524 | 1.00 | 41.48 | O |
| ATOM | 3315 | CB | SER | B | 214 | -12.307 | 34.291 | 4.971 | 1.00 | 35.76 | C |
| ATOM | 3316 | OG | SER | B | 214 | -11.918 | 35.574 | 5.422 | 1.00 | 40.23 | O |
| ATOM | 3317 | N | PHE | B | 215 | -9.804 | 33.297 | 3.530 | 1.00 | 36.45 | N |
| ATOM | 3318 | CA | PHE | B | 215 | -8.651 | 33.746 | 2.767 | 1.00 | 39.24 | C |
| ATOM | 3319 | C | PHE | B | 215 | -9.114 | 34.202 | 1.388 | 1.00 | 45.83 | C |
| ATOM | 3320 | O | PHE | B | 215 | -10.185 | 33.804 | 0.916 | 1.00 | 37.02 | O |
| ATOM | 3321 | CB | PHE | B | 215 | -7.598 | 32.640 | 2.645 | 1.00 | 32.01 | C |
| ATOM | 3322 | CG | PHE | B | 215 | -8.066 | 31.428 | 1.878 | 1.00 | 38.83 | C |
| ATOM | 3323 | CD1 | PHE | B | 215 | -8.729 | 30.397 | 2.524 | 1.00 | 31.21 | C |
| ATOM | 3324 | CD2 | PHE | B | 215 | -7.830 | 31.315 | 0.516 | 1.00 | 35.76 | C |
| ATOM | 3325 | CE1 | PHE | B | 215 | -9.156 | 29.279 | 1.825 | 1.00 | 33.60 | C |
| ATOM | 3326 | CE2 | PHE | B | 215 | -8.251 | 30.201 | -0.190 | 1.00 | 35.19 | C |
| ATOM | 3327 | CZ | PHE | B | 215 | -8.915 | 29.180 | 0.466 | 1.00 | 34.00 | C |
| ATOM | 3328 | N | ASN | B | 216 | -8.316 | 35.057 | 0.761 | 1.00 | 45.06 | N |
| ATOM | 3329 | CA | ASN | B | 216 | -8.533 | 35.429 | -0.629 | 1.00 | 42.67 | C |
| ATOM | 3330 | C | ASN | B | 216 | -7.591 | 34.626 | -1.499 | 1.00 | 39.47 | C |
| ATOM | 3331 | O | ASN | B | 216 | -6.388 | 34.607 | -1.255 | 1.00 | 45.25 | O |

(continued)

| | | | | | | | | | | | | |
|---|---|---|---|---|---|---|---|---|---|---|---|---|
| ATOM | 3332 | CB | ASN | B | 216 | -8.310 | 36.926 | -0.843 | 1.00 | 45.51 | | C |
| ATOM | 3333 | CG | ASN | B | 216 | -9.256 | 37.778 | -0.024 | 1.00 | 49.43 | | C |
| ATOM | 3334 | ND2 | ASN | B | 216 | -8.785 | 38.943 | 0.402 | 1.00 | 55.43 | | N |
| ATOM | 3335 | OD1 | ASN | B | 216 | -10.396 | 37.393 | 0.227 | 1.00 | 51.90 | | O |
| ATOM | 3336 | N | ARG | B | 217 | -8.138 | 33.946 | -2.499 | 1.00 | 39.98 | | N |
| ATOM | 3337 | CA | ARG | B | 217 | -7.327 | 33.135 | -3.396 | 1.00 | 43.37 | | C |
| ATOM | 3338 | C | ARG | B | 217 | -6.257 | 33.985 | -4.069 | 1.00 | 49.33 | | C |
| ATOM | 3339 | O | ARG | B | 217 | -6.559 | 35.019 | -4.665 | 1.00 | 52.85 | | O |
| ATOM | 3340 | CB | ARG | B | 217 | -8.207 | 32.457 | -4.449 | 1.00 | 42.40 | | C |
| ATOM | 3341 | CG | ARG | B | 217 | -7.441 | 31.578 | -5.420 | 1.00 | 38.23 | | C |
| ATOM | 3342 | CD | ARG | B | 217 | -8.358 | 31.012 | -6.485 | 1.00 | 38.31 | | C |
| ATOM | 3343 | NE | ARG | B | 217 | -9.059 | 32.068 | -7.204 | 1.00 | 42.76 | | N |
| ATOM | 3344 | CZ | ARG | B | 217 | -8.588 | 32.665 | -8.292 | 1.00 | 51.33 | | C |
| ATOM | 3345 | NH1 | ARG | B | 217 | -7.414 | 32.303 | -8.793 | 1.00 | 42.73 | | N |
| ATOM | 3346 | NH2 | ARG | B | 217 | -9.294 | 33.622 | -8.883 | 1.00 | 48.38 | | N |
| ATOM | 3347 | N | GLY | B | 218 | -5.004 | 33.555 | -3.952 | 1.00 | 53.01 | | N |
| ATOM | 3348 | CA | GLY | B | 218 | -3.897 | 34.253 | -4.582 | 1.00 | 57.34 | | C |
| ATOM | 3349 | C | GLY | B | 218 | -3.338 | 35.410 | -3.771 | 1.00 | 63.03 | | C |
| ATOM | 3350 | O | GLY | B | 218 | -2.818 | 36.373 | -4.334 | 1.00 | 67.80 | | O |
| ATOM | 3351 | N | GLU | B | 219 | -3.443 | 35.321 | -2.449 | 1.00 | 60.01 | | N |
| ATOM | 3352 | CA | GLU | B | 219 | -2.900 | 36.353 | -1.571 | 1.00 | 61.79 | | C |
| ATOM | 3353 | C | GLU | B | 219 | -2.130 | 35.742 | -0.405 | 1.00 | 61.79 | | C |
| ATOM | 3354 | O | GLU | B | 219 | -1.976 | 34.525 | -0.321 | 1.00 | 69.81 | | O |
| ATOM | 3355 | CB | GLU | B | 219 | -4.015 | 37.259 | -1.042 | 1.00 | 62.53 | | C |
| ATOM | 3356 | CG | GLU | B | 219 | -4.663 | 38.151 | -2.096 | 1.00 | 56.22 | | C |
| ATOM | 3357 | CD | GLU | B | 219 | -5.625 | 39.161 | -1.489 | 1.00 | 74.19 | | C |
| ATOM | 3358 | OE1 | GLU | B | 219 | -5.593 | 39.345 | -0.252 | 1.00 | 78.86 | | O |
| ATOM | 3359 | OE2 | GLU | B | 219 | -6.413 | 39.770 | -2.247 | 1.00 | 64.28 | | O |
| TER | | | | | | | | | | | | |
| ATOM | 3360 | N | THR | C | 152 | -29.241 | -45.858 | 26.627 | 1.00 | 80.47 | D000 | N |
| ATOM | 3361 | CA | THR | C | 152 | -29.487 | -45.616 | 28.045 | 1.00 | 91.58 | D000 | C |
| ATOM | 3362 | C | THR | C | 152 | -29.269 | -44.146 | 28.405 | 1.00 | 88.87 | D000 | C |
| ATOM | 3363 | O | THR | C | 152 | -28.934 | -43.329 | 27.545 | 1.00 | 88.60 | D000 | O |
| ATOM | 3364 | CB | THR | C | 152 | -28.582 | -46.499 | 28.936 | 1.00 | 90.05 | D000 | C |

(continued)

| | | | | | | | | | | | | |
|---|---|---|---|---|---|---|---|---|---|---|---|---|
| ATOM | 3365 | CG2 | THR | C | 152 | -29.009 | -47.960 | 28.857 | 1.00 | 70.32 | D000 | C |
| ATOM | 3366 | OG1 | THR | C | 152 | -27.220 | -46.382 | 28.506 | 1.00 | 79.38 | D000 | O |
| ATOM | 3367 | N | CYS | C | 153 | -29.465 | -43.813 | 29.677 | 1.00 | 83.40 | D000 | N |
| ATOM | 3368 | CA | CYS | C | 153 | -29.261 | -42.446 | 30.143 | 1.00 | 72.69 | D000 | C |
| ATOM | 3369 | C | CYS | C | 153 | -28.836 | -42.415 | 31.611 | 1.00 | 63.73 | D000 | C |
| ATOM | 3370 | O | CYS | C | 153 | -28.923 | -43.419 | 32.318 | 1.00 | 52.26 | D000 | O |
| ATOM | 3371 | CB | CYS | C | 153 | -30.532 | -41.616 | 29.941 | 1.00 | 73.56 | D000 | C |
| ATOM | 3372 | SG | CYS | C | 153 | -30.281 | -39.826 | 30.081 | 1.00 | 98.30 | D000 | S |
| ATOM | 3373 | N | CYS | C | 154 | -28.370 | -41.254 | 32.059 | 1.00 | 61.15 | D000 | N |
| ATOM | 3374 | CA | CYS | C | 154 | -27.916 | -41.085 | 33.434 | 1.00 | 58.46 | D000 | C |
| ATOM | 3375 | C | CYS | C | 154 | -29.071 | -41.141 | 34.432 | 1.00 | 51.28 | D000 | C |
| ATOM | 3376 | O | CYS | C | 154 | -30.208 | -40.827 | 34.086 | 1.00 | 47.54 | D000 | O |
| ATOM | 3377 | CB | CYS | C | 154 | -27.160 | -39.758 | 33.580 | 1.00 | 53.06 | D000 | C |
| ATOM | 3378 | SG | CYS | C | 154 | -25.446 | -39.818 | 33.000 | 1.00 | 71.64 | D000 | S |
| ATOM | 3379 | N | PRO | C | 155 | -28.781 | -41.556 | 35.674 | 1.00 | 43.23 | D000 | N |
| ATOM | 3380 | CA | PRO | C | 155 | -29.767 | -41.494 | 36.757 | 1.00 | 48.50 | D000 | C |
| ATOM | 3381 | C | PRO | C | 155 | -30.193 | -40.062 | 37.062 | 1.00 | 49.23 | D000 | C |
| ATOM | 3382 | O | PRO | C | 155 | -29.481 | -39.117 | 36.708 | 1.00 | 42.32 | D000 | O |
| ATOM | 3383 | CB | PRO | C | 155 | -29.020 | -42.104 | 37.953 | 1.00 | 42.80 | D000 | C |
| ATOM | 3384 | CG | PRO | C | 155 | -27.576 | -41.991 | 37.609 | 1.00 | 50.48 | D000 | C |
| ATOM | 3385 | CD | PRO | C | 155 | -27.514 | -42.158 | 36.122 | 1.00 | 50.94 | D000 | C |
| ATOM | 3386 | N | VAL | C | 156 | -31.343 | -39.913 | 37.713 | 1.00 | 40.74 | D000 | N |
| ATOM | 3387 | CA | VAL | C | 156 | -31.863 | -38.598 | 38.077 | 1.00 | 48.19 | D000 | C |
| ATOM | 3388 | C | VAL | C | 156 | -30.837 | -37.822 | 38.897 | 1.00 | 42.17 | D000 | C |
| ATOM | 3389 | O | VAL | C | 156 | -30.149 | -38.403 | 39.739 | 1.00 | 40.87 | D000 | O |
| ATOM | 3390 | CB | VAL | C | 156 | -33.183 | -38.717 | 38.875 | 1.00 | 54.12 | D000 | C |
| ATOM | 3391 | CG1 | VAL | C | 156 | -33.775 | -37.341 | 39.149 | 1.00 | 44. 84 | D000 | C |
| ATOM | 3392 | CG2 | VAL | C | 156 | -34.181 | -39.590 | 38.122 | 1.00 | 50.34 | D000 | C |
| ATOM | 3393 | N | ASN | C | 157 | -30.727 | -36.522 | 38.610 | 1.00 | 41.21 | D000 | N |
| ATOM | 3394 | CA | ASN | C | 157 | -29.820 | -35.593 | 39.292 | 1.00 | 41. 02 | D000 | C |
| ATOM | 3395 | C | ASN | C | 157 | -28.353 | -35.786 | 38.927 | 1.00 | 39.65 | D000 | C |
| ATOM | 3396 | O | ASN | C | 157 | -27.481 | -35.121 | 39.485 | 1.00 | 47.91 | D000 | O |
| ATOM | 3397 | CB | ASN | C | 157 | -29.979 | -35.689 | 40.812 | 1.00 | 45.93 | D000 | C |
| ATOM | 3398 | CG | ASN | C | 157 | -31.360 | -35.289 | 41.276 | 1.00 | 51. 89 | D000 | C |

(continued)

| | | | | | | | | | | | | |
|---|---|---|---|---|---|---|---|---|---|---|---|---|
| ATOM | 3399 | ND2 | ASN | C | 157 | -31.885 | -36.011 | 42.260 | 1.00 | 51.21 | D000 | N |
| ATOM | 3400 | OD1 | ASN | C | 157 | -31.953 | -34.347 | 40.752 | 1.00 | 55.30 | D000 | O |
| ATOM | 3401 | N | TRP | C | 158 | -28.081 | -36.696 | 37.997 | 1.00 | 38.04 | D000 | N |
| ATOM | 3402 | CA | TRP | C | 158 | -26.737 | -36.841 | 37.449 | 1.00 | 35.52 | D000 | C |
| ATOM | 3403 | C | TRP | C | 158 | -26.694 | -36.212 | 36.061 | 1.00 | 41.15 | D000 | C |
| ATOM | 3404 | O | TRP | C | 158 | -27.720 | -36.118 | 35.387 | 1.00 | 32.47 | D000 | O |
| ATOM | 3405 | CB | TRP | C | 158 | -26.323 | -38.313 | 37.394 | 1.00 | 35.29 | D000 | c |
| ATOM | 3406 | CG | TRP | C | 158 | -26.115 | -38.930 | 38.751 | 1.00 | 40.11 | D000 | c |
| ATOM | 3407 | CD1 | TRP | C | 158 | -27.045 | -39.064 | 39.741 | 1.00 | 38.54 | D000 | c |
| ATOM | 3408 | CD2 | TRP | C | 158 | -24.903 | -39.505 | 39.262 | 1.00 | 36.91 | D000 | c |
| ATOM | 3409 | CE2 | TRP | C | 158 | -25.175 | -39.961 | 40.568 | 1.00 | 38.54 | D000 | c |
| ATOM | 3410 | CE3 | TRP | C | 158 | -23.615 | -39.676 | 38.743 | 1.00 | 32.86 | D000 | C |
| ATOM | 3411 | NE1 | TRP | C | 158 | -26.488 | -39.677 | 40.837 | 1.00 | 42.00 | D000 | N |
| ATOM | 3412 | CZ2 | TRP | C | 158 | -24.209 | -40.576 | 41.362 | 1.00 | 34.01 | D000 | C |
| ATOM | 3413 | CZ3 | TRP | C | 158 | -22.657 | -40.286 | 39.533 | 1.00 | 31.10 | D000 | C |
| ATOM | 3414 | CH2 | TRP | C | 158 | -22.958 | -40.728 | 40.827 | 1.00 | 31.72 | D000 | C |
| ATOM | 3415 | N | VAL | c | 159 | -25.511 | -35.775 | 35.640 | 1.00 | 37.12 | D000 | N |
| ATOM | 3416 | CA | VAL | c | 159 | -25.359 | -35.091 | 34.360 | 1.00 | 35.19 | D000 | C |
| ATOM | 3417 | C | VAL | c | 159 | -24.434 | -35.876 | 33.422 | 1.00 | 42.52 | D000 | C |
| ATOM | 3418 | O | VAL | c | 159 | -23.383 | -36.372 | 33.837 | 1.00 | 37.10 | D000 | O |
| ATOM | 3419 | CB | VAL | c | 159 | -24.811 | -33.660 | 34.557 | 1.00 | 36.85 | D000 | C |
| ATOM | 3420 | CG1 | VAL | c | 159 | -24.794 | -32.907 | 33.240 | 1.00 | 35.63 | D000 | C |
| ATOM | 3421 | CG2 | VAL | c | 159 | -25.650 | -32.909 | 35.583 | 1.00 | 36.56 | D000 | C |
| ATOM | 3422 | N | GLU | c | 160 | -24.827 | -35.987 | 32.157 | 1.00 | 36.06 | D000 | N |
| ATOM | 3423 | CA | GLU | c | 160 | -24.070 | -36.784 | 31.197 | 1.00 | 43.73 | D000 | C |
| ATOM | 3424 | C | GLU | c | 160 | -23.047 | -35.975 | 30.405 | 1.00 | 36.77 | D000 | C |
| ATOM | 3425 | O | GLU | c | 160 | -23.328 | -34.874 | 29.939 | 1.00 | 41. 07 | D000 | O |
| ATOM | 3426 | CB | GLU | c | 160 | -25.018 | -37.481 | 30.221 | 1.00 | 43.94 | D000 | C |
| ATOM | 3427 | CG | GLU | c | 160 | -24.301 | -38.291 | 29.158 | 1.00 | 50.04 | D000 | C |
| ATOM | 3428 | CD | GLU | c | 160 | -25.222 | -38.743 | 28.045 | 1.00 | 67.78 | D000 | C |
| ATOM | 3429 | OE1 | GLU | c | 160 | -26.430 | -38.930 | 28.308 | 1.00 | 69.69 | D000 | O |
| ATOM | 3430 | OE2 | GLU | c | 160 | -24.735 | -38.904 | 26.905 | 1.00 | 67.23 | D000 | O |
| ATOM | 3431 | N | HIS | c | 161 | -21.856 | -36.541 | 30.261 | 1.00 | 37.45 | D000 | N |
| ATOM | 3432 | CA | HIS | c | 161 | -20.829 | -35.993 | 29.388 | 1.00 | 38.41 | D000 | C |

(continued)

| | | | | | | | | | | | |
|---|---|---|---|---|---|---|---|---|---|---|---|
| ATOM | 3433 | C | HIS | c | 161 | -19.987 | -37.135 | 28.842 | 1.00 | 40.31 | D000 | C |
| ATOM | 3434 | O | HIS | c | 161 | -19.301 | -37.816 | 29.604 | 1.00 | 36.78 | D000 | O |
| ATOM | 3435 | CB | HIS | c | 161 | -19.951 | -34.987 | 30.134 | 1.00 | 35.44 | D000 | C |
| ATOM | 3436 | CG | HIS | c | 161 | -18.785 | -34.487 | 29.334 | 1.00 | 39.39 | D000 | C |
| ATOM | 3437 | CD2 | HIS | c | 161 | -17.492 | -34.888 | 29.300 | 1.00 | 37.49 | D000 | C |
| ATOM | 3438 | ND1 | HIS | c | 161 | -18.888 | -33.445 | 28.439 | 1.00 | 37.81 | D000 | N |
| ATOM | 3439 | CE1 | HIS | c | 161 | -17.707 | -33.223 | 27.887 | 1.00 | 36.44 | D000 | C |
| ATOM | 3440 | NE2 | HIS | c | 161 | -16.844 | -34.085 | 28.390 | 1.00 | 39.12 | D000 | N |
| ATOM | 3441 | N | GLU | c | 162 | -20.066 | -37.349 | 27.529 | 1.00 | 45.55 | D000 | N |
| ATOM | 3442 | CA | GLU | c | 162 | -19.271 | -38.365 | 26.834 | 1.00 | 40.64 | D000 | C |
| ATOM | 3443 | C | GLU | c | 162 | -19.341 | -39.749 | 27.484 | 1.00 | 41.12 | D000 | C |
| ATOM | 3444 | O | GLU | c | 162 | -18.318 | -40.305 | 27.887 | 1.00 | 42.56 | D000 | O |
| ATOM | 3445 | CB | GLU | c | 162 | -17.806 | -37.919 | 26.744 | 1.00 | 41.10 | D000 | C |
| ATOM | 3446 | CG | GLU | c | 162 | -17.596 | -36.576 | 26.052 | 1.00 | 49.56 | D000 | C |
| ATOM | 3447 | CD | GLU | c | 162 | -18.120 | -36.554 | 24.622 | 1.00 | 68.97 | D000 | C |
| ATOM | 3448 | OE1 | GLU | c | 162 | -18.103 | -37.612 | 23.955 | 1.00 | 61.44 | D000 | O |
| ATOM | 3449 | OE2 | GLU | c | 162 | -18.551 | -35.472 | 24.163 | 1.00 | 82.19 | D000 | O |
| ATOM | 3450 | N | ARG | c | 163 | -20.550 | -40.289 | 27.586 | 1.00 | 39.55 | D000 | N |
| ATOM | 3451 | CA | ARG | c | 163 | -20.790 | -41.614 | 28.175 | 1.00 | 50.72 | D000 | C |
| ATOM | 3452 | C | ARG | c | 163 | -20.305 | -41.757 | 29.628 | 1.00 | 46.47 | D000 | C |
| ATOM | 3453 | O | ARG | c | 163 | -20.132 | -42.870 | 30.127 | 1.00 | 44.94 | D000 | O |
| ATOM | 3454 | CB | ARG | c | 163 | -20.156 | -42.710 | 27.306 | 1.00 | 49.40 | D000 | C |
| ATOM | 3455 | CG | ARG | c | 163 | -20.768 | -42.827 | 25.907 | 1.00 | 51.79 | D000 | C |
| ATOM | 3456 | CD | ARG | c | 163 | -20.685 | -44.252 | 25.348 | 1.00 | 44.72 | D000 | C |
| ATOM | 3457 | NE | ARG | c | 163 | -21.723 | -45.140 | 25.879 | 1.00 | 51.93 | D000 | N |
| ATOM | 3458 | CZ | ARG | c | 163 | -21.483 | -46.234 | 26.606 | 1.00 | 59.01 | D000 | C |
| ATOM | 3459 | NH1 | ARG | c | 163 | -22.492 | -46.980 | 27.044 | 1.00 | 45.88 | D000 | N |
| ATOM | 3460 | NH2 | ARG | c | 163 | -20.236 | -46.586 | 26.897 | 1.00 | 49.85 | D000 | N |
| ATOM | 3461 | N | SER | c | 164 | -20.099 | -40.633 | 30.306 | 1.00 | 43.03 | D000 | N |
| ATOM | 3462 | CA | SER | c | 164 | -19.865 | -40.646 | 31.746 | 1.00 | 37.64 | D000 | C |
| ATOM | 3463 | C | SER | c | 164 | -20.969 | -39.876 | 32.459 | 1.00 | 37.23 | D000 | C |
| ATOM | 3464 | O | SER | c | 164 | -21.536 | -38.933 | 31.903 | 1.00 | 37.58 | D000 | O |
| ATOM | 3465 | CB | SER | c | 164 | -18.498 | -40.052 | 32.092 | 1.00 | 37.20 | D000 | C |
| ATOM | 3466 | OG | SER | c | 164 | -17.509 | -41.061 | 32.172 | 1.00 | 40.60 | D000 | O |

(continued)

| | | | | | | | | | | | |
|---|---|---|---|---|---|---|---|---|---|---|---|
| ATOM | 3467 | N | CYS | c | 165 | -21.272 | -40.283 | 33.687 | 1.00 | 36.28 | D000 | N |
| ATOM | 3468 | CA | CYS | c | 165 | -22.296 | -39.624 | 34.493 | 1.00 | 35.32 | D000 | C |
| ATOM | 3469 | C | CYS | c | 165 | -21.666 | -38.921 | 35.688 | 1.00 | 39.53 | D000 | C |
| ATOM | 3470 | O | CYS | C | 165 | -20.843 | -39.504 | 36.398 | 1.00 | 33.29 | D000 | O |
| ATOM | 3471 | CB | CYS | c | 165 | -23.344 | -40.632 | 34.974 | 1.00 | 42.45 | D000 | C |
| ATOM | 3472 | SG | CYS | c | 165 | -24.315 | -41.382 | 33.657 | 1.00 | 55.85 | D000 | S |
| ATOM | 3473 | N | TYR | c | 166 | -22.065 | -37.674 | 35.915 | 1.00 | 29.38 | D000 | N |
| ATOM | 3474 | CA | TYR | c | 166 | -21.475 | -36.870 | 36.976 | 1.00 | 33.48 | D000 | C |
| ATOM | 3475 | C | TYR | c | 166 | -22.532 | -36.391 | 37.963 | 1.00 | 33.01 | D000 | C |
| ATOM | 3476 | O | TYR | C | 166 | -23.665 | -36.110 | 37.589 | 1.00 | 33.24 | D000 | O |
| ATOM | 3477 | CB | TYR | C | 166 | -20.730 | -35.667 | 36.388 | 1.00 | 32.05 | D000 | c |
| ATOM | 3478 | CG | TYR | C | 166 | -19.658 | -36.030 | 35.383 | 1.00 | 31. 06 | D000 | c |
| ATOM | 3479 | CD1 | TYR | C | 166 | -19.990 | -36.385 | 34.080 | 1.00 | 30.70 | D000 | c |
| ATOM | 3480 | CD2 | TYR | C | 166 | -18.313 | -36.003 | 35.732 | 1.00 | 30.70 | D000 | c |
| ATOM | 3481 | CE1 | TYR | C | 166 | -19.017 | -36.713 | 33.153 | 1.00 | 33.69 | D000 | c |
| ATOM | 3482 | CE2 | TYR | C | 166 | -17.329 | -36.329 | 34.807 | 1.00 | 32.22 | D000 | c |
| ATOM | 3483 | CZ | TYR | C | 166 | -17.690 | -36.682 | 33.519 | 1.00 | 32.45 | D000 | c |
| ATOM | 3484 | OH | TYR | C | 166 | -16.723 | -37.007 | 32.593 | 1.00 | 35.47 | D000 | O |
| ATOM | 3485 | N | TRP | C | 167 | -22.156 | -36.309 | 39.231 | 1.00 | 33.71 | D000 | N |
| ATOM | 3486 | CA | TRP | C | 167 | -23.032 | -35.743 | 40.246 | 1.00 | 35.55 | D000 | C |
| ATOM | 3487 | C | TRP | C | 167 | -22.262 | -34.685 | 41.017 | 1.00 | 32.66 | D000 | C |
| ATOM | 3488 | O | TRP | C | 167 | -21.140 | -34.931 | 41.470 | 1.00 | 31.46 | D000 | O |
| ATOM | 3489 | CB | TRP | C | 167 | -23.559 | -36.827 | 41.186 | 1.00 | 35.80 | D000 | C |
| ATOM | 3490 | CG | TRP | C | 167 | -24.458 | -36.290 | 42.265 | 1.00 | 43.58 | D000 | C |
| ATOM | 3491 | CD1 | TRP | C | 167 | -25.805 | -36.079 | 42.183 | 1.00 | 38.70 | D000 | C |
| ATOM | 3492 | CD2 | TRP | C | 167 | -24.070 | -35.896 | 43.587 | 1.00 | 35.59 | D000 | C |
| ATOM | 3493 | CE2 | TRP | C | 167 | -25.235 | -35.456 | 44.251 | 1.00 | 41.09 | D000 | C |
| ATOM | 3494 | CE3 | TRP | C | 167 | -22.852 | -35.869 | 44.271 | 1.00 | 35.37 | D000 | C |
| ATOM | 3495 | NE1 | TRP | C | 167 | -26.280 | -35.577 | 43.373 | 1.00 | 39.42 | D000 | N |
| ATOM | 3496 | CZ2 | TRP | C | 167 | -25.215 | -34.997 | 45.567 | 1.00 | 38.44 | D000 | C |
| ATOM | 3497 | CZ3 | TRP | C | 167 | -22.835 | -35.415 | 45.579 | 1.00 | 37.33 | D000 | C |
| ATOM | 3498 | CH2 | TRP | C | 167 | -24.008 | -34.985 | 46.213 | 1.00 | 36.75 | D000 | C |
| ATOM | 3499 | N | PHE | C | 168 | -22.858 | -33.505 | 41.155 | 1.00 | 33.58 | D000 | N |
| ATOM | 3500 | CA | PHE | C | 168 | -22.172 | -32.374 | 41.778 | 1.00 | 33.25 | D000 | C |

(continued)

| | | | | | | | | | | | |
|---|---|---|---|---|---|---|---|---|---|---|---|
| ATOM | 3501 | C | PHE | C | 168 | -22.773 | -32.041 | 43.134 | 1.00 | 31.97 | D000 | C |
| ATOM | 3502 | O | PHE | C | 168 | -23.953 | -31.721 | 43.227 | 1.00 | 34.21 | D000 | O |
| ATOM | 3503 | CB | PHE | C | 168 | -22.229 | -31.148 | 40.861 | 1.00 | 28.44 | D000 | C |
| ATOM | 3504 | CG | PHE | C | 168 | -21.573 | -31.363 | 39.526 | 1.00 | 30.16 | D000 | C |
| ATOM | 3505 | CD1 | PHE | C | 168 | -22.287 | -31.896 | 38.463 | 1.00 | 31.96 | D000 | C |
| ATOM | 3506 | CD2 | PHE | C | 168 | -20.239 | -31. 038 | 39.337 | 1.00 | 29.17 | D000 | C |
| ATOM | 3507 | CE1 | PHE | C | 168 | -21.682 | -32.099 | 37.229 | 1.00 | 29.38 | D000 | C |
| ATOM | 3508 | CE2 | PHE | C | 168 | -19.625 | -31.242 | 38.114 | 1.00 | 28.14 | D000 | C |
| ATOM | 3509 | CZ | PHE | C | 168 | -20.348 | -31.776 | 37.057 | 1.00 | 28.28 | D000 | C |
| ATOM | 3510 | N | SER | C | 169 | -21.961 | -32.110 | 44.186 | 1.00 | 30.11 | D000 | N |
| ATOM | 3511 | CA | SER | C | 169 | -22.462 | -31.851 | 45.528 | 1.00 | 31.18 | D000 | C |
| ATOM | 3512 | C | SER | C | 169 | -22.693 | -30.360 | 45.740 | 1.00 | 37.87 | D000 | C |
| ATOM | 3513 | O | SER | C | 169 | -22.121 | -29.522 | 45.038 | 1.00 | 34.20 | D000 | O |
| ATOM | 3514 | CB | SER | C | 169 | -21.494 | -32.389 | 46.583 | 1.00 | 30.35 | D000 | C |
| ATOM | 3515 | OG | SER | C | 169 | -20.471 | -31.453 | 46.859 | 1.00 | 30.05 | D000 | O |
| ATOM | 3516 | N | ARG | C | 170 | -23.536 | -30.027 | 46.708 | 1.00 | 37.76 | D000 | N |
| ATOM | 3517 | CA | ARG | C | 170 | -23.767 | -28.628 | 47.046 | 1.00 | 42.71 | D000 | C |
| ATOM | 3518 | C | ARG | C | 170 | -23.330 | -28.383 | 48.488 | 1.00 | 43.83 | D000 | C |
| ATOM | 3519 | O | ARG | C | 170 | -23.437 | -27.274 | 49.010 | 1.00 | 47.78 | D000 | O |
| ATOM | 3520 | CB | ARG | C | 170 | -25.237 | -28.254 | 46.829 | 1.00 | 44.70 | D000 | C |
| ATOM | 3521 | CG | ARG | C | 170 | -25.717 | -28.513 | 45.395 | 1.00 | 57.03 | D000 | C |
| ATOM | 3522 | CD | ARG | C | 170 | -27.063 | -27.862 | 45.082 | 1.00 | 63.29 | D000 | C |
| ATOM | 3523 | NE | ARG | C | 170 | -28.144 | -28.365 | 45.925 | 1.00 | 84.25 | D000 | N |
| ATOM | 3524 | CZ | ARG | C | 170 | -28.822 | -29.485 | 45.688 | 1.00 | 84.39 | D000 | C |
| ATOM | 3525 | NH1 | ARG | C | 170 | -29.792 | -29.862 | 46.511 | 1.00 | 72.14 | D000 | N |
| ATOM | 3526 | NH2 | ARG | C | 170 | -28.528 | -30.230 | 44.631 | 1.00 | 91.59 | D000 | N |
| ATOM | 3527 | N | SER | C | 171 | -22.817 | -29.439 | 49.112 | 1.00 | 39.23 | D000 | N |
| ATOM | 3528 | CA | SER | C | 171 | -22.298 | -29.372 | 50.470 | 1.00 | 41.01 | D000 | C |
| ATOM | 3529 | C | SER | C | 171 | -20.827 | -29.773 | 50.489 | 1.00 | 37.33 | D000 | C |
| ATOM | 3530 | O | SER | C | 171 | -20.305 | -30.281 | 49.499 | 1.00 | 31.87 | D000 | O |
| ATOM | 3531 | CB | SER | C | 171 | -23.106 | -30.277 | 51.401 | 1.00 | 36.62 | D000 | C |
| ATOM | 3532 | OG | SER | C | 171 | -23.000 | -31.633 | 51.004 | 1.00 | 43.48 | D000 | O |
| ATOM | 3533 | N | GLY | C | 172 | -20.166 | -29.549 | 51.620 | 1.00 | 31.79 | D000 | N |
| ATOM | 3534 | CA | GLY | C | 172 | -18.749 | -29.840 | 51.743 | 1.00 | 31.58 | D000 | C |

(continued)

| | | | | | | | | | | | | |
|---|---|---|---|---|---|---|---|---|---|---|---|---|
| ATOM | 3535 | C | GLY | C | 172 | -18.428 | -31.069 | 52.573 | 1.00 | 37.61 | D000 | C |
| ATOM | 3536 | O | GLY | C | 172 | -19.161 | -31.427 | 53.495 | 1.00 | 34.62 | D000 | O |
| ATOM | 3537 | N | LYS | C | 173 | -17.317 | -31.714 | 52.231 | 1.00 | 32.21 | D000 | N |
| ATOM | 3538 | CA | LYS | C | 173 | -16.818 | -32.878 | 52.954 | 1.00 | 30.84 | D000 | C |
| ATOM | 3539 | C | LYS | C | 173 | -15.300 | -32.903 | 52.885 | 1.00 | 36.15 | D000 | C |
| ATOM | 3540 | O | LYS | C | 173 | -14.715 | -32.463 | 51.894 | 1.00 | 35.63 | D000 | O |
| ATOM | 3541 | CB | LYS | C | 173 | -17.365 | -34.184 | 52.367 | 1.00 | 35.20 | D000 | C |
| ATOM | 3542 | CG | LYS | C | 173 | -18.746 | -34.590 | 52.824 | 1.00 | 33.26 | D000 | C |
| ATOM | 3543 | CD | LYS | C | 173 | -19.092 | -35.966 | 52.277 | 1.00 | 36.71 | D000 | C |
| ATOM | 3544 | CE | LYS | C | 173 | -20.518 | -36.364 | 52.617 | 1.00 | 39.30 | D000 | C |
| ATOM | 3545 | NZ | LYS | C | 173 | -20.706 | -36.532 | 54.082 | 1.00 | 42.46 | D000 | N |
| ATOM | 3546 | N | ALA | C | 174 | -14.665 | -33.423 | 53.930 | 1.00 | 34.50 | D000 | N |
| ATOM | 3547 | CA | ALA | C | 174 | -13.244 | -33.733 | 53.871 | 1.00 | 32.78 | D000 | c |
| ATOM | 3548 | C | ALA | C | 174 | -13.011 | -34.731 | 52.737 | 1.00 | 30.79 | D000 | c |
| ATOM | 3549 | O | ALA | C | 174 | -13.918 | -35.486 | 52.375 | 1.00 | 27.51 | D000 | O |
| ATOM | 3550 | CB | ALA | C | 174 | -12.756 | -34.296 | 55.201 | 1.00 | 32.37 | D000 | C |
| ATOM | 3551 | N | TRP | C | 175 | -11.802 | -34.736 | 52.182 | 1.00 | 28.74 | D000 | N |
| ATOM | 3552 | CA | TRP | C | 175 | -11.510 | -35.556 | 51.011 | 1.00 | 34.63 | D000 | C |
| ATOM | 3553 | C | TRP | C | 175 | -11.853 | -37.026 | 51.230 | 1.00 | 31.83 | D000 | C |
| ATOM | 3554 | O | TRP | C | 175 | -12.520 | -37.640 | 50.398 | 1.00 | 31.59 | D000 | O |
| ATOM | 3555 | CB | TRP | C | 175 | -10.041 | -35.430 | 50.606 | 1.00 | 31.97 | D000 | C |
| ATOM | 3556 | CG | TRP | C | 175 | -9.771 | -36.017 | 49.251 | 1.00 | 27.08 | D000 | C |
| ATOM | 3557 | CD1 | TRP | C | 175 | -9.812 | -35.364 | 48.051 | 1.00 | 33.57 | D000 | C |
| ATOM | 3558 | CD2 | TRP | C | 175 | -9.442 | -37.378 | 48.955 | 1.00 | 30.56 | D000 | C |
| ATOM | 3559 | CE2 | TRP | C | 175 | -9.285 | -37.476 | 47.558 | 1.00 | 29.73 | D000 | C |
| ATOM | 3560 | CE3 | TRP | C | 175 | -9.257 | -38.524 | 49.737 | 1.00 | 31.43 | D000 | C |
| ATOM | 3561 | NE1 | TRP | C | 175 | -9.518 | -36.234 | 47.029 | 1.00 | 30.16 | D000 | N |
| ATOM | 3562 | CZ2 | TRP | C | 175 | -8.958 | -38.674 | 46.926 | 1.00 | 31.89 | D000 | C |
| ATOM | 3563 | CZ3 | TRP | C | 175 | -8.934 | -39.711 | 49.107 | 1.00 | 33.11 | D000 | C |
| ATOM | 3564 | CH2 | TRP | C | 175 | -8.787 | -39.777 | 47.715 | 1.00 | 31.57 | D000 | C |
| ATOM | 3565 | N | ALA | C | 176 | -11.402 | -37.585 | 52.349 | 1.00 | 36.55 | D000 | N |
| ATOM | 3566 | CA | ALA | C | 176 | -11.654 | -38.992 | 52.642 | 1.00 | 34.85 | D000 | C |
| ATOM | 3567 | C | ALA | C | 176 | -13.153 | -39.287 | 52.720 | 1.00 | 31.81 | D000 | C |
| ATOM | 3568 | O | ALA | C | 176 | -13.611 | -40.335 | 52.264 | 1.00 | 32.34 | D000 | O |

(continued)

| | | | | | | | | | | | | |
|---|---|---|---|---|---|---|---|---|---|---|---|---|
| ATOM | 3569 | CB | ALA | C | 176 | -10.961 | -39.397 | 53.940 | 1.00 | 39.21 | D000 | C |
| ATOM | 3570 | N | ASP | C | 177 | -13.921 | -38.362 | 53.286 | 1.00 | 29.24 | D000 | N |
| ATOM | 3571 | CA | ASP | C | 177 | -15.363 | -38.562 | 53.387 | 1.00 | 30.70 | D000 | C |
| ATOM | 3572 | C | ASP | C | 177 | -16.048 | -38.465 | 52.021 | 1.00 | 34.41 | D000 | C |
| ATOM | 3573 | O | ASP | C | 177 | -17.005 | -39.186 | 51.749 | 1.00 | 30.91 | D000 | O |
| ATOM | 3574 | CB | ASP | C | 177 | -15.976 | -37.560 | 54.363 | 1.00 | 36.68 | D000 | C |
| ATOM | 3575 | CG | ASP | CC | 177 | -15.641 | -37.883 | 55.810 | 1.00 | 47.91 | D000 | C |
| ATOM | 3576 | OD1 | ASP | C | 177 | -15.459 | -39.080 | 56.124 | 1.00 | 48.52 | D000 | O |
| ATOM | 3577 | OD2 | ASP | C | 177 | -15.561 | -36.943 | 56.631 | 1.00 | 44.32 | D000 | O |
| ATOM | 3578 | N | ALA | C | 178 | -15.558 | -37.575 | 51.164 | 1.00 | 33.94 | D000 | N |
| ATOM | 3579 | CA | ALA | C | 178 | -16.104 | -37.450 | 49.814 | 1.00 | 37.48 | D000 | C |
| ATOM | 3580 | C | ALA | C | 178 | -15.776 | -38.696 | 48.994 | 1.00 | 30.71 | D000 | C |
| ATOM | 3581 | O | ALA | C | 178 | -16.620 | -39.219 | 48.265 | 1.00 | 33.32 | D000 | O |
| ATOM | 3582 | CB | ALA | C | 178 | -15.568 | -36.197 | 49.130 | 1.00 | 29.20 | D000 | C |
| ATOM | 3583 | N | ASP | C | 179 | -14.540 | -39.161 | 49.134 | 1.00 | 30.70 | D000 | N |
| ATOM | 3584 | CA | ASP | C | 179 | -14.078 | -40.389 | 48.501 | 1.00 | 27.81 | D000 | C |
| ATOM | 3585 | C | ASP | C | 179 | -14.986 | -41.564 | 48.868 | 1.00 | 36.66 | D000 | C |
| ATOM | 3586 | O | ASP | C | 179 | -15.411 | -42.333 | 48.005 | 1.00 | 36.40 | D000 | O |
| ATOM | 3587 | CB | ASP | C | 179 | -12.630 | -40.671 | 48.918 | 1.00 | 31.15 | D000 | C |
| ATOM | 3588 | CG | ASP | C | 179 | -12.068 | -41.935 | 48.289 | 1.00 | 39.74 | D000 | C |
| ATOM | 3589 | OD1 | ASP | C | 179 | -12.435 | -42.255 | 47.139 | 1.00 | 49.64 | D000 | O |
| ATOM | 3590 | OD2 | ASP | C | 179 | -11.247 | -42.607 | 48.947 | 1.00 | 47.11 | D000 | O |
| ATOM | 3591 | N | ASN | C | 180 | -15.299 | -41.680 | 50.153 | 1.00 | 32.38 | D000 | N |
| ATOM | 3592 | CA | ASN | C | 180 | -16.155 | -42.751 | 50.642 | 1.00 | 33.10 | D000 | C |
| ATOM | 3593 | C | ASN | C | 180 | -17.598 | -42.602 | 50.155 | 1.00 | 32.93 | D000 | C |
| ATOM | 3594 | O | ASN | C | 180 | -18.224 | -43.582 | 49.755 | 1.00 | 36.93 | D000 | O |
| ATOM | 3595 | CB | ASN | C | 180 | -16.108 | -42.799 | 52.171 | 1.00 | 39.79 | D000 | C |
| ATOM | 3596 | CG | ASN | C | 180 | -16.936 | -43.931 | 52.745 | 1.00 | 50.05 | D000 | C |
| ATOM | 3597 | ND2 | ASN | C | 180 | -16.557 | -45.165 | 52.427 | 1.00 | 44.28 | D000 | N |
| ATOM | 3598 | OD1 | ASN | C | 180 | -17.911 | -43.699 | 53.461 | 1.00 | 53.44 | D000 | O |
| ATOM | 3599 | N | TYR | C | 181 | -18.120 | -41.378 | 50.182 | 1.00 | 33.15 | D000 | N |
| ATOM | 3600 | CA | TYR | C | 181 | -19.460 | -41.105 | 49.659 | 1.00 | 36.25 | D000 | C |
| ATOM | 3601 | C | TYR | C | 181 | -19.609 | -41.606 | 48.218 | 1.00 | 38.76 | D000 | C |
| ATOM | 3602 | O | TYR | C | 181 | -20.608 | -42.238 | 47.875 | 1.00 | 36.89 | D000 | O |

(continued)

| | | | | | | | | | | | | |
|---|---|---|---|---|---|---|---|---|---|---|---|---|
| ATOM | 3603 | CB | TYR | C | 181 | -19.775 | -39.605 | 49.728 | 1.00 | 29.33 | D000 | C |
| ATOM | 3604 | CG | TYR | C | 181 | -21.139 | -39.226 | 49.184 | 1.00 | 38.78 | D000 | C |
| ATOM | 3605 | CD1 | TYR | C | 181 | -21.333 | -39.016 | 47.820 | 1.00 | 39.24 | D000 | C |
| ATOM | 3606 | CD2 | TYR | C | 181 | -22.232 | -39.065 | 50.032 | 1.00 | 37.86 | D000 | C |
| ATOM | 3607 | CE1 | TYR | C | 181 | -22.574 | -38.670 | 47.316 | 1.00 | 41.79 | D000 | C |
| ATOM | 3608 | CE2 | TYR | C | 181 | -23.484 | -38.716 | 49.534 | 1.00 | 30.18 | D000 | C |
| ATOM | 3609 | CZ | TYR | C | 181 | -23.643 | -38.520 | 48.173 | 1.00 | 43.34 | D000 | C |
| ATOM | 3610 | OH | TYR | C | 181 | -24.868 | -38.175 | 47.654 | 1.00 | 43.72 | D000 | O |
| ATOM | 3611 | N | CYS | C | 182 | -18.617 | -41.321 | 47.379 | 1.00 | 32.27 | D000 | N |
| ATOM | 3612 | CA | CYS | C | 182 | -18.688 | -41.720 | 45.977 | 1.00 | 33.21 | D000 | C |
| ATOM | 3613 | C | CYS | C | 182 | -18.666 | -43.240 | 45.819 | 1.00 | 36.35 | D000 | C |
| ATOM | 3614 | O | CYS | C | 182 | -19.433 | -43.793 | 45.032 | 1.00 | 28.20 | D000 | O |
| ATOM | 3615 | CB | CYS | C | 182 | -17.550 | -41.079 | 45.177 | 1.00 | 33.72 | D000 | C |
| ATOM | 3616 | SG | CYS | C | 182 | -17.775 | -39.294 | 44.910 | 1.00 | 35.82 | D000 | S |
| ATOM | 3617 | N | ARG | C | 183 | -17.798 | -43.911 | 46.573 | 1.00 | 38.71 | D000 | N |
| ATOM | 3618 | CA | ARG | C | 183 | -17.731 | -45.373 | 46.546 | 1.00 | 38.40 | D000 | c |
| ATOM | 3619 | C | ARG | C | 183 | -19.071 | -45.997 | 46.942 | 1.00 | 41.03 | D000 | c |
| ATOM | 3620 | O | ARG | C | 183 | -19.466 | -47.030 | 46.409 | 1.00 | 42.99 | D000 | O |
| ATOM | 3621 | CB | ARG | C | 183 | -16.621 | -45.880 | 47.473 | 1.00 | 39.02 | D000 | c |
| ATOM | 3622 | CG | ARG | C | 183 | -15.217 | -45.538 | 47.002 | 1.00 | 46.96 | D000 | c |
| ATOM | 3623 | CD | ARG | C | 183 | -14.169 | -45.992 | 48.007 | 1.00 | 60.57 | D000 | C |
| ATOM | 3624 | NE | ARG | C | 183 | -12.816 | -45.655 | 47.570 | 1.00 | 80.77 | D000 | N |
| ATOM | 3625 | CZ | ARG | C | 183 | -11.722 | -45.845 | 48.301 | 1.00 | 85.00 | D000 | C |
| ATOM | 3626 | NH1 | ARG | C | 183 | -11.814 | -46.372 | 49.516 | 1.00 | 72.80 | D000 | N |
| ATOM | 3627 | NH2 | ARG | C | 183 | -10.532 | -45.505 | 47.818 | 1.00 | 88.26 | D000 | N |
| ATOM | 3628 | N | LEU | C | 184 | -19.769 | -45.348 | 47.869 | 1.00 | 38.52 | D000 | N |
| ATOM | 3629 | CA | LEU | C | 184 | -21.063 | -45.822 | 48.348 | 1.00 | 45.53 | D000 | C |
| ATOM | 3630 | C | LEU | C | 184 | -22.165 | -45.627 | 47.305 | 1.00 | 44.46 | D000 | C |
| ATOM | 3631 | O | LEU | C | 184 | -23.192 | -46.300 | 47.344 | 1.00 | 49.42 | D000 | O |
| ATOM | 3632 | CB | LEU | C | 184 | -21.436 | -45.110 | 49.656 | 1.00 | 38.94 | D000 | C |
| ATOM | 3633 | CG | LEU | C | 184 | -21.111 | -45.837 | 50.967 | 1.00 | 47.53 | D000 | C |
| ATOM | 3634 | CD1 | LEU | C | 184 | -19.766 | -46.545 | 50.898 | 1.00 | 51.14 | D000 | C |
| ATOM | 3635 | CD2 | LEU | C | 184 | -21.138 | -44 . 869 | 52.138 | 1.00 | 42.20 | D000 | C |
| ATOM | 3636 | N | GLU | C | 185 | -21.953 | -44.701 | 46.376 | 1.00 | 42.20 | D000 | N |

(continued)

| | | | | | | | | | | | |
|---|---|---|---|---|---|---|---|---|---|---|---|
| ATOM | 3637 | CA | GLU | C | 185 | -22.903 | -44.483 | 45.288 | 1.00 | 39.20 | D000 | C |
| ATOM | 3638 | C | GLU | C | 185 | -22.504 | -45.293 | 44.056 | 1.00 | 40.02 | D000 | C |
| ATOM | 3639 | O | GLU | C | 185 | -22.992 | -45.040 | 42.954 | 1.00 | 41.00 | D000 | O |
| ATOM | 3640 | CB | GLU | C | 185 | -22.991 | -42.997 | 44.936 | 1.00 | 43.28 | D000 | C |
| ATOM | 3641 | CG | GLU | C | 185 | -23.645 | -42.139 | 46.005 | 1.00 | 50.04 | D000 | C |
| ATOM | 3642 | CD | GLU | C | 185 | -25.132 | -42.410 | 46.144 | 1.00 | 63.83 | D000 | C |
| ATOM | 3643 | OE1 | GLU | C | 185 | -25.853 | -42.327 | 45.125 | 1.00 | 64.40 | D000 | O |
| ATOM | 3644 | OE2 | GLU | C | 185 | -25.578 | -42.711 | 47.274 | 1.00 | 66.65 | D000 | O |
| ATOM | 3645 | N | ASP | C | 186 | -21.618 | -46.266 | 44.266 | 1.00 | 39.71 | D000 | N |
| ATOM | 3646 | CA | ASP | C | 186 | -21.073 | -47.104 | 43.197 | 1.00 | 46.14 | D000 | C |
| ATOM | 3647 | C | ASP | C | 186 | -20.374 | -46.235 | 42.149 | 1.00 | 46.36 | D000 | C |
| ATOM | 3648 | O | ASP | C | 186 | -20.472 | -46.469 | 40.940 | 1.00 | 37.33 | D000 | O |
| ATOM | 3649 | CB | ASP | C | 186 | -22.172 | -47.957 | 42.555 | 1.00 | 48.03 | D000 | C |
| ATOM | 3650 | CG | ASP | C | 186 | -21.616 | -49.072 | 41.689 | 1.00 | 57.58 | D000 | C |
| ATOM | 3651 | OD1 | ASP | C | 186 | -20.621 | -49.705 | 42.104 | 1.00 | 69.48 | D000 | O |
| ATOM | 3652 | OD2 | ASP | C | 186 | -22.167 | -49.306 | 40.592 | 1.00 | 63.55 | D000 | O |
| ATOM | 3653 | N | ALA | C | 187 | -19.667 | -45.222 | 42.634 | 1.00 | 37.49 | D000 | N |
| ATOM | 3654 | CA | ALA | C | 187 | -18.974 | -44.291 | 41.763 | 1.00 | 36.04 | D000 | C |
| ATOM | 3655 | C | ALA | C | 187 | -17.592 | -43.992 | 42.322 | 1.00 | 37.13 | D000 | C |
| ATOM | 3656 | O | ALA | C | 187 | -17.086 | -44.715 | 43.183 | 1.00 | 35.71 | D000 | O |
| ATOM | 3657 | CB | ALA | C | 187 | -19.784 | -43.005 | 41.603 | 1.00 | 29.96 | D000 | C |
| ATOM | 3658 | N | HIS | C | 188 | -16.981 | -42.924 | 41.826 | 1.00 | 31.72 | D000 | N |
| ATOM | 3659 | CA | HIS | C | 188 | -15.693 | -42.486 | 42.332 | 1.00 | 29.40 | D000 | C |
| ATOM | 3660 | C | HIS | C | 188 | -15.569 | -40.982 | 42.157 | 1.00 | 28.01 | D000 | C |
| ATOM | 3661 | O | HIS | C | 188 | -16.263 | -40.391 | 41.329 | 1.00 | 26.39 | D000 | O |
| ATOM | 3662 | CB | HIS | C | 188 | -14.556 | -43.211 | 41.618 | 1.00 | 23.87 | D000 | C |
| ATOM | 3663 | CG | HIS | C | 188 | -14.541 | -43.002 | 40.137 | 1.00 | 35.80 | D000 | C |
| ATOM | 3664 | CD2 | HIS | C | 188 | -14.996 | -43.776 | 39.123 | 1.00 | 32.29 | D000 | C |
| ATOM | 3665 | ND1 | HIS | C | 188 | -14.009 | -41.874 | 39.548 | 1.00 | 32.82 | D000 | N |
| ATOM | 3666 | CE1 | HIS | C | 188 | -14.134 | -41.964 | 38.236 | 1.00 | 33.00 | D000 | C |
| ATOM | 3667 | NE2 | HIS | C | 188 | -14.729 | -43.109 | 37.951 | 1.00 | 33.93 | D000 | N |
| ATOM | 3668 | N | LEU | C | 189 | -14.708 | -40.364 | 42.958 | 1.00 | 26.16 | D000 | N |
| ATOM | 3669 | CA | LEU | C | 189 | -14.400 | -38.950 | 42.784 | 1.00 | 28.90 | D000 | C |
| ATOM | 3670 | C | LEU | C | 189 | -13.909 | -38.716 | 41.362 | 1.00 | 29.00 | D000 | C |

(continued)

| | | | | | | | | | | | |
|---|---|---|---|---|---|---|---|---|---|---|---|
| ATOM | 3671 | O | LEU | C | 189 | -13.176 | -39.541 | 40.805 | 1.00 | 27.16 | D000 | O |
| ATOM | 3672 | CB | LEU | C | 189 | -13.356 | -38.488 | 43.796 | 1.00 | 26.67 | D000 | C |
| ATOM | 3673 | CG | LEU | C | 189 | -13.882 | -38.242 | 45.208 | 1.00 | 33.12 | D000 | C |
| ATOM | 3674 | CD1 | LEU | C | 189 | -12.735 | -37.973 | 46.169 | 1.00 | 33.71 | D000 | C |
| ATOM | 3675 | CD2 | LEU | C | 189 | -14 . 857 | -37.076 | 45.186 | 1.00 | 27.90 | D000 | C |
| ATOM | 3676 | N | VAL | C | 190 | -14.320 | -37.592 | 40.786 | 1.00 | 27.74 | D000 | N |
| ATOM | 3677 | CA | VAL | C | 190 | -14.117 | -37.333 | 39.365 | 1.00 | 27.15 | D000 | C |
| ATOM | 3678 | C | VAL | C | 190 | -12.644 | -37.428 | 38.968 | 1.00 | 29.08 | D000 | C |
| ATOM | 3679 | O | VAL | C | 190 | -11.743 | -36.982 | 39.690 | 1.00 | 26.29 | D000 | O |
| ATOM | 3680 | CB | VAL | C | 190 | -14.699 | -35.946 | 38.957 | 1.00 | 29.42 | D000 | C |
| ATOM | 3681 | CG1 | VAL | C | 190 | -13.956 | -34.796 | 39.654 | 1.00 | 25.30 | D000 | C |
| ATOM | 3682 | CG2 | VAL | C | 190 | -14.705 | -35.779 | 37.434 | 1.00 | 23.06 | D000 | C |
| ATOM | 3683 | N | VAL | C | 191 | -12.420 | -38.079 | 37.834 | 1.00 | 26.83 | D000 | N |
| ATOM | 3684 | CA | VAL | C | 191 | -11.106 | -38.192 | 37.221 | 1.00 | 30.57 | D000 | C |
| ATOM | 3685 | C | VAL | C | 191 | -11.136 | -37.388 | 35.927 | 1.00 | 28.23 | D000 | C |
| ATOM | 3686 | O | VAL | C | 191 | -12.017 | -37.590 | 35.096 | 1.00 | 26.03 | D000 | O |
| ATOM | 3687 | CB | VAL | C | 191 | -10.739 | -39.664 | 36.942 | 1.00 | 28.44 | D000 | C |
| ATOM | 3688 | CG1 | VAL | C | 191 | -9.433 | -39.763 | 36.170 | 1.00 | 25.60 | D000 | C |
| ATOM | 3689 | CG2 | VAL | C | 191 | -10.663 | -40.445 | 38.256 | 1.00 | 26.79 | D000 | C |
| ATOM | 3690 | N | VAL | C | 192 | -10.200 | -36.457 | 35.770 | 1.00 | 28.93 | D000 | N |
| ATOM | 3691 | CA | VAL | C | 192 | -10.224 | -35.550 | 34.629 | 1.00 | 27.42 | D000 | C |
| ATOM | 3692 | C | VAL | C | 192 | -9.181 | -35.963 | 33.602 | 1.00 | 31. 88 | D000 | C |
| ATOM | 3693 | O | VAL | C | 192 | -7.984 | -35.957 | 33.888 | 1.00 | 27.55 | D000 | O |
| ATOM | 3694 | CB | VAL | C | 192 | -9.979 | -34.094 | 35.061 | 1.00 | 23.02 | D000 | C |
| ATOM | 3695 | CG1 | VAL | C | 192 | -10.008 | -33.170 | 33.851 | 1.00 | 23.08 | D000 | C |
| ATOM | 3696 | CG2 | VAL | C | 192 | -11.017 | -33.670 | 36.085 | 1.00 | 21.27 | D000 | C |
| ATOM | 3697 | N | THR | C | 193 | -9.637 | -36.314 | 32.403 | 1.00 | 25.64 | D000 | N |
| ATOM | 3698 | CA | THR | C | 193 | -8.751 | -36.918 | 31.415 | 1.00 | 32.64 | D000 | C |
| ATOM | 3699 | C | THR | C | 193 | -8.608 | -36.120 | 30.125 | 1.00 | 31. 08 | D000 | C |
| ATOM | 3700 | O | THR | C | 193 | -7.920 | -36.558 | 29.206 | 1.00 | 30.10 | D000 | O |
| ATOM | 3701 | CB | THR | C | 193 | -9.223 | -38.333 | 31.040 | 1.00 | 33.34 | D000 | C |
| ATOM | 3702 | CG2 | THR | C | 193 | -9.327 | -39.208 | 32.285 | 1.00 | 32.24 | D000 | C |
| ATOM | 3703 | OG1 | THR | C | 193 | -10.501 | -38.252 | 30.398 | 1.00 | 34.27 | D000 | O |
| ATOM | 3704 | N | SER | C | 194 | -9.247 | -34.958 | 30.049 | 1.00 | 29.11 | D000 | N |

(continued)

| | | | | | | | | | | | | |
|---|---|---|---|---|---|---|---|---|---|---|---|---|
| ATOM | 3705 | CA | SER | C | 194 | -9.115 | -34.111 | 28.866 | 1.00 | 29.77 | D000 | C |
| ATOM | 3706 | C | SER | C | 194 | -9.525 | -32.671 | 29.159 | 1.00 | 29.25 | D000 | C |
| ATOM | 3707 | O | SER | C | 194 | -10.227 | -32.400 | 30.137 | 1.00 | 23.94 | D000 | O |
| ATOM | 3708 | CB | SER | C | 194 | -9.956 | -34.663 | 27.708 | 1.00 | 25.83 | D000 | C |
| ATOM | 3709 | OG | SER | C | 194 | -11.335 | -34.423 | 27.935 | 1.00 | 28.83 | D000 | O |
| ATOM | 3710 | N | TRP | C | 195 | -9.080 | -31.753 | 28.305 | 1.00 | 28.44 | D000 | N |
| ATOM | 3711 | CA | TRP | C | 195 | -9.456 | -30.343 | 28.409 | 1.00 | 29.60 | D000 | C |
| ATOM | 3712 | C | TRP | C | 195 | -10.972 | -30.177 | 28.304 | 1.00 | 25.02 | D000 | C |
| ATOM | 3713 | O | TRP | C | 195 | -11.553 | -29.331 | 28.974 | 1.00 | 29.97 | D000 | O |
| ATOM | 3714 | CB | TRP | C | 195 | -8.737 | -29.531 | 27.323 | 1.00 | 26.47 | D000 | C |
| ATOM | 3715 | CG | TRP | C | 195 | -9.010 | -28.039 | 27.283 | 1.00 | 32.42 | D000 | C |
| ATOM | 3716 | CD1 | TRP | C | 195 | -9.356 | -27.306 | 26.181 | 1.00 | 34.68 | D000 | C |
| ATOM | 3717 | CD2 | TRP | C | 195 | -8.932 | -27.101 | 28.375 | 1.00 | 30.83 | D000 | C |
| ATOM | 3718 | CE2 | TRP | C | 195 | -9.245 | -25.827 | 27.853 | 1.00 | 34.03 | D000 | C |
| ATOM | 3719 | CE3 | TRP | C | 195 | -8.631 | -27.215 | 29.736 | 1.00 | 30.71 | D000 | C |
| ATOM | 3720 | NE1 | TRP | C | 195 | -9.498 | -25.981 | 26.515 | 1.00 | 32.39 | D000 | N |
| ATOM | 3721 | CZ2 | TRP | C | 195 | -9.271 | -24.678 | 28.645 | 1.00 | 31. 06 | D000 | C |
| ATOM | 3722 | CZ3 | TRP | C | 195 | -8.655 | -26.071 | 30.523 | 1.00 | 32.95 | D000 | C |
| ATOM | 3723 | CH2 | TRP | C | 195 | -8.969 | -24.820 | 29.974 | 1.00 | 30.07 | D000 | C |
| ATOM | 3724 | N | GLU | C | 196 | -11.607 | -30.999 | 27.471 | 1.00 | 26.97 | D000 | N |
| ATOM | 3725 | CA | GLU | C | 196 | -13.060 | -30.960 | 27.306 | 1.00 | 30.42 | D000 | C |
| ATOM | 3726 | C | GLU | C | 196 | -13.794 | -31.365 | 28.580 | 1.00 | 34.02 | D000 | C |
| ATOM | 3727 | O | GLU | C | 196 | -14.802 | -30.757 | 28.943 | 1.00 | 28.31 | D000 | O |
| ATOM | 3728 | CB | GLU | C | 196 | -13.505 | -31.871 | 26.158 | 1.00 | 30.46 | D000 | C |
| ATOM | 3729 | CG | GLU | C | 196 | -13.221 | -31.329 | 24.769 | 1.00 | 41.10 | D000 | C |
| ATOM | 3730 | CD | GLU | C | 196 | -11.772 | -31.498 | 24.353 | 1.00 | 46.52 | D000 | C |
| ATOM | 3731 | OE1 | GLU | C | 196 | -11.002 | -32.155 | 25.092 | 1.00 | 42.32 | D000 | O |
| ATOM | 3732 | OE2 | GLU | C | 196 | -11.406 | -30.973 | 23.281 | 1.00 | 46.49 | D000 | O |
| ATOM | 3733 | N | GLU | C | 197 | -13.297 | -32.404 | 29.245 | 1.00 | 29.50 | D000 | N |
| ATOM | 3734 | CA | GLU | C | 197 | -13.899 | -32.854 | 30.489 | 1.00 | 26.78 | D000 | C |
| ATOM | 3735 | C | GLU | C | 197 | -13.708 | -31.790 | 31.574 | 1.00 | 26.09 | D000 | C |
| ATOM | 3736 | O | GLU | C | 197 | -14.631 | -31.493 | 32.329 | 1.00 | 27.28 | D000 | O |
| ATOM | 3737 | CB | GLU | C | 197 | -13.305 | -34.198 | 30.930 | 1.00 | 26.61 | D000 | C |
| ATOM | 3738 | CG | GLU | C | 197 | -14.055 | -34.841 | 32.100 | 1.00 | 27.35 | D000 | C |

(continued)

| | | | | | | | | | | | |
|---|---|---|---|---|---|---|---|---|---|---|---|
| ATOM | 3739 | CD | GLU | C | 197 | -13.518 | -36.215 | 32.488 | 1.00 | 37.87 | D000 | C |
| ATOM | 3740 | OE1 | GLU | C | 197 | -12.434 | -36.602 | 32.003 | 1.00 | 33.97 | D000 | O |
| ATOM | 3741 | OE2 | GLU | C | 197 | -14.187 | -36.910 | 33.285 | 1.00 | 39.22 | D000 | O |
| ATOM | 3742 | N | GLN | C | 198 | -12.514 | -31.205 | 31.629 | 1.00 | 25.72 | D000 | N |
| ATOM | 3743 | CA | GLN | C | 198 | -12.214 | -30.140 | 32.588 | 1.00 | 25.80 | D000 | C |
| ATOM | 3744 | C | GLN | C | 198 | -13.189 | -28.975 | 32.463 | 1.00 | 29.00 | D000 | C |
| ATOM | 3745 | O | GLN | C | 198 | -13.685 | -28.458 | 33.464 | 1.00 | 24.52 | D000 | O |
| ATOM | 3746 | CB | GLN | C | 198 | -10.783 | -29.632 | 32.394 | 1.00 | 23.78 | D000 | C |
| ATOM | 3747 | CG | GLN | C | 198 | -10.506 | -28.288 | 33.045 | 1.00 | 25.75 | D000 | C |
| ATOM | 3748 | CD | GLN | C | 198 | -10.246 | -28.397 | 34.537 | 1.00 | 33.71 | D000 | C |
| ATOM | 3749 | NE2 | GLN | C | 198 | -10.599 | -27.350 | 35.279 | 1.00 | 25.10 | D000 | N |
| ATOM | 3750 | OE1 | GLN | C | 198 | -9.734 | -29.410 | 35.017 | 1.00 | 28.06 | D000 | O |
| ATOM | 3751 | N | LYS | C | 199 | -13.466 | -28.574 | 31.226 | 1.00 | 30.60 | D000 | N |
| ATOM | 3752 | CA | LYS | C | 199 | -14.326 | -27.424 | 30.974 | 1.00 | 34.08 | D000 | C |
| ATOM | 3753 | C | LYS | C | 199 | -15.770 | -27.743 | 31.319 | 1.00 | 28.60 | D000 | C |
| ATOM | 3754 | O | LYS | C | 199 | -16.489 | -26.895 | 31.848 | 1.00 | 25.57 | D000 | O |
| ATOM | 3755 | CB | LYS | C | 199 | -14.212 | -26.978 | 29.517 | 1.00 | 28.25 | D000 | C |
| ATOM | 3756 | CG | LYS | C | 199 | -12.855 | -26.386 | 29.183 | 1.00 | 34.79 | D000 | C |
| ATOM | 3757 | CD | LYS | C | 199 | -12.532 | -26.545 | 27.711 | 1.00 | 43.54 | D000 | C |
| ATOM | 3758 | CE | LYS | C | 199 | -13.150 | -25.447 | 26.892 | 1.00 | 38.89 | D000 | C |
| ATOM | 3759 | NZ | LYS | C | 199 | -12.740 | -25.539 | 25.453 | 1.00 | 35.02 | D000 | N |
| ATOM | 3760 | N | PHE | C | 200 | -16.188 | -28.968 | 31.013 | 1.00 | 29.54 | D000 | N |
| ATOM | 3761 | CA | PHE | C | 200 | -17.515 | -29.436 | 31.393 | 1.00 | 30.79 | D000 | C |
| ATOM | 3762 | C | PHE | C | 200 | -17.682 | -29.381 | 32.909 | 1.00 | 25.62 | D000 | C |
| ATOM | 3763 | O | PHE | C | 200 | -18.651 | -28.807 | 33.414 | 1.00 | 25.66 | D000 | O |
| ATOM | 3764 | CB | PHE | C | 200 | -17.762 | -30.861 | 30.891 | 1.00 | 27.79 | D000 | C |
| ATOM | 3765 | CG | PHE | C | 200 | -18.939 | -31.530 | 31.541 | 1.00 | 33.05 | D000 | C |
| ATOM | 3766 | CD1 | PHE | C | 200 | -20.230 | -31.216 | 31.154 | 1.00 | 29.57 | D000 | C |
| ATOM | 3767 | CD2 | PHE | C | 200 | -18.754 | -32.467 | 32.547 | 1.00 | 33.93 | D000 | C |
| ATOM | 3768 | CE1 | PHE | C | 200 | -21.315 | -31.826 | 31.753 | 1.00 | 31.19 | D000 | C |
| ATOM | 3769 | CE2 | PHE | C | 200 | -19.837 | -33.077 | 33.154 | 1.00 | 30.44 | D000 | C |
| ATOM | 3770 | CZ | PHE | C | 200 | -21.118 | -32.756 | 32.755 | 1.00 | 33.80 | D000 | C |
| ATOM | 3771 | N | VAL | C | 201 | -16.730 | -29.971 | 33.627 | 1.00 | 27.40 | D000 | N |
| ATOM | 3772 | CA | VAL | C | 201 | -16.756 | -29.964 | 35.088 | 1.00 | 26.65 | D000 | C |

(continued)

| | | | | | | | | | | | |
|---|---|---|---|---|---|---|---|---|---|---|---|
| ATOM | 3773 | C | VAL | C | 201 | -16.750 | -28.533 | 35.609 | 1.00 | 33.34 | D000 | C |
| ATOM | 3774 | O | VAL | C | 201 | -17.593 | -28.149 | 36.428 | 1.00 | 25.82 | D000 | O |
| ATOM | 3775 | CB | VAL | C | 201 | -15.557 | -30.727 | 35.679 | 1.00 | 30.52 | D000 | C |
| ATOM | 3776 | CG1 | VAL | C | 201 | -15.448 | -30.466 | 37.173 | 1.00 | 27.81 | D000 | C |
| ATOM | 3777 | CG2 | VAL | C | 201 | -15.677 | -32.219 | 35.392 | 1.00 | 24.80 | D000 | C |
| ATOM | 3778 | N | GLN | C | 202 | -15.804 | -27.748 | 35.098 | 1.00 | 28.75 | D000 | N |
| ATOM | 3779 | CA | GLN | C | 202 | -15.629 | -26.353 | 35.482 | 1.00 | 28.03 | D000 | C |
| ATOM | 3780 | C | GLN | C | 202 | -16.905 | -25.544 | 35.295 | 1.00 | 26.50 | D000 | C |
| ATOM | 3781 | O | GLN | C | 202 | -17.221 | -24.662 | 36.097 | 1.00 | 30.81 | D000 | O |
| ATOM | 3782 | CB | GLN | C | 202 | -14.495 | -25.736 | 34.663 | 1.00 | 28.31 | D000 | C |
| ATOM | 3783 | CG | GLN | C | 202 | -13.779 | -24.603 | 35.333 | 1.00 | 34.63 | D000 | C |
| ATOM | 3784 | CD | GLN | C | 202 | -12.592 | -24.127 | 34.524 | 1.00 | 36.40 | D000 | C |
| ATOM | 3785 | NE2 | GLN | C | 202 | -12.382 | -22.815 | 34.494 | 1.00 | 34.64 | D000 | N |
| ATOM | 3786 | OE1 | GLN | C | 202 | -11.874 | -24.931 | 33.924 | 1.00 | 33.68 | D000 | O |
| ATOM | 3787 | N | HIS | C | 203 | -17.640 | -25.848 | 34.232 | 1.00 | 28.19 | D000 | N |
| ATOM | 3788 | CA | HIS | C | 203 | -18.895 | -25.158 | 33.971 | 1.00 | 28.76 | D000 | C |
| ATOM | 3789 | C | HIS | C | 203 | -19.921 | -25.389 | 35.081 | 1.00 | 37.82 | D000 | C |
| ATOM | 3790 | O | HIS | C | 203 | -20.664 | -24.477 | 35.450 | 1.00 | 30.91 | D000 | O |
| ATOM | 3791 | CB | HIS | C | 203 | -19.490 | -25.597 | 32.633 | 1.00 | 27.45 | D000 | C |
| ATOM | 3792 | CG | HIS | C | 203 | -20.836 | -25.002 | 32.361 | 1.00 | 35.85 | D000 | C |
| ATOM | 3793 | CD2 | HIS | C | 203 | -21.189 | -23.825 | 31.791 | 1.00 | 40.47 | D000 | C |
| ATOM | 3794 | ND1 | HIS | C | 203 | -22.010 | -25.619 | 32.728 | 1.00 | 42.36 | D000 | N |
| ATOM | 3795 | CE1 | HIS | C B | 203 | -23.035 | -24.857 | 32.380 | 1.00 | 38.39 | D000 | C |
| ATOM | 3796 | NE2 | HIS | C | 203 | -22.561 | -23.764 | 31.810 | 1.00 | 42.37 | D000 | N |
| ATOM | 3797 | N | HIS | C | 204 | -19.970 | -26.608 | 35.612 | 1.00 | 31.47 | D000 | N |
| ATOM | 3798 | CA | HIS | C | 204 | -20.992 | -26.941 | 36.602 | 1.00 | 30.96 | D000 | C |
| ATOM | 3799 | C | HIS | C | 204 | -20.619 | -26.543 | 38.023 | 1.00 | 29.48 | D000 | C |
| ATOM | 3800 | O | HIS | C | 204 | -21.489 | -26.143 | 38.796 | 1.00 | 28.82 | D000 | O |
| ATOM | 3801 | CB | HIS | C | 204 | -21.317 | -28.434 | 36.545 | 1.00 | 30.72 | D000 | C |
| ATOM | 3802 | CG | HIS | C | 204 | -22.172 | -28.808 | 35.376 | 1.00 | 31.65 | D000 | C |
| ATOM | 3803 | CD2 | HIS | C | 204 | -21.889 | -29.495 | 34.244 | 1.00 | 34.41 | D000 | C |
| ATOM | 3804 | ND1 | HIS | C | 204 | -23.494 | -28.433 | 35.274 | 1.00 | 39.82 | D000 | N |
| ATOM | 3805 | CE1 | HIS | C | 204 | -23.993 | -28.884 | 34.138 | 1.00 | 35.14 | D000 | C |
| ATOM | 3806 | NE2 | HIS | C | 204 | -23.038 | -29.531 | 33.493 | 1.00 | 39.82 | D000 | N |

(continued)

| | | | | | | | | | | | | |
|---|---|---|---|---|---|---|---|---|---|---|---|---|
| ATOM | 3807 | N | ILE | C | 205 | -19.340 | -26.628 | 38.375 | 1.00 | 26.45 | D000 | N |
| ATOM | 3808 | CA | ILE | C | 205 | -18.942 | -26.275 | 39.735 | 1.00 | 32.57 | D000 | C |
| ATOM | 3809 | C | ILE | C | 205 | -18.811 | -24.758 | 39.911 | 1.00 | 29.31 | D000 | C |
| ATOM | 3810 | O | ILE | C | 205 | -18.976 | -24.244 | 41.015 | 1.00 | 30.32 | D000 | O |
| ATOM | 3811 | CB | ILE | C | 205 | -17.613 | -26.955 | 40.152 | 1.00 | 31.86 | D000 | C |
| ATOM | 3812 | CG1 | ILE | C | 205 | -16.437 | -26.421 | 39.339 | 1.00 | 31.34 | D000 | C |
| ATOM | 3813 | CG2 | ILE | C | 205 | -17.710 | -28.477 | 40.014 | 1.00 | 29.23 | D000 | C |
| ATOM | 3814 | CD1 | ILE | C | 205 | -15.107 | -26.968 | 39.802 | 1.00 | 32.36 | D000 | C |
| ATOM | 3815 | N | GLY | C | 206 | -18.540 | -24.043 | 38.825 | 1.00 | 24.89 | D000 | N |
| ATOM | 3816 | CA | GLY | C | 206 | -18.307 | -22.613 | 38.912 | 1.00 | 26.12 | D000 | C |
| ATOM | 3817 | C | GLY | C | 206 | -17.033 | -22.318 | 39.688 | 1.00 | 33.16 | D000 | C |
| ATOM | 3818 | O | GLY | C | 206 | -16.182 | -23.197 | 39.845 | 1.00 | 27.62 | D000 | O |
| ATOM | 3819 | N | PRO | C | 207 | -16.895 | -21.081 | 40.189 | 1.00 | 27.47 | D000 | N |
| ATOM | 3820 | CA | PRO | C | 207 | -15.679 | -20.657 | 40.895 | 1.00 | 28.28 | D000 | C |
| ATOM | 3821 | C | PRO | C | 207 | -15.651 | -21.107 | 42.360 | 1.00 | 33.47 | D000 | C |
| ATOM | 3822 | O | PRO | C | 207 | -15.598 | -20.266 | 43.255 | 1.00 | 30.13 | D000 | O |
| ATOM | 3823 | CB | PRO | C | 207 | -15.741 | -19.130 | 40.792 | 1.00 | 27.65 | D000 | C |
| ATOM | 3824 | CG | PRO | C | 207 | -17.204 | -18.830 | 40.787 | 1.00 | 30.21 | D000 | C |
| ATOM | 3825 | CD | PRO | C | 207 | -17 . 873 | -19.984 | 40.065 | 1.00 | 29.69 | D000 | C |
| ATOM | 3826 | N | VAL | C | 208 | -15.674 | -22.417 | 42.594 | 1.00 | 36.48 | D000 | N |
| ATOM | 3827 | CA | VAL | C | 208 | -15.803 | -22.969 | 43.944 | 1.00 | 29.83 | D000 | C |
| ATOM | 3828 | C | VAL | C | 208 | -14.809 | -24.105 | 44.182 | 1.00 | 33.04 | D000 | C |
| ATOM | 3829 | O | VAL | C | 208 | -14.697 | -25.001 | 43.351 | 1.00 | 26.89 | D000 | O |
| ATOM | 3830 | CB | VAL | | 208 | -17.233 | -23.504 | 44.191 | 1.00 | 35.14 | D000 | C |
| ATOM | 3831 | CG1 | VAL | C | 208 | -17.349 | -24.108 | 45.585 | 1.00 | 33.92 | D000 | c |
| ATOM | 3832 | CG2 | VAL | C | 208 | -18.271 | -22.402 | 43.981 | 1.00 | 32.86 | D000 | C |
| ATOM | 3833 | N | ASN | C | 209 | -14.092 | -24.071 | 45.306 | 1.00 | 30.99 | D000 | N |
| ATOM | 3834 | CA | ASN | C | 209 | -13.164 | -25.153 | 45.649 | 1.00 | 31.35 | D000 | C |
| ATOM | 3835 | C | ASN | C | 209 | -13.881 | -26.500 | 45.682 | 1.00 | 32.29 | D000 | C |
| ATOM | 3836 | O | ASN | C | 209 | -14.881 | -26.666 | 46.379 | 1.00 | 29.08 | D000 | O |
| ATOM | 3837 | CB | ASN | C | 209 | -12.486 | -24.895 | 46.994 | 1.00 | 25.98 | D000 | C |
| ATOM | 3838 | CG | ASN | C | 209 | -11.461 | -23.781 | 46.931 | 1.00 | 30.97 | D000 | C |
| ATOM | 3839 | ND2 | ASN | C | 209 | -11.376 | -22.997 | 48.000 | 1.00 | 35.10 | D000 | N |
| ATOM | 3840 | OD1 | ASN | C | 209 | -10.750 | -23.626 | 45.938 | 1.00 | 30.97 | D000 | O |

(continued)

| | | | | | | | | | | | |
|---|---|---|---|---|---|---|---|---|---|---|---|
| ATOM | 3841 | N | THR | C | 210 | -13.363 | -27.457 | 44.919 | 1.00 | 30.16 | D000 | N |
| ATOM | 3842 | CA | THR | C | 210 | -14.049 | -28.725 | 44.705 | 1.00 | 26.80 | D000 | C |
| ATOM | 3843 | C | THR | C | 210 | -13.049 | -29.865 | 44.549 | 1.00 | 26.68 | D000 | C |
| ATOM | 3844 | O | THR | C | 210 | -12.182 | -29.822 | 43.673 | 1.00 | 30.56 | D000 | O |
| ATOM | 3845 | CB | THR | C | 210 | -14.949 | -28.655 | 43.456 | 1.00 | 29.42 | D000 | C |
| ATOM | 3846 | CG2 | THR | C | 210 | -15.785 | -29.916 | 43.312 | 1.00 | 22.22 | D000 | C |
| ATOM | 3847 | OG1 | THR | C | 210 | -15.813 | -27.515 | 43.556 | 1.00 | 26.16 | D000 | O |
| ATOM | 3848 | N | TRP | C | 211 | -13.165 | -30.872 | 45.410 | 1.00 | 25.09 | D000 | N |
| ATOM | 3849 | CA | TRP | C | 211 | -12.294 | -32.041 | 45.357 | 1.00 | 30.07 | D000 | C |
| ATOM | 3850 | C | TRP | C | 211 | -12.423 | -32.796 | 44.038 | 1.00 | 36.81 | D000 | C |
| ATOM | 3851 | O | TRP | C | 211 | -13.510 | -32.874 | 43.456 | 1.00 | 27.60 | D000 | O |
| ATOM | 3852 | CB | TRP | C | 211 | -12.605 | -33.013 | 46.501 | 1.00 | 27.04 | D000 | C |
| ATOM | 3853 | CG | TRP | C | 211 | -12.261 | -32.549 | 47.900 | 1.00 | 28.12 | D000 | C |
| ATOM | 3854 | CD1 | TRP | C | 211 | -13.097 | -32.529 | 48.983 | 1.00 | 28.01 | D000 | C |
| ATOM | 3855 | CD2 | TRP | C | 211 | -10.991 | -32.072 | 48.366 | 1.00 | 26.23 | D000 | C |
| ATOM | 3856 | CE2 | TRP | C | 211 | -11.136 | -31.771 | 49.739 | 1.00 | 31.66 | D000 | C |
| ATOM | 3857 | CE3 | TRP | C | 211 | -9.751 | -31.857 | 47.756 | 1.00 | 23.53 | D000 | C |
| ATOM | 3858 | NE1 | TRP | C | 211 | -12.428 | -32.060 | 50.091 | 1.00 | 29.06 | D000 | N |
| ATOM | 3859 | CZ2 | TRP | C | 211 | -10.084 | -31.274 | 50.509 | 1.00 | 31.81 | D000 | C |
| ATOM | 3860 | CZ3 | TRP | C | 211 | -8.709 | -31.361 | 48.522 | 1.00 | 26.50 | D000 | C |
| ATOM | 3861 | CH2 | TRP | C | 211 | -8.881 | -31.078 | 49.885 | 1.00 | 32.71 | D000 | C |
| ATOM | 3862 | N | MET | c | 212 | -11.310 | -33.363 | 43.585 | 1.00 | 29.78 | D000 | N |
| ATOM | 3863 | CA | MET | c | 212 | -11.327 | -34.332 | 42.500 | 1.00 | 26.98 | D000 | C |
| ATOM | 3864 | C | MET | c | 212 | -10.681 | -35.620 | 43.009 | 1.00 | 30.71 | D000 | C |
| ATOM | 3865 | O | MET | c | 212 | -10.179 | -35.655 | 44.134 | 1.00 | 26.90 | D000 | O |
| ATOM | 3866 | CB | MET | c | 212 | -10.598 | -33.793 | 41.266 | 1.00 | 28.53 | D000 | C |
| ATOM | 3867 | CG | MET | c | 212 | -9.082 | -33.872 | 41.344 | 1.00 | 27.04 | D000 | C |
| ATOM | 3868 | SD | MET | c | 212 | -8.287 | -32.882 | 40.061 | 1.00 | 30.51 | D000 | S |
| ATOM | 3869 | CE | MET | c | 212 | -8.761 | -31.231 | 40.597 | 1.00 | 22.33 | D000 | C |
| ATOM | 3870 | N | GLY | c | 213 | -10.693 | -36.673 | 42.195 | 1.00 | 23.24 | D000 | N |
| ATOM | 3871 | CA | GLY | c | 213 | -10.149 | -37.955 | 42.612 | 1.00 | 21.72 | D000 | C |
| ATOM | 3872 | C | GLY | c | 213 | -8.655 | -38.108 | 42.405 | 1.00 | 27.75 | D000 | C |
| ATOM | 3873 | O | GLY | c | 213 | -8.199 | -39.106 | 41.851 | 1.00 | 28.41 | D000 | O |
| ATOM | 3874 | N | LEU | c | 214 | -7.891 | -37.124 | 42.870 | 1.00 | 25.92 | D000 | N |

(continued)

| | | | | | | | | | | | |
|---|---|---|---|---|---|---|---|---|---|---|---|
| ATOM | 3875 | CA | LEU | c | 214 | -6.443 | -37.109 | 42.691 | 1.00 | 26.73 | D000 | C |
| ATOM | 3876 | C | LEU | c | 214 | -5.755 | -36.799 | 44.018 | 1.00 | 25.75 | D000 | C |
| ATOM | 3877 | O | LEU | c | 214 | -6.073 | -35.802 | 44.659 | 1.00 | 25.95 | D000 | O |
| ATOM | 3878 | CB | LEU | c | 214 | -6.049 | -36.076 | 41.629 | 1.00 | 24.90 | D000 | C |
| ATOM | 3879 | CG | LEU | c | 214 | -4.566 | -35.777 | 41.383 | 1.00 | 25.82 | D000 | C |
| ATOM | 3880 | CD1 | LEU | c | 214 | -3.840 | -37.006 | 40.861 | 1.00 | 22.24 | D000 | C |
| ATOM | 3881 | CD2 | LEU | c | 214 | -4.428 | -34.615 | 40.402 | 1.00 | 20.00 | D000 | C |
| ATOM | 3882 | N | HIS | c | 215 | -4.819 | -37.653 | 44.426 | 1.00 | 28.82 | D000 | N |
| ATOM | 3883 | CA | HIS | c | 215 | -4.139 | -37.488 | 45.709 | 1.00 | 25.47 | D000 | C |
| ATOM | 3884 | C | HIS | c | 215 | -2.797 | -38.207 | 45.727 | 1.00 | 27.39 | D000 | C |
| ATOM | 3885 | O | HIS | c | 215 | -2.570 | -39.123 | 44.938 | 1.00 | 28.76 | D000 | O |
| ATOM | 3886 | CB | HIS | c | 215 | -5.011 | -38.011 | 46.850 | 1.00 | 25.17 | D000 | C |
| ATOM | 3887 | CG | HIS | c | 215 | -5.082 | -39.505 | 46.915 | 1.00 | 33.85 | D000 | C |
| ATOM | 3888 | CD2 | HIS | c | 215 | -5.484 | -40.419 | 45.999 | 1.00 | 36.27 | D000 | C |
| ATOM | 3889 | ND1 | HIS | c | 215 | -4.705 | -40.221 | 48.030 | 1.00 | 43.52 | D000 | N |
| ATOM | 3890 | CE1 | HIS | c | 215 | -4 . 874 | -41.511 | 47.800 | 1.00 | 45.66 | D000 | C |
| ATOM | 3891 | NE2 | HIS | c | 215 | -5.347 | -41.657 | 46.575 | 1.00 | 38.89 | D000 | N |
| ATOM | 3892 | N | ASP | C | 216 | -1.908 | -37.803 | 46.632 | 1.00 | 31.91 | D000 | N |
| ATOM | 3893 | CA | ASP | C | 216 | -0.623 | -38.485 | 46.760 | 1.00 | 28.17 | D000 | C |
| ATOM | 3894 | C | ASP | C | 216 | -0.369 | -38.944 | 48.199 | 1.00 | 35.16 | D000 | C |
| ATOM | 3895 | O | ASP | C | 216 | 0.764 | -38.955 | 48.668 | 1.00 | 31.36 | D000 | O |
| ATOM | 3896 | CB | ASP | C | 216 | 0.518 | -37.585 | 46.258 | 1.00 | 28.42 | D000 | C |
| ATOM | 3897 | CG | ASP | C | 216 | 0.795 | -36.394 | 47.171 | 1.00 | 35.01 | D000 | C |
| ATOM | 3898 | OD1 | ASP | C | 216 | 0.013 | -36.142 | 48.110 | 1.00 | 28.86 | D000 | O |
| ATOM | 3899 | OD2 | ASP | C | 216 | 1.805 | -35.695 | 46.936 | 1.00 | 31.47 | D000 | O |
| ATOM | 3900 | N | GLN | c | 217 | -1.432 | -39.332 | 48.894 | 1.00 | 31. 06 | D000 | N |
| ATOM | 3901 | CA | GLN | c | 217 | -1.305 | -39.781 | 50.278 | 1.00 | 41.47 | D000 | C |
| ATOM | 3902 | C | GLN | C | 217 | -0.531 | -41.103 | 50.385 | 1.00 | 38.57 | D000 | c |
| ATOM | 3903 | O | GLN | C | 217 | 0.157 | -41.346 | 51.374 | 1.00 | 44.82 | D000 | O |
| ATOM | 3904 | CB | GLN | C | 217 | -2.688 | -39.926 | 50.918 | 1.00 | 37.52 | D000 | c |
| ATOM | 3905 | CG | GLN | C | 217 | -3.504 | -38. 644 | 50.919 | 1.00 | 33.20 | D000 | c |
| ATOM | 3906 | CD | GLN | C | 217 | -4.837 | -38.799 | 51.626 | 1.00 | 39.32 | D000 | C |
| ATOM | 3907 | NE2 | GLN | C | 217 | -5.810 | -39.389 | 50.941 | 1.00 | 31.27 | D000 | N |
| ATOM | 3908 | OE1 | GLN | C | 217 | -4.989 | -38.391 | 52.775 | 1.00 | 48.65 | D000 | O |

(continued)

| | | | | | | | | | | | | |
|---|---|---|---|---|---|---|---|---|---|---|---|---|
| ATOM | 3909 | N | ASN | C | 218 | -0.630 | -41.939 | 49.356 | 1.00 | 38.48 | D000 | N |
| ATOM | 3910 | CA | ASN | C | 218 | 0.018 | -43.251 | 49.363 | 1.00 | 53.29 | D000 | C |
| ATOM | 3911 | C | ASN | C | 218 | 1.205 | -43.357 | 48.405 | 1.00 | 47.43 | D000 | C |
| ATOM | 3912 | O | ASN | C | 218 | 1.397 | -44.389 | 47.761 | 1.00 | 58.53 | D000 | O |
| ATOM | 3913 | CB | ASN | C | 218 | -1.004 | -44.339 | 49.018 | 1.00 | 57.61 | D000 | C |
| ATOM | 3914 | CG | ASN | C | 218 | -2.213 | -44.313 | 49.933 | 1.00 | 65.75 | D000 | C |
| ATOM | 3915 | ND2 | ASN | C | 218 | -1.966 | -44.302 | 51.241 | 1.00 | 59.68 | D000 | N |
| ATOM | 3916 | OD1 | ASN | C | 218 | -3.356 | -44.293 | 49.471 | 1.00 | 59.70 | D000 | O |
| ATOM | 3917 | N | GLY | C | 219 | 2.001 | -42.299 | 48.313 | 1.00 | 35.56 | D000 | N |
| ATOM | 3918 | CA | GLY | C | 219 | 3.081 | -42.255 | 47.344 | 1.00 | 36.85 | D000 | C |
| ATOM | 3919 | C | GLY | C | 219 | 2.873 | -41.128 | 46.353 | 1.00 | 34.18 | D000 | C |
| ATOM | 3920 | O | GLY | C | 219 | 2.347 | -40.080 | 46.716 | 1.00 | 32.25 | D000 | O |
| ATOM | 3921 | N | PRO | C | 220 | 3.284 | -41.332 | 45.092 | 1.00 | 35.79 | D000 | N |
| ATOM | 3922 | CA | PRO | C | 220 | 3.144 | -40.284 | 44.072 | 1.00 | 33.07 | D000 | C |
| ATOM | 3923 | C | PRO | C | 220 | 1.683 | -40.001 | 43.728 | 1.00 | 31.10 | D000 | C |
| ATOM | 3924 | O | PRO | C | 220 | 0.801 | -40.767 | 44.117 | 1.00 | 29.49 | D000 | O |
| ATOM | 3925 | CB | PRO | C | 220 | 3.897 | -40.859 | 42.867 | 1.00 | 37.25 | D000 | C |
| ATOM | 3926 | CG | PRO | C | 220 | 3.868 | -42.336 | 43.073 | 1.00 | 41.99 | D000 | C |
| ATOM | 3927 | CD | PRO | C | 220 | 3.925 | -42.547 | 44.558 | 1.00 | 34.70 | D000 | C |
| ATOM | 3928 | N | TRP | C | 221 | 1.433 | -38.904 | 43.019 | 1.00 | 28.69 | D000 | N |
| ATOM | 3929 | CA | TRP | C | 221 | 0.070 | -38.514 | 42.668 | 1.00 | 29.66 | D000 | C |
| ATOM | 3930 | C | TRP | C | 221 | -0.626 | -39.607 | 41.857 | 1.00 | 29.27 | D000 | C |
| ATOM | 3931 | O | TRP | C | 221 | -0.081 | -40.110 | 40.879 | 1.00 | 26.36 | D000 | O |
| ATOM | 3932 | CB | TRP | C | 221 | 0.075 | -37.185 | 41.900 | 1.00 | 27.58 | D000 | C |
| ATOM | 3933 | CG | TRP | C | 221 | 0.406 | -36.020 | 42.782 | 1.00 | 30.86 | D000 | C |
| ATOM | 3934 | CD1 | TRP | C | 221 | 1.629 | -35.437 | 42.947 | 1.00 | 32.27 | D000 | C |
| ATOM | 3935 | CD2 | TRP | C | 221 | -0.495 | -35.311 | 43.643 | 1.00 | 27.75 | D000 | C |
| ATOM | 3936 | CE2 | TRP | C | 221 | 0.253 | -34.309 | 44.298 | 1.00 | 32.54 | D000 | C |
| ATOM | 3937 | CE3 | TRP | C | 221 | -1.861 | -35.428 | 43.926 | 1.00 | 25.76 | D000 | C |
| ATOM | 3938 | NE1 | TRP | C | 221 | 1.545 | -34.406 | 43.853 | 1.00 | 32.24 | D000 | N |
| ATOM | 3939 | CZ2 | TRP | C | 221 | -0.320 | -33.425 | 45.219 | 1.00 | 27.09 | D000 | C |
| ATOM | 3940 | CZ3 | TRP | C | 221 | -2.430 | -34.549 | 44 . 841 | 1.00 | 29.33 | D000 | C |
| ATOM | 3941 | CH2 | TRP | C | 221 | -1.658 | -33.560 | 45.474 | 1.00 | 29.04 | D000 | C |
| ATOM | 3942 | N | LYS | C | 222 | -1.826 | -39.981 | 42.289 | 1.00 | 30.12 | D000 | N |

(continued)

| | | | | | | | | | | | | |
|---|---|---|---|---|---|---|---|---|---|---|---|---|
| ATOM | 3943 | CA | LYS | C | 222 | -2.585 | -41.046 | 41.643 | 1.00 | 30.34 | D000 | C |
| ATOM | 3944 | C | LYS | C | 222 | -4.058 | -40.687 | 41.502 | 1.00 | 27.82 | D000 | C |
| ATOM | 3945 | O | LYS | C | 222 | -4.627 | -40.026 | 42.370 | 1.00 | 29.51 | D000 | O |
| ATOM | 3946 | CB | LYS | C | 222 | -2.453 | -42.351 | 42.436 | 1.00 | 35.42 | D000 | C |
| ATOM | 3947 | CG | LYS | C | 222 | -1.072 | -42.983 | 42.388 | 1.00 | 42.04 | D000 | C |
| ATOM | 3948 | CD | LYS | C | 222 | -0.966 | -44 . 138 | 43.373 | 1.00 | 62.76 | D000 | C |
| ATOM | 3949 | CE | LYS | C | 222 | 0.452 | -44.687 | 43.443 | 1.00 | 66.16 | D000 | C |
| ATOM | 3950 | NZ | LYS | C | 222 | 0.599 | -45.699 | 44.533 | 1.00 | 72.02 | D000 | N |
| ATOM | 3951 | N | TRP | C | 223 | -4.670 | -41.121 | 40.406 | 1.00 | 29.14 | D000 | N |
| ATOM | 3952 | CA | TRP | C | 223 | -6.119 | -41.022 | 40.249 | 1.00 | 31.28 | D000 | C |
| ATOM | 3953 | C | TRP | C | 223 | -6.787 | -42.218 | 40.920 | 1.00 | 29.31 | D000 | C |
| ATOM | 3954 | O | TRP | C | 223 | -6.254 | -43.321 | 40.883 | 1.00 | 30.17 | D000 | O |
| ATOM | 3955 | CB | TRP | C | 223 | -6.512 | -40.959 | 38.774 | 1.00 | 23.94 | D000 | C |
| ATOM | 3956 | CG | TRP | C | 223 | -6.104 | -39.694 | 38.085 | 1.00 | 27.95 | D000 | C |
| ATOM | 3957 | CD1 | TRP | C | 223 | -5.059 | -39.530 | 37.226 | 1.00 | 23.23 | D000 | C |
| ATOM | 3958 | CD2 | TRP | C | 223 | -6.742 | -38.414 | 38.191 | 1.00 | 24.93 | D000 | C |
| ATOM | 3959 | CE2 | TRP | C | 223 | -6.026 | -37.523 | 37.365 | 1.00 | 23.92 | D000 | C |
| ATOM | 3960 | CE3 | TRP | C | 223 | -7.851 | -37.938 | 38.901 | 1.00 | 23.92 | D000 | C |
| ATOM | 3961 | NE1 | TRP | C | 223 | -5.002 | -38.228 | 36.790 | 1.00 | 25.78 | D000 | N |
| ATOM | 3962 | CZ2 | TRP | C | 223 | -6.382 | -36.182 | 37.228 | 1.00 | 28.71 | D000 | C |
| ATOM | 3963 | CZ3 | TRP | C | 223 | -8.201 | -36.603 | 38.771 | 1.00 | 28.69 | D000 | C |
| ATOM | 3964 | CH2 | TRP | C | 223 | -7.468 | -35.740 | 37.941 | 1.00 | 27.80 | D000 | C |
| ATOM | 3965 | N | VAL | C | 224 | -7.958 | -42.001 | 41.514 | 1.00 | 28.47 | D000 | N |
| ATOM | 3966 | CA | VAL | C | 224 | -8.620 | -43.038 | 42.302 | 1.00 | 29.16 | D000 | C |
| ATOM | 3967 | C | VAL | C | 224 | -9.055 | -44.259 | 41.486 | 1.00 | 31.96 | D000 | C |
| ATOM | 3968 | O | VAL | C | 224 | -9.186 | -45.349 | 42.038 | 1.00 | 33.21 | D000 | O |
| ATOM | 3969 | CB | VAL | C | 224 | -9.866 | -42.485 | 43.037 | 1.00 | 27.95 | D000 | C |
| ATOM | 3970 | CG1 | VAL | C | 224 | -9.463 | -41.450 | 44.071 | 1.00 | 29.62 | D000 | C |
| ATOM | 3971 | CG2 | VAL | C | 224 | -10.863 | -41.900 | 42.050 | 1.00 | 31.35 | D000 | C |
| ATOM | 3972 | N | ASP | C | 225 | -9.272 | -44.083 | 40.184 | 1.00 | 30.51 | D000 | N |
| ATOM | 3973 | CA | ASP | C | 225 | -9.755 | -45.182 | 39.347 | 1.00 | 33.42 | D000 | c |
| ATOM | 3974 | C | ASP | C | 225 | -8.622 | -45.903 | 38.615 | 1.00 | 35.01 | D000 | c |
| ATOM | 3975 | O | ASP | C | 225 | -8.866 | -46.705 | 37.717 | 1.00 | 45.07 | D000 | O |
| ATOM | 3976 | CB | ASP | C | 225 | -10.798 | -44.673 | 38.337 | 1.00 | 34.24 | D000 | c |

(continued)

| | | | | | | | | | | | | |
|---|---|---|---|---|---|---|---|---|---|---|---|---|
| ATOM | 3977 | CG | ASP | C | 225 | -10.187 | -43.849 | 37.205 | 1.00 | 39.60 | D000 | c |
| ATOM | 3978 | OD1 | ASP | C | 225 | -9.032 | -43.379 | 37.334 | 1.00 | 35.30 | D000 | O |
| ATOM | 3979 | OD2 | ASP | C | 225 | -10.878 | -43.658 | 36.179 | 1.00 | 41.15 | D000 | O |
| ATOM | 3980 | N | GLY | C | 226 | -7.383 | -45.605 | 38.989 | 1.00 | 34.08 | D000 | N |
| ATOM | 3981 | CA | GLY | C | 226 | -6.241 | -46.272 | 38.389 | 1.00 | 29.68 | D000 | C |
| ATOM | 3982 | C | GLY | C | 226 | -5.723 | -45.631 | 37.115 | 1.00 | 34.65 | D000 | C |
| ATOM | 3983 | O | GLY | C | 226 | -4.725 | -46.086 | 36.555 | 1.00 | 35.05 | D000 | O |
| ATOM | 3984 | N | THR | C | 227 | -6.394 | -44.578 | 36.652 | 1.00 | 34.54 | D000 | N |
| ATOM | 3985 | CA | THR | C | 227 | -5.926 | -43.829 | 35.486 | 1.00 | 32.36 | D000 | C |
| ATOM | 3986 | C | THR | C | 227 | -4.500 | -43.333 | 35.721 | 1.00 | 35.82 | D000 | C |
| ATOM | 3987 | O | THR | C | 227 | -4.196 | -42.779 | 36.779 | 1.00 | 36.25 | D000 | O |
| ATOM | 3988 | CB | THR | C | 227 | -6.840 | -42.625 | 35.175 | 1.00 | 36.95 | D000 | C |
| ATOM | 3989 | CG2 | THR | C | 227 | -6.315 | -41.834 | 33.980 | 1.00 | 37.07 | D000 | C |
| ATOM | 3990 | OG1 | THR | C | 227 | -8.164 | -43.089 | 34.891 | 1.00 | 40.09 | D000 | O |
| ATOM | 3991 | N | ASP | C | 228 | -3.625 | -43.540 | 34.743 | 1.00 | 35.21 | D000 | N |
| ATOM | 3992 | CA | ASP | C | 228 | -2.227 | -43.165 | 34.900 | 1.00 | 35.66 | D000 | C |
| ATOM | 3993 | C | ASP | C | 228 | -2.063 | -41.648 | 34.951 | 1.00 | 40.45 | D000 | C |
| ATOM | 3994 | O | ASP | C | 228 | -2.550 | -40.926 | 34.080 | 1.00 | 33.72 | D000 | O |
| ATOM | 3995 | CB | ASP | C | 228 | -1.378 | -43.748 | 33.773 | 1.00 | 36.06 | D000 | C |
| ATOM | 3996 | CG | ASP | C | 228 | 0.098 | -43.451 | 33.953 | 1.00 | 42.86 | D000 | C |
| ATOM | 3997 | OD1 | ASP | C | 228 | 0.720 | -44.052 | 34.855 | 1.00 | 52.83 | D000 | O |
| ATOM | 3998 | OD2 | ASP | C | 228 | 0.636 | -42.613 | 33.199 | 1.00 | 43.62 | D000 | O |
| ATOM | 3999 | N | TYR | C | 229 | -1.372 | -41.168 | 35.978 | 1.00 | 34.02 | D000 | N |
| ATOM | 4000 | CA | TYR | C | 229 | -1.267 | -39.735 | 36.202 | 1.00 | 35.25 | D000 | C |
| ATOM | 4001 | C | TYR | C | 229 | -0.210 | -39.078 | 35.320 | 1.00 | 32.64 | D000 | C |
| ATOM | 4002 | O | TYR | C | 229 | -0.470 | -38.045 | 34.713 | 1.00 | 32.76 | D000 | O |
| ATOM | 4003 | CB | TYR | C | 229 | -0.967 | -39.448 | 37.676 | 1.00 | 34.94 | D000 | C |
| ATOM | 4004 | CG | TYR | C | 229 | -0.488 | -38.039 | 37.936 | 1.00 | 31.42 | D000 | C |
| ATOM | 4005 | CD1 | TYR | C | 229 | -1.381 | -36.975 | 37.988 | 1.00 | 28.67 | D000 | C |
| ATOM | 4006 | CD2 | TYR | C | 229 | 0.860 | -37.773 | 38.129 | 1.00 | 36.14 | D000 | C |
| ATOM | 4007 | CE1 | TYR | C | 229 | -0.941 | -35.684 | 38.220 | 1.00 | 24.57 | D000 | C |
| ATOM | 4008 | CE2 | TYR | C | 229 | 1.309 | -36.493 | 38.366 | 1.00 | 33.76 | D000 | C |
| ATOM | 4009 | CZ | TYR | C | 229 | 0.407 | -35.452 | 38.412 | 1.00 | 34.92 | D000 | C |
| ATOM | 4010 | OH | TYR | C | 229 | 0.869 | -34.176 | 38.646 | 1.00 | 35.23 | D000 | O |

(continued)

| | | | | | | | | | | | | |
|---|---|---|---|---|---|---|---|---|---|---|---|---|
| ATOM | 4011 | N | GLU | C | 230 | 0.973 | -39.679 | 35.244 | 1.00 | 32.24 | D000 | N |
| ATOM | 4012 | CA | GLU | C | 230 | 2.108 | -39.042 | 34.578 | 1.00 | 40.08 | D000 | C |
| ATOM | 4013 | C | GLU | C | 230 | 1.866 | -38.810 | 33.086 | 1.00 | 34.17 | D000 | C |
| ATOM | 4014 | O | GLU | C | 230 | 2.253 | -37.777 | 32.545 | 1.00 | 32.51 | D000 | O |
| ATOM | 4015 | CB | GLU | C | 230 | 3.381 | -39.869 | 34.774 | 1.00 | 40.46 | D000 | C |
| ATOM | 4016 | CG | GLU | C | 230 | 4.639 | -39.157 | 34.302 | 1.00 | 62.30 | D000 | C |
| ATOM | 4017 | CD | GLU | C | 230 | 5.913 | -39.799 | 34 . 821 | 1.00 | 100.35 | D000 | C |
| ATOM | 4018 | OE1 | GLU | C | 230 | 5.824 | -40.683 | 35.701 | 1.00 | 96.71 | D000 | O |
| ATOM | 4019 | OE2 | GLU | C | 230 | 7.006 | -39.416 | 34.349 | 1.00 | 104.16 | D000 | O |
| ATOM | 4020 | N | THR | C | 231 | 1.210 | -39.760 | 32.428 | 1.00 | 32.37 | D000 | N |
| ATOM | 4021 | CA | THR | C | 231 | 0.942 | -39.638 | 31.001 | 1.00 | 35.98 | D000 | C |
| ATOM | 4022 | C | THR | C | 231 | -0.426 | -39.018 | 30.742 | 1.00 | 40.00 | D000 | C |
| ATOM | 4023 | O | THR | C | 231 | -0.829 | -38.837 | 29.593 | 1.00 | 34.33 | D000 | O |
| ATOM | 4024 | CB | THR | C | 231 | 1.015 | -41.005 | 30.291 | 1.00 | 45.18 | D000 | C |
| ATOM | 4025 | CG2 | THR | C | 231 | 2.392 | -41.634 | 30.485 | 1.00 | 36.03 | D000 | C |
| ATOM | 4026 | OG1 | THR | C | 231 | 0.004 | -41.878 | 30.813 | 1.00 | 40.41 | D000 | O |
| ATOM | 4027 | N | GLY | C | 232 | -1.135 | -38.685 | 31.815 | 1.00 | 31.90 | D000 | N |
| ATOM | 4028 | CA | GLY | C | 232 | -2.478 | -38.155 | 31.689 | 1.00 | 35.88 | D000 | C |
| ATOM | 4029 | C | GLY | C | 232 | -2.556 | -36.641 | 31.641 | 1.00 | 31.67 | D000 | C |
| ATOM | 4030 | O | GLY | C | 232 | -1.569 | -35.940 | 31.855 | 1.00 | 34.20 | D000 | O |
| ATOM | 4031 | N | PHE | C | 233 | -3.754 | -36.148 | 31.351 | 1.00 | 34.66 | D000 | N |
| ATOM | 4032 | CA | PHE | C | 233 | -4.057 | -34.723 | 31.357 | 1.00 | 30.10 | D000 | C |
| ATOM | 4033 | C | PHE | C | 233 | -3.792 | -34.089 | 32.730 | 1.00 | 28.30 | D000 | C |
| ATOM | 4034 | O | PHE | C | 233 | -4.053 | -34.702 | 33.766 | 1.00 | 31.59 | D000 | O |
| ATOM | 4035 | CB | PHE | C | 233 | -5.520 | -34.520 | 30.932 | 1.00 | 29.82 | D000 | C |
| ATOM | 4036 | CG | PHE | C | 233 | -6.022 | -33.115 | 31.094 | 1.00 | 33.74 | D000 | C |
| ATOM | 4037 | CD1 | PHE | C | 233 | -5.829 | -32.175 | 30.088 | 1.00 | 27.88 | D000 | C |
| ATOM | 4038 | CD2 | PHE | C | 233 | -6.715 | -32.739 | 32.240 | 1.00 | 24.62 | D000 | C |
| ATOM | 4039 | CE1 | PHE | C | 233 | -6.298 | -30.878 | 30.231 | 1.00 | 27.56 | D000 | C |
| ATOM | 4040 | CE2 | PHE | C | 233 | -7.184 | -31.441 | 32.389 | 1.00 | 32.64 | D000 | C |
| ATOM | 4041 | CZ | PHE | C | 233 | -6.975 | -30.510 | 31.381 | 1.00 | 27.36 | D000 | C |
| ATOM | 4042 | N | LYS | C | 234 | -3.267 | -32.866 | 32.730 | 1.00 | 29.76 | D000 | N |
| ATOM | 4043 | CA | LYS | C | 234 | -3.045 | -32.109 | 33.963 | 1.00 | 29.48 | D000 | C |
| ATOM | 4044 | C | LYS | C | 234 | -3.455 | -30.646 | 33.789 | 1.00 | 31.02 | D000 | c |

(continued)

| | | | | | | | | | | | |
|---|---|---|---|---|---|---|---|---|---|---|---|
| ATOM | 4045 | O | LYS | C | 234 | -3.316 | -30.077 | 32.704 | 1.00 | 25.86 | D000 | O |
| ATOM | 4046 | CB | LYS | C | 234 | -1.579 | -32.189 | 34.397 | 1.00 | 32.09 | D000 | c |
| ATOM | 4047 | CG | LYS | C | 234 | -1.056 | -33.603 | 34.611 | 1.00 | 30.92 | D000 | c |
| ATOM | 4048 | CD | LYS | C | 234 | 0.422 | -33.598 | 34.955 | 1.00 | 34.48 | D000 | c |
| ATOM | 4049 | CE | LYS | C | 234 | 1.026 | -34.993 | 34 . 843 | 1.00 | 34.77 | D000 | C |
| ATOM | 4050 | NZ | LYS | C | 234 | 1.050 | -35.479 | 33.432 | 1.00 | 34.51 | D000 | N |
| ATOM | 4051 | N | ASN | C | 235 | -3.957 | -30.040 | 34.861 | 1.00 | 29.35 | D000 | N |
| ATOM | 4052 | CA | ASN | C | 235 | -4.382 | -28.644 | 34.822 | 1.00 | 26.46 | D000 | C |
| ATOM | 4053 | C | ASN | C | 235 | -4.012 | -27.912 | 36.116 | 1.00 | 26.48 | D000 | C |
| ATOM | 4054 | O | ASN | C | 235 | -4.814 | -27.157 | 36.659 | 1.00 | 29.79 | D000 | O |
| ATOM | 4055 | CB | ASN | C | 235 | -5.896 | -28.559 | 34.573 | 1.00 | 25.06 | D000 | C |
| ATOM | 4056 | CG | ASN | C | 235 | -6.345 | -27.173 | 34.127 | 1.00 | 27.67 | D000 | C |
| ATOM | 4057 | ND2 | ASN | C | 235 | -7.464 | -26.705 | 34.673 | 1.00 | 26.28 | D000 | N |
| ATOM | 4058 | OD1 | ASN | C | 235 | -5.692 | -26.533 | 33.308 | 1.00 | 27.02 | D000 | O |
| ATOM | 4059 | N | TRP | C | 236 | -2.795 | -28.145 | 36.607 | 1.00 | 27.50 | D000 | N |
| ATOM | 4060 | CA | TRP | C | 236 | -2.325 | -27.526 | 37.850 | 1.00 | 27.74 | D000 | C |
| ATOM | 4061 | C | TRP | C | 236 | -2.206 | -26.010 | 37.747 | 1.00 | 27.99 | D000 | C |
| ATOM | 4062 | O | TRP | C | 236 | -1.870 | -25.474 | 36.690 | 1.00 | 31.33 | D000 | O |
| ATOM | 4063 | CB | TRP | C | 236 | -0.960 | -28.092 | 38.264 | 1.00 | 23.47 | D000 | C |
| ATOM | 4064 | CG | TRP | C | 236 | -0.967 | -29.533 | 38.710 | 1.00 | 24.40 | D000 | C |
| ATOM | 4065 | CD1 | TRP | C | 236 | -0.544 | -30.616 | 37.990 | 1.00 | 25.63 | D000 | C |
| ATOM | 4066 | CD2 | TRP | C | 236 | -1.407 | -30.040 | 39.979 | 1.00 | 27.52 | D000 | C |
| ATOM | 4067 | CE2 | TRP | C | 236 | -1.224 | -31.442 | 39.955 | 1.00 | 25.85 | D000 | C |
| ATOM | 4068 | CE3 | TRP | C | 236 | -1.940 | -29.450 | 41.132 | 1.00 | 25.98 | D000 | C |
| ATOM | 4069 | NE1 | TRP | C | 236 | -0.696 | -31.766 | 38.732 | 1.00 | 26.26 | D000 | N |
| ATOM | 4070 | CZ2 | TRP | C | 236 | -1.551 | -32.258 | 41.037 | 1.00 | 25.53 | D000 | C |
| ATOM | 4071 | CZ3 | TRP | C | 236 | -2.267 | -30.263 | 42.208 | 1.00 | 25.29 | D000 | C |
| ATOM | 4072 | CH2 | TRP | C | 236 | -2.071 | -31.654 | 42.152 | 1.00 | 26.90 | D000 | C |
| ATOM | 4073 | N | ARG | C | 237 | -2.473 | -25.323 | 38.855 | 1.00 | 29.23 | D000 | N |
| ATOM | 4074 | CA | ARG | C | 237 | -2.100 | -23.918 | 38.983 | 1.00 | 34.44 | D000 | C |
| ATOM | 4075 | C | ARG | C | 237 | -0.586 | -23.790 | 38.820 | 1.00 | 34.81 | D000 | C |
| ATOM | 4076 | O | ARG | C | 237 | 0.147 | -24.755 | 39.052 | 1.00 | 28.62 | D000 | O |
| ATOM | 4077 | CB | ARG | C | 237 | -2.547 | -23.349 | 40.337 | 1.00 | 30.99 | D000 | C |
| ATOM | 4078 | CG | ARG | C | 237 | -4.048 | -23.189 | 40.474 | 1.00 | 37.60 | D000 | C |

(continued)

| | | | | | | | | | | | |
|---|---|---|---|---|---|---|---|---|---|---|---|
| ATOM | 4079 | CD | ARG | C | 237 | -4.455 | -21.737 | 40.591 | 1.00 | 42.14 | D000 | C |
| ATOM | 4080 | NE | ARG | C | 237 | -4.482 | -21.299 | 41.980 | 1.00 | 49.94 | D000 | N |
| ATOM | 4081 | CZ | ARG | C | 237 | -5.493 | -20.641 | 42.538 | 1.00 | 55.18 | D000 | C |
| ATOM | 4082 | NH1 | ARG | C | 237 | -6.568 | -20.334 | 41.822 | 1.00 | 41. 85 | D000 | N |
| ATOM | 4083 | NH2 | ARG | C | 237 | -5.428 | -20.287 | 43.814 | 1.00 | 55.93 | D000 | N |
| ATOM | 4084 | N | PRO | C | 238 | -0.113 | -22.606 | 38.401 | 1.00 | 36.47 | D000 | N |
| ATOM | 4085 | CA | PRO | C | 238 | 1.334 | -22.380 | 38.314 | 1.00 | 36.67 | D000 | C |
| ATOM | 4086 | C | PRO | C | 238 | 2.051 | -22.730 | 39.619 | 1.00 | 31.29 | D000 | C |
| ATOM | 4087 | O | PRO | C | 238 | 1.581 | -22.352 | 40.692 | 1.00 | 39.20 | D000 | O |
| ATOM | 4088 | CB | PRO | C | 238 | 1.435 | -20.881 | 38.015 | 1.00 | 41.44 | D000 | C |
| ATOM | 4089 | CG | PRO | C | 238 | 0.172 | -20.566 | 37.297 | 1.00 | 41.47 | D000 | C |
| ATOM | 4090 | CD | PRO | C | 238 | -0.886 | -21.461 | 37.885 | 1.00 | 34.59 | D000 | C |
| ATOM | 4091 | N | GLU | C | 239 | 3.149 | -23.473 | 39.502 | 1.00 | 34.14 | D000 | N |
| ATOM | 4092 | CA | GLU | C | 239 | 4.016 | -23.854 | 40.624 | 1.00 | 40.34 | D000 | C |
| ATOM | 4093 | C | GLU | C | 239 | 3.405 | -24.898 | 41.566 | 1.00 | 40.27 | D000 | C |
| ATOM | 4094 | O | GLU | C | 239 | 3.952 | -25.164 | 42.636 | 1.00 | 43.88 | D000 | O |
| ATOM | 4095 | CB | GLU | C | 239 | 4.428 | -22.615 | 41.426 | 1.00 | 43.42 | D000 | C |
| ATOM | 4096 | CG | GLU | C | 239 | 5.157 | -21.571 | 40.598 | 1.00 | 50.20 | D000 | C |
| ATOM | 4097 | CD | GLU | C | 239 | 5.859 | -20.539 | 41.453 | 1.00 | 75.90 | D000 | C |
| ATOM | 4098 | OE1 | GLU | C | 239 | 5.212 | -19.540 | 41.836 | 1.00 | 82.09 | D000 | O |
| ATOM | 4099 | OE2 | GLU | C | 239 | 7.059 | -20.732 | 41.744 | 1.00 | 84.44 | D000 | O |
| ATOM | 4100 | N | GLN | C | 240 | 2.285 | -25.493 | 41.165 | 1.00 | 33.28 | D000 | N |
| ATOM | 4101 | CA | GLN | C | 240 | 1.703 | -26.611 | 41.904 | 1.00 | 31.36 | D000 | C |
| ATOM | 4102 | C | GLN | C | 240 | 1.873 | -27.891 | 41.082 | 1.00 | 31.46 | D000 | C |
| ATOM | 4103 | O | GLN | C | 240 | 1.926 | -27.827 | 39.856 | 1.00 | 30.03 | D000 | O |
| ATOM | 4104 | CB | GLN | C | 240 | 0.224 | -26.354 | 42.208 | 1.00 | 28.17 | D000 | C |
| ATOM | 4105 | CG | GLN | C | 240 | -0.065 | -25.007 | 42.862 | 1.00 | 36.91 | D000 | C |
| ATOM | 4106 | CD | GLN | C | 240 | 0.799 | -24.737 | 44.083 | 1.00 | 42.84 | D000 | C |
| ATOM | 4107 | NE2 | GLN | C | 240 | 1.452 | -23.580 | 44.095 | 1.00 | 37.20 | D000 | N |
| ATOM | 4108 | OE1 | GLN | C | 240 | 0.885 | -25.559 | 45.001 | 1.00 | 41.85 | D000 | O |
| ATOM | 4109 | N | PRO | C | 241 | 1.968 | -29.060 | 41.746 | 1.00 | 31.89 | D000 | N |
| ATOM | 4110 | CA | PRO | C | 241 | 1.952 | -29.276 | 43.200 | 1.00 | 36.63 | D000 | C |
| ATOM | 4111 | C | PRO | C | 241 | 3.307 | -28.999 | 43.845 | 1.00 | 34.40 | D000 | C |
| ATOM | 4112 | O | PRO | C | 241 | 4.332 | -29.013 | 43.162 | 1.00 | 31.05 | D000 | O |

(continued)

| | | | | | | | | | | | | |
|---|---|---|---|---|---|---|---|---|---|---|---|---|
| ATOM | 4113 | CB | PRO | C | 241 | 1.583 | -30.757 | 43.326 | 1.00 | 30.98 | D000 | C |
| ATOM | 4114 | CG | PRO | C | 241 | 2.166 | -31.380 | 42.097 | 1.00 | 28.05 | D000 | C |
| ATOM | 4115 | CD | PRO | C | 241 | 2.042 | -30.334 | 41.004 | 1.00 | 28.29 | D000 | C |
| ATOM | 4116 | N | ASP | C | 242 | 3.306 | -28.762 | 45.151 | 1.00 | 31.20 | D000 | N |
| ATOM | 4117 | CA | ASP | C | 242 | 4.534 | -28.429 | 45.869 | 1.00 | 33.88 | D000 | C |
| ATOM | 4118 | C | ASP | C | 242 | 5.428 | -29.640 | 46.150 | 1.00 | 36.07 | D000 | C |
| ATOM | 4119 | O | ASP | C | 242 | 6.583 | -29.482 | 46.545 | 1.00 | 35.39 | D000 | O |
| ATOM | 4120 | CB | ASP | C | 242 | 4.189 | -27.712 | 47.176 | 1.00 | 30.91 | D000 | C |
| ATOM | 4121 | CG | ASP | C | 242 | 3.796 | -26.257 | 46.953 | 1.00 | 42.15 | D000 | C |
| ATOM | 4122 | OD2 | ASP | C | 242 | 2.709 | -25.848 | 47.409 | 1.00 | 41.70 | D000 | O |
| ATOM | 4123 | OD1 | ASP | C | 242 | 4.571 | -25.527 | 46.298 | 1.00 | 38.03 | D000 | O |
| ATOM | 4124 | N | ASP | C | 243 | 4.894 | -30.841 | 45.935 | 1.00 | 33.28 | D000 | N |
| ATOM | 4125 | CA | ASP | C | 243 | 5.659 | -32.080 | 46.098 | 1.00 | 33.76 | D000 | C |
| ATOM | 4126 | C | ASP | C | 243 | 5.140 | -33.135 | 45.123 | 1.00 | 36.43 | D000 | C |
| ATOM | 4127 | O | ASP | C | 243 | 4.000 | -33.045 | 44.662 | 1.00 | 31.00 | D000 | O |
| ATOM | 4128 | CB | ASP | C | 243 | 5.562 | -32.592 | 47.539 | 1.00 | 33.56 | D000 | C |
| ATOM | 4129 | CG | ASP | C | 243 | 6.805 | -33.360 | 47.980 | 1.00 | 45.01 | D000 | C |
| ATOM | 4130 | OD1 | ASP | C | 243 | 7.510 | -33.935 | 47.118 | 1.00 | 32.68 | D000 | O |
| ATOM | 4131 | OD2 | ASP | C | 243 | 7.073 | -33.389 | 49.201 | 1.00 | 41.68 | D000 | O |
| ATOM | 4132 | O | TRP | C | 244 | 4.635 | -37.408 | 43.978 | 1.00 | 29.40 | D000 | O |
| ATOM | 4133 | N | TRP | C | 244 | 5.959 | -34.135 | 44.811 | 1.00 | 26.76 | D000 | N |
| ATOM | 4134 | CA | TRP | C | 244 | 5.530 | -35.179 | 43.886 | 1.00 | 32.96 | D000 | C |
| ATOM | 4135 | C | TRP | C | 244 | 5.096 | -36.460 | 44.610 | 1.00 | 33.98 | D000 | C |
| ATOM | 4136 | CB | TRP | C | 244 | 6.635 | -35.486 | 42.872 | 1.00 | 22.49 | D000 | C |
| ATOM | 4137 | CG | TRP | C | 244 | 7.926 | -35.939 | 43.487 | 1.00 | 31.66 | D000 | C |
| ATOM | 4138 | CD1 | TRP | C | 244 | 8.985 | -35.153 | 43.851 | 1.00 | 35.24 | D000 | C |
| ATOM | 4139 | CD2 | TRP | C | 244 | 8.301 | -37.286 | 43.797 | 1.00 | 28.87 | D000 | C |
| ATOM | 4140 | NE1 | TRP | C | 244 | 9.992 | -35.930 | 44.374 | 1.00 | 39.02 | D000 | N |
| ATOM | 4141 | CE2 | TRP | C | 244 | 9.597 | -37.243 | 44.351 | 1.00 | 34.79 | D000 | C |
| ATOM | 4142 | CE3 | TRP | C | 244 | 7.666 | -38.526 | 43.661 | 1.00 | 27.95 | D000 | C |
| ATOM | 4143 | CZ2 | TRP | C | 244 | 10.267 | -38.390 | 44.770 | 1.00 | 32.14 | D000 | C |
| ATOM | 4144 | CZ3 | TRP | C | 244 | 8.333 | -39.662 | 44.076 | 1.00 | 30.86 | D000 | C |
| ATOM | 4145 | CH2 | TRP | C | 244 | 9.621 | -39.586 | 44.624 | 1.00 | 31.78 | D000 | C |
| ATOM | 4146 | O | TYR | C | 245 | 4.758 | -35.925 | 48.510 | 1.00 | 29.36 | D000 | O |

(continued)

| | | | | | | | | | | | |
|---|---|---|---|---|---|---|---|---|---|---|---|
| ATOM | 4147 | N | TYR | C | 245 | 5.250 | -36.491 | 45.930 | 1.00 | 32.02 | D000 | N |
| ATOM | 4148 | CA | TYR | C | 245 | 4.614 | -37.534 | 46.735 | 1.00 | 28.48 | D000 | C |
| ATOM | 4149 | C | TYR | C | 245 | 4.247 | -36.969 | 48.099 | 1.00 | 30.14 | D000 | C |
| ATOM | 4150 | CB | TYR | C | 245 | 5.502 | -38.778 | 46.871 | 1.00 | 25.38 | D000 | C |
| ATOM | 4151 | CG | TYR | C | 245 | 6.734 | -38.633 | 47.739 | 1.00 | 32.91 | D000 | C |
| ATOM | 4152 | CD2 | TYR | C | 245 | 6.785 | -39.200 | 49.009 | 1.00 | 31.25 | D000 | C |
| ATOM | 4153 | CD1 | TYR | C | 245 | 7.865 | -37.975 | 47.270 | 1.00 | 30.40 | D000 | C |
| ATOM | 4154 | CE2 | TYR | C | 245 | 7.920 | -39.093 | 49.795 | 1.00 | 28.79 | D000 | C |
| ATOM | 4155 | CE1 | TYR | C | 245 | 8.999 | -37.860 | 48.049 | 1.00 | 32.06 | D000 | C |
| ATOM | 4156 | CZ | TYR | C | 245 | 9.025 | -38.420 | 49.307 | 1.00 | 29.81 | D000 | C |
| ATOM | 4157 | OH | TYR | C | 245 | 10.159 | -38.300 | 50.077 | 1.00 | 27.57 | D000 | O |
| ATOM | 4158 | O | GLY | C | 246 | 4.628 | -37.473 | 51.323 | 1.00 | 34.84 | D000 | O |
| ATOM | 4159 | N | GLY | C | 246 | 3.340 | -37.650 | 48.789 | 1.00 | 31.19 | D000 | N |
| ATOM | 4160 | CA | GLY | C | 246 | 2.703 | -37.067 | 49.955 | 1.00 | 35.76 | D000 | C |
| ATOM | 4161 | C | GLY | C | 246 | 3.427 | -37.207 | 51.272 | 1.00 | 33.62 | D000 | C |
| ATOM | 4162 | O | HIS | C | 247 | 2.259 | -37.710 | 55.785 | 1.00 | 43.05 | D000 | O |
| ATOM | 4163 | N | HIS | C | 247 | 2.664 | -37.031 | 52.345 | 1.00 | 38.43 | D000 | N |
| ATOM | 4164 | CA | HIS | C | 247 | 3.202 | -36.998 | 53.694 | 1.00 | 39.05 | D000 | C |
| ATOM | 4165 | C | HIS | C | 247 | 2.411 | -37.945 | 54.588 | 1.00 | 38.77 | D000 | C |
| ATOM | 4166 | CB | HIS | C | 247 | 3.164 | -35.565 | 54.234 | 1.00 | 39.25 | D000 | C |
| ATOM | 4167 | CG | HIS | C | 247 | 3.692 | -34.553 | 53.266 | 1.00 | 36.64 | D000 | C |
| ATOM | 4168 | ND1 | HIS | C | 247 | 4.970 | -34.039 | 53.346 | 1.00 | 46.82 | D000 | N |
| ATOM | 4169 | CD2 | HIS | C | 247 | 3.126 | -33.978 | 52.179 | 1.00 | 35.16 | D000 | C |
| ATOM | 4170 | CE1 | HIS | C | 247 | 5.163 | -33.185 | 52.358 | 1.00 | 42.11 | D000 | C |
| ATOM | 4171 | NE2 | HIS | C | 247 | 4.059 | -33.130 | 51.632 | 1.00 | 47.04 | D000 | N |
| ATOM | 4172 | O | GLY | C | 248 | -0.747 | -38.509 | 54.445 | 1.00 | 40.24 | D000 | O |
| ATOM | 4173 | N | GLY | C | 248 | 1.903 | -39. 018 | 53.991 | 1.00 | 40.96 | D000 | N |
| ATOM | 4174 | CA | GLY | C | 248 | 1.118 | -39.995 | 54.720 | 1.00 | 37.66 | D000 | C |
| ATOM | 4175 | C | GLY | C | 248 | -0.330 | -39.578 | 54.902 | 1.00 | 47.53 | D000 | C |
| ATOM | 4176 | O | LEU | C | 249 | -3.773 | -38.603 | 57.140 | 1.00 | 54.25 | D000 | O |
| ATOM | 4177 | N | LEU | C | 249 | -1.097 | -40.433 | 55.574 | 1.00 | 52.35 | D000 | N |
| ATOM | 4178 | CA | LEU | C | 249 | -2.508 | -40.173 | 55.842 | 1.00 | 52.11 | D000 | C |
| ATOM | 4179 | C | LEU | C | 249 | -2.689 | -39.160 | 56.968 | 1.00 | 52.40 | D000 | C |
| ATOM | 4180 | CB | LEU | C | 249 | -3.233 | -41.474 | 56.202 | 1.00 | 42.33 | D000 | C |

(continued)

| | | | | | | | | | | | | |
|---|---|---|---|---|---|---|---|---|---|---|---|---|
| ATOM | 4181 | CG | LEU | C | 249 | -3.333 | -42.572 | 55.139 | 1.00 | 60.62 | D000 | C |
| ATOM | 4182 | CD2 | LEU | C | 249 | -4.287 | -42.165 | 54.027 | 1.00 | 56.35 | D000 | C |
| ATOM | 4183 | CD1 | LEU | C | 249 | -3.789 | -43.874 | 55.773 | 1.00 | 55.71 | D000 | C |
| ATOM | 4184 | O | GLY | c | 250 | -1.984 | -35.849 | 59.639 | 1.00 | 57.54 | D000 | O |
| ATOM | 4185 | N | GLY | C | 250 | -1.622 | -38.924 | 57.727 | 1.00 | 52.24 | D000 | N |
| ATOM | 4186 | CA | GLY | C | 250 | -1.698 | -38.112 | 58.930 | 1.00 | 50.41 | D000 | C |
| ATOM | 4187 | C | GLY | C | 250 | -1.663 | -36.608 | 58.725 | 1.00 | 57.93 | D000 | C |
| ATOM | 4188 | N | GLY | C | 251 | -1.269 | -36.168 | 57.535 | 1.00 | 52.64 | D000 | N |
| ATOM | 4189 | CA | GLY | C | 251 | -1.203 | -34.746 | 57.250 | 1.00 | 44.98 | D000 | C |
| ATOM | 4190 | C | GLY | C | 251 | -0.639 | -34.433 | 55.880 | 1.00 | 46.45 | D000 | C |
| ATOM | 4191 | O | GLY | C | 251 | -0.459 | -35.329 | 55.051 | 1.00 | 43.28 | D000 | O |
| ATOM | 4192 | N | GLY | C | 252 | -0.369 | -33.152 | 55.642 | 1.00 | 37.49 | D000 | N |
| ATOM | 4193 | CA | GLY | C | 252 | 0.205 | -32.710 | 54.387 | 1.00 | 32.69 | D000 | C |
| ATOM | 4194 | C | GLY | C | 252 | -0.831 | -32.272 | 53.366 | 1.00 | 34.76 | D000 | C |
| ATOM | 4195 | O | GLY | C | 252 | -2.005 | -32.646 | 53.445 | 1.00 | 30.54 | D000 | O |
| ATOM | 4196 | N | GLU | C | 253 | -0.385 | -31.472 | 52.402 | 1.00 | 33.99 | D000 | N |
| ATOM | 4197 | CA | GLU | C | 253 | -1.250 | -30.989 | 51.331 | 1.00 | 40.45 | D000 | C |
| ATOM | 4198 | C | GLU | C | 253 | -1.285 | -32.029 | 50.222 | 1.00 | 29.00 | D000 | C |
| ATOM | 4199 | O | GLU | C | 253 | -0.676 | -31.860 | 49.168 | 1.00 | 35.30 | D000 | O |
| ATOM | 4200 | CB | GLU | C | 253 | -0.757 | -29.632 | 50.821 | 1.00 | 34.32 | D000 | C |
| ATOM | 4201 | CG | GLU | C | 253 | -0.717 | -28.572 | 51.926 | 1.00 | 42.40 | D000 | C |
| ATOM | 4202 | CD | GLU | C | 253 | -0.225 | -27.217 | 51.444 | 1.00 | 58. 06 | D000 | C |
| ATOM | 4203 | OE1 | GLU | C | 253 | -0.620 | -26.792 | 50.337 | 1.00 | 59.58 | D000 | O |
| ATOM | 4204 | OE2 | GLU | C | 253 | 0.557 | -26.574 | 52.177 | 1.00 | 57.93 | D000 | O |
| ATOM | 4205 | N | ASP | C | 254 | -2.015 | -33.109 | 50.484 | 1.00 | 27.38 | D000 | N |
| ATOM | 4206 | CA | ASP | C | 254 | -1.921 | -34.327 | 49.693 | 1.00 | 31.18 | D000 | C |
| ATOM | 4207 | C | ASP | C | 254 | -3.140 | -34.599 | 48.805 | 1.00 | 33.58 | D000 | C |
| ATOM | 4208 | O | ASP | C | 254 | -3.216 | -35.646 | 48.158 | 1.00 | 27.45 | D000 | O |
| ATOM | 4209 | CB | ASP | C | 254 | -1.700 | -35.535 | 50.623 | 1.00 | 29.94 | D000 | C |
| ATOM | 4210 | CG | ASP | C | 254 | -0.346 | -35.504 | 51.328 | 1.00 | 36.48 | D000 | C |
| ATOM | 4211 | OD1 | ASP | C | 254 | 0.494 | -34.630 | 51.013 | 1.00 | 28.05 | D000 | O |
| ATOM | 4212 | OD2 | ASP | C | 254 | -0.117 | -36.374 | 52.196 | 1.00 | 35.72 | D000 | O |
| ATOM | 4213 | N | CYS | C | 255 | -4.090 | -33.670 | 48.768 | 1.00 | 26.75 | D000 | N |
| ATOM | 4214 | CA | CYS | C | 255 | -5.294 | -33.867 | 47.963 | 1.00 | 26.33 | D000 | C |

(continued)

| | | | | | | | | | | | |
|---|---|---|---|---|---|---|---|---|---|---|---|
| ATOM | 4215 | C | CYS | C | 255 | -5.518 | -32.727 | 46.977 | 1.00 | 32.54 | D000 | C |
| ATOM | 4216 | O | CYS | C | 255 | -5.323 | -31.559 | 47.316 | 1.00 | 27.66 | D000 | O |
| ATOM | 4217 | CB | CYS | C | 255 | -6.514 | -34.024 | 48.869 | 1.00 | 27.73 | D000 | C |
| ATOM | 4218 | SG | CYS | C | 255 | -6.407 | -35.447 | 49.969 | 1.00 | 35.91 | D000 | S |
| ATOM | 4219 | N | ALA | C | 256 | -5.930 | -33.072 | 45.758 | 1.00 | 28.09 | D000 | N |
| ATOM | 4220 | CA | ALA | C | 256 | -6.116 | -32.073 | 44.710 | 1.00 | 28.14 | D000 | C |
| ATOM | 4221 | C | ALA | C | 256 | -7.560 | -31.598 | 44.637 | 1.00 | 28.47 | D000 | C |
| ATOM | 4222 | O | ALA | C | 256 | -8.500 | -32.389 | 44.749 | 1.00 | 30.35 | D000 | O |
| ATOM | 4223 | CB | ALA | C | 256 | -5.678 | -32.627 | 43.357 | 1.00 | 25.70 | D000 | C |
| ATOM | 4224 | N | HIS | C | 257 | -7.732 | -30.298 | 44.444 | 1.00 | 28.56 | D000 | N |
| ATOM | 4225 | CA | HIS | C | 257 | -9.054 | -29.742 | 44.223 | 1.00 | 25.18 | D000 | C |
| ATOM | 4226 | C | HIS | C | 257 | -9.020 | -28.689 | 43.127 | 1.00 | 31.48 | D000 | C |
| ATOM | 4227 | O | HIS | C | 257 | -7.990 | -28.054 | 42.895 | 1.00 | 26.66 | D000 | O |
| ATOM | 4228 | CB | HIS | C | 257 | -9.615 | -29.139 | 45.514 | 1.00 | 24.94 | D000 | C |
| ATOM | 4229 | CG | HIS | C | 257 | -8.842 | -27.958 | 46.023 | 1.00 | 29.72 | D000 | C |
| ATOM | 4230 | CD2 | HIS | C | 257 | -7.685 | -27.880 | 46.715 | 1.00 | 26.19 | D000 | C |
| ATOM | 4231 | ND1 | HIS | C | 257 | -9.275 | -26.658 | 45.852 | 1.00 | 31.92 | D000 | N |
| ATOM | 4232 | CE1 | HIS | C | 257 | -8.411 | -25.834 | 46.415 | 1.00 | 33.33 | D000 | C |
| ATOM | 4233 | NE2 | HIS | C | 257 | -7.434 | -26.546 | 46.943 | 1.00 | 31.64 | D000 | N |
| ATOM | 4234 | N | PHE | C | 258 | -10.146 | -28.528 | 42.441 | 1.00 | 25.00 | D000 | N |
| ATOM | 4235 | CA | PHE | C | 258 | -10.340 | -27.385 | 41.567 | 1.00 | 28.97 | D000 | C |
| ATOM | 4236 | C | PHE | C | 258 | -10.347 | -26.129 | 42.421 | 1.00 | 30.92 | D000 | C |
| ATOM | 4237 | O | PHE | C | 258 | -10.917 | -26.128 | 43.511 | 1.00 | 27.46 | D000 | O |
| ATOM | 4238 | CB | PHE | C | 258 | -11.655 | -27.491 | 40.788 | 1.00 | 28.67 | D000 | C |
| ATOM | 4239 | CG | PHE | C | 258 | -11.790 | -28.752 | 39.975 | 1.00 | 28.57 | D000 | C |
| ATOM | 4240 | CD1 | PHE | C | 258 | -11.253 | -28. 831 | 38.699 | 1.00 | 24.14 | D000 | C |
| ATOM | 4241 | CD2 | PHE | C | 258 | -12.475 | -29.845 | 40.477 | 1.00 | 26.98 | D000 | C |
| ATOM | 4242 | CE1 | PHE | C | 258 | -11.385 | -29.982 | 37.944 | 1.00 | 27.83 | D000 | C |
| ATOM | 4243 | CE2 | PHE | C | 258 | -12.610 | -31.002 | 39.727 | 1.00 | 29.05 | D000 | C |
| ATOM | 4244 | CZ | PHE | C | 258 | -12.064 | -31.067 | 38.458 | 1.00 | 26.68 | D000 | C |
| ATOM | 4245 | N | THR | C | 259 | -9.709 | -25.068 | 41.935 | 1.00 | 31.52 | D000 | N |
| ATOM | 4246 | CA | THR | C | 259 | -9.778 | -23.766 | 42.596 | 1.00 | 27.35 | D000 | C |
| ATOM | 4247 | C | THR | C | 259 | -10.853 | -22.908 | 41.947 | 1.00 | 32.37 | D000 | C |
| ATOM | 4248 | O | THR | C | 259 | -11.547 | -23.361 | 41.038 | 1.00 | 28.98 | D000 | O |

(continued)

| | | | | | | | | | | | | |
|---|---|---|---|---|---|---|---|---|---|---|---|---|
| ATOM | 4249 | CB | THR | C | 259 | -8.450 | -23.013 | 42.517 | 1.00 | 30.50 | D000 | C |
| ATOM | 4250 | CG2 | THR | C | 259 | -7.336 | -23.832 | 43.146 | 1.00 | 29.47 | D000 | C |
| ATOM | 4251 | OG1 | THR | C | 259 | -8.141 | -22.759 | 41.139 | 1.00 | 26.60 | D000 | O |
| ATOM | 4252 | N | ASP | C | 260 | -10.966 | -21.657 | 42.383 | 1.00 | 33.47 | D000 | N |
| ATOM | 4253 | CA | ASP | C | 260 | -11.989 | -20.772 | 41.841 | 1.00 | 32.04 | D000 | C |
| ATOM | 4254 | C | ASP | C | 260 | -11.660 | -20.274 | 40.432 | 1.00 | 28.10 | D000 | C |
| ATOM | 4255 | O | ASP | C | 260 | -12.469 | -19.571 | 39.833 | 1.00 | 35.94 | D000 | O |
| ATOM | 4256 | CB | ASP | C | 260 | -12.232 | -19.574 | 42.774 | 1.00 | 31.62 | D000 | C |
| ATOM | 4257 | CG | ASP | C | 260 | -10.952 | -18.844 | 43.150 | 1.00 | 39.51 | D000 | C |
| ATOM | 4258 | OD1 | ASP | C | 260 | -9.894 | -19.114 | 42.544 | 1.00 | 46.04 | D000 | O |
| ATOM | 4259 | OD2 | ASP | C | 260 | -11.010 | -17.986 | 44.057 | 1.00 | 48.87 | D000 | O |
| ATOM | 4260 | N | ASP | C | 261 | -10.494 | -20.631 | 39.890 | 1.00 | 32.99 | D000 | N |
| ATOM | 4261 | CA | ASP | C | 261 | -10.219 | -20.308 | 38.483 | 1.00 | 33.63 | D000 | C |
| ATOM | 4262 | C | ASP | C | 261 | -10.169 | -21.568 | 37.618 | 1.00 | 34.02 | D000 | C |
| ATOM | 4263 | O | ASP | C | 261 | -9.808 | -21.517 | 36.440 | 1.00 | 35.61 | D000 | O |
| ATOM | 4264 | CB | ASP | C | 261 | -8.920 | -19.493 | 38.330 | 1.00 | 34.41 | D000 | C |
| ATOM | 4265 | CG | ASP | C | 261 | -7.663 | -20.257 | 38.749 | 1.00 | 41.30 | D000 | C |
| ATOM | 4266 | OD1 | ASP | C | 261 | -7.662 | -21.504 | 38.780 | 1.00 | 38.03 | D000 | O |
| ATOM | 4267 | OD2 | ASP | C | 261 | -6.644 | -19.591 | 39.031 | 1.00 | 47.65 | D000 | O |
| ATOM | 4268 | N | GLY | C | 262 | -10.525 | -22.701 | 38.214 | 1.00 | 34.67 | D000 | N |
| ATOM | 4269 | CA | GLY | C | 262 | -10.608 | -23.949 | 37.477 | 1.00 | 29.76 | D000 | C |
| ATOM | 4270 | C | GLY | C | 262 | -9.366 | -24.810 | 37.571 | 1.00 | 25.05 | D000 | C |
| ATOM | 4271 | O | GLY | C | 262 | -9.449 | -26.038 | 37.513 | 1.00 | 31.23 | D000 | O |
| ATOM | 4272 | N | ARG | C | 263 | -8.207 | -24.177 | 37.710 | 1.00 | 26.83 | D000 | N |
| ATOM | 4273 | CA | ARG | C | 263 | -6.952 | -24.919 | 37.778 | 1.00 | 26.39 | D000 | C |
| ATOM | 4274 | C | ARG | C | 263 | -6.771 | -25.578 | 39.148 | 1.00 | 30.19 | D000 | C |
| ATOM | 4275 | O | ARG | C | 263 | -7.392 | -25.172 | 40.135 | 1.00 | 28.43 | D000 | O |
| ATOM | 4276 | CB | ARG | C | 263 | -5.775 | -24.000 | 37.443 | 1.00 | 27.07 | D000 | C |
| ATOM | 4277 | CG | ARG | C | 263 | -5.652 | -23.711 | 35.943 | 1.00 | 29.33 | D000 | C |
| ATOM | 4278 | CD | ARG | C | 263 | -4.595 | -22.643 | 35.649 | 1.00 | 33.17 | D000 | C |
| ATOM | 4279 | NE | ARG | C | 263 | -5.071 | -21.305 | 35.982 | 1.00 | 44.51 | D000 | N |
| ATOM | 4280 | CZ | ARG | C | 263 | -5.787 | -20.547 | 35.156 | 1.00 | 52.96 | D000 | C |
| ATOM | 4281 | NH1 | ARG | C | 263 | -6.105 | -21.002 | 33.951 | 1.00 | 49.17 | D000 | N |
| ATOM | 4282 | NH2 | ARG | C | 263 | -6.188 | -19.340 | 35.532 | 1.00 | 51.57 | D000 | N |

(continued)

| | | | | | | | | | | | |
|---|---|---|---|---|---|---|---|---|---|---|---|
| ATOM | 4283 | N | TRP | C | 264 | -5.916 | -26.596 | 39.202 | 1.00 | 26.05 | D000 | N |
| ATOM | 4284 | CA | TRP | C | 264 | -5.847 | -27.477 | 40.365 | 1.00 | 28.26 | D000 | C |
| ATOM | 4285 | C | TRP | C | 264 | -4.859 | -27.000 | 41.415 | 1.00 | 27.92 | D000 | C |
| ATOM | 4286 | O | TRP | C | 264 | -3.844 | -26.385 | 41.097 | 1.00 | 28.53 | D000 | O |
| ATOM | 4287 | CB | TRP | C | 264 | -5.460 | -28.897 | 39.938 | 1.00 | 24.67 | D000 | C |
| ATOM | 4288 | CG | TRP | C | 264 | -6.268 | -29.456 | 38.814 | 1.00 | 25.08 | D000 | C |
| ATOM | 4289 | CD1 | TRP | C | 264 | -7.436 | -28.958 | 38.304 | 1.00 | 26.48 | D000 | C |
| ATOM | 4290 | CD2 | TRP | C | 264 | -5.963 | -30.627 | 38.048 | 1.00 | 26.55 | D000 | C |
| ATOM | 4291 | CE2 | TRP | C | 264 | -6.994 | -30.785 | 37.098 | 1.00 | 22.22 | D000 | C |
| ATOM | 4292 | CE3 | TRP | C | 264 | -4.923 | -31.565 | 38.081 | 1.00 | 25.16 | D000 | C |
| ATOM | 4293 | NE1 | TRP | C | 264 | -7.879 | -29.752 | 37.276 | 1.00 | 26.28 | D000 | N |
| ATOM | 4294 | CZ2 | TRP | C | 264 | -7.012 | -31.837 | 36.181 | 1.00 | 27.60 | D000 | C |
| ATOM | 4295 | CZ3 | TRP | C | 264 | -4.944 | -32.616 | 37.170 | 1.00 | 22.92 | D000 | C |
| ATOM | 4296 | CH2 | TRP | C | 264 | -5.979 | -32.739 | 36.232 | 1.00 | 24.94 | D000 | C |
| ATOM | 4297 | N | ASN | C | 265 | -5.155 | -27.313 | 42.670 | 1.00 | 30.65 | D000 | N |
| ATOM | 4298 | CA | ASN | C | 265 | -4.243 | -27.018 | 43.762 | 1.00 | 29.62 | D000 | C |
| ATOM | 4299 | C | ASN | C | 265 | -4.178 | -28.182 | 44.735 | 1.00 | 27.94 | D000 | C |
| ATOM | 4300 | O | ASN | C | 265 | -5.157 | -28.904 | 44.910 | 1.00 | 27.71 | D000 | O |
| ATOM | 4301 | CB | ASN | C | 265 | -4.668 | -25.746 | 44.490 | 1.00 | 31.63 | D000 | C |
| ATOM | 4302 | CG | ASN | C | 265 | -3.717 | -25.373 | 45.608 | 1.00 | 37.30 | D000 | C |
| ATOM | 4303 | ND2 | ASN | C | 265 | -4.220 | -25.357 | 46.839 | 1.00 | 35.10 | D000 | N |
| ATOM | 4304 | OD1 | ASN | C | 265 | -2.541 | -25.102 | 45.367 | 1.00 | 39.83 | D000 | O |
| ATOM | 4305 | N | ASP | C | 266 | -3.021 | -28.364 | 45.358 | 1.00 | 24.21 | D000 | N |
| ATOM | 4306 | CA | ASP | C | 266 | -2.850 | -29.405 | 46.366 | 1.00 | 28.45 | D000 | C |
| ATOM | 4307 | C | ASP | C | 266 | -3.066 | -28.832 | 47.763 | 1.00 | 36.19 | D000 | C |
| ATOM | 4308 | O | ASP | C | 266 | -2.397 | -27.880 | 48.157 | 1.00 | 35.56 | D000 | O |
| ATOM | 4309 | CB | ASP | C | 266 | -1.463 | -30.046 | 46.253 | 1.00 | 25.57 | D000 | C |
| ATOM | 4310 | CG | ASP | C | 266 | -0.349 | -29.020 | 46.030 | 1.00 | 36.15 | D000 | C |
| ATOM | 4311 | OD1 | ASP | C | 266 | -0.572 | -28.006 | 45.326 | 1.00 | 34.08 | D000 | O |
| ATOM | 4312 | OD2 | ASP | C | 266 | 0.762 | -29.236 | 46.554 | 1.00 | 38.58 | D000 | O |
| ATOM | 4313 | N | ASP | C | 267 | -4.007 | -29.412 | 48.507 | 1.00 | 32.84 | D000 | N |
| ATOM | 4314 | CA | ASP | C | 267 | -4.386 | -28.883 | 49 . 817 | 1.00 | 30.12 | D000 | C |
| ATOM | 4315 | C | ASP | C | 267 | -4.571 | -30.002 | 50.845 | 1.00 | 33.99 | D000 | C |
| ATOM | 4316 | O | ASP | C | 267 | -4.684 | -31.181 | 50.488 | 1.00 | 23.96 | D000 | O |

(continued)

| | | | | | | | | | | | | |
|---|---|---|---|---|---|---|---|---|---|---|---|---|
| ATOM | 4317 | CB | ASP | C | 267 | -5.675 | -28.058 | 49.701 | 1.00 | 33.91 | D000 | C |
| ATOM | 4318 | CG | ASP | C | 267 | -5.729 | -26.898 | 50.691 | 1.00 | 39.18 | D000 | C |
| ATOM | 4319 | OD1 | ASP | C | 267 | -4.872 | -26.833 | 51.601 | 1.00 | 39.63 | D000 | O |
| ATOM | 4320 | OD2 | ASP | C | 267 | -6.640 | -26.051 | 50.560 | 1.00 | 41.15 | D000 | O |
| ATOM | 4321 | N | VAL | C | 268 | -4.610 | -29.625 | 52.121 | 1.00 | 30.38 | D000 | N |
| ATOM | 4322 | CA | VAL | C | 268 | -4.808 | -30.587 | 53.202 | 1.00 | 34.77 | D000 | C |
| ATOM | 4323 | C | VAL | C | 268 | -6.169 | -31.260 | 53.054 | 1.00 | 31.59 | D000 | C |
| ATOM | 4324 | O | VAL | C | 268 | -7.157 | -30.630 | 52.685 | 1.00 | 30.27 | D000 | O |
| ATOM | 4325 | CB | VAL | C | 268 | -4.683 | -29.923 | 54.596 | 1.00 | 36.31 | D000 | C |
| ATOM | 4326 | CG1 | VAL | C | 268 | -3.302 | -29.294 | 54.757 | 1.00 | 31.26 | D000 | C |
| ATOM | 4327 | CG2 | VAL | C | 268 | -5.770 | -28.881 | 54.801 | 1.00 | 33.82 | D000 | C |
| ATOM | 4328 | N | CYS | C | 269 | -6.209 | -32.555 | 53.334 | 1.00 | 32.86 | D000 | N |
| ATOM | 4329 | CA | CYS | C | 269 | -7.369 | -33.364 | 52.991 | 1.00 | 31.61 | D000 | C |
| ATOM | 4330 | C | CYS | C | 269 | -8.559 | -33.098 | 53.917 | 1.00 | 36.95 | D000 | C |
| ATOM | 4331 | O | CYS | C | 269 | -9.684 | -33.504 | 53.627 | 1.00 | 36.14 | D000 | O |
| ATOM | 4332 | CB | CYS | C | 269 | -6.984 | -34.839 | 53.009 | 1.00 | 44.62 | D000 | C |
| ATOM | 4333 | SG | CYS | C | 269 | -5.637 | -35.218 | 51.830 | 1.00 | 60.76 | D000 | S |
| ATOM | 4334 | N | GLN | C | 270 | -8.305 | -32.384 | 55.009 | 1.00 | 31.38 | D000 | N |
| ATOM | 4335 | CA | GLN | C | 270 | -9.324 | -32.047 | 55.992 | 1.00 | 34.32 | D000 | C |
| ATOM | 4336 | C | GLN | C | 270 | -10.328 | -30.995 | 55.495 | 1.00 | 35.28 | D000 | C |
| ATOM | 4337 | O | GLN | C | 270 | -11.434 | -30.895 | 56.022 | 1.00 | 38.13 | D000 | O |
| ATOM | 4338 | CB | GLN | C | 270 | -8.640 | -31.552 | 57.265 | 1.00 | 41.74 | D000 | C |
| ATOM | 4339 | CG | GLN | C | 270 | -9.526 | -31.531 | 58.491 | 1.00 | 57.53 | D000 | C |
| ATOM | 4340 | CD | GLN | C | 270 | -8.755 | -31.148 | 59.743 | 1.00 | 62.03 | D000 | C |
| ATOM | 4341 | NE2 | GLN | C | 270 | -9.057 | -29.975 | 60.280 | 1.00 | 54.32 | D000 | N |
| ATOM | 4342 | OE1 | GLN | C | 270 | -7.898 | -31.898 | 60.222 | 1.00 | 60.58 | D000 | O |
| ATOM | 4343 | N | ARG | C | 271 | -9.940 | -30.207 | 54.495 | 1.00 | 30.92 | D000 | N |
| ATOM | 4344 | CA | ARG | C | 271 | -10.790 | -29.124 | 53.996 | 1.00 | 30.72 | D000 | C |
| ATOM | 4345 | C | ARG | C | 271 | -12.159 | -29.633 | 53.533 | 1.00 | 38. 04 | D000 | C |
| ATOM | 4346 | O | ARG | C | 271 | -12.246 | -30.543 | 52.709 | 1.00 | 31.45 | D000 | O |
| ATOM | 4347 | CB | ARG | C | 271 | -10.096 | -28.385 | 52.849 | 1.00 | 34.39 | D000 | C |
| ATOM | 4348 | CG | ARG | C | 271 | -8.799 | -27.676 | 53.234 | 1.00 | 35.61 | D000 | C |
| ATOM | 4349 | CD | ARG | C | 271 | -9.066 | -26.393 | 53.999 | 1.00 | 43.95 | D000 | C |
| ATOM | 4350 | NE | ARG | C | 271 | -7.837 | -25.661 | 54.292 | 1.00 | 42.12 | D000 | N |

(continued)

| | | | | | | | | | | | | |
|---|---|---|---|---|---|---|---|---|---|---|---|---|
| ATOM | 4351 | CZ | ARG | C | 271 | -7.169 | -25.750 | 55.438 | 1.00 | 45.47 | D000 | C |
| ATOM | 4352 | NH1 | ARG | C | 271 | -7.606 | -26.547 | 56.406 | 1.00 | 42.46 | D000 | N |
| ATOM | 4353 | NH2 | ARG | C | 271 | -6.059 | -25.048 | 55.614 | 1.00 | 47.94 | D000 | N |
| ATOM | 4354 | N | PRO | C | 272 | -13.237 | -29.045 | 54.074 | 1.00 | 35.02 | D000 | N |
| ATOM | 4355 | CA | PRO | C | 272 | -14.604 | -29.446 | 53.724 | 1.00 | 34.48 | D000 | C |
| ATOM | 4356 | C | PRO | C | 272 | -15.064 | -28.856 | 52.392 | 1.00 | 34.36 | D000 | C |
| ATOM | 4357 | O | PRO | C | 272 | -16.036 | -28.104 | 52.355 | 1.00 | 37.58 | D000 | O |
| ATOM | 4358 | CB | PRO | C | 272 | -15.433 | -28.887 | 54 .881 | 1.00 | 36.83 | D000 | C |
| ATOM | 4359 | CG | PRO | C | 272 | -14.678 | -27.668 | 55.306 | 1.00 | 32.07 | D000 | C |
| ATOM | 4360 | CD | PRO | C | 272 | -13.217 | -28.007 | 55.121 | 1.00 | 35.31 | D000 | C |
| ATOM | 4361 | N | TYR | C | 273 | -14.370 | -29.197 | 51.312 | 1.00 | 34.00 | D000 | N |
| ATOM | 4362 | CA | TYR | C | 273 | -14.725 | -28.690 | 49.994 | 1.00 | 34.47 | D000 | C |
| ATOM | 4363 | C | TYR | C | 273 | -15.886 | -29.467 | 49.399 | 1.00 | 31. 88 | D000 | C |
| ATOM | 4364 | O | TYR | C | 273 | -16.205 | -30.571 | 49.848 | 1.00 | 34.84 | D000 | O |
| ATOM | 4365 | CB | TYR | C | 273 | -13.523 | -28.760 | 49.048 | 1.00 | 33.39 | D000 | C |
| ATOM | 4366 | CG | TYR | C | 273 | -12.391 | -27.827 | 49.412 | 1.00 | 29.84 | D000 | C |
| ATOM | 4367 | CD1 | TYR | C | 273 | -12.634 | -26.642 | 50.096 | 1.00 | 31.84 | D000 | C |
| ATOM | 4368 | CD2 | TYR | C | 273 | -11.077 | -28.131 | 49.065 | 1.00 | 27.54 | D000 | C |
| ATOM | 4369 | CE1 | TYR | C | 273 | -11.595 | -25.778 | 50.431 | 1.00 | 30.36 | D000 | C |
| ATOM | 4370 | CE2 | TYR | C | 273 | -10.036 | -27.280 | 49.391 | 1.00 | 31.31 | D000 | C |
| ATOM | 4371 | CZ | TYR | C | 273 | -10.303 | -26.104 | 50.075 | 1.00 | 31.94 | D000 | C |
| ATOM | 4372 | OH | TYR | C | 273 | -9.273 | -25.261 | 50.404 | 1.00 | 32.60 | D000 | O |
| ATOM | 4373 | N | ARG | C | 274 | -16.510 | -28.891 | 48.377 | 1.00 | 29.76 | D000 | N |
| ATOM | 4374 | CA | ARG | C | 274 | -17.470 | -29.627 | 47.566 | 1.00 | 28.81 | D000 | C |
| ATOM | 4375 | C | ARG | C | 274 | -16.750 | -30.733 | 46.802 | 1.00 | 30.11 | D000 | C |
| ATOM | 4376 | O | ARG | C | 274 | -15.520 | -30.772 | 46.766 | 1.00 | 28.45 | D000 | O |
| ATOM | 4377 | CB | ARG | C | 274 | -18.191 | -28.689 | 46.601 | 1.00 | 31.26 | D000 | C |
| ATOM | 4378 | CG | ARG | C | 274 | -19.110 | -27.703 | 47.298 | 1.00 | 35.55 | D000 | C |
| ATOM | 4379 | CD | ARG | C | 274 | -19.871 | -26. 844 | 46.309 | 1.00 | 33.61 | D000 | C |
| ATOM | 4380 | NE | ARG | C | 274 | -20.885 | -26.046 | 46.984 | 1.00 | 39.40 | D000 | N |
| ATOM | 4381 | CZ | ARG | C | 274 | -21.630 | -25.121 | 46.389 | 1.00 | 48.65 | D000 | C |
| ATOM | 4382 | NH1 | ARG | C | 274 | -21.474 | -24.867 | 45.097 | 1.00 | 48.98 | D000 | N |
| ATOM | 4383 | NH2 | ARG | C | 274 | -22.531 | -24.446 | 47.090 | 1.00 | 55.24 | D000 | N |
| ATOM | 4384 | N | TRP | C | 275 | -17.507 | -31.639 | 46.198 | 1.00 | 28.06 | D000 | N |

(continued)

| | | | | | | | | | | | | |
|---|---|---|---|---|---|---|---|---|---|---|---|---|
| ATOM | 4385 | CA | TRP | C | 275 | -16.887 | -32.709 | 45.432 | 1.00 | 28.84 | D000 | C |
| ATOM | 4386 | C | TRP | C | 275 | -17.754 | -33.114 | 44.252 | 1.00 | 29.72 | D000 | C |
| ATOM | 4387 | O | TRP | C | 275 | -18.905 | -32.693 | 44.130 | 1.00 | 26.78 | D000 | O |
| ATOM | 4388 | CB | TRP | C | 275 | -16.601 | -33.927 | 46.322 | 1.00 | 29.81 | D000 | C |
| ATOM | 4389 | CG | TRP | C | 275 | -17 . 824 | -34.715 | 46.717 | 1.00 | 31.96 | D000 | C |
| ATOM | 4390 | CD1 | TRP | C | 275 | -18.293 | -35.855 | 46.125 | 1.00 | 31.44 | D000 | C |
| ATOM | 4391 | CD2 | TRP | C | 275 | -18.726 | -34.423 | 47.795 | 1.00 | 29.40 | D000 | C |
| ATOM | 4392 | CE2 | TRP | C | 275 | -19.718 | -35.426 | 47.793 | 1.00 | 31.93 | D000 | C |
| ATOM | 4393 | CE3 | TRP | C | 275 | -18.791 | -33.412 | 48.759 | 1.00 | 32.50 | D000 | C |
| ATOM | 4394 | NE1 | TRP | C | 275 | -19.430 | -36.288 | 46.767 | 1.00 | 36.68 | D000 | N |
| ATOM | 4395 | CZ2 | TRP | C | 275 | -20.761 | -35.447 | 48.718 | 1.00 | 33.08 | D000 | C |
| ATOM | 4396 | CZ3 | TRP | C | 275 | -19.829 | -33.433 | 49.676 | 1.00 | 33.96 | D000 | C |
| ATOM | 4397 | CH2 | TRP | C | 275 | -20.801 | -34.443 | 49.648 | 1.00 | 35.93 | D000 | C |
| ATOM | 4398 | N | VAL | C | 276 | -17.178 | -33.926 | 43.376 | 1.00 | 28.10 | D000 | N |
| ATOM | 4399 | CA | VAL | C | 276 | -17.870 | -34.412 | 42.195 | 1.00 | 29.03 | D000 | C |
| ATOM | 4400 | C | VAL | C | 276 | -17.737 | -35.924 | 42.114 | 1.00 | 27.19 | D000 | C |
| ATOM | 4401 | O | VAL | C | 276 | -16.628 | -36.454 | 42.177 | 1.00 | 28.31 | D000 | O |
| ATOM | 4402 | CB | VAL | C | 276 | -17.302 | -33.780 | 40.905 | 1.00 | 28.00 | D000 | C |
| ATOM | 4403 | CG1 | VAL | C | 276 | -18.046 | -34.293 | 39.688 | 1.00 | 27.41 | D000 | C |
| ATOM | 4404 | CG2 | VAL | C | 276 | -17.364 | -32.259 | 40.982 | 1.00 | 28.48 | D000 | C |
| ATOM | 4405 | N | CYS | C | 277 | -18.862 | -36.619 | 41.988 | 1.00 | 26.93 | D000 | N |
| ATOM | 4406 | CA | CYS | C | 277 | -18.832 | -38.058 | 41.751 | 1.00 | 33.14 | D000 | C |
| ATOM | 4407 | C | CYS | C | 277 | -18.994 | -38.338 | 40.262 | 1.00 | 37.44 | D000 | C |
| ATOM | 4408 | O | CYS | C | 277 | -19.699 | -37.612 | 39.564 | 1.00 | 33.29 | D000 | O |
| ATOM | 4409 | CB | CYS | C | 277 | -19.922 | -38.770 | 42.562 | 1.00 | 32.89 | D000 | C |
| ATOM | 4410 | SG | CYS | C | 277 | -19.660 | -38.700 | 44.364 | 1.00 | 47.93 | D000 | S |
| ATOM | 4411 | N | GLU | C | 278 | -18.324 | -39.386 | 39.787 | 1.00 | 30.10 | D000 | N |
| ATOM | 4412 | CA | GLU | C | 278 | -18.367 | -39.774 | 38.386 | 1.00 | 28.53 | D000 | C |
| ATOM | 4413 | C | GLU | C | 278 | -18.528 | -41.288 | 38.252 | 1.00 | 32.73 | D000 | C |
| ATOM | 4414 | O | GLU | C | 278 | -17.931 | -42.050 | 39.008 | 1.00 | 29.46 | D000 | O |
| ATOM | 4415 | CB | GLU | C | 278 | -17.095 | -39.315 | 37.655 | 1.00 | 32.72 | D000 | C |
| ATOM | 4416 | CG | GLU | C | 278 | -16.913 | -39.932 | 36.260 | 1.00 | 32.26 | D000 | C |
| ATOM | 4417 | CD | GLU | C | 278 | -15.636 | -39.483 | 35.555 | 1.00 | 39.23 | D000 | C |
| ATOM | 4418 | OE1 | GLU | C | 278 | -14.646 | -39.151 | 36.241 | 1.00 | 36.80 | D000 | O |

(continued)

| | | | | | | | | | | | | |
|---|---|---|---|---|---|---|---|---|---|---|---|---|
| ATOM | 4419 | OE2 | GLU | C | 278 | -15.622 | -39.463 | 34.303 | 1.00 | 40.68 | D000 | O |
| ATOM | 4420 | N | THR | C | 279 | -19.346 | -41.717 | 37.297 | 1.00 | 27.04 | D000 | N |
| ATOM | 4421 | CA | THR | C | 279 | -19.453 | -43.132 | 36.963 | 1.00 | 34.21 | D000 | C |
| ATOM | 4422 | C | THR | C | 279 | -19.688 | -43.285 | 35.460 | 1.00 | 36.69 | D000 | C |
| ATOM | 4423 | O | THR | C | 279 | -19.848 | -42.296 | 34.746 | 1.00 | 39.25 | D000 | O |
| ATOM | 4424 | CB | THR | C | 279 | -20.586 | -43.826 | 37.758 | 1.00 | 39.26 | D000 | C |
| ATOM | 4425 | CG2 | THR | C | 279 | -21.950 | -43.311 | 37.326 | 1.00 | 34.73 | D000 | C |
| ATOM | 4426 | OG1 | THR | C | 279 | -20.522 | -45.244 | 37.554 | 1.00 | 42.37 | D000 | O |
| ATOM | 4427 | N | GLU | C | 280 | -19.704 | -44.522 | 34.980 | 1.00 | 32.85 | D000 | N |
| ATOM | 4428 | CA | GLU | C | 280 | -19.835 | -44.782 | 33.548 | 1.00 | 50.68 | D000 | C |
| ATOM | 4429 | C | GLU | C | 280 | -21.239 | -45.225 | 33.156 | 1.00 | 52.22 | D000 | C |
| ATOM | 4430 | O | GLU | C | 280 | -22.086 | -45.481 | 34.011 | 1.00 | 55.73 | D000 | O |
| ATOM | 4431 | CB | GLU | C | 280 | -18.829 | -45.846 | 33.118 | 1.00 | 52.68 | D000 | C |
| ATOM | 4432 | CG | GLU | C | 280 | -17.416 | -45.583 | 33.593 | 1.00 | 64.50 | D000 | C |
| ATOM | 4433 | CD | GLU | C | 280 | -16.653 | -46.863 | 33.862 | 1.00 | 89.69 | D000 | C |
| ATOM | 4434 | OE1 | GLU | C | 280 | -17.283 | -47.943 | 33.861 | 1.00 | 93.00 | D000 | O |
| ATOM | 4435 | OE2 | GLU | C | 280 | -15.424 | -46.790 | 34.078 | 1.00 | 104.15 | D000 | O |
| ATOM | 4436 | O | LEU | C | 281 | -22.641 | -47.686 | 29.761 | 1.00 | 76.02 | D000 | O |
| ATOM | 4437 | N | LEU | C | 281 | -21.477 | -45.319 | 31.852 | 1.00 | 63.81 | D000 | N |
| ATOM | 4438 | CA | LEU | C | 281 | -22.728 | -45.870 | 31.340 | 1.00 | 65.22 | D000 | C |
| ATOM | 4439 | C | LEU | C | 281 | -22.565 | -47.338 | 30.941 | 1.00 | 72.08 | D000 | C |
| ATOM | 4440 | CB | LEU | C | 281 | -23.228 | -45.044 | 30.158 | 1.00 | 59.22 | D000 | C |
| ATOM | 4441 | CG | LEU | C | 281 | -23.879 | -43.727 | 30.578 | 1.00 | 63.11 | D000 | C |
| ATOM | 4442 | CD1 | LEU | C | 281 | -24.279 | -42.891 | 29.370 | 1.00 | 48.41 | D000 | C |
| ATOM | 4443 | CD2 | LEU | C | 281 | -25.083 | -44.021 | 31.458 | 1.00 | 67.92 | D000 | C |
| ATOM | 4444 | O | ASP | C | 282 | -20.760 | -51.176 | 30.617 | 1.00 | 50.93 | D000 | O |
| ATOM | 4445 | N | ASP | C | 282 | -22.342 | -48.183 | 31.947 | 1.00 | 77.43 | D000 | N |
| ATOM | 4446 | CA | ASP | C | 282 | -22.184 | -49.629 | 31.778 | 1.00 | 80.26 | D000 | C |
| ATOM | 4447 | C | ASP | C | 282 | -21.144 | -50.009 | 30.726 | 1.00 | 71.14 | D000 | C |
| ATOM | 4448 | CB | ASP | C | 282 | -23.529 | -50.272 | 31.433 | 1.00 | 81.46 | D000 | C |
| ATOM | 4449 | CG | ASP | C | 282 | -24.448 | -50.366 | 32.632 | 1.00 | 80.75 | D000 | C |
| ATOM | 4450 | OD1 | ASP | C | 282 | -24.417 | -49.445 | 33.476 | 1.00 | 77.18 | D000 | O |
| ATOM | 4451 | OD2 | ASP | C | 282 | -25.192 | -51.364 | 32.736 | 1.00 | 78.02 | D000 | O |
| TER | | | | | | | | | | | | |

## Table 10.3

| | | | | | | | | | | | |
|---|---|---|---|---|---|---|---|---|---|---|---|
| ATOM | 1 | N | GLN | C | 1 | 12.778 | 87.875 | 6.343 | 1.00 | 63.46 | N |
| ATOM | 2 | CA | GLN | C | 1 | 12.935 | 86.437 | 6.168 | 1.00 | 57.97 | C |
| ATOM | 3 | C | GLN | C | 1 | 11.798 | 85.716 | 6.845 | 1.00 | 55.78 | C |
| ATOM | 4 | O | GLN | C | 1 | 11.088 | 86.308 | 7.656 | 1.00 | 58.48 | O |
| ATOM | 5 | CB | GLN | C | 1 | 14.271 | 85.946 | 6.714 | 1.00 | 51.88 | C |
| ATOM | 6 | CG | GLN | C | 1 | 14.814 | 84.739 | 5.974 | 1.00 | 59.79 | C |
| ATOM | 7 | CD | GLN | C | 1 | 14.717 | 84.869 | 4.445 | 1.00 | 78.61 | C |
| ATOM | 8 | OE1 | GLN | C | 1 | 14.676 | 85.980 | 3.896 | 1.00 | 61.52 | O |
| ATOM | 9 | NE2 | GLN | C | 1 | 14.669 | 83.721 | 3.753 | 1.00 | 84.10 | N |
| ATOM | 10 | N | VAL | C | 2 | 11.615 | 84.445 | 6.497 | 1.00 | 50.14 | N |
| ATOM | 11 | CA | VAL | C | 2 | 10.404 | 83.710 | 6.828 | 1.00 | 46.16 | C |
| ATOM | 12 | C | VAL | C | 2 | 10.800 | 82.363 | 7.396 | 1.00 | 45.45 | C |
| ATOM | 13 | O | VAL | C | 2 | 11.642 | 81.667 | 6.822 | 1.00 | 49.37 | O |
| ATOM | 14 | CB | VAL | C | 2 | 9.493 | 83.521 | 5.596 | 1.00 | 45.22 | C |
| ATOM | 15 | CG1 | VAL | C | 2 | 8.322 | 82.567 | 5.911 | 1.00 | 45.47 | C |
| ATOM | 16 | CG2 | VAL | C | 2 | 8.965 | 84.848 | 5.109 | 1.00 | 40.64 | C |
| ATOM | 17 | N | GLN | C | 3 | 10.163 | 81.983 | 8.498 | 1.00 | 50.52 | N |
| ATOM | 18 | CA | GLN | C | 3 | 10.262 | 80.648 | 9.066 | 1.00 | 49.86 | C |
| ATOM | 19 | C | GLN | C | 3 | 8.856 | 80.074 | 9.158 | 1.00 | 45.53 | C |
| ATOM | 20 | O | GLN | C | 3 | 7.924 | 80.766 | 9.596 | 1.00 | 40.46 | O |
| ATOM | 21 | CB | GLN | C | 3 | 10.933 | 80.674 | 10.449 | 1.00 | 52.58 | C |
| ATOM | 22 | CG | GLN | C | 3 | 12.476 | 80.689 | 10.448 | 1.00 | 67.48 | C |
| ATOM | 23 | CD | GLN | C | 3 | 13.103 | 82.051 | 10.135 | 1.00 | 74.06 | C |
| ATOM | 24 | OE1 | GLN | C | 3 | 12.444 | 83.102 | 10.186 | 1.00 | 75.22 | O |
| ATOM | 25 | NE2 | GLN | C | 3 | 14.394 | 82.033 | 9.811 | 1.00 | 80.56 | N |
| ATOM | 26 | N | LEU | C | 4 | 8.702 | 78.828 | 8.714 | 1.00 | 36.58 | N |
| ATOM | 27 | CA | LEU | C | 4 | 7.451 | 78.093 | 8.811 | 1.00 | 35.24 | C |
| ATOM | 28 | C | LEU | C | 4 | 7.674 | 76.836 | 9.634 | 1.00 | 35.32 | C |
| ATOM | 29 | O | LEU | C | 4 | 8.563 | 76.043 | 9.319 | 1.00 | 34.10 | O |
| ATOM | 30 | CB | LEU | C | 4 | 6.925 | 77.728 | 7.417 | 1.00 | 35.59 | C |
| ATOM | 31 | CG | LEU | C | 4 | 6.747 | 78.896 | 6.452 | 1.00 | 38.97 | C |
| ATOM | 32 | CD1 | LEU | C | 4 | 6.422 | 78.423 | 5.045 | 1.00 | 39.74 | C |
| ATOM | 33 | CD2 | LEU | C | 4 | 5.653 | 79.806 | 6.970 | 1.00 | 42.54 | C |
| ATOM | 34 | N | VAL | C | 5 | 6.835 | 76.623 | 10.647 | 1.00 | 40.31 | N |
| ATOM | 35 | CA | VAL | C | 5 | 6.970 | 75.492 | 11.568 | 1.00 | 38.30 | C |
| ATOM | 36 | C | VAL | C | 5 | 5.625 | 74.776 | 11.675 | 1.00 | 41.55 | C |
| ATOM | 37 | O | VAL | C | 5 | 4.663 | 75.325 | 12.232 | 1.00 | 47.07 | O |
| ATOM | 38 | CB | VAL | C | 5 | 7.451 | 75.939 | 12.956 | 1.00 | 35.88 | C |
| ATOM | 39 | CG1 | VAL | C | 5 | 7.568 | 74.743 | 13.871 | 1.00 | 36.28 | C |
| ATOM | 40 | CG2 | VAL | C | 5 | 8.763 | 76.660 | 12.847 | 1.00 | 26.29 | C |
| ATOM | 41 | N | GLU | C | 6 | 5.558 | 73.556 | 11.169 | 1.00 | 42.37 | N |
| ATOM | 42 | CA | GLU | C | 6 | 4.354 | 72.741 | 11.241 | 1.00 | 43.70 | C |
| ATOM | 43 | C | GLU | C | 6 | 4.281 | 71.970 | 12.559 | 1.00 | 43.98 | C |
| ATOM | 44 | O | GLU | C | 6 | 5.290 | 71.677 | 13.196 | 1.00 | 49.49 | O |
| ATOM | 45 | CB | GLU | C | 6 | 4.290 | 71.749 | 10.081 | 1.00 | 44.93 | C |
| ATOM | 46 | CG | GLU | C | 6 | 4.481 | 72.363 | 8.699 | 1.00 | 44.29 | C |
| ATOM | 47 | CD | GLU | C | 6 | 5.949 | 72.419 | 8.267 | 1.00 | 44.27 | C |
| ATOM | 48 | OE1 | GLU | C | 6 | 6.215 | 72.317 | 7.041 | 1.00 | 42.60 | O |
| ATOM | 49 | OE2 | GLU | C | 6 | 6.832 | 72.490 | 9.153 | 1.00 | 47.85 | O1- |
| ATOM | 50 | N | SER | C | 7 | 3.061 | 71.619 | 12.950 | 1.00 | 48.19 | N |
| ATOM | 51 | CA | SER | C | 7 | 2.851 | 70.788 | 14.128 | 1.00 | 51.74 | C |
| ATOM | 52 | C | SER | C | 7 | 1.509 | 70.084 | 13.994 | 1.00 | 45.51 | C |
| ATOM | 53 | O | SER | C | 7 | 0.681 | 70.437 | 13.151 | 1.00 | 43.94 | O |
| ATOM | 54 | CB | SER | C | 7 | 2.904 | 71.620 | 15.410 | 1.00 | 38.38 | C |
| ATOM | 55 | OG | SER | C | 7 | 1.907 | 72.623 | 15.354 | 1.00 | 49.56 | O |
| ATOM | 56 | N | GLY | C | 8 | 1.310 | 69.067 | 14.829 | 1.00 | 48.93 | N |
| ATOM | 57 | CA | GLY | C | 8 | 0.030 | 68.405 | 14.930 | 1.00 | 34.09 | C |
| ATOM | 58 | C | GLY | C | 8 | -0.065 | 67.069 | 14.246 | 1.00 | 43.11 | C |
| ATOM | 59 | O | GLY | C | 8 | -1.148 | 66.466 | 14.255 | 1.00 | 46.79 | O |
| ATOM | 60 | N | GLY | C | 9 | 1.015 | 66.567 | 13.666 | 1.00 | 37.58 | N |
| ATOM | 61 | CA | GLY | C | 9 | 0.947 | 65.229 | 13.116 | 1.00 | 47.86 | C |
| ATOM | 62 | C | GLY | C | 9 | 0.747 | 64.199 | 14.226 | 1.00 | 51.47 | C |
| ATOM | 63 | O | GLY | C | 9 | 0.716 | 64.509 | 15.419 | 1.00 | 54.50 | O |
| ATOM | 64 | N | GLY | C | 10 | 0.731 | 62.943 | 13.833 | 1.00 | 46.74 | N |
| ATOM | 65 | CA | GLY | C | 10 | 0.618 | 61.871 | 14.804 | 1.00 | 46.77 | C |
| ATOM | 66 | C | GLY | C | 10 | -0.035 | 60.648 | 14.193 | 1.00 | 52.50 | C |
| ATOM | 67 | O | GLY | C | 10 | -0.310 | 60.586 | 12.994 | 1.00 | 47.51 | O |
| ATOM | 68 | N | VAL | C | 11 | -0.256 | 59.651 | 15.049 | 1.00 | 51.48 | N |
| ATOM | 69 | CA | VAL | C | 11 | -0.832 | 58.380 | 14.625 | 1.00 | 53.97 | C |

| | | | | | | | | | | | |
|---|---|---|---|---|---|---|---|---|---|---|---|
| ATOM | 70 | C | VAL | C | 11 | -2.320 | 58.434 | 14.900 | 1.00 | 52.43 | C |
| ATOM | 71 | O | VAL | C | 11 | -2.739 | 58.881 | 15.975 | 1.00 | 50.83 | O |
| ATOM | 72 | CB | VAL | C | 11 | -0.183 | 57.184 | 15.345 | 1.00 | 43.91 | C |
| ATOM | 73 | CG1 | VAL | C | 11 | -0.742 | 55.891 | 14.787 | 1.00 | 43.96 | C |
| ATOM | 74 | CG2 | VAL | C | 11 | 1.317 | 57.226 | 15.201 | 1.00 | 43.94 | C |
| ATOM | 75 | N | VAL | C | 12 | -3.117 | 58.015 | 13.916 | 1.00 | 45.99 | N |
| ATOM | 76 | CA | VAL | C | 12 | -4.570 | 58.072 | 13.996 | 1.00 | 46.88 | C |
| ATOM | 77 | C | VAL | C | 12 | -5.115 | 56.950 | 13.139 | 1.00 | 46.73 | C |
| ATOM | 78 | O | VAL | C | 12 | -4.469 | 56.494 | 12.191 | 1.00 | 47.91 | O |
| ATOM | 79 | CB | VAL | C | 12 | -5.179 | 59.425 | 13.540 | 1.00 | 47.29 | C |
| ATOM | 80 | CG1 | VAL | C | 12 | -4.763 | 60.547 | 14.468 | 1.00 | 46.14 | C |
| ATOM | 81 | CG2 | VAL | C | 12 | -4.755 | 59.739 | 12.130 | 1.00 | 48.50 | C |
| ATOM | 82 | N | GLN | C | 13 | -6.316 | 56.547 | 13.443 | 1.00 | 51.98 | N |
| ATOM | 83 | CA | GLN | C | 13 | -7.001 | 55.451 | 12.787 | 1.00 | 48.11 | C |
| ATOM | 84 | C | GLN | C | 13 | -7.748 | 55.950 | 11.559 | 1.00 | 42.02 | C |
| ATOM | 85 | O | GLN | C | 13 | -8.237 | 57.082 | 11.548 | 1.00 | 45.22 | O |
| ATOM | 86 | CB | GLN | C | 13 | -8.005 | 54.841 | 13.740 | 1.00 | 57.37 | C |
| ATOM | 87 | CG | GLN | C | 13 | -7.409 | 54.422 | 15.035 | 1.00 | 67.65 | C |
| ATOM | 88 | CD | GLN | C | 13 | -8.286 | 53.439 | 15.730 | 1.00 | 79.97 | C |
| ATOM | 89 | OE1 | GLN | C | 13 | -8.845 | 53.737 | 16.788 | 1.00 | 93.60 | O |
| ATOM | 90 | NE2 | GLN | C | 13 | -8.446 | 52.258 | 15.130 | 1.00 | 91.83 | N |
| ATOM | 91 | N | PRO | C | 14 | -7.878 | 55.101 | 10.541 | 1.00 | 38.64 | N |
| ATOM | 92 | CA | PRO | C | 14 | -8.652 | 55.475 | 9.351 | 1.00 | 37.32 | C |
| ATOM | 93 | C | PRO | C | 14 | -10.050 | 55.954 | 9.725 | 1.00 | 48.06 | C |
| ATOM | 94 | O | PRO | C | 14 | -10.688 | 55.419 | 10.634 | 1.00 | 50.66 | O |
| ATOM | 95 | CB | PRO | C | 14 | -8.711 | 54.174 | 8.539 | 1.00 | 35.18 | C |
| ATOM | 96 | CG | PRO | C | 14 | -7.556 | 53.370 | 9.005 | 1.00 | 40.76 | C |
| ATOM | 97 | CD | PRO | C | 14 | -7.351 | 53.727 | 10.456 | 1.00 | 40.72 | C |
| ATOM | 98 | N | GLY | C | 15 | -10.525 | 56.982 | 9.023 | 1.00 | 45.47 | N |
| ATOM | 99 | CA | GLY | C | 15 | -11.836 | 57.526 | 9.264 | 1.00 | 39.13 | C |
| ATOM | 100 | C | GLY | C | 15 | -11.886 | 58.622 | 10.303 | 1.00 | 40.65 | C |
| ATOM | 101 | O | GLY | C | 15 | -12.887 | 59.331 | 10.374 | 1.00 | 51.10 | O |
| ATOM | 102 | N | ARG | C | 16 | -10.842 | 58.781 | 11.109 | 1.00 | 42.12 | N |
| ATOM | 103 | CA | ARG | C | 16 | -10.775 | 59.854 | 12.087 | 1.00 | 42.11 | C |
| ATOM | 104 | C | ARG | C | 16 | -10.275 | 61.158 | 11.450 | 1.00 | 49.72 | C |
| ATOM | 105 | O | ARG | C | 16 | -10.040 | 61.255 | 10.237 | 1.00 | 41.14 | O |
| ATOM | 106 | CB | ARG | C | 16 | -9.865 | 59.456 | 13.243 | 1.00 | 48.99 | C |
| ATOM | 107 | CG | ARG | C | 16 | -10.287 | 58.203 | 13.961 | 1.00 | 58.16 | C |
| ATOM | 108 | CD | ARG | C | 16 | -11.621 | 58.360 | 14.645 | 1.00 | 60.17 | C |
| ATOM | 109 | NE | ARG | C | 16 | -11.592 | 57.775 | 15.983 | 1.00 | 78.30 | N |
| ATOM | 110 | CZ | ARG | C | 16 | -12.669 | 57.597 | 16.743 | 1.00 | 90.64 | C |
| ATOM | 111 | NH1 | ARG | C | 16 | -13.866 | 57.952 | 16.295 | 1.00 | 92.41 | N1+ |
| ATOM | 112 | NH2 | ARG | C | 16 | -12.552 | 57.058 | 17.950 | 1.00 | 96.43 | N |
| ATOM | 113 | N | SER | C | 17 | -10.093 | 62.174 | 12.299 | 1.00 | 44.14 | N |
| ATOM | 114 | CA | SER | C | 17 | -9.731 | 63.516 | 11.876 | 1.00 | 45.83 | C |
| ATOM | 115 | C | SER | C | 17 | -8.490 | 63.998 | 12.604 | 1.00 | 44.89 | C |
| ATOM | 116 | O | SER | C | 17 | -8.207 | 63.600 | 13.738 | 1.00 | 51.51 | O |
| ATOM | 117 | CB | SER | C | 17 | -10.853 | 64.535 | 12.132 | 1.00 | 45.46 | C |
| ATOM | 118 | OG | SER | C | 17 | -11.938 | 64.263 | 11.274 | 1.00 | 59.74 | O |
| ATOM | 119 | N | LEU | C | 18 | -7.817 | 64.947 | 11.961 | 1.00 | 41.62 | N |
| ATOM | 120 | CA | LEU | C | 18 | -6.610 | 65.573 | 12.473 | 1.00 | 44.03 | C |
| ATOM | 121 | C | LEU | C | 18 | -6.536 | 66.971 | 11.886 | 1.00 | 41.33 | C |
| ATOM | 122 | O | LEU | C | 18 | -6.961 | 67.203 | 10.756 | 1.00 | 44.87 | O |
| ATOM | 123 | CB | LEU | C | 18 | -5.366 | 64.772 | 12.079 | 1.00 | 41.97 | C |
| ATOM | 124 | CG | LEU | C | 18 | -4.114 | 64.794 | 12.940 | 1.00 | 54.29 | C |
| ATOM | 125 | CD1 | LEU | C | 18 | -4.440 | 64.407 | 14.380 | 1.00 | 50.61 | C |
| ATOM | 126 | CD2 | LEU | C | 18 | -3.075 | 63.836 | 12.334 | 1.00 | 49.38 | C |
| ATOM | 127 | N | ARG | C | 19 | -6.000 | 67.902 | 12.654 | 1.00 | 39.81 | N |
| ATOM | 128 | CA | ARG | C | 19 | -5.810 | 69.262 | 12.189 | 1.00 | 40.64 | C |
| ATOM | 129 | C | ARG | C | 19 | -4.334 | 69.605 | 12.283 | 1.00 | 48.86 | C |
| ATOM | 130 | O | ARG | C | 19 | -3.778 | 69.652 | 13.386 | 1.00 | 48.74 | O |
| ATOM | 131 | CB | ARG | C | 19 | -6.650 | 70.222 | 13.021 | 1.00 | 41.19 | C |
| ATOM | 132 | CG | ARG | C | 19 | -6.806 | 71.632 | 12.452 | 1.00 | 46.04 | C |
| ATOM | 133 | CD | ARG | C | 19 | -6.323 | 72.509 | 13.554 | 1.00 | 49.64 | C |
| ATOM | 134 | NE | ARG | C | 19 | -7.182 | 73.633 | 13.872 | 1.00 | 52.33 | N |
| ATOM | 135 | CZ | ARG | C | 19 | -6.947 | 74.447 | 14.900 | 1.00 | 60.69 | C |
| ATOM | 136 | NH1 | ARG | C | 19 | -5.914 | 74.205 | 15.702 | 1.00 | 60.16 | N1+ |
| ATOM | 137 | NH2 | ARG | C | 19 | -7.741 | 75.487 | 15.142 | 1.00 | 61.67 | N |
| ATOM | 138 | N | LEU | C | 20 | -3.716 | 69.890 | 11.137 | 1.00 | 44.47 | N |
| ATOM | 139 | CA | LEU | C | 20 | -2.328 | 70.331 | 11.121 | 1.00 | 42.17 | C |
| ATOM | 140 | C | LEU | C | 20 | -2.270 | 71.840 | 11.202 | 1.00 | 40.77 | C |

| | | | | | | | | | | | |
|---|---|---|---|---|---|---|---|---|---|---|---|
| ATOM | 141 | O | LEU | C | 20 | -3.145 | 72.544 | 10.691 | 1.00 | 39.38 | O |
| ATOM | 142 | CB | LEU | C | 20 | -1.591 | 69.860 | 9.864 | 1.00 | 32.38 | C |
| ATOM | 143 | CG | LEU | C | 20 | -1.658 | 68.360 | 9.649 | 1.00 | 39.39 | C |
| ATOM | 144 | CD1 | LEU | C | 20 | -0.808 | 67.907 | 8.483 | 1.00 | 38.47 | C |
| ATOM | 145 | CD2 | LEU | C | 20 | -1.266 | 67.633 | 10.930 | 1.00 | 41.15 | C |
| ATOM | 146 | N | SER | C | 21 | -1.221 | 72.328 | 11.845 | 1.00 | 39.02 | N |
| ATOM | 147 | CA | SER | C | 21 | -0.951 | 73.748 | 11.950 | 1.00 | 40.69 | C |
| ATOM | 148 | C | SER | C | 21 | 0.423 | 74.053 | 11.372 | 1.00 | 47.84 | C |
| ATOM | 149 | O | SER | C | 21 | 1.339 | 73.221 | 11.416 | 1.00 | 45.59 | O |
| ATOM | 150 | CB | SER | C | 21 | -1.011 | 74.211 | 13.397 | 1.00 | 38.57 | C |
| ATOM | 151 | OG | SER | C | 21 | -2.330 | 74.127 | 13.871 | 1.00 | 56.62 | O |
| ATOM | 152 | N | CYS | C | 22 | 0.546 | 75.263 | 10.834 | 1.00 | 38.99 | N |
| ATOM | 153 | CA | CYS | C | 22 | 1.795 | 75.794 | 10.299 | 1.00 | 44.99 | C |
| ATOM | 154 | C | CYS | C | 22 | 1.951 | 77.220 | 10.827 | 1.00 | 46.04 | C |
| ATOM | 155 | O | CYS | C | 22 | 1.172 | 78.113 | 10.465 | 1.00 | 41.82 | O |
| ATOM | 156 | CB | CYS | C | 22 | 1.788 | 75.761 | 8.768 | 1.00 | 44.73 | C |
| ATOM | 157 | SG | CYS | C | 22 | 3.139 | 76.705 | 7.941 | 1.00 | 52.54 | S |
| ATOM | 158 | N | ALA | C | 23 | 2.921 | 77.417 | 11.717 | 1.00 | 39.89 | N |
| ATOM | 159 | CA | ALA | C | 23 | 3.158 | 78.694 | 12.375 | 1.00 | 39.98 | C |
| ATOM | 160 | C | ALA | C | 23 | 4.271 | 79.454 | 11.659 | 1.00 | 41.32 | C |
| ATOM | 161 | O | ALA | C | 23 | 5.359 | 78.912 | 11.428 | 1.00 | 44.39 | O |
| ATOM | 162 | CB | ALA | C | 23 | 3.522 | 78.488 | 13.850 | 1.00 | 35.18 | C |
| ATOM | 163 | N | ALA | C | 24 | 3.999 | 80.703 | 11.327 | 1.00 | 39.85 | N |
| ATOM | 164 | CA | ALA | C | 24 | 4.895 | 81.524 | 10.537 | 1.00 | 43.27 | C |
| ATOM | 165 | C | ALA | C | 24 | 5.477 | 82.657 | 11.372 | 1.00 | 46.72 | C |
| ATOM | 166 | O | ALA | C | 24 | 4.801 | 83.237 | 12.220 | 1.00 | 48.85 | O |
| ATOM | 167 | CB | ALA | C | 24 | 4.157 | 82.110 | 9.339 | 1.00 | 38.90 | C |
| ATOM | 168 | N | SER | C | 25 | 6.720 | 83.018 | 11.076 | 1.00 | 44.32 | N |
| ATOM | 169 | CA | SER | C | 25 | 7.328 | 84.177 | 11.710 | 1.00 | 46.12 | C |
| ATOM | 170 | C | SER | C | 25 | 8.226 | 84.874 | 10.695 | 1.00 | 44.36 | C |
| ATOM | 171 | O | SER | C | 25 | 8.623 | 84.304 | 9.673 | 1.00 | 43.53 | O |
| ATOM | 172 | CB | SER | C | 25 | 8.122 | 83.783 | 12.966 | 1.00 | 46.62 | C |
| ATOM | 173 | OG | SER | C | 25 | 9.191 | 82.915 | 12.642 | 1.00 | 42.94 | O |
| ATOM | 174 | N | GLY | C | 26 | 8.563 | 86.112 | 11.001 | 1.00 | 46.40 | N |
| ATOM | 175 | CA | GLY | C | 26 | 9.394 | 86.915 | 10.124 | 1.00 | 42.45 | C |
| ATOM | 176 | C | GLY | C | 26 | 8.527 | 87.884 | 9.326 | 1.00 | 52.00 | C |
| ATOM | 177 | O | GLY | C | 26 | 7.673 | 88.572 | 9.891 | 1.00 | 48.03 | O |
| ATOM | 178 | N | PHE | C | 27 | 8.729 | 87.910 | 8.013 | 1.00 | 49.49 | N |
| ATOM | 179 | CA | PHE | C | 27 | 7.986 | 88.820 | 7.151 | 1.00 | 45.04 | C |
| ATOM | 180 | C | PHE | C | 27 | 6.479 | 88.576 | 7.273 | 1.00 | 44.14 | C |
| ATOM | 181 | O | PHE | C | 27 | 6.025 | 87.430 | 7.293 | 1.00 | 47.90 | O |
| ATOM | 182 | CB | PHE | C | 27 | 8.449 | 88.611 | 5.712 | 1.00 | 45.76 | C |
| ATOM | 183 | CG | PHE | C | 27 | 7.704 | 89.421 | 4.711 | 1.00 | 48.04 | C |
| ATOM | 184 | CD1 | PHE | C | 27 | 7.470 | 90.769 | 4.937 | 1.00 | 50.27 | C |
| ATOM | 185 | CD2 | PHE | C | 27 | 7.236 | 88.838 | 3.539 | 1.00 | 49.31 | C |
| ATOM | 186 | CE1 | PHE | C | 27 | 6.784 | 91.537 | 4.011 | 1.00 | 53.69 | C |
| ATOM | 187 | CE2 | PHE | C | 27 | 6.550 | 89.591 | 2.606 | 1.00 | 47.82 | C |
| ATOM | 188 | CZ | PHE | C | 27 | 6.320 | 90.951 | 2.846 | 1.00 | 49.54 | C |
| ATOM | 189 | N | THR | C | 28 | 5.709 | 89.671 | 7.335 | 1.00 | 44.86 | N |
| ATOM | 190 | CA | THR | C | 28 | 4.261 | 89.693 | 7.577 | 1.00 | 38.38 | C |
| ATOM | 191 | C | THR | C | 28 | 3.475 | 88.544 | 6.941 | 1.00 | 42.91 | C |
| ATOM | 192 | O | THR | C | 28 | 3.343 | 88.483 | 5.711 | 1.00 | 36.78 | O |
| ATOM | 193 | CB | THR | C | 28 | 3.679 | 90.998 | 7.038 | 1.00 | 49.68 | C |
| ATOM | 194 | OG1 | THR | C | 28 | 4.434 | 92.106 | 7.536 | 1.00 | 46.23 | O |
| ATOM | 195 | CG2 | THR | C | 28 | 2.208 | 91.133 | 7.410 | 1.00 | 44.54 | C |
| ATOM | 196 | N | PHE | C | 29 | 2.891 | 87.687 | 7.790 | 1.00 | 38.44 | N |
| ATOM | 197 | CA | PHE | C | 29 | 2.154 | 86.502 | 7.350 | 1.00 | 40.01 | C |
| ATOM | 198 | C | PHE | C | 29 | 1.089 | 86.822 | 6.304 | 1.00 | 36.82 | C |
| ATOM | 199 | O | PHE | C | 29 | 0.942 | 86.092 | 5.316 | 1.00 | 36.46 | O |
| ATOM | 200 | CB | PHE | C | 29 | 1.508 | 85.858 | 8.571 | 1.00 | 39.47 | C |
| ATOM | 201 | CG | PHE | C | 29 | 0.772 | 84.586 | 8.296 | 1.00 | 41.07 | C |
| ATOM | 202 | CD1 | PHE | C | 29 | 1.443 | 83.470 | 7.809 | 1.00 | 39.25 | C |
| ATOM | 203 | CD2 | PHE | C | 29 | -0.586 | 84.484 | 8.581 | 1.00 | 37.61 | C |
| ATOM | 204 | CE1 | PHE | C | 29 | 0.782 | 82.271 | 7.600 | 1.00 | 37.53 | C |
| ATOM | 205 | CE2 | PHE | C | 29 | -1.260 | 83.280 | 8.380 | 1.00 | 42.81 | C |
| ATOM | 206 | CZ | PHE | C | 29 | -0.575 | 82.170 | 7.878 | 1.00 | 41.35 | C |
| ATOM | 207 | N | SER | C | 30 | 0.352 | 87.917 | 6.489 | 1.00 | 34.06 | N |
| ATOM | 208 | CA | SER | C | 30 | -0.719 | 88.270 | 5.564 | 1.00 | 37.45 | C |
| ATOM | 209 | C | SER | C | 30 | -0.200 | 88.707 | 4.203 | 1.00 | 39.61 | C |
| ATOM | 210 | O | SER | C | 30 | -1.018 | 88.991 | 3.319 | 1.00 | 35.46 | O |
| ATOM | 211 | CB | SER | C | 30 | -1.587 | 89.392 | 6.141 | 1.00 | 31.23 | C |

| | | | | | | | | | | | |
|---|---|---|---|---|---|---|---|---|---|---|---|
| ATOM | 212 | OG | SER | C | 30 | -0.804 | 90.566 | 6.341 | 1.00 | 38.68 | O |
| ATOM | 213 | N | SER | C | 31 | 1.115 | 88.752 | 3.998 | 1.00 | 31.95 | N |
| ATOM | 214 | CA | SER | C | 31 | 1.653 | 89.144 | 2.705 | 1.00 | 42.10 | C |
| ATOM | 215 | C | SER | C | 31 | 1.858 | 87.979 | 1.743 | 1.00 | 36.93 | C |
| ATOM | 216 | O | SER | C | 31 | 2.347 | 88.199 | 0.629 | 1.00 | 40.45 | O |
| ATOM | 217 | CB | SER | C | 31 | 2.975 | 89.887 | 2.883 | 1.00 | 38.00 | C |
| ATOM | 218 | OG | SER | C | 31 | 2.725 | 91.165 | 3.425 | 1.00 | 50.55 | O |
| ATOM | 219 | N | TYR | C | 32 | 1.476 | 86.759 | 2.103 | 1.00 | 32.77 | N |
| ATOM | 220 | CA | TYR | C | 32 | 1.671 | 85.678 | 1.145 | 1.00 | 37.31 | C |
| ATOM | 221 | C | TYR | C | 32 | 0.613 | 84.597 | 1.312 | 1.00 | 35.54 | C |
| ATOM | 222 | O | TYR | C | 32 | 0.059 | 84.403 | 2.401 | 1.00 | 33.81 | O |
| ATOM | 223 | CB | TYR | C | 32 | 3.083 | 85.087 | 1.282 | 1.00 | 33.59 | C |
| ATOM | 224 | CG | TYR | C | 32 | 3.516 | 84.779 | 2.701 | 1.00 | 29.77 | C |
| ATOM | 225 | CD1 | TYR | C | 32 | 3.130 | 83.599 | 3.315 | 1.00 | 33.82 | C |
| ATOM | 226 | CD2 | TYR | C | 32 | 4.353 | 85.643 | 3.405 | 1.00 | 38.18 | C |
| ATOM | 227 | CE1 | TYR | C | 32 | 3.550 | 83.271 | 4.603 | 1.00 | 38.72 | C |
| ATOM | 228 | CE2 | TYR | C | 32 | 4.781 | 85.340 | 4.703 | 1.00 | 39.15 | C |
| ATOM | 229 | CZ | TYR | C | 32 | 4.365 | 84.145 | 5.296 | 1.00 | 45.73 | C |
| ATOM | 230 | OH | TYR | C | 32 | 4.741 | 83.816 | 6.579 | 1.00 | 41.78 | O |
| ATOM | 231 | N | GLY | C | 33 | 0.344 | 83.893 | 0.209 | 1.00 | 34.05 | N |
| ATOM | 232 | CA | GLY | C | 33 | -0.428 | 82.670 | 0.260 | 1.00 | 32.21 | C |
| ATOM | 233 | C | GLY | C | 33 | 0.446 | 81.497 | 0.679 | 1.00 | 39.56 | C |
| ATOM | 234 | O | GLY | C | 33 | 1.665 | 81.640 | 0.823 | 1.00 | 32.33 | O |
| ATOM | 235 | N | LEU | C | 34 | -0.196 | 80.340 | 0.907 | 1.00 | 32.53 | N |
| ATOM | 236 | CA | LEU | C | 34 | 0.521 | 79.169 | 1.401 | 1.00 | 34.46 | C |
| ATOM | 237 | C | LEU | C | 34 | -0.060 | 77.886 | 0.824 | 1.00 | 30.24 | C |
| ATOM | 238 | O | LEU | C | 34 | -1.200 | 77.841 | 0.360 | 1.00 | 33.75 | O |
| ATOM | 239 | CB | LEU | C | 34 | 0.515 | 79.085 | 2.933 | 1.00 | 38.99 | C |
| ATOM | 240 | CG | LEU | C | 34 | 1.236 | 80.236 | 3.655 | 1.00 | 41.08 | C |
| ATOM | 241 | CD1 | LEU | C | 34 | 0.223 | 81.245 | 4.217 | 1.00 | 33.75 | C |
| ATOM | 242 | CD2 | LEU | C | 34 | 2.165 | 79.704 | 4.725 | 1.00 | 35.97 | C |
| ATOM | 243 | N | HIS | C | 35 | 0.768 | 76.846 | 0.863 | 1.00 | 31.72 | N |
| ATOM | 244 | CA | HIS | C | 35 | 0.503 | 75.507 | 0.371 | 1.00 | 29.96 | C |
| ATOM | 245 | C | HIS | C | 35 | 0.618 | 74.498 | 1.498 | 1.00 | 33.37 | C |
| ATOM | 246 | O | HIS | C | 35 | 1.323 | 74.717 | 2.484 | 1.00 | 34.65 | O |
| ATOM | 247 | CB | HIS | C | 35 | 1.534 | 75.064 | -0.671 | 1.00 | 31.82 | C |
| ATOM | 248 | CG | HIS | C | 35 | 1.514 | 75.835 | -1.949 | 1.00 | 38.58 | C |
| ATOM | 249 | ND1 | HIS | C | 35 | 0.781 | 75.435 | -3.047 | 1.00 | 31.99 | N |
| ATOM | 250 | CD2 | HIS | C | 35 | 2.200 | 76.940 | -2.333 | 1.00 | 34.19 | C |
| ATOM | 251 | CE1 | HIS | C | 35 | 1.008 | 76.269 | -4.046 | 1.00 | 28.49 | C |
| ATOM | 252 | NE2 | HIS | C | 35 | 1.855 | 77.197 | -3.635 | 1.00 | 33.84 | N |
| ATOM | 253 | N | TRP | C | 36 | -0.010 | 73.347 | 1.298 | 1.00 | 29.55 | N |
| ATOM | 254 | CA | TRP | C | 36 | 0.381 | 72.110 | 1.957 | 1.00 | 30.04 | C |
| ATOM | 255 | C | TRP | C | 36 | 0.870 | 71.151 | 0.887 | 1.00 | 32.99 | C |
| ATOM | 256 | O | TRP | C | 36 | 0.217 | 70.995 | -0.149 | 1.00 | 33.39 | O |
| ATOM | 257 | CB | TRP | C | 36 | -0.771 | 71.490 | 2.735 | 1.00 | 30.51 | C |
| ATOM | 258 | CG | TRP | C | 36 | -1.084 | 72.233 | 3.977 | 1.00 | 36.65 | C |
| ATOM | 259 | CD1 | TRP | C | 36 | -2.063 | 73.191 | 4.152 | 1.00 | 36.08 | C |
| ATOM | 260 | CD2 | TRP | C | 36 | -0.447 | 72.073 | 5.246 | 1.00 | 33.16 | C |
| ATOM | 261 | NE1 | TRP | C | 36 | -2.055 | 73.637 | 5.456 | 1.00 | 34.80 | N |
| ATOM | 262 | CE2 | TRP | C | 36 | -1.073 | 72.972 | 6.146 | 1.00 | 31.89 | C |
| ATOM | 263 | CE3 | TRP | C | 36 | 0.602 | 71.272 | 5.706 | 1.00 | 33.27 | C |
| ATOM | 264 | CZ2 | TRP | C | 36 | -0.690 | 73.079 | 7.475 | 1.00 | 35.29 | C |
| ATOM | 265 | CZ3 | TRP | C | 36 | 0.976 | 71.373 | 7.022 | 1.00 | 36.31 | C |
| ATOM | 266 | CH2 | TRP | C | 36 | 0.334 | 72.276 | 7.898 | 1.00 | 37.43 | C |
| ATOM | 267 | N | VAL | C | 37 | 2.037 | 70.544 | 1.114 | 1.00 | 31.46 | N |
| ATOM | 268 | CA | VAL | C | 37 | 2.588 | 69.540 | 0.215 | 1.00 | 28.53 | C |
| ATOM | 269 | C | VAL | C | 37 | 2.937 | 68.334 | 1.066 | 1.00 | 37.43 | C |
| ATOM | 270 | O | VAL | C | 37 | 3.354 | 68.473 | 2.221 | 1.00 | 38.22 | O |
| ATOM | 271 | CB | VAL | C | 37 | 3.832 | 70.056 | -0.554 | 1.00 | 32.46 | C |
| ATOM | 272 | CG1 | VAL | C | 37 | 4.424 | 68.959 | -1.463 | 1.00 | 28.26 | C |
| ATOM | 273 | CG2 | VAL | C | 37 | 3.501 | 71.313 | -1.364 | 1.00 | 25.69 | C |
| ATOM | 274 | N | ARG | C | 38 | 2.787 | 67.142 | 0.503 | 1.00 | 30.65 | N |
| ATOM | 275 | CA | ARG | C | 38 | 3.030 | 65.960 | 1.303 | 1.00 | 35.42 | C |
| ATOM | 276 | C | ARG | C | 38 | 3.936 | 64.983 | 0.565 | 1.00 | 33.75 | C |
| ATOM | 277 | O | ARG | C | 38 | 4.133 | 65.061 | -0.657 | 1.00 | 31.48 | O |
| ATOM | 278 | CB | ARG | C | 38 | 1.721 | 65.273 | 1.714 | 1.00 | 31.04 | C |
| ATOM | 279 | CG | ARG | C | 38 | 1.037 | 64.459 | 0.654 | 1.00 | 31.84 | C |
| ATOM | 280 | CD | ARG | C | 38 | -0.318 | 63.986 | 1.190 | 1.00 | 28.76 | C |
| ATOM | 281 | NE | ARG | C | 38 | -1.059 | 63.233 | 0.184 | 1.00 | 33.40 | N |
| ATOM | 282 | CZ | ARG | C | 38 | -2.280 | 62.718 | 0.368 | 1.00 | 33.79 | C |

| | | | | | | | | | | | |
|---|---|---|---|---|---|---|---|---|---|---|---|
| ATOM | 283 | NH1 | ARG | C | 38 | -2.940 | 62.867 | 1.523 | 1.00 | 29.44 | N1+ |
| ATOM | 284 | NH2 | ARG | C | 38 | -2.845 | 62.057 | -0.617 | 1.00 | 27.56 | N |
| ATOM | 285 | N | GLN | C | 39 | 4.517 | 64.077 | 1.344 | 1.00 | 31.44 | N |
| ATOM | 286 | CA | GLN | C | 39 | 5.466 | 63.101 | 0.809 | 1.00 | 33.90 | C |
| ATOM | 287 | C | GLN | C | 39 | 5.299 | 61.833 | 1.621 | 1.00 | 30.60 | C |
| ATOM | 288 | O | GLN | C | 39 | 5.589 | 61.823 | 2.816 | 1.00 | 37.53 | O |
| ATOM | 289 | CB | GLN | C | 39 | 6.908 | 63.606 | 0.880 | 1.00 | 29.62 | C |
| ATOM | 290 | CG | GLN | C | 39 | 7.950 | 62.614 | 0.336 | 1.00 | 33.68 | C |
| ATOM | 291 | CD | GLN | C | 39 | 9.317 | 63.272 | 0.142 | 1.00 | 38.26 | C |
| ATOM | 292 | OE1 | GLN | C | 39 | 9.832 | 63.941 | 1.041 | 1.00 | 36.27 | O |
| ATOM | 293 | NE2 | GLN | C | 39 | 9.884 | 63.121 | -1.051 | 1.00 | 31.46 | N |
| ATOM | 294 | N | ALA | C | 40 | 4.806 | 60.795 | 0.980 | 1.00 | 33.74 | N |
| ATOM | 295 | CA | ALA | C | 40 | 4.687 | 59.501 | 1.621 | 1.00 | 42.24 | C |
| ATOM | 296 | C | ALA | C | 40 | 6.082 | 58.868 | 1.723 | 1.00 | 41.93 | C |
| ATOM | 297 | O | ALA | C | 40 | 6.967 | 59.180 | 0.921 | 1.00 | 39.79 | O |
| ATOM | 298 | CB | ALA | C | 40 | 3.726 | 58.625 | 0.811 | 1.00 | 21.89 | C |
| ATOM | 299 | N | PRO | C | 41 | 6.304 | 57.974 | 2.691 | 1.00 | 47.02 | N |
| ATOM | 300 | CA | PRO | C | 41 | 7.671 | 57.444 | 2.920 | 1.00 | 46.31 | C |
| ATOM | 301 | C | PRO | C | 41 | 8.252 | 56.774 | 1.681 | 1.00 | 40.48 | C |
| ATOM | 302 | O | PRO | C | 41 | 7.677 | 55.834 | 1.127 | 1.00 | 49.95 | O |
| ATOM | 303 | CB | PRO | C | 41 | 7.475 | 56.438 | 4.065 | 1.00 | 38.60 | C |
| ATOM | 304 | CG | PRO | C | 41 | 6.179 | 56.872 | 4.754 | 1.00 | 43.78 | C |
| ATOM | 305 | CD | PRO | C | 41 | 5.319 | 57.433 | 3.651 | 1.00 | 44.84 | C |
| ATOM | 306 | N | GLY | C | 42 | 9.416 | 57.254 | 1.256 | 1.00 | 49.67 | N |
| ATOM | 307 | CA | GLY | C | 42 | 10.049 | 56.738 | 0.054 | 1.00 | 47.19 | C |
| ATOM | 308 | C | GLY | C | 42 | 9.476 | 57.189 | -1.279 | 1.00 | 48.22 | C |
| ATOM | 309 | O | GLY | C | 42 | 9.825 | 56.588 | -2.300 | 1.00 | 45.62 | O |
| ATOM | 310 | N | LYS | C | 43 | 8.627 | 58.223 | -1.325 | 1.00 | 42.77 | N |
| ATOM | 311 | CA | LYS | C | 43 | 7.904 | 58.581 | -2.556 | 1.00 | 40.19 | C |
| ATOM | 312 | C | LYS | C | 43 | 8.149 | 60.040 | -2.956 | 1.00 | 36.26 | C |
| ATOM | 313 | O | LYS | C | 43 | 8.895 | 60.789 | -2.310 | 1.00 | 41.39 | O |
| ATOM | 314 | CB | LYS | C | 43 | 6.402 | 58.338 | -2.390 | 1.00 | 43.74 | C |
| ATOM | 315 | CG | LYS | C | 43 | 6.020 | 56.900 | -2.068 | 1.00 | 44.39 | C |
| ATOM | 316 | CD | LYS | C | 43 | 6.527 | 55.936 | -3.112 | 1.00 | 56.25 | C |
| ATOM | 317 | CE | LYS | C | 43 | 5.920 | 54.540 | -2.918 | 1.00 | 72.49 | C |
| ATOM | 318 | NZ | LYS | C | 43 | 6.709 | 53.467 | -3.617 | 1.00 | 73.82 | N1+ |
| ATOM | 319 | N | GLY | C | 44 | 7.498 | 60.457 | -4.036 | 1.00 | 35.63 | N |
| ATOM | 320 | CA | GLY | C | 44 | 7.709 | 61.788 | -4.551 | 1.00 | 29.55 | C |
| ATOM | 321 | C | GLY | C | 44 | 6.833 | 62.802 | -3.843 | 1.00 | 34.99 | C |
| ATOM | 322 | O | GLY | C | 44 | 6.008 | 62.475 | -2.990 | 1.00 | 36.77 | O |
| ATOM | 323 | N | LEU | C | 45 | 7.026 | 64.067 | -4.205 | 1.00 | 30.55 | N |
| ATOM | 324 | CA | LEU | C | 45 | 6.192 | 65.134 | -3.664 | 1.00 | 32.18 | C |
| ATOM | 325 | C | LEU | C | 45 | 4.783 | 65.057 | -4.257 | 1.00 | 31.76 | C |
| ATOM | 326 | O | LEU | C | 45 | 4.597 | 64.740 | -5.440 | 1.00 | 25.93 | O |
| ATOM | 327 | CB | LEU | C | 45 | 6.820 | 66.508 | -3.939 | 1.00 | 31.66 | C |
| ATOM | 328 | CG | LEU | C | 45 | 8.233 | 66.714 | -3.370 | 1.00 | 32.55 | C |
| ATOM | 329 | CD1 | LEU | C | 45 | 8.776 | 68.091 | -3.682 | 1.00 | 27.48 | C |
| ATOM | 330 | CD2 | LEU | C | 45 | 8.219 | 66.491 | -1.871 | 1.00 | 28.43 | C |
| ATOM | 331 | N | GLU | C | 46 | 3.784 | 65.298 | -3.413 | 1.00 | 27.12 | N |
| ATOM | 332 | CA | GLU | C | 46 | 2.404 | 65.402 | -3.865 | 1.00 | 32.84 | C |
| ATOM | 333 | C | GLU | C | 46 | 1.799 | 66.669 | -3.286 | 1.00 | 32.06 | C |
| ATOM | 334 | O | GLU | C | 46 | 1.754 | 66.837 | -2.062 | 1.00 | 31.76 | O |
| ATOM | 335 | CB | GLU | C | 46 | 1.579 | 64.183 | -3.451 | 1.00 | 36.78 | C |
| ATOM | 336 | CG | GLU | C | 46 | 0.226 | 64.173 | -4.126 | 1.00 | 44.38 | C |
| ATOM | 337 | CD | GLU | C | 46 | -0.758 | 63.166 | -3.539 | 1.00 | 46.89 | C |
| ATOM | 338 | OE1 | GLU | C | 46 | -0.435 | 62.467 | -2.548 | 1.00 | 45.34 | O |
| ATOM | 339 | OE2 | GLU | C | 46 | -1.871 | 63.088 | -4.092 | 1.00 | 50.23 | O1- |
| ATOM | 340 | N | TRP | C | 47 | 1.330 | 67.548 | -4.162 | 1.00 | 27.92 | N |
| ATOM | 341 | CA | TRP | C | 47 | 0.676 | 68.764 | -3.715 | 1.00 | 31.90 | C |
| ATOM | 342 | C | TRP | C | 47 | -0.664 | 68.439 | -3.079 | 1.00 | 31.01 | C |
| ATOM | 343 | O | TRP | C | 47 | -1.386 | 67.555 | -3.546 | 1.00 | 32.31 | O |
| ATOM | 344 | CB | TRP | C | 47 | 0.485 | 69.713 | -4.900 | 1.00 | 35.33 | C |
| ATOM | 345 | CG | TRP | C | 47 | -0.297 | 70.972 | -4.630 | 1.00 | 30.11 | C |
| ATOM | 346 | CD1 | TRP | C | 47 | 0.104 | 72.068 | -3.910 | 1.00 | 29.39 | C |
| ATOM | 347 | CD2 | TRP | C | 47 | -1.616 | 71.270 | -5.118 | 1.00 | 29.40 | C |
| ATOM | 348 | NE1 | TRP | C | 47 | -0.886 | 73.032 | -3.925 | 1.00 | 32.24 | N |
| ATOM | 349 | CE2 | TRP | C | 47 | -1.952 | 72.563 | -4.660 | 1.00 | 34.57 | C |
| ATOM | 350 | CE3 | TRP | C | 47 | -2.534 | 70.575 | -5.913 | 1.00 | 30.62 | C |
| ATOM | 351 | CZ2 | TRP | C | 47 | -3.190 | 73.165 | -4.958 | 1.00 | 29.29 | C |
| ATOM | 352 | CZ3 | TRP | C | 47 | -3.764 | 71.177 | -6.215 | 1.00 | 31.59 | C |
| ATOM | 353 | CH2 | TRP | C | 47 | -4.075 | 72.459 | -5.736 | 1.00 | 32.18 | C |

| | | | | | | | | | | | |
|---|---|---|---|---|---|---|---|---|---|---|---|
| ATOM | 354 | N | VAL | C | 48 | -1.010 | 69.189 | -2.031 | 1.00 | 31.17 | N |
| ATOM | 355 | CA | VAL | C | 48 | -2.268 | 69.019 | -1.303 | 1.00 | 28.29 | C |
| ATOM | 356 | C | VAL | C | 48 | -3.239 | 70.192 | -1.553 | 1.00 | 34.03 | C |
| ATOM | 357 | O | VAL | C | 48 | -4.371 | 69.989 | -2.002 | 1.00 | 29.40 | O |
| ATOM | 358 | CB | VAL | C | 48 | -2.007 | 68.824 | 0.206 | 1.00 | 29.92 | C |
| ATOM | 359 | CG1 | VAL | C | 48 | -3.321 | 68.671 | 0.944 | 1.00 | 32.67 | C |
| ATOM | 360 | CG2 | VAL | C | 48 | -1.117 | 67.606 | 0.433 | 1.00 | 28.26 | C |
| ATOM | 361 | N | ALA | C | 49 | -2.825 | 71.421 | -1.237 | 1.00 | 30.79 | N |
| ATOM | 362 | CA | ALA | C | 49 | -3.747 | 72.546 | -1.373 | 1.00 | 31.82 | C |
| ATOM | 363 | C | ALA | C | 49 | -2.974 | 73.854 | -1.325 | 1.00 | 32.29 | C |
| ATOM | 364 | O | ALA | C | 49 | -1.845 | 73.908 | -0.823 | 1.00 | 30.87 | O |
| ATOM | 365 | CB | ALA | C | 49 | -4.821 | 72.517 | -0.277 | 1.00 | 30.36 | C |
| ATOM | 366 | N | AVAL | C | 50 | -3.605 | 74.914 | -1.835 | 0.50 | 31.84 | N |
| ATOM | 367 | CA | AVAL | C | 50 | -3.060 | 76.269 | -1.763 | 0.50 | 30.56 | C |
| ATOM | 368 | C | AVAL | C | 50 | -4.179 | 77.222 | -1.373 | 0.50 | 31.68 | C |
| ATOM | 369 | O | AVAL | C | 50 | -5.333 | 77.047 | -1.784 | 0.50 | 29.47 | O |
| ATOM | 370 | CB | AVAL | C | 50 | -2.408 | 76.711 | -3.093 | 0.50 | 30.12 | C |
| ATOM | 371 | CG1 | AVAL | C | 50 | -3.459 | 76.855 | -4.204 | 0.50 | 28.96 | C |
| ATOM | 372 | CG2 | AVAL | C | 50 | -1.624 | 77.987 | -2.912 | 0.50 | 24.87 | C |
| ATOM | 373 | N | BVAL | C | 50 | -3.584 | 74.900 | -1.900 | 0.50 | 32.14 | N |
| ATOM | 374 | CA | BVAL | C | 50 | -3.117 | 76.279 | -1.776 | 0.50 | 30.77 | C |
| ATOM | 375 | C | BVAL | C | 50 | -4.231 | 77.106 | -1.160 | 0.50 | 31.70 | C |
| ATOM | 376 | O | BVAL | C | 50 | -5.415 | 76.765 | -1.240 | 0.50 | 30.32 | O |
| ATOM | 377 | CB | BVAL | C | 50 | -2.698 | 76.932 | -3.115 | 0.50 | 30.01 | C |
| ATOM | 378 | CG1 | BVAL | C | 50 | -2.103 | 75.960 | -4.000 | 0.50 | 36.11 | C |
| ATOM | 379 | CG2 | BVAL | C | 50 | -3.896 | 77.491 | -3.831 | 0.50 | 31.66 | C |
| ATOM | 380 | N | ILE | C | 51 | -3.834 | 78.232 | -0.574 | 1.00 | 31.49 | N |
| ATOM | 381 | CA | ILE | C | 51 | -4.770 | 79.260 | -0.151 | 1.00 | 28.89 | C |
| ATOM | 382 | C | ILE | C | 51 | -4.143 | 80.593 | -0.519 | 1.00 | 37.27 | C |
| ATOM | 383 | O | ILE | C | 51 | -2.913 | 80.736 | -0.534 | 1.00 | 32.74 | O |
| ATOM | 384 | CB | ILE | C | 51 | -5.085 | 79.203 | 1.356 | 1.00 | 33.18 | C |
| ATOM | 385 | CG1 | ILE | C | 51 | -6.238 | 80.154 | 1.691 | 1.00 | 31.97 | C |
| ATOM | 386 | CG2 | ILE | C | 51 | -3.837 | 79.550 | 2.210 | 1.00 | 26.57 | C |
| ATOM | 387 | CD1 | ILE | C | 51 | -6.766 | 79.971 | 3.093 | 1.00 | 31.63 | C |
| ATOM | 388 | N | TRP | C | 52 | -5.000 | 81.561 | -0.838 | 1.00 | 30.70 | N |
| ATOM | 389 | CA | TRP | C | 52 | -4.563 | 82.882 | -1.241 | 1.00 | 25.99 | C |
| ATOM | 390 | C | TRP | C | 52 | -4.040 | 83.652 | -0.036 | 1.00 | 29.12 | C |
| ATOM | 391 | O | TRP | C | 52 | -4.302 | 83.303 | 1.117 | 1.00 | 36.14 | O |
| ATOM | 392 | CB | TRP | C | 52 | -5.722 | 83.638 | -1.898 | 1.00 | 27.78 | C |
| ATOM | 393 | CG | TRP | C | 52 | -5.405 | 84.135 | -3.270 | 1.00 | 33.59 | C |
| ATOM | 394 | CD1 | TRP | C | 52 | -5.333 | 85.445 | -3.676 | 1.00 | 32.68 | C |
| ATOM | 395 | CD2 | TRP | C | 52 | -5.102 | 83.340 | -4.426 | 1.00 | 30.76 | C |
| ATOM | 396 | NE1 | TRP | C | 52 | -5.004 | 85.510 | -5.010 | 1.00 | 32.81 | N |
| ATOM | 397 | CE2 | TRP | C | 52 | -4.853 | 84.235 | -5.495 | 1.00 | 33.10 | C |
| ATOM | 398 | CE3 | TRP | C | 52 | -4.989 | 81.962 | -4.657 | 1.00 | 33.14 | C |
| ATOM | 399 | CZ2 | TRP | C | 52 | -4.516 | 83.792 | -6.779 | 1.00 | 32.67 | C |
| ATOM | 400 | CZ3 | TRP | C | 52 | -4.670 | 81.524 | -5.941 | 1.00 | 33.56 | C |
| ATOM | 401 | CH2 | TRP | C | 52 | -4.435 | 82.436 | -6.983 | 1.00 | 32.43 | C |
| ATOM | 402 | N | TYR | C | 53 | -3.304 | 84.726 | -0.315 | 1.00 | 29.93 | N |
| ATOM | 403 | CA | TYR | C | 53 | -2.784 | 85.558 | 0.768 | 1.00 | 38.06 | C |
| ATOM | 404 | C | TYR | C | 53 | -3.898 | 86.148 | 1.630 | 1.00 | 40.02 | C |
| ATOM | 405 | O | TYR | C | 53 | -3.690 | 86.389 | 2.829 | 1.00 | 43.14 | O |
| ATOM | 406 | CB | TYR | C | 53 | -1.915 | 86.685 | 0.203 | 1.00 | 36.99 | C |
| ATOM | 407 | CG | TYR | C | 53 | -2.522 | 87.380 | -0.992 | 1.00 | 42.26 | C |
| ATOM | 408 | CD1 | TYR | C | 53 | -3.449 | 88.409 | -0.831 | 1.00 | 39.71 | C |
| ATOM | 409 | CD2 | TYR | C | 53 | -2.172 | 86.999 | -2.290 | 1.00 | 38.52 | C |
| ATOM | 410 | CE1 | TYR | C | 53 | -4.015 | 89.042 | -1.927 | 1.00 | 38.70 | C |
| ATOM | 411 | CE2 | TYR | C | 53 | -2.720 | 87.631 | -3.397 | 1.00 | 43.88 | C |
| ATOM | 412 | CZ | TYR | C | 53 | -3.647 | 88.654 | -3.211 | 1.00 | 46.93 | C |
| ATOM | 413 | OH | TYR | C | 53 | -4.205 | 89.264 | -4.318 | 1.00 | 49.29 | O |
| ATOM | 414 | N | ASP | C | 54 | -5.082 | 86.385 | 1.058 | 1.00 | 35.51 | N |
| ATOM | 415 | CA | ASP | C | 54 | -6.200 | 86.943 | 1.811 | 1.00 | 38.58 | C |
| ATOM | 416 | C | ASP | C | 54 | -7.258 | 85.899 | 2.166 | 1.00 | 37.13 | C |
| ATOM | 417 | O | ASP | C | 54 | -8.397 | 86.262 | 2.468 | 1.00 | 42.80 | O |
| ATOM | 418 | CB | ASP | C | 54 | -6.839 | 88.093 | 1.036 | 1.00 | 35.61 | C |
| ATOM | 419 | CG | ASP | C | 54 | -7.319 | 87.664 | -0.336 | 1.00 | 39.47 | C |
| ATOM | 420 | OD1 | ASP | C | 54 | -7.355 | 86.435 | -0.617 | 1.00 | 40.37 | O |
| ATOM | 421 | OD2 | ASP | C | 54 | -7.678 | 88.551 | -1.129 | 1.00 | 40.04 | O1- |
| ATOM | 422 | N | GLY | C | 55 | -6.921 | 84.614 | 2.110 | 1.00 | 38.50 | N |
| ATOM | 423 | CA | GLY | C | 55 | -7.878 | 83.579 | 2.451 | 1.00 | 30.61 | C |
| ATOM | 424 | C | GLY | C | 55 | -9.007 | 83.374 | 1.465 | 1.00 | 39.39 | C |

| | | | | | | | | | | | |
|---|---|---|---|---|---|---|---|---|---|---|---|
| ATOM | 425 | O | GLY | C | 55 | -9.946 | 82.625 | 1.773 | 1.00 | 37.86 | O |
| ATOM | 426 | N | SER | C | 56 | -8.944 | 83.988 | 0.276 | 1.00 | 37.78 | N |
| ATOM | 427 | CA | SER | C | 56 | -10.036 | 83.866 | -0.689 | 1.00 | 30.88 | C |
| ATOM | 428 | C | SER | C | 56 | -9.948 | 82.566 | -1.485 | 1.00 | 29.51 | C |
| ATOM | 429 | O | SER | C | 56 | -10.561 | 81.560 | -1.107 | 1.00 | 35.66 | O |
| ATOM | 430 | CB | SER | C | 56 | -10.050 | 85.075 | -1.634 | 1.00 | 31.22 | C |
| ATOM | 431 | OG | SER | C | 56 | -8.824 | 85.214 | -2.331 | 1.00 | 32.22 | O |
| ATOM | 432 | N | ASN | C | 57 | -9.192 | 82.555 | -2.578 | 1.00 | 28.37 | N |
| ATOM | 433 | CA | ASN | C | 57 | -9.143 | 81.363 | -3.423 | 1.00 | 35.02 | C |
| ATOM | 434 | C | ASN | C | 57 | -8.480 | 80.196 | -2.691 | 1.00 | 32.70 | C |
| ATOM | 435 | O | ASN | C | 57 | -7.544 | 80.388 | -1.909 | 1.00 | 29.84 | O |
| ATOM | 436 | CB | ASN | C | 57 | -8.407 | 81.655 | -4.733 | 1.00 | 29.59 | C |
| ATOM | 437 | CG | ASN | C | 57 | -9.224 | 82.532 | -5.682 | 1.00 | 35.30 | C |
| ATOM | 438 | OD1 | ASN | C | 57 | -10.082 | 83.295 | -5.248 | 1.00 | 31.05 | O |
| ATOM | 439 | ND2 | ASN | C | 57 | -8.982 | 82.390 | -6.988 | 1.00 | 33.63 | N |
| ATOM | 440 | N | LYS | C | 58 | -9.018 | 78.992 | -2.915 | 1.00 | 28.81 | N |
| ATOM | 441 | CA | LYS | C | 58 | -8.510 | 77.731 | -2.374 | 1.00 | 33.21 | C |
| ATOM | 442 | C | LYS | C | 58 | -8.561 | 76.699 | -3.485 | 1.00 | 31.65 | C |
| ATOM | 443 | O | LYS | C | 58 | -9.609 | 76.539 | -4.111 | 1.00 | 31.96 | O |
| ATOM | 444 | CB | LYS | C | 58 | -9.356 | 77.210 | -1.201 | 1.00 | 29.22 | C |
| ATOM | 445 | CG | LYS | C | 58 | -9.438 | 78.136 | -0.030 | 1.00 | 39.00 | C |
| ATOM | 446 | CD | LYS | C | 58 | -10.443 | 77.655 | 1.024 | 1.00 | 28.02 | C |
| ATOM | 447 | CE | LYS | C | 58 | -10.456 | 78.663 | 2.179 | 1.00 | 29.93 | C |
| ATOM | 448 | NZ | LYS | C | 58 | -11.039 | 78.171 | 3.465 | 1.00 | 34.24 | N1+ |
| ATOM | 449 | N | TYR | C | 59 | -7.465 | 75.969 | -3.693 | 1.00 | 33.17 | N |
| ATOM | 450 | CA | TYR | C | 59 | -7.402 | 74.893 | -4.680 | 1.00 | 31.69 | C |
| ATOM | 451 | C | TYR | C | 59 | -6.931 | 73.623 | -3.988 | 1.00 | 32.39 | C |
| ATOM | 452 | O | TYR | C | 59 | -6.165 | 73.681 | -3.025 | 1.00 | 32.73 | O |
| ATOM | 453 | CB | TYR | C | 59 | -6.450 | 75.203 | -5.856 | 1.00 | 33.11 | C |
| ATOM | 454 | CG | TYR | C | 59 | -6.646 | 76.536 | -6.574 | 1.00 | 41.69 | C |
| ATOM | 455 | CD1 | TYR | C | 59 | -6.539 | 77.741 | -5.914 | 1.00 | 48.94 | C |
| ATOM | 456 | CD2 | TYR | C | 59 | -6.892 | 76.574 | -7.925 | 1.00 | 52.57 | C |
| ATOM | 457 | CE1 | TYR | C | 59 | -6.714 | 78.936 | -6.579 | 1.00 | 54.05 | C |
| ATOM | 458 | CE2 | TYR | C | 59 | -7.055 | 77.762 | -8.590 | 1.00 | 51.75 | C |
| ATOM | 459 | CZ | TYR | C | 59 | -6.973 | 78.936 | -7.916 | 1.00 | 42.38 | C |
| ATOM | 460 | OH | TYR | C | 59 | -7.155 | 80.120 | -8.583 | 1.00 | 40.63 | O |
| ATOM | 461 | N | TYR | C | 60 | -7.401 | 72.470 | -4.475 | 1.00 | 28.26 | N |
| ATOM | 462 | CA | TYR | C | 60 | -7.080 | 71.205 | -3.835 | 1.00 | 26.89 | C |
| ATOM | 463 | C | TYR | C | 60 | -6.720 | 70.156 | -4.864 | 1.00 | 32.21 | C |
| ATOM | 464 | O | TYR | C | 60 | -7.276 | 70.128 | -5.967 | 1.00 | 30.27 | O |
| ATOM | 465 | CB | TYR | C | 60 | -8.232 | 70.657 | -3.004 | 1.00 | 28.01 | C |
| ATOM | 466 | CG | TYR | C | 60 | -8.665 | 71.517 | -1.854 | 1.00 | 34.24 | C |
| ATOM | 467 | CD1 | TYR | C | 60 | -9.607 | 72.540 | -2.036 | 1.00 | 31.75 | C |
| ATOM | 468 | CD2 | TYR | C | 60 | -8.169 | 71.297 | -0.570 | 1.00 | 31.69 | C |
| ATOM | 469 | CE1 | TYR | C | 60 | -10.020 | 73.341 | -0.963 | 1.00 | 28.22 | C |
| ATOM | 470 | CE2 | TYR | C | 60 | -8.595 | 72.093 | 0.512 | 1.00 | 30.87 | C |
| ATOM | 471 | CZ | TYR | C | 60 | -9.516 | 73.111 | 0.301 | 1.00 | 28.55 | C |
| ATOM | 472 | OH | TYR | C | 60 | -9.924 | 73.903 | 1.354 | 1.00 | 30.94 | O |
| ATOM | 473 | N | ALA | C | 61 | -5.807 | 69.267 | -4.468 | 1.00 | 27.28 | N |
| ATOM | 474 | CA | ALA | C | 61 | -5.533 | 68.075 | -5.252 | 1.00 | 28.59 | C |
| ATOM | 475 | C | ALA | C | 61 | -6.745 | 67.149 | -5.238 | 1.00 | 34.61 | C |
| ATOM | 476 | O | ALA | C | 61 | -7.493 | 67.080 | -4.260 | 1.00 | 31.50 | O |
| ATOM | 477 | CB | ALA | C | 61 | -4.315 | 67.342 | -4.706 | 1.00 | 23.81 | C |
| ATOM | 478 | N | ASP | C | 62 | -6.920 | 66.423 | -6.341 | 1.00 | 38.58 | N |
| ATOM | 479 | CA | ASP | C | 62 | -8.069 | 65.537 | -6.487 | 1.00 | 40.90 | C |
| ATOM | 480 | C | ASP | C | 62 | -8.129 | 64.490 | -5.382 | 1.00 | 41.29 | C |
| ATOM | 481 | O | ASP | C | 62 | -9.210 | 64.175 | -4.884 | 1.00 | 43.64 | O |
| ATOM | 482 | CB | ASP | C | 62 | -8.027 | 64.862 | -7.858 | 1.00 | 41.48 | C |
| ATOM | 483 | CG | ASP | C | 62 | -8.775 | 65.657 | -8.908 | 1.00 | 52.54 | C |
| ATOM | 484 | OD1 | ASP | C | 62 | -9.074 | 66.853 | -8.639 | 1.00 | 50.91 | O |
| ATOM | 485 | OD2 | ASP | C | 62 | -9.074 | 65.083 | -9.984 | 1.00 | 54.03 | O1- |
| ATOM | 486 | N | SER | C | 63 | -6.978 | 63.960 | -4.967 | 1.00 | 38.35 | N |
| ATOM | 487 | CA | SER | C | 63 | -6.931 | 62.918 | -3.948 | 1.00 | 40.77 | C |
| ATOM | 488 | C | SER | C | 63 | -7.429 | 63.398 | -2.589 | 1.00 | 41.73 | C |
| ATOM | 489 | O | SER | C | 63 | -7.557 | 62.586 | -1.666 | 1.00 | 37.97 | O |
| ATOM | 490 | CB | SER | C | 63 | -5.493 | 62.386 | -3.804 | 1.00 | 39.37 | C |
| ATOM | 491 | OG | SER | C | 63 | -4.582 | 63.445 | -3.547 | 1.00 | 39.82 | O |
| ATOM | 492 | N | VAL | C | 64 | -7.730 | 64.681 | -2.445 | 1.00 | 37.08 | N |
| ATOM | 493 | CA | VAL | C | 64 | -7.937 | 65.265 | -1.130 | 1.00 | 34.49 | C |
| ATOM | 494 | C | VAL | C | 64 | -9.234 | 66.090 | -1.105 | 1.00 | 35.91 | C |
| ATOM | 495 | O | VAL | C | 64 | -9.766 | 66.419 | -0.033 | 1.00 | 35.61 | O |

| | | | | | | | | | | | |
|---|---|---|---|---|---|---|---|---|---|---|---|
| ATOM | 496 | CB | VAL | C | 64 | -6.652 | 66.052 | -0.800 | 1.00 | 37.75 | C |
| ATOM | 497 | CG1 | VAL | C | 64 | -6.895 | 67.438 | -0.272 | 1.00 | 34.28 | C |
| ATOM | 498 | CG2 | VAL | C | 64 | -5.734 | 65.214 | 0.078 | 1.00 | 37.52 | C |
| ATOM | 499 | N | LYS | C | 65 | -9.774 | 66.389 | -2.294 | 1.00 | 33.05 | N |
| ATOM | 500 | CA | LYS | C | 65 | -11.007 | 67.169 | -2.424 | 1.00 | 35.12 | C |
| ATOM | 501 | C | LYS | C | 65 | -12.143 | 66.581 | -1.599 | 1.00 | 41.45 | C |
| ATOM | 502 | O | LYS | C | 65 | -12.378 | 65.368 | -1.600 | 1.00 | 37.85 | O |
| ATOM | 503 | CB | LYS | C | 65 | -11.463 | 67.244 | -3.888 | 1.00 | 35.87 | C |
| ATOM | 504 | CG | LYS | C | 65 | -10.771 | 68.290 | -4.714 | 1.00 | 35.54 | C |
| ATOM | 505 | CD | LYS | C | 65 | -11.454 | 68.428 | -6.057 | 1.00 | 42.42 | C |
| ATOM | 506 | CE | LYS | C | 65 | -10.975 | 69.680 | -6.795 | 1.00 | 38.45 | C |
| ATOM | 507 | NZ | LYS | C | 65 | -9.588 | 69.479 | -7.281 | 1.00 | 40.01 | N1+ |
| ATOM | 508 | N | GLY | C | 66 | -12.876 | 67.460 | -0.925 | 1.00 | 38.42 | N |
| ATOM | 509 | CA | GLY | C | 66 | -14.008 | 67.048 | -0.131 | 1.00 | 34.79 | C |
| ATOM | 510 | C | GLY | C | 66 | -13.656 | 66.580 | 1.252 | 1.00 | 43.32 | C |
| ATOM | 511 | O | GLY | C | 66 | -14.504 | 66.628 | 2.139 | 1.00 | 44.56 | O |
| ATOM | 512 | N | ARG | C | 67 | -12.416 | 66.177 | 1.488 | 1.00 | 42.85 | N |
| ATOM | 513 | CA | ARG | C | 67 | -12.040 | 65.681 | 2.798 | 1.00 | 33.59 | C |
| ATOM | 514 | C | ARG | C | 67 | -11.185 | 66.653 | 3.587 | 1.00 | 38.28 | C |
| ATOM | 515 | O | ARG | C | 67 | -11.308 | 66.698 | 4.812 | 1.00 | 35.39 | O |
| ATOM | 516 | CB | ARG | C | 67 | -11.317 | 64.333 | 2.663 | 1.00 | 38.43 | C |
| ATOM | 517 | CG | ARG | C | 67 | -12.241 | 63.220 | 2.171 | 1.00 | 33.48 | C |
| ATOM | 518 | CD | ARG | C | 67 | -11.540 | 61.860 | 2.172 | 1.00 | 39.76 | C |
| ATOM | 519 | NE | ARG | C | 67 | -10.284 | 61.891 | 1.423 | 1.00 | 38.65 | N |
| ATOM | 520 | CZ | ARG | C | 67 | -9.081 | 61.717 | 1.967 | 1.00 | 39.28 | C |
| ATOM | 521 | NH1 | ARG | C | 67 | -8.981 | 61.482 | 3.259 | 1.00 | 31.68 | N1+ |
| ATOM | 522 | NH2 | ARG | C | 67 | -7.977 | 61.785 | 1.224 | 1.00 | 36.70 | N |
| ATOM | 523 | N | PHE | C | 68 | -10.304 | 67.413 | 2.922 | 1.00 | 37.96 | N |
| ATOM | 524 | CA | PHE | C | 68 | -9.395 | 68.344 | 3.576 | 1.00 | 30.65 | C |
| ATOM | 525 | C | PHE | C | 68 | -9.874 | 69.766 | 3.351 | 1.00 | 34.00 | C |
| ATOM | 526 | O | PHE | C | 68 | -10.488 | 70.071 | 2.325 | 1.00 | 36.05 | O |
| ATOM | 527 | CB | PHE | C | 68 | -7.959 | 68.224 | 3.047 | 1.00 | 37.02 | C |
| ATOM | 528 | CG | PHE | C | 68 | -7.264 | 66.900 | 3.360 | 1.00 | 38.71 | C |
| ATOM | 529 | CD1 | PHE | C | 68 | -7.954 | 65.814 | 3.888 | 1.00 | 35.33 | C |
| ATOM | 530 | CD2 | PHE | C | 68 | -5.894 | 66.767 | 3.140 | 1.00 | 37.81 | C |
| ATOM | 531 | CE1 | PHE | C | 68 | -7.299 | 64.615 | 4.164 | 1.00 | 36.61 | C |
| ATOM | 532 | CE2 | PHE | C | 68 | -5.218 | 65.569 | 3.425 | 1.00 | 33.40 | C |
| ATOM | 533 | CZ | PHE | C | 68 | -5.919 | 64.498 | 3.934 | 1.00 | 40.51 | C |
| ATOM | 534 | N | THR | C | 69 | -9.582 | 70.646 | 4.307 | 1.00 | 35.34 | N |
| ATOM | 535 | CA | THR | C | 69 | -9.924 | 72.055 | 4.163 | 1.00 | 29.08 | C |
| ATOM | 536 | C | THR | C | 69 | -8.743 | 72.900 | 4.621 | 1.00 | 31.52 | C |
| ATOM | 537 | O | THR | C | 69 | -8.229 | 72.710 | 5.725 | 1.00 | 33.87 | O |
| ATOM | 538 | CB | THR | C | 69 | -11.198 | 72.407 | 4.954 | 1.00 | 32.76 | C |
| ATOM | 539 | OG1 | THR | C | 69 | -12.294 | 71.663 | 4.427 | 1.00 | 36.96 | O |
| ATOM | 540 | CG2 | THR | C | 69 | -11.545 | 73.923 | 4.828 | 1.00 | 25.39 | C |
| ATOM | 541 | N | ILE | C | 70 | -8.309 | 73.827 | 3.778 | 1.00 | 33.09 | N |
| ATOM | 542 | CA | ILE | C | 70 | -7.201 | 74.720 | 4.113 | 1.00 | 29.02 | C |
| ATOM | 543 | C | ILE | C | 70 | -7.786 | 76.038 | 4.602 | 1.00 | 32.22 | C |
| ATOM | 544 | O | ILE | C | 70 | -8.810 | 76.503 | 4.093 | 1.00 | 33.11 | O |
| ATOM | 545 | CB | ILE | C | 70 | -6.265 | 74.911 | 2.899 | 1.00 | 35.27 | C |
| ATOM | 546 | CG1 | ILE | C | 70 | -4.942 | 75.579 | 3.288 | 1.00 | 31.47 | C |
| ATOM | 547 | CG2 | ILE | C | 70 | -6.939 | 75.726 | 1.800 | 1.00 | 23.76 | C |
| ATOM | 548 | CD1 | ILE | C | 70 | -3.965 | 75.590 | 2.138 | 1.00 | 31.12 | C |
| ATOM | 549 | N | SER | C | 71 | -7.174 | 76.618 | 5.627 | 1.00 | 35.69 | N |
| ATOM | 550 | CA | SER | C | 71 | -7.670 | 77.883 | 6.153 | 1.00 | 38.73 | C |
| ATOM | 551 | C | SER | C | 71 | -6.513 | 78.588 | 6.845 | 1.00 | 37.39 | C |
| ATOM | 552 | O | SER | C | 71 | -5.444 | 78.009 | 7.046 | 1.00 | 41.40 | O |
| ATOM | 553 | CB | SER | C | 71 | -8.849 | 77.655 | 7.100 | 1.00 | 37.80 | C |
| ATOM | 554 | OG | SER | C | 71 | -8.444 | 76.880 | 8.217 | 1.00 | 38.72 | O |
| ATOM | 555 | N | ARG | C | 72 | -6.726 | 79.852 | 7.204 | 1.00 | 35.68 | N |
| ATOM | 556 | CA | ARG | C | 72 | -5.674 | 80.600 | 7.876 | 1.00 | 36.89 | C |
| ATOM | 557 | C | ARG | C | 72 | -6.274 | 81.545 | 8.908 | 1.00 | 40.29 | C |
| ATOM | 558 | O | ARG | C | 72 | -7.421 | 81.973 | 8.791 | 1.00 | 38.24 | O |
| ATOM | 559 | CB | ARG | C | 72 | -4.825 | 81.385 | 6.864 | 1.00 | 35.87 | C |
| ATOM | 560 | CG | ARG | C | 72 | -5.636 | 82.390 | 6.057 | 1.00 | 34.72 | C |
| ATOM | 561 | CD | ARG | C | 72 | -4.828 | 82.983 | 4.903 | 1.00 | 30.54 | C |
| ATOM | 562 | NE | ARG | C | 72 | -3.674 | 83.722 | 5.397 | 1.00 | 39.63 | N |
| ATOM | 563 | CZ | ARG | C | 72 | -2.575 | 83.951 | 4.694 | 1.00 | 41.45 | C |
| ATOM | 564 | NH1 | ARG | C | 72 | -2.475 | 83.499 | 3.449 | 1.00 | 37.50 | N1+ |
| ATOM | 565 | NH2 | ARG | C | 72 | -1.579 | 84.643 | 5.240 | 1.00 | 38.82 | N |
| ATOM | 566 | N | ASP | C | 73 | -5.478 | 81.878 | 9.920 | 1.00 | 38.55 | N |

| | | | | | | | | | | | |
|---|---|---|---|---|---|---|---|---|---|---|---|
| ATOM | 567 | CA | ASP | C | 73 | -5.852 | 82.875 | 10.919 | 1.00 | 40.45 | C |
| ATOM | 568 | C | ASP | C | 73 | -4.704 | 83.871 | 11.015 | 1.00 | 44.31 | C |
| ATOM | 569 | O | ASP | C | 73 | -3.699 | 83.601 | 11.676 | 1.00 | 47.19 | O |
| ATOM | 570 | CB | ASP | C | 73 | -6.142 | 82.239 | 12.277 | 1.00 | 45.34 | C |
| ATOM | 571 | CG | ASP | C | 73 | -6.685 | 83.247 | 13.303 | 1.00 | 48.06 | C |
| ATOM | 572 | OD1 | ASP | C | 73 | -6.492 | 84.479 | 13.138 | 1.00 | 54.90 | O |
| ATOM | 573 | OD2 | ASP | C | 73 | -7.277 | 82.797 | 14.305 | 1.00 | 53.87 | O1- |
| ATOM | 574 | N | ASN | C | 74 | -4.865 | 85.028 | 10.374 | 1.00 | 39.84 | N |
| ATOM | 575 | CA | ASN | C | 74 | -3.778 | 85.991 | 10.341 | 1.00 | 43.99 | C |
| ATOM | 576 | C | ASN | C | 74 | -3.450 | 86.497 | 11.739 | 1.00 | 48.38 | C |
| ATOM | 577 | O | ASN | C | 74 | -2.282 | 86.767 | 12.040 | 1.00 | 55.63 | O |
| ATOM | 578 | CB | ASN | C | 74 | -4.126 | 87.136 | 9.385 | 1.00 | 39.05 | C |
| ATOM | 579 | CG | ASN | C | 74 | -3.974 | 86.735 | 7.922 | 1.00 | 46.52 | C |
| ATOM | 580 | OD1 | ASN | C | 74 | -3.620 | 85.591 | 7.606 | 1.00 | 45.49 | O |
| ATOM | 581 | ND2 | ASN | C | 74 | -4.238 | 87.668 | 7.024 | 1.00 | 41.67 | N |
| ATOM | 582 | N | SER | C | 75 | -4.444 | 86.570 | 12.626 | 1.00 | 52.35 | N |
| ATOM | 583 | CA | SER | C | 75 | -4.172 | 87.063 | 13.973 | 1.00 | 49.93 | C |
| ATOM | 584 | C | SER | C | 75 | -3.251 | 86.138 | 14.759 | 1.00 | 51.44 | C |
| ATOM | 585 | O | SER | C | 75 | -2.606 | 86.596 | 15.705 | 1.00 | 53.63 | O |
| ATOM | 586 | CB | SER | C | 75 | -5.480 | 87.277 | 14.735 | 1.00 | 45.73 | C |
| ATOM | 587 | OG | SER | C | 75 | -6.025 | 86.059 | 15.205 | 1.00 | 53.39 | O |
| ATOM | 588 | N | LYS | C | 76 | -3.158 | 84.860 | 14.387 | 1.00 | 49.51 | N |
| ATOM | 589 | CA | LYS | C | 76 | -2.235 | 83.925 | 15.018 | 1.00 | 43.14 | C |
| ATOM | 590 | C | LYS | C | 76 | -1.069 | 83.563 | 14.114 | 1.00 | 50.55 | C |
| ATOM | 591 | O | LYS | C | 76 | -0.325 | 82.632 | 14.440 | 1.00 | 45.43 | O |
| ATOM | 592 | CB | LYS | C | 76 | -2.951 | 82.633 | 15.422 | 1.00 | 45.66 | C |
| ATOM | 593 | CG | LYS | C | 76 | -4.185 | 82.802 | 16.282 | 1.00 | 49.74 | C |
| ATOM | 594 | CD | LYS | C | 76 | -4.789 | 81.447 | 16.612 | 1.00 | 50.31 | C |
| ATOM | 595 | CE | LYS | C | 76 | -6.149 | 81.588 | 17.303 | 1.00 | 56.50 | C |
| ATOM | 596 | NZ | LYS | C | 76 | -6.769 | 80.256 | 17.607 | 1.00 | 68.70 | N1+ |
| ATOM | 597 | N | ASN | C | 77 | -0.896 | 84.275 | 12.992 | 1.00 | 47.54 | N |
| ATOM | 598 | CA | ASN | C | 77 | 0.093 | 83.953 | 11.963 | 1.00 | 44.06 | C |
| ATOM | 599 | C | ASN | C | 77 | 0.174 | 82.458 | 11.667 | 1.00 | 45.22 | C |
| ATOM | 600 | O | ASN | C | 77 | 1.278 | 81.913 | 11.537 | 1.00 | 43.31 | O |
| ATOM | 601 | CB | ASN | C | 77 | 1.470 | 84.476 | 12.358 | 1.00 | 41.59 | C |
| ATOM | 602 | CG | ASN | C | 77 | 1.530 | 85.984 | 12.365 | 1.00 | 44.41 | C |
| ATOM | 603 | OD1 | ASN | C | 77 | 0.827 | 86.646 | 11.609 | 1.00 | 48.47 | O |
| ATOM | 604 | ND2 | ASN | C | 77 | 2.354 | 86.535 | 13.232 | 1.00 | 44.19 | N |
| ATOM | 605 | N | THR | C | 78 | -0.981 | 81.789 | 11.547 | 1.00 | 36.70 | N |
| ATOM | 606 | CA | THR | C | 78 | -1.021 | 80.336 | 11.472 | 1.00 | 40.83 | C |
| ATOM | 607 | C | THR | C | 78 | -1.861 | 79.905 | 10.280 | 1.00 | 44.26 | C |
| ATOM | 608 | O | THR | C | 78 | -2.909 | 80.500 | 10.003 | 1.00 | 37.17 | O |
| ATOM | 609 | CB | THR | C | 78 | -1.599 | 79.724 | 12.774 | 1.00 | 40.36 | C |
| ATOM | 610 | OG1 | THR | C | 78 | -0.819 | 80.156 | 13.888 | 1.00 | 45.18 | O |
| ATOM | 611 | CG2 | THR | C | 78 | -1.572 | 78.205 | 12.739 | 1.00 | 38.53 | C |
| ATOM | 612 | N | LEU | C | 79 | -1.365 | 78.886 | 9.574 | 1.00 | 39.56 | N |
| ATOM | 613 | CA | LEU | C | 79 | -2.067 | 78.174 | 8.513 | 1.00 | 33.74 | C |
| ATOM | 614 | C | LEU | C | 79 | -2.557 | 76.821 | 9.033 | 1.00 | 37.68 | C |
| ATOM | 615 | O | LEU | C | 79 | -1.875 | 76.162 | 9.822 | 1.00 | 39.25 | O |
| ATOM | 616 | CB | LEU | C | 79 | -1.133 | 77.969 | 7.307 | 1.00 | 40.59 | C |
| ATOM | 617 | CG | LEU | C | 79 | -1.615 | 77.032 | 6.178 | 1.00 | 40.09 | C |
| ATOM | 618 | CD1 | LEU | C | 79 | -2.574 | 77.739 | 5.277 | 1.00 | 28.75 | C |
| ATOM | 619 | CD2 | LEU | C | 79 | -0.476 | 76.386 | 5.372 | 1.00 | 31.11 | C |
| ATOM | 620 | N | TYR | C | 80 | -3.738 | 76.399 | 8.599 | 1.00 | 35.37 | N |
| ATOM | 621 | CA | TYR | C | 80 | -4.296 | 75.136 | 9.067 | 1.00 | 38.96 | C |
| ATOM | 622 | C | TYR | C | 80 | -4.592 | 74.197 | 7.909 | 1.00 | 36.52 | C |
| ATOM | 623 | O | TYR | C | 80 | -4.895 | 74.629 | 6.798 | 1.00 | 37.65 | O |
| ATOM | 624 | CB | TYR | C | 80 | -5.592 | 75.328 | 9.872 | 1.00 | 36.20 | C |
| ATOM | 625 | CG | TYR | C | 80 | -5.421 | 76.193 | 11.073 | 1.00 | 41.77 | C |
| ATOM | 626 | CD1 | TYR | C | 80 | -4.830 | 75.697 | 12.234 | 1.00 | 41.02 | C |
| ATOM | 627 | CD2 | TYR | C | 80 | -5.845 | 77.516 | 11.052 | 1.00 | 39.70 | C |
| ATOM | 628 | CE1 | TYR | C | 80 | -4.655 | 76.511 | 13.345 | 1.00 | 44.22 | C |
| ATOM | 629 | CE2 | TYR | C | 80 | -5.685 | 78.340 | 12.152 | 1.00 | 40.29 | C |
| ATOM | 630 | CZ | TYR | C | 80 | -5.089 | 77.837 | 13.297 | 1.00 | 49.44 | C |
| ATOM | 631 | OH | TYR | C | 80 | -4.938 | 78.666 | 14.388 | 1.00 | 54.21 | O |
| ATOM | 632 | N | LEU | C | 81 | -4.520 | 72.901 | 8.194 | 1.00 | 36.72 | N |
| ATOM | 633 | CA | LEU | C | 81 | -5.055 | 71.869 | 7.317 | 1.00 | 41.79 | C |
| ATOM | 634 | C | LEU | C | 81 | -5.970 | 70.979 | 8.151 | 1.00 | 40.31 | C |
| ATOM | 635 | O | LEU | C | 81 | -5.497 | 70.223 | 9.006 | 1.00 | 40.72 | O |
| ATOM | 636 | CB | LEU | C | 81 | -3.943 | 71.050 | 6.665 | 1.00 | 36.53 | C |
| ATOM | 637 | CG | LEU | C | 81 | -4.473 | 70.006 | 5.677 | 1.00 | 29.95 | C |

| | | | | | | | | | | | |
|---|---|---|---|---|---|---|---|---|---|---|---|
| ATOM | 638 | CD1 | LEU | C | 81 | -5.070 | 70.688 | 4.452 | 1.00 | 35.27 | C |
| ATOM | 639 | CD2 | LEU | C | 81 | -3.369 | 69.024 | 5.277 | 1.00 | 31.44 | C |
| ATOM | 640 | N | GLN | C | 82 | -7.274 | 71.101 | 7.926 | 1.00 | 38.93 | N |
| ATOM | 641 | CA | GLN | C | 82 | -8.277 | 70.254 | 8.566 | 1.00 | 35.95 | C |
| ATOM | 642 | C | GLN | C | 82 | -8.454 | 68.996 | 7.728 | 1.00 | 36.48 | C |
| ATOM | 643 | O | GLN | C | 82 | -8.834 | 69.081 | 6.557 | 1.00 | 41.11 | O |
| ATOM | 644 | CB | GLN | C | 82 | -9.606 | 71.009 | 8.718 | 1.00 | 32.87 | C |
| ATOM | 645 | CG | GLN | C | 82 | -10.720 | 70.190 | 9.383 | 1.00 | 27.13 | C |
| ATOM | 646 | CD | GLN | C | 82 | -10.339 | 69.723 | 10.783 | 1.00 | 41.21 | C |
| ATOM | 647 | OE1 | GLN | C | 82 | -9.875 | 70.511 | 11.621 | 1.00 | 41.00 | O |
| ATOM | 648 | NE2 | GLN | C | 82 | -10.466 | 68.418 | 11.018 | 1.00 | 37.49 | N |
| ATOM | 649 | N | MET | C | 83 | -8.136 | 67.839 | 8.304 | 1.00 | 36.04 | N |
| ATOM | 650 | CA | MET | C | 83 | -8.151 | 66.557 | 7.594 | 1.00 | 39.43 | C |
| ATOM | 651 | C | MET | C | 83 | -9.242 | 65.670 | 8.174 | 1.00 | 41.49 | C |
| ATOM | 652 | O | MET | C | 83 | -9.145 | 65.225 | 9.321 | 1.00 | 46.39 | O |
| ATOM | 653 | CB | MET | C | 83 | -6.791 | 65.854 | 7.669 | 1.00 | 42.85 | C |
| ATOM | 654 | CG | MET | C | 83 | -5.616 | 66.618 | 7.030 | 1.00 | 44.37 | C |
| ATOM | 655 | SD | MET | C | 83 | -4.047 | 65.719 | 7.166 | 1.00 | 45.89 | S |
| ATOM | 656 | CE | MET | C | 83 | -3.833 | 65.851 | 8.911 | 1.00 | 41.39 | C |
| ATOM | 657 | N | ASN | C | 84 | -10.284 | 65.432 | 7.400 | 1.00 | 37.96 | N |
| ATOM | 658 | CA | ASN | C | 84 | -11.368 | 64.560 | 7.811 | 1.00 | 38.76 | C |
| ATOM | 659 | C | ASN | C | 84 | -11.332 | 63.268 | 7.002 | 1.00 | 40.86 | C |
| ATOM | 660 | O | ASN | C | 84 | -10.744 | 63.205 | 5.918 | 1.00 | 40.04 | O |
| ATOM | 661 | CB | ASN | C | 84 | -12.715 | 65.273 | 7.649 | 1.00 | 33.01 | C |
| ATOM | 662 | CG | ASN | C | 84 | -12.807 | 66.525 | 8.498 | 1.00 | 37.92 | C |
| ATOM | 663 | OD1 | ASN | C | 84 | -12.179 | 66.617 | 9.548 | 1.00 | 46.79 | O |
| ATOM | 664 | ND2 | ASN | C | 84 | -13.617 | 67.480 | 8.071 | 1.00 | 40.77 | N |
| ATOM | 665 | N | SER | C | 85 | -11.956 | 62.226 | 7.547 | 1.00 | 43.43 | N |
| ATOM | 666 | CA | SER | C | 85 | -12.072 | 60.945 | 6.852 | 1.00 | 40.65 | C |
| ATOM | 667 | C | SER | C | 85 | -10.714 | 60.421 | 6.406 | 1.00 | 39.67 | C |
| ATOM | 668 | O | SER | C | 85 | -10.529 | 60.022 | 5.252 | 1.00 | 36.84 | O |
| ATOM | 669 | CB | SER | C | 85 | -13.010 | 61.055 | 5.654 | 1.00 | 38.73 | C |
| ATOM | 670 | OG | SER | C | 85 | -14.333 | 61.254 | 6.094 | 1.00 | 48.16 | O |
| ATOM | 671 | N | LEU | C | 86 | -9.757 | 60.421 | 7.331 | 1.00 | 38.73 | N |
| ATOM | 672 | CA | LEU | C | 86 | -8.390 | 60.056 | 6.977 | 1.00 | 37.86 | C |
| ATOM | 673 | C | LEU | C | 86 | -8.328 | 58.625 | 6.457 | 1.00 | 38.76 | C |
| ATOM | 674 | O | LEU | C | 86 | -9.063 | 57.749 | 6.909 | 1.00 | 43.94 | O |
| ATOM | 675 | CB | LEU | C | 86 | -7.471 | 60.241 | 8.184 | 1.00 | 39.02 | C |
| ATOM | 676 | CG | LEU | C | 86 | -7.098 | 61.711 | 8.395 | 1.00 | 38.85 | C |
| ATOM | 677 | CD1 | LEU | C | 86 | -6.467 | 61.983 | 9.765 | 1.00 | 37.48 | C |
| ATOM | 678 | CD2 | LEU | C | 86 | -6.157 | 62.137 | 7.290 | 1.00 | 41.68 | C |
| ATOM | 679 | N | ARG | C | 87 | -7.468 | 58.400 | 5.470 | 1.00 | 41.40 | N |
| ATOM | 680 | CA | ARG | C | 87 | -7.251 | 57.088 | 4.882 | 1.00 | 37.22 | C |
| ATOM | 681 | C | ARG | C | 87 | -5.782 | 56.695 | 5.005 | 1.00 | 43.89 | C |
| ATOM | 682 | O | ARG | C | 87 | -4.900 | 57.546 | 5.190 | 1.00 | 37.28 | O |
| ATOM | 683 | CB | ARG | C | 87 | -7.661 | 57.052 | 3.409 | 1.00 | 41.43 | C |
| ATOM | 684 | CG | ARG | C | 87 | -8.995 | 57.661 | 3.103 | 1.00 | 47.94 | C |
| ATOM | 685 | CD | ARG | C | 87 | -9.556 | 57.025 | 1.858 | 1.00 | 52.28 | C |
| ATOM | 686 | NE | ARG | C | 87 | -9.802 | 57.968 | 0.770 | 1.00 | 59.65 | N |
| ATOM | 687 | CZ | ARG | C | 87 | -10.945 | 58.629 | 0.592 | 1.00 | 56.84 | C |
| ATOM | 688 | NH1 | ARG | C | 87 | -11.947 | 58.483 | 1.459 | 1.00 | 62.30 | N1+ |
| ATOM | 689 | NH2 | ARG | C | 87 | -11.079 | 59.444 | -0.449 | 1.00 | 58.47 | N |
| ATOM | 690 | N | VAL | C | 88 | -5.536 | 55.384 | 4.900 | 1.00 | 37.13 | N |
| ATOM | 691 | CA | VAL | C | 88 | -4.184 | 54.851 | 5.067 | 1.00 | 40.40 | C |
| ATOM | 692 | C | VAL | C | 88 | -3.218 | 55.550 | 4.113 | 1.00 | 41.32 | C |
| ATOM | 693 | O | VAL | C | 88 | -2.142 | 56.021 | 4.510 | 1.00 | 39.20 | O |
| ATOM | 694 | CB | VAL | C | 88 | -4.191 | 53.320 | 4.863 | 1.00 | 38.65 | C |
| ATOM | 695 | CG1 | VAL | C | 88 | -2.774 | 52.764 | 4.702 | 1.00 | 37.78 | C |
| ATOM | 696 | CG2 | VAL | C | 88 | -4.883 | 52.634 | 6.045 | 1.00 | 35.60 | C |
| ATOM | 697 | N | GLU | C | 89 | -3.621 | 55.684 | 2.856 | 1.00 | 36.74 | N |
| ATOM | 698 | CA | GLU | C | 89 | -2.794 | 56.334 | 1.849 | 1.00 | 37.93 | C |
| ATOM | 699 | C | GLU | C | 89 | -2.629 | 57.845 | 2.082 | 1.00 | 39.72 | C |
| ATOM | 700 | O | GLU | C | 89 | -1.953 | 58.480 | 1.274 | 1.00 | 41.02 | O |
| ATOM | 701 | CB | GLU | C | 89 | -3.324 | 56.041 | 0.434 | 1.00 | 30.08 | C |
| ATOM | 702 | CG | GLU | C | 89 | -4.747 | 56.489 | 0.150 | 1.00 | 44.00 | C |
| ATOM | 703 | CD | GLU | C | 89 | -5.802 | 55.528 | 0.720 | 1.00 | 65.19 | C |
| ATOM | 704 | OE1 | GLU | C | 89 | -7.001 | 55.726 | 0.398 | 1.00 | 72.89 | O |
| ATOM | 705 | OE2 | GLU | C | 89 | -5.434 | 54.580 | 1.479 | 1.00 | 57.08 | O1- |
| ATOM | 706 | N | ASP | C | 90 | -3.261 | 58.456 | 3.092 | 1.00 | 34.66 | N |
| ATOM | 707 | CA | ASP | C | 90 | -2.892 | 59.824 | 3.461 | 1.00 | 35.63 | C |
| ATOM | 708 | C | ASP | C | 90 | -1.648 | 59.885 | 4.344 | 1.00 | 36.64 | C |

| | | | | | | | | | | | |
|---|---|---|---|---|---|---|---|---|---|---|---|
| ATOM | 709 | O | ASP | C | 90 | -1.213 | 60.994 | 4.704 | 1.00 | 34.03 | O |
| ATOM | 710 | CB | ASP | C | 90 | -4.032 | 60.545 | 4.200 | 1.00 | 37.08 | C |
| ATOM | 711 | CG | ASP | C | 90 | -5.265 | 60.761 | 3.338 | 1.00 | 41.53 | C |
| ATOM | 712 | OD1 | ASP | C | 90 | -5.126 | 61.135 | 2.135 | 1.00 | 39.64 | O |
| ATOM | 713 | OD2 | ASP | C | 90 | -6.380 | 60.541 | 3.882 | 1.00 | 36.67 | O1- |
| ATOM | 714 | N | THR | C | 91 | -1.085 | 58.733 | 4.713 | 1.00 | 35.16 | N |
| ATOM | 715 | CA | THR | C | 91 | 0.114 | 58.684 | 5.552 | 1.00 | 38.29 | C |
| ATOM | 716 | C | THR | C | 91 | 1.260 | 59.354 | 4.826 | 1.00 | 31.75 | C |
| ATOM | 717 | O | THR | C | 91 | 1.572 | 58.989 | 3.690 | 1.00 | 36.79 | O |
| ATOM | 718 | CB | THR | C | 91 | 0.484 | 57.225 | 5.857 | 1.00 | 49.62 | C |
| ATOM | 719 | OG1 | THR | C | 91 | -0.561 | 56.594 | 6.611 | 1.00 | 40.91 | O |
| ATOM | 720 | CG2 | THR | C | 91 | 1.824 | 57.138 | 6.602 | 1.00 | 35.10 | C |
| ATOM | 721 | N | ALA | C | 92 | 1.876 | 60.340 | 5.462 | 1.00 | 34.05 | N |
| ATOM | 722 | CA | ALA | C | 92 | 2.886 | 61.136 | 4.778 | 1.00 | 32.82 | C |
| ATOM | 723 | C | ALA | C | 92 | 3.495 | 62.111 | 5.760 | 1.00 | 30.84 | C |
| ATOM | 724 | O | ALA | C | 92 | 2.938 | 62.368 | 6.827 | 1.00 | 30.26 | O |
| ATOM | 725 | CB | ALA | C | 92 | 2.304 | 61.923 | 3.596 | 1.00 | 36.15 | C |
| ATOM | 726 | N | VAL | C | 93 | 4.648 | 62.657 | 5.382 | 1.00 | 35.57 | N |
| ATOM | 727 | CA | VAL | C | 93 | 5.111 | 63.910 | 5.967 | 1.00 | 35.12 | C |
| ATOM | 728 | C | VAL | C | 93 | 4.408 | 65.064 | 5.265 | 1.00 | 34.14 | C |
| ATOM | 729 | O | VAL | C | 93 | 4.322 | 65.099 | 4.032 | 1.00 | 35.63 | O |
| ATOM | 730 | CB | VAL | C | 93 | 6.633 | 64.038 | 5.857 | 1.00 | 34.90 | C |
| ATOM | 731 | CG1 | VAL | C | 93 | 7.077 | 65.442 | 6.323 | 1.00 | 28.59 | C |
| ATOM | 732 | CG2 | VAL | C | 93 | 7.297 | 62.925 | 6.680 | 1.00 | 31.28 | C |
| ATOM | 733 | N | TYR | C | 94 | 3.897 | 66.006 | 6.045 | 1.00 | 33.45 | N |
| ATOM | 734 | CA | TYR | C | 94 | 3.184 | 67.159 | 5.518 | 1.00 | 37.19 | C |
| ATOM | 735 | C | TYR | C | 94 | 4.043 | 68.395 | 5.707 | 1.00 | 36.22 | C |
| ATOM | 736 | O | TYR | C | 94 | 4.511 | 68.654 | 6.820 | 1.00 | 37.12 | O |
| ATOM | 737 | CB | TYR | C | 94 | 1.826 | 67.335 | 6.216 | 1.00 | 29.33 | C |
| ATOM | 738 | CG | TYR | C | 94 | 0.818 | 66.349 | 5.708 | 1.00 | 38.48 | C |
| ATOM | 739 | CD1 | TYR | C | 94 | 0.931 | 64.986 | 6.003 | 1.00 | 33.38 | C |
| ATOM | 740 | CD2 | TYR | C | 94 | -0.223 | 66.760 | 4.887 | 1.00 | 34.11 | C |
| ATOM | 741 | CE1 | TYR | C | 94 | 0.012 | 64.069 | 5.508 | 1.00 | 35.06 | C |
| ATOM | 742 | CE2 | TYR | C | 94 | -1.143 | 65.848 | 4.386 | 1.00 | 32.26 | C |
| ATOM | 743 | CZ | TYR | C | 94 | -1.033 | 64.510 | 4.706 | 1.00 | 31.31 | C |
| ATOM | 744 | OH | TYR | C | 94 | -1.955 | 63.614 | 4.184 | 1.00 | 29.76 | O |
| ATOM | 745 | N | TYR | C | 95 | 4.240 | 69.154 | 4.625 | 1.00 | 33.06 | N |
| ATOM | 746 | CA | TYR | C | 95 | 4.988 | 70.408 | 4.660 | 1.00 | 37.25 | C |
| ATOM | 747 | C | TYR | C | 95 | 4.091 | 71.583 | 4.292 | 1.00 | 38.95 | C |
| ATOM | 748 | O | TYR | C | 95 | 3.283 | 71.491 | 3.356 | 1.00 | 34.73 | O |
| ATOM | 749 | CB | TYR | C | 95 | 6.160 | 70.409 | 3.680 | 1.00 | 33.44 | C |
| ATOM | 750 | CG | TYR | C | 95 | 7.187 | 69.311 | 3.833 | 1.00 | 36.03 | C |
| ATOM | 751 | CD1 | TYR | C | 95 | 8.260 | 69.444 | 4.718 | 1.00 | 30.51 | C |
| ATOM | 752 | CD2 | TYR | C | 95 | 7.124 | 68.165 | 3.036 | 1.00 | 34.65 | C |
| ATOM | 753 | CE1 | TYR | C | 95 | 9.231 | 68.431 | 4.825 | 1.00 | 35.04 | C |
| ATOM | 754 | CE2 | TYR | C | 95 | 8.078 | 67.163 | 3.132 | 1.00 | 35.40 | C |
| ATOM | 755 | CZ | TYR | C | 95 | 9.124 | 67.295 | 4.023 | 1.00 | 36.07 | C |
| ATOM | 756 | OH | TYR | C | 95 | 10.054 | 66.281 | 4.097 | 1.00 | 43.55 | O |
| ATOM | 757 | N | CYS | C | 96 | 4.277 | 72.713 | 4.969 | 1.00 | 32.29 | N |
| ATOM | 758 | CA | CYS | C | 96 | 3.722 | 73.935 | 4.419 | 1.00 | 35.55 | C |
| ATOM | 759 | C | CYS | C | 96 | 4.819 | 74.668 | 3.674 | 1.00 | 34.71 | C |
| ATOM | 760 | O | CYS | C | 96 | 6.007 | 74.463 | 3.919 | 1.00 | 37.92 | O |
| ATOM | 761 | CB | CYS | C | 96 | 3.090 | 74.846 | 5.484 | 1.00 | 37.18 | C |
| ATOM | 762 | SG | CYS | C | 96 | 4.149 | 75.311 | 6.821 | 1.00 | 55.09 | S |
| ATOM | 763 | N | ALA | C | 97 | 4.391 | 75.493 | 2.720 | 1.00 | 37.06 | N |
| ATOM | 764 | CA | ALA | C | 97 | 5.252 | 76.388 | 1.965 | 1.00 | 33.86 | C |
| ATOM | 765 | C | ALA | C | 97 | 4.453 | 77.646 | 1.659 | 1.00 | 36.16 | C |
| ATOM | 766 | O | ALA | C | 97 | 3.224 | 77.620 | 1.638 | 1.00 | 37.96 | O |
| ATOM | 767 | CB | ALA | C | 97 | 5.744 | 75.736 | 0.667 | 1.00 | 31.03 | C |
| ATOM | 768 | N | ASN | C | 98 | 5.141 | 78.756 | 1.411 | 1.00 | 36.24 | N |
| ATOM | 769 | CA | ASN | C | 98 | 4.402 | 79.933 | 0.984 | 1.00 | 36.16 | C |
| ATOM | 770 | C | ASN | C | 98 | 4.643 | 80.154 | -0.505 | 1.00 | 33.85 | C |
| ATOM | 771 | O | ASN | C | 98 | 5.379 | 79.405 | -1.150 | 1.00 | 34.25 | O |
| ATOM | 772 | CB | ASN | C | 98 | 4.750 | 81.159 | 1.836 | 1.00 | 33.87 | C |
| ATOM | 773 | CG | ASN | C | 98 | 6.152 | 81.689 | 1.602 | 1.00 | 36.19 | C |
| ATOM | 774 | OD1 | ASN | C | 98 | 6.912 | 81.172 | 0.781 | 1.00 | 35.81 | O |
| ATOM | 775 | ND2 | ASN | C | 98 | 6.490 | 82.759 | 2.312 | 1.00 | 32.46 | N |
| ATOM | 776 | N | TRP | C | 99 | 4.001 | 81.179 | -1.060 | 1.00 | 29.34 | N |
| ATOM | 777 | CA | TRP | C | 99 | 4.253 | 81.534 | -2.451 | 1.00 | 32.42 | C |
| ATOM | 778 | C | TRP | C | 99 | 4.141 | 83.043 | -2.609 | 1.00 | 34.35 | C |
| ATOM | 779 | O | TRP | C | 99 | 3.294 | 83.677 | -1.978 | 1.00 | 36.87 | O |

| | | | | | | | | | | | |
|---|---|---|---|---|---|---|---|---|---|---|---|
| ATOM | 780 | CB | TRP | C | 99 | 3.301 | 80.808 | -3.409 | 1.00 | 31.13 | C |
| ATOM | 781 | CG | TRP | C | 99 | 1.832 | 81.172 | -3.311 | 1.00 | 38.60 | C |
| ATOM | 782 | CD1 | TRP | C | 99 | 0.869 | 80.545 | -2.558 | 1.00 | 36.35 | C |
| ATOM | 783 | CD2 | TRP | C | 99 | 1.160 | 82.217 | -4.028 | 1.00 | 33.13 | C |
| ATOM | 784 | NE1 | TRP | C | 99 | -0.359 | 81.151 | -2.754 | 1.00 | 32.28 | N |
| ATOM | 785 | CE2 | TRP | C | 99 | -0.207 | 82.175 | -3.653 | 1.00 | 36.97 | C |
| ATOM | 786 | CE3 | TRP | C | 99 | 1.580 | 83.187 | -4.949 | 1.00 | 33.81 | C |
| ATOM | 787 | CZ2 | TRP | C | 99 | -1.155 | 83.078 | -4.160 | 1.00 | 35.91 | C |
| ATOM | 788 | CZ3 | TRP | C | 99 | 0.639 | 84.078 | -5.459 | 1.00 | 33.25 | C |
| ATOM | 789 | CH2 | TRP | C | 99 | -0.712 | 84.016 | -5.059 | 1.00 | 34.18 | C |
| ATOM | 790 | N | TYR | C | 100 | 5.011 | 83.606 | -3.443 | 1.00 | 35.97 | N |
| ATOM | 791 | CA | TYR | C | 100 | 5.148 | 85.048 | -3.651 | 1.00 | 34.82 | C |
| ATOM | 792 | C | TYR | C | 100 | 4.693 | 85.512 | -5.022 | 1.00 | 35.71 | C |
| ATOM | 793 | O | TYR | C | 100 | 4.101 | 86.591 | -5.143 | 1.00 | 33.01 | O |
| ATOM | 794 | CB | TYR | C | 100 | 6.615 | 85.472 | -3.493 | 1.00 | 28.22 | C |
| ATOM | 795 | CG | TYR | C | 100 | 7.184 | 85.286 | -2.114 | 1.00 | 36.73 | C |
| ATOM | 796 | CD1 | TYR | C | 100 | 6.415 | 85.536 | -0.971 | 1.00 | 34.55 | C |
| ATOM | 797 | CD2 | TYR | C | 100 | 8.496 | 84.852 | -1.946 | 1.00 | 35.48 | C |
| ATOM | 798 | CE1 | TYR | C | 100 | 6.951 | 85.365 | 0.299 | 1.00 | 36.55 | C |
| ATOM | 799 | CE2 | TYR | C | 100 | 9.032 | 84.666 | -0.684 | 1.00 | 33.46 | C |
| ATOM | 800 | CZ | TYR | C | 100 | 8.263 | 84.929 | 0.430 | 1.00 | 36.36 | C |
| ATOM | 801 | OH | TYR | C | 100 | 8.813 | 84.749 | 1.674 | 1.00 | 36.68 | O |
| ATOM | 802 | N | TYR | C | 101 | 4.990 | 84.731 | -6.059 | 1.00 | 32.01 | N |
| ATOM | 803 | CA | TYR | C | 101 | 4.927 | 85.197 | -7.439 | 1.00 | 35.14 | C |
| ATOM | 804 | C | TYR | C | 101 | 3.758 | 84.539 | -8.159 | 1.00 | 31.46 | C |
| ATOM | 805 | O | TYR | C | 101 | 2.795 | 85.215 | -8.492 | 1.00 | 32.52 | O |
| ATOM | 806 | CB | TYR | C | 101 | 6.249 | 84.921 | -8.157 | 1.00 | 25.12 | C |
| ATOM | 807 | CG | TYR | C | 101 | 7.430 | 85.482 | -7.409 | 1.00 | 31.29 | C |
| ATOM | 808 | CD1 | TYR | C | 101 | 7.594 | 86.850 | -7.307 | 1.00 | 30.52 | C |
| ATOM | 809 | CD2 | TYR | C | 101 | 8.385 | 84.653 | -6.796 | 1.00 | 34.01 | C |
| ATOM | 810 | CE1 | TYR | C | 101 | 8.646 | 87.393 | -6.644 | 1.00 | 31.83 | C |
| ATOM | 811 | CE2 | TYR | C | 101 | 9.470 | 85.205 | -6.099 | 1.00 | 29.33 | C |
| ATOM | 812 | CZ | TYR | C | 101 | 9.586 | 86.589 | -6.042 | 1.00 | 34.04 | C |
| ATOM | 813 | OH | TYR | C | 101 | 10.613 | 87.238 | -5.396 | 1.00 | 38.85 | O |
| ATOM | 814 | N | TYR | C | 102 | 3.818 | 83.241 | -8.407 | 1.00 | 32.18 | N |
| ATOM | 815 | CA | TYR | C | 102 | 2.692 | 82.528 | -8.984 | 1.00 | 26.83 | C |
| ATOM | 816 | C | TYR | C | 102 | 2.185 | 81.474 | -8.000 | 1.00 | 34.57 | C |
| ATOM | 817 | O | TYR | C | 102 | 2.959 | 80.888 | -7.237 | 1.00 | 30.16 | O |
| ATOM | 818 | CB | TYR | C | 102 | 3.069 | 81.916 | -10.328 | 1.00 | 26.41 | C |
| ATOM | 819 | CG | TYR | C | 102 | 4.422 | 81.207 | -10.424 | 1.00 | 31.59 | C |
| ATOM | 820 | CD1 | TYR | C | 102 | 5.586 | 81.894 | -10.776 | 1.00 | 25.87 | C |
| ATOM | 821 | CD2 | TYR | C | 102 | 4.510 | 79.827 | -10.218 | 1.00 | 34.03 | C |
| ATOM | 822 | CE1 | TYR | C | 102 | 6.807 | 81.225 | -10.900 | 1.00 | 28.32 | C |
| ATOM | 823 | CE2 | TYR | C | 102 | 5.708 | 79.150 | -10.339 | 1.00 | 29.44 | C |
| ATOM | 824 | CZ | TYR | C | 102 | 6.856 | 79.842 | -10.677 | 1.00 | 37.60 | C |
| ATOM | 825 | OH | TYR | C | 102 | 8.030 | 79.125 | -10.804 | 1.00 | 31.27 | O |
| ATOM | 826 | N | TYR | C | 103 | 0.861 | 81.255 | -8.005 | 1.00 | 34.62 | N |
| ATOM | 827 | CA | TYR | C | 103 | 0.206 | 80.545 | -6.908 | 1.00 | 30.24 | C |
| ATOM | 828 | C | TYR | C | 103 | 0.561 | 79.069 | -6.860 | 1.00 | 33.90 | C |
| ATOM | 829 | O | TYR | C | 103 | 0.334 | 78.431 | -5.826 | 1.00 | 33.35 | O |
| ATOM | 830 | CB | TYR | C | 103 | -1.330 | 80.708 | -6.981 | 1.00 | 32.59 | C |
| ATOM | 831 | CG | TYR | C | 103 | -1.968 | 80.050 | -8.196 | 1.00 | 30.66 | C |
| ATOM | 832 | CD1 | TYR | C | 103 | -2.097 | 80.744 | -9.398 | 1.00 | 32.62 | C |
| ATOM | 833 | CD2 | TYR | C | 103 | -2.439 | 78.739 | -8.145 | 1.00 | 31.75 | C |
| ATOM | 834 | CE1 | TYR | C | 103 | -2.677 | 80.151 | -10.526 | 1.00 | 34.17 | C |
| ATOM | 835 | CE2 | TYR | C | 103 | -3.030 | 78.132 | -9.277 | 1.00 | 35.45 | C |
| ATOM | 836 | CZ | TYR | C | 103 | -3.140 | 78.851 | -10.460 | 1.00 | 38.01 | C |
| ATOM | 837 | OH | TYR | C | 103 | -3.707 | 78.286 | -11.585 | 1.00 | 43.93 | O |
| ATOM | 838 | N | TYR | C | 104 | 1.090 | 78.503 | -7.941 | 1.00 | 33.41 | N |
| ATOM | 839 | CA | TYR | C | 104 | 1.431 | 77.088 | -7.959 | 1.00 | 31.54 | C |
| ATOM | 840 | C | TYR | C | 104 | 2.921 | 76.846 | -7.775 | 1.00 | 35.80 | C |
| ATOM | 841 | O | TYR | C | 104 | 3.383 | 75.706 | -7.933 | 1.00 | 34.83 | O |
| ATOM | 842 | CB | TYR | C | 104 | 0.942 | 76.437 | -9.254 | 1.00 | 31.39 | C |
| ATOM | 843 | CG | TYR | C | 104 | 1.310 | 77.192 | -10.505 | 1.00 | 35.92 | C |
| ATOM | 844 | CD1 | TYR | C | 104 | 2.537 | 76.979 | -11.134 | 1.00 | 29.90 | C |
| ATOM | 845 | CD2 | TYR | C | 104 | 0.431 | 78.136 | -11.058 | 1.00 | 37.72 | C |
| ATOM | 846 | CE1 | TYR | C | 104 | 2.887 | 77.682 | -12.297 | 1.00 | 31.96 | C |
| ATOM | 847 | CE2 | TYR | C | 104 | 0.773 | 78.853 | -12.216 | 1.00 | 33.33 | C |
| ATOM | 848 | CZ | TYR | C | 104 | 1.997 | 78.611 | -12.832 | 1.00 | 37.08 | C |
| ATOM | 849 | OH | TYR | C | 104 | 2.322 | 79.290 | -13.988 | 1.00 | 38.85 | O |
| ATOM | 850 | N | GLY | C | 105 | 3.677 | 77.881 | -7.424 | 1.00 | 30.57 | N |

| | | | | | | | | | | | | |
|---|---|---|---|---|---|---|---|---|---|---|---|---|
| ATOM | 851 | CA | GLY | C | 105 | 5.069 | 77.729 | -7.046 | 1.00 | 31.29 | C |
| ATOM | 852 | C | GLY | C | 105 | 5.189 | 77.804 | -5.536 | 1.00 | 35.97 | C |
| ATOM | 853 | O | GLY | C | 105 | 4.381 | 78.439 | -4.880 | 1.00 | 39.78 | O |
| ATOM | 854 | N | MET | C | 106 | 6.174 | 77.104 | -4.983 | 1.00 | 35.79 | N |
| ATOM | 855 | CA | MET | C | 106 | 6.477 | 77.149 | -3.560 | 1.00 | 29.65 | C |
| ATOM | 856 | C | MET | C | 106 | 7.811 | 77.844 | -3.359 | 1.00 | 34.76 | C |
| ATOM | 857 | O | MET | C | 106 | 8.814 | 77.447 | -3.964 | 1.00 | 40.34 | O |
| ATOM | 858 | CB | MET | C | 106 | 6.531 | 75.749 | -2.967 | 1.00 | 33.58 | C |
| ATOM | 859 | CG | MET | C | 106 | 5.200 | 75.079 | -2.997 | 1.00 | 40.63 | C |
| ATOM | 860 | SD | MET | C | 106 | 5.175 | 73.827 | -4.259 | 1.00 | 38.99 | S |
| ATOM | 861 | CE | MET | C | 106 | 3.449 | 73.894 | -4.753 | 1.00 | 39.97 | C |
| ATOM | 862 | N | ASP | C | 107 | 7.833 | 78.872 | -2.510 | 1.00 | 32.14 | N |
| ATOM | 863 | CA | ASP | C | 107 | 9.038 | 79.679 | -2.359 | 1.00 | 36.28 | C |
| ATOM | 864 | C | ASP | C | 107 | 9.814 | 79.363 | -1.087 | 1.00 | 36.05 | C |
| ATOM | 865 | O | ASP | C | 107 | 10.987 | 79.000 | -1.168 | 1.00 | 43.22 | O |
| ATOM | 866 | CB | ASP | C | 107 | 8.684 | 81.166 | -2.419 | 1.00 | 35.85 | C |
| ATOM | 867 | CG | ASP | C | 107 | 8.243 | 81.582 | -3.791 | 1.00 | 36.58 | C |
| ATOM | 868 | OD1 | ASP | C | 107 | 9.126 | 81.733 | -4.663 | 1.00 | 39.74 | O1- |
| ATOM | 869 | OD2 | ASP | C | 107 | 7.024 | 81.741 | -4.005 | 1.00 | 38.36 | O |
| ATOM | 870 | N | VAL | C | 108 | 9.185 | 79.486 | 0.079 | 1.00 | 34.27 | N |
| ATOM | 871 | CA | VAL | C | 108 | 9.795 | 79.131 | 1.354 | 1.00 | 37.63 | C |
| ATOM | 872 | C | VAL | C | 108 | 9.081 | 77.906 | 1.893 | 1.00 | 35.71 | C |
| ATOM | 873 | O | VAL | C | 108 | 7.853 | 77.818 | 1.820 | 1.00 | 36.94 | O |
| ATOM | 874 | CB | VAL | C | 108 | 9.716 | 80.275 | 2.375 | 1.00 | 39.13 | C |
| ATOM | 875 | CG1 | VAL | C | 108 | 10.395 | 79.854 | 3.662 | 1.00 | 34.94 | C |
| ATOM | 876 | CG2 | VAL | C | 108 | 10.334 | 81.534 | 1.809 | 1.00 | 37.63 | C |
| ATOM | 877 | N | TRP | C | 109 | 9.845 | 76.969 | 2.442 | 1.00 | 35.86 | N |
| ATOM | 878 | CA | TRP | C | 109 | 9.305 | 75.713 | 2.950 | 1.00 | 38.43 | C |
| ATOM | 879 | C | TRP | C | 109 | 9.523 | 75.578 | 4.452 | 1.00 | 36.07 | C |
| ATOM | 880 | O | TRP | C | 109 | 10.470 | 76.127 | 5.011 | 1.00 | 34.83 | O |
| ATOM | 881 | CB | TRP | C | 109 | 9.935 | 74.510 | 2.270 | 1.00 | 28.64 | C |
| ATOM | 882 | CG | TRP | C | 109 | 9.627 | 74.379 | 0.834 | 1.00 | 32.20 | C |
| ATOM | 883 | CD1 | TRP | C | 109 | 10.053 | 75.196 | -0.178 | 1.00 | 33.19 | C |
| ATOM | 884 | CD2 | TRP | C | 109 | 8.857 | 73.338 | 0.215 | 1.00 | 30.88 | C |
| ATOM | 885 | NE1 | TRP | C | 109 | 9.589 | 74.729 | -1.385 | 1.00 | 32.70 | N |
| ATOM | 886 | CE2 | TRP | C | 109 | 8.853 | 73.589 | -1.171 | 1.00 | 32.73 | C |
| ATOM | 887 | CE3 | TRP | C | 109 | 8.175 | 72.219 | 0.696 | 1.00 | 31.39 | C |
| ATOM | 888 | CZ2 | TRP | C | 109 | 8.183 | 72.759 | -2.079 | 1.00 | 27.14 | C |
| ATOM | 889 | CZ3 | TRP | C | 109 | 7.510 | 71.395 | -0.205 | 1.00 | 25.68 | C |
| ATOM | 890 | CH2 | TRP | C | 109 | 7.532 | 71.661 | -1.575 | 1.00 | 25.11 | C |
| ATOM | 891 | N | GLY | C | 110 | 8.611 | 74.852 | 5.095 | 1.00 | 38.56 | N |
| ATOM | 892 | CA | GLY | C | 110 | 8.732 | 74.483 | 6.489 | 1.00 | 37.65 | C |
| ATOM | 893 | C | GLY | C | 110 | 9.578 | 73.243 | 6.689 | 1.00 | 38.90 | C |
| ATOM | 894 | O | GLY | C | 110 | 10.348 | 72.825 | 5.821 | 1.00 | 44.35 | O |
| ATOM | 895 | N | GLN | C | 111 | 9.417 | 72.638 | 7.864 | 1.00 | 37.08 | N |
| ATOM | 896 | CA | GLN | C | 111 | 10.257 | 71.526 | 8.265 | 1.00 | 37.72 | C |
| ATOM | 897 | C | GLN | C | 111 | 9.554 | 70.171 | 8.274 | 1.00 | 39.10 | C |
| ATOM | 898 | O | GLN | C | 111 | 10.241 | 69.143 | 8.268 | 1.00 | 46.62 | O |
| ATOM | 899 | CB | GLN | C | 111 | 10.854 | 71.823 | 9.646 | 1.00 | 41.49 | C |
| ATOM | 900 | CG | GLN | C | 111 | 9.971 | 71.407 | 10.795 | 1.00 | 57.57 | C |
| ATOM | 901 | CD | GLN | C | 111 | 10.317 | 72.142 | 12.083 | 1.00 | 71.42 | C |
| ATOM | 902 | OE1 | GLN | C | 111 | 11.162 | 73.046 | 12.079 | 1.00 | 67.32 | O |
| ATOM | 903 | NE2 | GLN | C | 111 | 9.662 | 71.761 | 13.194 | 1.00 | 62.29 | N |
| ATOM | 904 | N | GLY | C | 112 | 8.229 | 70.137 | 8.247 | 1.00 | 38.85 | N |
| ATOM | 905 | CA | GLY | C | 112 | 7.483 | 68.892 | 8.185 | 1.00 | 37.13 | C |
| ATOM | 906 | C | GLY | C | 112 | 6.892 | 68.477 | 9.528 | 1.00 | 40.61 | C |
| ATOM | 907 | O | GLY | C | 112 | 7.389 | 68.837 | 10.601 | 1.00 | 43.71 | O |
| ATOM | 908 | N | THR | C | 113 | 5.771 | 67.754 | 9.462 | 1.00 | 38.16 | N |
| ATOM | 909 | CA | THR | C | 113 | 5.125 | 67.066 | 10.574 | 1.00 | 37.93 | C |
| ATOM | 910 | C | THR | C | 113 | 4.592 | 65.777 | 9.967 | 1.00 | 40.18 | C |
| ATOM | 911 | O | THR | C | 113 | 4.106 | 65.791 | 8.834 | 1.00 | 43.97 | O |
| ATOM | 912 | CB | THR | C | 113 | 4.008 | 67.924 | 11.231 | 1.00 | 40.04 | C |
| ATOM | 913 | OG1 | THR | C | 113 | 3.470 | 67.248 | 12.370 | 1.00 | 48.01 | O |
| ATOM | 914 | CG2 | THR | C | 113 | 2.863 | 68.248 | 10.265 | 1.00 | 39.86 | C |
| ATOM | 915 | N | ATHR | C | 114 | 4.716 | 64.665 | 10.689 | 0.50 | 39.81 | N |
| ATOM | 916 | CA | ATHR | C | 114 | 4.418 | 63.359 | 10.110 | 0.50 | 39.05 | C |
| ATOM | 917 | C | ATHR | C | 114 | 3.038 | 62.873 | 10.545 | 0.50 | 39.83 | C |
| ATOM | 918 | O | ATHR | C | 114 | 2.650 | 63.015 | 11.709 | 0.50 | 39.84 | O |
| ATOM | 919 | CB | ATHR | C | 114 | 5.484 | 62.320 | 10.487 | 0.50 | 39.38 | C |
| ATOM | 920 | OG1 | ATHR | C | 114 | 5.310 | 61.937 | 11.853 | 0.50 | 49.44 | O |
| ATOM | 921 | CG2 | ATHR | C | 114 | 6.901 | 62.878 | 10.295 | 0.50 | 34.14 | C |

| | | | | | | | | | | | |
|---|---|---|---|---|---|---|---|---|---|---|---|
| ATOM | 922 | N | BTHR | C | 114 | 4.722 | 64.656 | 10.669 | 0.50 | 39.81 | N |
| ATOM | 923 | CA | BTHR | C | 114 | 4.413 | 63.376 | 10.040 | 0.50 | 39.04 | C |
| ATOM | 924 | C | BTHR | C | 114 | 3.083 | 62.819 | 10.539 | 0.50 | 39.82 | C |
| ATOM | 925 | O | BTHR | C | 114 | 2.766 | 62.880 | 11.731 | 0.50 | 39.90 | O |
| ATOM | 926 | CB | BTHR | C | 114 | 5.554 | 62.360 | 10.232 | 0.50 | 39.16 | C |
| ATOM | 927 | OG1 | BTHR | C | 114 | 5.037 | 61.021 | 10.248 | 0.50 | 33.47 | O |
| ATOM | 928 | CG2 | BTHR | C | 114 | 6.343 | 62.647 | 11.490 | 0.50 | 42.84 | C |
| ATOM | 929 | N | VAL | C | 115 | 2.298 | 62.308 | 9.592 | 1.00 | 38.42 | N |
| ATOM | 930 | CA | VAL | C | 115 | 0.962 | 61.778 | 9.817 | 1.00 | 36.25 | C |
| ATOM | 931 | C | VAL | C | 115 | 0.968 | 60.298 | 9.446 | 1.00 | 37.55 | C |
| ATOM | 932 | O | VAL | C | 115 | 1.310 | 59.940 | 8.313 | 1.00 | 39.55 | O |
| ATOM | 933 | CB | VAL | C | 115 | -0.092 | 62.536 | 8.991 | 1.00 | 37.32 | C |
| ATOM | 934 | CG1 | VAL | C | 115 | -1.443 | 61.798 | 9.049 | 1.00 | 33.92 | C |
| ATOM | 935 | CG2 | VAL | C | 115 | -0.223 | 63.961 | 9.492 | 1.00 | 31.70 | C |
| ATOM | 936 | N | THR | C | 116 | 0.580 | 59.443 | 10.388 | 1.00 | 35.49 | N |
| ATOM | 937 | CA | THR | C | 116 | 0.471 | 58.014 | 10.145 | 1.00 | 38.36 | C |
| ATOM | 938 | C | THR | C | 116 | -0.987 | 57.603 | 10.309 | 1.00 | 43.76 | C |
| ATOM | 939 | O | THR | C | 116 | -1.596 | 57.873 | 11.350 | 1.00 | 43.30 | O |
| ATOM | 940 | CB | THR | C | 116 | 1.379 | 57.223 | 11.096 | 1.00 | 45.91 | C |
| ATOM | 941 | OG1 | THR | C | 116 | 2.734 | 57.660 | 10.937 | 1.00 | 47.40 | O |
| ATOM | 942 | CG2 | THR | C | 116 | 1.342 | 55.751 | 10.742 | 1.00 | 42.59 | C |
| ATOM | 943 | N | VAL | C | 117 | -1.553 | 56.977 | 9.283 | 1.00 | 36.12 | N |
| ATOM | 944 | CA | VAL | C | 117 | -2.926 | 56.501 | 9.346 | 1.00 | 41.60 | C |
| ATOM | 945 | C | VAL | C | 117 | -2.891 | 54.978 | 9.276 | 1.00 | 45.74 | C |
| ATOM | 946 | O | VAL | C | 117 | -2.634 | 54.394 | 8.211 | 1.00 | 44.01 | O |
| ATOM | 947 | CB | VAL | C | 117 | -3.813 | 57.107 | 8.244 | 1.00 | 42.69 | C |
| ATOM | 948 | CG1 | VAL | C | 117 | -5.257 | 56.664 | 8.437 | 1.00 | 34.13 | C |
| ATOM | 949 | CG2 | VAL | C | 117 | -3.720 | 58.666 | 8.235 | 1.00 | 38.65 | C |
| ATOM | 950 | N | SER | C | 118 | -3.181 | 54.330 | 10.407 | 1.00 | 42.18 | N |
| ATOM | 951 | CA | SER | C | 118 | -3.101 | 52.882 | 10.500 | 1.00 | 45.65 | C |
| ATOM | 952 | C | SER | C | 118 | -4.244 | 52.329 | 11.345 | 1.00 | 46.76 | C |
| ATOM | 953 | O | SER | C | 118 | -4.596 | 52.891 | 12.389 | 1.00 | 43.45 | O |
| ATOM | 954 | CB | SER | C | 118 | -1.764 | 52.442 | 11.101 | 1.00 | 45.42 | C |
| ATOM | 955 | OG | SER | C | 118 | -1.694 | 51.028 | 11.142 | 1.00 | 53.46 | O |
| ATOM | 956 | N | SER | C | 119 | -4.800 | 51.204 | 10.901 | 1.00 | 39.96 | N |
| ATOM | 957 | CA | SER | C | 119 | -5.765 | 50.456 | 11.697 | 1.00 | 55.76 | C |
| ATOM | 958 | C | SER | C | 119 | -5.145 | 49.241 | 12.384 | 1.00 | 58.97 | C |
| ATOM | 959 | O | SER | C | 119 | -5.879 | 48.417 | 12.940 | 1.00 | 56.42 | O |
| ATOM | 960 | CB | SER | C | 119 | -6.956 | 50.024 | 10.833 | 1.00 | 47.59 | C |
| ATOM | 961 | OG | SER | C | 119 | -6.537 | 49.473 | 9.592 | 1.00 | 60.90 | O |
| ATOM | 962 | N | ALA | C | 120 | -3.821 | 49.092 | 12.325 | 1.00 | 53.37 | N |
| ATOM | 963 | CA | ALA | C | 120 | -3.157 | 47.942 | 12.923 | 1.00 | 49.27 | C |
| ATOM | 964 | C | ALA | C | 120 | -3.115 | 48.063 | 14.439 | 1.00 | 53.61 | C |
| ATOM | 965 | O | ALA | C | 120 | -3.088 | 49.160 | 15.007 | 1.00 | 51.80 | O |
| ATOM | 966 | CB | ALA | C | 120 | -1.736 | 47.799 | 12.376 | 1.00 | 54.97 | C |
| ATOM | 967 | N | SER | C | 121 | -3.101 | 46.918 | 15.104 | 1.00 | 50.81 | GZ00 N |
| ATOM | 968 | CA | SER | C | 121 | -2.922 | 46.921 | 16.546 | 1.00 | 62.62 | GZ00 C |
| ATOM | 969 | C | SER | C | 121 | -1.817 | 45.945 | 16.925 | 1.00 | 55.18 | GZ00 C |
| ATOM | 970 | O | SER | C | 121 | -1.492 | 45.015 | 16.175 | 1.00 | 50.22 | GZ00 O |
| ATOM | 971 | CB | SER | C | 121 | -4.226 | 46.571 | 17.271 | 1.00 | 60.87 | GZ00 C |
| ATOM | 972 | OG | SER | C | 121 | -4.902 | 45.539 | 16.576 | 1.00 | 67.02 | GZ00 O |
| ATOM | 973 | N | THR | C | 122 | -1.276 | 46.170 | 18.123 | 1.00 | 43.63 | GZ00 N |
| ATOM | 974 | CA | THR | C | 122 | -0.125 | 45.444 | 18.640 | 1.00 | 49.19 | GZ00 C |
| ATOM | 975 | C | THR | C | 122 | -0.197 | 43.955 | 18.342 | 1.00 | 48.67 | GZ00 C |
| ATOM | 976 | O | THR | C | 122 | -1.207 | 43.300 | 18.591 | 1.00 | 54.63 | GZ00 O |
| ATOM | 977 | CB | THR | C | 122 | -0.014 | 45.661 | 20.147 | 1.00 | 51.65 | GZ00 C |
| ATOM | 978 | OG1 | THR | C | 122 | 0.017 | 47.074 | 20.426 | 1.00 | 49.30 | GZ00 O |
| ATOM | 979 | CG2 | THR | C | 122 | 1.260 | 44.988 | 20.690 | 1.00 | 49.88 | GZ00 C |
| ATOM | 980 | N | LYS | C | 123 | 0.890 | 43.430 | 17.795 | 1.00 | 50.80 | GZ00 N |
| ATOM | 981 | CA | LYS | C | 123 | 0.976 | 42.015 | 17.485 | 1.00 | 49.10 | GZ00 C |
| ATOM | 982 | C | LYS | C | 123 | 2.446 | 41.622 | 17.506 | 1.00 | 56.41 | GZ00 C |
| ATOM | 983 | O | LYS | C | 123 | 3.290 | 42.358 | 16.985 | 1.00 | 49.74 | GZ00 O |
| ATOM | 984 | CB | LYS | C | 123 | 0.338 | 41.693 | 16.133 | 1.00 | 44.99 | GZ00 C |
| ATOM | 985 | CG | LYS | C | 123 | 0.499 | 40.238 | 15.807 | 1.00 | 44.28 | GZ00 C |
| ATOM | 986 | CD | LYS | C | 123 | -0.078 | 39.820 | 14.484 | 1.00 | 49.15 | GZ00 C |
| ATOM | 987 | CE | LYS | C | 123 | 0.361 | 38.368 | 14.217 | 1.00 | 62.00 | GZ00 C |
| ATOM | 988 | NZ | LYS | C | 123 | -0.182 | 37.795 | 12.951 | 1.00 | 72.41 | GZ00 N1+ |
| ATOM | 989 | N | GLY | C | 124 | 2.752 | 40.507 | 18.178 | 1.00 | 53.97 | GZ00 N |
| ATOM | 990 | CA | GLY | C | 124 | 4.094 | 39.983 | 18.217 | 1.00 | 39.15 | GZ00 C |
| ATOM | 991 | C | GLY | C | 124 | 4.382 | 39.194 | 16.962 | 1.00 | 43.80 | GZ00 C |
| ATOM | 992 | O | GLY | C | 124 | 3.474 | 38.686 | 16.297 | 1.00 | 46.60 | GZ00 O |

| | | | | | | | | | | | |
|---|---|---|---|---|---|---|---|---|---|---|---|
| ATOM | 993 | N | PRO | C | 125 | 5.658 | 39.060 | 16.625 | 1.00 | 47.07 | GZ00 N |
| ATOM | 994 | CA | PRO | C | 125 | 6.043 | 38.419 | 15.362 | 1.00 | 48.03 | GZ00 C |
| ATOM | 995 | C | PRO | C | 125 | 6.129 | 36.902 | 15.443 | 1.00 | 43.83 | GZ00 C |
| ATOM | 996 | O | PRO | C | 125 | 6.341 | 36.326 | 16.503 | 1.00 | 45.86 | GZ00 O |
| ATOM | 997 | CB | PRO | C | 125 | 7.440 | 38.999 | 15.095 | 1.00 | 45.07 | GZ00 C |
| ATOM | 998 | CG | PRO | C | 125 | 7.989 | 39.254 | 16.464 | 1.00 | 48.96 | GZ00 C |
| ATOM | 999 | CD | PRO | C | 125 | 6.800 | 39.689 | 17.314 | 1.00 | 46.04 | GZ00 C |
| ATOM | 1000 | N | SER | C | 126 | 5.963 | 36.270 | 14.280 | 1.00 | 42.83 | GZ00 N |
| ATOM | 1001 | CA | SER | C | 126 | 6.393 | 34.900 | 14.053 | 1.00 | 46.91 | GZ00 C |
| ATOM | 1002 | C | SER | C | 126 | 7.817 | 34.900 | 13.498 | 1.00 | 49.79 | GZ00 C |
| ATOM | 1003 | O | SER | C | 126 | 8.157 | 35.713 | 12.635 | 1.00 | 45.82 | GZ00 O |
| ATOM | 1004 | CB | SER | C | 126 | 5.461 | 34.192 | 13.071 | 1.00 | 50.64 | GZ00 C |
| ATOM | 1005 | OG | SER | C | 126 | 4.145 | 34.158 | 13.572 | 1.00 | 58.04 | GZ00 O |
| ATOM | 1006 | N | VAL | C | 127 | 8.645 | 33.980 | 13.986 | 1.00 | 49.68 | GZ00 N |
| ATOM | 1007 | CA | VAL | C | 127 | 10.059 | 33.915 | 13.624 | 1.00 | 45.00 | GZ00 C |
| ATOM | 1008 | C | VAL | C | 127 | 10.335 | 32.584 | 12.950 | 1.00 | 42.10 | GZ00 C |
| ATOM | 1009 | O | VAL | C | 127 | 10.021 | 31.528 | 13.504 | 1.00 | 57.42 | GZ00 O |
| ATOM | 1010 | CB | VAL | C | 127 | 10.958 | 34.110 | 14.855 | 1.00 | 44.31 | GZ00 C |
| ATOM | 1011 | CG1 | VAL | C | 127 | 12.431 | 34.119 | 14.456 | 1.00 | 47.62 | GZ00 C |
| ATOM | 1012 | CG2 | VAL | C | 127 | 10.577 | 35.406 | 15.554 | 1.00 | 41.45 | GZ00 C |
| ATOM | 1013 | N | PHE | C | 128 | 10.912 | 32.635 | 11.758 | 1.00 | 43.32 | GZ00 N |
| ATOM | 1014 | CA | PHE | C | 128 | 11.244 | 31.451 | 10.983 | 1.00 | 44.87 | GZ00 C |
| ATOM | 1015 | C | PHE | C | 128 | 12.729 | 31.447 | 10.629 | 1.00 | 50.79 | GZ00 C |
| ATOM | 1016 | O | PHE | C | 128 | 13.327 | 32.511 | 10.435 | 1.00 | 47.46 | GZ00 O |
| ATOM | 1017 | CB | PHE | C | 128 | 10.425 | 31.399 | 9.694 | 1.00 | 48.10 | GZ00 C |
| ATOM | 1018 | CG | PHE | C | 128 | 8.942 | 31.384 | 9.922 | 1.00 | 58.19 | GZ00 C |
| ATOM | 1019 | CD1 | PHE | C | 128 | 8.272 | 30.200 | 10.176 | 1.00 | 58.46 | GZ00 C |
| ATOM | 1020 | CD2 | PHE | C | 128 | 8.220 | 32.566 | 9.912 | 1.00 | 56.25 | GZ00 C |
| ATOM | 1021 | CE1 | PHE | C | 128 | 6.912 | 30.198 | 10.387 | 1.00 | 58.84 | GZ00 C |
| ATOM | 1022 | CE2 | PHE | C | 128 | 6.855 | 32.568 | 10.128 | 1.00 | 55.91 | GZ00 C |
| ATOM | 1023 | CZ | PHE | C | 128 | 6.205 | 31.387 | 10.358 | 1.00 | 55.09 | GZ00 C |
| ATOM | 1024 | N | PRO | C | 129 | 13.360 | 30.275 | 10.560 | 1.00 | 52.29 | GZ00 N |
| ATOM | 1025 | CA | PRO | C | 129 | 14.779 | 30.230 | 10.200 | 1.00 | 40.71 | GZ00 C |
| ATOM | 1026 | C | PRO | C | 129 | 14.975 | 30.363 | 8.705 | 1.00 | 44.60 | GZ00 C |
| ATOM | 1027 | O | PRO | C | 129 | 14.205 | 29.819 | 7.907 | 1.00 | 48.43 | GZ00 O |
| ATOM | 1028 | CB | PRO | C | 129 | 15.218 | 28.840 | 10.681 | 1.00 | 53.19 | GZ00 C |
| ATOM | 1029 | CG | PRO | C | 129 | 13.998 | 28.000 | 10.486 | 1.00 | 46.32 | GZ00 C |
| ATOM | 1030 | CD | PRO | C | 129 | 12.829 | 28.925 | 10.840 | 1.00 | 50.96 | GZ00 C |
| ATOM | 1031 | N | LEU | C | 130 | 16.017 | 31.101 | 8.330 | 1.00 | 39.68 | GZ00 N |
| ATOM | 1032 | CA | LEU | C | 130 | 16.463 | 31.190 | 6.947 | 1.00 | 38.79 | GZ00 C |
| ATOM | 1033 | C | LEU | C | 130 | 17.725 | 30.330 | 6.838 | 1.00 | 45.86 | GZ00 C |
| ATOM | 1034 | O | LEU | C | 130 | 18.834 | 30.778 | 7.150 | 1.00 | 44.51 | GZ00 O |
| ATOM | 1035 | CB | LEU | C | 130 | 16.699 | 32.647 | 6.568 | 1.00 | 41.81 | GZ00 C |
| ATOM | 1036 | CG | LEU | C | 130 | 15.436 | 33.491 | 6.795 | 1.00 | 45.70 | GZ00 C |
| ATOM | 1037 | CD1 | LEU | C | 130 | 15.639 | 34.968 | 6.444 | 1.00 | 40.24 | GZ00 C |
| ATOM | 1038 | CD2 | LEU | C | 130 | 14.275 | 32.910 | 6.003 | 1.00 | 44.72 | GZ00 C |
| ATOM | 1039 | N | ALA | C | 131 | 17.552 | 29.096 | 6.402 | 1.00 | 46.13 | GZ00 N |
| ATOM | 1040 | CA | ALA | C | 131 | 18.618 | 28.113 | 6.528 | 1.00 | 43.66 | GZ00 C |
| ATOM | 1041 | C | ALA | C | 131 | 19.672 | 28.301 | 5.441 | 1.00 | 44.63 | GZ00 C |
| ATOM | 1042 | O | ALA | C | 131 | 19.339 | 28.524 | 4.278 | 1.00 | 46.42 | GZ00 O |
| ATOM | 1043 | CB | ALA | C | 131 | 18.060 | 26.703 | 6.457 | 1.00 | 36.60 | GZ00 C |
| ATOM | 1044 | N | PRO | C | 132 | 20.943 | 28.159 | 5.792 | 1.00 | 50.91 | GZ00 N |
| ATOM | 1045 | CA | PRO | C | 132 | 22.009 | 28.205 | 4.784 | 1.00 | 52.92 | GZ00 C |
| ATOM | 1046 | C | PRO | C | 132 | 22.027 | 26.951 | 3.921 | 1.00 | 63.10 | GZ00 C |
| ATOM | 1047 | O | PRO | C | 132 | 21.557 | 25.876 | 4.315 | 1.00 | 65.83 | GZ00 O |
| ATOM | 1048 | CB | PRO | C | 132 | 23.279 | 28.301 | 5.631 | 1.00 | 51.34 | GZ00 C |
| ATOM | 1049 | CG | PRO | C | 132 | 22.910 | 27.548 | 6.901 | 1.00 | 47.48 | GZ00 C |
| ATOM | 1050 | CD | PRO | C | 132 | 21.454 | 27.876 | 7.145 | 1.00 | 49.36 | GZ00 C |
| ATOM | 1051 | N | SER | C | 133 | 22.606 | 27.103 | 2.724 | 1.00 | 65.77 | GZ00 N |
| ATOM | 1052 | CA | SER | C | 133 | 22.642 | 26.041 | 1.718 | 1.00 | 75.34 | GZ00 C |
| ATOM | 1053 | C | SER | C | 133 | 23.713 | 26.317 | 0.668 | 1.00 | 79.20 | GZ00 C |
| ATOM | 1054 | O | SER | C | 133 | 24.893 | 26.462 | 0.990 | 1.00 | 81.66 | GZ00 O |
| ATOM | 1055 | CB | SER | C | 133 | 21.272 | 25.900 | 1.043 | 1.00 | 74.63 | GZ00 C |
| ATOM | 1056 | OG | SER | C | 133 | 20.727 | 27.170 | 0.711 | 1.00 | 72.62 | GZ00 O |
| ATOM | 1057 | N | SER | C | 134 | 23.317 | 26.372 | -0.599 | 1.00 | 91.08 | GZ00 N |
| ATOM | 1058 | CA | SER | C | 134 | 24.136 | 27.032 | -1.612 | 1.00 | 84.25 | GZ00 C |
| ATOM | 1059 | C | SER | C | 134 | 23.720 | 28.503 | -1.692 | 1.00 | 85.59 | GZ00 C |
| ATOM | 1060 | O | SER | C | 134 | 23.292 | 29.031 | -2.718 | 1.00 | 89.34 | GZ00 O |
| ATOM | 1061 | CB | SER | C | 134 | 24.022 | 26.312 | -2.950 | 1.00 | 90.76 | GZ00 C |
| ATOM | 1062 | OG | SER | C | 134 | 24.604 | 25.016 | -2.867 | 1.00 | 71.01 | GZ00 O |
| ATOM | 1063 | N | SER | C | 136 | 23.766 | 29.125 | -0.514 | 1.00 | 87.58 | GZ00 N |

| | | | | | | | | | | | |
|---|---|---|---|---|---|---|---|---|---|---|---|
| ATOM | 1064 | CA | SER | C | 136 | 23.877 | 30.568 | -0.336 | 1.00 | 76.32 | GZ00 C |
| ATOM | 1065 | C | SER | C | 136 | 25.274 | 30.904 | 0.199 | 1.00 | 70.77 | GZ00 C |
| ATOM | 1066 | O | SER | C | 136 | 25.441 | 31.713 | 1.121 | 1.00 | 56.45 | GZ00 O |
| ATOM | 1067 | CB | SER | C | 136 | 22.777 | 31.098 | 0.588 | 1.00 | 64.27 | GZ00 C |
| ATOM | 1068 | OG | SER | C | 136 | 22.863 | 30.567 | 1.905 | 1.00 | 55.15 | GZ00 O |
| ATOM | 1069 | N | THR | C | 137 | 26.294 | 30.248 | -0.363 | 1.00 | 68.34 | GZ00 N |
| ATOM | 1070 | CA | THR | C | 137 | 27.686 | 30.490 | -0.008 | 1.00 | 69.91 | GZ00 C |
| ATOM | 1071 | C | THR | C | 137 | 28.416 | 31.190 | -1.157 | 1.00 | 67.19 | GZ00 C |
| ATOM | 1072 | O | THR | C | 137 | 28.180 | 30.891 | -2.332 | 1.00 | 69.81 | GZ00 O |
| ATOM | 1073 | CB | THR | C | 137 | 28.407 | 29.183 | 0.387 | 1.00 | 64.52 | GZ00 C |
| ATOM | 1074 | OG1 | THR | C | 137 | 29.096 | 28.626 | -0.739 | 1.00 | 77.04 | GZ00 O |
| ATOM | 1075 | CG2 | THR | C | 137 | 27.427 | 28.167 | 0.946 | 1.00 | 58.14 | GZ00 C |
| ATOM | 1076 | N | SER | C | 138 | 29.289 | 32.144 | -0.810 | 1.00 | 68.23 | GZ00 N |
| ATOM | 1077 | CA | SER | C | 138 | 30.030 | 32.950 | -1.784 | 1.00 | 66.23 | GZ00 C |
| ATOM | 1078 | C | SER | C | 138 | 31.526 | 32.891 | -1.454 | 1.00 | 65.41 | GZ00 C |
| ATOM | 1079 | O | SER | C | 138 | 32.054 | 33.661 | -0.629 | 1.00 | 62.93 | GZ00 O |
| ATOM | 1080 | CB | SER | C | 138 | 29.508 | 34.383 | -1.828 | 1.00 | 70.26 | GZ00 C |
| ATOM | 1081 | OG | SER | C | 138 | 29.183 | 34.732 | -3.162 | 1.00 | 77.29 | GZ00 O |
| ATOM | 1082 | N | GLY | C | 139 | 32.210 | 31.988 | -2.153 | 1.00 | 65.74 | GZ00 N |
| ATOM | 1083 | CA | GLY | C | 139 | 33.577 | 31.673 | -1.822 | 1.00 | 55.52 | GZ00 C |
| ATOM | 1084 | C | GLY | C | 139 | 33.581 | 30.952 | -0.495 | 1.00 | 52.92 | GZ00 C |
| ATOM | 1085 | O | GLY | C | 139 | 32.963 | 29.889 | -0.339 | 1.00 | 56.98 | GZ00 O |
| ATOM | 1086 | N | GLY | C | 140 | 34.241 | 31.550 | 0.483 | 1.00 | 39.11 | GZ00 N |
| ATOM | 1087 | CA | GLY | C | 140 | 34.394 | 30.917 | 1.767 | 1.00 | 37.84 | GZ00 C |
| ATOM | 1088 | C | GLY | C | 140 | 33.435 | 31.476 | 2.782 | 1.00 | 35.31 | GZ00 C |
| ATOM | 1089 | O | GLY | C | 140 | 33.686 | 31.410 | 3.987 | 1.00 | 35.90 | GZ00 O |
| ATOM | 1090 | N | THR | C | 141 | 32.312 | 31.990 | 2.314 | 1.00 | 30.60 | GZ00 N |
| ATOM | 1091 | CA | THR | C | 141 | 31.371 | 32.678 | 3.180 | 1.00 | 35.07 | GZ00 C |
| ATOM | 1092 | C | THR | C | 141 | 29.975 | 32.121 | 2.959 | 1.00 | 45.03 | GZ00 C |
| ATOM | 1093 | O | THR | C | 141 | 29.638 | 31.698 | 1.858 | 1.00 | 45.95 | GZ00 O |
| ATOM | 1094 | CB | THR | C | 141 | 31.469 | 34.196 | 2.898 | 1.00 | 34.50 | GZ00 C |
| ATOM | 1095 | OG1 | THR | C | 141 | 31.995 | 34.851 | 4.055 | 1.00 | 45.09 | GZ00 O |
| ATOM | 1096 | CG2 | THR | C | 141 | 30.182 | 34.807 | 2.446 | 1.00 | 40.84 | GZ00 C |
| ATOM | 1097 | N | ALA | C | 142 | 29.186 | 32.058 | 4.026 | 1.00 | 40.91 | GZ00 N |
| ATOM | 1098 | CA | ALA | C | 142 | 27.833 | 31.528 | 3.943 | 1.00 | 37.80 | GZ00 C |
| ATOM | 1099 | C | ALA | C | 142 | 26.893 | 32.457 | 4.683 | 1.00 | 34.33 | GZ00 C |
| ATOM | 1100 | O | ALA | C | 142 | 27.251 | 33.006 | 5.730 | 1.00 | 37.65 | GZ00 O |
| ATOM | 1101 | CB | ALA | C | 142 | 27.729 | 30.125 | 4.539 | 1.00 | 37.82 | GZ00 C |
| ATOM | 1102 | N | ALA | C | 143 | 25.685 | 32.608 | 4.148 | 1.00 | 31.94 | GZ00 N |
| ATOM | 1103 | CA | ALA | C | 143 | 24.649 | 33.451 | 4.731 | 1.00 | 31.30 | GZ00 C |
| ATOM | 1104 | C | ALA | C | 143 | 23.527 | 32.602 | 5.324 | 1.00 | 38.60 | GZ00 C |
| ATOM | 1105 | O | ALA | C | 143 | 23.079 | 31.624 | 4.715 | 1.00 | 36.49 | GZ00 O |
| ATOM | 1106 | CB | ALA | C | 143 | 24.062 | 34.401 | 3.689 | 1.00 | 33.71 | GZ00 C |
| ATOM | 1107 | N | LEU | C | 144 | 23.099 | 32.976 | 6.529 | 1.00 | 37.05 | GZ00 N |
| ATOM | 1108 | CA | LEU | C | 144 | 21.975 | 32.361 | 7.208 | 1.00 | 40.29 | GZ00 C |
| ATOM | 1109 | C | LEU | C | 144 | 21.227 | 33.458 | 7.957 | 1.00 | 40.87 | GZ00 C |
| ATOM | 1110 | O | LEU | C | 144 | 21.800 | 34.504 | 8.281 | 1.00 | 41.49 | GZ00 O |
| ATOM | 1111 | CB | LEU | C | 144 | 22.463 | 31.247 | 8.144 | 1.00 | 42.35 | GZ00 C |
| ATOM | 1112 | CG | LEU | C | 144 | 23.403 | 31.747 | 9.239 | 1.00 | 47.05 | GZ00 C |
| ATOM | 1113 | CD1 | LEU | C | 144 | 22.647 | 31.926 | 10.545 | 1.00 | 49.65 | GZ00 C |
| ATOM | 1114 | CD2 | LEU | C | 144 | 24.583 | 30.834 | 9.410 | 1.00 | 40.72 | GZ00 C |
| ATOM | 1115 | N | GLY | C | 145 | 19.949 | 33.219 | 8.252 | 1.00 | 37.19 | GZ00 N |
| ATOM | 1116 | CA | GLY | C | 145 | 19.181 | 34.289 | 8.851 | 1.00 | 39.70 | GZ00 C |
| ATOM | 1117 | C | GLY | C | 145 | 17.884 | 33.886 | 9.522 | 1.00 | 40.16 | GZ00 C |
| ATOM | 1118 | O | GLY | C | 145 | 17.574 | 32.702 | 9.685 | 1.00 | 44.79 | GZ00 O |
| ATOM | 1119 | N | CYS | C | 146 | 17.133 | 34.916 | 9.910 | 1.00 | 38.48 | GZ00 N |
| ATOM | 1120 | CA | CYS | C | 146 | 15.879 | 34.810 | 10.641 | 1.00 | 40.61 | GZ00 C |
| ATOM | 1121 | C | CYS | C | 146 | 14.846 | 35.719 | 9.998 | 1.00 | 48.21 | GZ00 C |
| ATOM | 1122 | O | CYS | C | 146 | 15.099 | 36.916 | 9.820 | 1.00 | 41.15 | GZ00 O |
| ATOM | 1123 | CB | CYS | C | 146 | 16.058 | 35.212 | 12.114 | 1.00 | 36.30 | GZ00 C |
| ATOM | 1124 | SG | CYS | C | 146 | 16.447 | 33.783 | 13.156 | 1.00 | 70.07 | GZ00 S |
| ATOM | 1125 | N | LEU | C | 147 | 13.679 | 35.163 | 9.688 | 1.00 | 47.77 | GZ00 N |
| ATOM | 1126 | CA | LEU | C | 147 | 12.549 | 35.928 | 9.168 | 1.00 | 37.79 | GZ00 C |
| ATOM | 1127 | C | LEU | C | 147 | 11.641 | 36.320 | 10.333 | 1.00 | 45.95 | GZ00 C |
| ATOM | 1128 | O | LEU | C | 147 | 11.217 | 35.458 | 11.112 | 1.00 | 45.78 | GZ00 O |
| ATOM | 1129 | CB | LEU | C | 147 | 11.786 | 35.101 | 8.140 | 1.00 | 39.44 | GZ00 C |
| ATOM | 1130 | CG | LEU | C | 147 | 10.457 | 35.637 | 7.600 | 1.00 | 42.29 | GZ00 C |
| ATOM | 1131 | CD1 | LEU | C | 147 | 10.605 | 37.010 | 6.924 | 1.00 | 35.22 | GZ00 C |
| ATOM | 1132 | CD2 | LEU | C | 147 | 9.902 | 34.623 | 6.626 | 1.00 | 41.37 | GZ00 C |
| ATOM | 1133 | N | VAL | C | 148 | 11.392 | 37.614 | 10.493 | 1.00 | 36.60 | GZ00 N |
| ATOM | 1134 | CA | VAL | C | 148 | 10.604 | 38.136 | 11.607 | 1.00 | 39.69 | GZ00 C |

| | | | | | | | | | | | |
|---|---|---|---|---|---|---|---|---|---|---|---|
| ATOM | 1135 | C | VAL | C | 148 | 9.309 | 38.707 | 11.027 | 1.00 | 47.33 | GZ00 C |
| ATOM | 1136 | O | VAL | C | 148 | 9.266 | 39.872 | 10.617 | 1.00 | 45.03 | GZ00 O |
| ATOM | 1137 | CB | VAL | C | 148 | 11.375 | 39.192 | 12.409 | 1.00 | 42.14 | GZ00 C |
| ATOM | 1138 | CG1 | VAL | C | 148 | 10.549 | 39.661 | 13.557 | 1.00 | 34.51 | GZ00 C |
| ATOM | 1139 | CG2 | VAL | C | 148 | 12.701 | 38.631 | 12.933 | 1.00 | 38.14 | GZ00 C |
| ATOM | 1140 | N | LYS | C | 149 | 8.226 | 37.923 | 11.045 | 1.00 | 47.26 | GZ00 N |
| ATOM | 1141 | CA | LYS | C | 149 | 7.039 | 38.205 | 10.244 | 1.00 | 49.61 | GZ00 C |
| ATOM | 1142 | C | LYS | C | 149 | 5.887 | 38.797 | 11.059 | 1.00 | 48.25 | GZ00 C |
| ATOM | 1143 | O | LYS | C | 149 | 5.698 | 38.455 | 12.228 | 1.00 | 50.60 | GZ00 O |
| ATOM | 1144 | CB | LYS | C | 149 | 6.563 | 36.933 | 9.547 | 1.00 | 49.65 | GZ00 C |
| ATOM | 1145 | CG | LYS | C | 149 | 6.317 | 37.131 | 8.074 | 1.00 | 60.63 | GZ00 C |
| ATOM | 1146 | CD | LYS | C | 149 | 5.120 | 36.330 | 7.579 | 1.00 | 59.80 | GZ00 C |
| ATOM | 1147 | CE | LYS | C | 149 | 5.405 | 34.849 | 7.597 | 1.00 | 63.78 | GZ00 C |
| ATOM | 1148 | NZ | LYS | C | 149 | 4.321 | 34.077 | 6.887 | 1.00 | 62.06 | GZ00 N1+ |
| ATOM | 1149 | N | ASP | C | 150 | 5.154 | 39.729 | 10.430 | 1.00 | 52.54 | GZ00 N |
| ATOM | 1150 | CA | ASP | C | 150 | 3.795 | 40.160 | 10.809 | 1.00 | 43.96 | GZ00 C |
| ATOM | 1151 | C | ASP | C | 150 | 3.707 | 40.710 | 12.236 | 1.00 | 45.35 | GZ00 C |
| ATOM | 1152 | O | ASP | C | 150 | 2.955 | 40.200 | 13.068 | 1.00 | 47.95 | GZ00 O |
| ATOM | 1153 | CB | ASP | C | 150 | 2.782 | 39.021 | 10.633 | 1.00 | 43.67 | GZ00 C |
| ATOM | 1154 | CG | ASP | C | 150 | 2.587 | 38.624 | 9.190 | 1.00 | 49.50 | GZ00 C |
| ATOM | 1155 | OD1 | ASP | C | 150 | 2.874 | 39.436 | 8.284 | 1.00 | 47.60 | GZ00 O |
| ATOM | 1156 | OD2 | ASP | C | 150 | 2.133 | 37.486 | 8.957 | 1.00 | 63.54 | GZ00 O1- |
| ATOM | 1157 | N | TYR | C | 151 | 4.437 | 41.792 | 12.498 | 1.00 | 38.78 | GZ00 N |
| ATOM | 1158 | CA | TYR | C | 151 | 4.395 | 42.435 | 13.802 | 1.00 | 42.10 | GZ00 C |
| ATOM | 1159 | C | TYR | C | 151 | 4.012 | 43.911 | 13.689 | 1.00 | 41.99 | GZ00 C |
| ATOM | 1160 | O | TYR | C | 151 | 4.115 | 44.534 | 12.628 | 1.00 | 46.82 | GZ00 O |
| ATOM | 1161 | CB | TYR | C | 151 | 5.751 | 42.308 | 14.529 | 1.00 | 46.66 | GZ00 C |
| ATOM | 1162 | CG | TYR | C | 151 | 6.892 | 43.080 | 13.890 | 1.00 | 45.23 | GZ00 C |
| ATOM | 1163 | CD1 | TYR | C | 151 | 7.665 | 42.517 | 12.871 | 1.00 | 43.89 | GZ00 C |
| ATOM | 1164 | CD2 | TYR | C | 151 | 7.206 | 44.366 | 14.317 | 1.00 | 45.36 | GZ00 C |
| ATOM | 1165 | CE1 | TYR | C | 151 | 8.726 | 43.223 | 12.280 | 1.00 | 43.44 | GZ00 C |
| ATOM | 1166 | CE2 | TYR | C | 151 | 8.259 | 45.082 | 13.739 | 1.00 | 52.49 | GZ00 C |
| ATOM | 1167 | CZ | TYR | C | 151 | 9.018 | 44.504 | 12.721 | 1.00 | 50.42 | GZ00 C |
| ATOM | 1168 | OH | TYR | C | 151 | 10.045 | 45.224 | 12.149 | 1.00 | 41.96 | GZ00 O |
| ATOM | 1169 | N | PHE | C | 152 | 3.617 | 44.474 | 14.824 | 1.00 | 40.95 | GZ00 N |
| ATOM | 1170 | CA | PHE | C | 152 | 3.224 | 45.868 | 14.926 | 1.00 | 45.04 | GZ00 C |
| ATOM | 1171 | C | PHE | C | 152 | 3.209 | 46.273 | 16.388 | 1.00 | 42.56 | GZ00 C |
| ATOM | 1172 | O | PHE | C | 152 | 2.802 | 45.496 | 17.235 | 1.00 | 45.01 | GZ00 O |
| ATOM | 1173 | CB | PHE | C | 152 | 1.847 | 46.113 | 14.294 | 1.00 | 47.75 | GZ00 C |
| ATOM | 1174 | CG | PHE | C | 152 | 1.465 | 47.558 | 14.260 | 1.00 | 48.38 | GZ00 C |
| ATOM | 1175 | CD1 | PHE | C | 152 | 0.824 | 48.148 | 15.339 | 1.00 | 48.32 | GZ00 C |
| ATOM | 1176 | CD2 | PHE | C | 152 | 1.783 | 48.342 | 13.167 | 1.00 | 51.92 | GZ00 C |
| ATOM | 1177 | CE1 | PHE | C | 152 | 0.510 | 49.493 | 15.322 | 1.00 | 46.78 | GZ00 C |
| ATOM | 1178 | CE2 | PHE | C | 152 | 1.451 | 49.693 | 13.144 | 1.00 | 49.11 | GZ00 C |
| ATOM | 1179 | CZ | PHE | C | 152 | 0.824 | 50.262 | 14.228 | 1.00 | 41.05 | GZ00 C |
| ATOM | 1180 | N | PRO | C | 153 | 3.698 | 47.479 | 16.702 | 1.00 | 42.68 | GZ00 N |
| ATOM | 1181 | CA | PRO | C | 153 | 4.419 | 48.428 | 15.848 | 1.00 | 52.96 | GZ00 C |
| ATOM | 1182 | C | PRO | C | 153 | 5.930 | 48.163 | 15.829 | 1.00 | 52.60 | GZ00 C |
| ATOM | 1183 | O | PRO | C | 153 | 6.398 | 47.155 | 16.353 | 1.00 | 54.71 | GZ00 O |
| ATOM | 1184 | CB | PRO | C | 153 | 4.152 | 49.766 | 16.532 | 1.00 | 43.17 | GZ00 C |
| ATOM | 1185 | CG | PRO | C | 153 | 4.224 | 49.393 | 17.978 | 1.00 | 39.59 | GZ00 C |
| ATOM | 1186 | CD | PRO | C | 153 | 3.545 | 48.016 | 18.065 | 1.00 | 40.76 | GZ00 C |
| ATOM | 1187 | N | GLU | C | 154 | 6.682 | 49.068 | 15.220 | 1.00 | 50.80 | GZ00 N |
| ATOM | 1188 | CA | GLU | C | 154 | 8.130 | 49.012 | 15.287 | 1.00 | 47.84 | GZ00 C |
| ATOM | 1189 | C | GLU | C | 154 | 8.530 | 49.382 | 16.706 | 1.00 | 45.65 | GZ00 C |
| ATOM | 1190 | O | GLU | C | 154 | 7.755 | 49.990 | 17.417 | 1.00 | 48.79 | GZ00 O |
| ATOM | 1191 | CB | GLU | C | 154 | 8.749 | 49.958 | 14.264 | 1.00 | 45.04 | GZ00 C |
| ATOM | 1192 | CG | GLU | C | 154 | 8.425 | 49.586 | 12.831 | 1.00 | 49.34 | GZ00 C |
| ATOM | 1193 | CD | GLU | C | 154 | 9.635 | 49.012 | 12.119 | 1.00 | 59.99 | GZ00 C |
| ATOM | 1194 | OE1 | GLU | C | 154 | 10.114 | 49.663 | 11.168 | 1.00 | 58.68 | GZ00 O |
| ATOM | 1195 | OE2 | GLU | C | 154 | 10.120 | 47.929 | 12.535 | 1.00 | 57.95 | GZ00 O1- |
| ATOM | 1196 | N | PRO | C | 155 | 9.737 | 49.006 | 17.137 | 1.00 | 50.54 | GZ00 N |
| ATOM | 1197 | CA | PRO | C | 155 | 10.755 | 48.195 | 16.472 | 1.00 | 44.50 | GZ00 C |
| ATOM | 1198 | C | PRO | C | 155 | 10.803 | 46.753 | 16.983 | 1.00 | 53.89 | GZ00 C |
| ATOM | 1199 | O | PRO | C | 155 | 10.174 | 46.436 | 17.999 | 1.00 | 55.35 | GZ00 O |
| ATOM | 1200 | CB | PRO | C | 155 | 12.031 | 48.921 | 16.845 | 1.00 | 44.74 | GZ00 C |
| ATOM | 1201 | CG | PRO | C | 155 | 11.773 | 49.333 | 18.257 | 1.00 | 44.99 | GZ00 C |
| ATOM | 1202 | CD | PRO | C | 155 | 10.279 | 49.641 | 18.350 | 1.00 | 39.64 | GZ00 C |
| ATOM | 1203 | N | VAL | C | 156 | 11.530 | 45.887 | 16.278 | 1.00 | 45.82 | GZ00 N |
| ATOM | 1204 | CA | VAL | C | 156 | 12.054 | 44.666 | 16.870 | 1.00 | 55.31 | GZ00 C |
| ATOM | 1205 | C | VAL | C | 156 | 13.562 | 44.814 | 16.945 | 1.00 | 52.03 | GZ00 C |

| | | | | | | | | | | | | |
|---|---|---|---|---|---|---|---|---|---|---|---|---|
| ATOM | 1206 | O | VAL | C | 156 | 14.183 | 45.530 | 16.153 | 1.00 | 55.45 | GZ00 | O |
| ATOM | 1207 | CB | VAL | C | 156 | 11.698 | 43.375 | 16.105 | 1.00 | 52.00 | GZ00 | C |
| ATOM | 1208 | CG1 | VAL | C | 156 | 10.204 | 43.139 | 16.092 | 1.00 | 51.44 | GZ00 | C |
| ATOM | 1209 | CG2 | VAL | C | 156 | 12.231 | 43.446 | 14.719 | 1.00 | 48.82 | GZ00 | C |
| ATOM | 1210 | N | THR | C | 157 | 14.153 | 44.146 | 17.919 | 1.00 | 53.96 | GZ00 | N |
| ATOM | 1211 | CA | THR | C | 157 | 15.596 | 44.007 | 17.989 | 1.00 | 54.98 | GZ00 | C |
| ATOM | 1212 | C | THR | C | 157 | 15.949 | 42.554 | 17.732 | 1.00 | 54.62 | GZ00 | C |
| ATOM | 1213 | O | THR | C | 157 | 15.221 | 41.644 | 18.142 | 1.00 | 54.87 | GZ00 | O |
| ATOM | 1214 | CB | THR | C | 157 | 16.140 | 44.458 | 19.341 | 1.00 | 45.77 | GZ00 | C |
| ATOM | 1215 | OG1 | THR | C | 157 | 15.663 | 43.570 | 20.361 | 1.00 | 57.07 | GZ00 | O |
| ATOM | 1216 | CG2 | THR | C | 157 | 15.691 | 45.877 | 19.627 | 1.00 | 51.52 | GZ00 | C |
| ATOM | 1217 | N | VAL | C | 158 | 17.042 | 42.345 | 17.015 | 1.00 | 51.30 | GZ00 | N |
| ATOM | 1218 | CA | VAL | C | 158 | 17.521 | 41.010 | 16.706 | 1.00 | 47.26 | GZ00 | C |
| ATOM | 1219 | C | VAL | C | 158 | 18.988 | 40.957 | 17.085 | 1.00 | 50.93 | GZ00 | C |
| ATOM | 1220 | O | VAL | C | 158 | 19.767 | 41.833 | 16.692 | 1.00 | 49.49 | GZ00 | O |
| ATOM | 1221 | CB | VAL | C | 158 | 17.343 | 40.660 | 15.219 | 1.00 | 46.28 | GZ00 | C |
| ATOM | 1222 | CG1 | VAL | C | 158 | 17.733 | 39.213 | 14.986 | 1.00 | 46.68 | GZ00 | C |
| ATOM | 1223 | CG2 | VAL | C | 158 | 15.911 | 40.951 | 14.752 | 1.00 | 43.16 | GZ00 | C |
| ATOM | 1224 | N | SER | C | 159 | 19.361 | 39.943 | 17.850 | 1.00 | 51.31 | GZ00 | N |
| ATOM | 1225 | CA | SER | C | 159 | 20.756 | 39.667 | 18.134 | 1.00 | 44.21 | GZ00 | C |
| ATOM | 1226 | C | SER | C | 159 | 21.001 | 38.215 | 17.769 | 1.00 | 45.69 | GZ00 | C |
| ATOM | 1227 | O | SER | C | 159 | 20.063 | 37.437 | 17.578 | 1.00 | 44.40 | GZ00 | O |
| ATOM | 1228 | CB | SER | C | 159 | 21.126 | 39.946 | 19.605 | 1.00 | 39.33 | GZ00 | C |
| ATOM | 1229 | OG | SER | C | 159 | 20.384 | 39.132 | 20.511 | 1.00 | 48.22 | GZ00 | O |
| ATOM | 1230 | N | TRP | C | 160 | 22.270 | 37.863 | 17.648 | 1.00 | 44.65 | GZ00 | N |
| ATOM | 1231 | CA | TRP | C | 160 | 22.671 | 36.505 | 17.332 | 1.00 | 38.87 | GZ00 | C |
| ATOM | 1232 | C | TRP | C | 160 | 23.533 | 35.984 | 18.468 | 1.00 | 43.64 | GZ00 | C |
| ATOM | 1233 | O | TRP | C | 160 | 24.351 | 36.729 | 19.025 | 1.00 | 42.33 | GZ00 | O |
| ATOM | 1234 | CB | TRP | C | 160 | 23.420 | 36.436 | 15.998 | 1.00 | 36.78 | GZ00 | C |
| ATOM | 1235 | CG | TRP | C | 160 | 22.520 | 36.662 | 14.845 | 1.00 | 41.44 | GZ00 | C |
| ATOM | 1236 | CD1 | TRP | C | 160 | 22.178 | 37.861 | 14.294 | 1.00 | 39.79 | GZ00 | C |
| ATOM | 1237 | CD2 | TRP | C | 160 | 21.786 | 35.664 | 14.123 | 1.00 | 40.51 | GZ00 | C |
| ATOM | 1238 | NE1 | TRP | C | 160 | 21.304 | 37.671 | 13.245 | 1.00 | 37.10 | GZ00 | N |
| ATOM | 1239 | CE2 | TRP | C | 160 | 21.038 | 36.335 | 13.125 | 1.00 | 36.83 | GZ00 | C |
| ATOM | 1240 | CE3 | TRP | C | 160 | 21.695 | 34.270 | 14.216 | 1.00 | 38.13 | GZ00 | C |
| ATOM | 1241 | CZ2 | TRP | C | 160 | 20.209 | 35.662 | 12.228 | 1.00 | 37.10 | GZ00 | C |
| ATOM | 1242 | CZ3 | TRP | C | 160 | 20.878 | 33.596 | 13.314 | 1.00 | 42.58 | GZ00 | C |
| ATOM | 1243 | CH2 | TRP | C | 160 | 20.142 | 34.294 | 12.336 | 1.00 | 43.28 | GZ00 | C |
| ATOM | 1244 | N | ASN | C | 161 | 23.291 | 34.718 | 18.825 | 1.00 | 41.18 | GZ00 | N |
| ATOM | 1245 | CA | ASN | C | 161 | 23.956 | 34.018 | 19.929 | 1.00 | 45.75 | GZ00 | C |
| ATOM | 1246 | C | ASN | C | 161 | 24.057 | 34.898 | 21.169 | 1.00 | 45.89 | GZ00 | C |
| ATOM | 1247 | O | ASN | C | 161 | 25.123 | 35.079 | 21.763 | 1.00 | 49.59 | GZ00 | O |
| ATOM | 1248 | CB | ASN | C | 161 | 25.315 | 33.479 | 19.496 | 1.00 | 36.85 | GZ00 | C |
| ATOM | 1249 | CG | ASN | C | 161 | 25.181 | 32.440 | 18.399 | 1.00 | 44.50 | GZ00 | C |
| ATOM | 1250 | OD1 | ASN | C | 161 | 24.067 | 32.010 | 18.062 | 1.00 | 46.14 | GZ00 | O |
| ATOM | 1251 | ND2 | ASN | C | 161 | 26.306 | 31.997 | 17.865 | 1.00 | 43.54 | GZ00 | N |
| ATOM | 1252 | N | SER | C | 162 | 22.918 | 35.503 | 21.508 | 1.00 | 46.88 | GZ00 | N |
| ATOM | 1253 | CA | SER | C | 162 | 22.740 | 36.290 | 22.727 | 1.00 | 43.45 | GZ00 | C |
| ATOM | 1254 | C | SER | C | 162 | 23.687 | 37.476 | 22.784 | 1.00 | 47.59 | GZ00 | C |
| ATOM | 1255 | O | SER | C | 162 | 24.110 | 37.883 | 23.866 | 1.00 | 49.00 | GZ00 | O |
| ATOM | 1256 | CB | SER | C | 162 | 22.879 | 35.412 | 23.972 | 1.00 | 38.83 | GZ00 | C |
| ATOM | 1257 | OG | SER | C | 162 | 22.029 | 34.280 | 23.821 | 1.00 | 48.75 | GZ00 | O |
| ATOM | 1258 | N | GLY | C | 163 | 24.028 | 38.040 | 21.622 | 1.00 | 48.38 | GZ00 | N |
| ATOM | 1259 | CA | GLY | C | 163 | 24.887 | 39.205 | 21.551 | 1.00 | 40.94 | GZ00 | C |
| ATOM | 1260 | C | GLY | C | 163 | 26.359 | 38.927 | 21.292 | 1.00 | 53.03 | GZ00 | C |
| ATOM | 1261 | O | GLY | C | 163 | 27.105 | 39.878 | 20.999 | 1.00 | 49.49 | GZ00 | O |
| ATOM | 1262 | N | ALA | C | 164 | 26.804 | 37.665 | 21.374 | 1.00 | 39.35 | GZ00 | N |
| ATOM | 1263 | CA | ALA | C | 164 | 28.227 | 37.381 | 21.179 | 1.00 | 50.46 | GZ00 | C |
| ATOM | 1264 | C | ALA | C | 164 | 28.658 | 37.609 | 19.732 | 1.00 | 52.21 | GZ00 | C |
| ATOM | 1265 | O | ALA | C | 164 | 29.776 | 38.077 | 19.475 | 1.00 | 52.82 | GZ00 | O |
| ATOM | 1266 | CB | ALA | C | 164 | 28.544 | 35.947 | 21.599 | 1.00 | 41.14 | GZ00 | C |
| ATOM | 1267 | N | LEU | C | 165 | 27.793 | 37.267 | 18.780 | 1.00 | 42.69 | GZ00 | N |
| ATOM | 1268 | CA | LEU | C | 165 | 28.085 | 37.349 | 17.355 | 1.00 | 43.42 | GZ00 | C |
| ATOM | 1269 | C | LEU | C | 165 | 27.513 | 38.653 | 16.821 | 1.00 | 44.45 | GZ00 | C |
| ATOM | 1270 | O | LEU | C | 165 | 26.292 | 38.824 | 16.778 | 1.00 | 55.89 | GZ00 | O |
| ATOM | 1271 | CB | LEU | C | 165 | 27.476 | 36.152 | 16.631 | 1.00 | 43.81 | GZ00 | C |
| ATOM | 1272 | CG | LEU | C | 165 | 27.622 | 36.030 | 15.125 | 1.00 | 44.99 | GZ00 | C |
| ATOM | 1273 | CD1 | LEU | C | 165 | 29.090 | 36.095 | 14.649 | 1.00 | 39.41 | GZ00 | C |
| ATOM | 1274 | CD2 | LEU | C | 165 | 26.958 | 34.734 | 14.708 | 1.00 | 42.07 | GZ00 | C |
| ATOM | 1275 | N | THR | C | 166 | 28.389 | 39.563 | 16.411 | 1.00 | 43.67 | GZ00 | N |
| ATOM | 1276 | CA | THR | C | 166 | 28.013 | 40.872 | 15.890 | 1.00 | 50.96 | GZ00 | C |

| | | | | | | | | | | | | |
|---|---|---|---|---|---|---|---|---|---|---|---|---|
| ATOM | 1277 | C | THR | C | 166 | 28.621 | 41.169 | 14.521 | 1.00 | 48.02 | GZ00 | C |
| ATOM | 1278 | O | THR | C | 166 | 27.950 | 41.770 | 13.676 | 1.00 | 46.23 | GZ00 | O |
| ATOM | 1279 | CB | THR | C | 166 | 28.415 | 41.954 | 16.912 | 1.00 | 52.37 | GZ00 | C |
| ATOM | 1280 | OG1 | THR | C | 166 | 29.835 | 41.950 | 17.073 | 1.00 | 48.81 | GZ00 | O |
| ATOM | 1281 | CG2 | THR | C | 166 | 27.770 | 41.663 | 18.271 | 1.00 | 45.85 | GZ00 | C |
| ATOM | 1282 | N | SER | C | 167 | 29.856 | 40.736 | 14.268 | 1.00 | 44.72 | GZ00 | N |
| ATOM | 1283 | CA | SER | C | 167 | 30.445 | 40.880 | 12.941 | 1.00 | 46.25 | GZ00 | C |
| ATOM | 1284 | C | SER | C | 167 | 29.673 | 40.045 | 11.937 | 1.00 | 42.05 | GZ00 | C |
| ATOM | 1285 | O | SER | C | 167 | 29.319 | 38.894 | 12.201 | 1.00 | 43.05 | GZ00 | O |
| ATOM | 1286 | CB | SER | C | 167 | 31.909 | 40.437 | 12.933 | 1.00 | 39.29 | GZ00 | C |
| ATOM | 1287 | OG | SER | C | 167 | 32.675 | 41.320 | 13.706 | 1.00 | 49.43 | GZ00 | O |
| ATOM | 1288 | N | GLY | C | 168 | 29.424 | 40.629 | 10.779 | 1.00 | 39.63 | GZ00 | N |
| ATOM | 1289 | CA | GLY | C | 168 | 28.689 | 39.955 | 9.740 | 1.00 | 37.44 | GZ00 | C |
| ATOM | 1290 | C | GLY | C | 168 | 27.189 | 39.958 | 9.919 | 1.00 | 39.53 | GZ00 | C |
| ATOM | 1291 | O | GLY | C | 168 | 26.500 | 39.304 | 9.131 | 1.00 | 41.74 | GZ00 | O |
| ATOM | 1292 | N | VAL | C | 169 | 26.653 | 40.702 | 10.893 | 1.00 | 37.21 | GZ00 | N |
| ATOM | 1293 | CA | VAL | C | 169 | 25.219 | 40.711 | 11.158 | 1.00 | 39.62 | GZ00 | C |
| ATOM | 1294 | C | VAL | C | 169 | 24.583 | 41.858 | 10.391 | 1.00 | 34.91 | GZ00 | C |
| ATOM | 1295 | O | VAL | C | 169 | 24.991 | 43.010 | 10.529 | 1.00 | 35.65 | GZ00 | O |
| ATOM | 1296 | CB | VAL | C | 169 | 24.921 | 40.858 | 12.660 | 1.00 | 42.01 | GZ00 | C |
| ATOM | 1297 | CG1 | VAL | C | 169 | 23.404 | 41.063 | 12.867 | 1.00 | 36.86 | GZ00 | C |
| ATOM | 1298 | CG2 | VAL | C | 169 | 25.410 | 39.673 | 13.434 | 1.00 | 42.36 | GZ00 | C |
| ATOM | 1299 | N | HIS | C | 170 | 23.545 | 41.564 | 9.630 | 1.00 | 37.60 | GZ00 | N |
| ATOM | 1300 | CA | HIS | C | 170 | 22.842 | 42.603 | 8.889 | 1.00 | 37.31 | GZ00 | C |
| ATOM | 1301 | C | HIS | C | 170 | 21.345 | 42.438 | 9.134 | 1.00 | 37.74 | GZ00 | C |
| ATOM | 1302 | O | HIS | C | 170 | 20.704 | 41.559 | 8.554 | 1.00 | 38.41 | GZ00 | O |
| ATOM | 1303 | CB | HIS | C | 170 | 23.180 | 42.538 | 7.406 | 1.00 | 34.76 | GZ00 | C |
| ATOM | 1304 | CG | HIS | C | 170 | 22.719 | 43.735 | 6.634 | 1.00 | 43.07 | GZ00 | C |
| ATOM | 1305 | ND1 | HIS | C | 170 | 22.966 | 43.897 | 5.287 | 1.00 | 43.71 | GZ00 | N |
| ATOM | 1306 | CD2 | HIS | C | 170 | 22.022 | 44.830 | 7.023 | 1.00 | 40.56 | GZ00 | C |
| ATOM | 1307 | CE1 | HIS | C | 170 | 22.453 | 45.045 | 4.883 | 1.00 | 45.93 | GZ00 | C |
| ATOM | 1308 | NE2 | HIS | C | 170 | 21.866 | 45.626 | 5.915 | 1.00 | 45.52 | GZ00 | N |
| ATOM | 1309 | N | THR | C | 171 | 20.787 | 43.270 | 10.006 | 1.00 | 40.13 | GZ00 | N |
| ATOM | 1310 | CA | THR | C | 171 | 19.342 | 43.337 | 10.182 | 1.00 | 39.22 | GZ00 | C |
| ATOM | 1311 | C | THR | C | 171 | 18.800 | 44.400 | 9.230 | 1.00 | 39.29 | GZ00 | C |
| ATOM | 1312 | O | THR | C | 171 | 19.079 | 45.581 | 9.404 | 1.00 | 36.69 | GZ00 | O |
| ATOM | 1313 | CB | THR | C | 171 | 18.984 | 43.680 | 11.629 | 1.00 | 39.31 | GZ00 | C |
| ATOM | 1314 | OG1 | THR | C | 171 | 19.473 | 42.647 | 12.494 | 1.00 | 41.01 | GZ00 | O |
| ATOM | 1315 | CG2 | THR | C | 171 | 17.445 | 43.812 | 11.801 | 1.00 | 33.43 | GZ00 | C |
| ATOM | 1316 | N | PHE | C | 172 | 17.989 | 43.982 | 8.262 | 1.00 | 38.72 | GZ00 | N |
| ATOM | 1317 | CA | PHE | C | 172 | 17.457 | 44.839 | 7.215 | 1.00 | 38.37 | GZ00 | C |
| ATOM | 1318 | C | PHE | C | 172 | 16.263 | 45.638 | 7.728 | 1.00 | 39.77 | GZ00 | C |
| ATOM | 1319 | O | PHE | C | 172 | 15.478 | 45.140 | 8.537 | 1.00 | 41.83 | GZ00 | O |
| ATOM | 1320 | CB | PHE | C | 172 | 16.990 | 44.017 | 6.013 | 1.00 | 35.77 | GZ00 | C |
| ATOM | 1321 | CG | PHE | C | 172 | 18.092 | 43.449 | 5.195 | 1.00 | 34.43 | GZ00 | C |
| ATOM | 1322 | CD1 | PHE | C | 172 | 18.884 | 42.416 | 5.679 | 1.00 | 40.25 | GZ00 | C |
| ATOM | 1323 | CD2 | PHE | C | 172 | 18.354 | 43.957 | 3.936 | 1.00 | 40.94 | GZ00 | C |
| ATOM | 1324 | CE1 | PHE | C | 172 | 19.914 | 41.886 | 4.906 | 1.00 | 37.45 | GZ00 | C |
| ATOM | 1325 | CE2 | PHE | C | 172 | 19.389 | 43.434 | 3.162 | 1.00 | 43.89 | GZ00 | C |
| ATOM | 1326 | CZ | PHE | C | 172 | 20.164 | 42.395 | 3.650 | 1.00 | 36.64 | GZ00 | C |
| ATOM | 1327 | N | PRO | C | 173 | 16.092 | 46.871 | 7.252 | 1.00 | 37.95 | GZ00 | N |
| ATOM | 1328 | CA | PRO | C | 173 | 14.883 | 47.639 | 7.596 | 1.00 | 33.97 | GZ00 | C |
| ATOM | 1329 | C | PRO | C | 173 | 13.616 | 46.905 | 7.173 | 1.00 | 35.10 | GZ00 | C |
| ATOM | 1330 | O | PRO | C | 173 | 13.585 | 46.195 | 6.163 | 1.00 | 33.25 | GZ00 | O |
| ATOM | 1331 | CB | PRO | C | 173 | 15.064 | 48.945 | 6.817 | 1.00 | 35.19 | GZ00 | C |
| ATOM | 1332 | CG | PRO | C | 173 | 16.573 | 49.046 | 6.616 | 1.00 | 34.04 | GZ00 | C |
| ATOM | 1333 | CD | PRO | C | 173 | 17.049 | 47.638 | 6.441 | 1.00 | 32.78 | GZ00 | C |
| ATOM | 1334 | N | ALA | C | 174 | 12.550 | 47.109 | 7.945 | 1.00 | 39.15 | GZ00 | N |
| ATOM | 1335 | CA | ALA | C | 174 | 11.316 | 46.339 | 7.783 | 1.00 | 39.81 | GZ00 | C |
| ATOM | 1336 | C | ALA | C | 174 | 10.533 | 46.750 | 6.532 | 1.00 | 35.98 | GZ00 | C |
| ATOM | 1337 | O | ALA | C | 174 | 10.677 | 47.856 | 6.028 | 1.00 | 39.58 | GZ00 | O |
| ATOM | 1338 | CB | ALA | C | 174 | 10.432 | 46.527 | 9.013 | 1.00 | 43.39 | GZ00 | C |
| ATOM | 1339 | N | VAL | C | 175 | 9.686 | 45.824 | 6.013 | 1.00 | 38.79 | GZ00 | N |
| ATOM | 1340 | CA | VAL | C | 175 | 8.639 | 46.209 | 5.066 | 1.00 | 39.33 | GZ00 | C |
| ATOM | 1341 | C | VAL | C | 175 | 7.400 | 46.592 | 5.844 | 1.00 | 44.21 | GZ00 | C |
| ATOM | 1342 | O | VAL | C | 175 | 7.056 | 45.957 | 6.847 | 1.00 | 43.28 | GZ00 | O |
| ATOM | 1343 | CB | VAL | C | 175 | 8.256 | 45.087 | 4.084 | 1.00 | 50.56 | GZ00 | C |
| ATOM | 1344 | CG1 | VAL | C | 175 | 8.851 | 45.303 | 2.713 | 1.00 | 51.62 | GZ00 | C |
| ATOM | 1345 | CG2 | VAL | C | 175 | 8.510 | 43.718 | 4.667 | 1.00 | 44.82 | GZ00 | C |
| ATOM | 1346 | N | LEU | C | 176 | 6.686 | 47.587 | 5.342 | 1.00 | 46.20 | GZ00 | N |
| ATOM | 1347 | CA | LEU | C | 176 | 5.329 | 47.863 | 5.785 | 1.00 | 41.81 | GZ00 | C |

| | | | | | | | | | | | | |
|---|---|---|---|---|---|---|---|---|---|---|---|---|
| ATOM | 1348 | C | LEU | C | 176 | 4.400 | 47.199 | 4.775 | 1.00 | 42.53 | GZ00 | C |
| ATOM | 1349 | O | LEU | C | 176 | 4.405 | 47.554 | 3.595 | 1.00 | 55.35 | GZ00 | O |
| ATOM | 1350 | CB | LEU | C | 176 | 5.082 | 49.361 | 5.889 | 1.00 | 43.16 | GZ00 | C |
| ATOM | 1351 | CG | LEU | C | 176 | 3.653 | 49.738 | 6.270 | 1.00 | 46.81 | GZ00 | C |
| ATOM | 1352 | CD1 | LEU | C | 176 | 3.258 | 48.982 | 7.523 | 1.00 | 39.25 | GZ00 | C |
| ATOM | 1353 | CD2 | LEU | C | 176 | 3.565 | 51.241 | 6.509 | 1.00 | 41.80 | GZ00 | C |
| ATOM | 1354 | N | GLN | C | 177 | 3.666 | 46.189 | 5.219 | 1.00 | 44.53 | GZ00 | N |
| ATOM | 1355 | CA | GLN | C | 177 | 2.809 | 45.403 | 4.345 | 1.00 | 50.59 | GZ00 | C |
| ATOM | 1356 | C | GLN | C | 177 | 1.442 | 46.069 | 4.173 | 1.00 | 49.14 | GZ00 | C |
| ATOM | 1357 | O | GLN | C | 177 | 0.999 | 46.856 | 5.018 | 1.00 | 43.05 | GZ00 | O |
| ATOM | 1358 | CB | GLN | C | 177 | 2.634 | 43.990 | 4.909 | 1.00 | 46.96 | GZ00 | C |
| ATOM | 1359 | CG | GLN | C | 177 | 3.923 | 43.188 | 5.105 | 1.00 | 49.24 | GZ00 | C |
| ATOM | 1360 | CD | GLN | C | 177 | 3.666 | 41.894 | 5.863 | 1.00 | 56.86 | GZ00 | C |
| ATOM | 1361 | OE1 | GLN | C | 177 | 3.214 | 40.898 | 5.285 | 1.00 | 62.22 | GZ00 | O |
| ATOM | 1362 | NE2 | GLN | C | 177 | 3.939 | 41.905 | 7.170 | 1.00 | 48.26 | GZ00 | N |
| ATOM | 1363 | N | SER | C | 178 | 0.751 | 45.698 | 3.084 | 1.00 | 45.38 | GZ00 | N |
| ATOM | 1364 | CA | SER | C | 178 | -0.624 | 46.166 | 2.867 | 1.00 | 48.86 | GZ00 | C |
| ATOM | 1365 | C | SER | C | 178 | -1.544 | 45.854 | 4.039 | 1.00 | 46.49 | GZ00 | C |
| ATOM | 1366 | O | SER | C | 178 | -2.528 | 46.562 | 4.260 | 1.00 | 57.16 | GZ00 | O |
| ATOM | 1367 | CB | SER | C | 178 | -1.216 | 45.531 | 1.617 | 1.00 | 50.40 | GZ00 | C |
| ATOM | 1368 | OG | SER | C | 178 | -0.204 | 45.003 | 0.795 | 1.00 | 71.70 | GZ00 | O |
| ATOM | 1369 | N | SER | C | 179 | -1.258 | 44.793 | 4.786 | 1.00 | 50.74 | GZ00 | N |
| ATOM | 1370 | CA | SER | C | 179 | -1.988 | 44.477 | 6.007 | 1.00 | 43.18 | GZ00 | C |
| ATOM | 1371 | C | SER | C | 179 | -1.776 | 45.503 | 7.110 | 1.00 | 49.34 | GZ00 | C |
| ATOM | 1372 | O | SER | C | 179 | -2.482 | 45.454 | 8.121 | 1.00 | 48.58 | GZ00 | O |
| ATOM | 1373 | CB | SER | C | 179 | -1.546 | 43.111 | 6.513 | 1.00 | 44.27 | GZ00 | C |
| ATOM | 1374 | OG | SER | C | 179 | -0.258 | 43.204 | 7.095 | 1.00 | 48.97 | GZ00 | O |
| ATOM | 1375 | N | GLY | C | 180 | -0.798 | 46.398 | 6.970 | 1.00 | 46.74 | GZ00 | N |
| ATOM | 1376 | CA | GLY | C | 180 | -0.440 | 47.281 | 8.059 | 1.00 | 45.92 | GZ00 | C |
| ATOM | 1377 | C | GLY | C | 180 | 0.566 | 46.707 | 9.037 | 1.00 | 50.25 | GZ00 | C |
| ATOM | 1378 | O | GLY | C | 180 | 0.916 | 47.389 | 10.012 | 1.00 | 43.25 | GZ00 | O |
| ATOM | 1379 | N | LEU | C | 181 | 1.039 | 45.481 | 8.813 | 1.00 | 44.89 | GZ00 | N |
| ATOM | 1380 | CA | LEU | C | 181 | 2.003 | 44.836 | 9.695 | 1.00 | 49.99 | GZ00 | C |
| ATOM | 1381 | C | LEU | C | 181 | 3.408 | 44.908 | 9.101 | 1.00 | 47.59 | GZ00 | C |
| ATOM | 1382 | O | LEU | C | 181 | 3.594 | 45.023 | 7.887 | 1.00 | 47.03 | GZ00 | O |
| ATOM | 1383 | CB | LEU | C | 181 | 1.621 | 43.373 | 9.938 | 1.00 | 43.97 | GZ00 | C |
| ATOM | 1384 | CG | LEU | C | 181 | 0.269 | 43.159 | 10.616 | 1.00 | 50.80 | GZ00 | C |
| ATOM | 1385 | CD1 | LEU | C | 181 | -0.047 | 41.676 | 10.719 | 1.00 | 43.98 | GZ00 | C |
| ATOM | 1386 | CD2 | LEU | C | 181 | 0.246 | 43.830 | 11.981 | 1.00 | 46.56 | GZ00 | C |
| ATOM | 1387 | N | TYR | C | 182 | 4.404 | 44.833 | 9.973 | 1.00 | 45.32 | GZ00 | N |
| ATOM | 1388 | CA | TYR | C | 182 | 5.793 | 44.866 | 9.536 | 1.00 | 45.62 | GZ00 | C |
| ATOM | 1389 | C | TYR | C | 182 | 6.381 | 43.465 | 9.437 | 1.00 | 44.85 | GZ00 | C |
| ATOM | 1390 | O | TYR | C | 182 | 5.955 | 42.536 | 10.124 | 1.00 | 51.18 | GZ00 | O |
| ATOM | 1391 | CB | TYR | C | 182 | 6.645 | 45.701 | 10.484 | 1.00 | 44.25 | GZ00 | C |
| ATOM | 1392 | CG | TYR | C | 182 | 6.265 | 47.162 | 10.529 | 1.00 | 49.19 | GZ00 | C |
| ATOM | 1393 | CD1 | TYR | C | 182 | 6.805 | 48.072 | 9.628 | 1.00 | 49.61 | GZ00 | C |
| ATOM | 1394 | CD2 | TYR | C | 182 | 5.368 | 47.633 | 11.474 | 1.00 | 51.64 | GZ00 | C |
| ATOM | 1395 | CE1 | TYR | C | 182 | 6.474 | 49.408 | 9.678 | 1.00 | 43.85 | GZ00 | C |
| ATOM | 1396 | CE2 | TYR | C | 182 | 5.029 | 48.974 | 11.530 | 1.00 | 55.94 | GZ00 | C |
| ATOM | 1397 | CZ | TYR | C | 182 | 5.587 | 49.853 | 10.626 | 1.00 | 50.36 | GZ00 | C |
| ATOM | 1398 | OH | TYR | C | 182 | 5.243 | 51.177 | 10.679 | 1.00 | 43.75 | GZ00 | O |
| ATOM | 1399 | N | SER | C | 183 | 7.374 | 43.326 | 8.569 | 1.00 | 46.44 | GZ00 | N |
| ATOM | 1400 | CA | SER | C | 183 | 8.231 | 42.152 | 8.534 | 1.00 | 42.34 | GZ00 | C |
| ATOM | 1401 | C | SER | C | 183 | 9.673 | 42.600 | 8.304 | 1.00 | 42.61 | GZ00 | C |
| ATOM | 1402 | O | SER | C | 183 | 9.927 | 43.635 | 7.681 | 1.00 | 42.07 | GZ00 | O |
| ATOM | 1403 | CB | SER | C | 183 | 7.799 | 41.173 | 7.452 | 1.00 | 40.89 | GZ00 | C |
| ATOM | 1404 | OG | SER | C | 183 | 6.531 | 40.617 | 7.751 | 1.00 | 50.66 | GZ00 | O |
| ATOM | 1405 | N | LEU | C | 184 | 10.622 | 41.838 | 8.842 | 1.00 | 42.23 | GZ00 | N |
| ATOM | 1406 | CA | LEU | C | 184 | 12.018 | 42.081 | 8.515 | 1.00 | 39.25 | GZ00 | C |
| ATOM | 1407 | C | LEU | C | 184 | 12.794 | 40.772 | 8.518 | 1.00 | 43.48 | GZ00 | C |
| ATOM | 1408 | O | LEU | C | 184 | 12.337 | 39.746 | 9.029 | 1.00 | 40.77 | GZ00 | O |
| ATOM | 1409 | CB | LEU | C | 184 | 12.650 | 43.095 | 9.473 | 1.00 | 35.18 | GZ00 | C |
| ATOM | 1410 | CG | LEU | C | 184 | 12.809 | 42.808 | 10.965 | 1.00 | 45.00 | GZ00 | C |
| ATOM | 1411 | CD1 | LEU | C | 184 | 13.869 | 41.700 | 11.303 | 1.00 | 40.29 | GZ00 | C |
| ATOM | 1412 | CD2 | LEU | C | 184 | 13.218 | 44.117 | 11.588 | 1.00 | 33.99 | GZ00 | C |
| ATOM | 1413 | N | SER | C | 185 | 14.012 | 40.842 | 7.986 | 1.00 | 44.24 | GZ00 | N |
| ATOM | 1414 | CA | SER | C | 185 | 14.963 | 39.747 | 8.041 | 1.00 | 35.64 | GZ00 | C |
| ATOM | 1415 | C | SER | C | 185 | 16.258 | 40.228 | 8.669 | 1.00 | 41.38 | GZ00 | C |
| ATOM | 1416 | O | SER | C | 185 | 16.650 | 41.392 | 8.526 | 1.00 | 39.54 | GZ00 | O |
| ATOM | 1417 | CB | SER | C | 185 | 15.260 | 39.197 | 6.661 | 1.00 | 36.15 | GZ00 | C |
| ATOM | 1418 | OG | SER | C | 185 | 14.075 | 38.752 | 6.055 | 1.00 | 40.64 | GZ00 | O |

| | | | | | | | | | | | | |
|---|---|---|---|---|---|---|---|---|---|---|---|---|
| ATOM | 1419 | N | SER | C | 186 | 16.927 | 39.308 | 9.349 | 1.00 | 38.32 | GZ00 | N |
| ATOM | 1420 | CA | SER | C | 186 | 18.265 | 39.524 | 9.871 | 1.00 | 36.92 | GZ00 | C |
| ATOM | 1421 | C | SER | C | 186 | 19.108 | 38.382 | 9.349 | 1.00 | 40.04 | GZ00 | C |
| ATOM | 1422 | O | SER | C | 186 | 18.687 | 37.226 | 9.434 | 1.00 | 43.87 | GZ00 | O |
| ATOM | 1423 | CB | SER | C | 186 | 18.273 | 39.558 | 11.396 | 1.00 | 38.94 | GZ00 | C |
| ATOM | 1424 | OG | SER | C | 186 | 19.589 | 39.675 | 11.902 | 1.00 | 39.68 | GZ00 | O |
| ATOM | 1425 | N | VAL | C | 187 | 20.267 | 38.698 | 8.773 | 1.00 | 38.05 | GZ00 | N |
| ATOM | 1426 | CA | VAL | C | 187 | 21.164 | 37.675 | 8.245 | 1.00 | 39.23 | GZ00 | C |
| ATOM | 1427 | C | VAL | C | 187 | 22.543 | 37.869 | 8.849 | 1.00 | 38.02 | GZ00 | C |
| ATOM | 1428 | O | VAL | C | 187 | 22.977 | 38.990 | 9.130 | 1.00 | 40.44 | GZ00 | O |
| ATOM | 1429 | CB | VAL | C | 187 | 21.291 | 37.714 | 6.708 | 1.00 | 42.17 | GZ00 | C |
| ATOM | 1430 | CG1 | VAL | C | 187 | 19.984 | 37.334 | 6.048 | 1.00 | 51.50 | GZ00 | C |
| ATOM | 1431 | CG2 | VAL | C | 187 | 21.733 | 39.100 | 6.265 | 1.00 | 42.55 | GZ00 | C |
| ATOM | 1432 | N | VAL | C | 188 | 23.252 | 36.770 | 9.018 | 1.00 | 36.36 | GZ00 | N |
| ATOM | 1433 | CA | VAL | C | 188 | 24.640 | 36.836 | 9.426 | 1.00 | 36.04 | GZ00 | C |
| ATOM | 1434 | C | VAL | C | 188 | 25.458 | 36.076 | 8.397 | 1.00 | 29.41 | GZ00 | C |
| ATOM | 1435 | O | VAL | C | 188 | 25.037 | 35.025 | 7.901 | 1.00 | 36.76 | GZ00 | O |
| ATOM | 1436 | CB | VAL | C | 188 | 24.848 | 36.314 | 10.861 | 1.00 | 42.84 | GZ00 | C |
| ATOM | 1437 | CG1 | VAL | C | 188 | 24.200 | 34.977 | 11.043 | 1.00 | 38.37 | GZ00 | C |
| ATOM | 1438 | CG2 | VAL | C | 188 | 26.343 | 36.272 | 11.210 | 1.00 | 41.22 | GZ00 | C |
| ATOM | 1439 | N | THR | C | 189 | 26.558 | 36.673 | 8.000 | 1.00 | 28.33 | GZ00 | N |
| ATOM | 1440 | CA | THR | C | 189 | 27.541 | 36.056 | 7.132 | 1.00 | 32.84 | GZ00 | C |
| ATOM | 1441 | C | THR | C | 189 | 28.628 | 35.434 | 8.001 | 1.00 | 34.03 | GZ00 | C |
| ATOM | 1442 | O | THR | C | 189 | 29.180 | 36.100 | 8.883 | 1.00 | 35.30 | GZ00 | O |
| ATOM | 1443 | CB | THR | C | 189 | 28.159 | 37.112 | 6.215 | 1.00 | 32.93 | GZ00 | C |
| ATOM | 1444 | OG1 | THR | C | 189 | 27.144 | 37.724 | 5.438 | 1.00 | 45.99 | GZ00 | O |
| ATOM | 1445 | CG2 | THR | C | 189 | 29.182 | 36.491 | 5.293 | 1.00 | 42.71 | GZ00 | C |
| ATOM | 1446 | N | VAL | C | 190 | 28.939 | 34.171 | 7.745 | 1.00 | 34.62 | GZ00 | N |
| ATOM | 1447 | CA | VAL | C | 190 | 29.911 | 33.429 | 8.544 | 1.00 | 31.43 | GZ00 | C |
| ATOM | 1448 | C | VAL | C | 190 | 30.828 | 32.676 | 7.590 | 1.00 | 33.62 | GZ00 | C |
| ATOM | 1449 | O | VAL | C | 190 | 30.489 | 32.452 | 6.412 | 1.00 | 32.38 | GZ00 | O |
| ATOM | 1450 | CB | VAL | C | 190 | 29.217 | 32.438 | 9.514 | 1.00 | 32.44 | GZ00 | C |
| ATOM | 1451 | CG1 | VAL | C | 190 | 28.249 | 33.160 | 10.466 | 1.00 | 30.52 | GZ00 | C |
| ATOM | 1452 | CG2 | VAL | C | 190 | 28.516 | 31.364 | 8.716 | 1.00 | 32.19 | GZ00 | C |
| ATOM | 1453 | N | PRO | C | 191 | 31.989 | 32.240 | 8.078 | 1.00 | 30.12 | GZ00 | N |
| ATOM | 1454 | CA | PRO | C | 191 | 32.826 | 31.359 | 7.271 | 1.00 | 32.76 | GZ00 | C |
| ATOM | 1455 | C | PRO | C | 191 | 32.058 | 30.080 | 6.963 | 1.00 | 30.65 | GZ00 | C |
| ATOM | 1456 | O | PRO | C | 191 | 31.363 | 29.531 | 7.820 | 1.00 | 33.10 | GZ00 | O |
| ATOM | 1457 | CB | PRO | C | 191 | 34.045 | 31.120 | 8.172 | 1.00 | 32.26 | GZ00 | C |
| ATOM | 1458 | CG | PRO | C | 191 | 34.060 | 32.297 | 9.081 | 1.00 | 34.77 | GZ00 | C |
| ATOM | 1459 | CD | PRO | C | 191 | 32.645 | 32.579 | 9.350 | 1.00 | 27.68 | GZ00 | C |
| ATOM | 1460 | N | SER | C | 192 | 32.149 | 29.634 | 5.711 | 1.00 | 29.53 | GZ00 | N |
| ATOM | 1461 | CA | SER | C | 192 | 31.350 | 28.487 | 5.287 | 1.00 | 39.33 | GZ00 | C |
| ATOM | 1462 | C | SER | C | 192 | 31.675 | 27.230 | 6.099 | 1.00 | 36.23 | GZ00 | C |
| ATOM | 1463 | O | SER | C | 192 | 30.769 | 26.485 | 6.472 | 1.00 | 42.93 | GZ00 | O |
| ATOM | 1464 | CB | SER | C | 192 | 31.541 | 28.240 | 3.789 | 1.00 | 38.81 | GZ00 | C |
| ATOM | 1465 | OG | SER | C | 192 | 32.874 | 27.909 | 3.459 | 1.00 | 43.23 | GZ00 | O |
| ATOM | 1466 | N | SER | C | 193 | 32.938 | 27.035 | 6.471 | 1.00 | 34.82 | GZ00 | N |
| ATOM | 1467 | CA | SER | C | 193 | 33.323 | 25.884 | 7.287 | 1.00 | 34.53 | GZ00 | C |
| ATOM | 1468 | C | SER | C | 193 | 32.696 | 25.922 | 8.671 | 1.00 | 34.09 | GZ00 | C |
| ATOM | 1469 | O | SER | C | 193 | 32.757 | 24.928 | 9.386 | 1.00 | 39.08 | GZ00 | O |
| ATOM | 1470 | CB | SER | C | 193 | 34.853 | 25.776 | 7.406 | 1.00 | 30.12 | GZ00 | C |
| ATOM | 1471 | OG | SER | C | 193 | 35.392 | 26.892 | 8.093 | 1.00 | 36.36 | GZ00 | O |
| ATOM | 1472 | N | SER | C | 194 | 32.163 | 27.056 | 9.102 | 1.00 | 38.37 | GZ00 | N |
| ATOM | 1473 | CA | SER | C | 194 | 31.530 | 27.087 | 10.411 | 1.00 | 33.39 | GZ00 | C |
| ATOM | 1474 | C | SER | C | 194 | 30.113 | 26.516 | 10.401 | 1.00 | 42.74 | GZ00 | C |
| ATOM | 1475 | O | SER | C | 194 | 29.523 | 26.338 | 11.474 | 1.00 | 39.11 | GZ00 | O |
| ATOM | 1476 | CB | SER | C | 194 | 31.499 | 28.525 | 10.943 | 1.00 | 36.98 | GZ00 | C |
| ATOM | 1477 | OG | SER | C | 194 | 32.808 | 29.033 | 11.127 | 1.00 | 48.78 | GZ00 | O |
| ATOM | 1478 | N | LEU | C | 195 | 29.544 | 26.221 | 9.235 | 1.00 | 39.64 | GZ00 | N |
| ATOM | 1479 | CA | LEU | C | 195 | 28.171 | 25.716 | 9.219 | 1.00 | 49.46 | GZ00 | C |
| ATOM | 1480 | C | LEU | C | 195 | 28.073 | 24.358 | 9.912 | 1.00 | 44.13 | GZ00 | C |
| ATOM | 1481 | O | LEU | C | 195 | 27.079 | 24.064 | 10.584 | 1.00 | 58.41 | GZ00 | O |
| ATOM | 1482 | CB | LEU | C | 195 | 27.658 | 25.619 | 7.783 | 1.00 | 39.54 | GZ00 | C |
| ATOM | 1483 | CG | LEU | C | 195 | 27.446 | 26.952 | 7.097 | 1.00 | 40.14 | GZ00 | C |
| ATOM | 1484 | CD1 | LEU | C | 195 | 26.943 | 26.739 | 5.696 | 1.00 | 44.58 | GZ00 | C |
| ATOM | 1485 | CD2 | LEU | C | 195 | 26.470 | 27.772 | 7.904 | 1.00 | 40.80 | GZ00 | C |
| ATOM | 1486 | N | GLY | C | 196 | 29.081 | 23.506 | 9.744 | 1.00 | 51.36 | GZ00 | N |
| ATOM | 1487 | CA | GLY | C | 196 | 29.046 | 22.221 | 10.420 | 1.00 | 65.18 | GZ00 | C |
| ATOM | 1488 | C | GLY | C | 196 | 29.407 | 22.268 | 11.889 | 1.00 | 66.41 | GZ00 | C |
| ATOM | 1489 | O | GLY | C | 196 | 29.013 | 21.375 | 12.644 | 1.00 | 61.49 | GZ00 | O |

| | | | | | | | | | | | | |
|---|---|---|---|---|---|---|---|---|---|---|---|---|
| ATOM | 1490 | N | THR | C | 197 | 30.128 | 23.300 | 12.314 | 1.00 | 62.25 | GZ00 | N |
| ATOM | 1491 | CA | THR | C | 197 | 30.755 | 23.340 | 13.626 | 1.00 | 58.93 | GZ00 | C |
| ATOM | 1492 | C | THR | C | 197 | 29.995 | 24.161 | 14.655 | 1.00 | 58.26 | GZ00 | C |
| ATOM | 1493 | O | THR | C | 197 | 30.047 | 23.842 | 15.845 | 1.00 | 62.50 | GZ00 | O |
| ATOM | 1494 | CB | THR | C | 197 | 32.165 | 23.899 | 13.486 | 1.00 | 60.74 | GZ00 | C |
| ATOM | 1495 | OG1 | THR | C | 197 | 32.802 | 23.224 | 12.405 | 1.00 | 62.30 | GZ00 | O |
| ATOM | 1496 | CG2 | THR | C | 197 | 32.961 | 23.642 | 14.759 | 1.00 | 74.83 | GZ00 | C |
| ATOM | 1497 | N | GLN | C | 198 | 29.373 | 25.261 | 14.243 | 1.00 | 52.75 | GZ00 | N |
| ATOM | 1498 | CA | GLN | C | 198 | 28.836 | 26.250 | 15.166 | 1.00 | 50.37 | GZ00 | C |
| ATOM | 1499 | C | GLN | C | 198 | 27.314 | 26.260 | 15.176 | 1.00 | 47.44 | GZ00 | C |
| ATOM | 1500 | O | GLN | C | 198 | 26.650 | 25.910 | 14.197 | 1.00 | 47.96 | GZ00 | O |
| ATOM | 1501 | CB | GLN | C | 198 | 29.345 | 27.650 | 14.839 | 1.00 | 49.33 | GZ00 | C |
| ATOM | 1502 | CG | GLN | C | 198 | 30.852 | 27.725 | 14.782 | 1.00 | 55.27 | GZ00 | C |
| ATOM | 1503 | CD | GLN | C | 198 | 31.515 | 27.225 | 16.062 | 1.00 | 61.24 | GZ00 | C |
| ATOM | 1504 | OE1 | GLN | C | 198 | 32.566 | 26.585 | 16.007 | 1.00 | 51.30 | GZ00 | O |
| ATOM | 1505 | NE2 | GLN | C | 198 | 30.903 | 27.517 | 17.218 | 1.00 | 56.92 | GZ00 | N |
| ATOM | 1506 | N | THR | C | 199 | 26.773 | 26.623 | 16.322 | 1.00 | 52.47 | GZ00 | N |
| ATOM | 1507 | CA | THR | C | 199 | 25.343 | 26.782 | 16.481 | 1.00 | 55.26 | GZ00 | C |
| ATOM | 1508 | C | THR | C | 199 | 25.008 | 28.268 | 16.402 | 1.00 | 49.37 | GZ00 | C |
| ATOM | 1509 | O | THR | C | 199 | 25.618 | 29.094 | 17.092 | 1.00 | 42.11 | GZ00 | O |
| ATOM | 1510 | CB | THR | C | 199 | 24.875 | 26.156 | 17.795 | 1.00 | 51.51 | GZ00 | C |
| ATOM | 1511 | OG1 | THR | C | 199 | 24.993 | 24.734 | 17.686 | 1.00 | 63.19 | GZ00 | O |
| ATOM | 1512 | CG2 | THR | C | 199 | 23.428 | 26.505 | 18.070 | 1.00 | 53.59 | GZ00 | C |
| ATOM | 1513 | N | TYR | C | 200 | 24.078 | 28.610 | 15.518 | 1.00 | 48.90 | GZ00 | N |
| ATOM | 1514 | CA | TYR | C | 200 | 23.662 | 29.990 | 15.315 | 1.00 | 48.61 | GZ00 | C |
| ATOM | 1515 | C | TYR | C | 200 | 22.209 | 30.127 | 15.721 | 1.00 | 46.13 | GZ00 | C |
| ATOM | 1516 | O | TYR | C | 200 | 21.332 | 29.443 | 15.173 | 1.00 | 44.41 | GZ00 | O |
| ATOM | 1517 | CB | TYR | C | 200 | 23.885 | 30.425 | 13.871 | 1.00 | 40.49 | GZ00 | C |
| ATOM | 1518 | CG | TYR | C | 200 | 25.348 | 30.403 | 13.527 | 1.00 | 43.98 | GZ00 | C |
| ATOM | 1519 | CD1 | TYR | C | 200 | 26.196 | 31.359 | 14.053 | 1.00 | 42.42 | GZ00 | C |
| ATOM | 1520 | CD2 | TYR | C | 200 | 25.891 | 29.415 | 12.718 | 1.00 | 41.47 | GZ00 | C |
| ATOM | 1521 | CE1 | TYR | C | 200 | 27.536 | 31.356 | 13.770 | 1.00 | 39.19 | GZ00 | C |
| ATOM | 1522 | CE2 | TYR | C | 200 | 27.254 | 29.408 | 12.425 | 1.00 | 45.38 | GZ00 | C |
| ATOM | 1523 | CZ | TYR | C | 200 | 28.062 | 30.394 | 12.954 | 1.00 | 39.23 | GZ00 | C |
| ATOM | 1524 | OH | TYR | C | 200 | 29.417 | 30.433 | 12.699 | 1.00 | 45.19 | GZ00 | O |
| ATOM | 1525 | N | ILE | C | 201 | 21.975 | 30.996 | 16.697 | 1.00 | 42.87 | GZ00 | N |
| ATOM | 1526 | CA | ILE | C | 201 | 20.663 | 31.253 | 17.264 | 1.00 | 42.84 | GZ00 | C |
| ATOM | 1527 | C | ILE | C | 201 | 20.414 | 32.743 | 17.188 | 1.00 | 44.10 | GZ00 | C |
| ATOM | 1528 | O | ILE | C | 201 | 21.240 | 33.530 | 17.663 | 1.00 | 46.46 | GZ00 | O |
| ATOM | 1529 | CB | ILE | C | 201 | 20.581 | 30.788 | 18.727 | 1.00 | 50.09 | GZ00 | C |
| ATOM | 1530 | CG1 | ILE | C | 201 | 20.743 | 29.262 | 18.814 | 1.00 | 45.99 | GZ00 | C |
| ATOM | 1531 | CG2 | ILE | C | 201 | 19.298 | 31.312 | 19.375 | 1.00 | 42.60 | GZ00 | C |
| ATOM | 1532 | CD1 | ILE | C | 201 | 20.953 | 28.790 | 20.208 | 1.00 | 32.65 | GZ00 | C |
| ATOM | 1533 | N | CYS | C | 202 | 19.279 | 33.131 | 16.604 | 1.00 | 42.47 | GZ00 | N |
| ATOM | 1534 | CA | CYS | C | 202 | 18.862 | 34.523 | 16.601 | 1.00 | 43.27 | GZ00 | C |
| ATOM | 1535 | C | CYS | C | 202 | 17.860 | 34.776 | 17.723 | 1.00 | 49.37 | GZ00 | C |
| ATOM | 1536 | O | CYS | C | 202 | 17.021 | 33.922 | 18.040 | 1.00 | 48.50 | GZ00 | O |
| ATOM | 1537 | CB | CYS | C | 202 | 18.262 | 34.923 | 15.250 | 1.00 | 47.82 | GZ00 | C |
| ATOM | 1538 | SG | CYS | C | 202 | 16.539 | 34.525 | 15.045 | 1.00 | 59.08 | GZ00 | S |
| ATOM | 1539 | N | ASN | C | 203 | 17.976 | 35.945 | 18.341 | 1.00 | 44.56 | GZ00 | N |
| ATOM | 1540 | CA | ASN | C | 203 | 17.167 | 36.321 | 19.492 | 1.00 | 39.69 | GZ00 | C |
| ATOM | 1541 | C | ASN | C | 203 | 16.329 | 37.519 | 19.079 | 1.00 | 46.86 | GZ00 | C |
| ATOM | 1542 | O | ASN | C | 203 | 16.862 | 38.616 | 18.876 | 1.00 | 43.01 | GZ00 | O |
| ATOM | 1543 | CB | ASN | C | 203 | 18.043 | 36.660 | 20.698 | 1.00 | 40.17 | GZ00 | C |
| ATOM | 1544 | CG | ASN | C | 203 | 19.277 | 35.769 | 20.785 | 1.00 | 50.91 | GZ00 | C |
| ATOM | 1545 | OD1 | ASN | C | 203 | 20.407 | 36.239 | 20.626 | 1.00 | 44.43 | GZ00 | O |
| ATOM | 1546 | ND2 | ASN | C | 203 | 19.057 | 34.467 | 20.968 | 1.00 | 40.98 | GZ00 | N |
| ATOM | 1547 | N | VAL | C | 204 | 15.027 | 37.320 | 18.972 | 1.00 | 44.56 | GZ00 | N |
| ATOM | 1548 | CA | VAL | C | 204 | 14.126 | 38.360 | 18.515 | 1.00 | 46.34 | GZ00 | C |
| ATOM | 1549 | C | VAL | C | 204 | 13.377 | 38.894 | 19.715 | 1.00 | 43.52 | GZ00 | C |
| ATOM | 1550 | O | VAL | C | 204 | 12.839 | 38.122 | 20.516 | 1.00 | 51.43 | GZ00 | O |
| ATOM | 1551 | CB | VAL | C | 204 | 13.166 | 37.826 | 17.447 | 1.00 | 50.83 | GZ00 | C |
| ATOM | 1552 | CG1 | VAL | C | 204 | 12.204 | 38.932 | 17.006 | 1.00 | 40.92 | GZ00 | C |
| ATOM | 1553 | CG2 | VAL | C | 204 | 13.984 | 37.271 | 16.291 | 1.00 | 38.78 | GZ00 | C |
| ATOM | 1554 | N | ASN | C | 205 | 13.361 | 40.208 | 19.858 | 1.00 | 40.86 | GZ00 | N |
| ATOM | 1555 | CA | ASN | C | 205 | 12.687 | 40.856 | 20.970 | 1.00 | 54.57 | GZ00 | C |
| ATOM | 1556 | C | ASN | C | 205 | 11.772 | 41.947 | 20.431 | 1.00 | 58.30 | GZ00 | C |
| ATOM | 1557 | O | ASN | C | 205 | 12.237 | 42.886 | 19.776 | 1.00 | 55.70 | GZ00 | O |
| ATOM | 1558 | CB | ASN | C | 205 | 13.691 | 41.445 | 21.965 | 1.00 | 49.16 | GZ00 | C |
| ATOM | 1559 | CG | ASN | C | 205 | 13.009 | 42.013 | 23.204 | 1.00 | 69.12 | GZ00 | C |
| ATOM | 1560 | OD1 | ASN | C | 205 | 11.837 | 41.702 | 23.489 | 1.00 | 63.20 | GZ00 | O |

| | | | | | | | | | | | | |
|---|---|---|---|---|---|---|---|---|---|---|---|---|
| ATOM | 1561 | ND2 | ASN | C | 205 | 13.716 | 42.884 | 23.921 | 1.00 | 72.08 | GZ00 | N |
| ATOM | 1562 | N | HIS | C | 206 | 10.482 | 41.839 | 20.732 | 1.00 | 55.60 | GZ00 | N |
| ATOM | 1563 | CA | HIS | C | 206 | 9.489 | 42.834 | 20.326 | 1.00 | 56.54 | GZ00 | C |
| ATOM | 1564 | C | HIS | C | 206 | 8.843 | 43.347 | 21.611 | 1.00 | 58.64 | GZ00 | C |
| ATOM | 1565 | O | HIS | C | 206 | 7.857 | 42.788 | 22.098 | 1.00 | 54.70 | GZ00 | O |
| ATOM | 1566 | CB | HIS | C | 206 | 8.472 | 42.249 | 19.356 | 1.00 | 51.09 | GZ00 | C |
| ATOM | 1567 | CG | HIS | C | 206 | 7.482 | 43.251 | 18.851 | 1.00 | 60.00 | GZ00 | C |
| ATOM | 1568 | ND1 | HIS | C | 206 | 6.150 | 42.954 | 18.660 | 1.00 | 56.09 | GZ00 | N |
| ATOM | 1569 | CD2 | HIS | C | 206 | 7.633 | 44.548 | 18.494 | 1.00 | 60.16 | GZ00 | C |
| ATOM | 1570 | CE1 | HIS | C | 206 | 5.525 | 44.026 | 18.209 | 1.00 | 55.91 | GZ00 | C |
| ATOM | 1571 | NE2 | HIS | C | 206 | 6.402 | 45.007 | 18.100 | 1.00 | 50.50 | GZ00 | N |
| ATOM | 1572 | N | LYS | C | 207 | 9.435 | 44.398 | 22.179 | 1.00 | 59.62 | GZ00 | N |
| ATOM | 1573 | CA | LYS | C | 207 | 8.977 | 44.892 | 23.475 | 1.00 | 60.73 | GZ00 | C |
| ATOM | 1574 | C | LYS | C | 207 | 7.519 | 45.338 | 23.493 | 1.00 | 69.23 | GZ00 | C |
| ATOM | 1575 | O | LYS | C | 207 | 6.848 | 45.083 | 24.509 | 1.00 | 66.50 | GZ00 | O |
| ATOM | 1576 | CB | LYS | C | 207 | 9.906 | 46.014 | 23.956 | 1.00 | 58.40 | GZ00 | C |
| ATOM | 1577 | CG | LYS | C | 207 | 11.292 | 45.481 | 24.321 | 1.00 | 69.34 | GZ00 | C |
| ATOM | 1578 | CD | LYS | C | 207 | 12.257 | 46.543 | 24.809 | 1.00 | 77.26 | GZ00 | C |
| ATOM | 1579 | CE | LYS | C | 207 | 13.596 | 45.890 | 25.148 | 1.00 | 81.04 | GZ00 | C |
| ATOM | 1580 | NZ | LYS | C | 207 | 14.641 | 46.873 | 25.530 | 1.00 | 87.24 | GZ00 | N1+ |
| ATOM | 1581 | N | PRO | C | 208 | 6.956 | 45.959 | 22.441 | 1.00 | 69.40 | GZ00 | N |
| ATOM | 1582 | CA | PRO | C | 208 | 5.540 | 46.372 | 22.538 | 1.00 | 59.57 | GZ00 | C |
| ATOM | 1583 | C | PRO | C | 208 | 4.585 | 45.244 | 22.898 | 1.00 | 64.04 | GZ00 | C |
| ATOM | 1584 | O | PRO | C | 208 | 3.640 | 45.466 | 23.666 | 1.00 | 69.66 | GZ00 | O |
| ATOM | 1585 | CB | PRO | C | 208 | 5.257 | 46.942 | 21.145 | 1.00 | 53.38 | GZ00 | C |
| ATOM | 1586 | CG | PRO | C | 208 | 6.582 | 47.479 | 20.704 | 1.00 | 65.93 | GZ00 | C |
| ATOM | 1587 | CD | PRO | C | 208 | 7.608 | 46.524 | 21.241 | 1.00 | 55.91 | GZ00 | C |
| ATOM | 1588 | N | SER | C | 209 | 4.814 | 44.034 | 22.401 | 1.00 | 60.28 | GZ00 | N |
| ATOM | 1589 | CA | SER | C | 209 | 4.019 | 42.880 | 22.786 | 1.00 | 57.00 | GZ00 | C |
| ATOM | 1590 | C | SER | C | 209 | 4.762 | 41.960 | 23.758 | 1.00 | 67.68 | GZ00 | C |
| ATOM | 1591 | O | SER | C | 209 | 4.284 | 40.848 | 24.024 | 1.00 | 64.38 | GZ00 | O |
| ATOM | 1592 | CB | SER | C | 209 | 3.586 | 42.091 | 21.546 | 1.00 | 56.00 | GZ00 | C |
| ATOM | 1593 | OG | SER | C | 209 | 4.691 | 41.469 | 20.915 | 1.00 | 63.30 | GZ00 | O |
| ATOM | 1594 | N | ASN | C | 210 | 5.918 | 42.392 | 24.271 | 1.00 | 68.38 | GZ00 | N |
| ATOM | 1595 | CA | ASN | C | 210 | 6.846 | 41.563 | 25.049 | 1.00 | 66.63 | GZ00 | C |
| ATOM | 1596 | C | ASN | C | 210 | 6.898 | 40.139 | 24.505 | 1.00 | 64.18 | GZ00 | C |
| ATOM | 1597 | O | ASN | C | 210 | 6.538 | 39.164 | 25.164 | 1.00 | 67.42 | GZ00 | O |
| ATOM | 1598 | CB | ASN | C | 210 | 6.528 | 41.574 | 26.543 | 1.00 | 74.01 | GZ00 | C |
| ATOM | 1599 | CG | ASN | C | 210 | 5.055 | 41.657 | 26.828 | 1.00 | 83.26 | GZ00 | C |
| ATOM | 1600 | OD1 | ASN | C | 210 | 4.483 | 42.750 | 26.885 | 1.00 | 89.02 | GZ00 | O |
| ATOM | 1601 | ND2 | ASN | C | 210 | 4.420 | 40.502 | 26.997 | 1.00 | 83.49 | GZ00 | N |
| ATOM | 1602 | N | THR | C | 211 | 7.358 | 40.045 | 23.268 | 1.00 | 61.55 | GZ00 | N |
| ATOM | 1603 | CA | THR | C | 211 | 7.633 | 38.772 | 22.633 | 1.00 | 57.57 | GZ00 | C |
| ATOM | 1604 | C | THR | C | 211 | 9.143 | 38.608 | 22.609 | 1.00 | 60.01 | GZ00 | C |
| ATOM | 1605 | O | THR | C | 211 | 9.862 | 39.532 | 22.214 | 1.00 | 65.19 | GZ00 | O |
| ATOM | 1606 | CB | THR | C | 211 | 7.089 | 38.734 | 21.209 | 1.00 | 54.23 | GZ00 | C |
| ATOM | 1607 | OG1 | THR | C | 211 | 5.691 | 39.013 | 21.227 | 1.00 | 60.96 | GZ00 | O |
| ATOM | 1608 | CG2 | THR | C | 211 | 7.316 | 37.377 | 20.586 | 1.00 | 57.50 | GZ00 | C |
| ATOM | 1609 | N | LYS | C | 212 | 9.620 | 37.460 | 23.084 | 1.00 | 60.21 | GZ00 | N |
| ATOM | 1610 | CA | LYS | C | 212 | 11.018 | 37.076 | 22.951 | 1.00 | 52.01 | GZ00 | C |
| ATOM | 1611 | C | LYS | C | 212 | 11.042 | 35.689 | 22.350 | 1.00 | 52.91 | GZ00 | C |
| ATOM | 1612 | O | LYS | C | 212 | 10.456 | 34.760 | 22.906 | 1.00 | 57.94 | GZ00 | O |
| ATOM | 1613 | CB | LYS | C | 212 | 11.753 | 37.110 | 24.290 | 1.00 | 47.41 | GZ00 | C |
| ATOM | 1614 | CG | LYS | C | 212 | 11.691 | 38.475 | 24.978 | 1.00 | 57.15 | GZ00 | C |
| ATOM | 1615 | CD | LYS | C | 212 | 12.644 | 38.565 | 26.174 | 1.00 | 66.23 | GZ00 | C |
| ATOM | 1616 | CE | LYS | C | 212 | 12.433 | 39.861 | 26.969 | 1.00 | 79.81 | GZ00 | C |
| ATOM | 1617 | NZ | LYS | C | 212 | 13.705 | 40.467 | 27.503 | 1.00 | 78.51 | GZ00 | N1+ |
| ATOM | 1618 | N | VAL | C | 213 | 11.710 | 35.555 | 21.215 | 1.00 | 55.55 | GZ00 | N |
| ATOM | 1619 | CA | VAL | C | 213 | 11.839 | 34.287 | 20.520 | 1.00 | 49.66 | GZ00 | C |
| ATOM | 1620 | C | VAL | C | 213 | 13.319 | 34.021 | 20.272 | 1.00 | 50.74 | GZ00 | C |
| ATOM | 1621 | O | VAL | C | 213 | 14.050 | 34.906 | 19.810 | 1.00 | 48.29 | GZ00 | O |
| ATOM | 1622 | CB | VAL | C | 213 | 11.051 | 34.291 | 19.199 | 1.00 | 51.80 | GZ00 | C |
| ATOM | 1623 | CG1 | VAL | C | 213 | 11.260 | 32.982 | 18.441 | 1.00 | 52.63 | GZ00 | C |
| ATOM | 1624 | CG2 | VAL | C | 213 | 9.583 | 34.508 | 19.477 | 1.00 | 52.72 | GZ00 | C |
| ATOM | 1625 | N | ASP | C | 214 | 13.757 | 32.813 | 20.598 | 1.00 | 51.96 | GZ00 | N |
| ATOM | 1626 | CA | ASP | C | 214 | 15.054 | 32.301 | 20.199 | 1.00 | 43.41 | GZ00 | C |
| ATOM | 1627 | C | ASP | C | 214 | 14.791 | 31.231 | 19.160 | 1.00 | 52.09 | GZ00 | C |
| ATOM | 1628 | O | ASP | C | 214 | 13.923 | 30.374 | 19.356 | 1.00 | 60.39 | GZ00 | O |
| ATOM | 1629 | CB | ASP | C | 214 | 15.820 | 31.717 | 21.384 | 1.00 | 47.17 | GZ00 | C |
| ATOM | 1630 | CG | ASP | C | 214 | 16.093 | 32.742 | 22.473 | 1.00 | 53.34 | GZ00 | C |
| ATOM | 1631 | OD1 | ASP | C | 214 | 16.343 | 33.921 | 22.145 | 1.00 | 53.82 | GZ00 | O |

| | | | | | | | | | | | | |
|---|---|---|---|---|---|---|---|---|---|---|---|---|
| ATOM | 1632 | OD2 | ASP | C | 214 | 16.071 | 32.367 | 23.665 | 1.00 | 64.40 | GZ00 | O1- |
| ATOM | 1633 | N | LYS | C | 215 | 15.527 | 31.282 | 18.060 | 1.00 | 49.22 | GZ00 | N |
| ATOM | 1634 | CA | LYS | C | 215 | 15.322 | 30.324 | 16.993 | 1.00 | 46.64 | GZ00 | C |
| ATOM | 1635 | C | LYS | C | 215 | 16.691 | 29.904 | 16.489 | 1.00 | 51.67 | GZ00 | C |
| ATOM | 1636 | O | LYS | C | 215 | 17.531 | 30.748 | 16.165 | 1.00 | 50.73 | GZ00 | O |
| ATOM | 1637 | CB | LYS | C | 215 | 14.459 | 30.916 | 15.877 | 1.00 | 41.93 | GZ00 | C |
| ATOM | 1638 | CG | LYS | C | 215 | 14.213 | 29.983 | 14.699 | 1.00 | 58.37 | GZ00 | C |
| ATOM | 1639 | CD | LYS | C | 215 | 13.161 | 28.894 | 14.993 | 1.00 | 57.43 | GZ00 | C |
| ATOM | 1640 | CE | LYS | C | 215 | 11.848 | 29.473 | 15.498 | 1.00 | 54.91 | GZ00 | C |
| ATOM | 1641 | NZ | LYS | C | 215 | 10.704 | 28.533 | 15.296 | 1.00 | 59.67 | GZ00 | N1+ |
| ATOM | 1642 | N | LYS | C | 216 | 16.916 | 28.597 | 16.475 | 1.00 | 48.55 | GZ00 | N |
| ATOM | 1643 | CA | LYS | C | 216 | 18.160 | 28.025 | 16.004 | 1.00 | 44.55 | GZ00 | C |
| ATOM | 1644 | C | LYS | C | 216 | 18.075 | 27.873 | 14.498 | 1.00 | 51.23 | GZ00 | C |
| ATOM | 1645 | O | LYS | C | 216 | 17.073 | 27.377 | 13.973 | 1.00 | 48.07 | GZ00 | O |
| ATOM | 1646 | CB | LYS | C | 216 | 18.384 | 26.667 | 16.671 | 1.00 | 53.16 | GZ00 | C |
| ATOM | 1647 | CG | LYS | C | 216 | 19.666 | 25.939 | 16.297 | 1.00 | 58.06 | GZ00 | C |
| ATOM | 1648 | CD | LYS | C | 216 | 19.698 | 24.568 | 16.972 | 1.00 | 68.72 | GZ00 | C |
| ATOM | 1649 | CE | LYS | C | 216 | 21.064 | 23.901 | 16.884 | 1.00 | 76.90 | GZ00 | C |
| ATOM | 1650 | NZ | LYS | C | 216 | 21.133 | 22.703 | 17.775 | 1.00 | 81.06 | GZ00 | N1+ |
| ATOM | 1651 | N | VAL | C | 217 | 19.122 | 28.296 | 13.802 | 1.00 | 42.74 | GZ00 | N |
| ATOM | 1652 | CA | VAL | C | 217 | 19.158 | 28.228 | 12.355 | 1.00 | 45.91 | GZ00 | C |
| ATOM | 1653 | C | VAL | C | 217 | 20.199 | 27.179 | 12.003 | 1.00 | 52.09 | GZ00 | C |
| ATOM | 1654 | O | VAL | C | 217 | 21.399 | 27.371 | 12.243 | 1.00 | 50.43 | GZ00 | O |
| ATOM | 1655 | CB | VAL | C | 217 | 19.472 | 29.594 | 11.727 | 1.00 | 47.85 | GZ00 | C |
| ATOM | 1656 | CG1 | VAL | C | 217 | 19.352 | 29.534 | 10.217 | 1.00 | 36.68 | GZ00 | C |
| ATOM | 1657 | CG2 | VAL | C | 217 | 18.552 | 30.667 | 12.314 | 1.00 | 38.98 | GZ00 | C |
| ATOM | 1658 | N | GLU | C | 218 | 19.742 | 26.069 | 11.431 | 1.00 | 54.61 | GZ00 | N |
| ATOM | 1659 | CA | GLU | C | 218 | 20.591 | 24.937 | 11.107 | 1.00 | 54.05 | GZ00 | C |
| ATOM | 1660 | C | GLU | C | 218 | 20.622 | 24.755 | 9.603 | 1.00 | 49.13 | GZ00 | C |
| ATOM | 1661 | O | GLU | C | 218 | 19.647 | 25.082 | 8.922 | 1.00 | 58.50 | GZ00 | O |
| ATOM | 1662 | CB | GLU | C | 218 | 20.080 | 23.637 | 11.742 | 1.00 | 65.26 | GZ00 | C |
| ATOM | 1663 | CG | GLU | C | 218 | 19.944 | 23.671 | 13.253 | 1.00 | 70.38 | GZ00 | C |
| ATOM | 1664 | CD | GLU | C | 218 | 19.413 | 22.365 | 13.822 | 1.00 | 84.00 | GZ00 | C |
| ATOM | 1665 | OE1 | GLU | C | 218 | 18.889 | 21.534 | 13.040 | 1.00 | 82.26 | GZ00 | O |
| ATOM | 1666 | OE2 | GLU | C | 218 | 19.515 | 22.180 | 15.056 | 1.00 | 91.02 | GZ00 | O1- |
| ATOM | 1667 | N | PRO | C | 219 | 21.716 | 24.232 | 9.056 | 1.00 | 57.01 | GZ00 | N |
| ATOM | 1668 | CA | PRO | C | 219 | 21.755 | 23.952 | 7.612 | 1.00 | 53.03 | GZ00 | C |
| ATOM | 1669 | C | PRO | C | 219 | 20.705 | 22.941 | 7.170 | 1.00 | 58.97 | GZ00 | C |
| ATOM | 1670 | O | PRO | C | 219 | 19.991 | 22.384 | 8.008 | 1.00 | 62.86 | GZ00 | O |
| ATOM | 1671 | CB | PRO | C | 219 | 23.185 | 23.443 | 7.393 | 1.00 | 49.20 | GZ00 | C |
| ATOM | 1672 | CG | PRO | C | 219 | 23.796 | 23.307 | 8.761 | 1.00 | 47.12 | GZ00 | C |
| ATOM | 1673 | CD | PRO | C | 219 | 23.051 | 24.186 | 9.671 | 1.00 | 55.30 | GZ00 | C |
| ATOM | 1674 | N | LYS | C | 220 | 20.623 | 22.700 | 5.857 | 1.00 | 71.75 | GZ00 | N |
| ATOM | 1675 | CA | LYS | C | 220 | 19.610 | 21.841 | 5.209 | 1.00 | 68.27 | GZ00 | C |
| ATOM | 1676 | C | LYS | C | 220 | 18.226 | 22.491 | 5.306 | 1.00 | 73.79 | GZ00 | C |
| ATOM | 1677 | O | LYS | C | 220 | 17.355 | 22.046 | 6.058 | 1.00 | 80.81 | GZ00 | O |
| ATOM | 1678 | CB | LYS | C | 220 | 19.570 | 20.424 | 5.812 | 1.00 | 60.33 | GZ00 | C |
| ATOM | 1679 | CG | LYS | C | 220 | 18.562 | 19.498 | 5.140 | 1.00 | 75.19 | GZ00 | C |
| ATOM | 1680 | CD | LYS | C | 220 | 18.194 | 18.314 | 6.009 | 1.00 | 81.62 | GZ00 | C |
| ATOM | 1681 | CE | LYS | C | 220 | 17.204 | 17.408 | 5.284 | 1.00 | 83.68 | GZ00 | C |
| ATOM | 1682 | NZ | LYS | C | 220 | 15.922 | 18.107 | 4.974 | 1.00 | 81.30 | GZ00 | N1+ |
| TER | | | | | | | | | | | | |
| ATOM | 1683 | N | GLN | D | 1 | 3.622 | 57.068 | -14.037 | 1.00 | 73.81 | | N |
| ATOM | 1684 | CA | GLN | D | 1 | 2.256 | 57.519 | -13.830 | 1.00 | 77.03 | | C |
| ATOM | 1685 | C | GLN | D | 1 | 2.220 | 58.975 | -13.340 | 1.00 | 71.19 | | C |
| ATOM | 1686 | O | GLN | D | 1 | 1.170 | 59.618 | -13.403 | 1.00 | 70.55 | | O |
| ATOM | 1687 | CB | GLN | D | 1 | 1.535 | 56.594 | -12.844 | 1.00 | 76.72 | | C |
| ATOM | 1688 | CG | GLN | D | 1 | 0.016 | 56.704 | -12.856 | 1.00 | 83.42 | | C |
| ATOM | 1689 | CD | GLN | D | 1 | -0.568 | 56.892 | -11.458 | 1.00 | 94.69 | | C |
| ATOM | 1690 | OE1 | GLN | D | 1 | 0.127 | 56.720 | -10.448 | 1.00 | 86.56 | | O |
| ATOM | 1691 | NE2 | GLN | D | 1 | -1.851 | 57.255 | -11.395 | 1.00 | 91.59 | | N |
| ATOM | 1692 | N | SER | D | 2 | 3.352 | 59.504 | -12.865 | 1.00 | 62.32 | | N |
| ATOM | 1693 | CA | SER | D | 2 | 3.404 | 60.928 | -12.539 | 1.00 | 62.06 | | C |
| ATOM | 1694 | C | SER | D | 2 | 3.359 | 61.752 | -13.826 | 1.00 | 54.47 | | C |
| ATOM | 1695 | O | SER | D | 2 | 3.840 | 61.331 | -14.885 | 1.00 | 54.37 | | O |
| ATOM | 1696 | CB | SER | D | 2 | 4.637 | 61.269 | -11.685 | 1.00 | 55.55 | | C |
| ATOM | 1697 | OG | SER | D | 2 | 5.815 | 61.429 | -12.445 | 1.00 | 44.68 | | O |
| ATOM | 1698 | N | VAL | D | 3 | 2.765 | 62.942 | -13.725 | 1.00 | 51.09 | | N |
| ATOM | 1699 | CA | VAL | D | 3 | 2.399 | 63.693 | -14.926 | 1.00 | 44.98 | | C |
| ATOM | 1700 | C | VAL | D | 3 | 3.625 | 64.277 | -15.623 | 1.00 | 44.14 | | C |
| ATOM | 1701 | O | VAL | D | 3 | 3.682 | 64.317 | -16.858 | 1.00 | 42.24 | | O |

| | | | | | | | | | | | |
|---|---|---|---|---|---|---|---|---|---|---|---|
| ATOM | 1702 | CB | VAL | D | 3 | 1.355 | 64.762 | -14.561 | 1.00 | 48.49 | C |
| ATOM | 1703 | CG1 | VAL | D | 3 | 1.035 | 65.656 | -15.762 | 1.00 | 33.38 | C |
| ATOM | 1704 | CG2 | VAL | D | 3 | 0.095 | 64.072 | -13.994 | 1.00 | 36.61 | C |
| ATOM | 1705 | N | LEU | D | 4 | 4.610 | 64.757 | -14.867 | 1.00 | 40.88 | N |
| ATOM | 1706 | CA | LEU | D | 4 | 5.895 | 65.115 | -15.451 | 1.00 | 38.31 | C |
| ATOM | 1707 | C | LEU | D | 4 | 6.842 | 63.946 | -15.255 | 1.00 | 42.46 | C |
| ATOM | 1708 | O | LEU | D | 4 | 6.841 | 63.318 | -14.195 | 1.00 | 41.68 | O |
| ATOM | 1709 | CB | LEU | D | 4 | 6.484 | 66.365 | -14.808 | 1.00 | 36.49 | C |
| ATOM | 1710 | CG | LEU | D | 4 | 5.476 | 67.494 | -14.624 | 1.00 | 34.13 | C |
| ATOM | 1711 | CD1 | LEU | D | 4 | 6.074 | 68.656 | -13.825 | 1.00 | 34.44 | C |
| ATOM | 1712 | CD2 | LEU | D | 4 | 4.978 | 67.953 | -15.972 | 1.00 | 29.23 | C |
| ATOM | 1713 | N | THR | D | 5 | 7.637 | 63.640 | -16.273 | 1.00 | 39.33 | N |
| ATOM | 1714 | CA | THR | D | 5 | 8.541 | 62.503 | -16.203 | 1.00 | 34.69 | C |
| ATOM | 1715 | C | THR | D | 5 | 9.987 | 62.990 | -16.144 | 1.00 | 41.85 | C |
| ATOM | 1716 | O | THR | D | 5 | 10.420 | 63.814 | -16.959 | 1.00 | 32.54 | O |
| ATOM | 1717 | CB | THR | D | 5 | 8.307 | 61.529 | -17.363 | 1.00 | 40.41 | C |
| ATOM | 1718 | OG1 | THR | D | 5 | 8.542 | 62.190 | -18.596 | 1.00 | 58.56 | O |
| ATOM | 1719 | CG2 | THR | D | 5 | 6.858 | 61.060 | -17.366 | 1.00 | 35.80 | C |
| ATOM | 1720 | N | GLN | D | 6 | 10.703 | 62.522 | -15.132 | 1.00 | 33.22 | N |
| ATOM | 1721 | CA | GLN | D | 6 | 12.100 | 62.739 | -14.872 | 1.00 | 31.46 | C |
| ATOM | 1722 | C | GLN | D | 6 | 12.802 | 61.390 | -14.902 | 1.00 | 35.85 | C |
| ATOM | 1723 | O | GLN | D | 6 | 12.187 | 60.381 | -14.554 | 1.00 | 35.36 | O |
| ATOM | 1724 | CB | GLN | D | 6 | 12.302 | 63.370 | -13.490 | 1.00 | 31.54 | C |
| ATOM | 1725 | CG | GLN | D | 6 | 11.670 | 64.731 | -13.302 | 1.00 | 32.96 | C |
| ATOM | 1726 | CD | GLN | D | 6 | 11.827 | 65.238 | -11.884 | 1.00 | 38.63 | C |
| ATOM | 1727 | OE1 | GLN | D | 6 | 10.841 | 65.645 | -11.248 | 1.00 | 36.32 | O |
| ATOM | 1728 | NE2 | GLN | D | 6 | 13.073 | 65.220 | -11.367 | 1.00 | 31.40 | N |
| ATOM | 1729 | N | PRO | D | 7 | 14.069 | 61.323 | -15.306 | 1.00 | 39.24 | N |
| ATOM | 1730 | CA | PRO | D | 7 | 14.832 | 60.087 | -15.078 | 1.00 | 33.07 | C |
| ATOM | 1731 | C | PRO | D | 7 | 14.881 | 59.791 | -13.588 | 1.00 | 34.60 | C |
| ATOM | 1732 | O | PRO | D | 7 | 15.015 | 60.714 | -12.764 | 1.00 | 32.93 | O |
| ATOM | 1733 | CB | PRO | D | 7 | 16.235 | 60.402 | -15.652 | 1.00 | 31.22 | C |
| ATOM | 1734 | CG | PRO | D | 7 | 16.356 | 61.910 | -15.589 | 1.00 | 34.93 | C |
| ATOM | 1735 | CD | PRO | D | 7 | 14.921 | 62.430 | -15.806 | 1.00 | 35.72 | C |
| ATOM | 1736 | N | PRO | D | 8 | 14.755 | 58.518 | -13.197 | 1.00 | 33.41 | N |
| ATOM | 1737 | CA | PRO | D | 8 | 14.682 | 58.200 | -11.756 | 1.00 | 33.71 | C |
| ATOM | 1738 | C | PRO | D | 8 | 15.987 | 58.443 | -11.027 | 1.00 | 36.29 | C |
| ATOM | 1739 | O | PRO | D | 8 | 15.966 | 58.847 | -9.855 | 1.00 | 35.95 | O |
| ATOM | 1740 | CB | PRO | D | 8 | 14.299 | 56.705 | -11.730 | 1.00 | 31.63 | C |
| ATOM | 1741 | CG | PRO | D | 8 | 14.777 | 56.181 | -13.032 | 1.00 | 33.24 | C |
| ATOM | 1742 | CD | PRO | D | 8 | 14.568 | 57.333 | -14.040 | 1.00 | 28.70 | C |
| ATOM | 1743 | N | SER | D | 9 | 17.128 | 58.252 | -11.679 | 1.00 | 31.20 | N |
| ATOM | 1744 | CA | SER | D | 9 | 18.367 | 58.463 | -10.945 | 1.00 | 41.37 | C |
| ATOM | 1745 | C | SER | D | 9 | 19.485 | 58.910 | -11.869 | 1.00 | 41.68 | C |
| ATOM | 1746 | O | SER | D | 9 | 19.492 | 58.642 | -13.075 | 1.00 | 39.62 | O |
| ATOM | 1747 | CB | SER | D | 9 | 18.788 | 57.197 | -10.195 | 1.00 | 32.55 | C |
| ATOM | 1748 | OG | SER | D | 9 | 18.984 | 56.168 | -11.129 | 1.00 | 46.27 | O |
| ATOM | 1749 | N | VAL | D | 10 | 20.465 | 59.553 | -11.259 | 1.00 | 35.48 | N |
| ATOM | 1750 | CA | VAL | D | 10 | 21.529 | 60.199 | -12.000 | 1.00 | 37.87 | C |
| ATOM | 1751 | C | VAL | D | 10 | 22.736 | 60.253 | -11.082 | 1.00 | 34.78 | C |
| ATOM | 1752 | O | VAL | D | 10 | 22.605 | 60.523 | -9.880 | 1.00 | 36.71 | O |
| ATOM | 1753 | CB | VAL | D | 10 | 21.041 | 61.588 | -12.472 | 1.00 | 40.73 | C |
| ATOM | 1754 | CG1 | VAL | D | 10 | 21.926 | 62.691 | -11.971 | 1.00 | 40.07 | C |
| ATOM | 1755 | CG2 | VAL | D | 10 | 20.845 | 61.613 | -13.973 | 1.00 | 38.95 | C |
| ATOM | 1756 | N | SER | D | 11 | 23.915 | 59.963 | -11.625 | 1.00 | 37.46 | N |
| ATOM | 1757 | CA | SER | D | 11 | 25.092 | 59.962 | -10.762 | 1.00 | 35.14 | C |
| ATOM | 1758 | C | SER | D | 11 | 26.324 | 60.390 | -11.542 | 1.00 | 31.82 | C |
| ATOM | 1759 | O | SER | D | 11 | 26.474 | 60.057 | -12.715 | 1.00 | 37.48 | O |
| ATOM | 1760 | CB | SER | D | 11 | 25.311 | 58.580 | -10.130 | 1.00 | 33.24 | C |
| ATOM | 1761 | OG | SER | D | 11 | 25.640 | 57.636 | -11.125 | 1.00 | 37.23 | O |
| ATOM | 1762 | N | ALA | D | 12 | 27.196 | 61.139 | -10.877 | 1.00 | 35.26 | N |
| ATOM | 1763 | CA | ALA | D | 12 | 28.433 | 61.620 | -11.469 | 1.00 | 34.57 | C |
| ATOM | 1764 | C | ALA | D | 12 | 29.379 | 61.996 | -10.338 | 1.00 | 39.68 | C |
| ATOM | 1765 | O | ALA | D | 12 | 28.955 | 62.218 | -9.199 | 1.00 | 31.45 | O |
| ATOM | 1766 | CB | ALA | D | 12 | 28.200 | 62.814 | -12.409 | 1.00 | 31.29 | C |
| ATOM | 1767 | N | ALA | D | 13 | 30.673 | 62.071 | -10.679 | 1.00 | 36.93 | N |
| ATOM | 1768 | CA | ALA | D | 13 | 31.748 | 62.366 | -9.733 | 1.00 | 40.49 | C |
| ATOM | 1769 | C | ALA | D | 13 | 31.847 | 63.860 | -9.433 | 1.00 | 40.25 | C |
| ATOM | 1770 | O | ALA | D | 13 | 31.411 | 64.695 | -10.236 | 1.00 | 39.24 | O |
| ATOM | 1771 | CB | ALA | D | 13 | 33.081 | 61.875 | -10.291 | 1.00 | 30.80 | C |
| ATOM | 1772 | N | PRO | D | 14 | 32.434 | 64.225 | -8.288 | 1.00 | 33.52 | N |

| | | | | | | | | | | | |
|---|---|---|---|---|---|---|---|---|---|---|---|
| ATOM | 1773 | CA | PRO | D | 14 | 32.647 | 65.646 | -7.994 | 1.00 | 38.76 | C |
| ATOM | 1774 | C | PRO | D | 14 | 33.382 | 66.324 | -9.141 | 1.00 | 44.78 | C |
| ATOM | 1775 | O | PRO | D | 14 | 34.233 | 65.718 | -9.798 | 1.00 | 36.43 | O |
| ATOM | 1776 | CB | PRO | D | 14 | 33.485 | 65.629 | -6.711 | 1.00 | 35.31 | C |
| ATOM | 1777 | CG | PRO | D | 14 | 33.152 | 64.283 | -6.060 | 1.00 | 30.30 | C |
| ATOM | 1778 | CD | PRO | D | 14 | 32.928 | 63.345 | -7.208 | 1.00 | 32.96 | C |
| ATOM | 1779 | N | GLY | D | 15 | 33.008 | 67.579 | -9.410 | 1.00 | 45.92 | N |
| ATOM | 1780 | CA | GLY | D | 15 | 33.585 | 68.342 | -10.484 | 1.00 | 34.84 | C |
| ATOM | 1781 | C | GLY | D | 15 | 32.932 | 68.138 | -11.829 | 1.00 | 41.92 | C |
| ATOM | 1782 | O | GLY | D | 15 | 33.143 | 68.956 | -12.728 | 1.00 | 49.13 | O |
| ATOM | 1783 | N | GLN | D | 16 | 32.143 | 67.083 | -12.001 | 1.00 | 37.37 | N |
| ATOM | 1784 | CA | GLN | D | 16 | 31.533 | 66.844 | -13.302 | 1.00 | 48.89 | C |
| ATOM | 1785 | C | GLN | D | 16 | 30.220 | 67.627 | -13.500 | 1.00 | 47.03 | C |
| ATOM | 1786 | O | GLN | D | 16 | 29.744 | 68.367 | -12.628 | 1.00 | 39.51 | O |
| ATOM | 1787 | CB | GLN | D | 16 | 31.306 | 65.355 | -13.502 | 1.00 | 50.09 | C |
| ATOM | 1788 | CG | GLN | D | 16 | 32.570 | 64.589 | -13.809 | 1.00 | 57.03 | C |
| ATOM | 1789 | CD | GLN | D | 16 | 32.260 | 63.311 | -14.566 | 1.00 | 75.65 | C |
| ATOM | 1790 | OE1 | GLN | D | 16 | 31.830 | 62.302 | -13.979 | 1.00 | 62.08 | O |
| ATOM | 1791 | NE2 | GLN | D | 16 | 32.445 | 63.353 | -15.886 | 1.00 | 86.71 | N |
| ATOM | 1792 | N | LYS | D | 17 | 29.674 | 67.481 | -14.711 | 1.00 | 50.37 | N |
| ATOM | 1793 | CA | LYS | D | 17 | 28.424 | 68.054 | -15.189 | 1.00 | 47.34 | C |
| ATOM | 1794 | C | LYS | D | 17 | 27.325 | 67.004 | -15.183 | 1.00 | 51.01 | C |
| ATOM | 1795 | O | LYS | D | 17 | 27.566 | 65.822 | -15.440 | 1.00 | 57.21 | O |
| ATOM | 1796 | CB | LYS | D | 17 | 28.537 | 68.558 | -16.635 | 1.00 | 48.76 | C |
| ATOM | 1797 | CG | LYS | D | 17 | 29.325 | 69.812 | -16.889 | 1.00 | 66.24 | C |
| ATOM | 1798 | CD | LYS | D | 17 | 29.537 | 69.967 | -18.408 | 1.00 | 76.78 | C |
| ATOM | 1799 | CE | LYS | D | 17 | 30.502 | 71.109 | -18.742 | 1.00 | 85.45 | C |
| ATOM | 1800 | NZ | LYS | D | 17 | 30.739 | 71.204 | -20.209 | 1.00 | 85.79 | N |
| ATOM | 1801 | N | AVAL | D | 18 | 26.098 | 67.456 | -14.947 | 0.60 | 46.54 | N |
| ATOM | 1802 | CA | AVAL | D | 18 | 24.936 | 66.572 | -14.935 | 0.60 | 45.04 | C |
| ATOM | 1803 | C | AVAL | D | 18 | 23.728 | 67.369 | -15.412 | 0.60 | 42.17 | C |
| ATOM | 1804 | O | AVAL | D | 18 | 23.622 | 68.572 | -15.150 | 0.60 | 42.21 | O |
| ATOM | 1805 | CB | AVAL | D | 18 | 24.745 | 65.964 | -13.527 | 0.60 | 43.33 | C |
| ATOM | 1806 | CG1 | AVAL | D | 18 | 23.328 | 66.101 | -13.049 | 0.60 | 41.29 | C |
| ATOM | 1807 | CG2 | AVAL | D | 18 | 25.187 | 64.524 | -13.516 | 0.60 | 41.57 | C |
| ATOM | 1808 | N | BVAL | D | 18 | 26.101 | 67.445 | -14.920 | 0.40 | 46.52 | N |
| ATOM | 1809 | CA | BVAL | D | 18 | 24.961 | 66.551 | -15.030 | 0.40 | 45.04 | C |
| ATOM | 1810 | C | BVAL | D | 18 | 23.744 | 67.363 | -15.441 | 0.40 | 42.19 | C |
| ATOM | 1811 | O | BVAL | D | 18 | 23.643 | 68.561 | -15.158 | 0.40 | 42.16 | O |
| ATOM | 1812 | CB | BVAL | D | 18 | 24.729 | 65.769 | -13.718 | 0.40 | 43.17 | C |
| ATOM | 1813 | CG1 | BVAL | D | 18 | 24.057 | 66.644 | -12.663 | 0.40 | 39.96 | C |
| ATOM | 1814 | CG2 | BVAL | D | 18 | 23.942 | 64.530 | -14.005 | 0.40 | 42.54 | C |
| ATOM | 1815 | N | THR | D | 19 | 22.831 | 66.698 | -16.143 | 1.00 | 46.67 | N |
| ATOM | 1816 | CA | THR | D | 19 | 21.594 | 67.285 | -16.650 | 1.00 | 42.21 | C |
| ATOM | 1817 | C | THR | D | 19 | 20.412 | 66.449 | -16.178 | 1.00 | 42.60 | C |
| ATOM | 1818 | O | THR | D | 19 | 20.469 | 65.212 | -16.217 | 1.00 | 40.66 | O |
| ATOM | 1819 | CB | THR | D | 19 | 21.646 | 67.356 | -18.189 | 1.00 | 36.82 | C |
| ATOM | 1820 | OG1 | THR | D | 19 | 21.854 | 68.714 | -18.572 | 1.00 | 49.09 | O |
| ATOM | 1821 | CG2 | THR | D | 19 | 20.396 | 66.796 | -18.859 | 1.00 | 40.02 | C |
| ATOM | 1822 | N | ILE | D | 20 | 19.343 | 67.117 | -15.733 | 1.00 | 35.88 | N |
| ATOM | 1823 | CA | ILE | D | 20 | 18.132 | 66.439 | -15.267 | 1.00 | 33.46 | C |
| ATOM | 1824 | C | ILE | D | 20 | 16.954 | 66.997 | -16.042 | 1.00 | 38.36 | C |
| ATOM | 1825 | O | ILE | D | 20 | 16.680 | 68.200 | -15.976 | 1.00 | 36.03 | O |
| ATOM | 1826 | CB | ILE | D | 20 | 17.907 | 66.602 | -13.753 | 1.00 | 38.27 | C |
| ATOM | 1827 | CG1 | ILE | D | 20 | 19.043 | 65.927 | -12.976 | 1.00 | 32.31 | C |
| ATOM | 1828 | CG2 | ILE | D | 20 | 16.555 | 65.993 | -13.341 | 1.00 | 34.30 | C |
| ATOM | 1829 | CD1 | ILE | D | 20 | 19.010 | 66.199 | -11.490 | 1.00 | 28.88 | C |
| ATOM | 1830 | N | SER | D | 21 | 16.253 | 66.127 | -16.764 | 1.00 | 31.98 | N |
| ATOM | 1831 | CA | SER | D | 21 | 15.188 | 66.575 | -17.643 | 1.00 | 36.18 | C |
| ATOM | 1832 | C | SER | D | 21 | 13.814 | 66.346 | -17.011 | 1.00 | 40.44 | C |
| ATOM | 1833 | O | SER | D | 21 | 13.639 | 65.541 | -16.094 | 1.00 | 35.10 | O |
| ATOM | 1834 | CB | SER | D | 21 | 15.267 | 65.862 | -18.988 | 1.00 | 35.20 | C |
| ATOM | 1835 | OG | SER | D | 21 | 15.097 | 64.461 | -18.840 | 1.00 | 43.09 | O |
| ATOM | 1836 | N | CYS | D | 22 | 12.829 | 67.052 | -17.552 | 1.00 | 32.32 | N |
| ATOM | 1837 | CA | CYS | D | 22 | 11.476 | 67.044 | -17.012 | 1.00 | 36.57 | C |
| ATOM | 1838 | C | CYS | D | 22 | 10.549 | 67.317 | -18.192 | 1.00 | 36.72 | C |
| ATOM | 1839 | O | CYS | D | 22 | 10.444 | 68.454 | -18.656 | 1.00 | 33.99 | O |
| ATOM | 1840 | CB | CYS | D | 22 | 11.339 | 68.103 | -15.933 | 1.00 | 34.58 | C |
| ATOM | 1841 | SG | CYS | D | 22 | 9.650 | 68.451 | -15.371 | 1.00 | 54.36 | S |
| ATOM | 1842 | N | SER | D | 23 | 9.915 | 66.282 | -18.703 | 1.00 | 34.27 | N |
| ATOM | 1843 | CA | SER | D | 23 | 9.059 | 66.460 | -19.859 | 1.00 | 42.72 | C |

| | | | | | | | | | | | |
|---|---|---|---|---|---|---|---|---|---|---|---|
| ATOM | 1844 | C | SER | D | 23 | 7.582 | 66.345 | -19.454 | 1.00 | 41.50 | C |
| ATOM | 1845 | O | SER | D | 23 | 7.209 | 65.545 | -18.582 | 1.00 | 29.92 | O |
| ATOM | 1846 | CB | SER | D | 23 | 9.450 | 65.476 | -20.954 | 1.00 | 34.92 | C |
| ATOM | 1847 | OG | SER | D | 23 | 8.893 | 64.230 | -20.677 | 1.00 | 50.39 | O |
| ATOM | 1848 | N | GLY | D | 24 | 6.765 | 67.218 | -20.037 | 1.00 | 36.33 | N |
| ATOM | 1849 | CA | GLY | D | 24 | 5.336 | 67.237 | -19.809 | 1.00 | 38.73 | C |
| ATOM | 1850 | C | GLY | D | 24 | 4.600 | 67.510 | -21.103 | 1.00 | 39.14 | C |
| ATOM | 1851 | O | GLY | D | 24 | 4.942 | 66.945 | -22.139 | 1.00 | 42.42 | O |
| ATOM | 1852 | N | SER | D | 25 | 3.620 | 68.406 | -21.078 | 1.00 | 36.67 | N |
| ATOM | 1853 | CA | SER | D | 25 | 2.743 | 68.598 | -22.221 | 1.00 | 39.25 | C |
| ATOM | 1854 | C | SER | D | 25 | 2.382 | 70.072 | -22.346 | 1.00 | 38.01 | C |
| ATOM | 1855 | O | SER | D | 25 | 2.754 | 70.902 | -21.506 | 1.00 | 33.82 | O |
| ATOM | 1856 | CB | SER | D | 25 | 1.496 | 67.732 | -22.067 | 1.00 | 41.16 | C |
| ATOM | 1857 | OG | SER | D | 25 | 0.709 | 68.227 | -20.994 | 1.00 | 50.29 | O |
| ATOM | 1858 | N | SER | D | 26 | 1.657 | 70.404 | -23.420 | 1.00 | 31.89 | N |
| ATOM | 1859 | CA | SER | D | 26 | 1.329 | 71.809 | -23.656 | 1.00 | 36.67 | C |
| ATOM | 1860 | C | SER | D | 26 | 0.486 | 72.395 | -22.527 | 1.00 | 38.14 | C |
| ATOM | 1861 | O | SER | D | 26 | 0.548 | 73.600 | -22.271 | 1.00 | 38.11 | O |
| ATOM | 1862 | CB | SER | D | 26 | 0.624 | 71.976 | -25.002 | 1.00 | 31.63 | C |
| ATOM | 1863 | OG | SER | D | 26 | -0.356 | 70.980 | -25.146 | 1.00 | 53.99 | O |
| ATOM | 1864 | N | SER | D | 27 | -0.279 | 71.575 | -21.818 | 1.00 | 33.81 | N |
| ATOM | 1865 | CA | SER | D | 27 | -1.109 | 72.150 | -20.773 | 1.00 | 37.80 | C |
| ATOM | 1866 | C | SER | D | 27 | -0.387 | 72.281 | -19.431 | 1.00 | 40.08 | C |
| ATOM | 1867 | O | SER | D | 27 | -0.954 | 72.862 | -18.498 | 1.00 | 36.37 | O |
| ATOM | 1868 | CB | SER | D | 27 | -2.393 | 71.336 | -20.608 | 1.00 | 31.40 | C |
| ATOM | 1869 | OG | SER | D | 27 | -2.088 | 70.014 | -20.236 | 1.00 | 50.96 | O |
| ATOM | 1870 | N | ASN | D | 28 | 0.839 | 71.765 | -19.295 | 1.00 | 34.89 | N |
| ATOM | 1871 | CA | ASN | D | 28 | 1.555 | 72.073 | -18.068 | 1.00 | 33.57 | C |
| ATOM | 1872 | C | ASN | D | 28 | 2.831 | 72.854 | -18.389 | 1.00 | 34.78 | C |
| ATOM | 1873 | O | ASN | D | 28 | 2.800 | 74.091 | -18.405 | 1.00 | 36.75 | O |
| ATOM | 1874 | CB | ASN | D | 28 | 1.817 | 70.808 | -17.214 | 1.00 | 34.30 | C |
| ATOM | 1875 | CG | ASN | D | 28 | 2.277 | 69.580 | -18.027 | 1.00 | 34.72 | C |
| ATOM | 1876 | OD1 | ASN | D | 28 | 3.345 | 69.568 | -18.641 | 1.00 | 33.73 | O |
| ATOM | 1877 | ND2 | ASN | D | 28 | 1.500 | 68.515 | -17.949 | 1.00 | 35.11 | N |
| ATOM | 1878 | N | ILE | D | 29 | 3.952 | 72.171 | -18.633 | 1.00 | 33.54 | N |
| ATOM | 1879 | CA | ILE | D | 29 | 5.218 | 72.866 | -18.877 | 1.00 | 34.11 | C |
| ATOM | 1880 | C | ILE | D | 29 | 5.107 | 73.810 | -20.072 | 1.00 | 36.11 | C |
| ATOM | 1881 | O | ILE | D | 29 | 5.691 | 74.897 | -20.082 | 1.00 | 33.26 | O |
| ATOM | 1882 | CB | ILE | D | 29 | 6.350 | 71.840 | -19.063 | 1.00 | 33.30 | C |
| ATOM | 1883 | CG1 | ILE | D | 29 | 6.604 | 71.107 | -17.741 | 1.00 | 32.39 | C |
| ATOM | 1884 | CG2 | ILE | D | 29 | 7.621 | 72.520 | -19.555 | 1.00 | 25.88 | C |
| ATOM | 1885 | CD1 | ILE | D | 29 | 7.724 | 70.138 | -17.806 | 1.00 | 36.95 | C |
| ATOM | 1886 | N | GLY | D | 30 | 4.368 | 73.412 | -21.103 | 1.00 | 38.51 | N |
| ATOM | 1887 | CA | GLY | D | 30 | 4.274 | 74.237 | -22.289 | 1.00 | 30.45 | C |
| ATOM | 1888 | C | GLY | D | 30 | 3.608 | 75.580 | -22.062 | 1.00 | 38.50 | C |
| ATOM | 1889 | O | GLY | D | 30 | 3.763 | 76.475 | -22.889 | 1.00 | 40.64 | O |
| ATOM | 1890 | N | ASN | D | 31 | 2.836 | 75.744 | -20.987 | 1.00 | 37.18 | N |
| ATOM | 1891 | CA | ASN | D | 31 | 2.178 | 77.031 | -20.815 | 1.00 | 37.16 | C |
| ATOM | 1892 | C | ASN | D | 31 | 2.192 | 77.595 | -19.395 | 1.00 | 36.18 | C |
| ATOM | 1893 | O | ASN | D | 31 | 1.488 | 78.576 | -19.137 | 1.00 | 42.11 | O |
| ATOM | 1894 | CB | ASN | D | 31 | 0.731 | 76.942 | -21.340 | 1.00 | 41.60 | C |
| ATOM | 1895 | CG | ASN | D | 31 | 0.681 | 77.025 | -22.867 | 1.00 | 53.09 | C |
| ATOM | 1896 | OD1 | ASN | D | 31 | 0.759 | 78.110 | -23.442 | 1.00 | 63.01 | O |
| ATOM | 1897 | ND2 | ASN | D | 31 | 0.591 | 75.877 | -23.525 | 1.00 | 45.20 | N |
| ATOM | 1898 | N | ASN | D | 32 | 2.985 | 77.059 | -18.471 | 1.00 | 38.29 | N |
| ATOM | 1899 | CA | ASN | D | 32 | 3.026 | 77.622 | -17.128 | 1.00 | 35.50 | C |
| ATOM | 1900 | C | ASN | D | 32 | 4.467 | 77.757 | -16.662 | 1.00 | 33.99 | C |
| ATOM | 1901 | O | ASN | D | 32 | 5.394 | 77.247 | -17.291 | 1.00 | 37.19 | O |
| ATOM | 1902 | CB | ASN | D | 32 | 2.192 | 76.787 | -16.158 | 1.00 | 29.83 | C |
| ATOM | 1903 | CG | ASN | D | 32 | 0.731 | 76.754 | -16.548 | 1.00 | 35.04 | C |
| ATOM | 1904 | OD1 | ASN | D | 32 | 0.003 | 77.720 | -16.321 | 1.00 | 41.43 | O |
| ATOM | 1905 | ND2 | ASN | D | 32 | 0.299 | 75.657 | -17.166 | 1.00 | 38.66 | N |
| ATOM | 1906 | N | TYR | D | 33 | 4.647 | 78.497 | -15.572 | 1.00 | 30.88 | N |
| ATOM | 1907 | CA | TYR | D | 33 | 5.973 | 78.725 | -15.020 | 1.00 | 31.61 | C |
| ATOM | 1908 | C | TYR | D | 33 | 6.529 | 77.443 | -14.422 | 1.00 | 39.01 | C |
| ATOM | 1909 | O | TYR | D | 33 | 5.815 | 76.704 | -13.751 | 1.00 | 35.01 | O |
| ATOM | 1910 | CB | TYR | D | 33 | 5.925 | 79.804 | -13.951 | 1.00 | 27.05 | C |
| ATOM | 1911 | CG | TYR | D | 33 | 5.492 | 81.136 | -14.476 | 1.00 | 32.55 | C |
| ATOM | 1912 | CD1 | TYR | D | 33 | 6.214 | 81.780 | -15.478 | 1.00 | 29.14 | C |
| ATOM | 1913 | CD2 | TYR | D | 33 | 4.358 | 81.770 | -13.965 | 1.00 | 38.90 | C |
| ATOM | 1914 | CE1 | TYR | D | 33 | 5.805 | 83.036 | -15.967 | 1.00 | 36.47 | C |

| | | | | | | | | | | | |
|---|---|---|---|---|---|---|---|---|---|---|---|
| ATOM | 1915 | CE2 | TYR | D | 33 | 3.948 | 83.013 | -14.438 | 1.00 | 33.42 | C |
| ATOM | 1916 | CZ | TYR | D | 33 | 4.671 | 83.642 | -15.438 | 1.00 | 37.47 | C |
| ATOM | 1917 | OH | TYR | D | 33 | 4.246 | 84.866 | -15.903 | 1.00 | 36.22 | O |
| ATOM | 1918 | N | VAL | D | 34 | 7.821 | 77.195 | -14.623 | 1.00 | 33.97 | N |
| ATOM | 1919 | CA | VAL | D | 34 | 8.440 | 75.966 | -14.138 | 1.00 | 32.04 | C |
| ATOM | 1920 | C | VAL | D | 34 | 9.272 | 76.250 | -12.896 | 1.00 | 31.06 | C |
| ATOM | 1921 | O | VAL | D | 34 | 9.986 | 77.254 | -12.832 | 1.00 | 32.29 | O |
| ATOM | 1922 | CB | VAL | D | 34 | 9.292 | 75.308 | -15.236 | 1.00 | 33.98 | C |
| ATOM | 1923 | CG1 | VAL | D | 34 | 10.002 | 74.100 | -14.684 | 1.00 | 30.92 | C |
| ATOM | 1924 | CG2 | VAL | D | 34 | 8.407 | 74.884 | -16.397 | 1.00 | 34.56 | C |
| ATOM | 1925 | N | SER | D | 35 | 9.166 | 75.373 | -11.896 | 1.00 | 31.29 | N |
| ATOM | 1926 | CA | SER | D | 35 | 9.991 | 75.425 | -10.697 | 1.00 | 30.60 | C |
| ATOM | 1927 | C | SER | D | 35 | 10.791 | 74.134 | -10.560 | 1.00 | 35.34 | C |
| ATOM | 1928 | O | SER | D | 35 | 10.367 | 73.068 | -11.017 | 1.00 | 31.50 | O |
| ATOM | 1929 | CB | SER | D | 35 | 9.166 | 75.614 | -9.423 | 1.00 | 26.83 | C |
| ATOM | 1930 | OG | SER | D | 35 | 8.548 | 76.889 | -9.407 | 1.00 | 31.37 | O |
| ATOM | 1931 | N | TRP | D | 36 | 11.967 | 74.248 | -9.938 | 1.00 | 31.19 | N |
| ATOM | 1932 | CA | TRP | D | 36 | 12.774 | 73.104 | -9.559 | 1.00 | 30.26 | C |
| ATOM | 1933 | C | TRP | D | 36 | 12.996 | 73.142 | -8.060 | 1.00 | 29.04 | C |
| ATOM | 1934 | O | TRP | D | 36 | 13.286 | 74.196 | -7.491 | 1.00 | 29.53 | O |
| ATOM | 1935 | CB | TRP | D | 36 | 14.112 | 73.084 | -10.274 | 1.00 | 25.90 | C |
| ATOM | 1936 | CG | TRP | D | 36 | 14.009 | 72.705 | -11.713 | 1.00 | 32.87 | C |
| ATOM | 1937 | CD1 | TRP | D | 36 | 13.867 | 73.552 | -12.773 | 1.00 | 33.05 | C |
| ATOM | 1938 | CD2 | TRP | D | 36 | 14.049 | 71.383 | -12.259 | 1.00 | 32.92 | C |
| ATOM | 1939 | NE1 | TRP | D | 36 | 13.812 | 72.835 | -13.947 | 1.00 | 33.23 | N |
| ATOM | 1940 | CE2 | TRP | D | 36 | 13.941 | 71.505 | -13.660 | 1.00 | 29.67 | C |
| ATOM | 1941 | CE3 | TRP | D | 36 | 14.187 | 70.108 | -11.701 | 1.00 | 32.52 | C |
| ATOM | 1942 | CZ2 | TRP | D | 36 | 13.946 | 70.401 | -14.512 | 1.00 | 32.64 | C |
| ATOM | 1943 | CZ3 | TRP | D | 36 | 14.185 | 69.012 | -12.546 | 1.00 | 30.06 | C |
| ATOM | 1944 | CH2 | TRP | D | 36 | 14.058 | 69.166 | -13.936 | 1.00 | 35.04 | C |
| ATOM | 1945 | N | TYR | D | 37 | 12.874 | 71.982 | -7.429 | 1.00 | 31.89 | N |
| ATOM | 1946 | CA | TYR | D | 37 | 13.051 | 71.848 | -5.995 | 1.00 | 29.68 | C |
| ATOM | 1947 | C | TYR | D | 37 | 14.168 | 70.859 | -5.693 | 1.00 | 36.72 | C |
| ATOM | 1948 | O | TYR | D | 37 | 14.324 | 69.839 | -6.383 | 1.00 | 33.31 | O |
| ATOM | 1949 | CB | TYR | D | 37 | 11.735 | 71.417 | -5.343 | 1.00 | 28.40 | C |
| ATOM | 1950 | CG | TYR | D | 37 | 10.598 | 72.378 | -5.659 | 1.00 | 30.46 | C |
| ATOM | 1951 | CD1 | TYR | D | 37 | 10.417 | 73.540 | -4.921 | 1.00 | 27.52 | C |
| ATOM | 1952 | CD2 | TYR | D | 37 | 9.723 | 72.130 | -6.719 | 1.00 | 29.85 | C |
| ATOM | 1953 | CE1 | TYR | D | 37 | 9.368 | 74.423 | -5.219 | 1.00 | 32.37 | C |
| ATOM | 1954 | CE2 | TYR | D | 37 | 8.691 | 72.993 | -7.019 | 1.00 | 27.34 | C |
| ATOM | 1955 | CZ | TYR | D | 37 | 8.517 | 74.137 | -6.271 | 1.00 | 30.55 | C |
| ATOM | 1956 | OH | TYR | D | 37 | 7.495 | 74.995 | -6.595 | 1.00 | 35.11 | O |
| ATOM | 1957 | N | GLN | D | 38 | 14.938 | 71.174 | -4.653 | 1.00 | 32.56 | N |
| ATOM | 1958 | CA | GLN | D | 38 | 16.019 | 70.332 | -4.174 | 1.00 | 30.13 | C |
| ATOM | 1959 | C | GLN | D | 38 | 15.690 | 69.837 | -2.770 | 1.00 | 33.68 | C |
| ATOM | 1960 | O | GLN | D | 38 | 15.362 | 70.635 | -1.880 | 1.00 | 31.93 | O |
| ATOM | 1961 | CB | GLN | D | 38 | 17.349 | 71.102 | -4.170 | 1.00 | 30.01 | C |
| ATOM | 1962 | CG | GLN | D | 38 | 18.527 | 70.323 | -3.564 | 1.00 | 28.04 | C |
| ATOM | 1963 | CD | GLN | D | 38 | 19.729 | 71.214 | -3.280 | 1.00 | 34.96 | C |
| ATOM | 1964 | OE1 | GLN | D | 38 | 19.664 | 72.147 | -2.473 | 1.00 | 32.51 | O |
| ATOM | 1965 | NE2 | GLN | D | 38 | 20.828 | 70.944 | -3.971 | 1.00 | 33.81 | N |
| ATOM | 1966 | N | GLN | D | 39 | 15.790 | 68.528 | -2.572 | 1.00 | 32.31 | N |
| ATOM | 1967 | CA | GLN | D | 39 | 15.552 | 67.918 | -1.266 | 1.00 | 38.47 | C |
| ATOM | 1968 | C | GLN | D | 39 | 16.805 | 67.162 | -0.843 | 1.00 | 33.22 | C |
| ATOM | 1969 | O | GLN | D | 39 | 17.057 | 66.051 | -1.324 | 1.00 | 33.95 | O |
| ATOM | 1970 | CB | GLN | D | 39 | 14.336 | 67.001 | -1.294 | 1.00 | 32.82 | C |
| ATOM | 1971 | CG | GLN | D | 39 | 13.941 | 66.543 | 0.090 | 1.00 | 38.29 | C |
| ATOM | 1972 | CD | GLN | D | 39 | 12.621 | 65.809 | 0.118 | 1.00 | 34.34 | C |
| ATOM | 1973 | OE1 | GLN | D | 39 | 12.221 | 65.165 | -0.862 | 1.00 | 38.36 | O |
| ATOM | 1974 | NE2 | GLN | D | 39 | 11.909 | 65.942 | 1.236 | 1.00 | 30.94 | N |
| ATOM | 1975 | N | LEU | D | 40 | 17.612 | 67.779 | 0.023 | 1.00 | 37.41 | N |
| ATOM | 1976 | CA | LEU | D | 40 | 18.766 | 67.069 | 0.571 | 1.00 | 41.50 | C |
| ATOM | 1977 | C | LEU | D | 40 | 18.281 | 65.913 | 1.450 | 1.00 | 44.75 | C |
| ATOM | 1978 | O | LEU | D | 40 | 17.202 | 65.988 | 2.051 | 1.00 | 39.49 | O |
| ATOM | 1979 | CB | LEU | D | 40 | 19.659 | 68.020 | 1.368 | 1.00 | 41.85 | C |
| ATOM | 1980 | CG | LEU | D | 40 | 20.144 | 69.262 | 0.592 | 1.00 | 42.10 | C |
| ATOM | 1981 | CD1 | LEU | D | 40 | 20.614 | 70.363 | 1.515 | 1.00 | 38.12 | C |
| ATOM | 1982 | CD2 | LEU | D | 40 | 21.236 | 68.903 | -0.380 | 1.00 | 31.04 | C |
| ATOM | 1983 | N | PRO | D | 41 | 19.045 | 64.827 | 1.520 | 1.00 | 47.88 | N |
| ATOM | 1984 | CA | PRO | D | 41 | 18.568 | 63.625 | 2.225 | 1.00 | 42.38 | C |
| ATOM | 1985 | C | PRO | D | 41 | 18.152 | 63.935 | 3.656 | 1.00 | 45.34 | C |

| | | | | | | | | | | | |
|---|---|---|---|---|---|---|---|---|---|---|---|
| ATOM | 1986 | O | PRO | D | 41 | 18.876 | 64.603 | 4.404 | 1.00 | 46.53 | O |
| ATOM | 1987 | CB | PRO | D | 41 | 19.780 | 62.691 | 2.182 | 1.00 | 43.44 | C |
| ATOM | 1988 | CG | PRO | D | 41 | 20.639 | 63.217 | 1.062 | 1.00 | 46.87 | C |
| ATOM | 1989 | CD | PRO | D | 41 | 20.427 | 64.690 | 1.030 | 1.00 | 42.00 | C |
| ATOM | 1990 | N | GLY | D | 42 | 16.963 | 63.459 | 4.029 | 1.00 | 38.47 | N |
| ATOM | 1991 | CA | GLY | D | 42 | 16.400 | 63.729 | 5.346 | 1.00 | 39.47 | C |
| ATOM | 1992 | C | GLY | D | 42 | 15.980 | 65.164 | 5.648 | 1.00 | 51.21 | C |
| ATOM | 1993 | O | GLY | D | 42 | 15.801 | 65.501 | 6.822 | 1.00 | 48.36 | O |
| ATOM | 1994 | N | THR | D | 43 | 15.793 | 66.022 | 4.642 | 1.00 | 44.85 | N |
| ATOM | 1995 | CA | THR | D | 43 | 15.393 | 67.415 | 4.867 | 1.00 | 47.53 | C |
| ATOM | 1996 | C | THR | D | 43 | 14.123 | 67.747 | 4.090 | 1.00 | 39.84 | C |
| ATOM | 1997 | O | THR | D | 43 | 13.599 | 66.944 | 3.315 | 1.00 | 42.92 | O |
| ATOM | 1998 | CB | THR | D | 43 | 16.503 | 68.414 | 4.475 | 1.00 | 45.75 | C |
| ATOM | 1999 | OG1 | THR | D | 43 | 16.602 | 68.488 | 3.045 | 1.00 | 36.10 | O |
| ATOM | 2000 | CG2 | THR | D | 43 | 17.857 | 67.992 | 5.056 | 1.00 | 41.52 | C |
| ATOM | 2001 | N | ALA | D | 44 | 13.619 | 68.952 | 4.318 | 1.00 | 38.36 | N |
| ATOM | 2002 | CA | ALA | D | 44 | 12.484 | 69.426 | 3.553 | 1.00 | 38.31 | C |
| ATOM | 2003 | C | ALA | D | 44 | 12.916 | 69.799 | 2.137 | 1.00 | 39.83 | C |
| ATOM | 2004 | O | ALA | D | 44 | 14.100 | 70.032 | 1.878 | 1.00 | 37.35 | O |
| ATOM | 2005 | CB | ALA | D | 44 | 11.849 | 70.632 | 4.238 | 1.00 | 38.73 | C |
| ATOM | 2006 | N | PRO | D | 45 | 11.977 | 69.838 | 1.194 | 1.00 | 36.33 | N |
| ATOM | 2007 | CA | PRO | D | 45 | 12.277 | 70.462 | -0.097 | 1.00 | 35.97 | C |
| ATOM | 2008 | C | PRO | D | 45 | 12.678 | 71.911 | 0.121 | 1.00 | 33.44 | C |
| ATOM | 2009 | O | PRO | D | 45 | 12.384 | 72.519 | 1.155 | 1.00 | 33.83 | O |
| ATOM | 2010 | CB | PRO | D | 45 | 10.948 | 70.365 | -0.872 | 1.00 | 28.82 | C |
| ATOM | 2011 | CG | PRO | D | 45 | 10.265 | 69.198 | -0.245 | 1.00 | 36.55 | C |
| ATOM | 2012 | CD | PRO | D | 45 | 10.615 | 69.281 | 1.225 | 1.00 | 34.97 | C |
| ATOM | 2013 | N | LYS | D | 46 | 13.362 | 72.460 | -0.880 | 1.00 | 39.06 | N |
| ATOM | 2014 | CA | LYS | D | 46 | 13.782 | 73.855 | -0.903 | 1.00 | 35.89 | C |
| ATOM | 2015 | C | LYS | D | 46 | 13.682 | 74.312 | -2.350 | 1.00 | 36.30 | C |
| ATOM | 2016 | O | LYS | D | 46 | 13.953 | 73.521 | -3.262 | 1.00 | 37.16 | O |
| ATOM | 2017 | CB | LYS | D | 46 | 15.212 | 73.986 | -0.348 | 1.00 | 36.94 | C |
| ATOM | 2018 | CG | LYS | D | 46 | 15.919 | 75.292 | -0.569 | 1.00 | 43.26 | C |
| ATOM | 2019 | CD | LYS | D | 46 | 17.430 | 75.058 | -0.548 | 1.00 | 46.21 | C |
| ATOM | 2020 | CE | LYS | D | 46 | 18.178 | 76.121 | 0.262 | 1.00 | 53.10 | C |
| ATOM | 2021 | NZ | LYS | D | 46 | 19.663 | 75.960 | 0.110 | 1.00 | 59.22 | N1+ |
| ATOM | 2022 | N | LEU | D | 47 | 13.270 | 75.565 | -2.569 | 1.00 | 31.33 | N |
| ATOM | 2023 | CA | LEU | D | 47 | 13.200 | 76.086 | -3.931 | 1.00 | 31.83 | C |
| ATOM | 2024 | C | LEU | D | 47 | 14.601 | 76.299 | -4.513 | 1.00 | 35.29 | C |
| ATOM | 2025 | O | LEU | D | 47 | 15.454 | 76.963 | -3.911 | 1.00 | 33.02 | O |
| ATOM | 2026 | CB | LEU | D | 47 | 12.417 | 77.388 | -3.962 | 1.00 | 26.35 | C |
| ATOM | 2027 | CG | LEU | D | 47 | 12.230 | 78.066 | -5.326 | 1.00 | 35.90 | C |
| ATOM | 2028 | CD1 | LEU | D | 47 | 11.484 | 77.183 | -6.337 | 1.00 | 30.55 | C |
| ATOM | 2029 | CD2 | LEU | D | 47 | 11.508 | 79.411 | -5.153 | 1.00 | 30.96 | C |
| ATOM | 2030 | N | LEU | D | 48 | 14.837 | 75.725 | -5.687 | 1.00 | 34.87 | N |
| ATOM | 2031 | CA | LEU | D | 48 | 16.123 | 75.799 | -6.374 | 1.00 | 37.46 | C |
| ATOM | 2032 | C | LEU | D | 48 | 16.107 | 76.811 | -7.513 | 1.00 | 33.53 | C |
| ATOM | 2033 | O | LEU | D | 48 | 17.043 | 77.600 | -7.660 | 1.00 | 38.96 | O |
| ATOM | 2034 | CB | LEU | D | 48 | 16.516 | 74.416 | -6.926 | 1.00 | 27.18 | C |
| ATOM | 2035 | CG | LEU | D | 48 | 17.944 | 74.289 | -7.452 | 1.00 | 28.56 | C |
| ATOM | 2036 | CD1 | LEU | D | 48 | 18.892 | 74.353 | -6.289 | 1.00 | 32.28 | C |
| ATOM | 2037 | CD2 | LEU | D | 48 | 18.153 | 72.987 | -8.196 | 1.00 | 32.35 | C |
| ATOM | 2038 | N | LEU | D | 49 | 15.063 | 76.766 | -8.334 | 1.00 | 29.89 | N |
| ATOM | 2039 | CA | LEU | D | 49 | 14.871 | 77.609 | -9.500 | 1.00 | 31.38 | C |
| ATOM | 2040 | C | LEU | D | 49 | 13.383 | 77.885 | -9.629 | 1.00 | 35.27 | C |
| ATOM | 2041 | O | LEU | D | 49 | 12.563 | 76.964 | -9.504 | 1.00 | 32.72 | O |
| ATOM | 2042 | CB | LEU | D | 49 | 15.360 | 76.932 | -10.789 | 1.00 | 28.28 | C |
| ATOM | 2043 | CG | LEU | D | 49 | 16.848 | 76.621 | -10.937 | 1.00 | 40.25 | C |
| ATOM | 2044 | CD1 | LEU | D | 49 | 17.100 | 75.739 | -12.173 | 1.00 | 34.47 | C |
| ATOM | 2045 | CD2 | LEU | D | 49 | 17.642 | 77.943 | -10.996 | 1.00 | 32.52 | C |
| ATOM | 2046 | N | TYR | D | 50 | 13.031 | 79.136 | -9.909 | 1.00 | 28.76 | N |
| ATOM | 2047 | CA | TYR | D | 50 | 11.656 | 79.423 | -10.287 | 1.00 | 31.24 | C |
| ATOM | 2048 | C | TYR | D | 50 | 11.659 | 80.149 | -11.622 | 1.00 | 31.13 | C |
| ATOM | 2049 | O | TYR | D | 50 | 12.697 | 80.634 | -12.077 | 1.00 | 32.85 | O |
| ATOM | 2050 | CB | TYR | D | 50 | 10.923 | 80.211 | -9.196 | 1.00 | 30.09 | C |
| ATOM | 2051 | CG | TYR | D | 50 | 11.519 | 81.554 | -8.869 | 1.00 | 27.45 | C |
| ATOM | 2052 | CD1 | TYR | D | 50 | 12.726 | 81.672 | -8.163 | 1.00 | 31.73 | C |
| ATOM | 2053 | CD2 | TYR | D | 50 | 10.859 | 82.708 | -9.238 | 1.00 | 29.79 | C |
| ATOM | 2054 | CE1 | TYR | D | 50 | 13.253 | 82.920 | -7.843 | 1.00 | 34.25 | C |
| ATOM | 2055 | CE2 | TYR | D | 50 | 11.366 | 83.964 | -8.921 | 1.00 | 36.09 | C |
| ATOM | 2056 | CZ | TYR | D | 50 | 12.546 | 84.072 | -8.229 | 1.00 | 36.80 | C |

| | | | | | | | | | | | |
|---|---|---|---|---|---|---|---|---|---|---|---|
| ATOM | 2057 | OH | TYR | D | 50 | 12.995 | 85.340 | -7.954 | 1.00 | 34.65 | O |
| ATOM | 2058 | N | ASP | D | 51 | 10.487 | 80.198 | -12.260 | 1.00 | 33.46 | N |
| ATOM | 2059 | CA | ASP | D | 51 | 10.371 | 80.678 | -13.638 | 1.00 | 34.13 | C |
| ATOM | 2060 | C | ASP | D | 51 | 11.474 | 80.078 | -14.517 | 1.00 | 35.70 | C |
| ATOM | 2061 | O | ASP | D | 51 | 12.269 | 80.788 | -15.139 | 1.00 | 32.50 | O |
| ATOM | 2062 | CB | ASP | D | 51 | 10.410 | 82.199 | -13.683 | 1.00 | 33.19 | C |
| ATOM | 2063 | CG | ASP | D | 51 | 10.091 | 82.738 | -15.053 | 1.00 | 36.53 | C |
| ATOM | 2064 | OD1 | ASP | D | 51 | 9.413 | 82.007 | -15.813 | 1.00 | 37.05 | O |
| ATOM | 2065 | OD2 | ASP | D | 51 | 10.507 | 83.882 | -15.361 | 1.00 | 36.34 | O1- |
| ATOM | 2066 | N | SER | D | 52 | 11.565 | 78.745 | -14.494 | 1.00 | 35.41 | N |
| ATOM | 2067 | CA | SER | D | 52 | 12.525 | 77.980 | -15.295 | 1.00 | 35.10 | C |
| ATOM | 2068 | C | SER | D | 52 | 13.986 | 78.153 | -14.884 | 1.00 | 34.26 | C |
| ATOM | 2069 | O | SER | D | 52 | 14.730 | 77.167 | -14.836 | 1.00 | 34.09 | O |
| ATOM | 2070 | CB | SER | D | 52 | 12.393 | 78.333 | -16.779 | 1.00 | 29.40 | C |
| ATOM | 2071 | OG | SER | D | 52 | 11.152 | 77.892 | -17.275 | 1.00 | 42.15 | O |
| ATOM | 2072 | N | ASN | D | 53 | 14.437 | 79.386 | -14.628 | 1.00 | 32.85 | N |
| ATOM | 2073 | CA | ASN | D | 53 | 15.878 | 79.558 | -14.463 | 1.00 | 32.56 | C |
| ATOM | 2074 | C | ASN | D | 53 | 16.290 | 80.660 | -13.495 | 1.00 | 34.70 | C |
| ATOM | 2075 | O | ASN | D | 53 | 17.467 | 81.027 | -13.485 | 1.00 | 34.38 | O |
| ATOM | 2076 | CB | ASN | D | 53 | 16.536 | 79.814 | -15.804 | 1.00 | 29.46 | C |
| ATOM | 2077 | CG | ASN | D | 53 | 16.066 | 81.113 | -16.443 | 1.00 | 40.20 | C |
| ATOM | 2078 | OD1 | ASN | D | 53 | 15.192 | 81.817 | -15.918 | 1.00 | 32.59 | O |
| ATOM | 2079 | ND2 | ASN | D | 53 | 16.652 | 81.440 | -17.583 | 1.00 | 37.64 | N |
| ATOM | 2080 | N | LYS | D | 54 | 15.391 | 81.191 | -12.677 | 1.00 | 36.81 | N |
| ATOM | 2081 | CA | LYS | D | 54 | 15.761 | 82.209 | -11.713 | 1.00 | 29.79 | C |
| ATOM | 2082 | C | LYS | D | 54 | 16.163 | 81.540 | -10.408 | 1.00 | 35.07 | C |
| ATOM | 2083 | O | LYS | D | 54 | 15.433 | 80.686 | -9.887 | 1.00 | 34.40 | O |
| ATOM | 2084 | CB | LYS | D | 54 | 14.601 | 83.166 | -11.469 | 1.00 | 34.19 | C |
| ATOM | 2085 | CG | LYS | D | 54 | 14.979 | 84.334 | -10.607 | 1.00 | 39.09 | C |
| ATOM | 2086 | CD | LYS | D | 54 | 15.984 | 85.215 | -11.319 | 1.00 | 36.81 | C |
| ATOM | 2087 | CE | LYS | D | 54 | 16.480 | 86.282 | -10.370 | 1.00 | 39.74 | C |
| ATOM | 2088 | NZ | LYS | D | 54 | 17.279 | 87.308 | -11.098 | 1.00 | 49.26 | N1+ |
| ATOM | 2089 | N | ARG | D | 55 | 17.318 | 81.924 | -9.890 | 1.00 | 38.57 | N |
| ATOM | 2090 | C | ARG | D | 55 | 17.333 | 82.285 | -7.476 | 1.00 | 39.73 | C |
| ATOM | 2091 | O | ARG | D | 55 | 17.511 | 83.503 | -7.533 | 1.00 | 41.35 | O |
| ATOM | 2092 | CA | AARG | D | 55 | 17.814 | 81.402 | -8.621 | 0.50 | 38.68 | C |
| ATOM | 2093 | CB | AARG | D | 55 | 19.341 | 81.344 | -8.605 | 0.50 | 37.77 | C |
| ATOM | 2094 | CG | AARG | D | 55 | 19.958 | 80.202 | -9.404 | 0.50 | 40.11 | C |
| ATOM | 2095 | CD | AARG | D | 55 | 21.494 | 80.251 | -9.387 | 0.50 | 40.35 | C |
| ATOM | 2096 | NE | AARG | D | 55 | 22.017 | 81.378 | -10.159 | 0.50 | 39.90 | N |
| ATOM | 2097 | CZ | AARG | D | 55 | 22.738 | 82.377 | -9.659 | 0.50 | 39.21 | C |
| ATOM | 2098 | NH1 | AARG | D | 55 | 23.141 | 83.349 | -10.459 | 0.50 | 39.24 | N1+ |
| ATOM | 2099 | NH2 | AARG | D | 55 | 23.060 | 82.406 | -8.369 | 0.50 | 34.82 | N |
| ATOM | 2100 | CA | BARG | D | 55 | 17.803 | 81.399 | -8.621 | 0.50 | 38.68 | C |
| ATOM | 2101 | CB | BARG | D | 55 | 19.324 | 81.321 | -8.607 | 0.50 | 37.74 | C |
| ATOM | 2102 | CG | BARG | D | 55 | 19.902 | 80.435 | -9.684 | 0.50 | 40.11 | C |
| ATOM | 2103 | CD | BARG | D | 55 | 21.390 | 80.690 | -9.875 | 0.50 | 41.27 | C |
| ATOM | 2104 | NE | BARG | D | 55 | 21.881 | 80.029 | -11.080 | 0.50 | 44.29 | N |
| ATOM | 2105 | CZ | BARG | D | 55 | 21.979 | 80.606 | -12.275 | 0.50 | 41.10 | C |
| ATOM | 2106 | NH1 | BARG | D | 55 | 21.632 | 81.878 | -12.448 | 0.50 | 43.98 | N1+ |
| ATOM | 2107 | NH2 | BARG | D | 55 | 22.436 | 79.903 | -13.299 | 0.50 | 37.99 | N |
| ATOM | 2108 | N | PRO | D | 56 | 16.709 | 81.722 | -6.444 | 1.00 | 42.43 | N |
| ATOM | 2109 | CA | PRO | D | 56 | 16.550 | 82.476 | -5.202 | 1.00 | 39.14 | C |
| ATOM | 2110 | C | PRO | D | 56 | 17.937 | 82.781 | -4.673 | 1.00 | 39.14 | C |
| ATOM | 2111 | O | PRO | D | 56 | 18.921 | 82.132 | -5.037 | 1.00 | 39.24 | O |
| ATOM | 2112 | CB | PRO | D | 56 | 15.800 | 81.513 | -4.264 | 1.00 | 40.40 | C |
| ATOM | 2113 | CG | PRO | D | 56 | 15.350 | 80.384 | -5.079 | 1.00 | 36.67 | C |
| ATOM | 2114 | CD | PRO | D | 56 | 16.195 | 80.347 | -6.344 | 1.00 | 38.20 | C |
| ATOM | 2115 | N | SER | D | 57 | 18.025 | 83.787 | -3.819 | 1.00 | 38.52 | N |
| ATOM | 2116 | CA | SER | D | 57 | 19.326 | 84.087 | -3.245 | 1.00 | 45.88 | C |
| ATOM | 2117 | C | SER | D | 57 | 19.760 | 82.911 | -2.381 | 1.00 | 44.76 | C |
| ATOM | 2118 | O | SER | D | 57 | 18.928 | 82.206 | -1.807 | 1.00 | 46.46 | O |
| ATOM | 2119 | CB | SER | D | 57 | 19.282 | 85.384 | -2.441 | 1.00 | 45.60 | C |
| ATOM | 2120 | OG | SER | D | 57 | 18.126 | 85.430 | -1.652 | 1.00 | 61.35 | O |
| ATOM | 2121 | N | GLY | D | 58 | 21.056 | 82.630 | -2.375 | 1.00 | 45.80 | N |
| ATOM | 2122 | CA | GLY | D | 58 | 21.558 | 81.468 | -1.677 | 1.00 | 38.95 | C |
| ATOM | 2123 | C | GLY | D | 58 | 21.780 | 80.253 | -2.549 | 1.00 | 45.79 | C |
| ATOM | 2124 | O | GLY | D | 58 | 22.406 | 79.290 | -2.093 | 1.00 | 50.05 | O |
| ATOM | 2125 | N | ILE | D | 59 | 21.280 | 80.246 | -3.777 | 1.00 | 44.59 | N |
| ATOM | 2126 | CA | ILE | D | 59 | 21.479 | 79.123 | -4.684 | 1.00 | 37.66 | C |
| ATOM | 2127 | C | ILE | D | 59 | 22.682 | 79.446 | -5.571 | 1.00 | 42.15 | C |

| | | | | | | | | | | | |
|---|---|---|---|---|---|---|---|---|---|---|---|
| ATOM | 2128 | O | ILE | D | 59 | 22.645 | 80.454 | -6.294 | 1.00 | 41.34 | O |
| ATOM | 2129 | CB | ILE | D | 59 | 20.232 | 78.831 | -5.528 | 1.00 | 40.49 | C |
| ATOM | 2130 | CG1 | ILE | D | 59 | 19.045 | 78.500 | -4.616 | 1.00 | 37.44 | C |
| ATOM | 2131 | CG2 | ILE | D | 59 | 20.527 | 77.690 | -6.542 | 1.00 | 36.08 | C |
| ATOM | 2132 | CD1 | ILE | D | 59 | 19.273 | 77.277 | -3.723 | 1.00 | 34.90 | C |
| ATOM | 2133 | N | PRO | D | 60 | 23.724 | 78.612 | -5.571 | 1.00 | 38.05 | N |
| ATOM | 2134 | CA | PRO | D | 60 | 24.938 | 78.914 | -6.342 | 1.00 | 42.90 | C |
| ATOM | 2135 | C | PRO | D | 60 | 24.666 | 78.936 | -7.835 | 1.00 | 40.36 | C |
| ATOM | 2136 | O | PRO | D | 60 | 23.778 | 78.245 | -8.333 | 1.00 | 40.44 | O |
| ATOM | 2137 | CB | PRO | D | 60 | 25.888 | 77.754 | -5.990 | 1.00 | 43.52 | C |
| ATOM | 2138 | CG | PRO | D | 60 | 25.222 | 76.963 | -4.934 | 1.00 | 44.09 | C |
| ATOM | 2139 | CD | PRO | D | 60 | 23.756 | 77.268 | -4.984 | 1.00 | 41.65 | C |
| ATOM | 2140 | N | ALA | D | 61 | 25.495 | 79.695 | -8.557 | 1.00 | 38.15 | N |
| ATOM | 2141 | CA | ALA | D | 61 | 25.351 | 79.835 | -10.003 | 1.00 | 46.92 | C |
| ATOM | 2142 | C | ALA | D | 61 | 25.639 | 78.546 | -10.780 | 1.00 | 41.95 | C |
| ATOM | 2143 | O | ALA | D | 61 | 25.376 | 78.501 | -11.990 | 1.00 | 41.08 | O |
| ATOM | 2144 | CB | ALA | D | 61 | 26.268 | 80.955 | -10.507 | 1.00 | 37.90 | C |
| ATOM | 2145 | N | ARG | D | 62 | 26.167 | 77.501 | -10.144 | 1.00 | 40.02 | N |
| ATOM | 2146 | CA | ARG | D | 62 | 26.364 | 76.277 | -10.909 | 1.00 | 39.12 | C |
| ATOM | 2147 | C | ARG | D | 62 | 25.061 | 75.511 | -11.136 | 1.00 | 41.84 | C |
| ATOM | 2148 | O | ARG | D | 62 | 25.060 | 74.529 | -11.894 | 1.00 | 38.44 | O |
| ATOM | 2149 | CB | ARG | D | 62 | 27.411 | 75.384 | -10.240 | 1.00 | 40.13 | C |
| ATOM | 2150 | CG | ARG | D | 62 | 27.061 | 74.940 | -8.830 | 1.00 | 51.16 | C |
| ATOM | 2151 | CD | ARG | D | 62 | 28.110 | 73.962 | -8.276 | 1.00 | 54.07 | C |
| ATOM | 2152 | NE | ARG | D | 62 | 27.617 | 73.327 | -7.065 | 1.00 | 42.44 | N |
| ATOM | 2153 | CZ | ARG | D | 62 | 27.632 | 73.937 | -5.888 | 1.00 | 48.82 | C |
| ATOM | 2154 | NH1 | ARG | D | 62 | 28.139 | 75.165 | -5.790 | 1.00 | 55.75 | N1+ |
| ATOM | 2155 | NH2 | ARG | D | 62 | 27.145 | 73.336 | -4.819 | 1.00 | 44.87 | N |
| ATOM | 2156 | N | PHE | D | 63 | 23.959 | 75.946 | -10.518 | 1.00 | 35.81 | N |
| ATOM | 2157 | CA | PHE | D | 63 | 22.630 | 75.461 | -10.857 | 1.00 | 36.89 | C |
| ATOM | 2158 | C | PHE | D | 63 | 22.032 | 76.381 | -11.914 | 1.00 | 41.20 | C |
| ATOM | 2159 | O | PHE | D | 63 | 22.021 | 77.610 | -11.749 | 1.00 | 39.30 | O |
| ATOM | 2160 | CB | PHE | D | 63 | 21.724 | 75.420 | -9.622 | 1.00 | 36.15 | C |
| ATOM | 2161 | CG | PHE | D | 63 | 22.177 | 74.455 | -8.550 | 1.00 | 35.88 | C |
| ATOM | 2162 | CD1 | PHE | D | 63 | 23.020 | 74.870 | -7.539 | 1.00 | 36.00 | C |
| ATOM | 2163 | CD2 | PHE | D | 63 | 21.747 | 73.132 | -8.556 | 1.00 | 38.64 | C |
| ATOM | 2164 | CE1 | PHE | D | 63 | 23.443 | 73.983 | -6.550 | 1.00 | 43.00 | C |
| ATOM | 2165 | CE2 | PHE | D | 63 | 22.165 | 72.239 | -7.572 | 1.00 | 37.52 | C |
| ATOM | 2166 | CZ | PHE | D | 63 | 23.013 | 72.664 | -6.565 | 1.00 | 36.44 | C |
| ATOM | 2167 | N | SER | D | 64 | 21.548 | 75.795 | -13.005 | 1.00 | 35.81 | N |
| ATOM | 2168 | CA | SER | D | 64 | 20.854 | 76.595 | -14.008 | 1.00 | 37.12 | C |
| ATOM | 2169 | C | SER | D | 64 | 19.753 | 75.760 | -14.648 | 1.00 | 38.07 | C |
| ATOM | 2170 | O | SER | D | 64 | 19.691 | 74.529 | -14.505 | 1.00 | 38.30 | O |
| ATOM | 2171 | CB | SER | D | 64 | 21.812 | 77.151 | -15.076 | 1.00 | 31.57 | C |
| ATOM | 2172 | OG | SER | D | 64 | 22.505 | 76.104 | -15.730 | 1.00 | 44.67 | O |
| ATOM | 2173 | N | GLY | D | 65 | 18.868 | 76.454 | -15.349 | 1.00 | 34.85 | N |
| ATOM | 2174 | CA | GLY | D | 65 | 17.712 | 75.817 | -15.927 | 1.00 | 38.26 | C |
| ATOM | 2175 | C | GLY | D | 65 | 17.422 | 76.371 | -17.302 | 1.00 | 40.50 | C |
| ATOM | 2176 | O | GLY | D | 65 | 17.829 | 77.479 | -17.661 | 1.00 | 39.25 | O |
| ATOM | 2177 | N | SER | D | 66 | 16.706 | 75.570 | -18.071 | 1.00 | 33.85 | N |
| ATOM | 2178 | CA | SER | D | 66 | 16.204 | 76.050 | -19.344 | 1.00 | 35.96 | C |
| ATOM | 2179 | C | SER | D | 66 | 14.865 | 75.386 | -19.584 | 1.00 | 39.56 | C |
| ATOM | 2180 | O | SER | D | 66 | 14.493 | 74.412 | -18.914 | 1.00 | 36.55 | O |
| ATOM | 2181 | CB | SER | D | 66 | 17.164 | 75.765 | -20.490 | 1.00 | 36.16 | C |
| ATOM | 2182 | OG | SER | D | 66 | 17.463 | 74.379 | -20.517 | 1.00 | 43.70 | O |
| ATOM | 2183 | N | LYS | D | 67 | 14.132 | 75.959 | -20.522 | 1.00 | 31.53 | N |
| ATOM | 2184 | CA | LYS | D | 67 | 12.820 | 75.482 | -20.896 | 1.00 | 33.07 | C |
| ATOM | 2185 | C | LYS | D | 67 | 12.746 | 75.549 | -22.407 | 1.00 | 38.33 | C |
| ATOM | 2186 | O | LYS | D | 67 | 13.238 | 76.494 | -23.014 | 1.00 | 43.23 | O |
| ATOM | 2187 | CB | LYS | D | 67 | 11.690 | 76.313 | -20.249 | 1.00 | 32.47 | C |
| ATOM | 2188 | CG | LYS | D | 67 | 10.269 | 75.914 | -20.705 | 1.00 | 28.80 | C |
| ATOM | 2189 | CD | LYS | D | 67 | 9.194 | 76.736 | -20.001 | 1.00 | 35.59 | C |
| ATOM | 2190 | CE | LYS | D | 67 | 7.890 | 76.733 | -20.788 | 1.00 | 45.34 | C |
| ATOM | 2191 | NZ | LYS | D | 67 | 6.693 | 77.312 | -20.018 | 1.00 | 45.74 | N1+ |
| ATOM | 2192 | N | SER | D | 68 | 12.175 | 74.523 | -23.008 | 1.00 | 40.72 | N |
| ATOM | 2193 | CA | SER | D | 68 | 11.983 | 74.502 | -24.446 | 1.00 | 40.77 | C |
| ATOM | 2194 | C | SER | D | 68 | 10.705 | 73.722 | -24.702 | 1.00 | 40.33 | C |
| ATOM | 2195 | O | SER | D | 68 | 10.665 | 72.518 | -24.439 | 1.00 | 41.36 | O |
| ATOM | 2196 | CB | SER | D | 68 | 13.179 | 73.866 | -25.139 | 1.00 | 44.26 | C |
| ATOM | 2197 | OG | SER | D | 68 | 12.935 | 73.761 | -26.526 | 1.00 | 55.67 | O |
| ATOM | 2198 | N | GLY | D | 69 | 9.655 | 74.408 | -25.145 | 1.00 | 37.35 | N |

| | | | | | | | | | | | |
|---|---|---|---|---|---|---|---|---|---|---|---|
| ATOM | 2199 | CA | GLY | D | 69 | 8.413 | 73.715 | -25.466 | 1.00 | 34.14 | C |
| ATOM | 2200 | C | GLY | D | 69 | 7.800 | 73.055 | -24.242 | 1.00 | 37.93 | C |
| ATOM | 2201 | O | GLY | D | 69 | 7.543 | 73.700 | -23.220 | 1.00 | 34.02 | O |
| ATOM | 2202 | N | THR | D | 70 | 7.592 | 71.738 | -24.310 | 1.00 | 38.48 | N |
| ATOM | 2203 | CA | THR | D | 70 | 6.956 | 71.011 | -23.219 | 1.00 | 38.13 | C |
| ATOM | 2204 | C | THR | D | 70 | 7.967 | 70.318 | -22.304 | 1.00 | 39.79 | C |
| ATOM | 2205 | O | THR | D | 70 | 7.590 | 69.416 | -21.549 | 1.00 | 40.93 | O |
| ATOM | 2206 | CB | THR | D | 70 | 5.968 | 69.982 | -23.771 | 1.00 | 37.94 | C |
| ATOM | 2207 | OG1 | THR | D | 70 | 6.672 | 69.053 | -24.608 | 1.00 | 38.51 | O |
| ATOM | 2208 | CG2 | THR | D | 70 | 4.899 | 70.665 | -24.571 | 1.00 | 27.17 | C |
| ATOM | 2209 | N | SER | D | 71 | 9.240 | 70.702 | -22.357 | 1.00 | 32.96 | N |
| ATOM | 2210 | CA | SER | D | 71 | 10.202 | 70.129 | -21.431 | 1.00 | 40.52 | C |
| ATOM | 2211 | C | SER | D | 71 | 11.091 | 71.219 | -20.853 | 1.00 | 36.32 | C |
| ATOM | 2212 | O | SER | D | 71 | 11.231 | 72.312 | -21.411 | 1.00 | 44.52 | O |
| ATOM | 2213 | CB | SER | D | 71 | 11.049 | 69.031 | -22.089 | 1.00 | 48.49 | C |
| ATOM | 2214 | OG | SER | D | 71 | 11.991 | 69.599 | -22.976 | 1.00 | 58.32 | O |
| ATOM | 2215 | N | ALA | D | 72 | 11.645 | 70.915 | -19.686 | 1.00 | 31.74 | N |
| ATOM | 2216 | CA | ALA | D | 72 | 12.519 | 71.800 | -18.942 | 1.00 | 32.67 | C |
| ATOM | 2217 | C | ALA | D | 72 | 13.688 | 70.976 | -18.443 | 1.00 | 37.05 | C |
| ATOM | 2218 | O | ALA | D | 72 | 13.541 | 69.784 | -18.161 | 1.00 | 35.94 | O |
| ATOM | 2219 | CB | ALA | D | 72 | 11.796 | 72.461 | -17.758 | 1.00 | 29.67 | C |
| ATOM | 2220 | N | THR | D | 73 | 14.861 | 71.594 | -18.349 | 1.00 | 33.25 | N |
| ATOM | 2221 | CA | THR | D | 73 | 15.969 | 70.834 | -17.809 | 1.00 | 33.69 | C |
| ATOM | 2222 | C | THR | D | 73 | 16.765 | 71.667 | -16.813 | 1.00 | 36.67 | C |
| ATOM | 2223 | O | THR | D | 73 | 16.912 | 72.886 | -16.951 | 1.00 | 37.39 | O |
| ATOM | 2224 | CB | THR | D | 73 | 16.875 | 70.239 | -18.916 | 1.00 | 40.87 | C |
| ATOM | 2225 | OG1 | THR | D | 73 | 18.214 | 70.724 | -18.799 | 1.00 | 48.32 | O |
| ATOM | 2226 | CG2 | THR | D | 73 | 16.335 | 70.483 | -20.289 | 1.00 | 31.17 | C |
| ATOM | 2227 | N | LEU | D | 74 | 17.224 | 70.979 | -15.775 | 1.00 | 36.18 | N |
| ATOM | 2228 | CA | LEU | D | 74 | 18.084 | 71.536 | -14.749 | 1.00 | 40.71 | C |
| ATOM | 2229 | C | LEU | D | 74 | 19.514 | 71.068 | -15.005 | 1.00 | 39.16 | C |
| ATOM | 2230 | O | LEU | D | 74 | 19.747 | 69.887 | -15.269 | 1.00 | 36.86 | O |
| ATOM | 2231 | CB | LEU | D | 74 | 17.597 | 71.088 | -13.375 | 1.00 | 32.52 | C |
| ATOM | 2232 | CG | LEU | D | 74 | 18.632 | 71.088 | -12.262 | 1.00 | 33.76 | C |
| ATOM | 2233 | CD1 | LEU | D | 74 | 18.979 | 72.495 | -11.837 | 1.00 | 34.80 | C |
| ATOM | 2234 | CD2 | LEU | D | 74 | 18.078 | 70.285 | -11.105 | 1.00 | 28.46 | C |
| ATOM | 2235 | N | GLY | D | 75 | 20.456 | 71.992 | -14.964 | 1.00 | 39.23 | N |
| ATOM | 2236 | CA | GLY | D | 75 | 21.861 | 71.676 | -15.155 | 1.00 | 34.28 | C |
| ATOM | 2237 | C | GLY | D | 75 | 22.643 | 71.975 | -13.891 | 1.00 | 35.73 | C |
| ATOM | 2238 | O | GLY | D | 75 | 22.380 | 72.971 | -13.212 | 1.00 | 31.27 | O |
| ATOM | 2239 | N | ILE | D | 76 | 23.572 | 71.083 | -13.555 | 1.00 | 37.91 | N |
| ATOM | 2240 | CA | ILE | D | 76 | 24.460 | 71.270 | -12.416 | 1.00 | 37.96 | C |
| ATOM | 2241 | C | ILE | D | 76 | 25.880 | 71.043 | -12.900 | 1.00 | 44.61 | C |
| ATOM | 2242 | O | ILE | D | 76 | 26.245 | 69.912 | -13.243 | 1.00 | 45.05 | O |
| ATOM | 2243 | CB | ILE | D | 76 | 24.132 | 70.324 | -11.259 | 1.00 | 37.47 | C |
| ATOM | 2244 | CG1 | ILE | D | 76 | 22.612 | 70.258 | -11.056 | 1.00 | 38.58 | C |
| ATOM | 2245 | CG2 | ILE | D | 76 | 24.842 | 70.801 | -10.019 | 1.00 | 34.75 | C |
| ATOM | 2246 | CD1 | ILE | D | 76 | 22.182 | 69.396 | -9.926 | 1.00 | 35.25 | C |
| ATOM | 2247 | N | THR | D | 77 | 26.684 | 72.103 | -12.916 | 1.00 | 41.92 | N |
| ATOM | 2248 | CA | THR | D | 77 | 28.105 | 72.001 | -13.217 | 1.00 | 45.22 | C |
| ATOM | 2249 | C | THR | D | 77 | 28.911 | 71.995 | -11.922 | 1.00 | 49.05 | C |
| ATOM | 2250 | O | THR | D | 77 | 28.433 | 72.413 | -10.863 | 1.00 | 46.09 | O |
| ATOM | 2251 | CB | THR | D | 77 | 28.570 | 73.156 | -14.111 | 1.00 | 45.74 | C |
| ATOM | 2252 | OG1 | THR | D | 77 | 28.494 | 74.389 | -13.383 | 1.00 | 45.10 | O |
| ATOM | 2253 | CG2 | THR | D | 77 | 27.707 | 73.246 | -15.356 | 1.00 | 41.50 | C |
| ATOM | 2254 | N | GLY | D | 78 | 30.136 | 71.474 | -12.015 | 1.00 | 50.10 | N |
| ATOM | 2255 | CA | GLY | D | 78 | 31.054 | 71.450 | -10.893 | 1.00 | 40.32 | C |
| ATOM | 2256 | C | GLY | D | 78 | 30.509 | 70.763 | -9.660 | 1.00 | 47.60 | C |
| ATOM | 2257 | O | GLY | D | 78 | 30.599 | 71.321 | -8.562 | 1.00 | 46.13 | O |
| ATOM | 2258 | N | LEU | D | 79 | 29.977 | 69.545 | -9.822 | 1.00 | 44.28 | N |
| ATOM | 2259 | CA | LEU | D | 79 | 29.291 | 68.851 | -8.735 | 1.00 | 41.62 | C |
| ATOM | 2260 | C | LEU | D | 79 | 30.097 | 68.875 | -7.445 | 1.00 | 41.28 | C |
| ATOM | 2261 | O | LEU | D | 79 | 31.306 | 68.625 | -7.445 | 1.00 | 43.99 | O |
| ATOM | 2262 | CB | LEU | D | 79 | 29.044 | 67.391 | -9.108 | 1.00 | 44.54 | C |
| ATOM | 2263 | CG | LEU | D | 79 | 27.718 | 66.829 | -9.591 | 1.00 | 41.80 | C |
| ATOM | 2264 | CD1 | LEU | D | 79 | 26.548 | 67.743 | -9.317 | 1.00 | 41.01 | C |
| ATOM | 2265 | CD2 | LEU | D | 79 | 27.807 | 66.456 | -11.048 | 1.00 | 48.29 | C |
| ATOM | 2266 | N | GLN | D | 80 | 29.407 | 69.147 | -6.341 | 1.00 | 38.12 | N |
| ATOM | 2267 | CA | GLN | D | 80 | 29.955 | 69.025 | -5.000 | 1.00 | 37.63 | C |
| ATOM | 2268 | C | GLN | D | 80 | 29.166 | 67.992 | -4.207 | 1.00 | 39.83 | C |
| ATOM | 2269 | O | GLN | D | 80 | 27.975 | 67.771 | -4.461 | 1.00 | 44.36 | O |

| | | | | | | | | | | | |
|---|---|---|---|---|---|---|---|---|---|---|---|
| ATOM | 2270 | CB | GLN | D | 80 | 29.925 | 70.349 | -4.277 | 1.00 | 35.93 | C |
| ATOM | 2271 | CG | GLN | D | 80 | 30.598 | 71.424 | -5.072 | 1.00 | 44.41 | C |
| ATOM | 2272 | CD | GLN | D | 80 | 30.602 | 72.746 | -4.351 | 1.00 | 48.07 | C |
| ATOM | 2273 | OE1 | GLN | D | 80 | 30.141 | 72.848 | -3.211 | 1.00 | 51.38 | O |
| ATOM | 2274 | NE2 | GLN | D | 80 | 31.108 | 73.778 | -5.018 | 1.00 | 49.68 | N |
| ATOM | 2275 | N | THR | D | 81 | 29.838 | 67.362 | -3.236 | 1.00 | 39.23 | N |
| ATOM | 2276 | CA | THR | D | 81 | 29.185 | 66.313 | -2.459 | 1.00 | 43.43 | C |
| ATOM | 2277 | C | THR | D | 81 | 27.905 | 66.827 | -1.825 | 1.00 | 38.49 | C |
| ATOM | 2278 | O | THR | D | 81 | 26.949 | 66.066 | -1.660 | 1.00 | 43.22 | O |
| ATOM | 2279 | CB | THR | D | 81 | 30.122 | 65.740 | -1.386 | 1.00 | 33.08 | C |
| ATOM | 2280 | OG1 | THR | D | 81 | 30.589 | 66.796 | -0.558 | 1.00 | 47.69 | O |
| ATOM | 2281 | CG2 | THR | D | 81 | 31.307 | 65.035 | -2.020 | 1.00 | 35.64 | C |
| ATOM | 2282 | N | GLY | D | 82 | 27.859 | 68.112 | -1.498 | 1.00 | 35.25 | N |
| ATOM | 2283 | CA | GLY | D | 82 | 26.664 | 68.722 | -0.952 | 1.00 | 40.04 | C |
| ATOM | 2284 | C | GLY | D | 82 | 25.510 | 68.895 | -1.929 | 1.00 | 38.60 | C |
| ATOM | 2285 | O | GLY | D | 82 | 24.437 | 69.339 | -1.510 | 1.00 | 35.12 | O |
| ATOM | 2286 | N | ASP | D | 83 | 25.694 | 68.543 | -3.200 | 1.00 | 33.43 | N |
| ATOM | 2287 | CA | ASP | D | 83 | 24.619 | 68.564 | -4.179 | 1.00 | 37.84 | C |
| ATOM | 2288 | C | ASP | D | 83 | 23.834 | 67.251 | -4.245 | 1.00 | 43.45 | C |
| ATOM | 2289 | O | ASP | D | 83 | 22.814 | 67.191 | -4.957 | 1.00 | 40.21 | O |
| ATOM | 2290 | CB | ASP | D | 83 | 25.181 | 68.882 | -5.566 | 1.00 | 33.25 | C |
| ATOM | 2291 | CG | ASP | D | 83 | 25.971 | 70.173 | -5.593 | 1.00 | 42.35 | C |
| ATOM | 2292 | OD1 | ASP | D | 83 | 25.680 | 71.085 | -4.788 | 1.00 | 36.88 | O |
| ATOM | 2293 | OD2 | ASP | D | 83 | 26.882 | 70.284 | -6.440 | 1.00 | 44.43 | O1- |
| ATOM | 2294 | N | GLU | D | 84 | 24.310 | 66.192 | -3.577 | 1.00 | 37.08 | N |
| ATOM | 2295 | CA | GLU | D | 84 | 23.578 | 64.931 | -3.529 | 1.00 | 38.14 | C |
| ATOM | 2296 | C | GLU | D | 84 | 22.192 | 65.159 | -2.929 | 1.00 | 37.73 | C |
| ATOM | 2297 | O | GLU | D | 84 | 22.075 | 65.642 | -1.800 | 1.00 | 36.95 | O |
| ATOM | 2298 | CB | GLU | D | 84 | 24.359 | 63.904 | -2.708 | 1.00 | 33.68 | C |
| ATOM | 2299 | CG | GLU | D | 84 | 23.683 | 62.544 | -2.653 | 1.00 | 41.33 | C |
| ATOM | 2300 | CD | GLU | D | 84 | 24.682 | 61.389 | -2.458 | 1.00 | 46.09 | C |
| ATOM | 2301 | OE1 | GLU | D | 84 | 24.374 | 60.429 | -1.716 | 1.00 | 49.48 | O |
| ATOM | 2302 | OE2 | GLU | D | 84 | 25.766 | 61.427 | -3.078 | 1.00 | 45.22 | O1- |
| ATOM | 2303 | N | ALA | D | 85 | 21.146 | 64.836 | -3.688 | 1.00 | 31.07 | N |
| ATOM | 2304 | CA | ALA | D | 85 | 19.786 | 65.206 | -3.298 | 1.00 | 29.56 | C |
| ATOM | 2305 | C | ALA | D | 85 | 18.818 | 64.596 | -4.293 | 1.00 | 32.77 | C |
| ATOM | 2306 | O | ALA | D | 85 | 19.218 | 64.022 | -5.314 | 1.00 | 32.03 | O |
| ATOM | 2307 | CB | ALA | D | 85 | 19.590 | 66.729 | -3.227 | 1.00 | 30.06 | C |
| ATOM | 2308 | N | ASP | D | 86 | 17.531 | 64.697 | -3.959 | 1.00 | 30.95 | N |
| ATOM | 2309 | CA | ASP | D | 86 | 16.457 | 64.474 | -4.921 | 1.00 | 30.44 | C |
| ATOM | 2310 | C | ASP | D | 86 | 16.015 | 65.817 | -5.481 | 1.00 | 33.26 | C |
| ATOM | 2311 | O | ASP | D | 86 | 15.958 | 66.816 | -4.752 | 1.00 | 29.91 | O |
| ATOM | 2312 | CB | ASP | D | 86 | 15.261 | 63.780 | -4.283 | 1.00 | 36.23 | C |
| ATOM | 2313 | CG | ASP | D | 86 | 15.600 | 62.414 | -3.757 | 1.00 | 39.79 | C |
| ATOM | 2314 | OD1 | ASP | D | 86 | 16.287 | 61.640 | -4.460 | 1.00 | 49.35 | O |
| ATOM | 2315 | OD2 | ASP | D | 86 | 15.202 | 62.134 | -2.620 | 1.00 | 52.11 | O1- |
| ATOM | 2316 | N | TYR | D | 87 | 15.723 | 65.834 | -6.775 | 1.00 | 28.24 | N |
| ATOM | 2317 | CA | TYR | D | 87 | 15.349 | 67.045 | -7.481 | 1.00 | 30.37 | C |
| ATOM | 2318 | C | TYR | D | 87 | 13.993 | 66.832 | -8.116 | 1.00 | 30.53 | C |
| ATOM | 2319 | O | TYR | D | 87 | 13.736 | 65.781 | -8.716 | 1.00 | 31.12 | O |
| ATOM | 2320 | CB | TYR | D | 87 | 16.407 | 67.425 | -8.550 | 1.00 | 31.35 | C |
| ATOM | 2321 | CG | TYR | D | 87 | 17.707 | 67.860 | -7.930 | 1.00 | 28.53 | C |
| ATOM | 2322 | CD2 | TYR | D | 87 | 18.711 | 66.933 | -7.628 | 1.00 | 29.65 | C |
| ATOM | 2323 | CD1 | TYR | D | 87 | 17.919 | 69.192 | -7.596 | 1.00 | 30.97 | C |
| ATOM | 2324 | CE2 | TYR | D | 87 | 19.913 | 67.350 | -7.040 | 1.00 | 36.12 | C |
| ATOM | 2325 | CE1 | TYR | D | 87 | 19.110 | 69.620 | -6.999 | 1.00 | 32.34 | C |
| ATOM | 2326 | CZ | TYR | D | 87 | 20.101 | 68.700 | -6.728 | 1.00 | 36.00 | C |
| ATOM | 2327 | OH | TYR | D | 87 | 21.261 | 69.124 | -6.123 | 1.00 | 36.35 | O |
| ATOM | 2328 | N | TYR | D | 88 | 13.130 | 67.833 | -7.986 | 1.00 | 31.77 | N |
| ATOM | 2329 | CA | TYR | D | 88 | 11.763 | 67.756 | -8.495 | 1.00 | 30.69 | C |
| ATOM | 2330 | C | TYR | D | 88 | 11.442 | 68.977 | -9.350 | 1.00 | 33.16 | C |
| ATOM | 2331 | O | TYR | D | 88 | 11.697 | 70.123 | -8.943 | 1.00 | 27.49 | O |
| ATOM | 2332 | CB | TYR | D | 88 | 10.755 | 67.662 | -7.330 | 1.00 | 28.47 | C |
| ATOM | 2333 | CG | TYR | D | 88 | 10.863 | 66.418 | -6.469 | 1.00 | 32.12 | C |
| ATOM | 2334 | CD1 | TYR | D | 88 | 10.157 | 65.254 | -6.797 | 1.00 | 33.44 | C |
| ATOM | 2335 | CD2 | TYR | D | 88 | 11.639 | 66.407 | -5.312 | 1.00 | 35.30 | C |
| ATOM | 2336 | CE1 | TYR | D | 88 | 10.246 | 64.107 | -6.011 | 1.00 | 33.26 | C |
| ATOM | 2337 | CE2 | TYR | D | 88 | 11.735 | 65.256 | -4.505 | 1.00 | 28.88 | C |
| ATOM | 2338 | CZ | TYR | D | 88 | 11.038 | 64.121 | -4.860 | 1.00 | 35.12 | C |
| ATOM | 2339 | OH | TYR | D | 88 | 11.138 | 63.001 | -4.067 | 1.00 | 41.20 | O |
| ATOM | 2340 | N | CYS | D | 89 | 10.860 | 68.743 | -10.520 | 1.00 | 29.10 | N |

| | | | | | | | | | | | |
|---|---|---|---|---|---|---|---|---|---|---|---|
| ATOM | 2341 | CA | CYS | D | 89 | 10.217 | 69.855 | -11.206 | 1.00 | 33.19 | C |
| ATOM | 2342 | C | CYS | D | 89 | 8.723 | 69.907 | -10.850 | 1.00 | 37.48 | C |
| ATOM | 2343 | O | CYS | D | 89 | 8.110 | 68.906 | -10.450 | 1.00 | 33.89 | O |
| ATOM | 2344 | CB | CYS | D | 89 | 10.397 | 69.751 | -12.722 | 1.00 | 35.57 | C |
| ATOM | 2345 | SG | CYS | D | 89 | 9.788 | 68.160 | -13.353 | 1.00 | 50.43 | S |
| ATOM | 2346 | N | GLY | D | 90 | 8.153 | 71.103 | -10.985 | 1.00 | 35.51 | N |
| ATOM | 2347 | CA | GLY | D | 90 | 6.747 | 71.329 | -10.696 | 1.00 | 31.51 | C |
| ATOM | 2348 | C | GLY | D | 90 | 6.184 | 72.474 | -11.510 | 1.00 | 32.91 | C |
| ATOM | 2349 | O | GLY | D | 90 | 6.886 | 73.440 | -11.821 | 1.00 | 37.81 | O |
| ATOM | 2350 | N | THR | D | 91 | 4.905 | 72.347 | -11.876 | 1.00 | 29.44 | N |
| ATOM | 2351 | CA | THR | D | 91 | 4.112 | 73.391 | -12.523 | 1.00 | 35.31 | C |
| ATOM | 2352 | C | THR | D | 91 | 2.648 | 73.192 | -12.187 | 1.00 | 34.83 | C |
| ATOM | 2353 | O | THR | D | 91 | 2.252 | 72.229 | -11.533 | 1.00 | 31.62 | O |
| ATOM | 2354 | CB | THR | D | 91 | 4.039 | 73.364 | -14.063 | 1.00 | 36.67 | C |
| ATOM | 2355 | OG1 | THR | D | 91 | 4.722 | 72.256 | -14.635 | 1.00 | 42.40 | O |
| ATOM | 2356 | CG2 | THR | D | 91 | 4.377 | 74.637 | -14.683 | 1.00 | 25.98 | C |
| ATOM | 2357 | N | TRP | D | 92 | 1.845 | 74.047 | -12.800 | 1.00 | 33.75 | N |
| ATOM | 2358 | CA | TRP | D | 92 | 0.422 | 73.882 | -12.929 | 1.00 | 32.47 | C |
| ATOM | 2359 | C | TRP | D | 92 | 0.088 | 73.103 | -14.202 | 1.00 | 34.81 | C |
| ATOM | 2360 | O | TRP | D | 92 | 0.757 | 73.209 | -15.235 | 1.00 | 30.64 | O |
| ATOM | 2361 | CB | TRP | D | 92 | -0.234 | 75.251 | -12.956 | 1.00 | 33.91 | C |
| ATOM | 2362 | CG | TRP | D | 92 | -1.719 | 75.251 | -12.940 | 1.00 | 35.91 | C |
| ATOM | 2363 | CD1 | TRP | D | 92 | -2.553 | 75.591 | -13.969 | 1.00 | 31.74 | C |
| ATOM | 2364 | CD2 | TRP | D | 92 | -2.557 | 74.949 | -11.818 | 1.00 | 34.97 | C |
| ATOM | 2365 | NE1 | TRP | D | 92 | -3.860 | 75.519 | -13.554 | 1.00 | 37.78 | N |
| ATOM | 2366 | CE2 | TRP | D | 92 | -3.893 | 75.127 | -12.238 | 1.00 | 39.89 | C |
| ATOM | 2367 | CE3 | TRP | D | 92 | -2.308 | 74.567 | -10.494 | 1.00 | 27.09 | C |
| ATOM | 2368 | CZ2 | TRP | D | 92 | -4.979 | 74.921 | -11.380 | 1.00 | 36.03 | C |
| ATOM | 2369 | CZ3 | TRP | D | 92 | -3.384 | 74.357 | -9.643 | 1.00 | 35.46 | C |
| ATOM | 2370 | CH2 | TRP | D | 92 | -4.706 | 74.542 | -10.087 | 1.00 | 35.32 | C |
| ATOM | 2371 | N | ASP | D | 93 | -0.943 | 72.296 | -14.109 | 1.00 | 32.62 | N |
| ATOM | 2372 | CA | ASP | D | 93 | -1.540 | 71.671 | -15.277 | 1.00 | 38.27 | C |
| ATOM | 2373 | C | ASP | D | 93 | -2.925 | 72.275 | -15.476 | 1.00 | 37.51 | C |
| ATOM | 2374 | O | ASP | D | 93 | -3.809 | 72.089 | -14.634 | 1.00 | 39.63 | O |
| ATOM | 2375 | CB | ASP | D | 93 | -1.609 | 70.162 | -15.121 | 1.00 | 37.98 | C |
| ATOM | 2376 | CG | ASP | D | 93 | -2.016 | 69.490 | -16.396 | 1.00 | 37.56 | C |
| ATOM | 2377 | OD1 | ASP | D | 93 | -3.109 | 69.827 | -16.919 | 1.00 | 38.96 | O1- |
| ATOM | 2378 | OD2 | ASP | D | 93 | -1.226 | 68.659 | -16.884 | 1.00 | 37.13 | O |
| ATOM | 2379 | N | SER | D | 94 | -3.094 | 73.010 | -16.583 | 1.00 | 40.55 | N |
| ATOM | 2380 | CA | SER | D | 94 | -4.324 | 73.756 | -16.838 | 1.00 | 43.35 | C |
| ATOM | 2381 | C | SER | D | 94 | -5.502 | 72.843 | -17.132 | 1.00 | 41.15 | C |
| ATOM | 2382 | O | SER | D | 94 | -6.643 | 73.204 | -16.848 | 1.00 | 47.84 | O |
| ATOM | 2383 | CB | SER | D | 94 | -4.124 | 74.716 | -18.009 | 1.00 | 37.93 | C |
| ATOM | 2384 | OG | SER | D | 94 | -3.093 | 75.653 | -17.720 | 1.00 | 50.29 | O |
| ATOM | 2385 | N | SER | D | 95 | -5.261 | 71.659 | -17.656 | 1.00 | 39.23 | N |
| ATOM | 2386 | CA | SER | D | 95 | -6.392 | 70.814 | -17.991 | 1.00 | 45.37 | C |
| ATOM | 2387 | C | SER | D | 95 | -6.795 | 69.916 | -16.843 | 1.00 | 44.47 | C |
| ATOM | 2388 | O | SER | D | 95 | -7.981 | 69.617 | -16.704 | 1.00 | 52.00 | O |
| ATOM | 2389 | CB | SER | D | 95 | -6.084 | 69.960 | -19.223 | 1.00 | 44.25 | C |
| ATOM | 2390 | OG | SER | D | 95 | -5.390 | 68.785 | -18.856 | 1.00 | 58.75 | O |
| ATOM | 2391 | N | LEU | D | 96 | -5.845 | 69.487 | -16.012 | 1.00 | 43.88 | N |
| ATOM | 2392 | CA | LEU | D | 96 | -6.173 | 68.775 | -14.780 | 1.00 | 37.73 | C |
| ATOM | 2393 | C | LEU | D | 96 | -6.491 | 69.723 | -13.621 | 1.00 | 36.61 | C |
| ATOM | 2394 | O | LEU | D | 96 | -6.885 | 69.255 | -12.550 | 1.00 | 38.29 | O |
| ATOM | 2395 | CB | LEU | D | 96 | -5.019 | 67.839 | -14.391 | 1.00 | 41.42 | C |
| ATOM | 2396 | CG | LEU | D | 96 | -4.577 | 66.750 | -15.380 | 1.00 | 39.10 | C |
| ATOM | 2397 | CD1 | LEU | D | 96 | -3.261 | 66.160 | -14.963 | 1.00 | 34.94 | C |
| ATOM | 2398 | CD2 | LEU | D | 96 | -5.612 | 65.640 | -15.506 | 1.00 | 35.48 | C |
| ATOM | 2399 | N | ASN | D | 97 | -6.353 | 71.034 | -13.811 | 1.00 | 37.50 | N |
| ATOM | 2400 | CA | ASN | D | 97 | -6.524 | 72.032 | -12.750 | 1.00 | 39.48 | C |
| ATOM | 2401 | C | ASN | D | 97 | -5.885 | 71.606 | -11.418 | 1.00 | 35.80 | C |
| ATOM | 2402 | O | ASN | D | 97 | -6.519 | 71.581 | -10.367 | 1.00 | 35.42 | O |
| ATOM | 2403 | CB | ASN | D | 97 | -8.002 | 72.403 | -12.591 | 1.00 | 33.46 | C |
| ATOM | 2404 | CG | ASN | D | 97 | -8.304 | 73.766 | -13.211 | 1.00 | 59.81 | C |
| ATOM | 2405 | OD1 | ASN | D | 97 | -8.600 | 74.731 | -12.490 | 1.00 | 63.64 | O |
| ATOM | 2406 | ND2 | ASN | D | 97 | -8.104 | 73.888 | -14.535 | 1.00 | 48.78 | N |
| ATOM | 2407 | N | THR | D | 98 | -4.584 | 71.319 | -11.468 | 1.00 | 34.96 | N |
| ATOM | 2408 | CA | THR | D | 98 | -3.880 | 70.883 | -10.267 | 1.00 | 40.51 | C |
| ATOM | 2409 | C | THR | D | 98 | -2.389 | 71.197 | -10.382 | 1.00 | 33.13 | C |
| ATOM | 2410 | O | THR | D | 98 | -1.843 | 71.349 | -11.482 | 1.00 | 33.25 | O |
| ATOM | 2411 | CB | THR | D | 98 | -4.095 | 69.378 | -10.000 | 1.00 | 31.21 | C |

| | | | | | | | | | | | |
|---|---|---|---|---|---|---|---|---|---|---|---|
| ATOM | 2412 | OG1 | THR | D | 98 | -3.498 | 69.048 | -8.749 | 1.00 | 36.06 | O |
| ATOM | 2413 | CG2 | THR | D | 98 | -3.428 | 68.531 | -11.061 | 1.00 | 31.76 | C |
| ATOM | 2414 | N | VAL | D | 99 | -1.730 | 71.290 | -9.222 | 1.00 | 29.98 | N |
| ATOM | 2415 | CA | VAL | D | 99 | -0.267 | 71.342 | -9.211 | 1.00 | 34.24 | C |
| ATOM | 2416 | C | VAL | D | 99 | 0.296 | 69.972 | -9.558 | 1.00 | 34.16 | C |
| ATOM | 2417 | O | VAL | D | 99 | -0.215 | 68.936 | -9.120 | 1.00 | 34.33 | O |
| ATOM | 2418 | CB | VAL | D | 99 | 0.278 | 71.796 | -7.849 | 1.00 | 33.92 | C |
| ATOM | 2419 | CG1 | VAL | D | 99 | 1.787 | 71.619 | -7.819 | 1.00 | 30.13 | C |
| ATOM | 2420 | CG2 | VAL | D | 99 | -0.114 | 73.241 | -7.535 | 1.00 | 28.38 | C |
| ATOM | 2421 | N | VAL | D | 100 | 1.375 | 69.952 | -10.321 | 1.00 | 29.37 | N |
| ATOM | 2422 | CA | VAL | D | 100 | 1.871 | 68.706 | -10.875 | 1.00 | 30.81 | C |
| ATOM | 2423 | C | VAL | D | 100 | 3.380 | 68.659 | -10.653 | 1.00 | 33.23 | C |
| ATOM | 2424 | O | VAL | D | 100 | 4.072 | 69.662 | -10.890 | 1.00 | 32.58 | O |
| ATOM | 2425 | CB | VAL | D | 100 | 1.442 | 68.618 | -12.352 | 1.00 | 32.14 | C |
| ATOM | 2426 | CG1 | VAL | D | 100 | 2.538 | 68.362 | -13.255 | 1.00 | 38.21 | C |
| ATOM | 2427 | CG2 | VAL | D | 100 | 0.327 | 67.591 | -12.507 | 1.00 | 35.01 | C |
| ATOM | 2428 | N | PHE | D | 101 | 3.869 | 67.537 | -10.090 | 1.00 | 32.44 | N |
| ATOM | 2429 | CA | PHE | D | 101 | 5.292 | 67.296 | -9.826 | 1.00 | 28.74 | C |
| ATOM | 2430 | C | PHE | D | 101 | 5.824 | 66.191 | -10.728 | 1.00 | 30.41 | C |
| ATOM | 2431 | O | PHE | D | 101 | 5.091 | 65.280 | -11.116 | 1.00 | 36.07 | O |
| ATOM | 2432 | CB | PHE | D | 101 | 5.571 | 66.879 | -8.367 | 1.00 | 29.14 | C |
| ATOM | 2433 | CG | PHE | D | 101 | 5.505 | 68.006 | -7.372 | 1.00 | 29.14 | C |
| ATOM | 2434 | CD1 | PHE | D | 101 | 6.472 | 68.991 | -7.355 | 1.00 | 29.41 | C |
| ATOM | 2435 | CD2 | PHE | D | 101 | 4.483 | 68.067 | -6.438 | 1.00 | 29.40 | C |
| ATOM | 2436 | CE1 | PHE | D | 101 | 6.421 | 70.041 | -6.430 | 1.00 | 30.71 | C |
| ATOM | 2437 | CE2 | PHE | D | 101 | 4.412 | 69.107 | -5.519 | 1.00 | 31.48 | C |
| ATOM | 2438 | CZ | PHE | D | 101 | 5.388 | 70.100 | -5.509 | 1.00 | 28.28 | C |
| ATOM | 2439 | N | GLY | D | 102 | 7.112 | 66.269 | -11.069 | 1.00 | 35.21 | N |
| ATOM | 2440 | CA | GLY | D | 102 | 7.774 | 65.104 | -11.621 | 1.00 | 30.07 | C |
| ATOM | 2441 | C | GLY | D | 102 | 7.949 | 64.054 | -10.539 | 1.00 | 34.24 | C |
| ATOM | 2442 | O | GLY | D | 102 | 7.809 | 64.324 | -9.342 | 1.00 | 32.05 | O |
| ATOM | 2443 | N | GLY | D | 103 | 8.282 | 62.833 | -10.962 | 1.00 | 33.87 | N |
| ATOM | 2444 | CA | GLY | D | 103 | 8.500 | 61.756 | -9.998 | 1.00 | 30.14 | C |
| ATOM | 2445 | C | GLY | D | 103 | 9.767 | 61.896 | -9.164 | 1.00 | 33.06 | C |
| ATOM | 2446 | O | GLY | D | 103 | 9.929 | 61.153 | -8.195 | 1.00 | 33.57 | O |
| ATOM | 2447 | N | GLY | D | 104 | 10.649 | 62.840 | -9.500 | 1.00 | 34.22 | N |
| ATOM | 2448 | CA | GLY | D | 104 | 11.866 | 63.034 | -8.746 | 1.00 | 30.60 | C |
| ATOM | 2449 | C | GLY | D | 104 | 13.056 | 62.330 | -9.363 | 1.00 | 32.84 | C |
| ATOM | 2450 | O | GLY | D | 104 | 12.939 | 61.230 | -9.914 | 1.00 | 37.98 | O |
| ATOM | 2451 | N | THR | D | 105 | 14.219 | 62.953 | -9.267 | 1.00 | 33.92 | N |
| ATOM | 2452 | CA | THR | D | 105 | 15.460 | 62.372 | -9.749 | 1.00 | 32.14 | C |
| ATOM | 2453 | C | THR | D | 105 | 16.422 | 62.308 | -8.581 | 1.00 | 35.31 | C |
| ATOM | 2454 | O | THR | D | 105 | 16.683 | 63.335 | -7.945 | 1.00 | 33.86 | O |
| ATOM | 2455 | CB | THR | D | 105 | 16.052 | 63.202 | -10.878 | 1.00 | 35.69 | C |
| ATOM | 2456 | OG1 | THR | D | 105 | 15.118 | 63.236 | -11.966 | 1.00 | 33.00 | O |
| ATOM | 2457 | CG2 | THR | D | 105 | 17.374 | 62.575 | -11.328 | 1.00 | 32.50 | C |
| ATOM | 2458 | N | LYS | D | 106 | 16.895 | 61.095 | -8.260 | 1.00 | 35.10 | N |
| ATOM | 2459 | CA | LYS | D | 106 | 17.920 | 60.928 | -7.232 | 1.00 | 39.20 | C |
| ATOM | 2460 | C | LYS | D | 106 | 19.287 | 61.212 | -7.857 | 1.00 | 35.77 | C |
| ATOM | 2461 | O | LYS | D | 106 | 19.709 | 60.517 | -8.796 | 1.00 | 33.65 | O |
| ATOM | 2462 | CB | LYS | D | 106 | 17.875 | 59.523 | -6.612 | 1.00 | 37.13 | C |
| ATOM | 2463 | CG | LYS | D | 106 | 18.991 | 59.257 | -5.575 | 1.00 | 41.72 | C |
| ATOM | 2464 | CD | LYS | D | 106 | 18.843 | 57.864 | -4.893 | 1.00 | 66.03 | C |
| ATOM | 2465 | CE | LYS | D | 106 | 20.060 | 57.472 | -4.002 | 1.00 | 43.48 | C |
| ATOM | 2466 | NZ | LYS | D | 106 | 20.399 | 58.527 | -2.969 | 1.00 | 59.67 | N1+ |
| ATOM | 2467 | N | LEU | D | 107 | 19.955 | 62.253 | -7.355 | 1.00 | 37.32 | N |
| ATOM | 2468 | CA | LEU | D | 107 | 21.313 | 62.606 | -7.757 | 1.00 | 39.36 | C |
| ATOM | 2469 | C | LEU | D | 107 | 22.264 | 62.033 | -6.720 | 1.00 | 37.65 | C |
| ATOM | 2470 | O | LEU | D | 107 | 22.211 | 62.419 | -5.542 | 1.00 | 38.47 | O |
| ATOM | 2471 | CB | LEU | D | 107 | 21.518 | 64.129 | -7.855 | 1.00 | 26.67 | C |
| ATOM | 2472 | CG | LEU | D | 107 | 22.784 | 64.731 | -8.570 | 1.00 | 37.81 | C |
| ATOM | 2473 | CD1 | LEU | D | 107 | 23.134 | 66.182 | -8.167 | 1.00 | 38.40 | C |
| ATOM | 2474 | CD2 | LEU | D | 107 | 24.043 | 63.923 | -8.466 | 1.00 | 34.25 | C |
| ATOM | 2475 | N | THR | D | 108 | 23.176 | 61.171 | -7.148 | 1.00 | 30.15 | N |
| ATOM | 2476 | CA | THR | D | 108 | 24.270 | 60.823 | -6.255 | 1.00 | 39.12 | C |
| ATOM | 2477 | C | THR | D | 108 | 25.591 | 61.351 | -6.810 | 1.00 | 33.08 | C |
| ATOM | 2478 | O | THR | D | 108 | 25.888 | 61.234 | -8.004 | 1.00 | 36.87 | O |
| ATOM | 2479 | CB | THR | D | 108 | 24.311 | 59.316 | -5.951 | 1.00 | 42.14 | C |
| ATOM | 2480 | OG1 | THR | D | 108 | 25.514 | 58.733 | -6.459 | 1.00 | 51.43 | O |
| ATOM | 2481 | CG2 | THR | D | 108 | 23.075 | 58.603 | -6.471 | 1.00 | 39.89 | C |
| ATOM | 2482 | N | VAL | D | 109 | 26.338 | 62.011 | -5.950 | 1.00 | 28.89 | N |

| | | | | | | | | | | | |
|---|---|---|---|---|---|---|---|---|---|---|---|
| ATOM | 2483 | CA | VAL | D | 109 | 27.679 | 62.461 | -6.272 | 1.00 | 36.64 | C |
| ATOM | 2484 | C | VAL | D | 109 | 28.632 | 61.337 | -5.873 | 1.00 | 36.03 | C |
| ATOM | 2485 | O | VAL | D | 109 | 28.838 | 61.088 | -4.686 | 1.00 | 34.38 | O |
| ATOM | 2486 | CB | VAL | D | 109 | 27.999 | 63.769 | -5.547 | 1.00 | 39.07 | C |
| ATOM | 2487 | CG1 | VAL | D | 109 | 29.402 | 64.264 | -5.930 | 1.00 | 39.69 | C |
| ATOM | 2488 | CG2 | VAL | D | 109 | 26.914 | 64.807 | -5.871 | 1.00 | 39.03 | C |
| ATOM | 2489 | N | LEU | D | 110 | 29.179 | 60.637 | -6.868 | 1.00 | 36.80 | N |
| ATOM | 2490 | CA | LEU | D | 110 | 29.935 | 59.407 | -6.646 | 1.00 | 35.35 | C |
| ATOM | 2491 | C | LEU | D | 110 | 31.166 | 59.653 | -5.794 | 1.00 | 38.39 | C |
| ATOM | 2492 | O | LEU | D | 110 | 32.109 | 60.294 | -6.255 | 1.00 | 39.60 | O |
| ATOM | 2493 | CB | LEU | D | 110 | 30.358 | 58.813 | -7.978 | 1.00 | 31.68 | C |
| ATOM | 2494 | CG | LEU | D | 110 | 29.190 | 58.470 | -8.886 | 1.00 | 42.08 | C |
| ATOM | 2495 | CD1 | LEU | D | 110 | 29.720 | 58.230 | -10.264 | 1.00 | 33.32 | C |
| ATOM | 2496 | CD2 | LEU | D | 110 | 28.489 | 57.239 | -8.343 | 1.00 | 40.75 | C |
| ATOM | 2497 | N | SER | D | 111 | 31.182 | 59.144 | -4.564 | 1.00 | 38.34 | N |
| ATOM | 2498 | CA | SER | D | 111 | 32.330 | 59.296 | -3.680 | 1.00 | 44.10 | C |
| ATOM | 2499 | C | SER | D | 111 | 32.991 | 57.956 | -3.361 | 1.00 | 43.27 | C |
| ATOM | 2500 | O | SER | D | 111 | 33.843 | 57.885 | -2.476 | 1.00 | 38.62 | O |
| ATOM | 2501 | CB | SER | D | 111 | 31.917 | 60.001 | -2.396 | 1.00 | 39.42 | C |
| ATOM | 2502 | OG | SER | D | 111 | 30.920 | 59.235 | -1.763 | 1.00 | 48.72 | O |
| ATOM | 2503 | N | GLN | D | 112 | 32.606 | 56.901 | -4.058 | 1.00 | 36.34 | N |
| ATOM | 2504 | CA | GLN | D | 112 | 33.300 | 55.623 | -4.010 | 1.00 | 36.47 | C |
| ATOM | 2505 | C | GLN | D | 112 | 32.964 | 54.900 | -5.300 | 1.00 | 39.73 | C |
| ATOM | 2506 | O | GLN | D | 112 | 32.074 | 55.336 | -6.045 | 1.00 | 41.47 | O |
| ATOM | 2507 | CB | GLN | D | 112 | 32.884 | 54.798 | -2.774 | 1.00 | 34.11 | C |
| ATOM | 2508 | CG | GLN | D | 112 | 31.427 | 54.370 | -2.745 | 1.00 | 40.26 | C |
| ATOM | 2509 | CD | GLN | D | 112 | 31.098 | 53.480 | -1.545 | 1.00 | 44.54 | C |
| ATOM | 2510 | OE1 | GLN | D | 112 | 31.422 | 52.289 | -1.542 | 1.00 | 49.63 | O |
| ATOM | 2511 | NE2 | GLN | D | 112 | 30.446 | 54.054 | -0.523 | 1.00 | 32.71 | N |
| ATOM | 2512 | N | PRO | D | 113 | 33.662 | 53.816 | -5.614 | 1.00 | 35.83 | GZ00 N |
| ATOM | 2513 | CA | PRO | D | 113 | 33.350 | 53.105 | -6.859 | 1.00 | 29.36 | GZ00 C |
| ATOM | 2514 | C | PRO | D | 113 | 31.937 | 52.559 | -6.824 | 1.00 | 41.40 | GZ00 C |
| ATOM | 2515 | O | PRO | D | 113 | 31.391 | 52.249 | -5.763 | 1.00 | 33.72 | GZ00 O |
| ATOM | 2516 | CB | PRO | D | 113 | 34.371 | 51.969 | -6.897 | 1.00 | 29.06 | GZ00 C |
| ATOM | 2517 | CG | PRO | D | 113 | 35.545 | 52.491 | -6.029 | 1.00 | 36.02 | GZ00 C |
| ATOM | 2518 | CD | PRO | D | 113 | 34.878 | 53.300 | -4.948 | 1.00 | 37.13 | GZ00 C |
| ATOM | 2519 | N | LYS | D | 114 | 31.346 | 52.445 | -8.007 | 1.00 | 41.30 | GZ00 N |
| ATOM | 2520 | CA | LYS | D | 114 | 30.050 | 51.806 | -8.112 | 1.00 | 39.99 | GZ00 C |
| ATOM | 2521 | C | LYS | D | 114 | 30.168 | 50.332 | -7.726 | 1.00 | 42.17 | GZ00 C |
| ATOM | 2522 | O | LYS | D | 114 | 31.189 | 49.691 | -7.965 | 1.00 | 37.31 | GZ00 O |
| ATOM | 2523 | CB | LYS | D | 114 | 29.510 | 51.967 | -9.527 | 1.00 | 38.27 | GZ00 C |
| ATOM | 2524 | CG | LYS | D | 114 | 29.198 | 53.426 | -9.890 | 1.00 | 48.75 | GZ00 C |
| ATOM | 2525 | CD | LYS | D | 114 | 28.597 | 53.502 | -11.284 | 1.00 | 54.69 | GZ00 C |
| ATOM | 2526 | CE | LYS | D | 114 | 27.812 | 54.779 | -11.536 | 1.00 | 43.93 | GZ00 C |
| ATOM | 2527 | NZ | LYS | D | 114 | 26.918 | 54.529 | -12.718 | 1.00 | 45.98 | GZ00 N1+ |
| ATOM | 2528 | N | ALA | D | 115 | 29.108 | 49.806 | -7.107 | 1.00 | 37.03 | GZ00 N |
| ATOM | 2529 | CA | ALA | D | 115 | 29.084 | 48.455 | -6.562 | 1.00 | 31.87 | GZ00 C |
| ATOM | 2530 | C | ALA | D | 115 | 27.754 | 47.802 | -6.906 | 1.00 | 41.28 | GZ00 C |
| ATOM | 2531 | O | ALA | D | 115 | 26.691 | 48.304 | -6.514 | 1.00 | 37.09 | GZ00 O |
| ATOM | 2532 | CB | ALA | D | 115 | 29.280 | 48.470 | -5.040 | 1.00 | 29.90 | GZ00 C |
| ATOM | 2533 | N | ALA | D | 116 | 27.816 | 46.676 | -7.610 | 1.00 | 34.16 | GZ00 N |
| ATOM | 2534 | CA | ALA | D | 116 | 26.622 | 45.929 | -7.933 | 1.00 | 37.15 | GZ00 C |
| ATOM | 2535 | C | ALA | D | 116 | 26.071 | 45.289 | -6.658 | 1.00 | 37.53 | GZ00 C |
| ATOM | 2536 | O | ALA | D | 116 | 26.816 | 45.037 | -5.715 | 1.00 | 41.85 | GZ00 O |
| ATOM | 2537 | CB | ALA | D | 116 | 26.937 | 44.870 | -8.989 | 1.00 | 28.78 | GZ00 C |
| ATOM | 2538 | N | PRO | D | 117 | 24.772 | 45.028 | -6.594 | 1.00 | 40.96 | GZ00 N |
| ATOM | 2539 | CA | PRO | D | 117 | 24.207 | 44.481 | -5.355 | 1.00 | 39.65 | GZ00 C |
| ATOM | 2540 | C | PRO | D | 117 | 24.590 | 43.022 | -5.180 | 1.00 | 40.39 | GZ00 C |
| ATOM | 2541 | O | PRO | D | 117 | 24.686 | 42.263 | -6.149 | 1.00 | 43.42 | GZ00 O |
| ATOM | 2542 | CB | PRO | D | 117 | 22.699 | 44.620 | -5.568 | 1.00 | 37.24 | GZ00 C |
| ATOM | 2543 | CG | PRO | D | 117 | 22.553 | 44.435 | -7.049 | 1.00 | 32.78 | GZ00 C |
| ATOM | 2544 | CD | PRO | D | 117 | 23.761 | 45.109 | -7.664 | 1.00 | 38.78 | GZ00 C |
| ATOM | 2545 | N | SER | D | 118 | 24.817 | 42.626 | -3.934 | 1.00 | 40.21 | GZ00 N |
| ATOM | 2546 | CA | SER | D | 118 | 24.859 | 41.203 | -3.626 | 1.00 | 43.65 | GZ00 C |
| ATOM | 2547 | C | SER | D | 118 | 23.460 | 40.793 | -3.190 | 1.00 | 44.22 | GZ00 C |
| ATOM | 2548 | O | SER | D | 118 | 22.822 | 41.475 | -2.371 | 1.00 | 39.75 | GZ00 O |
| ATOM | 2549 | CB | SER | D | 118 | 25.901 | 40.867 | -2.559 | 1.00 | 37.70 | GZ00 C |
| ATOM | 2550 | OG | SER | D | 118 | 25.559 | 41.468 | -1.335 | 1.00 | 51.03 | GZ00 O |
| ATOM | 2551 | N | VAL | D | 119 | 22.982 | 39.701 | -3.774 | 1.00 | 39.65 | GZ00 N |
| ATOM | 2552 | CA | VAL | D | 119 | 21.609 | 39.244 | -3.641 | 1.00 | 39.06 | GZ00 C |
| ATOM | 2553 | C | VAL | D | 119 | 21.647 | 37.843 | -3.065 | 1.00 | 44.09 | GZ00 C |

| | | | | | | | | | | | | |
|---|---|---|---|---|---|---|---|---|---|---|---|---|
| ATOM | 2554 | O | VAL | D | 119 | 22.315 | 36.966 | -3.626 | 1.00 | 39.00 | GZ00 | O |
| ATOM | 2555 | CB | VAL | D | 119 | 20.894 | 39.235 | -5.007 | 1.00 | 40.58 | GZ00 | C |
| ATOM | 2556 | CG1 | VAL | D | 119 | 19.469 | 38.765 | -4.865 | 1.00 | 33.51 | GZ00 | C |
| ATOM | 2557 | CG2 | VAL | D | 119 | 20.964 | 40.602 | -5.658 | 1.00 | 38.29 | GZ00 | C |
| ATOM | 2558 | N | THR | D | 120 | 20.928 | 37.620 | -1.963 | 1.00 | 38.97 | GZ00 | N |
| ATOM | 2559 | CA | THR | D | 120 | 20.719 | 36.256 | -1.507 | 1.00 | 40.40 | GZ00 | C |
| ATOM | 2560 | C | THR | D | 120 | 19.218 | 36.005 | -1.360 | 1.00 | 41.57 | GZ00 | C |
| ATOM | 2561 | O | THR | D | 120 | 18.487 | 36.826 | -0.798 | 1.00 | 39.96 | GZ00 | O |
| ATOM | 2562 | CB | THR | D | 120 | 21.524 | 35.939 | -0.211 | 1.00 | 48.39 | GZ00 | C |
| ATOM | 2563 | OG1 | THR | D | 120 | 20.649 | 35.655 | 0.882 | 1.00 | 55.57 | GZ00 | O |
| ATOM | 2564 | CG2 | THR | D | 120 | 22.506 | 37.053 | 0.178 | 1.00 | 42.25 | GZ00 | C |
| ATOM | 2565 | N | LEU | D | 121 | 18.761 | 34.879 | -1.903 | 1.00 | 42.65 | GZ00 | N |
| ATOM | 2566 | CA | LEU | D | 121 | 17.343 | 34.562 | -2.031 | 1.00 | 44.51 | GZ00 | C |
| ATOM | 2567 | C | LEU | D | 121 | 17.063 | 33.332 | -1.184 | 1.00 | 42.61 | GZ00 | C |
| ATOM | 2568 | O | LEU | D | 121 | 17.626 | 32.269 | -1.444 | 1.00 | 39.39 | GZ00 | O |
| ATOM | 2569 | CB | LEU | D | 121 | 16.993 | 34.281 | -3.495 | 1.00 | 42.30 | GZ00 | C |
| ATOM | 2570 | CG | LEU | D | 121 | 15.574 | 34.358 | -4.059 | 1.00 | 45.15 | GZ00 | C |
| ATOM | 2571 | CD1 | LEU | D | 121 | 15.333 | 33.234 | -5.034 | 1.00 | 45.85 | GZ00 | C |
| ATOM | 2572 | CD2 | LEU | D | 121 | 14.485 | 34.413 | -2.986 | 1.00 | 44.86 | GZ00 | C |
| ATOM | 2573 | N | PHE | D | 122 | 16.200 | 33.467 | -0.209 | 1.00 | 37.70 | GZ00 | N |
| ATOM | 2574 | CA | PHE | D | 122 | 15.865 | 32.293 | 0.587 | 1.00 | 43.33 | GZ00 | C |
| ATOM | 2575 | C | PHE | D | 122 | 14.487 | 31.766 | 0.221 | 1.00 | 49.36 | GZ00 | C |
| ATOM | 2576 | O | PHE | D | 122 | 13.539 | 32.551 | 0.069 | 1.00 | 41.30 | GZ00 | O |
| ATOM | 2577 | CB | PHE | D | 122 | 15.850 | 32.606 | 2.079 | 1.00 | 39.48 | GZ00 | C |
| ATOM | 2578 | CG | PHE | D | 122 | 17.181 | 32.910 | 2.660 | 1.00 | 38.94 | GZ00 | C |
| ATOM | 2579 | CD1 | PHE | D | 122 | 18.057 | 31.887 | 2.982 | 1.00 | 38.46 | GZ00 | C |
| ATOM | 2580 | CD2 | PHE | D | 122 | 17.533 | 34.229 | 2.949 | 1.00 | 36.53 | GZ00 | C |
| ATOM | 2581 | CE1 | PHE | D | 122 | 19.288 | 32.169 | 3.550 | 1.00 | 45.61 | GZ00 | C |
| ATOM | 2582 | CE2 | PHE | D | 122 | 18.740 | 34.526 | 3.528 | 1.00 | 38.72 | GZ00 | C |
| ATOM | 2583 | CZ | PHE | D | 122 | 19.636 | 33.494 | 3.830 | 1.00 | 40.64 | GZ00 | C |
| ATOM | 2584 | N | PRO | D | 123 | 14.347 | 30.448 | 0.130 | 1.00 | 50.41 | GZ00 | N |
| ATOM | 2585 | CA | PRO | D | 123 | 13.024 | 29.850 | -0.048 | 1.00 | 46.66 | GZ00 | C |
| ATOM | 2586 | C | PRO | D | 123 | 12.262 | 29.853 | 1.266 | 1.00 | 47.32 | GZ00 | C |
| ATOM | 2587 | O | PRO | D | 123 | 12.829 | 30.182 | 2.317 | 1.00 | 41.78 | GZ00 | O |
| ATOM | 2588 | CB | PRO | D | 123 | 13.356 | 28.423 | -0.505 | 1.00 | 53.34 | GZ00 | C |
| ATOM | 2589 | CG | PRO | D | 123 | 14.625 | 28.122 | 0.208 | 1.00 | 54.44 | GZ00 | C |
| ATOM | 2590 | CD | PRO | D | 123 | 15.402 | 29.428 | 0.251 | 1.00 | 49.34 | GZ00 | C |
| ATOM | 2591 | N | PRO | D | 124 | 10.968 | 29.530 | 1.259 | 1.00 | 54.23 | GZ00 | N |
| ATOM | 2592 | CA | PRO | D | 124 | 10.243 | 29.456 | 2.533 | 1.00 | 49.80 | GZ00 | C |
| ATOM | 2593 | C | PRO | D | 124 | 10.766 | 28.290 | 3.353 | 1.00 | 47.27 | GZ00 | C |
| ATOM | 2594 | O | PRO | D | 124 | 11.127 | 27.246 | 2.809 | 1.00 | 40.84 | GZ00 | O |
| ATOM | 2595 | CB | PRO | D | 124 | 8.786 | 29.239 | 2.109 | 1.00 | 52.85 | GZ00 | C |
| ATOM | 2596 | CG | PRO | D | 124 | 8.882 | 28.604 | 0.771 | 1.00 | 58.62 | GZ00 | C |
| ATOM | 2597 | CD | PRO | D | 124 | 10.120 | 29.144 | 0.117 | 1.00 | 50.64 | GZ00 | C |
| ATOM | 2598 | N | SER | D | 125 | 10.848 | 28.487 | 4.665 | 1.00 | 47.15 | GZ00 | N |
| ATOM | 2599 | CA | SER | D | 125 | 11.315 | 27.415 | 5.533 | 1.00 | 46.19 | GZ00 | C |
| ATOM | 2600 | C | SER | D | 125 | 10.226 | 26.360 | 5.725 | 1.00 | 55.91 | GZ00 | C |
| ATOM | 2601 | O | SER | D | 125 | 9.024 | 26.627 | 5.602 | 1.00 | 48.89 | GZ00 | O |
| ATOM | 2602 | CB | SER | D | 125 | 11.725 | 27.963 | 6.895 | 1.00 | 46.67 | GZ00 | C |
| ATOM | 2603 | OG | SER | D | 125 | 10.587 | 28.428 | 7.610 | 1.00 | 43.78 | GZ00 | O |
| ATOM | 2604 | N | SER | D | 126 | 10.661 | 25.150 | 6.076 | 1.00 | 53.45 | GZ00 | N |
| ATOM | 2605 | CA | SER | D | 126 | 9.697 | 24.081 | 6.294 | 1.00 | 49.33 | GZ00 | C |
| ATOM | 2606 | C | SER | D | 126 | 8.769 | 24.420 | 7.455 | 1.00 | 51.40 | GZ00 | C |
| ATOM | 2607 | O | SER | D | 126 | 7.564 | 24.166 | 7.392 | 1.00 | 57.31 | GZ00 | O |
| ATOM | 2608 | CB | SER | D | 126 | 10.432 | 22.769 | 6.528 | 1.00 | 46.88 | GZ00 | C |
| ATOM | 2609 | OG | SER | D | 126 | 11.378 | 22.938 | 7.552 | 1.00 | 58.67 | GZ00 | O |
| ATOM | 2610 | N | GLU | D | 127 | 9.303 | 25.045 | 8.500 | 1.00 | 46.78 | GZ00 | N |
| ATOM | 2611 | CA | GLU | D | 127 | 8.464 | 25.457 | 9.621 | 1.00 | 50.81 | GZ00 | C |
| ATOM | 2612 | C | GLU | D | 127 | 7.347 | 26.393 | 9.173 | 1.00 | 60.09 | GZ00 | C |
| ATOM | 2613 | O | GLU | D | 127 | 6.256 | 26.382 | 9.759 | 1.00 | 61.69 | GZ00 | O |
| ATOM | 2614 | CB | GLU | D | 127 | 9.329 | 26.118 | 10.686 | 1.00 | 53.57 | GZ00 | C |
| ATOM | 2615 | CG | GLU | D | 127 | 10.626 | 25.340 | 10.894 | 1.00 | 62.62 | GZ00 | C |
| ATOM | 2616 | CD | GLU | D | 127 | 11.443 | 25.816 | 12.072 | 1.00 | 72.05 | GZ00 | C |
| ATOM | 2617 | OE1 | GLU | D | 127 | 10.899 | 26.562 | 12.933 | 1.00 | 69.83 | GZ00 | O |
| ATOM | 2618 | OE2 | GLU | D | 127 | 12.635 | 25.426 | 12.129 | 1.00 | 72.47 | GZ00 | O1- |
| ATOM | 2619 | N | GLU | D | 128 | 7.608 | 27.240 | 8.170 | 1.00 | 56.85 | GZ00 | N |
| ATOM | 2620 | CA | GLU | D | 128 | 6.568 | 28.148 | 7.700 | 1.00 | 54.23 | GZ00 | C |
| ATOM | 2621 | C | GLU | D | 128 | 5.590 | 27.441 | 6.774 | 1.00 | 52.82 | GZ00 | C |
| ATOM | 2622 | O | GLU | D | 128 | 4.390 | 27.728 | 6.804 | 1.00 | 53.66 | GZ00 | O |
| ATOM | 2623 | CB | GLU | D | 128 | 7.174 | 29.364 | 6.983 | 1.00 | 52.45 | GZ00 | C |
| ATOM | 2624 | CG | GLU | D | 128 | 6.132 | 30.482 | 6.745 | 1.00 | 57.44 | GZ00 | C |

| | | | | | | | | | | | | |
|---|---|---|---|---|---|---|---|---|---|---|---|---|
| ATOM | 2625 | CD | GLU | D | 128 | 6.545 | 31.555 | 5.722 | 1.00 | 58.50 | GZ00 | C |
| ATOM | 2626 | OE1 | GLU | D | 128 | 5.856 | 32.590 | 5.684 | 1.00 | 54.07 | GZ00 | O |
| ATOM | 2627 | OE2 | GLU | D | 128 | 7.518 | 31.373 | 4.950 | 1.00 | 49.75 | GZ00 | O1- |
| ATOM | 2628 | N | LEU | D | 129 | 6.095 | 26.539 | 5.927 | 1.00 | 52.50 | GZ00 | N |
| ATOM | 2629 | CA | LEU | D | 129 | 5.226 | 25.707 | 5.104 | 1.00 | 51.40 | GZ00 | C |
| ATOM | 2630 | C | LEU | D | 129 | 4.279 | 24.879 | 5.969 | 1.00 | 59.74 | GZ00 | C |
| ATOM | 2631 | O | LEU | D | 129 | 3.106 | 24.702 | 5.623 | 1.00 | 63.72 | GZ00 | O |
| ATOM | 2632 | CB | LEU | D | 129 | 6.078 | 24.807 | 4.214 | 1.00 | 51.65 | GZ00 | C |
| ATOM | 2633 | CG | LEU | D | 129 | 6.868 | 25.538 | 3.131 | 1.00 | 52.87 | GZ00 | C |
| ATOM | 2634 | CD1 | LEU | D | 129 | 7.857 | 24.604 | 2.459 | 1.00 | 44.54 | GZ00 | C |
| ATOM | 2635 | CD2 | LEU | D | 129 | 5.917 | 26.140 | 2.101 | 1.00 | 49.64 | GZ00 | C |
| ATOM | 2636 | N | GLN | D | 130 | 4.767 | 24.388 | 7.115 | 1.00 | 54.99 | GZ00 | N |
| ATOM | 2637 | CA | GLN | D | 130 | 3.913 | 23.640 | 8.032 | 1.00 | 66.06 | GZ00 | C |
| ATOM | 2638 | C | GLN | D | 130 | 2.760 | 24.489 | 8.552 | 1.00 | 64.20 | GZ00 | C |
| ATOM | 2639 | O | GLN | D | 130 | 1.673 | 23.958 | 8.810 | 1.00 | 68.64 | GZ00 | O |
| ATOM | 2640 | CB | GLN | D | 130 | 4.729 | 23.097 | 9.210 | 1.00 | 64.34 | GZ00 | C |
| ATOM | 2641 | CG | GLN | D | 130 | 5.729 | 22.005 | 8.863 | 1.00 | 65.01 | GZ00 | C |
| ATOM | 2642 | CD | GLN | D | 130 | 6.363 | 21.409 | 10.113 | 1.00 | 78.91 | GZ00 | C |
| ATOM | 2643 | OE1 | GLN | D | 130 | 5.663 | 21.103 | 11.083 | 1.00 | 88.40 | GZ00 | O |
| ATOM | 2644 | NE2 | GLN | D | 130 | 7.691 | 21.262 | 10.108 | 1.00 | 62.99 | GZ00 | N |
| ATOM | 2645 | N | ALA | D | 131 | 2.972 | 25.794 | 8.729 | 1.00 | 53.92 | GZ00 | N |
| ATOM | 2646 | CA | ALA | D | 131 | 1.903 | 26.700 | 9.129 | 1.00 | 51.41 | GZ00 | C |
| ATOM | 2647 | C | ALA | D | 131 | 1.100 | 27.196 | 7.943 | 1.00 | 56.66 | GZ00 | C |
| ATOM | 2648 | O | ALA | D | 131 | 0.366 | 28.184 | 8.075 | 1.00 | 63.18 | GZ00 | O |
| ATOM | 2649 | CB | ALA | D | 131 | 2.459 | 27.889 | 9.915 | 1.00 | 45.31 | GZ00 | C |
| ATOM | 2650 | N | ASN | D | 132 | 1.246 | 26.543 | 6.784 | 1.00 | 55.73 | GZ00 | N |
| ATOM | 2651 | CA | ASN | D | 132 | 0.472 | 26.840 | 5.573 | 1.00 | 65.28 | GZ00 | C |
| ATOM | 2652 | C | ASN | D | 132 | 0.645 | 28.283 | 5.100 | 1.00 | 66.61 | GZ00 | C |
| ATOM | 2653 | O | ASN | D | 132 | -0.264 | 28.869 | 4.507 | 1.00 | 68.44 | GZ00 | O |
| ATOM | 2654 | CB | ASN | D | 132 | -1.018 | 26.502 | 5.762 | 1.00 | 67.97 | GZ00 | C |
| ATOM | 2655 | CG | ASN | D | 132 | -1.347 | 25.065 | 5.335 | 1.00 | 88.31 | GZ00 | C |
| ATOM | 2656 | OD1 | ASN | D | 132 | -1.487 | 24.776 | 4.134 | 1.00 | 78.43 | GZ00 | O |
| ATOM | 2657 | ND2 | ASN | D | 132 | -1.448 | 24.155 | 6.318 | 1.00 | 80.50 | GZ00 | N |
| ATOM | 2658 | N | LYS | D | 133 | 1.812 | 28.866 | 5.352 | 1.00 | 68.70 | GZ00 | N |
| ATOM | 2659 | CA | LYS | D | 133 | 2.244 | 30.090 | 4.696 | 1.00 | 59.67 | GZ00 | C |
| ATOM | 2660 | C | LYS | D | 133 | 3.522 | 29.799 | 3.923 | 1.00 | 57.71 | GZ00 | C |
| ATOM | 2661 | O | LYS | D | 133 | 4.135 | 28.737 | 4.069 | 1.00 | 58.17 | GZ00 | O |
| ATOM | 2662 | CB | LYS | D | 133 | 2.468 | 31.225 | 5.701 | 1.00 | 52.27 | GZ00 | C |
| ATOM | 2663 | CG | LYS | D | 133 | 1.236 | 31.650 | 6.454 | 1.00 | 58.15 | GZ00 | C |
| ATOM | 2664 | CD | LYS | D | 133 | 1.590 | 32.644 | 7.547 | 1.00 | 82.46 | GZ00 | C |
| ATOM | 2665 | CE | LYS | D | 133 | 0.389 | 32.977 | 8.428 | 1.00 | 90.01 | GZ00 | C |
| ATOM | 2666 | NZ | LYS | D | 133 | -0.049 | 31.789 | 9.221 | 1.00 | 85.20 | GZ00 | N1+ |
| ATOM | 2667 | N | ALA | D | 134 | 3.923 | 30.754 | 3.087 | 1.00 | 59.30 | GZ00 | N |
| ATOM | 2668 | CA | ALA | D | 134 | 5.167 | 30.615 | 2.328 | 1.00 | 57.19 | GZ00 | C |
| ATOM | 2669 | C | ALA | D | 134 | 5.607 | 31.994 | 1.872 | 1.00 | 56.76 | GZ00 | C |
| ATOM | 2670 | O | ALA | D | 134 | 4.842 | 32.692 | 1.196 | 1.00 | 55.80 | GZ00 | O |
| ATOM | 2671 | CB | ALA | D | 134 | 4.985 | 29.678 | 1.129 | 1.00 | 53.33 | GZ00 | C |
| ATOM | 2672 | N | THR | D | 135 | 6.826 | 32.399 | 2.238 | 1.00 | 51.12 | GZ00 | N |
| ATOM | 2673 | CA | THR | D | 135 | 7.368 | 33.652 | 1.730 | 1.00 | 46.27 | GZ00 | C |
| ATOM | 2674 | C | THR | D | 135 | 8.764 | 33.429 | 1.168 | 1.00 | 50.06 | GZ00 | C |
| ATOM | 2675 | O | THR | D | 135 | 9.604 | 32.783 | 1.803 | 1.00 | 41.99 | GZ00 | O |
| ATOM | 2676 | CB | THR | D | 135 | 7.422 | 34.735 | 2.803 | 1.00 | 43.17 | GZ00 | C |
| ATOM | 2677 | OG1 | THR | D | 135 | 8.619 | 34.572 | 3.555 | 1.00 | 70.40 | GZ00 | O |
| ATOM | 2678 | CG2 | THR | D | 135 | 6.270 | 34.632 | 3.735 | 1.00 | 40.85 | GZ00 | C |
| ATOM | 2679 | N | LEU | D | 136 | 9.002 | 33.964 | -0.025 | 1.00 | 49.86 | GZ00 | N |
| ATOM | 2680 | CA | LEU | D | 136 | 10.344 | 34.047 | -0.574 | 1.00 | 43.03 | GZ00 | C |
| ATOM | 2681 | C | LEU | D | 136 | 10.956 | 35.371 | -0.127 | 1.00 | 44.71 | GZ00 | C |
| ATOM | 2682 | O | LEU | D | 136 | 10.274 | 36.400 | -0.088 | 1.00 | 45.54 | GZ00 | O |
| ATOM | 2683 | CB | LEU | D | 136 | 10.328 | 33.951 | -2.101 | 1.00 | 48.82 | GZ00 | C |
| ATOM | 2684 | CG | LEU | D | 136 | 9.744 | 32.685 | -2.734 | 1.00 | 50.57 | GZ00 | C |
| ATOM | 2685 | CD1 | LEU | D | 136 | 9.722 | 32.777 | -4.241 | 1.00 | 43.90 | GZ00 | C |
| ATOM | 2686 | CD2 | LEU | D | 136 | 10.592 | 31.534 | -2.313 | 1.00 | 54.51 | GZ00 | C |
| ATOM | 2687 | N | VAL | D | 137 | 12.231 | 35.329 | 0.251 | 1.00 | 38.61 | GZ00 | N |
| ATOM | 2688 | CA | VAL | D | 137 | 12.914 | 36.459 | 0.867 | 1.00 | 39.76 | GZ00 | C |
| ATOM | 2689 | C | VAL | D | 137 | 14.144 | 36.781 | 0.037 | 1.00 | 43.30 | GZ00 | C |
| ATOM | 2690 | O | VAL | D | 137 | 15.046 | 35.946 | -0.086 | 1.00 | 43.19 | GZ00 | O |
| ATOM | 2691 | CB | VAL | D | 137 | 13.286 | 36.167 | 2.326 | 1.00 | 40.83 | GZ00 | C |
| ATOM | 2692 | CG1 | VAL | D | 137 | 13.930 | 37.386 | 2.954 | 1.00 | 40.34 | GZ00 | C |
| ATOM | 2693 | CG2 | VAL | D | 137 | 12.032 | 35.756 | 3.116 | 1.00 | 39.27 | GZ00 | C |
| ATOM | 2694 | N | CYS | D | 138 | 14.161 | 37.969 | -0.563 | 1.00 | 39.42 | GZ00 | N |
| ATOM | 2695 | CA | CYS | D | 138 | 15.270 | 38.436 | -1.392 | 1.00 | 38.06 | GZ00 | C |

| | | | | | | | | | | | |
|---|---|---|---|---|---|---|---|---|---|---|---|
| ATOM | 2696 | C | CYS | D | 138 | 15.958 | 39.590 | -0.669 | 1.00 | 42.98 | GZ00 C |
| ATOM | 2697 | O | CYS | D | 138 | 15.379 | 40.679 | -0.549 | 1.00 | 42.34 | GZ00 O |
| ATOM | 2698 | CB | CYS | D | 138 | 14.764 | 38.869 | -2.767 | 1.00 | 46.28 | GZ00 C |
| ATOM | 2699 | SG | CYS | D | 138 | 16.005 | 39.055 | -4.058 | 1.00 | 46.59 | GZ00 S |
| ATOM | 2700 | N | LEU | D | 139 | 17.165 | 39.345 | -0.149 | 1.00 | 35.86 | GZ00 N |
| ATOM | 2701 | CA | LEU | D | 139 | 17.953 | 40.386 | 0.503 | 1.00 | 34.94 | GZ00 C |
| ATOM | 2702 | C | LEU | D | 139 | 18.994 | 40.946 | -0.456 | 1.00 | 40.09 | GZ00 C |
| ATOM | 2703 | O | LEU | D | 139 | 19.692 | 40.198 | -1.153 | 1.00 | 38.00 | GZ00 O |
| ATOM | 2704 | CB | LEU | D | 139 | 18.641 | 39.914 | 1.784 | 1.00 | 38.60 | GZ00 C |
| ATOM | 2705 | CG | LEU | D | 139 | 17.851 | 39.429 | 2.994 | 1.00 | 42.05 | GZ00 C |
| ATOM | 2706 | CD1 | LEU | D | 139 | 16.412 | 39.996 | 2.992 | 1.00 | 34.31 | GZ00 C |
| ATOM | 2707 | CD2 | LEU | D | 139 | 17.888 | 37.954 | 3.110 | 1.00 | 41.56 | GZ00 C |
| ATOM | 2708 | N | ILE | D | 140 | 19.072 | 42.271 | -0.498 | 1.00 | 36.85 | GZ00 N |
| ATOM | 2709 | CA | ILE | D | 140 | 19.852 | 42.991 | -1.490 | 1.00 | 40.84 | GZ00 C |
| ATOM | 2710 | C | ILE | D | 140 | 20.717 | 43.982 | -0.735 | 1.00 | 39.30 | GZ00 C |
| ATOM | 2711 | O | ILE | D | 140 | 20.196 | 44.821 | 0.012 | 1.00 | 36.82 | GZ00 O |
| ATOM | 2712 | CB | ILE | D | 140 | 18.946 | 43.703 | -2.510 | 1.00 | 36.07 | GZ00 C |
| ATOM | 2713 | CG1 | ILE | D | 140 | 17.852 | 42.738 | -2.998 | 1.00 | 41.84 | GZ00 C |
| ATOM | 2714 | CG2 | ILE | D | 140 | 19.749 | 44.191 | -3.673 | 1.00 | 33.32 | GZ00 C |
| ATOM | 2715 | CD1 | ILE | D | 140 | 16.606 | 43.414 | -3.540 | 1.00 | 40.09 | GZ00 C |
| ATOM | 2716 | N | SER | D | 141 | 22.028 | 43.899 | -0.928 | 1.00 | 33.24 | GZ00 N |
| ATOM | 2717 | CA | SER | D | 141 | 22.918 | 44.678 | -0.083 | 1.00 | 40.45 | GZ00 C |
| ATOM | 2718 | C | SER | D | 141 | 24.146 | 45.120 | -0.865 | 1.00 | 40.51 | GZ00 C |
| ATOM | 2719 | O | SER | D | 141 | 24.453 | 44.592 | -1.948 | 1.00 | 33.29 | GZ00 O |
| ATOM | 2720 | CB | SER | D | 141 | 23.348 | 43.878 | 1.155 | 1.00 | 39.86 | GZ00 C |
| ATOM | 2721 | OG | SER | D | 141 | 23.964 | 42.664 | 0.748 | 1.00 | 40.92 | GZ00 O |
| ATOM | 2722 | N | ASP | D | 142 | 24.835 | 46.117 | -0.288 | 1.00 | 34.55 | GZ00 N |
| ATOM | 2723 | CA | ASP | D | 142 | 26.137 | 46.573 | -0.776 | 1.00 | 45.39 | GZ00 C |
| ATOM | 2724 | C | ASP | D | 142 | 26.059 | 47.148 | -2.192 | 1.00 | 42.72 | GZ00 C |
| ATOM | 2725 | O | ASP | D | 142 | 26.985 | 46.983 | -2.988 | 1.00 | 37.05 | GZ00 O |
| ATOM | 2726 | CB | ASP | D | 142 | 27.165 | 45.436 | -0.742 | 1.00 | 42.27 | GZ00 C |
| ATOM | 2727 | CG | ASP | D | 142 | 27.636 | 45.114 | 0.650 | 1.00 | 45.03 | GZ00 C |
| ATOM | 2728 | OD1 | ASP | D | 142 | 27.734 | 46.043 | 1.489 | 1.00 | 49.79 | GZ00 O |
| ATOM | 2729 | OD2 | ASP | D | 142 | 27.890 | 43.921 | 0.902 | 1.00 | 52.25 | GZ00 O1- |
| ATOM | 2730 | N | PHE | D | 143 | 24.967 | 47.826 | -2.529 | 1.00 | 33.33 | GZ00 N |
| ATOM | 2731 | CA | PHE | D | 143 | 24.908 | 48.407 | -3.858 | 1.00 | 35.29 | GZ00 C |
| ATOM | 2732 | C | PHE | D | 143 | 25.067 | 49.921 | -3.775 | 1.00 | 33.78 | GZ00 C |
| ATOM | 2733 | O | PHE | D | 143 | 24.717 | 50.556 | -2.773 | 1.00 | 36.42 | GZ00 O |
| ATOM | 2734 | CB | PHE | D | 143 | 23.637 | 48.009 | -4.617 | 1.00 | 34.97 | GZ00 C |
| ATOM | 2735 | CG | PHE | D | 143 | 22.340 | 48.333 | -3.911 | 1.00 | 35.61 | GZ00 C |
| ATOM | 2736 | CD1 | PHE | D | 143 | 21.712 | 49.549 | -4.113 | 1.00 | 32.64 | GZ00 C |
| ATOM | 2737 | CD2 | PHE | D | 143 | 21.709 | 47.379 | -3.112 | 1.00 | 38.53 | GZ00 C |
| ATOM | 2738 | CE1 | PHE | D | 143 | 20.485 | 49.834 | -3.493 | 1.00 | 41.16 | GZ00 C |
| ATOM | 2739 | CE2 | PHE | D | 143 | 20.494 | 47.658 | -2.486 | 1.00 | 40.15 | GZ00 C |
| ATOM | 2740 | CZ | PHE | D | 143 | 19.880 | 48.890 | -2.672 | 1.00 | 32.95 | GZ00 C |
| ATOM | 2741 | N | TYR | D | 144 | 25.665 | 50.471 | -4.824 | 1.00 | 35.76 | GZ00 N |
| ATOM | 2742 | CA | TYR | D | 144 | 26.002 | 51.882 | -4.906 | 1.00 | 37.17 | GZ00 C |
| ATOM | 2743 | C | TYR | D | 144 | 26.142 | 52.286 | -6.363 | 1.00 | 41.07 | GZ00 C |
| ATOM | 2744 | O | TYR | D | 144 | 26.899 | 51.649 | -7.096 | 1.00 | 40.05 | GZ00 O |
| ATOM | 2745 | CB | TYR | D | 144 | 27.305 | 52.176 | -4.182 | 1.00 | 31.07 | GZ00 C |
| ATOM | 2746 | CG | TYR | D | 144 | 27.652 | 53.644 | -4.148 | 1.00 | 33.84 | GZ00 C |
| ATOM | 2747 | CD1 | TYR | D | 144 | 27.125 | 54.478 | -3.168 | 1.00 | 34.22 | GZ00 C |
| ATOM | 2748 | CD2 | TYR | D | 144 | 28.485 | 54.207 | -5.107 | 1.00 | 34.49 | GZ00 C |
| ATOM | 2749 | CE1 | TYR | D | 144 | 27.433 | 55.822 | -3.129 | 1.00 | 36.40 | GZ00 C |
| ATOM | 2750 | CE2 | TYR | D | 144 | 28.800 | 55.559 | -5.076 | 1.00 | 35.36 | GZ00 C |
| ATOM | 2751 | CZ | TYR | D | 144 | 28.271 | 56.358 | -4.082 | 1.00 | 35.95 | GZ00 C |
| ATOM | 2752 | OH | TYR | D | 144 | 28.578 | 57.699 | -4.037 | 1.00 | 40.59 | GZ00 O |
| ATOM | 2753 | N | PRO | D | 145 | 25.442 | 53.357 | -6.782 | 1.00 | 35.13 | GZ00 N |
| ATOM | 2754 | CA | PRO | D | 145 | 24.556 | 54.168 | -5.930 | 1.00 | 41.08 | GZ00 C |
| ATOM | 2755 | C | PRO | D | 145 | 23.258 | 53.476 | -5.465 | 1.00 | 39.60 | GZ00 C |
| ATOM | 2756 | O | PRO | D | 145 | 22.944 | 52.359 | -5.874 | 1.00 | 35.16 | GZ00 O |
| ATOM | 2757 | CB | PRO | D | 145 | 24.231 | 55.395 | -6.811 | 1.00 | 40.78 | GZ00 C |
| ATOM | 2758 | CG | PRO | D | 145 | 24.659 | 55.048 | -8.190 | 1.00 | 39.08 | GZ00 C |
| ATOM | 2759 | CD | PRO | D | 145 | 25.708 | 53.989 | -8.088 | 1.00 | 36.76 | GZ00 C |
| ATOM | 2760 | N | GLY | D | 146 | 22.514 | 54.161 | -4.600 | 1.00 | 34.87 | GZ00 N |
| ATOM | 2761 | CA | GLY | D | 146 | 21.429 | 53.525 | -3.892 | 1.00 | 34.83 | GZ00 C |
| ATOM | 2762 | C | GLY | D | 146 | 20.087 | 53.444 | -4.593 | 1.00 | 36.93 | GZ00 C |
| ATOM | 2763 | O | GLY | D | 146 | 19.088 | 53.931 | -4.062 | 1.00 | 34.26 | GZ00 O |
| ATOM | 2764 | N | ALA | D | 147 | 20.037 | 52.844 | -5.777 | 1.00 | 32.90 | GZ00 N |
| ATOM | 2765 | CA | ALA | D | 147 | 18.750 | 52.609 | -6.410 | 1.00 | 32.84 | GZ00 C |
| ATOM | 2766 | C | ALA | D | 147 | 18.802 | 51.314 | -7.178 | 1.00 | 32.60 | GZ00 C |

| | | | | | | | | | | | |
|---|---|---|---|---|---|---|---|---|---|---|---|
| ATOM | 2767 | O | ALA | D | 147 | 19.792 | 51.008 | -7.841 | 1.00 | 39.02 | GZ00 O |
| ATOM | 2768 | CB | ALA | D | 147 | 18.326 | 53.719 | -7.365 | 1.00 | 29.65 | GZ00 C |
| ATOM | 2769 | N | VAL | D | 148 | 17.684 | 50.603 | -7.141 | 1.00 | 33.58 | GZ00 N |
| ATOM | 2770 | CA | VAL | D | 148 | 17.588 | 49.253 | -7.651 | 1.00 | 34.66 | GZ00 C |
| ATOM | 2771 | C | VAL | D | 148 | 16.121 | 49.045 | -7.985 | 1.00 | 41.83 | GZ00 C |
| ATOM | 2772 | O | VAL | D | 148 | 15.247 | 49.684 | -7.396 | 1.00 | 32.66 | GZ00 O |
| ATOM | 2773 | CB | VAL | D | 148 | 18.126 | 48.264 | -6.582 | 1.00 | 38.73 | GZ00 C |
| ATOM | 2774 | CG1 | VAL | D | 148 | 17.030 | 47.484 | -5.915 | 1.00 | 37.24 | GZ00 C |
| ATOM | 2775 | CG2 | VAL | D | 148 | 19.218 | 47.381 | -7.146 | 1.00 | 41.31 | GZ00 C |
| ATOM | 2776 | N | THR | D | 149 | 15.845 | 48.198 | -8.967 | 1.00 | 38.40 | GZ00 N |
| ATOM | 2777 | CA | THR | D | 149 | 14.472 | 47.753 | -9.182 | 1.00 | 44.38 | GZ00 C |
| ATOM | 2778 | C | THR | D | 149 | 14.427 | 46.229 | -9.138 | 1.00 | 46.22 | GZ00 C |
| ATOM | 2779 | O | THR | D | 149 | 15.385 | 45.556 | -9.546 | 1.00 | 43.23 | GZ00 O |
| ATOM | 2780 | CB | THR | D | 149 | 13.899 | 48.266 | -10.513 | 1.00 | 46.92 | GZ00 C |
| ATOM | 2781 | OG1 | THR | D | 149 | 14.727 | 47.815 | -11.599 | 1.00 | 50.51 | GZ00 O |
| ATOM | 2782 | CG2 | THR | D | 149 | 13.810 | 49.796 | -10.512 | 1.00 | 38.30 | GZ00 C |
| ATOM | 2783 | N | VAL | D | 150 | 13.311 | 45.686 | -8.642 | 1.00 | 36.64 | GZ00 N |
| ATOM | 2784 | CA | VAL | D | 150 | 13.203 | 44.262 | -8.355 | 1.00 | 39.12 | GZ00 C |
| ATOM | 2785 | C | VAL | D | 150 | 12.046 | 43.662 | -9.128 | 1.00 | 38.75 | GZ00 C |
| ATOM | 2786 | O | VAL | D | 150 | 10.920 | 44.160 | -9.054 | 1.00 | 46.61 | GZ00 O |
| ATOM | 2787 | CB | VAL | D | 150 | 13.032 | 43.987 | -6.853 | 1.00 | 39.37 | GZ00 C |
| ATOM | 2788 | CG1 | VAL | D | 150 | 12.929 | 42.488 | -6.631 | 1.00 | 41.26 | GZ00 C |
| ATOM | 2789 | CG2 | VAL | D | 150 | 14.213 | 44.522 | -6.121 | 1.00 | 34.76 | GZ00 C |
| ATOM | 2790 | N | ALA | D | 151 | 12.333 | 42.596 | -9.865 | 1.00 | 39.71 | GZ00 N |
| ATOM | 2791 | CA | ALA | D | 151 | 11.333 | 41.805 | -10.560 | 1.00 | 44.18 | GZ00 C |
| ATOM | 2792 | C | ALA | D | 151 | 11.434 | 40.350 | -10.127 | 1.00 | 50.02 | GZ00 C |
| ATOM | 2793 | O | ALA | D | 151 | 12.534 | 39.784 | -10.032 | 1.00 | 47.26 | GZ00 O |
| ATOM | 2794 | CB | ALA | D | 151 | 11.494 | 41.895 | -12.081 | 1.00 | 37.34 | GZ00 C |
| ATOM | 2795 | N | TRP | D | 152 | 10.276 | 39.748 | -9.889 | 1.00 | 47.18 | GZ00 N |
| ATOM | 2796 | CA | TRP | D | 152 | 10.164 | 38.343 | -9.544 | 1.00 | 47.04 | GZ00 C |
| ATOM | 2797 | C | TRP | D | 152 | 9.629 | 37.556 | -10.731 | 1.00 | 49.55 | GZ00 C |
| ATOM | 2798 | O | TRP | D | 152 | 8.800 | 38.055 | -11.498 | 1.00 | 59.75 | GZ00 O |
| ATOM | 2799 | CB | TRP | D | 152 | 9.248 | 38.155 | -8.345 | 1.00 | 41.47 | GZ00 C |
| ATOM | 2800 | CG | TRP | D | 152 | 9.799 | 38.641 | -7.051 | 1.00 | 45.78 | GZ00 C |
| ATOM | 2801 | CD1 | TRP | D | 152 | 9.786 | 39.918 | -6.579 | 1.00 | 49.07 | GZ00 C |
| ATOM | 2802 | CD2 | TRP | D | 152 | 10.400 | 37.837 | -6.026 | 1.00 | 43.34 | GZ00 C |
| ATOM | 2803 | NE1 | TRP | D | 152 | 10.361 | 39.967 | -5.325 | 1.00 | 46.95 | GZ00 N |
| ATOM | 2804 | CE2 | TRP | D | 152 | 10.736 | 38.700 | -4.961 | 1.00 | 46.03 | GZ00 C |
| ATOM | 2805 | CE3 | TRP | D | 152 | 10.699 | 36.468 | -5.914 | 1.00 | 44.86 | GZ00 C |
| ATOM | 2806 | CZ2 | TRP | D | 152 | 11.364 | 38.245 | -3.792 | 1.00 | 48.01 | GZ00 C |
| ATOM | 2807 | CZ3 | TRP | D | 152 | 11.318 | 36.008 | -4.748 | 1.00 | 46.08 | GZ00 C |
| ATOM | 2808 | CH2 | TRP | D | 152 | 11.649 | 36.905 | -3.702 | 1.00 | 46.48 | GZ00 C |
| ATOM | 2809 | N | LYS | D | 153 | 10.109 | 36.330 | -10.882 | 1.00 | 54.87 | GZ00 N |
| ATOM | 2810 | CA | LYS | D | 153 | 9.649 | 35.434 | -11.929 | 1.00 | 57.27 | GZ00 C |
| ATOM | 2811 | C | LYS | D | 153 | 9.247 | 34.090 | -11.331 | 1.00 | 66.16 | GZ00 C |
| ATOM | 2812 | O | LYS | D | 153 | 9.884 | 33.591 | -10.396 | 1.00 | 64.92 | GZ00 O |
| ATOM | 2813 | CB | LYS | D | 153 | 10.728 | 35.247 | -12.999 | 1.00 | 61.27 | GZ00 C |
| ATOM | 2814 | CG | LYS | D | 153 | 10.832 | 36.442 | -13.910 | 1.00 | 66.71 | GZ00 C |
| ATOM | 2815 | CD | LYS | D | 153 | 11.805 | 36.232 | -15.044 | 1.00 | 71.68 | GZ00 C |
| ATOM | 2816 | CE | LYS | D | 153 | 11.839 | 37.483 | -15.930 | 1.00 | 79.74 | GZ00 C |
| ATOM | 2817 | NZ | LYS | D | 153 | 12.960 | 37.469 | -16.930 | 1.00 | 89.60 | GZ00 N1+ |
| ATOM | 2818 | N | ALA | D | 154 | 8.145 | 33.543 | -11.836 | 1.00 | 68.05 | GZ00 N |
| ATOM | 2819 | CA | ALA | D | 154 | 7.729 | 32.169 | -11.568 | 1.00 | 66.27 | GZ00 C |
| ATOM | 2820 | C | ALA | D | 154 | 7.933 | 31.392 | -12.864 | 1.00 | 70.66 | GZ00 C |
| ATOM | 2821 | O | ALA | D | 154 | 7.229 | 31.641 | -13.848 | 1.00 | 74.13 | GZ00 O |
| ATOM | 2822 | CB | ALA | D | 154 | 6.280 | 32.108 | -11.092 | 1.00 | 57.49 | GZ00 C |
| ATOM | 2823 | N | ASP | D | 155 | 8.892 | 30.464 | -12.866 | 1.00 | 69.01 | GZ00 N |
| ATOM | 2824 | CA | ASP | D | 155 | 9.320 | 29.751 | -14.079 | 1.00 | 76.96 | GZ00 C |
| ATOM | 2825 | C | ASP | D | 155 | 9.440 | 30.697 | -15.267 | 1.00 | 78.16 | GZ00 C |
| ATOM | 2826 | O | ASP | D | 155 | 8.849 | 30.482 | -16.327 | 1.00 | 86.27 | GZ00 O |
| ATOM | 2827 | CB | ASP | D | 155 | 8.342 | 28.625 | -14.445 | 1.00 | 79.86 | GZ00 C |
| ATOM | 2828 | CG | ASP | D | 155 | 8.311 | 27.503 | -13.442 | 1.00 | 77.78 | GZ00 C |
| ATOM | 2829 | OD1 | ASP | D | 155 | 9.379 | 27.134 | -12.919 | 1.00 | 80.60 | GZ00 O1- |
| ATOM | 2830 | OD2 | ASP | D | 155 | 7.205 | 26.974 | -13.197 | 1.00 | 86.69 | GZ00 O |
| ATOM | 2831 | N | SER | D | 156 | 10.188 | 31.777 | -15.083 | 1.00 | 72.25 | GZ00 N |
| ATOM | 2832 | CA | SER | D | 156 | 10.451 | 32.741 | -16.147 | 1.00 | 79.24 | GZ00 C |
| ATOM | 2833 | C | SER | D | 156 | 9.207 | 33.506 | -16.599 | 1.00 | 70.74 | GZ00 C |
| ATOM | 2834 | O | SER | D | 156 | 9.242 | 34.141 | -17.656 | 1.00 | 70.59 | GZ00 O |
| ATOM | 2835 | CB | SER | D | 156 | 11.103 | 32.085 | -17.366 | 1.00 | 79.59 | GZ00 C |
| ATOM | 2836 | OG | SER | D | 156 | 10.117 | 31.458 | -18.157 | 1.00 | 81.93 | GZ00 O |
| ATOM | 2837 | N | SER | D | 157 | 8.086 | 33.449 | -15.840 | 1.00 | 66.95 | GZ00 N |

| | | | | | | | | | | | | |
|---|---|---|---|---|---|---|---|---|---|---|---|---|
| ATOM | 2838 | CA | SER | D | 157 | 6.934 | 34.326 | -16.090 | 1.00 | 65.14 | GZ00 | C |
| ATOM | 2839 | C | SER | D | 157 | 6.878 | 35.425 | -15.047 | 1.00 | 67.53 | GZ00 | C |
| ATOM | 2840 | O | SER | D | 157 | 7.031 | 35.145 | -13.848 | 1.00 | 64.92 | GZ00 | O |
| ATOM | 2841 | CB | SER | D | 157 | 5.624 | 33.544 | -16.060 | 1.00 | 63.23 | GZ00 | C |
| ATOM | 2842 | OG | SER | D | 157 | 5.562 | 32.633 | -17.136 | 1.00 | 75.81 | GZ00 | O |
| ATOM | 2843 | N | PRO | D | 158 | 6.652 | 36.670 | -15.446 | 1.00 | 66.82 | GZ00 | N |
| ATOM | 2844 | CA | PRO | D | 158 | 6.585 | 37.756 | -14.462 | 1.00 | 56.39 | GZ00 | C |
| ATOM | 2845 | C | PRO | D | 158 | 5.538 | 37.504 | -13.392 | 1.00 | 59.58 | GZ00 | C |
| ATOM | 2846 | O | PRO | D | 158 | 4.450 | 36.989 | -13.660 | 1.00 | 66.88 | GZ00 | O |
| ATOM | 2847 | CB | PRO | D | 158 | 6.236 | 38.986 | -15.308 | 1.00 | 52.59 | GZ00 | C |
| ATOM | 2848 | CG | PRO | D | 158 | 5.845 | 38.451 | -16.652 | 1.00 | 66.17 | GZ00 | C |
| ATOM | 2849 | CD | PRO | D | 158 | 6.585 | 37.169 | -16.824 | 1.00 | 63.85 | GZ00 | C |
| ATOM | 2850 | N | VAL | D | 159 | 5.900 | 37.834 | -12.160 | 1.00 | 59.53 | GZ00 | N |
| ATOM | 2851 | CA | VAL | D | 159 | 5.012 | 37.741 | -11.012 | 1.00 | 57.33 | GZ00 | C |
| ATOM | 2852 | C | VAL | D | 159 | 4.480 | 39.133 | -10.721 | 1.00 | 64.87 | GZ00 | C |
| ATOM | 2853 | O | VAL | D | 159 | 5.261 | 40.073 | -10.535 | 1.00 | 75.66 | GZ00 | O |
| ATOM | 2854 | CB | VAL | D | 159 | 5.735 | 37.169 | -9.785 | 1.00 | 58.46 | GZ00 | C |
| ATOM | 2855 | CG1 | VAL | D | 159 | 4.831 | 37.239 | -8.567 | 1.00 | 61.48 | GZ00 | C |
| ATOM | 2856 | CG2 | VAL | D | 159 | 6.174 | 35.737 | -10.050 | 1.00 | 55.69 | GZ00 | C |
| ATOM | 2857 | N | LYS | D | 160 | 3.157 | 39.269 | -10.654 | 1.00 | 74.88 | GZ00 | N |
| ATOM | 2858 | CA | LYS | D | 160 | 2.552 | 40.558 | -10.329 | 1.00 | 76.06 | GZ00 | C |
| ATOM | 2859 | C | LYS | D | 160 | 2.274 | 40.696 | -8.831 | 1.00 | 82.77 | GZ00 | C |
| ATOM | 2860 | O | LYS | D | 160 | 2.850 | 41.565 | -8.160 | 1.00 | 78.66 | GZ00 | O |
| ATOM | 2861 | CB | LYS | D | 160 | 1.265 | 40.755 | -11.142 | 1.00 | 83.14 | GZ00 | C |
| ATOM | 2862 | CG | LYS | D | 160 | 1.460 | 41.529 | -12.445 | 1.00 | 91.80 | GZ00 | C |
| ATOM | 2863 | CD | LYS | D | 160 | 2.006 | 42.934 | -12.175 | 1.00100.07 | | GZ00 | C |
| ATOM | 2864 | CE | LYS | D | 160 | 1.058 | 43.758 | -11.302 | 1.00 | 96.51 | GZ00 | C |
| ATOM | 2865 | NZ | LYS | D | 160 | 1.729 | 44.973 | -10.750 | 1.00 | 94.46 | GZ00 | N1+ |
| ATOM | 2866 | N | ALA | D | 161 | 1.410 | 39.836 | -8.293 | 1.00 | 72.49 | GZ00 | N |
| ATOM | 2867 | CA | ALA | D | 161 | 0.885 | 40.022 | -6.947 | 1.00 | 69.27 | GZ00 | C |
| ATOM | 2868 | C | ALA | D | 161 | 1.739 | 39.312 | -5.903 | 1.00 | 63.52 | GZ00 | C |
| ATOM | 2869 | O | ALA | D | 161 | 2.490 | 38.379 | -6.201 | 1.00 | 56.61 | GZ00 | O |
| ATOM | 2870 | CB | ALA | D | 161 | -0.551 | 39.514 | -6.859 | 1.00 | 71.61 | GZ00 | C |
| ATOM | 2871 | N | GLY | D | 162 | 1.608 | 39.774 | -4.659 | 1.00 | 50.21 | GZ00 | N |
| ATOM | 2872 | CA | GLY | D | 162 | 2.313 | 39.191 | -3.536 | 1.00 | 58.65 | GZ00 | C |
| ATOM | 2873 | C | GLY | D | 162 | 3.711 | 39.714 | -3.291 | 1.00 | 57.47 | GZ00 | C |
| ATOM | 2874 | O | GLY | D | 162 | 4.417 | 39.167 | -2.426 | 1.00 | 54.30 | GZ00 | O |
| ATOM | 2875 | N | VAL | D | 163 | 4.135 | 40.745 | -4.023 | 1.00 | 51.81 | GZ00 | N |
| ATOM | 2876 | CA | VAL | D | 163 | 5.480 | 41.291 | -3.929 | 1.00 | 45.48 | GZ00 | C |
| ATOM | 2877 | C | VAL | D | 163 | 5.431 | 42.543 | -3.074 | 1.00 | 47.42 | GZ00 | C |
| ATOM | 2878 | O | VAL | D | 163 | 4.618 | 43.442 | -3.316 | 1.00 | 44.17 | GZ00 | O |
| ATOM | 2879 | CB | VAL | D | 163 | 6.060 | 41.604 | -5.319 | 1.00 | 46.37 | GZ00 | C |
| ATOM | 2880 | CG1 | VAL | D | 163 | 7.423 | 42.278 | -5.184 | 1.00 | 50.48 | GZ00 | C |
| ATOM | 2881 | CG2 | VAL | D | 163 | 6.183 | 40.338 | -6.140 | 1.00 | 47.00 | GZ00 | C |
| ATOM | 2882 | N | GLU | D | 164 | 6.301 | 42.609 | -2.076 | 1.00 | 45.49 | GZ00 | N |
| ATOM | 2883 | CA | GLU | D | 164 | 6.498 | 43.832 | -1.326 | 1.00 | 39.71 | GZ00 | C |
| ATOM | 2884 | C | GLU | D | 164 | 7.990 | 44.067 | -1.169 | 1.00 | 48.75 | GZ00 | C |
| ATOM | 2885 | O | GLU | D | 164 | 8.749 | 43.133 | -0.864 | 1.00 | 44.83 | GZ00 | O |
| ATOM | 2886 | CB | GLU | D | 164 | 5.764 | 43.793 | 0.009 | 1.00 | 46.37 | GZ00 | C |
| ATOM | 2887 | CG | GLU | D | 164 | 4.308 | 44.218 | -0.195 | 1.00 | 59.54 | GZ00 | C |
| ATOM | 2888 | CD | GLU | D | 164 | 3.415 | 43.931 | 0.978 | 1.00 | 60.43 | GZ00 | C |
| ATOM | 2889 | OE1 | GLU | D | 164 | 3.928 | 43.465 | 2.015 | 1.00 | 70.95 | GZ00 | O |
| ATOM | 2890 | OE2 | GLU | D | 164 | 2.197 | 44.189 | 0.869 | 1.00 | 56.32 | GZ00 | O1- |
| ATOM | 2891 | N | THR | D | 165 | 8.400 | 45.308 | -1.434 | 1.00 | 43.26 | GZ00 | N |
| ATOM | 2892 | CA | THR | D | 165 | 9.794 | 45.691 | -1.572 | 1.00 | 34.34 | GZ00 | C |
| ATOM | 2893 | C | THR | D | 165 | 10.043 | 46.955 | -0.778 | 1.00 | 37.84 | GZ00 | C |
| ATOM | 2894 | O | THR | D | 165 | 9.267 | 47.903 | -0.883 | 1.00 | 43.88 | GZ00 | O |
| ATOM | 2895 | CB | THR | D | 165 | 10.122 | 45.903 | -3.040 | 1.00 | 37.47 | GZ00 | C |
| ATOM | 2896 | OG1 | THR | D | 165 | 9.909 | 44.671 | -3.730 | 1.00 | 39.61 | GZ00 | O |
| ATOM | 2897 | CG2 | THR | D | 165 | 11.578 | 46.354 | -3.240 | 1.00 | 38.05 | GZ00 | C |
| ATOM | 2898 | N | THR | D | 166 | 11.131 | 46.982 | -0.011 | 1.00 | 37.74 | GZ00 | N |
| ATOM | 2899 | CA | THR | D | 166 | 11.464 | 48.177 | 0.757 | 1.00 | 47.21 | GZ00 | C |
| ATOM | 2900 | C | THR | D | 166 | 12.099 | 49.231 | -0.141 | 1.00 | 40.41 | GZ00 | C |
| ATOM | 2901 | O | THR | D | 166 | 12.548 | 48.951 | -1.256 | 1.00 | 41.42 | GZ00 | O |
| ATOM | 2902 | CB | THR | D | 166 | 12.470 | 47.888 | 1.882 | 1.00 | 40.73 | GZ00 | C |
| ATOM | 2903 | OG1 | THR | D | 166 | 13.677 | 47.375 | 1.311 | 1.00 | 36.35 | GZ00 | O |
| ATOM | 2904 | CG2 | THR | D | 166 | 11.922 | 46.878 | 2.882 | 1.00 | 40.85 | GZ00 | C |
| ATOM | 2905 | N | THR | D | 167 | 12.135 | 50.451 | 0.365 | 1.00 | 42.55 | GZ00 | N |
| ATOM | 2906 | CA | THR | D | 167 | 12.976 | 51.467 | -0.237 | 1.00 | 49.39 | GZ00 | C |
| ATOM | 2907 | C | THR | D | 167 | 14.429 | 51.231 | 0.164 | 1.00 | 47.70 | GZ00 | C |
| ATOM | 2908 | O | THR | D | 167 | 14.702 | 50.656 | 1.218 | 1.00 | 50.42 | GZ00 | O |

| | | | | | | | | | | | |
|---|---|---|---|---|---|---|---|---|---|---|---|
| ATOM | 2909 | CB | THR | D | 167 | 12.564 | 52.846 | 0.231 | 1.00 | 42.03 | GZ00 C |
| ATOM | 2910 | OG1 | THR | D | 167 | 12.784 | 52.916 | 1.640 | 1.00 | 55.83 | GZ00 O |
| ATOM | 2911 | CG2 | THR | D | 167 | 11.133 | 53.079 | -0.052 | 1.00 | 40.08 | GZ00 C |
| ATOM | 2912 | N | PRO | D | 168 | 15.383 | 51.666 | -0.647 | 1.00 | 50.17 | GZ00 N |
| ATOM | 2913 | CA | PRO | D | 168 | 16.789 | 51.463 | -0.271 | 1.00 | 46.73 | GZ00 C |
| ATOM | 2914 | C | PRO | D | 168 | 17.132 | 52.312 | 0.943 | 1.00 | 48.10 | GZ00 C |
| ATOM | 2915 | O | PRO | D | 168 | 16.592 | 53.405 | 1.140 | 1.00 | 47.07 | GZ00 O |
| ATOM | 2916 | CB | PRO | D | 168 | 17.572 | 51.906 | -1.515 | 1.00 | 44.24 | GZ00 C |
| ATOM | 2917 | CG | PRO | D | 168 | 16.579 | 52.648 | -2.378 | 1.00 | 51.68 | GZ00 C |
| ATOM | 2918 | CD | PRO | D | 168 | 15.216 | 52.134 | -2.030 | 1.00 | 42.84 | GZ00 C |
| ATOM | 2919 | N | SER | D | 169 | 18.006 | 51.773 | 1.789 | 1.00 | 38.56 | GZ00 N |
| ATOM | 2920 | CA | SER | D | 169 | 18.460 | 52.490 | 2.976 | 1.00 | 44.06 | GZ00 C |
| ATOM | 2921 | C | SER | D | 169 | 19.974 | 52.364 | 3.091 | 1.00 | 40.12 | GZ00 C |
| ATOM | 2922 | O | SER | D | 169 | 20.562 | 51.347 | 2.703 | 1.00 | 42.12 | GZ00 O |
| ATOM | 2923 | CB | SER | D | 169 | 17.776 | 51.973 | 4.261 | 1.00 | 37.35 | GZ00 C |
| ATOM | 2924 | OG | SER | D | 169 | 18.098 | 50.608 | 4.455 | 1.00 | 51.17 | GZ00 O |
| ATOM | 2925 | N | LYS | D | 170 | 20.601 | 53.409 | 3.614 | 1.00 | 38.28 | GZ00 N |
| ATOM | 2926 | CA | LYS | D | 170 | 22.053 | 53.472 | 3.624 | 1.00 | 42.03 | GZ00 C |
| ATOM | 2927 | C | LYS | D | 170 | 22.610 | 52.541 | 4.691 | 1.00 | 41.78 | GZ00 C |
| ATOM | 2928 | O | LYS | D | 170 | 22.160 | 52.564 | 5.839 | 1.00 | 44.51 | GZ00 O |
| ATOM | 2929 | CB | LYS | D | 170 | 22.542 | 54.897 | 3.877 | 1.00 | 38.33 | GZ00 C |
| ATOM | 2930 | CG | LYS | D | 170 | 24.014 | 55.068 | 3.482 | 1.00 | 45.43 | GZ00 C |
| ATOM | 2931 | CD | LYS | D | 170 | 24.575 | 56.405 | 3.934 | 1.00 | 48.05 | GZ00 C |
| ATOM | 2932 | CE | LYS | D | 170 | 23.962 | 57.548 | 3.176 | 1.00 | 54.50 | GZ00 C |
| ATOM | 2933 | NZ | LYS | D | 170 | 24.635 | 58.806 | 3.569 | 1.00 | 69.11 | GZ00 N1+ |
| ATOM | 2934 | N | GLN | D | 171 | 23.589 | 51.723 | 4.305 | 1.00 | 41.93 | GZ00 N |
| ATOM | 2935 | CA | GLN | D | 171 | 24.346 | 50.889 | 5.228 | 1.00 | 47.20 | GZ00 C |
| ATOM | 2936 | C | GLN | D | 171 | 25.483 | 51.692 | 5.858 | 1.00 | 45.26 | GZ00 C |
| ATOM | 2937 | O | GLN | D | 171 | 25.816 | 52.797 | 5.422 | 1.00 | 47.74 | GZ00 O |
| ATOM | 2938 | CB | GLN | D | 171 | 24.933 | 49.672 | 4.508 | 1.00 | 38.79 | GZ00 C |
| ATOM | 2939 | CG | GLN | D | 171 | 23.914 | 48.809 | 3.823 | 1.00 | 38.60 | GZ00 C |
| ATOM | 2940 | CD | GLN | D | 171 | 24.560 | 47.787 | 2.932 | 1.00 | 45.79 | GZ00 C |
| ATOM | 2941 | OE1 | GLN | D | 171 | 23.900 | 46.859 | 2.433 | 1.00 | 43.80 | GZ00 O |
| ATOM | 2942 | NE2 | GLN | D | 171 | 25.868 | 47.942 | 2.715 | 1.00 | 45.31 | GZ00 N |
| ATOM | 2943 | N | SER | D | 172 | 26.118 | 51.089 | 6.865 | 1.00 | 49.48 | GZ00 N |
| ATOM | 2944 | CA | SER | D | 172 | 27.258 | 51.724 | 7.529 | 1.00 | 45.76 | GZ00 C |
| ATOM | 2945 | C | SER | D | 172 | 28.391 | 52.012 | 6.557 | 1.00 | 43.14 | GZ00 C |
| ATOM | 2946 | O | SER | D | 172 | 29.073 | 53.033 | 6.675 | 1.00 | 51.69 | GZ00 O |
| ATOM | 2947 | CB | SER | D | 172 | 27.769 | 50.826 | 8.658 | 1.00 | 55.56 | GZ00 C |
| ATOM | 2948 | OG | SER | D | 172 | 26.906 | 50.898 | 9.777 | 1.00 | 71.74 | GZ00 O |
| ATOM | 2949 | N | ASN | D | 173 | 28.628 | 51.122 | 5.601 | 1.00 | 40.90 | GZ00 N |
| ATOM | 2950 | CA | ASN | D | 173 | 29.697 | 51.363 | 4.646 | 1.00 | 37.43 | GZ00 C |
| ATOM | 2951 | C | ASN | D | 173 | 29.304 | 52.349 | 3.542 | 1.00 | 44.12 | GZ00 C |
| ATOM | 2952 | O | ASN | D | 173 | 30.080 | 52.526 | 2.588 | 1.00 | 41.37 | GZ00 O |
| ATOM | 2953 | CB | ASN | D | 173 | 30.171 | 50.030 | 4.038 | 1.00 | 41.28 | GZ00 C |
| ATOM | 2954 | CG | ASN | D | 173 | 29.139 | 49.370 | 3.147 | 1.00 | 44.26 | GZ00 C |
| ATOM | 2955 | OD1 | ASN | D | 173 | 28.038 | 49.887 | 2.944 | 1.00 | 43.64 | GZ00 O |
| ATOM | 2956 | ND2 | ASN | D | 173 | 29.500 | 48.208 | 2.597 | 1.00 | 50.50 | GZ00 N |
| ATOM | 2957 | N | ASN | D | 174 | 28.142 | 52.999 | 3.669 | 1.00 | 41.55 | GZ00 N |
| ATOM | 2958 | CA | ASN | D | 174 | 27.608 | 54.005 | 2.759 | 1.00 | 44.88 | GZ00 C |
| ATOM | 2959 | C | ASN | D | 174 | 27.151 | 53.429 | 1.426 | 1.00 | 44.23 | GZ00 C |
| ATOM | 2960 | O | ASN | D | 174 | 26.811 | 54.200 | 0.525 | 1.00 | 39.27 | GZ00 O |
| ATOM | 2961 | CB | ASN | D | 174 | 28.610 | 55.139 | 2.484 | 1.00 | 42.38 | GZ00 C |
| ATOM | 2962 | CG | ASN | D | 174 | 28.741 | 56.082 | 3.653 | 1.00 | 45.60 | GZ00 C |
| ATOM | 2963 | OD1 | ASN | D | 174 | 27.827 | 56.220 | 4.457 | 1.00 | 52.29 | GZ00 O |
| ATOM | 2964 | ND2 | ASN | D | 174 | 29.895 | 56.706 | 3.775 | 1.00 | 48.22 | GZ00 N |
| ATOM | 2965 | N | LYS | D | 175 | 27.187 | 52.114 | 1.242 | 1.00 | 43.55 | GZ00 N |
| ATOM | 2966 | CA | LYS | D | 175 | 26.380 | 51.487 | 0.204 | 1.00 | 46.60 | GZ00 C |
| ATOM | 2967 | C | LYS | D | 175 | 24.958 | 51.250 | 0.748 | 1.00 | 40.26 | GZ00 C |
| ATOM | 2968 | O | LYS | D | 175 | 24.628 | 51.624 | 1.877 | 1.00 | 36.35 | GZ00 O |
| ATOM | 2969 | CB | LYS | D | 175 | 27.054 | 50.204 | -0.272 | 1.00 | 43.68 | GZ00 C |
| ATOM | 2970 | CG | LYS | D | 175 | 28.501 | 50.379 | -0.772 | 1.00 | 42.53 | GZ00 C |
| ATOM | 2971 | CD | LYS | D | 175 | 29.043 | 48.983 | -1.135 | 1.00 | 47.00 | GZ00 C |
| ATOM | 2972 | CE | LYS | D | 175 | 30.522 | 48.940 | -1.403 | 1.00 | 54.60 | GZ00 C |
| ATOM | 2973 | NZ | LYS | D | 175 | 30.884 | 47.568 | -1.857 | 1.00 | 65.92 | GZ00 N |
| ATOM | 2974 | N | TYR | D | 176 | 24.091 | 50.623 | -0.042 | 1.00 | 37.23 | GZ00 N |
| ATOM | 2975 | CA | TYR | D | 176 | 22.676 | 50.583 | 0.302 | 1.00 | 36.34 | GZ00 C |
| ATOM | 2976 | C | TYR | D | 176 | 22.145 | 49.158 | 0.349 | 1.00 | 42.73 | GZ00 C |
| ATOM | 2977 | O | TYR | D | 176 | 22.657 | 48.244 | -0.314 | 1.00 | 36.34 | GZ00 O |
| ATOM | 2978 | CB | TYR | D | 176 | 21.844 | 51.409 | -0.693 | 1.00 | 36.80 | GZ00 C |
| ATOM | 2979 | CG | TYR | D | 176 | 22.044 | 52.899 | -0.563 | 1.00 | 36.36 | GZ00 C |

| | | | | | | | | | | | | |
|---|---|---|---|---|---|---|---|---|---|---|---|---|
| ATOM | 2980 | CD2 | TYR | D | 176 | 21.057 | 53.710 | -0.021 | 1.00 | 37.31 | GZ00 | C |
| ATOM | 2981 | CD1 | TYR | D | 176 | 23.222 | 53.497 | -0.996 | 1.00 | 41.41 | GZ00 | C |
| ATOM | 2982 | CE2 | TYR | D | 176 | 21.252 | 55.072 | 0.098 | 1.00 | 40.59 | GZ00 | C |
| ATOM | 2983 | CE1 | TYR | D | 176 | 23.423 | 54.857 | -0.883 | 1.00 | 39.52 | GZ00 | C |
| ATOM | 2984 | CZ | TYR | D | 176 | 22.440 | 55.639 | -0.347 | 1.00 | 39.72 | GZ00 | C |
| ATOM | 2985 | OH | TYR | D | 176 | 22.662 | 56.981 | -0.238 | 1.00 | 49.13 | GZ00 | O |
| ATOM | 2986 | N | ALA | D | 177 | 21.056 | 49.003 | 1.103 | 1.00 | 41.16 | GZ00 | N |
| ATOM | 2987 | CA | ALA | D | 177 | 20.371 | 47.728 | 1.246 | 1.00 | 39.60 | GZ00 | C |
| ATOM | 2988 | C | ALA | D | 177 | 18.886 | 47.901 | 0.957 | 1.00 | 37.33 | GZ00 | C |
| ATOM | 2989 | O | ALA | D | 177 | 18.321 | 48.987 | 1.140 | 1.00 | 41.92 | GZ00 | O |
| ATOM | 2990 | CB | ALA | D | 177 | 20.552 | 47.133 | 2.665 | 1.00 | 35.41 | GZ00 | C |
| ATOM | 2991 | N | ALA | D | 178 | 18.271 | 46.809 | 0.488 | 1.00 | 36.97 | GZ00 | N |
| ATOM | 2992 | CA | ALA | D | 178 | 16.829 | 46.711 | 0.308 | 1.00 | 39.14 | GZ00 | C |
| ATOM | 2993 | C | ALA | D | 178 | 16.415 | 45.246 | 0.381 | 1.00 | 41.15 | GZ00 | C |
| ATOM | 2994 | O | ALA | D | 178 | 17.230 | 44.339 | 0.192 | 1.00 | 38.19 | GZ00 | O |
| ATOM | 2995 | CB | ALA | D | 178 | 16.380 | 47.324 | -1.023 | 1.00 | 31.46 | GZ00 | C |
| ATOM | 2996 | N | SER | D | 179 | 15.125 | 45.012 | 0.628 | 1.00 | 39.23 | GZ00 | N |
| ATOM | 2997 | CA | SER | D | 179 | 14.626 | 43.644 | 0.607 | 1.00 | 42.89 | GZ00 | C |
| ATOM | 2998 | C | SER | D | 179 | 13.279 | 43.569 | -0.100 | 1.00 | 42.30 | GZ00 | C |
| ATOM | 2999 | O | SER | D | 179 | 12.526 | 44.543 | -0.157 | 1.00 | 39.32 | GZ00 | O |
| ATOM | 3000 | CB | SER | D | 179 | 14.561 | 43.043 | 2.026 | 1.00 | 37.57 | GZ00 | C |
| ATOM | 3001 | OG | SER | D | 179 | 14.059 | 43.953 | 2.960 | 1.00 | 39.68 | GZ00 | O |
| ATOM | 3002 | N | SER | D | 180 | 13.012 | 42.399 | -0.673 | 1.00 | 39.99 | GZ00 | N |
| ATOM | 3003 | CA | SER | D | 180 | 11.786 | 42.133 | -1.407 | 1.00 | 45.51 | GZ00 | C |
| ATOM | 3004 | C | SER | D | 180 | 11.212 | 40.781 | -0.972 | 1.00 | 51.71 | GZ00 | C |
| ATOM | 3005 | O | SER | D | 180 | 11.932 | 39.774 | -0.921 | 1.00 | 43.37 | GZ00 | O |
| ATOM | 3006 | CB | SER | D | 180 | 12.062 | 42.153 | -2.911 | 1.00 | 42.12 | GZ00 | C |
| ATOM | 3007 | OG | SER | D | 180 | 10.869 | 42.076 | -3.655 | 1.00 | 40.91 | GZ00 | O |
| ATOM | 3008 | N | TYR | D | 181 | 9.914 | 40.756 | -0.686 | 1.00 | 44.23 | GZ00 | N |
| ATOM | 3009 | CA | TYR | D | 181 | 9.237 | 39.561 | -0.206 | 1.00 | 45.60 | GZ00 | C |
| ATOM | 3010 | C | TYR | D | 181 | 8.159 | 39.158 | -1.203 | 1.00 | 48.02 | GZ00 | C |
| ATOM | 3011 | O | TYR | D | 181 | 7.369 | 40.001 | -1.637 | 1.00 | 44.19 | GZ00 | O |
| ATOM | 3012 | CB | TYR | D | 181 | 8.612 | 39.804 | 1.164 | 1.00 | 39.73 | GZ00 | C |
| ATOM | 3013 | CG | TYR | D | 181 | 9.601 | 40.149 | 2.246 | 1.00 | 42.39 | GZ00 | C |
| ATOM | 3014 | CD2 | TYR | D | 181 | 10.094 | 39.167 | 3.100 | 1.00 | 35.39 | GZ00 | C |
| ATOM | 3015 | CD1 | TYR | D | 181 | 10.050 | 41.464 | 2.416 | 1.00 | 38.95 | GZ00 | C |
| ATOM | 3016 | CE2 | TYR | D | 181 | 11.005 | 39.477 | 4.098 | 1.00 | 38.77 | GZ00 | C |
| ATOM | 3017 | CE1 | TYR | D | 181 | 10.958 | 41.786 | 3.414 | 1.00 | 42.49 | GZ00 | C |
| ATOM | 3018 | CZ | TYR | D | 181 | 11.437 | 40.785 | 4.254 | 1.00 | 40.93 | GZ00 | C |
| ATOM | 3019 | OH | TYR | D | 181 | 12.335 | 41.092 | 5.250 | 1.00 | 36.93 | GZ00 | O |
| ATOM | 3020 | N | LEU | D | 182 | 8.137 | 37.878 | -1.576 | 1.00 | 39.63 | GZ00 | N |
| ATOM | 3021 | CA | LEU | D | 182 | 7.075 | 37.312 | -2.399 | 1.00 | 48.32 | GZ00 | C |
| ATOM | 3022 | C | LEU | D | 182 | 6.258 | 36.347 | -1.533 | 1.00 | 49.68 | GZ00 | C |
| ATOM | 3023 | O | LEU | D | 182 | 6.774 | 35.321 | -1.072 | 1.00 | 44.59 | GZ00 | O |
| ATOM | 3024 | CB | LEU | D | 182 | 7.672 | 36.616 | -3.621 | 1.00 | 45.93 | GZ00 | C |
| ATOM | 3025 | CG | LEU | D | 182 | 6.711 | 35.859 | -4.540 | 1.00 | 54.86 | GZ00 | C |
| ATOM | 3026 | CD1 | LEU | D | 182 | 5.643 | 36.822 | -5.050 | 1.00 | 49.90 | GZ00 | C |
| ATOM | 3027 | CD2 | LEU | D | 182 | 7.467 | 35.191 | -5.703 | 1.00 | 43.02 | GZ00 | C |
| ATOM | 3028 | N | SER | D | 183 | 4.992 | 36.680 | -1.295 | 1.00 | 52.23 | GZ00 | N |
| ATOM | 3029 | CA | SER | D | 183 | 4.099 | 35.806 | -0.538 | 1.00 | 47.98 | GZ00 | C |
| ATOM | 3030 | C | SER | D | 183 | 3.397 | 34.840 | -1.481 | 1.00 | 47.04 | GZ00 | C |
| ATOM | 3031 | O | SER | D | 183 | 2.956 | 35.225 | -2.564 | 1.00 | 52.72 | GZ00 | O |
| ATOM | 3032 | CB | SER | D | 183 | 3.072 | 36.608 | 0.251 | 1.00 | 45.88 | GZ00 | C |
| ATOM | 3033 | OG | SER | D | 183 | 3.720 | 37.533 | 1.102 | 1.00 | 53.76 | GZ00 | O |
| ATOM | 3034 | N | LEU | D | 184 | 3.356 | 33.574 | -1.088 | 1.00 | 51.61 | GZ00 | N |
| ATOM | 3035 | CA | LEU | D | 184 | 2.741 | 32.523 | -1.874 | 1.00 | 51.65 | GZ00 | C |
| ATOM | 3036 | C | LEU | D | 184 | 1.951 | 31.621 | -0.944 | 1.00 | 60.30 | GZ00 | C |
| ATOM | 3037 | O | LEU | D | 184 | 2.089 | 31.679 | 0.284 | 1.00 | 57.40 | GZ00 | O |
| ATOM | 3038 | CB | LEU | D | 184 | 3.777 | 31.687 | -2.623 | 1.00 | 53.86 | GZ00 | C |
| ATOM | 3039 | CG | LEU | D | 184 | 4.672 | 32.341 | -3.654 | 1.00 | 51.77 | GZ00 | C |
| ATOM | 3040 | CD1 | LEU | D | 184 | 5.638 | 31.317 | -4.213 | 1.00 | 50.48 | GZ00 | C |
| ATOM | 3041 | CD2 | LEU | D | 184 | 3.803 | 32.927 | -4.738 | 1.00 | 58.87 | GZ00 | C |
| ATOM | 3042 | N | THR | D | 185 | 1.097 | 30.745 | -1.569 | 1.00 | 63.83 | GZ00 | N |
| ATOM | 3043 | CA | THR | D | 185 | 0.577 | 29.598 | -0.843 | 1.00 | 63.50 | GZ00 | C |
| ATOM | 3044 | C | THR | D | 185 | 1.550 | 28.438 | -0.977 | 1.00 | 60.27 | GZ00 | C |
| ATOM | 3045 | O | THR | D | 185 | 2.270 | 28.332 | -1.982 | 1.00 | 54.12 | GZ00 | O |
| ATOM | 3046 | CB | THR | D | 185 | -0.782 | 29.160 | -1.397 | 1.00 | 58.04 | GZ00 | C |
| ATOM | 3047 | OG1 | THR | D | 185 | -0.611 | 28.645 | -2.727 | 1.00 | 62.01 | GZ00 | O |
| ATOM | 3048 | CG2 | THR | D | 185 | -1.765 | 30.324 | -1.417 | 1.00 | 49.17 | GZ00 | C |
| ATOM | 3049 | N | PRO | D | 186 | 1.567 | 27.545 | 0.008 | 1.00 | 56.65 | GZ00 | N |
| ATOM | 3050 | CA | PRO | D | 186 | 2.337 | 26.307 | -0.160 | 1.00 | 61.35 | GZ00 | C |

| | | | | | | | | | | | | |
|---|---|---|---|---|---|---|---|---|---|---|---|---|
| ATOM | 3051 | C | PRO | D | 186 | 2.054 | 25.617 | -1.484 | 1.00 | 70.92 | GZ00 | C |
| ATOM | 3052 | O | PRO | D | 186 | 2.972 | 25.043 | -2.089 | 1.00 | 71.62 | GZ00 | O |
| ATOM | 3053 | CB | PRO | D | 186 | 1.893 | 25.474 | 1.047 | 1.00 | 64.94 | GZ00 | C |
| ATOM | 3054 | CG | PRO | D | 186 | 1.565 | 26.502 | 2.095 | 1.00 | 56.23 | GZ00 | C |
| ATOM | 3055 | CD | PRO | D | 186 | 0.970 | 27.657 | 1.353 | 1.00 | 57.72 | GZ00 | C |
| ATOM | 3056 | N | GLU | D | 187 | 0.815 | 25.725 | -1.981 | 1.00 | 72.13 | GZ00 | N |
| ATOM | 3057 | CA | GLU | D | 187 | 0.440 | 25.117 | -3.255 | 1.00 | 73.38 | GZ00 | C |
| ATOM | 3058 | C | GLU | D | 187 | 1.182 | 25.782 | -4.408 | 1.00 | 71.22 | GZ00 | C |
| ATOM | 3059 | O | GLU | D | 187 | 1.817 | 25.105 | -5.229 | 1.00 | 67.47 | GZ00 | O |
| ATOM | 3060 | CB | GLU | D | 187 | -1.074 | 25.235 | -3.473 | 1.00 | 72.72 | GZ00 | C |
| ATOM | 3061 | CG | GLU | D | 187 | -1.909 | 25.465 | -2.206 | 1.00 | 73.60 | GZ00 | C |
| ATOM | 3062 | CD | GLU | D | 187 | -1.655 | 24.438 | -1.114 | 1.00 | 91.17 | GZ00 | C |
| ATOM | 3063 | OE1 | GLU | D | 187 | -1.492 | 23.239 | -1.442 | 1.00 | 95.11 | GZ00 | O |
| ATOM | 3064 | OE2 | GLU | D | 187 | -1.608 | 24.842 | 0.072 | 1.00 | 86.41 | GZ00 | O1- |
| ATOM | 3065 | N | GLN | D | 188 | 1.080 | 27.116 | -4.499 | 1.00 | 63.92 | GZ00 | N |
| ATOM | 3066 | CA | GLN | D | 188 | 1.803 | 27.852 | -5.532 | 1.00 | 67.27 | GZ00 | C |
| ATOM | 3067 | C | GLN | D | 188 | 3.302 | 27.589 | -5.466 | 1.00 | 68.44 | GZ00 | C |
| ATOM | 3068 | O | GLN | D | 188 | 3.959 | 27.448 | -6.507 | 1.00 | 68.93 | GZ00 | O |
| ATOM | 3069 | CB | GLN | D | 188 | 1.524 | 29.347 | -5.411 | 1.00 | 69.32 | GZ00 | C |
| ATOM | 3070 | CG | GLN | D | 188 | 0.092 | 29.737 | -5.677 | 1.00 | 64.52 | GZ00 | C |
| ATOM | 3071 | CD | GLN | D | 188 | -0.225 | 31.124 | -5.174 | 1.00 | 74.32 | GZ00 | C |
| ATOM | 3072 | OE1 | GLN | D | 188 | 0.362 | 31.585 | -4.199 | 1.00 | 75.61 | GZ00 | O |
| ATOM | 3073 | NE2 | GLN | D | 188 | -1.147 | 31.808 | -5.847 | 1.00 | 79.67 | GZ00 | N |
| ATOM | 3074 | N | TRP | D | 189 | 3.865 | 27.522 | -4.255 | 1.00 | 65.05 | GZ00 | N |
| ATOM | 3075 | CA | TRP | D | 189 | 5.304 | 27.301 | -4.130 | 1.00 | 65.52 | GZ00 | C |
| ATOM | 3076 | C | TRP | D | 189 | 5.697 | 25.960 | -4.734 | 1.00 | 70.69 | GZ00 | C |
| ATOM | 3077 | O | TRP | D | 189 | 6.669 | 25.871 | -5.497 | 1.00 | 69.56 | GZ00 | O |
| ATOM | 3078 | CB | TRP | D | 189 | 5.730 | 27.397 | -2.659 | 1.00 | 64.52 | GZ00 | C |
| ATOM | 3079 | CG | TRP | D | 189 | 7.127 | 26.879 | -2.336 | 1.00 | 69.67 | GZ00 | C |
| ATOM | 3080 | CD1 | TRP | D | 189 | 7.448 | 25.892 | -1.442 | 1.00 | 67.82 | GZ00 | C |
| ATOM | 3081 | CD2 | TRP | D | 189 | 8.372 | 27.335 | -2.885 | 1.00 | 66.64 | GZ00 | C |
| ATOM | 3082 | NE1 | TRP | D | 189 | 8.806 | 25.699 | -1.414 | 1.00 | 62.01 | GZ00 | N |
| ATOM | 3083 | CE2 | TRP | D | 189 | 9.399 | 26.569 | -2.287 | 1.00 | 64.99 | GZ00 | C |
| ATOM | 3084 | CE3 | TRP | D | 189 | 8.720 | 28.305 | -3.833 | 1.00 | 68.36 | GZ00 | C |
| ATOM | 3085 | CZ2 | TRP | D | 189 | 10.750 | 26.753 | -2.592 | 1.00 | 66.69 | GZ00 | C |
| ATOM | 3086 | CZ3 | TRP | D | 189 | 10.067 | 28.481 | -4.146 | 1.00 | 68.79 | GZ00 | C |
| ATOM | 3087 | CH2 | TRP | D | 189 | 11.064 | 27.704 | -3.527 | 1.00 | 63.38 | GZ00 | C |
| ATOM | 3088 | N | LYS | D | 190 | 4.925 | 24.909 | -4.437 | 1.00 | 74.93 | GZ00 | N |
| ATOM | 3089 | CA | LYS | D | 190 | 5.269 | 23.562 | -4.875 | 1.00 | 74.61 | GZ00 | C |
| ATOM | 3090 | C | LYS | D | 190 | 4.919 | 23.317 | -6.334 | 1.00 | 72.06 | GZ00 | C |
| ATOM | 3091 | O | LYS | D | 190 | 5.471 | 22.395 | -6.946 | 1.00 | 76.50 | GZ00 | O |
| ATOM | 3092 | CB | LYS | D | 190 | 4.556 | 22.538 | -3.988 | 1.00 | 72.08 | GZ00 | C |
| ATOM | 3093 | CG | LYS | D | 190 | 5.012 | 22.585 | -2.539 | 1.00 | 73.58 | GZ00 | C |
| ATOM | 3094 | CD | LYS | D | 190 | 4.154 | 21.723 | -1.635 | 1.00 | 86.29 | GZ00 | C |
| ATOM | 3095 | CE | LYS | D | 190 | 4.625 | 21.833 | -0.191 | 1.00 | 92.57 | GZ00 | C |
| ATOM | 3096 | NZ | LYS | D | 190 | 3.732 | 21.118 | 0.762 | 1.00 | 99.72 | GZ00 | N1+ |
| ATOM | 3097 | N | SER | D | 191 | 4.050 | 24.142 | -6.915 | 1.00 | 68.71 | GZ00 | N |
| ATOM | 3098 | CA | SER | D | 191 | 3.582 | 23.882 | -8.272 | 1.00 | 73.46 | GZ00 | C |
| ATOM | 3099 | C | SER | D | 191 | 4.650 | 24.213 | -9.315 | 1.00 | 72.57 | GZ00 | C |
| ATOM | 3100 | O | SER | D | 191 | 4.878 | 23.431 | -10.244 | 1.00 | 79.90 | GZ00 | O |
| ATOM | 3101 | CB | SER | D | 191 | 2.289 | 24.655 | -8.529 | 1.00 | 70.09 | GZ00 | C |
| ATOM | 3102 | OG | SER | D | 191 | 2.531 | 26.044 | -8.539 | 1.00 | 81.55 | GZ00 | O |
| ATOM | 3103 | N | HIS | D | 192 | 5.313 | 25.363 | -9.190 | 1.00 | 72.67 | GZ00 | N |
| ATOM | 3104 | CA | HIS | D | 192 | 6.267 | 25.792 | -10.210 | 1.00 | 69.13 | GZ00 | C |
| ATOM | 3105 | C | HIS | D | 192 | 7.641 | 25.135 | -10.038 | 1.00 | 66.99 | GZ00 | C |
| ATOM | 3106 | O | HIS | D | 192 | 8.020 | 24.695 | -8.951 | 1.00 | 72.31 | GZ00 | O |
| ATOM | 3107 | CB | HIS | D | 192 | 6.420 | 27.313 | -10.199 | 1.00 | 72.85 | GZ00 | C |
| ATOM | 3108 | CG | HIS | D | 192 | 5.178 | 28.049 | -10.597 | 1.00 | 71.64 | GZ00 | C |
| ATOM | 3109 | ND1 | HIS | D | 192 | 4.172 | 28.355 | -9.704 | 1.00 | 70.93 | GZ00 | N |
| ATOM | 3110 | CD2 | HIS | D | 192 | 4.763 | 28.504 | -11.803 | 1.00 | 68.93 | GZ00 | C |
| ATOM | 3111 | CE1 | HIS | D | 192 | 3.202 | 28.990 | -10.338 | 1.00 | 65.00 | GZ00 | C |
| ATOM | 3112 | NE2 | HIS | D | 192 | 3.534 | 29.089 | -11.614 | 1.00 | 73.86 | GZ00 | N |
| ATOM | 3113 | N | ARG | D | 193 | 8.390 | 25.070 | -11.148 | 1.00 | 64.89 | GZ00 | N |
| ATOM | 3114 | CA | ARG | D | 193 | 9.731 | 24.476 | -11.135 | 1.00 | 69.27 | GZ00 | C |
| ATOM | 3115 | C | ARG | D | 193 | 10.705 | 25.313 | -10.313 | 1.00 | 67.85 | GZ00 | C |
| ATOM | 3116 | O | ARG | D | 193 | 11.542 | 24.769 | -9.586 | 1.00 | 68.86 | GZ00 | O |
| ATOM | 3117 | CB | ARG | D | 193 | 10.269 | 24.354 | -12.565 | 1.00 | 71.73 | GZ00 | C |
| ATOM | 3118 | CG | ARG | D | 193 | 9.414 | 23.557 | -13.540 | 1.00 | 81.33 | GZ00 | C |
| ATOM | 3119 | CD | ARG | D | 193 | 9.846 | 23.822 | -14.993 | 1.00 | 88.10 | GZ00 | C |
| ATOM | 3120 | NE | ARG | D | 193 | 8.722 | 24.419 | -15.728 | 1.00 | 99.94 | GZ00 | N |
| ATOM | 3121 | CZ | ARG | D | 193 | 8.814 | 25.092 | -16.875 | 1.00 | 101.58 | GZ00 | C |

| | | | | | | | | | | | | |
|---|---|---|---|---|---|---|---|---|---|---|---|---|
| ATOM | 3122 | NH1 | ARG | D | 193 | 7.716 | 25.589 | -17.441 | 1.00 | 88.37 | GZ00 | N1+ |
| ATOM | 3123 | NH2 | ARG | D | 193 | 9.997 | 25.305 | -17.440 | 1.00 | 99.51 | GZ00 | N |
| ATOM | 3124 | N | SER | D | 194 | 10.619 | 26.637 | -10.428 | 1.00 | 68.05 | GZ00 | N |
| ATOM | 3125 | CA | SER | D | 194 | 11.553 | 27.534 | -9.768 | 1.00 | 62.46 | GZ00 | C |
| ATOM | 3126 | C | SER | D | 194 | 10.936 | 28.917 | -9.658 | 1.00 | 66.77 | GZ00 | C |
| ATOM | 3127 | O | SER | D | 194 | 9.957 | 29.242 | -10.334 | 1.00 | 71.03 | GZ00 | O |
| ATOM | 3128 | CB | SER | D | 194 | 12.876 | 27.622 | -10.529 | 1.00 | 66.04 | GZ00 | C |
| ATOM | 3129 | OG | SER | D | 194 | 12.739 | 28.441 | -11.681 | 1.00 | 66.32 | GZ00 | O |
| ATOM | 3130 | N | TYR | D | 195 | 11.520 | 29.722 | -8.782 | 1.00 | 64.92 | GZ00 | N |
| ATOM | 3131 | CA | TYR | D | 195 | 11.219 | 31.142 | -8.688 | 1.00 | 63.56 | GZ00 | C |
| ATOM | 3132 | C | TYR | D | 195 | 12.522 | 31.927 | -8.748 | 1.00 | 61.50 | GZ00 | C |
| ATOM | 3133 | O | TYR | D | 195 | 13.580 | 31.431 | -8.351 | 1.00 | 54.93 | GZ00 | O |
| ATOM | 3134 | CB | TYR | D | 195 | 10.481 | 31.482 | -7.408 | 1.00 | 57.26 | GZ00 | C |
| ATOM | 3135 | CG | TYR | D | 195 | 9.037 | 31.058 | -7.392 | 1.00 | 64.67 | GZ00 | C |
| ATOM | 3136 | CD1 | TYR | D | 195 | 8.679 | 29.742 | -7.126 | 1.00 | 69.64 | GZ00 | C |
| ATOM | 3137 | CD2 | TYR | D | 195 | 8.025 | 31.986 | -7.618 | 1.00 | 66.11 | GZ00 | C |
| ATOM | 3138 | CE1 | TYR | D | 195 | 7.350 | 29.362 | -7.094 | 1.00 | 72.89 | GZ00 | C |
| ATOM | 3139 | CE2 | TYR | D | 195 | 6.704 | 31.621 | -7.592 | 1.00 | 63.75 | GZ00 | C |
| ATOM | 3140 | CZ | TYR | D | 195 | 6.369 | 30.311 | -7.336 | 1.00 | 69.62 | GZ00 | C |
| ATOM | 3141 | OH | TYR | D | 195 | 5.043 | 29.958 | -7.309 | 1.00 | 71.41 | GZ00 | O |
| ATOM | 3142 | N | SER | D | 196 | 12.441 | 33.161 | -9.235 | 1.00 | 58.30 | GZ00 | N |
| ATOM | 3143 | CA | SER | D | 196 | 13.627 | 33.990 | -9.396 | 1.00 | 54.75 | GZ00 | C |
| ATOM | 3144 | C | SER | D | 196 | 13.387 | 35.383 | -8.852 | 1.00 | 52.46 | GZ00 | C |
| ATOM | 3145 | O | SER | D | 196 | 12.282 | 35.924 | -8.944 | 1.00 | 50.50 | GZ00 | O |
| ATOM | 3146 | CB | SER | D | 196 | 14.068 | 34.102 | -10.856 | 1.00 | 55.06 | GZ00 | C |
| ATOM | 3147 | OG | SER | D | 196 | 14.543 | 32.862 | -11.337 | 1.00 | 59.56 | GZ00 | O |
| ATOM | 3148 | N | CYS | D | 197 | 14.434 | 35.941 | -8.263 | 1.00 | 51.88 | GZ00 | N |
| ATOM | 3149 | CA | CYS | D | 197 | 14.485 | 37.334 | -7.862 | 1.00 | 48.95 | GZ00 | C |
| ATOM | 3150 | C | CYS | D | 197 | 15.499 | 38.031 | -8.771 | 1.00 | 49.99 | GZ00 | C |
| ATOM | 3151 | O | CYS | D | 197 | 16.667 | 37.641 | -8.809 | 1.00 | 46.06 | GZ00 | O |
| ATOM | 3152 | CB | CYS | D | 197 | 14.868 | 37.443 | -6.389 | 1.00 | 50.65 | GZ00 | C |
| ATOM | 3153 | SG | CYS | D | 197 | 14.970 | 39.121 | -5.807 | 1.00 | 59.52 | GZ00 | S |
| ATOM | 3154 | N | GLN | D | 198 | 15.048 | 39.021 | -9.538 | 1.00 | 50.95 | GZ00 | N |
| ATOM | 3155 | CA | GLN | D | 198 | 15.912 | 39.731 | -10.476 | 1.00 | 45.62 | GZ00 | C |
| ATOM | 3156 | C | GLN | D | 198 | 16.058 | 41.165 | -10.006 | 1.00 | 45.99 | GZ00 | C |
| ATOM | 3157 | O | GLN | D | 198 | 15.064 | 41.888 | -9.860 | 1.00 | 43.74 | GZ00 | O |
| ATOM | 3158 | CB | GLN | D | 198 | 15.372 | 39.667 | -11.901 | 1.00 | 43.17 | GZ00 | C |
| ATOM | 3159 | CG | GLN | D | 198 | 15.534 | 38.283 | -12.527 | 1.00 | 59.20 | GZ00 | C |
| ATOM | 3160 | CD | GLN | D | 198 | 14.770 | 38.107 | -13.837 | 1.00 | 67.77 | GZ00 | C |
| ATOM | 3161 | OE1 | GLN | D | 198 | 14.034 | 38.996 | -14.273 | 1.00 | 68.74 | GZ00 | O |
| ATOM | 3162 | NE2 | GLN | D | 198 | 14.933 | 36.945 | -14.460 | 1.00 | 69.92 | GZ00 | N |
| ATOM | 3163 | N | VAL | D | 199 | 17.291 | 41.556 | -9.722 | 1.00 | 43.86 | GZ00 | N |
| ATOM | 3164 | CA | VAL | D | 199 | 17.600 | 42.869 | -9.177 | 1.00 | 43.86 | GZ00 | C |
| ATOM | 3165 | C | VAL | D | 199 | 18.389 | 43.626 | -10.233 | 1.00 | 44.40 | GZ00 | C |
| ATOM | 3166 | O | VAL | D | 199 | 19.480 | 43.197 | -10.619 | 1.00 | 41.88 | GZ00 | O |
| ATOM | 3167 | CB | VAL | D | 199 | 18.389 | 42.750 | -7.865 | 1.00 | 40.13 | GZ00 | C |
| ATOM | 3168 | CG1 | VAL | D | 199 | 18.560 | 44.088 | -7.241 | 1.00 | 33.53 | GZ00 | C |
| ATOM | 3169 | CG2 | VAL | D | 199 | 17.679 | 41.778 | -6.906 | 1.00 | 37.93 | GZ00 | C |
| ATOM | 3170 | N | THR | D | 200 | 17.838 | 44.732 | -10.719 | 1.00 | 43.84 | GZ00 | N |
| ATOM | 3171 | CA | THR | D | 200 | 18.513 | 45.558 | -11.712 | 1.00 | 42.52 | GZ00 | C |
| ATOM | 3172 | C | THR | D | 200 | 19.125 | 46.783 | -11.044 | 1.00 | 39.89 | GZ00 | C |
| ATOM | 3173 | O | THR | D | 200 | 18.442 | 47.506 | -10.310 | 1.00 | 39.87 | GZ00 | O |
| ATOM | 3174 | CB | THR | D | 200 | 17.542 | 45.987 | -12.813 | 1.00 | 48.84 | GZ00 | C |
| ATOM | 3175 | OG1 | THR | D | 200 | 16.958 | 44.805 | -13.380 | 1.00 | 46.64 | GZ00 | O |
| ATOM | 3176 | CG2 | THR | D | 200 | 18.290 | 46.806 | -13.918 | 1.00 | 33.12 | GZ00 | C |
| ATOM | 3177 | N | HIS | D | 201 | 20.406 | 47.016 | -11.313 | 1.00 | 40.27 | GZ00 | N |
| ATOM | 3178 | CA | HIS | D | 201 | 21.145 | 48.125 | -10.723 | 1.00 | 39.47 | GZ00 | C |
| ATOM | 3179 | C | HIS | D | 201 | 22.047 | 48.723 | -11.790 | 1.00 | 44.85 | GZ00 | C |
| ATOM | 3180 | O | HIS | D | 201 | 22.892 | 48.013 | -12.345 | 1.00 | 39.41 | GZ00 | O |
| ATOM | 3181 | CB | HIS | D | 201 | 21.973 | 47.660 | -9.532 | 1.00 | 37.22 | GZ00 | C |
| ATOM | 3182 | CG | HIS | D | 201 | 22.797 | 48.738 | -8.905 | 1.00 | 34.72 | GZ00 | C |
| ATOM | 3183 | ND1 | HIS | D | 201 | 24.134 | 48.915 | -9.190 | 1.00 | 33.61 | GZ00 | N |
| ATOM | 3184 | CD2 | HIS | D | 201 | 22.486 | 49.658 | -7.960 | 1.00 | 33.33 | GZ00 | C |
| ATOM | 3185 | CE1 | HIS | D | 201 | 24.608 | 49.907 | -8.455 | 1.00 | 40.99 | GZ00 | C |
| ATOM | 3186 | NE2 | HIS | D | 201 | 23.628 | 50.377 | -7.699 | 1.00 | 33.68 | GZ00 | N |
| ATOM | 3187 | N | GLU | D | 202 | 21.840 | 50.010 | -12.100 | 1.00 | 47.82 | GZ00 | N |
| ATOM | 3188 | CA | GLU | D | 202 | 22.672 | 50.726 | -13.064 | 1.00 | 45.90 | GZ00 | C |
| ATOM | 3189 | C | GLU | D | 202 | 22.753 | 49.963 | -14.385 | 1.00 | 44.77 | GZ00 | C |
| ATOM | 3190 | O | GLU | D | 202 | 23.828 | 49.757 | -14.947 | 1.00 | 45.87 | GZ00 | O |
| ATOM | 3191 | CB | GLU | D | 202 | 24.068 | 50.973 | -12.485 | 1.00 | 40.73 | GZ00 | C |
| ATOM | 3192 | CG | GLU | D | 202 | 24.078 | 51.833 | -11.241 | 1.00 | 37.51 | GZ00 | C |

| | | | | | | | | | | | |
|---|---|---|---|---|---|---|---|---|---|---|---|
| ATOM | 3193 | CD | GLU | D | 202 | 23.780 | 53.301 | -11.543 | 1.00 | 53.62 | GZ00 C |
| ATOM | 3194 | OE1 | GLU | D | 202 | 24.472 | 53.880 | -12.414 | 1.00 | 54.80 | GZ00 O |
| ATOM | 3195 | OE2 | GLU | D | 202 | 22.853 | 53.878 | -10.924 | 1.00 | 56.28 | GZ00 O1- |
| ATOM | 3196 | N | GLY | D | 203 | 21.600 | 49.498 | -14.862 | 1.00 | 40.18 | GZ00 N |
| ATOM | 3197 | CA | GLY | D | 203 | 21.584 | 48.775 | -16.117 | 1.00 | 41.95 | GZ00 C |
| ATOM | 3198 | C | GLY | D | 203 | 22.137 | 47.357 | -16.110 | 1.00 | 51.05 | GZ00 C |
| ATOM | 3199 | O | GLY | D | 203 | 22.259 | 46.760 | -17.186 | 1.00 | 50.18 | GZ00 O |
| ATOM | 3200 | N | SER | D | 206 | 22.505 | 46.793 | -14.958 | 1.00 | 47.41 | GZ00 N |
| ATOM | 3201 | CA | SER | D | 206 | 22.921 | 45.390 | -14.906 | 1.00 | 50.13 | GZ00 C |
| ATOM | 3202 | C | SER | D | 206 | 22.101 | 44.640 | -13.874 | 1.00 | 46.73 | GZ00 C |
| ATOM | 3203 | O | SER | D | 206 | 21.921 | 45.117 | -12.747 | 1.00 | 45.75 | GZ00 O |
| ATOM | 3204 | CB | SER | D | 206 | 24.404 | 45.243 | -14.607 | 1.00 | 46.12 | GZ00 C |
| ATOM | 3205 | OG | SER | D | 206 | 25.155 | 45.750 | -15.688 | 1.00 | 57.53 | GZ00 O |
| ATOM | 3206 | N | THR | D | 207 | 21.592 | 43.477 | -14.262 | 1.00 | 41.69 | GZ00 N |
| ATOM | 3207 | CA | THR | D | 207 | 20.697 | 42.720 | -13.400 | 1.00 | 50.12 | GZ00 C |
| ATOM | 3208 | C | THR | D | 207 | 21.425 | 41.525 | -12.797 | 1.00 | 47.45 | GZ00 C |
| ATOM | 3209 | O | THR | D | 207 | 22.129 | 40.793 | -13.501 | 1.00 | 43.02 | GZ00 O |
| ATOM | 3210 | CB | THR | D | 207 | 19.449 | 42.249 | -14.155 | 1.00 | 49.49 | GZ00 C |
| ATOM | 3211 | OG1 | THR | D | 207 | 19.797 | 41.130 | -14.968 | 1.00 | 59.30 | GZ00 O |
| ATOM | 3212 | CG2 | THR | D | 207 | 18.885 | 43.358 | -15.031 | 1.00 | 37.86 | GZ00 C |
| ATOM | 3213 | N | VAL | D | 208 | 21.234 | 41.328 | -11.495 | 1.00 | 45.06 | GZ00 N |
| ATOM | 3214 | CA | VAL | D | 208 | 21.724 | 40.163 | -10.767 | 1.00 | 49.03 | GZ00 C |
| ATOM | 3215 | C | VAL | D | 208 | 20.514 | 39.301 | -10.447 | 1.00 | 44.06 | GZ00 C |
| ATOM | 3216 | O | VAL | D | 208 | 19.493 | 39.815 | -9.974 | 1.00 | 50.95 | GZ00 O |
| ATOM | 3217 | CB | VAL | D | 208 | 22.467 | 40.579 | -9.484 | 1.00 | 48.97 | GZ00 C |
| ATOM | 3218 | CG1 | VAL | D | 208 | 22.858 | 39.372 | -8.668 | 1.00 | 41.12 | GZ00 C |
| ATOM | 3219 | CG2 | VAL | D | 208 | 23.700 | 41.376 | -9.829 | 1.00 | 39.61 | GZ00 C |
| ATOM | 3220 | N | GLU | D | 209 | 20.614 | 38.005 | -10.709 | 1.00 | 41.82 | GZ00 N |
| ATOM | 3221 | CA | GLU | D | 209 | 19.483 | 37.103 | -10.538 | 1.00 | 43.40 | GZ00 C |
| ATOM | 3222 | C | GLU | D | 209 | 19.835 | 35.970 | -9.586 | 1.00 | 50.61 | GZ00 C |
| ATOM | 3223 | O | GLU | D | 209 | 20.917 | 35.383 | -9.674 | 1.00 | 57.28 | GZ00 O |
| ATOM | 3224 | CB | GLU | D | 209 | 19.042 | 36.508 | -11.873 | 1.00 | 49.58 | GZ00 C |
| ATOM | 3225 | CG | GLU | D | 209 | 17.851 | 35.561 | -11.761 | 1.00 | 62.21 | GZ00 C |
| ATOM | 3226 | CD | GLU | D | 209 | 17.419 | 34.999 | -13.108 | 1.00 | 75.52 | GZ00 C |
| ATOM | 3227 | OE1 | GLU | D | 209 | 17.297 | 33.759 | -13.240 | 1.00 | 85.24 | GZ00 O |
| ATOM | 3228 | OE2 | GLU | D | 209 | 17.155 | 35.806 | -14.026 | 1.00 | 77.80 | GZ00 O1- |
| ATOM | 3229 | N | LYS | D | 210 | 18.902 | 35.636 | -8.705 | 1.00 | 48.46 | GZ00 N |
| ATOM | 3230 | CA | LYS | D | 210 | 19.006 | 34.443 | -7.885 | 1.00 | 52.58 | GZ00 C |
| ATOM | 3231 | C | LYS | D | 210 | 17.741 | 33.630 | -8.066 | 1.00 | 49.39 | GZ00 C |
| ATOM | 3232 | O | LYS | D | 210 | 16.639 | 34.182 | -8.150 | 1.00 | 51.83 | GZ00 O |
| ATOM | 3233 | CB | LYS | D | 210 | 19.228 | 34.785 | -6.401 | 1.00 | 50.40 | GZ00 C |
| ATOM | 3234 | CG | LYS | D | 210 | 20.608 | 35.341 | -6.116 | 1.00 | 46.93 | GZ00 C |
| ATOM | 3235 | CD | LYS | D | 210 | 21.675 | 34.454 | -6.726 | 1.00 | 45.41 | GZ00 C |
| ATOM | 3236 | CE | LYS | D | 210 | 23.095 | 35.008 | -6.476 | 1.00 | 52.18 | GZ00 C |
| ATOM | 3237 | NZ | LYS | D | 210 | 23.541 | 34.804 | -5.069 | 1.00 | 57.98 | GZ00 N1+ |
| ATOM | 3238 | N | THR | D | 211 | 17.900 | 32.324 | -8.168 | 1.00 | 47.49 | GZ00 N |
| ATOM | 3239 | CA | THR | D | 211 | 16.750 | 31.460 | -8.337 | 1.00 | 56.28 | GZ00 C |
| ATOM | 3240 | C | THR | D | 211 | 16.772 | 30.374 | -7.276 | 1.00 | 53.77 | GZ00 C |
| ATOM | 3241 | O | THR | D | 211 | 17.832 | 29.917 | -6.845 | 1.00 | 60.67 | GZ00 O |
| ATOM | 3242 | CB | THR | D | 211 | 16.708 | 30.815 | -9.722 | 1.00 | 53.88 | GZ00 C |
| ATOM | 3243 | OG1 | THR | D | 211 | 17.474 | 29.617 | -9.690 | 1.00 | 68.57 | GZ00 O |
| ATOM | 3244 | CG2 | THR | D | 211 | 17.315 | 31.729 | -10.774 | 1.00 | 54.97 | GZ00 C |
| ATOM | 3245 | N | VAL | D | 212 | 15.583 | 29.943 | -6.893 | 1.00 | 54.81 | GZ00 N |
| ATOM | 3246 | CA | VAL | D | 212 | 15.393 | 28.942 | -5.858 | 1.00 | 54.01 | GZ00 C |
| ATOM | 3247 | C | VAL | D | 212 | 14.264 | 28.011 | -6.313 | 1.00 | 62.68 | GZ00 C |
| ATOM | 3248 | O | VAL | D | 212 | 13.334 | 28.429 | -7.016 | 1.00 | 62.49 | GZ00 O |
| ATOM | 3249 | CB | VAL | D | 212 | 15.121 | 29.656 | -4.510 | 1.00 | 51.80 | GZ00 C |
| ATOM | 3250 | CG1 | VAL | D | 212 | 13.642 | 30.033 | -4.363 | 1.00 | 48.35 | GZ00 C |
| ATOM | 3251 | CG2 | VAL | D | 212 | 15.626 | 28.849 | -3.351 | 1.00 | 63.22 | GZ00 C |
| ATOM | 3252 | N | ALA | D | 213 | 14.367 | 26.729 | -5.951 | 1.00 | 62.74 | GZ00 N |
| ATOM | 3253 | CA | ALA | D | 213 | 13.422 | 25.713 | -6.406 | 1.00 | 61.72 | GZ00 C |
| ATOM | 3254 | C | ALA | D | 213 | 12.828 | 24.959 | -5.225 | 1.00 | 66.88 | GZ00 C |
| ATOM | 3255 | O | ALA | D | 213 | 13.502 | 24.746 | -4.213 | 1.00 | 69.81 | GZ00 O |
| ATOM | 3256 | CB | ALA | D | 213 | 14.074 | 24.712 | -7.370 | 1.00 | 52.61 | GZ00 C |
| ATOM | 3257 | N | PRO | D | 214 | 11.554 | 24.566 | -5.318 | 1.00 | 71.94 | GZ00 N |
| ATOM | 3258 | CA | PRO | D | 214 | 10.911 | 23.880 | -4.181 | 1.00 | 73.82 | GZ00 C |
| ATOM | 3259 | C | PRO | D | 214 | 11.485 | 22.508 | -3.872 | 1.00 | 83.15 | GZ00 C |
| ATOM | 3260 | O | PRO | D | 214 | 11.296 | 22.024 | -2.748 | 1.00 | 87.02 | GZ00 O |
| ATOM | 3261 | CB | PRO | D | 214 | 9.437 | 23.798 | -4.599 | 1.00 | 71.13 | GZ00 C |
| ATOM | 3262 | CG | PRO | D | 214 | 9.430 | 24.016 | -6.077 | 1.00 | 76.39 | GZ00 C |
| ATOM | 3263 | CD | PRO | D | 214 | 10.598 | 24.897 | -6.388 | 1.00 | 73.27 | GZ00 C |

```
ATOM   3264  N   THR D 215      12.167  21.863  -4.815  1.00 81.47      GZ00 N
ATOM   3265  CA  THR D 215      12.918  20.647  -4.512  1.00 88.36      GZ00 C
ATOM   3266  C   THR D 215      14.100  21.003  -3.608  1.00 95.90      GZ00 C
ATOM   3267  O   THR D 215      15.126  21.513  -4.071  1.00 92.30      GZ00 O
ATOM   3268  CB  THR D 215      13.383  19.978  -5.802  1.00 93.99      GZ00 C
ATOM   3269  OG1 THR D 215      14.373  20.797  -6.434  1.00 97.00      GZ00 O
ATOM   3270  CG2 THR D 215      12.210  19.791  -6.759  1.00 92.11      GZ00 C
ATOM   3271  N   GLU D 216      13.956  20.757  -2.306  1.00 96.37      GZ00 N
ATOM   3272  CA  GLU D 216      14.997  21.115  -1.340  1.00 94.49      GZ00 C
ATOM   3273  C   GLU D 216      15.586  19.863  -0.689  1.00103.70      GZ00 C
ATOM   3274  O   GLU D 216      16.586  19.934   0.031  1.00103.19      GZ00 O
ATOM   3275  CB  GLU D 216      14.453  22.060  -0.258  1.00 98.63      GZ00 C
ATOM   3276  CG  GLU D 216      14.051  23.474  -0.741  1.00 99.48      GZ00 C
ATOM   3277  CD  GLU D 216      13.551  24.375   0.397  1.00 94.82      GZ00 C
ATOM   3278  OE1 GLU D 216      14.356  24.704   1.304  1.00 90.53      GZ00 O
ATOM   3279  OE2 GLU D 216      12.349  24.740   0.394  1.00 84.44      GZ00 O1-
TER
ATOM   3280  N   GLN A   1     -34.534  69.246 -14.750  1.00 68.93           N
ATOM   3281  CA  GLN A   1     -35.181  70.408 -14.148  1.00 63.15           C
ATOM   3282  C   GLN A   1     -34.574  70.759 -12.783  1.00 64.87           C
ATOM   3283  O   GLN A   1     -34.907  70.176 -11.757  1.00 73.80           O
ATOM   3284  CB  GLN A   1     -36.678  70.168 -14.001  1.00 72.61           C
ATOM   3285  CG  GLN A   1     -37.438  71.350 -13.425  1.00 79.05           C
ATOM   3286  CD  GLN A   1     -38.607  70.901 -12.574  1.00 82.55           C
ATOM   3287  OE1 GLN A   1     -38.585  69.803 -12.013  1.00 76.25           O
ATOM   3288  NE2 GLN A   1     -39.638  71.743 -12.478  1.00 75.07           N
ATOM   3289  N   VAL A   2     -33.670  71.708 -12.792  1.00 52.55           N
ATOM   3290  CA  VAL A   2     -33.090  72.273 -11.587  1.00 39.46           C
ATOM   3291  C   VAL A   2     -33.760  73.615 -11.340  1.00 37.36           C
ATOM   3292  O   VAL A   2     -34.051  74.357 -12.286  1.00 42.98           O
ATOM   3293  CB  VAL A   2     -31.561  72.402 -11.744  1.00 37.95           C
ATOM   3294  CG1 VAL A   2     -30.942  73.173 -10.605  1.00 32.40           C
ATOM   3295  CG2 VAL A   2     -30.938  71.007 -11.812  1.00 38.29           C
ATOM   3296  N   GLN A   3     -34.074  73.905 -10.086  1.00 28.57           N
ATOM   3297  CA  GLN A   3     -34.530  75.234  -9.705  1.00 36.17           C
ATOM   3298  C   GLN A   3     -33.616  75.802  -8.623  1.00 32.07           C
ATOM   3299  O   GLN A   3     -33.291  75.112  -7.650  1.00 33.31           O
ATOM   3300  CB  GLN A   3     -35.981  75.198  -9.230  1.00 33.26           C
ATOM   3301  CG  GLN A   3     -36.980  75.166 -10.396  1.00 48.28           C
ATOM   3302  CD  GLN A   3     -38.422  75.482  -9.975  1.00 66.43           C
ATOM   3303  OE1 GLN A   3     -38.748  75.535  -8.779  1.00 59.16           O
ATOM   3304  NE2 GLN A   3     -39.284  75.714 -10.964  1.00 65.91           N
ATOM   3305  N   LEU A   4     -33.202  77.051  -8.798  1.00 28.83           N
ATOM   3306  CA  LEU A   4     -32.404  77.779  -7.823  1.00 34.07           C
ATOM   3307  C   LEU A   4     -33.199  79.004  -7.385  1.00 31.68           C
ATOM   3308  O   LEU A   4     -33.759  79.699  -8.231  1.00 35.93           O
ATOM   3309  CB  LEU A   4     -31.054  78.181  -8.431  1.00 31.35           C
ATOM   3310  CG  LEU A   4     -30.271  77.022  -9.054  1.00 35.66           C
ATOM   3311  CD1 LEU A   4     -29.061  77.538  -9.848  1.00 33.22           C
ATOM   3312  CD2 LEU A   4     -29.820  76.024  -7.986  1.00 28.58           C
ATOM   3313  N   VAL A   5     -33.296  79.236  -6.074  1.00 31.24           N
ATOM   3314  CA  VAL A   5     -34.075  80.340  -5.501  1.00 29.71           C
ATOM   3315  C   VAL A   5     -33.192  81.106  -4.521  1.00 30.40           C
ATOM   3316  O   VAL A   5     -32.883  80.594  -3.439  1.00 34.07           O
ATOM   3317  CB  VAL A   5     -35.352  79.849  -4.790  1.00 33.16           C
ATOM   3318  CG1 VAL A   5     -36.116  81.023  -4.194  1.00 22.32           C
ATOM   3319  CG2 VAL A   5     -36.244  79.088  -5.746  1.00 27.68           C
ATOM   3320  N   GLU A   6     -32.830  82.344  -4.866  1.00 27.45           N
ATOM   3321  CA  GLU A   6     -32.039  83.183  -3.970  1.00 34.31           C
ATOM   3322  C   GLU A   6     -32.938  83.895  -2.964  1.00 35.66           C
ATOM   3323  O   GLU A   6     -34.094  84.211  -3.250  1.00 35.15           O
ATOM   3324  CB  GLU A   6     -31.242  84.251  -4.738  1.00 35.33           C
ATOM   3325  CG  GLU A   6     -30.370  83.745  -5.877  1.00 36.08           C
ATOM   3326  CD  GLU A   6     -31.115  83.636  -7.209  1.00 39.10           C
ATOM   3327  OE1 GLU A   6     -32.369  83.508  -7.199  1.00 35.08           O
ATOM   3328  OE2 GLU A   6     -30.432  83.628  -8.263  1.00 35.04           O1-
ATOM   3329  N   SER A   7     -32.368  84.204  -1.800  1.00 36.13           N
ATOM   3330  CA  SER A   7     -33.075  84.945  -0.763  1.00 34.15           C
ATOM   3331  C   SER A   7     -32.033  85.588   0.132  1.00 31.67           C
ATOM   3332  O   SER A   7     -30.848  85.263   0.062  1.00 34.14           O
ATOM   3333  CB  SER A   7     -34.004  84.045   0.060  1.00 29.39           C
```

| | | | | | | | | | | | |
|---|---|---|---|---|---|---|---|---|---|---|---|
| ATOM | 3334 | OG | SER | A | 7 | -33.246 | 83.029 | 0.704 | 1.00 | 39.57 | O |
| ATOM | 3335 | N | GLY | A | 8 | -32.484 | 86.520 | 0.962 | 1.00 | 33.72 | N |
| ATOM | 3336 | CA | GLY | A | 8 | -31.621 | 87.148 | 1.934 | 1.00 | 31.16 | C |
| ATOM | 3337 | C | GLY | A | 8 | -31.165 | 88.543 | 1.590 | 1.00 | 40.14 | C |
| ATOM | 3338 | O | GLY | A | 8 | -30.323 | 89.095 | 2.311 | 1.00 | 41.88 | O |
| ATOM | 3339 | N | GLY | A | 9 | -31.679 | 89.131 | 0.518 | 1.00 | 30.46 | N |
| ATOM | 3340 | CA | GLY | A | 9 | -31.281 | 90.478 | 0.150 | 1.00 | 42.56 | C |
| ATOM | 3341 | C | GLY | A | 9 | -31.699 | 91.554 | 1.150 | 1.00 | 45.12 | C |
| ATOM | 3342 | O | GLY | A | 9 | -31.989 | 91.303 | 2.319 | 1.00 | 45.51 | O |
| ATOM | 3343 | N | GLY | A | 10 | -31.614 | 92.794 | 0.695 | 1.00 | 40.16 | N |
| ATOM | 3344 | CA | GLY | A | 10 | -32.173 | 93.880 | 1.461 | 1.00 | 36.55 | C |
| ATOM | 3345 | C | GLY | A | 10 | -31.303 | 95.118 | 1.379 | 1.00 | 41.88 | C |
| ATOM | 3346 | O | GLY | A | 10 | -30.334 | 95.184 | 0.617 | 1.00 | 34.74 | O |
| ATOM | 3347 | N | VAL | A | 11 | -31.676 | 96.112 | 2.184 | 1.00 | 37.50 | N |
| ATOM | 3348 | CA | VAL | A | 11 | -30.953 | 97.369 | 2.303 | 1.00 | 38.58 | C |
| ATOM | 3349 | C | VAL | A | 11 | -30.020 | 97.243 | 3.493 | 1.00 | 43.54 | C |
| ATOM | 3350 | O | VAL | A | 11 | -30.423 | 96.759 | 4.555 | 1.00 | 47.55 | O |
| ATOM | 3351 | CB | VAL | A | 11 | -31.911 | 98.559 | 2.483 | 1.00 | 48.63 | C |
| ATOM | 3352 | CG1 | VAL | A | 11 | -31.118 | 99.850 | 2.585 | 1.00 | 43.07 | C |
| ATOM | 3353 | CG2 | VAL | A | 11 | -32.901 | 98.626 | 1.323 | 1.00 | 45.11 | C |
| ATOM | 3354 | N | VAL | A | 12 | -28.773 | 97.676 | 3.324 | 1.00 | 36.28 | N |
| ATOM | 3355 | CA | VAL | A | 12 | -27.767 | 97.511 | 4.364 | 1.00 | 44.38 | C |
| ATOM | 3356 | C | VAL | A | 12 | -26.802 | 98.696 | 4.298 | 1.00 | 42.35 | C |
| ATOM | 3357 | O | VAL | A | 12 | -26.638 | 99.336 | 3.253 | 1.00 | 42.56 | O |
| ATOM | 3358 | CB | VAL | A | 12 | -27.051 | 96.138 | 4.207 | 1.00 | 47.04 | C |
| ATOM | 3359 | CG1 | VAL | A | 12 | -26.272 | 96.069 | 2.897 | 1.00 | 44.35 | C |
| ATOM | 3360 | CG2 | VAL | A | 12 | -26.125 | 95.864 | 5.359 | 1.00 | 49.20 | C |
| ATOM | 3361 | N | GLN | A | 13 | -26.197 | 99.009 | 5.427 | 1.00 | 43.55 | N |
| ATOM | 3362 | CA | GLN | A | 13 | -25.312 | 100.156 | 5.405 | 1.00 | 44.17 | C |
| ATOM | 3363 | C | GLN | A | 13 | -23.884 | 99.743 | 5.086 | 1.00 | 47.16 | C |
| ATOM | 3364 | O | GLN | A | 13 | -23.471 | 98.619 | 5.399 | 1.00 | 45.14 | O |
| ATOM | 3365 | CB | GLN | A | 13 | -25.334 | 100.881 | 6.734 | 1.00 | 45.16 | C |
| ATOM | 3366 | CG | GLN | A | 13 | -26.698 | 101.357 | 7.135 | 1.00 | 59.94 | C |
| ATOM | 3367 | CD | GLN | A | 13 | -26.607 | 102.516 | 8.099 | 1.00 | 69.00 | C |
| ATOM | 3368 | OE1 | GLN | A | 13 | -26.037 | 103.566 | 7.771 | 1.00 | 69.47 | O |
| ATOM | 3369 | NE2 | GLN | A | 13 | -27.151 | 102.335 | 9.297 | 1.00 | 70.89 | N |
| ATOM | 3370 | N | PRO | A | 14 | -23.132 | 100.657 | 4.472 | 1.00 | 41.71 | N |
| ATOM | 3371 | CA | PRO | A | 14 | -21.726 | 100.372 | 4.167 | 1.00 | 38.64 | C |
| ATOM | 3372 | C | PRO | A | 14 | -21.001 | 99.888 | 5.411 | 1.00 | 41.46 | C |
| ATOM | 3373 | O | PRO | A | 14 | -21.225 | 100.390 | 6.512 | 1.00 | 43.96 | O |
| ATOM | 3374 | CB | PRO | A | 14 | -21.191 | 101.725 | 3.683 | 1.00 | 36.30 | C |
| ATOM | 3375 | CG | PRO | A | 14 | -22.417 | 102.449 | 3.166 | 1.00 | 37.34 | C |
| ATOM | 3376 | CD | PRO | A | 14 | -23.553 | 101.995 | 4.015 | 1.00 | 42.17 | C |
| ATOM | 3377 | N | GLY | A | 15 | -20.150 | 98.877 | 5.237 | 1.00 | 39.52 | N |
| ATOM | 3378 | CA | GLY | A | 15 | -19.389 | 98.318 | 6.335 | 1.00 | 35.94 | C |
| ATOM | 3379 | C | GLY | A | 15 | -20.077 | 97.199 | 7.088 | 1.00 | 40.72 | C |
| ATOM | 3380 | O | GLY | A | 15 | -19.407 | 96.463 | 7.826 | 1.00 | 44.51 | O |
| ATOM | 3381 | N | ARG | A | 16 | -21.388 | 97.045 | 6.924 | 1.00 | 39.84 | N |
| ATOM | 3382 | CA | ARG | A | 16 | -22.114 | 95.953 | 7.556 | 1.00 | 53.52 | C |
| ATOM | 3383 | C | ARG | A | 16 | -21.962 | 94.660 | 6.746 | 1.00 | 46.84 | C |
| ATOM | 3384 | O | ARG | A | 16 | -21.259 | 94.600 | 5.727 | 1.00 | 39.84 | O |
| ATOM | 3385 | CB | ARG | A | 16 | -23.592 | 96.303 | 7.706 | 1.00 | 54.09 | C |
| ATOM | 3386 | CG | ARG | A | 16 | -23.880 | 97.531 | 8.533 | 1.00 | 61.92 | C |
| ATOM | 3387 | CD | ARG | A | 16 | -23.430 | 97.323 | 9.960 | 1.00 | 69.75 | C |
| ATOM | 3388 | NE | ARG | A | 16 | -24.436 | 97.796 | 10.906 | 1.00 | 97.69 | N |
| ATOM | 3389 | CZ | ARG | A | 16 | -24.245 | 97.901 | 12.218 | 1.00 | 105.26 | C |
| ATOM | 3390 | NH1 | ARG | A | 16 | -23.075 | 97.566 | 12.754 | 1.00 | 97.15 | N1+ |
| ATOM | 3391 | NH2 | ARG | A | 16 | -25.226 | 98.346 | 12.995 | 1.00 | 108.93 | N |
| ATOM | 3392 | N | SER | A | 17 | -22.664 | 93.625 | 7.206 | 1.00 | 37.36 | N |
| ATOM | 3393 | CA | SER | A | 17 | -22.586 | 92.271 | 6.692 | 1.00 | 37.19 | C |
| ATOM | 3394 | C | SER | A | 17 | -23.962 | 91.796 | 6.274 | 1.00 | 41.75 | C |
| ATOM | 3395 | O | SER | A | 17 | -24.980 | 92.251 | 6.795 | 1.00 | 39.73 | O |
| ATOM | 3396 | CB | SER | A | 17 | -22.044 | 91.275 | 7.738 | 1.00 | 42.19 | C |
| ATOM | 3397 | OG | SER | A | 17 | -20.664 | 91.495 | 7.994 | 1.00 | 55.67 | O |
| ATOM | 3398 | N | LEU | A | 18 | -23.976 | 90.833 | 5.361 | 1.00 | 40.66 | N |
| ATOM | 3399 | CA | LEU | A | 18 | -25.220 | 90.256 | 4.877 | 1.00 | 42.98 | C |
| ATOM | 3400 | C | LEU | A | 18 | -24.901 | 88.861 | 4.361 | 1.00 | 40.59 | C |
| ATOM | 3401 | O | LEU | A | 18 | -23.804 | 88.623 | 3.850 | 1.00 | 41.78 | O |
| ATOM | 3402 | CB | LEU | A | 18 | -25.806 | 91.135 | 3.773 | 1.00 | 44.15 | C |
| ATOM | 3403 | CG | LEU | A | 18 | -27.278 | 91.269 | 3.460 | 1.00 | 54.36 | C |
| ATOM | 3404 | CD1 | LEU | A | 18 | -28.038 | 91.644 | 4.712 | 1.00 | 49.30 | C |

| | | | | | | | | | | | |
|---|---|---|---|---|---|---|---|---|---|---|---|
| ATOM | 3405 | CD2 | LEU | A | 18 | -27.392 | 92.395 | 2.454 | 1.00 | 49.78 | C |
| ATOM | 3406 | N | ARG | A | 19 | -25.848 | 87.940 | 4.497 | 1.00 | 30.82 | N |
| ATOM | 3407 | CA | ARG | A | 19 | -25.670 | 86.595 | 3.965 | 1.00 | 34.82 | C |
| ATOM | 3408 | C | ARG | A | 19 | -26.783 | 86.270 | 2.981 | 1.00 | 34.54 | C |
| ATOM | 3409 | O | ARG | A | 19 | -27.959 | 86.364 | 3.329 | 1.00 | 38.10 | O |
| ATOM | 3410 | CB | ARG | A | 19 | -25.635 | 85.549 | 5.073 | 1.00 | 35.74 | C |
| ATOM | 3411 | CG | ARG | A | 19 | -25.012 | 84.268 | 4.606 | 1.00 | 37.23 | C |
| ATOM | 3412 | CD | ARG | A | 19 | -25.223 | 83.128 | 5.579 | 1.00 | 38.05 | C |
| ATOM | 3413 | NE | ARG | A | 19 | -26.633 | 82.826 | 5.730 | 1.00 | 39.69 | N |
| ATOM | 3414 | CZ | ARG | A | 19 | -27.107 | 81.838 | 6.477 | 1.00 | 47.15 | C |
| ATOM | 3415 | NH1 | ARG | A | 19 | -28.419 | 81.642 | 6.559 | 1.00 | 43.39 | N1+ |
| ATOM | 3416 | NH2 | ARG | A | 19 | -26.271 | 81.043 | 7.130 | 1.00 | 46.94 | N |
| ATOM | 3417 | N | LEU | A | 20 | -26.416 | 85.921 | 1.750 | 1.00 | 33.95 | N |
| ATOM | 3418 | CA | LEU | A | 20 | -27.386 | 85.431 | 0.783 | 1.00 | 32.04 | C |
| ATOM | 3419 | C | LEU | A | 20 | -27.486 | 83.908 | 0.841 | 1.00 | 35.48 | C |
| ATOM | 3420 | O | LEU | A | 20 | -26.522 | 83.206 | 1.174 | 1.00 | 34.13 | O |
| ATOM | 3421 | CB | LEU | A | 20 | -27.019 | 85.873 | -0.631 | 1.00 | 26.84 | C |
| ATOM | 3422 | CG | LEU | A | 20 | -26.829 | 87.380 | -0.792 | 1.00 | 31.60 | C |
| ATOM | 3423 | CD1 | LEU | A | 20 | -26.621 | 87.704 | -2.261 | 1.00 | 25.55 | C |
| ATOM | 3424 | CD2 | LEU | A | 20 | -28.028 | 88.140 | -0.220 | 1.00 | 24.15 | C |
| ATOM | 3425 | N | SER | A | 21 | -28.675 | 83.403 | 0.528 | 1.00 | 33.17 | N |
| ATOM | 3426 | CA | SER | A | 21 | -28.924 | 81.976 | 0.391 | 1.00 | 32.39 | C |
| ATOM | 3427 | C | SER | A | 21 | -29.441 | 81.658 | -1.002 | 1.00 | 34.28 | C |
| ATOM | 3428 | O | SER | A | 21 | -30.123 | 82.470 | -1.633 | 1.00 | 35.15 | O |
| ATOM | 3429 | CB | SER | A | 21 | -29.944 | 81.457 | 1.411 | 1.00 | 29.91 | C |
| ATOM | 3430 | OG | SER | A | 21 | -29.401 | 81.456 | 2.706 | 1.00 | 32.59 | O |
| ATOM | 3431 | N | CYS | A | 22 | -29.122 | 80.452 | -1.463 | 1.00 | 29.55 | N |
| ATOM | 3432 | CA | CYS | A | 22 | -29.608 | 79.954 | -2.740 | 1.00 | 29.82 | C |
| ATOM | 3433 | C | CYS | A | 22 | -30.059 | 78.520 | -2.506 | 1.00 | 34.08 | C |
| ATOM | 3434 | O | CYS | A | 22 | -29.225 | 77.635 | -2.286 | 1.00 | 33.80 | O |
| ATOM | 3435 | CB | CYS | A | 22 | -28.515 | 80.040 | -3.803 | 1.00 | 37.60 | C |
| ATOM | 3436 | SG | CYS | A | 22 | -28.846 | 79.175 | -5.362 | 1.00 | 44.81 | S |
| ATOM | 3437 | N | ALA | A | 23 | -31.366 | 78.297 | -2.544 | 1.00 | 27.47 | N |
| ATOM | 3438 | CA | ALA | A | 23 | -31.956 | 77.006 | -2.235 | 1.00 | 31.62 | C |
| ATOM | 3439 | C | ALA | A | 23 | -32.152 | 76.263 | -3.540 | 1.00 | 32.14 | C |
| ATOM | 3440 | O | ALA | A | 23 | -32.753 | 76.799 | -4.480 | 1.00 | 32.98 | O |
| ATOM | 3441 | CB | ALA | A | 23 | -33.296 | 77.155 | -1.500 | 1.00 | 23.36 | C |
| ATOM | 3442 | N | ALA | A | 24 | -31.646 | 75.037 | -3.593 | 1.00 | 31.79 | N |
| ATOM | 3443 | CA | ALA | A | 24 | -31.642 | 74.239 | -4.809 | 1.00 | 29.92 | C |
| ATOM | 3444 | C | ALA | A | 24 | -32.595 | 73.062 | -4.685 | 1.00 | 29.71 | C |
| ATOM | 3445 | O | ALA | A | 24 | -32.735 | 72.476 | -3.612 | 1.00 | 39.67 | O |
| ATOM | 3446 | CB | ALA | A | 24 | -30.238 | 73.724 | -5.104 | 1.00 | 27.23 | C |
| ATOM | 3447 | N | SER | A | 25 | -33.239 | 72.708 | -5.790 | 1.00 | 32.42 | N |
| ATOM | 3448 | CA | SER | A | 25 | -34.067 | 71.515 | -5.851 | 1.00 | 28.53 | C |
| ATOM | 3449 | C | SER | A | 25 | -34.108 | 70.982 | -7.273 | 1.00 | 34.80 | C |
| ATOM | 3450 | O | SER | A | 25 | -33.754 | 71.688 | -8.228 | 1.00 | 28.65 | O |
| ATOM | 3451 | CB | SER | A | 25 | -35.484 | 71.822 | -5.400 | 1.00 | 36.68 | C |
| ATOM | 3452 | OG | SER | A | 25 | -36.046 | 72.798 | -6.269 | 1.00 | 34.63 | O |
| ATOM | 3453 | N | GLY | A | 26 | -34.558 | 69.720 | -7.410 | 1.00 | 31.26 | N |
| ATOM | 3454 | CA | GLY | A | 26 | -34.824 | 69.150 | -8.718 | 1.00 | 31.69 | C |
| ATOM | 3455 | C | GLY | A | 26 | -33.759 | 68.254 | -9.323 | 1.00 | 50.48 | C |
| ATOM | 3456 | O | GLY | A | 26 | -33.944 | 67.786 | -10.460 | 1.00 | 59.29 | O |
| ATOM | 3457 | N | PHE | A | 27 | -32.666 | 68.001 | -8.613 | 1.00 | 34.76 | N |
| ATOM | 3458 | CA | PHE | A | 27 | -31.536 | 67.198 | -9.082 | 1.00 | 36.25 | C |
| ATOM | 3459 | C | PHE | A | 27 | -30.495 | 67.347 | -8.004 | 1.00 | 35.91 | C |
| ATOM | 3460 | O | PHE | A | 27 | -30.163 | 68.473 | -7.622 | 1.00 | 41.91 | O |
| ATOM | 3461 | CB | PHE | A | 27 | -30.986 | 67.647 | -10.455 | 1.00 | 34.34 | C |
| ATOM | 3462 | CG | PHE | A | 27 | -29.671 | 66.974 | -10.868 | 1.00 | 35.77 | C |
| ATOM | 3463 | CD1 | PHE | A | 27 | -29.467 | 65.601 | -10.706 | 1.00 | 38.27 | C |
| ATOM | 3464 | CD2 | PHE | A | 27 | -28.645 | 67.728 | -11.455 | 1.00 | 39.96 | C |
| ATOM | 3465 | CE1 | PHE | A | 27 | -28.246 | 64.992 | -11.075 | 1.00 | 42.34 | C |
| ATOM | 3466 | CE2 | PHE | A | 27 | -27.422 | 67.132 | -11.848 | 1.00 | 39.02 | C |
| ATOM | 3467 | CZ | PHE | A | 27 | -27.221 | 65.758 | -11.651 | 1.00 | 37.64 | C |
| ATOM | 3468 | N | THR | A | 28 | -29.960 | 66.224 | -7.555 | 1.00 | 34.00 | N |
| ATOM | 3469 | CA | THR | A | 28 | -29.086 | 66.145 | -6.398 | 1.00 | 32.16 | C |
| ATOM | 3470 | C | THR | A | 28 | -28.160 | 67.347 | -6.297 | 1.00 | 32.73 | C |
| ATOM | 3471 | O | THR | A | 28 | -27.324 | 67.570 | -7.174 | 1.00 | 34.74 | O |
| ATOM | 3472 | CB | THR | A | 28 | -28.286 | 64.858 | -6.521 | 1.00 | 39.57 | C |
| ATOM | 3473 | OG1 | THR | A | 28 | -29.211 | 63.801 | -6.780 | 1.00 | 48.76 | O |
| ATOM | 3474 | CG2 | THR | A | 28 | -27.527 | 64.570 | -5.250 | 1.00 | 39.97 | C |
| ATOM | 3475 | N | PHE | A | 29 | -28.340 | 68.137 | -5.232 | 1.00 | 37.84 | N |

| | | | | | | | | | | | | |
|---|---|---|---|---|---|---|---|---|---|---|---|---|
| ATOM | 3476 | CA | PHE | A | 29 | -27.545 | 69.348 | -5.012 | 1.00 | 34.13 | | C |
| ATOM | 3477 | C | PHE | A | 29 | -26.050 | 69.063 | -5.057 | 1.00 | 35.71 | | C |
| ATOM | 3478 | O | PHE | A | 29 | -25.277 | 69.821 | -5.666 | 1.00 | 31.39 | | O |
| ATOM | 3479 | CB | PHE | A | 29 | -27.924 | 69.954 | -3.661 | 1.00 | 33.04 | | C |
| ATOM | 3480 | CG | PHE | A | 29 | -27.217 | 71.251 | -3.309 | 1.00 | 29.10 | | C |
| ATOM | 3481 | CD1 | PHE | A | 29 | -27.347 | 72.386 | -4.112 | 1.00 | 28.49 | | C |
| ATOM | 3482 | CD2 | PHE | A | 29 | -26.505 | 71.364 | -2.117 | 1.00 | 30.52 | | C |
| ATOM | 3483 | CE1 | PHE | A | 29 | -26.748 | 73.607 | -3.754 | 1.00 | 29.07 | | C |
| ATOM | 3484 | CE2 | PHE | A | 29 | -25.893 | 72.592 | -1.755 | 1.00 | 33.69 | | C |
| ATOM | 3485 | CZ | PHE | A | 29 | -26.023 | 73.707 | -2.578 | 1.00 | 27.96 | | C |
| ATOM | 3486 | N | SER | A | 30 | -25.628 | 67.963 | -4.434 | 1.00 | 33.43 | | N |
| ATOM | 3487 | CA | SER | A | 30 | -24.208 | 67.634 | -4.348 | 1.00 | 38.52 | | C |
| ATOM | 3488 | C | SER | A | 30 | -23.621 | 67.208 | -5.682 | 1.00 | 33.12 | | C |
| ATOM | 3489 | O | SER | A | 30 | -22.422 | 66.936 | -5.741 | 1.00 | 35.08 | | O |
| ATOM | 3490 | CB | SER | A | 30 | -23.988 | 66.534 | -3.303 | 1.00 | 28.69 | | C |
| ATOM | 3491 | OG | SER | A | 30 | -24.728 | 65.385 | -3.669 | 1.00 | 38.89 | | O |
| ATOM | 3492 | N | SER | A | 31 | -24.419 | 67.147 | -6.740 | 1.00 | 27.16 | | N |
| ATOM | 3493 | CA | SER | A | 31 | -23.905 | 66.778 | -8.045 | 1.00 | 38.98 | | C |
| ATOM | 3494 | C | SER | A | 31 | -23.443 | 67.962 | -8.896 | 1.00 | 39.11 | | C |
| ATOM | 3495 | O | SER | A | 31 | -23.036 | 67.740 | -10.050 | 1.00 | 33.54 | | O |
| ATOM | 3496 | CB | SER | A | 31 | -24.944 | 65.966 | -8.806 | 1.00 | 31.50 | | C |
| ATOM | 3497 | OG | SER | A | 31 | -25.034 | 64.689 | -8.205 | 1.00 | 43.55 | | O |
| ATOM | 3498 | N | TYR | A | 32 | -23.469 | 69.194 | -8.377 | 1.00 | 30.49 | | N |
| ATOM | 3499 | CA | TYR | A | 32 | -22.942 | 70.307 | -9.164 | 1.00 | 28.71 | | C |
| ATOM | 3500 | C | TYR | A | 32 | -22.356 | 71.382 | -8.268 | 1.00 | 29.76 | | C |
| ATOM | 3501 | O | TYR | A | 32 | -22.712 | 71.503 | -7.092 | 1.00 | 29.23 | | O |
| ATOM | 3502 | CB | TYR | A | 32 | -24.002 | 70.910 | -10.078 | 1.00 | 34.80 | | C |
| ATOM | 3503 | CG | TYR | A | 32 | -25.331 | 71.155 | -9.416 | 1.00 | 32.18 | | C |
| ATOM | 3504 | CD1 | TYR | A | 32 | -25.549 | 72.299 | -8.660 | 1.00 | 33.21 | | C |
| ATOM | 3505 | CD2 | TYR | A | 32 | -26.364 | 70.259 | -9.562 | 1.00 | 32.73 | | C |
| ATOM | 3506 | CE1 | TYR | A | 32 | -26.770 | 72.536 | -8.050 | 1.00 | 37.14 | | C |
| ATOM | 3507 | CE2 | TYR | A | 32 | -27.589 | 70.484 | -8.953 | 1.00 | 36.08 | | C |
| ATOM | 3508 | CZ | TYR | A | 32 | -27.783 | 71.621 | -8.202 | 1.00 | 34.54 | | C |
| ATOM | 3509 | OH | TYR | A | 32 | -28.995 | 71.854 | -7.605 | 1.00 | 42.41 | | O |
| ATOM | 3510 | N | GLY | A | 33 | -21.391 | 72.118 | -8.828 | 1.00 | 29.06 | | N |
| ATOM | 3511 | CA | GLY | A | 33 | -20.917 | 73.336 | -8.213 | 1.00 | 26.22 | | C |
| ATOM | 3512 | C | GLY | A | 33 | -21.823 | 74.508 | -8.562 | 1.00 | 32.33 | | C |
| ATOM | 3513 | O | GLY | A | 33 | -22.749 | 74.389 | -9.373 | 1.00 | 32.28 | | O |
| ATOM | 3514 | N | LEU | A | 34 | -21.549 | 75.660 | -7.944 | 1.00 | 28.11 | | N |
| ATOM | 3515 | CA | LEU | A | 34 | -22.397 | 76.825 | -8.149 | 1.00 | 28.06 | | C |
| ATOM | 3516 | C | LEU | A | 34 | -21.579 | 78.107 | -8.137 | 1.00 | 32.81 | | C |
| ATOM | 3517 | O | LEU | A | 34 | -20.482 | 78.185 | -7.562 | 1.00 | 30.26 | | O |
| ATOM | 3518 | CB | LEU | A | 34 | -23.503 | 76.934 | -7.083 | 1.00 | 25.69 | | C |
| ATOM | 3519 | CG | LEU | A | 34 | -24.496 | 75.772 | -7.075 | 1.00 | 29.24 | | C |
| ATOM | 3520 | CD1 | LEU | A | 34 | -24.122 | 74.810 | -5.948 | 1.00 | 25.51 | | C |
| ATOM | 3521 | CD2 | LEU | A | 34 | -25.937 | 76.248 | -6.951 | 1.00 | 28.69 | | C |
| ATOM | 3522 | N | HIS | A | 35 | -22.174 | 79.124 | -8.757 | 1.00 | 25.76 | | N |
| ATOM | 3523 | CA | HIS | A | 35 | -21.645 | 80.469 | -8.877 | 1.00 | 28.30 | | C |
| ATOM | 3524 | C | HIS | A | 35 | -22.559 | 81.459 | -8.167 | 1.00 | 28.45 | | C |
| ATOM | 3525 | O | HIS | A | 35 | -23.771 | 81.255 | -8.047 | 1.00 | 26.56 | | O |
| ATOM | 3526 | CB | HIS | A | 35 | -21.583 | 80.946 | -10.334 | 1.00 | 27.75 | | C |
| ATOM | 3527 | CG | HIS | A | 35 | -20.599 | 80.236 | -11.204 | 1.00 | 34.48 | | C |
| ATOM | 3528 | ND1 | HIS | A | 35 | -19.305 | 80.689 | -11.377 | 1.00 | 31.96 | | N |
| ATOM | 3529 | CD2 | HIS | A | 35 | -20.742 | 79.174 | -12.034 | 1.00 | 27.73 | | C |
| ATOM | 3530 | CE1 | HIS | A | 35 | -18.693 | 79.927 | -12.263 | 1.00 | 30.71 | | C |
| ATOM | 3531 | NE2 | HIS | A | 35 | -19.537 | 78.989 | -12.664 | 1.00 | 32.82 | | N |
| ATOM | 3532 | N | TRP | A | 36 | -21.971 | 82.583 | -7.793 | 1.00 | 24.52 | | N |
| ATOM | 3533 | CA | TRP | A | 36 | -22.702 | 83.815 | -7.578 | 1.00 | 24.53 | | C |
| ATOM | 3534 | C | TRP | A | 36 | -22.276 | 84.804 | -8.656 | 1.00 | 30.64 | | C |
| ATOM | 3535 | O | TRP | A | 36 | -21.078 | 84.979 | -8.895 | 1.00 | 31.45 | | O |
| ATOM | 3536 | CB | TRP | A | 36 | -22.431 | 84.389 | -6.184 | 1.00 | 25.38 | | C |
| ATOM | 3537 | CG | TRP | A | 36 | -23.102 | 83.636 | -5.061 | 1.00 | 34.21 | | C |
| ATOM | 3538 | CD1 | TRP | A | 36 | -22.528 | 82.700 | -4.225 | 1.00 | 27.01 | | C |
| ATOM | 3539 | CD2 | TRP | A | 36 | -24.483 | 83.734 | -4.662 | 1.00 | 29.26 | | C |
| ATOM | 3540 | NE1 | TRP | A | 36 | -23.464 | 82.236 | -3.328 | 1.00 | 29.61 | | N |
| ATOM | 3541 | CE2 | TRP | A | 36 | -24.669 | 82.850 | -3.574 | 1.00 | 29.15 | | C |
| ATOM | 3542 | CE3 | TRP | A | 36 | -25.569 | 84.500 | -5.104 | 1.00 | 31.70 | | C |
| ATOM | 3543 | CZ2 | TRP | A | 36 | -25.902 | 82.707 | -2.925 | 1.00 | 29.62 | | C |
| ATOM | 3544 | CZ3 | TRP | A | 36 | -26.795 | 84.353 | -4.464 | 1.00 | 31.58 | | C |
| ATOM | 3545 | CH2 | TRP | A | 36 | -26.950 | 83.447 | -3.390 | 1.00 | 32.02 | | C |
| ATOM | 3546 | N | VAL | A | 37 | -23.255 | 85.430 | -9.315 | 1.00 | 26.17 | | N |

| | | | | | | | | | | | |
|---|---|---|---|---|---|---|---|---|---|---|---|
| ATOM | 3547 | CA | VAL | A | 37 | -23.052 | 86.487 | -10.305 | 1.00 | 26.93 | C |
| ATOM | 3548 | C | VAL | A | 37 | -23.911 | 87.683 | -9.909 | 1.00 | 31.18 | C |
| ATOM | 3549 | O | VAL | A | 37 | -24.979 | 87.514 | -9.311 | 1.00 | 32.57 | O |
| ATOM | 3550 | CB | VAL | A | 37 | -23.436 | 86.003 | -11.725 | 1.00 | 30.07 | C |
| ATOM | 3551 | CG1 | VAL | A | 37 | -23.336 | 87.141 | -12.741 | 1.00 | 25.17 | C |
| ATOM | 3552 | CG2 | VAL | A | 37 | -22.577 | 84.797 | -12.141 | 1.00 | 27.92 | C |
| ATOM | 3553 | N | ARG | A | 38 | -23.451 | 88.900 | -10.217 | 1.00 | 26.83 | N |
| ATOM | 3554 | CA | ARG | A | 38 | -24.222 | 90.086 | -9.838 | 1.00 | 28.59 | C |
| ATOM | 3555 | C | ARG | A | 38 | -24.327 | 91.074 | -10.987 | 1.00 | 32.00 | C |
| ATOM | 3556 | O | ARG | A | 38 | -23.546 | 91.042 | -11.942 | 1.00 | 34.12 | O |
| ATOM | 3557 | CB | ARG | A | 38 | -23.649 | 90.790 | -8.616 | 1.00 | 24.52 | C |
| ATOM | 3558 | CG | ARG | A | 38 | -22.405 | 91.581 | -8.863 | 1.00 | 27.87 | C |
| ATOM | 3559 | CD | ARG | A | 38 | -21.832 | 92.016 | -7.532 | 1.00 | 25.42 | C |
| ATOM | 3560 | NE | ARG | A | 38 | -20.569 | 92.691 | -7.719 | 1.00 | 26.21 | N |
| ATOM | 3561 | CZ | ARG | A | 38 | -19.825 | 93.186 | -6.735 | 1.00 | 33.88 | C |
| ATOM | 3562 | NH1 | ARG | A | 38 | -20.207 | 93.058 | -5.470 | 1.00 | 31.46 | N1+ |
| ATOM | 3563 | NH2 | ARG | A | 38 | -18.700 | 93.822 | -7.026 | 1.00 | 27.28 | N |
| ATOM | 3564 | N | GLN | A | 39 | -25.317 | 91.961 | -10.874 | 1.00 | 31.14 | N |
| ATOM | 3565 | CA | GLN | A | 39 | -25.594 | 92.942 | -11.923 | 1.00 | 26.67 | C |
| ATOM | 3566 | C | GLN | A | 39 | -26.067 | 94.237 | -11.278 | 1.00 | 33.37 | C |
| ATOM | 3567 | O | GLN | A | 39 | -27.144 | 94.282 | -10.657 | 1.00 | 30.44 | O |
| ATOM | 3568 | CB | GLN | A | 39 | -26.635 | 92.419 | -12.901 | 1.00 | 29.71 | C |
| ATOM | 3569 | CG | GLN | A | 39 | -26.977 | 93.391 | -14.031 | 1.00 | 30.87 | C |
| ATOM | 3570 | CD | GLN | A | 39 | -27.797 | 92.710 | -15.108 | 1.00 | 35.35 | C |
| ATOM | 3571 | OE1 | GLN | A | 39 | -28.796 | 92.048 | -14.812 | 1.00 | 37.94 | O |
| ATOM | 3572 | NE2 | GLN | A | 39 | -27.370 | 92.841 | -16.358 | 1.00 | 26.92 | N |
| ATOM | 3573 | N | ALA | A | 40 | -25.269 | 95.279 | -11.425 | 1.00 | 28.05 | N |
| ATOM | 3574 | CA | ALA | A | 40 | -25.664 | 96.588 | -10.926 | 1.00 | 34.65 | C |
| ATOM | 3575 | C | ALA | A | 40 | -26.709 | 97.199 | -11.859 | 1.00 | 34.83 | C |
| ATOM | 3576 | O | ALA | A | 40 | -26.757 | 96.861 | -13.039 | 1.00 | 32.60 | O |
| ATOM | 3577 | CB | ALA | A | 40 | -24.443 | 97.499 | -10.812 | 1.00 | 29.23 | C |
| ATOM | 3578 | N | PRO | A | 41 | -27.556 | 98.095 | -11.352 | 1.00 | 41.70 | N |
| ATOM | 3579 | CA | PRO | A | 41 | -28.654 | 98.645 | -12.175 | 1.00 | 37.62 | C |
| ATOM | 3580 | C | PRO | A | 41 | -28.143 | 99.327 | -13.440 | 1.00 | 38.87 | C |
| ATOM | 3581 | O | PRO | A | 41 | -27.259 | 100.188 | -13.392 | 1.00 | 37.58 | O |
| ATOM | 3582 | CB | PRO | A | 41 | -29.330 | 99.656 | -11.236 | 1.00 | 34.86 | C |
| ATOM | 3583 | CG | PRO | A | 41 | -28.936 | 99.229 | -9.859 | 1.00 | 40.20 | C |
| ATOM | 3584 | CD | PRO | A | 41 | -27.557 | 98.641 | -9.983 | 1.00 | 35.94 | C |
| ATOM | 3585 | N | GLY | A | 42 | -28.698 | 98.918 | -14.578 | 1.00 | 36.04 | N |
| ATOM | 3586 | CA | GLY | A | 42 | -28.295 | 99.408 | -15.887 | 1.00 | 32.92 | C |
| ATOM | 3587 | C | GLY | A | 42 | -26.969 | 98.887 | -16.429 | 1.00 | 42.89 | C |
| ATOM | 3588 | O | GLY | A | 42 | -26.492 | 99.395 | -17.446 | 1.00 | 48.01 | O |
| ATOM | 3589 | N | LYS | A | 43 | -26.358 | 97.882 | -15.817 | 1.00 | 37.13 | N |
| ATOM | 3590 | CA | LYS | A | 43 | -25.003 | 97.473 | -16.196 | 1.00 | 39.84 | C |
| ATOM | 3591 | C | LYS | A | 43 | -24.961 | 95.989 | -16.548 | 1.00 | 36.76 | C |
| ATOM | 3592 | O | LYS | A | 43 | -25.989 | 95.301 | -16.591 | 1.00 | 33.65 | O |
| ATOM | 3593 | CB | LYS | A | 43 | -24.004 | 97.816 | -15.094 | 1.00 | 35.22 | C |
| ATOM | 3594 | CG | LYS | A | 43 | -23.960 | 99.326 | -14.833 | 1.00 | 42.47 | C |
| ATOM | 3595 | CD | LYS | A | 43 | -22.804 | 99.688 | -13.941 | 1.00 | 57.71 | C |
| ATOM | 3596 | CE | LYS | A | 43 | -22.589 | 101.201 | -13.864 | 1.00 | 72.06 | C |
| ATOM | 3597 | NZ | LYS | A | 43 | -21.339 | 101.531 | -13.103 | 1.00 | 70.64 | N1+ |
| ATOM | 3598 | N | GLY | A | 44 | -23.743 | 95.520 | -16.834 | 1.00 | 31.12 | N |
| ATOM | 3599 | CA | GLY | A | 44 | -23.510 | 94.165 | -17.290 | 1.00 | 28.92 | C |
| ATOM | 3600 | C | GLY | A | 44 | -23.333 | 93.158 | -16.166 | 1.00 | 33.92 | C |
| ATOM | 3601 | O | GLY | A | 44 | -23.295 | 93.484 | -14.979 | 1.00 | 35.65 | O |
| ATOM | 3602 | N | LEU | A | 45 | -23.202 | 91.898 | -16.569 | 1.00 | 31.47 | N |
| ATOM | 3603 | CA | LEU | A | 45 | -22.959 | 90.839 | -15.606 | 1.00 | 26.60 | C |
| ATOM | 3604 | C | LEU | A | 45 | -21.542 | 90.959 | -15.072 | 1.00 | 31.15 | C |
| ATOM | 3605 | O | LEU | A | 45 | -20.601 | 91.246 | -15.820 | 1.00 | 31.96 | O |
| ATOM | 3606 | CB | LEU | A | 45 | -23.173 | 89.462 | -16.256 | 1.00 | 28.04 | C |
| ATOM | 3607 | CG | LEU | A | 45 | -24.575 | 89.294 | -16.881 | 1.00 | 33.18 | C |
| ATOM | 3608 | CD1 | LEU | A | 45 | -24.804 | 87.921 | -17.544 | 1.00 | 29.82 | C |
| ATOM | 3609 | CD2 | LEU | A | 45 | -25.639 | 89.585 | -15.859 | 1.00 | 27.89 | C |
| ATOM | 3610 | N | GLU | A | 46 | -21.397 | 90.735 | -13.767 | 1.00 | 30.38 | N |
| ATOM | 3611 | CA | GLU | A | 46 | -20.101 | 90.660 | -13.112 | 1.00 | 31.96 | C |
| ATOM | 3612 | C | GLU | A | 46 | -20.049 | 89.376 | -12.306 | 1.00 | 28.71 | C |
| ATOM | 3613 | O | GLU | A | 46 | -20.899 | 89.156 | -11.440 | 1.00 | 28.54 | O |
| ATOM | 3614 | CB | GLU | A | 46 | -19.854 | 91.859 | -12.196 | 1.00 | 30.82 | C |
| ATOM | 3615 | CG | GLU | A | 46 | -18.411 | 91.886 | -11.708 | 1.00 | 35.43 | C |
| ATOM | 3616 | CD | GLU | A | 46 | -18.145 | 92.863 | -10.551 | 1.00 | 42.19 | C |
| ATOM | 3617 | OE1 | GLU | A | 46 | -19.076 | 93.544 | -10.038 | 1.00 | 34.18 | O |

| | | | | | | | | | | | |
|---|---|---|---|---|---|---|---|---|---|---|---|
| ATOM | 3618 | OE2 | GLU | A | 46 | -16.968 | 92.932 | -10.154 | 1.00 | 46.04 | O1- |
| ATOM | 3619 | N | TRP | A | 47 | -19.052 | 88.537 | -12.584 | 1.00 | 28.91 | N |
| ATOM | 3620 | CA | TRP | A | 47 | -18.888 | 87.300 | -11.834 | 1.00 | 28.05 | C |
| ATOM | 3621 | C | TRP | A | 47 | -18.433 | 87.610 | -10.414 | 1.00 | 28.38 | C |
| ATOM | 3622 | O | TRP | A | 47 | -17.652 | 88.534 | -10.199 | 1.00 | 29.03 | O |
| ATOM | 3623 | CB | TRP | A | 47 | -17.882 | 86.396 | -12.547 | 1.00 | 28.79 | C |
| ATOM | 3624 | CG | TRP | A | 47 | -17.570 | 85.150 | -11.808 | 1.00 | 27.80 | C |
| ATOM | 3625 | CD1 | TRP | A | 47 | -18.396 | 84.090 | -11.618 | 1.00 | 26.75 | C |
| ATOM | 3626 | CD2 | TRP | A | 47 | -16.330 | 84.810 | -11.186 | 1.00 | 29.28 | C |
| ATOM | 3627 | NE1 | TRP | A | 47 | -17.759 | 83.119 | -10.900 | 1.00 | 30.04 | N |
| ATOM | 3628 | CE2 | TRP | A | 47 | -16.487 | 83.529 | -10.623 | 1.00 | 26.53 | C |
| ATOM | 3629 | CE3 | TRP | A | 47 | -15.093 | 85.455 | -11.067 | 1.00 | 30.37 | C |
| ATOM | 3630 | CZ2 | TRP | A | 47 | -15.454 | 82.870 | -9.945 | 1.00 | 30.41 | C |
| ATOM | 3631 | CZ3 | TRP | A | 47 | -14.058 | 84.797 | -10.376 | 1.00 | 27.15 | C |
| ATOM | 3632 | CH2 | TRP | A | 47 | -14.251 | 83.527 | -9.832 | 1.00 | 30.81 | C |
| ATOM | 3633 | N | VAL | A | 48 | -18.923 | 86.836 | -9.441 | 1.00 | 26.85 | N |
| ATOM | 3634 | CA | VAL | A | 48 | -18.590 | 87.031 | -8.026 | 1.00 | 26.22 | C |
| ATOM | 3635 | C | VAL | A | 48 | -17.740 | 85.890 | -7.475 | 1.00 | 27.91 | C |
| ATOM | 3636 | O | VAL | A | 48 | -16.641 | 86.118 | -6.962 | 1.00 | 31.20 | O |
| ATOM | 3637 | CB | VAL | A | 48 | -19.858 | 87.245 | -7.171 | 1.00 | 29.89 | C |
| ATOM | 3638 | CG1 | VAL | A | 48 | -19.465 | 87.415 | -5.709 | 1.00 | 27.30 | C |
| ATOM | 3639 | CG2 | VAL | A | 48 | -20.640 | 88.465 | -7.680 | 1.00 | 27.25 | C |
| ATOM | 3640 | N | ALA | A | 49 | -18.234 | 84.656 | -7.552 | 1.00 | 27.92 | N |
| ATOM | 3641 | CA | ALA | A | 49 | -17.498 | 83.540 | -6.964 | 1.00 | 27.21 | C |
| ATOM | 3642 | C | ALA | A | 49 | -18.028 | 82.240 | -7.528 | 1.00 | 29.25 | C |
| ATOM | 3643 | O | ALA | A | 49 | -19.155 | 82.178 | -8.025 | 1.00 | 29.04 | O |
| ATOM | 3644 | CB | ALA | A | 49 | -17.590 | 83.500 | -5.429 | 1.00 | 22.18 | C |
| ATOM | 3645 | N | VAL | A | 50 | -17.187 | 81.190 | -7.435 | 1.00 | 25.65 | N |
| ATOM | 3646 | CA | VAL | A | 50 | -17.592 | 79.835 | -7.777 | 1.00 | 25.46 | C |
| ATOM | 3647 | C | VAL | A | 50 | -17.129 | 78.894 | -6.678 | 1.00 | 28.11 | C |
| ATOM | 3648 | O | VAL | A | 50 | -16.137 | 79.144 | -5.988 | 1.00 | 30.11 | O |
| ATOM | 3649 | CB | VAL | A | 50 | -17.068 | 79.380 | -9.162 | 1.00 | 31.87 | C |
| ATOM | 3650 | CG1 | VAL | A | 50 | -15.561 | 79.238 | -9.175 | 1.00 | 28.23 | C |
| ATOM | 3651 | CG2 | VAL | A | 50 | -17.767 | 78.084 | -9.606 | 1.00 | 28.82 | C |
| ATOM | 3652 | N | ILE | A | 51 | -17.884 | 77.821 | -6.484 | 1.00 | 29.93 | N |
| ATOM | 3653 | CA | ILE | A | 51 | -17.514 | 76.799 | -5.522 | 1.00 | 25.88 | C |
| ATOM | 3654 | C | ILE | A | 51 | -17.657 | 75.447 | -6.203 | 1.00 | 26.86 | C |
| ATOM | 3655 | O | ILE | A | 51 | -18.486 | 75.262 | -7.100 | 1.00 | 26.48 | O |
| ATOM | 3656 | CB | ILE | A | 51 | -18.354 | 76.879 | -4.227 | 1.00 | 28.38 | C |
| ATOM | 3657 | CG1 | ILE | A | 51 | -17.776 | 75.932 | -3.163 | 1.00 | 29.68 | C |
| ATOM | 3658 | CG2 | ILE | A | 51 | -19.831 | 76.565 | -4.498 | 1.00 | 23.38 | C |
| ATOM | 3659 | CD1 | ILE | A | 51 | -18.344 | 76.180 | -1.775 | 1.00 | 28.59 | C |
| ATOM | 3660 | N | TRP | A | 52 | -16.821 | 74.510 | -5.793 | 1.00 | 24.08 | N |
| ATOM | 3661 | CA | TRP | A | 52 | -16.856 | 73.188 | -6.392 | 1.00 | 28.60 | C |
| ATOM | 3662 | C | TRP | A | 52 | -18.074 | 72.397 | -5.899 | 1.00 | 30.88 | C |
| ATOM | 3663 | O | TRP | A | 52 | -18.688 | 72.722 | -4.879 | 1.00 | 26.43 | O |
| ATOM | 3664 | CB | TRP | A | 52 | -15.559 | 72.452 | -6.066 | 1.00 | 30.05 | C |
| ATOM | 3665 | CG | TRP | A | 52 | -14.821 | 71.992 | -7.256 | 1.00 | 31.59 | C |
| ATOM | 3666 | CD1 | TRP | A | 52 | -14.551 | 70.701 | -7.601 | 1.00 | 34.80 | C |
| ATOM | 3667 | CD2 | TRP | A | 52 | -14.265 | 72.811 | -8.293 | 1.00 | 30.63 | C |
| ATOM | 3668 | NE1 | TRP | A | 52 | -13.842 | 70.664 | -8.781 | 1.00 | 33.66 | N |
| ATOM | 3669 | CE2 | TRP | A | 52 | -13.652 | 71.942 | -9.228 | 1.00 | 31.14 | C |
| ATOM | 3670 | CE3 | TRP | A | 52 | -14.220 | 74.183 | -8.522 | 1.00 | 28.98 | C |
| ATOM | 3671 | CZ2 | TRP | A | 52 | -12.999 | 72.404 | -10.368 | 1.00 | 30.12 | C |
| ATOM | 3672 | CZ3 | TRP | A | 52 | -13.564 | 74.646 | -9.663 | 1.00 | 37.05 | C |
| ATOM | 3673 | CH2 | TRP | A | 52 | -12.953 | 73.756 | -10.562 | 1.00 | 36.23 | C |
| ATOM | 3674 | N | TYR | A | 53 | -18.401 | 71.330 | -6.635 | 1.00 | 28.62 | N |
| ATOM | 3675 | CA | TYR | A | 53 | -19.539 | 70.484 | -6.281 | 1.00 | 31.01 | C |
| ATOM | 3676 | C | TYR | A | 53 | -19.373 | 69.846 | -4.909 | 1.00 | 36.12 | C |
| ATOM | 3677 | O | TYR | A | 53 | -20.370 | 69.557 | -4.240 | 1.00 | 36.43 | O |
| ATOM | 3678 | CB | TYR | A | 53 | -19.743 | 69.393 | -7.329 | 1.00 | 31.97 | C |
| ATOM | 3679 | CG | TYR | A | 53 | -18.458 | 68.713 | -7.742 | 1.00 | 35.13 | C |
| ATOM | 3680 | CD1 | TYR | A | 53 | -17.918 | 67.672 | -6.989 | 1.00 | 37.05 | C |
| ATOM | 3681 | CD2 | TYR | A | 53 | -17.791 | 69.104 | -8.896 | 1.00 | 37.33 | C |
| ATOM | 3682 | CE1 | TYR | A | 53 | -16.755 | 67.058 | -7.366 | 1.00 | 36.11 | C |
| ATOM | 3683 | CE2 | TYR | A | 53 | -16.614 | 68.492 | -9.286 | 1.00 | 40.57 | C |
| ATOM | 3684 | CZ | TYR | A | 53 | -16.095 | 67.479 | -8.521 | 1.00 | 40.64 | C |
| ATOM | 3685 | OH | TYR | A | 53 | -14.916 | 66.886 | -8.923 | 1.00 | 51.83 | O |
| ATOM | 3686 | N | ASP | A | 54 | -18.136 | 69.613 | -4.468 | 1.00 | 31.86 | N |
| ATOM | 3687 | CA | ASP | A | 54 | -17.896 | 69.044 | -3.150 | 1.00 | 32.47 | C |
| ATOM | 3688 | C | ASP | A | 54 | -17.433 | 70.090 | -2.141 | 1.00 | 32.55 | C |

| | | | | | | | | | | | |
|---|---|---|---|---|---|---|---|---|---|---|---|
| ATOM | 3689 | O | ASP | A | 54 | -16.894 | 69.732 | -1.097 | 1.00 | 34.26 | O |
| ATOM | 3690 | CB | ASP | A | 54 | -16.882 | 67.901 | -3.245 | 1.00 | 33.69 | C |
| ATOM | 3691 | CG | ASP | A | 54 | -15.550 | 68.344 | -3.878 | 1.00 | 41.63 | C |
| ATOM | 3692 | OD1 | ASP | A | 54 | -15.332 | 69.574 | -4.029 | 1.00 | 35.39 | O |
| ATOM | 3693 | OD2 | ASP | A | 54 | -14.721 | 67.462 | -4.224 | 1.00 | 41.66 | O1- |
| ATOM | 3694 | N | GLY | A | 55 | -17.633 | 71.376 | -2.428 | 1.00 | 35.34 | N |
| ATOM | 3695 | CA | GLY | A | 55 | -17.197 | 72.416 | -1.516 | 1.00 | 28.60 | C |
| ATOM | 3696 | C | GLY | A | 55 | -15.696 | 72.626 | -1.397 | 1.00 | 30.85 | C |
| ATOM | 3697 | O | GLY | A | 55 | -15.261 | 73.340 | -0.483 | 1.00 | 31.85 | O |
| ATOM | 3698 | N | SER | A | 56 | -14.880 | 72.030 | -2.271 | 1.00 | 31.41 | N |
| ATOM | 3699 | CA | SER | A | 56 | -13.427 | 72.159 | -2.138 | 1.00 | 28.55 | C |
| ATOM | 3700 | C | SER | A | 56 | -12.921 | 73.456 | -2.773 | 1.00 | 29.96 | C |
| ATOM | 3701 | O | SER | A | 56 | -12.790 | 74.463 | -2.071 | 1.00 | 35.87 | O |
| ATOM | 3702 | CB | SER | A | 56 | -12.716 | 70.922 | -2.715 | 1.00 | 29.73 | C |
| ATOM | 3703 | OG | SER | A | 56 | -13.055 | 70.688 | -4.069 | 1.00 | 33.14 | O |
| ATOM | 3704 | N | ASN | A | 57 | -12.648 | 73.468 | -4.080 | 1.00 | 27.89 | N |
| ATOM | 3705 | CA | ASN | A | 57 | -12.094 | 74.669 | -4.708 | 1.00 | 30.78 | C |
| ATOM | 3706 | C | ASN | A | 57 | -13.090 | 75.836 | -4.663 | 1.00 | 34.96 | C |
| ATOM | 3707 | O | ASN | A | 57 | -14.296 | 75.655 | -4.883 | 1.00 | 29.93 | O |
| ATOM | 3708 | CB | ASN | A | 57 | -11.712 | 74.392 | -6.167 | 1.00 | 28.41 | C |
| ATOM | 3709 | CG | ASN | A | 57 | -10.430 | 73.551 | -6.316 | 1.00 | 36.02 | C |
| ATOM | 3710 | OD1 | ASN | A | 57 | -10.034 | 72.789 | -5.427 | 1.00 | 31.33 | O |
| ATOM | 3711 | ND2 | ASN | A | 57 | -9.779 | 73.703 | -7.462 | 1.00 | 33.91 | N |
| ATOM | 3712 | N | LYS | A | 58 | -12.562 | 77.042 | -4.418 | 1.00 | 30.71 | N |
| ATOM | 3713 | CA | LYS | A | 58 | -13.307 | 78.299 | -4.384 | 1.00 | 31.44 | C |
| ATOM | 3714 | C | LYS | A | 58 | -12.504 | 79.356 | -5.117 | 1.00 | 31.50 | C |
| ATOM | 3715 | O | LYS | A | 58 | -11.306 | 79.491 | -4.858 | 1.00 | 35.00 | O |
| ATOM | 3716 | CB | LYS | A | 58 | -13.541 | 78.796 | -2.953 | 1.00 | 28.26 | C |
| ATOM | 3717 | CG | LYS | A | 58 | -14.308 | 77.853 | -2.071 | 1.00 | 32.31 | C |
| ATOM | 3718 | CD | LYS | A | 58 | -14.359 | 78.350 | -0.623 | 1.00 | 29.79 | C |
| ATOM | 3719 | CE | LYS | A | 58 | -15.146 | 77.337 | 0.247 | 1.00 | 31.84 | C |
| ATOM | 3720 | NZ | LYS | A | 58 | -15.624 | 77.954 | 1.523 | 1.00 | 28.78 | N1+ |
| ATOM | 3721 | N | TYR | A | 59 | -13.143 | 80.105 | -6.025 | 1.00 | 31.81 | N |
| ATOM | 3722 | CA | TYR | A | 59 | -12.489 | 81.224 | -6.704 | 1.00 | 29.74 | C |
| ATOM | 3723 | C | TYR | A | 59 | -13.349 | 82.463 | -6.543 | 1.00 | 31.86 | C |
| ATOM | 3724 | O | TYR | A | 59 | -14.581 | 82.379 | -6.491 | 1.00 | 36.21 | O |
| ATOM | 3725 | CB | TYR | A | 59 | -12.258 | 80.990 | -8.212 | 1.00 | 35.36 | C |
| ATOM | 3726 | CG | TYR | A | 59 | -11.620 | 79.662 | -8.593 | 1.00 | 35.36 | C |
| ATOM | 3727 | CD1 | TYR | A | 59 | -10.873 | 78.936 | -7.693 | 1.00 | 49.05 | C |
| ATOM | 3728 | CD2 | TYR | A | 59 | -11.776 | 79.141 | -9.849 | 1.00 | 46.01 | C |
| ATOM | 3729 | CE1 | TYR | A | 59 | -10.317 | 77.708 | -8.033 | 1.00 | 56.82 | C |
| ATOM | 3730 | CE2 | TYR | A | 59 | -11.212 | 77.925 | -10.198 | 1.00 | 50.42 | C |
| ATOM | 3731 | CZ | TYR | A | 59 | -10.494 | 77.215 | -9.292 | 1.00 | 45.11 | C |
| ATOM | 3732 | OH | TYR | A | 59 | -9.945 | 76.014 | -9.656 | 1.00 | 44.41 | O |
| ATOM | 3733 | N | TYR | A | 60 | -12.706 | 83.617 | -6.491 | 1.00 | 28.31 | N |
| ATOM | 3734 | CA | TYR | A | 60 | -13.418 | 84.868 | -6.277 | 1.00 | 31.56 | C |
| ATOM | 3735 | C | TYR | A | 60 | -12.950 | 85.928 | -7.263 | 1.00 | 33.13 | C |
| ATOM | 3736 | O | TYR | A | 60 | -11.782 | 85.963 | -7.657 | 1.00 | 36.90 | O |
| ATOM | 3737 | CB | TYR | A | 60 | -13.221 | 85.407 | -4.884 | 1.00 | 26.95 | C |
| ATOM | 3738 | CG | TYR | A | 60 | -13.706 | 84.522 | -3.780 | 1.00 | 31.53 | C |
| ATOM | 3739 | CD1 | TYR | A | 60 | -12.877 | 83.520 | -3.249 | 1.00 | 34.43 | C |
| ATOM | 3740 | CD2 | TYR | A | 60 | -14.958 | 84.703 | -3.225 | 1.00 | 29.79 | C |
| ATOM | 3741 | CE1 | TYR | A | 60 | -13.308 | 82.707 | -2.215 | 1.00 | 28.05 | C |
| ATOM | 3742 | CE2 | TYR | A | 60 | -15.400 | 83.896 | -2.183 | 1.00 | 32.79 | C |
| ATOM | 3743 | CZ | TYR | A | 60 | -14.567 | 82.900 | -1.679 | 1.00 | 33.02 | C |
| ATOM | 3744 | OH | TYR | A | 60 | -14.995 | 82.098 | -0.641 | 1.00 | 31.57 | O |
| ATOM | 3745 | N | ALA | A | 61 | -13.872 | 86.804 | -7.650 | 1.00 | 23.00 | N |
| ATOM | 3746 | CA | ALA | A | 61 | -13.464 | 87.976 | -8.406 | 1.00 | 28.65 | C |
| ATOM | 3747 | C | ALA | A | 61 | -12.612 | 88.888 | -7.526 | 1.00 | 32.06 | C |
| ATOM | 3748 | O | ALA | A | 61 | -12.759 | 88.923 | -6.301 | 1.00 | 30.34 | O |
| ATOM | 3749 | CB | ALA | A | 61 | -14.679 | 88.736 | -8.937 | 1.00 | 25.89 | C |
| ATOM | 3750 | N | ASP | A | 62 | -11.713 | 89.633 | -8.171 | 1.00 | 32.65 | N |
| ATOM | 3751 | CA | ASP | A | 62 | -10.805 | 90.522 | -7.445 | 1.00 | 32.02 | C |
| ATOM | 3752 | C | ASP | A | 62 | -11.551 | 91.559 | -6.605 | 1.00 | 31.30 | C |
| ATOM | 3753 | O | ASP | A | 62 | -11.125 | 91.882 | -5.492 | 1.00 | 33.95 | O |
| ATOM | 3754 | CB | ASP | A | 62 | -9.856 | 91.208 | -8.428 | 1.00 | 30.42 | C |
| ATOM | 3755 | CG | ASP | A | 62 | -8.574 | 90.404 | -8.671 | 1.00 | 42.21 | C |
| ATOM | 3756 | OD1 | ASP | A | 62 | -8.521 | 89.196 | -8.308 | 1.00 | 50.07 | O |
| ATOM | 3757 | OD2 | ASP | A | 62 | -7.625 | 90.972 | -9.257 | 1.00 | 56.07 | O1- |
| ATOM | 3758 | N | SER | A | 63 | -12.672 | 92.082 | -7.106 | 1.00 | 28.74 | N |
| ATOM | 3759 | CA | SER | A | 63 | -13.409 | 93.118 | -6.383 | 1.00 | 32.42 | C |

| | | | | | | | | | | | |
|---|---|---|---|---|---|---|---|---|---|---|---|
| ATOM | 3760 | C | SER | A | 63 | -13.980 | 92.646 | -5.048 | 1.00 | 39.97 | C |
| ATOM | 3761 | O | SER | A | 63 | -14.490 | 93.477 | -4.280 | 1.00 | 38.00 | O |
| ATOM | 3762 | CB | SER | A | 63 | -14.542 | 93.641 | -7.263 | 1.00 | 35.16 | C |
| ATOM | 3763 | OG | SER | A | 63 | -15.314 | 92.561 | -7.747 | 1.00 | 42.20 | O |
| ATOM | 3764 | N | VAL | A | 64 | -13.865 | 91.356 | -4.740 | 1.00 | 33.69 | N |
| ATOM | 3765 | CA | VAL | A | 64 | -14.600 | 90.717 | -3.663 | 1.00 | 31.03 | C |
| ATOM | 3766 | C | VAL | A | 64 | -13.681 | 89.860 | -2.780 | 1.00 | 35.18 | C |
| ATOM | 3767 | O | VAL | A | 64 | -14.040 | 89.490 | -1.651 | 1.00 | 33.73 | O |
| ATOM | 3768 | CB | VAL | A | 64 | -15.740 | 89.918 | -4.330 | 1.00 | 34.61 | C |
| ATOM | 3769 | CG1 | VAL | A | 64 | -15.732 | 88.417 | -4.001 | 1.00 | 22.27 | C |
| ATOM | 3770 | CG2 | VAL | A | 64 | -17.031 | 90.609 | -4.115 | 1.00 | 28.14 | C |
| ATOM | 3771 | N | LYS | A | 65 | -12.476 | 89.566 | -3.273 | 1.00 | 30.69 | N |
| ATOM | 3772 | CA | LYS | A | 65 | -11.498 | 88.785 | -2.511 | 1.00 | 32.30 | C |
| ATOM | 3773 | C | LYS | A | 65 | -11.300 | 89.377 | -1.126 | 1.00 | 35.92 | C |
| ATOM | 3774 | O | LYS | A | 65 | -11.119 | 90.590 | -0.975 | 1.00 | 37.34 | O |
| ATOM | 3775 | CB | LYS | A | 65 | -10.155 | 88.769 | -3.242 | 1.00 | 31.37 | C |
| ATOM | 3776 | CG | LYS | A | 65 | -10.056 | 87.772 | -4.365 | 1.00 | 35.93 | C |
| ATOM | 3777 | CD | LYS | A | 65 | -8.637 | 87.667 | -4.881 | 1.00 | 39.14 | C |
| ATOM | 3778 | CE | LYS | A | 65 | -8.443 | 86.411 | -5.742 | 1.00 | 40.69 | C |
| ATOM | 3779 | NZ | LYS | A | 65 | -9.097 | 86.500 | -7.077 | 1.00 | 44.27 | N1+ |
| ATOM | 3780 | N | GLY | A | 66 | -11.300 | 88.509 | -0.118 | 1.00 | 36.76 | N |
| ATOM | 3781 | CA | GLY | A | 66 | -11.109 | 88.926 | 1.249 | 1.00 | 34.77 | C |
| ATOM | 3782 | C | GLY | A | 66 | -12.355 | 89.409 | 1.957 | 1.00 | 37.43 | C |
| ATOM | 3783 | O | GLY | A | 66 | -12.361 | 89.462 | 3.185 | 1.00 | 43.41 | O |
| ATOM | 3784 | N | ARG | A | 67 | -13.407 | 89.779 | 1.231 | 1.00 | 37.34 | N |
| ATOM | 3785 | CA | ARG | A | 67 | -14.630 | 90.281 | 1.849 | 1.00 | 34.16 | C |
| ATOM | 3786 | C | ARG | A | 67 | -15.789 | 89.302 | 1.783 | 1.00 | 32.20 | C |
| ATOM | 3787 | O | ARG | A | 67 | -16.554 | 89.208 | 2.745 | 1.00 | 35.10 | O |
| ATOM | 3788 | CB | ARG | A | 67 | -15.047 | 91.609 | 1.199 | 1.00 | 28.81 | C |
| ATOM | 3789 | CG | ARG | A | 67 | -14.047 | 92.745 | 1.481 | 1.00 | 38.29 | C |
| ATOM | 3790 | CD | ARG | A | 67 | -14.504 | 94.103 | 0.942 | 1.00 | 39.40 | C |
| ATOM | 3791 | NE | ARG | A | 67 | -14.857 | 94.018 | -0.470 | 1.00 | 34.94 | N |
| ATOM | 3792 | CZ | ARG | A | 67 | -16.086 | 94.209 | -0.939 | 1.00 | 36.27 | C |
| ATOM | 3793 | NH1 | ARG | A | 67 | -17.074 | 94.532 | -0.113 | 1.00 | 29.46 | N1+ |
| ATOM | 3794 | NH2 | ARG | A | 67 | -16.325 | 94.083 | -2.236 | 1.00 | 33.40 | N |
| ATOM | 3795 | N | PHE | A | 68 | -15.936 | 88.558 | 0.688 | 1.00 | 31.92 | N |
| ATOM | 3796 | CA | PHE | A | 68 | -17.046 | 87.626 | 0.523 | 1.00 | 33.41 | C |
| ATOM | 3797 | C | PHE | A | 68 | -16.540 | 86.204 | 0.697 | 1.00 | 34.08 | C |
| ATOM | 3798 | O | PHE | A | 68 | -15.382 | 85.903 | 0.405 | 1.00 | 35.01 | O |
| ATOM | 3799 | CB | PHE | A | 68 | -17.727 | 87.743 | -0.850 | 1.00 | 29.41 | C |
| ATOM | 3800 | CG | PHE | A | 68 | -18.410 | 89.076 | -1.108 | 1.00 | 31.18 | C |
| ATOM | 3801 | CD1 | PHE | A | 68 | -18.242 | 90.156 | -0.256 | 1.00 | 28.93 | C |
| ATOM | 3802 | CD2 | PHE | A | 68 | -19.236 | 89.231 | -2.206 | 1.00 | 29.68 | C |
| ATOM | 3803 | CE1 | PHE | A | 68 | -18.851 | 91.388 | -0.518 | 1.00 | 33.26 | C |
| ATOM | 3804 | CE2 | PHE | A | 68 | -19.865 | 90.444 | -2.462 | 1.00 | 36.39 | C |
| ATOM | 3805 | CZ | PHE | A | 68 | -19.660 | 91.532 | -1.618 | 1.00 | 35.74 | C |
| ATOM | 3806 | N | THR | A | 69 | -17.413 | 85.325 | 1.177 | 1.00 | 33.37 | N |
| ATOM | 3807 | CA | THR | A | 69 | -17.054 | 83.917 | 1.293 | 1.00 | 32.05 | C |
| ATOM | 3808 | C | THR | A | 69 | -18.206 | 83.085 | 0.775 | 1.00 | 30.76 | C |
| ATOM | 3809 | O | THR | A | 69 | -19.354 | 83.292 | 1.177 | 1.00 | 28.56 | O |
| ATOM | 3810 | CB | THR | A | 69 | -16.720 | 83.513 | 2.739 | 1.00 | 38.78 | C |
| ATOM | 3811 | OG1 | THR | A | 69 | -15.552 | 84.216 | 3.173 | 1.00 | 33.71 | O |
| ATOM | 3812 | CG2 | THR | A | 69 | -16.475 | 81.992 | 2.856 | 1.00 | 30.01 | C |
| ATOM | 3813 | N | ILE | A | 70 | -17.892 | 82.169 | -0.143 | 1.00 | 28.12 | N |
| ATOM | 3814 | CA | ILE | A | 70 | -18.862 | 81.246 | -0.708 | 1.00 | 31.36 | C |
| ATOM | 3815 | C | ILE | A | 70 | -18.754 | 79.918 | 0.040 | 1.00 | 32.93 | C |
| ATOM | 3816 | O | ILE | A | 70 | -17.655 | 79.472 | 0.402 | 1.00 | 33.29 | O |
| ATOM | 3817 | CB | ILE | A | 70 | -18.656 | 81.079 | -2.230 | 1.00 | 31.45 | C |
| ATOM | 3818 | CG1 | ILE | A | 70 | -19.832 | 80.302 | -2.852 | 1.00 | 29.85 | C |
| ATOM | 3819 | CG2 | ILE | A | 70 | -17.257 | 80.401 | -2.554 | 1.00 | 29.79 | C |
| ATOM | 3820 | CD1 | ILE | A | 70 | -19.805 | 80.244 | -4.411 | 1.00 | 27.30 | C |
| ATOM | 3821 | N | SER | A | 71 | -19.899 | 79.300 | 0.307 | 1.00 | 30.39 | N |
| ATOM | 3822 | CA | SER | A | 71 | -19.935 | 78.023 | 1.017 | 1.00 | 34.30 | C |
| ATOM | 3823 | C | SER | A | 71 | -21.233 | 77.320 | 0.648 | 1.00 | 30.83 | C |
| ATOM | 3824 | O | SER | A | 71 | -22.137 | 77.905 | 0.046 | 1.00 | 34.13 | O |
| ATOM | 3825 | CB | SER | A | 71 | -19.827 | 78.201 | 2.544 | 1.00 | 28.89 | C |
| ATOM | 3826 | OG | SER | A | 71 | -20.944 | 78.930 | 3.075 | 1.00 | 31.85 | O |
| ATOM | 3827 | N | ARG | A | 72 | -21.333 | 76.064 | 1.046 | 1.00 | 33.01 | N |
| ATOM | 3828 | CA | ARG | A | 72 | -22.531 | 75.307 | 0.753 | 1.00 | 33.51 | C |
| ATOM | 3829 | C | ARG | A | 72 | -22.820 | 74.404 | 1.941 | 1.00 | 36.68 | C |
| ATOM | 3830 | O | ARG | A | 72 | -21.918 | 74.070 | 2.713 | 1.00 | 32.75 | O |

| | | | | | | | | | | | |
|---|---|---|---|---|---|---|---|---|---|---|---|
| ATOM | 3831 | CB | ARG | A | 72 | -22.364 | 74.492 | -0.545 | 1.00 | 29.96 | C |
| ATOM | 3832 | CG | ARG | A | 72 | -21.230 | 73.458 | -0.489 | 1.00 | 29.86 | C |
| ATOM | 3833 | CD | ARG | A | 72 | -20.985 | 72.827 | -1.859 | 1.00 | 31.26 | C |
| ATOM | 3834 | NE | ARG | A | 72 | -22.179 | 72.130 | -2.344 | 1.00 | 36.14 | N |
| ATOM | 3835 | CZ | ARG | A | 72 | -22.480 | 71.942 | -3.627 | 1.00 | 33.42 | C |
| ATOM | 3836 | NH1 | ARG | A | 72 | -21.683 | 72.400 | -4.575 | 1.00 | 31.33 | N1+ |
| ATOM | 3837 | NH2 | ARG | A | 72 | -23.599 | 71.322 | -3.972 | 1.00 | 33.70 | N |
| ATOM | 3838 | N | ASP | A | 73 | -24.094 | 74.046 | 2.108 | 1.00 | 35.23 | N |
| ATOM | 3839 | CA | ASP | A | 73 | -24.523 | 73.070 | 3.119 | 1.00 | 36.76 | C |
| ATOM | 3840 | C | ASP | A | 73 | -25.407 | 72.032 | 2.428 | 1.00 | 39.51 | C |
| ATOM | 3841 | O | ASP | A | 73 | -26.597 | 72.270 | 2.181 | 1.00 | 34.82 | O |
| ATOM | 3842 | CB | ASP | A | 73 | -25.251 | 73.761 | 4.266 | 1.00 | 34.22 | C |
| ATOM | 3843 | CG | ASP | A | 73 | -25.616 | 72.808 | 5.397 | 1.00 | 42.95 | C |
| ATOM | 3844 | OD1 | ASP | A | 73 | -25.733 | 71.576 | 5.175 | 1.00 | 42.54 | O |
| ATOM | 3845 | OD2 | ASP | A | 73 | -25.774 | 73.306 | 6.532 | 1.00 | 48.48 | O1- |
| ATOM | 3846 | N | ASN | A | 74 | -24.825 | 70.874 | 2.117 | 1.00 | 37.32 | N |
| ATOM | 3847 | CA | ASN | A | 74 | -25.569 | 69.888 | 1.350 | 1.00 | 37.99 | C |
| ATOM | 3848 | C | ASN | A | 74 | -26.773 | 69.366 | 2.126 | 1.00 | 38.75 | C |
| ATOM | 3849 | O | ASN | A | 74 | -27.823 | 69.104 | 1.530 | 1.00 | 38.15 | O |
| ATOM | 3850 | CB | ASN | A | 74 | -24.634 | 68.757 | 0.923 | 1.00 | 38.26 | C |
| ATOM | 3851 | CG | ASN | A | 74 | -23.691 | 69.174 | -0.228 | 1.00 | 43.82 | C |
| ATOM | 3852 | OD1 | ASN | A | 74 | -23.726 | 70.314 | -0.699 | 1.00 | 41.08 | O |
| ATOM | 3853 | ND2 | ASN | A | 74 | -22.837 | 68.252 | -0.664 | 1.00 | 42.34 | N |
| ATOM | 3854 | N | SER | A | 75 | -26.678 | 69.273 | 3.454 | 1.00 | 37.68 | N |
| ATOM | 3855 | CA | SER | A | 75 | -27.820 | 68.764 | 4.205 | 1.00 | 40.41 | C |
| ATOM | 3856 | C | SER | A | 75 | -29.031 | 69.694 | 4.108 | 1.00 | 39.19 | C |
| ATOM | 3857 | O | SER | A | 75 | -30.152 | 69.250 | 4.331 | 1.00 | 38.81 | O |
| ATOM | 3858 | CB | SER | A | 75 | -27.433 | 68.533 | 5.666 | 1.00 | 34.54 | C |
| ATOM | 3859 | OG | SER | A | 75 | -27.418 | 69.751 | 6.380 | 1.00 | 41.65 | O |
| ATOM | 3860 | N | LYS | A | 76 | -28.840 | 70.966 | 3.765 | 1.00 | 39.96 | N |
| ATOM | 3861 | CA | LYS | A | 76 | -29.947 | 71.888 | 3.540 | 1.00 | 32.91 | C |
| ATOM | 3862 | C | LYS | A | 76 | -30.127 | 72.270 | 2.076 | 1.00 | 33.45 | C |
| ATOM | 3863 | O | LYS | A | 76 | -30.819 | 73.255 | 1.793 | 1.00 | 32.73 | O |
| ATOM | 3864 | CB | LYS | A | 76 | -29.760 | 73.156 | 4.371 | 1.00 | 34.21 | C |
| ATOM | 3865 | CG | LYS | A | 76 | -29.536 | 72.893 | 5.843 | 1.00 | 39.47 | C |
| ATOM | 3866 | CD | LYS | A | 76 | -29.433 | 74.203 | 6.610 | 1.00 | 37.51 | C |
| ATOM | 3867 | CE | LYS | A | 76 | -29.059 | 73.960 | 8.060 | 1.00 | 36.30 | C |
| ATOM | 3868 | NZ | LYS | A | 76 | -28.974 | 75.226 | 8.812 | 1.00 | 53.39 | N1+ |
| ATOM | 3869 | N | ASN | A | 77 | -29.486 | 71.560 | 1.142 | 1.00 | 34.23 | N |
| ATOM | 3870 | CA | ASN | A | 77 | -29.510 | 71.936 | -0.275 | 1.00 | 34.08 | C |
| ATOM | 3871 | C | ASN | A | 77 | -29.340 | 73.439 | -0.531 | 1.00 | 33.69 | C |
| ATOM | 3872 | O | ASN | A | 77 | -30.019 | 73.994 | -1.395 | 1.00 | 31.88 | O |
| ATOM | 3873 | CB | ASN | A | 77 | -30.814 | 71.468 | -0.910 | 1.00 | 35.11 | C |
| ATOM | 3874 | CG | ASN | A | 77 | -30.897 | 69.952 | -1.020 | 1.00 | 45.26 | C |
| ATOM | 3875 | OD1 | ASN | A | 77 | -29.889 | 69.267 | -1.184 | 1.00 | 51.24 | O |
| ATOM | 3876 | ND2 | ASN | A | 77 | -32.095 | 69.426 | -0.909 | 1.00 | 53.41 | N |
| ATOM | 3877 | N | THR | A | 78 | -28.427 | 74.101 | 0.184 | 1.00 | 28.09 | N |
| ATOM | 3878 | CA | THR | A | 78 | -28.320 | 75.550 | 0.139 | 1.00 | 34.16 | C |
| ATOM | 3879 | C | THR | A | 78 | -26.883 | 75.999 | -0.101 | 1.00 | 31.55 | C |
| ATOM | 3880 | O | THR | A | 78 | -25.941 | 75.476 | 0.499 | 1.00 | 29.95 | O |
| ATOM | 3881 | CB | THR | A | 78 | -28.867 | 76.150 | 1.432 | 1.00 | 32.28 | C |
| ATOM | 3882 | OG1 | THR | A | 78 | -30.230 | 75.746 | 1.559 | 1.00 | 36.31 | O |
| ATOM | 3883 | CG2 | THR | A | 78 | -28.789 | 77.662 | 1.405 | 1.00 | 32.86 | C |
| ATOM | 3884 | N | LEU | A | 79 | -26.747 | 76.977 | -0.989 | 1.00 | 33.81 | N |
| ATOM | 3885 | CA | LEU | A | 79 | -25.515 | 77.701 | -1.273 | 1.00 | 30.18 | C |
| ATOM | 3886 | C | LEU | A | 79 | -25.571 | 79.060 | -0.585 | 1.00 | 32.22 | C |
| ATOM | 3887 | O | LEU | A | 79 | -26.606 | 79.724 | -0.617 | 1.00 | 34.07 | O |
| ATOM | 3888 | CB | LEU | A | 79 | -25.381 | 77.913 | -2.782 | 1.00 | 31.42 | C |
| ATOM | 3889 | CG | LEU | A | 79 | -24.281 | 78.860 | -3.263 | 1.00 | 32.10 | C |
| ATOM | 3890 | CD1 | LEU | A | 79 | -22.973 | 78.101 | -3.223 | 1.00 | 23.19 | C |
| ATOM | 3891 | CD2 | LEU | A | 79 | -24.601 | 79.393 | -4.668 | 1.00 | 27.71 | C |
| ATOM | 3892 | N | TYR | A | 80 | -24.450 | 79.498 | -0.015 | 1.00 | 30.45 | N |
| ATOM | 3893 | CA | TYR | A | 80 | -24.385 | 80.758 | 0.715 | 1.00 | 29.65 | C |
| ATOM | 3894 | C | TYR | A | 80 | -23.352 | 81.714 | 0.114 | 1.00 | 36.35 | C |
| ATOM | 3895 | O | TYR | A | 80 | -22.353 | 81.294 | -0.486 | 1.00 | 29.45 | O |
| ATOM | 3896 | CB | TYR | A | 80 | -24.022 | 80.536 | 2.194 | 1.00 | 29.90 | C |
| ATOM | 3897 | CG | TYR | A | 80 | -24.972 | 79.655 | 2.914 | 1.00 | 35.65 | C |
| ATOM | 3898 | CD1 | TYR | A | 80 | -26.209 | 80.148 | 3.347 | 1.00 | 31.27 | C |
| ATOM | 3899 | CD2 | TYR | A | 80 | -24.656 | 78.311 | 3.162 | 1.00 | 32.71 | C |
| ATOM | 3900 | CE1 | TYR | A | 80 | -27.109 | 79.334 | 4.006 | 1.00 | 30.61 | C |
| ATOM | 3901 | CE2 | TYR | A | 80 | -25.548 | 77.480 | 3.834 | 1.00 | 34.98 | C |

| | | | | | | | | | | | |
|---|---|---|---|---|---|---|---|---|---|---|---|
| ATOM | 3902 | CZ | TYR | A | 80 | -26.780 | 77.999 | 4.253 | 1.00 | 43.60 | C |
| ATOM | 3903 | OH | TYR | A | 80 | -27.691 | 77.183 | 4.903 | 1.00 | 43.60 | O |
| ATOM | 3904 | N | LEU | A | 81 | -23.608 | 83.016 | 0.286 | 1.00 | 33.92 | N |
| ATOM | 3905 | CA | LEU | A | 81 | -22.616 | 84.067 | 0.062 | 1.00 | 32.11 | C |
| ATOM | 3906 | C | LEU | A | 81 | -22.609 | 84.955 | 1.294 | 1.00 | 35.72 | C |
| ATOM | 3907 | O | LEU | A | 81 | -23.559 | 85.712 | 1.534 | 1.00 | 31.37 | O |
| ATOM | 3908 | CB | LEU | A | 81 | -22.899 | 84.890 | -1.193 | 1.00 | 29.72 | C |
| ATOM | 3909 | CG | LEU | A | 81 | -21.820 | 85.907 | -1.554 | 1.00 | 30.90 | C |
| ATOM | 3910 | CD1 | LEU | A | 81 | -20.535 | 85.193 | -2.008 | 1.00 | 31.13 | C |
| ATOM | 3911 | CD2 | LEU | A | 81 | -22.311 | 86.863 | -2.620 | 1.00 | 29.96 | C |
| ATOM | 3912 | N | GLN | A | 82 | -21.546 | 84.836 | 2.077 | 1.00 | 35.14 | N |
| ATOM | 3913 | CA | GLN | A | 82 | -21.322 | 85.681 | 3.236 | 1.00 | 35.67 | C |
| ATOM | 3914 | C | GLN | A | 82 | -20.628 | 86.948 | 2.753 | 1.00 | 33.89 | C |
| ATOM | 3915 | O | GLN | A | 82 | -19.504 | 86.883 | 2.239 | 1.00 | 36.17 | O |
| ATOM | 3916 | CB | GLN | A | 82 | -20.463 | 84.940 | 4.264 | 1.00 | 33.97 | C |
| ATOM | 3917 | CG | GLN | A | 82 | -20.206 | 85.726 | 5.526 | 1.00 | 35.10 | C |
| ATOM | 3918 | CD | GLN | A | 82 | -21.509 | 86.106 | 6.226 | 1.00 | 41.65 | C |
| ATOM | 3919 | OE1 | GLN | A | 82 | -22.419 | 85.279 | 6.375 | 1.00 | 41.07 | O |
| ATOM | 3920 | NE2 | GLN | A | 82 | -21.598 | 87.354 | 6.669 | 1.00 | 39.37 | N |
| ATOM | 3921 | N | MET | A | 83 | -21.290 | 88.089 | 2.892 | 1.00 | 34.01 | N |
| ATOM | 3922 | CA | MET | A | 83 | -20.762 | 89.349 | 2.360 | 1.00 | 40.25 | C |
| ATOM | 3923 | C | MET | A | 83 | -20.390 | 90.231 | 3.540 | 1.00 | 34.20 | C |
| ATOM | 3924 | O | MET | A | 83 | -21.271 | 90.753 | 4.231 | 1.00 | 43.36 | O |
| ATOM | 3925 | CB | MET | A | 83 | -21.782 | 90.054 | 1.460 | 1.00 | 35.88 | C |
| ATOM | 3926 | CG | MET | A | 83 | -22.247 | 89.246 | 0.242 | 1.00 | 40.32 | C |
| ATOM | 3927 | SD | MET | A | 83 | -23.444 | 90.120 | -0.837 | 1.00 | 45.75 | S |
| ATOM | 3928 | CE | MET | A | 83 | -24.869 | 90.178 | 0.214 | 1.00 | 39.89 | C |
| ATOM | 3929 | N | ASN | A | 84 | -19.098 | 90.416 | 3.767 | 1.00 | 34.41 | N |
| ATOM | 3930 | CA | ASN | A | 84 | -18.649 | 91.282 | 4.849 | 1.00 | 35.66 | C |
| ATOM | 3931 | C | ASN | A | 84 | -18.114 | 92.576 | 4.266 | 1.00 | 35.32 | C |
| ATOM | 3932 | O | ASN | A | 84 | -17.798 | 92.660 | 3.078 | 1.00 | 37.11 | O |
| ATOM | 3933 | CB | ASN | A | 84 | -17.563 | 90.616 | 5.694 | 1.00 | 33.02 | C |
| ATOM | 3934 | CG | ASN | A | 84 | -18.033 | 89.347 | 6.358 | 1.00 | 38.60 | C |
| ATOM | 3935 | OD1 | ASN | A | 84 | -19.196 | 89.206 | 6.717 | 1.00 | 38.86 | O |
| ATOM | 3936 | ND2 | ASN | A | 84 | -17.127 | 88.398 | 6.499 | 1.00 | 43.30 | N |
| ATOM | 3937 | N | SER | A | 85 | -18.051 | 93.598 | 5.113 | 1.00 | 37.32 | N |
| ATOM | 3938 | CA | SER | A | 85 | -17.446 | 94.870 | 4.742 | 1.00 | 40.34 | C |
| ATOM | 3939 | C | SER | A | 85 | -18.091 | 95.435 | 3.483 | 1.00 | 36.53 | C |
| ATOM | 3940 | O | SER | A | 85 | -17.417 | 95.833 | 2.536 | 1.00 | 42.50 | O |
| ATOM | 3941 | CB | SER | A | 85 | -15.936 | 94.707 | 4.545 | 1.00 | 41.25 | C |
| ATOM | 3942 | OG | SER | A | 85 | -15.303 | 94.426 | 5.777 | 1.00 | 48.30 | O |
| ATOM | 3943 | N | LEU | A | 86 | -19.419 | 95.440 | 3.469 | 1.00 | 36.42 | N |
| ATOM | 3944 | CA | LEU | A | 86 | -20.121 | 95.839 | 2.269 | 1.00 | 30.29 | C |
| ATOM | 3945 | C | LEU | A | 86 | -19.768 | 97.269 | 1.898 | 1.00 | 37.87 | C |
| ATOM | 3946 | O | LEU | A | 86 | -19.530 | 98.126 | 2.759 | 1.00 | 39.88 | O |
| ATOM | 3947 | CB | LEU | A | 86 | -21.620 | 95.670 | 2.458 | 1.00 | 37.21 | C |
| ATOM | 3948 | CG | LEU | A | 86 | -22.026 | 94.199 | 2.267 | 1.00 | 41.48 | C |
| ATOM | 3949 | CD1 | LEU | A | 86 | -23.420 | 93.965 | 2.745 | 1.00 | 31.33 | C |
| ATOM | 3950 | CD2 | LEU | A | 86 | -21.912 | 93.809 | 0.788 | 1.00 | 34.28 | C |
| ATOM | 3951 | N | ARG | A | 87 | -19.673 | 97.500 | 0.601 | 1.00 | 31.11 | N |
| ATOM | 3952 | CA | ARG | A | 87 | -19.397 | 98.809 | 0.054 | 1.00 | 36.58 | C |
| ATOM | 3953 | C | ARG | A | 87 | -20.539 | 99.186 | -0.890 | 1.00 | 37.14 | C |
| ATOM | 3954 | O | ARG | A | 87 | -21.233 | 98.322 | -1.439 | 1.00 | 30.93 | O |
| ATOM | 3955 | CB | ARG | A | 87 | -18.047 | 98.816 | -0.688 | 1.00 | 36.84 | C |
| ATOM | 3956 | CG | ARG | A | 87 | -16.843 | 98.100 | -0.017 | 1.00 | 39.75 | C |
| ATOM | 3957 | CD | ARG | A | 87 | -15.620 | 98.762 | -0.560 | 1.00 | 50.01 | C |
| ATOM | 3958 | NE | ARG | A | 87 | -14.715 | 97.898 | -1.313 | 1.00 | 57.70 | N |
| ATOM | 3959 | CZ | ARG | A | 87 | -13.636 | 97.274 | -0.840 | 1.00 | 54.84 | C |
| ATOM | 3960 | NH1 | ARG | A | 87 | -13.275 | 97.378 | 0.442 | 1.00 | 55.39 | N1+ |
| ATOM | 3961 | NH2 | ARG | A | 87 | -12.907 | 96.541 | -1.682 | 1.00 | 46.24 | N |
| ATOM | 3962 | N | VAL | A | 88 | -20.706 | 100.495 | -1.088 | 1.00 | 33.94 | N |
| ATOM | 3963 | CA | VAL | A | 88 | -21.786 | 101.015 | -1.927 | 1.00 | 35.85 | C |
| ATOM | 3964 | C | VAL | A | 88 | -21.768 | 100.348 | -3.297 | 1.00 | 33.17 | C |
| ATOM | 3965 | O | VAL | A | 88 | -22.812 | 99.996 | -3.856 | 1.00 | 34.21 | O |
| ATOM | 3966 | CB | VAL | A | 88 | -21.681 | 102.557 | -2.018 | 1.00 | 41.36 | C |
| ATOM | 3967 | CG1 | VAL | A | 88 | -22.508 | 103.125 | -3.182 | 1.00 | 34.83 | C |
| ATOM | 3968 | CG2 | VAL | A | 88 | -22.191 | 103.172 | -0.697 | 1.00 | 32.83 | C |
| ATOM | 3969 | N | GLU | A | 89 | -20.577 | 100.142 | -3.842 | 1.00 | 32.51 | N |
| ATOM | 3970 | CA | GLU | A | 89 | -20.416 | 99.535 | -5.149 | 1.00 | 32.60 | C |
| ATOM | 3971 | C | GLU | A | 89 | -20.831 | 98.070 | -5.195 | 1.00 | 35.92 | C |
| ATOM | 3972 | O | GLU | A | 89 | -20.914 | 97.531 | -6.303 | 1.00 | 32.77 | O |

| | | | | | | | | | | | |
|---|---|---|---|---|---|---|---|---|---|---|---|
| ATOM | 3973 | CB | GLU | A | 89 | -18.947 | 99.625 | -5.598 | 1.00 | 33.36 | C |
| ATOM | 3974 | CG | GLU | A | 89 | -18.249 | 100.917 | -5.267 | 1.00 | 52.01 | C |
| ATOM | 3975 | CD | GLU | A | 89 | -17.581 | 100.894 | -3.898 | 1.00 | 57.81 | C |
| ATOM | 3976 | OE1 | GLU | A | 89 | -18.121 | 101.544 | -2.969 | 1.00 | 46.95 | O |
| ATOM | 3977 | OE2 | GLU | A | 89 | -16.513 | 100.230 | -3.762 | 1.00 | 68.05 | O1- |
| ATOM | 3978 | N | ASP | A | 90 | -21.123 | 97.427 | -4.048 | 1.00 | 30.72 | N |
| ATOM | 3979 | CA | ASP | A | 90 | -21.653 | 96.063 | -4.058 | 1.00 | 34.90 | C |
| ATOM | 3980 | C | ASP | A | 90 | -23.142 | 96.017 | -4.360 | 1.00 | 35.83 | C |
| ATOM | 3981 | O | ASP | A | 90 | -23.710 | 94.912 | -4.422 | 1.00 | 33.53 | O |
| ATOM | 3982 | CB | ASP | A | 90 | -21.377 | 95.351 | -2.721 | 1.00 | 28.51 | C |
| ATOM | 3983 | CG | ASP | A | 90 | -19.884 | 95.173 | -2.453 | 1.00 | 37.19 | C |
| ATOM | 3984 | OD1 | ASP | A | 90 | -19.142 | 94.821 | -3.411 | 1.00 | 36.43 | O |
| ATOM | 3985 | OD2 | ASP | A | 90 | -19.439 | 95.412 | -1.300 | 1.00 | 36.43 | O1- |
| ATOM | 3986 | N | THR | A | 91 | -23.778 | 97.180 | -4.534 | 1.00 | 30.48 | N |
| ATOM | 3987 | CA | THR | A | 91 | -25.207 | 97.244 | -4.843 | 1.00 | 34.41 | C |
| ATOM | 3988 | C | THR | A | 91 | -25.488 | 96.586 | -6.183 | 1.00 | 29.60 | C |
| ATOM | 3989 | O | THR | A | 91 | -24.889 | 96.958 | -7.192 | 1.00 | 33.51 | O |
| ATOM | 3990 | CB | THR | A | 91 | -25.655 | 98.705 | -4.865 | 1.00 | 34.98 | C |
| ATOM | 3991 | OG1 | THR | A | 91 | -25.482 | 99.260 | -3.556 | 1.00 | 33.04 | O |
| ATOM | 3992 | CG2 | THR | A | 91 | -27.119 | 98.836 | -5.317 | 1.00 | 22.08 | C |
| ATOM | 3993 | N | ALA | A | 92 | -26.384 | 95.601 | -6.196 | 1.00 | 26.49 | N |
| ATOM | 3994 | CA | ALA | A | 92 | -26.628 | 94.831 | -7.418 | 1.00 | 33.82 | C |
| ATOM | 3995 | C | ALA | A | 92 | -27.764 | 93.858 | -7.167 | 1.00 | 31.11 | C |
| ATOM | 3996 | O | ALA | A | 92 | -28.109 | 93.559 | -6.019 | 1.00 | 33.45 | O |
| ATOM | 3997 | CB | ALA | A | 92 | -25.374 | 94.036 | -7.893 | 1.00 | 29.95 | C |
| ATOM | 3998 | N | VAL | A | 93 | -28.318 | 93.336 | -8.255 | 1.00 | 27.74 | N |
| ATOM | 3999 | CA | VAL | A | 93 | -29.052 | 92.083 | -8.161 | 1.00 | 27.69 | C |
| ATOM | 4000 | C | VAL | A | 93 | -28.035 | 90.949 | -8.156 | 1.00 | 32.05 | C |
| ATOM | 4001 | O | VAL | A | 93 | -27.098 | 90.935 | -8.970 | 1.00 | 32.98 | O |
| ATOM | 4002 | CB | VAL | A | 93 | -30.043 | 91.925 | -9.321 | 1.00 | 30.90 | C |
| ATOM | 4003 | CG1 | VAL | A | 93 | -30.675 | 90.530 | -9.260 | 1.00 | 27.42 | C |
| ATOM | 4004 | CG2 | VAL | A | 93 | -31.106 | 93.006 | -9.272 | 1.00 | 22.48 | C |
| ATOM | 4005 | N | TYR | A | 94 | -28.210 | 90.005 | -7.236 | 1.00 | 28.80 | N |
| ATOM | 4006 | CA | TYR | A | 94 | -27.333 | 88.851 | -7.090 | 1.00 | 27.10 | C |
| ATOM | 4007 | C | TYR | A | 94 | -28.052 | 87.579 | -7.535 | 1.00 | 30.77 | C |
| ATOM | 4008 | O | TYR | A | 94 | -29.164 | 87.290 | -7.070 | 1.00 | 32.21 | O |
| ATOM | 4009 | CB | TYR | A | 94 | -26.849 | 88.711 | -5.643 | 1.00 | 25.98 | C |
| ATOM | 4010 | CG | TYR | A | 94 | -25.744 | 89.678 | -5.299 | 1.00 | 35.21 | C |
| ATOM | 4011 | CD1 | TYR | A | 94 | -26.003 | 91.042 | -5.195 | 1.00 | 33.48 | C |
| ATOM | 4012 | CD2 | TYR | A | 94 | -24.448 | 89.235 | -5.071 | 1.00 | 28.53 | C |
| ATOM | 4013 | CE1 | TYR | A | 94 | -25.011 | 91.927 | -4.887 | 1.00 | 31.11 | C |
| ATOM | 4014 | CE2 | TYR | A | 94 | -23.439 | 90.127 | -4.754 | 1.00 | 34.57 | C |
| ATOM | 4015 | CZ | TYR | A | 94 | -23.734 | 91.482 | -4.674 | 1.00 | 34.25 | C |
| ATOM | 4016 | OH | TYR | A | 94 | -22.756 | 92.410 | -4.395 | 1.00 | 31.02 | O |
| ATOM | 4017 | N | TYR | A | 95 | -27.399 | 86.815 | -8.412 | 1.00 | 27.50 | N |
| ATOM | 4018 | CA | TYR | A | 95 | -27.904 | 85.566 | -8.958 | 1.00 | 28.28 | C |
| ATOM | 4019 | C | TYR | A | 95 | -26.990 | 84.436 | -8.531 | 1.00 | 30.88 | C |
| ATOM | 4020 | O | TYR | A | 95 | -25.769 | 84.610 | -8.464 | 1.00 | 30.35 | O |
| ATOM | 4021 | CB | TYR | A | 95 | -27.948 | 85.576 | -10.497 | 1.00 | 23.11 | C |
| ATOM | 4022 | CG | TYR | A | 95 | -28.742 | 86.673 | -11.106 | 1.00 | 28.17 | C |
| ATOM | 4023 | CD1 | TYR | A | 95 | -30.120 | 86.537 | -11.313 | 1.00 | 35.38 | C |
| ATOM | 4024 | CD2 | TYR | A | 95 | -28.128 | 87.864 | -11.486 | 1.00 | 31.26 | C |
| ATOM | 4025 | CE1 | TYR | A | 95 | -30.863 | 87.559 | -11.882 | 1.00 | 29.38 | C |
| ATOM | 4026 | CE2 | TYR | A | 95 | -28.854 | 88.887 | -12.059 | 1.00 | 27.86 | C |
| ATOM | 4027 | CZ | TYR | A | 95 | -30.210 | 88.733 | -12.251 | 1.00 | 32.29 | C |
| ATOM | 4028 | OH | TYR | A | 95 | -30.913 | 89.752 | -12.812 | 1.00 | 36.01 | O |
| ATOM | 4029 | N | CYS | A | 96 | -27.576 | 83.288 | -8.237 | 1.00 | 28.07 | N |
| ATOM | 4030 | CA | CYS | A | 96 | -26.795 | 82.069 | -8.201 | 1.00 | 31.83 | C |
| ATOM | 4031 | C | CYS | A | 96 | -27.035 | 81.314 | -9.498 | 1.00 | 30.66 | C |
| ATOM | 4032 | O | CYS | A | 96 | -28.063 | 81.482 | -10.165 | 1.00 | 27.69 | O |
| ATOM | 4033 | CB | CYS | A | 96 | -27.113 | 81.176 | -6.989 | 1.00 | 35.63 | C |
| ATOM | 4034 | SG | CYS | A | 96 | -28.807 | 80.660 | -6.800 | 1.00 | 52.39 | S |
| ATOM | 4035 | N | ALA | A | 97 | -26.055 | 80.500 | -9.860 | 1.00 | 25.32 | N |
| ATOM | 4036 | CA | ALA | A | 97 | -26.171 | 79.615 | -11.005 | 1.00 | 29.44 | C |
| ATOM | 4037 | C | ALA | A | 97 | -25.383 | 78.346 | -10.695 | 1.00 | 29.42 | C |
| ATOM | 4038 | O | ALA | A | 97 | -24.512 | 78.346 | -9.823 | 1.00 | 32.47 | O |
| ATOM | 4039 | CB | ALA | A | 97 | -25.671 | 80.294 | -12.287 | 1.00 | 26.70 | C |
| ATOM | 4040 | N | ASN | A | 98 | -25.693 | 77.251 | -11.396 | 1.00 | 25.38 | N |
| ATOM | 4041 | CA | ASN | A | 98 | -24.856 | 76.072 | -11.236 | 1.00 | 31.07 | C |
| ATOM | 4042 | C | ASN | A | 98 | -23.922 | 75.893 | -12.441 | 1.00 | 25.38 | C |
| ATOM | 4043 | O | ASN | A | 98 | -23.936 | 76.655 | -13.416 | 1.00 | 26.89 | O |

| | | | | | | | | | | | |
|---|---|---|---|---|---|---|---|---|---|---|---|
| ATOM | 4044 | CB | ASN | A | 98 | -25.714 | 74.832 | -10.962 | 1.00 | 26.42 | C |
| ATOM | 4045 | CG | ASN | A | 98 | -26.528 | 74.385 | -12.153 | 1.00 | 31.33 | C |
| ATOM | 4046 | OD1 | ASN | A | 98 | -26.444 | 74.948 | -13.257 | 1.00 | 31.54 | O |
| ATOM | 4047 | ND2 | ASN | A | 98 | -27.362 | 73.366 | -11.925 | 1.00 | 34.14 | N |
| ATOM | 4048 | N | TRP | A | 99 | -23.088 | 74.869 | -12.372 | 1.00 | 27.06 | N |
| ATOM | 4049 | CA | TRP | A | 99 | -22.251 | 74.549 | -13.521 | 1.00 | 30.36 | C |
| ATOM | 4050 | C | TRP | A | 99 | -22.064 | 73.046 | -13.590 | 1.00 | 30.04 | C |
| ATOM | 4051 | O | TRP | A | 99 | -21.903 | 72.391 | -12.555 | 1.00 | 29.39 | O |
| ATOM | 4052 | CB | TRP | A | 99 | -20.897 | 75.266 | -13.450 | 1.00 | 25.45 | C |
| ATOM | 4053 | CG | TRP | A | 99 | -19.972 | 74.863 | -12.353 | 1.00 | 29.81 | C |
| ATOM | 4054 | CD1 | TRP | A | 99 | -19.834 | 75.449 | -11.122 | 1.00 | 28.02 | C |
| ATOM | 4055 | CD2 | TRP | A | 99 | -18.997 | 73.810 | -12.408 | 1.00 | 26.75 | C |
| ATOM | 4056 | NE1 | TRP | A | 99 | -18.818 | 74.830 | -10.414 | 1.00 | 23.44 | N |
| ATOM | 4057 | CE2 | TRP | A | 99 | -18.300 | 73.816 | -11.178 | 1.00 | 25.89 | C |
| ATOM | 4058 | CE3 | TRP | A | 99 | -18.634 | 72.880 | -13.385 | 1.00 | 28.32 | C |
| ATOM | 4059 | CZ2 | TRP | A | 99 | -17.278 | 72.904 | -10.891 | 1.00 | 30.78 | C |
| ATOM | 4060 | CZ3 | TRP | A | 99 | -17.613 | 71.972 | -13.101 | 1.00 | 30.19 | C |
| ATOM | 4061 | CH2 | TRP | A | 99 | -16.951 | 71.992 | -11.859 | 1.00 | 28.00 | C |
| ATOM | 4062 | N | TYR | A | 100 | -22.084 | 72.513 | -14.814 | 1.00 | 30.79 | N |
| ATOM | 4063 | CA | TYR | A | 100 | -22.044 | 71.070 | -15.081 | 1.00 | 34.23 | C |
| ATOM | 4064 | C | TYR | A | 100 | -20.741 | 70.594 | -15.703 | 1.00 | 36.36 | C |
| ATOM | 4065 | O | TYR | A | 100 | -20.246 | 69.521 | -15.339 | 1.00 | 31.49 | O |
| ATOM | 4066 | CB | TYR | A | 100 | -23.188 | 70.657 | -16.023 | 1.00 | 29.48 | C |
| ATOM | 4067 | CG | TYR | A | 100 | -24.563 | 70.844 | -15.457 | 1.00 | 29.45 | C |
| ATOM | 4068 | CD1 | TYR | A | 100 | -24.812 | 70.621 | -14.106 | 1.00 | 32.61 | C |
| ATOM | 4069 | CD2 | TYR | A | 100 | -25.623 | 71.233 | -16.273 | 1.00 | 31.46 | C |
| ATOM | 4070 | CE1 | TYR | A | 100 | -26.081 | 70.796 | -13.576 | 1.00 | 32.67 | C |
| ATOM | 4071 | CE2 | TYR | A | 100 | -26.904 | 71.403 | -15.756 | 1.00 | 30.44 | C |
| ATOM | 4072 | CZ | TYR | A | 100 | -27.124 | 71.173 | -14.404 | 1.00 | 31.95 | C |
| ATOM | 4073 | OH | TYR | A | 100 | -28.372 | 71.337 | -13.866 | 1.00 | 30.17 | O |
| ATOM | 4074 | N | TYR | A | 101 | -20.183 | 71.382 | -16.626 | 1.00 | 29.07 | N |
| ATOM | 4075 | CA | TYR | A | 101 | -19.125 | 70.940 | -17.527 | 1.00 | 29.25 | C |
| ATOM | 4076 | C | TYR | A | 101 | -17.797 | 71.608 | -17.189 | 1.00 | 34.28 | C |
| ATOM | 4077 | O | TYR | A | 101 | -16.868 | 70.944 | -16.723 | 1.00 | 35.11 | O |
| ATOM | 4078 | CB | TYR | A | 101 | -19.548 | 71.243 | -18.955 | 1.00 | 24.67 | C |
| ATOM | 4079 | CG | TYR | A | 101 | -20.914 | 70.690 | -19.282 | 1.00 | 32.07 | C |
| ATOM | 4080 | CD1 | TYR | A | 101 | -21.119 | 69.317 | -19.342 | 1.00 | 32.40 | C |
| ATOM | 4081 | CD2 | TYR | A | 101 | -21.996 | 71.534 | -19.549 | 1.00 | 27.53 | C |
| ATOM | 4082 | CE1 | TYR | A | 101 | -22.357 | 68.791 | -19.657 | 1.00 | 33.92 | C |
| ATOM | 4083 | CE2 | TYR | A | 101 | -23.255 | 71.019 | -19.850 | 1.00 | 27.34 | C |
| ATOM | 4084 | CZ | TYR | A | 101 | -23.419 | 69.641 | -19.902 | 1.00 | 34.29 | C |
| ATOM | 4085 | OH | TYR | A | 101 | -24.622 | 69.081 | -20.215 | 1.00 | 35.46 | O |
| ATOM | 4086 | N | TYR | A | 102 | -17.670 | 72.911 | -17.415 | 1.00 | 28.83 | N |
| ATOM | 4087 | CA | TYR | A | 102 | -16.483 | 73.631 | -16.998 | 1.00 | 29.54 | C |
| ATOM | 4088 | C | TYR | A | 102 | -16.872 | 74.660 | -15.951 | 1.00 | 31.00 | C |
| ATOM | 4089 | O | TYR | A | 102 | -17.957 | 75.244 | -16.004 | 1.00 | 33.23 | O |
| ATOM | 4090 | CB | TYR | A | 102 | -15.761 | 74.287 | -18.187 | 1.00 | 27.78 | C |
| ATOM | 4091 | CG | TYR | A | 102 | -16.664 | 74.965 | -19.187 | 1.00 | 30.93 | C |
| ATOM | 4092 | CD1 | TYR | A | 102 | -17.186 | 74.251 | -20.261 | 1.00 | 31.23 | C |
| ATOM | 4093 | CD2 | TYR | A | 102 | -16.985 | 76.326 | -19.074 | 1.00 | 27.14 | C |
| ATOM | 4094 | CE1 | TYR | A | 102 | -18.004 | 74.864 | -21.204 | 1.00 | 29.52 | C |
| ATOM | 4095 | CE2 | TYR | A | 102 | -17.801 | 76.957 | -20.027 | 1.00 | 26.32 | C |
| ATOM | 4096 | CZ | TYR | A | 102 | -18.311 | 76.217 | -21.086 | 1.00 | 29.71 | C |
| ATOM | 4097 | OH | TYR | A | 102 | -19.120 | 76.803 | -22.045 | 1.00 | 27.38 | O |
| ATOM | 4098 | N | TYR | A | 103 | -15.960 | 74.870 | -15.000 | 1.00 | 33.17 | N |
| ATOM | 4099 | CA | TYR | A | 103 | -16.282 | 75.569 | -13.768 | 1.00 | 30.18 | C |
| ATOM | 4100 | C | TYR | A | 103 | -16.585 | 77.043 | -13.983 | 1.00 | 30.85 | C |
| ATOM | 4101 | O | TYR | A | 103 | -17.198 | 77.659 | -13.111 | 1.00 | 31.82 | O |
| ATOM | 4102 | CB | TYR | A | 103 | -15.133 | 75.420 | -12.772 | 1.00 | 30.32 | C |
| ATOM | 4103 | CG | TYR | A | 103 | -13.820 | 76.094 | -13.175 | 1.00 | 36.74 | C |
| ATOM | 4104 | CD1 | TYR | A | 103 | -12.864 | 75.419 | -13.922 | 1.00 | 34.48 | C |
| ATOM | 4105 | CD2 | TYR | A | 103 | -13.527 | 77.399 | -12.776 | 1.00 | 36.39 | C |
| ATOM | 4106 | CE1 | TYR | A | 103 | -11.652 | 76.031 | -14.266 | 1.00 | 37.99 | C |
| ATOM | 4107 | CE2 | TYR | A | 103 | -12.332 | 78.012 | -13.120 | 1.00 | 34.39 | C |
| ATOM | 4108 | CZ | TYR | A | 103 | -11.394 | 77.329 | -13.860 | 1.00 | 40.90 | C |
| ATOM | 4109 | OH | TYR | A | 103 | -10.196 | 77.947 | -14.206 | 1.00 | 43.10 | O |
| ATOM | 4110 | N | TYR | A | 104 | -16.189 | 77.616 | -15.112 | 1.00 | 27.43 | N |
| ATOM | 4111 | CA | TYR | A | 104 | -16.416 | 79.027 | -15.373 | 1.00 | 29.07 | C |
| ATOM | 4112 | C | TYR | A | 104 | -17.599 | 79.242 | -16.291 | 1.00 | 28.87 | C |
| ATOM | 4113 | O | TYR | A | 104 | -17.791 | 80.354 | -16.780 | 1.00 | 35.12 | O |
| ATOM | 4114 | CB | TYR | A | 104 | -15.149 | 79.690 | -15.953 | 1.00 | 25.68 | C |

| | | | | | | | | | | | |
|---|---|---|---|---|---|---|---|---|---|---|---|
| ATOM | 4115 | CG | TYR | A | 104 | -14.499 | 78.931 | -17.095 | 1.00 | 30.36 | C |
| ATOM | 4116 | CD1 | TYR | A | 104 | -13.573 | 77.894 | -16.846 | 1.00 | 29.10 | C |
| ATOM | 4117 | CD2 | TYR | A | 104 | -14.813 | 79.233 | -18.429 | 1.00 | 27.13 | C |
| ATOM | 4118 | CE1 | TYR | A | 104 | -12.964 | 77.181 | -17.913 | 1.00 | 27.53 | C |
| ATOM | 4119 | CE2 | TYR | A | 104 | -14.225 | 78.528 | -19.494 | 1.00 | 27.81 | C |
| ATOM | 4120 | CZ | TYR | A | 104 | -13.296 | 77.508 | -19.232 | 1.00 | 31.37 | C |
| ATOM | 4121 | OH | TYR | A | 104 | -12.708 | 76.829 | -20.282 | 1.00 | 33.25 | O |
| ATOM | 4122 | N | GLY | A | 105 | -18.385 | 78.199 | -16.546 | 1.00 | 33.05 | N |
| ATOM | 4123 | CA | GLY | A | 105 | -19.642 | 78.324 | -17.262 | 1.00 | 28.37 | C |
| ATOM | 4124 | C | GLY | A | 105 | -20.806 | 78.259 | -16.290 | 1.00 | 33.54 | C |
| ATOM | 4125 | O | GLY | A | 105 | -20.731 | 77.601 | -15.262 | 1.00 | 36.54 | O |
| ATOM | 4126 | N | MET | A | 106 | -21.885 | 78.955 | -16.614 | 1.00 | 33.38 | N |
| ATOM | 4127 | CA | MET | A | 106 | -23.107 | 78.909 | -15.818 | 1.00 | 33.92 | C |
| ATOM | 4128 | C | MET | A | 106 | -24.192 | 78.210 | -16.619 | 1.00 | 33.40 | C |
| ATOM | 4129 | O | MET | A | 106 | -24.516 | 78.645 | -17.727 | 1.00 | 41.54 | O |
| ATOM | 4130 | CB | MET | A | 106 | -23.575 | 80.314 | -15.438 | 1.00 | 31.98 | C |
| ATOM | 4131 | CG | MET | A | 106 | -22.640 | 81.023 | -14.492 | 1.00 | 38.84 | C |
| ATOM | 4132 | SD | MET | A | 106 | -21.690 | 82.292 | -15.342 | 1.00 | 39.21 | S |
| ATOM | 4133 | CE | MET | A | 106 | -20.191 | 82.280 | -14.355 | 1.00 | 44.14 | C |
| ATOM | 4134 | N | ASP | A | 107 | -24.786 | 77.161 | -16.049 | 1.00 | 30.51 | N |
| ATOM | 4135 | CA | ASP | A | 107 | -25.760 | 76.380 | -16.809 | 1.00 | 30.12 | C |
| ATOM | 4136 | C | ASP | A | 107 | -27.213 | 76.688 | -16.421 | 1.00 | 31.04 | C |
| ATOM | 4137 | O | ASP | A | 107 | -28.019 | 76.977 | -17.300 | 1.00 | 30.61 | O |
| ATOM | 4138 | CB | ASP | A | 107 | -25.432 | 74.882 | -16.678 | 1.00 | 32.36 | C |
| ATOM | 4139 | CG | ASP | A | 107 | -24.108 | 74.509 | -17.383 | 1.00 | 34.37 | C |
| ATOM | 4140 | OD1 | ASP | A | 107 | -24.105 | 74.413 | -18.627 | 1.00 | 32.86 | O1- |
| ATOM | 4141 | OD2 | ASP | A | 107 | -23.060 | 74.339 | -16.705 | 1.00 | 36.09 | O |
| ATOM | 4142 | N | VAL | A | 108 | -27.581 | 76.603 | -15.138 | 1.00 | 32.41 | N |
| ATOM | 4143 | CA | VAL | A | 108 | -28.922 | 76.954 | -14.662 | 1.00 | 26.51 | C |
| ATOM | 4144 | C | VAL | A | 108 | -28.818 | 78.175 | -13.754 | 1.00 | 32.76 | C |
| ATOM | 4145 | O | VAL | A | 108 | -27.911 | 78.254 | -12.920 | 1.00 | 31.62 | O |
| ATOM | 4146 | CB | VAL | A | 108 | -29.590 | 75.776 | -13.915 | 1.00 | 28.43 | C |
| ATOM | 4147 | CG1 | VAL | A | 108 | -31.028 | 76.146 | -13.506 | 1.00 | 27.65 | C |
| ATOM | 4148 | CG2 | VAL | A | 108 | -29.617 | 74.516 | -14.803 | 1.00 | 30.87 | C |
| ATOM | 4149 | N | TRP | A | 109 | -29.752 | 79.117 | -13.893 | 1.00 | 33.08 | N |
| ATOM | 4150 | CA | TRP | A | 109 | -29.744 | 80.348 | -13.102 | 1.00 | 34.02 | C |
| ATOM | 4151 | C | TRP | A | 109 | -30.981 | 80.440 | -12.208 | 1.00 | 33.68 | C |
| ATOM | 4152 | O | TRP | A | 109 | -32.064 | 79.966 | -12.563 | 1.00 | 33.19 | O |
| ATOM | 4153 | CB | TRP | A | 109 | -29.716 | 81.605 | -13.997 | 1.00 | 30.84 | C |
| ATOM | 4154 | CG | TRP | A | 109 | -28.485 | 81.762 | -14.834 | 1.00 | 36.98 | C |
| ATOM | 4155 | CD1 | TRP | A | 109 | -28.097 | 80.972 | -15.893 | 1.00 | 30.27 | C |
| ATOM | 4156 | CD2 | TRP | A | 109 | -27.503 | 82.805 | -14.730 | 1.00 | 31.30 | C |
| ATOM | 4157 | NE1 | TRP | A | 109 | -26.927 | 81.459 | -16.432 | 1.00 | 31.83 | N |
| ATOM | 4158 | CE2 | TRP | A | 109 | -26.534 | 82.571 | -15.731 | 1.00 | 29.54 | C |
| ATOM | 4159 | CE3 | TRP | A | 109 | -27.339 | 83.902 | -13.875 | 1.00 | 30.44 | C |
| ATOM | 4160 | CZ2 | TRP | A | 109 | -25.409 | 83.392 | -15.896 | 1.00 | 31.19 | C |
| ATOM | 4161 | CZ3 | TRP | A | 109 | -26.223 | 84.723 | -14.042 | 1.00 | 30.38 | C |
| ATOM | 4162 | CH2 | TRP | A | 109 | -25.271 | 84.460 | -15.043 | 1.00 | 29.90 | C |
| ATOM | 4163 | N | GLY | A | 110 | -30.820 | 81.113 | -11.066 | 1.00 | 29.11 | N |
| ATOM | 4164 | CA | GLY | A | 110 | -31.931 | 81.457 | -10.200 | 1.00 | 35.80 | C |
| ATOM | 4165 | C | GLY | A | 110 | -32.644 | 82.701 | -10.704 | 1.00 | 37.01 | C |
| ATOM | 4166 | O | GLY | A | 110 | -32.532 | 83.075 | -11.871 | 1.00 | 37.79 | O |
| ATOM | 4167 | N | GLN | A | 111 | -33.399 | 83.358 | -9.817 | 1.00 | 35.07 | N |
| ATOM | 4168 | CA | GLN | A | 111 | -34.189 | 84.495 | -10.276 | 1.00 | 30.15 | C |
| ATOM | 4169 | C | GLN | A | 111 | -33.645 | 85.841 | -9.831 | 1.00 | 32.38 | C |
| ATOM | 4170 | O | GLN | A | 111 | -34.080 | 86.862 | -10.372 | 1.00 | 33.82 | O |
| ATOM | 4171 | CB | GLN | A | 111 | -35.644 | 84.410 | -9.787 | 1.00 | 37.66 | C |
| ATOM | 4172 | CG | GLN | A | 111 | -35.874 | 85.115 | -8.454 | 1.00 | 36.90 | C |
| ATOM | 4173 | CD | GLN | A | 111 | -35.642 | 84.208 | -7.281 | 1.00 | 42.01 | C |
| ATOM | 4174 | OE1 | GLN | A | 111 | -35.896 | 82.996 | -7.380 | 1.00 | 52.02 | O |
| ATOM | 4175 | NE2 | GLN | A | 111 | -35.176 | 84.777 | -6.142 | 1.00 | 30.60 | N |
| ATOM | 4176 | N | GLY | A | 112 | -32.755 | 85.872 | -8.846 | 1.00 | 31.52 | N |
| ATOM | 4177 | CA | GLY | A | 112 | -32.098 | 87.079 | -8.391 | 1.00 | 29.27 | C |
| ATOM | 4178 | C | GLY | A | 112 | -32.707 | 87.635 | -7.114 | 1.00 | 30.54 | C |
| ATOM | 4179 | O | GLY | A | 112 | -33.900 | 87.487 | -6.840 | 1.00 | 36.59 | O |
| ATOM | 4180 | N | THR | A | 113 | -31.873 | 88.297 | -6.326 | 1.00 | 32.24 | N |
| ATOM | 4181 | CA | THR | A | 113 | -32.301 | 89.057 | -5.166 | 1.00 | 33.85 | C |
| ATOM | 4182 | C | THR | A | 113 | -31.459 | 90.323 | -5.122 | 1.00 | 32.53 | C |
| ATOM | 4183 | O | THR | A | 113 | -30.284 | 90.295 | -5.496 | 1.00 | 30.25 | O |
| ATOM | 4184 | CB | THR | A | 113 | -32.150 | 88.240 | -3.878 | 1.00 | 31.09 | C |
| ATOM | 4185 | OG1 | THR | A | 113 | -32.645 | 89.016 | -2.780 | 1.00 | 36.12 | O |

| | | | | | | | | | | | | |
|---|---|---|---|---|---|---|---|---|---|---|---|---|
| ATOM | 4186 | CG2 | THR | A | 113 | -30.672 | 87.868 | -3.623 | 1.00 | 30.38 | | C |
| ATOM | 4187 | N | ATHR | A | 114 | -32.052 | 91.460 | -4.734 | 0.50 | 32.93 | | N |
| ATOM | 4188 | CA | ATHR | A | 114 | -31.300 | 92.714 | -4.776 | 0.50 | 30.39 | | C |
| ATOM | 4189 | C | ATHR | A | 114 | -30.691 | 93.019 | -3.419 | 0.50 | 31.37 | | C |
| ATOM | 4190 | O | ATHR | A | 114 | -31.310 | 92.805 | -2.374 | 0.50 | 33.91 | | O |
| ATOM | 4191 | CB | ATHR | A | 114 | -32.120 | 93.928 | -5.266 | 0.50 | 31.38 | | C |
| ATOM | 4192 | OG1 | ATHR | A | 114 | -31.978 | 95.061 | -4.375 | 0.50 | 27.49 | | O |
| ATOM | 4193 | CG2 | ATHR | A | 114 | -33.533 | 93.593 | -5.476 | 0.50 | 26.66 | | C |
| ATOM | 4194 | N | BTHR | A | 114 | -32.061 | 91.410 | -4.642 | 0.50 | 32.98 | | N |
| ATOM | 4195 | CA | BTHR | A | 114 | -31.413 | 92.712 | -4.641 | 0.50 | 30.32 | | C |
| ATOM | 4196 | C | BTHR | A | 114 | -30.650 | 92.930 | -3.345 | 0.50 | 31.38 | | C |
| ATOM | 4197 | O | BTHR | A | 114 | -31.141 | 92.610 | -2.260 | 0.50 | 34.03 | | O |
| ATOM | 4198 | CB | BTHR | A | 114 | -32.434 | 93.834 | -4.810 | 0.50 | 30.28 | | C |
| ATOM | 4199 | OG1 | BTHR | A | 114 | -33.299 | 93.846 | -3.681 | 0.50 | 37.62 | | O |
| ATOM | 4200 | CG2 | BTHR | A | 114 | -33.258 | 93.627 | -6.018 | 0.50 | 25.95 | | C |
| ATOM | 4201 | N | VAL | A | 115 | -29.451 | 93.489 | -3.463 | 1.00 | 29.38 | | N |
| ATOM | 4202 | CA | VAL | A | 115 | -28.711 | 93.973 | -2.317 | 1.00 | 31.80 | | C |
| ATOM | 4203 | C | VAL | A | 115 | -28.460 | 95.452 | -2.583 | 1.00 | 32.96 | | C |
| ATOM | 4204 | O | VAL | A | 115 | -27.891 | 95.811 | -3.622 | 1.00 | 32.78 | | O |
| ATOM | 4205 | CB | VAL | A | 115 | -27.392 | 93.209 | -2.130 | 1.00 | 36.32 | | C |
| ATOM | 4206 | CG1 | VAL | A | 115 | -26.569 | 93.856 | -1.020 | 1.00 | 35.15 | | C |
| ATOM | 4207 | CG2 | VAL | A | 115 | -27.651 | 91.686 | -1.886 | 1.00 | 32.11 | | C |
| ATOM | 4208 | N | THR | A | 116 | -28.917 | 96.307 | -1.676 | 1.00 | 32.75 | | N |
| ATOM | 4209 | CA | THR | A | 116 | -28.678 | 97.741 | -1.754 | 1.00 | 34.57 | | C |
| ATOM | 4210 | C | THR | A | 116 | -27.787 | 98.139 | -0.584 | 1.00 | 34.59 | | C |
| ATOM | 4211 | O | THR | A | 116 | -28.121 | 97.881 | 0.580 | 1.00 | 36.50 | | O |
| ATOM | 4212 | CB | THR | A | 116 | -29.995 | 98.522 | -1.736 | 1.00 | 43.36 | | C |
| ATOM | 4213 | OG1 | THR | A | 116 | -30.802 | 98.125 | -2.856 | 1.00 | 40.71 | | O |
| ATOM | 4214 | CG2 | THR | A | 116 | -29.731 | 100.021 | -1.809 | 1.00 | 29.94 | | C |
| ATOM | 4215 | N | VAL | A | 117 | -26.649 | 98.738 | -0.890 | 1.00 | 34.23 | | N |
| ATOM | 4216 | CA | VAL | A | 117 | -25.719 | 99.181 | 0.134 | 1.00 | 34.01 | | C |
| ATOM | 4217 | C | VAL | A | 117 | -25.756 | 100.691 | 0.076 | 1.00 | 33.64 | | C |
| ATOM | 4218 | O | VAL | A | 117 | -25.318 | 101.293 | -0.906 | 1.00 | 37.46 | | O |
| ATOM | 4219 | CB | VAL | A | 117 | -24.301 | 98.631 | -0.073 | 1.00 | 36.31 | | C |
| ATOM | 4220 | CG1 | VAL | A | 117 | -23.392 | 99.053 | 1.093 | 1.00 | 33.46 | | C |
| ATOM | 4221 | CG2 | VAL | A | 117 | -24.317 | 97.090 | -0.232 | 1.00 | 29.32 | | C |
| ATOM | 4222 | N | SER | A | 118 | -26.359 | 101.302 | 1.081 | 1.00 | 39.03 | | N |
| ATOM | 4223 | CA | SER | A | 118 | -26.566 | 102.738 | 1.077 | 1.00 | 39.96 | | C |
| ATOM | 4224 | C | SER | A | 118 | -26.419 | 103.243 | 2.497 | 1.00 | 45.19 | | C |
| ATOM | 4225 | O | SER | A | 118 | -26.858 | 102.579 | 3.439 | 1.00 | 47.46 | | O |
| ATOM | 4226 | CB | SER | A | 118 | -27.961 | 103.097 | 0.529 | 1.00 | 38.81 | | C |
| ATOM | 4227 | OG | SER | A | 118 | -28.165 | 104.501 | 0.460 | 1.00 | 48.85 | | O |
| ATOM | 4228 | N | SER | A | 119 | -25.802 | 104.406 | 2.653 | 1.00 | 46.28 | | N |
| ATOM | 4229 | CA | SER | A | 119 | -25.847 | 105.090 | 3.935 | 1.00 | 53.97 | | C |
| ATOM | 4230 | C | SER | A | 119 | -26.871 | 106.217 | 3.954 | 1.00 | 56.65 | | C |
| ATOM | 4231 | O | SER | A | 119 | -26.985 | 106.913 | 4.961 | 1.00 | 67.20 | | O |
| ATOM | 4232 | CB | SER | A | 119 | -24.468 | 105.628 | 4.292 | 1.00 | 47.03 | | C |
| ATOM | 4233 | OG | SER | A | 119 | -23.903 | 106.260 | 3.168 | 1.00 | 54.58 | | O |
| ATOM | 4234 | N | ALA | A | 120 | -27.649 | 106.385 | 2.890 | 1.00 | 48.46 | | N |
| ATOM | 4235 | CA | ALA | A | 120 | -28.631 | 107.451 | 2.863 | 1.00 | 46.93 | | C |
| ATOM | 4236 | C | ALA | A | 120 | -29.791 | 107.119 | 3.793 | 1.00 | 50.99 | | C |
| ATOM | 4237 | O | ALA | A | 120 | -30.104 | 105.957 | 4.047 | 1.00 | 62.07 | | O |
| ATOM | 4238 | CB | ALA | A | 120 | -29.138 | 107.672 | 1.439 | 1.00 | 43.79 | | C |
| ATOM | 4239 | N | SER | A | 121 | -30.399 | 108.159 | 4.341 | 1.00 | 61.71 | GZ00 | N |
| ATOM | 4240 | CA | SER | A | 121 | -31.605 | 108.063 | 5.149 | 1.00 | 54.23 | GZ00 | C |
| ATOM | 4241 | C | SER | A | 121 | -32.579 | 109.105 | 4.648 | 1.00 | 47.84 | GZ00 | C |
| ATOM | 4242 | O | SER | A | 121 | -32.202 | 110.015 | 3.907 | 1.00 | 55.11 | GZ00 | O |
| ATOM | 4243 | CB | SER | A | 121 | -31.338 | 108.273 | 6.640 | 1.00 | 50.17 | GZ00 | C |
| ATOM | 4244 | OG | SER | A | 121 | -30.386 | 109.312 | 6.829 | 1.00 | 66.59 | GZ00 | O |
| ATOM | 4245 | N | THR | A | 122 | -33.838 | 108.938 | 5.041 | 1.00 | 46.14 | GZ00 | N |
| ATOM | 4246 | CA | THR | A | 122 | -34.934 | 109.776 | 4.572 | 1.00 | 52.42 | GZ00 | C |
| ATOM | 4247 | C | THR | A | 122 | -34.557 | 111.248 | 4.510 | 1.00 | 56.26 | GZ00 | C |
| ATOM | 4248 | O | THR | A | 122 | -33.924 | 111.783 | 5.426 | 1.00 | 48.53 | GZ00 | O |
| ATOM | 4249 | CB | THR | A | 122 | -36.129 | 109.606 | 5.486 | 1.00 | 52.24 | GZ00 | C |
| ATOM | 4250 | OG1 | THR | A | 122 | -36.428 | 108.215 | 5.593 | 1.00 | 57.02 | GZ00 | O |
| ATOM | 4251 | CG2 | THR | A | 122 | -37.310 | 110.342 | 4.912 | 1.00 | 53.18 | GZ00 | C |
| ATOM | 4252 | N | LYS | A | 123 | -34.894 | 111.876 | 3.385 | 1.00 | 54.75 | GZ00 | N |
| ATOM | 4253 | CA | LYS | A | 123 | -34.659 | 113.296 | 3.176 | 1.00 | 45.96 | GZ00 | C |
| ATOM | 4254 | C | LYS | A | 123 | -35.645 | 113.763 | 2.125 | 1.00 | 53.79 | GZ00 | C |
| ATOM | 4255 | O | LYS | A | 123 | -35.799 | 113.095 | 1.099 | 1.00 | 47.21 | GZ00 | O |
| ATOM | 4256 | CB | LYS | A | 123 | -33.228 | 113.577 | 2.727 | 1.00 | 48.41 | GZ00 | C |

| | | | | | | | | | | | |
|---|---|---|---|---|---|---|---|---|---|---|---|
| ATOM | 4257 | CG | LYS | A | 123 | -32.969 | 115.041 | 2.451 | 1.00 | 46.73 | GZ00 C |
| ATOM | 4258 | CD | LYS | A | 123 | -31.559 | 115.291 | 1.972 | 1.00 | 52.19 | GZ00 C |
| ATOM | 4259 | CE | LYS | A | 123 | -31.365 | 116.750 | 1.520 | 1.00 | 54.71 | GZ00 C |
| ATOM | 4260 | NZ | LYS | A | 123 | -32.367 | 117.163 | 0.481 | 1.00 | 49.08 | GZ00 N1+ |
| ATOM | 4261 | N | GLY | A | 124 | -36.311 | 114.898 | 2.382 | 1.00 | 53.18 | GZ00 N |
| ATOM | 4262 | CA | GLY | A | 124 | -37.243 | 115.482 | 1.440 | 1.00 | 35.65 | GZ00 C |
| ATOM | 4263 | C | GLY | A | 124 | -36.503 | 116.234 | 0.347 | 1.00 | 38.66 | GZ00 C |
| ATOM | 4264 | O | GLY | A | 124 | -35.330 | 116.589 | 0.499 | 1.00 | 39.24 | GZ00 O |
| ATOM | 4265 | N | PRO | A | 125 | -37.165 | 116.475 | -0.782 | 1.00 | 39.30 | GZ00 N |
| ATOM | 4266 | CA | PRO | A | 125 | -36.474 | 117.071 | -1.933 | 1.00 | 45.65 | GZ00 C |
| ATOM | 4267 | C | PRO | A | 125 | -36.378 | 118.586 | -1.844 | 1.00 | 46.64 | GZ00 C |
| ATOM | 4268 | O | PRO | A | 125 | -37.212 | 119.253 | -1.230 | 1.00 | 46.44 | GZ00 O |
| ATOM | 4269 | CB | PRO | A | 125 | -37.378 | 116.684 | -3.110 | 1.00 | 44.97 | GZ00 C |
| ATOM | 4270 | CG | PRO | A | 125 | -38.764 | 116.674 | -2.504 | 1.00 | 34.50 | GZ00 C |
| ATOM | 4271 | CD | PRO | A | 125 | -38.573 | 116.145 | -1.088 | 1.00 | 36.15 | GZ00 C |
| ATOM | 4272 | N | SER | A | 126 | -35.358 | 119.126 | -2.502 | 1.00 | 46.29 | GZ00 N |
| ATOM | 4273 | CA | SER | A | 126 | -35.329 | 120.540 | -2.858 | 1.00 | 46.55 | GZ00 C |
| ATOM | 4274 | C | SER | A | 126 | -35.926 | 120.698 | -4.247 | 1.00 | 51.08 | GZ00 C |
| ATOM | 4275 | O | SER | A | 126 | -35.646 | 119.893 | -5.139 | 1.00 | 52.88 | GZ00 O |
| ATOM | 4276 | CB | SER | A | 126 | -33.903 | 121.093 | -2.860 | 1.00 | 40.26 | GZ00 C |
| ATOM | 4277 | OG | SER | A | 126 | -33.293 | 120.973 | -1.591 | 1.00 | 55.79 | GZ00 O |
| ATOM | 4278 | N | VAL | A | 127 | -36.744 | 121.735 | -4.438 | 1.00 | 47.44 | GZ00 N |
| ATOM | 4279 | CA | VAL | A | 127 | -37.420 | 121.956 | -5.713 | 1.00 | 40.11 | GZ00 C |
| ATOM | 4280 | C | VAL | A | 127 | -36.980 | 123.290 | -6.314 | 1.00 | 43.78 | GZ00 C |
| ATOM | 4281 | O | VAL | A | 127 | -37.076 | 124.339 | -5.665 | 1.00 | 52.62 | GZ00 O |
| ATOM | 4282 | CB | VAL | A | 127 | -38.948 | 121.898 | -5.553 | 1.00 | 45.33 | GZ00 C |
| ATOM | 4283 | CG1 | VAL | A | 127 | -39.618 | 122.036 | -6.908 | 1.00 | 44.26 | GZ00 C |
| ATOM | 4284 | CG2 | VAL | A | 127 | -39.359 | 120.584 | -4.873 | 1.00 | 41.94 | GZ00 C |
| ATOM | 4285 | N | PHE | A | 128 | -36.517 | 123.250 | -7.564 | 1.00 | 40.88 | GZ00 N |
| ATOM | 4286 | CA | PHE | A | 128 | -36.074 | 124.425 | -8.285 | 1.00 | 43.70 | GZ00 C |
| ATOM | 4287 | C | PHE | A | 128 | -36.857 | 124.590 | -9.578 | 1.00 | 46.15 | GZ00 C |
| ATOM | 4288 | O | PHE | A | 128 | -37.192 | 123.598 | -10.227 | 1.00 | 47.49 | GZ00 O |
| ATOM | 4289 | CB | PHE | A | 128 | -34.582 | 124.356 | -8.615 | 1.00 | 42.40 | GZ00 C |
| ATOM | 4290 | CG | PHE | A | 128 | -33.714 | 124.253 | -7.406 | 1.00 | 49.59 | GZ00 C |
| ATOM | 4291 | CD1 | PHE | A | 128 | -33.404 | 125.389 | -6.666 | 1.00 | 47.01 | GZ00 C |
| ATOM | 4292 | CD2 | PHE | A | 128 | -33.188 | 123.033 | -7.012 | 1.00 | 45.42 | GZ00 C |
| ATOM | 4293 | CE1 | PHE | A | 128 | -32.604 | 125.308 | -5.538 | 1.00 | 45.05 | GZ00 C |
| ATOM | 4294 | CE2 | PHE | A | 128 | -32.379 | 122.946 | -5.890 | 1.00 | 55.33 | GZ00 C |
| ATOM | 4295 | CZ | PHE | A | 128 | -32.084 | 124.085 | -5.152 | 1.00 | 51.39 | GZ00 C |
| ATOM | 4296 | N | PRO | A | 129 | -37.153 | 125.823 | -9.985 | 1.00 | 44.34 | GZ00 N |
| ATOM | 4297 | CA | PRO | A | 129 | -37.894 | 126.021 | -11.233 | 1.00 | 46.78 | GZ00 C |
| ATOM | 4298 | C | PRO | A | 129 | -36.975 | 125.867 | -12.426 | 1.00 | 45.16 | GZ00 C |
| ATOM | 4299 | O | PRO | A | 129 | -35.815 | 126.280 | -12.397 | 1.00 | 53.62 | GZ00 O |
| ATOM | 4300 | CB | PRO | A | 129 | -38.404 | 127.460 | -11.109 | 1.00 | 47.94 | GZ00 C |
| ATOM | 4301 | CG | PRO | A | 129 | -37.334 | 128.141 | -10.336 | 1.00 | 40.44 | GZ00 C |
| ATOM | 4302 | CD | PRO | A | 129 | -36.844 | 127.103 | -9.323 | 1.00 | 47.69 | GZ00 C |
| ATOM | 4303 | N | LEU | A | 130 | -37.486 | 125.227 | -13.465 | 1.00 | 44.60 | GZ00 N |
| ATOM | 4304 | CA | LEU | A | 130 | -36.794 | 125.147 | -14.749 | 1.00 | 49.12 | GZ00 C |
| ATOM | 4305 | C | LEU | A | 130 | -37.528 | 126.108 | -15.677 | 1.00 | 54.30 | GZ00 C |
| ATOM | 4306 | O | LEU | A | 130 | -38.580 | 125.769 | -16.225 | 1.00 | 53.88 | GZ00 O |
| ATOM | 4307 | CB | LEU | A | 130 | -36.764 | 123.714 | -15.283 | 1.00 | 52.10 | GZ00 C |
| ATOM | 4308 | CG | LEU | A | 130 | -36.085 | 122.707 | -14.338 | 1.00 | 49.99 | GZ00 C |
| ATOM | 4309 | CD1 | LEU | A | 130 | -36.064 | 121.284 | -14.876 | 1.00 | 44.72 | GZ00 C |
| ATOM | 4310 | CD2 | LEU | A | 130 | -34.670 | 123.167 | -14.003 | 1.00 | 49.22 | GZ00 C |
| ATOM | 4311 | N | ALA | A | 131 | -36.979 | 127.339 | -15.828 | 1.00 | 51.83 | GZ00 N |
| ATOM | 4312 | CA | ALA | A | 131 | -37.752 | 128.405 | -16.461 | 1.00 | 60.57 | GZ00 C |
| ATOM | 4313 | C | ALA | A | 131 | -37.777 | 128.241 | -17.979 | 1.00 | 58.44 | GZ00 C |
| ATOM | 4314 | O | ALA | A | 131 | -36.767 | 127.860 | -18.578 | 1.00 | 62.01 | GZ00 O |
| ATOM | 4315 | CB | ALA | A | 131 | -37.182 | 129.780 | -16.118 | 1.00 | 52.12 | GZ00 C |
| ATOM | 4316 | N | PRO | A | 132 | -38.922 | 128.508 | -18.610 | 1.00 | 63.89 | GZ00 N |
| ATOM | 4317 | CA | PRO | A | 132 | -38.974 | 128.551 | -20.076 | 1.00 | 60.84 | GZ00 C |
| ATOM | 4318 | C | PRO | A | 132 | -38.312 | 129.818 | -20.583 | 1.00 | 68.50 | GZ00 C |
| ATOM | 4319 | O | PRO | A | 132 | -38.605 | 130.917 | -20.107 | 1.00 | 78.71 | GZ00 O |
| ATOM | 4320 | CB | PRO | A | 132 | -40.477 | 128.546 | -20.374 | 1.00 | 61.98 | GZ00 C |
| ATOM | 4321 | CG | PRO | A | 132 | -41.076 | 129.218 | -19.186 | 1.00 | 59.91 | GZ00 C |
| ATOM | 4322 | CD | PRO | A | 132 | -40.239 | 128.787 | -18.005 | 1.00 | 64.04 | GZ00 C |
| ATOM | 4323 | N | SER | A | 133 | -37.403 | 129.660 | -21.536 | 1.00 | 78.25 | GZ00 N |
| ATOM | 4324 | CA | SER | A | 133 | -36.712 | 130.783 | -22.151 | 1.00 | 90.93 | GZ00 C |
| ATOM | 4325 | C | SER | A | 133 | -36.986 | 130.784 | -23.647 | 1.00 | 95.12 | GZ00 C |
| ATOM | 4326 | O | SER | A | 133 | -37.432 | 129.785 | -24.219 | 1.00 | 92.73 | GZ00 O |
| ATOM | 4327 | CB | SER | A | 133 | -35.195 | 130.741 | -21.878 | 1.00 | 93.56 | GZ00 C |

```
ATOM   4328  OG  SER A 133     -34.721 129.411 -21.727  1.00 92.59      GZ00 O
ATOM   4329  N   SER A 134     -36.724 131.923 -24.279  1.00101.62      GZ00 N
ATOM   4330  CA  SER A 134     -37.027 132.073 -25.696  1.00101.63      GZ00 C
ATOM   4331  C   SER A 134     -35.957 131.408 -26.549  1.00 98.00      GZ00 C
ATOM   4332  O   SER A 134     -36.000 130.197 -26.770  1.00105.90      GZ00 O
ATOM   4333  CB  SER A 134     -37.165 133.548 -26.069  1.00 96.91      GZ00 C
ATOM   4334  OG  SER A 134     -37.994 133.692 -27.209  1.00104.41      GZ00 O
ATOM   4335  N   GLY A 139     -42.971 131.608 -30.259  1.00100.95      GZ00 N
ATOM   4336  CA  GLY A 139     -44.001 130.908 -31.008  1.00110.26      GZ00 C
ATOM   4337  C   GLY A 139     -43.531 129.600 -31.626  1.00116.15      GZ00 C
ATOM   4338  O   GLY A 139     -42.529 129.576 -32.345  1.00129.42      GZ00 O
ATOM   4339  N   GLY A 140     -44.252 128.512 -31.352  1.00102.94      GZ00 N
ATOM   4340  CA  GLY A 140     -45.429 128.577 -30.509  1.00 98.08      GZ00 C
ATOM   4341  C   GLY A 140     -45.549 127.510 -29.435  1.00 93.20      GZ00 C
ATOM   4342  O   GLY A 140     -46.648 127.229 -28.960  1.00 88.91      GZ00 O
ATOM   4343  N   THR A 141     -44.427 126.906 -29.049  1.00 94.52      GZ00 N
ATOM   4344  CA  THR A 141     -44.434 125.891 -28.005  1.00 84.86      GZ00 C
ATOM   4345  C   THR A 141     -43.275 126.163 -27.053  1.00 82.99      GZ00 C
ATOM   4346  O   THR A 141     -42.191 126.566 -27.485  1.00 81.83      GZ00 O
ATOM   4347  CB  THR A 141     -44.353 124.474 -28.595  1.00 90.26      GZ00 C
ATOM   4348  OG1 THR A 141     -44.628 123.513 -27.566  1.00 85.35      GZ00 O
ATOM   4349  CG2 THR A 141     -42.971 124.203 -29.232  1.00 79.54      GZ00 C
ATOM   4350  N   ALA A 142     -43.506 125.953 -25.756  1.00 80.86      GZ00 N
ATOM   4351  CA  ALA A 142     -42.487 126.212 -24.745  1.00 77.11      GZ00 C
ATOM   4352  C   ALA A 142     -42.460 125.082 -23.731  1.00 70.08      GZ00 C
ATOM   4353  O   ALA A 142     -43.508 124.527 -23.382  1.00 69.86      GZ00 O
ATOM   4354  CB  ALA A 142     -42.731 127.539 -24.014  1.00 76.03      GZ00 C
ATOM   4355  N   ALA A 143     -41.262 124.748 -23.262  1.00 59.75      GZ00 N
ATOM   4356  CA  ALA A 143     -41.079 123.717 -22.248  1.00 63.56      GZ00 C
ATOM   4357  C   ALA A 143     -40.608 124.351 -20.947  1.00 58.18      GZ00 C
ATOM   4358  O   ALA A 143     -39.664 125.152 -20.948  1.00 54.35      GZ00 O
ATOM   4359  CB  ALA A 143     -40.072 122.666 -22.710  1.00 62.26      GZ00 C
ATOM   4360  N   LEU A 144     -41.241 123.961 -19.840  1.00 51.02      GZ00 N
ATOM   4361  CA  LEU A 144     -40.864 124.417 -18.507  1.00 54.73      GZ00 C
ATOM   4362  C   LEU A 144     -41.027 123.254 -17.537  1.00 53.32      GZ00 C
ATOM   4363  O   LEU A 144     -41.753 122.293 -17.812  1.00 53.18      GZ00 O
ATOM   4364  CB  LEU A 144     -41.709 125.621 -18.047  1.00 46.73      GZ00 C
ATOM   4365  CG  LEU A 144     -43.215 125.351 -17.951  1.00 56.02      GZ00 C
ATOM   4366  CD1 LEU A 144     -43.665 125.025 -16.521  1.00 52.68      GZ00 C
ATOM   4367  CD2 LEU A 144     -44.014 126.520 -18.513  1.00 58.59      GZ00 C
ATOM   4368  N   GLY A 145     -40.359 123.341 -16.391  1.00 51.88      GZ00 N
ATOM   4369  CA  GLY A 145     -40.399 122.209 -15.497  1.00 48.27      GZ00 C
ATOM   4370  C   GLY A 145     -39.960 122.514 -14.085  1.00 51.16      GZ00 C
ATOM   4371  O   GLY A 145     -39.791 123.673 -13.698  1.00 53.82      GZ00 O
ATOM   4372  N   CYS A 146     -39.798 121.434 -13.315  1.00 44.69      GZ00 N
ATOM   4373  CA  CYS A 146     -39.413 121.470 -11.910  1.00 47.10      GZ00 C
ATOM   4374  C   CYS A 146     -38.297 120.462 -11.680  1.00 45.37      GZ00 C
ATOM   4375  O   CYS A 146     -38.433 119.290 -12.038  1.00 42.62      GZ00 O
ATOM   4376  CB  CYS A 146     -40.597 121.139 -10.990  1.00 47.73      GZ00 C
ATOM   4377  SG  CYS A 146     -41.531 122.590 -10.443  1.00 76.23      GZ00 S
ATOM   4378  N   LEU A 147     -37.207 120.910 -11.080  1.00 47.76      GZ00 N
ATOM   4379  CA  LEU A 147     -36.102 120.037 -10.725  1.00 40.76      GZ00 C
ATOM   4380  C   LEU A 147     -36.281 119.619  -9.279  1.00 43.78      GZ00 C
ATOM   4381  O   LEU A 147     -36.273 120.470  -8.388  1.00 44.48      GZ00 O
ATOM   4382  CB  LEU A 147     -34.767 120.742 -10.938  1.00 37.72      GZ00 C
ATOM   4383  CG  LEU A 147     -33.513 120.047 -10.400  1.00 47.51      GZ00 C
ATOM   4384  CD1 LEU A 147     -33.360 118.653 -10.996  1.00 40.15      GZ00 C
ATOM   4385  CD2 LEU A 147     -32.260 120.918 -10.669  1.00 37.25      GZ00 C
ATOM   4386  N   VAL A 148     -36.396 118.311  -9.046  1.00 48.64      GZ00 N
ATOM   4387  CA  VAL A 148     -36.699 117.743  -7.733  1.00 46.35      GZ00 C
ATOM   4388  C   VAL A 148     -35.441 117.031  -7.252  1.00 49.34      GZ00 C
ATOM   4389  O   VAL A 148     -35.200 115.861  -7.576  1.00 50.72      GZ00 O
ATOM   4390  CB  VAL A 148     -37.896 116.788  -7.790  1.00 38.94      GZ00 C
ATOM   4391  CG1 VAL A 148     -38.234 116.266  -6.410  1.00 40.39      GZ00 C
ATOM   4392  CG2 VAL A 148     -39.087 117.487  -8.380  1.00 40.04      GZ00 C
ATOM   4393  N   LYS A 149     -34.660 117.721  -6.440  1.00 45.78      GZ00 N
ATOM   4394  CA  LYS A 149     -33.291 117.334  -6.155  1.00 45.80      GZ00 C
ATOM   4395  C   LYS A 149     -33.137 116.710  -4.774  1.00 47.10      GZ00 C
ATOM   4396  O   LYS A 149     -33.791 117.126  -3.815  1.00 47.43      GZ00 O
ATOM   4397  CB  LYS A 149     -32.379 118.554  -6.266  1.00 47.24      GZ00 C
ATOM   4398  CG  LYS A 149     -31.174 118.293  -7.097  1.00 56.69      GZ00 C
```

| | | | | | | | | | | | | |
|---|---|---|---|---|---|---|---|---|---|---|---|---|
| ATOM | 4399 | CD | LYS | A | 149 | -29.975 | 119.004 | -6.564 | 1.00 | 59.82 | GZ00 | C |
| ATOM | 4400 | CE | LYS | A | 149 | -30.099 | 120.481 | -6.760 | 1.00 | 57.45 | GZ00 | C |
| ATOM | 4401 | NZ | LYS | A | 149 | -28.781 | 121.117 | -6.434 | 1.00 | 60.94 | GZ00 | N1+ |
| ATOM | 4402 | N | ASP | A | 150 | -32.254 | 115.710 | -4.694 | 1.00 | 49.92 | GZ00 | N |
| ATOM | 4403 | CA | ASP | A | 150 | -31.630 | 115.231 | -3.456 | 1.00 | 45.59 | GZ00 | C |
| ATOM | 4404 | C | ASP | A | 150 | -32.643 | 114.736 | -2.423 | 1.00 | 44.92 | GZ00 | C |
| ATOM | 4405 | O | ASP | A | 150 | -32.679 | 115.208 | -1.289 | 1.00 | 51.88 | GZ00 | O |
| ATOM | 4406 | CB | ASP | A | 150 | -30.741 | 116.314 | -2.838 | 1.00 | 42.77 | GZ00 | C |
| ATOM | 4407 | CG | ASP | A | 150 | -29.533 | 116.637 | -3.689 | 1.00 | 56.92 | GZ00 | C |
| ATOM | 4408 | OD1 | ASP | A | 150 | -29.136 | 115.782 | -4.512 | 1.00 | 57.01 | GZ00 | O |
| ATOM | 4409 | OD2 | ASP | A | 150 | -28.970 | 117.747 | -3.525 | 1.00 | 62.51 | GZ00 | O1- |
| ATOM | 4410 | N | TYR | A | 151 | -33.415 | 113.725 | -2.802 | 1.00 | 45.00 | GZ00 | N |
| ATOM | 4411 | CA | TYR | A | 151 | -34.367 | 113.102 | -1.894 | 1.00 | 49.20 | GZ00 | C |
| ATOM | 4412 | C | TYR | A | 151 | -34.088 | 111.606 | -1.765 | 1.00 | 57.02 | GZ00 | C |
| ATOM | 4413 | O | TYR | A | 151 | -33.356 | 111.009 | -2.563 | 1.00 | 54.16 | GZ00 | O |
| ATOM | 4414 | CB | TYR | A | 151 | -35.807 | 113.316 | -2.346 | 1.00 | 41.84 | GZ00 | C |
| ATOM | 4415 | CG | TYR | A | 151 | -36.146 | 112.630 | -3.642 | 1.00 | 53.39 | GZ00 | C |
| ATOM | 4416 | CD1 | TYR | A | 151 | -36.579 | 111.294 | -3.672 | 1.00 | 58.67 | GZ00 | C |
| ATOM | 4417 | CD2 | TYR | A | 151 | -36.045 | 113.316 | -4.845 | 1.00 | 47.72 | GZ00 | C |
| ATOM | 4418 | CE1 | TYR | A | 151 | -36.898 | 110.679 | -4.879 | 1.00 | 55.52 | GZ00 | C |
| ATOM | 4419 | CE2 | TYR | A | 151 | -36.365 | 112.718 | -6.041 | 1.00 | 47.90 | GZ00 | C |
| ATOM | 4420 | CZ | TYR | A | 151 | -36.782 | 111.403 | -6.063 | 1.00 | 54.44 | GZ00 | C |
| ATOM | 4421 | OH | TYR | A | 151 | -37.077 | 110.834 | -7.286 | 1.00 | 56.96 | GZ00 | O |
| ATOM | 4422 | N | PHE | A | 152 | -34.688 | 111.011 | -0.737 | 1.00 | 52.17 | GZ00 | N |
| ATOM | 4423 | CA | PHE | A | 152 | -34.494 | 109.613 | -0.424 | 1.00 | 56.95 | GZ00 | C |
| ATOM | 4424 | C | PHE | A | 152 | -35.543 | 109.170 | 0.575 | 1.00 | 59.21 | GZ00 | C |
| ATOM | 4425 | O | PHE | A | 152 | -35.877 | 109.929 | 1.472 | 1.00 | 56.82 | GZ00 | O |
| ATOM | 4426 | CB | PHE | A | 152 | -33.097 | 109.382 | 0.144 | 1.00 | 56.68 | GZ00 | C |
| ATOM | 4427 | CG | PHE | A | 152 | -32.764 | 107.946 | 0.349 | 1.00 | 56.75 | GZ00 | C |
| ATOM | 4428 | CD1 | PHE | A | 152 | -33.106 | 107.301 | 1.528 | 1.00 | 57.37 | GZ00 | C |
| ATOM | 4429 | CD2 | PHE | A | 152 | -32.097 | 107.233 | -0.640 | 1.00 | 61.45 | GZ00 | C |
| ATOM | 4430 | CE1 | PHE | A | 152 | -32.796 | 105.963 | 1.718 | 1.00 | 62.30 | GZ00 | C |
| ATOM | 4431 | CE2 | PHE | A | 152 | -31.784 | 105.899 | -0.457 | 1.00 | 54.88 | GZ00 | C |
| ATOM | 4432 | CZ | PHE | A | 152 | -32.136 | 105.258 | 0.722 | 1.00 | 52.68 | GZ00 | C |
| ATOM | 4433 | N | PRO | A | 153 | -36.081 | 107.947 | 0.414 | 1.00 | 64.15 | GZ00 | N |
| ATOM | 4434 | CA | PRO | A | 153 | -35.890 | 107.052 | -0.728 | 1.00 | 60.57 | GZ00 | C |
| ATOM | 4435 | C | PRO | A | 153 | -36.949 | 107.343 | -1.784 | 1.00 | 57.01 | GZ00 | C |
| ATOM | 4436 | O | PRO | A | 153 | -37.736 | 108.261 | -1.579 | 1.00 | 57.18 | GZ00 | O |
| ATOM | 4437 | CB | PRO | A | 153 | -36.104 | 105.684 | -0.109 | 1.00 | 56.69 | GZ00 | C |
| ATOM | 4438 | CG | PRO | A | 153 | -37.250 | 105.962 | 0.843 | 1.00 | 50.72 | GZ00 | C |
| ATOM | 4439 | CD | PRO | A | 153 | -36.942 | 107.317 | 1.434 | 1.00 | 59.37 | GZ00 | C |
| ATOM | 4440 | N | GLU | A | 154 | -37.022 | 106.550 | -2.848 | 1.00 | 55.69 | GZ00 | N |
| ATOM | 4441 | CA | GLU | A | 154 | -38.086 | 106.727 | -3.833 | 1.00 | 52.35 | GZ00 | C |
| ATOM | 4442 | C | GLU | A | 154 | -39.389 | 106.318 | -3.182 | 1.00 | 47.39 | GZ00 | C |
| ATOM | 4443 | O | GLU | A | 154 | -39.364 | 105.619 | -2.171 | 1.00 | 52.96 | GZ00 | O |
| ATOM | 4444 | CB | GLU | A | 154 | -37.805 | 105.905 | -5.093 | 1.00 | 53.56 | GZ00 | C |
| ATOM | 4445 | CG | GLU | A | 154 | -36.530 | 106.322 | -5.818 | 1.00 | 58.65 | GZ00 | C |
| ATOM | 4446 | CD | GLU | A | 154 | -36.727 | 106.415 | -7.315 | 1.00 | 67.26 | GZ00 | C |
| ATOM | 4447 | OE1 | GLU | A | 154 | -36.043 | 105.662 | -8.041 | 1.00 | 74.85 | GZ00 | O |
| ATOM | 4448 | OE2 | GLU | A | 154 | -37.572 | 107.227 | -7.765 | 1.00 | 66.72 | GZ00 | O1- |
| ATOM | 4449 | N | PRO | A | 155 | -40.533 | 106.779 | -3.713 | 1.00 | 45.32 | GZ00 | N |
| ATOM | 4450 | CA | PRO | A | 155 | -40.792 | 107.703 | -4.827 | 1.00 | 55.68 | GZ00 | C |
| ATOM | 4451 | C | PRO | A | 155 | -41.144 | 109.144 | -4.437 | 1.00 | 57.15 | GZ00 | C |
| ATOM | 4452 | O | PRO | A | 155 | -41.413 | 109.427 | -3.272 | 1.00 | 61.59 | GZ00 | O |
| ATOM | 4453 | CB | PRO | A | 155 | -42.020 | 107.086 | -5.474 | 1.00 | 54.31 | GZ00 | C |
| ATOM | 4454 | CG | PRO | A | 155 | -42.813 | 106.627 | -4.281 | 1.00 | 36.78 | GZ00 | C |
| ATOM | 4455 | CD | PRO | A | 155 | -41.792 | 106.177 | -3.235 | 1.00 | 36.78 | GZ00 | C |
| ATOM | 4456 | N | VAL | A | 156 | -41.135 | 110.047 | -5.417 | 1.00 | 56.20 | GZ00 | N |
| ATOM | 4457 | CA | VAL | A | 156 | -41.884 | 111.294 | -5.339 | 1.00 | 56.13 | GZ00 | C |
| ATOM | 4458 | C | VAL | A | 156 | -42.962 | 111.242 | -6.407 | 1.00 | 55.59 | GZ00 | C |
| ATOM | 4459 | O | VAL | A | 156 | -42.818 | 110.570 | -7.432 | 1.00 | 66.78 | GZ00 | O |
| ATOM | 4460 | CB | VAL | A | 156 | -41.023 | 112.567 | -5.514 | 1.00 | 57.46 | GZ00 | C |
| ATOM | 4461 | CG1 | VAL | A | 156 | -40.008 | 112.698 | -4.399 | 1.00 | 56.24 | GZ00 | C |
| ATOM | 4462 | CG2 | VAL | A | 156 | -40.327 | 112.555 | -6.838 | 1.00 | 58.46 | GZ00 | C |
| ATOM | 4463 | N | THR | A | 157 | -44.052 | 111.949 | -6.156 | 1.00 | 55.63 | GZ00 | N |
| ATOM | 4464 | CA | THR | A | 157 | -45.091 | 112.189 | -7.145 | 1.00 | 48.48 | GZ00 | C |
| ATOM | 4465 | C | THR | A | 157 | -45.079 | 113.673 | -7.480 | 1.00 | 56.97 | GZ00 | C |
| ATOM | 4466 | O | THR | A | 157 | -44.819 | 114.506 | -6.606 | 1.00 | 54.54 | GZ00 | O |
| ATOM | 4467 | CB | THR | A | 157 | -46.464 | 111.777 | -6.608 | 1.00 | 58.63 | GZ00 | C |
| ATOM | 4468 | OG1 | THR | A | 157 | -46.837 | 112.659 | -5.542 | 1.00 | 69.16 | GZ00 | O |
| ATOM | 4469 | CG2 | THR | A | 157 | -46.413 | 110.369 | -6.046 | 1.00 | 52.02 | GZ00 | C |

| | | | | | | | | | | | | |
|---|---|---|---|---|---|---|---|---|---|---|---|---|
| ATOM | 4470 | N | VAL | A | 158 | -45.317 | 114.003 | -8.747 | 1.00 | 52.48 | GZ00 | N |
| ATOM | 4471 | CA | VAL | A | 158 | -45.344 | 115.385 | -9.209 | 1.00 | 49.55 | GZ00 | C |
| ATOM | 4472 | C | VAL | A | 158 | -46.619 | 115.606 | -10.001 | 1.00 | 53.68 | GZ00 | C |
| ATOM | 4473 | O | VAL | A | 158 | -46.942 | 114.809 | -10.885 | 1.00 | 58.24 | GZ00 | O |
| ATOM | 4474 | CB | VAL | A | 158 | -44.131 | 115.743 | -10.087 | 1.00 | 53.34 | GZ00 | C |
| ATOM | 4475 | CG1 | VAL | A | 158 | -44.181 | 117.245 | -10.447 | 1.00 | 47.23 | GZ00 | C |
| ATOM | 4476 | CG2 | VAL | A | 158 | -42.819 | 115.357 | -9.407 | 1.00 | 50.02 | GZ00 | C |
| ATOM | 4477 | N | SER | A | 159 | -47.337 | 116.684 | -9.693 | 1.00 | 53.96 | GZ00 | N |
| ATOM | 4478 | CA | SER | A | 159 | -48.485 | 117.098 | -10.485 | 1.00 | 54.02 | GZ00 | C |
| ATOM | 4479 | C | SER | A | 159 | -48.334 | 118.563 | -10.856 | 1.00 | 57.99 | GZ00 | C |
| ATOM | 4480 | O | SER | A | 159 | -47.487 | 119.282 | -10.319 | 1.00 | 58.35 | GZ00 | O |
| ATOM | 4481 | CB | SER | A | 159 | -49.802 | 116.870 | -9.741 | 1.00 | 57.03 | GZ00 | C |
| ATOM | 4482 | OG | SER | A | 159 | -49.841 | 117.623 | -8.548 | 1.00 | 64.29 | GZ00 | O |
| ATOM | 4483 | N | TRP | A | 160 | -49.143 | 119.008 | -11.804 | 1.00 | 52.93 | GZ00 | N |
| ATOM | 4484 | CA | TRP | A | 160 | -49.127 | 120.403 | -12.198 | 1.00 | 53.69 | GZ00 | C |
| ATOM | 4485 | C | TRP | A | 160 | -50.507 | 120.996 | -11.965 | 1.00 | 60.22 | GZ00 | C |
| ATOM | 4486 | O | TRP | A | 160 | -51.524 | 120.353 | -12.254 | 1.00 | 56.05 | GZ00 | O |
| ATOM | 4487 | CB | TRP | A | 160 | -48.667 | 120.551 | -13.654 | 1.00 | 52.01 | GZ00 | C |
| ATOM | 4488 | CG | TRP | A | 160 | -47.199 | 120.239 | -13.791 | 1.00 | 61.18 | GZ00 | C |
| ATOM | 4489 | CD1 | TRP | A | 160 | -46.630 | 119.002 | -13.982 | 1.00 | 51.63 | GZ00 | C |
| ATOM | 4490 | CD2 | TRP | A | 160 | -46.108 | 121.169 | -13.703 | 1.00 | 51.85 | GZ00 | C |
| ATOM | 4491 | NE1 | TRP | A | 160 | -45.260 | 119.116 | -14.035 | 1.00 | 51.65 | GZ00 | N |
| ATOM | 4492 | CE2 | TRP | A | 160 | -44.914 | 120.433 | -13.871 | 1.00 | 55.17 | GZ00 | C |
| ATOM | 4493 | CE3 | TRP | A | 160 | -46.025 | 122.550 | -13.513 | 1.00 | 49.96 | GZ00 | C |
| ATOM | 4494 | CZ2 | TRP | A | 160 | -43.654 | 121.035 | -13.847 | 1.00 | 52.75 | GZ00 | C |
| ATOM | 4495 | CZ3 | TRP | A | 160 | -44.772 | 123.148 | -13.502 | 1.00 | 55.98 | GZ00 | C |
| ATOM | 4496 | CH2 | TRP | A | 160 | -43.604 | 122.389 | -13.663 | 1.00 | 51.25 | GZ00 | C |
| ATOM | 4497 | N | ASN | A | 161 | -50.521 | 122.215 | -11.417 | 1.00 | 59.30 | GZ00 | N |
| ATOM | 4498 | CA | ASN | A | 161 | -51.738 | 122.946 | -11.048 | 1.00 | 53.77 | GZ00 | C |
| ATOM | 4499 | C | ASN | A | 161 | -52.737 | 122.039 | -10.336 | 1.00 | 58.01 | GZ00 | C |
| ATOM | 4500 | O | ASN | A | 161 | -53.929 | 122.014 | -10.642 | 1.00 | 64.68 | GZ00 | O |
| ATOM | 4501 | CB | ASN | A | 161 | -52.353 | 123.615 | -12.267 | 1.00 | 42.97 | GZ00 | C |
| ATOM | 4502 | CG | ASN | A | 161 | -51.430 | 124.645 | -12.851 | 1.00 | 59.88 | GZ00 | C |
| ATOM | 4503 | OD1 | ASN | A | 161 | -50.421 | 124.988 | -12.231 | 1.00 | 57.52 | GZ00 | O |
| ATOM | 4504 | ND2 | ASN | A | 161 | -51.743 | 125.141 | -14.043 | 1.00 | 62.17 | GZ00 | N |
| ATOM | 4505 | N | SER | A | 162 | -52.218 | 121.280 | -9.367 | 1.00 | 60.34 | GZ00 | N |
| ATOM | 4506 | CA | SER | A | 162 | -53.022 | 120.430 | -8.483 | 1.00 | 66.27 | GZ00 | C |
| ATOM | 4507 | C | SER | A | 162 | -53.790 | 119.353 | -9.244 | 1.00 | 71.65 | GZ00 | C |
| ATOM | 4508 | O | SER | A | 162 | -54.873 | 118.941 | -8.821 | 1.00 | 79.00 | GZ00 | O |
| ATOM | 4509 | CB | SER | A | 162 | -53.984 | 121.273 | -7.641 | 1.00 | 67.29 | GZ00 | C |
| ATOM | 4510 | OG | SER | A | 162 | -53.287 | 122.322 | -6.987 | 1.00 | 70.91 | GZ00 | O |
| ATOM | 4511 | N | GLY | A | 163 | -53.224 | 118.858 | -10.349 | 1.00 | 70.85 | GZ00 | N |
| ATOM | 4512 | CA | GLY | A | 163 | -53.876 | 117.868 | -11.181 | 1.00 | 59.17 | GZ00 | C |
| ATOM | 4513 | C | GLY | A | 163 | -54.598 | 118.443 | -12.385 | 1.00 | 68.56 | GZ00 | C |
| ATOM | 4514 | O | GLY | A | 163 | -55.017 | 117.675 | -13.255 | 1.00 | 76.49 | GZ00 | O |
| ATOM | 4515 | N | ALA | A | 164 | -54.744 | 119.772 | -12.463 | 1.00 | 67.36 | GZ00 | N |
| ATOM | 4516 | CA | ALA | A | 164 | -55.483 | 120.392 | -13.561 | 1.00 | 63.96 | GZ00 | C |
| ATOM | 4517 | C | ALA | A | 164 | -54.755 | 120.239 | -14.885 | 1.00 | 68.34 | GZ00 | C |
| ATOM | 4518 | O | ALA | A | 164 | -55.391 | 120.060 | -15.927 | 1.00 | 75.07 | GZ00 | O |
| ATOM | 4519 | CB | ALA | A | 164 | -55.712 | 121.879 | -13.280 | 1.00 | 57.29 | GZ00 | C |
| ATOM | 4520 | N | LEU | A | 165 | -53.431 | 120.361 | -14.874 | 1.00 | 70.33 | GZ00 | N |
| ATOM | 4521 | CA | LEU | A | 165 | -52.620 | 120.265 | -16.082 | 1.00 | 64.91 | GZ00 | C |
| ATOM | 4522 | C | LEU | A | 165 | -52.055 | 118.846 | -16.163 | 1.00 | 69.76 | GZ00 | C |
| ATOM | 4523 | O | LEU | A | 165 | -51.263 | 118.438 | -15.303 | 1.00 | 66.93 | GZ00 | O |
| ATOM | 4524 | CB | LEU | A | 165 | -51.521 | 121.324 | -16.062 | 1.00 | 64.77 | GZ00 | C |
| ATOM | 4525 | CG | LEU | A | 165 | -50.556 | 121.397 | -17.245 | 1.00 | 68.60 | GZ00 | C |
| ATOM | 4526 | CD1 | LEU | A | 165 | -51.313 | 121.483 | -18.560 | 1.00 | 68.89 | GZ00 | C |
| ATOM | 4527 | CD2 | LEU | A | 165 | -49.654 | 122.612 | -17.083 | 1.00 | 68.22 | GZ00 | C |
| ATOM | 4528 | N | THR | A | 166 | -52.506 | 118.076 | -17.164 | 1.00 | 75.54 | GZ00 | N |
| ATOM | 4529 | CA | THR | A | 166 | -52.042 | 116.700 | -17.363 | 1.00 | 72.10 | GZ00 | C |
| ATOM | 4530 | C | THR | A | 166 | -51.554 | 116.504 | -18.792 | 1.00 | 68.22 | GZ00 | C |
| ATOM | 4531 | O | THR | A | 166 | -50.624 | 115.732 | -19.042 | 1.00 | 71.78 | GZ00 | O |
| ATOM | 4532 | CB | THR | A | 166 | -53.133 | 115.662 | -17.055 | 1.00 | 67.33 | GZ00 | C |
| ATOM | 4533 | OG1 | THR | A | 166 | -54.242 | 115.850 | -17.938 | 1.00 | 72.47 | GZ00 | O |
| ATOM | 4534 | CG2 | THR | A | 166 | -53.609 | 115.751 | -15.608 | 1.00 | 70.69 | GZ00 | C |
| ATOM | 4535 | N | SER | A | 167 | -52.193 | 117.175 | -19.742 | 1.00 | 70.73 | GZ00 | N |
| ATOM | 4536 | CA | SER | A | 167 | -51.742 | 117.096 | -21.121 | 1.00 | 77.77 | GZ00 | C |
| ATOM | 4537 | C | SER | A | 167 | -50.357 | 117.710 | -21.242 | 1.00 | 66.92 | GZ00 | C |
| ATOM | 4538 | O | SER | A | 167 | -50.104 | 118.796 | -20.717 | 1.00 | 69.96 | GZ00 | O |
| ATOM | 4539 | CB | SER | A | 167 | -52.734 | 117.819 | -22.039 | 1.00 | 74.39 | GZ00 | C |
| ATOM | 4540 | OG | SER | A | 167 | -52.114 | 118.270 | -23.233 | 1.00 | 68.17 | GZ00 | O |

| | | | | | | | | | | | | |
|---|---|---|---|---|---|---|---|---|---|---|---|---|
| ATOM | 4541 | N | GLY | A | 168 | -49.455 | 117.012 | -21.932 | 1.00 | 64.19 | GZ00 | N |
| ATOM | 4542 | CA | GLY | A | 168 | -48.125 | 117.554 | -22.141 | 1.00 | 62.50 | GZ00 | C |
| ATOM | 4543 | C | GLY | A | 168 | -47.169 | 117.414 | -20.977 | 1.00 | 58.51 | GZ00 | C |
| ATOM | 4544 | O | GLY | A | 168 | -46.115 | 118.053 | -20.994 | 1.00 | 54.75 | GZ00 | O |
| ATOM | 4545 | N | VAL | A | 169 | -47.512 | 116.619 | -19.963 | 1.00 | 59.87 | GZ00 | N |
| ATOM | 4546 | CA | VAL | A | 169 | -46.709 | 116.442 | -18.757 | 1.00 | 53.22 | GZ00 | C |
| ATOM | 4547 | C | VAL | A | 169 | -45.837 | 115.206 | -18.906 | 1.00 | 57.24 | GZ00 | C |
| ATOM | 4548 | O | VAL | A | 169 | -46.341 | 114.121 | -19.219 | 1.00 | 54.95 | GZ00 | O |
| ATOM | 4549 | CB | VAL | A | 169 | -47.605 | 116.322 | -17.514 | 1.00 | 52.90 | GZ00 | C |
| ATOM | 4550 | CG1 | VAL | A | 169 | -46.789 | 115.872 | -16.311 | 1.00 | 50.21 | GZ00 | C |
| ATOM | 4551 | CG2 | VAL | A | 169 | -48.279 | 117.657 | -17.237 | 1.00 | 56.97 | GZ00 | C |
| ATOM | 4552 | N | HIS | A | 170 | -44.533 | 115.362 | -18.656 | 1.00 | 49.30 | GZ00 | N |
| ATOM | 4553 | CA | HIS | A | 170 | -43.571 | 114.263 | -18.742 | 1.00 | 46.23 | GZ00 | C |
| ATOM | 4554 | C | HIS | A | 170 | -42.653 | 114.312 | -17.519 | 1.00 | 45.18 | GZ00 | C |
| ATOM | 4555 | O | HIS | A | 170 | -41.702 | 115.095 | -17.482 | 1.00 | 48.42 | GZ00 | O |
| ATOM | 4556 | CB | HIS | A | 170 | -42.786 | 114.360 | -20.050 | 1.00 | 46.88 | GZ00 | C |
| ATOM | 4557 | CG | HIS | A | 170 | -41.995 | 113.136 | -20.400 | 1.00 | 50.83 | GZ00 | C |
| ATOM | 4558 | ND1 | HIS | A | 170 | -41.756 | 112.109 | -19.507 | 1.00 | 51.60 | GZ00 | N |
| ATOM | 4559 | CD2 | HIS | A | 170 | -41.374 | 112.784 | -21.551 | 1.00 | 45.43 | GZ00 | C |
| ATOM | 4560 | CE1 | HIS | A | 170 | -41.020 | 111.180 | -20.092 | 1.00 | 44.58 | GZ00 | C |
| ATOM | 4561 | NE2 | HIS | A | 170 | -40.776 | 111.565 | -21.332 | 1.00 | 49.60 | GZ00 | N |
| ATOM | 4562 | N | THR | A | 171 | -42.927 | 113.466 | -16.529 | 1.00 | 43.26 | GZ00 | N |
| ATOM | 4563 | CA | THR | A | 171 | -42.052 | 113.277 | -15.380 | 1.00 | 42.92 | GZ00 | C |
| ATOM | 4564 | C | THR | A | 171 | -41.082 | 112.128 | -15.662 | 1.00 | 43.51 | GZ00 | C |
| ATOM | 4565 | O | THR | A | 171 | -41.505 | 111.018 | -15.974 | 1.00 | 42.46 | GZ00 | O |
| ATOM | 4566 | CB | THR | A | 171 | -42.883 | 113.022 | -14.124 | 1.00 | 37.89 | GZ00 | C |
| ATOM | 4567 | OG1 | THR | A | 171 | -43.681 | 114.176 | -13.878 | 1.00 | 47.19 | GZ00 | O |
| ATOM | 4568 | CG2 | THR | A | 171 | -42.006 | 112.776 | -12.906 | 1.00 | 32.66 | GZ00 | C |
| ATOM | 4569 | N | PHE | A | 172 | -39.793 | 112.421 | -15.634 | 1.00 | 46.02 | GZ00 | N |
| ATOM | 4570 | CA | PHE | A | 172 | -38.722 | 111.494 | -15.965 | 1.00 | 45.07 | GZ00 | C |
| ATOM | 4571 | C | PHE | A | 172 | -38.336 | 110.651 | -14.754 | 1.00 | 49.93 | GZ00 | C |
| ATOM | 4572 | O | PHE | A | 172 | -38.364 | 111.141 | -13.628 | 1.00 | 51.84 | GZ00 | O |
| ATOM | 4573 | CB | PHE | A | 172 | -37.501 | 112.256 | -16.475 | 1.00 | 38.72 | GZ00 | C |
| ATOM | 4574 | CG | PHE | A | 172 | -37.680 | 112.793 | -17.855 | 1.00 | 43.18 | GZ00 | C |
| ATOM | 4575 | CD1 | PHE | A | 172 | -38.599 | 113.810 | -18.104 | 1.00 | 42.98 | GZ00 | C |
| ATOM | 4576 | CD2 | PHE | A | 172 | -36.942 | 112.277 | -18.917 | 1.00 | 40.18 | GZ00 | C |
| ATOM | 4577 | CE1 | PHE | A | 172 | -38.782 | 114.304 | -19.389 | 1.00 | 43.59 | GZ00 | C |
| ATOM | 4578 | CE2 | PHE | A | 172 | -37.108 | 112.779 | -20.216 | 1.00 | 44.39 | GZ00 | C |
| ATOM | 4579 | CZ | PHE | A | 172 | -38.030 | 113.797 | -20.449 | 1.00 | 45.10 | GZ00 | C |
| ATOM | 4580 | N | PRO | A | 173 | -37.971 | 109.388 | -14.970 | 1.00 | 45.23 | GZ00 | N |
| ATOM | 4581 | CA | PRO | A | 173 | -37.427 | 108.573 | -13.877 | 1.00 | 46.41 | GZ00 | C |
| ATOM | 4582 | C | PRO | A | 173 | -36.181 | 109.215 | -13.275 | 1.00 | 46.24 | GZ00 | C |
| ATOM | 4583 | O | PRO | A | 173 | -35.412 | 109.890 | -13.963 | 1.00 | 49.60 | GZ00 | O |
| ATOM | 4584 | CB | PRO | A | 173 | -37.083 | 107.243 | -14.568 | 1.00 | 43.84 | GZ00 | C |
| ATOM | 4585 | CG | PRO | A | 173 | -37.966 | 107.212 | -15.777 | 1.00 | 42.54 | GZ00 | C |
| ATOM | 4586 | CD | PRO | A | 173 | -38.103 | 108.633 | -16.230 | 1.00 | 42.67 | GZ00 | C |
| ATOM | 4587 | N | ALA | A | 174 | -35.971 | 108.971 | -11.983 | 1.00 | 43.82 | GZ00 | N |
| ATOM | 4588 | CA | ALA | A | 174 | -34.906 | 109.615 | -11.225 | 1.00 | 43.70 | GZ00 | C |
| ATOM | 4589 | C | ALA | A | 174 | -33.520 | 109.033 | -11.547 | 1.00 | 43.64 | GZ00 | C |
| ATOM | 4590 | O | ALA | A | 174 | -33.392 | 107.905 | -12.020 | 1.00 | 48.77 | GZ00 | O |
| ATOM | 4591 | CB | ALA | A | 174 | -35.195 | 109.486 | -9.733 | 1.00 | 46.19 | GZ00 | C |
| ATOM | 4592 | N | VAL | A | 175 | -32.460 | 109.857 | -11.304 | 1.00 | 40.72 | GZ00 | N |
| ATOM | 4593 | CA | VAL | A | 175 | -31.066 | 109.414 | -11.216 | 1.00 | 40.07 | GZ00 | C |
| ATOM | 4594 | C | VAL | A | 175 | -30.766 | 109.037 | -9.779 | 1.00 | 49.10 | GZ00 | C |
| ATOM | 4595 | O | VAL | A | 175 | -31.260 | 109.680 | -8.847 | 1.00 | 49.26 | GZ00 | O |
| ATOM | 4596 | CB | VAL | A | 175 | -30.053 | 110.495 | -11.665 | 1.00 | 47.05 | GZ00 | C |
| ATOM | 4597 | CG1 | VAL | A | 175 | -29.752 | 110.424 | -13.122 | 1.00 | 49.41 | GZ00 | C |
| ATOM | 4598 | CG2 | VAL | A | 175 | -30.479 | 111.906 | -11.241 | 1.00 | 43.16 | GZ00 | C |
| ATOM | 4599 | N | LEU | A | 176 | -29.930 | 108.011 | -9.593 | 1.00 | 45.95 | GZ00 | N |
| ATOM | 4600 | CA | LEU | A | 176 | -29.317 | 107.724 | -8.305 | 1.00 | 45.89 | GZ00 | C |
| ATOM | 4601 | C | LEU | A | 176 | -27.916 | 108.315 | -8.322 | 1.00 | 50.82 | GZ00 | C |
| ATOM | 4602 | O | LEU | A | 176 | -27.048 | 107.853 | -9.066 | 1.00 | 64.34 | GZ00 | O |
| ATOM | 4603 | CB | LEU | A | 176 | -29.278 | 106.231 | -8.012 | 1.00 | 50.15 | GZ00 | C |
| ATOM | 4604 | CG | LEU | A | 176 | -28.524 | 105.913 | -6.714 | 1.00 | 55.35 | GZ00 | C |
| ATOM | 4605 | CD1 | LEU | A | 176 | -29.054 | 106.726 | -5.525 | 1.00 | 46.25 | GZ00 | C |
| ATOM | 4606 | CD2 | LEU | A | 176 | -28.546 | 104.409 | -6.408 | 1.00 | 46.32 | GZ00 | C |
| ATOM | 4607 | N | GLN | A | 177 | -27.691 | 109.322 | -7.492 | 1.00 | 52.50 | GZ00 | N |
| ATOM | 4608 | CA | GLN | A | 177 | -26.422 | 110.026 | -7.518 | 1.00 | 63.27 | GZ00 | C |
| ATOM | 4609 | C | GLN | A | 177 | -25.368 | 109.285 | -6.697 | 1.00 | 56.88 | GZ00 | C |
| ATOM | 4610 | O | GLN | A | 177 | -25.678 | 108.456 | -5.831 | 1.00 | 51.21 | GZ00 | O |
| ATOM | 4611 | CB | GLN | A | 177 | -26.600 | 111.448 | -6.985 | 1.00 | 64.89 | GZ00 | C |

| | | | | | | | | | | | | |
|---|---|---|---|---|---|---|---|---|---|---|---|---|
| ATOM | 4612 | CG | GLN | A | 177 | -27.646 | 112.247 | -7.732 | 1.00 | 67.73 | GZ00 | C |
| ATOM | 4613 | CD | GLN | A | 177 | -27.955 | 113.563 | -7.061 | 1.00 | 68.75 | GZ00 | C |
| ATOM | 4614 | OE1 | GLN | A | 177 | -27.236 | 114.552 | -7.240 | 1.00 | 87.07 | GZ00 | O |
| ATOM | 4615 | NE2 | GLN | A | 177 | -29.018 | 113.581 | -6.259 | 1.00 | 58.03 | GZ00 | N |
| ATOM | 4616 | N | SER | A | 178 | -24.100 | 109.596 | -6.997 | 1.00 | 53.78 | GZ00 | N |
| ATOM | 4617 | CA | SER | A | 178 | -22.982 | 109.070 | -6.216 | 1.00 | 54.16 | GZ00 | C |
| ATOM | 4618 | C | SER | A | 178 | -23.113 | 109.421 | -4.744 | 1.00 | 58.69 | GZ00 | C |
| ATOM | 4619 | O | SER | A | 178 | -22.550 | 108.730 | -3.889 | 1.00 | 66.24 | GZ00 | O |
| ATOM | 4620 | CB | SER | A | 178 | -21.648 | 109.588 | -6.769 | 1.00 | 54.96 | GZ00 | C |
| ATOM | 4621 | OG | SER | A | 178 | -21.589 | 111.004 | -6.780 | 1.00 | 68.48 | GZ00 | O |
| ATOM | 4622 | N | SER | A | 179 | -23.821 | 110.508 | -4.432 | 1.00 | 59.99 | GZ00 | N |
| ATOM | 4623 | CA | SER | A | 179 | -24.134 | 110.871 | -3.058 | 1.00 | 57.94 | GZ00 | C |
| ATOM | 4624 | C | SER | A | 179 | -25.059 | 109.870 | -2.372 | 1.00 | 50.10 | GZ00 | C |
| ATOM | 4625 | O | SER | A | 179 | -25.232 | 109.952 | -1.154 | 1.00 | 50.29 | GZ00 | O |
| ATOM | 4626 | CB | SER | A | 179 | -24.787 | 112.254 | -3.030 | 1.00 | 52.99 | GZ00 | C |
| ATOM | 4627 | OG | SER | A | 179 | -26.135 | 112.159 | -3.478 | 1.00 | 61.58 | GZ00 | O |
| ATOM | 4628 | N | GLY | A | 180 | -25.671 | 108.949 | -3.113 | 1.00 | 43.93 | GZ00 | N |
| ATOM | 4629 | CA | GLY | A | 180 | -26.698 | 108.079 | -2.571 | 1.00 | 44.88 | GZ00 | C |
| ATOM | 4630 | C | GLY | A | 180 | -28.110 | 108.631 | -2.606 | 1.00 | 52.65 | GZ00 | C |
| ATOM | 4631 | O | GLY | A | 180 | -29.037 | 107.923 | -2.196 | 1.00 | 57.69 | GZ00 | O |
| ATOM | 4632 | N | LEU | A | 181 | -28.306 | 109.863 | -3.094 | 1.00 | 54.40 | GZ00 | N |
| ATOM | 4633 | CA | LEU | A | 181 | -29.609 | 110.513 | -3.171 | 1.00 | 55.39 | GZ00 | C |
| ATOM | 4634 | C | LEU | A | 181 | -30.141 | 110.518 | -4.600 | 1.00 | 52.55 | GZ00 | C |
| ATOM | 4635 | O | LEU | A | 181 | -29.377 | 110.492 | -5.571 | 1.00 | 47.86 | GZ00 | O |
| ATOM | 4636 | CB | LEU | A | 181 | -29.523 | 111.954 | -2.660 | 1.00 | 50.22 | GZ00 | C |
| ATOM | 4637 | CG | LEU | A | 181 | -29.108 | 112.109 | -1.207 | 1.00 | 54.13 | GZ00 | C |
| ATOM | 4638 | CD1 | LEU | A | 181 | -28.919 | 113.590 | -0.837 | 1.00 | 45.91 | GZ00 | C |
| ATOM | 4639 | CD2 | LEU | A | 181 | -30.175 | 111.435 | -0.357 | 1.00 | 50.94 | GZ00 | C |
| ATOM | 4640 | N | TYR | A | 182 | -31.469 | 110.607 | -4.712 | 1.00 | 45.18 | GZ00 | N |
| ATOM | 4641 | CA | TYR | A | 182 | -32.159 | 110.643 | -5.991 | 1.00 | 44.17 | GZ00 | C |
| ATOM | 4642 | C | TYR | A | 182 | -32.489 | 112.069 | -6.403 | 1.00 | 48.41 | GZ00 | C |
| ATOM | 4643 | O | TYR | A | 182 | -32.683 | 112.946 | -5.562 | 1.00 | 50.13 | GZ00 | O |
| ATOM | 4644 | CB | TYR | A | 182 | -33.454 | 109.832 | -5.939 | 1.00 | 51.31 | GZ00 | C |
| ATOM | 4645 | CG | TYR | A | 182 | -33.222 | 108.357 | -5.719 | 1.00 | 51.52 | GZ00 | C |
| ATOM | 4646 | CD1 | TYR | A | 182 | -32.971 | 107.499 | -6.796 | 1.00 | 53.09 | GZ00 | C |
| ATOM | 4647 | CD2 | TYR | A | 182 | -33.255 | 107.817 | -4.441 | 1.00 | 49.04 | GZ00 | C |
| ATOM | 4648 | CE1 | TYR | A | 182 | -32.746 | 106.144 | -6.595 | 1.00 | 57.42 | GZ00 | C |
| ATOM | 4649 | CE2 | TYR | A | 182 | -33.037 | 106.464 | -4.231 | 1.00 | 58.73 | GZ00 | C |
| ATOM | 4650 | CZ | TYR | A | 182 | -32.784 | 105.634 | -5.305 | 1.00 | 56.88 | GZ00 | C |
| ATOM | 4651 | OH | TYR | A | 182 | -32.567 | 104.299 | -5.082 | 1.00 | 64.47 | GZ00 | O |
| ATOM | 4652 | N | SER | A | 183 | -32.565 | 112.285 | -7.718 | 1.00 | 46.87 | GZ00 | N |
| ATOM | 4653 | CA | SER | A | 183 | -33.102 | 113.509 | -8.298 | 1.00 | 44.44 | GZ00 | C |
| ATOM | 4654 | C | SER | A | 183 | -33.911 | 113.162 | -9.541 | 1.00 | 46.59 | GZ00 | C |
| ATOM | 4655 | O | SER | A | 183 | -33.582 | 112.222 | -10.262 | 1.00 | 44.22 | GZ00 | O |
| ATOM | 4656 | CB | SER | A | 183 | -31.992 | 114.502 | -8.679 | 1.00 | 39.93 | GZ00 | C |
| ATOM | 4657 | OG | SER | A | 183 | -31.284 | 114.978 | -7.545 | 1.00 | 50.25 | GZ00 | O |
| ATOM | 4658 | N | LEU | A | 184 | -34.945 | 113.951 | -9.821 | 1.00 | 43.36 | GZ00 | N |
| ATOM | 4659 | CA | LEU | A | 184 | -35.653 | 113.810 | -11.084 | 1.00 | 43.02 | GZ00 | C |
| ATOM | 4660 | C | LEU | A | 184 | -36.113 | 115.182 | -11.552 | 1.00 | 47.62 | GZ00 | C |
| ATOM | 4661 | O | LEU | A | 184 | -36.058 | 116.171 | -10.815 | 1.00 | 47.17 | GZ00 | O |
| ATOM | 4662 | CB | LEU | A | 184 | -36.840 | 112.839 | -10.975 | 1.00 | 38.26 | GZ00 | C |
| ATOM | 4663 | CG | LEU | A | 184 | -38.061 | 113.103 | -10.085 | 1.00 | 43.98 | GZ00 | C |
| ATOM | 4664 | CD1 | LEU | A | 184 | -38.998 | 114.216 | -10.603 | 1.00 | 40.01 | GZ00 | C |
| ATOM | 4665 | CD2 | LEU | A | 184 | -38.840 | 111.795 | -9.902 | 1.00 | 43.99 | GZ00 | C |
| ATOM | 4666 | N | SER | A | 185 | -36.568 | 115.229 | -12.801 | 1.00 | 43.75 | GZ00 | N |
| ATOM | 4667 | CA | SER | A | 185 | -37.198 | 116.411 | -13.358 | 1.00 | 38.83 | GZ00 | C |
| ATOM | 4668 | C | SER | A | 185 | -38.565 | 116.044 | -13.909 | 1.00 | 43.09 | GZ00 | C |
| ATOM | 4669 | O | SER | A | 185 | -38.803 | 114.901 | -14.312 | 1.00 | 45.04 | GZ00 | O |
| ATOM | 4670 | CB | SER | A | 185 | -36.346 | 117.037 | -14.462 | 1.00 | 38.03 | GZ00 | C |
| ATOM | 4671 | OG | SER | A | 185 | -35.075 | 117.365 | -13.952 | 1.00 | 38.25 | GZ00 | O |
| ATOM | 4672 | N | SER | A | 186 | -39.468 | 117.023 | -13.889 | 1.00 | 39.49 | GZ00 | N |
| ATOM | 4673 | CA | SER | A | 186 | -40.767 | 116.950 | -14.544 | 1.00 | 41.04 | GZ00 | C |
| ATOM | 4674 | C | SER | A | 186 | -40.899 | 118.159 | -15.449 | 1.00 | 42.89 | GZ00 | C |
| ATOM | 4675 | O | SER | A | 186 | -40.584 | 119.272 | -15.032 | 1.00 | 44.02 | GZ00 | O |
| ATOM | 4676 | CB | SER | A | 186 | -41.917 | 116.931 | -13.536 | 1.00 | 44.24 | GZ00 | C |
| ATOM | 4677 | OG | SER | A | 186 | -43.174 | 117.016 | -14.199 | 1.00 | 48.30 | GZ00 | O |
| ATOM | 4678 | N | VAL | A | 187 | -41.298 | 117.945 | -16.698 | 1.00 | 47.04 | GZ00 | N |
| ATOM | 4679 | CA | VAL | A | 187 | -41.489 | 119.062 | -17.613 | 1.00 | 45.11 | GZ00 | C |
| ATOM | 4680 | C | VAL | A | 187 | -42.849 | 118.971 | -18.275 | 1.00 | 51.54 | GZ00 | C |
| ATOM | 4681 | O | VAL | A | 187 | -43.401 | 117.887 | -18.494 | 1.00 | 53.22 | GZ00 | O |
| ATOM | 4682 | CB | VAL | A | 187 | -40.385 | 119.152 | -18.666 | 1.00 | 43.95 | GZ00 | C |

```
ATOM   4683  CG1 VAL A 187     -39.102 119.540 -17.976  1.00 54.28      GZ00 C
ATOM   4684  CG2 VAL A 187     -40.236 117.828 -19.352  1.00 52.33      GZ00 C
ATOM   4685  N   VAL A 188     -43.405 120.132 -18.564  1.00 54.43      GZ00 N
ATOM   4686  CA  VAL A 188     -44.641 120.228 -19.315  1.00 58.17      GZ00 C
ATOM   4687  C   VAL A 188     -44.385 121.161 -20.480  1.00 59.55      GZ00 C
ATOM   4688  O   VAL A 188     -43.710 122.188 -20.330  1.00 58.11      GZ00 O
ATOM   4689  CB  VAL A 188     -45.821 120.710 -18.443  1.00 59.93      GZ00 C
ATOM   4690  CG1 VAL A 188     -45.449 121.979 -17.680  1.00 60.26      GZ00 C
ATOM   4691  CG2 VAL A 188     -47.062 120.912 -19.297  1.00 61.12      GZ00 C
ATOM   4692  N   THR A 189     -44.859 120.768 -21.655  1.00 62.33      GZ00 N
ATOM   4693  CA  THR A 189     -44.814 121.638 -22.815  1.00 66.67      GZ00 C
ATOM   4694  C   THR A 189     -46.153 122.362 -22.930  1.00 69.89      GZ00 C
ATOM   4695  O   THR A 189     -47.220 121.739 -22.815  1.00 62.33      GZ00 O
ATOM   4696  CB  THR A 189     -44.477 120.837 -24.072  1.00 67.75      GZ00 C
ATOM   4697  OG1 THR A 189     -44.384 119.450 -23.731  1.00 69.60      GZ00 O
ATOM   4698  CG2 THR A 189     -43.130 121.278 -24.620  1.00 74.71      GZ00 C
ATOM   4699  N   VAL A 190     -46.084 123.681 -23.102  1.00 66.60      GZ00 N
ATOM   4700  CA  VAL A 190     -47.263 124.547 -23.133  1.00 66.10      GZ00 C
ATOM   4701  C   VAL A 190     -47.136 125.490 -24.322  1.00 73.09      GZ00 C
ATOM   4702  O   VAL A 190     -46.045 125.641 -24.901  1.00 71.87      GZ00 O
ATOM   4703  CB  VAL A 190     -47.425 125.349 -21.818  1.00 64.66      GZ00 C
ATOM   4704  CG1 VAL A 190     -47.569 124.428 -20.610  1.00 58.32      GZ00 C
ATOM   4705  CG2 VAL A 190     -46.261 126.316 -21.640  1.00 59.75      GZ00 C
ATOM   4706  N   PRO A 191     -48.245 126.108 -24.739  1.00 74.14      GZ00 N
ATOM   4707  CA  PRO A 191     -48.160 127.158 -25.763  1.00 74.58      GZ00 C
ATOM   4708  C   PRO A 191     -47.354 128.344 -25.261  1.00 76.75      GZ00 C
ATOM   4709  O   PRO A 191     -47.577 128.839 -24.154  1.00 81.03      GZ00 O
ATOM   4710  CB  PRO A 191     -49.625 127.539 -26.000  1.00 69.86      GZ00 C
ATOM   4711  CG  PRO A 191     -50.402 126.339 -25.563  1.00 73.10      GZ00 C
ATOM   4712  CD  PRO A 191     -49.645 125.802 -24.391  1.00 71.77      GZ00 C
ATOM   4713  N   SER A 192     -46.422 128.813 -26.093  1.00 74.74      GZ00 N
ATOM   4714  CA  SER A 192     -45.569 129.925 -25.684  1.00 84.32      GZ00 C
ATOM   4715  C   SER A 192     -46.375 131.203 -25.449  1.00 87.52      GZ00 C
ATOM   4716  O   SER A 192     -46.000 132.033 -24.611  1.00 90.45      GZ00 O
ATOM   4717  CB  SER A 192     -44.469 130.145 -26.720  1.00 82.62      GZ00 C
ATOM   4718  OG  SER A 192     -45.022 130.471 -27.977  1.00 97.04      GZ00 O
ATOM   4719  N   SER A 193     -47.472 131.392 -26.187  1.00 89.79      GZ00 N
ATOM   4720  CA  SER A 193     -48.307 132.573 -25.998  1.00 89.74      GZ00 C
ATOM   4721  C   SER A 193     -48.972 132.612 -24.626  1.00 93.59      GZ00 C
ATOM   4722  O   SER A 193     -49.320 133.699 -24.148  1.00 95.20      GZ00 O
ATOM   4723  CB  SER A 193     -49.389 132.613 -27.076  1.00 81.94      GZ00 C
ATOM   4724  OG  SER A 193     -50.265 131.503 -26.929  1.00 74.81      GZ00 O
ATOM   4725  N   SER A 194     -49.138 131.462 -23.975  1.00 87.57      GZ00 N
ATOM   4726  CA  SER A 194     -49.771 131.416 -22.662  1.00 89.09      GZ00 C
ATOM   4727  C   SER A 194     -48.823 131.758 -21.516  1.00 91.10      GZ00 C
ATOM   4728  O   SER A 194     -49.278 131.840 -20.368  1.00 89.32      GZ00 O
ATOM   4729  CB  SER A 194     -50.372 130.030 -22.418  1.00 81.55      GZ00 C
ATOM   4730  OG  SER A 194     -49.347 129.061 -22.241  1.00 88.02      GZ00 O
ATOM   4731  N   LEU A 195     -47.528 131.951 -21.791  1.00 87.53      GZ00 N
ATOM   4732  CA  LEU A 195     -46.560 132.107 -20.708  1.00 88.74      GZ00 C
ATOM   4733  C   LEU A 195     -46.827 133.359 -19.882  1.00 89.05      GZ00 C
ATOM   4734  O   LEU A 195     -46.677 133.340 -18.654  1.00 89.42      GZ00 O
ATOM   4735  CB  LEU A 195     -45.137 132.120 -21.267  1.00 85.76      GZ00 C
ATOM   4736  CG  LEU A 195     -44.685 130.772 -21.838  1.00 84.17      GZ00 C
ATOM   4737  CD1 LEU A 195     -43.245 130.846 -22.337  1.00 78.51      GZ00 C
ATOM   4738  CD2 LEU A 195     -44.872 129.643 -20.827  1.00 71.43      GZ00 C
ATOM   4739  N   GLY A 196     -47.240 134.451 -20.529  1.00 92.67      GZ00 N
ATOM   4740  CA  GLY A 196     -47.552 135.661 -19.789  1.00 83.87      GZ00 C
ATOM   4741  C   GLY A 196     -48.867 135.611 -19.045  1.00 82.81      GZ00 C
ATOM   4742  O   GLY A 196     -49.054 136.368 -18.090  1.00 84.91      GZ00 O
ATOM   4743  N   THR A 197     -49.775 134.728 -19.455  1.00 85.57      GZ00 N
ATOM   4744  CA  THR A 197     -51.143 134.692 -18.952  1.00 86.43      GZ00 C
ATOM   4745  C   THR A 197     -51.368 133.631 -17.888  1.00 86.30      GZ00 C
ATOM   4746  O   THR A 197     -52.058 133.892 -16.898  1.00 86.17      GZ00 O
ATOM   4747  CB  THR A 197     -52.121 134.417 -20.097  1.00 86.40      GZ00 C
ATOM   4748  OG1 THR A 197     -51.849 135.303 -21.187  1.00 91.08      GZ00 O
ATOM   4749  CG2 THR A 197     -53.569 134.569 -19.626  1.00 80.19      GZ00 C
ATOM   4750  N   GLN A 198     -50.770 132.454 -18.048  1.00 84.30      GZ00 N
ATOM   4751  CA  GLN A 198     -51.154 131.288 -17.272  1.00 78.44      GZ00 C
ATOM   4752  C   GLN A 198     -50.118 131.004 -16.194  1.00 79.40      GZ00 C
ATOM   4753  O   GLN A 198     -48.910 131.155 -16.412  1.00 78.37      GZ00 O
```

```
ATOM   4754  CB  GLN A 198     -51.315 130.071 -18.184  1.00 76.20      GZ00 C
ATOM   4755  CG  GLN A 198     -51.543 128.762 -17.443  1.00 79.56      GZ00 C
ATOM   4756  CD  GLN A 198     -52.867 128.724 -16.710  1.00 81.18      GZ00 C
ATOM   4757  OE1 GLN A 198     -53.881 129.172 -17.236  1.00 93.21      GZ00 O
ATOM   4758  NE2 GLN A 198     -52.867 128.185 -15.493  1.00 73.89      GZ00 N
ATOM   4759  N   THR A 199     -50.606 130.551 -15.043  1.00 73.01      GZ00 N
ATOM   4760  CA  THR A 199     -49.774 130.248 -13.891  1.00 76.95      GZ00 C
ATOM   4761  C   THR A 199     -49.518 128.747 -13.834  1.00 74.20      GZ00 C
ATOM   4762  O   THR A 199     -50.462 127.944 -13.805  1.00 70.39      GZ00 O
ATOM   4763  CB  THR A 199     -50.445 130.746 -12.610  1.00 71.33      GZ00 C
ATOM   4764  OG1 THR A 199     -50.421 132.179 -12.601  1.00 73.00      GZ00 O
ATOM   4765  CG2 THR A 199     -49.728 130.224 -11.382  1.00 65.14      GZ00 C
ATOM   4766  N   TYR A 200     -48.242 128.376 -13.805  1.00 68.08      GZ00 N
ATOM   4767  CA  TYR A 200     -47.829 126.982 -13.781  1.00 61.58      GZ00 C
ATOM   4768  C   TYR A 200     -47.135 126.697 -12.461  1.00 60.20      GZ00 C
ATOM   4769  O   TYR A 200     -46.093 127.293 -12.153  1.00 57.85      GZ00 O
ATOM   4770  CB  TYR A 200     -46.911 126.674 -14.955  1.00 57.54      GZ00 C
ATOM   4771  CG  TYR A 200     -47.580 126.881 -16.287  1.00 62.50      GZ00 C
ATOM   4772  CD1 TYR A 200     -48.564 126.013 -16.731  1.00 61.12      GZ00 C
ATOM   4773  CD2 TYR A 200     -47.218 127.943 -17.106  1.00 67.26      GZ00 C
ATOM   4774  CE1 TYR A 200     -49.178 126.194 -17.951  1.00 65.39      GZ00 C
ATOM   4775  CE2 TYR A 200     -47.820 128.135 -18.333  1.00 69.26      GZ00 C
ATOM   4776  CZ  TYR A 200     -48.802 127.257 -18.753  1.00 70.05      GZ00 C
ATOM   4777  OH  TYR A 200     -49.409 127.447 -19.979  1.00 69.68      GZ00 O
ATOM   4778  N   ILE A 201     -47.710 125.775 -11.699  1.00 54.42      GZ00 N
ATOM   4779  CA  ILE A 201     -47.224 125.396 -10.384  1.00 55.01      GZ00 C
ATOM   4780  C   ILE A 201     -47.026 123.891 -10.383  1.00 53.67      GZ00 C
ATOM   4781  O   ILE A 201     -47.944 123.147 -10.738  1.00 51.99      GZ00 O
ATOM   4782  CB  ILE A 201     -48.220 125.791  -9.275  1.00 53.52      GZ00 C
ATOM   4783  CG1 ILE A 201     -48.406 127.304  -9.225  1.00 59.92      GZ00 C
ATOM   4784  CG2 ILE A 201     -47.757 125.257  -7.920  1.00 51.50      GZ00 C
ATOM   4785  CD1 ILE A 201     -49.568 127.719  -8.359  1.00 59.25      GZ00 C
ATOM   4786  N   CYS A 202     -45.846 123.434  -9.979  1.00 49.43      GZ00 N
ATOM   4787  CA  CYS A 202     -45.655 122.005  -9.794  1.00 52.57      GZ00 C
ATOM   4788  C   CYS A 202     -45.814 121.642  -8.324  1.00 50.37      GZ00 C
ATOM   4789  O   CYS A 202     -45.364 122.364  -7.433  1.00 52.12      GZ00 O
ATOM   4790  CB  CYS A 202     -44.301 121.549 -10.331  1.00 62.40      GZ00 C
ATOM   4791  SG  CYS A 202     -42.952 121.799  -9.238  1.00 64.20      GZ00 S
ATOM   4792  N   ASN A 203     -46.465 120.516  -8.082  1.00 47.36      GZ00 N
ATOM   4793  CA  ASN A 203     -46.831 120.068  -6.747  1.00 46.99      GZ00 C
ATOM   4794  C   ASN A 203     -46.058 118.791  -6.497  1.00 48.88      GZ00 C
ATOM   4795  O   ASN A 203     -46.287 117.778  -7.163  1.00 57.36      GZ00 O
ATOM   4796  CB  ASN A 203     -48.342 119.850  -6.635  1.00 48.02      GZ00 C
ATOM   4797  CG  ASN A 203     -49.137 120.936  -7.344  1.00 55.33      GZ00 C
ATOM   4798  OD1 ASN A 203     -49.750 120.696  -8.385  1.00 60.59      GZ00 O
ATOM   4799  ND2 ASN A 203     -49.148 122.139  -6.765  1.00 49.46      GZ00 N
ATOM   4800  N   VAL A 204     -45.120 118.858  -5.568  1.00 44.47      GZ00 N
ATOM   4801  CA  VAL A 204     -44.215 117.764  -5.274  1.00 47.87      GZ00 C
ATOM   4802  C   VAL A 204     -44.619 117.151  -3.949  1.00 46.74      GZ00 C
ATOM   4803  O   VAL A 204     -44.770 117.864  -2.952  1.00 50.77      GZ00 O
ATOM   4804  CB  VAL A 204     -42.755 118.248  -5.231  1.00 44.63      GZ00 C
ATOM   4805  CG1 VAL A 204     -41.843 117.090  -4.887  1.00 38.03      GZ00 C
ATOM   4806  CG2 VAL A 204     -42.380 118.903  -6.567  1.00 40.53      GZ00 C
ATOM   4807  N   ASN A 205     -44.767 115.832  -3.928  1.00 48.79      GZ00 N
ATOM   4808  CA  ASN A 205     -45.133 115.123  -2.714  1.00 47.93      GZ00 C
ATOM   4809  C   ASN A 205     -44.153 113.974  -2.534  1.00 49.20      GZ00 C
ATOM   4810  O   ASN A 205     -43.964 113.166  -3.445  1.00 60.40      GZ00 O
ATOM   4811  CB  ASN A 205     -46.588 114.648  -2.781  1.00 53.37      GZ00 C
ATOM   4812  CG  ASN A 205     -47.095 114.117  -1.449  1.00 66.37      GZ00 C
ATOM   4813  OD1 ASN A 205     -46.479 114.336  -0.404  1.00 69.20      GZ00 O
ATOM   4814  ND2 ASN A 205     -48.254 113.468  -1.473  1.00 77.20      GZ00 N
ATOM   4815  N   HIS A 206     -43.476 113.954  -1.396  1.00 45.52      GZ00 N
ATOM   4816  CA  HIS A 206     -42.524 112.919  -1.028  1.00 43.97      GZ00 C
ATOM   4817  C   HIS A 206     -42.971 112.323   0.309  1.00 53.01      GZ00 C
ATOM   4818  O   HIS A 206     -42.520 112.750   1.378  1.00 53.77      GZ00 O
ATOM   4819  CB  HIS A 206     -41.137 113.478  -0.951  1.00 40.28      GZ00 C
ATOM   4820  CG  HIS A 206     -40.104 112.463  -0.585  1.00 48.58      GZ00 C
ATOM   4821  ND1 HIS A 206     -39.422 112.498   0.611  1.00 53.96      GZ00 N
ATOM   4822  CD2 HIS A 206     -39.646 111.374  -1.247  1.00 48.75      GZ00 C
ATOM   4823  CE1 HIS A 206     -38.579 111.482   0.667  1.00 51.43      GZ00 C
ATOM   4824  NE2 HIS A 206     -38.689 110.790  -0.453  1.00 52.90      GZ00 N
```

| | | | | | | | | | | | |
|---|---|---|---|---|---|---|---|---|---|---|---|
| ATOM | 4825 | N | LYS | A | 207 | -43.829 | 111.306 | 0.233 | 1.00 | 54.92 | GZ00 N |
| ATOM | 4826 | CA | LYS | A | 207 | -44.424 | 110.729 | 1.434 | 1.00 | 48.15 | GZ00 C |
| ATOM | 4827 | C | LYS | A | 207 | -43.418 | 110.140 | 2.423 | 1.00 | 52.26 | GZ00 C |
| ATOM | 4828 | O | LYS | A | 207 | -43.648 | 110.282 | 3.635 | 1.00 | 59.42 | GZ00 O |
| ATOM | 4829 | CB | LYS | A | 207 | -45.481 | 109.710 | 1.007 | 1.00 | 49.63 | GZ00 C |
| ATOM | 4830 | CG | LYS | A | 207 | -46.665 | 110.413 | 0.313 | 1.00 | 60.89 | GZ00 C |
| ATOM | 4831 | CD | LYS | A | 207 | -47.752 | 109.458 | -0.167 | 1.00 | 75.35 | GZ00 C |
| ATOM | 4832 | CE | LYS | A | 207 | -48.892 | 110.226 | -0.848 | 1.00 | 81.98 | GZ00 C |
| ATOM | 4833 | NZ | LYS | A | 207 | -49.961 | 109.352 | -1.445 | 1.00 | 90.80 | GZ00 N1+ |
| ATOM | 4834 | N | PRO | A | 208 | -42.313 | 109.506 | 2.016 | 1.00 | 53.20 | GZ00 N |
| ATOM | 4835 | CA | PRO | A | 208 | -41.376 | 108.969 | 3.026 | 1.00 | 54.32 | GZ00 C |
| ATOM | 4836 | C | PRO | A | 208 | -40.864 | 109.993 | 4.034 | 1.00 | 57.05 | GZ00 C |
| ATOM | 4837 | O | PRO | A | 208 | -40.579 | 109.623 | 5.178 | 1.00 | 57.60 | GZ00 O |
| ATOM | 4838 | CB | PRO | A | 208 | -40.231 | 108.409 | 2.174 | 1.00 | 48.60 | GZ00 C |
| ATOM | 4839 | CG | PRO | A | 208 | -40.871 | 108.022 | 0.913 | 1.00 | 50.40 | GZ00 C |
| ATOM | 4840 | CD | PRO | A | 208 | -41.948 | 109.055 | 0.660 | 1.00 | 50.23 | GZ00 C |
| ATOM | 4841 | N | SER | A | 209 | -40.691 | 111.254 | 3.636 | 1.00 | 61.65 | GZ00 N |
| ATOM | 4842 | CA | SER | A | 209 | -40.273 | 112.325 | 4.535 | 1.00 | 62.97 | GZ00 C |
| ATOM | 4843 | C | SER | A | 209 | -41.416 | 113.260 | 4.930 | 1.00 | 61.87 | GZ00 C |
| ATOM | 4844 | O | SER | A | 209 | -41.174 | 114.251 | 5.630 | 1.00 | 60.78 | GZ00 O |
| ATOM | 4845 | CB | SER | A | 209 | -39.165 | 113.147 | 3.879 | 1.00 | 59.23 | GZ00 C |
| ATOM | 4846 | OG | SER | A | 209 | -39.685 | 113.856 | 2.755 | 1.00 | 55.04 | GZ00 O |
| ATOM | 4847 | N | ASN | A | 210 | -42.646 | 112.940 | 4.539 | 1.00 | 55.47 | GZ00 N |
| ATOM | 4848 | CA | ASN | A | 210 | -43.808 | 113.822 | 4.651 | 1.00 | 56.64 | GZ00 C |
| ATOM | 4849 | C | ASN | A | 210 | -43.460 | 115.261 | 4.290 | 1.00 | 56.98 | GZ00 C |
| ATOM | 4850 | O | ASN | A | 210 | -43.558 | 116.181 | 5.100 | 1.00 | 62.92 | GZ00 O |
| ATOM | 4851 | CB | ASN | A | 210 | -44.398 | 113.761 | 6.053 | 1.00 | 60.66 | GZ00 C |
| ATOM | 4852 | CG | ASN | A | 210 | -44.555 | 112.358 | 6.550 | 1.00 | 60.50 | GZ00 C |
| ATOM | 4853 | OD1 | ASN | A | 210 | -45.556 | 111.702 | 6.273 | 1.00 | 64.24 | GZ00 O |
| ATOM | 4854 | ND2 | ASN | A | 210 | -43.562 | 111.880 | 7.288 | 1.00 | 60.19 | GZ00 N |
| ATOM | 4855 | N | THR | A | 211 | -43.011 | 115.442 | 3.057 | 1.00 | 54.32 | GZ00 N |
| ATOM | 4856 | CA | THR | A | 211 | -42.767 | 116.765 | 2.504 | 1.00 | 47.32 | GZ00 C |
| ATOM | 4857 | C | THR | A | 211 | -43.723 | 117.022 | 1.349 | 1.00 | 47.89 | GZ00 C |
| ATOM | 4858 | O | THR | A | 211 | -43.910 | 116.159 | 0.489 | 1.00 | 54.45 | GZ00 O |
| ATOM | 4859 | CB | THR | A | 211 | -41.317 | 116.913 | 2.046 | 1.00 | 49.88 | GZ00 C |
| ATOM | 4860 | OG1 | THR | A | 211 | -40.444 | 116.645 | 3.148 | 1.00 | 51.07 | GZ00 O |
| ATOM | 4861 | CG2 | THR | A | 211 | -41.061 | 118.318 | 1.560 | 1.00 | 42.66 | GZ00 C |
| ATOM | 4862 | N | LYS | A | 212 | -44.338 | 118.194 | 1.339 | 1.00 | 50.89 | GZ00 N |
| ATOM | 4863 | CA | LYS | A | 212 | -45.112 | 118.659 | 0.198 | 1.00 | 47.65 | GZ00 C |
| ATOM | 4864 | C | LYS | A | 212 | -44.570 | 120.029 | -0.168 | 1.00 | 47.72 | GZ00 C |
| ATOM | 4865 | O | LYS | A | 212 | -44.386 | 120.878 | 0.712 | 1.00 | 52.21 | GZ00 O |
| ATOM | 4866 | CB | LYS | A | 212 | -46.611 | 118.715 | 0.514 | 1.00 | 52.77 | GZ00 C |
| ATOM | 4867 | CG | LYS | A | 212 | -47.140 | 117.383 | 1.052 | 1.00 | 63.53 | GZ00 C |
| ATOM | 4868 | CD | LYS | A | 212 | -48.662 | 117.295 | 1.167 | 1.00 | 63.25 | GZ00 C |
| ATOM | 4869 | CE | LYS | A | 212 | -49.049 | 116.010 | 1.920 | 1.00 | 71.43 | GZ00 C |
| ATOM | 4870 | NZ | LYS | A | 212 | -50.290 | 115.345 | 1.415 | 1.00 | 66.03 | GZ00 N1+ |
| ATOM | 4871 | N | VAL | A | 213 | -44.223 | 120.212 | -1.437 | 1.00 | 41.63 | GZ00 N |
| ATOM | 4872 | CA | VAL | A | 213 | -43.729 | 121.485 | -1.935 | 1.00 | 44.71 | GZ00 C |
| ATOM | 4873 | C | VAL | A | 213 | -44.585 | 121.894 | -3.121 | 1.00 | 48.49 | GZ00 C |
| ATOM | 4874 | O | VAL | A | 213 | -44.882 | 121.063 | -3.983 | 1.00 | 53.14 | GZ00 O |
| ATOM | 4875 | CB | VAL | A | 213 | -42.246 | 121.419 | -2.347 | 1.00 | 43.73 | GZ00 C |
| ATOM | 4876 | CG1 | VAL | A | 213 | -41.816 | 122.764 | -2.924 | 1.00 | 39.48 | GZ00 C |
| ATOM | 4877 | CG2 | VAL | A | 213 | -41.362 | 121.029 | -1.165 | 1.00 | 37.84 | GZ00 C |
| ATOM | 4878 | N | ASP | A | 214 | -45.015 | 123.156 | -3.139 | 1.00 | 47.97 | GZ00 N |
| ATOM | 4879 | CA | ASP | A | 214 | -45.585 | 123.795 | -4.317 | 1.00 | 44.59 | GZ00 C |
| ATOM | 4880 | C | ASP | A | 214 | -44.598 | 124.838 | -4.810 | 1.00 | 46.54 | GZ00 C |
| ATOM | 4881 | O | ASP | A | 214 | -44.025 | 125.579 | -4.009 | 1.00 | 54.01 | GZ00 O |
| ATOM | 4882 | CB | ASP | A | 214 | -46.929 | 124.477 | -4.017 | 1.00 | 51.53 | GZ00 C |
| ATOM | 4883 | CG | ASP | A | 214 | -47.982 | 123.512 | -3.513 | 1.00 | 63.26 | GZ00 C |
| ATOM | 4884 | OD2 | ASP | A | 214 | -48.796 | 123.914 | -2.654 | 1.00 | 87.44 | GZ00 O1- |
| ATOM | 4885 | OD1 | ASP | A | 214 | -48.012 | 122.352 | -3.969 | 1.00 | 69.85 | GZ00 O |
| ATOM | 4886 | N | LYS | A | 215 | -44.377 | 124.888 | -6.119 | 1.00 | 44.93 | GZ00 N |
| ATOM | 4887 | CA | LYS | A | 215 | -43.442 | 125.855 | -6.679 | 1.00 | 46.63 | GZ00 C |
| ATOM | 4888 | C | LYS | A | 215 | -43.978 | 126.399 | -7.988 | 1.00 | 52.58 | GZ00 C |
| ATOM | 4889 | O | LYS | A | 215 | -44.348 | 125.626 | -8.878 | 1.00 | 52.15 | GZ00 O |
| ATOM | 4890 | CB | LYS | A | 215 | -42.051 | 125.248 | -6.899 | 1.00 | 47.58 | GZ00 C |
| ATOM | 4891 | CG | LYS | A | 215 | -40.969 | 125.964 | -6.129 | 1.00 | 50.19 | GZ00 C |
| ATOM | 4892 | CD | LYS | A | 215 | -40.152 | 126.858 | -7.027 | 1.00 | 48.08 | GZ00 C |
| ATOM | 4893 | CE | LYS | A | 215 | -39.413 | 127.923 | -6.211 | 1.00 | 55.88 | GZ00 C |
| ATOM | 4894 | NZ | LYS | A | 215 | -38.638 | 127.415 | -5.023 | 1.00 | 63.59 | GZ00 N1+ |
| ATOM | 4895 | N | LYS | A | 216 | -44.024 | 127.727 | -8.093 | 1.00 | 50.59 | GZ00 N |

```
ATOM   4896  CA  LYS A 216     -44.437 128.396  -9.312  1.00 51.04      GZ00 C
ATOM   4897  C   LYS A 216     -43.241 128.550 -10.244  1.00 53.52      GZ00 C
ATOM   4898  O   LYS A 216     -42.135 128.880  -9.807  1.00 49.36      GZ00 O
ATOM   4899  CB  LYS A 216     -45.052 129.760  -8.996  1.00 56.62      GZ00 C
ATOM   4900  CG  LYS A 216     -45.566 130.514 -10.215  1.00 61.12      GZ00 C
ATOM   4901  CD  LYS A 216     -46.136 131.864  -9.823  1.00 67.36      GZ00 C
ATOM   4902  CE  LYS A 216     -46.919 132.483 -10.962  1.00 66.87      GZ00 C
ATOM   4903  NZ  LYS A 216     -47.732 133.632 -10.483  1.00 75.15      GZ00 N1+
ATOM   4904  N   VAL A 217     -43.462 128.277 -11.526  1.00 51.79      GZ00 N
ATOM   4905  CA  VAL A 217     -42.414 128.342 -12.536  1.00 53.46      GZ00 C
ATOM   4906  C   VAL A 217     -42.741 129.494 -13.465  1.00 54.54      GZ00 C
ATOM   4907  O   VAL A 217     -43.747 129.455 -14.179  1.00 59.89      GZ00 O
ATOM   4908  CB  VAL A 217     -42.292 127.021 -13.308  1.00 55.15      GZ00 C
ATOM   4909  CG1 VAL A 217     -41.110 127.076 -14.255  1.00 49.43      GZ00 C
ATOM   4910  CG2 VAL A 217     -42.184 125.845 -12.330  1.00 40.65      GZ00 C
ATOM   4911  N   GLU A 218     -41.905 130.525 -13.455  1.00 58.16      GZ00 N
ATOM   4912  CA  GLU A 218     -42.169 131.714 -14.244  1.00 61.44      GZ00 C
ATOM   4913  C   GLU A 218     -41.064 131.965 -15.263  1.00 73.28      GZ00 C
ATOM   4914  O   GLU A 218     -39.904 131.589 -15.043  1.00 69.38      GZ00 O
ATOM   4915  CB  GLU A 218     -42.312 132.955 -13.350  1.00 65.18      GZ00 C
ATOM   4916  CG  GLU A 218     -43.436 132.867 -12.323  1.00 71.84      GZ00 C
ATOM   4917  CD  GLU A 218     -43.518 134.104 -11.427  1.00 80.98      GZ00 C
ATOM   4918  OE1 GLU A 218     -42.537 134.884 -11.391  1.00 69.24      GZ00 O
ATOM   4919  OE2 GLU A 218     -44.568 134.295 -10.769  1.00 78.83      GZ00 O1-
ATOM   4920  N   PRO A 219     -41.401 132.592 -16.400  1.00 72.81      GZ00 N
ATOM   4921  CA  PRO A 219     -40.374 133.004 -17.371  1.00 73.28      GZ00 C
ATOM   4922  C   PRO A 219     -39.387 134.017 -16.815  1.00 77.52      GZ00 C
ATOM   4923  O   PRO A 219     -39.500 134.420 -15.653  1.00 81.78      GZ00 O
ATOM   4924  CB  PRO A 219     -41.194 133.614 -18.517  1.00 73.25      GZ00 C
ATOM   4925  CG  PRO A 219     -42.619 133.679 -18.024  1.00 67.58      GZ00 C
ATOM   4926  CD  PRO A 219     -42.759 132.663 -16.958  1.00 67.19      GZ00 C
ATOM   4927  N   LYS A 220     -38.405 134.405 -17.627  1.00 81.29      GZ00 N
ATOM   4928  CA  LYS A 220     -37.376 135.375 -17.234  1.00 82.90      GZ00 C
ATOM   4929  C   LYS A 220     -36.507 134.815 -16.117  1.00 88.96      GZ00 C
ATOM   4930  O   LYS A 220     -36.186 133.626 -16.112  1.00 89.60      GZ00 O
ATOM   4931  CB  LYS A 220     -38.003 136.710 -16.809  1.00 83.44      GZ00 C
ATOM   4932  CG  LYS A 220     -37.004 137.772 -16.372  1.00 79.50      GZ00 C
ATOM   4933  CD  LYS A 220     -36.117 138.217 -17.516  1.00 78.87      GZ00 C
ATOM   4934  CE  LYS A 220     -35.095 139.231 -17.031  1.00 76.44      GZ00 C
ATOM   4935  NZ  LYS A 220     -34.316 139.811 -18.154  1.00 72.11      GZ00 N1+
TER
ATOM   4936  N   GLN X   1     -13.182  99.213 -18.301  1.00 76.62           N
ATOM   4937  CA  GLN X   1     -13.330  98.583 -19.611  1.00 76.63           C
ATOM   4938  C   GLN X   1     -13.561  97.073 -19.505  1.00 74.72           C
ATOM   4939  O   GLN X   1     -12.891  96.390 -18.727  1.00 80.15           O
ATOM   4940  CB  GLN X   1     -12.095  98.863 -20.464  1.00 81.35           C
ATOM   4941  CG  GLN X   1     -11.908 100.324 -20.759  1.00 87.31           C
ATOM   4942  CD  GLN X   1     -13.144 100.930 -21.400  1.00101.98           C
ATOM   4943  OE1 GLN X   1     -13.815 101.779 -20.806  1.00102.82           O
ATOM   4944  NE2 GLN X   1     -13.454 100.491 -22.619  1.00 97.35           N
ATOM   4945  N   SER X   2     -14.506  96.551 -20.287  1.00 67.44           N
ATOM   4946  CA  SER X   2     -14.749  95.113 -20.298  1.00 60.09           C
ATOM   4947  C   SER X   2     -13.572  94.377 -20.927  1.00 57.36           C
ATOM   4948  O   SER X   2     -12.944  94.860 -21.875  1.00 53.67           O
ATOM   4949  CB  SER X   2     -16.026  94.785 -21.086  1.00 46.51           C
ATOM   4950  OG  SER X   2     -17.130  94.586 -20.224  1.00 58.04           O
ATOM   4951  N   VAL X   3     -13.284  93.183 -20.403  1.00 47.89           N
ATOM   4952  CA  VAL X   3     -12.146  92.428 -20.923  1.00 46.54           C
ATOM   4953  C   VAL X   3     -12.475  91.869 -22.301  1.00 47.23           C
ATOM   4954  O   VAL X   3     -11.622  91.855 -23.201  1.00 45.87           O
ATOM   4955  CB  VAL X   3     -11.731  91.309 -19.951  1.00 44.31           C
ATOM   4956  CG1 VAL X   3     -10.685  90.401 -20.593  1.00 34.16           C
ATOM   4957  CG2 VAL X   3     -11.202  91.895 -18.640  1.00 39.33           C
ATOM   4958  N   LEU X   4     -13.716  91.408 -22.490  1.00 42.72           N
ATOM   4959  CA  LEU X   4     -14.234  91.004 -23.790  1.00 40.50           C
ATOM   4960  C   LEU X   4     -15.023  92.164 -24.377  1.00 38.73           C
ATOM   4961  O   LEU X   4     -15.756  92.844 -23.656  1.00 38.68           O
ATOM   4962  CB  LEU X   4     -15.120  89.761 -23.666  1.00 32.58           C
ATOM   4963  CG  LEU X   4     -14.559  88.652 -22.763  1.00 35.07           C
ATOM   4964  CD1 LEU X   4     -15.572  87.502 -22.591  1.00 33.11           C
ATOM   4965  CD2 LEU X   4     -13.193  88.130 -23.260  1.00 26.20           C
```

| | | | | | | | | | | | |
|---|---|---|---|---|---|---|---|---|---|---|---|
| ATOM | 4966 | N | THR | X | 5 | -14.874 | 92.394 | -25.683 | 1.00 | 30.86 | N |
| ATOM | 4967 | CA | THR | X | 5 | -15.506 | 93.541 | -26.318 | 1.00 | 31.01 | C |
| ATOM | 4968 | C | THR | X | 5 | -16.635 | 93.087 | -27.228 | 1.00 | 32.92 | C |
| ATOM | 4969 | O | THR | X | 5 | -16.412 | 92.301 | -28.156 | 1.00 | 34.89 | O |
| ATOM | 4970 | CB | THR | X | 5 | -14.488 | 94.361 | -27.114 | 1.00 | 35.46 | C |
| ATOM | 4971 | OG1 | THR | X | 5 | -13.373 | 94.678 | -26.280 | 1.00 | 37.79 | O |
| ATOM | 4972 | CG2 | THR | X | 5 | -15.130 | 95.668 | -27.583 | 1.00 | 23.38 | C |
| ATOM | 4973 | N | GLN | X | 6 | -17.836 | 93.608 | -26.972 | 1.00 | 31.00 | N |
| ATOM | 4974 | CA | GLN | X | 6 | -19.060 | 93.406 | -27.730 | 1.00 | 33.70 | C |
| ATOM | 4975 | C | GLN | X | 6 | -19.549 | 94.756 | -28.224 | 1.00 | 33.00 | C |
| ATOM | 4976 | O | GLN | X | 6 | -19.399 | 95.759 | -27.519 | 1.00 | 30.42 | O |
| ATOM | 4977 | CB | GLN | X | 6 | -20.188 | 92.765 | -26.892 | 1.00 | 30.43 | C |
| ATOM | 4978 | CG | GLN | X | 6 | -19.910 | 91.392 | -26.335 | 1.00 | 28.80 | C |
| ATOM | 4979 | CD | GLN | X | 6 | -21.049 | 90.852 | -25.456 | 1.00 | 32.96 | C |
| ATOM | 4980 | OE1 | GLN | X | 6 | -20.823 | 90.407 | -24.329 | 1.00 | 32.89 | O |
| ATOM | 4981 | NE2 | GLN | X | 6 | -22.266 | 90.879 | -25.978 | 1.00 | 26.46 | N |
| ATOM | 4982 | N | PRO | X | 7 | -20.179 | 94.813 | -29.392 | 1.00 | 28.38 | N |
| ATOM | 4983 | CA | PRO | X | 7 | -20.880 | 96.043 | -29.796 | 1.00 | 33.26 | C |
| ATOM | 4984 | C | PRO | X | 7 | -21.977 | 96.356 | -28.791 | 1.00 | 38.21 | C |
| ATOM | 4985 | O | PRO | X | 7 | -22.613 | 95.435 | -28.252 | 1.00 | 35.17 | O |
| ATOM | 4986 | CB | PRO | X | 7 | -21.461 | 95.693 | -31.181 | 1.00 | 33.21 | C |
| ATOM | 4987 | CG | PRO | X | 7 | -21.607 | 94.178 | -31.146 | 1.00 | 34.98 | C |
| ATOM | 4988 | CD | PRO | X | 7 | -20.480 | 93.673 | -30.276 | 1.00 | 33.91 | C |
| ATOM | 4989 | N | PRO | X | 8 | -22.189 | 97.632 | -28.468 | 1.00 | 34.91 | N |
| ATOM | 4990 | CA | PRO | X | 8 | -23.156 | 97.962 | -27.407 | 1.00 | 29.83 | C |
| ATOM | 4991 | C | PRO | X | 8 | -24.604 | 97.731 | -27.792 | 1.00 | 37.56 | C |
| ATOM | 4992 | O | PRO | X | 8 | -25.412 | 97.420 | -26.908 | 1.00 | 36.02 | O |
| ATOM | 4993 | CB | PRO | X | 8 | -22.879 | 99.443 | -27.119 | 1.00 | 28.75 | C |
| ATOM | 4994 | CG | PRO | X | 8 | -22.237 | 99.952 | -28.379 | 1.00 | 41.44 | C |
| ATOM | 4995 | CD | PRO | X | 8 | -21.416 | 98.803 | -28.911 | 1.00 | 31.17 | C |
| ATOM | 4996 | N | SER | X | 9 | -24.980 | 97.891 | -29.057 | 1.00 | 30.64 | N |
| ATOM | 4997 | CA | SER | X | 9 | -26.373 | 97.657 | -29.406 | 1.00 | 33.12 | C |
| ATOM | 4998 | C | SER | X | 9 | -26.487 | 97.243 | -30.864 | 1.00 | 36.96 | C |
| ATOM | 4999 | O | SER | X | 9 | -25.582 | 97.451 | -31.685 | 1.00 | 34.35 | O |
| ATOM | 5000 | CB | SER | X | 9 | -27.257 | 98.873 | -29.146 | 1.00 | 35.68 | C |
| ATOM | 5001 | OG | SER | X | 9 | -26.883 | 99.958 | -29.965 | 1.00 | 42.47 | O |
| ATOM | 5002 | N | VAL | X | 10 | -27.628 | 96.644 | -31.164 | 1.00 | 33.72 | N |
| ATOM | 5003 | CA | VAL | X | 10 | -27.851 | 95.977 | -32.431 | 1.00 | 34.33 | C |
| ATOM | 5004 | C | VAL | X | 10 | -29.358 | 95.932 | -32.642 | 1.00 | 34.97 | C |
| ATOM | 5005 | O | VAL | X | 10 | -30.118 | 95.725 | -31.688 | 1.00 | 36.04 | O |
| ATOM | 5006 | CB | VAL | X | 10 | -27.170 | 94.585 | -32.397 | 1.00 | 36.02 | C |
| ATOM | 5007 | CG1 | VAL | X | 10 | -28.071 | 93.486 | -32.875 | 1.00 | 38.30 | C |
| ATOM | 5008 | CG2 | VAL | X | 10 | -25.823 | 94.625 | -33.148 | 1.00 | 34.46 | C |
| ATOM | 5009 | N | SER | X | 11 | -29.801 | 96.196 | -33.872 | 1.00 | 35.57 | N |
| ATOM | 5010 | CA | SER | X | 11 | -31.238 | 96.220 | -34.114 | 1.00 | 38.43 | C |
| ATOM | 5011 | C | SER | X | 11 | -31.565 | 95.782 | -35.531 | 1.00 | 38.48 | C |
| ATOM | 5012 | O | SER | X | 11 | -30.839 | 96.101 | -36.473 | 1.00 | 39.31 | O |
| ATOM | 5013 | CB | SER | X | 11 | -31.841 | 97.609 | -33.833 | 1.00 | 38.56 | C |
| ATOM | 5014 | OG | SER | X | 11 | -31.368 | 98.589 | -34.727 | 1.00 | 43.26 | O |
| ATOM | 5015 | N | ALA | X | 12 | -32.670 | 95.054 | -35.669 | 1.00 | 37.39 | N |
| ATOM | 5016 | CA | ALA | X | 12 | -33.132 | 94.581 | -36.965 | 1.00 | 35.97 | C |
| ATOM | 5017 | C | ALA | X | 12 | -34.610 | 94.221 | -36.869 | 1.00 | 39.63 | C |
| ATOM | 5018 | O | ALA | X | 12 | -35.144 | 93.969 | -35.782 | 1.00 | 37.40 | O |
| ATOM | 5019 | CB | ALA | X | 12 | -32.317 | 93.374 | -37.454 | 1.00 | 32.40 | C |
| ATOM | 5020 | N | ALA | X | 13 | -35.245 | 94.137 | -38.035 | 1.00 | 39.79 | N |
| ATOM | 5021 | CA | ALA | X | 13 | -36.664 | 93.835 | -38.153 | 1.00 | 40.54 | C |
| ATOM | 5022 | C | ALA | X | 13 | -36.949 | 92.341 | -37.964 | 1.00 | 39.36 | C |
| ATOM | 5023 | O | ALA | X | 13 | -36.060 | 91.493 | -38.120 | 1.00 | 38.64 | O |
| ATOM | 5024 | CB | ALA | X | 13 | -37.170 | 94.286 | -39.518 | 1.00 | 26.30 | C |
| ATOM | 5025 | N | PRO | X | 14 | -38.182 | 91.992 | -37.606 | 1.00 | 39.35 | N |
| ATOM | 5026 | CA | PRO | X | 14 | -38.543 | 90.572 | -37.547 | 1.00 | 40.01 | C |
| ATOM | 5027 | C | PRO | X | 14 | -38.206 | 89.887 | -38.865 | 1.00 | 43.81 | C |
| ATOM | 5028 | O | PRO | X | 14 | -38.330 | 90.469 | -39.949 | 1.00 | 40.74 | O |
| ATOM | 5029 | CB | PRO | X | 14 | -40.050 | 90.596 | -37.287 | 1.00 | 28.16 | C |
| ATOM | 5030 | CG | PRO | X | 14 | -40.292 | 91.919 | -36.614 | 1.00 | 38.63 | C |
| ATOM | 5031 | CD | PRO | X | 14 | -39.300 | 92.873 | -37.204 | 1.00 | 38.01 | C |
| ATOM | 5032 | N | GLY | X | 15 | -37.742 | 88.649 | -38.754 | 1.00 | 42.53 | N |
| ATOM | 5033 | CA | GLY | X | 15 | -37.352 | 87.856 | -39.886 | 1.00 | 39.20 | C |
| ATOM | 5034 | C | GLY | X | 15 | -35.927 | 88.040 | -40.349 | 1.00 | 44.97 | C |
| ATOM | 5035 | O | GLY | X | 15 | -35.426 | 87.186 | -41.077 | 1.00 | 48.28 | O |
| ATOM | 5036 | N | GLN | X | 16 | -35.257 | 89.115 | -39.944 | 1.00 | 41.58 | N |

| | | | | | | | | | | | |
|---|---|---|---|---|---|---|---|---|---|---|---|
| ATOM | 5037 | CA | GLN | X | 16 | -33.915 | 89.385 | -40.433 | 1.00 | 43.47 | C |
| ATOM | 5038 | C | GLN | X | 16 | -32.862 | 88.623 | -39.621 | 1.00 | 47.57 | C |
| ATOM | 5039 | O | GLN | X | 16 | -33.167 | 87.903 | -38.664 | 1.00 | 46.60 | O |
| ATOM | 5040 | CB | GLN | X | 16 | -33.635 | 90.888 | -40.417 | 1.00 | 52.99 | C |
| ATOM | 5041 | CG | GLN | X | 16 | -34.303 | 91.702 | -41.539 | 1.00 | 46.17 | C |
| ATOM | 5042 | CD | GLN | X | 16 | -33.566 | 93.024 | -41.807 | 1.00 | 74.76 | C |
| ATOM | 5043 | OE1 | GLN | X | 16 | -33.715 | 94.021 | -41.064 | 1.00 | 62.51 | O |
| ATOM | 5044 | NE2 | GLN | X | 16 | -32.758 | 93.034 | -42.866 | 1.00 | 79.00 | N |
| ATOM | 5045 | N | LYS | X | 17 | -31.607 | 88.762 | -40.053 | 1.00 | 53.20 | N |
| ATOM | 5046 | CA | LYS | X | 17 | -30.415 | 88.195 | -39.436 | 1.00 | 49.52 | C |
| ATOM | 5047 | C | LYS | X | 17 | -29.695 | 89.254 | -38.627 | 1.00 | 51.74 | C |
| ATOM | 5048 | O | LYS | X | 17 | -29.796 | 90.454 | -38.883 | 1.00 | 62.58 | O |
| ATOM | 5049 | CB | LYS | X | 17 | -29.417 | 87.662 | -40.469 | 1.00 | 49.86 | C |
| ATOM | 5050 | CG | LYS | X | 17 | -29.783 | 86.391 | -41.150 | 1.00 | 61.26 | C |
| ATOM | 5051 | CD | LYS | X | 17 | -28.839 | 86.137 | -42.317 | 1.00 | 75.06 | C |
| ATOM | 5052 | CE | LYS | X | 17 | -29.295 | 84.939 | -43.148 | 1.00 | 82.27 | C |
| ATOM | 5053 | NZ | LYS | X | 17 | -28.391 | 84.723 | -44.304 | 1.00 | 82.49 | N |
| ATOM | 5054 | N | VAL | X | 18 | -28.913 | 88.785 | -37.670 | 1.00 | 51.92 | N |
| ATOM | 5055 | CA | VAL | X | 18 | -28.083 | 89.670 | -36.876 | 1.00 | 47.06 | C |
| ATOM | 5056 | C | VAL | X | 18 | -26.882 | 88.857 | -36.416 | 1.00 | 42.24 | C |
| ATOM | 5057 | O | VAL | X | 18 | -26.969 | 87.645 | -36.210 | 1.00 | 39.00 | O |
| ATOM | 5058 | CB | VAL | X | 18 | -28.932 | 90.288 | -35.735 | 1.00 | 50.43 | C |
| ATOM | 5059 | CG1 | VAL | X | 18 | -28.437 | 89.925 | -34.363 | 1.00 | 44.21 | C |
| ATOM | 5060 | CG2 | VAL | X | 18 | -29.054 | 91.783 | -35.919 | 1.00 | 48.19 | C |
| ATOM | 5061 | N | THR | X | 19 | -25.745 | 89.520 | -36.323 | 1.00 | 40.09 | N |
| ATOM | 5062 | CA | THR | X | 19 | -24.519 | 88.924 | -35.825 | 1.00 | 43.49 | C |
| ATOM | 5063 | C | THR | X | 19 | -24.005 | 89.770 | -34.672 | 1.00 | 39.57 | C |
| ATOM | 5064 | O | THR | X | 19 | -24.036 | 91.000 | -34.737 | 1.00 | 39.43 | O |
| ATOM | 5065 | CB | THR | X | 19 | -23.484 | 88.816 | -36.961 | 1.00 | 40.40 | C |
| ATOM | 5066 | OG1 | THR | X | 19 | -23.352 | 87.443 | -37.322 | 1.00 | 51.45 | O |
| ATOM | 5067 | CG2 | THR | X | 19 | -22.132 | 89.395 | -36.582 | 1.00 | 45.74 | C |
| ATOM | 5068 | N | ILE | X | 20 | -23.557 | 89.116 | -33.607 | 1.00 | 36.67 | N |
| ATOM | 5069 | CA | ILE | X | 20 | -23.009 | 89.805 | -32.449 | 1.00 | 34.08 | C |
| ATOM | 5070 | C | ILE | X | 20 | -21.638 | 89.219 | -32.166 | 1.00 | 35.80 | C |
| ATOM | 5071 | O | ILE | X | 20 | -21.517 | 88.008 | -31.946 | 1.00 | 34.42 | O |
| ATOM | 5072 | CB | ILE | X | 20 | -23.917 | 89.679 | -31.210 | 1.00 | 36.76 | C |
| ATOM | 5073 | CG1 | ILE | X | 20 | -25.269 | 90.345 | -31.467 | 1.00 | 35.10 | C |
| ATOM | 5074 | CG2 | ILE | X | 20 | -23.257 | 90.324 | -29.996 | 1.00 | 27.82 | C |
| ATOM | 5075 | CD1 | ILE | X | 20 | -26.276 | 90.127 | -30.364 | 1.00 | 28.66 | C |
| ATOM | 5076 | N | SER | X | 21 | -20.614 | 90.068 | -32.153 | 1.00 | 38.15 | N |
| ATOM | 5077 | CA | SER | X | 21 | -19.237 | 89.614 | -31.980 | 1.00 | 37.42 | C |
| ATOM | 5078 | C | SER | X | 21 | -18.762 | 89.818 | -30.547 | 1.00 | 38.58 | C |
| ATOM | 5079 | O | SER | X | 21 | -19.344 | 90.570 | -29.762 | 1.00 | 39.24 | O |
| ATOM | 5080 | CB | SER | X | 21 | -18.290 | 90.351 | -32.932 | 1.00 | 30.39 | C |
| ATOM | 5081 | OG | SER | X | 21 | -18.312 | 91.741 | -32.656 | 1.00 | 45.43 | O |
| ATOM | 5082 | N | CYS | X | 22 | -17.662 | 89.152 | -30.226 | 1.00 | 31.32 | N |
| ATOM | 5083 | CA | CYS | X | 22 | -17.099 | 89.156 | -28.883 | 1.00 | 33.00 | C |
| ATOM | 5084 | C | CYS | X | 22 | -15.601 | 88.936 | -29.035 | 1.00 | 36.67 | C |
| ATOM | 5085 | O | CYS | X | 22 | -15.179 | 87.824 | -29.367 | 1.00 | 33.05 | O |
| ATOM | 5086 | CB | CYS | X | 22 | -17.737 | 88.067 | -28.029 | 1.00 | 33.80 | C |
| ATOM | 5087 | SG | CYS | X | 22 | -16.950 | 87.740 | -26.422 | 1.00 | 49.12 | S |
| ATOM | 5088 | N | SER | X | 23 | -14.806 | 89.986 | -28.830 | 1.00 | 35.27 | N |
| ATOM | 5089 | CA | SER | X | 23 | -13.362 | 89.917 | -29.015 | 1.00 | 36.02 | C |
| ATOM | 5090 | C | SER | X | 23 | -12.635 | 89.893 | -27.682 | 1.00 | 35.20 | C |
| ATOM | 5091 | O | SER | X | 23 | -12.961 | 90.659 | -26.765 | 1.00 | 32.83 | O |
| ATOM | 5092 | CB | SER | X | 23 | -12.847 | 91.105 | -29.824 | 1.00 | 34.28 | C |
| ATOM | 5093 | OG | SER | X | 23 | -13.487 | 91.109 | -31.082 | 1.00 | 60.42 | O |
| ATOM | 5094 | N | GLY | X | 24 | -11.631 | 89.027 | -27.600 | 1.00 | 29.76 | N |
| ATOM | 5095 | CA | GLY | X | 24 | -10.801 | 88.942 | -26.426 | 1.00 | 32.74 | C |
| ATOM | 5096 | C | GLY | X | 24 | -9.364 | 88.732 | -26.822 | 1.00 | 36.96 | C |
| ATOM | 5097 | O | GLY | X | 24 | -8.898 | 89.366 | -27.766 | 1.00 | 35.74 | O |
| ATOM | 5098 | N | SER | X | 25 | -8.666 | 87.829 | -26.132 | 1.00 | 34.51 | N |
| ATOM | 5099 | CA | SER | X | 25 | -7.238 | 87.632 | -26.326 | 1.00 | 35.15 | C |
| ATOM | 5100 | C | SER | X | 25 | -6.925 | 86.161 | -26.114 | 1.00 | 38.46 | C |
| ATOM | 5101 | O | SER | X | 25 | -7.793 | 85.366 | -25.733 | 1.00 | 35.89 | O |
| ATOM | 5102 | CB | SER | X | 25 | -6.411 | 88.485 | -25.366 | 1.00 | 31.48 | C |
| ATOM | 5103 | OG | SER | X | 25 | -6.531 | 87.980 | -24.047 | 1.00 | 41.73 | O |
| ATOM | 5104 | N | SER | X | 26 | -5.656 | 85.811 | -26.338 | 1.00 | 32.62 | N |
| ATOM | 5105 | CA | SER | X | 26 | -5.255 | 84.410 | -26.269 | 1.00 | 40.22 | C |
| ATOM | 5106 | C | SER | X | 26 | -5.478 | 83.825 | -24.886 | 1.00 | 39.50 | C |
| ATOM | 5107 | O | SER | X | 26 | -5.701 | 82.616 | -24.755 | 1.00 | 43.16 | O |

| | | | | | | | | | | | |
|---|---|---|---|---|---|---|---|---|---|---|---|
| ATOM | 5108 | CB | SER | X | 26 | -3.779 | 84.236 | -26.645 | 1.00 | 33.65 | C |
| ATOM | 5109 | OG | SER | X | 26 | -2.978 | 85.154 | -25.933 | 1.00 | 50.17 | O |
| ATOM | 5110 | N | SER | X | 27 | -5.442 | 84.650 | -23.847 | 1.00 | 37.12 | N |
| ATOM | 5111 | CA | SER | X | 27 | -5.564 | 84.097 | -22.504 | 1.00 | 35.96 | C |
| ATOM | 5112 | C | SER | X | 27 | -7.010 | 83.927 | -22.062 | 1.00 | 36.99 | C |
| ATOM | 5113 | O | SER | X | 27 | -7.236 | 83.380 | -20.976 | 1.00 | 37.69 | O |
| ATOM | 5114 | CB | SER | X | 27 | -4.848 | 84.982 | -21.480 | 1.00 | 32.77 | C |
| ATOM | 5115 | OG | SER | X | 27 | -5.484 | 86.238 | -21.459 | 1.00 | 46.10 | O |
| ATOM | 5116 | N | ASN | X | 28 | -7.992 | 84.407 | -22.840 | 1.00 | 35.46 | N |
| ATOM | 5117 | CA | ASN | X | 28 | -9.372 | 84.080 | -22.495 | 1.00 | 33.17 | C |
| ATOM | 5118 | C | ASN | X | 28 | -10.056 | 83.327 | -23.636 | 1.00 | 34.99 | C |
| ATOM | 5119 | O | ASN | X | 28 | -10.028 | 82.089 | -23.659 | 1.00 | 30.44 | O |
| ATOM | 5120 | CB | ASN | X | 28 | -10.161 | 85.324 | -22.072 | 1.00 | 29.46 | C |
| ATOM | 5121 | CG | ASN | X | 28 | -9.945 | 86.529 | -22.981 | 1.00 | 33.39 | C |
| ATOM | 5122 | OD1 | ASN | X | 28 | -10.325 | 86.527 | -24.163 | 1.00 | 33.37 | O |
| ATOM | 5123 | ND2 | ASN | X | 28 | -9.394 | 87.594 | -22.408 | 1.00 | 28.13 | N |
| ATOM | 5124 | N | ILE | X | 29 | -10.677 | 84.041 | -24.577 | 1.00 | 26.63 | N |
| ATOM | 5125 | CA | ILE | X | 29 | -11.370 | 83.349 | -25.660 | 1.00 | 33.95 | C |
| ATOM | 5126 | C | ILE | X | 29 | -10.423 | 82.422 | -26.417 | 1.00 | 37.55 | C |
| ATOM | 5127 | O | ILE | X | 29 | -10.814 | 81.335 | -26.857 | 1.00 | 33.77 | O |
| ATOM | 5128 | CB | ILE | X | 29 | -12.029 | 84.355 | -26.611 | 1.00 | 31.78 | C |
| ATOM | 5129 | CG1 | ILE | X | 29 | -13.165 | 85.074 | -25.894 | 1.00 | 37.08 | C |
| ATOM | 5130 | CG2 | ILE | X | 29 | -12.570 | 83.634 | -27.846 | 1.00 | 27.80 | C |
| ATOM | 5131 | CD1 | ILE | X | 29 | -13.921 | 85.975 | -26.801 | 1.00 | 36.60 | C |
| ATOM | 5132 | N | GLY | X | 30 | -9.171 | 82.838 | -26.596 | 1.00 | 38.13 | N |
| ATOM | 5133 | CA | GLY | X | 30 | -8.264 | 82.044 | -27.399 | 1.00 | 32.55 | C |
| ATOM | 5134 | C | GLY | X | 30 | -7.961 | 80.683 | -26.819 | 1.00 | 33.31 | C |
| ATOM | 5135 | O | GLY | X | 30 | -7.589 | 79.781 | -27.559 | 1.00 | 43.57 | O |
| ATOM | 5136 | N | ASN | X | 31 | -8.136 | 80.495 | -25.521 | 1.00 | 40.07 | N |
| ATOM | 5137 | CA | ASN | X | 31 | -7.797 | 79.191 | -24.968 | 1.00 | 37.30 | C |
| ATOM | 5138 | C | ASN | X | 31 | -8.808 | 78.618 | -23.980 | 1.00 | 36.23 | C |
| ATOM | 5139 | O | ASN | X | 31 | -8.500 | 77.622 | -23.324 | 1.00 | 37.83 | O |
| ATOM | 5140 | CB | ASN | X | 31 | -6.405 | 79.245 | -24.343 | 1.00 | 43.50 | C |
| ATOM | 5141 | CG | ASN | X | 31 | -5.314 | 79.117 | -25.408 | 1.00 | 56.73 | C |
| ATOM | 5142 | OD1 | ASN | X | 31 | -5.006 | 78.007 | -25.858 | 1.00 | 58.50 | O |
| ATOM | 5143 | ND2 | ASN | X | 31 | -4.764 | 80.253 | -25.851 | 1.00 | 48.77 | N |
| ATOM | 5144 | N | ASN | X | 32 | -10.015 | 79.157 | -23.886 | 1.00 | 33.81 | N |
| ATOM | 5145 | CA | ASN | X | 32 | -10.979 | 78.603 | -22.954 | 1.00 | 35.74 | C |
| ATOM | 5146 | C | ASN | X | 32 | -12.338 | 78.460 | -23.628 | 1.00 | 33.34 | C |
| ATOM | 5147 | O | ASN | X | 32 | -12.583 | 79.011 | -24.700 | 1.00 | 34.66 | O |
| ATOM | 5148 | CB | ASN | X | 32 | -11.035 | 79.466 | -21.699 | 1.00 | 32.31 | C |
| ATOM | 5149 | CG | ASN | X | 32 | -9.711 | 79.508 | -20.983 | 1.00 | 33.00 | C |
| ATOM | 5150 | OD1 | ASN | X | 32 | -9.339 | 78.559 | -20.300 | 1.00 | 35.81 | O |
| ATOM | 5151 | ND2 | ASN | X | 32 | -8.979 | 80.606 | -21.141 | 1.00 | 40.72 | N |
| ATOM | 5152 | N | TYR | X | 33 | -13.220 | 77.691 | -22.998 | 1.00 | 33.41 | N |
| ATOM | 5153 | CA | TYR | X | 33 | -14.543 | 77.451 | -23.572 | 1.00 | 35.49 | C |
| ATOM | 5154 | C | TYR | X | 33 | -15.355 | 78.735 | -23.542 | 1.00 | 30.39 | C |
| ATOM | 5155 | O | TYR | X | 33 | -15.333 | 79.455 | -22.555 | 1.00 | 29.67 | O |
| ATOM | 5156 | CB | TYR | X | 33 | -15.281 | 76.348 | -22.793 | 1.00 | 28.71 | C |
| ATOM | 5157 | CG | TYR | X | 33 | -14.627 | 74.978 | -22.878 | 1.00 | 33.53 | C |
| ATOM | 5158 | CD1 | TYR | X | 33 | -14.513 | 74.311 | -24.098 | 1.00 | 27.38 | C |
| ATOM | 5159 | CD2 | TYR | X | 33 | -14.115 | 74.356 | -21.740 | 1.00 | 32.47 | C |
| ATOM | 5160 | CE1 | TYR | X | 33 | -13.915 | 73.063 | -24.178 | 1.00 | 30.49 | C |
| ATOM | 5161 | CE2 | TYR | X | 33 | -13.514 | 73.101 | -21.813 | 1.00 | 31.14 | C |
| ATOM | 5162 | CZ | TYR | X | 33 | -13.409 | 72.468 | -23.043 | 1.00 | 31.76 | C |
| ATOM | 5163 | OH | TYR | X | 33 | -12.815 | 71.235 | -23.121 | 1.00 | 31.17 | O |
| ATOM | 5164 | N | VAL | X | 34 | -16.106 | 79.006 | -24.602 | 1.00 | 29.15 | N |
| ATOM | 5165 | CA | VAL | X | 34 | -16.887 | 80.240 | -24.708 | 1.00 | 36.08 | C |
| ATOM | 5166 | C | VAL | X | 34 | -18.366 | 79.954 | -24.434 | 1.00 | 32.87 | C |
| ATOM | 5167 | O | VAL | X | 34 | -18.934 | 79.011 | -24.996 | 1.00 | 30.79 | O |
| ATOM | 5168 | CB | VAL | X | 34 | -16.704 | 80.865 | -26.098 | 1.00 | 33.81 | C |
| ATOM | 5169 | CG1 | VAL | X | 34 | -17.582 | 82.090 | -26.255 | 1.00 | 27.19 | C |
| ATOM | 5170 | CG2 | VAL | X | 34 | -15.243 | 81.172 | -26.332 | 1.00 | 32.31 | C |
| ATOM | 5171 | N | SER | X | 35 | -19.008 | 80.798 | -23.617 | 1.00 | 32.88 | N |
| ATOM | 5172 | CA | SER | X | 35 | -20.444 | 80.696 | -23.349 | 1.00 | 30.32 | C |
| ATOM | 5173 | C | SER | X | 35 | -21.176 | 81.975 | -23.759 | 1.00 | 32.72 | C |
| ATOM | 5174 | O | SER | X | 35 | -20.616 | 83.075 | -23.709 | 1.00 | 28.89 | O |
| ATOM | 5175 | CB | SER | X | 35 | -20.725 | 80.419 | -21.869 | 1.00 | 28.37 | C |
| ATOM | 5176 | OG | SER | X | 35 | -20.265 | 79.144 | -21.480 | 1.00 | 28.41 | O |
| ATOM | 5177 | N | TRP | X | 36 | -22.446 | 81.832 | -24.154 | 1.00 | 28.96 | N |
| ATOM | 5178 | CA | TRP | X | 36 | -23.308 | 82.972 | -24.451 | 1.00 | 27.19 | C |

| | | | | | | | | | | | |
|---|---|---|---|---|---|---|---|---|---|---|---|
| ATOM | 5179 | C | TRP | X | 36 | -24.541 | 82.934 | -23.566 | 1.00 | 32.55 | C |
| ATOM | 5180 | O | TRP | X | 36 | -25.151 | 81.873 | -23.374 | 1.00 | 33.96 | O |
| ATOM | 5181 | CB | TRP | X | 36 | -23.738 | 83.010 | -25.915 | 1.00 | 28.66 | C |
| ATOM | 5182 | CG | TRP | X | 36 | -22.661 | 83.470 | -26.893 | 1.00 | 32.03 | C |
| ATOM | 5183 | CD1 | TRP | X | 36 | -21.775 | 82.676 | -27.576 | 1.00 | 30.86 | C |
| ATOM | 5184 | CD2 | TRP | X | 36 | -22.382 | 84.821 | -27.305 | 1.00 | 29.58 | C |
| ATOM | 5185 | NE1 | TRP | X | 36 | -20.965 | 83.451 | -28.377 | 1.00 | 29.86 | N |
| ATOM | 5186 | CE2 | TRP | X | 36 | -21.320 | 84.767 | -28.231 | 1.00 | 32.46 | C |
| ATOM | 5187 | CE3 | TRP | X | 36 | -22.926 | 86.067 | -26.979 | 1.00 | 30.71 | C |
| ATOM | 5188 | CZ2 | TRP | X | 36 | -20.784 | 85.917 | -28.827 | 1.00 | 34.25 | C |
| ATOM | 5189 | CZ3 | TRP | X | 36 | -22.391 | 87.204 | -27.571 | 1.00 | 32.12 | C |
| ATOM | 5190 | CH2 | TRP | X | 36 | -21.329 | 87.120 | -28.480 | 1.00 | 31.97 | C |
| ATOM | 5191 | N | TYR | X | 37 | -24.909 | 84.105 | -23.040 | 1.00 | 34.18 | N |
| ATOM | 5192 | CA | TYR | X | 37 | -26.054 | 84.256 | -22.152 | 1.00 | 28.55 | C |
| ATOM | 5193 | C | TYR | X | 37 | -27.044 | 85.232 | -22.763 | 1.00 | 30.63 | C |
| ATOM | 5194 | O | TYR | X | 37 | -26.646 | 86.220 | -23.399 | 1.00 | 30.08 | O |
| ATOM | 5195 | CB | TYR | X | 37 | -25.607 | 84.731 | -20.760 | 1.00 | 24.98 | C |
| ATOM | 5196 | CG | TYR | X | 37 | -24.567 | 83.797 | -20.195 | 1.00 | 29.34 | C |
| ATOM | 5197 | CD1 | TYR | X | 37 | -24.941 | 82.601 | -19.573 | 1.00 | 27.60 | C |
| ATOM | 5198 | CD2 | TYR | X | 37 | -23.210 | 84.059 | -20.346 | 1.00 | 27.93 | C |
| ATOM | 5199 | CE1 | TYR | X | 37 | -23.978 | 81.699 | -19.056 | 1.00 | 30.73 | C |
| ATOM | 5200 | CE2 | TYR | X | 37 | -22.234 | 83.172 | -19.828 | 1.00 | 31.38 | C |
| ATOM | 5201 | CZ | TYR | X | 37 | -22.629 | 81.990 | -19.195 | 1.00 | 32.90 | C |
| ATOM | 5202 | OH | TYR | X | 37 | -21.685 | 81.119 | -18.701 | 1.00 | 30.71 | O |
| ATOM | 5203 | N | GLN | X | 38 | -28.331 | 84.926 | -22.592 | 1.00 | 29.50 | N |
| ATOM | 5204 | CA | GLN | X | 38 | -29.430 | 85.780 | -23.026 | 1.00 | 30.90 | C |
| ATOM | 5205 | C | GLN | X | 38 | -30.211 | 86.252 | -21.807 | 1.00 | 31.01 | C |
| ATOM | 5206 | O | GLN | X | 38 | -30.612 | 85.436 | -20.970 | 1.00 | 29.04 | O |
| ATOM | 5207 | CB | GLN | X | 38 | -30.365 | 85.044 | -23.979 | 1.00 | 27.81 | C |
| ATOM | 5208 | CG | GLN | X | 38 | -31.587 | 85.851 | -24.407 | 1.00 | 27.64 | C |
| ATOM | 5209 | CD | GLN | X | 38 | -32.640 | 84.958 | -25.007 | 1.00 | 34.54 | C |
| ATOM | 5210 | OE1 | GLN | X | 38 | -33.141 | 84.043 | -24.329 | 1.00 | 34.50 | O |
| ATOM | 5211 | NE2 | GLN | X | 38 | -32.985 | 85.195 | -26.279 | 1.00 | 26.87 | N |
| ATOM | 5212 | N | GLN | X | 39 | -30.416 | 87.566 | -21.705 | 1.00 | 29.58 | N |
| ATOM | 5213 | CA | GLN | X | 39 | -31.199 | 88.157 | -20.623 | 1.00 | 29.79 | C |
| ATOM | 5214 | C | GLN | X | 39 | -32.378 | 88.907 | -21.252 | 1.00 | 36.36 | C |
| ATOM | 5215 | O | GLN | X | 39 | -32.234 | 90.040 | -21.731 | 1.00 | 37.54 | O |
| ATOM | 5216 | CB | GLN | X | 39 | -30.341 | 89.067 | -19.749 | 1.00 | 26.28 | C |
| ATOM | 5217 | CG | GLN | X | 39 | -31.077 | 89.513 | -18.479 | 1.00 | 31.07 | C |
| ATOM | 5218 | CD | GLN | X | 39 | -30.207 | 90.302 | -17.520 | 1.00 | 33.21 | C |
| ATOM | 5219 | OE1 | GLN | X | 39 | -29.255 | 90.951 | -17.926 | 1.00 | 33.77 | O |
| ATOM | 5220 | NE2 | GLN | X | 39 | -30.542 | 90.260 | -16.241 | 1.00 | 34.37 | N |
| ATOM | 5221 | N | LEU | X | 40 | -33.539 | 88.255 | -21.271 | 1.00 | 32.04 | N |
| ATOM | 5222 | CA | LEU | X | 40 | -34.756 | 88.889 | -21.729 | 1.00 | 34.90 | C |
| ATOM | 5223 | C | LEU | X | 40 | -35.132 | 90.007 | -20.753 | 1.00 | 38.54 | C |
| ATOM | 5224 | O | LEU | X | 40 | -34.759 | 89.948 | -19.575 | 1.00 | 31.68 | O |
| ATOM | 5225 | CB | LEU | X | 40 | -35.872 | 87.851 | -21.832 | 1.00 | 33.07 | C |
| ATOM | 5226 | CG | LEU | X | 40 | -35.490 | 86.643 | -22.697 | 1.00 | 39.00 | C |
| ATOM | 5227 | CD1 | LEU | X | 40 | -36.411 | 85.450 | -22.373 | 1.00 | 38.05 | C |
| ATOM | 5228 | CD2 | LEU | X | 40 | -35.493 | 86.947 | -24.228 | 1.00 | 26.12 | C |
| ATOM | 5229 | N | PRO | X | 41 | -35.826 | 91.047 | -21.227 | 1.00 | 36.64 | N |
| ATOM | 5230 | CA | PRO | X | 41 | -36.146 | 92.208 | -20.371 | 1.00 | 41.13 | C |
| ATOM | 5231 | C | PRO | X | 41 | -36.855 | 91.770 | -19.096 | 1.00 | 38.67 | C |
| ATOM | 5232 | O | PRO | X | 41 | -37.737 | 90.913 | -19.128 | 1.00 | 32.82 | O |
| ATOM | 5233 | CB | PRO | X | 41 | -37.079 | 93.058 | -21.246 | 1.00 | 36.21 | C |
| ATOM | 5234 | CG | PRO | X | 41 | -36.885 | 92.555 | -22.651 | 1.00 | 38.84 | C |
| ATOM | 5235 | CD | PRO | X | 41 | -36.528 | 91.097 | -22.519 | 1.00 | 39.30 | C |
| ATOM | 5236 | N | GLY | X | 42 | -36.410 | 92.308 | -17.961 | 1.00 | 37.25 | N |
| ATOM | 5237 | CA | GLY | X | 42 | -36.981 | 91.946 | -16.678 | 1.00 | 39.06 | C |
| ATOM | 5238 | C | GLY | X | 42 | -36.772 | 90.514 | -16.194 | 1.00 | 47.15 | C |
| ATOM | 5239 | O | GLY | X | 42 | -37.474 | 90.080 | -15.269 | 1.00 | 47.29 | O |
| ATOM | 5240 | N | THR | X | 43 | -35.830 | 89.759 | -16.760 | 1.00 | 42.87 | N |
| ATOM | 5241 | CA | THR | X | 43 | -35.594 | 88.390 | -16.295 | 1.00 | 41.60 | C |
| ATOM | 5242 | C | THR | X | 43 | -34.125 | 88.218 | -15.937 | 1.00 | 34.86 | C |
| ATOM | 5243 | O | THR | X | 43 | -33.283 | 89.070 | -16.225 | 1.00 | 35.66 | O |
| ATOM | 5244 | CB | THR | X | 43 | -35.966 | 87.300 | -17.340 | 1.00 | 38.72 | C |
| ATOM | 5245 | OG1 | THR | X | 43 | -34.965 | 87.232 | -18.372 | 1.00 | 37.04 | O |
| ATOM | 5246 | CG2 | THR | X | 43 | -37.299 | 87.578 | -18.007 | 1.00 | 39.86 | C |
| ATOM | 5247 | N | ALA | X | 44 | -33.838 | 87.056 | -15.351 | 1.00 | 36.79 | N |
| ATOM | 5248 | CA | ALA | X | 44 | -32.495 | 86.614 | -15.069 | 1.00 | 33.26 | C |
| ATOM | 5249 | C | ALA | X | 44 | -31.804 | 86.190 | -16.368 | 1.00 | 34.93 | C |

| | | | | | | | | | | | |
|---|---|---|---|---|---|---|---|---|---|---|---|
| ATOM | 5250 | O | ALA | X | 44 | -32.466 | 85.879 | -17.358 | 1.00 | 32.13 | O |
| ATOM | 5251 | CB | ALA | X | 44 | -32.537 | 85.449 | -14.085 | 1.00 | 29.97 | C |
| ATOM | 5252 | N | PRO | X | 45 | -30.470 | 86.187 | -16.399 | 1.00 | 31.73 | N |
| ATOM | 5253 | CA | PRO | X | 45 | -29.779 | 85.591 | -17.540 | 1.00 | 29.03 | C |
| ATOM | 5254 | C | PRO | X | 45 | -30.152 | 84.124 | -17.700 | 1.00 | 32.97 | C |
| ATOM | 5255 | O | PRO | X | 45 | -30.631 | 83.455 | -16.779 | 1.00 | 27.00 | O |
| ATOM | 5256 | CB | PRO | X | 45 | -28.294 | 85.750 | -17.187 | 1.00 | 29.30 | C |
| ATOM | 5257 | CG | PRO | X | 45 | -28.267 | 86.928 | -16.262 | 1.00 | 32.14 | C |
| ATOM | 5258 | CD | PRO | X | 45 | -29.524 | 86.784 | -15.442 | 1.00 | 28.71 | C |
| ATOM | 5259 | N | LYS | X | 46 | -29.905 | 83.624 | -18.898 | 1.00 | 28.36 | N |
| ATOM | 5260 | CA | LYS | X | 46 | -30.155 | 82.237 | -19.238 | 1.00 | 34.68 | C |
| ATOM | 5261 | C | LYS | X | 46 | -29.009 | 81.792 | -20.131 | 1.00 | 35.47 | C |
| ATOM | 5262 | O | LYS | X | 46 | -28.503 | 82.593 | -20.930 | 1.00 | 27.61 | O |
| ATOM | 5263 | CB | LYS | X | 46 | -31.500 | 82.135 | -19.949 | 1.00 | 31.10 | C |
| ATOM | 5264 | CG | LYS | X | 46 | -31.750 | 80.915 | -20.700 | 1.00 | 41.49 | C |
| ATOM | 5265 | CD | LYS | X | 46 | -32.725 | 81.196 | -21.858 | 1.00 | 47.60 | C |
| ATOM | 5266 | CE | LYS | X | 46 | -33.943 | 81.979 | -21.420 | 1.00 | 48.37 | C |
| ATOM | 5267 | NZ | LYS | X | 46 | -35.081 | 81.729 | -22.345 | 1.00 | 49.13 | N1+ |
| ATOM | 5268 | N | LEU | X | 47 | -28.574 | 80.539 | -19.977 | 1.00 | 28.77 | N |
| ATOM | 5269 | CA | LEU | X | 47 | -27.546 | 80.027 | -20.872 | 1.00 | 24.97 | C |
| ATOM | 5270 | C | LEU | X | 47 | -28.138 | 79.828 | -22.264 | 1.00 | 32.77 | C |
| ATOM | 5271 | O | LEU | X | 47 | -29.190 | 79.200 | -22.426 | 1.00 | 29.18 | O |
| ATOM | 5272 | CB | LEU | X | 47 | -26.955 | 78.730 | -20.347 | 1.00 | 26.07 | C |
| ATOM | 5273 | CG | LEU | X | 47 | -25.872 | 78.105 | -21.231 | 1.00 | 29.09 | C |
| ATOM | 5274 | CD1 | LEU | X | 47 | -24.689 | 79.042 | -21.402 | 1.00 | 29.03 | C |
| ATOM | 5275 | CD2 | LEU | X | 47 | -25.420 | 76.789 | -20.647 | 1.00 | 27.90 | C |
| ATOM | 5276 | N | LEU | X | 48 | -27.482 | 80.418 | -23.260 | 1.00 | 30.28 | N |
| ATOM | 5277 | CA | LEU | X | 48 | -27.901 | 80.361 | -24.651 | 1.00 | 33.70 | C |
| ATOM | 5278 | C | LEU | X | 48 | -27.075 | 79.352 | -25.441 | 1.00 | 38.84 | C |
| ATOM | 5279 | O | LEU | X | 48 | -27.633 | 78.536 | -26.179 | 1.00 | 35.80 | O |
| ATOM | 5280 | CB | LEU | X | 48 | -27.787 | 81.757 | -25.286 | 1.00 | 30.10 | C |
| ATOM | 5281 | CG | LEU | X | 48 | -28.388 | 81.949 | -26.684 | 1.00 | 36.25 | C |
| ATOM | 5282 | CD1 | LEU | X | 48 | -29.916 | 81.905 | -26.686 | 1.00 | 29.13 | C |
| ATOM | 5283 | CD2 | LEU | X | 48 | -27.887 | 83.232 | -27.342 | 1.00 | 32.77 | C |
| ATOM | 5284 | N | LEU | X | 49 | -25.749 | 79.397 | -25.279 | 1.00 | 38.10 | N |
| ATOM | 5285 | CA | LEU | X | 49 | -24.799 | 78.535 | -25.968 | 1.00 | 29.61 | C |
| ATOM | 5286 | C | LEU | X | 49 | -23.642 | 78.253 | -25.028 | 1.00 | 31.16 | C |
| ATOM | 5287 | O | LEU | X | 49 | -23.153 | 79.160 | -24.352 | 1.00 | 32.51 | O |
| ATOM | 5288 | CB | LEU | X | 49 | -24.241 | 79.177 | -27.235 | 1.00 | 29.50 | C |
| ATOM | 5289 | CG | LEU | X | 49 | -25.158 | 79.484 | -28.410 | 1.00 | 35.20 | C |
| ATOM | 5290 | CD1 | LEU | X | 49 | -24.385 | 80.328 | -29.434 | 1.00 | 32.31 | C |
| ATOM | 5291 | CD2 | LEU | X | 49 | -25.624 | 78.172 | -29.024 | 1.00 | 29.29 | C |
| ATOM | 5292 | N | TYR | X | 50 | -23.204 | 77.003 | -24.983 | 1.00 | 27.72 | N |
| ATOM | 5293 | CA | TYR | X | 50 | -21.976 | 76.679 | -24.285 | 1.00 | 28.60 | C |
| ATOM | 5294 | C | TYR | X | 50 | -21.037 | 75.963 | -25.242 | 1.00 | 30.23 | C |
| ATOM | 5295 | O | TYR | X | 50 | -21.429 | 75.569 | -26.346 | 1.00 | 31.08 | O |
| ATOM | 5296 | CB | TYR | X | 50 | -22.250 | 75.864 | -23.018 | 1.00 | 28.65 | C |
| ATOM | 5297 | CG | TYR | X | 50 | -22.900 | 74.531 | -23.227 | 1.00 | 33.55 | C |
| ATOM | 5298 | CD1 | TYR | X | 50 | -24.246 | 74.422 | -23.613 | 1.00 | 37.48 | C |
| ATOM | 5299 | CD2 | TYR | X | 50 | -22.196 | 73.384 | -22.985 | 1.00 | 29.14 | C |
| ATOM | 5300 | CE1 | TYR | X | 50 | -24.833 | 73.183 | -23.799 | 1.00 | 34.48 | C |
| ATOM | 5301 | CE2 | TYR | X | 50 | -22.768 | 72.149 | -23.152 | 1.00 | 39.17 | C |
| ATOM | 5302 | CZ | TYR | X | 50 | -24.076 | 72.041 | -23.550 | 1.00 | 38.17 | C |
| ATOM | 5303 | OH | TYR | X | 50 | -24.590 | 70.771 | -23.700 | 1.00 | 35.08 | O |
| ATOM | 5304 | N | ASP | X | 51 | -19.768 | 75.877 | -24.840 | 1.00 | 31.56 | N |
| ATOM | 5305 | CA | ASP | X | 51 | -18.698 | 75.390 | -25.713 | 1.00 | 29.21 | C |
| ATOM | 5306 | C | ASP | X | 51 | -18.816 | 76.000 | -27.122 | 1.00 | 34.44 | C |
| ATOM | 5307 | O | ASP | X | 51 | -18.908 | 75.308 | -28.144 | 1.00 | 31.23 | O |
| ATOM | 5308 | CB | ASP | X | 51 | -18.699 | 73.865 | -25.753 | 1.00 | 30.93 | C |
| ATOM | 5309 | CG | ASP | X | 51 | -17.509 | 73.308 | -26.507 | 1.00 | 34.52 | C |
| ATOM | 5310 | OD1 | ASP | X | 51 | -16.467 | 74.009 | -26.589 | 1.00 | 38.97 | O |
| ATOM | 5311 | OD2 | ASP | X | 51 | -17.633 | 72.187 | -27.047 | 1.00 | 37.63 | O1- |
| ATOM | 5312 | N | SER | X | 52 | -18.882 | 77.333 | -27.151 | 1.00 | 29.36 | N |
| ATOM | 5313 | CA | SER | X | 52 | -18.957 | 78.121 | -28.379 | 1.00 | 32.69 | C |
| ATOM | 5314 | C | SER | X | 52 | -20.258 | 77.970 | -29.163 | 1.00 | 32.50 | C |
| ATOM | 5315 | O | SER | X | 52 | -20.805 | 78.977 | -29.637 | 1.00 | 32.67 | O |
| ATOM | 5316 | CB | SER | X | 52 | -17.781 | 77.808 | -29.305 | 1.00 | 32.99 | C |
| ATOM | 5317 | OG | SER | X | 52 | -16.569 | 78.277 | -28.744 | 1.00 | 42.26 | O |
| ATOM | 5318 | N | ASN | X | 53 | -20.772 | 76.746 | -29.333 | 1.00 | 31.33 | N |
| ATOM | 5319 | CA | ASN | X | 53 | -21.880 | 76.604 | -30.271 | 1.00 | 33.53 | C |
| ATOM | 5320 | C | ASN | X | 53 | -22.896 | 75.532 | -29.886 | 1.00 | 36.41 | C |

| | | | | | | | | | | | |
|---|---|---|---|---|---|---|---|---|---|---|---|
| ATOM | 5321 | O | ASN | X | 53 | -23.725 | 75.172 | -30.735 | 1.00 | 34.64 | O |
| ATOM | 5322 | CB | ASN | X | 53 | -21.326 | 76.305 | -31.671 | 1.00 | 29.24 | C |
| ATOM | 5323 | CG | ASN | X | 53 | -20.625 | 74.953 | -31.730 | 1.00 | 38.39 | C |
| ATOM | 5324 | OD1 | ASN | X | 53 | -20.519 | 74.254 | -30.721 | 1.00 | 35.56 | O |
| ATOM | 5325 | ND2 | ASN | X | 53 | -20.163 | 74.573 | -32.906 | 1.00 | 35.38 | N |
| ATOM | 5326 | N | LYS | X | 54 | -22.879 | 75.013 | -28.657 | 1.00 | 37.93 | N |
| ATOM | 5327 | CA | LYS | X | 54 | -23.821 | 73.978 | -28.239 | 1.00 | 37.84 | C |
| ATOM | 5328 | C | LYS | X | 54 | -25.063 | 74.592 | -27.605 | 1.00 | 33.93 | C |
| ATOM | 5329 | O | LYS | X | 54 | -24.957 | 75.380 | -26.656 | 1.00 | 33.00 | O |
| ATOM | 5330 | CB | LYS | X | 54 | -23.165 | 73.014 | -27.250 | 1.00 | 34.82 | C |
| ATOM | 5331 | CG | LYS | X | 54 | -24.025 | 71.814 | -26.907 | 1.00 | 36.40 | C |
| ATOM | 5332 | CD | LYS | X | 54 | -24.191 | 70.937 | -28.131 | 1.00 | 37.62 | C |
| ATOM | 5333 | CE | LYS | X | 54 | -25.209 | 69.832 | -27.894 | 1.00 | 33.30 | C |
| ATOM | 5334 | NZ | LYS | X | 54 | -25.145 | 68.918 | -29.053 | 1.00 | 47.57 | N1+ |
| ATOM | 5335 | N | AARG | X | 55 | -26.235 | 74.212 | -28.121 | 0.50 | 35.72 | N |
| ATOM | 5336 | CA | AARG | X | 55 | -27.511 | 74.703 | -27.600 | 0.50 | 37.84 | C |
| ATOM | 5337 | C | AARG | X | 55 | -27.955 | 73.849 | -26.418 | 0.50 | 37.19 | C |
| ATOM | 5338 | O | AARG | X | 55 | -28.052 | 72.622 | -26.555 | 0.50 | 39.62 | O |
| ATOM | 5339 | CB | AARG | X | 55 | -28.585 | 74.683 | -28.683 | 0.50 | 35.53 | C |
| ATOM | 5340 | CG | AARG | X | 55 | -28.487 | 75.808 | -29.722 | 0.50 | 38.76 | C |
| ATOM | 5341 | CD | AARG | X | 55 | -29.610 | 75.753 | -30.781 | 0.50 | 37.60 | C |
| ATOM | 5342 | NE | AARG | X | 55 | -29.427 | 74.636 | -31.705 | 0.50 | 42.24 | N |
| ATOM | 5343 | CZ | AARG | X | 55 | -30.198 | 73.555 | -31.759 | 0.50 | 40.39 | C |
| ATOM | 5344 | NH1 | AARG | X | 55 | -29.919 | 72.598 | -32.628 | 0.50 | 43.66 | N1+ |
| ATOM | 5345 | NH2 | AARG | X | 55 | -31.248 | 73.434 | -30.961 | 0.50 | 39.76 | N |
| ATOM | 5346 | N | BARG | X | 55 | -26.235 | 74.213 | -28.110 | 0.50 | 35.73 | N |
| ATOM | 5347 | CA | BARG | X | 55 | -27.499 | 74.742 | -27.594 | 0.50 | 37.86 | C |
| ATOM | 5348 | C | BARG | X | 55 | -27.999 | 73.875 | -26.441 | 0.50 | 37.17 | C |
| ATOM | 5349 | O | BARG | X | 55 | -28.165 | 72.661 | -26.619 | 0.50 | 39.65 | O |
| ATOM | 5350 | CB | BARG | X | 55 | -28.548 | 74.810 | -28.701 | 0.50 | 35.58 | C |
| ATOM | 5351 | CG | BARG | X | 55 | -28.335 | 75.971 | -29.695 | 0.50 | 38.67 | C |
| ATOM | 5352 | CD | BARG | X | 55 | -29.274 | 75.940 | -30.919 | 0.50 | 37.26 | C |
| ATOM | 5353 | NE | BARG | X | 55 | -28.921 | 74.866 | -31.845 | 0.50 | 42.86 | N |
| ATOM | 5354 | CZ | BARG | X | 55 | -28.062 | 74.983 | -32.859 | 0.50 | 43.30 | C |
| ATOM | 5355 | NH1 | BARG | X | 55 | -27.465 | 76.140 | -33.108 | 0.50 | 28.23 | N1+ |
| ATOM | 5356 | NH2 | BARG | X | 55 | -27.803 | 73.928 | -33.632 | 0.50 | 47.26 | N |
| ATOM | 5357 | N | PRO | X | 56 | -28.209 | 74.432 | -25.251 | 1.00 | 39.61 | N |
| ATOM | 5358 | CA | PRO | X | 56 | -28.924 | 73.683 | -24.208 | 1.00 | 37.27 | C |
| ATOM | 5359 | C | PRO | X | 56 | -30.328 | 73.384 | -24.708 | 1.00 | 37.17 | C |
| ATOM | 5360 | O | PRO | X | 56 | -30.836 | 74.048 | -25.614 | 1.00 | 35.06 | O |
| ATOM | 5361 | CB | PRO | X | 56 | -28.960 | 74.643 | -23.020 | 1.00 | 32.87 | C |
| ATOM | 5362 | CG | PRO | X | 56 | -28.041 | 75.768 | -23.374 | 1.00 | 39.79 | C |
| ATOM | 5363 | CD | PRO | X | 56 | -27.841 | 75.794 | -24.846 | 1.00 | 34.03 | C |
| ATOM | 5364 | N | SER | X | 57 | -30.973 | 72.378 | -24.127 | 1.00 | 35.37 | N |
| ATOM | 5365 | CA | SER | X | 57 | -32.324 | 72.095 | -24.603 | 1.00 | 45.77 | C |
| ATOM | 5366 | C | SER | X | 57 | -33.240 | 73.271 | -24.286 | 1.00 | 39.53 | C |
| ATOM | 5367 | O | SER | X | 57 | -33.082 | 73.960 | -23.274 | 1.00 | 42.51 | O |
| ATOM | 5368 | CB | SER | X | 57 | -32.860 | 70.777 | -24.033 | 1.00 | 44.76 | C |
| ATOM | 5369 | OG | SER | X | 57 | -32.486 | 70.611 | -22.686 | 1.00 | 57.37 | O |
| ATOM | 5370 | N | GLY | X | 58 | -34.149 | 73.551 | -25.208 | 1.00 | 46.11 | N |
| ATOM | 5371 | CA | GLY | X | 58 | -35.017 | 74.704 | -25.108 | 1.00 | 38.41 | C |
| ATOM | 5372 | C | GLY | X | 58 | -34.575 | 75.906 | -25.915 | 1.00 | 46.36 | C |
| ATOM | 5373 | O | GLY | X | 58 | -35.360 | 76.846 | -26.056 | 1.00 | 47.75 | O |
| ATOM | 5374 | N | ILE | X | 59 | -33.356 | 75.916 | -26.442 | 1.00 | 38.47 | N |
| ATOM | 5375 | CA | ILE | X | 59 | -32.858 | 77.042 | -27.231 | 1.00 | 39.45 | C |
| ATOM | 5376 | C | ILE | X | 59 | -33.096 | 76.740 | -28.706 | 1.00 | 38.36 | C |
| ATOM | 5377 | O | ILE | X | 59 | -32.567 | 75.743 | -29.218 | 1.00 | 41.39 | O |
| ATOM | 5378 | CB | ILE | X | 59 | -31.372 | 77.301 | -26.950 | 1.00 | 41.60 | C |
| ATOM | 5379 | CG1 | ILE | X | 59 | -31.165 | 77.604 | -25.460 | 1.00 | 39.46 | C |
| ATOM | 5380 | CG2 | ILE | X | 59 | -30.858 | 78.449 | -27.839 | 1.00 | 36.16 | C |
| ATOM | 5381 | CD1 | ILE | X | 59 | -31.957 | 78.855 | -24.970 | 1.00 | 33.58 | C |
| ATOM | 5382 | N | PRO | X | 60 | -33.829 | 77.583 | -29.429 | 1.00 | 38.04 | N |
| ATOM | 5383 | CA | PRO | X | 60 | -34.124 | 77.311 | -30.841 | 1.00 | 40.67 | C |
| ATOM | 5384 | C | PRO | X | 60 | -32.877 | 77.315 | -31.712 | 1.00 | 41.79 | C |
| ATOM | 5385 | O | PRO | X | 60 | -31.899 | 78.020 | -31.439 | 1.00 | 39.12 | O |
| ATOM | 5386 | CB | PRO | X | 60 | -35.047 | 78.468 | -31.240 | 1.00 | 37.50 | C |
| ATOM | 5387 | CG | PRO | X | 60 | -35.340 | 79.184 | -30.024 | 1.00 | 40.79 | C |
| ATOM | 5388 | CD | PRO | X | 60 | -34.325 | 78.891 | -29.004 | 1.00 | 41.33 | C |
| ATOM | 5389 | N | ALA | X | 61 | -32.956 | 76.574 | -32.821 | 1.00 | 41.88 | N |
| ATOM | 5390 | CA | ALA | X | 61 | -31.816 | 76.464 | -33.727 | 1.00 | 41.28 | C |
| ATOM | 5391 | C | ALA | X | 61 | -31.500 | 77.762 | -34.449 | 1.00 | 37.80 | C |

| | | | | | | | | | | | |
|---|---|---|---|---|---|---|---|---|---|---|---|
| ATOM | 5392 | O | ALA | X | 61 | -30.440 | 77.855 | -35.066 | 1.00 | 37.27 | O |
| ATOM | 5393 | CB | ALA | X | 61 | -32.043 | 75.362 | -34.759 | 1.00 | 34.30 | C |
| ATOM | 5394 | N | ARG | X | 62 | -32.356 | 78.779 | -34.366 | 1.00 | 38.43 | N |
| ATOM | 5395 | CA | ARG | X | 62 | -31.978 | 80.019 | -35.031 | 1.00 | 42.28 | C |
| ATOM | 5396 | C | ARG | X | 62 | -30.888 | 80.776 | -34.273 | 1.00 | 39.16 | C |
| ATOM | 5397 | O | ARG | X | 62 | -30.360 | 81.763 | -34.795 | 1.00 | 42.60 | O |
| ATOM | 5398 | CB | ARG | X | 62 | -33.215 | 80.894 | -35.260 | 1.00 | 40.18 | C |
| ATOM | 5399 | CG | ARG | X | 62 | -33.956 | 81.319 | -34.029 | 1.00 | 45.22 | C |
| ATOM | 5400 | CD | ARG | X | 62 | -35.169 | 82.228 | -34.403 | 1.00 | 57.00 | C |
| ATOM | 5401 | NE | ARG | X | 62 | -35.710 | 82.874 | -33.214 | 1.00 | 45.98 | N |
| ATOM | 5402 | CZ | ARG | X | 62 | -36.485 | 82.241 | -32.345 | 1.00 | 47.84 | C |
| ATOM | 5403 | NH1 | ARG | X | 62 | -36.807 | 80.976 | -32.560 | 1.00 | 50.34 | N1+ |
| ATOM | 5404 | NH2 | ARG | X | 62 | -36.923 | 82.855 | -31.261 | 1.00 | 51.21 | N |
| ATOM | 5405 | N | PHE | X | 63 | -30.522 | 80.330 | -33.074 | 1.00 | 40.25 | N |
| ATOM | 5406 | CA | PHE | X | 63 | -29.323 | 80.816 | -32.410 | 1.00 | 41.09 | C |
| ATOM | 5407 | C | PHE | X | 63 | -28.171 | 79.886 | -32.756 | 1.00 | 41.66 | C |
| ATOM | 5408 | O | PHE | X | 63 | -28.299 | 78.666 | -32.622 | 1.00 | 35.82 | O |
| ATOM | 5409 | CB | PHE | X | 63 | -29.510 | 80.887 | -30.891 | 1.00 | 33.46 | C |
| ATOM | 5410 | CG | PHE | X | 63 | -30.586 | 81.832 | -30.473 | 1.00 | 36.45 | C |
| ATOM | 5411 | CD1 | PHE | X | 63 | -30.310 | 83.184 | -30.280 | 1.00 | 39.83 | C |
| ATOM | 5412 | CD2 | PHE | X | 63 | -31.884 | 81.377 | -30.287 | 1.00 | 34.93 | C |
| ATOM | 5413 | CE1 | PHE | X | 63 | -31.325 | 84.085 | -29.906 | 1.00 | 36.05 | C |
| ATOM | 5414 | CE2 | PHE | X | 63 | -32.889 | 82.251 | -29.923 | 1.00 | 38.93 | C |
| ATOM | 5415 | CZ | PHE | X | 63 | -32.614 | 83.614 | -29.734 | 1.00 | 35.26 | C |
| ATOM | 5416 | N | SER | X | 64 | -27.053 | 80.455 | -33.207 | 1.00 | 33.96 | N |
| ATOM | 5417 | CA | SER | X | 64 | -25.866 | 79.646 | -33.436 | 1.00 | 36.60 | C |
| ATOM | 5418 | C | SER | X | 64 | -24.626 | 80.455 | -33.101 | 1.00 | 33.97 | C |
| ATOM | 5419 | O | SER | X | 64 | -24.663 | 81.684 | -32.984 | 1.00 | 37.04 | O |
| ATOM | 5420 | CB | SER | X | 64 | -25.792 | 79.134 | -34.878 | 1.00 | 33.50 | C |
| ATOM | 5421 | OG | SER | X | 64 | -25.828 | 80.219 | -35.797 | 1.00 | 41.55 | O |
| ATOM | 5422 | N | GLY | X | 65 | -23.514 | 79.743 | -32.965 | 1.00 | 33.67 | N |
| ATOM | 5423 | CA | GLY | X | 65 | -22.271 | 80.366 | -32.569 | 1.00 | 32.37 | C |
| ATOM | 5424 | C | GLY | X | 65 | -21.099 | 79.800 | -33.342 | 1.00 | 37.26 | C |
| ATOM | 5425 | O | GLY | X | 65 | -21.144 | 78.694 | -33.885 | 1.00 | 37.56 | O |
| ATOM | 5426 | N | SER | X | 66 | -20.044 | 80.596 | -33.386 | 1.00 | 36.95 | N |
| ATOM | 5427 | CA | SER | X | 66 | -18.782 | 80.154 | -33.937 | 1.00 | 39.89 | C |
| ATOM | 5428 | C | SER | X | 66 | -17.675 | 80.805 | -33.137 | 1.00 | 38.97 | C |
| ATOM | 5429 | O | SER | X | 66 | -17.884 | 81.794 | -32.427 | 1.00 | 42.94 | O |
| ATOM | 5430 | CB | SER | X | 66 | -18.645 | 80.521 | -35.416 | 1.00 | 43.54 | C |
| ATOM | 5431 | OG | SER | X | 66 | -18.842 | 81.914 | -35.579 | 1.00 | 44.16 | O |
| ATOM | 5432 | N | LYS | X | 67 | -16.493 | 80.236 | -33.268 | 1.00 | 37.88 | N |
| ATOM | 5433 | CA | LYS | X | 67 | -15.318 | 80.724 | -32.578 | 1.00 | 38.71 | C |
| ATOM | 5434 | C | LYS | X | 67 | -14.182 | 80.683 | -33.584 | 1.00 | 41.62 | C |
| ATOM | 5435 | O | LYS | X | 67 | -14.049 | 79.712 | -34.332 | 1.00 | 36.78 | O |
| ATOM | 5436 | CB | LYS | X | 67 | -14.996 | 79.866 | -31.337 | 1.00 | 34.63 | C |
| ATOM | 5437 | CG | LYS | X | 67 | -13.718 | 80.278 | -30.577 | 1.00 | 39.36 | C |
| ATOM | 5438 | CD | LYS | X | 67 | -13.483 | 79.408 | -29.335 | 1.00 | 43.58 | C |
| ATOM | 5439 | CE | LYS | X | 67 | -12.029 | 79.470 | -28.865 | 1.00 | 49.06 | C |
| ATOM | 5440 | NZ | LYS | X | 67 | -11.798 | 78.829 | -27.509 | 1.00 | 45.19 | N1+ |
| ATOM | 5441 | N | SER | X | 68 | -13.372 | 81.733 | -33.603 | 1.00 | 32.64 | N |
| ATOM | 5442 | CA | SER | X | 68 | -12.213 | 81.766 | -34.484 | 1.00 | 39.05 | C |
| ATOM | 5443 | C | SER | X | 68 | -11.107 | 82.533 | -33.774 | 1.00 | 44.53 | C |
| ATOM | 5444 | O | SER | X | 68 | -11.196 | 83.760 | -33.617 | 1.00 | 37.82 | O |
| ATOM | 5445 | CB | SER | X | 68 | -12.578 | 82.412 | -35.816 | 1.00 | 40.60 | C |
| ATOM | 5446 | OG | SER | X | 68 | -11.432 | 82.493 | -36.637 | 1.00 | 69.58 | O |
| ATOM | 5447 | N | GLY | X | 69 | -10.073 | 81.825 | -33.344 | 1.00 | 41.45 | N |
| ATOM | 5448 | CA | GLY | X | 69 | -8.973 | 82.520 | -32.683 | 1.00 | 38.45 | C |
| ATOM | 5449 | C | GLY | X | 69 | -9.424 | 83.162 | -31.382 | 1.00 | 38.09 | C |
| ATOM | 5450 | O | GLY | X | 69 | -9.944 | 82.493 | -30.485 | 1.00 | 40.05 | O |
| ATOM | 5451 | N | THR | X | 70 | -9.248 | 84.479 | -31.260 | 1.00 | 38.95 | N |
| ATOM | 5452 | CA | THR | X | 70 | -9.575 | 85.191 | -30.033 | 1.00 | 31.37 | C |
| ATOM | 5453 | C | THR | X | 70 | -10.914 | 85.919 | -30.101 | 1.00 | 36.24 | C |
| ATOM | 5454 | O | THR | X | 70 | -11.192 | 86.796 | -29.271 | 1.00 | 37.77 | O |
| ATOM | 5455 | CB | THR | X | 70 | -8.458 | 86.171 | -29.692 | 1.00 | 36.81 | C |
| ATOM | 5456 | OG1 | THR | X | 70 | -8.349 | 87.134 | -30.744 | 1.00 | 30.74 | O |
| ATOM | 5457 | CG2 | THR | X | 70 | -7.134 | 85.414 | -29.544 | 1.00 | 25.19 | C |
| ATOM | 5458 | N | SER | X | 71 | -11.765 | 85.574 | -31.048 | 1.00 | 37.57 | N |
| ATOM | 5459 | CA | SER | X | 71 | -13.081 | 86.175 | -31.072 | 1.00 | 38.07 | C |
| ATOM | 5460 | C | SER | X | 71 | -14.125 | 85.082 | -31.262 | 1.00 | 38.54 | C |
| ATOM | 5461 | O | SER | X | 71 | -13.834 | 83.992 | -31.759 | 1.00 | 37.91 | O |
| ATOM | 5462 | CB | SER | X | 71 | -13.185 | 87.249 | -32.162 | 1.00 | 40.23 | C |

| | | | | | | | | | | | |
|---|---|---|---|---|---|---|---|---|---|---|---|
| ATOM | 5463 | OG | SER | X | 71 | -13.241 | 86.662 | -33.448 | 1.00 | 48.33 | O |
| ATOM | 5464 | N | ALA | X | 72 | -15.344 | 85.387 | -30.821 | 1.00 | 30.70 | N |
| ATOM | 5465 | CA | ALA | X | 72 | -16.491 | 84.505 | -30.944 | 1.00 | 32.65 | C |
| ATOM | 5466 | C | ALA | X | 72 | -17.649 | 85.344 | -31.464 | 1.00 | 33.91 | C |
| ATOM | 5467 | O | ALA | X | 72 | -17.724 | 86.538 | -31.199 | 1.00 | 35.37 | O |
| ATOM | 5468 | CB | ALA | X | 72 | -16.847 | 83.861 | -29.596 | 1.00 | 28.85 | C |
| ATOM | 5469 | N | ATHR | X | 73 | -18.543 | 84.722 | -32.219 | 0.60 | 34.81 | N |
| ATOM | 5470 | CA | ATHR | X | 73 | -19.686 | 85.428 | -32.781 | 0.60 | 36.26 | C |
| ATOM | 5471 | C | ATHR | X | 73 | -20.968 | 84.644 | -32.530 | 0.60 | 38.23 | C |
| ATOM | 5472 | O | ATHR | X | 73 | -21.021 | 83.423 | -32.730 | 0.60 | 36.94 | O |
| ATOM | 5473 | CB | ATHR | X | 73 | -19.528 | 85.662 | -34.285 | 0.60 | 37.26 | C |
| ATOM | 5474 | OG1 | ATHR | X | 73 | -19.317 | 84.403 | -34.929 | 0.60 | 39.56 | O |
| ATOM | 5475 | CG2 | ATHR | X | 73 | -18.350 | 86.575 | -34.575 | 0.60 | 38.40 | C |
| ATOM | 5476 | N | BTHR | X | 73 | -18.549 | 84.710 | -32.211 | 0.40 | 34.91 | N |
| ATOM | 5477 | CA | BTHR | X | 73 | -19.684 | 85.400 | -32.812 | 0.40 | 36.39 | C |
| ATOM | 5478 | C | BTHR | X | 73 | -20.970 | 84.636 | -32.529 | 0.40 | 38.11 | C |
| ATOM | 5479 | O | BTHR | X | 73 | -21.026 | 83.413 | -32.708 | 0.40 | 36.77 | O |
| ATOM | 5480 | CB | BTHR | X | 73 | -19.509 | 85.557 | -34.327 | 0.40 | 37.24 | C |
| ATOM | 5481 | OG1 | BTHR | X | 73 | -18.230 | 86.138 | -34.608 | 0.40 | 37.63 | O |
| ATOM | 5482 | CG2 | BTHR | X | 73 | -20.620 | 86.434 | -34.910 | 0.40 | 32.90 | C |
| ATOM | 5483 | N | LEU | X | 74 | -21.997 | 85.359 | -32.097 | 1.00 | 35.38 | N |
| ATOM | 5484 | CA | LEU | X | 74 | -23.326 | 84.804 | -31.943 | 1.00 | 34.79 | C |
| ATOM | 5485 | C | LEU | X | 74 | -24.147 | 85.205 | -33.156 | 1.00 | 36.97 | C |
| ATOM | 5486 | O | LEU | X | 74 | -24.156 | 86.379 | -33.545 | 1.00 | 33.92 | O |
| ATOM | 5487 | CB | LEU | X | 74 | -23.986 | 85.314 | -30.665 | 1.00 | 32.61 | C |
| ATOM | 5488 | CG | LEU | X | 74 | -25.507 | 85.206 | -30.626 | 1.00 | 31.72 | C |
| ATOM | 5489 | CD1 | LEU | X | 74 | -25.882 | 83.725 | -30.473 | 1.00 | 30.32 | C |
| ATOM | 5490 | CD2 | LEU | X | 74 | -26.036 | 86.026 | -29.454 | 1.00 | 27.99 | C |
| ATOM | 5491 | N | GLY | X | 75 | -24.840 | 84.247 | -33.745 | 1.00 | 36.52 | N |
| ATOM | 5492 | CA | GLY | X | 75 | -25.703 | 84.512 | -34.884 | 1.00 | 34.24 | C |
| ATOM | 5493 | C | GLY | X | 75 | -27.148 | 84.242 | -34.527 | 1.00 | 37.87 | C |
| ATOM | 5494 | O | GLY | X | 75 | -27.451 | 83.245 | -33.871 | 1.00 | 37.63 | O |
| ATOM | 5495 | N | ILE | X | 76 | -28.035 | 85.141 | -34.957 | 1.00 | 39.75 | N |
| ATOM | 5496 | CA | ILE | X | 76 | -29.479 | 84.985 | -34.802 | 1.00 | 41.67 | C |
| ATOM | 5497 | C | ILE | X | 76 | -30.121 | 85.182 | -36.168 | 1.00 | 42.37 | C |
| ATOM | 5498 | O | ILE | X | 76 | -30.094 | 86.290 | -36.715 | 1.00 | 44.35 | O |
| ATOM | 5499 | CB | ILE | X | 76 | -30.078 | 85.974 | -33.790 | 1.00 | 40.33 | C |
| ATOM | 5500 | CG1 | ILE | X | 76 | -29.186 | 86.075 | -32.552 | 1.00 | 41.66 | C |
| ATOM | 5501 | CG2 | ILE | X | 76 | -31.481 | 85.526 | -33.397 | 1.00 | 35.42 | C |
| ATOM | 5502 | CD1 | ILE | X | 76 | -29.726 | 86.959 | -31.465 | 1.00 | 37.72 | C |
| ATOM | 5503 | N | THR | X | 77 | -30.686 | 84.119 | -36.725 | 1.00 | 43.91 | N |
| ATOM | 5504 | CA | THR | X | 77 | -31.472 | 84.236 | -37.948 | 1.00 | 48.21 | C |
| ATOM | 5505 | C | THR | X | 77 | -32.958 | 84.232 | -37.615 | 1.00 | 43.41 | C |
| ATOM | 5506 | O | THR | X | 77 | -33.376 | 83.754 | -36.561 | 1.00 | 53.87 | O |
| ATOM | 5507 | CB | THR | X | 77 | -31.176 | 83.090 | -38.915 | 1.00 | 48.60 | C |
| ATOM | 5508 | OG1 | THR | X | 77 | -31.675 | 81.879 | -38.346 | 1.00 | 50.81 | O |
| ATOM | 5509 | CG2 | THR | X | 77 | -29.695 | 82.947 | -39.136 | 1.00 | 39.95 | C |
| ATOM | 5510 | N | GLY | X | 78 | -33.757 | 84.758 | -38.536 | 1.00 | 46.47 | N |
| ATOM | 5511 | CA | GLY | X | 78 | -35.202 | 84.734 | -38.389 | 1.00 | 39.22 | C |
| ATOM | 5512 | C | GLY | X | 78 | -35.720 | 85.391 | -37.128 | 1.00 | 45.27 | C |
| ATOM | 5513 | O | GLY | X | 78 | -36.522 | 84.791 | -36.407 | 1.00 | 43.17 | O |
| ATOM | 5514 | N | LEU | X | 79 | -35.270 | 86.624 | -36.870 | 1.00 | 46.54 | N |
| ATOM | 5515 | CA | LEU | X | 79 | -35.555 | 87.331 | -35.624 | 1.00 | 38.18 | C |
| ATOM | 5516 | C | LEU | X | 79 | -37.039 | 87.325 | -35.285 | 1.00 | 35.99 | C |
| ATOM | 5517 | O | LEU | X | 79 | -37.888 | 87.609 | -36.131 | 1.00 | 36.59 | O |
| ATOM | 5518 | CB | LEU | X | 79 | -35.059 | 88.776 | -35.730 | 1.00 | 39.04 | C |
| ATOM | 5519 | CG | LEU | X | 79 | -33.807 | 89.295 | -34.996 | 1.00 | 40.52 | C |
| ATOM | 5520 | CD1 | LEU | X | 79 | -33.440 | 88.520 | -33.740 | 1.00 | 39.26 | C |
| ATOM | 5521 | CD2 | LEU | X | 79 | -32.618 | 89.397 | -35.910 | 1.00 | 54.14 | C |
| ATOM | 5522 | N | GLN | X | 80 | -37.345 | 87.044 | -34.026 | 1.00 | 38.14 | N |
| ATOM | 5523 | CA | GLN | X | 80 | -38.697 | 87.144 | -33.497 | 1.00 | 39.98 | C |
| ATOM | 5524 | C | GLN | X | 80 | -38.700 | 88.184 | -32.389 | 1.00 | 41.62 | C |
| ATOM | 5525 | O | GLN | X | 80 | -37.668 | 88.422 | -31.753 | 1.00 | 36.20 | O |
| ATOM | 5526 | CB | GLN | X | 80 | -39.186 | 85.813 | -32.942 | 1.00 | 33.25 | C |
| ATOM | 5527 | CG | GLN | X | 80 | -39.044 | 84.680 | -33.904 | 1.00 | 43.46 | C |
| ATOM | 5528 | CD | GLN | X | 80 | -39.580 | 83.358 | -33.355 | 1.00 | 50.00 | C |
| ATOM | 5529 | OE1 | GLN | X | 80 | -39.521 | 82.335 | -34.031 | 1.00 | 49.67 | O |
| ATOM | 5530 | NE2 | GLN | X | 80 | -40.101 | 83.378 | -32.131 | 1.00 | 47.24 | N |
| ATOM | 5531 | N | THR | X | 81 | -39.865 | 88.806 | -32.161 | 1.00 | 42.32 | N |
| ATOM | 5532 | CA | THR | X | 81 | -39.935 | 89.865 | -31.159 | 1.00 | 40.35 | C |
| ATOM | 5533 | C | THR | X | 81 | -39.450 | 89.373 | -29.794 | 1.00 | 36.41 | C |

| | | | | | | | | | | | |
|---|---|---|---|---|---|---|---|---|---|---|---|
| ATOM | 5534 | O | THR | X | 81 | -38.817 | 90.132 | -29.050 | 1.00 | 35.09 | O |
| ATOM | 5535 | CB | THR | X | 81 | -41.369 | 90.415 | -31.067 | 1.00 | 40.85 | C |
| ATOM | 5536 | OG1 | THR | X | 81 | -42.261 | 89.347 | -30.772 | 1.00 | 57.82 | O |
| ATOM | 5537 | CG2 | THR | X | 81 | -41.798 | 90.998 | -32.377 | 1.00 | 43.15 | C |
| ATOM | 5538 | N | GLY | X | 82 | -39.684 | 88.094 | -29.474 | 1.00 | 36.68 | N |
| ATOM | 5539 | CA | GLY | X | 82 | -39.232 | 87.486 | -28.236 | 1.00 | 28.64 | C |
| ATOM | 5540 | C | GLY | X | 82 | -37.724 | 87.287 | -28.129 | 1.00 | 37.77 | C |
| ATOM | 5541 | O | GLY | X | 82 | -37.248 | 86.752 | -27.124 | 1.00 | 34.17 | O |
| ATOM | 5542 | N | ASP | X | 83 | -36.959 | 87.652 | -29.150 | 1.00 | 30.06 | N |
| ATOM | 5543 | CA | ASP | X | 83 | -35.509 | 87.630 | -29.043 | 1.00 | 32.84 | C |
| ATOM | 5544 | C | ASP | X | 83 | -34.923 | 88.941 | -28.517 | 1.00 | 34.01 | C |
| ATOM | 5545 | O | ASP | X | 83 | -33.729 | 88.984 | -28.208 | 1.00 | 31.84 | O |
| ATOM | 5546 | CB | ASP | X | 83 | -34.907 | 87.303 | -30.404 | 1.00 | 31.73 | C |
| ATOM | 5547 | CG | ASP | X | 83 | -35.427 | 85.990 | -30.952 | 1.00 | 41.54 | C |
| ATOM | 5548 | OD1 | ASP | X | 83 | -35.746 | 85.088 | -30.133 | 1.00 | 36.80 | O |
| ATOM | 5549 | OD2 | ASP | X | 83 | -35.522 | 85.868 | -32.194 | 1.00 | 42.52 | O1- |
| ATOM | 5550 | N | GLU | X | 84 | -35.724 | 90.002 | -28.424 | 1.00 | 31.24 | N |
| ATOM | 5551 | CA | GLU | X | 84 | -35.262 | 91.272 | -27.871 | 1.00 | 37.59 | C |
| ATOM | 5552 | C | GLU | X | 84 | -34.719 | 91.031 | -26.459 | 1.00 | 32.26 | C |
| ATOM | 5553 | O | GLU | X | 84 | -35.402 | 90.452 | -25.609 | 1.00 | 35.56 | O |
| ATOM | 5554 | CB | GLU | X | 84 | -36.416 | 92.283 | -27.890 | 1.00 | 30.20 | C |
| ATOM | 5555 | CG | GLU | X | 84 | -36.070 | 93.629 | -27.345 | 1.00 | 45.30 | C |
| ATOM | 5556 | CD | GLU | X | 84 | -36.947 | 94.775 | -27.890 | 1.00 | 45.92 | C |
| ATOM | 5557 | OE1 | GLU | X | 84 | -37.343 | 95.625 | -27.064 | 1.00 | 55.26 | O |
| ATOM | 5558 | OE2 | GLU | X | 84 | -37.238 | 94.832 | -29.112 | 1.00 | 39.84 | O1- |
| ATOM | 5559 | N | ALA | X | 85 | -33.455 | 91.385 | -26.241 | 1.00 | 33.55 | N |
| ATOM | 5560 | CA | ALA | X | 85 | -32.741 | 90.944 | -25.047 | 1.00 | 30.96 | C |
| ATOM | 5561 | C | ALA | X | 85 | -31.361 | 91.575 | -25.034 | 1.00 | 28.54 | C |
| ATOM | 5562 | O | ALA | X | 85 | -30.916 | 92.168 | -26.021 | 1.00 | 32.81 | O |
| ATOM | 5563 | CB | ALA | X | 85 | -32.594 | 89.409 | -24.997 | 1.00 | 30.64 | C |
| ATOM | 5564 | N | ASP | X | 86 | -30.680 | 91.413 | -23.903 | 1.00 | 29.41 | N |
| ATOM | 5565 | CA | ASP | X | 86 | -29.249 | 91.647 | -23.793 | 1.00 | 31.96 | C |
| ATOM | 5566 | C | ASP | X | 86 | -28.504 | 90.322 | -23.884 | 1.00 | 32.23 | C |
| ATOM | 5567 | O | ASP | X | 86 | -28.925 | 89.318 | -23.303 | 1.00 | 33.09 | O |
| ATOM | 5568 | CB | ASP | X | 86 | -28.907 | 92.357 | -22.482 | 1.00 | 33.01 | C |
| ATOM | 5569 | CG | ASP | X | 86 | -29.599 | 93.708 | -22.367 | 1.00 | 39.16 | C |
| ATOM | 5570 | OD1 | ASP | X | 86 | -29.651 | 94.437 | -23.368 | 1.00 | 40.13 | O |
| ATOM | 5571 | OD2 | ASP | X | 86 | -30.101 | 94.037 | -21.280 | 1.00 | 48.35 | O1- |
| ATOM | 5572 | N | TYR | X | 87 | -27.386 | 90.329 | -24.599 | 1.00 | 31.45 | N |
| ATOM | 5573 | CA | TYR | X | 87 | -26.601 | 89.129 | -24.842 | 1.00 | 28.71 | C |
| ATOM | 5574 | C | TYR | X | 87 | -25.182 | 89.374 | -24.358 | 1.00 | 32.26 | C |
| ATOM | 5575 | O | TYR | X | 87 | -24.609 | 90.435 | -24.628 | 1.00 | 31.02 | O |
| ATOM | 5576 | CB | TYR | X | 87 | -26.621 | 88.762 | -26.326 | 1.00 | 26.11 | C |
| ATOM | 5577 | CG | TYR | X | 87 | -28.002 | 88.352 | -26.816 | 1.00 | 30.12 | C |
| ATOM | 5578 | CD1 | TYR | X | 87 | -28.925 | 89.305 | -27.224 | 1.00 | 29.60 | C |
| ATOM | 5579 | CD2 | TYR | X | 87 | -28.378 | 87.014 | -26.863 | 1.00 | 28.20 | C |
| ATOM | 5580 | CE1 | TYR | X | 87 | -30.188 | 88.932 | -27.678 | 1.00 | 34.26 | C |
| ATOM | 5581 | CE2 | TYR | X | 87 | -29.626 | 86.630 | -27.320 | 1.00 | 32.08 | C |
| ATOM | 5582 | CZ | TYR | X | 87 | -30.527 | 87.596 | -27.726 | 1.00 | 34.03 | C |
| ATOM | 5583 | OH | TYR | X | 87 | -31.777 | 87.223 | -28.150 | 1.00 | 31.35 | O |
| ATOM | 5584 | N | TYR | X | 88 | -24.632 | 88.394 | -23.642 | 1.00 | 29.11 | N |
| ATOM | 5585 | CA | TYR | X | 88 | -23.311 | 88.458 | -23.036 | 1.00 | 28.65 | C |
| ATOM | 5586 | C | TYR | X | 88 | -22.534 | 87.201 | -23.398 | 1.00 | 32.64 | C |
| ATOM | 5587 | O | TYR | X | 88 | -23.054 | 86.084 | -23.241 | 1.00 | 32.58 | O |
| ATOM | 5588 | CB | TYR | X | 88 | -23.408 | 88.547 | -21.508 | 1.00 | 27.95 | C |
| ATOM | 5589 | CG | TYR | X | 88 | -24.118 | 89.757 | -20.959 | 1.00 | 30.42 | C |
| ATOM | 5590 | CD1 | TYR | X | 88 | -25.501 | 89.759 | -20.792 | 1.00 | 28.19 | C |
| ATOM | 5591 | CD2 | TYR | X | 88 | -23.401 | 90.890 | -20.574 | 1.00 | 31.06 | C |
| ATOM | 5592 | CE1 | TYR | X | 88 | -26.162 | 90.867 | -20.275 | 1.00 | 27.13 | C |
| ATOM | 5593 | CE2 | TYR | X | 88 | -24.041 | 92.005 | -20.067 | 1.00 | 32.77 | C |
| ATOM | 5594 | CZ | TYR | X | 88 | -25.424 | 91.989 | -19.920 | 1.00 | 36.54 | C |
| ATOM | 5595 | OH | TYR | X | 88 | -26.055 | 93.090 | -19.405 | 1.00 | 30.95 | O |
| ATOM | 5596 | N | CYS | X | 89 | -21.287 | 87.372 | -23.833 | 1.00 | 28.04 | N |
| ATOM | 5597 | CA | CYS | X | 89 | -20.334 | 86.269 | -23.902 | 1.00 | 28.74 | C |
| ATOM | 5598 | C | CYS | X | 89 | -19.528 | 86.212 | -22.607 | 1.00 | 32.37 | C |
| ATOM | 5599 | O | CYS | X | 89 | -19.378 | 87.212 | -21.902 | 1.00 | 35.41 | O |
| ATOM | 5600 | CB | CYS | X | 89 | -19.378 | 86.436 | -25.082 | 1.00 | 33.37 | C |
| ATOM | 5601 | SG | CYS | X | 89 | -18.463 | 88.060 | -25.073 | 1.00 | 43.75 | S |
| ATOM | 5602 | N | GLY | X | 90 | -19.005 | 85.032 | -22.303 | 1.00 | 32.61 | N |
| ATOM | 5603 | CA | GLY | X | 90 | -18.237 | 84.821 | -21.083 | 1.00 | 31.53 | C |
| ATOM | 5604 | C | GLY | X | 90 | -17.235 | 83.695 | -21.244 | 1.00 | 33.08 | C |

| | | | | | | | | | | | |
|---|---|---|---|---|---|---|---|---|---|---|---|
| ATOM | 5605 | O | GLY | X | 90 | -17.445 | 82.767 | -22.030 | 1.00 | 35.81 | O |
| ATOM | 5606 | N | THR | X | 91 | -16.107 | 83.816 | -20.531 | 1.00 | 25.99 | N |
| ATOM | 5607 | CA | THR | X | 91 | -15.089 | 82.778 | -20.428 | 1.00 | 29.82 | C |
| ATOM | 5608 | C | THR | X | 91 | -14.309 | 82.951 | -19.143 | 1.00 | 31.29 | C |
| ATOM | 5609 | O | THR | X | 91 | -14.533 | 83.878 | -18.365 | 1.00 | 34.63 | O |
| ATOM | 5610 | CB | THR | X | 91 | -13.968 | 82.816 | -21.479 | 1.00 | 38.46 | C |
| ATOM | 5611 | OG1 | THR | X | 91 | -14.245 | 83.717 | -22.547 | 1.00 | 45.88 | O |
| ATOM | 5612 | CG2 | THR | X | 91 | -13.680 | 81.476 | -21.973 | 1.00 | 27.98 | C |
| ATOM | 5613 | N | TRP | X | 92 | -13.306 | 82.097 | -19.016 | 1.00 | 27.25 | N |
| ATOM | 5614 | CA | TRP | X | 92 | -12.219 | 82.230 | -18.077 | 1.00 | 29.16 | C |
| ATOM | 5615 | C | TRP | X | 92 | -11.070 | 83.003 | -18.714 | 1.00 | 32.78 | C |
| ATOM | 5616 | O | TRP | X | 92 | -10.800 | 82.873 | -19.912 | 1.00 | 30.90 | O |
| ATOM | 5617 | CB | TRP | X | 92 | -11.757 | 80.836 | -17.670 | 1.00 | 27.36 | C |
| ATOM | 5618 | CG | TRP | X | 92 | -10.715 | 80.810 | -16.612 | 1.00 | 35.33 | C |
| ATOM | 5619 | CD1 | TRP | X | 92 | -9.392 | 80.455 | -16.757 | 1.00 | 34.95 | C |
| ATOM | 5620 | CD2 | TRP | X | 92 | -10.900 | 81.116 | -15.227 | 1.00 | 34.91 | C |
| ATOM | 5621 | NE1 | TRP | X | 92 | -8.751 | 80.510 | -15.535 | 1.00 | 33.88 | N |
| ATOM | 5622 | CE2 | TRP | X | 92 | -9.647 | 80.927 | -14.584 | 1.00 | 35.68 | C |
| ATOM | 5623 | CE3 | TRP | X | 92 | -11.998 | 81.527 | -14.465 | 1.00 | 29.27 | C |
| ATOM | 5624 | CZ2 | TRP | X | 92 | -9.471 | 81.141 | -13.221 | 1.00 | 32.90 | C |
| ATOM | 5625 | CZ3 | TRP | X | 92 | -11.821 | 81.740 | -13.107 | 1.00 | 30.95 | C |
| ATOM | 5626 | CH2 | TRP | X | 92 | -10.571 | 81.542 | -12.498 | 1.00 | 36.18 | C |
| ATOM | 5627 | N | ASP | X | 93 | -10.389 | 83.802 | -17.915 | 1.00 | 34.71 | N |
| ATOM | 5628 | CA | ASP | X | 93 | -9.143 | 84.431 | -18.340 | 1.00 | 35.49 | C |
| ATOM | 5629 | C | ASP | X | 93 | -8.004 | 83.809 | -17.537 | 1.00 | 35.61 | C |
| ATOM | 5630 | O | ASP | X | 93 | -7.912 | 84.013 | -16.322 | 1.00 | 38.60 | O |
| ATOM | 5631 | CB | ASP | X | 93 | -9.192 | 85.940 | -18.151 | 1.00 | 30.03 | C |
| ATOM | 5632 | CG | ASP | X | 93 | -8.045 | 86.639 | -18.857 | 1.00 | 39.44 | C |
| ATOM | 5633 | OD1 | ASP | X | 93 | -6.878 | 86.282 | -18.573 | 1.00 | 37.16 | O1- |
| ATOM | 5634 | OD2 | ASP | X | 93 | -8.317 | 87.531 | -19.701 | 1.00 | 32.36 | O |
| ATOM | 5635 | N | SER | X | 94 | -7.137 | 83.061 | -18.223 | 1.00 | 33.93 | N |
| ATOM | 5636 | CA | SER | X | 94 | -6.072 | 82.302 | -17.571 | 1.00 | 37.23 | C |
| ATOM | 5637 | C | SER | X | 94 | -5.010 | 83.199 | -16.958 | 1.00 | 43.27 | C |
| ATOM | 5638 | O | SER | X | 94 | -4.321 | 82.779 | -16.024 | 1.00 | 47.17 | O |
| ATOM | 5639 | CB | SER | X | 94 | -5.415 | 81.337 | -18.557 | 1.00 | 35.59 | C |
| ATOM | 5640 | OG | SER | X | 94 | -6.349 | 80.369 | -19.056 | 1.00 | 47.61 | O |
| ATOM | 5641 | N | SER | X | 95 | -4.851 | 84.415 | -17.456 | 1.00 | 36.82 | N |
| ATOM | 5642 | CA | SER | X | 95 | -3.842 | 85.284 | -16.877 | 1.00 | 40.13 | C |
| ATOM | 5643 | C | SER | X | 95 | -4.390 | 86.199 | -15.784 | 1.00 | 43.12 | C |
| ATOM | 5644 | O | SER | X | 95 | -3.652 | 86.546 | -14.858 | 1.00 | 44.13 | O |
| ATOM | 5645 | CB | SER | X | 95 | -3.177 | 86.104 | -17.979 | 1.00 | 38.64 | C |
| ATOM | 5646 | OG | SER | X | 95 | -3.953 | 87.241 | -18.283 | 1.00 | 52.16 | O |
| ATOM | 5647 | N | LEU | X | 96 | -5.653 | 86.623 | -15.858 | 1.00 | 49.30 | N |
| ATOM | 5648 | CA | LEU | X | 96 | -6.260 | 87.359 | -14.750 | 1.00 | 41.53 | C |
| ATOM | 5649 | C | LEU | X | 96 | -6.849 | 86.436 | -13.685 | 1.00 | 36.33 | C |
| ATOM | 5650 | O | LEU | X | 96 | -7.332 | 86.925 | -12.663 | 1.00 | 40.59 | O |
| ATOM | 5651 | CB | LEU | X | 96 | -7.345 | 88.299 | -15.263 | 1.00 | 31.21 | C |
| ATOM | 5652 | CG | LEU | X | 96 | -6.975 | 89.345 | -16.315 | 1.00 | 35.69 | C |
| ATOM | 5653 | CD1 | LEU | X | 96 | -8.257 | 89.972 | -16.878 | 1.00 | 32.73 | C |
| ATOM | 5654 | CD2 | LEU | X | 96 | -6.072 | 90.443 | -15.740 | 1.00 | 33.32 | C |
| ATOM | 5655 | N | ASN | X | 97 | -6.819 | 85.128 | -13.908 | 1.00 | 33.69 | N |
| ATOM | 5656 | CA | ASN | X | 97 | -7.457 | 84.113 | -13.054 | 1.00 | 40.52 | C |
| ATOM | 5657 | C | ASN | X | 97 | -8.862 | 84.515 | -12.585 | 1.00 | 37.02 | C |
| ATOM | 5658 | O | ASN | X | 97 | -9.170 | 84.528 | -11.394 | 1.00 | 38.37 | O |
| ATOM | 5659 | CB | ASN | X | 97 | -6.566 | 83.762 | -11.863 | 1.00 | 40.15 | C |
| ATOM | 5660 | CG | ASN | X | 97 | -5.793 | 82.485 | -12.103 | 1.00 | 56.68 | C |
| ATOM | 5661 | OD1 | ASN | X | 97 | -6.057 | 81.431 | -11.474 | 1.00 | 58.82 | O |
| ATOM | 5662 | ND2 | ASN | X | 97 | -4.866 | 82.541 | -13.069 | 1.00 | 45.20 | N |
| ATOM | 5663 | N | THR | X | 98 | -9.736 | 84.807 | -13.551 | 1.00 | 33.40 | N |
| ATOM | 5664 | CA | THR | X | 98 | -11.086 | 85.225 | -13.198 | 1.00 | 36.45 | C |
| ATOM | 5665 | C | THR | X | 98 | -12.042 | 84.934 | -14.345 | 1.00 | 31.02 | C |
| ATOM | 5666 | O | THR | X | 98 | -11.637 | 84.809 | -15.497 | 1.00 | 29.12 | O |
| ATOM | 5667 | CB | THR | X | 98 | -11.138 | 86.713 | -12.830 | 1.00 | 32.72 | C |
| ATOM | 5668 | OG1 | THR | X | 98 | -12.455 | 87.038 | -12.382 | 1.00 | 37.61 | O |
| ATOM | 5669 | CG2 | THR | X | 98 | -10.840 | 87.569 | -14.031 | 1.00 | 32.00 | C |
| ATOM | 5670 | N | VAL | X | 99 | -13.326 | 84.815 | -14.005 | 1.00 | 30.06 | N |
| ATOM | 5671 | CA | VAL | X | 99 | -14.358 | 84.769 | -15.027 | 1.00 | 24.45 | C |
| ATOM | 5672 | C | VAL | X | 99 | -14.563 | 86.160 | -15.611 | 1.00 | 29.75 | C |
| ATOM | 5673 | O | VAL | X | 99 | -14.620 | 87.174 | -14.897 | 1.00 | 26.93 | O |
| ATOM | 5674 | CB | VAL | X | 99 | -15.680 | 84.233 | -14.460 | 1.00 | 28.00 | C |
| ATOM | 5675 | CG1 | VAL | X | 99 | -16.739 | 84.291 | -15.532 | 1.00 | 26.99 | C |

| | | | | | | | | | | |
|---|---|---|---|---|---|---|---|---|---|---|
| ATOM | 5676 | CG2 | VAL | X | 99 | -15.525 | 82.801 | -13.869 | 1.00 29.57 | C |
| ATOM | 5677 | N | VAL | X | 100 | -14.775 | 86.208 | -16.906 | 1.00 29.21 | N |
| ATOM | 5678 | CA | VAL | X | 100 | -14.748 | 87.464 | -17.623 | 1.00 28.67 | C |
| ATOM | 5679 | C | VAL | X | 100 | -15.948 | 87.512 | -18.574 | 1.00 32.26 | C |
| ATOM | 5680 | O | VAL | X | 100 | -16.225 | 86.546 | -19.296 | 1.00 33.30 | O |
| ATOM | 5681 | CB | VAL | X | 100 | -13.355 | 87.559 | -18.285 | 1.00 34.89 | C |
| ATOM | 5682 | CG1 | VAL | X | 100 | -13.294 | 87.188 | -19.773 | 1.00 28.07 | C |
| ATOM | 5683 | CG2 | VAL | X | 100 | -12.657 | 88.799 | -17.889 | 1.00 30.67 | C |
| ATOM | 5684 | N | PHE | X | 101 | -16.709 | 88.599 | -18.512 | 1.00 29.76 | N |
| ATOM | 5685 | CA | PHE | X | 101 | -17.876 | 88.803 | -19.365 | 1.00 37.29 | C |
| ATOM | 5686 | C | PHE | X | 101 | -17.634 | 89.939 | -20.348 | 1.00 33.64 | C |
| ATOM | 5687 | O | PHE | X | 101 | -16.940 | 90.913 | -20.039 | 1.00 36.04 | O |
| ATOM | 5688 | CB | PHE | X | 101 | -19.138 | 89.186 | -18.567 | 1.00 33.79 | C |
| ATOM | 5689 | CG | PHE | X | 101 | -19.780 | 88.069 | -17.808 | 1.00 32.73 | C |
| ATOM | 5690 | CD1 | PHE | X | 101 | -20.482 | 87.064 | -18.464 | 1.00 38.68 | C |
| ATOM | 5691 | CD2 | PHE | X | 101 | -19.729 | 88.057 | -16.407 | 1.00 31.51 | C |
| ATOM | 5692 | CE1 | PHE | X | 101 | -21.114 | 86.044 | -17.743 | 1.00 34.99 | C |
| ATOM | 5693 | CE2 | PHE | X | 101 | -20.345 | 87.044 | -15.674 | 1.00 28.97 | C |
| ATOM | 5694 | CZ | PHE | X | 101 | -21.040 | 86.028 | -16.349 | 1.00 30.18 | C |
| ATOM | 5695 | N | GLY | X | 102 | -18.283 | 89.846 | -21.507 | 1.00 31.53 | N |
| ATOM | 5696 | CA | GLY | X | 102 | -18.420 | 91.004 | -22.354 | 1.00 30.93 | C |
| ATOM | 5697 | C | GLY | X | 102 | -19.343 | 92.039 | -21.708 | 1.00 38.01 | C |
| ATOM | 5698 | O | GLY | X | 102 | -20.047 | 91.780 | -20.724 | 1.00 32.78 | O |
| ATOM | 5699 | N | GLY | X | 103 | -19.311 | 93.246 | -22.272 | 1.00 29.12 | N |
| ATOM | 5700 | CA | GLY | X | 103 | -20.117 | 94.353 | -21.794 | 1.00 29.37 | C |
| ATOM | 5701 | C | GLY | X | 103 | -21.583 | 94.244 | -22.120 | 1.00 33.08 | C |
| ATOM | 5702 | O | GLY | X | 103 | -22.376 | 95.015 | -21.576 | 1.00 35.44 | O |
| ATOM | 5703 | N | GLY | X | 104 | -21.964 | 93.300 | -22.974 | 1.00 34.27 | N |
| ATOM | 5704 | CA | GLY | X | 104 | -23.358 | 93.149 | -23.326 | 1.00 29.50 | C |
| ATOM | 5705 | C | GLY | X | 104 | -23.731 | 93.836 | -24.619 | 1.00 29.53 | C |
| ATOM | 5706 | O | GLY | X | 104 | -23.197 | 94.891 | -24.951 | 1.00 32.35 | O |
| ATOM | 5707 | N | THR | X | 105 | -24.636 | 93.220 | -25.369 | 1.00 34.88 | N |
| ATOM | 5708 | CA | THR | X | 105 | -25.182 | 93.780 | -26.591 | 1.00 32.20 | C |
| ATOM | 5709 | C | THR | X | 105 | -26.689 | 93.826 | -26.424 | 1.00 34.52 | C |
| ATOM | 5710 | O | THR | X | 105 | -27.326 | 92.787 | -26.221 | 1.00 32.97 | O |
| ATOM | 5711 | CB | THR | X | 105 | -24.791 | 92.943 | -27.812 | 1.00 30.26 | C |
| ATOM | 5712 | OG1 | THR | X | 105 | -23.367 | 92.949 | -27.935 | 1.00 36.62 | O |
| ATOM | 5713 | CG2 | THR | X | 105 | -25.397 | 93.520 | -29.084 | 1.00 27.38 | C |
| ATOM | 5714 | N | LYS | X | 106 | -27.246 | 95.027 | -26.490 | 1.00 33.22 | N |
| ATOM | 5715 | CA | LYS | X | 106 | -28.686 | 95.209 | -26.472 | 1.00 35.13 | C |
| ATOM | 5716 | C | LYS | X | 106 | -29.210 | 94.955 | -27.877 | 1.00 32.47 | C |
| ATOM | 5717 | O | LYS | X | 106 | -28.824 | 95.647 | -28.822 | 1.00 37.14 | O |
| ATOM | 5718 | CB | LYS | X | 106 | -29.035 | 96.619 | -26.001 | 1.00 33.00 | C |
| ATOM | 5719 | CG | LYS | X | 106 | -30.515 | 96.974 | -26.093 | 1.00 40.50 | C |
| ATOM | 5720 | CD | LYS | X | 106 | -30.760 | 98.406 | -25.536 | 1.00 50.00 | C |
| ATOM | 5721 | CE | LYS | X | 106 | -32.202 | 98.919 | -25.776 | 1.00 54.95 | C |
| ATOM | 5722 | NZ | LYS | X | 106 | -33.307 | 98.040 | -25.259 | 1.00 53.87 | N1+ |
| ATOM | 5723 | N | LEU | X | 107 | -30.039 | 93.941 | -28.022 | 1.00 31.69 | N |
| ATOM | 5724 | CA | LEU | X | 107 | -30.702 | 93.641 | -29.282 | 1.00 35.95 | C |
| ATOM | 5725 | C | LEU | X | 107 | -32.109 | 94.195 | -29.217 | 1.00 34.06 | C |
| ATOM | 5726 | O | LEU | X | 107 | -32.890 | 93.810 | -28.335 | 1.00 36.06 | O |
| ATOM | 5727 | CB | LEU | X | 107 | -30.764 | 92.135 | -29.542 | 1.00 29.30 | C |
| ATOM | 5728 | CG | LEU | X | 107 | -31.101 | 91.566 | -30.925 | 1.00 36.45 | C |
| ATOM | 5729 | CD1 | LEU | X | 107 | -31.582 | 90.117 | -30.792 | 1.00 47.08 | C |
| ATOM | 5730 | CD2 | LEU | X | 107 | -32.108 | 92.310 | -31.732 | 1.00 35.75 | C |
| ATOM | 5731 | N | ATHR | X | 108 | -32.447 | 95.068 | -30.150 | 0.50 29.34 | N |
| ATOM | 5732 | CA | ATHR | X | 108 | -33.819 | 95.519 | -30.281 | 0.50 34.47 | C |
| ATOM | 5733 | C | ATHR | X | 108 | -34.372 | 94.993 | -31.598 | 0.50 34.43 | C |
| ATOM | 5734 | O | ATHR | X | 108 | -33.730 | 95.114 | -32.645 | 0.50 33.84 | O |
| ATOM | 5735 | CB | ATHR | X | 108 | -33.929 | 97.050 | -30.167 | 0.50 35.43 | C |
| ATOM | 5736 | OG1ATHR | | X | 108 | -34.721 | 97.575 | -31.244 | 0.50 39.77 | O |
| ATOM | 5737 | CG2ATHR | | X | 108 | -32.553 | 97.713 | -30.114 | 0.50 36.64 | C |
| ATOM | 5738 | N | BTHR | X | 108 | -32.435 | 95.083 | -30.149 | 0.50 28.73 | N |
| ATOM | 5739 | CA | BTHR | X | 108 | -33.795 | 95.559 | -30.334 | 0.50 34.59 | C |
| ATOM | 5740 | C | BTHR | X | 108 | -34.344 | 94.934 | -31.610 | 0.50 34.22 | C |
| ATOM | 5741 | O | BTHR | X | 108 | -33.682 | 94.959 | -32.651 | 0.50 33.77 | O |
| ATOM | 5742 | CB | BTHR | X | 108 | -33.864 | 97.095 | -30.407 | 0.50 35.40 | C |
| ATOM | 5743 | OG1BTHR | | X | 108 | -33.311 | 97.671 | -29.217 | 0.50 29.14 | O |
| ATOM | 5744 | CG2BTHR | | X | 108 | -35.332 | 97.571 | -30.561 | 0.50 36.81 | C |
| ATOM | 5745 | N | VAL | X | 109 | -35.533 | 94.354 | -31.521 | 1.00 34.56 | N |
| ATOM | 5746 | CA | VAL | X | 109 | -36.242 | 93.847 | -32.687 | 1.00 37.76 | C |

| | | | | | | | | | | | |
|---|---|---|---|---|---|---|---|---|---|---|---|
| ATOM | 5747 | C | VAL | X | 109 | -37.211 | 94.947 | -33.124 | 1.00 | 37.56 | | C |
| ATOM | 5748 | O | VAL | X | 109 | -38.195 | 95.233 | -32.435 | 1.00 | 39.51 | | O |
| ATOM | 5749 | CB | VAL | X | 109 | -36.957 | 92.526 | -32.370 | 1.00 | 36.89 | | C |
| ATOM | 5750 | CG1 | VAL | X | 109 | -37.657 | 91.986 | -33.592 | 1.00 | 37.00 | | C |
| ATOM | 5751 | CG2 | VAL | X | 109 | -35.944 | 91.498 | -31.854 | 1.00 | 35.12 | | C |
| ATOM | 5752 | N | LEU | X | 110 | -36.903 | 95.596 | -34.249 | 1.00 | 40.19 | | N |
| ATOM | 5753 | CA | LEU | X | 110 | -37.598 | 96.804 | -34.693 | 1.00 | 37.97 | | C |
| ATOM | 5754 | C | LEU | X | 110 | -39.089 | 96.596 | -34.915 | 1.00 | 42.37 | | C |
| ATOM | 5755 | O | LEU | X | 110 | -39.492 | 95.862 | -35.816 | 1.00 | 42.06 | | O |
| ATOM | 5756 | CB | LEU | X | 110 | -36.952 | 97.330 | -35.974 | 1.00 | 32.89 | | C |
| ATOM | 5757 | CG | LEU | X | 110 | -35.483 | 97.738 | -35.777 | 1.00 | 44.78 | | C |
| ATOM | 5758 | CD1 | LEU | X | 110 | -34.740 | 97.954 | -37.095 | 1.00 | 36.15 | | C |
| ATOM | 5759 | CD2 | LEU | X | 110 | -35.451 | 98.999 | -34.936 | 1.00 | 37.61 | | C |
| ATOM | 5760 | N | SER | X | 111 | -39.923 | 97.222 | -34.090 | 1.00 | 44.41 | | N |
| ATOM | 5761 | CA | SER | X | 111 | -41.366 | 97.119 | -34.268 | 1.00 | 50.21 | | C |
| ATOM | 5762 | C | SER | X | 111 | -42.007 | 98.460 | -34.601 | 1.00 | 49.69 | | C |
| ATOM | 5763 | O | SER | X | 111 | -43.235 | 98.560 | -34.605 | 1.00 | 52.76 | | O |
| ATOM | 5764 | CB | SER | X | 111 | -42.017 | 96.500 | -33.028 | 1.00 | 44.40 | | C |
| ATOM | 5765 | OG | SER | X | 111 | -41.726 | 97.270 | -31.878 | 1.00 | 60.88 | | O |
| ATOM | 5766 | N | GLN | X | 112 | -41.211 | 99.487 | -34.878 | 1.00 | 47.15 | | N |
| ATOM | 5767 | CA | GLN | X | 112 | -41.707 | 100.757 | -35.390 | 1.00 | 48.33 | | C |
| ATOM | 5768 | C | GLN | X | 112 | -40.538 | 101.473 | -36.048 | 1.00 | 46.20 | | C |
| ATOM | 5769 | O | GLN | X | 112 | -39.389 | 101.022 | -35.937 | 1.00 | 50.86 | | O |
| ATOM | 5770 | CB | GLN | X | 112 | -42.328 | 101.613 | -34.268 | 1.00 | 45.44 | | C |
| ATOM | 5771 | CG | GLN | X | 112 | -41.332 | 102.017 | -33.210 | 1.00 | 54.29 | | C |
| ATOM | 5772 | CD | GLN | X | 112 | -41.921 | 102.957 | -32.174 | 1.00 | 55.17 | | C |
| ATOM | 5773 | OE1 | GLN | X | 112 | -42.268 | 102.535 | -31.064 | 1.00 | 50.12 | | O |
| ATOM | 5774 | NE2 | GLN | X | 112 | -42.016 | 104.247 | -32.523 | 1.00 | 51.29 | | N |
| ATOM | 5775 | N | PRO | X | 113 | -40.791 | 102.573 | -36.760 | 1.00 | 47.86 | GZ00 | N |
| ATOM | 5776 | CA | PRO | X | 113 | -39.684 | 103.305 | -37.392 | 1.00 | 44.33 | GZ00 | C |
| ATOM | 5777 | C | PRO | X | 113 | -38.722 | 103.861 | -36.360 | 1.00 | 47.98 | GZ00 | C |
| ATOM | 5778 | O | PRO | X | 113 | -39.099 | 104.165 | -35.229 | 1.00 | 51.31 | GZ00 | O |
| ATOM | 5779 | CB | PRO | X | 113 | -40.386 | 104.439 | -38.148 | 1.00 | 44.96 | GZ00 | C |
| ATOM | 5780 | CG | PRO | X | 113 | -41.754 | 103.943 | -38.377 | 1.00 | 46.92 | GZ00 | C |
| ATOM | 5781 | CD | PRO | X | 113 | -42.098 | 103.123 | -37.162 | 1.00 | 47.64 | GZ00 | C |
| ATOM | 5782 | N | LYS | X | 114 | -37.464 | 104.002 | -36.764 | 1.00 | 48.55 | GZ00 | N |
| ATOM | 5783 | CA | LYS | X | 114 | -36.492 | 104.609 | -35.876 | 1.00 | 42.68 | GZ00 | C |
| ATOM | 5784 | C | LYS | X | 114 | -36.864 | 106.065 | -35.628 | 1.00 | 49.83 | GZ00 | C |
| ATOM | 5785 | O | LYS | X | 114 | -37.429 | 106.739 | -36.491 | 1.00 | 52.51 | GZ00 | O |
| ATOM | 5786 | CB | LYS | X | 114 | -35.082 | 104.485 | -36.457 | 1.00 | 45.63 | GZ00 | C |
| ATOM | 5787 | CG | LYS | X | 114 | -34.596 | 103.034 | -36.553 | 1.00 | 50.09 | GZ00 | C |
| ATOM | 5788 | CD | LYS | X | 114 | -33.151 | 102.950 | -37.039 | 1.00 | 58.16 | GZ00 | C |
| ATOM | 5789 | CE | LYS | X | 114 | -32.468 | 101.608 | -36.704 | 1.00 | 40.47 | GZ00 | C |
| ATOM | 5790 | NZ | LYS | X | 114 | -30.975 | 101.802 | -36.780 | 1.00 | 37.72 | GZ00 | N1+ |
| ATOM | 5791 | N | ALA | X | 115 | -36.559 | 106.540 | -34.424 | 1.00 | 48.82 | GZ00 | N |
| ATOM | 5792 | CA | ALA | X | 115 | -36.951 | 107.875 | -33.987 | 1.00 | 42.61 | GZ00 | C |
| ATOM | 5793 | C | ALA | X | 115 | -35.771 | 108.535 | -33.298 | 1.00 | 43.87 | GZ00 | C |
| ATOM | 5794 | O | ALA | X | 115 | -35.255 | 108.014 | -32.302 | 1.00 | 42.55 | GZ00 | O |
| ATOM | 5795 | CB | ALA | X | 115 | -38.151 | 107.809 | -33.040 | 1.00 | 37.00 | GZ00 | C |
| ATOM | 5796 | N | ALA | X | 116 | -35.349 | 109.676 | -33.825 | 1.00 | 42.05 | GZ00 | N |
| ATOM | 5797 | CA | ALA | X | 116 | -34.281 | 110.428 | -33.189 | 1.00 | 51.37 | GZ00 | C |
| ATOM | 5798 | C | ALA | X | 116 | -34.776 | 111.034 | -31.871 | 1.00 | 46.85 | GZ00 | C |
| ATOM | 5799 | O | ALA | X | 116 | -35.976 | 111.217 | -31.674 | 1.00 | 42.99 | GZ00 | O |
| ATOM | 5800 | CB | ALA | X | 116 | -33.779 | 111.525 | -34.121 | 1.00 | 48.36 | GZ00 | C |
| ATOM | 5801 | N | PRO | X | 117 | -33.881 | 111.279 | -30.922 | 1.00 | 47.49 | GZ00 | N |
| ATOM | 5802 | CA | PRO | X | 117 | -34.320 | 111.846 | -29.642 | 1.00 | 49.41 | GZ00 | C |
| ATOM | 5803 | C | PRO | X | 117 | -34.616 | 113.337 | -29.751 | 1.00 | 53.03 | GZ00 | C |
| ATOM | 5804 | O | PRO | X | 117 | -33.903 | 114.088 | -30.426 | 1.00 | 49.88 | GZ00 | O |
| ATOM | 5805 | CB | PRO | X | 117 | -33.120 | 111.582 | -28.721 | 1.00 | 48.21 | GZ00 | C |
| ATOM | 5806 | CG | PRO | X | 117 | -31.929 | 111.617 | -29.651 | 1.00 | 40.15 | GZ00 | C |
| ATOM | 5807 | CD | PRO | X | 117 | -32.431 | 110.999 | -30.939 | 1.00 | 47.80 | GZ00 | C |
| ATOM | 5808 | N | SER | X | 118 | -35.660 | 113.776 | -29.048 | 1.00 | 43.61 | GZ00 | N |
| ATOM | 5809 | CA | SER | X | 118 | -35.846 | 115.206 | -28.812 | 1.00 | 49.92 | GZ00 | C |
| ATOM | 5810 | C | SER | X | 118 | -35.152 | 115.555 | -27.504 | 1.00 | 43.16 | GZ00 | C |
| ATOM | 5811 | O | SER | X | 118 | -35.266 | 114.824 | -26.515 | 1.00 | 50.92 | GZ00 | O |
| ATOM | 5812 | CB | SER | X | 118 | -37.325 | 115.608 | -28.770 | 1.00 | 47.75 | GZ00 | C |
| ATOM | 5813 | OG | SER | X | 118 | -37.961 | 115.002 | -27.671 | 1.00 | 58.79 | GZ00 | O |
| ATOM | 5814 | N | VAL | X | 119 | -34.377 | 116.627 | -27.528 | 1.00 | 41.52 | GZ00 | N |
| ATOM | 5815 | CA | VAL | X | 119 | -33.512 | 117.022 | -26.426 | 1.00 | 44.89 | GZ00 | C |
| ATOM | 5816 | C | VAL | X | 119 | -33.886 | 118.439 | -26.021 | 1.00 | 52.97 | GZ00 | C |
| ATOM | 5817 | O | VAL | X | 119 | -33.914 | 119.338 | -26.869 | 1.00 | 55.14 | GZ00 | O |

| | | | | | | | | | | | |
|---|---|---|---|---|---|---|---|---|---|---|---|
| ATOM | 5818 | CB | VAL | X | 119 | -32.030 | 116.957 | -26.837 | 1.00 | 42.97 | GZ00 C |
| ATOM | 5819 | CG1 | VAL | X | 119 | -31.122 | 117.319 | -25.668 | 1.00 | 42.13 | GZ00 C |
| ATOM | 5820 | CG2 | VAL | X | 119 | -31.694 | 115.590 | -27.416 | 1.00 | 46.14 | GZ00 C |
| ATOM | 5821 | N | THR | X | 120 | -34.183 | 118.644 | -24.741 | 1.00 | 53.84 | GZ00 N |
| ATOM | 5822 | CA | THR | X | 120 | -34.297 | 119.997 | -24.223 | 1.00 | 52.90 | GZ00 C |
| ATOM | 5823 | C | THR | X | 120 | -33.396 | 120.136 | -23.000 | 1.00 | 49.02 | GZ00 C |
| ATOM | 5824 | O | THR | X | 120 | -33.365 | 119.257 | -22.135 | 1.00 | 44.38 | GZ00 O |
| ATOM | 5825 | CB | THR | X | 120 | -35.779 | 120.402 | -23.935 | 1.00 | 46.21 | GZ00 C |
| ATOM | 5826 | OG1 | THR | X | 120 | -35.982 | 120.611 | -22.536 | 1.00 | 53.27 | GZ00 O |
| ATOM | 5827 | CG2 | THR | X | 120 | -36.767 | 119.365 | -24.468 | 1.00 | 51.58 | GZ00 C |
| ATOM | 5828 | N | LEU | X | 121 | -32.652 | 121.246 | -22.960 | 1.00 | 49.59 | GZ00 N |
| ATOM | 5829 | CA | LEU | X | 121 | -31.580 | 121.506 | -22.006 | 1.00 | 46.69 | GZ00 C |
| ATOM | 5830 | C | LEU | X | 121 | -31.926 | 122.718 | -21.147 | 1.00 | 49.22 | GZ00 C |
| ATOM | 5831 | O | LEU | X | 121 | -32.183 | 123.802 | -21.676 | 1.00 | 57.48 | GZ00 O |
| ATOM | 5832 | CB | LEU | X | 121 | -30.282 | 121.762 | -22.756 | 1.00 | 42.85 | GZ00 C |
| ATOM | 5833 | CG | LEU | X | 121 | -28.925 | 121.456 | -22.159 | 1.00 | 49.09 | GZ00 C |
| ATOM | 5834 | CD1 | LEU | X | 121 | -27.908 | 122.373 | -22.827 | 1.00 | 42.63 | GZ00 C |
| ATOM | 5835 | CD2 | LEU | X | 121 | -28.888 | 121.557 | -20.653 | 1.00 | 50.57 | GZ00 C |
| ATOM | 5836 | N | PHE | X | 122 | -31.952 | 122.534 | -19.834 | 1.00 | 51.49 | GZ00 N |
| ATOM | 5837 | CA | PHE | X | 122 | -32.197 | 123.630 | -18.917 | 1.00 | 48.21 | GZ00 C |
| ATOM | 5838 | C | PHE | X | 122 | -30.914 | 124.009 | -18.215 | 1.00 | 52.42 | GZ00 C |
| ATOM | 5839 | O | PHE | X | 122 | -30.201 | 123.122 | -17.727 | 1.00 | 47.78 | GZ00 O |
| ATOM | 5840 | CB | PHE | X | 122 | -33.245 | 123.263 | -17.872 | 1.00 | 44.54 | GZ00 C |
| ATOM | 5841 | CG | PHE | X | 122 | -34.637 | 123.136 | -18.419 | 1.00 | 55.06 | GZ00 C |
| ATOM | 5842 | CD1 | PHE | X | 122 | -35.407 | 124.269 | -18.664 | 1.00 | 49.10 | GZ00 C |
| ATOM | 5843 | CD2 | PHE | X | 122 | -35.197 | 121.885 | -18.648 | 1.00 | 51.91 | GZ00 C |
| ATOM | 5844 | CE1 | PHE | X | 122 | -36.697 | 124.155 | -19.151 | 1.00 | 52.05 | GZ00 C |
| ATOM | 5845 | CE2 | PHE | X | 122 | -36.487 | 121.766 | -19.133 | 1.00 | 44.48 | GZ00 C |
| ATOM | 5846 | CZ | PHE | X | 122 | -37.238 | 122.899 | -19.385 | 1.00 | 49.83 | GZ00 C |
| ATOM | 5847 | N | PRO | X | 123 | -30.613 | 125.302 | -18.116 | 1.00 | 55.21 | GZ00 N |
| ATOM | 5848 | CA | PRO | X | 123 | -29.470 | 125.760 | -17.316 | 1.00 | 49.75 | GZ00 C |
| ATOM | 5849 | C | PRO | X | 123 | -29.834 | 125.768 | -15.844 | 1.00 | 49.06 | GZ00 C |
| ATOM | 5850 | O | PRO | X | 123 | -31.016 | 125.607 | -15.493 | 1.00 | 43.82 | GZ00 O |
| ATOM | 5851 | CB | PRO | X | 123 | -29.233 | 127.183 | -17.842 | 1.00 | 55.50 | GZ00 C |
| ATOM | 5852 | CG | PRO | X | 123 | -30.612 | 127.648 | -18.178 | 1.00 | 51.07 | GZ00 C |
| ATOM | 5853 | CD | PRO | X | 123 | -31.353 | 126.426 | -18.710 | 1.00 | 47.80 | GZ00 C |
| ATOM | 5854 | N | PRO | X | 124 | -28.866 | 125.964 | -14.950 | 1.00 | 48.22 | GZ00 N |
| ATOM | 5855 | CA | PRO | X | 124 | -29.212 | 126.031 | -13.526 | 1.00 | 50.20 | GZ00 C |
| ATOM | 5856 | C | PRO | X | 124 | -30.040 | 127.272 | -13.245 | 1.00 | 49.48 | GZ00 C |
| ATOM | 5857 | O | PRO | X | 124 | -29.819 | 128.331 | -13.832 | 1.00 | 60.15 | GZ00 O |
| ATOM | 5858 | CB | PRO | X | 124 | -27.850 | 126.075 | -12.822 | 1.00 | 45.39 | GZ00 C |
| ATOM | 5859 | CG | PRO | X | 124 | -26.917 | 126.606 | -13.838 | 1.00 | 51.55 | GZ00 C |
| ATOM | 5860 | CD | PRO | X | 124 | -27.423 | 126.159 | -15.178 | 1.00 | 47.41 | GZ00 C |
| ATOM | 5861 | N | SER | X | 125 | -31.021 | 127.122 | -12.367 | 1.00 | 47.01 | GZ00 N |
| ATOM | 5862 | CA | SER | X | 125 | -31.863 | 128.243 | -11.982 | 1.00 | 52.47 | GZ00 C |
| ATOM | 5863 | C | SER | X | 125 | -31.107 | 129.183 | -11.033 | 1.00 | 58.92 | GZ00 C |
| ATOM | 5864 | O | SER | X | 125 | -30.113 | 128.806 | -10.387 | 1.00 | 46.42 | GZ00 O |
| ATOM | 5865 | CB | SER | X | 125 | -33.156 | 127.749 | -11.322 | 1.00 | 46.03 | GZ00 C |
| ATOM | 5866 | OG | SER | X | 125 | -32.890 | 127.191 | -10.049 | 1.00 | 47.81 | GZ00 O |
| ATOM | 5867 | N | SER | X | 126 | -31.591 | 130.436 | -10.966 | 1.00 | 57.48 | GZ00 N |
| ATOM | 5868 | CA | SER | X | 126 | -30.955 | 131.426 | -10.096 | 1.00 | 51.60 | GZ00 C |
| ATOM | 5869 | C | SER | X | 126 | -31.069 | 131.029 | -8.631 | 1.00 | 45.30 | GZ00 C |
| ATOM | 5870 | O | SER | X | 126 | -30.103 | 131.177 | -7.870 | 1.00 | 56.73 | GZ00 O |
| ATOM | 5871 | CB | SER | X | 126 | -31.552 | 132.818 | -10.327 | 1.00 | 46.73 | GZ00 C |
| ATOM | 5872 | OG | SER | X | 126 | -32.956 | 132.793 | -10.192 | 1.00 | 64.48 | GZ00 O |
| ATOM | 5873 | N | GLU | X | 127 | -32.222 | 130.484 | -8.219 | 1.00 | 49.07 | GZ00 N |
| ATOM | 5874 | CA | GLU | X | 127 | -32.347 | 130.017 | -6.839 | 1.00 | 53.87 | GZ00 C |
| ATOM | 5875 | C | GLU | X | 127 | -31.302 | 128.962 | -6.507 | 1.00 | 55.39 | GZ00 C |
| ATOM | 5876 | O | GLU | X | 127 | -30.761 | 128.948 | -5.394 | 1.00 | 53.30 | GZ00 O |
| ATOM | 5877 | CB | GLU | X | 127 | -33.733 | 129.444 | -6.558 | 1.00 | 46.74 | GZ00 C |
| ATOM | 5878 | CG | GLU | X | 127 | -34.909 | 130.267 | -7.005 | 1.00 | 50.20 | GZ00 C |
| ATOM | 5879 | CD | GLU | X | 127 | -36.221 | 129.697 | -6.458 | 1.00 | 67.37 | GZ00 C |
| ATOM | 5880 | OE1 | GLU | X | 127 | -36.163 | 128.878 | -5.501 | 1.00 | 58.54 | GZ00 O |
| ATOM | 5881 | OE2 | GLU | X | 127 | -37.303 | 130.059 | -6.988 | 1.00 | 72.90 | GZ00 O1- |
| ATOM | 5882 | N | GLU | X | 128 | -30.978 | 128.088 | -7.466 | 1.00 | 52.88 | GZ00 N |
| ATOM | 5883 | CA | GLU | X | 128 | -30.012 | 127.035 | -7.162 | 1.00 | 58.48 | GZ00 C |
| ATOM | 5884 | C | GLU | X | 128 | -28.600 | 127.593 | -7.114 | 1.00 | 54.84 | GZ00 C |
| ATOM | 5885 | O | GLU | X | 128 | -27.778 | 127.141 | -6.305 | 1.00 | 50.17 | GZ00 O |
| ATOM | 5886 | CB | GLU | X | 128 | -30.100 | 125.882 | -8.171 | 1.00 | 51.98 | GZ00 C |
| ATOM | 5887 | CG | GLU | X | 128 | -29.266 | 124.683 | -7.739 | 1.00 | 50.39 | GZ00 C |
| ATOM | 5888 | CD | GLU | X | 128 | -29.022 | 123.641 | -8.841 | 1.00 | 56.66 | GZ00 C |

| | | | | | | | | | | | |
|---|---|---|---|---|---|---|---|---|---|---|---|
| ATOM | 5889 | OE1 | GLU | X | 128 | -28.576 | 122.533 | -8.481 | 1.00 | 51.79 | GZ00 O |
| ATOM | 5890 | OE2 | GLU | X | 128 | -29.257 | 123.912 | -10.044 | 1.00 | 50.04 | GZ00 O1- |
| ATOM | 5891 | N | LEU | X | 129 | -28.300 | 128.569 | -7.971 | 1.00 | 51.67 | GZ00 N |
| ATOM | 5892 | CA | LEU | X | 129 | -27.020 | 129.247 | -7.835 | 1.00 | 57.61 | GZ00 C |
| ATOM | 5893 | C | LEU | X | 129 | -26.899 | 129.856 | -6.440 | 1.00 | 54.69 | GZ00 C |
| ATOM | 5894 | O | LEU | X | 129 | -25.876 | 129.685 | -5.768 | 1.00 | 56.10 | GZ00 O |
| ATOM | 5895 | CB | LEU | X | 129 | -26.861 | 130.296 | -8.938 | 1.00 | 51.14 | GZ00 C |
| ATOM | 5896 | CG | LEU | X | 129 | -26.749 | 129.691 | -10.350 | 1.00 | 59.65 | GZ00 C |
| ATOM | 5897 | CD1 | LEU | X | 129 | -26.772 | 130.745 | -11.454 | 1.00 | 51.42 | GZ00 C |
| ATOM | 5898 | CD2 | LEU | X | 129 | -25.537 | 128.778 | -10.502 | 1.00 | 48.62 | GZ00 C |
| ATOM | 5899 | N | GLN | X | 130 | -27.979 | 130.476 | -5.947 | 1.00 | 51.64 | GZ00 N |
| ATOM | 5900 | CA | GLN | X | 130 | -27.987 | 131.040 | -4.600 | 1.00 | 54.55 | GZ00 C |
| ATOM | 5901 | C | GLN | X | 130 | -27.717 | 130.007 | -3.525 | 1.00 | 57.65 | GZ00 C |
| ATOM | 5902 | O | GLN | X | 130 | -27.308 | 130.384 | -2.425 | 1.00 | 62.14 | GZ00 O |
| ATOM | 5903 | CB | GLN | X | 130 | -29.325 | 131.707 | -4.308 | 1.00 | 49.30 | GZ00 C |
| ATOM | 5904 | CG | GLN | X | 130 | -29.494 | 133.013 | -5.001 | 1.00 | 55.98 | GZ00 C |
| ATOM | 5905 | CD | GLN | X | 130 | -29.020 | 134.140 | -4.135 | 1.00 | 68.65 | GZ00 C |
| ATOM | 5906 | OE1 | GLN | X | 130 | -27.848 | 134.527 | -4.169 | 1.00 | 64.86 | GZ00 O |
| ATOM | 5907 | NE2 | GLN | X | 130 | -29.925 | 134.657 | -3.313 | 1.00 | 63.69 | GZ00 N |
| ATOM | 5908 | N | ALA | X | 131 | -27.980 | 128.727 | -3.788 | 1.00 | 58.30 | GZ00 N |
| ATOM | 5909 | CA | ALA | X | 131 | -27.635 | 127.669 | -2.849 | 1.00 | 53.42 | GZ00 C |
| ATOM | 5910 | C | ALA | X | 131 | -26.223 | 127.155 | -3.057 | 1.00 | 55.16 | GZ00 C |
| ATOM | 5911 | O | ALA | X | 131 | -25.840 | 126.154 | -2.444 | 1.00 | 55.67 | GZ00 O |
| ATOM | 5912 | CB | ALA | X | 131 | -28.629 | 126.517 | -2.951 | 1.00 | 53.39 | GZ00 C |
| ATOM | 5913 | N | ASN | X | 132 | -25.445 | 127.820 | -3.909 | 1.00 | 57.80 | GZ00 N |
| ATOM | 5914 | CA | ASN | X | 132 | -24.062 | 127.430 | -4.188 | 1.00 | 65.38 | GZ00 C |
| ATOM | 5915 | C | ASN | X | 132 | -23.983 | 126.038 | -4.819 | 1.00 | 64.86 | GZ00 C |
| ATOM | 5916 | O | ASN | X | 132 | -23.125 | 125.221 | -4.474 | 1.00 | 58.78 | GZ00 O |
| ATOM | 5917 | CB | ASN | X | 132 | -23.198 | 127.502 | -2.930 | 1.00 | 57.21 | GZ00 C |
| ATOM | 5918 | CG | ASN | X | 132 | -21.742 | 127.673 | -3.259 | 1.00 | 66.70 | GZ00 C |
| ATOM | 5919 | OD1 | ASN | X | 132 | -21.389 | 128.368 | -4.221 | 1.00 | 68.31 | GZ00 O |
| ATOM | 5920 | ND2 | ASN | X | 132 | -20.880 | 127.021 | -2.485 | 1.00 | 71.71 | GZ00 N |
| ATOM | 5921 | N | LYS | X | 133 | -24.891 | 125.766 | -5.750 | 1.00 | 63.21 | GZ00 N |
| ATOM | 5922 | CA | LYS | X | 133 | -24.866 | 124.558 | -6.558 | 1.00 | 55.69 | GZ00 C |
| ATOM | 5923 | C | LYS | X | 133 | -25.304 | 124.950 | -7.959 | 1.00 | 58.70 | GZ00 C |
| ATOM | 5924 | O | LYS | X | 133 | -25.800 | 126.057 | -8.195 | 1.00 | 60.57 | GZ00 O |
| ATOM | 5925 | CB | LYS | X | 133 | -25.778 | 123.468 | -5.978 | 1.00 | 57.74 | GZ00 C |
| ATOM | 5926 | CG | LYS | X | 133 | -25.404 | 122.972 | -4.574 | 1.00 | 60.29 | GZ00 C |
| ATOM | 5927 | CD | LYS | X | 133 | -26.528 | 122.090 | -3.989 | 1.00 | 77.16 | GZ00 C |
| ATOM | 5928 | CE | LYS | X | 133 | -26.295 | 121.712 | -2.516 | 1.00 | 67.68 | GZ00 C |
| ATOM | 5929 | NZ | LYS | X | 133 | -27.578 | 121.494 | -1.757 | 1.00 | 61.53 | GZ00 N1+ |
| ATOM | 5930 | N | ALA | X | 134 | -25.128 | 124.033 | -8.899 | 1.00 | 57.91 | GZ00 N |
| ATOM | 5931 | CA | ALA | X | 134 | -25.550 | 124.292 | -10.273 | 1.00 | 57.46 | GZ00 C |
| ATOM | 5932 | C | ALA | X | 134 | -25.676 | 122.951 | -10.970 | 1.00 | 58.56 | GZ00 C |
| ATOM | 5933 | O | ALA | X | 134 | -24.715 | 122.173 | -10.980 | 1.00 | 56.94 | GZ00 O |
| ATOM | 5934 | CB | ALA | X | 134 | -24.546 | 125.194 | -10.994 | 1.00 | 46.64 | GZ00 C |
| ATOM | 5935 | N | THR | X | 135 | -26.850 | 122.650 | -11.519 | 1.00 | 55.26 | GZ00 N |
| ATOM | 5936 | CA | THR | X | 135 | -26.986 | 121.434 | -12.308 | 1.00 | 47.36 | GZ00 C |
| ATOM | 5937 | C | THR | X | 135 | -27.631 | 121.778 | -13.640 | 1.00 | 45.07 | GZ00 C |
| ATOM | 5938 | O | THR | X | 135 | -28.602 | 122.540 | -13.693 | 1.00 | 43.66 | GZ00 O |
| ATOM | 5939 | CB | THR | X | 135 | -27.779 | 120.335 | -11.579 | 1.00 | 46.59 | GZ00 C |
| ATOM | 5940 | OG1 | THR | X | 135 | -29.174 | 120.523 | -11.787 | 1.00 | 60.99 | GZ00 O |
| ATOM | 5941 | CG2 | THR | X | 135 | -27.487 | 120.331 | -10.088 | 1.00 | 40.51 | GZ00 C |
| ATOM | 5942 | N | LEU | X | 136 | -27.050 | 121.251 | -14.711 | 1.00 | 43.91 | GZ00 N |
| ATOM | 5943 | CA | LEU | X | 136 | -27.635 | 121.315 | -16.036 | 1.00 | 42.15 | GZ00 C |
| ATOM | 5944 | C | LEU | X | 136 | -28.501 | 120.080 | -16.231 | 1.00 | 50.30 | GZ00 C |
| ATOM | 5945 | O | LEU | X | 136 | -28.140 | 118.976 | -15.812 | 1.00 | 46.46 | GZ00 O |
| ATOM | 5946 | CB | LEU | X | 136 | -26.544 | 121.383 | -17.101 | 1.00 | 46.62 | GZ00 C |
| ATOM | 5947 | CG | LEU | X | 136 | -25.574 | 122.574 | -17.036 | 1.00 | 48.02 | GZ00 C |
| ATOM | 5948 | CD1 | LEU | X | 136 | -24.445 | 122.431 | -18.044 | 1.00 | 53.43 | GZ00 C |
| ATOM | 5949 | CD2 | LEU | X | 136 | -26.330 | 123.797 | -17.372 | 1.00 | 47.48 | GZ00 C |
| ATOM | 5950 | N | VAL | X | 137 | -29.654 | 120.270 | -16.849 | 1.00 | 48.09 | GZ00 N |
| ATOM | 5951 | CA | VAL | X | 137 | -30.643 | 119.214 | -16.962 | 1.00 | 49.70 | GZ00 C |
| ATOM | 5952 | C | VAL | X | 137 | -30.865 | 118.972 | -18.443 | 1.00 | 47.78 | GZ00 C |
| ATOM | 5953 | O | VAL | X | 137 | -31.364 | 119.852 | -19.156 | 1.00 | 46.56 | GZ00 O |
| ATOM | 5954 | CB | VAL | X | 137 | -31.951 | 119.574 | -16.249 | 1.00 | 41.53 | GZ00 C |
| ATOM | 5955 | CG1 | VAL | X | 137 | -32.927 | 118.433 | -16.354 | 1.00 | 38.78 | GZ00 C |
| ATOM | 5956 | CG2 | VAL | X | 137 | -31.677 | 119.926 | -14.782 | 1.00 | 37.08 | GZ00 C |
| ATOM | 5957 | N | CYS | X | 138 | -30.480 | 117.787 | -18.905 | 1.00 | 41.44 | GZ00 N |
| ATOM | 5958 | CA | CYS | X | 138 | -30.653 | 117.371 | -20.292 | 1.00 | 44.75 | GZ00 C |
| ATOM | 5959 | C | CYS | X | 138 | -31.717 | 116.286 | -20.316 | 1.00 | 42.51 | GZ00 C |

| | | | | | | | | | | | | |
|---|---|---|---|---|---|---|---|---|---|---|---|---|
| ATOM | 5960 | O | CYS | X | 138 | -31.459 | 115.150 | -19.904 | 1.00 | 45.75 | GZ00 | O |
| ATOM | 5961 | CB | CYS | X | 138 | -29.341 | 116.850 | -20.872 | 1.00 | 41.85 | GZ00 | C |
| ATOM | 5962 | SG | CYS | X | 138 | -29.335 | 116.711 | -22.686 | 1.00 | 48.77 | GZ00 | S |
| ATOM | 5963 | N | LEU | X | 139 | -32.904 | 116.627 | -20.803 | 1.00 | 40.25 | GZ00 | N |
| ATOM | 5964 | CA | LEU | X | 139 | -33.998 | 115.670 | -20.918 | 1.00 | 44.13 | GZ00 | C |
| ATOM | 5965 | C | LEU | X | 139 | -34.091 | 115.164 | -22.348 | 1.00 | 48.33 | GZ00 | C |
| ATOM | 5966 | O | LEU | X | 139 | -34.096 | 115.956 | -23.296 | 1.00 | 49.49 | GZ00 | O |
| ATOM | 5967 | CB | LEU | X | 139 | -35.323 | 116.286 | -20.469 | 1.00 | 40.97 | GZ00 | C |
| ATOM | 5968 | CG | LEU | X | 139 | -35.163 | 116.830 | -19.046 | 1.00 | 45.88 | GZ00 | C |
| ATOM | 5969 | CD1 | LEU | X | 139 | -34.991 | 118.304 | -19.121 | 1.00 | 55.62 | GZ00 | C |
| ATOM | 5970 | CD2 | LEU | X | 139 | -36.336 | 116.513 | -18.183 | 1.00 | 56.00 | GZ00 | C |
| ATOM | 5971 | N | ILE | X | 140 | -34.185 | 113.843 | -22.489 | 1.00 | 43.98 | GZ00 | N |
| ATOM | 5972 | CA | ILE | X | 140 | -34.065 | 113.151 | -23.763 | 1.00 | 39.00 | GZ00 | C |
| ATOM | 5973 | C | ILE | X | 140 | -35.287 | 112.261 | -23.897 | 1.00 | 41.08 | GZ00 | C |
| ATOM | 5974 | O | ILE | X | 140 | -35.562 | 111.453 | -23.003 | 1.00 | 45.21 | GZ00 | O |
| ATOM | 5975 | CB | ILE | X | 140 | -32.776 | 112.314 | -23.820 | 1.00 | 38.23 | GZ00 | C |
| ATOM | 5976 | CG1 | ILE | X | 140 | -31.593 | 113.141 | -23.326 | 1.00 | 38.68 | GZ00 | C |
| ATOM | 5977 | CG2 | ILE | X | 140 | -32.517 | 111.813 | -25.212 | 1.00 | 37.81 | GZ00 | C |
| ATOM | 5978 | CD1 | ILE | X | 140 | -30.465 | 112.309 | -22.831 | 1.00 | 36.60 | GZ00 | C |
| ATOM | 5979 | N | SER | X | 141 | -36.037 | 112.410 | -24.986 | 1.00 | 38.32 | GZ00 | N |
| ATOM | 5980 | CA | SER | X | 141 | -37.296 | 111.688 | -25.062 | 1.00 | 41.71 | GZ00 | C |
| ATOM | 5981 | C | SER | X | 141 | -37.616 | 111.305 | -26.496 | 1.00 | 42.47 | GZ00 | C |
| ATOM | 5982 | O | SER | X | 141 | -37.050 | 111.841 | -27.451 | 1.00 | 41.57 | GZ00 | O |
| ATOM | 5983 | CB | SER | X | 141 | -38.442 | 112.523 | -24.495 | 1.00 | 41.65 | GZ00 | C |
| ATOM | 5984 | OG | SER | X | 141 | -38.508 | 113.735 | -25.214 | 1.00 | 44.00 | GZ00 | O |
| ATOM | 5985 | N | ASP | X | 142 | -38.557 | 110.367 | -26.620 | 1.00 | 46.32 | GZ00 | N |
| ATOM | 5986 | CA | ASP | X | 142 | -39.150 | 109.987 | -27.901 | 1.00 | 48.85 | GZ00 | C |
| ATOM | 5987 | C | ASP | X | 142 | -38.129 | 109.382 | -28.860 | 1.00 | 51.47 | GZ00 | C |
| ATOM | 5988 | O | ASP | X | 142 | -38.175 | 109.623 | -30.072 | 1.00 | 44.48 | GZ00 | O |
| ATOM | 5989 | CB | ASP | X | 142 | -39.846 | 111.179 | -28.546 | 1.00 | 47.17 | GZ00 | C |
| ATOM | 5990 | CG | ASP | X | 142 | -41.165 | 111.502 | -27.888 | 1.00 | 60.06 | GZ00 | C |
| ATOM | 5991 | OD1 | ASP | X | 142 | -41.856 | 110.559 | -27.429 | 1.00 | 53.08 | GZ00 | O |
| ATOM | 5992 | OD2 | ASP | X | 142 | -41.509 | 112.706 | -27.833 | 1.00 | 72.08 | GZ00 | O1- |
| ATOM | 5993 | N | PHE | X | 143 | -37.183 | 108.607 | -28.329 | 1.00 | 42.34 | GZ00 | N |
| ATOM | 5994 | CA | PHE | X | 143 | -36.209 | 107.975 | -29.202 | 1.00 | 50.27 | GZ00 | C |
| ATOM | 5995 | C | PHE | X | 143 | -36.431 | 106.471 | -29.275 | 1.00 | 46.86 | GZ00 | C |
| ATOM | 5996 | O | PHE | X | 143 | -36.969 | 105.852 | -28.352 | 1.00 | 43.04 | GZ00 | O |
| ATOM | 5997 | CB | PHE | X | 143 | -34.773 | 108.296 | -28.793 | 1.00 | 38.71 | GZ00 | C |
| ATOM | 5998 | CG | PHE | X | 143 | -34.426 | 107.944 | -27.388 | 1.00 | 40.75 | GZ00 | C |
| ATOM | 5999 | CD1 | PHE | X | 143 | -33.898 | 106.698 | -27.084 | 1.00 | 36.49 | GZ00 | C |
| ATOM | 6000 | CD2 | PHE | X | 143 | -34.537 | 108.889 | -26.377 | 1.00 | 38.84 | GZ00 | C |
| ATOM | 6001 | CE1 | PHE | X | 143 | -33.510 | 106.389 | -25.788 | 1.00 | 41.99 | GZ00 | C |
| ATOM | 6002 | CE2 | PHE | X | 143 | -34.161 | 108.588 | -25.078 | 1.00 | 39.59 | GZ00 | C |
| ATOM | 6003 | CZ | PHE | X | 143 | -33.645 | 107.330 | -24.777 | 1.00 | 38.14 | GZ00 | C |
| ATOM | 6004 | N | TYR | X | 144 | -36.071 | 105.909 | -30.422 | 1.00 | 41.60 | GZ00 | N |
| ATOM | 6005 | CA | TYR | X | 144 | -36.247 | 104.485 | -30.675 | 1.00 | 40.98 | GZ00 | C |
| ATOM | 6006 | C | TYR | X | 144 | -35.290 | 104.053 | -31.769 | 1.00 | 46.46 | GZ00 | C |
| ATOM | 6007 | O | TYR | X | 144 | -35.237 | 104.680 | -32.843 | 1.00 | 42.13 | GZ00 | O |
| ATOM | 6008 | CB | TYR | X | 144 | -37.685 | 104.146 | -31.067 | 1.00 | 43.87 | GZ00 | C |
| ATOM | 6009 | CG | TYR | X | 144 | -37.912 | 102.658 | -31.265 | 1.00 | 41.93 | GZ00 | C |
| ATOM | 6010 | CD1 | TYR | X | 144 | -38.183 | 101.821 | -30.184 | 1.00 | 44.51 | GZ00 | C |
| ATOM | 6011 | CD2 | TYR | X | 144 | -37.838 | 102.090 | -32.527 | 1.00 | 35.71 | GZ00 | C |
| ATOM | 6012 | CE1 | TYR | X | 144 | -38.379 | 100.445 | -30.358 | 1.00 | 40.63 | GZ00 | C |
| ATOM | 6013 | CE2 | TYR | X | 144 | -38.050 | 100.733 | -32.717 | 1.00 | 40.34 | GZ00 | C |
| ATOM | 6014 | CZ | TYR | X | 144 | -38.309 | 99.912 | -31.631 | 1.00 | 41.12 | GZ00 | C |
| ATOM | 6015 | OH | TYR | X | 144 | -38.511 | 98.567 | -31.831 | 1.00 | 40.40 | GZ00 | O |
| ATOM | 6016 | N | PRO | X | 145 | -34.530 | 102.974 | -31.511 | 1.00 | 40.55 | GZ00 | N |
| ATOM | 6017 | CA | PRO | X | 145 | -34.563 | 102.164 | -30.284 | 1.00 | 43.72 | GZ00 | C |
| ATOM | 6018 | C | PRO | X | 145 | -33.998 | 102.849 | -29.028 | 1.00 | 43.96 | GZ00 | C |
| ATOM | 6019 | O | PRO | X | 145 | -33.489 | 103.965 | -29.115 | 1.00 | 40.33 | GZ00 | O |
| ATOM | 6020 | CB | PRO | X | 145 | -33.699 | 100.937 | -30.644 | 1.00 | 43.12 | GZ00 | C |
| ATOM | 6021 | CG | PRO | X | 145 | -32.956 | 101.292 | -31.849 | 1.00 | 42.92 | GZ00 | C |
| ATOM | 6022 | CD | PRO | X | 145 | -33.714 | 102.356 | -32.570 | 1.00 | 40.02 | GZ00 | C |
| ATOM | 6023 | N | GLY | X | 146 | -34.102 | 102.165 | -27.884 | 1.00 | 38.67 | GZ00 | N |
| ATOM | 6024 | CA | GLY | X | 146 | -33.811 | 102.737 | -26.584 | 1.00 | 36.90 | GZ00 | C |
| ATOM | 6025 | C | GLY | X | 146 | -32.366 | 102.660 | -26.134 | 1.00 | 38.27 | GZ00 | C |
| ATOM | 6026 | O | GLY | X | 146 | -32.068 | 102.118 | -25.070 | 1.00 | 42.45 | GZ00 | O |
| ATOM | 6027 | N | ALA | X | 147 | -31.462 | 103.201 | -26.930 | 1.00 | 33.88 | GZ00 | N |
| ATOM | 6028 | CA | ALA | X | 147 | -30.063 | 103.278 | -26.560 | 1.00 | 36.10 | GZ00 | C |
| ATOM | 6029 | C | ALA | X | 147 | -29.541 | 104.597 | -27.083 | 1.00 | 33.68 | GZ00 | C |
| ATOM | 6030 | O | ALA | X | 147 | -29.858 | 105.007 | -28.203 | 1.00 | 39.90 | GZ00 | O |

| | | | | | | | | | | | | |
|---|---|---|---|---|---|---|---|---|---|---|---|---|
| ATOM | 6031 | CB | ALA | X | 147 | -29.225 | 102.121 | -27.118 | 1.00 | 29.64 | GZ00 | C |
| ATOM | 6032 | N | VAL | X | 148 | -28.712 | 105.231 | -26.263 | 1.00 | 33.29 | GZ00 | N |
| ATOM | 6033 | CA | VAL | X | 148 | -28.309 | 106.608 | -26.467 | 1.00 | 34.38 | GZ00 | C |
| ATOM | 6034 | C | VAL | X | 148 | -27.005 | 106.806 | -25.702 | 1.00 | 44.52 | GZ00 | C |
| ATOM | 6035 | O | VAL | X | 148 | -26.787 | 106.191 | -24.652 | 1.00 | 39.89 | GZ00 | O |
| ATOM | 6036 | CB | VAL | X | 148 | -29.464 | 107.518 | -25.970 | 1.00 | 40.00 | GZ00 | C |
| ATOM | 6037 | CG1 | VAL | X | 148 | -28.993 | 108.560 | -25.020 | 1.00 | 41.53 | GZ00 | C |
| ATOM | 6038 | CG2 | VAL | X | 148 | -30.271 | 108.073 | -27.138 | 1.00 | 35.87 | GZ00 | C |
| ATOM | 6039 | N | THR | X | 149 | -26.133 | 107.663 | -26.213 | 1.00 | 36.77 | GZ00 | N |
| ATOM | 6040 | CA | THR | X | 149 | -24.987 | 108.051 | -25.409 | 1.00 | 38.57 | GZ00 | C |
| ATOM | 6041 | C | THR | X | 149 | -24.960 | 109.563 | -25.294 | 1.00 | 44.30 | GZ00 | C |
| ATOM | 6042 | O | THR | X | 149 | -25.353 | 110.287 | -26.217 | 1.00 | 42.08 | GZ00 | O |
| ATOM | 6043 | CB | THR | X | 149 | -23.650 | 107.565 | -25.975 | 1.00 | 48.56 | GZ00 | C |
| ATOM | 6044 | OG1 | THR | X | 149 | -23.455 | 108.130 | -27.276 | 1.00 | 56.60 | GZ00 | O |
| ATOM | 6045 | CG2 | THR | X | 149 | -23.624 | 106.035 | -26.054 | 1.00 | 37.17 | GZ00 | C |
| ATOM | 6046 | N | VAL | X | 150 | -24.503 | 110.036 | -24.146 | 1.00 | 43.54 | GZ00 | N |
| ATOM | 6047 | CA | VAL | X | 150 | -24.564 | 111.452 | -23.836 | 1.00 | 47.15 | GZ00 | C |
| ATOM | 6048 | C | VAL | X | 150 | -23.160 | 111.946 | -23.536 | 1.00 | 46.29 | GZ00 | C |
| ATOM | 6049 | O | VAL | X | 150 | -22.484 | 111.422 | -22.642 | 1.00 | 44.10 | GZ00 | O |
| ATOM | 6050 | CB | VAL | X | 150 | -25.519 | 111.726 | -22.663 | 1.00 | 42.61 | GZ00 | C |
| ATOM | 6051 | CG1 | VAL | X | 150 | -25.584 | 113.212 | -22.388 | 1.00 | 45.14 | GZ00 | C |
| ATOM | 6052 | CG2 | VAL | X | 150 | -26.919 | 111.152 | -22.975 | 1.00 | 37.35 | GZ00 | C |
| ATOM | 6053 | N | ALA | X | 151 | -22.741 | 112.977 | -24.260 | 1.00 | 43.99 | GZ00 | N |
| ATOM | 6054 | CA | ALA | X | 151 | -21.484 | 113.665 | -24.002 | 1.00 | 55.42 | GZ00 | C |
| ATOM | 6055 | C | ALA | X | 151 | -21.769 | 115.133 | -23.706 | 1.00 | 56.11 | GZ00 | C |
| ATOM | 6056 | O | ALA | X | 151 | -22.614 | 115.758 | -24.361 | 1.00 | 51.73 | GZ00 | O |
| ATOM | 6057 | CB | ALA | X | 151 | -20.528 | 113.545 | -25.190 | 1.00 | 47.91 | GZ00 | C |
| ATOM | 6058 | N | TRP | X | 152 | -21.091 | 115.665 | -22.693 | 1.00 | 48.76 | GZ00 | N |
| ATOM | 6059 | CA | TRP | X | 152 | -21.207 | 117.064 | -22.312 | 1.00 | 52.98 | GZ00 | C |
| ATOM | 6060 | C | TRP | X | 152 | -19.957 | 117.821 | -22.742 | 1.00 | 62.60 | GZ00 | C |
| ATOM | 6061 | O | TRP | X | 152 | -18.840 | 117.307 | -22.638 | 1.00 | 63.13 | GZ00 | O |
| ATOM | 6062 | CB | TRP | X | 152 | -21.402 | 117.202 | -20.798 | 1.00 | 54.47 | GZ00 | C |
| ATOM | 6063 | CG | TRP | X | 152 | -22.732 | 116.706 | -20.310 | 1.00 | 55.93 | GZ00 | C |
| ATOM | 6064 | CD1 | TRP | X | 152 | -23.070 | 115.415 | -20.021 | 1.00 | 50.45 | GZ00 | C |
| ATOM | 6065 | CD2 | TRP | X | 152 | -23.908 | 117.493 | -20.059 | 1.00 | 54.33 | GZ00 | C |
| ATOM | 6066 | NE1 | TRP | X | 152 | -24.380 | 115.350 | -19.603 | 1.00 | 45.37 | GZ00 | N |
| ATOM | 6067 | CE2 | TRP | X | 152 | -24.919 | 116.608 | -19.624 | 1.00 | 49.73 | GZ00 | C |
| ATOM | 6068 | CE3 | TRP | X | 152 | -24.205 | 118.856 | -20.162 | 1.00 | 49.64 | GZ00 | C |
| ATOM | 6069 | CZ2 | TRP | X | 152 | -26.201 | 117.046 | -19.286 | 1.00 | 49.17 | GZ00 | C |
| ATOM | 6070 | CZ3 | TRP | X | 152 | -25.488 | 119.290 | -19.834 | 1.00 | 47.92 | GZ00 | C |
| ATOM | 6071 | CH2 | TRP | X | 152 | -26.464 | 118.390 | -19.400 | 1.00 | 49.61 | GZ00 | C |
| ATOM | 6072 | N | LYS | X | 153 | -20.141 | 119.053 | -23.204 | 1.00 | 65.10 | GZ00 | N |
| ATOM | 6073 | CA | LYS | X | 153 | -19.019 | 119.883 | -23.602 | 1.00 | 62.17 | GZ00 | C |
| ATOM | 6074 | C | LYS | X | 153 | -19.122 | 121.266 | -22.971 | 1.00 | 70.98 | GZ00 | C |
| ATOM | 6075 | O | LYS | X | 153 | -20.213 | 121.849 | -22.883 | 1.00 | 60.73 | GZ00 | O |
| ATOM | 6076 | CB | LYS | X | 153 | -18.918 | 119.968 | -25.133 | 1.00 | 62.97 | GZ00 | C |
| ATOM | 6077 | CG | LYS | X | 153 | -18.343 | 118.679 | -25.728 | 1.00 | 71.97 | GZ00 | C |
| ATOM | 6078 | CD | LYS | X | 153 | -18.093 | 118.773 | -27.212 | 1.00 | 76.62 | GZ00 | C |
| ATOM | 6079 | CE | LYS | X | 153 | -17.488 | 117.483 | -27.757 | 1.00 | 78.74 | GZ00 | C |
| ATOM | 6080 | NZ | LYS | X | 153 | -17.470 | 117.510 | -29.257 | 1.00 | 82.65 | GZ00 | N1+ |
| ATOM | 6081 | N | ALA | X | 154 | -17.966 | 121.767 | -22.517 | 1.00 | 68.61 | GZ00 | N |
| ATOM | 6082 | CA | ALA | X | 154 | -17.783 | 123.138 | -22.053 | 1.00 | 70.61 | GZ00 | C |
| ATOM | 6083 | C | ALA | X | 154 | -16.988 | 123.883 | -23.122 | 1.00 | 76.84 | GZ00 | C |
| ATOM | 6084 | O | ALA | X | 154 | -15.785 | 123.645 | -23.282 | 1.00 | 70.93 | GZ00 | O |
| ATOM | 6085 | CB | ALA | X | 154 | -17.057 | 123.158 | -20.709 | 1.00 | 58.77 | GZ00 | C |
| ATOM | 6086 | N | ASP | X | 155 | -17.672 | 124.765 | -23.853 | 1.00 | 75.44 | GZ00 | N |
| ATOM | 6087 | CA | ASP | X | 155 | -17.137 | 125.491 | -25.013 | 1.00 | 86.60 | GZ00 | C |
| ATOM | 6088 | C | ASP | X | 155 | -16.167 | 124.618 | -25.821 | 1.00 | 88.28 | GZ00 | C |
| ATOM | 6089 | O | ASP | X | 155 | -14.980 | 124.919 | -25.980 | 1.00 | 92.56 | GZ00 | O |
| ATOM | 6090 | CB | ASP | X | 155 | -16.529 | 126.864 | -24.643 | 1.00 | 93.75 | GZ00 | C |
| ATOM | 6091 | CG | ASP | X | 155 | -15.592 | 126.838 | -23.429 | 1.00 | 92.88 | GZ00 | C |
| ATOM | 6092 | OD2 | ASP | X | 155 | -15.842 | 127.652 | -22.504 | 1.00 | 84.61 | GZ00 | O1- |
| ATOM | 6093 | OD1 | ASP | X | 155 | -14.599 | 126.064 | -23.414 | 1.00 | 88.83 | GZ00 | O |
| ATOM | 6094 | N | SER | X | 156 | -16.707 | 123.493 | -26.297 | 1.00 | 79.32 | GZ00 | N |
| ATOM | 6095 | CA | SER | X | 156 | -16.051 | 122.520 | -27.174 | 1.00 | 78.60 | GZ00 | C |
| ATOM | 6096 | C | SER | X | 156 | -14.971 | 121.688 | -26.498 | 1.00 | 74.64 | GZ00 | C |
| ATOM | 6097 | O | SER | X | 156 | -14.249 | 120.963 | -27.189 | 1.00 | 84.36 | GZ00 | O |
| ATOM | 6098 | CB | SER | X | 156 | -15.436 | 123.186 | -28.416 | 1.00 | 73.87 | GZ00 | C |
| ATOM | 6099 | OG | SER | X | 156 | -16.405 | 123.895 | -29.165 | 1.00 | 81.66 | GZ00 | O |
| ATOM | 6100 | N | SER | X | 157 | -14.854 | 121.728 | -25.183 | 1.00 | 69.01 | GZ00 | N |
| ATOM | 6101 | CA | SER | X | 157 | -13.979 | 120.769 | -24.541 | 1.00 | 69.77 | GZ00 | C |

| | | | | | | | | | | | | |
|---|---|---|---|---|---|---|---|---|---|---|---|---|
| ATOM | 6102 | C | SER | X | 157 | -14.813 | 119.693 | -23.873 | 1.00 | 80.18 | GZ00 | C |
| ATOM | 6103 | O | SER | X | 157 | -15.795 | 120.013 | -23.189 | 1.00 | 78.55 | GZ00 | O |
| ATOM | 6104 | CB | SER | X | 157 | -13.084 | 121.447 | -23.505 | 1.00 | 71.70 | GZ00 | C |
| ATOM | 6105 | OG | SER | X | 157 | -12.182 | 122.329 | -24.136 | 1.00 | 83.37 | GZ00 | O |
| ATOM | 6106 | N | PRO | X | 158 | -14.476 | 118.419 | -24.062 | 1.00 | 82.78 | GZ00 | N |
| ATOM | 6107 | CA | PRO | X | 158 | -15.228 | 117.349 | -23.395 | 1.00 | 76.46 | GZ00 | C |
| ATOM | 6108 | C | PRO | X | 158 | -15.217 | 117.510 | -21.881 | 1.00 | 71.70 | GZ00 | C |
| ATOM | 6109 | O | PRO | X | 158 | -14.185 | 117.812 | -21.277 | 1.00 | 72.61 | GZ00 | O |
| ATOM | 6110 | CB | PRO | X | 158 | -14.501 | 116.072 | -23.839 | 1.00 | 73.01 | GZ00 | C |
| ATOM | 6111 | CG | PRO | X | 158 | -13.220 | 116.534 | -24.487 | 1.00 | 79.70 | GZ00 | C |
| ATOM | 6112 | CD | PRO | X | 158 | -13.513 | 117.891 | -25.037 | 1.00 | 80.30 | GZ00 | C |
| ATOM | 6113 | N | VAL | X | 159 | -16.375 | 117.266 | -21.266 | 1.00 | 66.35 | GZ00 | N |
| ATOM | 6114 | CA | VAL | X | 159 | -16.548 | 117.386 | -19.823 | 1.00 | 62.00 | GZ00 | C |
| ATOM | 6115 | C | VAL | X | 159 | -16.401 | 116.006 | -19.204 | 1.00 | 69.04 | GZ00 | C |
| ATOM | 6116 | O | VAL | X | 159 | -17.189 | 115.099 | -19.491 | 1.00 | 77.71 | GZ00 | O |
| ATOM | 6117 | CB | VAL | X | 159 | -17.910 | 117.997 | -19.467 | 1.00 | 64.97 | GZ00 | C |
| ATOM | 6118 | CG1 | VAL | X | 159 | -18.084 | 118.013 | -17.959 | 1.00 | 62.71 | GZ00 | C |
| ATOM | 6119 | CG2 | VAL | X | 159 | -18.059 | 119.400 | -20.075 | 1.00 | 59.52 | GZ00 | C |
| ATOM | 6120 | N | LYS | X | 160 | -15.413 | 115.857 | -18.328 | 1.00 | 74.85 | GZ00 | N |
| ATOM | 6121 | CA | LYS | X | 160 | -14.967 | 114.550 | -17.867 | 1.00 | 79.58 | GZ00 | C |
| ATOM | 6122 | C | LYS | X | 160 | -15.580 | 114.124 | -16.535 | 1.00 | 75.16 | GZ00 | C |
| ATOM | 6123 | O | LYS | X | 160 | -15.412 | 112.964 | -16.143 | 1.00 | 82.11 | GZ00 | O |
| ATOM | 6124 | CB | LYS | X | 160 | -13.429 | 114.543 | -17.758 | 1.00 | 85.50 | GZ00 | C |
| ATOM | 6125 | CG | LYS | X | 160 | -12.723 | 113.330 | -18.377 | 1.00 | 89.99 | GZ00 | C |
| ATOM | 6126 | CD | LYS | X | 160 | -12.558 | 113.438 | -19.900 | 1.00 | 91.72 | GZ00 | C |
| ATOM | 6127 | CE | LYS | X | 160 | -11.807 | 112.217 | -20.463 | 1.00101.89 | | GZ00 | C |
| ATOM | 6128 | NZ | LYS | X | 160 | -11.786 | 112.147 | -21.960 | 1.00 | 92.33 | GZ00 | N1+ |
| ATOM | 6129 | N | ALA | X | 161 | -16.284 | 115.011 | -15.833 | 1.00 | 64.13 | GZ00 | N |
| ATOM | 6130 | CA | ALA | X | 161 | -16.778 | 114.677 | -14.503 | 1.00 | 64.11 | GZ00 | C |
| ATOM | 6131 | C | ALA | X | 161 | -18.053 | 115.454 | -14.206 | 1.00 | 60.81 | GZ00 | C |
| ATOM | 6132 | O | ALA | X | 161 | -18.346 | 116.472 | -14.836 | 1.00 | 59.19 | GZ00 | O |
| ATOM | 6133 | CB | ALA | X | 161 | -15.728 | 114.953 | -13.424 | 1.00 | 61.76 | GZ00 | C |
| ATOM | 6134 | N | GLY | X | 162 | -18.809 | 114.954 | -13.231 | 1.00 | 53.42 | GZ00 | N |
| ATOM | 6135 | CA | GLY | X | 162 | -20.048 | 115.575 | -12.823 | 1.00 | 55.17 | GZ00 | C |
| ATOM | 6136 | C | GLY | X | 162 | -21.273 | 115.133 | -13.595 | 1.00 | 59.06 | GZ00 | C |
| ATOM | 6137 | O | GLY | X | 162 | -22.353 | 115.706 | -13.396 | 1.00 | 55.42 | GZ00 | O |
| ATOM | 6138 | N | VAL | X | 163 | -21.147 | 114.128 | -14.458 | 1.00 | 52.82 | GZ00 | N |
| ATOM | 6139 | CA | VAL | X | 163 | -22.234 | 113.680 | -15.316 | 1.00 | 48.37 | GZ00 | C |
| ATOM | 6140 | C | VAL | X | 163 | -22.874 | 112.455 | -14.689 | 1.00 | 49.71 | GZ00 | C |
| ATOM | 6141 | O | VAL | X | 163 | -22.179 | 111.511 | -14.296 | 1.00 | 50.43 | GZ00 | O |
| ATOM | 6142 | CB | VAL | X | 163 | -21.738 | 113.364 | -16.737 | 1.00 | 45.16 | GZ00 | C |
| ATOM | 6143 | CG1 | VAL | X | 163 | -22.863 | 112.761 | -17.564 | 1.00 | 41.69 | GZ00 | C |
| ATOM | 6144 | CG2 | VAL | X | 163 | -21.220 | 114.622 | -17.401 | 1.00 | 46.72 | GZ00 | C |
| ATOM | 6145 | N | GLU | X | 164 | -24.197 | 112.468 | -14.599 | 1.00 | 47.13 | GZ00 | N |
| ATOM | 6146 | CA | GLU | X | 164 | -24.964 | 111.300 | -14.201 | 1.00 | 53.09 | GZ00 | C |
| ATOM | 6147 | C | GLU | X | 164 | -26.115 | 111.140 | -15.178 | 1.00 | 46.61 | GZ00 | C |
| ATOM | 6148 | O | GLU | X | 164 | -26.815 | 112.113 | -15.471 | 1.00 | 51.75 | GZ00 | O |
| ATOM | 6149 | CB | GLU | X | 164 | -25.447 | 111.448 | -12.757 | 1.00 | 51.29 | GZ00 | C |
| ATOM | 6150 | CG | GLU | X | 164 | -24.369 | 110.989 | -11.771 | 1.00 | 66.35 | GZ00 | C |
| ATOM | 6151 | CD | GLU | X | 164 | -24.588 | 111.481 | -10.349 | 1.00 | 80.34 | GZ00 | C |
| ATOM | 6152 | OE1 | GLU | X | 164 | -25.618 | 112.153 | -10.090 | 1.00 | 84.11 | GZ00 | O |
| ATOM | 6153 | OE2 | GLU | X | 164 | -23.723 | 111.189 | -9.486 | 1.00 | 74.82 | GZ00 | O1- |
| ATOM | 6154 | N | THR | X | 165 | -26.291 | 109.923 | -15.699 | 1.00 | 44.17 | GZ00 | N |
| ATOM | 6155 | CA | THR | X | 165 | -27.216 | 109.646 | -16.791 | 1.00 | 41.22 | GZ00 | C |
| ATOM | 6156 | C | THR | X | 165 | -28.051 | 108.419 | -16.450 | 1.00 | 43.11 | GZ00 | C |
| ATOM | 6157 | O | THR | X | 165 | -27.536 | 107.449 | -15.891 | 1.00 | 46.87 | GZ00 | O |
| ATOM | 6158 | CB | THR | X | 165 | -26.455 | 109.433 | -18.098 | 1.00 | 41.49 | GZ00 | C |
| ATOM | 6159 | OG1 | THR | X | 165 | -25.742 | 110.625 | -18.421 | 1.00 | 41.60 | GZ00 | O |
| ATOM | 6160 | CG2 | THR | X | 165 | -27.397 | 109.091 | -19.242 | 1.00 | 40.37 | GZ00 | C |
| ATOM | 6161 | N | THR | X | 166 | -29.352 | 108.492 | -16.715 | 1.00 | 45.39 | GZ00 | N |
| ATOM | 6162 | CA | THR | X | 166 | -30.228 | 107.356 | -16.466 | 1.00 | 48.92 | GZ00 | C |
| ATOM | 6163 | C | THR | X | 166 | -30.165 | 106.353 | -17.611 | 1.00 | 43.82 | GZ00 | C |
| ATOM | 6164 | O | THR | X | 166 | -29.861 | 106.689 | -18.756 | 1.00 | 48.48 | GZ00 | O |
| ATOM | 6165 | CB | THR | X | 166 | -31.684 | 107.786 | -16.285 | 1.00 | 42.46 | GZ00 | C |
| ATOM | 6166 | OG1 | THR | X | 166 | -32.133 | 108.501 | -17.446 | 1.00 | 42.85 | GZ00 | O |
| ATOM | 6167 | CG2 | THR | X | 166 | -31.833 | 108.639 | -15.075 | 1.00 | 49.93 | GZ00 | C |
| ATOM | 6168 | N | VAL | X | 167 | -30.481 | 105.109 | -17.287 | 1.00 | 43.83 | GZ00 | N |
| ATOM | 6169 | CA | VAL | X | 167 | -30.761 | 104.109 | -18.306 | 1.00 | 47.99 | GZ00 | C |
| ATOM | 6170 | C | VAL | X | 167 | -32.044 | 104.535 | -19.004 | 1.00 | 43.14 | GZ00 | C |
| ATOM | 6171 | O | VAL | X | 167 | -32.921 | 105.146 | -18.381 | 1.00 | 51.16 | GZ00 | O |
| ATOM | 6172 | CB | VAL | X | 167 | -30.905 | 102.716 | -17.689 | 1.00 | 47.80 | GZ00 | C |

| | | | | | | | | | | | | |
|---|---|---|---|---|---|---|---|---|---|---|---|---|
| ATOM | 6173 | CG1 | VAL | X | 167 | -29.736 | 102.444 | -16.786 | 1.00 | 37.96 | GZ00 | C |
| ATOM | 6174 | CG2 | VAL | X | 167 | -32.215 | 102.646 | -16.913 | 1.00 | 51.34 | GZ00 | C |
| ATOM | 6175 | N | PRO | X | 168 | -32.201 | 104.266 | -20.280 | 1.00 | 44.56 | GZ00 | N |
| ATOM | 6176 | CA | PRO | X | 168 | -33.464 | 104.616 | -20.934 | 1.00 | 41.00 | GZ00 | C |
| ATOM | 6177 | C | PRO | X | 168 | -34.607 | 103.782 | -20.385 | 1.00 | 47.02 | GZ00 | C |
| ATOM | 6178 | O | PRO | X | 168 | -34.429 | 102.648 | -19.941 | 1.00 | 49.69 | GZ00 | O |
| ATOM | 6179 | CB | PRO | X | 168 | -33.202 | 104.334 | -22.419 | 1.00 | 43.99 | GZ00 | C |
| ATOM | 6180 | CG | PRO | X | 168 | -31.908 | 103.574 | -22.452 | 1.00 | 46.78 | GZ00 | C |
| ATOM | 6181 | CD | PRO | X | 168 | -31.141 | 103.904 | -21.227 | 1.00 | 39.42 | GZ00 | C |
| ATOM | 6182 | N | SER | X | 169 | -35.784 | 104.384 | -20.368 | 1.00 | 47.67 | GZ00 | N |
| ATOM | 6183 | CA | SER | X | 169 | -36.987 | 103.723 | -19.907 | 1.00 | 50.31 | GZ00 | C |
| ATOM | 6184 | C | SER | X | 169 | -38.095 | 103.966 | -20.920 | 1.00 | 49.36 | GZ00 | C |
| ATOM | 6185 | O | SER | X | 169 | -38.168 | 105.022 | -21.563 | 1.00 | 43.92 | GZ00 | O |
| ATOM | 6186 | CB | SER | X | 169 | -37.417 | 104.225 | -18.517 | 1.00 | 43.96 | GZ00 | C |
| ATOM | 6187 | OG | SER | X | 169 | -37.708 | 105.604 | -18.594 | 1.00 | 55.02 | GZ00 | O |
| ATOM | 6188 | N | LYS | X | 170 | -38.955 | 102.967 | -21.057 | 1.00 | 52.10 | GZ00 | N |
| ATOM | 6189 | CA | LYS | X | 170 | -39.974 | 102.986 | -22.090 | 1.00 | 53.24 | GZ00 | C |
| ATOM | 6190 | C | LYS | X | 170 | -41.088 | 103.939 | -21.683 | 1.00 | 52.61 | GZ00 | C |
| ATOM | 6191 | O | LYS | X | 170 | -41.529 | 103.938 | -20.531 | 1.00 | 55.96 | GZ00 | O |
| ATOM | 6192 | CB | LYS | X | 170 | -40.491 | 101.563 | -22.339 | 1.00 | 57.92 | GZ00 | C |
| ATOM | 6193 | CG | LYS | X | 170 | -41.220 | 101.352 | -23.678 | 1.00 | 61.39 | GZ00 | C |
| ATOM | 6194 | CD | LYS | X | 170 | -41.730 | 99.902 | -23.841 | 1.00 | 60.71 | GZ00 | C |
| ATOM | 6195 | CE | LYS | X | 170 | -40.545 | 98.928 | -24.020 | 1.00 | 68.36 | GZ00 | C |
| ATOM | 6196 | NZ | LYS | X | 170 | -40.911 | 97.510 | -24.330 | 1.00 | 76.29 | GZ00 | N1+ |
| ATOM | 6197 | N | GLN | X | 171 | -41.485 | 104.794 | -22.619 | 1.00 | 49.96 | GZ00 | N |
| ATOM | 6198 | CA | GLN | X | 171 | -42.633 | 105.668 | -22.499 | 1.00 | 48.46 | GZ00 | C |
| ATOM | 6199 | C | GLN | X | 171 | -43.897 | 104.888 | -22.851 | 1.00 | 55.64 | GZ00 | C |
| ATOM | 6200 | O | GLN | X | 171 | -43.843 | 103.749 | -23.324 | 1.00 | 58.51 | GZ00 | O |
| ATOM | 6201 | CB | GLN | X | 171 | -42.474 | 106.880 | -23.422 | 1.00 | 47.95 | GZ00 | C |
| ATOM | 6202 | CG | GLN | X | 171 | -41.214 | 107.699 | -23.166 | 1.00 | 44.29 | GZ00 | C |
| ATOM | 6203 | CD | GLN | X | 171 | -40.919 | 108.737 | -24.253 | 1.00 | 49.84 | GZ00 | C |
| ATOM | 6204 | OE1 | GLN | X | 171 | -40.035 | 109.590 | -24.090 | 1.00 | 47.77 | GZ00 | O |
| ATOM | 6205 | NE2 | GLN | X | 171 | -41.651 | 108.667 | -25.365 | 1.00 | 49.40 | GZ00 | N |
| ATOM | 6206 | N | SER | X | 172 | -45.052 | 105.506 | -22.605 | 1.00 | 61.57 | GZ00 | N |
| ATOM | 6207 | CA | SER | X | 172 | -46.313 | 104.841 | -22.927 | 1.00 | 67.22 | GZ00 | C |
| ATOM | 6208 | C | SER | X | 172 | -46.410 | 104.535 | -24.419 | 1.00 | 65.89 | GZ00 | C |
| ATOM | 6209 | O | SER | X | 172 | -46.922 | 103.477 | -24.808 | 1.00 | 65.81 | GZ00 | O |
| ATOM | 6210 | CB | SER | X | 172 | -47.491 | 105.696 | -22.460 | 1.00 | 55.65 | GZ00 | C |
| ATOM | 6211 | OG | SER | X | 172 | -47.391 | 106.989 | -23.016 | 1.00 | 65.82 | GZ00 | O |
| ATOM | 6212 | N | ASN | X | 173 | -45.891 | 105.434 | -25.268 | 1.00 | 64.43 | GZ00 | N |
| ATOM | 6213 | CA | ASN | X | 173 | -45.898 | 105.274 | -26.722 | 1.00 | 55.79 | GZ00 | C |
| ATOM | 6214 | C | ASN | X | 173 | -44.815 | 104.319 | -27.236 | 1.00 | 60.52 | GZ00 | C |
| ATOM | 6215 | O | ASN | X | 173 | -44.548 | 104.300 | -28.446 | 1.00 | 58.94 | GZ00 | O |
| ATOM | 6216 | CB | ASN | X | 173 | -45.762 | 106.634 | -27.419 | 1.00 | 56.26 | GZ00 | C |
| ATOM | 6217 | CG | ASN | X | 173 | -44.404 | 107.291 | -27.198 | 1.00 | 62.34 | GZ00 | C |
| ATOM | 6218 | OD1 | ASN | X | 173 | -43.536 | 106.762 | -26.496 | 1.00 | 55.11 | GZ00 | O |
| ATOM | 6219 | ND2 | ASN | X | 173 | -44.207 | 108.450 | -27.834 | 1.00 | 61.15 | GZ00 | N |
| ATOM | 6220 | N | ASN | X | 174 | -44.162 | 103.573 | -26.348 | 1.00 | 57.78 | GZ00 | N |
| ATOM | 6221 | CA | ASN | X | 174 | -43.139 | 102.573 | -26.649 | 1.00 | 62.92 | GZ00 | C |
| ATOM | 6222 | C | ASN | X | 174 | -41.849 | 103.159 | -27.207 | 1.00 | 62.49 | GZ00 | C |
| ATOM | 6223 | O | ASN | X | 174 | -40.923 | 102.390 | -27.512 | 1.00 | 56.92 | GZ00 | O |
| ATOM | 6224 | CB | ASN | X | 174 | -43.647 | 101.485 | -27.598 | 1.00 | 64.96 | GZ00 | C |
| ATOM | 6225 | CG | ASN | X | 174 | -44.491 | 100.463 | -26.877 | 1.00 | 72.73 | GZ00 | C |
| ATOM | 6226 | OD1 | ASN | X | 174 | -44.329 | 100.265 | -25.673 | 1.00 | 66.54 | GZ00 | O |
| ATOM | 6227 | ND2 | ASN | X | 174 | -45.394 | 99.809 | -27.599 | 1.00 | 78.95 | GZ00 | N |
| ATOM | 6228 | N | LYS | X | 175 | -41.738 | 104.481 | -27.328 | 1.00 | 58.39 | GZ00 | N |
| ATOM | 6229 | CA | LYS | X | 175 | -40.429 | 105.092 | -27.468 | 1.00 | 52.42 | GZ00 | C |
| ATOM | 6230 | C | LYS | X | 175 | -39.796 | 105.219 | -26.086 | 1.00 | 49.60 | GZ00 | C |
| ATOM | 6231 | O | LYS | X | 175 | -40.366 | 104.789 | -25.077 | 1.00 | 48.97 | GZ00 | O |
| ATOM | 6232 | CB | LYS | X | 175 | -40.537 | 106.429 | -28.183 | 1.00 | 49.13 | GZ00 | C |
| ATOM | 6233 | CG | LYS | X | 175 | -41.199 | 106.317 | -29.545 | 1.00 | 48.64 | GZ00 | C |
| ATOM | 6234 | CD | LYS | X | 175 | -41.226 | 107.678 | -30.217 | 1.00 | 55.03 | GZ00 | C |
| ATOM | 6235 | CE | LYS | X | 175 | -42.052 | 107.683 | -31.482 | 1.00 | 50.14 | GZ00 | C |
| ATOM | 6236 | NZ | LYS | X | 175 | -41.863 | 108.996 | -32.147 | 1.00 | 65.58 | GZ00 | N |
| ATOM | 6237 | N | TYR | X | 176 | -38.595 | 105.789 | -26.028 | 1.00 | 45.23 | GZ00 | N |
| ATOM | 6238 | CA | TYR | X | 176 | -37.810 | 105.757 | -24.803 | 1.00 | 46.64 | GZ00 | C |
| ATOM | 6239 | C | TYR | X | 176 | -37.405 | 107.159 | -24.375 | 1.00 | 46.24 | GZ00 | C |
| ATOM | 6240 | O | TYR | X | 176 | -37.332 | 108.091 | -25.185 | 1.00 | 42.79 | GZ00 | O |
| ATOM | 6241 | CB | TYR | X | 176 | -36.559 | 104.893 | -24.956 | 1.00 | 40.24 | GZ00 | C |
| ATOM | 6242 | CG | TYR | X | 176 | -36.865 | 103.419 | -25.082 | 1.00 | 46.22 | GZ00 | C |
| ATOM | 6243 | CD1 | TYR | X | 176 | -37.356 | 102.882 | -26.276 | 1.00 | 40.78 | GZ00 | C |

```
ATOM   6244  CD2 TYR X 176     -36.683 102.567 -24.006  1.00 47.03      GZ00 C
ATOM   6245  CE1 TYR X 176     -37.628 101.537 -26.389  1.00 43.89      GZ00 C
ATOM   6246  CE2 TYR X 176     -36.965 101.215 -24.107  1.00 51.38      GZ00 C
ATOM   6247  CZ  TYR X 176     -37.439 100.706 -25.294  1.00 52.19      GZ00 C
ATOM   6248  OH  TYR X 176     -37.713  99.359 -25.380  1.00 57.66      GZ00 O
ATOM   6249  N   ALA X 177     -37.152 107.290 -23.072  1.00 42.19      GZ00 N
ATOM   6250  CA  ALA X 177     -36.737 108.543 -22.464  1.00 42.58      GZ00 C
ATOM   6251  C   ALA X 177     -35.553 108.309 -21.543  1.00 39.64      GZ00 C
ATOM   6252  O   ALA X 177     -35.393 107.231 -20.966  1.00 43.45      GZ00 O
ATOM   6253  CB  ALA X 177     -37.873 109.197 -21.673  1.00 36.73      GZ00 C
ATOM   6254  N   ALA X 178     -34.735 109.339 -21.397  1.00 35.05      GZ00 N
ATOM   6255  CA  ALA X 178     -33.635 109.306 -20.451  1.00 41.99      GZ00 C
ATOM   6256  C   ALA X 178     -33.310 110.743 -20.087  1.00 37.98      GZ00 C
ATOM   6257  O   ALA X 178     -33.750 111.681 -20.751  1.00 42.02      GZ00 O
ATOM   6258  CB  ALA X 178     -32.406 108.580 -21.021  1.00 32.61      GZ00 C
ATOM   6259  N   SER X 179     -32.574 110.910 -18.999  1.00 43.75      GZ00 N
ATOM   6260  CA  SER X 179     -32.093 112.230 -18.639  1.00 42.28      GZ00 C
ATOM   6261  C   SER X 179     -30.654 112.109 -18.170  1.00 40.65      GZ00 C
ATOM   6262  O   SER X 179     -30.220 111.051 -17.713  1.00 48.12      GZ00 O
ATOM   6263  CB  SER X 179     -32.956 112.897 -17.572  1.00 35.06      GZ00 C
ATOM   6264  OG  SER X 179     -33.250 112.013 -16.525  1.00 37.63      GZ00 O
ATOM   6265  N   SER X 180     -29.921 113.202 -18.326  1.00 41.05      GZ00 N
ATOM   6266  CA  SER X 180     -28.523 113.321 -17.950  1.00 41.22      GZ00 C
ATOM   6267  C   SER X 180     -28.336 114.625 -17.185  1.00 47.40      GZ00 C
ATOM   6268  O   SER X 180     -28.846 115.667 -17.606  1.00 38.98      GZ00 O
ATOM   6269  CB  SER X 180     -27.628 113.305 -19.187  1.00 42.77      GZ00 C
ATOM   6270  OG  SER X 180     -26.270 113.291 -18.824  1.00 40.90      GZ00 O
ATOM   6271  N   TYR X 181     -27.616 114.556 -16.062  1.00 49.96      GZ00 N
ATOM   6272  CA  TYR X 181     -27.366 115.685 -15.179  1.00 43.04      GZ00 C
ATOM   6273  C   TYR X 181     -25.872 115.961 -15.084  1.00 48.60      GZ00 C
ATOM   6274  O   TYR X 181     -25.089 115.054 -14.778  1.00 50.29      GZ00 O
ATOM   6275  CB  TYR X 181     -27.922 115.419 -13.778  1.00 32.89      GZ00 C
ATOM   6276  CG  TYR X 181     -29.417 115.274 -13.722  1.00 40.29      GZ00 C
ATOM   6277  CD2 TYR X 181     -30.224 116.358 -13.413  1.00 36.67      GZ00 C
ATOM   6278  CD1 TYR X 181     -30.030 114.053 -13.999  1.00 41.31      GZ00 C
ATOM   6279  CE2 TYR X 181     -31.614 116.238 -13.366  1.00 40.95      GZ00 C
ATOM   6280  CE1 TYR X 181     -31.402 113.909 -13.951  1.00 38.36      GZ00 C
ATOM   6281  CZ  TYR X 181     -32.195 115.000 -13.636  1.00 46.60      GZ00 C
ATOM   6282  OH  TYR X 181     -33.561 114.845 -13.591  1.00 40.50      GZ00 O
ATOM   6283  N   LEU X 182     -25.488 117.220 -15.298  1.00 46.12      GZ00 N
ATOM   6284  CA  LEU X 182     -24.119 117.692 -15.092  1.00 47.69      GZ00 C
ATOM   6285  C   LEU X 182     -24.094 118.629 -13.889  1.00 50.67      GZ00 C
ATOM   6286  O   LEU X 182     -24.748 119.679 -13.906  1.00 45.67      GZ00 O
ATOM   6287  CB  LEU X 182     -23.582 118.410 -16.332  1.00 50.05      GZ00 C
ATOM   6288  CG  LEU X 182     -22.207 119.070 -16.145  1.00 51.24      GZ00 C
ATOM   6289  CD1 LEU X 182     -21.143 118.063 -15.726  1.00 46.82      GZ00 C
ATOM   6290  CD2 LEU X 182     -21.779 119.815 -17.399  1.00 48.62      GZ00 C
ATOM   6291  N   SER X 183     -23.382 118.231 -12.839  1.00 44.73      GZ00 N
ATOM   6292  CA  SER X 183     -23.218 119.066 -11.658  1.00 53.81      GZ00 C
ATOM   6293  C   SER X 183     -21.967 119.928 -11.797  1.00 58.46      GZ00 C
ATOM   6294  O   SER X 183     -20.896 119.430 -12.152  1.00 65.34      GZ00 O
ATOM   6295  CB  SER X 183     -23.124 118.218 -10.393  1.00 46.16      GZ00 C
ATOM   6296  OG  SER X 183     -24.237 117.370 -10.277  1.00 53.51      GZ00 O
ATOM   6297  N   LEU X 184     -22.104 121.208 -11.480  1.00 52.70      GZ00 N
ATOM   6298  CA  LEU X 184     -21.025 122.174 -11.566  1.00 59.11      GZ00 C
ATOM   6299  C   LEU X 184     -21.049 123.030 -10.308  1.00 62.24      GZ00 C
ATOM   6300  O   LEU X 184     -22.011 123.007  -9.537  1.00 61.48      GZ00 O
ATOM   6301  CB  LEU X 184     -21.178 123.079 -12.796  1.00 57.83      GZ00 C
ATOM   6302  CG  LEU X 184     -21.196 122.468 -14.189  1.00 57.44      GZ00 C
ATOM   6303  CD1 LEU X 184     -21.426 123.563 -15.208  1.00 52.24      GZ00 C
ATOM   6304  CD2 LEU X 184     -19.901 121.740 -14.475  1.00 62.82      GZ00 C
ATOM   6305  N   THR X 185     -19.978 123.805 -10.106  1.00 64.47      GZ00 N
ATOM   6306  CA  THR X 185     -20.070 124.923  -9.176  1.00 60.49      GZ00 C
ATOM   6307  C   THR X 185     -20.580 126.161  -9.899  1.00 61.60      GZ00 C
ATOM   6308  O   THR X 185     -20.455 126.279 -11.129  1.00 56.95      GZ00 O
ATOM   6309  CB  THR X 185     -18.710 125.225  -8.555  1.00 61.74      GZ00 C
ATOM   6310  OG1 THR X 185     -17.813 125.690  -9.577  1.00 64.33      GZ00 O
ATOM   6311  CG2 THR X 185     -18.149 124.005  -7.840  1.00 49.10      GZ00 C
ATOM   6312  N   PRO X 186     -21.194 127.094  -9.165  1.00 63.67      GZ00 N
ATOM   6313  CA  PRO X 186     -21.536 128.392  -9.774  1.00 66.91      GZ00 C
ATOM   6314  C   PRO X 186     -20.364 129.034 -10.512  1.00 66.84      GZ00 C
```

```
ATOM   6315  O   PRO X 186     -20.570 129.710 -11.529  1.00 66.08        GZ00 O
ATOM   6316  CB  PRO X 186     -21.974 129.227  -8.564  1.00 55.91        GZ00 C
ATOM   6317  CG  PRO X 186     -22.496 128.215  -7.584  1.00 54.51        GZ00 C
ATOM   6318  CD  PRO X 186     -21.625 127.004  -7.757  1.00 56.56        GZ00 C
ATOM   6319  N   GLU X 187     -19.133 128.818 -10.030  1.00 69.10        GZ00 N
ATOM   6320  CA  GLU X 187     -17.938 129.365 -10.676  1.00 76.90        GZ00 C
ATOM   6321  C   GLU X 187     -17.718 128.740 -12.049  1.00 75.85        GZ00 C
ATOM   6322  O   GLU X 187     -17.599 129.450 -13.059  1.00 69.16        GZ00 O
ATOM   6323  CB  GLU X 187     -16.708 129.124  -9.791  1.00 78.07        GZ00 C
ATOM   6324  CG  GLU X 187     -17.004 128.966  -8.297  1.00 81.84        GZ00 C
ATOM   6325  CD  GLU X 187     -17.761 130.158  -7.714  1.00100.11        GZ00 C
ATOM   6326  OE1 GLU X 187     -17.435 131.317  -8.086  1.00 95.34        GZ00 O
ATOM   6327  OE2 GLU X 187     -18.692 129.930  -6.901  1.00102.20        GZ00 O1-
ATOM   6328  N   GLN X 188     -17.652 127.402 -12.095  1.00 68.87        GZ00 N
ATOM   6329  CA  GLN X 188     -17.499 126.699 -13.361  1.00 62.65        GZ00 C
ATOM   6330  C   GLN X 188     -18.597 127.093 -14.333  1.00 65.88        GZ00 C
ATOM   6331  O   GLN X 188     -18.350 127.252 -15.533  1.00 66.09        GZ00 O
ATOM   6332  CB  GLN X 188     -17.540 125.195 -13.127  1.00 65.71        GZ00 C
ATOM   6333  CG  GLN X 188     -16.451 124.635 -12.256  1.00 60.72        GZ00 C
ATOM   6334  CD  GLN X 188     -16.821 123.252 -11.764  1.00 66.14        GZ00 C
ATOM   6335  OE1 GLN X 188     -17.996 122.941 -11.617  1.00 70.69        GZ00 O
ATOM   6336  NE2 GLN X 188     -15.827 122.419 -11.504  1.00 73.28        GZ00 N
ATOM   6337  N   TRP X 189     -19.821 127.251 -13.828  1.00 60.70        GZ00 N
ATOM   6338  CA  TRP X 189     -20.941 127.588 -14.695  1.00 61.89        GZ00 C
ATOM   6339  C   TRP X 189     -20.728 128.932 -15.381  1.00 66.39        GZ00 C
ATOM   6340  O   TRP X 189     -20.889 129.048 -16.601  1.00 69.50        GZ00 O
ATOM   6341  CB  TRP X 189     -22.233 127.581 -13.880  1.00 53.09        GZ00 C
ATOM   6342  CG  TRP X 189     -23.388 128.262 -14.520  1.00 48.26        GZ00 C
ATOM   6343  CD1 TRP X 189     -24.099 129.288 -13.998  1.00 54.93        GZ00 C
ATOM   6344  CD2 TRP X 189     -23.972 127.976 -15.799  1.00 53.12        GZ00 C
ATOM   6345  NE1 TRP X 189     -25.102 129.665 -14.859  1.00 60.99        GZ00 N
ATOM   6346  CE2 TRP X 189     -25.047 128.878 -15.976  1.00 52.94        GZ00 C
ATOM   6347  CE3 TRP X 189     -23.704 127.040 -16.803  1.00 54.03        GZ00 C
ATOM   6348  CZ2 TRP X 189     -25.861 128.873 -17.116  1.00 55.81        GZ00 C
ATOM   6349  CZ3 TRP X 189     -24.512 127.036 -17.944  1.00 57.95        GZ00 C
ATOM   6350  CH2 TRP X 189     -25.577 127.953 -18.090  1.00 55.87        GZ00 C
ATOM   6351  N   LYS X 190     -20.337 129.957 -14.619  1.00 75.20        GZ00 N
ATOM   6352  CA  LYS X 190     -20.232 131.302 -15.184  1.00 80.83        GZ00 C
ATOM   6353  C   LYS X 190     -18.940 131.535 -15.962  1.00 78.13        GZ00 C
ATOM   6354  O   LYS X 190     -18.891 132.458 -16.786  1.00 75.06        GZ00 O
ATOM   6355  CB  LYS X 190     -20.398 132.357 -14.081  1.00 73.65        GZ00 C
ATOM   6356  CG  LYS X 190     -21.825 132.394 -13.491  1.00 81.54        GZ00 C
ATOM   6357  CD  LYS X 190     -21.973 133.326 -12.286  1.00 80.94        GZ00 C
ATOM   6358  CE  LYS X 190     -23.413 133.330 -11.769  1.00 79.11        GZ00 C
ATOM   6359  NZ  LYS X 190     -23.602 134.127 -10.516  1.00 82.23        GZ00 N1+
ATOM   6360  N   SER X 191     -17.917 130.698 -15.765  1.00 70.70        GZ00 N
ATOM   6361  CA  SER X 191     -16.625 130.963 -16.388  1.00 77.12        GZ00 C
ATOM   6362  C   SER X 191     -16.645 130.672 -17.891  1.00 80.54        GZ00 C
ATOM   6363  O   SER X 191     -16.183 131.494 -18.691  1.00 90.60        GZ00 O
ATOM   6364  CB  SER X 191     -15.528 130.163 -15.676  1.00 70.51        GZ00 C
ATOM   6365  OG  SER X 191     -15.673 128.776 -15.878  1.00 73.70        GZ00 O
ATOM   6366  N   HIS X 192     -17.193 129.529 -18.303  1.00 79.21        GZ00 N
ATOM   6367  CA  HIS X 192     -17.144 129.148 -19.709  1.00 68.99        GZ00 C
ATOM   6368  C   HIS X 192     -18.216 129.880 -20.503  1.00 68.52        GZ00 C
ATOM   6369  O   HIS X 192     -19.228 130.326 -19.959  1.00 75.08        GZ00 O
ATOM   6370  CB  HIS X 192     -17.328 127.646 -19.858  1.00 67.33        GZ00 C
ATOM   6371  CG  HIS X 192     -16.211 126.849 -19.270  1.00 72.96        GZ00 C
ATOM   6372  ND1 HIS X 192     -16.133 126.565 -17.924  1.00 69.38        GZ00 N
ATOM   6373  CD2 HIS X 192     -15.134 126.260 -19.844  1.00 69.24        GZ00 C
ATOM   6374  CE1 HIS X 192     -15.051 125.843 -17.690  1.00 77.85        GZ00 C
ATOM   6375  NE2 HIS X 192     -14.427 125.644 -18.839  1.00 78.93        GZ00 N
ATOM   6376  N   ARG X 193     -17.980 130.015 -21.811  1.00 67.36        GZ00 N
ATOM   6377  CA  ARG X 193     -18.945 130.743 -22.627  1.00 70.06        GZ00 C
ATOM   6378  C   ARG X 193     -20.261 129.998 -22.711  1.00 73.81        GZ00 C
ATOM   6379  O   ARG X 193     -21.329 130.620 -22.671  1.00 71.37        GZ00 O
ATOM   6380  CB  ARG X 193     -18.411 131.013 -24.033  1.00 83.17        GZ00 C
ATOM   6381  CG  ARG X 193     -17.125 131.815 -24.098  1.00 91.89        GZ00 C
ATOM   6382  CD  ARG X 193     -16.644 131.932 -25.540  1.00 98.59        GZ00 C
ATOM   6383  NE  ARG X 193     -15.566 132.909 -25.682  1.00110.88        GZ00 N
ATOM   6384  CZ  ARG X 193     -14.801 133.031 -26.763  1.00111.56        GZ00 C
ATOM   6385  NH1 ARG X 193     -13.838 133.947 -26.798  1.00100.02        GZ00 N1+
```

| | | | | | | | | | | | | |
|---|---|---|---|---|---|---|---|---|---|---|---|---|
| ATOM | 6386 | NH2 | ARG | X | 193 | -15.019 | 132.260 | -27.824 | 1.00112.85 | | GZ00 | N |
| ATOM | 6387 | N | SER | X | 194 | -20.207 | 128.670 | -22.831 | 1.00 | 80.31 | GZ00 | N |
| ATOM | 6388 | CA | SER | X | 194 | -21.414 | 127.865 | -22.991 | 1.00 | 74.11 | GZ00 | C |
| ATOM | 6389 | C | SER | X | 194 | -21.137 | 126.416 | -22.618 | 1.00 | 71.90 | GZ00 | C |
| ATOM | 6390 | O | SER | X | 194 | -19.991 | 125.955 | -22.604 | 1.00 | 68.02 | GZ00 | O |
| ATOM | 6391 | CB | SER | X | 194 | -21.945 | 127.911 | -24.425 | 1.00 | 69.53 | GZ00 | C |
| ATOM | 6392 | OG | SER | X | 194 | -21.159 | 127.065 | -25.246 | 1.00 | 67.74 | GZ00 | O |
| ATOM | 6393 | N | TYR | X | 195 | -22.224 | 125.690 | -22.382 | 1.00 | 67.26 | GZ00 | N |
| ATOM | 6394 | CA | TYR | X | 195 | -22.180 | 124.258 | -22.168 | 1.00 | 63.57 | GZ00 | C |
| ATOM | 6395 | C | TYR | X | 195 | -23.120 | 123.573 | -23.142 | 1.00 | 64.49 | GZ00 | C |
| ATOM | 6396 | O | TYR | X | 195 | -24.117 | 124.154 | -23.584 | 1.00 | 56.47 | GZ00 | O |
| ATOM | 6397 | CB | TYR | X | 195 | -22.572 | 123.889 | -20.766 | 1.00 | 57.00 | GZ00 | C |
| ATOM | 6398 | CG | TYR | X | 195 | -21.519 | 124.195 | -19.752 | 1.00 | 60.50 | GZ00 | C |
| ATOM | 6399 | CD1 | TYR | X | 195 | -21.344 | 125.486 | -19.268 | 1.00 | 63.71 | GZ00 | C |
| ATOM | 6400 | CD2 | TYR | X | 195 | -20.712 | 123.184 | -19.254 | 1.00 | 60.86 | GZ00 | C |
| ATOM | 6401 | CE1 | TYR | X | 195 | -20.383 | 125.757 | -18.319 | 1.00 | 66.00 | GZ00 | C |
| ATOM | 6402 | CE2 | TYR | X | 195 | -19.756 | 123.440 | -18.310 | 1.00 | 67.49 | GZ00 | C |
| ATOM | 6403 | CZ | TYR | X | 195 | -19.588 | 124.727 | -17.850 | 1.00 | 65.78 | GZ00 | C |
| ATOM | 6404 | OH | TYR | X | 195 | -18.626 | 124.963 | -16.906 | 1.00 | 71.05 | GZ00 | O |
| ATOM | 6405 | N | SER | X | 196 | -22.786 | 122.326 | -23.470 | 1.00 | 62.79 | GZ00 | N |
| ATOM | 6406 | CA | SER | X | 196 | -23.538 | 121.568 | -24.453 | 1.00 | 61.48 | GZ00 | C |
| ATOM | 6407 | C | SER | X | 196 | -23.826 | 120.160 | -23.952 | 1.00 | 61.15 | GZ00 | C |
| ATOM | 6408 | O | SER | X | 196 | -23.001 | 119.525 | -23.279 | 1.00 | 54.07 | GZ00 | O |
| ATOM | 6409 | CB | SER | X | 196 | -22.791 | 121.504 | -25.783 | 1.00 | 63.75 | GZ00 | C |
| ATOM | 6410 | OG | SER | X | 196 | -22.721 | 122.789 | -26.372 | 1.00 | 69.20 | GZ00 | O |
| ATOM | 6411 | N | CYS | X | 197 | -25.014 | 119.690 | -24.294 | 1.00 | 52.83 | GZ00 | N |
| ATOM | 6412 | CA | CYS | X | 197 | -25.428 | 118.315 | -24.085 | 1.00 | 51.91 | GZ00 | C |
| ATOM | 6413 | C | CYS | X | 197 | -25.524 | 117.683 | -25.463 | 1.00 | 55.35 | GZ00 | C |
| ATOM | 6414 | O | CYS | X | 197 | -26.303 | 118.149 | -26.305 | 1.00 | 55.75 | GZ00 | O |
| ATOM | 6415 | CB | CYS | X | 197 | -26.772 | 118.256 | -23.354 | 1.00 | 51.31 | GZ00 | C |
| ATOM | 6416 | SG | CYS | X | 197 | -27.349 | 116.579 | -23.083 | 1.00 | 51.29 | GZ00 | S |
| ATOM | 6417 | N | GLN | X | 198 | -24.716 | 116.655 | -25.704 | 1.00 | 49.43 | GZ00 | N |
| ATOM | 6418 | CA | GLN | X | 198 | -24.668 | 115.979 | -26.996 | 1.00 | 53.99 | GZ00 | C |
| ATOM | 6419 | C | GLN | X | 198 | -25.193 | 114.555 | -26.839 | 1.00 | 53.90 | GZ00 | C |
| ATOM | 6420 | O | GLN | X | 198 | -24.579 | 113.736 | -26.139 | 1.00 | 49.13 | GZ00 | O |
| ATOM | 6421 | CB | GLN | X | 198 | -23.245 | 115.967 | -27.554 | 1.00 | 51.29 | GZ00 | C |
| ATOM | 6422 | CG | GLN | X | 198 | -22.782 | 117.315 | -28.078 | 1.00 | 67.46 | GZ00 | C |
| ATOM | 6423 | CD | GLN | X | 198 | -21.293 | 117.357 | -28.388 | 1.00 | 69.14 | GZ00 | C |
| ATOM | 6424 | OE1 | GLN | X | 198 | -20.565 | 116.404 | -28.128 | 1.00 | 72.78 | GZ00 | O |
| ATOM | 6425 | NE2 | GLN | X | 198 | -20.838 | 118.470 | -28.943 | 1.00 | 73.70 | GZ00 | N |
| ATOM | 6426 | N | VAL | X | 199 | -26.299 | 114.256 | -27.519 | 1.00 | 45.97 | GZ00 | N |
| ATOM | 6427 | CA | VAL | X | 199 | -26.970 | 112.961 | -27.422 | 1.00 | 47.62 | GZ00 | C |
| ATOM | 6428 | C | VAL | X | 199 | -26.780 | 112.222 | -28.743 | 1.00 | 49.39 | GZ00 | C |
| ATOM | 6429 | O | VAL | X | 199 | -27.294 | 112.648 | -29.786 | 1.00 | 43.67 | GZ00 | O |
| ATOM | 6430 | CB | VAL | X | 199 | -28.465 | 113.117 | -27.091 | 1.00 | 38.03 | GZ00 | C |
| ATOM | 6431 | CG1 | VAL | X | 199 | -29.116 | 111.753 | -26.854 | 1.00 | 36.64 | GZ00 | C |
| ATOM | 6432 | CG2 | VAL | X | 199 | -28.642 | 114.009 | -25.881 | 1.00 | 43.16 | GZ00 | C |
| ATOM | 6433 | N | THR | X | 200 | -26.069 | 111.100 | -28.699 | 1.00 | 44.37 | GZ00 | N |
| ATOM | 6434 | CA | THR | X | 200 | -25.870 | 110.288 | -29.888 | 1.00 | 47.13 | GZ00 | C |
| ATOM | 6435 | C | THR | X | 200 | -26.837 | 109.107 | -29.884 | 1.00 | 46.93 | GZ00 | C |
| ATOM | 6436 | O | THR | X | 200 | -26.959 | 108.389 | -28.886 | 1.00 | 37.31 | GZ00 | O |
| ATOM | 6437 | CB | THR | X | 200 | -24.426 | 109.819 | -29.990 | 1.00 | 47.07 | GZ00 | C |
| ATOM | 6438 | OG1 | THR | X | 200 | -23.583 | 110.975 | -30.046 | 1.00 | 49.61 | GZ00 | O |
| ATOM | 6439 | CG2 | THR | X | 200 | -24.222 | 108.955 | -31.246 | 1.00 | 48.16 | GZ00 | C |
| ATOM | 6440 | N | HIS | X | 201 | -27.533 | 108.936 | -31.002 | 1.00 | 40.95 | GZ00 | N |
| ATOM | 6441 | CA | HIS | X | 201 | -28.533 | 107.899 | -31.163 | 1.00 | 45.21 | GZ00 | C |
| ATOM | 6442 | C | HIS | X | 201 | -28.414 | 107.309 | -32.559 | 1.00 | 45.62 | GZ00 | C |
| ATOM | 6443 | O | HIS | X | 201 | -28.609 | 108.023 | -33.549 | 1.00 | 41.52 | GZ00 | O |
| ATOM | 6444 | CB | HIS | X | 201 | -29.928 | 108.468 | -30.953 | 1.00 | 37.81 | GZ00 | C |
| ATOM | 6445 | CG | HIS | X | 201 | -31.009 | 107.472 | -31.174 | 1.00 | 40.25 | GZ00 | C |
| ATOM | 6446 | ND1 | HIS | X | 201 | -31.717 | 107.395 | -32.354 | 1.00 | 39.48 | GZ00 | N |
| ATOM | 6447 | CD2 | HIS | X | 201 | -31.492 | 106.492 | -30.372 | 1.00 | 32.60 | GZ00 | C |
| ATOM | 6448 | CE1 | HIS | X | 201 | -32.614 | 106.425 | -32.257 | 1.00 | 41.48 | GZ00 | C |
| ATOM | 6449 | NE2 | HIS | X | 201 | -32.500 | 105.865 | -31.063 | 1.00 | 36.04 | GZ00 | N |
| ATOM | 6450 | N | GLU | X | 202 | -28.100 | 106.014 | -32.636 | 1.00 | 40.07 | GZ00 | N |
| ATOM | 6451 | CA | GLU | X | 202 | -27.996 | 105.307 | -33.916 | 1.00 | 39.77 | GZ00 | C |
| ATOM | 6452 | C | GLU | X | 202 | -27.068 | 106.042 | -34.889 | 1.00 | 44.82 | GZ00 | C |
| ATOM | 6453 | O | GLU | X | 202 | -27.387 | 106.247 | -36.062 | 1.00 | 40.88 | GZ00 | O |
| ATOM | 6454 | CB | GLU | X | 202 | -29.376 | 105.095 | -34.535 | 1.00 | 36.52 | GZ00 | C |
| ATOM | 6455 | CG | GLU | X | 202 | -30.307 | 104.272 | -33.669 | 1.00 | 41.95 | GZ00 | C |
| ATOM | 6456 | CD | GLU | X | 202 | -29.852 | 102.821 | -33.573 | 1.00 | 51.67 | GZ00 | C |

| | | | | | | | | | | | | |
|---|---|---|---|---|---|---|---|---|---|---|---|---|
| ATOM | 6457 | OE1 | GLU | X | 202 | -29.603 | 102.188 | -34.631 | 1.00 | 51.18 | GZ00 | O |
| ATOM | 6458 | OE2 | GLU | X | 202 | -29.735 | 102.316 | -32.433 | 1.00 | 49.62 | GZ00 | O1- |
| ATOM | 6459 | N | GLY | X | 203 | -25.911 | 106.464 | -34.381 | 1.00 | 41.22 | GZ00 | N |
| ATOM | 6460 | CA | GLY | X | 203 | -24.926 | 107.146 | -35.193 | 1.00 | 42.73 | GZ00 | C |
| ATOM | 6461 | C | GLY | X | 203 | -25.235 | 108.578 | -35.586 | 1.00 | 47.72 | GZ00 | C |
| ATOM | 6462 | O | GLY | X | 203 | -24.445 | 109.169 | -36.321 | 1.00 | 53.27 | GZ00 | O |
| ATOM | 6463 | N | SER | X | 206 | -26.322 | 109.176 | -35.101 | 1.00 | 52.11 | GZ00 | N |
| ATOM | 6464 | CA | SER | X | 206 | -26.642 | 110.577 | -35.375 | 1.00 | 50.86 | GZ00 | C |
| ATOM | 6465 | C | SER | X | 206 | -26.773 | 111.328 | -34.056 | 1.00 | 52.21 | GZ00 | C |
| ATOM | 6466 | O | SER | X | 206 | -27.448 | 110.853 | -33.136 | 1.00 | 52.48 | GZ00 | O |
| ATOM | 6467 | CB | SER | X | 206 | -27.944 | 110.707 | -36.167 | 1.00 | 47.57 | GZ00 | C |
| ATOM | 6468 | OG | SER | X | 206 | -27.838 | 110.125 | -37.454 | 1.00 | 59.00 | GZ00 | O |
| ATOM | 6469 | N | THR | X | 207 | -26.128 | 112.488 | -33.953 | 1.00 | 50.76 | GZ00 | N |
| ATOM | 6470 | CA | THR | X | 207 | -26.108 | 113.241 | -32.704 | 1.00 | 55.87 | GZ00 | C |
| ATOM | 6471 | C | THR | X | 207 | -27.025 | 114.462 | -32.764 | 1.00 | 57.12 | GZ00 | C |
| ATOM | 6472 | O | THR | X | 207 | -27.074 | 115.180 | -33.767 | 1.00 | 57.64 | GZ00 | O |
| ATOM | 6473 | CB | THR | X | 207 | -24.696 | 113.690 | -32.344 | 1.00 | 48.26 | GZ00 | C |
| ATOM | 6474 | OG1 | THR | X | 207 | -24.359 | 114.813 | -33.157 | 1.00 | 73.99 | GZ00 | O |
| ATOM | 6475 | CG2 | THR | X | 207 | -23.702 | 112.580 | -32.600 | 1.00 | 47.46 | GZ00 | C |
| ATOM | 6476 | N | VAL | X | 208 | -27.777 | 114.659 | -31.690 | 1.00 | 49.79 | GZ00 | N |
| ATOM | 6477 | CA | VAL | X | 208 | -28.598 | 115.842 | -31.472 | 1.00 | 52.71 | GZ00 | C |
| ATOM | 6478 | C | VAL | X | 208 | -27.951 | 116.636 | -30.343 | 1.00 | 55.59 | GZ00 | C |
| ATOM | 6479 | O | VAL | X | 208 | -27.557 | 116.063 | -29.317 | 1.00 | 52.08 | GZ00 | O |
| ATOM | 6480 | CB | VAL | X | 208 | -30.047 | 115.452 | -31.131 | 1.00 | 51.79 | GZ00 | C |
| ATOM | 6481 | CG1 | VAL | X | 208 | -30.880 | 116.672 | -30.802 | 1.00 | 49.77 | GZ00 | C |
| ATOM | 6482 | CG2 | VAL | X | 208 | -30.666 | 114.651 | -32.276 | 1.00 | 48.08 | GZ00 | C |
| ATOM | 6483 | N | GLU | X | 209 | -27.787 | 117.939 | -30.547 | 1.00 | 56.25 | GZ00 | N |
| ATOM | 6484 | CA | GLU | X | 209 | -27.107 | 118.778 | -29.571 | 1.00 | 56.90 | GZ00 | C |
| ATOM | 6485 | C | GLU | X | 209 | -27.976 | 119.957 | -29.174 | 1.00 | 58.39 | GZ00 | C |
| ATOM | 6486 | O | GLU | X | 209 | -28.692 | 120.525 | -30.005 | 1.00 | 55.14 | GZ00 | O |
| ATOM | 6487 | CB | GLU | X | 209 | -25.772 | 119.293 | -30.087 | 1.00 | 57.63 | GZ00 | C |
| ATOM | 6488 | CG | GLU | X | 209 | -25.088 | 120.229 | -29.113 | 1.00 | 64.92 | GZ00 | C |
| ATOM | 6489 | CD | GLU | X | 209 | -23.766 | 120.754 | -29.630 | 1.00 | 76.32 | GZ00 | C |
| ATOM | 6490 | OE1 | GLU | X | 209 | -22.869 | 119.942 | -29.932 | 1.00 | 78.95 | GZ00 | O |
| ATOM | 6491 | OE2 | GLU | X | 209 | -23.636 | 121.986 | -29.766 | 1.00 | 85.57 | GZ00 | O1- |
| ATOM | 6492 | N | LYS | X | 210 | -27.935 | 120.282 | -27.886 | 1.00 | 62.74 | GZ00 | N |
| ATOM | 6493 | CA | LYS | X | 210 | -28.467 | 121.522 | -27.345 | 1.00 | 53.79 | GZ00 | C |
| ATOM | 6494 | C | LYS | X | 210 | -27.379 | 122.171 | -26.505 | 1.00 | 56.56 | GZ00 | C |
| ATOM | 6495 | O | LYS | X | 210 | -26.616 | 121.476 | -25.823 | 1.00 | 56.28 | GZ00 | O |
| ATOM | 6496 | CB | LYS | X | 210 | -29.711 | 121.279 | -26.513 | 1.00 | 53.44 | GZ00 | C |
| ATOM | 6497 | CG | LYS | X | 210 | -30.907 | 120.841 | -27.324 | 1.00 | 54.02 | GZ00 | C |
| ATOM | 6498 | CD | LYS | X | 210 | -31.098 | 121.748 | -28.509 | 1.00 | 54.29 | GZ00 | C |
| ATOM | 6499 | CE | LYS | X | 210 | -32.343 | 121.367 | -29.282 | 1.00 | 55.10 | GZ00 | C |
| ATOM | 6500 | NZ | LYS | X | 210 | -33.545 | 121.832 | -28.528 | 1.00 | 61.72 | GZ00 | N1+ |
| ATOM | 6501 | N | THR | X | 211 | -27.270 | 123.498 | -26.592 | 1.00 | 59.32 | GZ00 | N |
| ATOM | 6502 | CA | THR | X | 211 | -26.267 | 124.229 | -25.828 | 1.00 | 59.32 | GZ00 | C |
| ATOM | 6503 | C | THR | X | 211 | -26.946 | 125.341 | -25.039 | 1.00 | 55.85 | GZ00 | C |
| ATOM | 6504 | O | THR | X | 211 | -27.984 | 125.869 | -25.443 | 1.00 | 52.37 | GZ00 | O |
| ATOM | 6505 | CB | THR | X | 211 | -25.177 | 124.830 | -26.725 | 1.00 | 59.58 | GZ00 | C |
| ATOM | 6506 | OG1 | THR | X | 211 | -25.586 | 126.134 | -27.141 | 1.00 | 68.27 | GZ00 | O |
| ATOM | 6507 | CG2 | THR | X | 211 | -24.975 | 123.970 | -27.975 | 1.00 | 57.88 | GZ00 | C |
| ATOM | 6508 | N | VAL | X | 212 | -26.348 | 125.706 | -23.911 | 1.00 | 52.69 | GZ00 | N |
| ATOM | 6509 | CA | VAL | X | 212 | -26.936 | 126.709 | -23.035 | 1.00 | 63.41 | GZ00 | C |
| ATOM | 6510 | C | VAL | X | 212 | -25.825 | 127.640 | -22.548 | 1.00 | 67.46 | GZ00 | C |
| ATOM | 6511 | O | VAL | X | 212 | -24.665 | 127.233 | -22.408 | 1.00 | 65.23 | GZ00 | O |
| ATOM | 6512 | CB | VAL | X | 212 | -27.702 | 126.029 | -21.866 | 1.00 | 57.88 | GZ00 | C |
| ATOM | 6513 | CG1 | VAL | X | 212 | -26.783 | 125.712 | -20.700 | 1.00 | 44.64 | GZ00 | C |
| ATOM | 6514 | CG2 | VAL | X | 212 | -28.897 | 126.846 | -21.442 | 1.00 | 65.99 | GZ00 | C |
| ATOM | 6515 | N | ALA | X | 213 | -26.174 | 128.916 | -22.332 | 1.00 | 65.82 | GZ00 | N |
| ATOM | 6516 | CA | ALA | X | 213 | -25.166 | 129.919 | -21.988 | 1.00 | 71.28 | GZ00 | C |
| ATOM | 6517 | C | ALA | X | 213 | -25.503 | 130.667 | -20.702 | 1.00 | 73.86 | GZ00 | C |
| ATOM | 6518 | O | ALA | X | 213 | -26.677 | 130.982 | -20.445 | 1.00 | 75.77 | GZ00 | O |
| ATOM | 6519 | CB | ALA | X | 213 | -25.003 | 130.928 | -23.133 | 1.00 | 69.34 | GZ00 | C |
| ATOM | 6520 | N | PRO | X | 214 | -24.486 | 130.994 | -19.889 | 1.00 | 70.92 | GZ00 | N |
| ATOM | 6521 | CA | PRO | X | 214 | -24.746 | 131.692 | -18.614 | 1.00 | 72.76 | GZ00 | C |
| ATOM | 6522 | C | PRO | X | 214 | -25.314 | 133.090 | -18.773 | 1.00 | 82.62 | GZ00 | C |
| ATOM | 6523 | O | PRO | X | 214 | -25.933 | 133.596 | -17.827 | 1.00 | 89.01 | GZ00 | O |
| ATOM | 6524 | CB | PRO | X | 214 | -23.366 | 131.732 | -17.943 | 1.00 | 66.71 | GZ00 | C |
| ATOM | 6525 | CG | PRO | X | 214 | -22.637 | 130.585 | -18.525 | 1.00 | 65.72 | GZ00 | C |
| ATOM | 6526 | CD | PRO | X | 214 | -23.119 | 130.445 | -19.944 | 1.00 | 70.60 | GZ00 | C |
| ATOM | 6527 | N | THR | X | 215 | -25.149 | 133.725 | -19.928 | 1.00 | 84.77 | GZ00 | N |

```
ATOM   6528  CA  THR X 215     -25.832 134.986 -20.202  1.00 96.30      GZ00 C
ATOM   6529  C   THR X 215     -27.332 134.702 -20.252  1.00 95.28      GZ00 C
ATOM   6530  O   THR X 215     -27.859 134.231 -21.265  1.00 91.48      GZ00 O
ATOM   6531  CB  THR X 215     -25.328 135.593 -21.510  1.00 97.14      GZ00 C
ATOM   6532  OG1 THR X 215     -25.743 134.773 -22.612  1.00 87.67      GZ00 O
ATOM   6533  CG2 THR X 215     -23.793 135.691 -21.507  1.00 90.01      GZ00 C
ATOM   6534  N   GLU X 216     -28.027 134.994 -19.152  1.00 96.06      GZ00 N
ATOM   6535  CA  GLU X 216     -29.446 134.662 -19.010  1.00 98.88      GZ00 C
ATOM   6536  C   GLU X 216     -30.325 135.917 -18.923  1.00 99.86      GZ00 C
ATOM   6537  O   GLU X 216     -31.553 135.830 -18.821  1.00 94.58      GZ00 O
ATOM   6538  CB  GLU X 216     -29.662 133.791 -17.762  1.00104.37      GZ00 C
ATOM   6539  CG  GLU X 216     -29.007 132.394 -17.811  1.00103.51      GZ00 C
ATOM   6540  CD  GLU X 216     -29.281 131.561 -16.556  1.00100.31      GZ00 C
ATOM   6541  OE1 GLU X 216     -30.463 131.201 -16.329  1.00 98.05      GZ00 O
ATOM   6542  OE2 GLU X 216     -28.323 131.288 -15.788  1.00 94.94      GZ00 O1-
TER
ATOM   6543  N   THR E 152      -7.503 113.907   0.585  1.00 78.03      B000 N
ATOM   6544  CA  THR E 152      -7.804 113.632   1.990  1.00 91.98      B000 C
ATOM   6545  C   THR E 152      -6.989 112.446   2.537  1.00 87.18      B000 C
ATOM   6546  O   THR E 152      -5.914 112.642   3.113  1.00 87.17      B000 O
ATOM   6547  CB  THR E 152      -9.331 113.377   2.199  1.00 99.46      B000 C
ATOM   6548  OG1 THR E 152      -9.547 112.614   3.396  1.00103.56      B000 O
ATOM   6549  CG2 THR E 152      -9.959 112.661   0.997  1.00 88.04      B000 C
ATOM   6550  N   CYS E 153      -7.511 111.230   2.378  1.00 83.15      B000 N
ATOM   6551  CA  CYS E 153      -6.804 109.997   2.699  1.00 86.88      B000 C
ATOM   6552  C   CYS E 153      -6.722 109.106   1.463  1.00 80.76      B000 C
ATOM   6553  O   CYS E 153      -7.463 109.278   0.486  1.00 71.27      B000 O
ATOM   6554  CB  CYS E 153      -7.471 109.227   3.860  1.00 86.61      B000 C
ATOM   6555  SG  CYS E 153      -6.738 109.471   5.513  1.00 90.81      B000 S
ATOM   6556  N   CYS E 154      -5.817 108.133   1.525  1.00 72.71      B000 N
ATOM   6557  CA  CYS E 154      -5.632 107.218   0.411  1.00 64.86      B000 C
ATOM   6558  C   CYS E 154      -6.850 106.311   0.243  1.00 66.48      B000 C
ATOM   6559  O   CYS E 154      -7.564 106.023   1.210  1.00 66.73      B000 O
ATOM   6560  CB  CYS E 154      -4.385 106.373   0.626  1.00 57.69      B000 C
ATOM   6561  SG  CYS E 154      -2.833 107.296   0.371  1.00 72.69      B000 S
ATOM   6562  N   PRO E 155      -7.115 105.854  -0.978  1.00 56.60      B000 N
ATOM   6563  CA  PRO E 155      -8.220 104.914  -1.192  1.00 57.19      B000 C
ATOM   6564  C   PRO E 155      -8.052 103.661  -0.345  1.00 62.02      B000 C
ATOM   6565  O   PRO E 155      -7.012 103.404   0.267  1.00 60.28      B000 O
ATOM   6566  CB  PRO E 155      -8.143 104.589  -2.687  1.00 52.85      B000 C
ATOM   6567  CG  PRO E 155      -7.332 105.677  -3.277  1.00 56.95      B000 C
ATOM   6568  CD  PRO E 155      -6.392 106.158  -2.220  1.00 54.57      B000 C
ATOM   6569  N   VAL E 156      -9.123 102.871  -0.306  1.00 64.34      B000 N
ATOM   6570  CA  VAL E 156      -9.121 101.644   0.477  1.00 62.48      B000 C
ATOM   6571  C   VAL E 156      -8.013 100.726  -0.021  1.00 57.31      B000 C
ATOM   6572  O   VAL E 156      -7.843 100.536  -1.235  1.00 55.79      B000 O
ATOM   6573  CB  VAL E 156     -10.498 100.968   0.373  1.00 58.69      B000 C
ATOM   6574  CG1 VAL E 156     -10.628  99.853   1.400  1.00 62.99      B000 C
ATOM   6575  CG2 VAL E 156     -11.612 102.013   0.508  1.00 72.16      B000 C
ATOM   6576  N   ASN E 157      -7.244 100.163   0.915  1.00 49.37      B000 N
ATOM   6577  CA  ASN E 157      -6.142  99.218   0.687  1.00 55.22      B000 C
ATOM   6578  C   ASN E 157      -4.871  99.882   0.162  1.00 52.64      B000 C
ATOM   6579  O   ASN E 157      -3.871  99.180  -0.036  1.00 49.46      B000 O
ATOM   6580  CB  ASN E 157      -6.513  98.075  -0.276  1.00 51.75      B000 C
ATOM   6581  CG  ASN E 157      -7.713  97.282   0.202  1.00 58.73      B000 C
ATOM   6582  OD1 ASN E 157      -7.839  97.001   1.392  1.00 60.67      B000 O
ATOM   6583  ND2 ASN E 157      -8.609  96.935  -0.720  1.00 58.30      B000 N
ATOM   6584  N   TRP E 158      -4.868 101.187  -0.082  1.00 49.22      B000 N
ATOM   6585  CA  TRP E 158      -3.644 101.885  -0.433  1.00 45.78      B000 C
ATOM   6586  C   TRP E 158      -2.983 102.464   0.818  1.00 47.63      B000 C
ATOM   6587  O   TRP E 158      -3.618 102.655   1.855  1.00 53.96      B000 O
ATOM   6588  CB  TRP E 158      -3.933 102.979  -1.453  1.00 48.76      B000 C
ATOM   6589  CG  TRP E 158      -4.381 102.473  -2.793  1.00 40.31      B000 C
ATOM   6590  CD1 TRP E 158      -5.498 101.731  -3.072  1.00 46.46      B000 C
ATOM   6591  CD2 TRP E 158      -3.718 102.683  -4.043  1.00 35.79      B000 C
ATOM   6592  NE1 TRP E 158      -5.573 101.465  -4.432  1.00 39.86      B000 N
ATOM   6593  CE2 TRP E 158      -4.495 102.050  -5.047  1.00 45.74      B000 C
ATOM   6594  CE3 TRP E 158      -2.548 103.355  -4.417  1.00 40.22      B000 C
ATOM   6595  CZ2 TRP E 158      -4.132 102.067  -6.395  1.00 41.97      B000 C
ATOM   6596  CZ3 TRP E 158      -2.193 103.378  -5.764  1.00 41.46      B000 C
ATOM   6597  CH2 TRP E 158      -2.980 102.727  -6.732  1.00 40.39      B000 C
```

```
ATOM   6598  N   VAL E 159      -1.689 102.734   0.702  1.00 44.22      B000 N
ATOM   6599  CA  VAL E 159      -0.837 103.172   1.800  1.00 48.43      B000 C
ATOM   6600  C   VAL E 159      -0.297 104.558   1.461  1.00 58.07      B000 C
ATOM   6601  O   VAL E 159       0.103 104.818   0.318  1.00 55.09      B000 O
ATOM   6602  CB  VAL E 159       0.330 102.189   2.039  1.00 52.63      B000 C
ATOM   6603  CG1 VAL E 159       1.251 102.704   3.126  1.00 54.92      B000 C
ATOM   6604  CG2 VAL E 159      -0.184 100.818   2.407  1.00 53.05      B000 C
ATOM   6605  N   GLU E 160      -0.275 105.443   2.456  1.00 57.15      B000 N
ATOM   6606  CA  GLU E 160       0.120 106.833   2.256  1.00 59.51      B000 C
ATOM   6607  C   GLU E 160       1.577 107.054   2.654  1.00 55.30      B000 C
ATOM   6608  O   GLU E 160       2.043 106.531   3.673  1.00 50.50      B000 O
ATOM   6609  CB  GLU E 160      -0.809 107.756   3.046  1.00 51.43      B000 C
ATOM   6610  CG  GLU E 160      -0.440 109.219   3.035  1.00 65.54      B000 C
ATOM   6611  CD  GLU E 160      -1.336 110.058   3.952  1.00 77.96      B000 C
ATOM   6612  OE1 GLU E 160      -2.418 109.568   4.357  1.00 79.30      B000 O
ATOM   6613  OE2 GLU E 160      -0.951 111.203   4.281  1.00 75.66      B000 O1-
ATOM   6614  N   HIS E 161       2.304 107.801   1.822  1.00 53.62      B000 N
ATOM   6615  CA  HIS E 161       3.658 108.222   2.177  1.00 53.50      B000 C
ATOM   6616  C   HIS E 161       4.013 109.474   1.393  1.00 61.66      B000 C
ATOM   6617  O   HIS E 161       3.896 109.472   0.159  1.00 55.60      B000 O
ATOM   6618  CB  HIS E 161       4.656 107.120   1.884  1.00 50.97      B000 C
ATOM   6619  CG  HIS E 161       6.076 107.514   2.135  1.00 58.56      B000 C
ATOM   6620  ND1 HIS E 161       6.845 108.173   1.196  1.00 62.19      B000 N
ATOM   6621  CD2 HIS E 161       6.877 107.322   3.213  1.00 53.33      B000 C
ATOM   6622  CE1 HIS E 161       8.056 108.376   1.687  1.00 63.04      B000 C
ATOM   6623  NE2 HIS E 161       8.102 107.869   2.909  1.00 61.41      B000 N
ATOM   6624  N   GLU E 162       4.410 110.541   2.112  1.00 61.25      B000 N
ATOM   6625  CA  GLU E 162       4.767 111.850   1.561  1.00 55.02      B000 C
ATOM   6626  C   GLU E 162       3.975 112.247   0.322  1.00 62.64      B000 C
ATOM   6627  O   GLU E 162       4.537 112.301  -0.778  1.00 75.86      B000 O
ATOM   6628  CB  GLU E 162       6.267 111.936   1.225  1.00 60.44      B000 C
ATOM   6629  CG  GLU E 162       7.223 111.633   2.366  1.00 61.24      B000 C
ATOM   6630  CD  GLU E 162       8.697 111.610   1.933  1.00 86.98      B000 C
ATOM   6631  OE1 GLU E 162       9.025 111.099   0.822  1.00 83.67      B000 O
ATOM   6632  OE2 GLU E 162       9.538 112.097   2.725  1.00 95.90      B000 O1-
ATOM   6633  N   ARG E 163       2.679 112.496   0.469  1.00 55.87      B000 N
ATOM   6634  CA  ARG E 163       1.819 113.000  -0.602  1.00 76.66      B000 C
ATOM   6635  C   ARG E 163       1.554 112.012  -1.738  1.00 68.88      B000 C
ATOM   6636  O   ARG E 163       0.922 112.388  -2.739  1.00 65.44      B000 O
ATOM   6637  CB  ARG E 163       2.398 114.288  -1.215  1.00 89.70      B000 C
ATOM   6638  CG  ARG E 163       2.928 115.328  -0.208  1.00 98.04      B000 C
ATOM   6639  CD  ARG E 163       3.983 116.257  -0.835  1.00106.40      B000 C
ATOM   6640  NE  ARG E 163       3.452 117.044  -1.950  1.00124.58      B000 N
ATOM   6641  CZ  ARG E 163       3.588 116.723  -3.236  1.00120.88      B000 C
ATOM   6642  NH1 ARG E 163       4.245 115.622  -3.585  1.00111.66      B000 N1+
ATOM   6643  NH2 ARG E 163       3.063 117.503  -4.176  1.00118.93      B000 N
ATOM   6644  N   SER E 164       1.973 110.758  -1.622  1.00 63.49      B000 N
ATOM   6645  CA  SER E 164       1.606 109.768  -2.620  1.00 57.22      B000 C
ATOM   6646  C   SER E 164       0.888 108.601  -1.952  1.00 52.22      B000 C
ATOM   6647  O   SER E 164       1.071 108.329  -0.761  1.00 49.98      B000 O
ATOM   6648  CB  SER E 164       2.823 109.290  -3.411  1.00 56.71      B000 C
ATOM   6649  OG  SER E 164       3.161 110.233  -4.411  1.00 65.04      B000 O
ATOM   6650  N   CYS E 165       0.066 107.913  -2.740  1.00 50.88      B000 N
ATOM   6651  CA  CYS E 165      -0.614 106.691  -2.322  1.00 53.42      B000 C
ATOM   6652  C   CYS E 165      -0.042 105.497  -3.076  1.00 50.12      B000 C
ATOM   6653  O   CYS E 165       0.124 105.555  -4.300  1.00 46.54      B000 O
ATOM   6654  CB  CYS E 165      -2.118 106.793  -2.576  1.00 56.64      B000 C
ATOM   6655  SG  CYS E 165      -2.945 108.028  -1.541  1.00 67.86      B000 S
ATOM   6656  N   TYR E 166       0.226 104.410  -2.350  1.00 52.80      B000 N
ATOM   6657  CA  TYR E 166       0.904 103.231  -2.882  1.00 48.23      B000 C
ATOM   6658  C   TYR E 166       0.064 101.984  -2.650  1.00 48.96      B000 C
ATOM   6659  O   TYR E 166      -0.538 101.821  -1.584  1.00 46.07      B000 O
ATOM   6660  CB  TYR E 166       2.265 103.033  -2.222  1.00 45.49      B000 C
ATOM   6661  CG  TYR E 166       3.196 104.186  -2.406  1.00 51.14      B000 C
ATOM   6662  CD1 TYR E 166       3.000 105.382  -1.709  1.00 50.78      B000 C
ATOM   6663  CD2 TYR E 166       4.296 104.081  -3.249  1.00 49.21      B000 C
ATOM   6664  CE1 TYR E 166       3.859 106.442  -1.867  1.00 52.01      B000 C
ATOM   6665  CE2 TYR E 166       5.175 105.138  -3.408  1.00 52.82      B000 C
ATOM   6666  CZ  TYR E 166       4.954 106.317  -2.714  1.00 56.72      B000 C
ATOM   6667  OH  TYR E 166       5.829 107.368  -2.874  1.00 55.75      B000 O
ATOM   6668  N   TRP E 167       0.064 101.085  -3.634  1.00 43.75      B000 N
```

| | | | | | | | | | | | | |
|---|---|---|---|---|---|---|---|---|---|---|---|---|
| ATOM | 6669 | CA | TRP | E | 167 | -0.639 | 99.813 | -3.539 | 1.00 | 41.16 | B000 | C |
| ATOM | 6670 | C | TRP | E | 167 | 0.346 | 98.684 | -3.794 | 1.00 | 42.24 | B000 | C |
| ATOM | 6671 | O | TRP | E | 167 | 1.054 | 98.693 | -4.804 | 1.00 | 37.42 | B000 | O |
| ATOM | 6672 | CB | TRP | E | 167 | -1.804 | 99.743 | -4.530 | 1.00 | 42.53 | B000 | C |
| ATOM | 6673 | CG | TRP | E | 167 | -2.599 | 98.467 | -4.395 | 1.00 | 46.44 | B000 | C |
| ATOM | 6674 | CD1 | TRP | E | 167 | -3.662 | 98.242 | -3.560 | 1.00 | 49.81 | B000 | C |
| ATOM | 6675 | CD2 | TRP | E | 167 | -2.395 | 97.251 | -5.116 | 1.00 | 43.13 | B000 | C |
| ATOM | 6676 | NE1 | TRP | E | 167 | -4.114 | 96.955 | -3.706 | 1.00 | 51.07 | B000 | N |
| ATOM | 6677 | CE2 | TRP | E | 167 | -3.366 | 96.330 | -4.665 | 1.00 | 46.50 | B000 | C |
| ATOM | 6678 | CE3 | TRP | E | 167 | -1.491 | 96.852 | -6.103 | 1.00 | 40.37 | B000 | C |
| ATOM | 6679 | CZ2 | TRP | E | 167 | -3.455 | 95.041 | -5.165 | 1.00 | 36.86 | B000 | C |
| ATOM | 6680 | CZ3 | TRP | E | 167 | -1.587 | 95.579 | -6.604 | 1.00 | 39.69 | B000 | C |
| ATOM | 6681 | CH2 | TRP | E | 167 | -2.554 | 94.682 | -6.133 | 1.00 | 42.15 | B000 | C |
| ATOM | 6682 | N | PHE | E | 168 | 0.377 | 97.709 | -2.891 | 1.00 | 42.39 | B000 | N |
| ATOM | 6683 | CA | PHE | E | 168 | 1.369 | 96.645 | -2.921 | 1.00 | 39.44 | B000 | C |
| ATOM | 6684 | C | PHE | E | 168 | 0.718 | 95.320 | -3.300 | 1.00 | 41.71 | B000 | C |
| ATOM | 6685 | O | PHE | E | 168 | -0.056 | 94.762 | -2.515 | 1.00 | 39.56 | B000 | O |
| ATOM | 6686 | CB | PHE | E | 168 | 2.054 | 96.539 | -1.567 | 1.00 | 33.24 | B000 | C |
| ATOM | 6687 | CG | PHE | E | 168 | 2.834 | 97.745 | -1.209 | 1.00 | 45.15 | B000 | C |
| ATOM | 6688 | CD1 | PHE | E | 168 | 4.165 | 97.858 | -1.603 | 1.00 | 43.49 | B000 | C |
| ATOM | 6689 | CD2 | PHE | E | 168 | 2.251 | 98.776 | -0.472 | 1.00 | 43.18 | B000 | C |
| ATOM | 6690 | CE1 | PHE | E | 168 | 4.917 | 98.991 | -1.279 | 1.00 | 48.63 | B000 | C |
| ATOM | 6691 | CE2 | PHE | E | 168 | 2.986 | 99.913 | -0.135 | 1.00 | 49.01 | B000 | C |
| ATOM | 6692 | CZ | PHE | E | 168 | 4.326 | 100.026 | -0.541 | 1.00 | 48.19 | B000 | C |
| ATOM | 6693 | N | SER | E | 169 | 1.081 | 94.788 | -4.469 | 1.00 | 34.62 | B000 | N |
| ATOM | 6694 | CA | SER | E | 169 | 0.586 | 93.478 | -4.863 | 1.00 | 37.57 | B000 | C |
| ATOM | 6695 | C | SER | E | 169 | 1.143 | 92.402 | -3.936 | 1.00 | 34.54 | B000 | C |
| ATOM | 6696 | O | SER | E | 169 | 2.169 | 92.574 | -3.280 | 1.00 | 36.05 | B000 | O |
| ATOM | 6697 | CB | SER | E | 169 | 0.978 | 93.141 | -6.308 | 1.00 | 33.43 | B000 | C |
| ATOM | 6698 | OG | SER | E | 169 | 2.306 | 92.629 | -6.362 | 1.00 | 34.63 | B000 | O |
| ATOM | 6699 | N | ARG | E | 170 | 0.449 | 91.272 | -3.899 | 1.00 | 35.37 | B000 | N |
| ATOM | 6700 | CA | ARG | E | 170 | 0.918 | 90.093 | -3.184 | 1.00 | 37.90 | B000 | C |
| ATOM | 6701 | C | ARG | E | 170 | 0.997 | 88.896 | -4.131 | 1.00 | 35.62 | B000 | C |
| ATOM | 6702 | O | ARG | E | 170 | 0.924 | 87.742 | -3.715 | 1.00 | 37.12 | B000 | O |
| ATOM | 6703 | CB | ARG | E | 170 | 0.028 | 89.827 | -1.967 | 1.00 | 36.19 | B000 | C |
| ATOM | 6704 | CG | ARG | E | 170 | 0.216 | 90.900 | -0.870 | 1.00 | 40.70 | B000 | C |
| ATOM | 6705 | CD | ARG | E | 170 | -0.749 | 90.780 | 0.304 | 1.00 | 45.71 | B000 | C |
| ATOM | 6706 | NE | ARG | E | 170 | -2.082 | 91.308 | -0.011 | 1.00 | 51.47 | B000 | N |
| ATOM | 6707 | CZ | ARG | E | 170 | -3.156 | 91.221 | 0.782 | 1.00 | 53.18 | B000 | C |
| ATOM | 6708 | NH1 | ARG | E | 170 | -3.089 | 90.606 | 1.963 | 1.00 | 52.87 | B000 | N1+ |
| ATOM | 6709 | NH2 | ARG | E | 170 | -4.315 | 91.737 | 0.384 | 1.00 | 52.00 | B000 | N |
| ATOM | 6710 | N | SER | E | 171 | 1.194 | 89.177 | -5.414 | 1.00 | 34.01 | B000 | N |
| ATOM | 6711 | CA | SER | E | 171 | 1.334 | 88.153 | -6.435 | 1.00 | 37.47 | B000 | C |
| ATOM | 6712 | C | SER | E | 171 | 2.092 | 88.761 | -7.607 | 1.00 | 36.87 | B000 | C |
| ATOM | 6713 | O | SER | E | 171 | 2.269 | 89.978 | -7.692 | 1.00 | 35.85 | B000 | O |
| ATOM | 6714 | CB | SER | E | 171 | -0.030 | 87.625 | -6.873 | 1.00 | 33.20 | B000 | C |
| ATOM | 6715 | OG | SER | E | 171 | -0.796 | 88.688 | -7.418 | 1.00 | 38.78 | B000 | O |
| ATOM | 6716 | N | GLY | E | 172 | 2.519 | 87.895 | -8.521 | 1.00 | 30.60 | B000 | N |
| ATOM | 6717 | CA | GLY | E | 172 | 3.411 | 88.281 | -9.590 | 1.00 | 32.88 | B000 | C |
| ATOM | 6718 | C | GLY | E | 172 | 2.710 | 88.558 | -10.905 | 1.00 | 33.42 | B000 | C |
| ATOM | 6719 | O | GLY | E | 172 | 1.628 | 88.050 | -11.168 | 1.00 | 38.97 | B000 | O |
| ATOM | 6720 | N | LYS | E | 173 | 3.360 | 89.389 | -11.723 | 1.00 | 34.61 | B000 | N |
| ATOM | 6721 | CA | LYS | E | 173 | 2.968 | 89.700 | -13.090 | 1.00 | 31.95 | B000 | C |
| ATOM | 6722 | C | LYS | E | 173 | 4.217 | 90.055 | -13.875 | 1.00 | 32.50 | B000 | C |
| ATOM | 6723 | O | LYS | E | 173 | 5.168 | 90.618 | -13.327 | 1.00 | 29.30 | B000 | O |
| ATOM | 6724 | CB | LYS | E | 173 | 1.999 | 90.887 | -13.185 | 1.00 | 35.19 | B000 | C |
| ATOM | 6725 | CG | LYS | E | 173 | 0.540 | 90.586 | -12.876 | 1.00 | 36.71 | B000 | C |
| ATOM | 6726 | CD | LYS | E | 173 | -0.272 | 91.847 | -13.150 | 1.00 | 36.28 | B000 | C |
| ATOM | 6727 | CE | LYS | E | 173 | -1.752 | 91.726 | -12.775 | 1.00 | 37.38 | B000 | C |
| ATOM | 6728 | NZ | LYS | E | 173 | -2.515 | 90.874 | -13.720 | 1.00 | 40.13 | B000 | N1+ |
| ATOM | 6729 | N | ALA | E | 174 | 4.199 | 89.739 | -15.165 | 1.00 | 34.25 | B000 | N |
| ATOM | 6730 | CA | ALA | E | 174 | 5.174 | 90.319 | -16.074 | 1.00 | 31.99 | B000 | C |
| ATOM | 6731 | C | ALA | E | 174 | 5.058 | 91.834 | -16.026 | 1.00 | 33.17 | B000 | C |
| ATOM | 6732 | O | ALA | E | 174 | 3.962 | 92.387 | -15.834 | 1.00 | 28.18 | B000 | O |
| ATOM | 6733 | CB | ALA | E | 174 | 4.938 | 89.837 | -17.503 | 1.00 | 26.95 | B000 | C |
| ATOM | 6734 | N | TRP | E | 175 | 6.199 | 92.506 | -16.231 | 1.00 | 27.62 | B000 | N |
| ATOM | 6735 | CA | TRP | E | 175 | 6.257 | 93.962 | -16.080 | 1.00 | 28.95 | B000 | C |
| ATOM | 6736 | C | TRP | E | 175 | 5.178 | 94.667 | -16.903 | 1.00 | 31.61 | B000 | C |
| ATOM | 6737 | O | TRP | E | 175 | 4.487 | 95.560 | -16.403 | 1.00 | 35.29 | B000 | O |
| ATOM | 6738 | CB | TRP | E | 175 | 7.641 | 94.464 | -16.472 | 1.00 | 34.77 | B000 | C |
| ATOM | 6739 | CG | TRP | E | 175 | 7.893 | 95.874 | -16.085 | 1.00 | 35.77 | B000 | C |

| | | | | | | | | | | | | |
|---|---|---|---|---|---|---|---|---|---|---|---|---|
| ATOM | 6740 | CD1 | TRP | E | 175 | 8.505 | 96.308 | -14.950 | 1.00 | 35.08 | B000 | C |
| ATOM | 6741 | CD2 | TRP | E | 175 | 7.574 | 97.046 | -16.846 | 1.00 | 33.62 | B000 | C |
| ATOM | 6742 | NE1 | TRP | E | 175 | 8.582 | 97.688 | -14.946 | 1.00 | 39.22 | B000 | N |
| ATOM | 6743 | CE2 | TRP | E | 175 | 8.021 | 98.164 | -16.099 | 1.00 | 37.24 | B000 | C |
| ATOM | 6744 | CE3 | TRP | E | 175 | 6.965 | 97.259 | -18.086 | 1.00 | 30.92 | B000 | C |
| ATOM | 6745 | CZ2 | TRP | E | 175 | 7.878 | 99.471 | -16.550 | 1.00 | 34.67 | B000 | C |
| ATOM | 6746 | CZ3 | TRP | E | 175 | 6.812 | 98.559 | -18.531 | 1.00 | 41.35 | B000 | C |
| ATOM | 6747 | CH2 | TRP | E | 175 | 7.273 | 99.651 | -17.769 | 1.00 | 41.09 | B000 | C |
| ATOM | 6748 | N | ALA | E | 176 | 5.003 | 94.268 | -18.164 | 1.00 | 31.68 | B000 | N |
| ATOM | 6749 | CA | ALA | E | 176 | 4.017 | 94.938 | -19.011 | 1.00 | 34.92 | B000 | C |
| ATOM | 6750 | C | ALA | E | 176 | 2.598 | 94.731 | -18.504 | 1.00 | 42.84 | B000 | C |
| ATOM | 6751 | O | ALA | E | 176 | 1.760 | 95.625 | -18.637 | 1.00 | 44.50 | B000 | O |
| ATOM | 6752 | CB | ALA | E | 176 | 4.121 | 94.464 | -20.459 | 1.00 | 27.15 | B000 | C |
| ATOM | 6753 | N | ASP | E | 177 | 2.296 | 93.569 | -17.928 | 1.00 | 38.72 | B000 | N |
| ATOM | 6754 | CA | ASP | E | 177 | 0.959 | 93.397 | -17.376 | 1.00 | 38.20 | B000 | C |
| ATOM | 6755 | C | ASP | E | 177 | 0.800 | 94.189 | -16.085 | 1.00 | 37.56 | B000 | C |
| ATOM | 6756 | O | ASP | E | 177 | -0.277 | 94.742 | -15.824 | 1.00 | 35.06 | B000 | O |
| ATOM | 6757 | CB | ASP | E | 177 | 0.658 | 91.915 | -17.144 | 1.00 | 38.76 | B000 | C |
| ATOM | 6758 | CG | ASP | E | 177 | 0.524 | 91.144 | -18.441 | 1.00 | 44.40 | B000 | C |
| ATOM | 6759 | OD1 | ASP | E | 177 | -0.060 | 91.696 | -19.397 | 1.00 | 46.46 | B000 | O |
| ATOM | 6760 | OD2 | ASP | E | 177 | 1.027 | 90.000 | -18.513 | 1.00 | 46.19 | B000 | O1- |
| ATOM | 6761 | N | ALA | E | 178 | 1.851 | 94.231 | -15.255 | 1.00 | 34.06 | B000 | N |
| ATOM | 6762 | CA | ALA | E | 178 | 1.810 | 95.046 | -14.045 | 1.00 | 37.97 | B000 | C |
| ATOM | 6763 | C | ALA | E | 178 | 1.710 | 96.521 | -14.403 | 1.00 | 38.84 | B000 | C |
| ATOM | 6764 | O | ALA | E | 178 | 1.028 | 97.294 | -13.722 | 1.00 | 36.52 | B000 | O |
| ATOM | 6765 | CB | ALA | E | 178 | 3.040 | 94.776 | -13.174 | 1.00 | 36.54 | B000 | C |
| ATOM | 6766 | N | ASP | E | 179 | 2.391 | 96.921 | -15.471 | 1.00 | 38.85 | B000 | N |
| ATOM | 6767 | CA | ASP | E | 179 | 2.284 | 98.283 | -15.969 | 1.00 | 39.27 | B000 | C |
| ATOM | 6768 | C | ASP | E | 179 | 0.840 | 98.610 | -16.344 | 1.00 | 44.49 | B000 | C |
| ATOM | 6769 | O | ASP | E | 179 | 0.287 | 99.630 | -15.911 | 1.00 | 43.34 | B000 | O |
| ATOM | 6770 | CB | ASP | E | 179 | 3.233 | 98.437 | -17.155 | 1.00 | 41.76 | B000 | C |
| ATOM | 6771 | CG | ASP | E | 179 | 3.180 | 99.817 | -17.795 | 1.00 | 53.27 | B000 | C |
| ATOM | 6772 | OD1 | ASP | E | 179 | 3.353 | 100.836 | -17.081 | 1.00 | 46.80 | B000 | O |
| ATOM | 6773 | OD2 | ASP | E | 179 | 2.980 | 99.863 | -19.031 | 1.00 | 46.92 | B000 | O1- |
| ATOM | 6774 | N | ASN | E | 180 | 0.193 | 97.723 | -17.106 | 1.00 | 42.03 | B000 | N |
| ATOM | 6775 | CA | ASN | E | 180 | -1.199 | 97.957 | -17.482 | 1.00 | 44.06 | B000 | C |
| ATOM | 6776 | C | ASN | E | 180 | -2.105 | 97.976 | -16.261 | 1.00 | 47.76 | B000 | C |
| ATOM | 6777 | O | ASN | E | 180 | -3.071 | 98.746 | -16.212 | 1.00 | 47.34 | B000 | O |
| ATOM | 6778 | CB | ASN | E | 180 | -1.687 | 96.905 | -18.488 | 1.00 | 43.06 | B000 | C |
| ATOM | 6779 | CG | ASN | E | 180 | -1.015 | 97.046 | -19.870 | 1.00 | 67.38 | B000 | C |
| ATOM | 6780 | OD1 | ASN | E | 180 | -0.489 | 98.113 | -20.216 | 1.00 | 70.80 | B000 | O |
| ATOM | 6781 | ND2 | ASN | E | 180 | -1.053 | 95.971 | -20.670 | 1.00 | 65.59 | B000 | N |
| ATOM | 6782 | N | TYR | E | 181 | -1.826 | 97.126 | -15.271 | 1.00 | 39.12 | B000 | N |
| ATOM | 6783 | CA | TYR | E | 181 | -2.653 | 97.125 | -14.068 | 1.00 | 43.01 | B000 | C |
| ATOM | 6784 | C | TYR | E | 181 | -2.651 | 98.498 | -13.388 | 1.00 | 42.74 | B000 | C |
| ATOM | 6785 | O | TYR | E | 181 | -3.710 | 99.015 | -13.016 | 1.00 | 42.29 | B000 | O |
| ATOM | 6786 | CB | TYR | E | 181 | -2.191 | 96.021 | -13.109 | 1.00 | 37.06 | B000 | C |
| ATOM | 6787 | CG | TYR | E | 181 | -2.917 | 96.012 | -11.794 | 1.00 | 38.21 | B000 | C |
| ATOM | 6788 | CD1 | TYR | E | 181 | -2.558 | 96.897 | -10.772 | 1.00 | 34.22 | B000 | C |
| ATOM | 6789 | CD2 | TYR | E | 181 | -3.956 | 95.118 | -11.558 | 1.00 | 37.19 | B000 | C |
| ATOM | 6790 | CE1 | TYR | E | 181 | -3.234 | 96.912 | -9.562 | 1.00 | 38.89 | B000 | C |
| ATOM | 6791 | CE2 | TYR | E | 181 | -4.640 | 95.112 | -10.337 | 1.00 | 36.01 | B000 | C |
| ATOM | 6792 | CZ | TYR | E | 181 | -4.276 | 96.010 | -9.345 | 1.00 | 44.74 | B000 | C |
| ATOM | 6793 | OH | TYR | E | 181 | -4.927 | 95.998 | -8.128 | 1.00 | 40.18 | B000 | O |
| ATOM | 6794 | N | CYS | E | 182 | -1.472 | 99.100 | -13.203 | 1.00 | 38.84 | B000 | N |
| ATOM | 6795 | CA | CYS | E | 182 | -1.422 | 100.388 | -12.511 | 1.00 | 45.63 | B000 | C |
| ATOM | 6796 | C | CYS | E | 182 | -2.149 | 101.467 | -13.312 | 1.00 | 47.11 | B000 | C |
| ATOM | 6797 | O | CYS | E | 182 | -2.911 | 102.262 | -12.747 | 1.00 | 44.58 | B000 | O |
| ATOM | 6798 | CB | CYS | E | 182 | 0.031 | 100.804 | -12.239 | 1.00 | 42.50 | B000 | C |
| ATOM | 6799 | SG | CYS | E | 182 | 0.953 | 99.774 | -11.010 | 1.00 | 49.37 | B000 | S |
| ATOM | 6800 | N | ARG | E | 183 | -1.957 | 101.483 | -14.636 | 1.00 | 46.95 | B000 | N |
| ATOM | 6801 | CA | ARG | E | 183 | -2.612 | 102.478 | -15.481 | 1.00 | 44.11 | B000 | C |
| ATOM | 6802 | C | ARG | E | 183 | -4.130 | 102.366 | -15.394 | 1.00 | 50.00 | B000 | C |
| ATOM | 6803 | O | ARG | E | 183 | -4.833 | 103.386 | -15.359 | 1.00 | 44.76 | B000 | O |
| ATOM | 6804 | CB | ARG | E | 183 | -2.113 | 102.348 | -16.926 | 1.00 | 40.71 | B000 | C |
| ATOM | 6805 | CG | ARG | E | 183 | -0.821 | 103.153 | -17.173 | 1.00 | 63.11 | B000 | C |
| ATOM | 6806 | CD | ARG | E | 183 | 0.070 | 102.668 | -18.340 | 1.00 | 66.43 | B000 | C |
| ATOM | 6807 | NE | ARG | E | 183 | 1.428 | 103.228 | -18.202 | 1.00 | 85.06 | B000 | N |
| ATOM | 6808 | CZ | ARG | E | 183 | 2.473 | 102.943 | -18.989 | 1.00 | 82.14 | B000 | C |
| ATOM | 6809 | NH1 | ARG | E | 183 | 2.333 | 102.088 | -19.998 | 1.00 | 86.34 | B000 | N1+ |
| ATOM | 6810 | NH2 | ARG | E | 183 | 3.667 | 103.501 | -18.759 | 1.00 | 56.98 | B000 | N |

| | | | | | | | | | | | | |
|---|---|---|---|---|---|---|---|---|---|---|---|---|
| ATOM | 6811 | N | LEU | E | 184 | -4.656 | 101.136 | -15.327 | 1.00 | 45.91 | B000 | N |
| ATOM | 6812 | CA | LEU | E | 184 | -6.094 | 100.958 | -15.156 | 1.00 | 43.34 | B000 | C |
| ATOM | 6813 | C | LEU | E | 184 | -6.575 | 101.442 | -13.804 | 1.00 | 44.25 | B000 | C |
| ATOM | 6814 | O | LEU | E | 184 | -7.769 | 101.694 | -13.646 | 1.00 | 51.06 | B000 | O |
| ATOM | 6815 | CB | LEU | E | 184 | -6.497 | 99.490 | -15.302 | 1.00 | 47.73 | B000 | C |
| ATOM | 6816 | CG | LEU | E | 184 | -6.471 | 98.840 | -16.680 | 1.00 | 54.62 | B000 | C |
| ATOM | 6817 | CD1 | LEU | E | 184 | -6.890 | 97.380 | -16.546 | 1.00 | 41.74 | B000 | C |
| ATOM | 6818 | CD2 | LEU | E | 184 | -7.353 | 99.606 | -17.678 | 1.00 | 43.16 | B000 | C |
| ATOM | 6819 | N | GLU | E | 185 | -5.685 | 101.579 | -12.827 | 1.00 | 49.85 | B000 | N |
| ATOM | 6820 | CA | GLU | E | 185 | -6.032 | 102.169 | -11.541 | 1.00 | 49.61 | B000 | C |
| ATOM | 6821 | C | GLU | E | 185 | -5.766 | 103.658 | -11.502 | 1.00 | 48.64 | B000 | C |
| ATOM | 6822 | O | GLU | E | 185 | -5.704 | 104.233 | -10.412 | 1.00 | 53.97 | B000 | O |
| ATOM | 6823 | CB | GLU | E | 185 | -5.258 | 101.484 | -10.416 | 1.00 | 52.10 | B000 | C |
| ATOM | 6824 | CG | GLU | E | 185 | -5.516 | 100.002 | -10.355 | 1.00 | 58.31 | B000 | C |
| ATOM | 6825 | CD | GLU | E | 185 | -6.899 | 99.698 | -9.827 | 1.00 | 64.50 | B000 | C |
| ATOM | 6826 | OE1 | GLU | E | 185 | -7.284 | 100.277 | -8.780 | 1.00 | 62.30 | B000 | O |
| ATOM | 6827 | OE2 | GLU | E | 185 | -7.614 | 98.911 | -10.485 | 1.00 | 74.36 | B000 | O1- |
| ATOM | 6828 | N | ASP | E | 186 | -5.571 | 104.286 | -12.662 | 1.00 | 44.48 | B000 | N |
| ATOM | 6829 | CA | ASP | E | 186 | -5.173 | 105.692 | -12.731 | 1.00 | 54.36 | B000 | C |
| ATOM | 6830 | C | ASP | E | 186 | -3.929 | 105.951 | -11.874 | 1.00 | 51.31 | B000 | C |
| ATOM | 6831 | O | ASP | E | 186 | -3.859 | 106.883 | -11.069 | 1.00 | 57.99 | B000 | O |
| ATOM | 6832 | CB | ASP | E | 186 | -6.337 | 106.604 | -12.332 | 1.00 | 56.91 | B000 | C |
| ATOM | 6833 | CG | ASP | E | 186 | -6.105 | 108.048 | -12.733 | 1.00 | 74.54 | B000 | C |
| ATOM | 6834 | OD1 | ASP | E | 186 | -5.482 | 108.279 | -13.798 | 1.00 | 78.01 | B000 | O |
| ATOM | 6835 | OD2 | ASP | E | 186 | -6.541 | 108.948 | -11.982 | 1.00 | 75.17 | B000 | O1- |
| ATOM | 6836 | N | ALA | E | 187 | -2.940 | 105.083 | -12.033 | 1.00 | 50.91 | B000 | N |
| ATOM | 6837 | CA | ALA | E | 187 | -1.713 | 105.165 | -11.265 | 1.00 | 42.37 | B000 | C |
| ATOM | 6838 | C | ALA | E | 187 | -0.593 | 104.718 | -12.183 | 1.00 | 37.72 | B000 | C |
| ATOM | 6839 | O | ALA | E | 187 | -0.810 | 104.469 | -13.372 | 1.00 | 44.89 | B000 | O |
| ATOM | 6840 | CB | ALA | E | 187 | -1.820 | 104.332 | -9.980 | 1.00 | 41.53 | B000 | C |
| ATOM | 6841 | N | HIS | E | 188 | 0.612 | 104.603 | -11.641 | 1.00 | 40.45 | B000 | N |
| ATOM | 6842 | CA | HIS | E | 188 | 1.724 | 104.095 | -12.422 | 1.00 | 40.26 | B000 | C |
| ATOM | 6843 | C | HIS | E | 188 | 2.622 | 103.249 | -11.526 | 1.00 | 42.46 | B000 | C |
| ATOM | 6844 | O | HIS | E | 188 | 2.526 | 103.288 | -10.296 | 1.00 | 40.81 | B000 | O |
| ATOM | 6845 | CB | HIS | E | 188 | 2.499 | 105.237 | -13.079 | 1.00 | 40.08 | B000 | C |
| ATOM | 6846 | CG | HIS | E | 188 | 2.996 | 106.266 | -12.107 | 1.00 | 47.93 | B000 | C |
| ATOM | 6847 | ND1 | HIS | E | 188 | 4.133 | 106.085 | -11.346 | 1.00 | 46.18 | B000 | N |
| ATOM | 6848 | CD2 | HIS | E | 188 | 2.508 | 107.486 | -11.772 | 1.00 | 40.80 | B000 | C |
| ATOM | 6849 | CE1 | HIS | E | 188 | 4.327 | 107.151 | -10.587 | 1.00 | 51.18 | B000 | C |
| ATOM | 6850 | NE2 | HIS | E | 188 | 3.358 | 108.017 | -10.829 | 1.00 | 50.42 | B000 | N |
| ATOM | 6851 | N | LEU | E | 189 | 3.518 | 102.490 | -12.157 | 1.00 | 37.36 | B000 | N |
| ATOM | 6852 | CA | LEU | E | 189 | 4.459 | 101.686 | -11.393 | 1.00 | 38.98 | B000 | C |
| ATOM | 6853 | C | LEU | E | 189 | 5.384 | 102.583 | -10.588 | 1.00 | 38.79 | B000 | C |
| ATOM | 6854 | O | LEU | E | 189 | 5.766 | 103.663 | -11.040 | 1.00 | 44.62 | B000 | O |
| ATOM | 6855 | CB | LEU | E | 189 | 5.264 | 100.782 | -12.324 | 1.00 | 42.15 | B000 | C |
| ATOM | 6856 | CG | LEU | E | 189 | 4.561 | 99.535 | -12.851 | 1.00 | 38.45 | B000 | C |
| ATOM | 6857 | CD1 | LEU | E | 189 | 5.376 | 98.948 | -13.979 | 1.00 | 31.59 | B000 | C |
| ATOM | 6858 | CD2 | LEU | E | 189 | 4.384 | 98.533 | -11.707 | 1.00 | 36.53 | B000 | C |
| ATOM | 6859 | N | VAL | E | 190 | 5.742 | 102.130 | -9.386 | 1.00 | 33.02 | B000 | N |
| ATOM | 6860 | CA | VAL | E | 190 | 6.360 | 103.022 | -8.410 | 1.00 | 37.67 | B000 | C |
| ATOM | 6861 | C | VAL | E | 190 | 7.627 | 103.653 | -8.977 | 1.00 | 41.59 | B000 | C |
| ATOM | 6862 | O | VAL | E | 190 | 8.477 | 102.978 | -9.571 | 1.00 | 39.37 | B000 | O |
| ATOM | 6863 | CB | VAL | E | 190 | 6.626 | 102.274 | -7.093 | 1.00 | 40.50 | B000 | C |
| ATOM | 6864 | CG1 | VAL | E | 190 | 7.570 | 101.068 | -7.292 | 1.00 | 36.01 | B000 | C |
| ATOM | 6865 | CG2 | VAL | E | 190 | 7.216 | 103.229 | -6.056 | 1.00 | 39.66 | B000 | C |
| ATOM | 6866 | N | VAL | E | 191 | 7.751 | 104.967 | -8.792 | 1.00 | 42.38 | B000 | N |
| ATOM | 6867 | CA | VAL | E | 191 | 8.934 | 105.734 | -9.169 | 1.00 | 38.93 | B000 | C |
| ATOM | 6868 | C | VAL | E | 191 | 9.618 | 106.183 | -7.886 | 1.00 | 41.89 | B000 | C |
| ATOM | 6869 | O | VAL | E | 191 | 8.994 | 106.850 | -7.054 | 1.00 | 49.00 | B000 | O |
| ATOM | 6870 | CB | VAL | E | 191 | 8.569 | 106.923 | -10.062 | 1.00 | 39.27 | B000 | C |
| ATOM | 6871 | CG1 | VAL | E | 191 | 9.802 | 107.734 | -10.381 | 1.00 | 41.98 | B000 | C |
| ATOM | 6872 | CG2 | VAL | E | 191 | 7.920 | 106.410 | -11.351 | 1.00 | 37.38 | B000 | C |
| ATOM | 6873 | N | VAL | E | 192 | 10.885 | 105.796 | -7.712 | 1.00 | 36.78 | B000 | N |
| ATOM | 6874 | CA | VAL | E | 192 | 11.606 | 106.006 | -6.456 | 1.00 | 43.13 | B000 | C |
| ATOM | 6875 | C | VAL | E | 192 | 12.466 | 107.255 | -6.601 | 1.00 | 46.90 | B000 | C |
| ATOM | 6876 | O | VAL | E | 192 | 13.429 | 107.274 | -7.375 | 1.00 | 45.02 | B000 | O |
| ATOM | 6877 | CB | VAL | E | 192 | 12.459 | 104.793 | -6.065 | 1.00 | 41.45 | B000 | C |
| ATOM | 6878 | CG1 | VAL | E | 192 | 13.073 | 105.013 | -4.679 | 1.00 | 35.87 | B000 | C |
| ATOM | 6879 | CG2 | VAL | E | 192 | 11.618 | 103.512 | -6.094 | 1.00 | 37.77 | B000 | C |
| ATOM | 6880 | N | THR | E | 193 | 12.117 | 108.311 | -5.870 | 1.00 | 47.21 | B000 | N |
| ATOM | 6881 | CA | THR | E | 193 | 12.800 | 109.589 | -6.012 | 1.00 | 54.54 | B000 | C |

| | | | | | | | | | | | |
|---|---|---|---|---|---|---|---|---|---|---|---|
| ATOM | 6882 | C | THR | E | 193 | 13.689 | 109.963 | -4.828 | 1.00 | 53.96 | B000 C |
| ATOM | 6883 | O | THR | E | 193 | 14.342 | 111.005 | -4.886 | 1.00 | 57.35 | B000 O |
| ATOM | 6884 | CB | THR | E | 193 | 11.775 | 110.701 | -6.279 | 1.00 | 47.81 | B000 C |
| ATOM | 6885 | OG1 | THR | E | 193 | 10.889 | 110.829 | -5.158 | 1.00 | 54.26 | B000 O |
| ATOM | 6886 | CG2 | THR | E | 193 | 10.954 | 110.359 | -7.522 | 1.00 | 49.33 | B000 C |
| ATOM | 6887 | N | SER | E | 194 | 13.765 | 109.142 | -3.779 | 1.00 | 50.25 | B000 N |
| ATOM | 6888 | CA | SER | E | 194 | 14.493 | 109.535 | -2.577 | 1.00 | 47.10 | B000 C |
| ATOM | 6889 | C | SER | E | 194 | 14.737 | 108.321 | -1.701 | 1.00 | 54.45 | B000 C |
| ATOM | 6890 | O | SER | E | 194 | 14.097 | 107.277 | -1.857 | 1.00 | 55.35 | B000 O |
| ATOM | 6891 | CB | SER | E | 194 | 13.727 | 110.596 | -1.779 | 1.00 | 50.58 | B000 C |
| ATOM | 6892 | OG | SER | E | 194 | 12.588 | 110.027 | -1.152 | 1.00 | 52.89 | B000 O |
| ATOM | 6893 | N | TRP | E | 195 | 15.656 | 108.488 | -0.749 | 1.00 | 54.05 | B000 N |
| ATOM | 6894 | CA | TRP | E | 195 | 15.954 | 107.418 | 0.196 | 1.00 | 53.90 | B000 C |
| ATOM | 6895 | C | TRP | E | 195 | 14.742 | 107.048 | 1.047 | 1.00 | 56.80 | B000 C |
| ATOM | 6896 | O | TRP | E | 195 | 14.512 | 105.862 | 1.323 | 1.00 | 53.39 | B000 O |
| ATOM | 6897 | CB | TRP | E | 195 | 17.124 | 107.822 | 1.086 | 1.00 | 52.43 | B000 C |
| ATOM | 6898 | CG | TRP | E | 195 | 18.407 | 107.331 | 0.552 | 1.00 | 62.39 | B000 C |
| ATOM | 6899 | CD1 | TRP | E | 195 | 19.444 | 108.092 | 0.068 | 1.00 | 63.42 | B000 C |
| ATOM | 6900 | CD2 | TRP | E | 195 | 18.804 | 105.965 | 0.402 | 1.00 | 65.61 | B000 C |
| ATOM | 6901 | NE1 | TRP | E | 195 | 20.464 | 107.278 | -0.362 | 1.00 | 65.18 | B000 N |
| ATOM | 6902 | CE2 | TRP | E | 195 | 20.100 | 105.969 | -0.170 | 1.00 | 69.72 | B000 C |
| ATOM | 6903 | CE3 | TRP | E | 195 | 18.199 | 104.739 | 0.703 | 1.00 | 59.71 | B000 C |
| ATOM | 6904 | CZ2 | TRP | E | 195 | 20.797 | 104.792 | -0.447 | 1.00 | 59.79 | B000 C |
| ATOM | 6905 | CZ3 | TRP | E | 195 | 18.890 | 103.571 | 0.425 | 1.00 | 54.31 | B000 C |
| ATOM | 6906 | CH2 | TRP | E | 195 | 20.174 | 103.605 | -0.142 | 1.00 | 59.74 | B000 C |
| ATOM | 6907 | N | GLU | E | 196 | 13.961 | 108.038 | 1.487 | 1.00 | 55.10 | B000 N |
| ATOM | 6908 | CA | GLU | E | 196 | 12.781 | 107.723 | 2.292 | 1.00 | 58.29 | B000 C |
| ATOM | 6909 | C | GLU | E | 196 | 11.786 | 106.895 | 1.505 | 1.00 | 53.21 | B000 C |
| ATOM | 6910 | O | GLU | E | 196 | 11.299 | 105.874 | 1.999 | 1.00 | 56.81 | B000 O |
| ATOM | 6911 | CB | GLU | E | 196 | 12.127 | 108.987 | 2.849 | 1.00 | 67.43 | B000 C |
| ATOM | 6912 | CG | GLU | E | 196 | 12.825 | 109.494 | 4.092 | 1.00 | 75.36 | B000 C |
| ATOM | 6913 | CD | GLU | E | 196 | 14.139 | 110.132 | 3.758 | 1.00 | 90.49 | B000 C |
| ATOM | 6914 | OE1 | GLU | E | 196 | 14.259 | 110.606 | 2.610 | 1.00 | 89.70 | B000 O |
| ATOM | 6915 | OE2 | GLU | E | 196 | 15.055 | 110.128 | 4.613 | 1.00 | 94.74 | B000 O1- |
| ATOM | 6916 | N | GLU | E | 197 | 11.460 | 107.326 | 0.281 | 1.00 | 51.77 | B000 N |
| ATOM | 6917 | CA | GLU | E | 197 | 10.589 | 106.525 | -0.570 | 1.00 | 52.50 | B000 C |
| ATOM | 6918 | C | GLU | E | 197 | 11.155 | 105.116 | -0.740 | 1.00 | 51.92 | B000 C |
| ATOM | 6919 | O | GLU | E | 197 | 10.436 | 104.125 | -0.567 | 1.00 | 48.95 | B000 O |
| ATOM | 6920 | CB | GLU | E | 197 | 10.376 | 107.217 | -1.920 | 1.00 | 44.93 | B000 C |
| ATOM | 6921 | CG | GLU | E | 197 | 9.163 | 106.692 | -2.685 | 1.00 | 46.53 | B000 C |
| ATOM | 6922 | CD | GLU | E | 197 | 8.827 | 107.482 | -3.949 | 1.00 | 48.66 | B000 C |
| ATOM | 6923 | OE1 | GLU | E | 197 | 9.651 | 108.313 | -4.402 | 1.00 | 48.86 | B000 O |
| ATOM | 6924 | OE2 | GLU | E | 197 | 7.727 | 107.257 | -4.507 | 1.00 | 50.40 | B000 O1- |
| ATOM | 6925 | N | GLN | E | 198 | 12.462 | 105.006 | -1.006 | 1.00 | 45.93 | B000 N |
| ATOM | 6926 | CA | GLN | E | 198 | 13.088 | 103.692 | -1.102 | 1.00 | 44.41 | B000 C |
| ATOM | 6927 | C | GLN | E | 198 | 12.894 | 102.888 | 0.183 | 1.00 | 52.88 | B000 C |
| ATOM | 6928 | O | GLN | E | 198 | 12.542 | 101.701 | 0.145 | 1.00 | 50.74 | B000 O |
| ATOM | 6929 | CB | GLN | E | 198 | 14.572 | 103.831 | -1.416 | 1.00 | 39.60 | B000 C |
| ATOM | 6930 | CG | GLN | E | 198 | 15.367 | 102.576 | -1.148 | 1.00 | 36.79 | B000 C |
| ATOM | 6931 | CD | GLN | E | 198 | 15.210 | 101.541 | -2.254 | 1.00 | 42.68 | B000 C |
| ATOM | 6932 | OE1 | GLN | E | 198 | 14.942 | 101.881 | -3.399 | 1.00 | 38.01 | B000 O |
| ATOM | 6933 | NE2 | GLN | E | 198 | 15.359 | 100.273 | -1.906 | 1.00 | 40.53 | B000 N |
| ATOM | 6934 | N | LYS | E | 199 | 13.135 | 103.512 | 1.337 | 1.00 | 51.74 | B000 N |
| ATOM | 6935 | CA | LYS | E | 199 | 13.004 | 102.770 | 2.587 | 1.00 | 50.49 | B000 C |
| ATOM | 6936 | C | LYS | E | 199 | 11.544 | 102.452 | 2.890 | 1.00 | 51.58 | B000 C |
| ATOM | 6937 | O | LYS | E | 199 | 11.242 | 101.379 | 3.427 | 1.00 | 48.97 | B000 O |
| ATOM | 6938 | CB | LYS | E | 199 | 13.670 | 103.539 | 3.731 | 1.00 | 53.77 | B000 C |
| ATOM | 6939 | CG | LYS | E | 199 | 15.157 | 103.755 | 3.500 | 1.00 | 55.44 | B000 C |
| ATOM | 6940 | CD | LYS | E | 199 | 15.992 | 103.547 | 4.755 | 1.00 | 75.31 | B000 C |
| ATOM | 6941 | CE | LYS | E | 199 | 17.390 | 104.165 | 4.583 | 1.00 | 85.03 | B000 C |
| ATOM | 6942 | NZ | LYS | E | 199 | 18.151 | 104.301 | 5.862 | 1.00 | 83.45 | B000 N1+ |
| ATOM | 6943 | N | PHE | E | 200 | 10.631 | 103.358 | 2.531 | 1.00 | 48.81 | B000 N |
| ATOM | 6944 | CA | PHE | E | 200 | 9.204 | 103.098 | 2.691 | 1.00 | 48.98 | B000 C |
| ATOM | 6945 | C | PHE | E | 200 | 8.773 | 101.845 | 1.931 | 1.00 | 55.28 | B000 C |
| ATOM | 6946 | O | PHE | E | 200 | 8.032 | 101.010 | 2.465 | 1.00 | 54.84 | B000 O |
| ATOM | 6947 | CB | PHE | E | 200 | 8.406 | 104.312 | 2.224 | 1.00 | 46.91 | B000 C |
| ATOM | 6948 | CG | PHE | E | 200 | 6.965 | 104.034 | 2.006 | 1.00 | 50.71 | B000 C |
| ATOM | 6949 | CD1 | PHE | E | 200 | 6.112 | 103.847 | 3.085 | 1.00 | 53.71 | B000 C |
| ATOM | 6950 | CD2 | PHE | E | 200 | 6.449 | 103.965 | 0.722 | 1.00 | 51.62 | B000 C |
| ATOM | 6951 | CE1 | PHE | E | 200 | 4.756 | 103.588 | 2.891 | 1.00 | 52.00 | B000 C |
| ATOM | 6952 | CE2 | PHE | E | 200 | 5.097 | 103.710 | 0.512 | 1.00 | 50.54 | B000 C |

| | | | | | | | | | | | | |
|---|---|---|---|---|---|---|---|---|---|---|---|---|
| ATOM | 6953 | CZ | PHE | E | 200 | 4.248 | 103.518 | 1.605 | 1.00 | 54.40 | B000 | C |
| ATOM | 6954 | N | VAL | E | 201 | 9.229 | 101.697 | 0.680 | 1.00 | 51.49 | B000 | N |
| ATOM | 6955 | CA | VAL | E | 201 | 8.826 | 100.553 | -0.137 | 1.00 | 48.07 | B000 | C |
| ATOM | 6956 | C | VAL | E | 201 | 9.420 | 99.254 | 0.413 | 1.00 | 49.06 | B000 | C |
| ATOM | 6957 | O | VAL | E | 201 | 8.706 | 98.253 | 0.569 | 1.00 | 46.98 | B000 | O |
| ATOM | 6958 | CB | VAL | E | 201 | 9.208 | 100.788 | -1.611 | 1.00 | 46.66 | B000 | C |
| ATOM | 6959 | CG1 | VAL | E | 201 | 9.067 | 99.495 | -2.434 | 1.00 | 41.41 | B000 | C |
| ATOM | 6960 | CG2 | VAL | E | 201 | 8.349 | 101.902 | -2.212 | 1.00 | 37.70 | B000 | C |
| ATOM | 6961 | N | GLN | E | 202 | 10.728 | 99.252 | 0.723 | 1.00 | 49.32 | B000 | N |
| ATOM | 6962 | CA | GLN | E | 202 | 11.380 | 98.063 | 1.284 | 1.00 | 50.74 | B000 | C |
| ATOM | 6963 | C | GLN | E | 202 | 10.622 | 97.528 | 2.481 | 1.00 | 51.55 | B000 | C |
| ATOM | 6964 | O | GLN | E | 202 | 10.464 | 96.310 | 2.640 | 1.00 | 56.45 | B000 | O |
| ATOM | 6965 | CB | GLN | E | 202 | 12.803 | 98.371 | 1.749 | 1.00 | 47.47 | B000 | C |
| ATOM | 6966 | CG | GLN | E | 202 | 13.739 | 98.831 | 0.710 | 1.00 | 55.69 | B000 | C |
| ATOM | 6967 | CD | GLN | E | 202 | 15.139 | 98.987 | 1.254 | 1.00 | 56.47 | B000 | C |
| ATOM | 6968 | OE1 | GLN | E | 202 | 15.907 | 99.831 | 0.790 | 1.00 | 57.62 | B000 | O |
| ATOM | 6969 | NE2 | GLN | E | 202 | 15.490 | 98.154 | 2.219 | 1.00 | 53.11 | B000 | N |
| ATOM | 6970 | N | HIS | E | 203 | 10.189 | 98.433 | 3.360 | 1.00 | 51.67 | B000 | N |
| ATOM | 6971 | CA | HIS | E | 203 | 9.452 | 98.030 | 4.546 | 1.00 | 53.23 | B000 | C |
| ATOM | 6972 | C | HIS | E | 203 | 8.230 | 97.215 | 4.173 | 1.00 | 58.75 | B000 | C |
| ATOM | 6973 | O | HIS | E | 203 | 7.901 | 96.227 | 4.840 | 1.00 | 59.40 | B000 | O |
| ATOM | 6974 | CB | HIS | E | 203 | 9.043 | 99.256 | 5.346 | 1.00 | 52.17 | B000 | C |
| ATOM | 6975 | CG | HIS | E | 203 | 8.280 | 98.924 | 6.587 | 1.00 | 63.88 | B000 | C |
| ATOM | 6976 | ND1 | HIS | E | 203 | 6.924 | 99.142 | 6.710 | 1.00 | 66.92 | B000 | N |
| ATOM | 6977 | CD2 | HIS | E | 203 | 8.681 | 98.359 | 7.750 | 1.00 | 59.72 | B000 | C |
| ATOM | 6978 | CE1 | HIS | E | 203 | 6.525 | 98.741 | 7.904 | 1.00 | 64.58 | B000 | C |
| ATOM | 6979 | NE2 | HIS | E | 203 | 7.572 | 98.265 | 8.555 | 1.00 | 67.38 | B000 | N |
| ATOM | 6980 | N | HIS | E | 204 | 7.548 | 97.609 | 3.105 | 1.00 | 51.87 | B000 | N |
| ATOM | 6981 | CA | HIS | E | 204 | 6.320 | 96.930 | 2.742 | 1.00 | 47.42 | B000 | C |
| ATOM | 6982 | C | HIS | E | 204 | 6.528 | 95.712 | 1.851 | 1.00 | 45.87 | B000 | C |
| ATOM | 6983 | O | HIS | E | 204 | 5.734 | 94.781 | 1.934 | 1.00 | 46.13 | B000 | O |
| ATOM | 6984 | CB | HIS | E | 204 | 5.375 | 97.925 | 2.086 | 1.00 | 43.64 | B000 | C |
| ATOM | 6985 | CG | HIS | E | 204 | 4.761 | 98.864 | 3.071 | 1.00 | 55.52 | B000 | C |
| ATOM | 6986 | ND1 | HIS | E | 204 | 5.277 | 100.116 | 3.331 | 1.00 | 62.43 | B000 | N |
| ATOM | 6987 | CD2 | HIS | E | 204 | 3.719 | 98.703 | 3.917 | 1.00 | 51.63 | B000 | C |
| ATOM | 6988 | CE1 | HIS | E | 204 | 4.551 | 100.702 | 4.265 | 1.00 | 56.76 | B000 | C |
| ATOM | 6989 | NE2 | HIS | E | 204 | 3.598 | 99.867 | 4.634 | 1.00 | 58.19 | B000 | N |
| ATOM | 6990 | N | ILE | E | 205 | 7.554 | 95.673 | 0.998 | 1.00 | 45.67 | B000 | N |
| ATOM | 6991 | CA | ILE | E | 205 | 7.676 | 94.532 | 0.097 | 1.00 | 43.17 | B000 | C |
| ATOM | 6992 | C | ILE | E | 205 | 8.408 | 93.363 | 0.749 | 1.00 | 43.69 | B000 | C |
| ATOM | 6993 | O | ILE | E | 205 | 8.193 | 92.215 | 0.356 | 1.00 | 42.25 | B000 | O |
| ATOM | 6994 | CB | ILE | E | 205 | 8.346 | 94.913 | -1.235 | 1.00 | 39.71 | B000 | C |
| ATOM | 6995 | CG1 | ILE | E | 205 | 9.791 | 95.341 | -1.023 | 1.00 | 34.62 | B000 | C |
| ATOM | 6996 | CG2 | ILE | E | 205 | 7.572 | 96.011 | -1.954 | 1.00 | 32.37 | B000 | C |
| ATOM | 6997 | CD1 | ILE | E | 205 | 10.470 | 95.665 | -2.317 | 1.00 | 33.57 | B000 | C |
| ATOM | 6998 | N | GLY | E | 206 | 9.273 | 93.616 | 1.728 | 1.00 | 45.56 | B000 | N |
| ATOM | 6999 | CA | GLY | E | 206 | 10.038 | 92.555 | 2.352 | 1.00 | 38.77 | B000 | C |
| ATOM | 7000 | C | GLY | E | 206 | 11.058 | 91.943 | 1.409 | 1.00 | 45.53 | B000 | C |
| ATOM | 7001 | O | GLY | E | 206 | 11.640 | 92.616 | 0.551 | 1.00 | 53.68 | B000 | O |
| ATOM | 7002 | N | PRO | E | 207 | 11.268 | 90.646 | 1.525 | 1.00 | 41.49 | B000 | N |
| ATOM | 7003 | CA | PRO | E | 207 | 12.312 | 89.995 | 0.720 | 1.00 | 48.79 | B000 | C |
| ATOM | 7004 | C | PRO | E | 207 | 11.816 | 89.452 | -0.616 | 1.00 | 39.32 | B000 | C |
| ATOM | 7005 | O | PRO | E | 207 | 12.233 | 88.359 | -0.999 | 1.00 | 49.37 | B000 | O |
| ATOM | 7006 | CB | PRO | E | 207 | 12.736 | 88.837 | 1.624 | 1.00 | 42.37 | B000 | C |
| ATOM | 7007 | CG | PRO | E | 207 | 11.386 | 88.411 | 2.205 | 1.00 | 39.72 | B000 | C |
| ATOM | 7008 | CD | PRO | E | 207 | 10.597 | 89.687 | 2.422 | 1.00 | 38.72 | B000 | C |
| ATOM | 7009 | N | VAL | E | 208 | 10.949 | 90.169 | -1.333 | 1.00 | 40.46 | B000 | N |
| ATOM | 7010 | CA | VAL | E | 208 | 10.315 | 89.659 | -2.546 | 1.00 | 37.68 | B000 | C |
| ATOM | 7011 | C | VAL | E | 208 | 10.706 | 90.542 | -3.729 | 1.00 | 38.16 | B000 | C |
| ATOM | 7012 | O | VAL | E | 208 | 10.561 | 91.767 | -3.665 | 1.00 | 39.77 | B000 | O |
| ATOM | 7013 | CB | VAL | E | 208 | 8.785 | 89.601 | -2.383 | 1.00 | 40.03 | B000 | C |
| ATOM | 7014 | CG1 | VAL | E | 208 | 8.133 | 88.976 | -3.608 | 1.00 | 32.03 | B000 | C |
| ATOM | 7015 | CG2 | VAL | E | 208 | 8.427 | 88.819 | -1.106 | 1.00 | 41.62 | B000 | C |
| ATOM | 7016 | N | ASN | E | 209 | 11.186 | 89.917 | -4.812 | 1.00 | 35.17 | B000 | N |
| ATOM | 7017 | CA | ASN | E | 209 | 11.506 | 90.657 | -6.028 | 1.00 | 30.25 | B000 | C |
| ATOM | 7018 | C | ASN | E | 209 | 10.268 | 91.389 | -6.522 | 1.00 | 39.60 | B000 | C |
| ATOM | 7019 | O | ASN | E | 209 | 9.189 | 90.792 | -6.646 | 1.00 | 35.43 | B000 | O |
| ATOM | 7020 | CB | ASN | E | 209 | 12.020 | 89.710 | -7.105 | 1.00 | 33.04 | B000 | C |
| ATOM | 7021 | CG | ASN | E | 209 | 13.430 | 89.196 | -6.817 | 1.00 | 37.45 | B000 | C |
| ATOM | 7022 | OD1 | ASN | E | 209 | 14.282 | 89.914 | -6.286 | 1.00 | 39.88 | B000 | O |
| ATOM | 7023 | ND2 | ASN | E | 209 | 13.676 | 87.948 | -7.166 | 1.00 | 34.14 | B000 | N |

| | | | | | | | | | | | | |
|---|---|---|---|---|---|---|---|---|---|---|---|---|
| ATOM | 7024 | N | THR | E | 210 | 10.417 | 92.686 | -6.801 | 1.00 | 32.18 | B000 | N |
| ATOM | 7025 | CA | THR | E | 210 | 9.258 | 93.553 | -6.998 | 1.00 | 34.27 | B000 | C |
| ATOM | 7026 | C | THR | E | 210 | 9.571 | 94.589 | -8.068 | 1.00 | 34.48 | B000 | C |
| ATOM | 7027 | O | THR | E | 210 | 10.533 | 95.349 | -7.932 | 1.00 | 31.68 | B000 | O |
| ATOM | 7028 | CB | THR | E | 210 | 8.864 | 94.225 | -5.667 | 1.00 | 33.37 | B000 | C |
| ATOM | 7029 | OG1 | THR | E | 210 | 8.661 | 93.210 | -4.675 | 1.00 | 35.38 | B000 | O |
| ATOM | 7030 | CG2 | THR | E | 210 | 7.604 | 95.081 | -5.789 | 1.00 | 26.61 | B000 | C |
| ATOM | 7031 | N | TRP | E | 211 | 8.743 | 94.626 | -9.112 | 1.00 | 29.97 | B000 | N |
| ATOM | 7032 | CA | TRP | E | 211 | 8.933 | 95.562 | -10.208 | 1.00 | 27.14 | B000 | C |
| ATOM | 7033 | C | TRP | E | 211 | 8.801 | 97.006 | -9.731 | 1.00 | 35.31 | B000 | C |
| ATOM | 7034 | O | TRP | E | 211 | 7.981 | 97.316 | -8.863 | 1.00 | 35.67 | B000 | O |
| ATOM | 7035 | CB | TRP | E | 211 | 7.887 | 95.312 | -11.297 | 1.00 | 30.90 | B000 | C |
| ATOM | 7036 | CG | TRP | E | 211 | 8.066 | 94.071 | -12.124 | 1.00 | 34.50 | B000 | C |
| ATOM | 7037 | CD1 | TRP | E | 211 | 7.104 | 93.138 | -12.433 | 1.00 | 29.56 | B000 | C |
| ATOM | 7038 | CD2 | TRP | E | 211 | 9.267 | 93.640 | -12.788 | 1.00 | 34.63 | B000 | C |
| ATOM | 7039 | NE1 | TRP | E | 211 | 7.630 | 92.160 | -13.254 | 1.00 | 28.60 | B000 | N |
| ATOM | 7040 | CE2 | TRP | E | 211 | 8.955 | 92.439 | -13.482 | 1.00 | 35.28 | B000 | C |
| ATOM | 7041 | CE3 | TRP | E | 211 | 10.575 | 94.146 | -12.860 | 1.00 | 35.02 | B000 | C |
| ATOM | 7042 | CZ2 | TRP | E | 211 | 9.904 | 91.741 | -14.231 | 1.00 | 29.26 | B000 | C |
| ATOM | 7043 | CZ3 | TRP | E | 211 | 11.518 | 93.452 | -13.602 | 1.00 | 36.35 | B000 | C |
| ATOM | 7044 | CH2 | TRP | E | 211 | 11.177 | 92.251 | -14.275 | 1.00 | 30.95 | B000 | C |
| ATOM | 7045 | N | MET | E | 212 | 9.605 | 97.895 | -10.316 | 1.00 | 32.55 | B000 | N |
| ATOM | 7046 | CA | MET | E | 212 | 9.380 | 99.331 | -10.239 | 1.00 | 29.40 | B000 | C |
| ATOM | 7047 | C | MET | E | 212 | 9.191 | 99.869 | -11.652 | 1.00 | 37.49 | B000 | C |
| ATOM | 7048 | O | MET | E | 212 | 9.421 | 99.163 | -12.641 | 1.00 | 33.69 | B000 | O |
| ATOM | 7049 | CB | MET | E | 212 | 10.532 | 100.045 | -9.538 | 1.00 | 32.77 | B000 | C |
| ATOM | 7050 | CG | MET | E | 212 | 11.802 | 100.197 | -10.374 | 1.00 | 37.02 | B000 | C |
| ATOM | 7051 | SD | MET | E | 212 | 13.201 | 100.683 | -9.319 | 1.00 | 36.83 | B000 | S |
| ATOM | 7052 | CE | MET | E | 212 | 13.620 | 99.156 | -8.467 | 1.00 | 32.94 | B000 | C |
| ATOM | 7053 | N | GLY | E | 213 | 8.730 | 101.124 | -11.741 | 1.00 | 37.19 | B000 | N |
| ATOM | 7054 | CA | GLY | E | 213 | 8.440 | 101.761 | -13.019 | 1.00 | 32.81 | B000 | C |
| ATOM | 7055 | C | GLY | E | 213 | 9.662 | 102.269 | -13.761 | 1.00 | 40.32 | B000 | C |
| ATOM | 7056 | O | GLY | E | 213 | 9.719 | 103.432 | -14.187 | 1.00 | 38.40 | B000 | O |
| ATOM | 7057 | N | LEU | E | 214 | 10.634 | 101.380 | -13.941 | 1.00 | 34.17 | B000 | N |
| ATOM | 7058 | CA | LEU | E | 214 | 11.934 | 101.714 | -14.503 | 1.00 | 39.27 | B000 | C |
| ATOM | 7059 | C | LEU | E | 214 | 12.321 | 100.610 | -15.473 | 1.00 | 39.76 | B000 | C |
| ATOM | 7060 | O | LEU | E | 214 | 12.335 | 99.431 | -15.100 | 1.00 | 41.29 | B000 | O |
| ATOM | 7061 | CB | LEU | E | 214 | 12.988 | 101.877 | -13.395 | 1.00 | 36.55 | B000 | C |
| ATOM | 7062 | CG | LEU | E | 214 | 14.450 | 102.064 | -13.797 | 1.00 | 42.98 | B000 | C |
| ATOM | 7063 | CD1 | LEU | E | 214 | 14.624 | 103.269 | -14.744 | 1.00 | 38.04 | B000 | C |
| ATOM | 7064 | CD2 | LEU | E | 214 | 15.305 | 102.224 | -12.529 | 1.00 | 37.75 | B000 | C |
| ATOM | 7065 | N | HIS | E | 215 | 12.601 | 100.987 | -16.718 | 1.00 | 36.19 | B000 | N |
| ATOM | 7066 | CA | HIS | E | 215 | 12.875 | 100.024 | -17.774 | 1.00 | 39.33 | B000 | C |
| ATOM | 7067 | C | HIS | E | 215 | 13.746 | 100.691 | -18.829 | 1.00 | 41.39 | B000 | C |
| ATOM | 7068 | O | HIS | E | 215 | 13.843 | 101.921 | -18.899 | 1.00 | 36.50 | B000 | O |
| ATOM | 7069 | CB | HIS | E | 215 | 11.588 | 99.528 | -18.420 | 1.00 | 37.31 | B000 | C |
| ATOM | 7070 | CG | HIS | E | 215 | 10.877 | 100.605 | -19.156 | 1.00 | 43.54 | B000 | C |
| ATOM | 7071 | ND1 | HIS | E | 215 | 10.975 | 100.759 | -20.521 | 1.00 | 59.96 | B000 | N |
| ATOM | 7072 | CD2 | HIS | E | 215 | 10.149 | 101.654 | -18.708 | 1.00 | 43.07 | B000 | C |
| ATOM | 7073 | CE1 | HIS | E | 215 | 10.292 | 101.830 | -20.888 | 1.00 | 55.73 | B000 | C |
| ATOM | 7074 | NE2 | HIS | E | 215 | 9.780 | 102.387 | -19.807 | 1.00 | 46.66 | B000 | N |
| ATOM | 7075 | N | ASP | E | 216 | 14.341 | 99.858 | -19.680 | 1.00 | 35.15 | B000 | N |
| ATOM | 7076 | CA | ASP | E | 216 | 15.301 | 100.290 | -20.686 | 1.00 | 39.15 | B000 | C |
| ATOM | 7077 | C | ASP | E | 216 | 14.788 | 99.998 | -22.085 | 1.00 | 42.63 | B000 | C |
| ATOM | 7078 | O | ASP | E | 216 | 15.571 | 99.773 | -23.004 | 1.00 | 42.03 | B000 | O |
| ATOM | 7079 | CB | ASP | E | 216 | 16.648 | 99.600 | -20.467 | 1.00 | 41.75 | B000 | C |
| ATOM | 7080 | CG | ASP | E | 216 | 16.632 | 98.097 | -20.849 | 1.00 | 44.42 | B000 | C |
| ATOM | 7081 | OD1 | ASP | E | 216 | 15.551 | 97.505 | -21.130 | 1.00 | 37.16 | B000 | O |
| ATOM | 7082 | OD2 | ASP | E | 216 | 17.732 | 97.502 | -20.848 | 1.00 | 43.61 | B000 | O1- |
| ATOM | 7083 | N | GLN | E | 217 | 13.476 | 99.947 | -22.253 | 1.00 | 46.29 | B000 | N |
| ATOM | 7084 | CA | GLN | E | 217 | 12.991 | 99.214 | -23.411 | 1.00 | 59.57 | B000 | C |
| ATOM | 7085 | C | GLN | E | 217 | 13.208 | 99.957 | -24.721 | 1.00 | 65.22 | B000 | C |
| ATOM | 7086 | O | GLN | E | 217 | 13.083 | 99.331 | -25.778 | 1.00 | 73.01 | B000 | O |
| ATOM | 7087 | CB | GLN | E | 217 | 11.516 | 98.820 | -23.200 | 1.00 | 62.75 | B000 | C |
| ATOM | 7088 | CG | GLN | E | 217 | 11.375 | 97.683 | -22.125 | 1.00 | 61.06 | B000 | C |
| ATOM | 7089 | CD | GLN | E | 217 | 9.984 | 97.034 | -22.070 | 1.00 | 67.45 | B000 | C |
| ATOM | 7090 | OE1 | GLN | E | 217 | 9.134 | 97.409 | -21.253 | 1.00 | 58.29 | B000 | O |
| ATOM | 7091 | NE2 | GLN | E | 217 | 9.759 | 96.044 | -22.936 | 1.00 | 73.72 | B000 | N |
| ATOM | 7092 | N | ASN | E | 218 | 13.622 | 101.226 | -24.688 | 1.00 | 54.85 | B000 | N |
| ATOM | 7093 | CA | ASN | E | 218 | 14.065 | 101.896 | -25.900 | 1.00 | 56.88 | B000 | C |
| ATOM | 7094 | C | ASN | E | 218 | 15.579 | 101.999 | -25.997 | 1.00 | 63.85 | B000 | C |

| | | | | | | | | | | | | |
|---|---|---|---|---|---|---|---|---|---|---|---|---|
| ATOM | 7095 | O | ASN | E | 218 | 16.099 | 102.476 | -27.010 | 1.00 | 65.96 | B000 | O |
| ATOM | 7096 | CB | ASN | E | 218 | 13.440 | 103.283 | -25.982 | 1.00 | 68.65 | B000 | C |
| ATOM | 7097 | CG | ASN | E | 218 | 11.988 | 103.230 | -26.379 | 1.00 | 76.18 | B000 | C |
| ATOM | 7098 | OD1 | ASN | E | 218 | 11.117 | 103.732 | -25.663 | 1.00 | 80.28 | B000 | O |
| ATOM | 7099 | ND2 | ASN | E | 218 | 11.710 | 102.603 | -27.524 | 1.00 | 70.77 | B000 | N |
| ATOM | 7100 | N | GLY | E | 219 | 16.294 | 101.546 | -24.980 | 1.00 | 55.99 | B000 | N |
| ATOM | 7101 | CA | GLY | E | 219 | 17.721 | 101.702 | -24.908 | 1.00 | 47.24 | B000 | C |
| ATOM | 7102 | C | GLY | E | 219 | 18.086 | 102.395 | -23.615 | 1.00 | 46.37 | B000 | C |
| ATOM | 7103 | O | GLY | E | 219 | 18.657 | 101.797 | -22.697 | 1.00 | 45.37 | B000 | O |
| ATOM | 7104 | N | PRO | E | 220 | 17.760 | 103.679 | -23.515 | 1.00 | 47.23 | B000 | N |
| ATOM | 7105 | CA | PRO | E | 220 | 18.027 | 104.396 | -22.266 | 1.00 | 48.53 | B000 | C |
| ATOM | 7106 | C | PRO | E | 220 | 17.055 | 103.993 | -21.169 | 1.00 | 44.15 | B000 | C |
| ATOM | 7107 | O | PRO | E | 220 | 15.875 | 103.736 | -21.413 | 1.00 | 47.26 | B000 | O |
| ATOM | 7108 | CB | PRO | E | 220 | 17.875 | 105.872 | -22.658 | 1.00 | 43.34 | B000 | C |
| ATOM | 7109 | CG | PRO | E | 220 | 17.055 | 105.868 | -23.864 | 1.00 | 50.66 | B000 | C |
| ATOM | 7110 | CD | PRO | E | 220 | 17.359 | 104.593 | -24.598 | 1.00 | 46.49 | B000 | C |
| ATOM | 7111 | N | TRP | E | 221 | 17.592 | 103.878 | -19.961 | 1.00 | 41.42 | B000 | N |
| ATOM | 7112 | CA | TRP | E | 221 | 16.767 | 103.695 | -18.783 | 1.00 | 38.08 | B000 | C |
| ATOM | 7113 | C | TRP | E | 221 | 15.871 | 104.916 | -18.561 | 1.00 | 43.21 | B000 | C |
| ATOM | 7114 | O | TRP | E | 221 | 16.325 | 106.062 | -18.630 | 1.00 | 45.08 | B000 | O |
| ATOM | 7115 | CB | TRP | E | 221 | 17.672 | 103.446 | -17.577 | 1.00 | 37.90 | B000 | C |
| ATOM | 7116 | CG | TRP | E | 221 | 18.256 | 102.057 | -17.559 | 1.00 | 35.77 | B000 | C |
| ATOM | 7117 | CD1 | TRP | E | 221 | 19.520 | 101.683 | -17.939 | 1.00 | 39.29 | B000 | C |
| ATOM | 7118 | CD2 | TRP | E | 221 | 17.586 | 100.853 | -17.157 | 1.00 | 36.68 | B000 | C |
| ATOM | 7119 | NE1 | TRP | E | 221 | 19.676 | 100.323 | -17.789 | 1.00 | 39.97 | B000 | N |
| ATOM | 7120 | CE2 | TRP | E | 221 | 18.501 | 99.790 | -17.318 | 1.00 | 39.47 | B000 | C |
| ATOM | 7121 | CE3 | TRP | E | 221 | 16.296 | 100.571 | -16.676 | 1.00 | 37.85 | B000 | C |
| ATOM | 7122 | CZ2 | TRP | E | 221 | 18.176 | 98.469 | -16.997 | 1.00 | 34.61 | B000 | C |
| ATOM | 7123 | CZ3 | TRP | E | 221 | 15.970 | 99.246 | -16.364 | 1.00 | 38.91 | B000 | C |
| ATOM | 7124 | CH2 | TRP | E | 221 | 16.909 | 98.220 | -16.523 | 1.00 | 36.77 | B000 | C |
| ATOM | 7125 | N | LYS | E | 222 | 14.591 | 104.666 | -18.298 | 1.00 | 42.42 | B000 | N |
| ATOM | 7126 | CA | LYS | E | 222 | 13.600 | 105.715 | -18.110 | 1.00 | 45.91 | B000 | C |
| ATOM | 7127 | C | LYS | E | 222 | 12.648 | 105.317 | -16.994 | 1.00 | 49.90 | B000 | C |
| ATOM | 7128 | O | LYS | E | 222 | 12.383 | 104.131 | -16.772 | 1.00 | 45.80 | B000 | O |
| ATOM | 7129 | CB | LYS | E | 222 | 12.766 | 105.968 | -19.368 | 1.00 | 48.31 | B000 | C |
| ATOM | 7130 | CG | LYS | E | 222 | 13.519 | 106.492 | -20.561 | 1.00 | 57.72 | B000 | C |
| ATOM | 7131 | CD | LYS | E | 222 | 12.640 | 106.357 | -21.800 | 1.00 | 65.32 | B000 | C |
| ATOM | 7132 | CE | LYS | E | 222 | 12.661 | 104.903 | -22.305 | 1.00 | 75.88 | B000 | C |
| ATOM | 7133 | NZ | LYS | E | 222 | 12.007 | 104.696 | -23.635 | 1.00 | 76.97 | B000 | N1+ |
| ATOM | 7134 | N | TRP | E | 223 | 12.133 | 106.321 | -16.298 | 1.00 | 47.14 | B000 | N |
| ATOM | 7135 | CA | TRP | E | 223 | 11.031 | 106.134 | -15.372 | 1.00 | 41.03 | B000 | C |
| ATOM | 7136 | C | TRP | E | 223 | 9.714 | 106.314 | -16.124 | 1.00 | 47.51 | B000 | C |
| ATOM | 7137 | O | TRP | E | 223 | 9.617 | 107.117 | -17.056 | 1.00 | 50.81 | B000 | O |
| ATOM | 7138 | CB | TRP | E | 223 | 11.115 | 107.123 | -14.218 | 1.00 | 39.77 | B000 | C |
| ATOM | 7139 | CG | TRP | E | 223 | 12.293 | 106.912 | -13.328 | 1.00 | 40.93 | B000 | C |
| ATOM | 7140 | CD1 | TRP | E | 223 | 13.467 | 107.621 | -13.338 | 1.00 | 41.70 | B000 | C |
| ATOM | 7141 | CD2 | TRP | E | 223 | 12.410 | 105.957 | -12.270 | 1.00 | 41.60 | B000 | C |
| ATOM | 7142 | NE1 | TRP | E | 223 | 14.311 | 107.148 | -12.366 | 1.00 | 40.09 | B000 | N |
| ATOM | 7143 | CE2 | TRP | E | 223 | 13.688 | 106.129 | -11.692 | 1.00 | 39.35 | B000 | C |
| ATOM | 7144 | CE3 | TRP | E | 223 | 11.561 | 104.962 | -11.758 | 1.00 | 42.49 | B000 | C |
| ATOM | 7145 | CZ2 | TRP | E | 223 | 14.141 | 105.342 | -10.627 | 1.00 | 38.41 | B000 | C |
| ATOM | 7146 | CZ3 | TRP | E | 223 | 12.010 | 104.183 | -10.698 | 1.00 | 39.66 | B000 | C |
| ATOM | 7147 | CH2 | TRP | E | 223 | 13.291 | 104.374 | -10.147 | 1.00 | 37.68 | B000 | C |
| ATOM | 7148 | N | VAL | E | 224 | 8.693 | 105.555 | -15.715 | 1.00 | 38.47 | B000 | N |
| ATOM | 7149 | CA | VAL | E | 224 | 7.441 | 105.568 | -16.467 | 1.00 | 44.59 | B000 | C |
| ATOM | 7150 | C | VAL | E | 224 | 6.732 | 106.924 | -16.423 | 1.00 | 46.85 | B000 | C |
| ATOM | 7151 | O | VAL | E | 224 | 5.908 | 107.197 | -17.298 | 1.00 | 50.06 | B000 | O |
| ATOM | 7152 | CB | VAL | E | 224 | 6.487 | 104.458 | -15.967 | 1.00 | 42.40 | B000 | C |
| ATOM | 7153 | CG1 | VAL | E | 224 | 7.059 | 103.070 | -16.288 | 1.00 | 42.00 | B000 | C |
| ATOM | 7154 | CG2 | VAL | E | 224 | 6.227 | 104.600 | -14.475 | 1.00 | 34.29 | B000 | C |
| ATOM | 7155 | N | ASP | E | 225 | 7.017 | 107.790 | -15.444 | 1.00 | 48.54 | B000 | N |
| ATOM | 7156 | CA | ASP | E | 225 | 6.329 | 109.079 | -15.338 | 1.00 | 53.13 | B000 | C |
| ATOM | 7157 | C | ASP | E | 225 | 7.149 | 110.250 | -15.865 | 1.00 | 51.10 | B000 | C |
| ATOM | 7158 | O | ASP | E | 225 | 6.709 | 111.394 | -15.749 | 1.00 | 47.62 | B000 | O |
| ATOM | 7159 | CB | ASP | E | 225 | 5.898 | 109.358 | -13.886 | 1.00 | 39.25 | B000 | C |
| ATOM | 7160 | CG | ASP | E | 225 | 7.078 | 109.617 | -12.948 | 1.00 | 49.56 | B000 | C |
| ATOM | 7161 | OD1 | ASP | E | 225 | 8.244 | 109.417 | -13.352 | 1.00 | 47.55 | B000 | O |
| ATOM | 7162 | OD2 | ASP | E | 225 | 6.834 | 109.961 | -11.767 | 1.00 | 55.28 | B000 | O1- |
| ATOM | 7163 | N | GLY | E | 226 | 8.319 | 110.001 | -16.449 | 1.00 | 50.70 | B000 | N |
| ATOM | 7164 | CA | GLY | E | 226 | 9.118 | 111.058 | -17.029 | 1.00 | 50.75 | B000 | C |
| ATOM | 7165 | C | GLY | E | 226 | 10.206 | 111.608 | -16.128 | 1.00 | 49.85 | B000 | C |

| | | | | | | | | | | | | |
|---|---|---|---|---|---|---|---|---|---|---|---|---|
| ATOM | 7166 | O | GLY | E | 226 | 11.083 | 112.321 | -16.614 | 1.00 | 55.13 | B000 | O |
| ATOM | 7167 | N | THR | E | 227 | 10.149 | 111.328 | -14.832 | 1.00 | 46.51 | B000 | N |
| ATOM | 7168 | CA | THR | E | 227 | 11.258 | 111.622 | -13.942 | 1.00 | 42.53 | B000 | C |
| ATOM | 7169 | C | THR | E | 227 | 12.579 | 111.301 | -14.624 | 1.00 | 50.97 | B000 | C |
| ATOM | 7170 | O | THR | E | 227 | 12.760 | 110.213 | -15.178 | 1.00 | 57.64 | B000 | O |
| ATOM | 7171 | CB | THR | E | 227 | 11.117 | 110.795 | -12.667 | 1.00 | 48.55 | B000 | C |
| ATOM | 7172 | OG1 | THR | E | 227 | 9.834 | 111.037 | -12.070 | 1.00 | 46.24 | B000 | O |
| ATOM | 7173 | CG2 | THR | E | 227 | 12.226 | 111.120 | -11.690 | 1.00 | 41.66 | B000 | C |
| ATOM | 7174 | N | ASP | E | 228 | 13.490 | 112.268 | -14.601 | 1.00 | 59.03 | B000 | N |
| ATOM | 7175 | CA | ASP | E | 228 | 14.770 | 112.131 | -15.280 | 1.00 | 45.94 | B000 | C |
| ATOM | 7176 | C | ASP | E | 228 | 15.624 | 111.088 | -14.580 | 1.00 | 51.75 | B000 | C |
| ATOM | 7177 | O | ASP | E | 228 | 15.872 | 111.182 | -13.374 | 1.00 | 56.28 | B000 | O |
| ATOM | 7178 | CB | ASP | E | 228 | 15.501 | 113.474 | -15.311 | 1.00 | 47.30 | B000 | C |
| ATOM | 7179 | CG | ASP | E | 228 | 16.845 | 113.379 | -15.998 | 1.00 | 56.00 | B000 | C |
| ATOM | 7180 | OD1 | ASP | E | 228 | 16.859 | 113.296 | -17.250 | 1.00 | 54.17 | B000 | O |
| ATOM | 7181 | OD2 | ASP | E | 228 | 17.881 | 113.348 | -15.290 | 1.00 | 56.42 | B000 | O1- |
| ATOM | 7182 | N | TYR | E | 229 | 16.112 | 110.114 | -15.350 | 1.00 | 52.14 | B000 | N |
| ATOM | 7183 | CA | TYR | E | 229 | 16.829 | 109.000 | -14.743 | 1.00 | 46.06 | B000 | C |
| ATOM | 7184 | C | TYR | E | 229 | 18.227 | 109.400 | -14.287 | 1.00 | 47.79 | B000 | C |
| ATOM | 7185 | O | TYR | E | 229 | 18.667 | 108.998 | -13.201 | 1.00 | 47.55 | B000 | O |
| ATOM | 7186 | CB | TYR | E | 229 | 16.901 | 107.830 | -15.729 | 1.00 | 43.66 | B000 | C |
| ATOM | 7187 | CG | TYR | E | 229 | 17.852 | 106.745 | -15.304 | 1.00 | 42.09 | B000 | C |
| ATOM | 7188 | CD1 | TYR | E | 229 | 17.523 | 105.882 | -14.269 | 1.00 | 38.14 | B000 | C |
| ATOM | 7189 | CD2 | TYR | E | 229 | 19.090 | 106.594 | -15.917 | 1.00 | 42.41 | B000 | C |
| ATOM | 7190 | CE1 | TYR | E | 229 | 18.387 | 104.882 | -13.864 | 1.00 | 40.35 | B000 | C |
| ATOM | 7191 | CE2 | TYR | E | 229 | 19.968 | 105.589 | -15.517 | 1.00 | 46.35 | B000 | C |
| ATOM | 7192 | CZ | TYR | E | 229 | 19.599 | 104.734 | -14.486 | 1.00 | 46.45 | B000 | C |
| ATOM | 7193 | OH | TYR | E | 229 | 20.450 | 103.738 | -14.064 | 1.00 | 40.57 | B000 | O |
| ATOM | 7194 | N | GLU | E | 230 | 18.954 | 110.170 | -15.103 | 1.00 | 49.15 | B000 | N |
| ATOM | 7195 | CA | GLU | E | 230 | 20.375 | 110.365 | -14.821 | 1.00 | 46.97 | B000 | C |
| ATOM | 7196 | C | GLU | E | 230 | 20.591 | 111.198 | -13.556 | 1.00 | 51.12 | B000 | C |
| ATOM | 7197 | O | GLU | E | 230 | 21.457 | 110.879 | -12.738 | 1.00 | 53.36 | B000 | O |
| ATOM | 7198 | CB | GLU | E | 230 | 21.082 | 110.992 | -16.017 | 1.00 | 50.20 | B000 | C |
| ATOM | 7199 | CG | GLU | E | 230 | 22.607 | 110.803 | -15.963 | 1.00 | 63.82 | B000 | C |
| ATOM | 7200 | CD | GLU | E | 230 | 23.021 | 109.363 | -15.585 | 1.00 | 76.02 | B000 | C |
| ATOM | 7201 | OE1 | GLU | E | 230 | 22.553 | 108.396 | -16.253 | 1.00 | 66.88 | B000 | O |
| ATOM | 7202 | OE2 | GLU | E | 230 | 23.821 | 109.201 | -14.625 | 1.00 | 79.76 | B000 | O1- |
| ATOM | 7203 | N | THR | E | 231 | 19.809 | 112.248 | -13.359 | 1.00 | 48.10 | B000 | N |
| ATOM | 7204 | CA | THR | E | 231 | 19.981 | 113.081 | -12.176 | 1.00 | 56.05 | B000 | C |
| ATOM | 7205 | C | THR | E | 231 | 19.161 | 112.602 | -10.988 | 1.00 | 60.28 | B000 | C |
| ATOM | 7206 | O | THR | E | 231 | 19.280 | 113.183 | -9.900 | 1.00 | 51.86 | B000 | O |
| ATOM | 7207 | CB | THR | E | 231 | 19.606 | 114.531 | -12.495 | 1.00 | 52.22 | B000 | C |
| ATOM | 7208 | OG1 | THR | E | 231 | 18.217 | 114.591 | -12.844 | 1.00 | 53.83 | B000 | O |
| ATOM | 7209 | CG2 | THR | E | 231 | 20.445 | 115.043 | -13.668 | 1.00 | 46.56 | B000 | C |
| ATOM | 7210 | N | GLY | E | 232 | 18.363 | 111.540 | -11.160 | 1.00 | 55.84 | B000 | N |
| ATOM | 7211 | CA | GLY | E | 232 | 17.470 | 111.077 | -10.123 | 1.00 | 48.35 | B000 | C |
| ATOM | 7212 | C | GLY | E | 232 | 18.105 | 110.067 | -9.184 | 1.00 | 48.84 | B000 | C |
| ATOM | 7213 | O | GLY | E | 232 | 19.249 | 109.642 | -9.345 | 1.00 | 44.82 | B000 | O |
| ATOM | 7214 | N | PHE | E | 233 | 17.313 | 109.679 | -8.185 | 1.00 | 47.45 | B000 | N |
| ATOM | 7215 | CA | PHE | E | 233 | 17.724 | 108.672 | -7.218 | 1.00 | 45.48 | B000 | C |
| ATOM | 7216 | C | PHE | E | 233 | 17.998 | 107.339 | -7.920 | 1.00 | 45.39 | B000 | C |
| ATOM | 7217 | O | PHE | E | 233 | 17.309 | 106.963 | -8.870 | 1.00 | 43.33 | B000 | O |
| ATOM | 7218 | CB | PHE | E | 233 | 16.627 | 108.531 | -6.141 | 1.00 | 43.07 | B000 | C |
| ATOM | 7219 | CG | PHE | E | 233 | 16.952 | 107.537 | -5.062 | 1.00 | 49.50 | B000 | C |
| ATOM | 7220 | CD1 | PHE | E | 233 | 17.819 | 107.865 | -4.031 | 1.00 | 49.13 | B000 | C |
| ATOM | 7221 | CD2 | PHE | E | 233 | 16.393 | 106.267 | -5.077 | 1.00 | 46.36 | B000 | C |
| ATOM | 7222 | CE1 | PHE | E | 233 | 18.130 | 106.941 | -3.043 | 1.00 | 56.48 | B000 | C |
| ATOM | 7223 | CE2 | PHE | E | 233 | 16.703 | 105.332 | -4.089 | 1.00 | 43.16 | B000 | C |
| ATOM | 7224 | CZ | PHE | E | 233 | 17.570 | 105.668 | -3.073 | 1.00 | 50.08 | B000 | C |
| ATOM | 7225 | N | LYS | E | 234 | 19.027 | 106.631 | -7.461 | 1.00 | 43.29 | B000 | N |
| ATOM | 7226 | CA | LYS | E | 234 | 19.350 | 105.304 | -7.964 | 1.00 | 42.55 | B000 | C |
| ATOM | 7227 | C | LYS | E | 234 | 19.835 | 104.464 | -6.800 | 1.00 | 48.47 | B000 | C |
| ATOM | 7228 | O | LYS | E | 234 | 20.501 | 104.989 | -5.904 | 1.00 | 44.87 | B000 | O |
| ATOM | 7229 | CB | LYS | E | 234 | 20.433 | 105.329 | -9.047 | 1.00 | 43.84 | B000 | C |
| ATOM | 7230 | CG | LYS | E | 234 | 20.093 | 106.096 | -10.319 | 1.00 | 43.53 | B000 | C |
| ATOM | 7231 | CD | LYS | E | 234 | 21.305 | 106.028 | -11.250 | 1.00 | 42.53 | B000 | C |
| ATOM | 7232 | CE | LYS | E | 234 | 21.235 | 107.016 | -12.398 | 1.00 | 46.96 | B000 | C |
| ATOM | 7233 | NZ | LYS | E | 234 | 21.273 | 108.424 | -11.907 | 1.00 | 55.21 | B000 | N1+ |
| ATOM | 7234 | N | ASN | E | 235 | 19.486 | 103.171 | -6.793 | 1.00 | 46.39 | B000 | N |
| ATOM | 7235 | CA | ASN | E | 235 | 19.925 | 102.276 | -5.717 | 1.00 | 42.44 | B000 | C |
| ATOM | 7236 | C | ASN | E | 235 | 20.263 | 100.885 | -6.277 | 1.00 | 41.99 | B000 | C |

| | | | | | | | | | | | | |
|---|---|---|---|---|---|---|---|---|---|---|---|---|
| ATOM | 7237 | O | ASN | E | 235 | 19.881 | 99.849 | -5.728 | 1.00 | 41.55 | B000 | O |
| ATOM | 7238 | CB | ASN | E | 235 | 18.867 | 102.211 | -4.605 | 1.00 | 37.90 | B000 | C |
| ATOM | 7239 | CG | ASN | E | 235 | 19.353 | 101.448 | -3.380 | 1.00 | 40.64 | B000 | C |
| ATOM | 7240 | OD1 | ASN | E | 235 | 20.538 | 101.458 | -3.079 | 1.00 | 45.11 | B000 | O |
| ATOM | 7241 | ND2 | ASN | E | 235 | 18.449 | 100.769 | -2.685 | 1.00 | 41.74 | B000 | N |
| ATOM | 7242 | N | TRP | E | 236 | 21.051 | 100.853 | -7.346 | 1.00 | 39.52 | B000 | N |
| ATOM | 7243 | CA | TRP | E | 236 | 21.407 | 99.599 | -7.994 | 1.00 | 37.91 | B000 | C |
| ATOM | 7244 | C | TRP | E | 236 | 22.266 | 98.720 | -7.099 | 1.00 | 42.70 | B000 | C |
| ATOM | 7245 | O | TRP | E | 236 | 23.054 | 99.199 | -6.281 | 1.00 | 46.72 | B000 | O |
| ATOM | 7246 | CB | TRP | E | 236 | 22.178 | 99.848 | -9.299 | 1.00 | 34.60 | B000 | C |
| ATOM | 7247 | CG | TRP | E | 236 | 21.377 | 100.494 | -10.396 | 1.00 | 43.16 | B000 | C |
| ATOM | 7248 | CD1 | TRP | E | 236 | 21.452 | 101.794 | -10.810 | 1.00 | 36.82 | B000 | C |
| ATOM | 7249 | CD2 | TRP | E | 236 | 20.363 | 99.873 | -11.207 | 1.00 | 38.15 | B000 | C |
| ATOM | 7250 | NE1 | TRP | E | 236 | 20.565 | 102.017 | -11.839 | 1.00 | 41.26 | B000 | N |
| ATOM | 7251 | CE2 | TRP | E | 236 | 19.884 | 100.857 | -12.103 | 1.00 | 39.75 | B000 | C |
| ATOM | 7252 | CE3 | TRP | E | 236 | 19.831 | 98.581 | -11.273 | 1.00 | 36.10 | B000 | C |
| ATOM | 7253 | CZ2 | TRP | E | 236 | 18.894 | 100.591 | -13.054 | 1.00 | 35.54 | B000 | C |
| ATOM | 7254 | CZ3 | TRP | E | 236 | 18.850 | 98.311 | -12.219 | 1.00 | 35.95 | B000 | C |
| ATOM | 7255 | CH2 | TRP | E | 236 | 18.389 | 99.317 | -13.096 | 1.00 | 41.76 | B000 | C |
| ATOM | 7256 | N | ARG | E | 237 | 22.102 | 97.410 | -7.274 | 1.00 | 45.40 | B000 | N |
| ATOM | 7257 | CA | ARG | E | 237 | 23.050 | 96.440 | -6.763 | 1.00 | 47.18 | B000 | C |
| ATOM | 7258 | C | ARG | E | 237 | 24.449 | 96.720 | -7.314 | 1.00 | 51.81 | B000 | C |
| ATOM | 7259 | O | ARG | E | 237 | 24.595 | 97.350 | -8.368 | 1.00 | 43.69 | B000 | O |
| ATOM | 7260 | CB | ARG | E | 237 | 22.634 | 95.041 | -7.185 | 1.00 | 46.24 | B000 | C |
| ATOM | 7261 | CG | ARG | E | 237 | 21.530 | 94.422 | -6.393 | 1.00 | 51.40 | B000 | C |
| ATOM | 7262 | CD | ARG | E | 237 | 22.096 | 93.585 | -5.285 | 1.00 | 58.45 | B000 | C |
| ATOM | 7263 | NE | ARG | E | 237 | 21.038 | 92.772 | -4.710 | 1.00 | 70.66 | B000 | N |
| ATOM | 7264 | CZ | ARG | E | 237 | 21.262 | 91.703 | -3.961 | 1.00 | 72.34 | B000 | C |
| ATOM | 7265 | NH1 | ARG | E | 237 | 20.232 | 90.990 | -3.494 | 1.00 | 63.81 | B000 | N1+ |
| ATOM | 7266 | NH2 | ARG | E | 237 | 22.525 | 91.368 | -3.681 | 1.00 | 60.30 | B000 | N |
| ATOM | 7267 | N | PRO | E | 238 | 25.489 | 96.240 | -6.628 | 1.00 | 52.18 | B000 | N |
| ATOM | 7268 | CA | PRO | E | 238 | 26.853 | 96.359 | -7.163 | 1.00 | 56.03 | B000 | C |
| ATOM | 7269 | C | PRO | E | 238 | 26.950 | 95.788 | -8.569 | 1.00 | 52.87 | B000 | C |
| ATOM | 7270 | O | PRO | E | 238 | 26.528 | 94.660 | -8.830 | 1.00 | 56.32 | B000 | O |
| ATOM | 7271 | CB | PRO | E | 238 | 27.694 | 95.545 | -6.171 | 1.00 | 52.68 | B000 | C |
| ATOM | 7272 | CG | PRO | E | 238 | 26.934 | 95.614 | -4.891 | 1.00 | 56.74 | B000 | C |
| ATOM | 7273 | CD | PRO | E | 238 | 25.473 | 95.617 | -5.291 | 1.00 | 58.40 | B000 | C |
| ATOM | 7274 | N | GLU | E | 239 | 27.508 | 96.588 | -9.477 | 1.00 | 54.34 | B000 | N |
| ATOM | 7275 | CA | GLU | E | 239 | 27.753 | 96.254 | -10.877 | 1.00 | 53.49 | B000 | C |
| ATOM | 7276 | C | GLU | E | 239 | 26.481 | 96.144 | -11.711 | 1.00 | 55.15 | B000 | C |
| ATOM | 7277 | O | GLU | E | 239 | 26.545 | 95.684 | -12.862 | 1.00 | 57.25 | B000 | O |
| ATOM | 7278 | CB | GLU | E | 239 | 28.578 | 94.974 | -11.029 | 1.00 | 50.60 | B000 | C |
| ATOM | 7279 | CG | GLU | E | 239 | 30.001 | 95.104 | -10.514 | 1.00 | 66.65 | B000 | C |
| ATOM | 7280 | CD | GLU | E | 239 | 30.663 | 93.760 | -10.297 | 1.00 | 83.88 | B000 | C |
| ATOM | 7281 | OE1 | GLU | E | 239 | 30.038 | 92.728 | -10.639 | 1.00 | 75.71 | B000 | O |
| ATOM | 7282 | OE2 | GLU | E | 239 | 31.816 | 93.739 | -9.808 | 1.00 | 96.45 | B000 | O1- |
| ATOM | 7283 | N | GLN | E | 240 | 25.336 | 96.571 | -11.187 | 1.00 | 42.77 | B000 | N |
| ATOM | 7284 | CA | GLN | E | 240 | 24.126 | 96.666 | -11.990 | 1.00 | 44.13 | B000 | C |
| ATOM | 7285 | C | GLN | E | 240 | 23.886 | 98.142 | -12.320 | 1.00 | 39.19 | B000 | C |
| ATOM | 7286 | O | GLN | E | 240 | 24.353 | 99.013 | -11.600 | 1.00 | 37.74 | B000 | O |
| ATOM | 7287 | CB | GLN | E | 240 | 22.931 | 96.054 | -11.253 | 1.00 | 41.92 | B000 | C |
| ATOM | 7288 | CG | GLN | E | 240 | 23.174 | 94.638 | -10.737 | 1.00 | 41.56 | B000 | C |
| ATOM | 7289 | CD | GLN | E | 240 | 23.857 | 93.758 | -11.761 | 1.00 | 47.34 | B000 | C |
| ATOM | 7290 | OE1 | GLN | E | 240 | 23.461 | 93.713 | -12.927 | 1.00 | 45.90 | B000 | O |
| ATOM | 7291 | NE2 | GLN | E | 240 | 24.908 | 93.071 | -11.336 | 1.00 | 53.15 | B000 | N |
| ATOM | 7292 | N | PRO | E | 241 | 23.176 | 98.433 | -13.423 | 1.00 | 39.17 | B000 | N |
| ATOM | 7293 | CA | PRO | E | 241 | 22.582 | 97.492 | -14.382 | 1.00 | 41.50 | B000 | C |
| ATOM | 7294 | C | PRO | E | 241 | 23.654 | 96.945 | -15.327 | 1.00 | 39.88 | B000 | C |
| ATOM | 7295 | O | PRO | E | 241 | 24.800 | 97.332 | -15.130 | 1.00 | 47.11 | B000 | O |
| ATOM | 7296 | CB | PRO | E | 241 | 21.539 | 98.350 | -15.114 | 1.00 | 41.31 | B000 | C |
| ATOM | 7297 | CG | PRO | E | 241 | 22.116 | 99.770 | -15.042 | 1.00 | 40.16 | B000 | C |
| ATOM | 7298 | CD | PRO | E | 241 | 22.851 | 99.844 | -13.732 | 1.00 | 35.10 | B000 | C |
| ATOM | 7299 | N | ASP | E | 242 | 23.325 | 96.074 | -16.287 | 1.00 | 39.07 | B000 | N |
| ATOM | 7300 | CA | ASP | E | 242 | 24.337 | 95.571 | -17.221 | 1.00 | 42.10 | B000 | C |
| ATOM | 7301 | C | ASP | E | 242 | 25.112 | 96.732 | -17.827 | 1.00 | 45.64 | B000 | C |
| ATOM | 7302 | O | ASP | E | 242 | 24.512 | 97.697 | -18.317 | 1.00 | 42.87 | B000 | O |
| ATOM | 7303 | CB | ASP | E | 242 | 23.691 | 94.761 | -18.348 | 1.00 | 42.84 | B000 | C |
| ATOM | 7304 | CG | ASP | E | 242 | 22.886 | 93.568 | -17.852 | 1.00 | 43.37 | B000 | C |
| ATOM | 7305 | OD1 | ASP | E | 242 | 23.330 | 92.905 | -16.895 | 1.00 | 46.50 | B000 | O |
| ATOM | 7306 | OD2 | ASP | E | 242 | 21.800 | 93.308 | -18.430 | 1.00 | 43.11 | B000 | O1- |
| ATOM | 7307 | N | ASP | E | 243 | 26.448 | 96.634 | -17.808 | 1.00 | 39.31 | B000 | N |

| | | | | | | | | | | | | |
|---|---|---|---|---|---|---|---|---|---|---|---|---|
| ATOM | 7308 | CA | ASP | E | 243 | 27.300 | 97.761 | -18.190 | 1.00 | 47.79 | B000 | C |
| ATOM | 7309 | C | ASP | E | 243 | 27.812 | 97.693 | -19.621 | 1.00 | 43.78 | B000 | C |
| ATOM | 7310 | O | ASP | E | 243 | 28.698 | 98.475 | -19.976 | 1.00 | 46.39 | B000 | O |
| ATOM | 7311 | CB | ASP | E | 243 | 28.506 | 97.880 | -17.250 | 1.00 | 43.08 | B000 | C |
| ATOM | 7312 | CG | ASP | E | 243 | 29.395 | 96.633 | -17.252 | 1.00 | 54.01 | B000 | C |
| ATOM | 7313 | OD1 | ASP | E | 243 | 29.151 | 95.676 | -18.025 | 1.00 | 56.31 | B000 | O |
| ATOM | 7314 | OD2 | ASP | E | 243 | 30.350 | 96.604 | -16.453 | 1.00 | 60.30 | B000 | O1- |
| ATOM | 7315 | N | TRP | E | 244 | 27.332 | 96.754 | -20.435 | 1.00 | 42.79 | B000 | N |
| ATOM | 7316 | CA | TRP | E | 244 | 27.941 | 96.563 | -21.742 | 1.00 | 44.21 | B000 | C |
| ATOM | 7317 | C | TRP | E | 244 | 27.283 | 97.361 | -22.860 | 1.00 | 43.63 | B000 | C |
| ATOM | 7318 | O | TRP | E | 244 | 27.674 | 97.194 | -24.018 | 1.00 | 43.92 | B000 | O |
| ATOM | 7319 | CB | TRP | E | 244 | 28.043 | 95.072 | -22.108 | 1.00 | 43.09 | B000 | C |
| ATOM | 7320 | CG | TRP | E | 244 | 26.891 | 94.182 | -21.886 | 1.00 | 44.80 | B000 | C |
| ATOM | 7321 | CD1 | TRP | E | 244 | 25.879 | 93.900 | -22.770 | 1.00 | 47.43 | B000 | C |
| ATOM | 7322 | CD2 | TRP | E | 244 | 26.668 | 93.355 | -20.746 | 1.00 | 43.82 | B000 | C |
| ATOM | 7323 | NE1 | TRP | E | 244 | 25.016 | 92.980 | -22.226 | 1.00 | 44.04 | B000 | N |
| ATOM | 7324 | CE2 | TRP | E | 244 | 25.482 | 92.621 | -20.987 | 1.00 | 46.21 | B000 | C |
| ATOM | 7325 | CE3 | TRP | E | 244 | 27.346 | 93.171 | -19.540 | 1.00 | 42.76 | B000 | C |
| ATOM | 7326 | CZ2 | TRP | E | 244 | 24.957 | 91.729 | -20.060 | 1.00 | 44.01 | B000 | C |
| ATOM | 7327 | CZ3 | TRP | E | 244 | 26.827 | 92.292 | -18.622 | 1.00 | 42.24 | B000 | C |
| ATOM | 7328 | CH2 | TRP | E | 244 | 25.641 | 91.579 | -18.883 | 1.00 | 49.08 | B000 | C |
| ATOM | 7329 | N | TYR | E | 245 | 26.330 | 98.242 | -22.550 | 1.00 | 43.45 | B000 | N |
| ATOM | 7330 | CA | TYR | E | 245 | 25.626 | 99.013 | -23.574 | 1.00 | 45.01 | B000 | C |
| ATOM | 7331 | C | TYR | E | 245 | 26.012 | 100.481 | -23.628 | 1.00 | 42.12 | B000 | C |
| ATOM | 7332 | O | TYR | E | 245 | 25.598 | 101.169 | -24.560 | 1.00 | 45.10 | B000 | O |
| ATOM | 7333 | CB | TYR | E | 245 | 24.099 | 98.947 | -23.366 | 1.00 | 42.26 | B000 | C |
| ATOM | 7334 | CG | TYR | E | 245 | 23.544 | 97.553 | -23.256 | 1.00 | 43.72 | B000 | C |
| ATOM | 7335 | CD1 | TYR | E | 245 | 23.355 | 96.770 | -24.391 | 1.00 | 40.29 | B000 | C |
| ATOM | 7336 | CD2 | TYR | E | 245 | 23.224 | 97.006 | -22.016 | 1.00 | 39.35 | B000 | C |
| ATOM | 7337 | CE1 | TYR | E | 245 | 22.841 | 95.500 | -24.301 | 1.00 | 39.08 | B000 | C |
| ATOM | 7338 | CE2 | TYR | E | 245 | 22.717 | 95.722 | -21.918 | 1.00 | 43.84 | B000 | C |
| ATOM | 7339 | CZ | TYR | E | 245 | 22.533 | 94.976 | -23.064 | 1.00 | 41.96 | B000 | C |
| ATOM | 7340 | OH | TYR | E | 245 | 22.036 | 93.706 | -22.975 | 1.00 | 45.97 | B000 | O |
| ATOM | 7341 | N | GLY | E | 246 | 26.710 | 101.001 | -22.638 | 1.00 | 41.64 | B000 | N |
| ATOM | 7342 | CA | GLY | E | 246 | 26.989 | 102.420 | -22.592 | 1.00 | 43.53 | B000 | C |
| ATOM | 7343 | C | GLY | E | 246 | 26.255 | 103.119 | -21.455 | 1.00 | 47.49 | B000 | C |
| ATOM | 7344 | O | GLY | E | 246 | 25.273 | 102.626 | -20.898 | 1.00 | 44.82 | B000 | O |
| ATOM | 7345 | N | HIS | E | 247 | 26.714 | 104.343 | -21.189 | 1.00 | 50.58 | B000 | N |
| ATOM | 7346 | CA | HIS | E | 247 | 26.284 | 105.118 | -20.030 | 1.00 | 42.87 | B000 | C |
| ATOM | 7347 | C | HIS | E | 247 | 24.783 | 105.367 | -20.033 | 1.00 | 44.35 | B000 | C |
| ATOM | 7348 | O | HIS | E | 247 | 24.236 | 105.933 | -20.985 | 1.00 | 45.35 | B000 | O |
| ATOM | 7349 | CB | HIS | E | 247 | 27.044 | 106.440 | -20.003 | 1.00 | 53.73 | B000 | C |
| ATOM | 7350 | CG | HIS | E | 247 | 26.584 | 107.373 | -18.933 | 1.00 | 56.75 | B000 | C |
| ATOM | 7351 | ND1 | HIS | E | 247 | 26.920 | 107.206 | -17.607 | 1.00 | 59.80 | B000 | N |
| ATOM | 7352 | CD2 | HIS | E | 247 | 25.799 | 108.476 | -18.991 | 1.00 | 55.82 | B000 | C |
| ATOM | 7353 | CE1 | HIS | E | 247 | 26.367 | 108.172 | -16.894 | 1.00 | 66.18 | B000 | C |
| ATOM | 7354 | NE2 | HIS | E | 247 | 25.682 | 108.956 | -17.710 | 1.00 | 63.47 | B000 | N |
| ATOM | 7355 | N | GLY | E | 248 | 24.117 | 104.949 | -18.954 | 1.00 | 45.02 | B000 | N |
| ATOM | 7356 | CA | GLY | E | 248 | 22.680 | 105.131 | -18.847 | 1.00 | 45.46 | B000 | C |
| ATOM | 7357 | C | GLY | E | 248 | 21.849 | 104.287 | -19.791 | 1.00 | 46.59 | B000 | C |
| ATOM | 7358 | O | GLY | E | 248 | 20.659 | 104.568 | -19.979 | 1.00 | 40.42 | B000 | O |
| ATOM | 7359 | N | LEU | E | 249 | 22.431 | 103.242 | -20.380 | 1.00 | 42.75 | B000 | N |
| ATOM | 7360 | CA | LEU | E | 249 | 21.760 | 102.493 | -21.430 | 1.00 | 42.93 | B000 | C |
| ATOM | 7361 | C | LEU | E | 249 | 21.579 | 101.026 | -21.050 | 1.00 | 42.71 | B000 | C |
| ATOM | 7362 | O | LEU | E | 249 | 22.327 | 100.466 | -20.239 | 1.00 | 35.38 | B000 | O |
| ATOM | 7363 | CB | LEU | E | 249 | 22.536 | 102.595 | -22.750 | 1.00 | 39.36 | B000 | C |
| ATOM | 7364 | CG | LEU | E | 249 | 22.689 | 104.006 | -23.343 | 1.00 | 42.86 | B000 | C |
| ATOM | 7365 | CD1 | LEU | E | 249 | 23.340 | 103.916 | -24.714 | 1.00 | 39.37 | B000 | C |
| ATOM | 7366 | CD2 | LEU | E | 249 | 21.350 | 104.700 | -23.442 | 1.00 | 40.64 | B000 | C |
| ATOM | 7367 | N | GLY | E | 250 | 20.569 | 100.410 | -21.653 | 1.00 | 40.13 | B000 | N |
| ATOM | 7368 | CA | GLY | E | 250 | 20.394 | 98.976 | -21.584 | 1.00 | 34.39 | B000 | C |
| ATOM | 7369 | C | GLY | E | 250 | 20.162 | 98.460 | -22.986 | 1.00 | 42.96 | B000 | C |
| ATOM | 7370 | O | GLY | E | 250 | 20.251 | 99.242 | -23.936 | 1.00 | 39.19 | B000 | O |
| ATOM | 7371 | N | GLY | E | 251 | 19.857 | 97.167 | -23.135 | 1.00 | 42.21 | B000 | N |
| ATOM | 7372 | CA | GLY | E | 251 | 19.622 | 96.533 | -24.418 | 1.00 | 38.08 | B000 | C |
| ATOM | 7373 | C | GLY | E | 251 | 18.179 | 96.437 | -24.892 | 1.00 | 41.51 | B000 | C |
| ATOM | 7374 | O | GLY | E | 251 | 17.932 | 95.813 | -25.932 | 1.00 | 47.66 | B000 | O |
| ATOM | 7375 | N | GLY | E | 252 | 17.209 | 97.010 | -24.169 | 1.00 | 45.08 | B000 | N |
| ATOM | 7376 | CA | GLY | E | 252 | 15.832 | 97.049 | -24.628 | 1.00 | 44.10 | B000 | C |
| ATOM | 7377 | C | GLY | E | 252 | 14.893 | 95.971 | -24.115 | 1.00 | 40.91 | B000 | C |
| ATOM | 7378 | O | GLY | E | 252 | 13.707 | 96.009 | -24.452 | 1.00 | 51.51 | B000 | O |

| | | | | | | | | | | | | |
|---|---|---|---|---|---|---|---|---|---|---|---|---|
| ATOM | 7379 | N | GLU | E | 253 | 15.380 | 94.997 | -23.351 | 1.00 | 49.13 | B000 | N |
| ATOM | 7380 | CA | GLU | E | 253 | 14.569 | 93.886 | -22.849 | 1.00 | 44.54 | B000 | C |
| ATOM | 7381 | C | GLU | E | 253 | 14.410 | 93.867 | -21.335 | 1.00 | 40.80 | B000 | C |
| ATOM | 7382 | O | GLU | E | 253 | 13.844 | 92.902 | -20.810 | 1.00 | 43.41 | B000 | O |
| ATOM | 7383 | CB | GLU | E | 253 | 15.180 | 92.537 | -23.273 | 1.00 | 49.30 | B000 | C |
| ATOM | 7384 | CG | GLU | E | 253 | 15.553 | 92.443 | -24.748 | 1.00 | 58.40 | B000 | C |
| ATOM | 7385 | CD | GLU | E | 253 | 14.373 | 92.111 | -25.651 | 1.00 | 68.11 | B000 | C |
| ATOM | 7386 | OE1 | GLU | E | 253 | 13.919 | 90.934 | -25.628 | 1.00 | 71.69 | B000 | O |
| ATOM | 7387 | OE2 | GLU | E | 253 | 13.900 | 93.027 | -26.378 | 1.00 | 62.49 | B000 | O1- |
| ATOM | 7388 | N | ASP | E | 254 | 14.951 | 94.848 | -20.611 | 1.00 | 41.14 | B000 | N |
| ATOM | 7389 | CA | ASP | E | 254 | 15.056 | 94.754 | -19.159 | 1.00 | 36.67 | B000 | C |
| ATOM | 7390 | C | ASP | E | 254 | 14.148 | 95.742 | -18.448 | 1.00 | 37.02 | B000 | C |
| ATOM | 7391 | O | ASP | E | 254 | 13.733 | 96.776 | -18.990 | 1.00 | 37.00 | B000 | O |
| ATOM | 7392 | CB | ASP | E | 254 | 16.497 | 94.967 | -18.685 | 1.00 | 34.36 | B000 | C |
| ATOM | 7393 | CG | ASP | E | 254 | 17.333 | 93.693 | -18.779 | 1.00 | 37.96 | B000 | C |
| ATOM | 7394 | OD1 | ASP | E | 254 | 16.776 | 92.662 | -19.226 | 1.00 | 36.64 | B000 | O |
| ATOM | 7395 | OD2 | ASP | E | 254 | 18.547 | 93.740 | -18.453 | 1.00 | 41.46 | B000 | O1- |
| ATOM | 7396 | N | CYS | E | 255 | 13.822 | 95.368 | -17.219 | 1.00 | 35.51 | B000 | N |
| ATOM | 7397 | CA | CYS | E | 255 | 13.086 | 96.210 | -16.300 | 1.00 | 32.38 | B000 | C |
| ATOM | 7398 | C | CYS | E | 255 | 13.781 | 96.107 | -14.955 | 1.00 | 37.45 | B000 | C |
| ATOM | 7399 | O | CYS | E | 255 | 14.493 | 95.137 | -14.689 | 1.00 | 34.78 | B000 | O |
| ATOM | 7400 | CB | CYS | E | 255 | 11.612 | 95.789 | -16.219 | 1.00 | 34.76 | B000 | C |
| ATOM | 7401 | SG | CYS | E | 255 | 10.671 | 96.035 | -17.761 | 1.00 | 37.35 | B000 | S |
| ATOM | 7402 | N | ALA | E | 256 | 13.609 | 97.138 | -14.123 | 1.00 | 31.93 | B000 | N |
| ATOM | 7403 | CA | ALA | E | 256 | 14.263 | 97.192 | -12.823 | 1.00 | 34.86 | B000 | C |
| ATOM | 7404 | C | ALA | E | 256 | 13.329 | 96.663 | -11.744 | 1.00 | 35.59 | B000 | C |
| ATOM | 7405 | O | ALA | E | 256 | 12.138 | 96.994 | -11.720 | 1.00 | 30.92 | B000 | O |
| ATOM | 7406 | CB | ALA | E | 256 | 14.688 | 98.621 | -12.480 | 1.00 | 33.92 | B000 | C |
| ATOM | 7407 | N | HIS | E | 257 | 13.870 | 95.833 | -10.854 | 1.00 | 28.95 | B000 | N |
| ATOM | 7408 | CA | HIS | E | 257 | 13.097 | 95.348 | -9.721 | 1.00 | 35.16 | B000 | C |
| ATOM | 7409 | C | HIS | E | 257 | 13.897 | 95.485 | -8.434 | 1.00 | 35.13 | B000 | C |
| ATOM | 7410 | O | HIS | E | 257 | 15.130 | 95.402 | -8.425 | 1.00 | 34.18 | B000 | O |
| ATOM | 7411 | CB | HIS | E | 257 | 12.655 | 93.884 | -9.909 | 1.00 | 30.40 | B000 | C |
| ATOM | 7412 | CG | HIS | E | 257 | 13.789 | 92.909 | -10.005 | 1.00 | 36.00 | B000 | C |
| ATOM | 7413 | ND1 | HIS | E | 257 | 14.213 | 92.160 | -8.930 | 1.00 | 36.61 | B000 | N |
| ATOM | 7414 | CD2 | HIS | E | 257 | 14.576 | 92.547 | -11.050 | 1.00 | 29.26 | B000 | C |
| ATOM | 7415 | CE1 | HIS | E | 257 | 15.205 | 91.373 | -9.306 | 1.00 | 36.08 | B000 | C |
| ATOM | 7416 | NE2 | HIS | E | 257 | 15.444 | 91.589 | -10.588 | 1.00 | 41.23 | B000 | N |
| ATOM | 7417 | N | PHE | E | 258 | 13.178 | 95.722 | -7.344 | 1.00 | 32.98 | B000 | N |
| ATOM | 7418 | CA | PHE | E | 258 | 13.783 | 95.567 | -6.036 | 1.00 | 35.47 | B000 | C |
| ATOM | 7419 | C | PHE | E | 258 | 14.126 | 94.102 | -5.825 | 1.00 | 36.56 | B000 | C |
| ATOM | 7420 | O | PHE | E | 258 | 13.342 | 93.217 | -6.182 | 1.00 | 37.64 | B000 | O |
| ATOM | 7421 | CB | PHE | E | 258 | 12.828 | 96.024 | -4.943 | 1.00 | 34.07 | B000 | C |
| ATOM | 7422 | CG | PHE | E | 258 | 12.279 | 97.408 | -5.140 | 1.00 | 36.26 | B000 | C |
| ATOM | 7423 | CD1 | PHE | E | 258 | 12.991 | 98.528 | -4.707 | 1.00 | 31.80 | B000 | C |
| ATOM | 7424 | CD2 | PHE | E | 258 | 11.025 | 97.588 | -5.714 | 1.00 | 31.31 | B000 | C |
| ATOM | 7425 | CE1 | PHE | E | 258 | 12.469 | 99.798 | -4.865 | 1.00 | 33.16 | B000 | C |
| ATOM | 7426 | CE2 | PHE | E | 258 | 10.492 | 98.861 | -5.882 | 1.00 | 39.41 | B000 | C |
| ATOM | 7427 | CZ | PHE | E | 258 | 11.208 | 99.972 | -5.462 | 1.00 | 34.38 | B000 | C |
| ATOM | 7428 | N | THR | E | 259 | 15.302 | 93.853 | -5.249 | 1.00 | 37.86 | B000 | N |
| ATOM | 7429 | CA | THR | E | 259 | 15.716 | 92.540 | -4.772 | 1.00 | 42.13 | B000 | C |
| ATOM | 7430 | C | THR | E | 259 | 15.405 | 92.419 | -3.289 | 1.00 | 44.25 | B000 | C |
| ATOM | 7431 | O | THR | E | 259 | 14.879 | 93.339 | -2.660 | 1.00 | 47.92 | B000 | O |
| ATOM | 7432 | CB | THR | E | 259 | 17.209 | 92.303 | -4.996 | 1.00 | 42.54 | B000 | C |
| ATOM | 7433 | OG1 | THR | E | 259 | 17.953 | 93.161 | -4.117 | 1.00 | 44.35 | B000 | O |
| ATOM | 7434 | CG2 | THR | E | 259 | 17.579 | 92.593 | -6.433 | 1.00 | 39.50 | B000 | C |
| ATOM | 7435 | N | ASP | E | 260 | 15.785 | 91.280 | -2.712 | 1.00 | 46.15 | B000 | N |
| ATOM | 7436 | CA | ASP | E | 260 | 15.408 | 91.003 | -1.334 | 1.00 | 48.77 | B000 | C |
| ATOM | 7437 | C | ASP | E | 260 | 16.141 | 91.866 | -0.318 | 1.00 | 51.79 | B000 | C |
| ATOM | 7438 | O | ASP | E | 260 | 15.772 | 91.841 | 0.862 | 1.00 | 57.39 | B000 | O |
| ATOM | 7439 | CB | ASP | E | 260 | 15.627 | 89.530 | -1.002 | 1.00 | 48.59 | B000 | C |
| ATOM | 7440 | CG | ASP | E | 260 | 17.039 | 89.064 | -1.282 | 1.00 | 52.19 | B000 | C |
| ATOM | 7441 | OD1 | ASP | E | 260 | 17.934 | 89.890 | -1.563 | 1.00 | 54.71 | B000 | O |
| ATOM | 7442 | OD2 | ASP | E | 260 | 17.255 | 87.847 | -1.199 | 1.00 | 57.65 | B000 | O1- |
| ATOM | 7443 | N | ASP | E | 261 | 17.175 | 92.602 | -0.719 | 1.00 | 46.10 | B000 | N |
| ATOM | 7444 | CA | ASP | E | 261 | 17.795 | 93.567 | 0.177 | 1.00 | 47.68 | B000 | C |
| ATOM | 7445 | C | ASP | E | 261 | 17.411 | 95.007 | -0.158 | 1.00 | 43.64 | B000 | C |
| ATOM | 7446 | O | ASP | E | 261 | 17.959 | 95.938 | 0.434 | 1.00 | 50.15 | B000 | O |
| ATOM | 7447 | CB | ASP | E | 261 | 19.322 | 93.386 | 0.195 | 1.00 | 43.46 | B000 | C |
| ATOM | 7448 | CG | ASP | E | 261 | 20.013 | 93.851 | -1.092 | 1.00 | 51.38 | B000 | C |
| ATOM | 7449 | OD1 | ASP | E | 261 | 19.414 | 94.549 | -1.938 | 1.00 | 53.10 | B000 | O |

| | | | | | | | | | | | | |
|---|---|---|---|---|---|---|---|---|---|---|---|---|
| ATOM | 7450 | OD2 | ASP | E | 261 | 21.201 | 93.523 | -1.249 | 1.00 | 61.28 | B000 | O1- |
| ATOM | 7451 | N | GLY | E | 262 | 16.490 | 95.208 | -1.098 | 1.00 | 41.82 | B000 | N |
| ATOM | 7452 | CA | GLY | E | 262 | 16.009 | 96.524 | -1.460 | 1.00 | 37.30 | B000 | C |
| ATOM | 7453 | C | GLY | E | 262 | 16.761 | 97.180 | -2.597 | 1.00 | 36.57 | B000 | C |
| ATOM | 7454 | O | GLY | E | 262 | 16.212 | 98.052 | -3.272 | 1.00 | 41.75 | B000 | O |
| ATOM | 7455 | N | ARG | E | 263 | 18.012 | 96.804 | -2.802 | 1.00 | 38.36 | B000 | N |
| ATOM | 7456 | CA | ARG | E | 263 | 18.764 | 97.311 | -3.928 | 1.00 | 41.20 | B000 | C |
| ATOM | 7457 | C | ARG | E | 263 | 18.224 | 96.727 | -5.227 | 1.00 | 45.69 | B000 | C |
| ATOM | 7458 | O | ARG | E | 263 | 17.553 | 95.687 | -5.250 | 1.00 | 42.28 | B000 | O |
| ATOM | 7459 | CB | ARG | E | 263 | 20.242 | 96.991 | -3.749 | 1.00 | 41.55 | B000 | C |
| ATOM | 7460 | CG | ARG | E | 263 | 20.841 | 97.820 | -2.615 | 1.00 | 42.80 | B000 | C |
| ATOM | 7461 | CD | ARG | E | 263 | 22.274 | 97.464 | -2.375 | 1.00 | 42.94 | B000 | C |
| ATOM | 7462 | NE | ARG | E | 263 | 22.431 | 96.060 | -2.029 | 1.00 | 50.20 | B000 | N |
| ATOM | 7463 | CZ | ARG | E | 263 | 23.609 | 95.464 | -1.881 | 1.00 | 58.64 | B000 | C |
| ATOM | 7464 | NH1 | ARG | E | 263 | 24.728 | 96.164 | -2.057 | 1.00 | 53.73 | B000 | N1+ |
| ATOM | 7465 | NH2 | ARG | E | 263 | 23.670 | 94.172 | -1.573 | 1.00 | 56.41 | B000 | N |
| ATOM | 7466 | N | TRP | E | 264 | 18.506 | 97.428 | -6.314 | 1.00 | 43.23 | B000 | N |
| ATOM | 7467 | CA | TRP | E | 264 | 17.854 | 97.186 | -7.588 | 1.00 | 42.16 | B000 | C |
| ATOM | 7468 | C | TRP | E | 264 | 18.687 | 96.288 | -8.491 | 1.00 | 43.43 | B000 | C |
| ATOM | 7469 | O | TRP | E | 264 | 19.921 | 96.322 | -8.465 | 1.00 | 41.15 | B000 | O |
| ATOM | 7470 | CB | TRP | E | 264 | 17.595 | 98.493 | -8.322 | 1.00 | 37.69 | B000 | C |
| ATOM | 7471 | CG | TRP | E | 264 | 16.910 | 99.526 | -7.547 | 1.00 | 42.39 | B000 | C |
| ATOM | 7472 | CD1 | TRP | E | 264 | 16.342 | 99.410 | -6.306 | 1.00 | 42.33 | B000 | C |
| ATOM | 7473 | CD2 | TRP | E | 264 | 16.714 | 100.874 | -7.958 | 1.00 | 41.30 | B000 | C |
| ATOM | 7474 | NE1 | TRP | E | 264 | 15.797 | 100.613 | -5.924 | 1.00 | 37.50 | B000 | N |
| ATOM | 7475 | CE2 | TRP | E | 264 | 16.011 | 101.530 | -6.921 | 1.00 | 43.31 | B000 | C |
| ATOM | 7476 | CE3 | TRP | E | 264 | 17.068 | 101.595 | -9.104 | 1.00 | 38.49 | B000 | C |
| ATOM | 7477 | CZ2 | TRP | E | 264 | 15.649 | 102.882 | -6.999 | 1.00 | 46.31 | B000 | C |
| ATOM | 7478 | CZ3 | TRP | E | 264 | 16.716 | 102.938 | -9.179 | 1.00 | 44.68 | B000 | C |
| ATOM | 7479 | CH2 | TRP | E | 264 | 16.005 | 103.564 | -8.135 | 1.00 | 47.08 | B000 | C |
| ATOM | 7480 | N | ASN | E | 265 | 17.986 | 95.533 | -9.339 | 1.00 | 40.37 | B000 | N |
| ATOM | 7481 | CA | ASN | E | 265 | 18.601 | 94.718 | -10.374 | 1.00 | 38.67 | B000 | C |
| ATOM | 7482 | C | ASN | E | 265 | 17.770 | 94.816 | -11.652 | 1.00 | 35.88 | B000 | C |
| ATOM | 7483 | O | ASN | E | 265 | 16.561 | 95.055 | -11.608 | 1.00 | 33.72 | B000 | O |
| ATOM | 7484 | CB | ASN | E | 265 | 18.731 | 93.267 | -9.899 | 1.00 | 40.88 | B000 | C |
| ATOM | 7485 | CG | ASN | E | 265 | 19.363 | 92.369 | -10.940 | 1.00 | 49.30 | B000 | C |
| ATOM | 7486 | OD1 | ASN | E | 265 | 20.484 | 92.622 | -11.373 | 1.00 | 46.08 | B000 | O |
| ATOM | 7487 | ND2 | ASN | E | 265 | 18.667 | 91.288 | -11.315 | 1.00 | 49.35 | B000 | N |
| ATOM | 7488 | N | ASP | E | 266 | 18.431 | 94.646 | -12.795 | 1.00 | 37.44 | B000 | N |
| ATOM | 7489 | CA | ASP | E | 266 | 17.769 | 94.648 | -14.092 | 1.00 | 34.38 | B000 | C |
| ATOM | 7490 | C | ASP | E | 266 | 17.585 | 93.211 | -14.559 | 1.00 | 35.40 | B000 | C |
| ATOM | 7491 | O | ASP | E | 266 | 18.516 | 92.408 | -14.481 | 1.00 | 38.07 | B000 | O |
| ATOM | 7492 | CB | ASP | E | 266 | 18.549 | 95.463 | -15.137 | 1.00 | 34.61 | B000 | C |
| ATOM | 7493 | CG | ASP | E | 266 | 20.046 | 95.092 | -15.230 | 1.00 | 42.09 | B000 | C |
| ATOM | 7494 | OD1 | ASP | E | 266 | 20.690 | 94.762 | -14.202 | 1.00 | 40.07 | B000 | O |
| ATOM | 7495 | OD2 | ASP | E | 266 | 20.593 | 95.167 | -16.358 | 1.00 | 40.68 | B000 | O1- |
| ATOM | 7496 | N | ASP | E | 267 | 16.372 | 92.880 | -15.001 | 1.00 | 37.34 | B000 | N |
| ATOM | 7497 | CA | ASP | E | 267 | 16.034 | 91.524 | -15.412 | 1.00 | 36.69 | B000 | C |
| ATOM | 7498 | C | ASP | E | 267 | 15.052 | 91.576 | -16.581 | 1.00 | 35.84 | B000 | C |
| ATOM | 7499 | O | ASP | E | 267 | 14.431 | 92.609 | -16.843 | 1.00 | 37.16 | B000 | O |
| ATOM | 7500 | CB | ASP | E | 267 | 15.448 | 90.748 | -14.230 | 1.00 | 33.55 | B000 | C |
| ATOM | 7501 | CG | ASP | E | 267 | 15.724 | 89.244 | -14.319 | 1.00 | 44.66 | B000 | C |
| ATOM | 7502 | OD1 | ASP | E | 267 | 16.128 | 88.792 | -15.410 | 1.00 | 43.90 | B000 | O |
| ATOM | 7503 | OD2 | ASP | E | 267 | 15.535 | 88.518 | -13.301 | 1.00 | 45.36 | B000 | O1- |
| ATOM | 7504 | N | VAL | E | 268 | 14.901 | 90.455 | -17.294 | 1.00 | 32.21 | B000 | N |
| ATOM | 7505 | CA | VAL | E | 268 | 13.973 | 90.457 | -18.423 | 1.00 | 34.18 | B000 | C |
| ATOM | 7506 | C | VAL | E | 268 | 12.553 | 90.719 | -17.928 | 1.00 | 37.30 | B000 | C |
| ATOM | 7507 | O | VAL | E | 268 | 12.123 | 90.211 | -16.883 | 1.00 | 38.04 | B000 | O |
| ATOM | 7508 | CB | VAL | E | 268 | 14.050 | 89.154 | -19.239 | 1.00 | 37.36 | B000 | C |
| ATOM | 7509 | CG1 | VAL | E | 268 | 15.409 | 89.057 | -19.938 | 1.00 | 36.15 | B000 | C |
| ATOM | 7510 | CG2 | VAL | E | 268 | 13.806 | 87.936 | -18.370 | 1.00 | 35.18 | B000 | C |
| ATOM | 7511 | N | CYS | E | 269 | 11.809 | 91.513 | -18.697 | 1.00 | 36.57 | B000 | N |
| ATOM | 7512 | CA | CYS | E | 269 | 10.538 | 92.045 | -18.240 | 1.00 | 36.70 | B000 | C |
| ATOM | 7513 | C | CYS | E | 269 | 9.449 | 90.995 | -18.103 | 1.00 | 34.59 | B000 | C |
| ATOM | 7514 | O | CYS | E | 269 | 8.418 | 91.294 | -17.500 | 1.00 | 35.91 | B000 | O |
| ATOM | 7515 | CB | CYS | E | 269 | 10.088 | 93.163 | -19.185 | 1.00 | 42.38 | B000 | C |
| ATOM | 7516 | SG | CYS | E | 269 | 11.267 | 94.591 | -19.164 | 1.00 | 53.92 | B000 | S |
| ATOM | 7517 | N | GLN | E | 270 | 9.643 | 89.777 | -18.595 | 1.00 | 33.91 | B000 | N |
| ATOM | 7518 | CA | GLN | E | 270 | 8.591 | 88.778 | -18.422 | 1.00 | 38.11 | B000 | C |
| ATOM | 7519 | C | GLN | E | 270 | 8.652 | 88.057 | -17.086 | 1.00 | 33.78 | B000 | C |
| ATOM | 7520 | O | GLN | E | 270 | 7.760 | 87.247 | -16.828 | 1.00 | 33.42 | B000 | O |

| | | | | | | | | | | | | |
|---|---|---|---|---|---|---|---|---|---|---|---|---|
| ATOM | 7521 | CB | GLN | E | 270 | 8.578 | 87.723 | -19.549 | 1.00 | 38.43 | B000 | C |
| ATOM | 7522 | CG | GLN | E | 270 | 9.815 | 87.602 | -20.390 | 1.00 | 44.86 | B000 | C |
| ATOM | 7523 | CD | GLN | E | 270 | 10.038 | 88.821 | -21.260 | 1.00 | 52.91 | B000 | C |
| ATOM | 7524 | OE1 | GLN | E | 270 | 11.140 | 89.358 | -21.293 | 1.00 | 55.48 | B000 | O |
| ATOM | 7525 | NE2 | GLN | E | 270 | 8.986 | 89.280 | -21.949 | 1.00 | 51.33 | B000 | N |
| ATOM | 7526 | N | ARG | E | 271 | 9.649 | 88.338 | -16.229 | 1.00 | 31.87 | B000 | N |
| ATOM | 7527 | CA | ARG | E | 271 | 9.677 | 87.758 | -14.889 | 1.00 | 29.54 | B000 | C |
| ATOM | 7528 | C | ARG | E | 271 | 8.356 | 88.049 | -14.171 | 1.00 | 36.60 | B000 | C |
| ATOM | 7529 | O | ARG | E | 271 | 7.873 | 89.192 | -14.210 | 1.00 | 35.38 | B000 | O |
| ATOM | 7530 | CB | ARG | E | 271 | 10.829 | 88.325 | -14.050 | 1.00 | 28.67 | B000 | C |
| ATOM | 7531 | CG | ARG | E | 271 | 12.220 | 87.973 | -14.479 | 1.00 | 27.89 | B000 | C |
| ATOM | 7532 | CD | ARG | E | 271 | 12.418 | 86.476 | -14.518 | 1.00 | 31.48 | B000 | C |
| ATOM | 7533 | NE | ARG | E | 271 | 13.822 | 86.098 | -14.676 | 1.00 | 33.37 | B000 | N |
| ATOM | 7534 | CZ | ARG | E | 271 | 14.203 | 84.864 | -14.987 | 1.00 | 37.02 | B000 | C |
| ATOM | 7535 | NH1 | ARG | E | 271 | 13.268 | 83.937 | -15.180 | 1.00 | 30.74 | B000 | N1+ |
| ATOM | 7536 | NH2 | ARG | E | 271 | 15.492 | 84.540 | -15.076 | 1.00 | 29.23 | B000 | N |
| ATOM | 7537 | N | PRO | E | 272 | 7.778 | 87.122 | -13.565 | 1.00 | 34.45 | B000 | N |
| ATOM | 7538 | CA | PRO | E | 272 | 6.493 | 87.373 | -12.870 | 1.00 | 35.22 | B000 | C |
| ATOM | 7539 | C | PRO | E | 272 | 6.670 | 87.925 | -11.455 | 1.00 | 35.16 | B000 | C |
| ATOM | 7540 | O | PRO | E | 272 | 6.234 | 87.321 | -10.470 | 1.00 | 31.89 | B000 | O |
| ATOM | 7541 | CB | PRO | E | 272 | 5.847 | 85.979 | -12.867 | 1.00 | 31.77 | B000 | C |
| ATOM | 7542 | CG | PRO | E | 272 | 7.003 | 85.036 | -12.787 | 1.00 | 32.91 | B000 | C |
| ATOM | 7543 | CD | PRO | E | 272 | 8.114 | 85.689 | -13.621 | 1.00 | 30.82 | B000 | C |
| ATOM | 7544 | N | TYR | E | 273 | 7.295 | 89.101 | -11.344 | 1.00 | 31.62 | B000 | N |
| ATOM | 7545 | CA | TYR | E | 273 | 7.555 | 89.672 | -10.028 | 1.00 | 32.44 | B000 | C |
| ATOM | 7546 | C | TYR | E | 273 | 6.327 | 90.398 | -9.474 | 1.00 | 33.52 | B000 | C |
| ATOM | 7547 | O | TYR | E | 273 | 5.355 | 90.667 | -10.183 | 1.00 | 33.94 | B000 | O |
| ATOM | 7548 | CB | TYR | E | 273 | 8.752 | 90.615 | -10.089 | 1.00 | 32.72 | B000 | C |
| ATOM | 7549 | CG | TYR | E | 273 | 10.050 | 89.893 | -10.371 | 1.00 | 34.81 | B000 | C |
| ATOM | 7550 | CD1 | TYR | E | 273 | 10.215 | 88.558 | -10.004 | 1.00 | 30.34 | B000 | C |
| ATOM | 7551 | CD2 | TYR | E | 273 | 11.125 | 90.546 | -10.996 | 1.00 | 34.47 | B000 | C |
| ATOM | 7552 | CE1 | TYR | E | 273 | 11.428 | 87.883 | -10.249 | 1.00 | 31.47 | B000 | C |
| ATOM | 7553 | CE2 | TYR | E | 273 | 12.329 | 89.879 | -11.245 | 1.00 | 31.66 | B000 | C |
| ATOM | 7554 | CZ | TYR | E | 273 | 12.468 | 88.549 | -10.867 | 1.00 | 32.58 | B000 | C |
| ATOM | 7555 | OH | TYR | E | 273 | 13.639 | 87.883 | -11.117 | 1.00 | 37.35 | B000 | O |
| ATOM | 7556 | N | ARG | E | 274 | 6.356 | 90.671 | -8.170 | 1.00 | 34.37 | B000 | N |
| ATOM | 7557 | CA | ARG | E | 274 | 5.350 | 91.549 | -7.579 | 1.00 | 32.60 | B000 | C |
| ATOM | 7558 | C | ARG | E | 274 | 5.542 | 92.978 | -8.089 | 1.00 | 35.51 | B000 | C |
| ATOM | 7559 | O | ARG | E | 274 | 6.533 | 93.300 | -8.757 | 1.00 | 31.65 | B000 | O |
| ATOM | 7560 | CB | ARG | E | 274 | 5.426 | 91.519 | -6.048 | 1.00 | 31.74 | B000 | C |
| ATOM | 7561 | CG | ARG | E | 274 | 5.044 | 90.177 | -5.455 | 1.00 | 35.22 | B000 | C |
| ATOM | 7562 | CD | ARG | E | 274 | 4.922 | 90.200 | -3.952 | 1.00 | 36.81 | B000 | C |
| ATOM | 7563 | NE | ARG | E | 274 | 4.320 | 88.966 | -3.474 | 1.00 | 36.66 | B000 | N |
| ATOM | 7564 | CZ | ARG | E | 274 | 4.010 | 88.714 | -2.206 | 1.00 | 38.82 | B000 | C |
| ATOM | 7565 | NH1 | ARG | E | 274 | 4.266 | 89.605 | -1.260 | 1.00 | 40.20 | B000 | N1+ |
| ATOM | 7566 | NH2 | ARG | E | 274 | 3.432 | 87.563 | -1.886 | 1.00 | 37.93 | B000 | N |
| ATOM | 7567 | N | TRP | E | 275 | 4.591 | 93.857 | -7.757 | 1.00 | 34.71 | B000 | N |
| ATOM | 7568 | CA | TRP | E | 275 | 4.709 | 95.242 | -8.207 | 1.00 | 35.25 | B000 | C |
| ATOM | 7569 | C | TRP | E | 275 | 4.038 | 96.177 | -7.209 | 1.00 | 33.77 | B000 | C |
| ATOM | 7570 | O | TRP | E | 275 | 3.288 | 95.762 | -6.316 | 1.00 | 34.64 | B000 | O |
| ATOM | 7571 | CB | TRP | E | 275 | 4.133 | 95.429 | -9.622 | 1.00 | 29.33 | B000 | C |
| ATOM | 7572 | CG | TRP | E | 275 | 2.633 | 95.433 | -9.666 | 1.00 | 41.26 | B000 | C |
| ATOM | 7573 | CD1 | TRP | E | 275 | 1.824 | 96.531 | -9.687 | 1.00 | 32.79 | B000 | C |
| ATOM | 7574 | CD2 | TRP | E | 275 | 1.762 | 94.290 | -9.659 | 1.00 | 35.30 | B000 | C |
| ATOM | 7575 | NE1 | TRP | E | 275 | 0.516 | 96.147 | -9.707 | 1.00 | 34.43 | B000 | N |
| ATOM | 7576 | CE2 | TRP | E | 275 | 0.441 | 94.779 | -9.687 | 1.00 | 41.97 | B000 | C |
| ATOM | 7577 | CE3 | TRP | E | 275 | 1.971 | 92.908 | -9.650 | 1.00 | 34.18 | B000 | C |
| ATOM | 7578 | CZ2 | TRP | E | 275 | -0.681 | 93.928 | -9.698 | 1.00 | 39.53 | B000 | C |
| ATOM | 7579 | CZ3 | TRP | E | 275 | 0.861 | 92.059 | -9.654 | 1.00 | 40.96 | B000 | C |
| ATOM | 7580 | CH2 | TRP | E | 275 | -0.445 | 92.574 | -9.680 | 1.00 | 40.67 | B000 | C |
| ATOM | 7581 | N | VAL | E | 276 | 4.345 | 97.453 | -7.367 | 1.00 | 32.75 | B000 | N |
| ATOM | 7582 | CA | VAL | E | 276 | 3.827 | 98.520 | -6.528 | 1.00 | 36.54 | B000 | C |
| ATOM | 7583 | C | VAL | E | 276 | 3.248 | 99.578 | -7.451 | 1.00 | 43.35 | B000 | C |
| ATOM | 7584 | O | VAL | E | 276 | 3.916 | 99.998 | -8.404 | 1.00 | 39.24 | B000 | O |
| ATOM | 7585 | CB | VAL | E | 276 | 4.932 | 99.125 | -5.636 | 1.00 | 39.71 | B000 | C |
| ATOM | 7586 | CG1 | VAL | E | 276 | 4.364 | 100.226 | -4.708 | 1.00 | 36.15 | B000 | C |
| ATOM | 7587 | CG2 | VAL | E | 276 | 5.626 | 98.033 | -4.830 | 1.00 | 33.07 | B000 | C |
| ATOM | 7588 | N | CYS | E | 277 | 2.015 | 100.004 | -7.176 | 1.00 | 41.27 | B000 | N |
| ATOM | 7589 | CA | CYS | E | 277 | 1.403 | 101.127 | -7.878 | 1.00 | 39.77 | B000 | C |
| ATOM | 7590 | C | CYS | E | 277 | 1.513 | 102.387 | -7.022 | 1.00 | 46.66 | B000 | C |
| ATOM | 7591 | O | CYS | E | 277 | 1.519 | 102.321 | -5.786 | 1.00 | 41.72 | B000 | O |

```
ATOM   7592  CB  CYS E 277      -0.063 100.847  -8.209  1.00 41.42      B000 C
ATOM   7593  SG  CYS E 277      -0.364  99.509  -9.393  1.00 51.15      B000 S
ATOM   7594  N   GLU E 278       1.622 103.536  -7.698  1.00 43.54      B000 N
ATOM   7595  CA  GLU E 278       1.776 104.841  -7.069  1.00 38.79      B000 C
ATOM   7596  C   GLU E 278       0.862 105.858  -7.751  1.00 45.95      B000 C
ATOM   7597  O   GLU E 278       0.782 105.914  -8.988  1.00 39.44      B000 O
ATOM   7598  CB  GLU E 278       3.243 105.302  -7.137  1.00 47.37      B000 C
ATOM   7599  CG  GLU E 278       3.523 106.665  -6.506  1.00 54.07      B000 C
ATOM   7600  CD  GLU E 278       4.983 107.122  -6.640  1.00 55.74      B000 C
ATOM   7601  OE1 GLU E 278       5.813 106.384  -7.214  1.00 49.09      B000 O
ATOM   7602  OE2 GLU E 278       5.299 108.234  -6.158  1.00 63.05      B000 O1-
ATOM   7603  N   THR E 279       0.194 106.677  -6.933  1.00 44.07      B000 N
ATOM   7604  CA  THR E 279      -0.653 107.764  -7.416  1.00 49.75      B000 C
ATOM   7605  C   THR E 279      -0.567 108.944  -6.440  1.00 52.93      B000 C
ATOM   7606  O   THR E 279       0.043 108.854  -5.367  1.00 51.44      B000 O
ATOM   7607  CB  THR E 279      -2.103 107.288  -7.604  1.00 51.62      B000 C
ATOM   7608  OG1 THR E 279      -2.814 108.206  -8.438  1.00 58.36      B000 O
ATOM   7609  CG2 THR E 279      -2.815 107.204  -6.271  1.00 48.77      B000 C
ATOM   7610  N   GLU E 280      -1.195 110.058  -6.818  1.00 57.24      B000 N
ATOM   7611  CA  GLU E 280      -1.083 111.317  -6.090  1.00 59.43      B000 C
ATOM   7612  C   GLU E 280      -2.071 111.410  -4.922  1.00 60.25      B000 C
ATOM   7613  O   GLU E 280      -2.950 110.567  -4.738  1.00 57.16      B000 O
ATOM   7614  CB  GLU E 280      -1.329 112.479  -7.040  1.00 72.93      B000 C
ATOM   7615  CG  GLU E 280      -1.062 112.139  -8.487  1.00 81.08      B000 C
ATOM   7616  CD  GLU E 280      -0.073 113.097  -9.119  1.00105.55      B000 C
ATOM   7617  OE1 GLU E 280      -0.010 114.263  -8.665  1.00104.59      B000 O
ATOM   7618  OE2 GLU E 280       0.648 112.680 -10.055  1.00113.25      B000 O1-
ATOM   7619  N   LEU E 281      -1.917 112.479  -4.135  1.00 77.88      B000 N
ATOM   7620  CA  LEU E 281      -2.807 112.826  -3.011  1.00 70.25      B000 C
ATOM   7621  C   LEU E 281      -2.681 111.825  -1.876  1.00 67.47      B000 C
ATOM   7622  O   LEU E 281      -2.139 112.153  -0.819  1.00 68.88      B000 O
ATOM   7623  CB  LEU E 281      -4.279 112.937  -3.459  1.00 61.71      B000 C
ATOM   7624  CG  LEU E 281      -4.707 114.296  -4.035  1.00 80.45      B000 C
ATOM   7625  CD1 LEU E 281      -5.181 114.179  -5.490  1.00 71.81      B000 C
ATOM   7626  CD2 LEU E 281      -5.774 114.969  -3.143  1.00 70.21      B000 C
TER
ATOM   7627  O   THR F 152     -18.909  43.540  -2.518  1.00 85.67      B000 O
ATOM   7628  N   THR F 152     -17.763  41.399  -0.880  1.00 85.67      B000 N
ATOM   7629  CA  THR F 152     -16.968  42.333  -1.678  1.00 88.53      B000 C
ATOM   7630  C   THR F 152     -17.777  43.608  -2.017  1.00 91.37      B000 C
ATOM   7631  CB  THR F 152     -16.431  41.637  -2.979  1.00 92.20      B000 C
ATOM   7632  OG1 THR F 152     -15.806  42.597  -3.844  1.00 90.90      B000 O
ATOM   7633  CG2 THR F 152     -17.541  40.894  -3.731  1.00 84.33      B000 C
ATOM   7634  N   CYS F 153     -17.202  44.774  -1.715  1.00 87.63      B000 N
ATOM   7635  CA  CYS F 153     -17.870  46.050  -1.926  1.00 82.80      B000 C
ATOM   7636  C   CYS F 153     -17.103  46.899  -2.940  1.00 77.69      B000 C
ATOM   7637  O   CYS F 153     -15.918  46.673  -3.212  1.00 67.09      B000 O
ATOM   7638  CB  CYS F 153     -18.029  46.817  -0.596  1.00 79.27      B000 C
ATOM   7639  SG  CYS F 153     -19.643  46.575   0.239  1.00104.11      B000 S
ATOM   7640  N   CYS F 154     -17.803  47.886  -3.508  1.00 76.91      B000 N
ATOM   7641  CA  CYS F 154     -17.185  48.793  -4.462  1.00 59.34      B000 C
ATOM   7642  C   CYS F 154     -16.202  49.711  -3.745  1.00 54.16      B000 C
ATOM   7643  O   CYS F 154     -16.398  50.041  -2.575  1.00 63.36      B000 O
ATOM   7644  CB  CYS F 154     -18.244  49.634  -5.172  1.00 55.19      B000 C
ATOM   7645  SG  CYS F 154     -19.186  48.756  -6.443  1.00 69.85      B000 S
ATOM   7646  N   PRO F 155     -15.156  50.163  -4.429  1.00 53.28      B000 N
ATOM   7647  CA  PRO F 155     -14.236  51.130  -3.812  1.00 56.60      B000 C
ATOM   7648  C   PRO F 155     -14.973  52.379  -3.346  1.00 57.95      B000 C
ATOM   7649  O   PRO F 155     -16.130  52.640  -3.696  1.00 55.60      B000 O
ATOM   7650  CB  PRO F 155     -13.243  51.459  -4.932  1.00 48.00      B000 C
ATOM   7651  CG  PRO F 155     -13.374  50.336  -5.913  1.00 55.49      B000 C
ATOM   7652  CD  PRO F 155     -14.777  49.826  -5.811  1.00 51.46      B000 C
ATOM   7653  N   VAL F 156     -14.268  53.174  -2.539  1.00 56.46      B000 N
ATOM   7654  CA  VAL F 156     -14.855  54.394  -1.991  1.00 63.80      B000 C
ATOM   7655  C   VAL F 156     -15.250  55.333  -3.126  1.00 59.48      B000 C
ATOM   7656  O   VAL F 156     -14.488  55.538  -4.081  1.00 58.09      B000 O
ATOM   7657  CB  VAL F 156     -13.873  55.058  -1.014  1.00 62.21      B000 C
ATOM   7658  CG1 VAL F 156     -14.577  56.145  -0.211  1.00 55.24      B000 C
ATOM   7659  CG2 VAL F 156     -13.256  54.002  -0.096  1.00 73.19      B000 C
ATOM   7660  N   ASN F 157     -16.460  55.891  -3.031  1.00 53.19      B000 N
ATOM   7661  CA  ASN F 157     -17.093  56.831  -3.964  1.00 54.02      B000 C
```

| | | | | | | | | | | | | |
|---|---|---|---|---|---|---|---|---|---|---|---|---|
| ATOM | 7662 | C | ASN | F | 157 | -17.605 | 56.142 | -5.227 | 1.00 | 54.32 | B000 | C |
| ATOM | 7663 | O | ASN | F | 157 | -18.209 | 56.814 | -6.072 | 1.00 | 53.93 | B000 | O |
| ATOM | 7664 | CB | ASN | F | 157 | -16.170 | 57.985 | -4.387 | 1.00 | 49.92 | B000 | C |
| ATOM | 7665 | CG | ASN | F | 157 | -15.632 | 58.759 | -3.202 | 1.00 | 59.88 | B000 | C |
| ATOM | 7666 | OD1 | ASN | F | 157 | -16.357 | 59.008 | -2.236 | 1.00 | 56.12 | B000 | O |
| ATOM | 7667 | ND2 | ASN | F | 157 | -14.346 | 59.136 | -3.262 | 1.00 | 53.76 | B000 | N |
| ATOM | 7668 | N | TRP | F | 158 | -17.394 | 54.844 | -5.396 | 1.00 | 49.14 | B000 | N |
| ATOM | 7669 | CA | TRP | F | 158 | -18.015 | 54.160 | -6.516 | 1.00 | 47.06 | B000 | C |
| ATOM | 7670 | C | TRP | F | 158 | -19.353 | 53.577 | -6.080 | 1.00 | 46.07 | B000 | C |
| ATOM | 7671 | O | TRP | F | 158 | -19.629 | 53.429 | -4.891 | 1.00 | 48.01 | B000 | O |
| ATOM | 7672 | CB | TRP | F | 158 | -17.109 | 53.069 | -7.059 | 1.00 | 48.92 | B000 | C |
| ATOM | 7673 | CG | TRP | F | 158 | -15.830 | 53.586 | -7.632 | 1.00 | 42.48 | B000 | C |
| ATOM | 7674 | CD1 | TRP | F | 158 | -14.854 | 54.269 | -6.967 | 1.00 | 46.04 | B000 | C |
| ATOM | 7675 | CD2 | TRP | F | 158 | -15.364 | 53.425 | -8.975 | 1.00 | 40.30 | B000 | C |
| ATOM | 7676 | NE1 | TRP | F | 158 | -13.811 | 54.549 | -7.812 | 1.00 | 40.04 | B000 | N |
| ATOM | 7677 | CE2 | TRP | F | 158 | -14.098 | 54.047 | -9.053 | 1.00 | 40.63 | B000 | C |
| ATOM | 7678 | CE3 | TRP | F | 158 | -15.900 | 52.830 | -10.124 | 1.00 | 38.93 | B000 | C |
| ATOM | 7679 | CZ2 | TRP | F | 158 | -13.358 | 54.092 | -10.234 | 1.00 | 36.03 | B000 | C |
| ATOM | 7680 | CZ3 | TRP | F | 158 | -15.164 | 52.864 | -11.283 | 1.00 | 40.94 | B000 | C |
| ATOM | 7681 | CH2 | TRP | F | 158 | -13.908 | 53.499 | -11.336 | 1.00 | 38.94 | B000 | C |
| ATOM | 7682 | N | VAL | F | 159 | -20.207 | 53.303 | -7.065 | 1.00 | 48.15 | B000 | N |
| ATOM | 7683 | CA | VAL | F | 159 | -21.584 | 52.872 | -6.854 | 1.00 | 43.77 | B000 | C |
| ATOM | 7684 | C | VAL | F | 159 | -21.777 | 51.526 | -7.536 | 1.00 | 53.64 | B000 | C |
| ATOM | 7685 | O | VAL | F | 159 | -21.317 | 51.328 | -8.666 | 1.00 | 50.16 | B000 | O |
| ATOM | 7686 | CB | VAL | F | 159 | -22.571 | 53.918 | -7.409 | 1.00 | 47.04 | B000 | C |
| ATOM | 7687 | CG1 | VAL | F | 159 | -24.008 | 53.459 | -7.275 | 1.00 | 49.61 | B000 | C |
| ATOM | 7688 | CG2 | VAL | F | 159 | -22.361 | 55.239 | -6.711 | 1.00 | 48.78 | B000 | C |
| ATOM | 7689 | N | GLU | F | 160 | -22.478 | 50.611 | -6.864 | 1.00 | 57.06 | B000 | N |
| ATOM | 7690 | CA | GLU | F | 160 | -22.632 | 49.242 | -7.343 | 1.00 | 56.04 | B000 | C |
| ATOM | 7691 | C | GLU | F | 160 | -23.958 | 49.061 | -8.070 | 1.00 | 50.46 | B000 | C |
| ATOM | 7692 | O | GLU | F | 160 | -25.000 | 49.538 | -7.619 | 1.00 | 54.39 | B000 | O |
| ATOM | 7693 | CB | GLU | F | 160 | -22.568 | 48.223 | -6.201 | 1.00 | 59.56 | B000 | C |
| ATOM | 7694 | CG | GLU | F | 160 | -22.813 | 46.795 | -6.710 | 1.00 | 68.99 | B000 | C |
| ATOM | 7695 | CD | GLU | F | 160 | -23.041 | 45.748 | -5.621 | 1.00 | 80.25 | B000 | C |
| ATOM | 7696 | OE1 | GLU | F | 160 | -22.777 | 46.016 | -4.427 | 1.00 | 79.75 | B000 | O |
| ATOM | 7697 | OE2 | GLU | F | 160 | -23.491 | 44.637 | -5.985 | 1.00 | 78.26 | B000 | O1- |
| ATOM | 7698 | N | HIS | F | 161 | -23.912 | 48.328 | -9.175 | 1.00 | 51.49 | B000 | N |
| ATOM | 7699 | CA | HIS | F | 161 | -25.104 | 47.913 | -9.892 | 1.00 | 49.49 | B000 | C |
| ATOM | 7700 | C | HIS | F | 161 | -24.786 | 46.601 | -10.587 | 1.00 | 53.28 | B000 | C |
| ATOM | 7701 | O | HIS | F | 161 | -23.801 | 46.508 | -11.332 | 1.00 | 47.92 | B000 | O |
| ATOM | 7702 | CB | HIS | F | 161 | -25.561 | 48.959 | -10.901 | 1.00 | 47.30 | B000 | C |
| ATOM | 7703 | CG | HIS | F | 161 | -26.699 | 48.503 | -11.759 | 1.00 | 52.32 | B000 | C |
| ATOM | 7704 | ND1 | HIS | F | 161 | -26.511 | 47.794 | -12.930 | 1.00 | 49.15 | B000 | N |
| ATOM | 7705 | CD2 | HIS | F | 161 | -28.039 | 48.641 | -11.611 | 1.00 | 49.74 | B000 | C |
| ATOM | 7706 | CE1 | HIS | F | 161 | -27.687 | 47.540 | -13.478 | 1.00 | 52.61 | B000 | C |
| ATOM | 7707 | NE2 | HIS | F | 161 | -28.630 | 48.037 | -12.696 | 1.00 | 54.26 | B000 | N |
| ATOM | 7708 | N | GLU | F | 162 | -25.666 | 45.618 | -10.386 | 1.00 | 61.24 | B000 | N |
| ATOM | 7709 | CA | GLU | F | 162 | -25.426 | 44.226 | -10.751 | 1.00 | 57.31 | B000 | C |
| ATOM | 7710 | C | GLU | F | 162 | -24.028 | 43.809 | -10.315 | 1.00 | 59.22 | B000 | C |
| ATOM | 7711 | O | GLU | F | 162 | -23.715 | 43.833 | -9.118 | 1.00 | 59.32 | B000 | O |
| ATOM | 7712 | CB | GLU | F | 162 | -25.596 | 44.005 | -12.253 | 1.00 | 54.87 | B000 | C |
| ATOM | 7713 | CG | GLU | F | 162 | -26.901 | 44.546 | -12.835 | 1.00 | 59.80 | B000 | C |
| ATOM | 7714 | CD | GLU | F | 162 | -28.197 | 43.921 | -12.303 | 1.00 | 84.54 | B000 | C |
| ATOM | 7715 | OE1 | GLU | F | 162 | -28.210 | 43.290 | -11.211 | 1.00 | 82.94 | B000 | O |
| ATOM | 7716 | OE2 | GLU | F | 162 | -29.231 | 44.078 | -13.007 | 1.00 | 83.83 | B000 | O1- |
| ATOM | 7717 | N | ARG | F | 163 | -23.163 | 43.459 | -11.263 | 1.00 | 55.56 | B000 | N |
| ATOM | 7718 | CA | ARG | F | 163 | -21.811 | 43.028 | -10.919 | 1.00 | 70.50 | B000 | C |
| ATOM | 7719 | C | ARG | F | 163 | -20.730 | 44.041 | -11.292 | 1.00 | 65.87 | B000 | C |
| ATOM | 7720 | O | ARG | F | 163 | -19.541 | 43.699 | -11.301 | 1.00 | 61.99 | B000 | O |
| ATOM | 7721 | CB | ARG | F | 163 | -21.533 | 41.645 | -11.517 | 1.00 | 71.43 | B000 | C |
| ATOM | 7722 | CG | ARG | F | 163 | -22.636 | 40.621 | -11.170 | 1.00 | 84.91 | B000 | C |
| ATOM | 7723 | CD | ARG | F | 163 | -23.371 | 40.027 | -12.366 | 1.00 | 94.82 | B000 | C |
| ATOM | 7724 | NE | ARG | F | 163 | -23.213 | 38.571 | -12.447 | 1.00110.28 | | B000 | N |
| ATOM | 7725 | CZ | ARG | F | 163 | -22.337 | 37.951 | -13.238 | 1.00109.36 | | B000 | C |
| ATOM | 7726 | NH1 | ARG | F | 163 | -21.538 | 38.656 | -14.031 | 1.00107.55 | | B000 | N1+ |
| ATOM | 7727 | NH2 | ARG | F | 163 | -22.266 | 36.625 | -13.245 | 1.00108.51 | | B000 | N |
| ATOM | 7728 | N | SER | F | 164 | -21.104 | 45.289 | -11.551 | 1.00 | 62.99 | B000 | N |
| ATOM | 7729 | CA | SER | F | 164 | -20.137 | 46.326 | -11.872 | 1.00 | 54.70 | B000 | C |
| ATOM | 7730 | C | SER | F | 164 | -20.163 | 47.454 | -10.845 | 1.00 | 52.59 | B000 | C |
| ATOM | 7731 | O | SER | F | 164 | -21.153 | 47.666 | -10.136 | 1.00 | 53.73 | B000 | O |
| ATOM | 7732 | CB | SER | F | 164 | -20.405 | 46.896 | -13.272 | 1.00 | 56.24 | B000 | C |

| | | | | | | | | | | | | |
|---|---|---|---|---|---|---|---|---|---|---|---|---|
| ATOM | 7733 | OG | SER | F | 164 | -19.909 | 46.041 | -14.287 | 1.00 | 65.36 | B000 | O |
| ATOM | 7734 | N | CYS | F | 165 | -19.036 | 48.161 | -10.771 | 1.00 | 51.69 | B000 | N |
| ATOM | 7735 | CA | CYS | F | 165 | -18.866 | 49.373 | -9.981 | 1.00 | 51.61 | B000 | C |
| ATOM | 7736 | C | CYS | F | 165 | -18.723 | 50.574 | -10.909 | 1.00 | 50.55 | B000 | C |
| ATOM | 7737 | O | CYS | F | 165 | -18.004 | 50.511 | -11.912 | 1.00 | 48.71 | B000 | O |
| ATOM | 7738 | CB | CYS | F | 165 | -17.633 | 49.280 | -9.076 | 1.00 | 55.01 | B000 | C |
| ATOM | 7739 | SG | CYS | F | 165 | -17.771 | 48.053 | -7.751 | 1.00 | 71.45 | B000 | S |
| ATOM | 7740 | N | TYR | F | 166 | -19.410 | 51.670 | -10.577 | 1.00 | 48.64 | B000 | N |
| ATOM | 7741 | CA | TYR | F | 166 | -19.481 | 52.838 | -11.449 | 1.00 | 45.58 | B000 | C |
| ATOM | 7742 | C | TYR | F | 166 | -19.040 | 54.104 | -10.722 | 1.00 | 51.09 | B000 | C |
| ATOM | 7743 | O | TYR | F | 166 | -19.325 | 54.283 | -9.533 | 1.00 | 49.78 | B000 | O |
| ATOM | 7744 | CB | TYR | F | 166 | -20.886 | 53.045 | -11.982 | 1.00 | 39.51 | B000 | C |
| ATOM | 7745 | CG | TYR | F | 166 | -21.427 | 51.889 | -12.777 | 1.00 | 45.13 | B000 | C |
| ATOM | 7746 | CD1 | TYR | F | 166 | -21.884 | 50.732 | -12.151 | 1.00 | 50.08 | B000 | C |
| ATOM | 7747 | CD2 | TYR | F | 166 | -21.471 | 51.945 | -14.159 | 1.00 | 46.42 | B000 | C |
| ATOM | 7748 | CE1 | TYR | F | 166 | -22.393 | 49.667 | -12.887 | 1.00 | 48.67 | B000 | C |
| ATOM | 7749 | CE2 | TYR | F | 166 | -21.968 | 50.892 | -14.902 | 1.00 | 52.75 | B000 | C |
| ATOM | 7750 | CZ | TYR | F | 166 | -22.429 | 49.752 | -14.264 | 1.00 | 51.87 | B000 | C |
| ATOM | 7751 | OH | TYR | F | 166 | -22.922 | 48.712 | -15.024 | 1.00 | 47.43 | B000 | O |
| ATOM | 7752 | N | TRP | F | 167 | -18.358 | 54.989 | -11.446 | 1.00 | 41.54 | B000 | N |
| ATOM | 7753 | CA | TRP | F | 167 | -17.941 | 56.279 | -10.914 | 1.00 | 39.17 | B000 | C |
| ATOM | 7754 | C | TRP | F | 167 | -18.472 | 57.396 | -11.821 | 1.00 | 40.61 | B000 | C |
| ATOM | 7755 | O | TRP | F | 167 | -18.300 | 57.352 | -13.048 | 1.00 | 39.85 | B000 | O |
| ATOM | 7756 | CB | TRP | F | 167 | -16.408 | 56.328 | -10.776 | 1.00 | 39.26 | B000 | C |
| ATOM | 7757 | CG | TRP | F | 167 | -15.921 | 57.583 | -10.131 | 1.00 | 47.81 | B000 | C |
| ATOM | 7758 | CD1 | TRP | F | 167 | -15.728 | 57.812 | -8.787 | 1.00 | 43.33 | B000 | C |
| ATOM | 7759 | CD2 | TRP | F | 167 | -15.599 | 58.806 | -10.799 | 1.00 | 36.88 | B000 | C |
| ATOM | 7760 | NE1 | TRP | F | 167 | -15.292 | 59.110 | -8.588 | 1.00 | 37.39 | B000 | N |
| ATOM | 7761 | CE2 | TRP | F | 167 | -15.191 | 59.735 | -9.807 | 1.00 | 42.60 | B000 | C |
| ATOM | 7762 | CE3 | TRP | F | 167 | -15.593 | 59.202 | -12.140 | 1.00 | 35.30 | B000 | C |
| ATOM | 7763 | CZ2 | TRP | F | 167 | -14.796 | 61.049 | -10.122 | 1.00 | 32.52 | B000 | C |
| ATOM | 7764 | CZ3 | TRP | F | 167 | -15.177 | 60.506 | -12.455 | 1.00 | 41.65 | B000 | C |
| ATOM | 7765 | CH2 | TRP | F | 167 | -14.805 | 61.415 | -11.442 | 1.00 | 34.25 | B000 | C |
| ATOM | 7766 | N | PHE | F | 168 | -19.128 | 58.386 | -11.215 | 1.00 | 32.85 | B000 | N |
| ATOM | 7767 | CA | PHE | F | 168 | -19.816 | 59.459 | -11.933 | 1.00 | 36.14 | B000 | C |
| ATOM | 7768 | C | PHE | F | 168 | -19.062 | 60.768 | -11.757 | 1.00 | 33.17 | B000 | C |
| ATOM | 7769 | O | PHE | F | 168 | -18.933 | 61.266 | -10.640 | 1.00 | 32.62 | B000 | O |
| ATOM | 7770 | CB | PHE | F | 168 | -21.257 | 59.611 | -11.439 | 1.00 | 31.85 | B000 | C |
| ATOM | 7771 | CG | PHE | F | 168 | -22.092 | 58.410 | -11.718 | 1.00 | 43.90 | B000 | C |
| ATOM | 7772 | CD1 | PHE | F | 168 | -22.788 | 58.303 | -12.918 | 1.00 | 37.94 | B000 | C |
| ATOM | 7773 | CD2 | PHE | F | 168 | -22.121 | 57.349 | -10.824 | 1.00 | 37.92 | B000 | C |
| ATOM | 7774 | CE1 | PHE | F | 168 | -23.534 | 57.188 | -13.204 | 1.00 | 33.97 | B000 | C |
| ATOM | 7775 | CE2 | PHE | F | 168 | -22.863 | 56.231 | -11.102 | 1.00 | 43.27 | B000 | C |
| ATOM | 7776 | CZ | PHE | F | 168 | -23.576 | 56.147 | -12.299 | 1.00 | 39.89 | B000 | C |
| ATOM | 7777 | N | SER | F | 169 | -18.544 | 61.311 | -12.851 | 1.00 | 34.64 | B000 | N |
| ATOM | 7778 | CA | SER | F | 169 | -17.896 | 62.613 | -12.753 | 1.00 | 37.47 | B000 | C |
| ATOM | 7779 | C | SER | F | 169 | -18.933 | 63.691 | -12.453 | 1.00 | 35.48 | B000 | C |
| ATOM | 7780 | O | SER | F | 169 | -20.132 | 63.531 | -12.708 | 1.00 | 35.12 | B000 | O |
| ATOM | 7781 | CB | SER | F | 169 | -17.154 | 62.974 | -14.046 | 1.00 | 31.74 | B000 | C |
| ATOM | 7782 | OG | SER | F | 169 | -18.061 | 63.533 | -14.999 | 1.00 | 33.74 | B000 | O |
| ATOM | 7783 | N | ARG | F | 170 | -18.454 | 64.810 | -11.921 | 1.00 | 32.66 | B000 | N |
| ATOM | 7784 | CA | ARG | F | 170 | -19.292 | 65.983 | -11.718 | 1.00 | 35.64 | B000 | C |
| ATOM | 7785 | C | ARG | F | 170 | -18.692 | 67.198 | -12.423 | 1.00 | 37.46 | B000 | C |
| ATOM | 7786 | O | ARG | F | 170 | -18.918 | 68.347 | -12.037 | 1.00 | 32.51 | B000 | O |
| ATOM | 7787 | CB | ARG | F | 170 | -19.527 | 66.211 | -10.228 | 1.00 | 30.96 | B000 | C |
| ATOM | 7788 | CG | ARG | F | 170 | -20.457 | 65.128 | -9.645 | 1.00 | 33.93 | B000 | C |
| ATOM | 7789 | CD | ARG | F | 170 | -20.628 | 65.237 | -8.142 | 1.00 | 36.53 | B000 | C |
| ATOM | 7790 | NE | ARG | F | 170 | -19.466 | 64.712 | -7.440 | 1.00 | 46.87 | B000 | N |
| ATOM | 7791 | CZ | ARG | F | 170 | -19.272 | 64.766 | -6.121 | 1.00 | 50.98 | B000 | C |
| ATOM | 7792 | NH1 | ARG | F | 170 | -18.153 | 64.253 | -5.600 | 1.00 | 44.20 | B000 | N1+ |
| ATOM | 7793 | NH2 | ARG | F | 170 | -20.184 | 65.325 | -5.321 | 1.00 | 43.86 | B000 | N |
| ATOM | 7794 | N | SER | F | 171 | -17.935 | 66.933 | -13.484 | 1.00 | 32.24 | B000 | N |
| ATOM | 7795 | CA | SER | F | 171 | -17.355 | 67.964 | -14.328 | 1.00 | 32.82 | B000 | C |
| ATOM | 7796 | C | SER | F | 171 | -17.068 | 67.317 | -15.673 | 1.00 | 33.70 | B000 | C |
| ATOM | 7797 | O | SER | F | 171 | -17.093 | 66.090 | -15.807 | 1.00 | 32.42 | B000 | O |
| ATOM | 7798 | CB | SER | F | 171 | -16.078 | 68.565 | -13.724 | 1.00 | 32.95 | B000 | C |
| ATOM | 7799 | OG | SER | F | 171 | -15.041 | 67.584 | -13.595 | 1.00 | 37.93 | B000 | O |
| ATOM | 7800 | N | GLY | F | 172 | -16.766 | 68.160 | -16.662 | 1.00 | 27.87 | B000 | N |
| ATOM | 7801 | CA | GLY | F | 172 | -16.607 | 67.725 | -18.032 | 1.00 | 30.72 | B000 | C |
| ATOM | 7802 | C | GLY | F | 172 | -15.165 | 67.477 | -18.471 | 1.00 | 37.04 | B000 | C |
| ATOM | 7803 | O | GLY | F | 172 | -14.203 | 68.006 | -17.891 | 1.00 | 31.19 | B000 | O |

| | | | | | | | | | | | | |
|---|---|---|---|---|---|---|---|---|---|---|---|---|
| ATOM | 7804 | N | LYS | F | 173 | -15.039 | 66.642 | -19.510 | 1.00 | 31.65 | B000 | N |
| ATOM | 7805 | CA | LYS | F | 173 | -13.782 | 66.382 | -20.205 | 1.00 | 34.23 | B000 | C |
| ATOM | 7806 | C | LYS | F | 173 | -14.091 | 66.048 | -21.659 | 1.00 | 34.10 | B000 | C |
| ATOM | 7807 | O | LYS | F | 173 | -15.137 | 65.468 | -21.967 | 1.00 | 27.67 | B000 | O |
| ATOM | 7808 | CB | LYS | F | 173 | -12.986 | 65.212 | -19.599 | 1.00 | 36.67 | B000 | C |
| ATOM | 7809 | CG | LYS | F | 173 | -12.212 | 65.491 | -18.319 | 1.00 | 36.30 | B000 | C |
| ATOM | 7810 | CD | LYS | F | 173 | -11.406 | 64.251 | -17.943 | 1.00 | 31.78 | B000 | C |
| ATOM | 7811 | CE | LYS | F | 173 | -10.707 | 64.369 | -16.569 | 1.00 | 33.56 | B000 | C |
| ATOM | 7812 | NZ | LYS | F | 173 | -9.556 | 65.313 | -16.523 | 1.00 | 45.73 | B000 | N1+ |
| ATOM | 7813 | N | ALA | F | 174 | -13.167 | 66.400 | -22.557 | 1.00 | 31.22 | B000 | N |
| ATOM | 7814 | CA | ALA | F | 174 | -13.226 | 65.821 | -23.890 | 1.00 | 31.29 | B000 | C |
| ATOM | 7815 | C | ALA | F | 174 | -13.149 | 64.297 | -23.774 | 1.00 | 30.96 | B000 | C |
| ATOM | 7816 | O | ALA | F | 174 | -12.525 | 63.758 | -22.851 | 1.00 | 33.23 | B000 | O |
| ATOM | 7817 | CB | ALA | F | 174 | -12.099 | 66.364 | -24.763 | 1.00 | 29.41 | B000 | C |
| ATOM | 7818 | N | TRP | F | 175 | -13.823 | 63.602 | -24.698 | 1.00 | 28.04 | B000 | N |
| ATOM | 7819 | CA | TRP | F | 175 | -13.972 | 62.145 | -24.586 | 1.00 | 34.38 | B000 | C |
| ATOM | 7820 | C | TRP | F | 175 | -12.623 | 61.449 | -24.421 | 1.00 | 37.26 | B000 | C |
| ATOM | 7821 | O | TRP | F | 175 | -12.450 | 60.587 | -23.549 | 1.00 | 35.08 | B000 | O |
| ATOM | 7822 | CB | TRP | F | 175 | -14.700 | 61.599 | -25.815 | 1.00 | 28.70 | B000 | C |
| ATOM | 7823 | CG | TRP | F | 175 | -15.152 | 60.160 | -25.730 | 1.00 | 37.75 | B000 | C |
| ATOM | 7824 | CD1 | TRP | F | 175 | -16.401 | 59.702 | -25.374 | 1.00 | 32.61 | B000 | C |
| ATOM | 7825 | CD2 | TRP | F | 175 | -14.381 | 58.992 | -26.056 | 1.00 | 35.80 | B000 | C |
| ATOM | 7826 | NE1 | TRP | F | 175 | -16.440 | 58.337 | -25.446 | 1.00 | 36.32 | B000 | N |
| ATOM | 7827 | CE2 | TRP | F | 175 | -15.219 | 57.874 | -25.866 | 1.00 | 39.10 | B000 | C |
| ATOM | 7828 | CE3 | TRP | F | 175 | -13.067 | 58.786 | -26.488 | 1.00 | 34.63 | B000 | C |
| ATOM | 7829 | CZ2 | TRP | F | 175 | -14.788 | 56.573 | -26.097 | 1.00 | 34.59 | B000 | C |
| ATOM | 7830 | CZ3 | TRP | F | 175 | -12.637 | 57.494 | -26.720 | 1.00 | 37.06 | B000 | C |
| ATOM | 7831 | CH2 | TRP | F | 175 | -13.493 | 56.402 | -26.517 | 1.00 | 41.92 | B000 | C |
| ATOM | 7832 | N | ALA | F | 176 | -11.637 | 61.842 | -25.230 | 1.00 | 31.21 | B000 | N |
| ATOM | 7833 | CA | ALA | F | 176 | -10.356 | 61.158 | -25.149 | 1.00 | 35.56 | B000 | C |
| ATOM | 7834 | C | ALA | F | 176 | -9.749 | 61.306 | -23.765 | 1.00 | 38.45 | B000 | C |
| ATOM | 7835 | O | ALA | F | 176 | -9.108 | 60.373 | -23.270 | 1.00 | 39.51 | B000 | O |
| ATOM | 7836 | CB | ALA | F | 176 | -9.403 | 61.684 | -26.229 | 1.00 | 24.62 | B000 | C |
| ATOM | 7837 | N | ASP | F | 177 | -9.964 | 62.449 | -23.107 | 1.00 | 34.61 | B000 | N |
| ATOM | 7838 | CA | ASP | F | 177 | -9.426 | 62.601 | -21.759 | 1.00 | 35.23 | B000 | C |
| ATOM | 7839 | C | ASP | F | 177 | -10.233 | 61.808 | -20.751 | 1.00 | 37.46 | B000 | C |
| ATOM | 7840 | O | ASP | F | 177 | -9.663 | 61.241 | -19.812 | 1.00 | 34.54 | B000 | O |
| ATOM | 7841 | CB | ASP | F | 177 | -9.379 | 64.072 | -21.351 | 1.00 | 34.09 | B000 | C |
| ATOM | 7842 | CG | ASP | F | 177 | -8.386 | 64.859 | -22.167 | 1.00 | 41.05 | B000 | C |
| ATOM | 7843 | OD1 | ASP | F | 177 | -7.313 | 64.292 | -22.458 | 1.00 | 42.78 | B000 | O |
| ATOM | 7844 | OD2 | ASP | F | 177 | -8.681 | 66.028 | -22.531 | 1.00 | 43.34 | B000 | O1- |
| ATOM | 7845 | N | ALA | F | 178 | -11.561 | 61.775 | -20.907 | 1.00 | 34.72 | B000 | N |
| ATOM | 7846 | CA | ALA | F | 178 | -12.357 | 60.941 | -20.010 | 1.00 | 40.52 | B000 | C |
| ATOM | 7847 | C | ALA | F | 178 | -11.978 | 59.471 | -20.186 | 1.00 | 36.67 | B000 | C |
| ATOM | 7848 | O | ALA | F | 178 | -11.853 | 58.724 | -19.208 | 1.00 | 36.37 | B000 | O |
| ATOM | 7849 | CB | ALA | F | 178 | -13.850 | 61.165 | -20.268 | 1.00 | 32.74 | B000 | C |
| ATOM | 7850 | N | ASP | F | 179 | -11.714 | 59.070 | -21.424 | 1.00 | 34.82 | B000 | N |
| ATOM | 7851 | CA | ASP | F | 179 | -11.257 | 57.715 | -21.706 | 1.00 | 36.26 | B000 | C |
| ATOM | 7852 | C | ASP | F | 179 | -9.957 | 57.397 | -20.955 | 1.00 | 42.39 | B000 | C |
| ATOM | 7853 | O | ASP | F | 179 | -9.861 | 56.368 | -20.275 | 1.00 | 44.65 | B000 | O |
| ATOM | 7854 | CB | ASP | F | 179 | -11.107 | 57.584 | -23.221 | 1.00 | 41.84 | B000 | C |
| ATOM | 7855 | CG | ASP | F | 179 | -10.618 | 56.220 | -23.670 | 1.00 | 52.24 | B000 | C |
| ATOM | 7856 | OD1 | ASP | F | 179 | -11.237 | 55.192 | -23.304 | 1.00 | 48.91 | B000 | O |
| ATOM | 7857 | OD2 | ASP | F | 179 | -9.620 | 56.195 | -24.430 | 1.00 | 51.25 | B000 | O1- |
| ATOM | 7858 | N | ASN | F | 180 | -8.959 | 58.292 | -21.019 | 1.00 | 36.75 | B000 | N |
| ATOM | 7859 | CA | ASN | F | 180 | -7.704 | 58.044 | -20.302 | 1.00 | 37.14 | B000 | C |
| ATOM | 7860 | C | ASN | F | 180 | -7.923 | 58.008 | -18.796 | 1.00 | 39.01 | B000 | C |
| ATOM | 7861 | O | ASN | F | 180 | -7.306 | 57.205 | -18.089 | 1.00 | 45.89 | B000 | O |
| ATOM | 7862 | CB | ASN | F | 180 | -6.654 | 59.118 | -20.615 | 1.00 | 46.92 | B000 | C |
| ATOM | 7863 | CG | ASN | F | 180 | -6.153 | 59.087 | -22.053 | 1.00 | 58.49 | B000 | C |
| ATOM | 7864 | OD1 | ASN | F | 180 | -6.232 | 58.070 | -22.751 | 1.00 | 61.49 | B000 | O |
| ATOM | 7865 | ND2 | ASN | F | 180 | -5.630 | 60.232 | -22.507 | 1.00 | 67.59 | B000 | N |
| ATOM | 7866 | N | TYR | F | 181 | -8.782 | 58.887 | -18.283 | 1.00 | 43.70 | B000 | N |
| ATOM | 7867 | CA | TYR | F | 181 | -9.073 | 58.899 | -16.852 | 1.00 | 39.91 | B000 | C |
| ATOM | 7868 | C | TYR | F | 181 | -9.597 | 57.540 | -16.394 | 1.00 | 37.95 | B000 | C |
| ATOM | 7869 | O | TYR | F | 181 | -9.186 | 57.026 | -15.351 | 1.00 | 39.77 | B000 | O |
| ATOM | 7870 | CB | TYR | F | 181 | -10.089 | 60.009 | -16.524 | 1.00 | 38.63 | B000 | C |
| ATOM | 7871 | CG | TYR | F | 181 | -10.493 | 60.066 | -15.056 | 1.00 | 41.58 | B000 | C |
| ATOM | 7872 | CD1 | TYR | F | 181 | -11.438 | 59.167 | -14.527 | 1.00 | 31.11 | B000 | C |
| ATOM | 7873 | CD2 | TYR | F | 181 | -9.928 | 61.018 | -14.193 | 1.00 | 31.82 | B000 | C |
| ATOM | 7874 | CE1 | TYR | F | 181 | -11.782 | 59.205 | -13.193 | 1.00 | 31.20 | B000 | C |

| | | | | | | | | | | | | |
|---|---|---|---|---|---|---|---|---|---|---|---|---|
| ATOM | 7875 | CE2 | TYR | F | 181 | -10.285 | 61.067 | -12.856 | 1.00 | 32.07 | B000 | C |
| ATOM | 7876 | CZ | TYR | F | 181 | -11.206 | 60.153 | -12.364 | 1.00 | 37.73 | B000 | C |
| ATOM | 7877 | OH | TYR | F | 181 | -11.565 | 60.192 | -11.049 | 1.00 | 44.00 | B000 | O |
| ATOM | 7878 | N | CYS | F | 182 | -10.545 | 56.962 | -17.133 | 1.00 | 38.33 | B000 | N |
| ATOM | 7879 | CA | CYS | F | 182 | -11.080 | 55.671 | -16.702 | 1.00 | 46.48 | B000 | C |
| ATOM | 7880 | C | CYS | F | 182 | -10.008 | 54.591 | -16.772 | 1.00 | 43.13 | B000 | C |
| ATOM | 7881 | O | CYS | F | 182 | -9.877 | 53.782 | -15.848 | 1.00 | 43.36 | B000 | O |
| ATOM | 7882 | CB | CYS | F | 182 | -12.309 | 55.282 | -17.529 | 1.00 | 39.09 | B000 | C |
| ATOM | 7883 | SG | CYS | F | 182 | -13.808 | 56.319 | -17.269 | 1.00 | 47.89 | B000 | S |
| ATOM | 7884 | N | ARG | F | 183 | -9.196 | 54.599 | -17.834 | 1.00 | 45.20 | B000 | N |
| ATOM | 7885 | CA | ARG | F | 183 | -8.147 | 53.596 | -17.968 | 1.00 | 43.53 | B000 | C |
| ATOM | 7886 | C | ARG | F | 183 | -7.166 | 53.667 | -16.806 | 1.00 | 46.41 | B000 | C |
| ATOM | 7887 | O | ARG | F | 183 | -6.782 | 52.629 | -16.254 | 1.00 | 47.85 | B000 | O |
| ATOM | 7888 | CB | ARG | F | 183 | -7.439 | 53.764 | -19.316 | 1.00 | 41.85 | B000 | C |
| ATOM | 7889 | CG | ARG | F | 183 | -8.193 | 53.051 | -20.444 | 1.00 | 61.73 | B000 | C |
| ATOM | 7890 | CD | ARG | F | 183 | -7.918 | 53.573 | -21.866 | 1.00 | 68.72 | B000 | C |
| ATOM | 7891 | NE | ARG | F | 183 | -8.968 | 53.099 | -22.784 | 1.00 | 81.47 | B000 | N |
| ATOM | 7892 | CZ | ARG | F | 183 | -9.062 | 53.405 | -24.082 | 1.00 | 84.12 | B000 | C |
| ATOM | 7893 | NH1 | ARG | F | 183 | -8.149 | 54.199 | -24.652 | 1.00 | 78.81 | B000 | N1+ |
| ATOM | 7894 | NH2 | ARG | F | 183 | -10.082 | 52.925 | -24.811 | 1.00 | 60.08 | B000 | N |
| ATOM | 7895 | N | LEU | F | 184 | -6.805 | 54.884 | -16.367 | 1.00 | 42.66 | B000 | N |
| ATOM | 7896 | CA | LEU | F | 184 | -5.910 | 55.030 | -15.220 | 1.00 | 41.52 | B000 | C |
| ATOM | 7897 | C | LEU | F | 184 | -6.542 | 54.558 | -13.921 | 1.00 | 45.58 | B000 | C |
| ATOM | 7898 | O | LEU | F | 184 | -5.822 | 54.349 | -12.944 | 1.00 | 51.35 | B000 | O |
| ATOM | 7899 | CB | LEU | F | 184 | -5.441 | 56.481 | -15.057 | 1.00 | 36.16 | B000 | C |
| ATOM | 7900 | CG | LEU | F | 184 | -4.410 | 57.032 | -16.056 | 1.00 | 52.39 | B000 | C |
| ATOM | 7901 | CD1 | LEU | F | 184 | -4.128 | 58.507 | -15.803 | 1.00 | 41.04 | B000 | C |
| ATOM | 7902 | CD2 | LEU | F | 184 | -3.105 | 56.258 | -15.999 | 1.00 | 43.74 | B000 | C |
| ATOM | 7903 | N | GLU | F | 185 | -7.862 | 54.403 | -13.875 | 1.00 | 49.78 | B000 | N |
| ATOM | 7904 | CA | GLU | F | 185 | -8.548 | 53.829 | -12.724 | 1.00 | 54.22 | B000 | C |
| ATOM | 7905 | C | GLU | F | 185 | -8.765 | 52.324 | -12.849 | 1.00 | 48.30 | B000 | C |
| ATOM | 7906 | O | GLU | F | 185 | -9.579 | 51.769 | -12.104 | 1.00 | 50.24 | B000 | O |
| ATOM | 7907 | CB | GLU | F | 185 | -9.905 | 54.506 | -12.523 | 1.00 | 53.96 | B000 | C |
| ATOM | 7908 | CG | GLU | F | 185 | -9.835 | 55.994 | -12.368 | 1.00 | 48.05 | B000 | C |
| ATOM | 7909 | CD | GLU | F | 185 | -9.246 | 56.395 | -11.042 | 1.00 | 60.24 | B000 | C |
| ATOM | 7910 | OE1 | GLU | F | 185 | -9.666 | 55.804 | -10.016 | 1.00 | 64.06 | B000 | O |
| ATOM | 7911 | OE2 | GLU | F | 185 | -8.376 | 57.300 | -11.033 | 1.00 | 63.60 | B000 | O1- |
| ATOM | 7912 | N | ASP | F | 186 | -8.062 | 51.659 | -13.765 | 1.00 | 48.69 | B000 | N |
| ATOM | 7913 | CA | ASP | F | 186 | -8.311 | 50.254 | -14.090 | 1.00 | 49.60 | B000 | C |
| ATOM | 7914 | C | ASP | F | 186 | -9.782 | 50.054 | -14.431 | 1.00 | 52.34 | B000 | C |
| ATOM | 7915 | O | ASP | F | 186 | -10.445 | 49.131 | -13.950 | 1.00 | 49.55 | B000 | O |
| ATOM | 7916 | CB | ASP | F | 186 | -7.889 | 49.319 | -12.951 | 1.00 | 53.42 | B000 | C |
| ATOM | 7917 | CG | ASP | F | 186 | -6.443 | 49.508 | -12.545 | 1.00 | 79.16 | B000 | C |
| ATOM | 7918 | OD1 | ASP | F | 186 | -5.652 | 50.034 | -13.366 | 1.00 | 85.17 | B000 | O |
| ATOM | 7919 | OD2 | ASP | F | 186 | -6.093 | 49.125 | -11.403 | 1.00 | 86.75 | B000 | O1- |
| ATOM | 7920 | N | ALA | F | 187 | -10.303 | 50.958 | -15.252 | 1.00 | 50.66 | B000 | N |
| ATOM | 7921 | CA | ALA | F | 187 | -11.705 | 50.915 | -15.627 | 1.00 | 43.40 | B000 | C |
| ATOM | 7922 | C | ALA | F | 187 | -11.822 | 51.404 | -17.061 | 1.00 | 39.32 | B000 | C |
| ATOM | 7923 | O | ALA | F | 187 | -10.822 | 51.623 | -17.752 | 1.00 | 42.43 | B000 | O |
| ATOM | 7924 | CB | ALA | F | 187 | -12.559 | 51.716 | -14.636 | 1.00 | 37.47 | B000 | C |
| ATOM | 7925 | N | HIS | F | 188 | -13.048 | 51.547 | -17.523 | 1.00 | 38.43 | B000 | N |
| ATOM | 7926 | CA | HIS | F | 188 | -13.273 | 52.036 | -18.864 | 1.00 | 42.96 | B000 | C |
| ATOM | 7927 | C | HIS | F | 188 | -14.539 | 52.878 | -18.839 | 1.00 | 39.88 | B000 | C |
| ATOM | 7928 | O | HIS | F | 188 | -15.291 | 52.871 | -17.865 | 1.00 | 47.38 | B000 | O |
| ATOM | 7929 | CB | HIS | F | 188 | -13.377 | 50.872 | -19.852 | 1.00 | 40.78 | B000 | C |
| ATOM | 7930 | CG | HIS | F | 188 | -14.461 | 49.901 | -19.512 | 1.00 | 40.48 | B000 | C |
| ATOM | 7931 | ND1 | HIS | F | 188 | -15.783 | 50.124 | -19.830 | 1.00 | 46.61 | B000 | N |
| ATOM | 7932 | CD2 | HIS | F | 188 | -14.427 | 48.716 | -18.858 | 1.00 | 45.33 | B000 | C |
| ATOM | 7933 | CE1 | HIS | F | 188 | -16.515 | 49.109 | -19.403 | 1.00 | 45.57 | B000 | C |
| ATOM | 7934 | NE2 | HIS | F | 188 | -15.716 | 48.241 | -18.811 | 1.00 | 46.51 | B000 | N |
| ATOM | 7935 | N | LEU | F | 189 | -14.764 | 53.613 | -19.918 | 1.00 | 37.01 | B000 | N |
| ATOM | 7936 | CA | LEU | F | 189 | -15.978 | 54.400 | -20.036 | 1.00 | 37.46 | B000 | C |
| ATOM | 7937 | C | LEU | F | 189 | -17.191 | 53.475 | -20.121 | 1.00 | 43.07 | B000 | C |
| ATOM | 7938 | O | LEU | F | 189 | -17.135 | 52.415 | -20.754 | 1.00 | 37.38 | B000 | O |
| ATOM | 7939 | CB | LEU | F | 189 | -15.891 | 55.299 | -21.263 | 1.00 | 31.01 | B000 | C |
| ATOM | 7940 | CG | LEU | F | 189 | -14.995 | 56.532 | -21.160 | 1.00 | 35.76 | B000 | C |
| ATOM | 7941 | CD1 | LEU | F | 189 | -14.766 | 57.097 | -22.537 | 1.00 | 33.40 | B000 | C |
| ATOM | 7942 | CD2 | LEU | F | 189 | -15.624 | 57.607 | -20.257 | 1.00 | 34.63 | B000 | C |
| ATOM | 7943 | N | VAL | F | 190 | -18.301 | 53.904 | -19.502 | 1.00 | 37.96 | B000 | N |
| ATOM | 7944 | CA | VAL | F | 190 | -19.417 | 53.006 | -19.237 | 1.00 | 34.67 | B000 | C |
| ATOM | 7945 | C | VAL | F | 190 | -19.928 | 52.393 | -20.536 | 1.00 | 42.14 | B000 | C |

| | | | | | | | | | | | | |
|---|---|---|---|---|---|---|---|---|---|---|---|---|
| ATOM | 7946 | O | VAL | F | 190 | -20.044 | 53.064 | -21.573 | 1.00 | 40.38 | B000 | O |
| ATOM | 7947 | CB | VAL | F | 190 | -20.551 | 53.722 | -18.477 | 1.00 | 36.38 | B000 | C |
| ATOM | 7948 | CG1 | VAL | F | 190 | -21.205 | 54.857 | -19.319 | 1.00 | 31.50 | B000 | C |
| ATOM | 7949 | CG2 | VAL | F | 190 | -21.573 | 52.718 | -18.006 | 1.00 | 38.12 | B000 | C |
| ATOM | 7950 | N | VAL | F | 191 | -20.178 | 51.085 | -20.485 | 1.00 | 40.92 | B000 | N |
| ATOM | 7951 | CA | VAL | F | 191 | -20.787 | 50.325 | -21.567 | 1.00 | 40.00 | B000 | C |
| ATOM | 7952 | C | VAL | F | 191 | -22.162 | 49.884 | -21.082 | 1.00 | 41.46 | B000 | C |
| ATOM | 7953 | O | VAL | F | 191 | -22.265 | 49.187 | -20.063 | 1.00 | 51.69 | B000 | O |
| ATOM | 7954 | CB | VAL | F | 191 | -19.928 | 49.113 | -21.966 | 1.00 | 43.90 | B000 | C |
| ATOM | 7955 | CG1 | VAL | F | 191 | -20.596 | 48.350 | -23.106 | 1.00 | 38.60 | B000 | C |
| ATOM | 7956 | CG2 | VAL | F | 191 | -18.503 | 49.546 | -22.338 | 1.00 | 38.08 | B000 | C |
| ATOM | 7957 | N | VAL | F | 192 | -23.209 | 50.284 | -21.805 | 1.00 | 39.60 | B000 | N |
| ATOM | 7958 | CA | VAL | F | 192 | -24.603 | 50.091 | -21.402 | 1.00 | 40.55 | B000 | C |
| ATOM | 7959 | C | VAL | F | 192 | -25.139 | 48.872 | -22.134 | 1.00 | 42.33 | B000 | C |
| ATOM | 7960 | O | VAL | F | 192 | -25.454 | 48.955 | -23.326 | 1.00 | 43.63 | B000 | O |
| ATOM | 7961 | CB | VAL | F | 192 | -25.464 | 51.319 | -21.747 | 1.00 | 43.28 | B000 | C |
| ATOM | 7962 | CG1 | VAL | F | 192 | -26.869 | 51.182 | -21.155 | 1.00 | 32.94 | B000 | C |
| ATOM | 7963 | CG2 | VAL | F | 192 | -24.772 | 52.629 | -21.333 | 1.00 | 35.84 | B000 | C |
| ATOM | 7964 | N | THR | F | 193 | -25.341 | 47.762 | -21.429 | 1.00 | 48.94 | B000 | N |
| ATOM | 7965 | CA | THR | F | 193 | -25.745 | 46.531 | -22.105 | 1.00 | 47.71 | B000 | C |
| ATOM | 7966 | C | THR | F | 193 | -27.211 | 46.169 | -21.896 | 1.00 | 49.57 | B000 | C |
| ATOM | 7967 | O | THR | F | 193 | -27.654 | 45.172 | -22.465 | 1.00 | 51.89 | B000 | O |
| ATOM | 7968 | CB | THR | F | 193 | -24.866 | 45.344 | -21.673 | 1.00 | 37.74 | B000 | C |
| ATOM | 7969 | OG1 | THR | F | 193 | -25.053 | 45.097 | -20.280 | 1.00 | 43.14 | B000 | O |
| ATOM | 7970 | CG2 | THR | F | 193 | -23.361 | 45.624 | -21.935 | 1.00 | 34.77 | B000 | C |
| ATOM | 7971 | N | SER | F | 194 | -27.986 | 46.957 | -21.143 | 1.00 | 45.16 | B000 | N |
| ATOM | 7972 | CA | SER | F | 194 | -29.364 | 46.570 | -20.845 | 1.00 | 43.88 | B000 | C |
| ATOM | 7973 | C | SER | F | 194 | -30.163 | 47.775 | -20.395 | 1.00 | 47.69 | B000 | C |
| ATOM | 7974 | O | SER | F | 194 | -29.609 | 48.782 | -19.938 | 1.00 | 48.48 | B000 | O |
| ATOM | 7975 | CB | SER | F | 194 | -29.443 | 45.506 | -19.738 | 1.00 | 52.95 | B000 | C |
| ATOM | 7976 | OG | SER | F | 194 | -29.162 | 46.065 | -18.455 | 1.00 | 47.85 | B000 | O |
| ATOM | 7977 | N | TRP | F | 195 | -31.488 | 47.622 | -20.464 | 1.00 | 48.46 | B000 | N |
| ATOM | 7978 | CA | TRP | F | 195 | -32.386 | 48.680 | -20.012 | 1.00 | 44.88 | B000 | C |
| ATOM | 7979 | C | TRP | F | 195 | -32.160 | 49.001 | -18.546 | 1.00 | 47.13 | B000 | C |
| ATOM | 7980 | O | TRP | F | 195 | -32.238 | 50.165 | -18.133 | 1.00 | 45.99 | B000 | O |
| ATOM | 7981 | CB | TRP | F | 195 | -33.843 | 48.288 | -20.250 | 1.00 | 41.25 | B000 | C |
| ATOM | 7982 | CG | TRP | F | 195 | -34.410 | 48.910 | -21.469 | 1.00 | 54.27 | B000 | C |
| ATOM | 7983 | CD1 | TRP | F | 195 | -34.863 | 48.275 | -22.601 | 1.00 | 50.60 | B000 | C |
| ATOM | 7984 | CD2 | TRP | F | 195 | -34.541 | 50.319 | -21.716 | 1.00 | 55.13 | B000 | C |
| ATOM | 7985 | NE1 | TRP | F | 195 | -35.307 | 49.212 | -23.521 | 1.00 | 52.12 | B000 | N |
| ATOM | 7986 | CE2 | TRP | F | 195 | -35.114 | 50.470 | -23.005 | 1.00 | 54.46 | B000 | C |
| ATOM | 7987 | CE3 | TRP | F | 195 | -34.246 | 51.467 | -20.964 | 1.00 | 50.27 | B000 | C |
| ATOM | 7988 | CZ2 | TRP | F | 195 | -35.391 | 51.727 | -23.561 | 1.00 | 62.09 | B000 | C |
| ATOM | 7989 | CZ3 | TRP | F | 195 | -34.530 | 52.721 | -21.513 | 1.00 | 54.11 | B000 | C |
| ATOM | 7990 | CH2 | TRP | F | 195 | -35.091 | 52.837 | -22.804 | 1.00 | 59.63 | B000 | C |
| ATOM | 7991 | N | GLU | F | 196 | -31.875 | 47.980 | -17.747 | 1.00 | 43.73 | B000 | N |
| ATOM | 7992 | CA | GLU | F | 196 | -31.638 | 48.202 | -16.330 | 1.00 | 46.25 | B000 | C |
| ATOM | 7993 | C | GLU | F | 196 | -30.421 | 49.094 | -16.133 | 1.00 | 48.83 | B000 | C |
| ATOM | 7994 | O | GLU | F | 196 | -30.505 | 50.141 | -15.480 | 1.00 | 50.61 | B000 | O |
| ATOM | 7995 | CB | GLU | F | 196 | -31.445 | 46.853 | -15.623 | 1.00 | 52.52 | B000 | C |
| ATOM | 7996 | CG | GLU | F | 196 | -32.744 | 46.023 | -15.500 | 1.00 | 56.68 | B000 | C |
| ATOM | 7997 | CD | GLU | F | 196 | -33.141 | 45.323 | -16.823 | 1.00 | 67.73 | B000 | C |
| ATOM | 7998 | OE1 | GLU | F | 196 | -32.250 | 45.039 | -17.660 | 1.00 | 66.56 | B000 | O |
| ATOM | 7999 | OE2 | GLU | F | 196 | -34.348 | 45.063 | -17.030 | 1.00 | 76.24 | B000 | O1- |
| ATOM | 8000 | N | GLU | F | 197 | -29.293 | 48.717 | -16.744 | 1.00 | 40.21 | B000 | N |
| ATOM | 8001 | CA | GLU | F | 197 | -28.089 | 49.533 | -16.666 | 1.00 | 46.56 | B000 | C |
| ATOM | 8002 | C | GLU | F | 197 | -28.364 | 50.956 | -17.149 | 1.00 | 46.02 | B000 | C |
| ATOM | 8003 | O | GLU | F | 197 | -27.964 | 51.927 | -16.494 | 1.00 | 39.09 | B000 | O |
| ATOM | 8004 | CB | GLU | F | 197 | -26.965 | 48.881 | -17.475 | 1.00 | 41.68 | B000 | C |
| ATOM | 8005 | CG | GLU | F | 197 | -25.566 | 49.351 | -17.097 | 1.00 | 46.61 | B000 | C |
| ATOM | 8006 | CD | GLU | F | 197 | -24.465 | 48.581 | -17.830 | 1.00 | 49.56 | B000 | C |
| ATOM | 8007 | OE1 | GLU | F | 197 | -24.781 | 47.861 | -18.816 | 1.00 | 45.94 | B000 | O |
| ATOM | 8008 | OE2 | GLU | F | 197 | -23.283 | 48.692 | -17.413 | 1.00 | 45.17 | B000 | O1- |
| ATOM | 8009 | N | GLN | F | 198 | -29.087 | 51.091 | -18.271 | 1.00 | 37.03 | B000 | N |
| ATOM | 8010 | CA | GLN | F | 198 | -29.451 | 52.403 | -18.794 | 1.00 | 36.33 | B000 | C |
| ATOM | 8011 | C | GLN | F | 198 | -30.217 | 53.231 | -17.769 | 1.00 | 44.55 | B000 | C |
| ATOM | 8012 | O | GLN | F | 198 | -29.896 | 54.400 | -17.546 | 1.00 | 40.80 | B000 | O |
| ATOM | 8013 | CB | GLN | F | 198 | -30.275 | 52.248 | -20.065 | 1.00 | 35.40 | B000 | C |
| ATOM | 8014 | CG | GLN | F | 198 | -31.035 | 53.513 | -20.445 | 1.00 | 35.73 | B000 | C |
| ATOM | 8015 | CD | GLN | F | 198 | -30.137 | 54.563 | -21.106 | 1.00 | 43.38 | B000 | C |
| ATOM | 8016 | OE1 | GLN | F | 198 | -29.107 | 54.240 | -21.704 | 1.00 | 38.10 | B000 | O |

| | | | | | | | | | | | | |
|---|---|---|---|---|---|---|---|---|---|---|---|---|
| ATOM | 8017 | NE2 | GLN | F | 198 | -30.535 | 55.821 | -21.007 | 1.00 | 38.22 | B000 | N |
| ATOM | 8018 | N | LYS | F | 199 | -31.229 | 52.641 | -17.123 | 1.00 | 47.51 | B000 | N |
| ATOM | 8019 | CA | LYS | F | 199 | -32.018 | 53.399 | -16.154 | 1.00 | 44.51 | B000 | C |
| ATOM | 8020 | C | LYS | F | 199 | -31.196 | 53.714 | -14.918 | 1.00 | 39.60 | B000 | C |
| ATOM | 8021 | O | LYS | F | 199 | -31.308 | 54.805 | -14.351 | 1.00 | 43.02 | B000 | O |
| ATOM | 8022 | CB | LYS | F | 199 | -33.276 | 52.619 | -15.760 | 1.00 | 48.82 | B000 | C |
| ATOM | 8023 | CG | LYS | F | 199 | -34.257 | 52.311 | -16.891 | 1.00 | 50.78 | B000 | C |
| ATOM | 8024 | CD | LYS | F | 199 | -35.683 | 52.463 | -16.392 | 1.00 | 65.99 | B000 | C |
| ATOM | 8025 | CE | LYS | F | 199 | -36.698 | 51.763 | -17.281 | 1.00 | 72.04 | B000 | C |
| ATOM | 8026 | NZ | LYS | F | 199 | -38.023 | 51.636 | -16.575 | 1.00 | 76.98 | B000 | N1+ |
| ATOM | 8027 | N | PHE | F | 200 | -30.351 | 52.773 | -14.502 | 1.00 | 38.71 | B000 | N |
| ATOM | 8028 | CA | PHE | F | 200 | -29.447 | 53.007 | -13.388 | 1.00 | 38.04 | B000 | C |
| ATOM | 8029 | C | PHE | F | 200 | -28.557 | 54.224 | -13.641 | 1.00 | 47.55 | B000 | C |
| ATOM | 8030 | O | PHE | F | 200 | -28.381 | 55.073 | -12.761 | 1.00 | 42.70 | B000 | O |
| ATOM | 8031 | CB | PHE | F | 200 | -28.604 | 51.759 | -13.163 | 1.00 | 40.51 | B000 | C |
| ATOM | 8032 | CG | PHE | F | 200 | -27.381 | 51.999 | -12.347 | 1.00 | 41.69 | B000 | C |
| ATOM | 8033 | CD1 | PHE | F | 200 | -27.473 | 52.215 | -10.980 | 1.00 | 38.29 | B000 | C |
| ATOM | 8034 | CD2 | PHE | F | 200 | -26.130 | 52.027 | -12.953 | 1.00 | 37.96 | B000 | C |
| ATOM | 8035 | CE1 | PHE | F | 200 | -26.330 | 52.448 | -10.216 | 1.00 | 42.63 | B000 | C |
| ATOM | 8036 | CE2 | PHE | F | 200 | -24.978 | 52.252 | -12.205 | 1.00 | 39.67 | B000 | C |
| ATOM | 8037 | CZ | PHE | F | 200 | -25.076 | 52.466 | -10.830 | 1.00 | 44.08 | B000 | C |
| ATOM | 8038 | N | VAL | F | 201 | -27.984 | 54.328 | -14.842 | 1.00 | 43.83 | B000 | N |
| ATOM | 8039 | CA | VAL | F | 201 | -27.087 | 55.445 | -15.131 | 1.00 | 40.81 | B000 | C |
| ATOM | 8040 | C | VAL | F | 201 | -27.865 | 56.756 | -15.182 | 1.00 | 44.68 | B000 | C |
| ATOM | 8041 | O | VAL | F | 201 | -27.442 | 57.758 | -14.596 | 1.00 | 42.14 | B000 | O |
| ATOM | 8042 | CB | VAL | F | 201 | -26.313 | 55.196 | -16.438 | 1.00 | 40.73 | B000 | C |
| ATOM | 8043 | CG1 | VAL | F | 201 | -25.631 | 56.475 | -16.900 | 1.00 | 38.56 | B000 | C |
| ATOM | 8044 | CG2 | VAL | F | 201 | -25.297 | 54.053 | -16.260 | 1.00 | 34.68 | B000 | C |
| ATOM | 8045 | N | GLN | F | 202 | -29.005 | 56.768 | -15.894 | 1.00 | 37.85 | B000 | N |
| ATOM | 8046 | CA | GLN | F | 202 | -29.859 | 57.956 | -15.959 | 1.00 | 43.72 | B000 | C |
| ATOM | 8047 | C | GLN | F | 202 | -30.185 | 58.499 | -14.582 | 1.00 | 46.87 | B000 | C |
| ATOM | 8048 | O | GLN | F | 202 | -30.211 | 59.720 | -14.367 | 1.00 | 46.96 | B000 | O |
| ATOM | 8049 | CB | GLN | F | 202 | -31.184 | 57.645 | -16.634 | 1.00 | 43.62 | B000 | C |
| ATOM | 8050 | CG | GLN | F | 202 | -31.156 | 57.250 | -18.044 | 1.00 | 46.33 | B000 | C |
| ATOM | 8051 | CD | GLN | F | 202 | -32.572 | 57.120 | -18.537 | 1.00 | 50.37 | B000 | C |
| ATOM | 8052 | OE1 | GLN | F | 202 | -32.871 | 56.353 | -19.451 | 1.00 | 47.40 | B000 | O |
| ATOM | 8053 | NE2 | GLN | F | 202 | -33.469 | 57.864 | -17.901 | 1.00 | 48.25 | B000 | N |
| ATOM | 8054 | N | HIS | F | 203 | -30.503 | 57.603 | -13.654 | 1.00 | 41.20 | B000 | N |
| ATOM | 8055 | CA | HIS | F | 203 | -30.825 | 58.034 | -12.307 | 1.00 | 44.02 | B000 | C |
| ATOM | 8056 | C | HIS | F | 203 | -29.701 | 58.866 | -11.707 | 1.00 | 47.89 | B000 | C |
| ATOM | 8057 | O | HIS | F | 203 | -29.958 | 59.896 | -11.078 | 1.00 | 47.99 | B000 | O |
| ATOM | 8058 | CB | HIS | F | 203 | -31.106 | 56.827 | -11.421 | 1.00 | 40.71 | B000 | C |
| ATOM | 8059 | CG | HIS | F | 203 | -31.413 | 57.206 | -10.013 | 1.00 | 53.47 | B000 | C |
| ATOM | 8060 | ND1 | HIS | F | 203 | -30.514 | 57.028 | -8.982 | 1.00 | 56.30 | B000 | N |
| ATOM | 8061 | CD2 | HIS | F | 203 | -32.499 | 57.805 | -9.470 | 1.00 | 46.30 | B000 | C |
| ATOM | 8062 | CE1 | HIS | F | 203 | -31.044 | 57.478 | -7.858 | 1.00 | 55.89 | B000 | C |
| ATOM | 8063 | NE2 | HIS | F | 203 | -32.248 | 57.953 | -8.127 | 1.00 | 56.08 | B000 | N |
| ATOM | 8064 | N | HIS | F | 204 | -28.444 | 58.458 | -11.918 | 1.00 | 41.43 | B000 | N |
| ATOM | 8065 | CA | HIS | F | 204 | -27.328 | 59.148 | -11.283 | 1.00 | 39.37 | B000 | C |
| ATOM | 8066 | C | HIS | F | 204 | -26.814 | 60.355 | -12.055 | 1.00 | 40.69 | B000 | C |
| ATOM | 8067 | O | HIS | F | 204 | -26.317 | 61.291 | -11.426 | 1.00 | 42.64 | B000 | O |
| ATOM | 8068 | CB | HIS | F | 204 | -26.174 | 58.188 | -11.035 | 1.00 | 43.78 | B000 | C |
| ATOM | 8069 | CG | HIS | F | 204 | -26.401 | 57.295 | -9.864 | 1.00 | 48.22 | B000 | C |
| ATOM | 8070 | ND1 | HIS | F | 204 | -26.903 | 56.017 | -9.983 | 1.00 | 46.54 | B000 | N |
| ATOM | 8071 | CD2 | HIS | F | 204 | -26.255 | 57.524 | -8.538 | 1.00 | 40.29 | B000 | C |
| ATOM | 8072 | CE1 | HIS | F | 204 | -27.021 | 55.484 | -8.781 | 1.00 | 48.73 | B000 | C |
| ATOM | 8073 | NE2 | HIS | F | 204 | -26.638 | 56.378 | -7.887 | 1.00 | 42.03 | B000 | N |
| ATOM | 8074 | N | ILE | F | 205 | -26.916 | 60.374 | -13.387 | 1.00 | 37.14 | B000 | N |
| ATOM | 8075 | CA | ILE | F | 205 | -26.350 | 61.499 | -14.131 | 1.00 | 36.61 | B000 | C |
| ATOM | 8076 | C | ILE | F | 205 | -27.319 | 62.674 | -14.148 | 1.00 | 42.45 | B000 | C |
| ATOM | 8077 | O | ILE | F | 205 | -26.895 | 63.820 | -14.292 | 1.00 | 40.59 | B000 | O |
| ATOM | 8078 | CB | ILE | F | 205 | -25.950 | 61.116 | -15.576 | 1.00 | 34.16 | B000 | C |
| ATOM | 8079 | CG1 | ILE | F | 205 | -27.167 | 60.709 | -16.402 | 1.00 | 34.84 | B000 | C |
| ATOM | 8080 | CG2 | ILE | F | 205 | -24.871 | 60.040 | -15.609 | 1.00 | 29.47 | B000 | C |
| ATOM | 8081 | CD1 | ILE | F | 205 | -26.829 | 60.403 | -17.814 | 1.00 | 37.88 | B000 | C |
| ATOM | 8082 | N | GLY | F | 206 | -28.620 | 62.416 | -14.017 | 1.00 | 43.01 | B000 | N |
| ATOM | 8083 | CA | GLY | F | 206 | -29.611 | 63.456 | -14.094 | 1.00 | 36.11 | B000 | C |
| ATOM | 8084 | C | GLY | F | 206 | -29.691 | 64.031 | -15.491 | 1.00 | 41.59 | B000 | C |
| ATOM | 8085 | O | GLY | F | 206 | -29.511 | 63.335 | -16.493 | 1.00 | 41.37 | B000 | O |
| ATOM | 8086 | N | PRO | F | 207 | -29.950 | 65.320 | -15.574 | 1.00 | 36.32 | B000 | N |
| ATOM | 8087 | CA | PRO | F | 207 | -30.143 | 65.969 | -16.875 | 1.00 | 39.41 | B000 | C |

| | | | | | | | | | | | |
|---|---|---|---|---|---|---|---|---|---|---|---|
| ATOM | 8088 | C | PRO | F | 207 | -28.881 | 66.583 | -17.484 | 1.00 | 35.53 | B000 C |
| ATOM | 8089 | O | PRO | F | 207 | -28.943 | 67.699 | -18.000 | 1.00 | 50.85 | B000 O |
| ATOM | 8090 | CB | PRO | F | 207 | -31.148 | 67.068 | -16.531 | 1.00 | 38.74 | B000 C |
| ATOM | 8091 | CG | PRO | F | 207 | -30.663 | 67.508 | -15.160 | 1.00 | 35.34 | B000 C |
| ATOM | 8092 | CD | PRO | F | 207 | -30.199 | 66.246 | -14.457 | 1.00 | 39.53 | B000 C |
| ATOM | 8093 | N | VAL | F | 208 | -27.741 | 65.910 | -17.422 | 1.00 | 33.23 | B000 N |
| ATOM | 8094 | CA | VAL | F | 208 | -26.468 | 66.469 | -17.858 | 1.00 | 39.13 | B000 C |
| ATOM | 8095 | C | VAL | F | 208 | -25.919 | 65.604 | -18.985 | 1.00 | 35.96 | B000 C |
| ATOM | 8096 | O | VAL | F | 208 | -25.881 | 64.378 | -18.856 | 1.00 | 36.23 | B000 O |
| ATOM | 8097 | CB | VAL | F | 208 | -25.470 | 66.542 | -16.690 | 1.00 | 35.73 | B000 C |
| ATOM | 8098 | CG1 | VAL | F | 208 | -24.205 | 67.239 | -17.130 | 1.00 | 31.91 | B000 C |
| ATOM | 8099 | CG2 | VAL | F | 208 | -26.108 | 67.258 | -15.495 | 1.00 | 37.34 | B000 C |
| ATOM | 8100 | N | ASN | F | 209 | -25.509 | 66.232 | -20.088 | 1.00 | 32.26 | B000 N |
| ATOM | 8101 | CA | ASN | F | 209 | -24.855 | 65.476 | -21.157 | 1.00 | 32.97 | B000 C |
| ATOM | 8102 | C | ASN | F | 209 | -23.617 | 64.755 | -20.626 | 1.00 | 34.25 | B000 C |
| ATOM | 8103 | O | ASN | F | 209 | -22.796 | 65.346 | -19.917 | 1.00 | 33.13 | B000 O |
| ATOM | 8104 | CB | ASN | F | 209 | -24.481 | 66.389 | -22.315 | 1.00 | 30.89 | B000 C |
| ATOM | 8105 | CG | ASN | F | 209 | -25.679 | 66.764 | -23.167 | 1.00 | 37.35 | B000 C |
| ATOM | 8106 | OD1 | ASN | F | 209 | -26.585 | 65.948 | -23.377 | 1.00 | 36.11 | B000 O |
| ATOM | 8107 | ND2 | ASN | F | 209 | -25.682 | 67.989 | -23.685 | 1.00 | 31.49 | B000 N |
| ATOM | 8108 | N | THR | F | 210 | -23.508 | 63.458 | -20.934 | 1.00 | 30.22 | B000 N |
| ATOM | 8109 | CA | THR | F | 210 | -22.544 | 62.592 | -20.266 | 1.00 | 31.09 | B000 C |
| ATOM | 8110 | C | THR | F | 210 | -21.985 | 61.559 | -21.244 | 1.00 | 34.46 | B000 C |
| ATOM | 8111 | O | THR | F | 210 | -22.756 | 60.821 | -21.873 | 1.00 | 32.16 | B000 O |
| ATOM | 8112 | CB | THR | F | 210 | -23.210 | 61.900 | -19.071 | 1.00 | 32.23 | B000 C |
| ATOM | 8113 | OG1 | THR | F | 210 | -23.838 | 62.882 | -18.229 | 1.00 | 31.01 | B000 O |
| ATOM | 8114 | CG2 | THR | F | 210 | -22.187 | 61.116 | -18.256 | 1.00 | 27.20 | B000 C |
| ATOM | 8115 | N | TRP | F | 211 | -20.650 | 61.510 | -21.371 | 1.00 | 29.01 | B000 N |
| ATOM | 8116 | CA | TRP | F | 211 | -19.987 | 60.567 | -22.278 | 1.00 | 30.78 | B000 C |
| ATOM | 8117 | C | TRP | F | 211 | -20.208 | 59.116 | -21.857 | 1.00 | 33.57 | B000 C |
| ATOM | 8118 | O | TRP | F | 211 | -20.215 | 58.792 | -20.667 | 1.00 | 33.68 | B000 O |
| ATOM | 8119 | CB | TRP | F | 211 | -18.473 | 60.834 | -22.329 | 1.00 | 28.32 | B000 C |
| ATOM | 8120 | CG | TRP | F | 211 | -18.041 | 62.091 | -23.074 | 1.00 | 29.99 | B000 C |
| ATOM | 8121 | CD1 | TRP | F | 211 | -17.177 | 63.044 | -22.623 | 1.00 | 28.61 | B000 C |
| ATOM | 8122 | CD2 | TRP | F | 211 | -18.423 | 62.498 | -24.404 | 1.00 | 30.26 | B000 C |
| ATOM | 8123 | NE1 | TRP | F | 211 | -16.989 | 64.012 | -23.580 | 1.00 | 32.26 | B000 N |
| ATOM | 8124 | CE2 | TRP | F | 211 | -17.742 | 63.709 | -24.681 | 1.00 | 29.27 | B000 C |
| ATOM | 8125 | CE3 | TRP | F | 211 | -19.273 | 61.958 | -25.381 | 1.00 | 26.34 | B000 C |
| ATOM | 8126 | CZ2 | TRP | F | 211 | -17.891 | 64.402 | -25.888 | 1.00 | 28.81 | B000 C |
| ATOM | 8127 | CZ3 | TRP | F | 211 | -19.431 | 62.640 | -26.575 | 1.00 | 28.36 | B000 C |
| ATOM | 8128 | CH2 | TRP | F | 211 | -18.739 | 63.866 | -26.818 | 1.00 | 34.89 | B000 C |
| ATOM | 8129 | N | MET | F | 212 | -20.359 | 58.232 | -22.845 | 1.00 | 36.13 | B000 N |
| ATOM | 8130 | CA | MET | F | 212 | -20.261 | 56.789 | -22.634 | 1.00 | 36.28 | B000 C |
| ATOM | 8131 | C | MET | F | 212 | -19.149 | 56.198 | -23.506 | 1.00 | 41.20 | B000 C |
| ATOM | 8132 | O | MET | F | 212 | -18.610 | 56.848 | -24.412 | 1.00 | 38.23 | B000 O |
| ATOM | 8133 | CB | MET | F | 212 | -21.598 | 56.096 | -22.919 | 1.00 | 36.01 | B000 C |
| ATOM | 8134 | CG | MET | F | 212 | -21.950 | 55.930 | -24.386 | 1.00 | 32.65 | B000 C |
| ATOM | 8135 | SD | MET | F | 212 | -23.698 | 55.486 | -24.615 | 1.00 | 40.40 | B000 S |
| ATOM | 8136 | CE | MET | F | 212 | -24.541 | 57.090 | -24.376 | 1.00 | 32.88 | B000 C |
| ATOM | 8137 | N | GLY | F | 213 | -18.810 | 54.942 | -23.233 | 1.00 | 41.45 | B000 N |
| ATOM | 8138 | CA | GLY | F | 213 | -17.729 | 54.287 | -23.952 | 1.00 | 36.96 | B000 C |
| ATOM | 8139 | C | GLY | F | 213 | -18.090 | 53.824 | -25.345 | 1.00 | 39.99 | B000 C |
| ATOM | 8140 | O | GLY | F | 213 | -17.905 | 52.645 | -25.677 | 1.00 | 38.24 | B000 O |
| ATOM | 8141 | N | LEU | F | 214 | -18.614 | 54.732 | -26.173 | 1.00 | 34.09 | B000 N |
| ATOM | 8142 | CA | LEU | F | 214 | -19.135 | 54.371 | -27.489 | 1.00 | 37.28 | B000 C |
| ATOM | 8143 | C | LEU | F | 214 | -18.700 | 55.449 | -28.470 | 1.00 | 36.04 | B000 C |
| ATOM | 8144 | O | LEU | F | 214 | -18.965 | 56.632 | -28.244 | 1.00 | 38.95 | B000 O |
| ATOM | 8145 | CB | LEU | F | 214 | -20.673 | 54.216 | -27.453 | 1.00 | 36.90 | B000 C |
| ATOM | 8146 | CG | LEU | F | 214 | -21.474 | 53.990 | -28.753 | 1.00 | 39.75 | B000 C |
| ATOM | 8147 | CD1 | LEU | F | 214 | -20.997 | 52.758 | -29.501 | 1.00 | 40.07 | B000 C |
| ATOM | 8148 | CD2 | LEU | F | 214 | -22.967 | 53.871 | -28.493 | 1.00 | 34.46 | B000 C |
| ATOM | 8149 | N | HIS | F | 215 | -18.023 | 55.054 | -29.545 | 1.00 | 41.21 | B000 N |
| ATOM | 8150 | CA | HIS | F | 215 | -17.454 | 56.028 | -30.471 | 1.00 | 43.32 | B000 C |
| ATOM | 8151 | C | HIS | F | 215 | -17.360 | 55.421 | -31.860 | 1.00 | 44.63 | B000 C |
| ATOM | 8152 | O | HIS | F | 215 | -17.496 | 54.209 | -32.041 | 1.00 | 41.89 | B000 O |
| ATOM | 8153 | CB | HIS | F | 215 | -16.081 | 56.493 | -30.014 | 1.00 | 37.84 | B000 C |
| ATOM | 8154 | CG | HIS | F | 215 | -15.059 | 55.406 | -30.023 | 1.00 | 48.13 | B000 C |
| ATOM | 8155 | ND1 | HIS | F | 215 | -14.161 | 55.238 | -31.054 | 1.00 | 51.31 | B000 N |
| ATOM | 8156 | CD2 | HIS | F | 215 | -14.806 | 54.416 | -29.137 | 1.00 | 50.71 | B000 C |
| ATOM | 8157 | CE1 | HIS | F | 215 | -13.391 | 54.198 | -30.797 | 1.00 | 52.71 | B000 C |
| ATOM | 8158 | NE2 | HIS | F | 215 | -13.760 | 53.683 | -29.639 | 1.00 | 46.57 | B000 N |

| | | | | | | | | | | | | |
|---|---|---|---|---|---|---|---|---|---|---|---|---|
| ATOM | 8159 | N | ASP | F | 216 | -17.144 | 56.288 | -32.853 | 1.00 | 40.97 | B000 | N |
| ATOM | 8160 | CA | ASP | F | 216 | -17.135 | 55.874 | -34.252 | 1.00 | 42.50 | B000 | C |
| ATOM | 8161 | C | ASP | F | 216 | -15.783 | 56.113 | -34.912 | 1.00 | 43.44 | B000 | C |
| ATOM | 8162 | O | ASP | F | 216 | -15.701 | 56.251 | -36.131 | 1.00 | 42.45 | B000 | O |
| ATOM | 8163 | CB | ASP | F | 216 | -18.241 | 56.589 | -35.029 | 1.00 | 40.42 | B000 | C |
| ATOM | 8164 | CG | ASP | F | 216 | -17.950 | 58.067 | -35.268 | 1.00 | 43.85 | B000 | C |
| ATOM | 8165 | OD1 | ASP | F | 216 | -16.970 | 58.617 | -34.703 | 1.00 | 43.41 | B000 | O |
| ATOM | 8166 | OD2 | ASP | F | 216 | -18.733 | 58.690 | -36.024 | 1.00 | 45.80 | B000 | O1- |
| ATOM | 8167 | N | GLN | F | 217 | -14.710 | 56.137 | -34.128 | 1.00 | 51.10 | B000 | N |
| ATOM | 8168 | CA | GLN | F | 217 | -13.512 | 56.817 | -34.600 | 1.00 | 58.50 | B000 | C |
| ATOM | 8169 | C | GLN | F | 217 | -12.746 | 56.030 | -35.663 | 1.00 | 59.01 | B000 | C |
| ATOM | 8170 | O | GLN | F | 217 | -11.858 | 56.600 | -36.302 | 1.00 | 64.06 | B000 | O |
| ATOM | 8171 | CB | GLN | F | 217 | -12.653 | 57.214 | -33.385 | 1.00 | 60.37 | B000 | C |
| ATOM | 8172 | CG | GLN | F | 217 | -13.415 | 58.358 | -32.581 | 1.00 | 65.68 | B000 | C |
| ATOM | 8173 | CD | GLN | F | 217 | -12.601 | 59.087 | -31.493 | 1.00 | 70.67 | B000 | C |
| ATOM | 8174 | OE1 | GLN | F | 217 | -12.640 | 58.714 | -30.307 | 1.00 | 52.11 | B000 | O |
| ATOM | 8175 | NE2 | GLN | F | 217 | -11.925 | 60.175 | -31.884 | 1.00 | 67.51 | B000 | N |
| ATOM | 8176 | N | ASN | F | 218 | -13.108 | 54.777 | -35.930 | 1.00 | 52.55 | B000 | N |
| ATOM | 8177 | CA | ASN | F | 218 | -12.597 | 54.084 | -37.103 | 1.00 | 58.15 | B000 | C |
| ATOM | 8178 | C | ASN | F | 218 | -13.615 | 53.968 | -38.223 | 1.00 | 65.45 | B000 | C |
| ATOM | 8179 | O | ASN | F | 218 | -13.292 | 53.430 | -39.289 | 1.00 | 62.69 | B000 | O |
| ATOM | 8180 | CB | ASN | F | 218 | -12.114 | 52.698 | -36.724 | 1.00 | 71.05 | B000 | C |
| ATOM | 8181 | CG | ASN | F | 218 | -10.817 | 52.743 | -35.976 | 1.00 | 81.96 | B000 | C |
| ATOM | 8182 | OD1 | ASN | F | 218 | -10.715 | 52.221 | -34.854 | 1.00 | 76.60 | B000 | O |
| ATOM | 8183 | ND2 | ASN | F | 218 | -9.816 | 53.410 | -36.568 | 1.00 | 58.18 | B000 | N |
| ATOM | 8184 | N | GLY | F | 219 | -14.834 | 54.440 | -38.007 | 1.00 | 51.95 | B000 | N |
| ATOM | 8185 | CA | GLY | F | 219 | -15.897 | 54.245 | -38.957 | 1.00 | 50.90 | B000 | C |
| ATOM | 8186 | C | GLY | F | 219 | -17.074 | 53.561 | -38.292 | 1.00 | 41.92 | B000 | C |
| ATOM | 8187 | O | GLY | F | 219 | -18.131 | 54.156 | -38.073 | 1.00 | 49.46 | B000 | O |
| ATOM | 8188 | N | PRO | F | 220 | -16.902 | 52.297 | -37.938 | 1.00 | 48.10 | B000 | N |
| ATOM | 8189 | CA | PRO | F | 220 | -17.984 | 51.582 | -37.251 | 1.00 | 50.76 | B000 | C |
| ATOM | 8190 | C | PRO | F | 220 | -18.125 | 52.032 | -35.803 | 1.00 | 45.49 | B000 | C |
| ATOM | 8191 | O | PRO | F | 220 | -17.137 | 52.233 | -35.095 | 1.00 | 50.03 | B000 | O |
| ATOM | 8192 | CB | PRO | F | 220 | -17.550 | 50.113 | -37.340 | 1.00 | 44.33 | B000 | C |
| ATOM | 8193 | CG | PRO | F | 220 | -16.058 | 50.172 | -37.501 | 1.00 | 53.78 | B000 | C |
| ATOM | 8194 | CD | PRO | F | 220 | -15.762 | 51.421 | -38.268 | 1.00 | 48.76 | B000 | C |
| ATOM | 8195 | N | TRP | F | 221 | -19.371 | 52.181 | -35.365 | 1.00 | 42.06 | B000 | N |
| ATOM | 8196 | CA | TRP | F | 221 | -19.631 | 52.403 | -33.953 | 1.00 | 44.69 | B000 | C |
| ATOM | 8197 | C | TRP | F | 221 | -19.168 | 51.194 | -33.147 | 1.00 | 38.24 | B000 | C |
| ATOM | 8198 | O | TRP | F | 221 | -19.473 | 50.053 | -33.498 | 1.00 | 49.53 | B000 | O |
| ATOM | 8199 | CB | TRP | F | 221 | -21.123 | 52.668 | -33.733 | 1.00 | 41.17 | B000 | C |
| ATOM | 8200 | CG | TRP | F | 221 | -21.529 | 54.055 | -34.121 | 1.00 | 43.26 | B000 | C |
| ATOM | 8201 | CD1 | TRP | F | 221 | -22.156 | 54.450 | -35.268 | 1.00 | 36.74 | B000 | C |
| ATOM | 8202 | CD2 | TRP | F | 221 | -21.300 | 55.244 | -33.355 | 1.00 | 34.96 | B000 | C |
| ATOM | 8203 | NE1 | TRP | F | 221 | -22.336 | 55.817 | -35.259 | 1.00 | 35.73 | B000 | N |
| ATOM | 8204 | CE2 | TRP | F | 221 | -21.824 | 56.322 | -34.089 | 1.00 | 37.88 | B000 | C |
| ATOM | 8205 | CE3 | TRP | F | 221 | -20.709 | 55.492 | -32.116 | 1.00 | 35.21 | B000 | C |
| ATOM | 8206 | CZ2 | TRP | F | 221 | -21.781 | 57.639 | -33.617 | 1.00 | 42.62 | B000 | C |
| ATOM | 8207 | CZ3 | TRP | F | 221 | -20.663 | 56.791 | -31.648 | 1.00 | 39.03 | B000 | C |
| ATOM | 8208 | CH2 | TRP | F | 221 | -21.194 | 57.848 | -32.395 | 1.00 | 38.23 | B000 | C |
| ATOM | 8209 | N | LYS | F | 222 | -18.432 | 51.450 | -32.065 | 1.00 | 41.84 | B000 | N |
| ATOM | 8210 | CA | LYS | F | 222 | -17.829 | 50.409 | -31.245 | 1.00 | 41.88 | B000 | C |
| ATOM | 8211 | C | LYS | F | 222 | -17.885 | 50.777 | -29.770 | 1.00 | 45.01 | B000 | C |
| ATOM | 8212 | O | LYS | F | 222 | -17.788 | 51.953 | -29.409 | 1.00 | 38.97 | B000 | O |
| ATOM | 8213 | CB | LYS | F | 222 | -16.367 | 50.176 | -31.639 | 1.00 | 47.29 | B000 | C |
| ATOM | 8214 | CG | LYS | F | 222 | -16.184 | 49.600 | -33.033 | 1.00 | 60.26 | B000 | C |
| ATOM | 8215 | CD | LYS | F | 222 | -14.729 | 49.672 | -33.464 | 1.00 | 61.80 | B000 | C |
| ATOM | 8216 | CE | LYS | F | 222 | -14.371 | 51.081 | -33.924 | 1.00 | 69.59 | B000 | C |
| ATOM | 8217 | NZ | LYS | F | 222 | -13.030 | 51.109 | -34.562 | 1.00 | 80.69 | B000 | N1+ |
| ATOM | 8218 | N | TRP | F | 223 | -18.007 | 49.751 | -28.918 | 1.00 | 43.07 | B000 | N |
| ATOM | 8219 | CA | TRP | F | 223 | -17.864 | 49.911 | -27.476 | 1.00 | 42.29 | B000 | C |
| ATOM | 8220 | C | TRP | F | 223 | -16.411 | 49.702 | -27.074 | 1.00 | 45.99 | B000 | C |
| ATOM | 8221 | O | TRP | F | 223 | -15.708 | 48.879 | -27.661 | 1.00 | 52.22 | B000 | O |
| ATOM | 8222 | CB | TRP | F | 223 | -18.762 | 48.940 | -26.696 | 1.00 | 41.28 | B000 | C |
| ATOM | 8223 | CG | TRP | F | 223 | -20.258 | 49.200 | -26.825 | 1.00 | 43.60 | B000 | C |
| ATOM | 8224 | CD1 | TRP | F | 223 | -21.155 | 48.512 | -27.605 | 1.00 | 41.95 | B000 | C |
| ATOM | 8225 | CD2 | TRP | F | 223 | -21.014 | 50.224 | -26.158 | 1.00 | 45.65 | B000 | C |
| ATOM | 8226 | NE1 | TRP | F | 223 | -22.422 | 49.047 | -27.462 | 1.00 | 42.77 | B000 | N |
| ATOM | 8227 | CE2 | TRP | F | 223 | -22.366 | 50.089 | -26.575 | 1.00 | 41.46 | B000 | C |
| ATOM | 8228 | CE3 | TRP | F | 223 | -20.684 | 51.237 | -25.249 | 1.00 | 40.03 | B000 | C |
| ATOM | 8229 | CZ2 | TRP | F | 223 | -23.376 | 50.933 | -26.118 | 1.00 | 42.16 | B000 | C |

| | | | | | | | | | | | | |
|---|---|---|---|---|---|---|---|---|---|---|---|---|
| ATOM | 8230 | CZ3 | TRP | F | 223 | -21.695 | 52.075 | -24.792 | 1.00 | 38.55 | B000 | C |
| ATOM | 8231 | CH2 | TRP | F | 223 | -23.023 | 51.918 | -25.225 | 1.00 | 39.46 | B000 | C |
| ATOM | 8232 | N | VAL | F | 224 | -15.966 | 50.458 | -26.065 | 1.00 | 39.89 | B000 | N |
| ATOM | 8233 | CA | VAL | F | 224 | -14.557 | 50.478 | -25.696 | 1.00 | 41.43 | B000 | C |
| ATOM | 8234 | C | VAL | F | 224 | -14.084 | 49.166 | -25.090 | 1.00 | 43.72 | B000 | C |
| ATOM | 8235 | O | VAL | F | 224 | -12.878 | 48.924 | -25.048 | 1.00 | 41.80 | B000 | O |
| ATOM | 8236 | CB | VAL | F | 224 | -14.257 | 51.635 | -24.718 | 1.00 | 38.57 | B000 | C |
| ATOM | 8237 | CG1 | VAL | F | 224 | -14.434 | 52.978 | -25.419 | 1.00 | 35.91 | B000 | C |
| ATOM | 8238 | CG2 | VAL | F | 224 | -15.154 | 51.522 | -23.509 | 1.00 | 37.24 | B000 | C |
| ATOM | 8239 | N | ASP | F | 225 | -14.978 | 48.330 | -24.572 | 1.00 | 44.78 | B000 | N |
| ATOM | 8240 | CA | ASP | F | 225 | -14.559 | 47.074 | -23.971 | 1.00 | 40.21 | B000 | C |
| ATOM | 8241 | C | ASP | F | 225 | -14.719 | 45.908 | -24.924 | 1.00 | 47.22 | B000 | C |
| ATOM | 8242 | O | ASP | F | 225 | -14.534 | 44.763 | -24.513 | 1.00 | 49.29 | B000 | O |
| ATOM | 8243 | CB | ASP | F | 225 | -15.321 | 46.788 | -22.679 | 1.00 | 41.32 | B000 | C |
| ATOM | 8244 | CG | ASP | F | 225 | -16.790 | 46.487 | -22.914 | 1.00 | 46.80 | B000 | C |
| ATOM | 8245 | OD1 | ASP | F | 225 | -17.305 | 46.755 | -24.021 | 1.00 | 40.18 | B000 | O |
| ATOM | 8246 | OD2 | ASP | F | 225 | -17.426 | 45.946 | -21.982 | 1.00 | 55.82 | B000 | O1- |
| ATOM | 8247 | N | GLY | F | 226 | -15.078 | 46.173 | -26.181 | 1.00 | 46.15 | B000 | N |
| ATOM | 8248 | CA | GLY | F | 226 | -15.202 | 45.151 | -27.189 | 1.00 | 44.93 | B000 | C |
| ATOM | 8249 | C | GLY | F | 226 | -16.606 | 44.610 | -27.394 | 1.00 | 49.56 | B000 | C |
| ATOM | 8250 | O | GLY | F | 226 | -16.858 | 43.993 | -28.440 | 1.00 | 52.14 | B000 | O |
| ATOM | 8251 | N | THR | F | 227 | -17.522 | 44.843 | -26.443 | 1.00 | 45.78 | B000 | N |
| ATOM | 8252 | CA | THR | F | 227 | -18.938 | 44.496 | -26.585 | 1.00 | 38.28 | B000 | C |
| ATOM | 8253 | C | THR | F | 227 | -19.437 | 44.781 | -27.996 | 1.00 | 49.56 | B000 | C |
| ATOM | 8254 | O | THR | F | 227 | -19.216 | 45.868 | -28.544 | 1.00 | 51.44 | B000 | O |
| ATOM | 8255 | CB | THR | F | 227 | -19.779 | 45.282 | -25.571 | 1.00 | 46.77 | B000 | C |
| ATOM | 8256 | OG1 | THR | F | 227 | -19.284 | 45.062 | -24.244 | 1.00 | 49.45 | B000 | O |
| ATOM | 8257 | CG2 | THR | F | 227 | -21.244 | 44.872 | -25.628 | 1.00 | 32.96 | B000 | C |
| ATOM | 8258 | N | ASP | F | 228 | -20.089 | 43.791 | -28.594 | 1.00 | 48.42 | B000 | N |
| ATOM | 8259 | CA | ASP | F | 228 | -20.557 | 43.942 | -29.964 | 1.00 | 52.56 | B000 | C |
| ATOM | 8260 | C | ASP | F | 228 | -21.653 | 44.996 | -30.034 | 1.00 | 47.16 | B000 | C |
| ATOM | 8261 | O | ASP | F | 228 | -22.638 | 44.926 | -29.297 | 1.00 | 54.76 | B000 | O |
| ATOM | 8262 | CB | ASP | F | 228 | -21.080 | 42.609 | -30.513 | 1.00 | 52.04 | B000 | C |
| ATOM | 8263 | CG | ASP | F | 228 | -21.620 | 42.741 | -31.938 | 1.00 | 51.96 | B000 | C |
| ATOM | 8264 | OD1 | ASP | F | 228 | -20.800 | 42.871 | -32.867 | 1.00 | 55.04 | B000 | O |
| ATOM | 8265 | OD2 | ASP | F | 228 | -22.857 | 42.731 | -32.133 | 1.00 | 53.62 | B000 | O1- |
| ATOM | 8266 | N | TYR | F | 229 | -21.480 | 45.975 | -30.926 | 1.00 | 49.63 | B000 | N |
| ATOM | 8267 | CA | TYR | F | 229 | -22.438 | 47.072 | -31.032 | 1.00 | 51.91 | B000 | C |
| ATOM | 8268 | C | TYR | F | 229 | -23.725 | 46.641 | -31.741 | 1.00 | 51.02 | B000 | C |
| ATOM | 8269 | O | TYR | F | 229 | -24.823 | 47.054 | -31.343 | 1.00 | 48.95 | B000 | O |
| ATOM | 8270 | CB | TYR | F | 229 | -21.800 | 48.280 | -31.749 | 1.00 | 41.08 | B000 | C |
| ATOM | 8271 | CG | TYR | F | 229 | -22.801 | 49.368 | -32.155 | 1.00 | 42.20 | B000 | C |
| ATOM | 8272 | CD1 | TYR | F | 229 | -23.377 | 50.216 | -31.208 | 1.00 | 40.58 | B000 | C |
| ATOM | 8273 | CD2 | TYR | F | 229 | -23.178 | 49.530 | -33.482 | 1.00 | 40.58 | B000 | C |
| ATOM | 8274 | CE1 | TYR | F | 229 | -24.293 | 51.223 | -31.586 | 1.00 | 37.62 | B000 | C |
| ATOM | 8275 | CE2 | TYR | F | 229 | -24.088 | 50.511 | -33.864 | 1.00 | 42.49 | B000 | C |
| ATOM | 8276 | CZ | TYR | F | 229 | -24.634 | 51.359 | -32.910 | 1.00 | 40.45 | B000 | C |
| ATOM | 8277 | OH | TYR | F | 229 | -25.527 | 52.323 | -33.311 | 1.00 | 45.55 | B000 | O |
| ATOM | 8278 | N | GLU | F | 230 | -23.605 | 45.833 | -32.800 | 1.00 | 57.91 | B000 | N |
| ATOM | 8279 | CA | GLU | F | 230 | -24.743 | 45.560 | -33.679 | 1.00 | 55.10 | B000 | C |
| ATOM | 8280 | C | GLU | F | 230 | -25.831 | 44.782 | -32.956 | 1.00 | 53.04 | B000 | C |
| ATOM | 8281 | O | GLU | F | 230 | -27.014 | 45.118 | -33.055 | 1.00 | 52.09 | B000 | O |
| ATOM | 8282 | CB | GLU | F | 230 | -24.273 | 44.782 | -34.909 | 1.00 | 58.23 | B000 | C |
| ATOM | 8283 | CG | GLU | F | 230 | -25.270 | 44.727 | -36.048 | 1.00 | 61.69 | B000 | C |
| ATOM | 8284 | CD | GLU | F | 230 | -25.912 | 46.067 | -36.325 | 1.00 | 76.73 | B000 | C |
| ATOM | 8285 | OE1 | GLU | F | 230 | -27.160 | 46.119 | -36.285 | 1.00 | 75.40 | B000 | O |
| ATOM | 8286 | OE2 | GLU | F | 230 | -25.178 | 47.067 | -36.556 | 1.00 | 78.82 | B000 | O1- |
| ATOM | 8287 | N | THR | F | 231 | -25.449 | 43.767 | -32.201 | 1.00 | 51.54 | B000 | N |
| ATOM | 8288 | CA | THR | F | 231 | -26.410 | 42.954 | -31.478 | 1.00 | 55.16 | B000 | C |
| ATOM | 8289 | C | THR | F | 231 | -26.687 | 43.461 | -30.072 | 1.00 | 58.68 | B000 | C |
| ATOM | 8290 | O | THR | F | 231 | -27.516 | 42.866 | -29.371 | 1.00 | 54.74 | B000 | O |
| ATOM | 8291 | CB | THR | F | 231 | -25.922 | 41.510 | -31.415 | 1.00 | 49.05 | B000 | C |
| ATOM | 8292 | OG1 | THR | F | 231 | -24.684 | 41.463 | -30.698 | 1.00 | 52.11 | B000 | O |
| ATOM | 8293 | CG2 | THR | F | 231 | -25.715 | 40.964 | -32.820 | 1.00 | 45.52 | B000 | C |
| ATOM | 8294 | N | GLY | F | 232 | -26.030 | 44.546 | -29.646 | 1.00 | 55.48 | B000 | N |
| ATOM | 8295 | CA | GLY | F | 232 | -26.155 | 45.022 | -28.285 | 1.00 | 46.35 | B000 | C |
| ATOM | 8296 | C | GLY | F | 232 | -27.303 | 46.007 | -28.088 | 1.00 | 42.93 | B000 | C |
| ATOM | 8297 | O | GLY | F | 232 | -27.997 | 46.417 | -29.016 | 1.00 | 45.48 | B000 | O |
| ATOM | 8298 | N | PHE | F | 233 | -27.484 | 46.390 | -26.830 | 1.00 | 40.91 | B000 | N |
| ATOM | 8299 | CA | PHE | F | 233 | -28.463 | 47.401 | -26.462 | 1.00 | 38.08 | B000 | C |
| ATOM | 8300 | C | PHE | F | 233 | -28.162 | 48.738 | -27.146 | 1.00 | 45.77 | B000 | C |

| | | | | | | | | | | | | |
|---|---|---|---|---|---|---|---|---|---|---|---|---|
| ATOM | 8301 | O | PHE | F | 233 | -26.997 | 49.125 | -27.305 | 1.00 | 39.98 | B000 | O |
| ATOM | 8302 | CB | PHE | F | 233 | -28.440 | 47.548 | -24.939 | 1.00 | 42.77 | B000 | C |
| ATOM | 8303 | CG | PHE | F | 233 | -29.411 | 48.544 | -24.406 | 1.00 | 46.27 | B000 | C |
| ATOM | 8304 | CD1 | PHE | F | 233 | -30.749 | 48.224 | -24.274 | 1.00 | 47.88 | B000 | C |
| ATOM | 8305 | CD2 | PHE | F | 233 | -28.981 | 49.804 | -24.013 | 1.00 | 42.70 | B000 | C |
| ATOM | 8306 | CE1 | PHE | F | 233 | -31.650 | 49.153 | -23.777 | 1.00 | 51.88 | B000 | C |
| ATOM | 8307 | CE2 | PHE | F | 233 | -29.871 | 50.726 | -23.515 | 1.00 | 41.09 | B000 | C |
| ATOM | 8308 | CZ | PHE | F | 233 | -31.206 | 50.404 | -23.391 | 1.00 | 45.56 | B000 | C |
| ATOM | 8309 | N | LYS | F | 234 | -29.225 | 49.439 | -27.562 | 1.00 | 40.43 | B000 | N |
| ATOM | 8310 | CA | LYS | F | 234 | -29.131 | 50.788 | -28.118 | 1.00 | 41.63 | B000 | C |
| ATOM | 8311 | C | LYS | F | 234 | -30.309 | 51.634 | -27.654 | 1.00 | 46.34 | B000 | C |
| ATOM | 8312 | O | LYS | F | 234 | -31.441 | 51.156 | -27.610 | 1.00 | 48.63 | B000 | O |
| ATOM | 8313 | CB | LYS | F | 234 | -29.123 | 50.778 | -29.649 | 1.00 | 44.55 | B000 | C |
| ATOM | 8314 | CG | LYS | F | 234 | -27.951 | 50.067 | -30.289 | 1.00 | 40.30 | B000 | C |
| ATOM | 8315 | CD | LYS | F | 234 | -28.092 | 50.103 | -31.807 | 1.00 | 43.14 | B000 | C |
| ATOM | 8316 | CE | LYS | F | 234 | -27.123 | 49.127 | -32.446 | 1.00 | 48.35 | B000 | C |
| ATOM | 8317 | NZ | LYS | F | 234 | -27.471 | 47.729 | -32.045 | 1.00 | 53.70 | B000 | N1+ |
| ATOM | 8318 | N | ASN | F | 235 | -30.060 | 52.909 | -27.362 | 1.00 | 47.83 | B000 | N |
| ATOM | 8319 | CA | ASN | F | 235 | -31.120 | 53.815 | -26.906 | 1.00 | 38.15 | B000 | C |
| ATOM | 8320 | C | ASN | F | 235 | -30.936 | 55.217 | -27.509 | 1.00 | 39.90 | B000 | C |
| ATOM | 8321 | O | ASN | F | 235 | -30.978 | 56.235 | -26.819 | 1.00 | 38.13 | B000 | O |
| ATOM | 8322 | CB | ASN | F | 235 | -31.154 | 53.827 | -25.376 | 1.00 | 37.57 | B000 | C |
| ATOM | 8323 | CG | ASN | F | 235 | -32.291 | 54.652 | -24.816 | 1.00 | 44.55 | B000 | C |
| ATOM | 8324 | OD1 | ASN | F | 235 | -33.337 | 54.797 | -25.445 | 1.00 | 47.07 | B000 | O |
| ATOM | 8325 | ND2 | ASN | F | 235 | -32.088 | 55.204 | -23.620 | 1.00 | 46.24 | B000 | N |
| ATOM | 8326 | N | TRP | F | 236 | -30.749 | 55.278 | -28.826 | 1.00 | 36.87 | B000 | N |
| ATOM | 8327 | CA | TRP | F | 236 | -30.517 | 56.540 | -29.518 | 1.00 | 38.92 | B000 | C |
| ATOM | 8328 | C | TRP | F | 236 | -31.753 | 57.439 | -29.510 | 1.00 | 41.60 | B000 | C |
| ATOM | 8329 | O | TRP | F | 236 | -32.890 | 56.967 | -29.580 | 1.00 | 44.93 | B000 | O |
| ATOM | 8330 | CB | TRP | F | 236 | -30.123 | 56.288 | -30.973 | 1.00 | 32.28 | B000 | C |
| ATOM | 8331 | CG | TRP | F | 236 | -28.813 | 55.606 | -31.170 | 1.00 | 44.09 | B000 | C |
| ATOM | 8332 | CD1 | TRP | F | 236 | -28.612 | 54.296 | -31.518 | 1.00 | 40.32 | B000 | C |
| ATOM | 8333 | CD2 | TRP | F | 236 | -27.511 | 56.192 | -31.037 | 1.00 | 41.90 | B000 | C |
| ATOM | 8334 | NE1 | TRP | F | 236 | -27.266 | 54.034 | -31.608 | 1.00 | 35.68 | B000 | N |
| ATOM | 8335 | CE2 | TRP | F | 236 | -26.568 | 55.182 | -31.327 | 1.00 | 41.49 | B000 | C |
| ATOM | 8336 | CE3 | TRP | F | 236 | -27.051 | 57.474 | -30.700 | 1.00 | 37.67 | B000 | C |
| ATOM | 8337 | CZ2 | TRP | F | 236 | -25.183 | 55.420 | -31.300 | 1.00 | 41.56 | B000 | C |
| ATOM | 8338 | CZ3 | TRP | F | 236 | -25.686 | 57.715 | -30.683 | 1.00 | 37.32 | B000 | C |
| ATOM | 8339 | CH2 | TRP | F | 236 | -24.762 | 56.689 | -30.982 | 1.00 | 41.77 | B000 | C |
| ATOM | 8340 | N | ARG | F | 237 | -31.508 | 58.757 | -29.481 | 1.00 | 35.83 | B000 | N |
| ATOM | 8341 | CA | ARG | F | 237 | -32.537 | 59.734 | -29.801 | 1.00 | 44.59 | B000 | C |
| ATOM | 8342 | C | ARG | F | 237 | -33.103 | 59.444 | -31.181 | 1.00 | 48.74 | B000 | C |
| ATOM | 8343 | O | ARG | F | 237 | -32.434 | 58.828 | -32.017 | 1.00 | 49.21 | B000 | O |
| ATOM | 8344 | CB | ARG | F | 237 | -31.976 | 61.164 | -29.788 | 1.00 | 36.97 | B000 | C |
| ATOM | 8345 | CG | ARG | F | 237 | -31.858 | 61.766 | -28.411 | 1.00 | 45.68 | B000 | C |
| ATOM | 8346 | CD | ARG | F | 237 | -33.080 | 62.584 | -28.086 | 1.00 | 53.50 | B000 | C |
| ATOM | 8347 | NE | ARG | F | 237 | -32.997 | 63.225 | -26.778 | 1.00 | 54.24 | B000 | N |
| ATOM | 8348 | CZ | ARG | F | 237 | -32.798 | 64.523 | -26.580 | 1.00 | 66.27 | B000 | C |
| ATOM | 8349 | NH1 | ARG | F | 237 | -32.738 | 65.003 | -25.335 | 1.00 | 53.38 | B000 | N1+ |
| ATOM | 8350 | NH2 | ARG | F | 237 | -32.650 | 65.337 | -27.621 | 1.00 | 69.85 | B000 | N |
| ATOM | 8351 | N | PRO | F | 238 | -34.326 | 59.897 | -31.447 | 1.00 | 53.51 | B000 | N |
| ATOM | 8352 | CA | PRO | F | 238 | -34.896 | 59.751 | -32.790 | 1.00 | 53.89 | B000 | C |
| ATOM | 8353 | C | PRO | F | 238 | -33.963 | 60.284 | -33.869 | 1.00 | 55.90 | B000 | C |
| ATOM | 8354 | O | PRO | F | 238 | -33.458 | 61.409 | -33.783 | 1.00 | 47.93 | B000 | O |
| ATOM | 8355 | CB | PRO | F | 238 | -36.184 | 60.577 | -32.707 | 1.00 | 53.03 | B000 | C |
| ATOM | 8356 | CG | PRO | F | 238 | -36.568 | 60.511 | -31.263 | 1.00 | 50.27 | B000 | C |
| ATOM | 8357 | CD | PRO | F | 238 | -35.285 | 60.495 | -30.496 | 1.00 | 54.51 | B000 | C |
| ATOM | 8358 | N | GLU | F | 239 | -33.727 | 59.454 | -34.887 | 1.00 | 48.36 | B000 | N |
| ATOM | 8359 | CA | GLU | F | 239 | -32.951 | 59.792 | -36.076 | 1.00 | 55.08 | B000 | C |
| ATOM | 8360 | C | GLU | F | 239 | -31.463 | 59.925 | -35.799 | 1.00 | 51.71 | B000 | C |
| ATOM | 8361 | O | GLU | F | 239 | -30.717 | 60.378 | -36.673 | 1.00 | 53.39 | B000 | O |
| ATOM | 8362 | CB | GLU | F | 239 | -33.471 | 61.081 | -36.715 | 1.00 | 54.02 | B000 | C |
| ATOM | 8363 | CG | GLU | F | 239 | -34.894 | 60.933 | -37.217 | 1.00 | 64.17 | B000 | C |
| ATOM | 8364 | CD | GLU | F | 239 | -35.603 | 62.267 | -37.357 | 1.00 | 82.12 | B000 | C |
| ATOM | 8365 | OE1 | GLU | F | 239 | -34.947 | 63.318 | -37.174 | 1.00 | 79.31 | B000 | O |
| ATOM | 8366 | OE2 | GLU | F | 239 | -36.828 | 62.260 | -37.609 | 1.00 | 90.31 | B000 | O1- |
| ATOM | 8367 | N | GLN | F | 240 | -31.010 | 59.539 | -34.613 | 1.00 | 45.35 | B000 | N |
| ATOM | 8368 | CA | GLN | F | 240 | -29.594 | 59.480 | -34.313 | 1.00 | 45.02 | B000 | C |
| ATOM | 8369 | C | GLN | F | 240 | -29.207 | 58.001 | -34.305 | 1.00 | 40.34 | B000 | C |
| ATOM | 8370 | O | GLN | F | 240 | -30.041 | 57.164 | -33.989 | 1.00 | 38.58 | B000 | O |
| ATOM | 8371 | CB | GLN | F | 240 | -29.274 | 60.163 | -32.964 | 1.00 | 40.27 | B000 | C |

| | | | | | | | | | | | | |
|---|---|---|---|---|---|---|---|---|---|---|---|---|
| ATOM | 8372 | CG | GLN | F | 240 | -29.798 | 61.588 | -32.801 | 1.00 | 35.48 | B000 | C |
| ATOM | 8373 | CD | GLN | F | 240 | -29.552 | 62.460 | -34.038 | 1.00 | 52.50 | B000 | C |
| ATOM | 8374 | OE1 | GLN | F | 240 | -28.448 | 62.496 | -34.599 | 1.00 | 44.08 | B000 | O |
| ATOM | 8375 | NE2 | GLN | F | 240 | -30.594 | 63.166 | -34.470 | 1.00 | 52.73 | B000 | N |
| ATOM | 8376 | N | PRO | F | 241 | -27.933 | 57.680 | -34.609 | 1.00 | 41.65 | B000 | N |
| ATOM | 8377 | CA | PRO | F | 241 | -26.875 | 58.646 | -34.945 | 1.00 | 36.96 | B000 | C |
| ATOM | 8378 | C | PRO | F | 241 | -27.011 | 59.145 | -36.366 | 1.00 | 42.81 | B000 | C |
| ATOM | 8379 | O | PRO | F | 241 | -27.998 | 58.769 | -37.004 | 1.00 | 45.53 | B000 | O |
| ATOM | 8380 | CB | PRO | F | 241 | -25.586 | 57.853 | -34.749 | 1.00 | 38.94 | B000 | C |
| ATOM | 8381 | CG | PRO | F | 241 | -25.986 | 56.428 | -34.992 | 1.00 | 43.27 | B000 | C |
| ATOM | 8382 | CD | PRO | F | 241 | -27.423 | 56.298 | -34.511 | 1.00 | 38.99 | B000 | C |
| ATOM | 8383 | N | ASP | F | 242 | -26.097 | 60.013 | -36.818 | 1.00 | 39.75 | B000 | N |
| ATOM | 8384 | CA | ASP | F | 242 | -26.139 | 60.504 | -38.190 | 1.00 | 36.75 | B000 | C |
| ATOM | 8385 | C | ASP | F | 242 | -26.229 | 59.323 | -39.143 | 1.00 | 43.99 | B000 | C |
| ATOM | 8386 | O | ASP | F | 242 | -25.504 | 58.332 | -38.991 | 1.00 | 46.47 | B000 | O |
| ATOM | 8387 | CB | ASP | F | 242 | -24.894 | 61.335 | -38.515 | 1.00 | 49.95 | B000 | C |
| ATOM | 8388 | CG | ASP | F | 242 | -24.677 | 62.473 | -37.551 | 1.00 | 48.49 | B000 | C |
| ATOM | 8389 | OD1 | ASP | F | 242 | -25.683 | 63.064 | -37.104 | 1.00 | 52.88 | B000 | O |
| ATOM | 8390 | OD2 | ASP | F | 242 | -23.497 | 62.767 | -37.248 | 1.00 | 50.34 | B000 | O |
| ATOM | 8391 | N | ASP | F | 243 | -27.147 | 59.405 | -40.097 | 1.00 | 38.82 | B000 | N |
| ATOM | 8392 | CA | ASP | F | 243 | -27.412 | 58.269 | -40.967 | 1.00 | 51.83 | B000 | C |
| ATOM | 8393 | C | ASP | F | 243 | -26.703 | 58.364 | -42.308 | 1.00 | 46.33 | B000 | C |
| ATOM | 8394 | O | ASP | F | 243 | -26.951 | 57.526 | -43.172 | 1.00 | 49.46 | B000 | O |
| ATOM | 8395 | CB | ASP | F | 243 | -28.919 | 58.079 | -41.193 | 1.00 | 42.32 | B000 | C |
| ATOM | 8396 | CG | ASP | F | 243 | -29.605 | 59.327 | -41.786 | 1.00 | 64.08 | B000 | C |
| ATOM | 8397 | OD1 | ASP | F | 243 | -28.935 | 60.346 | -42.070 | 1.00 | 56.96 | B000 | O |
| ATOM | 8398 | OD2 | ASP | F | 243 | -30.842 | 59.286 | -41.971 | 1.00 | 77.59 | B000 | O |
| ATOM | 8399 | N | TRP | F | 244 | -25.799 | 59.322 | -42.495 | 1.00 | 46.94 | B000 | N |
| ATOM | 8400 | CA | TRP | F | 244 | -25.235 | 59.541 | -43.822 | 1.00 | 43.08 | B000 | C |
| ATOM | 8401 | C | TRP | F | 244 | -24.046 | 58.643 | -44.134 | 1.00 | 41.86 | B000 | C |
| ATOM | 8402 | O | TRP | F | 244 | -23.528 | 58.721 | -45.250 | 1.00 | 43.66 | B000 | O |
| ATOM | 8403 | CB | TRP | F | 244 | -24.765 | 60.989 | -43.994 | 1.00 | 49.74 | B000 | C |
| ATOM | 8404 | CG | TRP | F | 244 | -25.371 | 61.991 | -43.064 | 1.00 | 50.88 | B000 | C |
| ATOM | 8405 | CD1 | TRP | F | 244 | -26.695 | 62.261 | -42.902 | 1.00 | 52.58 | B000 | C |
| ATOM | 8406 | CD2 | TRP | F | 244 | -24.674 | 62.891 | -42.206 | 1.00 | 42.95 | B000 | C |
| ATOM | 8407 | NE1 | TRP | F | 244 | -26.876 | 63.254 | -41.976 | 1.00 | 50.24 | B000 | N |
| ATOM | 8408 | CE2 | TRP | F | 244 | -25.650 | 63.672 | -41.536 | 1.00 | 51.72 | B000 | C |
| ATOM | 8409 | CE3 | TRP | F | 244 | -23.333 | 63.117 | -41.940 | 1.00 | 40.66 | B000 | C |
| ATOM | 8410 | CZ2 | TRP | F | 244 | -25.322 | 64.660 | -40.608 | 1.00 | 34.70 | B000 | C |
| ATOM | 8411 | CZ3 | TRP | F | 244 | -23.008 | 64.096 | -41.014 | 1.00 | 51.45 | B000 | C |
| ATOM | 8412 | CH2 | TRP | F | 244 | -24.002 | 64.849 | -40.357 | 1.00 | 45.14 | B000 | C |
| ATOM | 8413 | N | TYR | F | 245 | -23.649 | 57.749 | -43.225 | 1.00 | 38.07 | B000 | N |
| ATOM | 8414 | CA | TYR | F | 245 | -22.447 | 56.950 | -43.423 | 1.00 | 34.89 | B000 | C |
| ATOM | 8415 | C | TYR | F | 245 | -22.709 | 55.483 | -43.712 | 1.00 | 44.67 | B000 | C |
| ATOM | 8416 | O | TYR | F | 245 | -21.778 | 54.779 | -44.103 | 1.00 | 52.17 | B000 | O |
| ATOM | 8417 | CB | TYR | F | 245 | -21.529 | 57.030 | -42.191 | 1.00 | 38.23 | B000 | C |
| ATOM | 8418 | CG | TYR | F | 245 | -21.240 | 58.429 | -41.761 | 1.00 | 43.82 | B000 | C |
| ATOM | 8419 | CD1 | TYR | F | 245 | -20.344 | 59.219 | -42.479 | 1.00 | 42.91 | B000 | C |
| ATOM | 8420 | CD2 | TYR | F | 245 | -21.868 | 58.976 | -40.638 | 1.00 | 38.95 | B000 | C |
| ATOM | 8421 | CE1 | TYR | F | 245 | -20.090 | 60.513 | -42.101 | 1.00 | 39.31 | B000 | C |
| ATOM | 8422 | CE2 | TYR | F | 245 | -21.616 | 60.266 | -40.249 | 1.00 | 40.49 | B000 | C |
| ATOM | 8423 | CZ | TYR | F | 245 | -20.719 | 61.032 | -40.978 | 1.00 | 43.99 | B000 | C |
| ATOM | 8424 | OH | TYR | F | 245 | -20.449 | 62.323 | -40.589 | 1.00 | 46.79 | B000 | O |
| ATOM | 8425 | N | GLY | F | 246 | -23.918 | 54.993 | -43.508 | 1.00 | 42.86 | B000 | N |
| ATOM | 8426 | CA | GLY | F | 246 | -24.172 | 53.575 | -43.641 | 1.00 | 47.08 | B000 | C |
| ATOM | 8427 | C | GLY | F | 246 | -24.546 | 52.918 | -42.321 | 1.00 | 48.63 | B000 | C |
| ATOM | 8428 | O | GLY | F | 246 | -24.464 | 53.502 | -41.238 | 1.00 | 46.31 | B000 | O |
| ATOM | 8429 | N | HIS | F | 247 | -25.011 | 51.682 | -42.461 | 1.00 | 45.64 | B000 | N |
| ATOM | 8430 | CA | HIS | F | 247 | -25.504 | 50.908 | -41.338 | 1.00 | 45.98 | B000 | C |
| ATOM | 8431 | C | HIS | F | 247 | -24.400 | 50.682 | -40.309 | 1.00 | 49.00 | B000 | C |
| ATOM | 8432 | O | HIS | F | 247 | -23.329 | 50.161 | -40.640 | 1.00 | 47.43 | B000 | O |
| ATOM | 8433 | CB | HIS | F | 247 | -26.052 | 49.570 | -41.836 | 1.00 | 48.96 | B000 | C |
| ATOM | 8434 | CG | HIS | F | 247 | -26.470 | 48.656 | -40.731 | 1.00 | 52.43 | B000 | C |
| ATOM | 8435 | ND1 | HIS | F | 247 | -27.650 | 48.819 | -40.037 | 1.00 | 52.47 | B000 | N |
| ATOM | 8436 | CD2 | HIS | F | 247 | -25.835 | 47.606 | -40.157 | 1.00 | 55.93 | B000 | C |
| ATOM | 8437 | CE1 | HIS | F | 247 | -27.737 | 47.891 | -39.102 | 1.00 | 55.95 | B000 | C |
| ATOM | 8438 | NE2 | HIS | F | 247 | -26.649 | 47.142 | -39.154 | 1.00 | 65.30 | B000 | N |
| ATOM | 8439 | N | GLY | F | 248 | -24.656 | 51.080 | -39.063 | 1.00 | 45.09 | B000 | N |
| ATOM | 8440 | CA | GLY | F | 248 | -23.694 | 50.884 | -37.992 | 1.00 | 43.43 | B000 | C |
| ATOM | 8441 | C | GLY | F | 248 | -22.442 | 51.723 | -38.086 | 1.00 | 44.64 | B000 | C |
| ATOM | 8442 | O | GLY | F | 248 | -21.463 | 51.431 | -37.391 | 1.00 | 45.17 | B000 | O |

| | | | | | | | | | | | | |
|---|---|---|---|---|---|---|---|---|---|---|---|---|
| ATOM | 8443 | N | LEU | F | 249 | -22.449 | 52.777 | -38.907 | 1.00 | 44.92 | B000 | N |
| ATOM | 8444 | CA | LEU | F | 249 | -21.244 | 53.532 | -39.232 | 1.00 | 47.53 | B000 | C |
| ATOM | 8445 | C | LEU | F | 249 | -21.355 | 55.001 | -38.833 | 1.00 | 45.44 | B000 | C |
| ATOM | 8446 | O | LEU | F | 249 | -22.456 | 55.561 | -38.758 | 1.00 | 38.63 | B000 | O |
| ATOM | 8447 | CB | LEU | F | 249 | -20.942 | 53.464 | -40.737 | 1.00 | 45.64 | B000 | C |
| ATOM | 8448 | CG | LEU | F | 249 | -20.640 | 52.090 | -41.349 | 1.00 | 49.29 | B000 | C |
| ATOM | 8449 | CD1 | LEU | F | 249 | -20.212 | 52.237 | -42.809 | 1.00 | 41.47 | B000 | C |
| ATOM | 8450 | CD2 | LEU | F | 249 | -19.590 | 51.359 | -40.538 | 1.00 | 35.93 | B000 | C |
| ATOM | 8451 | N | GLY | F | 250 | -20.186 | 55.619 | -38.598 | 1.00 | 43.79 | B000 | N |
| ATOM | 8452 | CA | GLY | F | 250 | -20.033 | 57.062 | -38.423 | 1.00 | 40.09 | B000 | C |
| ATOM | 8453 | C | GLY | F | 250 | -18.890 | 57.625 | -39.262 | 1.00 | 46.39 | B000 | C |
| ATOM | 8454 | O | GLY | F | 250 | -18.312 | 56.892 | -40.068 | 1.00 | 46.10 | B000 | O |
| ATOM | 8455 | N | ALA | F | 251 | -18.553 | 58.910 | -39.081 | 1.00 | 38.76 | B000 | N |
| ATOM | 8456 | CA | ALA | F | 251 | -17.474 | 59.596 | -39.795 | 1.00 | 44.18 | B000 | C |
| ATOM | 8457 | C | ALA | F | 251 | -16.114 | 59.542 | -39.122 | 1.00 | 41.29 | B000 | C |
| ATOM | 8458 | O | ALA | F | 251 | -15.172 | 60.113 | -39.673 | 1.00 | 48.88 | B000 | O |
| ATOM | 8459 | CB | ALA | F | 251 | -17.762 | 61.085 | -39.974 | 1.00 | 67.76 | B000 | C |
| ATOM | 8460 | N | GLY | F | 252 | -15.989 | 58.946 | -37.942 | 1.00 | 43.97 | B000 | N |
| ATOM | 8461 | CA | GLY | F | 252 | -14.714 | 58.903 | -37.255 | 1.00 | 44.53 | B000 | C |
| ATOM | 8462 | C | GLY | F | 252 | -14.440 | 60.030 | -36.276 | 1.00 | 47.60 | B000 | C |
| ATOM | 8463 | O | GLY | F | 252 | -13.427 | 59.971 | -35.575 | 1.00 | 55.35 | B000 | O |
| ATOM | 8464 | N | GLU | F | 253 | -15.306 | 61.045 | -36.183 | 1.00 | 45.85 | B000 | N |
| ATOM | 8465 | CA | GLU | F | 253 | -15.090 | 62.165 | -35.268 | 1.00 | 42.89 | B000 | C |
| ATOM | 8466 | C | GLU | F | 253 | -16.069 | 62.200 | -34.096 | 1.00 | 42.96 | B000 | C |
| ATOM | 8467 | O | GLU | F | 253 | -15.975 | 63.117 | -33.272 | 1.00 | 41.71 | B000 | O |
| ATOM | 8468 | CB | GLU | F | 253 | -15.188 | 63.512 | -36.017 | 1.00 | 43.68 | B000 | C |
| ATOM | 8469 | CG | GLU | F | 253 | -14.363 | 63.614 | -37.321 | 1.00 | 57.15 | B000 | C |
| ATOM | 8470 | CD | GLU | F | 253 | -12.875 | 63.967 | -37.117 | 1.00 | 70.88 | B000 | C |
| ATOM | 8471 | OE1 | GLU | F | 253 | -12.568 | 65.139 | -36.771 | 1.00 | 71.64 | B000 | O |
| ATOM | 8472 | OE2 | GLU | F | 253 | -12.008 | 63.079 | -37.331 | 1.00 | 63.29 | B000 | O |
| ATOM | 8473 | N | ASP | F | 254 | -17.000 | 61.242 | -33.982 | 1.00 | 36.37 | B000 | N |
| ATOM | 8474 | CA | ASP | F | 254 | -18.107 | 61.378 | -33.045 | 1.00 | 37.71 | B000 | C |
| ATOM | 8475 | C | ASP | F | 254 | -17.988 | 60.412 | -31.877 | 1.00 | 36.53 | B000 | C |
| ATOM | 8476 | O | ASP | F | 254 | -17.374 | 59.349 | -31.973 | 1.00 | 36.91 | B000 | O |
| ATOM | 8477 | CB | ASP | F | 254 | -19.473 | 61.157 | -33.717 | 1.00 | 35.74 | B000 | C |
| ATOM | 8478 | CG | ASP | F | 254 | -20.006 | 62.400 | -34.396 | 1.00 | 38.14 | B000 | C |
| ATOM | 8479 | OD2 | ASP | F | 254 | -21.148 | 62.350 | -34.936 | 1.00 | 36.55 | B000 | O |
| ATOM | 8480 | OD1 | ASP | F | 254 | -19.280 | 63.427 | -34.386 | 1.00 | 41.05 | B000 | O1- |
| ATOM | 8481 | N | CYS | F | 255 | -18.603 | 60.799 | -30.769 | 1.00 | 38.85 | B000 | N |
| ATOM | 8482 | CA | CYS | F | 255 | -18.733 | 59.942 | -29.606 | 1.00 | 28.30 | B000 | C |
| ATOM | 8483 | C | CYS | F | 255 | -20.176 | 60.039 | -29.135 | 1.00 | 32.80 | B000 | C |
| ATOM | 8484 | O | CYS | F | 255 | -20.878 | 61.013 | -29.418 | 1.00 | 34.41 | B000 | O |
| ATOM | 8485 | CB | CYS | F | 255 | -17.749 | 60.342 | -28.515 | 1.00 | 32.19 | B000 | C |
| ATOM | 8486 | SG | CYS | F | 255 | -15.954 | 60.090 | -28.944 | 1.00 | 42.14 | B000 | S |
| ATOM | 8487 | N | ALA | F | 256 | -20.630 | 59.006 | -28.447 | 1.00 | 31.53 | B000 | N |
| ATOM | 8488 | CA | ALA | F | 256 | -22.004 | 58.933 | -27.991 | 1.00 | 30.16 | B000 | C |
| ATOM | 8489 | C | ALA | F | 256 | -22.096 | 59.438 | -26.562 | 1.00 | 34.02 | B000 | C |
| ATOM | 8490 | O | ALA | F | 256 | -21.281 | 59.074 | -25.706 | 1.00 | 31.73 | B000 | O |
| ATOM | 8491 | CB | ALA | F | 256 | -22.530 | 57.498 | -28.071 | 1.00 | 28.94 | B000 | C |
| ATOM | 8492 | N | HIS | F | 257 | -23.103 | 60.261 | -26.301 | 1.00 | 28.80 | B000 | N |
| ATOM | 8493 | CA | HIS | F | 257 | -23.347 | 60.726 | -24.947 | 1.00 | 33.62 | B000 | C |
| ATOM | 8494 | C | HIS | F | 257 | -24.822 | 60.589 | -24.608 | 1.00 | 29.12 | B000 | C |
| ATOM | 8495 | O | HIS | F | 257 | -25.696 | 60.659 | -25.483 | 1.00 | 29.71 | B000 | O |
| ATOM | 8496 | CB | HIS | F | 257 | -22.895 | 62.209 | -24.730 | 1.00 | 31.05 | B000 | C |
| ATOM | 8497 | CG | HIS | F | 257 | -23.622 | 63.196 | -25.584 | 1.00 | 27.54 | B000 | C |
| ATOM | 8498 | ND1 | HIS | F | 257 | -24.699 | 63.926 | -25.124 | 1.00 | 35.11 | B000 | N |
| ATOM | 8499 | CD2 | HIS | F | 257 | -23.433 | 63.578 | -26.873 | 1.00 | 31.63 | B000 | C |
| ATOM | 8500 | CE1 | HIS | F | 257 | -25.140 | 64.719 | -26.088 | 1.00 | 30.09 | B000 | C |
| ATOM | 8501 | NE2 | HIS | F | 257 | -24.389 | 64.529 | -27.160 | 1.00 | 34.93 | B000 | N |
| ATOM | 8502 | N | PHE | F | 258 | -25.083 | 60.369 | -23.324 | 1.00 | 31.39 | B000 | N |
| ATOM | 8503 | CA | PHE | F | 258 | -26.433 | 60.529 | -22.810 | 1.00 | 37.72 | B000 | C |
| ATOM | 8504 | C | PHE | F | 258 | -26.830 | 61.993 | -22.915 | 1.00 | 35.33 | B000 | C |
| ATOM | 8505 | O | PHE | F | 258 | -26.020 | 62.879 | -22.636 | 1.00 | 37.08 | B000 | O |
| ATOM | 8506 | CB | PHE | F | 258 | -26.519 | 60.088 | -21.354 | 1.00 | 31.41 | B000 | C |
| ATOM | 8507 | CG | PHE | F | 258 | -26.032 | 58.691 | -21.094 | 1.00 | 30.94 | B000 | C |
| ATOM | 8508 | CD1 | PHE | F | 258 | -26.884 | 57.598 | -21.263 | 1.00 | 34.60 | B000 | C |
| ATOM | 8509 | CD2 | PHE | F | 258 | -24.750 | 58.465 | -20.612 | 1.00 | 31.04 | B000 | C |
| ATOM | 8510 | CE1 | PHE | F | 258 | -26.449 | 56.297 | -20.988 | 1.00 | 35.62 | B000 | C |
| ATOM | 8511 | CE2 | PHE | F | 258 | -24.295 | 57.160 | -20.350 | 1.00 | 37.38 | B000 | C |
| ATOM | 8512 | CZ | PHE | F | 258 | -25.156 | 56.076 | -20.534 | 1.00 | 34.44 | B000 | C |
| ATOM | 8513 | N | THR | F | 259 | -28.072 | 62.242 | -23.340 | 1.00 | 39.97 | B000 | N |

| | | | | | | | | | | | |
|---|---|---|---|---|---|---|---|---|---|---|---|
| ATOM | 8514 | CA | THR | F | 259 | -28.683 | 63.565 | -23.297 | 1.00 | 37.91 | B000 C |
| ATOM | 8515 | C | THR | F | 259 | -29.508 | 63.699 | -22.023 | 1.00 | 38.85 | B000 C |
| ATOM | 8516 | O | THR | F | 259 | -29.572 | 62.785 | -21.197 | 1.00 | 42.20 | B000 O |
| ATOM | 8517 | CB | THR | F | 259 | -29.566 | 63.817 | -24.514 | 1.00 | 38.22 | B000 C |
| ATOM | 8518 | OG1 | THR | F | 259 | -30.726 | 62.979 | -24.427 | 1.00 | 40.99 | B000 O |
| ATOM | 8519 | CG2 | THR | F | 259 | -28.812 | 63.526 | -25.789 | 1.00 | 35.73 | B000 C |
| ATOM | 8520 | N | ASP | F | 260 | -30.176 | 64.845 | -21.874 | 1.00 | 41.22 | B000 N |
| ATOM | 8521 | CA | ASP | F | 260 | -30.859 | 65.107 | -20.611 | 1.00 | 45.54 | B000 C |
| ATOM | 8522 | C | ASP | F | 260 | -32.098 | 64.241 | -20.394 | 1.00 | 44.40 | B000 C |
| ATOM | 8523 | O | ASP | F | 260 | -32.576 | 64.175 | -19.260 | 1.00 | 47.44 | B000 O |
| ATOM | 8524 | CB | ASP | F | 260 | -31.211 | 66.595 | -20.486 | 1.00 | 42.85 | B000 C |
| ATOM | 8525 | CG | ASP | F | 260 | -32.026 | 67.112 | -21.654 | 1.00 | 50.76 | B000 C |
| ATOM | 8526 | OD1 | ASP | F | 260 | -32.520 | 66.298 | -22.465 | 1.00 | 48.72 | B000 O |
| ATOM | 8527 | OD2 | ASP | F | 260 | -32.156 | 68.351 | -21.769 | 1.00 | 54.59 | B000 O1- |
| ATOM | 8528 | N | ASP | F | 261 | -32.598 | 63.531 | -21.406 | 1.00 | 42.99 | B000 N |
| ATOM | 8529 | CA | ASP | F | 261 | -33.649 | 62.544 | -21.167 | 1.00 | 41.51 | B000 C |
| ATOM | 8530 | C | ASP | F | 261 | -33.118 | 61.115 | -21.152 | 1.00 | 43.32 | B000 C |
| ATOM | 8531 | O | ASP | F | 261 | -33.911 | 60.169 | -21.146 | 1.00 | 48.34 | B000 O |
| ATOM | 8532 | CB | ASP | F | 261 | -34.795 | 62.676 | -22.192 | 1.00 | 37.72 | B000 C |
| ATOM | 8533 | CG | ASP | F | 261 | -34.432 | 62.185 | -23.606 | 1.00 | 50.07 | B000 C |
| ATOM | 8534 | OD1 | ASP | F | 261 | -33.408 | 61.504 | -23.808 | 1.00 | 49.28 | B000 O |
| ATOM | 8535 | OD2 | ASP | F | 261 | -35.212 | 62.470 | -24.543 | 1.00 | 57.06 | B000 O1- |
| ATOM | 8536 | N | GLY | F | 262 | -31.801 | 60.929 | -21.192 | 1.00 | 37.84 | B000 N |
| ATOM | 8537 | CA | GLY | F | 262 | -31.223 | 59.600 | -21.120 | 1.00 | 39.30 | B000 C |
| ATOM | 8538 | C | GLY | F | 262 | -30.982 | 58.938 | -22.466 | 1.00 | 41.80 | B000 C |
| ATOM | 8539 | O | GLY | F | 262 | -30.102 | 58.076 | -22.575 | 1.00 | 39.59 | B000 O |
| ATOM | 8540 | N | ARG | F | 263 | -31.736 | 59.319 | -23.494 | 1.00 | 35.33 | B000 N |
| ATOM | 8541 | CA | ARG | F | 263 | -31.468 | 58.795 | -24.822 | 1.00 | 38.94 | B000 C |
| ATOM | 8542 | C | ARG | F | 263 | -30.159 | 59.370 | -25.357 | 1.00 | 41.24 | B000 C |
| ATOM | 8543 | O | ARG | F | 263 | -29.688 | 60.429 | -24.926 | 1.00 | 40.40 | B000 O |
| ATOM | 8544 | CB | ARG | F | 263 | -32.646 | 59.085 | -25.760 | 1.00 | 39.18 | B000 C |
| ATOM | 8545 | CG | ARG | F | 263 | -33.888 | 58.222 | -25.393 | 1.00 | 46.56 | B000 C |
| ATOM | 8546 | CD | ARG | F | 263 | -35.120 | 58.506 | -26.239 | 1.00 | 38.08 | B000 C |
| ATOM | 8547 | NE | ARG | F | 263 | -35.507 | 59.908 | -26.134 | 1.00 | 51.22 | B000 N |
| ATOM | 8548 | CZ | ARG | F | 263 | -36.495 | 60.464 | -26.827 | 1.00 | 58.02 | B000 C |
| ATOM | 8549 | NH1 | ARG | F | 263 | -37.218 | 59.731 | -27.673 | 1.00 | 52.64 | B000 N1+ |
| ATOM | 8550 | NH2 | ARG | F | 263 | -36.754 | 61.756 | -26.676 | 1.00 | 51.34 | B000 N |
| ATOM | 8551 | N | TRP | F | 264 | -29.579 | 58.655 | -26.311 | 1.00 | 35.20 | B000 N |
| ATOM | 8552 | CA | TRP | F | 264 | -28.211 | 58.875 | -26.746 | 1.00 | 33.30 | B000 C |
| ATOM | 8553 | C | TRP | F | 264 | -28.146 | 59.805 | -27.951 | 1.00 | 36.94 | B000 C |
| ATOM | 8554 | O | TRP | F | 264 | -29.059 | 59.860 | -28.785 | 1.00 | 39.27 | B000 O |
| ATOM | 8555 | CB | TRP | F | 264 | -27.546 | 57.555 | -27.110 | 1.00 | 32.18 | B000 C |
| ATOM | 8556 | CG | TRP | F | 264 | -27.593 | 56.508 | -26.057 | 1.00 | 35.42 | B000 C |
| ATOM | 8557 | CD1 | TRP | F | 264 | -28.003 | 56.652 | -24.766 | 1.00 | 40.13 | B000 C |
| ATOM | 8558 | CD2 | TRP | F | 264 | -27.196 | 55.143 | -26.203 | 1.00 | 36.70 | B000 C |
| ATOM | 8559 | NE1 | TRP | F | 264 | -27.880 | 55.459 | -24.095 | 1.00 | 37.06 | B000 N |
| ATOM | 8560 | CE2 | TRP | F | 264 | -27.388 | 54.516 | -24.960 | 1.00 | 38.24 | B000 C |
| ATOM | 8561 | CE3 | TRP | F | 264 | -26.688 | 54.387 | -27.270 | 1.00 | 40.49 | B000 C |
| ATOM | 8562 | CZ2 | TRP | F | 264 | -27.101 | 53.169 | -24.756 | 1.00 | 35.99 | B000 C |
| ATOM | 8563 | CZ3 | TRP | F | 264 | -26.407 | 53.045 | -27.061 | 1.00 | 32.82 | B000 C |
| ATOM | 8564 | CH2 | TRP | F | 264 | -26.613 | 52.457 | -25.819 | 1.00 | 37.82 | B000 C |
| ATOM | 8565 | N | ASN | F | 265 | -27.044 | 60.540 | -28.034 | 1.00 | 31.28 | B000 N |
| ATOM | 8566 | CA | ASN | F | 265 | -26.763 | 61.379 | -29.187 | 1.00 | 33.60 | B000 C |
| ATOM | 8567 | C | ASN | F | 265 | -25.275 | 61.280 | -29.513 | 1.00 | 33.44 | B000 C |
| ATOM | 8568 | O | ASN | F | 265 | -24.446 | 60.973 | -28.645 | 1.00 | 29.37 | B000 O |
| ATOM | 8569 | CB | ASN | F | 265 | -27.200 | 62.832 | -28.912 | 1.00 | 31.57 | B000 C |
| ATOM | 8570 | CG | ASN | F | 265 | -26.915 | 63.768 | -30.076 | 1.00 | 39.82 | B000 C |
| ATOM | 8571 | OD1 | ASN | F | 265 | -27.466 | 63.615 | -31.166 | 1.00 | 41.22 | B000 O |
| ATOM | 8572 | ND2 | ASN | F | 265 | -26.003 | 64.724 | -29.856 | 1.00 | 36.27 | B000 N |
| ATOM | 8573 | N | ASP | F | 266 | -24.950 | 61.494 | -30.786 | 1.00 | 31.29 | B000 N |
| ATOM | 8574 | CA | ASP | F | 266 | -23.572 | 61.488 | -31.255 | 1.00 | 31.23 | B000 C |
| ATOM | 8575 | C | ASP | F | 266 | -23.107 | 62.919 | -31.488 | 1.00 | 32.82 | B000 C |
| ATOM | 8576 | O | ASP | F | 266 | -23.788 | 63.693 | -32.166 | 1.00 | 30.42 | B000 O |
| ATOM | 8577 | CB | ASP | F | 266 | -23.408 | 60.650 | -32.536 | 1.00 | 36.08 | B000 C |
| ATOM | 8578 | CG | ASP | F | 266 | -24.411 | 61.018 | -33.661 | 1.00 | 40.88 | B000 C |
| ATOM | 8579 | OD1 | ASP | F | 266 | -25.586 | 61.367 | -33.390 | 1.00 | 36.41 | B000 O |
| ATOM | 8580 | OD2 | ASP | F | 266 | -24.013 | 60.933 | -34.844 | 1.00 | 40.99 | B000 O1- |
| ATOM | 8581 | N | ASP | F | 267 | -21.943 | 63.261 | -30.935 | 1.00 | 35.94 | B000 N |
| ATOM | 8582 | CA | ASP | F | 267 | -21.403 | 64.616 | -31.027 | 1.00 | 36.54 | B000 C |
| ATOM | 8583 | C | ASP | F | 267 | -19.880 | 64.547 | -31.126 | 1.00 | 34.86 | B000 C |
| ATOM | 8584 | O | ASP | F | 267 | -19.271 | 63.507 | -30.850 | 1.00 | 34.33 | B000 O |

```
ATOM   8585  CB  ASP F 267     -21.835  65.470 -29.834  1.00 30.38      B000 C
ATOM   8586  CG  ASP F 267     -21.905  66.947 -30.180  1.00 43.85      B000 C
ATOM   8587  OD1 ASP F 267     -21.377  67.294 -31.265  1.00 39.52      B000 O
ATOM   8588  OD2 ASP F 267     -22.463  67.744 -29.369  1.00 37.84      B000 O1-
ATOM   8589  N   VAL F 268     -19.263  65.675 -31.519  1.00 30.07      B000 N
ATOM   8590  CA  VAL F 268     -17.811  65.692 -31.674  1.00 34.19      B000 C
ATOM   8591  C   VAL F 268     -17.155  65.408 -30.331  1.00 32.91      B000 C
ATOM   8592  O   VAL F 268     -17.605  65.876 -29.277  1.00 34.33      B000 O
ATOM   8593  CB  VAL F 268     -17.313  67.013 -32.292  1.00 36.17      B000 C
ATOM   8594  CG1 VAL F 268     -17.784  67.114 -33.734  1.00 30.93      B000 C
ATOM   8595  CG2 VAL F 268     -17.786  68.204 -31.515  1.00 29.63      B000 C
ATOM   8596  N   CYS F 269     -16.096  64.605 -30.366  1.00 30.58      B000 N
ATOM   8597  CA  CYS F 269     -15.512  64.059 -29.147  1.00 32.39      B000 C
ATOM   8598  C   CYS F 269     -14.819  65.101 -28.276  1.00 35.95      B000 C
ATOM   8599  O   CYS F 269     -14.508  64.795 -27.110  1.00 34.91      B000 O
ATOM   8600  CB  CYS F 269     -14.527  62.940 -29.502  1.00 40.17      B000 C
ATOM   8601  SG  CYS F 269     -15.360  61.521 -30.340  1.00 50.58      B000 S
ATOM   8602  N   GLN F 270     -14.605  66.326 -28.768  1.00 30.41      B000 N
ATOM   8603  CA  GLN F 270     -13.991  67.316 -27.886  1.00 37.36      B000 C
ATOM   8604  C   GLN F 270     -14.990  68.026 -26.982  1.00 33.95      B000 C
ATOM   8605  O   GLN F 270     -14.541  68.820 -26.158  1.00 37.08      B000 O
ATOM   8606  CB  GLN F 270     -13.179  68.371 -28.659  1.00 34.85      B000 C
ATOM   8607  CG  GLN F 270     -13.560  68.598 -30.097  1.00 52.16      B000 C
ATOM   8608  CD  GLN F 270     -13.134  67.433 -30.977  1.00 59.41      B000 C
ATOM   8609  OE1 GLN F 270     -13.911  66.970 -31.818  1.00 54.59      B000 O
ATOM   8610  NE2 GLN F 270     -11.912  66.916 -30.748  1.00 48.02      B000 N
ATOM   8611  N   ARG F 271     -16.302  67.744 -27.083  1.00 27.57      B000 N
ATOM   8612  CA  ARG F 271     -17.274  68.311 -26.158  1.00 29.87      B000 C
ATOM   8613  C   ARG F 271     -16.853  68.013 -24.720  1.00 34.79      B000 C
ATOM   8614  O   ARG F 271     -16.512  66.861 -24.400  1.00 30.23      B000 O
ATOM   8615  CB  ARG F 271     -18.688  67.750 -26.374  1.00 30.54      B000 C
ATOM   8616  CG  ARG F 271     -19.384  68.131 -27.667  1.00 31.79      B000 C
ATOM   8617  CD  ARG F 271     -19.505  69.613 -27.794  1.00 33.75      B000 C
ATOM   8618  NE  ARG F 271     -20.347  70.002 -28.916  1.00 33.09      B000 N
ATOM   8619  CZ  ARG F 271     -20.385  71.243 -29.388  1.00 38.41      B000 C
ATOM   8620  NH1 ARG F 271     -19.614  72.177 -28.817  1.00 34.05      B000 N1+
ATOM   8621  NH2 ARG F 271     -21.173  71.556 -30.417  1.00 29.00      B000 N
ATOM   8622  N   PRO F 272     -16.862  68.955 -23.879  1.00 32.65      B000 N
ATOM   8623  CA  PRO F 272     -16.460  68.718 -22.481  1.00 27.76      B000 C
ATOM   8624  C   PRO F 272     -17.615  68.190 -21.630  1.00 31.78      B000 C
ATOM   8625  O   PRO F 272     -18.061  68.832 -20.676  1.00 29.70      B000 O
ATOM   8626  CB  PRO F 272     -15.983  70.111 -22.042  1.00 28.08      B000 C
ATOM   8627  CG  PRO F 272     -16.860  71.083 -22.841  1.00 28.41      B000 C
ATOM   8628  CD  PRO F 272     -17.059  70.391 -24.190  1.00 31.14      B000 C
ATOM   8629  N   TYR F 273     -18.129  67.005 -21.987  1.00 32.02      B000 N
ATOM   8630  CA  TYR F 273     -19.251  66.438 -21.248  1.00 31.15      B000 C
ATOM   8631  C   TYR F 273     -18.759  65.726 -19.999  1.00 28.60      B000 C
ATOM   8632  O   TYR F 273     -17.577  65.427 -19.850  1.00 27.10      B000 O
ATOM   8633  CB  TYR F 273     -20.061  65.473 -22.113  1.00 28.19      B000 C
ATOM   8634  CG  TYR F 273     -20.762  66.174 -23.244  1.00 30.22      B000 C
ATOM   8635  CD1 TYR F 273     -21.111  67.519 -23.133  1.00 29.80      B000 C
ATOM   8636  CD2 TYR F 273     -21.057  65.512 -24.440  1.00 28.43      B000 C
ATOM   8637  CE1 TYR F 273     -21.765  68.193 -24.178  1.00 29.36      B000 C
ATOM   8638  CE2 TYR F 273     -21.695  66.178 -25.493  1.00 26.89      B000 C
ATOM   8639  CZ  TYR F 273     -22.039  67.519 -25.355  1.00 30.78      B000 C
ATOM   8640  OH  TYR F 273     -22.668  68.191 -26.375  1.00 33.01      B000 O
ATOM   8641  N   ARG F 274     -19.688  65.469 -19.083  1.00 31.67      B000 N
ATOM   8642  CA  ARG F 274     -19.383  64.573 -17.977  1.00 32.33      B000 C
ATOM   8643  C   ARG F 274     -19.177  63.156 -18.523  1.00 29.48      B000 C
ATOM   8644  O   ARG F 274     -19.403  62.875 -19.703  1.00 29.20      B000 O
ATOM   8645  CB  ARG F 274     -20.501  64.618 -16.931  1.00 33.56      B000 C
ATOM   8646  CG  ARG F 274     -20.639  65.990 -16.246  1.00 34.67      B000 C
ATOM   8647  CD  ARG F 274     -21.615  65.998 -15.050  1.00 38.53      B000 C
ATOM   8648  NE  ARG F 274     -21.480  67.235 -14.265  1.00 37.38      B000 N
ATOM   8649  CZ  ARG F 274     -22.106  67.482 -13.117  1.00 34.94      B000 C
ATOM   8650  NH1 ARG F 274     -22.944  66.591 -12.600  1.00 33.17      B000 N1+
ATOM   8651  NH2 ARG F 274     -21.886  68.623 -12.476  1.00 31.06      B000 N
ATOM   8652  N   TRP F 275     -18.741  62.250 -17.662  1.00 29.84      B000 N
ATOM   8653  CA  TRP F 275     -18.518  60.878 -18.100  1.00 34.86      B000 C
ATOM   8654  C   TRP F 275     -18.726  59.935 -16.926  1.00 36.82      B000 C
ATOM   8655  O   TRP F 275     -18.766  60.351 -15.763  1.00 32.31      B000 O
```

```
ATOM   8656  CB  TRP F 275     -17.112  60.684 -18.679  1.00 28.66          B000 C
ATOM   8657  CG  TRP F 275     -16.078  60.676 -17.628  1.00 32.25          B000 C
ATOM   8658  CD1 TRP F 275     -15.531  59.586 -17.019  1.00 34.72          B000 C
ATOM   8659  CD2 TRP F 275     -15.448  61.826 -17.041  1.00 33.73          B000 C
ATOM   8660  NE1 TRP F 275     -14.587  59.989 -16.085  1.00 33.65          B000 N
ATOM   8661  CE2 TRP F 275     -14.521  61.358 -16.087  1.00 32.03          B000 C
ATOM   8662  CE3 TRP F 275     -15.575  63.203 -17.238  1.00 32.63          B000 C
ATOM   8663  CZ2 TRP F 275     -13.732  62.217 -15.334  1.00 34.54          B000 C
ATOM   8664  CZ3 TRP F 275     -14.798  64.057 -16.476  1.00 32.70          B000 C
ATOM   8665  CH2 TRP F 275     -13.890  63.563 -15.542  1.00 33.77          B000 C
ATOM   8666  N   VAL F 276     -18.840  58.646 -17.247  1.00 33.42          B000 N
ATOM   8667  CA  VAL F 276     -19.034  57.591 -16.256  1.00 37.19          B000 C
ATOM   8668  C   VAL F 276     -17.985  56.504 -16.480  1.00 41.17          B000 C
ATOM   8669  O   VAL F 276     -17.762  56.073 -17.620  1.00 38.26          B000 O
ATOM   8670  CB  VAL F 276     -20.454  56.999 -16.338  1.00 37.30          B000 C
ATOM   8671  CG1 VAL F 276     -20.677  55.983 -15.212  1.00 34.94          B000 C
ATOM   8672  CG2 VAL F 276     -21.488  58.117 -16.312  1.00 29.37          B000 C
ATOM   8673  N   CYS F 277     -17.328  56.085 -15.402  1.00 36.51          B000 N
ATOM   8674  CA  CYS F 277     -16.406  54.958 -15.437  1.00 39.62          B000 C
ATOM   8675  C   CYS F 277     -17.094  53.700 -14.909  1.00 45.76          B000 C
ATOM   8676  O   CYS F 277     -17.945  53.759 -14.015  1.00 44.66          B000 O
ATOM   8677  CB  CYS F 277     -15.146  55.230 -14.605  1.00 40.57          B000 C
ATOM   8678  SG  CYS F 277     -14.058  56.553 -15.196  1.00 50.52          B000 S
ATOM   8679  N   GLU F 278     -16.695  52.557 -15.462  1.00 39.15          B000 N
ATOM   8680  CA  GLU F 278     -17.250  51.260 -15.112  1.00 44.84          B000 C
ATOM   8681  C   GLU F 278     -16.120  50.256 -14.936  1.00 47.20          B000 C
ATOM   8682  O   GLU F 278     -15.173  50.230 -15.729  1.00 47.48          B000 O
ATOM   8683  CB  GLU F 278     -18.203  50.751 -16.194  1.00 38.93          B000 C
ATOM   8684  CG  GLU F 278     -18.836  49.416 -15.857  1.00 46.64          B000 C
ATOM   8685  CD  GLU F 278     -19.732  48.897 -16.963  1.00 49.83          B000 C
ATOM   8686  OE1 GLU F 278     -19.861  49.572 -18.007  1.00 51.92          B000 O
ATOM   8687  OE2 GLU F 278     -20.316  47.813 -16.790  1.00 61.81          B000 O1-
ATOM   8688  N   THR F 279     -16.212  49.447 -13.886  1.00 44.44          B000 N
ATOM   8689  CA  THR F 279     -15.287  48.339 -13.689  1.00 49.01          B000 C
ATOM   8690  C   THR F 279     -16.035  47.205 -13.000  1.00 55.21          B000 C
ATOM   8691  O   THR F 279     -17.179  47.359 -12.564  1.00 50.33          B000 O
ATOM   8692  CB  THR F 279     -14.035  48.775 -12.899  1.00 50.55          B000 C
ATOM   8693  OG1 THR F 279     -13.019  47.768 -13.000  1.00 60.70          B000 O
ATOM   8694  CG2 THR F 279     -14.349  49.063 -11.433  1.00 40.44          B000 C
ATOM   8695  N   GLU F 280     -15.392  46.046 -12.929  1.00 62.33          B000 N
ATOM   8696  CA  GLU F 280     -16.042  44.856 -12.389  1.00 60.95          B000 C
ATOM   8697  C   GLU F 280     -15.846  44.774 -10.881  1.00 59.82          B000 C
ATOM   8698  O   GLU F 280     -14.755  45.070 -10.384  1.00 61.25          B000 O
ATOM   8699  CB  GLU F 280     -15.489  43.597 -13.056  1.00 64.94          B000 C
ATOM   8700  CG  GLU F 280     -13.982  43.637 -13.300  1.00 75.16          B000 C
ATOM   8701  CD  GLU F 280     -13.631  44.136 -14.695  1.00 87.07          B000 C
ATOM   8702  OE1 GLU F 280     -14.294  43.684 -15.660  1.00 92.63          B000 O
ATOM   8703  OE2 GLU F 280     -12.707  44.977 -14.825  1.00 87.02          B000 O1-
ATOM   8704  N   GLU F 281     -16.934  44.447 -10.167  1.00 59.93          B000 N
ATOM   8705  CA  GLU F 281     -16.960  43.756  -8.848  1.00 67.16          B000 C
ATOM   8706  C   GLU F 281     -18.241  44.090  -8.095  1.00 67.27          B000 C
ATOM   8707  O   GLU F 281     -18.257  44.100  -6.862  1.00 68.70          B000 O
ATOM   8708  CB  GLU F 281     -15.757  44.085  -7.951  1.00 63.68          B000 C
ATOM   8709  CG  GLU F 281     -15.131  42.885  -7.230  1.00 68.36          B000 C
ATOM   8710  CD  GLU F 281     -14.442  41.881  -8.172  1.00 83.09          B000 C
ATOM   8711  OE1 GLU F 281     -15.127  41.287  -9.037  1.00 77.83          B000 O
ATOM   8712  OE2 GLU F 281     -13.210  41.679  -8.043  1.00 76.05          B000 O
TER
HETATM 9170  C1  GAL G 401      19.131  84.986 -13.073  1.00 67.87               C
HETATM 9171  O1  GAL G 401      19.230  83.939 -14.075  1.00 65.01               O
HETATM 9172  C2  GAL G 401      19.826  84.527 -11.743  1.00 63.05               C
HETATM 9173  O2  GAL G 401      19.202  83.373 -11.240  1.00 42.62               O
HETATM 9174  C3  GAL G 401      19.822  85.673 -10.639  1.00 71.79               C
HETATM 9175  O3  GAL G 401      20.324  85.299  -9.317  1.00 63.98               O
HETATM 9176  C4  GAL G 401      20.547  86.954 -11.192  1.00 74.66               C
HETATM 9177  O4  GAL G 401      21.936  86.736 -11.495  1.00 79.63               O
HETATM 9178  C5  GAL G 401      19.774  87.370 -12.469  1.00 75.84               C
HETATM 9179  O5  GAL G 401      19.667  86.275 -13.538  1.00 68.13               O
HETATM 9180  C6  GAL G 401      20.270  88.659 -13.125  1.00 68.64               C
HETATM 9181  O6  GAL G 401      19.157  89.413 -13.631  1.00 52.02               O
TER
```

```
HETATM 9182  C1  GAL G 402     -25.502  71.256 -32.056  1.00 74.77           C
HETATM 9183  O1  GAL G 402     -24.812  72.366 -32.713  1.00 60.32           O
HETATM 9184  C2  GAL G 402     -26.779  71.727 -31.263  1.00 62.62           C
HETATM 9185  O2  GAL G 402     -26.494  72.728 -30.323  1.00 41.25           O
HETATM 9186  C3  GAL G 402     -27.470  70.522 -30.515  1.00 66.23           C
HETATM 9187  O3  GAL G 402     -28.624  70.910 -29.737  1.00 63.41           O
HETATM 9188  C4  GAL G 402     -27.905  69.432 -31.526  1.00 75.11           C
HETATM 9189  O4  GAL G 402     -29.064  69.854 -32.279  1.00 80.69           O
HETATM 9190  C5  GAL G 402     -26.702  69.051 -32.464  1.00 82.81           C
HETATM 9191  O5  GAL G 402     -25.897  70.216 -33.013  1.00 81.70           O
HETATM 9192  C6  GAL G 402     -27.102  68.166 -33.664  1.00 85.18           C
HETATM 9193  O6  GAL G 402     -26.406  68.545 -34.844  1.00 94.12           O
TER
HETATM 9194 CA    CA H   1      21.469  91.701 -14.212  1.00 79.84          Ca
TER
HETATM 9195 CA    CA H   2     -26.073  64.419 -34.040  1.00 79.26          Ca
TER
HETATM 9196 CA    CA H   3     -18.333  61.223 -36.994  1.00 75.64          Ca
HETATM 9197 CA    CA H   4      18.240  94.784 -21.928  1.00 73.55          Ca
TER
```

## Table 10.4

| | | | | | | | | | | | |
|---|---|---|---|---|---|---|---|---|---|---|---|
| ATOM | 1 | O | GLN | A | 1 | -24.853 | -26.439 | 84.334 | 1.00 | 39.67 | O |
| ATOM | 2 | N | GLN | A | 1 | -23.947 | -29.300 | 85.222 | 1.00 | 49.60 | N |
| ATOM | 3 | CA | GLN | A | 1 | -24.872 | -28.345 | 85.852 | 1.00 | 56.88 | C |
| ATOM | 4 | C | GLN | A | 1 | -25.501 | -27.209 | 85.050 | 1.00 | 51.47 | C |
| ATOM | 5 | CB | GLN | A | 1 | -24.208 | -27.745 | 87.074 | 1.00 | 45.63 | C |
| ATOM | 6 | CG | GLN | A | 1 | -23.967 | -28.823 | 88.054 | 1.00 | 44.21 | C |
| ATOM | 7 | CD | GLN | A | 1 | -25.274 | -29.457 | 88.407 | 1.00 | 54.28 | C |
| ATOM | 8 | OE1 | GLN | A | 1 | -26.030 | -28.899 | 89.200 | 1.00 | 62.35 | O |
| ATOM | 9 | NE2 | GLN | A | 1 | -25.592 | -30.592 | 87.778 | 1.00 | 57.03 | N |
| ATOM | 10 | N | VAL | A | 2 | -26.812 | -27.135 | 85.264 | 1.00 | 51.10 | N |
| ATOM | 11 | CA | VAL | A | 2 | -27.671 | -26.124 | 84.683 | 1.00 | 42.93 | C |
| ATOM | 12 | C | VAL | A | 2 | -27.754 | -24.968 | 85.669 | 1.00 | 44.75 | C |
| ATOM | 13 | O | VAL | A | 2 | -28.236 | -25.134 | 86.793 | 1.00 | 42.04 | O |
| ATOM | 14 | CB | VAL | A | 2 | -29.061 | -26.696 | 84.378 | 1.00 | 39.51 | C |
| ATOM | 15 | CG1 | VAL | A | 2 | -29.809 | -25.773 | 83.468 | 1.00 | 44.64 | C |
| ATOM | 16 | CG2 | VAL | A | 2 | -28.948 | -28.065 | 83.754 | 1.00 | 39.26 | C |
| ATOM | 17 | N | GLN | A | 3 | -27.240 | -23.812 | 85.269 | 1.00 | 45.15 | N |
| ATOM | 18 | CA | GLN | A | 3 | -27.403 | -22.586 | 86.031 | 1.00 | 44.60 | C |
| ATOM | 19 | C | GLN | A | 3 | -28.455 | -21.725 | 85.355 | 1.00 | 34.85 | C |
| ATOM | 20 | O | GLN | A | 3 | -28.496 | -21.629 | 84.128 | 1.00 | 39.72 | O |
| ATOM | 21 | CB | GLN | A | 3 | -26.091 | -21.792 | 86.128 | 1.00 | 50.01 | C |
| ATOM | 22 | CG | GLN | A | 3 | -24.978 | -22.456 | 86.931 | 1.00 | 56.60 | C |
| ATOM | 23 | CD | GLN | A | 3 | -23.919 | -23.128 | 86.052 | 1.00 | 59.49 | C |
| ATOM | 24 | OE1 | GLN | A | 3 | -24.036 | -23.141 | 84.811 | 1.00 | 56.42 | O |
| ATOM | 25 | NE2 | GLN | A | 3 | -22.874 | -23.691 | 86.692 | 1.00 | 48.31 | N |
| ATOM | 26 | N | LEU | A | 4 | -29.296 | -21.109 | 86.161 | 1.00 | 34.42 | N |
| ATOM | 27 | CA | LEU | A | 4 | -30.179 | -20.024 | 85.752 | 1.00 | 31.59 | C |
| ATOM | 28 | C | LEU | A | 4 | -29.640 | -18.812 | 86.491 | 1.00 | 37.33 | C |
| ATOM | 29 | O | LEU | A | 4 | -29.854 | -18.673 | 87.696 | 1.00 | 44.24 | O |
| ATOM | 30 | CB | LEU | A | 4 | -31.634 | -20.283 | 86.124 | 1.00 | 31.99 | C |
| ATOM | 31 | CG | LEU | A | 4 | -32.309 | -21.572 | 85.670 | 1.00 | 34.73 | C |
| ATOM | 32 | CD1 | LEU | A | 4 | -33.852 | -21.433 | 85.729 | 1.00 | 32.19 | C |
| ATOM | 33 | CD2 | LEU | A | 4 | -31.838 | -21.916 | 84.287 | 1.00 | 30.49 | C |
| ATOM | 34 | N | GLN | A | 5 | -28.883 | -17.976 | 85.797 | 1.00 | 40.51 | N |
| ATOM | 35 | CA | GLN | A | 5 | -28.269 | -16.810 | 86.412 | 1.00 | 38.81 | C |
| ATOM | 36 | C | GLN | A | 5 | -29.203 | -15.614 | 86.259 | 1.00 | 37.64 | C |
| ATOM | 37 | O | GLN | A | 5 | -29.714 | -15.358 | 85.170 | 1.00 | 41.01 | O |
| ATOM | 38 | CB | GLN | A | 5 | -26.911 | -16.544 | 85.768 | 1.00 | 38.94 | C |
| ATOM | 39 | CG | GLN | A | 5 | -26.103 | -17.812 | 85.595 | 1.00 | 46.98 | C |
| ATOM | 40 | CD | GLN | A | 5 | -24.690 | -17.565 | 85.045 | 1.00 | 61.68 | C |
| ATOM | 41 | OE1 | GLN | A | 5 | -24.523 | -17.011 | 83.956 | 1.00 | 63.24 | O |
| ATOM | 42 | NE2 | GLN | A | 5 | -23.671 | -17.982 | 85.800 | 1.00 | 53.29 | N |
| ATOM | 43 | N | GLN | A | 6 | -29.465 | -14.912 | 87.348 | 1.00 | 37.71 | N |
| ATOM | 44 | CA | GLN | A | 6 | -30.413 | -13.810 | 87.326 | 1.00 | 37.43 | C |
| ATOM | 45 | C | GLN | A | 6 | -29.670 | -12.491 | 87.484 | 1.00 | 41.79 | C |
| ATOM | 46 | O | GLN | A | 6 | -28.694 | -12.405 | 88.237 | 1.00 | 42.07 | O |
| ATOM | 47 | CB | GLN | A | 6 | -31.445 | -13.932 | 88.448 | 1.00 | 34.57 | C |
| ATOM | 48 | CG | GLN | A | 6 | -32.083 | -15.276 | 88.561 | 1.00 | 37.33 | C |
| ATOM | 49 | CD | GLN | A | 6 | -33.308 | -15.279 | 89.458 | 1.00 | 36.27 | C |
| ATOM | 50 | OE1 | GLN | A | 6 | -33.695 | -16.319 | 89.966 | 1.00 | 30.06 | O |
| ATOM | 51 | NE2 | GLN | A | 6 | -33.920 | -14.117 | 89.653 | 1.00 | 32.89 | N |
| ATOM | 52 | N | TRP | A | 7 | -30.144 | -11.463 | 86.785 | 1.00 | 36.25 | N |
| ATOM | 53 | CA | TRP | A | 7 | -29.729 | -10.098 | 87.059 | 1.00 | 33.75 | C |
| ATOM | 54 | C | TRP | A | 7 | -30.876 | -9.160 | 86.714 | 1.00 | 37.39 | C |
| ATOM | 55 | O | TRP | A | 7 | -31.883 | -9.557 | 86.120 | 1.00 | 39.47 | O |
| ATOM | 56 | CB | TRP | A | 7 | -28.452 | -9.723 | 86.303 | 1.00 | 33.51 | C |
| ATOM | 57 | CG | TRP | A | 7 | -28.542 | -9.763 | 84.822 | 1.00 | 34.91 | C |
| ATOM | 58 | CD1 | TRP | A | 7 | -28.929 | -8.739 | 83.990 | 1.00 | 37.52 | C |
| ATOM | 59 | CD2 | TRP | A | 7 | -28.206 | -10.865 | 83.970 | 1.00 | 37.56 | C |
| ATOM | 60 | NE1 | TRP | A | 7 | -28.872 | -9.149 | 82.675 | 1.00 | 37.51 | N |
| ATOM | 61 | CE2 | TRP | A | 7 | -28.425 | -10.446 | 82.635 | 1.00 | 36.81 | C |
| ATOM | 62 | CE3 | TRP | A | 7 | -27.737 | -12.160 | 84.204 | 1.00 | 43.59 | C |
| ATOM | 63 | CZ2 | TRP | A | 7 | -28.199 | -11.280 | 81.542 | 1.00 | 35.12 | C |
| ATOM | 64 | CZ3 | TRP | A | 7 | -27.517 | -12.993 | 83.112 | 1.00 | 48.14 | C |
| ATOM | 65 | CH2 | TRP | A | 7 | -27.754 | -12.547 | 81.797 | 1.00 | 43.93 | C |
| ATOM | 66 | N | GLY | A | 8 | -30.711 | -7.909 | 87.092 | 1.00 | 31.94 | N |
| ATOM | 67 | CA | GLY | A | 8 | -31.730 | -6.894 | 86.915 | 1.00 | 32.15 | C |
| ATOM | 68 | C | GLY | A | 8 | -31.597 | -6.027 | 88.146 | 1.00 | 36.03 | C |
| ATOM | 69 | O | GLY | A | 8 | -31.196 | -6.479 | 89.217 | 1.00 | 34.32 | O |

| | | | | | | | | | | | |
|---|---|---|---|---|---|---|---|---|---|---|---|
| ATOM | 70 | N | ALA | A | 9 | -31.911 | -4.745 | 87.991 | 1.00 | 39.30 | N |
| ATOM | 71 | CA | ALA | A | 9 | -31.830 | -3.844 | 89.129 | 1.00 | 40.16 | C |
| ATOM | 72 | C | ALA | A | 9 | -32.853 | -4.275 | 90.168 | 1.00 | 41.09 | C |
| ATOM | 73 | O | ALA | A | 9 | -34.026 | -4.460 | 89.845 | 1.00 | 43.60 | O |
| ATOM | 74 | CB | ALA | A | 9 | -32.073 | -2.399 | 88.690 | 1.00 | 33.25 | C |
| ATOM | 75 | N | GLY | A | 10 | -32.405 | -4.458 | 91.411 | 1.00 | 37.64 | N |
| ATOM | 76 | CA | GLY | A | 10 | -33.275 | -4.938 | 92.461 | 1.00 | 28.47 | C |
| ATOM | 77 | C | GLY | A | 10 | -33.861 | -3.889 | 93.382 | 1.00 | 36.57 | C |
| ATOM | 78 | O | GLY | A | 10 | -34.710 | -4.214 | 94.213 | 1.00 | 43.35 | O |
| ATOM | 79 | N | LEU | A | 11 | -33.418 | -2.638 | 93.273 | 1.00 | 36.99 | N |
| ATOM | 80 | CA | LEU | A | 11 | -33.946 | -1.533 | 94.067 | 1.00 | 34.54 | C |
| ATOM | 81 | C | LEU | A | 11 | -34.748 | -0.595 | 93.168 | 1.00 | 35.85 | C |
| ATOM | 82 | O | LEU | A | 11 | -34.244 | -0.129 | 92.144 | 1.00 | 38.05 | O |
| ATOM | 83 | CB | LEU | A | 11 | -32.818 | -0.764 | 94.760 | 1.00 | 36.89 | C |
| ATOM | 84 | CG | LEU | A | 11 | -33.040 | -0.447 | 96.238 | 1.00 | 40.06 | C |
| ATOM | 85 | CD1 | LEU | A | 11 | -31.989 | 0.520 | 96.748 | 1.00 | 38.21 | C |
| ATOM | 86 | CD2 | LEU | A | 11 | -34.443 | 0.097 | 96.487 | 1.00 | 39.59 | C |
| ATOM | 87 | N | LEU | A | 12 | -35.997 | -0.333 | 93.537 | 1.00 | 36.22 | N |
| ATOM | 88 | CA | LEU | A | 12 | -36.874 | 0.495 | 92.724 | 1.00 | 38.68 | C |
| ATOM | 89 | C | LEU | A | 12 | -37.710 | 1.388 | 93.624 | 1.00 | 37.27 | C |
| ATOM | 90 | O | LEU | A | 12 | -37.955 | 1.071 | 94.791 | 1.00 | 40.06 | O |
| ATOM | 91 | CB | LEU | A | 12 | -37.815 | -0.342 | 91.843 | 1.00 | 36.78 | C |
| ATOM | 92 | CG | LEU | A | 12 | -37.179 | -1.328 | 90.872 | 1.00 | 36.77 | C |
| ATOM | 93 | CD1 | LEU | A | 12 | -38.285 | -2.117 | 90.244 | 1.00 | 42.02 | C |
| ATOM | 94 | CD2 | LEU | A | 12 | -36.382 | -0.610 | 89.812 | 1.00 | 40.37 | C |
| ATOM | 95 | N | LYS | A | 13 | -38.164 | 2.508 | 93.053 | 1.00 | 36.92 | N |
| ATOM | 96 | CA | LYS | A | 13 | -39.141 | 3.391 | 93.665 | 1.00 | 36.42 | C |
| ATOM | 97 | C | LYS | A | 13 | -40.505 | 3.163 | 93.036 | 1.00 | 35.60 | C |
| ATOM | 98 | O | LYS | A | 13 | -40.605 | 2.637 | 91.921 | 1.00 | 39.97 | O |
| ATOM | 99 | CB | LYS | A | 13 | -38.711 | 4.848 | 93.499 | 1.00 | 41.56 | C |
| ATOM | 100 | CG | LYS | A | 13 | -37.227 | 5.088 | 93.781 | 1.00 | 52.81 | C |
| ATOM | 101 | CD | LYS | A | 13 | -36.541 | 5.678 | 92.538 | 1.00 | 68.42 | C |
| ATOM | 102 | CE | LYS | A | 13 | -35.056 | 5.297 | 92.399 | 1.00 | 70.40 | C |
| ATOM | 103 | NZ | LYS | A | 13 | -34.516 | 5.679 | 91.038 | 1.00 | 67.86 | N1+ |
| ATOM | 104 | N | PRO | A | 14 | -41.587 | 3.512 | 93.736 | 1.00 | 32.85 | N |
| ATOM | 105 | CA | PRO | A | 14 | -42.932 | 3.257 | 93.200 | 1.00 | 31.49 | C |
| ATOM | 106 | C | PRO | A | 14 | -43.114 | 3.852 | 91.806 | 1.00 | 38.32 | C |
| ATOM | 107 | O | PRO | A | 14 | -42.521 | 4.880 | 91.465 | 1.00 | 35.32 | O |
| ATOM | 108 | CB | PRO | A | 14 | -43.852 | 3.929 | 94.218 | 1.00 | 25.23 | C |
| ATOM | 109 | CG | PRO | A | 14 | -43.104 | 3.927 | 95.441 | 1.00 | 29.72 | C |
| ATOM | 110 | CD | PRO | A | 14 | -41.646 | 4.040 | 95.105 | 1.00 | 33.69 | C |
| ATOM | 111 | N | SER | A | 15 | -43.920 | 3.155 | 90.996 | 1.00 | 36.29 | N |
| ATOM | 112 | CA | SER | A | 15 | -44.274 | 3.500 | 89.622 | 1.00 | 33.19 | C |
| ATOM | 113 | C | SER | A | 15 | -43.184 | 3.147 | 88.614 | 1.00 | 34.56 | C |
| ATOM | 114 | O | SER | A | 15 | -43.472 | 3.106 | 87.414 | 1.00 | 34.31 | O |
| ATOM | 115 | CB | SER | A | 15 | -44.597 | 4.994 | 89.468 | 1.00 | 38.65 | C |
| ATOM | 116 | OG | SER | A | 15 | -43.421 | 5.751 | 89.168 | 1.00 | 34.68 | O |
| ATOM | 117 | N | GLU | A | 16 | -41.943 | 2.899 | 89.051 | 1.00 | 30.66 | N |
| ATOM | 118 | CA | GLU | A | 16 | -40.952 | 2.443 | 88.083 | 1.00 | 28.63 | C |
| ATOM | 119 | C | GLU | A | 16 | -41.330 | 1.055 | 87.551 | 1.00 | 33.32 | C |
| ATOM | 120 | O | GLU | A | 16 | -42.281 | 0.410 | 88.010 | 1.00 | 35.10 | O |
| ATOM | 121 | CB | GLU | A | 16 | -39.539 | 2.437 | 88.684 | 1.00 | 30.89 | C |
| ATOM | 122 | CG | GLU | A | 16 | -39.075 | 3.729 | 89.360 | 1.00 | 31.88 | C |
| ATOM | 123 | CD | GLU | A | 16 | -37.549 | 3.780 | 89.616 | 1.00 | 49.97 | C |
| ATOM | 124 | OE1 | GLU | A | 16 | -36.907 | 2.727 | 89.843 | 1.00 | 54.84 | O |
| ATOM | 125 | OE2 | GLU | A | 16 | -36.967 | 4.885 | 89.556 | 1.00 | 57.53 | O |
| ATOM | 126 | N | THR | A | 17 | -40.583 | 0.583 | 86.565 | 1.00 | 32.70 | N |
| ATOM | 127 | CA | THR | A | 17 | -40.829 | -0.744 | 86.034 | 1.00 | 35.01 | C |
| ATOM | 128 | C | THR | A | 17 | -39.637 | -1.655 | 86.318 | 1.00 | 38.16 | C |
| ATOM | 129 | O | THR | A | 17 | -38.473 | -1.238 | 86.259 | 1.00 | 36.52 | O |
| ATOM | 130 | CB | THR | A | 17 | -41.198 | -0.690 | 84.543 | 1.00 | 36.57 | C |
| ATOM | 131 | OG1 | THR | A | 17 | -40.325 | -1.515 | 83.766 | 1.00 | 34.00 | O |
| ATOM | 132 | CG2 | THR | A | 17 | -41.198 | 0.711 | 84.055 | 1.00 | 37.19 | C |
| ATOM | 133 | N | LEU | A | 18 | -39.955 | -2.874 | 86.733 | 1.00 | 35.90 | N |
| ATOM | 134 | CA | LEU | A | 18 | -38.969 | -3.873 | 87.100 | 1.00 | 33.49 | C |
| ATOM | 135 | C | LEU | A | 18 | -38.603 | -4.669 | 85.861 | 1.00 | 32.72 | C |
| ATOM | 136 | O | LEU | A | 18 | -39.482 | -5.056 | 85.082 | 1.00 | 30.94 | O |
| ATOM | 137 | CB | LEU | A | 18 | -39.537 | -4.770 | 88.209 | 1.00 | 34.34 | C |
| ATOM | 138 | CG | LEU | A | 18 | -38.899 | -6.037 | 88.790 | 1.00 | 34.31 | C |
| ATOM | 139 | CD1 | LEU | A | 18 | -39.067 | -7.231 | 87.855 | 1.00 | 31.61 | C |
| ATOM | 140 | CD2 | LEU | A | 18 | -37.433 | -5.823 | 89.128 | 1.00 | 34.33 | C |

| | | | | | | | | | | | |
|---|---|---|---|---|---|---|---|---|---|---|---|
| ATOM | 141 | N | SER | A | 19 | -37.309 | -4.888 | 85.660 | 1.00 | 31.86 | N |
| ATOM | 142 | CA | SER | A | 19 | -36.877 | -5.700 | 84.536 | 1.00 | 35.19 | C |
| ATOM | 143 | C | SER | A | 19 | -35.754 | -6.613 | 85.001 | 1.00 | 31.16 | C |
| ATOM | 144 | O | SER | A | 19 | -34.777 | -6.145 | 85.596 | 1.00 | 30.06 | O |
| ATOM | 145 | CB | SER | A | 19 | -36.449 | -4.841 | 83.346 | 1.00 | 33.07 | C |
| ATOM | 146 | OG | SER | A | 19 | -35.218 | -4.239 | 83.612 | 1.00 | 47.41 | O |
| ATOM | 147 | N | LEU | A | 20 | -35.934 | -7.916 | 84.768 | 1.00 | 25.55 | N |
| ATOM | 148 | CA | LEU | A | 20 | -35.022 | -8.962 | 85.214 | 1.00 | 28.53 | C |
| ATOM | 149 | C | LEU | A | 20 | -34.777 | -9.941 | 84.074 | 1.00 | 27.96 | C |
| ATOM | 150 | O | LEU | A | 20 | -35.643 | -10.142 | 83.220 | 1.00 | 27.00 | O |
| ATOM | 151 | CB | LEU | A | 20 | -35.596 | -9.712 | 86.425 | 1.00 | 26.52 | C |
| ATOM | 152 | CG | LEU | A | 20 | -35.939 | -8.899 | 87.678 | 1.00 | 29.44 | C |
| ATOM | 153 | CD1 | LEU | A | 20 | -36.684 | -9.780 | 88.650 | 1.00 | 34.23 | C |
| ATOM | 154 | CD2 | LEU | A | 20 | -34.683 | -8.369 | 88.365 | 1.00 | 31.36 | C |
| ATOM | 155 | N | THR | A | 21 | -33.589 | -10.552 | 84.061 | 1.00 | 28.15 | N |
| ATOM | 156 | CA | THR | A | 21 | -33.237 | -11.526 | 83.034 | 1.00 | 30.18 | C |
| ATOM | 157 | C | THR | A | 21 | -32.621 | -12.772 | 83.663 | 1.00 | 33.03 | C |
| ATOM | 158 | O | THR | A | 21 | -31.918 | -12.678 | 84.673 | 1.00 | 38.27 | O |
| ATOM | 159 | CB | THR | A | 21 | -32.240 | -10.913 | 82.023 | 1.00 | 32.85 | C |
| ATOM | 160 | OG1 | THR | A | 21 | -32.757 | -9.670 | 81.536 | 1.00 | 32.16 | O |
| ATOM | 161 | CG2 | THR | A | 21 | -31.977 | -11.866 | 80.841 | 1.00 | 28.45 | C |
| ATOM | 162 | N | CYS | A | 22 | -32.883 | -13.939 | 83.053 | 1.00 | 27.52 | N |
| ATOM | 163 | CA | CYS | A | 22 | -32.197 | -15.190 | 83.374 | 1.00 | 32.00 | C |
| ATOM | 164 | C | CYS | A | 22 | -31.452 | -15.675 | 82.149 | 1.00 | 29.74 | C |
| ATOM | 165 | O | CYS | A | 22 | -31.974 | -15.629 | 81.036 | 1.00 | 26.79 | O |
| ATOM | 166 | CB | CYS | A | 22 | -33.116 | -16.343 | 83.872 | 1.00 | 27.00 | C |
| ATOM | 167 | SG | CYS | A | 22 | -33.354 | -16.118 | 85.624 | 1.00 | 68.31 | S |
| ATOM | 168 | N | ALA | A | 23 | -30.228 | -16.135 | 82.379 | 1.00 | 32.24 | N |
| ATOM | 169 | CA | ALA | A | 23 | -29.397 | -16.762 | 81.370 | 1.00 | 32.34 | C |
| ATOM | 170 | C | ALA | A | 23 | -29.311 | -18.234 | 81.721 | 1.00 | 30.10 | C |
| ATOM | 171 | O | ALA | A | 23 | -29.020 | -18.577 | 82.867 | 1.00 | 34.73 | O |
| ATOM | 172 | CB | ALA | A | 23 | -28.004 | -16.125 | 81.339 | 1.00 | 32.68 | C |
| ATOM | 173 | N | VAL | A | 24 | -29.614 | -19.093 | 80.763 | 1.00 | 27.15 | N |
| ATOM | 174 | CA | VAL | A | 24 | -29.616 | -20.533 | 80.977 | 1.00 | 32.40 | C |
| ATOM | 175 | C | VAL | A | 24 | -28.371 | -21.124 | 80.342 | 1.00 | 32.49 | C |
| ATOM | 176 | O | VAL | A | 24 | -28.115 | -20.916 | 79.155 | 1.00 | 31.92 | O |
| ATOM | 177 | CB | VAL | A | 24 | -30.872 | -21.204 | 80.377 | 1.00 | 31.63 | C |
| ATOM | 178 | CG1 | VAL | A | 24 | -30.833 | -22.694 | 80.642 | 1.00 | 23.27 | C |
| ATOM | 179 | CG2 | VAL | A | 24 | -32.187 | -20.590 | 80.898 | 1.00 | 27.72 | C |
| ATOM | 180 | N | SER | A | 25 | -27.609 | -21.875 | 81.117 | 1.00 | 37.38 | N |
| ATOM | 181 | CA | SER | A | 25 | -26.471 | -22.620 | 80.602 | 1.00 | 34.69 | C |
| ATOM | 182 | C | SER | A | 25 | -26.544 | -24.037 | 81.136 | 1.00 | 37.68 | C |
| ATOM | 183 | O | SER | A | 25 | -27.107 | -24.279 | 82.206 | 1.00 | 45.11 | O |
| ATOM | 184 | CB | SER | A | 25 | -25.146 | -21.979 | 81.013 | 1.00 | 35.05 | C |
| ATOM | 185 | OG | SER | A | 25 | -25.168 | -21.665 | 82.396 | 1.00 | 40.52 | O |
| ATOM | 186 | N | GLY | A | 26 | -25.978 | -24.977 | 80.405 | 1.00 | 31.17 | N |
| ATOM | 187 | CA | GLY | A | 26 | -25.970 | -26.319 | 80.939 | 1.00 | 34.95 | C |
| ATOM | 188 | C | GLY | A | 26 | -26.773 | -27.339 | 80.181 | 1.00 | 44.75 | C |
| ATOM | 189 | O | GLY | A | 26 | -26.261 | -28.422 | 79.895 | 1.00 | 53.93 | O |
| ATOM | 190 | N | GLY | A | 27 | -28.035 | -27.049 | 79.897 | 1.00 | 43.99 | N |
| ATOM | 191 | CA | GLY | A | 27 | -28.827 | -28.022 | 79.179 | 1.00 | 39.13 | C |
| ATOM | 192 | C | GLY | A | 27 | -29.426 | -27.412 | 77.935 | 1.00 | 44.00 | C |
| ATOM | 193 | O | GLY | A | 27 | -28.940 | -26.394 | 77.426 | 1.00 | 44.61 | O |
| ATOM | 194 | N | SER | A | 28 | -30.525 | -27.991 | 77.475 | 1.00 | 48.00 | N |
| ATOM | 195 | CA | SER | A | 28 | -31.204 | -27.507 | 76.286 | 1.00 | 42.16 | C |
| ATOM | 196 | C | SER | A | 28 | -32.051 | -26.285 | 76.611 | 1.00 | 41.49 | C |
| ATOM | 197 | O | SER | A | 28 | -32.302 | -25.961 | 77.778 | 1.00 | 37.48 | O |
| ATOM | 198 | CB | SER | A | 28 | -32.071 | -28.605 | 75.684 | 1.00 | 39.00 | C |
| ATOM | 199 | OG | SER | A | 28 | -31.253 | -29.679 | 75.263 | 1.00 | 45.34 | O |
| ATOM | 200 | N | PHE | A | 29 | -32.448 | -25.569 | 75.552 | 1.00 | 39.22 | N |
| ATOM | 201 | CA | PHE | A | 29 | -33.327 | -24.425 | 75.695 | 1.00 | 34.42 | C |
| ATOM | 202 | C | PHE | A | 29 | -34.696 | -24.653 | 75.097 | 1.00 | 38.24 | C |
| ATOM | 203 | O | PHE | A | 29 | -35.628 | -23.923 | 75.439 | 1.00 | 40.29 | O |
| ATOM | 204 | CB | PHE | A | 29 | -32.710 | -23.181 | 75.041 | 1.00 | 30.96 | C |
| ATOM | 205 | CG | PHE | A | 29 | -33.191 | -21.874 | 75.633 | 1.00 | 31.50 | C |
| ATOM | 206 | CD1 | PHE | A | 29 | -33.148 | -21.663 | 77.004 | 1.00 | 33.59 | C |
| ATOM | 207 | CD2 | PHE | A | 29 | -33.660 | -20.850 | 74.823 | 1.00 | 28.85 | C |
| ATOM | 208 | CE1 | PHE | A | 29 | -33.578 | -20.450 | 77.564 | 1.00 | 31.05 | C |
| ATOM | 209 | CE2 | PHE | A | 29 | -34.073 | -19.653 | 75.359 | 1.00 | 29.76 | C |
| ATOM | 210 | CZ | PHE | A | 29 | -34.042 | -19.449 | 76.740 | 1.00 | 30.19 | C |
| ATOM | 211 | N | ARG | A | 30 | -34.855 | -25.653 | 74.242 | 1.00 | 41.39 | N |

| | | | | | | | | | | | |
|---|---|---|---|---|---|---|---|---|---|---|---|
| ATOM | 212 | CA | ARG | A | 30 | -36.009 | -25.653 | 73.362 | 1.00 | 44.20 | C |
| ATOM | 213 | C | ARG | A | 30 | -37.145 | -26.551 | 73.833 | 1.00 | 39.55 | C |
| ATOM | 214 | O | ARG | A | 30 | -38.265 | -26.412 | 73.331 | 1.00 | 42.77 | O |
| ATOM | 215 | CB | ARG | A | 30 | -35.568 | -26.002 | 71.939 | 1.00 | 31.85 | C |
| ATOM | 216 | CG | ARG | A | 30 | -35.075 | -27.382 | 71.728 | 1.00 | 39.91 | C |
| ATOM | 217 | CD | ARG | A | 30 | -34.814 | -27.548 | 70.245 | 1.00 | 43.27 | C |
| ATOM | 218 | NE | ARG | A | 30 | -33.604 | -28.305 | 69.892 | 1.00 | 49.24 | N |
| ATOM | 219 | CZ | ARG | A | 30 | -32.393 | -28.213 | 70.464 | 1.00 | 57.67 | C |
| ATOM | 220 | NH1 | ARG | A | 30 | -31.409 | -28.969 | 69.985 | 1.00 | 63.98 | N |
| ATOM | 221 | NH2 | ARG | A | 30 | -32.135 | -27.413 | 71.512 | 1.00 | 54.11 | N |
| ATOM | 222 | N | TYR | A | 31 | -36.902 | -27.434 | 74.789 | 1.00 | 36.60 | N |
| ATOM | 223 | CA | TYR | A | 31 | -37.940 | -28.340 | 75.264 | 1.00 | 40.80 | C |
| ATOM | 224 | C | TYR | A | 31 | -38.685 | -27.821 | 76.489 | 1.00 | 37.66 | C |
| ATOM | 225 | O | TYR | A | 31 | -39.628 | -28.470 | 76.946 | 1.00 | 36.93 | O |
| ATOM | 226 | CB | TYR | A | 31 | -37.334 | -29.699 | 75.619 | 1.00 | 37.87 | C |
| ATOM | 227 | CG | TYR | A | 31 | -36.422 | -30.240 | 74.573 | 1.00 | 39.45 | C |
| ATOM | 228 | CD1 | TYR | A | 31 | -36.888 | -30.500 | 73.300 | 1.00 | 42.59 | C |
| ATOM | 229 | CD2 | TYR | A | 31 | -35.088 | -30.528 | 74.861 | 1.00 | 44.81 | C |
| ATOM | 230 | CE1 | TYR | A | 31 | -36.047 | -31.005 | 72.325 | 1.00 | 45.84 | C |
| ATOM | 231 | CE2 | TYR | A | 31 | -34.240 | -31.049 | 73.890 | 1.00 | 43.49 | C |
| ATOM | 232 | CZ | TYR | A | 31 | -34.729 | -31.281 | 72.625 | 1.00 | 43.29 | C |
| ATOM | 233 | OH | TYR | A | 31 | -33.922 | -31.803 | 71.648 | 1.00 | 43.72 | O |
| ATOM | 234 | N | TYR | A | 32 | -38.302 | -26.677 | 77.027 | 1.00 | 39.36 | N |
| ATOM | 235 | CA | TYR | A | 32 | -38.735 | -26.280 | 78.350 | 1.00 | 34.43 | C |
| ATOM | 236 | C | TYR | A | 32 | -39.587 | -25.028 | 78.263 | 1.00 | 38.74 | C |
| ATOM | 237 | O | TYR | A | 32 | -39.501 | -24.256 | 77.305 | 1.00 | 39.83 | O |
| ATOM | 238 | CB | TYR | A | 32 | -37.533 | -26.028 | 79.265 | 1.00 | 33.39 | C |
| ATOM | 239 | CG | TYR | A | 32 | -36.634 | -27.226 | 79.343 | 1.00 | 38.75 | C |
| ATOM | 240 | CD2 | TYR | A | 32 | -35.377 | -27.236 | 78.750 | 1.00 | 32.24 | C |
| ATOM | 241 | CD1 | TYR | A | 32 | -37.061 | -28.368 | 80.004 | 1.00 | 38.64 | C |
| ATOM | 242 | CE2 | TYR | A | 32 | -34.570 | -28.364 | 78.823 | 1.00 | 39.82 | C |
| ATOM | 243 | CE1 | TYR | A | 32 | -36.270 | -29.495 | 80.088 | 1.00 | 40.18 | C |
| ATOM | 244 | CZ | TYR | A | 32 | -35.029 | -29.505 | 79.499 | 1.00 | 46.72 | C |
| ATOM | 245 | OH | TYR | A | 32 | -34.278 | -30.668 | 79.604 | 1.00 | 41.88 | O |
| ATOM | 246 | N | TYR | A | 33 | -40.420 | -24.842 | 79.282 | 1.00 | 31.10 | N |
| ATOM | 247 | CA | TYR | A | 33 | -41.042 | -23.558 | 79.529 | 1.00 | 30.58 | C |
| ATOM | 248 | C | TYR | A | 33 | -40.221 | -22.800 | 80.563 | 1.00 | 30.77 | C |
| ATOM | 249 | O | TYR | A | 33 | -39.686 | -23.392 | 81.507 | 1.00 | 29.88 | O |
| ATOM | 250 | CB | TYR | A | 33 | -42.491 | -23.726 | 79.987 | 1.00 | 30.38 | C |
| ATOM | 251 | CG | TYR | A | 33 | -43.440 | -23.801 | 78.834 | 1.00 | 33.35 | C |
| ATOM | 252 | CD2 | TYR | A | 33 | -44.211 | -22.698 | 78.485 | 1.00 | 33.18 | C |
| ATOM | 253 | CD1 | TYR | A | 33 | -43.550 | -24.966 | 78.059 | 1.00 | 33.82 | C |
| ATOM | 254 | CE2 | TYR | A | 33 | -45.080 | -22.746 | 77.407 | 1.00 | 36.40 | C |
| ATOM | 255 | CE1 | TYR | A | 33 | -44.430 | -25.030 | 76.976 | 1.00 | 33.85 | C |
| ATOM | 256 | CZ | TYR | A | 33 | -45.186 | -23.909 | 76.655 | 1.00 | 39.33 | C |
| ATOM | 257 | OH | TYR | A | 33 | -46.051 | -23.924 | 75.590 | 1.00 | 39.05 | O |
| ATOM | 258 | N | TRP | A | 34 | -40.133 | -21.490 | 80.381 | 1.00 | 25.37 | N |
| ATOM | 259 | CA | TRP | A | 34 | -39.285 | -20.639 | 81.189 | 1.00 | 25.30 | C |
| ATOM | 260 | C | TRP | A | 34 | -40.172 | -19.666 | 81.940 | 1.00 | 30.32 | C |
| ATOM | 261 | O | TRP | A | 34 | -41.013 | -18.993 | 81.328 | 1.00 | 28.04 | O |
| ATOM | 262 | CB | TRP | A | 34 | -38.242 | -19.932 | 80.310 | 1.00 | 27.04 | C |
| ATOM | 263 | CG | TRP | A | 34 | -37.375 | -20.952 | 79.641 | 1.00 | 28.16 | C |
| ATOM | 264 | CD1 | TRP | A | 34 | -37.436 | -21.371 | 78.333 | 1.00 | 31.12 | C |
| ATOM | 265 | CD2 | TRP | A | 34 | -36.410 | -21.787 | 80.279 | 1.00 | 28.13 | C |
| ATOM | 266 | NE1 | TRP | A | 34 | -36.525 | -22.375 | 78.110 | 1.00 | 27.45 | N |
| ATOM | 267 | CE2 | TRP | A | 34 | -35.881 | -22.653 | 79.287 | 1.00 | 31.81 | C |
| ATOM | 268 | CE3 | TRP | A | 34 | -35.920 | -21.875 | 81.587 | 1.00 | 28.15 | C |
| ATOM | 269 | CZ2 | TRP | A | 34 | -34.880 | -23.591 | 79.567 | 1.00 | 31.29 | C |
| ATOM | 270 | CZ3 | TRP | A | 34 | -34.911 | -22.807 | 81.864 | 1.00 | 29.29 | C |
| ATOM | 271 | CH2 | TRP | A | 34 | -34.411 | -23.654 | 80.858 | 1.00 | 25.81 | C |
| ATOM | 272 | N | SER | A | 35 | -39.993 | -19.608 | 83.268 | 1.00 | 28.16 | N |
| ATOM | 273 | CA | SER | A | 35 | -41.009 | -19.048 | 84.150 | 1.00 | 29.83 | C |
| ATOM | 274 | C | SER | A | 35 | -40.463 | -17.999 | 85.106 | 1.00 | 27.98 | C |
| ATOM | 275 | O | SER | A | 35 | -39.266 | -17.907 | 85.361 | 1.00 | 25.65 | O |
| ATOM | 276 | CB | SER | A | 35 | -41.677 | -20.145 | 84.957 | 1.00 | 25.41 | C |
| ATOM | 277 | OG | SER | A | 35 | -42.298 | -21.049 | 84.068 | 1.00 | 33.06 | O |
| ATOM | 278 | N | TRP | A | 36 | -41.388 | -17.224 | 85.655 | 1.00 | 26.15 | N |
| ATOM | 279 | CA | TRP | A | 36 | -41.091 | -16.278 | 86.712 | 1.00 | 23.95 | C |
| ATOM | 280 | C | TRP | A | 36 | -42.037 | -16.535 | 87.877 | 1.00 | 26.92 | C |
| ATOM | 281 | O | TRP | A | 36 | -43.249 | -16.680 | 87.693 | 1.00 | 24.36 | O |
| ATOM | 282 | CB | TRP | A | 36 | -41.200 | -14.847 | 86.204 | 1.00 | 21.96 | C |

| | | | | | | | | | | | |
|---|---|---|---|---|---|---|---|---|---|---|---|
| ATOM | 283 | CG | TRP | A | 36 | -40.123 | -14.545 | 85.243 | 1.00 | 27.15 | C |
| ATOM | 284 | CD1 | TRP | A | 36 | -40.208 | -14.586 | 83.880 | 1.00 | 28.66 | C |
| ATOM | 285 | CD2 | TRP | A | 36 | -38.764 | -14.186 | 85.550 | 1.00 | 30.43 | C |
| ATOM | 286 | NE1 | TRP | A | 36 | -38.998 | -14.263 | 83.319 | 1.00 | 28.45 | N |
| ATOM | 287 | CE2 | TRP | A | 36 | -38.092 | -14.011 | 84.318 | 1.00 | 31.09 | C |
| ATOM | 288 | CE3 | TRP | A | 36 | -38.051 | -13.988 | 86.744 | 1.00 | 26.18 | C |
| ATOM | 289 | CZ2 | TRP | A | 36 | -36.736 | -13.635 | 84.245 | 1.00 | 30.83 | C |
| ATOM | 290 | CZ3 | TRP | A | 36 | -36.710 | -13.624 | 86.669 | 1.00 | 31.94 | C |
| ATOM | 291 | CH2 | TRP | A | 36 | -36.065 | -13.444 | 85.426 | 1.00 | 27.34 | C |
| ATOM | 292 | N | ILE | A | 37 | -41.470 | -16.623 | 89.072 | 1.00 | 28.61 | N |
| ATOM | 293 | CA | ILE | A | 37 | -42.220 | -16.887 | 90.289 | 1.00 | 25.71 | C |
| ATOM | 294 | C | ILE | A | 37 | -41.703 | -15.924 | 91.344 | 1.00 | 26.51 | C |
| ATOM | 295 | O | ILE | A | 37 | -40.485 | -15.758 | 91.473 | 1.00 | 28.46 | O |
| ATOM | 296 | CB | ILE | A | 37 | -42.043 | -18.359 | 90.726 | 1.00 | 28.49 | C |
| ATOM | 297 | CG1 | ILE | A | 37 | -42.496 | -19.298 | 89.602 | 1.00 | 20.22 | C |
| ATOM | 298 | CG2 | ILE | A | 37 | -42.842 | -18.657 | 91.998 | 1.00 | 30.40 | C |
| ATOM | 299 | CD1 | ILE | A | 37 | -41.935 | -20.641 | 89.672 | 1.00 | 19.85 | C |
| ATOM | 300 | N | ARG | A | 38 | -42.608 | -15.271 | 92.089 | 1.00 | 27.82 | N |
| ATOM | 301 | CA | ARG | A | 38 | -42.178 | -14.383 | 93.172 | 1.00 | 31.79 | C |
| ATOM | 302 | C | ARG | A | 38 | -42.654 | -14.877 | 94.535 | 1.00 | 29.28 | C |
| ATOM | 303 | O | ARG | A | 38 | -43.711 | -15.493 | 94.666 | 1.00 | 30.31 | O |
| ATOM | 304 | CB | ARG | A | 38 | -42.642 | -12.902 | 92.983 | 1.00 | 25.98 | C |
| ATOM | 305 | CG | ARG | A | 38 | -44.132 | -12.704 | 92.827 | 1.00 | 30.50 | C |
| ATOM | 306 | CD | ARG | A | 38 | -44.718 | -11.710 | 93.796 | 1.00 | 31.27 | C |
| ATOM | 307 | NE | ARG | A | 38 | -44.660 | -10.332 | 93.330 | 1.00 | 38.33 | N |
| ATOM | 308 | CZ | ARG | A | 38 | -45.723 | -9.550 | 93.118 | 1.00 | 40.05 | C |
| ATOM | 309 | NH1 | ARG | A | 38 | -46.967 | -9.987 | 93.311 | 1.00 | 29.85 | N1+ |
| ATOM | 310 | NH2 | ARG | A | 38 | -45.529 | -8.307 | 92.711 | 1.00 | 35.67 | N |
| ATOM | 311 | N | GLN | A | 39 | -41.874 | -14.544 | 95.561 | 1.00 | 29.83 | N |
| ATOM | 312 | CA | GLN | A | 39 | -42.189 | -14.894 | 96.940 | 1.00 | 29.44 | C |
| ATOM | 313 | C | GLN | A | 39 | -42.070 | -13.629 | 97.776 | 1.00 | 30.99 | C |
| ATOM | 314 | O | GLN | A | 39 | -40.945 | -13.189 | 98.072 | 1.00 | 30.65 | O |
| ATOM | 315 | CB | GLN | A | 39 | -41.278 | -15.997 | 97.467 | 1.00 | 26.80 | C |
| ATOM | 316 | CG | GLN | A | 39 | -41.737 | -16.561 | 98.799 | 1.00 | 29.10 | C |
| ATOM | 317 | CD | GLN | A | 39 | -41.020 | -17.825 | 99.195 | 1.00 | 32.69 | C |
| ATOM | 318 | OE1 | GLN | A | 39 | -39.808 | -17.945 | 99.051 | 1.00 | 34.15 | O |
| ATOM | 319 | NE2 | GLN | A | 39 | -41.773 | -18.787 | 99.702 | 1.00 | 36.84 | N |
| ATOM | 320 | N | PRO | A | 40 | -43.199 | -13.006 | 98.142 | 1.00 | 32.88 | N |
| ATOM | 321 | CA | PRO | A | 40 | -43.142 | -11.823 | 99.012 | 1.00 | 32.74 | C |
| ATOM | 322 | C | PRO | A | 40 | -42.699 | -12.202 | 100.414 | 1.00 | 37.01 | C |
| ATOM | 323 | O | PRO | A | 40 | -42.970 | -13.320 | 100.883 | 1.00 | 37.15 | O |
| ATOM | 324 | CB | PRO | A | 40 | -44.590 | -11.305 | 99.005 | 1.00 | 28.52 | C |
| ATOM | 325 | CG | PRO | A | 40 | -45.230 | -11.963 | 97.786 | 1.00 | 33.90 | C |
| ATOM | 326 | CD | PRO | A | 40 | -44.566 | -13.290 | 97.668 | 1.00 | 32.61 | C |
| ATOM | 327 | N | PRO | A | 41 | -42.011 | -11.304 | 101.120 | 1.00 | 41.34 | N |
| ATOM | 328 | CA | PRO | A | 41 | -41.405 | -11.689 | 102.405 | 1.00 | 35.97 | C |
| ATOM | 329 | C | PRO | A | 41 | -42.486 | -12.089 | 103.398 | 1.00 | 39.15 | C |
| ATOM | 330 | O | PRO | A | 41 | -43.499 | -11.400 | 103.564 | 1.00 | 39.19 | O |
| ATOM | 331 | CB | PRO | A | 41 | -40.643 | -10.430 | 102.834 | 1.00 | 35.82 | C |
| ATOM | 332 | CG | PRO | A | 41 | -41.378 | -9.304 | 102.151 | 1.00 | 36.54 | C |
| ATOM | 333 | CD | PRO | A | 41 | -41.862 | -9.861 | 100.839 | 1.00 | 32.74 | C |
| ATOM | 334 | N | GLY | A | 42 | -42.264 | -13.220 | 104.057 | 1.00 | 39.49 | N |
| ATOM | 335 | CA | GLY | A | 42 | -43.319 | -13.884 | 104.786 | 1.00 | 43.62 | C |
| ATOM | 336 | C | GLY | A | 42 | -44.017 | -14.964 | 103.996 | 1.00 | 43.06 | C |
| ATOM | 337 | O | GLY | A | 42 | -45.137 | -15.343 | 104.347 | 1.00 | 44.55 | O |
| ATOM | 338 | N | LYS | A | 43 | -43.392 | -15.449 | 102.924 | 1.00 | 49.76 | N |
| ATOM | 339 | CA | LYS | A | 43 | -43.711 | -16.719 | 102.276 | 1.00 | 46.50 | C |
| ATOM | 340 | C | LYS | A | 43 | -44.975 | -16.606 | 101.432 | 1.00 | 41.59 | C |
| ATOM | 341 | O | LYS | A | 43 | -45.732 | -15.629 | 101.536 | 1.00 | 39.86 | O |
| ATOM | 342 | CB | LYS | A | 43 | -43.844 | -17.858 | 103.320 | 1.00 | 55.91 | C |
| ATOM | 343 | CG | LYS | A | 43 | -42.513 | -18.447 | 103.914 | 1.00 | 45.31 | C |
| ATOM | 344 | CD | LYS | A | 43 | -41.860 | -19.485 | 102.968 | 1.00 | 46.63 | C |
| ATOM | 345 | CE | LYS | A | 43 | -41.212 | -20.692 | 103.684 | 1.00 | 40.96 | C |
| ATOM | 346 | NZ | LYS | A | 43 | -39.846 | -20.507 | 104.327 | 1.00 | 38.05 | N |
| ATOM | 347 | N | GLY | A | 44 | -45.192 | -17.625 | 100.601 | 1.00 | 39.58 | N |
| ATOM | 348 | CA | GLY | A | 44 | -46.241 | -17.682 | 99.609 | 1.00 | 36.71 | C |
| ATOM | 349 | C | GLY | A | 44 | -45.620 | -17.601 | 98.231 | 1.00 | 37.64 | C |
| ATOM | 350 | O | GLY | A | 44 | -44.862 | -16.666 | 97.961 | 1.00 | 40.33 | O |
| ATOM | 351 | N | LEU | A | 45 | -45.870 | -18.568 | 97.359 | 1.00 | 32.84 | N |
| ATOM | 352 | CA | LEU | A | 45 | -45.302 | -18.533 | 96.019 | 1.00 | 31.46 | C |
| ATOM | 353 | C | LEU | A | 45 | -46.409 | -18.152 | 95.047 | 1.00 | 32.57 | C |

| | | | | | | | | | | | |
|---|---|---|---|---|---|---|---|---|---|---|---|
| ATOM | 354 | O | LEU | A | 45 | -47.476 | -18.773 | 95.054 | 1.00 | 37.03 | O |
| ATOM | 355 | CB | LEU | A | 45 | -44.665 | -19.877 | 95.649 | 1.00 | 29.61 | C |
| ATOM | 356 | CG | LEU | A | 45 | -43.412 | -20.297 | 96.434 | 1.00 | 28.20 | C |
| ATOM | 357 | CD1 | LEU | A | 45 | -43.088 | -21.788 | 96.284 | 1.00 | 22.57 | C |
| ATOM | 358 | CD2 | LEU | A | 45 | -42.235 | -19.474 | 95.976 | 1.00 | 25.94 | C |
| ATOM | 359 | N | GLU | A | 46 | -46.150 | -17.152 | 94.201 | 1.00 | 27.39 | N |
| ATOM | 360 | CA | GLU | A | 46 | -47.092 | -16.747 | 93.168 | 1.00 | 31.51 | C |
| ATOM | 361 | C | GLU | A | 46 | -46.444 | -16.851 | 91.798 | 1.00 | 35.34 | C |
| ATOM | 362 | O | GLU | A | 46 | -45.360 | -16.303 | 91.565 | 1.00 | 32.92 | O |
| ATOM | 363 | CB | GLU | A | 46 | -47.602 | -15.323 | 93.347 | 1.00 | 29.82 | C |
| ATOM | 364 | CG | GLU | A | 46 | -47.130 | -14.572 | 94.552 | 1.00 | 39.23 | C |
| ATOM | 365 | CD | GLU | A | 46 | -47.805 | -13.180 | 94.638 | 1.00 | 55.69 | C |
| ATOM | 366 | OE1 | GLU | A | 46 | -48.162 | -12.613 | 93.554 | 1.00 | 51.35 | O |
| ATOM | 367 | OE2 | GLU | A | 46 | -48.001 | -12.680 | 95.784 | 1.00 | 52.13 | O1- |
| ATOM | 368 | N | TRP | A | 47 | -47.137 | -17.524 | 90.893 | 1.00 | 29.44 | N |
| ATOM | 369 | CA | TRP | A | 47 | -46.647 | -17.778 | 89.551 | 1.00 | 30.35 | C |
| ATOM | 370 | C | TRP | A | 47 | -46.982 | -16.605 | 88.644 | 1.00 | 32.75 | C |
| ATOM | 371 | O | TRP | A | 47 | -48.143 | -16.202 | 88.550 | 1.00 | 36.68 | O |
| ATOM | 372 | CB | TRP | A | 47 | -47.265 | -19.068 | 89.037 | 1.00 | 24.36 | C |
| ATOM | 373 | CG | TRP | A | 47 | -46.961 | -19.432 | 87.637 | 1.00 | 29.61 | C |
| ATOM | 374 | CD1 | TRP | A | 47 | -45.883 | -20.133 | 87.188 | 1.00 | 25.71 | C |
| ATOM | 375 | CD2 | TRP | A | 47 | -47.784 | -19.186 | 86.495 | 1.00 | 28.45 | C |
| ATOM | 376 | NE1 | TRP | A | 47 | -45.979 | -20.319 | 85.840 | 1.00 | 26.95 | N |
| ATOM | 377 | CE2 | TRP | A | 47 | -47.136 | -19.748 | 85.388 | 1.00 | 25.32 | C |
| ATOM | 378 | CE3 | TRP | A | 47 | -49.001 | -18.540 | 86.303 | 1.00 | 25.64 | C |
| ATOM | 379 | CZ2 | TRP | A | 47 | -47.662 | -19.687 | 84.112 | 1.00 | 26.63 | C |
| ATOM | 380 | CZ3 | TRP | A | 47 | -49.517 | -18.479 | 85.032 | 1.00 | 29.15 | C |
| ATOM | 381 | CH2 | TRP | A | 47 | -48.853 | -19.054 | 83.953 | 1.00 | 27.51 | C |
| ATOM | 382 | N | PHE | A | 48 | -45.966 | -16.059 | 87.975 | 1.00 | 33.76 | N |
| ATOM | 383 | CA | PHE | A | 48 | -46.182 | -14.896 | 87.119 | 1.00 | 32.37 | C |
| ATOM | 384 | C | PHE | A | 48 | -46.601 | -15.253 | 85.708 | 1.00 | 32.57 | C |
| ATOM | 385 | O | PHE | A | 48 | -47.502 | -14.615 | 85.160 | 1.00 | 39.41 | O |
| ATOM | 386 | CB | PHE | A | 48 | -44.925 | -14.027 | 87.073 | 1.00 | 31.35 | C |
| ATOM | 387 | CG | PHE | A | 48 | -44.998 | -12.843 | 87.979 | 1.00 | 30.88 | C |
| ATOM | 388 | CD2 | PHE | A | 48 | -44.484 | -11.622 | 87.593 | 1.00 | 32.09 | C |
| ATOM | 389 | CD1 | PHE | A | 48 | -45.606 | -12.952 | 89.221 | 1.00 | 31.12 | C |
| ATOM | 390 | CE2 | PHE | A | 48 | -44.562 | -10.519 | 88.440 | 1.00 | 34.85 | C |
| ATOM | 391 | CE1 | PHE | A | 48 | -45.695 | -11.855 | 90.073 | 1.00 | 34.60 | C |
| ATOM | 392 | CZ | PHE | A | 48 | -45.165 | -10.639 | 89.683 | 1.00 | 36.89 | C |
| ATOM | 393 | N | GLY | A | 49 | -45.982 | -16.255 | 85.111 | 1.00 | 31.20 | N |
| ATOM | 394 | CA | GLY | A | 49 | -46.195 | -16.531 | 83.707 | 1.00 | 30.09 | C |
| ATOM | 395 | C | GLY | A | 49 | -45.089 | -17.418 | 83.178 | 1.00 | 31.55 | C |
| ATOM | 396 | O | GLY | A | 49 | -44.201 | -17.849 | 83.913 | 1.00 | 31.30 | O |
| ATOM | 397 | N | GLU | A | 50 | -45.176 | -17.704 | 81.881 | 1.00 | 28.58 | N |
| ATOM | 398 | CA | GLU | A | 50 | -44.233 | -18.608 | 81.247 | 1.00 | 27.28 | C |
| ATOM | 399 | C | GLU | A | 50 | -44.118 | -18.247 | 79.773 | 1.00 | 31.68 | C |
| ATOM | 400 | O | GLU | A | 50 | -45.015 | -17.630 | 79.199 | 1.00 | 32.98 | O |
| ATOM | 401 | CB | GLU | A | 50 | -44.669 | -20.060 | 81.434 | 1.00 | 26.85 | C |
| ATOM | 402 | CG | GLU | A | 50 | -46.040 | -20.348 | 80.874 | 1.00 | 29.78 | C |
| ATOM | 403 | CD | GLU | A | 50 | -46.438 | -21.812 | 81.020 | 1.00 | 35.37 | C |
| ATOM | 404 | OE1 | GLU | A | 50 | -47.350 | -22.259 | 80.271 | 1.00 | 37.88 | O |
| ATOM | 405 | OE2 | GLU | A | 50 | -45.834 | -22.514 | 81.875 | 1.00 | 28.88 | O |
| ATOM | 406 | N | ILE | A | 51 | -42.989 | -18.610 | 79.165 | 1.00 | 32.55 | N |
| ATOM | 407 | CA | ILE | A | 51 | -42.790 | -18.425 | 77.735 | 1.00 | 28.12 | C |
| ATOM | 408 | C | ILE | A | 51 | -42.187 | -19.702 | 77.167 | 1.00 | 34.07 | C |
| ATOM | 409 | O | ILE | A | 51 | -41.470 | -20.426 | 77.867 | 1.00 | 29.86 | O |
| ATOM | 410 | CB | ILE | A | 51 | -41.917 | -17.191 | 77.464 | 1.00 | 29.20 | C |
| ATOM | 411 | CG1 | ILE | A | 51 | -41.860 | -16.866 | 75.954 | 1.00 | 32.21 | C |
| ATOM | 412 | CG2 | ILE | A | 51 | -40.574 | -17.350 | 78.147 | 1.00 | 26.55 | C |
| ATOM | 413 | CD1 | ILE | A | 51 | -41.585 | -15.399 | 75.639 | 1.00 | 26.20 | C |
| ATOM | 414 | N | SER | A | 52 | -42.453 | -19.969 | 75.876 | 1.00 | 37.40 | N |
| ATOM | 415 | CA | SER | A | 52 | -42.398 | -21.343 | 75.378 | 1.00 | 41.14 | C |
| ATOM | 416 | C | SER | A | 52 | -41.162 | -21.701 | 74.563 | 1.00 | 43.60 | C |
| ATOM | 417 | O | SER | A | 52 | -40.851 | -22.904 | 74.473 | 1.00 | 56.16 | O |
| ATOM | 418 | CB | SER | A | 52 | -43.624 | -21.655 | 74.508 | 1.00 | 44.90 | C |
| ATOM | 419 | OG | SER | A | 52 | -43.499 | -21.091 | 73.211 | 1.00 | 42.47 | O |
| ATOM | 420 | N | HIS | A | 53 | -40.465 | -20.709 | 73.992 | 1.00 | 38.93 | N |
| ATOM | 421 | CA | HIS | A | 53 | -39.361 | -20.843 | 73.025 | 1.00 | 46.69 | C |
| ATOM | 422 | C | HIS | A | 53 | -39.838 | -20.322 | 71.682 | 1.00 | 40.68 | C |
| ATOM | 423 | O | HIS | A | 53 | -39.045 | -19.789 | 70.908 | 1.00 | 48.98 | O |
| ATOM | 424 | CB | HIS | A | 53 | -38.827 | -22.278 | 72.833 | 1.00 | 47.29 | C |

| | | | | | | | | | | | |
|---|---|---|---|---|---|---|---|---|---|---|---|
| ATOM | 425 | CG | HIS | A | 53 | -37.623 | -22.370 | 71.938 | 1.00 | 50.45 | C |
| ATOM | 426 | ND1 | HIS | A | 53 | -36.332 | -22.400 | 72.429 | 1.00 | 48.68 | N |
| ATOM | 427 | CD2 | HIS | A | 53 | -37.510 | -22.432 | 70.589 | 1.00 | 50.73 | C |
| ATOM | 428 | CE1 | HIS | A | 53 | -35.477 | -22.464 | 71.421 | 1.00 | 44.63 | C |
| ATOM | 429 | NE2 | HIS | A | 53 | -36.166 | -22.483 | 70.294 | 1.00 | 45.11 | N |
| ATOM | 430 | N | SER | A | 54 | -41.136 | -20.418 | 71.427 | 1.00 | 43.19 | N |
| ATOM | 431 | CA | SER | A | 54 | -41.723 | -19.986 | 70.163 | 1.00 | 37.71 | C |
| ATOM | 432 | C | SER | A | 54 | -41.978 | -18.486 | 69.934 | 1.00 | 41.91 | C |
| ATOM | 433 | O | SER | A | 54 | -42.167 | -18.132 | 68.783 | 1.00 | 58.83 | O |
| ATOM | 434 | CB | SER | A | 54 | -43.072 | -20.685 | 69.930 | 1.00 | 41.65 | C |
| ATOM | 435 | OG | SER | A | 54 | -43.284 | -21.786 | 70.799 | 1.00 | 58.13 | O |
| ATOM | 436 | N | GLY | A | 55 | -42.077 | -17.595 | 70.928 | 1.00 | 41.19 | N |
| ATOM | 437 | CA | GLY | A | 55 | -42.199 | -17.864 | 72.342 | 1.00 | 37.12 | C |
| ATOM | 438 | C | GLY | A | 55 | -43.565 | -17.363 | 72.782 | 1.00 | 38.09 | C |
| ATOM | 439 | O | GLY | A | 55 | -43.782 | -16.170 | 73.025 | 1.00 | 35.63 | O |
| ATOM | 440 | N | SER | A | 56 | -44.514 | -18.290 | 72.843 | 1.00 | 35.38 | N |
| ATOM | 441 | CA | SER | A | 56 | -45.839 | -17.960 | 73.328 | 1.00 | 36.92 | C |
| ATOM | 442 | C | SER | A | 56 | -45.838 | -17.864 | 74.851 | 1.00 | 39.87 | C |
| ATOM | 443 | O | SER | A | 56 | -45.057 | -18.536 | 75.536 | 1.00 | 38.19 | O |
| ATOM | 444 | CB | SER | A | 56 | -46.855 | -19.002 | 72.851 | 1.00 | 43.51 | C |
| ATOM | 445 | OG | SER | A | 56 | -46.477 | -20.320 | 73.214 | 1.00 | 52.12 | O |
| ATOM | 446 | N | THR | A | 57 | -46.745 | -17.035 | 75.381 | 1.00 | 36.78 | N |
| ATOM | 447 | CA | THR | A | 57 | -46.758 | -16.688 | 76.792 | 1.00 | 35.49 | C |
| ATOM | 448 | C | THR | A | 57 | -48.106 | -17.037 | 77.412 | 1.00 | 36.54 | C |
| ATOM | 449 | O | THR | A | 57 | -49.129 | -17.068 | 76.738 | 1.00 | 36.43 | O |
| ATOM | 450 | CB | THR | A | 57 | -46.464 | -15.195 | 77.048 | 1.00 | 34.28 | C |
| ATOM | 451 | OG1 | THR | A | 57 | -47.488 | -14.390 | 76.463 | 1.00 | 34.38 | O |
| ATOM | 452 | CG2 | THR | A | 57 | -45.110 | -14.791 | 76.511 | 1.00 | 32.77 | C |
| ATOM | 453 | N | ASN | A | 58 | -48.081 | -17.324 | 78.707 | 1.00 | 34.99 | N |
| ATOM | 454 | CA | ASN | A | 58 | -49.282 | -17.540 | 79.497 | 1.00 | 35.60 | C |
| ATOM | 455 | C | ASN | A | 58 | -49.013 | -16.847 | 80.812 | 1.00 | 38.61 | C |
| ATOM | 456 | O | ASN | A | 58 | -48.117 | -17.271 | 81.548 | 1.00 | 38.39 | O |
| ATOM | 457 | CB | ASN | A | 58 | -49.562 | -19.013 | 79.731 | 1.00 | 33.14 | C |
| ATOM | 458 | CG | ASN | A | 58 | -49.588 | -19.785 | 78.459 | 1.00 | 38.06 | C |
| ATOM | 459 | OD1 | ASN | A | 58 | -50.518 | -19.669 | 77.673 | 1.00 | 42.44 | O |
| ATOM | 460 | ND2 | ASN | A | 58 | -48.555 | -20.583 | 78.234 | 1.00 | 37.72 | N |
| ATOM | 461 | N | TYR | A | 59 | -49.778 | -15.800 | 81.101 | 1.00 | 35.54 | N |
| ATOM | 462 | CA | TYR | A | 59 | -49.557 | -14.988 | 82.281 | 1.00 | 36.21 | C |
| ATOM | 463 | C | TYR | A | 59 | -50.617 | -15.291 | 83.325 | 1.00 | 34.45 | C |
| ATOM | 464 | O | TYR | A | 59 | -51.674 | -15.857 | 83.041 | 1.00 | 36.30 | O |
| ATOM | 465 | CB | TYR | A | 59 | -49.603 | -13.498 | 81.943 | 1.00 | 35.28 | C |
| ATOM | 466 | CG | TYR | A | 59 | -48.633 | -13.043 | 80.880 | 1.00 | 35.74 | C |
| ATOM | 467 | CD1 | TYR | A | 59 | -47.259 | -13.085 | 81.082 | 1.00 | 34.19 | C |
| ATOM | 468 | CD2 | TYR | A | 59 | -49.101 | -12.544 | 79.678 | 1.00 | 35.17 | C |
| ATOM | 469 | CE1 | TYR | A | 59 | -46.383 | -12.644 | 80.107 | 1.00 | 33.92 | C |
| ATOM | 470 | CE2 | TYR | A | 59 | -48.241 | -12.098 | 78.700 | 1.00 | 31.35 | C |
| ATOM | 471 | CZ | TYR | A | 59 | -46.888 | -12.143 | 78.912 | 1.00 | 37.17 | C |
| ATOM | 472 | OH | TYR | A | 59 | -46.055 | -11.690 | 77.915 | 1.00 | 33.15 | O |
| ATOM | 473 | N | ASN | A | 60 | -50.335 | -14.882 | 84.531 | 1.00 | 37.70 | N |
| ATOM | 474 | CA | ASN | A | 60 | -51.356 | -14.909 | 85.549 | 1.00 | 38.74 | C |
| ATOM | 475 | C | ASN | A | 60 | -52.356 | -13.791 | 85.274 | 1.00 | 42.68 | C |
| ATOM | 476 | O | ASN | A | 60 | -51.962 | -12.615 | 85.220 | 1.00 | 41.91 | O |
| ATOM | 477 | CB | ASN | A | 60 | -50.706 | -14.771 | 86.911 | 1.00 | 35.36 | C |
| ATOM | 478 | CG | ASN | A | 60 | -51.663 | -15.019 | 88.031 | 1.00 | 37.89 | C |
| ATOM | 479 | OD1 | ASN | A | 60 | -52.874 | -14.862 | 87.873 | 1.00 | 38.66 | O |
| ATOM | 480 | ND2 | ASN | A | 60 | -51.142 | -15.541 | 89.137 | 1.00 | 36.71 | N |
| ATOM | 481 | N | PRO | A | 61 | -53.642 | -14.102 | 85.084 | 1.00 | 43.65 | N |
| ATOM | 482 | CA | PRO | A | 61 | -54.609 | -13.045 | 84.741 | 1.00 | 37.57 | C |
| ATOM | 483 | C | PRO | A | 61 | -54.670 | -11.924 | 85.762 | 1.00 | 40.30 | C |
| ATOM | 484 | O | PRO | A | 61 | -54.997 | -10.786 | 85.405 | 1.00 | 42.33 | O |
| ATOM | 485 | CB | PRO | A | 61 | -55.931 | -13.820 | 84.677 | 1.00 | 32.38 | C |
| ATOM | 486 | CG | PRO | A | 61 | -55.502 | -15.207 | 84.249 | 1.00 | 39.32 | C |
| ATOM | 487 | CD | PRO | A | 61 | -54.273 | -15.439 | 85.092 | 1.00 | 39.25 | C |
| ATOM | 488 | N | SER | A | 62 | -54.321 | -12.218 | 87.016 | 1.00 | 40.22 | N |
| ATOM | 489 | CA | SER | A | 62 | -54.367 | -11.233 | 88.092 | 1.00 | 39.59 | C |
| ATOM | 490 | C | SER | A | 62 | -53.386 | -10.087 | 87.868 | 1.00 | 45.31 | C |
| ATOM | 491 | O | SER | A | 62 | -53.617 | -8.957 | 88.319 | 1.00 | 43.39 | O |
| ATOM | 492 | CB | SER | A | 62 | -54.058 | -11.920 | 89.419 | 1.00 | 40.43 | C |
| ATOM | 493 | OG | SER | A | 62 | -53.155 | -11.129 | 90.177 | 1.00 | 48.57 | O |
| ATOM | 494 | N | LEU | A | 63 | -52.237 | -10.383 | 87.272 | 1.00 | 45.35 | N |
| ATOM | 495 | CA | LEU | A | 63 | -51.297 | -9.329 | 86.930 | 1.00 | 44.69 | C |

| | | | | | | | | | | | |
|---|---|---|---|---|---|---|---|---|---|---|---|
| ATOM | 496 | C | LEU | A | 63 | -51.894 | -8.416 | 85.869 | 1.00 | 53.96 | C |
| ATOM | 497 | O | LEU | A | 63 | -51.658 | -7.201 | 85.861 | 1.00 | 54.71 | O |
| ATOM | 498 | CB | LEU | A | 63 | -50.001 | -9.971 | 86.440 | 1.00 | 43.57 | C |
| ATOM | 499 | CG | LEU | A | 63 | -49.158 | -10.633 | 87.535 | 1.00 | 43.51 | C |
| ATOM | 500 | CD1 | LEU | A | 63 | -47.844 | -11.134 | 86.978 | 1.00 | 40.31 | C |
| ATOM | 501 | CD2 | LEU | A | 63 | -48.888 | -9.663 | 88.691 | 1.00 | 43.97 | C |
| ATOM | 502 | N | LYS | A | 64 | -52.663 | -9.003 | 84.955 | 1.00 | 58.46 | N |
| ATOM | 503 | CA | LYS | A | 64 | -53.324 | -8.320 | 83.854 | 1.00 | 58.94 | C |
| ATOM | 504 | C | LYS | A | 64 | -52.277 | -7.745 | 82.912 | 1.00 | 51.96 | C |
| ATOM | 505 | O | LYS | A | 64 | -51.377 | -8.460 | 82.452 | 1.00 | 48.34 | O |
| ATOM | 506 | CB | LYS | A | 64 | -54.274 | -7.204 | 84.304 | 1.00 | 52.16 | C |
| ATOM | 507 | CG | LYS | A | 64 | -55.571 | -7.258 | 83.486 | 1.00 | 62.77 | C |
| ATOM | 508 | CD | LYS | A | 64 | -56.400 | -5.965 | 83.488 | 1.00 | 81.85 | C |
| ATOM | 509 | CE | LYS | A | 64 | -57.048 | -5.593 | 84.810 | 1.00 | 81.71 | C |
| ATOM | 510 | NZ | LYS | A | 64 | -57.659 | -4.226 | 84.694 | 1.00 | 73.63 | N |
| ATOM | 511 | N | ALA | A | 65 | -52.386 | -6.444 | 82.659 | 1.00 | 41.71 | N |
| ATOM | 512 | CA | ALA | A | 65 | -51.539 | -5.768 | 81.693 | 1.00 | 43.20 | C |
| ATOM | 513 | C | ALA | A | 65 | -50.168 | -5.367 | 82.231 | 1.00 | 38.62 | C |
| ATOM | 514 | O | ALA | A | 65 | -49.389 | -4.808 | 81.463 | 1.00 | 42.39 | O |
| ATOM | 515 | CB | ALA | A | 65 | -52.249 | -4.540 | 81.128 | 1.00 | 46.41 | C |
| ATOM | 516 | N | ARG | A | 66 | -49.867 | -5.542 | 83.523 | 1.00 | 38.64 | N |
| ATOM | 517 | CA | ARG | A | 66 | -48.595 | -5.016 | 84.025 | 1.00 | 34.40 | C |
| ATOM | 518 | C | ARG | A | 66 | -47.377 | -5.847 | 83.627 | 1.00 | 36.18 | C |
| ATOM | 519 | O | ARG | A | 66 | -46.249 | -5.349 | 83.752 | 1.00 | 31.12 | O |
| ATOM | 520 | CB | ARG | A | 66 | -48.586 | -4.863 | 85.546 | 1.00 | 33.68 | C |
| ATOM | 521 | CG | ARG | A | 66 | -49.764 | -4.132 | 86.091 | 1.00 | 40.09 | C |
| ATOM | 522 | CD | ARG | A | 66 | -49.537 | -3.649 | 87.519 | 1.00 | 40.22 | C |
| ATOM | 523 | NE | ARG | A | 66 | -49.265 | -4.663 | 88.539 | 1.00 | 35.47 | N |
| ATOM | 524 | CZ | ARG | A | 66 | -48.148 | -4.713 | 89.268 | 1.00 | 34.74 | C |
| ATOM | 525 | NH1 | ARG | A | 66 | -47.168 | -3.842 | 89.065 | 1.00 | 33.41 | N1+ |
| ATOM | 526 | NH2 | ARG | A | 66 | -48.004 | -5.640 | 90.205 | 1.00 | 40.56 | N |
| ATOM | 527 | N | VAL | A | 67 | -47.559 | -7.095 | 83.188 | 1.00 | 34.62 | N |
| ATOM | 528 | CA | VAL | A | 67 | -46.463 | -8.049 | 83.084 | 1.00 | 36.24 | C |
| ATOM | 529 | C | VAL | A | 67 | -46.186 | -8.411 | 81.626 | 1.00 | 36.09 | C |
| ATOM | 530 | O | VAL | A | 67 | -47.113 | -8.575 | 80.828 | 1.00 | 36.24 | O |
| ATOM | 531 | CB | VAL | A | 67 | -46.782 | -9.292 | 83.935 | 1.00 | 34.93 | C |
| ATOM | 532 | CG1 | VAL | A | 67 | -47.992 | -9.994 | 83.388 | 1.00 | 41.56 | C |
| ATOM | 533 | CG2 | VAL | A | 67 | -45.596 | -10.222 | 83.981 | 1.00 | 36.82 | C |
| ATOM | 534 | N | THR | A | 68 | -44.894 | -8.531 | 81.289 | 1.00 | 35.40 | N |
| ATOM | 535 | CA | THR | A | 68 | -44.405 | -8.998 | 79.998 | 1.00 | 33.94 | C |
| ATOM | 536 | C | THR | A | 68 | -43.267 | -9.973 | 80.231 | 1.00 | 33.46 | C |
| ATOM | 537 | O | THR | A | 68 | -42.412 | -9.739 | 81.087 | 1.00 | 34.05 | O |
| ATOM | 538 | CB | THR | A | 68 | -43.854 | -7.882 | 79.090 | 1.00 | 37.93 | C |
| ATOM | 539 | OG1 | THR | A | 68 | -44.745 | -6.763 | 79.052 | 1.00 | 38.69 | O |
| ATOM | 540 | CG2 | THR | A | 68 | -43.621 | -8.423 | 77.672 | 1.00 | 27.13 | C |
| ATOM | 541 | N | ILE | A | 69 | -43.262 | -11.064 | 79.471 | 1.00 | 35.17 | N |
| ATOM | 542 | CA | ILE | A | 69 | -42.163 | -12.021 | 79.455 | 1.00 | 31.25 | C |
| ATOM | 543 | C | ILE | A | 69 | -41.710 | -12.169 | 78.008 | 1.00 | 37.24 | C |
| ATOM | 544 | O | ILE | A | 69 | -42.541 | -12.313 | 77.103 | 1.00 | 33.89 | O |
| ATOM | 545 | CB | ILE | A | 69 | -42.574 | -13.382 | 80.051 | 1.00 | 27.84 | C |
| ATOM | 546 | CG1 | ILE | A | 69 | -42.912 | -13.212 | 81.523 | 1.00 | 31.71 | C |
| ATOM | 547 | CG2 | ILE | A | 69 | -41.468 | -14.407 | 79.896 | 1.00 | 25.74 | C |
| ATOM | 548 | CD1 | ILE | A | 69 | -43.605 | -14.385 | 82.110 | 1.00 | 30.41 | C |
| ATOM | 549 | N | SER | A | 70 | -40.401 | -12.101 | 77.789 | 1.00 | 32.26 | N |
| ATOM | 550 | CA | SER | A | 70 | -39.826 | -12.197 | 76.460 | 1.00 | 29.37 | C |
| ATOM | 551 | C | SER | A | 70 | -38.678 | -13.195 | 76.500 | 1.00 | 33.52 | C |
| ATOM | 552 | O | SER | A | 70 | -38.103 | -13.470 | 77.557 | 1.00 | 32.58 | O |
| ATOM | 553 | CB | SER | A | 70 | -39.322 | -10.841 | 75.963 | 1.00 | 30.41 | C |
| ATOM | 554 | OG | SER | A | 70 | -38.395 | -10.282 | 76.888 | 1.00 | 41.93 | O |
| ATOM | 555 | N | ILE | A | 71 | -38.333 | -13.724 | 75.331 | 1.00 | 30.14 | N |
| ATOM | 556 | CA | ILE | A | 71 | -37.327 | -14.761 | 75.222 | 1.00 | 29.53 | C |
| ATOM | 557 | C | ILE | A | 71 | -36.358 | -14.414 | 74.097 | 1.00 | 34.98 | C |
| ATOM | 558 | O | ILE | A | 71 | -36.762 | -13.926 | 73.035 | 1.00 | 33.60 | O |
| ATOM | 559 | CB | ILE | A | 71 | -37.992 | -16.132 | 75.000 | 1.00 | 29.32 | C |
| ATOM | 560 | CG1 | ILE | A | 71 | -37.001 | -17.267 | 75.279 | 1.00 | 29.60 | C |
| ATOM | 561 | CG2 | ILE | A | 71 | -38.575 | -16.224 | 73.606 | 1.00 | 30.62 | C |
| ATOM | 562 | CD1 | ILE | A | 71 | -37.644 | -18.637 | 75.331 | 1.00 | 29.78 | C |
| ATOM | 563 | N | ASP | A | 72 | -35.076 | -14.658 | 74.339 | 1.00 | 33.23 | N |
| ATOM | 564 | CA | ASP | A | 72 | -34.016 | -14.453 | 73.355 | 1.00 | 32.69 | C |
| ATOM | 565 | C | ASP | A | 72 | -33.394 | -15.828 | 73.138 | 1.00 | 36.12 | C |
| ATOM | 566 | O | ASP | A | 72 | -32.525 | -16.242 | 73.910 | 1.00 | 37.95 | O |

| | | | | | | | | | | | |
|---|---|---|---|---|---|---|---|---|---|---|---|
| ATOM | 567 | CB | ASP | A | 72 | -33.008 | -13.415 | 73.862 | 1.00 | 31.36 | C |
| ATOM | 568 | CG | ASP | A | 72 | -31.876 | -13.113 | 72.861 | 1.00 | 47.78 | C |
| ATOM | 569 | OD2 | ASP | A | 72 | -31.214 | -12.051 | 73.040 | 1.00 | 43.89 | O |
| ATOM | 570 | OD1 | ASP | A | 72 | -31.629 | -13.924 | 71.921 | 1.00 | 48.56 | O |
| ATOM | 571 | N | THR | A | 73 | -33.847 | -16.547 | 72.100 | 1.00 | 39.39 | N |
| ATOM | 572 | CA | THR | A | 73 | -33.375 | -17.916 | 71.888 | 1.00 | 36.77 | C |
| ATOM | 573 | C | THR | A | 73 | -31.919 | -17.989 | 71.429 | 1.00 | 43.07 | C |
| ATOM | 574 | O | THR | A | 73 | -31.301 | -19.055 | 71.535 | 1.00 | 46.66 | O |
| ATOM | 575 | CB | THR | A | 73 | -34.251 | -18.621 | 70.868 | 1.00 | 36.60 | C |
| ATOM | 576 | OG1 | THR | A | 73 | -34.346 | -17.803 | 69.699 | 1.00 | 47.78 | O |
| ATOM | 577 | CG2 | THR | A | 73 | -35.625 | -18.860 | 71.428 | 1.00 | 33.19 | C |
| ATOM | 578 | N | SER | A | 74 | -31.349 | -16.886 | 70.949 | 1.00 | 40.76 | N |
| ATOM | 579 | CA | SER | A | 74 | -29.949 | -16.891 | 70.549 | 1.00 | 43.57 | C |
| ATOM | 580 | C | SER | A | 74 | -29.032 | -16.974 | 71.758 | 1.00 | 50.46 | C |
| ATOM | 581 | O | SER | A | 74 | -28.083 | -17.779 | 71.784 | 1.00 | 52.51 | O |
| ATOM | 582 | CB | SER | A | 74 | -29.654 | -15.639 | 69.744 | 1.00 | 48.36 | C |
| ATOM | 583 | OG | SER | A | 74 | -30.524 | -15.613 | 68.635 | 1.00 | 60.90 | O |
| ATOM | 584 | O | LYS | A | 75 | -28.334 | -16.929 | 76.160 | 1.00 | 39.85 | O |
| ATOM | 585 | N | LYS | A | 75 | -29.291 | -16.123 | 72.754 | 1.00 | 40.62 | N |
| ATOM | 586 | CA | LYS | A | 75 | -28.494 | -15.995 | 73.967 | 1.00 | 44.56 | C |
| ATOM | 587 | C | LYS | A | 75 | -28.929 | -16.951 | 75.076 | 1.00 | 39.19 | C |
| ATOM | 588 | CB | LYS | A | 75 | -28.574 | -14.552 | 74.490 | 1.00 | 42.65 | C |
| ATOM | 589 | CG | LYS | A | 75 | -28.087 | -13.508 | 73.502 | 1.00 | 48.05 | C |
| ATOM | 590 | CD | LYS | A | 75 | -28.250 | -12.099 | 74.045 | 1.00 | 49.13 | C |
| ATOM | 591 | CE | LYS | A | 75 | -28.043 | -11.079 | 72.924 | 1.00 | 55.25 | C |
| ATOM | 592 | NZ | LYS | A | 75 | -27.500 | -9.765 | 73.364 | 1.00 | 55.09 | N |
| ATOM | 593 | N | ASN | A | 76 | -29.936 | -17.793 | 74.823 | 1.00 | 43.17 | N |
| ATOM | 594 | CA | ASN | A | 76 | -30.578 | -18.625 | 75.845 | 1.00 | 36.87 | C |
| ATOM | 595 | C | ASN | A | 76 | -30.949 | -17.780 | 77.073 | 1.00 | 35.94 | C |
| ATOM | 596 | O | ASN | A | 76 | -30.668 | -18.130 | 78.221 | 1.00 | 33.33 | O |
| ATOM | 597 | CB | ASN | A | 76 | -29.696 | -19.823 | 76.213 | 1.00 | 32.65 | C |
| ATOM | 598 | CG | ASN | A | 76 | -29.729 | -20.940 | 75.144 | 1.00 | 41.58 | C |
| ATOM | 599 | OD1 | ASN | A | 76 | -30.207 | -20.738 | 74.016 | 1.00 | 42.66 | O |
| ATOM | 600 | ND2 | ASN | A | 76 | -29.215 | -22.117 | 75.500 | 1.00 | 36.55 | N |
| ATOM | 601 | N | GLN | A | 77 | -31.635 | -16.668 | 76.813 | 1.00 | 29.24 | N |
| ATOM | 602 | CA | GLN | A | 77 | -32.064 | -15.729 | 77.828 | 1.00 | 28.20 | C |
| ATOM | 603 | C | GLN | A | 77 | -33.578 | -15.515 | 77.767 | 1.00 | 32.38 | C |
| ATOM | 604 | O | GLN | A | 77 | -34.233 | -15.760 | 76.747 | 1.00 | 32.97 | O |
| ATOM | 605 | CB | GLN | A | 77 | -31.365 | -14.391 | 77.668 | 1.00 | 31.15 | C |
| ATOM | 606 | CG | GLN | A | 77 | -29.894 | -14.376 | 78.032 | 1.00 | 35.97 | C |
| ATOM | 607 | CD | GLN | A | 77 | -29.313 | -12.956 | 77.951 | 1.00 | 39.65 | C |
| ATOM | 608 | OE1 | GLN | A | 77 | -29.979 | -12.017 | 77.502 | 1.00 | 40.22 | O |
| ATOM | 609 | NE2 | GLN | A | 77 | -28.096 | -12.792 | 78.433 | 1.00 | 41.23 | N |
| ATOM | 610 | N | PHE | A | 78 | -34.148 | -15.096 | 78.891 | 1.00 | 24.83 | N |
| ATOM | 611 | CA | PHE | A | 78 | -35.544 | -14.682 | 78.904 | 1.00 | 30.44 | C |
| ATOM | 612 | C | PHE | A | 78 | -35.711 | -13.657 | 80.014 | 1.00 | 28.14 | C |
| ATOM | 613 | O | PHE | A | 78 | -34.863 | -13.544 | 80.899 | 1.00 | 29.63 | O |
| ATOM | 614 | CB | PHE | A | 78 | -36.519 | -15.877 | 79.038 | 1.00 | 27.85 | C |
| ATOM | 615 | CG | PHE | A | 78 | -36.399 | -16.648 | 80.334 | 1.00 | 29.82 | C |
| ATOM | 616 | CD1 | PHE | A | 78 | -35.434 | -17.641 | 80.482 | 1.00 | 28.03 | C |
| ATOM | 617 | CD2 | PHE | A | 78 | -37.290 | -16.414 | 81.385 | 1.00 | 27.75 | C |
| ATOM | 618 | CE1 | PHE | A | 78 | -35.334 | -18.366 | 81.658 | 1.00 | 28.84 | C |
| ATOM | 619 | CE2 | PHE | A | 78 | -37.201 | -17.127 | 82.571 | 1.00 | 29.25 | C |
| ATOM | 620 | CZ | PHE | A | 78 | -36.224 | -18.108 | 82.713 | 1.00 | 31.58 | C |
| ATOM | 621 | N | SER | A | 79 | -36.779 | -12.866 | 79.933 | 1.00 | 27.98 | N |
| ATOM | 622 | CA | SER | A | 79 | -36.845 | -11.640 | 80.717 | 1.00 | 29.89 | C |
| ATOM | 623 | C | SER | A | 79 | -38.230 | -11.432 | 81.299 | 1.00 | 27.88 | C |
| ATOM | 624 | O | SER | A | 79 | -39.225 | -11.992 | 80.827 | 1.00 | 27.43 | O |
| ATOM | 625 | CB | SER | A | 79 | -36.437 | -10.410 | 79.881 | 1.00 | 27.31 | C |
| ATOM | 626 | OG | SER | A | 79 | -35.076 | -10.523 | 79.495 | 1.00 | 33.81 | O |
| ATOM | 627 | N | LEU | A | 80 | -38.270 | -10.585 | 82.327 | 1.00 | 24.79 | N |
| ATOM | 628 | CA | LEU | A | 80 | -39.503 | -10.224 | 82.998 | 1.00 | 25.66 | C |
| ATOM | 629 | C | LEU | A | 80 | -39.605 | -8.711 | 83.069 | 1.00 | 30.62 | C |
| ATOM | 630 | O | LEU | A | 80 | -38.669 | -8.045 | 83.525 | 1.00 | 32.93 | O |
| ATOM | 631 | CB | LEU | A | 80 | -39.563 | -10.806 | 84.409 | 1.00 | 30.64 | C |
| ATOM | 632 | CG | LEU | A | 80 | -40.803 | -10.348 | 85.179 | 1.00 | 29.58 | C |
| ATOM | 633 | CD2 | LEU | A | 80 | -40.702 | -10.668 | 86.648 | 1.00 | 29.00 | C |
| ATOM | 634 | CD1 | LEU | A | 80 | -42.002 | -11.007 | 84.558 | 1.00 | 27.41 | C |
| ATOM | 635 | N | LYS | A | 81 | -40.750 | -8.174 | 82.655 | 1.00 | 30.10 | N |
| ATOM | 636 | CA | LYS | A | 81 | -41.086 | -6.769 | 82.845 | 1.00 | 30.96 | C |
| ATOM | 637 | C | LYS | A | 81 | -42.340 | -6.677 | 83.705 | 1.00 | 30.22 | C |

| | | | | | | | | | | | |
|---|---|---|---|---|---|---|---|---|---|---|---|
| ATOM | 638 | O | LYS | A | 81 | -43.320 | -7.382 | 83.453 | 1.00 | 32.80 | O |
| ATOM | 639 | CB | LYS | A | 81 | -41.299 | -6.065 | 81.499 | 1.00 | 30.39 | C |
| ATOM | 640 | CG | LYS | A | 81 | -40.033 | -5.832 | 80.708 | 1.00 | 30.35 | C |
| ATOM | 641 | CD | LYS | A | 81 | -39.381 | -4.518 | 81.077 | 1.00 | 41.11 | C |
| ATOM | 642 | CE | LYS | A | 81 | -38.053 | -4.352 | 80.368 | 1.00 | 43.35 | C |
| ATOM | 643 | NZ | LYS | A | 81 | -38.187 | -4.617 | 78.920 | 1.00 | 51.59 | N |
| ATOM | 644 | N | LEU | A | 82 | -42.298 | -5.837 | 84.739 | 1.00 | 30.39 | N |
| ATOM | 645 | CA | LEU | A | 82 | -43.450 | -5.573 | 85.601 | 1.00 | 29.80 | C |
| ATOM | 646 | C | LEU | A | 82 | -43.573 | -4.071 | 85.755 | 1.00 | 32.85 | C |
| ATOM | 647 | O | LEU | A | 82 | -42.697 | -3.440 | 86.352 | 1.00 | 31.95 | O |
| ATOM | 648 | CB | LEU | A | 82 | -43.304 | -6.239 | 86.968 | 1.00 | 32.00 | C |
| ATOM | 649 | CG | LEU | A | 82 | -44.442 | -6.002 | 87.959 | 1.00 | 32.12 | C |
| ATOM | 650 | CD1 | LEU | A | 82 | -45.711 | -6.714 | 87.526 | 1.00 | 26.83 | C |
| ATOM | 651 | CD2 | LEU | A | 82 | -44.016 | -6.435 | 89.349 | 1.00 | 28.77 | C |
| ATOM | 652 | N | ARG | A | 83 | -44.645 | -3.505 | 85.209 | 1.00 | 35.72 | N |
| ATOM | 653 | CA | ARG | A | 83 | -44.790 | -2.062 | 85.113 | 1.00 | 33.38 | C |
| ATOM | 654 | C | ARG | A | 83 | -45.506 | -1.514 | 86.344 | 1.00 | 34.77 | C |
| ATOM | 655 | O | ARG | A | 83 | -46.221 | -2.234 | 87.051 | 1.00 | 37.08 | O |
| ATOM | 656 | CB | ARG | A | 83 | -45.556 | -1.686 | 83.838 | 1.00 | 30.94 | C |
| ATOM | 657 | CG | ARG | A | 83 | -44.709 | -1.827 | 82.572 | 1.00 | 31.53 | C |
| ATOM | 658 | CD | ARG | A | 83 | -45.526 | -1.799 | 81.301 | 1.00 | 34.78 | C |
| ATOM | 659 | NE | ARG | A | 83 | -46.273 | -3.036 | 81.049 | 1.00 | 36.35 | N |
| ATOM | 660 | CZ | ARG | A | 83 | -45.806 | -4.149 | 80.484 | 1.00 | 32.43 | C |
| ATOM | 661 | NH1 | ARG | A | 83 | -46.625 | -5.178 | 80.327 | 1.00 | 35.09 | N |
| ATOM | 662 | NH2 | ARG | A | 83 | -44.550 | -4.257 | 80.084 | 1.00 | 29.31 | N |
| ATOM | 663 | N | SER | A | 84 | -45.335 | -0.214 | 86.571 | 1.00 | 37.19 | N |
| ATOM | 664 | CA | SER | A | 84 | -46.105 | 0.534 | 87.573 | 1.00 | 35.36 | C |
| ATOM | 665 | C | SER | A | 84 | -46.068 | -0.152 | 88.936 | 1.00 | 39.37 | C |
| ATOM | 666 | O | SER | A | 84 | -47.092 | -0.470 | 89.546 | 1.00 | 39.10 | O |
| ATOM | 667 | CB | SER | A | 84 | -47.548 | 0.713 | 87.111 | 1.00 | 33.54 | C |
| ATOM | 668 | OG | SER | A | 84 | -47.598 | 1.374 | 85.864 | 1.00 | 48.40 | O |
| ATOM | 669 | N | VAL | A | 85 | -44.868 | -0.296 | 89.432 | 1.00 | 35.87 | N |
| ATOM | 670 | CA | VAL | A | 85 | -44.588 | -1.159 | 90.568 | 1.00 | 36.45 | C |
| ATOM | 671 | C | VAL | A | 85 | -44.889 | -0.417 | 91.872 | 1.00 | 34.18 | C |
| ATOM | 672 | O | VAL | A | 85 | -44.767 | 0.807 | 91.943 | 1.00 | 36.97 | O |
| ATOM | 673 | CB | VAL | A | 85 | -43.117 | -1.606 | 90.424 | 1.00 | 35.38 | C |
| ATOM | 674 | CG1 | VAL | A | 85 | -42.214 | -0.855 | 91.349 | 1.00 | 36.25 | C |
| ATOM | 675 | CG2 | VAL | A | 85 | -42.981 | -3.082 | 90.543 | 1.00 | 34.41 | C |
| ATOM | 676 | N | THR | A | 86 | -45.346 | -1.130 | 92.907 | 1.00 | 36.49 | N |
| ATOM | 677 | CA | THR | A | 86 | -45.612 | -0.502 | 94.212 | 1.00 | 32.44 | C |
| ATOM | 678 | C | THR | A | 86 | -44.908 | -1.279 | 95.326 | 1.00 | 33.48 | C |
| ATOM | 679 | O | THR | A | 86 | -44.209 | -2.271 | 95.087 | 1.00 | 34.30 | O |
| ATOM | 680 | CB | THR | A | 86 | -47.107 | -0.413 | 94.556 | 1.00 | 32.14 | C |
| ATOM | 681 | OG1 | THR | A | 86 | -47.526 | -1.616 | 95.204 | 1.00 | 36.59 | O |
| ATOM | 682 | CG2 | THR | A | 86 | -47.956 | -0.200 | 93.321 | 1.00 | 27.41 | C |
| ATOM | 683 | N | ALA | A | 87 | -45.082 | -0.810 | 96.565 | 1.00 | 35.31 | N |
| ATOM | 684 | CA | ALA | A | 87 | -44.442 | -1.484 | 97.698 | 1.00 | 33.73 | C |
| ATOM | 685 | C | ALA | A | 87 | -44.927 | -2.921 | 97.822 | 1.00 | 31.88 | C |
| ATOM | 686 | O | ALA | A | 87 | -44.184 | -3.798 | 98.273 | 1.00 | 31.04 | O |
| ATOM | 687 | CB | ALA | A | 87 | -44.701 | -0.719 | 98.996 | 1.00 | 25.59 | C |
| ATOM | 688 | N | ALA | A | 88 | -46.161 | -3.186 | 97.408 | 1.00 | 30.13 | N |
| ATOM | 689 | CA | ALA | A | 88 | -46.689 | -4.537 | 97.438 | 1.00 | 27.33 | C |
| ATOM | 690 | C | ALA | A | 88 | -45.962 | -5.482 | 96.487 | 1.00 | 29.67 | C |
| ATOM | 691 | O | ALA | A | 88 | -46.226 | -6.681 | 96.532 | 1.00 | 32.42 | O |
| ATOM | 692 | CB | ALA | A | 88 | -48.185 | -4.510 | 97.113 | 1.00 | 20.61 | C |
| ATOM | 693 | N | ASP | A | 89 | -45.073 | -4.987 | 95.628 | 1.00 | 29.86 | N |
| ATOM | 694 | CA | ASP | A | 89 | -44.275 | -5.840 | 94.756 | 1.00 | 27.31 | C |
| ATOM | 695 | C | ASP | A | 89 | -42.892 | -6.166 | 95.332 | 1.00 | 29.16 | C |
| ATOM | 696 | O | ASP | A | 89 | -42.057 | -6.737 | 94.618 | 1.00 | 28.53 | O |
| ATOM | 697 | CB | ASP | A | 89 | -44.141 | -5.200 | 93.359 | 1.00 | 31.57 | C |
| ATOM | 698 | CG | ASP | A | 89 | -45.504 | -4.988 | 92.660 | 1.00 | 35.37 | C |
| ATOM | 699 | OD1 | ASP | A | 89 | -46.204 | -5.990 | 92.421 | 1.00 | 36.17 | O |
| ATOM | 700 | OD2 | ASP | A | 89 | -45.880 | -3.830 | 92.332 | 1.00 | 34.69 | O1- |
| ATOM | 701 | N | THR | A | 90 | -42.616 | -5.798 | 96.590 | 1.00 | 31.96 | N |
| ATOM | 702 | CA | THR | A | 90 | -41.400 | -6.246 | 97.270 | 1.00 | 27.24 | C |
| ATOM | 703 | C | THR | A | 90 | -41.440 | -7.754 | 97.476 | 1.00 | 25.30 | C |
| ATOM | 704 | O | THR | A | 90 | -42.379 | -8.277 | 98.074 | 1.00 | 30.54 | O |
| ATOM | 705 | CB | THR | A | 90 | -41.237 | -5.541 | 98.604 | 1.00 | 25.80 | C |
| ATOM | 706 | OG1 | THR | A | 90 | -40.966 | -4.159 | 98.369 | 1.00 | 34.42 | O |
| ATOM | 707 | CG2 | THR | A | 90 | -40.111 | -6.162 | 99.391 | 1.00 | 23.83 | C |
| ATOM | 708 | N | ALA | A | 91 | -40.460 | -8.455 | 96.928 | 1.00 | 26.62 | N |

| | | | | | | | | | | | |
|---|---|---|---|---|---|---|---|---|---|---|---|
| ATOM | 709 | CA | ALA | A | 91 | -40.455 | -9.906 | 96.947 | 1.00 | 26.31 | C |
| ATOM | 710 | C | ALA | A | 91 | -39.136 | -10.402 | 96.385 | 1.00 | 31.03 | C |
| ATOM | 711 | O | ALA | A | 91 | -38.386 | -9.658 | 95.738 | 1.00 | 27.14 | O |
| ATOM | 712 | CB | ALA | A | 91 | -41.605 | -10.497 | 96.134 | 1.00 | 25.43 | C |
| ATOM | 713 | N | VAL | A | 92 | -38.876 | -11.680 | 96.632 | 1.00 | 31.34 | N |
| ATOM | 714 | CA | VAL | A | 92 | -37.860 | -12.385 | 95.875 | 1.00 | 31.22 | C |
| ATOM | 715 | C | VAL | A | 92 | -38.510 | -12.880 | 94.591 | 1.00 | 32.03 | C |
| ATOM | 716 | O | VAL | A | 92 | -39.615 | -13.429 | 94.616 | 1.00 | 32.99 | O |
| ATOM | 717 | CB | VAL | A | 92 | -37.243 | -13.525 | 96.695 | 1.00 | 25.83 | C |
| ATOM | 718 | CG1 | VAL | A | 92 | -36.306 | -14.333 | 95.817 | 1.00 | 27.86 | C |
| ATOM | 719 | CG2 | VAL | A | 92 | -36.479 | -12.962 | 97.892 | 1.00 | 20.30 | C |
| ATOM | 720 | N | TYR | A | 93 | -37.871 | -12.596 | 93.460 | 1.00 | 31.17 | N |
| ATOM | 721 | CA | TYR | A | 93 | -38.321 | -13.013 | 92.141 | 1.00 | 25.61 | C |
| ATOM | 722 | C | TYR | A | 93 | -37.385 | -14.112 | 91.680 | 1.00 | 27.44 | C |
| ATOM | 723 | O | TYR | A | 93 | -36.167 | -13.932 | 91.702 | 1.00 | 29.84 | O |
| ATOM | 724 | CB | TYR | A | 93 | -38.327 | -11.832 | 91.166 | 1.00 | 24.64 | C |
| ATOM | 725 | CG | TYR | A | 93 | -39.424 | -10.852 | 91.484 | 1.00 | 26.39 | C |
| ATOM | 726 | CD1 | TYR | A | 93 | -39.346 | -10.047 | 92.606 | 1.00 | 24.17 | C |
| ATOM | 727 | CD2 | TYR | A | 93 | -40.556 | -10.756 | 90.675 | 1.00 | 29.39 | C |
| ATOM | 728 | CE1 | TYR | A | 93 | -40.360 | -9.180 | 92.927 | 1.00 | 30.45 | C |
| ATOM | 729 | CE2 | TYR | A | 93 | -41.573 | -9.886 | 90.974 | 1.00 | 28.95 | C |
| ATOM | 730 | CZ | TYR | A | 93 | -41.470 | -9.096 | 92.108 | 1.00 | 30.87 | C |
| ATOM | 731 | OH | TYR | A | 93 | -42.478 | -8.228 | 92.442 | 1.00 | 29.44 | O |
| ATOM | 732 | N | TYR | A | 94 | -37.946 | -15.271 | 91.356 | 1.00 | 27.38 | N |
| ATOM | 733 | CA | TYR | A | 94 | -37.195 | -16.387 | 90.803 | 1.00 | 27.27 | C |
| ATOM | 734 | C | TYR | A | 94 | -37.582 | -16.602 | 89.347 | 1.00 | 27.79 | C |
| ATOM | 735 | O | TYR | A | 94 | -38.728 | -16.360 | 88.943 | 1.00 | 25.82 | O |
| ATOM | 736 | CB | TYR | A | 94 | -37.453 | -17.695 | 91.544 | 1.00 | 24.75 | C |
| ATOM | 737 | CG | TYR | A | 94 | -37.170 | -17.737 | 92.995 | 1.00 | 23.43 | C |
| ATOM | 738 | CD1 | TYR | A | 94 | -35.885 | -17.935 | 93.462 | 1.00 | 28.16 | C |
| ATOM | 739 | CD2 | TYR | A | 94 | -38.199 | -17.643 | 93.910 | 1.00 | 25.00 | C |
| ATOM | 740 | CE1 | TYR | A | 94 | -35.621 | -18.000 | 94.820 | 1.00 | 32.06 | C |
| ATOM | 741 | CE2 | TYR | A | 94 | -37.955 | -17.702 | 95.271 | 1.00 | 29.99 | C |
| ATOM | 742 | CZ | TYR | A | 94 | -36.666 | -17.881 | 95.720 | 1.00 | 33.84 | C |
| ATOM | 743 | OH | TYR | A | 94 | -36.428 | -17.954 | 97.069 | 1.00 | 39.80 | O |
| ATOM | 744 | N | CYS | A | 95 | -36.618 | -17.046 | 88.560 | 1.00 | 23.05 | N |
| ATOM | 745 | CA | CYS | A | 95 | -36.940 | -17.717 | 87.319 | 1.00 | 26.62 | C |
| ATOM | 746 | C | CYS | A | 95 | -36.809 | -19.212 | 87.547 | 1.00 | 27.72 | C |
| ATOM | 747 | O | CYS | A | 95 | -36.050 | -19.661 | 88.416 | 1.00 | 27.65 | O |
| ATOM | 748 | CB | CYS | A | 95 | -36.029 | -17.289 | 86.177 | 1.00 | 33.36 | C |
| ATOM | 749 | SG | CYS | A | 95 | -34.315 | -17.693 | 86.436 | 1.00 | 42.59 | S |
| ATOM | 750 | N | ALA | A | 96 | -37.549 | -19.988 | 86.766 | 1.00 | 25.63 | N |
| ATOM | 751 | CA | ALA | A | 96 | -37.488 | -21.432 | 86.941 | 1.00 | 24.92 | C |
| ATOM | 752 | C | ALA | A | 96 | -37.693 | -22.118 | 85.603 | 1.00 | 23.67 | C |
| ATOM | 753 | O | ALA | A | 96 | -38.234 | -21.545 | 84.650 | 1.00 | 22.72 | O |
| ATOM | 754 | CB | ALA | A | 96 | -38.514 | -21.933 | 87.971 | 1.00 | 20.77 | C |
| ATOM | 755 | N | ARG | A | 97 | -37.232 | -23.359 | 85.549 | 1.00 | 29.65 | N |
| ATOM | 756 | CA | ARG | A | 97 | -37.442 | -24.222 | 84.397 | 1.00 | 27.16 | C |
| ATOM | 757 | C | ARG | A | 97 | -38.707 | -25.049 | 84.601 | 1.00 | 25.26 | C |
| ATOM | 758 | O | ARG | A | 97 | -38.796 | -25.837 | 85.543 | 1.00 | 26.21 | O |
| ATOM | 759 | CB | ARG | A | 97 | -36.241 | -25.141 | 84.199 | 1.00 | 28.02 | C |
| ATOM | 760 | CG | ARG | A | 97 | -36.333 | -25.943 | 82.927 | 1.00 | 30.37 | C |
| ATOM | 761 | CD | ARG | A | 97 | -35.057 | -26.647 | 82.678 | 1.00 | 30.79 | C |
| ATOM | 762 | NE | ARG | A | 97 | -35.199 | -28.067 | 82.916 | 1.00 | 37.06 | N |
| ATOM | 763 | CZ | ARG | A | 97 | -34.181 | -28.869 | 83.182 | 1.00 | 41.04 | C |
| ATOM | 764 | NH1 | ARG | A | 97 | -32.962 | -28.358 | 83.257 | 1.00 | 44.36 | N |
| ATOM | 765 | NH2 | ARG | A | 97 | -34.378 | -30.172 | 83.387 | 1.00 | 43.81 | N |
| ATOM | 766 | N | ASP | A | 98 | -39.663 | -24.894 | 83.701 | 1.00 | 28.03 | N |
| ATOM | 767 | CA | ASP | A | 98 | -40.928 | -25.608 | 83.765 | 1.00 | 28.42 | C |
| ATOM | 768 | C | ASP | A | 98 | -40.781 | -26.830 | 82.871 | 1.00 | 29.02 | C |
| ATOM | 769 | O | ASP | A | 98 | -40.568 | -26.690 | 81.661 | 1.00 | 30.24 | O |
| ATOM | 770 | CB | ASP | A | 98 | -42.074 | -24.693 | 83.315 | 1.00 | 28.02 | C |
| ATOM | 771 | CG | ASP | A | 98 | -43.460 | -25.253 | 83.615 | 1.00 | 32.08 | C |
| ATOM | 772 | OD2 | ASP | A | 98 | -44.210 | -24.615 | 84.387 | 1.00 | 34.95 | O |
| ATOM | 773 | OD1 | ASP | A | 98 | -43.837 | -26.292 | 83.050 | 1.00 | 33.28 | O |
| ATOM | 774 | N | TYR | A | 99 | -40.940 | -28.023 | 83.458 | 1.00 | 30.78 | N |
| ATOM | 775 | CA | TYR | A | 99 | -40.738 | -29.329 | 82.815 | 1.00 | 30.04 | C |
| ATOM | 776 | C | TYR | A | 99 | -41.625 | -30.324 | 83.561 | 1.00 | 33.93 | C |
| ATOM | 777 | O | TYR | A | 99 | -41.159 | -31.204 | 84.293 | 1.00 | 38.31 | O |
| ATOM | 778 | CB | TYR | A | 99 | -39.272 | -29.767 | 82.827 | 1.00 | 29.21 | C |
| ATOM | 779 | CG | TYR | A | 99 | -38.929 | -30.840 | 81.794 | 1.00 | 34.85 | C |

| | | | | | | | | | | | |
|---|---|---|---|---|---|---|---|---|---|---|---|
| ATOM | 780 | CD1 | TYR | A | 99 | -39.322 | -30.698 | 80.458 | 1.00 | 35.04 | C |
| ATOM | 781 | CD2 | TYR | A | 99 | -38.133 | -31.940 | 82.127 | 1.00 | 32.30 | C |
| ATOM | 782 | CE1 | TYR | A | 99 | -38.982 | -31.651 | 79.496 | 1.00 | 36.24 | C |
| ATOM | 783 | CE2 | TYR | A | 99 | -37.783 | -32.896 | 81.172 | 1.00 | 28.54 | C |
| ATOM | 784 | CZ | TYR | A | 99 | -38.209 | -32.749 | 79.865 | 1.00 | 35.98 | C |
| ATOM | 785 | OH | TYR | A | 99 | -37.874 | -33.692 | 78.917 | 1.00 | 34.12 | O |
| ATOM | 786 | N | GLY | A | 100 | -42.933 | -30.172 | 83.358 | 1.00 | 28.51 | N |
| ATOM | 787 | CA | GLY | A | 100 | -43.910 | -30.735 | 84.262 | 1.00 | 24.10 | C |
| ATOM | 788 | C | GLY | A | 100 | -44.030 | -29.821 | 85.465 | 1.00 | 27.81 | C |
| ATOM | 789 | O | GLY | A | 100 | -44.871 | -28.922 | 85.491 | 1.00 | 27.49 | O |
| ATOM | 790 | N | ALA | A | 101 | -43.179 | -30.038 | 86.462 | 1.00 | 27.49 | N |
| ATOM | 791 | CA | ALA | A | 101 | -43.007 | -29.155 | 87.602 | 1.00 | 27.47 | C |
| ATOM | 792 | C | ALA | A | 101 | -41.783 | -28.256 | 87.421 | 1.00 | 30.09 | C |
| ATOM | 793 | O | ALA | A | 101 | -41.094 | -28.287 | 86.395 | 1.00 | 27.64 | O |
| ATOM | 794 | CB | ALA | A | 101 | -42.895 | -29.967 | 88.884 | 1.00 | 25.99 | C |
| ATOM | 795 | N | PHE | A | 102 | -41.526 | -27.427 | 88.434 | 1.00 | 23.58 | N |
| ATOM | 796 | CA | PHE | A | 102 | -40.378 | -26.521 | 88.429 | 1.00 | 29.82 | C |
| ATOM | 797 | C | PHE | A | 102 | -39.181 | -27.242 | 89.042 | 1.00 | 28.21 | C |
| ATOM | 798 | O | PHE | A | 102 | -39.007 | -27.255 | 90.268 | 1.00 | 26.65 | O |
| ATOM | 799 | CB | PHE | A | 102 | -40.704 | -25.241 | 89.185 | 1.00 | 24.30 | C |
| ATOM | 800 | CG | PHE | A | 102 | -41.831 | -24.469 | 88.585 | 1.00 | 29.71 | C |
| ATOM | 801 | CD1 | PHE | A | 102 | -41.705 | -23.921 | 87.306 | 1.00 | 27.01 | C |
| ATOM | 802 | CD2 | PHE | A | 102 | -43.012 | -24.265 | 89.297 | 1.00 | 28.29 | C |
| ATOM | 803 | CE1 | PHE | A | 102 | -42.719 | -23.201 | 86.752 | 1.00 | 23.76 | C |
| ATOM | 804 | CE2 | PHE | A | 102 | -44.047 | -23.538 | 88.738 | 1.00 | 27.29 | C |
| ATOM | 805 | CZ | PHE | A | 102 | -43.897 | -23.010 | 87.461 | 1.00 | 27.69 | C |
| ATOM | 806 | N | ASP | A | 103 | -38.333 | -27.818 | 88.179 | 1.00 | 24.44 | N |
| ATOM | 807 | CA | ASP | A | 103 | -37.235 | -28.648 | 88.657 | 1.00 | 28.27 | C |
| ATOM | 808 | C | ASP | A | 103 | -35.943 | -27.887 | 88.873 | 1.00 | 27.03 | C |
| ATOM | 809 | O | ASP | A | 103 | -35.131 | -28.306 | 89.693 | 1.00 | 37.49 | O |
| ATOM | 810 | CB | ASP | A | 103 | -36.989 | -29.838 | 87.721 | 1.00 | 31.73 | C |
| ATOM | 811 | CG | ASP | A | 103 | -36.673 | -29.436 | 86.302 | 1.00 | 39.51 | C |
| ATOM | 812 | OD1 | ASP | A | 103 | -36.346 | -28.251 | 86.038 | 1.00 | 36.08 | O |
| ATOM | 813 | OD2 | ASP | A | 103 | -36.735 | -30.340 | 85.438 | 1.00 | 44.53 | O1- |
| ATOM | 814 | N | ILE | A | 104 | -35.734 | -26.768 | 88.205 | 1.00 | 32.07 | N |
| ATOM | 815 | CA | ILE | A | 104 | -34.562 | -25.951 | 88.461 | 1.00 | 32.48 | C |
| ATOM | 816 | C | ILE | A | 104 | -35.014 | -24.530 | 88.674 | 1.00 | 30.55 | C |
| ATOM | 817 | O | ILE | A | 104 | -35.832 | -24.011 | 87.910 | 1.00 | 32.04 | O |
| ATOM | 818 | CB | ILE | A | 104 | -33.537 | -25.999 | 87.318 | 1.00 | 33.95 | C |
| ATOM | 819 | CG1 | ILE | A | 104 | -33.052 | -27.421 | 87.124 | 1.00 | 31.05 | C |
| ATOM | 820 | CG2 | ILE | A | 104 | -32.357 | -25.103 | 87.642 | 1.00 | 29.90 | C |
| ATOM | 821 | CD1 | ILE | A | 104 | -32.035 | -27.491 | 86.097 | 1.00 | 37.82 | C |
| ATOM | 822 | N | TRP | A | 105 | -34.437 | -23.891 | 89.678 | 1.00 | 30.90 | N |
| ATOM | 823 | CA | TRP | A | 105 | -34.761 | -22.534 | 90.062 | 1.00 | 29.84 | C |
| ATOM | 824 | C | TRP | A | 105 | -33.493 | -21.703 | 90.002 | 1.00 | 35.42 | C |
| ATOM | 825 | O | TRP | A | 105 | -32.384 | -22.226 | 90.145 | 1.00 | 32.10 | O |
| ATOM | 826 | CB | TRP | A | 105 | -35.340 | -22.469 | 91.479 | 1.00 | 30.72 | C |
| ATOM | 827 | CG | TRP | A | 105 | -36.660 | -23.174 | 91.650 | 1.00 | 28.36 | C |
| ATOM | 828 | CD1 | TRP | A | 105 | -36.900 | -24.508 | 91.535 | 1.00 | 26.86 | C |
| ATOM | 829 | CD2 | TRP | A | 105 | -37.906 | -22.570 | 92.029 | 1.00 | 27.83 | C |
| ATOM | 830 | NE1 | TRP | A | 105 | -38.221 | -24.775 | 91.787 | 1.00 | 26.76 | N |
| ATOM | 831 | CE2 | TRP | A | 105 | -38.862 | -23.601 | 92.093 | 1.00 | 27.46 | C |
| ATOM | 832 | CE3 | TRP | A | 105 | -38.306 | -21.246 | 92.305 | 1.00 | 24.64 | C |
| ATOM | 833 | CZ2 | TRP | A | 105 | -40.203 | -23.359 | 92.425 | 1.00 | 26.14 | C |
| ATOM | 834 | CZ3 | TRP | A | 105 | -39.632 | -21.002 | 92.628 | 1.00 | 26.09 | C |
| ATOM | 835 | CH2 | TRP | A | 105 | -40.569 | -22.052 | 92.682 | 1.00 | 27.22 | C |
| ATOM | 836 | N | GLY | A | 106 | -33.675 | -20.409 | 89.739 | 1.00 | 36.95 | N |
| ATOM | 837 | CA | GLY | A | 106 | -32.621 | -19.444 | 89.932 | 1.00 | 30.80 | C |
| ATOM | 838 | C | GLY | A | 106 | -32.355 | -19.129 | 91.400 | 1.00 | 35.16 | C |
| ATOM | 839 | O | GLY | A | 106 | -33.108 | -19.455 | 92.312 | 1.00 | 36.82 | O |
| ATOM | 840 | N | GLN | A | 107 | -31.218 | -18.464 | 91.603 | 1.00 | 47.39 | N |
| ATOM | 841 | CA | GLN | A | 107 | -30.810 | -17.934 | 92.899 | 1.00 | 36.74 | C |
| ATOM | 842 | C | GLN | A | 107 | -31.901 | -17.089 | 93.533 | 1.00 | 39.96 | C |
| ATOM | 843 | O | GLN | A | 107 | -32.042 | -17.063 | 94.766 | 1.00 | 42.16 | O |
| ATOM | 844 | CB | GLN | A | 107 | -29.557 | -17.093 | 92.676 | 1.00 | 42.92 | C |
| ATOM | 845 | CG | GLN | A | 107 | -29.167 | -17.061 | 91.154 | 1.00 | 49.03 | C |
| ATOM | 846 | CD | GLN | A | 107 | -28.508 | -15.762 | 90.696 | 1.00 | 54.29 | C |
| ATOM | 847 | OE1 | GLN | A | 107 | -28.954 | -14.661 | 91.047 | 1.00 | 58.14 | O |
| ATOM | 848 | NE2 | GLN | A | 107 | -27.442 | -15.886 | 89.898 | 1.00 | 50.79 | N |
| ATOM | 849 | N | GLY | A | 108 | -32.681 | -16.410 | 92.707 | 1.00 | 31.86 | N |
| ATOM | 850 | CA | GLY | A | 108 | -33.598 | -15.378 | 93.128 | 1.00 | 27.03 | C |

| | | | | | | | | | | | |
|---|---|---|---|---|---|---|---|---|---|---|---|
| ATOM | 851 | C | GLY | A | 108 | -32.949 | -14.010 | 93.074 | 1.00 | 30.24 | C |
| ATOM | 852 | O | GLY | A | 108 | -31.729 | -13.864 | 93.142 | 1.00 | 37.43 | O |
| ATOM | 853 | N | THR | A | 109 | -33.797 | -12.994 | 92.950 | 1.00 | 25.96 | N |
| ATOM | 854 | CA | THR | A | 109 | -33.387 | -11.598 | 92.963 | 1.00 | 27.32 | C |
| ATOM | 855 | C | THR | A | 109 | -34.249 | -10.910 | 94.000 | 1.00 | 31.41 | C |
| ATOM | 856 | O | THR | A | 109 | -35.475 | -10.935 | 93.890 | 1.00 | 32.07 | O |
| ATOM | 857 | CB | THR | A | 109 | -33.582 | -10.910 | 91.604 | 1.00 | 34.49 | C |
| ATOM | 858 | OG1 | THR | A | 109 | -32.815 | -11.575 | 90.587 | 1.00 | 40.25 | O |
| ATOM | 859 | CG2 | THR | A | 109 | -33.151 | -9.449 | 91.696 | 1.00 | 26.29 | C |
| ATOM | 860 | N | MET | A | 110 | -33.618 | -10.317 | 95.010 | 1.00 | 34.98 | N |
| ATOM | 861 | CA | MET | A | 110 | -34.341 | -9.610 | 96.058 | 1.00 | 32.57 | C |
| ATOM | 862 | C | MET | A | 110 | -34.744 | -8.234 | 95.546 | 1.00 | 36.42 | C |
| ATOM | 863 | O | MET | A | 110 | -33.881 | -7.407 | 95.236 | 1.00 | 38.21 | O |
| ATOM | 864 | CB | MET | A | 110 | -33.471 | -9.495 | 97.301 | 1.00 | 32.17 | C |
| ATOM | 865 | CG | MET | A | 110 | -34.222 | -9.231 | 98.593 | 1.00 | 40.32 | C |
| ATOM | 866 | SD | MET | A | 110 | -33.067 | -9.017 | 99.988 | 1.00 | 67.95 | S |
| ATOM | 867 | CE | MET | A | 110 | -31.758 | -10.224 | 99.627 | 1.00 | 33.01 | C |
| ATOM | 868 | N | VAL | A | 111 | -36.046 | -7.977 | 95.455 | 1.00 | 33.03 | N |
| ATOM | 869 | CA | VAL | A | 111 | -36.549 | -6.745 | 94.860 | 1.00 | 33.46 | C |
| ATOM | 870 | C | VAL | A | 111 | -37.261 | -5.962 | 95.940 | 1.00 | 32.80 | C |
| ATOM | 871 | O | VAL | A | 111 | -38.250 | -6.441 | 96.502 | 1.00 | 34.50 | O |
| ATOM | 872 | CB | VAL | A | 111 | -37.500 | -7.004 | 93.679 | 1.00 | 34.14 | C |
| ATOM | 873 | CG1 | VAL | A | 111 | -38.216 | -5.736 | 93.323 | 1.00 | 25.66 | C |
| ATOM | 874 | CG2 | VAL | A | 111 | -36.748 | -7.559 | 92.470 | 1.00 | 30.59 | C |
| ATOM | 875 | N | THR | A | 112 | -36.777 | -4.756 | 96.211 | 1.00 | 33.30 | N |
| ATOM | 876 | CA | THR | A | 112 | -37.383 | -3.856 | 97.176 | 1.00 | 31.95 | C |
| ATOM | 877 | C | THR | A | 112 | -37.950 | -2.664 | 96.425 | 1.00 | 36.12 | C |
| ATOM | 878 | O | THR | A | 112 | -37.249 | -2.038 | 95.621 | 1.00 | 37.45 | O |
| ATOM | 879 | CB | THR | A | 112 | -36.362 | -3.387 | 98.207 | 1.00 | 31.08 | C |
| ATOM | 880 | OG1 | THR | A | 112 | -35.660 | -4.523 | 98.708 | 1.00 | 33.39 | O |
| ATOM | 881 | CG2 | THR | A | 112 | -37.042 | -2.661 | 99.366 | 1.00 | 29.77 | C |
| ATOM | 882 | N | VAL | A | 113 | -39.210 | -2.344 | 96.684 | 1.00 | 31.43 | N |
| ATOM | 883 | CA | VAL | A | 113 | -39.827 | -1.173 | 96.089 | 1.00 | 36.35 | C |
| ATOM | 884 | C | VAL | A | 113 | -40.215 | -0.226 | 97.216 | 1.00 | 36.75 | C |
| ATOM | 885 | O | VAL | A | 113 | -41.039 | -0.569 | 98.072 | 1.00 | 39.18 | O |
| ATOM | 886 | CB | VAL | A | 113 | -41.043 | -1.550 | 95.234 | 1.00 | 34.86 | C |
| ATOM | 887 | CG1 | VAL | A | 113 | -41.651 | -0.305 | 94.602 | 1.00 | 34.99 | C |
| ATOM | 888 | CG2 | VAL | A | 113 | -40.631 | -2.533 | 94.188 | 1.00 | 34.01 | C |
| ATOM | 889 | N | SER | A | 114 | -39.653 | 0.974 | 97.192 | 1.00 | 36.38 | N |
| ATOM | 890 | CA | SER | A | 114 | -39.797 | 1.848 | 98.340 | 1.00 | 36.28 | C |
| ATOM | 891 | C | SER | A | 114 | -39.395 | 3.273 | 97.977 | 1.00 | 40.12 | C |
| ATOM | 892 | O | SER | A | 114 | -38.525 | 3.507 | 97.123 | 1.00 | 40.70 | O |
| ATOM | 893 | CB | SER | A | 114 | -38.954 | 1.341 | 99.507 | 1.00 | 31.28 | C |
| ATOM | 894 | OG | SER | A | 114 | -38.863 | 2.336 | 100.505 | 1.00 | 39.09 | O |
| ATOM | 895 | N | SER | A | 115 | -40.017 | 4.217 | 98.672 | 1.00 | 38.69 | N |
| ATOM | 896 | CA | SER | A | 115 | -39.621 | 5.613 | 98.576 | 1.00 | 42.10 | C |
| ATOM | 897 | C | SER | A | 115 | -38.296 | 5.892 | 99.267 | 1.00 | 46.74 | C |
| ATOM | 898 | O | SER | A | 115 | -37.675 | 6.920 | 98.973 | 1.00 | 42.92 | O |
| ATOM | 899 | CB | SER | A | 115 | -40.717 | 6.490 | 99.162 | 1.00 | 39.28 | C |
| ATOM | 900 | OG | SER | A | 115 | -41.885 | 6.392 | 98.349 | 1.00 | 47.59 | O |
| ATOM | 901 | N | ALA | A | 116 | -37.853 | 5.008 | 100.168 | 1.00 | 39.54 | N |
| ATOM | 902 | CA | ALA | A | 116 | -36.656 | 5.257 | 100.954 | 1.00 | 35.81 | C |
| ATOM | 903 | C | ALA | A | 116 | -35.417 | 5.270 | 100.067 | 1.00 | 37.57 | C |
| ATOM | 904 | O | ALA | A | 116 | -35.411 | 4.730 | 98.963 | 1.00 | 38.65 | O |
| ATOM | 905 | CB | ALA | A | 116 | -36.507 | 4.199 | 102.035 | 1.00 | 35.27 | C |
| ATOM | 906 | N | SER | A | 117 | -34.347 | 5.880 | 100.571 | 1.00 | 38.00 | N |
| ATOM | 907 | CA | SER | A | 117 | -33.079 | 5.924 | 99.855 | 1.00 | 40.96 | C |
| ATOM | 908 | C | SER | A | 117 | -32.040 | 5.082 | 100.572 | 1.00 | 39.82 | C |
| ATOM | 909 | O | SER | A | 117 | -32.108 | 4.893 | 101.790 | 1.00 | 40.84 | O |
| ATOM | 910 | CB | SER | A | 117 | -32.561 | 7.352 | 99.715 | 1.00 | 38.33 | C |
| ATOM | 911 | OG | SER | A | 117 | -33.448 | 8.084 | 98.896 | 1.00 | 52.15 | O |
| ATOM | 912 | N | THR | A | 118 | -31.078 | 4.576 | 99.803 | 1.00 | 34.77 | N |
| ATOM | 913 | CA | THR | A | 118 | -30.059 | 3.709 | 100.378 | 1.00 | 35.77 | C |
| ATOM | 914 | C | THR | A | 118 | -29.387 | 4.392 | 101.560 | 1.00 | 40.75 | C |
| ATOM | 915 | O | THR | A | 118 | -29.127 | 5.595 | 101.535 | 1.00 | 45.66 | O |
| ATOM | 916 | CB | THR | A | 118 | -29.030 | 3.346 | 99.320 | 1.00 | 33.58 | C |
| ATOM | 917 | OG1 | THR | A | 118 | -29.662 | 2.534 | 98.320 | 1.00 | 42.35 | O |
| ATOM | 918 | CG2 | THR | A | 118 | -27.854 | 2.607 | 99.941 | 1.00 | 35.40 | C |
| ATOM | 919 | N | LYS | A | 119 | -29.152 | 3.629 | 102.621 | 1.00 | 37.56 | N |
| ATOM | 920 | CA | LYS | A | 119 | -28.635 | 4.215 | 103.848 | 1.00 | 36.23 | C |
| ATOM | 921 | C | LYS | A | 119 | -28.000 | 3.126 | 104.697 | 1.00 | 36.78 | C |

| | | | | | | | | | | | |
|---|---|---|---|---|---|---|---|---|---|---|---|
| ATOM | 922 | O | LYS | A | 119 | -28.629 | 2.100 | 104.959 | 1.00 | 31.04 | O |
| ATOM | 923 | CB | LYS | A | 119 | -29.742 | 4.904 | 104.631 | 1.00 | 36.08 | C |
| ATOM | 924 | CG | LYS | A | 119 | -29.224 | 5.571 | 105.867 | 1.00 | 32.45 | C |
| ATOM | 925 | CD | LYS | A | 119 | -30.331 | 6.005 | 106.761 | 1.00 | 36.11 | C |
| ATOM | 926 | CE | LYS | A | 119 | -29.719 | 6.656 | 107.950 | 1.00 | 41.55 | C |
| ATOM | 927 | NZ | LYS | A | 119 | -28.719 | 5.693 | 108.485 | 1.00 | 40.27 | N1+ |
| ATOM | 928 | N | GLY | A | 120 | -26.761 | 3.351 | 105.115 | 1.00 | 32.34 | N |
| ATOM | 929 | CA | GLY | A | 120 | -26.058 | 2.407 | 105.937 | 1.00 | 32.74 | C |
| ATOM | 930 | C | GLY | A | 120 | -26.563 | 2.417 | 107.364 | 1.00 | 33.41 | C |
| ATOM | 931 | O | GLY | A | 120 | -27.264 | 3.331 | 107.801 | 1.00 | 34.70 | O |
| ATOM | 932 | N | PRO | A | 121 | -26.210 | 1.393 | 108.120 | 1.00 | 31.38 | N |
| ATOM | 933 | CA | PRO | A | 121 | -26.705 | 1.283 | 109.493 | 1.00 | 36.27 | C |
| ATOM | 934 | C | PRO | A | 121 | -25.863 | 2.075 | 110.478 | 1.00 | 29.36 | C |
| ATOM | 935 | O | PRO | A | 121 | -24.723 | 2.442 | 110.213 | 1.00 | 28.70 | O |
| ATOM | 936 | CB | PRO | A | 121 | -26.592 | -0.217 | 109.784 | 1.00 | 32.75 | C |
| ATOM | 937 | CG | PRO | A | 121 | -25.450 | -0.653 | 108.950 | 1.00 | 31.60 | C |
| ATOM | 938 | CD | PRO | A | 121 | -25.485 | 0.190 | 107.692 | 1.00 | 31.22 | C |
| ATOM | 939 | N | SER | A | 122 | -26.481 | 2.365 | 111.614 | 1.00 | 28.35 | N |
| ATOM | 940 | CA | SER | A | 122 | -25.790 | 2.748 | 112.834 | 1.00 | 27.65 | C |
| ATOM | 941 | C | SER | A | 122 | -25.766 | 1.529 | 113.743 | 1.00 | 28.89 | C |
| ATOM | 942 | O | SER | A | 122 | -26.780 | 0.843 | 113.874 | 1.00 | 33.72 | O |
| ATOM | 943 | CB | SER | A | 122 | -26.490 | 3.915 | 113.528 | 1.00 | 27.60 | C |
| ATOM | 944 | OG | SER | A | 122 | -26.537 | 5.035 | 112.665 | 1.00 | 32.84 | O |
| ATOM | 945 | N | VAL | A | 123 | -24.615 | 1.239 | 114.341 | 1.00 | 30.13 | N |
| ATOM | 946 | CA | VAL | A | 123 | -24.439 | 0.062 | 115.188 | 1.00 | 29.26 | C |
| ATOM | 947 | C | VAL | A | 123 | -24.266 | 0.513 | 116.638 | 1.00 | 28.50 | C |
| ATOM | 948 | O | VAL | A | 123 | -23.290 | 1.188 | 116.982 | 1.00 | 36.71 | O |
| ATOM | 949 | CB | VAL | A | 123 | -23.257 | -0.800 | 114.713 | 1.00 | 29.30 | C |
| ATOM | 950 | CG1 | VAL | A | 123 | -23.161 | -2.079 | 115.535 | 1.00 | 32.16 | C |
| ATOM | 951 | CG2 | VAL | A | 123 | -23.421 | -1.143 | 113.243 | 1.00 | 29.73 | C |
| ATOM | 952 | N | PHE | A | 124 | -25.204 | 0.154 | 117.477 | 1.00 | 29.11 | N |
| ATOM | 953 | CA | PHE | A | 124 | -25.117 | 0.453 | 118.892 | 1.00 | 30.43 | C |
| ATOM | 954 | C | PHE | A | 124 | -24.914 | -0.818 | 119.710 | 1.00 | 30.05 | C |
| ATOM | 955 | O | PHE | A | 124 | -25.432 | -1.871 | 119.341 | 1.00 | 28.55 | O |
| ATOM | 956 | CB | PHE | A | 124 | -26.387 | 1.149 | 119.375 | 1.00 | 29.02 | C |
| ATOM | 957 | CG | PHE | A | 124 | -26.765 | 2.328 | 118.559 | 1.00 | 31.89 | C |
| ATOM | 958 | CD1 | PHE | A | 124 | -25.952 | 3.448 | 118.525 | 1.00 | 37.36 | C |
| ATOM | 959 | CD2 | PHE | A | 124 | -27.948 | 2.345 | 117.848 | 1.00 | 33.36 | C |
| ATOM | 960 | CE1 | PHE | A | 124 | -26.309 | 4.550 | 117.784 | 1.00 | 33.03 | C |
| ATOM | 961 | CE2 | PHE | A | 124 | -28.307 | 3.451 | 117.112 | 1.00 | 32.67 | C |
| ATOM | 962 | CZ | PHE | A | 124 | -27.486 | 4.548 | 117.085 | 1.00 | 31.27 | C |
| ATOM | 963 | N | PRO | A | 125 | -24.212 | -0.744 | 120.845 | 1.00 | 34.75 | N |
| ATOM | 964 | CA | PRO | A | 125 | -23.997 | -1.942 | 121.663 | 1.00 | 33.50 | C |
| ATOM | 965 | C | PRO | A | 125 | -25.209 | -2.306 | 122.514 | 1.00 | 33.10 | C |
| ATOM | 966 | O | PRO | A | 125 | -25.956 | -1.452 | 122.991 | 1.00 | 31.28 | O |
| ATOM | 967 | CB | PRO | A | 125 | -22.808 | -1.549 | 122.546 | 1.00 | 27.55 | C |
| ATOM | 968 | CG | PRO | A | 125 | -22.913 | -0.105 | 122.656 | 1.00 | 24.38 | C |
| ATOM | 969 | CD | PRO | A | 125 | -23.459 | 0.407 | 121.378 | 1.00 | 30.67 | C |
| ATOM | 970 | N | LEU | A | 126 | -25.395 | -3.609 | 122.688 | 1.00 | 29.05 | N |
| ATOM | 971 | CA | LEU | A | 126 | -26.264 | -4.145 | 123.724 | 1.00 | 31.66 | C |
| ATOM | 972 | C | LEU | A | 126 | -25.315 | -4.627 | 124.801 | 1.00 | 33.65 | C |
| ATOM | 973 | O | LEU | A | 126 | -24.728 | -5.701 | 124.688 | 1.00 | 37.65 | O |
| ATOM | 974 | CB | LEU | A | 126 | -27.167 | -5.249 | 123.195 | 1.00 | 30.81 | C |
| ATOM | 975 | CG | LEU | A | 126 | -27.997 | -4.672 | 122.052 | 1.00 | 29.82 | C |
| ATOM | 976 | CD1 | LEU | A | 126 | -28.827 | -5.730 | 121.357 | 1.00 | 26.48 | C |
| ATOM | 977 | CD2 | LEU | A | 126 | -28.860 | -3.574 | 122.603 | 1.00 | 30.33 | C |
| ATOM | 978 | N | ALA | A | 127 | -25.143 | -3.805 | 125.835 | 1.00 | 38.61 | N |
| ATOM | 979 | CA | ALA | A | 127 | -24.053 | -4.011 | 126.768 | 1.00 | 36.09 | C |
| ATOM | 980 | C | ALA | A | 127 | -24.399 | -5.128 | 127.738 | 1.00 | 40.57 | C |
| ATOM | 981 | O | ALA | A | 127 | -25.510 | -5.137 | 128.289 | 1.00 | 36.21 | O |
| ATOM | 982 | CB | ALA | A | 127 | -23.763 | -2.733 | 127.529 | 1.00 | 35.02 | C |
| ATOM | 983 | N | PRO | A | 128 | -23.478 | -6.062 | 127.986 | 1.00 | 38.34 | N |
| ATOM | 984 | CA | PRO | A | 128 | -23.759 | -7.137 | 128.941 | 1.00 | 45.51 | C |
| ATOM | 985 | C | PRO | A | 128 | -24.091 | -6.569 | 130.309 | 1.00 | 56.56 | C |
| ATOM | 986 | O | PRO | A | 128 | -23.360 | -5.736 | 130.865 | 1.00 | 52.82 | O |
| ATOM | 987 | CB | PRO | A | 128 | -22.463 | -7.953 | 128.968 | 1.00 | 43.58 | C |
| ATOM | 988 | CG | PRO | A | 128 | -21.433 | -7.043 | 128.440 | 1.00 | 45.25 | C |
| ATOM | 989 | CD | PRO | A | 128 | -22.124 | -6.162 | 127.441 | 1.00 | 39.79 | C |
| ATOM | 990 | N | SER | A | 129 | -25.212 | -7.049 | 130.837 | 1.00 | 66.36 | N |
| ATOM | 991 | CA | SER | A | 129 | -25.759 | -6.677 | 132.128 | 1.00 | 72.65 | C |
| ATOM | 992 | C | SER | A | 129 | -24.673 | -6.810 | 133.195 | 1.00 | 76.84 | C |

| | | | | | | | | | | | | |
|---|---|---|---|---|---|---|---|---|---|---|---|---|
| ATOM | 993 | O | SER | A | 129 | -24.175 | -7.914 | 133.453 | 1.00 | 78.41 | O |
| ATOM | 994 | CB | SER | A | 129 | -26.983 | -7.568 | 132.406 | 1.00 | 69.58 | C |
| ATOM | 995 | OG | SER | A | 129 | -27.910 | -7.001 | 133.309 | 1.00 | 70.69 | O |
| ATOM | 996 | N | SER | A | 130 | -24.252 | -5.681 | 133.776 | 1.00 | 77.98 | N |
| ATOM | 997 | CA | SER | A | 130 | -23.377 | -5.731 | 134.943 | 1.00 | 85.61 | C |
| ATOM | 998 | C | SER | A | 130 | -23.994 | -6.604 | 136.037 | 1.00 | 90.91 | C |
| ATOM | 999 | O | SER | A | 130 | -23.273 | -7.223 | 136.835 | 1.00 | 87.54 | O |
| ATOM | 1000 | CB | SER | A | 130 | -23.089 | -4.307 | 135.444 | 1.00 | 90.00 | C |
| ATOM | 1001 | OG | SER | A | 130 | -24.274 | -3.529 | 135.594 | 1.00 | 85.47 | O |
| ATOM | 1002 | N | LYS | A | 131 | -25.331 | -6.708 | 136.043 | 1.00 | 95.09 | N |
| ATOM | 1003 | CA | LYS | A | 131 | -26.101 | -7.524 | 136.974 | 1.00 | 92.61 | C |
| ATOM | 1004 | C | LYS | A | 131 | -26.337 | -8.936 | 136.436 | 1.00 | 92.99 | C |
| ATOM | 1005 | O | LYS | A | 131 | -27.391 | -9.540 | 136.695 | 1.00 | 88.38 | O |
| ATOM | 1006 | CB | LYS | A | 131 | -27.436 | -6.839 | 137.284 | 1.00 | 84.94 | C |
| ATOM | 1007 | CG | LYS | A | 131 | -27.308 | -5.523 | 138.053 | 1.00 | 86.39 | C |
| ATOM | 1008 | CD | LYS | A | 131 | -28.673 | -4.977 | 138.437 | 1.00 | 85.07 | C |
| ATOM | 1009 | CE | LYS | A | 131 | -28.583 | -4.070 | 139.646 | 1.00 | 74.74 | C |
| ATOM | 1010 | NZ | LYS | A | 131 | -29.939 | -3.594 | 140.022 | 1.00 | 70.33 | N1+ |
| ATOM | 1011 | N | SER | A | 132 | -25.386 | -9.453 | 135.655 | 1.00 | 91.67 | N |
| ATOM | 1012 | CA | SER | A | 132 | -25.423 | -10.837 | 135.210 | 1.00 | 89.98 | C |
| ATOM | 1013 | C | SER | A | 132 | -25.152 | -11.744 | 136.398 | 1.00 | 95.97 | C |
| ATOM | 1014 | O | SER | A | 132 | -24.296 | -11.439 | 137.237 | 1.00 | 97.89 | O |
| ATOM | 1015 | CB | SER | A | 132 | -24.366 | -11.076 | 134.130 | 1.00 | 80.08 | C |
| ATOM | 1016 | OG | SER | A | 132 | -24.779 | -10.567 | 132.876 | 1.00 | 75.09 | O |
| ATOM | 1017 | N | THR | A | 133 | -25.903 | -12.846 | 136.495 | 1.00 | 96.88 | N |
| ATOM | 1018 | CA | THR | A | 133 | -25.589 | -13.828 | 137.529 | 1.00 | 94.98 | C |
| ATOM | 1019 | C | THR | A | 133 | -24.133 | -14.276 | 137.346 | 1.00 | 90.31 | C |
| ATOM | 1020 | O | THR | A | 133 | -23.836 | -15.107 | 136.480 | 1.00 | 81.64 | O |
| ATOM | 1021 | CB | THR | A | 133 | -26.595 | -15.001 | 137.481 | 1.00 | 90.13 | C |
| ATOM | 1022 | OG1 | THR | A | 133 | -26.943 | -15.313 | 136.117 | 1.00 | 86.99 | O |
| ATOM | 1023 | CG2 | THR | A | 133 | -27.872 | -14.664 | 138.271 | 1.00 | 77.20 | C |
| ATOM | 1024 | N | SER | A | 134 | -23.219 | -13.722 | 138.160 | 1.00 | 93.80 | N |
| ATOM | 1025 | CA | SER | A | 134 | -21.784 | -13.900 | 137.935 | 1.00 | 90.34 | C |
| ATOM | 1026 | C | SER | A | 134 | -21.364 | -15.323 | 138.275 | 1.00 | 87.03 | C |
| ATOM | 1027 | O | SER | A | 134 | -21.597 | -15.802 | 139.389 | 1.00 | 88.65 | O |
| ATOM | 1028 | CB | SER | A | 134 | -20.971 | -12.892 | 138.748 | 1.00 | 81.79 | C |
| ATOM | 1029 | OG | SER | A | 134 | -19.872 | -12.426 | 137.986 | 1.00 | 69.79 | O |
| ATOM | 1030 | N | GLY | A | 135 | -20.701 | -15.981 | 137.329 | 1.00 | 83.14 | N |
| ATOM | 1031 | CA | GLY | A | 135 | -20.552 | -17.418 | 137.366 | 1.00 | 79.97 | C |
| ATOM | 1032 | C | GLY | A | 135 | -21.634 | -18.150 | 136.607 | 1.00 | 75.17 | C |
| ATOM | 1033 | O | GLY | A | 135 | -21.672 | -19.387 | 136.643 | 1.00 | 70.13 | O |
| ATOM | 1034 | N | GLY | A | 136 | -22.531 | -17.411 | 135.954 | 1.00 | 73.84 | N |
| ATOM | 1035 | CA | GLY | A | 136 | -23.577 | -17.924 | 135.095 | 1.00 | 61.25 | C |
| ATOM | 1036 | C | GLY | A | 136 | -23.388 | -17.420 | 133.676 | 1.00 | 63.22 | C |
| ATOM | 1037 | O | GLY | A | 136 | -22.265 | -17.397 | 133.152 | 1.00 | 56.06 | O |
| ATOM | 1038 | N | THR | A | 137 | -24.476 | -16.996 | 133.040 | 1.00 | 59.59 | N |
| ATOM | 1039 | CA | THR | A | 137 | -24.446 | -16.674 | 131.626 | 1.00 | 48.48 | C |
| ATOM | 1040 | C | THR | A | 137 | -24.845 | -15.227 | 131.387 | 1.00 | 51.06 | C |
| ATOM | 1041 | O | THR | A | 137 | -25.789 | -14.712 | 132.002 | 1.00 | 56.26 | O |
| ATOM | 1042 | CB | THR | A | 137 | -25.342 | -17.624 | 130.849 | 1.00 | 47.89 | C |
| ATOM | 1043 | OG1 | THR | A | 137 | -24.756 | -18.935 | 130.893 | 1.00 | 52.57 | O |
| ATOM | 1044 | CG2 | THR | A | 137 | -25.461 | -17.182 | 129.398 | 1.00 | 39.41 | C |
| ATOM | 1045 | N | ALA | A | 138 | -24.088 | -14.574 | 130.510 | 1.00 | 44.74 | N |
| ATOM | 1046 | CA | ALA | A | 138 | -24.313 | -13.195 | 130.117 | 1.00 | 41.78 | C |
| ATOM | 1047 | C | ALA | A | 138 | -24.782 | -13.142 | 128.670 | 1.00 | 43.49 | C |
| ATOM | 1048 | O | ALA | A | 138 | -24.337 | -13.933 | 127.833 | 1.00 | 41.75 | O |
| ATOM | 1049 | CB | ALA | A | 138 | -23.036 | -12.369 | 130.273 | 1.00 | 44.03 | C |
| ATOM | 1050 | N | ALA | A | 139 | -25.689 | -12.215 | 128.384 | 1.00 | 43.46 | N |
| ATOM | 1051 | CA | ALA | A | 139 | -26.067 | -11.872 | 127.022 | 1.00 | 39.95 | C |
| ATOM | 1052 | C | ALA | A | 139 | -25.455 | -10.522 | 126.665 | 1.00 | 35.35 | C |
| ATOM | 1053 | O | ALA | A | 139 | -25.393 | -9.618 | 127.504 | 1.00 | 31.47 | O |
| ATOM | 1054 | CB | ALA | A | 139 | -27.593 | -11.827 | 126.865 | 1.00 | 27.83 | C |
| ATOM | 1055 | N | LEU | A | 140 | -25.005 | -10.399 | 125.419 | 1.00 | 30.62 | N |
| ATOM | 1056 | CA | LEU | A | 140 | -24.563 | -9.129 | 124.863 | 1.00 | 32.63 | C |
| ATOM | 1057 | C | LEU | A | 140 | -24.915 | -9.129 | 123.382 | 1.00 | 32.74 | C |
| ATOM | 1058 | O | LEU | A | 140 | -25.288 | -10.161 | 122.824 | 1.00 | 33.31 | O |
| ATOM | 1059 | CB | LEU | A | 140 | -23.067 | -8.925 | 125.098 | 1.00 | 36.31 | C |
| ATOM | 1060 | CG | LEU | A | 140 | -22.182 | -10.012 | 124.493 | 1.00 | 33.45 | C |
| ATOM | 1061 | CD1 | LEU | A | 140 | -21.538 | -9.500 | 123.223 | 1.00 | 31.90 | C |
| ATOM | 1062 | CD2 | LEU | A | 140 | -21.146 | -10.511 | 125.497 | 1.00 | 33.92 | C |
| ATOM | 1063 | N | GLY | A | 141 | -24.839 | -7.966 | 122.745 | 1.00 | 31.54 | N |

| | | | | | | | | | | | |
|---|---|---|---|---|---|---|---|---|---|---|---|
| ATOM | 1064 | CA | GLY | A | 141 | -25.151 | -7.940 | 121.332 | 1.00 | 28.33 | C |
| ATOM | 1065 | C | GLY | A | 141 | -24.948 | -6.589 | 120.690 | 1.00 | 27.25 | C |
| ATOM | 1066 | O | GLY | A | 141 | -24.461 | -5.640 | 121.304 | 1.00 | 31.88 | O |
| ATOM | 1067 | N | CYS | A | 142 | -25.353 | -6.529 | 119.428 | 1.00 | 25.30 | N |
| ATOM | 1068 | CA | CYS | A | 142 | -25.336 | -5.319 | 118.623 | 1.00 | 28.40 | C |
| ATOM | 1069 | C | CYS | A | 142 | -26.729 | -5.018 | 118.105 | 1.00 | 27.36 | C |
| ATOM | 1070 | O | CYS | A | 142 | -27.484 | -5.930 | 117.757 | 1.00 | 27.52 | O |
| ATOM | 1071 | CB | CYS | A | 142 | -24.402 | -5.447 | 117.435 | 1.00 | 30.69 | C |
| ATOM | 1072 | SG | CYS | A | 142 | -22.691 | -5.228 | 117.879 | 1.00 | 51.18 | S |
| ATOM | 1073 | N | LEU | A | 143 | -27.056 | -3.735 | 118.049 | 1.00 | 27.56 | N |
| ATOM | 1074 | CA | LEU | A | 143 | -28.293 | -3.248 | 117.460 | 1.00 | 27.81 | C |
| ATOM | 1075 | C | LEU | A | 143 | -27.931 | -2.565 | 116.145 | 1.00 | 27.63 | C |
| ATOM | 1076 | O | LEU | A | 143 | -27.155 | -1.613 | 116.136 | 1.00 | 31.79 | O |
| ATOM | 1077 | CB | LEU | A | 143 | -28.985 | -2.274 | 118.406 | 1.00 | 31.36 | C |
| ATOM | 1078 | CG | LEU | A | 143 | -30.346 | -1.766 | 117.963 | 1.00 | 31.23 | C |
| ATOM | 1079 | CD1 | LEU | A | 143 | -31.250 | -2.954 | 117.862 | 1.00 | 28.10 | C |
| ATOM | 1080 | CD2 | LEU | A | 143 | -30.899 | -0.706 | 118.886 | 1.00 | 32.28 | C |
| ATOM | 1081 | N | VAL | A | 144 | -28.436 | -3.083 | 115.036 | 1.00 | 30.74 | N |
| ATOM | 1082 | CA | VAL | A | 144 | -28.101 | -2.590 | 113.702 | 1.00 | 27.82 | C |
| ATOM | 1083 | C | VAL | A | 144 | -29.311 | -1.800 | 113.216 | 1.00 | 30.66 | C |
| ATOM | 1084 | O | VAL | A | 144 | -30.289 | -2.381 | 112.744 | 1.00 | 30.67 | O |
| ATOM | 1085 | CB | VAL | A | 144 | -27.732 | -3.744 | 112.769 | 1.00 | 28.14 | C |
| ATOM | 1086 | CG1 | VAL | A | 144 | -27.347 | -3.252 | 111.375 | 1.00 | 23.30 | C |
| ATOM | 1087 | CG2 | VAL | A | 144 | -26.591 | -4.541 | 113.411 | 1.00 | 25.34 | C |
| ATOM | 1088 | N | LYS | A | 145 | -29.269 | -0.470 | 113.374 | 1.00 | 33.19 | N |
| ATOM | 1089 | CA | LYS | A | 145 | -30.446 | 0.383 | 113.245 | 1.00 | 31.94 | C |
| ATOM | 1090 | C | LYS | A | 145 | -30.392 | 1.292 | 112.020 | 1.00 | 34.55 | C |
| ATOM | 1091 | O | LYS | A | 145 | -29.322 | 1.758 | 111.612 | 1.00 | 32.70 | O |
| ATOM | 1092 | CB | LYS | A | 145 | -30.637 | 1.229 | 114.504 | 1.00 | 34.83 | C |
| ATOM | 1093 | CG | LYS | A | 145 | -32.055 | 1.752 | 114.654 | 1.00 | 39.24 | C |
| ATOM | 1094 | CD | LYS | A | 145 | -32.309 | 2.302 | 116.035 | 1.00 | 37.76 | C |
| ATOM | 1095 | CE | LYS | A | 145 | -33.802 | 2.550 | 116.282 | 1.00 | 47.55 | C |
| ATOM | 1096 | NZ | LYS | A | 145 | -34.483 | 3.453 | 115.296 | 1.00 | 48.57 | N1+ |
| ATOM | 1097 | N | ASP | A | 146 | -31.564 | 1.494 | 111.414 | 1.00 | 35.65 | N |
| ATOM | 1098 | CA | ASP | A | 146 | -31.815 | 2.517 | 110.399 | 1.00 | 31.21 | C |
| ATOM | 1099 | C | ASP | A | 146 | -30.985 | 2.308 | 109.134 | 1.00 | 34.08 | C |
| ATOM | 1100 | O | ASP | A | 146 | -30.204 | 3.170 | 108.721 | 1.00 | 35.04 | O |
| ATOM | 1101 | CB | ASP | A | 146 | -31.568 | 3.919 | 110.960 | 1.00 | 31.93 | C |
| ATOM | 1102 | CG | ASP | A | 146 | -32.501 | 4.276 | 112.097 | 1.00 | 39.35 | C |
| ATOM | 1103 | OD1 | ASP | A | 146 | -33.621 | 3.731 | 112.154 | 1.00 | 37.78 | O |
| ATOM | 1104 | OD2 | ASP | A | 146 | -32.150 | 5.184 | 112.878 | 1.00 | 48.93 | O1- |
| ATOM | 1105 | N | TYR | A | 147 | -31.184 | 1.169 | 108.490 | 1.00 | 35.70 | N |
| ATOM | 1106 | CA | TYR | A | 147 | -30.527 | 0.965 | 107.205 | 1.00 | 32.13 | C |
| ATOM | 1107 | C | TYR | A | 147 | -31.548 | 0.662 | 106.127 | 1.00 | 30.25 | C |
| ATOM | 1108 | O | TYR | A | 147 | -32.711 | 0.385 | 106.401 | 1.00 | 34.62 | O |
| ATOM | 1109 | CB | TYR | A | 147 | -29.480 | -0.146 | 107.270 | 1.00 | 29.22 | C |
| ATOM | 1110 | CG | TYR | A | 147 | -30.026 | -1.497 | 107.584 | 1.00 | 31.09 | C |
| ATOM | 1111 | CD1 | TYR | A | 147 | -30.476 | -2.344 | 106.572 | 1.00 | 34.27 | C |
| ATOM | 1112 | CD2 | TYR | A | 147 | -30.068 | -1.953 | 108.896 | 1.00 | 31.93 | C |
| ATOM | 1113 | CE1 | TYR | A | 147 | -30.965 | -3.615 | 106.863 | 1.00 | 33.60 | C |
| ATOM | 1114 | CE2 | TYR | A | 147 | -30.547 | -3.216 | 109.201 | 1.00 | 34.30 | C |
| ATOM | 1115 | CZ | TYR | A | 147 | -30.998 | -4.039 | 108.186 | 1.00 | 35.75 | C |
| ATOM | 1116 | OH | TYR | A | 147 | -31.474 | -5.283 | 108.511 | 1.00 | 33.90 | O |
| ATOM | 1117 | N | PHE | A | 148 | -31.087 | 0.720 | 104.889 | 1.00 | 36.14 | N |
| ATOM | 1118 | CA | PHE | A | 148 | -31.931 | 0.444 | 103.740 | 1.00 | 36.37 | C |
| ATOM | 1119 | C | PHE | A | 148 | -31.063 | 0.353 | 102.506 | 1.00 | 35.17 | C |
| ATOM | 1120 | O | PHE | A | 148 | -30.171 | 1.168 | 102.324 | 1.00 | 40.37 | O |
| ATOM | 1121 | CB | PHE | A | 148 | -32.994 | 1.533 | 103.573 | 1.00 | 35.29 | C |
| ATOM | 1122 | CG | PHE | A | 148 | -33.914 | 1.300 | 102.429 | 1.00 | 38.94 | C |
| ATOM | 1123 | CD2 | PHE | A | 148 | -33.582 | 1.733 | 101.154 | 1.00 | 37.28 | C |
| ATOM | 1124 | CD1 | PHE | A | 148 | -35.116 | 0.633 | 102.619 | 1.00 | 43.66 | C |
| ATOM | 1125 | CE2 | PHE | A | 148 | -34.431 | 1.509 | 100.090 | 1.00 | 40.33 | C |
| ATOM | 1126 | CE1 | PHE | A | 148 | -35.980 | 0.405 | 101.558 | 1.00 | 40.99 | C |
| ATOM | 1127 | CZ | PHE | A | 148 | -35.632 | 0.843 | 100.288 | 1.00 | 41.29 | C |
| ATOM | 1128 | N | PRO | A | 149 | -31.317 | -0.641 | 101.646 | 1.00 | 38.61 | N |
| ATOM | 1129 | CA | PRO | A | 149 | -32.320 | -1.693 | 101.818 | 1.00 | 36.40 | C |
| ATOM | 1130 | C | PRO | A | 149 | -31.736 | -2.913 | 102.534 | 1.00 | 32.84 | C |
| ATOM | 1131 | O | PRO | A | 149 | -30.607 | -2.867 | 103.010 | 1.00 | 32.70 | O |
| ATOM | 1132 | CB | PRO | A | 149 | -32.679 | -2.036 | 100.378 | 1.00 | 36.43 | C |
| ATOM | 1133 | CG | PRO | A | 149 | -31.331 | -1.921 | 99.683 | 1.00 | 33.06 | C |
| ATOM | 1134 | CD | PRO | A | 149 | -30.649 | -0.743 | 100.332 | 1.00 | 34.54 | C |

| | | | | | | | | | | | |
|---|---|---|---|---|---|---|---|---|---|---|---|
| ATOM | 1135 | N | GLU | A | 150 | -32.499 | -3.995 | 102.601 | 1.00 | 33.19 | N |
| ATOM | 1136 | CA | GLU | A | 150 | -31.952 | -5.302 | 102.970 | 1.00 | 32.31 | C |
| ATOM | 1137 | C | GLU | A | 150 | -30.980 | -5.788 | 101.891 | 1.00 | 29.80 | C |
| ATOM | 1138 | O | GLU | A | 150 | -31.160 | -5.456 | 100.713 | 1.00 | 29.74 | O |
| ATOM | 1139 | CB | GLU | A | 150 | -33.087 | -6.301 | 103.166 | 1.00 | 28.58 | C |
| ATOM | 1140 | CG | GLU | A | 150 | -33.977 | -6.000 | 104.360 | 1.00 | 29.38 | C |
| ATOM | 1141 | CD | GLU | A | 150 | -33.563 | -6.767 | 105.602 | 1.00 | 39.48 | C |
| ATOM | 1142 | OE1 | GLU | A | 150 | -34.378 | -7.590 | 106.099 | 1.00 | 41.15 | O |
| ATOM | 1143 | OE2 | GLU | A | 150 | -32.413 | -6.566 | 106.065 | 1.00 | 41.49 | O1- |
| ATOM | 1144 | N | PRO | A | 151 | -29.992 | -6.630 | 102.263 | 1.00 | 31.91 | N |
| ATOM | 1145 | CA | PRO | A | 151 | -29.735 | -7.230 | 103.581 | 1.00 | 31.76 | C |
| ATOM | 1146 | C | PRO | A | 151 | -28.502 | -6.701 | 104.317 | 1.00 | 35.19 | C |
| ATOM | 1147 | O | PRO | A | 151 | -27.684 | -5.998 | 103.713 | 1.00 | 34.57 | O |
| ATOM | 1148 | CB | PRO | A | 151 | -29.510 | -8.690 | 103.223 | 1.00 | 23.34 | C |
| ATOM | 1149 | CG | PRO | A | 151 | -28.788 | -8.605 | 101.939 | 1.00 | 20.46 | C |
| ATOM | 1150 | CD | PRO | A | 151 | -29.218 | -7.334 | 101.225 | 1.00 | 25.12 | C |
| ATOM | 1151 | N | VAL | A | 152 | -28.379 | -7.046 | 105.602 | 1.00 | 30.30 | N |
| ATOM | 1152 | CA | VAL | A | 152 | -27.128 | -6.911 | 106.335 | 1.00 | 29.66 | C |
| ATOM | 1153 | C | VAL | A | 152 | -26.726 | -8.297 | 106.815 | 1.00 | 33.28 | C |
| ATOM | 1154 | O | VAL | A | 152 | -27.580 | -9.147 | 107.078 | 1.00 | 31.82 | O |
| ATOM | 1155 | CB | VAL | A | 152 | -27.221 | -5.959 | 107.550 | 1.00 | 31.08 | C |
| ATOM | 1156 | CG1 | VAL | A | 152 | -27.344 | -4.523 | 107.116 | 1.00 | 36.88 | C |
| ATOM | 1157 | CG2 | VAL | A | 152 | -28.382 | -6.349 | 108.403 | 1.00 | 32.42 | C |
| ATOM | 1158 | N | THR | A | 153 | -25.419 | -8.509 | 106.967 | 1.00 | 32.49 | N |
| ATOM | 1159 | CA | THR | A | 153 | -24.873 | -9.711 | 107.585 | 1.00 | 31.87 | C |
| ATOM | 1160 | C | THR | A | 153 | -24.123 | -9.336 | 108.858 | 1.00 | 33.40 | C |
| ATOM | 1161 | O | THR | A | 153 | -23.517 | -8.264 | 108.952 | 1.00 | 35.81 | O |
| ATOM | 1162 | CB | THR | A | 153 | -23.924 | -10.442 | 106.644 | 1.00 | 27.89 | C |
| ATOM | 1163 | OG1 | THR | A | 153 | -22.904 | -9.528 | 106.239 | 1.00 | 36.53 | O |
| ATOM | 1164 | CG2 | THR | A | 153 | -24.676 | -10.910 | 105.408 | 1.00 | 29.45 | C |
| ATOM | 1165 | N | VAL | A | 154 | -24.189 | -10.213 | 109.852 | 1.00 | 24.86 | N |
| ATOM | 1166 | CA | VAL | A | 154 | -23.508 | -10.013 | 111.116 | 1.00 | 27.57 | C |
| ATOM | 1167 | C | VAL | A | 154 | -22.745 | -11.281 | 111.472 | 1.00 | 29.28 | C |
| ATOM | 1168 | O | VAL | A | 154 | -23.316 | -12.374 | 111.480 | 1.00 | 35.29 | O |
| ATOM | 1169 | CB | VAL | A | 154 | -24.499 | -9.646 | 112.240 | 1.00 | 30.65 | C |
| ATOM | 1170 | CG1 | VAL | A | 154 | -23.770 | -9.393 | 113.542 | 1.00 | 30.74 | C |
| ATOM | 1171 | CG2 | VAL | A | 154 | -25.312 | -8.454 | 111.849 | 1.00 | 29.70 | C |
| ATOM | 1172 | N | SER | A | 155 | -21.463 | -11.137 | 111.766 | 1.00 | 29.75 | N |
| ATOM | 1173 | CA | SER | A | 155 | -20.720 | -12.183 | 112.446 | 1.00 | 32.79 | C |
| ATOM | 1174 | C | SER | A | 155 | -20.100 | -11.604 | 113.711 | 1.00 | 36.14 | C |
| ATOM | 1175 | O | SER | A | 155 | -20.134 | -10.393 | 113.961 | 1.00 | 34.64 | O |
| ATOM | 1176 | CB | SER | A | 155 | -19.648 | -12.800 | 111.550 | 1.00 | 28.09 | C |
| ATOM | 1177 | OG | SER | A | 155 | -18.682 | -11.839 | 111.164 | 1.00 | 32.25 | O |
| ATOM | 1178 | N | TRP | A | 156 | -19.522 | -12.495 | 114.504 | 1.00 | 28.20 | N |
| ATOM | 1179 | CA | TRP | A | 156 | -18.940 | -12.149 | 115.781 | 1.00 | 30.93 | C |
| ATOM | 1180 | C | TRP | A | 156 | -17.495 | -12.610 | 115.807 | 1.00 | 35.40 | C |
| ATOM | 1181 | O | TRP | A | 156 | -17.202 | -13.750 | 115.427 | 1.00 | 34.28 | O |
| ATOM | 1182 | CB | TRP | A | 156 | -19.731 | -12.786 | 116.913 | 1.00 | 30.86 | C |
| ATOM | 1183 | CG | TRP | A | 156 | -20.988 | -12.046 | 117.160 | 1.00 | 32.29 | C |
| ATOM | 1184 | CD1 | TRP | A | 156 | -22.184 | -12.252 | 116.553 | 1.00 | 32.31 | C |
| ATOM | 1185 | CD2 | TRP | A | 156 | -21.168 | -10.939 | 118.048 | 1.00 | 31.77 | C |
| ATOM | 1186 | NE1 | TRP | A | 156 | -23.106 | -11.348 | 117.012 | 1.00 | 33.15 | N |
| ATOM | 1187 | CE2 | TRP | A | 156 | -22.508 | -10.529 | 117.933 | 1.00 | 31.90 | C |
| ATOM | 1188 | CE3 | TRP | A | 156 | -20.325 | -10.253 | 118.929 | 1.00 | 33.09 | C |
| ATOM | 1189 | CZ2 | TRP | A | 156 | -23.037 | -9.477 | 118.679 | 1.00 | 29.54 | C |
| ATOM | 1190 | CZ3 | TRP | A | 156 | -20.852 | -9.197 | 119.665 | 1.00 | 34.64 | C |
| ATOM | 1191 | CH2 | TRP | A | 156 | -22.199 | -8.828 | 119.539 | 1.00 | 31.80 | C |
| ATOM | 1192 | N | ASN | A | 157 | -16.603 | -11.723 | 116.262 | 1.00 | 28.12 | N |
| ATOM | 1193 | CA | ASN | A | 157 | -15.185 | -12.020 | 116.346 | 1.00 | 27.84 | C |
| ATOM | 1194 | C | ASN | A | 157 | -14.675 | -12.543 | 115.010 | 1.00 | 31.80 | C |
| ATOM | 1195 | O | ASN | A | 157 | -13.925 | -13.515 | 114.956 | 1.00 | 33.84 | O |
| ATOM | 1196 | CB | ASN | A | 157 | -14.888 | -13.031 | 117.461 | 1.00 | 29.77 | C |
| ATOM | 1197 | CG | ASN | A | 157 | -15.039 | -12.460 | 118.845 | 1.00 | 26.46 | C |
| ATOM | 1198 | OD1 | ASN | A | 157 | -15.346 | -11.294 | 119.024 | 1.00 | 30.29 | O |
| ATOM | 1199 | ND2 | ASN | A | 157 | -14.823 | -13.294 | 119.842 | 1.00 | 33.06 | N |
| ATOM | 1200 | N | SER | A | 158 | -15.102 | -11.898 | 113.922 | 1.00 | 31.97 | N |
| ATOM | 1201 | CA | SER | A | 158 | -14.661 | -12.248 | 112.570 | 1.00 | 33.51 | C |
| ATOM | 1202 | C | SER | A | 158 | -14.956 | -13.699 | 112.215 | 1.00 | 36.13 | C |
| ATOM | 1203 | O | SER | A | 158 | -14.224 | -14.307 | 111.438 | 1.00 | 42.62 | O |
| ATOM | 1204 | CB | SER | A | 158 | -13.170 | -11.972 | 112.375 | 1.00 | 28.57 | C |
| ATOM | 1205 | OG | SER | A | 158 | -12.839 | -10.678 | 112.818 | 1.00 | 41.72 | O |

| | | | | | | | | | | | |
|---|---|---|---|---|---|---|---|---|---|---|---|
| ATOM | 1206 | N | GLY | A | 159 | -16.007 | -14.278 | 112.791 | 1.00 | 36.17 | N |
| ATOM | 1207 | CA | GLY | A | 159 | -16.371 | -15.654 | 112.532 | 1.00 | 30.18 | C |
| ATOM | 1208 | C | GLY | A | 159 | -15.840 | -16.648 | 113.540 | 1.00 | 32.92 | C |
| ATOM | 1209 | O | GLY | A | 159 | -16.226 | -17.823 | 113.497 | 1.00 | 29.97 | O |
| ATOM | 1210 | N | ALA | A | 160 | -14.960 | -16.217 | 114.437 | 1.00 | 37.02 | N |
| ATOM | 1211 | CA | ALA | A | 160 | -14.420 | -17.132 | 115.431 | 1.00 | 32.79 | C |
| ATOM | 1212 | C | ALA | A | 160 | -15.442 | -17.476 | 116.505 | 1.00 | 34.92 | C |
| ATOM | 1213 | O | ALA | A | 160 | -15.345 | -18.540 | 117.121 | 1.00 | 48.32 | O |
| ATOM | 1214 | CB | ALA | A | 160 | -13.164 | -16.532 | 116.061 | 1.00 | 28.82 | C |
| ATOM | 1215 | N | LEU | A | 161 | -16.416 | -16.617 | 116.751 | 1.00 | 35.16 | N |
| ATOM | 1216 | CA | LEU | A | 161 | -17.432 | -16.878 | 117.759 | 1.00 | 33.95 | C |
| ATOM | 1217 | C | LEU | A | 161 | -18.713 | -17.278 | 117.047 | 1.00 | 32.88 | C |
| ATOM | 1218 | O | LEU | A | 161 | -19.239 | -16.519 | 116.234 | 1.00 | 35.85 | O |
| ATOM | 1219 | CB | LEU | A | 161 | -17.653 | -15.658 | 118.648 | 1.00 | 30.18 | C |
| ATOM | 1220 | CG | LEU | A | 161 | -18.778 | -15.719 | 119.683 | 1.00 | 35.02 | C |
| ATOM | 1221 | CD1 | LEU | A | 161 | -18.826 | -17.042 | 120.432 | 1.00 | 28.23 | C |
| ATOM | 1222 | CD2 | LEU | A | 161 | -18.673 | -14.517 | 120.661 | 1.00 | 33.30 | C |
| ATOM | 1223 | N | THR | A | 162 | -19.233 | -18.450 | 117.394 | 1.00 | 39.79 | N |
| ATOM | 1224 | CA | THR | A | 162 | -20.298 | -19.078 | 116.630 | 1.00 | 33.54 | C |
| ATOM | 1225 | C | THR | A | 162 | -21.336 | -19.691 | 117.569 | 1.00 | 32.35 | C |
| ATOM | 1226 | O | THR | A | 162 | -22.541 | -19.506 | 117.391 | 1.00 | 30.46 | O |
| ATOM | 1227 | CB | THR | A | 162 | -19.662 | -20.099 | 115.669 | 1.00 | 33.49 | C |
| ATOM | 1228 | OG1 | THR | A | 162 | -19.956 | -19.724 | 114.318 | 1.00 | 27.42 | O |
| ATOM | 1229 | CG2 | THR | A | 162 | -20.098 | -21.543 | 115.959 | 1.00 | 34.98 | C |
| ATOM | 1230 | N | SER | A | 163 | -20.885 | -20.391 | 118.598 | 1.00 | 34.58 | N |
| ATOM | 1231 | CA | SER | A | 163 | -21.813 | -20.925 | 119.583 | 1.00 | 35.61 | C |
| ATOM | 1232 | C | SER | A | 163 | -22.538 | -19.785 | 120.301 | 1.00 | 35.40 | C |
| ATOM | 1233 | O | SER | A | 163 | -21.925 | -18.781 | 120.681 | 1.00 | 37.87 | O |
| ATOM | 1234 | CB | SER | A | 163 | -21.044 | -21.799 | 120.578 | 1.00 | 35.71 | C |
| ATOM | 1235 | OG | SER | A | 163 | -21.864 | -22.247 | 121.633 | 1.00 | 43.49 | O |
| ATOM | 1236 | N | GLY | A | 164 | -23.856 | -19.911 | 120.442 | 1.00 | 28.98 | N |
| ATOM | 1237 | CA | GLY | A | 164 | -24.625 | -18.958 | 121.206 | 1.00 | 25.01 | C |
| ATOM | 1238 | C | GLY | A | 164 | -25.128 | -17.744 | 120.446 | 1.00 | 33.29 | C |
| ATOM | 1239 | O | GLY | A | 164 | -25.869 | -16.946 | 121.031 | 1.00 | 33.20 | O |
| ATOM | 1240 | N | VAL | A | 165 | -24.754 | -17.576 | 119.160 | 1.00 | 30.78 | N |
| ATOM | 1241 | CA | VAL | A | 165 | -25.156 | -16.414 | 118.374 | 1.00 | 25.26 | C |
| ATOM | 1242 | C | VAL | A | 165 | -26.588 | -16.586 | 117.885 | 1.00 | 31.13 | C |
| ATOM | 1243 | O | VAL | A | 165 | -26.998 | -17.679 | 117.480 | 1.00 | 36.05 | O |
| ATOM | 1244 | CB | VAL | A | 165 | -24.189 | -16.187 | 117.195 | 1.00 | 24.24 | C |
| ATOM | 1245 | CG1 | VAL | A | 165 | -24.660 | -15.030 | 116.320 | 1.00 | 26.94 | C |
| ATOM | 1246 | CG2 | VAL | A | 165 | -22.806 | -15.872 | 117.701 | 1.00 | 26.51 | C |
| ATOM | 1247 | N | HIS | A | 166 | -27.368 | -15.507 | 117.975 | 1.00 | 30.95 | N |
| ATOM | 1248 | CA | HIS | A | 166 | -28.639 | -15.358 | 117.275 | 1.00 | 27.16 | C |
| ATOM | 1249 | C | HIS | A | 166 | -28.634 | -14.011 | 116.567 | 1.00 | 29.61 | C |
| ATOM | 1250 | O | HIS | A | 166 | -28.486 | -12.968 | 117.218 | 1.00 | 28.82 | O |
| ATOM | 1251 | CB | HIS | A | 166 | -29.841 | -15.434 | 118.225 | 1.00 | 26.50 | C |
| ATOM | 1252 | CG | HIS | A | 166 | -29.913 | -16.697 | 119.017 | 1.00 | 29.14 | C |
| ATOM | 1253 | ND1 | HIS | A | 166 | -30.069 | -17.932 | 118.430 | 1.00 | 31.57 | N |
| ATOM | 1254 | CD2 | HIS | A | 166 | -29.894 | -16.914 | 120.349 | 1.00 | 29.50 | C |
| ATOM | 1255 | CE1 | HIS | A | 166 | -30.123 | -18.859 | 119.368 | 1.00 | 30.30 | C |
| ATOM | 1256 | NE2 | HIS | A | 166 | -30.025 | -18.267 | 120.541 | 1.00 | 29.10 | N |
| ATOM | 1257 | N | THR | A | 167 | -28.765 | -14.030 | 115.242 | 1.00 | 32.27 | N |
| ATOM | 1258 | CA | THR | A | 167 | -29.023 | -12.828 | 114.457 | 1.00 | 30.55 | C |
| ATOM | 1259 | C | THR | A | 167 | -30.473 | -12.820 | 113.997 | 1.00 | 29.36 | C |
| ATOM | 1260 | O | THR | A | 167 | -30.914 | -13.738 | 113.306 | 1.00 | 34.07 | O |
| ATOM | 1261 | CB | THR | A | 167 | -28.089 | -12.737 | 113.264 | 1.00 | 24.74 | C |
| ATOM | 1262 | OG1 | THR | A | 167 | -26.740 | -12.697 | 113.745 | 1.00 | 35.43 | O |
| ATOM | 1263 | CG2 | THR | A | 167 | -28.377 | -11.481 | 112.509 | 1.00 | 23.22 | C |
| ATOM | 1264 | N | PHE | A | 168 | -31.182 | -11.797 | 114.351 | 1.00 | 28.41 | N |
| ATOM | 1265 | CA | PHE | A | 168 | -32.626 | -11.776 | 114.226 | 1.00 | 28.58 | C |
| ATOM | 1266 | C | PHE | A | 168 | -33.064 | -11.245 | 112.869 | 1.00 | 30.77 | C |
| ATOM | 1267 | O | PHE | A | 168 | -32.304 | -10.564 | 112.176 | 1.00 | 31.27 | O |
| ATOM | 1268 | CB | PHE | A | 168 | -33.222 | -10.953 | 115.358 | 1.00 | 22.95 | C |
| ATOM | 1269 | CG | PHE | A | 168 | -33.164 | -11.654 | 116.672 | 1.00 | 28.82 | C |
| ATOM | 1270 | CD1 | PHE | A | 168 | -34.177 | -12.526 | 117.052 | 1.00 | 30.01 | C |
| ATOM | 1271 | CD2 | PHE | A | 168 | -32.089 | -11.484 | 117.521 | 1.00 | 26.59 | C |
| ATOM | 1272 | CE1 | PHE | A | 168 | -34.120 | -13.192 | 118.269 | 1.00 | 26.13 | C |
| ATOM | 1273 | CE2 | PHE | A | 168 | -32.034 | -12.153 | 118.738 | 1.00 | 24.89 | C |
| ATOM | 1274 | CZ | PHE | A | 168 | -33.047 | -12.999 | 119.109 | 1.00 | 25.58 | C |
| ATOM | 1275 | N | PRO | A | 169 | -34.268 | -11.613 | 112.428 | 1.00 | 30.91 | N |
| ATOM | 1276 | CA | PRO | A | 169 | -34.825 | -10.991 | 111.221 | 1.00 | 28.45 | C |

| | | | | | | | | | | | |
|---|---|---|---|---|---|---|---|---|---|---|---|
| ATOM | 1277 | C | PRO | A | 169 | -35.017 | -9.500 | 111.445 | 1.00 | 30.28 | C |
| ATOM | 1278 | O | PRO | A | 169 | -35.392 | -9.063 | 112.534 | 1.00 | 26.91 | O |
| ATOM | 1279 | CB | PRO | A | 169 | -36.166 | -11.705 | 111.036 | 1.00 | 21.98 | C |
| ATOM | 1280 | CG | PRO | A | 169 | -36.064 | -12.951 | 111.870 | 1.00 | 25.73 | C |
| ATOM | 1281 | CD | PRO | A | 169 | -35.157 | -12.642 | 112.999 | 1.00 | 24.24 | C |
| ATOM | 1282 | N | ALA | A | 170 | -34.718 | -8.713 | 110.419 | 1.00 | 29.94 | N |
| ATOM | 1283 | CA | ALA | A | 170 | -34.909 | -7.277 | 110.530 | 1.00 | 28.66 | C |
| ATOM | 1284 | C | ALA | A | 170 | -36.397 | -6.962 | 110.631 | 1.00 | 24.51 | C |
| ATOM | 1285 | O | ALA | A | 170 | -37.231 | -7.715 | 110.147 | 1.00 | 20.86 | O |
| ATOM | 1286 | CB | ALA | A | 170 | -34.289 | -6.564 | 109.326 | 1.00 | 28.82 | C |
| ATOM | 1287 | N | VAL | A | 171 | -36.725 | -5.833 | 111.264 | 1.00 | 25.89 | N |
| ATOM | 1288 | CA | VAL | A | 171 | -38.070 | -5.279 | 111.200 | 1.00 | 24.76 | C |
| ATOM | 1289 | C | VAL | A | 171 | -38.012 | -4.012 | 110.358 | 1.00 | 28.48 | C |
| ATOM | 1290 | O | VAL | A | 171 | -36.997 | -3.307 | 110.326 | 1.00 | 27.98 | O |
| ATOM | 1291 | CB | VAL | A | 171 | -38.700 | -4.967 | 112.577 | 1.00 | 27.45 | C |
| ATOM | 1292 | CG1 | VAL | A | 171 | -38.882 | -6.245 | 113.387 | 1.00 | 27.65 | C |
| ATOM | 1293 | CG2 | VAL | A | 171 | -37.913 | -3.890 | 113.334 | 1.00 | 27.22 | C |
| ATOM | 1294 | N | LEU | A | 172 | -39.111 | -3.745 | 109.653 | 1.00 | 25.17 | N |
| ATOM | 1295 | CA | LEU | A | 172 | -39.301 | -2.534 | 108.867 | 1.00 | 27.51 | C |
| ATOM | 1296 | C | LEU | A | 172 | -40.092 | -1.517 | 109.692 | 1.00 | 31.05 | C |
| ATOM | 1297 | O | LEU | A | 172 | -41.270 | -1.730 | 109.995 | 1.00 | 33.68 | O |
| ATOM | 1298 | CB | LEU | A | 172 | -40.015 | -2.862 | 107.565 | 1.00 | 27.53 | C |
| ATOM | 1299 | CG | LEU | A | 172 | -40.360 | -1.652 | 106.713 | 1.00 | 31.73 | C |
| ATOM | 1300 | CD1 | LEU | A | 172 | -39.092 | -0.851 | 106.423 | 1.00 | 31.62 | C |
| ATOM | 1301 | CD2 | LEU | A | 172 | -41.011 | -2.118 | 105.405 | 1.00 | 28.68 | C |
| ATOM | 1302 | N | GLN | A | 173 | -39.439 | -0.422 | 110.052 | 1.00 | 25.80 | N |
| ATOM | 1303 | CA | GLN | A | 173 | -40.029 | 0.612 | 110.877 | 1.00 | 31.71 | C |
| ATOM | 1304 | C | GLN | A | 173 | -40.841 | 1.595 | 110.033 | 1.00 | 34.79 | C |
| ATOM | 1305 | O | GLN | A | 173 | -40.706 | 1.669 | 108.805 | 1.00 | 31.87 | O |
| ATOM | 1306 | CB | GLN | A | 173 | -38.945 | 1.372 | 111.634 | 1.00 | 33.05 | C |
| ATOM | 1307 | CG | GLN | A | 173 | -37.969 | 0.495 | 112.392 | 1.00 | 33.17 | C |
| ATOM | 1308 | CD | GLN | A | 173 | -36.669 | 1.222 | 112.686 | 1.00 | 35.50 | C |
| ATOM | 1309 | OE1 | GLN | A | 173 | -36.309 | 1.424 | 113.850 | 1.00 | 39.44 | O |
| ATOM | 1310 | NE2 | GLN | A | 173 | -35.960 | 1.626 | 111.629 | 1.00 | 33.32 | N |
| ATOM | 1311 | N | SER | A | 174 | -41.681 | 2.378 | 110.726 | 1.00 | 34.71 | N |
| ATOM | 1312 | CA | SER | A | 174 | -42.563 | 3.323 | 110.049 | 1.00 | 30.21 | C |
| ATOM | 1313 | C | SER | A | 174 | -41.773 | 4.343 | 109.245 | 1.00 | 31.66 | C |
| ATOM | 1314 | O | SER | A | 174 | -42.322 | 4.937 | 108.314 | 1.00 | 34.94 | O |
| ATOM | 1315 | CB | SER | A | 174 | -43.461 | 4.017 | 111.063 | 1.00 | 31.49 | C |
| ATOM | 1316 | OG | SER | A | 174 | -42.708 | 4.905 | 111.882 | 1.00 | 40.96 | O |
| ATOM | 1317 | N | SER | A | 175 | -40.480 | 4.495 | 109.533 | 1.00 | 29.45 | N |
| ATOM | 1318 | CA | SER | A | 175 | -39.594 | 5.363 | 108.769 | 1.00 | 29.62 | C |
| ATOM | 1319 | C | SER | A | 175 | -39.270 | 4.831 | 107.374 | 1.00 | 37.39 | C |
| ATOM | 1320 | O | SER | A | 175 | -38.665 | 5.564 | 106.581 | 1.00 | 35.03 | O |
| ATOM | 1321 | CB | SER | A | 175 | -38.288 | 5.558 | 109.531 | 1.00 | 26.45 | C |
| ATOM | 1322 | OG | SER | A | 175 | -37.522 | 4.366 | 109.537 | 1.00 | 33.35 | O |
| ATOM | 1323 | N | GLY | A | 176 | -39.610 | 3.574 | 107.068 | 1.00 | 33.52 | N |
| ATOM | 1324 | CA | GLY | A | 176 | -39.139 | 2.922 | 105.865 | 1.00 | 26.71 | C |
| ATOM | 1325 | C | GLY | A | 176 | -37.767 | 2.308 | 105.988 | 1.00 | 28.94 | C |
| ATOM | 1326 | O | GLY | A | 176 | -37.323 | 1.632 | 105.061 | 1.00 | 33.48 | O |
| ATOM | 1327 | N | LEU | A | 177 | -37.105 | 2.482 | 107.118 | 1.00 | 31.53 | N |
| ATOM | 1328 | CA | LEU | A | 177 | -35.796 | 1.912 | 107.374 | 1.00 | 32.69 | C |
| ATOM | 1329 | C | LEU | A | 177 | -35.924 | 0.601 | 108.155 | 1.00 | 33.78 | C |
| ATOM | 1330 | O | LEU | A | 177 | -36.839 | 0.412 | 108.965 | 1.00 | 27.20 | O |
| ATOM | 1331 | CB | LEU | A | 177 | -34.937 | 2.932 | 108.138 | 1.00 | 30.37 | C |
| ATOM | 1332 | CG | LEU | A | 177 | -34.691 | 4.252 | 107.381 | 1.00 | 31.43 | C |
| ATOM | 1333 | CD1 | LEU | A | 177 | -33.897 | 5.220 | 108.180 | 1.00 | 29.09 | C |
| ATOM | 1334 | CD2 | LEU | A | 177 | -33.948 | 4.024 | 106.072 | 1.00 | 32.81 | C |
| ATOM | 1335 | N | TYR | A | 178 | -34.979 | -0.299 | 107.915 | 1.00 | 32.33 | N |
| ATOM | 1336 | CA | TYR | A | 178 | -34.938 | -1.580 | 108.601 | 1.00 | 31.28 | C |
| ATOM | 1337 | C | TYR | A | 178 | -34.061 | -1.480 | 109.848 | 1.00 | 31.26 | C |
| ATOM | 1338 | O | TYR | A | 178 | -33.221 | -0.589 | 109.975 | 1.00 | 30.90 | O |
| ATOM | 1339 | CB | TYR | A | 178 | -34.399 | -2.679 | 107.681 | 1.00 | 30.14 | C |
| ATOM | 1340 | CG | TYR | A | 178 | -35.311 | -3.040 | 106.535 | 1.00 | 29.91 | C |
| ATOM | 1341 | CD2 | TYR | A | 178 | -35.140 | -2.470 | 105.275 | 1.00 | 30.71 | C |
| ATOM | 1342 | CD1 | TYR | A | 178 | -36.324 | -3.960 | 106.703 | 1.00 | 28.37 | C |
| ATOM | 1343 | CE2 | TYR | A | 178 | -35.966 | -2.793 | 104.226 | 1.00 | 32.21 | C |
| ATOM | 1344 | CE1 | TYR | A | 178 | -37.160 | -4.287 | 105.667 | 1.00 | 29.97 | C |
| ATOM | 1345 | CZ | TYR | A | 178 | -36.984 | -3.704 | 104.426 | 1.00 | 34.62 | C |
| ATOM | 1346 | OH | TYR | A | 178 | -37.822 | -4.043 | 103.385 | 1.00 | 35.36 | O |
| ATOM | 1347 | N | SER | A | 179 | -34.265 | -2.425 | 110.764 | 1.00 | 31.86 | N |

| | | | | | | | | | | | |
|---|---|---|---|---|---|---|---|---|---|---|---|
| ATOM | 1348 | CA | SER | A | 179 | -33.513 | -2.503 | 112.007 | 1.00 | 29.05 | C |
| ATOM | 1349 | C | SER | A | 179 | -33.405 | -3.960 | 112.453 | 1.00 | 30.76 | C |
| ATOM | 1350 | O | SER | A | 179 | -34.418 | -4.660 | 112.534 | 1.00 | 31.95 | O |
| ATOM | 1351 | CB | SER | A | 179 | -34.185 | -1.669 | 113.086 | 1.00 | 29.95 | C |
| ATOM | 1352 | OG | SER | A | 179 | -33.208 | -1.108 | 113.936 | 1.00 | 40.38 | O |
| ATOM | 1353 | N | LEU | A | 180 | -32.192 | -4.427 | 112.737 | 1.00 | 28.38 | N |
| ATOM | 1354 | CA | LEU | A | 180 | -32.044 | -5.768 | 113.282 | 1.00 | 31.63 | C |
| ATOM | 1355 | C | LEU | A | 180 | -31.118 | -5.768 | 114.492 | 1.00 | 29.92 | C |
| ATOM | 1356 | O | LEU | A | 180 | -30.388 | -4.810 | 114.763 | 1.00 | 25.79 | O |
| ATOM | 1357 | CB | LEU | A | 180 | -31.555 | -6.783 | 112.224 | 1.00 | 30.26 | C |
| ATOM | 1358 | CG | LEU | A | 180 | -30.143 | -7.049 | 111.701 | 1.00 | 31.57 | C |
| ATOM | 1359 | CD1 | LEU | A | 180 | -29.103 | -7.464 | 112.770 | 1.00 | 28.74 | C |
| ATOM | 1360 | CD2 | LEU | A | 180 | -30.291 | -8.158 | 110.658 | 1.00 | 28.38 | C |
| ATOM | 1361 | N | SER | A | 181 | -31.159 | -6.872 | 115.228 | 1.00 | 25.89 | N |
| ATOM | 1362 | CA | SER | A | 181 | -30.261 | -7.060 | 116.348 | 1.00 | 29.69 | C |
| ATOM | 1363 | C | SER | A | 181 | -29.651 | -8.454 | 116.283 | 1.00 | 34.25 | C |
| ATOM | 1364 | O | SER | A | 181 | -30.228 | -9.383 | 115.710 | 1.00 | 28.49 | O |
| ATOM | 1365 | CB | SER | A | 181 | -30.972 | -6.840 | 117.680 | 1.00 | 32.18 | C |
| ATOM | 1366 | OG | SER | A | 181 | -31.900 | -7.868 | 117.927 | 1.00 | 34.51 | O |
| ATOM | 1367 | N | SER | A | 182 | -28.451 | -8.572 | 116.852 | 1.00 | 34.33 | N |
| ATOM | 1368 | CA | SER | A | 182 | -27.720 | -9.826 | 116.939 | 1.00 | 31.79 | C |
| ATOM | 1369 | C | SER | A | 182 | -27.166 | -9.956 | 118.351 | 1.00 | 29.53 | C |
| ATOM | 1370 | O | SER | A | 182 | -26.634 | -8.992 | 118.899 | 1.00 | 29.22 | O |
| ATOM | 1371 | CB | SER | A | 182 | -26.589 | -9.875 | 115.889 | 1.00 | 28.22 | C |
| ATOM | 1372 | OG | SER | A | 182 | -25.793 | -11.040 | 116.033 | 1.00 | 32.19 | O |
| ATOM | 1373 | N | VAL | A | 183 | -27.302 | -11.140 | 118.946 | 1.00 | 31.33 | N |
| ATOM | 1374 | CA | VAL | A | 183 | -26.891 | -11.353 | 120.329 | 1.00 | 32.43 | C |
| ATOM | 1375 | C | VAL | A | 183 | -26.090 | -12.648 | 120.461 | 1.00 | 34.27 | C |
| ATOM | 1376 | O | VAL | A | 183 | -26.118 | -13.530 | 119.599 | 1.00 | 34.59 | O |
| ATOM | 1377 | CB | VAL | A | 183 | -28.100 | -11.386 | 121.290 | 1.00 | 34.07 | C |
| ATOM | 1378 | CG1 | VAL | A | 183 | -28.915 | -10.138 | 121.139 | 1.00 | 30.31 | C |
| ATOM | 1379 | CG2 | VAL | A | 183 | -28.962 | -12.619 | 121.027 | 1.00 | 34.20 | C |
| ATOM | 1380 | N | VAL | A | 184 | -25.370 | -12.750 | 121.567 | 1.00 | 30.31 | N |
| ATOM | 1381 | CA | VAL | A | 184 | -24.615 | -13.950 | 121.895 | 1.00 | 34.88 | C |
| ATOM | 1382 | C | VAL | A | 184 | -24.629 | -14.101 | 123.409 | 1.00 | 36.21 | C |
| ATOM | 1383 | O | VAL | A | 184 | -24.549 | -13.112 | 124.145 | 1.00 | 36.42 | O |
| ATOM | 1384 | CB | VAL | A | 184 | -23.172 | -13.893 | 121.322 | 1.00 | 36.52 | C |
| ATOM | 1385 | CG1 | VAL | A | 184 | -22.405 | -12.622 | 121.770 | 1.00 | 28.53 | C |
| ATOM | 1386 | CG2 | VAL | A | 184 | -22.413 | -15.146 | 121.668 | 1.00 | 28.44 | C |
| ATOM | 1387 | N | THR | A | 185 | -24.800 | -15.335 | 123.876 | 1.00 | 37.42 | N |
| ATOM | 1388 | CA | THR | A | 185 | -24.681 | -15.649 | 125.290 | 1.00 | 33.86 | C |
| ATOM | 1389 | C | THR | A | 185 | -23.296 | -16.235 | 125.522 | 1.00 | 36.91 | C |
| ATOM | 1390 | O | THR | A | 185 | -22.829 | -17.074 | 124.744 | 1.00 | 36.01 | O |
| ATOM | 1391 | CB | THR | A | 185 | -25.772 | -16.614 | 125.758 | 1.00 | 36.45 | C |
| ATOM | 1392 | OG1 | THR | A | 185 | -25.864 | -17.719 | 124.850 | 1.00 | 42.60 | O |
| ATOM | 1393 | CG2 | THR | A | 185 | -27.110 | -15.912 | 125.804 | 1.00 | 34.96 | C |
| ATOM | 1394 | N | VAL | A | 186 | -22.632 | -15.755 | 126.567 | 1.00 | 40.17 | N |
| ATOM | 1395 | CA | VAL | A | 186 | -21.268 | -16.144 | 126.917 | 1.00 | 38.85 | C |
| ATOM | 1396 | C | VAL | A | 186 | -21.196 | -16.288 | 128.432 | 1.00 | 47.21 | C |
| ATOM | 1397 | O | VAL | A | 186 | -22.080 | -15.806 | 129.155 | 1.00 | 45.09 | O |
| ATOM | 1398 | CB | VAL | A | 186 | -20.244 | -15.102 | 126.425 | 1.00 | 36.70 | C |
| ATOM | 1399 | CG1 | VAL | A | 186 | -20.351 | -14.919 | 124.902 | 1.00 | 33.73 | C |
| ATOM | 1400 | CG2 | VAL | A | 186 | -20.485 | -13.768 | 127.107 | 1.00 | 35.85 | C |
| ATOM | 1401 | N | PRO | A | 187 | -20.161 | -16.964 | 128.944 | 1.00 | 47.74 | N |
| ATOM | 1402 | CA | PRO | A | 187 | -20.031 | -17.090 | 130.404 | 1.00 | 46.98 | C |
| ATOM | 1403 | C | PRO | A | 187 | -19.776 | -15.738 | 131.058 | 1.00 | 48.85 | C |
| ATOM | 1404 | O | PRO | A | 187 | -19.016 | -14.922 | 130.538 | 1.00 | 46.36 | O |
| ATOM | 1405 | CB | PRO | A | 187 | -18.830 | -18.026 | 130.571 | 1.00 | 43.33 | C |
| ATOM | 1406 | CG | PRO | A | 187 | -18.751 | -18.759 | 129.303 | 1.00 | 39.13 | C |
| ATOM | 1407 | CD | PRO | A | 187 | -19.170 | -17.794 | 128.244 | 1.00 | 43.58 | C |
| ATOM | 1408 | N | SER | A | 188 | -20.423 | -15.509 | 132.208 | 1.00 | 50.33 | N |
| ATOM | 1409 | CA | SER | A | 188 | -20.194 | -14.283 | 132.972 | 1.00 | 47.34 | C |
| ATOM | 1410 | C | SER | A | 188 | -18.715 | -14.066 | 133.275 | 1.00 | 55.46 | C |
| ATOM | 1411 | O | SER | A | 188 | -18.208 | -12.942 | 133.158 | 1.00 | 49.43 | O |
| ATOM | 1412 | CB | SER | A | 188 | -21.001 | -14.310 | 134.262 | 1.00 | 52.91 | C |
| ATOM | 1413 | OG | SER | A | 188 | -22.363 | -14.112 | 133.973 | 1.00 | 59.47 | O |
| ATOM | 1414 | N | SER | A | 189 | -18.010 | -15.127 | 133.685 | 1.00 | 55.39 | N |
| ATOM | 1415 | CA | SER | A | 189 | -16.590 | -15.005 | 134.003 | 1.00 | 55.76 | C |
| ATOM | 1416 | C | SER | A | 189 | -15.769 | -14.513 | 132.811 | 1.00 | 58.34 | C |
| ATOM | 1417 | O | SER | A | 189 | -14.681 | -13.951 | 133.002 | 1.00 | 59.39 | O |
| ATOM | 1418 | CB | SER | A | 189 | -16.042 | -16.351 | 134.478 | 1.00 | 56.17 | C |

| | | | | | | | | | | | |
|---|---|---|---|---|---|---|---|---|---|---|---|
| ATOM | 1419 | OG | SER | A | 189 | -16.101 | -17.316 | 133.435 | 1.00 | 52.74 | O |
| ATOM | 1420 | N | SER | A | 190 | -16.276 | -14.687 | 131.590 | 1.00 | 53.51 | N |
| ATOM | 1421 | CA | SER | A | 190 | -15.528 | -14.312 | 130.396 | 1.00 | 56.22 | C |
| ATOM | 1422 | C | SER | A | 190 | -15.594 | -12.816 | 130.086 | 1.00 | 54.08 | C |
| ATOM | 1423 | O | SER | A | 190 | -14.802 | -12.330 | 129.265 | 1.00 | 51.28 | O |
| ATOM | 1424 | CB | SER | A | 190 | -16.020 | -15.138 | 129.197 | 1.00 | 51.36 | C |
| ATOM | 1425 | OG | SER | A | 190 | -17.368 | -14.852 | 128.863 | 1.00 | 46.74 | O |
| ATOM | 1426 | N | LEU | A | 191 | -16.523 | -12.081 | 130.693 | 1.00 | 53.01 | N |
| ATOM | 1427 | CA | LEU | A | 191 | -16.577 | -10.644 | 130.464 | 1.00 | 53.09 | C |
| ATOM | 1428 | C | LEU | A | 191 | -15.339 | -9.981 | 131.058 | 1.00 | 54.27 | C |
| ATOM | 1429 | O | LEU | A | 191 | -14.790 | -10.428 | 132.071 | 1.00 | 58.30 | O |
| ATOM | 1430 | CB | LEU | A | 191 | -17.839 | -10.054 | 131.081 | 1.00 | 38.62 | C |
| ATOM | 1431 | CG | LEU | A | 191 | -19.093 | -10.795 | 130.646 | 1.00 | 45.96 | C |
| ATOM | 1432 | CD1 | LEU | A | 191 | -20.297 | -10.313 | 131.431 | 1.00 | 42.60 | C |
| ATOM | 1433 | CD2 | LEU | A | 191 | -19.308 | -10.675 | 129.131 | 1.00 | 40.49 | C |
| ATOM | 1434 | N | GLY | A | 192 | -14.896 | -8.905 | 130.421 | 1.00 | 50.04 | N |
| ATOM | 1435 | CA | GLY | A | 192 | -13.722 | -8.196 | 130.886 | 1.00 | 59.39 | C |
| ATOM | 1436 | C | GLY | A | 192 | -12.384 | -8.841 | 130.570 | 1.00 | 59.35 | C |
| ATOM | 1437 | O | GLY | A | 192 | -11.382 | -8.120 | 130.468 | 1.00 | 65.09 | O |
| ATOM | 1438 | N | THR | A | 193 | -12.332 | -10.163 | 130.376 | 1.00 | 50.42 | N |
| ATOM | 1439 | CA | THR | A | 193 | -11.152 | -10.830 | 129.835 | 1.00 | 51.34 | C |
| ATOM | 1440 | C | THR | A | 193 | -11.269 | -11.178 | 128.350 | 1.00 | 53.60 | C |
| ATOM | 1441 | O | THR | A | 193 | -10.257 | -11.147 | 127.651 | 1.00 | 57.09 | O |
| ATOM | 1442 | CB | THR | A | 193 | -10.822 | -12.109 | 130.622 | 1.00 | 56.32 | C |
| ATOM | 1443 | OG1 | THR | A | 193 | -11.943 | -12.512 | 131.418 | 1.00 | 57.77 | O |
| ATOM | 1444 | CG2 | THR | A | 193 | -9.595 | -11.892 | 131.523 | 1.00 | 48.53 | C |
| ATOM | 1445 | N | GLN | A | 194 | -12.451 | -11.519 | 127.838 | 1.00 | 53.81 | N |
| ATOM | 1446 | CA | GLN | A | 194 | -12.619 | -11.879 | 126.431 | 1.00 | 47.52 | C |
| ATOM | 1447 | C | GLN | A | 194 | -13.238 | -10.716 | 125.660 | 1.00 | 45.35 | C |
| ATOM | 1448 | O | GLN | A | 194 | -14.169 | -10.066 | 126.142 | 1.00 | 47.02 | O |
| ATOM | 1449 | CB | GLN | A | 194 | -13.512 | -13.111 | 126.292 | 1.00 | 46.52 | C |
| ATOM | 1450 | CG | GLN | A | 194 | -13.761 | -13.550 | 124.857 | 1.00 | 46.74 | C |
| ATOM | 1451 | CD | GLN | A | 194 | -12.496 | -13.985 | 124.141 | 1.00 | 53.73 | C |
| ATOM | 1452 | OE1 | GLN | A | 194 | -12.011 | -13.301 | 123.239 | 1.00 | 52.18 | O |
| ATOM | 1453 | NE2 | GLN | A | 194 | -11.967 | -15.148 | 124.528 | 1.00 | 50.74 | N |
| ATOM | 1454 | N | THR | A | 195 | -12.745 | -10.462 | 124.453 | 1.00 | 44.88 | N |
| ATOM | 1455 | CA | THR | A | 195 | -13.244 | -9.345 | 123.657 | 1.00 | 46.31 | C |
| ATOM | 1456 | C | THR | A | 195 | -14.325 | -9.802 | 122.680 | 1.00 | 43.67 | C |
| ATOM | 1457 | O | THR | A | 195 | -14.211 | -10.862 | 122.057 | 1.00 | 41.58 | O |
| ATOM | 1458 | CB | THR | A | 195 | -12.104 | -8.651 | 122.909 | 1.00 | 53.06 | C |
| ATOM | 1459 | OG1 | THR | A | 195 | -11.441 | -7.742 | 123.803 | 1.00 | 57.28 | O |
| ATOM | 1460 | CG2 | THR | A | 195 | -12.631 | -7.884 | 121.694 | 1.00 | 42.53 | C |
| ATOM | 1461 | N | TYR | A | 196 | -15.393 | -9.007 | 122.579 | 1.00 | 43.78 | N |
| ATOM | 1462 | CA | TYR | A | 196 | -16.551 | -9.321 | 121.750 | 1.00 | 36.48 | C |
| ATOM | 1463 | C | TYR | A | 196 | -16.798 | -8.201 | 120.755 | 1.00 | 34.63 | C |
| ATOM | 1464 | O | TYR | A | 196 | -17.090 | -7.066 | 121.139 | 1.00 | 34.38 | O |
| ATOM | 1465 | CB | TYR | A | 196 | -17.769 | -9.564 | 122.621 | 1.00 | 32.34 | C |
| ATOM | 1466 | CG | TYR | A | 196 | -17.567 | -10.773 | 123.470 | 1.00 | 38.36 | C |
| ATOM | 1467 | CD1 | TYR | A | 196 | -17.517 | -12.036 | 122.900 | 1.00 | 36.90 | C |
| ATOM | 1468 | CD2 | TYR | A | 196 | -17.359 | -10.655 | 124.830 | 1.00 | 39.09 | C |
| ATOM | 1469 | CE1 | TYR | A | 196 | -17.316 | -13.151 | 123.676 | 1.00 | 37.95 | C |
| ATOM | 1470 | CE2 | TYR | A | 196 | -17.147 | -11.755 | 125.605 | 1.00 | 39.61 | C |
| ATOM | 1471 | CZ | TYR | A | 196 | -17.129 | -13.001 | 125.028 | 1.00 | 39.92 | C |
| ATOM | 1472 | OH | TYR | A | 196 | -16.921 | -14.098 | 125.825 | 1.00 | 47.25 | O |
| ATOM | 1473 | N | ILE | A | 197 | -16.653 | -8.516 | 119.478 | 1.00 | 35.82 | N |
| ATOM | 1474 | CA | ILE | A | 197 | -16.831 | -7.548 | 118.405 | 1.00 | 36.37 | C |
| ATOM | 1475 | C | ILE | A | 197 | -17.841 | -8.108 | 117.422 | 1.00 | 30.22 | C |
| ATOM | 1476 | O | ILE | A | 197 | -17.783 | -9.287 | 117.065 | 1.00 | 32.67 | O |
| ATOM | 1477 | CB | ILE | A | 197 | -15.496 | -7.222 | 117.702 | 1.00 | 32.39 | C |
| ATOM | 1478 | CG1 | ILE | A | 197 | -14.528 | -6.609 | 118.709 | 1.00 | 32.03 | C |
| ATOM | 1479 | CG2 | ILE | A | 197 | -15.722 | -6.302 | 116.521 | 1.00 | 28.21 | C |
| ATOM | 1480 | CD1 | ILE | A | 197 | -13.138 | -6.617 | 118.246 | 1.00 | 30.83 | C |
| ATOM | 1481 | N | CYS | A | 198 | -18.784 | -7.287 | 117.016 | 1.00 | 34.51 | N |
| ATOM | 1482 | CA | CYS | A | 198 | -19.717 | -7.687 | 115.984 | 1.00 | 35.67 | C |
| ATOM | 1483 | C | CYS | A | 198 | -19.346 | -7.002 | 114.676 | 1.00 | 33.53 | C |
| ATOM | 1484 | O | CYS | A | 198 | -19.039 | -5.805 | 114.645 | 1.00 | 32.08 | O |
| ATOM | 1485 | CB | CYS | A | 198 | -21.157 | -7.379 | 116.404 | 1.00 | 36.62 | C |
| ATOM | 1486 | SG | CYS | A | 198 | -21.662 | -5.699 | 116.191 | 1.00 | 46.42 | S |
| ATOM | 1487 | N | ASN | A | 199 | -19.373 | -7.777 | 113.598 | 1.00 | 31.00 | N |
| ATOM | 1488 | CA | ASN | A | 199 | -18.952 | -7.325 | 112.279 | 1.00 | 34.62 | C |
| ATOM | 1489 | C | ASN | A | 199 | -20.212 | -7.179 | 111.438 | 1.00 | 30.03 | C |

| | | | | | | | | | | | |
|---|---|---|---|---|---|---|---|---|---|---|---|
| ATOM | 1490 | O | ASN | A | 199 | -20.926 | -8.157 | 111.205 | 1.00 | 33.61 | O |
| ATOM | 1491 | CB | ASN | A | 199 | -17.969 | -8.322 | 111.669 | 1.00 | 30.80 | C |
| ATOM | 1492 | CG | ASN | A | 199 | -16.998 | -8.861 | 112.691 | 1.00 | 33.45 | C |
| ATOM | 1493 | OD1 | ASN | A | 199 | -17.071 | -10.028 | 113.087 | 1.00 | 30.25 | O |
| ATOM | 1494 | ND2 | ASN | A | 199 | -16.090 | -8.004 | 113.146 | 1.00 | 31.39 | N |
| ATOM | 1495 | N | VAL | A | 200 | -20.530 | -5.961 | 111.036 | 1.00 | 25.18 | N |
| ATOM | 1496 | CA | VAL | A | 200 | -21.753 | -5.699 | 110.295 | 1.00 | 31.02 | C |
| ATOM | 1497 | C | VAL | A | 200 | -21.346 | -5.394 | 108.868 | 1.00 | 31.80 | C |
| ATOM | 1498 | O | VAL | A | 200 | -20.402 | -4.632 | 108.642 | 1.00 | 32.16 | O |
| ATOM | 1499 | CB | VAL | A | 200 | -22.581 | -4.546 | 110.896 | 1.00 | 26.09 | C |
| ATOM | 1500 | CG1 | VAL | A | 200 | -23.862 | -4.409 | 110.130 | 1.00 | 31.91 | C |
| ATOM | 1501 | CG2 | VAL | A | 200 | -22.900 | -4.792 | 112.348 | 1.00 | 27.12 | C |
| ATOM | 1502 | N | ASN | A | 201 | -22.022 | -6.016 | 107.910 | 1.00 | 31.12 | N |
| ATOM | 1503 | CA | ASN | A | 201 | -21.800 | -5.693 | 106.511 | 1.00 | 34.13 | C |
| ATOM | 1504 | C | ASN | A | 201 | -23.127 | -5.341 | 105.866 | 1.00 | 32.42 | C |
| ATOM | 1505 | O | ASN | A | 201 | -24.065 | -6.141 | 105.883 | 1.00 | 35.02 | O |
| ATOM | 1506 | CB | ASN | A | 201 | -21.122 | -6.839 | 105.767 | 1.00 | 36.09 | C |
| ATOM | 1507 | CG | ASN | A | 201 | -20.557 | -6.390 | 104.457 | 1.00 | 39.19 | C |
| ATOM | 1508 | OD1 | ASN | A | 201 | -20.860 | -5.296 | 104.000 | 1.00 | 43.01 | O |
| ATOM | 1509 | ND2 | ASN | A | 201 | -19.730 | -7.218 | 103.844 | 1.00 | 44.41 | N |
| ATOM | 1510 | N | HIS | A | 202 | -23.222 | -4.123 | 105.361 | 1.00 | 34.15 | N |
| ATOM | 1511 | CA | HIS | A | 202 | -24.349 | -3.682 | 104.561 | 1.00 | 33.64 | C |
| ATOM | 1512 | C | HIS | A | 202 | -23.801 | -3.403 | 103.168 | 1.00 | 39.81 | C |
| ATOM | 1513 | O | HIS | A | 202 | -23.463 | -2.264 | 102.836 | 1.00 | 37.99 | O |
| ATOM | 1514 | CB | HIS | A | 202 | -25.001 | -2.473 | 105.147 | 1.00 | 31.71 | C |
| ATOM | 1515 | CG | HIS | A | 202 | -26.244 | -2.052 | 104.425 | 1.00 | 37.75 | C |
| ATOM | 1516 | ND1 | HIS | A | 202 | -26.419 | -0.780 | 103.917 | 1.00 | 38.12 | N |
| ATOM | 1517 | CD2 | HIS | A | 202 | -27.377 | -2.735 | 104.129 | 1.00 | 32.94 | C |
| ATOM | 1518 | CE1 | HIS | A | 202 | -27.607 | -0.699 | 103.344 | 1.00 | 37.74 | C |
| ATOM | 1519 | NE2 | HIS | A | 202 | -28.210 | -1.871 | 103.463 | 1.00 | 34.91 | N |
| ATOM | 1520 | N | LYS | A | 203 | -23.690 | -4.461 | 102.359 | 1.00 | 34.79 | N |
| ATOM | 1521 | CA | LYS | A | 203 | -23.249 | -4.285 | 100.978 | 1.00 | 35.75 | C |
| ATOM | 1522 | C | LYS | A | 203 | -24.071 | -3.261 | 100.195 | 1.00 | 39.45 | C |
| ATOM | 1523 | O | LYS | A | 203 | -23.482 | -2.573 | 99.346 | 1.00 | 38.28 | O |
| ATOM | 1524 | CB | LYS | A | 203 | -23.166 | -5.647 | 100.266 | 1.00 | 29.77 | C |
| ATOM | 1525 | CG | LYS | A | 203 | -21.879 | -6.425 | 100.649 | 1.00 | 40.20 | C |
| ATOM | 1526 | CD | LYS | A | 203 | -20.652 | -5.726 | 99.988 | 1.00 | 63.25 | C |
| ATOM | 1527 | CE | LYS | A | 203 | -19.279 | -6.000 | 100.660 | 1.00 | 68.58 | C |
| ATOM | 1528 | NZ | LYS | A | 203 | -18.938 | -7.439 | 100.898 | 1.00 | 69.85 | N1+ |
| ATOM | 1529 | N | PRO | A | 204 | -25.392 | -3.108 | 100.390 | 1.00 | 41.00 | N |
| ATOM | 1530 | CA | PRO | A | 204 | -26.120 | -2.121 | 99.566 | 1.00 | 38.96 | C |
| ATOM | 1531 | C | PRO | A | 204 | -25.629 | -0.681 | 99.705 | 1.00 | 40.45 | C |
| ATOM | 1532 | O | PRO | A | 204 | -25.839 | 0.114 | 98.782 | 1.00 | 44.07 | O |
| ATOM | 1533 | CB | PRO | A | 204 | -27.568 | -2.272 | 100.043 | 1.00 | 36.97 | C |
| ATOM | 1534 | CG | PRO | A | 204 | -27.647 | -3.652 | 100.563 | 1.00 | 35.26 | C |
| ATOM | 1535 | CD | PRO | A | 204 | -26.327 | -3.952 | 101.168 | 1.00 | 33.30 | C |
| ATOM | 1536 | N | SER | A | 205 | -25.061 | -0.287 | 100.845 | 1.00 | 42.01 | N |
| ATOM | 1537 | CA | SER | A | 205 | -24.455 | 1.034 | 100.993 | 1.00 | 40.43 | C |
| ATOM | 1538 | C | SER | A | 205 | -22.931 | 0.956 | 101.094 | 1.00 | 39.05 | C |
| ATOM | 1539 | O | SER | A | 205 | -22.279 | 1.963 | 101.369 | 1.00 | 37.64 | O |
| ATOM | 1540 | CB | SER | A | 205 | -25.052 | 1.778 | 102.195 | 1.00 | 35.25 | C |
| ATOM | 1541 | OG | SER | A | 205 | -24.659 | 1.213 | 103.424 | 1.00 | 36.65 | O |
| ATOM | 1542 | N | ASN | A | 206 | -22.354 | -0.219 | 100.868 | 1.00 | 42.36 | N |
| ATOM | 1543 | CA | ASN | A | 206 | -20.919 | -0.449 | 101.017 | 1.00 | 44.31 | C |
| ATOM | 1544 | C | ASN | A | 206 | -20.414 | 0.021 | 102.382 | 1.00 | 46.67 | C |
| ATOM | 1545 | O | ASN | A | 206 | -19.385 | 0.680 | 102.506 | 1.00 | 50.37 | O |
| ATOM | 1546 | CB | ASN | A | 206 | -20.146 | 0.208 | 99.880 | 1.00 | 49.52 | C |
| ATOM | 1547 | CG | ASN | A | 206 | -19.229 | -0.766 | 99.187 | 1.00 | 61.70 | C |
| ATOM | 1548 | OD1 | ASN | A | 206 | -19.323 | -1.979 | 99.401 | 1.00 | 67.47 | O |
| ATOM | 1549 | ND2 | ASN | A | 206 | -18.336 | -0.253 | 98.352 | 1.00 | 70.26 | N |
| ATOM | 1550 | N | THR | A | 207 | -21.150 | -0.352 | 103.423 | 1.00 | 45.98 | N |
| ATOM | 1551 | CA | THR | A | 207 | -20.861 | 0.041 | 104.792 | 1.00 | 36.87 | C |
| ATOM | 1552 | C | THR | A | 207 | -20.417 | -1.180 | 105.574 | 1.00 | 37.03 | C |
| ATOM | 1553 | O | THR | A | 207 | -21.118 | -2.192 | 105.590 | 1.00 | 41.13 | O |
| ATOM | 1554 | CB | THR | A | 207 | -22.100 | 0.626 | 105.457 | 1.00 | 42.00 | C |
| ATOM | 1555 | OG1 | THR | A | 207 | -22.616 | 1.689 | 104.649 | 1.00 | 45.30 | O |
| ATOM | 1556 | CG2 | THR | A | 207 | -21.767 | 1.132 | 106.876 | 1.00 | 42.01 | C |
| ATOM | 1557 | N | LYS | A | 208 | -19.263 | -1.088 | 106.210 | 1.00 | 36.32 | N |
| ATOM | 1558 | CA | LYS | A | 208 | -18.793 | -2.110 | 107.127 | 1.00 | 32.74 | C |
| ATOM | 1559 | C | LYS | A | 208 | -18.511 | -1.465 | 108.478 | 1.00 | 34.61 | C |
| ATOM | 1560 | O | LYS | A | 208 | -18.088 | -0.307 | 108.545 | 1.00 | 39.61 | O |

| | | | | | | | | | | | |
|---|---|---|---|---|---|---|---|---|---|---|---|
| ATOM | 1561 | CB | LYS | A | 208 | -17.552 | -2.827 | 106.585 | 1.00 | 40.21 | C |
| ATOM | 1562 | CG | LYS | A | 208 | -17.865 | -3.863 | 105.502 | 1.00 | 39.11 | C |
| ATOM | 1563 | CD | LYS | A | 208 | -16.707 | -4.821 | 105.239 | 1.00 | 34.67 | C |
| ATOM | 1564 | CE | LYS | A | 208 | -15.518 | -4.128 | 104.606 | 1.00 | 49.09 | C |
| ATOM | 1565 | NZ | LYS | A | 208 | -14.349 | -5.076 | 104.456 | 1.00 | 57.65 | N1+ |
| ATOM | 1566 | N | VAL | A | 209 | -18.846 | -2.174 | 109.552 | 1.00 | 32.84 | N |
| ATOM | 1567 | CA | VAL | A | 209 | -18.671 | -1.684 | 110.917 | 1.00 | 32.20 | C |
| ATOM | 1568 | C | VAL | A | 209 | -18.247 | -2.844 | 111.797 | 1.00 | 34.40 | C |
| ATOM | 1569 | O | VAL | A | 209 | -18.906 | -3.889 | 111.817 | 1.00 | 38.11 | O |
| ATOM | 1570 | CB | VAL | A | 209 | -19.960 | -1.054 | 111.491 | 1.00 | 31.14 | C |
| ATOM | 1571 | CG1 | VAL | A | 209 | -19.816 | -0.826 | 112.974 | 1.00 | 27.96 | C |
| ATOM | 1572 | CG2 | VAL | A | 209 | -20.276 | 0.252 | 110.810 | 1.00 | 26.07 | C |
| ATOM | 1573 | N | ASP | A | 210 | -17.195 | -2.642 | 112.574 | 1.00 | 36.64 | N |
| ATOM | 1574 | CA | ASP | A | 210 | -16.821 | -3.545 | 113.659 | 1.00 | 33.62 | C |
| ATOM | 1575 | C | ASP | A | 210 | -17.053 | -2.791 | 114.962 | 1.00 | 30.39 | C |
| ATOM | 1576 | O | ASP | A | 210 | -16.490 | -1.718 | 115.159 | 1.00 | 32.48 | O |
| ATOM | 1577 | CB | ASP | A | 210 | -15.360 | -3.980 | 113.548 | 1.00 | 32.01 | C |
| ATOM | 1578 | CG | ASP | A | 210 | -15.084 | -4.843 | 112.325 | 1.00 | 42.55 | C |
| ATOM | 1579 | OD1 | ASP | A | 210 | -15.829 | -5.839 | 112.124 | 1.00 | 46.60 | O |
| ATOM | 1580 | OD2 | ASP | A | 210 | -14.119 | -4.527 | 111.570 | 1.00 | 34.51 | O1- |
| ATOM | 1581 | N | LYS | A | 211 | -17.904 | -3.319 | 115.827 | 1.00 | 34.41 | N |
| ATOM | 1582 | CA | LYS | A | 211 | -18.270 | -2.654 | 117.073 | 1.00 | 28.87 | C |
| ATOM | 1583 | C | LYS | A | 211 | -17.832 | -3.519 | 118.243 | 1.00 | 33.13 | C |
| ATOM | 1584 | O | LYS | A | 211 | -18.283 | -4.661 | 118.374 | 1.00 | 31.45 | O |
| ATOM | 1585 | CB | LYS | A | 211 | -19.775 | -2.409 | 117.155 | 1.00 | 30.56 | C |
| ATOM | 1586 | CG | LYS | A | 211 | -20.246 | -1.799 | 118.460 | 1.00 | 33.07 | C |
| ATOM | 1587 | CD | LYS | A | 211 | -19.646 | -0.430 | 118.618 | 1.00 | 36.20 | C |
| ATOM | 1588 | CE | LYS | A | 211 | -20.539 | 0.467 | 119.407 | 1.00 | 33.18 | C |
| ATOM | 1589 | NZ | LYS | A | 211 | -20.169 | 1.877 | 119.156 | 1.00 | 35.41 | N1+ |
| ATOM | 1590 | N | LYS | A | 212 | -16.962 | -2.972 | 119.094 | 1.00 | 35.68 | N |
| ATOM | 1591 | CA | LYS | A | 212 | -16.654 | -3.617 | 120.354 | 1.00 | 28.26 | C |
| ATOM | 1592 | C | LYS | A | 212 | -17.806 | -3.349 | 121.296 | 1.00 | 30.86 | C |
| ATOM | 1593 | O | LYS | A | 212 | -18.409 | -2.271 | 121.278 | 1.00 | 33.51 | O |
| ATOM | 1594 | CB | LYS | A | 212 | -15.341 | -3.112 | 120.955 | 1.00 | 31.23 | C |
| ATOM | 1595 | CG | LYS | A | 212 | -14.740 | -4.080 | 121.984 | 1.00 | 38.53 | C |
| ATOM | 1596 | CD | LYS | A | 212 | -13.567 | -3.503 | 122.778 | 1.00 | 42.63 | C |
| ATOM | 1597 | CE | LYS | A | 212 | -13.088 | -4.492 | 123.847 | 1.00 | 44.88 | C |
| ATOM | 1598 | NZ | LYS | A | 212 | -12.001 | -3.960 | 124.723 | 1.00 | 52.61 | N1+ |
| ATOM | 1599 | N | VAL | A | 213 | -18.175 | -4.376 | 122.039 | 1.00 | 30.30 | N |
| ATOM | 1600 | CA | VAL | A | 213 | -19.246 | -4.322 | 123.012 | 1.00 | 34.60 | C |
| ATOM | 1601 | C | VAL | A | 213 | -18.589 | -4.663 | 124.336 | 1.00 | 35.78 | C |
| ATOM | 1602 | O | VAL | A | 213 | -18.141 | -5.795 | 124.530 | 1.00 | 39.34 | O |
| ATOM | 1603 | CB | VAL | A | 213 | -20.380 | -5.300 | 122.667 | 1.00 | 36.86 | C |
| ATOM | 1604 | CG1 | VAL | A | 213 | -21.521 | -5.226 | 123.700 | 1.00 | 37.67 | C |
| ATOM | 1605 | CG2 | VAL | A | 213 | -20.903 | -5.028 | 121.278 | 1.00 | 27.28 | C |
| ATOM | 1606 | N | GLU | A | 214 | -18.489 | -3.687 | 125.228 | 1.00 | 43.48 | N |
| ATOM | 1607 | CA | GLU | A | 214 | -17.839 | -3.878 | 126.521 | 1.00 | 42.79 | C |
| ATOM | 1608 | C | GLU | A | 214 | -18.866 | -3.868 | 127.636 | 1.00 | 39.79 | C |
| ATOM | 1609 | O | GLU | A | 214 | -19.925 | -3.250 | 127.508 | 1.00 | 39.59 | O |
| ATOM | 1610 | CB | GLU | A | 214 | -16.795 | -2.787 | 126.788 | 1.00 | 42.03 | C |
| ATOM | 1611 | CG | GLU | A | 214 | -15.715 | -2.770 | 125.730 | 1.00 | 53.61 | C |
| ATOM | 1612 | CD | GLU | A | 214 | -14.732 | -1.636 | 125.888 | 1.00 | 60.49 | C |
| ATOM | 1613 | OE1 | GLU | A | 214 | -13.614 | -1.878 | 126.391 | 1.00 | 64.12 | O |
| ATOM | 1614 | OE2 | GLU | A | 214 | -15.085 | -0.500 | 125.505 | 1.00 | 65.45 | O1- |
| ATOM | 1615 | N | PRO | A | 215 | -18.599 | -4.573 | 128.724 | 1.00 | 42.19 | N |
| ATOM | 1616 | CA | PRO | A | 215 | -19.495 | -4.496 | 129.882 | 1.00 | 47.10 | C |
| ATOM | 1617 | C | PRO | A | 215 | -19.546 | -3.089 | 130.452 | 1.00 | 54.06 | C |
| ATOM | 1618 | O | PRO | A | 215 | -18.521 | -2.414 | 130.566 | 1.00 | 56.58 | O |
| ATOM | 1619 | CB | PRO | A | 215 | -18.885 | -5.492 | 130.874 | 1.00 | 49.68 | C |
| ATOM | 1620 | CG | PRO | A | 215 | -17.567 | -5.950 | 130.264 | 1.00 | 48.26 | C |
| ATOM | 1621 | CD | PRO | A | 215 | -17.651 | -5.695 | 128.809 | 1.00 | 45.54 | C |
| ATOM | 1622 | N | LYS | A | 216 | -20.760 | -2.636 | 130.768 | 1.00 | 59.07 | N |
| ATOM | 1623 | CA | LYS | A | 216 | -21.008 | -1.289 | 131.272 | 1.00 | 66.58 | C |
| ATOM | 1624 | C | LYS | A | 216 | -21.516 | -1.355 | 132.710 | 1.00 | 80.80 | C |
| ATOM | 1625 | O | LYS | A | 216 | -22.468 | -2.092 | 133.005 | 1.00 | 75.99 | O |
| ATOM | 1626 | CB | LYS | A | 216 | -22.036 | -0.555 | 130.408 | 1.00 | 66.80 | C |
| ATOM | 1627 | CG | LYS | A | 216 | -22.360 | 0.859 | 130.899 | 1.00 | 73.54 | C |
| ATOM | 1628 | CD | LYS | A | 216 | -23.096 | 1.653 | 129.834 | 1.00 | 77.62 | C |
| ATOM | 1629 | CE | LYS | A | 216 | -23.325 | 3.092 | 130.255 | 1.00 | 75.50 | C |
| ATOM | 1630 | NZ | LYS | A | 216 | -23.769 | 3.921 | 129.103 | 1.00 | 65.88 | N1+ |
| ATOM | 1631 | N | SER | A | 217 | -20.927 | -0.528 | 133.580 | 1.00 | 90.64 | N |

```
ATOM   1632  CA  SER A 217     -21.323  -0.451 134.994  1.00 92.77           C
ATOM   1633  C   SER A 217     -22.109   0.829 135.282  1.00 85.80           C
ATOM   1634  O   SER A 217     -23.332   0.797 135.455  1.00 82.11           O
ATOM   1635  CB  SER A 217     -20.096  -0.528 135.915  1.00 84.71           C
ATOM   1636  OG  SER A 217     -19.239   0.585 135.722  1.00 86.75           O
TER
ATOM   1637  N   GLU B   1     -59.401 -19.548  88.941  1.00 32.61           N
ATOM   1638  CA  GLU B   1     -58.164 -20.286  89.135  1.00 32.73           C
ATOM   1639  C   GLU B   1     -58.344 -21.415  90.159  1.00 39.88           C
ATOM   1640  O   GLU B   1     -59.196 -21.331  91.040  1.00 39.29           O
ATOM   1641  CB  GLU B   1     -57.054 -19.366  89.613  1.00 32.87           C
ATOM   1642  CG  GLU B   1     -57.037 -19.224  91.116  1.00 33.91           C
ATOM   1643  CD  GLU B   1     -56.083 -18.160  91.599  1.00 44.88           C
ATOM   1644  OE1 GLU B   1     -56.022 -17.970  92.832  1.00 55.17           O
ATOM   1645  OE2 GLU B   1     -55.378 -17.536  90.763  1.00 44.77           O1-
ATOM   1646  N   ILE B   2     -57.536 -22.470  90.052  1.00 36.20           N
ATOM   1647  CA  ILE B   2     -57.589 -23.542  91.037  1.00 31.00           C
ATOM   1648  C   ILE B   2     -56.950 -23.074  92.339  1.00 27.84           C
ATOM   1649  O   ILE B   2     -55.774 -22.694  92.370  1.00 27.28           O
ATOM   1650  CB  ILE B   2     -56.893 -24.797  90.521  1.00 30.83           C
ATOM   1651  CG1 ILE B   2     -57.571 -25.303  89.259  1.00 24.03           C
ATOM   1652  CG2 ILE B   2     -56.832 -25.856  91.649  1.00 24.77           C
ATOM   1653  CD1 ILE B   2     -56.866 -26.508  88.716  1.00 24.00           C
ATOM   1654  N   VAL B   3     -57.718 -23.116  93.424  1.00 28.01           N
ATOM   1655  CA  VAL B   3     -57.227 -22.750  94.748  1.00 31.60           C
ATOM   1656  C   VAL B   3     -56.810 -24.011  95.498  1.00 30.30           C
ATOM   1657  O   VAL B   3     -57.580 -24.976  95.602  1.00 30.30           O
ATOM   1658  CB  VAL B   3     -58.287 -21.965  95.535  1.00 30.01           C
ATOM   1659  CG1 VAL B   3     -57.782 -21.724  96.916  1.00 23.45           C
ATOM   1660  CG2 VAL B   3     -58.637 -20.645  94.815  1.00 24.87           C
ATOM   1661  N   LEU B   4     -55.591 -24.017  96.006  1.00 27.66           N
ATOM   1662  CA  LEU B   4     -55.047 -25.168  96.709  1.00 27.72           C
ATOM   1663  C   LEU B   4     -54.941 -24.793  98.170  1.00 30.07           C
ATOM   1664  O   LEU B   4     -54.281 -23.805  98.509  1.00 34.25           O
ATOM   1665  CB  LEU B   4     -53.677 -25.570  96.165  1.00 31.34           C
ATOM   1666  CG  LEU B   4     -53.596 -26.025  94.711  1.00 30.78           C
ATOM   1667  CD1 LEU B   4     -52.155 -26.475  94.361  1.00 29.60           C
ATOM   1668  CD2 LEU B   4     -54.571 -27.161  94.511  1.00 23.79           C
ATOM   1669  N   THR B   5     -55.591 -25.568  99.026  1.00 26.39           N
ATOM   1670  CA  THR B   5     -55.586 -25.330 100.458  1.00 23.07           C
ATOM   1671  C   THR B   5     -54.765 -26.407 101.130  1.00 26.07           C
ATOM   1672  O   THR B   5     -55.089 -27.591 101.034  1.00 29.54           O
ATOM   1673  CB  THR B   5     -57.007 -25.330 101.019  1.00 23.58           C
ATOM   1674  OG1 THR B   5     -57.813 -24.424 100.256  1.00 25.83           O
ATOM   1675  CG2 THR B   5     -56.992 -24.892 102.458  1.00 18.31           C
ATOM   1676  N   GLN B   6     -53.731 -26.003 101.830  1.00 23.61           N
ATOM   1677  CA  GLN B   6     -52.920 -26.943 102.569  1.00 25.57           C
ATOM   1678  C   GLN B   6     -53.328 -26.929 104.024  1.00 24.28           C
ATOM   1679  O   GLN B   6     -53.585 -25.871 104.592  1.00 28.38           O
ATOM   1680  CB  GLN B   6     -51.432 -26.623 102.442  1.00 21.43           C
ATOM   1681  CG  GLN B   6     -50.866 -27.125 101.172  1.00 25.09           C
ATOM   1682  CD  GLN B   6     -49.406 -26.785 101.003  1.00 29.92           C
ATOM   1683  OE1 GLN B   6     -49.065 -25.856 100.258  1.00 30.10           O
ATOM   1684  NE2 GLN B   6     -48.529 -27.542 101.671  1.00 23.00           N
ATOM   1685  N   SER B   7     -53.388 -28.113 104.609  1.00 28.46           N
ATOM   1686  CA  SER B   7     -53.526 -28.273 106.046  1.00 32.14           C
ATOM   1687  C   SER B   7     -52.626 -29.410 106.541  1.00 32.52           C
ATOM   1688  O   SER B   7     -52.238 -30.294 105.774  1.00 31.45           O
ATOM   1689  CB  SER B   7     -54.960 -28.558 106.404  1.00 26.70           C
ATOM   1690  OG  SER B   7     -55.208 -29.883 106.045  1.00 39.01           O
ATOM   1691  N   PRO B   8     -52.242 -29.367 107.816  1.00 35.79           N
ATOM   1692  CA  PRO B   8     -52.444 -28.237 108.728  1.00 32.17           C
ATOM   1693  C   PRO B   8     -51.456 -27.142 108.349  1.00 33.18           C
ATOM   1694  O   PRO B   8     -50.550 -27.465 107.590  1.00 35.18           O
ATOM   1695  CB  PRO B   8     -52.119 -28.837 110.103  1.00 26.39           C
ATOM   1696  CG  PRO B   8     -51.061 -29.854 109.786  1.00 33.66           C
ATOM   1697  CD  PRO B   8     -51.426 -30.438 108.422  1.00 30.11           C
ATOM   1698  N   GLY B   9     -51.584 -25.920 108.877  1.00 35.12           N
ATOM   1699  CA  GLY B   9     -50.583 -24.899 108.603  1.00 25.91           C
ATOM   1700  C   GLY B   9     -49.249 -25.202 109.264  1.00 27.68           C
ATOM   1701  O   GLY B   9     -48.196 -25.015 108.661  1.00 27.97           O
```

| | | | | | | | | | | | |
|---|---|---|---|---|---|---|---|---|---|---|---|
| ATOM | 1702 | N | THR | B | 10 | -49.274 | -25.726 | 110.487 | 1.00 | 26.27 | N |
| ATOM | 1703 | CA | THR | B | 10 | -48.056 | -26.118 | 111.179 | 1.00 | 27.02 | C |
| ATOM | 1704 | C | THR | B | 10 | -48.197 | -27.524 | 111.746 | 1.00 | 26.07 | C |
| ATOM | 1705 | O | THR | B | 10 | -49.251 | -27.890 | 112.264 | 1.00 | 30.58 | O |
| ATOM | 1706 | CB | THR | B | 10 | -47.725 | -25.143 | 112.310 | 1.00 | 26.29 | C |
| ATOM | 1707 | OG1 | THR | B | 10 | -47.699 | -23.812 | 111.789 | 1.00 | 30.87 | O |
| ATOM | 1708 | CG2 | THR | B | 10 | -46.368 | -25.480 | 112.929 | 1.00 | 18.58 | C |
| ATOM | 1709 | N | LEU | B | 11 | -47.117 | -28.296 | 111.669 | 1.00 | 28.05 | N |
| ATOM | 1710 | CA | LEU | B | 11 | -47.057 | -29.670 | 112.156 | 1.00 | 27.74 | C |
| ATOM | 1711 | C | LEU | B | 11 | -45.839 | -29.819 | 113.059 | 1.00 | 29.19 | C |
| ATOM | 1712 | O | LEU | B | 11 | -44.706 | -29.647 | 112.600 | 1.00 | 31.16 | O |
| ATOM | 1713 | CB | LEU | B | 11 | -46.954 | -30.632 | 110.978 | 1.00 | 33.49 | C |
| ATOM | 1714 | CG | LEU | B | 11 | -47.842 | -31.847 | 110.756 | 1.00 | 40.86 | C |
| ATOM | 1715 | CD1 | LEU | B | 11 | -47.188 | -32.642 | 109.625 | 1.00 | 33.69 | C |
| ATOM | 1716 | CD2 | LEU | B | 11 | -47.976 | -32.687 | 112.003 | 1.00 | 34.11 | C |
| ATOM | 1717 | N | SER | B | 12 | -46.055 | -30.135 | 114.336 | 1.00 | 32.77 | N |
| ATOM | 1718 | CA | SER | B | 12 | -44.956 | -30.381 | 115.271 | 1.00 | 30.27 | C |
| ATOM | 1719 | C | SER | B | 12 | -44.851 | -31.873 | 115.562 | 1.00 | 30.86 | C |
| ATOM | 1720 | O | SER | B | 12 | -45.793 | -32.475 | 116.083 | 1.00 | 35.59 | O |
| ATOM | 1721 | CB | SER | B | 12 | -45.145 | -29.592 | 116.560 | 1.00 | 27.94 | C |
| ATOM | 1722 | OG | SER | B | 12 | -45.372 | -28.227 | 116.236 | 1.00 | 38.87 | O |
| ATOM | 1723 | N | LEU | B | 13 | -43.694 | -32.453 | 115.245 | 1.00 | 31.96 | N |
| ATOM | 1724 | CA | LEU | B | 13 | -43.453 | -33.887 | 115.299 | 1.00 | 32.10 | C |
| ATOM | 1725 | C | LEU | B | 13 | -42.010 | -34.098 | 115.707 | 1.00 | 30.66 | C |
| ATOM | 1726 | O | LEU | B | 13 | -41.159 | -33.231 | 115.489 | 1.00 | 31.40 | O |
| ATOM | 1727 | CB | LEU | B | 13 | -43.690 | -34.572 | 113.949 | 1.00 | 32.81 | C |
| ATOM | 1728 | CG | LEU | B | 13 | -45.087 | -34.460 | 113.332 | 1.00 | 34.52 | C |
| ATOM | 1729 | CD1 | LEU | B | 13 | -45.085 | -35.018 | 111.924 | 1.00 | 32.83 | C |
| ATOM | 1730 | CD2 | LEU | B | 13 | -46.110 | -35.164 | 114.189 | 1.00 | 26.42 | C |
| ATOM | 1731 | N | SER | B | 14 | -41.745 | -35.232 | 116.316 | 1.00 | 28.88 | N |
| ATOM | 1732 | CA | SER | B | 14 | -40.359 | -35.534 | 116.639 | 1.00 | 32.75 | C |
| ATOM | 1733 | C | SER | B | 14 | -39.649 | -36.161 | 115.442 | 1.00 | 28.56 | C |
| ATOM | 1734 | O | SER | B | 14 | -40.281 | -36.766 | 114.580 | 1.00 | 31.22 | O |
| ATOM | 1735 | CB | SER | B | 14 | -40.292 | -36.469 | 117.838 | 1.00 | 36.49 | C |
| ATOM | 1736 | OG | SER | B | 14 | -40.626 | -35.749 | 119.019 | 1.00 | 44.98 | O |
| ATOM | 1737 | N | PRO | B | 15 | -38.341 | -36.015 | 115.345 | 1.00 | 29.37 | N |
| ATOM | 1738 | CA | PRO | B | 15 | -37.609 | -36.799 | 114.341 | 1.00 | 28.36 | C |
| ATOM | 1739 | C | PRO | B | 15 | -37.846 | -38.290 | 114.566 | 1.00 | 33.54 | C |
| ATOM | 1740 | O | PRO | B | 15 | -37.877 | -38.770 | 115.702 | 1.00 | 39.74 | O |
| ATOM | 1741 | CB | PRO | B | 15 | -36.149 | -36.404 | 114.575 | 1.00 | 31.85 | C |
| ATOM | 1742 | CG | PRO | B | 15 | -36.230 | -35.042 | 115.265 | 1.00 | 27.05 | C |
| ATOM | 1743 | CD | PRO | B | 15 | -37.481 | -35.086 | 116.097 | 1.00 | 29.92 | C |
| ATOM | 1744 | N | GLY | B | 16 | -37.994 | -39.032 | 113.467 | 1.00 | 34.12 | N |
| ATOM | 1745 | CA | GLY | B | 16 | -38.362 | -40.428 | 113.500 | 1.00 | 30.27 | C |
| ATOM | 1746 | C | GLY | B | 16 | -39.837 | -40.693 | 113.284 | 1.00 | 32.66 | C |
| ATOM | 1747 | O | GLY | B | 16 | -40.204 | -41.796 | 112.862 | 1.00 | 38.35 | O |
| ATOM | 1748 | N | GLU | B | 17 | -40.690 | -39.722 | 113.549 | 1.00 | 28.58 | N |
| ATOM | 1749 | CA | GLU | B | 17 | -42.105 | -39.947 | 113.346 | 1.00 | 27.09 | C |
| ATOM | 1750 | C | GLU | B | 17 | -42.502 | -39.762 | 111.878 | 1.00 | 32.66 | C |
| ATOM | 1751 | O | GLU | B | 17 | -41.728 | -39.318 | 111.016 | 1.00 | 27.01 | O |
| ATOM | 1752 | CB | GLU | B | 17 | -42.923 | -39.013 | 114.220 | 1.00 | 26.44 | C |
| ATOM | 1753 | CG | GLU | B | 17 | -42.896 | -39.366 | 115.689 | 1.00 | 35.87 | C |
| ATOM | 1754 | CD | GLU | B | 17 | -43.872 | -38.506 | 116.494 | 1.00 | 50.33 | C |
| ATOM | 1755 | OE1 | GLU | B | 17 | -43.659 | -37.269 | 116.602 | 1.00 | 47.95 | O |
| ATOM | 1756 | OE2 | GLU | B | 17 | -44.862 | -39.066 | 117.011 | 1.00 | 67.19 | O1- |
| ATOM | 1757 | N | ARG | B | 18 | -43.757 | -40.098 | 111.626 | 1.00 | 28.24 | N |
| ATOM | 1758 | CA | ARG | B | 18 | -44.377 | -40.060 | 110.321 | 1.00 | 29.25 | C |
| ATOM | 1759 | C | ARG | B | 18 | -45.174 | -38.768 | 110.187 | 1.00 | 31.06 | C |
| ATOM | 1760 | O | ARG | B | 18 | -45.801 | -38.322 | 111.151 | 1.00 | 28.90 | O |
| ATOM | 1761 | CB | ARG | B | 18 | -45.273 | -41.278 | 110.169 | 1.00 | 28.93 | C |
| ATOM | 1762 | CG | ARG | B | 18 | -46.000 | -41.420 | 108.875 | 1.00 | 35.84 | C |
| ATOM | 1763 | CD | ARG | B | 18 | -46.955 | -42.591 | 109.004 | 1.00 | 33.74 | C |
| ATOM | 1764 | NE | ARG | B | 18 | -47.724 | -42.806 | 107.792 | 1.00 | 42.81 | N |
| ATOM | 1765 | CZ | ARG | B | 18 | -47.249 | -43.450 | 106.731 | 1.00 | 49.16 | C |
| ATOM | 1766 | NH1 | ARG | B | 18 | -45.993 | -43.927 | 106.747 | 1.00 | 44.51 | N1+ |
| ATOM | 1767 | NH2 | ARG | B | 18 | -48.021 | -43.606 | 105.656 | 1.00 | 37.48 | N |
| ATOM | 1768 | N | ALA | B | 19 | -45.104 | -38.148 | 109.001 | 1.00 | 30.81 | N |
| ATOM | 1769 | CA | ALA | B | 19 | -45.764 | -36.880 | 108.699 | 1.00 | 27.46 | C |
| ATOM | 1770 | C | ALA | B | 19 | -46.674 | -37.020 | 107.488 | 1.00 | 27.67 | C |
| ATOM | 1771 | O | ALA | B | 19 | -46.284 | -37.594 | 106.466 | 1.00 | 29.51 | O |
| ATOM | 1772 | CB | ALA | B | 19 | -44.745 | -35.775 | 108.431 | 1.00 | 26.69 | C |

| | | | | | | | | | | | |
|---|---|---|---|---|---|---|---|---|---|---|---|
| ATOM | 1773 | N | THR | B | 20 | -47.863 | -36.437 | 107.576 | 1.00 | 23.65 | N |
| ATOM | 1774 | CA | THR | B | 20 | -48.833 | -36.478 | 106.489 | 1.00 | 28.01 | C |
| ATOM | 1775 | C | THR | B | 20 | -49.374 | -35.076 | 106.258 | 1.00 | 29.74 | C |
| ATOM | 1776 | O | THR | B | 20 | -49.974 | -34.477 | 107.155 | 1.00 | 31.62 | O |
| ATOM | 1777 | CB | THR | B | 20 | -49.966 | -37.477 | 106.791 | 1.00 | 31.91 | C |
| ATOM | 1778 | OG1 | THR | B | 20 | -49.516 | -38.802 | 106.486 | 1.00 | 38.10 | O |
| ATOM | 1779 | CG2 | THR | B | 20 | -51.212 | -37.192 | 105.973 | 1.00 | 31.11 | C |
| ATOM | 1780 | N | LEU | B | 21 | -49.163 | -34.561 | 105.054 | 1.00 | 28.91 | N |
| ATOM | 1781 | CA | LEU | B | 21 | -49.541 | -33.209 | 104.693 | 1.00 | 27.86 | C |
| ATOM | 1782 | C | LEU | B | 21 | -50.689 | -33.322 | 103.711 | 1.00 | 27.21 | C |
| ATOM | 1783 | O | LEU | B | 21 | -50.742 | -34.261 | 102.921 | 1.00 | 27.15 | O |
| ATOM | 1784 | CB | LEU | B | 21 | -48.377 | -32.459 | 104.031 | 1.00 | 24.41 | C |
| ATOM | 1785 | CG | LEU | B | 21 | -46.975 | -32.345 | 104.643 | 1.00 | 26.45 | C |
| ATOM | 1786 | CD1 | LEU | B | 21 | -46.405 | -30.957 | 104.460 | 1.00 | 27.98 | C |
| ATOM | 1787 | CD2 | LEU | B | 21 | -46.871 | -32.762 | 106.081 | 1.00 | 28.98 | C |
| ATOM | 1788 | N | SER | B | 22 | -51.620 | -32.390 | 103.764 | 1.00 | 27.44 | N |
| ATOM | 1789 | CA | SER | B | 22 | -52.739 | -32.439 | 102.843 | 1.00 | 27.56 | C |
| ATOM | 1790 | C | SER | B | 22 | -52.718 | -31.231 | 101.926 | 1.00 | 29.41 | C |
| ATOM | 1791 | O | SER | B | 22 | -52.281 | -30.148 | 102.321 | 1.00 | 23.55 | O |
| ATOM | 1792 | CB | SER | B | 22 | -54.067 | -32.506 | 103.571 | 1.00 | 22.71 | C |
| ATOM | 1793 | OG | SER | B | 22 | -54.374 | -33.869 | 103.733 | 1.00 | 40.86 | O |
| ATOM | 1794 | N | CYS | B | 23 | -53.188 | -31.445 | 100.693 | 1.00 | 24.46 | N |
| ATOM | 1795 | CA | CYS | B | 23 | -53.456 | -30.379 | 99.735 | 1.00 | 26.64 | C |
| ATOM | 1796 | C | CYS | B | 23 | -54.824 | -30.660 | 99.146 | 1.00 | 26.09 | C |
| ATOM | 1797 | O | CYS | B | 23 | -55.034 | -31.718 | 98.547 | 1.00 | 30.05 | O |
| ATOM | 1798 | CB | CYS | B | 23 | -52.392 | -30.330 | 98.623 | 1.00 | 29.42 | C |
| ATOM | 1799 | SG | CYS | B | 23 | -52.509 | -28.931 | 97.438 | 1.00 | 45.36 | S |
| ATOM | 1800 | N | ARG | B | 24 | -55.756 | -29.740 | 99.326 | 1.00 | 26.38 | N |
| ATOM | 1801 | CA | ARG | B | 24 | -57.084 | -29.874 | 98.762 | 1.00 | 25.14 | C |
| ATOM | 1802 | C | ARG | B | 24 | -57.249 | -28.870 | 97.633 | 1.00 | 27.66 | C |
| ATOM | 1803 | O | ARG | B | 24 | -56.984 | -27.677 | 97.815 | 1.00 | 27.74 | O |
| ATOM | 1804 | CB | ARG | B | 24 | -58.151 | -29.702 | 99.834 | 1.00 | 25.05 | C |
| ATOM | 1805 | CG | ARG | B | 24 | -58.148 | -30.888 | 100.781 | 1.00 | 39.63 | C |
| ATOM | 1806 | CD | ARG | B | 24 | -58.919 | -30.636 | 102.071 | 1.00 | 56.87 | C |
| ATOM | 1807 | NE | ARG | B | 24 | -60.337 | -30.971 | 101.921 | 1.00 | 70.81 | N |
| ATOM | 1808 | CZ | ARG | B | 24 | -60.899 | -32.091 | 102.373 | 1.00 | 75.66 | C |
| ATOM | 1809 | NH1 | ARG | B | 24 | -60.156 | -32.994 | 103.017 | 1.00 | 74.34 | N1+ |
| ATOM | 1810 | NH2 | ARG | B | 24 | -62.204 | -32.303 | 102.187 | 1.00 | 69.31 | N |
| ATOM | 1811 | N | ALA | B | 25 | -57.657 | -29.371 | 96.465 | 1.00 | 25.97 | N |
| ATOM | 1812 | CA | ALA | B | 25 | -57.809 | -28.586 | 95.252 | 1.00 | 24.73 | C |
| ATOM | 1813 | C | ALA | B | 25 | -59.271 | -28.236 | 95.024 | 1.00 | 25.92 | C |
| ATOM | 1814 | O | ALA | B | 25 | -60.147 | -29.102 | 95.093 | 1.00 | 27.92 | O |
| ATOM | 1815 | CB | ALA | B | 25 | -57.263 | -29.344 | 94.042 | 1.00 | 25.78 | C |
| ATOM | 1816 | N | SER | B | 26 | -59.524 | -26.969 | 94.744 | 1.00 | 28.48 | N |
| ATOM | 1817 | CA | SER | B | 26 | -60.840 | -26.517 | 94.342 | 1.00 | 26.13 | C |
| ATOM | 1818 | C | SER | B | 26 | -60.758 | -25.481 | 93.190 | 1.00 | 29.42 | C |
| ATOM | 1819 | O | SER | B | 26 | -60.242 | -24.370 | 93.373 | 1.00 | 30.69 | O |
| ATOM | 1820 | CB | SER | B | 26 | -61.562 | -25.932 | 95.549 | 1.00 | 24.33 | C |
| ATOM | 1821 | OG | SER | B | 26 | -62.823 | -25.420 | 95.178 | 1.00 | 39.40 | O |
| ATOM | 1822 | N | PRO | B | 27 | -61.248 | -25.837 | 91.994 | 1.00 | 27.29 | N |
| ATOM | 1823 | CA | PRO | B | 27 | -61.824 | -27.118 | 91.541 | 1.00 | 25.37 | C |
| ATOM | 1824 | C | PRO | B | 27 | -60.819 | -28.258 | 91.496 | 1.00 | 27.72 | C |
| ATOM | 1825 | O | PRO | B | 27 | -59.639 | -28.025 | 91.702 | 1.00 | 28.26 | O |
| ATOM | 1826 | CB | PRO | B | 27 | -62.331 | -26.806 | 90.123 | 1.00 | 22.36 | C |
| ATOM | 1827 | CG | PRO | B | 27 | -61.831 | -25.420 | 89.788 | 1.00 | 21.74 | C |
| ATOM | 1828 | CD | PRO | B | 27 | -61.584 | -24.724 | 91.085 | 1.00 | 22.47 | C |
| ATOM | 1829 | N | SER | B | 28 | -61.284 | -29.468 | 91.201 | 1.00 | 29.06 | N |
| ATOM | 1830 | CA | SER | B | 28 | -60.424 | -30.643 | 91.235 | 1.00 | 29.98 | C |
| ATOM | 1831 | C | SER | B | 28 | -59.291 | -30.567 | 90.206 | 1.00 | 31.92 | C |
| ATOM | 1832 | O | SER | B | 28 | -59.390 | -29.898 | 89.174 | 1.00 | 33.40 | O |
| ATOM | 1833 | CB | SER | B | 28 | -61.256 | -31.892 | 90.993 | 1.00 | 30.66 | C |
| ATOM | 1834 | OG | SER | B | 28 | -62.051 | -32.132 | 92.124 | 1.00 | 36.68 | O |
| ATOM | 1835 | N | VAL | B | 29 | -58.219 | -31.314 | 90.488 | 1.00 | 29.10 | N |
| ATOM | 1836 | CA | VAL | B | 29 | -57.081 | -31.501 | 89.588 | 1.00 | 27.86 | C |
| ATOM | 1837 | C | VAL | B | 29 | -57.110 | -32.960 | 89.140 | 1.00 | 34.14 | C |
| ATOM | 1838 | O | VAL | B | 29 | -56.339 | -33.796 | 89.630 | 1.00 | 31.49 | O |
| ATOM | 1839 | CB | VAL | B | 29 | -55.739 | -31.126 | 90.270 | 1.00 | 26.93 | C |
| ATOM | 1840 | CG1 | VAL | B | 29 | -54.536 | -31.343 | 89.352 | 1.00 | 31.84 | C |
| ATOM | 1841 | CG2 | VAL | B | 29 | -55.741 | -29.679 | 90.699 | 1.00 | 27.83 | C |
| ATOM | 1842 | N | ASN | B | 30 | -58.040 | -33.287 | 88.234 | 1.00 | 37.48 | N |
| ATOM | 1843 | CA | ASN | B | 30 | -58.225 | -34.670 | 87.784 | 1.00 | 33.93 | C |

| | | | | | | | | | | | |
|---|---|---|---|---|---|---|---|---|---|---|---|
| ATOM | 1844 | C | ASN | B | 30 | -57.128 | -35.163 | 86.860 | 1.00 | 28.81 | C |
| ATOM | 1845 | O | ASN | B | 30 | -57.130 | -36.351 | 86.526 | 1.00 | 35.16 | O |
| ATOM | 1846 | CB | ASN | B | 30 | -59.573 | -34.847 | 87.088 | 1.00 | 30.66 | C |
| ATOM | 1847 | CG | ASN | B | 30 | -60.750 | -34.648 | 88.034 | 1.00 | 32.10 | C |
| ATOM | 1848 | OD1 | ASN | B | 30 | -60.735 | -35.110 | 89.179 | 1.00 | 36.69 | O |
| ATOM | 1849 | ND2 | ASN | B | 30 | -61.763 | -33.952 | 87.564 | 1.00 | 29.33 | N |
| ATOM | 1850 | N | SER | B | 31 | -56.223 | -34.293 | 86.406 | 1.00 | 30.18 | N |
| ATOM | 1851 | CA | SER | B | 31 | -55.082 | -34.759 | 85.625 | 1.00 | 28.46 | C |
| ATOM | 1852 | C | SER | B | 31 | -54.004 | -35.394 | 86.484 | 1.00 | 30.09 | C |
| ATOM | 1853 | O | SER | B | 31 | -53.192 | -36.163 | 85.956 | 1.00 | 25.87 | O |
| ATOM | 1854 | CB | SER | B | 31 | -54.464 | -33.608 | 84.839 | 1.00 | 28.04 | C |
| ATOM | 1855 | OG | SER | B | 31 | -54.110 | -32.544 | 85.699 | 1.00 | 28.97 | O |
| ATOM | 1856 | N | GLY | B | 32 | -54.026 | -35.129 | 87.797 | 1.00 | 30.79 | N |
| ATOM | 1857 | CA | GLY | B | 32 | -52.930 | -35.453 | 88.685 | 1.00 | 23.28 | C |
| ATOM | 1858 | C | GLY | B | 32 | -51.708 | -34.581 | 88.501 | 1.00 | 25.51 | C |
| ATOM | 1859 | O | GLY | B | 32 | -50.636 | -34.931 | 88.982 | 1.00 | 24.92 | O |
| ATOM | 1860 | N | TYR | B | 33 | -51.838 | -33.436 | 87.830 | 1.00 | 25.51 | N |
| ATOM | 1861 | CA | TYR | B | 33 | -50.697 | -32.549 | 87.607 | 1.00 | 22.85 | C |
| ATOM | 1862 | C | TYR | B | 33 | -50.468 | -31.718 | 88.875 | 1.00 | 28.41 | C |
| ATOM | 1863 | O | TYR | B | 33 | -50.739 | -30.514 | 88.938 | 1.00 | 24.46 | O |
| ATOM | 1864 | CB | TYR | B | 33 | -50.934 | -31.657 | 86.394 | 1.00 | 25.72 | C |
| ATOM | 1865 | CG | TYR | B | 33 | -50.986 | -32.351 | 85.031 | 1.00 | 27.40 | C |
| ATOM | 1866 | CD1 | TYR | B | 33 | -50.607 | -33.684 | 84.869 | 1.00 | 25.12 | C |
| ATOM | 1867 | CD2 | TYR | B | 33 | -51.398 | -31.651 | 83.901 | 1.00 | 25.37 | C |
| ATOM | 1868 | CE1 | TYR | B | 33 | -50.659 | -34.306 | 83.618 | 1.00 | 27.61 | C |
| ATOM | 1869 | CE2 | TYR | B | 33 | -51.443 | -32.256 | 82.647 | 1.00 | 28.75 | C |
| ATOM | 1870 | CZ | TYR | B | 33 | -51.073 | -33.582 | 82.505 | 1.00 | 31.43 | C |
| ATOM | 1871 | OH | TYR | B | 33 | -51.108 | -34.164 | 81.251 | 1.00 | 25.68 | O |
| ATOM | 1872 | N | LEU | B | 34 | -49.964 | -32.400 | 89.908 | 1.00 | 26.49 | N |
| ATOM | 1873 | CA | LEU | B | 34 | -49.740 | -31.801 | 91.217 | 1.00 | 25.62 | C |
| ATOM | 1874 | C | LEU | B | 34 | -48.316 | -32.075 | 91.697 | 1.00 | 25.75 | C |
| ATOM | 1875 | O | LEU | B | 34 | -47.893 | -33.228 | 91.789 | 1.00 | 27.99 | O |
| ATOM | 1876 | CB | LEU | B | 34 | -50.742 | -32.328 | 92.239 | 1.00 | 25.15 | C |
| ATOM | 1877 | CG | LEU | B | 34 | -50.732 | -31.388 | 93.436 | 1.00 | 22.33 | C |
| ATOM | 1878 | CD1 | LEU | B | 34 | -52.055 | -30.748 | 93.532 | 1.00 | 24.71 | C |
| ATOM | 1879 | CD2 | LEU | B | 34 | -50.401 | -32.127 | 94.682 | 1.00 | 22.19 | C |
| ATOM | 1880 | N | ALA | B | 35 | -47.595 | -31.021 | 92.035 | 1.00 | 26.54 | N |
| ATOM | 1881 | CA | ALA | B | 35 | -46.221 | -31.114 | 92.497 | 1.00 | 27.18 | C |
| ATOM | 1882 | C | ALA | B | 35 | -46.154 | -30.759 | 93.973 | 1.00 | 25.51 | C |
| ATOM | 1883 | O | ALA | B | 35 | -47.050 | -30.117 | 94.516 | 1.00 | 24.44 | O |
| ATOM | 1884 | CB | ALA | B | 35 | -45.293 | -30.186 | 91.706 | 1.00 | 20.44 | C |
| ATOM | 1885 | N | TRP | B | 36 | -45.088 | -31.214 | 94.621 | 1.00 | 23.22 | N |
| ATOM | 1886 | CA | TRP | B | 36 | -44.790 | -30.852 | 95.995 | 1.00 | 26.05 | C |
| ATOM | 1887 | C | TRP | B | 36 | -43.368 | -30.316 | 96.037 | 1.00 | 25.42 | C |
| ATOM | 1888 | O | TRP | B | 36 | -42.462 | -30.896 | 95.429 | 1.00 | 26.45 | O |
| ATOM | 1889 | CB | TRP | B | 36 | -44.935 | -32.050 | 96.966 | 1.00 | 23.50 | C |
| ATOM | 1890 | CG | TRP | B | 36 | -46.328 | -32.470 | 97.286 | 1.00 | 23.73 | C |
| ATOM | 1891 | CD1 | TRP | B | 36 | -47.071 | -33.413 | 96.633 | 1.00 | 26.66 | C |
| ATOM | 1892 | CD2 | TRP | B | 36 | -47.144 | -32.006 | 98.375 | 1.00 | 28.12 | C |
| ATOM | 1893 | NE1 | TRP | B | 36 | -48.304 | -33.549 | 97.229 | 1.00 | 24.58 | N |
| ATOM | 1894 | CE2 | TRP | B | 36 | -48.374 | -32.709 | 98.306 | 1.00 | 26.57 | C |
| ATOM | 1895 | CE3 | TRP | B | 36 | -46.951 | -31.079 | 99.407 | 1.00 | 20.21 | C |
| ATOM | 1896 | CZ2 | TRP | B | 36 | -49.407 | -32.506 | 99.222 | 1.00 | 22.88 | C |
| ATOM | 1897 | CZ3 | TRP | B | 36 | -47.970 | -30.872 | 100.294 | 1.00 | 23.52 | C |
| ATOM | 1898 | CH2 | TRP | B | 36 | -49.190 | -31.584 | 100.203 | 1.00 | 24.77 | C |
| ATOM | 1899 | N | TYR | B | 37 | -43.173 | -29.219 | 96.764 | 1.00 | 25.12 | N |
| ATOM | 1900 | CA | TYR | B | 37 | -41.858 | -28.628 | 96.947 | 1.00 | 23.85 | C |
| ATOM | 1901 | C | TYR | B | 37 | -41.551 | -28.525 | 98.427 | 1.00 | 27.32 | C |
| ATOM | 1902 | O | TYR | B | 37 | -42.446 | -28.279 | 99.247 | 1.00 | 28.76 | O |
| ATOM | 1903 | CB | TYR | B | 37 | -41.764 | -27.229 | 96.341 | 1.00 | 25.99 | C |
| ATOM | 1904 | CG | TYR | B | 37 | -42.052 | -27.217 | 94.879 | 1.00 | 25.68 | C |
| ATOM | 1905 | CD2 | TYR | B | 37 | -41.025 | -27.346 | 93.944 | 1.00 | 27.12 | C |
| ATOM | 1906 | CD1 | TYR | B | 37 | -43.346 | -27.106 | 94.426 | 1.00 | 20.71 | C |
| ATOM | 1907 | CE2 | TYR | B | 37 | -41.296 | -27.349 | 92.584 | 1.00 | 23.06 | C |
| ATOM | 1908 | CE1 | TYR | B | 37 | -43.622 | -27.112 | 93.088 | 1.00 | 25.67 | C |
| ATOM | 1909 | CZ | TYR | B | 37 | -42.598 | -27.241 | 92.170 | 1.00 | 22.19 | C |
| ATOM | 1910 | OH | TYR | B | 37 | -42.911 | -27.244 | 90.831 | 1.00 | 28.45 | O |
| ATOM | 1911 | N | GLN | B | 38 | -40.267 | -28.679 | 98.742 | 1.00 | 24.78 | N |
| ATOM | 1912 | CA | GLN | B | 38 | -39.708 | -28.428 | 100.061 | 1.00 | 26.12 | C |
| ATOM | 1913 | C | GLN | B | 38 | -38.871 | -27.147 | 100.017 | 1.00 | 23.99 | C |
| ATOM | 1914 | O | GLN | B | 38 | -38.168 | -26.890 | 99.034 | 1.00 | 22.68 | O |

| | | | | | | | | | | | |
|---|---|---|---|---|---|---|---|---|---|---|---|
| ATOM | 1915 | CB | GLN | B | 38 | -38.852 | -29.619 | 100.509 | 1.00 | 24.24 | C |
| ATOM | 1916 | CG | GLN | B | 38 | -38.144 | -29.399 | 101.826 | 1.00 | 22.10 | C |
| ATOM | 1917 | CD | GLN | B | 38 | -37.108 | -30.460 | 102.113 | 1.00 | 26.91 | C |
| ATOM | 1918 | OE1 | GLN | B | 38 | -36.023 | -30.439 | 101.532 | 1.00 | 33.75 | O |
| ATOM | 1919 | NE2 | GLN | B | 38 | -37.418 | -31.376 | 103.032 | 1.00 | 24.91 | N |
| ATOM | 1920 | N | GLN | B | 39 | -38.944 | -26.339 | 101.072 | 1.00 | 19.06 | N |
| ATOM | 1921 | CA | GLN | B | 39 | -38.193 | -25.085 | 101.113 | 1.00 | 24.00 | C |
| ATOM | 1922 | C | GLN | B | 39 | -37.632 | -24.846 | 102.498 | 1.00 | 23.43 | C |
| ATOM | 1923 | O | GLN | B | 39 | -38.389 | -24.722 | 103.462 | 1.00 | 21.25 | O |
| ATOM | 1924 | CB | GLN | B | 39 | -39.043 | -23.881 | 100.719 | 1.00 | 25.47 | C |
| ATOM | 1925 | CG | GLN | B | 39 | -38.226 | -22.610 | 100.620 | 1.00 | 25.34 | C |
| ATOM | 1926 | CD | GLN | B | 39 | -39.027 | -21.423 | 100.161 | 1.00 | 31.02 | C |
| ATOM | 1927 | OE1 | GLN | B | 39 | -40.183 | -21.244 | 100.538 | 1.00 | 34.04 | O |
| ATOM | 1928 | NE2 | GLN | B | 39 | -38.418 | -20.601 | 99.334 | 1.00 | 33.16 | N |
| ATOM | 1929 | N | LYS | B | 40 | -36.319 | -24.712 | 102.575 | 1.00 | 25.20 | N |
| ATOM | 1930 | CA | LYS | B | 40 | -35.694 | -24.322 | 103.822 | 1.00 | 25.84 | C |
| ATOM | 1931 | C | LYS | B | 40 | -35.497 | -22.813 | 103.870 | 1.00 | 27.20 | C |
| ATOM | 1932 | O | LYS | B | 40 | -35.478 | -22.145 | 102.827 | 1.00 | 26.37 | O |
| ATOM | 1933 | CB | LYS | B | 40 | -34.382 | -25.077 | 103.991 | 1.00 | 27.92 | C |
| ATOM | 1934 | CG | LYS | B | 40 | -34.624 | -26.487 | 104.544 | 1.00 | 30.32 | C |
| ATOM | 1935 | CD | LYS | B | 40 | -33.459 | -27.416 | 104.259 | 1.00 | 42.40 | C |
| ATOM | 1936 | CE | LYS | B | 40 | -33.761 | -28.834 | 104.704 | 1.00 | 39.36 | C |
| ATOM | 1937 | NZ | LYS | B | 40 | -33.984 | -28.911 | 106.185 | 1.00 | 42.39 | N1+ |
| ATOM | 1938 | N | PRO | B | 41 | -35.419 | -22.231 | 105.072 | 1.00 | 28.86 | N |
| ATOM | 1939 | CA | PRO | B | 41 | -35.467 | -20.757 | 105.192 | 1.00 | 27.65 | C |
| ATOM | 1940 | C | PRO | B | 41 | -34.345 | -20.077 | 104.416 | 1.00 | 27.79 | C |
| ATOM | 1941 | O | PRO | B | 41 | -33.168 | -20.420 | 104.558 | 1.00 | 30.55 | O |
| ATOM | 1942 | CB | PRO | B | 41 | -35.331 | -20.523 | 106.701 | 1.00 | 25.62 | C |
| ATOM | 1943 | CG | PRO | B | 41 | -35.861 | -21.785 | 107.321 | 1.00 | 23.53 | C |
| ATOM | 1944 | CD | PRO | B | 41 | -35.415 | -22.885 | 106.393 | 1.00 | 22.03 | C |
| ATOM | 1945 | N | GLY | B | 42 | -34.724 | -19.113 | 103.577 | 1.00 | 30.24 | N |
| ATOM | 1946 | CA | GLY | B | 42 | -33.743 | -18.444 | 102.750 | 1.00 | 26.94 | C |
| ATOM | 1947 | C | GLY | B | 42 | -33.131 | -19.294 | 101.657 | 1.00 | 34.34 | C |
| ATOM | 1948 | O | GLY | B | 42 | -32.033 | -18.976 | 101.191 | 1.00 | 29.82 | O |
| ATOM | 1949 | N | GLN | B | 43 | -33.790 | -20.388 | 101.254 | 1.00 | 29.49 | N |
| ATOM | 1950 | CA | GLN | B | 43 | -33.330 | -21.236 | 100.164 | 1.00 | 29.08 | C |
| ATOM | 1951 | C | GLN | B | 43 | -34.400 | -21.299 | 99.086 | 1.00 | 26.57 | C |
| ATOM | 1952 | O | GLN | B | 43 | -35.578 | -21.019 | 99.329 | 1.00 | 28.43 | O |
| ATOM | 1953 | CB | GLN | B | 43 | -32.994 | -22.665 | 100.622 | 1.00 | 33.38 | C |
| ATOM | 1954 | CG | GLN | B | 43 | -32.182 | -22.796 | 101.894 | 1.00 | 31.30 | C |
| ATOM | 1955 | CD | GLN | B | 43 | -30.769 | -22.262 | 101.737 | 1.00 | 45.21 | C |
| ATOM | 1956 | OE1 | GLN | B | 43 | -30.173 | -22.361 | 100.665 | 1.00 | 48.17 | O |
| ATOM | 1957 | NE2 | GLN | B | 43 | -30.215 | -21.713 | 102.818 | 1.00 | 50.07 | N |
| ATOM | 1958 | N | THR | B | 44 | -33.988 | -21.698 | 97.895 | 1.00 | 28.06 | N |
| ATOM | 1959 | CA | THR | B | 44 | -34.976 | -21.828 | 96.832 | 1.00 | 32.50 | C |
| ATOM | 1960 | C | THR | B | 44 | -35.755 | -23.142 | 97.000 | 1.00 | 29.43 | C |
| ATOM | 1961 | O | THR | B | 44 | -35.282 | -24.077 | 97.652 | 1.00 | 28.21 | O |
| ATOM | 1962 | CB | THR | B | 44 | -34.303 | -21.769 | 95.453 | 1.00 | 28.68 | C |
| ATOM | 1963 | OG1 | THR | B | 44 | -33.844 | -23.064 | 95.087 | 1.00 | 33.54 | O |
| ATOM | 1964 | CG2 | THR | B | 44 | -33.095 | -20.847 | 95.473 | 1.00 | 35.23 | C |
| ATOM | 1965 | N | PRO | B | 45 | -36.982 | -23.217 | 96.495 | 1.00 | 29.94 | N |
| ATOM | 1966 | CA | PRO | B | 45 | -37.745 | -24.456 | 96.658 | 1.00 | 27.80 | C |
| ATOM | 1967 | C | PRO | B | 45 | -37.021 | -25.614 | 95.982 | 1.00 | 24.29 | C |
| ATOM | 1968 | O | PRO | B | 45 | -36.256 | -25.428 | 95.042 | 1.00 | 28.45 | O |
| ATOM | 1969 | CB | PRO | B | 45 | -39.079 | -24.140 | 95.967 | 1.00 | 26.59 | C |
| ATOM | 1970 | CG | PRO | B | 45 | -39.175 | -22.670 | 95.983 | 1.00 | 23.14 | C |
| ATOM | 1971 | CD | PRO | B | 45 | -37.774 | -22.177 | 95.813 | 1.00 | 27.43 | C |
| ATOM | 1972 | N | ARG | B | 46 | -37.249 | -26.817 | 96.485 | 1.00 | 24.84 | N |
| ATOM | 1973 | CA | ARG | B | 46 | -36.741 | -28.036 | 95.863 | 1.00 | 27.05 | C |
| ATOM | 1974 | C | ARG | B | 46 | -37.920 | -28.935 | 95.516 | 1.00 | 28.13 | C |
| ATOM | 1975 | O | ARG | B | 46 | -38.743 | -29.258 | 96.385 | 1.00 | 27.30 | O |
| ATOM | 1976 | CB | ARG | B | 46 | -35.754 | -28.763 | 96.782 | 1.00 | 27.57 | C |
| ATOM | 1977 | CG | ARG | B | 46 | -35.323 | -30.144 | 96.305 | 1.00 | 35.27 | C |
| ATOM | 1978 | CD | ARG | B | 46 | -34.354 | -30.799 | 97.302 | 1.00 | 42.68 | C |
| ATOM | 1979 | NE | ARG | B | 46 | -33.035 | -30.146 | 97.316 | 1.00 | 59.78 | N |
| ATOM | 1980 | CZ | ARG | B | 46 | -32.623 | -29.218 | 98.197 | 1.00 | 64.38 | C |
| ATOM | 1981 | NH1 | ARG | B | 46 | -33.408 | -28.780 | 99.199 | 1.00 | 56.48 | N1+ |
| ATOM | 1982 | NH2 | ARG | B | 46 | -31.399 | -28.715 | 98.076 | 1.00 | 61.89 | N |
| ATOM | 1983 | N | LEU | B | 47 | -38.007 | -29.318 | 94.244 | 1.00 | 28.50 | N |
| ATOM | 1984 | CA | LEU | B | 47 | -39.058 | -30.216 | 93.781 | 1.00 | 25.28 | C |
| ATOM | 1985 | C | LEU | B | 47 | -38.930 | -31.576 | 94.455 | 1.00 | 25.95 | C |

| | | | | | | | | | | | |
|---|---|---|---|---|---|---|---|---|---|---|---|
| ATOM | 1986 | O | LEU | B | 47 | -37.851 | -32.175 | 94.457 | 1.00 | 30.65 | O |
| ATOM | 1987 | CB | LEU | B | 47 | -38.970 | -30.365 | 92.262 | 1.00 | 27.11 | C |
| ATOM | 1988 | CG | LEU | B | 47 | -39.935 | -31.368 | 91.643 | 1.00 | 27.81 | C |
| ATOM | 1989 | CD1 | LEU | B | 47 | -41.357 | -30.870 | 91.856 | 1.00 | 21.28 | C |
| ATOM | 1990 | CD2 | LEU | B | 47 | -39.622 | -31.533 | 90.175 | 1.00 | 24.15 | C |
| ATOM | 1991 | N | LEU | B | 48 | -40.027 | -32.055 | 95.040 | 1.00 | 22.73 | N |
| ATOM | 1992 | CA | LEU | B | 48 | -40.056 | -33.353 | 95.715 | 1.00 | 28.05 | C |
| ATOM | 1993 | C | LEU | B | 48 | -40.844 | -34.397 | 94.953 | 1.00 | 30.46 | C |
| ATOM | 1994 | O | LEU | B | 48 | -40.406 | -35.548 | 94.862 | 1.00 | 28.89 | O |
| ATOM | 1995 | CB | LEU | B | 48 | -40.685 | -33.237 | 97.112 | 1.00 | 29.74 | C |
| ATOM | 1996 | CG | LEU | B | 48 | -40.032 | -32.529 | 98.287 | 1.00 | 28.54 | C |
| ATOM | 1997 | CD1 | LEU | B | 48 | -40.954 | -32.632 | 99.462 | 1.00 | 25.93 | C |
| ATOM | 1998 | CD2 | LEU | B | 48 | -38.712 | -33.198 | 98.616 | 1.00 | 30.43 | C |
| ATOM | 1999 | N | ILE | B | 49 | -42.012 | -33.999 | 94.435 | 1.00 | 26.40 | N |
| ATOM | 2000 | CA | ILE | B | 49 | -42.988 | -34.879 | 93.804 | 1.00 | 26.75 | C |
| ATOM | 2001 | C | ILE | B | 49 | -43.560 | -34.161 | 92.594 | 1.00 | 24.70 | C |
| ATOM | 2002 | O | ILE | B | 49 | -43.809 | -32.958 | 92.648 | 1.00 | 22.99 | O |
| ATOM | 2003 | CB | ILE | B | 49 | -44.129 | -35.264 | 94.780 | 1.00 | 26.52 | C |
| ATOM | 2004 | CG1 | ILE | B | 49 | -43.583 | -35.980 | 96.018 | 1.00 | 21.26 | C |
| ATOM | 2005 | CG2 | ILE | B | 49 | -45.215 | -36.049 | 94.058 | 1.00 | 20.52 | C |
| ATOM | 2006 | CD1 | ILE | B | 49 | -43.212 | -37.429 | 95.770 | 1.00 | 22.03 | C |
| ATOM | 2007 | N | PHE | B | 50 | -43.749 | -34.888 | 91.491 | 1.00 | 26.33 | N |
| ATOM | 2008 | CA | PHE | B | 50 | -44.548 | -34.395 | 90.375 | 1.00 | 26.42 | C |
| ATOM | 2009 | C | PHE | B | 50 | -45.564 | -35.460 | 89.959 | 1.00 | 30.44 | C |
| ATOM | 2010 | O | PHE | B | 50 | -45.467 | -36.634 | 90.342 | 1.00 | 27.91 | O |
| ATOM | 2011 | CB | PHE | B | 50 | -43.678 | -33.982 | 89.184 | 1.00 | 21.93 | C |
| ATOM | 2012 | CG | PHE | B | 50 | -42.877 | -35.114 | 88.610 | 1.00 | 30.11 | C |
| ATOM | 2013 | CD1 | PHE | B | 50 | -43.407 | -35.919 | 87.609 | 1.00 | 27.24 | C |
| ATOM | 2014 | CD2 | PHE | B | 50 | -41.599 | -35.383 | 89.079 | 1.00 | 28.83 | C |
| ATOM | 2015 | CE1 | PHE | B | 50 | -42.682 | -36.965 | 87.085 | 1.00 | 34.14 | C |
| ATOM | 2016 | CE2 | PHE | B | 50 | -40.865 | -36.430 | 88.547 | 1.00 | 34.66 | C |
| ATOM | 2017 | CZ | PHE | B | 50 | -41.409 | -37.230 | 87.553 | 1.00 | 31.45 | C |
| ATOM | 2018 | N | GLY | B | 51 | -46.579 | -35.022 | 89.216 | 1.00 | 29.06 | N |
| ATOM | 2019 | CA | GLY | B | 51 | -47.640 | -35.920 | 88.790 | 1.00 | 23.55 | C |
| ATOM | 2020 | C | GLY | B | 51 | -48.357 | -36.601 | 89.926 | 1.00 | 28.78 | C |
| ATOM | 2021 | O | GLY | B | 51 | -48.771 | -37.758 | 89.789 | 1.00 | 31.77 | O |
| ATOM | 2022 | N | ALA | B | 52 | -48.497 | -35.913 | 91.057 | 1.00 | 27.54 | N |
| ATOM | 2023 | CA | ALA | B | 52 | -49.180 | -36.389 | 92.253 | 1.00 | 27.65 | C |
| ATOM | 2024 | C | ALA | B | 52 | -48.403 | -37.468 | 92.995 | 1.00 | 27.89 | C |
| ATOM | 2025 | O | ALA | B | 52 | -48.422 | -37.465 | 94.231 | 1.00 | 25.68 | O |
| ATOM | 2026 | CB | ALA | B | 52 | -50.589 | -36.907 | 91.927 | 1.00 | 24.96 | C |
| ATOM | 2027 | N | SER | B | 53 | -47.679 | -38.358 | 92.289 | 1.00 | 24.53 | N |
| ATOM | 2028 | CA | SER | B | 53 | -47.036 | -39.468 | 93.002 | 1.00 | 27.35 | C |
| ATOM | 2029 | C | SER | B | 53 | -45.606 | -39.792 | 92.588 | 1.00 | 30.40 | C |
| ATOM | 2030 | O | SER | B | 53 | -44.970 | -40.616 | 93.257 | 1.00 | 31.18 | O |
| ATOM | 2031 | CB | SER | B | 53 | -47.855 | -40.745 | 92.844 | 1.00 | 27.15 | C |
| ATOM | 2032 | OG | SER | B | 53 | -48.178 | -40.933 | 91.485 | 1.00 | 33.66 | O |
| ATOM | 2033 | N | SER | B | 54 | -45.082 | -39.207 | 91.526 | 1.00 | 27.47 | N |
| ATOM | 2034 | CA | SER | B | 54 | -43.758 | -39.568 | 91.050 | 1.00 | 28.53 | C |
| ATOM | 2035 | C | SER | B | 54 | -42.717 | -38.787 | 91.833 | 1.00 | 28.97 | C |
| ATOM | 2036 | O | SER | B | 54 | -42.754 | -37.552 | 91.877 | 1.00 | 29.74 | O |
| ATOM | 2037 | CB | SER | B | 54 | -43.621 | -39.289 | 89.556 | 1.00 | 27.68 | C |
| ATOM | 2038 | OG | SER | B | 54 | -44.449 | -40.156 | 88.815 | 1.00 | 33.67 | O |
| ATOM | 2039 | N | ARG | B | 55 | -41.795 | -39.519 | 92.432 | 1.00 | 27.26 | N |
| ATOM | 2040 | CA | ARG | B | 55 | -40.657 | -38.955 | 93.138 | 1.00 | 32.14 | C |
| ATOM | 2041 | C | ARG | B | 55 | -39.710 | -38.275 | 92.152 | 1.00 | 30.42 | C |
| ATOM | 2042 | O | ARG | B | 55 | -39.438 | -38.817 | 91.084 | 1.00 | 34.35 | O |
| ATOM | 2043 | CB | ARG | B | 55 | -39.955 | -40.099 | 93.864 | 1.00 | 28.20 | C |
| ATOM | 2044 | CG | ARG | B | 55 | -39.168 | -39.786 | 95.061 | 1.00 | 33.52 | C |
| ATOM | 2045 | CD | ARG | B | 55 | -38.583 | -41.090 | 95.651 | 1.00 | 37.14 | C |
| ATOM | 2046 | NE | ARG | B | 55 | -39.581 | -41.791 | 96.442 | 1.00 | 41.03 | N |
| ATOM | 2047 | CZ | ARG | B | 55 | -40.134 | -42.948 | 96.118 | 1.00 | 41.47 | C |
| ATOM | 2048 | NH1 | ARG | B | 55 | -41.056 | -43.474 | 96.923 | 1.00 | 39.94 | N1+ |
| ATOM | 2049 | NH2 | ARG | B | 55 | -39.756 | -43.579 | 95.009 | 1.00 | 39.66 | N |
| ATOM | 2050 | N | ALA | B | 56 | -39.246 | -37.068 | 92.483 | 1.00 | 32.10 | N |
| ATOM | 2051 | CA | ALA | B | 56 | -38.216 | -36.416 | 91.686 | 1.00 | 34.96 | C |
| ATOM | 2052 | C | ALA | B | 56 | -36.843 | -37.013 | 91.987 | 1.00 | 37.35 | C |
| ATOM | 2053 | O | ALA | B | 56 | -36.647 | -37.718 | 92.975 | 1.00 | 43.05 | O |
| ATOM | 2054 | CB | ALA | B | 56 | -38.183 | -34.911 | 91.952 | 1.00 | 30.18 | C |
| ATOM | 2055 | N | THR | B | 57 | -35.892 | -36.740 | 91.111 | 1.00 | 37.37 | N |
| ATOM | 2056 | CA | THR | B | 57 | -34.521 | -37.183 | 91.324 | 1.00 | 38.34 | C |

| | | | | | | | | | | | |
|---|---|---|---|---|---|---|---|---|---|---|---|
| ATOM | 2057 | C | THR | B | 57 | -33.836 | -36.270 | 92.313 | 1.00 | 39.91 | C |
| ATOM | 2058 | O | THR | B | 57 | -34.179 | -35.087 | 92.386 | 1.00 | 46.64 | O |
| ATOM | 2059 | CB | THR | B | 57 | -33.716 | -37.185 | 90.020 | 1.00 | 45.59 | C |
| ATOM | 2060 | OG1 | THR | B | 57 | -34.516 | -36.612 | 88.969 | 1.00 | 40.75 | O |
| ATOM | 2061 | CG2 | THR | B | 57 | -33.271 | -38.620 | 89.674 | 1.00 | 44.70 | C |
| ATOM | 2062 | N | GLY | B | 58 | -32.937 | -36.780 | 93.143 | 1.00 | 39.76 | N |
| ATOM | 2063 | CA | GLY | B | 58 | -32.957 | -38.118 | 93.653 | 1.00 | 35.82 | C |
| ATOM | 2064 | C | GLY | B | 58 | -33.485 | -37.854 | 95.061 | 1.00 | 40.59 | C |
| ATOM | 2065 | O | GLY | B | 58 | -32.728 | -37.724 | 96.021 | 1.00 | 37.19 | O |
| ATOM | 2066 | N | ILE | B | 59 | -34.798 | -37.704 | 95.160 | 1.00 | 33.85 | N |
| ATOM | 2067 | CA | ILE | B | 59 | -35.455 | -37.513 | 96.450 | 1.00 | 31.14 | C |
| ATOM | 2068 | C | ILE | B | 59 | -35.518 | -38.865 | 97.158 | 1.00 | 38.31 | C |
| ATOM | 2069 | O | ILE | B | 59 | -35.890 | -39.859 | 96.524 | 1.00 | 35.35 | O |
| ATOM | 2070 | CB | ILE | B | 59 | -36.855 | -36.904 | 96.264 | 1.00 | 29.49 | C |
| ATOM | 2071 | CG1 | ILE | B | 59 | -36.817 | -35.375 | 96.109 | 1.00 | 29.46 | C |
| ATOM | 2072 | CG2 | ILE | B | 59 | -37.756 | -37.240 | 97.420 | 1.00 | 30.78 | C |
| ATOM | 2073 | CD1 | ILE | B | 59 | -35.937 | -34.826 | 95.062 | 1.00 | 37.31 | C |
| ATOM | 2074 | N | PRO | B | 60 | -35.097 | -38.960 | 98.459 | 1.00 | 38.88 | N |
| ATOM | 2075 | CA | PRO | B | 60 | -35.157 | -40.238 | 99.191 | 1.00 | 33.20 | C |
| ATOM | 2076 | C | PRO | B | 60 | -36.530 | -40.890 | 99.128 | 1.00 | 36.41 | C |
| ATOM | 2077 | O | PRO | B | 60 | -37.527 | -40.200 | 98.889 | 1.00 | 37.79 | O |
| ATOM | 2078 | CB | PRO | B | 60 | -34.831 | -39.817 | 100.629 | 1.00 | 34.92 | C |
| ATOM | 2079 | CG | PRO | B | 60 | -34.076 | -38.600 | 100.501 | 1.00 | 30.53 | C |
| ATOM | 2080 | CD | PRO | B | 60 | -34.609 | -37.876 | 99.320 | 1.00 | 31.01 | C |
| ATOM | 2081 | N | ASP | B | 61 | -36.626 | -42.202 | 99.320 | 1.00 | 33.86 | N |
| ATOM | 2082 | CA | ASP | B | 61 | -37.968 | -42.778 | 99.248 | 1.00 | 38.89 | C |
| ATOM | 2083 | C | ASP | B | 61 | -38.762 | -42.694 | 100.558 | 1.00 | 33.94 | C |
| ATOM | 2084 | O | ASP | B | 61 | -39.868 | -43.244 | 100.610 | 1.00 | 36.31 | O |
| ATOM | 2085 | CB | ASP | B | 61 | -37.897 | -44.220 | 98.751 | 1.00 | 43.25 | C |
| ATOM | 2086 | CG | ASP | B | 61 | -36.955 | -45.052 | 99.542 | 1.00 | 47.58 | C |
| ATOM | 2087 | OD1 | ASP | B | 61 | -36.741 | -44.735 | 100.731 | 1.00 | 49.56 | O |
| ATOM | 2088 | OD2 | ASP | B | 61 | -36.427 | -46.029 | 98.972 | 1.00 | 65.96 | O1- |
| ATOM | 2089 | N | ARG | B | 62 | -38.222 | -42.061 | 101.609 | 1.00 | 32.55 | N |
| ATOM | 2090 | CA | ARG | B | 62 | -39.015 | -41.532 | 102.717 | 1.00 | 32.36 | C |
| ATOM | 2091 | C | ARG | B | 62 | -40.241 | -40.805 | 102.198 | 1.00 | 29.63 | C |
| ATOM | 2092 | O | ARG | B | 62 | -41.318 | -40.850 | 102.794 | 1.00 | 30.70 | O |
| ATOM | 2093 | CB | ARG | B | 62 | -38.243 | -40.501 | 103.536 | 1.00 | 35.05 | C |
| ATOM | 2094 | CG | ARG | B | 62 | -37.041 | -40.958 | 104.180 | 1.00 | 36.82 | C |
| ATOM | 2095 | CD | ARG | B | 62 | -36.754 | -40.043 | 105.334 | 1.00 | 38.78 | C |
| ATOM | 2096 | NE | ARG | B | 62 | -36.191 | -38.739 | 105.001 | 1.00 | 39.12 | N |
| ATOM | 2097 | CZ | ARG | B | 62 | -35.001 | -38.571 | 104.422 | 1.00 | 39.01 | C |
| ATOM | 2098 | NH1 | ARG | B | 62 | -34.289 | -39.630 | 104.056 | 1.00 | 36.63 | N1+ |
| ATOM | 2099 | NH2 | ARG | B | 62 | -34.530 | -37.352 | 104.195 | 1.00 | 33.11 | N |
| ATOM | 2100 | N | PHE | B | 63 | -40.042 | -40.074 | 101.115 | 1.00 | 31.12 | N |
| ATOM | 2101 | CA | PHE | B | 63 | -41.075 | -39.242 | 100.534 | 1.00 | 35.11 | C |
| ATOM | 2102 | C | PHE | B | 63 | -41.916 | -40.080 | 99.586 | 1.00 | 34.68 | C |
| ATOM | 2103 | O | PHE | B | 63 | -41.373 | -40.790 | 98.729 | 1.00 | 30.34 | O |
| ATOM | 2104 | CB | PHE | B | 63 | -40.444 | -38.054 | 99.803 | 1.00 | 27.94 | C |
| ATOM | 2105 | CG | PHE | B | 63 | -39.701 | -37.135 | 100.713 | 1.00 | 28.72 | C |
| ATOM | 2106 | CD1 | PHE | B | 63 | -38.408 | -37.425 | 101.099 | 1.00 | 31.44 | C |
| ATOM | 2107 | CD2 | PHE | B | 63 | -40.308 | -36.008 | 101.227 | 1.00 | 30.94 | C |
| ATOM | 2108 | CE1 | PHE | B | 63 | -37.731 | -36.592 | 101.968 | 1.00 | 33.00 | C |
| ATOM | 2109 | CE2 | PHE | B | 63 | -39.630 | -35.178 | 102.097 | 1.00 | 29.25 | C |
| ATOM | 2110 | CZ | PHE | B | 63 | -38.344 | -35.471 | 102.464 | 1.00 | 27.63 | C |
| ATOM | 2111 | N | SER | B | 64 | -43.235 | -40.023 | 99.775 | 1.00 | 30.03 | N |
| ATOM | 2112 | CA | SER | B | 64 | -44.181 | -40.581 | 98.819 | 1.00 | 30.73 | C |
| ATOM | 2113 | C | SER | B | 64 | -45.432 | -39.715 | 98.844 | 1.00 | 27.36 | C |
| ATOM | 2114 | O | SER | B | 64 | -45.684 | -38.974 | 99.796 | 1.00 | 25.57 | O |
| ATOM | 2115 | CB | SER | B | 64 | -44.501 | -42.064 | 99.109 | 1.00 | 28.95 | C |
| ATOM | 2116 | OG | SER | B | 64 | -45.445 | -42.217 | 100.156 | 1.00 | 29.24 | O |
| ATOM | 2117 | N | ALA | B | 65 | -46.221 | -39.806 | 97.784 | 1.00 | 26.92 | N |
| ATOM | 2118 | CA | ALA | B | 65 | -47.407 | -38.974 | 97.723 | 1.00 | 30.37 | C |
| ATOM | 2119 | C | ALA | B | 65 | -48.448 | -39.670 | 96.870 | 1.00 | 30.47 | C |
| ATOM | 2120 | O | ALA | B | 65 | -48.134 | -40.578 | 96.096 | 1.00 | 31.92 | O |
| ATOM | 2121 | CB | ALA | B | 65 | -47.087 | -37.583 | 97.157 | 1.00 | 26.67 | C |
| ATOM | 2122 | N | SER | B | 66 | -49.692 | -39.216 | 97.007 | 1.00 | 27.32 | N |
| ATOM | 2123 | CA | SER | B | 66 | -50.792 | -39.781 | 96.242 | 1.00 | 30.42 | C |
| ATOM | 2124 | C | SER | B | 66 | -51.978 | -38.825 | 96.278 | 1.00 | 27.86 | C |
| ATOM | 2125 | O | SER | B | 66 | -51.955 | -37.788 | 96.938 | 1.00 | 30.99 | O |
| ATOM | 2126 | CB | SER | B | 66 | -51.165 | -41.164 | 96.778 | 1.00 | 32.05 | C |
| ATOM | 2127 | OG | SER | B | 66 | -51.732 | -41.026 | 98.065 | 1.00 | 42.20 | O |

| | | | | | | | | | | | |
|---|---|---|---|---|---|---|---|---|---|---|---|
| ATOM | 2128 | N | GLY | B | 67 | -53.025 | -39.190 | 95.559 | 1.00 | 32.79 | N |
| ATOM | 2129 | CA | GLY | B | 67 | -54.256 | -38.435 | 95.552 | 1.00 | 28.74 | C |
| ATOM | 2130 | C | GLY | B | 67 | -54.669 | -38.125 | 94.138 | 1.00 | 34.13 | C |
| ATOM | 2131 | O | GLY | B | 67 | -53.835 | -38.175 | 93.223 | 1.00 | 38.32 | O |
| ATOM | 2132 | N | SER | B | 68 | -55.943 | -37.795 | 93.936 | 1.00 | 35.14 | N |
| ATOM | 2133 | CA | SER | B | 68 | -56.352 | -37.221 | 92.663 | 1.00 | 35.12 | C |
| ATOM | 2134 | C | SER | B | 68 | -57.678 | -36.522 | 92.863 | 1.00 | 35.78 | C |
| ATOM | 2135 | O | SER | B | 68 | -58.409 | -36.804 | 93.810 | 1.00 | 40.40 | O |
| ATOM | 2136 | CB | SER | B | 68 | -56.473 | -38.268 | 91.551 | 1.00 | 42.92 | C |
| ATOM | 2137 | OG | SER | B | 68 | -56.634 | -37.637 | 90.276 | 1.00 | 47.38 | O |
| ATOM | 2138 | N | GLY | B | 69 | -57.990 | -35.635 | 91.924 | 1.00 | 38.09 | N |
| ATOM | 2139 | CA | GLY | B | 69 | -59.167 | -34.812 | 91.999 | 1.00 | 27.17 | C |
| ATOM | 2140 | C | GLY | B | 69 | -59.015 | -33.747 | 93.048 | 1.00 | 30.94 | C |
| ATOM | 2141 | O | GLY | B | 69 | -58.281 | -32.775 | 92.867 | 1.00 | 32.99 | O |
| ATOM | 2142 | N | ALA | B | 70 | -59.712 | -33.901 | 94.161 | 1.00 | 36.50 | N |
| ATOM | 2143 | CA | ALA | B | 70 | -59.738 | -32.812 | 95.113 | 1.00 | 33.81 | C |
| ATOM | 2144 | C | ALA | B | 70 | -58.726 | -32.974 | 96.231 | 1.00 | 32.23 | C |
| ATOM | 2145 | O | ALA | B | 70 | -58.401 | -31.980 | 96.877 | 1.00 | 32.47 | O |
| ATOM | 2146 | CB | ALA | B | 70 | -61.137 | -32.661 | 95.704 | 1.00 | 27.91 | C |
| ATOM | 2147 | N | ASP | B | 71 | -58.216 | -34.184 | 96.471 | 1.00 | 28.53 | N |
| ATOM | 2148 | CA | ASP | B | 71 | -57.431 | -34.467 | 97.669 | 1.00 | 32.89 | C |
| ATOM | 2149 | C | ASP | B | 71 | -56.068 | -35.055 | 97.343 | 1.00 | 29.82 | C |
| ATOM | 2150 | O | ASP | B | 71 | -55.966 | -36.028 | 96.596 | 1.00 | 32.00 | O |
| ATOM | 2151 | CB | ASP | B | 71 | -58.176 | -35.394 | 98.631 | 1.00 | 29.01 | C |
| ATOM | 2152 | CG | ASP | B | 71 | -59.067 | -34.621 | 99.579 | 1.00 | 49.76 | C |
| ATOM | 2153 | OD1 | ASP | B | 71 | -58.492 | -34.037 | 100.529 | 1.00 | 51.82 | O |
| ATOM | 2154 | OD2 | ASP | B | 71 | -60.313 | -34.586 | 99.395 | 1.00 | 55.52 | O1- |
| ATOM | 2155 | N | PHE | B | 72 | -55.023 | -34.452 | 97.915 | 1.00 | 28.01 | N |
| ATOM | 2156 | CA | PHE | B | 72 | -53.651 | -34.911 | 97.756 | 1.00 | 26.76 | C |
| ATOM | 2157 | C | PHE | B | 72 | -52.964 | -34.963 | 99.110 | 1.00 | 24.59 | C |
| ATOM | 2158 | O | PHE | B | 72 | -53.217 | -34.129 | 99.987 | 1.00 | 27.45 | O |
| ATOM | 2159 | CB | PHE | B | 72 | -52.889 | -34.002 | 96.778 | 1.00 | 29.56 | C |
| ATOM | 2160 | CG | PHE | B | 72 | -53.477 | -34.000 | 95.391 | 1.00 | 29.36 | C |
| ATOM | 2161 | CD1 | PHE | B | 72 | -54.491 | -33.128 | 95.058 | 1.00 | 29.76 | C |
| ATOM | 2162 | CD2 | PHE | B | 72 | -53.050 | -34.917 | 94.441 | 1.00 | 28.24 | C |
| ATOM | 2163 | CE1 | PHE | B | 72 | -55.056 | -33.152 | 93.790 | 1.00 | 33.06 | C |
| ATOM | 2164 | CE2 | PHE | B | 72 | -53.606 | -34.935 | 93.163 | 1.00 | 29.21 | C |
| ATOM | 2165 | CZ | PHE | B | 72 | -54.619 | -34.058 | 92.846 | 1.00 | 27.62 | C |
| ATOM | 2166 | N | THR | B | 73 | -52.117 | -35.955 | 99.300 | 1.00 | 20.91 | N |
| ATOM | 2167 | CA | THR | B | 73 | -51.335 | -36.002 | 100.516 | 1.00 | 24.42 | C |
| ATOM | 2168 | C | THR | B | 73 | -49.884 | -36.323 | 100.180 | 1.00 | 25.69 | C |
| ATOM | 2169 | O | THR | B | 73 | -49.583 | -37.044 | 99.216 | 1.00 | 24.83 | O |
| ATOM | 2170 | CB | THR | B | 73 | -51.917 | -37.002 | 101.553 | 1.00 | 23.96 | C |
| ATOM | 2171 | OG1 | THR | B | 73 | -51.922 | -38.307 | 101.005 | 1.00 | 31.75 | O |
| ATOM | 2172 | CG2 | THR | B | 73 | -53.357 | -36.634 | 101.919 | 1.00 | 29.11 | C |
| ATOM | 2173 | N | LEU | B | 74 | -48.989 | -35.720 | 100.963 | 1.00 | 25.80 | N |
| ATOM | 2174 | CA | LEU | B | 74 | -47.565 | -36.023 | 100.955 | 1.00 | 26.02 | C |
| ATOM | 2175 | C | LEU | B | 74 | -47.252 | -36.738 | 102.262 | 1.00 | 25.39 | C |
| ATOM | 2176 | O | LEU | B | 74 | -47.701 | -36.303 | 103.322 | 1.00 | 25.96 | O |
| ATOM | 2177 | CB | LEU | B | 74 | -46.730 | -34.747 | 100.811 | 1.00 | 22.71 | C |
| ATOM | 2178 | CG | LEU | B | 74 | -45.217 | -34.943 | 100.997 | 1.00 | 27.97 | C |
| ATOM | 2179 | CD1 | LEU | B | 74 | -44.602 | -35.660 | 99.806 | 1.00 | 23.58 | C |
| ATOM | 2180 | CD2 | LEU | B | 74 | -44.486 | -33.635 | 101.264 | 1.00 | 25.42 | C |
| ATOM | 2181 | N | THR | B | 75 | -46.551 | -37.866 | 102.182 | 1.00 | 25.21 | N |
| ATOM | 2182 | CA | THR | B | 75 | -46.187 | -38.649 | 103.358 | 1.00 | 24.77 | C |
| ATOM | 2183 | C | THR | B | 75 | -44.676 | -38.773 | 103.458 | 1.00 | 29.94 | C |
| ATOM | 2184 | O | THR | B | 75 | -44.012 | -39.180 | 102.496 | 1.00 | 30.23 | O |
| ATOM | 2185 | CB | THR | B | 75 | -46.830 | -40.040 | 103.345 | 1.00 | 27.93 | C |
| ATOM | 2186 | OG1 | THR | B | 75 | -48.185 | -39.936 | 103.785 | 1.00 | 33.93 | O |
| ATOM | 2187 | CG2 | THR | B | 75 | -46.105 | -40.998 | 104.261 | 1.00 | 32.07 | C |
| ATOM | 2188 | N | ILE | B | 76 | -44.145 | -38.402 | 104.621 | 1.00 | 29.36 | N |
| ATOM | 2189 | CA | ILE | B | 76 | -42.760 | -38.649 | 105.003 | 1.00 | 29.13 | C |
| ATOM | 2190 | C | ILE | B | 76 | -42.807 | -39.727 | 106.074 | 1.00 | 29.72 | C |
| ATOM | 2191 | O | ILE | B | 76 | -43.436 | -39.537 | 107.122 | 1.00 | 30.62 | O |
| ATOM | 2192 | CB | ILE | B | 76 | -42.070 | -37.369 | 105.508 | 1.00 | 26.60 | C |
| ATOM | 2193 | CG1 | ILE | B | 76 | -42.336 | -36.210 | 104.551 | 1.00 | 32.41 | C |
| ATOM | 2194 | CG2 | ILE | B | 76 | -40.581 | -37.563 | 105.617 | 1.00 | 24.68 | C |
| ATOM | 2195 | CD1 | ILE | B | 76 | -41.949 | -34.861 | 105.118 | 1.00 | 27.44 | C |
| ATOM | 2196 | N | SER | B | 77 | -42.195 | -40.878 | 105.798 | 1.00 | 31.29 | N |
| ATOM | 2197 | CA | SER | B | 77 | -42.453 | -42.041 | 106.645 | 1.00 | 34.52 | C |
| ATOM | 2198 | C | SER | B | 77 | -41.647 | -42.001 | 107.937 | 1.00 | 36.39 | C |

| | | | | | | | | | | | |
|---|---|---|---|---|---|---|---|---|---|---|---|
| ATOM | 2199 | O | SER | B | 77 | -42.148 | -42.404 | 108.994 | 1.00 | 42.84 | O |
| ATOM | 2200 | CB | SER | B | 77 | -42.183 | -43.327 | 105.871 | 1.00 | 28.00 | C |
| ATOM | 2201 | OG | SER | B | 77 | -40.838 | -43.394 | 105.441 | 1.00 | 30.78 | O |
| ATOM | 2202 | N | ARG | B | 78 | -40.419 | -41.502 | 107.873 | 1.00 | 29.86 | N |
| ATOM | 2203 | CA | ARG | B | 78 | -39.567 | -41.274 | 109.032 | 1.00 | 29.69 | C |
| ATOM | 2204 | C | ARG | B | 78 | -38.973 | -39.882 | 108.882 | 1.00 | 32.92 | C |
| ATOM | 2205 | O | ARG | B | 78 | -38.317 | -39.601 | 107.879 | 1.00 | 33.41 | O |
| ATOM | 2206 | CB | ARG | B | 78 | -38.467 | -42.342 | 109.133 | 1.00 | 39.68 | C |
| ATOM | 2207 | CG | ARG | B | 78 | -37.255 | -41.895 | 109.982 | 1.00 | 45.90 | C |
| ATOM | 2208 | CD | ARG | B | 78 | -36.191 | -42.997 | 110.245 | 1.00 | 50.49 | C |
| ATOM | 2209 | NE | ARG | B | 78 | -35.401 | -42.741 | 111.473 | 1.00 | 56.24 | N |
| ATOM | 2210 | CZ | ARG | B | 78 | -35.796 | -43.031 | 112.717 | 1.00 | 51.76 | C |
| ATOM | 2211 | NH1 | ARG | B | 78 | -35.013 | -42.754 | 113.768 | 1.00 | 42.94 | N1+ |
| ATOM | 2212 | NH2 | ARG | B | 78 | -36.989 | -43.591 | 112.915 | 1.00 | 53.21 | N |
| ATOM | 2213 | N | LEU | B | 79 | -39.284 | -38.987 | 109.813 | 1.00 | 33.51 | N |
| ATOM | 2214 | CA | LEU | B | 79 | -38.785 | -37.612 | 109.757 | 1.00 | 30.48 | C |
| ATOM | 2215 | C | LEU | B | 79 | -37.289 | -37.545 | 110.092 | 1.00 | 31.79 | C |
| ATOM | 2216 | O | LEU | B | 79 | -36.893 | -37.791 | 111.233 | 1.00 | 34.10 | O |
| ATOM | 2217 | CB | LEU | B | 79 | -39.590 | -36.756 | 110.722 | 1.00 | 29.33 | C |
| ATOM | 2218 | CG | LEU | B | 79 | -40.492 | -35.683 | 110.136 | 1.00 | 29.23 | C |
| ATOM | 2219 | CD1 | LEU | B | 79 | -40.779 | -35.916 | 108.706 | 1.00 | 23.33 | C |
| ATOM | 2220 | CD2 | LEU | B | 79 | -41.769 | -35.681 | 110.920 | 1.00 | 27.74 | C |
| ATOM | 2221 | N | GLU | B | 80 | -36.439 | -37.169 | 109.109 | 1.00 | 32.68 | N |
| ATOM | 2222 | CA | GLU | B | 80 | -35.028 | -36.892 | 109.360 | 1.00 | 33.86 | C |
| ATOM | 2223 | C | GLU | B | 80 | -34.836 | -35.408 | 109.665 | 1.00 | 35.88 | C |
| ATOM | 2224 | O | GLU | B | 80 | -35.701 | -34.593 | 109.339 | 1.00 | 34.51 | O |
| ATOM | 2225 | CB | GLU | B | 80 | -34.186 | -37.294 | 108.144 | 1.00 | 36.87 | C |
| ATOM | 2226 | CG | GLU | B | 80 | -34.116 | -38.792 | 107.876 | 1.00 | 36.11 | C |
| ATOM | 2227 | CD | GLU | B | 80 | -33.480 | -39.579 | 109.018 | 1.00 | 45.93 | C |
| ATOM | 2228 | OE1 | GLU | B | 80 | -33.946 | -40.719 | 109.264 | 1.00 | 47.66 | O |
| ATOM | 2229 | OE2 | GLU | B | 80 | -32.553 | -39.050 | 109.696 | 1.00 | 47.84 | O1- |
| ATOM | 2230 | N | PRO | B | 81 | -33.719 | -35.014 | 110.302 | 1.00 | 38.88 | N |
| ATOM | 2231 | CA | PRO | B | 81 | -33.544 | -33.588 | 110.659 | 1.00 | 34.21 | C |
| ATOM | 2232 | C | PRO | B | 81 | -33.719 | -32.635 | 109.488 | 1.00 | 36.66 | C |
| ATOM | 2233 | O | PRO | B | 81 | -34.340 | -31.574 | 109.634 | 1.00 | 35.85 | O |
| ATOM | 2234 | CB | PRO | B | 81 | -32.112 | -33.540 | 111.209 | 1.00 | 41.16 | C |
| ATOM | 2235 | CG | PRO | B | 81 | -31.881 | -34.922 | 111.747 | 1.00 | 43.24 | C |
| ATOM | 2236 | CD | PRO | B | 81 | -32.600 | -35.843 | 110.790 | 1.00 | 43.50 | C |
| ATOM | 2237 | N | GLU | B | 82 | -33.190 | -32.994 | 108.325 | 1.00 | 33.34 | N |
| ATOM | 2238 | CA | GLU | B | 82 | -33.310 | -32.225 | 107.093 | 1.00 | 34.68 | C |
| ATOM | 2239 | C | GLU | B | 82 | -34.704 | -32.235 | 106.492 | 1.00 | 36.53 | C |
| ATOM | 2240 | O | GLU | B | 82 | -34.861 | -31.767 | 105.360 | 1.00 | 32.72 | O |
| ATOM | 2241 | CB | GLU | B | 82 | -32.363 | -32.799 | 106.059 | 1.00 | 37.11 | C |
| ATOM | 2242 | CG | GLU | B | 82 | -32.783 | -34.160 | 105.573 | 1.00 | 41.97 | C |
| ATOM | 2243 | CD | GLU | B | 82 | -31.777 | -35.240 | 105.930 | 1.00 | 51.14 | C |
| ATOM | 2244 | OE1 | GLU | B | 82 | -31.259 | -35.252 | 107.093 | 1.00 | 45.81 | O |
| ATOM | 2245 | OE2 | GLU | B | 82 | -31.522 | -36.080 | 105.032 | 1.00 | 60.52 | O1- |
| ATOM | 2246 | N | ASP | B | 83 | -35.691 | -32.835 | 107.151 | 1.00 | 36.25 | N |
| ATOM | 2247 | CA | ASP | B | 83 | -37.045 | -32.856 | 106.622 | 1.00 | 30.47 | C |
| ATOM | 2248 | C | ASP | B | 83 | -37.924 | -31.782 | 107.227 | 1.00 | 26.07 | C |
| ATOM | 2249 | O | ASP | B | 83 | -39.029 | -31.554 | 106.726 | 1.00 | 22.59 | O |
| ATOM | 2250 | CB | ASP | B | 83 | -37.673 | -34.235 | 106.815 | 1.00 | 28.20 | C |
| ATOM | 2251 | CG | ASP | B | 83 | -36.966 | -35.300 | 105.987 | 1.00 | 37.30 | C |
| ATOM | 2252 | OD1 | ASP | B | 83 | -36.200 | -34.897 | 105.068 | 1.00 | 38.14 | O |
| ATOM | 2253 | OD2 | ASP | B | 83 | -37.177 | -36.519 | 106.234 | 1.00 | 37.91 | O1- |
| ATOM | 2254 | N | PHE | B | 84 | -37.420 | -31.069 | 108.221 | 1.00 | 22.98 | N |
| ATOM | 2255 | CA | PHE | B | 84 | -38.174 | -30.025 | 108.897 | 1.00 | 27.33 | C |
| ATOM | 2256 | C | PHE | B | 84 | -37.971 | -28.727 | 108.122 | 1.00 | 28.99 | C |
| ATOM | 2257 | O | PHE | B | 84 | -36.861 | -28.182 | 108.083 | 1.00 | 25.03 | O |
| ATOM | 2258 | CB | PHE | B | 84 | -37.721 | -29.926 | 110.346 | 1.00 | 27.37 | C |
| ATOM | 2259 | CG | PHE | B | 84 | -37.992 | -31.172 | 111.114 | 1.00 | 29.16 | C |
| ATOM | 2260 | CD2 | PHE | B | 84 | -39.193 | -31.337 | 111.794 | 1.00 | 27.59 | C |
| ATOM | 2261 | CD1 | PHE | B | 84 | -37.076 | -32.213 | 111.100 | 1.00 | 25.70 | C |
| ATOM | 2262 | CE2 | PHE | B | 84 | -39.456 | -32.507 | 112.497 | 1.00 | 31.00 | C |
| ATOM | 2263 | CE1 | PHE | B | 84 | -37.334 | -33.386 | 111.793 | 1.00 | 31.57 | C |
| ATOM | 2264 | CZ | PHE | B | 84 | -38.527 | -33.529 | 112.505 | 1.00 | 32.40 | C |
| ATOM | 2265 | N | ALA | B | 85 | -39.048 | -28.257 | 107.494 | 1.00 | 26.46 | N |
| ATOM | 2266 | CA | ALA | B | 85 | -39.022 | -27.288 | 106.408 | 1.00 | 23.46 | C |
| ATOM | 2267 | C | ALA | B | 85 | -40.457 | -26.866 | 106.126 | 1.00 | 23.53 | C |
| ATOM | 2268 | O | ALA | B | 85 | -41.404 | -27.329 | 106.771 | 1.00 | 22.25 | O |
| ATOM | 2269 | CB | ALA | B | 85 | -38.376 | -27.893 | 105.154 | 1.00 | 21.86 | C |

| | | | | | | | | | | | |
|---|---|---|---|---|---|---|---|---|---|---|---|
| ATOM | 2270 | N | VAL | B | 86 | -40.617 | -26.039 | 105.105 | 1.00 | 23.47 | N |
| ATOM | 2271 | CA | VAL | B | 86 | -41.936 | -25.680 | 104.600 | 1.00 | 23.19 | C |
| ATOM | 2272 | C | VAL | B | 86 | -42.201 | -26.477 | 103.330 | 1.00 | 22.81 | C |
| ATOM | 2273 | O | VAL | B | 86 | -41.314 | -26.672 | 102.500 | 1.00 | 22.61 | O |
| ATOM | 2274 | CB | VAL | B | 86 | -42.053 | -24.162 | 104.366 | 1.00 | 25.35 | C |
| ATOM | 2275 | CG1 | VAL | B | 86 | -43.330 | -23.837 | 103.664 | 1.00 | 26.56 | C |
| ATOM | 2276 | CG2 | VAL | B | 86 | -42.014 | -23.424 | 105.700 | 1.00 | 19.36 | C |
| ATOM | 2277 | N | TYR | B | 87 | -43.417 | -26.967 | 103.188 | 1.00 | 23.96 | N |
| ATOM | 2278 | CA | TYR | B | 87 | -43.798 | -27.738 | 102.021 | 1.00 | 25.43 | C |
| ATOM | 2279 | C | TYR | B | 87 | -44.915 | -27.002 | 101.304 | 1.00 | 27.51 | C |
| ATOM | 2280 | O | TYR | B | 87 | -45.806 | -26.452 | 101.949 | 1.00 | 24.76 | O |
| ATOM | 2281 | CB | TYR | B | 87 | -44.216 | -29.157 | 102.418 | 1.00 | 22.13 | C |
| ATOM | 2282 | CG | TYR | B | 87 | -43.036 | -29.940 | 102.943 | 1.00 | 26.73 | C |
| ATOM | 2283 | CD1 | TYR | B | 87 | -42.575 | -29.754 | 104.253 | 1.00 | 22.18 | C |
| ATOM | 2284 | CD2 | TYR | B | 87 | -42.355 | -30.833 | 102.129 | 1.00 | 22.28 | C |
| ATOM | 2285 | CE1 | TYR | B | 87 | -41.484 | -30.435 | 104.729 | 1.00 | 21.84 | C |
| ATOM | 2286 | CE2 | TYR | B | 87 | -41.256 | -31.533 | 102.612 | 1.00 | 25.81 | C |
| ATOM | 2287 | CZ | TYR | B | 87 | -40.816 | -31.334 | 103.906 | 1.00 | 22.45 | C |
| ATOM | 2288 | OH | TYR | B | 87 | -39.716 | -32.037 | 104.374 | 1.00 | 18.88 | O |
| ATOM | 2289 | N | PHE | B | 88 | -44.823 | -26.949 | 99.973 | 1.00 | 25.00 | N |
| ATOM | 2290 | CA | PHE | B | 88 | -45.780 | -26.258 | 99.124 | 1.00 | 26.03 | C |
| ATOM | 2291 | C | PHE | B | 88 | -46.299 | -27.240 | 98.077 | 1.00 | 30.78 | C |
| ATOM | 2292 | O | PHE | B | 88 | -45.519 | -28.026 | 97.519 | 1.00 | 30.24 | O |
| ATOM | 2293 | CB | PHE | B | 88 | -45.131 | -25.063 | 98.406 | 1.00 | 27.78 | C |
| ATOM | 2294 | CG | PHE | B | 88 | -44.809 | -23.887 | 99.307 | 1.00 | 31.23 | C |
| ATOM | 2295 | CD1 | PHE | B | 88 | -45.802 | -23.008 | 99.730 | 1.00 | 30.25 | C |
| ATOM | 2296 | CD2 | PHE | B | 88 | -43.494 | -23.634 | 99.690 | 1.00 | 30.31 | C |
| ATOM | 2297 | CE1 | PHE | B | 88 | -45.493 | -21.904 | 100.539 | 1.00 | 30.57 | C |
| ATOM | 2298 | CE2 | PHE | B | 88 | -43.185 | -22.549 | 100.489 | 1.00 | 34.11 | C |
| ATOM | 2299 | CZ | PHE | B | 88 | -44.197 | -21.678 | 100.917 | 1.00 | 34.33 | C |
| ATOM | 2300 | N | CYS | B | 89 | -47.603 | -27.210 | 97.808 | 1.00 | 25.66 | N |
| ATOM | 2301 | CA | CYS | B | 89 | -48.130 | -27.930 | 96.660 | 1.00 | 24.07 | C |
| ATOM | 2302 | C | CYS | B | 89 | -48.355 | -26.953 | 95.513 | 1.00 | 23.52 | C |
| ATOM | 2303 | O | CYS | B | 89 | -48.402 | -25.739 | 95.708 | 1.00 | 21.13 | O |
| ATOM | 2304 | CB | CYS | B | 89 | -49.416 | -28.709 | 96.998 | 1.00 | 21.36 | C |
| ATOM | 2305 | SG | CYS | B | 89 | -50.748 | -27.800 | 97.719 | 1.00 | 30.20 | S |
| ATOM | 2306 | N | GLN | B | 90 | -48.451 | -27.502 | 94.302 | 1.00 | 22.98 | N |
| ATOM | 2307 | CA | GLN | B | 90 | -48.547 | -26.702 | 93.085 | 1.00 | 23.61 | C |
| ATOM | 2308 | C | GLN | B | 90 | -49.265 | -27.514 | 92.017 | 1.00 | 24.59 | C |
| ATOM | 2309 | O | GLN | B | 90 | -48.979 | -28.703 | 91.859 | 1.00 | 24.95 | O |
| ATOM | 2310 | CB | GLN | B | 90 | -47.153 | -26.272 | 92.588 | 1.00 | 23.03 | C |
| ATOM | 2311 | CG | GLN | B | 90 | -47.199 | -25.382 | 91.346 | 1.00 | 24.92 | C |
| ATOM | 2312 | CD | GLN | B | 90 | -46.481 | -25.971 | 90.137 | 1.00 | 26.13 | C |
| ATOM | 2313 | OE1 | GLN | B | 90 | -45.402 | -26.556 | 90.261 | 1.00 | 32.43 | O |
| ATOM | 2314 | NE2 | GLN | B | 90 | -47.093 | -25.847 | 88.969 | 1.00 | 23.23 | N |
| ATOM | 2315 | N | GLN | B | 91 | -50.221 | -26.897 | 91.319 | 1.00 | 22.59 | N |
| ATOM | 2316 | CA | GLN | B | 91 | -50.911 | -27.567 | 90.224 | 1.00 | 24.25 | C |
| ATOM | 2317 | C | GLN | B | 91 | -50.510 | -26.926 | 88.899 | 1.00 | 25.92 | C |
| ATOM | 2318 | O | GLN | B | 91 | -50.206 | -25.731 | 88.830 | 1.00 | 24.83 | O |
| ATOM | 2319 | CB | GLN | B | 91 | -52.449 | -27.564 | 90.405 | 1.00 | 19.74 | C |
| ATOM | 2320 | CG | GLN | B | 91 | -53.125 | -26.219 | 90.299 | 1.00 | 21.60 | C |
| ATOM | 2321 | CD | GLN | B | 91 | -53.417 | -25.811 | 88.871 | 1.00 | 24.32 | C |
| ATOM | 2322 | OE1 | GLN | B | 91 | -53.515 | -26.657 | 87.975 | 1.00 | 23.50 | O |
| ATOM | 2323 | NE2 | GLN | B | 91 | -53.561 | -24.499 | 88.648 | 1.00 | 23.44 | N |
| ATOM | 2324 | N | TYR | B | 92 | -50.462 | -27.737 | 87.850 | 1.00 | 24.33 | N |
| ATOM | 2325 | CA | TYR | B | 92 | -50.099 | -27.241 | 86.524 | 1.00 | 26.73 | C |
| ATOM | 2326 | C | TYR | B | 92 | -51.027 | -27.842 | 85.493 | 1.00 | 27.55 | C |
| ATOM | 2327 | O | TYR | B | 92 | -50.626 | -28.137 | 84.362 | 1.00 | 29.60 | O |
| ATOM | 2328 | CB | TYR | B | 92 | -48.610 | -27.501 | 86.194 | 1.00 | 23.03 | C |
| ATOM | 2329 | CG | TYR | B | 92 | -48.133 | -28.903 | 86.494 | 1.00 | 22.43 | C |
| ATOM | 2330 | CD1 | TYR | B | 92 | -47.776 | -29.257 | 87.789 | 1.00 | 22.77 | C |
| ATOM | 2331 | CD2 | TYR | B | 92 | -48.036 | -29.874 | 85.496 | 1.00 | 22.32 | C |
| ATOM | 2332 | CE1 | TYR | B | 92 | -47.349 | -30.533 | 88.095 | 1.00 | 22.69 | C |
| ATOM | 2333 | CE2 | TYR | B | 92 | -47.589 | -31.154 | 85.788 | 1.00 | 21.88 | C |
| ATOM | 2334 | CZ | TYR | B | 92 | -47.254 | -31.478 | 87.096 | 1.00 | 23.71 | C |
| ATOM | 2335 | OH | TYR | B | 92 | -46.820 | -32.729 | 87.444 | 1.00 | 20.44 | O |
| ATOM | 2336 | N | GLU | B | 93 | -52.289 | -28.031 | 85.880 | 1.00 | 27.99 | N |
| ATOM | 2337 | CA | GLU | B | 93 | -53.312 | -28.512 | 84.958 | 1.00 | 30.82 | C |
| ATOM | 2338 | C | GLU | B | 93 | -53.999 | -27.364 | 84.244 | 1.00 | 31.11 | C |
| ATOM | 2339 | O | GLU | B | 93 | -54.182 | -27.424 | 83.026 | 1.00 | 30.30 | O |
| ATOM | 2340 | CB | GLU | B | 93 | -54.360 | -29.364 | 85.691 | 1.00 | 30.98 | C |

| | | | | | | | | | | | |
|---|---|---|---|---|---|---|---|---|---|---|---|
| ATOM | 2341 | CG | GLU | B | 93 | -55.690 | -29.496 | 84.939 | 1.00 | 25.68 | C |
| ATOM | 2342 | CD | GLU | B | 93 | -56.678 | -30.425 | 85.629 | 1.00 | 33.12 | C |
| ATOM | 2343 | OE1 | GLU | B | 93 | -56.247 | -31.498 | 86.123 | 1.00 | 31.54 | O |
| ATOM | 2344 | OE2 | GLU | B | 93 | -57.889 | -30.090 | 85.680 | 1.00 | 32.74 | O1- |
| ATOM | 2345 | N | SER | B | 94 | -54.366 | -26.310 | 84.970 | 1.00 | 30.35 | N |
| ATOM | 2346 | CA | SER | B | 94 | -55.097 | -25.199 | 84.378 | 1.00 | 32.23 | C |
| ATOM | 2347 | C | SER | B | 94 | -54.388 | -23.888 | 84.703 | 1.00 | 28.38 | C |
| ATOM | 2348 | O | SER | B | 94 | -54.044 | -23.617 | 85.861 | 1.00 | 23.42 | O |
| ATOM | 2349 | CB | SER | B | 94 | -56.556 | -25.171 | 84.862 | 1.00 | 32.62 | C |
| ATOM | 2350 | OG | SER | B | 94 | -57.144 | -23.891 | 84.656 | 1.00 | 44.26 | O |
| ATOM | 2351 | N | SER | B | 95 | -54.180 | -23.077 | 83.678 | 1.00 | 29.26 | N |
| ATOM | 2352 | CA | SER | B | 95 | -53.498 | -21.814 | 83.854 | 1.00 | 28.94 | C |
| ATOM | 2353 | C | SER | B | 95 | -54.464 | -20.862 | 84.524 | 1.00 | 28.67 | C |
| ATOM | 2354 | O | SER | B | 95 | -55.645 | -20.897 | 84.219 | 1.00 | 29.79 | O |
| ATOM | 2355 | CB | SER | B | 95 | -53.015 | -21.271 | 82.514 | 1.00 | 23.10 | C |
| ATOM | 2356 | OG | SER | B | 95 | -52.370 | -20.022 | 82.690 | 1.00 | 38.38 | O |
| ATOM | 2357 | N | PRO | B | 96 | -53.976 | -20.029 | 85.458 | 1.00 | 27.09 | N |
| ATOM | 2358 | CA | PRO | B | 96 | -52.599 | -19.892 | 85.956 | 1.00 | 26.26 | C |
| ATOM | 2359 | C | PRO | B | 96 | -52.131 | -21.034 | 86.857 | 1.00 | 27.92 | C |
| ATOM | 2360 | O | PRO | B | 96 | -52.933 | -21.550 | 87.627 | 1.00 | 31.29 | O |
| ATOM | 2361 | CB | PRO | B | 96 | -52.658 | -18.601 | 86.768 | 1.00 | 25.28 | C |
| ATOM | 2362 | CG | PRO | B | 96 | -54.047 | -18.557 | 87.258 | 1.00 | 27.55 | C |
| ATOM | 2363 | CD | PRO | B | 96 | -54.892 | -19.111 | 86.148 | 1.00 | 23.84 | C |
| ATOM | 2364 | N | TRP | B | 97 | -50.858 | -21.419 | 86.786 | 1.00 | 26.30 | N |
| ATOM | 2365 | CA | TRP | B | 97 | -50.330 | -22.305 | 87.811 | 1.00 | 26.57 | C |
| ATOM | 2366 | C | TRP | B | 97 | -50.524 | -21.661 | 89.178 | 1.00 | 25.80 | C |
| ATOM | 2367 | O | TRP | B | 97 | -50.307 | -20.460 | 89.358 | 1.00 | 25.76 | O |
| ATOM | 2368 | CB | TRP | B | 97 | -48.847 | -22.617 | 87.594 | 1.00 | 25.63 | C |
| ATOM | 2369 | CG | TRP | B | 97 | -48.411 | -23.337 | 86.309 | 1.00 | 28.94 | C |
| ATOM | 2370 | CD1 | TRP | B | 97 | -47.124 | -23.541 | 85.926 | 1.00 | 29.48 | C |
| ATOM | 2371 | CD2 | TRP | B | 97 | -49.229 | -23.928 | 85.264 | 1.00 | 29.78 | C |
| ATOM | 2372 | NE1 | TRP | B | 97 | -47.073 | -24.216 | 84.733 | 1.00 | 32.61 | N |
| ATOM | 2373 | CE2 | TRP | B | 97 | -48.344 | -24.459 | 84.296 | 1.00 | 28.86 | C |
| ATOM | 2374 | CE3 | TRP | B | 97 | -50.608 | -24.072 | 85.061 | 1.00 | 29.91 | C |
| ATOM | 2375 | CZ2 | TRP | B | 97 | -48.789 | -25.116 | 83.134 | 1.00 | 31.43 | C |
| ATOM | 2376 | CZ3 | TRP | B | 97 | -51.054 | -24.724 | 83.902 | 1.00 | 28.75 | C |
| ATOM | 2377 | CH2 | TRP | B | 97 | -50.140 | -25.232 | 82.951 | 1.00 | 31.01 | C |
| ATOM | 2378 | N | THR | B | 98 | -50.931 | -22.460 | 90.148 | 1.00 | 21.99 | N |
| ATOM | 2379 | CA | THR | B | 98 | -51.167 | -21.932 | 91.472 | 1.00 | 23.81 | C |
| ATOM | 2380 | C | THR | B | 98 | -50.474 | -22.819 | 92.488 | 1.00 | 23.94 | C |
| ATOM | 2381 | O | THR | B | 98 | -50.148 | -23.973 | 92.215 | 1.00 | 25.95 | O |
| ATOM | 2382 | CB | THR | B | 98 | -52.674 | -21.787 | 91.782 | 1.00 | 26.18 | C |
| ATOM | 2383 | OG1 | THR | B | 98 | -53.374 | -23.006 | 91.498 | 1.00 | 23.15 | O |
| ATOM | 2384 | CG2 | THR | B | 98 | -53.270 | -20.618 | 90.969 | 1.00 | 24.44 | C |
| ATOM | 2385 | N | PHE | B | 99 | -50.184 | -22.209 | 93.633 | 1.00 | 28.63 | N |
| ATOM | 2386 | CA | PHE | B | 99 | -49.511 | -22.803 | 94.776 | 1.00 | 26.82 | C |
| ATOM | 2387 | C | PHE | B | 99 | -50.436 | -22.773 | 95.988 | 1.00 | 29.46 | C |
| ATOM | 2388 | O | PHE | B | 99 | -51.311 | -21.912 | 96.089 | 1.00 | 31.15 | O |
| ATOM | 2389 | CB | PHE | B | 99 | -48.236 | -22.020 | 95.121 | 1.00 | 27.58 | C |
| ATOM | 2390 | CG | PHE | B | 99 | -47.123 | -22.166 | 94.115 | 1.00 | 26.17 | C |
| ATOM | 2391 | CD1 | PHE | B | 99 | -46.212 | -23.210 | 94.213 | 1.00 | 21.23 | C |
| ATOM | 2392 | CD2 | PHE | B | 99 | -46.970 | -21.236 | 93.096 | 1.00 | 26.56 | C |
| ATOM | 2393 | CE1 | PHE | B | 99 | -45.179 | -23.346 | 93.313 | 1.00 | 23.45 | C |
| ATOM | 2394 | CE2 | PHE | B | 99 | -45.935 | -21.362 | 92.177 | 1.00 | 28.21 | C |
| ATOM | 2395 | CZ | PHE | B | 99 | -45.028 | -22.420 | 92.291 | 1.00 | 25.30 | C |
| ATOM | 2396 | N | GLY | B | 100 | -50.252 | -23.722 | 96.909 | 1.00 | 29.01 | N |
| ATOM | 2397 | CA | GLY | B | 100 | -50.849 | -23.604 | 98.224 | 1.00 | 26.65 | C |
| ATOM | 2398 | C | GLY | B | 100 | -50.062 | -22.660 | 99.136 | 1.00 | 32.15 | C |
| ATOM | 2399 | O | GLY | B | 100 | -48.953 | -22.224 | 98.831 | 1.00 | 30.70 | O |
| ATOM | 2400 | N | GLN | B | 101 | -50.651 | -22.339 | 100.291 | 1.00 | 32.09 | N |
| ATOM | 2401 | CA | GLN | B | 101 | -49.953 | -21.455 | 101.219 | 1.00 | 27.63 | C |
| ATOM | 2402 | C | GLN | B | 101 | -48.788 | -22.134 | 101.926 | 1.00 | 31.28 | C |
| ATOM | 2403 | O | GLN | B | 101 | -47.993 | -21.430 | 102.555 | 1.00 | 31.67 | O |
| ATOM | 2404 | CB | GLN | B | 101 | -50.888 | -20.871 | 102.296 | 1.00 | 31.00 | C |
| ATOM | 2405 | CG | GLN | B | 101 | -52.371 | -21.297 | 102.285 | 1.00 | 41.04 | C |
| ATOM | 2406 | CD | GLN | B | 101 | -52.581 | -22.771 | 102.623 | 1.00 | 41.89 | C |
| ATOM | 2407 | OE1 | GLN | B | 101 | -52.927 | -23.567 | 101.736 | 1.00 | 36.09 | O |
| ATOM | 2408 | NE2 | GLN | B | 101 | -52.365 | -23.147 | 103.900 | 1.00 | 37.62 | N |
| ATOM | 2409 | N | GLY | B | 102 | -48.665 | -23.453 | 101.858 | 1.00 | 26.43 | N |
| ATOM | 2410 | CA | GLY | B | 102 | -47.548 | -24.085 | 102.535 | 1.00 | 24.50 | C |
| ATOM | 2411 | C | GLY | B | 102 | -47.895 | -24.678 | 103.898 | 1.00 | 27.19 | C |

| | | | | | | | | | | | |
|---|---|---|---|---|---|---|---|---|---|---|---|
| ATOM | 2412 | O | GLY | B | 102 | -48.800 | -24.215 | 104.600 | 1.00 | 27.42 | O |
| ATOM | 2413 | N | THR | B | 103 | -47.158 | -25.724 | 104.283 | 1.00 | 24.40 | N |
| ATOM | 2414 | CA | THR | B | 103 | -47.207 | -26.276 | 105.634 | 1.00 | 27.22 | C |
| ATOM | 2415 | C | THR | B | 103 | -45.806 | -26.259 | 106.239 | 1.00 | 25.76 | C |
| ATOM | 2416 | O | THR | B | 103 | -44.857 | -26.746 | 105.621 | 1.00 | 27.96 | O |
| ATOM | 2417 | CB | THR | B | 103 | -47.752 | -27.718 | 105.647 | 1.00 | 28.05 | C |
| ATOM | 2418 | OG1 | THR | B | 103 | -49.120 | -27.729 | 105.232 | 1.00 | 31.71 | O |
| ATOM | 2419 | CG2 | THR | B | 103 | -47.654 | -28.348 | 107.061 | 1.00 | 25.08 | C |
| ATOM | 2420 | N | LYS | B | 104 | -45.673 | -25.709 | 107.442 | 1.00 | 22.08 | N |
| ATOM | 2421 | CA | LYS | B | 104 | -44.409 | -25.758 | 108.159 | 1.00 | 26.19 | C |
| ATOM | 2422 | C | LYS | B | 104 | -44.360 | -27.021 | 109.014 | 1.00 | 27.07 | C |
| ATOM | 2423 | O | LYS | B | 104 | -45.205 | -27.212 | 109.889 | 1.00 | 28.44 | O |
| ATOM | 2424 | CB | LYS | B | 104 | -44.212 | -24.523 | 109.032 | 1.00 | 25.21 | C |
| ATOM | 2425 | CG | LYS | B | 104 | -42.846 | -24.510 | 109.709 | 1.00 | 26.78 | C |
| ATOM | 2426 | CD | LYS | B | 104 | -42.677 | -23.365 | 110.699 | 1.00 | 30.43 | C |
| ATOM | 2427 | CE | LYS | B | 104 | -41.301 | -23.463 | 111.391 | 1.00 | 45.22 | C |
| ATOM | 2428 | NZ | LYS | B | 104 | -40.967 | -22.316 | 112.301 | 1.00 | 44.27 | N1+ |
| ATOM | 2429 | N | VAL | B | 105 | -43.366 | -27.869 | 108.779 | 1.00 | 25.76 | N |
| ATOM | 2430 | CA | VAL | B | 105 | -43.119 | -29.033 | 109.624 | 1.00 | 29.32 | C |
| ATOM | 2431 | C | VAL | B | 105 | -42.005 | -28.664 | 110.597 | 1.00 | 27.51 | C |
| ATOM | 2432 | O | VAL | B | 105 | -40.841 | -28.516 | 110.211 | 1.00 | 25.98 | O |
| ATOM | 2433 | CB | VAL | B | 105 | -42.757 | -30.283 | 108.813 | 1.00 | 24.39 | C |
| ATOM | 2434 | CG1 | VAL | B | 105 | -42.488 | -31.445 | 109.776 | 1.00 | 26.12 | C |
| ATOM | 2435 | CG2 | VAL | B | 105 | -43.868 | -30.636 | 107.890 | 1.00 | 21.86 | C |
| ATOM | 2436 | N | GLU | B | 106 | -42.367 | -28.533 | 111.863 | 1.00 | 31.16 | N |
| ATOM | 2437 | CA | GLU | B | 106 | -41.473 | -28.092 | 112.919 | 1.00 | 28.93 | C |
| ATOM | 2438 | C | GLU | B | 106 | -41.144 | -29.242 | 113.875 | 1.00 | 33.11 | C |
| ATOM | 2439 | O | GLU | B | 106 | -41.922 | -30.197 | 114.030 | 1.00 | 30.26 | O |
| ATOM | 2440 | CB | GLU | B | 106 | -42.130 | -26.943 | 113.669 | 1.00 | 31.52 | C |
| ATOM | 2441 | CG | GLU | B | 106 | -41.324 | -26.435 | 114.830 | 1.00 | 41.69 | C |
| ATOM | 2442 | CD | GLU | B | 106 | -42.170 | -26.121 | 116.028 | 1.00 | 37.31 | C |
| ATOM | 2443 | OE1 | GLU | B | 106 | -42.903 | -27.027 | 116.485 | 1.00 | 37.32 | O |
| ATOM | 2444 | OE2 | GLU | B | 106 | -42.102 | -24.963 | 116.492 | 1.00 | 39.39 | O1- |
| ATOM | 2445 | N | ILE | B | 107 | -39.971 | -29.149 | 114.495 | 1.00 | 26.80 | N |
| ATOM | 2446 | CA | ILE | B | 107 | -39.486 | -30.168 | 115.421 | 1.00 | 28.39 | C |
| ATOM | 2447 | C | ILE | B | 107 | -40.205 | -30.038 | 116.755 | 1.00 | 32.03 | C |
| ATOM | 2448 | O | ILE | B | 107 | -40.118 | -29.003 | 117.420 | 1.00 | 27.69 | O |
| ATOM | 2449 | CB | ILE | B | 107 | -37.974 | -30.050 | 115.622 | 1.00 | 26.88 | C |
| ATOM | 2450 | CG1 | ILE | B | 107 | -37.240 | -30.423 | 114.344 | 1.00 | 27.26 | C |
| ATOM | 2451 | CG2 | ILE | B | 107 | -37.552 | -30.898 | 116.793 | 1.00 | 26.83 | C |
| ATOM | 2452 | CD1 | ILE | B | 107 | -35.799 | -30.043 | 114.347 | 1.00 | 30.15 | C |
| ATOM | 2453 | N | LYS | B | 108 | -40.866 | -31.106 | 117.182 | 1.00 | 31.78 | N |
| ATOM | 2454 | CA | LYS | B | 108 | -41.455 | -31.110 | 118.512 | 1.00 | 32.40 | C |
| ATOM | 2455 | C | LYS | B | 108 | -40.382 | -31.497 | 119.529 | 1.00 | 32.97 | C |
| ATOM | 2456 | O | LYS | B | 108 | -39.654 | -32.475 | 119.336 | 1.00 | 33.78 | O |
| ATOM | 2457 | CB | LYS | B | 108 | -42.649 | -32.069 | 118.567 | 1.00 | 34.38 | C |
| ATOM | 2458 | CG | LYS | B | 108 | -43.460 | -32.006 | 119.864 | 1.00 | 35.60 | C |
| ATOM | 2459 | CD | LYS | B | 108 | -44.379 | -33.223 | 120.000 | 1.00 | 34.87 | C |
| ATOM | 2460 | CE | LYS | B | 108 | -45.671 | -32.857 | 120.690 | 1.00 | 39.16 | C |
| ATOM | 2461 | NZ | LYS | B | 108 | -45.560 | -31.457 | 121.228 | 1.00 | 41.36 | N1+ |
| ATOM | 2462 | N | ARG | B | 109 | -40.270 | -30.720 | 120.604 | 1.00 | 30.43 | N |
| ATOM | 2463 | CA | ARG | B | 109 | -39.298 | -30.996 | 121.651 | 1.00 | 27.95 | C |
| ATOM | 2464 | C | ARG | B | 109 | -39.942 | -30.659 | 122.985 | 1.00 | 29.25 | C |
| ATOM | 2465 | O | ARG | B | 109 | -41.117 | -30.284 | 123.040 | 1.00 | 33.46 | O |
| ATOM | 2466 | CB | ARG | B | 109 | -37.993 | -30.224 | 121.445 | 1.00 | 27.54 | C |
| ATOM | 2467 | CG | ARG | B | 109 | -38.128 | -28.705 | 121.378 | 1.00 | 29.91 | C |
| ATOM | 2468 | CD | ARG | B | 109 | -36.873 | -28.023 | 121.928 | 1.00 | 26.11 | C |
| ATOM | 2469 | NE | ARG | B | 109 | -36.853 | -28.173 | 123.386 | 1.00 | 32.71 | N |
| ATOM | 2470 | CZ | ARG | B | 109 | -35.751 | -28.189 | 124.126 | 1.00 | 27.84 | C |
| ATOM | 2471 | NH1 | ARG | B | 109 | -35.827 | -28.351 | 125.449 | 1.00 | 24.67 | N1+ |
| ATOM | 2472 | NH2 | ARG | B | 109 | -34.577 | -28.039 | 123.536 | 1.00 | 23.42 | N |
| ATOM | 2473 | N | THR | B | 110 | -39.191 | -30.838 | 124.073 | 1.00 | 27.37 | N |
| ATOM | 2474 | CA | THR | B | 110 | -39.762 | -30.554 | 125.385 | 1.00 | 28.62 | C |
| ATOM | 2475 | C | THR | B | 110 | -39.775 | -29.056 | 125.631 | 1.00 | 29.53 | C |
| ATOM | 2476 | O | THR | B | 110 | -38.878 | -28.311 | 125.203 | 1.00 | 26.75 | O |
| ATOM | 2477 | CB | THR | B | 110 | -39.006 | -31.227 | 126.546 | 1.00 | 27.19 | C |
| ATOM | 2478 | OG1 | THR | B | 110 | -37.641 | -30.803 | 126.570 | 1.00 | 31.87 | O |
| ATOM | 2479 | CG2 | THR | B | 110 | -39.052 | -32.727 | 126.445 | 1.00 | 25.84 | C |
| ATOM | 2480 | N | VAL | B | 111 | -40.796 | -28.630 | 126.367 | 1.00 | 31.89 | N |
| ATOM | 2481 | CA | VAL | B | 111 | -40.905 | -27.236 | 126.752 | 1.00 | 33.07 | C |
| ATOM | 2482 | C | VAL | B | 111 | -39.618 | -26.751 | 127.407 | 1.00 | 31.48 | C |

| | | | | | | | | | | | |
|---|---|---|---|---|---|---|---|---|---|---|---|
| ATOM | 2483 | O | VAL | B | 111 | -38.982 | -27.463 | 128.195 | 1.00 | 31.62 | O |
| ATOM | 2484 | CB | VAL | B | 111 | -42.112 | -27.056 | 127.683 | 1.00 | 30.76 | C |
| ATOM | 2485 | CG1 | VAL | B | 111 | -42.176 | -25.612 | 128.149 | 1.00 | 27.34 | C |
| ATOM | 2486 | CG2 | VAL | B | 111 | -43.381 | -27.478 | 126.949 | 1.00 | 24.38 | C |
| ATOM | 2487 | N | ALA | B | 112 | -39.223 | -25.532 | 127.044 | 1.00 | 29.57 | N |
| ATOM | 2488 | CA | ALA | B | 112 | -38.047 | -24.863 | 127.582 | 1.00 | 25.09 | C |
| ATOM | 2489 | C | ALA | B | 112 | -38.425 | -23.405 | 127.788 | 1.00 | 30.16 | C |
| ATOM | 2490 | O | ALA | B | 112 | -38.831 | -22.734 | 126.835 | 1.00 | 27.80 | O |
| ATOM | 2491 | CB | ALA | B | 112 | -36.844 | -24.984 | 126.639 | 1.00 | 23.34 | C |
| ATOM | 2492 | N | ALA | B | 113 | -38.318 | -22.928 | 129.029 | 1.00 | 32.01 | N |
| ATOM | 2493 | CA | ALA | B | 113 | -38.663 | -21.549 | 129.326 | 1.00 | 28.03 | C |
| ATOM | 2494 | C | ALA | B | 113 | -37.579 | -20.640 | 128.768 | 1.00 | 29.40 | C |
| ATOM | 2495 | O | ALA | B | 113 | -36.412 | -21.035 | 128.710 | 1.00 | 31.93 | O |
| ATOM | 2496 | CB | ALA | B | 113 | -38.799 | -21.325 | 130.830 | 1.00 | 23.97 | C |
| ATOM | 2497 | N | PRO | B | 114 | -37.934 | -19.422 | 128.353 | 1.00 | 27.52 | N |
| ATOM | 2498 | CA | PRO | B | 114 | -36.911 | -18.489 | 127.860 | 1.00 | 27.42 | C |
| ATOM | 2499 | C | PRO | B | 114 | -36.095 | -17.920 | 129.004 | 1.00 | 27.03 | C |
| ATOM | 2500 | O | PRO | B | 114 | -36.590 | -17.719 | 130.108 | 1.00 | 29.23 | O |
| ATOM | 2501 | CB | PRO | B | 114 | -37.731 | -17.386 | 127.186 | 1.00 | 27.64 | C |
| ATOM | 2502 | CG | PRO | B | 114 | -39.012 | -17.376 | 127.998 | 1.00 | 27.54 | C |
| ATOM | 2503 | CD | PRO | B | 114 | -39.274 | -18.806 | 128.403 | 1.00 | 24.48 | C |
| ATOM | 2504 | N | SER | B | 115 | -34.835 | -17.635 | 128.730 | 1.00 | 27.91 | N |
| ATOM | 2505 | CA | SER | B | 115 | -34.092 | -16.739 | 129.592 | 1.00 | 25.63 | C |
| ATOM | 2506 | C | SER | B | 115 | -34.236 | -15.331 | 129.028 | 1.00 | 25.72 | C |
| ATOM | 2507 | O | SER | B | 115 | -34.238 | -15.130 | 127.814 | 1.00 | 27.20 | O |
| ATOM | 2508 | CB | SER | B | 115 | -32.628 | -17.157 | 129.726 | 1.00 | 24.15 | C |
| ATOM | 2509 | OG | SER | B | 115 | -32.139 | -17.720 | 128.535 | 1.00 | 40.34 | O |
| ATOM | 2510 | N | VAL | B | 116 | -34.430 | -14.371 | 129.922 | 1.00 | 26.54 | N |
| ATOM | 2511 | CA | VAL | B | 116 | -34.857 | -13.023 | 129.579 | 1.00 | 28.89 | C |
| ATOM | 2512 | C | VAL | B | 116 | -33.762 | -12.037 | 129.979 | 1.00 | 26.93 | C |
| ATOM | 2513 | O | VAL | B | 116 | -33.243 | -12.093 | 131.094 | 1.00 | 31.62 | O |
| ATOM | 2514 | CB | VAL | B | 116 | -36.203 | -12.695 | 130.259 | 1.00 | 28.35 | C |
| ATOM | 2515 | CG1 | VAL | B | 116 | -36.709 | -11.313 | 129.860 | 1.00 | 28.35 | C |
| ATOM | 2516 | CG2 | VAL | B | 116 | -37.212 | -13.740 | 129.882 | 1.00 | 18.89 | C |
| ATOM | 2517 | N | PHE | B | 117 | -33.406 | -11.151 | 129.063 | 1.00 | 25.01 | N |
| ATOM | 2518 | CA | PHE | B | 117 | -32.424 | -10.118 | 129.311 | 1.00 | 25.46 | C |
| ATOM | 2519 | C | PHE | B | 117 | -32.963 | -8.826 | 128.726 | 1.00 | 28.62 | C |
| ATOM | 2520 | O | PHE | B | 117 | -33.533 | -8.832 | 127.632 | 1.00 | 31.30 | O |
| ATOM | 2521 | CB | PHE | B | 117 | -31.081 | -10.413 | 128.650 | 1.00 | 25.62 | C |
| ATOM | 2522 | CG | PHE | B | 117 | -30.536 | -11.799 | 128.892 | 1.00 | 29.84 | C |
| ATOM | 2523 | CD1 | PHE | B | 117 | -30.935 | -12.876 | 128.093 | 1.00 | 24.22 | C |
| ATOM | 2524 | CD2 | PHE | B | 117 | -29.542 | -12.015 | 129.835 | 1.00 | 31.65 | C |
| ATOM | 2525 | CE1 | PHE | B | 117 | -30.405 | -14.136 | 128.283 | 1.00 | 22.90 | C |
| ATOM | 2526 | CE2 | PHE | B | 117 | -28.998 | -13.290 | 130.013 | 1.00 | 29.63 | C |
| ATOM | 2527 | CZ | PHE | B | 117 | -29.430 | -14.339 | 129.234 | 1.00 | 24.06 | C |
| ATOM | 2528 | N | ILE | B | 118 | -32.755 | -7.717 | 129.428 | 1.00 | 28.33 | N |
| ATOM | 2529 | CA | ILE | B | 118 | -33.211 | -6.409 | 128.973 | 1.00 | 27.52 | C |
| ATOM | 2530 | C | ILE | B | 118 | -31.997 | -5.501 | 128.878 | 1.00 | 27.02 | C |
| ATOM | 2531 | O | ILE | B | 118 | -31.130 | -5.541 | 129.750 | 1.00 | 28.87 | O |
| ATOM | 2532 | CB | ILE | B | 118 | -34.280 | -5.811 | 129.911 | 1.00 | 29.46 | C |
| ATOM | 2533 | CG1 | ILE | B | 118 | -34.774 | -4.480 | 129.347 | 1.00 | 30.76 | C |
| ATOM | 2534 | CG2 | ILE | B | 118 | -33.752 | -5.639 | 131.343 | 1.00 | 25.68 | C |
| ATOM | 2535 | CD1 | ILE | B | 118 | -35.736 | -3.775 | 130.229 | 1.00 | 29.17 | C |
| ATOM | 2536 | N | PHE | B | 119 | -31.925 | -4.700 | 127.811 | 1.00 | 28.09 | N |
| ATOM | 2537 | CA | PHE | B | 119 | -30.765 | -3.875 | 127.503 | 1.00 | 24.90 | C |
| ATOM | 2538 | C | PHE | B | 119 | -31.149 | -2.406 | 127.478 | 1.00 | 29.17 | C |
| ATOM | 2539 | O | PHE | B | 119 | -32.037 | -2.018 | 126.706 | 1.00 | 31.62 | O |
| ATOM | 2540 | CB | PHE | B | 119 | -30.163 | -4.224 | 126.148 | 1.00 | 26.03 | C |
| ATOM | 2541 | CG | PHE | B | 119 | -29.643 | -5.603 | 126.045 | 1.00 | 29.01 | C |
| ATOM | 2542 | CD1 | PHE | B | 119 | -28.383 | -5.913 | 126.505 | 1.00 | 26.67 | C |
| ATOM | 2543 | CD2 | PHE | B | 119 | -30.400 | -6.594 | 125.430 | 1.00 | 30.15 | C |
| ATOM | 2544 | CE1 | PHE | B | 119 | -27.897 | -7.200 | 126.403 | 1.00 | 29.34 | C |
| ATOM | 2545 | CE2 | PHE | B | 119 | -29.927 | -7.882 | 125.314 | 1.00 | 25.71 | C |
| ATOM | 2546 | CZ | PHE | B | 119 | -28.670 | -8.191 | 125.806 | 1.00 | 29.15 | C |
| ATOM | 2547 | N | PRO | B | 120 | -30.468 | -1.550 | 128.231 | 1.00 | 34.18 | N |
| ATOM | 2548 | CA | PRO | B | 120 | -30.747 | -0.106 | 128.152 | 1.00 | 34.68 | C |
| ATOM | 2549 | C | PRO | B | 120 | -30.251 | 0.460 | 126.834 | 1.00 | 34.50 | C |
| ATOM | 2550 | O | PRO | B | 120 | -29.378 | -0.140 | 126.188 | 1.00 | 36.34 | O |
| ATOM | 2551 | CB | PRO | B | 120 | -29.963 | 0.474 | 129.339 | 1.00 | 33.20 | C |
| ATOM | 2552 | CG | PRO | B | 120 | -29.568 | -0.746 | 130.190 | 1.00 | 37.48 | C |
| ATOM | 2553 | CD | PRO | B | 120 | -29.417 | -1.862 | 129.212 | 1.00 | 32.46 | C |

| | | | | | | | | | | | |
|---|---|---|---|---|---|---|---|---|---|---|---|
| ATOM | 2554 | N | PRO | B | 121 | -30.761 | 1.616 | 126.402 | 1.00 | 35.15 | N |
| ATOM | 2555 | CA | PRO | B | 121 | -30.200 | 2.248 | 125.203 | 1.00 | 34.06 | C |
| ATOM | 2556 | C | PRO | B | 121 | -28.766 | 2.697 | 125.452 | 1.00 | 32.75 | C |
| ATOM | 2557 | O | PRO | B | 121 | -28.410 | 3.117 | 126.551 | 1.00 | 34.53 | O |
| ATOM | 2558 | CB | PRO | B | 121 | -31.138 | 3.436 | 124.958 | 1.00 | 30.93 | C |
| ATOM | 2559 | CG | PRO | B | 121 | -31.733 | 3.718 | 126.279 | 1.00 | 29.12 | C |
| ATOM | 2560 | CD | PRO | B | 121 | -31.870 | 2.404 | 126.965 | 1.00 | 30.02 | C |
| ATOM | 2561 | N | SER | B | 122 | -27.930 | 2.583 | 124.431 | 1.00 | 35.60 | N |
| ATOM | 2562 | CA | SER | B | 122 | -26.558 | 3.043 | 124.576 | 1.00 | 40.70 | C |
| ATOM | 2563 | C | SER | B | 122 | -26.517 | 4.570 | 124.585 | 1.00 | 42.19 | C |
| ATOM | 2564 | O | SER | B | 122 | -27.311 | 5.239 | 123.913 | 1.00 | 40.94 | O |
| ATOM | 2565 | CB | SER | B | 122 | -25.677 | 2.503 | 123.437 | 1.00 | 38.69 | C |
| ATOM | 2566 | OG | SER | B | 122 | -25.921 | 3.200 | 122.223 | 1.00 | 33.97 | O |
| ATOM | 2567 | N | ASP | B | 123 | -25.550 | 5.120 | 125.328 | 1.00 | 41.84 | N |
| ATOM | 2568 | CA | ASP | B | 123 | -25.391 | 6.569 | 125.357 | 1.00 | 44.12 | C |
| ATOM | 2569 | C | ASP | B | 123 | -25.119 | 7.126 | 123.965 | 1.00 | 48.91 | C |
| ATOM | 2570 | O | ASP | B | 123 | -25.453 | 8.285 | 123.682 | 1.00 | 47.81 | O |
| ATOM | 2571 | CB | ASP | B | 123 | -24.267 | 6.968 | 126.311 | 1.00 | 47.34 | C |
| ATOM | 2572 | CG | ASP | B | 123 | -24.693 | 6.950 | 127.779 | 1.00 | 59.18 | C |
| ATOM | 2573 | OD1 | ASP | B | 123 | -25.864 | 7.272 | 128.086 | 1.00 | 59.74 | O |
| ATOM | 2574 | OD2 | ASP | B | 123 | -23.839 | 6.640 | 128.638 | 1.00 | 65.74 | O1- |
| ATOM | 2575 | N | GLU | B | 124 | -24.542 | 6.312 | 123.079 | 1.00 | 44.91 | N |
| ATOM | 2576 | CA | GLU | B | 124 | -24.273 | 6.776 | 121.722 | 1.00 | 48.45 | C |
| ATOM | 2577 | C | GLU | B | 124 | -25.559 | 7.001 | 120.919 | 1.00 | 48.55 | C |
| ATOM | 2578 | O | GLU | B | 124 | -25.649 | 7.962 | 120.145 | 1.00 | 48.61 | O |
| ATOM | 2579 | CB | GLU | B | 124 | -23.360 | 5.788 | 121.014 | 1.00 | 46.13 | C |
| ATOM | 2580 | CG | GLU | B | 124 | -22.949 | 6.279 | 119.661 | 1.00 | 52.72 | C |
| ATOM | 2581 | CD | GLU | B | 124 | -22.264 | 5.209 | 118.846 | 1.00 | 59.55 | C |
| ATOM | 2582 | OE1 | GLU | B | 124 | -22.167 | 5.408 | 117.608 | 1.00 | 50.04 | O |
| ATOM | 2583 | OE2 | GLU | B | 124 | -21.850 | 4.175 | 119.443 | 1.00 | 55.47 | O1- |
| ATOM | 2584 | N | GLN | B | 125 | -26.574 | 6.143 | 121.093 | 1.00 | 46.17 | N |
| ATOM | 2585 | CA | GLN | B | 125 | -27.839 | 6.369 | 120.391 | 1.00 | 41.17 | C |
| ATOM | 2586 | C | GLN | B | 125 | -28.632 | 7.506 | 121.018 | 1.00 | 44.73 | C |
| ATOM | 2587 | O | GLN | B | 125 | -29.391 | 8.187 | 120.315 | 1.00 | 41.05 | O |
| ATOM | 2588 | CB | GLN | B | 125 | -28.687 | 5.095 | 120.366 | 1.00 | 36.73 | C |
| ATOM | 2589 | CG | GLN | B | 125 | -30.012 | 5.222 | 119.593 | 1.00 | 33.48 | C |
| ATOM | 2590 | CD | GLN | B | 125 | -31.023 | 4.118 | 119.918 | 1.00 | 37.11 | C |
| ATOM | 2591 | OE1 | GLN | B | 125 | -30.936 | 3.430 | 120.947 | 1.00 | 35.61 | O |
| ATOM | 2592 | NE2 | GLN | B | 125 | -31.976 | 3.925 | 119.016 | 1.00 | 41.82 | N |
| ATOM | 2593 | N | LEU | B | 126 | -28.493 | 7.704 | 122.337 | 1.00 | 43.68 | N |
| ATOM | 2594 | CA | LEU | B | 126 | -29.168 | 8.813 | 123.006 | 1.00 | 45.10 | C |
| ATOM | 2595 | C | LEU | B | 126 | -28.719 | 10.152 | 122.434 | 1.00 | 49.62 | C |
| ATOM | 2596 | O | LEU | B | 126 | -29.526 | 11.085 | 122.313 | 1.00 | 49.20 | O |
| ATOM | 2597 | CB | LEU | B | 126 | -28.906 | 8.751 | 124.510 | 1.00 | 46.51 | C |
| ATOM | 2598 | CG | LEU | B | 126 | -29.691 | 7.699 | 125.300 | 1.00 | 44.00 | C |
| ATOM | 2599 | CD1 | LEU | B | 126 | -29.263 | 7.692 | 126.754 | 1.00 | 37.07 | C |
| ATOM | 2600 | CD2 | LEU | B | 126 | -31.208 | 7.898 | 125.167 | 1.00 | 36.79 | C |
| ATOM | 2601 | N | LYS | B | 127 | -27.428 | 10.263 | 122.083 | 1.00 | 47.40 | N |
| ATOM | 2602 | CA | LYS | B | 127 | -26.902 | 11.463 | 121.438 | 1.00 | 44.91 | C |
| ATOM | 2603 | C | LYS | B | 127 | -27.707 | 11.875 | 120.208 | 1.00 | 50.97 | C |
| ATOM | 2604 | O | LYS | B | 127 | -27.782 | 13.065 | 119.883 | 1.00 | 56.06 | O |
| ATOM | 2605 | CB | LYS | B | 127 | -25.441 | 11.244 | 121.060 | 1.00 | 53.95 | C |
| ATOM | 2606 | CG | LYS | B | 127 | -24.474 | 11.931 | 122.009 | 1.00 | 65.16 | C |
| ATOM | 2607 | CD | LYS | B | 127 | -24.039 | 11.038 | 123.160 | 1.00 | 60.17 | C |
| ATOM | 2608 | CE | LYS | B | 127 | -22.846 | 10.198 | 122.738 | 1.00 | 65.63 | C |
| ATOM | 2609 | NZ | LYS | B | 127 | -21.833 | 11.021 | 122.005 | 1.00 | 65.70 | N1+ |
| ATOM | 2610 | N | SER | B | 128 | -28.302 | 10.918 | 119.506 | 1.00 | 48.42 | N |
| ATOM | 2611 | CA | SER | B | 128 | -29.026 | 11.194 | 118.274 | 1.00 | 44.86 | C |
| ATOM | 2612 | C | SER | B | 128 | -30.527 | 11.393 | 118.485 | 1.00 | 48.97 | C |
| ATOM | 2613 | O | SER | B | 128 | -31.271 | 11.432 | 117.498 | 1.00 | 56.04 | O |
| ATOM | 2614 | CB | SER | B | 128 | -28.814 | 10.057 | 117.266 | 1.00 | 51.64 | C |
| ATOM | 2615 | OG | SER | B | 128 | -29.460 | 8.856 | 117.686 | 1.00 | 48.78 | O |
| ATOM | 2616 | N | GLY | B | 129 | -30.999 | 11.500 | 119.728 | 1.00 | 42.11 | N |
| ATOM | 2617 | CA | GLY | B | 129 | -32.383 | 11.859 | 119.953 | 1.00 | 39.56 | C |
| ATOM | 2618 | C | GLY | B | 129 | -33.356 | 10.703 | 120.071 | 1.00 | 47.15 | C |
| ATOM | 2619 | O | GLY | B | 129 | -34.553 | 10.945 | 120.312 | 1.00 | 45.83 | O |
| ATOM | 2620 | N | THR | B | 130 | -32.894 | 9.459 | 119.903 | 1.00 | 44.00 | N |
| ATOM | 2621 | CA | THR | B | 130 | -33.745 | 8.278 | 119.983 | 1.00 | 37.83 | C |
| ATOM | 2622 | C | THR | B | 130 | -33.231 | 7.272 | 121.008 | 1.00 | 36.90 | C |
| ATOM | 2623 | O | THR | B | 130 | -32.023 | 7.117 | 121.190 | 1.00 | 41.23 | O |
| ATOM | 2624 | CB | THR | B | 130 | -33.871 | 7.637 | 118.606 | 1.00 | 40.93 | C |

| | | | | | | | | | | | |
|---|---|---|---|---|---|---|---|---|---|---|---|
| ATOM | 2625 | OG1 | THR | B | 130 | -34.572 | 8.543 | 117.740 | 1.00 | 34.84 | O |
| ATOM | 2626 | CG2 | THR | B | 130 | -34.617 | 6.297 | 118.679 | 1.00 | 44.09 | C |
| ATOM | 2627 | N | ALA | B | 131 | -34.155 | 6.598 | 121.686 | 1.00 | 35.77 | N |
| ATOM | 2628 | CA | ALA | B | 131 | -33.841 | 5.535 | 122.636 | 1.00 | 35.93 | C |
| ATOM | 2629 | C | ALA | B | 131 | -34.469 | 4.216 | 122.186 | 1.00 | 35.42 | C |
| ATOM | 2630 | O | ALA | B | 131 | -35.693 | 4.128 | 121.999 | 1.00 | 37.68 | O |
| ATOM | 2631 | CB | ALA | B | 131 | -34.346 | 5.896 | 124.039 | 1.00 | 33.77 | C |
| ATOM | 2632 | N | SER | B | 132 | -33.645 | 3.183 | 122.055 | 1.00 | 29.61 | N |
| ATOM | 2633 | CA | SER | B | 132 | -34.127 | 1.837 | 121.791 | 1.00 | 29.37 | C |
| ATOM | 2634 | C | SER | B | 132 | -33.845 | 0.960 | 122.999 | 1.00 | 24.73 | C |
| ATOM | 2635 | O | SER | B | 132 | -32.689 | 0.774 | 123.367 | 1.00 | 30.61 | O |
| ATOM | 2636 | CB | SER | B | 132 | -33.466 | 1.265 | 120.545 | 1.00 | 28.99 | C |
| ATOM | 2637 | OG | SER | B | 132 | -33.838 | 2.013 | 119.420 | 1.00 | 32.09 | O |
| ATOM | 2638 | N | VAL | B | 133 | -34.890 | 0.411 | 123.599 | 1.00 | 29.26 | N |
| ATOM | 2639 | CA | VAL | B | 133 | -34.753 | -0.593 | 124.654 | 1.00 | 30.39 | C |
| ATOM | 2640 | C | VAL | B | 133 | -35.021 | -1.960 | 124.044 | 1.00 | 28.40 | C |
| ATOM | 2641 | O | VAL | B | 133 | -35.963 | -2.123 | 123.264 | 1.00 | 30.58 | O |
| ATOM | 2642 | CB | VAL | B | 133 | -35.717 | -0.303 | 125.816 | 1.00 | 29.07 | C |
| ATOM | 2643 | CG1 | VAL | B | 133 | -35.309 | -1.095 | 127.049 | 1.00 | 27.89 | C |
| ATOM | 2644 | CG2 | VAL | B | 133 | -35.751 | 1.186 | 126.090 | 1.00 | 26.20 | C |
| ATOM | 2645 | N | VAL | B | 134 | -34.203 | -2.948 | 124.392 | 1.00 | 26.36 | N |
| ATOM | 2646 | CA | VAL | B | 134 | -34.262 | -4.261 | 123.762 | 1.00 | 28.94 | C |
| ATOM | 2647 | C | VAL | B | 134 | -34.505 | -5.315 | 124.830 | 1.00 | 29.71 | C |
| ATOM | 2648 | O | VAL | B | 134 | -33.851 | -5.305 | 125.880 | 1.00 | 30.17 | O |
| ATOM | 2649 | CB | VAL | B | 134 | -32.973 | -4.576 | 122.980 | 1.00 | 30.23 | C |
| ATOM | 2650 | CG1 | VAL | B | 134 | -33.115 | -5.912 | 122.281 | 1.00 | 22.43 | C |
| ATOM | 2651 | CG2 | VAL | B | 134 | -32.648 | -3.460 | 121.991 | 1.00 | 24.76 | C |
| ATOM | 2652 | N | CYS | B | 135 | -35.438 | -6.226 | 124.560 | 1.00 | 27.09 | N |
| ATOM | 2653 | CA | CYS | B | 135 | -35.688 | -7.373 | 125.423 | 1.00 | 30.29 | C |
| ATOM | 2654 | C | CYS | B | 135 | -35.351 | -8.641 | 124.645 | 1.00 | 30.83 | C |
| ATOM | 2655 | O | CYS | B | 135 | -35.841 | -8.828 | 123.528 | 1.00 | 31.86 | O |
| ATOM | 2656 | CB | CYS | B | 135 | -37.149 | -7.402 | 125.895 | 1.00 | 28.77 | C |
| ATOM | 2657 | SG | CYS | B | 135 | -37.549 | -8.569 | 127.279 | 1.00 | 36.90 | S |
| ATOM | 2658 | N | LEU | B | 136 | -34.537 | -9.514 | 125.236 | 1.00 | 28.65 | N |
| ATOM | 2659 | CA | LEU | B | 136 | -34.113 | -10.767 | 124.610 | 1.00 | 26.89 | C |
| ATOM | 2660 | C | LEU | B | 136 | -34.712 | -11.963 | 125.340 | 1.00 | 27.49 | C |
| ATOM | 2661 | O | LEU | B | 136 | -34.462 | -12.158 | 126.536 | 1.00 | 26.81 | O |
| ATOM | 2662 | CB | LEU | B | 136 | -32.587 | -10.874 | 124.591 | 1.00 | 23.26 | C |
| ATOM | 2663 | CG | LEU | B | 136 | -32.021 | -12.242 | 124.215 | 1.00 | 26.55 | C |
| ATOM | 2664 | CD1 | LEU | B | 136 | -32.394 | -12.677 | 122.784 | 1.00 | 29.37 | C |
| ATOM | 2665 | CD2 | LEU | B | 136 | -30.539 | -12.206 | 124.383 | 1.00 | 26.86 | C |
| ATOM | 2666 | N | LEU | B | 137 | -35.471 | -12.776 | 124.612 | 1.00 | 27.17 | N |
| ATOM | 2667 | CA | LEU | B | 137 | -36.003 | -14.046 | 125.109 | 1.00 | 24.46 | C |
| ATOM | 2668 | C | LEU | B | 137 | -35.244 | -15.151 | 124.412 | 1.00 | 25.24 | C |
| ATOM | 2669 | O | LEU | B | 137 | -35.476 | -15.416 | 123.232 | 1.00 | 27.19 | O |
| ATOM | 2670 | CB | LEU | B | 137 | -37.498 | -14.197 | 124.847 | 1.00 | 24.41 | C |
| ATOM | 2671 | CG | LEU | B | 137 | -38.513 | -13.411 | 125.671 | 1.00 | 26.56 | C |
| ATOM | 2672 | CD1 | LEU | B | 137 | -38.301 | -11.899 | 125.512 | 1.00 | 22.36 | C |
| ATOM | 2673 | CD2 | LEU | B | 137 | -39.922 | -13.845 | 125.275 | 1.00 | 24.34 | C |
| ATOM | 2674 | N | ASN | B | 138 | -34.374 | -15.820 | 125.147 | 1.00 | 27.07 | N |
| ATOM | 2675 | CA | ASN | B | 138 | -33.403 | -16.731 | 124.575 | 1.00 | 27.89 | C |
| ATOM | 2676 | C | ASN | B | 138 | -33.800 | -18.186 | 124.822 | 1.00 | 28.77 | C |
| ATOM | 2677 | O | ASN | B | 138 | -34.161 | -18.546 | 125.945 | 1.00 | 33.61 | O |
| ATOM | 2678 | CB | ASN | B | 138 | -32.033 | -16.411 | 125.163 | 1.00 | 31.08 | C |
| ATOM | 2679 | CG | ASN | B | 138 | -30.904 | -16.806 | 124.256 | 1.00 | 37.01 | C |
| ATOM | 2680 | OD1 | ASN | B | 138 | -30.870 | -16.438 | 123.076 | 1.00 | 34.03 | O |
| ATOM | 2681 | ND2 | ASN | B | 138 | -29.933 | -17.509 | 124.815 | 1.00 | 40.66 | N |
| ATOM | 2682 | N | ASN | B | 139 | -33.800 | -18.987 | 123.747 | 1.00 | 26.63 | N |
| ATOM | 2683 | CA | ASN | B | 139 | -33.861 | -20.463 | 123.722 | 1.00 | 26.50 | C |
| ATOM | 2684 | C | ASN | B | 139 | -35.075 | -21.037 | 124.462 | 1.00 | 25.84 | C |
| ATOM | 2685 | O | ASN | B | 139 | -34.958 | -21.778 | 125.436 | 1.00 | 30.08 | O |
| ATOM | 2686 | CB | ASN | B | 139 | -32.559 | -21.104 | 124.220 | 1.00 | 27.61 | C |
| ATOM | 2687 | CG | ASN | B | 139 | -31.415 | -20.897 | 123.232 | 1.00 | 35.63 | C |
| ATOM | 2688 | OD1 | ASN | B | 139 | -31.150 | -19.770 | 122.822 | 1.00 | 31.84 | O |
| ATOM | 2689 | ND2 | ASN | B | 139 | -30.785 | -21.990 | 122.787 | 1.00 | 33.37 | N |
| ATOM | 2690 | N | PHE | B | 140 | -36.249 | -20.756 | 123.903 | 1.00 | 27.05 | N |
| ATOM | 2691 | CA | PHE | B | 140 | -37.494 | -21.275 | 124.449 | 1.00 | 28.39 | C |
| ATOM | 2692 | C | PHE | B | 140 | -38.206 | -22.147 | 123.413 | 1.00 | 30.25 | C |
| ATOM | 2693 | O | PHE | B | 140 | -37.990 | -22.032 | 122.203 | 1.00 | 30.54 | O |
| ATOM | 2694 | CB | PHE | B | 140 | -38.430 | -20.144 | 124.905 | 1.00 | 25.56 | C |
| ATOM | 2695 | CG | PHE | B | 140 | -38.791 | -19.192 | 123.813 | 1.00 | 27.91 | C |

| | | | | | | | | | | | |
|---|---|---|---|---|---|---|---|---|---|---|---|
| ATOM | 2696 | CD1 | PHE | B | 140 | -37.989 | -18.086 | 123.541 | 1.00 | 25.72 | C |
| ATOM | 2697 | CD2 | PHE | B | 140 | -39.922 | -19.410 | 123.030 | 1.00 | 27.50 | C |
| ATOM | 2698 | CE1 | PHE | B | 140 | -38.309 | -17.202 | 122.515 | 1.00 | 24.13 | C |
| ATOM | 2699 | CE2 | PHE | B | 140 | -40.257 | -18.524 | 122.002 | 1.00 | 29.76 | C |
| ATOM | 2700 | CZ | PHE | B | 140 | -39.438 | -17.415 | 121.744 | 1.00 | 27.82 | C |
| ATOM | 2701 | N | TYR | B | 141 | -39.054 | -23.030 | 123.914 | 1.00 | 27.15 | N |
| ATOM | 2702 | CA | TYR | B | 141 | -39.947 | -23.833 | 123.101 | 1.00 | 27.13 | C |
| ATOM | 2703 | C | TYR | B | 141 | -41.183 | -24.086 | 123.982 | 1.00 | 29.40 | C |
| ATOM | 2704 | O | TYR | B | 141 | -41.048 | -24.404 | 125.162 | 1.00 | 28.49 | O |
| ATOM | 2705 | CB | TYR | B | 141 | -39.278 | -25.150 | 122.634 | 1.00 | 27.47 | C |
| ATOM | 2706 | CG | TYR | B | 141 | -40.171 | -25.919 | 121.693 | 1.00 | 28.08 | C |
| ATOM | 2707 | CD1 | TYR | B | 141 | -41.133 | -26.780 | 122.196 | 1.00 | 30.33 | C |
| ATOM | 2708 | CD2 | TYR | B | 141 | -40.098 | -25.746 | 120.308 | 1.00 | 24.75 | C |
| ATOM | 2709 | CE1 | TYR | B | 141 | -42.002 | -27.456 | 121.359 | 1.00 | 30.51 | C |
| ATOM | 2710 | CE2 | TYR | B | 141 | -40.969 | -26.402 | 119.464 | 1.00 | 25.25 | C |
| ATOM | 2711 | CZ | TYR | B | 141 | -41.930 | -27.270 | 120.002 | 1.00 | 30.79 | C |
| ATOM | 2712 | OH | TYR | B | 141 | -42.841 | -27.974 | 119.229 | 1.00 | 28.57 | O |
| ATOM | 2713 | N | PRO | B | 142 | -42.394 | -23.975 | 123.414 | 1.00 | 29.92 | N |
| ATOM | 2714 | CA | PRO | B | 142 | -42.663 | -23.735 | 121.996 | 1.00 | 28.29 | C |
| ATOM | 2715 | C | PRO | B | 142 | -42.638 | -22.263 | 121.586 | 1.00 | 29.24 | C |
| ATOM | 2716 | O | PRO | B | 142 | -42.345 | -21.399 | 122.404 | 1.00 | 30.25 | O |
| ATOM | 2717 | CB | PRO | B | 142 | -44.067 | -24.327 | 121.825 | 1.00 | 28.67 | C |
| ATOM | 2718 | CG | PRO | B | 142 | -44.726 | -24.029 | 123.121 | 1.00 | 25.40 | C |
| ATOM | 2719 | CD | PRO | B | 142 | -43.635 | -24.261 | 124.160 | 1.00 | 27.51 | C |
| ATOM | 2720 | N | ARG | B | 143 | -43.042 | -22.022 | 120.332 | 1.00 | 33.09 | N |
| ATOM | 2721 | CA | ARG | B | 143 | -42.828 | -20.754 | 119.634 | 1.00 | 32.76 | C |
| ATOM | 2722 | C | ARG | B | 143 | -43.567 | -19.587 | 120.289 | 1.00 | 33.19 | C |
| ATOM | 2723 | O | ARG | B | 143 | -43.061 | -18.461 | 120.295 | 1.00 | 34.09 | O |
| ATOM | 2724 | CB | ARG | B | 143 | -43.256 | -20.953 | 118.174 | 1.00 | 30.54 | C |
| ATOM | 2725 | CG | ARG | B | 143 | -43.343 | -19.750 | 117.269 | 1.00 | 30.54 | C |
| ATOM | 2726 | CD | ARG | B | 143 | -42.071 | -19.508 | 116.495 | 1.00 | 35.91 | C |
| ATOM | 2727 | NE | ARG | B | 143 | -42.253 | -18.751 | 115.239 | 1.00 | 36.61 | N |
| ATOM | 2728 | CZ | ARG | B | 143 | -42.878 | -17.569 | 115.145 | 1.00 | 37.84 | C |
| ATOM | 2729 | NH1 | ARG | B | 143 | -43.464 | -17.009 | 116.203 | 1.00 | 40.08 | N1+ |
| ATOM | 2730 | NH2 | ARG | B | 143 | -42.943 | -16.947 | 113.987 | 1.00 | 35.58 | N |
| ATOM | 2731 | N | GLU | B | 144 | -44.743 | -19.837 | 120.856 | 1.00 | 31.25 | N |
| ATOM | 2732 | CA | GLU | B | 144 | -45.607 | -18.768 | 121.346 | 1.00 | 35.56 | C |
| ATOM | 2733 | C | GLU | B | 144 | -45.024 | -18.121 | 122.602 | 1.00 | 34.95 | C |
| ATOM | 2734 | O | GLU | B | 144 | -44.602 | -18.808 | 123.537 | 1.00 | 34.88 | O |
| ATOM | 2735 | CB | GLU | B | 144 | -47.021 | -19.299 | 121.646 | 1.00 | 30.65 | C |
| ATOM | 2736 | CG | GLU | B | 144 | -47.821 | -19.817 | 120.448 | 1.00 | 34.84 | C |
| ATOM | 2737 | CD | GLU | B | 144 | -47.385 | -21.206 | 119.951 | 1.00 | 44.49 | C |
| ATOM | 2738 | OE1 | GLU | B | 144 | -47.117 | -22.107 | 120.783 | 1.00 | 41.20 | O |
| ATOM | 2739 | OE2 | GLU | B | 144 | -47.333 | -21.404 | 118.716 | 1.00 | 47.18 | O1- |
| ATOM | 2740 | N | ALA | B | 145 | -45.002 | -16.795 | 122.623 | 1.00 | 29.26 | N |
| ATOM | 2741 | CA | ALA | B | 145 | -44.486 | -16.077 | 123.774 | 1.00 | 34.47 | C |
| ATOM | 2742 | C | ALA | B | 145 | -45.102 | -14.691 | 123.756 | 1.00 | 33.83 | C |
| ATOM | 2743 | O | ALA | B | 145 | -45.457 | -14.194 | 122.684 | 1.00 | 35.59 | O |
| ATOM | 2744 | CB | ALA | B | 145 | -42.952 | -16.007 | 123.737 | 1.00 | 31.26 | C |
| ATOM | 2745 | N | LYS | B | 146 | -45.250 | -14.077 | 124.937 | 1.00 | 26.49 | N |
| ATOM | 2746 | CA | LYS | B | 146 | -45.754 | -12.707 | 125.026 | 1.00 | 28.76 | C |
| ATOM | 2747 | C | LYS | B | 146 | -44.766 | -11.790 | 125.756 | 1.00 | 32.11 | C |
| ATOM | 2748 | O | LYS | B | 146 | -44.336 | -12.085 | 126.880 | 1.00 | 25.51 | O |
| ATOM | 2749 | CB | LYS | B | 146 | -47.130 | -12.683 | 125.687 | 1.00 | 34.49 | C |
| ATOM | 2750 | CG | LYS | B | 146 | -47.879 | -11.354 | 125.547 | 1.00 | 44.62 | C |
| ATOM | 2751 | CD | LYS | B | 146 | -49.339 | -11.502 | 126.002 | 1.00 | 51.17 | C |
| ATOM | 2752 | CE | LYS | B | 146 | -50.157 | -10.231 | 125.771 | 1.00 | 55.61 | C |
| ATOM | 2753 | NZ | LYS | B | 146 | -51.627 | -10.552 | 125.678 | 1.00 | 51.90 | N1+ |
| ATOM | 2754 | N | VAL | B | 147 | -44.417 | -10.672 | 125.113 | 1.00 | 32.22 | N |
| ATOM | 2755 | CA | VAL | B | 147 | -43.616 | -9.609 | 125.712 | 1.00 | 28.21 | C |
| ATOM | 2756 | C | VAL | B | 147 | -44.534 | -8.434 | 126.015 | 1.00 | 34.36 | C |
| ATOM | 2757 | O | VAL | B | 147 | -45.332 | -8.029 | 125.165 | 1.00 | 33.36 | O |
| ATOM | 2758 | CB | VAL | B | 147 | -42.464 | -9.160 | 124.794 | 1.00 | 27.98 | C |
| ATOM | 2759 | CG1 | VAL | B | 147 | -41.768 | -7.931 | 125.372 | 1.00 | 29.12 | C |
| ATOM | 2760 | CG2 | VAL | B | 147 | -41.472 | -10.275 | 124.560 | 1.00 | 32.19 | C |
| ATOM | 2761 | N | GLN | B | 148 | -44.444 | -7.904 | 127.230 | 1.00 | 35.67 | N |
| ATOM | 2762 | CA | GLN | B | 148 | -45.128 | -6.673 | 127.593 | 1.00 | 36.29 | C |
| ATOM | 2763 | C | GLN | B | 148 | -44.100 | -5.665 | 128.073 | 1.00 | 33.05 | C |
| ATOM | 2764 | O | GLN | B | 148 | -43.303 | -5.968 | 128.966 | 1.00 | 29.76 | O |
| ATOM | 2765 | CB | GLN | B | 148 | -46.192 | -6.913 | 128.676 | 1.00 | 36.98 | C |
| ATOM | 2766 | CG | GLN | B | 148 | -47.614 | -6.800 | 128.154 | 1.00 | 47.87 | C |

| | | | | | | | | | | | |
|---|---|---|---|---|---|---|---|---|---|---|---|
| ATOM | 2767 | CD | GLN | B | 148 | -48.651 | -7.188 | 129.192 | 1.00 | 64.59 | C |
| ATOM | 2768 | OE1 | GLN | B | 148 | -48.420 | -7.050 | 130.398 | 1.00 | 64.90 | O |
| ATOM | 2769 | NE2 | GLN | B | 148 | -49.806 | -7.674 | 128.728 | 1.00 | 58.09 | N |
| ATOM | 2770 | N | TRP | B | 149 | -44.099 | -4.485 | 127.465 | 1.00 | 34.92 | N |
| ATOM | 2771 | CA | TRP | B | 149 | -43.234 | -3.400 | 127.918 | 1.00 | 35.07 | C |
| ATOM | 2772 | C | TRP | B | 149 | -43.941 | -2.522 | 128.952 | 1.00 | 31.98 | C |
| ATOM | 2773 | O | TRP | B | 149 | -45.105 | -2.154 | 128.778 | 1.00 | 33.16 | O |
| ATOM | 2774 | CB | TRP | B | 149 | -42.797 | -2.549 | 126.730 | 1.00 | 28.25 | C |
| ATOM | 2775 | CG | TRP | B | 149 | -41.704 | -3.158 | 125.876 | 1.00 | 31.59 | C |
| ATOM | 2776 | CD1 | TRP | B | 149 | -41.851 | -3.717 | 124.640 | 1.00 | 28.61 | C |
| ATOM | 2777 | CD2 | TRP | B | 149 | -40.305 | -3.236 | 126.186 | 1.00 | 28.06 | C |
| ATOM | 2778 | NE1 | TRP | B | 149 | -40.640 | -4.134 | 124.164 | 1.00 | 31.98 | N |
| ATOM | 2779 | CE2 | TRP | B | 149 | -39.671 | -3.852 | 125.091 | 1.00 | 31.31 | C |
| ATOM | 2780 | CE3 | TRP | B | 149 | -39.529 | -2.842 | 127.280 | 1.00 | 32.39 | C |
| ATOM | 2781 | CZ2 | TRP | B | 149 | -38.289 | -4.080 | 125.048 | 1.00 | 29.16 | C |
| ATOM | 2782 | CZ3 | TRP | B | 149 | -38.159 | -3.066 | 127.242 | 1.00 | 36.30 | C |
| ATOM | 2783 | CH2 | TRP | B | 149 | -37.550 | -3.676 | 126.125 | 1.00 | 32.50 | C |
| ATOM | 2784 | N | LYS | B | 150 | -43.228 | -2.174 | 130.023 | 1.00 | 34.33 | N |
| ATOM | 2785 | CA | LYS | B | 150 | -43.736 | -1.240 | 131.030 | 1.00 | 35.21 | C |
| ATOM | 2786 | C | LYS | B | 150 | -42.711 | -0.143 | 131.257 | 1.00 | 36.04 | C |
| ATOM | 2787 | O | LYS | B | 150 | -41.534 | -0.433 | 131.489 | 1.00 | 40.23 | O |
| ATOM | 2788 | CB | LYS | B | 150 | -44.050 | -1.918 | 132.374 | 1.00 | 29.19 | C |
| ATOM | 2789 | CG | LYS | B | 150 | -45.326 | -2.755 | 132.421 | 1.00 | 32.86 | C |
| ATOM | 2790 | CD | LYS | B | 150 | -45.296 | -3.677 | 133.643 | 1.00 | 42.43 | C |
| ATOM | 2791 | CE | LYS | B | 150 | -46.327 | -4.800 | 133.536 | 1.00 | 54.13 | C |
| ATOM | 2792 | NZ | LYS | B | 150 | -46.598 | -5.502 | 134.839 | 1.00 | 48.84 | N1+ |
| ATOM | 2793 | N | VAL | B | 151 | -43.158 | 1.107 | 131.189 | 1.00 | 34.12 | N |
| ATOM | 2794 | CA | VAL | B | 151 | -42.355 | 2.270 | 131.559 | 1.00 | 34.61 | C |
| ATOM | 2795 | C | VAL | B | 151 | -43.003 | 2.921 | 132.784 | 1.00 | 36.08 | C |
| ATOM | 2796 | O | VAL | B | 151 | -44.118 | 3.448 | 132.699 | 1.00 | 35.70 | O |
| ATOM | 2797 | CB | VAL | B | 151 | -42.241 | 3.255 | 130.392 | 1.00 | 31.07 | C |
| ATOM | 2798 | CG1 | VAL | B | 151 | -41.307 | 4.377 | 130.732 | 1.00 | 31.60 | C |
| ATOM | 2799 | CG2 | VAL | B | 151 | -41.752 | 2.528 | 129.173 | 1.00 | 37.80 | C |
| ATOM | 2800 | N | ASP | B | 152 | -42.299 | 2.902 | 133.921 | 1.00 | 39.23 | N |
| ATOM | 2801 | CA | ASP | B | 152 | -42.854 | 3.340 | 135.217 | 1.00 | 38.88 | C |
| ATOM | 2802 | C | ASP | B | 152 | -44.243 | 2.743 | 135.428 | 1.00 | 37.74 | C |
| ATOM | 2803 | O | ASP | B | 152 | -45.194 | 3.412 | 135.836 | 1.00 | 35.76 | O |
| ATOM | 2804 | CB | ASP | B | 152 | -42.848 | 4.863 | 135.343 | 1.00 | 36.31 | C |
| ATOM | 2805 | CG | ASP | B | 152 | -41.451 | 5.404 | 135.628 | 1.00 | 44.31 | C |
| ATOM | 2806 | OD1 | ASP | B | 152 | -40.688 | 4.719 | 136.357 | 1.00 | 39.45 | O |
| ATOM | 2807 | OD2 | ASP | B | 152 | -41.093 | 6.479 | 135.094 | 1.00 | 46.68 | O1- |
| ATOM | 2808 | N | ASN | B | 153 | -44.333 | 1.457 | 135.097 | 1.00 | 38.94 | N |
| ATOM | 2809 | CA | ASN | B | 153 | -45.491 | 0.579 | 135.212 | 1.00 | 39.28 | C |
| ATOM | 2810 | C | ASN | B | 153 | -46.660 | 0.990 | 134.321 | 1.00 | 37.08 | C |
| ATOM | 2811 | O | ASN | B | 153 | -47.783 | 0.508 | 134.502 | 1.00 | 39.74 | O |
| ATOM | 2812 | CB | ASN | B | 153 | -45.933 | 0.433 | 136.666 | 1.00 | 37.94 | C |
| ATOM | 2813 | CG | ASN | B | 153 | -46.502 | -0.927 | 136.937 | 1.00 | 41.53 | C |
| ATOM | 2814 | OD1 | ASN | B | 153 | -47.693 | -1.071 | 137.197 | 1.00 | 48.49 | O |
| ATOM | 2815 | ND2 | ASN | B | 153 | -45.658 | -1.953 | 136.827 | 1.00 | 47.40 | N |
| ATOM | 2816 | N | ALA | B | 154 | -46.418 | 1.824 | 133.322 | 1.00 | 27.73 | N |
| ATOM | 2817 | CA | ALA | B | 154 | -47.403 | 2.060 | 132.281 | 1.00 | 31.59 | C |
| ATOM | 2818 | C | ALA | B | 154 | -47.152 | 1.064 | 131.145 | 1.00 | 35.73 | C |
| ATOM | 2819 | O | ALA | B | 154 | -46.034 | 1.006 | 130.611 | 1.00 | 34.76 | O |
| ATOM | 2820 | CB | ALA | B | 154 | -47.314 | 3.503 | 131.785 | 1.00 | 30.19 | C |
| ATOM | 2821 | N | LEU | B | 155 | -48.168 | 0.246 | 130.817 | 1.00 | 31.02 | N |
| ATOM | 2822 | CA | LEU | B | 155 | -48.092 | -0.680 | 129.680 | 1.00 | 29.47 | C |
| ATOM | 2823 | C | LEU | B | 155 | -47.940 | 0.045 | 128.364 | 1.00 | 29.26 | C |
| ATOM | 2824 | O | LEU | B | 155 | -48.816 | 0.809 | 127.967 | 1.00 | 32.19 | O |
| ATOM | 2825 | CB | LEU | B | 155 | -49.321 | -1.578 | 129.581 | 1.00 | 26.14 | C |
| ATOM | 2826 | CG | LEU | B | 155 | -49.137 | -2.963 | 130.188 | 1.00 | 41.59 | C |
| ATOM | 2827 | CD1 | LEU | B | 155 | -49.358 | -2.997 | 131.716 | 1.00 | 37.40 | C |
| ATOM | 2828 | CD2 | LEU | B | 155 | -49.966 | -3.985 | 129.430 | 1.00 | 49.03 | C |
| ATOM | 2829 | N | GLN | B | 156 | -46.891 | -0.293 | 127.632 | 1.00 | 32.37 | N |
| ATOM | 2830 | CA | GLN | B | 156 | -46.687 | 0.225 | 126.289 | 1.00 | 31.54 | C |
| ATOM | 2831 | C | GLN | B | 156 | -47.433 | -0.647 | 125.293 | 1.00 | 27.02 | C |
| ATOM | 2832 | O | GLN | B | 156 | -47.493 | -1.864 | 125.444 | 1.00 | 36.94 | O |
| ATOM | 2833 | CB | GLN | B | 156 | -45.193 | 0.270 | 125.966 | 1.00 | 31.74 | C |
| ATOM | 2834 | CG | GLN | B | 156 | -44.395 | 0.948 | 127.069 | 1.00 | 31.12 | C |
| ATOM | 2835 | CD | GLN | B | 156 | -44.877 | 2.358 | 127.299 | 1.00 | 33.29 | C |
| ATOM | 2836 | OE1 | GLN | B | 156 | -44.789 | 3.213 | 126.412 | 1.00 | 33.92 | O |
| ATOM | 2837 | NE2 | GLN | B | 156 | -45.445 | 2.599 | 128.470 | 1.00 | 34.06 | N |

| | | | | | | | | | | | |
|---|---|---|---|---|---|---|---|---|---|---|---|
| ATOM | 2838 | N | SER | B | 157 | -48.069 | -0.017 | 124.322 | 1.00 | 25.15 | N |
| ATOM | 2839 | CA | SER | B | 157 | -48.818 | -0.745 | 123.309 | 1.00 | 24.13 | C |
| ATOM | 2840 | C | SER | B | 157 | -48.684 | 0.008 | 121.998 | 1.00 | 28.91 | C |
| ATOM | 2841 | O | SER | B | 157 | -49.132 | 1.153 | 121.903 | 1.00 | 33.39 | O |
| ATOM | 2842 | CB | SER | B | 157 | -50.284 | -0.869 | 123.724 | 1.00 | 27.59 | C |
| ATOM | 2843 | OG | SER | B | 157 | -51.056 | -1.511 | 122.738 | 1.00 | 34.07 | O |
| ATOM | 2844 | N | GLY | B | 158 | -48.052 | -0.597 | 121.005 | 1.00 | 23.39 | N |
| ATOM | 2845 | CA | GLY | B | 158 | -47.959 | 0.017 | 119.698 | 1.00 | 22.69 | C |
| ATOM | 2846 | C | GLY | B | 158 | -46.630 | 0.665 | 119.370 | 1.00 | 26.97 | C |
| ATOM | 2847 | O | GLY | B | 158 | -46.467 | 1.166 | 118.256 | 1.00 | 30.74 | O |
| ATOM | 2848 | N | ASN | B | 159 | -45.695 | 0.721 | 120.312 | 1.00 | 26.14 | N |
| ATOM | 2849 | CA | ASN | B | 159 | -44.412 | 1.373 | 120.086 | 1.00 | 25.51 | C |
| ATOM | 2850 | C | ASN | B | 159 | -43.236 | 0.401 | 120.206 | 1.00 | 30.63 | C |
| ATOM | 2851 | O | ASN | B | 159 | -42.149 | 0.779 | 120.660 | 1.00 | 25.81 | O |
| ATOM | 2852 | CB | ASN | B | 159 | -44.236 | 2.542 | 121.043 | 1.00 | 22.42 | C |
| ATOM | 2853 | CG | ASN | B | 159 | -44.537 | 2.165 | 122.463 | 1.00 | 29.01 | C |
| ATOM | 2854 | OD1 | ASN | B | 159 | -44.940 | 1.021 | 122.757 | 1.00 | 30.10 | O |
| ATOM | 2855 | ND2 | ASN | B | 159 | -44.335 | 3.114 | 123.370 | 1.00 | 32.61 | N |
| ATOM | 2856 | N | SER | B | 160 | -43.439 | -0.859 | 119.819 | 1.00 | 27.65 | N |
| ATOM | 2857 | CA | SER | B | 160 | -42.360 | -1.827 | 119.814 | 1.00 | 29.05 | C |
| ATOM | 2858 | C | SER | B | 160 | -42.510 | -2.753 | 118.615 | 1.00 | 28.89 | C |
| ATOM | 2859 | O | SER | B | 160 | -43.602 | -2.939 | 118.089 | 1.00 | 29.48 | O |
| ATOM | 2860 | CB | SER | B | 160 | -42.328 | -2.626 | 121.112 | 1.00 | 26.29 | C |
| ATOM | 2861 | OG | SER | B | 160 | -43.447 | -3.475 | 121.181 | 1.00 | 31.67 | O |
| ATOM | 2862 | N | GLN | B | 161 | -41.400 | -3.357 | 118.197 | 1.00 | 28.03 | N |
| ATOM | 2863 | CA | GLN | B | 161 | -41.425 | -4.362 | 117.147 | 1.00 | 26.96 | C |
| ATOM | 2864 | C | GLN | B | 161 | -40.634 | -5.596 | 117.581 | 1.00 | 31.51 | C |
| ATOM | 2865 | O | GLN | B | 161 | -39.759 | -5.537 | 118.451 | 1.00 | 30.01 | O |
| ATOM | 2866 | CB | GLN | B | 161 | -40.871 | -3.794 | 115.850 | 1.00 | 27.24 | C |
| ATOM | 2867 | CG | GLN | B | 161 | -41.747 | -2.764 | 115.183 | 1.00 | 27.43 | C |
| ATOM | 2868 | CD | GLN | B | 161 | -41.064 | -2.142 | 113.976 | 1.00 | 35.97 | C |
| ATOM | 2869 | OE1 | GLN | B | 161 | -40.203 | -1.267 | 114.121 | 1.00 | 35.92 | O |
| ATOM | 2870 | NE2 | GLN | B | 161 | -41.418 | -2.607 | 112.782 | 1.00 | 30.28 | N |
| ATOM | 2871 | N | GLU | B | 162 | -40.938 | -6.720 | 116.938 | 1.00 | 31.79 | N |
| ATOM | 2872 | CA | GLU | B | 162 | -40.438 | -8.034 | 117.331 | 1.00 | 29.63 | C |
| ATOM | 2873 | C | GLU | B | 162 | -40.040 | -8.837 | 116.104 | 1.00 | 30.95 | C |
| ATOM | 2874 | O | GLU | B | 162 | -40.654 | -8.718 | 115.042 | 1.00 | 26.14 | O |
| ATOM | 2875 | CB | GLU | B | 162 | -41.503 | -8.877 | 117.989 | 1.00 | 23.86 | C |
| ATOM | 2876 | CG | GLU | B | 162 | -41.567 | -8.969 | 119.420 | 1.00 | 34.85 | C |
| ATOM | 2877 | CD | GLU | B | 162 | -42.807 | -9.765 | 119.781 | 1.00 | 41.65 | C |
| ATOM | 2878 | OE1 | GLU | B | 162 | -43.430 | -10.284 | 118.835 | 1.00 | 38.04 | O |
| ATOM | 2879 | OE2 | GLU | B | 162 | -43.177 | -9.858 | 120.973 | 1.00 | 45.93 | O1- |
| ATOM | 2880 | N | SER | B | 163 | -39.057 | -9.712 | 116.280 | 1.00 | 31.61 | N |
| ATOM | 2881 | CA | SER | B | 163 | -38.794 | -10.763 | 115.312 | 1.00 | 25.04 | C |
| ATOM | 2882 | C | SER | B | 163 | -38.348 | -11.995 | 116.088 | 1.00 | 28.10 | C |
| ATOM | 2883 | O | SER | B | 163 | -37.857 | -11.890 | 117.218 | 1.00 | 25.85 | O |
| ATOM | 2884 | CB | SER | B | 163 | -37.781 | -10.326 | 114.254 | 1.00 | 26.93 | C |
| ATOM | 2885 | OG | SER | B | 163 | -36.461 | -10.447 | 114.714 | 1.00 | 29.93 | O |
| ATOM | 2886 | N | VAL | B | 164 | -38.587 | -13.169 | 115.504 | 1.00 | 29.93 | N |
| ATOM | 2887 | CA | VAL | B | 164 | -38.200 | -14.441 | 116.104 | 1.00 | 26.45 | C |
| ATOM | 2888 | C | VAL | B | 164 | -37.323 | -15.214 | 115.130 | 1.00 | 23.48 | C |
| ATOM | 2889 | O | VAL | B | 164 | -37.520 | -15.156 | 113.918 | 1.00 | 28.17 | O |
| ATOM | 2890 | CB | VAL | B | 164 | -39.430 | -15.281 | 116.521 | 1.00 | 30.77 | C |
| ATOM | 2891 | CG1 | VAL | B | 164 | -40.375 | -14.468 | 117.392 | 1.00 | 26.66 | C |
| ATOM | 2892 | CG2 | VAL | B | 164 | -40.156 | -15.786 | 115.308 | 1.00 | 40.64 | C |
| ATOM | 2893 | N | THR | B | 165 | -36.328 | -15.908 | 115.663 | 1.00 | 28.51 | N |
| ATOM | 2894 | CA | THR | B | 165 | -35.442 | -16.716 | 114.848 | 1.00 | 27.61 | C |
| ATOM | 2895 | C | THR | B | 165 | -36.154 | -17.967 | 114.344 | 1.00 | 29.58 | C |
| ATOM | 2896 | O | THR | B | 165 | -37.279 | -18.300 | 114.751 | 1.00 | 28.80 | O |
| ATOM | 2897 | CB | THR | B | 165 | -34.195 | -17.113 | 115.641 | 1.00 | 28.32 | C |
| ATOM | 2898 | OG1 | THR | B | 165 | -34.575 | -17.596 | 116.939 | 1.00 | 33.79 | O |
| ATOM | 2899 | CG2 | THR | B | 165 | -33.275 | -15.942 | 115.795 | 1.00 | 25.22 | C |
| ATOM | 2900 | N | GLU | B | 166 | -35.489 | -18.634 | 113.402 | 1.00 | 28.57 | N |
| ATOM | 2901 | CA | GLU | B | 166 | -35.851 | -19.992 | 113.026 | 1.00 | 29.18 | C |
| ATOM | 2902 | C | GLU | B | 166 | -35.474 | -20.953 | 114.149 | 1.00 | 25.81 | C |
| ATOM | 2903 | O | GLU | B | 166 | -34.705 | -20.622 | 115.046 | 1.00 | 28.21 | O |
| ATOM | 2904 | CB | GLU | B | 166 | -35.158 | -20.398 | 111.722 | 1.00 | 25.69 | C |
| ATOM | 2905 | CG | GLU | B | 166 | -35.615 | -19.673 | 110.472 | 1.00 | 23.14 | C |
| ATOM | 2906 | CD | GLU | B | 166 | -37.112 | -19.776 | 110.263 | 1.00 | 35.69 | C |
| ATOM | 2907 | OE1 | GLU | B | 166 | -37.691 | -20.801 | 110.689 | 1.00 | 45.01 | O |
| ATOM | 2908 | OE2 | GLU | B | 166 | -37.715 | -18.853 | 109.661 | 1.00 | 37.00 | O1- |

| | | | | | | | | | | | |
|---|---|---|---|---|---|---|---|---|---|---|---|
| ATOM | 2909 | N | GLN | B | 167 | -36.061 | -22.138 | 114.120 | 1.00 | 29.35 | N |
| ATOM | 2910 | CA | GLN | B | 167 | -35.749 | -23.146 | 115.126 | 1.00 | 27.70 | C |
| ATOM | 2911 | C | GLN | B | 167 | -34.250 | -23.426 | 115.143 | 1.00 | 26.23 | C |
| ATOM | 2912 | O | GLN | B | 167 | -33.622 | -23.530 | 114.093 | 1.00 | 25.94 | O |
| ATOM | 2913 | CB | GLN | B | 167 | -36.538 | -24.406 | 114.823 | 1.00 | 25.14 | C |
| ATOM | 2914 | CG | GLN | B | 167 | -36.995 | -25.171 | 116.012 | 1.00 | 27.67 | C |
| ATOM | 2915 | CD | GLN | B | 167 | -38.037 | -26.211 | 115.661 | 1.00 | 25.13 | C |
| ATOM | 2916 | OE1 | GLN | B | 167 | -38.324 | -26.473 | 114.489 | 1.00 | 27.17 | O |
| ATOM | 2917 | NE2 | GLN | B | 167 | -38.591 | -26.819 | 116.672 | 1.00 | 23.92 | N |
| ATOM | 2918 | N | ASP | B | 168 | -33.660 | -23.496 | 116.338 | 1.00 | 30.80 | N |
| ATOM | 2919 | CA | ASP | B | 168 | -32.202 | -23.493 | 116.448 | 1.00 | 28.93 | C |
| ATOM | 2920 | C | ASP | B | 168 | -31.602 | -24.806 | 115.962 | 1.00 | 31.78 | C |
| ATOM | 2921 | O | ASP | B | 168 | -31.981 | -25.882 | 116.433 | 1.00 | 30.59 | O |
| ATOM | 2922 | CB | ASP | B | 168 | -31.771 | -23.268 | 117.895 | 1.00 | 31.58 | C |
| ATOM | 2923 | CG | ASP | B | 168 | -30.269 | -23.104 | 118.028 | 1.00 | 34.74 | C |
| ATOM | 2924 | OD1 | ASP | B | 168 | -29.610 | -24.086 | 118.410 | 1.00 | 36.21 | O1- |
| ATOM | 2925 | OD2 | ASP | B | 168 | -29.737 | -22.026 | 117.677 | 1.00 | 38.15 | O |
| ATOM | 2926 | N | SER | B | 169 | -30.583 | -24.714 | 115.101 | 1.00 | 37.85 | N |
| ATOM | 2927 | CA | SER | B | 169 | -30.001 | -25.906 | 114.488 | 1.00 | 32.86 | C |
| ATOM | 2928 | C | SER | B | 169 | -29.342 | -26.845 | 115.487 | 1.00 | 33.78 | C |
| ATOM | 2929 | O | SER | B | 169 | -29.068 | -27.992 | 115.126 | 1.00 | 37.40 | O |
| ATOM | 2930 | CB | SER | B | 169 | -28.999 | -25.515 | 113.412 | 1.00 | 35.71 | C |
| ATOM | 2931 | OG | SER | B | 169 | -27.919 | -24.817 | 113.973 | 1.00 | 45.05 | O |
| ATOM | 2932 | N | LYS | B | 170 | -29.105 | -26.415 | 116.725 | 1.00 | 35.30 | N |
| ATOM | 2933 | CA | LYS | B | 170 | -28.484 | -27.260 | 117.742 | 1.00 | 32.69 | C |
| ATOM | 2934 | C | LYS | B | 170 | -29.445 | -27.779 | 118.816 | 1.00 | 33.51 | C |
| ATOM | 2935 | O | LYS | B | 170 | -29.365 | -28.957 | 119.161 | 1.00 | 33.92 | O |
| ATOM | 2936 | CB | LYS | B | 170 | -27.311 | -26.510 | 118.389 | 1.00 | 32.22 | C |
| ATOM | 2937 | CG | LYS | B | 170 | -26.523 | -27.313 | 119.398 | 1.00 | 35.45 | C |
| ATOM | 2938 | CD | LYS | B | 170 | -25.696 | -26.399 | 120.284 | 1.00 | 39.33 | C |
| ATOM | 2939 | CE | LYS | B | 170 | -24.687 | -27.150 | 121.125 | 1.00 | 41.85 | C |
| ATOM | 2940 | NZ | LYS | B | 170 | -23.466 | -26.291 | 121.260 | 1.00 | 49.14 | N1+ |
| ATOM | 2941 | N | ASP | B | 171 | -30.386 | -26.985 | 119.360 | 1.00 | 29.53 | N |
| ATOM | 2942 | CA | ASP | B | 171 | -31.298 | -27.535 | 120.366 | 1.00 | 24.54 | C |
| ATOM | 2943 | C | ASP | B | 171 | -32.772 | -27.335 | 120.036 | 1.00 | 25.46 | C |
| ATOM | 2944 | O | ASP | B | 171 | -33.615 | -27.519 | 120.926 | 1.00 | 22.30 | O |
| ATOM | 2945 | CB | ASP | B | 171 | -31.001 | -27.011 | 121.795 | 1.00 | 25.26 | C |
| ATOM | 2946 | CG | ASP | B | 171 | -31.151 | -25.471 | 121.965 | 1.00 | 35.81 | C |
| ATOM | 2947 | OD1 | ASP | B | 171 | -31.871 | -24.789 | 121.205 | 1.00 | 38.80 | O |
| ATOM | 2948 | OD2 | ASP | B | 171 | -30.555 | -24.925 | 122.927 | 1.00 | 42.26 | O1- |
| ATOM | 2949 | N | SER | B | 172 | -33.096 | -26.958 | 118.791 | 1.00 | 24.40 | N |
| ATOM | 2950 | CA | SER | B | 172 | -34.458 | -26.783 | 118.271 | 1.00 | 22.81 | C |
| ATOM | 2951 | C | SER | B | 172 | -35.296 | -25.735 | 119.023 | 1.00 | 29.46 | C |
| ATOM | 2952 | O | SER | B | 172 | -36.540 | -25.777 | 118.980 | 1.00 | 30.46 | O |
| ATOM | 2953 | CB | SER | B | 172 | -35.204 | -28.122 | 118.240 | 1.00 | 27.26 | C |
| ATOM | 2954 | OG | SER | B | 172 | -34.476 | -29.076 | 117.480 | 1.00 | 29.50 | O |
| ATOM | 2955 | N | THR | B | 173 | -34.685 | -24.758 | 119.688 | 1.00 | 26.53 | N |
| ATOM | 2956 | CA | THR | B | 173 | -35.479 | -23.750 | 120.371 | 1.00 | 30.13 | C |
| ATOM | 2957 | C | THR | B | 173 | -35.667 | -22.512 | 119.487 | 1.00 | 28.94 | C |
| ATOM | 2958 | O | THR | B | 173 | -35.137 | -22.397 | 118.377 | 1.00 | 28.80 | O |
| ATOM | 2959 | CB | THR | B | 173 | -34.869 | -23.380 | 121.742 | 1.00 | 29.06 | C |
| ATOM | 2960 | OG1 | THR | B | 173 | -33.536 | -22.886 | 121.592 | 1.00 | 27.56 | O |
| ATOM | 2961 | CG2 | THR | B | 173 | -34.849 | -24.577 | 122.671 | 1.00 | 23.15 | C |
| ATOM | 2962 | N | TYR | B | 174 | -36.479 | -21.598 | 119.989 | 1.00 | 27.86 | N |
| ATOM | 2963 | CA | TYR | B | 174 | -36.710 | -20.306 | 119.375 | 1.00 | 27.59 | C |
| ATOM | 2964 | C | TYR | B | 174 | -36.103 | -19.210 | 120.245 | 1.00 | 28.01 | C |
| ATOM | 2965 | O | TYR | B | 174 | -35.906 | -19.388 | 121.452 | 1.00 | 28.60 | O |
| ATOM | 2966 | CB | TYR | B | 174 | -38.210 | -20.069 | 119.181 | 1.00 | 27.30 | C |
| ATOM | 2967 | CG | TYR | B | 174 | -38.818 | -20.983 | 118.147 | 1.00 | 30.86 | C |
| ATOM | 2968 | CD1 | TYR | B | 174 | -38.743 | -20.672 | 116.791 | 1.00 | 26.78 | C |
| ATOM | 2969 | CD2 | TYR | B | 174 | -39.452 | -22.171 | 118.521 | 1.00 | 31.18 | C |
| ATOM | 2970 | CE1 | TYR | B | 174 | -39.283 | -21.496 | 115.842 | 1.00 | 27.76 | C |
| ATOM | 2971 | CE2 | TYR | B | 174 | -39.994 | -23.016 | 117.567 | 1.00 | 30.49 | C |
| ATOM | 2972 | CZ | TYR | B | 174 | -39.906 | -22.666 | 116.224 | 1.00 | 31.46 | C |
| ATOM | 2973 | OH | TYR | B | 174 | -40.448 | -23.480 | 115.262 | 1.00 | 32.46 | O |
| ATOM | 2974 | N | SER | B | 175 | -35.773 | -18.087 | 119.621 | 1.00 | 26.84 | N |
| ATOM | 2975 | CA | SER | B | 175 | -35.396 | -16.886 | 120.355 | 1.00 | 28.52 | C |
| ATOM | 2976 | C | SER | B | 175 | -36.172 | -15.696 | 119.814 | 1.00 | 28.84 | C |
| ATOM | 2977 | O | SER | B | 175 | -36.595 | -15.687 | 118.656 | 1.00 | 24.93 | O |
| ATOM | 2978 | CB | SER | B | 175 | -33.904 | -16.608 | 120.274 | 1.00 | 31.06 | C |
| ATOM | 2979 | OG | SER | B | 175 | -33.199 | -17.479 | 121.133 | 1.00 | 35.19 | O |

| | | | | | | | | | | | |
|---|---|---|---|---|---|---|---|---|---|---|---|
| ATOM | 2980 | N | LEU | B | 176 | -36.367 | -14.694 | 120.671 | 1.00 | 29.11 | N |
| ATOM | 2981 | CA | LEU | B | 176 | -37.165 | -13.529 | 120.323 | 1.00 | 27.58 | C |
| ATOM | 2982 | C | LEU | B | 176 | -36.445 | -12.262 | 120.739 | 1.00 | 24.37 | C |
| ATOM | 2983 | O | LEU | B | 176 | -35.796 | -12.218 | 121.779 | 1.00 | 27.04 | O |
| ATOM | 2984 | CB | LEU | B | 176 | -38.553 | -13.589 | 120.973 | 1.00 | 29.13 | C |
| ATOM | 2985 | CG | LEU | B | 176 | -39.558 | -12.517 | 120.560 | 1.00 | 30.07 | C |
| ATOM | 2986 | CD1 | LEU | B | 176 | -40.920 | -13.129 | 120.631 | 1.00 | 30.39 | C |
| ATOM | 2987 | CD2 | LEU | B | 176 | -39.485 | -11.303 | 121.483 | 1.00 | 24.93 | C |
| ATOM | 2988 | N | SER | B | 177 | -36.556 | -11.235 | 119.905 | 1.00 | 29.33 | N |
| ATOM | 2989 | CA | SER | B | 177 | -35.972 | -9.932 | 120.178 | 1.00 | 26.82 | C |
| ATOM | 2990 | C | SER | B | 177 | -37.041 | -8.863 | 119.992 | 1.00 | 27.17 | C |
| ATOM | 2991 | O | SER | B | 177 | -37.669 | -8.809 | 118.934 | 1.00 | 25.25 | O |
| ATOM | 2992 | CB | SER | B | 177 | -34.782 | -9.694 | 119.255 | 1.00 | 26.28 | C |
| ATOM | 2993 | OG | SER | B | 177 | -34.307 | -8.377 | 119.379 | 1.00 | 27.89 | O |
| ATOM | 2994 | N | SER | B | 178 | -37.232 | -8.006 | 121.013 | 1.00 | 25.92 | N |
| ATOM | 2995 | CA | SER | B | 178 | -38.255 | -6.966 | 121.020 | 1.00 | 23.84 | C |
| ATOM | 2996 | C | SER | B | 178 | -37.624 | -5.603 | 121.261 | 1.00 | 27.80 | C |
| ATOM | 2997 | O | SER | B | 178 | -36.822 | -5.443 | 122.189 | 1.00 | 29.43 | O |
| ATOM | 2998 | CB | SER | B | 178 | -39.308 | -7.226 | 122.096 | 1.00 | 28.27 | C |
| ATOM | 2999 | OG | SER | B | 178 | -40.307 | -6.215 | 122.090 | 1.00 | 26.54 | O |
| ATOM | 3000 | N | THR | B | 179 | -38.007 | -4.617 | 120.444 | 1.00 | 26.02 | N |
| ATOM | 3001 | CA | THR | B | 179 | -37.450 | -3.266 | 120.535 | 1.00 | 30.99 | C |
| ATOM | 3002 | C | THR | B | 179 | -38.536 | -2.247 | 120.809 | 1.00 | 25.23 | C |
| ATOM | 3003 | O | THR | B | 179 | -39.357 | -1.967 | 119.938 | 1.00 | 24.43 | O |
| ATOM | 3004 | CB | THR | B | 179 | -36.714 | -2.839 | 119.274 | 1.00 | 31.29 | C |
| ATOM | 3005 | OG1 | THR | B | 179 | -35.681 | -3.781 | 118.962 | 1.00 | 35.76 | O |
| ATOM | 3006 | CG2 | THR | B | 179 | -36.121 | -1.472 | 119.512 | 1.00 | 23.65 | C |
| ATOM | 3007 | N | LEU | B | 180 | -38.489 | -1.662 | 121.997 | 1.00 | 26.49 | N |
| ATOM | 3008 | CA | LEU | B | 180 | -39.263 | -0.482 | 122.349 | 1.00 | 28.48 | C |
| ATOM | 3009 | C | LEU | B | 180 | -38.502 | 0.769 | 121.939 | 1.00 | 24.67 | C |
| ATOM | 3010 | O | LEU | B | 180 | -37.356 | 0.949 | 122.350 | 1.00 | 26.90 | O |
| ATOM | 3011 | CB | LEU | B | 180 | -39.510 | -0.482 | 123.855 | 1.00 | 30.84 | C |
| ATOM | 3012 | CG | LEU | B | 180 | -40.291 | 0.643 | 124.498 | 1.00 | 34.83 | C |
| ATOM | 3013 | CD1 | LEU | B | 180 | -41.724 | 0.580 | 123.976 | 1.00 | 34.95 | C |
| ATOM | 3014 | CD2 | LEU | B | 180 | -40.215 | 0.471 | 126.015 | 1.00 | 31.30 | C |
| ATOM | 3015 | N | THR | B | 181 | -39.142 | 1.642 | 121.161 | 1.00 | 25.09 | N |
| ATOM | 3016 | CA | THR | B | 181 | -38.529 | 2.892 | 120.712 | 1.00 | 34.09 | C |
| ATOM | 3017 | C | THR | B | 181 | -39.245 | 4.090 | 121.341 | 1.00 | 40.80 | C |
| ATOM | 3018 | O | THR | B | 181 | -40.484 | 4.142 | 121.354 | 1.00 | 36.26 | O |
| ATOM | 3019 | CB | THR | B | 181 | -38.548 | 3.005 | 119.181 | 1.00 | 33.92 | C |
| ATOM | 3020 | OG1 | THR | B | 181 | -37.870 | 1.881 | 118.610 | 1.00 | 36.57 | O |
| ATOM | 3021 | CG2 | THR | B | 181 | -37.793 | 4.230 | 118.744 | 1.00 | 36.41 | C |
| ATOM | 3022 | N | LEU | B | 182 | -38.452 | 5.031 | 121.882 | 1.00 | 36.61 | N |
| ATOM | 3023 | CA | LEU | B | 182 | -38.910 | 6.286 | 122.483 | 1.00 | 33.83 | C |
| ATOM | 3024 | C | LEU | B | 182 | -38.034 | 7.434 | 121.997 | 1.00 | 37.62 | C |
| ATOM | 3025 | O | LEU | B | 182 | -36.923 | 7.235 | 121.506 | 1.00 | 36.88 | O |
| ATOM | 3026 | CB | LEU | B | 182 | -38.837 | 6.287 | 124.017 | 1.00 | 39.93 | C |
| ATOM | 3027 | CG | LEU | B | 182 | -39.550 | 5.271 | 124.901 | 1.00 | 43.75 | C |
| ATOM | 3028 | CD1 | LEU | B | 182 | -39.188 | 5.503 | 126.364 | 1.00 | 42.80 | C |
| ATOM | 3029 | CD2 | LEU | B | 182 | -41.051 | 5.401 | 124.698 | 1.00 | 48.91 | C |
| ATOM | 3030 | N | SER | B | 183 | -38.515 | 8.653 | 122.183 | 1.00 | 40.17 | N |
| ATOM | 3031 | CA | SER | B | 183 | -37.616 | 9.784 | 122.023 | 1.00 | 39.39 | C |
| ATOM | 3032 | C | SER | B | 183 | -36.639 | 9.844 | 123.195 | 1.00 | 39.08 | C |
| ATOM | 3033 | O | SER | B | 183 | -36.882 | 9.278 | 124.259 | 1.00 | 39.26 | O |
| ATOM | 3034 | CB | SER | B | 183 | -38.401 | 11.088 | 121.963 | 1.00 | 38.95 | C |
| ATOM | 3035 | OG | SER | B | 183 | -39.031 | 11.322 | 123.210 | 1.00 | 35.92 | O |
| ATOM | 3036 | N | LYS | B | 184 | -35.510 | 10.530 | 122.986 | 1.00 | 42.26 | N |
| ATOM | 3037 | CA | LYS | B | 184 | -34.617 | 10.803 | 124.109 | 1.00 | 40.17 | C |
| ATOM | 3038 | C | LYS | B | 184 | -35.348 | 11.566 | 125.208 | 1.00 | 41.28 | C |
| ATOM | 3039 | O | LYS | B | 184 | -35.138 | 11.310 | 126.401 | 1.00 | 38.53 | O |
| ATOM | 3040 | CB | LYS | B | 184 | -33.405 | 11.605 | 123.646 | 1.00 | 42.87 | C |
| ATOM | 3041 | CG | LYS | B | 184 | -32.462 | 11.986 | 124.806 | 1.00 | 52.77 | C |
| ATOM | 3042 | CD | LYS | B | 184 | -31.228 | 12.771 | 124.339 | 1.00 | 51.17 | C |
| ATOM | 3043 | CE | LYS | B | 184 | -30.325 | 13.153 | 125.500 | 1.00 | 56.17 | C |
| ATOM | 3044 | NZ | LYS | B | 184 | -30.191 | 14.632 | 125.670 | 1.00 | 63.96 | N1+ |
| ATOM | 3045 | N | ALA | B | 185 | -36.219 | 12.500 | 124.816 | 1.00 | 39.51 | N |
| ATOM | 3046 | CA | ALA | B | 185 | -36.995 | 13.275 | 125.780 | 1.00 | 38.41 | C |
| ATOM | 3047 | C | ALA | B | 185 | -37.914 | 12.389 | 126.613 | 1.00 | 39.82 | C |
| ATOM | 3048 | O | ALA | B | 185 | -37.897 | 12.456 | 127.844 | 1.00 | 44.20 | O |
| ATOM | 3049 | CB | ALA | B | 185 | -37.806 | 14.346 | 125.057 | 1.00 | 33.82 | C |
| ATOM | 3050 | N | ASP | B | 186 | -38.764 | 11.591 | 125.959 | 1.00 | 42.91 | N |

| | | | | | | | | | | | |
|---|---|---|---|---|---|---|---|---|---|---|---|
| ATOM | 3051 | CA | ASP | B | 186 | -39.646 | 10.690 | 126.697 | 1.00 | 37.37 | C |
| ATOM | 3052 | C | ASP | B | 186 | -38.844 | 9.750 | 127.573 | 1.00 | 37.99 | C |
| ATOM | 3053 | O | ASP | B | 186 | -39.192 | 9.506 | 128.736 | 1.00 | 37.79 | O |
| ATOM | 3054 | CB | ASP | B | 186 | -40.512 | 9.876 | 125.739 | 1.00 | 42.26 | C |
| ATOM | 3055 | CG | ASP | B | 186 | -41.589 | 10.703 | 125.065 | 1.00 | 48.82 | C |
| ATOM | 3056 | OD1 | ASP | B | 186 | -42.264 | 11.496 | 125.752 | 1.00 | 49.68 | O |
| ATOM | 3057 | OD2 | ASP | B | 186 | -41.778 | 10.531 | 123.839 | 1.00 | 61.13 | O1- |
| ATOM | 3058 | N | TYR | B | 187 | -37.781 | 9.181 | 127.006 | 1.00 | 41.31 | N |
| ATOM | 3059 | CA | TYR | B | 187 | -36.932 | 8.262 | 127.745 | 1.00 | 37.24 | C |
| ATOM | 3060 | C | TYR | B | 187 | -36.398 | 8.907 | 129.016 | 1.00 | 35.05 | C |
| ATOM | 3061 | O | TYR | B | 187 | -36.349 | 8.272 | 130.075 | 1.00 | 36.76 | O |
| ATOM | 3062 | CB | TYR | B | 187 | -35.786 | 7.785 | 126.852 | 1.00 | 37.33 | C |
| ATOM | 3063 | CG | TYR | B | 187 | -34.823 | 6.915 | 127.604 | 1.00 | 37.18 | C |
| ATOM | 3064 | CD1 | TYR | B | 187 | -35.223 | 5.663 | 128.068 | 1.00 | 34.09 | C |
| ATOM | 3065 | CD2 | TYR | B | 187 | -33.539 | 7.359 | 127.900 | 1.00 | 27.91 | C |
| ATOM | 3066 | CE1 | TYR | B | 187 | -34.363 | 4.870 | 128.780 | 1.00 | 33.76 | C |
| ATOM | 3067 | CE2 | TYR | B | 187 | -32.678 | 6.577 | 128.618 | 1.00 | 30.33 | C |
| ATOM | 3068 | CZ | TYR | B | 187 | -33.090 | 5.328 | 129.058 | 1.00 | 31.48 | C |
| ATOM | 3069 | OH | TYR | B | 187 | -32.233 | 4.528 | 129.785 | 1.00 | 31.99 | O |
| ATOM | 3070 | N | GLU | B | 188 | -35.991 | 10.166 | 128.935 | 1.00 | 39.77 | N |
| ATOM | 3071 | CA | GLU | B | 188 | -35.344 | 10.782 | 130.083 | 1.00 | 44.77 | C |
| ATOM | 3072 | C | GLU | B | 188 | -36.325 | 11.334 | 131.097 | 1.00 | 40.36 | C |
| ATOM | 3073 | O | GLU | B | 188 | -35.879 | 11.832 | 132.131 | 1.00 | 41.81 | O |
| ATOM | 3074 | CB | GLU | B | 188 | -34.358 | 11.849 | 129.625 | 1.00 | 39.20 | C |
| ATOM | 3075 | CG | GLU | B | 188 | -32.978 | 11.224 | 129.620 | 1.00 | 45.15 | C |
| ATOM | 3076 | CD | GLU | B | 188 | -31.974 | 11.914 | 128.723 | 1.00 | 56.05 | C |
| ATOM | 3077 | OE1 | GLU | B | 188 | -32.317 | 12.924 | 128.049 | 1.00 | 48.75 | O |
| ATOM | 3078 | OE2 | GLU | B | 188 | -30.831 | 11.394 | 128.688 | 1.00 | 59.27 | O1- |
| ATOM | 3079 | N | LYS | B | 189 | -37.630 | 11.197 | 130.852 | 1.00 | 37.43 | N |
| ATOM | 3080 | CA | LYS | B | 189 | -38.687 | 11.560 | 131.788 | 1.00 | 38.07 | C |
| ATOM | 3081 | C | LYS | B | 189 | -39.236 | 10.363 | 132.579 | 1.00 | 40.62 | C |
| ATOM | 3082 | O | LYS | B | 189 | -40.315 | 10.465 | 133.167 | 1.00 | 46.60 | O |
| ATOM | 3083 | CB | LYS | B | 189 | -39.813 | 12.270 | 131.030 | 1.00 | 37.89 | C |
| ATOM | 3084 | CG | LYS | B | 189 | -39.327 | 13.568 | 130.380 | 1.00 | 51.23 | C |
| ATOM | 3085 | CD | LYS | B | 189 | -40.333 | 14.215 | 129.404 | 1.00 | 62.14 | C |
| ATOM | 3086 | CE | LYS | B | 189 | -39.717 | 15.470 | 128.720 | 1.00 | 60.71 | C |
| ATOM | 3087 | NZ | LYS | B | 189 | -40.587 | 16.123 | 127.684 | 1.00 | 51.31 | N1+ |
| ATOM | 3088 | N | HIS | B | 190 | -38.541 | 9.223 | 132.588 | 1.00 | 41.83 | N |
| ATOM | 3089 | CA | HIS | B | 190 | -39.025 | 8.026 | 133.271 | 1.00 | 35.94 | C |
| ATOM | 3090 | C | HIS | B | 190 | -37.865 | 7.259 | 133.879 | 1.00 | 35.72 | C |
| ATOM | 3091 | O | HIS | B | 190 | -36.701 | 7.484 | 133.543 | 1.00 | 36.41 | O |
| ATOM | 3092 | CB | HIS | B | 190 | -39.810 | 7.120 | 132.337 | 1.00 | 37.87 | C |
| ATOM | 3093 | CG | HIS | B | 190 | -41.069 | 7.745 | 131.840 | 1.00 | 37.33 | C |
| ATOM | 3094 | ND1 | HIS | B | 190 | -41.173 | 8.323 | 130.596 | 1.00 | 40.44 | N |
| ATOM | 3095 | CD2 | HIS | B | 190 | -42.264 | 7.926 | 132.442 | 1.00 | 37.28 | C |
| ATOM | 3096 | CE1 | HIS | B | 190 | -42.386 | 8.813 | 130.442 | 1.00 | 36.52 | C |
| ATOM | 3097 | NE2 | HIS | B | 190 | -43.068 | 8.583 | 131.548 | 1.00 | 42.61 | N |
| ATOM | 3098 | N | LYS | B | 191 | -38.188 | 6.365 | 134.806 | 1.00 | 32.95 | N |
| ATOM | 3099 | CA | LYS | B | 191 | -37.139 | 5.707 | 135.580 | 1.00 | 44.01 | C |
| ATOM | 3100 | C | LYS | B | 191 | -37.065 | 4.204 | 135.352 | 1.00 | 37.37 | C |
| ATOM | 3101 | O | LYS | B | 191 | -35.988 | 3.687 | 135.032 | 1.00 | 37.81 | O |
| ATOM | 3102 | CB | LYS | B | 191 | -37.316 | 6.017 | 137.084 | 1.00 | 41.79 | C |
| ATOM | 3103 | CG | LYS | B | 191 | -36.346 | 5.312 | 138.042 | 1.00 | 43.26 | C |
| ATOM | 3104 | CD | LYS | B | 191 | -36.731 | 5.623 | 139.511 | 1.00 | 54.07 | C |
| ATOM | 3105 | CE | LYS | B | 191 | -35.668 | 5.177 | 140.515 | 1.00 | 56.60 | C |
| ATOM | 3106 | NZ | LYS | B | 191 | -36.031 | 5.546 | 141.905 | 1.00 | 51.73 | N1+ |
| ATOM | 3107 | N | VAL | B | 192 | -38.164 | 3.482 | 135.530 | 1.00 | 31.57 | N |
| ATOM | 3108 | CA | VAL | B | 192 | -38.156 | 2.027 | 135.503 | 1.00 | 34.89 | C |
| ATOM | 3109 | C | VAL | B | 192 | -38.596 | 1.564 | 134.126 | 1.00 | 35.04 | C |
| ATOM | 3110 | O | VAL | B | 192 | -39.687 | 1.918 | 133.661 | 1.00 | 35.31 | O |
| ATOM | 3111 | CB | VAL | B | 192 | -39.054 | 1.439 | 136.599 | 1.00 | 34.98 | C |
| ATOM | 3112 | CG1 | VAL | B | 192 | -39.122 | -0.073 | 136.464 | 1.00 | 30.15 | C |
| ATOM | 3113 | CG2 | VAL | B | 192 | -38.522 | 1.842 | 137.965 | 1.00 | 36.73 | C |
| ATOM | 3114 | N | TYR | B | 193 | -37.737 | 0.785 | 133.473 | 1.00 | 36.15 | N |
| ATOM | 3115 | CA | TYR | B | 193 | -38.019 | 0.172 | 132.181 | 1.00 | 35.85 | C |
| ATOM | 3116 | C | TYR | B | 193 | -38.078 | -1.327 | 132.381 | 1.00 | 29.50 | C |
| ATOM | 3117 | O | TYR | B | 193 | -37.178 | -1.898 | 132.995 | 1.00 | 31.86 | O |
| ATOM | 3118 | CB | TYR | B | 193 | -36.958 | 0.576 | 131.150 | 1.00 | 28.31 | C |
| ATOM | 3119 | CG | TYR | B | 193 | -37.137 | 2.020 | 130.781 | 1.00 | 32.21 | C |
| ATOM | 3120 | CD1 | TYR | B | 193 | -36.656 | 3.034 | 131.613 | 1.00 | 34.12 | C |
| ATOM | 3121 | CD2 | TYR | B | 193 | -37.831 | 2.384 | 129.628 | 1.00 | 32.20 | C |

| | | | | | | | | | | | |
|---|---|---|---|---|---|---|---|---|---|---|---|
| ATOM | 3122 | CE1 | TYR | B | 193 | -36.859 | 4.366 | 131.313 | 1.00 | 31.90 | C |
| ATOM | 3123 | CE2 | TYR | B | 193 | -38.023 | 3.724 | 129.299 | 1.00 | 38.14 | C |
| ATOM | 3124 | CZ | TYR | B | 193 | -37.536 | 4.707 | 130.163 | 1.00 | 37.14 | C |
| ATOM | 3125 | OH | TYR | B | 193 | -37.717 | 6.025 | 129.872 | 1.00 | 39.52 | O |
| ATOM | 3126 | N | ALA | B | 194 | -39.149 | -1.954 | 131.908 | 1.00 | 28.74 | N |
| ATOM | 3127 | CA | ALA | B | 194 | -39.300 | -3.384 | 132.148 | 1.00 | 34.71 | C |
| ATOM | 3128 | C | ALA | B | 194 | -39.970 | -4.081 | 130.963 | 1.00 | 33.72 | C |
| ATOM | 3129 | O | ALA | B | 194 | -40.748 | -3.479 | 130.211 | 1.00 | 34.66 | O |
| ATOM | 3130 | CB | ALA | B | 194 | -40.083 | -3.634 | 133.442 | 1.00 | 29.91 | C |
| ATOM | 3131 | N | CYS | B | 195 | -39.623 | -5.349 | 130.776 | 1.00 | 32.36 | N |
| ATOM | 3132 | CA | CYS | B | 195 | -40.382 | -6.251 | 129.919 | 1.00 | 37.46 | C |
| ATOM | 3133 | C | CYS | B | 195 | -40.814 | -7.461 | 130.732 | 1.00 | 40.08 | C |
| ATOM | 3134 | O | CYS | B | 195 | -40.045 | -8.000 | 131.544 | 1.00 | 39.11 | O |
| ATOM | 3135 | CB | CYS | B | 195 | -39.615 | -6.703 | 128.639 | 1.00 | 33.36 | C |
| ATOM | 3136 | SG | CYS | B | 195 | -37.984 | -7.394 | 128.903 | 1.00 | 49.70 | S |
| ATOM | 3137 | N | GLU | B | 196 | -42.077 | -7.822 | 130.550 | 1.00 | 36.65 | N |
| ATOM | 3138 | CA | GLU | B | 196 | -42.702 | -8.939 | 131.228 | 1.00 | 37.99 | C |
| ATOM | 3139 | C | GLU | B | 196 | -42.961 | -10.009 | 130.181 | 1.00 | 33.89 | C |
| ATOM | 3140 | O | GLU | B | 196 | -43.502 | -9.711 | 129.111 | 1.00 | 30.27 | O |
| ATOM | 3141 | CB | GLU | B | 196 | -43.986 | -8.481 | 131.926 | 1.00 | 37.34 | C |
| ATOM | 3142 | CG | GLU | B | 196 | -44.748 | -9.548 | 132.707 | 1.00 | 42.18 | C |
| ATOM | 3143 | CD | GLU | B | 196 | -46.068 | -9.008 | 133.278 | 1.00 | 51.19 | C |
| ATOM | 3144 | OE1 | GLU | B | 196 | -46.538 | -7.957 | 132.796 | 1.00 | 55.09 | O |
| ATOM | 3145 | OE2 | GLU | B | 196 | -46.618 | -9.605 | 134.234 | 1.00 | 54.09 | O1- |
| ATOM | 3146 | N | VAL | B | 197 | -42.549 | -11.236 | 130.491 | 1.00 | 33.59 | N |
| ATOM | 3147 | CA | VAL | B | 197 | -42.499 | -12.350 | 129.550 | 1.00 | 28.73 | C |
| ATOM | 3148 | C | VAL | B | 197 | -43.396 | -13.467 | 130.057 | 1.00 | 31.19 | C |
| ATOM | 3149 | O | VAL | B | 197 | -43.137 | -14.053 | 131.117 | 1.00 | 32.63 | O |
| ATOM | 3150 | CB | VAL | B | 197 | -41.057 | -12.851 | 129.355 | 1.00 | 29.79 | C |
| ATOM | 3151 | CG1 | VAL | B | 197 | -41.040 | -14.182 | 128.614 | 1.00 | 27.12 | C |
| ATOM | 3152 | CG2 | VAL | B | 197 | -40.238 | -11.808 | 128.589 | 1.00 | 28.85 | C |
| ATOM | 3153 | N | THR | B | 198 | -44.436 | -13.778 | 129.293 | 1.00 | 32.91 | N |
| ATOM | 3154 | CA | THR | B | 198 | -45.297 | -14.926 | 129.549 | 1.00 | 29.91 | C |
| ATOM | 3155 | C | THR | B | 198 | -45.009 | -16.037 | 128.547 | 1.00 | 32.14 | C |
| ATOM | 3156 | O | THR | B | 198 | -45.017 | -15.808 | 127.328 | 1.00 | 29.74 | O |
| ATOM | 3157 | CB | THR | B | 198 | -46.763 | -14.516 | 129.502 | 1.00 | 29.14 | C |
| ATOM | 3158 | OG1 | THR | B | 198 | -46.977 | -13.539 | 130.518 | 1.00 | 36.91 | O |
| ATOM | 3159 | CG2 | THR | B | 198 | -47.664 | -15.701 | 129.771 | 1.00 | 31.29 | C |
| ATOM | 3160 | N | HIS | B | 199 | -44.758 | -17.237 | 129.066 | 1.00 | 29.27 | N |
| ATOM | 3161 | CA | HIS | B | 199 | -44.464 | -18.393 | 128.233 | 1.00 | 32.48 | C |
| ATOM | 3162 | C | HIS | B | 199 | -44.929 | -19.638 | 128.964 | 1.00 | 34.28 | C |
| ATOM | 3163 | O | HIS | B | 199 | -44.998 | -19.645 | 130.194 | 1.00 | 35.68 | O |
| ATOM | 3164 | CB | HIS | B | 199 | -42.975 | -18.520 | 127.932 | 1.00 | 28.76 | C |
| ATOM | 3165 | CG | HIS | B | 199 | -42.659 | -19.599 | 126.950 | 1.00 | 31.19 | C |
| ATOM | 3166 | ND1 | HIS | B | 199 | -42.284 | -20.867 | 127.331 | 1.00 | 29.79 | N |
| ATOM | 3167 | CD2 | HIS | B | 199 | -42.657 | -19.594 | 125.595 | 1.00 | 31.07 | C |
| ATOM | 3168 | CE1 | HIS | B | 199 | -42.066 | -21.596 | 126.252 | 1.00 | 29.75 | C |
| ATOM | 3169 | NE2 | HIS | B | 199 | -42.280 | -20.845 | 125.185 | 1.00 | 26.47 | N |
| ATOM | 3170 | N | GLN | B | 200 | -45.239 | -20.693 | 128.204 | 1.00 | 28.40 | N |
| ATOM | 3171 | CA | GLN | B | 200 | -45.833 | -21.862 | 128.839 | 1.00 | 28.53 | C |
| ATOM | 3172 | C | GLN | B | 200 | -44.841 | -22.620 | 129.706 | 1.00 | 34.07 | C |
| ATOM | 3173 | O | GLN | B | 200 | -45.270 | -23.425 | 130.535 | 1.00 | 36.20 | O |
| ATOM | 3174 | CB | GLN | B | 200 | -46.471 | -22.791 | 127.805 | 1.00 | 34.90 | C |
| ATOM | 3175 | CG | GLN | B | 200 | -45.638 | -23.987 | 127.362 | 1.00 | 36.70 | C |
| ATOM | 3176 | CD | GLN | B | 200 | -46.492 | -25.092 | 126.712 | 1.00 | 43.78 | C |
| ATOM | 3177 | OE1 | GLN | B | 200 | -46.443 | -26.257 | 127.124 | 1.00 | 43.09 | O |
| ATOM | 3178 | NE2 | GLN | B | 200 | -47.268 | -24.723 | 125.692 | 1.00 | 41.04 | N |
| ATOM | 3179 | N | GLY | B | 201 | -43.538 | -22.378 | 129.558 | 1.00 | 35.51 | N |
| ATOM | 3180 | CA | GLY | B | 201 | -42.576 | -22.968 | 130.464 | 1.00 | 29.44 | C |
| ATOM | 3181 | C | GLY | B | 201 | -42.338 | -22.163 | 131.720 | 1.00 | 33.22 | C |
| ATOM | 3182 | O | GLY | B | 201 | -41.524 | -22.553 | 132.563 | 1.00 | 33.63 | O |
| ATOM | 3183 | N | LEU | B | 202 | -43.062 | -21.058 | 131.884 | 1.00 | 32.15 | N |
| ATOM | 3184 | CA | LEU | B | 202 | -42.986 | -20.209 | 133.061 | 1.00 | 36.14 | C |
| ATOM | 3185 | C | LEU | B | 202 | -44.298 | -20.336 | 133.808 | 1.00 | 37.68 | C |
| ATOM | 3186 | O | LEU | B | 202 | -45.352 | -20.003 | 133.256 | 1.00 | 37.45 | O |
| ATOM | 3187 | CB | LEU | B | 202 | -42.765 | -18.743 | 132.669 | 1.00 | 36.59 | C |
| ATOM | 3188 | CG | LEU | B | 202 | -41.529 | -18.334 | 131.867 | 1.00 | 27.10 | C |
| ATOM | 3189 | CD1 | LEU | B | 202 | -41.560 | -16.866 | 131.516 | 1.00 | 29.06 | C |
| ATOM | 3190 | CD2 | LEU | B | 202 | -40.313 | -18.626 | 132.669 | 1.00 | 31.65 | C |
| ATOM | 3191 | N | SER | B | 203 | -44.227 | -20.741 | 135.082 | 1.00 | 42.21 | N |
| ATOM | 3192 | CA | SER | B | 203 | -45.444 | -20.866 | 135.880 | 1.00 | 41.93 | C |

| | | | | | | | | | | | |
|---|---|---|---|---|---|---|---|---|---|---|---|
| ATOM | 3193 | C | SER | B | 203 | -46.098 | -19.513 | 136.127 | 1.00 | 42.43 | C |
| ATOM | 3194 | O | SER | B | 203 | -47.312 | -19.458 | 136.334 | 1.00 | 41.82 | O |
| ATOM | 3195 | CB | SER | B | 203 | -45.145 | -21.561 | 137.214 | 1.00 | 44.30 | C |
| ATOM | 3196 | OG | SER | B | 203 | -44.114 | -20.886 | 137.919 | 1.00 | 57.08 | O |
| ATOM | 3197 | N | SER | B | 204 | -45.333 | -18.425 | 136.065 | 1.00 | 41.83 | N |
| ATOM | 3198 | CA | SER | B | 204 | -45.886 | -17.077 | 136.094 | 1.00 | 43.80 | C |
| ATOM | 3199 | C | SER | B | 204 | -44.928 | -16.136 | 135.369 | 1.00 | 41.32 | C |
| ATOM | 3200 | O | SER | B | 204 | -43.777 | -16.501 | 135.094 | 1.00 | 39.18 | O |
| ATOM | 3201 | CB | SER | B | 204 | -46.175 | -16.616 | 137.537 | 1.00 | 37.72 | C |
| ATOM | 3202 | OG | SER | B | 204 | -45.011 | -16.540 | 138.333 | 1.00 | 37.17 | O |
| ATOM | 3203 | N | PRO | B | 205 | -45.400 | -14.953 | 134.971 | 1.00 | 34.11 | N |
| ATOM | 3204 | CA | PRO | B | 205 | -44.574 | -14.046 | 134.171 | 1.00 | 33.10 | C |
| ATOM | 3205 | C | PRO | B | 205 | -43.256 | -13.665 | 134.836 | 1.00 | 39.09 | C |
| ATOM | 3206 | O | PRO | B | 205 | -43.148 | -13.550 | 136.053 | 1.00 | 45.40 | O |
| ATOM | 3207 | CB | PRO | B | 205 | -45.475 | -12.821 | 133.999 | 1.00 | 37.31 | C |
| ATOM | 3208 | CG | PRO | B | 205 | -46.835 | -13.372 | 134.001 | 1.00 | 39.03 | C |
| ATOM | 3209 | CD | PRO | B | 205 | -46.817 | -14.553 | 134.944 | 1.00 | 38.32 | C |
| ATOM | 3210 | N | VAL | B | 206 | -42.232 | -13.502 | 134.016 | 1.00 | 34.71 | N |
| ATOM | 3211 | CA | VAL | B | 206 | -40.922 | -13.075 | 134.467 | 1.00 | 35.91 | C |
| ATOM | 3212 | C | VAL | B | 206 | -40.692 | -11.645 | 133.984 | 1.00 | 38.85 | C |
| ATOM | 3213 | O | VAL | B | 206 | -40.938 | -11.328 | 132.811 | 1.00 | 36.12 | O |
| ATOM | 3214 | CB | VAL | B | 206 | -39.836 | -14.036 | 133.951 | 1.00 | 34.83 | C |
| ATOM | 3215 | CG1 | VAL | B | 206 | -38.475 | -13.474 | 134.183 | 1.00 | 32.65 | C |
| ATOM | 3216 | CG2 | VAL | B | 206 | -39.970 | -15.355 | 134.650 | 1.00 | 35.57 | C |
| ATOM | 3217 | N | THR | B | 207 | -40.236 | -10.774 | 134.883 | 1.00 | 38.08 | N |
| ATOM | 3218 | CA | THR | B | 207 | -39.870 | -9.416 | 134.504 | 1.00 | 40.84 | C |
| ATOM | 3219 | C | THR | B | 207 | -38.378 | -9.204 | 134.736 | 1.00 | 39.54 | C |
| ATOM | 3220 | O | THR | B | 207 | -37.836 | -9.618 | 135.761 | 1.00 | 42.64 | O |
| ATOM | 3221 | CB | THR | B | 207 | -40.678 | -8.361 | 135.276 | 1.00 | 37.84 | C |
| ATOM | 3222 | OG1 | THR | B | 207 | -42.077 | -8.538 | 135.017 | 1.00 | 43.59 | O |
| ATOM | 3223 | CG2 | THR | B | 207 | -40.291 | -6.972 | 134.837 | 1.00 | 32.49 | C |
| ATOM | 3224 | N | LYS | B | 208 | -37.715 | -8.590 | 133.762 | 1.00 | 38.06 | N |
| ATOM | 3225 | CA | LYS | B | 208 | -36.376 | -8.048 | 133.928 | 1.00 | 36.13 | C |
| ATOM | 3226 | C | LYS | B | 208 | -36.503 | -6.553 | 133.717 | 1.00 | 30.77 | C |
| ATOM | 3227 | O | LYS | B | 208 | -37.237 | -6.108 | 132.840 | 1.00 | 34.40 | O |
| ATOM | 3228 | CB | LYS | B | 208 | -35.349 | -8.617 | 132.922 | 1.00 | 32.55 | C |
| ATOM | 3229 | CG | LYS | B | 208 | -35.155 | -10.137 | 132.891 | 1.00 | 33.52 | C |
| ATOM | 3230 | CD | LYS | B | 208 | -34.907 | -10.808 | 134.236 | 1.00 | 35.71 | C |
| ATOM | 3231 | CE | LYS | B | 208 | -34.562 | -12.283 | 133.994 | 1.00 | 39.12 | C |
| ATOM | 3232 | NZ | LYS | B | 208 | -34.393 | -13.134 | 135.206 | 1.00 | 41.39 | N1+ |
| ATOM | 3233 | N | SER | B | 209 | -35.841 | -5.768 | 134.543 | 1.00 | 37.37 | N |
| ATOM | 3234 | CA | SER | B | 209 | -36.013 | -4.330 | 134.455 | 1.00 | 33.94 | C |
| ATOM | 3235 | C | SER | B | 209 | -34.700 | -3.653 | 134.776 | 1.00 | 29.43 | C |
| ATOM | 3236 | O | SER | B | 209 | -33.780 | -4.271 | 135.305 | 1.00 | 31.80 | O |
| ATOM | 3237 | CB | SER | B | 209 | -37.096 | -3.848 | 135.420 | 1.00 | 33.21 | C |
| ATOM | 3238 | OG | SER | B | 209 | -36.754 | -4.262 | 136.724 | 1.00 | 35.51 | O |
| ATOM | 3239 | N | PHE | B | 210 | -34.653 | -2.351 | 134.517 | 1.00 | 34.31 | N |
| ATOM | 3240 | CA | PHE | B | 210 | -33.559 | -1.507 | 134.978 | 1.00 | 37.85 | C |
| ATOM | 3241 | C | PHE | B | 210 | -34.108 | -0.121 | 135.302 | 1.00 | 39.37 | C |
| ATOM | 3242 | O | PHE | B | 210 | -35.098 | 0.319 | 134.705 | 1.00 | 33.58 | O |
| ATOM | 3243 | CB | PHE | B | 210 | -32.434 | -1.395 | 133.935 | 1.00 | 26.33 | C |
| ATOM | 3244 | CG | PHE | B | 210 | -32.827 | -0.647 | 132.698 | 1.00 | 33.39 | C |
| ATOM | 3245 | CD1 | PHE | B | 210 | -32.688 | 0.736 | 132.632 | 1.00 | 31.43 | C |
| ATOM | 3246 | CD2 | PHE | B | 210 | -33.342 | -1.321 | 131.597 | 1.00 | 32.75 | C |
| ATOM | 3247 | CE1 | PHE | B | 210 | -33.052 | 1.438 | 131.490 | 1.00 | 35.66 | C |
| ATOM | 3248 | CE2 | PHE | B | 210 | -33.702 | -0.630 | 130.436 | 1.00 | 33.47 | C |
| ATOM | 3249 | CZ | PHE | B | 210 | -33.559 | 0.752 | 130.380 | 1.00 | 34.55 | C |
| ATOM | 3250 | N | ASN | B | 211 | -33.475 | 0.547 | 136.284 | 1.00 | 42.75 | N |
| ATOM | 3251 | CA | ASN | B | 211 | -33.738 | 1.955 | 136.583 | 1.00 | 38.70 | C |
| ATOM | 3252 | C | ASN | B | 211 | -32.818 | 2.832 | 135.752 | 1.00 | 43.04 | C |
| ATOM | 3253 | O | ASN | B | 211 | -31.602 | 2.610 | 135.717 | 1.00 | 39.58 | O |
| ATOM | 3254 | CB | ASN | B | 211 | -33.515 | 2.295 | 138.054 | 1.00 | 48.54 | C |
| ATOM | 3255 | CG | ASN | B | 211 | -34.216 | 1.354 | 138.988 | 1.00 | 51.99 | C |
| ATOM | 3256 | OD1 | ASN | B | 211 | -35.237 | 0.765 | 138.642 | 1.00 | 48.81 | O |
| ATOM | 3257 | ND2 | ASN | B | 211 | -33.672 | 1.208 | 140.193 | 1.00 | 53.46 | N |
| ATOM | 3258 | N | ARG | B | 212 | -33.396 | 3.826 | 135.088 | 1.00 | 42.30 | N |
| ATOM | 3259 | CA | ARG | B | 212 | -32.585 | 4.725 | 134.279 | 1.00 | 44.00 | C |
| ATOM | 3260 | C | ARG | B | 212 | -31.547 | 5.416 | 135.166 | 1.00 | 53.42 | C |
| ATOM | 3261 | O | ARG | B | 212 | -31.838 | 5.807 | 136.304 | 1.00 | 49.38 | O |
| ATOM | 3262 | CB | ARG | B | 212 | -33.478 | 5.748 | 133.579 | 1.00 | 35.08 | C |
| ATOM | 3263 | CG | ARG | B | 212 | -32.768 | 6.567 | 132.543 | 1.00 | 35.57 | C |

| | | | | | | | | | | | |
|---|---|---|---|---|---|---|---|---|---|---|---|
| ATOM | 3264 | CD | ARG | B | 212 | -33.745 | 7.458 | 131.855 | 1.00 | 39.63 | C |
| ATOM | 3265 | NE | ARG | B | 212 | -34.383 | 8.298 | 132.850 | 1.00 | 48.56 | N |
| ATOM | 3266 | CZ | ARG | B | 212 | -33.927 | 9.489 | 133.210 | 1.00 | 47.89 | C |
| ATOM | 3267 | NH1 | ARG | B | 212 | -32.853 | 9.988 | 132.617 | 1.00 | 49.63 | N1+ |
| ATOM | 3268 | NH2 | ARG | B | 212 | -34.554 | 10.182 | 134.150 | 1.00 | 49.80 | N |
| ATOM | 3269 | N | GLY | B | 213 | -30.313 | 5.513 | 134.660 | 1.00 | 57.27 | N |
| ATOM | 3270 | CA | GLY | B | 213 | -29.233 | 6.174 | 135.372 | 1.00 | 56.62 | C |
| ATOM | 3271 | C | GLY | B | 213 | -28.594 | 5.389 | 136.502 | 1.00 | 64.15 | C |
| ATOM | 3272 | O | GLY | B | 213 | -27.852 | 5.979 | 137.297 | 1.00 | 72.50 | O |
| ATOM | 3273 | N | GLU | B | 214 | -28.872 | 4.093 | 136.624 | 1.00 | 62.54 | N |
| ATOM | 3274 | CA | GLU | B | 214 | -28.300 | 3.285 | 137.703 | 1.00 | 62.11 | C |
| ATOM | 3275 | C | GLU | B | 214 | -27.713 | 1.996 | 137.157 | 1.00 | 59.03 | C |
| ATOM | 3276 | O | GLU | B | 214 | -26.518 | 1.940 | 136.863 | 1.00 | 63.86 | O |
| ATOM | 3277 | CB | GLU | B | 214 | -29.353 | 3.001 | 138.781 | 1.00 | 51.33 | C |
| ATOM | 3278 | CG | GLU | B | 214 | -29.912 | 4.302 | 139.342 | 1.00 | 61.22 | C |
| ATOM | 3279 | CD | GLU | B | 214 | -31.228 | 4.170 | 140.105 | 1.00 | 66.11 | C |
| ATOM | 3280 | OE1 | GLU | B | 214 | -31.496 | 3.123 | 140.751 | 1.00 | 61.27 | O |
| ATOM | 3281 | OE2 | GLU | B | 214 | -32.004 | 5.152 | 140.044 | 1.00 | 64.67 | O1- |
| TER | | | | | | | | | | | |
| ATOM | 3282 | N | GLN | C | 1 | -37.672 | -36.358 | 87.095 | 1.00 | 48.43 | N |
| ATOM | 3283 | CA | GLN | C | 1 | -37.796 | -35.842 | 85.733 | 1.00 | 53.92 | C |
| ATOM | 3284 | C | GLN | C | 1 | -36.907 | -36.762 | 84.921 | 1.00 | 48.53 | C |
| ATOM | 3285 | O | GLN | C | 1 | -37.375 | -37.531 | 84.085 | 1.00 | 49.71 | O |
| ATOM | 3286 | CB | GLN | C | 1 | -37.335 | -34.383 | 85.628 | 1.00 | 47.24 | C |
| ATOM | 3287 | CG | GLN | C | 1 | -37.925 | -33.494 | 86.693 | 1.00 | 41.90 | C |
| ATOM | 3288 | CD | GLN | C | 1 | -37.270 | -33.771 | 88.072 | 1.00 | 54.37 | C |
| ATOM | 3289 | OE1 | GLN | C | 1 | -37.245 | -34.914 | 88.549 | 1.00 | 44.69 | O |
| ATOM | 3290 | NE2 | GLN | C | 1 | -36.710 | -32.731 | 88.690 | 1.00 | 59.79 | N |
| ATOM | 3291 | N | VAL | C | 2 | -35.614 | -36.686 | 85.222 | 1.00 | 51.69 | N |
| ATOM | 3292 | CA | VAL | C | 2 | -34.607 | -37.545 | 84.617 | 1.00 | 47.55 | C |
| ATOM | 3293 | C | VAL | C | 2 | -34.423 | -38.731 | 85.545 | 1.00 | 47.63 | C |
| ATOM | 3294 | O | VAL | C | 2 | -33.898 | -38.581 | 86.647 | 1.00 | 46.25 | O |
| ATOM | 3295 | CB | VAL | C | 2 | -33.299 | -36.782 | 84.412 | 1.00 | 42.21 | C |
| ATOM | 3296 | CG1 | VAL | C | 2 | -32.374 | -37.549 | 83.497 | 1.00 | 38.77 | C |
| ATOM | 3297 | CG2 | VAL | C | 2 | -33.625 | -35.409 | 83.861 | 1.00 | 38.63 | C |
| ATOM | 3298 | N | GLN | C | 3 | -34.870 | -39.908 | 85.122 | 1.00 | 44.76 | N |
| ATOM | 3299 | CA | GLN | C | 3 | -34.623 | -41.114 | 85.894 | 1.00 | 46.20 | C |
| ATOM | 3300 | C | GLN | C | 3 | -33.571 | -41.955 | 85.194 | 1.00 | 38.10 | C |
| ATOM | 3301 | O | GLN | C | 3 | -33.475 | -41.955 | 83.965 | 1.00 | 39.47 | O |
| ATOM | 3302 | CB | GLN | C | 3 | -35.902 | -41.931 | 86.152 | 1.00 | 46.17 | C |
| ATOM | 3303 | CG | GLN | C | 3 | -36.857 | -42.057 | 84.981 | 1.00 | 54.72 | C |
| ATOM | 3304 | CD | GLN | C | 3 | -37.939 | -40.974 | 84.954 | 1.00 | 59.64 | C |
| ATOM | 3305 | OE1 | GLN | C | 3 | -38.022 | -40.180 | 83.996 | 1.00 | 55.09 | O |
| ATOM | 3306 | NE2 | GLN | C | 3 | -38.794 | -40.952 | 85.996 | 1.00 | 49.26 | N |
| ATOM | 3307 | N | LEU | C | 4 | -32.728 | -42.584 | 85.993 | 1.00 | 36.10 | N |
| ATOM | 3308 | CA | LEU | C | 4 | -31.834 | -43.639 | 85.548 | 1.00 | 36.24 | C |
| ATOM | 3309 | C | LEU | C | 4 | -32.349 | -44.892 | 86.234 | 1.00 | 38.70 | C |
| ATOM | 3310 | O | LEU | C | 4 | -32.006 | -45.155 | 87.383 | 1.00 | 41.03 | O |
| ATOM | 3311 | CB | LEU | C | 4 | -30.377 | -43.351 | 85.916 | 1.00 | 31.18 | C |
| ATOM | 3312 | CG | LEU | C | 4 | -29.695 | -42.051 | 85.479 | 1.00 | 32.11 | C |
| ATOM | 3313 | CD1 | LEU | C | 4 | -28.199 | -42.201 | 85.552 | 1.00 | 34.48 | C |
| ATOM | 3314 | CD2 | LEU | C | 4 | -30.085 | -41.672 | 84.084 | 1.00 | 36.90 | C |
| ATOM | 3315 | N | GLN | C | 5 | -33.149 | -45.677 | 85.525 | 1.00 | 44.15 | N |
| ATOM | 3316 | CA | GLN | C | 5 | -33.773 | -46.856 | 86.108 | 1.00 | 45.12 | C |
| ATOM | 3317 | C | GLN | C | 5 | -32.880 | -48.063 | 85.887 | 1.00 | 41.78 | C |
| ATOM | 3318 | O | GLN | C | 5 | -32.475 | -48.340 | 84.755 | 1.00 | 41.54 | O |
| ATOM | 3319 | CB | GLN | C | 5 | -35.152 | -47.084 | 85.489 | 1.00 | 49.54 | C |
| ATOM | 3320 | CG | GLN | C | 5 | -35.943 | -48.256 | 86.071 | 1.00 | 56.47 | C |
| ATOM | 3321 | CD | GLN | C | 5 | -37.440 | -48.116 | 85.797 | 1.00 | 74.65 | C |
| ATOM | 3322 | OE1 | GLN | C | 5 | -37.935 | -47.009 | 85.533 | 1.00 | 74.08 | O |
| ATOM | 3323 | NE2 | GLN | C | 5 | -38.166 | -49.236 | 85.850 | 1.00 | 70.76 | N |
| ATOM | 3324 | N | GLN | C | 6 | -32.592 | -48.786 | 86.963 | 1.00 | 36.00 | N |
| ATOM | 3325 | CA | GLN | C | 6 | -31.668 | -49.905 | 86.917 | 1.00 | 37.25 | C |
| ATOM | 3326 | C | GLN | C | 6 | -32.432 | -51.207 | 87.049 | 1.00 | 40.30 | C |
| ATOM | 3327 | O | GLN | C | 6 | -33.416 | -51.281 | 87.787 | 1.00 | 48.42 | O |
| ATOM | 3328 | CB | GLN | C | 6 | -30.617 | -49.841 | 88.032 | 1.00 | 37.59 | C |
| ATOM | 3329 | CG | GLN | C | 6 | -29.875 | -48.541 | 88.142 | 1.00 | 40.04 | C |
| ATOM | 3330 | CD | GLN | C | 6 | -28.646 | -48.621 | 89.041 | 1.00 | 38.65 | C |
| ATOM | 3331 | OE1 | GLN | C | 6 | -28.173 | -47.599 | 89.535 | 1.00 | 36.68 | O |
| ATOM | 3332 | NE2 | GLN | C | 6 | -28.111 | -49.834 | 89.239 | 1.00 | 31.43 | N |
| ATOM | 3333 | N | TRP | C | 7 | -31.969 | -52.228 | 86.334 | 1.00 | 36.46 | N |

| | | | | | | | | | | | |
|---|---|---|---|---|---|---|---|---|---|---|---|
| ATOM | 3334 | CA | TRP | C | 7 | -32.424 | -53.585 | 86.573 | 1.00 | 39.72 | C |
| ATOM | 3335 | C | TRP | C | 7 | -31.284 | -54.527 | 86.258 | 1.00 | 37.13 | C |
| ATOM | 3336 | O | TRP | C | 7 | -30.252 | -54.125 | 85.709 | 1.00 | 36.52 | O |
| ATOM | 3337 | CB | TRP | C | 7 | -33.669 | -53.937 | 85.756 | 1.00 | 36.24 | C |
| ATOM | 3338 | CG | TRP | C | 7 | -33.515 | -53.880 | 84.294 | 1.00 | 38.80 | C |
| ATOM | 3339 | CD1 | TRP | C | 7 | -33.103 | -54.893 | 83.464 | 1.00 | 37.72 | C |
| ATOM | 3340 | CD2 | TRP | C | 7 | -33.812 | -52.760 | 83.446 | 1.00 | 41.40 | C |
| ATOM | 3341 | NE1 | TRP | C | 7 | -33.111 | -54.462 | 82.153 | 1.00 | 39.38 | N |
| ATOM | 3342 | CE2 | TRP | C | 7 | -33.544 | -53.159 | 82.113 | 1.00 | 39.28 | C |
| ATOM | 3343 | CE3 | TRP | C | 7 | -34.270 | -51.460 | 83.683 | 1.00 | 41.24 | C |
| ATOM | 3344 | CZ2 | TRP | C | 7 | -33.713 | -52.298 | 81.022 | 1.00 | 37.17 | C |
| ATOM | 3345 | CZ3 | TRP | C | 7 | -34.443 | -50.603 | 82.589 | 1.00 | 42.48 | C |
| ATOM | 3346 | CH2 | TRP | C | 7 | -34.164 | -51.032 | 81.278 | 1.00 | 38.07 | C |
| ATOM | 3347 | N | GLY | C | 8 | -31.479 | -55.779 | 86.640 | 1.00 | 30.53 | N |
| ATOM | 3348 | CA | GLY | C | 8 | -30.480 | -56.819 | 86.491 | 1.00 | 29.00 | C |
| ATOM | 3349 | C | GLY | C | 8 | -30.542 | -57.704 | 87.715 | 1.00 | 28.80 | C |
| ATOM | 3350 | O | GLY | C | 8 | -30.936 | -57.284 | 88.802 | 1.00 | 35.52 | O |
| ATOM | 3351 | N | ALA | C | 9 | -30.175 | -58.964 | 87.536 | 1.00 | 31.95 | N |
| ATOM | 3352 | CA | ALA | C | 9 | -30.163 | -59.902 | 88.650 | 1.00 | 36.64 | C |
| ATOM | 3353 | C | ALA | C | 9 | -29.108 | -59.490 | 89.672 | 1.00 | 40.72 | C |
| ATOM | 3354 | O | ALA | C | 9 | -27.936 | -59.312 | 89.329 | 1.00 | 44.07 | O |
| ATOM | 3355 | CB | ALA | C | 9 | -29.893 | -61.324 | 88.152 | 1.00 | 32.88 | C |
| ATOM | 3356 | N | GLY | C | 10 | -29.528 | -59.350 | 90.927 | 1.00 | 40.53 | N |
| ATOM | 3357 | CA | GLY | C | 10 | -28.651 | -58.938 | 92.001 | 1.00 | 34.92 | C |
| ATOM | 3358 | C | GLY | C | 10 | -28.135 | -60.045 | 92.894 | 1.00 | 32.59 | C |
| ATOM | 3359 | O | GLY | C | 10 | -27.259 | -59.787 | 93.722 | 1.00 | 37.26 | O |
| ATOM | 3360 | N | LEU | C | 11 | -28.643 | -61.269 | 92.760 | 1.00 | 33.32 | N |
| ATOM | 3361 | CA | LEU | C | 11 | -28.155 | -62.401 | 93.550 | 1.00 | 35.63 | C |
| ATOM | 3362 | C | LEU | C | 11 | -27.388 | -63.330 | 92.625 | 1.00 | 37.18 | C |
| ATOM | 3363 | O | LEU | C | 11 | -27.945 | -63.832 | 91.641 | 1.00 | 40.37 | O |
| ATOM | 3364 | CB | LEU | C | 11 | -29.292 | -63.162 | 94.238 | 1.00 | 33.21 | C |
| ATOM | 3365 | CG | LEU | C | 11 | -29.045 | -63.745 | 95.639 | 1.00 | 35.68 | C |
| ATOM | 3366 | CD1 | LEU | C | 11 | -29.828 | -65.026 | 95.836 | 1.00 | 37.14 | C |
| ATOM | 3367 | CD2 | LEU | C | 11 | -27.589 | -63.991 | 95.974 | 1.00 | 34.37 | C |
| ATOM | 3368 | N | LEU | C | 12 | -26.125 | -63.573 | 92.957 | 1.00 | 33.96 | N |
| ATOM | 3369 | CA | LEU | C | 12 | -25.238 | -64.365 | 92.126 | 1.00 | 37.97 | C |
| ATOM | 3370 | C | LEU | C | 12 | -24.363 | -65.240 | 93.002 | 1.00 | 35.65 | C |
| ATOM | 3371 | O | LEU | C | 12 | -24.068 | -64.894 | 94.147 | 1.00 | 36.30 | O |
| ATOM | 3372 | CB | LEU | C | 12 | -24.331 | -63.494 | 91.246 | 1.00 | 34.37 | C |
| ATOM | 3373 | CG | LEU | C | 12 | -24.960 | -62.530 | 90.262 | 1.00 | 37.66 | C |
| ATOM | 3374 | CD1 | LEU | C | 12 | -23.856 | -61.733 | 89.638 | 1.00 | 43.23 | C |
| ATOM | 3375 | CD2 | LEU | C | 12 | -25.713 | -63.294 | 89.190 | 1.00 | 44.30 | C |
| ATOM | 3376 | N | LYS | C | 13 | -23.932 | -66.390 | 92.428 | 1.00 | 36.66 | N |
| ATOM | 3377 | CA | LYS | C | 13 | -22.957 | -67.321 | 92.963 | 1.00 | 38.43 | C |
| ATOM | 3378 | C | LYS | C | 13 | -21.606 | -67.082 | 92.306 | 1.00 | 39.27 | C |
| ATOM | 3379 | O | LYS | C | 13 | -21.543 | -66.620 | 91.160 | 1.00 | 39.31 | O |
| ATOM | 3380 | CB | LYS | C | 13 | -23.391 | -68.766 | 92.715 | 1.00 | 36.21 | C |
| ATOM | 3381 | CG | LYS | C | 13 | -24.872 | -68.896 | 92.517 | 1.00 | 42.22 | C |
| ATOM | 3382 | CD | LYS | C | 13 | -25.398 | -70.074 | 93.270 | 1.00 | 45.83 | C |
| ATOM | 3383 | CE | LYS | C | 13 | -25.107 | -69.921 | 94.743 | 1.00 | 45.08 | C |
| ATOM | 3384 | NZ | LYS | C | 13 | -25.676 | -71.073 | 95.499 | 1.00 | 49.42 | N1+ |
| ATOM | 3385 | N | PRO | C | 14 | -20.517 | -67.383 | 93.016 | 1.00 | 33.06 | N |
| ATOM | 3386 | CA | PRO | C | 14 | -19.180 | -67.135 | 92.464 | 1.00 | 31.17 | C |
| ATOM | 3387 | C | PRO | C | 14 | -19.019 | -67.705 | 91.063 | 1.00 | 35.18 | C |
| ATOM | 3388 | O | PRO | C | 14 | -19.621 | -68.717 | 90.712 | 1.00 | 37.79 | O |
| ATOM | 3389 | CB | PRO | C | 14 | -18.258 | -67.830 | 93.463 | 1.00 | 24.90 | C |
| ATOM | 3390 | CG | PRO | C | 14 | -19.016 | -67.795 | 94.737 | 1.00 | 29.59 | C |
| ATOM | 3391 | CD | PRO | C | 14 | -20.461 | -67.876 | 94.405 | 1.00 | 27.79 | C |
| ATOM | 3392 | N | SER | C | 15 | -18.235 | -66.995 | 90.251 | 1.00 | 38.99 | N |
| ATOM | 3393 | CA | SER | C | 15 | -17.811 | -67.278 | 88.882 | 1.00 | 33.18 | C |
| ATOM | 3394 | C | SER | C | 15 | -18.911 | -66.976 | 87.855 | 1.00 | 35.06 | C |
| ATOM | 3395 | O | SER | C | 15 | -18.601 | -66.892 | 86.661 | 1.00 | 34.68 | O |
| ATOM | 3396 | CB | SER | C | 15 | -17.276 | -68.717 | 88.689 | 1.00 | 35.00 | C |
| ATOM | 3397 | OG | SER | C | 15 | -18.279 | -69.665 | 88.363 | 1.00 | 36.60 | O |
| ATOM | 3398 | N | GLU | C | 16 | -20.172 | -66.781 | 88.266 | 1.00 | 33.98 | N |
| ATOM | 3399 | CA | GLU | C | 16 | -21.187 | -66.305 | 87.322 | 1.00 | 35.39 | C |
| ATOM | 3400 | C | GLU | C | 16 | -20.811 | -64.890 | 86.852 | 1.00 | 35.84 | C |
| ATOM | 3401 | O | GLU | C | 16 | -19.841 | -64.279 | 87.324 | 1.00 | 35.76 | O |
| ATOM | 3402 | CB | GLU | C | 16 | -22.590 | -66.308 | 87.953 | 1.00 | 33.60 | C |
| ATOM | 3403 | CG | GLU | C | 16 | -22.982 | -67.595 | 88.701 | 1.00 | 38.40 | C |
| ATOM | 3404 | CD | GLU | C | 16 | -24.487 | -67.720 | 89.061 | 1.00 | 49.48 | C |

| | | | | | | | | | | | |
|---|---|---|---|---|---|---|---|---|---|---|---|
| ATOM | 3405 | OE1 | GLU | C | 16 | -25.199 | -66.698 | 89.225 | 1.00 | 50.75 | O |
| ATOM | 3406 | OE2 | GLU | C | 16 | -24.950 | -68.866 | 89.261 | 1.00 | 51.03 | O1- |
| ATOM | 3407 | N | THR | C | 17 | -21.570 | -64.359 | 85.905 | 1.00 | 32.01 | N |
| ATOM | 3408 | CA | THR | C | 17 | -21.301 | -63.010 | 85.451 | 1.00 | 32.01 | C |
| ATOM | 3409 | C | THR | C | 17 | -22.463 | -62.094 | 85.818 | 1.00 | 39.55 | C |
| ATOM | 3410 | O | THR | C | 17 | -23.637 | -62.460 | 85.685 | 1.00 | 36.43 | O |
| ATOM | 3411 | CB | THR | C | 17 | -20.978 | -62.950 | 83.939 | 1.00 | 34.07 | C |
| ATOM | 3412 | OG1 | THR | C | 17 | -21.956 | -62.180 | 83.230 | 1.00 | 39.64 | O |
| ATOM | 3413 | CG2 | THR | C | 17 | -20.841 | -64.327 | 83.325 | 1.00 | 35.94 | C |
| ATOM | 3414 | N | LEU | C | 18 | -22.109 | -60.901 | 86.295 | 1.00 | 37.34 | N |
| ATOM | 3415 | CA | LEU | C | 18 | -23.065 | -59.889 | 86.703 | 1.00 | 30.52 | C |
| ATOM | 3416 | C | LEU | C | 18 | -23.481 | -59.095 | 85.476 | 1.00 | 34.01 | C |
| ATOM | 3417 | O | LEU | C | 18 | -22.639 | -58.717 | 84.651 | 1.00 | 35.55 | O |
| ATOM | 3418 | CB | LEU | C | 18 | -22.437 | -59.006 | 87.782 | 1.00 | 29.47 | C |
| ATOM | 3419 | CG | LEU | C | 18 | -22.993 | -57.708 | 88.384 | 1.00 | 35.82 | C |
| ATOM | 3420 | CD1 | LEU | C | 18 | -22.732 | -56.524 | 87.491 | 1.00 | 32.88 | C |
| ATOM | 3421 | CD2 | LEU | C | 18 | -24.488 | -57.846 | 88.679 | 1.00 | 31.62 | C |
| ATOM | 3422 | N | SER | C | 19 | -24.783 | -58.870 | 85.340 | 1.00 | 31.00 | N |
| ATOM | 3423 | CA | SER | C | 19 | -25.301 | -58.146 | 84.187 | 1.00 | 36.91 | C |
| ATOM | 3424 | C | SER | C | 19 | -26.378 | -57.173 | 84.657 | 1.00 | 32.12 | C |
| ATOM | 3425 | O | SER | C | 19 | -27.371 | -57.593 | 85.262 | 1.00 | 30.22 | O |
| ATOM | 3426 | CB | SER | C | 19 | -25.835 | -59.143 | 83.141 | 1.00 | 33.82 | C |
| ATOM | 3427 | OG | SER | C | 19 | -26.265 | -58.506 | 81.963 | 1.00 | 42.48 | O |
| ATOM | 3428 | N | LEU | C | 20 | -26.175 | -55.877 | 84.364 | 1.00 | 30.42 | N |
| ATOM | 3429 | CA | LEU | C | 20 | -27.041 | -54.785 | 84.804 | 1.00 | 30.56 | C |
| ATOM | 3430 | C | LEU | C | 20 | -27.300 | -53.840 | 83.643 | 1.00 | 29.54 | C |
| ATOM | 3431 | O | LEU | C | 20 | -26.430 | -53.618 | 82.801 | 1.00 | 31.80 | O |
| ATOM | 3432 | CB | LEU | C | 20 | -26.416 | -53.973 | 85.970 | 1.00 | 25.88 | C |
| ATOM | 3433 | CG | LEU | C | 20 | -26.042 | -54.745 | 87.243 | 1.00 | 27.95 | C |
| ATOM | 3434 | CD1 | LEU | C | 20 | -25.312 | -53.866 | 88.232 | 1.00 | 25.17 | C |
| ATOM | 3435 | CD2 | LEU | C | 20 | -27.288 | -55.391 | 87.897 | 1.00 | 28.86 | C |
| ATOM | 3436 | N | THR | C | 21 | -28.479 | -53.236 | 83.636 | 1.00 | 29.19 | N |
| ATOM | 3437 | CA | THR | C | 21 | -28.844 | -52.262 | 82.616 | 1.00 | 32.34 | C |
| ATOM | 3438 | C | THR | C | 21 | -29.444 | -51.043 | 83.297 | 1.00 | 33.93 | C |
| ATOM | 3439 | O | THR | C | 21 | -30.059 | -51.155 | 84.360 | 1.00 | 41.09 | O |
| ATOM | 3440 | CB | THR | C | 21 | -29.833 | -52.839 | 81.576 | 1.00 | 32.67 | C |
| ATOM | 3441 | OG1 | THR | C | 21 | -29.282 | -54.034 | 81.016 | 1.00 | 29.98 | O |
| ATOM | 3442 | CG2 | THR | C | 21 | -30.116 | -51.832 | 80.457 | 1.00 | 28.76 | C |
| ATOM | 3443 | N | CYS | C | 22 | -29.221 | -49.880 | 82.686 | 1.00 | 28.26 | N |
| ATOM | 3444 | CA | CYS | C | 22 | -29.766 | -48.596 | 83.090 | 1.00 | 26.22 | C |
| ATOM | 3445 | C | CYS | C | 22 | -30.582 | -48.024 | 81.939 | 1.00 | 35.11 | C |
| ATOM | 3446 | O | CYS | C | 22 | -30.119 | -48.022 | 80.794 | 1.00 | 35.88 | O |
| ATOM | 3447 | CB | CYS | C | 22 | -28.601 | -47.671 | 83.441 | 1.00 | 34.45 | C |
| ATOM | 3448 | SG | CYS | C | 22 | -28.863 | -46.199 | 84.443 | 1.00 | 53.91 | S |
| ATOM | 3449 | N | ALA | C | 23 | -31.786 | -47.526 | 82.223 | 1.00 | 41.07 | N |
| ATOM | 3450 | CA | ALA | C | 23 | -32.593 | -46.849 | 81.211 | 1.00 | 32.31 | C |
| ATOM | 3451 | C | ALA | C | 23 | -32.715 | -45.377 | 81.565 | 1.00 | 38.18 | C |
| ATOM | 3452 | O | ALA | C | 23 | -33.077 | -45.034 | 82.693 | 1.00 | 40.40 | O |
| ATOM | 3453 | CB | ALA | C | 23 | -33.971 | -47.489 | 81.072 | 1.00 | 31.29 | C |
| ATOM | 3454 | N | VAL | C | 24 | -32.419 | -44.515 | 80.601 | 1.00 | 37.14 | N |
| ATOM | 3455 | CA | VAL | C | 24 | -32.433 | -43.067 | 80.777 | 1.00 | 34.17 | C |
| ATOM | 3456 | C | VAL | C | 24 | -33.726 | -42.531 | 80.183 | 1.00 | 39.79 | C |
| ATOM | 3457 | O | VAL | C | 24 | -34.063 | -42.841 | 79.033 | 1.00 | 44.85 | O |
| ATOM | 3458 | CB | VAL | C | 24 | -31.202 | -42.429 | 80.111 | 1.00 | 35.68 | C |
| ATOM | 3459 | CG1 | VAL | C | 24 | -31.194 | -40.927 | 80.274 | 1.00 | 33.16 | C |
| ATOM | 3460 | CG2 | VAL | C | 24 | -29.937 | -43.034 | 80.689 | 1.00 | 35.47 | C |
| ATOM | 3461 | N | SER | C | 25 | -34.433 | -41.694 | 80.936 | 1.00 | 40.19 | N |
| ATOM | 3462 | CA | SER | C | 25 | -35.722 | -41.217 | 80.453 | 1.00 | 37.50 | C |
| ATOM | 3463 | C | SER | C | 25 | -35.843 | -39.707 | 80.355 | 1.00 | 46.83 | C |
| ATOM | 3464 | O | SER | C | 25 | -36.323 | -39.206 | 79.339 | 1.00 | 56.63 | O |
| ATOM | 3465 | CB | SER | C | 25 | -36.842 | -41.735 | 81.372 | 1.00 | 39.57 | C |
| ATOM | 3466 | OG | SER | C | 25 | -36.448 | -42.914 | 82.077 | 1.00 | 44.46 | O |
| ATOM | 3467 | N | GLY | C | 26 | -35.362 | -38.958 | 81.342 | 1.00 | 46.38 | N |
| ATOM | 3468 | CA | GLY | C | 26 | -35.703 | -37.542 | 81.413 | 1.00 | 45.90 | C |
| ATOM | 3469 | C | GLY | C | 26 | -35.175 | -36.666 | 80.290 | 1.00 | 45.91 | C |
| ATOM | 3470 | O | GLY | C | 26 | -35.926 | -35.873 | 79.720 | 1.00 | 50.30 | O |
| ATOM | 3471 | N | GLY | C | 27 | -33.881 | -36.752 | 79.983 | 1.00 | 41.90 | N |
| ATOM | 3472 | CA | GLY | C | 27 | -33.299 | -35.764 | 79.082 | 1.00 | 33.59 | C |
| ATOM | 3473 | C | GLY | C | 27 | -32.626 | -36.313 | 77.847 | 1.00 | 35.39 | C |
| ATOM | 3474 | O | GLY | C | 27 | -33.065 | -37.324 | 77.298 | 1.00 | 41.91 | O |
| ATOM | 3475 | N | SER | C | 28 | -31.574 | -35.646 | 77.382 | 1.00 | 41.18 | N |

| | | | | | | | | | | | |
|---|---|---|---|---|---|---|---|---|---|---|---|
| ATOM | 3476 | CA | SER | C | 28 | -30.856 | -36.113 | 76.204 | 1.00 | 38.29 | C |
| ATOM | 3477 | C | SER | C | 28 | -29.975 | -37.312 | 76.538 | 1.00 | 36.07 | C |
| ATOM | 3478 | O | SER | C | 28 | -29.631 | -37.575 | 77.693 | 1.00 | 37.41 | O |
| ATOM | 3479 | CB | SER | C | 28 | -29.983 | -35.007 | 75.609 | 1.00 | 38.36 | C |
| ATOM | 3480 | OG | SER | C | 28 | -30.761 | -33.920 | 75.126 | 1.00 | 40.67 | O |
| ATOM | 3481 | N | PHE | C | 29 | -29.605 | -38.042 | 75.494 | 1.00 | 36.30 | N |
| ATOM | 3482 | CA | PHE | C | 29 | -28.717 | -39.178 | 75.630 | 1.00 | 36.41 | C |
| ATOM | 3483 | C | PHE | C | 29 | -27.350 | -38.932 | 75.015 | 1.00 | 41.79 | C |
| ATOM | 3484 | O | PHE | C | 29 | -26.408 | -39.669 | 75.329 | 1.00 | 42.57 | O |
| ATOM | 3485 | CB | PHE | C | 29 | -29.357 | -40.414 | 74.999 | 1.00 | 29.89 | C |
| ATOM | 3486 | CG | PHE | C | 29 | -28.843 | -41.712 | 75.541 | 1.00 | 31.96 | C |
| ATOM | 3487 | CD1 | PHE | C | 29 | -28.786 | -41.939 | 76.912 | 1.00 | 37.24 | C |
| ATOM | 3488 | CD2 | PHE | C | 29 | -28.466 | -42.735 | 74.682 | 1.00 | 32.71 | C |
| ATOM | 3489 | CE1 | PHE | C | 29 | -28.325 | -43.161 | 77.428 | 1.00 | 31.78 | C |
| ATOM | 3490 | CE2 | PHE | C | 29 | -28.010 | -43.953 | 75.182 | 1.00 | 32.65 | C |
| ATOM | 3491 | CZ | PHE | C | 29 | -27.946 | -44.167 | 76.558 | 1.00 | 31.78 | C |
| ATOM | 3492 | N | ARG | C | 30 | -27.207 | -37.893 | 74.190 | 1.00 | 36.62 | N |
| ATOM | 3493 | CA | ARG | C | 30 | -26.097 | -37.790 | 73.261 | 1.00 | 40.24 | C |
| ATOM | 3494 | C | ARG | C | 30 | -24.969 | -36.896 | 73.757 | 1.00 | 41.65 | C |
| ATOM | 3495 | O | ARG | C | 30 | -23.861 | -36.972 | 73.223 | 1.00 | 37.36 | O |
| ATOM | 3496 | CB | ARG | C | 30 | -26.601 | -37.249 | 71.922 | 1.00 | 37.58 | C |
| ATOM | 3497 | CG | ARG | C | 30 | -27.046 | -35.805 | 72.068 | 1.00 | 51.10 | C |
| ATOM | 3498 | CD | ARG | C | 30 | -27.335 | -35.130 | 70.748 | 1.00 | 52.17 | C |
| ATOM | 3499 | NE | ARG | C | 30 | -28.733 | -35.284 | 70.381 | 1.00 | 58.44 | N |
| ATOM | 3500 | CZ | ARG | C | 30 | -29.297 | -34.695 | 69.334 | 1.00 | 65.83 | C |
| ATOM | 3501 | NH1 | ARG | C | 30 | -30.583 | -34.897 | 69.073 | 1.00 | 66.12 | N1+ |
| ATOM | 3502 | NH2 | ARG | C | 30 | -28.575 | -33.907 | 68.548 | 1.00 | 74.46 | N |
| ATOM | 3503 | N | TYR | C | 31 | -25.210 | -36.062 | 74.758 | 1.00 | 44.38 | N |
| ATOM | 3504 | CA | TYR | C | 31 | -24.179 | -35.148 | 75.227 | 1.00 | 38.68 | C |
| ATOM | 3505 | C | TYR | C | 31 | -23.376 | -35.702 | 76.382 | 1.00 | 38.31 | C |
| ATOM | 3506 | O | TYR | C | 31 | -22.476 | -35.022 | 76.876 | 1.00 | 37.88 | O |
| ATOM | 3507 | CB | TYR | C | 31 | -24.791 | -33.816 | 75.660 | 1.00 | 39.97 | C |
| ATOM | 3508 | CG | TYR | C | 31 | -25.693 | -33.157 | 74.646 | 1.00 | 38.66 | C |
| ATOM | 3509 | CD1 | TYR | C | 31 | -25.199 | -32.718 | 73.428 | 1.00 | 39.15 | C |
| ATOM | 3510 | CD2 | TYR | C | 31 | -27.032 | -32.967 | 74.913 | 1.00 | 40.52 | C |
| ATOM | 3511 | CE1 | TYR | C | 31 | -26.008 | -32.113 | 72.518 | 1.00 | 41.25 | C |
| ATOM | 3512 | CE2 | TYR | C | 31 | -27.852 | -32.352 | 74.011 | 1.00 | 45.11 | C |
| ATOM | 3513 | CZ | TYR | C | 31 | -27.342 | -31.928 | 72.814 | 1.00 | 48.11 | C |
| ATOM | 3514 | OH | TYR | C | 31 | -28.178 | -31.317 | 71.914 | 1.00 | 50.76 | O |
| ATOM | 3515 | N | TYR | C | 32 | -23.699 | -36.894 | 76.851 | 1.00 | 41.37 | N |
| ATOM | 3516 | CA | TYR | C | 32 | -23.187 | -37.357 | 78.121 | 1.00 | 33.57 | C |
| ATOM | 3517 | C | TYR | C | 32 | -22.357 | -38.615 | 77.953 | 1.00 | 35.33 | C |
| ATOM | 3518 | O | TYR | C | 32 | -22.466 | -39.346 | 76.967 | 1.00 | 39.05 | O |
| ATOM | 3519 | CB | TYR | C | 32 | -24.325 | -37.630 | 79.090 | 1.00 | 34.60 | C |
| ATOM | 3520 | CG | TYR | C | 32 | -25.238 | -36.454 | 79.258 | 1.00 | 38.37 | C |
| ATOM | 3521 | CD1 | TYR | C | 32 | -24.873 | -35.386 | 80.057 | 1.00 | 38.78 | C |
| ATOM | 3522 | CD2 | TYR | C | 32 | -26.472 | -36.408 | 78.610 | 1.00 | 37.80 | C |
| ATOM | 3523 | CE1 | TYR | C | 32 | -25.711 | -34.288 | 80.207 | 1.00 | 42.05 | C |
| ATOM | 3524 | CE2 | TYR | C | 32 | -27.310 | -35.329 | 78.749 | 1.00 | 41.43 | C |
| ATOM | 3525 | CZ | TYR | C | 32 | -26.927 | -34.266 | 79.551 | 1.00 | 45.45 | C |
| ATOM | 3526 | OH | TYR | C | 32 | -27.758 | -33.180 | 79.704 | 1.00 | 53.26 | O |
| ATOM | 3527 | N | TYR | C | 33 | -21.511 | -38.840 | 78.942 | 1.00 | 36.12 | N |
| ATOM | 3528 | CA | TYR | C | 33 | -20.902 | -40.133 | 79.185 | 1.00 | 33.30 | C |
| ATOM | 3529 | C | TYR | C | 33 | -21.731 | -40.841 | 80.236 | 1.00 | 29.90 | C |
| ATOM | 3530 | O | TYR | C | 33 | -22.282 | -40.201 | 81.135 | 1.00 | 34.09 | O |
| ATOM | 3531 | CB | TYR | C | 33 | -19.453 | -39.988 | 79.655 | 1.00 | 32.91 | C |
| ATOM | 3532 | CG | TYR | C | 33 | -18.490 | -39.876 | 78.500 | 1.00 | 32.64 | C |
| ATOM | 3533 | CD1 | TYR | C | 33 | -18.235 | -38.637 | 77.891 | 1.00 | 29.43 | C |
| ATOM | 3534 | CD2 | TYR | C | 33 | -17.843 | -41.004 | 78.005 | 1.00 | 32.78 | C |
| ATOM | 3535 | CE1 | TYR | C | 33 | -17.352 | -38.526 | 76.834 | 1.00 | 31.81 | C |
| ATOM | 3536 | CE2 | TYR | C | 33 | -16.947 | -40.905 | 76.954 | 1.00 | 37.66 | C |
| ATOM | 3537 | CZ | TYR | C | 33 | -16.714 | -39.671 | 76.363 | 1.00 | 36.45 | C |
| ATOM | 3538 | OH | TYR | C | 33 | -15.840 | -39.599 | 75.307 | 1.00 | 33.16 | O |
| ATOM | 3539 | N | TRP | C | 34 | -21.845 | -42.154 | 80.099 | 1.00 | 31.46 | N |
| ATOM | 3540 | CA | TRP | C | 34 | -22.687 | -42.974 | 80.957 | 1.00 | 28.61 | C |
| ATOM | 3541 | C | TRP | C | 34 | -21.790 | -43.981 | 81.658 | 1.00 | 31.34 | C |
| ATOM | 3542 | O | TRP | C | 34 | -20.997 | -44.666 | 80.994 | 1.00 | 26.44 | O |
| ATOM | 3543 | CB | TRP | C | 34 | -23.790 | -43.628 | 80.118 | 1.00 | 25.06 | C |
| ATOM | 3544 | CG | TRP | C | 34 | -24.610 | -42.557 | 79.485 | 1.00 | 31.01 | C |
| ATOM | 3545 | CD1 | TRP | C | 34 | -24.506 | -42.091 | 78.205 | 1.00 | 32.39 | C |
| ATOM | 3546 | CD2 | TRP | C | 34 | -25.632 | -41.770 | 80.113 | 1.00 | 33.17 | C |

| | | | | | | | | | | | |
|---|---|---|---|---|---|---|---|---|---|---|---|
| ATOM | 3547 | NE1 | TRP | C | 34 | -25.403 | -41.072 | 77.993 | 1.00 | 33.65 | N |
| ATOM | 3548 | CE2 | TRP | C | 34 | -26.108 | -40.853 | 79.145 | 1.00 | 34.22 | C |
| ATOM | 3549 | CE3 | TRP | C | 34 | -26.205 | -41.761 | 81.389 | 1.00 | 28.29 | C |
| ATOM | 3550 | CZ2 | TRP | C | 34 | -27.131 | -39.940 | 79.416 | 1.00 | 33.64 | C |
| ATOM | 3551 | CZ3 | TRP | C | 34 | -27.211 | -40.834 | 81.659 | 1.00 | 30.29 | C |
| ATOM | 3552 | CH2 | TRP | C | 34 | -27.660 | -39.944 | 80.682 | 1.00 | 29.13 | C |
| ATOM | 3553 | N | SER | C | 35 | -21.903 | -44.049 | 82.998 | 1.00 | 28.84 | N |
| ATOM | 3554 | CA | SER | C | 35 | -20.898 | -44.663 | 83.856 | 1.00 | 26.34 | C |
| ATOM | 3555 | C | SER | C | 35 | -21.495 | -45.683 | 84.815 | 1.00 | 25.62 | C |
| ATOM | 3556 | O | SER | C | 35 | -22.691 | -45.688 | 85.096 | 1.00 | 28.59 | O |
| ATOM | 3557 | CB | SER | C | 35 | -20.153 | -43.603 | 84.674 | 1.00 | 25.36 | C |
| ATOM | 3558 | OG | SER | C | 35 | -19.476 | -42.699 | 83.828 | 1.00 | 36.45 | O |
| ATOM | 3559 | N | TRP | C | 36 | -20.611 | -46.515 | 85.359 | 1.00 | 24.98 | N |
| ATOM | 3560 | CA | TRP | C | 36 | -20.914 | -47.468 | 86.419 | 1.00 | 22.69 | C |
| ATOM | 3561 | C | TRP | C | 36 | -19.937 | -47.262 | 87.567 | 1.00 | 26.01 | C |
| ATOM | 3562 | O | TRP | C | 36 | -18.723 | -47.189 | 87.354 | 1.00 | 30.88 | O |
| ATOM | 3563 | CB | TRP | C | 36 | -20.829 | -48.911 | 85.911 | 1.00 | 27.21 | C |
| ATOM | 3564 | CG | TRP | C | 36 | -21.922 | -49.263 | 84.951 | 1.00 | 31.29 | C |
| ATOM | 3565 | CD1 | TRP | C | 36 | -21.856 | -49.288 | 83.581 | 1.00 | 27.11 | C |
| ATOM | 3566 | CD2 | TRP | C | 36 | -23.263 | -49.599 | 85.293 | 1.00 | 28.25 | C |
| ATOM | 3567 | NE1 | TRP | C | 36 | -23.082 | -49.636 | 83.050 | 1.00 | 25.91 | N |
| ATOM | 3568 | CE2 | TRP | C | 36 | -23.959 | -49.843 | 84.083 | 1.00 | 31.98 | C |
| ATOM | 3569 | CE3 | TRP | C | 36 | -23.942 | -49.732 | 86.504 | 1.00 | 23.12 | C |
| ATOM | 3570 | CZ2 | TRP | C | 36 | -25.310 | -50.203 | 84.059 | 1.00 | 30.86 | C |
| ATOM | 3571 | CZ3 | TRP | C | 36 | -25.280 | -50.087 | 86.479 | 1.00 | 26.83 | C |
| ATOM | 3572 | CH2 | TRP | C | 36 | -25.954 | -50.315 | 85.266 | 1.00 | 26.13 | C |
| ATOM | 3573 | N | ILE | C | 37 | -20.469 | -47.181 | 88.779 | 1.00 | 27.24 | N |
| ATOM | 3574 | CA | ILE | C | 37 | -19.713 | -46.954 | 90.007 | 1.00 | 25.13 | C |
| ATOM | 3575 | C | ILE | C | 37 | -20.228 | -47.955 | 91.036 | 1.00 | 26.55 | C |
| ATOM | 3576 | O | ILE | C | 37 | -21.442 | -48.138 | 91.155 | 1.00 | 29.45 | O |
| ATOM | 3577 | CB | ILE | C | 37 | -19.904 | -45.502 | 90.503 | 1.00 | 24.76 | C |
| ATOM | 3578 | CG1 | ILE | C | 37 | -19.417 | -44.509 | 89.444 | 1.00 | 26.03 | C |
| ATOM | 3579 | CG2 | ILE | C | 37 | -19.245 | -45.259 | 91.853 | 1.00 | 28.36 | C |
| ATOM | 3580 | CD1 | ILE | C | 37 | -20.031 | -43.142 | 89.552 | 1.00 | 27.83 | C |
| ATOM | 3581 | N | ARG | C | 38 | -19.328 | -48.630 | 91.755 | 1.00 | 21.22 | N |
| ATOM | 3582 | CA | ARG | C | 38 | -19.764 | -49.538 | 92.810 | 1.00 | 25.95 | C |
| ATOM | 3583 | C | ARG | C | 38 | -19.274 | -49.069 | 94.183 | 1.00 | 31.35 | C |
| ATOM | 3584 | O | ARG | C | 38 | -18.283 | -48.340 | 94.308 | 1.00 | 26.88 | O |
| ATOM | 3585 | CB | ARG | C | 38 | -19.314 | -50.993 | 92.559 | 1.00 | 23.96 | C |
| ATOM | 3586 | CG | ARG | C | 38 | -17.834 | -51.151 | 92.450 | 1.00 | 33.62 | C |
| ATOM | 3587 | CD | ARG | C | 38 | -17.312 | -52.176 | 93.382 | 1.00 | 31.96 | C |
| ATOM | 3588 | NE | ARG | C | 38 | -17.339 | -53.503 | 92.796 | 1.00 | 36.58 | N |
| ATOM | 3589 | CZ | ARG | C | 38 | -16.278 | -54.294 | 92.657 | 1.00 | 33.65 | C |
| ATOM | 3590 | NH1 | ARG | C | 38 | -15.072 | -53.910 | 93.057 | 1.00 | 31.68 | N1+ |
| ATOM | 3591 | NH2 | ARG | C | 38 | -16.436 | -55.483 | 92.123 | 1.00 | 28.23 | N |
| ATOM | 3592 | N | GLN | C | 39 | -20.015 | -49.467 | 95.215 | 1.00 | 27.22 | N |
| ATOM | 3593 | CA | GLN | C | 39 | -19.688 | -49.135 | 96.599 | 1.00 | 29.87 | C |
| ATOM | 3594 | C | GLN | C | 39 | -19.795 | -50.406 | 97.437 | 1.00 | 28.44 | C |
| ATOM | 3595 | O | GLN | C | 39 | -20.911 | -50.833 | 97.779 | 1.00 | 29.08 | O |
| ATOM | 3596 | CB | GLN | C | 39 | -20.621 | -48.042 | 97.105 | 1.00 | 24.56 | C |
| ATOM | 3597 | CG | GLN | C | 39 | -20.252 | -47.429 | 98.435 | 1.00 | 28.85 | C |
| ATOM | 3598 | CD | GLN | C | 39 | -21.101 | -46.198 | 98.718 | 1.00 | 36.48 | C |
| ATOM | 3599 | OE1 | GLN | C | 39 | -22.318 | -46.187 | 98.497 | 1.00 | 42.72 | O |
| ATOM | 3600 | NE2 | GLN | C | 39 | -20.459 | -45.144 | 99.169 | 1.00 | 38.36 | N |
| ATOM | 3601 | N | PRO | C | 40 | -18.674 | -51.040 | 97.791 | 1.00 | 30.67 | N |
| ATOM | 3602 | CA | PRO | C | 40 | -18.757 | -52.243 | 98.625 | 1.00 | 27.55 | C |
| ATOM | 3603 | C | PRO | C | 40 | -19.218 | -51.877 | 100.021 | 1.00 | 33.77 | C |
| ATOM | 3604 | O | PRO | C | 40 | -18.964 | -50.757 | 100.496 | 1.00 | 34.43 | O |
| ATOM | 3605 | CB | PRO | C | 40 | -17.315 | -52.776 | 98.621 | 1.00 | 27.60 | C |
| ATOM | 3606 | CG | PRO | C | 40 | -16.645 | -52.058 | 97.420 | 1.00 | 26.34 | C |
| ATOM | 3607 | CD | PRO | C | 40 | -17.294 | -50.736 | 97.371 | 1.00 | 28.79 | C |
| ATOM | 3608 | N | PRO | C | 41 | -19.933 | -52.772 | 100.711 | 1.00 | 38.11 | N |
| ATOM | 3609 | CA | PRO | C | 41 | -20.568 | -52.374 | 101.976 | 1.00 | 33.02 | C |
| ATOM | 3610 | C | PRO | C | 41 | -19.528 | -51.959 | 103.001 | 1.00 | 40.48 | C |
| ATOM | 3611 | O | PRO | C | 41 | -18.509 | -52.636 | 103.187 | 1.00 | 40.42 | O |
| ATOM | 3612 | CB | PRO | C | 41 | -21.327 | -53.631 | 102.408 | 1.00 | 37.98 | C |
| ATOM | 3613 | CG | PRO | C | 41 | -20.611 | -54.752 | 101.751 | 1.00 | 37.85 | C |
| ATOM | 3614 | CD | PRO | C | 41 | -20.123 | -54.207 | 100.431 | 1.00 | 37.87 | C |
| ATOM | 3615 | N | GLY | C | 42 | -19.761 | -50.786 | 103.604 | 1.00 | 40.56 | N |
| ATOM | 3616 | CA | GLY | C | 42 | -18.805 | -50.159 | 104.482 | 1.00 | 38.30 | C |
| ATOM | 3617 | C | GLY | C | 42 | -17.794 | -49.262 | 103.801 | 1.00 | 45.76 | C |

| | | | | | | | | | | | |
|---|---|---|---|---|---|---|---|---|---|---|---|
| ATOM | 3618 | O | GLY | C | 42 | -17.212 | -48.400 | 104.462 | 1.00 | 47.20 | O |
| ATOM | 3619 | N | LYS | C | 43 | -17.557 | -49.445 | 102.505 | 1.00 | 44.96 | N |
| ATOM | 3620 | CA | LYS | C | 43 | -16.391 | -48.889 | 101.836 | 1.00 | 42.23 | C |
| ATOM | 3621 | C | LYS | C | 43 | -16.754 | -47.617 | 101.060 | 1.00 | 40.08 | C |
| ATOM | 3622 | O | LYS | C | 43 | -17.835 | -47.040 | 101.222 | 1.00 | 39.81 | O |
| ATOM | 3623 | CB | LYS | C | 43 | -15.764 | -49.967 | 100.939 | 1.00 | 39.38 | C |
| ATOM | 3624 | CG | LYS | C | 43 | -15.348 | -51.222 | 101.710 | 1.00 | 43.20 | C |
| ATOM | 3625 | CD | LYS | C | 43 | -14.763 | -50.832 | 103.075 | 1.00 | 44.43 | C |
| ATOM | 3626 | CE | LYS | C | 43 | -14.563 | -52.031 | 103.993 | 1.00 | 51.32 | C |
| ATOM | 3627 | NZ | LYS | C | 43 | -13.263 | -52.735 | 103.784 | 1.00 | 55.64 | N1+ |
| ATOM | 3628 | N | GLY | C | 44 | -15.827 | -47.168 | 100.217 | 1.00 | 38.92 | N |
| ATOM | 3629 | CA | GLY | C | 44 | -16.003 | -45.991 | 99.404 | 1.00 | 38.32 | C |
| ATOM | 3630 | C | GLY | C | 44 | -16.454 | -46.330 | 98.000 | 1.00 | 39.62 | C |
| ATOM | 3631 | O | GLY | C | 44 | -17.012 | -47.402 | 97.737 | 1.00 | 42.04 | O |
| ATOM | 3632 | N | LEU | C | 45 | -16.159 | -45.425 | 97.075 | 1.00 | 32.83 | N |
| ATOM | 3633 | CA | LEU | C | 45 | -16.642 | -45.491 | 95.709 | 1.00 | 27.76 | C |
| ATOM | 3634 | C | LEU | C | 45 | -15.528 | -45.909 | 94.759 | 1.00 | 30.80 | C |
| ATOM | 3635 | O | LEU | C | 45 | -14.385 | -45.473 | 94.905 | 1.00 | 37.23 | O |
| ATOM | 3636 | CB | LEU | C | 45 | -17.213 | -44.134 | 95.309 | 1.00 | 28.31 | C |
| ATOM | 3637 | CG | LEU | C | 45 | -18.413 | -43.760 | 96.186 | 1.00 | 29.60 | C |
| ATOM | 3638 | CD1 | LEU | C | 45 | -18.761 | -42.284 | 96.033 | 1.00 | 25.47 | C |
| ATOM | 3639 | CD2 | LEU | C | 45 | -19.619 | -44.621 | 95.903 | 1.00 | 26.22 | C |
| ATOM | 3640 | N | GLU | C | 46 | -15.834 | -46.819 | 93.839 | 1.00 | 25.91 | N |
| ATOM | 3641 | CA | GLU | C | 46 | -14.895 | -47.185 | 92.791 | 1.00 | 30.55 | C |
| ATOM | 3642 | C | GLU | C | 46 | -15.566 | -47.029 | 91.433 | 1.00 | 35.38 | C |
| ATOM | 3643 | O | GLU | C | 46 | -16.677 | -47.523 | 91.215 | 1.00 | 35.98 | O |
| ATOM | 3644 | CB | GLU | C | 46 | -14.340 | -48.609 | 92.936 | 1.00 | 34.86 | C |
| ATOM | 3645 | CG | GLU | C | 46 | -14.868 | -49.426 | 94.118 | 1.00 | 42.88 | C |
| ATOM | 3646 | CD | GLU | C | 46 | -14.142 | -50.792 | 94.274 | 1.00 | 55.29 | C |
| ATOM | 3647 | OE1 | GLU | C | 46 | -13.692 | -51.365 | 93.231 | 1.00 | 52.24 | O |
| ATOM | 3648 | OE2 | GLU | C | 46 | -14.010 | -51.261 | 95.444 | 1.00 | 42.79 | O1- |
| ATOM | 3649 | N | TRP | C | 47 | -14.880 | -46.345 | 90.524 | 1.00 | 35.22 | N |
| ATOM | 3650 | CA | TRP | C | 47 | -15.389 | -46.093 | 89.189 | 1.00 | 31.80 | C |
| ATOM | 3651 | C | TRP | C | 47 | -15.070 | -47.293 | 88.313 | 1.00 | 32.40 | C |
| ATOM | 3652 | O | TRP | C | 47 | -13.919 | -47.720 | 88.238 | 1.00 | 34.10 | O |
| ATOM | 3653 | CB | TRP | C | 47 | -14.750 | -44.817 | 88.647 | 1.00 | 27.85 | C |
| ATOM | 3654 | CG | TRP | C | 47 | -15.028 | -44.369 | 87.213 | 1.00 | 29.38 | C |
| ATOM | 3655 | CD1 | TRP | C | 47 | -16.066 | -43.591 | 86.769 | 1.00 | 28.35 | C |
| ATOM | 3656 | CD2 | TRP | C | 47 | -14.194 | -44.605 | 86.072 | 1.00 | 31.89 | C |
| ATOM | 3657 | NE1 | TRP | C | 47 | -15.932 | -43.340 | 85.418 | 1.00 | 29.56 | N |
| ATOM | 3658 | CE2 | TRP | C | 47 | -14.792 | -43.954 | 84.970 | 1.00 | 29.80 | C |
| ATOM | 3659 | CE3 | TRP | C | 47 | -12.996 | -45.312 | 85.873 | 1.00 | 29.70 | C |
| ATOM | 3660 | CZ2 | TRP | C | 47 | -14.234 | -43.990 | 83.699 | 1.00 | 30.22 | C |
| ATOM | 3661 | CZ3 | TRP | C | 47 | -12.455 | -45.356 | 84.615 | 1.00 | 26.84 | C |
| ATOM | 3662 | CH2 | TRP | C | 47 | -13.068 | -44.696 | 83.541 | 1.00 | 31.06 | C |
| ATOM | 3663 | N | PHE | C | 48 | -16.096 | -47.849 | 87.666 | 1.00 | 29.92 | N |
| ATOM | 3664 | CA | PHE | C | 48 | -15.868 | -48.990 | 86.792 | 1.00 | 29.20 | C |
| ATOM | 3665 | C | PHE | C | 48 | -15.444 | -48.547 | 85.397 | 1.00 | 29.36 | C |
| ATOM | 3666 | O | PHE | C | 48 | -14.536 | -49.142 | 84.808 | 1.00 | 30.45 | O |
| ATOM | 3667 | CB | PHE | C | 48 | -17.123 | -49.875 | 86.744 | 1.00 | 27.65 | C |
| ATOM | 3668 | CG | PHE | C | 48 | -17.029 | -51.079 | 87.638 | 1.00 | 30.68 | C |
| ATOM | 3669 | CD1 | PHE | C | 48 | -16.381 | -50.992 | 88.876 | 1.00 | 34.82 | C |
| ATOM | 3670 | CD2 | PHE | C | 48 | -17.539 | -52.300 | 87.246 | 1.00 | 31.72 | C |
| ATOM | 3671 | CE1 | PHE | C | 48 | -16.247 | -52.101 | 89.705 | 1.00 | 32.25 | C |
| ATOM | 3672 | CE2 | PHE | C | 48 | -17.423 | -53.420 | 88.075 | 1.00 | 30.94 | C |
| ATOM | 3673 | CZ | PHE | C | 48 | -16.775 | -53.319 | 89.298 | 1.00 | 33.98 | C |
| ATOM | 3674 | N | GLY | C | 49 | -16.078 | -47.519 | 84.858 | 1.00 | 28.11 | N |
| ATOM | 3675 | CA | GLY | C | 49 | -15.826 | -47.128 | 83.488 | 1.00 | 29.63 | C |
| ATOM | 3676 | C | GLY | C | 49 | -16.967 | -46.291 | 82.950 | 1.00 | 32.96 | C |
| ATOM | 3677 | O | GLY | C | 49 | -17.956 | -46.032 | 83.640 | 1.00 | 32.51 | O |
| ATOM | 3678 | N | GLU | C | 50 | -16.831 | -45.916 | 81.674 | 1.00 | 31.40 | N |
| ATOM | 3679 | CA | GLU | C | 50 | -17.768 | -45.012 | 81.011 | 1.00 | 31.61 | C |
| ATOM | 3680 | C | GLU | C | 50 | -17.854 | -45.334 | 79.519 | 1.00 | 30.45 | C |
| ATOM | 3681 | O | GLU | C | 50 | -16.891 | -45.819 | 78.920 | 1.00 | 31.68 | O |
| ATOM | 3682 | CB | GLU | C | 50 | -17.341 | -43.542 | 81.204 | 1.00 | 28.42 | C |
| ATOM | 3683 | CG | GLU | C | 50 | -15.895 | -43.237 | 80.715 | 1.00 | 27.97 | C |
| ATOM | 3684 | CD | GLU | C | 50 | -15.501 | -41.745 | 80.792 | 1.00 | 33.91 | C |
| ATOM | 3685 | OE1 | GLU | C | 50 | -14.488 | -41.342 | 80.144 | 1.00 | 32.01 | O |
| ATOM | 3686 | OE2 | GLU | C | 50 | -16.189 | -40.969 | 81.511 | 1.00 | 33.87 | O1- |
| ATOM | 3687 | N | ILE | C | 51 | -19.006 | -45.009 | 78.914 | 1.00 | 31.49 | N |
| ATOM | 3688 | CA | ILE | C | 51 | -19.254 | -45.162 | 77.481 | 1.00 | 29.58 | C |

| | | | | | | | | | | | |
|---|---|---|---|---|---|---|---|---|---|---|---|
| ATOM | 3689 | C | ILE | C | 51 | -19.904 | -43.883 | 76.949 | 1.00 | 36.19 | C |
| ATOM | 3690 | O | ILE | C | 51 | -20.636 | -43.193 | 77.666 | 1.00 | 32.17 | O |
| ATOM | 3691 | CB | ILE | C | 51 | -20.128 | -46.407 | 77.173 | 1.00 | 30.54 | C |
| ATOM | 3692 | CG1 | ILE | C | 51 | -20.175 | -46.709 | 75.657 | 1.00 | 30.91 | C |
| ATOM | 3693 | CG2 | ILE | C | 51 | -21.541 | -46.263 | 77.756 | 1.00 | 22.08 | C |
| ATOM | 3694 | CD1 | ILE | C | 51 | -20.472 | -48.177 | 75.340 | 1.00 | 21.13 | C |
| ATOM | 3695 | N | SER | C | 52 | -19.687 | -43.604 | 75.652 | 1.00 | 44.18 | N |
| ATOM | 3696 | CA | SER | C | 52 | -19.747 | -42.237 | 75.128 | 1.00 | 42.64 | C |
| ATOM | 3697 | C | SER | C | 52 | -21.001 | -41.880 | 74.342 | 1.00 | 41.94 | C |
| ATOM | 3698 | O | SER | C | 52 | -21.272 | -40.681 | 74.190 | 1.00 | 50.21 | O |
| ATOM | 3699 | CB | SER | C | 52 | -18.545 | -41.962 | 74.210 | 1.00 | 46.91 | C |
| ATOM | 3700 | OG | SER | C | 52 | -18.720 | -42.561 | 72.930 | 1.00 | 51.82 | O |
| ATOM | 3701 | N | HIS | C | 53 | -21.739 | -42.865 | 73.831 | 1.00 | 36.80 | N |
| ATOM | 3702 | CA | HIS | C | 53 | -22.860 | -42.688 | 72.900 | 1.00 | 46.33 | C |
| ATOM | 3703 | C | HIS | C | 53 | -22.439 | -43.129 | 71.515 | 1.00 | 45.86 | C |
| ATOM | 3704 | O | HIS | C | 53 | -23.241 | -43.704 | 70.781 | 1.00 | 49.43 | O |
| ATOM | 3705 | CB | HIS | C | 53 | -23.410 | -41.251 | 72.789 | 1.00 | 46.91 | C |
| ATOM | 3706 | CG | HIS | C | 53 | -24.569 | -41.127 | 71.846 | 1.00 | 52.45 | C |
| ATOM | 3707 | ND1 | HIS | C | 53 | -24.405 | -40.953 | 70.488 | 1.00 | 47.45 | N |
| ATOM | 3708 | CD2 | HIS | C | 53 | -25.907 | -41.185 | 72.059 | 1.00 | 50.31 | C |
| ATOM | 3709 | CE1 | HIS | C | 53 | -25.590 | -40.915 | 69.905 | 1.00 | 43.08 | C |
| ATOM | 3710 | NE2 | HIS | C | 53 | -26.519 | -41.054 | 70.835 | 1.00 | 45.78 | N |
| ATOM | 3711 | N | SER | C | 54 | -21.183 | -42.881 | 71.170 | 1.00 | 47.96 | N |
| ATOM | 3712 | CA | SER | C | 54 | -20.637 | -43.213 | 69.863 | 1.00 | 43.93 | C |
| ATOM | 3713 | C | SER | C | 54 | -20.189 | -44.668 | 69.568 | 1.00 | 48.52 | C |
| ATOM | 3714 | O | SER | C | 54 | -19.920 | -44.942 | 68.399 | 1.00 | 63.14 | O |
| ATOM | 3715 | CB | SER | C | 54 | -19.464 | -42.299 | 69.569 | 1.00 | 40.27 | C |
| ATOM | 3716 | OG | SER | C | 54 | -18.399 | -42.599 | 70.436 | 1.00 | 55.03 | O |
| ATOM | 3717 | N | GLY | C | 55 | -19.968 | -45.572 | 70.529 | 1.00 | 41.03 | N |
| ATOM | 3718 | CA | GLY | C | 55 | -19.813 | -45.352 | 71.953 | 1.00 | 41.92 | C |
| ATOM | 3719 | C | GLY | C | 55 | -18.437 | -45.851 | 72.360 | 1.00 | 42.37 | C |
| ATOM | 3720 | O | GLY | C | 55 | -18.224 | -47.051 | 72.578 | 1.00 | 27.61 | O |
| ATOM | 3721 | N | SER | C | 56 | -17.483 | -44.927 | 72.441 | 1.00 | 44.03 | N |
| ATOM | 3722 | CA | SER | C | 56 | -16.156 | -45.301 | 72.891 | 1.00 | 40.77 | C |
| ATOM | 3723 | C | SER | C | 56 | -16.173 | -45.524 | 74.394 | 1.00 | 39.74 | C |
| ATOM | 3724 | O | SER | C | 56 | -17.034 | -45.019 | 75.114 | 1.00 | 41.06 | O |
| ATOM | 3725 | CB | SER | C | 56 | -15.122 | -44.243 | 72.520 | 1.00 | 39.37 | C |
| ATOM | 3726 | OG | SER | C | 56 | -15.509 | -42.986 | 73.018 | 1.00 | 48.88 | O |
| ATOM | 3727 | N | THR | C | 57 | -15.206 | -46.299 | 74.857 | 1.00 | 38.23 | N |
| ATOM | 3728 | CA | THR | C | 57 | -15.193 | -46.845 | 76.196 | 1.00 | 29.07 | C |
| ATOM | 3729 | C | THR | C | 57 | -13.912 | -46.459 | 76.932 | 1.00 | 32.82 | C |
| ATOM | 3730 | O | THR | C | 57 | -12.865 | -46.266 | 76.312 | 1.00 | 34.18 | O |
| ATOM | 3731 | CB | THR | C | 57 | -15.325 | -48.352 | 76.054 | 1.00 | 31.23 | C |
| ATOM | 3732 | OG1 | THR | C | 57 | -16.527 | -48.801 | 76.681 | 1.00 | 34.61 | O |
| ATOM | 3733 | CG2 | THR | C | 57 | -14.109 | -49.052 | 76.571 | 1.00 | 31.55 | C |
| ATOM | 3734 | N | ASN | C | 58 | -14.002 | -46.318 | 78.257 | 1.00 | 31.01 | N |
| ATOM | 3735 | CA | ASN | C | 58 | -12.838 | -46.118 | 79.121 | 1.00 | 27.41 | C |
| ATOM | 3736 | C | ASN | C | 58 | -13.078 | -46.909 | 80.389 | 1.00 | 30.68 | C |
| ATOM | 3737 | O | ASN | C | 58 | -14.007 | -46.592 | 81.136 | 1.00 | 31.50 | O |
| ATOM | 3738 | CB | ASN | C | 58 | -12.590 | -44.649 | 79.491 | 1.00 | 24.84 | C |
| ATOM | 3739 | CG | ASN | C | 58 | -12.449 | -43.760 | 78.297 | 1.00 | 28.91 | C |
| ATOM | 3740 | OD1 | ASN | C | 58 | -11.439 | -43.792 | 77.598 | 1.00 | 34.90 | O |
| ATOM | 3741 | ND2 | ASN | C | 58 | -13.458 | -42.937 | 78.055 | 1.00 | 33.30 | N |
| ATOM | 3742 | N | TYR | C | 59 | -12.249 | -47.915 | 80.640 | 1.00 | 29.82 | N |
| ATOM | 3743 | CA | TYR | C | 59 | -12.450 | -48.796 | 81.775 | 1.00 | 33.27 | C |
| ATOM | 3744 | C | TYR | C | 59 | -11.447 | -48.512 | 82.871 | 1.00 | 30.48 | C |
| ATOM | 3745 | O | TYR | C | 59 | -10.402 | -47.903 | 82.644 | 1.00 | 32.69 | O |
| ATOM | 3746 | CB | TYR | C | 59 | -12.326 | -50.263 | 81.379 | 1.00 | 31.17 | C |
| ATOM | 3747 | CG | TYR | C | 59 | -13.321 | -50.726 | 80.367 | 1.00 | 34.24 | C |
| ATOM | 3748 | CD1 | TYR | C | 59 | -14.679 | -50.757 | 80.670 | 1.00 | 35.71 | C |
| ATOM | 3749 | CD2 | TYR | C | 59 | -12.913 | -51.192 | 79.125 | 1.00 | 34.28 | C |
| ATOM | 3750 | CE1 | TYR | C | 59 | -15.611 | -51.214 | 79.741 | 1.00 | 37.87 | C |
| ATOM | 3751 | CE2 | TYR | C | 59 | -13.837 | -51.665 | 78.194 | 1.00 | 33.51 | C |
| ATOM | 3752 | CZ | TYR | C | 59 | -15.183 | -51.659 | 78.498 | 1.00 | 33.62 | C |
| ATOM | 3753 | OH | TYR | C | 59 | -16.095 | -52.115 | 77.572 | 1.00 | 34.03 | O |
| ATOM | 3754 | N | ASN | C | 60 | -11.765 | -48.993 | 84.060 | 1.00 | 29.37 | N |
| ATOM | 3755 | CA | ASN | C | 60 | -10.778 | -48.990 | 85.128 | 1.00 | 38.84 | C |
| ATOM | 3756 | C | ASN | C | 60 | -9.747 | -50.095 | 84.877 | 1.00 | 40.23 | C |
| ATOM | 3757 | O | ASN | C | 60 | -10.119 | -51.280 | 84.829 | 1.00 | 38.66 | O |
| ATOM | 3758 | CB | ASN | C | 60 | -11.437 | -49.191 | 86.480 | 1.00 | 32.03 | C |
| ATOM | 3759 | CG | ASN | C | 60 | -10.484 | -48.942 | 87.617 | 1.00 | 34.48 | C |

| | | | | | | | | | | | |
|---|---|---|---|---|---|---|---|---|---|---|---|
| ATOM | 3760 | OD1 | ASN | C | 60 | -9.270 | -48.906 | 87.422 | 1.00 | 34.69 | O |
| ATOM | 3761 | ND2 | ASN | C | 60 | -11.018 | -48.808 | 88.819 | 1.00 | 30.93 | N |
| ATOM | 3762 | N | PRO | C | 61 | -8.458 | -49.763 | 84.741 | 1.00 | 38.19 | N |
| ATOM | 3763 | CA | PRO | C | 61 | -7.459 | -50.799 | 84.419 | 1.00 | 38.23 | C |
| ATOM | 3764 | C | PRO | C | 61 | -7.385 | -51.943 | 85.412 | 1.00 | 36.76 | C |
| ATOM | 3765 | O | PRO | C | 61 | -6.981 | -53.047 | 85.035 | 1.00 | 42.26 | O |
| ATOM | 3766 | CB | PRO | C | 61 | -6.156 | -49.991 | 84.368 | 1.00 | 37.21 | C |
| ATOM | 3767 | CG | PRO | C | 61 | -6.609 | -48.658 | 83.864 | 1.00 | 35.33 | C |
| ATOM | 3768 | CD | PRO | C | 61 | -7.870 | -48.412 | 84.660 | 1.00 | 38.00 | C |
| ATOM | 3769 | N | SER | C | 62 | -7.766 | -51.718 | 86.663 | 1.00 | 37.94 | N |
| ATOM | 3770 | CA | SER | C | 62 | -7.755 | -52.781 | 87.665 | 1.00 | 41.69 | C |
| ATOM | 3771 | C | SER | C | 62 | -8.781 | -53.878 | 87.376 | 1.00 | 45.04 | C |
| ATOM | 3772 | O | SER | C | 62 | -8.587 | -55.025 | 87.790 | 1.00 | 52.57 | O |
| ATOM | 3773 | CB | SER | C | 62 | -8.016 | -52.197 | 89.039 | 1.00 | 36.72 | C |
| ATOM | 3774 | OG | SER | C | 62 | -9.370 | -51.807 | 89.106 | 1.00 | 43.47 | O |
| ATOM | 3775 | N | LEU | C | 63 | -9.927 | -53.530 | 86.792 | 1.00 | 39.84 | N |
| ATOM | 3776 | CA | LEU | C | 63 | -10.881 | -54.557 | 86.394 | 1.00 | 42.71 | C |
| ATOM | 3777 | C | LEU | C | 63 | -10.311 | -55.431 | 85.298 | 1.00 | 47.05 | C |
| ATOM | 3778 | O | LEU | C | 63 | -10.608 | -56.635 | 85.235 | 1.00 | 44.87 | O |
| ATOM | 3779 | CB | LEU | C | 63 | -12.177 | -53.929 | 85.914 | 1.00 | 39.14 | C |
| ATOM | 3780 | CG | LEU | C | 63 | -12.942 | -53.409 | 87.106 | 1.00 | 41.14 | C |
| ATOM | 3781 | CD1 | LEU | C | 63 | -14.324 | -52.965 | 86.651 | 1.00 | 39.64 | C |
| ATOM | 3782 | CD2 | LEU | C | 63 | -13.003 | -54.481 | 88.194 | 1.00 | 37.86 | C |
| ATOM | 3783 | N | LYS | C | 64 | -9.555 | -54.816 | 84.389 | 1.00 | 50.10 | N |
| ATOM | 3784 | CA | LYS | C | 64 | -8.831 | -55.520 | 83.346 | 1.00 | 45.99 | C |
| ATOM | 3785 | C | LYS | C | 64 | -9.797 | -56.175 | 82.376 | 1.00 | 46.14 | C |
| ATOM | 3786 | O | LYS | C | 64 | -10.663 | -55.500 | 81.805 | 1.00 | 47.95 | O |
| ATOM | 3787 | CB | LYS | C | 64 | -7.875 | -56.515 | 84.000 | 1.00 | 45.38 | C |
| ATOM | 3788 | CG | LYS | C | 64 | -6.494 | -56.522 | 83.397 | 1.00 | 56.60 | C |
| ATOM | 3789 | CD | LYS | C | 64 | -5.651 | -57.503 | 84.141 | 1.00 | 57.39 | C |
| ATOM | 3790 | CE | LYS | C | 64 | -5.450 | -56.943 | 85.551 | 1.00 | 64.49 | C |
| ATOM | 3791 | NZ | LYS | C | 64 | -4.775 | -57.891 | 86.483 | 1.00 | 75.39 | N1+ |
| ATOM | 3792 | N | ALA | C | 65 | -9.676 | -57.484 | 82.203 | 1.00 | 43.80 | N |
| ATOM | 3793 | CA | ALA | C | 65 | -10.484 | -58.168 | 81.210 | 1.00 | 43.71 | C |
| ATOM | 3794 | C | ALA | C | 65 | -11.908 | -58.474 | 81.670 | 1.00 | 40.71 | C |
| ATOM | 3795 | O | ALA | C | 65 | -12.730 | -58.856 | 80.830 | 1.00 | 46.79 | O |
| ATOM | 3796 | CB | ALA | C | 65 | -9.789 | -59.462 | 80.808 | 1.00 | 46.98 | C |
| ATOM | 3797 | N | ARG | C | 66 | -12.232 | -58.303 | 82.958 | 1.00 | 34.69 | N |
| ATOM | 3798 | CA | ARG | C | 66 | -13.508 | -58.800 | 83.490 | 1.00 | 35.91 | C |
| ATOM | 3799 | C | ARG | C | 66 | -14.721 | -57.969 | 83.088 | 1.00 | 34.48 | C |
| ATOM | 3800 | O | ARG | C | 66 | -15.841 | -58.467 | 83.207 | 1.00 | 34.86 | O |
| ATOM | 3801 | CB | ARG | C | 66 | -13.467 | -58.840 | 85.013 | 1.00 | 37.25 | C |
| ATOM | 3802 | CG | ARG | C | 66 | -12.241 | -59.497 | 85.584 | 1.00 | 41.56 | C |
| ATOM | 3803 | CD | ARG | C | 66 | -12.258 | -59.442 | 87.090 | 1.00 | 33.48 | C |
| ATOM | 3804 | NE | ARG | C | 66 | -13.537 | -59.870 | 87.586 | 1.00 | 36.42 | N |
| ATOM | 3805 | CZ | ARG | C | 66 | -14.096 | -59.444 | 88.710 | 1.00 | 31.00 | C |
| ATOM | 3806 | NH1 | ARG | C | 66 | -15.275 | -59.921 | 89.045 | 1.00 | 32.67 | N |
| ATOM | 3807 | NH2 | ARG | C | 66 | -13.499 | -58.556 | 89.483 | 1.00 | 29.13 | N |
| ATOM | 3808 | N | VAL | C | 67 | -14.545 | -56.720 | 82.650 | 1.00 | 36.81 | N |
| ATOM | 3809 | CA | VAL | C | 67 | -15.642 | -55.760 | 82.552 | 1.00 | 33.37 | C |
| ATOM | 3810 | C | VAL | C | 67 | -15.923 | -55.381 | 81.104 | 1.00 | 38.37 | C |
| ATOM | 3811 | O | VAL | C | 67 | -15.005 | -55.251 | 80.288 | 1.00 | 41.21 | O |
| ATOM | 3812 | CB | VAL | C | 67 | -15.353 | -54.501 | 83.391 | 1.00 | 36.36 | C |
| ATOM | 3813 | CG1 | VAL | C | 67 | -14.134 | -53.780 | 82.865 | 1.00 | 34.65 | C |
| ATOM | 3814 | CG2 | VAL | C | 67 | -16.552 | -53.582 | 83.346 | 1.00 | 36.14 | C |
| ATOM | 3815 | N | THR | C | 68 | -17.203 | -55.212 | 80.792 | 1.00 | 35.08 | N |
| ATOM | 3816 | CA | THR | C | 68 | -17.654 | -54.712 | 79.510 | 1.00 | 32.85 | C |
| ATOM | 3817 | C | THR | C | 68 | -18.769 | -53.715 | 79.758 | 1.00 | 29.58 | C |
| ATOM | 3818 | O | THR | C | 68 | -19.686 | -53.981 | 80.536 | 1.00 | 30.01 | O |
| ATOM | 3819 | CB | THR | C | 68 | -18.196 | -55.829 | 78.605 | 1.00 | 36.95 | C |
| ATOM | 3820 | OG1 | THR | C | 68 | -17.324 | -56.957 | 78.657 | 1.00 | 39.01 | O |
| ATOM | 3821 | CG2 | THR | C | 68 | -18.339 | -55.320 | 77.154 | 1.00 | 28.49 | C |
| ATOM | 3822 | N | ILE | C | 69 | -18.719 | -52.595 | 79.060 | 1.00 | 33.88 | N |
| ATOM | 3823 | CA | ILE | C | 69 | -19.792 | -51.617 | 79.088 | 1.00 | 33.76 | C |
| ATOM | 3824 | C | ILE | C | 69 | -20.257 | -51.398 | 77.658 | 1.00 | 34.31 | C |
| ATOM | 3825 | O | ILE | C | 69 | -19.439 | -51.192 | 76.757 | 1.00 | 34.33 | O |
| ATOM | 3826 | CB | ILE | C | 69 | -19.349 | -50.307 | 79.752 | 1.00 | 25.14 | C |
| ATOM | 3827 | CG1 | ILE | C | 69 | -19.047 | -50.590 | 81.218 | 1.00 | 24.64 | C |
| ATOM | 3828 | CG2 | ILE | C | 69 | -20.418 | -49.246 | 79.567 | 1.00 | 21.90 | C |
| ATOM | 3829 | CD1 | ILE | C | 69 | -18.299 | -49.486 | 81.965 | 1.00 | 29.90 | C |
| ATOM | 3830 | N | SER | C | 70 | -21.567 | -51.459 | 77.455 | 1.00 | 35.47 | N |

| | | | | | | | | | | | |
|---|---|---|---|---|---|---|---|---|---|---|---|
| ATOM | 3831 | CA | SER | C | 70 | -22.170 | -51.319 | 76.141 | 1.00 | 33.35 | C |
| ATOM | 3832 | C | SER | C | 70 | -23.321 | -50.323 | 76.226 | 1.00 | 31.43 | C |
| ATOM | 3833 | O | SER | C | 70 | -23.862 | -50.073 | 77.305 | 1.00 | 30.39 | O |
| ATOM | 3834 | CB | SER | C | 70 | -22.654 | -52.668 | 75.638 | 1.00 | 23.50 | C |
| ATOM | 3835 | OG | SER | C | 70 | -23.508 | -53.222 | 76.609 | 1.00 | 35.38 | O |
| ATOM | 3836 | N | ILE | C | 71 | -23.683 | -49.755 | 75.074 | 1.00 | 30.99 | N |
| ATOM | 3837 | CA | ILE | C | 71 | -24.705 | -48.722 | 74.981 | 1.00 | 30.48 | C |
| ATOM | 3838 | C | ILE | C | 71 | -25.660 | -49.058 | 73.838 | 1.00 | 34.95 | C |
| ATOM | 3839 | O | ILE | C | 71 | -25.246 | -49.587 | 72.806 | 1.00 | 34.86 | O |
| ATOM | 3840 | CB | ILE | C | 71 | -24.064 | -47.335 | 74.790 | 1.00 | 32.81 | C |
| ATOM | 3841 | CG1 | ILE | C | 71 | -25.061 | -46.237 | 75.120 | 1.00 | 29.16 | C |
| ATOM | 3842 | CG2 | ILE | C | 71 | -23.480 | -47.167 | 73.376 | 1.00 | 30.88 | C |
| ATOM | 3843 | CD1 | ILE | C | 71 | -24.442 | -44.876 | 75.149 | 1.00 | 30.80 | C |
| ATOM | 3844 | N | ASP | C | 72 | -26.949 | -48.798 | 74.042 | 1.00 | 39.76 | N |
| ATOM | 3845 | CA | ASP | C | 72 | -27.988 | -48.981 | 73.025 | 1.00 | 35.04 | C |
| ATOM | 3846 | C | ASP | C | 72 | -28.651 | -47.630 | 72.771 | 1.00 | 39.02 | C |
| ATOM | 3847 | O | ASP | C | 72 | -29.552 | -47.241 | 73.518 | 1.00 | 43.96 | O |
| ATOM | 3848 | CB | ASP | C | 72 | -29.012 | -50.011 | 73.491 | 1.00 | 37.61 | C |
| ATOM | 3849 | CG | ASP | C | 72 | -30.119 | -50.283 | 72.465 | 1.00 | 45.97 | C |
| ATOM | 3850 | OD1 | ASP | C | 72 | -30.612 | -49.345 | 71.798 | 1.00 | 48.11 | O |
| ATOM | 3851 | OD2 | ASP | C | 72 | -30.518 | -51.461 | 72.346 | 1.00 | 49.16 | O1- |
| ATOM | 3852 | N | THR | C | 73 | -28.231 | -46.910 | 71.726 | 1.00 | 38.09 | N |
| ATOM | 3853 | CA | THR | C | 73 | -28.807 | -45.586 | 71.501 | 1.00 | 40.83 | C |
| ATOM | 3854 | C | THR | C | 73 | -30.255 | -45.626 | 71.029 | 1.00 | 44.67 | C |
| ATOM | 3855 | O | THR | C | 73 | -30.892 | -44.568 | 70.997 | 1.00 | 48.41 | O |
| ATOM | 3856 | CB | THR | C | 73 | -27.996 | -44.759 | 70.502 | 1.00 | 39.06 | C |
| ATOM | 3857 | OG1 | THR | C | 73 | -27.774 | -45.521 | 69.308 | 1.00 | 46.00 | O |
| ATOM | 3858 | CG2 | THR | C | 73 | -26.679 | -44.327 | 71.114 | 1.00 | 40.64 | C |
| ATOM | 3859 | O | SER | C | 74 | -33.984 | -45.804 | 71.487 | 1.00 | 55.05 | O |
| ATOM | 3860 | N | SER | C | 74 | -30.781 | -46.782 | 70.622 | 1.00 | 41.41 | N |
| ATOM | 3861 | CA | SER | C | 74 | -32.194 | -46.829 | 70.244 | 1.00 | 49.65 | C |
| ATOM | 3862 | C | SER | C | 74 | -33.087 | -46.665 | 71.466 | 1.00 | 55.28 | C |
| ATOM | 3863 | CB | SER | C | 74 | -32.524 | -48.149 | 69.539 | 1.00 | 50.83 | C |
| ATOM | 3864 | OG | SER | C | 74 | -31.745 | -48.338 | 68.375 | 1.00 | 60.96 | O |
| ATOM | 3865 | O | LYS | C | 75 | -33.672 | -46.626 | 75.929 | 1.00 | 49.05 | O |
| ATOM | 3866 | N | LYS | C | 75 | -32.833 | -47.481 | 72.498 | 1.00 | 48.96 | N |
| ATOM | 3867 | CA | LYS | C | 75 | -33.603 | -47.551 | 73.730 | 1.00 | 48.48 | C |
| ATOM | 3868 | C | LYS | C | 75 | -33.120 | -46.598 | 74.819 | 1.00 | 45.98 | C |
| ATOM | 3869 | CB | LYS | C | 75 | -33.594 | -48.986 | 74.274 | 1.00 | 47.37 | C |
| ATOM | 3870 | CG | LYS | C | 75 | -34.202 | -49.988 | 73.320 | 1.00 | 54.32 | C |
| ATOM | 3871 | CD | LYS | C | 75 | -34.219 | -51.397 | 73.886 | 1.00 | 53.30 | C |
| ATOM | 3872 | CE | LYS | C | 75 | -34.656 | -52.383 | 72.819 | 1.00 | 62.19 | C |
| ATOM | 3873 | NZ | LYS | C | 75 | -35.421 | -53.518 | 73.409 | 1.00 | 63.45 | N |
| ATOM | 3874 | O | ASN | C | 76 | -31.622 | -45.589 | 77.918 | 1.00 | 39.83 | O |
| ATOM | 3875 | N | ASN | C | 76 | -32.109 | -45.772 | 74.550 | 1.00 | 41.83 | N |
| ATOM | 3876 | CA | ASN | C | 76 | -31.496 | -44.951 | 75.593 | 1.00 | 43.47 | C |
| ATOM | 3877 | C | ASN | C | 76 | -31.146 | -45.811 | 76.807 | 1.00 | 36.05 | C |
| ATOM | 3878 | CB | ASN | C | 76 | -32.402 | -43.771 | 75.976 | 1.00 | 36.10 | C |
| ATOM | 3879 | CG | ASN | C | 76 | -32.342 | -42.656 | 74.963 | 1.00 | 38.85 | C |
| ATOM | 3880 | OD1 | ASN | C | 76 | -31.821 | -42.833 | 73.857 | 1.00 | 46.14 | O |
| ATOM | 3881 | ND2 | ASN | C | 76 | -32.863 | -41.498 | 75.326 | 1.00 | 45.02 | N |
| ATOM | 3882 | N | GLN | C | 77 | -30.384 | -46.868 | 76.554 | 1.00 | 35.70 | N |
| ATOM | 3883 | CA | GLN | C | 77 | -29.976 | -47.812 | 77.580 | 1.00 | 35.90 | C |
| ATOM | 3884 | C | GLN | C | 77 | -28.472 | -48.013 | 77.506 | 1.00 | 36.92 | C |
| ATOM | 3885 | O | GLN | C | 77 | -27.858 | -47.827 | 76.452 | 1.00 | 34.91 | O |
| ATOM | 3886 | CB | GLN | C | 77 | -30.697 | -49.147 | 77.409 | 1.00 | 33.22 | C |
| ATOM | 3887 | CG | GLN | C | 77 | -32.184 | -49.061 | 77.707 | 1.00 | 37.44 | C |
| ATOM | 3888 | CD | GLN | C | 77 | -32.889 | -50.386 | 77.542 | 1.00 | 35.36 | C |
| ATOM | 3889 | OE1 | GLN | C | 77 | -32.301 | -51.358 | 77.088 | 1.00 | 30.09 | O |
| ATOM | 3890 | NE2 | GLN | C | 77 | -34.154 | -50.431 | 77.919 | 1.00 | 37.97 | N |
| ATOM | 3891 | N | PHE | C | 78 | -27.882 | -48.404 | 78.638 | 1.00 | 30.45 | N |
| ATOM | 3892 | CA | PHE | C | 78 | -26.495 | -48.839 | 78.650 | 1.00 | 28.00 | C |
| ATOM | 3893 | C | PHE | C | 78 | -26.342 | -49.884 | 79.739 | 1.00 | 32.46 | C |
| ATOM | 3894 | O | PHE | C | 78 | -27.128 | -49.933 | 80.686 | 1.00 | 33.14 | O |
| ATOM | 3895 | CB | PHE | C | 78 | -25.501 | -47.668 | 78.812 | 1.00 | 29.59 | C |
| ATOM | 3896 | CG | PHE | C | 78 | -25.617 | -46.911 | 80.109 | 1.00 | 29.92 | C |
| ATOM | 3897 | CD1 | PHE | C | 78 | -26.541 | -45.898 | 80.252 | 1.00 | 28.69 | C |
| ATOM | 3898 | CD2 | PHE | C | 78 | -24.767 | -47.182 | 81.160 | 1.00 | 29.77 | C |
| ATOM | 3899 | CE1 | PHE | C | 78 | -26.635 | -45.191 | 81.422 | 1.00 | 31.87 | C |
| ATOM | 3900 | CE2 | PHE | C | 78 | -24.865 | -46.484 | 82.343 | 1.00 | 32.84 | C |
| ATOM | 3901 | CZ | PHE | C | 78 | -25.800 | -45.481 | 82.475 | 1.00 | 32.57 | C |

| | | | | | | | | | | | |
|---|---|---|---|---|---|---|---|---|---|---|---|
| ATOM | 3902 | N | SER | C | 79 | -25.321 | -50.732 | 79.595 | 1.00 | 30.53 | N |
| ATOM | 3903 | CA | SER | C | 79 | -25.288 | -51.973 | 80.344 | 1.00 | 27.00 | C |
| ATOM | 3904 | C | SER | C | 79 | -23.887 | -52.250 | 80.869 | 1.00 | 29.56 | C |
| ATOM | 3905 | O | SER | C | 79 | -22.890 | -51.729 | 80.365 | 1.00 | 29.66 | O |
| ATOM | 3906 | CB | SER | C | 79 | -25.778 | -53.134 | 79.474 | 1.00 | 30.28 | C |
| ATOM | 3907 | OG | SER | C | 79 | -27.128 | -52.938 | 79.104 | 1.00 | 31.71 | O |
| ATOM | 3908 | N | LEU | C | 80 | -23.833 | -53.114 | 81.879 | 1.00 | 24.67 | N |
| ATOM | 3909 | CA | LEU | C | 80 | -22.595 | -53.502 | 82.522 | 1.00 | 26.25 | C |
| ATOM | 3910 | C | LEU | C | 80 | -22.509 | -55.012 | 82.551 | 1.00 | 31.30 | C |
| ATOM | 3911 | O | LEU | C | 80 | -23.454 | -55.677 | 82.982 | 1.00 | 32.63 | O |
| ATOM | 3912 | CB | LEU | C | 80 | -22.519 | -52.968 | 83.957 | 1.00 | 30.49 | C |
| ATOM | 3913 | CG | LEU | C | 80 | -21.310 | -53.471 | 84.764 | 1.00 | 31.84 | C |
| ATOM | 3914 | CD1 | LEU | C | 80 | -19.986 | -52.935 | 84.206 | 1.00 | 24.42 | C |
| ATOM | 3915 | CD2 | LEU | C | 80 | -21.459 | -53.149 | 86.240 | 1.00 | 29.26 | C |
| ATOM | 3916 | N | LYS | C | 81 | -21.377 | -55.549 | 82.111 | 1.00 | 29.00 | N |
| ATOM | 3917 | CA | LYS | C | 81 | -21.065 | -56.958 | 82.299 | 1.00 | 31.81 | C |
| ATOM | 3918 | C | LYS | C | 81 | -19.803 | -57.045 | 83.139 | 1.00 | 31.17 | C |
| ATOM | 3919 | O | LYS | C | 81 | -18.822 | -56.361 | 82.845 | 1.00 | 34.99 | O |
| ATOM | 3920 | CB | LYS | C | 81 | -20.864 | -57.684 | 80.964 | 1.00 | 28.39 | C |
| ATOM | 3921 | CG | LYS | C | 81 | -22.140 | -57.880 | 80.163 | 1.00 | 33.95 | C |
| ATOM | 3922 | CD | LYS | C | 81 | -22.873 | -59.151 | 80.545 | 1.00 | 40.33 | C |
| ATOM | 3923 | CE | LYS | C | 81 | -24.202 | -59.282 | 79.817 | 1.00 | 42.26 | C |
| ATOM | 3924 | NZ | LYS | C | 81 | -24.025 | -59.096 | 78.349 | 1.00 | 53.78 | N1+ |
| ATOM | 3925 | N | LEU | C | 82 | -19.835 | -57.857 | 84.193 | 1.00 | 30.70 | N |
| ATOM | 3926 | CA | LEU | C | 82 | -18.652 | -58.141 | 85.004 | 1.00 | 28.52 | C |
| ATOM | 3927 | C | LEU | C | 82 | -18.571 | -59.645 | 85.180 | 1.00 | 31.87 | C |
| ATOM | 3928 | O | LEU | C | 82 | -19.434 | -60.247 | 85.824 | 1.00 | 37.82 | O |
| ATOM | 3929 | CB | LEU | C | 82 | -18.701 | -57.439 | 86.356 | 1.00 | 33.35 | C |
| ATOM | 3930 | CG | LEU | C | 82 | -17.543 | -57.699 | 87.330 | 1.00 | 35.28 | C |
| ATOM | 3931 | CD1 | LEU | C | 82 | -16.244 | -57.034 | 86.881 | 1.00 | 26.92 | C |
| ATOM | 3932 | CD2 | LEU | C | 82 | -17.950 | -57.297 | 88.741 | 1.00 | 25.50 | C |
| ATOM | 3933 | N | ARG | C | 83 | -17.554 | -60.251 | 84.600 | 1.00 | 36.83 | N |
| ATOM | 3934 | CA | ARG | C | 83 | -17.451 | -61.703 | 84.527 | 1.00 | 39.83 | C |
| ATOM | 3935 | C | ARG | C | 83 | -16.737 | -62.289 | 85.745 | 1.00 | 38.25 | C |
| ATOM | 3936 | O | ARG | C | 83 | -16.042 | -61.586 | 86.490 | 1.00 | 34.22 | O |
| ATOM | 3937 | CB | ARG | C | 83 | -16.745 | -62.101 | 83.221 | 1.00 | 31.30 | C |
| ATOM | 3938 | CG | ARG | C | 83 | -17.726 | -62.259 | 82.070 | 1.00 | 37.56 | C |
| ATOM | 3939 | CD | ARG | C | 83 | -17.165 | -62.116 | 80.661 | 1.00 | 34.40 | C |
| ATOM | 3940 | NE | ARG | C | 83 | -17.224 | -60.730 | 80.213 | 1.00 | 37.07 | N |
| ATOM | 3941 | CZ | ARG | C | 83 | -16.174 | -59.946 | 80.029 | 1.00 | 39.95 | C |
| ATOM | 3942 | NH1 | ARG | C | 83 | -16.359 | -58.701 | 79.618 | 1.00 | 31.66 | N1+ |
| ATOM | 3943 | NH2 | ARG | C | 83 | -14.947 | -60.412 | 80.249 | 1.00 | 46.34 | N |
| ATOM | 3944 | N | SER | C | 84 | -16.975 | -63.586 | 85.964 | 1.00 | 35.95 | N |
| ATOM | 3945 | CA | SER | C | 84 | -16.215 | -64.416 | 86.908 | 1.00 | 36.75 | C |
| ATOM | 3946 | C | SER | C | 84 | -16.089 | -63.725 | 88.271 | 1.00 | 37.32 | C |
| ATOM | 3947 | O | SER | C | 84 | -15.020 | -63.344 | 88.754 | 1.00 | 41.30 | O |
| ATOM | 3948 | CB | SER | C | 84 | -14.857 | -64.809 | 86.316 | 1.00 | 31.04 | C |
| ATOM | 3949 | OG | SER | C | 84 | -14.036 | -63.687 | 86.179 | 1.00 | 38.56 | O |
| ATOM | 3950 | N | VAL | C | 85 | -17.235 | -63.589 | 88.856 | 1.00 | 33.08 | N |
| ATOM | 3951 | CA | VAL | C | 85 | -17.473 | -62.751 | 90.014 | 1.00 | 32.08 | C |
| ATOM | 3952 | C | VAL | C | 85 | -17.088 | -63.499 | 91.292 | 1.00 | 32.80 | C |
| ATOM | 3953 | O | VAL | C | 85 | -17.190 | -64.723 | 91.359 | 1.00 | 32.85 | O |
| ATOM | 3954 | CB | VAL | C | 85 | -18.965 | -62.372 | 89.921 | 1.00 | 32.31 | C |
| ATOM | 3955 | CG1 | VAL | C | 85 | -19.791 | -63.006 | 90.981 | 1.00 | 35.43 | C |
| ATOM | 3956 | CG2 | VAL | C | 85 | -19.159 | -60.893 | 89.771 | 1.00 | 33.02 | C |
| ATOM | 3957 | N | THR | C | 86 | -16.596 | -62.785 | 92.310 | 1.00 | 36.06 | N |
| ATOM | 3958 | CA | THR | C | 86 | -16.263 | -63.389 | 93.614 | 1.00 | 32.42 | C |
| ATOM | 3959 | C | THR | C | 86 | -16.976 | -62.624 | 94.727 | 1.00 | 32.82 | C |
| ATOM | 3960 | O | THR | C | 86 | -17.644 | -61.619 | 94.485 | 1.00 | 32.02 | O |
| ATOM | 3961 | CB | THR | C | 86 | -14.760 | -63.395 | 93.919 | 1.00 | 32.77 | C |
| ATOM | 3962 | OG1 | THR | C | 86 | -14.390 | -62.115 | 94.438 | 1.00 | 35.84 | O |
| ATOM | 3963 | CG2 | THR | C | 86 | -13.914 | -63.727 | 92.683 | 1.00 | 31.07 | C |
| ATOM | 3964 | N | ALA | C | 87 | -16.813 | -63.084 | 95.972 | 1.00 | 34.97 | N |
| ATOM | 3965 | CA | ALA | C | 87 | -17.533 | -62.446 | 97.079 | 1.00 | 35.92 | C |
| ATOM | 3966 | C | ALA | C | 87 | -17.170 | -60.978 | 97.205 | 1.00 | 36.06 | C |
| ATOM | 3967 | O | ALA | C | 87 | -18.015 | -60.163 | 97.593 | 1.00 | 34.31 | O |
| ATOM | 3968 | CB | ALA | C | 87 | -17.241 | -63.146 | 98.407 | 1.00 | 24.03 | C |
| ATOM | 3969 | N | ALA | C | 88 | -15.927 | -60.626 | 96.853 | 1.00 | 32.27 | N |
| ATOM | 3970 | CA | ALA | C | 88 | -15.448 | -59.255 | 96.922 | 1.00 | 32.44 | C |
| ATOM | 3971 | C | ALA | C | 88 | -16.179 | -58.331 | 95.964 | 1.00 | 35.33 | C |
| ATOM | 3972 | O | ALA | C | 88 | -15.973 | -57.119 | 96.027 | 1.00 | 41.10 | O |

| | | | | | | | | | | | |
|---|---|---|---|---|---|---|---|---|---|---|---|
| ATOM | 3973 | CB | ALA | C | 88 | -13.953 | -59.211 | 96.632 | 1.00 | 26.07 | C |
| ATOM | 3974 | N | ASP | C | 89 | -17.024 | -58.861 | 95.090 | 1.00 | 31.77 | N |
| ATOM | 3975 | CA | ASP | C | 89 | -17.818 | -58.037 | 94.203 | 1.00 | 29.68 | C |
| ATOM | 3976 | C | ASP | C | 89 | -19.198 | -57.735 | 94.785 | 1.00 | 28.71 | C |
| ATOM | 3977 | O | ASP | C | 89 | -20.017 | -57.099 | 94.115 | 1.00 | 29.06 | O |
| ATOM | 3978 | CB | ASP | C | 89 | -17.943 | -58.712 | 92.825 | 1.00 | 36.29 | C |
| ATOM | 3979 | CG | ASP | C | 89 | -16.584 | -58.922 | 92.122 | 1.00 | 33.46 | C |
| ATOM | 3980 | OD1 | ASP | C | 89 | -15.893 | -57.927 | 91.817 | 1.00 | 37.02 | O |
| ATOM | 3981 | OD2 | ASP | C | 89 | -16.209 | -60.092 | 91.867 | 1.00 | 34.16 | O1- |
| ATOM | 3982 | N | THR | C | 90 | -19.454 | -58.137 | 96.028 | 1.00 | 27.76 | N |
| ATOM | 3983 | CA | THR | C | 90 | -20.653 | -57.712 | 96.747 | 1.00 | 34.87 | C |
| ATOM | 3984 | C | THR | C | 90 | -20.606 | -56.194 | 96.997 | 1.00 | 27.98 | C |
| ATOM | 3985 | O | THR | C | 90 | -19.694 | -55.699 | 97.659 | 1.00 | 27.17 | O |
| ATOM | 3986 | CB | THR | C | 90 | -20.747 | -58.492 | 98.058 | 1.00 | 32.97 | C |
| ATOM | 3987 | OG1 | THR | C | 90 | -20.912 | -59.884 | 97.753 | 1.00 | 34.09 | O |
| ATOM | 3988 | CG2 | THR | C | 90 | -21.914 | -58.010 | 98.923 | 1.00 | 24.31 | C |
| ATOM | 3989 | N | ALA | C | 91 | -21.575 | -55.456 | 96.461 | 1.00 | 24.84 | N |
| ATOM | 3990 | CA | ALA | C | 91 | -21.553 | -53.998 | 96.516 | 1.00 | 25.95 | C |
| ATOM | 3991 | C | ALA | C | 91 | -22.876 | -53.467 | 95.992 | 1.00 | 29.10 | C |
| ATOM | 3992 | O | ALA | C | 91 | -23.667 | -54.198 | 95.393 | 1.00 | 30.38 | O |
| ATOM | 3993 | CB | ALA | C | 91 | -20.408 | -53.408 | 95.698 | 1.00 | 27.41 | C |
| ATOM | 3994 | N | VAL | C | 92 | -23.121 | -52.183 | 96.249 | 1.00 | 28.41 | N |
| ATOM | 3995 | CA | VAL | C | 92 | -24.154 | -51.471 | 95.517 | 1.00 | 25.07 | C |
| ATOM | 3996 | C | VAL | C | 92 | -23.533 | -50.973 | 94.227 | 1.00 | 26.45 | C |
| ATOM | 3997 | O | VAL | C | 92 | -22.473 | -50.348 | 94.242 | 1.00 | 29.34 | O |
| ATOM | 3998 | CB | VAL | C | 92 | -24.746 | -50.313 | 96.338 | 1.00 | 25.04 | C |
| ATOM | 3999 | CG1 | VAL | C | 92 | -25.682 | -49.486 | 95.466 | 1.00 | 22.80 | C |
| ATOM | 4000 | CG2 | VAL | C | 92 | -25.542 | -50.843 | 97.503 | 1.00 | 26.44 | C |
| ATOM | 4001 | N | TYR | C | 93 | -24.177 | -51.270 | 93.107 | 1.00 | 29.41 | N |
| ATOM | 4002 | CA | TYR | C | 93 | -23.720 | -50.835 | 91.800 | 1.00 | 25.88 | C |
| ATOM | 4003 | C | TYR | C | 93 | -24.646 | -49.722 | 91.336 | 1.00 | 24.87 | C |
| ATOM | 4004 | O | TYR | C | 93 | -25.854 | -49.927 | 91.241 | 1.00 | 28.12 | O |
| ATOM | 4005 | CB | TYR | C | 93 | -23.703 | -52.015 | 90.826 | 1.00 | 23.05 | C |
| ATOM | 4006 | CG | TYR | C | 93 | -22.607 | -53.020 | 91.156 | 1.00 | 28.89 | C |
| ATOM | 4007 | CD1 | TYR | C | 93 | -22.704 | -53.859 | 92.280 | 1.00 | 24.24 | C |
| ATOM | 4008 | CD2 | TYR | C | 93 | -21.462 | -53.115 | 90.359 | 1.00 | 26.34 | C |
| ATOM | 4009 | CE1 | TYR | C | 93 | -21.702 | -54.752 | 92.593 | 1.00 | 23.55 | C |
| ATOM | 4010 | CE2 | TYR | C | 93 | -20.449 | -53.996 | 90.670 | 1.00 | 26.02 | C |
| ATOM | 4011 | CZ | TYR | C | 93 | -20.566 | -54.818 | 91.783 | 1.00 | 26.56 | C |
| ATOM | 4012 | OH | TYR | C | 93 | -19.535 | -55.695 | 92.078 | 1.00 | 23.40 | O |
| ATOM | 4013 | N | TYR | C | 94 | -24.085 | -48.550 | 91.070 | 1.00 | 23.22 | N |
| ATOM | 4014 | CA | TYR | C | 94 | -24.829 | -47.415 | 90.541 | 1.00 | 28.65 | C |
| ATOM | 4015 | C | TYR | C | 94 | -24.425 | -47.179 | 89.097 | 1.00 | 25.80 | C |
| ATOM | 4016 | O | TYR | C | 94 | -23.241 | -47.248 | 88.766 | 1.00 | 24.60 | O |
| ATOM | 4017 | CB | TYR | C | 94 | -24.547 | -46.097 | 91.309 | 1.00 | 25.22 | C |
| ATOM | 4018 | CG | TYR | C | 94 | -24.796 | -46.109 | 92.775 | 1.00 | 22.71 | C |
| ATOM | 4019 | CD1 | TYR | C | 94 | -26.082 | -45.978 | 93.280 | 1.00 | 24.72 | C |
| ATOM | 4020 | CD2 | TYR | C | 94 | -23.738 | -46.219 | 93.672 | 1.00 | 20.09 | C |
| ATOM | 4021 | CE1 | TYR | C | 94 | -26.311 | -45.997 | 94.640 | 1.00 | 22.52 | C |
| ATOM | 4022 | CE2 | TYR | C | 94 | -23.950 | -46.237 | 95.020 | 1.00 | 17.42 | C |
| ATOM | 4023 | CZ | TYR | C | 94 | -25.240 | -46.119 | 95.508 | 1.00 | 27.12 | C |
| ATOM | 4024 | OH | TYR | C | 94 | -25.463 | -46.130 | 96.877 | 1.00 | 35.58 | O |
| ATOM | 4025 | N | CYS | C | 95 | -25.391 | -46.795 | 88.272 | 1.00 | 25.05 | N |
| ATOM | 4026 | CA | CYS | C | 95 | -25.096 | -46.099 | 87.030 | 1.00 | 26.50 | C |
| ATOM | 4027 | C | CYS | C | 95 | -25.220 | -44.602 | 87.287 | 1.00 | 28.56 | C |
| ATOM | 4028 | O | CYS | C | 95 | -25.943 | -44.161 | 88.192 | 1.00 | 25.46 | O |
| ATOM | 4029 | CB | CYS | C | 95 | -26.022 | -46.563 | 85.883 | 1.00 | 27.62 | C |
| ATOM | 4030 | SG | CYS | C | 95 | -27.838 | -46.477 | 86.215 | 1.00 | 41.12 | S |
| ATOM | 4031 | N | ALA | C | 96 | -24.469 | -43.816 | 86.520 | 1.00 | 26.44 | N |
| ATOM | 4032 | CA | ALA | C | 96 | -24.522 | -42.377 | 86.723 | 1.00 | 27.54 | C |
| ATOM | 4033 | C | ALA | C | 96 | -24.302 | -41.656 | 85.408 | 1.00 | 32.97 | C |
| ATOM | 4034 | O | ALA | C | 96 | -23.727 | -42.202 | 84.464 | 1.00 | 34.61 | O |
| ATOM | 4035 | CB | ALA | C | 96 | -23.493 | -41.909 | 87.746 | 1.00 | 24.37 | C |
| ATOM | 4036 | N | ARG | C | 97 | -24.772 | -40.412 | 85.364 | 1.00 | 35.37 | N |
| ATOM | 4037 | CA | ARG | C | 97 | -24.529 | -39.520 | 84.244 | 1.00 | 26.30 | C |
| ATOM | 4038 | C | ARG | C | 97 | -23.290 | -38.679 | 84.524 | 1.00 | 28.79 | C |
| ATOM | 4039 | O | ARG | C | 97 | -23.208 | -38.000 | 85.551 | 1.00 | 27.36 | O |
| ATOM | 4040 | CB | ARG | C | 97 | -25.732 | -38.630 | 83.997 | 1.00 | 29.64 | C |
| ATOM | 4041 | CG | ARG | C | 97 | -25.581 | -37.755 | 82.773 | 1.00 | 32.76 | C |
| ATOM | 4042 | CD | ARG | C | 97 | -26.887 | -37.112 | 82.453 | 1.00 | 32.80 | C |
| ATOM | 4043 | NE | ARG | C | 97 | -26.856 | -35.720 | 82.854 | 1.00 | 39.43 | N |

| | | | | | | | | | | | |
|---|---|---|---|---|---|---|---|---|---|---|---|
| ATOM | 4044 | CZ | ARG | C | 97 | -27.927 | -34.974 | 83.090 | 1.00 | 37.66 | C |
| ATOM | 4045 | NH1 | ARG | C | 97 | -27.763 | -33.707 | 83.447 | 1.00 | 44.97 | N1+ |
| ATOM | 4046 | NH2 | ARG | C | 97 | -29.146 | -35.483 | 82.978 | 1.00 | 39.25 | N |
| ATOM | 4047 | N | ASP | C | 98 | -22.322 | -38.755 | 83.621 | 1.00 | 30.15 | N |
| ATOM | 4048 | CA | ASP | C | 98 | -21.054 | -38.053 | 83.724 | 1.00 | 29.73 | C |
| ATOM | 4049 | C | ASP | C | 98 | -21.145 | -36.751 | 82.929 | 1.00 | 33.62 | C |
| ATOM | 4050 | O | ASP | C | 98 | -21.487 | -36.781 | 81.742 | 1.00 | 31.03 | O |
| ATOM | 4051 | CB | ASP | C | 98 | -19.936 | -38.949 | 83.190 | 1.00 | 30.30 | C |
| ATOM | 4052 | CG | ASP | C | 98 | -18.555 | -38.414 | 83.490 | 1.00 | 31.40 | C |
| ATOM | 4053 | OD1 | ASP | C | 98 | -18.240 | -37.292 | 83.033 | 1.00 | 31.40 | O1- |
| ATOM | 4054 | OD2 | ASP | C | 98 | -17.779 | -39.139 | 84.160 | 1.00 | 32.47 | O |
| ATOM | 4055 | N | TYR | C | 99 | -20.901 | -35.610 | 83.601 | 1.00 | 32.07 | N |
| ATOM | 4056 | CA | TYR | C | 99 | -20.981 | -34.283 | 82.971 | 1.00 | 29.21 | C |
| ATOM | 4057 | C | TYR | C | 99 | -20.323 | -33.081 | 83.723 | 1.00 | 28.12 | C |
| ATOM | 4058 | O | TYR | C | 99 | -21.081 | -32.255 | 84.196 | 1.00 | 36.95 | O |
| ATOM | 4059 | CB | TYR | C | 99 | -22.484 | -33.958 | 82.761 | 1.00 | 35.21 | C |
| ATOM | 4060 | CG | TYR | C | 99 | -22.884 | -32.899 | 81.729 | 1.00 | 37.19 | C |
| ATOM | 4061 | CD1 | TYR | C | 99 | -22.518 | -33.004 | 80.378 | 1.00 | 37.05 | C |
| ATOM | 4062 | CD2 | TYR | C | 99 | -23.629 | -31.784 | 82.114 | 1.00 | 38.73 | C |
| ATOM | 4063 | CE1 | TYR | C | 99 | -22.905 | -32.034 | 79.447 | 1.00 | 36.99 | C |
| ATOM | 4064 | CE2 | TYR | C | 99 | -24.008 | -30.801 | 81.195 | 1.00 | 32.76 | C |
| ATOM | 4065 | CZ | TYR | C | 99 | -23.652 | -30.924 | 79.871 | 1.00 | 40.53 | C |
| ATOM | 4066 | OH | TYR | C | 99 | -24.045 | -29.934 | 78.971 | 1.00 | 41.60 | O |
| ATOM | 4067 | N | GLY | C | 100 | -19.003 | -32.924 | 83.921 | 1.00 | 28.67 | N |
| ATOM | 4068 | CA | GLY | C | 100 | -17.960 | -33.926 | 83.970 | 1.00 | 29.97 | C |
| ATOM | 4069 | C | GLY | C | 100 | -17.825 | -34.458 | 85.387 | 1.00 | 28.43 | C |
| ATOM | 4070 | O | GLY | C | 100 | -17.023 | -35.343 | 85.668 | 1.00 | 35.72 | O |
| ATOM | 4071 | N | ALA | C | 101 | -18.638 | -33.941 | 86.294 | 1.00 | 28.13 | N |
| ATOM | 4072 | CA | ALA | C | 101 | -18.846 | -34.644 | 87.546 | 1.00 | 23.31 | C |
| ATOM | 4073 | C | ALA | C | 101 | -20.080 | -35.531 | 87.385 | 1.00 | 29.38 | C |
| ATOM | 4074 | O | ALA | C | 101 | -20.763 | -35.484 | 86.363 | 1.00 | 31.34 | O |
| ATOM | 4075 | CB | ALA | C | 101 | -19.026 | -33.659 | 88.689 | 1.00 | 26.55 | C |
| ATOM | 4076 | N | PHE | C | 102 | -20.379 | -36.347 | 88.395 | 1.00 | 25.69 | N |
| ATOM | 4077 | CA | PHE | C | 102 | -21.562 | -37.213 | 88.337 | 1.00 | 30.27 | C |
| ATOM | 4078 | C | PHE | C | 102 | -22.754 | -36.480 | 88.948 | 1.00 | 27.72 | C |
| ATOM | 4079 | O | PHE | C | 102 | -22.912 | -36.444 | 90.170 | 1.00 | 25.66 | O |
| ATOM | 4080 | CB | PHE | C | 102 | -21.314 | -38.547 | 89.035 | 1.00 | 28.00 | C |
| ATOM | 4081 | CG | PHE | C | 102 | -20.216 | -39.368 | 88.406 | 1.00 | 28.82 | C |
| ATOM | 4082 | CD1 | PHE | C | 102 | -20.377 | -39.915 | 87.135 | 1.00 | 26.74 | C |
| ATOM | 4083 | CD2 | PHE | C | 102 | -19.002 | -39.556 | 89.069 | 1.00 | 25.45 | C |
| ATOM | 4084 | CE1 | PHE | C | 102 | -19.376 | -40.658 | 86.548 | 1.00 | 23.07 | C |
| ATOM | 4085 | CE2 | PHE | C | 102 | -17.987 | -40.307 | 88.488 | 1.00 | 27.62 | C |
| ATOM | 4086 | CZ | PHE | C | 102 | -18.173 | -40.859 | 87.220 | 1.00 | 24.95 | C |
| ATOM | 4087 | N | ASP | C | 103 | -23.611 | -35.906 | 88.097 | 1.00 | 25.30 | N |
| ATOM | 4088 | CA | ASP | C | 103 | -24.746 | -35.131 | 88.591 | 1.00 | 29.00 | C |
| ATOM | 4089 | C | ASP | C | 103 | -26.021 | -35.943 | 88.801 | 1.00 | 32.32 | C |
| ATOM | 4090 | O | ASP | C | 103 | -26.862 | -35.523 | 89.596 | 1.00 | 36.85 | O |
| ATOM | 4091 | CB | ASP | C | 103 | -25.067 | -33.937 | 87.671 | 1.00 | 26.81 | C |
| ATOM | 4092 | CG | ASP | C | 103 | -25.328 | -34.341 | 86.198 | 1.00 | 41.22 | C |
| ATOM | 4093 | OD1 | ASP | C | 103 | -25.539 | -35.538 | 85.880 | 1.00 | 33.40 | O1- |
| ATOM | 4094 | OD2 | ASP | C | 103 | -25.333 | -33.419 | 85.338 | 1.00 | 51.21 | O |
| ATOM | 4095 | N | ILE | C | 104 | -26.213 | -37.077 | 88.127 | 1.00 | 29.34 | N |
| ATOM | 4096 | CA | ILE | C | 104 | -27.410 | -37.884 | 88.337 | 1.00 | 28.45 | C |
| ATOM | 4097 | C | ILE | C | 104 | -27.007 | -39.332 | 88.536 | 1.00 | 30.33 | C |
| ATOM | 4098 | O | ILE | C | 104 | -26.120 | -39.840 | 87.842 | 1.00 | 31.30 | O |
| ATOM | 4099 | CB | ILE | C | 104 | -28.406 | -37.758 | 87.170 | 1.00 | 31.09 | C |
| ATOM | 4100 | CG1 | ILE | C | 104 | -28.861 | -36.309 | 87.017 | 1.00 | 29.48 | C |
| ATOM | 4101 | CG2 | ILE | C | 104 | -29.583 | -38.689 | 87.387 | 1.00 | 25.98 | C |
| ATOM | 4102 | CD1 | ILE | C | 104 | -29.838 | -36.138 | 85.943 | 1.00 | 31.56 | C |
| ATOM | 4103 | N | TRP | C | 105 | -27.666 | -39.992 | 89.480 | 1.00 | 27.28 | N |
| ATOM | 4104 | CA | TRP | C | 105 | -27.361 | -41.357 | 89.855 | 1.00 | 27.66 | C |
| ATOM | 4105 | C | TRP | C | 105 | -28.599 | -42.236 | 89.754 | 1.00 | 29.36 | C |
| ATOM | 4106 | O | TRP | C | 105 | -29.720 | -41.789 | 90.005 | 1.00 | 31.41 | O |
| ATOM | 4107 | CB | TRP | C | 105 | -26.830 | -41.398 | 91.288 | 1.00 | 28.75 | C |
| ATOM | 4108 | CG | TRP | C | 105 | -25.559 | -40.624 | 91.483 | 1.00 | 27.49 | C |
| ATOM | 4109 | CD1 | TRP | C | 105 | -25.394 | -39.267 | 91.400 | 1.00 | 27.23 | C |
| ATOM | 4110 | CD2 | TRP | C | 105 | -24.276 | -41.167 | 91.802 | 1.00 | 25.31 | C |
| ATOM | 4111 | NE1 | TRP | C | 105 | -24.078 | -38.935 | 91.650 | 1.00 | 25.97 | N |
| ATOM | 4112 | CE2 | TRP | C | 105 | -23.373 | -40.083 | 91.900 | 1.00 | 25.23 | C |
| ATOM | 4113 | CE3 | TRP | C | 105 | -23.801 | -42.466 | 92.016 | 1.00 | 25.04 | C |
| ATOM | 4114 | CZ2 | TRP | C | 105 | -22.026 | -40.259 | 92.188 | 1.00 | 22.01 | C |

| | | | | | | | | | | | |
|---|---|---|---|---|---|---|---|---|---|---|---|
| ATOM | 4115 | CZ3 | TRP | C | 105 | -22.461 | -42.639 | 92.307 | 1.00 | 27.62 | C |
| ATOM | 4116 | CH2 | TRP | C | 105 | -21.586 | -41.532 | 92.389 | 1.00 | 24.95 | C |
| ATOM | 4117 | N | GLY | C | 106 | -28.381 | -43.504 | 89.436 | 1.00 | 29.88 | N |
| ATOM | 4118 | CA | GLY | C | 106 | -29.424 | -44.496 | 89.587 | 1.00 | 34.79 | C |
| ATOM | 4119 | C | GLY | C | 106 | -29.697 | -44.833 | 91.048 | 1.00 | 34.11 | C |
| ATOM | 4120 | O | GLY | C | 106 | -28.952 | -44.456 | 91.951 | 1.00 | 31.75 | O |
| ATOM | 4121 | N | GLN | C | 107 | -30.828 | -45.525 | 91.277 | 1.00 | 37.10 | N |
| ATOM | 4122 | CA | GLN | C | 107 | -31.137 | -46.052 | 92.607 | 1.00 | 33.95 | C |
| ATOM | 4123 | C | GLN | C | 107 | -30.005 | -46.904 | 93.124 | 1.00 | 30.12 | C |
| ATOM | 4124 | O | GLN | C | 107 | -29.773 | -46.956 | 94.331 | 1.00 | 31.44 | O |
| ATOM | 4125 | CB | GLN | C | 107 | -32.411 | -46.921 | 92.621 | 1.00 | 38.95 | C |
| ATOM | 4126 | CG | GLN | C | 107 | -33.442 | -46.732 | 91.485 | 1.00 | 53.38 | C |
| ATOM | 4127 | CD | GLN | C | 107 | -33.137 | -47.557 | 90.207 | 1.00 | 55.26 | C |
| ATOM | 4128 | OE1 | GLN | C | 107 | -33.665 | -48.666 | 89.992 | 1.00 | 51.28 | O |
| ATOM | 4129 | NE2 | GLN | C | 107 | -32.299 | -46.993 | 89.348 | 1.00 | 50.94 | N |
| ATOM | 4130 | N | GLY | C | 108 | -29.311 | -47.597 | 92.233 | 1.00 | 30.68 | N |
| ATOM | 4131 | CA | GLY | C | 108 | -28.376 | -48.619 | 92.628 | 1.00 | 25.20 | C |
| ATOM | 4132 | C | GLY | C | 108 | -29.039 | -49.981 | 92.689 | 1.00 | 28.53 | C |
| ATOM | 4133 | O | GLY | C | 108 | -30.254 | -50.110 | 92.792 | 1.00 | 32.61 | O |
| ATOM | 4134 | N | THR | C | 109 | -28.203 | -51.008 | 92.614 | 1.00 | 26.94 | N |
| ATOM | 4135 | CA | THR | C | 109 | -28.607 | -52.403 | 92.680 | 1.00 | 22.92 | C |
| ATOM | 4136 | C | THR | C | 109 | -27.757 | -53.086 | 93.740 | 1.00 | 30.47 | C |
| ATOM | 4137 | O | THR | C | 109 | -26.523 | -53.049 | 93.674 | 1.00 | 32.40 | O |
| ATOM | 4138 | CB | THR | C | 109 | -28.418 | -53.094 | 91.321 | 1.00 | 29.24 | C |
| ATOM | 4139 | OG1 | THR | C | 109 | -29.226 | -52.453 | 90.327 | 1.00 | 29.50 | O |
| ATOM | 4140 | CG2 | THR | C | 109 | -28.754 | -54.591 | 91.395 | 1.00 | 25.49 | C |
| ATOM | 4141 | N | MET | C | 110 | -28.402 | -53.685 | 94.726 | 1.00 | 30.19 | N |
| ATOM | 4142 | CA | MET | C | 110 | -27.674 | -54.400 | 95.765 | 1.00 | 31.87 | C |
| ATOM | 4143 | C | MET | C | 110 | -27.271 | -55.749 | 95.187 | 1.00 | 30.25 | C |
| ATOM | 4144 | O | MET | C | 110 | -28.133 | -56.548 | 94.818 | 1.00 | 31.68 | O |
| ATOM | 4145 | CB | MET | C | 110 | -28.571 | -54.546 | 96.991 | 1.00 | 38.09 | C |
| ATOM | 4146 | CG | MET | C | 110 | -27.925 | -54.739 | 98.362 | 1.00 | 38.26 | C |
| ATOM | 4147 | SD | MET | C | 110 | -29.296 | -54.935 | 99.598 | 1.00 | 60.00 | S |
| ATOM | 4148 | CE | MET | C | 110 | -30.587 | -53.829 | 98.963 | 1.00 | 28.36 | C |
| ATOM | 4149 | N | VAL | C | 111 | -25.971 | -55.986 | 95.049 | 1.00 | 30.68 | N |
| ATOM | 4150 | CA | VAL | C | 111 | -25.467 | -57.191 | 94.399 | 1.00 | 30.46 | C |
| ATOM | 4151 | C | VAL | C | 111 | -24.685 | -57.989 | 95.422 | 1.00 | 30.94 | C |
| ATOM | 4152 | O | VAL | C | 111 | -23.698 | -57.495 | 95.978 | 1.00 | 34.81 | O |
| ATOM | 4153 | CB | VAL | C | 111 | -24.601 | -56.875 | 93.164 | 1.00 | 34.32 | C |
| ATOM | 4154 | CG1 | VAL | C | 111 | -23.821 | -58.100 | 92.745 | 1.00 | 28.86 | C |
| ATOM | 4155 | CG2 | VAL | C | 111 | -25.462 | -56.397 | 91.995 | 1.00 | 24.78 | C |
| ATOM | 4156 | N | THR | C | 112 | -25.136 | -59.214 | 95.676 | 1.00 | 33.49 | N |
| ATOM | 4157 | CA | THR | C | 112 | -24.495 | -60.147 | 96.593 | 1.00 | 29.04 | C |
| ATOM | 4158 | C | THR | C | 112 | -23.993 | -61.341 | 95.798 | 1.00 | 31.99 | C |
| ATOM | 4159 | O | THR | C | 112 | -24.749 | -61.926 | 95.013 | 1.00 | 28.19 | O |
| ATOM | 4160 | CB | THR | C | 112 | -25.483 | -60.629 | 97.670 | 1.00 | 27.70 | C |
| ATOM | 4161 | OG1 | THR | C | 112 | -26.126 | -59.513 | 98.275 | 1.00 | 27.51 | O |
| ATOM | 4162 | CG2 | THR | C | 112 | -24.800 | -61.447 | 98.736 | 1.00 | 30.55 | C |
| ATOM | 4163 | N | VAL | C | 113 | -22.726 | -61.696 | 95.985 | 1.00 | 29.97 | N |
| ATOM | 4164 | CA | VAL | C | 113 | -22.185 | -62.918 | 95.405 | 1.00 | 36.12 | C |
| ATOM | 4165 | C | VAL | C | 113 | -21.678 | -63.780 | 96.550 | 1.00 | 33.41 | C |
| ATOM | 4166 | O | VAL | C | 113 | -20.902 | -63.312 | 97.392 | 1.00 | 31.40 | O |
| ATOM | 4167 | CB | VAL | C | 113 | -21.119 | -62.648 | 94.319 | 1.00 | 37.87 | C |
| ATOM | 4168 | CG1 | VAL | C | 113 | -20.582 | -61.246 | 94.423 | 1.00 | 37.11 | C |
| ATOM | 4169 | CG2 | VAL | C | 113 | -20.016 | -63.719 | 94.288 | 1.00 | 28.90 | C |
| ATOM | 4170 | N | SER | C | 114 | -22.171 | -65.019 | 96.610 | 1.00 | 33.34 | N |
| ATOM | 4171 | CA | SER | C | 114 | -21.991 | -65.863 | 97.781 | 1.00 | 37.36 | C |
| ATOM | 4172 | C | SER | C | 114 | -22.309 | -67.316 | 97.449 | 1.00 | 34.25 | C |
| ATOM | 4173 | O | SER | C | 114 | -23.113 | -67.606 | 96.563 | 1.00 | 32.33 | O |
| ATOM | 4174 | CB | SER | C | 114 | -22.886 | -65.382 | 98.932 | 1.00 | 34.37 | C |
| ATOM | 4175 | OG | SER | C | 114 | -22.928 | -66.326 | 99.983 | 1.00 | 36.36 | O |
| ATOM | 4176 | N | SER | C | 115 | -21.682 | -68.224 | 98.195 | 1.00 | 38.98 | N |
| ATOM | 4177 | CA | SER | C | 115 | -22.065 | -69.629 | 98.129 | 1.00 | 38.55 | C |
| ATOM | 4178 | C | SER | C | 115 | -23.430 | -69.890 | 98.753 | 1.00 | 44.22 | C |
| ATOM | 4179 | O | SER | C | 115 | -24.069 | -70.889 | 98.410 | 1.00 | 48.62 | O |
| ATOM | 4180 | CB | SER | C | 115 | -21.032 | -70.491 | 98.847 | 1.00 | 40.98 | C |
| ATOM | 4181 | OG | SER | C | 115 | -19.794 | -70.465 | 98.158 | 1.00 | 57.58 | O |
| ATOM | 4182 | N | ALA | C | 116 | -23.912 | -69.003 | 99.620 | 1.00 | 34.12 | N |
| ATOM | 4183 | CA | ALA | C | 116 | -25.140 | -69.269 | 100.348 | 1.00 | 37.60 | C |
| ATOM | 4184 | C | ALA | C | 116 | -26.347 | -69.330 | 99.415 | 1.00 | 39.26 | C |
| ATOM | 4185 | O | ALA | C | 116 | -26.329 | -68.827 | 98.282 | 1.00 | 36.17 | O |

| | | | | | | | | | | | |
|---|---|---|---|---|---|---|---|---|---|---|---|
| ATOM | 4186 | CB | ALA | C | 116 | -25.366 | -68.194 | 101.413 | 1.00 | 33.12 | C |
| ATOM | 4187 | N | SER | C | 117 | -27.414 | -69.957 | 99.915 | 1.00 | 36.37 | N |
| ATOM | 4188 | CA | SER | C | 117 | -28.684 | -70.042 | 99.206 | 1.00 | 39.66 | C |
| ATOM | 4189 | C | SER | C | 117 | -29.745 | -69.252 | 99.960 | 1.00 | 36.99 | C |
| ATOM | 4190 | O | SER | C | 117 | -29.660 | -69.069 | 101.179 | 1.00 | 40.67 | O |
| ATOM | 4191 | CB | SER | C | 117 | -29.137 | -71.504 | 99.021 | 1.00 | 40.84 | C |
| ATOM | 4192 | OG | SER | C | 117 | -28.239 | -72.218 | 98.172 | 1.00 | 42.49 | O |
| ATOM | 4193 | N | THR | C | 118 | -30.735 | -68.770 | 99.215 | 1.00 | 35.22 | N |
| ATOM | 4194 | CA | THR | C | 118 | -31.796 | -67.963 | 99.796 | 1.00 | 34.00 | C |
| ATOM | 4195 | C | THR | C | 118 | -32.453 | -68.705 | 100.953 | 1.00 | 37.86 | C |
| ATOM | 4196 | O | THR | C | 118 | -32.680 | -69.912 | 100.889 | 1.00 | 37.72 | O |
| ATOM | 4197 | CB | THR | C | 118 | -32.829 | -67.600 | 98.724 | 1.00 | 27.84 | C |
| ATOM | 4198 | OG1 | THR | C | 118 | -32.225 | -66.729 | 97.771 | 1.00 | 31.08 | O |
| ATOM | 4199 | CG2 | THR | C | 118 | -34.051 | -66.887 | 99.332 | 1.00 | 36.20 | C |
| ATOM | 4200 | N | LYS | C | 119 | -32.740 | -67.973 | 102.024 | 1.00 | 38.37 | N |
| ATOM | 4201 | CA | LYS | C | 119 | -33.239 | -68.569 | 103.256 | 1.00 | 37.26 | C |
| ATOM | 4202 | C | LYS | C | 119 | -33.922 | -67.486 | 104.079 | 1.00 | 34.75 | C |
| ATOM | 4203 | O | LYS | C | 119 | -33.332 | -66.432 | 104.331 | 1.00 | 32.89 | O |
| ATOM | 4204 | CB | LYS | C | 119 | -32.093 | -69.201 | 104.040 | 1.00 | 32.21 | C |
| ATOM | 4205 | CG | LYS | C | 119 | -32.520 | -69.868 | 105.295 | 1.00 | 32.82 | C |
| ATOM | 4206 | CD | LYS | C | 119 | -31.299 | -70.193 | 106.108 | 1.00 | 34.06 | C |
| ATOM | 4207 | CE | LYS | C | 119 | -31.675 | -70.872 | 107.411 | 1.00 | 37.24 | C |
| ATOM | 4208 | NZ | LYS | C | 119 | -32.670 | -70.086 | 108.181 | 1.00 | 41.53 | N1+ |
| ATOM | 4209 | N | GLY | C | 120 | -35.166 | -67.738 | 104.474 | 1.00 | 34.46 | N |
| ATOM | 4210 | CA | GLY | C | 120 | -35.906 | -66.796 | 105.275 | 1.00 | 30.62 | C |
| ATOM | 4211 | C | GLY | C | 120 | -35.395 | -66.829 | 106.696 | 1.00 | 30.67 | C |
| ATOM | 4212 | O | GLY | C | 120 | -34.703 | -67.767 | 107.102 | 1.00 | 29.23 | O |
| ATOM | 4213 | N | PRO | C | 121 | -35.705 | -65.792 | 107.467 | 1.00 | 28.30 | N |
| ATOM | 4214 | CA | PRO | C | 121 | -35.198 | -65.683 | 108.838 | 1.00 | 30.58 | C |
| ATOM | 4215 | C | PRO | C | 121 | -36.084 | -66.380 | 109.863 | 1.00 | 30.35 | C |
| ATOM | 4216 | O | PRO | C | 121 | -37.286 | -66.554 | 109.668 | 1.00 | 31.40 | O |
| ATOM | 4217 | CB | PRO | C | 121 | -35.223 | -64.172 | 109.082 | 1.00 | 29.17 | C |
| ATOM | 4218 | CG | PRO | C | 121 | -36.377 | -63.701 | 108.245 | 1.00 | 27.47 | C |
| ATOM | 4219 | CD | PRO | C | 121 | -36.411 | -64.578 | 107.029 | 1.00 | 27.41 | C |
| ATOM | 4220 | N | SER | C | 122 | -35.465 | -66.726 | 110.991 | 1.00 | 27.52 | N |
| ATOM | 4221 | CA | SER | C | 122 | -36.176 | -67.066 | 112.219 | 1.00 | 26.31 | C |
| ATOM | 4222 | C | SER | C | 122 | -36.157 | -65.874 | 113.165 | 1.00 | 30.19 | C |
| ATOM | 4223 | O | SER | C | 122 | -35.108 | -65.263 | 113.389 | 1.00 | 33.03 | O |
| ATOM | 4224 | CB | SER | C | 122 | -35.553 | -68.272 | 112.920 | 1.00 | 33.10 | C |
| ATOM | 4225 | OG | SER | C | 122 | -35.607 | -69.425 | 112.115 | 1.00 | 42.58 | O |
| ATOM | 4226 | N | VAL | C | 123 | -37.305 | -65.555 | 113.736 | 1.00 | 30.40 | N |
| ATOM | 4227 | CA | VAL | C | 123 | -37.439 | -64.392 | 114.599 | 1.00 | 29.30 | C |
| ATOM | 4228 | C | VAL | C | 123 | -37.651 | -64.870 | 116.038 | 1.00 | 28.97 | C |
| ATOM | 4229 | O | VAL | C | 123 | -38.681 | -65.469 | 116.368 | 1.00 | 37.16 | O |
| ATOM | 4230 | CB | VAL | C | 123 | -38.576 | -63.481 | 114.123 | 1.00 | 27.97 | C |
| ATOM | 4231 | CG1 | VAL | C | 123 | -38.600 | -62.206 | 114.971 | 1.00 | 29.32 | C |
| ATOM | 4232 | CG2 | VAL | C | 123 | -38.448 | -63.196 | 112.593 | 1.00 | 23.94 | C |
| ATOM | 4233 | N | PHE | C | 124 | -36.703 | -64.598 | 116.890 | 1.00 | 27.59 | N |
| ATOM | 4234 | CA | PHE | C | 124 | -36.791 | -64.961 | 118.289 | 1.00 | 30.52 | C |
| ATOM | 4235 | C | PHE | C | 124 | -36.968 | -63.708 | 119.143 | 1.00 | 32.03 | C |
| ATOM | 4236 | O | PHE | C | 124 | -36.508 | -62.623 | 118.771 | 1.00 | 29.31 | O |
| ATOM | 4237 | CB | PHE | C | 124 | -35.539 | -65.723 | 118.760 | 1.00 | 34.19 | C |
| ATOM | 4238 | CG | PHE | C | 124 | -35.145 | -66.879 | 117.868 | 1.00 | 33.33 | C |
| ATOM | 4239 | CD1 | PHE | C | 124 | -35.968 | -67.985 | 117.730 | 1.00 | 37.57 | C |
| ATOM | 4240 | CD2 | PHE | C | 124 | -33.956 | -66.851 | 117.163 | 1.00 | 31.04 | C |
| ATOM | 4241 | CE1 | PHE | C | 124 | -35.618 | -69.033 | 116.892 | 1.00 | 34.49 | C |
| ATOM | 4242 | CE2 | PHE | C | 124 | -33.596 | -67.901 | 116.333 | 1.00 | 34.61 | C |
| ATOM | 4243 | CZ | PHE | C | 124 | -34.430 | -68.991 | 116.196 | 1.00 | 33.73 | C |
| ATOM | 4244 | N | PRO | C | 125 | -37.636 | -63.815 | 120.285 | 1.00 | 34.51 | N |
| ATOM | 4245 | CA | PRO | C | 125 | -37.839 | -62.633 | 121.124 | 1.00 | 30.32 | C |
| ATOM | 4246 | C | PRO | C | 125 | -36.613 | -62.280 | 121.951 | 1.00 | 32.51 | C |
| ATOM | 4247 | O | PRO | C | 125 | -35.832 | -63.136 | 122.369 | 1.00 | 36.65 | O |
| ATOM | 4248 | CB | PRO | C | 125 | -39.010 | -63.039 | 122.025 | 1.00 | 29.69 | C |
| ATOM | 4249 | CG | PRO | C | 125 | -38.894 | -64.526 | 122.107 | 1.00 | 32.87 | C |
| ATOM | 4250 | CD | PRO | C | 125 | -38.363 | -64.991 | 120.794 | 1.00 | 34.01 | C |
| ATOM | 4251 | N | LEU | C | 126 | -36.434 | -60.985 | 122.134 | 1.00 | 26.85 | N |
| ATOM | 4252 | CA | LEU | C | 126 | -35.595 | -60.422 | 123.175 | 1.00 | 26.86 | C |
| ATOM | 4253 | C | LEU | C | 126 | -36.568 | -59.902 | 124.234 | 1.00 | 31.33 | C |
| ATOM | 4254 | O | LEU | C | 126 | -37.153 | -58.825 | 124.081 | 1.00 | 27.81 | O |
| ATOM | 4255 | CB | LEU | C | 126 | -34.714 | -59.324 | 122.596 | 1.00 | 31.15 | C |
| ATOM | 4256 | CG | LEU | C | 126 | -33.839 | -59.786 | 121.435 | 1.00 | 29.11 | C |

| | | | | | | | | | | | |
|---|---|---|---|---|---|---|---|---|---|---|---|
| ATOM | 4257 | CD1 | LEU | C | 126 | -33.179 | -58.601 | 120.808 | 1.00 | 26.98 | C |
| ATOM | 4258 | CD2 | LEU | C | 126 | -32.786 | -60.749 | 121.950 | 1.00 | 28.83 | C |
| ATOM | 4259 | N | ALA | C | 127 | -36.784 | -60.706 | 125.302 | 1.00 | 34.13 | N |
| ATOM | 4260 | CA | ALA | C | 127 | -37.874 | -60.477 | 126.253 | 1.00 | 34.68 | C |
| ATOM | 4261 | C | ALA | C | 127 | -37.515 | -59.383 | 127.258 | 1.00 | 38.36 | C |
| ATOM | 4262 | O | ALA | C | 127 | -36.400 | -59.366 | 127.792 | 1.00 | 34.45 | O |
| ATOM | 4263 | CB | ALA | C | 127 | -38.229 | -61.756 | 126.999 | 1.00 | 34.71 | C |
| ATOM | 4264 | N | PRO | C | 128 | -38.453 | -58.480 | 127.552 | 1.00 | 43.79 | N |
| ATOM | 4265 | CA | PRO | C | 128 | -38.163 | -57.410 | 128.513 | 1.00 | 43.68 | C |
| ATOM | 4266 | C | PRO | C | 128 | -37.732 | -57.972 | 129.856 | 1.00 | 54.93 | C |
| ATOM | 4267 | O | PRO | C | 128 | -38.285 | -58.961 | 130.350 | 1.00 | 50.70 | O |
| ATOM | 4268 | CB | PRO | C | 128 | -39.481 | -56.625 | 128.600 | 1.00 | 40.46 | C |
| ATOM | 4269 | CG | PRO | C | 128 | -40.513 | -57.526 | 128.054 | 1.00 | 44.62 | C |
| ATOM | 4270 | CD | PRO | C | 128 | -39.823 | -58.379 | 127.020 | 1.00 | 37.96 | C |
| ATOM | 4271 | N | SER | C | 129 | -36.668 | -57.356 | 130.389 | 1.00 | 69.23 | N |
| ATOM | 4272 | CA | SER | C | 129 | -36.026 | -57.680 | 131.657 | 1.00 | 73.37 | C |
| ATOM | 4273 | C | SER | C | 129 | -37.065 | -57.798 | 132.767 | 1.00 | 80.23 | C |
| ATOM | 4274 | O | SER | C | 129 | -37.700 | -56.804 | 133.139 | 1.00 | 81.02 | O |
| ATOM | 4275 | CB | SER | C | 129 | -34.983 | -56.595 | 131.987 | 1.00 | 71.73 | C |
| ATOM | 4276 | OG | SER | C | 129 | -34.000 | -57.018 | 132.909 | 1.00 | 63.19 | O |
| ATOM | 4277 | N | SER | C | 130 | -37.246 | -59.018 | 133.289 | 1.00 | 83.03 | N |
| ATOM | 4278 | CA | SER | C | 130 | -38.109 | -59.226 | 134.446 | 1.00 | 85.79 | C |
| ATOM | 4279 | C | SER | C | 130 | -37.714 | -58.318 | 135.603 | 1.00 | 95.78 | C |
| ATOM | 4280 | O | SER | C | 130 | -38.566 | -57.941 | 136.424 | 1.00 | 93.56 | O |
| ATOM | 4281 | CB | SER | C | 130 | -38.040 | -60.696 | 134.882 | 1.00 | 88.42 | C |
| ATOM | 4282 | OG | SER | C | 130 | -36.688 | -61.134 | 135.021 | 1.00 | 84.37 | O |
| ATOM | 4283 | N | LYS | C | 131 | -36.428 | -57.953 | 135.673 | 1.00 | 96.01 | N |
| ATOM | 4284 | CA | LYS | C | 131 | -35.860 | -57.111 | 136.721 | 1.00 | 92.69 | C |
| ATOM | 4285 | C | LYS | C | 131 | -35.737 | -55.631 | 136.334 | 1.00 | 88.64 | C |
| ATOM | 4286 | O | LYS | C | 131 | -34.824 | -54.957 | 136.820 | 1.00 | 93.47 | O |
| ATOM | 4287 | CB | LYS | C | 131 | -34.490 | -57.660 | 137.135 | 1.00 | 85.98 | C |
| ATOM | 4288 | CG | LYS | C | 131 | -34.512 | -59.018 | 137.841 | 1.00 | 83.85 | C |
| ATOM | 4289 | CD | LYS | C | 131 | -33.113 | -59.398 | 138.328 | 1.00 | 86.44 | C |
| ATOM | 4290 | CE | LYS | C | 131 | -33.162 | -60.359 | 139.509 | 1.00 | 81.44 | C |
| ATOM | 4291 | NZ | LYS | C | 131 | -31.802 | -60.691 | 140.021 | 1.00 | 67.72 | N1+ |
| ATOM | 4292 | N | SER | C | 132 | -36.625 | -55.088 | 135.498 | 1.00 | 91.44 | N |
| ATOM | 4293 | CA | SER | C | 132 | -36.567 | -53.651 | 135.224 | 1.00 | 88.79 | C |
| ATOM | 4294 | C | SER | C | 132 | -36.967 | -52.857 | 136.461 | 1.00 | 93.64 | C |
| ATOM | 4295 | O | SER | C | 132 | -37.915 | -53.218 | 137.168 | 1.00 | 97.31 | O |
| ATOM | 4296 | CB | SER | C | 132 | -37.472 | -53.256 | 134.048 | 1.00 | 75.72 | C |
| ATOM | 4297 | OG | SER | C | 132 | -36.862 | -53.513 | 132.797 | 1.00 | 71.83 | O |
| ATOM | 4298 | N | THR | C | 133 | -36.229 | -51.769 | 136.723 | 1.00 | 99.77 | N |
| ATOM | 4299 | CA | THR | C | 133 | -36.569 | -50.845 | 137.806 | 1.00 | 97.90 | C |
| ATOM | 4300 | C | THR | C | 133 | -38.009 | -50.364 | 137.611 | 1.00 | 93.15 | C |
| ATOM | 4301 | O | THR | C | 133 | -38.291 | -49.593 | 136.684 | 1.00 | 89.44 | O |
| ATOM | 4302 | CB | THR | C | 133 | -35.563 | -49.669 | 137.882 | 1.00 | 87.55 | C |
| ATOM | 4303 | OG1 | THR | C | 133 | -35.179 | -49.234 | 136.565 | 1.00 | 83.45 | O |
| ATOM | 4304 | CG2 | THR | C | 133 | -34.303 | -50.077 | 138.653 | 1.00 | 84.85 | C |
| ATOM | 4305 | N | SER | C | 134 | -38.929 | -50.829 | 138.466 | 1.00 | 93.26 | N |
| ATOM | 4306 | CA | SER | C | 134 | -40.352 | -50.635 | 138.210 | 1.00 | 87.74 | C |
| ATOM | 4307 | C | SER | C | 134 | -40.679 | -49.153 | 138.274 | 1.00 | 85.43 | C |
| ATOM | 4308 | O | SER | C | 134 | -40.329 | -48.472 | 139.242 | 1.00 | 87.73 | O |
| ATOM | 4309 | CB | SER | C | 134 | -41.195 | -51.410 | 139.224 | 1.00 | 74.42 | C |
| ATOM | 4310 | OG | SER | C | 134 | -42.211 | -52.150 | 138.567 | 1.00 | 81.41 | O |
| ATOM | 4311 | N | GLY | C | 135 | -41.314 | -48.643 | 137.223 | 1.00 | 80.04 | N |
| ATOM | 4312 | CA | GLY | C | 135 | -41.395 | -47.211 | 137.051 | 1.00 | 78.15 | C |
| ATOM | 4313 | C | GLY | C | 135 | -40.279 | -46.614 | 136.227 | 1.00 | 80.05 | C |
| ATOM | 4314 | O | GLY | C | 135 | -40.186 | -45.381 | 136.144 | 1.00 | 77.05 | O |
| ATOM | 4315 | N | GLY | C | 136 | -39.414 | -47.444 | 135.639 | 1.00 | 72.87 | N |
| ATOM | 4316 | CA | GLY | C | 136 | -38.350 | -46.962 | 134.783 | 1.00 | 61.44 | C |
| ATOM | 4317 | C | GLY | C | 136 | -38.489 | -47.391 | 133.333 | 1.00 | 56.58 | C |
| ATOM | 4318 | O | GLY | C | 136 | -39.592 | -47.407 | 132.771 | 1.00 | 51.45 | O |
| ATOM | 4319 | N | THR | C | 137 | -37.382 | -47.773 | 132.713 | 1.00 | 53.14 | N |
| ATOM | 4320 | CA | THR | C | 137 | -37.361 | -48.017 | 131.279 | 1.00 | 47.91 | C |
| ATOM | 4321 | C | THR | C | 137 | -37.015 | -49.473 | 131.008 | 1.00 | 48.48 | C |
| ATOM | 4322 | O | THR | C | 137 | -36.090 | -50.028 | 131.617 | 1.00 | 47.97 | O |
| ATOM | 4323 | CB | THR | C | 137 | -36.370 | -47.081 | 130.592 | 1.00 | 48.09 | C |
| ATOM | 4324 | OG1 | THR | C | 137 | -36.886 | -45.745 | 130.650 | 1.00 | 54.06 | O |
| ATOM | 4325 | CG2 | THR | C | 137 | -36.140 | -47.485 | 129.123 | 1.00 | 43.01 | C |
| ATOM | 4326 | N | ALA | C | 138 | -37.781 | -50.087 | 130.110 | 1.00 | 38.89 | N |
| ATOM | 4327 | CA | ALA | C | 138 | -37.598 | -51.469 | 129.714 | 1.00 | 38.16 | C |

| | | | | | | | | | | | |
|---|---|---|---|---|---|---|---|---|---|---|---|
| ATOM | 4328 | C | ALA | C | 138 | -37.117 | -51.516 | 128.268 | 1.00 | 36.59 | C |
| ATOM | 4329 | O | ALA | C | 138 | -37.541 | -50.708 | 127.437 | 1.00 | 36.87 | O |
| ATOM | 4330 | CB | ALA | C | 138 | -38.908 | -52.249 | 129.871 | 1.00 | 31.25 | C |
| ATOM | 4331 | N | ALA | C | 139 | -36.232 | -52.454 | 127.964 | 1.00 | 33.10 | N |
| ATOM | 4332 | CA | ALA | C | 139 | -35.886 | -52.755 | 126.582 | 1.00 | 30.90 | C |
| ATOM | 4333 | C | ALA | C | 139 | -36.522 | -54.080 | 126.199 | 1.00 | 30.77 | C |
| ATOM | 4334 | O | ALA | C | 139 | -36.619 | -54.986 | 127.028 | 1.00 | 31.77 | O |
| ATOM | 4335 | CB | ALA | C | 139 | -34.371 | -52.823 | 126.376 | 1.00 | 25.95 | C |
| ATOM | 4336 | N | LEU | C | 140 | -36.978 | -54.176 | 124.952 | 1.00 | 29.69 | N |
| ATOM | 4337 | CA | LEU | C | 140 | -37.426 | -55.439 | 124.376 | 1.00 | 30.12 | C |
| ATOM | 4338 | C | LEU | C | 140 | -37.081 | -55.420 | 122.891 | 1.00 | 30.17 | C |
| ATOM | 4339 | O | LEU | C | 140 | -36.795 | -54.369 | 122.319 | 1.00 | 29.86 | O |
| ATOM | 4340 | CB | LEU | C | 140 | -38.930 | -55.666 | 124.605 | 1.00 | 29.80 | C |
| ATOM | 4341 | CG | LEU | C | 140 | -39.844 | -54.591 | 124.028 | 1.00 | 30.15 | C |
| ATOM | 4342 | CD1 | LEU | C | 140 | -40.427 | -55.049 | 122.720 | 1.00 | 29.13 | C |
| ATOM | 4343 | CD2 | LEU | C | 140 | -40.945 | -54.256 | 125.007 | 1.00 | 30.43 | C |
| ATOM | 4344 | N | GLY | C | 141 | -37.135 | -56.581 | 122.250 | 1.00 | 27.97 | N |
| ATOM | 4345 | CA | GLY | C | 141 | -36.809 | -56.584 | 120.844 | 1.00 | 27.01 | C |
| ATOM | 4346 | C | GLY | C | 141 | -37.043 | -57.919 | 120.174 | 1.00 | 30.67 | C |
| ATOM | 4347 | O | GLY | C | 141 | -37.628 | -58.835 | 120.754 | 1.00 | 31.90 | O |
| ATOM | 4348 | N | CYS | C | 142 | -36.604 | -57.978 | 118.908 | 1.00 | 28.16 | N |
| ATOM | 4349 | CA | CYS | C | 142 | -36.633 | -59.156 | 118.051 | 1.00 | 31.74 | C |
| ATOM | 4350 | C | CYS | C | 142 | -35.238 | -59.467 | 117.527 | 1.00 | 28.43 | C |
| ATOM | 4351 | O | CYS | C | 142 | -34.478 | -58.563 | 117.193 | 1.00 | 24.09 | O |
| ATOM | 4352 | CB | CYS | C | 142 | -37.563 | -58.947 | 116.858 | 1.00 | 32.06 | C |
| ATOM | 4353 | SG | CYS | C | 142 | -39.293 | -59.136 | 117.287 | 1.00 | 49.90 | S |
| ATOM | 4354 | N | LEU | C | 143 | -34.903 | -60.747 | 117.447 | 1.00 | 28.84 | N |
| ATOM | 4355 | CA | LEU | C | 143 | -33.651 | -61.187 | 116.845 | 1.00 | 26.55 | C |
| ATOM | 4356 | C | LEU | C | 143 | -33.989 | -61.904 | 115.550 | 1.00 | 27.72 | C |
| ATOM | 4357 | O | LEU | C | 143 | -34.719 | -62.892 | 115.562 | 1.00 | 29.16 | O |
| ATOM | 4358 | CB | LEU | C | 143 | -32.849 | -62.069 | 117.805 | 1.00 | 29.85 | C |
| ATOM | 4359 | CG | LEU | C | 143 | -31.504 | -62.623 | 117.312 | 1.00 | 31.81 | C |
| ATOM | 4360 | CD1 | LEU | C | 143 | -30.634 | -61.539 | 116.821 | 1.00 | 29.50 | C |
| ATOM | 4361 | CD2 | LEU | C | 143 | -30.809 | -63.273 | 118.476 | 1.00 | 34.71 | C |
| ATOM | 4362 | N | VAL | C | 144 | -33.506 | -61.374 | 114.434 | 1.00 | 24.21 | N |
| ATOM | 4363 | CA | VAL | C | 144 | -33.850 | -61.888 | 113.121 | 1.00 | 25.88 | C |
| ATOM | 4364 | C | VAL | C | 144 | -32.632 | -62.683 | 112.650 | 1.00 | 31.73 | C |
| ATOM | 4365 | O | VAL | C | 144 | -31.676 | -62.122 | 112.104 | 1.00 | 27.35 | O |
| ATOM | 4366 | CB | VAL | C | 144 | -34.237 | -60.756 | 112.162 | 1.00 | 29.39 | C |
| ATOM | 4367 | CG1 | VAL | C | 144 | -34.620 | -61.297 | 110.801 | 1.00 | 29.56 | C |
| ATOM | 4368 | CG2 | VAL | C | 144 | -35.395 | -59.939 | 112.721 | 1.00 | 21.42 | C |
| ATOM | 4369 | N | LYS | C | 145 | -32.658 | -64.003 | 112.849 | 1.00 | 26.44 | N |
| ATOM | 4370 | CA | LYS | C | 145 | -31.455 | -64.811 | 112.726 | 1.00 | 29.06 | C |
| ATOM | 4371 | C | LYS | C | 145 | -31.472 | -65.687 | 111.476 | 1.00 | 30.53 | C |
| ATOM | 4372 | O | LYS | C | 145 | -32.513 | -66.244 | 111.098 | 1.00 | 29.40 | O |
| ATOM | 4373 | CB | LYS | C | 145 | -31.253 | -65.686 | 113.974 | 1.00 | 29.44 | C |
| ATOM | 4374 | CG | LYS | C | 145 | -29.832 | -66.217 | 114.070 | 1.00 | 30.84 | C |
| ATOM | 4375 | CD | LYS | C | 145 | -29.511 | -66.818 | 115.405 | 1.00 | 37.83 | C |
| ATOM | 4376 | CE | LYS | C | 145 | -28.009 | -67.086 | 115.543 | 1.00 | 43.25 | C |
| ATOM | 4377 | NZ | LYS | C | 145 | -27.433 | -67.955 | 114.471 | 1.00 | 39.52 | N1+ |
| ATOM | 4378 | N | ASP | C | 146 | -30.298 | -65.782 | 110.837 | 1.00 | 28.14 | N |
| ATOM | 4379 | CA | ASP | C | 146 | -30.003 | -66.758 | 109.784 | 1.00 | 33.06 | C |
| ATOM | 4380 | C | ASP | C | 146 | -30.862 | -66.564 | 108.531 | 1.00 | 33.58 | C |
| ATOM | 4381 | O | ASP | C | 146 | -31.584 | -67.465 | 108.102 | 1.00 | 36.23 | O |
| ATOM | 4382 | CB | ASP | C | 146 | -30.185 | -68.177 | 110.330 | 1.00 | 36.19 | C |
| ATOM | 4383 | CG | ASP | C | 146 | -29.226 | -68.502 | 111.441 | 1.00 | 38.83 | C |
| ATOM | 4384 | OD1 | ASP | C | 146 | -28.041 | -68.058 | 111.404 | 1.00 | 37.01 | O |
| ATOM | 4385 | OD2 | ASP | C | 146 | -29.716 | -69.135 | 112.405 | 1.00 | 41.05 | O1- |
| ATOM | 4386 | N | TYR | C | 147 | -30.735 | -65.405 | 107.905 | 1.00 | 29.04 | N |
| ATOM | 4387 | CA | TYR | C | 147 | -31.400 | -65.202 | 106.626 | 1.00 | 29.40 | C |
| ATOM | 4388 | C | TYR | C | 147 | -30.361 | -64.899 | 105.547 | 1.00 | 29.62 | C |
| ATOM | 4389 | O | TYR | C | 147 | -29.186 | -64.645 | 105.826 | 1.00 | 30.41 | O |
| ATOM | 4390 | CB | TYR | C | 147 | -32.443 | -64.080 | 106.711 | 1.00 | 26.97 | C |
| ATOM | 4391 | CG | TYR | C | 147 | -31.863 | -62.715 | 106.999 | 1.00 | 28.14 | C |
| ATOM | 4392 | CD1 | TYR | C | 147 | -31.628 | -62.304 | 108.303 | 1.00 | 28.44 | C |
| ATOM | 4393 | CD2 | TYR | C | 147 | -31.529 | -61.840 | 105.963 | 1.00 | 29.70 | C |
| ATOM | 4394 | CE1 | TYR | C | 147 | -31.098 | -61.071 | 108.575 | 1.00 | 30.16 | C |
| ATOM | 4395 | CE2 | TYR | C | 147 | -30.994 | -60.586 | 106.226 | 1.00 | 29.35 | C |
| ATOM | 4396 | CZ | TYR | C | 147 | -30.788 | -60.204 | 107.538 | 1.00 | 32.07 | C |
| ATOM | 4397 | OH | TYR | C | 147 | -30.269 | -58.954 | 107.832 | 1.00 | 32.73 | O |
| ATOM | 4398 | N | PHE | C | 148 | -30.808 | -64.941 | 104.298 | 1.00 | 31.56 | N |

| | | | | | | | | | | | |
|---|---|---|---|---|---|---|---|---|---|---|---|
| ATOM | 4399 | CA | PHE | C | 148 | -29.950 | -64.658 | 103.161 | 1.00 | 31.89 | C |
| ATOM | 4400 | C | PHE | C | 148 | -30.781 | -64.535 | 101.900 | 1.00 | 31.76 | C |
| ATOM | 4401 | O | PHE | C | 148 | -31.634 | -65.367 | 101.661 | 1.00 | 30.72 | O |
| ATOM | 4402 | CB | PHE | C | 148 | -28.910 | -65.765 | 102.977 | 1.00 | 30.49 | C |
| ATOM | 4403 | CG | PHE | C | 148 | -27.980 | -65.524 | 101.839 | 1.00 | 32.65 | C |
| ATOM | 4404 | CD1 | PHE | C | 148 | -28.316 | -65.923 | 100.550 | 1.00 | 30.37 | C |
| ATOM | 4405 | CD2 | PHE | C | 148 | -26.766 | -64.876 | 102.053 | 1.00 | 31.85 | C |
| ATOM | 4406 | CE1 | PHE | C | 148 | -27.460 | -65.681 | 99.502 | 1.00 | 33.92 | C |
| ATOM | 4407 | CE2 | PHE | C | 148 | -25.903 | -64.629 | 101.010 | 1.00 | 31.05 | C |
| ATOM | 4408 | CZ | PHE | C | 148 | -26.244 | -65.032 | 99.734 | 1.00 | 36.04 | C |
| ATOM | 4409 | N | PRO | C | 149 | -30.503 | -63.514 | 101.066 | 1.00 | 37.13 | N |
| ATOM | 4410 | CA | PRO | C | 149 | -29.514 | -62.453 | 101.301 | 1.00 | 34.36 | C |
| ATOM | 4411 | C | PRO | C | 149 | -30.136 | -61.270 | 102.025 | 1.00 | 31.13 | C |
| ATOM | 4412 | O | PRO | C | 149 | -31.294 | -61.362 | 102.414 | 1.00 | 33.18 | O |
| ATOM | 4413 | CB | PRO | C | 149 | -29.116 | -62.050 | 99.885 | 1.00 | 28.91 | C |
| ATOM | 4414 | CG | PRO | C | 149 | -30.384 | -62.167 | 99.146 | 1.00 | 26.99 | C |
| ATOM | 4415 | CD | PRO | C | 149 | -31.093 | -63.390 | 99.717 | 1.00 | 27.60 | C |
| ATOM | 4416 | N | GLU | C | 150 | -29.394 | -60.176 | 102.157 | 1.00 | 27.31 | N |
| ATOM | 4417 | CA | GLU | C | 150 | -29.961 | -58.888 | 102.552 | 1.00 | 30.44 | C |
| ATOM | 4418 | C | GLU | C | 150 | -30.938 | -58.398 | 101.479 | 1.00 | 29.76 | C |
| ATOM | 4419 | O | GLU | C | 150 | -30.806 | -58.770 | 100.312 | 1.00 | 30.40 | O |
| ATOM | 4420 | CB | GLU | C | 150 | -28.839 | -57.872 | 102.761 | 1.00 | 29.87 | C |
| ATOM | 4421 | CG | GLU | C | 150 | -27.928 | -58.160 | 103.958 | 1.00 | 29.34 | C |
| ATOM | 4422 | CD | GLU | C | 150 | -28.365 | -57.400 | 105.210 | 1.00 | 38.78 | C |
| ATOM | 4423 | OE1 | GLU | C | 150 | -27.573 | -56.543 | 105.699 | 1.00 | 37.11 | O |
| ATOM | 4424 | OE2 | GLU | C | 150 | -29.512 | -57.629 | 105.680 | 1.00 | 39.42 | O1- |
| ATOM | 4425 | N | PRO | C | 151 | -31.930 | -57.571 | 101.855 | 1.00 | 28.20 | N |
| ATOM | 4426 | CA | PRO | C | 151 | -32.224 | -57.019 | 103.180 | 1.00 | 28.76 | C |
| ATOM | 4427 | C | PRO | C | 151 | -33.460 | -57.594 | 103.883 | 1.00 | 32.34 | C |
| ATOM | 4428 | O | PRO | C | 151 | -34.284 | -58.288 | 103.291 | 1.00 | 27.74 | O |
| ATOM | 4429 | CB | PRO | C | 151 | -32.488 | -55.554 | 102.867 | 1.00 | 21.65 | C |
| ATOM | 4430 | CG | PRO | C | 151 | -33.145 | -55.605 | 101.554 | 1.00 | 15.86 | C |
| ATOM | 4431 | CD | PRO | C | 151 | -32.640 | -56.807 | 100.810 | 1.00 | 20.18 | C |
| ATOM | 4432 | N | VAL | C | 152 | -33.572 | -57.269 | 105.165 | 1.00 | 31.09 | N |
| ATOM | 4433 | CA | VAL | C | 152 | -34.798 | -57.431 | 105.925 | 1.00 | 31.79 | C |
| ATOM | 4434 | C | VAL | C | 152 | -35.215 | -56.053 | 106.399 | 1.00 | 32.70 | C |
| ATOM | 4435 | O | VAL | C | 152 | -34.378 | -55.174 | 106.625 | 1.00 | 38.99 | O |
| ATOM | 4436 | CB | VAL | C | 152 | -34.616 | -58.366 | 107.130 | 1.00 | 32.39 | C |
| ATOM | 4437 | CG1 | VAL | C | 152 | -34.480 | -59.798 | 106.688 | 1.00 | 34.75 | C |
| ATOM | 4438 | CG2 | VAL | C | 152 | -33.386 | -57.962 | 107.854 | 1.00 | 32.91 | C |
| ATOM | 4439 | N | THR | C | 153 | -36.513 | -55.863 | 106.552 | 1.00 | 30.59 | N |
| ATOM | 4440 | CA | THR | C | 153 | -37.032 | -54.660 | 107.178 | 1.00 | 33.61 | C |
| ATOM | 4441 | C | THR | C | 153 | -37.799 | -55.060 | 108.426 | 1.00 | 32.20 | C |
| ATOM | 4442 | O | THR | C | 153 | -38.455 | -56.108 | 108.450 | 1.00 | 30.80 | O |
| ATOM | 4443 | CB | THR | C | 153 | -37.918 | -53.848 | 106.212 | 1.00 | 32.33 | C |
| ATOM | 4444 | OG1 | THR | C | 153 | -38.950 | -54.679 | 105.683 | 1.00 | 37.08 | O |
| ATOM | 4445 | CG2 | THR | C | 153 | -37.089 | -53.315 | 105.061 | 1.00 | 33.42 | C |
| ATOM | 4446 | N | VAL | C | 154 | -37.709 | -54.221 | 109.459 | 1.00 | 27.86 | N |
| ATOM | 4447 | CA | VAL | C | 154 | -38.401 | -54.438 | 110.727 | 1.00 | 29.82 | C |
| ATOM | 4448 | C | VAL | C | 154 | -39.162 | -53.168 | 111.090 | 1.00 | 26.12 | C |
| ATOM | 4449 | O | VAL | C | 154 | -38.574 | -52.085 | 111.121 | 1.00 | 30.95 | O |
| ATOM | 4450 | CB | VAL | C | 154 | -37.414 | -54.808 | 111.861 | 1.00 | 29.46 | C |
| ATOM | 4451 | CG1 | VAL | C | 154 | -38.155 | -55.064 | 113.142 | 1.00 | 29.26 | C |
| ATOM | 4452 | CG2 | VAL | C | 154 | -36.547 | -56.023 | 111.485 | 1.00 | 24.20 | C |
| ATOM | 4453 | N | SER | C | 155 | -40.459 | -53.293 | 111.373 | 1.00 | 28.07 | N |
| ATOM | 4454 | CA | SER | C | 155 | -41.207 | -52.221 | 112.033 | 1.00 | 29.61 | C |
| ATOM | 4455 | C | SER | C | 155 | -41.832 | -52.750 | 113.315 | 1.00 | 35.01 | C |
| ATOM | 4456 | O | SER | C | 155 | -41.790 | -53.949 | 113.616 | 1.00 | 36.17 | O |
| ATOM | 4457 | CB | SER | C | 155 | -42.310 | -51.632 | 111.151 | 1.00 | 25.87 | C |
| ATOM | 4458 | OG | SER | C | 155 | -43.302 | -52.591 | 110.859 | 1.00 | 28.75 | O |
| ATOM | 4459 | N | TRP | C | 156 | -42.424 | -51.846 | 114.080 | 1.00 | 29.64 | N |
| ATOM | 4460 | CA | TRP | C | 156 | -43.029 | -52.229 | 115.344 | 1.00 | 33.60 | C |
| ATOM | 4461 | C | TRP | C | 156 | -44.490 | -51.793 | 115.369 | 1.00 | 34.15 | C |
| ATOM | 4462 | O | TRP | C | 156 | -44.813 | -50.652 | 115.017 | 1.00 | 33.36 | O |
| ATOM | 4463 | CB | TRP | C | 156 | -42.226 | -51.655 | 116.514 | 1.00 | 31.35 | C |
| ATOM | 4464 | CG | TRP | C | 156 | -40.969 | -52.463 | 116.781 | 1.00 | 31.98 | C |
| ATOM | 4465 | CD1 | TRP | C | 156 | -39.734 | -52.302 | 116.196 | 1.00 | 33.14 | C |
| ATOM | 4466 | CD2 | TRP | C | 156 | -40.836 | -53.564 | 117.686 | 1.00 | 30.45 | C |
| ATOM | 4467 | NE1 | TRP | C | 156 | -38.849 | -53.230 | 116.690 | 1.00 | 25.96 | N |
| ATOM | 4468 | CE2 | TRP | C | 156 | -39.498 | -54.008 | 117.616 | 1.00 | 30.62 | C |
| ATOM | 4469 | CE3 | TRP | C | 156 | -41.717 | -54.213 | 118.566 | 1.00 | 32.88 | C |

| | | | | | | | | | | | |
|---|---|---|---|---|---|---|---|---|---|---|---|
| ATOM | 4470 | CZ2 | TRP | C | 156 | -39.025 | -55.076 | 118.391 | 1.00 | 31.18 | C |
| ATOM | 4471 | CZ3 | TRP | C | 156 | -41.243 | -55.274 | 119.341 | 1.00 | 27.61 | C |
| ATOM | 4472 | CH2 | TRP | C | 156 | -39.914 | -55.694 | 119.242 | 1.00 | 30.88 | C |
| ATOM | 4473 | N | ASN | C | 157 | -45.370 | -52.719 | 115.757 | 1.00 | 34.57 | N |
| ATOM | 4474 | CA | ASN | C | 157 | -46.805 | -52.459 | 115.826 | 1.00 | 28.72 | C |
| ATOM | 4475 | C | ASN | C | 157 | -47.299 | -51.895 | 114.495 | 1.00 | 36.26 | C |
| ATOM | 4476 | O | ASN | C | 157 | -47.997 | -50.880 | 114.432 | 1.00 | 35.07 | O |
| ATOM | 4477 | CB | ASN | C | 157 | -47.133 | -51.515 | 116.989 | 1.00 | 31.99 | C |
| ATOM | 4478 | CG | ASN | C | 157 | -46.897 | -52.156 | 118.368 | 1.00 | 33.31 | C |
| ATOM | 4479 | OD1 | ASN | C | 157 | -46.496 | -53.318 | 118.479 | 1.00 | 34.80 | O |
| ATOM | 4480 | ND2 | ASN | C | 157 | -47.136 | -51.387 | 119.418 | 1.00 | 32.04 | N |
| ATOM | 4481 | N | SER | C | 158 | -46.870 | -52.540 | 113.410 | 1.00 | 39.26 | N |
| ATOM | 4482 | CA | SER | C | 158 | -47.310 | -52.190 | 112.062 | 1.00 | 36.22 | C |
| ATOM | 4483 | C | SER | C | 158 | -46.991 | -50.739 | 111.719 | 1.00 | 35.47 | C |
| ATOM | 4484 | O | SER | C | 158 | -47.673 | -50.119 | 110.908 | 1.00 | 39.31 | O |
| ATOM | 4485 | CB | SER | C | 158 | -48.807 | -52.459 | 111.895 | 1.00 | 33.86 | C |
| ATOM | 4486 | OG | SER | C | 158 | -49.145 | -53.743 | 112.399 | 1.00 | 43.34 | O |
| ATOM | 4487 | N | GLY | C | 159 | -45.938 | -50.192 | 112.306 | 1.00 | 35.45 | N |
| ATOM | 4488 | CA | GLY | C | 159 | -45.566 | -48.813 | 112.078 | 1.00 | 33.17 | C |
| ATOM | 4489 | C | GLY | C | 159 | -46.053 | -47.833 | 113.124 | 1.00 | 36.82 | C |
| ATOM | 4490 | O | GLY | C | 159 | -45.608 | -46.684 | 113.114 | 1.00 | 41.61 | O |
| ATOM | 4491 | N | ALA | C | 160 | -46.919 | -48.260 | 114.048 | 1.00 | 38.45 | N |
| ATOM | 4492 | CA | ALA | C | 160 | -47.469 | -47.347 | 115.047 | 1.00 | 32.99 | C |
| ATOM | 4493 | C | ALA | C | 160 | -46.449 | -46.967 | 116.108 | 1.00 | 41.51 | C |
| ATOM | 4494 | O | ALA | C | 160 | -46.566 | -45.903 | 116.718 | 1.00 | 49.03 | O |
| ATOM | 4495 | CB | ALA | C | 160 | -48.695 | -47.977 | 115.719 | 1.00 | 29.64 | C |
| ATOM | 4496 | N | LEU | C | 161 | -45.463 | -47.818 | 116.362 | 1.00 | 40.25 | N |
| ATOM | 4497 | CA | LEU | C | 161 | -44.450 | -47.562 | 117.372 | 1.00 | 30.64 | C |
| ATOM | 4498 | C | LEU | C | 161 | -43.161 | -47.174 | 116.659 | 1.00 | 33.32 | C |
| ATOM | 4499 | O | LEU | C | 161 | -42.702 | -47.877 | 115.757 | 1.00 | 31.89 | O |
| ATOM | 4500 | CB | LEU | C | 161 | -44.269 | -48.786 | 118.270 | 1.00 | 31.29 | C |
| ATOM | 4501 | CG | LEU | C | 161 | -43.229 | -48.759 | 119.395 | 1.00 | 38.45 | C |
| ATOM | 4502 | CD1 | LEU | C | 161 | -43.249 | -47.451 | 120.164 | 1.00 | 23.88 | C |
| ATOM | 4503 | CD2 | LEU | C | 161 | -43.494 | -49.922 | 120.328 | 1.00 | 32.82 | C |
| ATOM | 4504 | N | THR | C | 162 | -42.606 | -46.032 | 117.032 | 1.00 | 37.42 | N |
| ATOM | 4505 | CA | THR | C | 162 | -41.535 | -45.418 | 116.262 | 1.00 | 33.06 | C |
| ATOM | 4506 | C | THR | C | 162 | -40.497 | -44.859 | 117.219 | 1.00 | 29.92 | C |
| ATOM | 4507 | O | THR | C | 162 | -39.296 | -45.096 | 117.071 | 1.00 | 28.52 | O |
| ATOM | 4508 | CB | THR | C | 162 | -42.139 | -44.328 | 115.362 | 1.00 | 36.32 | C |
| ATOM | 4509 | OG1 | THR | C | 162 | -42.104 | -44.761 | 113.998 | 1.00 | 34.07 | O |
| ATOM | 4510 | CG2 | THR | C | 162 | -41.443 | -42.968 | 115.527 | 1.00 | 36.07 | C |
| ATOM | 4511 | N | SER | C | 163 | -40.981 | -44.153 | 118.234 | 1.00 | 30.45 | N |
| ATOM | 4512 | CA | SER | C | 163 | -40.121 | -43.634 | 119.272 | 1.00 | 26.52 | C |
| ATOM | 4513 | C | SER | C | 163 | -39.452 | -44.780 | 120.014 | 1.00 | 30.83 | C |
| ATOM | 4514 | O | SER | C | 163 | -40.117 | -45.724 | 120.461 | 1.00 | 32.58 | O |
| ATOM | 4515 | CB | SER | C | 163 | -40.954 | -42.786 | 120.226 | 1.00 | 28.23 | C |
| ATOM | 4516 | OG | SER | C | 163 | -40.230 | -42.404 | 121.384 | 1.00 | 40.32 | O |
| ATOM | 4517 | N | GLY | C | 164 | -38.137 | -44.673 | 120.178 | 1.00 | 30.58 | N |
| ATOM | 4518 | CA | GLY | C | 164 | -37.364 | -45.625 | 120.941 | 1.00 | 29.23 | C |
| ATOM | 4519 | C | GLY | C | 164 | -36.839 | -46.804 | 120.153 | 1.00 | 31.45 | C |
| ATOM | 4520 | O | GLY | C | 164 | -36.059 | -47.589 | 120.704 | 1.00 | 33.69 | O |
| ATOM | 4521 | N | VAL | C | 165 | -37.207 | -46.930 | 118.878 | 1.00 | 27.51 | N |
| ATOM | 4522 | CA | VAL | C | 165 | -36.793 | -48.069 | 118.070 | 1.00 | 28.93 | C |
| ATOM | 4523 | C | VAL | C | 165 | -35.357 | -47.887 | 117.587 | 1.00 | 28.12 | C |
| ATOM | 4524 | O | VAL | C | 165 | -34.979 | -46.831 | 117.081 | 1.00 | 37.17 | O |
| ATOM | 4525 | CB | VAL | C | 165 | -37.748 | -48.269 | 116.884 | 1.00 | 26.69 | C |
| ATOM | 4526 | CG1 | VAL | C | 165 | -37.278 | -49.432 | 116.023 | 1.00 | 24.04 | C |
| ATOM | 4527 | CG2 | VAL | C | 165 | -39.163 | -48.502 | 117.367 | 1.00 | 24.09 | C |
| ATOM | 4528 | N | HIS | C | 166 | -34.566 | -48.942 | 117.693 | 1.00 | 30.28 | N |
| ATOM | 4529 | CA | HIS | C | 166 | -33.290 | -49.052 | 116.999 | 1.00 | 27.00 | C |
| ATOM | 4530 | C | HIS | C | 166 | -33.270 | -50.379 | 116.259 | 1.00 | 23.79 | C |
| ATOM | 4531 | O | HIS | C | 166 | -33.286 | -51.436 | 116.895 | 1.00 | 25.39 | O |
| ATOM | 4532 | CB | HIS | C | 166 | -32.105 | -49.007 | 117.975 | 1.00 | 27.83 | C |
| ATOM | 4533 | CG | HIS | C | 166 | -32.009 | -47.759 | 118.809 | 1.00 | 31.08 | C |
| ATOM | 4534 | ND1 | HIS | C | 166 | -31.757 | -46.516 | 118.271 | 1.00 | 32.94 | N |
| ATOM | 4535 | CD2 | HIS | C | 166 | -32.070 | -47.578 | 120.153 | 1.00 | 30.56 | C |
| ATOM | 4536 | CE1 | HIS | C | 166 | -31.681 | -45.622 | 119.242 | 1.00 | 30.93 | C |
| ATOM | 4537 | NE2 | HIS | C | 166 | -31.861 | -46.242 | 120.395 | 1.00 | 27.04 | N |
| ATOM | 4538 | N | THR | C | 167 | -33.209 | -50.338 | 114.928 | 1.00 | 24.71 | N |
| ATOM | 4539 | CA | THR | C | 167 | -32.896 | -51.526 | 114.139 | 1.00 | 22.25 | C |
| ATOM | 4540 | C | THR | C | 167 | -31.455 | -51.439 | 113.637 | 1.00 | 24.53 | C |

| | | | | | | | | | | | |
|---|---|---|---|---|---|---|---|---|---|---|---|
| ATOM | 4541 | O | THR | C | 167 | -31.093 | -50.498 | 112.927 | 1.00 | 26.79 | O |
| ATOM | 4542 | CB | THR | C | 167 | -33.857 | -51.694 | 112.980 | 1.00 | 20.41 | C |
| ATOM | 4543 | OG1 | THR | C | 167 | -35.176 | -51.829 | 113.501 | 1.00 | 28.97 | O |
| ATOM | 4544 | CG2 | THR | C | 167 | -33.529 | -52.967 | 112.219 | 1.00 | 21.90 | C |
| ATOM | 4545 | N | PHE | C | 168 | -30.658 | -52.417 | 113.986 | 1.00 | 25.58 | N |
| ATOM | 4546 | CA | PHE | C | 168 | -29.210 | -52.471 | 113.865 | 1.00 | 26.05 | C |
| ATOM | 4547 | C | PHE | C | 168 | -28.807 | -53.018 | 112.505 | 1.00 | 21.09 | C |
| ATOM | 4548 | O | PHE | C | 168 | -29.560 | -53.760 | 111.890 | 1.00 | 25.00 | O |
| ATOM | 4549 | CB | PHE | C | 168 | -28.623 | -53.352 | 114.969 | 1.00 | 23.55 | C |
| ATOM | 4550 | CG | PHE | C | 168 | -28.681 | -52.722 | 116.316 | 1.00 | 22.06 | C |
| ATOM | 4551 | CD1 | PHE | C | 168 | -29.812 | -52.855 | 117.113 | 1.00 | 23.76 | C |
| ATOM | 4552 | CD2 | PHE | C | 168 | -27.617 | -51.958 | 116.788 | 1.00 | 24.14 | C |
| ATOM | 4553 | CE1 | PHE | C | 168 | -29.892 | -52.232 | 118.390 | 1.00 | 24.66 | C |
| ATOM | 4554 | CE2 | PHE | C | 168 | -27.683 | -51.341 | 118.064 | 1.00 | 28.11 | C |
| ATOM | 4555 | CZ | PHE | C | 168 | -28.828 | -51.482 | 118.866 | 1.00 | 22.06 | C |
| ATOM | 4556 | N | PRO | C | 169 | -27.626 | -52.662 | 112.009 | 1.00 | 26.60 | N |
| ATOM | 4557 | CA | PRO | C | 169 | -27.121 | -53.295 | 110.778 | 1.00 | 25.79 | C |
| ATOM | 4558 | C | PRO | C | 169 | -26.907 | -54.793 | 110.988 | 1.00 | 26.33 | C |
| ATOM | 4559 | O | PRO | C | 169 | -26.457 | -55.228 | 112.050 | 1.00 | 23.48 | O |
| ATOM | 4560 | CB | PRO | C | 169 | -25.790 | -52.569 | 110.525 | 1.00 | 17.06 | C |
| ATOM | 4561 | CG | PRO | C | 169 | -25.829 | -51.348 | 111.350 | 1.00 | 17.09 | C |
| ATOM | 4562 | CD | PRO | C | 169 | -26.686 | -51.660 | 112.546 | 1.00 | 22.12 | C |
| ATOM | 4563 | N | ALA | C | 170 | -27.222 | -55.586 | 109.966 | 1.00 | 23.48 | N |
| ATOM | 4564 | CA | ALA | C | 170 | -27.009 | -57.027 | 110.064 | 1.00 | 26.56 | C |
| ATOM | 4565 | C | ALA | C | 170 | -25.523 | -57.369 | 110.178 | 1.00 | 25.54 | C |
| ATOM | 4566 | O | ALA | C | 170 | -24.650 | -56.604 | 109.782 | 1.00 | 26.69 | O |
| ATOM | 4567 | CB | ALA | C | 170 | -27.591 | -57.744 | 108.847 | 1.00 | 29.48 | C |
| ATOM | 4568 | N | VAL | C | 171 | -25.237 | -58.531 | 110.747 | 1.00 | 26.07 | N |
| ATOM | 4569 | CA | VAL | C | 171 | -23.899 | -59.104 | 110.683 | 1.00 | 24.19 | C |
| ATOM | 4570 | C | VAL | C | 171 | -23.934 | -60.333 | 109.789 | 1.00 | 26.45 | C |
| ATOM | 4571 | O | VAL | C | 171 | -24.935 | -61.047 | 109.711 | 1.00 | 31.14 | O |
| ATOM | 4572 | CB | VAL | C | 171 | -23.315 | -59.455 | 112.072 | 1.00 | 29.65 | C |
| ATOM | 4573 | CG1 | VAL | C | 171 | -23.042 | -58.193 | 112.858 | 1.00 | 27.11 | C |
| ATOM | 4574 | CG2 | VAL | C | 171 | -24.254 | -60.404 | 112.853 | 1.00 | 25.85 | C |
| ATOM | 4575 | N | LEU | C | 172 | -22.832 | -60.571 | 109.101 | 1.00 | 31.64 | N |
| ATOM | 4576 | CA | LEU | C | 172 | -22.658 | -61.767 | 108.288 | 1.00 | 28.57 | C |
| ATOM | 4577 | C | LEU | C | 172 | -21.868 | -62.750 | 109.143 | 1.00 | 26.67 | C |
| ATOM | 4578 | O | LEU | C | 172 | -20.718 | -62.492 | 109.489 | 1.00 | 30.87 | O |
| ATOM | 4579 | CB | LEU | C | 172 | -21.937 | -61.442 | 106.982 | 1.00 | 27.30 | C |
| ATOM | 4580 | CG | LEU | C | 172 | -21.662 | -62.583 | 105.992 | 1.00 | 32.63 | C |
| ATOM | 4581 | CD1 | LEU | C | 172 | -22.971 | -63.245 | 105.549 | 1.00 | 26.83 | C |
| ATOM | 4582 | CD2 | LEU | C | 172 | -20.839 | -62.101 | 104.772 | 1.00 | 28.21 | C |
| ATOM | 4583 | N | GLN | C | 173 | -22.503 | -63.845 | 109.522 | 1.00 | 27.90 | N |
| ATOM | 4584 | CA | GLN | C | 173 | -21.916 | -64.862 | 110.372 | 1.00 | 32.35 | C |
| ATOM | 4585 | C | GLN | C | 173 | -21.087 | -65.824 | 109.528 | 1.00 | 36.44 | C |
| ATOM | 4586 | O | GLN | C | 173 | -21.204 | -65.870 | 108.294 | 1.00 | 34.30 | O |
| ATOM | 4587 | CB | GLN | C | 173 | -23.010 | -65.632 | 111.111 | 1.00 | 33.56 | C |
| ATOM | 4588 | CG | GLN | C | 173 | -23.996 | -64.769 | 111.851 | 1.00 | 29.57 | C |
| ATOM | 4589 | CD | GLN | C | 173 | -25.319 | -65.478 | 112.079 | 1.00 | 34.07 | C |
| ATOM | 4590 | OE1 | GLN | C | 173 | -25.724 | -65.696 | 113.215 | 1.00 | 34.19 | O |
| ATOM | 4591 | NE2 | GLN | C | 173 | -26.002 | -65.836 | 110.991 | 1.00 | 32.53 | N |
| ATOM | 4592 | N | SER | C | 174 | -20.254 | -66.622 | 110.208 | 1.00 | 36.04 | N |
| ATOM | 4593 | CA | SER | C | 174 | -19.363 | -67.525 | 109.481 | 1.00 | 33.82 | C |
| ATOM | 4594 | C | SER | C | 174 | -20.138 | -68.509 | 108.622 | 1.00 | 32.65 | C |
| ATOM | 4595 | O | SER | C | 174 | -19.561 | -69.087 | 107.697 | 1.00 | 35.26 | O |
| ATOM | 4596 | CB | SER | C | 174 | -18.437 | -68.272 | 110.441 | 1.00 | 31.17 | C |
| ATOM | 4597 | OG | SER | C | 174 | -18.868 | -68.133 | 111.788 | 1.00 | 51.22 | O |
| ATOM | 4598 | N | SER | C | 175 | -21.438 | -68.678 | 108.879 | 1.00 | 30.00 | N |
| ATOM | 4599 | CA | SER | C | 175 | -22.267 | -69.541 | 108.055 | 1.00 | 23.99 | C |
| ATOM | 4600 | C | SER | C | 175 | -22.566 | -68.950 | 106.691 | 1.00 | 33.95 | C |
| ATOM | 4601 | O | SER | C | 175 | -23.018 | -69.689 | 105.810 | 1.00 | 36.33 | O |
| ATOM | 4602 | CB | SER | C | 175 | -23.588 | -69.851 | 108.767 | 1.00 | 31.25 | C |
| ATOM | 4603 | OG | SER | C | 175 | -24.482 | -68.743 | 108.809 | 1.00 | 33.60 | O |
| ATOM | 4604 | N | GLY | C | 176 | -22.299 | -67.659 | 106.473 | 1.00 | 34.11 | N |
| ATOM | 4605 | CA | GLY | C | 176 | -22.780 | -67.006 | 105.275 | 1.00 | 26.57 | C |
| ATOM | 4606 | C | GLY | C | 176 | -24.191 | -66.479 | 105.380 | 1.00 | 33.77 | C |
| ATOM | 4607 | O | GLY | C | 176 | -24.690 | -65.897 | 104.409 | 1.00 | 34.08 | O |
| ATOM | 4608 | N | LEU | C | 177 | -24.857 | -66.682 | 106.515 | 1.00 | 32.19 | N |
| ATOM | 4609 | CA | LEU | C | 177 | -26.185 | -66.145 | 106.765 | 1.00 | 30.75 | C |
| ATOM | 4610 | C | LEU | C | 177 | -26.072 | -64.870 | 107.584 | 1.00 | 33.31 | C |
| ATOM | 4611 | O | LEU | C | 177 | -25.122 | -64.683 | 108.347 | 1.00 | 33.18 | O |

| | | | | | | | | | | | |
|---|---|---|---|---|---|---|---|---|---|---|---|
| ATOM | 4612 | CB | LEU | C | 177 | -27.062 | -67.141 | 107.512 | 1.00 | 35.99 | C |
| ATOM | 4613 | CG | LEU | C | 177 | -27.316 | -68.487 | 106.843 | 1.00 | 34.64 | C |
| ATOM | 4614 | CD1 | LEU | C | 177 | -28.208 | -69.291 | 107.732 | 1.00 | 30.71 | C |
| ATOM | 4615 | CD2 | LEU | C | 177 | -27.932 | -68.291 | 105.469 | 1.00 | 29.23 | C |
| ATOM | 4616 | N | TYR | C | 178 | -27.048 | -63.992 | 107.413 | 1.00 | 32.29 | N |
| ATOM | 4617 | CA | TYR | C | 178 | -27.076 | -62.736 | 108.138 | 1.00 | 29.56 | C |
| ATOM | 4618 | C | TYR | C | 178 | -27.923 | -62.834 | 109.408 | 1.00 | 32.53 | C |
| ATOM | 4619 | O | TYR | C | 178 | -28.819 | -63.665 | 109.535 | 1.00 | 33.61 | O |
| ATOM | 4620 | CB | TYR | C | 178 | -27.616 | -61.627 | 107.249 | 1.00 | 26.03 | C |
| ATOM | 4621 | CG | TYR | C | 178 | -26.708 | -61.260 | 106.096 | 1.00 | 32.82 | C |
| ATOM | 4622 | CD1 | TYR | C | 178 | -25.696 | -60.309 | 106.248 | 1.00 | 25.25 | C |
| ATOM | 4623 | CD2 | TYR | C | 178 | -26.874 | -61.855 | 104.843 | 1.00 | 28.59 | C |
| ATOM | 4624 | CE1 | TYR | C | 178 | -24.890 | -59.978 | 105.188 | 1.00 | 27.51 | C |
| ATOM | 4625 | CE2 | TYR | C | 178 | -26.075 | -61.526 | 103.785 | 1.00 | 25.14 | C |
| ATOM | 4626 | CZ | TYR | C | 178 | -25.085 | -60.589 | 103.952 | 1.00 | 33.10 | C |
| ATOM | 4627 | OH | TYR | C | 178 | -24.299 | -60.260 | 102.873 | 1.00 | 31.54 | O |
| ATOM | 4628 | N | SER | C | 179 | -27.674 | -61.906 | 110.316 | 1.00 | 30.12 | N |
| ATOM | 4629 | CA | SER | C | 179 | -28.377 | -61.836 | 111.580 | 1.00 | 26.37 | C |
| ATOM | 4630 | C | SER | C | 179 | -28.484 | -60.373 | 111.974 | 1.00 | 28.91 | C |
| ATOM | 4631 | O | SER | C | 179 | -27.473 | -59.669 | 112.014 | 1.00 | 33.49 | O |
| ATOM | 4632 | CB | SER | C | 179 | -27.616 | -62.615 | 112.653 | 1.00 | 31.79 | C |
| ATOM | 4633 | OG | SER | C | 179 | -28.434 | -63.550 | 113.310 | 1.00 | 36.87 | O |
| ATOM | 4634 | N | LEU | C | 180 | -29.693 | -59.908 | 112.244 | 1.00 | 24.41 | N |
| ATOM | 4635 | CA | LEU | C | 180 | -29.847 | -58.581 | 112.796 | 1.00 | 26.13 | C |
| ATOM | 4636 | C | LEU | C | 180 | -30.787 | -58.623 | 113.991 | 1.00 | 33.77 | C |
| ATOM | 4637 | O | LEU | C | 180 | -31.507 | -59.597 | 114.238 | 1.00 | 33.18 | O |
| ATOM | 4638 | CB | LEU | C | 180 | -30.340 | -57.570 | 111.757 | 1.00 | 32.63 | C |
| ATOM | 4639 | CG | LEU | C | 180 | -31.742 | -57.362 | 111.177 | 1.00 | 31.41 | C |
| ATOM | 4640 | CD1 | LEU | C | 180 | -32.886 | -57.085 | 112.196 | 1.00 | 23.59 | C |
| ATOM | 4641 | CD2 | LEU | C | 180 | -31.574 | -56.181 | 110.212 | 1.00 | 23.09 | C |
| ATOM | 4642 | N | SER | C | 181 | -30.773 | -57.531 | 114.735 | 1.00 | 33.23 | N |
| ATOM | 4643 | CA | SER | C | 181 | -31.662 | -57.356 | 115.859 | 1.00 | 28.03 | C |
| ATOM | 4644 | C | SER | C | 181 | -32.311 | -55.982 | 115.766 | 1.00 | 26.07 | C |
| ATOM | 4645 | O | SER | C | 181 | -31.777 | -55.063 | 115.154 | 1.00 | 28.63 | O |
| ATOM | 4646 | CB | SER | C | 181 | -30.906 | -57.540 | 117.184 | 1.00 | 30.44 | C |
| ATOM | 4647 | OG | SER | C | 181 | -29.984 | -56.493 | 117.391 | 1.00 | 31.22 | O |
| ATOM | 4648 | N | SER | C | 182 | -33.506 | -55.881 | 116.326 | 1.00 | 27.13 | N |
| ATOM | 4649 | CA | SER | C | 182 | -34.271 | -54.649 | 116.409 | 1.00 | 27.94 | C |
| ATOM | 4650 | C | SER | C | 182 | -34.805 | -54.551 | 117.827 | 1.00 | 28.64 | C |
| ATOM | 4651 | O | SER | C | 182 | -35.302 | -55.543 | 118.363 | 1.00 | 26.17 | O |
| ATOM | 4652 | CB | SER | C | 182 | -35.424 | -54.637 | 115.408 | 1.00 | 25.46 | C |
| ATOM | 4653 | OG | SER | C | 182 | -36.221 | -53.487 | 115.599 | 1.00 | 27.32 | O |
| ATOM | 4654 | N | VAL | C | 183 | -34.683 | -53.373 | 118.446 | 1.00 | 27.27 | N |
| ATOM | 4655 | CA | VAL | C | 183 | -35.089 | -53.201 | 119.837 | 1.00 | 30.94 | C |
| ATOM | 4656 | C | VAL | C | 183 | -35.878 | -51.909 | 120.005 | 1.00 | 30.82 | C |
| ATOM | 4657 | O | VAL | C | 183 | -35.875 | -51.032 | 119.142 | 1.00 | 33.72 | O |
| ATOM | 4658 | CB | VAL | C | 183 | -33.886 | -53.209 | 120.800 | 1.00 | 27.96 | C |
| ATOM | 4659 | CG1 | VAL | C | 183 | -33.091 | -54.486 | 120.621 | 1.00 | 17.87 | C |
| ATOM | 4660 | CG2 | VAL | C | 183 | -33.031 | -51.953 | 120.585 | 1.00 | 29.36 | C |
| ATOM | 4661 | N | VAL | C | 184 | -36.585 | -51.813 | 121.122 | 1.00 | 25.36 | N |
| ATOM | 4662 | CA | VAL | C | 184 | -37.315 | -50.601 | 121.452 | 1.00 | 29.08 | C |
| ATOM | 4663 | C | VAL | C | 184 | -37.320 | -50.459 | 122.968 | 1.00 | 33.22 | C |
| ATOM | 4664 | O | VAL | C | 184 | -37.476 | -51.444 | 123.700 | 1.00 | 34.38 | O |
| ATOM | 4665 | CB | VAL | C | 184 | -38.734 | -50.602 | 120.812 | 1.00 | 30.67 | C |
| ATOM | 4666 | CG1 | VAL | C | 184 | -39.523 | -51.866 | 121.135 | 1.00 | 28.94 | C |
| ATOM | 4667 | CG2 | VAL | C | 184 | -39.531 | -49.352 | 121.221 | 1.00 | 30.72 | C |
| ATOM | 4668 | N | THR | C | 185 | -37.102 | -49.234 | 123.440 | 1.00 | 31.38 | N |
| ATOM | 4669 | CA | THR | C | 185 | -37.213 | -48.922 | 124.854 | 1.00 | 29.00 | C |
| ATOM | 4670 | C | THR | C | 185 | -38.591 | -48.319 | 125.074 | 1.00 | 30.90 | C |
| ATOM | 4671 | O | THR | C | 185 | -39.048 | -47.490 | 124.284 | 1.00 | 37.29 | O |
| ATOM | 4672 | CB | THR | C | 185 | -36.102 | -47.977 | 125.330 | 1.00 | 24.92 | C |
| ATOM | 4673 | OG1 | THR | C | 185 | -35.966 | -46.874 | 124.424 | 1.00 | 32.54 | O |
| ATOM | 4674 | CG2 | THR | C | 185 | -34.779 | -48.706 | 125.402 | 1.00 | 26.78 | C |
| ATOM | 4675 | N | VAL | C | 186 | -39.273 | -48.800 | 126.103 | 1.00 | 29.32 | N |
| ATOM | 4676 | CA | VAL | C | 186 | -40.625 | -48.377 | 126.458 | 1.00 | 34.69 | C |
| ATOM | 4677 | C | VAL | C | 186 | -40.698 | -48.292 | 127.970 | 1.00 | 36.57 | C |
| ATOM | 4678 | O | VAL | C | 186 | -39.835 | -48.836 | 128.675 | 1.00 | 34.20 | O |
| ATOM | 4679 | CB | VAL | C | 186 | -41.699 | -49.358 | 125.944 | 1.00 | 36.77 | C |
| ATOM | 4680 | CG1 | VAL | C | 186 | -41.645 | -49.516 | 124.421 | 1.00 | 27.93 | C |
| ATOM | 4681 | CG2 | VAL | C | 186 | -41.571 | -50.715 | 126.679 | 1.00 | 32.35 | C |
| ATOM | 4682 | N | PRO | C | 187 | -41.717 | -47.608 | 128.499 | 1.00 | 38.80 | N |

| | | | | | | | | | | | |
|---|---|---|---|---|---|---|---|---|---|---|---|
| ATOM | 4683 | CA | PRO | C | 187 | -41.851 | -47.521 | 129.958 | 1.00 | 40.40 | C |
| ATOM | 4684 | C | PRO | C | 187 | -42.128 | -48.886 | 130.574 | 1.00 | 43.67 | C |
| ATOM | 4685 | O | PRO | C | 187 | -42.872 | -49.703 | 130.024 | 1.00 | 42.12 | O |
| ATOM | 4686 | CB | PRO | C | 187 | -43.033 | -46.566 | 130.145 | 1.00 | 36.03 | C |
| ATOM | 4687 | CG | PRO | C | 187 | -43.095 | -45.798 | 128.889 | 1.00 | 29.12 | C |
| ATOM | 4688 | CD | PRO | C | 187 | -42.697 | -46.739 | 127.820 | 1.00 | 34.04 | C |
| ATOM | 4689 | N | SER | C | 188 | -41.501 | -49.128 | 131.728 | 1.00 | 47.08 | N |
| ATOM | 4690 | CA | SER | C | 188 | -41.753 | -50.348 | 132.489 | 1.00 | 48.24 | C |
| ATOM | 4691 | C | SER | C | 188 | -43.244 | -50.561 | 132.733 | 1.00 | 47.93 | C |
| ATOM | 4692 | O | SER | C | 188 | -43.776 | -51.660 | 132.523 | 1.00 | 45.02 | O |
| ATOM | 4693 | CB | SER | C | 188 | -41.011 | -50.260 | 133.813 | 1.00 | 50.96 | C |
| ATOM | 4694 | OG | SER | C | 188 | -39.631 | -50.377 | 133.576 | 1.00 | 59.84 | O |
| ATOM | 4695 | N | SER | C | 189 | -43.941 | -49.495 | 133.134 | 1.00 | 46.10 | N |
| ATOM | 4696 | CA | SER | C | 189 | -45.363 | -49.574 | 133.444 | 1.00 | 51.64 | C |
| ATOM | 4697 | C | SER | C | 189 | -46.222 | -50.037 | 132.263 | 1.00 | 51.86 | C |
| ATOM | 4698 | O | SER | C | 189 | -47.344 | -50.512 | 132.481 | 1.00 | 58.15 | O |
| ATOM | 4699 | CB | SER | C | 189 | -45.834 | -48.218 | 133.976 | 1.00 | 51.35 | C |
| ATOM | 4700 | OG | SER | C | 189 | -45.680 | -47.208 | 133.001 | 1.00 | 55.98 | O |
| ATOM | 4701 | N | SER | C | 190 | -45.740 | -49.918 | 131.022 | 1.00 | 51.05 | N |
| ATOM | 4702 | CA | SER | C | 190 | -46.558 | -50.310 | 129.870 | 1.00 | 50.46 | C |
| ATOM | 4703 | C | SER | C | 190 | -46.527 | -51.807 | 129.570 | 1.00 | 49.06 | C |
| ATOM | 4704 | O | SER | C | 190 | -47.396 | -52.279 | 128.830 | 1.00 | 45.72 | O |
| ATOM | 4705 | CB | SER | C | 190 | -46.113 | -49.566 | 128.610 | 1.00 | 40.88 | C |
| ATOM | 4706 | OG | SER | C | 190 | -44.817 | -49.995 | 128.218 | 1.00 | 41.80 | O |
| ATOM | 4707 | N | LEU | C | 191 | -45.553 | -52.559 | 130.096 | 1.00 | 47.92 | N |
| ATOM | 4708 | CA | LEU | C | 191 | -45.508 | -53.993 | 129.838 | 1.00 | 42.80 | C |
| ATOM | 4709 | C | LEU | C | 191 | -46.706 | -54.670 | 130.475 | 1.00 | 48.54 | C |
| ATOM | 4710 | O | LEU | C | 191 | -47.206 | -54.244 | 131.517 | 1.00 | 56.38 | O |
| ATOM | 4711 | CB | LEU | C | 191 | -44.230 | -54.612 | 130.387 | 1.00 | 39.21 | C |
| ATOM | 4712 | CG | LEU | C | 191 | -42.884 | -54.023 | 129.982 | 1.00 | 44.80 | C |
| ATOM | 4713 | CD1 | LEU | C | 191 | -41.792 | -54.654 | 130.804 | 1.00 | 34.81 | C |
| ATOM | 4714 | CD2 | LEU | C | 191 | -42.634 | -54.240 | 128.501 | 1.00 | 37.00 | C |
| ATOM | 4715 | N | GLY | C | 192 | -47.170 | -55.743 | 129.856 | 1.00 | 48.62 | N |
| ATOM | 4716 | CA | GLY | C | 192 | -48.335 | -56.412 | 130.386 | 1.00 | 56.13 | C |
| ATOM | 4717 | C | GLY | C | 192 | -49.643 | -55.692 | 130.132 | 1.00 | 55.78 | C |
| ATOM | 4718 | O | GLY | C | 192 | -50.704 | -56.327 | 130.177 | 1.00 | 56.01 | O |
| ATOM | 4719 | N | THR | C | 193 | -49.601 | -54.388 | 129.869 | 1.00 | 48.97 | N |
| ATOM | 4720 | CA | THR | C | 193 | -50.727 | -53.607 | 129.378 | 1.00 | 49.79 | C |
| ATOM | 4721 | C | THR | C | 193 | -50.644 | -53.332 | 127.883 | 1.00 | 49.11 | C |
| ATOM | 4722 | O | THR | C | 193 | -51.669 | -53.324 | 127.197 | 1.00 | 53.12 | O |
| ATOM | 4723 | CB | THR | C | 193 | -50.780 | -52.266 | 130.114 | 1.00 | 58.23 | C |
| ATOM | 4724 | OG1 | THR | C | 193 | -50.126 | -52.405 | 131.385 | 1.00 | 58.05 | O |
| ATOM | 4725 | CG2 | THR | C | 193 | -52.227 | -51.790 | 130.293 | 1.00 | 53.04 | C |
| ATOM | 4726 | N | GLN | C | 194 | -49.443 | -53.103 | 127.361 | 1.00 | 48.44 | N |
| ATOM | 4727 | CA | GLN | C | 194 | -49.241 | -52.741 | 125.967 | 1.00 | 42.09 | C |
| ATOM | 4728 | C | GLN | C | 194 | -48.676 | -53.911 | 125.177 | 1.00 | 37.51 | C |
| ATOM | 4729 | O | GLN | C | 194 | -47.808 | -54.636 | 125.660 | 1.00 | 40.92 | O |
| ATOM | 4730 | CB | GLN | C | 194 | -48.324 | -51.517 | 125.873 | 1.00 | 43.18 | C |
| ATOM | 4731 | CG | GLN | C | 194 | -48.013 | -51.097 | 124.469 | 1.00 | 46.41 | C |
| ATOM | 4732 | CD | GLN | C | 194 | -49.252 | -50.772 | 123.679 | 1.00 | 49.54 | C |
| ATOM | 4733 | OE1 | GLN | C | 194 | -49.610 | -51.489 | 122.735 | 1.00 | 42.75 | O |
| ATOM | 4734 | NE2 | GLN | C | 194 | -49.939 | -49.707 | 124.080 | 1.00 | 49.73 | N |
| ATOM | 4735 | N | THR | C | 195 | -49.176 | -54.093 | 123.963 | 1.00 | 36.34 | N |
| ATOM | 4736 | CA | THR | C | 195 | -48.744 | -55.181 | 123.101 | 1.00 | 39.27 | C |
| ATOM | 4737 | C | THR | C | 195 | -47.657 | -54.704 | 122.151 | 1.00 | 39.62 | C |
| ATOM | 4738 | O | THR | C | 195 | -47.782 | -53.650 | 121.523 | 1.00 | 38.44 | O |
| ATOM | 4739 | CB | THR | C | 195 | -49.921 | -55.761 | 122.312 | 1.00 | 43.59 | C |
| ATOM | 4740 | OG1 | THR | C | 195 | -50.568 | -56.759 | 123.110 | 1.00 | 45.10 | O |
| ATOM | 4741 | CG2 | THR | C | 195 | -49.470 | -56.364 | 120.984 | 1.00 | 36.94 | C |
| ATOM | 4742 | N | TYR | C | 196 | -46.604 | -55.500 | 122.044 | 1.00 | 39.64 | N |
| ATOM | 4743 | CA | TYR | C | 196 | -45.452 | -55.182 | 121.224 | 1.00 | 31.97 | C |
| ATOM | 4744 | C | TYR | C | 196 | -45.224 | -56.327 | 120.252 | 1.00 | 32.44 | C |
| ATOM | 4745 | O | TYR | C | 196 | -44.912 | -57.445 | 120.668 | 1.00 | 28.81 | O |
| ATOM | 4746 | CB | TYR | C | 196 | -44.231 | -54.930 | 122.098 | 1.00 | 33.73 | C |
| ATOM | 4747 | CG | TYR | C | 196 | -44.426 | -53.737 | 123.002 | 1.00 | 38.32 | C |
| ATOM | 4748 | CD1 | TYR | C | 196 | -44.552 | -52.458 | 122.474 | 1.00 | 33.41 | C |
| ATOM | 4749 | CD2 | TYR | C | 196 | -44.483 | -53.884 | 124.380 | 1.00 | 37.95 | C |
| ATOM | 4750 | CE1 | TYR | C | 196 | -44.728 | -51.370 | 123.289 | 1.00 | 33.25 | C |
| ATOM | 4751 | CE2 | TYR | C | 196 | -44.660 | -52.788 | 125.201 | 1.00 | 36.63 | C |
| ATOM | 4752 | CZ | TYR | C | 196 | -44.781 | -51.541 | 124.647 | 1.00 | 33.58 | C |
| ATOM | 4753 | OH | TYR | C | 196 | -44.966 | -50.453 | 125.459 | 1.00 | 38.73 | O |

| | | | | | | | | | | | |
|---|---|---|---|---|---|---|---|---|---|---|---|
| ATOM | 4754 | N | ILE | C | 197 | -45.395 | -56.038 | 118.964 | 1.00 | 31.69 | N |
| ATOM | 4755 | CA | ILE | C | 197 | -45.176 | -56.988 | 117.885 | 1.00 | 31.54 | C |
| ATOM | 4756 | C | ILE | C | 197 | -44.163 | -56.386 | 116.919 | 1.00 | 33.20 | C |
| ATOM | 4757 | O | ILE | C | 197 | -44.321 | -55.239 | 116.484 | 1.00 | 33.69 | O |
| ATOM | 4758 | CB | ILE | C | 197 | -46.476 | -57.288 | 117.131 | 1.00 | 31.06 | C |
| ATOM | 4759 | CG1 | ILE | C | 197 | -47.516 | -57.886 | 118.042 | 1.00 | 33.48 | C |
| ATOM | 4760 | CG2 | ILE | C | 197 | -46.201 | -58.193 | 115.952 | 1.00 | 33.02 | C |
| ATOM | 4761 | CD1 | ILE | C | 197 | -48.862 | -57.783 | 117.425 | 1.00 | 24.92 | C |
| ATOM | 4762 | N | CYS | C | 198 | -43.163 | -57.165 | 116.533 | 1.00 | 28.72 | N |
| ATOM | 4763 | CA | CYS | C | 198 | -42.254 | -56.721 | 115.489 | 1.00 | 35.10 | C |
| ATOM | 4764 | C | CYS | C | 198 | -42.613 | -57.390 | 114.159 | 1.00 | 33.06 | C |
| ATOM | 4765 | O | CYS | C | 198 | -42.986 | -58.566 | 114.107 | 1.00 | 34.76 | O |
| ATOM | 4766 | CB | CYS | C | 198 | -40.798 | -56.986 | 115.869 | 1.00 | 35.03 | C |
| ATOM | 4767 | SG | CYS | C | 198 | -40.302 | -58.637 | 115.586 | 1.00 | 44.99 | S |
| ATOM | 4768 | N | ASN | C | 199 | -42.585 | -56.609 | 113.098 | 1.00 | 28.45 | N |
| ATOM | 4769 | CA | ASN | C | 199 | -43.018 | -57.057 | 111.786 | 1.00 | 29.27 | C |
| ATOM | 4770 | C | ASN | C | 199 | -41.775 | -57.159 | 110.921 | 1.00 | 28.88 | C |
| ATOM | 4771 | O | ASN | C | 199 | -41.126 | -56.148 | 110.645 | 1.00 | 34.72 | O |
| ATOM | 4772 | CB | ASN | C | 199 | -44.058 | -56.094 | 111.214 | 1.00 | 29.40 | C |
| ATOM | 4773 | CG | ASN | C | 199 | -45.068 | -55.631 | 112.274 | 1.00 | 35.22 | C |
| ATOM | 4774 | OD1 | ASN | C | 199 | -45.044 | -54.468 | 112.719 | 1.00 | 33.27 | O |
| ATOM | 4775 | ND2 | ASN | C | 199 | -45.951 | -56.546 | 112.693 | 1.00 | 28.65 | N |
| ATOM | 4776 | N | VAL | C | 200 | -41.435 | -58.381 | 110.521 | 1.00 | 26.15 | N |
| ATOM | 4777 | CA | VAL | C | 200 | -40.230 | -58.679 | 109.758 | 1.00 | 28.67 | C |
| ATOM | 4778 | C | VAL | C | 200 | -40.617 | -59.030 | 108.329 | 1.00 | 30.60 | C |
| ATOM | 4779 | O | VAL | C | 200 | -41.478 | -59.890 | 108.106 | 1.00 | 28.53 | O |
| ATOM | 4780 | CB | VAL | C | 200 | -39.450 | -59.841 | 110.388 | 1.00 | 21.99 | C |
| ATOM | 4781 | CG1 | VAL | C | 200 | -38.183 | -60.065 | 109.624 | 1.00 | 27.77 | C |
| ATOM | 4782 | CG2 | VAL | C | 200 | -39.182 | -59.577 | 111.849 | 1.00 | 26.33 | C |
| ATOM | 4783 | N | ASN | C | 201 | -39.960 | -58.406 | 107.359 | 1.00 | 31.19 | N |
| ATOM | 4784 | CA | ASN | C | 201 | -40.153 | -58.800 | 105.970 | 1.00 | 33.45 | C |
| ATOM | 4785 | C | ASN | C | 201 | -38.805 | -59.085 | 105.341 | 1.00 | 35.94 | C |
| ATOM | 4786 | O | ASN | C | 201 | -37.952 | -58.195 | 105.238 | 1.00 | 38.25 | O |
| ATOM | 4787 | CB | ASN | C | 201 | -40.899 | -57.739 | 105.160 | 1.00 | 34.65 | C |
| ATOM | 4788 | CG | ASN | C | 201 | -41.430 | -58.284 | 103.832 | 1.00 | 38.40 | C |
| ATOM | 4789 | OD1 | ASN | C | 201 | -42.045 | -57.555 | 103.074 | 1.00 | 47.99 | O |
| ATOM | 4790 | ND2 | ASN | C | 201 | -41.203 | -59.568 | 103.557 | 1.00 | 39.32 | N |
| ATOM | 4791 | N | HIS | C | 202 | -38.630 | -60.310 | 104.893 | 1.00 | 35.27 | N |
| ATOM | 4792 | CA | HIS | C | 202 | -37.474 | -60.692 | 104.107 | 1.00 | 32.39 | C |
| ATOM | 4793 | C | HIS | C | 202 | -38.047 | -60.925 | 102.719 | 1.00 | 34.80 | C |
| ATOM | 4794 | O | HIS | C | 202 | -38.424 | -62.043 | 102.375 | 1.00 | 35.23 | O |
| ATOM | 4795 | CB | HIS | C | 202 | -36.777 | -61.922 | 104.669 | 1.00 | 29.35 | C |
| ATOM | 4796 | CG | HIS | C | 202 | -35.529 | -62.276 | 103.935 | 1.00 | 32.95 | C |
| ATOM | 4797 | ND1 | HIS | C | 202 | -35.318 | -63.520 | 103.385 | 1.00 | 37.12 | N |
| ATOM | 4798 | CD2 | HIS | C | 202 | -34.440 | -61.537 | 103.623 | 1.00 | 31.23 | C |
| ATOM | 4799 | CE1 | HIS | C | 202 | -34.145 | -63.538 | 102.778 | 1.00 | 33.20 | C |
| ATOM | 4800 | NE2 | HIS | C | 202 | -33.593 | -62.346 | 102.905 | 1.00 | 32.51 | N |
| ATOM | 4801 | N | LYS | C | 203 | -38.159 | -59.837 | 101.948 | 1.00 | 37.66 | N |
| ATOM | 4802 | CA | LYS | C | 203 | -38.635 | -59.928 | 100.569 | 1.00 | 34.00 | C |
| ATOM | 4803 | C | LYS | C | 203 | -37.907 | -60.983 | 99.750 | 1.00 | 36.61 | C |
| ATOM | 4804 | O | LYS | C | 203 | -38.585 | -61.707 | 99.002 | 1.00 | 36.77 | O |
| ATOM | 4805 | CB | LYS | C | 203 | -38.464 | -58.583 | 99.854 | 1.00 | 31.21 | C |
| ATOM | 4806 | CG | LYS | C | 203 | -39.438 | -57.458 | 100.162 | 1.00 | 39.44 | C |
| ATOM | 4807 | CD | LYS | C | 203 | -40.808 | -57.621 | 99.534 | 1.00 | 41.59 | C |
| ATOM | 4808 | CE | LYS | C | 203 | -41.815 | -56.681 | 100.199 | 1.00 | 47.06 | C |
| ATOM | 4809 | NZ | LYS | C | 203 | -41.248 | -55.297 | 100.259 | 1.00 | 45.84 | N1+ |
| ATOM | 4810 | N | PRO | C | 204 | -36.574 | -61.155 | 99.854 | 1.00 | 38.41 | N |
| ATOM | 4811 | CA | PRO | C | 204 | -35.907 | -62.124 | 98.962 | 1.00 | 33.23 | C |
| ATOM | 4812 | C | PRO | C | 204 | -36.392 | -63.552 | 99.111 | 1.00 | 37.59 | C |
| ATOM | 4813 | O | PRO | C | 204 | -36.358 | -64.297 | 98.129 | 1.00 | 44.57 | O |
| ATOM | 4814 | CB | PRO | C | 204 | -34.425 | -61.994 | 99.342 | 1.00 | 28.63 | C |
| ATOM | 4815 | CG | PRO | C | 204 | -34.306 | -60.634 | 99.898 | 1.00 | 32.37 | C |
| ATOM | 4816 | CD | PRO | C | 204 | -35.587 | -60.346 | 100.603 | 1.00 | 28.15 | C |
| ATOM | 4817 | N | SER | C | 205 | -36.857 | -63.964 | 100.287 | 1.00 | 37.25 | N |
| ATOM | 4818 | CA | SER | C | 205 | -37.381 | -65.312 | 100.469 | 1.00 | 37.14 | C |
| ATOM | 4819 | C | SER | C | 205 | -38.895 | -65.320 | 100.574 | 1.00 | 38.83 | C |
| ATOM | 4820 | O | SER | C | 205 | -39.480 | -66.367 | 100.869 | 1.00 | 37.58 | O |
| ATOM | 4821 | CB | SER | C | 205 | -36.771 | -65.963 | 101.709 | 1.00 | 34.91 | C |
| ATOM | 4822 | OG | SER | C | 205 | -37.253 | -65.348 | 102.893 | 1.00 | 33.36 | O |
| ATOM | 4823 | N | ASN | C | 206 | -39.532 | -64.173 | 100.334 | 1.00 | 40.37 | N |
| ATOM | 4824 | CA | ASN | C | 206 | -40.974 | -63.989 | 100.466 | 1.00 | 40.89 | C |

| | | | | | | | | | | | |
|---|---|---|---|---|---|---|---|---|---|---|---|
| ATOM | 4825 | C | ASN | C | 206 | -41.484 | -64.469 | 101.827 | 1.00 | 44.23 | C |
| ATOM | 4826 | O | ASN | C | 206 | -42.478 | -65.192 | 101.926 | 1.00 | 46.80 | O |
| ATOM | 4827 | CB | ASN | C | 206 | -41.703 | -64.684 | 99.332 | 1.00 | 49.25 | C |
| ATOM | 4828 | CG | ASN | C | 206 | -42.643 | -63.766 | 98.626 | 1.00 | 60.95 | C |
| ATOM | 4829 | OD1 | ASN | C | 206 | -42.272 | -63.130 | 97.632 | 1.00 | 64.04 | O |
| ATOM | 4830 | ND2 | ASN | C | 206 | -43.871 | -63.660 | 99.141 | 1.00 | 62.21 | N |
| ATOM | 4831 | N | THR | C | 207 | -40.773 | -64.069 | 102.880 | 1.00 | 37.63 | N |
| ATOM | 4832 | CA | THR | C | 207 | -41.070 | -64.446 | 104.256 | 1.00 | 33.70 | C |
| ATOM | 4833 | C | THR | C | 207 | -41.518 | -63.223 | 105.044 | 1.00 | 35.76 | C |
| ATOM | 4834 | O | THR | C | 207 | -40.806 | -62.218 | 105.096 | 1.00 | 34.16 | O |
| ATOM | 4835 | CB | THR | C | 207 | -39.844 | -65.055 | 104.920 | 1.00 | 30.61 | C |
| ATOM | 4836 | OG1 | THR | C | 207 | -39.390 | -66.163 | 104.140 | 1.00 | 32.93 | O |
| ATOM | 4837 | CG2 | THR | C | 207 | -40.164 | -65.490 | 106.323 | 1.00 | 30.98 | C |
| ATOM | 4838 | N | LYS | C | 208 | -42.686 | -63.311 | 105.663 | 1.00 | 35.22 | N |
| ATOM | 4839 | CA | LYS | C | 208 | -43.154 | -62.277 | 106.565 | 1.00 | 31.98 | C |
| ATOM | 4840 | C | LYS | C | 208 | -43.461 | -62.906 | 107.915 | 1.00 | 33.42 | C |
| ATOM | 4841 | O | LYS | C | 208 | -44.079 | -63.966 | 107.977 | 1.00 | 34.80 | O |
| ATOM | 4842 | CB | LYS | C | 208 | -44.375 | -61.587 | 105.990 | 1.00 | 29.96 | C |
| ATOM | 4843 | CG | LYS | C | 208 | -44.038 | -60.590 | 104.902 | 1.00 | 34.70 | C |
| ATOM | 4844 | CD | LYS | C | 208 | -45.215 | -59.721 | 104.524 | 1.00 | 37.48 | C |
| ATOM | 4845 | CE | LYS | C | 208 | -46.304 | -60.544 | 103.838 | 1.00 | 44.32 | C |
| ATOM | 4846 | NZ | LYS | C | 208 | -47.529 | -59.739 | 103.513 | 1.00 | 56.22 | N1+ |
| ATOM | 4847 | N | VAL | C | 209 | -43.064 | -62.232 | 108.994 | 1.00 | 29.95 | N |
| ATOM | 4848 | CA | VAL | C | 209 | -43.234 | -62.745 | 110.348 | 1.00 | 29.28 | C |
| ATOM | 4849 | C | VAL | C | 209 | -43.711 | -61.614 | 111.255 | 1.00 | 31.89 | C |
| ATOM | 4850 | O | VAL | C | 209 | -43.177 | -60.501 | 111.209 | 1.00 | 33.86 | O |
| ATOM | 4851 | CB | VAL | C | 209 | -41.930 | -63.382 | 110.883 | 1.00 | 29.99 | C |
| ATOM | 4852 | CG1 | VAL | C | 209 | -42.024 | -63.631 | 112.364 | 1.00 | 29.51 | C |
| ATOM | 4853 | CG2 | VAL | C | 209 | -41.659 | -64.720 | 110.193 | 1.00 | 28.13 | C |
| ATOM | 4854 | N | ASP | C | 210 | -44.744 | -61.888 | 112.051 | 1.00 | 32.06 | N |
| ATOM | 4855 | CA | ASP | C | 210 | -45.187 | -61.015 | 113.137 | 1.00 | 31.18 | C |
| ATOM | 4856 | C | ASP | C | 210 | -44.963 | -61.720 | 114.466 | 1.00 | 32.54 | C |
| ATOM | 4857 | O | ASP | C | 210 | -45.627 | -62.717 | 114.749 | 1.00 | 37.02 | O |
| ATOM | 4858 | CB | ASP | C | 210 | -46.667 | -60.664 | 112.988 | 1.00 | 32.07 | C |
| ATOM | 4859 | CG | ASP | C | 210 | -46.950 | -59.774 | 111.779 | 1.00 | 38.99 | C |
| ATOM | 4860 | OD1 | ASP | C | 210 | -46.283 | -58.727 | 111.607 | 1.00 | 38.00 | O |
| ATOM | 4861 | OD2 | ASP | C | 210 | -47.851 | -60.125 | 110.993 | 1.00 | 43.24 | O1- |
| ATOM | 4862 | N | LYS | C | 211 | -44.090 | -61.171 | 115.307 | 1.00 | 28.18 | N |
| ATOM | 4863 | CA | LYS | C | 211 | -43.714 | -61.808 | 116.561 | 1.00 | 27.79 | C |
| ATOM | 4864 | C | LYS | C | 211 | -44.107 | -60.919 | 117.735 | 1.00 | 35.35 | C |
| ATOM | 4865 | O | LYS | C | 211 | -43.646 | -59.778 | 117.840 | 1.00 | 31.15 | O |
| ATOM | 4866 | CB | LYS | C | 211 | -42.214 | -62.112 | 116.591 | 1.00 | 30.51 | C |
| ATOM | 4867 | CG | LYS | C | 211 | -41.695 | -62.682 | 117.906 | 1.00 | 29.62 | C |
| ATOM | 4868 | CD | LYS | C | 211 | -42.354 | -64.008 | 118.183 | 1.00 | 35.84 | C |
| ATOM | 4869 | CE | LYS | C | 211 | -41.473 | -64.913 | 119.014 | 1.00 | 40.39 | C |
| ATOM | 4870 | NZ | LYS | C | 211 | -41.983 | -66.295 | 118.890 | 1.00 | 39.30 | N1+ |
| ATOM | 4871 | N | LYS | C | 212 | -44.971 | -61.439 | 118.608 | 1.00 | 33.29 | N |
| ATOM | 4872 | CA | LYS | C | 212 | -45.256 | -60.779 | 119.867 | 1.00 | 29.95 | C |
| ATOM | 4873 | C | LYS | C | 212 | -44.162 | -61.099 | 120.869 | 1.00 | 33.39 | C |
| ATOM | 4874 | O | LYS | C | 212 | -43.713 | -62.248 | 120.985 | 1.00 | 41.06 | O |
| ATOM | 4875 | CB | LYS | C | 212 | -46.621 | -61.213 | 120.394 | 1.00 | 33.65 | C |
| ATOM | 4876 | CG | LYS | C | 212 | -47.235 | -60.285 | 121.413 | 1.00 | 32.68 | C |
| ATOM | 4877 | CD | LYS | C | 212 | -48.491 | -60.893 | 122.040 | 1.00 | 34.32 | C |
| ATOM | 4878 | CE | LYS | C | 212 | -49.020 | -59.999 | 123.150 | 1.00 | 34.90 | C |
| ATOM | 4879 | NZ | LYS | C | 212 | -50.184 | -60.548 | 123.882 | 1.00 | 52.78 | N1+ |
| ATOM | 4880 | N | VAL | C | 213 | -43.742 | -60.078 | 121.602 | 1.00 | 29.81 | N |
| ATOM | 4881 | CA | VAL | C | 213 | -42.705 | -60.195 | 122.612 | 1.00 | 28.67 | C |
| ATOM | 4882 | C | VAL | C | 213 | -43.332 | -59.803 | 123.948 | 1.00 | 35.48 | C |
| ATOM | 4883 | O | VAL | C | 213 | -43.668 | -58.634 | 124.170 | 1.00 | 34.53 | O |
| ATOM | 4884 | CB | VAL | C | 213 | -41.493 | -59.313 | 122.281 | 1.00 | 34.29 | C |
| ATOM | 4885 | CG1 | VAL | C | 213 | -40.355 | -59.481 | 123.311 | 1.00 | 29.32 | C |
| ATOM | 4886 | CG2 | VAL | C | 213 | -41.022 | -59.563 | 120.861 | 1.00 | 28.07 | C |
| ATOM | 4887 | N | GLU | C | 214 | -43.503 | -60.781 | 124.833 | 1.00 | 43.40 | N |
| ATOM | 4888 | CA | GLU | C | 214 | -44.067 | -60.573 | 126.149 | 1.00 | 43.27 | C |
| ATOM | 4889 | C | GLU | C | 214 | -42.990 | -60.735 | 127.209 | 1.00 | 49.50 | C |
| ATOM | 4890 | O | GLU | C | 214 | -42.011 | -61.461 | 126.997 | 1.00 | 50.46 | O |
| ATOM | 4891 | CB | GLU | C | 214 | -45.206 | -61.558 | 126.434 | 1.00 | 48.02 | C |
| ATOM | 4892 | CG | GLU | C | 214 | -46.365 | -61.449 | 125.439 | 1.00 | 53.85 | C |
| ATOM | 4893 | CD | GLU | C | 214 | -47.489 | -62.450 | 125.686 | 1.00 | 57.28 | C |
| ATOM | 4894 | OE1 | GLU | C | 214 | -48.525 | -62.026 | 126.249 | 1.00 | 58.32 | O |
| ATOM | 4895 | OE2 | GLU | C | 214 | -47.343 | -63.644 | 125.318 | 1.00 | 60.40 | O1- |

| | | | | | | | | | | | |
|---|---|---|---|---|---|---|---|---|---|---|---|
| ATOM | 4896 | N | PRO | C | 215 | -43.109 | -60.039 | 128.338 | 1.00 | 50.15 | N |
| ATOM | 4897 | CA | PRO | C | 215 | -42.182 | -60.293 | 129.452 | 1.00 | 50.10 | C |
| ATOM | 4898 | C | PRO | C | 215 | -42.336 | -61.727 | 129.928 | 1.00 | 54.89 | C |
| ATOM | 4899 | O | PRO | C | 215 | -43.450 | -62.225 | 130.077 | 1.00 | 62.80 | O |
| ATOM | 4900 | CB | PRO | C | 215 | -42.606 | -59.277 | 130.515 | 1.00 | 49.74 | C |
| ATOM | 4901 | CG | PRO | C | 215 | -43.879 | -58.612 | 129.977 | 1.00 | 51.15 | C |
| ATOM | 4902 | CD | PRO | C | 215 | -43.892 | -58.805 | 128.515 | 1.00 | 47.22 | C |
| ATOM | 4903 | N | LYS | C | 216 | -41.208 | -62.402 | 130.137 | 1.00 | 59.54 | N |
| ATOM | 4904 | CA | LYS | C | 216 | -41.205 | -63.833 | 130.410 | 1.00 | 68.05 | C |
| ATOM | 4905 | C | LYS | C | 216 | -40.699 | -64.127 | 131.820 | 1.00 | 79.82 | C |
| ATOM | 4906 | O | LYS | C | 216 | -39.755 | -63.482 | 132.304 | 1.00 | 68.19 | O |
| ATOM | 4907 | CB | LYS | C | 216 | -40.371 | -64.581 | 129.359 | 1.00 | 67.81 | C |
| ATOM | 4908 | CG | LYS | C | 216 | -40.384 | -66.093 | 129.519 | 1.00 | 71.95 | C |
| ATOM | 4909 | CD | LYS | C | 216 | -39.957 | -66.793 | 128.233 | 1.00 | 73.44 | C |
| ATOM | 4910 | CE | LYS | C | 216 | -40.151 | -68.301 | 128.345 | 1.00 | 66.41 | C |
| ATOM | 4911 | NZ | LYS | C | 216 | -40.055 | -68.966 | 127.016 | 1.00 | 77.32 | N1+ |
| ATOM | 4912 | N | SER | C | 217 | -41.360 | -65.090 | 132.479 | 1.00 | 86.12 | N |
| ATOM | 4913 | CA | SER | C | 217 | -41.015 | -65.548 | 133.838 | 1.00 | 92.36 | C |
| ATOM | 4914 | C | SER | C | 217 | -40.297 | -66.901 | 133.836 | 1.00 | 83.50 | C |
| ATOM | 4915 | O | SER | C | 217 | -40.341 | -67.644 | 132.851 | 1.00 | 85.79 | O |
| ATOM | 4916 | CB | SER | C | 217 | -42.273 | -65.660 | 134.707 | 1.00 | 84.83 | C |
| ATOM | 4917 | OG | SER | C | 217 | -43.144 | -66.656 | 134.191 | 1.00 | 75.85 | O |
| TER | | | | | | | | | | | |
| ATOM | 4918 | N | GLU | D | 1 | -2.709 | -44.362 | 88.539 | 1.00 | 46.38 | N |
| ATOM | 4919 | CA | GLU | D | 1 | -3.927 | -43.604 | 88.796 | 1.00 | 40.18 | C |
| ATOM | 4920 | C | GLU | D | 1 | -3.692 | -42.543 | 89.882 | 1.00 | 40.43 | C |
| ATOM | 4921 | O | GLU | D | 1 | -2.818 | -42.704 | 90.730 | 1.00 | 41.89 | O |
| ATOM | 4922 | CB | GLU | D | 1 | -5.056 | -44.548 | 89.205 | 1.00 | 33.14 | C |
| ATOM | 4923 | CG | GLU | D | 1 | -5.071 | -44.860 | 90.693 | 1.00 | 34.96 | C |
| ATOM | 4924 | CD | GLU | D | 1 | -6.026 | -45.981 | 91.081 | 1.00 | 47.71 | C |
| ATOM | 4925 | OE1 | GLU | D | 1 | -6.745 | -46.523 | 90.207 | 1.00 | 56.37 | O |
| ATOM | 4926 | OE2 | GLU | D | 1 | -6.060 | -46.324 | 92.281 | 1.00 | 57.65 | O1- |
| ATOM | 4927 | N | ILE | D | 2 | -4.482 | -41.470 | 89.840 | 1.00 | 35.77 | N |
| ATOM | 4928 | CA | ILE | D | 2 | -4.431 | -40.417 | 90.844 | 1.00 | 30.01 | C |
| ATOM | 4929 | C | ILE | D | 2 | -5.055 | -40.916 | 92.141 | 1.00 | 34.99 | C |
| ATOM | 4930 | O | ILE | D | 2 | -6.246 | -41.247 | 92.184 | 1.00 | 35.20 | O |
| ATOM | 4931 | CB | ILE | D | 2 | -5.147 | -39.166 | 90.336 | 1.00 | 32.58 | C |
| ATOM | 4932 | CG1 | ILE | D | 2 | -4.425 | -38.645 | 89.088 | 1.00 | 29.66 | C |
| ATOM | 4933 | CG2 | ILE | D | 2 | -5.297 | -38.133 | 91.458 | 1.00 | 29.46 | C |
| ATOM | 4934 | CD1 | ILE | D | 2 | -5.026 | -37.400 | 88.488 | 1.00 | 28.66 | C |
| ATOM | 4935 | N | VAL | D | 3 | -4.263 | -40.933 | 93.219 | 1.00 | 34.76 | N |
| ATOM | 4936 | CA | VAL | D | 3 | -4.726 | -41.369 | 94.533 | 1.00 | 30.38 | C |
| ATOM | 4937 | C | VAL | D | 3 | -5.153 | -40.143 | 95.325 | 1.00 | 31.44 | C |
| ATOM | 4938 | O | VAL | D | 3 | -4.409 | -39.155 | 95.403 | 1.00 | 31.03 | O |
| ATOM | 4939 | CB | VAL | D | 3 | -3.641 | -42.166 | 95.278 | 1.00 | 29.88 | C |
| ATOM | 4940 | CG1 | VAL | D | 3 | -4.089 | -42.450 | 96.695 | 1.00 | 25.14 | C |
| ATOM | 4941 | CG2 | VAL | D | 3 | -3.355 | -43.479 | 94.565 | 1.00 | 22.24 | C |
| ATOM | 4942 | N | LEU | D | 4 | -6.379 | -40.174 | 95.851 | 1.00 | 30.04 | N |
| ATOM | 4943 | CA | LEU | D | 4 | -6.942 | -39.055 | 96.604 | 1.00 | 28.75 | C |
| ATOM | 4944 | C | LEU | D | 4 | -7.087 | -39.490 | 98.050 | 1.00 | 29.11 | C |
| ATOM | 4945 | O | LEU | D | 4 | -7.809 | -40.450 | 98.337 | 1.00 | 37.17 | O |
| ATOM | 4946 | CB | LEU | D | 4 | -8.303 | -38.607 | 96.057 | 1.00 | 27.56 | C |
| ATOM | 4947 | CG | LEU | D | 4 | -8.478 | -38.095 | 94.619 | 1.00 | 27.56 | C |
| ATOM | 4948 | CD1 | LEU | D | 4 | -9.887 | -37.577 | 94.388 | 1.00 | 23.55 | C |
| ATOM | 4949 | CD2 | LEU | D | 4 | -7.477 | -37.038 | 94.270 | 1.00 | 27.03 | C |
| ATOM | 4950 | N | THR | D | 5 | -6.414 | -38.795 | 98.956 | 1.00 | 27.89 | N |
| ATOM | 4951 | CA | THR | D | 5 | -6.535 | -39.071 | 100.382 | 1.00 | 27.95 | C |
| ATOM | 4952 | C | THR | D | 5 | -7.291 | -37.915 | 101.020 | 1.00 | 27.80 | C |
| ATOM | 4953 | O | THR | D | 5 | -6.899 | -36.752 | 100.880 | 1.00 | 29.84 | O |
| ATOM | 4954 | CB | THR | D | 5 | -5.180 | -39.257 | 101.067 | 1.00 | 25.13 | C |
| ATOM | 4955 | OG1 | THR | D | 5 | -4.610 | -37.975 | 101.281 | 1.00 | 44.55 | O |
| ATOM | 4956 | CG2 | THR | D | 5 | -4.229 | -40.091 | 100.229 | 1.00 | 22.72 | C |
| ATOM | 4957 | N | GLN | D | 6 | -8.379 | -38.229 | 101.693 | 1.00 | 26.25 | N |
| ATOM | 4958 | CA | GLN | D | 6 | -9.154 | -37.231 | 102.401 | 1.00 | 29.68 | C |
| ATOM | 4959 | C | GLN | D | 6 | -8.770 | -37.227 | 103.870 | 1.00 | 29.42 | C |
| ATOM | 4960 | O | GLN | D | 6 | -8.596 | -38.287 | 104.475 | 1.00 | 30.59 | O |
| ATOM | 4961 | CB | GLN | D | 6 | -10.645 | -37.515 | 102.260 | 1.00 | 28.59 | C |
| ATOM | 4962 | CG | GLN | D | 6 | -11.199 | -37.068 | 100.946 | 1.00 | 26.38 | C |
| ATOM | 4963 | CD | GLN | D | 6 | -12.674 | -37.347 | 100.825 | 1.00 | 28.44 | C |
| ATOM | 4964 | OE1 | GLN | D | 6 | -13.063 | -38.310 | 100.182 | 1.00 | 30.52 | O |
| ATOM | 4965 | NE2 | GLN | D | 6 | -13.507 | -36.497 | 101.430 | 1.00 | 23.50 | N |

| | | | | | | | | | | | |
|---|---|---|---|---|---|---|---|---|---|---|---|
| ATOM | 4966 | N | SER | D | 7 | -8.646 | -36.032 | 104.442 | 1.00 | 32.95 | N |
| ATOM | 4967 | CA | SER | D | 7 | -8.474 | -35.923 | 105.893 | 1.00 | 34.15 | C |
| ATOM | 4968 | C | SER | D | 7 | -9.305 | -34.779 | 106.507 | 1.00 | 36.23 | C |
| ATOM | 4969 | O | SER | D | 7 | -9.692 | -33.818 | 105.822 | 1.00 | 34.31 | O |
| ATOM | 4970 | CB | SER | D | 7 | -7.007 | -35.732 | 106.234 | 1.00 | 32.05 | C |
| ATOM | 4971 | OG | SER | D | 7 | -6.622 | -34.406 | 105.965 | 1.00 | 36.70 | O |
| ATOM | 4972 | N | PRO | D | 8 | -9.657 | -34.917 | 107.791 | 1.00 | 35.26 | N |
| ATOM | 4973 | CA | PRO | D | 8 | -9.493 | -36.114 | 108.627 | 1.00 | 31.68 | C |
| ATOM | 4974 | C | PRO | D | 8 | -10.479 | -37.209 | 108.218 | 1.00 | 35.24 | C |
| ATOM | 4975 | O | PRO | D | 8 | -11.338 | -36.963 | 107.380 | 1.00 | 34.44 | O |
| ATOM | 4976 | CB | PRO | D | 8 | -9.812 | -35.599 | 110.022 | 1.00 | 31.95 | C |
| ATOM | 4977 | CG | PRO | D | 8 | -10.849 | -34.538 | 109.772 | 1.00 | 33.10 | C |
| ATOM | 4978 | CD | PRO | D | 8 | -10.446 | -33.874 | 108.471 | 1.00 | 32.84 | C |
| ATOM | 4979 | N | GLY | D | 9 | -10.345 | -38.409 | 108.776 | 1.00 | 36.77 | N |
| ATOM | 4980 | CA | GLY | D | 9 | -11.322 | -39.448 | 108.496 | 1.00 | 26.26 | C |
| ATOM | 4981 | C | GLY | D | 9 | -12.671 | -39.170 | 109.129 | 1.00 | 26.81 | C |
| ATOM | 4982 | O | GLY | D | 9 | -13.706 | -39.472 | 108.541 | 1.00 | 29.93 | O |
| ATOM | 4983 | N | THR | D | 10 | -12.681 | -38.619 | 110.349 | 1.00 | 29.94 | N |
| ATOM | 4984 | CA | THR | D | 10 | -13.909 | -38.207 | 111.025 | 1.00 | 29.91 | C |
| ATOM | 4985 | C | THR | D | 10 | -13.748 | -36.800 | 111.602 | 1.00 | 35.08 | C |
| ATOM | 4986 | O | THR | D | 10 | -12.672 | -36.421 | 112.072 | 1.00 | 37.12 | O |
| ATOM | 4987 | CB | THR | D | 10 | -14.324 | -39.175 | 112.138 | 1.00 | 28.55 | C |
| ATOM | 4988 | OG1 | THR | D | 10 | -14.423 | -40.503 | 111.612 | 1.00 | 29.83 | O |
| ATOM | 4989 | CG2 | THR | D | 10 | -15.682 | -38.775 | 112.712 | 1.00 | 26.70 | C |
| ATOM | 4990 | N | LEU | D | 11 | -14.823 | -36.022 | 111.527 | 1.00 | 33.72 | N |
| ATOM | 4991 | CA | LEU | D | 11 | -14.874 | -34.646 | 111.997 | 1.00 | 30.12 | C |
| ATOM | 4992 | C | LEU | D | 11 | -16.092 | -34.516 | 112.895 | 1.00 | 32.24 | C |
| ATOM | 4993 | O | LEU | D | 11 | -17.220 | -34.659 | 112.407 | 1.00 | 35.42 | O |
| ATOM | 4994 | CB | LEU | D | 11 | -15.010 | -33.702 | 110.811 | 1.00 | 31.05 | C |
| ATOM | 4995 | CG | LEU | D | 11 | -14.043 | -32.578 | 110.566 | 1.00 | 35.03 | C |
| ATOM | 4996 | CD1 | LEU | D | 11 | -14.736 | -31.626 | 109.611 | 1.00 | 32.51 | C |
| ATOM | 4997 | CD2 | LEU | D | 11 | -13.741 | -31.926 | 111.890 | 1.00 | 32.63 | C |
| ATOM | 4998 | N | SER | D | 12 | -15.880 | -34.220 | 114.183 | 1.00 | 27.22 | N |
| ATOM | 4999 | CA | SER | D | 12 | -16.969 | -34.041 | 115.149 | 1.00 | 31.54 | C |
| ATOM | 5000 | C | SER | D | 12 | -17.136 | -32.563 | 115.466 | 1.00 | 26.28 | C |
| ATOM | 5001 | O | SER | D | 12 | -16.200 | -31.925 | 115.940 | 1.00 | 31.22 | O |
| ATOM | 5002 | CB | SER | D | 12 | -16.711 | -34.815 | 116.443 | 1.00 | 30.19 | C |
| ATOM | 5003 | OG | SER | D | 12 | -16.413 | -36.172 | 116.169 | 1.00 | 30.39 | O |
| ATOM | 5004 | N | LEU | D | 13 | -18.320 | -32.023 | 115.209 | 1.00 | 28.43 | N |
| ATOM | 5005 | CA | LEU | D | 13 | -18.535 | -30.588 | 115.335 | 1.00 | 32.40 | C |
| ATOM | 5006 | C | LEU | D | 13 | -19.965 | -30.311 | 115.779 | 1.00 | 32.08 | C |
| ATOM | 5007 | O | LEU | D | 13 | -20.889 | -31.062 | 115.463 | 1.00 | 33.15 | O |
| ATOM | 5008 | CB | LEU | D | 13 | -18.229 | -29.860 | 114.013 | 1.00 | 29.60 | C |
| ATOM | 5009 | CG | LEU | D | 13 | -16.792 | -29.909 | 113.449 | 1.00 | 31.08 | C |
| ATOM | 5010 | CD1 | LEU | D | 13 | -16.740 | -29.357 | 112.045 | 1.00 | 34.57 | C |
| ATOM | 5011 | CD2 | LEU | D | 13 | -15.818 | -29.128 | 114.313 | 1.00 | 19.13 | C |
| ATOM | 5012 | N | SER | D | 14 | -20.148 | -29.201 | 116.468 | 1.00 | 33.43 | N |
| ATOM | 5013 | CA | SER | D | 14 | -21.515 | -28.841 | 116.784 | 1.00 | 34.69 | C |
| ATOM | 5014 | C | SER | D | 14 | -22.184 | -28.181 | 115.589 | 1.00 | 30.63 | C |
| ATOM | 5015 | O | SER | D | 14 | -21.528 | -27.507 | 114.789 | 1.00 | 26.53 | O |
| ATOM | 5016 | CB | SER | D | 14 | -21.563 | -27.869 | 117.963 | 1.00 | 40.13 | C |
| ATOM | 5017 | OG | SER | D | 14 | -21.315 | -28.526 | 119.189 | 1.00 | 49.17 | O |
| ATOM | 5018 | N | PRO | D | 15 | -23.496 | -28.324 | 115.471 | 1.00 | 30.71 | N |
| ATOM | 5019 | CA | PRO | D | 15 | -24.231 | -27.513 | 114.495 | 1.00 | 30.71 | C |
| ATOM | 5020 | C | PRO | D | 15 | -23.981 | -26.030 | 114.753 | 1.00 | 33.52 | C |
| ATOM | 5021 | O | PRO | D | 15 | -23.778 | -25.598 | 115.892 | 1.00 | 30.44 | O |
| ATOM | 5022 | CB | PRO | D | 15 | -25.694 | -27.907 | 114.732 | 1.00 | 29.82 | C |
| ATOM | 5023 | CG | PRO | D | 15 | -25.633 | -29.250 | 115.357 | 1.00 | 27.19 | C |
| ATOM | 5024 | CD | PRO | D | 15 | -24.368 | -29.263 | 116.188 | 1.00 | 28.99 | C |
| ATOM | 5025 | N | GLY | D | 16 | -23.917 | -25.264 | 113.673 | 1.00 | 34.76 | N |
| ATOM | 5026 | CA | GLY | D | 16 | -23.561 | -23.872 | 113.725 | 1.00 | 30.06 | C |
| ATOM | 5027 | C | GLY | D | 16 | -22.093 | -23.615 | 113.489 | 1.00 | 32.57 | C |
| ATOM | 5028 | O | GLY | D | 16 | -21.728 | -22.532 | 113.024 | 1.00 | 34.09 | O |
| ATOM | 5029 | N | GLU | D | 17 | -21.243 | -24.599 | 113.748 | 1.00 | 30.04 | N |
| ATOM | 5030 | CA | GLU | D | 17 | -19.828 | -24.365 | 113.578 | 1.00 | 29.50 | C |
| ATOM | 5031 | C | GLU | D | 17 | -19.442 | -24.466 | 112.111 | 1.00 | 33.26 | C |
| ATOM | 5032 | O | GLU | D | 17 | -20.236 | -24.848 | 111.242 | 1.00 | 28.54 | O |
| ATOM | 5033 | CB | GLU | D | 17 | -18.997 | -25.365 | 114.370 | 1.00 | 30.61 | C |
| ATOM | 5034 | CG | GLU | D | 17 | -18.967 | -25.187 | 115.863 | 1.00 | 38.28 | C |
| ATOM | 5035 | CD | GLU | D | 17 | -17.955 | -26.152 | 116.491 | 1.00 | 47.48 | C |
| ATOM | 5036 | OE1 | GLU | D | 17 | -16.740 | -25.933 | 116.281 | 1.00 | 51.97 | O |

| | | | | | | | | | | | |
|---|---|---|---|---|---|---|---|---|---|---|---|
| ATOM | 5037 | OE2 | GLU | D | 17 | -18.365 | -27.154 | 117.143 | 1.00 | 47.45 | O1- |
| ATOM | 5038 | N | ARG | D | 18 | -18.183 | -24.132 | 111.861 | 1.00 | 33.39 | N |
| ATOM | 5039 | CA | ARG | D | 18 | -17.585 | -24.128 | 110.544 | 1.00 | 30.46 | C |
| ATOM | 5040 | C | ARG | D | 18 | -16.823 | -25.439 | 110.354 | 1.00 | 35.01 | C |
| ATOM | 5041 | O | ARG | D | 18 | -16.148 | -25.911 | 111.273 | 1.00 | 36.75 | O |
| ATOM | 5042 | CB | ARG | D | 18 | -16.673 | -22.903 | 110.427 | 1.00 | 30.90 | C |
| ATOM | 5043 | CG | ARG | D | 18 | -15.935 | -22.722 | 109.136 | 1.00 | 33.87 | C |
| ATOM | 5044 | CD | ARG | D | 18 | -14.967 | -21.575 | 109.278 | 1.00 | 28.90 | C |
| ATOM | 5045 | NE | ARG | D | 18 | -14.168 | -21.350 | 108.073 | 1.00 | 42.50 | N |
| ATOM | 5046 | CZ | ARG | D | 18 | -14.603 | -20.689 | 106.995 | 1.00 | 46.47 | C |
| ATOM | 5047 | NH1 | ARG | D | 18 | -15.851 | -20.199 | 106.968 | 1.00 | 39.59 | N1+ |
| ATOM | 5048 | NH2 | ARG | D | 18 | -13.799 | -20.529 | 105.939 | 1.00 | 36.96 | N |
| ATOM | 5049 | N | ALA | D | 19 | -16.955 | -26.046 | 109.172 | 1.00 | 33.54 | N |
| ATOM | 5050 | CA | ALA | D | 19 | -16.302 | -27.317 | 108.864 | 1.00 | 31.96 | C |
| ATOM | 5051 | C | ALA | D | 19 | -15.394 | -27.131 | 107.661 | 1.00 | 27.02 | C |
| ATOM | 5052 | O | ALA | D | 19 | -15.760 | -26.443 | 106.707 | 1.00 | 28.38 | O |
| ATOM | 5053 | CB | ALA | D | 19 | -17.319 | -28.428 | 108.564 | 1.00 | 30.29 | C |
| ATOM | 5054 | N | THR | D | 20 | -14.203 | -27.715 | 107.720 | 1.00 | 24.79 | N |
| ATOM | 5055 | CA | THR | D | 20 | -13.250 | -27.647 | 106.619 | 1.00 | 25.95 | C |
| ATOM | 5056 | C | THR | D | 20 | -12.657 | -29.031 | 106.370 | 1.00 | 28.87 | C |
| ATOM | 5057 | O | THR | D | 20 | -12.056 | -29.619 | 107.272 | 1.00 | 27.37 | O |
| ATOM | 5058 | CB | THR | D | 20 | -12.160 | -26.618 | 106.920 | 1.00 | 33.57 | C |
| ATOM | 5059 | OG1 | THR | D | 20 | -12.678 | -25.302 | 106.669 | 1.00 | 35.59 | O |
| ATOM | 5060 | CG2 | THR | D | 20 | -10.942 | -26.842 | 106.026 | 1.00 | 35.30 | C |
| ATOM | 5061 | N | LEU | D | 21 | -12.845 | -29.553 | 105.153 | 1.00 | 27.92 | N |
| ATOM | 5062 | CA | LEU | D | 21 | -12.408 | -30.885 | 104.753 | 1.00 | 21.26 | C |
| ATOM | 5063 | C | LEU | D | 21 | -11.291 | -30.788 | 103.721 | 1.00 | 27.12 | C |
| ATOM | 5064 | O | LEU | D | 21 | -11.276 | -29.891 | 102.879 | 1.00 | 25.65 | O |
| ATOM | 5065 | CB | LEU | D | 21 | -13.567 | -31.688 | 104.171 | 1.00 | 24.71 | C |
| ATOM | 5066 | CG | LEU | D | 21 | -14.800 | -31.873 | 105.044 | 1.00 | 24.88 | C |
| ATOM | 5067 | CD1 | LEU | D | 21 | -15.745 | -32.804 | 104.393 | 1.00 | 25.75 | C |
| ATOM | 5068 | CD2 | LEU | D | 21 | -14.358 | -32.453 | 106.324 | 1.00 | 36.95 | C |
| ATOM | 5069 | N | SER | D | 22 | -10.364 | -31.732 | 103.777 | 1.00 | 25.30 | N |
| ATOM | 5070 | CA | SER | D | 22 | -9.207 | -31.718 | 102.906 | 1.00 | 29.86 | C |
| ATOM | 5071 | C | SER | D | 22 | -9.228 | -32.907 | 101.960 | 1.00 | 31.55 | C |
| ATOM | 5072 | O | SER | D | 22 | -9.637 | -34.010 | 102.338 | 1.00 | 32.22 | O |
| ATOM | 5073 | CB | SER | D | 22 | -7.908 | -31.743 | 103.711 | 1.00 | 29.48 | C |
| ATOM | 5074 | OG | SER | D | 22 | -7.484 | -30.430 | 103.983 | 1.00 | 48.01 | O |
| ATOM | 5075 | N | CYS | D | 23 | -8.748 | -32.668 | 100.743 | 1.00 | 23.08 | N |
| ATOM | 5076 | CA | CYS | D | 23 | -8.493 | -33.696 | 99.746 | 1.00 | 29.55 | C |
| ATOM | 5077 | C | CYS | D | 23 | -7.132 | -33.425 | 99.133 | 1.00 | 28.60 | C |
| ATOM | 5078 | O | CYS | D | 23 | -6.927 | -32.375 | 98.510 | 1.00 | 28.22 | O |
| ATOM | 5079 | CB | CYS | D | 23 | -9.577 | -33.692 | 98.661 | 1.00 | 32.45 | C |
| ATOM | 5080 | SG | CYS | D | 23 | -9.448 | -34.937 | 97.349 | 1.00 | 34.53 | S |
| ATOM | 5081 | N | ARG | D | 24 | -6.215 | -34.370 | 99.282 | 1.00 | 27.16 | N |
| ATOM | 5082 | CA | ARG | D | 24 | -4.896 | -34.261 | 98.682 | 1.00 | 30.26 | C |
| ATOM | 5083 | C | ARG | D | 24 | -4.755 | -35.243 | 97.517 | 1.00 | 29.93 | C |
| ATOM | 5084 | O | ARG | D | 24 | -5.021 | -36.442 | 97.663 | 1.00 | 32.04 | O |
| ATOM | 5085 | CB | ARG | D | 24 | -3.830 | -34.452 | 99.759 | 1.00 | 30.70 | C |
| ATOM | 5086 | CG | ARG | D | 24 | -3.797 | -33.212 | 100.656 | 1.00 | 40.46 | C |
| ATOM | 5087 | CD | ARG | D | 24 | -3.107 | -33.392 | 101.998 | 1.00 | 47.57 | C |
| ATOM | 5088 | NE | ARG | D | 24 | -2.760 | -32.094 | 102.596 | 1.00 | 64.41 | N |
| ATOM | 5089 | CZ | ARG | D | 24 | -3.482 | -31.436 | 103.512 | 1.00 | 65.33 | C |
| ATOM | 5090 | NH1 | ARG | D | 24 | -4.626 | -31.944 | 103.967 | 1.00 | 59.49 | N1+ |
| ATOM | 5091 | NH2 | ARG | D | 24 | -3.054 | -30.259 | 103.979 | 1.00 | 59.83 | N |
| ATOM | 5092 | N | ALA | D | 25 | -4.379 | -34.715 | 96.351 | 1.00 | 25.59 | N |
| ATOM | 5093 | CA | ALA | D | 25 | -4.224 | -35.487 | 95.126 | 1.00 | 27.28 | C |
| ATOM | 5094 | C | ALA | D | 25 | -2.745 | -35.776 | 94.882 | 1.00 | 29.22 | C |
| ATOM | 5095 | O | ALA | D | 25 | -1.914 | -34.868 | 94.943 | 1.00 | 32.95 | O |
| ATOM | 5096 | CB | ALA | D | 25 | -4.810 | -34.736 | 93.927 | 1.00 | 21.36 | C |
| ATOM | 5097 | N | SER | D | 26 | -2.428 | -37.029 | 94.593 | 1.00 | 26.23 | N |
| ATOM | 5098 | CA | SER | D | 26 | -1.100 | -37.425 | 94.171 | 1.00 | 26.45 | C |
| ATOM | 5099 | C | SER | D | 26 | -1.179 | -38.425 | 93.014 | 1.00 | 36.59 | C |
| ATOM | 5100 | O | SER | D | 26 | -1.636 | -39.568 | 93.195 | 1.00 | 38.09 | O |
| ATOM | 5101 | CB | SER | D | 26 | -0.330 | -38.048 | 95.323 | 1.00 | 31.96 | C |
| ATOM | 5102 | OG | SER | D | 26 | 0.947 | -38.468 | 94.888 | 1.00 | 40.33 | O |
| ATOM | 5103 | N | PRO | D | 27 | -0.729 | -38.019 | 91.817 | 1.00 | 33.93 | N |
| ATOM | 5104 | CA | PRO | D | 27 | -0.176 | -36.725 | 91.413 | 1.00 | 32.59 | C |
| ATOM | 5105 | C | PRO | D | 27 | -1.180 | -35.587 | 91.464 | 1.00 | 31.97 | C |
| ATOM | 5106 | O | PRO | D | 27 | -2.361 | -35.800 | 91.744 | 1.00 | 29.04 | O |
| ATOM | 5107 | CB | PRO | D | 27 | 0.255 | -36.957 | 89.967 | 1.00 | 25.77 | C |

| | | | | | | | | | | | |
|---|---|---|---|---|---|---|---|---|---|---|---|
| ATOM | 5108 | CG | PRO | D | 27 | 0.295 | -38.395 | 89.799 | 1.00 | 30.50 | C |
| ATOM | 5109 | CD | PRO | D | 27 | -0.695 | -38.986 | 90.712 | 1.00 | 33.60 | C |
| ATOM | 5110 | N | SER | D | 28 | -0.691 | -34.384 | 91.176 | 1.00 | 29.51 | N |
| ATOM | 5111 | CA | SER | D | 28 | -1.531 | -33.204 | 91.247 | 1.00 | 33.06 | C |
| ATOM | 5112 | C | SER | D | 28 | -2.689 | -33.306 | 90.258 | 1.00 | 31.21 | C |
| ATOM | 5113 | O | SER | D | 28 | -2.658 | -34.064 | 89.292 | 1.00 | 32.08 | O |
| ATOM | 5114 | CB | SER | D | 28 | -0.708 | -31.948 | 90.968 | 1.00 | 30.39 | C |
| ATOM | 5115 | OG | SER | D | 28 | 0.122 | -31.650 | 92.075 | 1.00 | 39.06 | O |
| ATOM | 5116 | N | VAL | D | 29 | -3.741 | -32.552 | 90.537 | 1.00 | 31.26 | N |
| ATOM | 5117 | CA | VAL | D | 29 | -4.834 | -32.398 | 89.589 | 1.00 | 31.02 | C |
| ATOM | 5118 | C | VAL | D | 29 | -4.828 | -30.937 | 89.154 | 1.00 | 30.74 | C |
| ATOM | 5119 | O | VAL | D | 29 | -5.671 | -30.137 | 89.581 | 1.00 | 32.15 | O |
| ATOM | 5120 | CB | VAL | D | 29 | -6.180 | -32.849 | 90.199 | 1.00 | 28.79 | C |
| ATOM | 5121 | CG1 | VAL | D | 29 | -7.331 | -32.660 | 89.220 | 1.00 | 29.57 | C |
| ATOM | 5122 | CG2 | VAL | D | 29 | -6.092 | -34.296 | 90.584 | 1.00 | 28.20 | C |
| ATOM | 5123 | N | ASN | D | 30 | -3.867 | -30.579 | 88.300 | 1.00 | 29.06 | N |
| ATOM | 5124 | CA | ASN | D | 30 | -3.706 | -29.191 | 87.881 | 1.00 | 27.78 | C |
| ATOM | 5125 | C | ASN | D | 30 | -4.829 | -28.695 | 86.974 | 1.00 | 28.98 | C |
| ATOM | 5126 | O | ASN | D | 30 | -4.903 | -27.494 | 86.715 | 1.00 | 34.22 | O |
| ATOM | 5127 | CB | ASN | D | 30 | -2.371 | -29.012 | 87.165 | 1.00 | 25.95 | C |
| ATOM | 5128 | CG | ASN | D | 30 | -1.177 | -29.247 | 88.072 | 1.00 | 29.67 | C |
| ATOM | 5129 | OD1 | ASN | D | 30 | -1.142 | -28.823 | 89.232 | 1.00 | 38.01 | O |
| ATOM | 5130 | ND2 | ASN | D | 30 | -0.188 | -29.923 | 87.546 | 1.00 | 31.76 | N |
| ATOM | 5131 | N | SER | D | 31 | -5.715 | -29.565 | 86.496 | 1.00 | 32.77 | N |
| ATOM | 5132 | CA | SER | D | 31 | -6.854 | -29.076 | 85.733 | 1.00 | 29.44 | C |
| ATOM | 5133 | C | SER | D | 31 | -7.919 | -28.456 | 86.623 | 1.00 | 29.07 | C |
| ATOM | 5134 | O | SER | D | 31 | -8.807 | -27.763 | 86.115 | 1.00 | 30.00 | O |
| ATOM | 5135 | CB | SER | D | 31 | -7.480 | -30.207 | 84.911 | 1.00 | 30.70 | C |
| ATOM | 5136 | OG | SER | D | 31 | -7.863 | -31.300 | 85.728 | 1.00 | 28.68 | O |
| ATOM | 5137 | N | GLY | D | 32 | -7.865 | -28.708 | 87.928 | 1.00 | 26.73 | N |
| ATOM | 5138 | CA | GLY | D | 32 | -8.956 | -28.326 | 88.788 | 1.00 | 24.35 | C |
| ATOM | 5139 | C | GLY | D | 32 | -10.205 | -29.141 | 88.582 | 1.00 | 28.63 | C |
| ATOM | 5140 | O | GLY | D | 32 | -11.275 | -28.720 | 89.003 | 1.00 | 23.12 | O |
| ATOM | 5141 | N | TYR | D | 33 | -10.109 | -30.294 | 87.917 | 1.00 | 28.39 | N |
| ATOM | 5142 | CA | TYR | D | 33 | -11.280 | -31.144 | 87.695 | 1.00 | 27.10 | C |
| ATOM | 5143 | C | TYR | D | 33 | -11.513 | -31.989 | 88.947 | 1.00 | 25.88 | C |
| ATOM | 5144 | O | TYR | D | 33 | -11.254 | -33.194 | 88.991 | 1.00 | 26.06 | O |
| ATOM | 5145 | CB | TYR | D | 33 | -11.095 | -32.028 | 86.468 | 1.00 | 27.24 | C |
| ATOM | 5146 | CG | TYR | D | 33 | -11.026 | -31.318 | 85.121 | 1.00 | 30.30 | C |
| ATOM | 5147 | CD1 | TYR | D | 33 | -11.355 | -29.972 | 84.977 | 1.00 | 24.29 | C |
| ATOM | 5148 | CD2 | TYR | D | 33 | -10.625 | -32.014 | 83.988 | 1.00 | 29.73 | C |
| ATOM | 5149 | CE1 | TYR | D | 33 | -11.272 | -29.351 | 83.739 | 1.00 | 29.00 | C |
| ATOM | 5150 | CE2 | TYR | D | 33 | -10.535 | -31.403 | 82.757 | 1.00 | 27.95 | C |
| ATOM | 5151 | CZ | TYR | D | 33 | -10.851 | -30.083 | 82.626 | 1.00 | 29.77 | C |
| ATOM | 5152 | OH | TYR | D | 33 | -10.753 | -29.528 | 81.366 | 1.00 | 27.33 | O |
| ATOM | 5153 | N | LEU | D | 34 | -11.990 | -31.331 | 89.993 | 1.00 | 24.59 | N |
| ATOM | 5154 | CA | LEU | D | 34 | -12.197 | -32.008 | 91.264 | 1.00 | 22.23 | C |
| ATOM | 5155 | C | LEU | D | 34 | -13.626 | -31.783 | 91.730 | 1.00 | 22.58 | C |
| ATOM | 5156 | O | LEU | D | 34 | -14.082 | -30.644 | 91.811 | 1.00 | 30.13 | O |
| ATOM | 5157 | CB | LEU | D | 34 | -11.211 | -31.517 | 92.326 | 1.00 | 22.34 | C |
| ATOM | 5158 | CG | LEU | D | 34 | -11.174 | -32.540 | 93.472 | 1.00 | 25.73 | C |
| ATOM | 5159 | CD1 | LEU | D | 34 | -9.862 | -33.273 | 93.510 | 1.00 | 24.20 | C |
| ATOM | 5160 | CD2 | LEU | D | 34 | -11.517 | -31.936 | 94.803 | 1.00 | 22.15 | C |
| ATOM | 5161 | N | ALA | D | 35 | -14.331 | -32.856 | 92.019 | 1.00 | 19.87 | N |
| ATOM | 5162 | CA | ALA | D | 35 | -15.711 | -32.778 | 92.454 | 1.00 | 21.92 | C |
| ATOM | 5163 | C | ALA | D | 35 | -15.824 | -33.191 | 93.917 | 1.00 | 23.07 | C |
| ATOM | 5164 | O | ALA | D | 35 | -14.962 | -33.884 | 94.461 | 1.00 | 19.99 | O |
| ATOM | 5165 | CB | ALA | D | 35 | -16.614 | -33.657 | 91.593 | 1.00 | 18.13 | C |
| ATOM | 5166 | N | TRP | D | 36 | -16.908 | -32.743 | 94.544 | 1.00 | 22.34 | N |
| ATOM | 5167 | CA | TRP | D | 36 | -17.251 | -33.112 | 95.911 | 1.00 | 25.99 | C |
| ATOM | 5168 | C | TRP | D | 36 | -18.673 | -33.656 | 95.941 | 1.00 | 24.08 | C |
| ATOM | 5169 | O | TRP | D | 36 | -19.573 | -33.115 | 95.291 | 1.00 | 23.80 | O |
| ATOM | 5170 | CB | TRP | D | 36 | -17.138 | -31.920 | 96.890 | 1.00 | 24.75 | C |
| ATOM | 5171 | CG | TRP | D | 36 | -15.751 | -31.504 | 97.216 | 1.00 | 23.08 | C |
| ATOM | 5172 | CD1 | TRP | D | 36 | -15.013 | -30.555 | 96.564 | 1.00 | 27.42 | C |
| ATOM | 5173 | CD2 | TRP | D | 36 | -14.917 | -32.009 | 98.268 | 1.00 | 23.07 | C |
| ATOM | 5174 | NE1 | TRP | D | 36 | -13.774 | -30.439 | 97.142 | 1.00 | 26.35 | N |
| ATOM | 5175 | CE2 | TRP | D | 36 | -13.685 | -31.316 | 98.193 | 1.00 | 26.49 | C |
| ATOM | 5176 | CE3 | TRP | D | 36 | -15.096 | -32.959 | 99.275 | 1.00 | 23.59 | C |
| ATOM | 5177 | CZ2 | TRP | D | 36 | -12.630 | -31.546 | 99.092 | 1.00 | 24.68 | C |
| ATOM | 5178 | CZ3 | TRP | D | 36 | -14.049 | -33.189 | 100.167 | 1.00 | 27.30 | C |

| | | | | | | | | | | | |
|---|---|---|---|---|---|---|---|---|---|---|---|
| ATOM | 5179 | CH2 | TRP | D | 36 | -12.829 | -32.484 | 100.064 | 1.00 | 27.81 | C |
| ATOM | 5180 | N | TYR | D | 37 | -18.861 | -34.737 | 96.689 | 1.00 | 22.56 | N |
| ATOM | 5181 | CA | TYR | D | 37 | -20.166 | -35.347 | 96.873 | 1.00 | 23.04 | C |
| ATOM | 5182 | C | TYR | D | 37 | -20.507 | -35.457 | 98.354 | 1.00 | 24.32 | C |
| ATOM | 5183 | O | TYR | D | 37 | -19.638 | -35.621 | 99.213 | 1.00 | 21.76 | O |
| ATOM | 5184 | CB | TYR | D | 37 | -20.222 | -36.732 | 96.232 | 1.00 | 22.03 | C |
| ATOM | 5185 | CG | TYR | D | 37 | -19.937 | -36.707 | 94.768 | 1.00 | 22.72 | C |
| ATOM | 5186 | CD1 | TYR | D | 37 | -18.634 | -36.802 | 94.302 | 1.00 | 19.01 | C |
| ATOM | 5187 | CD2 | TYR | D | 37 | -20.965 | -36.569 | 93.840 | 1.00 | 22.13 | C |
| ATOM | 5188 | CE1 | TYR | D | 37 | -18.355 | -36.772 | 92.962 | 1.00 | 20.66 | C |
| ATOM | 5189 | CE2 | TYR | D | 37 | -20.690 | -36.541 | 92.487 | 1.00 | 21.78 | C |
| ATOM | 5190 | CZ | TYR | D | 37 | -19.378 | -36.642 | 92.056 | 1.00 | 23.03 | C |
| ATOM | 5191 | OH | TYR | D | 37 | -19.071 | -36.606 | 90.716 | 1.00 | 26.68 | O |
| ATOM | 5192 | N | GLN | D | 38 | -21.790 | -35.368 | 98.640 | 1.00 | 24.80 | N |
| ATOM | 5193 | CA | GLN | D | 38 | -22.316 | -35.653 | 99.956 | 1.00 | 24.70 | C |
| ATOM | 5194 | C | GLN | D | 38 | -23.084 | -36.960 | 99.888 | 1.00 | 25.31 | C |
| ATOM | 5195 | O | GLN | D | 38 | -23.766 | -37.233 | 98.901 | 1.00 | 29.60 | O |
| ATOM | 5196 | CB | GLN | D | 38 | -23.231 | -34.524 | 100.429 | 1.00 | 26.48 | C |
| ATOM | 5197 | CG | GLN | D | 38 | -23.861 | -34.780 | 101.757 | 1.00 | 29.22 | C |
| ATOM | 5198 | CD | GLN | D | 38 | -24.925 | -33.785 | 102.068 | 1.00 | 32.98 | C |
| ATOM | 5199 | OE1 | GLN | D | 38 | -26.040 | -33.894 | 101.560 | 1.00 | 39.02 | O |
| ATOM | 5200 | NE2 | GLN | D | 38 | -24.591 | -32.780 | 102.888 | 1.00 | 28.25 | N |
| ATOM | 5201 | N | GLN | D | 39 | -22.956 | -37.781 | 100.916 | 1.00 | 22.53 | N |
| ATOM | 5202 | CA | GLN | D | 39 | -23.703 | -39.024 | 100.962 | 1.00 | 27.58 | C |
| ATOM | 5203 | C | GLN | D | 39 | -24.189 | -39.277 | 102.379 | 1.00 | 28.58 | C |
| ATOM | 5204 | O | GLN | D | 39 | -23.379 | -39.372 | 103.304 | 1.00 | 23.07 | O |
| ATOM | 5205 | CB | GLN | D | 39 | -22.875 | -40.207 | 100.468 | 1.00 | 26.29 | C |
| ATOM | 5206 | CG | GLN | D | 39 | -23.719 | -41.455 | 100.412 | 1.00 | 24.76 | C |
| ATOM | 5207 | CD | GLN | D | 39 | -23.000 | -42.610 | 99.847 | 1.00 | 27.83 | C |
| ATOM | 5208 | OE1 | GLN | D | 39 | -21.818 | -42.804 | 100.103 | 1.00 | 28.43 | O |
| ATOM | 5209 | NE2 | GLN | D | 39 | -23.697 | -43.390 | 99.042 | 1.00 | 31.88 | N |
| ATOM | 5210 | N | LYS | D | 40 | -25.520 | -39.390 | 102.538 | 1.00 | 28.80 | N |
| ATOM | 5211 | CA | LYS | D | 40 | -26.175 | -39.790 | 103.771 | 1.00 | 29.70 | C |
| ATOM | 5212 | C | LYS | D | 40 | -26.436 | -41.289 | 103.763 | 1.00 | 34.10 | C |
| ATOM | 5213 | O | LYS | D | 40 | -26.510 | -41.910 | 102.691 | 1.00 | 30.33 | O |
| ATOM | 5214 | CB | LYS | D | 40 | -27.478 | -39.021 | 103.946 | 1.00 | 31.38 | C |
| ATOM | 5215 | CG | LYS | D | 40 | -27.272 | -37.656 | 104.529 | 1.00 | 32.07 | C |
| ATOM | 5216 | CD | LYS | D | 40 | -28.432 | -36.768 | 104.221 | 1.00 | 42.17 | C |
| ATOM | 5217 | CE | LYS | D | 40 | -28.195 | -35.363 | 104.744 | 1.00 | 44.38 | C |
| ATOM | 5218 | NZ | LYS | D | 40 | -28.086 | -35.382 | 106.237 | 1.00 | 45.69 | N1+ |
| ATOM | 5219 | N | PRO | D | 41 | -26.551 | -41.899 | 104.951 | 1.00 | 33.47 | N |
| ATOM | 5220 | CA | PRO | D | 41 | -26.573 | -43.373 | 105.051 | 1.00 | 33.11 | C |
| ATOM | 5221 | C | PRO | D | 41 | -27.702 | -44.029 | 104.267 | 1.00 | 30.28 | C |
| ATOM | 5222 | O | PRO | D | 41 | -28.872 | -43.660 | 104.394 | 1.00 | 32.79 | O |
| ATOM | 5223 | CB | PRO | D | 41 | -26.726 | -43.612 | 106.558 | 1.00 | 34.12 | C |
| ATOM | 5224 | CG | PRO | D | 41 | -26.096 | -42.407 | 107.186 | 1.00 | 25.92 | C |
| ATOM | 5225 | CD | PRO | D | 41 | -26.471 | -41.265 | 106.280 | 1.00 | 26.33 | C |
| ATOM | 5226 | N | GLY | D | 42 | -27.342 | -45.026 | 103.460 | 1.00 | 29.14 | N |
| ATOM | 5227 | CA | GLY | D | 42 | -28.337 | -45.688 | 102.630 | 1.00 | 29.58 | C |
| ATOM | 5228 | C | GLY | D | 42 | -28.914 | -44.852 | 101.497 | 1.00 | 36.52 | C |
| ATOM | 5229 | O | GLY | D | 42 | -29.956 | -45.213 | 100.952 | 1.00 | 36.30 | O |
| ATOM | 5230 | N | GLN | D | 43 | -28.242 | -43.771 | 101.092 | 1.00 | 32.91 | N |
| ATOM | 5231 | CA | GLN | D | 43 | -28.685 | -42.898 | 100.016 | 1.00 | 27.04 | C |
| ATOM | 5232 | C | GLN | D | 43 | -27.619 | -42.825 | 98.930 | 1.00 | 27.93 | C |
| ATOM | 5233 | O | GLN | D | 43 | -26.452 | -43.162 | 99.153 | 1.00 | 27.99 | O |
| ATOM | 5234 | CB | GLN | D | 43 | -28.952 | -41.481 | 100.523 | 1.00 | 30.13 | C |
| ATOM | 5235 | CG | GLN | D | 43 | -29.659 | -41.422 | 101.838 | 1.00 | 33.40 | C |
| ATOM | 5236 | CD | GLN | D | 43 | -31.073 | -41.941 | 101.754 | 1.00 | 45.27 | C |
| ATOM | 5237 | OE1 | GLN | D | 43 | -31.760 | -41.731 | 100.750 | 1.00 | 42.82 | O |
| ATOM | 5238 | NE2 | GLN | D | 43 | -31.511 | -42.665 | 102.800 | 1.00 | 44.71 | N |
| ATOM | 5239 | N | THR | D | 44 | -28.032 | -42.367 | 97.742 | 1.00 | 25.68 | N |
| ATOM | 5240 | CA | THR | D | 44 | -27.045 | -42.187 | 96.692 | 1.00 | 30.33 | C |
| ATOM | 5241 | C | THR | D | 44 | -26.306 | -40.848 | 96.866 | 1.00 | 30.71 | C |
| ATOM | 5242 | O | THR | D | 44 | -26.834 | -39.904 | 97.464 | 1.00 | 26.05 | O |
| ATOM | 5243 | CB | THR | D | 44 | -27.702 | -42.278 | 95.303 | 1.00 | 28.63 | C |
| ATOM | 5244 | OG1 | THR | D | 44 | -28.240 | -41.013 | 94.924 | 1.00 | 32.11 | O |
| ATOM | 5245 | CG2 | THR | D | 44 | -28.834 | -43.319 | 95.292 | 1.00 | 29.12 | C |
| ATOM | 5246 | N | PRO | D | 45 | -25.070 | -40.756 | 96.375 | 1.00 | 25.93 | N |
| ATOM | 5247 | CA | PRO | D | 45 | -24.312 | -39.512 | 96.515 | 1.00 | 25.74 | C |
| ATOM | 5248 | C | PRO | D | 45 | -24.977 | -38.343 | 95.801 | 1.00 | 24.98 | C |
| ATOM | 5249 | O | PRO | D | 45 | -25.616 | -38.493 | 94.761 | 1.00 | 23.19 | O |

| | | | | | | | | | | | |
|---|---|---|---|---|---|---|---|---|---|---|---|
| ATOM | 5250 | CB | PRO | D | 45 | -22.962 | -39.857 | 95.878 | 1.00 | 28.44 | C |
| ATOM | 5251 | CG | PRO | D | 45 | -22.886 | -41.342 | 95.963 | 1.00 | 23.47 | C |
| ATOM | 5252 | CD | PRO | D | 45 | -24.265 | -41.820 | 95.764 | 1.00 | 23.77 | C |
| ATOM | 5253 | N | ARG | D | 46 | -24.812 | -37.163 | 96.382 | 1.00 | 28.06 | N |
| ATOM | 5254 | CA | ARG | D | 46 | -25.306 | -35.910 | 95.824 | 1.00 | 26.21 | C |
| ATOM | 5255 | C | ARG | D | 46 | -24.120 | -35.028 | 95.449 | 1.00 | 27.60 | C |
| ATOM | 5256 | O | ARG | D | 46 | -23.212 | -34.814 | 96.265 | 1.00 | 24.95 | O |
| ATOM | 5257 | CB | ARG | D | 46 | -26.203 | -35.199 | 96.836 | 1.00 | 28.93 | C |
| ATOM | 5258 | CG | ARG | D | 46 | -26.747 | -33.885 | 96.372 | 1.00 | 35.19 | C |
| ATOM | 5259 | CD | ARG | D | 46 | -28.129 | -33.660 | 96.926 | 1.00 | 42.94 | C |
| ATOM | 5260 | NE | ARG | D | 46 | -28.813 | -32.616 | 96.172 | 1.00 | 54.78 | N |
| ATOM | 5261 | CZ | ARG | D | 46 | -28.994 | -31.379 | 96.619 | 1.00 | 62.11 | C |
| ATOM | 5262 | NH1 | ARG | D | 46 | -29.621 | -30.481 | 95.858 | 1.00 | 53.78 | N1+ |
| ATOM | 5263 | NH2 | ARG | D | 46 | -28.553 | -31.045 | 97.837 | 1.00 | 63.79 | N |
| ATOM | 5264 | N | LEU | D | 47 | -24.116 | -34.535 | 94.216 | 1.00 | 26.63 | N |
| ATOM | 5265 | CA | LEU | D | 47 | -23.029 | -33.680 | 93.772 | 1.00 | 24.56 | C |
| ATOM | 5266 | C | LEU | D | 47 | -23.125 | -32.333 | 94.474 | 1.00 | 23.50 | C |
| ATOM | 5267 | O | LEU | D | 47 | -24.201 | -31.746 | 94.529 | 1.00 | 28.69 | O |
| ATOM | 5268 | CB | LEU | D | 47 | -23.083 | -33.508 | 92.264 | 1.00 | 21.59 | C |
| ATOM | 5269 | CG | LEU | D | 47 | -22.042 | -32.542 | 91.689 | 1.00 | 28.11 | C |
| ATOM | 5270 | CD1 | LEU | D | 47 | -20.587 | -33.047 | 91.831 | 1.00 | 23.37 | C |
| ATOM | 5271 | CD2 | LEU | D | 47 | -22.396 | -32.293 | 90.251 | 1.00 | 26.58 | C |
| ATOM | 5272 | N | LEU | D | 48 | -22.020 | -31.872 | 95.064 | 1.00 | 25.20 | N |
| ATOM | 5273 | CA | LEU | D | 48 | -21.967 | -30.576 | 95.756 | 1.00 | 29.85 | C |
| ATOM | 5274 | C | LEU | D | 48 | -21.153 | -29.530 | 95.021 | 1.00 | 28.28 | C |
| ATOM | 5275 | O | LEU | D | 48 | -21.553 | -28.371 | 94.962 | 1.00 | 26.16 | O |
| ATOM | 5276 | CB | LEU | D | 48 | -21.354 | -30.705 | 97.156 | 1.00 | 24.38 | C |
| ATOM | 5277 | CG | LEU | D | 48 | -22.032 | -31.429 | 98.300 | 1.00 | 29.64 | C |
| ATOM | 5278 | CD1 | LEU | D | 48 | -21.124 | -31.235 | 99.476 | 1.00 | 28.92 | C |
| ATOM | 5279 | CD2 | LEU | D | 48 | -23.421 | -30.868 | 98.604 | 1.00 | 30.54 | C |
| ATOM | 5280 | N | ILE | D | 49 | -19.998 | -29.933 | 94.505 | 1.00 | 24.89 | N |
| ATOM | 5281 | CA | ILE | D | 49 | -19.008 | -29.059 | 93.903 | 1.00 | 25.06 | C |
| ATOM | 5282 | C | ILE | D | 49 | -18.484 | -29.781 | 92.672 | 1.00 | 28.83 | C |
| ATOM | 5283 | O | ILE | D | 49 | -18.212 | -30.986 | 92.726 | 1.00 | 25.60 | O |
| ATOM | 5284 | CB | ILE | D | 49 | -17.833 | -28.787 | 94.879 | 1.00 | 27.73 | C |
| ATOM | 5285 | CG1 | ILE | D | 49 | -18.307 | -28.181 | 96.214 | 1.00 | 22.96 | C |
| ATOM | 5286 | CG2 | ILE | D | 49 | -16.678 | -28.055 | 94.176 | 1.00 | 21.77 | C |
| ATOM | 5287 | CD1 | ILE | D | 49 | -18.656 | -26.704 | 96.180 | 1.00 | 28.66 | C |
| ATOM | 5288 | N | PHE | D | 50 | -18.308 | -29.049 | 91.573 | 1.00 | 25.66 | N |
| ATOM | 5289 | CA | PHE | D | 50 | -17.533 | -29.563 | 90.460 | 1.00 | 23.52 | C |
| ATOM | 5290 | C | PHE | D | 50 | -16.420 | -28.576 | 90.149 | 1.00 | 30.77 | C |
| ATOM | 5291 | O | PHE | D | 50 | -16.505 | -27.377 | 90.451 | 1.00 | 26.94 | O |
| ATOM | 5292 | CB | PHE | D | 50 | -18.381 | -29.861 | 89.202 | 1.00 | 25.10 | C |
| ATOM | 5293 | CG | PHE | D | 50 | -19.084 | -28.669 | 88.645 | 1.00 | 29.89 | C |
| ATOM | 5294 | CD2 | PHE | D | 50 | -20.394 | -28.380 | 89.034 | 1.00 | 28.85 | C |
| ATOM | 5295 | CD1 | PHE | D | 50 | -18.460 | -27.848 | 87.720 | 1.00 | 26.32 | C |
| ATOM | 5296 | CE2 | PHE | D | 50 | -21.038 | -27.263 | 88.538 | 1.00 | 33.45 | C |
| ATOM | 5297 | CE1 | PHE | D | 50 | -19.111 | -26.747 | 87.212 | 1.00 | 31.34 | C |
| ATOM | 5298 | CZ | PHE | D | 50 | -20.412 | -26.457 | 87.616 | 1.00 | 33.07 | C |
| ATOM | 5299 | N | GLY | D | 51 | -15.381 | -29.125 | 89.536 | 1.00 | 29.64 | N |
| ATOM | 5300 | CA | GLY | D | 51 | -14.110 | -28.473 | 89.344 | 1.00 | 31.07 | C |
| ATOM | 5301 | C | GLY | D | 51 | -13.912 | -27.013 | 89.635 | 1.00 | 29.90 | C |
| ATOM | 5302 | O | GLY | D | 51 | -14.299 | -26.190 | 88.823 | 1.00 | 41.98 | O |
| ATOM | 5303 | N | ALA | D | 52 | -13.331 | -26.633 | 90.761 | 1.00 | 29.04 | N |
| ATOM | 5304 | CA | ALA | D | 52 | -13.045 | -27.404 | 91.963 | 1.00 | 27.42 | C |
| ATOM | 5305 | C | ALA | D | 52 | -13.634 | -26.483 | 93.033 | 1.00 | 31.29 | C |
| ATOM | 5306 | O | ALA | D | 52 | -13.389 | -26.630 | 94.242 | 1.00 | 26.21 | O |
| ATOM | 5307 | CB | ALA | D | 52 | -11.545 | -27.613 | 92.180 | 1.00 | 24.46 | C |
| ATOM | 5308 | N | SER | D | 53 | -14.356 | -25.473 | 92.528 | 1.00 | 25.45 | N |
| ATOM | 5309 | CA | SER | D | 53 | -14.951 | -24.408 | 93.321 | 1.00 | 29.64 | C |
| ATOM | 5310 | C | SER | D | 53 | -16.366 | -24.061 | 92.909 | 1.00 | 29.31 | C |
| ATOM | 5311 | O | SER | D | 53 | -17.001 | -23.251 | 93.595 | 1.00 | 29.52 | O |
| ATOM | 5312 | CB | SER | D | 53 | -14.102 | -23.144 | 93.229 | 1.00 | 24.22 | C |
| ATOM | 5313 | OG | SER | D | 53 | -13.751 | -22.931 | 91.878 | 1.00 | 30.08 | O |
| ATOM | 5314 | N | SER | D | 54 | -16.872 | -24.618 | 91.819 | 1.00 | 24.59 | N |
| ATOM | 5315 | CA | SER | D | 54 | -18.183 | -24.249 | 91.320 | 1.00 | 28.33 | C |
| ATOM | 5316 | C | SER | D | 54 | -19.261 | -25.017 | 92.073 | 1.00 | 29.42 | C |
| ATOM | 5317 | O | SER | D | 54 | -19.261 | -26.252 | 92.107 | 1.00 | 28.83 | O |
| ATOM | 5318 | CB | SER | D | 54 | -18.294 | -24.510 | 89.818 | 1.00 | 30.14 | C |
| ATOM | 5319 | OG | SER | D | 54 | -17.503 | -23.611 | 89.086 | 1.00 | 31.63 | O |
| ATOM | 5320 | N | ARG | D | 55 | -20.185 | -24.274 | 92.653 | 1.00 | 26.56 | N |

| | | | | | | | | | | | |
|---|---|---|---|---|---|---|---|---|---|---|---|
| ATOM | 5321 | CA | ARG | D | 55 | -21.342 | -24.858 | 93.293 | 1.00 | 26.99 | C |
| ATOM | 5322 | C | ARG | D | 55 | -22.293 | -25.465 | 92.261 | 1.00 | 31.42 | C |
| ATOM | 5323 | O | ARG | D | 55 | -22.594 | -24.854 | 91.230 | 1.00 | 34.25 | O |
| ATOM | 5324 | CB | ARG | D | 55 | -22.033 | -23.773 | 94.100 | 1.00 | 31.93 | C |
| ATOM | 5325 | CG | ARG | D | 55 | -22.782 | -24.249 | 95.296 | 1.00 | 39.96 | C |
| ATOM | 5326 | CD | ARG | D | 55 | -23.378 | -23.054 | 96.043 | 1.00 | 42.71 | C |
| ATOM | 5327 | NE | ARG | D | 55 | -22.393 | -22.357 | 96.853 | 1.00 | 39.62 | N |
| ATOM | 5328 | CZ | ARG | D | 55 | -21.943 | -21.142 | 96.574 | 1.00 | 42.81 | C |
| ATOM | 5329 | NH1 | ARG | D | 55 | -21.042 | -20.564 | 97.367 | 1.00 | 46.41 | N1+ |
| ATOM | 5330 | NH2 | ARG | D | 55 | -22.396 | -20.516 | 95.497 | 1.00 | 41.95 | N |
| ATOM | 5331 | N | ALA | D | 56 | -22.783 | -26.668 | 92.562 | 1.00 | 29.25 | N |
| ATOM | 5332 | CA | ALA | D | 56 | -23.814 | -27.344 | 91.783 | 1.00 | 34.27 | C |
| ATOM | 5333 | C | ALA | D | 56 | -25.176 | -26.678 | 91.997 | 1.00 | 36.12 | C |
| ATOM | 5334 | O | ALA | D | 56 | -25.355 | -25.830 | 92.877 | 1.00 | 36.11 | O |
| ATOM | 5335 | CB | ALA | D | 56 | -23.891 | -28.823 | 92.166 | 1.00 | 31.23 | C |
| ATOM | 5336 | N | THR | D | 57 | -26.154 | -27.081 | 91.184 | 1.00 | 36.05 | N |
| ATOM | 5337 | CA | THR | D | 57 | -27.475 | -26.471 | 91.274 | 1.00 | 46.10 | C |
| ATOM | 5338 | C | THR | D | 57 | -28.178 | -26.905 | 92.546 | 1.00 | 41.98 | C |
| ATOM | 5339 | O | THR | D | 57 | -28.091 | -28.063 | 92.960 | 1.00 | 44.69 | O |
| ATOM | 5340 | CB | THR | D | 57 | -28.345 | -26.861 | 90.081 | 1.00 | 48.23 | C |
| ATOM | 5341 | OG1 | THR | D | 57 | -27.504 | -27.188 | 88.968 | 1.00 | 51.51 | O |
| ATOM | 5342 | CG2 | THR | D | 57 | -29.281 | -25.707 | 89.713 | 1.00 | 39.36 | C |
| ATOM | 5343 | N | GLY | D | 58 | -28.898 | -25.966 | 93.148 | 1.00 | 36.11 | N |
| ATOM | 5344 | CA | GLY | D | 58 | -29.589 | -26.198 | 94.396 | 1.00 | 36.25 | C |
| ATOM | 5345 | C | GLY | D | 58 | -28.722 | -26.367 | 95.629 | 1.00 | 35.95 | C |
| ATOM | 5346 | O | GLY | D | 58 | -29.266 | -26.615 | 96.706 | 1.00 | 46.21 | O |
| ATOM | 5347 | N | ILE | D | 59 | -27.406 | -26.270 | 95.526 | 1.00 | 35.23 | N |
| ATOM | 5348 | CA | ILE | D | 59 | -26.552 | -26.443 | 96.707 | 1.00 | 37.35 | C |
| ATOM | 5349 | C | ILE | D | 59 | -26.499 | -25.124 | 97.480 | 1.00 | 32.79 | C |
| ATOM | 5350 | O | ILE | D | 59 | -26.110 | -24.102 | 96.902 | 1.00 | 34.26 | O |
| ATOM | 5351 | CB | ILE | D | 59 | -25.147 | -26.884 | 96.291 | 1.00 | 34.60 | C |
| ATOM | 5352 | CG1 | ILE | D | 59 | -25.150 | -28.289 | 95.658 | 1.00 | 33.46 | C |
| ATOM | 5353 | CG2 | ILE | D | 59 | -24.257 | -26.892 | 97.511 | 1.00 | 28.92 | C |
| ATOM | 5354 | CD1 | ILE | D | 59 | -25.990 | -29.321 | 96.371 | 1.00 | 33.20 | C |
| ATOM | 5355 | N | PRO | D | 60 | -26.822 | -25.098 | 98.776 | 1.00 | 35.94 | N |
| ATOM | 5356 | CA | PRO | D | 60 | -26.753 | -23.831 | 99.528 | 1.00 | 36.87 | C |
| ATOM | 5357 | C | PRO | D | 60 | -25.384 | -23.165 | 99.443 | 1.00 | 39.65 | C |
| ATOM | 5358 | O | PRO | D | 60 | -24.357 | -23.825 | 99.258 | 1.00 | 40.06 | O |
| ATOM | 5359 | CB | PRO | D | 60 | -27.068 | -24.261 | 100.967 | 1.00 | 39.32 | C |
| ATOM | 5360 | CG | PRO | D | 60 | -27.875 | -25.486 | 100.816 | 1.00 | 40.27 | C |
| ATOM | 5361 | CD | PRO | D | 60 | -27.346 | -26.199 | 99.601 | 1.00 | 37.18 | C |
| ATOM | 5362 | N | ASP | D | 61 | -25.355 | -21.841 | 99.615 | 1.00 | 37.82 | N |
| ATOM | 5363 | CA | ASP | D | 61 | -24.047 | -21.197 | 99.529 | 1.00 | 38.85 | C |
| ATOM | 5364 | C | ASP | D | 61 | -23.271 | -21.286 | 100.830 | 1.00 | 38.37 | C |
| ATOM | 5365 | O | ASP | D | 61 | -22.161 | -20.757 | 100.895 | 1.00 | 43.54 | O |
| ATOM | 5366 | CB | ASP | D | 61 | -24.153 | -19.736 | 99.070 | 1.00 | 39.37 | C |
| ATOM | 5367 | CG | ASP | D | 61 | -25.124 | -18.914 | 99.898 | 1.00 | 54.57 | C |
| ATOM | 5368 | OD1 | ASP | D | 61 | -25.360 | -19.253 | 101.082 | 1.00 | 61.72 | O |
| ATOM | 5369 | OD2 | ASP | D | 61 | -25.635 | -17.901 | 99.363 | 1.00 | 51.55 | O1- |
| ATOM | 5370 | N | ARG | D | 62 | -23.833 | -21.947 | 101.850 | 1.00 | 39.40 | N |
| ATOM | 5371 | CA | ARG | D | 62 | -23.032 | -22.496 | 102.939 | 1.00 | 36.54 | C |
| ATOM | 5372 | C | ARG | D | 62 | -21.805 | -23.229 | 102.414 | 1.00 | 34.81 | C |
| ATOM | 5373 | O | ARG | D | 62 | -20.738 | -23.199 | 103.037 | 1.00 | 31.24 | O |
| ATOM | 5374 | CB | ARG | D | 62 | -23.837 | -23.511 | 103.745 | 1.00 | 37.64 | C |
| ATOM | 5375 | CG | ARG | D | 62 | -25.054 | -23.076 | 104.366 | 1.00 | 36.64 | C |
| ATOM | 5376 | CD | ARG | D | 62 | -25.298 | -24.006 | 105.536 | 1.00 | 40.91 | C |
| ATOM | 5377 | NE | ARG | D | 62 | -25.716 | -25.370 | 105.214 | 1.00 | 38.63 | N |
| ATOM | 5378 | CZ | ARG | D | 62 | -26.848 | -25.692 | 104.593 | 1.00 | 43.76 | C |
| ATOM | 5379 | NH1 | ARG | D | 62 | -27.662 | -24.743 | 104.160 | 1.00 | 45.94 | N1+ |
| ATOM | 5380 | NH2 | ARG | D | 62 | -27.159 | -26.964 | 104.383 | 1.00 | 41.39 | N |
| ATOM | 5381 | N | PHE | D | 63 | -21.980 | -23.979 | 101.323 | 1.00 | 34.78 | N |
| ATOM | 5382 | CA | PHE | D | 63 | -20.936 | -24.809 | 100.738 | 1.00 | 34.76 | C |
| ATOM | 5383 | C | PHE | D | 63 | -20.101 | -23.966 | 99.797 | 1.00 | 32.41 | C |
| ATOM | 5384 | O | PHE | D | 63 | -20.646 | -23.271 | 98.933 | 1.00 | 35.79 | O |
| ATOM | 5385 | CB | PHE | D | 63 | -21.536 | -26.013 | 99.992 | 1.00 | 29.42 | C |
| ATOM | 5386 | CG | PHE | D | 63 | -22.210 | -27.001 | 100.898 | 1.00 | 28.36 | C |
| ATOM | 5387 | CD1 | PHE | D | 63 | -23.504 | -26.790 | 101.344 | 1.00 | 31.54 | C |
| ATOM | 5388 | CD2 | PHE | D | 63 | -21.537 | -28.120 | 101.336 | 1.00 | 28.75 | C |
| ATOM | 5389 | CE1 | PHE | D | 63 | -24.117 | -27.686 | 102.190 | 1.00 | 29.90 | C |
| ATOM | 5390 | CE2 | PHE | D | 63 | -22.149 | -29.017 | 102.180 | 1.00 | 31.42 | C |
| ATOM | 5391 | CZ | PHE | D | 63 | -23.441 | -28.796 | 102.611 | 1.00 | 27.00 | C |

| | | | | | | | | | | | |
|---|---|---|---|---|---|---|---|---|---|---|---|
| ATOM | 5392 | N | SER | D | 64 | -18.783 | -24.021 | 99.986 | 1.00 | 32.03 | N |
| ATOM | 5393 | CA | SER | D | 64 | -17.815 | -23.408 | 99.081 | 1.00 | 33.91 | C |
| ATOM | 5394 | C | SER | D | 64 | -16.566 | -24.279 | 99.051 | 1.00 | 26.96 | C |
| ATOM | 5395 | O | SER | D | 64 | -16.345 | -25.111 | 99.934 | 1.00 | 29.99 | O |
| ATOM | 5396 | CB | SER | D | 64 | -17.482 | -21.975 | 99.511 | 1.00 | 31.16 | C |
| ATOM | 5397 | OG | SER | D | 64 | -16.576 | -21.995 | 100.592 | 1.00 | 35.76 | O |
| ATOM | 5398 | N | ALA | D | 65 | -15.766 | -24.128 | 98.007 | 1.00 | 21.94 | N |
| ATOM | 5399 | CA | ALA | D | 65 | -14.588 | -24.982 | 97.928 | 1.00 | 27.79 | C |
| ATOM | 5400 | C | ALA | D | 65 | -13.547 | -24.302 | 97.063 | 1.00 | 28.20 | C |
| ATOM | 5401 | O | ALA | D | 65 | -13.859 | -23.392 | 96.297 | 1.00 | 28.35 | O |
| ATOM | 5402 | CB | ALA | D | 65 | -14.930 | -26.370 | 97.382 | 1.00 | 26.70 | C |
| ATOM | 5403 | N | SER | D | 66 | -12.305 | -24.767 | 97.185 | 1.00 | 31.90 | N |
| ATOM | 5404 | CA | SER | D | 66 | -11.196 | -24.189 | 96.432 | 1.00 | 32.71 | C |
| ATOM | 5405 | C | SER | D | 66 | -10.001 | -25.141 | 96.468 | 1.00 | 32.60 | C |
| ATOM | 5406 | O | SER | D | 66 | -10.005 | -26.170 | 97.149 | 1.00 | 34.80 | O |
| ATOM | 5407 | CB | SER | D | 66 | -10.787 | -22.831 | 97.003 | 1.00 | 27.36 | C |
| ATOM | 5408 | OG | SER | D | 66 | -10.156 | -23.031 | 98.253 | 1.00 | 32.54 | O |
| ATOM | 5409 | N | GLY | D | 67 | -8.948 | -24.745 | 95.780 | 1.00 | 31.68 | N |
| ATOM | 5410 | CA | GLY | D | 67 | -7.719 | -25.498 | 95.795 | 1.00 | 28.89 | C |
| ATOM | 5411 | C | GLY | D | 67 | -7.316 | -25.711 | 94.364 | 1.00 | 38.37 | C |
| ATOM | 5412 | O | GLY | D | 67 | -8.135 | -25.485 | 93.466 | 1.00 | 39.57 | O |
| ATOM | 5413 | N | SER | D | 68 | -6.057 | -26.066 | 94.125 | 1.00 | 36.68 | N |
| ATOM | 5414 | CA | SER | D | 68 | -5.677 | -26.559 | 92.813 | 1.00 | 36.53 | C |
| ATOM | 5415 | C | SER | D | 68 | -4.366 | -27.296 | 92.959 | 1.00 | 30.07 | C |
| ATOM | 5416 | O | SER | D | 68 | -3.660 | -27.148 | 93.952 | 1.00 | 38.18 | O |
| ATOM | 5417 | CB | SER | D | 68 | -5.559 | -25.449 | 91.757 | 1.00 | 44.96 | C |
| ATOM | 5418 | OG | SER | D | 68 | -5.439 | -26.014 | 90.444 | 1.00 | 48.06 | O |
| ATOM | 5419 | N | GLY | D | 69 | -4.062 | -28.097 | 91.954 | 1.00 | 29.21 | N |
| ATOM | 5420 | CA | GLY | D | 69 | -2.896 | -28.929 | 91.987 | 1.00 | 29.40 | C |
| ATOM | 5421 | C | GLY | D | 69 | -3.069 | -30.067 | 92.949 | 1.00 | 31.99 | C |
| ATOM | 5422 | O | GLY | D | 69 | -3.815 | -31.018 | 92.687 | 1.00 | 33.63 | O |
| ATOM | 5423 | N | ALA | D | 70 | -2.379 | -29.966 | 94.080 | 1.00 | 31.64 | N |
| ATOM | 5424 | CA | ALA | D | 70 | -2.287 | -31.065 | 95.022 | 1.00 | 31.80 | C |
| ATOM | 5425 | C | ALA | D | 70 | -3.275 | -30.980 | 96.171 | 1.00 | 31.37 | C |
| ATOM | 5426 | O | ALA | D | 70 | -3.550 | -32.008 | 96.792 | 1.00 | 36.24 | O |
| ATOM | 5427 | CB | ALA | D | 70 | -0.868 | -31.140 | 95.594 | 1.00 | 28.13 | C |
| ATOM | 5428 | N | ASP | D | 71 | -3.817 | -29.803 | 96.471 | 1.00 | 30.57 | N |
| ATOM | 5429 | CA | ASP | D | 71 | -4.565 | -29.587 | 97.706 | 1.00 | 33.18 | C |
| ATOM | 5430 | C | ASP | D | 71 | -5.945 | -29.009 | 97.415 | 1.00 | 32.16 | C |
| ATOM | 5431 | O | ASP | D | 71 | -6.063 | -27.965 | 96.768 | 1.00 | 31.72 | O |
| ATOM | 5432 | CB | ASP | D | 71 | -3.800 | -28.638 | 98.622 | 1.00 | 31.72 | C |
| ATOM | 5433 | CG | ASP | D | 71 | -2.722 | -29.331 | 99.396 | 1.00 | 47.35 | C |
| ATOM | 5434 | OD1 | ASP | D | 71 | -3.025 | -30.000 | 100.408 | 1.00 | 54.93 | O |
| ATOM | 5435 | OD2 | ASP | D | 71 | -1.553 | -29.211 | 98.980 | 1.00 | 57.78 | O1- |
| ATOM | 5436 | N | PHE | D | 72 | -6.981 | -29.643 | 97.939 | 1.00 | 28.95 | N |
| ATOM | 5437 | CA | PHE | D | 72 | -8.335 | -29.145 | 97.771 | 1.00 | 25.33 | C |
| ATOM | 5438 | C | PHE | D | 72 | -8.994 | -29.122 | 99.135 | 1.00 | 24.96 | C |
| ATOM | 5439 | O | PHE | D | 72 | -8.687 | -29.939 | 100.005 | 1.00 | 21.64 | O |
| ATOM | 5440 | CB | PHE | D | 72 | -9.121 | -30.008 | 96.781 | 1.00 | 26.11 | C |
| ATOM | 5441 | CG | PHE | D | 72 | -8.492 | -30.051 | 95.425 | 1.00 | 30.35 | C |
| ATOM | 5442 | CD1 | PHE | D | 72 | -7.479 | -30.982 | 95.144 | 1.00 | 29.20 | C |
| ATOM | 5443 | CD2 | PHE | D | 72 | -8.862 | -29.139 | 94.444 | 1.00 | 27.20 | C |
| ATOM | 5444 | CE1 | PHE | D | 72 | -6.862 | -31.014 | 93.898 | 1.00 | 31.22 | C |
| ATOM | 5445 | CE2 | PHE | D | 72 | -8.250 | -29.161 | 93.189 | 1.00 | 30.03 | C |
| ATOM | 5446 | CZ | PHE | D | 72 | -7.251 | -30.105 | 92.913 | 1.00 | 29.83 | C |
| ATOM | 5447 | N | THR | D | 73 | -9.842 | -28.133 | 99.354 | 1.00 | 23.98 | N |
| ATOM | 5448 | CA | THR | D | 73 | -10.593 | -28.118 | 100.589 | 1.00 | 27.63 | C |
| ATOM | 5449 | C | THR | D | 73 | -12.045 | -27.791 | 100.275 | 1.00 | 28.02 | C |
| ATOM | 5450 | O | THR | D | 73 | -12.363 | -27.129 | 99.275 | 1.00 | 25.48 | O |
| ATOM | 5451 | CB | THR | D | 73 | -9.993 | -27.156 | 101.661 | 1.00 | 32.96 | C |
| ATOM | 5452 | OG1 | THR | D | 73 | -10.611 | -25.872 | 101.585 | 1.00 | 38.47 | O |
| ATOM | 5453 | CG2 | THR | D | 73 | -8.473 | -26.987 | 101.511 | 1.00 | 24.84 | C |
| ATOM | 5454 | N | LEU | D | 74 | -12.926 | -28.368 | 101.090 | 1.00 | 30.82 | N |
| ATOM | 5455 | CA | LEU | D | 74 | -14.351 | -28.062 | 101.114 | 1.00 | 26.16 | C |
| ATOM | 5456 | C | LEU | D | 74 | -14.662 | -27.367 | 102.433 | 1.00 | 27.40 | C |
| ATOM | 5457 | O | LEU | D | 74 | -14.247 | -27.843 | 103.499 | 1.00 | 27.12 | O |
| ATOM | 5458 | CB | LEU | D | 74 | -15.184 | -29.336 | 100.953 | 1.00 | 21.42 | C |
| ATOM | 5459 | CG | LEU | D | 74 | -16.696 | -29.239 | 101.152 | 1.00 | 21.05 | C |
| ATOM | 5460 | CD1 | LEU | D | 74 | -17.360 | -28.535 | 100.013 | 1.00 | 24.58 | C |
| ATOM | 5461 | CD2 | LEU | D | 74 | -17.329 | -30.584 | 101.345 | 1.00 | 20.48 | C |
| ATOM | 5462 | N | THR | D | 75 | -15.378 | -26.243 | 102.365 | 1.00 | 24.12 | N |

| | | | | | | | | | | | |
|---|---|---|---|---|---|---|---|---|---|---|---|
| ATOM | 5463 | CA | THR | D | 75 | -15.762 | -25.486 | 103.548 | 1.00 | 24.98 | C |
| ATOM | 5464 | C | THR | D | 75 | -17.286 | -25.435 | 103.651 | 1.00 | 28.91 | C |
| ATOM | 5465 | O | THR | D | 75 | -17.971 | -25.077 | 102.687 | 1.00 | 33.13 | O |
| ATOM | 5466 | CB | THR | D | 75 | -15.167 | -24.066 | 103.513 | 1.00 | 29.48 | C |
| ATOM | 5467 | OG1 | THR | D | 75 | -13.776 | -24.123 | 103.842 | 1.00 | 31.85 | O |
| ATOM | 5468 | CG2 | THR | D | 75 | -15.840 | -23.161 | 104.544 | 1.00 | 30.01 | C |
| ATOM | 5469 | N | ILE | D | 76 | -17.820 | -25.840 | 104.799 | 1.00 | 25.85 | N |
| ATOM | 5470 | CA | ILE | D | 76 | -19.217 | -25.616 | 105.141 | 1.00 | 25.76 | C |
| ATOM | 5471 | C | ILE | D | 76 | -19.211 | -24.568 | 106.232 | 1.00 | 30.23 | C |
| ATOM | 5472 | O | ILE | D | 76 | -18.718 | -24.829 | 107.337 | 1.00 | 34.43 | O |
| ATOM | 5473 | CB | ILE | D | 76 | -19.922 | -26.893 | 105.619 | 1.00 | 28.28 | C |
| ATOM | 5474 | CG1 | ILE | D | 76 | -19.619 | -28.063 | 104.696 | 1.00 | 28.54 | C |
| ATOM | 5475 | CG2 | ILE | D | 76 | -21.443 | -26.672 | 105.686 | 1.00 | 28.62 | C |
| ATOM | 5476 | CD1 | ILE | D | 76 | -20.092 | -29.368 | 105.257 | 1.00 | 27.59 | C |
| ATOM | 5477 | N | SER | D | 77 | -19.775 | -23.395 | 105.945 | 1.00 | 30.77 | N |
| ATOM | 5478 | CA | SER | D | 77 | -19.475 | -22.251 | 106.803 | 1.00 | 33.53 | C |
| ATOM | 5479 | C | SER | D | 77 | -20.286 | -22.267 | 108.097 | 1.00 | 32.03 | C |
| ATOM | 5480 | O | SER | D | 77 | -19.781 | -21.840 | 109.141 | 1.00 | 35.78 | O |
| ATOM | 5481 | CB | SER | D | 77 | -19.681 | -20.943 | 106.038 | 1.00 | 25.30 | C |
| ATOM | 5482 | OG | SER | D | 77 | -21.029 | -20.795 | 105.672 | 1.00 | 30.61 | O |
| ATOM | 5483 | N | ARG | D | 78 | -21.539 | -22.727 | 108.051 | 1.00 | 34.30 | N |
| ATOM | 5484 | CA | ARG | D | 78 | -22.346 | -22.937 | 109.254 | 1.00 | 33.67 | C |
| ATOM | 5485 | C | ARG | D | 78 | -23.026 | -24.294 | 109.107 | 1.00 | 33.98 | C |
| ATOM | 5486 | O | ARG | D | 78 | -23.982 | -24.422 | 108.341 | 1.00 | 30.96 | O |
| ATOM | 5487 | CB | ARG | D | 78 | -23.387 | -21.830 | 109.423 | 1.00 | 34.14 | C |
| ATOM | 5488 | CG | ARG | D | 78 | -24.425 | -22.137 | 110.499 | 1.00 | 46.20 | C |
| ATOM | 5489 | CD | ARG | D | 78 | -25.375 | -20.962 | 110.761 | 1.00 | 46.68 | C |
| ATOM | 5490 | NE | ARG | D | 78 | -25.737 | -20.874 | 112.185 | 1.00 | 49.36 | N |
| ATOM | 5491 | CZ | ARG | D | 78 | -26.801 | -21.439 | 112.763 | 1.00 | 57.02 | C |
| ATOM | 5492 | NH1 | ARG | D | 78 | -27.008 | -21.283 | 114.074 | 1.00 | 54.06 | N1+ |
| ATOM | 5493 | NH2 | ARG | D | 78 | -27.666 | -22.153 | 112.047 | 1.00 | 59.27 | N |
| ATOM | 5494 | N | LEU | D | 79 | -22.616 | -25.273 | 109.904 | 1.00 | 31.63 | N |
| ATOM | 5495 | CA | LEU | D | 79 | -23.146 | -26.627 | 109.772 | 1.00 | 29.40 | C |
| ATOM | 5496 | C | LEU | D | 79 | -24.623 | -26.674 | 110.172 | 1.00 | 34.83 | C |
| ATOM | 5497 | O | LEU | D | 79 | -24.969 | -26.417 | 111.328 | 1.00 | 36.97 | O |
| ATOM | 5498 | CB | LEU | D | 79 | -22.333 | -27.580 | 110.640 | 1.00 | 26.40 | C |
| ATOM | 5499 | CG | LEU | D | 79 | -21.373 | -28.630 | 110.107 | 1.00 | 26.42 | C |
| ATOM | 5500 | CD1 | LEU | D | 79 | -21.113 | -28.496 | 108.643 | 1.00 | 29.85 | C |
| ATOM | 5501 | CD2 | LEU | D | 79 | -20.083 | -28.535 | 110.868 | 1.00 | 29.71 | C |
| ATOM | 5502 | N | GLU | D | 80 | -25.507 | -26.990 | 109.211 | 1.00 | 35.29 | N |
| ATOM | 5503 | CA | GLU | D | 80 | -26.911 | -27.234 | 109.503 | 1.00 | 33.25 | C |
| ATOM | 5504 | C | GLU | D | 80 | -27.130 | -28.711 | 109.784 | 1.00 | 37.31 | C |
| ATOM | 5505 | O | GLU | D | 80 | -26.269 | -29.536 | 109.472 | 1.00 | 34.23 | O |
| ATOM | 5506 | CB | GLU | D | 80 | -27.771 | -26.790 | 108.334 | 1.00 | 37.10 | C |
| ATOM | 5507 | CG | GLU | D | 80 | -27.822 | -25.300 | 108.123 | 1.00 | 37.33 | C |
| ATOM | 5508 | CD | GLU | D | 80 | -28.430 | -24.559 | 109.292 | 1.00 | 45.25 | C |
| ATOM | 5509 | OE1 | GLU | D | 80 | -29.311 | -25.110 | 109.996 | 1.00 | 48.39 | O |
| ATOM | 5510 | OE2 | GLU | D | 80 | -28.039 | -23.395 | 109.496 | 1.00 | 54.67 | O1- |
| ATOM | 5511 | N | PRO | D | 81 | -28.261 | -29.088 | 110.403 | 1.00 | 43.56 | N |
| ATOM | 5512 | CA | PRO | D | 81 | -28.458 | -30.514 | 110.743 | 1.00 | 45.48 | C |
| ATOM | 5513 | C | PRO | D | 81 | -28.252 | -31.464 | 109.571 | 1.00 | 42.65 | C |
| ATOM | 5514 | O | PRO | D | 81 | -27.600 | -32.508 | 109.728 | 1.00 | 39.13 | O |
| ATOM | 5515 | CB | PRO | D | 81 | -29.909 | -30.547 | 111.254 | 1.00 | 44.07 | C |
| ATOM | 5516 | CG | PRO | D | 81 | -30.130 | -29.185 | 111.794 | 1.00 | 40.26 | C |
| ATOM | 5517 | CD | PRO | D | 81 | -29.382 | -28.259 | 110.884 | 1.00 | 41.88 | C |
| ATOM | 5518 | N | GLU | D | 82 | -28.761 | -31.107 | 108.389 | 1.00 | 40.42 | N |
| ATOM | 5519 | CA | GLU | D | 82 | -28.620 | -31.911 | 107.178 | 1.00 | 37.02 | C |
| ATOM | 5520 | C | GLU | D | 82 | -27.222 | -31.924 | 106.602 | 1.00 | 38.11 | C |
| ATOM | 5521 | O | GLU | D | 82 | -27.053 | -32.461 | 105.498 | 1.00 | 37.79 | O |
| ATOM | 5522 | CB | GLU | D | 82 | -29.528 | -31.396 | 106.079 | 1.00 | 34.91 | C |
| ATOM | 5523 | CG | GLU | D | 82 | -29.092 | -30.088 | 105.479 | 1.00 | 42.26 | C |
| ATOM | 5524 | CD | GLU | D | 82 | -30.094 | -28.977 | 105.612 | 1.00 | 49.69 | C |
| ATOM | 5525 | OE1 | GLU | D | 82 | -30.683 | -28.801 | 106.716 | 1.00 | 59.37 | O |
| ATOM | 5526 | OE2 | GLU | D | 82 | -30.297 | -28.286 | 104.582 | 1.00 | 55.07 | O1- |
| ATOM | 5527 | N | ASP | D | 83 | -26.234 | -31.314 | 107.246 | 1.00 | 31.73 | N |
| ATOM | 5528 | CA | ASP | D | 83 | -24.893 | -31.308 | 106.690 | 1.00 | 31.81 | C |
| ATOM | 5529 | C | ASP | D | 83 | -24.033 | -32.399 | 107.278 | 1.00 | 27.67 | C |
| ATOM | 5530 | O | ASP | D | 83 | -22.905 | -32.577 | 106.833 | 1.00 | 28.90 | O |
| ATOM | 5531 | CB | ASP | D | 83 | -24.205 | -29.949 | 106.911 | 1.00 | 35.00 | C |
| ATOM | 5532 | CG | ASP | D | 83 | -24.923 | -28.802 | 106.203 | 1.00 | 36.30 | C |
| ATOM | 5533 | OD1 | ASP | D | 83 | -25.767 | -29.091 | 105.334 | 1.00 | 39.93 | O |

| | | | | | | | | | | | |
|---|---|---|---|---|---|---|---|---|---|---|---|
| ATOM | 5534 | OD2 | ASP | D | 83 | -24.663 | -27.615 | 106.524 | 1.00 | 33.84 | O1- |
| ATOM | 5535 | N | PHE | D | 84 | -24.531 | -33.133 | 108.262 | 1.00 | 28.52 | N |
| ATOM | 5536 | CA | PHE | D | 84 | -23.736 | -34.160 | 108.921 | 1.00 | 27.69 | C |
| ATOM | 5537 | C | PHE | D | 84 | -23.892 | -35.469 | 108.164 | 1.00 | 27.68 | C |
| ATOM | 5538 | O | PHE | D | 84 | -24.963 | -36.083 | 108.176 | 1.00 | 27.12 | O |
| ATOM | 5539 | CB | PHE | D | 84 | -24.127 | -34.276 | 110.391 | 1.00 | 26.49 | C |
| ATOM | 5540 | CG | PHE | D | 84 | -23.758 | -33.065 | 111.194 | 1.00 | 27.03 | C |
| ATOM | 5541 | CD1 | PHE | D | 84 | -22.520 | -32.992 | 111.822 | 1.00 | 27.26 | C |
| ATOM | 5542 | CD2 | PHE | D | 84 | -24.628 | -31.981 | 111.289 | 1.00 | 30.67 | C |
| ATOM | 5543 | CE1 | PHE | D | 84 | -22.156 | -31.865 | 112.562 | 1.00 | 31.27 | C |
| ATOM | 5544 | CE2 | PHE | D | 84 | -24.281 | -30.849 | 112.016 | 1.00 | 29.02 | C |
| ATOM | 5545 | CZ | PHE | D | 84 | -23.039 | -30.794 | 112.667 | 1.00 | 32.36 | C |
| ATOM | 5546 | N | ALA | D | 85 | -22.815 | -35.890 | 107.516 | 1.00 | 25.77 | N |
| ATOM | 5547 | CA | ALA | D | 85 | -22.859 | -36.899 | 106.469 | 1.00 | 21.15 | C |
| ATOM | 5548 | C | ALA | D | 85 | -21.420 | -37.274 | 106.118 | 1.00 | 22.96 | C |
| ATOM | 5549 | O | ALA | D | 85 | -20.472 | -36.875 | 106.800 | 1.00 | 22.25 | O |
| ATOM | 5550 | CB | ALA | D | 85 | -23.660 | -36.381 | 105.275 | 1.00 | 25.38 | C |
| ATOM | 5551 | N | VAL | D | 86 | -21.251 | -38.069 | 105.059 | 1.00 | 25.06 | N |
| ATOM | 5552 | CA | VAL | D | 86 | -19.939 | -38.399 | 104.521 | 1.00 | 20.70 | C |
| ATOM | 5553 | C | VAL | D | 86 | -19.708 | -37.553 | 103.278 | 1.00 | 22.14 | C |
| ATOM | 5554 | O | VAL | D | 86 | -20.628 | -37.341 | 102.488 | 1.00 | 27.68 | O |
| ATOM | 5555 | CB | VAL | D | 86 | -19.841 | -39.901 | 104.217 | 1.00 | 19.64 | C |
| ATOM | 5556 | CG1 | VAL | D | 86 | -18.533 | -40.230 | 103.523 | 1.00 | 22.34 | C |
| ATOM | 5557 | CG2 | VAL | D | 86 | -19.953 | -40.660 | 105.485 | 1.00 | 16.64 | C |
| ATOM | 5558 | N | TYR | D | 87 | -18.497 | -37.033 | 103.120 | 1.00 | 21.19 | N |
| ATOM | 5559 | CA | TYR | D | 87 | -18.151 | -36.206 | 101.975 | 1.00 | 24.17 | C |
| ATOM | 5560 | C | TYR | D | 87 | -17.024 | -36.879 | 101.219 | 1.00 | 25.06 | C |
| ATOM | 5561 | O | TYR | D | 87 | -16.084 | -37.381 | 101.837 | 1.00 | 25.52 | O |
| ATOM | 5562 | CB | TYR | D | 87 | -17.765 | -34.766 | 102.410 | 1.00 | 25.82 | C |
| ATOM | 5563 | CG | TYR | D | 87 | -18.963 | -34.036 | 102.978 | 1.00 | 24.53 | C |
| ATOM | 5564 | CD1 | TYR | D | 87 | -19.368 | -34.249 | 104.286 | 1.00 | 22.98 | C |
| ATOM | 5565 | CD2 | TYR | D | 87 | -19.735 | -33.198 | 102.178 | 1.00 | 22.74 | C |
| ATOM | 5566 | CE1 | TYR | D | 87 | -20.502 | -33.616 | 104.791 | 1.00 | 27.82 | C |
| ATOM | 5567 | CE2 | TYR | D | 87 | -20.857 | -32.573 | 102.662 | 1.00 | 22.11 | C |
| ATOM | 5568 | CZ | TYR | D | 87 | -21.244 | -32.777 | 103.966 | 1.00 | 28.03 | C |
| ATOM | 5569 | OH | TYR | D | 87 | -22.379 | -32.154 | 104.449 | 1.00 | 29.25 | O |
| ATOM | 5570 | N | PHE | D | 88 | -17.140 | -36.897 | 99.885 | 1.00 | 22.99 | N |
| ATOM | 5571 | CA | PHE | D | 88 | -16.222 | -37.590 | 98.990 | 1.00 | 25.06 | C |
| ATOM | 5572 | C | PHE | D | 88 | -15.653 | -36.649 | 97.952 | 1.00 | 27.58 | C |
| ATOM | 5573 | O | PHE | D | 88 | -16.403 | -35.890 | 97.327 | 1.00 | 28.74 | O |
| ATOM | 5574 | CB | PHE | D | 88 | -16.920 | -38.715 | 98.216 | 1.00 | 29.50 | C |
| ATOM | 5575 | CG | PHE | D | 88 | -17.173 | -39.937 | 99.013 | 1.00 | 30.12 | C |
| ATOM | 5576 | CD1 | PHE | D | 88 | -16.133 | -40.820 | 99.283 | 1.00 | 27.68 | C |
| ATOM | 5577 | CD2 | PHE | D | 88 | -18.442 | -40.231 | 99.470 | 1.00 | 26.34 | C |
| ATOM | 5578 | CE1 | PHE | D | 88 | -16.354 | -41.984 | 100.003 | 1.00 | 31.19 | C |
| ATOM | 5579 | CE2 | PHE | D | 88 | -18.664 | -41.389 | 100.198 | 1.00 | 31.81 | C |
| ATOM | 5580 | CZ | PHE | D | 88 | -17.614 | -42.268 | 100.470 | 1.00 | 27.93 | C |
| ATOM | 5581 | N | CYS | D | 89 | -14.350 | -36.745 | 97.704 | 1.00 | 26.11 | N |
| ATOM | 5582 | CA | CYS | D | 89 | -13.809 | -36.023 | 96.567 | 1.00 | 24.60 | C |
| ATOM | 5583 | C | CYS | D | 89 | -13.561 | -36.988 | 95.406 | 1.00 | 25.26 | C |
| ATOM | 5584 | O | CYS | D | 89 | -13.464 | -38.206 | 95.575 | 1.00 | 23.50 | O |
| ATOM | 5585 | CB | CYS | D | 89 | -12.537 | -35.249 | 96.941 | 1.00 | 23.65 | C |
| ATOM | 5586 | SG | CYS | D | 89 | -11.143 | -36.170 | 97.584 | 1.00 | 38.72 | S |
| ATOM | 5587 | N | GLN | D | 90 | -13.489 | -36.424 | 94.208 | 1.00 | 21.35 | N |
| ATOM | 5588 | CA | GLN | D | 90 | -13.364 | -37.237 | 93.006 | 1.00 | 24.00 | C |
| ATOM | 5589 | C | GLN | D | 90 | -12.697 | -36.404 | 91.921 | 1.00 | 24.00 | C |
| ATOM | 5590 | O | GLN | D | 90 | -13.073 | -35.249 | 91.715 | 1.00 | 23.20 | O |
| ATOM | 5591 | CB | GLN | D | 90 | -14.732 | -37.733 | 92.536 | 1.00 | 21.84 | C |
| ATOM | 5592 | CG | GLN | D | 90 | -14.703 | -38.606 | 91.307 | 1.00 | 21.39 | C |
| ATOM | 5593 | CD | GLN | D | 90 | -15.439 | -37.976 | 90.144 | 1.00 | 26.00 | C |
| ATOM | 5594 | OE1 | GLN | D | 90 | -16.481 | -37.331 | 90.333 | 1.00 | 29.02 | O |
| ATOM | 5595 | NE2 | GLN | D | 90 | -14.915 | -38.158 | 88.933 | 1.00 | 21.45 | N |
| ATOM | 5596 | N | GLN | D | 91 | -11.714 | -36.984 | 91.236 | 1.00 | 22.32 | N |
| ATOM | 5597 | CA | GLN | D | 91 | -11.051 | -36.316 | 90.130 | 1.00 | 24.41 | C |
| ATOM | 5598 | C | GLN | D | 91 | -11.459 | -36.980 | 88.818 | 1.00 | 26.03 | C |
| ATOM | 5599 | O | GLN | D | 91 | -11.628 | -38.205 | 88.750 | 1.00 | 23.79 | O |
| ATOM | 5600 | CB | GLN | D | 91 | -9.520 | -36.329 | 90.308 | 1.00 | 21.11 | C |
| ATOM | 5601 | CG | GLN | D | 91 | -8.866 | -37.697 | 90.214 | 1.00 | 21.28 | C |
| ATOM | 5602 | CD | GLN | D | 91 | -8.585 | -38.091 | 88.785 | 1.00 | 26.27 | C |
| ATOM | 5603 | OE1 | GLN | D | 91 | -8.432 | -37.231 | 87.917 | 1.00 | 28.92 | O |
| ATOM | 5604 | NE2 | GLN | D | 91 | -8.567 | -39.384 | 88.514 | 1.00 | 26.42 | N |

| | | | | | | | | | | | |
|---|---|---|---|---|---|---|---|---|---|---|---|
| ATOM | 5605 | N | TYR | D | 92 | -11.588 | -36.162 | 87.776 | 1.00 | 22.58 | N |
| ATOM | 5606 | CA | TYR | D | 92 | -11.946 | -36.594 | 86.432 | 1.00 | 26.01 | C |
| ATOM | 5607 | C | TYR | D | 92 | -11.013 | -35.927 | 85.432 | 1.00 | 27.20 | C |
| ATOM | 5608 | O | TYR | D | 92 | -11.409 | -35.517 | 84.334 | 1.00 | 25.89 | O |
| ATOM | 5609 | CB | TYR | D | 92 | -13.413 | -36.300 | 86.093 | 1.00 | 22.29 | C |
| ATOM | 5610 | CG | TYR | D | 92 | -13.889 | -34.864 | 86.367 | 1.00 | 26.32 | C |
| ATOM | 5611 | CD1 | TYR | D | 92 | -14.320 | -34.475 | 87.646 | 1.00 | 21.74 | C |
| ATOM | 5612 | CD2 | TYR | D | 92 | -13.903 | -33.893 | 85.344 | 1.00 | 26.96 | C |
| ATOM | 5613 | CE1 | TYR | D | 92 | -14.755 | -33.186 | 87.894 | 1.00 | 22.81 | C |
| ATOM | 5614 | CE2 | TYR | D | 92 | -14.347 | -32.585 | 85.587 | 1.00 | 24.44 | C |
| ATOM | 5615 | CZ | TYR | D | 92 | -14.764 | -32.247 | 86.867 | 1.00 | 27.75 | C |
| ATOM | 5616 | OH | TYR | D | 92 | -15.189 | -30.972 | 87.141 | 1.00 | 29.35 | O |
| ATOM | 5617 | N | GLU | D | 93 | -9.755 | -35.782 | 85.826 | 1.00 | 27.70 | N |
| ATOM | 5618 | CA | GLU | D | 93 | -8.729 | -35.263 | 84.936 | 1.00 | 29.92 | C |
| ATOM | 5619 | C | GLU | D | 93 | -8.055 | -36.384 | 84.141 | 1.00 | 31.61 | C |
| ATOM | 5620 | O | GLU | D | 93 | -7.883 | -36.278 | 82.926 | 1.00 | 33.23 | O |
| ATOM | 5621 | CB | GLU | D | 93 | -7.687 | -34.482 | 85.744 | 1.00 | 25.29 | C |
| ATOM | 5622 | CG | GLU | D | 93 | -6.386 | -34.312 | 84.993 | 1.00 | 28.20 | C |
| ATOM | 5623 | CD | GLU | D | 93 | -5.369 | -33.447 | 85.700 | 1.00 | 32.11 | C |
| ATOM | 5624 | OE1 | GLU | D | 93 | -5.735 | -32.389 | 86.285 | 1.00 | 26.85 | O |
| ATOM | 5625 | OE2 | GLU | D | 93 | -4.183 | -33.828 | 85.637 | 1.00 | 36.40 | O1- |
| ATOM | 5626 | N | SER | D | 94 | -7.694 | -37.471 | 84.810 | 1.00 | 28.99 | N |
| ATOM | 5627 | CA | SER | D | 94 | -6.939 | -38.555 | 84.213 | 1.00 | 28.53 | C |
| ATOM | 5628 | C | SER | D | 94 | -7.651 | -39.859 | 84.514 | 1.00 | 28.50 | C |
| ATOM | 5629 | O | SER | D | 94 | -8.040 | -40.110 | 85.661 | 1.00 | 26.38 | O |
| ATOM | 5630 | CB | SER | D | 94 | -5.499 | -38.586 | 84.754 | 1.00 | 27.94 | C |
| ATOM | 5631 | OG | SER | D | 94 | -4.912 | -39.865 | 84.570 | 1.00 | 33.48 | O |
| ATOM | 5632 | N | SER | D | 95 | -7.844 | -40.669 | 83.487 | 1.00 | 25.22 | N |
| ATOM | 5633 | CA | SER | D | 95 | -8.488 | -41.943 | 83.683 | 1.00 | 29.13 | C |
| ATOM | 5634 | C | SER | D | 95 | -7.512 | -42.941 | 84.345 | 1.00 | 28.02 | C |
| ATOM | 5635 | O | SER | D | 95 | -6.324 | -42.923 | 84.083 | 1.00 | 28.38 | O |
| ATOM | 5636 | CB | SER | D | 95 | -9.012 | -42.479 | 82.363 | 1.00 | 30.42 | C |
| ATOM | 5637 | OG | SER | D | 95 | -9.598 | -43.754 | 82.570 | 1.00 | 42.60 | O |
| ATOM | 5638 | N | PRO | D | 96 | -8.018 | -43.796 | 85.228 | 1.00 | 25.83 | N |
| ATOM | 5639 | CA | PRO | D | 96 | -9.421 | -43.894 | 85.652 | 1.00 | 27.86 | C |
| ATOM | 5640 | C | PRO | D | 96 | -9.883 | -42.764 | 86.572 | 1.00 | 29.69 | C |
| ATOM | 5641 | O | PRO | D | 96 | -9.078 | -42.248 | 87.363 | 1.00 | 29.09 | O |
| ATOM | 5642 | CB | PRO | D | 96 | -9.454 | -45.229 | 86.397 | 1.00 | 32.44 | C |
| ATOM | 5643 | CG | PRO | D | 96 | -8.081 | -45.372 | 86.953 | 1.00 | 29.69 | C |
| ATOM | 5644 | CD | PRO | D | 96 | -7.169 | -44.788 | 85.901 | 1.00 | 25.85 | C |
| ATOM | 5645 | N | TRP | D | 97 | -11.153 | -42.369 | 86.470 | 1.00 | 27.79 | N |
| ATOM | 5646 | CA | TRP | D | 97 | -11.727 | -41.539 | 87.513 | 1.00 | 23.51 | C |
| ATOM | 5647 | C | TRP | D | 97 | -11.541 | -42.239 | 88.855 | 1.00 | 27.99 | C |
| ATOM | 5648 | O | TRP | D | 97 | -11.697 | -43.459 | 88.964 | 1.00 | 26.14 | O |
| ATOM | 5649 | CB | TRP | D | 97 | -13.205 | -41.292 | 87.265 | 1.00 | 26.18 | C |
| ATOM | 5650 | CG | TRP | D | 97 | -13.628 | -40.489 | 86.072 | 1.00 | 26.55 | C |
| ATOM | 5651 | CD1 | TRP | D | 97 | -14.907 | -40.229 | 85.726 | 1.00 | 23.15 | C |
| ATOM | 5652 | CD2 | TRP | D | 97 | -12.804 | -39.864 | 85.056 | 1.00 | 32.73 | C |
| ATOM | 5653 | NE1 | TRP | D | 97 | -14.953 | -39.474 | 84.583 | 1.00 | 28.40 | N |
| ATOM | 5654 | CE2 | TRP | D | 97 | -13.683 | -39.228 | 84.151 | 1.00 | 27.44 | C |
| ATOM | 5655 | CE3 | TRP | D | 97 | -11.419 | -39.760 | 84.835 | 1.00 | 25.48 | C |
| ATOM | 5656 | CZ2 | TRP | D | 97 | -13.232 | -38.510 | 83.046 | 1.00 | 28.49 | C |
| ATOM | 5657 | CZ3 | TRP | D | 97 | -10.975 | -39.045 | 83.734 | 1.00 | 23.95 | C |
| ATOM | 5658 | CH2 | TRP | D | 97 | -11.876 | -38.438 | 82.849 | 1.00 | 28.01 | C |
| ATOM | 5659 | N | THR | D | 98 | -11.174 | -41.469 | 89.877 | 1.00 | 28.12 | N |
| ATOM | 5660 | CA | THR | D | 98 | -10.911 | -42.018 | 91.196 | 1.00 | 28.04 | C |
| ATOM | 5661 | C | THR | D | 98 | -11.606 | -41.178 | 92.255 | 1.00 | 27.40 | C |
| ATOM | 5662 | O | THR | D | 98 | -11.797 | -39.972 | 92.089 | 1.00 | 24.69 | O |
| ATOM | 5663 | CB | THR | D | 98 | -9.390 | -42.067 | 91.493 | 1.00 | 30.50 | C |
| ATOM | 5664 | OG1 | THR | D | 98 | -8.813 | -40.781 | 91.230 | 1.00 | 29.88 | O |
| ATOM | 5665 | CG2 | THR | D | 98 | -8.687 | -43.122 | 90.648 | 1.00 | 24.32 | C |
| ATOM | 5666 | N | PHE | D | 99 | -11.928 | -41.821 | 93.372 | 1.00 | 28.87 | N |
| ATOM | 5667 | CA | PHE | D | 99 | -12.603 | -41.187 | 94.492 | 1.00 | 28.08 | C |
| ATOM | 5668 | C | PHE | D | 99 | -11.686 | -41.223 | 95.704 | 1.00 | 31.16 | C |
| ATOM | 5669 | O | PHE | D | 99 | -10.854 | -42.128 | 95.835 | 1.00 | 28.56 | O |
| ATOM | 5670 | CB | PHE | D | 99 | -13.931 | -41.891 | 94.859 | 1.00 | 22.78 | C |
| ATOM | 5671 | CG | PHE | D | 99 | -15.028 | -41.690 | 93.863 | 1.00 | 25.65 | C |
| ATOM | 5672 | CD1 | PHE | D | 99 | -15.156 | -42.534 | 92.778 | 1.00 | 25.60 | C |
| ATOM | 5673 | CD2 | PHE | D | 99 | -15.945 | -40.658 | 94.015 | 1.00 | 25.03 | C |
| ATOM | 5674 | CE1 | PHE | D | 99 | -16.171 | -42.362 | 91.864 | 1.00 | 23.95 | C |
| ATOM | 5675 | CE2 | PHE | D | 99 | -16.957 | -40.474 | 93.100 | 1.00 | 25.16 | C |

| | | | | | | | | | | | |
|---|---|---|---|---|---|---|---|---|---|---|---|
| ATOM | 5676 | CZ | PHE | D | 99 | -17.066 | -41.336 | 92.011 | 1.00 | 23.49 | C |
| ATOM | 5677 | N | GLY | D | 100 | -11.852 | -40.218 | 96.597 | 1.00 | 26.03 | N |
| ATOM | 5678 | CA | GLY | D | 100 | -11.280 | -40.302 | 97.918 | 1.00 | 25.35 | C |
| ATOM | 5679 | C | GLY | D | 100 | -12.110 | -41.227 | 98.769 | 1.00 | 29.26 | C |
| ATOM | 5680 | O | GLY | D | 100 | -13.227 | -41.602 | 98.411 | 1.00 | 31.89 | O |
| ATOM | 5681 | N | GLN | D | 101 | -11.553 | -41.623 | 99.901 | 1.00 | 26.91 | N |
| ATOM | 5682 | CA | GLN | D | 101 | -12.213 | -42.607 | 100.740 | 1.00 | 29.14 | C |
| ATOM | 5683 | C | GLN | D | 101 | -13.218 | -42.004 | 101.718 | 1.00 | 33.18 | C |
| ATOM | 5684 | O | GLN | D | 101 | -13.871 | -42.757 | 102.446 | 1.00 | 31.79 | O |
| ATOM | 5685 | CB | GLN | D | 101 | -11.163 | -43.426 | 101.480 | 1.00 | 37.07 | C |
| ATOM | 5686 | CG | GLN | D | 101 | -10.254 | -44.203 | 100.497 | 1.00 | 50.48 | C |
| ATOM | 5687 | CD | GLN | D | 101 | -10.653 | -45.667 | 100.316 | 1.00 | 57.12 | C |
| ATOM | 5688 | OE1 | GLN | D | 101 | -10.139 | -46.549 | 101.022 | 1.00 | 66.11 | O |
| ATOM | 5689 | NE2 | GLN | D | 101 | -11.564 | -45.935 | 99.371 | 1.00 | 48.92 | N |
| ATOM | 5690 | N | GLY | D | 102 | -13.381 | -40.691 | 101.740 | 1.00 | 27.41 | N |
| ATOM | 5691 | CA | GLY | D | 102 | -14.489 | -40.090 | 102.452 | 1.00 | 24.59 | C |
| ATOM | 5692 | C | GLY | D | 102 | -14.084 | -39.542 | 103.806 | 1.00 | 24.13 | C |
| ATOM | 5693 | O | GLY | D | 102 | -13.158 | -40.032 | 104.454 | 1.00 | 30.96 | O |
| ATOM | 5694 | N | THR | D | 103 | -14.767 | -38.478 | 104.226 | 1.00 | 26.72 | N |
| ATOM | 5695 | CA | THR | D | 103 | -14.681 | -37.929 | 105.577 | 1.00 | 25.89 | C |
| ATOM | 5696 | C | THR | D | 103 | -16.080 | -37.902 | 106.164 | 1.00 | 25.39 | C |
| ATOM | 5697 | O | THR | D | 103 | -16.997 | -37.334 | 105.560 | 1.00 | 19.85 | O |
| ATOM | 5698 | CB | THR | D | 103 | -14.116 | -36.500 | 105.626 | 1.00 | 28.14 | C |
| ATOM | 5699 | OG1 | THR | D | 103 | -12.740 | -36.480 | 105.221 | 1.00 | 28.24 | O |
| ATOM | 5700 | CG2 | THR | D | 103 | -14.269 | -35.913 | 107.044 | 1.00 | 26.41 | C |
| ATOM | 5701 | N | LYS | D | 104 | -16.230 | -38.501 | 107.344 | 1.00 | 27.38 | N |
| ATOM | 5702 | CA | LYS | D | 104 | -17.489 | -38.491 | 108.073 | 1.00 | 27.31 | C |
| ATOM | 5703 | C | LYS | D | 104 | -17.515 | -37.253 | 108.966 | 1.00 | 28.48 | C |
| ATOM | 5704 | O | LYS | D | 104 | -16.604 | -37.032 | 109.773 | 1.00 | 28.70 | O |
| ATOM | 5705 | CB | LYS | D | 104 | -17.661 | -39.787 | 108.875 | 1.00 | 25.02 | C |
| ATOM | 5706 | CG | LYS | D | 104 | -19.009 | -39.923 | 109.600 | 1.00 | 29.05 | C |
| ATOM | 5707 | CD | LYS | D | 104 | -19.050 | -41.169 | 110.488 | 1.00 | 32.39 | C |
| ATOM | 5708 | CE | LYS | D | 104 | -20.386 | -41.319 | 111.265 | 1.00 | 49.50 | C |
| ATOM | 5709 | NZ | LYS | D | 104 | -20.557 | -42.613 | 112.082 | 1.00 | 40.68 | N1+ |
| ATOM | 5710 | N | VAL | D | 105 | -18.520 | -36.416 | 108.767 | 1.00 | 26.83 | N |
| ATOM | 5711 | CA | VAL | D | 105 | -18.791 | -35.267 | 109.621 | 1.00 | 27.60 | C |
| ATOM | 5712 | C | VAL | D | 105 | -19.905 | -35.666 | 110.574 | 1.00 | 25.51 | C |
| ATOM | 5713 | O | VAL | D | 105 | -21.054 | -35.808 | 110.157 | 1.00 | 24.45 | O |
| ATOM | 5714 | CB | VAL | D | 105 | -19.176 | -34.041 | 108.785 | 1.00 | 27.65 | C |
| ATOM | 5715 | CG1 | VAL | D | 105 | -19.528 | -32.864 | 109.682 | 1.00 | 29.48 | C |
| ATOM | 5716 | CG2 | VAL | D | 105 | -18.026 | -33.694 | 107.825 | 1.00 | 22.38 | C |
| ATOM | 5717 | N | GLU | D | 106 | -19.579 | -35.866 | 111.851 | 1.00 | 31.50 | N |
| ATOM | 5718 | CA | GLU | D | 106 | -20.568 | -36.309 | 112.826 | 1.00 | 28.01 | C |
| ATOM | 5719 | C | GLU | D | 106 | -20.867 | -35.198 | 113.826 | 1.00 | 31.26 | C |
| ATOM | 5720 | O | GLU | D | 106 | -20.043 | -34.311 | 114.068 | 1.00 | 29.59 | O |
| ATOM | 5721 | CB | GLU | D | 106 | -20.137 | -37.601 | 113.551 | 1.00 | 29.60 | C |
| ATOM | 5722 | CG | GLU | D | 106 | -18.949 | -37.531 | 114.521 | 1.00 | 33.75 | C |
| ATOM | 5723 | CD | GLU | D | 106 | -19.264 | -38.095 | 115.928 | 1.00 | 34.42 | C |
| ATOM | 5724 | OE1 | GLU | D | 106 | -19.053 | -37.378 | 116.921 | 1.00 | 40.25 | O |
| ATOM | 5725 | OE2 | GLU | D | 106 | -19.688 | -39.257 | 116.061 | 1.00 | 33.48 | O1- |
| ATOM | 5726 | N | ILE | D | 107 | -22.075 | -35.258 | 114.387 | 1.00 | 29.99 | N |
| ATOM | 5727 | CA | ILE | D | 107 | -22.541 | -34.269 | 115.347 | 1.00 | 27.29 | C |
| ATOM | 5728 | C | ILE | D | 107 | -21.815 | -34.467 | 116.668 | 1.00 | 32.55 | C |
| ATOM | 5729 | O | ILE | D | 107 | -21.916 | -35.529 | 117.291 | 1.00 | 29.14 | O |
| ATOM | 5730 | CB | ILE | D | 107 | -24.048 | -34.396 | 115.581 | 1.00 | 29.36 | C |
| ATOM | 5731 | CG1 | ILE | D | 107 | -24.859 | -33.985 | 114.363 | 1.00 | 29.05 | C |
| ATOM | 5732 | CG2 | ILE | D | 107 | -24.428 | -33.602 | 116.816 | 1.00 | 30.42 | C |
| ATOM | 5733 | CD1 | ILE | D | 107 | -26.308 | -34.346 | 114.489 | 1.00 | 29.83 | C |
| ATOM | 5734 | N | LYS | D | 108 | -21.120 | -33.428 | 117.121 | 1.00 | 35.45 | N |
| ATOM | 5735 | CA | LYS | D | 108 | -20.531 | -33.410 | 118.452 | 1.00 | 32.78 | C |
| ATOM | 5736 | C | LYS | D | 108 | -21.581 | -32.996 | 119.483 | 1.00 | 36.07 | C |
| ATOM | 5737 | O | LYS | D | 108 | -22.303 | -32.011 | 119.282 | 1.00 | 36.02 | O |
| ATOM | 5738 | CB | LYS | D | 108 | -19.355 | -32.445 | 118.484 | 1.00 | 34.28 | C |
| ATOM | 5739 | CG | LYS | D | 108 | -18.623 | -32.454 | 119.791 | 1.00 | 36.31 | C |
| ATOM | 5740 | CD | LYS | D | 108 | -17.705 | -31.273 | 119.907 | 1.00 | 36.98 | C |
| ATOM | 5741 | CE | LYS | D | 108 | -16.504 | -31.728 | 120.659 | 1.00 | 44.81 | C |
| ATOM | 5742 | NZ | LYS | D | 108 | -16.855 | -33.094 | 121.165 | 1.00 | 40.45 | N1+ |
| ATOM | 5743 | N | ARG | D | 109 | -21.682 | -33.760 | 120.572 | 1.00 | 31.47 | N |
| ATOM | 5744 | CA | ARG | D | 109 | -22.636 | -33.469 | 121.642 | 1.00 | 32.33 | C |
| ATOM | 5745 | C | ARG | D | 109 | -22.004 | -33.853 | 122.962 | 1.00 | 30.94 | C |
| ATOM | 5746 | O | ARG | D | 109 | -20.848 | -34.273 | 123.008 | 1.00 | 35.21 | O |

| | | | | | | | | | | | |
|---|---|---|---|---|---|---|---|---|---|---|---|
| ATOM | 5747 | CB | ARG | D | 109 | -23.957 | -34.203 | 121.464 | 1.00 | 26.67 | C |
| ATOM | 5748 | CG | ARG | D | 109 | -23.826 | -35.711 | 121.380 | 1.00 | 32.22 | C |
| ATOM | 5749 | CD | ARG | D | 109 | -25.081 | -36.397 | 121.890 | 1.00 | 28.47 | C |
| ATOM | 5750 | NE | ARG | D | 109 | -25.123 | -36.331 | 123.335 | 1.00 | 32.52 | N |
| ATOM | 5751 | CZ | ARG | D | 109 | -26.235 | -36.307 | 124.056 | 1.00 | 28.79 | C |
| ATOM | 5752 | NH1 | ARG | D | 109 | -26.159 | -36.242 | 125.377 | 1.00 | 28.77 | N1+ |
| ATOM | 5753 | NH2 | ARG | D | 109 | -27.406 | -36.312 | 123.460 | 1.00 | 25.19 | N |
| ATOM | 5754 | N | THR | D | 110 | -22.765 | -33.713 | 124.040 | 1.00 | 27.95 | N |
| ATOM | 5755 | CA | THR | D | 110 | -22.224 | -34.032 | 125.356 | 1.00 | 33.38 | C |
| ATOM | 5756 | C | THR | D | 110 | -22.147 | -35.538 | 125.572 | 1.00 | 32.27 | C |
| ATOM | 5757 | O | THR | D | 110 | -22.956 | -36.306 | 125.044 | 1.00 | 32.94 | O |
| ATOM | 5758 | CB | THR | D | 110 | -23.068 | -33.429 | 126.468 | 1.00 | 32.63 | C |
| ATOM | 5759 | OG1 | THR | D | 110 | -24.415 | -33.917 | 126.356 | 1.00 | 28.33 | O |
| ATOM | 5760 | CG2 | THR | D | 110 | -23.037 | -31.936 | 126.370 | 1.00 | 31.15 | C |
| ATOM | 5761 | N | VAL | D | 111 | -21.145 | -35.946 | 126.354 | 1.00 | 29.24 | N |
| ATOM | 5762 | CA | VAL | D | 111 | -20.999 | -37.339 | 126.750 | 1.00 | 29.80 | C |
| ATOM | 5763 | C | VAL | D | 111 | -22.298 | -37.861 | 127.374 | 1.00 | 31.10 | C |
| ATOM | 5764 | O | VAL | D | 111 | -22.978 | -37.165 | 128.137 | 1.00 | 36.10 | O |
| ATOM | 5765 | CB | VAL | D | 111 | -19.794 | -37.464 | 127.699 | 1.00 | 29.10 | C |
| ATOM | 5766 | CG1 | VAL | D | 111 | -19.627 | -38.899 | 128.181 | 1.00 | 32.56 | C |
| ATOM | 5767 | CG2 | VAL | D | 111 | -18.522 | -36.988 | 126.978 | 1.00 | 24.86 | C |
| ATOM | 5768 | N | ALA | D | 112 | -22.665 | -39.086 | 127.004 | 1.00 | 30.83 | N |
| ATOM | 5769 | CA | ALA | D | 112 | -23.886 | -39.735 | 127.460 | 1.00 | 27.58 | C |
| ATOM | 5770 | C | ALA | D | 112 | -23.597 | -41.211 | 127.684 | 1.00 | 30.58 | C |
| ATOM | 5771 | O | ALA | D | 112 | -23.169 | -41.899 | 126.759 | 1.00 | 35.39 | O |
| ATOM | 5772 | CB | ALA | D | 112 | -25.003 | -39.561 | 126.435 | 1.00 | 26.61 | C |
| ATOM | 5773 | N | ALA | D | 113 | -23.828 | -41.703 | 128.896 | 1.00 | 32.52 | N |
| ATOM | 5774 | CA | ALA | D | 113 | -23.548 | -43.102 | 129.185 | 1.00 | 31.32 | C |
| ATOM | 5775 | C | ALA | D | 113 | -24.588 | -44.017 | 128.544 | 1.00 | 31.91 | C |
| ATOM | 5776 | O | ALA | D | 113 | -25.757 | -43.648 | 128.414 | 1.00 | 30.66 | O |
| ATOM | 5777 | CB | ALA | D | 113 | -23.530 | -43.351 | 130.685 | 1.00 | 26.64 | C |
| ATOM | 5778 | N | PRO | D | 114 | -24.193 | -45.222 | 128.150 | 1.00 | 29.42 | N |
| ATOM | 5779 | CA | PRO | D | 114 | -25.182 | -46.176 | 127.649 | 1.00 | 29.26 | C |
| ATOM | 5780 | C | PRO | D | 114 | -26.021 | -46.730 | 128.781 | 1.00 | 29.73 | C |
| ATOM | 5781 | O | PRO | D | 114 | -25.589 | -46.804 | 129.925 | 1.00 | 28.84 | O |
| ATOM | 5782 | CB | PRO | D | 114 | -24.334 | -47.277 | 127.005 | 1.00 | 27.37 | C |
| ATOM | 5783 | CG | PRO | D | 114 | -23.042 | -47.236 | 127.766 | 1.00 | 30.02 | C |
| ATOM | 5784 | CD | PRO | D | 114 | -22.823 | -45.773 | 128.131 | 1.00 | 29.01 | C |
| ATOM | 5785 | N | SER | D | 115 | -27.255 | -47.068 | 128.454 | 1.00 | 28.42 | N |
| ATOM | 5786 | CA | SER | D | 115 | -28.020 | -47.994 | 129.254 | 1.00 | 24.77 | C |
| ATOM | 5787 | C | SER | D | 115 | -27.788 | -49.394 | 128.706 | 1.00 | 27.46 | C |
| ATOM | 5788 | O | SER | D | 115 | -27.741 | -49.606 | 127.489 | 1.00 | 25.81 | O |
| ATOM | 5789 | CB | SER | D | 115 | -29.502 | -47.638 | 129.221 | 1.00 | 27.83 | C |
| ATOM | 5790 | OG | SER | D | 115 | -29.644 | -46.253 | 129.452 | 1.00 | 30.47 | O |
| ATOM | 5791 | N | VAL | D | 116 | -27.612 | -50.342 | 129.612 | 1.00 | 26.91 | N |
| ATOM | 5792 | CA | VAL | D | 116 | -27.156 | -51.679 | 129.275 | 1.00 | 27.26 | C |
| ATOM | 5793 | C | VAL | D | 116 | -28.258 | -52.657 | 129.636 | 1.00 | 26.73 | C |
| ATOM | 5794 | O | VAL | D | 116 | -28.800 | -52.605 | 130.742 | 1.00 | 33.45 | O |
| ATOM | 5795 | CB | VAL | D | 116 | -25.850 | -52.032 | 130.010 | 1.00 | 29.29 | C |
| ATOM | 5796 | CG1 | VAL | D | 116 | -25.339 | -53.387 | 129.535 | 1.00 | 30.85 | C |
| ATOM | 5797 | CG2 | VAL | D | 116 | -24.814 | -50.930 | 129.803 | 1.00 | 24.64 | C |
| ATOM | 5798 | N | PHE | D | 117 | -28.591 | -53.535 | 128.703 | 1.00 | 25.90 | N |
| ATOM | 5799 | CA | PHE | D | 117 | -29.585 | -54.573 | 128.907 | 1.00 | 25.96 | C |
| ATOM | 5800 | C | PHE | D | 117 | -29.037 | -55.838 | 128.293 | 1.00 | 27.24 | C |
| ATOM | 5801 | O | PHE | D | 117 | -28.399 | -55.788 | 127.240 | 1.00 | 29.01 | O |
| ATOM | 5802 | CB | PHE | D | 117 | -30.930 | -54.282 | 128.244 | 1.00 | 26.04 | C |
| ATOM | 5803 | CG | PHE | D | 117 | -31.510 | -52.943 | 128.560 | 1.00 | 29.45 | C |
| ATOM | 5804 | CD1 | PHE | D | 117 | -31.119 | -51.810 | 127.851 | 1.00 | 28.96 | C |
| ATOM | 5805 | CD2 | PHE | D | 117 | -32.523 | -52.826 | 129.497 | 1.00 | 27.92 | C |
| ATOM | 5806 | CE1 | PHE | D | 117 | -31.698 | -50.590 | 128.119 | 1.00 | 31.67 | C |
| ATOM | 5807 | CE2 | PHE | D | 117 | -33.108 | -51.607 | 129.767 | 1.00 | 28.75 | C |
| ATOM | 5808 | CZ | PHE | D | 117 | -32.705 | -50.490 | 129.086 | 1.00 | 31.59 | C |
| ATOM | 5809 | N | ILE | D | 118 | -29.288 | -56.966 | 128.938 | 1.00 | 28.76 | N |
| ATOM | 5810 | CA | ILE | D | 118 | -28.835 | -58.252 | 128.429 | 1.00 | 30.93 | C |
| ATOM | 5811 | C | ILE | D | 118 | -30.053 | -59.149 | 128.291 | 1.00 | 27.51 | C |
| ATOM | 5812 | O | ILE | D | 118 | -30.949 | -59.115 | 129.134 | 1.00 | 28.60 | O |
| ATOM | 5813 | CB | ILE | D | 118 | -27.764 | -58.872 | 129.348 | 1.00 | 29.79 | C |
| ATOM | 5814 | CG1 | ILE | D | 118 | -27.230 | -60.167 | 128.757 | 1.00 | 33.20 | C |
| ATOM | 5815 | CG2 | ILE | D | 118 | -28.321 | -59.122 | 130.738 | 1.00 | 28.76 | C |
| ATOM | 5816 | CD1 | ILE | D | 118 | -26.243 | -60.873 | 129.679 | 1.00 | 35.26 | C |
| ATOM | 5817 | N | PHE | D | 119 | -30.108 | -59.909 | 127.204 | 1.00 | 28.10 | N |

| | | | | | | | | | | | |
|---|---|---|---|---|---|---|---|---|---|---|---|
| ATOM | 5818 | CA | PHE | D | 119 | -31.241 | -60.753 | 126.853 | 1.00 | 27.79 | C |
| ATOM | 5819 | C | PHE | D | 119 | -30.812 | -62.209 | 126.778 | 1.00 | 32.91 | C |
| ATOM | 5820 | O | PHE | D | 119 | -29.869 | -62.544 | 126.044 | 1.00 | 31.43 | O |
| ATOM | 5821 | CB | PHE | D | 119 | -31.858 | -60.343 | 125.507 | 1.00 | 29.66 | C |
| ATOM | 5822 | CG | PHE | D | 119 | -32.413 | -58.970 | 125.513 | 1.00 | 31.25 | C |
| ATOM | 5823 | CD1 | PHE | D | 119 | -33.683 | -58.729 | 126.018 | 1.00 | 26.70 | C |
| ATOM | 5824 | CD2 | PHE | D | 119 | -31.659 | -57.909 | 125.051 | 1.00 | 27.96 | C |
| ATOM | 5825 | CE1 | PHE | D | 119 | -34.198 | -57.450 | 126.055 | 1.00 | 28.84 | C |
| ATOM | 5826 | CE2 | PHE | D | 119 | -32.165 | -56.635 | 125.086 | 1.00 | 30.39 | C |
| ATOM | 5827 | CZ | PHE | D | 119 | -33.445 | -56.399 | 125.584 | 1.00 | 27.28 | C |
| ATOM | 5828 | N | PRO | D | 120 | -31.482 | -63.097 | 127.497 | 1.00 | 35.56 | N |
| ATOM | 5829 | CA | PRO | D | 120 | -31.187 | -64.530 | 127.394 | 1.00 | 36.54 | C |
| ATOM | 5830 | C | PRO | D | 120 | -31.661 | -65.088 | 126.065 | 1.00 | 34.90 | C |
| ATOM | 5831 | O | PRO | D | 120 | -32.495 | -64.470 | 125.387 | 1.00 | 33.38 | O |
| ATOM | 5832 | CB | PRO | D | 120 | -31.987 | -65.138 | 128.557 | 1.00 | 36.73 | C |
| ATOM | 5833 | CG | PRO | D | 120 | -32.403 | -63.956 | 129.408 | 1.00 | 42.07 | C |
| ATOM | 5834 | CD | PRO | D | 120 | -32.546 | -62.814 | 128.466 | 1.00 | 32.56 | C |
| ATOM | 5835 | N | PRO | D | 121 | -31.155 | -66.248 | 125.655 | 1.00 | 33.49 | N |
| ATOM | 5836 | CA | PRO | D | 121 | -31.711 | -66.900 | 124.463 | 1.00 | 35.36 | C |
| ATOM | 5837 | C | PRO | D | 121 | -33.137 | -67.359 | 124.718 | 1.00 | 32.32 | C |
| ATOM | 5838 | O | PRO | D | 121 | -33.510 | -67.738 | 125.829 | 1.00 | 33.72 | O |
| ATOM | 5839 | CB | PRO | D | 121 | -30.772 | -68.091 | 124.225 | 1.00 | 34.18 | C |
| ATOM | 5840 | CG | PRO | D | 121 | -30.187 | -68.357 | 125.588 | 1.00 | 34.16 | C |
| ATOM | 5841 | CD | PRO | D | 121 | -30.062 | -67.026 | 126.262 | 1.00 | 28.95 | C |
| ATOM | 5842 | N | SER | D | 122 | -33.949 | -67.282 | 123.672 | 1.00 | 37.30 | N |
| ATOM | 5843 | CA | SER | D | 122 | -35.326 | -67.733 | 123.761 | 1.00 | 35.72 | C |
| ATOM | 5844 | C | SER | D | 122 | -35.386 | -69.261 | 123.796 | 1.00 | 40.60 | C |
| ATOM | 5845 | O | SER | D | 122 | -34.510 | -69.961 | 123.264 | 1.00 | 37.13 | O |
| ATOM | 5846 | CB | SER | D | 122 | -36.141 | -67.176 | 122.589 | 1.00 | 30.63 | C |
| ATOM | 5847 | OG | SER | D | 122 | -35.821 | -67.837 | 121.384 | 1.00 | 34.59 | O |
| ATOM | 5848 | N | ASP | D | 123 | -36.413 | -69.776 | 124.481 | 1.00 | 44.14 | N |
| ATOM | 5849 | CA | ASP | D | 123 | -36.631 | -71.221 | 124.525 | 1.00 | 47.46 | C |
| ATOM | 5850 | C | ASP | D | 123 | -36.913 | -71.793 | 123.142 | 1.00 | 46.92 | C |
| ATOM | 5851 | O | ASP | D | 123 | -36.606 | -72.964 | 122.884 | 1.00 | 49.21 | O |
| ATOM | 5852 | CB | ASP | D | 123 | -37.776 | -71.541 | 125.478 | 1.00 | 54.79 | C |
| ATOM | 5853 | CG | ASP | D | 123 | -37.352 | -71.471 | 126.921 | 1.00 | 61.77 | C |
| ATOM | 5854 | OD1 | ASP | D | 123 | -36.195 | -71.849 | 127.207 | 1.00 | 56.99 | O |
| ATOM | 5855 | OD2 | ASP | D | 123 | -38.161 | -71.015 | 127.763 | 1.00 | 71.23 | O1- |
| ATOM | 5856 | N | GLU | D | 124 | -37.480 | -70.978 | 122.241 | 1.00 | 45.60 | N |
| ATOM | 5857 | CA | GLU | D | 124 | -37.753 | -71.411 | 120.869 | 1.00 | 49.64 | C |
| ATOM | 5858 | C | GLU | D | 124 | -36.463 | -71.644 | 120.084 | 1.00 | 50.62 | C |
| ATOM | 5859 | O | GLU | D | 124 | -36.383 | -72.565 | 119.259 | 1.00 | 49.45 | O |
| ATOM | 5860 | CB | GLU | D | 124 | -38.613 | -70.365 | 120.168 | 1.00 | 45.37 | C |
| ATOM | 5861 | CG | GLU | D | 124 | -39.053 | -70.740 | 118.759 | 1.00 | 60.55 | C |
| ATOM | 5862 | CD | GLU | D | 124 | -39.635 | -69.542 | 117.988 | 1.00 | 74.03 | C |
| ATOM | 5863 | OE1 | GLU | D | 124 | -39.948 | -68.502 | 118.626 | 1.00 | 67.91 | O |
| ATOM | 5864 | OE2 | GLU | D | 124 | -39.760 | -69.633 | 116.740 | 1.00 | 80.45 | O1- |
| ATOM | 5865 | N | GLN | D | 125 | -35.452 | -70.799 | 120.309 | 1.00 | 44.68 | N |
| ATOM | 5866 | CA | GLN | D | 125 | -34.161 | -70.979 | 119.660 | 1.00 | 44.38 | C |
| ATOM | 5867 | C | GLN | D | 125 | -33.365 | -72.110 | 120.296 | 1.00 | 44.17 | C |
| ATOM | 5868 | O | GLN | D | 125 | -32.617 | -72.803 | 119.596 | 1.00 | 44.30 | O |
| ATOM | 5869 | CB | GLN | D | 125 | -33.353 | -69.677 | 119.726 | 1.00 | 38.62 | C |
| ATOM | 5870 | CG | GLN | D | 125 | -32.017 | -69.721 | 118.987 | 1.00 | 34.83 | C |
| ATOM | 5871 | CD | GLN | D | 125 | -31.067 | -68.656 | 119.471 | 1.00 | 36.06 | C |
| ATOM | 5872 | OE1 | GLN | D | 125 | -31.232 | -68.116 | 120.570 | 1.00 | 37.90 | O |
| ATOM | 5873 | NE2 | GLN | D | 125 | -30.080 | -68.328 | 118.652 | 1.00 | 40.51 | N |
| ATOM | 5874 | N | LEU | D | 126 | -33.515 | -72.316 | 121.606 | 1.00 | 39.87 | N |
| ATOM | 5875 | CA | LEU | D | 126 | -32.798 | -73.405 | 122.254 | 1.00 | 43.24 | C |
| ATOM | 5876 | C | LEU | D | 126 | -33.200 | -74.756 | 121.683 | 1.00 | 49.92 | C |
| ATOM | 5877 | O | LEU | D | 126 | -32.343 | -75.623 | 121.467 | 1.00 | 50.55 | O |
| ATOM | 5878 | CB | LEU | D | 126 | -33.035 | -73.372 | 123.755 | 1.00 | 37.51 | C |
| ATOM | 5879 | CG | LEU | D | 126 | -32.229 | -72.275 | 124.411 | 1.00 | 38.73 | C |
| ATOM | 5880 | CD1 | LEU | D | 126 | -32.542 | -72.240 | 125.881 | 1.00 | 34.97 | C |
| ATOM | 5881 | CD2 | LEU | D | 126 | -30.723 | -72.461 | 124.145 | 1.00 | 38.73 | C |
| ATOM | 5882 | N | LYS | D | 127 | -34.486 | -74.933 | 121.365 | 1.00 | 50.02 | N |
| ATOM | 5883 | CA | LYS | D | 127 | -34.931 | -76.193 | 120.777 | 1.00 | 50.42 | C |
| ATOM | 5884 | C | LYS | D | 127 | -34.041 | -76.636 | 119.621 | 1.00 | 50.52 | C |
| ATOM | 5885 | O | LYS | D | 127 | -33.827 | -77.838 | 119.434 | 1.00 | 61.34 | O |
| ATOM | 5886 | CB | LYS | D | 127 | -36.378 | -76.066 | 120.287 | 1.00 | 54.82 | C |
| ATOM | 5887 | CG | LYS | D | 127 | -37.364 | -75.491 | 121.309 | 1.00 | 56.44 | C |
| ATOM | 5888 | CD | LYS | D | 127 | -38.834 | -75.811 | 120.959 | 1.00 | 64.32 | C |

| | | | | | | | | | | | |
|---|---|---|---|---|---|---|---|---|---|---|---|
| ATOM | 5889 | CE | LYS | D | 127 | -39.297 | -75.170 | 119.628 | 1.00 | 72.71 | C |
| ATOM | 5890 | NZ | LYS | D | 127 | -39.699 | -76.161 | 118.571 | 1.00 | 64.97 | N1+ |
| ATOM | 5891 | N | SER | D | 128 | -33.470 | -75.687 | 118.880 | 1.00 | 50.62 | N |
| ATOM | 5892 | CA | SER | D | 128 | -32.691 | -75.949 | 117.674 | 1.00 | 48.62 | C |
| ATOM | 5893 | C | SER | D | 128 | -31.181 | -76.031 | 117.919 | 1.00 | 52.68 | C |
| ATOM | 5894 | O | SER | D | 128 | -30.403 | -76.019 | 116.957 | 1.00 | 53.36 | O |
| ATOM | 5895 | CB | SER | D | 128 | -32.998 | -74.877 | 116.626 | 1.00 | 51.45 | C |
| ATOM | 5896 | OG | SER | D | 128 | -32.539 | -73.605 | 117.065 | 1.00 | 54.76 | O |
| ATOM | 5897 | N | GLY | D | 129 | -30.742 | -76.074 | 119.169 | 1.00 | 49.90 | N |
| ATOM | 5898 | CA | GLY | D | 129 | -29.355 | -76.368 | 119.452 | 1.00 | 48.29 | C |
| ATOM | 5899 | C | GLY | D | 129 | -28.416 | -75.187 | 119.533 | 1.00 | 52.26 | C |
| ATOM | 5900 | O | GLY | D | 129 | -27.243 | -75.382 | 119.882 | 1.00 | 50.58 | O |
| ATOM | 5901 | N | THR | D | 130 | -28.886 | -73.969 | 119.259 | 1.00 | 49.43 | N |
| ATOM | 5902 | CA | THR | D | 130 | -28.043 | -72.784 | 119.302 | 1.00 | 44.36 | C |
| ATOM | 5903 | C | THR | D | 130 | -28.614 | -71.774 | 120.285 | 1.00 | 44.57 | C |
| ATOM | 5904 | O | THR | D | 130 | -29.830 | -71.596 | 120.369 | 1.00 | 45.91 | O |
| ATOM | 5905 | CB | THR | D | 130 | -27.942 | -72.142 | 117.926 | 1.00 | 49.85 | C |
| ATOM | 5906 | OG1 | THR | D | 130 | -27.337 | -73.073 | 117.030 | 1.00 | 49.94 | O |
| ATOM | 5907 | CG2 | THR | D | 130 | -27.087 | -70.871 | 117.975 | 1.00 | 47.05 | C |
| ATOM | 5908 | N | ALA | D | 131 | -27.729 | -71.098 | 121.008 | 1.00 | 40.05 | N |
| ATOM | 5909 | CA | ALA | D | 131 | -28.104 | -70.036 | 121.924 | 1.00 | 38.97 | C |
| ATOM | 5910 | C | ALA | D | 131 | -27.432 | -68.727 | 121.512 | 1.00 | 37.81 | C |
| ATOM | 5911 | O | ALA | D | 131 | -26.209 | -68.683 | 121.329 | 1.00 | 38.34 | O |
| ATOM | 5912 | CB | ALA | D | 131 | -27.721 | -70.421 | 123.347 | 1.00 | 38.52 | C |
| ATOM | 5913 | N | SER | D | 132 | -28.224 | -67.667 | 121.359 | 1.00 | 33.37 | N |
| ATOM | 5914 | CA | SER | D | 132 | -27.707 | -66.314 | 121.152 | 1.00 | 30.63 | C |
| ATOM | 5915 | C | SER | D | 132 | -28.011 | -65.482 | 122.392 | 1.00 | 31.31 | C |
| ATOM | 5916 | O | SER | D | 132 | -29.166 | -65.369 | 122.800 | 1.00 | 39.28 | O |
| ATOM | 5917 | CB | SER | D | 132 | -28.321 | -65.659 | 119.913 | 1.00 | 31.88 | C |
| ATOM | 5918 | OG | SER | D | 132 | -27.968 | -66.338 | 118.727 | 1.00 | 32.64 | O |
| ATOM | 5919 | N | VAL | D | 133 | -26.999 | -64.942 | 123.008 | 1.00 | 32.01 | N |
| ATOM | 5920 | CA | VAL | D | 133 | -27.179 | -63.987 | 124.097 | 1.00 | 34.18 | C |
| ATOM | 5921 | C | VAL | D | 133 | -26.944 | -62.601 | 123.519 | 1.00 | 32.77 | C |
| ATOM | 5922 | O | VAL | D | 133 | -26.025 | -62.421 | 122.706 | 1.00 | 31.33 | O |
| ATOM | 5923 | CB | VAL | D | 133 | -26.213 | -64.294 | 125.261 | 1.00 | 34.59 | C |
| ATOM | 5924 | CG1 | VAL | D | 133 | -26.610 | -63.544 | 126.532 | 1.00 | 32.22 | C |
| ATOM | 5925 | CG2 | VAL | D | 133 | -26.180 | -65.792 | 125.517 | 1.00 | 30.53 | C |
| ATOM | 5926 | N | VAL | D | 134 | -27.781 | -61.625 | 123.893 | 1.00 | 27.21 | N |
| ATOM | 5927 | CA | VAL | D | 134 | -27.711 | -60.290 | 123.295 | 1.00 | 28.33 | C |
| ATOM | 5928 | C | VAL | D | 134 | -27.496 | -59.226 | 124.365 | 1.00 | 28.83 | C |
| ATOM | 5929 | O | VAL | D | 134 | -28.174 | -59.214 | 125.397 | 1.00 | 27.78 | O |
| ATOM | 5930 | CB | VAL | D | 134 | -28.950 | -59.955 | 122.450 | 1.00 | 27.75 | C |
| ATOM | 5931 | CG1 | VAL | D | 134 | -28.794 | -58.585 | 121.858 | 1.00 | 26.08 | C |
| ATOM | 5932 | CG2 | VAL | D | 134 | -29.096 | -60.957 | 121.319 | 1.00 | 30.78 | C |
| ATOM | 5933 | N | CYS | D | 135 | -26.565 | -58.313 | 124.092 | 1.00 | 27.54 | N |
| ATOM | 5934 | CA | CYS | D | 135 | -26.299 | -57.155 | 124.928 | 1.00 | 31.96 | C |
| ATOM | 5935 | C | CYS | D | 135 | -26.651 | -55.895 | 124.141 | 1.00 | 32.81 | C |
| ATOM | 5936 | O | CYS | D | 135 | -26.250 | -55.760 | 122.979 | 1.00 | 27.82 | O |
| ATOM | 5937 | CB | CYS | D | 135 | -24.827 | -57.154 | 125.365 | 1.00 | 32.75 | C |
| ATOM | 5938 | SG | CYS | D | 135 | -24.350 | -56.013 | 126.697 | 1.00 | 40.27 | S |
| ATOM | 5939 | N | LEU | D | 136 | -27.422 | -54.996 | 124.762 | 1.00 | 26.44 | N |
| ATOM | 5940 | CA | LEU | D | 136 | -27.826 | -53.726 | 124.161 | 1.00 | 24.84 | C |
| ATOM | 5941 | C | LEU | D | 136 | -27.168 | -52.559 | 124.898 | 1.00 | 28.43 | C |
| ATOM | 5942 | O | LEU | D | 136 | -27.375 | -52.380 | 126.107 | 1.00 | 27.53 | O |
| ATOM | 5943 | CB | LEU | D | 136 | -29.345 | -53.566 | 124.191 | 1.00 | 26.69 | C |
| ATOM | 5944 | CG | LEU | D | 136 | -29.908 | -52.191 | 123.787 | 1.00 | 30.11 | C |
| ATOM | 5945 | CD1 | LEU | D | 136 | -29.570 | -51.875 | 122.330 | 1.00 | 22.63 | C |
| ATOM | 5946 | CD2 | LEU | D | 136 | -31.439 | -52.087 | 124.042 | 1.00 | 23.90 | C |
| ATOM | 5947 | N | LEU | D | 137 | -26.403 | -51.746 | 124.169 | 1.00 | 27.96 | N |
| ATOM | 5948 | CA | LEU | D | 137 | -25.860 | -50.492 | 124.687 | 1.00 | 24.59 | C |
| ATOM | 5949 | C | LEU | D | 137 | -26.657 | -49.367 | 124.027 | 1.00 | 28.90 | C |
| ATOM | 5950 | O | LEU | D | 137 | -26.501 | -49.096 | 122.830 | 1.00 | 28.52 | O |
| ATOM | 5951 | CB | LEU | D | 137 | -24.364 | -50.380 | 124.419 | 1.00 | 20.03 | C |
| ATOM | 5952 | CG | LEU | D | 137 | -23.377 | -51.186 | 125.271 | 1.00 | 24.15 | C |
| ATOM | 5953 | CD1 | LEU | D | 137 | -23.602 | -52.697 | 125.252 | 1.00 | 20.47 | C |
| ATOM | 5954 | CD2 | LEU | D | 137 | -21.943 | -50.875 | 124.827 | 1.00 | 21.46 | C |
| ATOM | 5955 | N | ASN | D | 138 | -27.531 | -48.732 | 124.806 | 1.00 | 30.48 | N |
| ATOM | 5956 | CA | ASN | D | 138 | -28.551 | -47.836 | 124.285 | 1.00 | 28.96 | C |
| ATOM | 5957 | C | ASN | D | 138 | -28.215 | -46.368 | 124.560 | 1.00 | 31.63 | C |
| ATOM | 5958 | O | ASN | D | 138 | -27.933 | -45.994 | 125.705 | 1.00 | 33.79 | O |
| ATOM | 5959 | CB | ASN | D | 138 | -29.904 | -48.179 | 124.900 | 1.00 | 27.28 | C |

| | | | | | | | | | | | |
|---|---|---|---|---|---|---|---|---|---|---|---|
| ATOM | 5960 | CG | ASN | D | 138 | -31.052 | -47.802 | 124.004 | 1.00 | 31.25 | C |
| ATOM | 5961 | OD1 | ASN | D | 138 | -31.153 | -48.280 | 122.875 | 1.00 | 36.62 | O |
| ATOM | 5962 | ND2 | ASN | D | 138 | -31.880 | -46.874 | 124.463 | 1.00 | 36.73 | N |
| ATOM | 5963 | N | ASN | D | 139 | -28.258 | -45.549 | 123.495 | 1.00 | 33.24 | N |
| ATOM | 5964 | CA | ASN | D | 139 | -28.250 | -44.074 | 123.524 | 1.00 | 28.78 | C |
| ATOM | 5965 | C | ASN | D | 139 | -27.036 | -43.490 | 124.238 | 1.00 | 27.30 | C |
| ATOM | 5966 | O | ASN | D | 139 | -27.168 | -42.799 | 125.238 | 1.00 | 35.75 | O |
| ATOM | 5967 | CB | ASN | D | 139 | -29.520 | -43.529 | 124.171 | 1.00 | 27.41 | C |
| ATOM | 5968 | CG | ASN | D | 139 | -30.755 | -43.820 | 123.364 | 1.00 | 34.79 | C |
| ATOM | 5969 | OD1 | ASN | D | 139 | -30.693 | -44.255 | 122.215 | 1.00 | 31.67 | O |
| ATOM | 5970 | ND2 | ASN | D | 139 | -31.903 | -43.606 | 123.985 | 1.00 | 49.31 | N |
| ATOM | 5971 | N | PHE | D | 140 | -25.853 | -43.720 | 123.683 | 1.00 | 28.66 | N |
| ATOM | 5972 | CA | PHE | D | 140 | -24.631 | -43.200 | 124.279 | 1.00 | 30.52 | C |
| ATOM | 5973 | C | PHE | D | 140 | -23.895 | -42.295 | 123.301 | 1.00 | 30.45 | C |
| ATOM | 5974 | O | PHE | D | 140 | -24.127 | -42.323 | 122.089 | 1.00 | 31.04 | O |
| ATOM | 5975 | CB | PHE | D | 140 | -23.703 | -44.333 | 124.760 | 1.00 | 27.13 | C |
| ATOM | 5976 | CG | PHE | D | 140 | -23.324 | -45.299 | 123.685 | 1.00 | 30.02 | C |
| ATOM | 5977 | CD1 | PHE | D | 140 | -24.068 | -46.450 | 123.482 | 1.00 | 29.03 | C |
| ATOM | 5978 | CD2 | PHE | D | 140 | -22.224 | -45.059 | 122.868 | 1.00 | 27.79 | C |
| ATOM | 5979 | CE1 | PHE | D | 140 | -23.724 | -47.344 | 122.477 | 1.00 | 28.63 | C |
| ATOM | 5980 | CE2 | PHE | D | 140 | -21.869 | -45.950 | 121.866 | 1.00 | 25.04 | C |
| ATOM | 5981 | CZ | PHE | D | 140 | -22.619 | -47.090 | 121.666 | 1.00 | 25.93 | C |
| ATOM | 5982 | N | TYR | D | 141 | -23.033 | -41.452 | 123.855 | 1.00 | 27.31 | N |
| ATOM | 5983 | CA | TYR | D | 141 | -22.138 | -40.640 | 123.059 | 1.00 | 29.62 | C |
| ATOM | 5984 | C | TYR | D | 141 | -20.895 | -40.416 | 123.882 | 1.00 | 30.14 | C |
| ATOM | 5985 | O | TYR | D | 141 | -21.002 | -40.131 | 125.060 | 1.00 | 31.98 | O |
| ATOM | 5986 | CB | TYR | D | 141 | -22.777 | -39.292 | 122.657 | 1.00 | 30.01 | C |
| ATOM | 5987 | CG | TYR | D | 141 | -21.868 | -38.536 | 121.719 | 1.00 | 28.91 | C |
| ATOM | 5988 | CD1 | TYR | D | 141 | -21.906 | -38.764 | 120.356 | 1.00 | 24.75 | C |
| ATOM | 5989 | CD2 | TYR | D | 141 | -20.915 | -37.652 | 122.207 | 1.00 | 33.08 | C |
| ATOM | 5990 | CE1 | TYR | D | 141 | -21.055 | -38.124 | 119.505 | 1.00 | 27.43 | C |
| ATOM | 5991 | CE2 | TYR | D | 141 | -20.049 | -36.991 | 121.353 | 1.00 | 32.86 | C |
| ATOM | 5992 | CZ | TYR | D | 141 | -20.123 | -37.233 | 119.999 | 1.00 | 31.49 | C |
| ATOM | 5993 | OH | TYR | D | 141 | -19.254 | -36.584 | 119.149 | 1.00 | 29.36 | O |
| ATOM | 5994 | N | PRO | D | 142 | -19.704 | -40.530 | 123.272 | 1.00 | 33.14 | N |
| ATOM | 5995 | CA | PRO | D | 142 | -19.424 | -40.777 | 121.853 | 1.00 | 31.33 | C |
| ATOM | 5996 | C | PRO | D | 142 | -19.435 | -42.241 | 121.404 | 1.00 | 31.01 | C |
| ATOM | 5997 | O | PRO | D | 142 | -19.806 | -43.143 | 122.140 | 1.00 | 30.96 | O |
| ATOM | 5998 | CB | PRO | D | 142 | -18.028 | -40.201 | 121.691 | 1.00 | 27.46 | C |
| ATOM | 5999 | CG | PRO | D | 142 | -17.393 | -40.495 | 123.007 | 1.00 | 28.61 | C |
| ATOM | 6000 | CD | PRO | D | 142 | -18.464 | -40.243 | 124.019 | 1.00 | 27.72 | C |
| ATOM | 6001 | N | ARG | D | 143 | -18.974 | -42.430 | 120.167 | 1.00 | 35.41 | N |
| ATOM | 6002 | CA | ARG | D | 143 | -19.142 | -43.686 | 119.441 | 1.00 | 32.43 | C |
| ATOM | 6003 | C | ARG | D | 143 | -18.410 | -44.842 | 120.111 | 1.00 | 35.51 | C |
| ATOM | 6004 | O | ARG | D | 143 | -18.897 | -45.974 | 120.120 | 1.00 | 39.91 | O |
| ATOM | 6005 | CB | ARG | D | 143 | -18.633 | -43.477 | 118.018 | 1.00 | 36.16 | C |
| ATOM | 6006 | CG | ARG | D | 143 | -18.522 | -44.686 | 117.126 | 1.00 | 44.24 | C |
| ATOM | 6007 | CD | ARG | D | 143 | -19.818 | -44.835 | 116.397 | 1.00 | 40.17 | C |
| ATOM | 6008 | NE | ARG | D | 143 | -19.792 | -45.523 | 115.101 | 1.00 | 38.57 | N |
| ATOM | 6009 | CZ | ARG | D | 143 | -19.299 | -46.736 | 114.883 | 1.00 | 38.79 | C |
| ATOM | 6010 | NH1 | ARG | D | 143 | -18.689 | -47.407 | 115.852 | 1.00 | 42.03 | N1+ |
| ATOM | 6011 | NH2 | ARG | D | 143 | -19.395 | -47.265 | 113.675 | 1.00 | 43.44 | N |
| ATOM | 6012 | N | GLU | D | 144 | -17.256 | -44.580 | 120.697 | 1.00 | 31.31 | N |
| ATOM | 6013 | CA | GLU | D | 144 | -16.406 | -45.662 | 121.160 | 1.00 | 30.73 | C |
| ATOM | 6014 | C | GLU | D | 144 | -16.975 | -46.318 | 122.418 | 1.00 | 29.58 | C |
| ATOM | 6015 | O | GLU | D | 144 | -17.308 | -45.649 | 123.397 | 1.00 | 32.43 | O |
| ATOM | 6016 | CB | GLU | D | 144 | -14.994 | -45.126 | 121.391 | 1.00 | 28.89 | C |
| ATOM | 6017 | CG | GLU | D | 144 | -14.361 | -44.583 | 120.090 | 1.00 | 45.09 | C |
| ATOM | 6018 | CD | GLU | D | 144 | -14.920 | -43.198 | 119.638 | 1.00 | 55.20 | C |
| ATOM | 6019 | OE1 | GLU | D | 144 | -15.083 | -42.301 | 120.513 | 1.00 | 49.33 | O |
| ATOM | 6020 | OE2 | GLU | D | 144 | -15.215 | -43.020 | 118.417 | 1.00 | 49.10 | O1- |
| ATOM | 6021 | N | ALA | D | 145 | -17.065 | -47.640 | 122.396 | 1.00 | 25.69 | N |
| ATOM | 6022 | CA | ALA | D | 145 | -17.552 | -48.390 | 123.535 | 1.00 | 29.57 | C |
| ATOM | 6023 | C | ALA | D | 145 | -16.972 | -49.792 | 123.452 | 1.00 | 31.44 | C |
| ATOM | 6024 | O | ALA | D | 145 | -16.702 | -50.304 | 122.364 | 1.00 | 37.00 | O |
| ATOM | 6025 | CB | ALA | D | 145 | -19.095 | -48.415 | 123.584 | 1.00 | 25.18 | C |
| ATOM | 6026 | N | LYS | D | 146 | -16.792 | -50.416 | 124.607 | 1.00 | 31.28 | N |
| ATOM | 6027 | CA | LYS | D | 146 | -16.278 | -51.775 | 124.678 | 1.00 | 35.67 | C |
| ATOM | 6028 | C | LYS | D | 146 | -17.308 | -52.687 | 125.339 | 1.00 | 38.62 | C |
| ATOM | 6029 | O | LYS | D | 146 | -17.790 | -52.390 | 126.436 | 1.00 | 36.49 | O |
| ATOM | 6030 | CB | LYS | D | 146 | -14.972 | -51.810 | 125.466 | 1.00 | 33.50 | C |

| | | | | | | | | | | | |
|---|---|---|---|---|---|---|---|---|---|---|---|
| ATOM | 6031 | CG | LYS | D | 146 | -14.242 | -53.136 | 125.440 | 1.00 | 40.82 | C |
| ATOM | 6032 | CD | LYS | D | 146 | -12.883 | -53.049 | 126.167 | 1.00 | 52.07 | C |
| ATOM | 6033 | CE | LYS | D | 146 | -12.130 | -54.385 | 126.127 | 1.00 | 60.61 | C |
| ATOM | 6034 | NZ | LYS | D | 146 | -10.663 | -54.265 | 126.375 | 1.00 | 71.34 | N1+ |
| ATOM | 6035 | N | VAL | D | 147 | -17.649 | -53.786 | 124.663 | 1.00 | 35.29 | N |
| ATOM | 6036 | CA | VAL | D | 147 | -18.415 | -54.883 | 125.242 | 1.00 | 37.00 | C |
| ATOM | 6037 | C | VAL | D | 147 | -17.443 | -56.024 | 125.497 | 1.00 | 36.93 | C |
| ATOM | 6038 | O | VAL | D | 147 | -16.711 | -56.436 | 124.593 | 1.00 | 43.76 | O |
| ATOM | 6039 | CB | VAL | D | 147 | -19.568 | -55.348 | 124.331 | 1.00 | 32.86 | C |
| ATOM | 6040 | CG1 | VAL | D | 147 | -20.166 | -56.646 | 124.849 | 1.00 | 30.69 | C |
| ATOM | 6041 | CG2 | VAL | D | 147 | -20.638 | -54.322 | 124.288 | 1.00 | 31.55 | C |
| ATOM | 6042 | N | GLN | D | 148 | -17.443 | -56.529 | 126.720 | 1.00 | 35.29 | N |
| ATOM | 6043 | CA | GLN | D | 148 | -16.699 | -57.714 | 127.106 | 1.00 | 36.02 | C |
| ATOM | 6044 | C | GLN | D | 148 | -17.719 | -58.707 | 127.647 | 1.00 | 37.18 | C |
| ATOM | 6045 | O | GLN | D | 148 | -18.459 | -58.386 | 128.579 | 1.00 | 35.76 | O |
| ATOM | 6046 | CB | GLN | D | 148 | -15.637 | -57.350 | 128.153 | 1.00 | 38.02 | C |
| ATOM | 6047 | CG | GLN | D | 148 | -14.870 | -58.512 | 128.744 | 1.00 | 45.95 | C |
| ATOM | 6048 | CD | GLN | D | 148 | -13.798 | -59.062 | 127.806 | 1.00 | 52.43 | C |
| ATOM | 6049 | OE1 | GLN | D | 148 | -13.806 | -60.255 | 127.467 | 1.00 | 49.60 | O |
| ATOM | 6050 | NE2 | GLN | D | 148 | -12.857 | -58.196 | 127.398 | 1.00 | 47.07 | N |
| ATOM | 6051 | N | TRP | D | 149 | -17.787 | -59.886 | 127.037 | 1.00 | 36.10 | N |
| ATOM | 6052 | CA | TRP | D | 149 | -18.680 | -60.955 | 127.473 | 1.00 | 37.96 | C |
| ATOM | 6053 | C | TRP | D | 149 | -17.977 | -61.861 | 128.481 | 1.00 | 35.95 | C |
| ATOM | 6054 | O | TRP | D | 149 | -16.799 | -62.191 | 128.318 | 1.00 | 37.64 | O |
| ATOM | 6055 | CB | TRP | D | 149 | -19.142 | -61.800 | 126.281 | 1.00 | 33.14 | C |
| ATOM | 6056 | CG | TRP | D | 149 | -20.226 | -61.240 | 125.409 | 1.00 | 34.49 | C |
| ATOM | 6057 | CD1 | TRP | D | 149 | -20.083 | -60.721 | 124.147 | 1.00 | 33.46 | C |
| ATOM | 6058 | CD2 | TRP | D | 149 | -21.630 | -61.200 | 125.697 | 1.00 | 36.39 | C |
| ATOM | 6059 | NE1 | TRP | D | 149 | -21.301 | -60.343 | 123.643 | 1.00 | 29.56 | N |
| ATOM | 6060 | CE2 | TRP | D | 149 | -22.272 | -60.620 | 124.571 | 1.00 | 33.79 | C |
| ATOM | 6061 | CE3 | TRP | D | 149 | -22.406 | -61.584 | 126.795 | 1.00 | 31.81 | C |
| ATOM | 6062 | CZ2 | TRP | D | 149 | -23.653 | -60.408 | 124.518 | 1.00 | 28.35 | C |
| ATOM | 6063 | CZ3 | TRP | D | 149 | -23.777 | -61.367 | 126.741 | 1.00 | 37.34 | C |
| ATOM | 6064 | CH2 | TRP | D | 149 | -24.385 | -60.787 | 125.607 | 1.00 | 31.64 | C |
| ATOM | 6065 | N | LYS | D | 150 | -18.700 | -62.265 | 129.526 | 1.00 | 35.84 | N |
| ATOM | 6066 | CA | LYS | D | 150 | -18.195 | -63.242 | 130.494 | 1.00 | 39.20 | C |
| ATOM | 6067 | C | LYS | D | 150 | -19.214 | -64.355 | 130.710 | 1.00 | 34.68 | C |
| ATOM | 6068 | O | LYS | D | 150 | -20.391 | -64.086 | 130.979 | 1.00 | 33.70 | O |
| ATOM | 6069 | CB | LYS | D | 150 | -17.856 | -62.579 | 131.831 | 1.00 | 29.07 | C |
| ATOM | 6070 | CG | LYS | D | 150 | -16.601 | -61.761 | 131.779 | 1.00 | 33.89 | C |
| ATOM | 6071 | CD | LYS | D | 150 | -16.602 | -60.781 | 132.911 | 1.00 | 41.07 | C |
| ATOM | 6072 | CE | LYS | D | 150 | -15.607 | -59.667 | 132.710 | 1.00 | 43.52 | C |
| ATOM | 6073 | NZ | LYS | D | 150 | -15.402 | -58.953 | 134.001 | 1.00 | 45.35 | N1+ |
| ATOM | 6074 | N | VAL | D | 151 | -18.756 | -65.597 | 130.594 | 1.00 | 30.40 | N |
| ATOM | 6075 | CA | VAL | D | 151 | -19.524 | -66.777 | 130.977 | 1.00 | 36.11 | C |
| ATOM | 6076 | C | VAL | D | 151 | -18.820 | -67.400 | 132.176 | 1.00 | 36.89 | C |
| ATOM | 6077 | O | VAL | D | 151 | -17.662 | -67.816 | 132.061 | 1.00 | 34.20 | O |
| ATOM | 6078 | CB | VAL | D | 151 | -19.637 | -67.786 | 129.825 | 1.00 | 37.15 | C |
| ATOM | 6079 | CG1 | VAL | D | 151 | -20.568 | -68.901 | 130.211 | 1.00 | 34.22 | C |
| ATOM | 6080 | CG2 | VAL | D | 151 | -20.111 | -67.106 | 128.549 | 1.00 | 38.38 | C |
| ATOM | 6081 | N | ASP | D | 152 | -19.508 | -67.435 | 133.333 | 1.00 | 40.45 | N |
| ATOM | 6082 | CA | ASP | D | 152 | -18.928 | -67.863 | 134.626 | 1.00 | 35.46 | C |
| ATOM | 6083 | C | ASP | D | 152 | -17.564 | -67.218 | 134.854 | 1.00 | 37.61 | C |
| ATOM | 6084 | O | ASP | D | 152 | -16.602 | -67.868 | 135.265 | 1.00 | 40.92 | O |
| ATOM | 6085 | CB | ASP | D | 152 | -18.833 | -69.391 | 134.739 | 1.00 | 34.39 | C |
| ATOM | 6086 | CG | ASP | D | 152 | -20.200 | -70.051 | 134.992 | 1.00 | 43.58 | C |
| ATOM | 6087 | OD1 | ASP | D | 152 | -21.057 | -69.424 | 135.659 | 1.00 | 44.13 | O |
| ATOM | 6088 | OD2 | ASP | D | 152 | -20.411 | -71.206 | 134.549 | 1.00 | 44.21 | O1- |
| ATOM | 6089 | N | ASN | D | 153 | -17.493 | -65.920 | 134.560 | 1.00 | 36.04 | N |
| ATOM | 6090 | CA | ASN | D | 153 | -16.305 | -65.088 | 134.682 | 1.00 | 34.97 | C |
| ATOM | 6091 | C | ASN | D | 153 | -15.188 | -65.521 | 133.737 | 1.00 | 37.78 | C |
| ATOM | 6092 | O | ASN | D | 153 | -14.015 | -65.140 | 133.922 | 1.00 | 36.32 | O |
| ATOM | 6093 | CB | ASN | D | 153 | -15.802 | -65.009 | 136.126 | 1.00 | 33.76 | C |
| ATOM | 6094 | CG | ASN | D | 153 | -15.114 | -63.678 | 136.417 | 1.00 | 42.55 | C |
| ATOM | 6095 | OD1 | ASN | D | 153 | -15.704 | -62.598 | 136.248 | 1.00 | 42.84 | O |
| ATOM | 6096 | ND2 | ASN | D | 153 | -13.853 | -63.747 | 136.831 | 1.00 | 46.83 | N |
| ATOM | 6097 | N | ALA | D | 154 | -15.521 | -66.293 | 132.706 | 1.00 | 34.52 | N |
| ATOM | 6098 | CA | ALA | D | 154 | -14.577 | -66.555 | 131.629 | 1.00 | 35.50 | C |
| ATOM | 6099 | C | ALA | D | 154 | -14.784 | -65.526 | 130.521 | 1.00 | 38.64 | C |
| ATOM | 6100 | O | ALA | D | 154 | -15.880 | -65.417 | 129.954 | 1.00 | 35.76 | O |
| ATOM | 6101 | CB | ALA | D | 154 | -14.727 | -67.973 | 131.090 | 1.00 | 31.21 | C |

| | | | | | | | | | | | |
|---|---|---|---|---|---|---|---|---|---|---|---|
| ATOM | 6102 | N | LEU | D | 155 | -13.725 | -64.775 | 130.223 | 1.00 | 39.22 | N |
| ATOM | 6103 | CA | LEU | D | 155 | -13.728 | -63.839 | 129.108 | 1.00 | 34.49 | C |
| ATOM | 6104 | C | LEU | D | 155 | -13.970 | -64.578 | 127.815 | 1.00 | 31.95 | C |
| ATOM | 6105 | O | LEU | D | 155 | -13.276 | -65.550 | 127.518 | 1.00 | 33.49 | O |
| ATOM | 6106 | CB | LEU | D | 155 | -12.391 | -63.125 | 129.038 | 1.00 | 36.99 | C |
| ATOM | 6107 | CG | LEU | D | 155 | -12.350 | -61.800 | 129.779 | 1.00 | 48.06 | C |
| ATOM | 6108 | CD1 | LEU | D | 155 | -12.181 | -62.015 | 131.301 | 1.00 | 43.48 | C |
| ATOM | 6109 | CD2 | LEU | D | 155 | -11.219 | -60.978 | 129.190 | 1.00 | 51.82 | C |
| ATOM | 6110 | N | GLN | D | 156 | -14.996 | -64.166 | 127.080 | 1.00 | 36.40 | N |
| ATOM | 6111 | CA | GLN | D | 156 | -15.212 | -64.660 | 125.725 | 1.00 | 35.33 | C |
| ATOM | 6112 | C | GLN | D | 156 | -14.505 | -63.750 | 124.725 | 1.00 | 31.08 | C |
| ATOM | 6113 | O | GLN | D | 156 | -14.665 | -62.526 | 124.763 | 1.00 | 36.72 | O |
| ATOM | 6114 | CB | GLN | D | 156 | -16.703 | -64.738 | 125.398 | 1.00 | 32.10 | C |
| ATOM | 6115 | CG | GLN | D | 156 | -17.523 | -65.447 | 126.450 | 1.00 | 35.66 | C |
| ATOM | 6116 | CD | GLN | D | 156 | -17.059 | -66.878 | 126.701 | 1.00 | 36.63 | C |
| ATOM | 6117 | OE1 | GLN | D | 156 | -17.090 | -67.722 | 125.804 | 1.00 | 31.49 | O |
| ATOM | 6118 | NE2 | GLN | D | 156 | -16.638 | -67.153 | 127.929 | 1.00 | 35.80 | N |
| ATOM | 6119 | N | SER | D | 157 | -13.790 | -64.354 | 123.795 | 1.00 | 24.35 | N |
| ATOM | 6120 | CA | SER | D | 157 | -13.068 | -63.629 | 122.758 | 1.00 | 27.71 | C |
| ATOM | 6121 | C | SER | D | 157 | -13.201 | -64.434 | 121.474 | 1.00 | 29.57 | C |
| ATOM | 6122 | O | SER | D | 157 | -12.715 | -65.562 | 121.405 | 1.00 | 31.81 | O |
| ATOM | 6123 | CB | SER | D | 157 | -11.607 | -63.449 | 123.142 | 1.00 | 27.38 | C |
| ATOM | 6124 | OG | SER | D | 157 | -10.897 | -62.800 | 122.115 | 1.00 | 32.95 | O |
| ATOM | 6125 | N | GLY | D | 158 | -13.880 | -63.886 | 120.474 | 1.00 | 25.60 | N |
| ATOM | 6126 | CA | GLY | D | 158 | -13.995 | -64.518 | 119.171 | 1.00 | 23.02 | C |
| ATOM | 6127 | C | GLY | D | 158 | -15.329 | -65.156 | 118.862 | 1.00 | 31.71 | C |
| ATOM | 6128 | O | GLY | D | 158 | -15.544 | -65.567 | 117.710 | 1.00 | 30.83 | O |
| ATOM | 6129 | N | ASN | D | 159 | -16.253 | -65.223 | 119.824 | 1.00 | 28.19 | N |
| ATOM | 6130 | CA | ASN | D | 159 | -17.529 | -65.872 | 119.572 | 1.00 | 22.12 | C |
| ATOM | 6131 | C | ASN | D | 159 | -18.686 | -64.886 | 119.660 | 1.00 | 25.52 | C |
| ATOM | 6132 | O | ASN | D | 159 | -19.803 | -65.268 | 120.002 | 1.00 | 26.86 | O |
| ATOM | 6133 | CB | ASN | D | 159 | -17.749 | -67.054 | 120.516 | 1.00 | 25.99 | C |
| ATOM | 6134 | CG | ASN | D | 159 | -17.495 | -66.712 | 121.997 | 1.00 | 31.94 | C |
| ATOM | 6135 | OD1 | ASN | D | 159 | -17.103 | -65.589 | 122.345 | 1.00 | 28.21 | O |
| ATOM | 6136 | ND2 | ASN | D | 159 | -17.712 | -67.706 | 122.875 | 1.00 | 27.51 | N |
| ATOM | 6137 | N | SER | D | 160 | -18.445 | -63.625 | 119.295 | 1.00 | 27.33 | N |
| ATOM | 6138 | CA | SER | D | 160 | -19.484 | -62.606 | 119.297 | 1.00 | 26.68 | C |
| ATOM | 6139 | C | SER | D | 160 | -19.310 | -61.677 | 118.099 | 1.00 | 24.99 | C |
| ATOM | 6140 | O | SER | D | 160 | -18.224 | -61.571 | 117.525 | 1.00 | 29.23 | O |
| ATOM | 6141 | CB | SER | D | 160 | -19.475 | -61.812 | 120.614 | 1.00 | 27.08 | C |
| ATOM | 6142 | OG | SER | D | 160 | -18.327 | -61.009 | 120.707 | 1.00 | 26.36 | O |
| ATOM | 6143 | N | GLN | D | 161 | -20.407 | -61.024 | 117.708 | 1.00 | 24.70 | N |
| ATOM | 6144 | CA | GLN | D | 161 | -20.404 | -59.998 | 116.667 | 1.00 | 24.60 | C |
| ATOM | 6145 | C | GLN | D | 161 | -21.192 | -58.766 | 117.128 | 1.00 | 29.81 | C |
| ATOM | 6146 | O | GLN | D | 161 | -21.972 | -58.818 | 118.082 | 1.00 | 31.67 | O |
| ATOM | 6147 | CB | GLN | D | 161 | -20.954 | -60.560 | 115.380 | 1.00 | 22.23 | C |
| ATOM | 6148 | CG | GLN | D | 161 | -20.025 | -61.563 | 114.752 | 1.00 | 24.29 | C |
| ATOM | 6149 | CD | GLN | D | 161 | -20.631 | -62.200 | 113.532 | 1.00 | 27.64 | C |
| ATOM | 6150 | OE1 | GLN | D | 161 | -21.466 | -63.098 | 113.629 | 1.00 | 32.76 | O |
| ATOM | 6151 | NE2 | GLN | D | 161 | -20.246 | -61.717 | 112.372 | 1.00 | 23.06 | N |
| ATOM | 6152 | N | GLU | D | 162 | -20.965 | -57.641 | 116.458 | 1.00 | 30.47 | N |
| ATOM | 6153 | CA | GLU | D | 162 | -21.462 | -56.341 | 116.919 | 1.00 | 34.77 | C |
| ATOM | 6154 | C | GLU | D | 162 | -21.983 | -55.478 | 115.772 | 1.00 | 30.23 | C |
| ATOM | 6155 | O | GLU | D | 162 | -21.425 | -55.485 | 114.678 | 1.00 | 26.52 | O |
| ATOM | 6156 | CB | GLU | D | 162 | -20.360 | -55.528 | 117.568 | 1.00 | 32.41 | C |
| ATOM | 6157 | CG | GLU | D | 162 | -20.266 | -55.519 | 119.031 | 1.00 | 32.77 | C |
| ATOM | 6158 | CD | GLU | D | 162 | -19.053 | -54.695 | 119.437 | 1.00 | 41.06 | C |
| ATOM | 6159 | OE1 | GLU | D | 162 | -18.443 | -54.075 | 118.530 | 1.00 | 38.20 | O |
| ATOM | 6160 | OE2 | GLU | D | 162 | -18.704 | -54.667 | 120.637 | 1.00 | 50.77 | O1- |
| ATOM | 6161 | N | SER | D | 163 | -22.953 | -54.617 | 116.084 | 1.00 | 27.60 | N |
| ATOM | 6162 | CA | SER | D | 163 | -23.426 | -53.583 | 115.170 | 1.00 | 27.13 | C |
| ATOM | 6163 | C | SER | D | 163 | -23.656 | -52.284 | 115.923 | 1.00 | 25.97 | C |
| ATOM | 6164 | O | SER | D | 163 | -24.105 | -52.299 | 117.068 | 1.00 | 26.91 | O |
| ATOM | 6165 | CB | SER | D | 163 | -24.781 | -53.931 | 114.531 | 1.00 | 29.47 | C |
| ATOM | 6166 | OG | SER | D | 163 | -24.662 | -54.692 | 113.366 | 1.00 | 32.02 | O |
| ATOM | 6167 | N | VAL | D | 164 | -23.422 | -51.161 | 115.243 | 1.00 | 20.54 | N |
| ATOM | 6168 | CA | VAL | D | 164 | -23.727 | -49.843 | 115.781 | 1.00 | 23.87 | C |
| ATOM | 6169 | C | VAL | D | 164 | -24.632 | -49.109 | 114.794 | 1.00 | 25.79 | C |
| ATOM | 6170 | O | VAL | D | 164 | -24.411 | -49.146 | 113.580 | 1.00 | 28.96 | O |
| ATOM | 6171 | CB | VAL | D | 164 | -22.451 | -49.022 | 116.079 | 1.00 | 27.40 | C |
| ATOM | 6172 | CG1 | VAL | D | 164 | -22.804 | -47.685 | 116.731 | 1.00 | 26.22 | C |

| | | | | | | | | | | |
|---|---|---|---|---|---|---|---|---|---|---|
| ATOM | 6173 | CG2 | VAL | D | 164 | -21.543 | -49.782 | 116.997 | 1.00 25.86 | C |
| ATOM | 6174 | N | THR | D | 165 | -25.645 | -48.432 | 115.318 | 1.00 22.06 | N |
| ATOM | 6175 | CA | THR | D | 165 | -26.530 | -47.661 | 114.471 | 1.00 26.12 | C |
| ATOM | 6176 | C | THR | D | 165 | -25.845 | -46.376 | 113.991 | 1.00 27.66 | C |
| ATOM | 6177 | O | THR | D | 165 | -24.781 | -45.984 | 114.468 | 1.00 24.77 | O |
| ATOM | 6178 | CB | THR | D | 165 | -27.813 | -47.311 | 115.216 | 1.00 27.08 | C |
| ATOM | 6179 | OG1 | THR | D | 165 | -27.482 | -46.765 | 116.504 | 1.00 27.47 | O |
| ATOM | 6180 | CG2 | THR | D | 165 | -28.688 | -48.544 | 115.355 | 1.00 26.44 | C |
| ATOM | 6181 | N | GLU | D | 166 | -26.479 | -45.719 | 113.023 | 1.00 27.00 | N |
| ATOM | 6182 | CA | GLU | D | 166 | -26.079 | -44.370 | 112.686 | 1.00 27.71 | C |
| ATOM | 6183 | C | GLU | D | 166 | -26.495 | -43.432 | 113.817 | 1.00 33.03 | C |
| ATOM | 6184 | O | GLU | D | 166 | -27.359 | -43.761 | 114.640 | 1.00 33.28 | O |
| ATOM | 6185 | CB | GLU | D | 166 | -26.689 | -43.951 | 111.339 | 1.00 30.38 | C |
| ATOM | 6186 | CG | GLU | D | 166 | -26.119 | -44.711 | 110.120 | 1.00 25.40 | C |
| ATOM | 6187 | CD | GLU | D | 166 | -24.599 | -44.606 | 110.040 | 1.00 38.65 | C |
| ATOM | 6188 | OE1 | GLU | D | 166 | -24.038 | -43.552 | 110.423 | 1.00 46.73 | O |
| ATOM | 6189 | OE2 | GLU | D | 166 | -23.946 | -45.597 | 109.652 | 1.00 40.23 | O1- |
| ATOM | 6190 | N | GLN | D | 167 | -25.866 | -42.254 | 113.856 | 1.00 29.35 | N |
| ATOM | 6191 | CA | GLN | D | 167 | -26.181 | -41.280 | 114.885 | 1.00 27.68 | C |
| ATOM | 6192 | C | GLN | D | 167 | -27.675 | -40.977 | 114.888 | 1.00 30.18 | C |
| ATOM | 6193 | O | GLN | D | 167 | -28.273 | -40.743 | 113.841 | 1.00 28.88 | O |
| ATOM | 6194 | CB | GLN | D | 167 | -25.371 | -40.014 | 114.663 | 1.00 26.86 | C |
| ATOM | 6195 | CG | GLN | D | 167 | -25.050 | -39.318 | 115.952 | 1.00 30.75 | C |
| ATOM | 6196 | CD | GLN | D | 167 | -24.012 | -38.230 | 115.810 | 1.00 30.88 | C |
| ATOM | 6197 | OE1 | GLN | D | 167 | -23.630 | -37.851 | 114.707 | 1.00 26.28 | O |
| ATOM | 6198 | NE2 | GLN | D | 167 | -23.526 | -37.741 | 116.942 | 1.00 31.17 | N |
| ATOM | 6199 | N | ASP | D | 168 | -28.284 | -40.993 | 116.073 | 1.00 32.93 | N |
| ATOM | 6200 | CA | ASP | D | 168 | -29.740 | -40.995 | 116.145 | 1.00 30.89 | C |
| ATOM | 6201 | C | ASP | D | 168 | -30.322 | -39.647 | 115.730 | 1.00 32.78 | C |
| ATOM | 6202 | O | ASP | D | 168 | -29.864 | -38.588 | 116.175 | 1.00 32.90 | O |
| ATOM | 6203 | CB | ASP | D | 168 | -30.198 | -41.372 | 117.553 | 1.00 31.28 | C |
| ATOM | 6204 | CG | ASP | D | 168 | -31.716 | -41.521 | 117.659 | 1.00 38.67 | C |
| ATOM | 6205 | OD2 | ASP | D | 168 | -32.352 | -40.708 | 118.357 | 1.00 38.36 | O |
| ATOM | 6206 | OD1 | ASP | D | 168 | -32.272 | -42.488 | 117.088 | 1.00 46.18 | O1- |
| ATOM | 6207 | N | SER | D | 169 | -31.354 | -39.697 | 114.883 | 1.00 39.01 | N |
| ATOM | 6208 | CA | SER | D | 169 | -31.955 | -38.482 | 114.346 | 1.00 32.75 | C |
| ATOM | 6209 | C | SER | D | 169 | -32.582 | -37.588 | 115.413 | 1.00 37.05 | C |
| ATOM | 6210 | O | SER | D | 169 | -32.812 | -36.404 | 115.145 | 1.00 38.37 | O |
| ATOM | 6211 | CB | SER | D | 169 | -33.001 | -38.845 | 113.284 | 1.00 39.20 | C |
| ATOM | 6212 | OG | SER | D | 169 | -34.079 | -39.588 | 113.824 | 1.00 50.23 | O |
| ATOM | 6213 | N | LYS | D | 170 | -32.814 | -38.077 | 116.629 | 1.00 34.51 | N |
| ATOM | 6214 | CA | LYS | D | 170 | -33.421 | -37.203 | 117.625 | 1.00 34.07 | C |
| ATOM | 6215 | C | LYS | D | 170 | -32.468 | -36.701 | 118.701 | 1.00 33.50 | C |
| ATOM | 6216 | O | LYS | D | 170 | -32.589 | -35.552 | 119.108 | 1.00 37.46 | O |
| ATOM | 6217 | CB | LYS | D | 170 | -34.629 | -37.888 | 118.275 | 1.00 37.63 | C |
| ATOM | 6218 | CG | LYS | D | 170 | -35.389 | -37.006 | 119.243 | 1.00 37.03 | C |
| ATOM | 6219 | CD | LYS | D | 170 | -36.331 | -37.786 | 120.178 | 1.00 38.46 | C |
| ATOM | 6220 | CE | LYS | D | 170 | -37.575 | -38.283 | 119.424 | 1.00 45.87 | C |
| ATOM | 6221 | NZ | LYS | D | 170 | -38.639 | -38.916 | 120.302 | 1.00 39.82 | N1+ |
| ATOM | 6222 | N | ASP | D | 171 | -31.531 | -37.506 | 119.210 | 1.00 36.72 | N |
| ATOM | 6223 | CA | ASP | D | 171 | -30.652 | -37.034 | 120.285 | 1.00 30.44 | C |
| ATOM | 6224 | C | ASP | D | 171 | -29.163 | -37.175 | 119.956 | 1.00 31.83 | C |
| ATOM | 6225 | O | ASP | D | 171 | -28.327 | -37.027 | 120.864 | 1.00 24.40 | O |
| ATOM | 6226 | CB | ASP | D | 171 | -30.977 | -37.745 | 121.621 | 1.00 24.58 | C |
| ATOM | 6227 | CG | ASP | D | 171 | -30.724 | -39.290 | 121.598 | 1.00 37.38 | C |
| ATOM | 6228 | OD1 | ASP | D | 171 | -29.900 | -39.800 | 120.803 | 1.00 38.28 | O |
| ATOM | 6229 | OD2 | ASP | D | 171 | -31.345 | -40.026 | 122.402 | 1.00 44.17 | O1- |
| ATOM | 6230 | N | SER | D | 172 | -28.814 | -37.467 | 118.691 | 1.00 27.49 | N |
| ATOM | 6231 | CA | SER | D | 172 | -27.433 | -37.542 | 118.210 | 1.00 29.50 | C |
| ATOM | 6232 | C | SER | D | 172 | -26.575 | -38.575 | 118.954 | 1.00 33.88 | C |
| ATOM | 6233 | O | SER | D | 172 | -25.339 | -38.428 | 119.009 | 1.00 32.88 | O |
| ATOM | 6234 | CB | SER | D | 172 | -26.756 | -36.167 | 118.283 | 1.00 28.79 | C |
| ATOM | 6235 | OG | SER | D | 172 | -27.503 | -35.209 | 117.557 | 1.00 32.16 | O |
| ATOM | 6236 | N | THR | D | 173 | -27.185 | -39.602 | 119.550 | 1.00 25.27 | N |
| ATOM | 6237 | CA | THR | D | 173 | -26.443 | -40.663 | 120.214 | 1.00 26.81 | C |
| ATOM | 6238 | C | THR | D | 173 | -26.265 | -41.868 | 119.287 | 1.00 28.18 | C |
| ATOM | 6239 | O | THR | D | 173 | -26.791 | -41.932 | 118.163 | 1.00 26.72 | O |
| ATOM | 6240 | CB | THR | D | 173 | -27.127 | -41.101 | 121.529 | 1.00 29.20 | C |
| ATOM | 6241 | OG1 | THR | D | 173 | -28.454 | -41.578 | 121.279 | 1.00 26.42 | O |
| ATOM | 6242 | CG2 | THR | D | 173 | -27.181 | -39.948 | 122.547 | 1.00 27.06 | C |
| ATOM | 6243 | N | TYR | D | 174 | -25.492 | -42.824 | 119.787 | 1.00 24.11 | N |

| | | | | | | | | | | | |
|---|---|---|---|---|---|---|---|---|---|---|---|
| ATOM | 6244 | CA | TYR | D | 174 | -25.252 | -44.113 | 119.162 | 1.00 | 25.18 | C |
| ATOM | 6245 | C | TYR | D | 174 | -25.858 | -45.225 | 120.001 | 1.00 | 27.09 | C |
| ATOM | 6246 | O | TYR | D | 174 | -25.925 | -45.130 | 121.232 | 1.00 | 26.92 | O |
| ATOM | 6247 | CB | TYR | D | 174 | -23.740 | -44.371 | 118.993 | 1.00 | 24.71 | C |
| ATOM | 6248 | CG | TYR | D | 174 | -23.145 | -43.461 | 117.980 | 1.00 | 27.06 | C |
| ATOM | 6249 | CD1 | TYR | D | 174 | -23.212 | -43.778 | 116.625 | 1.00 | 23.86 | C |
| ATOM | 6250 | CD2 | TYR | D | 174 | -22.587 | -42.234 | 118.355 | 1.00 | 29.38 | C |
| ATOM | 6251 | CE1 | TYR | D | 174 | -22.697 | -42.931 | 115.671 | 1.00 | 28.55 | C |
| ATOM | 6252 | CE2 | TYR | D | 174 | -22.060 | -41.366 | 117.397 | 1.00 | 28.07 | C |
| ATOM | 6253 | CZ | TYR | D | 174 | -22.125 | -41.719 | 116.053 | 1.00 | 30.70 | C |
| ATOM | 6254 | OH | TYR | D | 174 | -21.626 | -40.882 | 115.081 | 1.00 | 29.66 | O |
| ATOM | 6255 | N | SER | D | 175 | -26.220 | -46.318 | 119.329 | 1.00 | 29.12 | N |
| ATOM | 6256 | CA | SER | D | 175 | -26.623 | -47.546 | 120.005 | 1.00 | 29.70 | C |
| ATOM | 6257 | C | SER | D | 175 | -25.851 | -48.723 | 119.437 | 1.00 | 27.77 | C |
| ATOM | 6258 | O | SER | D | 175 | -25.515 | -48.748 | 118.252 | 1.00 | 26.37 | O |
| ATOM | 6259 | CB | SER | D | 175 | -28.127 | -47.796 | 119.868 | 1.00 | 28.32 | C |
| ATOM | 6260 | OG | SER | D | 175 | -28.822 | -46.950 | 120.755 | 1.00 | 29.89 | O |
| ATOM | 6261 | N | LEU | D | 176 | -25.615 | -49.725 | 120.284 | 1.00 | 29.44 | N |
| ATOM | 6262 | CA | LEU | D | 176 | -24.808 | -50.884 | 119.918 | 1.00 | 26.61 | C |
| ATOM | 6263 | C | LEU | D | 176 | -25.499 | -52.174 | 120.346 | 1.00 | 25.01 | C |
| ATOM | 6264 | O | LEU | D | 176 | -26.055 | -52.255 | 121.444 | 1.00 | 25.06 | O |
| ATOM | 6265 | CB | LEU | D | 176 | -23.412 | -50.791 | 120.557 | 1.00 | 23.61 | C |
| ATOM | 6266 | CG | LEU | D | 176 | -22.361 | -51.831 | 120.176 | 1.00 | 26.86 | C |
| ATOM | 6267 | CD1 | LEU | D | 176 | -20.995 | -51.225 | 120.278 | 1.00 | 28.36 | C |
| ATOM | 6268 | CD2 | LEU | D | 176 | -22.428 | -53.052 | 121.099 | 1.00 | 25.35 | C |
| ATOM | 6269 | N | SER | D | 177 | -25.405 | -53.202 | 119.500 | 1.00 | 26.18 | N |
| ATOM | 6270 | CA | SER | D | 177 | -25.932 | -54.536 | 119.789 | 1.00 | 25.92 | C |
| ATOM | 6271 | C | SER | D | 177 | -24.840 | -55.587 | 119.589 | 1.00 | 25.55 | C |
| ATOM | 6272 | O | SER | D | 177 | -24.136 | -55.566 | 118.577 | 1.00 | 30.42 | O |
| ATOM | 6273 | CB | SER | D | 177 | -27.142 | -54.840 | 118.895 | 1.00 | 30.52 | C |
| ATOM | 6274 | OG | SER | D | 177 | -27.570 | -56.176 | 119.035 | 1.00 | 31.20 | O |
| ATOM | 6275 | N | SER | D | 178 | -24.668 | -56.474 | 120.574 | 1.00 | 28.18 | N |
| ATOM | 6276 | CA | SER | D | 178 | -23.659 | -57.527 | 120.542 | 1.00 | 25.27 | C |
| ATOM | 6277 | C | SER | D | 178 | -24.288 | -58.885 | 120.794 | 1.00 | 26.75 | C |
| ATOM | 6278 | O | SER | D | 178 | -25.102 | -59.040 | 121.705 | 1.00 | 31.18 | O |
| ATOM | 6279 | CB | SER | D | 178 | -22.560 | -57.312 | 121.568 | 1.00 | 30.71 | C |
| ATOM | 6280 | OG | SER | D | 178 | -21.592 | -58.348 | 121.447 | 1.00 | 32.24 | O |
| ATOM | 6281 | N | THR | D | 179 | -23.888 | -59.880 | 120.010 | 1.00 | 27.93 | N |
| ATOM | 6282 | CA | THR | D | 179 | -24.458 | -61.220 | 120.122 | 1.00 | 29.75 | C |
| ATOM | 6283 | C | THR | D | 179 | -23.379 | -62.248 | 120.444 | 1.00 | 31.38 | C |
| ATOM | 6284 | O | THR | D | 179 | -22.529 | -62.546 | 119.604 | 1.00 | 25.41 | O |
| ATOM | 6285 | CB | THR | D | 179 | -25.160 | -61.608 | 118.837 | 1.00 | 32.19 | C |
| ATOM | 6286 | OG1 | THR | D | 179 | -26.110 | -60.586 | 118.488 | 1.00 | 42.39 | O |
| ATOM | 6287 | CG2 | THR | D | 179 | -25.841 | -62.933 | 119.026 | 1.00 | 33.94 | C |
| ATOM | 6288 | N | LEU | D | 180 | -23.448 | -62.822 | 121.641 | 1.00 | 33.64 | N |
| ATOM | 6289 | CA | LEU | D | 180 | -22.651 | -63.988 | 121.989 | 1.00 | 26.84 | C |
| ATOM | 6290 | C | LEU | D | 180 | -23.377 | -65.233 | 121.509 | 1.00 | 28.96 | C |
| ATOM | 6291 | O | LEU | D | 180 | -24.576 | -65.383 | 121.759 | 1.00 | 33.24 | O |
| ATOM | 6292 | CB | LEU | D | 180 | -22.446 | -64.042 | 123.496 | 1.00 | 26.91 | C |
| ATOM | 6293 | CG | LEU | D | 180 | -21.584 | -65.169 | 124.035 | 1.00 | 32.83 | C |
| ATOM | 6294 | CD1 | LEU | D | 180 | -20.179 | -64.954 | 123.541 | 1.00 | 32.39 | C |
| ATOM | 6295 | CD2 | LEU | D | 180 | -21.624 | -65.196 | 125.566 | 1.00 | 35.83 | C |
| ATOM | 6296 | N | THR | D | 181 | -22.683 | -66.101 | 120.784 | 1.00 | 24.60 | N |
| ATOM | 6297 | CA | THR | D | 181 | -23.289 | -67.321 | 120.267 | 1.00 | 29.40 | C |
| ATOM | 6298 | C | THR | D | 181 | -22.656 | -68.546 | 120.928 | 1.00 | 35.38 | C |
| ATOM | 6299 | O | THR | D | 181 | -21.430 | -68.622 | 121.063 | 1.00 | 32.82 | O |
| ATOM | 6300 | CB | THR | D | 181 | -23.162 | -67.393 | 118.744 | 1.00 | 29.01 | C |
| ATOM | 6301 | OG1 | THR | D | 181 | -23.762 | -66.235 | 118.169 | 1.00 | 36.58 | O |
| ATOM | 6302 | CG2 | THR | D | 181 | -23.943 | -68.571 | 118.214 | 1.00 | 35.08 | C |
| ATOM | 6303 | N | LEU | D | 182 | -23.499 | -69.481 | 121.375 | 1.00 | 33.54 | N |
| ATOM | 6304 | CA | LEU | D | 182 | -23.061 | -70.743 | 121.957 | 1.00 | 31.73 | C |
| ATOM | 6305 | C | LEU | D | 182 | -23.908 | -71.860 | 121.379 | 1.00 | 38.06 | C |
| ATOM | 6306 | O | LEU | D | 182 | -25.010 | -71.638 | 120.866 | 1.00 | 39.76 | O |
| ATOM | 6307 | CB | LEU | D | 182 | -23.242 | -70.791 | 123.473 | 1.00 | 34.20 | C |
| ATOM | 6308 | CG | LEU | D | 182 | -22.667 | -69.731 | 124.395 | 1.00 | 37.93 | C |
| ATOM | 6309 | CD1 | LEU | D | 182 | -23.089 | -70.021 | 125.827 | 1.00 | 39.29 | C |
| ATOM | 6310 | CD2 | LEU | D | 182 | -21.154 | -69.692 | 124.276 | 1.00 | 41.23 | C |
| ATOM | 6311 | N | SER | D | 183 | -23.401 | -73.075 | 121.491 | 1.00 | 41.78 | N |
| ATOM | 6312 | CA | SER | D | 183 | -24.276 | -74.218 | 121.302 | 1.00 | 41.97 | C |
| ATOM | 6313 | C | SER | D | 183 | -25.171 | -74.354 | 122.533 | 1.00 | 40.93 | C |
| ATOM | 6314 | O | SER | D | 183 | -24.832 | -73.874 | 123.619 | 1.00 | 39.70 | O |

| | | | | | | | | | | | |
|---|---|---|---|---|---|---|---|---|---|---|---|
| ATOM | 6315 | CB | SER | D | 183 | -23.449 | -75.481 | 121.071 | 1.00 | 42.62 | C |
| ATOM | 6316 | OG | SER | D | 183 | -22.677 | -75.783 | 122.224 | 1.00 | 45.39 | O |
| ATOM | 6317 | N | LYS | D | 184 | -26.323 | -75.017 | 122.368 | 1.00 | 38.98 | N |
| ATOM | 6318 | CA | LYS | D | 184 | -27.172 | -75.267 | 123.536 | 1.00 | 46.77 | C |
| ATOM | 6319 | C | LYS | D | 184 | -26.428 | -76.067 | 124.602 | 1.00 | 45.69 | C |
| ATOM | 6320 | O | LYS | D | 184 | -26.586 | -75.820 | 125.806 | 1.00 | 44.13 | O |
| ATOM | 6321 | CB | LYS | D | 184 | -28.465 | -75.982 | 123.135 | 1.00 | 52.78 | C |
| ATOM | 6322 | CG | LYS | D | 184 | -29.383 | -76.279 | 124.334 | 1.00 | 45.82 | C |
| ATOM | 6323 | CD | LYS | D | 184 | -30.721 | -76.916 | 123.925 | 1.00 | 49.11 | C |
| ATOM | 6324 | CE | LYS | D | 184 | -30.567 | -78.342 | 123.414 | 1.00 | 56.53 | C |
| ATOM | 6325 | NZ | LYS | D | 184 | -31.811 | -78.834 | 122.761 | 1.00 | 58.33 | N1+ |
| ATOM | 6326 | N | ALA | D | 185 | -25.599 | -77.019 | 124.172 | 1.00 | 44.49 | N |
| ATOM | 6327 | CA | ALA | D | 185 | -24.835 | -77.822 | 125.116 | 1.00 | 44.16 | C |
| ATOM | 6328 | C | ALA | D | 185 | -23.903 | -76.953 | 125.958 | 1.00 | 46.66 | C |
| ATOM | 6329 | O | ALA | D | 185 | -23.932 | -77.018 | 127.193 | 1.00 | 48.01 | O |
| ATOM | 6330 | CB | ALA | D | 185 | -24.067 | -78.902 | 124.363 | 1.00 | 43.48 | C |
| ATOM | 6331 | N | ASP | D | 186 | -23.057 | -76.142 | 125.307 | 1.00 | 47.56 | N |
| ATOM | 6332 | CA | ASP | D | 186 | -22.203 | -75.208 | 126.042 | 1.00 | 43.67 | C |
| ATOM | 6333 | C | ASP | D | 186 | -23.040 | -74.263 | 126.887 | 1.00 | 41.82 | C |
| ATOM | 6334 | O | ASP | D | 186 | -22.744 | -74.029 | 128.065 | 1.00 | 43.44 | O |
| ATOM | 6335 | CB | ASP | D | 186 | -21.334 | -74.400 | 125.079 | 1.00 | 47.25 | C |
| ATOM | 6336 | CG | ASP | D | 186 | -20.218 | -75.210 | 124.472 | 1.00 | 49.80 | C |
| ATOM | 6337 | OD1 | ASP | D | 186 | -19.524 | -75.925 | 125.218 | 1.00 | 51.32 | O |
| ATOM | 6338 | OD2 | ASP | D | 186 | -20.038 | -75.131 | 123.241 | 1.00 | 59.81 | O1- |
| ATOM | 6339 | N | TYR | D | 187 | -24.100 | -73.713 | 126.296 | 1.00 | 42.10 | N |
| ATOM | 6340 | CA | TYR | D | 187 | -24.958 | -72.799 | 127.030 | 1.00 | 42.00 | C |
| ATOM | 6341 | C | TYR | D | 187 | -25.446 | -73.439 | 128.322 | 1.00 | 40.74 | C |
| ATOM | 6342 | O | TYR | D | 187 | -25.462 | -72.797 | 129.381 | 1.00 | 36.12 | O |
| ATOM | 6343 | CB | TYR | D | 187 | -26.148 | -72.356 | 126.154 | 1.00 | 35.24 | C |
| ATOM | 6344 | CG | TYR | D | 187 | -27.113 | -71.482 | 126.924 | 1.00 | 36.73 | C |
| ATOM | 6345 | CD1 | TYR | D | 187 | -26.712 | -70.236 | 127.403 | 1.00 | 33.83 | C |
| ATOM | 6346 | CD2 | TYR | D | 187 | -28.407 | -71.905 | 127.202 | 1.00 | 37.83 | C |
| ATOM | 6347 | CE1 | TYR | D | 187 | -27.570 | -69.443 | 128.137 | 1.00 | 35.37 | C |
| ATOM | 6348 | CE2 | TYR | D | 187 | -29.278 | -71.112 | 127.932 | 1.00 | 36.87 | C |
| ATOM | 6349 | CZ | TYR | D | 187 | -28.848 | -69.883 | 128.404 | 1.00 | 34.37 | C |
| ATOM | 6350 | OH | TYR | D | 187 | -29.704 | -69.082 | 129.125 | 1.00 | 33.53 | O |
| ATOM | 6351 | N | GLU | D | 188 | -25.818 | -74.715 | 128.266 | 1.00 | 44.28 | N |
| ATOM | 6352 | CA | GLU | D | 188 | -26.463 | -75.311 | 129.426 | 1.00 | 48.57 | C |
| ATOM | 6353 | C | GLU | D | 188 | -25.494 | -75.827 | 130.473 | 1.00 | 44.50 | C |
| ATOM | 6354 | O | GLU | D | 188 | -25.954 | -76.294 | 131.513 | 1.00 | 43.86 | O |
| ATOM | 6355 | CB | GLU | D | 188 | -27.384 | -76.431 | 128.984 | 1.00 | 46.87 | C |
| ATOM | 6356 | CG | GLU | D | 188 | -28.803 | -75.966 | 128.881 | 1.00 | 52.89 | C |
| ATOM | 6357 | CD | GLU | D | 188 | -29.586 | -76.862 | 127.993 | 1.00 | 60.48 | C |
| ATOM | 6358 | OE1 | GLU | D | 188 | -28.963 | -77.811 | 127.472 | 1.00 | 63.90 | O |
| ATOM | 6359 | OE2 | GLU | D | 188 | -30.800 | -76.623 | 127.817 | 1.00 | 70.92 | O1- |
| ATOM | 6360 | N | LYS | D | 189 | -24.184 | -75.709 | 130.249 | 1.00 | 42.21 | N |
| ATOM | 6361 | CA | LYS | D | 189 | -23.166 | -76.082 | 131.214 | 1.00 | 34.10 | C |
| ATOM | 6362 | C | LYS | D | 189 | -22.659 | -74.905 | 132.027 | 1.00 | 41.19 | C |
| ATOM | 6363 | O | LYS | D | 189 | -21.617 | -75.025 | 132.675 | 1.00 | 43.76 | O |
| ATOM | 6364 | CB | LYS | D | 189 | -21.981 | -76.727 | 130.501 | 1.00 | 42.21 | C |
| ATOM | 6365 | CG | LYS | D | 189 | -22.277 | -78.025 | 129.749 | 1.00 | 49.04 | C |
| ATOM | 6366 | CD | LYS | D | 189 | -21.065 | -78.418 | 128.889 | 1.00 | 50.62 | C |
| ATOM | 6367 | CE | LYS | D | 189 | -21.332 | -79.669 | 128.071 | 1.00 | 61.29 | C |
| ATOM | 6368 | NZ | LYS | D | 189 | -20.165 | -79.960 | 127.197 | 1.00 | 67.56 | N1+ |
| ATOM | 6369 | N | HIS | D | 190 | -23.349 | -73.768 | 132.011 | 1.00 | 40.38 | N |
| ATOM | 6370 | CA | HIS | D | 190 | -22.850 | -72.595 | 132.712 | 1.00 | 37.11 | C |
| ATOM | 6371 | C | HIS | D | 190 | -24.018 | -71.833 | 133.309 | 1.00 | 37.04 | C |
| ATOM | 6372 | O | HIS | D | 190 | -25.167 | -72.014 | 132.913 | 1.00 | 39.95 | O |
| ATOM | 6373 | CB | HIS | D | 190 | -22.044 | -71.702 | 131.782 | 1.00 | 41.03 | C |
| ATOM | 6374 | CG | HIS | D | 190 | -20.840 | -72.376 | 131.212 | 1.00 | 41.24 | C |
| ATOM | 6375 | ND1 | HIS | D | 190 | -19.759 | -72.747 | 131.983 | 1.00 | 41.48 | N |
| ATOM | 6376 | CD2 | HIS | D | 190 | -20.560 | -72.778 | 129.951 | 1.00 | 39.46 | C |
| ATOM | 6377 | CE1 | HIS | D | 190 | -18.856 | -73.333 | 131.218 | 1.00 | 36.30 | C |
| ATOM | 6378 | NE2 | HIS | D | 190 | -19.320 | -73.368 | 129.981 | 1.00 | 39.82 | N |
| ATOM | 6379 | N | LYS | D | 191 | -23.719 | -70.969 | 134.270 | 1.00 | 34.63 | N |
| ATOM | 6380 | CA | LYS | D | 191 | -24.775 | -70.340 | 135.046 | 1.00 | 45.76 | C |
| ATOM | 6381 | C | LYS | D | 191 | -24.874 | -68.842 | 134.790 | 1.00 | 46.36 | C |
| ATOM | 6382 | O | LYS | D | 191 | -25.944 | -68.357 | 134.407 | 1.00 | 46.07 | O |
| ATOM | 6383 | CB | LYS | D | 191 | -24.566 | -70.607 | 136.548 | 1.00 | 42.22 | C |
| ATOM | 6384 | CG | LYS | D | 191 | -25.568 | -69.875 | 137.451 | 1.00 | 49.74 | C |
| ATOM | 6385 | CD | LYS | D | 191 | -25.227 | -70.029 | 138.954 | 1.00 | 59.90 | C |

| | | | | | | | | | | | |
|---|---|---|---|---|---|---|---|---|---|---|---|
| ATOM | 6386 | CE | LYS | D | 191 | -26.350 | -69.510 | 139.853 | 1.00 | 60.67 | C |
| ATOM | 6387 | NZ | LYS | D | 191 | -26.031 | -69.671 | 141.293 | 1.00 | 66.07 | N1+ |
| ATOM | 6388 | N | VAL | D | 192 | -23.782 | -68.100 | 134.968 | 1.00 | 39.06 | N |
| ATOM | 6389 | CA | VAL | D | 192 | -23.809 | -66.644 | 134.941 | 1.00 | 38.75 | C |
| ATOM | 6390 | C | VAL | D | 192 | -23.378 | -66.173 | 133.553 | 1.00 | 39.96 | C |
| ATOM | 6391 | O | VAL | D | 192 | -22.277 | -66.490 | 133.091 | 1.00 | 41.97 | O |
| ATOM | 6392 | CB | VAL | D | 192 | -22.906 | -66.063 | 136.039 | 1.00 | 36.31 | C |
| ATOM | 6393 | CG1 | VAL | D | 192 | -22.876 | -64.544 | 135.989 | 1.00 | 34.38 | C |
| ATOM | 6394 | CG2 | VAL | D | 192 | -23.360 | -66.548 | 137.380 | 1.00 | 41.02 | C |
| ATOM | 6395 | N | TYR | D | 193 | -24.241 | -65.414 | 132.886 | 1.00 | 38.30 | N |
| ATOM | 6396 | CA | TYR | D | 193 | -23.924 | -64.789 | 131.608 | 1.00 | 35.90 | C |
| ATOM | 6397 | C | TYR | D | 193 | -23.862 | -63.290 | 131.845 | 1.00 | 33.90 | C |
| ATOM | 6398 | O | TYR | D | 193 | -24.747 | -62.734 | 132.487 | 1.00 | 36.52 | O |
| ATOM | 6399 | CB | TYR | D | 193 | -24.957 | -65.165 | 130.534 | 1.00 | 31.33 | C |
| ATOM | 6400 | CG | TYR | D | 193 | -24.828 | -66.629 | 130.183 | 1.00 | 35.91 | C |
| ATOM | 6401 | CD1 | TYR | D | 193 | -25.458 | -67.608 | 130.950 | 1.00 | 38.83 | C |
| ATOM | 6402 | CD2 | TYR | D | 193 | -23.971 | -67.049 | 129.171 | 1.00 | 38.88 | C |
| ATOM | 6403 | CE1 | TYR | D | 193 | -25.297 | -68.968 | 130.671 | 1.00 | 36.63 | C |
| ATOM | 6404 | CE2 | TYR | D | 193 | -23.799 | -68.410 | 128.885 | 1.00 | 39.70 | C |
| ATOM | 6405 | CZ | TYR | D | 193 | -24.461 | -69.359 | 129.646 | 1.00 | 36.21 | C |
| ATOM | 6406 | OH | TYR | D | 193 | -24.302 | -70.691 | 129.360 | 1.00 | 37.29 | O |
| ATOM | 6407 | N | ALA | D | 194 | -22.791 | -62.646 | 131.400 | 1.00 | 33.30 | N |
| ATOM | 6408 | CA | ALA | D | 194 | -22.654 | -61.226 | 131.670 | 1.00 | 34.22 | C |
| ATOM | 6409 | C | ALA | D | 194 | -22.000 | -60.510 | 130.488 | 1.00 | 36.04 | C |
| ATOM | 6410 | O | ALA | D | 194 | -21.164 | -61.083 | 129.782 | 1.00 | 35.02 | O |
| ATOM | 6411 | CB | ALA | D | 194 | -21.857 | -61.026 | 132.952 | 1.00 | 29.25 | C |
| ATOM | 6412 | N | CYS | D | 195 | -22.396 | -59.254 | 130.263 | 1.00 | 32.88 | N |
| ATOM | 6413 | CA | CYS | D | 195 | -21.638 | -58.361 | 129.389 | 1.00 | 39.71 | C |
| ATOM | 6414 | C | CYS | D | 195 | -21.246 | -57.121 | 130.170 | 1.00 | 37.39 | C |
| ATOM | 6415 | O | CYS | D | 195 | -22.084 | -56.498 | 130.839 | 1.00 | 33.62 | O |
| ATOM | 6416 | CB | CYS | D | 195 | -22.382 | -57.939 | 128.108 | 1.00 | 39.73 | C |
| ATOM | 6417 | SG | CYS | D | 195 | -23.983 | -57.183 | 128.349 | 1.00 | 55.69 | S |
| ATOM | 6418 | N | GLU | D | 196 | -19.981 | -56.753 | 130.035 | 1.00 | 33.07 | N |
| ATOM | 6419 | CA | GLU | D | 196 | -19.377 | -55.641 | 130.742 | 1.00 | 38.70 | C |
| ATOM | 6420 | C | GLU | D | 196 | -19.127 | -54.523 | 129.734 | 1.00 | 35.02 | C |
| ATOM | 6421 | O | GLU | D | 196 | -18.610 | -54.777 | 128.639 | 1.00 | 28.28 | O |
| ATOM | 6422 | CB | GLU | D | 196 | -18.078 | -56.104 | 131.410 | 1.00 | 35.85 | C |
| ATOM | 6423 | CG | GLU | D | 196 | -17.366 | -55.070 | 132.220 | 1.00 | 39.77 | C |
| ATOM | 6424 | CD | GLU | D | 196 | -15.962 | -55.516 | 132.610 | 1.00 | 46.03 | C |
| ATOM | 6425 | OE1 | GLU | D | 196 | -15.435 | -56.465 | 131.988 | 1.00 | 40.61 | O |
| ATOM | 6426 | OE2 | GLU | D | 196 | -15.373 | -54.880 | 133.511 | 1.00 | 57.77 | O1- |
| ATOM | 6427 | N | VAL | D | 197 | -19.512 | -53.297 | 130.097 | 1.00 | 34.66 | N |
| ATOM | 6428 | CA | VAL | D | 197 | -19.563 | -52.169 | 129.169 | 1.00 | 35.82 | C |
| ATOM | 6429 | C | VAL | D | 197 | -18.595 | -51.112 | 129.680 | 1.00 | 34.18 | C |
| ATOM | 6430 | O | VAL | D | 197 | -18.751 | -50.610 | 130.797 | 1.00 | 35.27 | O |
| ATOM | 6431 | CB | VAL | D | 197 | -20.993 | -51.601 | 129.024 | 1.00 | 31.64 | C |
| ATOM | 6432 | CG1 | VAL | D | 197 | -21.014 | -50.336 | 128.159 | 1.00 | 28.99 | C |
| ATOM | 6433 | CG2 | VAL | D | 197 | -21.944 | -52.640 | 128.462 | 1.00 | 28.78 | C |
| ATOM | 6434 | N | THR | D | 198 | -17.577 | -50.805 | 128.883 | 1.00 | 33.99 | N |
| ATOM | 6435 | CA | THR | D | 198 | -16.654 | -49.718 | 129.164 | 1.00 | 32.70 | C |
| ATOM | 6436 | C | THR | D | 198 | -16.983 | -48.563 | 128.229 | 1.00 | 40.52 | C |
| ATOM | 6437 | O | THR | D | 198 | -17.023 | -48.743 | 127.005 | 1.00 | 37.25 | O |
| ATOM | 6438 | CB | THR | D | 198 | -15.203 | -50.146 | 128.950 | 1.00 | 33.81 | C |
| ATOM | 6439 | OG1 | THR | D | 198 | -14.907 | -51.282 | 129.766 | 1.00 | 43.84 | O |
| ATOM | 6440 | CG2 | THR | D | 198 | -14.282 | -49.029 | 129.347 | 1.00 | 29.60 | C |
| ATOM | 6441 | N | HIS | D | 199 | -17.211 | -47.384 | 128.805 | 1.00 | 37.58 | N |
| ATOM | 6442 | CA | HIS | D | 199 | -17.528 | -46.198 | 128.030 | 1.00 | 31.22 | C |
| ATOM | 6443 | C | HIS | D | 199 | -17.075 | -44.973 | 128.805 | 1.00 | 34.53 | C |
| ATOM | 6444 | O | HIS | D | 199 | -17.002 | -44.999 | 130.033 | 1.00 | 35.60 | O |
| ATOM | 6445 | CB | HIS | D | 199 | -19.020 | -46.115 | 127.723 | 1.00 | 33.40 | C |
| ATOM | 6446 | CG | HIS | D | 199 | -19.383 | -44.969 | 126.828 | 1.00 | 34.76 | C |
| ATOM | 6447 | ND1 | HIS | D | 199 | -19.824 | -43.756 | 127.313 | 1.00 | 30.33 | N |
| ATOM | 6448 | CD2 | HIS | D | 199 | -19.360 | -44.849 | 125.479 | 1.00 | 32.19 | C |
| ATOM | 6449 | CE1 | HIS | D | 199 | -20.061 | -42.940 | 126.301 | 1.00 | 31.06 | C |
| ATOM | 6450 | NE2 | HIS | D | 199 | -19.778 | -43.575 | 125.178 | 1.00 | 31.67 | N |
| ATOM | 6451 | N | GLN | D | 200 | -16.786 | -43.888 | 128.085 | 1.00 | 29.72 | N |
| ATOM | 6452 | CA | GLN | D | 200 | -16.199 | -42.738 | 128.754 | 1.00 | 31.37 | C |
| ATOM | 6453 | C | GLN | D | 200 | -17.177 | -42.022 | 129.684 | 1.00 | 35.36 | C |
| ATOM | 6454 | O | GLN | D | 200 | -16.735 | -41.248 | 130.541 | 1.00 | 42.18 | O |
| ATOM | 6455 | CB | GLN | D | 200 | -15.629 | -41.779 | 127.723 | 1.00 | 33.74 | C |
| ATOM | 6456 | CG | GLN | D | 200 | -16.370 | -40.476 | 127.548 | 1.00 | 35.08 | C |

| | | | | | | | | | | | |
|---|---|---|---|---|---|---|---|---|---|---|---|
| ATOM | 6457 | CD | GLN | D | 200 | -15.538 | -39.467 | 126.778 | 1.00 | 39.26 | C |
| ATOM | 6458 | OE1 | GLN | D | 200 | -15.357 | -38.324 | 127.205 | 1.00 | 44.14 | O |
| ATOM | 6459 | NE2 | GLN | D | 200 | -15.015 | -39.893 | 125.639 | 1.00 | 40.13 | N |
| ATOM | 6460 | N | GLY | D | 201 | -18.482 | -42.261 | 129.550 | 1.00 | 34.51 | N |
| ATOM | 6461 | CA | GLY | D | 201 | -19.496 | -41.749 | 130.451 | 1.00 | 32.88 | C |
| ATOM | 6462 | C | GLY | D | 201 | -19.793 | -42.624 | 131.658 | 1.00 | 34.93 | C |
| ATOM | 6463 | O | GLY | D | 201 | -20.740 | -42.333 | 132.397 | 1.00 | 30.85 | O |
| ATOM | 6464 | N | LEU | D | 202 | -19.050 | -43.719 | 131.838 | 1.00 | 30.30 | N |
| ATOM | 6465 | CA | LEU | D | 202 | -19.137 | -44.588 | 133.007 | 1.00 | 35.37 | C |
| ATOM | 6466 | C | LEU | D | 202 | -17.845 | -44.491 | 133.805 | 1.00 | 41.52 | C |
| ATOM | 6467 | O | LEU | D | 202 | -16.751 | -44.633 | 133.240 | 1.00 | 41.27 | O |
| ATOM | 6468 | CB | LEU | D | 202 | -19.405 | -46.046 | 132.624 | 1.00 | 36.42 | C |
| ATOM | 6469 | CG | LEU | D | 202 | -20.723 | -46.250 | 131.880 | 1.00 | 35.17 | C |
| ATOM | 6470 | CD1 | LEU | D | 202 | -20.916 | -47.704 | 131.503 | 1.00 | 29.43 | C |
| ATOM | 6471 | CD2 | LEU | D | 202 | -21.865 | -45.735 | 132.713 | 1.00 | 29.90 | C |
| ATOM | 6472 | N | SER | D | 203 | -17.973 | -44.219 | 135.113 | 1.00 | 44.01 | N |
| ATOM | 6473 | CA | SER | D | 203 | -16.802 | -44.095 | 135.978 | 1.00 | 39.30 | C |
| ATOM | 6474 | C | SER | D | 203 | -16.068 | -45.416 | 136.165 | 1.00 | 41.65 | C |
| ATOM | 6475 | O | SER | D | 203 | -14.867 | -45.408 | 136.460 | 1.00 | 49.60 | O |
| ATOM | 6476 | CB | SER | D | 203 | -17.213 | -43.537 | 137.323 | 1.00 | 34.65 | C |
| ATOM | 6477 | OG | SER | D | 203 | -18.227 | -44.369 | 137.839 | 1.00 | 55.55 | O |
| ATOM | 6478 | N | SER | D | 204 | -16.749 | -46.545 | 136.003 | 1.00 | 42.27 | N |
| ATOM | 6479 | CA | SER | D | 204 | -16.084 | -47.841 | 135.927 | 1.00 | 45.69 | C |
| ATOM | 6480 | C | SER | D | 204 | -16.989 | -48.788 | 135.149 | 1.00 | 36.06 | C |
| ATOM | 6481 | O | SER | D | 204 | -18.181 | -48.508 | 134.979 | 1.00 | 38.46 | O |
| ATOM | 6482 | CB | SER | D | 204 | -15.755 | -48.401 | 137.332 | 1.00 | 48.88 | C |
| ATOM | 6483 | OG | SER | D | 204 | -16.914 | -48.719 | 138.093 | 1.00 | 47.01 | O |
| ATOM | 6484 | N | PRO | D | 205 | -16.453 | -49.888 | 134.637 | 1.00 | 32.30 | N |
| ATOM | 6485 | CA | PRO | D | 205 | -17.271 | -50.768 | 133.791 | 1.00 | 36.16 | C |
| ATOM | 6486 | C | PRO | D | 205 | -18.534 | -51.253 | 134.498 | 1.00 | 37.49 | C |
| ATOM | 6487 | O | PRO | D | 205 | -18.503 | -51.695 | 135.642 | 1.00 | 46.01 | O |
| ATOM | 6488 | CB | PRO | D | 205 | -16.313 | -51.925 | 133.468 | 1.00 | 34.46 | C |
| ATOM | 6489 | CG | PRO | D | 205 | -14.973 | -51.268 | 133.442 | 1.00 | 33.52 | C |
| ATOM | 6490 | CD | PRO | D | 205 | -15.013 | -50.158 | 134.481 | 1.00 | 32.12 | C |
| ATOM | 6491 | N | VAL | D | 206 | -19.629 | -51.261 | 133.750 | 1.00 | 37.13 | N |
| ATOM | 6492 | CA | VAL | D | 206 | -20.952 | -51.648 | 134.217 | 1.00 | 32.96 | C |
| ATOM | 6493 | C | VAL | D | 206 | -21.270 | -53.027 | 133.650 | 1.00 | 35.67 | C |
| ATOM | 6494 | O | VAL | D | 206 | -21.071 | -53.272 | 132.456 | 1.00 | 33.93 | O |
| ATOM | 6495 | CB | VAL | D | 206 | -21.984 | -50.601 | 133.769 | 1.00 | 30.63 | C |
| ATOM | 6496 | CG1 | VAL | D | 206 | -23.390 | -51.059 | 134.005 | 1.00 | 30.69 | C |
| ATOM | 6497 | CG2 | VAL | D | 206 | -21.703 | -49.316 | 134.479 | 1.00 | 38.52 | C |
| ATOM | 6498 | N | THR | D | 207 | -21.748 | -53.932 | 134.503 | 1.00 | 40.16 | N |
| ATOM | 6499 | CA | THR | D | 207 | -22.093 | -55.285 | 134.087 | 1.00 | 33.00 | C |
| ATOM | 6500 | C | THR | D | 207 | -23.587 | -55.538 | 134.269 | 1.00 | 33.37 | C |
| ATOM | 6501 | O | THR | D | 207 | -24.188 | -55.112 | 135.255 | 1.00 | 34.36 | O |
| ATOM | 6502 | CB | THR | D | 207 | -21.271 | -56.317 | 134.871 | 1.00 | 32.59 | C |
| ATOM | 6503 | OG1 | THR | D | 207 | -19.872 | -56.064 | 134.677 | 1.00 | 35.95 | O |
| ATOM | 6504 | CG2 | THR | D | 207 | -21.585 | -57.744 | 134.412 | 1.00 | 31.54 | C |
| ATOM | 6505 | N | LYS | D | 208 | -24.194 | -56.172 | 133.277 | 1.00 | 38.08 | N |
| ATOM | 6506 | CA | LYS | D | 208 | -25.529 | -56.734 | 133.393 | 1.00 | 33.58 | C |
| ATOM | 6507 | C | LYS | D | 208 | -25.435 | -58.233 | 133.155 | 1.00 | 31.51 | C |
| ATOM | 6508 | O | LYS | D | 208 | -24.797 | -58.679 | 132.202 | 1.00 | 36.26 | O |
| ATOM | 6509 | CB | LYS | D | 208 | -26.510 | -56.072 | 132.395 | 1.00 | 32.49 | C |
| ATOM | 6510 | CG | LYS | D | 208 | -26.633 | -54.559 | 132.573 | 1.00 | 34.11 | C |
| ATOM | 6511 | CD | LYS | D | 208 | -26.879 | -54.180 | 134.030 | 1.00 | 32.10 | C |
| ATOM | 6512 | CE | LYS | D | 208 | -27.135 | -52.694 | 134.212 | 1.00 | 31.43 | C |
| ATOM | 6513 | NZ | LYS | D | 208 | -28.595 | -52.396 | 134.289 | 1.00 | 30.11 | N1+ |
| ATOM | 6514 | N | SER | D | 209 | -26.099 | -59.013 | 133.995 | 1.00 | 40.89 | N |
| ATOM | 6515 | CA | SER | D | 209 | -25.955 | -60.457 | 133.950 | 1.00 | 34.12 | C |
| ATOM | 6516 | C | SER | D | 209 | -27.289 | -61.121 | 134.250 | 1.00 | 35.96 | C |
| ATOM | 6517 | O | SER | D | 209 | -28.265 | -60.469 | 134.620 | 1.00 | 35.44 | O |
| ATOM | 6518 | CB | SER | D | 209 | -24.856 | -60.923 | 134.926 | 1.00 | 34.50 | C |
| ATOM | 6519 | OG | SER | D | 209 | -25.082 | -60.438 | 136.238 | 1.00 | 29.12 | O |
| ATOM | 6520 | N | PHE | D | 210 | -27.333 | -62.431 | 134.025 | 1.00 | 39.78 | N |
| ATOM | 6521 | CA | PHE | D | 210 | -28.424 | -63.272 | 134.488 | 1.00 | 37.92 | C |
| ATOM | 6522 | C | PHE | D | 210 | -27.883 | -64.670 | 134.767 | 1.00 | 42.87 | C |
| ATOM | 6523 | O | PHE | D | 210 | -26.994 | -65.149 | 134.055 | 1.00 | 42.97 | O |
| ATOM | 6524 | CB | PHE | D | 210 | -29.555 | -63.296 | 133.464 | 1.00 | 34.40 | C |
| ATOM | 6525 | CG | PHE | D | 210 | -29.193 | -63.943 | 132.160 | 1.00 | 40.31 | C |
| ATOM | 6526 | CD1 | PHE | D | 210 | -29.365 | -65.304 | 131.973 | 1.00 | 38.92 | C |
| ATOM | 6527 | CD2 | PHE | D | 210 | -28.702 | -63.182 | 131.109 | 1.00 | 37.04 | C |

```
ATOM   6528  CE1 PHE D 210     -29.055 -65.893 130.777  1.00 36.51           C
ATOM   6529  CE2 PHE D 210     -28.393 -63.765 129.913  1.00 35.57           C
ATOM   6530  CZ  PHE D 210     -28.568 -65.125 129.745  1.00 36.28           C
ATOM   6531  N   ASN D 211     -28.433 -65.331 135.796  1.00 41.98           N
ATOM   6532  CA  ASN D 211     -28.093 -66.720 136.076  1.00 44.07           C
ATOM   6533  C   ASN D 211     -29.047 -67.583 135.273  1.00 45.82           C
ATOM   6534  O   ASN D 211     -30.260 -67.386 135.349  1.00 49.01           O
ATOM   6535  CB  ASN D 211     -28.194 -67.060 137.568  1.00 42.88           C
ATOM   6536  CG  ASN D 211     -27.474 -66.058 138.456  1.00 48.18           C
ATOM   6537  OD1 ASN D 211     -26.529 -65.407 138.034  1.00 55.66           O
ATOM   6538  ND2 ASN D 211     -27.920 -65.934 139.701  1.00 53.20           N
ATOM   6539  N   ARG D 212     -28.501 -68.538 134.512  1.00 42.56           N
ATOM   6540  CA  ARG D 212     -29.339 -69.369 133.655  1.00 44.17           C
ATOM   6541  C   ARG D 212     -30.389 -70.120 134.460  1.00 52.65           C
ATOM   6542  O   ARG D 212     -30.117 -70.632 135.550  1.00 57.95           O
ATOM   6543  CB  ARG D 212     -28.497 -70.365 132.870  1.00 44.08           C
ATOM   6544  CG  ARG D 212     -29.313 -71.160 131.848  1.00 39.96           C
ATOM   6545  CD  ARG D 212     -28.430 -72.092 131.021  1.00 41.55           C
ATOM   6546  NE  ARG D 212     -27.681 -72.968 131.906  1.00 52.14           N
ATOM   6547  CZ  ARG D 212     -28.121 -74.138 132.360  1.00 52.75           C
ATOM   6548  NH1 ARG D 212     -29.310 -74.604 131.997  1.00 55.42           N1+
ATOM   6549  NH2 ARG D 212     -27.365 -74.845 133.177  1.00 50.00           N
ATOM   6550  N   GLY D 213     -31.606 -70.150 133.923  1.00 55.21           N
ATOM   6551  CA  GLY D 213     -32.710 -70.874 134.521  1.00 61.80           C
ATOM   6552  C   GLY D 213     -33.365 -70.219 135.716  1.00 69.36           C
ATOM   6553  O   GLY D 213     -34.221 -70.844 136.350  1.00 78.60           O
ATOM   6554  N   GLU D 214     -33.041 -68.971 136.017  1.00 64.22           N
ATOM   6555  CA  GLU D 214     -33.596 -68.288 137.169  1.00 66.25           C
ATOM   6556  C   GLU D 214     -34.131 -66.936 136.710  1.00 74.30           C
ATOM   6557  O   GLU D 214     -34.125 -66.617 135.517  1.00 72.76           O
ATOM   6558  CB  GLU D 214     -32.534 -68.153 138.268  1.00 68.20           C
ATOM   6559  CG  GLU D 214     -31.925 -69.500 138.684  1.00 65.48           C
ATOM   6560  CD  GLU D 214     -30.610 -69.356 139.461  1.00 70.80           C
ATOM   6561  OE1 GLU D 214     -30.431 -68.335 140.180  1.00 56.20           O
ATOM   6562  OE2 GLU D 214     -29.760 -70.276 139.354  1.00 68.01           O1-
ATOM   6563  N   CYS D 215     -34.599 -66.138 137.668  1.00 83.83           N
ATOM   6564  CA  CYS D 215     -35.238 -64.844 137.389  1.00 81.44           C
ATOM   6565  C   CYS D 215     -34.229 -63.723 137.129  1.00 91.66           C
ATOM   6566  O   CYS D 215     -33.764 -63.051 138.055  1.00 89.67           O
ATOM   6567  CB  CYS D 215     -36.153 -64.449 138.554  1.00 87.78           C
ATOM   6568  SG  CYS D 215     -35.402 -64.679 140.202  1.00106.03           S
TER
ATOM   6569  N   THR I 152     -42.630 -26.640  53.407  1.00 78.60      D000 N
ATOM   6570  CA  THR I 152     -41.299 -26.066  53.631  1.00 90.61      D000 C
ATOM   6571  C   THR I 152     -41.155 -25.639  55.096  1.00 90.48      D000 C
ATOM   6572  O   THR I 152     -40.054 -25.653  55.660  1.00 85.79      D000 O
ATOM   6573  CB  THR I 152     -41.001 -24.845  52.694  1.00 84.39      D000 C
ATOM   6574  OG1 THR I 152     -42.169 -24.027  52.567  1.00 87.95      D000 O
ATOM   6575  CG2 THR I 152     -40.569 -25.297  51.311  1.00 74.94      D000 C
ATOM   6576  N   CYS I 153     -42.275 -25.254  55.707  1.00 89.21      D000 N
ATOM   6577  CA  CYS I 153     -42.264 -24.807  57.092  1.00 81.53      D000 C
ATOM   6578  C   CYS I 153     -43.556 -25.234  57.776  1.00 76.72      D000 C
ATOM   6579  O   CYS I 153     -44.575 -25.496  57.128  1.00 77.13      D000 O
ATOM   6580  CB  CYS I 153     -42.087 -23.284  57.178  1.00 79.48      D000 C
ATOM   6581  SG  CYS I 153     -40.357 -22.736  57.167  1.00 91.46      D000 S
ATOM   6582  N   CYS I 154     -43.494 -25.316  59.112  1.00 68.58      D000 N
ATOM   6583  CA  CYS I 154     -44.632 -25.662  59.947  1.00 56.00      D000 C
ATOM   6584  C   CYS I 154     -45.352 -24.407  60.411  1.00 54.60      D000 C
ATOM   6585  O   CYS I 154     -44.726 -23.355  60.580  1.00 53.80      D000 O
ATOM   6586  CB  CYS I 154     -44.181 -26.474  61.153  1.00 48.63      D000 C
ATOM   6587  SG  CYS I 154     -43.645 -28.112  60.680  1.00 63.81      D000 S
ATOM   6588  N   PRO I 155     -46.667 -24.480  60.621  1.00 47.88      D000 N
ATOM   6589  CA  PRO I 155     -47.397 -23.284  61.039  1.00 41.43      D000 C
ATOM   6590  C   PRO I 155     -46.878 -22.765  62.371  1.00 47.89      D000 C
ATOM   6591  O   PRO I 155     -46.206 -23.471  63.130  1.00 43.34      D000 O
ATOM   6592  CB  PRO I 155     -48.855 -23.757  61.137  1.00 38.59      D000 C
ATOM   6593  CG  PRO I 155     -48.818 -25.245  61.066  1.00 40.42      D000 C
ATOM   6594  CD  PRO I 155     -47.558 -25.631  60.385  1.00 47.80      D000 C
ATOM   6595  N   VAL I 156     -47.223 -21.503  62.648  1.00 50.69      D000 N
ATOM   6596  CA  VAL I 156     -46.805 -20.823  63.868  1.00 49.74      D000 C
ATOM   6597  C   VAL I 156     -47.161 -21.667  65.084  1.00 50.63      D000 C
```

| | | | | | | | | | | | | |
|---|---|---|---|---|---|---|---|---|---|---|---|---|
| ATOM | 6598 | O | VAL | I | 156 | -48.294 | -22.142 | 65.215 | 1.00 | 49.76 | D000 | O |
| ATOM | 6599 | CB | VAL | I | 156 | -47.471 | -19.435 | 63.931 | 1.00 | 56.06 | D000 | C |
| ATOM | 6600 | CG1 | VAL | I | 156 | -46.956 | -18.628 | 65.112 | 1.00 | 48.56 | D000 | C |
| ATOM | 6601 | CG2 | VAL | I | 156 | -47.275 | -18.685 | 62.605 | 1.00 | 59.05 | D000 | C |
| ATOM | 6602 | N | ASN | I | 157 | -46.178 | -21.872 | 65.968 | 1.00 | 51.11 | D000 | N |
| ATOM | 6603 | CA | ASN | I | 157 | -46.292 | -22.645 | 67.205 | 1.00 | 44.19 | D000 | C |
| ATOM | 6604 | C | ASN | I | 157 | -46.372 | -24.144 | 66.981 | 1.00 | 40.81 | D000 | C |
| ATOM | 6605 | O | ASN | I | 157 | -46.737 | -24.864 | 67.902 | 1.00 | 42.83 | D000 | O |
| ATOM | 6606 | CB | ASN | I | 157 | -47.464 | -22.193 | 68.081 | 1.00 | 45.04 | D000 | C |
| ATOM | 6607 | CG | ASN | I | 157 | -47.278 | -20.791 | 68.601 | 1.00 | 50.02 | D000 | C |
| ATOM | 6608 | OD1 | ASN | I | 157 | -46.197 | -20.437 | 69.063 | 1.00 | 54.69 | D000 | O |
| ATOM | 6609 | ND2 | ASN | I | 157 | -48.315 | -19.979 | 68.513 | 1.00 | 59.66 | D000 | N |
| ATOM | 6610 | N | TRP | I | 158 | -46.085 | -24.633 | 65.778 | 1.00 | 40.56 | D000 | N |
| ATOM | 6611 | CA | TRP | I | 158 | -45.839 | -26.051 | 65.566 | 1.00 | 38.45 | D000 | C |
| ATOM | 6612 | C | TRP | I | 158 | -44.345 | -26.293 | 65.371 | 1.00 | 40.31 | D000 | C |
| ATOM | 6613 | O | TRP | I | 158 | -43.584 | -25.397 | 65.007 | 1.00 | 39.61 | D000 | O |
| ATOM | 6614 | CB | TRP | I | 158 | -46.629 | -26.590 | 64.371 | 1.00 | 40.79 | D000 | C |
| ATOM | 6615 | CG | TRP | I | 158 | -48.118 | -26.566 | 64.574 | 1.00 | 40.96 | D000 | C |
| ATOM | 6616 | CD1 | TRP | I | 158 | -48.892 | -25.473 | 64.883 | 1.00 | 39.11 | D000 | C |
| ATOM | 6617 | CD2 | TRP | I | 158 | -49.025 | -27.667 | 64.427 | 1.00 | 36.12 | D000 | C |
| ATOM | 6618 | NE1 | TRP | I | 158 | -50.213 | -25.840 | 64.971 | 1.00 | 35.50 | D000 | N |
| ATOM | 6619 | CE2 | TRP | I | 158 | -50.325 | -27.175 | 64.688 | 1.00 | 32.39 | D000 | C |
| ATOM | 6620 | CE3 | TRP | I | 158 | -48.867 | -29.017 | 64.101 | 1.00 | 36.76 | D000 | C |
| ATOM | 6621 | CZ2 | TRP | I | 158 | -51.453 | -27.985 | 64.636 | 1.00 | 33.44 | D000 | C |
| ATOM | 6622 | CZ3 | TRP | I | 158 | -49.993 | -29.826 | 64.048 | 1.00 | 36.91 | D000 | C |
| ATOM | 6623 | CH2 | TRP | I | 158 | -51.271 | -29.308 | 64.321 | 1.00 | 34.08 | D000 | C |
| ATOM | 6624 | N | VAL | I | 159 | -43.939 | -27.531 | 65.615 | 1.00 | 42.08 | D000 | N |
| ATOM | 6625 | CA | VAL | I | 159 | -42.544 | -27.931 | 65.687 | 1.00 | 36.72 | D000 | C |
| ATOM | 6626 | C | VAL | I | 159 | -42.284 | -28.942 | 64.582 | 1.00 | 40.42 | D000 | C |
| ATOM | 6627 | O | VAL | I | 159 | -42.934 | -29.992 | 64.529 | 1.00 | 34.21 | D000 | O |
| ATOM | 6628 | CB | VAL | I | 159 | -42.222 | -28.551 | 67.060 | 1.00 | 40.12 | D000 | C |
| ATOM | 6629 | CG1 | VAL | I | 159 | -40.793 | -29.037 | 67.110 | 1.00 | 39.21 | D000 | C |
| ATOM | 6630 | CG2 | VAL | I | 159 | -42.486 | -27.563 | 68.171 | 1.00 | 43.22 | D000 | C |
| ATOM | 6631 | N | GLU | I | 160 | -41.300 | -28.660 | 63.739 | 1.00 | 45.25 | D000 | N |
| ATOM | 6632 | CA | GLU | I | 160 | -40.975 | -29.588 | 62.671 | 1.00 | 43.30 | D000 | C |
| ATOM | 6633 | C | GLU | I | 160 | -40.058 | -30.686 | 63.184 | 1.00 | 41.09 | D000 | C |
| ATOM | 6634 | O | GLU | I | 160 | -39.201 | -30.447 | 64.036 | 1.00 | 44.54 | D000 | O |
| ATOM | 6635 | CB | GLU | I | 160 | -40.309 | -28.861 | 61.510 | 1.00 | 49.23 | D000 | C |
| ATOM | 6636 | CG | GLU | I | 160 | -40.396 | -29.626 | 60.191 | 1.00 | 61.45 | D000 | C |
| ATOM | 6637 | CD | GLU | I | 160 | -39.556 | -28.996 | 59.095 | 1.00 | 74.24 | D000 | C |
| ATOM | 6638 | OE1 | GLU | I | 160 | -40.073 | -28.128 | 58.350 | 1.00 | 75.44 | D000 | O |
| ATOM | 6639 | OE2 | GLU | I | 160 | -38.365 | -29.367 | 58.996 | 1.00 | 82.31 | D000 | O1- |
| ATOM | 6640 | N | HIS | I | 161 | -40.262 | -31.899 | 62.675 | 1.00 | 34.25 | D000 | N |
| ATOM | 6641 | CA | HIS | I | 161 | -39.358 | -33.005 | 62.927 | 1.00 | 35.38 | D000 | C |
| ATOM | 6642 | C | HIS | I | 161 | -39.634 | -34.146 | 61.950 | 1.00 | 44.04 | D000 | C |
| ATOM | 6643 | O | HIS | I | 161 | -40.655 | -34.832 | 62.063 | 1.00 | 44.75 | D000 | O |
| ATOM | 6644 | CB | HIS | I | 161 | -39.491 | -33.493 | 64.372 | 1.00 | 34.41 | D000 | C |
| ATOM | 6645 | CG | HIS | I | 161 | -38.662 | -34.709 | 64.679 | 1.00 | 42.31 | D000 | C |
| ATOM | 6646 | ND1 | HIS | I | 161 | -37.356 | -34.632 | 65.127 | 1.00 | 39.49 | D000 | N |
| ATOM | 6647 | CD2 | HIS | I | 161 | -38.947 | -36.031 | 64.578 | 1.00 | 37.68 | D000 | C |
| ATOM | 6648 | CE1 | HIS | I | 161 | -36.884 | -35.854 | 65.308 | 1.00 | 36.84 | D000 | C |
| ATOM | 6649 | NE2 | HIS | I | 161 | -37.823 | -36.720 | 64.970 | 1.00 | 37.93 | D000 | N |
| ATOM | 6650 | N | GLU | I | 162 | -38.726 | -34.358 | 60.992 | 1.00 | 48.09 | D000 | N |
| ATOM | 6651 | CA | GLU | I | 162 | -38.750 | -35.513 | 60.090 | 1.00 | 42.19 | D000 | C |
| ATOM | 6652 | C | GLU | I | 162 | -39.999 | -35.567 | 59.225 | 1.00 | 46.13 | D000 | C |
| ATOM | 6653 | O | GLU | I | 162 | -40.676 | -36.597 | 59.149 | 1.00 | 46.71 | D000 | O |
| ATOM | 6654 | CB | GLU | I | 162 | -38.576 | -36.822 | 60.852 | 1.00 | 38.57 | D000 | C |
| ATOM | 6655 | CG | GLU | I | 162 | -37.232 | -36.891 | 61.474 | 1.00 | 49.45 | D000 | C |
| ATOM | 6656 | CD | GLU | I | 162 | -36.178 | -37.369 | 60.479 | 1.00 | 67.10 | D000 | C |
| ATOM | 6657 | OE1 | GLU | I | 162 | -36.210 | -38.565 | 60.098 | 1.00 | 74.81 | D000 | O |
| ATOM | 6658 | OE2 | GLU | I | 162 | -35.341 | -36.533 | 60.050 | 1.00 | 59.37 | D000 | O1- |
| ATOM | 6659 | N | ARG | I | 163 | -40.289 | -34.453 | 58.547 | 1.00 | 48.14 | D000 | N |
| ATOM | 6660 | CA | ARG | I | 163 | -41.446 | -34.362 | 57.648 | 1.00 | 57.33 | D000 | C |
| ATOM | 6661 | C | ARG | I | 163 | -42.768 | -34.545 | 58.387 | 1.00 | 53.34 | D000 | C |
| ATOM | 6662 | O | ARG | I | 163 | -43.712 | -35.129 | 57.854 | 1.00 | 56.97 | D000 | O |
| ATOM | 6663 | CB | ARG | I | 163 | -41.335 | -35.388 | 56.509 | 1.00 | 65.00 | D000 | C |
| ATOM | 6664 | CG | ARG | I | 163 | -40.188 | -35.176 | 55.528 | 1.00 | 68.66 | D000 | C |
| ATOM | 6665 | CD | ARG | I | 163 | -40.271 | -36.202 | 54.398 | 1.00 | 80.19 | D000 | C |
| ATOM | 6666 | NE | ARG | I | 163 | -41.472 | -36.074 | 53.577 | 1.00 | 89.56 | D000 | N |
| ATOM | 6667 | CZ | ARG | I | 163 | -41.971 | -37.066 | 52.841 | 1.00 | 92.42 | D000 | C |
| ATOM | 6668 | NH1 | ARG | I | 163 | -43.077 | -36.881 | 52.123 | 1.00 | 78.10 | D000 | N1+ |

| | | | | | | | | | | | | |
|---|---|---|---|---|---|---|---|---|---|---|---|---|
| ATOM | 6669 | NH2 | ARG | I | 163 | -41.362 | -38.249 | 52.832 | 1.00 | 94.56 | D000 | N |
| ATOM | 6670 | N | SER | I | 164 | -42.822 | -34.104 | 59.641 | 1.00 | 53.57 | D000 | N |
| ATOM | 6671 | CA | SER | I | 164 | -44.059 | -33.988 | 60.400 | 1.00 | 46.49 | D000 | C |
| ATOM | 6672 | C | SER | I | 164 | -44.026 | -32.697 | 61.203 | 1.00 | 51.04 | D000 | C |
| ATOM | 6673 | O | SER | I | 164 | -42.965 | -32.279 | 61.679 | 1.00 | 50.06 | D000 | O |
| ATOM | 6674 | CB | SER | I | 164 | -44.270 | -35.170 | 61.343 | 1.00 | 43.89 | D000 | C |
| ATOM | 6675 | OG | SER | I | 164 | -44.907 | -36.249 | 60.692 | 1.00 | 50.01 | D000 | O |
| ATOM | 6676 | N | CYS | I | 165 | -45.194 | -32.075 | 61.357 | 1.00 | 50.05 | D000 | N |
| ATOM | 6677 | CA | CYS | I | 165 | -45.370 | -30.919 | 62.233 | 1.00 | 47.87 | D000 | C |
| ATOM | 6678 | C | CYS | I | 165 | -46.135 | -31.340 | 63.489 | 1.00 | 43.11 | D000 | C |
| ATOM | 6679 | O | CYS | I | 165 | -47.190 | -31.975 | 63.399 | 1.00 | 42.93 | D000 | O |
| ATOM | 6680 | CB | CYS | I | 165 | -46.116 | -29.778 | 61.524 | 1.00 | 50.43 | D000 | C |
| ATOM | 6681 | SG | CYS | I | 165 | -45.326 | -29.058 | 60.031 | 1.00 | 58.62 | D000 | S |
| ATOM | 6682 | N | TYR | I | 166 | -45.622 | -30.957 | 64.656 | 1.00 | 43.41 | D000 | N |
| ATOM | 6683 | CA | TYR | I | 166 | -46.198 | -31.350 | 65.934 | 1.00 | 35.17 | D000 | C |
| ATOM | 6684 | C | TYR | I | 166 | -46.617 | -30.116 | 66.719 | 1.00 | 36.72 | D000 | C |
| ATOM | 6685 | O | TYR | I | 166 | -45.937 | -29.084 | 66.683 | 1.00 | 35.81 | D000 | O |
| ATOM | 6686 | CB | TYR | I | 166 | -45.214 | -32.158 | 66.761 | 1.00 | 28.42 | D000 | C |
| ATOM | 6687 | CG | TYR | I | 166 | -44.766 | -33.439 | 66.120 | 1.00 | 35.36 | D000 | C |
| ATOM | 6688 | CD1 | TYR | I | 166 | -43.703 | -33.461 | 65.194 | 1.00 | 40.74 | D000 | C |
| ATOM | 6689 | CD2 | TYR | I | 166 | -45.379 | -34.629 | 66.435 | 1.00 | 28.15 | D000 | C |
| ATOM | 6690 | CE1 | TYR | I | 166 | -43.280 | -34.646 | 64.614 | 1.00 | 31.55 | D000 | C |
| ATOM | 6691 | CE2 | TYR | I | 166 | -44.966 | -35.813 | 65.864 | 1.00 | 35.33 | D000 | C |
| ATOM | 6692 | CZ | TYR | I | 166 | -43.926 | -35.818 | 64.952 | 1.00 | 36.07 | D000 | C |
| ATOM | 6693 | OH | TYR | I | 166 | -43.557 | -37.022 | 64.406 | 1.00 | 37.06 | D000 | O |
| ATOM | 6694 | N | TRP | I | 167 | -47.735 | -30.235 | 67.438 | 1.00 | 33.78 | D000 | N |
| ATOM | 6695 | CA | TRP | I | 167 | -48.213 | -29.191 | 68.335 | 1.00 | 33.91 | D000 | C |
| ATOM | 6696 | C | TRP | I | 167 | -48.485 | -29.823 | 69.692 | 1.00 | 33.58 | D000 | C |
| ATOM | 6697 | O | TRP | I | 167 | -49.169 | -30.848 | 69.776 | 1.00 | 37.11 | D000 | O |
| ATOM | 6698 | CB | TRP | I | 167 | -49.478 | -28.509 | 67.776 | 1.00 | 37.58 | D000 | C |
| ATOM | 6699 | CG | TRP | I | 167 | -50.029 | -27.432 | 68.678 | 1.00 | 36.19 | D000 | C |
| ATOM | 6700 | CD1 | TRP | I | 167 | -49.648 | -26.122 | 68.725 | 1.00 | 37.64 | D000 | C |
| ATOM | 6701 | CD2 | TRP | I | 167 | -51.064 | -27.580 | 69.657 | 1.00 | 33.12 | D000 | C |
| ATOM | 6702 | NE1 | TRP | I | 167 | -50.365 | -25.452 | 69.692 | 1.00 | 32.45 | D000 | N |
| ATOM | 6703 | CE2 | TRP | I | 167 | -51.246 | -26.325 | 70.269 | 1.00 | 31.52 | D000 | C |
| ATOM | 6704 | CE3 | TRP | I | 167 | -51.846 | -28.659 | 70.083 | 1.00 | 35.72 | D000 | C |
| ATOM | 6705 | CZ2 | TRP | I | 167 | -52.183 | -26.116 | 71.277 | 1.00 | 34.14 | D000 | C |
| ATOM | 6706 | CZ3 | TRP | I | 167 | -52.782 | -28.447 | 71.087 | 1.00 | 32.27 | D000 | C |
| ATOM | 6707 | CH2 | TRP | I | 167 | -52.941 | -27.185 | 71.669 | 1.00 | 31.51 | D000 | C |
| ATOM | 6708 | N | PHE | I | 168 | -47.934 | -29.223 | 70.748 | 1.00 | 35.27 | D000 | N |
| ATOM | 6709 | CA | PHE | I | 168 | -47.963 | -29.784 | 72.101 | 1.00 | 31.67 | D000 | C |
| ATOM | 6710 | C | PHE | I | 168 | -48.837 | -28.898 | 72.975 | 1.00 | 31.00 | D000 | C |
| ATOM | 6711 | O | PHE | I | 168 | -48.468 | -27.757 | 73.263 | 1.00 | 28.23 | D000 | O |
| ATOM | 6712 | CB | PHE | I | 168 | -46.555 | -29.881 | 72.685 | 1.00 | 28.79 | D000 | C |
| ATOM | 6713 | CG | PHE | I | 168 | -45.609 | -30.707 | 71.860 | 1.00 | 29.81 | D000 | C |
| ATOM | 6714 | CD1 | PHE | I | 168 | -45.461 | -32.067 | 72.099 | 1.00 | 30.76 | D000 | C |
| ATOM | 6715 | CD2 | PHE | I | 168 | -44.881 | -30.129 | 70.836 | 1.00 | 27.05 | D000 | C |
| ATOM | 6716 | CE1 | PHE | I | 168 | -44.587 | -32.824 | 71.329 | 1.00 | 29.28 | D000 | C |
| ATOM | 6717 | CE2 | PHE | I | 168 | -44.009 | -30.878 | 70.073 | 1.00 | 27.44 | D000 | C |
| ATOM | 6718 | CZ | PHE | I | 168 | -43.869 | -32.224 | 70.310 | 1.00 | 26.73 | D000 | C |
| ATOM | 6719 | N | SER | I | 169 | -49.963 | -29.437 | 73.440 | 1.00 | 31.45 | D000 | N |
| ATOM | 6720 | CA | SER | I | 169 | -50.883 | -28.643 | 74.245 | 1.00 | 31.26 | D000 | C |
| ATOM | 6721 | C | SER | I | 169 | -50.266 | -28.259 | 75.591 | 1.00 | 25.88 | D000 | C |
| ATOM | 6722 | O | SER | I | 169 | -49.376 | -28.932 | 76.115 | 1.00 | 23.51 | D000 | O |
| ATOM | 6723 | CB | SER | I | 169 | -52.201 | -29.404 | 74.471 | 1.00 | 30.30 | D000 | C |
| ATOM | 6724 | OG | SER | I | 169 | -52.074 | -30.442 | 75.438 | 1.00 | 28.82 | D000 | O |
| ATOM | 6725 | N | ARG | I | 170 | -50.734 | -27.135 | 76.129 | 1.00 | 27.95 | D000 | N |
| ATOM | 6726 | CA | ARG | I | 170 | -50.447 | -26.722 | 77.495 | 1.00 | 34.22 | D000 | C |
| ATOM | 6727 | C | ARG | I | 170 | -51.671 | -26.846 | 78.392 | 1.00 | 33.58 | D000 | C |
| ATOM | 6728 | O | ARG | I | 170 | -51.627 | -26.414 | 79.546 | 1.00 | 31.86 | D000 | O |
| ATOM | 6729 | CB | ARG | I | 170 | -49.944 | -25.282 | 77.529 | 1.00 | 25.51 | D000 | C |
| ATOM | 6730 | CG | ARG | I | 170 | -48.455 | -25.122 | 77.693 | 1.00 | 33.77 | D000 | C |
| ATOM | 6731 | CD | ARG | I | 170 | -48.071 | -25.130 | 79.130 | 1.00 | 32.63 | D000 | C |
| ATOM | 6732 | NE | ARG | I | 170 | -47.020 | -26.113 | 79.328 | 1.00 | 38.28 | D000 | N |
| ATOM | 6733 | CZ | ARG | I | 170 | -46.005 | -25.992 | 80.166 | 1.00 | 33.99 | D000 | C |
| ATOM | 6734 | NH1 | ARG | I | 170 | -45.904 | -24.932 | 80.960 | 1.00 | 29.65 | D000 | N1+ |
| ATOM | 6735 | NH2 | ARG | I | 170 | -45.123 | -26.986 | 80.241 | 1.00 | 33.21 | D000 | N |
| ATOM | 6736 | N | SER | I | 171 | -52.760 | -27.420 | 77.888 | 1.00 | 27.31 | D000 | N |
| ATOM | 6737 | CA | SER | I | 171 | -53.943 | -27.684 | 78.682 | 1.00 | 24.95 | D000 | C |
| ATOM | 6738 | C | SER | I | 171 | -54.303 | -29.138 | 78.489 | 1.00 | 29.34 | D000 | C |
| ATOM | 6739 | O | SER | I | 171 | -53.693 | -29.847 | 77.682 | 1.00 | 31.49 | D000 | O |

| | | | | | | | | | | | | |
|---|---|---|---|---|---|---|---|---|---|---|---|---|
| ATOM | 6740 | CB | SER | I | 171 | -55.119 | -26.815 | 78.277 | 1.00 | 21.97 | D000 | C |
| ATOM | 6741 | OG | SER | I | 171 | -55.427 | -27.085 | 76.932 | 1.00 | 35.69 | D000 | O |
| ATOM | 6742 | N | GLY | I | 172 | -55.301 | -29.577 | 79.252 | 1.00 | 29.63 | D000 | N |
| ATOM | 6743 | CA | GLY | I | 172 | -55.700 | -30.970 | 79.280 | 1.00 | 24.44 | D000 | C |
| ATOM | 6744 | C | GLY | I | 172 | -57.075 | -31.181 | 78.703 | 1.00 | 25.61 | D000 | C |
| ATOM | 6745 | O | GLY | I | 172 | -57.864 | -30.245 | 78.637 | 1.00 | 27.53 | D000 | O |
| ATOM | 6746 | N | LYS | I | 173 | -57.363 | -32.400 | 78.273 | 1.00 | 25.19 | D000 | N |
| ATOM | 6747 | CA | LYS | I | 173 | -58.629 | -32.726 | 77.650 | 1.00 | 27.09 | D000 | C |
| ATOM | 6748 | C | LYS | I | 173 | -58.823 | -34.231 | 77.752 | 1.00 | 33.35 | D000 | C |
| ATOM | 6749 | O | LYS | I | 173 | -57.854 | -34.995 | 77.672 | 1.00 | 29.52 | D000 | O |
| ATOM | 6750 | CB | LYS | I | 173 | -58.693 | -32.295 | 76.172 | 1.00 | 27.91 | D000 | C |
| ATOM | 6751 | CG | LYS | I | 173 | -58.975 | -30.805 | 75.865 | 1.00 | 25.52 | D000 | C |
| ATOM | 6752 | CD | LYS | I | 173 | -59.510 | -30.671 | 74.410 | 1.00 | 26.32 | D000 | C |
| ATOM | 6753 | CE | LYS | I | 173 | -59.494 | -29.257 | 73.861 | 1.00 | 19.84 | D000 | C |
| ATOM | 6754 | NZ | LYS | I | 173 | -60.449 | -28.327 | 74.536 | 1.00 | 27.70 | D000 | N1+ |
| ATOM | 6755 | N | ALA | I | 174 | -60.076 | -34.643 | 77.956 | 1.00 | 29.60 | D000 | N |
| ATOM | 6756 | CA | ALA | I | 174 | -60.433 | -36.038 | 77.791 | 1.00 | 32.67 | D000 | C |
| ATOM | 6757 | C | ALA | I | 174 | -60.003 | -36.509 | 76.401 | 1.00 | 33.60 | D000 | C |
| ATOM | 6758 | O | ALA | I | 174 | -59.996 | -35.730 | 75.441 | 1.00 | 31.75 | D000 | O |
| ATOM | 6759 | CB | ALA | I | 174 | -61.943 | -36.202 | 77.997 | 1.00 | 28.75 | D000 | C |
| ATOM | 6760 | N | TRP | I | 175 | -59.641 | -37.796 | 76.289 | 1.00 | 30.30 | D000 | N |
| ATOM | 6761 | CA | TRP | I | 175 | -59.069 | -38.288 | 75.033 | 1.00 | 32.92 | D000 | C |
| ATOM | 6762 | C | TRP | I | 175 | -59.946 | -37.956 | 73.824 | 1.00 | 35.10 | D000 | C |
| ATOM | 6763 | O | TRP | I | 175 | -59.441 | -37.507 | 72.790 | 1.00 | 38.08 | D000 | O |
| ATOM | 6764 | CB | TRP | I | 175 | -58.822 | -39.795 | 75.102 | 1.00 | 33.10 | D000 | C |
| ATOM | 6765 | CG | TRP | I | 175 | -58.056 | -40.337 | 73.893 | 1.00 | 38.61 | D000 | C |
| ATOM | 6766 | CD1 | TRP | I | 175 | -56.729 | -40.615 | 73.835 | 1.00 | 38.20 | D000 | C |
| ATOM | 6767 | CD2 | TRP | I | 175 | -58.584 | -40.653 | 72.579 | 1.00 | 43.49 | D000 | C |
| ATOM | 6768 | NE1 | TRP | I | 175 | -56.388 | -41.079 | 72.580 | 1.00 | 35.04 | D000 | N |
| ATOM | 6769 | CE2 | TRP | I | 175 | -57.505 | -41.113 | 71.793 | 1.00 | 38.92 | D000 | C |
| ATOM | 6770 | CE3 | TRP | I | 175 | -59.863 | -40.593 | 71.996 | 1.00 | 43.77 | D000 | C |
| ATOM | 6771 | CZ2 | TRP | I | 175 | -57.662 | -41.517 | 70.456 | 1.00 | 44.27 | D000 | C |
| ATOM | 6772 | CZ3 | TRP | I | 175 | -60.016 | -40.989 | 70.650 | 1.00 | 43.79 | D000 | C |
| ATOM | 6773 | CH2 | TRP | I | 175 | -58.925 | -41.448 | 69.906 | 1.00 | 44.29 | D000 | C |
| ATOM | 6774 | N | ALA | I | 176 | -61.260 | -38.156 | 73.936 | 1.00 | 33.82 | D000 | N |
| ATOM | 6775 | CA | ALA | I | 176 | -62.154 | -37.894 | 72.815 | 1.00 | 28.11 | D000 | C |
| ATOM | 6776 | C | ALA | I | 176 | -62.108 | -36.432 | 72.394 | 1.00 | 37.58 | D000 | C |
| ATOM | 6777 | O | ALA | I | 176 | -62.150 | -36.111 | 71.196 | 1.00 | 36.74 | D000 | O |
| ATOM | 6778 | CB | ALA | I | 176 | -63.578 | -38.275 | 73.199 | 1.00 | 27.93 | D000 | C |
| ATOM | 6779 | N | ASP | I | 177 | -62.043 | -35.528 | 73.370 | 1.00 | 35.00 | D000 | N |
| ATOM | 6780 | CA | ASP | I | 177 | -62.036 | -34.117 | 73.044 | 1.00 | 32.37 | D000 | C |
| ATOM | 6781 | C | ASP | I | 177 | -60.712 | -33.703 | 72.435 | 1.00 | 34.46 | D000 | C |
| ATOM | 6782 | O | ASP | I | 177 | -60.672 | -32.790 | 71.591 | 1.00 | 33.36 | D000 | O |
| ATOM | 6783 | CB | ASP | I | 177 | -62.355 | -33.298 | 74.290 | 1.00 | 36.16 | D000 | C |
| ATOM | 6784 | CG | ASP | I | 177 | -63.729 | -33.623 | 74.876 | 1.00 | 40.76 | D000 | C |
| ATOM | 6785 | OD1 | ASP | I | 177 | -64.648 | -33.982 | 74.097 | 1.00 | 35.33 | D000 | O |
| ATOM | 6786 | OD2 | ASP | I | 177 | -63.888 | -33.508 | 76.122 | 1.00 | 45.92 | D000 | O1- |
| ATOM | 6787 | N | ALA | I | 178 | -59.622 | -34.340 | 72.869 | 1.00 | 33.13 | D000 | N |
| ATOM | 6788 | CA | ALA | I | 178 | -58.325 | -34.082 | 72.255 | 1.00 | 34.80 | D000 | C |
| ATOM | 6789 | C | ALA | I | 178 | -58.313 | -34.585 | 70.812 | 1.00 | 35.86 | D000 | C |
| ATOM | 6790 | O | ALA | I | 178 | -57.887 | -33.873 | 69.898 | 1.00 | 30.16 | D000 | O |
| ATOM | 6791 | CB | ALA | I | 178 | -57.224 | -34.734 | 73.085 | 1.00 | 29.11 | D000 | C |
| ATOM | 6792 | N | ASP | I | 179 | -58.825 | -35.803 | 70.601 | 1.00 | 38.39 | D000 | N |
| ATOM | 6793 | CA | ASP | I | 179 | -59.085 | -36.343 | 69.268 | 1.00 | 35.65 | D000 | C |
| ATOM | 6794 | C | ASP | I | 179 | -59.826 | -35.347 | 68.401 | 1.00 | 37.02 | D000 | C |
| ATOM | 6795 | O | ASP | I | 179 | -59.397 | -35.030 | 67.286 | 1.00 | 39.99 | D000 | O |
| ATOM | 6796 | CB | ASP | I | 179 | -59.886 | -37.642 | 69.413 | 1.00 | 41.79 | D000 | C |
| ATOM | 6797 | CG | ASP | I | 179 | -60.149 | -38.338 | 68.094 | 1.00 | 42.90 | D000 | C |
| ATOM | 6798 | OD1 | ASP | I | 179 | -59.371 | -38.120 | 67.149 | 1.00 | 43.83 | D000 | O |
| ATOM | 6799 | OD2 | ASP | I | 179 | -61.096 | -39.163 | 68.035 | 1.00 | 43.02 | D000 | O1- |
| ATOM | 6800 | N | ASN | I | 180 | -60.934 | -34.820 | 68.911 | 1.00 | 39.12 | D000 | N |
| ATOM | 6801 | CA | ASN | I | 180 | -61.715 | -33.882 | 68.117 | 1.00 | 39.76 | D000 | C |
| ATOM | 6802 | C | ASN | I | 180 | -60.945 | -32.601 | 67.851 | 1.00 | 32.43 | D000 | C |
| ATOM | 6803 | O | ASN | I | 180 | -61.062 | -32.026 | 66.768 | 1.00 | 38.63 | D000 | O |
| ATOM | 6804 | CB | ASN | I | 180 | -63.053 | -33.580 | 68.801 | 1.00 | 34.44 | D000 | C |
| ATOM | 6805 | CG | ASN | I | 180 | -63.983 | -34.773 | 68.793 | 1.00 | 38.43 | D000 | C |
| ATOM | 6806 | OD1 | ASN | I | 180 | -63.692 | -35.795 | 68.162 | 1.00 | 43.96 | D000 | O |
| ATOM | 6807 | ND2 | ASN | I | 180 | -65.126 | -34.642 | 69.458 | 1.00 | 38.70 | D000 | N |
| ATOM | 6808 | N | TYR | I | 181 | -60.142 | -32.144 | 68.814 | 1.00 | 37.95 | D000 | N |
| ATOM | 6809 | CA | TYR | I | 181 | -59.388 | -30.900 | 68.623 | 1.00 | 36.10 | D000 | C |
| ATOM | 6810 | C | TYR | I | 181 | -58.390 | -31.012 | 67.470 | 1.00 | 36.90 | D000 | C |

| | | | | | | | | | | | | |
|---|---|---|---|---|---|---|---|---|---|---|---|---|
| ATOM | 6811 | O | TYR | I | 181 | -58.240 | -30.077 | 66.667 | 1.00 | 35.28 | D000 | O |
| ATOM | 6812 | CB | TYR | I | 181 | -58.660 | -30.519 | 69.908 | 1.00 | 25.56 | D000 | C |
| ATOM | 6813 | CG | TYR | I | 181 | -57.826 | -29.264 | 69.780 | 1.00 | 28.66 | D000 | C |
| ATOM | 6814 | CD1 | TYR | I | 181 | -56.520 | -29.301 | 69.277 | 1.00 | 31.67 | D000 | C |
| ATOM | 6815 | CD2 | TYR | I | 181 | -58.336 | -28.043 | 70.162 | 1.00 | 25.48 | D000 | C |
| ATOM | 6816 | CE1 | TYR | I | 181 | -55.768 | -28.154 | 69.148 | 1.00 | 29.47 | D000 | C |
| ATOM | 6817 | CE2 | TYR | I | 181 | -57.579 | -26.897 | 70.054 | 1.00 | 29.61 | D000 | C |
| ATOM | 6818 | CZ | TYR | I | 181 | -56.299 | -26.952 | 69.546 | 1.00 | 33.58 | D000 | C |
| ATOM | 6819 | OH | TYR | I | 181 | -55.570 | -25.786 | 69.431 | 1.00 | 36.52 | D000 | O |
| ATOM | 6820 | N | CYS | I | 182 | -57.668 | -32.125 | 67.395 | 1.00 | 30.45 | D000 | N |
| ATOM | 6821 | CA | CYS | I | 182 | -56.669 | -32.250 | 66.346 | 1.00 | 39.92 | D000 | C |
| ATOM | 6822 | C | CYS | I | 182 | -57.323 | -32.304 | 64.964 | 1.00 | 42.48 | D000 | C |
| ATOM | 6823 | O | CYS | I | 182 | -56.879 | -31.607 | 64.034 | 1.00 | 37.23 | D000 | O |
| ATOM | 6824 | CB | CYS | I | 182 | -55.793 | -33.477 | 66.614 | 1.00 | 37.71 | D000 | C |
| ATOM | 6825 | SG | CYS | I | 182 | -54.685 | -33.283 | 68.064 | 1.00 | 42.44 | D000 | S |
| ATOM | 6826 | N | ARG | I | 183 | -58.413 | -33.073 | 64.832 | 1.00 | 33.83 | D000 | N |
| ATOM | 6827 | CA | ARG | I | 183 | -59.071 | -33.213 | 63.538 | 1.00 | 34.20 | D000 | C |
| ATOM | 6828 | C | ARG | I | 183 | -59.478 | -31.860 | 62.965 | 1.00 | 40.37 | D000 | C |
| ATOM | 6829 | O | ARG | I | 183 | -59.310 | -31.617 | 61.764 | 1.00 | 43.30 | D000 | O |
| ATOM | 6830 | CB | ARG | I | 183 | -60.275 | -34.128 | 63.670 | 1.00 | 36.10 | D000 | C |
| ATOM | 6831 | CG | ARG | I | 183 | -59.902 | -35.562 | 63.991 | 1.00 | 34.06 | D000 | C |
| ATOM | 6832 | CD | ARG | I | 183 | -61.081 | -36.236 | 64.587 | 1.00 | 39.52 | D000 | C |
| ATOM | 6833 | NE | ARG | I | 183 | -61.350 | -37.531 | 63.997 | 1.00 | 57.45 | D000 | N |
| ATOM | 6834 | CZ | ARG | I | 183 | -62.574 | -37.960 | 63.698 | 1.00 | 68.60 | D000 | C |
| ATOM | 6835 | NH1 | ARG | I | 183 | -63.619 | -37.172 | 63.920 | 1.00 | 55.03 | D000 | N1+ |
| ATOM | 6836 | NH2 | ARG | I | 183 | -62.754 | -39.165 | 63.159 | 1.00 | 76.79 | D000 | N |
| ATOM | 6837 | N | LEU | I | 184 | -59.980 | -30.952 | 63.805 | 1.00 | 34.08 | D000 | N |
| ATOM | 6838 | CA | LEU | I | 184 | -60.347 | -29.625 | 63.319 | 1.00 | 36.10 | D000 | C |
| ATOM | 6839 | C | LEU | I | 184 | -59.159 | -28.729 | 62.998 | 1.00 | 40.84 | D000 | C |
| ATOM | 6840 | O | LEU | I | 184 | -59.369 | -27.647 | 62.436 | 1.00 | 43.24 | D000 | O |
| ATOM | 6841 | CB | LEU | I | 184 | -61.207 | -28.901 | 64.344 | 1.00 | 42.44 | D000 | C |
| ATOM | 6842 | CG | LEU | I | 184 | -62.705 | -29.142 | 64.369 | 1.00 | 44.30 | D000 | C |
| ATOM | 6843 | CD1 | LEU | I | 184 | -62.982 | -30.550 | 64.684 | 1.00 | 40.27 | D000 | C |
| ATOM | 6844 | CD2 | LEU | I | 184 | -63.246 | -28.314 | 65.458 | 1.00 | 44.72 | D000 | C |
| ATOM | 6845 | N | GLU | I | 185 | -57.938 | -29.126 | 63.359 | 1.00 | 44.61 | D000 | N |
| ATOM | 6846 | CA | GLU | I | 185 | -56.714 | -28.453 | 62.938 | 1.00 | 42.87 | D000 | C |
| ATOM | 6847 | C | GLU | I | 185 | -56.132 | -29.068 | 61.664 | 1.00 | 45.96 | D000 | C |
| ATOM | 6848 | O | GLU | I | 185 | -55.000 | -28.744 | 61.284 | 1.00 | 41.44 | D000 | O |
| ATOM | 6849 | CB | GLU | I | 185 | -55.678 | -28.517 | 64.061 | 1.00 | 38.65 | D000 | C |
| ATOM | 6850 | CG | GLU | I | 185 | -56.035 | -27.729 | 65.294 | 1.00 | 42.71 | D000 | C |
| ATOM | 6851 | CD | GLU | I | 185 | -55.905 | -26.233 | 65.117 | 1.00 | 46.41 | D000 | C |
| ATOM | 6852 | OE1 | GLU | I | 185 | -54.995 | -25.799 | 64.379 | 1.00 | 49.20 | D000 | O |
| ATOM | 6853 | OE2 | GLU | I | 185 | -56.701 | -25.489 | 65.737 | 1.00 | 47.94 | D000 | O1- |
| ATOM | 6854 | N | ASP | I | 186 | -56.902 | -29.923 | 60.992 | 1.00 | 46.15 | D000 | N |
| ATOM | 6855 | CA | ASP | I | 186 | -56.421 | -30.765 | 59.904 | 1.00 | 46.32 | D000 | C |
| ATOM | 6856 | C | ASP | I | 186 | -55.202 | -31.570 | 60.344 | 1.00 | 45.43 | D000 | C |
| ATOM | 6857 | O | ASP | I | 186 | -54.175 | -31.625 | 59.670 | 1.00 | 47.14 | D000 | O |
| ATOM | 6858 | CB | ASP | I | 186 | -56.136 | -29.951 | 58.642 | 1.00 | 54.37 | D000 | C |
| ATOM | 6859 | CG | ASP | I | 186 | -56.259 | -30.795 | 57.356 | 1.00 | 66.46 | D000 | C |
| ATOM | 6860 | OD1 | ASP | I | 186 | -56.408 | -32.045 | 57.464 | 1.00 | 61.88 | D000 | O |
| ATOM | 6861 | OD2 | ASP | I | 186 | -56.193 | -30.206 | 56.242 | 1.00 | 69.65 | D000 | O1- |
| ATOM | 6862 | N | ALA | I | 187 | -55.318 | -32.197 | 61.505 | 1.00 | 42.36 | D000 | N |
| ATOM | 6863 | CA | ALA | I | 187 | -54.205 | -32.950 | 62.052 | 1.00 | 35.32 | D000 | C |
| ATOM | 6864 | C | ALA | I | 187 | -54.783 | -34.150 | 62.787 | 1.00 | 33.26 | D000 | C |
| ATOM | 6865 | O | ALA | I | 187 | -55.966 | -34.465 | 62.668 | 1.00 | 37.96 | D000 | O |
| ATOM | 6866 | CB | ALA | I | 187 | -53.337 | -32.044 | 62.933 | 1.00 | 32.84 | D000 | C |
| ATOM | 6867 | N | HIS | I | 188 | -53.962 | -34.816 | 63.567 | 1.00 | 30.88 | D000 | N |
| ATOM | 6868 | CA | HIS | I | 188 | -54.456 | -35.987 | 64.253 | 1.00 | 32.32 | D000 | C |
| ATOM | 6869 | C | HIS | I | 188 | -53.593 | -36.192 | 65.488 | 1.00 | 38.94 | D000 | C |
| ATOM | 6870 | O | HIS | I | 188 | -52.452 | -35.723 | 65.547 | 1.00 | 41.15 | D000 | O |
| ATOM | 6871 | CB | HIS | I | 188 | -54.419 | -37.199 | 63.331 | 1.00 | 28.57 | D000 | C |
| ATOM | 6872 | CG | HIS | I | 188 | -53.039 | -37.551 | 62.871 | 1.00 | 39.86 | D000 | C |
| ATOM | 6873 | ND1 | HIS | I | 188 | -52.317 | -38.593 | 63.414 | 1.00 | 43.77 | D000 | N |
| ATOM | 6874 | CD2 | HIS | I | 188 | -52.238 | -36.986 | 61.935 | 1.00 | 40.63 | D000 | C |
| ATOM | 6875 | CE1 | HIS | I | 188 | -51.136 | -38.662 | 62.824 | 1.00 | 43.40 | D000 | C |
| ATOM | 6876 | NE2 | HIS | I | 188 | -51.063 | -37.697 | 61.925 | 1.00 | 44.73 | D000 | N |
| ATOM | 6877 | N | LEU | I | 189 | -54.155 | -36.887 | 66.480 | 1.00 | 34.91 | D000 | N |
| ATOM | 6878 | CA | LEU | I | 189 | -53.389 | -37.240 | 67.667 | 1.00 | 36.57 | D000 | C |
| ATOM | 6879 | C | LEU | I | 189 | -52.134 | -38.003 | 67.269 | 1.00 | 36.22 | D000 | C |
| ATOM | 6880 | O | LEU | I | 189 | -52.181 | -38.892 | 66.414 | 1.00 | 36.87 | D000 | O |
| ATOM | 6881 | CB | LEU | I | 189 | -54.227 | -38.079 | 68.626 | 1.00 | 33.58 | D000 | C |

| | | | | | | | | | | | | |
|---|---|---|---|---|---|---|---|---|---|---|---|---|
| ATOM | 6882 | CG | LEU | I | 189 | -55.332 | -37.326 | 69.361 | 1.00 | 32.53 | D000 | C |
| ATOM | 6883 | CD1 | LEU | I | 189 | -56.060 | -38.310 | 70.251 | 1.00 | 31.98 | D000 | C |
| ATOM | 6884 | CD2 | LEU | I | 189 | -54.785 | -36.156 | 70.127 | 1.00 | 32.11 | D000 | C |
| ATOM | 6885 | N | VAL | I | 190 | -51.006 | -37.639 | 67.889 | 1.00 | 34.36 | D000 | N |
| ATOM | 6886 | CA | VAL | I | 190 | -49.706 | -38.113 | 67.426 | 1.00 | 36.90 | D000 | C |
| ATOM | 6887 | C | VAL | I | 190 | -49.705 | -39.621 | 67.302 | 1.00 | 34.99 | D000 | C |
| ATOM | 6888 | O | VAL | I | 190 | -50.277 | -40.340 | 68.126 | 1.00 | 38.49 | D000 | O |
| ATOM | 6889 | CB | VAL | I | 190 | -48.565 | -37.658 | 68.355 | 1.00 | 40.36 | D000 | C |
| ATOM | 6890 | CG1 | VAL | I | 190 | -48.720 | -38.264 | 69.755 | 1.00 | 36.50 | D000 | C |
| ATOM | 6891 | CG2 | VAL | I | 190 | -47.214 | -38.026 | 67.743 | 1.00 | 36.89 | D000 | C |
| ATOM | 6892 | N | VAL | I | 191 | -49.097 | -40.091 | 66.224 | 1.00 | 38.67 | D000 | N |
| ATOM | 6893 | CA | VAL | I | 191 | -48.933 | -41.503 | 65.926 | 1.00 | 40.35 | D000 | C |
| ATOM | 6894 | C | VAL | I | 191 | -47.436 | -41.758 | 65.901 | 1.00 | 37.87 | D000 | C |
| ATOM | 6895 | O | VAL | I | 191 | -46.719 | -41.196 | 65.064 | 1.00 | 39.61 | D000 | O |
| ATOM | 6896 | CB | VAL | I | 191 | -49.589 | -41.882 | 64.586 | 1.00 | 42.11 | D000 | C |
| ATOM | 6897 | CG1 | VAL | I | 191 | -49.208 | -43.319 | 64.164 | 1.00 | 36.05 | D000 | C |
| ATOM | 6898 | CG2 | VAL | I | 191 | -51.109 | -41.663 | 64.648 | 1.00 | 38.64 | D000 | C |
| ATOM | 6899 | N | VAL | I | 192 | -46.969 | -42.599 | 66.805 | 1.00 | 36.01 | D000 | N |
| ATOM | 6900 | CA | VAL | I | 192 | -45.545 | -42.767 | 67.055 | 1.00 | 41.63 | D000 | C |
| ATOM | 6901 | C | VAL | I | 192 | -45.088 | -44.039 | 66.361 | 1.00 | 37.03 | D000 | C |
| ATOM | 6902 | O | VAL | I | 192 | -45.528 | -45.138 | 66.718 | 1.00 | 40.05 | D000 | O |
| ATOM | 6903 | CB | VAL | I | 192 | -45.264 | -42.808 | 68.565 | 1.00 | 40.06 | D000 | C |
| ATOM | 6904 | CG1 | VAL | I | 192 | -43.805 | -43.021 | 68.828 | 1.00 | 43.89 | D000 | C |
| ATOM | 6905 | CG2 | VAL | I | 192 | -45.723 | -41.516 | 69.199 | 1.00 | 30.23 | D000 | C |
| ATOM | 6906 | N | THR | I | 193 | -44.188 | -43.897 | 65.377 | 1.00 | 40.22 | D000 | N |
| ATOM | 6907 | CA | THR | I | 193 | -43.828 | -45.009 | 64.496 | 1.00 | 46.20 | D000 | C |
| ATOM | 6908 | C | THR | I | 193 | -42.371 | -45.451 | 64.595 | 1.00 | 48.77 | D000 | C |
| ATOM | 6909 | O | THR | I | 193 | -42.009 | -46.439 | 63.948 | 1.00 | 50.27 | D000 | O |
| ATOM | 6910 | CB | THR | I | 193 | -44.081 | -44.649 | 63.021 | 1.00 | 37.88 | D000 | C |
| ATOM | 6911 | OG1 | THR | I | 193 | -43.196 | -43.588 | 62.655 | 1.00 | 46.69 | D000 | O |
| ATOM | 6912 | CG2 | THR | I | 193 | -45.516 | -44.167 | 62.808 | 1.00 | 35.86 | D000 | C |
| ATOM | 6913 | N | SER | I | 194 | -41.529 | -44.758 | 65.364 | 1.00 | 47.04 | D000 | N |
| ATOM | 6914 | CA | SER | I | 194 | -40.107 | -45.072 | 65.443 | 1.00 | 47.61 | D000 | C |
| ATOM | 6915 | C | SER | I | 194 | -39.578 | -44.648 | 66.802 | 1.00 | 47.34 | D000 | C |
| ATOM | 6916 | O | SER | I | 194 | -40.228 | -43.898 | 67.531 | 1.00 | 48.44 | D000 | O |
| ATOM | 6917 | CB | SER | I | 194 | -39.292 | -44.369 | 64.357 | 1.00 | 44.10 | D000 | C |
| ATOM | 6918 | OG | SER | I | 194 | -39.457 | -42.964 | 64.442 | 1.00 | 48.66 | D000 | O |
| ATOM | 6919 | N | TRP | I | 195 | -38.388 | -45.147 | 67.143 | 1.00 | 44.89 | D000 | N |
| ATOM | 6920 | CA | TRP | I | 195 | -37.764 | -44.738 | 68.392 | 1.00 | 43.95 | D000 | C |
| ATOM | 6921 | C | TRP | I | 195 | -37.376 | -43.274 | 68.361 | 1.00 | 46.62 | D000 | C |
| ATOM | 6922 | O | TRP | I | 195 | -37.488 | -42.577 | 69.379 | 1.00 | 43.24 | D000 | O |
| ATOM | 6923 | CB | TRP | I | 195 | -36.552 | -45.605 | 68.717 | 1.00 | 48.92 | D000 | C |
| ATOM | 6924 | CG | TRP | I | 195 | -36.802 | -46.281 | 70.004 | 1.00 | 55.85 | D000 | C |
| ATOM | 6925 | CD1 | TRP | I | 195 | -37.056 | -45.676 | 71.212 | 1.00 | 56.28 | D000 | C |
| ATOM | 6926 | CD2 | TRP | I | 195 | -36.901 | -47.690 | 70.230 | 1.00 | 56.55 | D000 | C |
| ATOM | 6927 | NE1 | TRP | I | 195 | -37.292 | -46.630 | 72.179 | 1.00 | 58.55 | D000 | N |
| ATOM | 6928 | CE2 | TRP | I | 195 | -37.199 | -47.874 | 71.605 | 1.00 | 65.39 | D000 | C |
| ATOM | 6929 | CE3 | TRP | I | 195 | -36.755 | -48.815 | 69.413 | 1.00 | 48.79 | D000 | C |
| ATOM | 6930 | CZ2 | TRP | I | 195 | -37.353 | -49.140 | 72.174 | 1.00 | 63.33 | D000 | C |
| ATOM | 6931 | CZ3 | TRP | I | 195 | -36.904 | -50.070 | 69.980 | 1.00 | 61.76 | D000 | C |
| ATOM | 6932 | CH2 | TRP | I | 195 | -37.198 | -50.223 | 71.349 | 1.00 | 69.03 | D000 | C |
| ATOM | 6933 | N | GLU | I | 196 | -36.914 | -42.786 | 67.210 | 1.00 | 43.07 | D000 | N |
| ATOM | 6934 | CA | GLU | I | 196 | -36.579 | -41.374 | 67.119 | 1.00 | 38.64 | D000 | C |
| ATOM | 6935 | C | GLU | I | 196 | -37.818 | -40.526 | 67.331 | 1.00 | 38.66 | D000 | C |
| ATOM | 6936 | O | GLU | I | 196 | -37.782 | -39.540 | 68.074 | 1.00 | 41.67 | D000 | O |
| ATOM | 6937 | CB | GLU | I | 196 | -35.919 | -41.061 | 65.783 | 1.00 | 37.74 | D000 | C |
| ATOM | 6938 | CG | GLU | I | 196 | -36.000 | -42.187 | 64.776 | 1.00 | 52.87 | D000 | C |
| ATOM | 6939 | CD | GLU | I | 196 | -35.141 | -43.397 | 65.156 | 1.00 | 66.98 | D000 | C |
| ATOM | 6940 | OE1 | GLU | I | 196 | -35.717 | -44.504 | 65.378 | 1.00 | 59.55 | D000 | O |
| ATOM | 6941 | OE2 | GLU | I | 196 | -33.899 | -43.224 | 65.260 | 1.00 | 74.05 | D000 | O1- |
| ATOM | 6942 | N | GLU | I | 197 | -38.945 | -40.923 | 66.737 | 1.00 | 39.39 | D000 | N |
| ATOM | 6943 | CA | GLU | I | 197 | -40.168 | -40.155 | 66.948 | 1.00 | 42.64 | D000 | C |
| ATOM | 6944 | C | GLU | I | 197 | -40.599 | -40.215 | 68.410 | 1.00 | 37.64 | D000 | C |
| ATOM | 6945 | O | GLU | I | 197 | -41.014 | -39.205 | 68.981 | 1.00 | 32.92 | D000 | O |
| ATOM | 6946 | CB | GLU | I | 197 | -41.283 | -40.664 | 66.038 | 1.00 | 40.56 | D000 | C |
| ATOM | 6947 | CG | GLU | I | 197 | -42.482 | -39.748 | 66.042 | 1.00 | 37.27 | D000 | C |
| ATOM | 6948 | CD | GLU | I | 197 | -43.515 | -40.096 | 64.990 | 1.00 | 42.36 | D000 | C |
| ATOM | 6949 | OE1 | GLU | I | 197 | -43.544 | -41.263 | 64.528 | 1.00 | 39.06 | D000 | O |
| ATOM | 6950 | OE2 | GLU | I | 197 | -44.277 | -39.173 | 64.601 | 1.00 | 44.17 | D000 | O1- |
| ATOM | 6951 | N | GLN | I | 198 | -40.485 | -41.396 | 69.026 | 1.00 | 37.19 | D000 | N |
| ATOM | 6952 | CA | GLN | I | 198 | -40.810 | -41.562 | 70.435 | 1.00 | 33.73 | D000 | C |

| | | | | | | | | | | | | |
|---|---|---|---|---|---|---|---|---|---|---|---|---|
| ATOM | 6953 | C | GLN | I | 198 | -39.920 | -40.689 | 71.301 | 1.00 | 39.61 | D000 | C |
| ATOM | 6954 | O | GLN | I | 198 | -40.401 | -39.995 | 72.206 | 1.00 | 35.04 | D000 | O |
| ATOM | 6955 | CB | GLN | I | 198 | -40.659 | -43.026 | 70.832 | 1.00 | 32.87 | D000 | C |
| ATOM | 6956 | CG | GLN | I | 198 | -40.502 | -43.235 | 72.323 | 1.00 | 37.28 | D000 | C |
| ATOM | 6957 | CD | GLN | I | 198 | -41.830 | -43.123 | 73.085 | 1.00 | 35.70 | D000 | C |
| ATOM | 6958 | OE1 | GLN | I | 198 | -42.902 | -43.371 | 72.539 | 1.00 | 30.32 | D000 | O |
| ATOM | 6959 | NE2 | GLN | I | 198 | -41.749 | -42.729 | 74.345 | 1.00 | 35.65 | D000 | N |
| ATOM | 6960 | N | LYS | I | 199 | -38.610 | -40.700 | 71.014 | 1.00 | 44.77 | D000 | N |
| ATOM | 6961 | CA | LYS | I | 199 | -37.666 | -39.882 | 71.765 | 1.00 | 39.06 | D000 | C |
| ATOM | 6962 | C | LYS | I | 199 | -37.934 | -38.405 | 71.551 | 1.00 | 32.39 | D000 | C |
| ATOM | 6963 | O | LYS | I | 199 | -37.949 | -37.629 | 72.509 | 1.00 | 31.60 | D000 | O |
| ATOM | 6964 | CB | LYS | I | 199 | -36.232 | -40.241 | 71.380 | 1.00 | 36.82 | D000 | C |
| ATOM | 6965 | CG | LYS | I | 199 | -35.661 | -41.318 | 72.265 | 1.00 | 38.84 | D000 | C |
| ATOM | 6966 | CD | LYS | I | 199 | -34.660 | -42.196 | 71.578 | 1.00 | 46.27 | D000 | C |
| ATOM | 6967 | CE | LYS | I | 199 | -33.601 | -41.398 | 70.901 | 1.00 | 41.73 | D000 | C |
| ATOM | 6968 | NZ | LYS | I | 199 | -32.530 | -42.355 | 70.568 | 1.00 | 49.72 | D000 | N1+ |
| ATOM | 6969 | N | PHE | I | 200 | -38.200 | -38.011 | 70.309 | 1.00 | 34.30 | D000 | N |
| ATOM | 6970 | CA | PHE | I | 200 | -38.496 | -36.613 | 70.030 | 1.00 | 32.36 | D000 | C |
| ATOM | 6971 | C | PHE | I | 200 | -39.712 | -36.127 | 70.813 | 1.00 | 34.19 | D000 | C |
| ATOM | 6972 | O | PHE | I | 200 | -39.724 | -34.995 | 71.313 | 1.00 | 32.22 | D000 | O |
| ATOM | 6973 | CB | PHE | I | 200 | -38.719 | -36.408 | 68.539 | 1.00 | 28.30 | D000 | C |
| ATOM | 6974 | CG | PHE | I | 200 | -39.389 | -35.123 | 68.221 | 1.00 | 29.58 | D000 | C |
| ATOM | 6975 | CD1 | PHE | I | 200 | -38.661 | -33.950 | 68.167 | 1.00 | 34.42 | D000 | C |
| ATOM | 6976 | CD2 | PHE | I | 200 | -40.750 | -35.070 | 67.988 | 1.00 | 32.89 | D000 | C |
| ATOM | 6977 | CE1 | PHE | I | 200 | -39.278 | -32.727 | 67.878 | 1.00 | 36.14 | D000 | C |
| ATOM | 6978 | CE2 | PHE | I | 200 | -41.373 | -33.852 | 67.708 | 1.00 | 32.23 | D000 | C |
| ATOM | 6979 | CZ | PHE | I | 200 | -40.628 | -32.679 | 67.656 | 1.00 | 32.52 | D000 | C |
| ATOM | 6980 | N | VAL | I | 201 | -40.766 | -36.949 | 70.896 | 1.00 | 34.21 | D000 | N |
| ATOM | 6981 | CA | VAL | I | 201 | -41.963 | -36.512 | 71.604 | 1.00 | 32.09 | D000 | C |
| ATOM | 6982 | C | VAL | I | 201 | -41.691 | -36.409 | 73.104 | 1.00 | 28.47 | D000 | C |
| ATOM | 6983 | O | VAL | I | 201 | -42.112 | -35.445 | 73.745 | 1.00 | 29.67 | D000 | O |
| ATOM | 6984 | CB | VAL | I | 201 | -43.166 | -37.423 | 71.280 | 1.00 | 33.84 | D000 | C |
| ATOM | 6985 | CG1 | VAL | I | 201 | -44.371 | -37.069 | 72.171 | 1.00 | 26.65 | D000 | C |
| ATOM | 6986 | CG2 | VAL | I | 201 | -43.564 | -37.259 | 69.826 | 1.00 | 29.93 | D000 | C |
| ATOM | 6987 | N | GLN | I | 202 | -40.948 | -37.368 | 73.675 | 1.00 | 29.76 | D000 | N |
| ATOM | 6988 | CA | GLN | I | 202 | -40.625 | -37.333 | 75.103 | 1.00 | 27.89 | D000 | C |
| ATOM | 6989 | C | GLN | I | 202 | -39.957 | -36.027 | 75.500 | 1.00 | 30.88 | D000 | C |
| ATOM | 6990 | O | GLN | I | 202 | -40.371 | -35.375 | 76.462 | 1.00 | 33.51 | D000 | O |
| ATOM | 6991 | CB | GLN | I | 202 | -39.692 | -38.471 | 75.456 | 1.00 | 36.89 | D000 | C |
| ATOM | 6992 | CG | GLN | I | 202 | -40.291 | -39.826 | 75.569 | 1.00 | 42.55 | D000 | C |
| ATOM | 6993 | CD | GLN | I | 202 | -39.269 | -40.793 | 76.129 | 1.00 | 48.57 | D000 | C |
| ATOM | 6994 | OE1 | GLN | I | 202 | -39.196 | -41.954 | 75.707 | 1.00 | 49.10 | D000 | O |
| ATOM | 6995 | NE2 | GLN | I | 202 | -38.440 | -40.305 | 77.064 | 1.00 | 45.91 | D000 | N |
| ATOM | 6996 | N | HIS | I | 203 | -38.934 | -35.620 | 74.748 | 1.00 | 28.33 | D000 | N |
| ATOM | 6997 | CA | HIS | I | 203 | -38.244 | -34.367 | 74.998 | 1.00 | 28.20 | D000 | C |
| ATOM | 6998 | C | HIS | I | 203 | -39.221 | -33.223 | 75.160 | 1.00 | 32.96 | D000 | C |
| ATOM | 6999 | O | HIS | I | 203 | -39.085 | -32.395 | 76.066 | 1.00 | 38.66 | D000 | O |
| ATOM | 7000 | CB | HIS | I | 203 | -37.308 | -34.053 | 73.837 | 1.00 | 38.51 | D000 | C |
| ATOM | 7001 | CG | HIS | I | 203 | -35.942 | -34.622 | 73.991 | 1.00 | 45.00 | D000 | C |
| ATOM | 7002 | ND1 | HIS | I | 203 | -35.086 | -34.222 | 74.994 | 1.00 | 43.86 | D000 | N |
| ATOM | 7003 | CD2 | HIS | I | 203 | -35.274 | -35.544 | 73.260 | 1.00 | 44.53 | D000 | C |
| ATOM | 7004 | CE1 | HIS | I | 203 | -33.951 | -34.887 | 74.882 | 1.00 | 48.63 | D000 | C |
| ATOM | 7005 | NE2 | HIS | I | 203 | -34.043 | -35.701 | 73.843 | 1.00 | 52.83 | D000 | N |
| ATOM | 7006 | N | HIS | I | 204 | -40.192 | -33.140 | 74.253 | 1.00 | 33.22 | D000 | N |
| ATOM | 7007 | CA | HIS | I | 204 | -41.092 | -32.000 | 74.229 | 1.00 | 33.76 | D000 | C |
| ATOM | 7008 | C | HIS | I | 204 | -42.207 | -32.107 | 75.257 | 1.00 | 30.31 | D000 | C |
| ATOM | 7009 | O | HIS | I | 204 | -42.651 | -31.076 | 75.766 | 1.00 | 32.42 | D000 | O |
| ATOM | 7010 | CB | HIS | I | 204 | -41.680 | -31.825 | 72.836 | 1.00 | 34.09 | D000 | C |
| ATOM | 7011 | CG | HIS | I | 204 | -40.730 | -31.209 | 71.872 | 1.00 | 33.98 | D000 | C |
| ATOM | 7012 | ND1 | HIS | I | 204 | -40.541 | -29.847 | 71.793 | 1.00 | 36.56 | D000 | N |
| ATOM | 7013 | CD2 | HIS | I | 204 | -39.894 | -31.764 | 70.964 | 1.00 | 37.43 | D000 | C |
| ATOM | 7014 | CE1 | HIS | I | 204 | -39.636 | -29.587 | 70.865 | 1.00 | 42.50 | D000 | C |
| ATOM | 7015 | NE2 | HIS | I | 204 | -39.224 | -30.732 | 70.349 | 1.00 | 42.23 | D000 | N |
| ATOM | 7016 | N | ILE | I | 205 | -42.658 | -33.313 | 75.609 | 1.00 | 27.51 | D000 | N |
| ATOM | 7017 | CA | ILE | I | 205 | -43.735 | -33.386 | 76.587 | 1.00 | 30.86 | D000 | C |
| ATOM | 7018 | C | ILE | I | 205 | -43.220 | -33.433 | 78.017 | 1.00 | 29.89 | D000 | C |
| ATOM | 7019 | O | ILE | I | 205 | -43.958 | -33.056 | 78.934 | 1.00 | 35.65 | D000 | O |
| ATOM | 7020 | CB | ILE | I | 205 | -44.686 | -34.591 | 76.395 | 1.00 | 31.24 | D000 | C |
| ATOM | 7021 | CG1 | ILE | I | 205 | -43.981 | -35.940 | 76.592 | 1.00 | 26.46 | D000 | C |
| ATOM | 7022 | CG2 | ILE | I | 205 | -45.357 | -34.529 | 75.064 | 1.00 | 28.37 | D000 | C |
| ATOM | 7023 | CD1 | ILE | I | 205 | -44.943 | -37.086 | 76.633 | 1.00 | 25.80 | D000 | C |

| | | | | | | | | | | | | |
|---|---|---|---|---|---|---|---|---|---|---|---|---|
| ATOM | 7024 | N | GLY | I | 206 | -41.978 | -33.852 | 78.238 | 1.00 | 28.95 | D000 | N |
| ATOM | 7025 | CA | GLY | I | 206 | -41.465 | -33.985 | 79.584 | 1.00 | 29.61 | D000 | C |
| ATOM | 7026 | C | GLY | I | 206 | -42.122 | -35.118 | 80.345 | 1.00 | 27.57 | D000 | C |
| ATOM | 7027 | O | GLY | I | 206 | -42.811 | -35.956 | 79.766 | 1.00 | 29.14 | D000 | O |
| ATOM | 7028 | N | PRO | I | 207 | -41.941 | -35.153 | 81.711 | 1.00 | 29.38 | D000 | N |
| ATOM | 7029 | CA | PRO | I | 207 | -42.433 | -36.295 | 82.509 | 1.00 | 32.33 | D000 | C |
| ATOM | 7030 | C | PRO | I | 207 | -43.904 | -36.143 | 82.896 | 1.00 | 35.00 | D000 | C |
| ATOM | 7031 | O | PRO | I | 207 | -44.262 | -36.029 | 84.077 | 1.00 | 27.86 | D000 | O |
| ATOM | 7032 | CB | PRO | I | 207 | -41.492 | -36.271 | 83.719 | 1.00 | 29.81 | D000 | C |
| ATOM | 7033 | CG | PRO | I | 207 | -41.194 | -34.812 | 83.899 | 1.00 | 30.51 | D000 | C |
| ATOM | 7034 | CD | PRO | I | 207 | -41.260 | -34.150 | 82.549 | 1.00 | 24.29 | D000 | C |
| ATOM | 7035 | N | VAL | I | 208 | -44.771 | -36.126 | 81.879 | 1.00 | 31.78 | D000 | N |
| ATOM | 7036 | CA | VAL | I | 208 | -46.167 | -35.726 | 81.999 | 1.00 | 26.40 | D000 | C |
| ATOM | 7037 | C | VAL | I | 208 | -47.040 | -36.722 | 81.258 | 1.00 | 27.82 | D000 | C |
| ATOM | 7038 | O | VAL | I | 208 | -46.764 | -37.034 | 80.098 | 1.00 | 30.68 | D000 | O |
| ATOM | 7039 | CB | VAL | I | 208 | -46.388 | -34.323 | 81.425 | 1.00 | 25.92 | D000 | C |
| ATOM | 7040 | CG1 | VAL | I | 208 | -47.832 | -33.981 | 81.495 | 1.00 | 28.17 | D000 | C |
| ATOM | 7041 | CG2 | VAL | I | 208 | -45.544 | -33.320 | 82.172 | 1.00 | 26.96 | D000 | C |
| ATOM | 7042 | N | ASN | I | 209 | -48.080 | -37.232 | 81.927 | 1.00 | 27.70 | D000 | N |
| ATOM | 7043 | CA | ASN | I | 209 | -49.054 | -38.093 | 81.264 | 1.00 | 25.54 | D000 | C |
| ATOM | 7044 | C | ASN | I | 209 | -49.688 | -37.350 | 80.096 | 1.00 | 29.53 | D000 | C |
| ATOM | 7045 | O | ASN | I | 209 | -50.263 | -36.271 | 80.277 | 1.00 | 30.75 | D000 | O |
| ATOM | 7046 | CB | ASN | I | 209 | -50.119 | -38.560 | 82.251 | 1.00 | 24.84 | D000 | C |
| ATOM | 7047 | CG | ASN | I | 209 | -49.588 | -39.606 | 83.223 | 1.00 | 31.55 | D000 | C |
| ATOM | 7048 | OD1 | ASN | I | 209 | -48.636 | -40.343 | 82.922 | 1.00 | 29.18 | D000 | O |
| ATOM | 7049 | ND2 | ASN | I | 209 | -50.193 | -39.667 | 84.407 | 1.00 | 32.11 | D000 | N |
| ATOM | 7050 | N | THR | I | 210 | -49.543 | -37.905 | 78.889 | 1.00 | 28.06 | D000 | N |
| ATOM | 7051 | CA | THR | I | 210 | -49.900 | -37.194 | 77.666 | 1.00 | 29.54 | D000 | C |
| ATOM | 7052 | C | THR | I | 210 | -50.593 | -38.164 | 76.716 | 1.00 | 30.18 | D000 | C |
| ATOM | 7053 | O | THR | I | 210 | -50.080 | -39.257 | 76.474 | 1.00 | 31.78 | D000 | O |
| ATOM | 7054 | CB | THR | I | 210 | -48.635 | -36.568 | 77.017 | 1.00 | 32.46 | D000 | C |
| ATOM | 7055 | OG1 | THR | I | 210 | -48.068 | -35.576 | 77.891 | 1.00 | 28.52 | D000 | O |
| ATOM | 7056 | CG2 | THR | I | 210 | -48.955 | -35.895 | 75.680 | 1.00 | 32.03 | D000 | C |
| ATOM | 7057 | N | TRP | I | 211 | -51.748 | -37.771 | 76.178 | 1.00 | 28.21 | D000 | N |
| ATOM | 7058 | CA | TRP | I | 211 | -52.484 | -38.637 | 75.257 | 1.00 | 30.26 | D000 | C |
| ATOM | 7059 | C | TRP | I | 211 | -51.777 | -38.768 | 73.915 | 1.00 | 34.53 | D000 | C |
| ATOM | 7060 | O | TRP | I | 211 | -51.243 | -37.784 | 73.388 | 1.00 | 36.53 | D000 | O |
| ATOM | 7061 | CB | TRP | I | 211 | -53.870 | -38.078 | 74.986 | 1.00 | 27.00 | D000 | C |
| ATOM | 7062 | CG | TRP | I | 211 | -54.837 | -38.105 | 76.098 | 1.00 | 29.16 | D000 | C |
| ATOM | 7063 | CD1 | TRP | I | 211 | -55.575 | -37.051 | 76.552 | 1.00 | 29.17 | D000 | C |
| ATOM | 7064 | CD2 | TRP | I | 211 | -55.232 | -39.235 | 76.871 | 1.00 | 27.40 | D000 | C |
| ATOM | 7065 | NE1 | TRP | I | 211 | -56.401 | -37.453 | 77.547 | 1.00 | 29.33 | D000 | N |
| ATOM | 7066 | CE2 | TRP | I | 211 | -56.208 | -38.791 | 77.777 | 1.00 | 31.18 | D000 | C |
| ATOM | 7067 | CE3 | TRP | I | 211 | -54.849 | -40.575 | 76.896 | 1.00 | 30.57 | D000 | C |
| ATOM | 7068 | CZ2 | TRP | I | 211 | -56.804 | -39.643 | 78.721 | 1.00 | 33.48 | D000 | C |
| ATOM | 7069 | CZ3 | TRP | I | 211 | -55.442 | -41.420 | 77.828 | 1.00 | 32.75 | D000 | C |
| ATOM | 7070 | CH2 | TRP | I | 211 | -56.414 | -40.952 | 78.721 | 1.00 | 31.65 | D000 | C |
| ATOM | 7071 | N | MET | I | 212 | -51.828 | -39.969 | 73.332 | 1.00 | 30.71 | D000 | N |
| ATOM | 7072 | CA | MET | I | 212 | -51.425 | -40.202 | 71.945 | 1.00 | 35.06 | D000 | C |
| ATOM | 7073 | C | MET | I | 212 | -52.588 | -40.816 | 71.166 | 1.00 | 34.50 | D000 | C |
| ATOM | 7074 | O | MET | I | 212 | -53.536 | -41.339 | 71.746 | 1.00 | 29.90 | D000 | O |
| ATOM | 7075 | CB | MET | I | 212 | -50.197 | -41.126 | 71.870 | 1.00 | 33.98 | D000 | C |
| ATOM | 7076 | CG | MET | I | 212 | -50.532 | -42.612 | 72.009 | 1.00 | 34.65 | D000 | C |
| ATOM | 7077 | SD | MET | I | 212 | -49.112 | -43.701 | 72.320 | 1.00 | 36.95 | D000 | S |
| ATOM | 7078 | CE | MET | I | 212 | -48.665 | -43.185 | 73.977 | 1.00 | 32.76 | D000 | C |
| ATOM | 7079 | N | GLY | I | 213 | -52.482 | -40.824 | 69.836 | 1.00 | 31.69 | D000 | N |
| ATOM | 7080 | CA | GLY | I | 213 | -53.575 | -41.340 | 69.027 | 1.00 | 34.66 | D000 | C |
| ATOM | 7081 | C | GLY | I | 213 | -53.764 | -42.849 | 68.974 | 1.00 | 32.70 | D000 | C |
| ATOM | 7082 | O | GLY | I | 213 | -53.862 | -43.424 | 67.900 | 1.00 | 36.23 | D000 | O |
| ATOM | 7083 | N | LEU | I | 214 | -53.871 | -43.509 | 70.116 | 1.00 | 34.81 | D000 | N |
| ATOM | 7084 | CA | LEU | I | 214 | -53.951 | -44.961 | 70.150 | 1.00 | 39.32 | D000 | C |
| ATOM | 7085 | C | LEU | I | 214 | -55.046 | -45.365 | 71.132 | 1.00 | 45.03 | D000 | C |
| ATOM | 7086 | O | LEU | I | 214 | -54.950 | -45.073 | 72.327 | 1.00 | 45.18 | D000 | O |
| ATOM | 7087 | CB | LEU | I | 214 | -52.589 | -45.544 | 70.557 | 1.00 | 39.33 | D000 | C |
| ATOM | 7088 | CG | LEU | I | 214 | -52.388 | -47.049 | 70.722 | 1.00 | 44.57 | D000 | C |
| ATOM | 7089 | CD1 | LEU | I | 214 | -52.448 | -47.717 | 69.369 | 1.00 | 42.58 | D000 | C |
| ATOM | 7090 | CD2 | LEU | I | 214 | -51.059 | -47.335 | 71.406 | 1.00 | 38.76 | D000 | C |
| ATOM | 7091 | N | HIS | I | 215 | -56.076 | -46.053 | 70.639 | 1.00 | 50.99 | D000 | N |
| ATOM | 7092 | CA | HIS | I | 215 | -57.202 | -46.449 | 71.479 | 1.00 | 54.24 | D000 | C |
| ATOM | 7093 | C | HIS | I | 215 | -57.809 | -47.741 | 70.957 | 1.00 | 53.49 | D000 | C |
| ATOM | 7094 | O | HIS | I | 215 | -57.726 | -48.039 | 69.766 | 1.00 | 59.77 | D000 | O |

| | | | | | | | | | | | |
|---|---|---|---|---|---|---|---|---|---|---|---|
| ATOM | 7095 | CB | HIS | I | 215 | -58.297 | -45.381 | 71.525 | 1.00 | 51.69 | D000 C |
| ATOM | 7096 | CG | HIS | I | 215 | -58.991 | -45.169 | 70.220 | 1.00 | 53.16 | D000 C |
| ATOM | 7097 | ND1 | HIS | I | 215 | -60.319 | -44.814 | 70.139 | 1.00 | 61.14 | D000 N |
| ATOM | 7098 | CD2 | HIS | I | 215 | -58.542 | -45.241 | 68.946 | 1.00 | 52.42 | D000 C |
| ATOM | 7099 | CE1 | HIS | I | 215 | -60.664 | -44.692 | 68.868 | 1.00 | 60.68 | D000 C |
| ATOM | 7100 | NE2 | HIS | I | 215 | -59.602 | -44.938 | 68.124 | 1.00 | 56.57 | D000 N |
| ATOM | 7101 | N | ASP | I | 216 | -58.446 | -48.489 | 71.850 | 1.00 | 51.53 | D000 N |
| ATOM | 7102 | CA | ASP | I | 216 | -59.163 | -49.708 | 71.488 | 1.00 | 63.11 | D000 C |
| ATOM | 7103 | C | ASP | I | 216 | -60.629 | -49.629 | 71.897 | 1.00 | 70.32 | D000 C |
| ATOM | 7104 | O | ASP | I | 216 | -61.218 | -50.615 | 72.345 | 1.00 | 73.44 | D000 O |
| ATOM | 7105 | CB | ASP | I | 216 | -58.504 | -50.956 | 72.083 | 1.00 | 62.05 | D000 C |
| ATOM | 7106 | CG | ASP | I | 216 | -58.816 | -51.157 | 73.565 | 1.00 | 63.11 | D000 C |
| ATOM | 7107 | OD1 | ASP | I | 216 | -59.134 | -50.177 | 74.265 | 1.00 | 66.02 | D000 O |
| ATOM | 7108 | OD2 | ASP | I | 216 | -58.783 | -52.313 | 74.025 | 1.00 | 71.37 | D000 O1- |
| ATOM | 7109 | N | GLN | I | 217 | -61.243 | -48.448 | 71.761 | 1.00 | 68.81 | D000 N |
| ATOM | 7110 | CA | GLN | I | 217 | -62.674 | -48.330 | 72.036 | 1.00 | 79.13 | D000 C |
| ATOM | 7111 | C | GLN | I | 217 | -63.497 | -49.323 | 71.214 | 1.00 | 82.76 | D000 C |
| ATOM | 7112 | O | GLN | I | 217 | -64.637 | -49.629 | 71.584 | 1.00 | 81.10 | D000 O |
| ATOM | 7113 | CB | GLN | I | 217 | -63.136 | -46.885 | 71.804 | 1.00 | 78.37 | D000 C |
| ATOM | 7114 | CG | GLN | I | 217 | -63.047 | -46.020 | 73.089 | 1.00 | 75.77 | D000 C |
| ATOM | 7115 | CD | GLN | I | 217 | -62.642 | -44.570 | 72.825 | 1.00 | 67.80 | D000 C |
| ATOM | 7116 | OE1 | GLN | I | 217 | -61.796 | -44.296 | 71.969 | 1.00 | 62.96 | D000 O |
| ATOM | 7117 | NE2 | GLN | I | 217 | -63.220 | -43.637 | 73.591 | 1.00 | 56.15 | D000 N |
| ATOM | 7118 | N | ASN | I | 218 | -62.934 | -49.838 | 70.118 | 1.00 | 81.51 | D000 N |
| ATOM | 7119 | CA | ASN | I | 218 | -63.546 | -50.918 | 69.346 | 1.00 | 86.93 | D000 C |
| ATOM | 7120 | C | ASN | I | 218 | -63.369 | -52.279 | 70.037 | 1.00 | 84.87 | D000 C |
| ATOM | 7121 | O | ASN | I | 218 | -64.349 | -52.951 | 70.386 | 1.00 | 79.46 | D000 O |
| ATOM | 7122 | CB | ASN | I | 218 | -62.928 | -50.932 | 67.942 | 1.00 | 92.18 | D000 C |
| ATOM | 7123 | CG | ASN | I | 218 | -63.905 | -51.382 | 66.867 | 1.00 | 99.76 | D000 C |
| ATOM | 7124 | OD1 | ASN | I | 218 | -63.626 | -51.249 | 65.669 | 1.00 | 98.33 | D000 O |
| ATOM | 7125 | ND2 | ASN | I | 218 | -65.059 | -51.902 | 67.285 | 1.00 | 97.53 | D000 N |
| ATOM | 7126 | N | GLY | I | 219 | -62.117 | -52.679 | 70.279 | 1.00 | 81.16 | D000 N |
| ATOM | 7127 | CA | GLY | I | 219 | -61.786 | -54.004 | 70.763 | 1.00 | 72.74 | D000 C |
| ATOM | 7128 | C | GLY | I | 219 | -60.286 | -54.248 | 70.697 | 1.00 | 71.27 | D000 C |
| ATOM | 7129 | O | GLY | I | 219 | -59.631 | -54.504 | 71.715 | 1.00 | 64.68 | D000 O |
| ATOM | 7130 | N | PRO | I | 220 | -59.712 | -54.165 | 69.493 | 1.00 | 71.97 | D000 N |
| ATOM | 7131 | CA | PRO | I | 220 | -58.254 | -54.149 | 69.354 | 1.00 | 66.99 | D000 C |
| ATOM | 7132 | C | PRO | I | 220 | -57.718 | -52.723 | 69.288 | 1.00 | 65.66 | D000 C |
| ATOM | 7133 | O | PRO | I | 220 | -58.435 | -51.765 | 68.980 | 1.00 | 63.20 | D000 O |
| ATOM | 7134 | CB | PRO | I | 220 | -58.025 | -54.861 | 68.014 | 1.00 | 60.23 | D000 C |
| ATOM | 7135 | CG | PRO | I | 220 | -59.311 | -54.670 | 67.242 | 1.00 | 61.26 | D000 C |
| ATOM | 7136 | CD | PRO | I | 220 | -60.369 | -54.114 | 68.172 | 1.00 | 66.08 | D000 C |
| ATOM | 7137 | N | TRP | I | 221 | -56.429 | -52.596 | 69.586 | 1.00 | 55.14 | D000 N |
| ATOM | 7138 | CA | TRP | I | 221 | -55.793 | -51.292 | 69.545 | 1.00 | 47.35 | D000 C |
| ATOM | 7139 | C | TRP | I | 221 | -55.622 | -50.832 | 68.103 | 1.00 | 49.66 | D000 C |
| ATOM | 7140 | O | TRP | I | 221 | -55.267 | -51.614 | 67.218 | 1.00 | 47.87 | D000 O |
| ATOM | 7141 | CB | TRP | I | 221 | -54.450 | -51.345 | 70.262 | 1.00 | 52.11 | D000 C |
| ATOM | 7142 | CG | TRP | I | 221 | -54.577 | -51.541 | 71.768 | 1.00 | 58.06 | D000 C |
| ATOM | 7143 | CD1 | TRP | I | 221 | -54.489 | -52.721 | 72.456 | 1.00 | 56.76 | D000 C |
| ATOM | 7144 | CD2 | TRP | I | 221 | -54.817 | -50.524 | 72.754 | 1.00 | 56.97 | D000 C |
| ATOM | 7145 | NE1 | TRP | I | 221 | -54.650 | -52.500 | 73.800 | 1.00 | 58.53 | D000 N |
| ATOM | 7146 | CE2 | TRP | I | 221 | -54.860 | -51.163 | 74.012 | 1.00 | 57.57 | D000 C |
| ATOM | 7147 | CE3 | TRP | I | 221 | -54.999 | -49.136 | 72.695 | 1.00 | 53.29 | D000 C |
| ATOM | 7148 | CZ2 | TRP | I | 221 | -55.078 | -50.464 | 75.201 | 1.00 | 53.89 | D000 C |
| ATOM | 7149 | CZ3 | TRP | I | 221 | -55.211 | -48.447 | 73.874 | 1.00 | 50.92 | D000 C |
| ATOM | 7150 | CH2 | TRP | I | 221 | -55.251 | -49.113 | 75.110 | 1.00 | 52.06 | D000 C |
| ATOM | 7151 | N | LYS | I | 222 | -55.888 | -49.550 | 67.870 | 1.00 | 47.59 | D000 N |
| ATOM | 7152 | CA | LYS | I | 222 | -55.833 | -48.951 | 66.546 | 1.00 | 47.00 | D000 C |
| ATOM | 7153 | C | LYS | I | 222 | -55.218 | -47.565 | 66.654 | 1.00 | 47.01 | D000 C |
| ATOM | 7154 | O | LYS | I | 222 | -55.421 | -46.869 | 67.650 | 1.00 | 48.52 | D000 O |
| ATOM | 7155 | CB | LYS | I | 222 | -57.240 | -48.830 | 65.933 | 1.00 | 47.34 | D000 C |
| ATOM | 7156 | CG | LYS | I | 222 | -58.076 | -50.090 | 66.018 | 1.00 | 46.82 | D000 C |
| ATOM | 7157 | CD | LYS | I | 222 | -59.483 | -49.822 | 65.523 | 1.00 | 66.09 | D000 C |
| ATOM | 7158 | CE | LYS | I | 222 | -60.297 | -51.108 | 65.437 | 1.00 | 78.89 | D000 C |
| ATOM | 7159 | NZ | LYS | I | 222 | -61.575 | -50.950 | 64.682 | 1.00 | 75.65 | D000 N1+ |
| ATOM | 7160 | N | TRP | I | 223 | -54.475 | -47.155 | 65.630 | 1.00 | 40.12 | D000 N |
| ATOM | 7161 | CA | TRP | I | 223 | -54.033 | -45.768 | 65.562 | 1.00 | 42.18 | D000 C |
| ATOM | 7162 | C | TRP | I | 223 | -55.057 | -44.910 | 64.815 | 1.00 | 40.82 | D000 C |
| ATOM | 7163 | O | TRP | I | 223 | -55.674 | -45.348 | 63.843 | 1.00 | 42.00 | D000 O |
| ATOM | 7164 | CB | TRP | I | 223 | -52.654 | -45.651 | 64.892 | 1.00 | 40.05 | D000 C |
| ATOM | 7165 | CG | TRP | I | 223 | -51.497 | -46.261 | 65.689 | 1.00 | 43.88 | D000 C |

| | | | | | | | | | | | | |
|---|---|---|---|---|---|---|---|---|---|---|---|---|
| ATOM | 7166 | CD1 | TRP | I | 223 | -50.994 | -47.529 | 65.568 | 1.00 | 43.26 | D000 | C |
| ATOM | 7167 | CD2 | TRP | I | 223 | -50.711 | -45.620 | 66.718 | 1.00 | 45.79 | D000 | C |
| ATOM | 7168 | NE1 | TRP | I | 223 | -49.959 | -47.722 | 66.455 | 1.00 | 40.92 | D000 | N |
| ATOM | 7169 | CE2 | TRP | I | 223 | -49.761 | -46.570 | 67.171 | 1.00 | 42.07 | D000 | C |
| ATOM | 7170 | CE3 | TRP | I | 223 | -50.730 | -44.341 | 67.310 | 1.00 | 40.91 | D000 | C |
| ATOM | 7171 | CZ2 | TRP | I | 223 | -48.837 | -46.284 | 68.182 | 1.00 | 38.74 | D000 | C |
| ATOM | 7172 | CZ3 | TRP | I | 223 | -49.809 | -44.054 | 68.305 | 1.00 | 37.29 | D000 | C |
| ATOM | 7173 | CH2 | TRP | I | 223 | -48.872 | -45.027 | 68.730 | 1.00 | 42.40 | D000 | C |
| ATOM | 7174 | N | VAL | I | 224 | -55.158 | -43.642 | 65.220 | 1.00 | 35.57 | D000 | N |
| ATOM | 7175 | CA | VAL | I | 224 | -56.214 | -42.782 | 64.710 | 1.00 | 38.56 | D000 | C |
| ATOM | 7176 | C | VAL | I | 224 | -56.046 | -42.420 | 63.229 | 1.00 | 44.84 | D000 | C |
| ATOM | 7177 | O | VAL | I | 224 | -57.010 | -41.965 | 62.593 | 1.00 | 41.50 | D000 | O |
| ATOM | 7178 | CB | VAL | I | 224 | -56.317 | -41.511 | 65.574 | 1.00 | 38.88 | D000 | C |
| ATOM | 7179 | CG1 | VAL | I | 224 | -56.643 | -41.880 | 66.984 | 1.00 | 34.96 | D000 | C |
| ATOM | 7180 | CG2 | VAL | I | 224 | -55.044 | -40.664 | 65.498 | 1.00 | 36.68 | D000 | C |
| ATOM | 7181 | N | ASP | I | 225 | -54.854 | -42.575 | 62.657 | 1.00 | 47.42 | D000 | N |
| ATOM | 7182 | CA | ASP | I | 225 | -54.634 | -42.202 | 61.264 | 1.00 | 45.07 | D000 | C |
| ATOM | 7183 | C | ASP | I | 225 | -54.667 | -43.405 | 60.323 | 1.00 | 47.81 | D000 | C |
| ATOM | 7184 | O | ASP | I | 225 | -54.433 | -43.248 | 59.117 | 1.00 | 47.18 | D000 | O |
| ATOM | 7185 | CB | ASP | I | 225 | -53.316 | -41.416 | 61.120 | 1.00 | 43.11 | D000 | C |
| ATOM | 7186 | CG | ASP | I | 225 | -52.062 | -42.297 | 61.130 | 1.00 | 45.43 | D000 | C |
| ATOM | 7187 | OD1 | ASP | I | 225 | -52.115 | -43.489 | 61.519 | 1.00 | 41.39 | D000 | O |
| ATOM | 7188 | OD2 | ASP | I | 225 | -50.996 | -41.765 | 60.728 | 1.00 | 46.94 | D000 | O1- |
| ATOM | 7189 | N | GLY | I | 226 | -54.980 | -44.593 | 60.847 | 1.00 | 47.45 | D000 | N |
| ATOM | 7190 | CA | GLY | I | 226 | -55.065 | -45.811 | 60.081 | 1.00 | 41.79 | D000 | C |
| ATOM | 7191 | C | GLY | I | 226 | -53.833 | -46.683 | 60.170 | 1.00 | 52.00 | D000 | C |
| ATOM | 7192 | O | GLY | I | 226 | -53.912 | -47.876 | 59.850 | 1.00 | 56.10 | D000 | O |
| ATOM | 7193 | N | THR | I | 227 | -52.713 | -46.128 | 60.647 | 1.00 | 51.85 | D000 | N |
| ATOM | 7194 | CA | THR | I | 227 | -51.473 | -46.886 | 60.769 | 1.00 | 46.63 | D000 | C |
| ATOM | 7195 | C | THR | I | 227 | -51.717 | -48.209 | 61.476 | 1.00 | 39.30 | D000 | C |
| ATOM | 7196 | O | THR | I | 227 | -52.498 | -48.293 | 62.422 | 1.00 | 45.35 | D000 | O |
| ATOM | 7197 | CB | THR | I | 227 | -50.425 | -46.078 | 61.535 | 1.00 | 41.84 | D000 | C |
| ATOM | 7198 | OG1 | THR | I | 227 | -50.287 | -44.782 | 60.931 | 1.00 | 39.58 | D000 | O |
| ATOM | 7199 | CG2 | THR | I | 227 | -49.093 | -46.792 | 61.502 | 1.00 | 32.58 | D000 | C |
| ATOM | 7200 | N | ASP | I | 228 | -51.097 | -49.259 | 60.965 | 1.00 | 42.37 | D000 | N |
| ATOM | 7201 | CA | ASP | I | 228 | -51.312 | -50.564 | 61.553 | 1.00 | 43.67 | D000 | C |
| ATOM | 7202 | C | ASP | I | 228 | -50.643 | -50.637 | 62.911 | 1.00 | 47.41 | D000 | C |
| ATOM | 7203 | O | ASP | I | 228 | -49.510 | -50.182 | 63.090 | 1.00 | 48.24 | D000 | O |
| ATOM | 7204 | CB | ASP | I | 228 | -50.780 | -51.670 | 60.657 | 1.00 | 44.10 | D000 | C |
| ATOM | 7205 | CG | ASP | I | 228 | -50.886 | -53.024 | 61.317 | 1.00 | 47.63 | D000 | C |
| ATOM | 7206 | OD1 | ASP | I | 228 | -52.011 | -53.436 | 61.652 | 1.00 | 48.15 | D000 | O |
| ATOM | 7207 | OD2 | ASP | I | 228 | -49.842 | -53.674 | 61.519 | 1.00 | 56.59 | D000 | O1- |
| ATOM | 7208 | N | TYR | I | 229 | -51.362 | -51.196 | 63.878 | 1.00 | 48.84 | D000 | N |
| ATOM | 7209 | CA | TYR | I | 229 | -50.808 | -51.333 | 65.220 | 1.00 | 52.99 | D000 | C |
| ATOM | 7210 | C | TYR | I | 229 | -49.895 | -52.557 | 65.345 | 1.00 | 52.23 | D000 | C |
| ATOM | 7211 | O | TYR | I | 229 | -48.800 | -52.471 | 65.919 | 1.00 | 51.49 | D000 | O |
| ATOM | 7212 | CB | TYR | I | 229 | -51.949 | -51.395 | 66.243 | 1.00 | 46.59 | D000 | C |
| ATOM | 7213 | CG | TYR | I | 229 | -51.529 | -51.806 | 67.625 | 1.00 | 44.81 | D000 | C |
| ATOM | 7214 | CD2 | TYR | I | 229 | -51.533 | -53.141 | 67.992 | 1.00 | 47.32 | D000 | C |
| ATOM | 7215 | CD1 | TYR | I | 229 | -51.096 | -50.875 | 68.549 | 1.00 | 47.52 | D000 | C |
| ATOM | 7216 | CE2 | TYR | I | 229 | -51.138 | -53.545 | 69.242 | 1.00 | 50.06 | D000 | C |
| ATOM | 7217 | CE1 | TYR | I | 229 | -50.700 | -51.268 | 69.830 | 1.00 | 49.62 | D000 | C |
| ATOM | 7218 | CZ | TYR | I | 229 | -50.723 | -52.613 | 70.161 | 1.00 | 51.78 | D000 | C |
| ATOM | 7219 | OH | TYR | I | 229 | -50.341 | -53.044 | 71.407 | 1.00 | 41.13 | D000 | O |
| ATOM | 7220 | N | GLU | I | 230 | -50.333 | -53.705 | 64.828 | 1.00 | 53.05 | D000 | N |
| ATOM | 7221 | CA | GLU | I | 230 | -49.710 | -54.967 | 65.221 | 1.00 | 54.20 | D000 | C |
| ATOM | 7222 | C | GLU | I | 230 | -48.276 | -55.102 | 64.708 | 1.00 | 52.15 | D000 | C |
| ATOM | 7223 | O | GLU | I | 230 | -47.392 | -55.578 | 65.430 | 1.00 | 54.02 | D000 | O |
| ATOM | 7224 | CB | GLU | I | 230 | -50.577 | -56.134 | 64.766 | 1.00 | 60.03 | D000 | C |
| ATOM | 7225 | CG | GLU | I | 230 | -50.249 | -57.386 | 65.539 | 1.00 | 67.01 | D000 | C |
| ATOM | 7226 | CD | GLU | I | 230 | -50.036 | -57.099 | 67.016 | 1.00 | 66.61 | D000 | C |
| ATOM | 7227 | OE1 | GLU | I | 230 | -51.027 | -56.765 | 67.706 | 1.00 | 68.50 | D000 | O |
| ATOM | 7228 | OE2 | GLU | I | 230 | -48.871 | -57.189 | 67.479 | 1.00 | 67.23 | D000 | O1- |
| ATOM | 7229 | N | THR | I | 231 | -48.019 | -54.721 | 63.470 | 1.00 | 52.10 | D000 | N |
| ATOM | 7230 | CA | THR | I | 231 | -46.651 | -54.762 | 62.968 | 1.00 | 54.05 | D000 | C |
| ATOM | 7231 | C | THR | I | 231 | -45.864 | -53.483 | 63.276 | 1.00 | 50.36 | D000 | C |
| ATOM | 7232 | O | THR | I | 231 | -44.664 | -53.426 | 62.977 | 1.00 | 52.95 | D000 | O |
| ATOM | 7233 | CB | THR | I | 231 | -46.649 | -55.055 | 61.450 | 1.00 | 52.18 | D000 | C |
| ATOM | 7234 | OG1 | THR | I | 231 | -47.303 | -53.996 | 60.732 | 1.00 | 44.52 | D000 | O |
| ATOM | 7235 | CG2 | THR | I | 231 | -47.325 | -56.388 | 61.140 | 1.00 | 44.56 | D000 | C |
| ATOM | 7236 | N | GLY | I | 232 | -46.494 | -52.478 | 63.907 | 1.00 | 46.91 | D000 | N |

| | | | | | | | | | | | | |
|---|---|---|---|---|---|---|---|---|---|---|---|---|
| ATOM | 7237 | CA | GLY | I | 232 | -45.878 | -51.180 | 64.126 | 1.00 | 47.37 | D000 | C |
| ATOM | 7238 | C | GLY | I | 232 | -45.126 | -51.024 | 65.460 | 1.00 | 47.60 | D000 | C |
| ATOM | 7239 | O | GLY | I | 232 | -45.082 | -51.911 | 66.317 | 1.00 | 49.36 | D000 | O |
| ATOM | 7240 | N | PHE | I | 233 | -44.496 | -49.860 | 65.599 | 1.00 | 41.36 | D000 | N |
| ATOM | 7241 | CA | PHE | I | 233 | -43.718 | -49.535 | 66.789 | 1.00 | 43.90 | D000 | C |
| ATOM | 7242 | C | PHE | I | 233 | -44.583 | -49.581 | 68.043 | 1.00 | 44.53 | D000 | C |
| ATOM | 7243 | O | PHE | I | 233 | -45.750 | -49.180 | 68.019 | 1.00 | 48.88 | D000 | O |
| ATOM | 7244 | CB | PHE | I | 233 | -43.083 | -48.144 | 66.644 | 1.00 | 46.48 | D000 | C |
| ATOM | 7245 | CG | PHE | I | 233 | -42.286 | -47.729 | 67.846 | 1.00 | 53.61 | D000 | C |
| ATOM | 7246 | CD1 | PHE | I | 233 | -40.995 | -48.197 | 68.026 | 1.00 | 46.82 | D000 | C |
| ATOM | 7247 | CD2 | PHE | I | 233 | -42.838 | -46.911 | 68.820 | 1.00 | 52.29 | D000 | C |
| ATOM | 7248 | CE1 | PHE | I | 233 | -40.276 | -47.855 | 69.139 | 1.00 | 48.20 | D000 | C |
| ATOM | 7249 | CE2 | PHE | I | 233 | -42.114 | -46.558 | 69.940 | 1.00 | 45.86 | D000 | C |
| ATOM | 7250 | CZ | PHE | I | 233 | -40.831 | -47.029 | 70.096 | 1.00 | 50.22 | D000 | C |
| ATOM | 7251 | N | LYS | I | 234 | -44.011 | -50.083 | 69.143 | 1.00 | 41.34 | D000 | N |
| ATOM | 7252 | CA | LYS | I | 234 | -44.704 | -50.159 | 70.428 | 1.00 | 44.31 | D000 | C |
| ATOM | 7253 | C | LYS | I | 234 | -43.739 | -49.876 | 71.575 | 1.00 | 45.74 | D000 | C |
| ATOM | 7254 | O | LYS | I | 234 | -42.561 | -50.223 | 71.499 | 1.00 | 52.26 | D000 | O |
| ATOM | 7255 | CB | LYS | I | 234 | -45.349 | -51.532 | 70.647 | 1.00 | 50.12 | D000 | C |
| ATOM | 7256 | CG | LYS | I | 234 | -46.423 | -51.935 | 69.649 | 1.00 | 46.91 | D000 | C |
| ATOM | 7257 | CD | LYS | I | 234 | -46.920 | -53.333 | 69.980 | 1.00 | 41.34 | D000 | C |
| ATOM | 7258 | CE | LYS | I | 234 | -47.495 | -53.986 | 68.756 | 1.00 | 52.95 | D000 | C |
| ATOM | 7259 | NZ | LYS | I | 234 | -46.449 | -54.101 | 67.706 | 1.00 | 54.61 | D000 | N1+ |
| ATOM | 7260 | N | ASN | I | 235 | -44.239 | -49.255 | 72.646 | 1.00 | 46.61 | D000 | N |
| ATOM | 7261 | CA | ASN | I | 235 | -43.386 | -48.905 | 73.785 | 1.00 | 40.27 | D000 | C |
| ATOM | 7262 | C | ASN | I | 235 | -44.180 | -48.890 | 75.098 | 1.00 | 44.39 | D000 | C |
| ATOM | 7263 | O | ASN | I | 235 | -44.138 | -47.925 | 75.865 | 1.00 | 45.23 | D000 | O |
| ATOM | 7264 | CB | ASN | I | 235 | -42.691 | -47.564 | 73.545 | 1.00 | 38.53 | D000 | C |
| ATOM | 7265 | CG | ASN | I | 235 | -41.620 | -47.270 | 74.577 | 1.00 | 40.11 | D000 | C |
| ATOM | 7266 | OD1 | ASN | I | 235 | -40.990 | -48.178 | 75.105 | 1.00 | 44.27 | D000 | O |
| ATOM | 7267 | ND2 | ASN | I | 235 | -41.420 | -46.000 | 74.879 | 1.00 | 41.29 | D000 | N |
| ATOM | 7268 | N | TRP | I | 236 | -44.905 | -49.971 | 75.387 | 1.00 | 43.99 | D000 | N |
| ATOM | 7269 | CA | TRP | I | 236 | -45.720 | -50.052 | 76.600 | 1.00 | 45.07 | D000 | C |
| ATOM | 7270 | C | TRP | I | 236 | -44.866 | -50.125 | 77.866 | 1.00 | 46.24 | D000 | C |
| ATOM | 7271 | O | TRP | I | 236 | -43.739 | -50.626 | 77.846 | 1.00 | 47.40 | D000 | O |
| ATOM | 7272 | CB | TRP | I | 236 | -46.614 | -51.289 | 76.550 | 1.00 | 44.75 | D000 | C |
| ATOM | 7273 | CG | TRP | I | 236 | -47.647 | -51.240 | 75.491 | 1.00 | 48.81 | D000 | C |
| ATOM | 7274 | CD1 | TRP | I | 236 | -47.616 | -51.876 | 74.288 | 1.00 | 46.27 | D000 | C |
| ATOM | 7275 | CD2 | TRP | I | 236 | -48.878 | -50.513 | 75.531 | 1.00 | 50.08 | D000 | C |
| ATOM | 7276 | NE1 | TRP | I | 236 | -48.749 | -51.591 | 73.573 | 1.00 | 46.50 | D000 | N |
| ATOM | 7277 | CE2 | TRP | I | 236 | -49.543 | -50.756 | 74.314 | 1.00 | 52.31 | D000 | C |
| ATOM | 7278 | CE3 | TRP | I | 236 | -49.482 | -49.680 | 76.480 | 1.00 | 48.48 | D000 | C |
| ATOM | 7279 | CZ2 | TRP | I | 236 | -50.787 | -50.195 | 74.018 | 1.00 | 53.44 | D000 | C |
| ATOM | 7280 | CZ3 | TRP | I | 236 | -50.708 | -49.126 | 76.192 | 1.00 | 49.54 | D000 | C |
| ATOM | 7281 | CH2 | TRP | I | 236 | -51.354 | -49.388 | 74.969 | 1.00 | 55.96 | D000 | C |
| ATOM | 7282 | N | ARG | I | 237 | -45.410 | -49.600 | 78.981 | 1.00 | 46.86 | D000 | N |
| ATOM | 7283 | CA | ARG | I | 237 | -44.869 | -49.924 | 80.304 | 1.00 | 52.81 | D000 | C |
| ATOM | 7284 | C | ARG | I | 237 | -45.128 | -51.400 | 80.593 | 1.00 | 57.44 | D000 | C |
| ATOM | 7285 | O | ARG | I | 237 | -46.167 | -51.926 | 80.193 | 1.00 | 65.84 | D000 | O |
| ATOM | 7286 | CB | ARG | I | 237 | -45.507 | -49.082 | 81.421 | 1.00 | 48.92 | D000 | C |
| ATOM | 7287 | CG | ARG | I | 237 | -45.148 | -47.579 | 81.479 | 1.00 | 52.62 | D000 | C |
| ATOM | 7288 | CD | ARG | I | 237 | -43.916 | -47.207 | 82.346 | 1.00 | 51.01 | D000 | C |
| ATOM | 7289 | NE | ARG | I | 237 | -44.221 | -47.118 | 83.786 | 1.00 | 55.72 | D000 | N |
| ATOM | 7290 | CZ | ARG | I | 237 | -44.309 | -45.995 | 84.513 | 1.00 | 52.55 | D000 | C |
| ATOM | 7291 | NH1 | ARG | I | 237 | -44.119 | -44.796 | 83.979 | 1.00 | 44.43 | D000 | N1+ |
| ATOM | 7292 | NH2 | ARG | I | 237 | -44.588 | -46.073 | 85.808 | 1.00 | 64.23 | D000 | N |
| ATOM | 7293 | N | PRO | I | 238 | -44.206 | -52.094 | 81.259 | 1.00 | 64.01 | D000 | N |
| ATOM | 7294 | CA | PRO | I | 238 | -44.450 | -53.500 | 81.615 | 1.00 | 65.03 | D000 | C |
| ATOM | 7295 | C | PRO | I | 238 | -45.771 | -53.685 | 82.356 | 1.00 | 74.35 | D000 | C |
| ATOM | 7296 | O | PRO | I | 238 | -46.138 | -52.874 | 83.210 | 1.00 | 79.19 | D000 | O |
| ATOM | 7297 | CB | PRO | I | 238 | -43.244 | -53.846 | 82.493 | 1.00 | 66.34 | D000 | C |
| ATOM | 7298 | CG | PRO | I | 238 | -42.143 | -53.019 | 81.911 | 1.00 | 66.55 | D000 | C |
| ATOM | 7299 | CD | PRO | I | 238 | -42.800 | -51.710 | 81.488 | 1.00 | 66.32 | D000 | C |
| ATOM | 7300 | N | GLU | I | 239 | -46.483 | -54.772 | 82.011 | 1.00 | 76.56 | D000 | N |
| ATOM | 7301 | CA | GLU | I | 239 | -47.850 | -55.186 | 82.415 | 1.00 | 81.96 | D000 | C |
| ATOM | 7302 | C | GLU | I | 239 | -48.960 | -54.622 | 81.521 | 1.00 | 85.73 | D000 | C |
| ATOM | 7303 | O | GLU | I | 239 | -50.134 | -54.970 | 81.747 | 1.00 | 90.20 | D000 | O |
| ATOM | 7304 | CB | GLU | I | 239 | -48.223 | -54.802 | 83.865 | 1.00 | 84.76 | D000 | C |
| ATOM | 7305 | CG | GLU | I | 239 | -48.026 | -55.831 | 84.975 | 1.00 | 89.72 | D000 | C |
| ATOM | 7306 | CD | GLU | I | 239 | -49.063 | -55.648 | 86.104 | 1.00 | 97.46 | D000 | C |
| ATOM | 7307 | OE1 | GLU | I | 239 | -50.264 | -55.495 | 85.787 | 1.00 | 95.00 | D000 | O |

| | | | | | | | | | | | | |
|---|---|---|---|---|---|---|---|---|---|---|---|---|
| ATOM | 7308 | OE2 | GLU | I | 239 | -48.689 | -55.645 | 87.301 | 1.00 | 99.60 | D000 | O1- |
| ATOM | 7309 | N | GLN | I | 240 | -48.655 | -53.809 | 80.513 | 1.00 | 78.77 | D000 | N |
| ATOM | 7310 | CA | GLN | I | 240 | -49.716 | -53.126 | 79.762 | 1.00 | 74.65 | D000 | C |
| ATOM | 7311 | C | GLN | I | 240 | -49.744 | -53.472 | 78.266 | 1.00 | 75.43 | D000 | C |
| ATOM | 7312 | O | GLN | I | 240 | -48.690 | -53.736 | 77.678 | 1.00 | 70.27 | D000 | O |
| ATOM | 7313 | CB | GLN | I | 240 | -49.570 | -51.610 | 79.956 | 1.00 | 69.99 | D000 | C |
| ATOM | 7314 | CG | GLN | I | 240 | -49.627 | -51.179 | 81.421 | 1.00 | 73.64 | D000 | C |
| ATOM | 7315 | CD | GLN | I | 240 | -51.003 | -51.423 | 82.047 | 1.00 | 79.48 | D000 | C |
| ATOM | 7316 | OE1 | GLN | I | 240 | -51.278 | -52.503 | 82.587 | 1.00 | 78.83 | D000 | O |
| ATOM | 7317 | NE2 | GLN | I | 240 | -51.877 | -50.413 | 81.968 | 1.00 | 68.96 | D000 | N |
| ATOM | 7318 | N | PRO | I | 241 | -50.945 | -53.443 | 77.634 | 1.00 | 74.07 | D000 | N |
| ATOM | 7319 | CA | PRO | I | 241 | -52.250 | -53.045 | 78.188 | 1.00 | 70.37 | D000 | C |
| ATOM | 7320 | C | PRO | I | 241 | -52.840 | -54.061 | 79.178 | 1.00 | 75.66 | D000 | C |
| ATOM | 7321 | O | PRO | I | 241 | -53.904 | -54.638 | 78.940 | 1.00 | 82.05 | D000 | O |
| ATOM | 7322 | CB | PRO | I | 241 | -53.129 | -52.936 | 76.941 | 1.00 | 70.97 | D000 | C |
| ATOM | 7323 | CG | PRO | I | 241 | -52.543 | -53.932 | 75.998 | 1.00 | 67.57 | D000 | C |
| ATOM | 7324 | CD | PRO | I | 241 | -51.061 | -53.841 | 76.215 | 1.00 | 64.21 | D000 | C |
| ATOM | 7325 | N | GLY | I | 252 | -62.815 | -49.790 | 79.716 | 1.00 | 70.62 | D000 | N |
| ATOM | 7326 | CA | GLY | I | 252 | -62.424 | -49.267 | 81.017 | 1.00 | 74.95 | D000 | C |
| ATOM | 7327 | C | GLY | I | 252 | -61.035 | -48.648 | 81.048 | 1.00 | 70.30 | D000 | C |
| ATOM | 7328 | O | GLY | I | 252 | -60.698 | -47.871 | 81.940 | 1.00 | 66.65 | D000 | O |
| ATOM | 7329 | N | GLU | I | 253 | -60.214 | -49.020 | 80.065 | 1.00 | 73.32 | D000 | N |
| ATOM | 7330 | CA | GLU | I | 253 | -58.880 | -48.472 | 79.814 | 1.00 | 64.01 | D000 | C |
| ATOM | 7331 | C | GLU | I | 253 | -58.695 | -48.525 | 78.298 | 1.00 | 60.63 | D000 | C |
| ATOM | 7332 | O | GLU | I | 253 | -57.927 | -49.303 | 77.737 | 1.00 | 61.66 | D000 | O |
| ATOM | 7333 | CB | GLU | I | 253 | -57.775 | -49.234 | 80.548 | 1.00 | 65.40 | D000 | C |
| ATOM | 7334 | CG | GLU | I | 253 | -57.837 | -49.139 | 82.057 | 1.00 | 69.16 | D000 | C |
| ATOM | 7335 | CD | GLU | I | 253 | -57.007 | -50.206 | 82.724 | 1.00 | 72.49 | D000 | C |
| ATOM | 7336 | OE1 | GLU | I | 253 | -55.760 | -50.161 | 82.597 | 1.00 | 66.87 | D000 | O |
| ATOM | 7337 | OE2 | GLU | I | 253 | -57.610 | -51.095 | 83.367 | 1.00 | 87.80 | D000 | O1- |
| ATOM | 7338 | N | ASP | I | 254 | -59.422 | -47.662 | 77.607 | 1.00 | 59.36 | D000 | N |
| ATOM | 7339 | CA | ASP | I | 254 | -59.495 | -47.720 | 76.163 | 1.00 | 59.89 | D000 | C |
| ATOM | 7340 | C | ASP | I | 254 | -58.603 | -46.688 | 75.471 | 1.00 | 54.78 | D000 | C |
| ATOM | 7341 | O | ASP | I | 254 | -58.661 | -46.575 | 74.247 | 1.00 | 56.31 | D000 | O |
| ATOM | 7342 | CB | ASP | I | 254 | -60.957 | -47.546 | 75.721 | 1.00 | 67.74 | D000 | C |
| ATOM | 7343 | CG | ASP | I | 254 | -61.915 | -48.563 | 76.378 | 1.00 | 69.29 | D000 | C |
| ATOM | 7344 | OD1 | ASP | I | 254 | -61.449 | -49.599 | 76.909 | 1.00 | 61.14 | D000 | O |
| ATOM | 7345 | OD2 | ASP | I | 254 | -63.146 | -48.308 | 76.361 | 1.00 | 69.38 | D000 | O1- |
| ATOM | 7346 | N | CYS | I | 255 | -57.789 | -45.925 | 76.210 | 1.00 | 47.78 | D000 | N |
| ATOM | 7347 | CA | CYS | I | 255 | -56.967 | -44.877 | 75.608 | 1.00 | 41.04 | D000 | C |
| ATOM | 7348 | C | CYS | I | 255 | -55.530 | -44.924 | 76.117 | 1.00 | 39.20 | D000 | C |
| ATOM | 7349 | O | CYS | I | 255 | -55.295 | -45.211 | 77.290 | 1.00 | 41.84 | D000 | O |
| ATOM | 7350 | CB | CYS | I | 255 | -57.559 | -43.510 | 75.866 | 1.00 | 41.88 | D000 | C |
| ATOM | 7351 | SG | CYS | I | 255 | -59.123 | -43.260 | 75.028 | 1.00 | 54.50 | D000 | S |
| ATOM | 7352 | N | ALA | I | 256 | -54.577 | -44.614 | 75.230 | 1.00 | 35.83 | D000 | N |
| ATOM | 7353 | CA | ALA | I | 256 | -53.144 | -44.757 | 75.487 | 1.00 | 36.99 | D000 | C |
| ATOM | 7354 | C | ALA | I | 256 | -52.466 | -43.414 | 75.756 | 1.00 | 31.83 | D000 | C |
| ATOM | 7355 | O | ALA | I | 256 | -52.700 | -42.427 | 75.047 | 1.00 | 30.75 | D000 | O |
| ATOM | 7356 | CB | ALA | I | 256 | -52.445 | -45.428 | 74.300 | 1.00 | 37.73 | D000 | C |
| ATOM | 7357 | N | HIS | I | 257 | -51.584 | -43.386 | 76.754 | 1.00 | 31.20 | D000 | N |
| ATOM | 7358 | CA | HIS | I | 257 | -50.871 | -42.153 | 77.060 | 1.00 | 31.95 | D000 | C |
| ATOM | 7359 | C | HIS | I | 257 | -49.411 | -42.453 | 77.347 | 1.00 | 31.16 | D000 | C |
| ATOM | 7360 | O | HIS | I | 257 | -49.056 | -43.554 | 77.782 | 1.00 | 30.03 | D000 | O |
| ATOM | 7361 | CB | HIS | I | 257 | -51.523 | -41.373 | 78.240 | 1.00 | 28.18 | D000 | C |
| ATOM | 7362 | CG | HIS | I | 257 | -51.545 | -42.119 | 79.546 | 1.00 | 35.85 | D000 | C |
| ATOM | 7363 | ND1 | HIS | I | 257 | -50.561 | -41.985 | 80.501 | 1.00 | 32.60 | D000 | N |
| ATOM | 7364 | CD2 | HIS | I | 257 | -52.433 | -43.010 | 80.051 | 1.00 | 37.79 | D000 | C |
| ATOM | 7365 | CE1 | HIS | I | 257 | -50.842 | -42.756 | 81.535 | 1.00 | 33.70 | D000 | C |
| ATOM | 7366 | NE2 | HIS | I | 257 | -51.975 | -43.385 | 81.290 | 1.00 | 38.87 | D000 | N |
| ATOM | 7367 | N | PHE | I | 258 | -48.566 | -41.458 | 77.069 | 1.00 | 32.31 | D000 | N |
| ATOM | 7368 | CA | PHE | I | 258 | -47.195 | -41.470 | 77.550 | 1.00 | 29.99 | D000 | C |
| ATOM | 7369 | C | PHE | I | 258 | -47.186 | -41.294 | 79.066 | 1.00 | 32.50 | D000 | C |
| ATOM | 7370 | O | PHE | I | 258 | -48.064 | -40.659 | 79.648 | 1.00 | 28.89 | D000 | O |
| ATOM | 7371 | CB | PHE | I | 258 | -46.375 | -40.346 | 76.926 | 1.00 | 31.38 | D000 | C |
| ATOM | 7372 | CG | PHE | I | 258 | -46.400 | -40.303 | 75.434 | 1.00 | 30.17 | D000 | C |
| ATOM | 7373 | CD1 | PHE | I | 258 | -45.501 | -41.033 | 74.692 | 1.00 | 31.17 | D000 | C |
| ATOM | 7374 | CD2 | PHE | I | 258 | -47.305 | -39.487 | 74.769 | 1.00 | 35.10 | D000 | C |
| ATOM | 7375 | CE1 | PHE | I | 258 | -45.522 | -40.966 | 73.303 | 1.00 | 36.51 | D000 | C |
| ATOM | 7376 | CE2 | PHE | I | 258 | -47.330 | -39.415 | 73.380 | 1.00 | 33.32 | D000 | C |
| ATOM | 7377 | CZ | PHE | I | 258 | -46.440 | -40.152 | 72.648 | 1.00 | 29.52 | D000 | C |
| ATOM | 7378 | N | THR | I | 259 | -46.212 | -41.900 | 79.709 | 1.00 | 33.00 | D000 | N |

| | | | | | | | | | | | |
|---|---|---|---|---|---|---|---|---|---|---|---|
| ATOM | 7379 | CA | THR | I | 259 | -46.015 | -41.713 | 81.132 | 1.00 | 34.18 | D000 C |
| ATOM | 7380 | C | THR | I | 259 | -44.809 | -40.809 | 81.341 | 1.00 | 36.61 | D000 C |
| ATOM | 7381 | O | THR | I | 259 | -44.172 | -40.339 | 80.389 | 1.00 | 38.23 | D000 O |
| ATOM | 7382 | CB | THR | I | 259 | -45.834 | -43.049 | 81.856 | 1.00 | 36.60 | D000 C |
| ATOM | 7383 | OG1 | THR | I | 259 | -44.598 | -43.658 | 81.448 | 1.00 | 38.29 | D000 O |
| ATOM | 7384 | CG2 | THR | I | 259 | -47.006 | -43.966 | 81.583 | 1.00 | 32.69 | D000 C |
| ATOM | 7385 | N | ASP | I | 260 | -44.535 | -40.524 | 82.610 | 1.00 | 36.88 | D000 N |
| ATOM | 7386 | CA | ASP | I | 260 | -43.424 | -39.664 | 82.983 | 1.00 | 36.02 | D000 C |
| ATOM | 7387 | C | ASP | I | 260 | -42.065 | -40.201 | 82.520 | 1.00 | 37.71 | D000 C |
| ATOM | 7388 | O | ASP | I | 260 | -41.091 | -39.443 | 82.545 | 1.00 | 42.78 | D000 O |
| ATOM | 7389 | CB | ASP | I | 260 | -43.475 | -39.440 | 84.501 | 1.00 | 38.98 | D000 C |
| ATOM | 7390 | CG | ASP | I | 260 | -43.369 | -40.730 | 85.292 | 1.00 | 40.08 | D000 C |
| ATOM | 7391 | OD1 | ASP | I | 260 | -43.567 | -41.816 | 84.709 | 1.00 | 45.52 | D000 O |
| ATOM | 7392 | OD2 | ASP | I | 260 | -43.171 | -40.658 | 86.518 | 1.00 | 38.74 | D000 O1- |
| ATOM | 7393 | N | ASP | I | 261 | -41.964 | -41.467 | 82.099 | 1.00 | 33.98 | D000 N |
| ATOM | 7394 | CA | ASP | I | 261 | -40.713 | -42.008 | 81.579 | 1.00 | 35.49 | D000 C |
| ATOM | 7395 | C | ASP | I | 261 | -40.771 | -42.320 | 80.090 | 1.00 | 41.58 | D000 C |
| ATOM | 7396 | O | ASP | I | 261 | -39.833 | -42.910 | 79.550 | 1.00 | 41.61 | D000 O |
| ATOM | 7397 | CB | ASP | I | 261 | -40.289 | -43.240 | 82.374 | 1.00 | 31.38 | D000 C |
| ATOM | 7398 | CG | ASP | I | 261 | -41.194 | -44.433 | 82.150 | 1.00 | 44.50 | D000 C |
| ATOM | 7399 | OD1 | ASP | I | 261 | -42.145 | -44.348 | 81.338 | 1.00 | 49.19 | D000 O |
| ATOM | 7400 | OD2 | ASP | I | 261 | -40.992 | -45.454 | 82.843 | 1.00 | 43.56 | D000 O1- |
| ATOM | 7401 | N | GLY | I | 262 | -41.853 | -41.954 | 79.414 | 1.00 | 43.44 | D000 N |
| ATOM | 7402 | CA | GLY | I | 262 | -41.983 | -42.127 | 77.991 | 1.00 | 39.17 | D000 C |
| ATOM | 7403 | C | GLY | I | 262 | -42.711 | -43.389 | 77.570 | 1.00 | 36.69 | D000 C |
| ATOM | 7404 | O | GLY | I | 262 | -43.387 | -43.377 | 76.537 | 1.00 | 31.77 | D000 O |
| ATOM | 7405 | N | ARG | I | 263 | -42.625 | -44.459 | 78.358 | 1.00 | 32.80 | D000 N |
| ATOM | 7406 | CA | ARG | I | 263 | -43.300 | -45.692 | 77.987 | 1.00 | 40.54 | D000 C |
| ATOM | 7407 | C | ARG | I | 263 | -44.820 | -45.549 | 78.138 | 1.00 | 42.34 | D000 C |
| ATOM | 7408 | O | ARG | I | 263 | -45.332 | -44.731 | 78.905 | 1.00 | 38.87 | D000 O |
| ATOM | 7409 | CB | ARG | I | 263 | -42.773 | -46.854 | 78.822 | 1.00 | 43.57 | D000 C |
| ATOM | 7410 | CG | ARG | I | 263 | -41.309 | -47.117 | 78.559 | 1.00 | 42.00 | D000 C |
| ATOM | 7411 | CD | ARG | I | 263 | -40.789 | -48.259 | 79.373 | 1.00 | 43.37 | D000 C |
| ATOM | 7412 | NE | ARG | I | 263 | -41.000 | -48.089 | 80.802 | 1.00 | 47.00 | D000 N |
| ATOM | 7413 | CZ | ARG | I | 263 | -40.718 | -49.024 | 81.706 | 1.00 | 54.61 | D000 C |
| ATOM | 7414 | NH1 | ARG | I | 263 | -40.206 | -50.187 | 81.325 | 1.00 | 56.14 | D000 N1+ |
| ATOM | 7415 | NH2 | ARG | I | 263 | -40.954 | -48.806 | 82.995 | 1.00 | 57.34 | D000 N |
| ATOM | 7416 | N | TRP | I | 264 | -45.545 | -46.362 | 77.390 | 1.00 | 41.85 | D000 N |
| ATOM | 7417 | CA | TRP | I | 264 | -46.964 | -46.115 | 77.212 | 1.00 | 40.32 | D000 C |
| ATOM | 7418 | C | TRP | I | 264 | -47.801 | -46.797 | 78.282 | 1.00 | 39.94 | D000 C |
| ATOM | 7419 | O | TRP | I | 264 | -47.377 | -47.759 | 78.922 | 1.00 | 45.09 | D000 O |
| ATOM | 7420 | CB | TRP | I | 264 | -47.427 | -46.610 | 75.851 | 1.00 | 41.81 | D000 C |
| ATOM | 7421 | CG | TRP | I | 264 | -46.709 | -46.051 | 74.695 | 1.00 | 36.80 | D000 C |
| ATOM | 7422 | CD1 | TRP | I | 264 | -45.834 | -45.016 | 74.688 | 1.00 | 37.17 | D000 C |
| ATOM | 7423 | CD2 | TRP | I | 264 | -46.807 | -46.512 | 73.349 | 1.00 | 41.41 | D000 C |
| ATOM | 7424 | NE1 | TRP | I | 264 | -45.377 | -44.795 | 73.417 | 1.00 | 37.55 | D000 N |
| ATOM | 7425 | CE2 | TRP | I | 264 | -45.957 | -45.707 | 72.574 | 1.00 | 42.77 | D000 C |
| ATOM | 7426 | CE3 | TRP | I | 264 | -47.530 | -47.534 | 72.724 | 1.00 | 37.74 | D000 C |
| ATOM | 7427 | CZ2 | TRP | I | 264 | -45.810 | -45.888 | 71.205 | 1.00 | 40.50 | D000 C |
| ATOM | 7428 | CZ3 | TRP | I | 264 | -47.378 | -47.716 | 71.383 | 1.00 | 38.97 | D000 C |
| ATOM | 7429 | CH2 | TRP | I | 264 | -46.536 | -46.894 | 70.631 | 1.00 | 44.66 | D000 C |
| ATOM | 7430 | N | ASN | I | 265 | -49.029 | -46.309 | 78.432 | 1.00 | 37.04 | D000 N |
| ATOM | 7431 | CA | ASN | I | 265 | -49.963 | -46.923 | 79.356 | 1.00 | 37.46 | D000 C |
| ATOM | 7432 | C | ASN | I | 265 | -51.387 | -46.554 | 78.963 | 1.00 | 37.95 | D000 C |
| ATOM | 7433 | O | ASN | I | 265 | -51.631 | -45.512 | 78.353 | 1.00 | 36.28 | D000 O |
| ATOM | 7434 | CB | ASN | I | 265 | -49.655 | -46.518 | 80.791 | 1.00 | 40.53 | D000 C |
| ATOM | 7435 | CG | ASN | I | 265 | -50.611 | -47.123 | 81.765 | 1.00 | 49.10 | D000 C |
| ATOM | 7436 | OD1 | ASN | I | 265 | -50.635 | -48.337 | 81.939 | 1.00 | 51.66 | D000 O |
| ATOM | 7437 | ND2 | ASN | I | 265 | -51.389 | -46.281 | 82.440 | 1.00 | 49.67 | D000 N |
| ATOM | 7438 | N | ASP | I | 266 | -52.314 | -47.446 | 79.291 | 1.00 | 45.60 | D000 N |
| ATOM | 7439 | CA | ASP | I | 266 | -53.738 | -47.257 | 79.058 | 1.00 | 45.74 | D000 C |
| ATOM | 7440 | C | ASP | I | 266 | -54.403 | -46.761 | 80.331 | 1.00 | 47.12 | D000 C |
| ATOM | 7441 | O | ASP | I | 266 | -54.070 | -47.212 | 81.429 | 1.00 | 52.08 | D000 O |
| ATOM | 7442 | CB | ASP | I | 266 | -54.413 | -48.561 | 78.615 | 1.00 | 52.43 | D000 C |
| ATOM | 7443 | CG | ASP | I | 266 | -54.095 | -49.732 | 79.538 | 1.00 | 60.22 | D000 C |
| ATOM | 7444 | OD1 | ASP | I | 266 | -52.905 | -49.973 | 79.827 | 1.00 | 64.14 | D000 O |
| ATOM | 7445 | OD2 | ASP | I | 266 | -55.035 | -50.421 | 79.977 | 1.00 | 67.69 | D000 O1- |
| ATOM | 7446 | N | ASP | I | 267 | -55.347 | -45.838 | 80.176 | 1.00 | 44.83 | D000 N |
| ATOM | 7447 | CA | ASP | I | 267 | -56.072 | -45.253 | 81.295 | 1.00 | 49.89 | D000 C |
| ATOM | 7448 | C | ASP | I | 267 | -57.475 | -44.917 | 80.805 | 1.00 | 46.26 | D000 C |
| ATOM | 7449 | O | ASP | I | 267 | -57.782 | -45.069 | 79.623 | 1.00 | 47.12 | D000 O |

| | | | | | | | | | | | | |
|---|---|---|---|---|---|---|---|---|---|---|---|---|
| ATOM | 7450 | CB | ASP | I | 267 | -55.339 | -44.020 | 81.860 | 1.00 | 46.24 | D000 | C |
| ATOM | 7451 | CG | ASP | I | 267 | -55.846 | -43.631 | 83.228 | 1.00 | 48.24 | D000 | C |
| ATOM | 7452 | OD1 | ASP | I | 267 | -56.671 | -44.391 | 83.773 | 1.00 | 56.95 | D000 | O |
| ATOM | 7453 | OD2 | ASP | I | 267 | -55.448 | -42.573 | 83.754 | 1.00 | 49.05 | D000 | O1- |
| ATOM | 7454 | N | VAL | I | 268 | -58.347 | -44.502 | 81.727 | 1.00 | 41.94 | D000 | N |
| ATOM | 7455 | CA | VAL | I | 268 | -59.707 | -44.168 | 81.328 | 1.00 | 44.09 | D000 | C |
| ATOM | 7456 | C | VAL | I | 268 | -59.676 | -42.946 | 80.419 | 1.00 | 44.94 | D000 | C |
| ATOM | 7457 | O | VAL | I | 268 | -58.896 | -42.007 | 80.629 | 1.00 | 44.57 | D000 | O |
| ATOM | 7458 | CB | VAL | I | 268 | -60.606 | -43.939 | 82.554 | 1.00 | 40.01 | D000 | C |
| ATOM | 7459 | CG1 | VAL | I | 268 | -60.463 | -45.079 | 83.506 | 1.00 | 42.61 | D000 | C |
| ATOM | 7460 | CG2 | VAL | I | 268 | -60.274 | -42.634 | 83.242 | 1.00 | 40.82 | D000 | C |
| ATOM | 7461 | N | CYS | I | 269 | -60.506 | -42.962 | 79.379 | 1.00 | 38.74 | D000 | N |
| ATOM | 7462 | CA | CYS | I | 269 | -60.422 | -41.901 | 78.385 | 1.00 | 41.24 | D000 | C |
| ATOM | 7463 | C | CYS | I | 269 | -60.908 | -40.569 | 78.913 | 1.00 | 35.89 | D000 | C |
| ATOM | 7464 | O | CYS | I | 269 | -60.800 | -39.554 | 78.219 | 1.00 | 40.53 | D000 | O |
| ATOM | 7465 | CB | CYS | I | 269 | -61.204 | -42.298 | 77.127 | 1.00 | 43.34 | D000 | C |
| ATOM | 7466 | SG | CYS | I | 269 | -60.582 | -43.835 | 76.370 | 1.00 | 57.52 | D000 | S |
| ATOM | 7467 | N | GLN | I | 270 | -61.422 | -40.534 | 80.127 | 1.00 | 37.09 | D000 | N |
| ATOM | 7468 | CA | GLN | I | 270 | -61.959 | -39.299 | 80.666 | 1.00 | 35.74 | D000 | C |
| ATOM | 7469 | C | GLN | I | 270 | -60.892 | -38.406 | 81.284 | 1.00 | 33.91 | D000 | C |
| ATOM | 7470 | O | GLN | I | 270 | -61.156 | -37.213 | 81.465 | 1.00 | 36.79 | D000 | O |
| ATOM | 7471 | CB | GLN | I | 270 | -63.029 | -39.618 | 81.709 | 1.00 | 43.76 | D000 | C |
| ATOM | 7472 | CG | GLN | I | 270 | -64.394 | -39.929 | 81.095 | 1.00 | 52.98 | D000 | C |
| ATOM | 7473 | CD | GLN | I | 270 | -64.451 | -41.352 | 80.534 | 1.00 | 53.45 | D000 | C |
| ATOM | 7474 | OE1 | GLN | I | 270 | -63.849 | -42.272 | 81.106 | 1.00 | 52.07 | D000 | O |
| ATOM | 7475 | NE2 | GLN | I | 270 | -65.156 | -41.535 | 79.407 | 1.00 | 46.18 | D000 | N |
| ATOM | 7476 | N | ARG | I | 271 | -59.698 | -38.940 | 81.589 | 1.00 | 33.55 | D000 | N |
| ATOM | 7477 | CA | ARG | I | 271 | -58.656 | -38.139 | 82.228 | 1.00 | 34.17 | D000 | C |
| ATOM | 7478 | C | ARG | I | 271 | -58.333 | -36.927 | 81.364 | 1.00 | 35.86 | D000 | C |
| ATOM | 7479 | O | ARG | I | 271 | -58.224 | -37.058 | 80.136 | 1.00 | 34.42 | D000 | O |
| ATOM | 7480 | CB | ARG | I | 271 | -57.380 | -38.947 | 82.464 | 1.00 | 36.48 | D000 | C |
| ATOM | 7481 | CG | ARG | I | 271 | -57.518 | -40.084 | 83.447 | 1.00 | 39.60 | D000 | C |
| ATOM | 7482 | CD | ARG | I | 271 | -57.065 | -39.699 | 84.838 | 1.00 | 45.14 | D000 | C |
| ATOM | 7483 | NE | ARG | I | 271 | -57.534 | -40.662 | 85.844 | 1.00 | 59.73 | D000 | N |
| ATOM | 7484 | CZ | ARG | I | 271 | -58.422 | -40.401 | 86.810 | 1.00 | 58.63 | D000 | C |
| ATOM | 7485 | NH1 | ARG | I | 271 | -58.962 | -39.185 | 86.947 | 1.00 | 54.22 | D000 | N1+ |
| ATOM | 7486 | NH2 | ARG | I | 271 | -58.758 | -41.360 | 87.660 | 1.00 | 55.45 | D000 | N |
| ATOM | 7487 | N | PRO | I | 272 | -58.230 | -35.749 | 81.936 | 1.00 | 32.00 | D000 | N |
| ATOM | 7488 | CA | PRO | I | 272 | -57.855 | -34.553 | 81.148 | 1.00 | 29.00 | D000 | C |
| ATOM | 7489 | C | PRO | I | 272 | -56.342 | -34.344 | 81.068 | 1.00 | 32.23 | D000 | C |
| ATOM | 7490 | O | PRO | I | 272 | -55.758 | -33.416 | 81.633 | 1.00 | 31.22 | D000 | O |
| ATOM | 7491 | CB | PRO | I | 272 | -58.583 | -33.433 | 81.892 | 1.00 | 26.05 | D000 | C |
| ATOM | 7492 | CG | PRO | I | 272 | -58.775 | -33.959 | 83.324 | 1.00 | 28.86 | D000 | C |
| ATOM | 7493 | CD | PRO | I | 272 | -58.499 | -35.435 | 83.347 | 1.00 | 27.78 | D000 | C |
| ATOM | 7494 | N | TYR | I | 273 | -55.680 | -35.225 | 80.325 | 1.00 | 32.52 | D000 | N |
| ATOM | 7495 | CA | TYR | I | 273 | -54.251 | -35.145 | 80.086 | 1.00 | 29.17 | D000 | C |
| ATOM | 7496 | C | TYR | I | 273 | -53.931 | -34.170 | 78.946 | 1.00 | 28.33 | D000 | C |
| ATOM | 7497 | O | TYR | I | 273 | -54.790 | -33.766 | 78.166 | 1.00 | 26.77 | D000 | O |
| ATOM | 7498 | CB | TYR | I | 273 | -53.689 | -36.524 | 79.748 | 1.00 | 26.56 | D000 | C |
| ATOM | 7499 | CG | TYR | I | 273 | -53.687 | -37.527 | 80.867 | 1.00 | 26.99 | D000 | C |
| ATOM | 7500 | CD1 | TYR | I | 273 | -53.588 | -37.129 | 82.194 | 1.00 | 29.63 | D000 | C |
| ATOM | 7501 | CD2 | TYR | I | 273 | -53.757 | -38.883 | 80.594 | 1.00 | 28.80 | D000 | C |
| ATOM | 7502 | CE1 | TYR | I | 273 | -53.582 | -38.057 | 83.212 | 1.00 | 28.90 | D000 | C |
| ATOM | 7503 | CE2 | TYR | I | 273 | -53.737 | -39.825 | 81.609 | 1.00 | 32.53 | D000 | C |
| ATOM | 7504 | CZ | TYR | I | 273 | -53.656 | -39.404 | 82.911 | 1.00 | 29.59 | D000 | C |
| ATOM | 7505 | OH | TYR | I | 273 | -53.634 | -40.338 | 83.910 | 1.00 | 29.82 | D000 | O |
| ATOM | 7506 | N | ARG | I | 274 | -52.672 | -33.768 | 78.882 | 1.00 | 28.32 | D000 | N |
| ATOM | 7507 | CA | ARG | I | 274 | -52.212 | -33.033 | 77.729 | 1.00 | 27.19 | D000 | C |
| ATOM | 7508 | C | ARG | I | 274 | -52.169 | -33.971 | 76.536 | 1.00 | 26.48 | D000 | C |
| ATOM | 7509 | O | ARG | I | 274 | -52.280 | -35.187 | 76.673 | 1.00 | 28.05 | D000 | O |
| ATOM | 7510 | CB | ARG | I | 274 | -50.857 | -32.403 | 78.011 | 1.00 | 24.95 | D000 | C |
| ATOM | 7511 | CG | ARG | I | 274 | -50.926 | -31.334 | 79.066 | 1.00 | 25.95 | D000 | C |
| ATOM | 7512 | CD | ARG | I | 274 | -49.541 | -30.802 | 79.382 | 1.00 | 28.18 | D000 | C |
| ATOM | 7513 | NE | ARG | I | 274 | -49.548 | -29.767 | 80.411 | 1.00 | 27.97 | D000 | N |
| ATOM | 7514 | CZ | ARG | I | 274 | -48.464 | -29.347 | 81.063 | 1.00 | 29.49 | D000 | C |
| ATOM | 7515 | NH1 | ARG | I | 274 | -47.274 | -29.877 | 80.802 | 1.00 | 24.44 | D000 | N1+ |
| ATOM | 7516 | NH2 | ARG | I | 274 | -48.567 | -28.400 | 81.992 | 1.00 | 28.37 | D000 | N |
| ATOM | 7517 | N | TRP | I | 275 | -52.038 | -33.395 | 75.344 | 1.00 | 27.91 | D000 | N |
| ATOM | 7518 | CA | TRP | I | 275 | -52.039 | -34.203 | 74.138 | 1.00 | 28.85 | D000 | C |
| ATOM | 7519 | C | TRP | I | 275 | -51.106 | -33.568 | 73.105 | 1.00 | 30.78 | D000 | C |
| ATOM | 7520 | O | TRP | I | 275 | -50.671 | -32.410 | 73.241 | 1.00 | 27.47 | D000 | O |

```
ATOM   7521  CB  TRP I 275     -53.473 -34.368  73.600  1.00 23.59          D000 C
ATOM   7522  CG  TRP I 275     -54.009 -33.123  72.979  1.00 26.26          D000 C
ATOM   7523  CD1 TRP I 275     -53.991 -32.808  71.648  1.00 26.24          D000 C
ATOM   7524  CD2 TRP I 275     -54.629 -32.005  73.643  1.00 27.84          D000 C
ATOM   7525  NE1 TRP I 275     -54.549 -31.571  71.442  1.00 27.63          D000 N
ATOM   7526  CE2 TRP I 275     -54.959 -31.059  72.646  1.00 29.00          D000 C
ATOM   7527  CE3 TRP I 275     -54.949 -31.718  74.971  1.00 27.22          D000 C
ATOM   7528  CZ2 TRP I 275     -55.589 -29.850  72.941  1.00 23.11          D000 C
ATOM   7529  CZ3 TRP I 275     -55.572 -30.511  75.258  1.00 26.94          D000 C
ATOM   7530  CH2 TRP I 275     -55.876 -29.595  74.248  1.00 27.13          D000 C
ATOM   7531  N   VAL I 276     -50.827 -34.338  72.051  1.00 26.77          D000 N
ATOM   7532  CA  VAL I 276     -49.950 -33.930  70.959  1.00 29.58          D000 C
ATOM   7533  C   VAL I 276     -50.723 -34.063  69.650  1.00 29.99          D000 C
ATOM   7534  O   VAL I 276     -51.233 -35.146  69.335  1.00 32.34          D000 O
ATOM   7535  CB  VAL I 276     -48.666 -34.790  70.916  1.00 34.01          D000 C
ATOM   7536  CG1 VAL I 276     -47.726 -34.358  69.768  1.00 25.34          D000 C
ATOM   7537  CG2 VAL I 276     -47.957 -34.821  72.259  1.00 22.27          D000 C
ATOM   7538  N   CYS I 277     -50.815 -32.973  68.892  1.00 30.05          D000 N
ATOM   7539  CA  CYS I 277     -51.289 -33.032  67.507  1.00 36.56          D000 C
ATOM   7540  C   CYS I 277     -50.113 -33.216  66.544  1.00 39.90          D000 C
ATOM   7541  O   CYS I 277     -49.037 -32.638  66.739  1.00 34.93          D000 O
ATOM   7542  CB  CYS I 277     -52.054 -31.764  67.111  1.00 33.98          D000 C
ATOM   7543  SG  CYS I 277     -53.711 -31.466  67.812  1.00 42.15          D000 S
ATOM   7544  N   GLU I 278     -50.336 -34.004  65.486  1.00 37.84          D000 N
ATOM   7545  CA  GLU I 278     -49.361 -34.207  64.419  1.00 38.01          D000 C
ATOM   7546  C   GLU I 278     -49.980 -33.930  63.054  1.00 40.54          D000 C
ATOM   7547  O   GLU I 278     -51.105 -34.354  62.766  1.00 41.44          D000 O
ATOM   7548  CB  GLU I 278     -48.801 -35.646  64.430  1.00 36.62          D000 C
ATOM   7549  CG  GLU I 278     -47.685 -35.926  63.414  1.00 36.43          D000 C
ATOM   7550  CD  GLU I 278     -47.249 -37.379  63.472  1.00 44.33          D000 C
ATOM   7551  OE1 GLU I 278     -47.852 -38.105  64.290  1.00 43.28          D000 O
ATOM   7552  OE2 GLU I 278     -46.341 -37.806  62.708  1.00 44.12          D000 O1-
ATOM   7553  N   THR I 279     -49.215 -33.280  62.187  1.00 40.86          D000 N
ATOM   7554  CA  THR I 279     -49.596 -33.153  60.789  1.00 45.51          D000 C
ATOM   7555  C   THR I 279     -48.349 -33.290  59.921  1.00 50.70          D000 C
ATOM   7556  O   THR I 279     -47.274 -32.791  60.269  1.00 52.15          D000 O
ATOM   7557  CB  THR I 279     -50.344 -31.830  60.515  1.00 40.68          D000 C
ATOM   7558  OG1 THR I 279     -50.995 -31.916  59.247  1.00 46.37          D000 O
ATOM   7559  CG2 THR I 279     -49.413 -30.622  60.530  1.00 40.76          D000 C
ATOM   7560  N   GLU I 280     -48.502 -33.993  58.801  1.00 51.69          D000 N
ATOM   7561  CA  GLU I 280     -47.419 -34.287  57.872  1.00 49.36          D000 C
ATOM   7562  C   GLU I 280     -47.271 -33.158  56.860  1.00 56.06          D000 C
ATOM   7563  O   GLU I 280     -48.253 -32.530  56.466  1.00 60.37          D000 O
ATOM   7564  CB  GLU I 280     -47.687 -35.615  57.154  1.00 46.67          D000 C
ATOM   7565  CG  GLU I 280     -47.603 -36.893  58.036  1.00 52.72          D000 C
ATOM   7566  CD  GLU I 280     -48.756 -37.083  59.067  1.00 61.50          D000 C
ATOM   7567  OE1 GLU I 280     -49.701 -36.259  59.124  1.00 55.57          D000 O
ATOM   7568  OE2 GLU I 280     -48.724 -38.095  59.816  1.00 65.48          D000 O1-
ATOM   7569  N   LEU I 281     -46.024 -32.894  56.450  1.00 68.28          D000 N
ATOM   7570  CA  LEU I 281     -45.668 -31.688  55.705  1.00 66.92          D000 C
ATOM   7571  C   LEU I 281     -45.742 -31.880  54.192  1.00 77.57          D000 C
ATOM   7572  O   LEU I 281     -45.234 -31.029  53.454  1.00 82.51          D000 O
ATOM   7573  CB  LEU I 281     -44.264 -31.217  56.095  1.00 62.30          D000 C
ATOM   7574  CG  LEU I 281     -44.155 -29.845  56.772  1.00 72.01          D000 C
ATOM   7575  CD1 LEU I 281     -42.720 -29.512  57.120  1.00 70.45          D000 C
ATOM   7576  CD2 LEU I 281     -44.746 -28.748  55.893  1.00 76.75          D000 C
ATOM   7577  N   ASP I 282     -46.399 -32.958  53.737  1.00 85.50          D000 N
ATOM   7578  CA  ASP I 282     -46.623 -33.356  52.326  1.00 86.01          D000 C
ATOM   7579  C   ASP I 282     -45.384 -34.002  51.715  1.00 79.75          D000 C
ATOM   7580  O   ASP I 282     -45.258 -35.228  51.711  1.00 78.04          D000 O
ATOM   7581  CB  ASP I 282     -47.092 -32.180  51.440  1.00 96.47          D000 C
ATOM   7582  CG  ASP I 282     -48.461 -31.617  51.857  1.00 96.65          D000 C
ATOM   7583  OD1 ASP I 282     -48.811 -31.701  53.055  1.00 90.88          D000 O
ATOM   7584  OD2 ASP I 282     -49.190 -31.087  50.977  1.00 93.64          D000 O1-
TER
ATOM   7585  N   THR P 152     -18.008 -35.866  53.223  1.00 83.74          D000 N
ATOM   7586  CA  THR P 152     -19.403 -36.301  53.203  1.00 93.67          D000 C
ATOM   7587  C   THR P 152     -19.882 -36.656  54.610  1.00 97.43          D000 C
ATOM   7588  O   THR P 152     -21.055 -36.465  54.949  1.00 99.74          D000 O
ATOM   7589  CB  THR P 152     -19.616 -37.526  52.274  1.00 95.00          D000 C
ATOM   7590  OG1 THR P 152     -18.515 -38.434  52.412  1.00 96.24          D000 O
```

| | | | | | | | | | | | | |
|---|---|---|---|---|---|---|---|---|---|---|---|---|
| ATOM | 7591 | CG2 | THR | P | 152 | -19.745 | -37.100 | 50.820 | 1.00 | 89.07 | D000 | C |
| ATOM | 7592 | N | CYS | P | 153 | -18.962 | -37.176 | 55.422 | 1.00 | 93.53 | D000 | N |
| ATOM | 7593 | CA | CYS | P | 153 | -19.248 | -37.564 | 56.798 | 1.00 | 86.06 | D000 | C |
| ATOM | 7594 | C | CYS | P | 153 | -18.009 | -37.320 | 57.642 | 1.00 | 73.09 | D000 | C |
| ATOM | 7595 | O | CYS | P | 153 | -16.902 | -37.129 | 57.131 | 1.00 | 68.83 | D000 | O |
| ATOM | 7596 | CB | CYS | P | 153 | -19.718 | -39.025 | 56.941 | 1.00 | 85.43 | D000 | C |
| ATOM | 7597 | SG | CYS | P | 153 | -21.535 | -39.281 | 56.843 | 1.00 | 98.27 | D000 | S |
| ATOM | 7598 | N | CYS | P | 154 | -18.209 | -37.292 | 58.847 | 1.00 | 71.85 | D000 | N |
| ATOM | 7599 | CA | CYS | P | 154 | -17.147 | -37.059 | 59.803 | 1.00 | 58.19 | D000 | C |
| ATOM | 7600 | C | CYS | P | 154 | -16.521 | -38.377 | 60.251 | 1.00 | 53.75 | D000 | C |
| ATOM | 7601 | O | CYS | P | 154 | -17.198 | -39.409 | 60.291 | 1.00 | 57.74 | D000 | O |
| ATOM | 7602 | CB | CYS | P | 154 | -17.697 | -36.308 | 61.010 | 1.00 | 51.76 | D000 | C |
| ATOM | 7603 | SG | CYS | P | 154 | -18.273 | -34.615 | 60.600 | 1.00 | 60.05 | D000 | S |
| ATOM | 7604 | N | PRO | P | 155 | -15.231 | -38.360 | 60.584 | 1.00 | 46.56 | D000 | N |
| ATOM | 7605 | CA | PRO | P | 155 | -14.547 | -39.591 | 61.004 | 1.00 | 44.27 | D000 | C |
| ATOM | 7606 | C | PRO | P | 155 | -15.153 | -40.210 | 62.258 | 1.00 | 48.95 | D000 | C |
| ATOM | 7607 | O | PRO | P | 155 | -15.934 | -39.588 | 62.987 | 1.00 | 49.26 | D000 | O |
| ATOM | 7608 | CB | PRO | P | 155 | -13.109 | -39.126 | 61.268 | 1.00 | 42.58 | D000 | C |
| ATOM | 7609 | CG | PRO | P | 155 | -12.971 | -37.842 | 60.535 | 1.00 | 40.45 | D000 | C |
| ATOM | 7610 | CD | PRO | P | 155 | -14.317 | -37.209 | 60.522 | 1.00 | 44.50 | D000 | C |
| ATOM | 7611 | N | VAL | P | 156 | -14.786 | -41.475 | 62.492 | 1.00 | 50.73 | D000 | N |
| ATOM | 7612 | CA | VAL | P | 156 | -15.268 | -42.191 | 63.670 | 1.00 | 48.55 | D000 | C |
| ATOM | 7613 | C | VAL | P | 156 | -14.926 | -41.390 | 64.914 | 1.00 | 49.57 | D000 | C |
| ATOM | 7614 | O | VAL | P | 156 | -13.791 | -40.923 | 65.071 | 1.00 | 49.11 | D000 | O |
| ATOM | 7615 | CB | VAL | P | 156 | -14.658 | -43.599 | 63.733 | 1.00 | 49.04 | D000 | C |
| ATOM | 7616 | CG1 | VAL | P | 156 | -15.733 | -44.639 | 63.993 | 1.00 | 37.71 | D000 | C |
| ATOM | 7617 | CG2 | VAL | P | 156 | -13.879 | -43.914 | 62.449 | 1.00 | 56.31 | D000 | C |
| ATOM | 7618 | N | ASN | P | 157 | -15.919 | -41.205 | 65.791 | 1.00 | 49.29 | D000 | N |
| ATOM | 7619 | CA | ASN | P | 157 | -15.831 | -40.490 | 67.074 | 1.00 | 45.69 | D000 | C |
| ATOM | 7620 | C | ASN | P | 157 | -15.791 | -38.976 | 66.917 | 1.00 | 42.84 | D000 | C |
| ATOM | 7621 | O | ASN | P | 157 | -15.571 | -38.265 | 67.913 | 1.00 | 42.59 | D000 | O |
| ATOM | 7622 | CB | ASN | P | 157 | -14.592 | -40.896 | 67.878 | 1.00 | 46.95 | D000 | C |
| ATOM | 7623 | CG | ASN | P | 157 | -14.548 | -42.372 | 68.177 | 1.00 | 48.62 | D000 | C |
| ATOM | 7624 | OD1 | ASN | P | 157 | -15.544 | -42.976 | 68.582 | 1.00 | 51.67 | D000 | O |
| ATOM | 7625 | ND2 | ASN | P | 157 | -13.396 | -42.973 | 67.937 | 1.00 | 38.36 | D000 | N |
| ATOM | 7626 | N | TRP | P | 158 | -16.009 | -38.453 | 65.719 | 1.00 | 42.42 | D000 | N |
| ATOM | 7627 | CA | TRP | P | 158 | -16.226 | -37.030 | 65.523 | 1.00 | 41.39 | D000 | C |
| ATOM | 7628 | C | TRP | P | 158 | -17.714 | -36.789 | 65.274 | 1.00 | 42.54 | D000 | C |
| ATOM | 7629 | O | TRP | P | 158 | -18.454 | -37.697 | 64.895 | 1.00 | 47.35 | D000 | O |
| ATOM | 7630 | CB | TRP | P | 158 | -15.395 | -36.490 | 64.348 | 1.00 | 38.79 | D000 | C |
| ATOM | 7631 | CG | TRP | P | 158 | -13.877 | -36.545 | 64.488 | 1.00 | 37.39 | D000 | C |
| ATOM | 7632 | CD1 | TRP | P | 158 | -13.107 | -37.655 | 64.704 | 1.00 | 39.44 | D000 | C |
| ATOM | 7633 | CD2 | TRP | P | 158 | -12.955 | -35.444 | 64.366 | 1.00 | 35.99 | D000 | C |
| ATOM | 7634 | NE1 | TRP | P | 158 | -11.763 | -37.316 | 64.728 | 1.00 | 31.69 | D000 | N |
| ATOM | 7635 | CE2 | TRP | P | 158 | -11.644 | -35.969 | 64.524 | 1.00 | 34.45 | D000 | C |
| ATOM | 7636 | CE3 | TRP | P | 158 | -13.107 | -34.070 | 64.140 | 1.00 | 35.66 | D000 | C |
| ATOM | 7637 | CZ2 | TRP | P | 158 | -10.501 | -35.165 | 64.471 | 1.00 | 30.75 | D000 | C |
| ATOM | 7638 | CZ3 | TRP | P | 158 | -11.970 | -33.273 | 64.083 | 1.00 | 35.96 | D000 | C |
| ATOM | 7639 | CH2 | TRP | P | 158 | -10.681 | -33.827 | 64.247 | 1.00 | 31.46 | D000 | C |
| ATOM | 7640 | N | VAL | P | 159 | -18.149 | -35.554 | 65.509 | 1.00 | 37.16 | D000 | N |
| ATOM | 7641 | CA | VAL | P | 159 | -19.556 | -35.188 | 65.499 | 1.00 | 34.88 | D000 | C |
| ATOM | 7642 | C | VAL | P | 159 | -19.788 | -34.115 | 64.443 | 1.00 | 38.07 | D000 | C |
| ATOM | 7643 | O | VAL | P | 159 | -19.116 | -33.083 | 64.447 | 1.00 | 39.78 | D000 | O |
| ATOM | 7644 | CB | VAL | P | 159 | -19.996 | -34.693 | 66.885 | 1.00 | 37.29 | D000 | C |
| ATOM | 7645 | CG1 | VAL | P | 159 | -21.462 | -34.286 | 66.872 | 1.00 | 33.76 | D000 | C |
| ATOM | 7646 | CG2 | VAL | P | 159 | -19.717 | -35.762 | 67.920 | 1.00 | 35.15 | D000 | C |
| ATOM | 7647 | N | GLU | P | 160 | -20.747 | -34.346 | 63.548 | 1.00 | 44.59 | D000 | N |
| ATOM | 7648 | CA | GLU | P | 160 | -21.060 | -33.351 | 62.529 | 1.00 | 41.41 | D000 | C |
| ATOM | 7649 | C | GLU | P | 160 | -22.016 | -32.294 | 63.071 | 1.00 | 42.80 | D000 | C |
| ATOM | 7650 | O | GLU | P | 160 | -22.890 | -32.577 | 63.894 | 1.00 | 40.17 | D000 | O |
| ATOM | 7651 | CB | GLU | P | 160 | -21.661 | -34.005 | 61.284 | 1.00 | 46.52 | D000 | C |
| ATOM | 7652 | CG | GLU | P | 160 | -21.512 | -33.122 | 60.047 | 1.00 | 56.68 | D000 | C |
| ATOM | 7653 | CD | GLU | P | 160 | -22.207 | -33.644 | 58.800 | 1.00 | 66.43 | D000 | C |
| ATOM | 7654 | OE1 | GLU | P | 160 | -22.148 | -34.876 | 58.533 | 1.00 | 67.75 | D000 | O |
| ATOM | 7655 | OE2 | GLU | P | 160 | -22.785 | -32.796 | 58.074 | 1.00 | 67.82 | D000 | O1- |
| ATOM | 7656 | N | HIS | P | 161 | -21.824 | -31.060 | 62.622 | 1.00 | 42.59 | D000 | N |
| ATOM | 7657 | CA | HIS | P | 161 | -22.738 | -29.980 | 62.952 | 1.00 | 40.24 | D000 | C |
| ATOM | 7658 | C | HIS | P | 161 | -22.456 | -28.824 | 62.011 | 1.00 | 43.60 | D000 | C |
| ATOM | 7659 | O | HIS | P | 161 | -21.382 | -28.220 | 62.096 | 1.00 | 45.56 | D000 | O |
| ATOM | 7660 | CB | HIS | P | 161 | -22.588 | -29.557 | 64.413 | 1.00 | 35.89 | D000 | C |
| ATOM | 7661 | CG | HIS | P | 161 | -23.438 | -28.383 | 64.790 | 1.00 | 44.75 | D000 | C |

```
ATOM   7662  ND1 HIS P 161     -24.736 -28.521  65.238  1.00 41.80       D000 N
ATOM   7663  CD2 HIS P 161     -23.178 -27.050  64.788  1.00 44.31       D000 C
ATOM   7664  CE1 HIS P 161     -25.239 -27.324  65.496  1.00 44.21       D000 C
ATOM   7665  NE2 HIS P 161     -24.315 -26.415  65.231  1.00 47.45       D000 N
ATOM   7666  N   GLU P 162     -23.391 -28.535  61.098  1.00 46.24       D000 N
ATOM   7667  CA  GLU P 162     -23.294 -27.382  60.201  1.00 47.89       D000 C
ATOM   7668  C   GLU P 162     -22.059 -27.512  59.301  1.00 50.82       D000 C
ATOM   7669  O   GLU P 162     -21.272 -26.573  59.127  1.00 44.74       D000 O
ATOM   7670  CB  GLU P 162     -23.249 -26.082  61.009  1.00 53.01       D000 C
ATOM   7671  CG  GLU P 162     -24.485 -25.787  61.849  1.00 58.08       D000 C
ATOM   7672  CD  GLU P 162     -25.529 -24.958  61.127  1.00 70.25       D000 C
ATOM   7673  OE1 GLU P 162     -25.288 -23.740  60.921  1.00 71.49       D000 O
ATOM   7674  OE2 GLU P 162     -26.575 -25.529  60.746  1.00 76.35       D000 O1-
ATOM   7675  N   ARG P 163     -21.884 -28.714  58.744  1.00 48.09       D000 N
ATOM   7676  CA  ARG P 163     -20.780 -29.023  57.836  1.00 57.37       D000 C
ATOM   7677  C   ARG P 163     -19.428 -28.733  58.490  1.00 57.98       D000 C
ATOM   7678  O   ARG P 163     -18.464 -28.324  57.834  1.00 57.75       D000 O
ATOM   7679  CB  ARG P 163     -20.918 -28.307  56.486  1.00 60.45       D000 C
ATOM   7680  CG  ARG P 163     -22.191 -28.694  55.747  1.00 58.49       D000 C
ATOM   7681  CD  ARG P 163     -22.201 -28.270  54.296  1.00 71.93       D000 C
ATOM   7682  NE  ARG P 163     -21.240 -29.050  53.518  1.00 71.00       D000 N
ATOM   7683  CZ  ARG P 163     -20.116 -28.576  52.983  1.00 75.19       D000 C
ATOM   7684  NH1 ARG P 163     -19.331 -29.398  52.298  1.00 77.55       D000 N1+
ATOM   7685  NH2 ARG P 163     -19.773 -27.296  53.117  1.00 67.90       D000 N
ATOM   7686  N   SER P 164     -19.350 -28.961  59.794  1.00 55.73       D000 N
ATOM   7687  CA  SER P 164     -18.096 -28.955  60.520  1.00 45.84       D000 C
ATOM   7688  C   SER P 164     -18.058 -30.235  61.329  1.00 47.92       D000 C
ATOM   7689  O   SER P 164     -19.103 -30.761  61.727  1.00 45.64       D000 O
ATOM   7690  CB  SER P 164     -17.950 -27.732  61.415  1.00 45.00       D000 C
ATOM   7691  OG  SER P 164     -17.414 -26.645  60.673  1.00 53.08       D000 O
ATOM   7692  N   CYS P 165     -16.854 -30.786  61.464  1.00 44.78       D000 N
ATOM   7693  CA  CYS P 165     -16.607 -31.976  62.262  1.00 41.69       D000 C
ATOM   7694  C   CYS P 165     -15.855 -31.593  63.523  1.00 41.12       D000 C
ATOM   7695  O   CYS P 165     -14.843 -30.886  63.464  1.00 42.93       D000 O
ATOM   7696  CB  CYS P 165     -15.813 -33.009  61.472  1.00 49.43       D000 C
ATOM   7697  SG  CYS P 165     -16.607 -33.584  59.964  1.00 54.63       D000 S
ATOM   7698  N   TYR P 166     -16.342 -32.073  64.651  1.00 38.95       D000 N
ATOM   7699  CA  TYR P 166     -15.815 -31.709  65.949  1.00 35.52       D000 C
ATOM   7700  C   TYR P 166     -15.385 -32.965  66.674  1.00 36.03       D000 C
ATOM   7701  O   TYR P 166     -16.004 -34.021  66.524  1.00 35.78       D000 O
ATOM   7702  CB  TYR P 166     -16.857 -30.955  66.776  1.00 34.96       D000 C
ATOM   7703  CG  TYR P 166     -17.320 -29.668  66.153  1.00 36.68       D000 C
ATOM   7704  CD1 TYR P 166     -18.334 -29.650  65.195  1.00 32.91       D000 C
ATOM   7705  CD2 TYR P 166     -16.732 -28.461  66.513  1.00 33.68       D000 C
ATOM   7706  CE1 TYR P 166     -18.753 -28.447  64.630  1.00 36.09       D000 C
ATOM   7707  CE2 TYR P 166     -17.141 -27.259  65.953  1.00 30.23       D000 C
ATOM   7708  CZ  TYR P 166     -18.149 -27.251  65.019  1.00 35.59       D000 C
ATOM   7709  OH  TYR P 166     -18.541 -26.044  64.482  1.00 35.19       D000 O
ATOM   7710  N   TRP P 167     -14.307 -32.851  67.435  1.00 34.48       D000 N
ATOM   7711  CA  TRP P 167     -13.853 -33.934  68.284  1.00 32.86       D000 C
ATOM   7712  C   TRP P 167     -13.586 -33.377  69.678  1.00 34.10       D000 C
ATOM   7713  O   TRP P 167     -12.924 -32.339  69.820  1.00 34.23       D000 O
ATOM   7714  CB  TRP P 167     -12.613 -34.593  67.695  1.00 31.80       D000 C
ATOM   7715  CG  TRP P 167     -12.104 -35.731  68.511  1.00 33.09       D000 C
ATOM   7716  CD1 TRP P 167     -12.489 -37.025  68.430  1.00 33.37       D000 C
ATOM   7717  CD2 TRP P 167     -11.128 -35.664  69.554  1.00 28.44       D000 C
ATOM   7718  NE1 TRP P 167     -11.793 -37.780  69.336  1.00 29.92       D000 N
ATOM   7719  CE2 TRP P 167     -10.948 -36.966  70.036  1.00 29.30       D000 C
ATOM   7720  CE3 TRP P 167     -10.351 -34.629  70.091  1.00 31.68       D000 C
ATOM   7721  CZ2 TRP P 167     -10.034 -37.270  71.048  1.00 34.20       D000 C
ATOM   7722  CZ3 TRP P 167      -9.442 -34.929  71.100  1.00 32.44       D000 C
ATOM   7723  CH2 TRP P 167      -9.294 -36.236  71.570  1.00 30.38       D000 C
ATOM   7724  N   PHE P 168     -14.102 -34.066  70.699  1.00 30.71       D000 N
ATOM   7725  CA  PHE P 168     -14.055 -33.612  72.088  1.00 32.96       D000 C
ATOM   7726  C   PHE P 168     -13.115 -34.503  72.884  1.00 29.97       D000 C
ATOM   7727  O   PHE P 168     -13.354 -35.706  73.001  1.00 33.55       D000 O
ATOM   7728  CB  PHE P 168     -15.460 -33.603  72.691  1.00 26.93       D000 C
ATOM   7729  CG  PHE P 168     -16.381 -32.711  71.950  1.00 26.93       D000 C
ATOM   7730  CD1 PHE P 168     -16.509 -31.381  72.314  1.00 25.66       D000 C
ATOM   7731  CD2 PHE P 168     -17.057 -33.170  70.831  1.00 28.28       D000 C
ATOM   7732  CE1 PHE P 168     -17.335 -30.515  71.594  1.00 29.79       D000 C
```

| | | | | | | | | | | | | |
|---|---|---|---|---|---|---|---|---|---|---|---|---|
| ATOM | 7733 | CE2 | PHE | P | 168 | -17.877 | -32.328 | 70.108 | 1.00 | 30.00 | D000 | C |
| ATOM | 7734 | CZ | PHE | P | 168 | -18.025 | -30.988 | 70.490 | 1.00 | 26.93 | D000 | C |
| ATOM | 7735 | N | SER | P | 169 | -12.026 | -33.922 | 73.385 | 1.00 | 30.64 | D000 | N |
| ATOM | 7736 | CA | SER | P | 169 | -11.061 | -34.707 | 74.145 | 1.00 | 34.97 | D000 | C |
| ATOM | 7737 | C | SER | P | 169 | -11.675 | -35.155 | 75.471 | 1.00 | 27.83 | D000 | C |
| ATOM | 7738 | O | SER | P | 169 | -12.547 | -34.495 | 76.036 | 1.00 | 28.09 | D000 | O |
| ATOM | 7739 | CB | SER | P | 169 | -9.761 | -33.908 | 74.389 | 1.00 | 29.33 | D000 | C |
| ATOM | 7740 | OG | SER | P | 169 | -9.906 | -32.926 | 75.407 | 1.00 | 28.03 | D000 | O |
| ATOM | 7741 | N | ARG | P | 170 | -11.219 | -36.298 | 75.960 | 1.00 | 26.00 | D000 | N |
| ATOM | 7742 | CA | ARG | P | 170 | -11.564 | -36.758 | 77.295 | 1.00 | 31.89 | D000 | C |
| ATOM | 7743 | C | ARG | P | 170 | -10.359 | -36.735 | 78.233 | 1.00 | 33.73 | D000 | C |
| ATOM | 7744 | O | ARG | P | 170 | -10.416 | -37.279 | 79.337 | 1.00 | 35.93 | D000 | O |
| ATOM | 7745 | CB | ARG | P | 170 | -12.183 | -38.148 | 77.207 | 1.00 | 26.68 | D000 | C |
| ATOM | 7746 | CG | ARG | P | 170 | -12.874 | -38.607 | 78.430 | 1.00 | 33.68 | D000 | C |
| ATOM | 7747 | CD | ARG | P | 170 | -14.298 | -38.263 | 78.356 | 1.00 | 32.40 | D000 | C |
| ATOM | 7748 | NE | ARG | P | 170 | -15.029 | -38.782 | 79.492 | 1.00 | 28.94 | D000 | N |
| ATOM | 7749 | CZ | ARG | P | 170 | -15.923 | -38.060 | 80.155 | 1.00 | 29.78 | D000 | C |
| ATOM | 7750 | NH1 | ARG | P | 170 | -16.586 | -38.582 | 81.182 | 1.00 | 24.35 | D000 | N1+ |
| ATOM | 7751 | NH2 | ARG | P | 170 | -16.137 | -36.805 | 79.779 | 1.00 | 23.51 | D000 | N |
| ATOM | 7752 | N | SER | P | 171 | -9.256 | -36.149 | 77.801 | 1.00 | 29.68 | D000 | N |
| ATOM | 7753 | CA | SER | P | 171 | -8.088 | -35.943 | 78.633 | 1.00 | 26.30 | D000 | C |
| ATOM | 7754 | C | SER | P | 171 | -7.684 | -34.481 | 78.520 | 1.00 | 31.62 | D000 | C |
| ATOM | 7755 | O | SER | P | 171 | -8.299 | -33.707 | 77.774 | 1.00 | 33.43 | D000 | O |
| ATOM | 7756 | CB | SER | P | 171 | -6.959 | -36.886 | 78.220 | 1.00 | 26.16 | D000 | C |
| ATOM | 7757 | OG | SER | P | 171 | -6.679 | -36.700 | 76.857 | 1.00 | 32.31 | D000 | O |
| ATOM | 7758 | N | GLY | P | 172 | -6.662 | -34.095 | 79.290 | 1.00 | 28.87 | D000 | N |
| ATOM | 7759 | CA | GLY | P | 172 | -6.215 | -32.713 | 79.329 | 1.00 | 27.34 | D000 | C |
| ATOM | 7760 | C | GLY | P | 172 | -4.841 | -32.482 | 78.725 | 1.00 | 28.11 | D000 | C |
| ATOM | 7761 | O | GLY | P | 172 | -4.034 | -33.407 | 78.627 | 1.00 | 29.36 | D000 | O |
| ATOM | 7762 | N | LYS | P | 173 | -4.567 | -31.249 | 78.312 | 1.00 | 26.28 | D000 | N |
| ATOM | 7763 | CA | LYS | P | 173 | -3.311 | -30.891 | 77.669 | 1.00 | 28.99 | D000 | C |
| ATOM | 7764 | C | LYS | P | 173 | -3.133 | -29.390 | 77.801 | 1.00 | 31.16 | D000 | C |
| ATOM | 7765 | O | LYS | P | 173 | -4.101 | -28.640 | 77.686 | 1.00 | 26.89 | D000 | O |
| ATOM | 7766 | CB | LYS | P | 173 | -3.274 | -31.249 | 76.168 | 1.00 | 29.64 | D000 | C |
| ATOM | 7767 | CG | LYS | P | 173 | -3.023 | -32.712 | 75.792 | 1.00 | 25.52 | D000 | C |
| ATOM | 7768 | CD | LYS | P | 173 | -2.577 | -32.794 | 74.349 | 1.00 | 26.55 | D000 | C |
| ATOM | 7769 | CE | LYS | P | 173 | -2.684 | -34.206 | 73.811 | 1.00 | 33.01 | D000 | C |
| ATOM | 7770 | NZ | LYS | P | 173 | -1.755 | -35.154 | 74.459 | 1.00 | 35.05 | D000 | N1+ |
| ATOM | 7771 | N | ALA | P | 174 | -1.890 | -28.962 | 78.015 | 1.00 | 32.75 | D000 | N |
| ATOM | 7772 | CA | ALA | P | 174 | -1.556 | -27.556 | 77.874 | 1.00 | 30.91 | D000 | C |
| ATOM | 7773 | C | ALA | P | 174 | -1.960 | -27.069 | 76.483 | 1.00 | 32.66 | D000 | C |
| ATOM | 7774 | O | ALA | P | 174 | -1.967 | -27.838 | 75.511 | 1.00 | 28.87 | D000 | O |
| ATOM | 7775 | CB | ALA | P | 174 | -0.062 | -27.349 | 78.113 | 1.00 | 32.92 | D000 | C |
| ATOM | 7776 | N | TRP | P | 175 | -2.308 | -25.777 | 76.396 | 1.00 | 31.13 | D000 | N |
| ATOM | 7777 | CA | TRP | P | 175 | -2.873 | -25.232 | 75.157 | 1.00 | 35.20 | D000 | C |
| ATOM | 7778 | C | TRP | P | 175 | -1.991 | -25.526 | 73.942 | 1.00 | 35.94 | D000 | C |
| ATOM | 7779 | O | TRP | P | 175 | -2.491 | -25.919 | 72.884 | 1.00 | 31.80 | D000 | O |
| ATOM | 7780 | CB | TRP | P | 175 | -3.105 | -23.719 | 75.294 | 1.00 | 35.59 | D000 | C |
| ATOM | 7781 | CG | TRP | P | 175 | -3.896 | -23.089 | 74.131 | 1.00 | 42.77 | D000 | C |
| ATOM | 7782 | CD1 | TRP | P | 175 | -5.235 | -22.812 | 74.113 | 1.00 | 41.04 | D000 | C |
| ATOM | 7783 | CD2 | TRP | P | 175 | -3.387 | -22.650 | 72.846 | 1.00 | 48.23 | D000 | C |
| ATOM | 7784 | NE1 | TRP | P | 175 | -5.594 | -22.244 | 72.908 | 1.00 | 42.82 | D000 | N |
| ATOM | 7785 | CE2 | TRP | P | 175 | -4.483 | -22.130 | 72.115 | 1.00 | 46.08 | D000 | C |
| ATOM | 7786 | CE3 | TRP | P | 175 | -2.117 | -22.651 | 72.244 | 1.00 | 45.34 | D000 | C |
| ATOM | 7787 | CZ2 | TRP | P | 175 | -4.348 | -21.616 | 70.816 | 1.00 | 42.61 | D000 | C |
| ATOM | 7788 | CZ3 | TRP | P | 175 | -1.988 | -22.133 | 70.948 | 1.00 | 42.70 | D000 | C |
| ATOM | 7789 | CH2 | TRP | P | 175 | -3.095 | -21.626 | 70.256 | 1.00 | 41.85 | D000 | C |
| ATOM | 7790 | N | ALA | P | 176 | -0.677 | -25.331 | 74.076 | 1.00 | 34.88 | D000 | N |
| ATOM | 7791 | CA | ALA | P | 176 | 0.226 | -25.561 | 72.957 | 1.00 | 30.60 | D000 | C |
| ATOM | 7792 | C | ALA | P | 176 | 0.226 | -27.022 | 72.528 | 1.00 | 36.67 | D000 | C |
| ATOM | 7793 | O | ALA | P | 176 | 0.291 | -27.325 | 71.328 | 1.00 | 33.09 | D000 | O |
| ATOM | 7794 | CB | ALA | P | 176 | 1.632 | -25.111 | 73.326 | 1.00 | 30.07 | D000 | C |
| ATOM | 7795 | N | ASP | P | 177 | 0.176 | -27.949 | 73.490 | 1.00 | 34.27 | D000 | N |
| ATOM | 7796 | CA | ASP | P | 177 | 0.168 | -29.353 | 73.103 | 1.00 | 30.80 | D000 | C |
| ATOM | 7797 | C | ASP | P | 177 | -1.161 | -29.738 | 72.466 | 1.00 | 29.53 | D000 | C |
| ATOM | 7798 | O | ASP | P | 177 | -1.190 | -30.546 | 71.540 | 1.00 | 28.69 | D000 | O |
| ATOM | 7799 | CB | ASP | P | 177 | 0.466 | -30.238 | 74.315 | 1.00 | 33.82 | D000 | C |
| ATOM | 7800 | CG | ASP | P | 177 | 1.804 | -29.908 | 74.967 | 1.00 | 40.40 | D000 | C |
| ATOM | 7801 | OD1 | ASP | P | 177 | 2.724 | -29.448 | 74.243 | 1.00 | 40.31 | D000 | O |
| ATOM | 7802 | OD2 | ASP | P | 177 | 1.934 | -30.111 | 76.206 | 1.00 | 43.49 | D000 | O1- |
| ATOM | 7803 | N | ALA | P | 178 | -2.267 | -29.156 | 72.931 | 1.00 | 33.12 | D000 | N |

| | | | | | | | | | | | | |
|---|---|---|---|---|---|---|---|---|---|---|---|---|
| ATOM | 7804 | CA | ALA | P | 178 | -3.558 | -29.421 | 72.307 | 1.00 | 31.89 | D000 | C |
| ATOM | 7805 | C | ALA | P | 178 | -3.617 | -28.832 | 70.899 | 1.00 | 35.82 | D000 | C |
| ATOM | 7806 | O | ALA | P | 178 | -4.104 | -29.487 | 69.969 | 1.00 | 35.46 | D000 | O |
| ATOM | 7807 | CB | ALA | P | 178 | -4.690 | -28.874 | 73.178 | 1.00 | 25.57 | D000 | C |
| ATOM | 7808 | N | ASP | P | 179 | -3.149 | -27.588 | 70.741 | 1.00 | 33.89 | D000 | N |
| ATOM | 7809 | CA | ASP | P | 179 | -2.957 | -26.971 | 69.429 | 1.00 | 35.32 | D000 | C |
| ATOM | 7810 | C | ASP | P | 179 | -2.204 | -27.905 | 68.481 | 1.00 | 38.70 | D000 | C |
| ATOM | 7811 | O | ASP | P | 179 | -2.660 | -28.182 | 67.365 | 1.00 | 37.15 | D000 | O |
| ATOM | 7812 | CB | ASP | P | 179 | -2.188 | -25.656 | 69.607 | 1.00 | 36.39 | D000 | C |
| ATOM | 7813 | CG | ASP | P | 179 | -2.089 | -24.840 | 68.331 | 1.00 | 45.78 | D000 | C |
| ATOM | 7814 | OD1 | ASP | P | 179 | -2.995 | -24.936 | 67.468 | 1.00 | 47.95 | D000 | O |
| ATOM | 7815 | OD2 | ASP | P | 179 | -1.093 | -24.096 | 68.191 | 1.00 | 50.66 | D000 | O1- |
| ATOM | 7816 | N | ASN | P | 180 | -1.049 | -28.410 | 68.918 | 1.00 | 32.54 | D000 | N |
| ATOM | 7817 | CA | ASN | P | 180 | -0.271 | -29.285 | 68.052 | 1.00 | 37.32 | D000 | C |
| ATOM | 7818 | C | ASN | P | 180 | -1.019 | -30.582 | 67.794 | 1.00 | 38.98 | D000 | C |
| ATOM | 7819 | O | ASN | P | 180 | -0.911 | -31.163 | 66.707 | 1.00 | 43.91 | D000 | O |
| ATOM | 7820 | CB | ASN | P | 180 | 1.118 | -29.563 | 68.660 | 1.00 | 33.88 | D000 | C |
| ATOM | 7821 | CG | ASN | P | 180 | 2.023 | -28.309 | 68.713 | 1.00 | 36.32 | D000 | C |
| ATOM | 7822 | OD1 | ASN | P | 180 | 1.696 | -27.243 | 68.174 | 1.00 | 45.19 | D000 | O |
| ATOM | 7823 | ND2 | ASN | P | 180 | 3.160 | -28.445 | 69.372 | 1.00 | 35.02 | D000 | N |
| ATOM | 7824 | N | TYR | P | 181 | -1.771 | -31.060 | 68.782 | 1.00 | 39.97 | D000 | N |
| ATOM | 7825 | CA | TYR | P | 181 | -2.544 | -32.280 | 68.589 | 1.00 | 39.12 | D000 | C |
| ATOM | 7826 | C | TYR | P | 181 | -3.578 | -32.097 | 67.471 | 1.00 | 38.18 | D000 | C |
| ATOM | 7827 | O | TYR | P | 181 | -3.740 | -32.963 | 66.601 | 1.00 | 35.54 | D000 | O |
| ATOM | 7828 | CB | TYR | P | 181 | -3.216 | -32.680 | 69.908 | 1.00 | 26.15 | D000 | C |
| ATOM | 7829 | CG | TYR | P | 181 | -4.103 | -33.884 | 69.760 | 1.00 | 30.23 | D000 | C |
| ATOM | 7830 | CD1 | TYR | P | 181 | -5.415 | -33.752 | 69.303 | 1.00 | 32.63 | D000 | C |
| ATOM | 7831 | CD2 | TYR | P | 181 | -3.642 | -35.158 | 70.052 | 1.00 | 31.11 | D000 | C |
| ATOM | 7832 | CE1 | TYR | P | 181 | -6.223 | -34.846 | 69.131 | 1.00 | 29.23 | D000 | C |
| ATOM | 7833 | CE2 | TYR | P | 181 | -4.463 | -36.267 | 69.898 | 1.00 | 26.83 | D000 | C |
| ATOM | 7834 | CZ | TYR | P | 181 | -5.750 | -36.093 | 69.433 | 1.00 | 27.61 | D000 | C |
| ATOM | 7835 | OH | TYR | P | 181 | -6.587 | -37.157 | 69.283 | 1.00 | 34.40 | D000 | O |
| ATOM | 7836 | N | CYS | P | 182 | -4.291 | -30.973 | 67.476 | 1.00 | 34.18 | D000 | N |
| ATOM | 7837 | CA | CYS | P | 182 | -5.297 | -30.784 | 66.443 | 1.00 | 40.39 | D000 | C |
| ATOM | 7838 | C | CYS | P | 182 | -4.643 | -30.653 | 65.076 | 1.00 | 39.40 | D000 | C |
| ATOM | 7839 | O | CYS | P | 182 | -5.119 | -31.245 | 64.098 | 1.00 | 36.09 | D000 | O |
| ATOM | 7840 | CB | CYS | P | 182 | -6.178 | -29.570 | 66.756 | 1.00 | 38.38 | D000 | C |
| ATOM | 7841 | SG | CYS | P | 182 | -7.302 | -29.774 | 68.185 | 1.00 | 41.03 | D000 | S |
| ATOM | 7842 | N | ARG | P | 183 | -3.541 | -29.899 | 64.997 | 1.00 | 38.60 | D000 | N |
| ATOM | 7843 | CA | ARG | P | 183 | -2.855 | -29.714 | 63.724 | 1.00 | 36.47 | D000 | C |
| ATOM | 7844 | C | ARG | P | 183 | -2.442 | -31.048 | 63.106 | 1.00 | 35.85 | D000 | C |
| ATOM | 7845 | O | ARG | P | 183 | -2.647 | -31.265 | 61.908 | 1.00 | 36.71 | D000 | O |
| ATOM | 7846 | CB | ARG | P | 183 | -1.648 | -28.802 | 63.900 | 1.00 | 36.20 | D000 | C |
| ATOM | 7847 | CG | ARG | P | 183 | -1.953 | -27.334 | 64.191 | 1.00 | 35.03 | D000 | C |
| ATOM | 7848 | CD | ARG | P | 183 | -0.707 | -26.729 | 64.791 | 1.00 | 45.48 | D000 | C |
| ATOM | 7849 | NE | ARG | P | 183 | -0.297 | -25.461 | 64.189 | 1.00 | 63.54 | D000 | N |
| ATOM | 7850 | CZ | ARG | P | 183 | 0.971 | -25.139 | 63.900 | 1.00 | 64.81 | D000 | C |
| ATOM | 7851 | NH1 | ARG | P | 183 | 1.957 | -25.996 | 64.123 | 1.00 | 42.00 | D000 | N1+ |
| ATOM | 7852 | NH2 | ARG | P | 183 | 1.259 | -23.956 | 63.368 | 1.00 | 73.94 | D000 | N |
| ATOM | 7853 | N | LEU | P | 184 | -1.906 | -31.972 | 63.906 | 1.00 | 34.56 | D000 | N |
| ATOM | 7854 | CA | LEU | P | 184 | -1.585 | -33.295 | 63.377 | 1.00 | 34.47 | D000 | C |
| ATOM | 7855 | C | LEU | P | 184 | -2.812 | -34.158 | 63.104 | 1.00 | 43.39 | D000 | C |
| ATOM | 7856 | O | LEU | P | 184 | -2.648 | -35.287 | 62.629 | 1.00 | 48.31 | D000 | O |
| ATOM | 7857 | CB | LEU | P | 184 | -0.669 | -34.073 | 64.311 | 1.00 | 40.32 | D000 | C |
| ATOM | 7858 | CG | LEU | P | 184 | 0.849 | -33.962 | 64.235 | 1.00 | 47.86 | D000 | C |
| ATOM | 7859 | CD1 | LEU | P | 184 | 1.306 | -33.127 | 63.057 | 1.00 | 34.57 | D000 | C |
| ATOM | 7860 | CD2 | LEU | P | 184 | 1.398 | -33.477 | 65.565 | 1.00 | 40.91 | D000 | C |
| ATOM | 7861 | N | GLU | P | 185 | -4.016 | -33.714 | 63.463 | 1.00 | 43.99 | D000 | N |
| ATOM | 7862 | CA | GLU | P | 185 | -5.220 | -34.401 | 63.011 | 1.00 | 42.68 | D000 | C |
| ATOM | 7863 | C | GLU | P | 185 | -5.746 | -33.822 | 61.714 | 1.00 | 42.10 | D000 | C |
| ATOM | 7864 | O | GLU | P | 185 | -6.877 | -34.117 | 61.325 | 1.00 | 41.36 | D000 | O |
| ATOM | 7865 | CB | GLU | P | 185 | -6.314 | -34.365 | 64.076 | 1.00 | 40.55 | D000 | C |
| ATOM | 7866 | CG | GLU | P | 185 | -5.941 | -35.182 | 65.257 | 1.00 | 41.11 | D000 | C |
| ATOM | 7867 | CD | GLU | P | 185 | -5.903 | -36.635 | 64.898 | 1.00 | 46.25 | D000 | C |
| ATOM | 7868 | OE1 | GLU | P | 185 | -6.722 | -37.051 | 64.048 | 1.00 | 49.14 | D000 | O |
| ATOM | 7869 | OE2 | GLU | P | 185 | -5.032 | -37.350 | 65.434 | 1.00 | 47.23 | D000 | O1- |
| ATOM | 7870 | N | ASP | P | 186 | -4.940 | -33.010 | 61.039 | 1.00 | 47.67 | D000 | N |
| ATOM | 7871 | CA | ASP | P | 186 | -5.390 | -32.232 | 59.896 | 1.00 | 45.07 | D000 | C |
| ATOM | 7872 | C | ASP | P | 186 | -6.621 | -31.416 | 60.279 | 1.00 | 43.94 | D000 | C |
| ATOM | 7873 | O | ASP | P | 186 | -7.601 | -31.332 | 59.539 | 1.00 | 49.84 | D000 | O |
| ATOM | 7874 | CB | ASP | P | 186 | -5.656 | -33.149 | 58.700 | 1.00 | 47.50 | D000 | C |

| | | | | | | | | | | | | |
|---|---|---|---|---|---|---|---|---|---|---|---|---|
| ATOM | 7875 | CG | ASP | P | 186 | -5.429 | -32.459 | 57.365 | 1.00 | 55.72 | D000 | C |
| ATOM | 7876 | OD1 | ASP | P | 186 | -5.209 | -31.227 | 57.346 | 1.00 | 54.34 | D000 | O |
| ATOM | 7877 | OD2 | ASP | P | 186 | -5.476 | -33.160 | 56.329 | 1.00 | 65.88 | D000 | O1- |
| ATOM | 7878 | N | ALA | P | 187 | -6.558 | -30.797 | 61.453 | 1.00 | 43.57 | D000 | N |
| ATOM | 7879 | CA | ALA | P | 187 | -7.683 | -30.054 | 62.009 | 1.00 | 40.06 | D000 | C |
| ATOM | 7880 | C | ALA | P | 187 | -7.136 | -28.872 | 62.799 | 1.00 | 39.06 | D000 | C |
| ATOM | 7881 | O | ALA | P | 187 | -5.965 | -28.502 | 62.673 | 1.00 | 41.54 | D000 | O |
| ATOM | 7882 | CB | ALA | P | 187 | -8.559 | -30.971 | 62.871 | 1.00 | 37.26 | D000 | C |
| ATOM | 7883 | N | HIS | P | 188 | -7.984 | -28.270 | 63.620 | 1.00 | 38.84 | D000 | N |
| ATOM | 7884 | CA | HIS | P | 188 | -7.523 | -27.141 | 64.411 | 1.00 | 42.15 | D000 | C |
| ATOM | 7885 | C | HIS | P | 188 | -8.399 | -26.992 | 65.655 | 1.00 | 44.84 | D000 | C |
| ATOM | 7886 | O | HIS | P | 188 | -9.536 | -27.477 | 65.704 | 1.00 | 37.93 | D000 | O |
| ATOM | 7887 | CB | HIS | P | 188 | -7.528 | -25.863 | 63.577 | 1.00 | 38.38 | D000 | C |
| ATOM | 7888 | CG | HIS | P | 188 | -8.885 | -25.488 | 63.084 | 1.00 | 39.56 | D000 | C |
| ATOM | 7889 | ND1 | HIS | P | 188 | -9.637 | -24.489 | 63.663 | 1.00 | 42.56 | D000 | N |
| ATOM | 7890 | CD2 | HIS | P | 188 | -9.644 | -26.008 | 62.092 | 1.00 | 38.88 | D000 | C |
| ATOM | 7891 | CE1 | HIS | P | 188 | -10.793 | -24.394 | 63.034 | 1.00 | 45.45 | D000 | C |
| ATOM | 7892 | NE2 | HIS | P | 188 | -10.822 | -25.305 | 62.075 | 1.00 | 47.23 | D000 | N |
| ATOM | 7893 | N | LEU | P | 189 | -7.834 | -26.338 | 66.673 | 1.00 | 39.67 | D000 | N |
| ATOM | 7894 | CA | LEU | P | 189 | -8.599 | -26.011 | 67.860 | 1.00 | 36.17 | D000 | C |
| ATOM | 7895 | C | LEU | P | 189 | -9.829 | -25.215 | 67.464 | 1.00 | 35.83 | D000 | C |
| ATOM | 7896 | O | LEU | P | 189 | -9.731 | -24.279 | 66.669 | 1.00 | 34.94 | D000 | O |
| ATOM | 7897 | CB | LEU | P | 189 | -7.733 | -25.219 | 68.832 | 1.00 | 37.51 | D000 | C |
| ATOM | 7898 | CG | LEU | P | 189 | -6.682 | -25.973 | 69.637 | 1.00 | 36.68 | D000 | C |
| ATOM | 7899 | CD1 | LEU | P | 189 | -5.830 | -25.001 | 70.439 | 1.00 | 36.06 | D000 | C |
| ATOM | 7900 | CD2 | LEU | P | 189 | -7.377 | -26.952 | 70.569 | 1.00 | 34.22 | D000 | C |
| ATOM | 7901 | N | VAL | P | 190 | -10.979 | -25.590 | 68.040 | 1.00 | 34.05 | D000 | N |
| ATOM | 7902 | CA | VAL | P | 190 | -12.274 | -25.094 | 67.585 | 1.00 | 34.93 | D000 | C |
| ATOM | 7903 | C | VAL | P | 190 | -12.304 | -23.565 | 67.518 | 1.00 | 35.33 | D000 | C |
| ATOM | 7904 | O | VAL | P | 190 | -11.823 | -22.852 | 68.410 | 1.00 | 34.50 | D000 | O |
| ATOM | 7905 | CB | VAL | P | 190 | -13.400 | -25.641 | 68.486 | 1.00 | 34.45 | D000 | C |
| ATOM | 7906 | CG1 | VAL | P | 190 | -13.221 | -25.185 | 69.924 | 1.00 | 32.88 | D000 | C |
| ATOM | 7907 | CG2 | VAL | P | 190 | -14.740 | -25.157 | 67.990 | 1.00 | 33.98 | D000 | C |
| ATOM | 7908 | N | VAL | P | 191 | -12.889 | -23.066 | 66.438 | 1.00 | 34.01 | D000 | N |
| ATOM | 7909 | CA | VAL | P | 191 | -13.051 | -21.643 | 66.182 | 1.00 | 35.07 | D000 | C |
| ATOM | 7910 | C | VAL | P | 191 | -14.544 | -21.382 | 66.161 | 1.00 | 36.61 | D000 | C |
| ATOM | 7911 | O | VAL | P | 191 | -15.256 | -21.923 | 65.306 | 1.00 | 41.56 | D000 | O |
| ATOM | 7912 | CB | VAL | P | 191 | -12.385 | -21.225 | 64.856 | 1.00 | 35.46 | D000 | C |
| ATOM | 7913 | CG1 | VAL | P | 191 | -12.817 | -19.830 | 64.431 | 1.00 | 35.96 | D000 | C |
| ATOM | 7914 | CG2 | VAL | P | 191 | -10.875 | -21.295 | 64.981 | 1.00 | 36.37 | D000 | C |
| ATOM | 7915 | N | VAL | P | 192 | -15.021 | -20.581 | 67.111 | 1.00 | 35.79 | D000 | N |
| ATOM | 7916 | CA | VAL | P | 192 | -16.450 | -20.417 | 67.355 | 1.00 | 37.67 | D000 | C |
| ATOM | 7917 | C | VAL | P | 192 | -16.893 | -19.094 | 66.754 | 1.00 | 37.75 | D000 | C |
| ATOM | 7918 | O | VAL | P | 192 | -16.442 | -18.024 | 67.186 | 1.00 | 38.75 | D000 | O |
| ATOM | 7919 | CB | VAL | P | 192 | -16.776 | -20.473 | 68.854 | 1.00 | 38.25 | D000 | C |
| ATOM | 7920 | CG1 | VAL | P | 192 | -18.267 | -20.314 | 69.053 | 1.00 | 35.90 | D000 | C |
| ATOM | 7921 | CG2 | VAL | P | 192 | -16.282 | -21.774 | 69.455 | 1.00 | 37.22 | D000 | C |
| ATOM | 7922 | N | THR | P | 193 | -17.795 | -19.159 | 65.778 | 1.00 | 36.90 | D000 | N |
| ATOM | 7923 | CA | THR | P | 193 | -18.151 | -17.982 | 64.995 | 1.00 | 42.26 | D000 | C |
| ATOM | 7924 | C | THR | P | 193 | -19.600 | -17.530 | 65.148 | 1.00 | 41.06 | D000 | C |
| ATOM | 7925 | O | THR | P | 193 | -19.950 | -16.480 | 64.605 | 1.00 | 46.91 | D000 | O |
| ATOM | 7926 | CB | THR | P | 193 | -17.813 | -18.204 | 63.503 | 1.00 | 40.10 | D000 | C |
| ATOM | 7927 | OG1 | THR | P | 193 | -18.471 | -19.382 | 63.012 | 1.00 | 45.59 | D000 | O |
| ATOM | 7928 | CG2 | THR | P | 193 | -16.302 | -18.323 | 63.286 | 1.00 | 36.33 | D000 | C |
| ATOM | 7929 | N | SER | P | 194 | -20.440 | -18.252 | 65.892 | 1.00 | 39.77 | D000 | N |
| ATOM | 7930 | CA | SER | P | 194 | -21.849 | -17.898 | 66.016 | 1.00 | 36.24 | D000 | C |
| ATOM | 7931 | C | SER | P | 194 | -22.416 | -18.395 | 67.342 | 1.00 | 40.36 | D000 | C |
| ATOM | 7932 | O | SER | P | 194 | -21.829 | -19.244 | 68.013 | 1.00 | 39.43 | D000 | O |
| ATOM | 7933 | CB | SER | P | 194 | -22.666 | -18.470 | 64.855 | 1.00 | 32.78 | D000 | C |
| ATOM | 7934 | OG | SER | P | 194 | -22.508 | -19.875 | 64.777 | 1.00 | 40.86 | D000 | O |
| ATOM | 7935 | N | TRP | P | 195 | -23.589 | -17.862 | 67.714 | 1.00 | 44.41 | D000 | N |
| ATOM | 7936 | CA | TRP | P | 195 | -24.275 | -18.381 | 68.894 | 1.00 | 41.04 | D000 | C |
| ATOM | 7937 | C | TRP | P | 195 | -24.665 | -19.830 | 68.684 | 1.00 | 37.41 | D000 | C |
| ATOM | 7938 | O | TRP | P | 195 | -24.633 | -20.639 | 69.618 | 1.00 | 38.72 | D000 | O |
| ATOM | 7939 | CB | TRP | P | 195 | -25.519 | -17.557 | 69.216 | 1.00 | 44.82 | D000 | C |
| ATOM | 7940 | CG | TRP | P | 195 | -25.243 | -16.309 | 70.020 | 1.00 | 51.32 | D000 | C |
| ATOM | 7941 | CD1 | TRP | P | 195 | -25.307 | -15.008 | 69.586 | 1.00 | 46.03 | D000 | C |
| ATOM | 7942 | CD2 | TRP | P | 195 | -24.854 | -16.251 | 71.395 | 1.00 | 47.53 | D000 | C |
| ATOM | 7943 | NE1 | TRP | P | 195 | -24.987 | -14.148 | 70.610 | 1.00 | 47.20 | D000 | N |
| ATOM | 7944 | CE2 | TRP | P | 195 | -24.706 | -14.885 | 71.733 | 1.00 | 51.18 | D000 | C |
| ATOM | 7945 | CE3 | TRP | P | 195 | -24.614 | -17.221 | 72.375 | 1.00 | 46.44 | D000 | C |

| | | | | | | | | | | | | |
|---|---|---|---|---|---|---|---|---|---|---|---|---|
| ATOM | 7946 | CZ2 | TRP | P | 195 | -24.331 | -14.464 | 73.016 | 1.00 | 55.85 | D000 | C |
| ATOM | 7947 | CZ3 | TRP | P | 195 | -24.241 | -16.801 | 73.654 | 1.00 | 53.40 | D000 | C |
| ATOM | 7948 | CH2 | TRP | P | 195 | -24.105 | -15.435 | 73.961 | 1.00 | 52.97 | D000 | C |
| ATOM | 7949 | N | GLU | P | 196 | -25.024 | -20.181 | 67.458 | 1.00 | 34.05 | D000 | N |
| ATOM | 7950 | CA | GLU | P | 196 | -25.384 | -21.557 | 67.174 | 1.00 | 36.55 | D000 | C |
| ATOM | 7951 | C | GLU | P | 196 | -24.200 | -22.484 | 67.430 | 1.00 | 37.31 | D000 | C |
| ATOM | 7952 | O | GLU | P | 196 | -24.343 | -23.528 | 68.078 | 1.00 | 36.90 | D000 | O |
| ATOM | 7953 | CB | GLU | P | 196 | -25.899 | -21.650 | 65.740 | 1.00 | 37.33 | D000 | C |
| ATOM | 7954 | CG | GLU | P | 196 | -25.908 | -23.036 | 65.156 | 1.00 | 54.30 | D000 | C |
| ATOM | 7955 | CD | GLU | P | 196 | -27.254 | -23.709 | 65.306 | 1.00 | 67.53 | D000 | C |
| ATOM | 7956 | OE1 | GLU | P | 196 | -28.111 | -23.139 | 66.025 | 1.00 | 74.43 | D000 | O |
| ATOM | 7957 | OE2 | GLU | P | 196 | -27.453 | -24.799 | 64.704 | 1.00 | 66.20 | D000 | O1- |
| ATOM | 7958 | N | GLU | P | 197 | -23.008 | -22.101 | 66.961 | 1.00 | 37.44 | D000 | N |
| ATOM | 7959 | CA | GLU | P | 197 | -21.837 | -22.949 | 67.173 | 1.00 | 37.15 | D000 | C |
| ATOM | 7960 | C | GLU | P | 197 | -21.448 | -23.002 | 68.652 | 1.00 | 36.59 | D000 | C |
| ATOM | 7961 | O | GLU | P | 197 | -21.077 | -24.064 | 69.166 | 1.00 | 34.20 | D000 | O |
| ATOM | 7962 | CB | GLU | P | 197 | -20.674 | -22.458 | 66.315 | 1.00 | 32.94 | D000 | C |
| ATOM | 7963 | CG | GLU | P | 197 | -19.417 | -23.347 | 66.343 | 1.00 | 35.50 | D000 | C |
| ATOM | 7964 | CD | GLU | P | 197 | -18.383 | -22.948 | 65.292 | 1.00 | 41.89 | D000 | C |
| ATOM | 7965 | OE1 | GLU | P | 197 | -18.466 | -21.807 | 64.772 | 1.00 | 45.12 | D000 | O |
| ATOM | 7966 | OE2 | GLU | P | 197 | -17.486 | -23.771 | 64.985 | 1.00 | 41.03 | D000 | O1- |
| ATOM | 7967 | N | GLN | P | 198 | -21.538 | -21.865 | 69.345 | 1.00 | 32.12 | D000 | N |
| ATOM | 7968 | CA | GLN | P | 198 | -21.193 | -21.792 | 70.759 | 1.00 | 31.52 | D000 | C |
| ATOM | 7969 | C | GLN | P | 198 | -22.083 | -22.703 | 71.598 | 1.00 | 35.74 | D000 | C |
| ATOM | 7970 | O | GLN | P | 198 | -21.600 | -23.403 | 72.501 | 1.00 | 30.28 | D000 | O |
| ATOM | 7971 | CB | GLN | P | 198 | -21.298 | -20.334 | 71.226 | 1.00 | 33.50 | D000 | C |
| ATOM | 7972 | CG | GLN | P | 198 | -21.391 | -20.126 | 72.734 | 1.00 | 33.84 | D000 | C |
| ATOM | 7973 | CD | GLN | P | 198 | -20.055 | -20.173 | 73.450 | 1.00 | 33.31 | D000 | C |
| ATOM | 7974 | OE1 | GLN | P | 198 | -19.009 | -19.859 | 72.878 | 1.00 | 30.27 | D000 | O |
| ATOM | 7975 | NE2 | GLN | P | 198 | -20.085 | -20.563 | 74.713 | 1.00 | 33.30 | D000 | N |
| ATOM | 7976 | N | LYS | P | 199 | -23.393 | -22.694 | 71.320 | 1.00 | 35.11 | D000 | N |
| ATOM | 7977 | CA | LYS | P | 199 | -24.328 | -23.523 | 72.070 | 1.00 | 32.09 | D000 | C |
| ATOM | 7978 | C | LYS | P | 199 | -24.070 | -25.009 | 71.828 | 1.00 | 32.10 | D000 | C |
| ATOM | 7979 | O | LYS | P | 199 | -24.103 | -25.811 | 72.767 | 1.00 | 31.39 | D000 | O |
| ATOM | 7980 | CB | LYS | P | 199 | -25.753 | -23.142 | 71.692 | 1.00 | 31.90 | D000 | C |
| ATOM | 7981 | CG | LYS | P | 199 | -26.283 | -21.993 | 72.497 | 1.00 | 34.02 | D000 | C |
| ATOM | 7982 | CD | LYS | P | 199 | -27.260 | -21.182 | 71.709 | 1.00 | 36.65 | D000 | C |
| ATOM | 7983 | CE | LYS | P | 199 | -28.314 | -22.071 | 71.096 | 1.00 | 40.59 | D000 | C |
| ATOM | 7984 | NZ | LYS | P | 199 | -29.444 | -21.249 | 70.594 | 1.00 | 48.36 | D000 | N1+ |
| ATOM | 7985 | N | PHE | P | 200 | -23.806 | -25.392 | 70.581 | 1.00 | 28.05 | D000 | N |
| ATOM | 7986 | CA | PHE | P | 200 | -23.515 | -26.788 | 70.283 | 1.00 | 28.75 | D000 | C |
| ATOM | 7987 | C | PHE | P | 200 | -22.286 | -27.284 | 71.036 | 1.00 | 32.43 | D000 | C |
| ATOM | 7988 | O | PHE | P | 200 | -22.287 | -28.399 | 71.568 | 1.00 | 30.79 | D000 | O |
| ATOM | 7989 | CB | PHE | P | 200 | -23.318 | -26.956 | 68.787 | 1.00 | 27.58 | D000 | C |
| ATOM | 7990 | CG | PHE | P | 200 | -22.644 | -28.222 | 68.417 | 1.00 | 29.26 | D000 | C |
| ATOM | 7991 | CD1 | PHE | P | 200 | -23.363 | -29.387 | 68.303 | 1.00 | 28.09 | D000 | C |
| ATOM | 7992 | CD2 | PHE | P | 200 | -21.279 | -28.247 | 68.172 | 1.00 | 31.84 | D000 | C |
| ATOM | 7993 | CE1 | PHE | P | 200 | -22.747 | -30.561 | 67.943 | 1.00 | 30.20 | D000 | C |
| ATOM | 7994 | CE2 | PHE | P | 200 | -20.647 | -29.415 | 67.799 | 1.00 | 31.13 | D000 | C |
| ATOM | 7995 | CZ | PHE | P | 200 | -21.385 | -30.577 | 67.682 | 1.00 | 33.34 | D000 | C |
| ATOM | 7996 | N | VAL | P | 201 | -21.220 | -26.478 | 71.083 | 1.00 | 30.80 | D000 | N |
| ATOM | 7997 | CA | VAL | P | 201 | -20.015 | -26.900 | 71.787 | 1.00 | 31.26 | D000 | C |
| ATOM | 7998 | C | VAL | P | 201 | -20.277 | -26.989 | 73.295 | 1.00 | 31.79 | D000 | C |
| ATOM | 7999 | O | VAL | P | 201 | -19.849 | -27.947 | 73.948 | 1.00 | 29.59 | D000 | O |
| ATOM | 8000 | CB | VAL | P | 201 | -18.836 | -25.959 | 71.458 | 1.00 | 30.91 | D000 | C |
| ATOM | 8001 | CG1 | VAL | P | 201 | -17.611 | -26.260 | 72.345 | 1.00 | 32.04 | D000 | C |
| ATOM | 8002 | CG2 | VAL | P | 201 | -18.433 | -26.104 | 70.020 | 1.00 | 33.28 | D000 | C |
| ATOM | 8003 | N | GLN | P | 202 | -21.001 | -26.012 | 73.862 | 1.00 | 30.50 | D000 | N |
| ATOM | 8004 | CA | GLN | P | 202 | -21.323 | -26.044 | 75.290 | 1.00 | 30.37 | D000 | C |
| ATOM | 8005 | C | GLN | P | 202 | -22.040 | -27.327 | 75.670 | 1.00 | 25.89 | D000 | C |
| ATOM | 8006 | O | GLN | P | 202 | -21.755 | -27.903 | 76.719 | 1.00 | 33.97 | D000 | O |
| ATOM | 8007 | CB | GLN | P | 202 | -22.203 | -24.865 | 75.701 | 1.00 | 35.09 | D000 | C |
| ATOM | 8008 | CG | GLN | P | 202 | -21.566 | -23.520 | 75.783 | 1.00 | 35.69 | D000 | C |
| ATOM | 8009 | CD | GLN | P | 202 | -22.490 | -22.528 | 76.480 | 1.00 | 47.70 | D000 | C |
| ATOM | 8010 | OE1 | GLN | P | 202 | -22.615 | -21.351 | 76.078 | 1.00 | 41.49 | D000 | O |
| ATOM | 8011 | NE2 | GLN | P | 202 | -23.110 | -22.990 | 77.575 | 1.00 | 49.12 | D000 | N |
| ATOM | 8012 | N | HIS | P | 203 | -23.025 | -27.742 | 74.871 | 1.00 | 25.10 | D000 | N |
| ATOM | 8013 | CA | HIS | P | 203 | -23.731 | -29.000 | 75.117 | 1.00 | 27.41 | D000 | C |
| ATOM | 8014 | C | HIS | P | 203 | -22.780 | -30.154 | 75.316 | 1.00 | 32.74 | D000 | C |
| ATOM | 8015 | O | HIS | P | 203 | -22.919 | -30.940 | 76.262 | 1.00 | 37.16 | D000 | O |
| ATOM | 8016 | CB | HIS | P | 203 | -24.638 | -29.354 | 73.947 | 1.00 | 31.40 | D000 | C |

| | | | | | | | | | | | | |
|---|---|---|---|---|---|---|---|---|---|---|---|---|
| ATOM | 8017 | CG | HIS | P | 203 | -26.051 | -28.936 | 74.117 | 1.00 | 37.58 | D000 | C |
| ATOM | 8018 | ND1 | HIS | P | 203 | -26.725 | -28.198 | 73.167 | 1.00 | 46.93 | D000 | N |
| ATOM | 8019 | CD2 | HIS | P | 203 | -26.942 | -29.204 | 75.098 | 1.00 | 43.64 | D000 | C |
| ATOM | 8020 | CE1 | HIS | P | 203 | -27.963 | -27.992 | 73.576 | 1.00 | 52.72 | D000 | C |
| ATOM | 8021 | NE2 | HIS | P | 203 | -28.122 | -28.598 | 74.742 | 1.00 | 57.54 | D000 | N |
| ATOM | 8022 | N | HIS | P | 204 | -21.809 | -30.280 | 74.406 | 1.00 | 33.87 | D000 | N |
| ATOM | 8023 | CA | HIS | P | 204 | -20.952 | -31.453 | 74.375 | 1.00 | 32.62 | D000 | C |
| ATOM | 8024 | C | HIS | P | 204 | -19.857 | -31.394 | 75.428 | 1.00 | 30.65 | D000 | C |
| ATOM | 8025 | O | HIS | P | 204 | -19.399 | -32.445 | 75.880 | 1.00 | 27.67 | D000 | O |
| ATOM | 8026 | CB | HIS | P | 204 | -20.373 | -31.635 | 72.969 | 1.00 | 25.37 | D000 | C |
| ATOM | 8027 | CG | HIS | P | 204 | -21.341 | -32.234 | 71.995 | 1.00 | 29.38 | D000 | C |
| ATOM | 8028 | ND1 | HIS | P | 204 | -21.525 | -33.593 | 71.869 | 1.00 | 30.92 | D000 | N |
| ATOM | 8029 | CD2 | HIS | P | 204 | -22.217 | -31.661 | 71.137 | 1.00 | 32.57 | D000 | C |
| ATOM | 8030 | CE1 | HIS | P | 204 | -22.445 | -33.835 | 70.953 | 1.00 | 28.23 | D000 | C |
| ATOM | 8031 | NE2 | HIS | P | 204 | -22.883 | -32.678 | 70.494 | 1.00 | 34.46 | D000 | N |
| ATOM | 8032 | N | ILE | P | 205 | -19.424 | -30.197 | 75.830 | 1.00 | 28.24 | D000 | N |
| ATOM | 8033 | CA | ILE | P | 205 | -18.324 | -30.104 | 76.778 | 1.00 | 27.46 | D000 | C |
| ATOM | 8034 | C | ILE | P | 205 | -18.778 | -30.056 | 78.220 | 1.00 | 33.06 | D000 | C |
| ATOM | 8035 | O | ILE | P | 205 | -17.952 | -30.295 | 79.111 | 1.00 | 35.59 | D000 | O |
| ATOM | 8036 | CB | ILE | P | 205 | -17.392 | -28.907 | 76.513 | 1.00 | 33.12 | D000 | C |
| ATOM | 8037 | CG1 | ILE | P | 205 | -18.123 | -27.564 | 76.648 | 1.00 | 26.96 | D000 | C |
| ATOM | 8038 | CG2 | ILE | P | 205 | -16.702 | -29.080 | 75.173 | 1.00 | 25.98 | D000 | C |
| ATOM | 8039 | CD1 | ILE | P | 205 | -17.174 | -26.412 | 76.624 | 1.00 | 23.08 | D000 | C |
| ATOM | 8040 | N | GLY | P | 206 | -20.043 | -29.713 | 78.482 | 1.00 | 35.40 | D000 | N |
| ATOM | 8041 | CA | GLY | P | 206 | -20.536 | -29.649 | 79.837 | 1.00 | 29.75 | D000 | C |
| ATOM | 8042 | C | GLY | P | 206 | -19.906 | -28.523 | 80.620 | 1.00 | 32.65 | D000 | C |
| ATOM | 8043 | O | GLY | P | 206 | -19.313 | -27.601 | 80.053 | 1.00 | 35.11 | D000 | O |
| ATOM | 8044 | N | PRO | P | 207 | -20.032 | -28.568 | 81.948 | 1.00 | 33.48 | D000 | N |
| ATOM | 8045 | CA | PRO | P | 207 | -19.544 | -27.464 | 82.797 | 1.00 | 31.43 | D000 | C |
| ATOM | 8046 | C | PRO | P | 207 | -18.059 | -27.610 | 83.123 | 1.00 | 36.17 | D000 | C |
| ATOM | 8047 | O | PRO | P | 207 | -17.637 | -27.650 | 84.288 | 1.00 | 38.20 | D000 | O |
| ATOM | 8048 | CB | PRO | P | 207 | -20.443 | -27.590 | 84.032 | 1.00 | 35.30 | D000 | C |
| ATOM | 8049 | CG | PRO | P | 207 | -20.723 | -29.073 | 84.132 | 1.00 | 31.37 | D000 | C |
| ATOM | 8050 | CD | PRO | P | 207 | -20.694 | -29.628 | 82.727 | 1.00 | 30.51 | D000 | C |
| ATOM | 8051 | N | VAL | P | 208 | -17.242 | -27.622 | 82.071 | 1.00 | 35.28 | D000 | N |
| ATOM | 8052 | CA | VAL | P | 208 | -15.854 | -28.057 | 82.127 | 1.00 | 31.19 | D000 | C |
| ATOM | 8053 | C | VAL | P | 208 | -14.990 | -27.043 | 81.389 | 1.00 | 32.34 | D000 | C |
| ATOM | 8054 | O | VAL | P | 208 | -15.252 | -26.754 | 80.217 | 1.00 | 30.13 | D000 | O |
| ATOM | 8055 | CB | VAL | P | 208 | -15.733 | -29.454 | 81.493 | 1.00 | 24.39 | D000 | C |
| ATOM | 8056 | CG1 | VAL | P | 208 | -14.304 | -29.927 | 81.433 | 1.00 | 19.72 | D000 | C |
| ATOM | 8057 | CG2 | VAL | P | 208 | -16.623 | -30.417 | 82.259 | 1.00 | 26.85 | D000 | C |
| ATOM | 8058 | N | ASN | P | 209 | -13.955 | -26.514 | 82.065 | 1.00 | 33.01 | D000 | N |
| ATOM | 8059 | CA | ASN | P | 209 | -12.987 | -25.628 | 81.404 | 1.00 | 28.33 | D000 | C |
| ATOM | 8060 | C | ASN | P | 209 | -12.325 | -26.339 | 80.224 | 1.00 | 28.70 | D000 | C |
| ATOM | 8061 | O | ASN | P | 209 | -11.666 | -27.369 | 80.409 | 1.00 | 26.30 | D000 | O |
| ATOM | 8062 | CB | ASN | P | 209 | -11.911 | -25.165 | 82.388 | 1.00 | 26.60 | D000 | C |
| ATOM | 8063 | CG | ASN | P | 209 | -12.401 | -24.116 | 83.370 | 1.00 | 27.81 | D000 | C |
| ATOM | 8064 | OD1 | ASN | P | 209 | -13.324 | -23.343 | 83.094 | 1.00 | 31.03 | D000 | O |
| ATOM | 8065 | ND2 | ASN | P | 209 | -11.774 | -24.083 | 84.528 | 1.00 | 22.70 | D000 | N |
| ATOM | 8066 | N | THR | P | 210 | -12.487 | -25.786 | 79.017 | 1.00 | 27.48 | D000 | N |
| ATOM | 8067 | CA | THR | P | 210 | -12.049 | -26.451 | 77.790 | 1.00 | 27.53 | D000 | C |
| ATOM | 8068 | C | THR | P | 210 | -11.385 | -25.441 | 76.860 | 1.00 | 29.97 | D000 | C |
| ATOM | 8069 | O | THR | P | 210 | -11.918 | -24.350 | 76.656 | 1.00 | 28.86 | D000 | O |
| ATOM | 8070 | CB | THR | P | 210 | -13.223 | -27.156 | 77.082 | 1.00 | 28.24 | D000 | C |
| ATOM | 8071 | OG1 | THR | P | 210 | -13.710 | -28.226 | 77.908 | 1.00 | 30.12 | D000 | O |
| ATOM | 8072 | CG2 | THR | P | 210 | -12.801 | -27.746 | 75.755 | 1.00 | 23.19 | D000 | C |
| ATOM | 8073 | N | TRP | P | 211 | -10.219 | -25.797 | 76.313 | 1.00 | 29.85 | D000 | N |
| ATOM | 8074 | CA | TRP | P | 211 | -9.514 | -24.902 | 75.399 | 1.00 | 31.37 | D000 | C |
| ATOM | 8075 | C | TRP | P | 211 | -10.280 | -24.746 | 74.099 | 1.00 | 34.18 | D000 | C |
| ATOM | 8076 | O | TRP | P | 211 | -10.884 | -25.707 | 73.604 | 1.00 | 32.26 | D000 | O |
| ATOM | 8077 | CB | TRP | P | 211 | -8.122 | -25.430 | 75.046 | 1.00 | 26.53 | D000 | C |
| ATOM | 8078 | CG | TRP | P | 211 | -7.115 | -25.421 | 76.131 | 1.00 | 30.84 | D000 | C |
| ATOM | 8079 | CD1 | TRP | P | 211 | -6.341 | -26.470 | 76.523 | 1.00 | 29.48 | D000 | C |
| ATOM | 8080 | CD2 | TRP | P | 211 | -6.740 | -24.312 | 76.959 | 1.00 | 29.42 | D000 | C |
| ATOM | 8081 | NE1 | TRP | P | 211 | -5.507 | -26.084 | 77.538 | 1.00 | 29.42 | D000 | N |
| ATOM | 8082 | CE2 | TRP | P | 211 | -5.740 | -24.768 | 77.832 | 1.00 | 29.17 | D000 | C |
| ATOM | 8083 | CE3 | TRP | P | 211 | -7.150 | -22.979 | 77.041 | 1.00 | 31.34 | D000 | C |
| ATOM | 8084 | CZ2 | TRP | P | 211 | -5.145 | -23.942 | 78.778 | 1.00 | 31.63 | D000 | C |
| ATOM | 8085 | CZ3 | TRP | P | 211 | -6.563 | -22.163 | 77.977 | 1.00 | 28.33 | D000 | C |
| ATOM | 8086 | CH2 | TRP | P | 211 | -5.568 | -22.642 | 78.830 | 1.00 | 30.22 | D000 | C |
| ATOM | 8087 | N | MET | P | 212 | -10.227 | -23.530 | 73.540 | 1.00 | 33.06 | D000 | N |

| | | | | | | | | | | | |
|---|---|---|---|---|---|---|---|---|---|---|---|
| ATOM | 8088 | CA | MET | P | 212 | -10.647 | -23.222 | 72.175 | 1.00 | 33.50 | D000 C |
| ATOM | 8089 | C | MET | P | 212 | -9.461 | -22.623 | 71.415 | 1.00 | 35.69 | D000 C |
| ATOM | 8090 | O | MET | P | 212 | -8.401 | -22.362 | 71.985 | 1.00 | 33.28 | D000 O |
| ATOM | 8091 | CB | MET | P | 212 | -11.841 | -22.265 | 72.153 | 1.00 | 27.35 | D000 C |
| ATOM | 8092 | CG | MET | P | 212 | -11.442 | -20.835 | 72.335 | 1.00 | 34.07 | D000 C |
| ATOM | 8093 | SD | MET | P | 212 | -12.807 | -19.717 | 72.680 | 1.00 | 33.50 | D000 S |
| ATOM | 8094 | CE | MET | P | 212 | -13.264 | -20.227 | 74.323 | 1.00 | 34.96 | D000 C |
| ATOM | 8095 | N | GLY | P | 213 | -9.615 | -22.465 | 70.099 | 1.00 | 38.96 | D000 N |
| ATOM | 8096 | CA | GLY | P | 213 | -8.538 | -21.941 | 69.266 | 1.00 | 36.71 | D000 C |
| ATOM | 8097 | C | GLY | P | 213 | -8.297 | -20.437 | 69.293 | 1.00 | 36.61 | D000 C |
| ATOM | 8098 | O | GLY | P | 213 | -8.265 | -19.807 | 68.237 | 1.00 | 34.15 | D000 O |
| ATOM | 8099 | N | LEU | P | 214 | -8.113 | -19.847 | 70.472 | 1.00 | 37.22 | D000 N |
| ATOM | 8100 | CA | LEU | P | 214 | -7.994 | -18.399 | 70.609 | 1.00 | 40.19 | D000 C |
| ATOM | 8101 | C | LEU | P | 214 | -6.897 | -18.065 | 71.618 | 1.00 | 44.61 | D000 C |
| ATOM | 8102 | O | LEU | P | 214 | -6.928 | -18.534 | 72.761 | 1.00 | 42.53 | D000 O |
| ATOM | 8103 | CB | LEU | P | 214 | -9.340 | -17.783 | 71.019 | 1.00 | 36.46 | D000 C |
| ATOM | 8104 | CG | LEU | P | 214 | -9.420 | -16.273 | 71.253 | 1.00 | 39.49 | D000 C |
| ATOM | 8105 | CD1 | LEU | P | 214 | -9.308 | -15.528 | 69.943 | 1.00 | 39.79 | D000 C |
| ATOM | 8106 | CD2 | LEU | P | 214 | -10.692 | -15.890 | 71.983 | 1.00 | 37.37 | D000 C |
| ATOM | 8107 | N | HIS | P | 215 | -5.914 | -17.274 | 71.191 | 1.00 | 47.99 | D000 N |
| ATOM | 8108 | CA | HIS | P | 215 | -4.787 | -16.902 | 72.042 | 1.00 | 53.52 | D000 C |
| ATOM | 8109 | C | HIS | P | 215 | -4.270 | -15.525 | 71.632 | 1.00 | 50.41 | D000 C |
| ATOM | 8110 | O | HIS | P | 215 | -4.496 | -15.076 | 70.506 | 1.00 | 50.05 | D000 O |
| ATOM | 8111 | CB | HIS | P | 215 | -3.668 | -17.963 | 71.988 | 1.00 | 48.40 | D000 C |
| ATOM | 8112 | CG | HIS | P | 215 | -3.026 | -18.112 | 70.645 | 1.00 | 51.27 | D000 C |
| ATOM | 8113 | ND1 | HIS | P | 215 | -1.709 | -18.488 | 70.490 | 1.00 | 52.99 | D000 N |
| ATOM | 8114 | CD2 | HIS | P | 215 | -3.517 | -17.937 | 69.394 | 1.00 | 48.48 | D000 C |
| ATOM | 8115 | CE1 | HIS | P | 215 | -1.417 | -18.539 | 69.203 | 1.00 | 55.12 | D000 C |
| ATOM | 8116 | NE2 | HIS | P | 215 | -2.496 | -18.205 | 68.517 | 1.00 | 52.31 | D000 N |
| ATOM | 8117 | N | ASP | P | 216 | -3.639 | -14.826 | 72.579 | 1.00 | 50.32 | D000 N |
| ATOM | 8118 | CA | ASP | P | 216 | -2.999 | -13.547 | 72.283 | 1.00 | 62.90 | D000 C |
| ATOM | 8119 | C | ASP | P | 216 | -1.529 | -13.584 | 72.680 | 1.00 | 66.62 | D000 C |
| ATOM | 8120 | O | ASP | P | 216 | -0.957 | -12.579 | 73.111 | 1.00 | 70.47 | D000 O |
| ATOM | 8121 | CB | ASP | P | 216 | -3.730 | -12.376 | 72.949 | 1.00 | 58.20 | D000 C |
| ATOM | 8122 | CG | ASP | P | 216 | -3.387 | -12.201 | 74.428 | 1.00 | 62.56 | D000 C |
| ATOM | 8123 | OD1 | ASP | P | 216 | -2.915 | -13.156 | 75.088 | 1.00 | 67.95 | D000 O |
| ATOM | 8124 | OD2 | ASP | P | 216 | -3.613 | -11.088 | 74.942 | 1.00 | 66.89 | D000 O1- |
| ATOM | 8125 | N | GLN | P | 217 | -0.907 | -14.751 | 72.520 | 1.00 | 64.97 | D000 N |
| ATOM | 8126 | CA | GLN | P | 217 | 0.521 | -14.866 | 72.771 | 1.00 | 76.33 | D000 C |
| ATOM | 8127 | C | GLN | P | 217 | 1.312 | -13.867 | 71.944 | 1.00 | 79.09 | D000 C |
| ATOM | 8128 | O | GLN | P | 217 | 2.439 | -13.521 | 72.314 | 1.00 | 84.36 | D000 O |
| ATOM | 8129 | CB | GLN | P | 217 | 0.981 | -16.309 | 72.509 | 1.00 | 76.19 | D000 C |
| ATOM | 8130 | CG | GLN | P | 217 | 0.950 | -17.206 | 73.776 | 1.00 | 69.70 | D000 C |
| ATOM | 8131 | CD | GLN | P | 217 | 0.539 | -18.645 | 73.498 | 1.00 | 61.34 | D000 C |
| ATOM | 8132 | OE1 | GLN | P | 217 | -0.353 | -18.897 | 72.690 | 1.00 | 62.21 | D000 O |
| ATOM | 8133 | NE2 | GLN | P | 217 | 1.177 | -19.597 | 74.185 | 1.00 | 61.50 | D000 N |
| ATOM | 8134 | N | ASN | P | 218 | 0.722 | -13.361 | 70.863 | 1.00 | 80.19 | D000 N |
| ATOM | 8135 | CA | ASN | P | 218 | 1.363 | -12.319 | 70.073 | 1.00 | 87.85 | D000 C |
| ATOM | 8136 | C | ASN | P | 218 | 1.275 | -10.964 | 70.775 | 1.00 | 79.48 | D000 C |
| ATOM | 8137 | O | ASN | P | 218 | 2.294 | -10.367 | 71.140 | 1.00 | 80.45 | D000 O |
| ATOM | 8138 | CB | ASN | P | 218 | 0.705 | -12.268 | 68.689 | 1.00 | 84.56 | D000 C |
| ATOM | 8139 | CG | ASN | P | 218 | 1.684 | -11.916 | 67.594 | 1.00 | 89.88 | D000 C |
| ATOM | 8140 | OD1 | ASN | P | 218 | 1.373 | -12.042 | 66.404 | 1.00 | 90.71 | D000 O |
| ATOM | 8141 | ND2 | ASN | P | 218 | 2.891 | -11.507 | 67.986 | 1.00 | 84.53 | D000 N |
| ATOM | 8142 | N | GLY | P | 219 | 0.059 | -10.514 | 71.040 | 1.00 | 74.18 | D000 N |
| ATOM | 8143 | CA | GLY | P | 219 | -0.212 | -9.187 | 71.530 | 1.00 | 68.61 | D000 C |
| ATOM | 8144 | C | GLY | P | 219 | -1.710 | -8.949 | 71.462 | 1.00 | 73.97 | D000 C |
| ATOM | 8145 | O | GLY | P | 219 | -2.366 | -8.740 | 72.489 | 1.00 | 71.33 | D000 O |
| ATOM | 8146 | N | PRO | P | 220 | -2.285 | -9.017 | 70.255 | 1.00 | 69.70 | D000 N |
| ATOM | 8147 | CA | PRO | P | 220 | -3.745 | -9.029 | 70.112 | 1.00 | 69.83 | D000 C |
| ATOM | 8148 | C | PRO | P | 220 | -4.304 | -10.446 | 70.009 | 1.00 | 67.70 | D000 C |
| ATOM | 8149 | O | PRO | P | 220 | -3.608 | -11.402 | 69.652 | 1.00 | 66.31 | D000 O |
| ATOM | 8150 | CB | PRO | P | 220 | -3.967 | -8.274 | 68.792 | 1.00 | 64.94 | D000 C |
| ATOM | 8151 | CG | PRO | P | 220 | -2.674 | -8.444 | 68.009 | 1.00 | 60.23 | D000 C |
| ATOM | 8152 | CD | PRO | P | 220 | -1.625 | -9.035 | 68.933 | 1.00 | 70.78 | D000 C |
| ATOM | 8153 | N | TRP | P | 221 | -5.595 | -10.568 | 70.309 | 1.00 | 58.78 | D000 N |
| ATOM | 8154 | CA | TRP | P | 221 | -6.244 | -11.868 | 70.228 | 1.00 | 52.55 | D000 C |
| ATOM | 8155 | C | TRP | P | 221 | -6.415 | -12.290 | 68.778 | 1.00 | 51.19 | D000 C |
| ATOM | 8156 | O | TRP | P | 221 | -6.795 | -11.486 | 67.920 | 1.00 | 53.28 | D000 O |
| ATOM | 8157 | CB | TRP | P | 221 | -7.601 | -11.831 | 70.940 | 1.00 | 52.62 | D000 C |
| ATOM | 8158 | CG | TRP | P | 221 | -7.438 | -11.729 | 72.425 | 1.00 | 50.70 | D000 C |

| | | | | | | | | | | | | |
|---|---|---|---|---|---|---|---|---|---|---|---|---|
| ATOM | 8159 | CD1 | TRP | P | 221 | -7.477 | -10.597 | 73.179 | 1.00 | 52.92 | D000 | C |
| ATOM | 8160 | CD2 | TRP | P | 221 | -7.164 | -12.807 | 73.332 | 1.00 | 48.63 | D000 | C |
| ATOM | 8161 | NE1 | TRP | P | 221 | -7.256 | -10.901 | 74.505 | 1.00 | 54.57 | D000 | N |
| ATOM | 8162 | CE2 | TRP | P | 221 | -7.058 | -12.251 | 74.624 | 1.00 | 49.93 | D000 | C |
| ATOM | 8163 | CE3 | TRP | P | 221 | -7.001 | -14.189 | 73.177 | 1.00 | 46.93 | D000 | C |
| ATOM | 8164 | CZ2 | TRP | P | 221 | -6.796 | -13.029 | 75.757 | 1.00 | 50.65 | D000 | C |
| ATOM | 8165 | CZ3 | TRP | P | 221 | -6.739 | -14.960 | 74.303 | 1.00 | 46.92 | D000 | C |
| ATOM | 8166 | CH2 | TRP | P | 221 | -6.643 | -14.377 | 75.575 | 1.00 | 48.14 | D000 | C |
| ATOM | 8167 | N | LYS | P | 222 | -6.132 | -13.568 | 68.513 | 1.00 | 50.38 | D000 | N |
| ATOM | 8168 | CA | LYS | P | 222 | -6.213 | -14.155 | 67.178 | 1.00 | 52.36 | D000 | C |
| ATOM | 8169 | C | LYS | P | 222 | -6.785 | -15.564 | 67.274 | 1.00 | 48.11 | D000 | C |
| ATOM | 8170 | O | LYS | P | 222 | -6.551 | -16.277 | 68.253 | 1.00 | 43.41 | D000 | O |
| ATOM | 8171 | CB | LYS | P | 222 | -4.842 | -14.247 | 66.473 | 1.00 | 48.15 | D000 | C |
| ATOM | 8172 | CG | LYS | P | 222 | -3.989 | -13.006 | 66.480 | 1.00 | 49.51 | D000 | C |
| ATOM | 8173 | CD | LYS | P | 222 | -2.623 | -13.343 | 65.879 | 1.00 | 63.49 | D000 | C |
| ATOM | 8174 | CE | LYS | P | 222 | -1.744 | -12.107 | 65.682 | 1.00 | 74.29 | D000 | C |
| ATOM | 8175 | NZ | LYS | P | 222 | -0.559 | -12.385 | 64.812 | 1.00 | 71.05 | D000 | N1+ |
| ATOM | 8176 | N | TRP | P | 223 | -7.518 | -15.967 | 66.239 | 1.00 | 46.69 | D000 | N |
| ATOM | 8177 | CA | TRP | P | 223 | -7.966 | -17.345 | 66.081 | 1.00 | 40.92 | D000 | C |
| ATOM | 8178 | C | TRP | P | 223 | -6.901 | -18.155 | 65.334 | 1.00 | 42.46 | D000 | C |
| ATOM | 8179 | O | TRP | P | 223 | -6.100 | -17.605 | 64.578 | 1.00 | 42.65 | D000 | O |
| ATOM | 8180 | CB | TRP | P | 223 | -9.314 | -17.398 | 65.346 | 1.00 | 38.25 | D000 | C |
| ATOM | 8181 | CG | TRP | P | 223 | -10.464 | -16.803 | 66.160 | 1.00 | 46.60 | D000 | C |
| ATOM | 8182 | CD1 | TRP | P | 223 | -10.918 | -15.508 | 66.115 | 1.00 | 44.78 | D000 | C |
| ATOM | 8183 | CD2 | TRP | P | 223 | -11.284 | -17.476 | 67.141 | 1.00 | 41.63 | D000 | C |
| ATOM | 8184 | NE1 | TRP | P | 223 | -11.954 | -15.337 | 67.003 | 1.00 | 45.05 | D000 | N |
| ATOM | 8185 | CE2 | TRP | P | 223 | -12.203 | -16.526 | 67.641 | 1.00 | 43.02 | D000 | C |
| ATOM | 8186 | CE3 | TRP | P | 223 | -11.328 | -18.786 | 67.644 | 1.00 | 40.53 | D000 | C |
| ATOM | 8187 | CZ2 | TRP | P | 223 | -13.152 | -16.843 | 68.628 | 1.00 | 38.24 | D000 | C |
| ATOM | 8188 | CZ3 | TRP | P | 223 | -12.271 | -19.097 | 68.619 | 1.00 | 38.15 | D000 | C |
| ATOM | 8189 | CH2 | TRP | P | 223 | -13.165 | -18.125 | 69.103 | 1.00 | 37.47 | D000 | C |
| ATOM | 8190 | N | VAL | P | 224 | -6.863 | -19.466 | 65.601 | 1.00 | 41.18 | D000 | N |
| ATOM | 8191 | CA | VAL | P | 224 | -5.813 | -20.316 | 65.049 | 1.00 | 40.27 | D000 | C |
| ATOM | 8192 | C | VAL | P | 224 | -5.952 | -20.568 | 63.553 | 1.00 | 43.70 | D000 | C |
| ATOM | 8193 | O | VAL | P | 224 | -4.977 | -20.993 | 62.918 | 1.00 | 45.37 | D000 | O |
| ATOM | 8194 | CB | VAL | P | 224 | -5.738 | -21.679 | 65.760 | 1.00 | 35.45 | D000 | C |
| ATOM | 8195 | CG1 | VAL | P | 224 | -5.402 | -21.490 | 67.200 | 1.00 | 39.13 | D000 | C |
| ATOM | 8196 | CG2 | VAL | P | 224 | -7.025 | -22.441 | 65.581 | 1.00 | 35.78 | D000 | C |
| ATOM | 8197 | N | ASP | P | 225 | -7.132 | -20.379 | 62.966 | 1.00 | 45.80 | D000 | N |
| ATOM | 8198 | CA | ASP | P | 225 | -7.297 | -20.628 | 61.535 | 1.00 | 47.35 | D000 | C |
| ATOM | 8199 | C | ASP | P | 225 | -7.260 | -19.343 | 60.721 | 1.00 | 44.18 | D000 | C |
| ATOM | 8200 | O | ASP | P | 225 | -7.418 | -19.380 | 59.496 | 1.00 | 41.75 | D000 | O |
| ATOM | 8201 | CB | ASP | P | 225 | -8.590 | -21.431 | 61.261 | 1.00 | 42.90 | D000 | C |
| ATOM | 8202 | CG | ASP | P | 225 | -9.864 | -20.593 | 61.367 | 1.00 | 45.62 | D000 | C |
| ATOM | 8203 | OD1 | ASP | P | 225 | -9.832 | -19.473 | 61.933 | 1.00 | 42.32 | D000 | O |
| ATOM | 8204 | OD2 | ASP | P | 225 | -10.912 | -21.079 | 60.878 | 1.00 | 49.08 | D000 | O1- |
| ATOM | 8205 | N | GLY | P | 226 | -7.023 | -18.215 | 61.373 | 1.00 | 42.84 | D000 | N |
| ATOM | 8206 | CA | GLY | P | 226 | -6.919 | -16.957 | 60.693 | 1.00 | 37.63 | D000 | C |
| ATOM | 8207 | C | GLY | P | 226 | -8.168 | -16.131 | 60.725 | 1.00 | 40.53 | D000 | C |
| ATOM | 8208 | O | GLY | P | 226 | -8.089 | -14.938 | 60.408 | 1.00 | 43.36 | D000 | O |
| ATOM | 8209 | N | THR | P | 227 | -9.315 | -16.725 | 61.089 | 1.00 | 42.48 | D000 | N |
| ATOM | 8210 | CA | THR | P | 227 | -10.562 | -15.973 | 61.156 | 1.00 | 39.26 | D000 | C |
| ATOM | 8211 | C | THR | P | 227 | -10.348 | -14.706 | 61.974 | 1.00 | 43.02 | D000 | C |
| ATOM | 8212 | O | THR | P | 227 | -9.666 | -14.722 | 63.005 | 1.00 | 41.15 | D000 | O |
| ATOM | 8213 | CB | THR | P | 227 | -11.684 | -16.819 | 61.760 | 1.00 | 38.82 | D000 | C |
| ATOM | 8214 | OG1 | THR | P | 227 | -11.803 | -18.060 | 61.044 | 1.00 | 45.33 | D000 | O |
| ATOM | 8215 | CG2 | THR | P | 227 | -13.015 | -16.065 | 61.694 | 1.00 | 30.94 | D000 | C |
| ATOM | 8216 | N | ASP | P | 228 | -10.899 | -13.600 | 61.485 | 1.00 | 35.97 | D000 | N |
| ATOM | 8217 | CA | ASP | P | 228 | -10.647 | -12.323 | 62.128 | 1.00 | 43.82 | D000 | C |
| ATOM | 8218 | C | ASP | P | 228 | -11.342 | -12.271 | 63.487 | 1.00 | 45.21 | D000 | C |
| ATOM | 8219 | O | ASP | P | 228 | -12.534 | -12.577 | 63.606 | 1.00 | 44.16 | D000 | O |
| ATOM | 8220 | CB | ASP | P | 228 | -11.127 | -11.167 | 61.243 | 1.00 | 38.78 | D000 | C |
| ATOM | 8221 | CG | ASP | P | 228 | -10.940 | -9.817 | 61.909 | 1.00 | 49.28 | D000 | C |
| ATOM | 8222 | OD1 | ASP | P | 228 | -9.775 | -9.419 | 62.162 | 1.00 | 54.97 | D000 | O |
| ATOM | 8223 | OD2 | ASP | P | 228 | -11.958 | -9.175 | 62.242 | 1.00 | 50.80 | D000 | O1- |
| ATOM | 8224 | N | TYR | P | 229 | -10.601 | -11.828 | 64.501 | 1.00 | 48.02 | D000 | N |
| ATOM | 8225 | CA | TYR | P | 229 | -11.132 | -11.730 | 65.857 | 1.00 | 49.96 | D000 | C |
| ATOM | 8226 | C | TYR | P | 229 | -11.979 | -10.481 | 66.041 | 1.00 | 49.04 | D000 | C |
| ATOM | 8227 | O | TYR | P | 229 | -13.064 | -10.540 | 66.628 | 1.00 | 53.87 | D000 | O |
| ATOM | 8228 | CB | TYR | P | 229 | -9.995 | -11.743 | 66.883 | 1.00 | 44.05 | D000 | C |
| ATOM | 8229 | CG | TYR | P | 229 | -10.439 | -11.358 | 68.269 | 1.00 | 45.49 | D000 | C |

| | | | | | | | | | | | | |
|---|---|---|---|---|---|---|---|---|---|---|---|---|
| ATOM | 8230 | CD2 | TYR | P | 229 | -10.286 | -10.061 | 68.725 | 1.00 | 53.43 | D000 | C |
| ATOM | 8231 | CD1 | TYR | P | 229 | -10.994 | -12.296 | 69.132 | 1.00 | 44.65 | D000 | C |
| ATOM | 8232 | CE2 | TYR | P | 229 | -10.684 | -9.695 | 69.997 | 1.00 | 56.58 | D000 | C |
| ATOM | 8233 | CE1 | TYR | P | 229 | -11.403 | -11.944 | 70.406 | 1.00 | 46.74 | D000 | C |
| ATOM | 8234 | CZ | TYR | P | 229 | -11.243 | -10.638 | 70.838 | 1.00 | 52.93 | D000 | C |
| ATOM | 8235 | OH | TYR | P | 229 | -11.634 | -10.250 | 72.104 | 1.00 | 43.44 | D000 | O |
| ATOM | 8236 | N | GLU | P | 230 | -11.503 | -9.341 | 65.555 | 1.00 | 51.91 | D000 | N |
| ATOM | 8237 | CA | GLU | P | 230 | -12.119 | -8.087 | 65.968 | 1.00 | 56.16 | D000 | C |
| ATOM | 8238 | C | GLU | P | 230 | -13.560 | -7.995 | 65.476 | 1.00 | 54.64 | D000 | C |
| ATOM | 8239 | O | GLU | P | 230 | -14.468 | -7.629 | 66.236 | 1.00 | 52.29 | D000 | O |
| ATOM | 8240 | CB | GLU | P | 230 | -11.292 | -6.908 | 65.456 | 1.00 | 50.78 | D000 | C |
| ATOM | 8241 | CG | GLU | P | 230 | -11.547 | -5.640 | 66.240 | 1.00 | 59.32 | D000 | C |
| ATOM | 8242 | CD | GLU | P | 230 | -11.610 | -5.897 | 67.751 | 1.00 | 65.21 | D000 | C |
| ATOM | 8243 | OE1 | GLU | P | 230 | -10.556 | -6.220 | 68.353 | 1.00 | 63.19 | D000 | O |
| ATOM | 8244 | OE2 | GLU | P | 230 | -12.718 | -5.790 | 68.330 | 1.00 | 66.54 | D000 | O1- |
| ATOM | 8245 | N | THR | P | 231 | -13.797 | -8.382 | 64.231 | 1.00 | 49.30 | D000 | N |
| ATOM | 8246 | CA | THR | P | 231 | -15.125 | -8.335 | 63.651 | 1.00 | 49.06 | D000 | C |
| ATOM | 8247 | C | THR | P | 231 | -15.943 | -9.595 | 63.913 | 1.00 | 48.66 | D000 | C |
| ATOM | 8248 | O | THR | P | 231 | -17.084 | -9.685 | 63.450 | 1.00 | 46.72 | D000 | O |
| ATOM | 8249 | CB | THR | P | 231 | -14.999 | -8.096 | 62.145 | 1.00 | 51.91 | D000 | C |
| ATOM | 8250 | OG1 | THR | P | 231 | -14.418 | -9.247 | 61.519 | 1.00 | 49.55 | D000 | O |
| ATOM | 8251 | CG2 | THR | P | 231 | -14.092 | -6.893 | 61.895 | 1.00 | 44.25 | D000 | C |
| ATOM | 8252 | N | GLY | P | 232 | -15.374 | -10.596 | 64.593 | 1.00 | 53.28 | D000 | N |
| ATOM | 8253 | CA | GLY | P | 232 | -16.057 | -11.861 | 64.770 | 1.00 | 51.71 | D000 | C |
| ATOM | 8254 | C | GLY | P | 232 | -16.832 | -11.982 | 66.082 | 1.00 | 49.39 | D000 | C |
| ATOM | 8255 | O | GLY | P | 232 | -16.750 | -11.136 | 66.974 | 1.00 | 45.77 | D000 | O |
| ATOM | 8256 | N | PHE | P | 233 | -17.569 | -13.087 | 66.178 | 1.00 | 46.98 | D000 | N |
| ATOM | 8257 | CA | PHE | P | 233 | -18.353 | -13.412 | 67.362 | 1.00 | 45.41 | D000 | C |
| ATOM | 8258 | C | PHE | P | 233 | -17.477 | -13.478 | 68.610 | 1.00 | 43.09 | D000 | C |
| ATOM | 8259 | O | PHE | P | 233 | -16.350 | -13.978 | 68.572 | 1.00 | 40.14 | D000 | O |
| ATOM | 8260 | CB | PHE | P | 233 | -19.050 | -14.755 | 67.133 | 1.00 | 43.80 | D000 | C |
| ATOM | 8261 | CG | PHE | P | 233 | -19.795 | -15.261 | 68.318 | 1.00 | 41.20 | D000 | C |
| ATOM | 8262 | CD1 | PHE | P | 233 | -21.056 | -14.778 | 68.620 | 1.00 | 42.87 | D000 | C |
| ATOM | 8263 | CD2 | PHE | P | 233 | -19.235 | -16.234 | 69.130 | 1.00 | 41.22 | D000 | C |
| ATOM | 8264 | CE1 | PHE | P | 233 | -21.746 | -15.250 | 69.718 | 1.00 | 43.07 | D000 | C |
| ATOM | 8265 | CE2 | PHE | P | 233 | -19.917 | -16.712 | 70.232 | 1.00 | 42.28 | D000 | C |
| ATOM | 8266 | CZ | PHE | P | 233 | -21.179 | -16.221 | 70.526 | 1.00 | 42.72 | D000 | C |
| ATOM | 8267 | N | LYS | P | 234 | -18.005 | -12.976 | 69.728 | 1.00 | 42.82 | D000 | N |
| ATOM | 8268 | CA | LYS | P | 234 | -17.292 | -12.988 | 71.000 | 1.00 | 37.90 | D000 | C |
| ATOM | 8269 | C | LYS | P | 234 | -18.268 | -13.294 | 72.133 | 1.00 | 41.60 | D000 | C |
| ATOM | 8270 | O | LYS | P | 234 | -19.440 | -12.901 | 72.093 | 1.00 | 44.48 | D000 | O |
| ATOM | 8271 | CB | LYS | P | 234 | -16.596 | -11.656 | 71.279 | 1.00 | 35.56 | D000 | C |
| ATOM | 8272 | CG | LYS | P | 234 | -15.570 | -11.240 | 70.265 | 1.00 | 40.67 | D000 | C |
| ATOM | 8273 | CD | LYS | P | 234 | -14.974 | -9.900 | 70.655 | 1.00 | 45.48 | D000 | C |
| ATOM | 8274 | CE | LYS | P | 234 | -14.396 | -9.152 | 69.453 | 1.00 | 51.18 | D000 | C |
| ATOM | 8275 | NZ | LYS | P | 234 | -15.432 | -8.815 | 68.436 | 1.00 | 50.83 | D000 | N1+ |
| ATOM | 8276 | N | ASN | P | 235 | -17.770 | -13.978 | 73.161 | 1.00 | 41.60 | D000 | N |
| ATOM | 8277 | CA | ASN | P | 235 | -18.619 | -14.402 | 74.273 | 1.00 | 37.71 | D000 | C |
| ATOM | 8278 | C | ASN | P | 235 | -17.822 | -14.433 | 75.583 | 1.00 | 37.90 | D000 | C |
| ATOM | 8279 | O | ASN | P | 235 | -17.903 | -15.379 | 76.370 | 1.00 | 37.72 | D000 | O |
| ATOM | 8280 | CB | ASN | P | 235 | -19.249 | -15.757 | 73.947 | 1.00 | 35.17 | D000 | C |
| ATOM | 8281 | CG | ASN | P | 235 | -20.286 | -16.187 | 74.963 | 1.00 | 40.71 | D000 | C |
| ATOM | 8282 | OD1 | ASN | P | 235 | -20.393 | -17.373 | 75.280 | 1.00 | 40.09 | D000 | O |
| ATOM | 8283 | ND2 | ASN | P | 235 | -21.034 | -15.228 | 75.504 | 1.00 | 41.85 | D000 | N |
| ATOM | 8284 | N | TRP | P | 236 | -17.096 | -13.353 | 75.867 | 1.00 | 39.79 | D000 | N |
| ATOM | 8285 | CA | TRP | P | 236 | -16.261 | -13.296 | 77.062 | 1.00 | 46.37 | D000 | C |
| ATOM | 8286 | C | TRP | P | 236 | -17.096 | -13.235 | 78.337 | 1.00 | 41.70 | D000 | C |
| ATOM | 8287 | O | TRP | P | 236 | -18.198 | -12.687 | 78.359 | 1.00 | 49.28 | D000 | O |
| ATOM | 8288 | CB | TRP | P | 236 | -15.335 | -12.082 | 77.009 | 1.00 | 45.14 | D000 | C |
| ATOM | 8289 | CG | TRP | P | 236 | -14.278 | -12.168 | 75.954 | 1.00 | 47.98 | D000 | C |
| ATOM | 8290 | CD1 | TRP | P | 236 | -14.263 | -11.524 | 74.742 | 1.00 | 45.52 | D000 | C |
| ATOM | 8291 | CD2 | TRP | P | 236 | -13.076 | -12.948 | 76.010 | 1.00 | 46.16 | D000 | C |
| ATOM | 8292 | NE1 | TRP | P | 236 | -13.122 | -11.854 | 74.047 | 1.00 | 47.86 | D000 | N |
| ATOM | 8293 | CE2 | TRP | P | 236 | -12.377 | -12.728 | 74.800 | 1.00 | 48.95 | D000 | C |
| ATOM | 8294 | CE3 | TRP | P | 236 | -12.524 | -13.813 | 76.961 | 1.00 | 44.69 | D000 | C |
| ATOM | 8295 | CZ2 | TRP | P | 236 | -11.155 | -13.347 | 74.517 | 1.00 | 41.16 | D000 | C |
| ATOM | 8296 | CZ3 | TRP | P | 236 | -11.303 | -14.427 | 76.679 | 1.00 | 46.91 | D000 | C |
| ATOM | 8297 | CH2 | TRP | P | 236 | -10.630 | -14.180 | 75.469 | 1.00 | 44.61 | D000 | C |
| ATOM | 8298 | N | ARG | P | 237 | -16.554 | -13.787 | 79.415 | 1.00 | 41.38 | D000 | N |
| ATOM | 8299 | CA | ARG | P | 237 | -17.130 | -13.493 | 80.718 | 1.00 | 53.87 | D000 | C |
| ATOM | 8300 | C | ARG | P | 237 | -16.928 | -12.007 | 80.992 | 1.00 | 61.28 | D000 | C |

| | | | | | | | | | | | |
|---|---|---|---|---|---|---|---|---|---|---|---|
| ATOM | 8301 | O | ARG | P | 237 | -15.886 | -11.452 | 80.630 | 1.00 | 66.27 | D000 O |
| ATOM | 8302 | CB | ARG | P | 237 | -16.478 | -14.322 | 81.833 | 1.00 | 53.42 | D000 C |
| ATOM | 8303 | CG | ARG | P | 237 | -16.800 | -15.823 | 81.816 | 1.00 | 47.57 | D000 C |
| ATOM | 8304 | CD | ARG | P | 237 | -18.062 | -16.175 | 82.614 | 1.00 | 51.09 | D000 C |
| ATOM | 8305 | NE | ARG | P | 237 | -17.828 | -16.314 | 84.057 | 1.00 | 58.37 | D000 N |
| ATOM | 8306 | CZ | ARG | P | 237 | -17.838 | -17.466 | 84.735 | 1.00 | 56.45 | D000 C |
| ATOM | 8307 | NH1 | ARG | P | 237 | -18.083 | -18.621 | 84.123 | 1.00 | 48.82 | D000 N1+ |
| ATOM | 8308 | NH2 | ARG | P | 237 | -17.612 | -17.462 | 86.046 | 1.00 | 63.83 | D000 N |
| ATOM | 8309 | N | PRO | P | 238 | -17.893 | -11.331 | 81.605 | 1.00 | 62.76 | D000 N |
| ATOM | 8310 | CA | PRO | P | 238 | -17.707 | -9.908 | 81.907 | 1.00 | 69.38 | D000 C |
| ATOM | 8311 | C | PRO | P | 238 | -16.409 | -9.654 | 82.665 | 1.00 | 74.57 | D000 C |
| ATOM | 8312 | O | PRO | P | 238 | -16.040 | -10.408 | 83.571 | 1.00 | 71.66 | D000 O |
| ATOM | 8313 | CB | PRO | P | 238 | -18.942 | -9.573 | 82.746 | 1.00 | 64.98 | D000 C |
| ATOM | 8314 | CG | PRO | P | 238 | -19.987 | -10.474 | 82.171 | 1.00 | 64.63 | D000 C |
| ATOM | 8315 | CD | PRO | P | 238 | -19.285 | -11.760 | 81.814 | 1.00 | 57.83 | D000 C |
| ATOM | 8316 | N | GLU | P | 239 | -15.705 | -8.593 | 82.248 | 1.00 | 76.88 | D000 N |
| ATOM | 8317 | CA | GLU | P | 239 | -14.381 | -8.126 | 82.688 | 1.00 | 83.12 | D000 C |
| ATOM | 8318 | C | GLU | P | 239 | -13.208 | -8.804 | 81.960 | 1.00 | 80.80 | D000 C |
| ATOM | 8319 | O | GLU | P | 239 | -12.049 | -8.510 | 82.297 | 1.00 | 84.63 | D000 O |
| ATOM | 8320 | CB | GLU | P | 239 | -14.170 | -8.253 | 84.207 | 1.00 | 83.81 | D000 C |
| ATOM | 8321 | CG | GLU | P | 239 | -14.580 | -6.991 | 84.959 | 1.00 | 91.55 | D000 C |
| ATOM | 8322 | CD | GLU | P | 239 | -13.794 | -6.763 | 86.239 | 1.00 | 97.32 | D000 C |
| ATOM | 8323 | OE1 | GLU | P | 239 | -14.226 | -7.250 | 87.308 | 1.00 | 93.15 | D000 O |
| ATOM | 8324 | OE2 | GLU | P | 239 | -12.745 | -6.086 | 86.172 | 1.00 | 96.55 | D000 O1- |
| ATOM | 8325 | N | GLN | P | 240 | -13.452 | -9.674 | 80.980 | 1.00 | 74.55 | D000 N |
| ATOM | 8326 | CA | GLN | P | 240 | -12.388 | -10.436 | 80.304 | 1.00 | 70.87 | D000 C |
| ATOM | 8327 | C | GLN | P | 240 | -12.327 | -10.058 | 78.811 | 1.00 | 70.85 | D000 C |
| ATOM | 8328 | O | GLN | P | 240 | -13.346 | -9.637 | 78.253 | 1.00 | 75.89 | D000 O |
| ATOM | 8329 | CB | GLN | P | 240 | -12.619 | -11.949 | 80.462 | 1.00 | 68.58 | D000 C |
| ATOM | 8330 | CG | GLN | P | 240 | -12.717 | -12.433 | 81.899 | 1.00 | 68.41 | D000 C |
| ATOM | 8331 | CD | GLN | P | 240 | -11.414 | -12.251 | 82.660 | 1.00 | 75.96 | D000 C |
| ATOM | 8332 | OE1 | GLN | P | 240 | -10.466 | -13.018 | 82.480 | 1.00 | 70.60 | D000 O |
| ATOM | 8333 | NE2 | GLN | P | 240 | -11.359 | -11.226 | 83.518 | 1.00 | 77.27 | D000 N |
| ATOM | 8334 | N | PRO | P | 241 | -11.147 | -10.193 | 78.150 | 1.00 | 69.18 | D000 N |
| ATOM | 8335 | CA | PRO | P | 241 | -9.849 | -10.736 | 78.592 | 1.00 | 66.74 | D000 C |
| ATOM | 8336 | C | PRO | P | 241 | -9.056 | -9.841 | 79.559 | 1.00 | 73.69 | D000 C |
| ATOM | 8337 | O | PRO | P | 241 | -9.074 | -8.611 | 79.454 | 1.00 | 81.53 | D000 O |
| ATOM | 8338 | CB | PRO | P | 241 | -9.088 | -10.907 | 77.273 | 1.00 | 58.34 | D000 C |
| ATOM | 8339 | CG | PRO | P | 241 | -9.646 | -9.842 | 76.388 | 1.00 | 54.80 | D000 C |
| ATOM | 8340 | CD | PRO | P | 241 | -11.106 | -9.787 | 76.727 | 1.00 | 57.51 | D000 C |
| ATOM | 8341 | N | GLU | P | 253 | -1.712 | -15.376 | 81.188 | 1.00 | 57.88 | D000 N |
| ATOM | 8342 | CA | GLU | P | 253 | -3.063 | -15.516 | 80.648 | 1.00 | 58.93 | D000 C |
| ATOM | 8343 | C | GLU | P | 253 | -3.184 | -15.191 | 79.148 | 1.00 | 57.34 | D000 C |
| ATOM | 8344 | O | GLU | P | 253 | -3.809 | -14.204 | 78.771 | 1.00 | 60.57 | D000 O |
| ATOM | 8345 | CB | GLU | P | 253 | -4.028 | -14.606 | 81.410 | 1.00 | 67.05 | D000 C |
| ATOM | 8346 | CG | GLU | P | 253 | -4.239 | -14.925 | 82.886 | 1.00 | 65.01 | D000 C |
| ATOM | 8347 | CD | GLU | P | 253 | -4.871 | -13.746 | 83.619 | 1.00 | 78.11 | D000 C |
| ATOM | 8348 | OE1 | GLU | P | 253 | -6.039 | -13.406 | 83.290 | 1.00 | 74.19 | D000 O |
| ATOM | 8349 | OE2 | GLU | P | 253 | -4.210 | -13.174 | 84.525 | 1.00 | 80.72 | D000 O1- |
| ATOM | 8350 | N | ASP | P | 254 | -2.582 | -16.015 | 78.297 | 1.00 | 59.04 | D000 N |
| ATOM | 8351 | CA | ASP | P | 254 | -2.582 | -15.774 | 76.863 | 1.00 | 57.72 | D000 C |
| ATOM | 8352 | C | ASP | P | 254 | -3.509 | -16.705 | 76.087 | 1.00 | 51.09 | D000 C |
| ATOM | 8353 | O | ASP | P | 254 | -3.528 | -16.640 | 74.857 | 1.00 | 48.82 | D000 O |
| ATOM | 8354 | CB | ASP | P | 254 | -1.159 | -15.911 | 76.296 | 1.00 | 61.92 | D000 C |
| ATOM | 8355 | CG | ASP | P | 254 | -0.161 | -14.973 | 76.935 | 1.00 | 62.66 | D000 C |
| ATOM | 8356 | OD1 | ASP | P | 254 | -0.567 | -13.960 | 77.547 | 1.00 | 61.96 | D000 O |
| ATOM | 8357 | OD2 | ASP | P | 254 | 1.048 | -15.281 | 76.834 | 1.00 | 65.49 | D000 O1- |
| ATOM | 8358 | N | CYS | P | 255 | -4.265 | -17.574 | 76.753 | 1.00 | 47.05 | D000 N |
| ATOM | 8359 | CA | CYS | P | 255 | -5.063 | -18.571 | 76.051 | 1.00 | 41.26 | D000 C |
| ATOM | 8360 | C | CYS | P | 255 | -6.488 | -18.590 | 76.580 | 1.00 | 38.11 | D000 C |
| ATOM | 8361 | O | CYS | P | 255 | -6.716 | -18.454 | 77.783 | 1.00 | 39.54 | D000 O |
| ATOM | 8362 | CB | CYS | P | 255 | -4.450 | -19.956 | 76.186 | 1.00 | 44.58 | D000 C |
| ATOM | 8363 | SG | CYS | P | 255 | -2.846 | -20.164 | 75.353 | 1.00 | 52.24 | D000 S |
| ATOM | 8364 | N | ALA | P | 256 | -7.440 | -18.776 | 75.674 | 1.00 | 36.50 | D000 N |
| ATOM | 8365 | CA | ALA | P | 256 | -8.857 | -18.645 | 75.980 | 1.00 | 35.39 | D000 C |
| ATOM | 8366 | C | ALA | P | 256 | -9.527 | -20.010 | 76.077 | 1.00 | 31.26 | D000 C |
| ATOM | 8367 | O | ALA | P | 256 | -9.240 | -20.914 | 75.281 | 1.00 | 30.62 | D000 O |
| ATOM | 8368 | CB | ALA | P | 256 | -9.555 | -17.793 | 74.921 | 1.00 | 38.76 | D000 C |
| ATOM | 8369 | N | HIS | P | 257 | -10.412 | -20.157 | 77.066 | 1.00 | 30.17 | D000 N |
| ATOM | 8370 | CA | HIS | P | 257 | -11.139 | -21.406 | 77.272 | 1.00 | 32.66 | D000 C |
| ATOM | 8371 | C | HIS | P | 257 | -12.600 | -21.131 | 77.611 | 1.00 | 33.59 | D000 C |

| | | | | | | | | | | | | |
|---|---|---|---|---|---|---|---|---|---|---|---|---|
| ATOM | 8372 | O | HIS | P | 257 | -12.962 | -20.057 | 78.108 | 1.00 | 31.39 | D000 | O |
| ATOM | 8373 | CB | HIS | P | 257 | -10.517 | -22.263 | 78.384 | 1.00 | 28.03 | D000 | C |
| ATOM | 8374 | CG | HIS | P | 257 | -10.526 | -21.604 | 79.727 | 1.00 | 34.24 | D000 | C |
| ATOM | 8375 | ND1 | HIS | P | 257 | -11.562 | -21.751 | 80.621 | 1.00 | 34.37 | D000 | N |
| ATOM | 8376 | CD2 | HIS | P | 257 | -9.620 | -20.802 | 80.335 | 1.00 | 38.40 | D000 | C |
| ATOM | 8377 | CE1 | HIS | P | 257 | -11.298 | -21.068 | 81.718 | 1.00 | 34.64 | D000 | C |
| ATOM | 8378 | NE2 | HIS | P | 257 | -10.128 | -20.479 | 81.569 | 1.00 | 37.12 | D000 | N |
| ATOM | 8379 | N | PHE | P | 258 | -13.444 | -22.124 | 77.309 | 1.00 | 33.23 | D000 | N |
| ATOM | 8380 | CA | PHE | P | 258 | -14.808 | -22.141 | 77.823 | 1.00 | 32.25 | D000 | C |
| ATOM | 8381 | C | PHE | P | 258 | -14.773 | -22.392 | 79.319 | 1.00 | 32.26 | D000 | C |
| ATOM | 8382 | O | PHE | P | 258 | -13.956 | -23.171 | 79.811 | 1.00 | 29.18 | D000 | O |
| ATOM | 8383 | CB | PHE | P | 258 | -15.652 | -23.232 | 77.153 | 1.00 | 30.58 | D000 | C |
| ATOM | 8384 | CG | PHE | P | 258 | -15.651 | -23.174 | 75.659 | 1.00 | 27.36 | D000 | C |
| ATOM | 8385 | CD1 | PHE | P | 258 | -16.550 | -22.394 | 74.987 | 1.00 | 28.97 | D000 | C |
| ATOM | 8386 | CD2 | PHE | P | 258 | -14.738 | -23.912 | 74.931 | 1.00 | 28.24 | D000 | C |
| ATOM | 8387 | CE1 | PHE | P | 258 | -16.541 | -22.343 | 73.614 | 1.00 | 34.71 | D000 | C |
| ATOM | 8388 | CE2 | PHE | P | 258 | -14.723 | -23.869 | 73.566 | 1.00 | 30.59 | D000 | C |
| ATOM | 8389 | CZ | PHE | P | 258 | -15.623 | -23.079 | 72.902 | 1.00 | 33.75 | D000 | C |
| ATOM | 8390 | N | THR | P | 259 | -15.684 | -21.742 | 80.033 | 1.00 | 36.07 | D000 | N |
| ATOM | 8391 | CA | THR | P | 259 | -15.852 | -21.887 | 81.471 | 1.00 | 37.54 | D000 | C |
| ATOM | 8392 | C | THR | P | 259 | -17.106 | -22.709 | 81.741 | 1.00 | 38.73 | D000 | C |
| ATOM | 8393 | O | THR | P | 259 | -17.882 | -23.018 | 80.828 | 1.00 | 40.70 | D000 | O |
| ATOM | 8394 | CB | THR | P | 259 | -15.949 | -20.510 | 82.148 | 1.00 | 41.98 | D000 | C |
| ATOM | 8395 | OG1 | THR | P | 259 | -17.200 | -19.888 | 81.813 | 1.00 | 41.80 | D000 | O |
| ATOM | 8396 | CG2 | THR | P | 259 | -14.799 | -19.598 | 81.696 | 1.00 | 30.57 | D000 | C |
| ATOM | 8397 | N | ASP | P | 260 | -17.342 | -23.006 | 83.020 | 1.00 | 37.73 | D000 | N |
| ATOM | 8398 | CA | ASP | P | 260 | -18.446 | -23.894 | 83.380 | 1.00 | 37.79 | D000 | C |
| ATOM | 8399 | C | ASP | P | 260 | -19.814 | -23.404 | 82.896 | 1.00 | 38.90 | D000 | C |
| ATOM | 8400 | O | ASP | P | 260 | -20.758 | -24.201 | 82.869 | 1.00 | 41.70 | D000 | O |
| ATOM | 8401 | CB | ASP | P | 260 | -18.483 | -24.128 | 84.901 | 1.00 | 35.64 | D000 | C |
| ATOM | 8402 | CG | ASP | P | 260 | -18.619 | -22.834 | 85.712 | 1.00 | 42.82 | D000 | C |
| ATOM | 8403 | OD1 | ASP | P | 260 | -18.241 | -21.740 | 85.225 | 1.00 | 48.13 | D000 | O |
| ATOM | 8404 | OD2 | ASP | P | 260 | -19.161 | -22.907 | 86.836 | 1.00 | 44.48 | D000 | O1- |
| ATOM | 8405 | N | ASP | P | 261 | -19.955 | -22.136 | 82.499 | 1.00 | 41.54 | D000 | N |
| ATOM | 8406 | CA | ASP | P | 261 | -21.226 | -21.648 | 81.973 | 1.00 | 42.82 | D000 | C |
| ATOM | 8407 | C | ASP | P | 261 | -21.155 | -21.336 | 80.487 | 1.00 | 41.84 | D000 | C |
| ATOM | 8408 | O | ASP | P | 261 | -22.099 | -20.769 | 79.939 | 1.00 | 43.69 | D000 | O |
| ATOM | 8409 | CB | ASP | P | 261 | -21.727 | -20.412 | 82.738 | 1.00 | 43.58 | D000 | C |
| ATOM | 8410 | CG | ASP | P | 261 | -20.950 | -19.119 | 82.416 | 1.00 | 49.58 | D000 | C |
| ATOM | 8411 | OD1 | ASP | P | 261 | -19.993 | -19.120 | 81.605 | 1.00 | 52.04 | D000 | O |
| ATOM | 8412 | OD2 | ASP | P | 261 | -21.333 | -18.065 | 82.974 | 1.00 | 48.73 | D000 | O1- |
| ATOM | 8413 | N | GLY | P | 262 | -20.046 | -21.659 | 79.826 | 1.00 | 41.79 | D000 | N |
| ATOM | 8414 | CA | GLY | P | 262 | -19.923 | -21.469 | 78.406 | 1.00 | 35.80 | D000 | C |
| ATOM | 8415 | C | GLY | P | 262 | -19.239 | -20.181 | 78.005 | 1.00 | 34.51 | D000 | C |
| ATOM | 8416 | O | GLY | P | 262 | -18.649 | -20.125 | 76.926 | 1.00 | 35.57 | D000 | O |
| ATOM | 8417 | N | ARG | P | 263 | -19.315 | -19.141 | 78.829 | 1.00 | 35.18 | D000 | N |
| ATOM | 8418 | CA | ARG | P | 263 | -18.644 | -17.901 | 78.475 | 1.00 | 39.45 | D000 | C |
| ATOM | 8419 | C | ARG | P | 263 | -17.120 | -18.052 | 78.581 | 1.00 | 36.67 | D000 | C |
| ATOM | 8420 | O | ARG | P | 263 | -16.588 | -18.949 | 79.235 | 1.00 | 34.59 | D000 | O |
| ATOM | 8421 | CB | ARG | P | 263 | -19.177 | -16.755 | 79.332 | 1.00 | 39.46 | D000 | C |
| ATOM | 8422 | CG | ARG | P | 263 | -20.633 | -16.427 | 79.008 | 1.00 | 41.29 | D000 | C |
| ATOM | 8423 | CD | ARG | P | 263 | -21.171 | -15.300 | 79.852 | 1.00 | 41.58 | D000 | C |
| ATOM | 8424 | NE | ARG | P | 263 | -21.060 | -15.629 | 81.269 | 1.00 | 57.11 | D000 | N |
| ATOM | 8425 | CZ | ARG | P | 263 | -21.316 | -14.794 | 82.274 | 1.00 | 54.20 | D000 | C |
| ATOM | 8426 | NH1 | ARG | P | 263 | -21.702 | -13.543 | 82.039 | 1.00 | 46.67 | D000 | N1+ |
| ATOM | 8427 | NH2 | ARG | P | 263 | -21.168 | -15.216 | 83.524 | 1.00 | 49.65 | D000 | N |
| ATOM | 8428 | N | TRP | P | 264 | -16.414 | -17.184 | 77.879 | 1.00 | 39.86 | D000 | N |
| ATOM | 8429 | CA | TRP | P | 264 | -14.984 | -17.349 | 77.652 | 1.00 | 38.07 | D000 | C |
| ATOM | 8430 | C | TRP | P | 264 | -14.144 | -16.707 | 78.750 | 1.00 | 36.92 | D000 | C |
| ATOM | 8431 | O | TRP | P | 264 | -14.565 | -15.769 | 79.434 | 1.00 | 38.51 | D000 | O |
| ATOM | 8432 | CB | TRP | P | 264 | -14.596 | -16.743 | 76.311 | 1.00 | 36.59 | D000 | C |
| ATOM | 8433 | CG | TRP | P | 264 | -15.334 | -17.316 | 75.157 | 1.00 | 33.59 | D000 | C |
| ATOM | 8434 | CD1 | TRP | P | 264 | -16.124 | -18.422 | 75.156 | 1.00 | 28.12 | D000 | C |
| ATOM | 8435 | CD2 | TRP | P | 264 | -15.343 | -16.808 | 73.823 | 1.00 | 37.35 | D000 | C |
| ATOM | 8436 | NE1 | TRP | P | 264 | -16.627 | -18.635 | 73.909 | 1.00 | 31.66 | D000 | N |
| ATOM | 8437 | CE2 | TRP | P | 264 | -16.158 | -17.661 | 73.064 | 1.00 | 38.06 | D000 | C |
| ATOM | 8438 | CE3 | TRP | P | 264 | -14.736 | -15.714 | 73.194 | 1.00 | 34.20 | D000 | C |
| ATOM | 8439 | CZ2 | TRP | P | 264 | -16.384 | -17.455 | 71.697 | 1.00 | 36.24 | D000 | C |
| ATOM | 8440 | CZ3 | TRP | P | 264 | -14.966 | -15.510 | 71.854 | 1.00 | 34.62 | D000 | C |
| ATOM | 8441 | CH2 | TRP | P | 264 | -15.783 | -16.375 | 71.118 | 1.00 | 35.86 | D000 | C |
| ATOM | 8442 | N | ASN | P | 265 | -12.908 | -17.179 | 78.854 | 1.00 | 32.60 | D000 | N |

| | | | | | | | | | | | | |
|---|---|---|---|---|---|---|---|---|---|---|---|---|
| ATOM | 8443 | CA | ASN | P | 265 | -11.991 | -16.649 | 79.844 | 1.00 | 35.02 | D000 | C |
| ATOM | 8444 | C | ASN | P | 265 | -10.562 | -16.955 | 79.416 | 1.00 | 35.40 | D000 | C |
| ATOM | 8445 | O | ASN | P | 265 | -10.303 | -17.966 | 78.762 | 1.00 | 34.37 | D000 | O |
| ATOM | 8446 | CB | ASN | P | 265 | -12.302 | -17.268 | 81.206 | 1.00 | 41.49 | D000 | C |
| ATOM | 8447 | CG | ASN | P | 265 | -11.391 | -16.785 | 82.295 | 1.00 | 45.67 | D000 | C |
| ATOM | 8448 | OD1 | ASN | P | 265 | -11.470 | -15.634 | 82.725 | 1.00 | 46.99 | D000 | O |
| ATOM | 8449 | ND2 | ASN | P | 265 | -10.486 | -17.660 | 82.730 | 1.00 | 42.25 | D000 | N |
| ATOM | 8450 | N | ASP | P | 266 | -9.635 | -16.081 | 79.804 | 1.00 | 41.02 | D000 | N |
| ATOM | 8451 | CA | ASP | P | 266 | -8.210 | -16.284 | 79.555 | 1.00 | 44.53 | D000 | C |
| ATOM | 8452 | C | ASP | P | 266 | -7.533 | -16.874 | 80.790 | 1.00 | 44.46 | D000 | C |
| ATOM | 8453 | O | ASP | P | 266 | -7.836 | -16.493 | 81.922 | 1.00 | 42.76 | D000 | O |
| ATOM | 8454 | CB | ASP | P | 266 | -7.513 | -14.977 | 79.143 | 1.00 | 48.13 | D000 | C |
| ATOM | 8455 | CG | ASP | P | 266 | -7.889 | -13.788 | 80.036 | 1.00 | 60.01 | D000 | C |
| ATOM | 8456 | OD1 | ASP | P | 266 | -9.096 | -13.510 | 80.179 | 1.00 | 60.58 | D000 | O |
| ATOM | 8457 | OD2 | ASP | P | 266 | -6.988 | -13.158 | 80.633 | 1.00 | 64.47 | D000 | O1- |
| ATOM | 8458 | N | ASP | P | 267 | -6.622 | -17.815 | 80.563 | 1.00 | 44.24 | D000 | N |
| ATOM | 8459 | CA | ASP | P | 267 | -5.909 | -18.469 | 81.647 | 1.00 | 42.61 | D000 | C |
| ATOM | 8460 | C | ASP | P | 267 | -4.508 | -18.821 | 81.152 | 1.00 | 44.14 | D000 | C |
| ATOM | 8461 | O | ASP | P | 267 | -4.233 | -18.787 | 79.952 | 1.00 | 41.89 | D000 | O |
| ATOM | 8462 | CB | ASP | P | 267 | -6.688 | -19.697 | 82.122 | 1.00 | 38.52 | D000 | C |
| ATOM | 8463 | CG | ASP | P | 267 | -6.223 | -20.188 | 83.464 | 1.00 | 45.95 | D000 | C |
| ATOM | 8464 | OD1 | ASP | P | 267 | -5.365 | -19.510 | 84.068 | 1.00 | 48.74 | D000 | O |
| ATOM | 8465 | OD2 | ASP | P | 267 | -6.710 | -21.244 | 83.919 | 1.00 | 43.95 | D000 | O1- |
| ATOM | 8466 | N | VAL | P | 268 | -3.612 | -19.180 | 82.076 | 1.00 | 47.41 | D000 | N |
| ATOM | 8467 | CA | VAL | P | 268 | -2.258 | -19.507 | 81.643 | 1.00 | 46.28 | D000 | C |
| ATOM | 8468 | C | VAL | P | 268 | -2.306 | -20.765 | 80.793 | 1.00 | 45.81 | D000 | C |
| ATOM | 8469 | O | VAL | P | 268 | -3.051 | -21.717 | 81.079 | 1.00 | 42.24 | D000 | O |
| ATOM | 8470 | CB | VAL | P | 268 | -1.274 | -19.643 | 82.822 | 1.00 | 45.28 | D000 | C |
| ATOM | 8471 | CG1 | VAL | P | 268 | -1.359 | -18.423 | 83.707 | 1.00 | 40.04 | D000 | C |
| ATOM | 8472 | CG2 | VAL | P | 268 | -1.489 | -20.937 | 83.609 | 1.00 | 40.72 | D000 | C |
| ATOM | 8473 | N | CYS | P | 269 | -1.525 | -20.756 | 79.717 | 1.00 | 45.30 | D000 | N |
| ATOM | 8474 | CA | CYS | P | 269 | -1.620 | -21.777 | 78.695 | 1.00 | 34.95 | D000 | C |
| ATOM | 8475 | C | CYS | P | 269 | -1.113 | -23.121 | 79.167 | 1.00 | 39.62 | D000 | C |
| ATOM | 8476 | O | CYS | P | 269 | -1.267 | -24.107 | 78.438 | 1.00 | 44.44 | D000 | O |
| ATOM | 8477 | CB | CYS | P | 269 | -0.849 | -21.317 | 77.456 | 1.00 | 47.03 | D000 | C |
| ATOM | 8478 | SG | CYS | P | 269 | -1.435 | -19.698 | 76.766 | 1.00 | 60.08 | D000 | S |
| ATOM | 8479 | N | GLN | P | 270 | -0.551 | -23.199 | 80.371 | 1.00 | 40.97 | D000 | N |
| ATOM | 8480 | CA | GLN | P | 270 | 0.007 | -24.461 | 80.837 | 1.00 | 39.29 | D000 | C |
| ATOM | 8481 | C | GLN | P | 270 | -1.038 | -25.378 | 81.448 | 1.00 | 33.15 | D000 | C |
| ATOM | 8482 | O | GLN | P | 270 | -0.769 | -26.577 | 81.579 | 1.00 | 30.99 | D000 | O |
| ATOM | 8483 | CB | GLN | P | 270 | 1.128 | -24.207 | 81.857 | 1.00 | 52.36 | D000 | C |
| ATOM | 8484 | CG | GLN | P | 270 | 2.531 | -23.978 | 81.256 | 1.00 | 68.27 | D000 | C |
| ATOM | 8485 | CD | GLN | P | 270 | 3.672 | -24.458 | 82.188 | 1.00 | 90.18 | D000 | C |
| ATOM | 8486 | OE1 | GLN | P | 270 | 3.426 | -25.009 | 83.272 | 1.00 | 96.23 | D000 | O |
| ATOM | 8487 | NE2 | GLN | P | 270 | 4.921 | -24.250 | 81.760 | 1.00 | 83.33 | D000 | N |
| ATOM | 8488 | N | ARG | P | 271 | -2.213 | -24.847 | 81.803 | 1.00 | 35.97 | D000 | N |
| ATOM | 8489 | CA | ARG | P | 271 | -3.258 | -25.641 | 82.453 | 1.00 | 35.38 | D000 | C |
| ATOM | 8490 | C | ARG | P | 271 | -3.614 | -26.831 | 81.577 | 1.00 | 34.50 | D000 | C |
| ATOM | 8491 | O | ARG | P | 271 | -3.835 | -26.657 | 80.369 | 1.00 | 35.09 | D000 | O |
| ATOM | 8492 | CB | ARG | P | 271 | -4.533 | -24.825 | 82.699 | 1.00 | 32.78 | D000 | C |
| ATOM | 8493 | CG | ARG | P | 271 | -4.404 | -23.700 | 83.663 | 1.00 | 35.79 | D000 | C |
| ATOM | 8494 | CD | ARG | P | 271 | -4.885 | -24.052 | 85.062 | 1.00 | 38.85 | D000 | C |
| ATOM | 8495 | NE | ARG | P | 271 | -4.351 | -23.079 | 86.026 | 1.00 | 53.17 | D000 | N |
| ATOM | 8496 | CZ | ARG | P | 271 | -3.487 | -23.363 | 87.009 | 1.00 | 57.60 | D000 | C |
| ATOM | 8497 | NH1 | ARG | P | 271 | -3.061 | -24.616 | 87.211 | 1.00 | 48.15 | D000 | N1+ |
| ATOM | 8498 | NH2 | ARG | P | 271 | -3.054 | -22.387 | 87.805 | 1.00 | 54.98 | D000 | N |
| ATOM | 8499 | N | PRO | P | 272 | -3.709 | -27.959 | 82.102 | 1.00 | 34.18 | D000 | N |
| ATOM | 8500 | CA | PRO | P | 272 | -4.104 | -29.152 | 81.304 | 1.00 | 30.39 | D000 | C |
| ATOM | 8501 | C | PRO | P | 272 | -5.617 | -29.326 | 81.191 | 1.00 | 30.82 | D000 | C |
| ATOM | 8502 | O | PRO | P | 272 | -6.222 | -30.227 | 81.774 | 1.00 | 30.88 | D000 | O |
| ATOM | 8503 | CB | PRO | P | 272 | -3.446 | -30.296 | 82.079 | 1.00 | 29.98 | D000 | C |
| ATOM | 8504 | CG | PRO | P | 272 | -3.279 | -29.776 | 83.527 | 1.00 | 33.19 | D000 | C |
| ATOM | 8505 | CD | PRO | P | 272 | -3.502 | -28.269 | 83.527 | 1.00 | 30.33 | D000 | C |
| ATOM | 8506 | N | TYR | P | 273 | -6.260 | -28.438 | 80.435 | 1.00 | 29.69 | D000 | N |
| ATOM | 8507 | CA | TYR | P | 273 | -7.685 | -28.556 | 80.183 | 1.00 | 26.06 | D000 | C |
| ATOM | 8508 | C | TYR | P | 273 | -7.957 | -29.510 | 79.021 | 1.00 | 31.32 | D000 | C |
| ATOM | 8509 | O | TYR | P | 273 | -7.075 | -29.834 | 78.220 | 1.00 | 34.09 | D000 | O |
| ATOM | 8510 | CB | TYR | P | 273 | -8.289 | -27.199 | 79.848 | 1.00 | 29.36 | D000 | C |
| ATOM | 8511 | CG | TYR | P | 273 | -8.313 | -26.189 | 80.968 | 1.00 | 31.45 | D000 | C |
| ATOM | 8512 | CD1 | TYR | P | 273 | -8.404 | -26.586 | 82.310 | 1.00 | 29.10 | D000 | C |
| ATOM | 8513 | CD2 | TYR | P | 273 | -8.243 | -24.832 | 80.685 | 1.00 | 26.14 | D000 | C |

| | | | | | | | | | | | | |
|---|---|---|---|---|---|---|---|---|---|---|---|---|
| ATOM | 8514 | CE1 | TYR | P | 273 | -8.422 | -25.650 | 83.325 | 1.00 | 23.99 | D000 | C |
| ATOM | 8515 | CE2 | TYR | P | 273 | -8.275 | -23.900 | 81.688 | 1.00 | 30.08 | D000 | C |
| ATOM | 8516 | CZ | TYR | P | 273 | -8.364 | -24.312 | 83.001 | 1.00 | 27.73 | D000 | C |
| ATOM | 8517 | OH | TYR | P | 273 | -8.380 | -23.357 | 83.973 | 1.00 | 30.77 | D000 | O |
| ATOM | 8518 | N | ARG | P | 274 | -9.208 | -29.949 | 78.931 | 1.00 | 28.38 | D000 | N |
| ATOM | 8519 | CA | ARG | P | 274 | -9.699 | -30.608 | 77.733 | 1.00 | 26.38 | D000 | C |
| ATOM | 8520 | C | ARG | P | 274 | -9.755 | -29.604 | 76.570 | 1.00 | 28.97 | D000 | C |
| ATOM | 8521 | O | ARG | P | 274 | -9.693 | -28.383 | 76.762 | 1.00 | 29.05 | D000 | O |
| ATOM | 8522 | CB | ARG | P | 274 | -11.090 | -31.183 | 78.003 | 1.00 | 26.93 | D000 | C |
| ATOM | 8523 | CG | ARG | P | 274 | -11.108 | -32.298 | 78.990 | 1.00 | 27.16 | D000 | C |
| ATOM | 8524 | CD | ARG | P | 274 | -12.512 | -32.728 | 79.284 | 1.00 | 29.23 | D000 | C |
| ATOM | 8525 | NE | ARG | P | 274 | -12.531 | -33.815 | 80.262 | 1.00 | 31.79 | D000 | N |
| ATOM | 8526 | CZ | ARG | P | 274 | -13.610 | -34.234 | 80.917 | 1.00 | 28.61 | D000 | C |
| ATOM | 8527 | NH1 | ARG | P | 274 | -14.801 | -33.662 | 80.721 | 1.00 | 27.83 | D000 | N1+ |
| ATOM | 8528 | NH2 | ARG | P | 274 | -13.495 | -35.241 | 81.767 | 1.00 | 30.68 | D000 | N |
| ATOM | 8529 | N | TRP | P | 275 | -9.894 | -30.113 | 75.345 | 1.00 | 23.49 | D000 | N |
| ATOM | 8530 | CA | TRP | P | 275 | -9.938 | -29.226 | 74.188 | 1.00 | 25.18 | D000 | C |
| ATOM | 8531 | C | TRP | P | 275 | -10.855 | -29.837 | 73.136 | 1.00 | 28.35 | D000 | C |
| ATOM | 8532 | O | TRP | P | 275 | -11.240 | -31.009 | 73.214 | 1.00 | 27.63 | D000 | O |
| ATOM | 8533 | CB | TRP | P | 275 | -8.524 | -28.953 | 73.614 | 1.00 | 26.97 | D000 | C |
| ATOM | 8534 | CG | TRP | P | 275 | -7.949 | -30.164 | 72.960 | 1.00 | 26.87 | D000 | C |
| ATOM | 8535 | CD1 | TRP | P | 275 | -7.973 | -30.473 | 71.628 | 1.00 | 25.57 | D000 | C |
| ATOM | 8536 | CD2 | TRP | P | 275 | -7.346 | -31.284 | 73.625 | 1.00 | 26.80 | D000 | C |
| ATOM | 8537 | NE1 | TRP | P | 275 | -7.403 | -31.713 | 71.419 | 1.00 | 26.56 | D000 | N |
| ATOM | 8538 | CE2 | TRP | P | 275 | -7.008 | -32.230 | 72.626 | 1.00 | 28.11 | D000 | C |
| ATOM | 8539 | CE3 | TRP | P | 275 | -7.044 | -31.573 | 74.966 | 1.00 | 24.47 | D000 | C |
| ATOM | 8540 | CZ2 | TRP | P | 275 | -6.388 | -33.439 | 72.926 | 1.00 | 26.32 | D000 | C |
| ATOM | 8541 | CZ3 | TRP | P | 275 | -6.439 | -32.772 | 75.265 | 1.00 | 25.01 | D000 | C |
| ATOM | 8542 | CH2 | TRP | P | 275 | -6.114 | -33.692 | 74.250 | 1.00 | 29.39 | D000 | C |
| ATOM | 8543 | N | VAL | P | 276 | -11.186 | -29.023 | 72.135 | 1.00 | 27.49 | D000 | N |
| ATOM | 8544 | CA | VAL | P | 276 | -12.064 | -29.405 | 71.039 | 1.00 | 29.28 | D000 | C |
| ATOM | 8545 | C | VAL | P | 276 | -11.347 | -29.157 | 69.720 | 1.00 | 32.15 | D000 | C |
| ATOM | 8546 | O | VAL | P | 276 | -11.010 | -28.009 | 69.399 | 1.00 | 30.70 | D000 | O |
| ATOM | 8547 | CB | VAL | P | 276 | -13.381 | -28.615 | 71.076 | 1.00 | 32.24 | D000 | C |
| ATOM | 8548 | CG1 | VAL | P | 276 | -14.255 | -28.997 | 69.881 | 1.00 | 28.45 | D000 | C |
| ATOM | 8549 | CG2 | VAL | P | 276 | -14.099 | -28.838 | 72.418 | 1.00 | 27.44 | D000 | C |
| ATOM | 8550 | N | CYS | P | 277 | -11.188 | -30.212 | 68.926 | 1.00 | 31.02 | D000 | N |
| ATOM | 8551 | CA | CYS | P | 277 | -10.688 | -30.089 | 67.560 | 1.00 | 33.70 | D000 | C |
| ATOM | 8552 | C | CYS | P | 277 | -11.856 | -29.883 | 66.594 | 1.00 | 36.60 | D000 | C |
| ATOM | 8553 | O | CYS | P | 277 | -12.924 | -30.476 | 66.769 | 1.00 | 35.91 | D000 | O |
| ATOM | 8554 | CB | CYS | P | 277 | -9.893 | -31.339 | 67.158 | 1.00 | 33.14 | D000 | C |
| ATOM | 8555 | SG | CYS | P | 277 | -8.261 | -31.627 | 67.947 | 1.00 | 35.59 | D000 | S |
| ATOM | 8556 | N | GLU | P | 278 | -11.644 | -29.037 | 65.572 | 1.00 | 36.89 | D000 | N |
| ATOM | 8557 | CA | GLU | P | 278 | -12.627 | -28.753 | 64.525 | 1.00 | 39.14 | D000 | C |
| ATOM | 8558 | C | GLU | P | 278 | -11.998 | -28.940 | 63.144 | 1.00 | 44.48 | D000 | C |
| ATOM | 8559 | O | GLU | P | 278 | -10.812 | -28.657 | 62.944 | 1.00 | 39.34 | D000 | O |
| ATOM | 8560 | CB | GLU | P | 278 | -13.201 | -27.309 | 64.639 | 1.00 | 33.16 | D000 | C |
| ATOM | 8561 | CG | GLU | P | 278 | -14.353 | -26.998 | 63.675 | 1.00 | 36.59 | D000 | C |
| ATOM | 8562 | CD | GLU | P | 278 | -14.820 | -25.530 | 63.719 | 1.00 | 44.66 | D000 | C |
| ATOM | 8563 | OE1 | GLU | P | 278 | -14.202 | -24.714 | 64.445 | 1.00 | 39.06 | D000 | O |
| ATOM | 8564 | OE2 | GLU | P | 278 | -15.829 | -25.200 | 63.049 | 1.00 | 41.28 | D000 | O1- |
| ATOM | 8565 | N | THR | P | 279 | -12.790 | -29.443 | 62.197 | 1.00 | 50.44 | D000 | N |
| ATOM | 8566 | CA | THR | P | 279 | -12.402 | -29.459 | 60.790 | 1.00 | 53.12 | D000 | C |
| ATOM | 8567 | C | THR | P | 279 | -13.617 | -29.195 | 59.898 | 1.00 | 49.51 | D000 | C |
| ATOM | 8568 | O | THR | P | 279 | -14.703 | -29.717 | 60.154 | 1.00 | 53.00 | D000 | O |
| ATOM | 8569 | CB | THR | P | 279 | -11.717 | -30.798 | 60.436 | 1.00 | 45.79 | D000 | C |
| ATOM | 8570 | OG1 | THR | P | 279 | -11.049 | -30.673 | 59.179 | 1.00 | 51.68 | D000 | O |
| ATOM | 8571 | CG2 | THR | P | 279 | -12.709 | -31.937 | 60.373 | 1.00 | 41.88 | D000 | C |
| ATOM | 8572 | N | GLU | P | 280 | -13.431 | -28.398 | 58.839 | 1.00 | 54.11 | D000 | N |
| ATOM | 8573 | CA | GLU | P | 280 | -14.518 | -28.026 | 57.929 | 1.00 | 55.44 | D000 | C |
| ATOM | 8574 | C | GLU | P | 280 | -14.664 | -29.061 | 56.808 | 1.00 | 60.78 | D000 | C |
| ATOM | 8575 | O | GLU | P | 280 | -13.673 | -29.610 | 56.323 | 1.00 | 66.29 | D000 | O |
| ATOM | 8576 | CB | GLU | P | 280 | -14.275 | -26.632 | 57.350 | 1.00 | 58.37 | D000 | C |
| ATOM | 8577 | CG | GLU | P | 280 | -14.341 | -25.476 | 58.376 | 1.00 | 56.42 | D000 | C |
| ATOM | 8578 | CD | GLU | P | 280 | -13.124 | -25.396 | 59.315 | 1.00 | 59.60 | D000 | C |
| ATOM | 8579 | OE1 | GLU | P | 280 | -12.166 | -26.179 | 59.134 | 1.00 | 63.77 | D000 | O |
| ATOM | 8580 | OE2 | GLU | P | 280 | -13.129 | -24.553 | 60.247 | 1.00 | 61.91 | D000 | O1- |
| ATOM | 8581 | N | LEU | P | 281 | -15.911 | -29.308 | 56.389 | 1.00 | 64.56 | D000 | N |
| ATOM | 8582 | CA | LEU | P | 281 | -16.292 | -30.535 | 55.682 | 1.00 | 69.60 | D000 | C |
| ATOM | 8583 | C | LEU | P | 281 | -16.310 | -30.448 | 54.149 | 1.00 | 73.85 | D000 | C |
| ATOM | 8584 | O | LEU | P | 281 | -16.945 | -31.306 | 53.521 | 1.00 | 77.45 | D000 | O |

```
ATOM   8585  CB  LEU P 281     -17.662 -31.015  56.164  1.00 60.03       D000 C
ATOM   8586  CG  LEU P 281     -17.690 -32.374  56.863  1.00 56.08       D000 C
ATOM   8587  CD1 LEU P 281     -19.116 -32.776  57.171  1.00 66.41       D000 C
ATOM   8588  CD2 LEU P 281     -17.006 -33.448  56.066  1.00 58.00       D000 C
ATOM   8589  N   ASP P 282     -15.668 -29.445  53.538  1.00 74.94       D000 N
ATOM   8590  CA  ASP P 282     -15.590 -29.289  52.056  1.00 74.61       D000 C
ATOM   8591  C   ASP P 282     -16.879 -28.715  51.457  1.00 69.05       D000 C
ATOM   8592  O   ASP P 282     -17.285 -27.589  51.750  1.00 68.03       D000 O
ATOM   8593  CB  ASP P 282     -15.291 -30.618  51.327  1.00 75.72       D000 C
ATOM   8594  CG  ASP P 282     -13.973 -31.262  51.748  1.00 83.45       D000 C
ATOM   8595  OD2 ASP P 282     -14.017 -32.153  52.639  1.00 81.11       D000 O
ATOM   8596  OD1 ASP P 282     -12.912 -30.910  51.169  1.00 76.47       D000 O1-
TER
HETATM 8597 CA    CA E   1     -46.046 -40.018  63.255  1.00 40.77           Ca
HETATM 8598 CA    CA E   2     -15.968 -22.911  63.354  1.00 43.19           Ca
TER
```

**Claims**

1. An interfering RNA of: asialoglycoprotein receptor 1 (ASGR-1), and/or asialoglycoprotein receptor 2 (ASGR-2) for use for treating or preventing a cardiovascular disease in a patient.

2. The interfering RNA for use of claim 1, wherein the interfering RNA is siRNA or shRNA.

3. The interfering RNA for use of claim 1,

   wherein the interfering RNA comprises a double-stranded RNA molecule which comprises two antiparallel strands of contiguous nucleotides that are sufficiently complementary to each other to hybridize to form a duplex region;
   wherein the interfering RNA is a double-stranded RNA molecule that comprises a sense strand and an antisense strand, wherein the sense strand comprises a sequence that is sufficiently complementary to the sequence of the antisense strand such that the two strands hybridize under physiological conditions to form a duplex region of about 15 to about 30 base pairs, or about 19 to about 21 base pairs in length; or
   wherein the interfering RNA is an siRNA formed from two separate strands of a sense strand and an antisense strand that hybridize to form a duplex region but are otherwise unconnected.

4. The interfering RNA for use of any one of claims 1-3, wherein

   the cardiovascular disease is coronary artery disease or myocardial infarction;
   the interfering RNA reduces blood LDL cholesterol level of the patient by at least 15% as compared to a predose level of blood LDL cholesterol in the patient;
   the interfering RNA reduces non-HDL cholesterol level of the patient by at least 5% as compared to a predose level of non-HDL cholesterol in the patient; or
   the interfering RNA increases ALP level of the patient by at least 30% as compared to a predose ALP level in the patient.

5. The interfering RNA for use of any one of claims 1-4, wherein

   the interfering RNA is a double-stranded RNA that comprises one or more modified nucleotides, and/or
   the interfering RNA is coupled to a targeting moiety, optionally wherein the interfering RNA is specifically targeted to liver cells using the surface expressed ASGR-1 and/or ASGR-2, and wherein expression of ASGR-1 and/or ASGR-2 is reduced in the liver cells due to the effect of the targeted interfering RNA.

6. The interfering RNA for use of any one of claims 1-5, wherein the interfering RNA is administered in combination with one or more other therapeutic agents useful for treating or preventing cardiovascular disease, wherein the interfering RNA is administered simultaneously, prior to, concurrent with, or subsequent to administering the one or more other therapeutic agents,
   optionally wherein the one or more other therapeutic agents is a statin, an anti- PCSK9 antibody or a combination thereof.

7. The interfering RNA for use of claim 1 that is an siRNA targeting ASGR-1, wherein the siRNA comprises:

   a sense strand; and
   an antisense strand,
   wherein the antisense strand comprises a region having a sequence that is substantially or fully complementary to an ASGR-1 mRNA sequence, and
   wherein the sense strand comprises a sequence that is sufficiently complementary to the sequence of the antisense strand such that the two strands hybridize under physiological conditions to form a duplex region that is about 15 to about 30 base pairs in length.

8. The interfering RNA for use of any one of claims 1-6, wherein the interfering RNA is a double-stranded RNA molecule comprising:

   a sense strand; and
   an antisense strand that is 15 to 30 nucleotides in length, and comprises a region having a sequence that is fully complementary to an ASGR-1 mRNA sequence and/or an ASGR-2 mRNA sequence.

9. The interfering RNA for use of any one of claims 1-6, wherein the interfering RNA is siRNA formed from two separate strands of a sense strand and an antisense strand that hybridize to form a duplex region but are otherwise unconnected, wherein

   the siRNA comprises a duplex region of about 19 to about 25 base pairs in length; or
   the siRNA comprises:

      a sense strand of about 15-30 nucleotides; and
      an antisense strand of about 15-30 nucleotides; or

   the siRNA comprises:

      a sense strand of about 19-25 nucleotides and
      an antisense strand of about 19-25 nucleotides.

10. The interfering RNA for use of any one of claims 1-6, 8 and 9, wherein said interfering RNA is an inhibitor of ASGR-1.

11. The interfering RNA for use of any one of claims 1-6, 8 and 9, wherein said interfering RNA is an inhibitor of ASGR-2.

12. The interfering RNA for use of claim 1, wherein the interfering RNA is a double- stranded RNA molecule that comprises a sense strand and an antisense strand, wherein the antisense strand comprises a region having a sequence that is substantially or fully complementary to an ASGR-1 mRNA sequence encoding a human ASGR-1 protein.

13. The interfering RNA for use of claim 1, wherein the interfering RNA is a double- stranded RNA molecule that comprises a sense strand and an antisense strand, wherein the antisense strand comprises a region having a sequence that is substantially or fully complementary to an ASGR-2 mRNA sequence encoding a human ASGR-2 protein.

14. The siRNA for use of claim 7, wherein the cardiovascular disease is coronary artery disease or myocardial infarction.

## Patentansprüche

1. Interferierende RNS von: Asialoglycoprotein-Rezeptor 1 (ASGR-1) und/oder Asialoglycoprotein-Rezeptor 2 (ASGR-2) zur Verwendung zur Behandlung oder Vorbeugung einer Herz-Kreislauf-Erkrankung bei einem Patienten.

2. Interferierende RNS zur Verwendung nach Anspruch 1, wobei die interferierende RNS siRNS oder shRNS ist.

3. Interferierende RNS zur Verwendung nach Anspruch 1,

wobei die interferierende RNS ein doppelsträngiges RNS-Molekül umfasst, das zwei antiparallele Stränge aneinandergrenzender Nukleotide umfasst, die ausreichend komplementär zueinander sind, um zu hybridisieren und eine Duplexregion zu bilden;
wobei die interferierende RNS ein doppelsträngiges RNS-Molekül ist, das einen Sense- Strang und einen Antisense-Strang umfasst, wobei der Sense-Strang eine Sequenz umfasst, die ausreichend komplementär zur Sequenz des Antisense-Strangs ist, sodass die beiden Stränge unter physiologischen Bedingungen hybridisieren, um eine Duplexregion mit einer Länge von etwa 15 bis etwa 30 Basenpaaren oder etwa 19 bis etwa 21 Basenpaaren zu bilden; oder
wobei die interferierende RNS eine siRNS ist, die aus zwei separaten Strängen eines Sense-Strangs und eines Antisense-Strangs gebildet wird, die hybridisieren, um eine Duplexregion zu bilden, ansonsten aber nicht verbunden sind.

**4.** Interferierende RNS zur Verwendung nach einem der Ansprüche 1-3, wobei

die Herz-Kreislauf-Erkrankung eine koronare Herzkrankheit oder ein Myokardinfarkt ist;
die interferierende RNS den LDL-Cholesterinspiegel im Blut des Patienten um mindestens 15 % senkt im Vergleich zu einem LDL-Cholesterinspiegel im Blut des Patienten vor der Verabreichung;
die interferierende RNS den Nicht-HDL-Cholesterinspiegel des Patienten um mindestens 5 % senkt im Vergleich zu einem Nicht-HDL-Cholesterinspiegel des Patienten vor der Verabreichung; oder
die interferierende RNS den ALP-Spiegel des Patienten um mindestens 30 % erhöht im Vergleich zum ALP-Spiegel des Patienten vor der Verabreichung.

**5.** Interferierende RNS zur Verwendung nach einem der Ansprüche 1-4, wobei

die interferierende RNS eine doppelsträngige RNS ist, die ein oder mehrere modifizierte Nukleotide umfasst, und/oder
die interferierende RNS an eine Targeting-Einheit gekoppelt ist, wobei wahlweise die interferierende RNS unter Verwendung des oberflächenexprimierten ASGR-1 und/oder ASGR-2 spezifisch auf Leberzellen gezielt wird und wobei die Expression von ASGR-1 und/oder ASGR-2 in den Leberzellen aufgrund der Wirkung der zielgerichteten interferierenden RNS reduziert wird.

**6.** Interferierende RNS zur Verwendung nach einem der Ansprüche 1-5, wobei die interferierende RNS in Kombination mit einem oder mehreren anderen therapeutischen Mitteln verabreicht wird, die zur Behandlung oder Vorbeugung von Herz-Kreislauf- Erkrankungen nützlich sind, wobei die interferierende RNS gleichzeitig mit, vor, parallel oder im Anschluss an die Verabreichung des einen oder der mehreren anderen therapeutischen Wirkstoffe verabreicht wird,
wobei wahlweise das eine oder die mehreren anderen therapeutischen Mittel ein Statin, ein Anti-PCSK9-Antikörper oder eine Kombination davon ist/sind.

**7.** Interferierende RNS zur Verwendung nach Anspruch 1, die eine auf ASGR-1 zielgerichtete siRNS ist, wobei die siRNS Folgendes umfasst:

einen Sense-Strang; und einen Antisense-Strang,
wobei der Antisense-Strang eine Region mit einer Sequenz umfasst, die im Wesentlichen oder vollständig komplementär zu einer ASGR-1 mRNS-Sequenz ist, und
wobei der Sense-Strang eine Sequenz umfasst, die ausreichend komplementär zur Sequenz des Antisense-Strangs ist, sodass die beiden Stränge unter physiologischen Bedingungen hybridisieren, um eine Duplexregion mit einer Länge von etwa 15 bis etwa 30 Basenpaaren zu bilden.

**8.** Interferierende RNS zur Verwendung nach einem der Ansprüche 1-6, wobei die interferierende RNS ein doppelsträngiges RNS-Molekül ist, das Folgendes umfasst:

einen Sense-Strang; und
ein Antisense-Strang, der 15 bis 30 Nukleotide lang ist und eine Region mit einer Sequenz umfasst, die vollständig komplementär zu einer ASGR-1 mRNS-Sequenz und/oder einer ASGR-2 mRNS-Sequenz ist.

**9.** Interferierende RNS zur Verwendung nach einem der Ansprüche 1-6, wobei die interferierende RNS siRNS ist, die aus zwei separaten Strängen eines Sense-Strangs und eines Antisense-Strangs gebildet wird, die hybridisieren, um eine Duplexregion zu bilden, aber ansonsten nicht verbunden sind, wobei

die siRNS eine Duplexregion mit einer Länge von etwa 19 bis etwa 25 Basenpaaren umfasst; oder
die siRNS Folgendes umfasst:

einen Sense-Strang von etwa 15-30 Nukleotiden; und einen Antisense-Strang von etwa 15-30 Nukleotiden; oder
die siRNS Folgendes umfasst:
einen Sense-Strang von etwa 19-25 Nukleotiden und einen Antisense-Strang von etwa 19-25 Nukleotiden.

**10.** Interferierende RNS zur Verwendung nach einem der Ansprüche 1-6, 8 und 9, wobei die interferierende RNS ein Inhibitor von ASGR-1 ist.

**11.** Interferierende RNS zur Verwendung nach einem der Ansprüche 1-6, 8 und 9, wobei die interferierende RNS ein Inhibitor von ASGR-2 ist.

**12.** Interferierende RNS zur Verwendung nach Anspruch 1, wobei die interferierende RNS ein doppelsträngiges RNS-Molekül ist, das einen Sense-Strang und einen Antisense- Strang umfasst, wobei der Antisense-Strang eine Region mit einer Sequenz umfasst, die im Wesentlichen oder vollständig komplementär zu einer ASGR-1 mRNS-Sequenz ist, die für ein menschliches ASGR-1 Protein kodiert.

**13.** Interferierende RNS zur Verwendung nach Anspruch 1, wobei die interferierende RNS ein doppelsträngiges RNS-Molekül ist, das einen Sense-Strang und einen Antisense- Strang umfasst, wobei der Antisense-Strang eine Region mit einer Sequenz umfasst, die im Wesentlichen oder vollständig komplementär zu einer ASGR-2 mRNS-Sequenz ist, die für ein menschliches ASGR-2 Protein kodiert.

**14.** siRNS zur Verwendung nach Anspruch 7, wobei die Herz-Kreislauf-Erkrankung eine koronare Herzkrankheit oder ein Myokardinfarkt ist.

## Revendications

**1.** ARN interférent du récepteur 1 de l'asialoglycoprotéine (ASGR-1) et/ou du récepteur 2 de l'asialoglycoprotéine (ASGR-2) destiné au traitement ou à la prévention d'une maladie cardiovasculaire chez un patient.

**2.** ARN interférent destiné à être utilisé selon la revendication 1, dans lequel l'ARN interférent est un siARN ou un shARN.

**3.** ARN interférent destiné à être utilisé selon la revendication 1,

dans lequel l'ARN interférent comprend une molécule d'ARN double brin qui comprend deux brins antiparallèles de nucléotides contigus qui sont suffisamment complémentaires l'un de l'autre pour s'hybrider et former une région duplex ;
dans lequel l'ARN interférent est une molécule d'ARN double brin qui comprend un brin sens et un brin antisens, dans lequel le brin sens comprend une séquence qui est suffisamment complémentaire de la séquence du brin antisens pour que les deux brins s'hybrident dans des conditions physiologiques pour former une région duplex d'une longueur allant d'environ 15 à environ 30 paires de bases, ou allant d'environ 19 à environ 21 paires de bases ; ou
dans lequel l'ARN interférent est un siARN formé de deux brins distincts d'un brin sens et d'un brin antisens qui s'hybrident pour former une région duplex mais ne sont pas reliés par ailleurs.

**4.** ARN interférent destiné à être utilisé selon l'une quelconque des revendications 1 à 3, dans lequel la maladie cardiovasculaire est une maladie coronarienne ou un infarctus du myocarde ;

l'ARN interférent réduit le taux de cholestérol LDL sanguin du patient d'au moins 15 % par rapport à un niveau pré-dosé de cholestérol LDL sanguin chez le patient ;
l'ARN interférent réduit le taux de cholestérol non-HDL du patient d'au moins 5 % par rapport à un niveau de cholestérol non-HDL pré-dosé chez le patient ; ou
l'ARN interférent augmente le taux d'ALP du patient d'au moins 30 % par rapport à un niveau d'ALP pré-dosé chez le patient.

5. ARN interférent destiné à être utilisé selon l'une quelconque des revendications 1 à 4, dans lequel

l'ARN interférent est un ARN double brin qui comprend un ou plusieurs nucléotides modifiés, et/ou
l'ARN interférent est couplé à une fraction de ciblage, éventuellement dans lequel l'ARN interférent est spécifiquement ciblé sur les cellules hépatiques à l'aide de l'ASGR-1 et/ou de l'ASGR-2 exprimés en surface, et dans lequel l'expression de l'ASGR-1 et/ou de l'ASGR-2 est réduite dans les cellules hépatiques en raison de l'effet de l'ARN interférent ciblé.

6. ARN interférent destiné à être utilisé selon l'une quelconque des revendications 1 à 5, dans lequel l'ARN interférent est administré en combinaison avec un ou plusieurs autres agents thérapeutiques utiles pour traiter ou prévenir les maladies cardiovasculaires, dans lequel l'ARN interférent est administré simultanément, avant, en même temps ou après l'administration d'un ou de plusieurs autres agents thérapeutiques,
éventuellement, dans lequel le ou les autres agents thérapeutiques sont une statine, un anticorps anti-PCSK9 ou une combinaison de ceux-ci.

7. ARN interférent destiné à être utilisé selon la revendication 1, qui est un siARN ciblant ASGR-1, dans lequel le siARN comprend :

un brin sens ; et
un brin antisens,
dans lequel le brin antisens comprend une région ayant une séquence qui est substantiellement ou entièrement complémentaire d'une séquence d'ARNm ASGR-1, et
dans lequel le brin sens comprend une séquence suffisamment complémentaire de la séquence du brin antisens pour que les deux brins s'hybrident dans des conditions physiologiques afin de former une région duplex d'une longueur allant d'environ 15 à environ 30 paires de bases.

8. ARN interférent destiné à être utilisé selon l'une quelconque des revendications 1 à 6, dans lequel l'ARN interférent est une molécule d'ARN à double brin comprenant :

un brin sens ; et
un brin antisens d'une longueur allant de 15 à 30 nucléotides et comprenant une région dont la séquence est entièrement complémentaire d'une séquence d'ARNm ASGR-1 et/ou d'une séquence d'ARNm ASGR-2.

9. ARN interférent destiné à être utilisé selon l'une quelconque des revendications 1 à 6, dans lequel l'ARN interférent est un siARN formé de deux brins séparés d'un brin sens et d'un brin antisens qui s'hybrident pour former une région duplex mais qui ne sont pas connectés par ailleurs, dans lequel

le siARN comprend une région duplex d'une longueur allant d'environ 19 à environ 25 paires de bases ; ou
le siARN comprend :

un brin sens d'environ 15 à 30 nucléotides ; et
un brin antisens d'environ 15 à 30 nucléotides ; ou

le siARN comprend :

un brin sens d'environ 19 à 25 nucléotides et
un brin antisens d'environ 19 à 25 nucléotides.

10. ARN interférent destiné à être utilisé selon l'une quelconque des revendications 1 à 6, 8 et 9, dans lequel ledit ARN interférent est un inhibiteur de l'ASGR-1.

11. ARN interférent destiné à être utilisé selon l'une quelconque des revendications 1 à 6, 8 et 9, dans lequel ledit ARN interférent est un inhibiteur de l'ASGR-2.

12. ARN interférent destiné à être utilisé selon la revendication 1, dans lequel l'ARN interférent est une molécule d'ARN double brin qui comprend un brin sens et un brin antisens, dans lequel le brin antisens comprend une région ayant une séquence qui est substantiellement ou entièrement complémentaire à une séquence d'ARNm ASGR-1 codant pour une protéine ASGR-1 humaine.

**13.** ARN interférent destiné à être utilisé selon la revendication 1, dans lequel l'ARN interférent est une molécule d'ARN double brin qui comprend un brin sens et un brin antisens, dans lequel le brin antisens comprend une région ayant une séquence qui est substantiellement ou entièrement complémentaire à une séquence d'ARNm ASGR-2 codant pour une protéine ASGR-2 humaine.

**14.** siARN destiné à être utilisé selon la revendication 7, dans lequel la maladie cardiovasculaire est une maladie coronarienne ou un infarctus du myocarde.

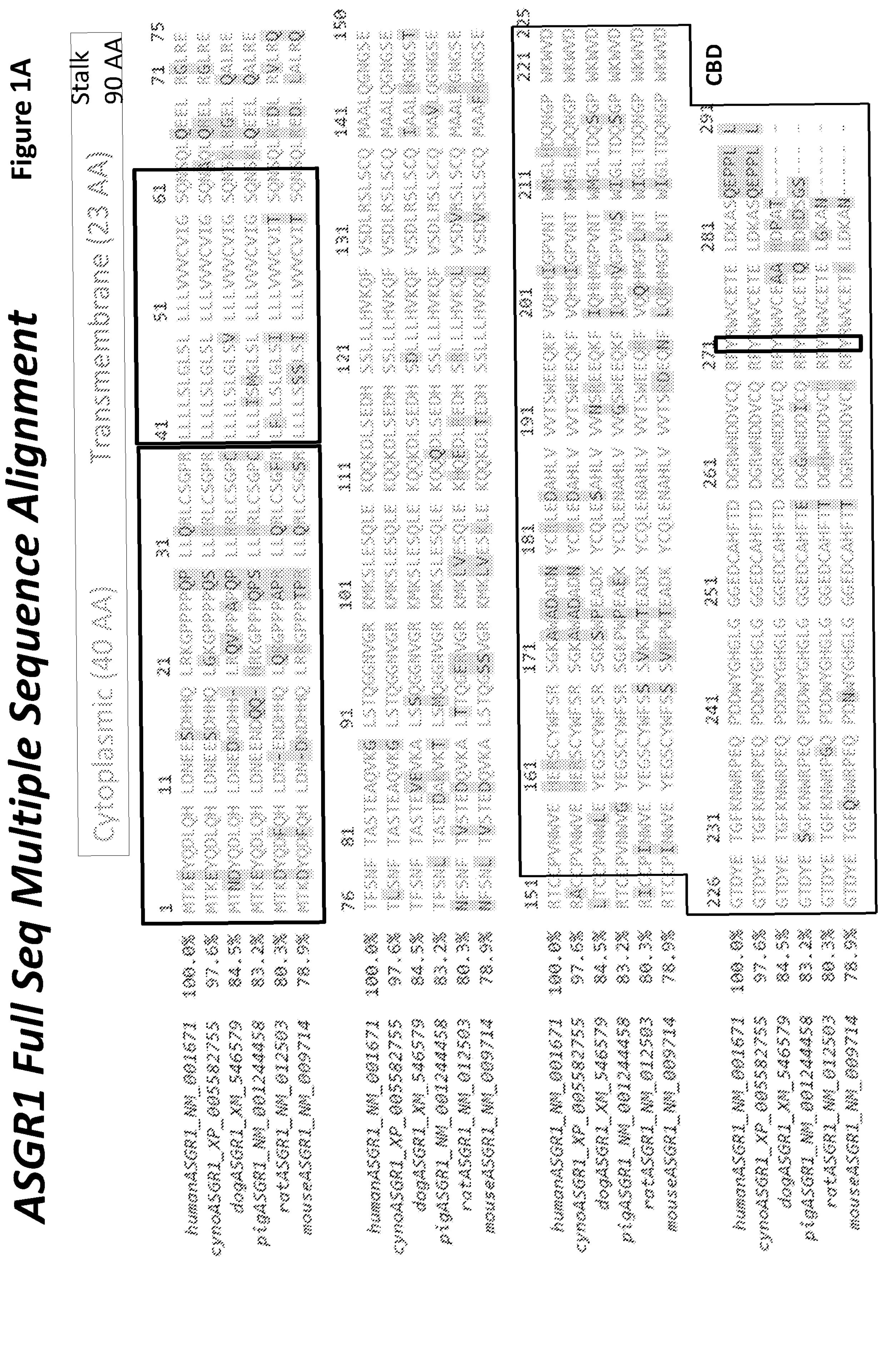
Figure 1A
ASGR1 Full Seq Multiple Sequence Alignment
Cytoplasmic (40 AA)
Transmembrane (23 AA)
Stalk
90 AA
CBD
humanASGR1_NM_001671 100.0%
cynoASGR1_XP_005582755 97.6%
dogASGR1_XM_546579 84.5%
pigASGR1_NM_001244458 83.2%
ratASGR1_NM_012503 80.3%
mouseASGR1_NM_009714 78.9%

Figure 1B

# Human ASGR1 Sequence Alignment

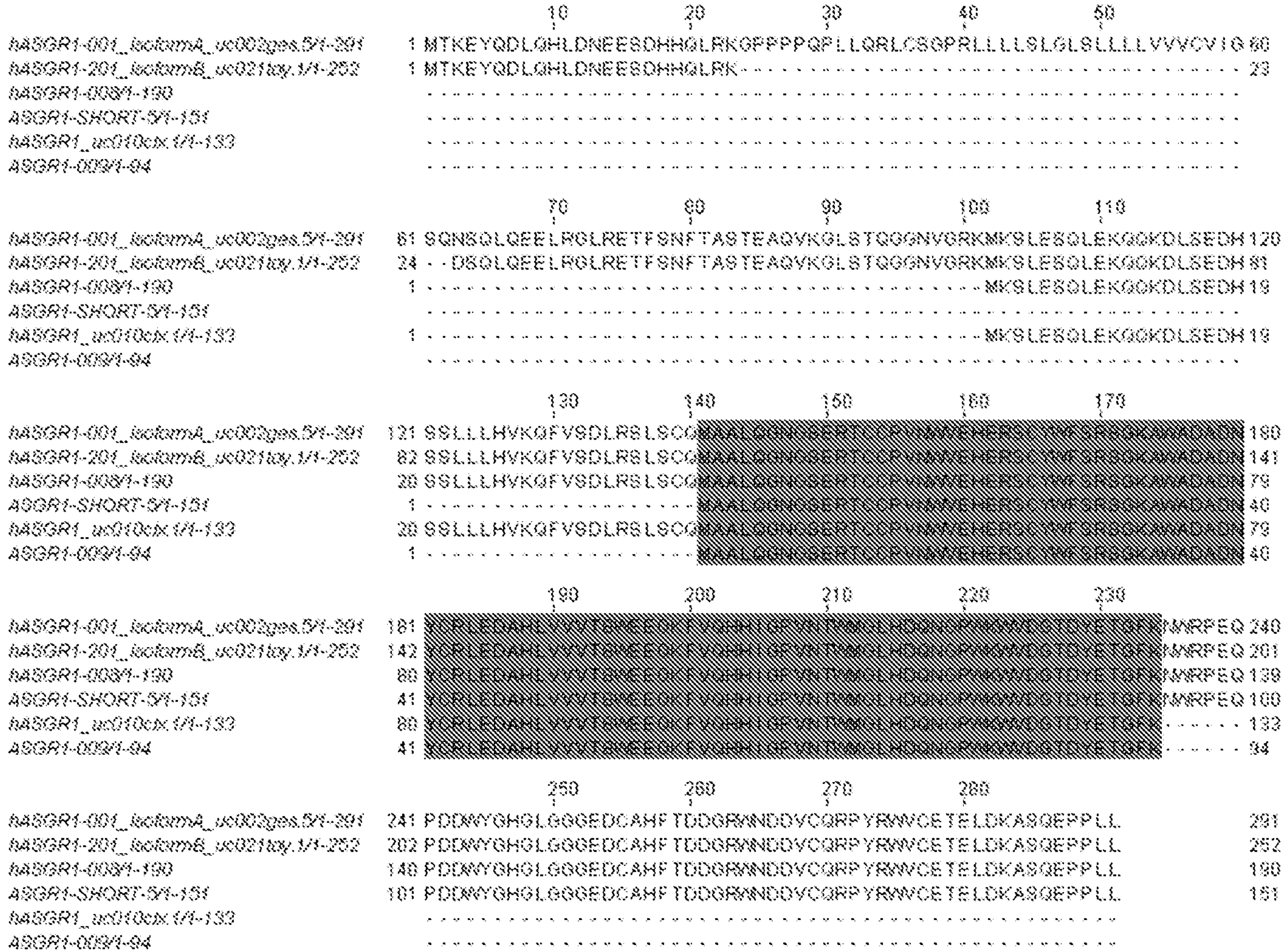

# ASGR2 Full Seq Multiple Sequence Alignment

Figure 2

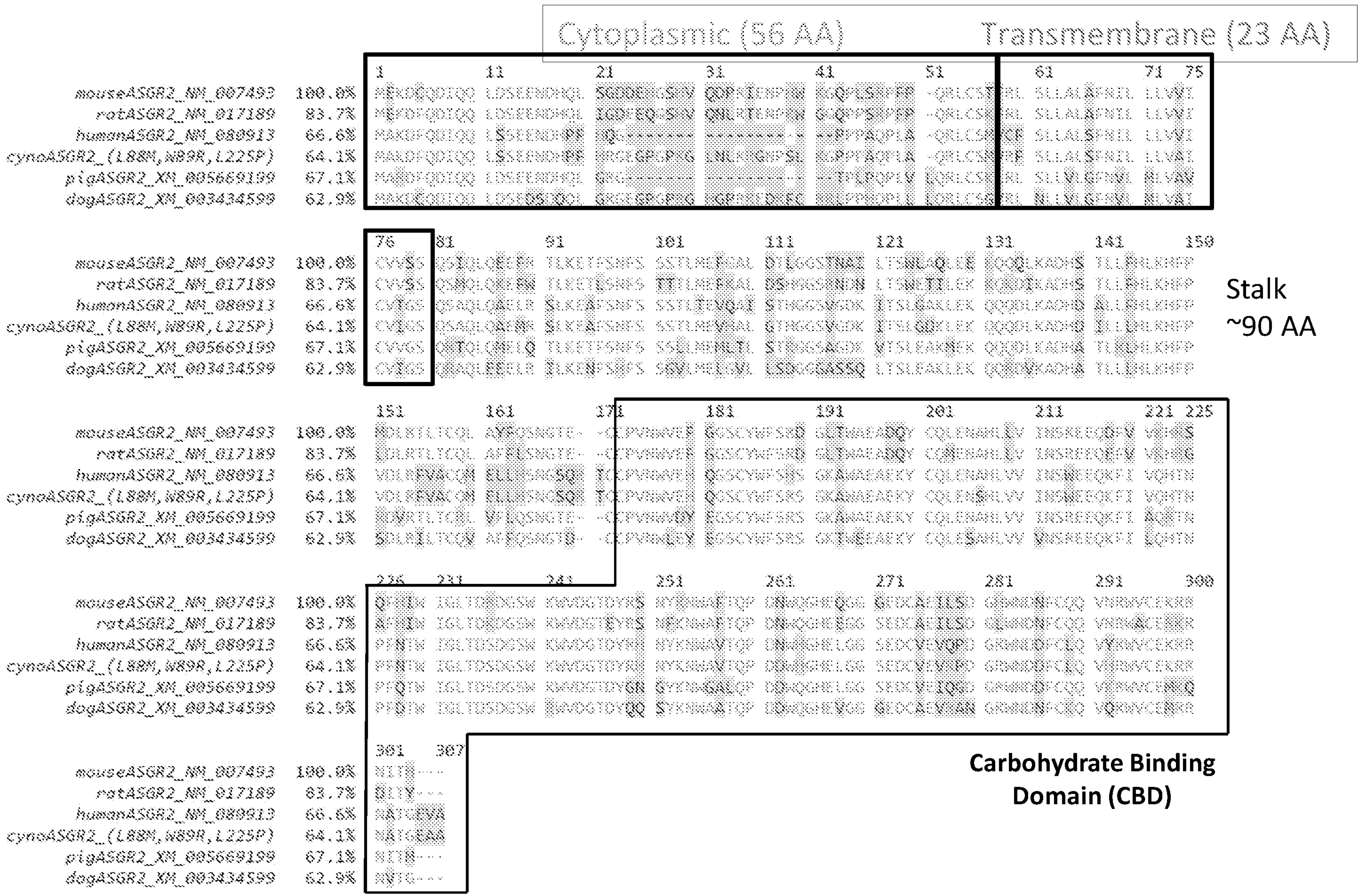

# *Human ASGR1 vs Human ASGR2v2 Alignment*

Figure 3

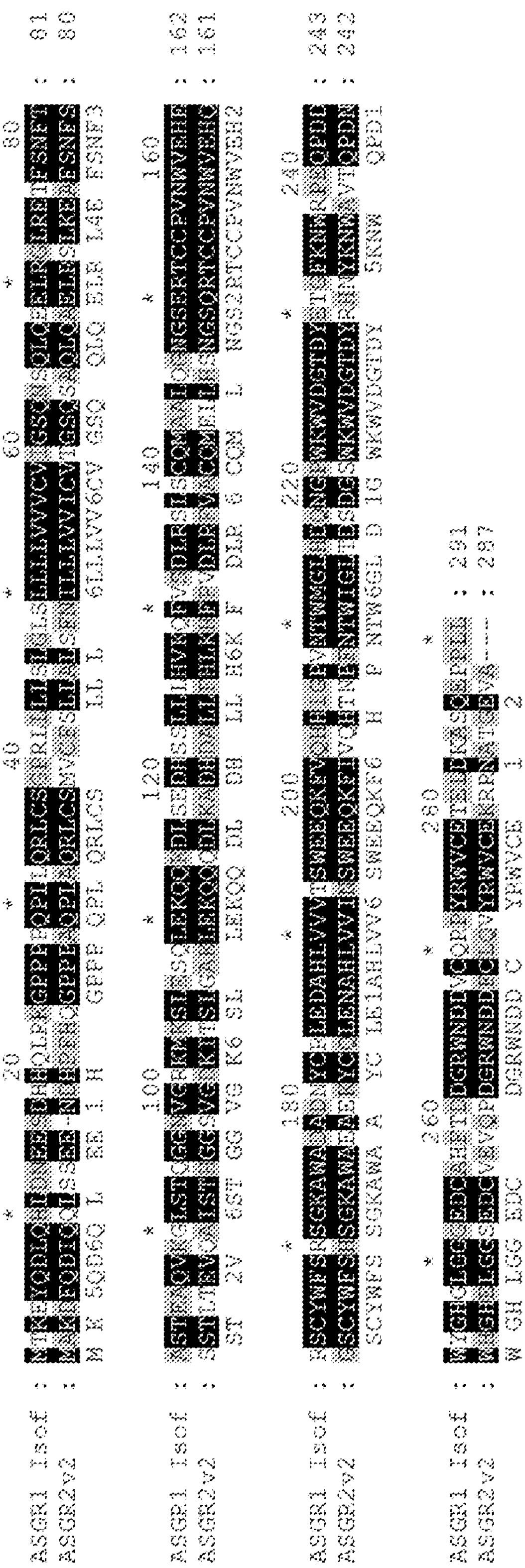

Figure 4

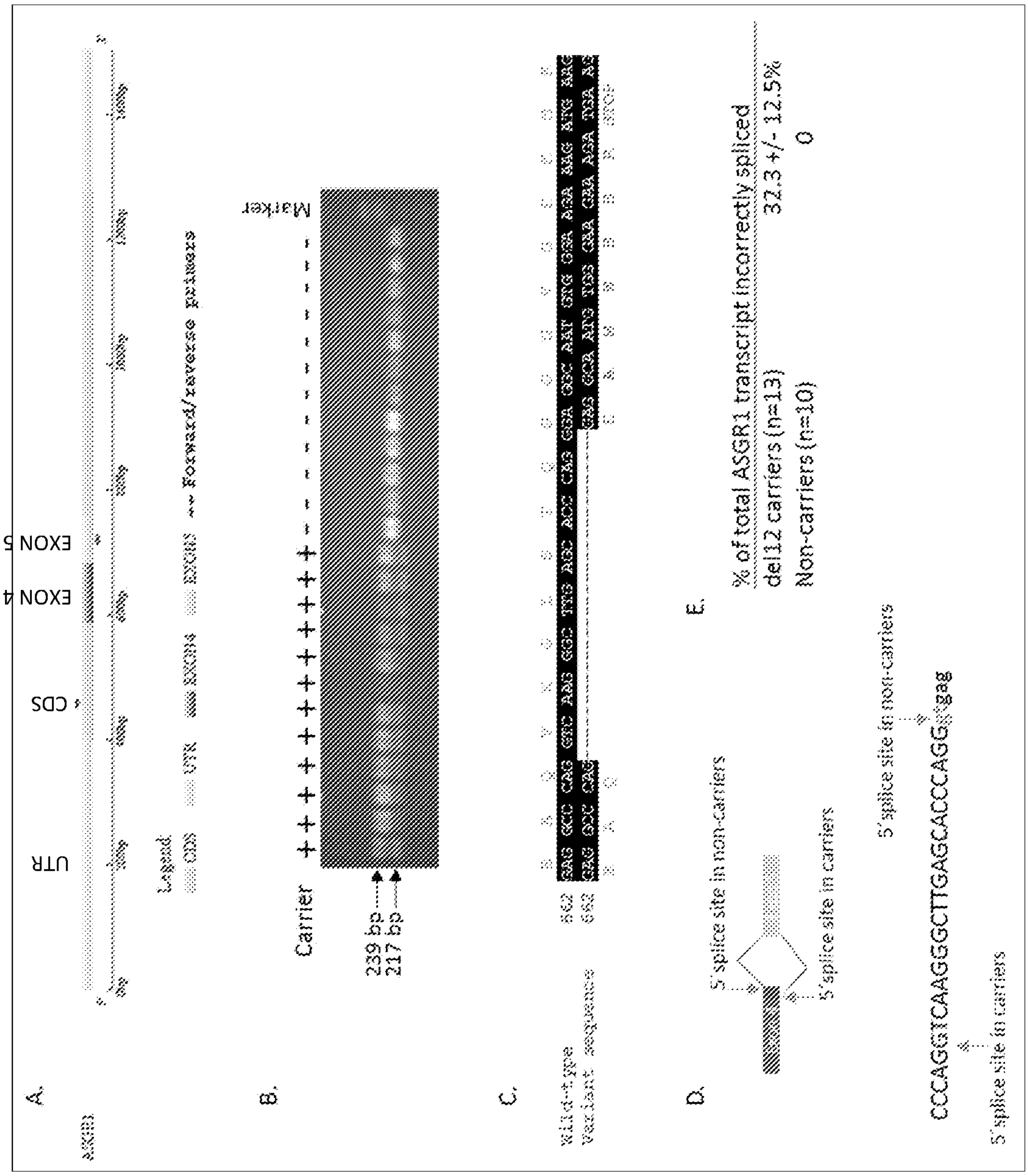
A.
UTR
CDS
EXON 4
EXON 5
Legend
Forward/reverse primers
B.
Carrier
Marker
239 bp
217 bp
C.
Wild-type
Variant sequence
D.
5' splice site in non-carriers
5' splice site in carriers
CCCAGGTCAAGGGCTTGAGCACCCAGGgag
E.
% of total ASGR1 transcript incorrectly spliced
del12 carriers (n=13) 32.3 +/- 12.5%
Non-carriers (n=10) 0

# Figure 5

A.

| Study | NA/NC | Frq (%) | OR (95% CI) | P value |
|---|---|---|---|---|
| Iceland | 33090/236254 | 0.41 | 0.64 (0.51–0.80) | $5.8\times10^{-5}$ |
| Denmark | 2258/7522 | 0.27 | 0.81 (0.41–1.61) | 0.55 |
| Emory | 1804/1823 | 0.74 | 0.67 (0.37–1.22) | 0.19 |
| Duke | 1268/712 | 0.35 | 0.34 (0.08–1.38) | 0.13 |
| UPenn | 728/488 | 0.31 | 0.67 (0.14–3.26) | 0.62 |
| UK–I | 653/664 | 0.83 | 0.55 (0.21–1.46) | 0.23 |
| UK–II | 2175/1494 | 0.77 | 0.78 (0.44–1.37) | 0.38 |
| New Zealand | 548/457 | 0.44 | 0.42 (0.08–2.18) | 0.30 |
| All | | | 0.66 (0.55–0.79) | $4.0\times10^{-6}$ |

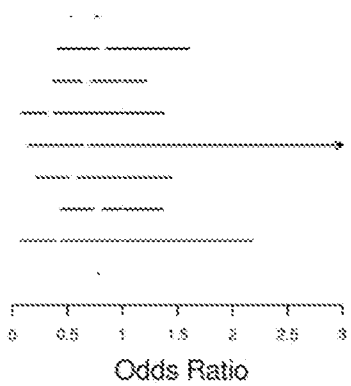


B.

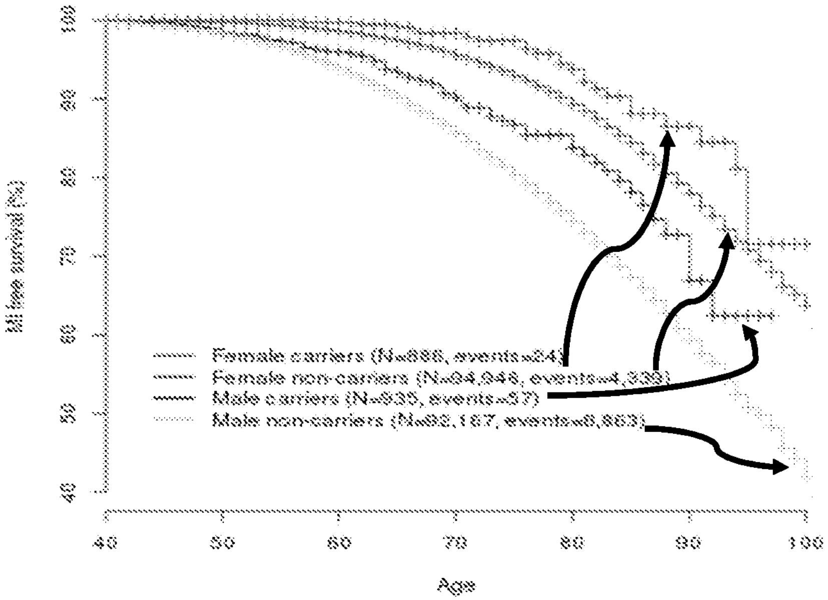

Figure 6

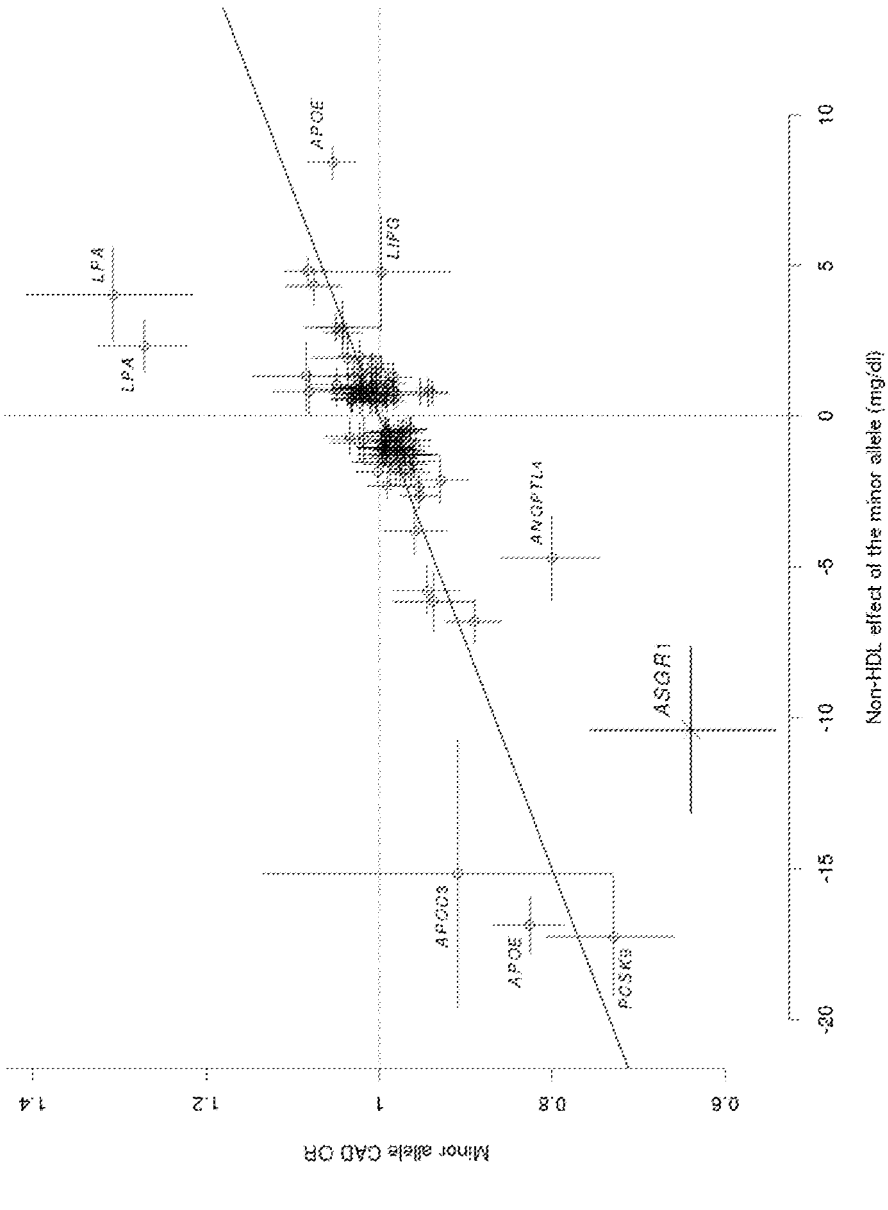
Minor allele CAD OR
1.4
1.2
1
0.8
0.6
Non-HDL effect of the minor allele (mg/dl)
-20
-15
-10
-5
0
5
10
LPA
LPA
APOE
LIPG
ANGPTL4
ASGR1
APOC3
APOE
PCSK9

Figure 7

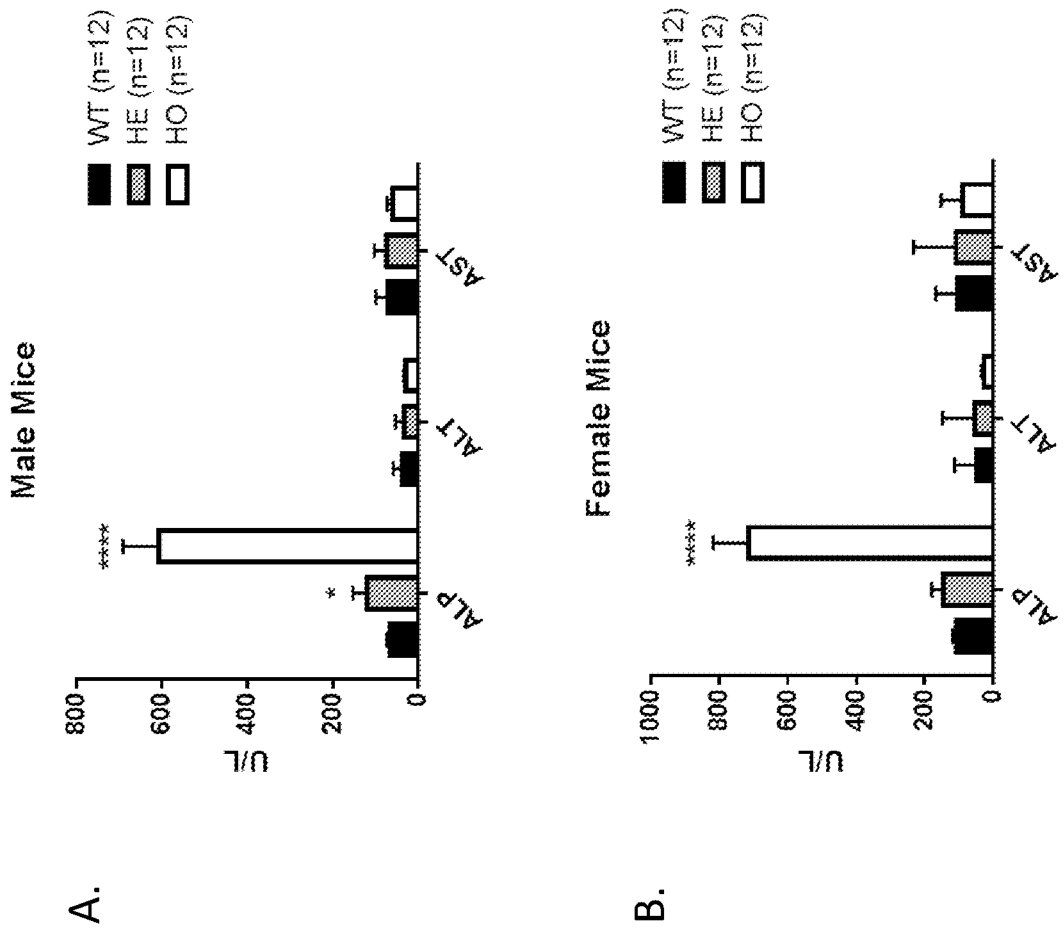
A.
Male Mice
U/L
800
600
400
200
0
ALP
ALT
AST
*
****
WT (n=12)
HE (n=12)
HO (n=12)
B.
Female Mice
U/L
1000
800
600
400
200
0
ALP
ALT
AST
****
WT (n=12)
HE (n=12)
HO (n=12)

**Figure 8**

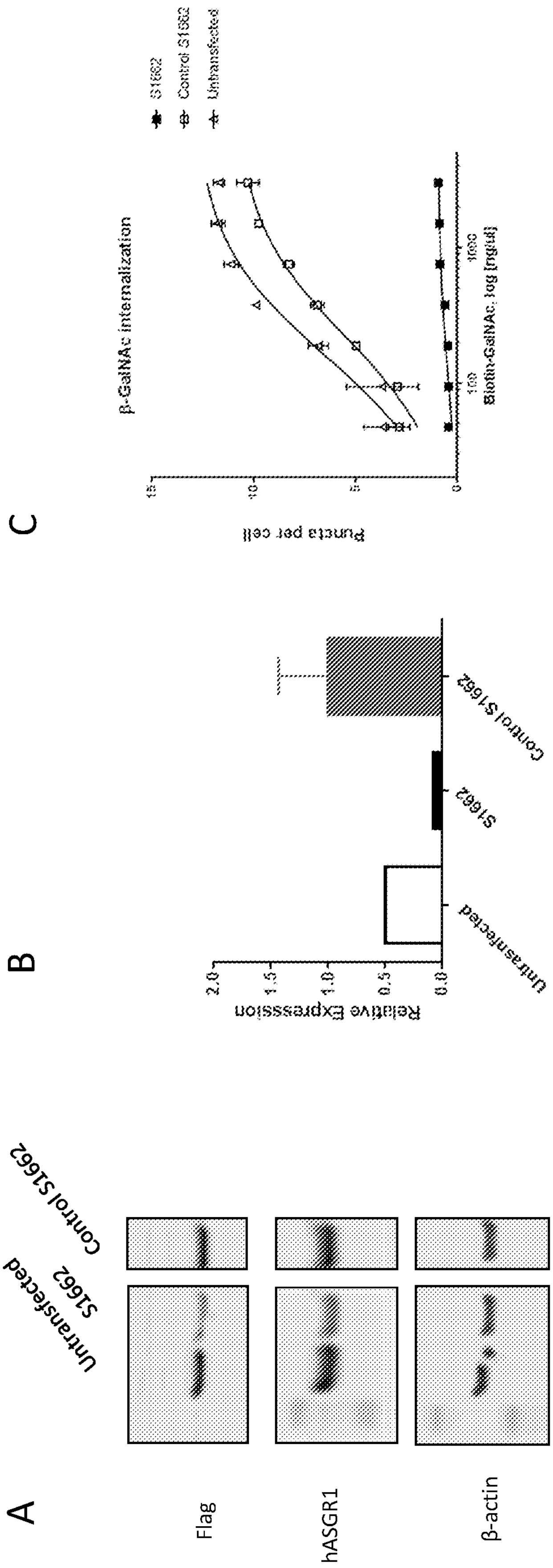
A
Flag
hASGR1
β-actin
Untransfected
S1662
Control S1662
B
Relative Expression
2.0
1.5
1.0
0.5
0.0
Untransfected
S1662
Control S1662
C
β-GalNAc internalization
Puncta per cell
15
10
5
0
100
1000
S1662
Control S1662
Untransfected

**Figure 9**

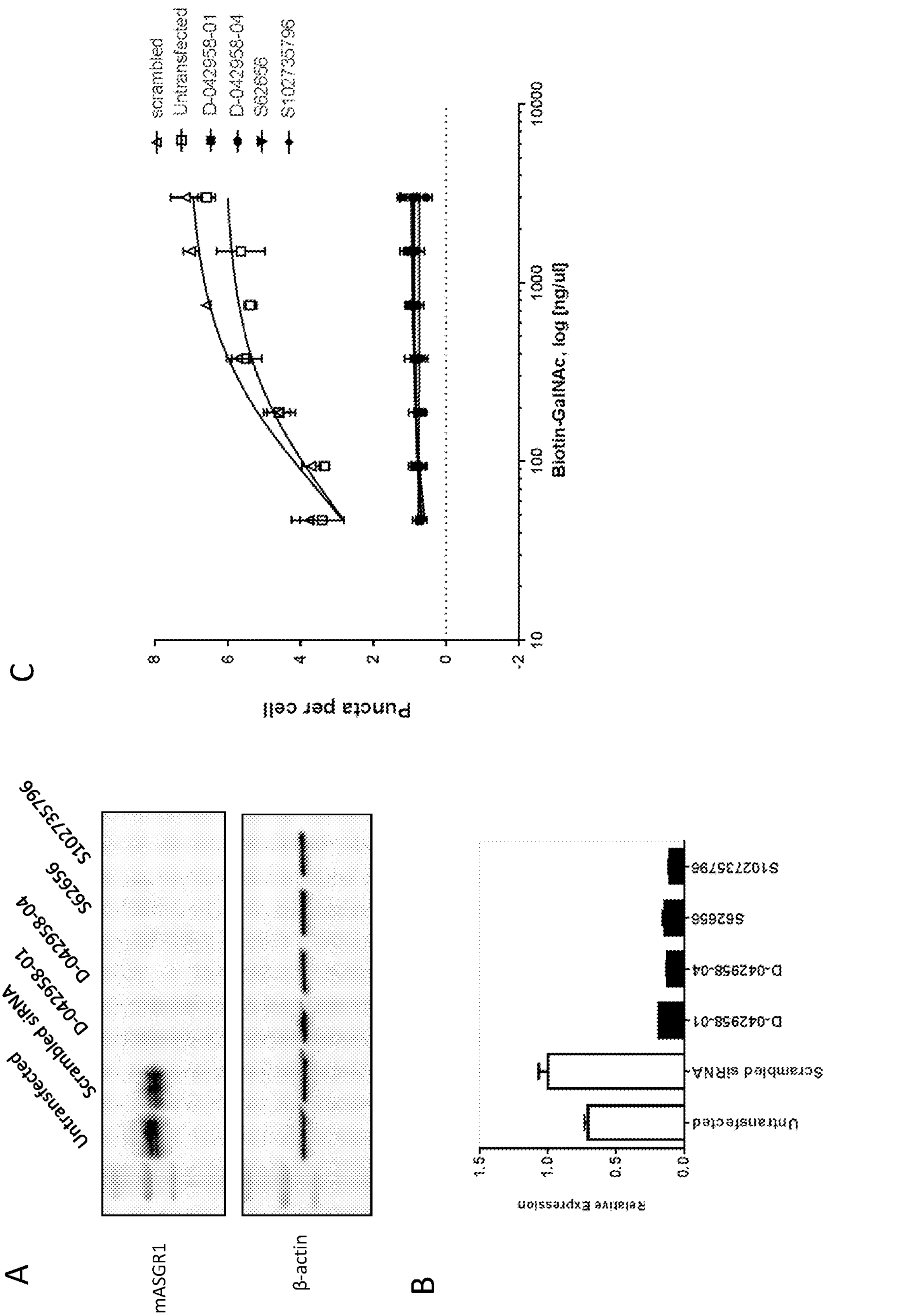
A
mASGR1
β-actin
Untransfected
Scrambled siRNA
D-042958-01
D-042958-04
S62656
S102735796
B
Relative Expression
1.5
1.0
0.5
0.0
Untransfected
Scrambled siRNA
D-042958-01
D-042958-04
S62656
S102735796
C
Puncta per cell
8
6
4
2
0
-2
10
100
1000
10000
Biotin-GalNAc, log [ng/ul]
scrambled
Untransfected
D-042958-01
D-042958-04
S62656
S102735796

Figure 10

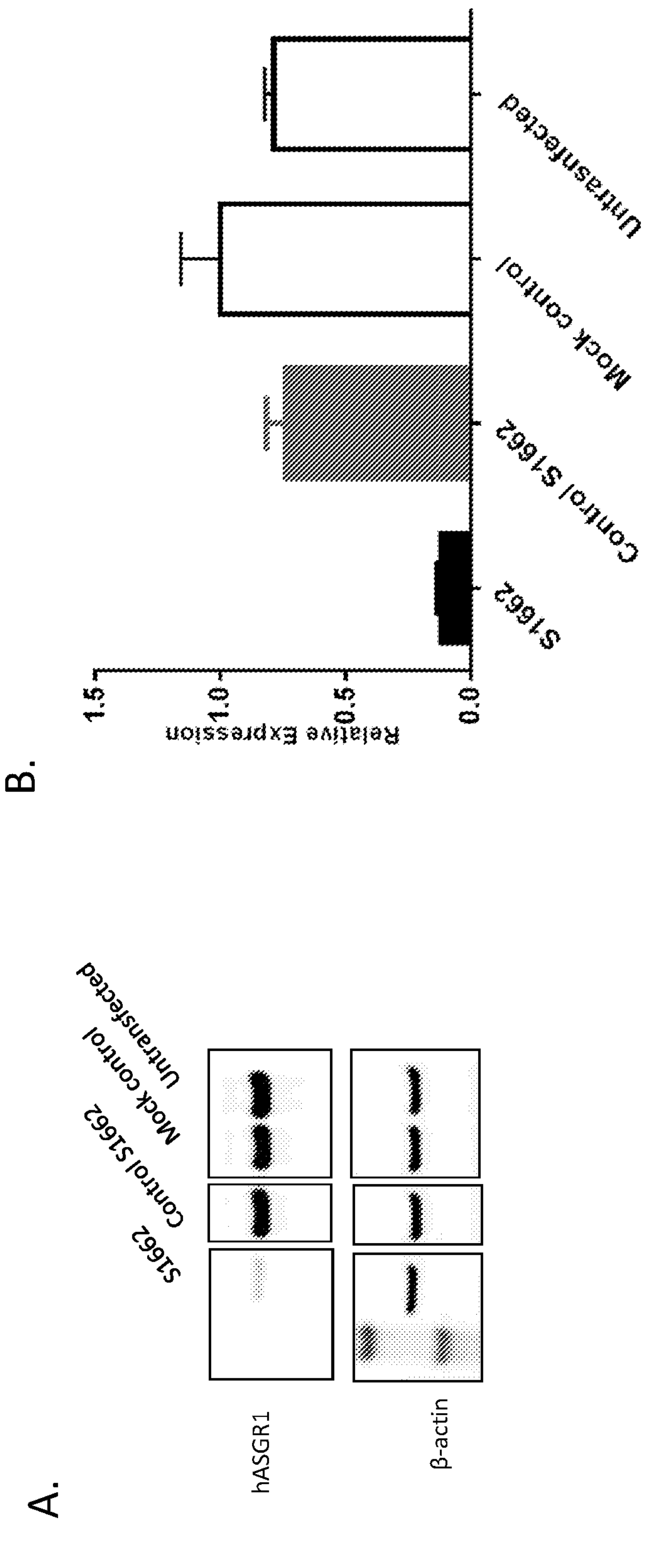
A.
hASGR1
β-actin
S1662
Control S1662
Mock control
Untransfected
B.
Relative Expression
0.0
0.5
1.0
1.5
S1662
Control S1662
Mock control
Untransfected

Figure 11

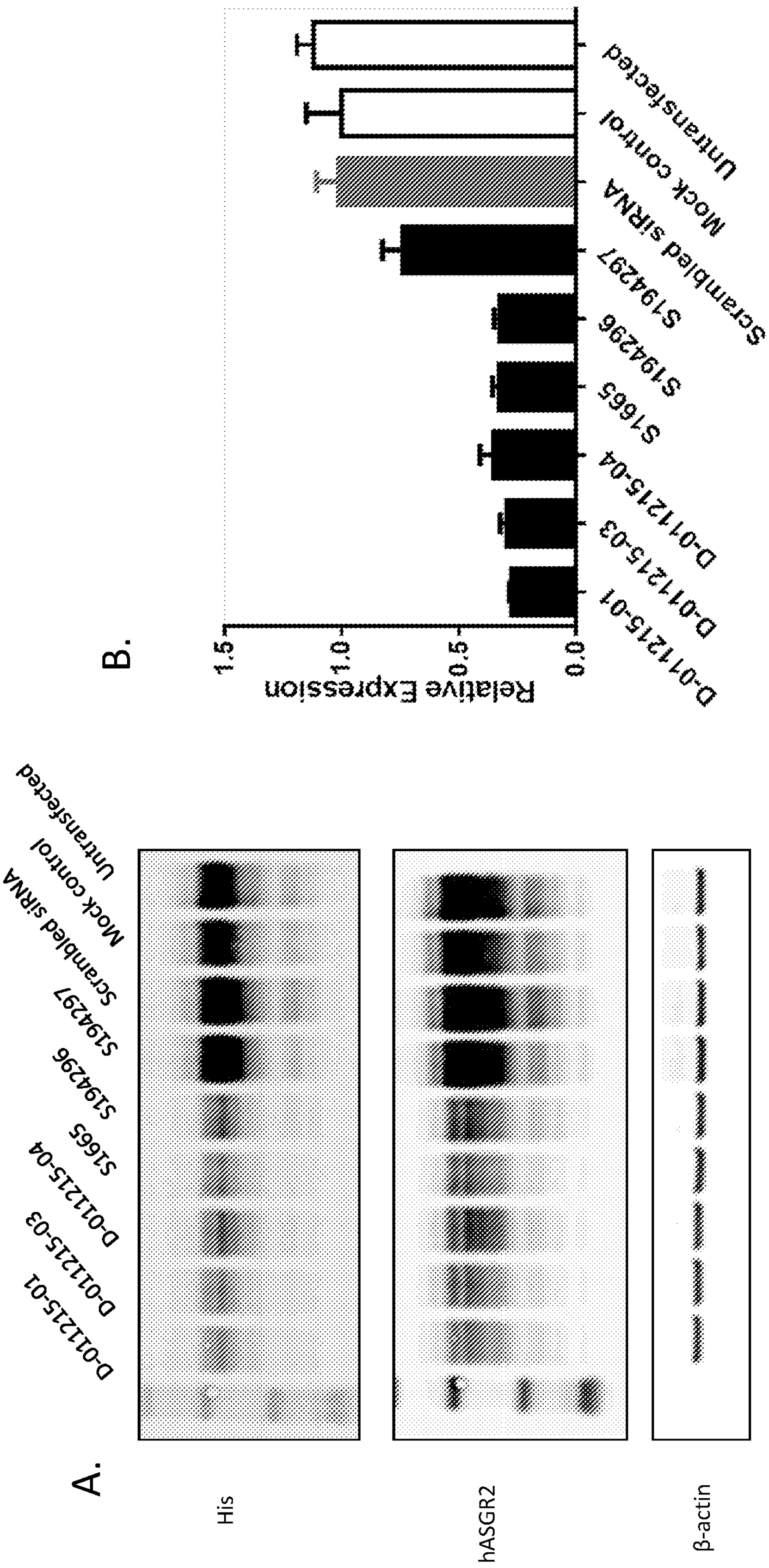
A.
His
hASGR2
β-actin
D-011215-01
D-011215-03
D-011215-04
S1665
S194296
S194297
Scrambled siRNA
Mock control
Untransfected
B.
Relative Expression
0.0
0.5
1.0
1.5
D-011215-01
D-011215-03
D-011215-04
S1665
S194296
S194297
Scrambled siRNA
Mock control
Untransfected

Figure 12

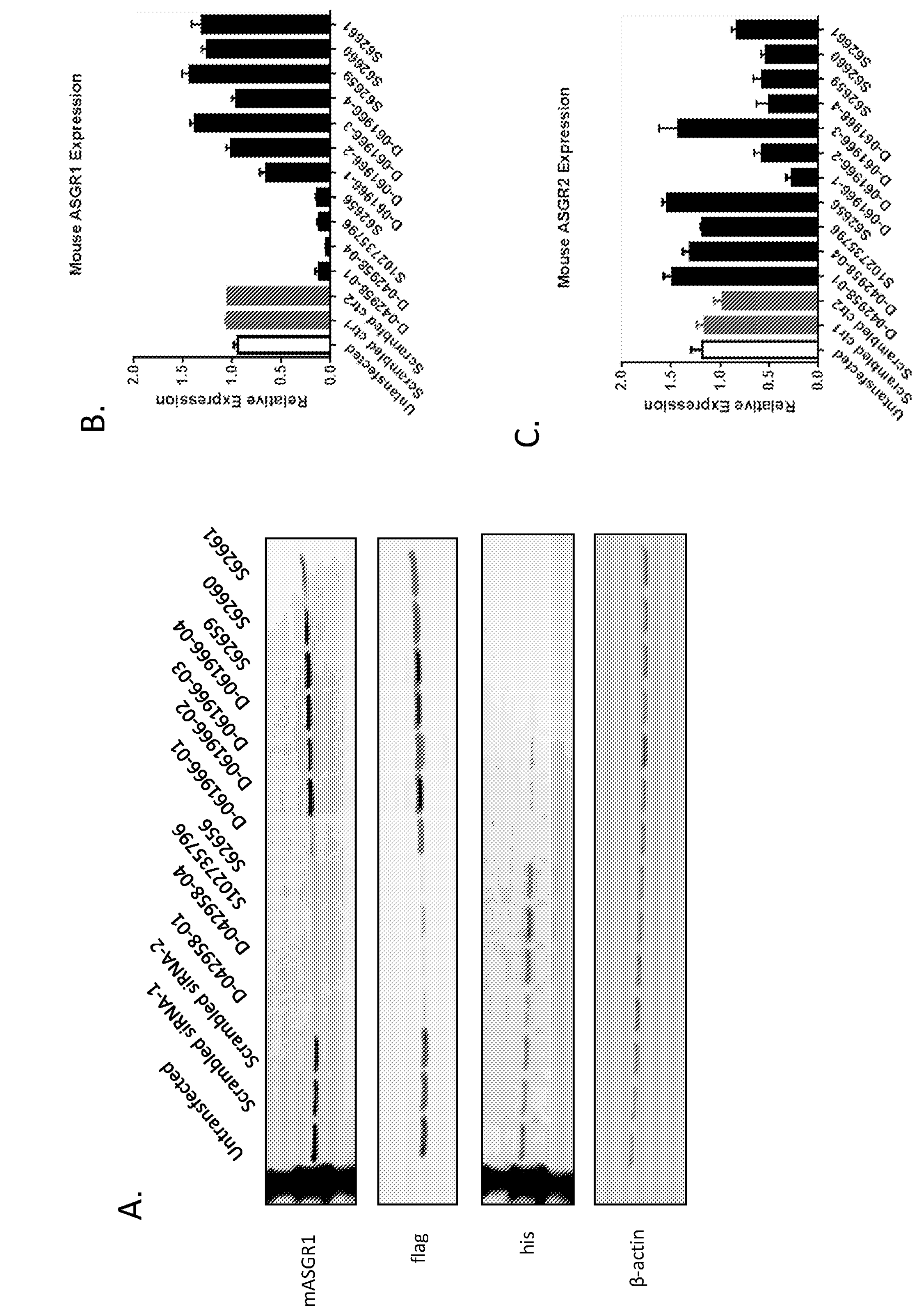
A.
Untransfected
Scrambled siRNA-1
Scrambled siRNA-2
D-042958-01
D-042958-04
S102735796
S62656
D-061966-01
D-061966-02
D-061966-03
D-061966-04
S62659
S62660
S62661
mASGR1
flag
his
β-actin
B.
Mouse ASGR1 Expression
Relative Expression
2.0
1.5
1.0
0.5
0.0
Untransfected
Scrambled ctr1
Scrambled ctr2
D-042958-01
D-042958-04
S102735796
S62656
D-061966-1
D-061966-2
D-061966-3
D-061966-4
S62659
S62660
S62661
C.
Mouse ASGR2 Expression
Relative Expression
2.0
1.5
1.0
0.5
0.0
Untransfected
Scrambled ctr1
Scrambled ctr2
D-042958-01
D-042958-04
S102735796
S62656
D-061966-1
D-061966-2
D-061966-3
D-061966-4
S62659
S62660
S62661

Figure 13

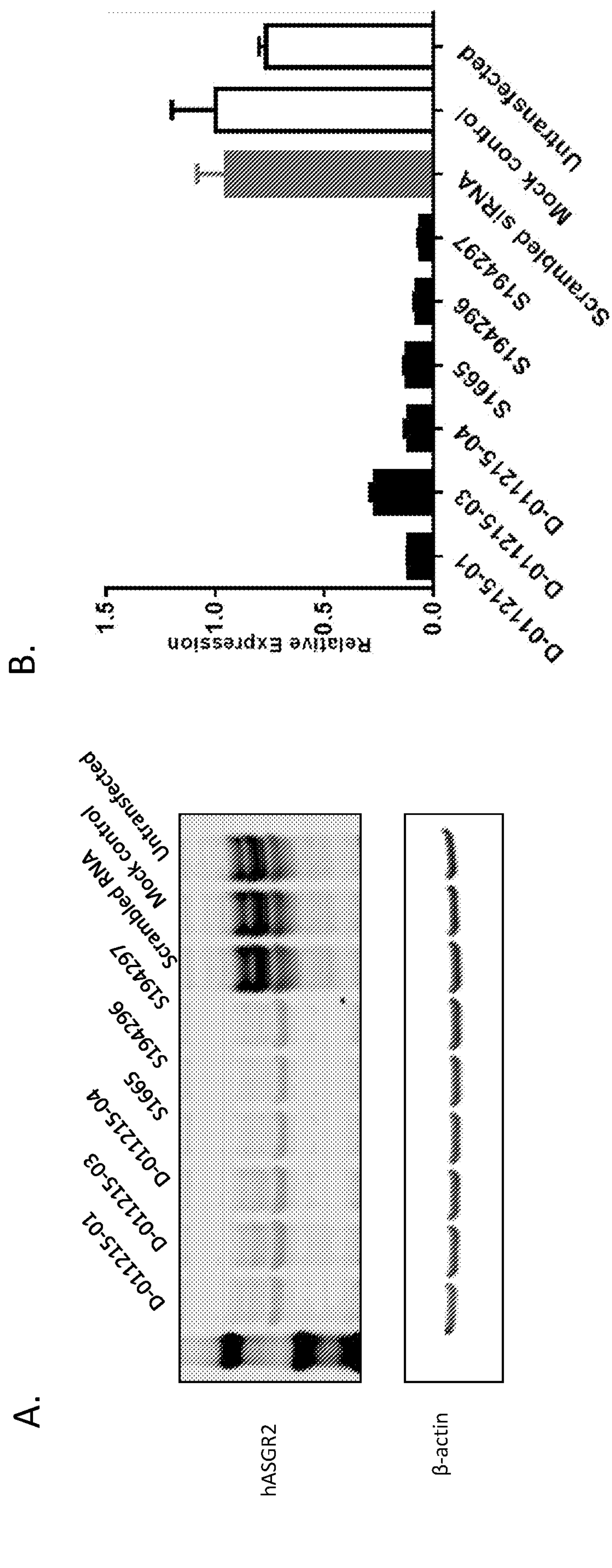
A.
hASGR2
β-actin
D-011215-01
D-011215-03
D-011215-04
S1665
S194296
S194297
Scrambled RNA
Mock control
Untransfected
B.
Relative Expression
0.0
0.5
1.0
1.5
D-011215-01
D-011215-03
D-011215-04
S1665
S194296
S194297
Scrambled siRNA
Mock control
Untransfected

Figure 14

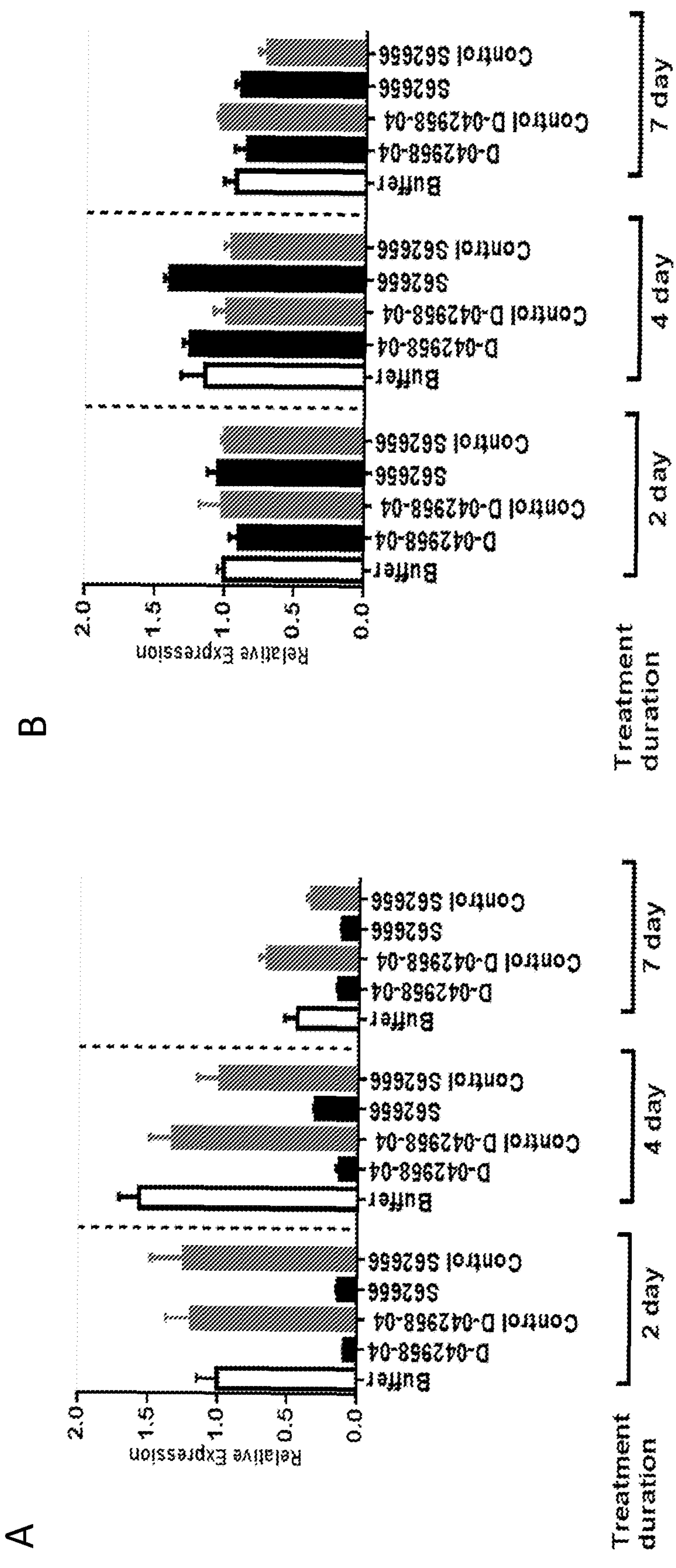
A
B
Relative Expression
0.0
0.5
1.0
1.5
2.0
Buffer
D-042968-04
Control D-042968-04
S62656
Control S62656
Treatment duration
2 day
4 day
7 day

Figure 15

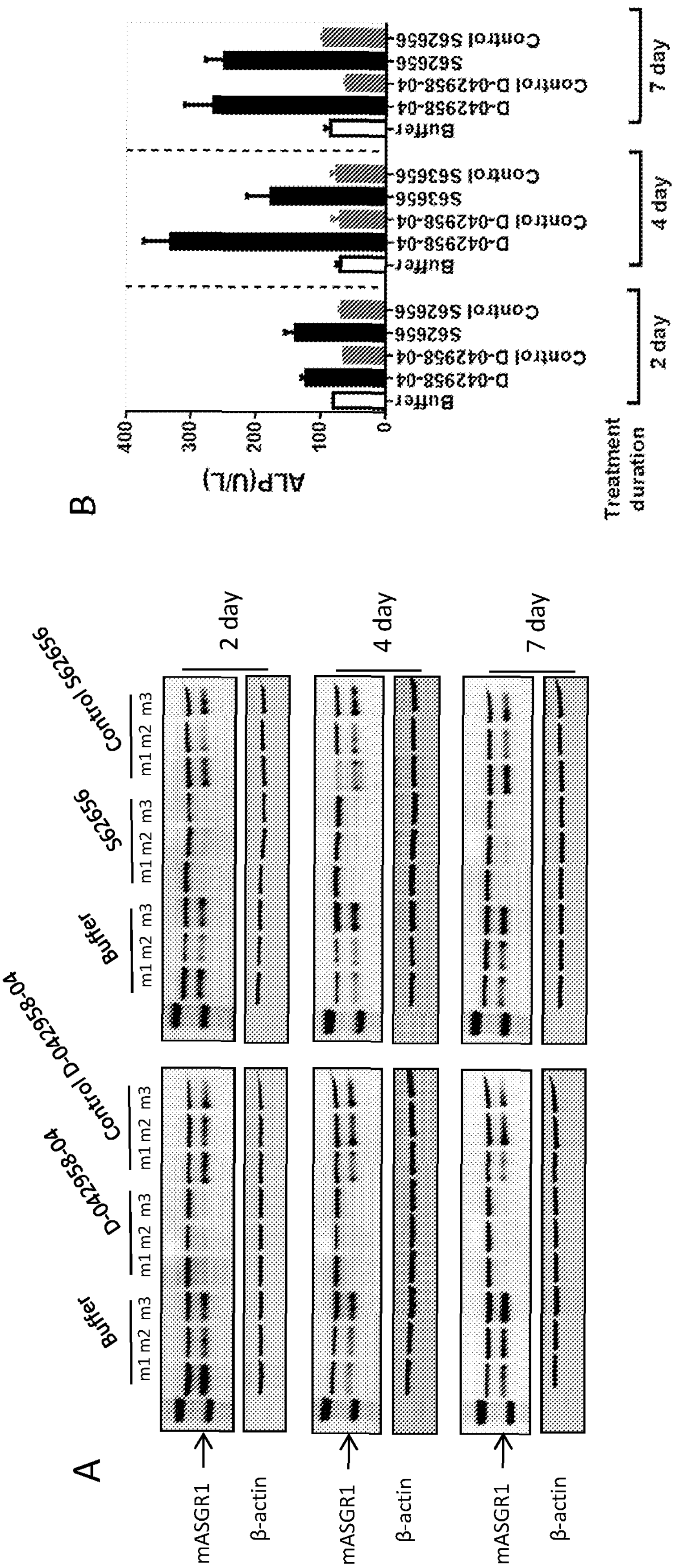
A
mASGR1
β-actin
Buffer
D-042958-04
Control D-042958-04
S62656
Control S62656
m1 m2 m3
2 day
4 day
7 day
B
ALP(U/L)
0
100
200
300
400
Buffer
D-042958-04
Control D-042958-04
S62656
Control S62656
S63656
Control S63656
Treatment duration

# Figure 16

A

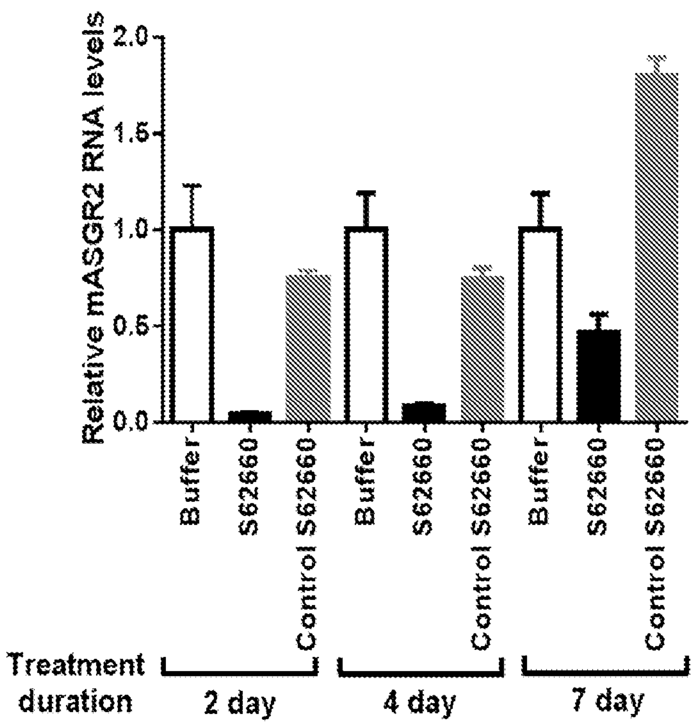
Relative mASGR2 RNA levels
2.0
1.5
1.0
0.5
0.0
Buffer
S62660
Control S62660
Buffer
S62660
Control S62660
Buffer
S62660
Control S62660
Treatment duration
2 day
4 day
7 day

B

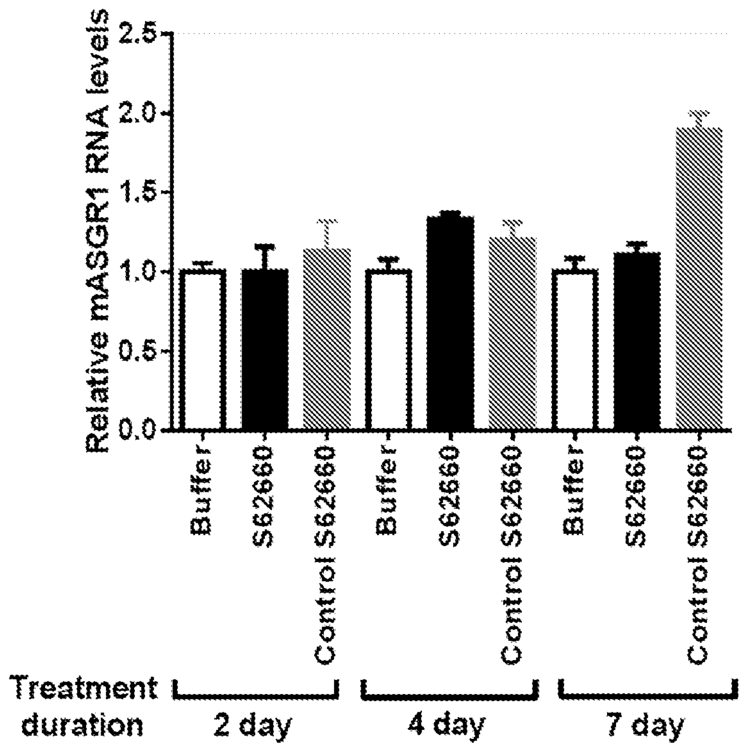
Relative mASGR1 RNA levels
2.5
2.0
1.5
1.0
0.5
0.0
Buffer
S62660
Control S62660
Buffer
S62660
Control S62660
Buffer
S62660
Control S62660
Treatment duration
2 day
4 day
7 day

Figure 17

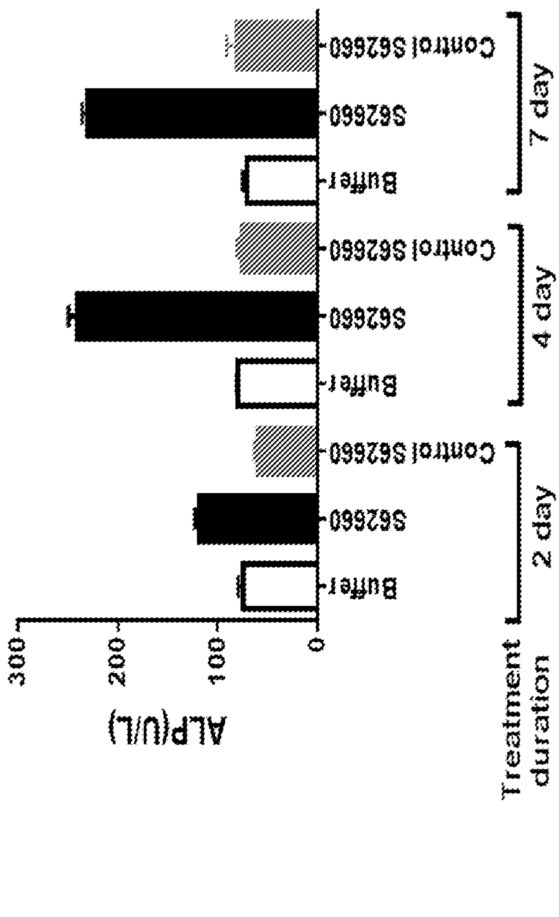
ALP(U/L)
300
200
100
0
Buffer
S62660
Control S62660
Buffer
S62660
Control S62660
Buffer
S62660
Control S62660
Treatment duration
2 day
4 day
7 day

Figure 18

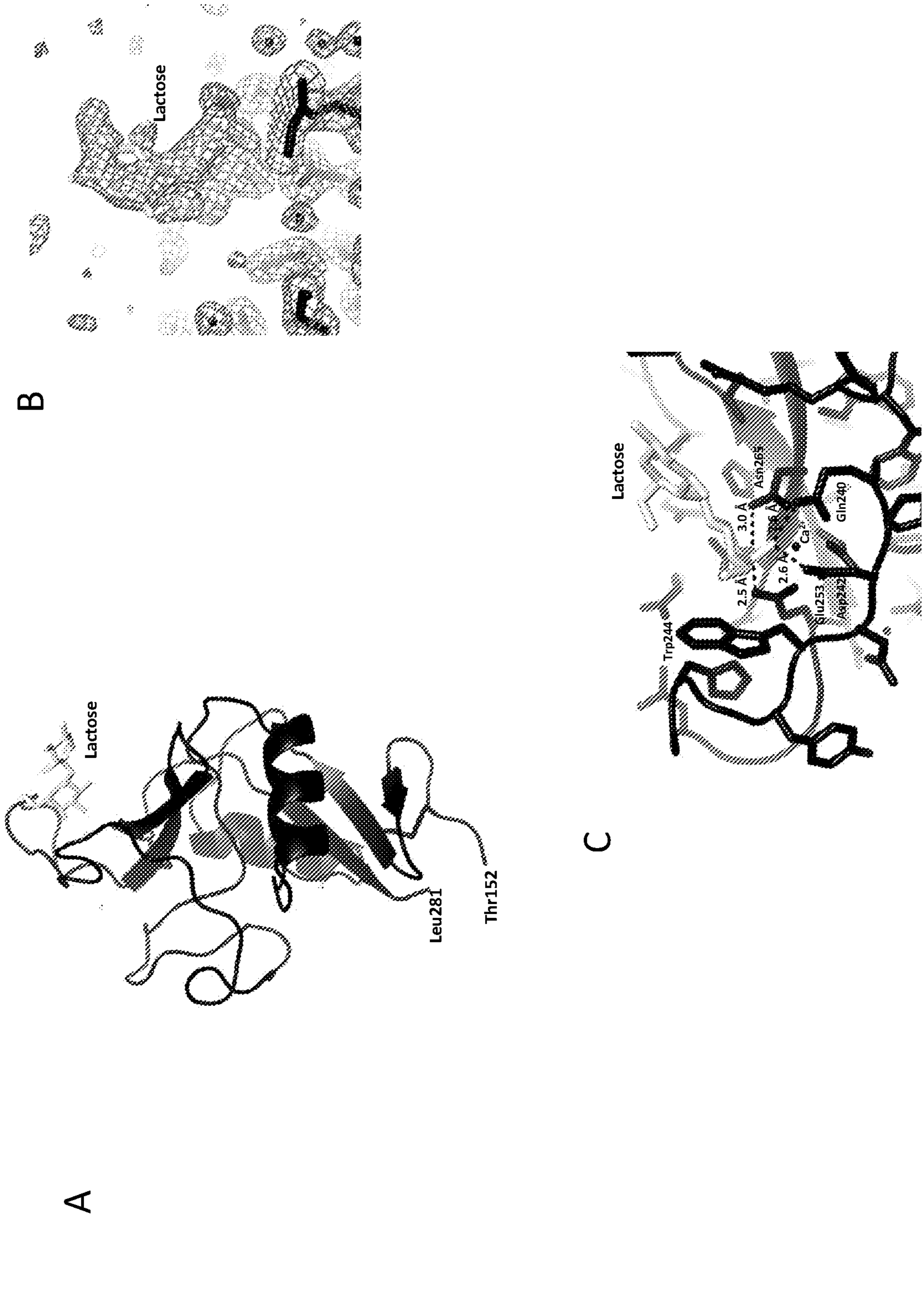
A
Lactose
Leu281
Thr152
B
Lactose
C
Lactose
Trp244
2.5 Å
2.6 Å
3.0 Å
Asn265
Gln240
Glu253
Asp242

Figure 19

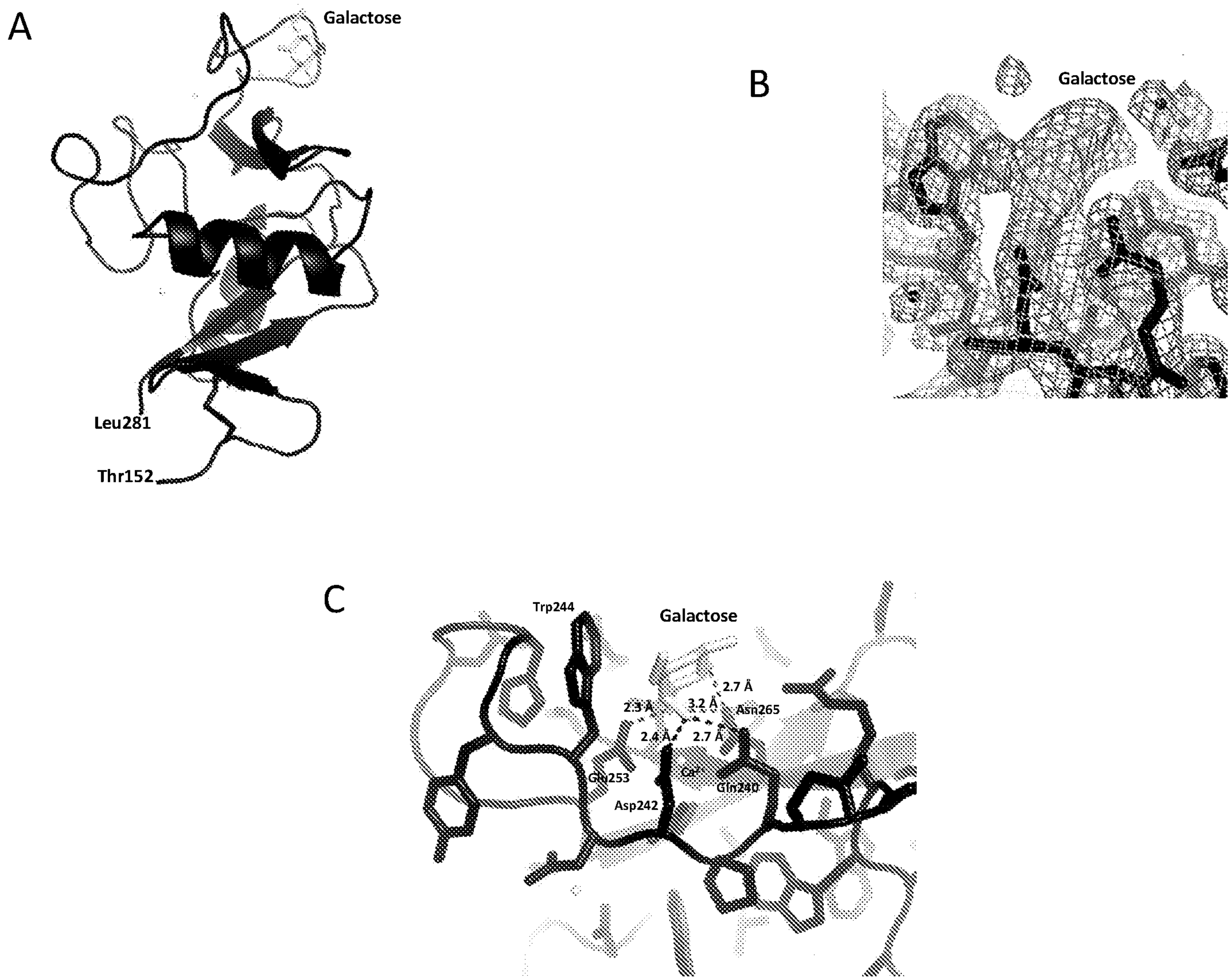
A
Galactose
Leu281
Thr152
B
Galactose
C
Trp244
Galactose
2.7 Å
2.3 Å
3.2 Å
Asn265
2.4 Å
2.7 Å
Glu253
Gln240
Asp242

Figure 20

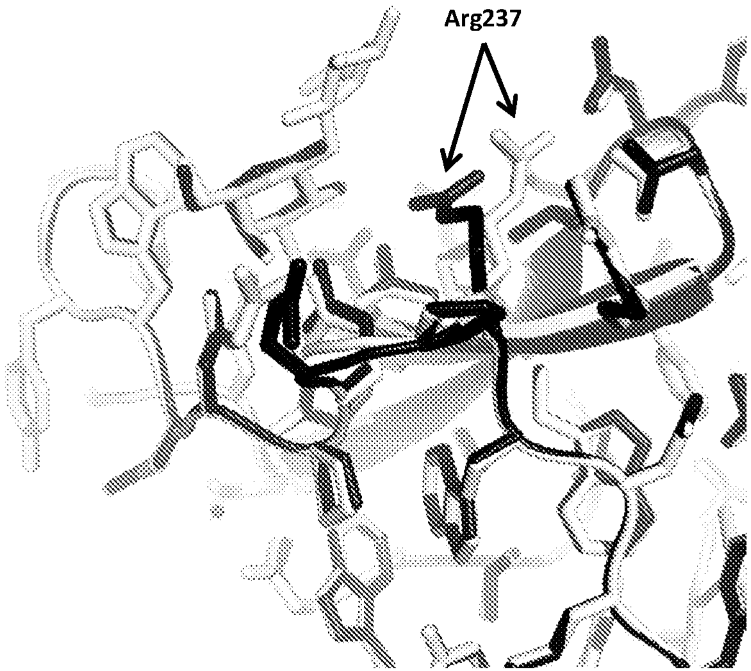

Figure 21

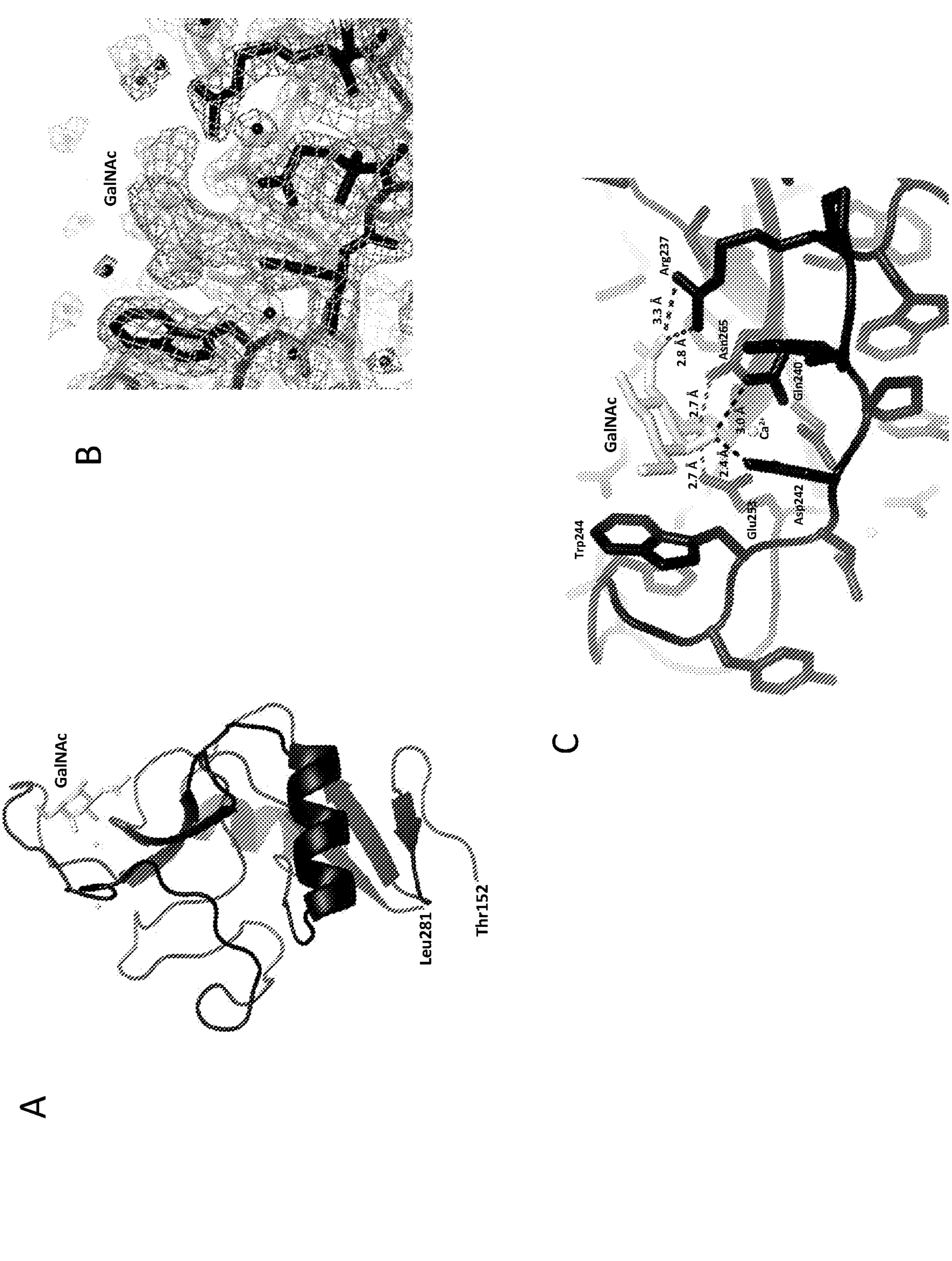
A
GalNAc
Leu281
Thr152
B
GalNAc
C
Trp244
GalNAc
Arg237
3.3 Å
2.8 Å
Asn265
2.7 Å
2.7 Å
2.4 Å
3.0 Å
Ca²⁺
Gln240
Glu252
Asp242

**Figure 22**

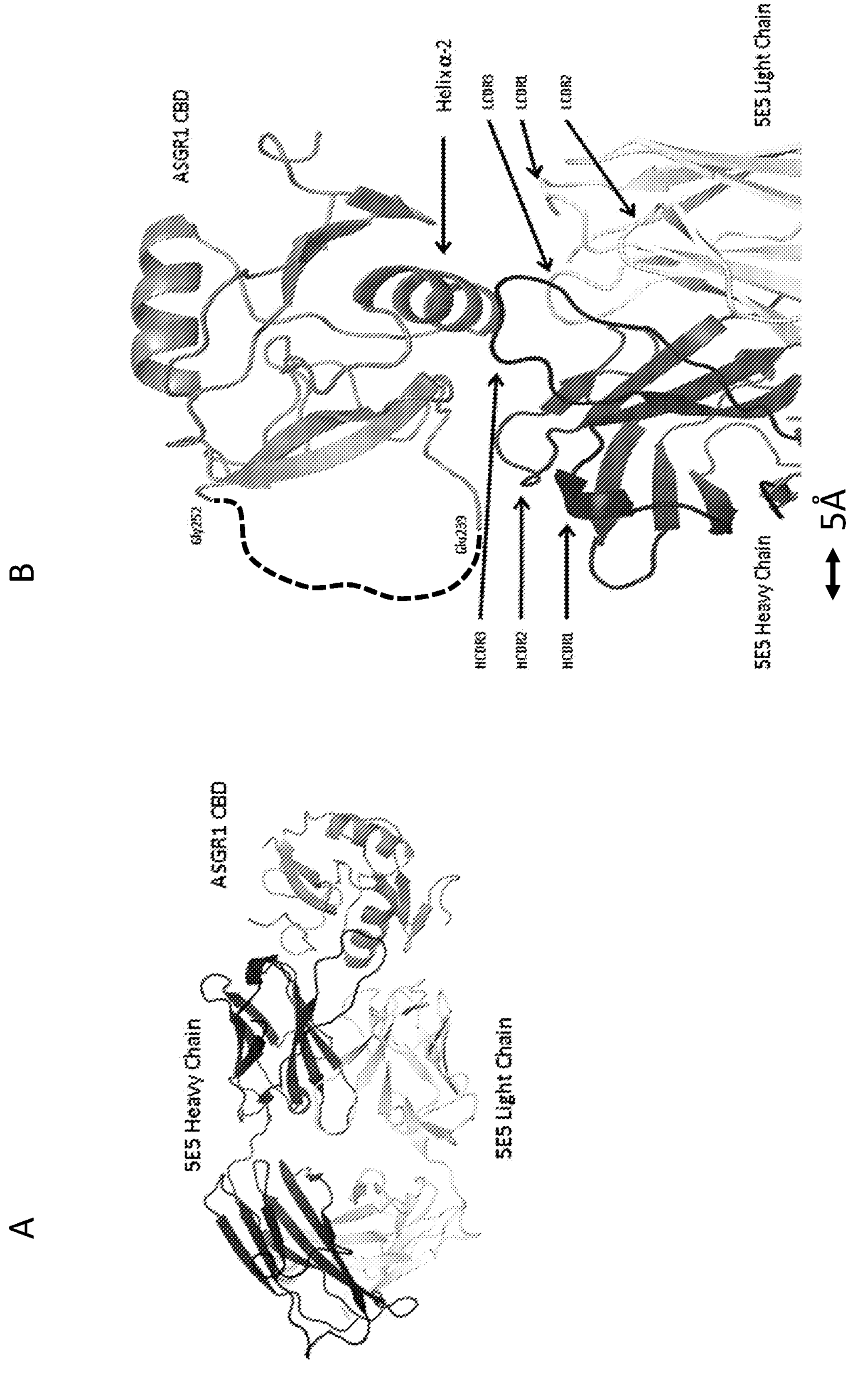
A
5E5 Heavy Chain
ASGR1 CBD
5E5 Light Chain
B
ASGR1 CBD
Helix α-2
LCDR3
LCDR1
LCDR2
5E5 Light Chain
Gly252
Glu239
HCDR3
HCDR2
HCDR1
5E5 Heavy Chain
5Å

# Figure 23

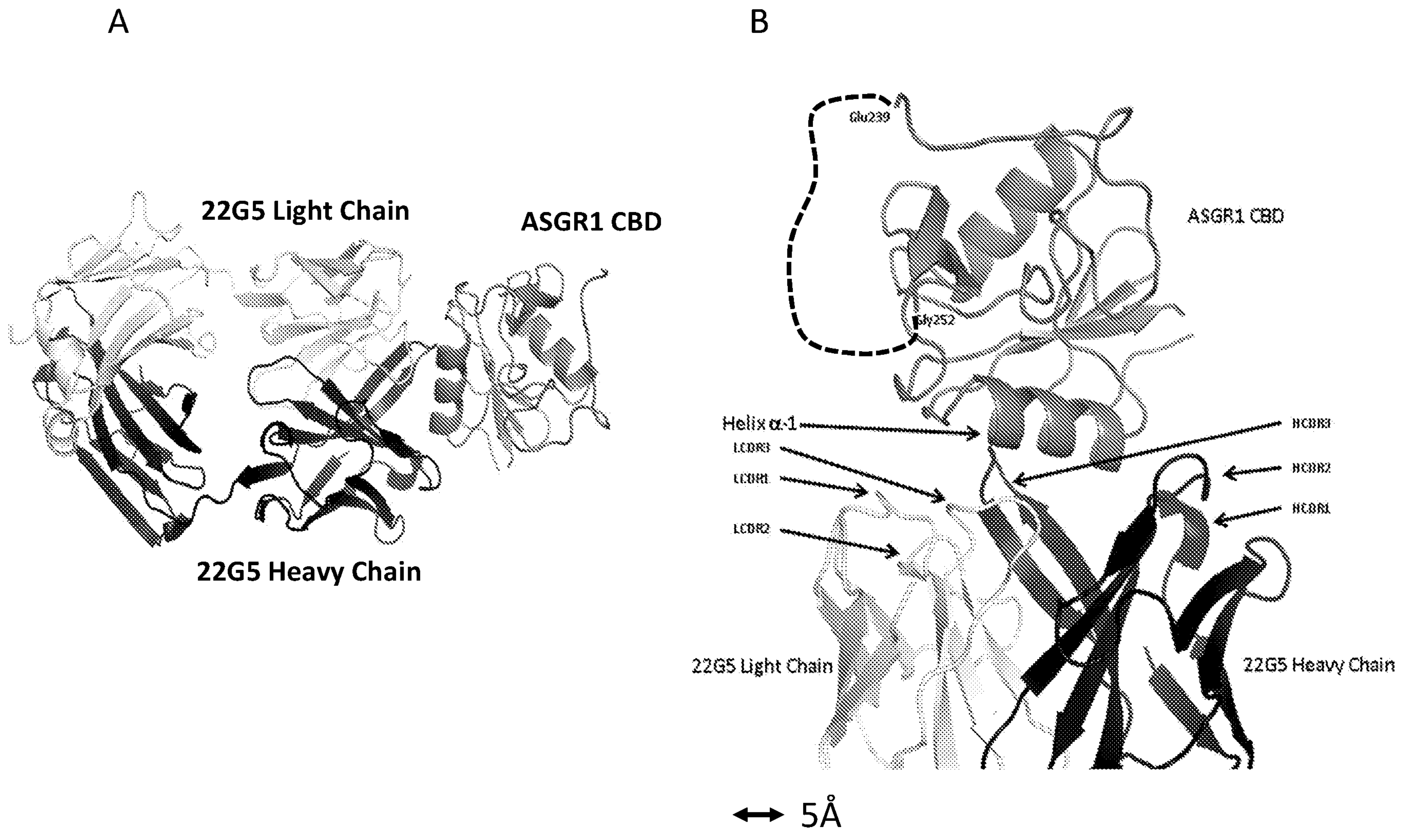
A
22G5 Light Chain
ASGR1 CBD
22G5 Heavy Chain
B
Glu239
Gly252
ASGR1 CBD
Helix α-1
LCDR3
LCDR1
LCDR2
HCDR3
HCDR2
HCDR1
22G5 Light Chain
22G5 Heavy Chain
5Å

Figure 24

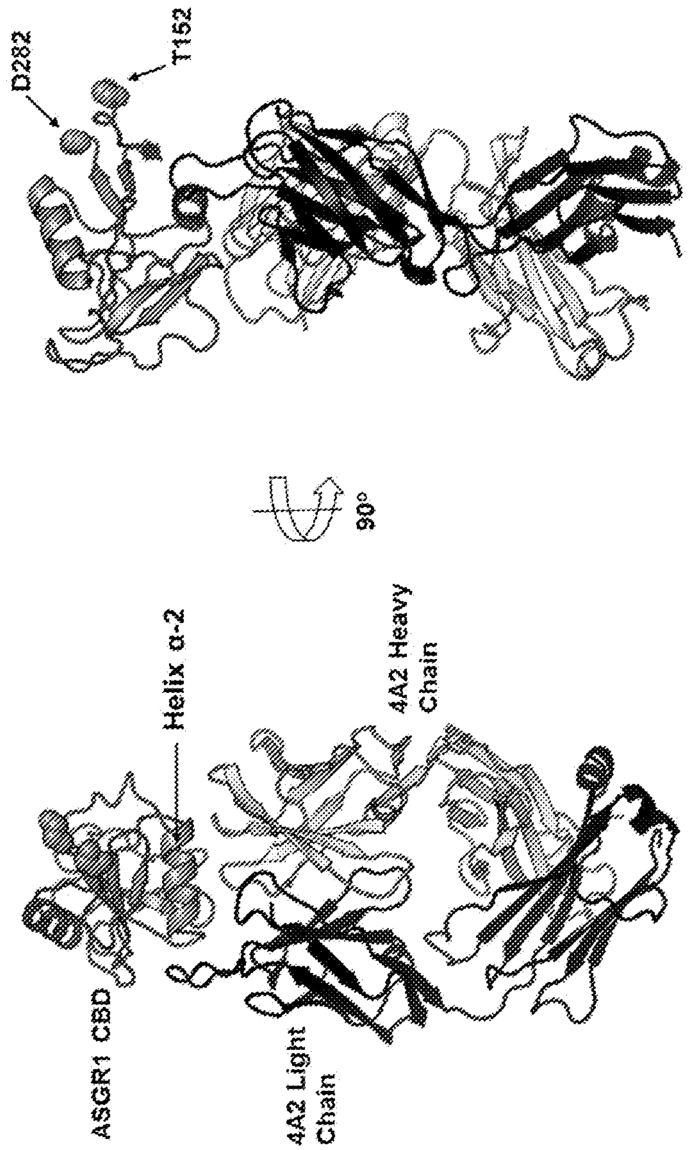
D282
T152
90°
ASGR1 CBD
Helix α-2
4A2 Light Chain
4A2 Heavy Chain

Figure 25

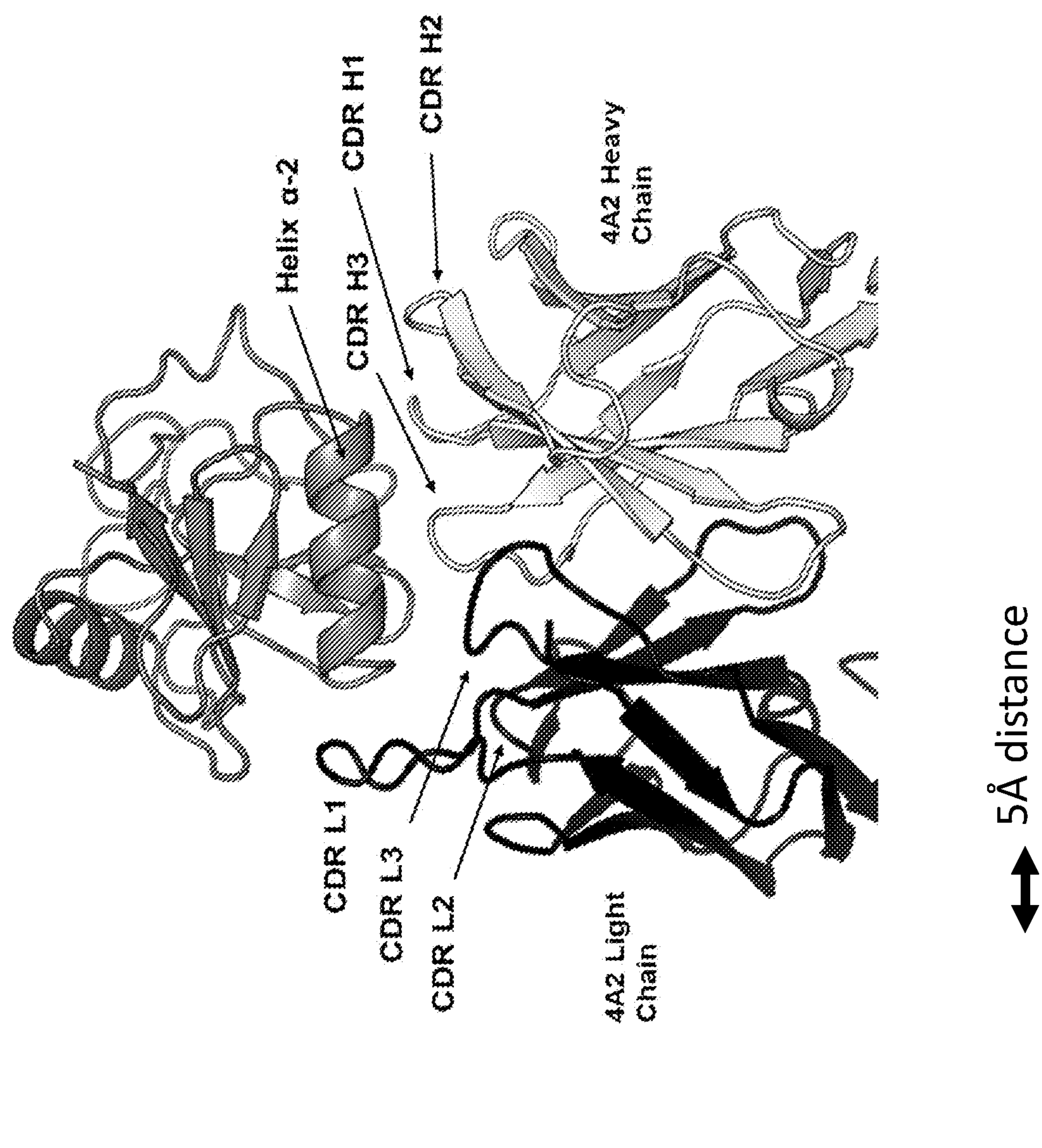
Helix α-2
CDR H1
CDR H2
CDR H3
4A2 Heavy Chain
CDR L1
CDR L3
CDR L2
4A2 Light Chain
5Å distance

Figure 26

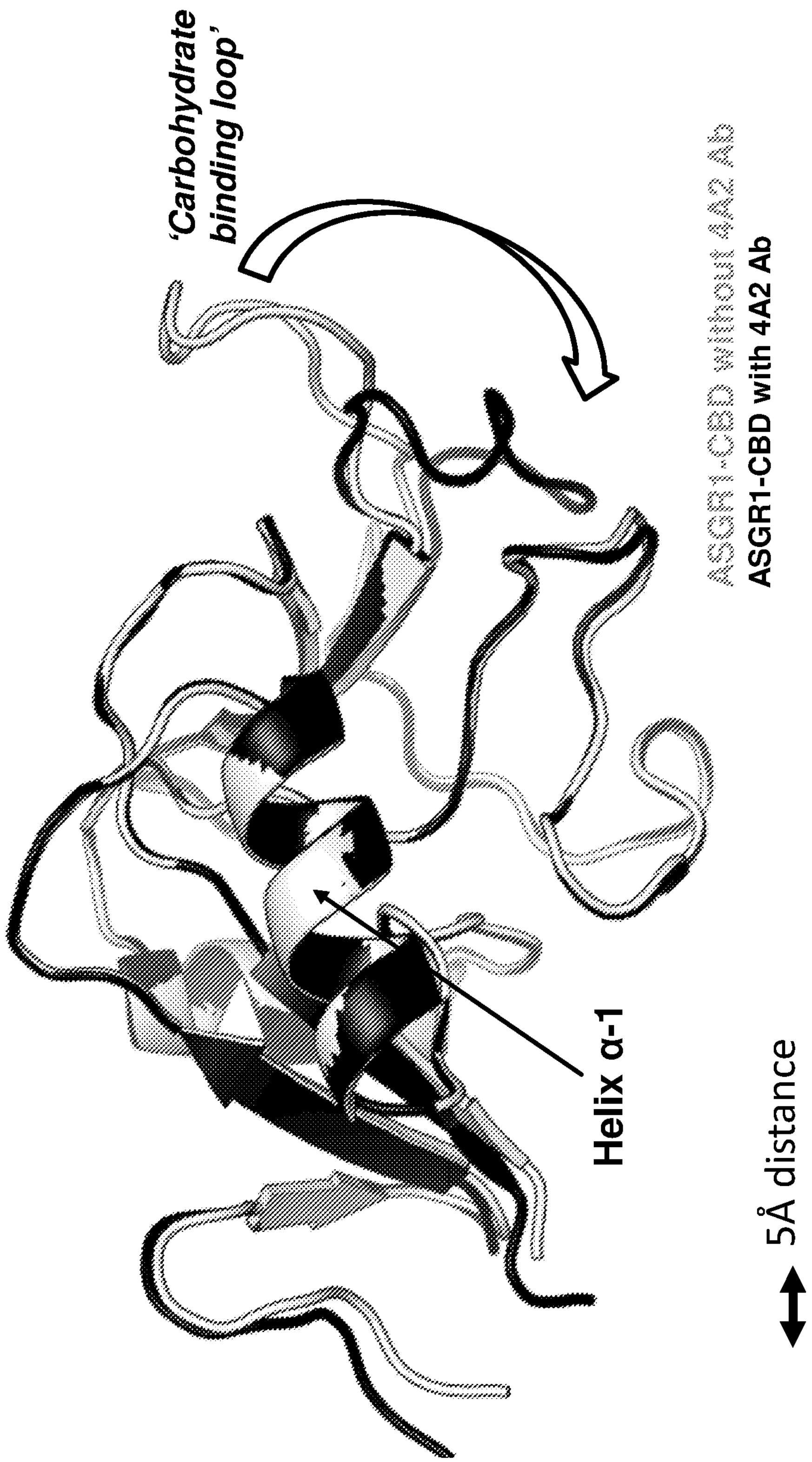
'Carbohydrate binding loop'
ASGR1-CBD without 4A2 Ab
ASGR1-CBD with 4A2 Ab
Helix α-1
5Å distance

Figure 27

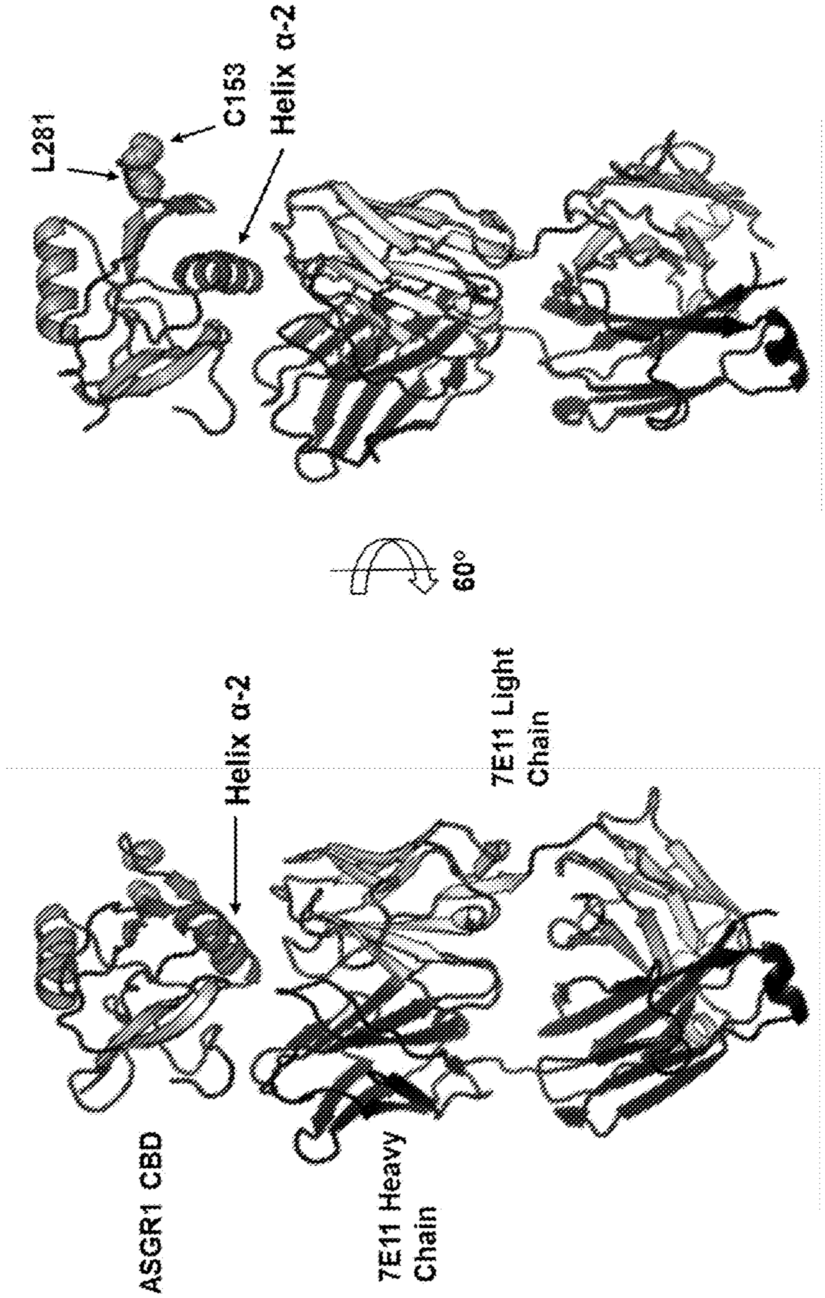

ASGR1 CBD
Helix α-2
7E11 Heavy Chain
7E11 Light Chain
60°
L281
C153
Helix α-2

Figure 28

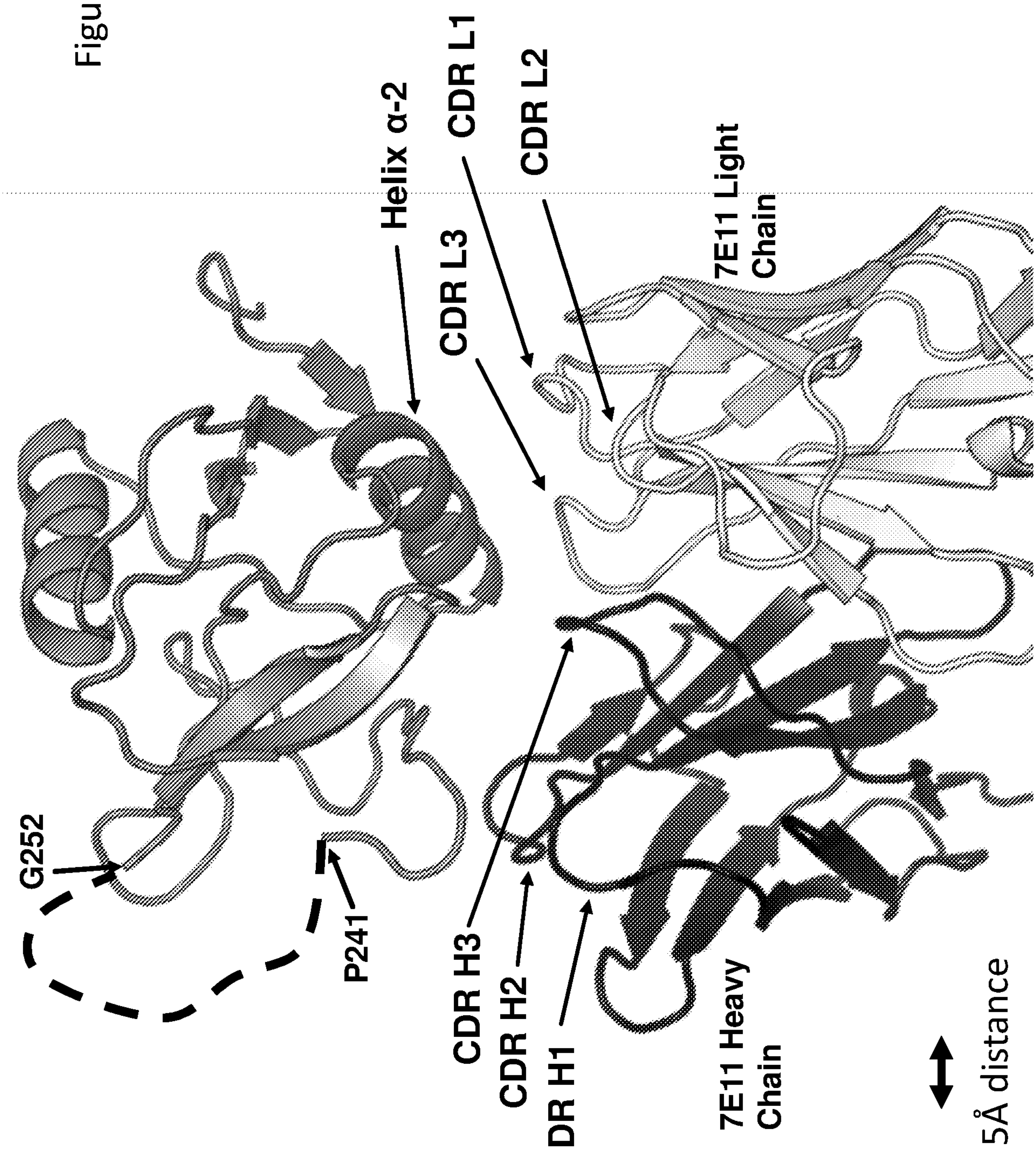
Helix α-2
CDR L1
CDR L2
CDR L3
7E11 Light Chain
G252
P241
CDR H3
CDR H2
CDR H1
7E11 Heavy Chain
5Å distance

Figure 29

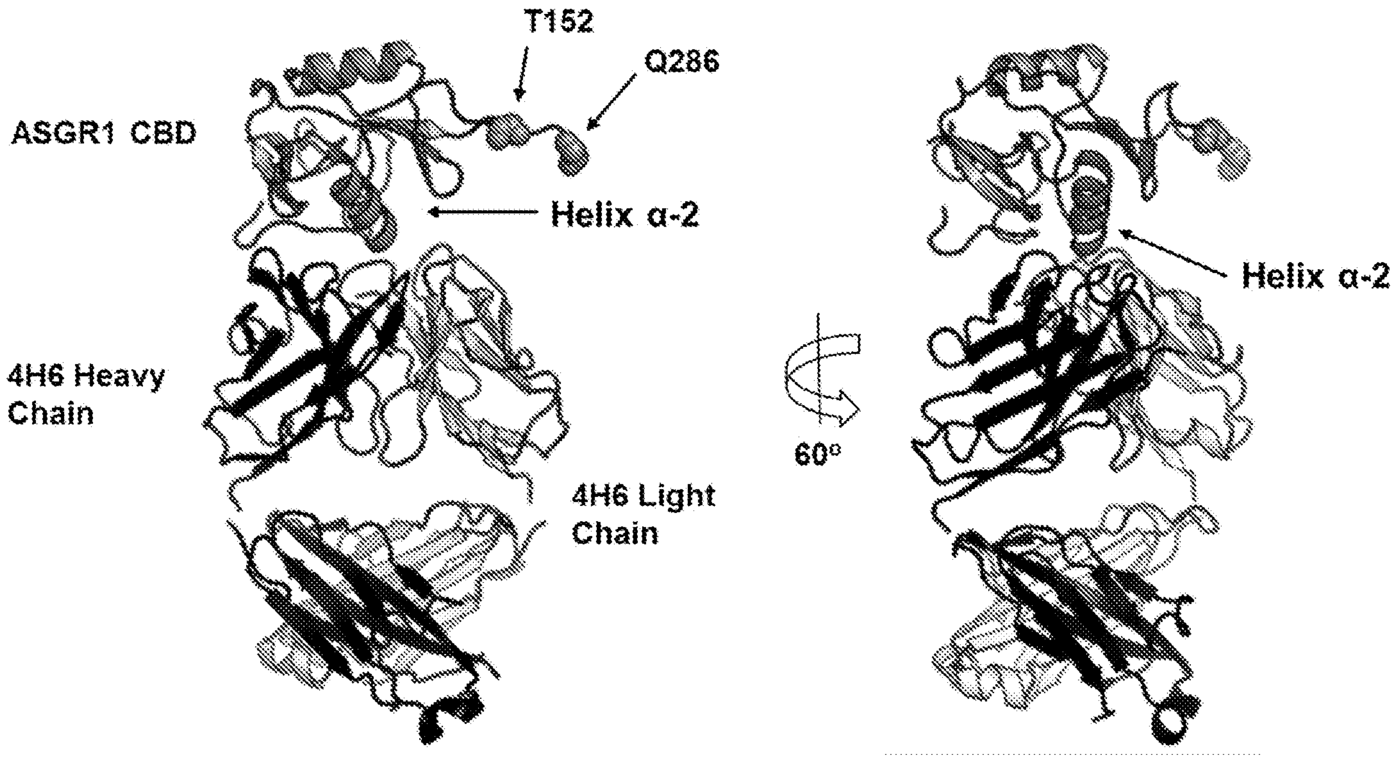
T152
Q286
ASGR1 CBD
Helix α-2
4H6 Heavy Chain
4H6 Light Chain
60°
Helix α-2

Figure 30

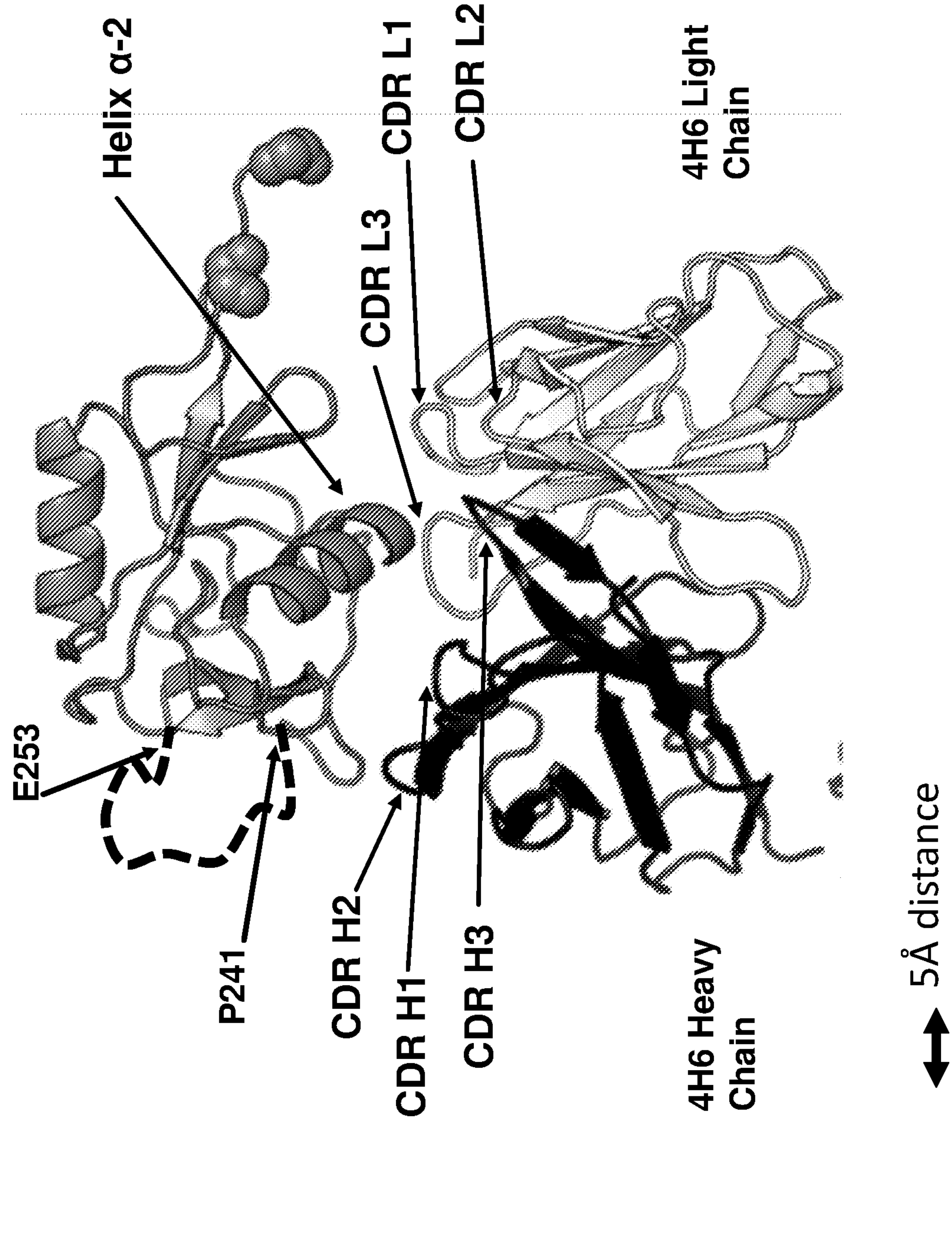
Helix α-2
CDR L1
CDR L2
CDR L3
4H6 Light Chain
E253
P241
CDR H2
CDR H1
CDR H3
4H6 Heavy Chain
5Å distance

Figure 31

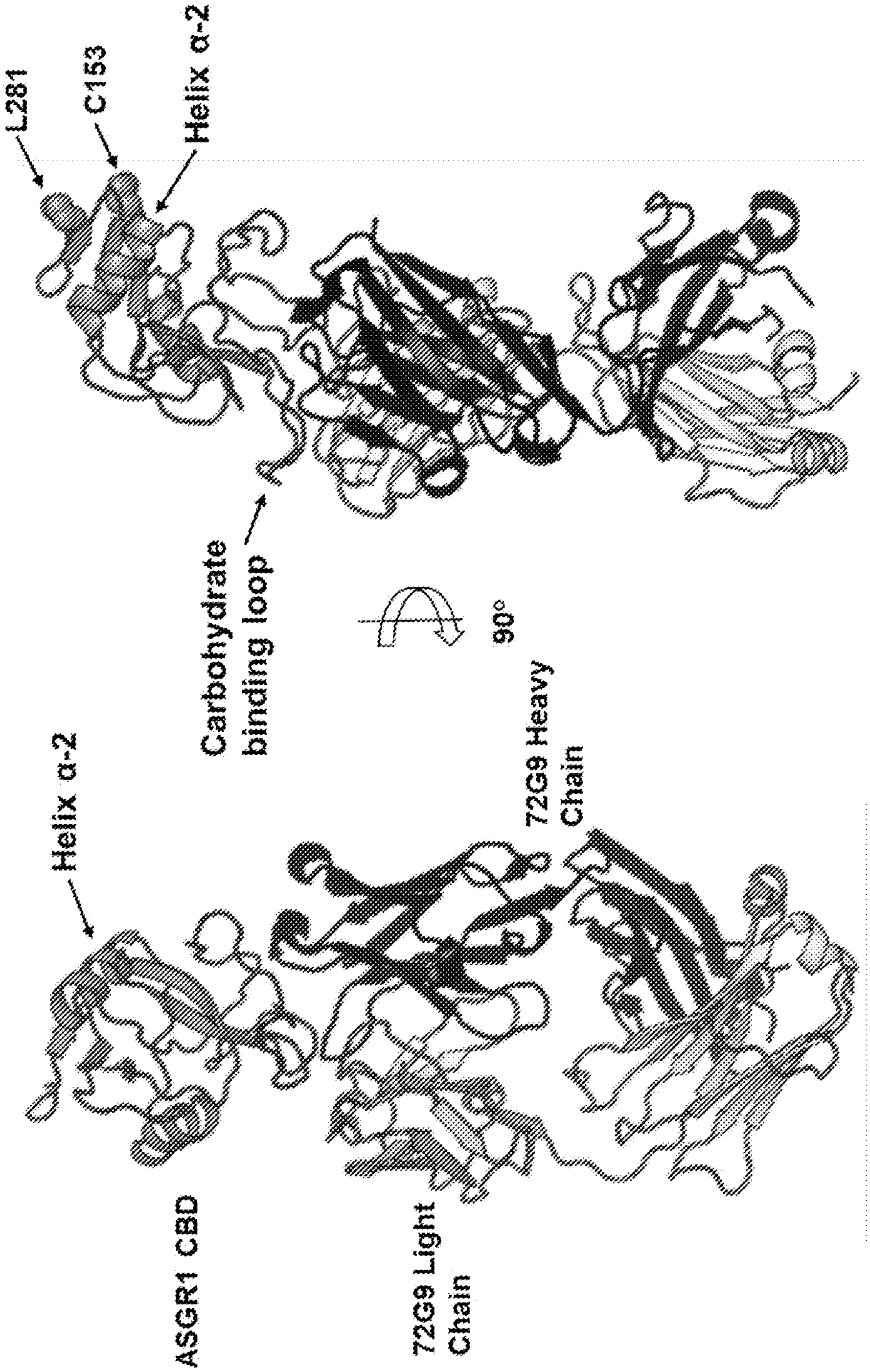
L281
C153
Helix α-2
Carbohydrate binding loop
90°
Helix α-2
72G9 Heavy Chain
ASGR1 CBD
72G9 Light Chain

Figure 32

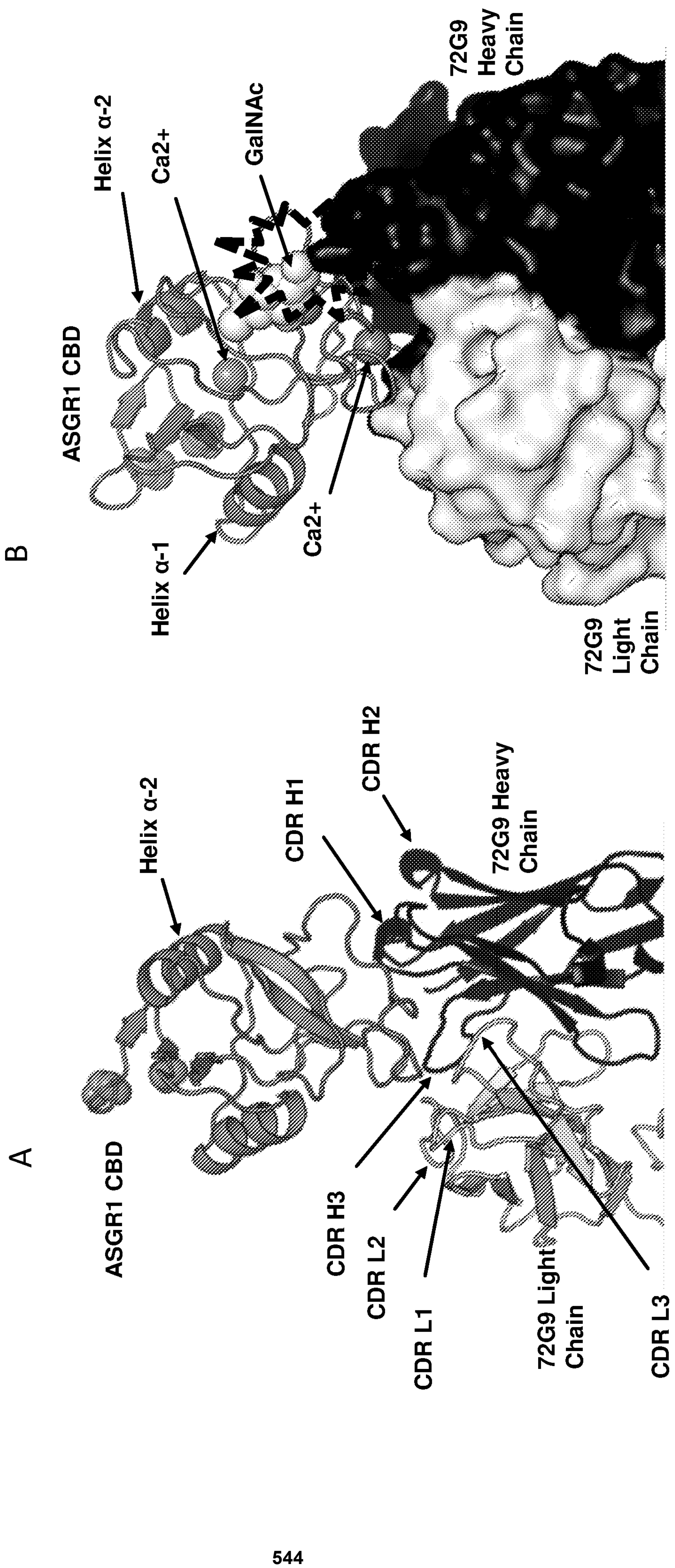
A
ASGR1 CBD
Helix α-2
CDR H1
CDR H2
72G9 Heavy Chain
CDR H3
CDR L2
CDR L1
72G9 Light Chain
CDR L3
B
ASGR1 CBD
Helix α-2
Ca2+
GalNAc
72G9 Heavy Chain
Helix α-1
Ca2+
72G9 Light Chain

Figure 33

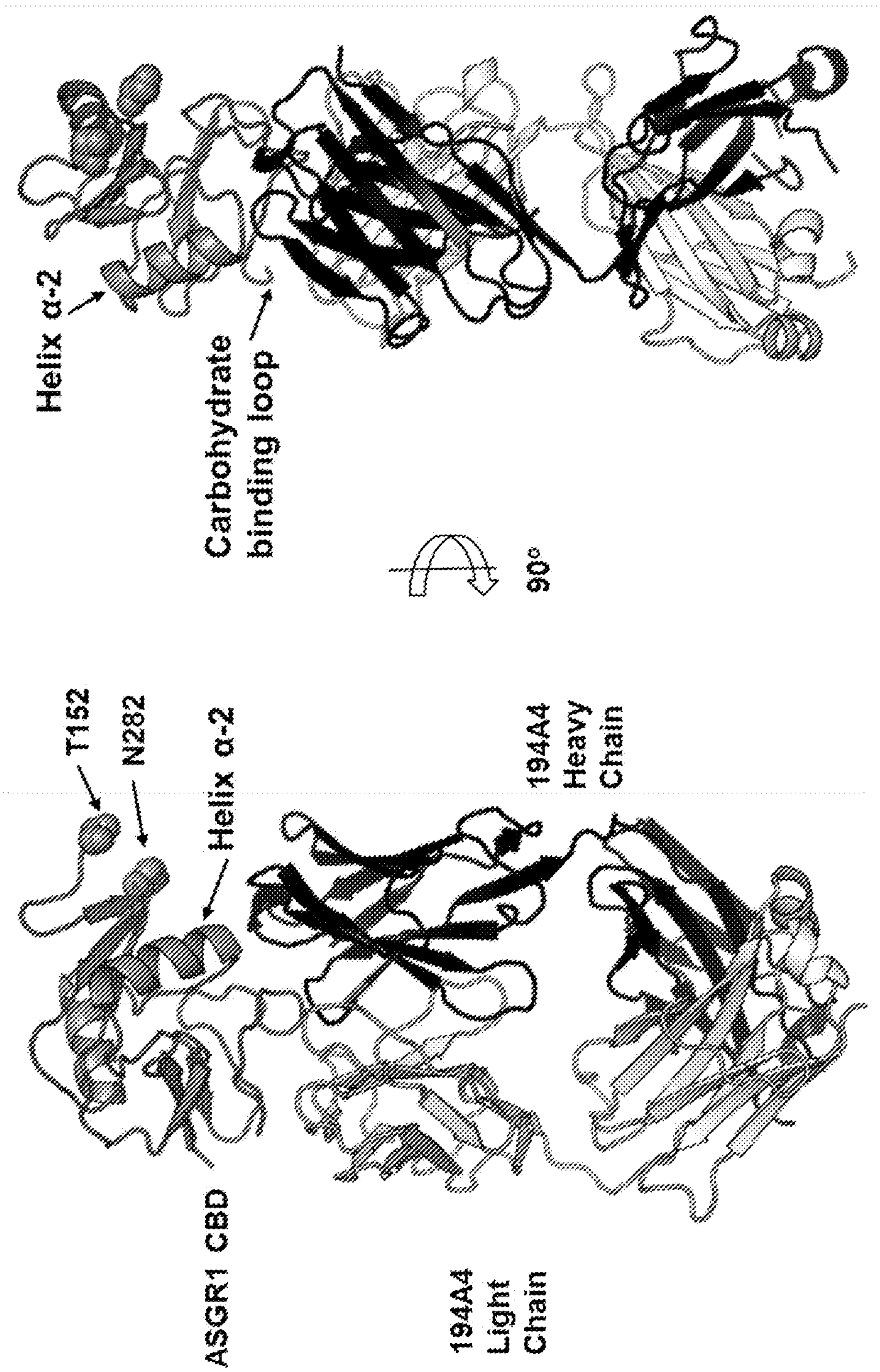
Helix α-2
Carbohydrate binding loop
90°
T152
N282
Helix α-2
194A4 Heavy Chain
ASGR1 CBD
194A4 Light Chain

Figure34

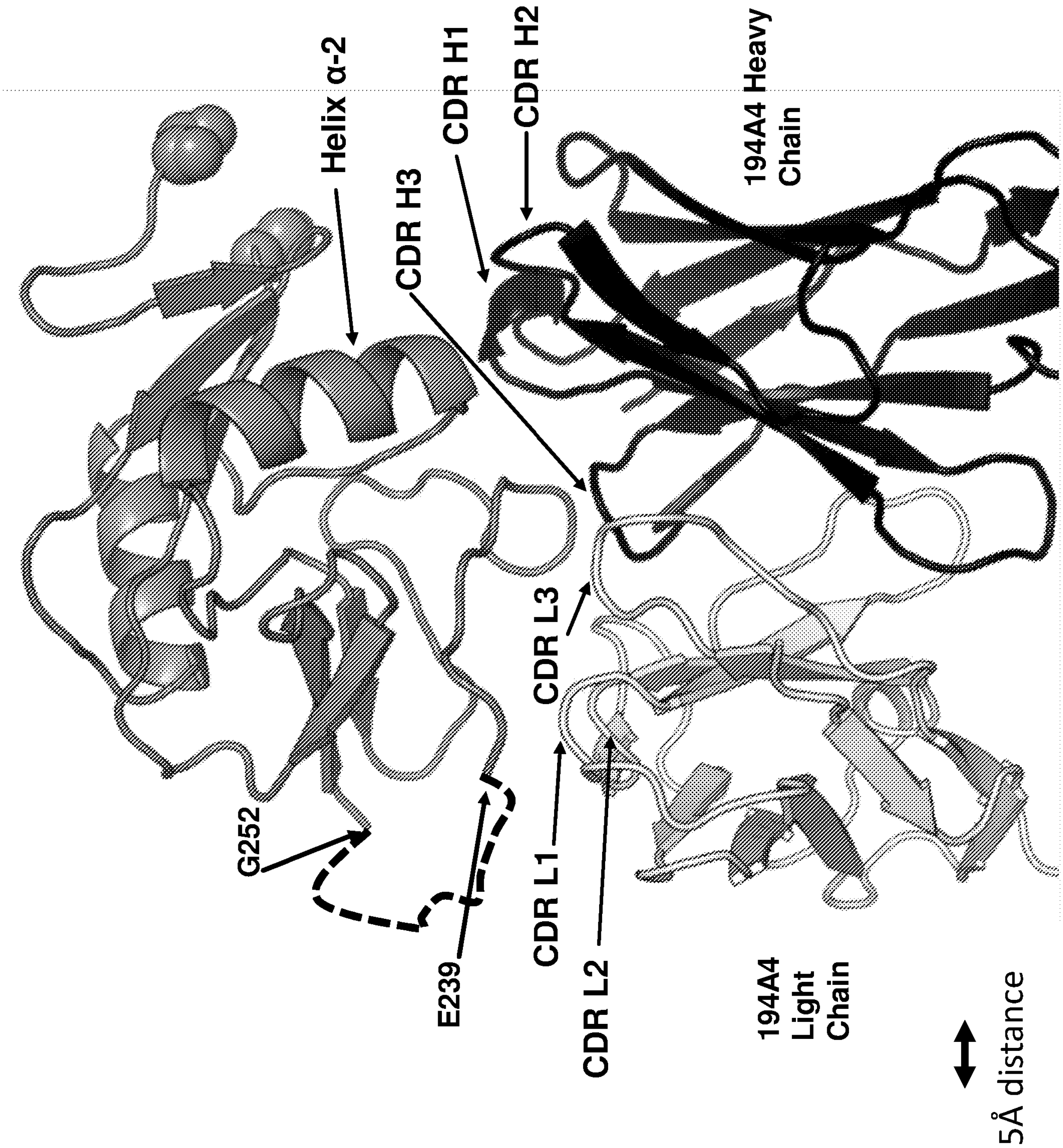
Helix α-2
CDR H1
CDR H2
CDR H3
194A4 Heavy Chain
G252
E239
CDR L1
CDR L2
CDR L3
194A4 Light Chain
5Å distance

Figure 35

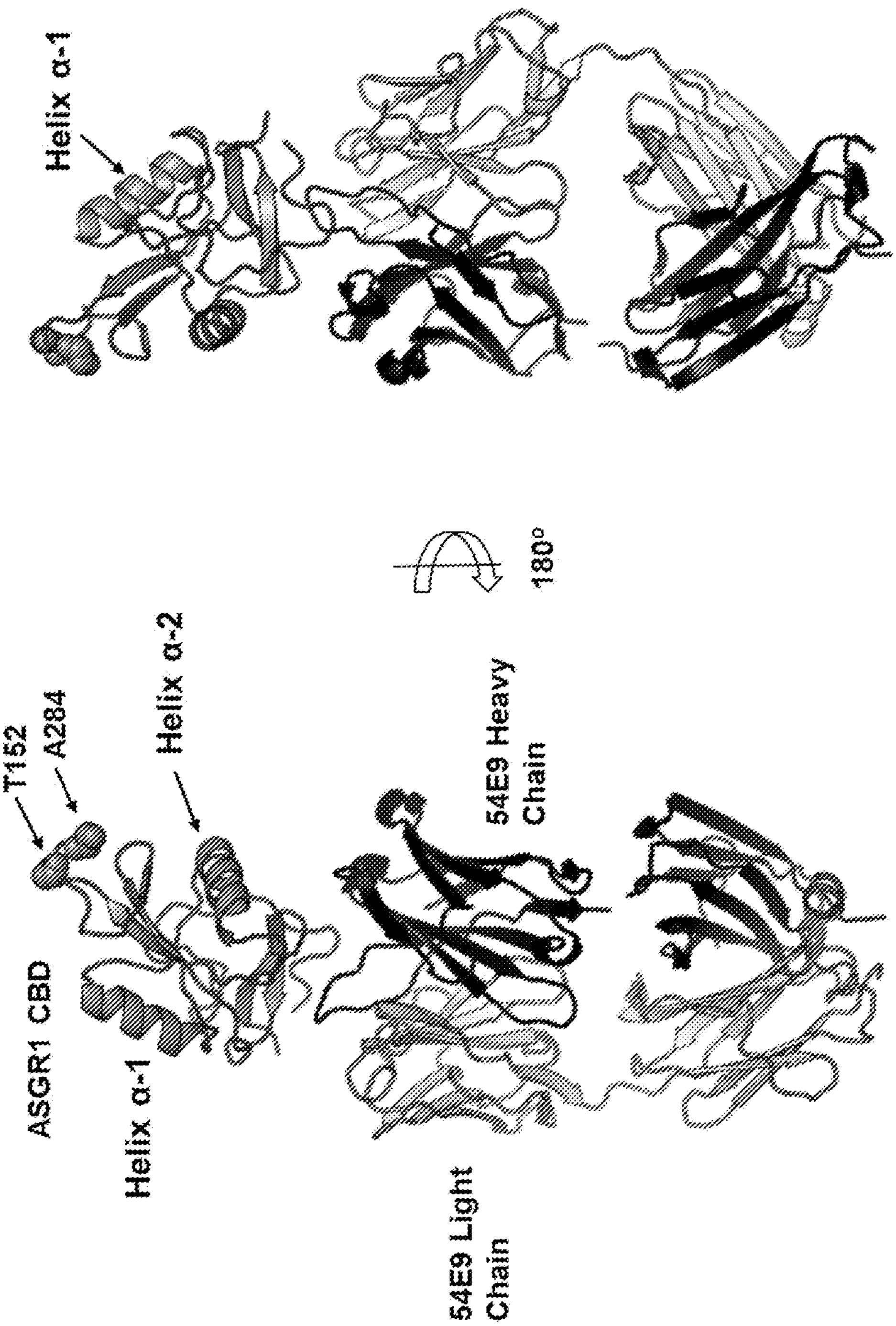
Helix α-1
180°
ASGR1 CBD
T152
A284
Helix α-2
Helix α-1
54E9 Heavy Chain
54E9 Light Chain

Figure 36

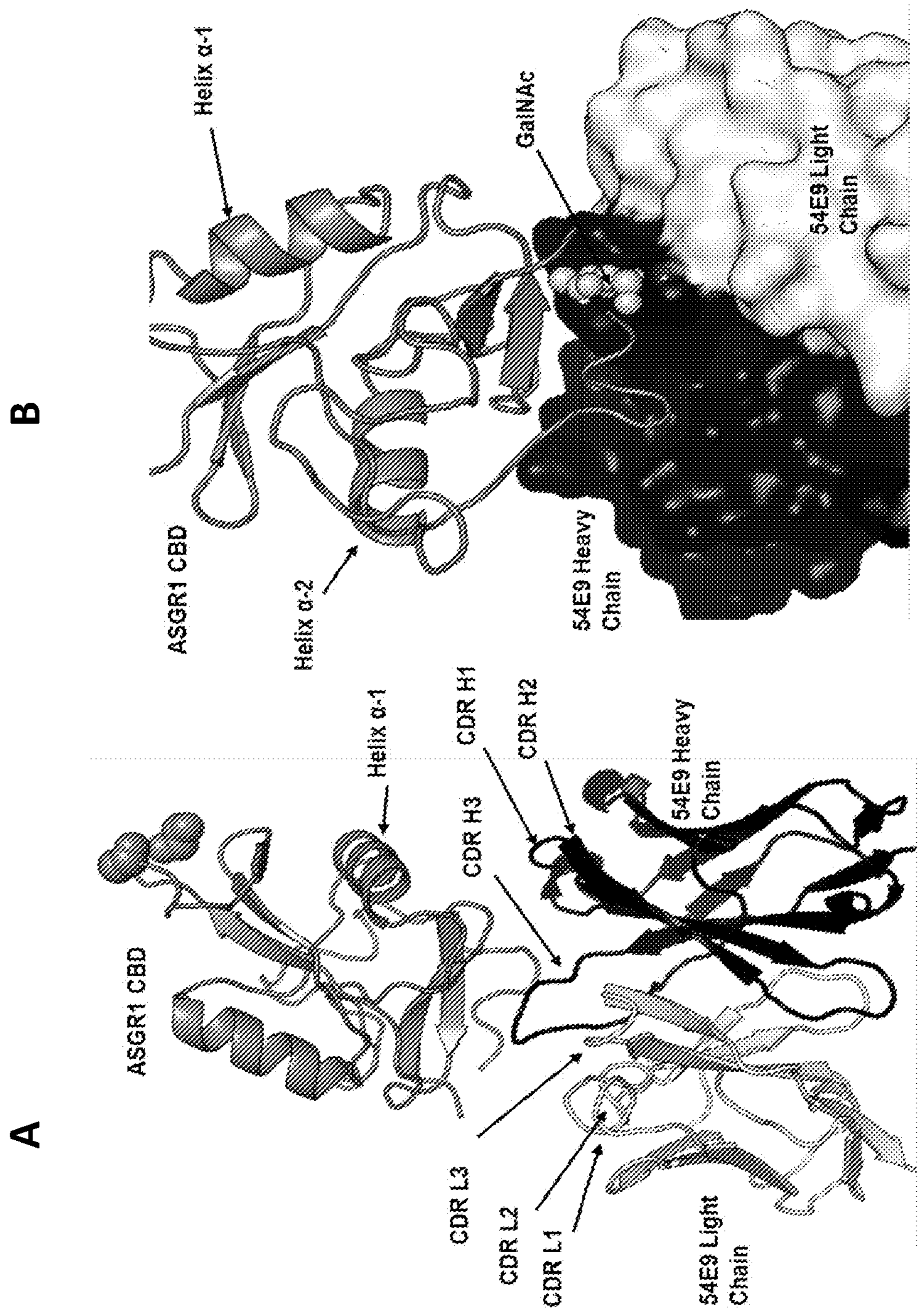
A
ASGR1 CBD
Helix α-1
CDR H1
CDR H2
CDR H3
54E9 Heavy Chain
CDR L3
CDR L2
CDR L1
54E9 Light Chain
B
ASGR1 CBD
Helix α-1
Helix α-2
GalNAc
54E9 Light Chain
54E9 Heavy Chain

Figure 37

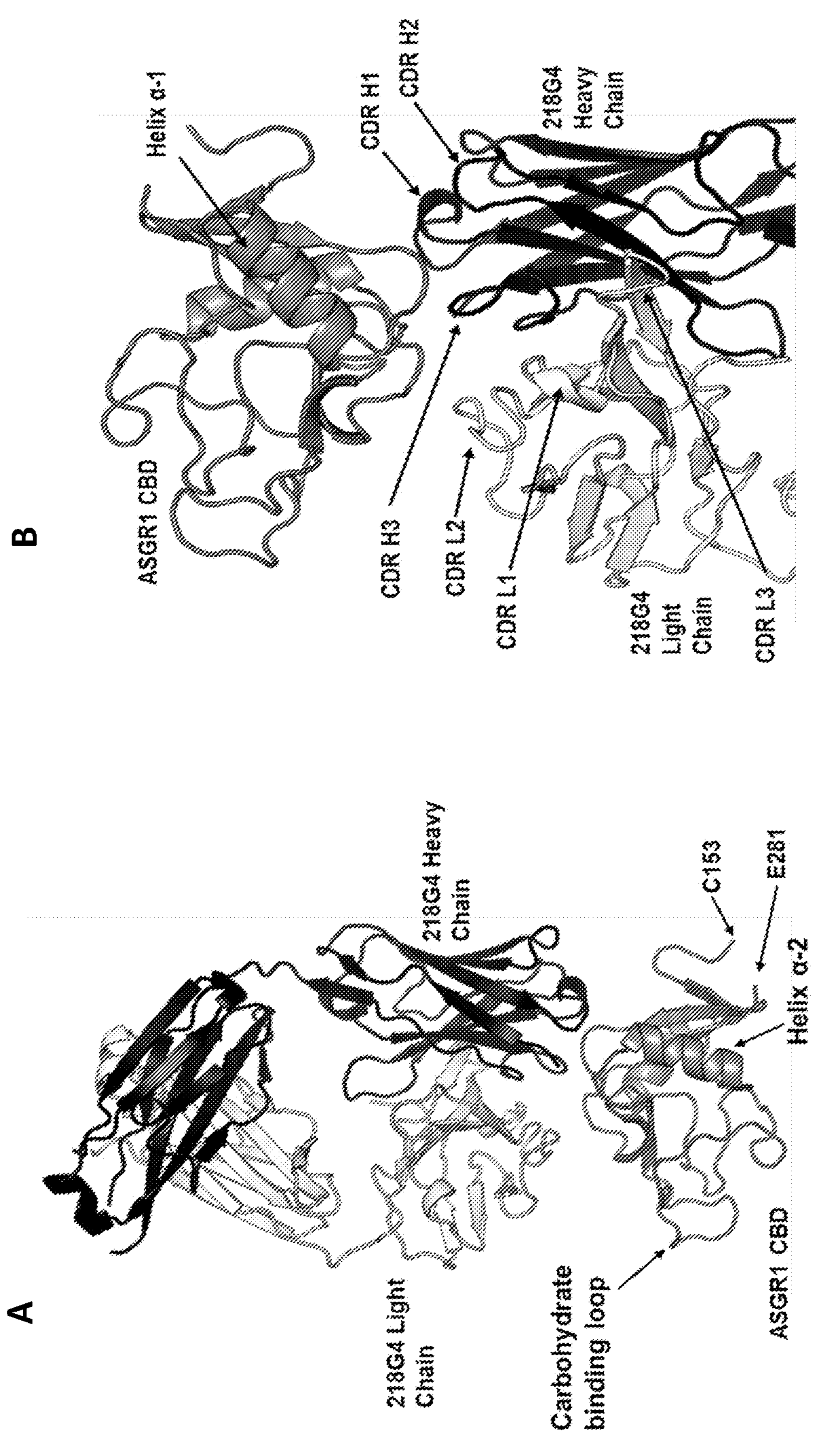
A
218G4 Light Chain
218G4 Heavy Chain
Carbohydrate binding loop
ASGR1 CBD
Helix α-2
C153
E281
B
ASGR1 CBD
Helix α-1
CDR H1
CDR H2
218G4 Heavy Chain
CDR H3
CDR L2
CDR L1
218G4 Light Chain
CDR L3

Figure 38

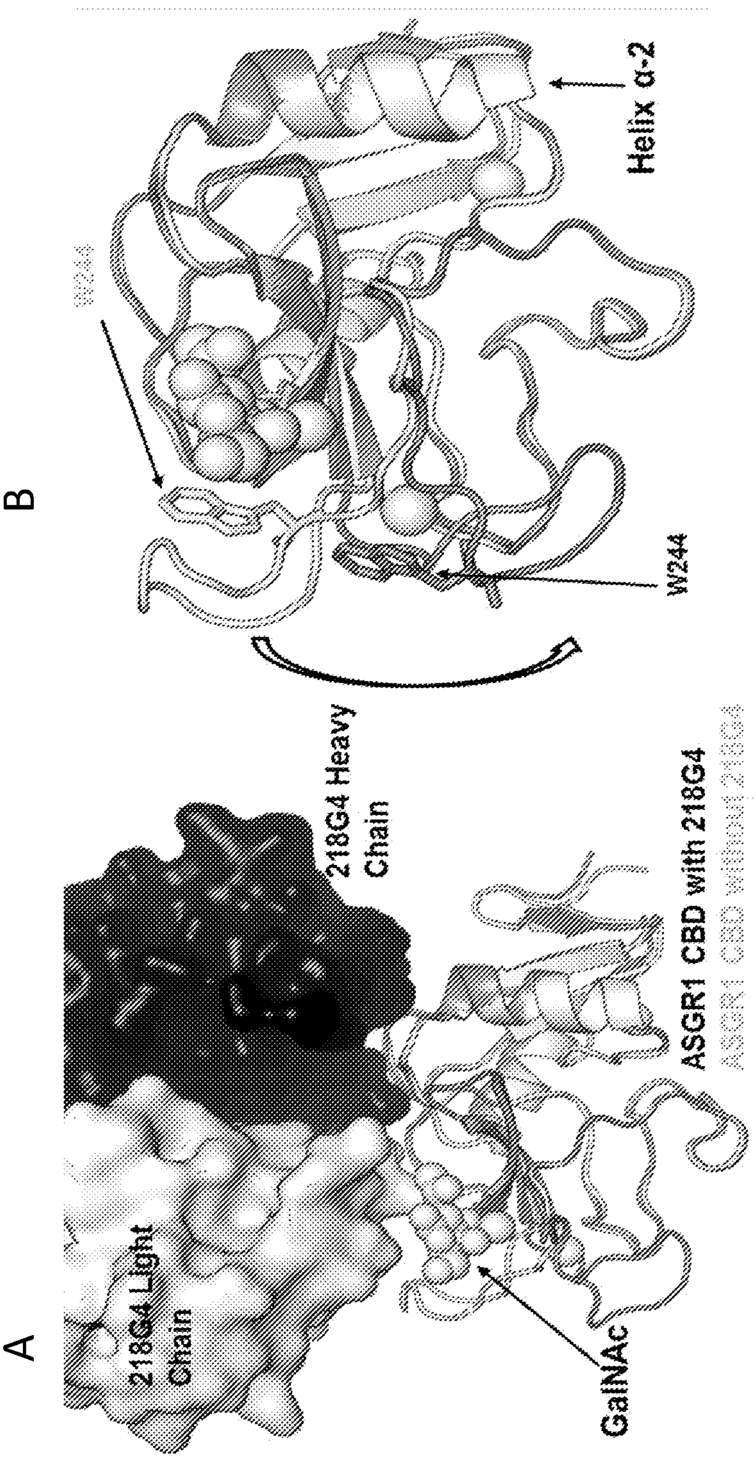
A
B
218G4 Light Chain
218G4 Heavy Chain
GalNAc
ASGR1 CBD with 218G4
ASGR1 CBD without 218G4
W244
W244
Helix α-2

Figure 39

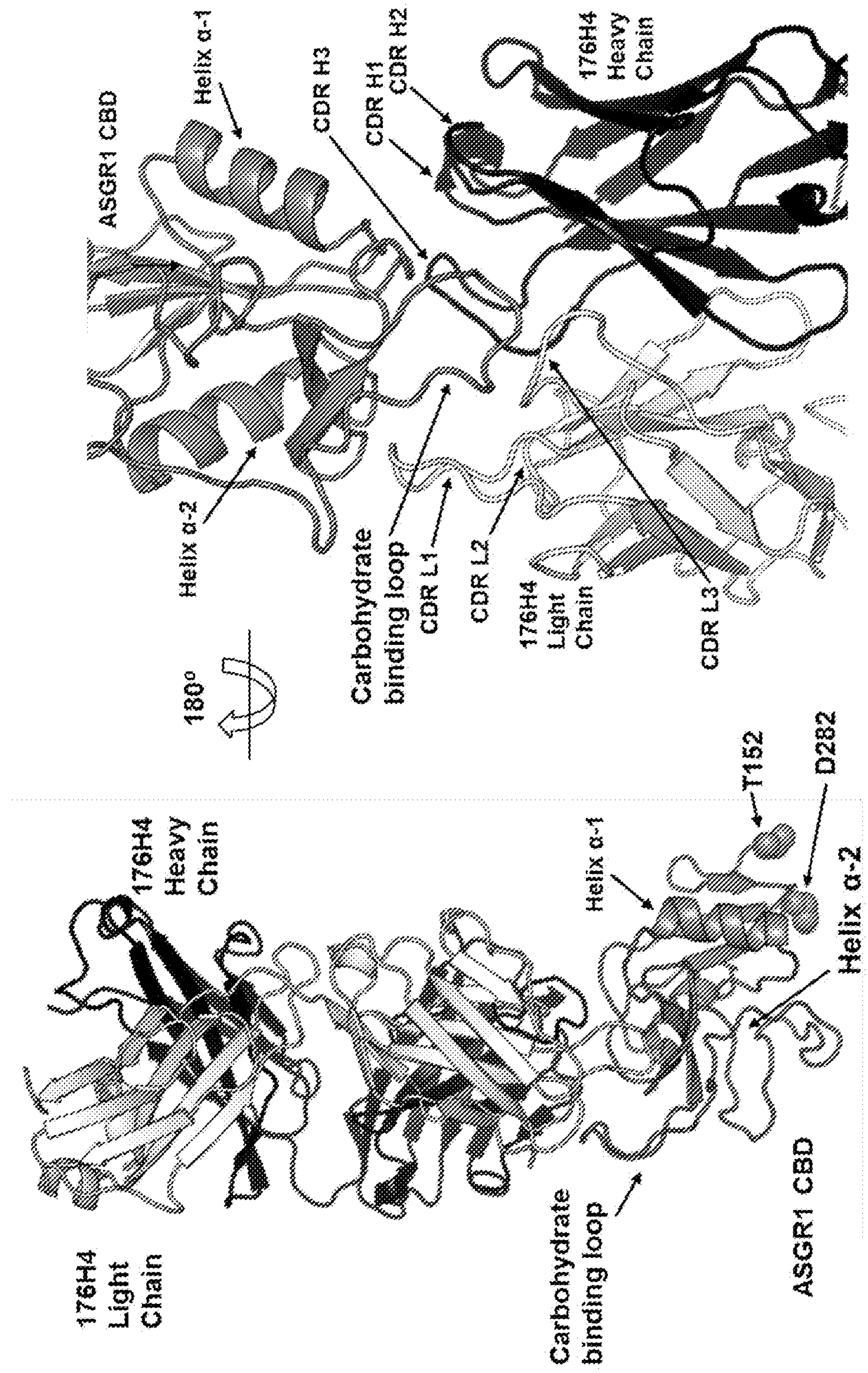
ASGR1 CBD
Helix α-1
CDR H3
CDR H1
CDR H2
176H4
Heavy
Chain
Helix α-2
Carbohydrate
binding loop
CDR L1
CDR L2
176H4
Light
Chain
CDR L3
180°
176H4
Heavy
Chain
Helix α-1
T152
D282
Helix α-2
176H4
Light
Chain
Carbohydrate
binding loop
ASGR1 CBD

Figure 40

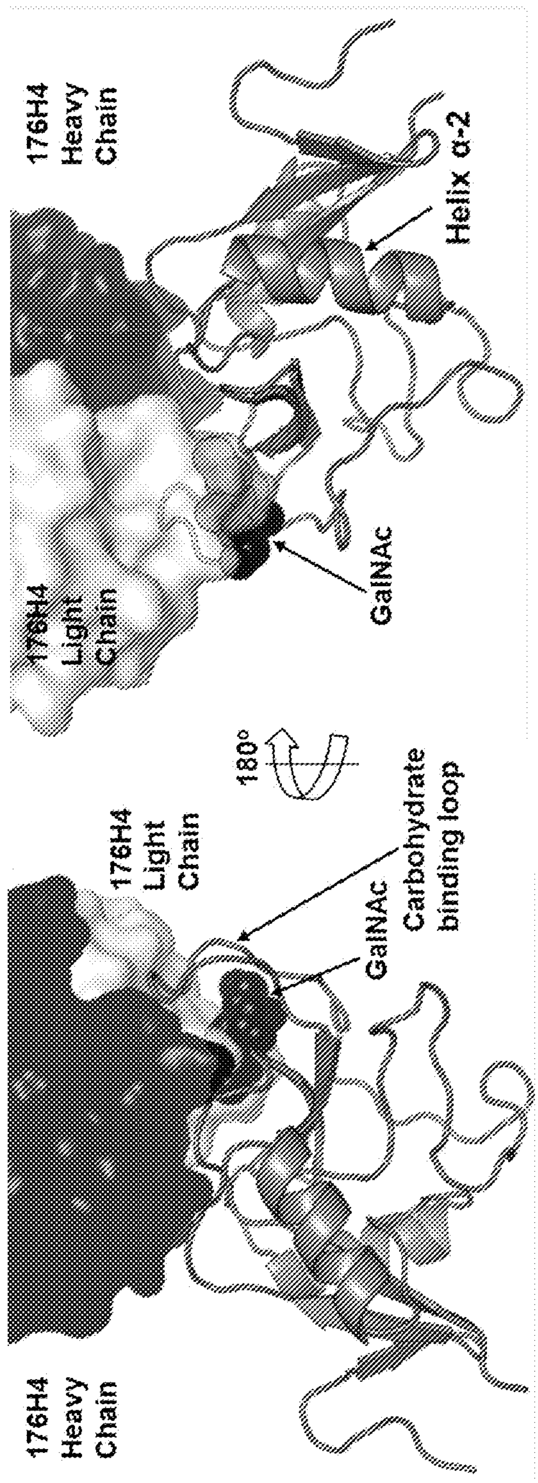
176H4 Heavy Chain
176H4 Light Chain
GalNAc
Carbohydrate binding loop
180°
176H4 Light Chain
GalNAc
176H4 Heavy Chain
Helix α-2

Figure 41

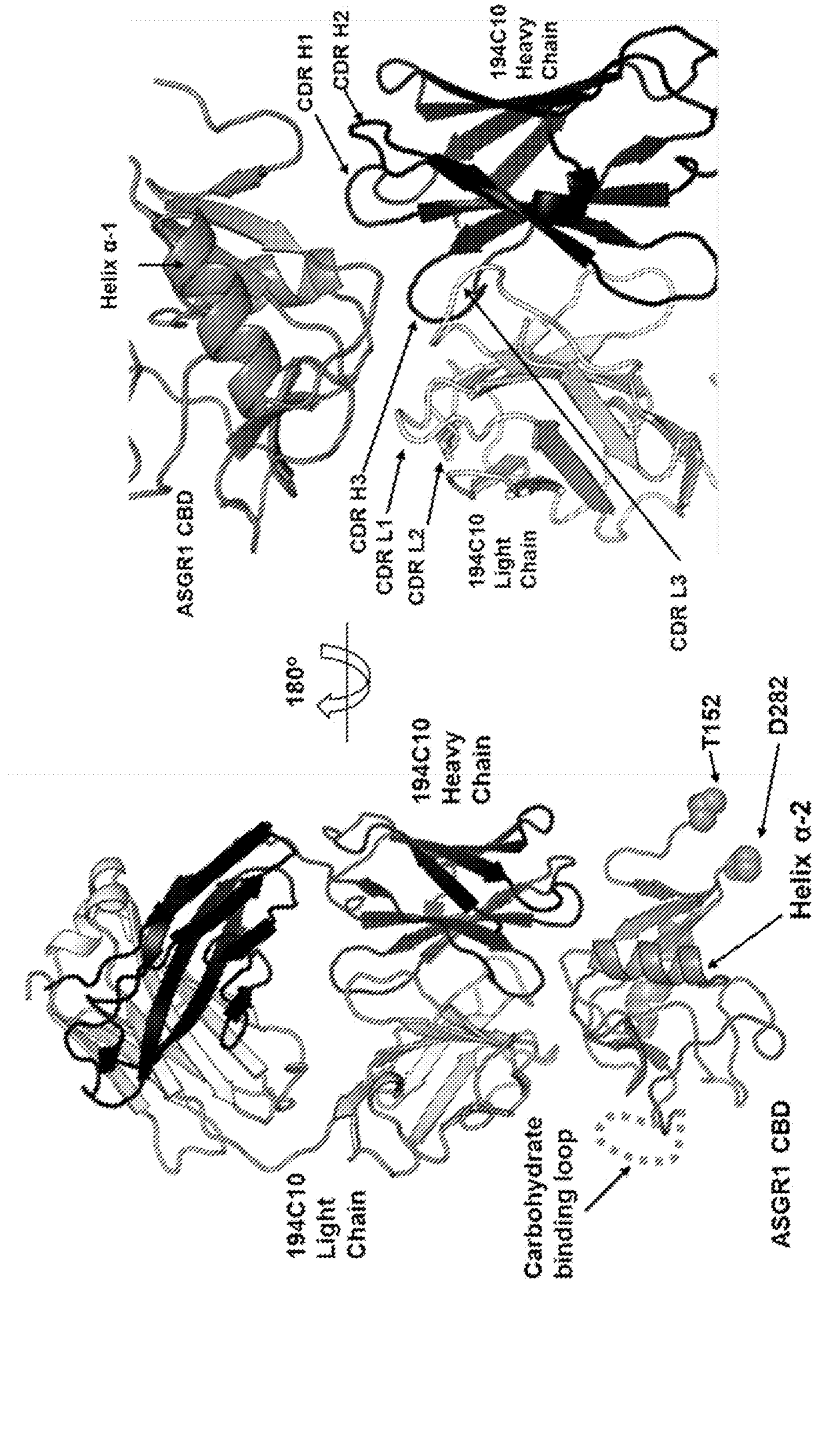
Helix α-1
ASGR1 CBD
CDR H1
CDR H2
194C10
Heavy
Chain
CDR H3
CDR L1
CDR L2
194C10
Light
Chain
CDR L3
180°
194C10
Heavy
Chain
T152
D282
Helix α-2
194C10
Light
Chain
Carbohydrate
binding loop
ASGR1 CBD

Figure 42

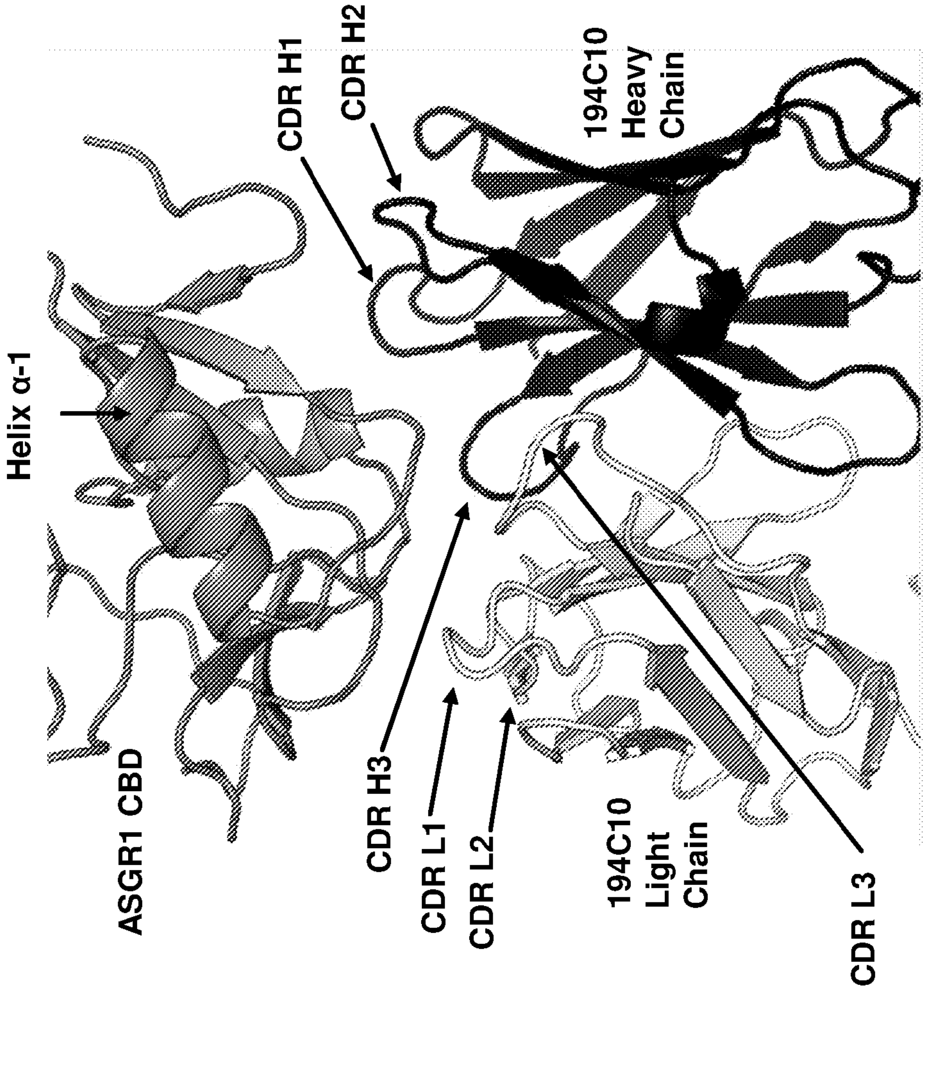
Helix α-1
ASGR1 CBD
CDR H1
CDR H2
194C10
Heavy
Chain
CDR H3
CDR L1
CDR L2
194C10
Light
Chain
CDR L3

# Figure 43

## Binding of antibody 7F4 to parental and ASGR-expressing CHO-S cells

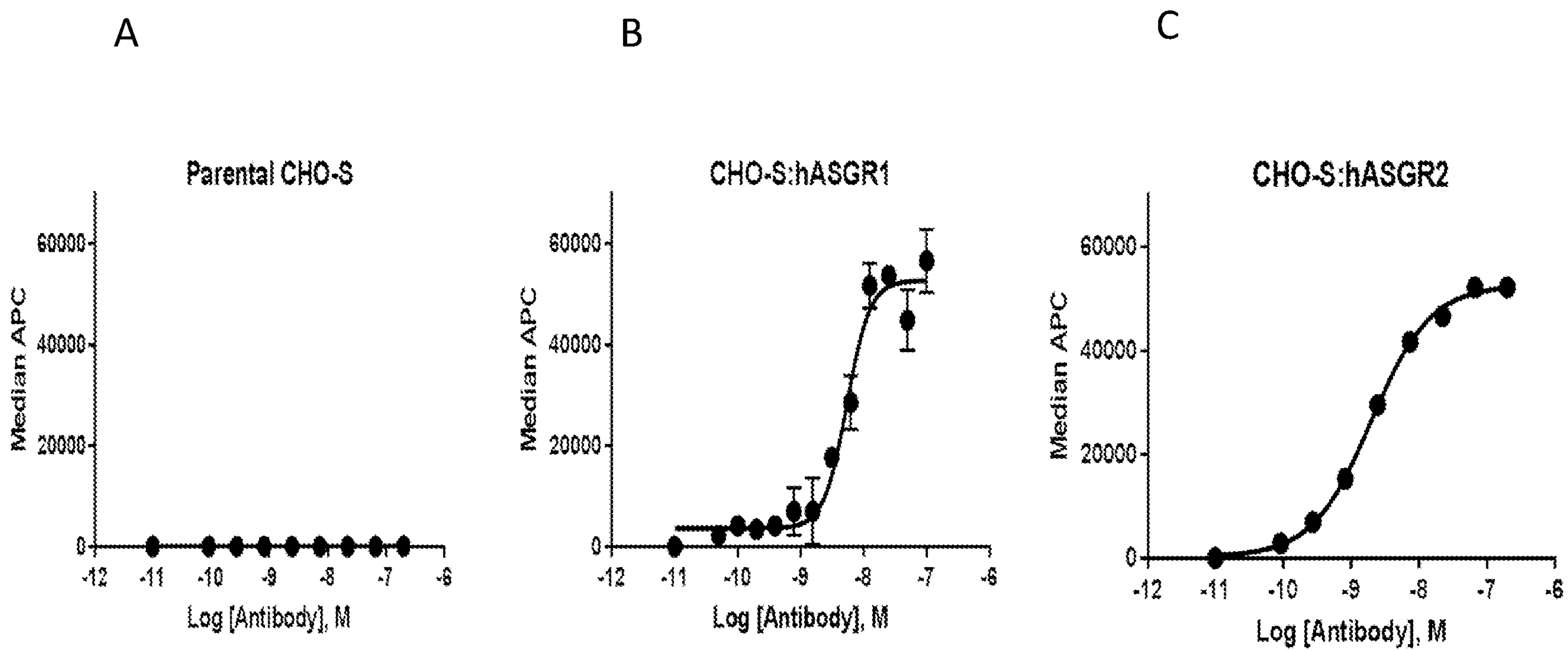

Figure 44

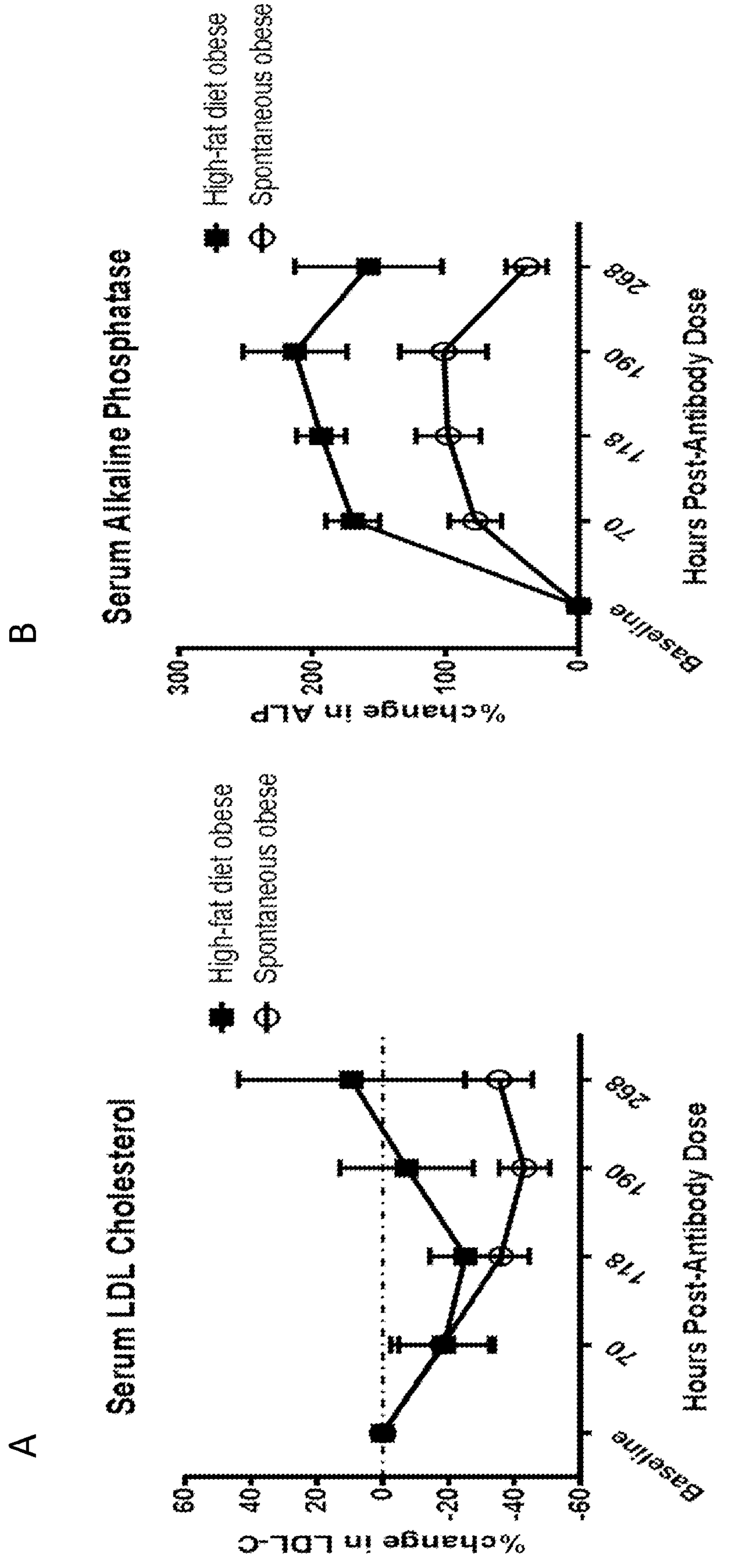


Data expressed as % change from baseline

Figure 45

A

B

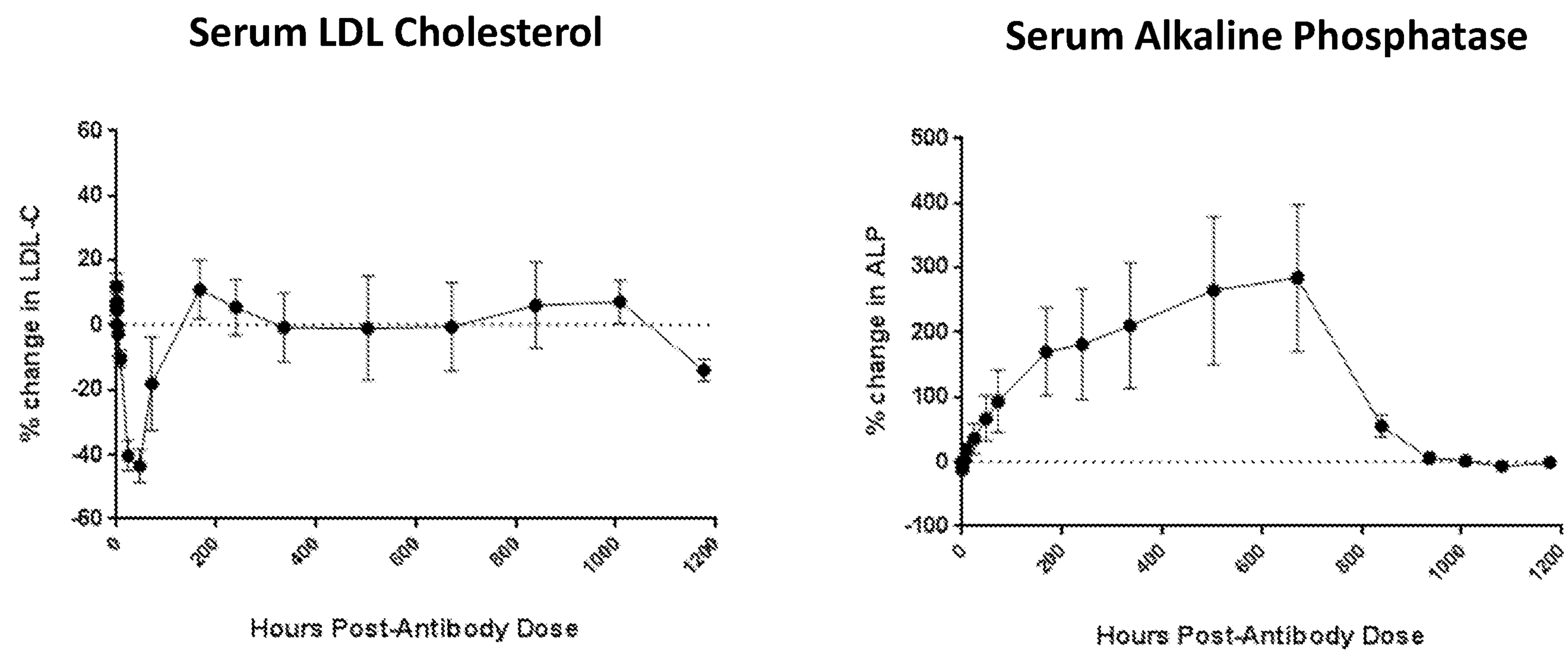


Data expressed as % change from baseline

# Figure 46

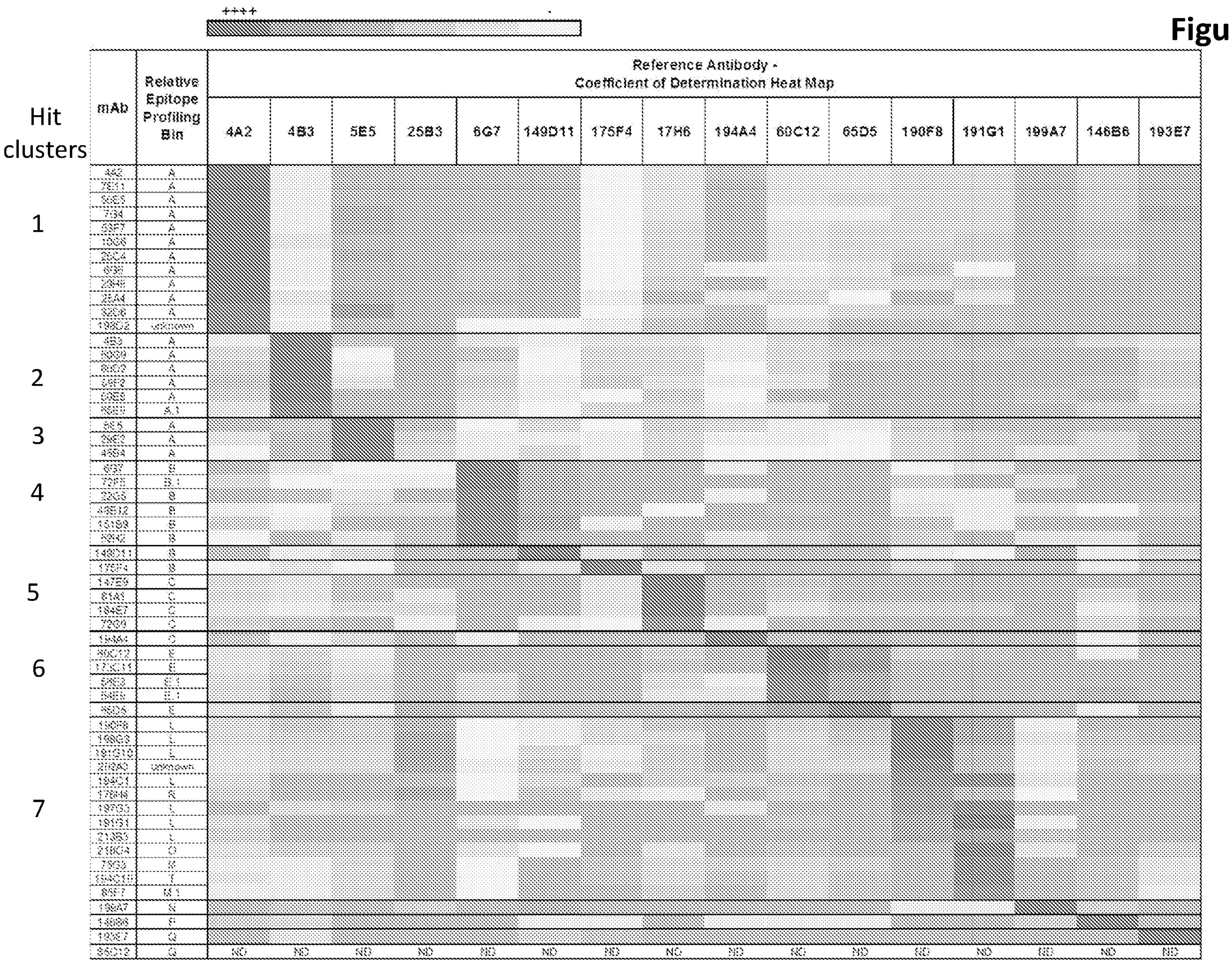
++++
-
Hit clusters
1
2
3
4
5
6
7
mAb
Relative Epitope Profiling Bin
Reference Antibody - Coefficient of Determination Heat Map
4A2
4B3
5E5
25B3
6G7
149D11
175F4
17H6
194A4
60C12
65D5
190F8
191G1
199A7
146B6
193E7
ND

Figure 47

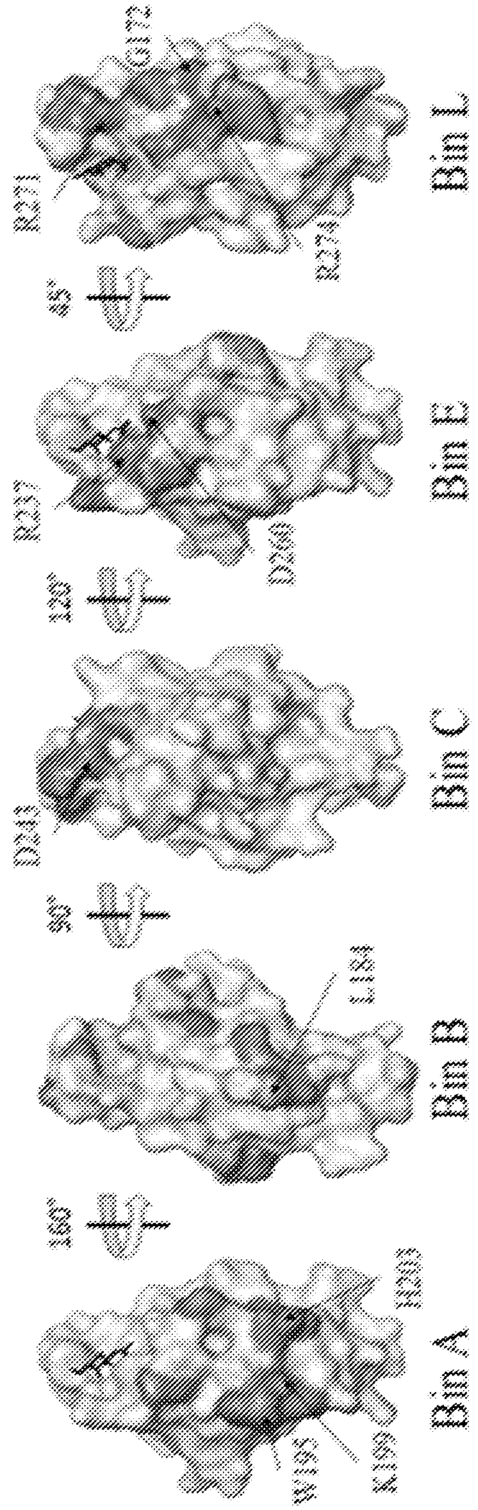
Bin A
Bin B
Bin C
Bin E
Bin L
160°
90°
120°
45°
W195
K199
H203
L184
D243
R237
D260
R271
R274

# FIGURE 48

Table 1

## Immunogens

| Name | Sequence Range | Tags | Amino Acid Sequence | Vector | SEQ ID NO: |
|---|---|---|---|---|---|
| muASGR1 | 1-284 | TCE | MTKDYQDFQHLDNDNDHHQLRRGPPPTPRLLQRLCSGSRLLLLSSSLSILLLVVVCVITSQNSQLREDLLALRQNF SNLTVSTEDQVKALSTQGSSVGRKMKLVESKLEKQQKDLTEDHSSLLLHVKQLVSDVRSLSCQMAAFRGNGSER TCCPINWVEYEGSCYWFSSSVRPWTEADKYCQLENAHLVVVTSRDEQNFLQRHMGPLNTWIGLTDQNGPWKW VDGTDYETGFQNWRPEQPDNWYGHGLGGGEDCAHFTTDGRWNDDVCRRPYRWVCETKLDKAN | pTT5 | 1 |
| muAsgr2 | 1-301 | TCE | MEKDCQDIQQLDSEENDHQLSGDDEHGSHVQDPRIENPHWKGQPLSRPFPQRLCSTFRLSLLALAFNILLLVVIC VVSSQSIQLQEEFRTLKETFSNFSSSTLMEFGALDTLGGSTNAILTSWLAQLEEKQQQLKADHSTLLFHLKHFPMD LRTLTCQLAYFQSNGTECCPVNWVEFGGSCYWFSRDGLTWAEADQYCQLENAHLLVINSREEQDFVVKHRSQF HIWIGLTDRDGSWKWVDGTDYRSNYRNWAFTQPDNWQGHEQGGGEDCAEILSDGHWNDNFCQQVNRWVCE KRRNITH | pTT5 | 2 |
| muASGR1(ECD) | 63-284 | TCE | SQLREDLLALRQNFSNLTVSTEDQVKALSTQGSSVGRKMKLVESKLEKQQKDLTEDHSSLLLHVKQLVSDVRSLS CQMAAFRGNGSERTCCPINWVEYEGSCYWFSSSVRPWTEADKYCQLENAHLVVVTSRDEQNFLQRHMGPLNT WIGLTDQNGPWKWVDGTDYETGFQNWRPEQPDNWYGHGLGGGEDCAHFTTDGRWNDDVCRRPYRWVCET KLDKAN | pSLX235a | 3 |
| muASGR1(CBD) | 153-284 | TCE | CPINWVEYEGSCYWFSSSVRPWTEADKYCQLENAHLVVVTSRDEQNFLQRHMGPLNTWIGLTDQNGPWKWVD GTDYETGFQNWRPEQPDNWYGHGLGGGEDCAHFTTDGRWNDDVCRRPYRWVCETKLDKAN | pSLX235a | 4 |
| huASGR1 | 1-291 | TCE | MTKEYQDLQHLDNEESDHHQLRKGPPPPQPLLQRLCSGPRLLLLSLGLSLLLLVVVCVIGSQNSQLQEELRGLRE TFSNFTASTEAQVKGLSTQGGNVGRKMKSLESQLEKQQKDLSEDHSSLLLHVKQFVSDLRSLSCQMAALQGNG SERTCCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPYRWVCETELDKASQEP PLL | pTT5 | 5 |
| huASGR2 | 1-287 | TCE | MAKDFQDIQQLSSEENDHPFHQGPPPAQPLAQRLCSMVCFSLLALSFNILLLVVICVTGSQSAQLQAELRSLKEAF SNFSSSTLTEVQAISTHGGSVGDKITSLGAKLEKQQQDLKADHDALLFHLKHFPVDLRFVACQMELLHSNGSQRT CCPVNWVEHQGSCYWFSHSGKAWAEAEKYCQLENAHLVVINSWEEQKFIVQHTNPFNTWIGLTDSDGSWKWV DGTDYRHNYKNWAVTQPDNWHGHELGGSEDCVEVQPDGRWNDDFCLQVYRWVCEKRRNATGEVA | pTT5 | 6 |
| huASGR1(ECD) | 64-291 | TCE | SQLQEELRGLRETFSNFTASTEAQVKGLSTQGGNVGRKMKSLESQLEKQQKDLSEDHSSLLLHVKQFVSDLRSL SCQMAALQGNGSERTCCPVNWVEHERSCYWFSRSGKAWADADNYCRLEDAHLVVVTSWEEQKFVQHHIGPV NTWMGLHDQNGPWKWVDGTDYETGFKNWRPEQPDDWYGHGLGGGEDCAHFTDDGRWNDDVCQRPYRWV CETELDKASQEPPLL | pSLX235a | 7 |
| huASGR1(CBD) | 154-291 | TCE | CPVNWVEHERSCYWFSRSGKAWADADNYCRLEDAHLVVVTSWEEQKFVQHHIGPVNTWMGLHDQNGPWKW VDGTDYETGFKNWRPEQPDDWYGHGLGGGEDCAHFTDDGRWNDDVCQRPYRWVCETELDKASQEPPLL | pSLX235a | 8 |

# FIGURE 48

| | | | | | |
|---|---|---|---|---|---|
| huASGR2(ECD) | 61-287 | TCE | QSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVA | pSLX235a | 9 |
| huASGR2(CBD) | 153-287 | TCE | CPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVA | pSLX235a | 10 |
| **Structural Work** | | | | | |
| **Name** | **Sequence Range** | **Tags** | **Amino Acid Sequence** | **Vector** | |
| M::huASGR1(154-281) | 154-281 | None | MCPVNWVEHERSCYWFSRSGKAWADADNYCRLEDAHLVVVTSWEEQKFVQHHIGPVNTWMGLHDQNGPWKWVDGTDYETGFKNWRPEQPDDWYGHGLGGGEDCAHFTDDGRWNDDVCQRPYRWVCETEL | pET21a | 11 |
| M::huASGR1(148-291) | 148-291 | None | MGSERTCCPVNWVEHERSCYWFSRSGKAWADADNYCRLEDAHLVVVTSWEEQKFVQHHIGPVNTWMGLHDQNGPWKWVDGTDYETGFKNWRPEQPDDWYGHGLGGGEDCAHFTDDGRWNDDVCQRPYRWVCETELDKASQEPPLL | pET21a | 12 |
| MA::huASGR1(148-291) | 148-291 | None | MAGSERTCCPVNWVEHERSCYWFSRSGKAWADADNYCRLEDAHLVVVTSWEEQKFVQHHIGPVNTWMGLHDQNGPWKWVDGTDYETGFKNWRPEQPDDWYGHGLGGGEDCAHFTDDGRWNDDVCQRPYRWVCETELDKASQEPPLL | pET21a | 13 |
| MA::huASGR1(154-281) | 154-281 | None | MACPVNWVEHERSCYWFSRSGKAWADADNYCRLEDAHLVVVTSWEEQKFVQHHIGPVNTWMGLHDQNGPWKWVDGTDYETGFKNWRPEQPDDWYGHGLGGGEDCAHFTDDGRWNDDVCQRPYRWVCETEL | pET21a | 14 |
| MA::huASGR1(60-153) | 60-153 | None | MAGSQNSQLQEELRGLRETFSNFTASTEAQVKGLSTQGGNVGRKMKSLESQLEKQQKDLSEDHSSLLLHVKQFVSDLRSLSCQMAALQGNGSERTC | pET21a | 15 |
| MA::huASGR1(60-291) | 60-291 | None | MAGSQNSQLQEELRGLRETFSNFTASTEAQVKGLSTQGGNVGRKMKSLESQLEKQQKDLSEDHSSLLLHVKQFVSDLRSLSCQMAALQGNGSERTCCPVNWVEHERSCYWFSRSGKAWADADNYCRLEDAHLVVVTSWEEQKFVQHHIGPVNTWMGLHDQNGPWKWVDGTDYETGFKNWRPEQPDDWYGHGLGGGEDCAHFTDDGRWNDDVCQRPYRWVCETELDKASQEPPLL | pET21a | 16 |
| MA::huASGR1(62-153) | 62-153 | None | MAQNSQLQEELRGLRETFSNFTASTEAQVKGLSTQGGNVGRKMKSLESQLEKQQKDLSEDHSSLLLHVKQFVSDLRSLSCQMAALQGNGSERTC | pET21a | 17 |
| MA::huASGR1(58-153) | 58-153 | None | MAVIGSQNSQLQEELRGLRETFSNFTASTEAQVKGLSTQGGNVGRKMKSLESQLEKQQKDLSEDHSSLLLHVKQFVSDLRSLSCQMAALQGNGSERTC | pET21a | 18 |
| MA::huASGR1(58-143) | 58-143 | None | MAVIGSQNSQLQEELRGLRETFSNFTASTEAQVKGLSTQGGNVGRKMKSLESQLEKQQKDLSEDHSSLLLHVKQFVSDLRSLSCQMAA | pET21a | 19 |
| MA::huASGR1(62-143) | 62-143 | None | MAQNSQLQEELRGLRETFSNFTASTEAQVKGLSTQGGNVGRKMKSLESQLEKQQKDLSEDHSSLLLHVKQFVSDLRSLSCQMAA | pET21a | 20 |
| MA::huASGR1(60-143) | 60-143 | None | MAGSQNSQLQEELRGLRETFSNFTASTEAQVKGLSTQGGNVGRKMKSLESQLEKQQKDLSEDHSSLLLHVKQFVSDLRSLSCQMAA | pET21a | 21 |

**FIGURE 48**

| | | | | | |
|---|---|---|---|---|---|
| MA::huASGR1(60-282) | 60-282 | None | MAGSQNSQLQEELRGLRETFSNFTASTEAQVKGLSTQGGNVGRKMKSLESQLEKQQKDLSEDHSSLLLHVKQFVSDLRSLSCQMAALQGNGSERTCCPVNWVEHERSCYWFSRSGKAWADADNYCRLEDAHLVVVTSWEEQKFVQHHIGPVNTWMGLHDQNGPWKWVDGTDYETGFKNWRPEQPDDWYGHGLGGGEDCAHFTDDGRWNDDVCQRPYRWVCETELD | pET21a | 22 |
| MA::huASGR1(62-291) | 62-291 | None | MAQNSQLQEELRGLRETFSNFTASTEAQVKGLSTQGGNVGRKMKSLESQLEKQQKDLSEDHSSLLLHVKQFVSDLRSLSCQMAALQGNGSERTCCPVNWVEHERSCYWFSRSGKAWADADNYCRLEDAHLVVVTSWEEQKFVQHHIGPVNTWMGLHDQNGPWKWVDGTDYETGFKNWRPEQPDDWYGHGLGGGEDCAHFTDDGRWNDDVCQRPYRWVCETELDKASQEPPLL | pET21a | 23 |
| MA::huASGR1(61-291) | 62-291 | None | MASQNSQLQEELRGLRETFSNFTASTEAQVKGLSTQGGNVGRKMKSLESQLEKQQKDLSEDHSSLLLHVKQFVSDLRSLSCQMAALQGNGSERTCCPVNWVEHERSCYWFSRSGKAWADADNYCRLEDAHLVVVTSWEEQKFVQHHIGPVNTWMGLHDQNGPWKWVDGTDYETGFKNWRPEQPDDWYGHGLGGGEDCAHFTDDGRWNDDVCQRPYRWVCETELDKASQEPPLL | pET21a | 24 |
| MA::huASGR1(58-291) | 58-291 | None | MAVIGSQNSQLQEELRGLRETFSNFTASTEAQVKGLSTQGGNVGRKMKSLESQLEKQQKDLSEDHSSLLLHVKQFVSDLRSLSCQMAALQGNGSERTCCPVNWVEHERSCYWFSRSGKAWADADNYCRLEDAHLVVVTSWEEQKFVQHHIGPVNTWMGLHDQNGPWKWVDGTDYETGFKNWRPEQPDDWYGHGLGGGEDCAHFTDDGRWNDDVCQRPYRWVCETELDKASQEPPLL | pET21a | 25 |
| MA::huASGR1(63-291) | 63-291 | None | MANSQLQEELRGLRETFSNFTASTEAQVKGLSTQGGNVGRKMKSLESQLEKQQKDLSEDHSSLLLHVKQFVSDLRSLSCQMAALQGNGSERTCCPVNWVEHERSCYWFSRSGKAWADADNYCRLEDAHLVVVTSWEEQKFVQHHIGPVNTWMGLHDQNGPWKWVDGTDYETGFKNWRPEQPDDWYGHGLGGGEDCAHFTDDGRWNDDVCQRPYRWVCETELDKASQEPPLL | pET21a | 26 |
| MA::huASGR2(59-287) | 59-287 | None | MAGSQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVA | pET21a | 27 |
| MA::huASGR2(57-287) | 57-287 | None | MAVTGSQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVA | pET21a | 28 |
| MA::huASGR2(60-287) | 60-287 | None | MASQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVA | pET21a | 29 |
| MA::huASGR2(61-287) | 61-287 | None | MAQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVA | pET21a | 30 |
| MA::huASGR2(62-287) | 62-287 | None | MASAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVA | pET21a | 31 |

# FIGURE 48

| Complex Formation Assays | | | | | |
|---|---|---|---|---|---|
| Name | Sequence Range | Tags | Amino Acid Sequence | Vector | |
| huASGR1::GS::SNAP26f | 1-291 | SNAP26f | MTKEYQDLQHLDNEESDHHQLRKGPPPPQPLLQRLCSGPRLLLLSLGLSLLLLVVVCVIGSQNSQLQEELRGLRE<br>TFSNFTASTEAQVKGLSTQGGNVGRKMKSLESQLEKQQKDLSEDHSSLLLHVKQFVSDLRSLSCQMAALQGNG<br>SERTCCPVNWVEHERSCYWFSRSGKAWADADNYCRLEDAHLVVVTSWEEQKFVQHHIGPVNTWMGLHDQNG<br>PWKWVDGTDYETGFKNWRPEQPDDWYGHGLGGGEDCAHFTDDGRWNDDVCQRPYRWVCETELDKASQEP<br>PLLGSMDKDCEMKRTTLDSPLGKLELSGCEQGLHRIIFLGKGTSAADAVEVPAPAAVLGGPEPLMQATAWLNAYF<br>HQPEAIEEFPVPALHHPVFQQESFTRQVLWKLLKVVKFGEVISYSHLAALAGNPAATAAVKTALSGNPVPILIPCHR<br>VVQGDLDVGGYEGGLAVKEWLLAHEGHRLGKPGLG | pIRES_puro3.1 | 32 |
| huASGR1::GS::(G4S)3::SN<br>AP26f | 1-291 | SNAP26f | MTKEYQDLQHLDNEESDHHQLRKGPPPPQPLLQRLCSGPRLLLLSLGLSLLLLVVVCVIGSQNSQLQEELRGLRE<br>TFSNFTASTEAQVKGLSTQGGNVGRKMKSLESQLEKQQKDLSEDHSSLLLHVKQFVSDLRSLSCQMAALQGNG<br>SERTCCPVNWVEHERSCYWFSRSGKAWADADNYCRLEDAHLVVVTSWEEQKFVQHHIGPVNTWMGLHDQNG<br>PWKWVDGTDYETGFKNWRPEQPDDWYGHGLGGGEDCAHFTDDGRWNDDVCQRPYRWVCETELDKASQEP<br>PLLGSGGGGSGGGGSGGGGSMDKDCEMKRTTLDSPLGKLELSGCEQGLHRIIFLGKGTSAADAVEVPAPAAVL<br>GGPEPLMQATAWLNAYFHQPEAIEEFPVPALHHPVFQQESFTRQVLWKLLKVVKFGEVISYSHLAALAGNPAATA<br>AVKTALSGNPVPILIPCHRVVQGDLDVGGYEGGLAVKEWLLAHEGHRLGKPGLG | pIRES_puro3.1<br>, pJif1 | 33 |
| huASGR1::GS::CLIP | 1-291 | CLIP | MTKEYQDLQHLDNEESDHHQLRKGPPPPQPLLQRLCSGPRLLLLSLGLSLLLLVVVCVIGSQNSQLQEELRGLRE<br>TFSNFTASTEAQVKGLSTQGGNVGRKMKSLESQLEKQQKDLSEDHSSLLLHVKQFVSDLRSLSCQMAALQGNG<br>SERTCCPVNWVEHERSCYWFSRSGKAWADADNYCRLEDAHLVVVTSWEEQKFVQHHIGPVNTWMGLHDQNG<br>PWKWVDGTDYETGFKNWRPEQPDDWYGHGLGGGEDCAHFTDDGRWNDDVCQRPYRWVCETELDKASQEP<br>PLLGSMDKDCEMKRTTLDSPLGKLELSGCEQGLHRIIFLGKGTSAADAVEVPAPAAVLGGPEPLIQATAWLNAYF<br>HQPEAIEEFPVPALHHPVFQQESFTRQVLWKLLKVVKFGEVISESHLAALVGNPAATAAVNTALDGNPVPILIPCH<br>RVVQGDSDVGPYLGGLAVKEWLLAHEGHRLGKPGLG | pIRES_puro3.1<br>, pJif1 | 34 |
| huASGR1::GS::(G4S)3::CL<br>IP | 1-291 | CLIP | MTKEYQDLQHLDNEESDHHQLRKGPPPPQPLLQRLCSGPRLLLLSLGLSLLLLVVVCVIGSQNSQLQEELRGLRE<br>TFSNFTASTEAQVKGLSTQGGNVGRKMKSLESQLEKQQKDLSEDHSSLLLHVKQFVSDLRSLSCQMAALQGNG<br>SERTCCPVNWVEHERSCYWFSRSGKAWADADNYCRLEDAHLVVVTSWEEQKFVQHHIGPVNTWMGLHDQNG<br>PWKWVDGTDYETGFKNWRPEQPDDWYGHGLGGGEDCAHFTDDGRWNDDVCQRPYRWVCETELDKASQEP<br>PLLGSGGGGSGGGGSGGGGSMDKDCEMKRTTLDSPLGKLELSGCEQGLHRIIFLGKGTSAADAVEVPAPAAVL<br>GGPEPLIQATAWLNAYFHQPEAIEEFPVPALHHPVFQQESFTRQVLWKLLKVVKFGEVISESHLAALVGNPAATAA<br>VNTALDGNPVPILIPCHRVVQGDSDVGPYLGGLAVKEWLLAHEGHRLGKPGLG | pIRES_puro3.1 | 35 |
| huASGR2::GS::SNAP26f | 1-287 | SNAP26f | MAKDFQDIQQLSSEENDHPFHQGPPPAQPLAQRLCSMVCFSLLALSFNILLLVVICVTGSQSAQLQAELRSLKEAF<br>SNFSSSTLTEVQAISTHGGSVGDKITSLGAKLEKQQQDLKADHDALLFHLKHFPVDLRFVACQMELLHSNGSQRT<br>CCPVNWVEHQGSCYWFSHSGKAWAEAEKYCQLENAHLVVINSWEEQKFIVQHTNPFNTWIGLTDSDGSWKWV<br>DGTDYRHNYKNWAVTQPDNWHGHELGGSEDCVEVQPDGRWNDDFCLQVYRWVCEKRRNATGEVAGSMDKD<br>CEMKRTTLDSPLGKLELSGCEQGLHRIIFLGKGTSAADAVEVPAPAAVLGGPEPLMQATAWLNAYFHQPEAIEEF<br>PVPALHHPVFQQESFTRQVLWKLLKVVKFGEVISYSHLAALAGNPAATAAVKTALSGNPVPILIPCHRVVQGDLDV<br>GGYEGGLAVKEWLLAHEGHRLGKPGLG | pIRES_puro3.1<br>, pJif1 | 36 |

# FIGURE 48

| | | | | | |
|---|---|---|---|---|---|
| huASGR2::GS::(G4S)3::SNAP26f | 1-287 | SNAP26f | MAKDFQDIQQLSSEENDHPFHQGPPPAQPLAQRLCSMVCFSLLALSFNILLLVVICVTGSQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVAGSGGGGSGGGGSGGGGSMDKDCEMKRTTLDSPLGKLELSGCEQGLHRIIFLGKGTSAADAVEVPAPAAVLGGPEPLMQATAWLNAYFHQPEAIEEFPVPALHHPVFQQESFTRQVLWKLLKVVKFGEVISYSHLAALAGNPAATAAVKTALSGNPVPILIPCHRVVQGDLDVGGYEGGLAVKEWLLAHEGHRLGKPGLG | pIRES_puro3.1 | 37 |
| huASGR2::GS::CLIP | 1-287 | CLIP | MAKDFQDIQQLSSEENDHPFHQGPPPAQPLAQRLCSMVCFSLLALSFNILLLVVICVTGSQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVAGSMDKDCEMKRTTLDSPLGKLELSGCEQGLHRIIFLGKGTSAADAVEVPAPAAVLGGPEPLIQATAWLNAYFHQPEAIEEFPVPALHHPVFQQESFTRQVLWKLLKVVKFGEVISESHLAALVGNPAATAAVNTALDGNPVPILIPCHRVVQGDSDVGPYLGGLAVKEWLLAHEGHRLGKPGLG | pIRES_puro3.1, pJif1 | 38 |
| huASGR2::GS::(G4S)3::CLIP | 1-287 | CLIP | MAKDFQDIQQLSSEENDHPFHQGPPPAQPLAQRLCSMVCFSLLALSFNILLLVVICVTGSQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVAGSGGGGSGGGGSGGGGSMDKDCEMKRTTLDSPLGKLELSGCEQGLHRIIFLGKGTSAADAVEVPAPAAVLGGPEPLIQATAWLNAYFHQPEAIEEFPVPALHHPVFQQESFTRQVLWKLLKVVKFGEVISESHLAALVGNPAATAAVNTALDGNPVPILIPCHRVVQGDSDVGPYLGGLAVKEWLLAHEGHRLGKPGLG | pIRES_puro3.1 | 39 |
| huASGR2(v4)::GS::SNAP26f | 1-306 | SNAP26f | MAKDFQDIQQLSSEENDHPFHQGEGPGTRRLNPRRGNPFLKGPPPAQPLAQRLCSMVCFSLLALSFNILLLVVICVTGSQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVAGSMDKDCEMKRTTLDSPLGKLELSGCEQGLHRIIFLGKGTSAADAVEVPAPAAVLGGPEPLMQATAWLNAYFHQPEAIEEFPVPALHHPVFQQESFTRQVLWKLLKVVKFGEVISYSHLAALAGNPAATAAVKTALSGNPVPILIPCHRVVQGDLDVGGYEGGLAVKEWLLAHEGHRLGKPGLG | pIRES_puro3.1, pJif1 | 40 |
| huASGR2(v4)::GS::(G4S)3::SNAP26f | 1-306 | SNAP26f | MAKDFQDIQQLSSEENDHPFHQGEGPGTRRLNPRRGNPFLKGPPPAQPLAQRLCSMVCFSLLALSFNILLLVVICVTGSQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVAGSGGGGSGGGGSGGGGSMDKDCEMKRTTLDSPLGKLELSGCEQGLHRIIFLGKGTSAADAVEVPAPAAVLGGPEPLMQATAWLNAYFHQPEAIEEFPVPALHHPVFQQESFTRQVLWKLLKVVKFGEVISYSHLAALAGNPAATAAVKTALSGNPVPILIPCHRVVQGDLDVGGYEGGLAVKEWLLAHEGHRLGKPGLG | pIRES_puro3.1 | 41 |
| huASGR2(v4)::GS::CLIP | 1-306 | CLIP | MAKDFQDIQQLSSEENDHPFHQGEGPGTRRLNPRRGNPFLKGPPPAQPLAQRLCSMVCFSLLALSFNILLLVVICVTGSQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVAGSMDKDCEMKRTTLDSPLGKLELSGCEQGLHRIIFLGKGTSAADAVEVPAPAAVLGGPEPLIQATAWLNAYFHQPEAIEEFPVPALHHPVFQQESFTRQVLWKLLKVVKFGEVISESHLAALVGNPAATAAVNTALDGNPVPILIPCHRVVQGDSDVGPYLGGLAVKEWLLAHEGHRLGKPGLG | pIRES_puro3.1 | 42 |

## FIGURE 48

| | | | | | |
|---|---|---|---|---|---|
| huASGR2(v4)::GS::(G4S)3::CLIP | 1-306 | CLIP | MAKDFQDIQQLSSEENDHPFHQGEGPGTRRLNPRRGNPFLKGPPPAQPLAQRLCSMVCFSLLALSFNILLLVVIC VTGSQSAQLQAELRSLKEAFSNFSSSTLTEVQAISTHGGSVGDKITSLGAKLEKQQQDLKADHDALLFHLKHFPVD LRFVACQMELLHSNGSQRTCCPVNWVEHQGSCYWFSHSGKAWAEAEKYCQLENAHLVVINSWEEQKFIVQHT NPFNTWIGLTDSDGSWKWVDGTDYRHNYKNWAVTQPDNWHGHELGGSEDCVEVQPDGRWNDDFCLQVYRW VCEKRRNATGEVAGSGGGGSGGGGSGGGGSMDKDCEMKRTTLDSPLGKLELSGCEQGLHRIIFLGKGTSAADA VEVPAPAAVLGGPEPLIQATAWLNAYFHQPEAIEEFPVPALHHPVFQQESFTRQVLWKLLKVVKFGEVISESHLAA LVGNPAATAAVNTALDGNPVPILIPCHRVVQGDSDVGPYLGGLAVKEWLLAHEGHRLGKPGLG | pIRES_puro3.1 | 43 |
| **Mammalian Expression** | | | | | |
| huASGR1 | 1-291 | None | MTKEYQDLQHLDNEESDHHQLRKGPPPPQPLLQRLCSGPRLLLLSLGLSLLLLVVVCVIGSQNSQLQEELRGLRE TFSNFTASTEAQVKGLSTQGGNVGRKMKSLESQLEKQQKDLSEDHSSLLLHVKQFVSDLRSLSCQMAALQGNG SERTCCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPYRWVCETELDKASQEP PLL | pTT5, pSLX235a, pJiF1 | 44 |
| huASGR1(Y273C) | 1-291 | None | MTKEYQDLQHLDNEESDHHQLRKGPPPPQPLLQRLCSGPRLLLLSLGLSLLLLVVVCVIGSQNSQLQEELRGLRE TFSNFTASTEAQVKGLSTQGGNVGRKMKSLESQLEKQQKDLSEDHSSLLLHVKQFVSDLRSLSCQMAALQGNG SERTCCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPCRWVCETELDKASQEP PLL | pTT5, pSLX235a, pJiF1 | 45 |
| huASGR1::Flag | 1-291 | Flag | MTKEYQDLQHLDNEESDHHQLRKGPPPPQPLLQRLCSGPRLLLLSLGLSLLLLVVVCVIGSQNSQLQEELRGLRE TFSNFTASTEAQVKGLSTQGGNVGRKMKSLESQLEKQQKDLSEDHSSLLLHVKQFVSDLRSLSCQMAALQGNG SERTCCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPYRWVCETELDKASQEP PLLDYKDDDDK | pTT5, pSLX235a | 46 |
| huASGR1(Y273C)::Flag | 1-291 | Flag | MTKEYQDLQHLDNEESDHHQLRKGPPPPQPLLQRLCSGPRLLLLSLGLSLLLLVVVCVIGSQNSQLQEELRGLRE TFSNFTASTEAQVKGLSTQGGNVGRKMKSLESQLEKQQKDLSEDHSSLLLHVKQFVSDLRSLSCQMAALQGNG SERTCCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPCRWVCETELDKASQEP PLLDYKDDDDK | pTT5, pSLX235a | 47 |
| cyASGR1 | 1-291 | None | MTKEYQDLQHLDNEESDHHQLGKGPPPPQSLLRRLCSGPRLLLLSLGLSLLLLVVVCVIGSQNAQLQRELRGLRE TLSNFTASTEAQVKGLSTQGGNVGRKMKSLESQLEKQQKDLSEDHSSLLLHVKQFVSDLRSLSCQMAALQGNG SERACCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPYRWVCETELDKASQEP PLL | pTT5, pSLX235a | 48 |
| cyASGR1(Y273C) | 1-291 | None | MTKEYQDLQHLDNEESDHHQLGKGPPPPQSLLRRLCSGPRLLLLSLGLSLLLLVVVCVIGSQNAQLQRELRGLRE TLSNFTASTEAQVKGLSTQGGNVGRKMKSLESQLEKQQKDLSEDHSSLLLHVKQFVSDLRSLSCQMAALQGNG SERACCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPCRWVCETELDKASQEP PLL | pTT5, pSLX235a | 49 |
| cyASGR1::Flag | 1-291 | Flag | MTKEYQDLQHLDNEESDHHQLGKGPPPPQSLLRRLCSGPRLLLLSLGLSLLLLVVVCVIGSQNAQLQRELRGLRE TLSNFTASTEAQVKGLSTQGGNVGRKMKSLESQLEKQQKDLSEDHSSLLLHVKQFVSDLRSLSCQMAALQGNG SERACCPVNWVEHERSCYWFSRSGKAWADADNYCRLEDAHLVVVTSWEEQKFVQHHIGPVNTWMGLHDQNG PWKWVDGTDYETGFKNWRPEQPDDWYGHGLGGGEDCAHFTDDGRWNDDVCQRPYRWVCETELDKASQEP PLLDYKDDDDK | pTT5, pSLX235a | 50 |

**FIGURE 48**

| | | | | | |
|---|---|---|---|---|---|
| cyASGR1(Y273C)::Flag | 1-291 | Flag | MTKEYQDLQHLDNEESDHHQLGKGPPPPQSLLRRLCSGPRLLLLSLGLSLLLLVVVCVIGSQNAQLQRELRGLRETLSNFTASTEAQVKGLSTQGGNVGRKMKSLESQLEKQQKDLSEDHSSLLLHVKQFVSDLRSLSCQMAALQGNGSERACCPVNWVEHERSCYWFSRSGKAWADADNYCRLEDAHLVVVTSWEEQKFVQHHIGPVNTWMGLHDQNGPWKWVDGTDYETGFKNWRPEQPDDWYGHGLGGGEDCAHFTDDGRWNDDVCQRPCRWVCETELDKASQEPPLLDYKDDDDK | pTT5, pSLX235a | 51 |
| muASGR1 | 1-284 | None | MTKDYQDFQHLDNDNDHHQLRRGPPPTPRLLQRLCSGSRLLLLSSSLSILLLVVVCVITSQNSQLREDLLALRQNFSNLTVSTEDQVKALSTQGSSVGRKMKLVESKLEKQQKDLTEDHSSLLLHVKQLVSDVRSLSCQMAAFRGNGSERTCCPINWVEYEGSCYWFSSSVRPWTEADKYCQLENAHLVVVTSRDEQNFLQRHMGPLNTWIGLTDQNGPWKWVDGTDYETGFQNWRPEQPDNWYGHGLGGGEDCAHFTTDGRWNDDVCRRPYRWVCETKLDKAN | pTT5, pJiF1 | 52 |
| muASGR1(Y272C) | 1-284 | None | MTKDYQDFQHLDNDNDHHQLRRGPPPTPRLLQRLCSGSRLLLLSSSLSILLLVVVCVITSQNSQLREDLLALRQNFSNLTVSTEDQVKALSTQGSSVGRKMKLVESKLEKQQKDLTEDHSSLLLHVKQLVSDVRSLSCQMAAFRGNGSERTCCPINWVEYEGSCYWFSSSVRPWTEADKYCQLENAHLVVVTSRDEQNFLQRHMGPLNTWIGLTDQNGPWKWVDGTDYETGFQNWRPEQPDNWYGHGLGGGEDCAHFTTDGRWNDDVCRRPCRWVCETKLDKAN | pTT5, pSLX235a, pJiF1 | 53 |
| ratASGR1 | 1-284 | None | MTKDYQDFQHLDNENDHHQLQRGPPPAPRLLQRLCSGFRLFLLSLGLSILLLVVVCVITSQNSQLREDLRVLRQNFSNFTVSTEDQVKALTTQGERVGRKMKLVESQLEKHQEDLREDHSRLLLHVKQLVSDVRSLSCQMAALRGNGSERICCPINWVEYEGSCYWFSSSVKPWTEADKYCQLENAHLVVVTSWEEQRFVQQHMGPLNTWIGLTDQNGPWKWVDGTDYETGFKNWRPGQPDDWYGHGLGGGEDCAHFTTDGHWNDDVCRRPYRWVCETELGKAN | pTT5, pSLX235a | 54 |
| ratASGR1(Y272C) | 1-284 | None | MTKDYQDFQHLDNENDHHQLQRGPPPAPRLLQRLCSGFRLFLLSLGLSILLLVVVCVITSQNSQLREDLRVLRQNFSNFTVSTEDQVKALTTQGERVGRKMKLVESQLEKHQEDLREDHSRLLLHVKQLVSDVRSLSCQMAALRGNGSERICCPINWVEYEGSCYWFSSSVKPWTEADKYCQLENAHLVVVTSWEEQRFVQQHMGPLNTWIGLTDQNGPWKWVDGTDYETGFKNWRPGQPDDWYGHGLGGGEDCAHFTTDGHWNDDVCRRPCRWVCETELGKAN | pTT5, pSLX235a | 55 |
| muASGR1::Flag | 1-284 | Flag | MTKDYQDFQHLDNDNDHHQLRRGPPPTPRLLQRLCSGSRLLLLSSSLSILLLVVVCVITSQNSQLREDLLALRQNFSNLTVSTEDQVKALSTQGSSVGRKMKLVESKLEKQQKDLTEDHSSLLLHVKQLVSDVRSLSCQMAAFRGNGSERTCCPINWVEYEGSCYWFSSSVRPWTEADKYCQLENAHLVVVTSRDEQNFLQRHMGPLNTWIGLTDQNGPWKWVDGTDYETGFQNWRPEQPDNWYGHGLGGGEDCAHFTTDGRWNDDVCRRPYRWVCETKLDKANDYKDDDDK | pTT5, pSLX235a | 56 |
| muASGR1(Y272C)::Flag | 1-284 | Flag | MTKDYQDFQHLDNDNDHHQLRRGPPPTPRLLQRLCSGSRLLLLSSSLSILLLVVVCVITSQNSQLREDLLALRQNFSNLTVSTEDQVKALSTQGSSVGRKMKLVESKLEKQQKDLTEDHSSLLLHVKQLVSDVRSLSCQMAAFRGNGSERTCCPINWVEYEGSCYWFSSSVRPWTEADKYCQLENAHLVVVTSRDEQNFLQRHMGPLNTWIGLTDQNGPWKWVDGTDYETGFQNWRPEQPDNWYGHGLGGGEDCAHFTTDGRWNDDVCRRPCRWVCETKLDKANDYKDDDDK | pTT5, pSLX235a | 57 |
| ratASGR1::Flag | 1-284 | Flag | MTKDYQDFQHLDNENDHHQLQRGPPPAPRLLQRLCSGFRLFLLSLGLSILLLVVVCVITSQNSQLREDLRVLRQNFSNFTVSTEDQVKALTTQGERVGRKMKLVESQLEKHQEDLREDHSRLLLHVKQLVSDVRSLSCQMAALRGNGSERICCPINWVEYEGSCYWFSSSVKPWTEADKYCQLENAHLVVVTSWEEQRFVQQHMGPLNTWIGLTDQNGPWKWVDGTDYETGFKNWRPGQPDDWYGHGLGGGEDCAHFTTDGHWNDDVCRRPYRWVCETELGKANDYKDDDDK | pTT5, pSLX235a | 58 |

# FIGURE 48

| | | | | | |
|---|---|---|---|---|---|
| ratASGR1(Y272C)::Flag | 1-284 | Flag | MTKDYQDFQHLDNENDHHQLQRGPPPAPRLLQRLCSGFRLFLLSLGLSILLLVVVCVITSQNSQLREDLRVLRQNFSNFTVSTEDQVKALTTQGERVGRKMKLVESQLEKHQEDLREDHSRLLLHVKQLVSDVRSLSCQMAALRGNGSERICCPINWVEYEGSCYWFSSSVKPWTEADKYCQLENAHLVVVTSWEEQRFVQQHMGPLNTWIGLTDQNGPWKWVDGTDYETGFKNWRPGQPDDWYGHGLGGGEDCAHFTTDGHWNDDVCRRPCRWVCETELGKANDYKDDDDK | pTT5, pSLX235a | 59 |
| huASGR2 | 1-306 | None | MAKDFQDIQQLSSEENDHPFHQGPPPAQPLAQRLCSMVCFSLLALSFNILLLVVICVTGSQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVA | pTT5, pSLX235a, pJiF1 | 60 |
| muASGR2 | 1-301 | None | MEKDCQDIQQLDSEENDHQLSGDDEHGSHVQDPRIENPHWKGQPLSRPFPQRLCSTFRLSLLALAFNILLLVVICVVSSQSIQLQEEFRTLKETFSNFSSSTLMEFGALDTLGGSTNAILTSWLAQLEEKQQQLKADHSTLLFHLKHFPMDLRTLTCQLAYFQSNGTECCPVNWVEFGGSCYWFSRDGLTWAEADQYCQLENAHLLVINSREEQDFVVKHRSQFHIWIGLTDRDGSWKWVDGTDYRSNYRNWAFTQPDNWQGHEQGGGEDCAEILSDGHWNDNFCQQVNRWVCEKRRNITH | pTT5, pJiF1 | 61 |
| ratASGR2 | 1-301 | None | MEKDFQDIQQLDSEENDHQLIGDEEQGSHVQNLRTENPRWGGQPPSRPFPQRLCSKFRLSLLALAFNILLLVVICVVSSQSMQLQKEFWTLKETLSNFSTTTLMEFKALDSHGGSRNDNLTSWETILEKKQKDIKADHSTLLFHLKHFPLDLRTLTCQLAFFLSNGTECCPVNWVEFGGSCYWFSRDGLTWAEADQYCQMENAHLLVINSREEQEFVVKHRGAFHIWIGLTDKDGSWKWVDGTEYRSNFKNWAFTQPDNWQGHEEGGSEDCAEILSDGLWNDNFCQQVNRWACERKRDITY | pTT5, pSLX235a | 62 |
| huASGR2::6xHis | 1-306 | 6xHis | MAKDFQDIQQLSSEENDHPFHQGPPPAQPLAQRLCSMVCFSLLALSFNILLLVVICVTGSQSAQLQAELRSLKEAFSNFSSSTLTEVQAISTHGGSVGDKITSLGAKLEKQQQDLKADHDALLFHLKHFPVDLRFVACQMELLHSNGSQRTCCPVNWVEHQGSCYWFSHSGKAWAEAEKYCQLENAHLVVINSWEEQKFIVQHTNPFNTWIGLTDSDGSWKWVDGTDYRHNYKNWAVTQPDNWHGHELGGSEDCVEVQPDGRWNDDFCLQVYRWVCEKRRNATGEVAHHHHHH | pTT5, pSLX235a | 63 |
| muASGR2::6xHis | 1-301 | 6xHis | MEKDCQDIQQLDSEENDHQLSGDDEHGSHVQDPRIENPHWKGQPLSRPFPQRLCSTFRLSLLALAFNILLLVVICVVSSQSIQLQEEFRTLKETFSNFSSSTLMEFGALDTLGGSTNAILTSWLAQLEEKQQQLKADHSTLLFHLKHFPMDLRTLTCQLAYFQSNGTECCPVNWVEFGGSCYWFSRDGLTWAEADQYCQLENAHLLVINSREEQDFVVKHRSQFHIWIGLTDRDGSWKWVDGTDYRSNYRNWAFTQPDNWQGHEQGGGEDCAEILSDGHWNDNFCQQVNRWVCEKRRNITHHHHHHH | pTT5, pSLX235a | 64 |
| ratA::6xHis | 1-301 | 6xHis | MEKDFQDIQQLDSEENDHQLIGDEEQGSHVQNLRTENPRWGGQPPSRPFPQRLCSKFRLSLLALAFNILLLVVICVVSSQSMQLQKEFWTLKETLSNFSTTTLMEFKALDSHGGSRNDNLTSWETILEKKQKDIKADHSTLLFHLKHFPLDLRTLTCQLAFFLSNGTECCPVNWVEFGGSCYWFSRDGLTWAEADQYCQMENAHLLVINSREEQEFVVKHRGAFHIWIGLTDKDGSWKWVDGTEYRSNFKNWAFTQPDNWQGHEEGGSEDCAEILSDGLWNDNFCQQVNRWACERKRDITYHHHHHH | pTT5, pSLX235a | 65 |
| dogASGR2::6xHis | 1-302 | 6xHis | MAKDCQDIQQLDSEDSDQQLGRGEGPGPRGHGPRREDRFCRRLPPHQPLLLQRLCSGYRLNLLVLGFNVLMLVAICVIGSQRAQLEEELRILKENFSHFSSGVLMELGVLLSDGGGASSQLTSLEAKLEKQQRDVKADHATLLLHLKHFPSDLRILTCQVAFFQSNGTDCCPVNWLEYEGSCYWFSRSGKTWEEAEKYCQLESAHLVVVNSREEQKFILQHTNPFDTWIGLTDSDGSWRWVDGTDYQQSYKNWAATQPDDWQGHEVGGGEDCAEVRANGRWNDNFCKQVQRWVCEMRRNVTGHHHHHH | pTT5, pSLX235a | 66 |

## FIGURE 48

| | | | | | |
|---|---|---|---|---|---|
| dogASGR2 | 1-302 | None | MAKDCQDIQQLDSEDSDQQLGRGEGPGPRGHGPRREDRFCRRLPPHQPLLLQRLCSGYRLNLLVLGFNVLMLVAICVIGSQRAQLEEELRILKENFSHFSSGVLMELGVLLSDGGGASSQLTSLEAKLEKQQRDVKADHATLLLHLKHFPSDLRILTCQVAFFQSNGTDCCPVNWLEYEGSCYWFSRSGKTWEEAEKYCQLESAHLVVVNSREEQKFILQHTNPFDTWIGLTDSDGSWRWVDGTDYQQSYKNWAATQPDDWQGHEVGGGEDCAEVRANGRWNDNFCKQVQRWVCEMRRNVTG | pTT5, pSLX235a | 67 |
| cyASGR2 | 1-306 | None | MAKDFQDIQQLSSEENDHPFHRGEGPGPRGLNLRRGNPSLKGPPPAQPLAQRLCSMVRFSLLALSFNILLLVAICVIGSQSAQLQAELWSLKEAFSNFSSSTLMEVHALGTHGGSVGDKITSLGDKLEKQQQDLKADHDILLLHLKHFPVDLRFVACQMELLHSNGSQRTCCPVNWVEHQGSCYWFSRSGKAWAEAEKYCQLENSHLVVINSWEEQKFIVQHTNLFNTWIGLTDSDGSWKWVDGTDYRHNYKNWAVTQPDDWHGHELGGSEDCVEVRPDGRWNDDFCLQVHRWVCEKRRNATGEAA | pTT5, pSLX235a | 68 |
| cyASGR2(L225P)::6xHis | 1-306 | 6xHis | MAKDFQDIQQLSSEENDHPFHRGEGPGPRGLNLRRGNPSLKGPPPAQPLAQRLCSMVRFSLLALSFNILLLVAICVIGSQSAQLQAELWSLKEAFSNFSSSTLMEVHALGTHGGSVGDKITSLGDKLEKQQQDLKADHDILLLHLKHFPVDLRFVACQMELLHSNGSQRTCCPVNWVEHQGSCYWFSRSGKAWAEAEKYCQLENSHLVVINSWEEQKFIVQHTNPFNTWIGLTDSDGSWKWVDGTDYRHNYKNWAVTQPDDWHGHELGGSEDCVEVRPDGRWNDDFCLQVHRWVCEKRRNATGEAAHHHHHH | pTT5, pSLX235a | 69 |
| pigASGR2::6xHis | 1-283 | 6xHis | MARDFQDIQQLDSEENDHQLGRGTPLPQPLVLQRLCSKLRLSLLVLGFNVLMLVAVCVVGSQRTQLQMELQTLKETFSNFSSSLLMEMLTLSTRGGSAGDKVTSLEAKMEKQQQDLKADHATLRLHLKHFPRDVRTLTCRLVFLQSNGTECCPVNWVDYEGSCYWFSRSGKAWAEAEKYCQLENAHLVVINSREEQKFIAQRTNPFQTWIGLTDSDGSWKWVDGTDYGNGYKNWGALQPDDWQGHELGGSEDCVEIQGDGRWNDDFCQQVKRWVCEMKQNITMHHHHHH | pTT5, pSLX235a | 70 |
| cyASGR2(L225P) | 1-306 | None | MAKDFQDIQQLSSEENDHPFHRGEGPGPRGLNLRRGNPSLKGPPPAQPLAQRLCSMVRFSLLALSFNILLLVAICVIGSQSAQLQAELWSLKEAFSNFSSSTLMEVHALGTHGGSVGDKITSLGDKLEKQQQDLKADHDILLLHLKHFPVDLRFVACQMELLHSNGSQRTCCPVNWVEHQGSCYWFSRSGKAWAEAEKYCQLENSHLVVINSWEEQKFIVQHTNPFNTWIGLTDSDGSWKWVDGTDYRHNYKNWAVTQPDDWHGHELGGSEDCVEVRPDGRWNDDFCLQVHRWVCEKRRNATGEAA | pTT5, pSLX235a | 71 |
| pigASGR2 | 1-283 | None | MARDFQDIQQLDSEENDHQLGRGTPLPQPLVLQRLCSKLRLSLLVLGFNVLMLVAVCVVGSQRTQLQMELQTLKETFSNFSSSLLMEMLTLSTRGGSAGDKVTSLEAKMEKQQQDLKADHATLRLHLKHFPRDVRTLTCRLVFLQSNGTECCPVNWVDYEGSCYWFSRSGKAWAEAEKYCQLENAHLVVINSREEQKFIAQRTNPFQTWIGLTDSDGSWKWVDGTDYGNGYKNWGALQPDDWQGHELGGSEDCVEIQGDGRWNDDFCQQVKRWVCEMKQNITM | pTT5, pSLX235a | 72 |
| dogASGR1 | 1-284 | None | MTNDYQDLQHLDNEDNDHHLRQVPPAPQPLLRRLCSGPCLLLLSLGLSVLLLVVVCVIGSQNSKLRGELQALRETFSNFTASTEVEVKALSSQGGNVGRKMKSLESQLEKQQKDLSEDHSDLLLHVKQFVSDLRSLSCQIAALHGNGSTLTCCPVNWLEYEGSCYWFSRSGKSWPEADKYCQLESAHLVVVNSREEQKFIQHHMGPVNTWMGLTDQSGPWKWVDGTDYETGFKNWRPEQPDDWYGHGLGGGEDCAHFTDDGRWNDDVCQRPYRWVCEAARDPAT | pTT5, pSLX235a | 73 |
| pigASGR1 | 1-286 | None | MTKEYQDLQHLDNEENDQQHRKGPPPQPSLLRRLCSGPCLLLISMGLSLLLLVVVCVIGSQNSKLQEELQALRETFSNLTASTDAKVKTLSMQGGNVGRKMKSLESQLEKQQQDLSEDHSSLLLHVKQFVSDLRSLSCQMAVLQGNGSERTCCPVNWVGYEGSCYWFSRSGKPWPEAEKYCQLENAHLVVVGSWEEQKFIQHHVGPVNSWIGLTDQSGPWKWVDGTDYESGFKNWRPEQPDDWYGHGLGGGEDCAHFTEDGGWNDDICQRPYRWVCETQRDRDSGS | pTT5, pSLX235a | 74 |
| dogASGR1::Flag | 1-284 | Flag | MTNDYQDLQHLDNEDNDHHLRQVPPAPQPLLRRLCSGPCLLLLSLGLSVLLLVVVCVIGSQNSKLRGELQALRETFSNFTASTEVEVKALSSQGGNVGRKMKSLESQLEKQQKDLSEDHSDLLLHVKQFVSDLRSLSCQIAALHGNGSTLTCCPVNWLEYEGSCYWFSRSGKSWPEADKYCQLESAHLVVVNSREEQKFIQHHMGPVNTWMGLTDQSGPWKWVDGTDYETGFKNWRPEQPDDWYGHGLGGGEDCAHFTDDGRWNDDVCQRPYRWVCEAARDPATDYKDDDDK | pTT5, pSLX235a | 75 |

**FIGURE 48**

| | | | | | |
|---|---|---|---|---|---|
| pigASGR1::Flag | 1-286 | Flag | MTKEYQDLQHLDNEENDQQHRKGPPPQPSLLRRLCSGPCLLLISMGLSLLLLVVVCVIGSQNSKLQEELQALRETFSNLTASTDAKVKTLSMQGGNVGRKMKSLESQLEKQQQDLSEDHSSLLLHVKQFVSDLRSLSCQMAVLQGNGSERTCCPVNWVGYEGSCYWFSRSGKPWPEAEKYCQLENAHLVVVGSWEEQKFIQHHVGPVNSWIGLTDQSGPWKWVDGTDYESGFKNWRPEQPDDWYGHGLGGGEDCAHFTEDGGWNDDICQRPYRWVCETQRDRDSGSDYKDDDDK | pTT5, pSLX235a | 76 |
| huASGR1(deCODE) | 1-94. | None | MTKEYQDLQHLDNEESDHHQLRKGPPPPQPLLQRLCSGPRLLLLSLGLSLLLLVVVCVIGSQNSQLQEELRGLRETFSNFTASTEAQEAMWEER | pJiF1 | 77 |
| huASGR1(deCODE)::Myc | 1-94. | Myc | MTKEYQDLQHLDNEESDHHQLRKGPPPPQPLLQRLCSGPRLLLLSLGLSLLLLVVVCVIGSQNSQLQEELRGLRETFSNFTASTEAQEAMWEERGQQKLISEEDL | pTT5 | 0 |

FIGURE 49

**Table 2A**
**Standard**
**IgG Antibody**
**VL CDRs**

| iPS# | Ab | Type | CDR1 | CDR2 | CDR3 |
|---|---|---|---|---|---|
| **iPS:451135** | **21-225_64A11** | NA | CGGGCAAGTCGGAGCGTTAGCAGATATTTAAAT | GTTGCATCCCGTTTGCAAAGT | CAACAGAGTGACAGTTTCCCTCTCACT |
| | | | SEQ ID NO:79 | SEQ ID NO:8091 | SEQ ID NO:16103 |
| | | AA | RASRSVSRYLN | VASRLQS | QQSDSFPLT |
| | | | SEQ ID NO:80 | SEQ ID NO:8092 | SEQ ID NO:16104 |
| **iPS:451141** | **21-225_164B11** | NA | AAGTCCAGCCAGAGTCTTTTAAAGAGCTCCAACAATAAGAGCTACTTAGCT | TGGGCATCTTCCCGGGAATCC | CAGCAATATTATAGTATTCCTCCCACT |
| | | | SEQ ID NO:81 | SEQ ID NO:8093 | SEQ ID NO:16105 |
| | | AA | KSSQSLLKSSNNKSYLA | WASSRES | QQYYSIPPT |
| | | | SEQ ID NO:82 | SEQ ID NO:8094 | SEQ ID NO:16106 |
| **iPS:451137** | **21-225_74A7** | NA | AAGTCCAGCCAGAGTGTTTTATTCAGCTCCAACAATTATAACTACTTAGCT | TGGGCATCTACCCGGGAATCC | CAGCAATATCATAGTTCTCCTCCGACG |
| | | | SEQ ID NO:83 | SEQ ID NO:8095 | SEQ ID NO:16107 |
| | | AA | KSSQSVLFSSNNYNYLA | WASTRES | QQYHSSPPT |
| | | | SEQ ID NO:84 | SEQ ID NO:8096 | SEQ ID NO:16108 |
| **iPS:451139** | **21-225_71A6** | NA | AAGTCTAGTCAGAGCCTCCTGCGTAGTGATGGAAAGACCCATTTGTAT | GAAGTTTCCAACCGGTTCTCT | ATGCAAAGTAAACAGCTTCCTCTCACT |
| | | | SEQ ID NO:85 | SEQ ID NO:8097 | SEQ ID NO:16109 |
| | | AA | KSSQSLLRSDGKTHLY | EVSNRFS | MQSKQLPLT |
| | | | SEQ ID NO:86 | SEQ ID NO:8098 | SEQ ID NO:16110 |

FIGURE 49

| | | | | | |
|---|---|---|---|---|---|
| **iPS:451143** | **21-225_66H11** | NA | CCGGCGAGTCAGGGCATTAG CAATTATTTAGCT | GGTGCATTCAATTTGCAC AGT | CAACAGTATAGTTGTTACCC ATTCACT |
| | | | SEQ ID NO:87 | SEQ ID NO:8099 | SEQ ID NO:16111 |
| | | AA | PASQGISNYLA | GAFNLHS | QQYSCYPFT |
| | | | SEQ ID NO:88 | SEQ ID NO:8100 | SEQ ID NO:16112 |
| **iPS:453445** | **21-225_148E10** | NA | TCTGGAGATAAATTGGGTAA TAAATATGTTTGT | CAAGATAGCAAGCGGCC CTCA | CAGGCGTGGGACAGGAACAC TTATGTGGTG |
| | | | SEQ ID NO:89 | SEQ ID NO:8101 | SEQ ID NO:16113 |
| | | AA | SGDKLGNKYVC | QDSKRPS | QAWDRNTYVV |
| | | | SEQ ID NO:90 | SEQ ID NO:8102 | SEQ ID NO:16114 |
| **iPS:453447** | **21-225_65F10** | NA | CGGGGGGGTCAGGGTATTAG CACATGGTTAGCA | GCTGCATCCATTTTGCAA AGT | CAACAGGGTAACATTTTCCC ATTCACT |
| | | | SEQ ID NO:91 | SEQ ID NO:8103 | SEQ ID NO:16115 |
| | | AA | RGGQGISTWLA | AASILQS | QQGNIFPFT |
| | | | SEQ ID NO:92 | SEQ ID NO:8104 | SEQ ID NO:16116 |
| **iPS:453449** | **21-225_208A2** | NA | CGGACAAGTCAGGGCATTAG AAATGATTTAGGC | GCTGCATCCAGTTTGCTT AGT | CTACAGTATAATAGTTACCC TCCCACC |
| | | | SEQ ID NO:93 | SEQ ID NO:8105 | SEQ ID NO:16117 |
| | | AA | RTSQGIRNDLG | AASSLLS | LQYNSYPPT |
| | | | SEQ ID NO:94 | SEQ ID NO:8106 | SEQ ID NO:16118 |
| **iPS:453451** | **21-225_52G11** | NA | CGGGCGAGTCAGGGTATTAG CAAATGGTTAGCC | GCTGCATCCAGTTTGCAA AGT | CAACAGGCTAACAGTTTCCC ATTCACT |
| | | | SEQ ID NO:95 | SEQ ID NO:8107 | SEQ ID NO:16119 |
| | | AA | RASQGISKWLA | AASSLQS | QQANSFPFT |
| | | | SEQ ID NO:96 | SEQ ID NO:8108 | SEQ ID NO:16120 |
| **iPS:453453** | **21-225_53F2** | NA | CGGGCGAGTCAGGGTATTAG CAAGTGGTTAGCC | GCTGCATCCAGTTTGCAA AGT | CAACAGGCTAACAGTTTCCC ATTCACT |
| | | | SEQ ID NO:97 | SEQ ID NO:8109 | SEQ ID NO:16121 |
| | | AA | RASQGISKWLA | AASSLQS | QQANSFPFT |
| | | | SEQ ID NO:98 | SEQ ID NO:8110 | SEQ ID NO:16122 |

# FIGURE 49

| | | | | | |
|---|---|---|---|---|---|
| **iPS:468810** | **21-225_74D5** | NA | AGGGCCAGTCAGAGTGTTAACAGCAACTACTTAGCC | GGTGCATCCAGCAGGGCCACT | CAGCAGTATGAAAGCTCGCCGTGGACG |
| | | | SEQ ID NO:99 | SEQ ID NO:8111 | SEQ ID NO:16123 |
| | | AA | RASQSVNSNYLA | GASSRAT | QQYESSPWT |
| | | | SEQ ID NO:100 | SEQ ID NO:8112 | SEQ ID NO:16124 |
| **iPS:468812** | **21-225_48H4** | NA | CGGGCAAGTCGAGACATTAGAAATGATTTAGGC | GCTGCATCCAGTTTGCAAAGT | CTACAACATTATAGTTACCCGCTCACT |
| | | | SEQ ID NO:101 | SEQ ID NO:8113 | SEQ ID NO:16125 |
| | | AA | RASRDIRNDLG | AASSLQS | LQHYSYPLT |
| | | | SEQ ID NO:102 | SEQ ID NO:8114 | SEQ ID NO:16126 |
| **iPS:468816** | **21-225_52G8** | NA | AAGTCTAGTCAGAGCCTCCTGCATAGTGAAGGAAAGACCTATTTGTAT | GAAGTTTCCAAGCGGCTCTCT | ATGCAAAGTATGCAGCTTCCGATTATC |
| | | | SEQ ID NO:103 | SEQ ID NO:8115 | SEQ ID NO:16127 |
| | | AA | KSSQSLLHSEGKTYLY | EVSKRLS | MQSMQLPII |
| | | | SEQ ID NO:104 | SEQ ID NO:8116 | SEQ ID NO:16128 |
| **iPS:468814** | **21-225_223D11** | NA | CGGGCGAGTCAGGGCATTAGCAATTATTTAGCC | GCTGCATCCACTTTGCAAAGT | CAACAGTATAGTGGTTACCCATTCACT |
| | | | SEQ ID NO:105 | SEQ ID NO:8117 | SEQ ID NO:16129 |
| | | AA | RASQGISNYLA | AASTLQS | QQYSGYPFT |
| | | | SEQ ID NO:106 | SEQ ID NO:8118 | SEQ ID NO:16130 |
| **iPS:468822** | **21-225_147E10** | NA | AAGTCTAGTCAGCGCCTCCTGCATGGTGATGGAAAGACCTATTTGTAT | GAAGTTTCCAACCGGTTCTCT | ATGCAAAGTATACAGGTTCCGTGGACG |
| | | | SEQ ID NO:107 | SEQ ID NO:8119 | SEQ ID NO:16131 |
| | | AA | KSSQRLLHGDGKTYLY | EVSNRFS | MQSIQVPWT |
| | | | SEQ ID NO:108 | SEQ ID NO:8120 | SEQ ID NO:16132 |
| **iPS:468824** | **21-225_73G6** | NA | CGGGCAAGTCAGGGCATTAGAAATGATTTAGGC | GCTGCATCCAGTTTGCAAAGT | CTACAGCATAATAGTTACCCGCTCACT |
| | | | SEQ ID NO:109 | SEQ ID NO:8121 | SEQ ID NO:16133 |

FIGURE 49

| | | | | | |
|---|---|---|---|---|---|
| | | AA | RASQGIRNDLG | AASSLQS | LQHNSYPLT |
| | | | SEQ ID NO:110 | SEQ ID NO:8122 | SEQ ID NO:16134 |
| iPS:468818 | 21-225_190C8 | NA | CGGGCAAGTCAGGGCATTAGAAATGATTTAGGC | GCTGCATCCAGTTTGCAAAGT | CTACAGCATAATGATTACCCGTTCACT |
| | | | SEQ ID NO:111 | SEQ ID NO:8123 | SEQ ID NO:16135 |
| | | AA | RASQGIRNDLG | AASSLQS | LQHNDYPFT |
| | | | SEQ ID NO:112 | SEQ ID NO:8124 | SEQ ID NO:16136 |
| iPS:468826 | 21-225_201C5 | NA | CGGGCAAGTCAGGGCATTAGACATGATTTAGGC | GCTGCATCCAGTTTGCAAAGT | CTACAGCATTATAGTTTCCCTCGGACG |
| | | | SEQ ID NO:113 | SEQ ID NO:8125 | SEQ ID NO:16137 |
| | | AA | RASQGIRHDLG | AASSLQS | LQHYSFPRT |
| | | | SEQ ID NO:114 | SEQ ID NO:8126 | SEQ ID NO:16138 |
| iPS:468828 | 21-225_162A10 | NA | AGGGCCAGTCAGACTGTTAACAGCAACTTAGCC | GGTGCATCCACCAGGGCCACT | CAGCAGTATAATGACTGGCCGTGCAGT |
| | | | SEQ ID NO:115 | SEQ ID NO:8127 | SEQ ID NO:16139 |
| | | AA | RASQTVNSNLA | GASTRAT | QQYNDWPCS |
| | | | SEQ ID NO:116 | SEQ ID NO:8128 | SEQ ID NO:16140 |
| iPS:468830 | 21-225_191G11 | NA | AGGACCAGTCAGAGTGTTTGGATTAGCGTAGCC | GGTGCAGCCACCAGGGCCACT | CAGCAGTATAATTACTGGCCGCTCACT |
| | | | SEQ ID NO:117 | SEQ ID NO:8129 | SEQ ID NO:16141 |
| | | AA | RTSQSVWISVA | GAATRAT | QQYNYWPLT |
| | | | SEQ ID NO:118 | SEQ ID NO:8130 | SEQ ID NO:16142 |
| iPS:468832 | 21-225_76H10 | NA | CGGGCAAGTCAGGACATTAGAAATTATTTAGGC | GGTGCATCCAGTTTGCAAAGT | CTACAGTATAATAGTTACCCATTCACT |
| | | | SEQ ID NO:119 | SEQ ID NO:8131 | SEQ ID NO:16143 |
| | | AA | RASQDIRNYLG | GASSLQS | LQYNSYPFT |
| | | | SEQ ID NO:120 | SEQ ID NO:8132 | SEQ ID NO:16144 |
| iPS:468834 | 21-225_94G10 | NA | AGGGCCAGTCAGAGTGTTAACAGCAACTACTTAGCC | GGTGCATCCAGCAGGGCCACT | CAGCAGTATGAAAGCTCGCCGTGGACG |
| | | | SEQ ID NO:121 | SEQ ID NO:8133 | SEQ ID NO:16145 |

FIGURE 49

| | | | | | |
|---|---|---|---|---|---|
| | | AA | RASQSVNSNYLA | GASSRAT | QQYESSPWT |
| | | | SEQ ID NO:122 | SEQ ID NO:8134 | SEQ ID NO:16146 |
| **iPS:468836** | **21-225_198E3** | NA | CGGGCAAGTCAGGGCATAA GAAAAGATTTAGGC | GCTGCATCCAGTTTGCAA AGT | CTACAACATTATCGTTACCC TTTCACT |
| | | | SEQ ID NO:123 | SEQ ID NO:8135 | SEQ ID NO:16147 |
| | | AA | RASQGIRKDLG | AASSLQS | LQHYRYPFT |
| | | | SEQ ID NO:124 | SEQ ID NO:8136 | SEQ ID NO:16148 |
| **iPS:468838** | **21-225_80E12** | NA | AGGGCCAGTCAGAGCGTTAA CAGCAACTACTTAGCC | GGTGCATCCAGCAGGGC CACT | CAGCAGTATGAAAGCTCGCC GTGGACG |
| | | | SEQ ID NO:125 | SEQ ID NO:8137 | SEQ ID NO:16149 |
| | | AA | RASQSVNSNYLA | GASSRAT | QQYESSPWT |
| | | | SEQ ID NO:126 | SEQ ID NO:8138 | SEQ ID NO:16150 |
| **iPS:468840** | **21-225_200H9** | NA | CGGGCAAGTCAGGGCATTAG AAATGATTTAGGC | GCTGCATCCAGTTTGCAA AGT | CTACAGCATAATAGTTACCC TCTCACT |
| | | | SEQ ID NO:127 | SEQ ID NO:8139 | SEQ ID NO:16151 |
| | | AA | RASQGIRNDLG | AASSLQS | LQHNSYPLT |
| | | | SEQ ID NO:128 | SEQ ID NO:8140 | SEQ ID NO:16152 |
| **iPS:468820** | **21-225_76E10** | NA | AGGGCCAGTCAGAGTGTTAA CAGCAACTACTTAGCC | GGTGCATCCAGCAGGGC CACT | CAGCAGTATGAAAGCTCGCC GTGGACG |
| | | | SEQ ID NO:129 | SEQ ID NO:8141 | SEQ ID NO:16153 |
| | | AA | RASQSVNSNYLA | GASSRAT | QQYESSPWT |
| | | | SEQ ID NO:130 | SEQ ID NO:8142 | SEQ ID NO:16154 |
| **iPS:468842** | **21-225_50H4** | NA | CGGGCAAGTCAGGGCATTAG AAATGATTTAGGC | GCTGCATCCAGTTTGCAA AGT | CTACAACATTATAGTTACCC TCGGACG |
| | | | SEQ ID NO:131 | SEQ ID NO:8143 | SEQ ID NO:16155 |
| | | AA | RASQGIRNDLG | AASSLQS | LQHYSYPRT |
| | | | SEQ ID NO:132 | SEQ ID NO:8144 | SEQ ID NO:16156 |
| **iPS:468844** | **21-225_48E10** | NA | CGGGCAAGTCAGGGCATTAG AAGTGATTTAGGC | ACTGCATCCAGTTTGCAA AGT | CTACAGTATAATAGTTACCC ATTCACT |
| | | | SEQ ID NO:133 | SEQ ID NO:8145 | SEQ ID NO:16157 |

FIGURE 49

| | | | | | |
|---|---|---|---|---|---|
| | | AA | RASQGIRSDLG | TASSLQS | LQYNSYPFT |
| | | | SEQ ID NO:134 | SEQ ID NO:8146 | SEQ ID NO:16158 |
| iPS:468846 | 21-225_53B10 | NA | CGGGCAAGTCAGGACATTAG AAATTATTTAGGC | GGTGCATCCAGTTTGCA AAGT | CTACAGTATAATAGTTACCC ATTCACT |
| | | | SEQ ID NO:135 | SEQ ID NO:8147 | SEQ ID NO:16159 |
| | | AA | RASQDIRNYLG | GASSLQS | LQYNSYPFT |
| | | | SEQ ID NO:136 | SEQ ID NO:8148 | SEQ ID NO:16160 |
| iPS:468848 | 21-225_54B1 | NA | CGGGCAAGTCAGAACATTAG CAGCTATTTAAAT | GCTGCATCCAGTTTGCAT AGT | CAACAGAGTTACAGAACCCC TCTGTGGACG |
| | | | SEQ ID NO:137 | SEQ ID NO:8149 | SEQ ID NO:16161 |
| | | AA | RASQNISSYLN | AASSLHS | QQSYRTPLWT |
| | | | SEQ ID NO:138 | SEQ ID NO:8150 | SEQ ID NO:16162 |
| iPS:468850 | 21-225_63F4 | NA | AAGTCCAGCCAGAGTGTTTT ATCCAGCTCCAACAATAACA ACTACTTAGCT | TGGGCATCTACCCGGGA ATCC | CAGCAATATTATACTACTCC GTGCAGT |
| | | | SEQ ID NO:139 | SEQ ID NO:8151 | SEQ ID NO:16163 |
| | | AA | KSSQSVLSSSNNNNYLA | WASTRES | QQYYTTPCS |
| | | | SEQ ID NO:140 | SEQ ID NO:8152 | SEQ ID NO:16164 |
| iPS:468852 | 21-225_71F3 | NA | AAGTCCAGCCAGAGTGTTTT ATCCAACTCCAACAATAACA ACTACTTAGCT | TGGGCATCTACCCGGGA ATCC | CAGCAATATTATACTACTCC GTGCAGT |
| | | | SEQ ID NO:141 | SEQ ID NO:8153 | SEQ ID NO:16165 |
| | | AA | KSSQSVLSNSNNNNYLA | WASTRES | QQYYTTPCS |
| | | | SEQ ID NO:142 | SEQ ID NO:8154 | SEQ ID NO:16166 |
| iPS:468854 | 21-225_72C4 | NA | AGGTCTGGTCAAAGCCTCGT ATACAGTGATGGAAACACCT ACTTGAAT | GAGGTTTCTAAGTGGGA CTCT | ATGCAAGGTACACACTGGCC GCTCACT |
| | | | SEQ ID NO:143 | SEQ ID NO:8155 | SEQ ID NO:16167 |
| | | AA | RSGQSLVYSDGNTYLN | EVSKWDS | MQGTHWPLT |
| | | | SEQ ID NO:144 | SEQ ID NO:8156 | SEQ ID NO:16168 |

FIGURE 49

| | | | | | |
|---|---|---|---|---|---|
| **iPS:468856** | **21-225_77C9** | NA | AGGTCTAGTCAAAGCCTCGTTTACAGTGTTGGAAACACCTCCTTGAGT | AAGGTTTCTAACTGGGACTCT | ATGCAAGGTACACACTGGCCATTCACT |
| | | | SEQ ID NO:145 | SEQ ID NO:8157 | SEQ ID NO:16169 |
| | | AA | RSSQSLVYSVGNTSLS | KVSNWDS | MQGTHWPFT |
| | | | SEQ ID NO:146 | SEQ ID NO:8158 | SEQ ID NO:16170 |
| **iPS:468858** | **21-225_148C9** | NA | CGGGCAAGTCGGGGCATTAGAGATGATTTAGGC | GCTGCATCCAGTTTGCAGAGT | CTACAGCATTATAGTTATCCTCGGACG |
| | | | SEQ ID NO:147 | SEQ ID NO:8159 | SEQ ID NO:16171 |
| | | AA | RASRGIRDDLG | AASSLQS | LQHYSYPRT |
| | | | SEQ ID NO:148 | SEQ ID NO:8160 | SEQ ID NO:16172 |
| **iPS:468860** | **21-225_224E7** | NA | CGGGCAAGTCAGGGCATTAGAAATGATTTAGGC | GCTGCATCCAGTTTGCAAAGT | CTACAACATTATAGTTACCCTCGGACG |
| | | | SEQ ID NO:149 | SEQ ID NO:8161 | SEQ ID NO:16173 |
| | | AA | RASQGIRNDLG | AASSLQS | LQHYSYPRT |
| | | | SEQ ID NO:150 | SEQ ID NO:8162 | SEQ ID NO:16174 |
| **iPS:468862** | **21-225_178H8** | NA | ACTGGAACCAGCAGTGACGTTGGTGGTTATAATTTTGTCTCC | GAGGTCAGTAATCGGCCCTCA | AGCTCATATACAAGCAGCTACACTTGGGTG |
| | | | SEQ ID NO:151 | SEQ ID NO:8163 | SEQ ID NO:16175 |
| | | AA | TGTSSDVGGYNFVS | EVSNRPS | SSYTSSYTWV |
| | | | SEQ ID NO:152 | SEQ ID NO:8164 | SEQ ID NO:16176 |
| **iPS:468864** | **21-225_60D6** | NA | TCTGGAAGCAGCTCCAACATCGGAAGTAATACTGTAAAC | AGTAATAATCAGCGGCCCTCA | GCAGCATGGGATGACAGCCTGAATGGTCCG |
| | | | SEQ ID NO:153 | SEQ ID NO:8165 | SEQ ID NO:16177 |
| | | AA | SGSSSNIGSNTVN | SNNQRPS | AAWDDSLNGP |
| | | | SEQ ID NO:154 | SEQ ID NO:8166 | SEQ ID NO:16178 |
| **iPS:468866** | | NA | ACTGGAGATGCAATGCCGAAAAAATATGCTTAT | GAGGACAGCAAGCGACCCTCC | AACTCAACAGACAGCAGTGGTAATCGGGTG |

FIGURE 49

| | | | | | |
|---|---|---|---|---|---|
| | **21-225_190C1** | | SEQ ID NO:155 | SEQ ID NO:8167 | SEQ ID NO:16179 |
| | | AA | TGDAMPKKYAY | EDSKRPS | NSTDSSGNRV |
| | | | SEQ ID NO:156 | SEQ ID NO:8168 | SEQ ID NO:16180 |
| **iPS:468868** | **21-225_74A1** | NA | CGGGCAAGTCAGGGCATTAG AAATGATTTAGGC | GCTGCATCCAGTTTGCAA AGT | CTACAGCATGATAGTTACCC TCTCACT |
| | | | SEQ ID NO:157 | SEQ ID NO:8169 | SEQ ID NO:16181 |
| | | AA | RASQGIRNDLG | AASSLQS | LQHDSYPLT |
| | | | SEQ ID NO:158 | SEQ ID NO:8170 | SEQ ID NO:16182 |
| **iPS:468870** | **21-225_74A8** | NA | AAGTCCAGCCAGAGTGTTTT GTACAGCTCCAACAGTCACA ACTACTTAGCT | TGGGCATCTACCCGGGA ATCC | CAGCAATATTATAGTACTCC GTGCAGT |
| | | | SEQ ID NO:159 | SEQ ID NO:8171 | SEQ ID NO:16183 |
| | | AA | KSSQSVLYSSNSHNYLA | WASTRES | QQYYSTPCS |
| | | | SEQ ID NO:160 | SEQ ID NO:8172 | SEQ ID NO:16184 |
| **iPS:472730** | **21-225_14B1_LC1** | NA | CGGGCAAGTCAGGACATTAG AGATAATTTAGGC | ACTGCATACAGTTTGCA AAGT | CTACAACATTATAATTACCC GCTCACT |
| | | | SEQ ID NO:161 | SEQ ID NO:8173 | SEQ ID NO:16185 |
| | | AA | RASQDIRDNLG | TAYSLQS | LQHYNYPLT |
| | | | SEQ ID NO:162 | SEQ ID NO:8174 | SEQ ID NO:16186 |
| **iPS:472731** | **21-225_14B1_LC2** | NA | TCTGGAGATAAATTGGGGGA TAAATATGCTTAC | CAAGATAGGAAGCGCCC CTCA | CAGGCGTGGGACAACAGCAC TGTGGTG |
| | | | SEQ ID NO:163 | SEQ ID NO:8175 | SEQ ID NO:16187 |
| | | AA | SGDKLGDKYAY | QDRKRPS | QAWDNSTVV |
| | | | SEQ ID NO:164 | SEQ ID NO:8176 | SEQ ID NO:16188 |
| **iPS:472732** | **21-225_2B10_LC1** | NA | AGGTCTAGTCAAAGCCTCGT ATACAGTGATGGAAACACCT TCTTGAAT | AAGGTTTCTAACTGGGA CTCT | ATACAAGGTACGCACTGGCC TTTCCCC |
| | | | SEQ ID NO:165 | SEQ ID NO:8177 | SEQ ID NO:16189 |
| | | AA | RSSQSLVYSDGNTFLN | KVSNWDS | IQGTHWPFP |
| | | | SEQ ID NO:166 | SEQ ID NO:8178 | SEQ ID NO:16190 |

FIGURE 49

| | | | | | |
|---|---|---|---|---|---|
| **iPS:472733** | **21-225_2B10_LC2** | NA | ACTGGAACCAGCAGTGACGTTGGTGGTTATAATTTTGTCTCC | GAGGTCAGTAATCGGCCCTCA | AGCTCATATACAAGCACCGGCACTGTGGTA |
| | | | SEQ ID NO:167 | SEQ ID NO:8179 | SEQ ID NO:16191 |
| | | AA | TGTSSDVGGYNFVS | EVSNRPS | SSYTSTGTVV |
| | | | SEQ ID NO:168 | SEQ ID NO:8180 | SEQ ID NO:16192 |
| **iPS:473253** | **21-225_7C3_LC1** | NA | CGGGCAAGTCAGGGCATTAGAAGTGATTTAGGC | GCTGCATCCAGTTTGCAAAGT | CTACAGCATAATAGTTACCTCCCCATCACC |
| | | | SEQ ID NO:169 | SEQ ID NO:8181 | SEQ ID NO:16193 |
| | | AA | RASQGIRSDLG | AASSLQS | LQHNSYLPIT |
| | | | SEQ ID NO:170 | SEQ ID NO:8182 | SEQ ID NO:16194 |
| **iPS:473254** | **21-225_7C3_LC2** | NA | CGGGCGAGTCAGGGTATTAGCAGCTGGTTAGCC | GCTGCATCCAGGTTGCAAAGT | CAACAGGCTAACAGTTTCCCATTCACT |
| | | | SEQ ID NO:171 | SEQ ID NO:8183 | SEQ ID NO:16195 |
| | | AA | RASQGISSWLA | AASRLQS | QQANSFPFT |
| | | | SEQ ID NO:172 | SEQ ID NO:8184 | SEQ ID NO:16196 |
| **iPS:473255** | **21-225_9F12_LC1** | NA | CGGGCGAGTCAGGGTATTAGCAGGTGGTTAGCC | GCTGCATCCAGATTGCAAAGT | CAACAGGCTAACAGTTTCCCATTCACT |
| | | | SEQ ID NO:173 | SEQ ID NO:8185 | SEQ ID NO:16197 |
| | | AA | RASQGISRWLA | AASRLQS | QQANSFPFT |
| | | | SEQ ID NO:174 | SEQ ID NO:8186 | SEQ ID NO:16198 |
| **iPS:473256** | **21-225_9F12_LC2** | NA | CGGGCAAGTCAGGGCATTAGAAATGATTTAGGC | GCTGCATCCAGTTTGCAAAGT | CTACAGCATAATAGTTACCTCCCCATCACC |
| | | | SEQ ID NO:175 | SEQ ID NO:8187 | SEQ ID NO:16199 |
| | | AA | RASQGIRNDLG | AASSLQS | LQHNSYLPIT |
| | | | SEQ ID NO:176 | SEQ ID NO:8188 | SEQ ID NO:16200 |
| **iPS:472742** | **21-225_30D9_LC2** | NA | TCTGGAGATAAATTGGGGGATAAATATGTTTAC | CAAGATAGGAAGCGGCCCTCA | CAGGCGTGGGACAACAGCACTGCGGTA |
| | | | SEQ ID NO:177 | SEQ ID NO:8189 | SEQ ID NO:16201 |
| | | AA | SGDKLGDKYVY | QDRKRPS | QAWDNSTAV |

FIGURE 49

| | | | | | |
|---|---|---|---|---|---|
| | | | SEQ ID NO:178 | SEQ ID NO:8190 | SEQ ID NO:16202 |
| **iPS:472741** | **21-225_30D9_LC1** | NA | AGGTCTAGTCAAAGCCTCGTATCCAGTGATGGAAACACCTTCTTGAAT | AAGGTTTCTAACTGGGACTCT | TTGCAAGGTACACACTGGCCTCTCACC |
| | | | SEQ ID NO:179 | SEQ ID NO:8191 | SEQ ID NO:16203 |
| | | AA | RSSQSLVSSDGNTFLN | KVSNWDS | LQGTHWPLT |
| | | | SEQ ID NO:180 | SEQ ID NO:8192 | SEQ ID NO:16204 |
| **iPS:472743** | **21-225_68G6** | NA | TCTGGAGATAAATTGGGGATAAATATACTTAC | CAAGATAGGAAGCGGCCCTCA | CAGGCGTGGGACAATAGTACTGCGGTA |
| | | | SEQ ID NO:181 | SEQ ID NO:8193 | SEQ ID NO:16205 |
| | | AA | SGDKLGDKYTY | QDRKRPS | QAWDNSTAV |
| | | | SEQ ID NO:182 | SEQ ID NO:8194 | SEQ ID NO:16206 |
| **iPS:392573** | **21-225_15G2** | NA | ACTGGAACCAGCAGTGACGTTGGTGGTTATAACTATGTCTCC | GAGGTCAGTAATCGGCCCTCA | ACCTCATATACAAGCACCAGCACTGTGGTC |
| | | | SEQ ID NO:183 | SEQ ID NO:8195 | SEQ ID NO:16207 |
| | | AA | TGTSSDVGGYNYVS | EVSNRPS | TSYTSTSTVV |
| | | | SEQ ID NO:184 | SEQ ID NO:8196 | SEQ ID NO:16208 |
| **iPS:392583** | **21-225_10B10** | NA | TCTGGAGATAAATTGGGGAATAAATATGCTTGG | CAAGATAGGAAGCGGCCCTCA | CAGGCGTGGGACAACAGCACTGTGGTT |
| | | | SEQ ID NO:185 | SEQ ID NO:8197 | SEQ ID NO:16209 |
| | | AA | SGDKLGNKYAW | QDRKRPS | QAWDNSTVV |
| | | | SEQ ID NO:186 | SEQ ID NO:8198 | SEQ ID NO:16210 |
| **iPS:392585** | **21-225_14H11** | NA | TCTGGAGATAAATTGGGGGAAAAATATGTTTGC | CAAGATACCAAGCGGCCCTCA | CAGGCGTGGGACAGCAGCACTATA |
| | | | SEQ ID NO:187 | SEQ ID NO:8199 | SEQ ID NO:16211 |
| | | AA | SGDKLGEKYVC | QDTKRPS | QAWDSSTI |
| | | | SEQ ID NO:188 | SEQ ID NO:8200 | SEQ ID NO:16212 |
| **iPS:392587** | | NA | TCTGGAGAGAAATTGGGGGATAAATATGTTTGT | CAAGATAGCAAGCGGCCCTCA | CAGGCGTGGAACAGCAGCAATGTGGTA |

**FIGURE 49**

| | | | | | |
|---|---|---|---|---|---|
| | **21-225_18G5** | | SEQ ID NO:189 | SEQ ID NO:8201 | SEQ ID NO:16213 |
| | | AA | SGEKLGDKYVC | QDSKRPS | QAWNSSNVV |
| | | | SEQ ID NO:190 | SEQ ID NO:8202 | SEQ ID NO:16214 |
| **iPS:392589** | **21-225_27H2** | NA | TCTGGAGATAAATTGGGGGATAAATATGCTTCC | CAAGATGGCAAGCGGCCCTCA | CAGGCGTGGGACAGCAGCACTTATGTGGTA |
| | | | SEQ ID NO:191 | SEQ ID NO:8203 | SEQ ID NO:16215 |
| | | AA | SGDKLGDKYAS | QDGKRPS | QAWDSSTYVV |
| | | | SEQ ID NO:192 | SEQ ID NO:8204 | SEQ ID NO:16216 |
| **iPS:392593** | **21-225_3E10** | NA | GGGGGAAACAACATTGGAAGTAAAGCTGTGCAC | AGCGATAGCAACCGGCCCTCA | CAGGTGTGGGACAGTAGTAGTGATCATGTGGTA |
| | | | SEQ ID NO:193 | SEQ ID NO:8205 | SEQ ID NO:16217 |
| | | AA | GGNNIGSKAVH | SDSNRPS | QVWDSSSDHVV |
| | | | SEQ ID NO:194 | SEQ ID NO:8206 | SEQ ID NO:16218 |
| **iPS:392596** | **21-225_12D8** | NA | ACCCTAAGCAGTGAGCACAGCACCTACACCATCGAA | GTTAAGAGTGATGGCAGCCACAGCAAGGGGGAC | GGAGAGAGCCACACGATTGATGGCCAAGTCGGTGTGGTA |
| | | | SEQ ID NO:195 | SEQ ID NO:8207 | SEQ ID NO:16219 |
| | | AA | TLSSEHSTYTIE | VKSDGSHSKGD | GESHTIDGQVGVV |
| | | | SEQ ID NO:196 | SEQ ID NO:8208 | SEQ ID NO:16220 |
| **iPS:392598** | **21-225_18E10** | NA | TCTGGAGATAGATTGGGGGATAAATATGCTTGG | CAAGATCGCAAGCGGCCCTCA | CAGGCGTGGGACAGCAGCACAGTGGTA |
| | | | SEQ ID NO:197 | SEQ ID NO:8209 | SEQ ID NO:16221 |
| | | AA | SGDRLGDKYAW | QDRKRPS | QAWDSSTVV |
| | | | SEQ ID NO:198 | SEQ ID NO:8210 | SEQ ID NO:16222 |
| **iPS:392618** | **21-225_16F10** | NA | AAGTCTAGTCAGAGCCTCCTGCATAGTGATGGAAAGACCCATTTGAAT | GAAGTTTCCTACCGGTTCTCT | TTTCAAAGTATACAGCTTCCGCTCACT |
| | | | SEQ ID NO:199 | SEQ ID NO:8211 | SEQ ID NO:16223 |
| | | AA | KSSQSLLHSDGKTHLN | EVSYRFS | FQSIQLPLT |
| | | | SEQ ID NO:200 | SEQ ID NO:8212 | SEQ ID NO:16224 |

FIGURE 49

| | | | | | |
|---|---|---|---|---|---|
| **iPS:392620** | **21-225_17H5** | NA | CGGGCGAGTCAGGGCATTAG CAATTATTTAGCC | GCTGCATCCAGTTTACAA AGT | CAACAGTATCATAGTTACCC ATTCACT |
| | | | SEQ ID NO:201 | SEQ ID NO:8213 | SEQ ID NO:16225 |
| | | AA | RASQGISNYLA | AASSLQS | QQYHSYPFT |
| | | | SEQ ID NO:202 | SEQ ID NO:8214 | SEQ ID NO:16226 |
| **iPS:392622** | **21-225_17H8** | NA | CGGGCAAGTCAGGGCATTAG AAATGATTTAGGC | GGTGCATCCAGTTTGCA AAGT | CTACAGCATAATAGTTACCC ACTCACT |
| | | | SEQ ID NO:203 | SEQ ID NO:8215 | SEQ ID NO:16227 |
| | | AA | RASQGIRNDLG | GASSLQS | LQHNSYPLT |
| | | | SEQ ID NO:204 | SEQ ID NO:8216 | SEQ ID NO:16228 |
| **iPS:392624** | **21-225_17H12** | NA | CGGGCAAGTCAGGACATTAG AAATGATTTAGGC | GCTGCATCCAGTTTGCAA AGT | CTACAGCATTATAGTTATAT GTTCACT |
| | | | SEQ ID NO:205 | SEQ ID NO:8217 | SEQ ID NO:16229 |
| | | AA | RASQDIRNDLG | AASSLQS | LQHYSYMFT |
| | | | SEQ ID NO:206 | SEQ ID NO:8218 | SEQ ID NO:16230 |
| **iPS:392626** | **21-225_18A5** | NA | CGGGCAAGTCAGGGCATTAG AAATGATTTAGGC | GCTGCATCCAGTTTGCAA AGT | CTACAGCATAATAGTTACCC GTGGACG |
| | | | SEQ ID NO:207 | SEQ ID NO:8219 | SEQ ID NO:16231 |
| | | AA | RASQGIRNDLG | AASSLQS | LQHNSYPWT |
| | | | SEQ ID NO:208 | SEQ ID NO:8220 | SEQ ID NO:16232 |
| **iPS:392628** | **21-225_20C2** | NA | CGGGCAAGTCAGGGCATTAG AAATGATTTAGGC | GCTGCATCCAGTTTGCAA AGT | CTTCAACATGCTAGTTACCC GCTCACT |
| | | | SEQ ID NO:209 | SEQ ID NO:8221 | SEQ ID NO:16233 |
| | | AA | RASQGIRNDLG | AASSLQS | LQHASYPLT |
| | | | SEQ ID NO:210 | SEQ ID NO:8222 | SEQ ID NO:16234 |
| **iPS:392630** | **21-225_20E5** | NA | CGGGCAAGTCAGGGCATTAG AAATGATTTAGGC | GCTGCATCCAGTTTGCAA AGT | CTACAGCATAATAGTTACCC GCTCACT |
| | | | SEQ ID NO:211 | SEQ ID NO:8223 | SEQ ID NO:16235 |
| | | AA | RASQGIRNDLG | AASSLQS | LQHNSYPLT |
| | | | SEQ ID NO:212 | SEQ ID NO:8224 | SEQ ID NO:16236 |

FIGURE 49

| | | | | | |
|---|---|---|---|---|---|
| **iPS:392632** | **21-225_16A11** | NA | CGGGCAAGTCAGGACATTAG AAATCATTTAGGC | GCTGCATCCAGTTTGCAA AGT | CTACAGTATAATAGTTATCC ATTCACT |
| | | | SEQ ID NO:213 | SEQ ID NO:8225 | SEQ ID NO:16237 |
| | | AA | RASQDIRNHLG | AASSLQS | LQYNSYPFT |
| | | | SEQ ID NO:214 | SEQ ID NO:8226 | SEQ ID NO:16238 |
| **iPS:392634** | **21-225_17H3** | NA | CGGGCAAGTCAGGGCATTAG AAGTGATTTAGGC | GCTGCATCCAGTTTGCAA AGT | CTACAGCAATATAGTTACCC TCGGACG |
| | | | SEQ ID NO:215 | SEQ ID NO:8227 | SEQ ID NO:16239 |
| | | AA | RASQGIRSDLG | AASSLQS | LQQYSYPRT |
| | | | SEQ ID NO:216 | SEQ ID NO:8228 | SEQ ID NO:16240 |
| **iPS:392636** | **21-225_17A6** | NA | CGGGCAAGTCAGACCATTAG CAACTATTTAAAT | GCTGCTTCCAGTTTGCAA AGT | CAACAGAGTCACACTTCCCC GCTCACT |
| | | | SEQ ID NO:217 | SEQ ID NO:8229 | SEQ ID NO:16241 |
| | | AA | RASQTISNYLN | AASSLQS | QQSHTSPLT |
| | | | SEQ ID NO:218 | SEQ ID NO:8230 | SEQ ID NO:16242 |
| **iPS:392638** | **21-225_17F9** | NA | CGGGCAAGTCAGGTCATTAG AAATGATTTAGGC | GCTGTATCCAGTTTGCAA AGT | CTACAACATAATACTTATCC GCTCACT |
| | | | SEQ ID NO:219 | SEQ ID NO:8231 | SEQ ID NO:16243 |
| | | AA | RASQVIRNDLG | AVSSLQS | LQHNTYPLT |
| | | | SEQ ID NO:220 | SEQ ID NO:8232 | SEQ ID NO:16244 |
| **iPS:392640** | **21-225_18A1** | NA | CGGGCAAGTCAGGGCATTAG AAATGATTTAGGC | GCTGCATCCAGTTTACAA AGT | CTACAGCATAATAGTTACCC GCTCACT |
| | | | SEQ ID NO:221 | SEQ ID NO:8233 | SEQ ID NO:16245 |
| | | AA | RASQGIRNDLG | AASSLQS | LQHNSYPLT |
| | | | SEQ ID NO:222 | SEQ ID NO:8234 | SEQ ID NO:16246 |
| **iPS:392642** | **21-225_18C6** | NA | CGGACAAGTCAGGGCATTAG AAATGATTTAGGC | GCTGCATCCAGTTTGCAA AGT | CTACAGCATAGTTCTTACCC GCTCACT |
| | | | SEQ ID NO:223 | SEQ ID NO:8235 | SEQ ID NO:16247 |
| | | AA | RTSQGIRNDLG | AASSLQS | LQHSSYPLT |
| | | | SEQ ID NO:224 | SEQ ID NO:8236 | SEQ ID NO:16248 |

**FIGURE 49**

| | | | | | |
|---|---|---|---|---|---|
| **iPS:392644** | **21-225_19E1** | NA | CGGGCAAGTCAGGGCATTAG AAATGATTTAGGC | GCTGCATCCAATTTACAA AGT | CTACAGCATAATAGTTTCCC GCTCACT |
| | | | SEQ ID NO:225 | SEQ ID NO:8237 | SEQ ID NO:16249 |
| | | AA | RASQGIRNDLG | AASNLQS | LQHNSFPLT |
| | | | SEQ ID NO:226 | SEQ ID NO:8238 | SEQ ID NO:16250 |
| **iPS:392646** | **21-225_20G2** | NA | CGGGCAAGTCAGGGCATTAG AAATGATTTAGGC | GCTGCATCCAGTTTCCAA AGT | CTACAGCATAATAGTTACCC GCTCACT |
| | | | SEQ ID NO:227 | SEQ ID NO:8239 | SEQ ID NO:16251 |
| | | AA | RASQGIRNDLG | AASSFQS | LQHNSYPLT |
| | | | SEQ ID NO:228 | SEQ ID NO:8240 | SEQ ID NO:16252 |
| **iPS:392648** | **21-225_16D11** | NA | CGGGCAAGTCAGACCATTAG CAACTATTTAAAT | GCTGCATCCAGTTTGCAA AGT | CAACAGAGTCACAGTTCCCC GCTCACT |
| | | | SEQ ID NO:229 | SEQ ID NO:8241 | SEQ ID NO:16253 |
| | | AA | RASQTISNYLN | AASSLQS | QQSHSSPLT |
| | | | SEQ ID NO:230 | SEQ ID NO:8242 | SEQ ID NO:16254 |
| **iPS:392650** | **21-225_17A4** | NA | CGGGCGAGTCAGGGTATTGG CAACTGGTTAGCC | GCTGCATCCAGTTTGCAA AGT | CAACAGGCTAACAGTTTCCC TCGGACG |
| | | | SEQ ID NO:231 | SEQ ID NO:8243 | SEQ ID NO:16255 |
| | | AA | RASQGIGNWLA | AASSLQS | QQANSFPRT |
| | | | SEQ ID NO:232 | SEQ ID NO:8244 | SEQ ID NO:16256 |
| **iPS:392652** | **21-225_17C6** | NA | CGGGCAAGTCAGAGCATTAA TACTTATTTAAAT | GCTGCATCCAGTTTGCAA AGT | CAACAGAGTTACAGAACCCC CTTTTTCACT |
| | | | SEQ ID NO:233 | SEQ ID NO:8245 | SEQ ID NO:16257 |
| | | AA | RASQSINTYLN | AASSLQS | QQSYRTPFFT |
| | | | SEQ ID NO:234 | SEQ ID NO:8246 | SEQ ID NO:16258 |
| **iPS:392654** | **21-225_17A10** | NA | CGGGCAAGTCAGGGCATTAG AAATGATTTAGGC | GCTGCATCCAGTTTACAA AGT | CTACAGCATAATAGTTACCC GCTCACT |
| | | | SEQ ID NO:235 | SEQ ID NO:8247 | SEQ ID NO:16259 |
| | | AA | RASQGIRNDLG | AASSLQS | LQHNSYPLT |
| | | | SEQ ID NO:236 | SEQ ID NO:8248 | SEQ ID NO:16260 |

**FIGURE 49**

| | | | | | |
|---|---|---|---|---|---|
| **iPS:392656** | **21-225_1F2** | NA | CGGGCAAGTCAGGGCATTAG AAATGATTTAGGC | GCTGCATCCAGTGTGCA AAGT | CTACAGCATAATAGTTACCC GCTCACT |
| | | | SEQ ID NO:237 | SEQ ID NO:8249 | SEQ ID NO:16261 |
| | | AA | RASQGIRNDLG | AASSVQS | LQHNSYPLT |
| | | | SEQ ID NO:238 | SEQ ID NO:8250 | SEQ ID NO:16262 |
| **iPS:392658** | **21-225_18E8** | NA | CGGGCAAGTCAGGGCATTAG AAATGATTTAGGC | GCTGCATCCAGTTTGCAA AGT | CTACAGCATAATAGTTACCC GCTCACT |
| | | | SEQ ID NO:239 | SEQ ID NO:8251 | SEQ ID NO:16263 |
| | | AA | RASQGIRNDLG | AASSLQS | LQHNSYPLT |
| | | | SEQ ID NO:240 | SEQ ID NO:8252 | SEQ ID NO:16264 |
| **iPS:392660** | **21-225_19B3** | NA | CGGGCAGGTCAGAACATTAT CAACTATTTAAAT | GTTGCATCCAGTTTGCAA AGT | CAACAGAGTTACAGTACCCC TTTCACT |
| | | | SEQ ID NO:241 | SEQ ID NO:8253 | SEQ ID NO:16265 |
| | | AA | RAGQNIINYLN | VASSLQS | QQSYSTPFT |
| | | | SEQ ID NO:242 | SEQ ID NO:8254 | SEQ ID NO:16266 |
| **iPS:392664** | **21-225_20F6** | NA | CGGGCAAGTCAGAGCATTAT CACCTATTTAAAT | ACTGCATCCAGTTTGCAA AGT | CAACAGACTTACAGTCCCCC GCTCACT |
| | | | SEQ ID NO:243 | SEQ ID NO:8255 | SEQ ID NO:16267 |
| | | AA | RASQSIITYLN | TASSLQS | QQTYSPPLT |
| | | | SEQ ID NO:244 | SEQ ID NO:8256 | SEQ ID NO:16268 |
| **iPS:392666** | **21-225_16F11** | NA | CGGGCAAGTCAGGGCATTAG AAATGATTTAGGC | GCTGCATCCAGTGTACA AAGT | CTACAGCATAATAGTTACCC GCTCACT |
| | | | SEQ ID NO:245 | SEQ ID NO:8257 | SEQ ID NO:16269 |
| | | AA | RASQGIRNDLG | AASSVQS | LQHNSYPLT |
| | | | SEQ ID NO:246 | SEQ ID NO:8258 | SEQ ID NO:16270 |
| **iPS:392668** | **21-225_17B4** | NA | CGGGCAAGTCAGAACATTAG TAGTTATTTAAAT | GGTGCATCCAGTTTGCA AACT | CAACAGAGTTACAGAACCCC CTTTTTCACT |
| | | | SEQ ID NO:247 | SEQ ID NO:8259 | SEQ ID NO:16271 |
| | | AA | RASQNISSYLN | GASSLQT | QQSYRTPFFT |
| | | | SEQ ID NO:248 | SEQ ID NO:8260 | SEQ ID NO:16272 |

FIGURE 49

| | | | | | |
|---|---|---|---|---|---|
| **iPS:392674** | **21-225_18C2** | NA | CGGGCAAGTCAGGGCATTAGAAATGATTTAGGC | ACTGCATCCAGTTTGCAAAGT | CTACAACATAATAGTTACCCGTGGACG |
| | | | SEQ ID NO:249 | SEQ ID NO:8261 | SEQ ID NO:16273 |
| | | AA | RASQGIRNDLG | TASSLQS | LQHNSYPWT |
| | | | SEQ ID NO:250 | SEQ ID NO:8262 | SEQ ID NO:16274 |
| **iPS:392676** | **21-225_19F3** | NA | CGGGCAAGTCAGGGCATAAGAAATGATTTAGGC | GCTGTTTCCAGTTTGCAAAGT | CTACAGCATGCCAGTTACCCGCTCACT |
| | | | SEQ ID NO:251 | SEQ ID NO:8263 | SEQ ID NO:16275 |
| | | AA | RASQGIRNDLG | AVSSLQS | LQHASYPLT |
| | | | SEQ ID NO:252 | SEQ ID NO:8264 | SEQ ID NO:16276 |
| **iPS:392678** | **21-225_20F3** | NA | CGGGCAAGTCAGAGCATTAGTAGTTATTTATAT | GCTGCATCCAGTTTGCAAAGT | CAACAGAGTTACAGTGCCCCTCCATTCACT |
| | | | SEQ ID NO:253 | SEQ ID NO:8265 | SEQ ID NO:16277 |
| | | AA | RASQSISSYLY | AASSLQS | QQSYSAPPFT |
| | | | SEQ ID NO:254 | SEQ ID NO:8266 | SEQ ID NO:16278 |
| **iPS:392680** | **21-225_20A7** | NA | CGGGCAAGTCAGGGCATTAGAAATGATTTAGGC | GCTGCATCCAGTTTGCAAAGT | CTACAGCATAATAGTTACCCGCTCACT |
| | | | SEQ ID NO:255 | SEQ ID NO:8267 | SEQ ID NO:16279 |
| | | AA | RASQGIRNDLG | AASSLQS | LQHNSYPLT |
| | | | SEQ ID NO:256 | SEQ ID NO:8268 | SEQ ID NO:16280 |
| **iPS:392682** | **21-225_16A12** | NA | CGGGCGAGTCAGGCCATTAACACTTATTTAGCC | GCTGCATCCAGTTTGCAAAGT | CAACAGTATTATAGTTATCCGCTCACT |
| | | | SEQ ID NO:257 | SEQ ID NO:8269 | SEQ ID NO:16281 |
| | | AA | RASQAINTYLA | AASSLQS | QQYYSYPLT |
| | | | SEQ ID NO:258 | SEQ ID NO:8270 | SEQ ID NO:16282 |
| **iPS:392684** | **21-225_17F4** | NA | CGGGCAAGTCAGGGCATTAGAAATGATTTAGGC | GCTGCATCCAGTTTGCAAAGT | CTACAGCATAATAGTTACCCATTCACT |
| | | | SEQ ID NO:259 | SEQ ID NO:8271 | SEQ ID NO:16283 |
| | | AA | RASQGIRNDLG | AASSLQS | LQHNSYPFT |
| | | | SEQ ID NO:260 | SEQ ID NO:8272 | SEQ ID NO:16284 |

FIGURE 49

| | | | | | |
|---|---|---|---|---|---|
| **iPS:392686** | **21-225_17C7** | NA | CGGGCAAGTCAGGGCATTAG AAATGATTTAGGC | GCTGCATCCAGTTTGCAA AGT | CTACAGCATAATAGTTACCC GTGGACG |
| | | | SEQ ID NO:261 | SEQ ID NO:8273 | SEQ ID NO:16285 |
| | | AA | RASQGIRNDLG | AASSLQS | LQHNSYPWT |
| | | | SEQ ID NO:262 | SEQ ID NO:8274 | SEQ ID NO:16286 |
| **iPS:392690** | **21-225_18F2** | NA | CGGGCAAGTCAGGGCATTAG AAATGATTTAGGC | GCTGCATCCAGTTTGCAA AGT | CTACAGCATAATAGTTACCC GTGGACG |
| | | | SEQ ID NO:263 | SEQ ID NO:8275 | SEQ ID NO:16287 |
| | | AA | RASQGIRNDLG | AASSLQS | LQHNSYPWT |
| | | | SEQ ID NO:264 | SEQ ID NO:8276 | SEQ ID NO:16288 |
| **iPS:392692** | **21-225_18G10** | NA | CGGGCGAGTCAGGACATTAG CTATTATTTAGCC | GTTGCATCCAGTTTGCAA AGT | TTACAGTATAATAGTTACCC ATTCACT |
| | | | SEQ ID NO:265 | SEQ ID NO:8277 | SEQ ID NO:16289 |
| | | AA | RASQDISYYLA | VASSLQS | LQYNSYPFT |
| | | | SEQ ID NO:266 | SEQ ID NO:8278 | SEQ ID NO:16290 |
| **iPS:392694** | **21-225_19A5** | NA | CGGGCAAGTCAGAACATTAT CAACTATTTAAAT | GTTGCATCCAATTTACAA GGT | CAACAGAGTTACAGTACCCC TTTCACT |
| | | | SEQ ID NO:267 | SEQ ID NO:8279 | SEQ ID NO:16291 |
| | | AA | RASQNIINYLN | VASNLQG | QQSYSTPFT |
| | | | SEQ ID NO:268 | SEQ ID NO:8280 | SEQ ID NO:16292 |
| **iPS:392696** | **21-225_20A4** | NA | CGGGCAAGTCAGAGCATTAT CAACTATTTAAAT | GCTGCATCCAGTTTGCAC AGT | CAACAGAGTTACAGAACCCC CTTATTCACT |
| | | | SEQ ID NO:269 | SEQ ID NO:8281 | SEQ ID NO:16293 |
| | | AA | RASQSIINYLN | AASSLHS | QQSYRTPLFT |
| | | | SEQ ID NO:270 | SEQ ID NO:8282 | SEQ ID NO:16294 |
| **iPS:392700** | **21-225_16E12** | NA | CGGGCAAGTCAGGGCATTAG AAATGATTTAGGC | GCTGCATCCAGTTTACAA AGT | CTACAGCATAATAGTTACCC GCTCACT |
| | | | SEQ ID NO:271 | SEQ ID NO:8283 | SEQ ID NO:16295 |
| | | AA | RASQGIRNDLG | AASSLQS | LQHNSYPLT |
| | | | SEQ ID NO:272 | SEQ ID NO:8284 | SEQ ID NO:16296 |

## FIGURE 49

| | | | | | |
|---|---|---|---|---|---|
| **iPS:392702** | **21-225_17F7** | NA | CGGGCAAGTCAGACCATTAGTAGTTTTTTAAAT | GCTGCTTCCAGTTTGCAAAGT | CAACAGAGTTACAGAACCCCCTTTTTCACT |
| | | | SEQ ID NO:273 | SEQ ID NO:8285 | SEQ ID NO:16297 |
| | | AA | RASQTISSFLN | AASSLQS | QQSYRTPFFT |
| | | | SEQ ID NO:274 | SEQ ID NO:8286 | SEQ ID NO:16298 |
| **iPS:392704** | **21-225_17F11** | NA | CGGGCAAGTCGGACCATTAACAACTATTTAAAT | GCTACATCCAGTTTACAAAGT | CAACAGACTTACAGTACCCCCTTATTCGCT |
| | | | SEQ ID NO:275 | SEQ ID NO:8287 | SEQ ID NO:16299 |
| | | AA | RASRTINNYLN | ATSSLQS | QQTYSTPLFA |
| | | | SEQ ID NO:276 | SEQ ID NO:8288 | SEQ ID NO:16300 |
| **iPS:392706** | **21-225_18A3** | NA | CGGGCAAGTCAGGGCATTAGAAATGATTTAGGC | GTTGCATCCAGTTTGCAAAGT | CTACAGCATAGTAGTTACCCGCTCACT |
| | | | SEQ ID NO:277 | SEQ ID NO:8289 | SEQ ID NO:16301 |
| | | AA | RASQGIRNDLG | VASSLQS | LQHSSYPLT |
| | | | SEQ ID NO:278 | SEQ ID NO:8290 | SEQ ID NO:16302 |
| **iPS:392708** | **21-225_18D11** | NA | CGGGCGAGTCAGGGCATTAGCTATTATTTAGCC | GTTGCATCCAGTTTGCAAAGT | CAACAGTATAATACTTACCCATTCACT |
| | | | SEQ ID NO:279 | SEQ ID NO:8291 | SEQ ID NO:16303 |
| | | AA | RASQGISYYLA | VASSLQS | QQYNTYPFT |
| | | | SEQ ID NO:280 | SEQ ID NO:8292 | SEQ ID NO:16304 |
| **iPS:392710** | **21-225_19A10** | NA | CGGGCAAGTCAGGGCATTAGAACTGATTTAGGC | ACTGCATCCAGTTTGCAAAGT | CTACAGCATAATGGTTACCCGTGGACG |
| | | | SEQ ID NO:281 | SEQ ID NO:8293 | SEQ ID NO:16305 |
| | | AA | RASQGIRTDLG | TASSLQS | LQHNGYPWT |
| | | | SEQ ID NO:282 | SEQ ID NO:8294 | SEQ ID NO:16306 |
| **iPS:392714** | **21-225_16G12** | NA | CGGGCGAGTCAGGACATTAGCAATTATTTAGCC | GCTGCATCCAGTTTGCAAAGT | CAACAGTATCATAGTTTCCCATTCACT |
| | | | SEQ ID NO:283 | SEQ ID NO:8295 | SEQ ID NO:16307 |
| | | AA | RASQDISNYLA | AASSLQS | QQYHSFPFT |
| | | | SEQ ID NO:284 | SEQ ID NO:8296 | SEQ ID NO:16308 |

FIGURE 49

| | | | | | |
|---|---|---|---|---|---|
| **iPS:392716** | **21-225_17B5** | NA | CGGGCAAGTCAGGGCATTAG AAATGATTTAGGC | GCTGCATCCAATTTGCAA AGT | CTACAGCATAATAGTTACCC GCTCACT |
| | | | SEQ ID NO:285 | SEQ ID NO:8297 | SEQ ID NO:16309 |
| | | AA | RASQGIRNDLG | AASNLQS | LQHNSYPLT |
| | | | SEQ ID NO:286 | SEQ ID NO:8298 | SEQ ID NO:16310 |
| **iPS:392718** | **21-225_17B8** | NA | AGGTCTAGTCAGAGCCTCCT GCATAGTAATGGGAACAACT CTTTGGAT | TTGGGTTCTCATCGGGCC TCC | ATGCAAGTTCTACAAACTCC TCCCCTCACT |
| | | | SEQ ID NO:287 | SEQ ID NO:8299 | SEQ ID NO:16311 |
| | | AA | RSSQSLLHSNGNNSLD | LGSHRAS | MQVLQTPPLT |
| | | | SEQ ID NO:288 | SEQ ID NO:8300 | SEQ ID NO:16312 |
| **iPS:392720** | **21-225_17A12** | NA | CGGGCAAGTCAGAGCATTAG TAGTTATTTAAAT | GCTGCATCCAGTTTGCAA AGT | CAACAGAGTTACAATACCCC CTTATTCACT |
| | | | SEQ ID NO:289 | SEQ ID NO:8301 | SEQ ID NO:16313 |
| | | AA | RASQSISSYLN | AASSLQS | QQSYNTPLFT |
| | | | SEQ ID NO:290 | SEQ ID NO:8302 | SEQ ID NO:16314 |
| **iPS:392722** | **21-225_18E12** | NA | CGGGCAAGTCAGAGCATTAG TAGTTATTTAAAT | GCTGCATCCAGTTTACAA AGT | CAACAGAGTTACAGAACCCC CTTTTTCACT |
| | | | SEQ ID NO:291 | SEQ ID NO:8303 | SEQ ID NO:16315 |
| | | AA | RASQSISSYLN | AASSLQS | QQSYRTPFFT |
| | | | SEQ ID NO:292 | SEQ ID NO:8304 | SEQ ID NO:16316 |
| **iPS:392726** | **21-225_20B5** | NA | CGGGCGAGTCAGGGCATTAA CAATTATTTAGCC | GCTGCATCCACTTTGCAA TCA | CAAAAGTATAACAGTGCCCC TCCGATCACC |
| | | | SEQ ID NO:293 | SEQ ID NO:8305 | SEQ ID NO:16317 |
| | | AA | RASQGINNYLA | AASTLQS | QKYNSAPPIT |
| | | | SEQ ID NO:294 | SEQ ID NO:8306 | SEQ ID NO:16318 |
| **iPS:392728** | **21-225_20F7** | NA | CGGGCGAGTCAGGGTATTAG CAGCTGGTTAGCC | GCTGCATCCAGTTTGCAA AGT | CAACAGGCTAACAGTTTCCC TCGGACG |
| | | | SEQ ID NO:295 | SEQ ID NO:8307 | SEQ ID NO:16319 |
| | | AA | RASQGISSWLA | AASSLQS | QQANSFPRT |

FIGURE 49

| | | | | | |
|---|---|---|---|---|---|
| | | | SEQ ID NO:296 | SEQ ID NO:8308 | SEQ ID NO:16320 |
| **iPS:392730** | **21-225_17A1** | NA | CGGGCAAGTCAGAACATTAACAATTATTTAAAT | ACTACATCTAGTTTACAAAGT | CAACAGAGTTACACTACCCCCACGTGGACG |
| | | | SEQ ID NO:297 | SEQ ID NO:8309 | SEQ ID NO:16321 |
| | | AA | RASQNINNYLN | TTSSLQS | QQSYTTPTWT |
| | | | SEQ ID NO:298 | SEQ ID NO:8310 | SEQ ID NO:16322 |
| **iPS:392732** | **21-225_17E5** | NA | CGGGCAAGTCAGGGCATTAGAAATGATTTAGGC | ACTGCATCCAGTTTGCAAAGT | CTACAACATAATAGTTACCCGTTGACG |
| | | | SEQ ID NO:299 | SEQ ID NO:8311 | SEQ ID NO:16323 |
| | | AA | RASQGIRNDLG | TASSLQS | LQHNSYPLT |
| | | | SEQ ID NO:300 | SEQ ID NO:8312 | SEQ ID NO:16324 |
| **iPS:392734** | **21-225_17D8** | NA | AGGGCCAGTCAGAGTGTTAGCAGCAACTTAGCC | GGTGCATCCACCAGGGCCAGT | CAGCAGTATAATAACTGGCCTCTGACG |
| | | | SEQ ID NO:301 | SEQ ID NO:8313 | SEQ ID NO:16325 |
| | | AA | RASQSVSSNLA | GASTRAS | QQYNNWPLT |
| | | | SEQ ID NO:302 | SEQ ID NO:8314 | SEQ ID NO:16326 |
| **iPS:392736** | **21-225_17B12** | NA | CGGGCAAGTCAGAATATTAACAACTATTTAAAT | ACTGCATCCAGTTTGCAAAGT | CAACAGACTTACACTACCCCCACGTGGACG |
| | | | SEQ ID NO:303 | SEQ ID NO:8315 | SEQ ID NO:16327 |
| | | AA | RASQNINNYLN | TASSLQS | QQTYTTPTWT |
| | | | SEQ ID NO:304 | SEQ ID NO:8316 | SEQ ID NO:16328 |
| **iPS:392738** | **21-225_18G4** | NA | CGGGCAAGTCAGAGCATTATCAGCTATTTAAAT | ACTGCATCCAGTTTGCAAACT | CAACAGACTTACAGTCCCCCGCTCACT |
| | | | SEQ ID NO:305 | SEQ ID NO:8317 | SEQ ID NO:16329 |
| | | AA | RASQSIISYLN | TASSLQT | QQTYSPPLT |
| | | | SEQ ID NO:306 | SEQ ID NO:8318 | SEQ ID NO:16330 |
| **iPS:392740** | **21-225_18H12** | NA | CGGGCAAGTCAGGGCATTAGAAATGATTTAGGC | ACTGCATCCAATTTGCAAAGT | CTACAACATAATAATTACCCGTGGACG |
| | | | SEQ ID NO:307 | SEQ ID NO:8319 | SEQ ID NO:16331 |
| | | AA | RASQGIRNDLG | TASNLQS | LQHNNYPWT |

FIGURE 49

| | | | | | |
|---|---|---|---|---|---|
| | | | SEQ ID NO:308 | SEQ ID NO:8320 | SEQ ID NO:16332 |
| **iPS:392742** | **21-225_20B2** | NA | CGGGCAAGTCAGGACATTAGAAATGATTTAGGC | GCTGCATCCAGTTTGCAAAGT | CTACAGCATTATAATTACCCTCGGGCG |
| | | | SEQ ID NO:309 | SEQ ID NO:8321 | SEQ ID NO:16333 |
| | | AA | RASQDIRNDLG | AASSLQS | LQHYNYPRA |
| | | | SEQ ID NO:310 | SEQ ID NO:8322 | SEQ ID NO:16334 |
| **iPS:392744** | **21-225_20D5** | NA | CGGGCAAGTCAGGGCATTAGAAATGATTTAGGC | GCTGCATCCAGTTTGCAAAGT | CTACAGCATAATAGTTACCCGTTCACT |
| | | | SEQ ID NO:311 | SEQ ID NO:8323 | SEQ ID NO:16335 |
| | | AA | RASQGIRNDLG | AASSLQS | LQHNSYPFT |
| | | | SEQ ID NO:312 | SEQ ID NO:8324 | SEQ ID NO:16336 |
| **iPS:392746** | **21-225_20H7** | NA | CGGACGAGTCAGGGCATTAACAATTATTTAGTC | GCTGCATCCAGTTTGCAAAGT | CAACAGTATTATAGTTACCCATTCACT |
| | | | SEQ ID NO:313 | SEQ ID NO:8325 | SEQ ID NO:16337 |
| | | AA | RTSQGINNYLV | AASSLQS | QQYYSYPFT |
| | | | SEQ ID NO:314 | SEQ ID NO:8326 | SEQ ID NO:16338 |
| **iPS:392748** | **21-225_20A8** | NA | CGGGCGAGTCAGGGCATTAATAATTATTTAGTC | GCTGCATCCAGTTTGCTGAGT | CAACAGTATAATAGTTACCCGATCACC |
| | | | SEQ ID NO:315 | SEQ ID NO:8327 | SEQ ID NO:16339 |
| | | AA | RASQGINNYLV | AASSLLS | QQYNSYPIT |
| | | | SEQ ID NO:316 | SEQ ID NO:8328 | SEQ ID NO:16340 |
| **iPS:392750** | **21-225_20A10** | NA | CGGGCAAGTCAGGGCATTAGAAATGATTTAGGC | GCTGCATCCAGTTTGCAAAGT | CTACAGCATAATAGATACCCGCTCACT |
| | | | SEQ ID NO:317 | SEQ ID NO:8329 | SEQ ID NO:16341 |
| | | AA | RASQGIRNDLG | AASSLQS | LQHNRYPLT |
| | | | SEQ ID NO:318 | SEQ ID NO:8330 | SEQ ID NO:16342 |
| **iPS:392754** | **21-225_21D3** | NA | CGGGCAAGTCAGAGCATTACTGGTTATTCAAAT | GCTACATACAGTTTGGAAAGT | CAACAGAGTTACAGTACCTCGATCACC |
| | | | SEQ ID NO:319 | SEQ ID NO:8331 | SEQ ID NO:16343 |
| | | AA | RASQSITGYSN | ATYSLES | QQSYSTSIT |

## FIGURE 49

| | | | | | |
|---|---|---|---|---|---|
| | | | SEQ ID NO:320 | SEQ ID NO:8332 | SEQ ID NO:16344 |
| **iPS:392758** | **21-225_21G11** | NA | CGGGCAAGTCAGGGCATTAG AAATGATTTAGGC | ACTGCATCCAGTTTGCAA AGT | CTACAACATAATAATTACCC GTGGACG |
| | | | SEQ ID NO:321 | SEQ ID NO:8333 | SEQ ID NO:16345 |
| | | AA | RASQGIRNDLG | TASSLQS | LQHNNYPWT |
| | | | SEQ ID NO:322 | SEQ ID NO:8334 | SEQ ID NO:16346 |
| **iPS:392760** | **21-225_22G3** | NA | CGGGCAAGTCAGAGCATTAG TAATTATTTAAAT | GCTGCTTCCAGTTTGCAA AGT | CAACAGAGTTTCAGAACCCC CTTTTTCACT |
| | | | SEQ ID NO:323 | SEQ ID NO:8335 | SEQ ID NO:16347 |
| | | AA | RASQSISNYLN | AASSLQS | QQSFRTPFFT |
| | | | SEQ ID NO:324 | SEQ ID NO:8336 | SEQ ID NO:16348 |
| **iPS:392762** | **21-225_22G5** | NA | CGGGCAAGTCAGAACATTAG CAGCTATTTAAAT | GCTGCATCCAGTTTGCAA AAT | CAACAGAGTTACAGAACCCC CTTATTCACT |
| | | | SEQ ID NO:325 | SEQ ID NO:8337 | SEQ ID NO:16349 |
| | | AA | RASQNISSYLN | AASSLQN | QQSYRTPLFT |
| | | | SEQ ID NO:326 | SEQ ID NO:8338 | SEQ ID NO:16350 |
| **iPS:392764** | **21-225_22G10** | NA | CGGGCAAGTCAGAGCATTTT CAGCTATTTAAAT | GCTGCATCCAGTTTACAA AGT | CAACAGAGTTTCAGAACCCC CTTATTCACT |
| | | | SEQ ID NO:327 | SEQ ID NO:8339 | SEQ ID NO:16351 |
| | | AA | RASQSIFSYLN | AASSLQS | QQSFRTPLFT |
| | | | SEQ ID NO:328 | SEQ ID NO:8340 | SEQ ID NO:16352 |
| **iPS:392766** | **21-225_23H4** | NA | CGGGCAAGTCAGAGCATTAG CAGGTATTTAAAT | TCTACATCCAGTTTGCAA AGT | CAACAGAGTTACAGTACCCC CACGTGGACG |
| | | | SEQ ID NO:329 | SEQ ID NO:8341 | SEQ ID NO:16353 |
| | | AA | RASQSISRYLN | STSSLQS | QQSYSTPTWT |
| | | | SEQ ID NO:330 | SEQ ID NO:8342 | SEQ ID NO:16354 |
| **iPS:392768** | **21-225_20B8** | NA | AGGGCCAGTCAGAGTGTTAG CAGCAACTTAGCC | GGTGCATCCACCAGGGC CAGT | CAGCAGTATAATAACTGTCC TCTGACG |
| | | | SEQ ID NO:331 | SEQ ID NO:8343 | SEQ ID NO:16355 |
| | | AA | RASQSVSSNLA | GASTRAS | QQYNNCPLT |

**FIGURE 49**

| | | | | | |
|---|---|---|---|---|---|
| | | | SEQ ID NO:332 | SEQ ID NO:8344 | SEQ ID NO:16356 |
| **iPS:392770** | **21-225_20C10** | NA | CGGGCAAGTCACCATATTAG CAACTATTTAAAT | ACTGCATCCAGTTTGCAA AGT | CAACAGAGTTACACTACCCC CACGTGGACG |
| | | | SEQ ID NO:333 | SEQ ID NO:8345 | SEQ ID NO:16357 |
| | | AA | RASHHISNYLN | TASSLQS | QQSYTTPTWT |
| | | | SEQ ID NO:334 | SEQ ID NO:8346 | SEQ ID NO:16358 |
| **iPS:392772** | **21-225_20E12** | NA | CGGGCAAGTCAGGGCATTAG AAATGATTTAGGC | GCTGCATCCAGTTTACAA AGT | CTACAGCATAATAGTTACCC GTTCACT |
| | | | SEQ ID NO:335 | SEQ ID NO:8347 | SEQ ID NO:16359 |
| | | AA | RASQGIRNDLG | AASSLQS | LQHNSYPFT |
| | | | SEQ ID NO:336 | SEQ ID NO:8348 | SEQ ID NO:16360 |
| **iPS:392774** | **21-225_21F3** | NA | CGGGCAAGTCAGGGCATTAG AAATGATTTAGGC | GCTGCATCCAGTTTGCAA AGT | CTACAGCATAGTAGTTACCC CCTCACT |
| | | | SEQ ID NO:337 | SEQ ID NO:8349 | SEQ ID NO:16361 |
| | | AA | RASQGIRNDLG | AASSLQS | LQHSSYPLT |
| | | | SEQ ID NO:338 | SEQ ID NO:8350 | SEQ ID NO:16362 |
| **iPS:392776** | **21-225_21A12** | NA | CGGGCGAGTCAAGGCATTAG CAAATATTTAGCC | GCTGCATCCAGTTTGCAA AGT | CAACAGTATAATAGTTACCC GTTCAGG |
| | | | SEQ ID NO:339 | SEQ ID NO:8351 | SEQ ID NO:16363 |
| | | AA | RASQGISKYLA | AASSLQS | QQYNSYPFR |
| | | | SEQ ID NO:340 | SEQ ID NO:8352 | SEQ ID NO:16364 |
| **iPS:392778** | **21-225_22H3** | NA | CGGGCAAGTCAGGACATTAG AAATAATTTAGGC | CCTGCATCCAGTTTGCAA ACT | CTACAGGATAATAGTTACCC ATTCACT |
| | | | SEQ ID NO:341 | SEQ ID NO:8353 | SEQ ID NO:16365 |
| | | AA | RASQDIRNNLG | PASSLQT | LQDNSYPFT |
| | | | SEQ ID NO:342 | SEQ ID NO:8354 | SEQ ID NO:16366 |
| **iPS:392780** | **21-225_22B7** | NA | CGGGCAAGTCAGGGCATTAG AAATGATTTAGGC | ACTGCATCCAGTTTGCAA AGT | CTACAGCATAATACTTACCC GCTCACT |
| | | | SEQ ID NO:343 | SEQ ID NO:8355 | SEQ ID NO:16367 |
| | | AA | RASQGIRNDLG | TASSLQS | LQHNTYPLT |

FIGURE 49

| | | | | | |
|---|---|---|---|---|---|
| | | | SEQ ID NO:344 | SEQ ID NO:8356 | SEQ ID NO:16368 |
| **iPS:392782** | **21-225_22B12** | NA | CGGGCGAGTCAGGACATTAG CAATTATTTAGCC | GGTGCATCCAGTTTGCG GAGT | CAACAGTATCATAGTTACCC ATTCACT |
| | | | SEQ ID NO:345 | SEQ ID NO:8357 | SEQ ID NO:16369 |
| | | AA | RASQDISNYLA | GASSLRS | QQYHSYPFT |
| | | | SEQ ID NO:346 | SEQ ID NO:8358 | SEQ ID NO:16370 |
| **iPS:392784** | **21-225_23C7** | NA | CGGGCGAGTCAGGGCATTGG CATTTATTTAGCC | GCTGCATCCAGTTTGCAA AGT | CAACAGTATAATAGTTACCC ATTCACT |
| | | | SEQ ID NO:347 | SEQ ID NO:8359 | SEQ ID NO:16371 |
| | | AA | RASQGIGIYLA | AASSLQS | QQYNSYPFT |
| | | | SEQ ID NO:348 | SEQ ID NO:8360 | SEQ ID NO:16372 |
| **iPS:392786** | **21-225_24E1** | NA | AAGTCCAGCCAGAGTGTTTT ATACACCTCCAACAATAACA ACTACTTAACT | TGGGCATCTACCCGGGA ATCC | CAGCAATTTTATAGTACTCC TCCGACG |
| | | | SEQ ID NO:349 | SEQ ID NO:8361 | SEQ ID NO:16373 |
| | | AA | KSSQSVLYTSNNNNYLT | WASTRES | QQFYSTPPT |
| | | | SEQ ID NO:350 | SEQ ID NO:8362 | SEQ ID NO:16374 |
| **iPS:392788** | **21-225_20C8** | NA | CGGGCAAGTCAGGGCATTAG AAATGATTTAGGC | GCTGCATCCAGTTTGCAA AGT | CTACAAGATAATAGTTACCC GTTCACT |
| | | | SEQ ID NO:351 | SEQ ID NO:8363 | SEQ ID NO:16375 |
| | | AA | RASQGIRNDLG | AASSLQS | LQDNSYPFT |
| | | | SEQ ID NO:352 | SEQ ID NO:8364 | SEQ ID NO:16376 |
| **iPS:392790** | **21-225_20D10** | NA | CGGGCAAGTCAGGGCATTAG AAATGATTTAGGC | GTTGCATACAGTTTGCAA AGT | ATACAGCAAAATAGTTACCC GTGGACG |
| | | | SEQ ID NO:353 | SEQ ID NO:8365 | SEQ ID NO:16377 |
| | | AA | RASQGIRNDLG | VAYSLQS | IQQNSYPWT |
| | | | SEQ ID NO:354 | SEQ ID NO:8366 | SEQ ID NO:16378 |
| **iPS:392792** | **21-225_20G12** | NA | CGGGCGAGTCAGGGCATTAG CAATTATTTAGCC | ACTGCATCCACTTTGCAA TCA | CAAAAGTATAACAGTGCCCC TCCGATCACC |
| | | | SEQ ID NO:355 | SEQ ID NO:8367 | SEQ ID NO:16379 |

FIGURE 49

| | | | | | |
|---|---|---|---|---|---|
| | | AA | RASQGISNYLA | TASTLQS | QKYNSAPPIT |
| | | | SEQ ID NO:356 | SEQ ID NO:8368 | SEQ ID NO:16380 |
| **iPS:392794** | **21-225_21H3** | NA | CGGGCAAGTCAGGGCATTAG AAATGATTTAGGC | GCTGCGTCCAGTGTGCA AACT | CTACAGCATAATAGTTATCC GCTCACT |
| | | | SEQ ID NO:357 | SEQ ID NO:8369 | SEQ ID NO:16381 |
| | | AA | RASQGIRNDLG | AASSVQT | LQHNSYPLT |
| | | | SEQ ID NO:358 | SEQ ID NO:8370 | SEQ ID NO:16382 |
| **iPS:392796** | **21-225_22A4** | NA | CGGGCAAGTCAGGGCATTAG AAATGATTTAGGC | GCTGCATCCAGTTTGCAA AGT | CTTCAGCATAATAGTTACCC GTGGACG |
| | | | SEQ ID NO:359 | SEQ ID NO:8371 | SEQ ID NO:16383 |
| | | AA | RASQGIRNDLG | AASSLQS | LQHNSYPWT |
| | | | SEQ ID NO:360 | SEQ ID NO:8372 | SEQ ID NO:16384 |
| **iPS:392798** | **21-225_22C7** | NA | CGGGCAAGTCAGAACATTAT CAGCTATTTAAAT | ATTGCATCCAGTTTGCAA AGT | CAACAGACTTACAGTACCCC GCTCACT |
| | | | SEQ ID NO:361 | SEQ ID NO:8373 | SEQ ID NO:16385 |
| | | AA | RASQNIISYLN | IASSLQS | QQTYSTPLT |
| | | | SEQ ID NO:362 | SEQ ID NO:8374 | SEQ ID NO:16386 |
| **iPS:392800** | **21-225_22D12** | NA | CGGGCAAGTCAGGGCATTAG AAATGATTTAGGC | GCTGCATCCAGTTTGCAG AGT | CTACAGCATAGTACTTACCC TCTCACT |
| | | | SEQ ID NO:363 | SEQ ID NO:8375 | SEQ ID NO:16387 |
| | | AA | RASQGIRNDLG | AASSLQS | LQHSTYPLT |
| | | | SEQ ID NO:364 | SEQ ID NO:8376 | SEQ ID NO:16388 |
| **iPS:392802** | **21-225_23E7** | NA | CGGGCGAGTCAGGGCATTAG CAATTATTTAGCC | GCTGCATCCAGTTTGCAA AGT | CAACAGTTTTATAGTTACCC ATTCACT |
| | | | SEQ ID NO:365 | SEQ ID NO:8377 | SEQ ID NO:16389 |
| | | AA | RASQGISNYLA | AASSLQS | QQFYSYPFT |
| | | | SEQ ID NO:366 | SEQ ID NO:8378 | SEQ ID NO:16390 |
| **iPS:392806** | **21-225_24H3** | NA | AGGGCCAGTCAGAGTGTTAG CAGCAACTTAGCC | TTTGCATCCATCAGGGCC ACT | CAGCAGTATAATAACTGGCC CATGTGCAGT |
| | | | SEQ ID NO:367 | SEQ ID NO:8379 | SEQ ID NO:16391 |

FIGURE 49

| | | | | | |
|---|---|---|---|---|---|
| | | AA | RASQSVSSNLA | FASIRAT | QQYNNWPMCS |
| | | | SEQ ID NO:368 | SEQ ID NO:8380 | SEQ ID NO:16392 |
| **iPS:392808** | **21-225_20F8** | NA | CGGGCAAGTCAGAGCATTAG CAGGTATTTAAAT | GCTGCTTCCAGTTTGCAA AGT | CAACAGAGTTACAATACCCC CACGTGGACG |
| | | | SEQ ID NO:369 | SEQ ID NO:8381 | SEQ ID NO:16393 |
| | | AA | RASQSISRYLN | AASSLQS | QQSYNTPTWT |
| | | | SEQ ID NO:370 | SEQ ID NO:8382 | SEQ ID NO:16394 |
| **iPS:392810** | **21-225_20H12** | NA | CGGGCAAGTCAGGGCATTAG AAATGATTTAGAC | GCTGCATCCAGTTTGCAA AGT | CTACAGCATAATAGTTACCC GCTCACT |
| | | | SEQ ID NO:371 | SEQ ID NO:8383 | SEQ ID NO:16395 |
| | | AA | RASQGIRNDLD | AASSLQS | LQHNSYPLT |
| | | | SEQ ID NO:372 | SEQ ID NO:8384 | SEQ ID NO:16396 |
| **iPS:392812** | **21-225_21F4** | NA | CGGGCAAGTCAGAACATTGG TAGTTATTTAAAT | GCTGCATCCAGTTTGCAA AGT | CAACAGAGTTACAGAACCCC CTTTTTCACT |
| | | | SEQ ID NO:373 | SEQ ID NO:8385 | SEQ ID NO:16397 |
| | | AA | RASQNIGSYLN | AASSLQS | QQSYRTPFFT |
| | | | SEQ ID NO:374 | SEQ ID NO:8386 | SEQ ID NO:16398 |
| **iPS:392814** | **21-225_22A1** | NA | AAGTCTAGTCAGAGCCTCCT GCATAGTGGTGGAAAGACCT ATTTATAT | GAAGTTTCCAACCGGTTC TCT | ATGCAAACTTTACACCTTCC GTGGACG |
| | | | SEQ ID NO:375 | SEQ ID NO:8387 | SEQ ID NO:16399 |
| | | AA | KSSQSLLHSGGKTYLY | EVSNRFS | MQTLHLPWT |
| | | | SEQ ID NO:376 | SEQ ID NO:8388 | SEQ ID NO:16400 |
| **iPS:392816** | **21-225_22E4** | NA | CGGGCAAGTCAGAACATTAG TAGTTATTTAAAT | GCTGCATCCAGTTTGCAA AGT | CAACAGAGTTACAATACCCC CTTATTCACT |
| | | | SEQ ID NO:377 | SEQ ID NO:8389 | SEQ ID NO:16401 |
| | | AA | RASQNISSYLN | AASSLQS | QQSYNTPLFT |
| | | | SEQ ID NO:378 | SEQ ID NO:8390 | SEQ ID NO:16402 |
| **iPS:392818** | | NA | CGGACAAGTCAGAACAGTA ACAGTTATTTAAAT | GCTGCATACAGTTTGGA AAGT | CAACAGACTTACGGTACCTC GATCACC |

FIGURE 49

| | | | | | |
|---|---|---|---|---|---|
| | **21-225_22D8** | | SEQ ID NO:379 | SEQ ID NO:8391 | SEQ ID NO:16403 |
| | | AA | RTSQNSNSYLN | AAYSLES | QQTYGTSIT |
| | | | SEQ ID NO:380 | SEQ ID NO:8392 | SEQ ID NO:16404 |
| **iPS:392820** | **21-225_23D1** | NA | CGGGCAAGTCAGGGCATCA GAAATGATTTAGGC | GCTGCATCCAGTTTACAA AGT | CTACAGCATAGTAGTTACCC TCTCACT |
| | | | SEQ ID NO:381 | SEQ ID NO:8393 | SEQ ID NO:16405 |
| | | AA | RASQGIRNDLG | AASSLQS | LQHSSYPLT |
| | | | SEQ ID NO:382 | SEQ ID NO:8394 | SEQ ID NO:16406 |
| **iPS:392822** | **21-225_23C8** | NA | CGGGCAAGTCAGGACATTAG AAATGATTTAGGC | GGTGCATCCAGTGTGCA AAGT | CTACAGCATAATAGTTACCC GCTCACT |
| | | | SEQ ID NO:383 | SEQ ID NO:8395 | SEQ ID NO:16407 |
| | | AA | RASQDIRNDLG | GASSVQS | LQHNSYPLT |
| | | | SEQ ID NO:384 | SEQ ID NO:8396 | SEQ ID NO:16408 |
| **iPS:392824** | **21-225_24E5** | NA | CGGGCAAGTCAGGGCATTAG AAATGATTTAGGC | GCTGCATCCAGTTTGCAA AGT | CTACAGCATAGTAATTACCC GCTCACT |
| | | | SEQ ID NO:385 | SEQ ID NO:8397 | SEQ ID NO:16409 |
| | | AA | RASQGIRNDLG | AASSLQS | LQHSNYPLT |
| | | | SEQ ID NO:386 | SEQ ID NO:8398 | SEQ ID NO:16410 |
| **iPS:392826** | **21-225_20B9** | NA | CGGGCGAGTCAGGGCATTAG CAATTATTTAGCC | GTTGCATCCAGTTTGCAA AGT | CAACAGTATAATACTTATCC ATTCACT |
| | | | SEQ ID NO:387 | SEQ ID NO:8399 | SEQ ID NO:16411 |
| | | AA | RASQGISNYLA | VASSLQS | QQYNTYPFT |
| | | | SEQ ID NO:388 | SEQ ID NO:8400 | SEQ ID NO:16412 |
| **iPS:392830** | **21-225_21A5** | NA | CGGGCAAGTCAGACCATTAG CAGCCATTTAAAT | GCTGCATCCAGTTTGCAA AGT | CAACAGAGTTACAATATCTC ATTCACT |
| | | | SEQ ID NO:389 | SEQ ID NO:8401 | SEQ ID NO:16413 |
| | | AA | RASQTISSHLN | AASSLQS | QQSYNISFT |
| | | | SEQ ID NO:390 | SEQ ID NO:8402 | SEQ ID NO:16414 |
| **iPS:392832** | | NA | CGGGCAAGTCAGGGCATTAG AAATGATTTAGGC | GCTGCATCCAGTTTACAA AGT | CTACAGCATAATAGTTACCC GTGGACG |

FIGURE 49

| | | | | | |
|---|---|---|---|---|---|
| | **21-225_21H8** | | SEQ ID NO:391 | SEQ ID NO:8403 | SEQ ID NO:16415 |
| | | AA | RASQGIRNDLG | AASSLQS | LQHNSYPWT |
| | | | SEQ ID NO:392 | SEQ ID NO:8404 | SEQ ID NO:16416 |
| **iPS:392834** | **21-225_22C1** | NA | CGGACAAGTCAGGGCATTAG AAATGATTTAGGC | GCTGCATCCAGTTTGCAA AGT | CTACAGCATAGTACTTACCC GCTCACT |
| | | | SEQ ID NO:393 | SEQ ID NO:8405 | SEQ ID NO:16417 |
| | | AA | RTSQGIRNDLG | AASSLQS | LQHSTYPLT |
| | | | SEQ ID NO:394 | SEQ ID NO:8406 | SEQ ID NO:16418 |
| **iPS:392836** | **21-225_22F4** | NA | CGGGCAAGTCAGGTCATTAG AGATGATTTAGGC | GCTGCATCCAGTTTGCAA AGT | CTACACCACTATAGTTATCC TCGGACG |
| | | | SEQ ID NO:395 | SEQ ID NO:8407 | SEQ ID NO:16419 |
| | | AA | RASQVIRDDLG | AASSLQS | LHHYSYPRT |
| | | | SEQ ID NO:396 | SEQ ID NO:8408 | SEQ ID NO:16420 |
| **iPS:392838** | **21-225_22G8** | NA | CGGACAAGTCAGGGCATTAG AAATGATTTAGGC | GCTGCATCCAGTTTGCAA AGT | CTACAGCATAATAGTTACCC GCTCACT |
| | | | SEQ ID NO:397 | SEQ ID NO:8409 | SEQ ID NO:16421 |
| | | AA | RTSQGIRNDLG | AASSLQS | LQHNSYPLT |
| | | | SEQ ID NO:398 | SEQ ID NO:8410 | SEQ ID NO:16422 |
| **iPS:392840** | **21-225_23G1** | NA | CGGGCGAGTCAGGGCATTAG CAATTATTTAGCC | GCTGCATCCAGTTTACAG AGT | CAACAGTATAATAGTTACCC ATTCACT |
| | | | SEQ ID NO:399 | SEQ ID NO:8411 | SEQ ID NO:16423 |
| | | AA | RASQGISNYLA | AASSLQS | QQYNSYPFT |
| | | | SEQ ID NO:400 | SEQ ID NO:8412 | SEQ ID NO:16424 |
| **iPS:392842** | **21-225_23G8** | NA | CGGGCGAGTCAGGGCATTAG AAATTATTTAGCC | GCTGCATCCAGTTTGCAA AGT | CAACAGTATAATAGTTACCC TTTCACT |
| | | | SEQ ID NO:401 | SEQ ID NO:8413 | SEQ ID NO:16425 |
| | | AA | RASQGIRNYLA | AASSLQS | QQYNSYPFT |
| | | | SEQ ID NO:402 | SEQ ID NO:8414 | SEQ ID NO:16426 |
| **iPS:392844** | | NA | CGGGCAAGTCAGGGCATTAG AAATGATTTAGGC | CCTGCATCCAGTTTGCAA AGT | CTACAGCATAATAGTTACCC GTGGACG |

FIGURE 49

| | | | | | |
|---|---|---|---|---|---|
| | **21-225_23E11** | | SEQ ID NO:403 | SEQ ID NO:8415 | SEQ ID NO:16427 |
| | | AA | RASQGIRNDLG | PASSLQS | LQHNSYPWT |
| | | | SEQ ID NO:404 | SEQ ID NO:8416 | SEQ ID NO:16428 |
| **iPS:392846** | **21-225_24B6** | NA | CGGGCAAGTCAGGACATTAG AAATGATTTAGGC | GCTGCATCCAGTTTGCAC AGT | CTACAGCATTATAGTTACCC TCGGACG |
| | | | SEQ ID NO:405 | SEQ ID NO:8417 | SEQ ID NO:16429 |
| | | AA | RASQDIRNDLG | AASSLHS | LQHYSYPRT |
| | | | SEQ ID NO:406 | SEQ ID NO:8418 | SEQ ID NO:16430 |
| **iPS:392848** | **21-225_20F9** | NA | CGGGCGAGTCAGGGCATTAG CAATTATTTAGCC | GCTGCATCCAGTTTGCAA AGT | CAACAGTATCATAGTTACCC GTGGACG |
| | | | SEQ ID NO:407 | SEQ ID NO:8419 | SEQ ID NO:16431 |
| | | AA | RASQGISNYLA | AASSLQS | QQYHSYPWT |
| | | | SEQ ID NO:408 | SEQ ID NO:8420 | SEQ ID NO:16432 |
| **iPS:392850** | **21-225_20H10** | NA | CGGGCAAGTCAGGGCATTAA AAATAATTTAGGC | GCTGCATCCAGTTTGCAA AGT | CTACAGCATAATAGTTACCC GCTCACT |
| | | | SEQ ID NO:409 | SEQ ID NO:8421 | SEQ ID NO:16433 |
| | | AA | RASQGIKNNLG | AASSLQS | LQHNSYPLT |
| | | | SEQ ID NO:410 | SEQ ID NO:8422 | SEQ ID NO:16434 |
| **iPS:392852** | **21-225_21A2** | NA | CGGGCAAGTCAGAGTATTAG TAGTTATTTAAAT | GCTGCATCCAGTTTGCAA AGT | CAACAGAGTTACAGAACCCC CTTTTTCACT |
| | | | SEQ ID NO:411 | SEQ ID NO:8423 | SEQ ID NO:16435 |
| | | AA | RASQSISSYLN | AASSLQS | QQSYRTPFFT |
| | | | SEQ ID NO:412 | SEQ ID NO:8424 | SEQ ID NO:16436 |
| **iPS:392854** | **21-225_21E5** | NA | CGGGCAAGTCAGGGCATTAG AAATGATTTAGGC | GCTACATCCAGTTTGCAA AGT | CTACAGCATAATAGTTACCC CCTCACT |
| | | | SEQ ID NO:413 | SEQ ID NO:8425 | SEQ ID NO:16437 |
| | | AA | RASQGIRNDLG | ATSSLQS | LQHNSYPLT |
| | | | SEQ ID NO:414 | SEQ ID NO:8426 | SEQ ID NO:16438 |
| **iPS:392856** | | NA | CGGGCGAGTCAGGACATTAG CAATTATTTAGCC | GCTGCATCCAGTTTGCAA AGT | CAACAGTATAATAGTTTCCC GCTCACT |

FIGURE 49

| | | | | | |
|---|---|---|---|---|---|
| | 21-225_22A2 | | SEQ ID NO:415 | SEQ ID NO:8427 | SEQ ID NO:16439 |
| | | AA | RASQDISNYLA | AASSLQS | QQYNSFPLT |
| | | | SEQ ID NO:416 | SEQ ID NO:8428 | SEQ ID NO:16440 |
| **iPS:392858** | **21-225_22H4** | NA | CGGGCAAGTCAGGGCATTAG AAATGATTTAGGC | GCTGCATCCAGTGTGCA AAGT | CTACAGCATAATAGTTACCC GCTCACT |
| | | | SEQ ID NO:417 | SEQ ID NO:8429 | SEQ ID NO:16441 |
| | | AA | RASQGIRNDLG | AASSVQS | LQHNSYPLT |
| | | | SEQ ID NO:418 | SEQ ID NO:8430 | SEQ ID NO:16442 |
| **iPS:392860** | **21-225_22H8** | NA | AAGTCTAGTCAGAGCCTCCT GCATAGTGATGGAAAGACCT TTTTGTAT | GAAGTTTCCAACCGGTTC TCT | ATGCAAAGTATACAGCTTCC GCTCTCA |
| | | | SEQ ID NO:419 | SEQ ID NO:8431 | SEQ ID NO:16443 |
| | | AA | KSSQSLLHSDGKTFLY | EVSNRFS | MQSIQLPLS |
| | | | SEQ ID NO:420 | SEQ ID NO:8432 | SEQ ID NO:16444 |
| **iPS:392864** | **21-225_23B9** | NA | AGGGCCAGTCAGAATGTTTA CAGCAGCTACTTAGCC | GGTGCATCCAGCAGGGC CAGT | CAGCAGTATGGTAGCTCACC TCGGACG |
| | | | SEQ ID NO:421 | SEQ ID NO:8433 | SEQ ID NO:16445 |
| | | AA | RASQNVYSSYLA | GASSRAS | QQYGSSPRT |
| | | | SEQ ID NO:422 | SEQ ID NO:8434 | SEQ ID NO:16446 |
| **iPS:392866** | **21-225_23H11** | NA | CGGGCAAGTCAGGGCATTAG AAATGATTTAGAC | GCTGCATCCAGTTTGCAA AGT | CTACAGCATAATCGTTACCC GCTCACT |
| | | | SEQ ID NO:423 | SEQ ID NO:8435 | SEQ ID NO:16447 |
| | | AA | RASQGIRNDLD | AASSLQS | LQHNRYPLT |
| | | | SEQ ID NO:424 | SEQ ID NO:8436 | SEQ ID NO:16448 |
| **iPS:392868** | **21-225_24D6** | NA | CAGGCGAGTCAGGACATTAA CAACTATTTAAAT | GATGCATCCGATTTGGA AACA | CAACAGTATGAAAATCTCCC GATCACC |
| | | | SEQ ID NO:425 | SEQ ID NO:8437 | SEQ ID NO:16449 |
| | | AA | QASQDINNYLN | DASDLET | QQYENLPIT |
| | | | SEQ ID NO:426 | SEQ ID NO:8438 | SEQ ID NO:16450 |

FIGURE 49

| | | | | | |
|---|---|---|---|---|---|
| **iPS:392870** | **21-225_20G9** | NA | CGGGCAAGTCAGGGCATTAG AAATGATTTAGGC | GCTGCATCCAGTTTGCAA AGT | CTACAGCATAGTACTTACCC TCTCACT |
| | | | SEQ ID NO:427 | SEQ ID NO:8439 | SEQ ID NO:16451 |
| | | AA | RASQGIRNDLG | AASSLQS | LQHSTYPLT |
| | | | SEQ ID NO:428 | SEQ ID NO:8440 | SEQ ID NO:16452 |
| **iPS:392872** | **21-225_20B11** | NA | CGGGCAAGTCAGGGCATTGG AAATGATTTAGGC | GCTGCATCCAGTTTGCAT AGT | CTACAGCATTATAGTTACCC TCGGACG |
| | | | SEQ ID NO:429 | SEQ ID NO:8441 | SEQ ID NO:16453 |
| | | AA | RASQGIGNDLG | AASSLHS | LQHYSYPRT |
| | | | SEQ ID NO:430 | SEQ ID NO:8442 | SEQ ID NO:16454 |
| **iPS:392874** | **21-225_21D2** | NA | CGGGCAAGTCAGAGCATTAG CGACTATTTAAAT | GATACATCCAGTTTGCA AAGT | CAACAGACTTACAATATTCT TCCGGAGCGCAGT |
| | | | SEQ ID NO:431 | SEQ ID NO:8443 | SEQ ID NO:16455 |
| | | AA | RASQSISDYLN | DTSSLQS | QQTYNILPERS |
| | | | SEQ ID NO:432 | SEQ ID NO:8444 | SEQ ID NO:16456 |
| **iPS:392876** | **21-225_21F7** | NA | CGGGCAAGTCAGGACATTAG AAATGATTTAGGC | GCTGCATCCAATTTTCAA AGT | CTACAGCATAATAATTACCC GTGGACG |
| | | | SEQ ID NO:433 | SEQ ID NO:8445 | SEQ ID NO:16457 |
| | | AA | RASQDIRNDLG | AASNFQS | LQHNNYPWT |
| | | | SEQ ID NO:434 | SEQ ID NO:8446 | SEQ ID NO:16458 |
| **iPS:392878** | **21-225_22C5** | NA | CGGGCAAGTCAGAACATTAG CAGCTATTTAAAT | GCTGCATCCGTTTTGCAA CAT | CAACAGAGTTACAGAACCCC CTTATTCACT |
| | | | SEQ ID NO:435 | SEQ ID NO:8447 | SEQ ID NO:16459 |
| | | AA | RASQNISSYLN | AASVLQH | QQSYRTPLFT |
| | | | SEQ ID NO:436 | SEQ ID NO:8448 | SEQ ID NO:16460 |
| **iPS:392880** | **21-225_22F9** | NA | CGGGCAAGTCAGGGCATTAG AAATGATTTAGGC | GCTGCATCCAGTTTACAA AGT | CTACAGCATAATAGTTACCC GCTCACT |
| | | | SEQ ID NO:437 | SEQ ID NO:8449 | SEQ ID NO:16461 |
| | | AA | RASQGIRNDLG | AASSLQS | LQHNSYPLT |
| | | | SEQ ID NO:438 | SEQ ID NO:8450 | SEQ ID NO:16462 |

FIGURE 49

| | | | | | |
|---|---|---|---|---|---|
| **iPS:392882** | **21-225_23A3** | NA | CGGGCAAGTCAGGGCATTAGAAATGATTTAGGC | GCTGCATCCAGTGTGCAAAGT | CTACAGCATAATAGTTACCCGCTCACT |
| | | | SEQ ID NO:439 | SEQ ID NO:8451 | SEQ ID NO:16463 |
| | | AA | RASQGIRNDLG | AASSVQS | LQHNSYPLT |
| | | | SEQ ID NO:440 | SEQ ID NO:8452 | SEQ ID NO:16464 |
| **iPS:392884** | **21-225_23A10** | NA | CGGGCAAGTCAGGGCATTAGAAATGATTTAGGC | GCTGCATCCAGTTTGCAAAGT | CTGCAACATTATAGTTACCCTCGGACG |
| | | | SEQ ID NO:441 | SEQ ID NO:8453 | SEQ ID NO:16465 |
| | | AA | RASQGIRNDLG | AASSLQS | LQHYSYPRT |
| | | | SEQ ID NO:442 | SEQ ID NO:8454 | SEQ ID NO:16466 |
| **iPS:392886** | **21-225_23A12** | NA | AAGTCCAGCCAGAGTGTTTTATACAGCTCCAACAATAACAATTATTTAGCT | TGGACATCTACCCGGGAATCC | CAGCAGTATTATGATACTCCTCCGACG |
| | | | SEQ ID NO:443 | SEQ ID NO:8455 | SEQ ID NO:16467 |
| | | AA | KSSQSVLYSSNNNNYLA | WTSTRES | QQYYDTPPT |
| | | | SEQ ID NO:444 | SEQ ID NO:8456 | SEQ ID NO:16468 |
| **iPS:392888** | **21-225_25A2** | NA | AAGTCTAGTCAGAGCCTCCTACATAGTGAAGGAAAGACCTATTTGTAT | GAAATTTCCAACCGGTTCTCT | ATGCAAAGTACACAGTTTCCGCTCACT |
| | | | SEQ ID NO:445 | SEQ ID NO:8457 | SEQ ID NO:16469 |
| | | AA | KSSQSLLHSEGKTYLY | EISNRFS | MQSTQFPLT |
| | | | SEQ ID NO:446 | SEQ ID NO:8458 | SEQ ID NO:16470 |
| **iPS:392890** | **21-225_20H9** | NA | CGGGCGAGTCAGGGCATTAGCAATTATTTAGCC | GCTGCATCCAGTTTGCAAAGT | CAACAGTATAATAGTTACCCATTCACT |
| | | | SEQ ID NO:447 | SEQ ID NO:8459 | SEQ ID NO:16471 |
| | | AA | RASQGISNYLA | AASSLQS | QQYNSYPFT |
| | | | SEQ ID NO:448 | SEQ ID NO:8460 | SEQ ID NO:16472 |
| **iPS:392892** | **21-225_20C11** | NA | CGGGCGAGTCAGGACATTAGCAATTATTTAGCC | GCTGCATCCAGTTTGCAAAGT | CAACAGTATCATAGTTTCCCATTCACT |
| | | | SEQ ID NO:449 | SEQ ID NO:8461 | SEQ ID NO:16473 |

**FIGURE 49**

| | | | | | |
|---|---|---|---|---|---|
| | | AA | RASQDISNYLA | AASSLQS | QQYHSFPFT |
| | | | SEQ ID NO:450 | SEQ ID NO:8462 | SEQ ID NO:16474 |
| **iPS:392894** | **21-225_21G2** | NA | CGGGCAAGTCAGGGCATTAG AAATGATTTAGGC | ACTTCATCCAGTTTGCAA AGT | CTACAACATAATAGTTACCC GTGGACG |
| | | | SEQ ID NO:451 | SEQ ID NO:8463 | SEQ ID NO:16475 |
| | | AA | RASQGIRNDLG | TSSSLQS | LQHNSYPWT |
| | | | SEQ ID NO:452 | SEQ ID NO:8464 | SEQ ID NO:16476 |
| **iPS:392896** | **21-225_21G7** | NA | CGGGCAAGTCAGGGCGTTAG AAATGATTTAGGC | GCTGCATCCAGTTTGCAA AGT | CTACAGCATAGTAGTTACCC GCTCACT |
| | | | SEQ ID NO:453 | SEQ ID NO:8465 | SEQ ID NO:16477 |
| | | AA | RASQGVRNDLG | AASSLQS | LQHSSYPLT |
| | | | SEQ ID NO:454 | SEQ ID NO:8466 | SEQ ID NO:16478 |
| **iPS:392898** | **21-225_21H10** | NA | AGGGCCAGTCAGAGTTTTAG CAGCAGCTACTTAGCC | GGTGCATCCAGCAGGGC CACT | CAGCAGTATGGTAGCTCACG CAGT |
| | | | SEQ ID NO:455 | SEQ ID NO:8467 | SEQ ID NO:16479 |
| | | AA | RASQSFSSSYLA | GASSRAT | QQYGSSRS |
| | | | SEQ ID NO:456 | SEQ ID NO:8468 | SEQ ID NO:16480 |
| **iPS:392900** | **21-225_22F2** | NA | CGGGCAAGTCAGGGCATTAG AAATGATTTAGGC | GCTGCATCCAGTTTACAA AGT | CTACAGCATAATAGTTACCC GCTCACT |
| | | | SEQ ID NO:457 | SEQ ID NO:8469 | SEQ ID NO:16481 |
| | | AA | RASQGIRNDLG | AASSLQS | LQHNSYPLT |
| | | | SEQ ID NO:458 | SEQ ID NO:8470 | SEQ ID NO:16482 |
| **iPS:392902** | **21-225_22D5** | NA | CGGGCAAGTCAGAACATTTT TAGTTATTTAAAT | GCTGCATCCAGTTTGCAA AGT | CAACAGAGTTACAGTACCCC CTTATTCACT |
| | | | SEQ ID NO:459 | SEQ ID NO:8471 | SEQ ID NO:16483 |
| | | AA | RASQNIFSYLN | AASSLQS | QQSYSTPLFT |
| | | | SEQ ID NO:460 | SEQ ID NO:8472 | SEQ ID NO:16484 |
| **iPS:392904** | **21-225_22G9** | NA | CGGGCAAGTCAGGGCATTAG AAATGATTTAGGC | GCTGCTTCCAGTTTGCAA AGT | CTACAGCATGCCAGTTACCC GCTCACT |
| | | | SEQ ID NO:461 | SEQ ID NO:8473 | SEQ ID NO:16485 |

FIGURE 49

| | | | | | |
|---|---|---|---|---|---|
| | | AA | RASQGIRNDLG | AASSLQS | LQHASYPLT |
| | | | SEQ ID NO:462 | SEQ ID NO:8474 | SEQ ID NO:16486 |
| **iPS:392908** | **21-225_23F12** | NA | CGGGCAAGTCAGGGCATTAG AAATGATTTAGGC | GTTGCATCCAGTTTGCAA AGT | CTACAGCATAATAGTTACCC GTGGACG |
| | | | SEQ ID NO:463 | SEQ ID NO:8475 | SEQ ID NO:16487 |
| | | AA | RASQGIRNDLG | VASSLQS | LQHNSYPWT |
| | | | SEQ ID NO:464 | SEQ ID NO:8476 | SEQ ID NO:16488 |
| **iPS:392912** | **21-225_25A9** | NA | CGGGCAAGTCAGGGCATTAG AAATGATTTAGGC | GCTGCATCCAGTTTGCAA AGT | CTACAGCATAATAGTTACCC ATTCACT |
| | | | SEQ ID NO:465 | SEQ ID NO:8477 | SEQ ID NO:16489 |
| | | AA | RASQGIRNDLG | AASSLQS | LQHNSYPFT |
| | | | SEQ ID NO:466 | SEQ ID NO:8478 | SEQ ID NO:16490 |
| **iPS:392914** | **21-225_25D12** | NA | CGGACAAGTCAGGGCATTAG AAATGATTTAGGC | GCTGCATCCAGTTTACAA AGT | CTACAGCATTATAGTTTCCCT CGGACG |
| | | | SEQ ID NO:467 | SEQ ID NO:8479 | SEQ ID NO:16491 |
| | | AA | RTSQGIRNDLG | AASSLQS | LQHYSFPRT |
| | | | SEQ ID NO:468 | SEQ ID NO:8480 | SEQ ID NO:16492 |
| **iPS:392916** | **21-225_27C5** | NA | CGGGCGAGTCAGGGTATTAG CAGCTGGTTAGCC | GGTGCATCCAGTTTGCA AAGT | CAACAGTATGACAGTTTCCC TCGGACG |
| | | | SEQ ID NO:469 | SEQ ID NO:8481 | SEQ ID NO:16493 |
| | | AA | RASQGISSWLA | GASSLQS | QQYDSFPRT |
| | | | SEQ ID NO:470 | SEQ ID NO:8482 | SEQ ID NO:16494 |
| **iPS:392918** | **21-225_28F5** | NA | CGGGCAAGTCAGGGCATTAG AAATGATTTAGGC | ACTGCATCCAGTTTGCAA AGT | CTACAGCATAATACTTACCC GTGGACG |
| | | | SEQ ID NO:471 | SEQ ID NO:8483 | SEQ ID NO:16495 |
| | | AA | RASQGIRNDLG | TASSLQS | LQHNTYPWT |
| | | | SEQ ID NO:472 | SEQ ID NO:8484 | SEQ ID NO:16496 |
| **iPS:392920** | **21-225_29G4** | NA | CGGGCAAGTCAGGGCATTAG AAATGATTTAGGC | GCTGCATCCAGTTTGCAA AGT | CTACAGCATAATAGTTACCC GCTCACT |
| | | | SEQ ID NO:473 | SEQ ID NO:8485 | SEQ ID NO:16497 |

FIGURE 49

| | | | | | |
|---|---|---|---|---|---|
| | | AA | RASQGIRNDLG | AASSLQS | LQHNSYPLT |
| | | | SEQ ID NO:474 | SEQ ID NO:8486 | SEQ ID NO:16498 |
| **iPS:392922** | **21-225_30G4** | NA | CGGGCAACTCAGAACATTTTCAGCTATTTAAAT | ACTGCATCCAGTTTGCAAGGT | CAACTCAGCTACAGTCCCCCGTACACT |
| | | | SEQ ID NO:475 | SEQ ID NO:8487 | SEQ ID NO:16499 |
| | | AA | RATQNIFSYLN | TASSLQG | QLSYSPPYT |
| | | | SEQ ID NO:476 | SEQ ID NO:8488 | SEQ ID NO:16500 |
| **iPS:392924** | **21-225_32H2** | NA | AGGTCTAGTCAGAGCCTCCTCCATAGTGATGGAAGGACCTATTTGTAT | GAACTTTCCAACCGGTTCTCT | TTGCAAAGTATACAATATCCCATCACC |
| | | | SEQ ID NO:477 | SEQ ID NO:8489 | SEQ ID NO:16501 |
| | | AA | RSSQSLLHSDGRTYLY | ELSNRFS | LQSIQYPIT |
| | | | SEQ ID NO:478 | SEQ ID NO:8490 | SEQ ID NO:16502 |
| **iPS:392928** | **21-225_25A4** | NA | AAGTCCAGCCAGAGTGTTTTATACAGCTCCCACAATAACAACTACTTAGCT | TGGGCATCTACCCGGGAATCC | CAGCAGTATTATAGTACTCCTCCGACG |
| | | | SEQ ID NO:479 | SEQ ID NO:8491 | SEQ ID NO:16503 |
| | | AA | KSSQSVLYSSHNNNYLA | WASTRES | QQYYSTPPT |
| | | | SEQ ID NO:480 | SEQ ID NO:8492 | SEQ ID NO:16504 |
| **iPS:392930** | **21-225_25H9** | NA | AAGTCTAGTCAGAGCCTCCTGCATGGTGATGGAAAGACCTATTTGTTT | GAAGTTTCCAATCGGTTCTCT | ATGCAAAGTATACAGCTTCCGTGGACG |
| | | | SEQ ID NO:481 | SEQ ID NO:8493 | SEQ ID NO:16505 |
| | | AA | KSSQSLLHGDGKTYLF | EVSNRFS | MQSIQLPWT |
| | | | SEQ ID NO:482 | SEQ ID NO:8494 | SEQ ID NO:16506 |
| **iPS:392934** | **21-225_27D5** | NA | CGGGCAAGTCAGGGCATTAGAAATGATTTAGGC | GCTGCATCCAGTTTGCAAAGT | CTACAGCATAATAGTTACCCATTCACT |
| | | | SEQ ID NO:483 | SEQ ID NO:8495 | SEQ ID NO:16507 |
| | | AA | RASQGIRNDLG | AASSLQS | LQHNSYPFT |
| | | | SEQ ID NO:484 | SEQ ID NO:8496 | SEQ ID NO:16508 |

FIGURE 49

| | | | | | |
|---|---|---|---|---|---|
| **iPS:392936** | **21-225_28B6** | NA | CGGTCTAGTCAAAGCCTCGTATATAGTGATGGAGACACCTACTTGAAT | AAGGTTTCTAACTGGGACTCT | ATGCATTGTACACACTGGCTCCTT |
| | | | SEQ ID NO:485 | SEQ ID NO:8497 | SEQ ID NO:16509 |
| | | AA | RSSQSLVYSDGDTYLN | KVSNWDS | MHCTHWLL |
| | | | SEQ ID NO:486 | SEQ ID NO:8498 | SEQ ID NO:16510 |
| **iPS:392938** | **21-225_29H4** | NA | AAGTCTAGTCAGAGCCTCCTGCATAGTGATGGAAAGACCTATTTGTAT | GAGGTTTCCCACCGGTTCTCT | ATGCAAAGTATACAGCATCCGTTCACT |
| | | | SEQ ID NO:487 | SEQ ID NO:8499 | SEQ ID NO:16511 |
| | | AA | KSSQSLLHSDGKTYLY | EVSHRFS | MQSIQHPFT |
| | | | SEQ ID NO:488 | SEQ ID NO:8500 | SEQ ID NO:16512 |
| **iPS:392940** | **21-225_29D9** | NA | CGGGCAAGTCAGGGCATTAGAAATGATTTAGGC | GCTGCATCCAGTTTGCAAAGT | CTACAGCATAATACTTACCCATTCACT |
| | | | SEQ ID NO:489 | SEQ ID NO:8501 | SEQ ID NO:16513 |
| | | AA | RASQGIRNDLG | AASSLQS | LQHNTYPFT |
| | | | SEQ ID NO:490 | SEQ ID NO:8502 | SEQ ID NO:16514 |
| **iPS:392942** | **21-225_30E9** | NA | CGGGCAAGTCAGGACATTAGAGATGATTTAGGC | GGTGCATTCAGCTTGCAAAGT | CTACAGCATACTAGTTACCCTCCTACT |
| | | | SEQ ID NO:491 | SEQ ID NO:8503 | SEQ ID NO:16515 |
| | | AA | RASQDIRDDLG | GAFSLQS | LQHTSYPPT |
| | | | SEQ ID NO:492 | SEQ ID NO:8504 | SEQ ID NO:16516 |
| **iPS:392944** | **21-225_31H5** | NA | CGGGCAAGTCAGGACATTAGAAGTGATTTAGGC | GCTGCATCCAGTTTGCAAAGT | ATACAACATATTATTTACCCTCCTACT |
| | | | SEQ ID NO:493 | SEQ ID NO:8505 | SEQ ID NO:16517 |
| | | AA | RASQDIRSDLG | AASSLQS | IQHIIYPPT |
| | | | SEQ ID NO:494 | SEQ ID NO:8506 | SEQ ID NO:16518 |
| **iPS:392948** | **21-225_25G5** | NA | CGGGCAAGTCAGGACATTAGAAATGATTTAGGC | GCTGCATCCAGTTTGCAAAGT | CTACAGCATAATAGTTACCCCTTCACT |
| | | | SEQ ID NO:495 | SEQ ID NO:8507 | SEQ ID NO:16519 |

FIGURE 49

| | | | | | |
|---|---|---|---|---|---|
| | | AA | RASQDIRNDLG | AASSLQS | LQHNSYPFT |
| | | | SEQ ID NO:496 | SEQ ID NO:8508 | SEQ ID NO:16520 |
| **iPS:392950** | **21-225_25C10** | NA | CGGGCGAGTCAGGGCATTAG CAATTATTTAGCC | GCTGCATCCAGTTTGCAA AGT | CAACAGTATCATAGTTACCC ATTCACT |
| | | | SEQ ID NO:497 | SEQ ID NO:8509 | SEQ ID NO:16521 |
| | | AA | RASQGISNYLA | AASSLQS | QQYHSYPFT |
| | | | SEQ ID NO:498 | SEQ ID NO:8510 | SEQ ID NO:16522 |
| **iPS:392952** | **21-225_26G1** | NA | CGGGCGAGTCAGGACATTAG CAATTATTTAGCC | GCTGCATCCAGTTTGCGA AGT | CAACAGTATCATAGTTACCC ATTCACT |
| | | | SEQ ID NO:499 | SEQ ID NO:8511 | SEQ ID NO:16523 |
| | | AA | RASQDISNYLA | AASSLRS | QQYHSYPFT |
| | | | SEQ ID NO:500 | SEQ ID NO:8512 | SEQ ID NO:16524 |
| **iPS:392954** | **21-225_26A10** | NA | CGGGCAAGTCAGAGCATTAG CAGCTATTTAAAT | GCTGCATCCAGCTTGCA AAGT | CAACAGAGTTACAGTACCCC TACGTGGACG |
| | | | SEQ ID NO:501 | SEQ ID NO:8513 | SEQ ID NO:16525 |
| | | AA | RASQSISSYLN | AASSLQS | QQSYSTPTWT |
| | | | SEQ ID NO:502 | SEQ ID NO:8514 | SEQ ID NO:16526 |
| **iPS:392956** | **21-225_27A11** | NA | CGGGCGAGTCAGGGTATTAG TAGTTGGTTAGCC | GGTGCATCCAGTTTGCA AAGT | CAACAGTCTGACAGTTTCCC TCGGACG |
| | | | SEQ ID NO:503 | SEQ ID NO:8515 | SEQ ID NO:16527 |
| | | AA | RASQGISSWLA | GASSLQS | QQSDSFPRT |
| | | | SEQ ID NO:504 | SEQ ID NO:8516 | SEQ ID NO:16528 |
| **iPS:392958** | **21-225_28C7** | NA | CGGGCAAGTCAGGGCATTAG AAATGATTTAGGC | ATTGCATCCAGTTTGCAA AGT | CTACAGCATAATACTTACCC GTGGACG |
| | | | SEQ ID NO:505 | SEQ ID NO:8517 | SEQ ID NO:16529 |
| | | AA | RASQGIRNDLG | IASSLQS | LQHNTYPWT |
| | | | SEQ ID NO:506 | SEQ ID NO:8518 | SEQ ID NO:16530 |
| **iPS:392960** | **21 225 29E6** | NA | AAGTCCAGCCAGAGTGTTTT ATACAGCTCCCACAATAACT ACTACTTAACT | TGGGCATCTTCCCGGGA ATCC | CAGCAGTATTATAGTACTCC TCCGACG |

**FIGURE 49**

| | | | | | |
|---|---|---|---|---|---|
| | **21-225_29E6** | | SEQ ID NO:507 | SEQ ID NO:8519 | SEQ ID NO:16531 |
| | | AA | KSSQSVLYSSHNNYYLT | WASSRES | QQYYSTPPT |
| | | | SEQ ID NO:508 | SEQ ID NO:8520 | SEQ ID NO:16532 |
| **iPS:392962** | **21-225_30A1** | NA | CGGGCGAGTCAGGGCATTAG CAATTATTTAGCC | GCTGCATCCAGTTTGCAA ACT | CAACAGTATAATAGTTACCC ATTCACT |
| | | | SEQ ID NO:509 | SEQ ID NO:8521 | SEQ ID NO:16533 |
| | | AA | RASQGISNYLA | AASSLQT | QQYNSYPFT |
| | | | SEQ ID NO:510 | SEQ ID NO:8522 | SEQ ID NO:16534 |
| **iPS:392964** | **21-225_31A8** | NA | CGGGCAAGTCAGGACATTAG AAGTGATTTAGGC | GCTGTATCCAGTTTGCAA AGT | CTACAGCATACTATTTACCC TCCTACT |
| | | | SEQ ID NO:511 | SEQ ID NO:8523 | SEQ ID NO:16535 |
| | | AA | RASQDIRSDLG | AVSSLQS | LQHTIYPPT |
| | | | SEQ ID NO:512 | SEQ ID NO:8524 | SEQ ID NO:16536 |
| **iPS:392966** | **21-225_32G3** | NA | CGGGCGAGTCAGGCCATTAG CAATTATTTAGCC | GATACATCCAGTTTGCA AAGT | CAACAGTATCATAGTTACCC GCTCACT |
| | | | SEQ ID NO:513 | SEQ ID NO:8525 | SEQ ID NO:16537 |
| | | AA | RASQAISNYLA | DTSSLQS | QQYHSYPLT |
| | | | SEQ ID NO:514 | SEQ ID NO:8526 | SEQ ID NO:16538 |
| **iPS:392968** | **21-225_25B6** | NA | CGGGCAAGTCAGGGCATTAG AAATGATTTAGGC | CGTGCATCCAGTTTGCAA AGT | CTACAGCATAATAGTTACCC ATTCACT |
| | | | SEQ ID NO:515 | SEQ ID NO:8527 | SEQ ID NO:16539 |
| | | AA | RASQGIRNDLG | RASSLQS | LQHNSYPFT |
| | | | SEQ ID NO:516 | SEQ ID NO:8528 | SEQ ID NO:16540 |
| **iPS:392972** | **21-225_26A2** | NA | CGGGCAAGTCAGGGCATTAG AAGTGATTTAGGC | ACTGCATCCAGTTTGCAG AGT | CTACAGCATAATCGTTACCC GTGGACG |
| | | | SEQ ID NO:517 | SEQ ID NO:8529 | SEQ ID NO:16541 |
| | | AA | RASQGIRSDLG | TASSLQS | LQHNRYPWT |
| | | | SEQ ID NO:518 | SEQ ID NO:8530 | SEQ ID NO:16542 |
| **iPS:392974** | | NA | CGGGCAAGTCAGGCCATTAG AAATGATTTAGGC | GCTGCATCCAGTTTGCAA AGT | CTACAGCATTATAATTACCC TCGCAGT |

FIGURE 49

| | | | | | |
|---|---|---|---|---|---|
| | **21-225_26A11** | | SEQ ID NO:519 | SEQ ID NO:8531 | SEQ ID NO:16543 |
| | | AA | RASQAIRNDLG | AASSLQS | LQHYNYPRS |
| | | | SEQ ID NO:520 | SEQ ID NO:8532 | SEQ ID NO:16544 |
| **iPS:392976** | **21-225_27H12** | NA | CGGGCGAGTCAGGGCATTAG CAATTATTTAGCC | GGTGCATCCAGTTTGCA AAGT | CAACAGTATTATAGTTACCC ATTCACT |
| | | | SEQ ID NO:521 | SEQ ID NO:8533 | SEQ ID NO:16545 |
| | | AA | RASQGISNYLA | GASSLQS | QQYYSYPFT |
| | | | SEQ ID NO:522 | SEQ ID NO:8534 | SEQ ID NO:16546 |
| **iPS:392978** | **21-225_28B8** | NA | CGGGCAAGTCAGGGCATTAG AAATGATTTAGGC | GCTGCATCCAGTTTGCAA AGT | CTACAGCATAATAGTTACCC ATTCACT |
| | | | SEQ ID NO:523 | SEQ ID NO:8535 | SEQ ID NO:16547 |
| | | AA | RASQGIRNDLG | AASSLQS | LQHNSYPFT |
| | | | SEQ ID NO:524 | SEQ ID NO:8536 | SEQ ID NO:16548 |
| **iPS:392980** | **21-225_29H6** | NA | CGGGCAAGTCAGGGCATTAG AAATGATTTAGGC | GCTGCATCCAGTTTGCAA AGT | CTACAGCATAATAGTTACCC GCTCACT |
| | | | SEQ ID NO:525 | SEQ ID NO:8537 | SEQ ID NO:16549 |
| | | AA | RASQGIRNDLG | AASSLQS | LQHNSYPLT |
| | | | SEQ ID NO:526 | SEQ ID NO:8538 | SEQ ID NO:16550 |
| **iPS:392982** | **21-225_30D1** | NA | CGGGCAAGTCAGGACATTAG AAGTGATTTAGGC | GCTGCATCCAGTTTGCAA AGT | CTACAGCATACTATTTACCC TCCTACT |
| | | | SEQ ID NO:527 | SEQ ID NO:8539 | SEQ ID NO:16551 |
| | | AA | RASQDIRSDLG | AASSLQS | LQHTIYPPT |
| | | | SEQ ID NO:528 | SEQ ID NO:8540 | SEQ ID NO:16552 |
| **iPS:392984** | **21-225_30E11** | NA | CGGGCAAGTCAGAGCATTAG CAACTATTTAAAT | GCTGCATCCAGTTTGCAA AGT | CAACAGAGTTACAGTACCCC ATTCACT |
| | | | SEQ ID NO:529 | SEQ ID NO:8541 | SEQ ID NO:16553 |
| | | AA | RASQSISNYLN | AASSLQS | QQSYSTPFT |
| | | | SEQ ID NO:530 | SEQ ID NO:8542 | SEQ ID NO:16554 |
| **iPS:392986** | | NA | CGGGCAAGTCAGGACATTAG AAGTGATTTAGGC | GGTGCATCCAGTTTGCA AAGT | CTACAGCATATTATTTACCCT CCTACT |

**FIGURE 49**

| | | | | | |
|---|---|---|---|---|---|
| | **21-225_31B8** | | SEQ ID NO:531 | SEQ ID NO:8543 | SEQ ID NO:16555 |
| | | AA | RASQDIRSDLG | GASSLQS | LQHIIYPPT |
| | | | SEQ ID NO:532 | SEQ ID NO:8544 | SEQ ID NO:16556 |
| **iPS:392988** | **21-225_25E6** | NA | CGGGCAAGTCAGGGCATTAG AAATGATTTAGGC | GCTGCATCCAGTTTGCAA AGT | CTACAGCATAATAGTTACCC GCTCACT |
| | | | SEQ ID NO:533 | SEQ ID NO:8545 | SEQ ID NO:16557 |
| | | AA | RASQGIRNDLG | AASSLQS | LQHNSYPLT |
| | | | SEQ ID NO:534 | SEQ ID NO:8546 | SEQ ID NO:16558 |
| **iPS:392990** | **21-225_25H10** | NA | CGGGCAAGTCGGGGCATTAG AAATGATTTAGGC | GCTGCATTCAGTTTGCAA AGT | CTACAGCATAATAGTTACCC GCTCACT |
| | | | SEQ ID NO:535 | SEQ ID NO:8547 | SEQ ID NO:16559 |
| | | AA | RASRGIRNDLG | AAFSLQS | LQHNSYPLT |
| | | | SEQ ID NO:536 | SEQ ID NO:8548 | SEQ ID NO:16560 |
| **iPS:392992** | **21-225_26C4** | NA | AAGTCCAGCCAGAGTGTTTT ATACCGCTCCAACAATTACA ACTACTTAGCT | TGGGCATCTACCCGGGA ATCC | CAGCAATATTATAGTACTCC TCCGACG |
| | | | SEQ ID NO:537 | SEQ ID NO:8549 | SEQ ID NO:16561 |
| | | AA | KSSQSVLYRSNNYNYLA | WASTRES | QQYYSTPPT |
| | | | SEQ ID NO:538 | SEQ ID NO:8550 | SEQ ID NO:16562 |
| **iPS:392994** | **21-225_26G11** | NA | AAGTCTAGTCAGACCCTCCT GCATGGTGAAGGAAAGACC TATTTGTAT | GAAGTTTCCAACCGGTTC TCT | ATGCAAAGTATAAAGCTTCC GCTCACT |
| | | | SEQ ID NO:539 | SEQ ID NO:8551 | SEQ ID NO:16563 |
| | | AA | KSSQTLLHGEGKTYLY | EVSNRFS | MQSIKLPLT |
| | | | SEQ ID NO:540 | SEQ ID NO:8552 | SEQ ID NO:16564 |
| **iPS:392996** | **21-225_28B1** | NA | CGGGCGAGTCAGGCTATCAA TGACTGGTTAGCC | GCTGCATCCAGTTTCCAA AGT | CAACAGGCTAGCAGTTTCCC ATTCACT |
| | | | SEQ ID NO:541 | SEQ ID NO:8553 | SEQ ID NO:16565 |
| | | AA | RASQAINDWLA | AASSFQS | QQASSFPFT |
| | | | SEQ ID NO:542 | SEQ ID NO:8554 | SEQ ID NO:16566 |

## FIGURE 49

| | | | | | |
|---|---|---|---|---|---|
| **iPS:392998** | **21-225_28A9** | NA | CGGGCAAGTCAGGGCATTAG AAATGATTTAGGC | GCTGCTTCTAGTTTGCAA AAT | CTACAGCATAATCGTTACCC ATTCACT |
| | | | SEQ ID NO:543 | SEQ ID NO:8555 | SEQ ID NO:16567 |
| | | AA | RASQGIRNDLG | AASSLQN | LQHNRYPFT |
| | | | SEQ ID NO:544 | SEQ ID NO:8556 | SEQ ID NO:16568 |
| **iPS:393000** | **21-225_29D7** | NA | CGGGCAAGTCAGGGCATTAG AAATGATTTAGGC | GCTGCATCCAGTTTACAA AGT | CTACAGCATAATAGTTACCC ATTCACT |
| | | | SEQ ID NO:545 | SEQ ID NO:8557 | SEQ ID NO:16569 |
| | | AA | RASQGIRNDLG | AASSLQS | LQHNSYPFT |
| | | | SEQ ID NO:546 | SEQ ID NO:8558 | SEQ ID NO:16570 |
| **iPS:393002** | **21-225_30G1** | NA | CGGGCAAGTCAGAACATTTA CAGCTATTTAAAT | GCTGCATCCAGTTTGCAT AGT | CAACAGAGTTACAGTACTCC GCTCACT |
| | | | SEQ ID NO:547 | SEQ ID NO:8559 | SEQ ID NO:16571 |
| | | AA | RASQNIYSYLN | AASSLHS | QQSYSTPLT |
| | | | SEQ ID NO:548 | SEQ ID NO:8560 | SEQ ID NO:16572 |
| **iPS:393004** | **21-225_30G11** | NA | CGGGCAAGTCAGGACATTAG AAGTGATTTAGGC | GCTGCATCCAGTTTGCAA AGT | CTACAGCATACTATTTACCC TCCTACT |
| | | | SEQ ID NO:549 | SEQ ID NO:8561 | SEQ ID NO:16573 |
| | | AA | RASQDIRSDLG | AASSLQS | LQHTIYPPT |
| | | | SEQ ID NO:550 | SEQ ID NO:8562 | SEQ ID NO:16574 |
| **iPS:393006** | **21-225_31G9** | NA | CGGGCAAGTCAGGGCATTAG AAATGATTTAGGC | CCTGCATCCAGTTTGCAA AGT | CTACAGGATAATAGTTACCC TTTCACT |
| | | | SEQ ID NO:551 | SEQ ID NO:8563 | SEQ ID NO:16575 |
| | | AA | RASQGIRNDLG | PASSLQS | LQDNSYPFT |
| | | | SEQ ID NO:552 | SEQ ID NO:8564 | SEQ ID NO:16576 |
| **iPS:393010** | **21-225_25E11** | NA | CGGGCGAGTCAGGGTATTAG CAACTGGTTAGCC | ACTGCATCCAGTTTGCAA GGT | CAACAGGCTAACAGTTTCCC AATCACT |
| | | | SEQ ID NO:553 | SEQ ID NO:8565 | SEQ ID NO:16577 |
| | | AA | RASQGISNWLA | TASSLQG | QQANSFPIT |
| | | | SEQ ID NO:554 | SEQ ID NO:8566 | SEQ ID NO:16578 |

## FIGURE 49

| | | | | | |
|---|---|---|---|---|---|
| **iPS:393012** | **21-225_26G7** | NA | AAGTCTAGTCAGAGCCTCCTGCATAGTGAGGGAAAGACCTATTTGTAT | GAAGTTTCCCACCGGCTCTCT | ATGCAAAGTATACAGCTTCCGCTCACT |
| | | | SEQ ID NO:555 | SEQ ID NO:8567 | SEQ ID NO:16579 |
| | | AA | KSSQSLLHSEGKTYLY | EVSHRLS | MQSIQLPLT |
| | | | SEQ ID NO:556 | SEQ ID NO:8568 | SEQ ID NO:16580 |
| **iPS:393014** | **21-225_26D12** | NA | CGGGCGAGTCAGGGTATTAGTAGTTGGTTAGCC | GGTGCATCCAGTTTGCAAAGT | CAACAGTCTGACAGTTTCCCTCGGACG |
| | | | SEQ ID NO:557 | SEQ ID NO:8569 | SEQ ID NO:16581 |
| | | AA | RASQGISSWLA | GASSLQS | QQSDSFPRT |
| | | | SEQ ID NO:558 | SEQ ID NO:8570 | SEQ ID NO:16582 |
| **iPS:393016** | **21-225_28F11** | NA | CGGGCGAGTCAGGGTATTAGCAACTGGTTAGCC | GCTGCATCCAATTTGCAAAGT | CAACAGGCTAACAGTCTCCCATTCACT |
| | | | SEQ ID NO:559 | SEQ ID NO:8571 | SEQ ID NO:16583 |
| | | AA | RASQGISNWLA | AASNLQS | QQANSLPFT |
| | | | SEQ ID NO:560 | SEQ ID NO:8572 | SEQ ID NO:16584 |
| **iPS:393018** | **21-225_29B8** | NA | CGGGCAAGTCAGGGCATTAGAAATGATTTAGGC | GCTGCATCCAGTTTGCAAAGT | CTACAGCATAATAGTTACCCGCTCACT |
| | | | SEQ ID NO:561 | SEQ ID NO:8573 | SEQ ID NO:16585 |
| | | AA | RASQGIRNDLG | AASSLQS | LQHNSYPLT |
| | | | SEQ ID NO:562 | SEQ ID NO:8574 | SEQ ID NO:16586 |
| **iPS:393020** | **21-225_30E2** | NA | CAGGCGAGTCAGTACATTAGCAACTATTTAAAT | GATGGATCCAGTTTGGAAACA | CAACAGTATGATAATCTCCCGATCACC |
| | | | SEQ ID NO:563 | SEQ ID NO:8575 | SEQ ID NO:16587 |
| | | AA | QASQYISNYLN | DGSSLET | QQYDNLPIT |
| | | | SEQ ID NO:564 | SEQ ID NO:8576 | SEQ ID NO:16588 |
| **iPS:393022** | **21-225_30H11** | NA | CGGGCAAGTCAGGGCATTAGAAATGATTTAGGC | CCTGCATCCAGTTTGCAAAGT | CTACAGGATAATAGTCATCCATTCACT |
| | | | SEQ ID NO:565 | SEQ ID NO:8577 | SEQ ID NO:16589 |
| | | AA | RASQGIRNDLG | PASSLQS | LQDNSHPFT |

**FIGURE 49**

| | | | | | |
|---|---|---|---|---|---|
| | | | SEQ ID NO:566 | SEQ ID NO:8578 | SEQ ID NO:16590 |
| **iPS:393024** | **21-225_31H9** | NA | CGGGCGAGTCAGGGTATTACCAGCTGGTTAACT | GATACATCCAGTTTGCAAAGT | CAACAGGGTAACAGTTTCCCATTCACT |
| | | | SEQ ID NO:567 | SEQ ID NO:8579 | SEQ ID NO:16591 |
| | | AA | RASQGITSWLT | DTSSLQS | QQGNSFPFT |
| | | | SEQ ID NO:568 | SEQ ID NO:8580 | SEQ ID NO:16592 |
| **iPS:393026** | **21-225_32B6** | NA | CGGGCAAGTCAGGGCATTAGAAATGATTTAGGC | ATTGCATCCAGTTTGCAAAGT | CTACAGCATAATAGTTACCCGTGGACG |
| | | | SEQ ID NO:569 | SEQ ID NO:8581 | SEQ ID NO:16593 |
| | | AA | RASQGIRNDLG | IASSLQS | LQHNSYPWT |
| | | | SEQ ID NO:570 | SEQ ID NO:8582 | SEQ ID NO:16594 |
| **iPS:393028** | **21-225_25D7** | NA | CGGGCGAGTCAGGATATTTTCGACTGGTTAGCC | GCTGCATCCAGTTTGCAAAGT | CAACAGGCTTACAGTTTCCCGTGGACG |
| | | | SEQ ID NO:571 | SEQ ID NO:8583 | SEQ ID NO:16595 |
| | | AA | RASQDIFDWLA | AASSLQS | QQAYSFPWT |
| | | | SEQ ID NO:572 | SEQ ID NO:8584 | SEQ ID NO:16596 |
| **iPS:393030** | **21-225_25H11** | NA | CGGGCAAGTCAGGGCATTAGAACTGATTTAGGC | GCTGCATCCAGTTTACAAAGT | CTACAGCATAATAGTTACCCGCTCACT |
| | | | SEQ ID NO:573 | SEQ ID NO:8585 | SEQ ID NO:16597 |
| | | AA | RASQGIRTDLG | AASSLQS | LQHNSYPLT |
| | | | SEQ ID NO:574 | SEQ ID NO:8586 | SEQ ID NO:16598 |
| **iPS:393032** | **21-225_26F8** | NA | AAGTCTAGTCAGAGCCTCCTGCATGGTGATGGAAAGACCTATTTGTAT | GAAGTTTCCAACCGGTTCTCT | ATGCAAAGTATACAGCTTCCGTGGACG |
| | | | SEQ ID NO:575 | SEQ ID NO:8587 | SEQ ID NO:16599 |
| | | AA | KSSQSLLHGDGKTYLY | EVSNRFS | MQSIQLPWT |
| | | | SEQ ID NO:576 | SEQ ID NO:8588 | SEQ ID NO:16600 |
| **iPS:393034** | **21-225_27F2** | NA | CGGGCAAGTCAGGGCATTAGAAATGATTTAGGC | GTTGCATCCAGTTTGCAAAGT | CTACAGCATAATAGTTACCCGCTCACT |
| | | | SEQ ID NO:577 | SEQ ID NO:8589 | SEQ ID NO:16601 |

**FIGURE 49**

| | | | | | |
|---|---|---|---|---|---|
| | | AA | RASQGIRNDLG | VASSLQS | LQHNSYPLT |
| | | | SEQ ID NO:578 | SEQ ID NO:8590 | SEQ ID NO:16602 |
| **iPS:393036** | **21-225_28G3** | NA | AAGTCTAGTCAGAGCCTCCTACATGGTGATGGAAAGACCTATTTGTAT | GAAGTTTCCAACCGGTTCTCT | ATGCAAAGTATACAGATTCCGTGGACG |
| | | | SEQ ID NO:579 | SEQ ID NO:8591 | SEQ ID NO:16603 |
| | | AA | KSSQSLLHGDGKTYLY | EVSNRFS | MQSIQIPWT |
| | | | SEQ ID NO:580 | SEQ ID NO:8592 | SEQ ID NO:16604 |
| **iPS:393038** | **21-225_29D8** | NA | CGGGCAAGTCAGGGCATTAGAAATGATTTAGGC | ACTGCATCCAGTTTGCAAAGT | CTACAGCATAATAGTTACCCATTCACT |
| | | | SEQ ID NO:581 | SEQ ID NO:8593 | SEQ ID NO:16605 |
| | | AA | RASQGIRNDLG | TASSLQS | LQHNSYPFT |
| | | | SEQ ID NO:582 | SEQ ID NO:8594 | SEQ ID NO:16606 |
| **iPS:393040** | **21-225_30E3** | NA | CGGGCAAGTCAGGACATTAGAGATGATTTAGGC | GCTGCATTCAGCTTGCAAAGT | CTACAGCATACTAGTTACCCTCCTACT |
| | | | SEQ ID NO:583 | SEQ ID NO:8595 | SEQ ID NO:16607 |
| | | AA | RASQDIRDDLG | AAFSLQS | LQHTSYPPT |
| | | | SEQ ID NO:584 | SEQ ID NO:8596 | SEQ ID NO:16608 |
| **iPS:393042** | **21-225_31F1** | NA | CGGGCAAGTCAGAGGATTAGCAGCTATTTAAAT | GCTGCATCCAGTTCGCAAAGT | CAACAGAGTTACATTACCCCGCTCACT |
| | | | SEQ ID NO:585 | SEQ ID NO:8597 | SEQ ID NO:16609 |
| | | AA | RASQRISSYLN | AASSSQS | QQSYITPLT |
| | | | SEQ ID NO:586 | SEQ ID NO:8598 | SEQ ID NO:16610 |
| **iPS:393044** | **21-225_25B8** | NA | AGGGCCAGTCAGAGTGTTAGAAGTAATTTAGCC | GGTGCATCCACCAGGGCCACT | CAGCAGTATAATAATTGGCCTCCGTGGCCG |
| | | | SEQ ID NO:587 | SEQ ID NO:8599 | SEQ ID NO:16611 |
| | | AA | RASQSVRSNLA | GASTRAT | QQYNNWPPWP |
| | | | SEQ ID NO:588 | SEQ ID NO:8600 | SEQ ID NO:16612 |
| **iPS:393046** | | NA | CGGGCAAGTCAGGCCATTAGAGATGATTTAGGC | GCTGCATCCAGTTTGCAAAGT | CTACAGCATTATAATTACCCTCGCAGT |

## FIGURE 49

| | | | | | |
|---|---|---|---|---|---|
| | 21-225_25A12 | | SEQ ID NO:589 | SEQ ID NO:8601 | SEQ ID NO:16613 |
| | | AA | RASQAIRDDLG | AASSLQS | LQHYNYPRS |
| | | | SEQ ID NO:590 | SEQ ID NO:8602 | SEQ ID NO:16614 |
| **iPS:393048** | 21-225_27C3 | NA | CGGGCAAGTCAGGGCATTAG AAATGATTTAGGC | GCTGCATCCAGTTTGCAA AGT | CTACAGCATAATCGTTACCC GCTCACT |
| | | | SEQ ID NO:591 | SEQ ID NO:8603 | SEQ ID NO:16615 |
| | | AA | RASQGIRNDLG | AASSLQS | LQHNRYPLT |
| | | | SEQ ID NO:592 | SEQ ID NO:8604 | SEQ ID NO:16616 |
| **iPS:393050** | 21-225_28C5 | NA | AGGGCCAGTCAGAGTGTTAG CAGCAACTTAGCC | GGTGCATCCACCAGGGC CACT | CAGCAGTATAATAATTGGCC TCCGTGGCCG |
| | | | SEQ ID NO:593 | SEQ ID NO:8605 | SEQ ID NO:16617 |
| | | AA | RASQSVSSNLA | GASTRAT | QQYNNWPPWP |
| | | | SEQ ID NO:594 | SEQ ID NO:8606 | SEQ ID NO:16618 |
| **iPS:393054** | 21-225_29G8 | NA | CGGGCAAGTCAGGGCATTAG AAATGATTTAGGC | GCTGCATCCAGTTTGCAA AGT | CTACAGCATAATAGTTATCC GCTCACT |
| | | | SEQ ID NO:595 | SEQ ID NO:8607 | SEQ ID NO:16619 |
| | | AA | RASQGIRNDLG | AASSLQS | LQHNSYPLT |
| | | | SEQ ID NO:596 | SEQ ID NO:8608 | SEQ ID NO:16620 |
| **iPS:393056** | 21-225_30F3 | NA | CGGGCAAGTCAGGGCATTAG AAATGATTTAGGC | ACTGCATCCAGTTTACAA AGT | CTACAGCATAATAGTTACCC GTTCACT |
| | | | SEQ ID NO:597 | SEQ ID NO:8609 | SEQ ID NO:16621 |
| | | AA | RASQGIRNDLG | TASSLQS | LQHNSYPFT |
| | | | SEQ ID NO:598 | SEQ ID NO:8610 | SEQ ID NO:16622 |
| **iPS:393058** | 21-225_31H3 | NA | CGGGCAAGTCAGGACATTAG AGATGATTTAGGC | GCTGCATTCAGCTTGCAA AGT | CTACAGCATACTAGTTACCC TCCTACT |
| | | | SEQ ID NO:599 | SEQ ID NO:8611 | SEQ ID NO:16623 |
| | | AA | RASQDIRDDLG | AAFSLQS | LQHTSYPPT |
| | | | SEQ ID NO:600 | SEQ ID NO:8612 | SEQ ID NO:16624 |
| **iPS:393060** | | NA | CGGGCAAGTCAGGACATTAG AAGTGATTTAGGC | GCTGCATCCAGTTTGCAA AGT | CTGCAGCATACTATTTACCC TCCTACT |

**FIGURE 49**

| | | | | | |
|---|---|---|---|---|---|
| | **21-225_32G12** | | SEQ ID NO:601 | SEQ ID NO:8613 | SEQ ID NO:16625 |
| | | AA | RASQDIRSDLG | AASSLQS | LQHTIYPPT |
| | | | SEQ ID NO:602 | SEQ ID NO:8614 | SEQ ID NO:16626 |
| **iPS:393062** | **21-225_33H3** | NA | CAGGCGAGTCAGGACATTTC CAACTTTTTAAAT | GATGCATCCAATTTGGTA ACA | CAACAGTATGATAATCTCCC GATCACC |
| | | | SEQ ID NO:603 | SEQ ID NO:8615 | SEQ ID NO:16627 |
| | | AA | QASQDISNFLN | DASNLVT | QQYDNLPIT |
| | | | SEQ ID NO:604 | SEQ ID NO:8616 | SEQ ID NO:16628 |
| **iPS:393064** | **21-225_33A9** | NA | CGGGCAAGTCAGAGCATTAG CAGGTATTTAAGT | GCTGCATCCAGTTTGCAA AGT | CAACAGAGTTACAATATCCC GATCACC |
| | | | SEQ ID NO:605 | SEQ ID NO:8617 | SEQ ID NO:16629 |
| | | AA | RASQSISRYLS | AASSLQS | QQSYNIPIT |
| | | | SEQ ID NO:606 | SEQ ID NO:8618 | SEQ ID NO:16630 |
| **iPS:393066** | **21-225_34D3** | NA | CGGGCAAGTCAGAACATTTA CAGCTATTTAAAT | GCTGCATCCAGTTTGCAT AGT | CAACAGAGTTACAGTACTCC GCTCACT |
| | | | SEQ ID NO:607 | SEQ ID NO:8619 | SEQ ID NO:16631 |
| | | AA | RASQNIYSYLN | AASSLHS | QQSYSTPLT |
| | | | SEQ ID NO:608 | SEQ ID NO:8620 | SEQ ID NO:16632 |
| **iPS:393068** | **21-225_34G9** | NA | CGGGCAAGTCAGGACATTAG AAGTGATTTAGGC | GCTGCATCCAGTTTGCAA AGT | CTCCAGCATACTATTTACCCT CCTACT |
| | | | SEQ ID NO:609 | SEQ ID NO:8621 | SEQ ID NO:16633 |
| | | AA | RASQDIRSDLG | AASSLQS | LQHTIYPPT |
| | | | SEQ ID NO:610 | SEQ ID NO:8622 | SEQ ID NO:16634 |
| **iPS:393072** | **21-225_36C5** | NA | CGGGCAAGTCAGGACATTAG AAGTGATTTAGGC | GCTGCATCCAGTTTGCAA AGT | CTCCATCATCCTATTTACCCT CCTACT |
| | | | SEQ ID NO:611 | SEQ ID NO:8623 | SEQ ID NO:16635 |
| | | AA | RASQDIRSDLG | AASSLQS | LHHPIYPPT |
| | | | SEQ ID NO:612 | SEQ ID NO:8624 | SEQ ID NO:16636 |
| **iPS:393074** | | NA | CGGACAAGTCAGGGCATTAG AAATGATTTAGGC | ACTGCATCCAGTTTGCAA AGT | CTACAGCATAATAGTTACCC GCTCACT |

FIGURE 49

| | | | | | |
|---|---|---|---|---|---|
| | **21-225_33B1** | | SEQ ID NO:613 | SEQ ID NO:8625 | SEQ ID NO:16637 |
| | | AA | RTSQGIRNDLG | TASSLQS | LQHNSYPLT |
| | | | SEQ ID NO:614 | SEQ ID NO:8626 | SEQ ID NO:16638 |
| **iPS:393076** | **21-225_33A4** | NA | CGGGCAAGTCAGGACATTAG AAATGATGTAGGC | GCTGCATCCAGTTTGCAA CGT | CTACAGCATTATAGTTACCC TCCTACT |
| | | | SEQ ID NO:615 | SEQ ID NO:8627 | SEQ ID NO:16639 |
| | | AA | RASQDIRNDVG | AASSLQR | LQHYSYPPT |
| | | | SEQ ID NO:616 | SEQ ID NO:8628 | SEQ ID NO:16640 |
| **iPS:393078** | **21-225_33H11** | NA | TGGGCGAGTCAGGGCATTAA CAGTTATTTAGCC | GCTGCATCCAGTTTGCAA GGT | CAACAGTTTAATAGTTACCC TCTGACG |
| | | | SEQ ID NO:617 | SEQ ID NO:8629 | SEQ ID NO:16641 |
| | | AA | WASQGINSYLA | AASSLQG | QQFNSYPLT |
| | | | SEQ ID NO:618 | SEQ ID NO:8630 | SEQ ID NO:16642 |
| **iPS:393080** | **21-225_34F3** | NA | CGGGCGAGTCAGGGTATTAG TAAGTGGTTAGCC | GCTGCATCCAGTTTGCAA AGT | CAACAGGCTAACAGTTTCCC TTTCACT |
| | | | SEQ ID NO:619 | SEQ ID NO:8631 | SEQ ID NO:16643 |
| | | AA | RASQGISKWLA | AASSLQS | QQANSFPFT |
| | | | SEQ ID NO:620 | SEQ ID NO:8632 | SEQ ID NO:16644 |
| **iPS:393082** | **21-225_34C11** | NA | CGGGCAAGTCAGAACATTAG GAACTTTTTAAAT | GGTGCATCCACTTTGCAA AGT | CAACAGACTTGCAGTACCCC GCTCACT |
| | | | SEQ ID NO:621 | SEQ ID NO:8633 | SEQ ID NO:16645 |
| | | AA | RASQNIRNFLN | GASTLQS | QQTCSTPLT |
| | | | SEQ ID NO:622 | SEQ ID NO:8634 | SEQ ID NO:16646 |
| **iPS:393084** | **21-225_35C6** | NA | CGGGCGAGTCAGGGTATTAG CAAATGGTTAGCC | GCTGCATCCAGTTTGCAG AGT | CAACAGGCTAACAGTTTCCC ATTCACT |
| | | | SEQ ID NO:623 | SEQ ID NO:8635 | SEQ ID NO:16647 |
| | | AA | RASQGISKWLA | AASSLQS | QQANSFPFT |
| | | | SEQ ID NO:624 | SEQ ID NO:8636 | SEQ ID NO:16648 |
| **iPS:393086** | | NA | CGGGCGAGTCAGGGTATTAG CAGATGGTTAGCC | GCTGCATCCCGTTTGCAA AGT | CAACAGGCTAACAGTTTCCC TTTCACT |

FIGURE 49

| | | | | | |
|---|---|---|---|---|---|
| | **21-225_36H5** | | SEQ ID NO:625 | SEQ ID NO:8637 | SEQ ID NO:16649 |
| | | AA | RASQGISRWLA | AASRLQS | QQANSFPFT |
| | | | SEQ ID NO:626 | SEQ ID NO:8638 | SEQ ID NO:16650 |
| **iPS:393088** | **21-225_33D1** | NA | AAGTCCATCCAGAGTGTTTT ATACAGATCCAACAATAAGA ACTACTTAACT | TGGGCATCTACCCGGGA ATCC | CAGCAATATTATAGTTCTCC GTGCAGT |
| | | | SEQ ID NO:627 | SEQ ID NO:8639 | SEQ ID NO:16651 |
| | | AA | KSIQSVLYRSNNKNYLT | WASTRES | QQYYSSPCS |
| | | | SEQ ID NO:628 | SEQ ID NO:8640 | SEQ ID NO:16652 |
| **iPS:393090** | **21-225_33A5** | NA | CGGGCGAGTCAGGGCATTAG CAATTACTTAGCC | GCTGCATCCAGTTTGCAA AGT | CAACAGTATAATAGTTACCC ATTCACT |
| | | | SEQ ID NO:629 | SEQ ID NO:8641 | SEQ ID NO:16653 |
| | | AA | RASQGISNYLA | AASSLQS | QQYNSYPFT |
| | | | SEQ ID NO:630 | SEQ ID NO:8642 | SEQ ID NO:16654 |
| **iPS:393092** | **21-225_33C12** | NA | CGGGCAAGTCAGAGCATTAT CAGCTATTTAAAT | GTTGCATCCAGTTTGCAA GGT | CAACAGAGTTACAGTACCCC GTACACT |
| | | | SEQ ID NO:631 | SEQ ID NO:8643 | SEQ ID NO:16655 |
| | | AA | RASQSIISYLN | VASSLQG | QQSYSTPYT |
| | | | SEQ ID NO:632 | SEQ ID NO:8644 | SEQ ID NO:16656 |
| **iPS:393094** | **21-225_34C4** | NA | CGGGCAAGTCAGGGCATTAG AAATGATTTAGGC | ACTGCATCCAATTTGCAA AGT | CTACAACATAGTTCTTACCC CATCACC |
| | | | SEQ ID NO:633 | SEQ ID NO:8645 | SEQ ID NO:16657 |
| | | AA | RASQGIRNDLG | TASNLQS | LQHSSYPIT |
| | | | SEQ ID NO:634 | SEQ ID NO:8646 | SEQ ID NO:16658 |
| **iPS:393096** | **21-225_34D11** | NA | CGGGCAAGTCAGGACATTAG AAGTGATTTAGGC | GCTGCATCCAGTTTGCAA AGT | CTACAGCATACTATTTACCC TCCTACT |
| | | | SEQ ID NO:635 | SEQ ID NO:8647 | SEQ ID NO:16659 |
| | | AA | RASQDIRSDLG | AASSLQS | LQHTIYPPT |
| | | | SEQ ID NO:636 | SEQ ID NO:8648 | SEQ ID NO:16660 |

**FIGURE 49**

| | | | | | |
|---|---|---|---|---|---|
| **iPS:393098** | **21-225_35G6** | NA | CGGGCGAGTCAGGGTATTAG CCGGTGGTTAGCC | GCTGCATCCAGGTTGCA AAGT | CAACAGGCTAACAGTTTCCC GTTCACT |
| | | | SEQ ID NO:637 | SEQ ID NO:8649 | SEQ ID NO:16661 |
| | | AA | RASQGISRWLA | AASRLQS | QQANSFPFT |
| | | | SEQ ID NO:638 | SEQ ID NO:8650 | SEQ ID NO:16662 |
| **iPS:393100** | **21-225_36B8** | NA | CGGGCAAGTCAGAGCATTAT CAGCTATTTAAAT | GTTGCATCCAGTTTGCAA AGT | CAACAGAGTTACAGTACCCC GTACACT |
| | | | SEQ ID NO:639 | SEQ ID NO:8651 | SEQ ID NO:16663 |
| | | AA | RASQSIISYLN | VASSLQS | QQSYSTPYT |
| | | | SEQ ID NO:640 | SEQ ID NO:8652 | SEQ ID NO:16664 |
| **iPS:393102** | **21-225_33F1** | NA | CGGACAAGTCAGGACATTAG AAGTGATTTAGGC | GCTGCATCCAGTTTGCAA AGT | CTACAGCATACTATTTACCC TCCTACT |
| | | | SEQ ID NO:641 | SEQ ID NO:8653 | SEQ ID NO:16665 |
| | | AA | RTSQDIRSDLG | AASSLQS | LQHTIYPPT |
| | | | SEQ ID NO:642 | SEQ ID NO:8654 | SEQ ID NO:16666 |
| **iPS:393104** | **21-225_33A7** | NA | CGGGCAAGTCAGGACATTAG AAGTGATTTAGGC | GTTGCATCCAGTTTGCAA AGT | CTACAGCATACTATTTACCC TCCTACT |
| | | | SEQ ID NO:643 | SEQ ID NO:8655 | SEQ ID NO:16667 |
| | | AA | RASQDIRSDLG | VASSLQS | LQHTIYPPT |
| | | | SEQ ID NO:644 | SEQ ID NO:8656 | SEQ ID NO:16668 |
| **iPS:393106** | **21-225_34A6** | NA | CGGACAAGTCAGGACATCA GAAATGATTTAGGC | GCTGCATCCAGTTTGCAA AGT | CTACAGCATAATAGTTACCC TCCTACT |
| | | | SEQ ID NO:645 | SEQ ID NO:8657 | SEQ ID NO:16669 |
| | | AA | RTSQDIRNDLG | AASSLQS | LQHNSYPPT |
| | | | SEQ ID NO:646 | SEQ ID NO:8658 | SEQ ID NO:16670 |
| **iPS:393108** | **21-225_34G11** | NA | CGGGCAAGTCAGAACATTAA CAGGTATTTAAAT | GGTGCATCCAGTTTGCA AAGT | CAACAGACTTACATTACCCC GCTCACT |
| | | | SEQ ID NO:647 | SEQ ID NO:8659 | SEQ ID NO:16671 |
| | | AA | RASQNINRYLN | GASSLQS | QQTYITPLT |
| | | | SEQ ID NO:648 | SEQ ID NO:8660 | SEQ ID NO:16672 |

FIGURE 49

| | | | | | |
|---|---|---|---|---|---|
| **iPS:393110** | **21-225_35B7** | NA | CGGGCAAGTCAGGACATTAG AAGTGATTTAGGC | GCTGCATCCAGTTTGCAA AGT | CTACAGCATACTATTTACCC TCCTACT |
| | | | SEQ ID NO:649 | SEQ ID NO:8661 | SEQ ID NO:16673 |
| | | AA | RASQDIRSDLG | AASSLQS | LQHTIYPPT |
| | | | SEQ ID NO:650 | SEQ ID NO:8662 | SEQ ID NO:16674 |
| **iPS:393112** | **21-225_33G1** | NA | CGGGCGAGTCAGGGTATTAG CAGGTGGTTAGCC | GGTGCATACAGTCTGCA AAGT | CAACAGGCTAACAGTTTCCC ATTCACT |
| | | | SEQ ID NO:651 | SEQ ID NO:8663 | SEQ ID NO:16675 |
| | | AA | RASQGISRWLA | GAYSLQS | QQANSFPFT |
| | | | SEQ ID NO:652 | SEQ ID NO:8664 | SEQ ID NO:16676 |
| **iPS:393114** | **21-225_33G12** | NA | CGGGCAAGTCAGAGCATTAG CAACTATTTAAAT | GCTGCATCCAGTTTGCAA AGT | CAACAGAGTTACAGTACCCC ATTCACT |
| | | | SEQ ID NO:653 | SEQ ID NO:8665 | SEQ ID NO:16677 |
| | | AA | RASQSISNYLN | AASSLQS | QQSYSTPFT |
| | | | SEQ ID NO:654 | SEQ ID NO:8666 | SEQ ID NO:16678 |
| **iPS:393116** | **21-225_34G7** | NA | CGGGCGAGTCAGCTTATTAG CAAGTGGTTAGCC | GCTGCATCCAGTTTGCAA AGT | CAACAGGCTAACAGTTTCCC ATTCACT |
| | | | SEQ ID NO:655 | SEQ ID NO:8667 | SEQ ID NO:16679 |
| | | AA | RASQLISKWLA | AASSLQS | QQANSFPFT |
| | | | SEQ ID NO:656 | SEQ ID NO:8668 | SEQ ID NO:16680 |
| **iPS:393118** | **21-225_34H11** | NA | CGGGCAAGTCAGGACATTAG AAATGATTTAGGC | GCTACATCCAGTTTGCAA AGT | CTACAGCATAATAGTTACCC TCCTACT |
| | | | SEQ ID NO:657 | SEQ ID NO:8669 | SEQ ID NO:16681 |
| | | AA | RASQDIRNDLG | ATSSLQS | LQHNSYPPT |
| | | | SEQ ID NO:658 | SEQ ID NO:8670 | SEQ ID NO:16682 |
| **iPS:393120** | **21-225_35H8** | NA | CGGGCGAGTCAGGCCATTAG TAATTATTTAGCC | GGTGCGTCCGGTTTGCA AAGT | CAACAGTATAATAGTTACCC ATTCACT |
| | | | SEQ ID NO:659 | SEQ ID NO:8671 | SEQ ID NO:16683 |
| | | AA | RASQAISNYLA | GASGLQS | QQYNSYPFT |
| | | | SEQ ID NO:660 | SEQ ID NO:8672 | SEQ ID NO:16684 |

FIGURE 49

| | | | | | |
|---|---|---|---|---|---|
| **iPS:393122** | **21-225_33B2** | NA | CGGGCAAGTCAGAGCATTATCAGCTATTTAAAT | GTTGCATCCAGTTTGCAAAGT | CAACAGAGTTACAGTACCCCGTACACT |
| | | | SEQ ID NO:661 | SEQ ID NO:8673 | SEQ ID NO:16685 |
| | | AA | RASQSIISYLN | VASSLQS | QQSYSTPYT |
| | | | SEQ ID NO:662 | SEQ ID NO:8674 | SEQ ID NO:16686 |
| **iPS:393124** | **21-225_33G7** | NA | CGGGCAAGTCAGGGCATTAGAAATGATTTAGGC | GCTGCATCCAGTTTGCAAAGT | CTACAGCATTATAGTTACCCTCCTACT |
| | | | SEQ ID NO:663 | SEQ ID NO:8675 | SEQ ID NO:16687 |
| | | AA | RASQGIRNDLG | AASSLQS | LQHYSYPPT |
| | | | SEQ ID NO:664 | SEQ ID NO:8676 | SEQ ID NO:16688 |
| **iPS:393126** | **21-225_35D1** | NA | CGGGCAAGTCAGGACATTAGAAGTGATTTAGGC | GCTGCATCCAGTTTGCAAAGT | CTACAGCATACTATTTACCCTCCCACT |
| | | | SEQ ID NO:665 | SEQ ID NO:8677 | SEQ ID NO:16689 |
| | | AA | RASQDIRSDLG | AASSLQS | LQHTIYPPT |
| | | | SEQ ID NO:666 | SEQ ID NO:8678 | SEQ ID NO:16690 |
| **iPS:393128** | **21-225_35F11** | NA | CGGGCAAGTCAGGACATTAGAAGTGATTTAGGC | GCTGCATCCAGTTTGCAAAGT | CTACAGCATACTGTTTACCCTCCTACT |
| | | | SEQ ID NO:667 | SEQ ID NO:8679 | SEQ ID NO:16691 |
| | | AA | RASQDIRSDLG | AASSLQS | LQHTVYPPT |
| | | | SEQ ID NO:668 | SEQ ID NO:8680 | SEQ ID NO:16692 |
| **iPS:393130** | **21-225_33C2** | NA | CGGGCAAGTCAGGGCATTAGAAATGATTTAGGC | GCTGCACCCAGTTTGCAAAGT | CTACAGCATAATAGTTACCCGTGGACG |
| | | | SEQ ID NO:669 | SEQ ID NO:8681 | SEQ ID NO:16693 |
| | | AA | RASQGIRNDLG | AAPSLQS | LQHNSYPWT |
| | | | SEQ ID NO:670 | SEQ ID NO:8682 | SEQ ID NO:16694 |
| **iPS:393132** | **21-225_33H7** | NA | CGGGCGAGTCAGGGTATTAGCCGGTGGTTAGCC | GCTGCATCCAGGTTGCAAAGT | CAACAGGCTAACATTTTCCCGTTCACT |
| | | | SEQ ID NO:671 | SEQ ID NO:8683 | SEQ ID NO:16695 |
| | | AA | RASQGISRWLA | AASRLQS | QQANIFPFT |
| | | | SEQ ID NO:672 | SEQ ID NO:8684 | SEQ ID NO:16696 |

FIGURE 49

| | | | | | |
|---|---|---|---|---|---|
| **iPS:393134** | **21-225_34C2** | NA | CGGGCAAGTCAGAGAATTATCAGCTATTTAAAT | GTTGCATCCAGTTTGCAAAGT | CAACAGAGTTACAGTACCCCGTACACT |
| | | | SEQ ID NO:673 | SEQ ID NO:8685 | SEQ ID NO:16697 |
| | | AA | RASQRIISYLN | VASSLQS | QQSYSTPYT |
| | | | SEQ ID NO:674 | SEQ ID NO:8686 | SEQ ID NO:16698 |
| **iPS:393136** | **21-225_34D8** | NA | CGGGCAAGTCAGATCATTATCAGCTATTTAAAT | GTTGCATCCAGTTTGCAAAGT | CAACAGAGTTACAGTACCCCGTACACT |
| | | | SEQ ID NO:675 | SEQ ID NO:8687 | SEQ ID NO:16699 |
| | | AA | RASQIIISYLN | VASSLQS | QQSYSTPYT |
| | | | SEQ ID NO:676 | SEQ ID NO:8688 | SEQ ID NO:16700 |
| **iPS:393138** | **21-225_35E3** | NA | CAGGCGAGTCAGGACATTTTCAACTATTTAAAT | GATGCCTCCAATTTGGAAACA | CAACAGTATGATAATCTCCCGATCACC |
| | | | SEQ ID NO:677 | SEQ ID NO:8689 | SEQ ID NO:16701 |
| | | AA | QASQDIFNYLN | DASNLET | QQYDNLPIT |
| | | | SEQ ID NO:678 | SEQ ID NO:8690 | SEQ ID NO:16702 |
| **iPS:393140** | **21-225_35H12** | NA | CGGGCGAGTCAGGGTATTAGCAGATGGTTAGCC | GCTGCATCCCGTTTGCAAAGT | CAACAGGCTAACAGTTTCCCATTCACT |
| | | | SEQ ID NO:679 | SEQ ID NO:8691 | SEQ ID NO:16703 |
| | | AA | RASQGISRWLA | AASRLQS | QQANSFPFT |
| | | | SEQ ID NO:680 | SEQ ID NO:8692 | SEQ ID NO:16704 |
| **iPS:393142** | **21-225_33A3** | NA | CGGGCGAGTCAGGGCATTAACAATTATTTAGCC | GCTGCATCCAGTTTGCAAAGT | CAACAGTTTAATAGTTACCCTCCGACG |
| | | | SEQ ID NO:681 | SEQ ID NO:8693 | SEQ ID NO:16705 |
| | | AA | RASQGINNYLA | AASSLQS | QQFNSYPPT |
| | | | SEQ ID NO:682 | SEQ ID NO:8694 | SEQ ID NO:16706 |
| **iPS:393144** | **21-225_34D2** | NA | AAGTCTAGTCAGAGCCTCCTGCATAGTGATGGAAAGACCTATTTGTAT | GAAGTTTCCAACCGGTTCTCT | ATGCAAAGTAAACAGCTTCCTCCT |
| | | | SEQ ID NO:683 | SEQ ID NO:8695 | SEQ ID NO:16707 |
| | | AA | KSSQSLLHSDGKTYLY | EVSNRFS | MQSKQLPP |

FIGURE 49

| | | | | | |
|---|---|---|---|---|---|
| | | | SEQ ID NO:684 | SEQ ID NO:8696 | SEQ ID NO:16708 |
| **iPS:393146** | **21-225_34G8** | NA | CGGGCAAGTCAGGACATTAG AAGTGATTTAGGC | GCTGCATCCAGTTTGCAA AGT | CTACAGCATACTATTTACCC TCCTACT |
| | | | SEQ ID NO:685 | SEQ ID NO:8697 | SEQ ID NO:16709 |
| | | AA | RASQDIRSDLG | AASSLQS | LQHTIYPPT |
| | | | SEQ ID NO:686 | SEQ ID NO:8698 | SEQ ID NO:16710 |
| **iPS:393148** | **21-225_35E5** | NA | CGGGCAAGTCAGAGCATTAG CAGCTATTTAAAT | GGTGCATCCAGTTTCCAA AGT | CACCAGAGTTACAATCTCCC GATCACC |
| | | | SEQ ID NO:687 | SEQ ID NO:8699 | SEQ ID NO:16711 |
| | | AA | RASQSISSYLN | GASSFQS | HQSYNLPIT |
| | | | SEQ ID NO:688 | SEQ ID NO:8700 | SEQ ID NO:16712 |
| **iPS:393150** | **21-225_36A5** | NA | CGGACAAGTCAGGACATTAG AAATGATTTAGGC | GCTGCATCCAGTTTGCAA AGT | CTACACCACAATAGTTACCC TCCTAAG |
| | | | SEQ ID NO:689 | SEQ ID NO:8701 | SEQ ID NO:16713 |
| | | AA | RTSQDIRNDLG | AASSLQS | LHHNSYPPK |
| | | | SEQ ID NO:690 | SEQ ID NO:8702 | SEQ ID NO:16714 |
| **iPS:393152** | **21-225_25B3** | NA | CGGGCGAGTCAGGGTATTAG CAGCTGGTTAGCC | GGTGCATCCAGTTTGCA AAGT | CAACAGTCTGACAGTTTCCC TCGGACG |
| | | | SEQ ID NO:691 | SEQ ID NO:8703 | SEQ ID NO:16715 |
| | | AA | RASQGISSWLA | GASSLQS | QQSDSFPRT |
| | | | SEQ ID NO:692 | SEQ ID NO:8704 | SEQ ID NO:16716 |
| **iPS:393166** | **21-225_27G6** | NA | TCTGGAGATAAATTGGGGA TAAATATGCTTGC | CAAGATAGGAAGCGGCC CTCA | CAGGCGTGGGACAGCAGCTC TTATGTGGTA |
| | | | SEQ ID NO:693 | SEQ ID NO:8705 | SEQ ID NO:16717 |
| | | AA | SGDKLGDKYAC | QDRKRPS | QAWDSSSYVV |
| | | | SEQ ID NO:694 | SEQ ID NO:8706 | SEQ ID NO:16718 |
| **iPS:393168** | **21-225_32B11** | NA | TCTGGAGATAAATTGGGGA TAAATATGCTTAC | CAAGATAGTAAGCGGTC CTCA | CAGGCGTGGGACAACAGCAC TGTGGTA |
| | | | SEQ ID NO:695 | SEQ ID NO:8707 | SEQ ID NO:16719 |
| | | AA | SGDKLGDKYAY | QDSKRSS | QAWDNSTVV |

FIGURE 49

| | | | | | |
|---|---|---|---|---|---|
| | | | SEQ ID NO:696 | SEQ ID NO:8708 | SEQ ID NO:16720 |
| **iPS:393172** | **21-225_3B12** | NA | TCTGGAGATAAATTGGGGGAAAAATATGCTTGC | CAAGATAGGAAGCGGCCCTCA | CAGGCGTGGGTCAACAACACTATGATA |
| | | | SEQ ID NO:697 | SEQ ID NO:8709 | SEQ ID NO:16721 |
| | | AA | SGDKLGEKYAC | QDRKRPS | QAWVNNTMI |
| | | | SEQ ID NO:698 | SEQ ID NO:8710 | SEQ ID NO:16722 |
| **iPS:393174** | **21-225_15D8** | NA | ACCCTAAGCAGTGAGCACAGCACCTACACCATCGAA | GTTAAGAGTGATGGCAGCCACAGCAAGGGGGAC | GGAGAGAGCCACACGATCGATGGCCAAGTCGGTGTGGTA |
| | | | SEQ ID NO:699 | SEQ ID NO:8711 | SEQ ID NO:16723 |
| | | AA | TLSSEHSTYTIE | VKSDGSHSKGD | GESHTIDGQVGVV |
| | | | SEQ ID NO:700 | SEQ ID NO:8712 | SEQ ID NO:16724 |
| **iPS:393176** | **21-225_27E7** | NA | TCTGGAGATAAATTGGGGAATAAATATGCTTGC | CAAGATAGCAAGCGGCCCTTA | CAGGCGTGGGACAGTAGTACTGTGGTA |
| | | | SEQ ID NO:701 | SEQ ID NO:8713 | SEQ ID NO:16725 |
| | | AA | SGDKLGNKYAC | QDSKRPL | QAWDSSTVV |
| | | | SEQ ID NO:702 | SEQ ID NO:8714 | SEQ ID NO:16726 |
| **iPS:393178** | **21-225_34D7** | NA | TCTGGAGATAAATTGGGGGAGAAATATGCTTAC | CAAGATAGCAAGCGGCCCTCA | CAGGCGTGGGACAACACCACTGTGGTA |
| | | | SEQ ID NO:703 | SEQ ID NO:8715 | SEQ ID NO:16727 |
| | | AA | SGDKLGEKYAY | QDSKRPS | QAWDNTTVV |
| | | | SEQ ID NO:704 | SEQ ID NO:8716 | SEQ ID NO:16728 |
| **iPS:393180** | **21-225_4G12** | NA | TCTGGAACCAACTCCAACATCGGAAGTTATACTGTAAAC | ATTAATAATCAGCGGCCCTCA | GCAGCATGGGATGACAGCCTGAATGGTCATGTGGTA |
| | | | SEQ ID NO:705 | SEQ ID NO:8717 | SEQ ID NO:16729 |
| | | AA | SGTNSNIGSYTVN | INNQRPS | AAWDDSLNGHVV |
| | | | SEQ ID NO:706 | SEQ ID NO:8718 | SEQ ID NO:16730 |
| **iPS:393182** | | NA | TCTGGAGATAAATTGGGGGATAAATATGCTTGC | CAAGATCGCAAGCGGCCCTCA | CAGGCGTGGGACAACAACACTGTGATA |

FIGURE 49

| | | | | | |
|---|---|---|---|---|---|
| | **21-225_4B3** | | SEQ ID NO:707 | SEQ ID NO:8719 | SEQ ID NO:16731 |
| | | AA | SGDKLGDKYAC | QDRKRPS | QAWDNNTVI |
| | | | SEQ ID NO:708 | SEQ ID NO:8720 | SEQ ID NO:16732 |
| **iPS:393184** | **21-225_15H11** | NA | TCTGGAGATAAATTGGGGA GAAATATGCTTGC | CAAGATAGGAAGCGGCC CTCA | CAGGCGTGGGACAGCAGCAC TGCGGTA |
| | | | SEQ ID NO:709 | SEQ ID NO:8721 | SEQ ID NO:16733 |
| | | AA | SGDKLGEKYAC | QDRKRPS | QAWDSSTAV |
| | | | SEQ ID NO:710 | SEQ ID NO:8722 | SEQ ID NO:16734 |
| **iPS:393186** | **21-225_27D9** | NA | TCTGGATATAAATTGGGGA TAAATATGCTTGC | CAAGATAGCAAGCGGCC CTCA | CAGGCGTGGGTCAACAACAC TGTA |
| | | | SEQ ID NO:711 | SEQ ID NO:8723 | SEQ ID NO:16735 |
| | | AA | SGYKLGDKYAC | QDSKRPS | QAWVNNTV |
| | | | SEQ ID NO:712 | SEQ ID NO:8724 | SEQ ID NO:16736 |
| **iPS:393188** | **21-225_34B9** | NA | TCTGGAGATAAATTGGGGA GAAATATGTTTCC | CAAGATAGTAAGCGGCC CTCA | CAGGCGTGGGACAGCAGCAC TGTA |
| | | | SEQ ID NO:713 | SEQ ID NO:8725 | SEQ ID NO:16737 |
| | | AA | SGDKLGEKYVS | QDSKRPS | QAWDSSTV |
| | | | SEQ ID NO:714 | SEQ ID NO:8726 | SEQ ID NO:16738 |
| **iPS:393192** | **21-225_12B1** | NA | TCTGGAGATAAATTGGGGA TAAATATGCTTGC | CAAGATCGCAAGCGGCC CTCA | CAGGCGTGGGACAACAACAC TGTGATA |
| | | | SEQ ID NO:715 | SEQ ID NO:8727 | SEQ ID NO:16739 |
| | | AA | SGDKLGDKYAC | QDRKRPS | QAWDNNTVI |
| | | | SEQ ID NO:716 | SEQ ID NO:8728 | SEQ ID NO:16740 |
| **iPS:393194** | **21-225_16D2** | NA | TCTGGAGATAAATTGGGGA TAAATATGCTTGC | CAAGATAGCAAGCGGCC CTCA | CAGGCGTGGGACAGCAGCAC TTATGTGGTA |
| | | | SEQ ID NO:717 | SEQ ID NO:8729 | SEQ ID NO:16741 |
| | | AA | SGDKLGDKYAC | QDSKRPS | QAWDSSTYVV |
| | | | SEQ ID NO:718 | SEQ ID NO:8730 | SEQ ID NO:16742 |
| **iPS:393196** | | NA | TCTGGAGATAAATTGGGGA AAAATATGCTTGC | CAAGATAGGAAGCGGCC CTCA | CAGGCGTGGGTCAATAACAC TATGATA |

**FIGURE 49**

| | | | | | |
|---|---|---|---|---|---|
| | **21-225_16G8** | | SEQ ID NO:719 | SEQ ID NO:8731 | SEQ ID NO:16743 |
| | | AA | SGDKLGEKYAC | QDRKRPS | QAWVNNTMI |
| | | | SEQ ID NO:720 | SEQ ID NO:8732 | SEQ ID NO:16744 |
| **iPS:393198** | **21-225_28A11** | NA | TCTGGAGATAAATTGGGGGATAAATATGCTTGC | CAAGATAGCAAGCGGCCCTCA | CAGGCGTGGGACAGTAGCACTTATGTGGTA |
| | | | SEQ ID NO:721 | SEQ ID NO:8733 | SEQ ID NO:16745 |
| | | AA | SGDKLGDKYAC | QDSKRPS | QAWDSSTYVV |
| | | | SEQ ID NO:722 | SEQ ID NO:8734 | SEQ ID NO:16746 |
| **iPS:393200** | **21-225_35E1** | NA | TCTGGAGATAAATTGGGGGAAAAATATGCTTAC | CAAGATAGGAAGCGGCCCTCA | CAGGCGTGGGACAACAGCACTGCGGTA |
| | | | SEQ ID NO:723 | SEQ ID NO:8735 | SEQ ID NO:16747 |
| | | AA | SGDKLGEKYAY | QDRKRPS | QAWDNSTAV |
| | | | SEQ ID NO:724 | SEQ ID NO:8736 | SEQ ID NO:16748 |
| **iPS:393202** | **21-225_6B4** | NA | TCTGGAGATAAATTGGGGGATAAATATGCTTGC | CAAGATCGCAAGCGGCCCTCA | CAGGCGTGGGACAACAACACTGTGATA |
| | | | SEQ ID NO:725 | SEQ ID NO:8737 | SEQ ID NO:16749 |
| | | AA | SGDKLGDKYAC | QDRKRPS | QAWDNNTVI |
| | | | SEQ ID NO:726 | SEQ ID NO:8738 | SEQ ID NO:16750 |
| **iPS:393204** | **21-225_8C12** | NA | GGGGGAAACAACATTGGAAGTAAAGCTGTGCAC | AGCGATAGCAACCGGCCCTCA | CAGGTGTGGGACAGTAGTAGTGATCATGTGGTA |
| | | | SEQ ID NO:727 | SEQ ID NO:8739 | SEQ ID NO:16751 |
| | | AA | GGNNIGSKAVH | SDSNRPS | QVWDSSSDHVV |
| | | | SEQ ID NO:728 | SEQ ID NO:8740 | SEQ ID NO:16752 |
| **iPS:393206** | **21-225_13F6** | NA | TCTGGAGATAAATTGGGGGATAAATATGCTTGC | CAAGATAGCAAGCGGCCCTCA | CAGGCGTGGGGCAACAGCACTGCTGTGGTA |
| | | | SEQ ID NO:729 | SEQ ID NO:8741 | SEQ ID NO:16753 |
| | | AA | SGDKLGDKYAC | QDSKRPS | QAWGNSTAVV |
| | | | SEQ ID NO:730 | SEQ ID NO:8742 | SEQ ID NO:16754 |
| **iPS:393208** | | NA | GGGGGAAACAACATTGGAAGTAAAAGTGTGCAC | GATGATACCGACCGGCCCTCA | CAGGTGTGGGATAGTAGCAGTGATCATGTGGTA |

# FIGURE 49

| | | | | | |
|---|---|---|---|---|---|
| | 21-225_16F3 | | SEQ ID NO:731 | SEQ ID NO:8743 | SEQ ID NO:16755 |
| | | AA | GGNNIGSKSVH | DDTDRPS | QVWDSSSDHVV |
| | | | SEQ ID NO:732 | SEQ ID NO:8744 | SEQ ID NO:16756 |
| **iPS:393210** | **21-225_17D3** | NA | TCTGGAGATAAATTGGGGA TAAATATGTTTAC | CAAGATAGGAAGCGGCC CTCA | CAGGCGTGGGACAGCATCAC TGCAGTA |
| | | | SEQ ID NO:733 | SEQ ID NO:8745 | SEQ ID NO:16757 |
| | | AA | SGDKLGDKYVY | QDRKRPS | QAWDSITAV |
| | | | SEQ ID NO:734 | SEQ ID NO:8746 | SEQ ID NO:16758 |
| **iPS:393212** | **21-225_30H6** | NA | TCTGGAGATAAATTGGGTAA TAAATATGCTTGC | CAAGATAGCAAGCGGCC CTCA | CAGGCGTGGGACAGCAGCAC TGTT |
| | | | SEQ ID NO:735 | SEQ ID NO:8747 | SEQ ID NO:16759 |
| | | AA | SGDKLGNKYAC | QDSKRPS | QAWDSSTV |
| | | | SEQ ID NO:736 | SEQ ID NO:8748 | SEQ ID NO:16760 |
| **iPS:393214** | **21-225_33A1** | NA | TCTGGAGATAAATTGGGGA TAAATTTGTTTAT | CAAGATAGCAAGCGGCC CTCA | CAGGCGTGGGACAGCACCAC CGTGGTA |
| | | | SEQ ID NO:737 | SEQ ID NO:8749 | SEQ ID NO:16761 |
| | | AA | SGDKLGDKFVY | QDSKRPS | QAWDSTTVV |
| | | | SEQ ID NO:738 | SEQ ID NO:8750 | SEQ ID NO:16762 |
| **iPS:393218** | **21-225_14G3** | NA | TCTGGAGATAAATTGGGGA TAAATATGTTTGC | CAAGATCGCAAGCGGCC CTCA | CAGGCGTGGGGCAACAGCAC TGCTGTGGTA |
| | | | SEQ ID NO:739 | SEQ ID NO:8751 | SEQ ID NO:16763 |
| | | AA | SGDKLGDKYVC | QDRKRPS | QAWGNSTAVV |
| | | | SEQ ID NO:740 | SEQ ID NO:8752 | SEQ ID NO:16764 |
| **iPS:393222** | **21-225_17F5** | NA | TCTGGAGATAAATTGGGGA AAAATATGCTTGC | CAAGATAGAAAGCGGCC CTCA | CAGGCGTGGGACAGCAGCAC GGTA |
| | | | SEQ ID NO:741 | SEQ ID NO:8753 | SEQ ID NO:16765 |
| | | AA | SGDKLGEKYAC | QDRKRPS | QAWDSSTV |
| | | | SEQ ID NO:742 | SEQ ID NO:8754 | SEQ ID NO:16766 |
| **iPS:393224** | | NA | TCTGGAGATAAATTGGGAAA TAAATATGCTTGC | CAAGATTCCAAGCGGCC CTCA | CAGGCGTGGGACAGCAGCAC TGTA |

**FIGURE 49**

| | | | | | |
|---|---|---|---|---|---|
| | **21-225_31C2** | | SEQ ID NO:743 | SEQ ID NO:8755 | SEQ ID NO:16767 |
| | | AA | SGDKLGNKYAC | QDSKRPS | QAWDSSTV |
| | | | SEQ ID NO:744 | SEQ ID NO:8756 | SEQ ID NO:16768 |
| **iPS:393226** | **21-225_33E6** | NA | TCTGGAGATAAATTGGGGGATAAATATGCTTAC | CAAGATAGGAAGCGGCCCTCA | CAGGCGTGGGACAACAGCACTGCGGTA |
| | | | SEQ ID NO:745 | SEQ ID NO:8757 | SEQ ID NO:16769 |
| | | AA | SGDKLGDKYAY | QDRKRPS | QAWDNSTAV |
| | | | SEQ ID NO:746 | SEQ ID NO:8758 | SEQ ID NO:16770 |
| **iPS:393230** | **21-225_9G9** | NA | TCTGGAACCAACTCCAACATCGGAAGTTATACTGTAAAC | ATTAATAATCAGCGGCCCTCA | GCAGCATGGGATGACAGCCTGAATGGTCATGTGGTA |
| | | | SEQ ID NO:747 | SEQ ID NO:8759 | SEQ ID NO:16771 |
| | | AA | SGTNSNIGSYTVN | INNQRPS | AAWDDSLNGHVV |
| | | | SEQ ID NO:748 | SEQ ID NO:8760 | SEQ ID NO:16772 |
| **iPS:393232** | **21-225_17F12** | NA | ACTGGAGCCAGCAGTGACGTTGGTGATTATAACTCTGTCTCC | GAGGTCAGTAATCGGCCCTCA | AGCTCATATACAAGCAGCATCACTGTGGTA |
| | | | SEQ ID NO:749 | SEQ ID NO:8761 | SEQ ID NO:16773 |
| | | AA | TGASSDVGDYNSVS | EVSNRPS | SSYTSSITVV |
| | | | SEQ ID NO:750 | SEQ ID NO:8762 | SEQ ID NO:16774 |
| **iPS:393234** | **21-225_26C10** | NA | TCTGGAGATAAATTGGGGGATAAATATGTTTGC | CAAGATAGCAAGCGGCCCTCA | CAGGCGTGGGTCAACAACACTGTA |
| | | | SEQ ID NO:751 | SEQ ID NO:8763 | SEQ ID NO:16775 |
| | | AA | SGDKLGDKYVC | QDSKRPS | QAWVNNTV |
| | | | SEQ ID NO:752 | SEQ ID NO:8764 | SEQ ID NO:16776 |
| **iPS:393345** | **21-225_5G7** | NA | TCTGGAGATAAATTGGGGAATAAATATGCTTGG | CAAGATAGGAAGCGGCCCTCA | CAGGCGTGGGACAACAGCACTGTGGTT |
| | | | SEQ ID NO:753 | SEQ ID NO:8765 | SEQ ID NO:16777 |
| | | AA | SGDKLGNKYAW | QDRKRPS | QAWDNSTVV |
| | | | SEQ ID NO:754 | SEQ ID NO:8766 | SEQ ID NO:16778 |

FIGURE 49

| | | | | | |
|---|---|---|---|---|---|
| **iPS:393368** | **21-225_29H8** | NA | AGGTCCAGCCAGACTATTTTACACAGCTCCAACAATTACAACTACTTAGCT | TGGGCATCTACCCGGGAATCC | CAGCAATATTATTGTACTCCTCCGACG |
| | | | SEQ ID NO:755 | SEQ ID NO:8767 | SEQ ID NO:16779 |
| | | AA | RSSQTILHSSNNYNYLA | WASTRES | QQYYCTPPT |
| | | | SEQ ID NO:756 | SEQ ID NO:8768 | SEQ ID NO:16780 |
| **iPS:393565** | **21-225_34B11** | NA | TCTGGAGATAAATTGGGGGATAAATATGCTTGC | CAAGATATGAAGCGGCCCTCA | CAGGCGTGGGACAACAGCACTGCGGTA |
| | | | SEQ ID NO:757 | SEQ ID NO:8769 | SEQ ID NO:16781 |
| | | AA | SGDKLGDKYAC | QDMKRPS | QAWDNSTAV |
| | | | SEQ ID NO:758 | SEQ ID NO:8770 | SEQ ID NO:16782 |
| **iPS:393802** | **21-225_3D12** | NA | AGGGCCAGTCAGAGTGTTAGCAGCAGCTACTTAGCC | GGTACATCCAGCAGGGCCACT | CAGCAGTATGGTAGTTCACGCAGT |
| | | | SEQ ID NO:759 | SEQ ID NO:8771 | SEQ ID NO:16783 |
| | | AA | RASQSVSSSYLA | GTSSRAT | QQYGSSRS |
| | | | SEQ ID NO:760 | SEQ ID NO:8772 | SEQ ID NO:16784 |
| **iPS:393804** | **21-225_5H7** | NA | CGGGCAAGTCAGGGCATTAGAAATGATTTAGGC | GTTACATCCAGTTTGCAAGGT | CTACAACATAATAGTTACCCGCTCACT |
| | | | SEQ ID NO:761 | SEQ ID NO:8773 | SEQ ID NO:16785 |
| | | AA | RASQGIRNDLG | VTSSLQG | LQHNSYPLT |
| | | | SEQ ID NO:762 | SEQ ID NO:8774 | SEQ ID NO:16786 |
| **iPS:393806** | **21-225_6A6** | NA | CGGGCAAGTCAGGGCATTAGAAATGATTTAGGC | GCTGCATCCAGTTTGCAAAGT | CTACAGCATAGTAGTTACCCGCTCACT |
| | | | SEQ ID NO:763 | SEQ ID NO:8775 | SEQ ID NO:16787 |
| | | AA | RASQGIRNDLG | AASSLQS | LQHSSYPLT |
| | | | SEQ ID NO:764 | SEQ ID NO:8776 | SEQ ID NO:16788 |
| **iPS:393808** | **21-225_1A2** | NA | CGGGCAAGTCAGGGCATTAGAAATGATTTAGGC | GCTGCATCCAGTTTGCAAAGT | CTACAGCATAATAGTCACCCTCTCACT |
| | | | SEQ ID NO:765 | SEQ ID NO:8777 | SEQ ID NO:16789 |
| | | AA | RASQGIRNDLG | AASSLQS | LQHNSHPLT |

FIGURE 49

| | | | | | |
|---|---|---|---|---|---|
| | | | SEQ ID NO:766 | SEQ ID NO:8778 | SEQ ID NO:16790 |
| **iPS:393810** | **21-225_5A4** | NA | CGGGCGAGTCAGGGTATTAG CACCTGGTTAGCC | GATGCATCCAGTTTGCA AAGT | CAACAGGCTAACAGTTTCCC GTGGACG |
| | | | SEQ ID NO:767 | SEQ ID NO:8779 | SEQ ID NO:16791 |
| | | AA | RASQGISTWLA | DASSLQS | QQANSFPWT |
| | | | SEQ ID NO:768 | SEQ ID NO:8780 | SEQ ID NO:16792 |
| **iPS:393812** | **21-225_6A11** | NA | CGGGCAAGTCAGGGCATTAG AAATGATTTAGGC | GCTGCATCCAGTTTGCAA AGT | CTACAGCATAATAGTTACCC GTGGACG |
| | | | SEQ ID NO:769 | SEQ ID NO:8781 | SEQ ID NO:16793 |
| | | AA | RASQGIRNDLG | AASSLQS | LQHNSYPWT |
| | | | SEQ ID NO:770 | SEQ ID NO:8782 | SEQ ID NO:16794 |
| **iPS:393814** | **21-225_7F4** | NA | CGGACAAGTCAGGGCATTAG AAATGATTTAGGC | GCTGCATCCAATTTGCAA AGT | CTACAGCATAGTGCTTACCC GCTCACT |
| | | | SEQ ID NO:771 | SEQ ID NO:8783 | SEQ ID NO:16795 |
| | | AA | RTSQGIRNDLG | AASNLQS | LQHSAYPLT |
| | | | SEQ ID NO:772 | SEQ ID NO:8784 | SEQ ID NO:16796 |
| **iPS:393816** | **21-225_6D4** | NA | CGGGCAAGTCAGGCCATTAG AAATGATTTAGGC | GCTGCATCCAGTTTGCAA AGT | CTACAGCATAGTAGTTACCC GCTCACT |
| | | | SEQ ID NO:773 | SEQ ID NO:8785 | SEQ ID NO:16797 |
| | | AA | RASQAIRNDLG | AASSLQS | LQHSSYPLT |
| | | | SEQ ID NO:774 | SEQ ID NO:8786 | SEQ ID NO:16798 |
| **iPS:393818** | **21-225_6G12** | NA | CGGGCAAGTCAGGGCATTAG AAATGATTTAGGC | GCTGCATCCACTTTGCAA AGT | CTACAGCATAATAGTTACCC GCTCACT |
| | | | SEQ ID NO:775 | SEQ ID NO:8787 | SEQ ID NO:16799 |
| | | AA | RASQGIRNDLG | AASTLQS | LQHNSYPLT |
| | | | SEQ ID NO:776 | SEQ ID NO:8788 | SEQ ID NO:16800 |
| **iPS:393820** | **21-225_8H7** | NA | CGGGCAAGTCAGGGCATTAG AAATGATTTAGGC | GCTGCATCCAGTTTGCAA AGT | CTACAGCATAATAGTTACCC GTTCACT |
| | | | SEQ ID NO:777 | SEQ ID NO:8789 | SEQ ID NO:16801 |
| | | AA | RASQGIRNDLG | AASSLQS | LQHNSYPFT |

FIGURE 49

| | | | | | |
|---|---|---|---|---|---|
| | | | SEQ ID NO:778 | SEQ ID NO:8790 | SEQ ID NO:16802 |
| **iPS:393822** | **21-225_15B11** | NA | CGGGCAAGTCAGGGCATTAG AAATGATTTAGGC | GCTGCATCCAGTTTGCAA AGT | CTACAGCATAATAGTTACCC ATTCACT |
| | | | SEQ ID NO:779 | SEQ ID NO:8791 | SEQ ID NO:16803 |
| | | AA | RASQGIRNDLG | AASSLQS | LQHNSYPFT |
| | | | SEQ ID NO:780 | SEQ ID NO:8792 | SEQ ID NO:16804 |
| **iPS:393824** | **21-225_10F12** | NA | CGGGCAAGTCAGAACATTAG TAGTTATTTAAAT | GCTGCATCCAGTTTGCAA AGT | CAACAGAGTTACAATACCCC CTTCTTCACT |
| | | | SEQ ID NO:781 | SEQ ID NO:8793 | SEQ ID NO:16805 |
| | | AA | RASQNISSYLN | AASSLQS | QQSYNTPFFT |
| | | | SEQ ID NO:782 | SEQ ID NO:8794 | SEQ ID NO:16806 |
| **iPS:393826** | **21-225_10G5** | NA | CGGGCAAGTCAGGGCATTAG AAATGATTTAGGC | GCTGCATCCAGTTTGCAA AGT | CTACAGCATAATAGTTACCC GCTCACT |
| | | | SEQ ID NO:783 | SEQ ID NO:8795 | SEQ ID NO:16807 |
| | | AA | RASQGIRNDLG | AASSLQS | LQHNSYPLT |
| | | | SEQ ID NO:784 | SEQ ID NO:8796 | SEQ ID NO:16808 |
| **iPS:393828** | **21-225_10H12** | NA | CGGGCAAGTCAGGGCATTAG AAATGATTTAGGC | GGTGCATCCAGTTTGCA AAGT | CTACAGCATAATAGTTACCC GCTCACT |
| | | | SEQ ID NO:785 | SEQ ID NO:8797 | SEQ ID NO:16809 |
| | | AA | RASQGIRNDLG | GASSLQS | LQHNSYPLT |
| | | | SEQ ID NO:786 | SEQ ID NO:8798 | SEQ ID NO:16810 |
| **iPS:393830** | **21-225_12A1** | NA | CGGGCAAGTCAGGGCATTAG AAATGATTTAGGC | GCTGCATCCAGTTTGCAA AGT | CTACAGCATAATAGTTACCC GCTCACT |
| | | | SEQ ID NO:787 | SEQ ID NO:8799 | SEQ ID NO:16811 |
| | | AA | RASQGIRNDLG | AASSLQS | LQHNSYPLT |
| | | | SEQ ID NO:788 | SEQ ID NO:8800 | SEQ ID NO:16812 |
| **iPS:393832** | **21-225_14B2** | NA | CGGGCAAGTCAGGGCATTAG AAATGATTTAGGC | GTTACATCCAGTTTGCAA GGT | CTACAACATAATAGTTACCC GCTCACT |
| | | | SEQ ID NO:789 | SEQ ID NO:8801 | SEQ ID NO:16813 |
| | | AA | RASQGIRNDLG | VTSSLQG | LQHNSYPLT |

FIGURE 49

| | | | | | |
|---|---|---|---|---|---|
| | | | SEQ ID NO:790 | SEQ ID NO:8802 | SEQ ID NO:16814 |
| **iPS:393836** | **21-225_15A2** | NA | CGGGCGAGTCAGGGCATTAG CAATTATTTAGCC | GCTGCATCCAGTTTGCAA AGT | CAACAGTATTATAGTTACCC ATTCACT |
| | | | SEQ ID NO:791 | SEQ ID NO:8803 | SEQ ID NO:16815 |
| | | AA | RASQGISNYLA | AASSLQS | QQYYSYPFT |
| | | | SEQ ID NO:792 | SEQ ID NO:8804 | SEQ ID NO:16816 |
| **iPS:393838** | **21-225_6G2** | NA | CGGGCAAGTCAGGGCATTAG AAATGATTTAGGC | GCTGCATCCAGTCTGCA AAGT | ATACAGCATAATAGTTACCT GTGGACG |
| | | | SEQ ID NO:793 | SEQ ID NO:8805 | SEQ ID NO:16817 |
| | | AA | RASQGIRNDLG | AASSLQS | IQHNSYLWT |
| | | | SEQ ID NO:794 | SEQ ID NO:8806 | SEQ ID NO:16818 |
| **iPS:393840** | **21-225_3F8** | NA | CGGGCAAGTCAGAGTATTCT CAGCTATTTAAAT | ACTACATCCAGTTTGCAA AGT | CAACAGACTTACAGTACCCC GCTCACT |
| | | | SEQ ID NO:795 | SEQ ID NO:8807 | SEQ ID NO:16819 |
| | | AA | RASQSILSYLN | TTSSLQS | QQTYSTPLT |
| | | | SEQ ID NO:796 | SEQ ID NO:8808 | SEQ ID NO:16820 |
| **iPS:393844** | **21-225_3G7** | NA | CGGGCAAGTCAGAACATTTA CAGGTATTTAAAT | GCTGCATCCAGTTCGCA AAGT | CAACAGAGTTACAGTCCCCC TTTCACT |
| | | | SEQ ID NO:797 | SEQ ID NO:8809 | SEQ ID NO:16821 |
| | | AA | RASQNIYRYLN | AASSSQS | QQSYSPPFT |
| | | | SEQ ID NO:798 | SEQ ID NO:8810 | SEQ ID NO:16822 |
| **iPS:393848** | **21-225_4H2** | NA | CGGGCAATTCAGAACATTAG CAGCTATTTAAAT | GCTGCATCCAGCTTGCA AAGT | CAACAGAGTTACAGAACCCC CTTATTCACT |
| | | | SEQ ID NO:799 | SEQ ID NO:8811 | SEQ ID NO:16823 |
| | | AA | RAIQNISSYLN | AASSLQS | QQSYRTPLFT |
| | | | SEQ ID NO:800 | SEQ ID NO:8812 | SEQ ID NO:16824 |
| **iPS:393852** | **21-225_12A10** | NA | CGGGCAAGTCAGAACATTTA CAGCTATTTAAAT | ACTGCATCCAGTTTGCAA AGT | CAGCAGAGTTACAGTCCCCC TCTCACT |
| | | | SEQ ID NO:801 | SEQ ID NO:8813 | SEQ ID NO:16825 |
| | | AA | RASQNIYSYLN | TASSLQS | QQSYSPPLT |

FIGURE 49

| | | | | | |
|---|---|---|---|---|---|
| | | | SEQ ID NO:802 | SEQ ID NO:8814 | SEQ ID NO:16826 |
| **iPS:393854** | **21-225_7H11** | NA | CTGGCAAGTCAGGGCATTAG AAATGATTTAGGC | GTTGCATGTAGTTTCCAA AGT | CTACAACATAATCTTTACCC GCTCACT |
| | | | SEQ ID NO:803 | SEQ ID NO:8815 | SEQ ID NO:16827 |
| | | AA | LASQGIRNDLG | VACSFQS | LQHNLYPLT |
| | | | SEQ ID NO:804 | SEQ ID NO:8816 | SEQ ID NO:16828 |
| **iPS:393856** | **21-225_14C2** | NA | CGGGCAAGTCAGGGCATTAG AAATGATTTAGAC | GCTGCATCCAGTTTGCAA AGT | GTACAGCATAATAGTTACCC GCTCACT |
| | | | SEQ ID NO:805 | SEQ ID NO:8817 | SEQ ID NO:16829 |
| | | AA | RASQGIRNDLD | AASSLQS | VQHNSYPLT |
| | | | SEQ ID NO:806 | SEQ ID NO:8818 | SEQ ID NO:16830 |
| **iPS:393862** | **21-225_5G2** | NA | CGGGCAAGTCAGAACATTAT TAGTTATTTAAAT | GGTGCATCCAGTTTGCA AAGT | CAACAGAGTTACAGTACTCC CTTATTCACT |
| | | | SEQ ID NO:807 | SEQ ID NO:8819 | SEQ ID NO:16831 |
| | | AA | RASQNIISYLN | GASSLQS | QQSYSTPLFT |
| | | | SEQ ID NO:808 | SEQ ID NO:8820 | SEQ ID NO:16832 |
| **iPS:393864** | **21-225_4C5** | NA | CGGGCAAGTCGGGGCATCA GAGGTGATTTAGGT | GCTGCATCCAATTTGCAA AGT | CTACAGCATTATAGTTACCC TCGGACG |
| | | | SEQ ID NO:809 | SEQ ID NO:8821 | SEQ ID NO:16833 |
| | | AA | RASRGIRGDLG | AASNLQS | LQHYSYPRT |
| | | | SEQ ID NO:810 | SEQ ID NO:8822 | SEQ ID NO:16834 |
| **iPS:393866** | **21-225_14E3** | NA | CGGGCAAGTCAGGGCATTAG AAATGATTTAGGC | TCTGCATCCAGTTTGCAA AGT | GTACAGCATTATAGTTACCC GTTCACT |
| | | | SEQ ID NO:811 | SEQ ID NO:8823 | SEQ ID NO:16835 |
| | | AA | RASQGIRNDLG | SASSLQS | VQHYSYPFT |
| | | | SEQ ID NO:812 | SEQ ID NO:8824 | SEQ ID NO:16836 |
| **iPS:393868** | **21-225_9C11** | NA | CGGGCAAGTCAGAACATTAG AAATTATTTAAAT | GTTGCATCCAGTTTGCAA AGT | CATCAGAGTAACAGTACTCC TCTCACG |
| | | | SEQ ID NO:813 | SEQ ID NO:8825 | SEQ ID NO:16837 |
| | | AA | RASQNIRNYLN | VASSLQS | HQSNSTPLT |

**FIGURE 49**

| | | | | | |
|---|---|---|---|---|---|
| | | | SEQ ID NO:814 | SEQ ID NO:8826 | SEQ ID NO:16838 |
| **iPS:393870** | **21-225_7B1** | NA | CGGGCGAGTCAGGACATTAG CAATCATTTAGTC | GCTGCATCCAGTTTACAA AGT | CACCAGTATAATAGTTACCC CTTCACT |
| | | | SEQ ID NO:815 | SEQ ID NO:8827 | SEQ ID NO:16839 |
| | | AA | RASQDISNHLV | AASSLQS | HQYNSYPFT |
| | | | SEQ ID NO:816 | SEQ ID NO:8828 | SEQ ID NO:16840 |
| **iPS:393872** | **21-225_2A11** | NA | CGGGCAAGTCAGGGCATTAG AAATGATTTAGGC | GCTGCATCCAGTTTGCAA AGT | CTACAGCATAATAGTTACCC GCTCACT |
| | | | SEQ ID NO:817 | SEQ ID NO:8829 | SEQ ID NO:16841 |
| | | AA | RASQGIRNDLG | AASSLQS | LQHNSYPLT |
| | | | SEQ ID NO:818 | SEQ ID NO:8830 | SEQ ID NO:16842 |
| **iPS:393874** | **21-225_4C8** | NA | CGGGCAAGTCAGGGCATTAG AAATGATTTAGGC | GCTGCATCCAGTTTGCAA AGT | CTACAGCATAATAGTTACCC GCTCACT |
| | | | SEQ ID NO:819 | SEQ ID NO:8831 | SEQ ID NO:16843 |
| | | AA | RASQGIRNDLG | AASSLQS | LQHNSYPLT |
| | | | SEQ ID NO:820 | SEQ ID NO:8832 | SEQ ID NO:16844 |
| **iPS:393876** | **21-225_9A1** | NA | CGGGCAAGTCAGGGCATTAG AAATGATTTAGGG | ACTGCATCCAGTTTGCAA AGT | ATACAGCATAATAGTTACCT GTGGACG |
| | | | SEQ ID NO:821 | SEQ ID NO:8833 | SEQ ID NO:16845 |
| | | AA | RASQGIRNDLG | TASSLQS | IQHNSYLWT |
| | | | SEQ ID NO:822 | SEQ ID NO:8834 | SEQ ID NO:16846 |
| **iPS:393878** | **21-225_7G12** | NA | CGGGCAAGTCAGAATATTAA CAACTATTTAAAT | ACTGCATCCAGTTTGCAA AGT | CAACAGAGTTACACTACCCC CACGTGGACG |
| | | | SEQ ID NO:823 | SEQ ID NO:8835 | SEQ ID NO:16847 |
| | | AA | RASQNINNYLN | TASSLQS | QQSYTTPTWT |
| | | | SEQ ID NO:824 | SEQ ID NO:8836 | SEQ ID NO:16848 |
| **iPS:393880** | **21-225_15A1** | NA | CGGGCAAGTCAGGGCATTAG AAATGATTTAGGC | GCTGCATCCAGTTTACAA AGT | CTACACAACAGTAGTTACCC TGTTAAG |
| | | | SEQ ID NO:825 | SEQ ID NO:8837 | SEQ ID NO:16849 |
| | | AA | RASQGIRNDLG | AASSLQS | LHNSSYPVK |

**FIGURE 49**

| | | | | | |
|---|---|---|---|---|---|
| | | | SEQ ID NO:826 | SEQ ID NO:8838 | SEQ ID NO:16850 |
| **iPS:393882** | **21-225_15E3** | NA | CGGGCAAGTCAGGGCATTAG AAATGATTTAGGC | GCTGCATCCAGTTCGCA AAGT | CTACAGCATCATAGTTACCC GCTCACT |
| | | | SEQ ID NO:827 | SEQ ID NO:8839 | SEQ ID NO:16851 |
| | | AA | RASQGIRNDLG | AASSSQS | LQHHSYPLT |
| | | | SEQ ID NO:828 | SEQ ID NO:8840 | SEQ ID NO:16852 |
| **iPS:393884** | **21-225_16F4** | NA | CGGGCAAGTCAGGGCATTAG AAATGATTTAGGC | GTTGCATCCAGTTTGCAA AGT | ATACAGCATAATAGTTATCC GTTCACT |
| | | | SEQ ID NO:829 | SEQ ID NO:8841 | SEQ ID NO:16853 |
| | | AA | RASQGIRNDLG | VASSLQS | IQHNSYPFT |
| | | | SEQ ID NO:830 | SEQ ID NO:8842 | SEQ ID NO:16854 |
| **iPS:393886** | **21-225_2G9** | NA | CGGGCAAGTCAGGGCATTAG AAATGATTTAGGT | GCTGCATCCAGTTTGCAA AGT | TTACAGCATGAAAGTTACCC TCTCACT |
| | | | SEQ ID NO:831 | SEQ ID NO:8843 | SEQ ID NO:16855 |
| | | AA | RASQGIRNDLG | AASSLQS | LQHESYPLT |
| | | | SEQ ID NO:832 | SEQ ID NO:8844 | SEQ ID NO:16856 |
| **iPS:393888** | **21-225_3E3** | NA | CGGGCAAGTCAGAGCATTAG AAGTTATTTAAAT | GGTACATCCAGTTTGCA AAGT | CAACAGAGTTACAGTACCCC CTTGTTCACT |
| | | | SEQ ID NO:833 | SEQ ID NO:8845 | SEQ ID NO:16857 |
| | | AA | RASQSIRSYLN | GTSSLQS | QQSYSTPLFT |
| | | | SEQ ID NO:834 | SEQ ID NO:8846 | SEQ ID NO:16858 |
| **iPS:393890** | **21-225_4B1** | NA | CGGGCAAGTCATACCATTAG AACCTATTTAAAC | GCTGCATCCAGTTTGCAA AGT | CAACAGAGTTACAATATCTC ATTCACT |
| | | | SEQ ID NO:835 | SEQ ID NO:8847 | SEQ ID NO:16859 |
| | | AA | RASHTIRTYLN | AASSLQS | QQSYNISFT |
| | | | SEQ ID NO:836 | SEQ ID NO:8848 | SEQ ID NO:16860 |
| **iPS:393892** | **21-225_6G7** | NA | CAGGCGAGTCAGGACATTAG CAACTATTTAAAT | GATGCATCCACTTTGGA AACA | CAACAGTATGATAATGTCCC GATCACC |
| | | | SEQ ID NO:837 | SEQ ID NO:8849 | SEQ ID NO:16861 |
| | | AA | QASQDISNYLN | DASTLET | QQYDNVPIT |

FIGURE 49

| | | | | | |
|---|---|---|---|---|---|
| | | | SEQ ID NO:838 | SEQ ID NO:8850 | SEQ ID NO:16862 |
| **iPS:393894** | **21-225_5E11** | NA | CGGGCGAGTCAGGGCATTAGCAATTATTTAGCC | GCTGCATCCAGTGTGCAGAGT | CACCAGTATCACAGTTACCCATTCACT |
| | | | SEQ ID NO:839 | SEQ ID NO:8851 | SEQ ID NO:16863 |
| | | AA | RASQGISNYLA | AASSVQS | HQYHSYPFT |
| | | | SEQ ID NO:840 | SEQ ID NO:8852 | SEQ ID NO:16864 |
| **iPS:393896** | **21-225_2A4** | NA | CGGGCGAGTCAGGGCATTAGCAATTATTTAGCC | ACTGCATCCAGTTTGCAAAGT | CAACAGTATAATAGTTACCCATTCACT |
| | | | SEQ ID NO:841 | SEQ ID NO:8853 | SEQ ID NO:16865 |
| | | AA | RASQGISNYLA | TASSLQS | QQYNSYPFT |
| | | | SEQ ID NO:842 | SEQ ID NO:8854 | SEQ ID NO:16866 |
| **iPS:393898** | **21-225_5F7** | NA | CGGGCAAGTCAGACCATTAGTAGTTATTTAAAT | GCTGCATCCAGTTTGCAAAGT | CAACAGAGTTACAATACCCCCTTATTCACT |
| | | | SEQ ID NO:843 | SEQ ID NO:8855 | SEQ ID NO:16867 |
| | | AA | RASQTISSYLN | AASSLQS | QQSYNTPLFT |
| | | | SEQ ID NO:844 | SEQ ID NO:8856 | SEQ ID NO:16868 |
| **iPS:393900** | **21-225_10E12** | NA | CGGGCAAGTCAGAACATTTACAGTTATTTAAAT | GCTACATCCAGTTTGCAAAGT | CAACAGAATTACAGTCCCCCTCTCACT |
| | | | SEQ ID NO:845 | SEQ ID NO:8857 | SEQ ID NO:16869 |
| | | AA | RASQNIYSYLN | ATSSLQS | QQNYSPPLT |
| | | | SEQ ID NO:846 | SEQ ID NO:8858 | SEQ ID NO:16870 |
| **iPS:393902** | **21-225_14E10** | NA | CGGACAAGTCAGGGCATTAGAAATGATTTAGGC | GCTGCATCCAGTTTACAAAGT | CTGCAGCATTATAGTTACCCTCGGACG |
| | | | SEQ ID NO:847 | SEQ ID NO:8859 | SEQ ID NO:16871 |
| | | AA | RTSQGIRNDLG | AASSLQS | LQHYSYPRT |
| | | | SEQ ID NO:848 | SEQ ID NO:8860 | SEQ ID NO:16872 |
| **iPS:393904** | **21-225_8H11** | NA | CGGGCAAGTCAGAACATTATCAGCTATTTAAAT | GTTACATCCAGTTTGCACAGT | CAACAGAGTTACAGTACCCCTTTCACT |
| | | | SEQ ID NO:849 | SEQ ID NO:8861 | SEQ ID NO:16873 |
| | | AA | RASQNIISYLN | VTSSLHS | QQSYSTPFT |

**FIGURE 49**

| | | | | | |
|---|---|---|---|---|---|
| | | | SEQ ID NO:850 | SEQ ID NO:8862 | SEQ ID NO:16874 |
| **iPS:393906** | **21-225_13D3** | NA | AGGGCCAGTCAGACTGTTAG CAGCAACTTAGCC | GGTGCATCCACCAGGGC CACT | CAGCAGTATCATGACTGGCC TCCGACG |
| | | | SEQ ID NO:851 | SEQ ID NO:8863 | SEQ ID NO:16875 |
| | | AA | RASQTVSSNLA | GASTRAT | QQYHDWPPT |
| | | | SEQ ID NO:852 | SEQ ID NO:8864 | SEQ ID NO:16876 |
| **iPS:393908** | **21-225_10E9** | NA | CGGGCAAGTCAGGACATTAG AAGTGATTTAGGC | GCTGCATCCAGTTTGCAA AGT | CTACAGCATTATAGTTACCC TCGGACG |
| | | | SEQ ID NO:853 | SEQ ID NO:8865 | SEQ ID NO:16877 |
| | | AA | RASQDIRSDLG | AASSLQS | LQHYSYPRT |
| | | | SEQ ID NO:854 | SEQ ID NO:8866 | SEQ ID NO:16878 |
| **iPS:393910** | **21-225_15F10** | NA | CAGGCGAATCAGGACATTAC CAACTTTTTAAAT | GATGCATCCAATTTGGA AACA | CAACAGTATGATAATCTCCC GATCACC |
| | | | SEQ ID NO:855 | SEQ ID NO:8867 | SEQ ID NO:16879 |
| | | AA | QANQDITNFLN | DASNLET | QQYDNLPIT |
| | | | SEQ ID NO:856 | SEQ ID NO:8868 | SEQ ID NO:16880 |
| **iPS:393912** | **21-225_16F6** | NA | CAGGCGAATCAGGACATTAC CAACTTTTTAAAT | GATGCATCCAATTTGGA AACA | CAACAGTATGATAATCTCCC GATCACC |
| | | | SEQ ID NO:857 | SEQ ID NO:8869 | SEQ ID NO:16881 |
| | | AA | QANQDITNFLN | DASNLET | QQYDNLPIT |
| | | | SEQ ID NO:858 | SEQ ID NO:8870 | SEQ ID NO:16882 |
| **iPS:393914** | **21-225_16B8** | NA | CGGGCGAGTCAGGGCATTAA CAATTATTTAGCC | GCTGCATCCAGTGTGCA GAGT | CACCAGTATCACAGTTACCC ATTCACT |
| | | | SEQ ID NO:859 | SEQ ID NO:8871 | SEQ ID NO:16883 |
| | | AA | RASQGINNYLA | AASSVQS | HQYHSYPFT |
| | | | SEQ ID NO:860 | SEQ ID NO:8872 | SEQ ID NO:16884 |
| **iPS:393916** | **21-225_2G4** | NA | CGGGCAAGTCAGGGCATTAG AAATGATTTAGGC | GCTGCATCCAGTTTGCAC AGT | CTACAACATTATAGTTTCCCT CGGACG |
| | | | SEQ ID NO:861 | SEQ ID NO:8873 | SEQ ID NO:16885 |
| | | AA | RASQGIRNDLG | AASSLHS | LQHYSFPRT |

FIGURE 49

| | | | | | |
|---|---|---|---|---|---|
| | | | SEQ ID NO:862 | SEQ ID NO:8874 | SEQ ID NO:16886 |
| **iPS:393920** | **21-225_1H12** | NA | CGGGCAAGTCAGAACATTTACAGGTATTTAAAT | ACTGCATCCAGTTTACAAAGT | CAACAGAGTTACAGTCCCCCTCTCACT |
| | | | SEQ ID NO:863 | SEQ ID NO:8875 | SEQ ID NO:16887 |
| | | AA | RASQNIYRYLN | TASSLQS | QQSYSPPLT |
| | | | SEQ ID NO:864 | SEQ ID NO:8876 | SEQ ID NO:16888 |
| **iPS:393922** | **21-225_2B2** | NA | CGGGCAAGTCAGGGCATTAGAAATGATTTAGGC | GCTGCATCCAGTTTACAAAGT | CTACAGCATAATAGTTACCCGCTCACT |
| | | | SEQ ID NO:865 | SEQ ID NO:8877 | SEQ ID NO:16889 |
| | | AA | RASQGIRNDLG | AASSLQS | LQHNSYPLT |
| | | | SEQ ID NO:866 | SEQ ID NO:8878 | SEQ ID NO:16890 |
| **iPS:393926** | **21-225_4G4** | NA | CGGGCAAGTCAGACCATTATCAGCTATTTAAAT | ACTGCATCCAGTTTGCAAAGT | CAACAGACTTACAGTACTCCGCTCACT |
| | | | SEQ ID NO:867 | SEQ ID NO:8879 | SEQ ID NO:16891 |
| | | AA | RASQTIISYLN | TASSLQS | QQTYSTPLT |
| | | | SEQ ID NO:868 | SEQ ID NO:8880 | SEQ ID NO:16892 |
| **iPS:393928** | **21-225_4E10** | NA | CGGGCAAGTCAGGGCATTAGAAATGATTTAGGC | GCTGCATCCAGTTTGCAAAGT | TTACAGCATGATAATTACCCTCTCACT |
| | | | SEQ ID NO:869 | SEQ ID NO:8881 | SEQ ID NO:16893 |
| | | AA | RASQGIRNDLG | AASSLQS | LQHDNYPLT |
| | | | SEQ ID NO:870 | SEQ ID NO:8882 | SEQ ID NO:16894 |
| **iPS:393930** | **21-225_7E11** | NA | CGGGCAAGTCAAAACATTATCAGCTATTTAAAT | ACTGCATCCAGTTTGCAAAGT | CAACAGACTTACAGTACCCCGCTCACT |
| | | | SEQ ID NO:871 | SEQ ID NO:8883 | SEQ ID NO:16895 |
| | | AA | RASQNIISYLN | TASSLQS | QQTYSTPLT |
| | | | SEQ ID NO:872 | SEQ ID NO:8884 | SEQ ID NO:16896 |
| **iPS:393932** | **21-225_10F5** | NA | CGGGCAAGTCAGAACATTTACAGGTATTTAAAT | ACTGCATCCAGTTTGCAAAGT | CAACAGAGTTACAGTCCCCCTCTCACT |
| | | | SEQ ID NO:873 | SEQ ID NO:8885 | SEQ ID NO:16897 |
| | | AA | RASQNIYRYLN | TASSLQS | QQSYSPPLT |

FIGURE 49

| | | | | | |
|---|---|---|---|---|---|
| | | | SEQ ID NO:874 | SEQ ID NO:8886 | SEQ ID NO:16898 |
| **iPS:393934** | **21-225_13E6** | NA | CGGGCAAGTCAGGGCATTAG AAATGATTTAGGC | GCTGCATCCAGTTTGCAA AGT | GTACAGCATAATAGTTACCC GCTCACT |
| | | | SEQ ID NO:875 | SEQ ID NO:8887 | SEQ ID NO:16899 |
| | | AA | RASQGIRNDLG | AASSLQS | VQHNSYPLT |
| | | | SEQ ID NO:876 | SEQ ID NO:8888 | SEQ ID NO:16900 |
| **iPS:393936** | **21-225_14A11** | NA | CGGGCAAGTCAGAGCATTAG CAGTTATTTAAAT | GCTGCATCCAGTTTGCAA AAT | CAACAGACTTACAGTAGCCC TCCATTCACT |
| | | | SEQ ID NO:877 | SEQ ID NO:8889 | SEQ ID NO:16901 |
| | | AA | RASQSISSYLN | AASSLQN | QQTYSSPPFT |
| | | | SEQ ID NO:878 | SEQ ID NO:8890 | SEQ ID NO:16902 |
| **iPS:393940** | **21-225_16B2** | NA | CGGGCAAGTCAGAGCATTAG CGGCTATTTAAAT | GCTGCATCCAGTTTGCAA AGT | CAACAGACTTACAATACCCC TCCGGAGCGCAGT |
| | | | SEQ ID NO:879 | SEQ ID NO:8891 | SEQ ID NO:16903 |
| | | AA | RASQSISGYLN | AASSLQS | QQTYNTPPERS |
| | | | SEQ ID NO:880 | SEQ ID NO:8892 | SEQ ID NO:16904 |
| **iPS:393942** | **21-225_11E5** | NA | AAGTCCAGCCAGAGTGTTTT ATACAGCTCCAACAATAACA ACTACTTAACT | TGGGCATCTACCCGGGA ATCC | CAGCAATATTATAGTACTCC TCCGACG |
| | | | SEQ ID NO:881 | SEQ ID NO:8893 | SEQ ID NO:16905 |
| | | AA | KSSQSVLYSSNNNNYLT | WASTRES | QQYYSTPPT |
| | | | SEQ ID NO:882 | SEQ ID NO:8894 | SEQ ID NO:16906 |
| **iPS:393944** | **21-225_14D6** | NA | CGGGCAAGTCAGGACATTAG AAATCATTTAGGC | GCTGCATCCAGTTTGCAA AGT | CTACAGTATAATAGTTACCC ATTCACT |
| | | | SEQ ID NO:883 | SEQ ID NO:8895 | SEQ ID NO:16907 |
| | | AA | RASQDIRNHLG | AASSLQS | LQYNSYPFT |
| | | | SEQ ID NO:884 | SEQ ID NO:8896 | SEQ ID NO:16908 |
| **iPS:393946** | **21-225_16A4** | NA | CGGGCGAGTCAGGACATTAG TAATTATTTAGCC | GCTGCATCCAGTTTGCAA AGT | CAACAGTATCATAGTTACCC GTGGACG |
| | | | SEQ ID NO:885 | SEQ ID NO:8897 | SEQ ID NO:16909 |

FIGURE 49

| | | | | | |
|---|---|---|---|---|---|
| | | AA | RASQDISNYLA | AASSLQS | QQYHSYPWT |
| | | | SEQ ID NO:886 | SEQ ID NO:8898 | SEQ ID NO:16910 |
| **iPS:393948** | **21-225_16A5** | NA | CGGGCAAGTCAGGGCATTAG AAATGATTTAGGC | GCTGCATCCAGTTTGCAA AGT | CTACAGCATAATAGTTACCC GTGGACG |
| | | | SEQ ID NO:887 | SEQ ID NO:8899 | SEQ ID NO:16911 |
| | | AA | RASQGIRNDLG | AASSLQS | LQHNSYPWT |
| | | | SEQ ID NO:888 | SEQ ID NO:8900 | SEQ ID NO:16912 |
| **iPS:393950** | **21-225_3H10** | NA | TCTGGAGATAAATTGGGGGA AAAATATGCTTGC | CAAGATAGGAAGCGGCC CTCA | CAGGCGTGGGTCAACAACAC TATGATA |
| | | | SEQ ID NO:889 | SEQ ID NO:8901 | SEQ ID NO:16913 |
| | | AA | SGDKLGEKYAC | QDRKRPS | QAWVNNTMI |
| | | | SEQ ID NO:890 | SEQ ID NO:8902 | SEQ ID NO:16914 |
| **iPS:393952** | **21-225_1F1** | NA | CGGGCGAGTCAGGGCATTAA CAATTATTTAGCC | GTTGCATCCAGTTTGCAA ACT | CAACAGTATAATAGTTACCC TCTCACT |
| | | | SEQ ID NO:891 | SEQ ID NO:8903 | SEQ ID NO:16915 |
| | | AA | RASQGINNYLA | VASSLQT | QQYNSYPLT |
| | | | SEQ ID NO:892 | SEQ ID NO:8904 | SEQ ID NO:16916 |
| **iPS:393954** | **21-225_4H6** | NA | CGGGCGAGTCAGGGTATTAG CAGGTGGTTAGCC | GGTGCATCCAGTTTGCA AAGT | CAACAGGCTAACAGTTTCCC ATTCACT |
| | | | SEQ ID NO:893 | SEQ ID NO:8905 | SEQ ID NO:16917 |
| | | AA | RASQGISRWLA | GASSLQS | QQANSFPFT |
| | | | SEQ ID NO:894 | SEQ ID NO:8906 | SEQ ID NO:16918 |
| **iPS:393956** | **21-225_4D7** | NA | CGGGCAAGTCAGAGCATTAG CGACTATTTAAAT | GATACAACCAGTTTGCA AAGT | CAACAGACTTACAATACCCC TCCGGAGCGCAGT |
| | | | SEQ ID NO:895 | SEQ ID NO:8907 | SEQ ID NO:16919 |
| | | AA | RASQSISDYLN | DTTSLQS | QQTYNTPPERS |
| | | | SEQ ID NO:896 | SEQ ID NO:8908 | SEQ ID NO:16920 |
| **iPS:393958** | **21-225_5H2** | NA | CGGGCAAGTCAGGGCATTAG AAATGATTTAGGC | GCTGCATCCAGTTTGCAA AGT | CTACAGCATAATAGTTACCC TCTCACT |
| | | | SEQ ID NO:897 | SEQ ID NO:8909 | SEQ ID NO:16921 |

FIGURE 49

| | | | | | |
|---|---|---|---|---|---|
| | | AA | RASQGIRNDLG | AASSLQS | LQHNSYPLT |
| | | | SEQ ID NO:898 | SEQ ID NO:8910 | SEQ ID NO:16922 |
| **iPS:393960** | **21-225_7G2** | NA | CGGGCAAGTCAGGGCATTAG AAATGATTTAGGC | ACTGCATCCAGTTTGCAA AGT | CTACAACATAATAGTTACCC GTGGACG |
| | | | SEQ ID NO:899 | SEQ ID NO:8911 | SEQ ID NO:16923 |
| | | AA | RASQGIRNDLG | TASSLQS | LQHNSYPWT |
| | | | SEQ ID NO:900 | SEQ ID NO:8912 | SEQ ID NO:16924 |
| **iPS:393962** | **21-225_7H7** | NA | CGGGCAAGTCAGGGCATTAG AAATGATTTAGGC | GCTGCATCCAGTTTGCAA AGT | CTACAGCATAGTAGTTACCC GTTCACT |
| | | | SEQ ID NO:901 | SEQ ID NO:8913 | SEQ ID NO:16925 |
| | | AA | RASQGIRNDLG | AASSLQS | LQHSSYPFT |
| | | | SEQ ID NO:902 | SEQ ID NO:8914 | SEQ ID NO:16926 |
| **iPS:393964** | **21-225_6G1** | NA | CGGACAAGTCAGAACATTAT CAGCTATTTAAAT | ACTGCATCCAATTTGCAA ACT | CAACAGCCTCACAGTCCCCC GCTCACT |
| | | | SEQ ID NO:903 | SEQ ID NO:8915 | SEQ ID NO:16927 |
| | | AA | RTSQNIISYLN | TASNLQT | QQPHSPPLT |
| | | | SEQ ID NO:904 | SEQ ID NO:8916 | SEQ ID NO:16928 |
| **iPS:393966** | **21-225_7F8** | NA | CGGGCAAGTCAGGGCATTGG AAATGATTTAGGC | GCTGCATCCAGTTTGCAA AGT | CTACAACATTATACTTACCC TCGGACG |
| | | | SEQ ID NO:905 | SEQ ID NO:8917 | SEQ ID NO:16929 |
| | | AA | RASQGIGNDLG | AASSLQS | LQHYTYPRT |
| | | | SEQ ID NO:906 | SEQ ID NO:8918 | SEQ ID NO:16930 |
| **iPS:393968** | **21-225_5A5** | NA | CGGGCGAGTCAGGGCATTAG CAATTATTTAGCC | GCTGCATCCAGTTTGCAA AGT | CAACAGTATAATAGTTACCC ATTCACT |
| | | | SEQ ID NO:907 | SEQ ID NO:8919 | SEQ ID NO:16931 |
| | | AA | RASQGISNYLA | AASSLQS | QQYNSYPFT |
| | | | SEQ ID NO:908 | SEQ ID NO:8920 | SEQ ID NO:16932 |
| **iPS:393972** | **21-225_7C9** | NA | CGGGCAAGTCAGGGCATTAG AAACGATTTAGGC | GCTGCATCCAGTTTGCAA AGT | CTACAGCTTTATAGTTACCCT CGGACG |
| | | | SEQ ID NO:909 | SEQ ID NO:8921 | SEQ ID NO:16933 |

**FIGURE 49**

| | | | | | |
|---|---|---|---|---|---|
| | | AA | RASQGIRNDLG | AASSLQS | LQLYSYPRT |
| | | | SEQ ID NO:910 | SEQ ID NO:8922 | SEQ ID NO:16934 |
| **iPS:393974** | **21-225_7C4** | NA | CGGGCAAGTCAGGGCATTAG AAATGATTTAGGC | GCTGCATCCAGTTTGCAA AGT | CTACAGCATAATAGTTACCC GCTCACT |
| | | | SEQ ID NO:911 | SEQ ID NO:8923 | SEQ ID NO:16935 |
| | | AA | RASQGIRNDLG | AASSLQS | LQHNSYPLT |
| | | | SEQ ID NO:912 | SEQ ID NO:8924 | SEQ ID NO:16936 |
| **iPS:393976** | **21-225_7E9** | NA | CGGGCAAGTCAGGGCATTAG AAATGATTTAGGC | ACTGCATCCAGTTTGCAA AGT | CTACAGCATAATAGTTACCC GCTCACT |
| | | | SEQ ID NO:913 | SEQ ID NO:8925 | SEQ ID NO:16937 |
| | | AA | RASQGIRNDLG | TASSLQS | LQHNSYPLT |
| | | | SEQ ID NO:914 | SEQ ID NO:8926 | SEQ ID NO:16938 |
| **iPS:393978** | **21-225_4C12** | NA | CGGGCAAGTCAGGGCATTAG AAATGATTTAGGC | GCTGCATCCAGTTTGCAC AGT | CTACAACATTATAGTTTCCCT CGGACG |
| | | | SEQ ID NO:915 | SEQ ID NO:8927 | SEQ ID NO:16939 |
| | | AA | RASQGIRNDLG | AASSLHS | LQHYSFPRT |
| | | | SEQ ID NO:916 | SEQ ID NO:8928 | SEQ ID NO:16940 |
| **iPS:393980** | **21-225_6D3** | NA | CGGACAAGTCAGAGCATTAG TACTTATTTAAAT | GCTGCATCCAGTTTGCAA AGT | CAACAGAGTTACAGAACCCC CTTTTTCACT |
| | | | SEQ ID NO:917 | SEQ ID NO:8929 | SEQ ID NO:16941 |
| | | AA | RTSQSISTYLN | AASSLQS | QQSYRTPFFT |
| | | | SEQ ID NO:918 | SEQ ID NO:8930 | SEQ ID NO:16942 |
| **iPS:393982** | **21-225_6C12** | NA | CGGGCAAGTCAGGGCATTAG AAGTAATTTAGGC | GCTGCATCCAGTTTGGA AAGT | CTACAGGATAATAGTTATCC GTTCACT |
| | | | SEQ ID NO:919 | SEQ ID NO:8931 | SEQ ID NO:16943 |
| | | AA | RASQGIRSNLG | AASSLES | LQDNSYPFT |
| | | | SEQ ID NO:920 | SEQ ID NO:8932 | SEQ ID NO:16944 |
| **iPS:393984** | **21-225_4F12** | NA | CGGGCAAGTCAGGGCATTAG AAATGATTTAGGC | GCTGCATCCAGTTTGCAA AGT | CTACAGCATAATAGTTACGC GCTCACT |
| | | | SEQ ID NO:921 | SEQ ID NO:8933 | SEQ ID NO:16945 |

FIGURE 49

| | | | | | |
|---|---|---|---|---|---|
| | | AA | RASQGIRNDLG | AASSLQS | LQHNSYALT |
| | | | SEQ ID NO:922 | SEQ ID NO:8934 | SEQ ID NO:16946 |
| iPS:393986 | 21-225_7G4 | NA | CGGGCAAGTCAGGGCATTAGAAATGATTTAGGC | GCTGCATCCAGTTTGCAAAGT | CTACATCAATATAGTTACCCTCGGACG |
| | | | SEQ ID NO:923 | SEQ ID NO:8935 | SEQ ID NO:16947 |
| | | AA | RASQGIRNDLG | AASSLQS | LHQYSYPRT |
| | | | SEQ ID NO:924 | SEQ ID NO:8936 | SEQ ID NO:16948 |
| iPS:393988 | 21-225_7F10 | NA | CGGGCGAGTCAGGACATTAGGAATTATTTAGCC | GTTGCATCCAGTTTGCAAAGT | CAACAGTATAATAGTTACCCTCTCACT |
| | | | SEQ ID NO:925 | SEQ ID NO:8937 | SEQ ID NO:16949 |
| | | AA | RASQDIRNYLA | VASSLQS | QQYNSYPLT |
| | | | SEQ ID NO:926 | SEQ ID NO:8938 | SEQ ID NO:16950 |
| iPS:393990 | 21-225_11G7 | NA | CGGGCAAGTCAGGGCATTAGAAATGATTTAGGC | GCTGCATCCAGTTTGCAAAGT | CTACAGCATAGTAATTACCCTCTCACT |
| | | | SEQ ID NO:927 | SEQ ID NO:8939 | SEQ ID NO:16951 |
| | | AA | RASQGIRNDLG | AASSLQS | LQHSNYPLT |
| | | | SEQ ID NO:928 | SEQ ID NO:8940 | SEQ ID NO:16952 |
| iPS:393992 | 21-225_14H8 | NA | CGGGCGAGTCAGGGCATTAGCTATTATTTAGCC | GTTGCATCCAGTTTGCAAAGT | CAACAGTATAATAGTTACCCATTCACT |
| | | | SEQ ID NO:929 | SEQ ID NO:8941 | SEQ ID NO:16953 |
| | | AA | RASQGISYYLA | VASSLQS | QQYNSYPFT |
| | | | SEQ ID NO:930 | SEQ ID NO:8942 | SEQ ID NO:16954 |
| iPS:393994 | 21-225_8C9 | NA | CGGGCAAGTCAGGCCATTAGAAATGATTTAGAC | GCTGCATCCAGTTTGCAAAGT | CTACAGCATAATAGTTATCCGCTCACT |
| | | | SEQ ID NO:931 | SEQ ID NO:8943 | SEQ ID NO:16955 |
| | | AA | RASQAIRNDLD | AASSLQS | LQHNSYPLT |
| | | | SEQ ID NO:932 | SEQ ID NO:8944 | SEQ ID NO:16956 |
| iPS:393996 | 21-225_15C11 | NA | CGGGCAAGTCAGGGCATTAGAAATGATTTAGGC | GCTGCATCCAGTTTGCAAAGT | CTACTGCATTATAGTTACCCTCGGACG |
| | | | SEQ ID NO:933 | SEQ ID NO:8945 | SEQ ID NO:16957 |

## FIGURE 49

| | | | | | |
|---|---|---|---|---|---|
| | | AA | RASQGIRNDLG | AASSLQS | LLHYSYPRT |
| | | | SEQ ID NO:934 | SEQ ID NO:8946 | SEQ ID NO:16958 |
| **iPS:393998** | **21-225_12B12** | NA | CGGGCAAGTCAGGGCATTAG AAATGATTTAGGC | ACTGCATCCAGTTTGCAA AGT | CTACAACATAATAGTTACCC GTGGACG |
| | | | SEQ ID NO:935 | SEQ ID NO:8947 | SEQ ID NO:16959 |
| | | AA | RASQGIRNDLG | TASSLQS | LQHNSYPWT |
| | | | SEQ ID NO:936 | SEQ ID NO:8948 | SEQ ID NO:16960 |
| **iPS:394000** | **21-225_11A2** | NA | CAGGCGAGTCAGGACATTAG CAACTATTTAAAT | GATGCATCCAATTTGGA AACA | CAACAGTATGATAATCTCCC GATCACC |
| | | | SEQ ID NO:937 | SEQ ID NO:8949 | SEQ ID NO:16961 |
| | | AA | QASQDISNYLN | DASNLET | QQYDNLPIT |
| | | | SEQ ID NO:938 | SEQ ID NO:8950 | SEQ ID NO:16962 |
| **iPS:394002** | **21-225_15G7** | NA | CGGGCAAGTCAGGGCATTAG AAATGATTTAGGC | GCTGCATCCAGTTTGCAA AGT | CTACAGCATAGTAATTACCC TCTCACT |
| | | | SEQ ID NO:939 | SEQ ID NO:8951 | SEQ ID NO:16963 |
| | | AA | RASQGIRNDLG | AASSLQS | LQHSNYPLT |
| | | | SEQ ID NO:940 | SEQ ID NO:8952 | SEQ ID NO:16964 |
| **iPS:394004** | **21-225_13A1** | NA | CAGGCGAGTCAGGACATTAA CAACTATTTAAAT | GATGGATCCAATTTGGA AACA | CAACAGTATGAAAATCTCCC GATCACT |
| | | | SEQ ID NO:941 | SEQ ID NO:8953 | SEQ ID NO:16965 |
| | | AA | QASQDINNYLN | DGSNLET | QQYENLPIT |
| | | | SEQ ID NO:942 | SEQ ID NO:8954 | SEQ ID NO:16966 |
| **iPS:394006** | **21-225_15C2** | NA | CAGGCGAGTCAGGACATTAC CAACTATTTAAAT | GATGCATCCAATTTGGA AACA | CAACAGTATGATAATCTCCC GATCACC |
| | | | SEQ ID NO:943 | SEQ ID NO:8955 | SEQ ID NO:16967 |
| | | AA | QASQDITNYLN | DASNLET | QQYDNLPIT |
| | | | SEQ ID NO:944 | SEQ ID NO:8956 | SEQ ID NO:16968 |
| **iPS:394008** | **21-225_15H8** | NA | CGGGCAAGTCAGGGCATTAG AAATGATTTAGGC | GCTGCATCCAGTTTGCAA AGT | CTACAGCATAATAGTTACCC GCTCACT |
| | | | SEQ ID NO:945 | SEQ ID NO:8957 | SEQ ID NO:16969 |

FIGURE 49

| | | | | | |
|---|---|---|---|---|---|
| | | AA | RASQGIRNDLG | AASSLQS | LQHNSYPLT |
| | | | SEQ ID NO:946 | SEQ ID NO:8958 | SEQ ID NO:16970 |
| **iPS:394010** | **21-225_12G5** | NA | CGGGCGAGTCAGGACATTAG CAATTATTTAGCC | GCTGCATACATTTTGCAA TCA | CAAAAGTATGACAGTGCCCC ATTCACT |
| | | | SEQ ID NO:947 | SEQ ID NO:8959 | SEQ ID NO:16971 |
| | | AA | RASQDISNYLA | AAYILQS | QKYDSAPFT |
| | | | SEQ ID NO:948 | SEQ ID NO:8960 | SEQ ID NO:16972 |
| **iPS:394012** | **21-225_15A3** | NA | CGGGCAAGTCAGAGCATTAT CAGCTATTTAAAT | ACTGCATCCAGTTTGCAA AGT | CAACAGACTTACAGTACCCC GCTCACT |
| | | | SEQ ID NO:949 | SEQ ID NO:8961 | SEQ ID NO:16973 |
| | | AA | RASQSIISYLN | TASSLQS | QQTYSTPLT |
| | | | SEQ ID NO:950 | SEQ ID NO:8962 | SEQ ID NO:16974 |
| **iPS:394014** | **21-225_8G6** | NA | CGGGCAAGTCAGAGCATTAG TAGTTATTTAAAT | GCTGCATCCAGTTTGCAA AGT | CAACAGAGTTACAGAACCCC CTTTTTCACT |
| | | | SEQ ID NO:951 | SEQ ID NO:8963 | SEQ ID NO:16975 |
| | | AA | RASQSISSYLN | AASSLQS | QQSYRTPFFT |
| | | | SEQ ID NO:952 | SEQ ID NO:8964 | SEQ ID NO:16976 |
| **iPS:394016** | **21-225_13D4** | NA | CGGGCAAGTCAGAGCATTTT CAGCTACTTAAAT | ACTGCATCCAGTTTGCAA AAT | CAACAGACTTACAGTCTTCC GCTCACT |
| | | | SEQ ID NO:953 | SEQ ID NO:8965 | SEQ ID NO:16977 |
| | | AA | RASQSIFSYLN | TASSLQN | QQTYSLPLT |
| | | | SEQ ID NO:954 | SEQ ID NO:8966 | SEQ ID NO:16978 |
| **iPS:394018** | **21-225_15B1** | NA | CGGGCGAGTCAGGGCATTAG CAATTATTTAGCC | GCTGCATCCAGTTTGCAA AGT | CAACAGTATCATAGTTACCC ATTCACT |
| | | | SEQ ID NO:955 | SEQ ID NO:8967 | SEQ ID NO:16979 |
| | | AA | RASQGISNYLA | AASSLQS | QQYHSYPFT |
| | | | SEQ ID NO:956 | SEQ ID NO:8968 | SEQ ID NO:16980 |
| **iPS:394020** | **21-225_15H10** | NA | CGGGCAAGTCAGGGCATTAG AAATGATTTAGGC | GCTGCATCCAGTTTGCAA AGT | CTACAGCATAATAGTTACCC GCTCACT |
| | | | SEQ ID NO:957 | SEQ ID NO:8969 | SEQ ID NO:16981 |

FIGURE 49

| | | | | | |
|---|---|---|---|---|---|
| | | AA | RASQGIRNDLG | AASSLQS | LQHNSYPLT |
| | | | SEQ ID NO:958 | SEQ ID NO:8970 | SEQ ID NO:16982 |
| **iPS:394022** | **21-225_16H6** | NA | CGGGCAAGTCAGAACATTAGCAGCTATTTAAAT | GCTGCATCCAGTTTGCAAAGT | CAACAGAGTTACAGAACCCCCTTATTCACT |
| | | | SEQ ID NO:959 | SEQ ID NO:8971 | SEQ ID NO:16983 |
| | | AA | RASQNISSYLN | AASSLQS | QQSYRTPLFT |
| | | | SEQ ID NO:960 | SEQ ID NO:8972 | SEQ ID NO:16984 |
| **iPS:394024** | **21-225_16B7** | NA | CGGGCAAGTCAGGGCATTAGAAATGATTTAGGC | GCTGCATCCAGTTTGCAAAGT | CTACAGCATAATAGTTACCCGCTCACT |
| | | | SEQ ID NO:961 | SEQ ID NO:8973 | SEQ ID NO:16985 |
| | | AA | RASQGIRNDLG | AASSLQS | LQHNSYPLT |
| | | | SEQ ID NO:962 | SEQ ID NO:8974 | SEQ ID NO:16986 |
| **iPS:394026** | **21-225_16C7** | NA | CGGGCGAGTCAGGGCATTAGCAATTATTTAGCC | GCTGCATCCAGTTTGCAAAGT | CAACAGTATAATAGTTACCCATTCACT |
| | | | SEQ ID NO:963 | SEQ ID NO:8975 | SEQ ID NO:16987 |
| | | AA | RASQGISNYLA | AASSLQS | QQYNSYPFT |
| | | | SEQ ID NO:964 | SEQ ID NO:8976 | SEQ ID NO:16988 |
| **iPS:394029** | **21-225_1B12** | NA | CAGGCGAGTCAGGACATTAACAACTATTTAAAT | GATGCATCCAATTTGGAAACA | CAACAGTATGAAAATCTCCCGATCACC |
| | | | SEQ ID NO:965 | SEQ ID NO:8977 | SEQ ID NO:16989 |
| | | AA | QASQDINNYLN | DASNLET | QQYENLPIT |
| | | | SEQ ID NO:966 | SEQ ID NO:8978 | SEQ ID NO:16990 |
| **iPS:394033** | **21-225_5F4** | NA | CGGGCAAGTCAGGGCATTCGAAATCATTTAGGC | GCTGCCTCCAGTTTGCAAAGT | CTACAGTATAATGGTTACCCATTCACT |
| | | | SEQ ID NO:967 | SEQ ID NO:8979 | SEQ ID NO:16991 |
| | | AA | RASQGIRNHLG | AASSLQS | LQYNGYPFT |
| | | | SEQ ID NO:968 | SEQ ID NO:8980 | SEQ ID NO:16992 |
| **iPS:394035** | **21-225_5G9** | NA | CAGGCGAGTCAGGGCATTAGCAACTCTTTAAAT | GATGCATCCAATTTGGAAACA | CAACAATATGATAATCTCCCGCTCACT |
| | | | SEQ ID NO:969 | SEQ ID NO:8981 | SEQ ID NO:16993 |

FIGURE 49

| | | | | | |
|---|---|---|---|---|---|
| | | AA | QASQGISNSLN | DASNLET | QQYDNLPLT |
| | | | SEQ ID NO:970 | SEQ ID NO:8982 | SEQ ID NO:16994 |
| **iPS:394037** | **21-225_4F4** | NA | CGGGCAAGTCAGGGCATTAG AAATGATTTAGGC | GCTGCGTCCAGTGTGCA AACT | CTACAGCATAATAGTTACCC GCTCACT |
| | | | SEQ ID NO:971 | SEQ ID NO:8983 | SEQ ID NO:16995 |
| | | AA | RASQGIRNDLG | AASSVQT | LQHNSYPLT |
| | | | SEQ ID NO:972 | SEQ ID NO:8984 | SEQ ID NO:16996 |
| **iPS:394041** | **21-225_5E5** | NA | CGGGCAAGTCAGGGCATTAG AAATGATTTAGGC | GCTGCATCCAGTTTGCAA AGT | CTACAGCATTATAGTTACCC TCGGACG |
| | | | SEQ ID NO:973 | SEQ ID NO:8985 | SEQ ID NO:16997 |
| | | AA | RASQGIRNDLG | AASSLQS | LQHYSYPRT |
| | | | SEQ ID NO:974 | SEQ ID NO:8986 | SEQ ID NO:16998 |
| **iPS:394043** | **21-225_3B1** | NA | CGGGCAAGTCAGAGTATTAA TAATTATTTAAAT | GCTACATCCAGTTTGCAA AAT | CAACAGAGTTACAGTACCCC CTTATTCACT |
| | | | SEQ ID NO:975 | SEQ ID NO:8987 | SEQ ID NO:16999 |
| | | AA | RASQSINNYLN | ATSSLQN | QQSYSTPLFT |
| | | | SEQ ID NO:976 | SEQ ID NO:8988 | SEQ ID NO:17000 |
| **iPS:394045** | **21-225_4H4** | NA | CGGGCAAGTCAGGGCATTAG AAATGATTTAGGC | GCTGCATCCAGTGTGCA AAGT | CTACAGCATAATAGTTACCC GCTCACT |
| | | | SEQ ID NO:977 | SEQ ID NO:8989 | SEQ ID NO:17001 |
| | | AA | RASQGIRNDLG | AASSVQS | LQHNSYPLT |
| | | | SEQ ID NO:978 | SEQ ID NO:8990 | SEQ ID NO:17002 |
| **iPS:394047** | **21-225_5E6** | NA | CAGGCGAGTCAGGACATTAA CAACTATTTAAAT | GATGCATCCAATTTGGA AACA | CAACAGTATGATAATCTCCC GATCACC |
| | | | SEQ ID NO:979 | SEQ ID NO:8991 | SEQ ID NO:17003 |
| | | AA | QASQDINNYLN | DASNLET | QQYDNLPIT |
| | | | SEQ ID NO:980 | SEQ ID NO:8992 | SEQ ID NO:17004 |
| **iPS:394049** | **21-225_13H5** | NA | CGGGCAAGTCAGGGCATTAG AAATGATTTAGGC | GCTGCATCCAGTTTGCAA ACT | CTACAGCATAATAGTTACCC TCTCACT |
| | | | SEQ ID NO:981 | SEQ ID NO:8993 | SEQ ID NO:17005 |

**FIGURE 49**

| | | | | | |
|---|---|---|---|---|---|
| | | AA | RASQGIRNDLG | AASSLQT | LQHNSYPLT |
| | | | SEQ ID NO:982 | SEQ ID NO:8994 | SEQ ID NO:17006 |
| **iPS:394051** | **21-225_9E5** | NA | CGGGCAAGTCAGAGCATTGC CAGTTATTTAAAT | GGTGCATCCAGTTTGCA AAGT | CAACAGAGTTACAGTACCCC CTTATTCAGT |
| | | | SEQ ID NO:983 | SEQ ID NO:8995 | SEQ ID NO:17007 |
| | | AA | RASQSIASYLN | GASSLQS | QQSYSTPLFS |
| | | | SEQ ID NO:984 | SEQ ID NO:8996 | SEQ ID NO:17008 |
| **iPS:394053** | **21-225_11F10** | NA | CGGGCAAGTCAGGGCATTAG AAATGATTTAGGC | GCTGCATCCAGTTTACAA AGT | CTACAGCATAGTAGTTACCC TCTCACT |
| | | | SEQ ID NO:985 | SEQ ID NO:8997 | SEQ ID NO:17009 |
| | | AA | RASQGIRNDLG | AASSLQS | LQHSSYPLT |
| | | | SEQ ID NO:986 | SEQ ID NO:8998 | SEQ ID NO:17010 |
| **iPS:394055** | **21-225_9C8** | NA | CGGGCGAGTCAGGGCATTAG CTATTATTTAGCC | GTTGCATCCAGTTTGCAA AGT | CAACAGTATGATAGTTACCC ATTCACT |
| | | | SEQ ID NO:987 | SEQ ID NO:8999 | SEQ ID NO:17011 |
| | | AA | RASQGISYYLA | VASSLQS | QQYDSYPFT |
| | | | SEQ ID NO:988 | SEQ ID NO:9000 | SEQ ID NO:17012 |
| **iPS:394057** | **21-225_15H1** | NA | CGGGCAAGTCAGGGCATTAG AAATGATTTAGGC | ACTGCATCCAGTTTGCAA AGT | CTACAGCATAGTAGTTACCC GCTCACT |
| | | | SEQ ID NO:989 | SEQ ID NO:9001 | SEQ ID NO:17013 |
| | | AA | RASQGIRNDLG | TASSLQS | LQHSSYPLT |
| | | | SEQ ID NO:990 | SEQ ID NO:9002 | SEQ ID NO:17014 |
| **iPS:394059** | **21-225_9E8** | NA | CGGGCAAGTCAGGGCATTAG AAATGATTTAGGC | GCTGCATCCAGTTTGCAA AGT | CTACAGCATAATAGTTACCC GCTCACT |
| | | | SEQ ID NO:991 | SEQ ID NO:9003 | SEQ ID NO:17015 |
| | | AA | RASQGIRNDLG | AASSLQS | LQHNSYPLT |
| | | | SEQ ID NO:992 | SEQ ID NO:9004 | SEQ ID NO:17016 |
| **iPS:394061** | **21 225 12D2** | NA | AGGTCTAGTCAGAGCCTCCT CCATAGTAATGGATACAACT ATTTGGAT | TTGGGTTCTAATCGGGCC TCC | ATGCAAGCTCTACAAACTCC TATCACC |

# FIGURE 49

| | | | | | |
|---|---|---|---|---|---|
| | 21-225_12D2 | | SEQ ID NO:993 | SEQ ID NO:9005 | SEQ ID NO:17017 |
| | | AA | RSSQSLLHSNGYNYLD | LGSNRAS | MQALQTPIT |
| | | | SEQ ID NO:994 | SEQ ID NO:9006 | SEQ ID NO:17018 |
| **iPS:394063** | **21-225_16A1** | NA | CGGGCAAGTCAGGGCATTAG AAATGATTTAGGC | GCTGCGTCCAGTTTGCAA AGT | CTACACCATAGTAATTACCC TCTCACT |
| | | | SEQ ID NO:995 | SEQ ID NO:9007 | SEQ ID NO:17019 |
| | | AA | RASQGIRNDLG | AASSLQS | LHHSNYPLT |
| | | | SEQ ID NO:996 | SEQ ID NO:9008 | SEQ ID NO:17020 |
| **iPS:394065** | **21-225_11E2** | NA | AAGTCCAACCAGAGAGTTTT ATCCAGCTCCAACAATCACA ACTACTTAGCT | TGGGCATCTACCCGGGA ATCC | CAGCAATATTTAGTACTCC ATTCACT |
| | | | SEQ ID NO:997 | SEQ ID NO:9009 | SEQ ID NO:17021 |
| | | AA | KSNQRVLSSSNNHNYLA | WASTRES | QQYFSTPFT |
| | | | SEQ ID NO:998 | SEQ ID NO:9010 | SEQ ID NO:17022 |
| **iPS:394067** | **21-225_12F2** | NA | CGGGCAAGTCAGGGCATTAG AAATGATTTAGGC | GCTGCATCCAGTTTGCAA AGT | CTACAGCATAATAGTTATCC GTGGACG |
| | | | SEQ ID NO:999 | SEQ ID NO:9011 | SEQ ID NO:17023 |
| | | AA | RASQGIRNDLG | AASSLQS | LQHNSYPWT |
| | | | SEQ ID NO:1000 | SEQ ID NO:9012 | SEQ ID NO:17024 |
| **iPS:394069** | **21-225_16H1** | NA | CGGGCAAGTCAGGGCATTAG AGATATTTTAGGC | GCTGCATCCAGTTTGCAA AAT | CTACAGTATCATAGTTACCC ATTCACT |
| | | | SEQ ID NO:1001 | SEQ ID NO:9013 | SEQ ID NO:17025 |
| | | AA | RASQGIRDILG | AASSLQN | LQYHSYPFT |
| | | | SEQ ID NO:1002 | SEQ ID NO:9014 | SEQ ID NO:17026 |
| **iPS:394071** | **21-225_10C7** | NA | AGGTCTAGTCAGAGCCTCCT GCATAGTAAGGGATACAACT ATTTGGAT | TTGGGTTCTAATCGGGCC TCC | ATGCAAGCTCTACAAACTCC TCTCACC |
| | | | SEQ ID NO:1003 | SEQ ID NO:9015 | SEQ ID NO:17027 |
| | | AA | RSSQSLLHSKGYNYLD | LGSNRAS | MQALQTPLT |
| | | | SEQ ID NO:1004 | SEQ ID NO:9016 | SEQ ID NO:17028 |

FIGURE 49

| | | | | | |
|---|---|---|---|---|---|
| **iPS:394073** | **21-225_15C9** | NA | CGGGCAAGTCAGGGCATTAG AAATGATTTAGGC | GCTGCATCCAGTTTGCAA AGT | CTACAACATACTAGTTACCC GCTCACT |
| | | | SEQ ID NO:1005 | SEQ ID NO:9017 | SEQ ID NO:17029 |
| | | AA | RASQGIRNDLG | AASSLQS | LQHTSYPLT |
| | | | SEQ ID NO:1006 | SEQ ID NO:9018 | SEQ ID NO:17030 |
| **iPS:394075** | **21-225_8D12** | NA | CGGGCAAGTCAGGGCATTAG AAATGATTTAGGC | GCTGCATCCAGTTTGCAA AGT | CTACAGCATAGTAGTTACCC GCTCACT |
| | | | SEQ ID NO:1007 | SEQ ID NO:9019 | SEQ ID NO:17031 |
| | | AA | RASQGIRNDLG | AASSLQS | LQHSSYPLT |
| | | | SEQ ID NO:1008 | SEQ ID NO:9020 | SEQ ID NO:17032 |
| **iPS:394077** | **21-225_8E12** | NA | CGGGCAAGTCAGAGCATTAG TAATTATTTAAAT | GCTGCATCCAGTTTACAA AGT | CAACAGAGTTACAGAACCCC CTTTTTCACT |
| | | | SEQ ID NO:1009 | SEQ ID NO:9021 | SEQ ID NO:17033 |
| | | AA | RASQSISNYLN | AASSLQS | QQSYRTPFFT |
| | | | SEQ ID NO:1010 | SEQ ID NO:9022 | SEQ ID NO:17034 |
| **iPS:394079** | **21-225_11F5** | NA | CGGGCAAGTCAGGGCATTAG AAATGATTTAGGC | GCTGCATCCAGTGTGCA AAGT | CTACAGCATAATAGTTACCC GCTCACT |
| | | | SEQ ID NO:1011 | SEQ ID NO:9023 | SEQ ID NO:17035 |
| | | AA | RASQGIRNDLG | AASSVQS | LQHNSYPLT |
| | | | SEQ ID NO:1012 | SEQ ID NO:9024 | SEQ ID NO:17036 |
| **iPS:394081** | **21-225_16B3** | NA | CAGGCGAGTCAGGACATTAA CAACTATTTAAAT | GATGCATCCAATTTGGA AACA | CAACAGTTTGATAATCTCCC GATCACC |
| | | | SEQ ID NO:1013 | SEQ ID NO:9025 | SEQ ID NO:17037 |
| | | AA | QASQDINNYLN | DASNLET | QQFDNLPIT |
| | | | SEQ ID NO:1014 | SEQ ID NO:9026 | SEQ ID NO:17038 |
| **iPS:394083** | **21-225_16E6** | NA | CGGGCAAGTCAGAGCATTAT CAGCTATTTAAAT | ACTACATCCAGTTTGCAA AGT | CAGCAGACTTACAGTACCCC GCTCACT |
| | | | SEQ ID NO:1015 | SEQ ID NO:9027 | SEQ ID NO:17039 |
| | | AA | RASQSIISYLN | TTSSLQS | QQTYSTPLT |
| | | | SEQ ID NO:1016 | SEQ ID NO:9028 | SEQ ID NO:17040 |

FIGURE 49

| | | | | | |
|---|---|---|---|---|---|
| **iPS:394085** | **21-225_8B11** | NA | AAGTCCAGCCAGAATGTTTTATACAACTCCAACAATAACAACTACTTAGCT | TGGGCATCTACCCGGAAATCC | CAGCAATATTATACTACTCCGTGCAGT |
| | | | SEQ ID NO:1017 | SEQ ID NO:9029 | SEQ ID NO:17041 |
| | | AA | KSSQNVLYNSNNNNYLA | WASTRKS | QQYYTTPCS |
| | | | SEQ ID NO:1018 | SEQ ID NO:9030 | SEQ ID NO:17042 |
| **iPS:394087** | **21-225_11A5** | NA | CGGGCAAGTCAGAACATTTATAGTTATTTAAAT | GCTGCATCCAGTTTGCAAAGT | CAACAGAGTTACAATACCCCCTTATTCACT |
| | | | SEQ ID NO:1019 | SEQ ID NO:9031 | SEQ ID NO:17043 |
| | | AA | RASQNIYSYLN | AASSLQS | QQSYNTPLFT |
| | | | SEQ ID NO:1020 | SEQ ID NO:9032 | SEQ ID NO:17044 |
| **iPS:394089** | **21-225_12E6** | NA | CGGGCAAGTCAGGGCATTAGAAGTGATTTAGGC | GCTGCATCCAGTTTGCAAAGT | CTACAGCATAATAGTTACCCGTGGACG |
| | | | SEQ ID NO:1021 | SEQ ID NO:9033 | SEQ ID NO:17045 |
| | | AA | RASQGIRSDLG | AASSLQS | LQHNSYPWT |
| | | | SEQ ID NO:1022 | SEQ ID NO:9034 | SEQ ID NO:17046 |
| **iPS:394091** | **21-225_13H3** | NA | CGGGCAAGTCAGGGCATTAGAAATGATTTAGGC | GCTGCATCCAGTTTACAAAGT | CTACAGCATAATAGTTACCCGCTCACT |
| | | | SEQ ID NO:1023 | SEQ ID NO:9035 | SEQ ID NO:17047 |
| | | AA | RASQGIRNDLG | AASSLQS | LQHNSYPLT |
| | | | SEQ ID NO:1024 | SEQ ID NO:9036 | SEQ ID NO:17048 |
| **iPS:394093** | **21-225_9D12** | NA | CGGGCAAGTCAGGGCATTAGAAATGATTTAGGC | GCTGCATCCAGTTTGCAAAGT | CTACAGCATAATAGTTACCCGTGGACG |
| | | | SEQ ID NO:1025 | SEQ ID NO:9037 | SEQ ID NO:17049 |
| | | AA | RASQGIRNDLG | AASSLQS | LQHNSYPWT |
| | | | SEQ ID NO:1026 | SEQ ID NO:9038 | SEQ ID NO:17050 |
| **iPS:394095** | **21-225_16H4** | NA | CGGGCAAGTCAGGGCATTAGAAATGATTTAGGC | ACTGCATCCAGTTTGCAAAGT | CTACAGCATAATAGTTACCCGTGGACG |
| | | | SEQ ID NO:1027 | SEQ ID NO:9039 | SEQ ID NO:17051 |
| | | AA | RASQGIRNDLG | TASSLQS | LQHNSYPWT |

FIGURE 49

| | | | | | |
|---|---|---|---|---|---|
| | | | SEQ ID NO:1028 | SEQ ID NO:9040 | SEQ ID NO:17052 |
| **iPS:394097** | **21-225_16G7** | NA | CGGGCAAGTCAGGGCATTAG AAATGATTTAGGC | GCTGCATCCAGTTTGCAA AGT | CTACAACATAATAGTTACCC GTGGACG |
| | | | SEQ ID NO:1029 | SEQ ID NO:9041 | SEQ ID NO:17053 |
| | | AA | RASQGIRNDLG | AASSLQS | LQHNSYPWT |
| | | | SEQ ID NO:1030 | SEQ ID NO:9042 | SEQ ID NO:17054 |
| **iPS:398470** | **21-225_14B7** | NA | TCTGGAGATAAATTGGGGAA TAAATATGCTTAC | CAAGATAGAAAGCGGCC CTCA | CAGGCGTGGAACAACAGCAC TGTGGTA |
| | | | SEQ ID NO:1031 | SEQ ID NO:9043 | SEQ ID NO:17055 |
| | | AA | SGDKLGNKYAY | QDRKRPS | QAWNNSTVV |
| | | | SEQ ID NO:1032 | SEQ ID NO:9044 | SEQ ID NO:17056 |
| **iPS:398472** | **21-225_16E4** | NA | TCTGGAGATAAATTGGGGGA TAAATATGTTTAC | CAAGATAGCAAGCGGCC CTCA | CAGGCGTGGGACAGCAGCAC TGTGGTT |
| | | | SEQ ID NO:1033 | SEQ ID NO:9045 | SEQ ID NO:17057 |
| | | AA | SGDKLGDKYVY | QDSKRPS | QAWDSSTVV |
| | | | SEQ ID NO:1034 | SEQ ID NO:9046 | SEQ ID NO:17058 |
| **iPS:398474** | **21-225_17B10** | NA | CGGTCAAGTCAGAGCATTAA CAGCTATTTAAAT | GCTGCATCCAGTTTGCAC AGT | CAACAGGGTTACAATACCCC CACGTGGACG |
| | | | SEQ ID NO:1035 | SEQ ID NO:9047 | SEQ ID NO:17059 |
| | | AA | RSSQSINSYLN | AASSLHS | QQGYNTPTWT |
| | | | SEQ ID NO:1036 | SEQ ID NO:9048 | SEQ ID NO:17060 |
| **iPS:398476** | **21-225_17C1** | NA | CGGGCAAGTCAGAACATTAA CGACTATTTAAAT | GCTGCATCCAATTTGCAA AGT | CAACAGACTTACAATACCCC TCCGGAGCGCAGT |
| | | | SEQ ID NO:1037 | SEQ ID NO:9049 | SEQ ID NO:17061 |
| | | AA | RASQNINDYLN | AASNLQS | QQTYNTPPERS |
| | | | SEQ ID NO:1038 | SEQ ID NO:9050 | SEQ ID NO:17062 |
| **iPS:398478** | **21-225_17C10** | NA | CGGGCGAGTCAGGGCATTAG CAATTATTTAGCC | GCTGCATCCACTTTGCAA TCA | CAAAAGTATAACAGTGCCCC TCCGCTCACC |
| | | | SEQ ID NO:1039 | SEQ ID NO:9051 | SEQ ID NO:17063 |
| | | AA | RASQGISNYLA | AASTLQS | QKYNSAPPLT |

**FIGURE 49**

| | | | | | |
|---|---|---|---|---|---|
| | | | SEQ ID NO:1040 | SEQ ID NO:9052 | SEQ ID NO:17064 |
| **iPS:398480** | **21-225_17G4** | NA | CGGACAAGTCAGAATATTAG CAACTATTTAAAT | GTTGCGTCCAGTTTCCCA AGT | CAACAGAGTAACTTTTTCCC GCTCACT |
| | | | SEQ ID NO:1041 | SEQ ID NO:9053 | SEQ ID NO:17065 |
| | | AA | RTSQNISNYLN | VASSFPS | QQSNFFPLT |
| | | | SEQ ID NO:1042 | SEQ ID NO:9054 | SEQ ID NO:17066 |
| **iPS:398482** | **21-225_17H6** | NA | CGGGCGAGTCGGGACATTAG CAATTATTTAGCC | ACTGCATCCAGTTTGCAA AGT | CAACAGTATCATAGTTACCC ATTCACT |
| | | | SEQ ID NO:1043 | SEQ ID NO:9055 | SEQ ID NO:17067 |
| | | AA | RASRDISNYLA | TASSLQS | QQYHSYPFT |
| | | | SEQ ID NO:1044 | SEQ ID NO:9056 | SEQ ID NO:17068 |
| **iPS:398484** | **21-225_18D4** | NA | CGGGCAAGTCAGGGCATTAG AAATGATTTAGGC | GCTGCATCCAGTTTGGA AAGT | CTACATCATAATAATTACCT CCCCATCACC |
| | | | SEQ ID NO:1045 | SEQ ID NO:9057 | SEQ ID NO:17069 |
| | | AA | RASQGIRNDLG | AASSLES | LHHNNYLPIT |
| | | | SEQ ID NO:1046 | SEQ ID NO:9058 | SEQ ID NO:17070 |
| **iPS:398486** | **21-225_19A1** | NA | CGGGCAAGTCATACCATTAC CAGCTATTTAAAT | GCTACATCCAATCTCCAA AGT | CAACAGAGTTACAACTTCCC GCTCACT |
| | | | SEQ ID NO:1047 | SEQ ID NO:9059 | SEQ ID NO:17071 |
| | | AA | RASHTITSYLN | ATSNLQS | QQSYNFPLT |
| | | | SEQ ID NO:1048 | SEQ ID NO:9060 | SEQ ID NO:17072 |
| **iPS:398488** | **21-225_19F6** | NA | TCTGGAGATAAATTGGGGGA TAAATATGCTTGC | CAAGATAGCAAGCGGCC CTCA | CAGGCGTGGGACAACAACAC TGTGGTA |
| | | | SEQ ID NO:1049 | SEQ ID NO:9061 | SEQ ID NO:17073 |
| | | AA | SGDKLGDKYAC | QDSKRPS | QAWDNNTVV |
| | | | SEQ ID NO:1050 | SEQ ID NO:9062 | SEQ ID NO:17074 |
| **iPS:398490** | **21-225_21D12** | NA | TCTGGAGATAAATTGGGGAA TAAATATGCTTAC | CAAGATAGAAAGAGGCC CTCA | CAGGCGTGGGACAACAGCAC TGTGGTA |
| | | | SEQ ID NO:1051 | SEQ ID NO:9063 | SEQ ID NO:17075 |
| | | AA | SGDKLGNKYAY | QDRKRPS | QAWDNSTVV |

**FIGURE 49**

| | | | | | |
|---|---|---|---|---|---|
| | | | SEQ ID NO:1052 | SEQ ID NO:9064 | SEQ ID NO:17076 |
| **iPS:398492** | **21-225_21F12** | NA | CGGGCGAGTCAGGGCATTAGGAATTTTTTAGCC | GCTGCATCCAGTTTGCAAACT | CAACAGTATAATAGTTTCCCATTCACT |
| | | | SEQ ID NO:1053 | SEQ ID NO:9065 | SEQ ID NO:17077 |
| | | AA | RASQGIRNFLA | AASSLQT | QQYNSFPFT |
| | | | SEQ ID NO:1054 | SEQ ID NO:9066 | SEQ ID NO:17078 |
| **iPS:398494** | **21-225_21H4** | NA | ACTGGAACCAGCAGTGACGTTGGTGGTTATAACTCTGTCTCC | GAGGTCAGTAATCGGCCCTCA | AGCTCATATACAAGGAGCAGCACTGTGGTA |
| | | | SEQ ID NO:1055 | SEQ ID NO:9067 | SEQ ID NO:17079 |
| | | AA | TGTSSDVGGYNSVS | EVSNRPS | SSYTRSSTVV |
| | | | SEQ ID NO:1056 | SEQ ID NO:9068 | SEQ ID NO:17080 |
| **iPS:398496** | **21-225_22D2** | NA | AAGTCCAGCCAGAGTGTTTTACACAGCTCCAACAATAACAACTACTTAGCT | TGGGCATCTACCCGGAAATCC | CAGCAATATTATAGTACTCCGTGCAGT |
| | | | SEQ ID NO:1057 | SEQ ID NO:9069 | SEQ ID NO:17081 |
| | | AA | KSSQSVLHSSNNNNYLA | WASTRKS | QQYYSTPCS |
| | | | SEQ ID NO:1058 | SEQ ID NO:9070 | SEQ ID NO:17082 |
| **iPS:398498** | **21-225_22E6** | NA | TCTGGAGATAAATTGGGGGAGAAATATGCTTGC | CAAGATAGAAAGCGGCCCTCA | CAGGCGTGGGACAGCAGCACTGCGGTA |
| | | | SEQ ID NO:1059 | SEQ ID NO:9071 | SEQ ID NO:17083 |
| | | AA | SGDKLGEKYAC | QDRKRPS | QAWDSSTAV |
| | | | SEQ ID NO:1060 | SEQ ID NO:9072 | SEQ ID NO:17084 |
| **iPS:398500** | **21-225_23A11** | NA | CGGGCGAGTCAGGACATTAGCAATTATTTAGCC | GCTGCATCCACTTTGCAAAGT | CAACAGTATAATAGTTACCCATTCACT |
| | | | SEQ ID NO:1061 | SEQ ID NO:9073 | SEQ ID NO:17085 |
| | | AA | RASQDISNYLA | AASTLQS | QQYNSYPFT |
| | | | SEQ ID NO:1062 | SEQ ID NO:9074 | SEQ ID NO:17086 |
| **iPS:398502** | | NA | CGGGCGAGTCAGGGTATTACCAAGTGGTTAGCC | GCTGCATCCAGTTTGCAAAGT | CAACAGGCTAACAGTTTCCCATTCACT |

FIGURE 49

| | | | | | |
|---|---|---|---|---|---|
| | **21-225_23B11** | | SEQ ID NO:1063 | SEQ ID NO:9075 | SEQ ID NO:17087 |
| | | AA | RASQGITKWLA | AASSLQS | QQANSFPFT |
| | | | SEQ ID NO:1064 | SEQ ID NO:9076 | SEQ ID NO:17088 |
| **iPS:398504** | **21-225_23D7** | NA | TCTGGAGAAAAATTGGGGG ATAAATATGTTTGT | CAAGATAGCAAGCGGCC CTCA | CAGGCGTGGAACAGCAGCAA TGTGGTA |
| | | | SEQ ID NO:1065 | SEQ ID NO:9077 | SEQ ID NO:17089 |
| | | AA | SGEKLGDKYVC | QDSKRPS | QAWNSSNVV |
| | | | SEQ ID NO:1066 | SEQ ID NO:9078 | SEQ ID NO:17090 |
| **iPS:398506** | **21-225_23G12** | NA | AAGTCCAGCCAGAGTATTTT ATTCAGCTCCAACAATAACA ACTACTTAGCT | TGGGCATCTACCCGGGA ATCC | CAGCAATATTCTAGTACTCC GTGGACG |
| | | | SEQ ID NO:1067 | SEQ ID NO:9079 | SEQ ID NO:17091 |
| | | AA | KSSQSILFSSNNNNYLA | WASTRES | QQYSSTPWT |
| | | | SEQ ID NO:1068 | SEQ ID NO:9080 | SEQ ID NO:17092 |
| **iPS:398508** | **21-225_24B1** | NA | AGGTCTAGTCAAAGCCTCGT ATACAGTGATGGAAACACCT ACTTGAAT | AAGGTTTCTAACTGGGA CTCT | ATGCAAGGTGCACACTGGCC TCCGATCACC |
| | | | SEQ ID NO:1069 | SEQ ID NO:9081 | SEQ ID NO:17093 |
| | | AA | RSSQSLVYSDGNTYLN | KVSNWDS | MQGAHWPPIT |
| | | | SEQ ID NO:1070 | SEQ ID NO:9082 | SEQ ID NO:17094 |
| **iPS:398510** | **21-225_25A3** | NA | AAGTCCAGCCAGAGTGTTTT ATACAGCTCCAACAATAAGA ACTACTTAGCT | TGGGCATCTACCCGGGA ATCC | CAGCAATATTATAGTACTCC GTGCAGT |
| | | | SEQ ID NO:1071 | SEQ ID NO:9083 | SEQ ID NO:17095 |
| | | AA | KSSQSVLYSSNNKNYLA | WASTRES | QQYYSTPCS |
| | | | SEQ ID NO:1072 | SEQ ID NO:9084 | SEQ ID NO:17096 |
| **iPS:398512** | **21-225_25E12** | NA | AAGTCCAGCCAGAGTGTTTT ATACCACTCCAACAATTACA ACTACTTAGCT | TGGGCATCTACCCGGGA GTCC | CAGCAATATTACAGTACTCC GTGCAGT |
| | | | SEQ ID NO:1073 | SEQ ID NO:9085 | SEQ ID NO:17097 |

FIGURE 49

| | | | | | |
|---|---|---|---|---|---|
| | | AA | KSSQSVLYHSNNYNYLA | WASTRES | QQYYSTPCS |
| | | | SEQ ID NO:1074 | SEQ ID NO:9086 | SEQ ID NO:17098 |
| **iPS:398516** | **21-225_26A9** | NA | AAGTCCAGCCAGAGTGTTTTATACAGCTCCAACAATAAGAACTACTTAGCT | TGGGCATCTACCCGGGAATCC | CAGCAATATTATAGTAGTCCGTGCAGT |
| | | | SEQ ID NO:1075 | SEQ ID NO:9087 | SEQ ID NO:17099 |
| | | AA | KSSQSVLYSSNNKNYLA | WASTRES | QQYYSSPCS |
| | | | SEQ ID NO:1076 | SEQ ID NO:9088 | SEQ ID NO:17100 |
| **iPS:398520** | **21-225_31C4** | NA | CGGGCGAGTCAGGGTATTAGCAAATGGTTAGCC | GCTGCATCCAGTTTGCAGAGT | CAACAGGCTAACAGTTTCCCATTCACT |
| | | | SEQ ID NO:1077 | SEQ ID NO:9089 | SEQ ID NO:17101 |
| | | AA | RASQGISKWLA | AASSLQS | QQANSFPFT |
| | | | SEQ ID NO:1078 | SEQ ID NO:9090 | SEQ ID NO:17102 |
| **iPS:398522** | **21-225_32A1** | NA | AAGTCCAGCCAGAGTGTTTTATACAGCTCCAACAATTACAACTACTTAGCT | TGGGCATCTACCCGGAAATCC | CAACAATATTATACTTCTCCGTGCAGT |
| | | | SEQ ID NO:1079 | SEQ ID NO:9091 | SEQ ID NO:17103 |
| | | AA | KSSQSVLYSSNNYNYLA | WASTRKS | QQYYTSPCS |
| | | | SEQ ID NO:1080 | SEQ ID NO:9092 | SEQ ID NO:17104 |
| **iPS:398524** | **21-225_32A5** | NA | AAGTCCAGCCAGAGTGTTTTACACAGCTCCAACAATAAGAACTACTTAGCT | TGGGCATCTACCCGGGAATCC | CAGCAATATTATAGTTCTCCGTGCAGT |
| | | | SEQ ID NO:1081 | SEQ ID NO:9093 | SEQ ID NO:17105 |
| | | AA | KSSQSVLHSSNNKNYLA | WASTRES | QQYYSSPCS |
| | | | SEQ ID NO:1082 | SEQ ID NO:9094 | SEQ ID NO:17106 |
| **iPS:398526** | **21-225_32B3** | NA | CGGGCGAGTCAGGGCATTAGCAATTATTTAGCC | GCTGCATCCAGTTTACAAAGT | CAACAGTATAATAGTTATCCATTCACT |
| | | | SEQ ID NO:1083 | SEQ ID NO:9095 | SEQ ID NO:17107 |
| | | AA | RASQGISNYLA | AASSLQS | QQYNSYPFT |
| | | | SEQ ID NO:1084 | SEQ ID NO:9096 | SEQ ID NO:17108 |

FIGURE 49

| | | | | | |
|---|---|---|---|---|---|
| **iPS:398528** | **21-225_32G1** | NA | CGGGCAAGTCAGGACATGAGAAGTGATTTAGGC | GCTGCATCCAGTTTGCAAAGT | CTACAGCATACTATTTCCCCTCCTACT |
| | | | SEQ ID NO:1085 | SEQ ID NO:9097 | SEQ ID NO:17109 |
| | | AA | RASQDMRSDLG | AASSLQS | LQHTISPPT |
| | | | SEQ ID NO:1086 | SEQ ID NO:9098 | SEQ ID NO:17110 |
| **iPS:398530** | **21-225_32G4** | NA | AGGTCAAGTCAAAGCCTCGTATACAGTGATGGAAACACCTACTTGAAT | AAGGTTTCTAACTGGGACTCT | ATGCAAGGTATACACTGGCTCACT |
| | | | SEQ ID NO:1087 | SEQ ID NO:9099 | SEQ ID NO:17111 |
| | | AA | RSSQSLVYSDGNTYLN | KVSNWDS | MQGIHWLT |
| | | | SEQ ID NO:1088 | SEQ ID NO:9100 | SEQ ID NO:17112 |
| **iPS:398532** | **21-225_33B7** | NA | CGGGCGAGTCAGGACATTAGCAATTATTTGGCC | GCTGCATCTAGTTTGCAAAGT | CAACAGTATCATAGTTACCCGCTCACC |
| | | | SEQ ID NO:1089 | SEQ ID NO:9101 | SEQ ID NO:17113 |
| | | AA | RASQDISNYLA | AASSLQS | QQYHSYPLT |
| | | | SEQ ID NO:1090 | SEQ ID NO:9102 | SEQ ID NO:17114 |
| **iPS:398534** | **21-225_33B8** | NA | CGGGCAAGTCAGGACATTAGAAGTGATTTAGGC | GCTGCATCCAGTTTGCAAAGT | CTACAGCATACTATTTACCCTCCTACT |
| | | | SEQ ID NO:1091 | SEQ ID NO:9103 | SEQ ID NO:17115 |
| | | AA | RASQDIRSDLG | AASSLQS | LQHTIYPPT |
| | | | SEQ ID NO:1092 | SEQ ID NO:9104 | SEQ ID NO:17116 |
| **iPS:398536** | **21-225_33D12** | NA | CGGGCAAGTCAGAGCATTAGAAGCTATTTAAAT | AGTGCATCCAGTTTGCAAAGT | CAACAGAGTTACAGTATCCCGATCACC |
| | | | SEQ ID NO:1093 | SEQ ID NO:9105 | SEQ ID NO:17117 |
| | | AA | RASQSIRSYLN | SASSLQS | QQSYSIPIT |
| | | | SEQ ID NO:1094 | SEQ ID NO:9106 | SEQ ID NO:17118 |
| **iPS:398538** | **21-225_34H7** | NA | AAGTCCAGCCAGAGTGTTTTATACAGCTCCAACAATTACAACTACTTAGCT | TGGGCATCTACCCGGAAATCC | CAGCAATATTATACTTCTCCGTGCAGT |
| | | | SEQ ID NO:1095 | SEQ ID NO:9107 | SEQ ID NO:17119 |

FIGURE 49

| | | | | | |
|---|---|---|---|---|---|
| | | AA | KSSQSVLYSSNNYNYLA | WASTRKS | QQYYTSPCS |
| | | | SEQ ID NO:1096 | SEQ ID NO:9108 | SEQ ID NO:17120 |
| **iPS:398540** | **21-225_35A6** | NA | CGGGCAAGTCAGGACATTAG AAGTGATTTAGGC | GCTACATCCAGTTTGCAA AGT | CTACAGCATACTATTTACCC TCCTACT |
| | | | SEQ ID NO:1097 | SEQ ID NO:9109 | SEQ ID NO:17121 |
| | | AA | RASQDIRSDLG | ATSSLQS | LQHTIYPPT |
| | | | SEQ ID NO:1098 | SEQ ID NO:9110 | SEQ ID NO:17122 |
| **iPS:398544** | **21-225_7C8** | NA | ACCCTAAGCAGTGAGCACAG CACCTACACCATCGAA | GTTAAGAGTGATGGCAG CCACAGCAAGGGGGAC | GGAGAGAGCCACCCGATTGA TGGCCAAGTCGGTGTGGTA |
| | | | SEQ ID NO:1099 | SEQ ID NO:9111 | SEQ ID NO:17123 |
| | | AA | TLSSEHSTYTIE | VKSDGSHSKGD | GESHPIDGQVGVV |
| | | | SEQ ID NO:1100 | SEQ ID NO:9112 | SEQ ID NO:17124 |
| **iPS:398546** | **21-225_9H10** | NA | TCTGGAGATAAATTGGGGGA TAAATATGCTTGC | CAAGATAGCAAGCGGCC CTCA | CAGGCGTGGGACAGCAGCAC TTATGTGGTA |
| | | | SEQ ID NO:1101 | SEQ ID NO:9113 | SEQ ID NO:17125 |
| | | AA | SGDKLGDKYAC | QDSKRPS | QAWDSSTYVV |
| | | | SEQ ID NO:1102 | SEQ ID NO:9114 | SEQ ID NO:17126 |
| **iPS:402219** | **21-225_1C12** | NA | CGGGCAAGTCAGGGCATTAG AAATGATTTAGGC | GCTGCATCCAGTTTGCAA AGT | CTACAACATAATAGTTACCC GCTCACT |
| | | | SEQ ID NO:1103 | SEQ ID NO:9115 | SEQ ID NO:17127 |
| | | AA | RASQGIRNDLG | AASSLQS | LQHNSYPLT |
| | | | SEQ ID NO:1104 | SEQ ID NO:9116 | SEQ ID NO:17128 |
| **iPS:402221** | **21-225_2C12** | NA | CGGGCGAGTCAGGGCATTAG CAATTATTTAGCC | GCTGCAACCAGTTTGCA AAGT | CAACAGTATTATAGTTACCC GATCACC |
| | | | SEQ ID NO:1105 | SEQ ID NO:9117 | SEQ ID NO:17129 |
| | | AA | RASQGISNYLA | AATSLQS | QQYYSYPIT |
| | | | SEQ ID NO:1106 | SEQ ID NO:9118 | SEQ ID NO:17130 |
| **iPS:402223** | | NA | CGGGCGAGTCAGGGTATTAG CAGATGGTTAGCC | GCTGCATCCCGTTTGCAA AGT | CAACAGGCTAACAGTTTCCC ATTCACT |

## FIGURE 49

| | | | | | |
|---|---|---|---|---|---|
| | **21-225_30A11** | | SEQ ID NO:1107 | SEQ ID NO:9119 | SEQ ID NO:17131 |
| | | AA | RASQGISRWLA | AASRLQS | QQANSFPFT |
| | | | SEQ ID NO:1108 | SEQ ID NO:9120 | SEQ ID NO:17132 |
| **iPS:402225** | **21-225_2B1** | NA | TCTGGAGATAAATTGGGGA TAAATATGCTTGC | CAAGATCGCAAGCGGCC CTCA | CAGGCGTGGGACAACAACAC TGTGGTA |
| | | | SEQ ID NO:1109 | SEQ ID NO:9121 | SEQ ID NO:17133 |
| | | AA | SGDKLGDKYAC | QDRKRPS | QAWDNNTVV |
| | | | SEQ ID NO:1110 | SEQ ID NO:9122 | SEQ ID NO:17134 |
| **iPS:402229** | **21-225_16H9** | NA | CGGGCAAGTCAGGGCATTAG AAATTATTTAGGC | GGTGCATCCAGTTTGCA AAGT | CTACAGTATCATAGTTATCT ATTCACT |
| | | | SEQ ID NO:1111 | SEQ ID NO:9123 | SEQ ID NO:17135 |
| | | AA | RASQGIRNYLG | GASSLQS | LQYHSYLFT |
| | | | SEQ ID NO:1112 | SEQ ID NO:9124 | SEQ ID NO:17136 |
| **iPS:402231** | **21-225_6D9** | NA | TCTGGAGATAAATTGGGGA AAAATATGCTTGC | CAAGATAAGAAGCGGCC CTCA | CAGGCGTGGGACAGCAGCAC TGTA |
| | | | SEQ ID NO:1113 | SEQ ID NO:9125 | SEQ ID NO:17137 |
| | | AA | SGDKLGEKYAC | QDKKRPS | QAWDSSTV |
| | | | SEQ ID NO:1114 | SEQ ID NO:9126 | SEQ ID NO:17138 |
| **iPS:402233** | **21-225_16D10** | NA | CGGGCGAGTCAGGACATAA GTAATTATTTAGCC | GCTACACCCAGTTTGCA GAGT | CAACAGTATAATAGTTACCC GCTCACT |
| | | | SEQ ID NO:1115 | SEQ ID NO:9127 | SEQ ID NO:17139 |
| | | AA | RASQDISNYLA | ATPSLQS | QQYNSYPLT |
| | | | SEQ ID NO:1116 | SEQ ID NO:9128 | SEQ ID NO:17140 |
| **iPS:402235** | **21-225_20F10** | NA | CGGGCGAGTCAGGGCATTAA CAATTATTTAGCC | GCTGCATCCAGTTTGCAA AGT | CAGCAATATAATAGTTACCC ATTCACT |
| | | | SEQ ID NO:1117 | SEQ ID NO:9129 | SEQ ID NO:17141 |
| | | AA | RASQGINNYLA | AASSLQS | QQYNSYPFT |
| | | | SEQ ID NO:1118 | SEQ ID NO:9130 | SEQ ID NO:17142 |
| **iPS:402237** | | NA | CGGGCGAGTCAGGGCATTGC CAATTATTTAGCC | GCTGCATCCAGTTTGCAA AGT | CAACAGTATCATAGTTACCC GCTCACT |

FIGURE 49

| | | | | | |
|---|---|---|---|---|---|
| | 21-225_23D11 | | SEQ ID NO:1119 | SEQ ID NO:9131 | SEQ ID NO:17143 |
| | | AA | RASQGIANYLA | AASSLQS | QQYHSYPLT |
| | | | SEQ ID NO:1120 | SEQ ID NO:9132 | SEQ ID NO:17144 |
| iPS:403868 | 21-225_19D11 | NA | CGGGCAAGTCAGGGCATTAG AAATGATTTAGGC | GCTACATCCAGTTTGCAA AGT | CTACAGTATTATAGTTACCC GCTCACT |
| | | | SEQ ID NO:1121 | SEQ ID NO:9133 | SEQ ID NO:17145 |
| | | AA | RASQGIRNDLG | ATSSLQS | LQYYSYPLT |
| | | | SEQ ID NO:1122 | SEQ ID NO:9134 | SEQ ID NO:17146 |
| iPS:403870 | 21-225_23G4 | NA | CGGGCAAGTCAGAACATTTA CAGCTATTTAAAT | GCTGCATCCAGTTTGCAA AGT | CAACAGAGTTACAATACCCC TCCGGAGTGCAAT |
| | | | SEQ ID NO:1123 | SEQ ID NO:9135 | SEQ ID NO:17147 |
| | | AA | RASQNIYSYLN | AASSLQS | QQSYNTPPECN |
| | | | SEQ ID NO:1124 | SEQ ID NO:9136 | SEQ ID NO:17148 |
| iPS:403872 | 21-225_8F11 | NA | CGGGCAAGTCAGGGCATTAG GAGTGATTTAGGC | GATGCATCCAGTGTGCA AAGT | CTACAACATTATACTTACCC GCTCACT |
| | | | SEQ ID NO:1125 | SEQ ID NO:9137 | SEQ ID NO:17149 |
| | | AA | RASQGIRSDLG | DASSVQS | LQHYTYPLT |
| | | | SEQ ID NO:1126 | SEQ ID NO:9138 | SEQ ID NO:17150 |
| iPS:404090 | 21-225_8D8 | NA | TCTGGAGATAAATTGGGGGA GAAATATGCTTGC | CAAGATAGGAAGCGGCC CTCA | CAGGCGTGGGACAGCAGCAC TGCGGTA |
| | | | SEQ ID NO:1127 | SEQ ID NO:9139 | SEQ ID NO:17151 |
| | | AA | SGDKLGEKYAC | QDRKRPS | QAWDSSTAV |
| | | | SEQ ID NO:1128 | SEQ ID NO:9140 | SEQ ID NO:17152 |
| iPS:412232 | 21-225_4A2 | NA | AAGTCCAGCCAGAGTATTTT ACACAGCTCCAACAATAACA ACTACTTAGCT | TGGGCATCTACCCGGGA ATCC | CAGCAATATTATAATACTCC AGTCACT |
| | | | SEQ ID NO:1129 | SEQ ID NO:9141 | SEQ ID NO:17153 |
| | | AA | KSSQSILHSSNNNNYLA | WASTRES | QQYYNTPVT |
| | | | SEQ ID NO:1130 | SEQ ID NO:9142 | SEQ ID NO:17154 |

# FIGURE 49

| | | | | | |
|---|---|---|---|---|---|
| **iPS:422894** | **21-225_4A2.001** | NA | AAGTCCAGCCAGAGTATTTTACACAGCTCCAACAATAACAACTACTTAGCT | TGGGCATCTACCCGGGAATCC | CAGCAATATTATAATACTCCAGTCACT |
| | | | SEQ ID NO:1131 | SEQ ID NO:9143 | SEQ ID NO:17155 |
| | | AA | KSSQSILHSSNNNNYLA | WASTRES | QQYYNTPVT |
| | | | SEQ ID NO:1132 | SEQ ID NO:9144 | SEQ ID NO:17156 |
| **iPS:423018** | **21-225_31D12_LC2** | NA | TCTGGAGATAAATTGGGGGATAAATATGCTTAC | CAAGATAGGAAGCGGCCCTCA | CAGGCGTGGGACAACAGCACTGCGGTA |
| | | | SEQ ID NO:1133 | SEQ ID NO:9145 | SEQ ID NO:17157 |
| | | AA | SGDKLGDKYAY | QDRKRPS | QAWDNSTAV |
| | | | SEQ ID NO:1134 | SEQ ID NO:9146 | SEQ ID NO:17158 |
| **iPS:423019** | **21-225_31D12_LC1** | NA | AGGTCTAGTCAAAGCCTCATATACAGTGATGGAAACACCTTCTTGAAT | AAGGTTTCTAATTGGGACTCT | ATGCAAGGTACACACTGGCCTCTCACC |
| | | | SEQ ID NO:1135 | SEQ ID NO:9147 | SEQ ID NO:17159 |
| | | AA | RSSQSLIYSDGNTFLN | KVSNWDS | MQGTHWPLT |
| | | | SEQ ID NO:1136 | SEQ ID NO:9148 | SEQ ID NO:17160 |
| **iPS:423314** | **21-225_12F11** | NA | AAGTCCAGCCAGAGTGTTTTACACAGCTCCAACAATTATAACTACTTAGCT | TGGGCATCTACCCGGGAATCC | CAGCAATATTATGATACTCCATTCACT |
| | | | SEQ ID NO:1137 | SEQ ID NO:9149 | SEQ ID NO:17161 |
| | | AA | KSSQSVLHSSNNYNYLA | WASTRES | QQYYDTPFT |
| | | | SEQ ID NO:1138 | SEQ ID NO:9150 | SEQ ID NO:17162 |
| **iPS:424419** | **21-225_25A4.001** | NA | AAGTCCAGCCAGAGTGTTTTATACAGCTCCCACAATAACAACTACTTAGCT | TGGGCATCTACCCGGGAATCC | CAGCAGTATTATAGTACTCCTCCGACG |
| | | | SEQ ID NO:1139 | SEQ ID NO:9151 | SEQ ID NO:17163 |
| | | AA | KSSQSVLYSSHNNNYLA | WASTRES | QQYYSTPPT |
| | | | SEQ ID NO:1140 | SEQ ID NO:9152 | SEQ ID NO:17164 |

**FIGURE 49**

| | | | | | |
|---|---|---|---|---|---|
| **iPS:424460** | **21-225_7E11.001** | NA | CGGGCAAGTCAAAACATTAT CAGCTATTTAAAT | ACTGCATCCAGTTTGCAA AGT | CAACAGACTTACAGTACCCC GCTCACT |
| | | | SEQ ID NO:1141 | SEQ ID NO:9153 | SEQ ID NO:17165 |
| | | AA | RASQNIISYLN | TASSLQS | QQTYSTPLT |
| | | | SEQ ID NO:1142 | SEQ ID NO:9154 | SEQ ID NO:17166 |
| **iPS:426108** | **21-225_10G6** | NA | CGGGCGAGTCAGGGTATTAG CAAATGGTTAGCC | GCTGCATATAGTTTACAA AGT | CAACAGGCTAACAGTTTCCC ATTCACT |
| | | | SEQ ID NO:1143 | SEQ ID NO:9155 | SEQ ID NO:17167 |
| | | AA | RASQGISKWLA | AAYSLQS | QQANSFPFT |
| | | | SEQ ID NO:1144 | SEQ ID NO:9156 | SEQ ID NO:17168 |
| **iPS:426110** | **21-225_12E9** | NA | CGGGCGAGTCAGGGTATTAG CAGCTGGTTAGCC | GCTGCATCCAGGTTGCA AAGT | CAACAGGCTAACAGTTTCCC ATTCACT |
| | | | SEQ ID NO:1145 | SEQ ID NO:9157 | SEQ ID NO:17169 |
| | | AA | RASQGISSWLA | AASRLQS | QQANSFPFT |
| | | | SEQ ID NO:1146 | SEQ ID NO:9158 | SEQ ID NO:17170 |
| **iPS:426112** | **21-225_12F12** | NA | AAGTCCAGCCAGACTGTTTT ATTCAGCTCCAACAATAACC ACTACTTAGCA | TGGGCATCTACCCGGGC ATCC | CAGCAATATTATAGTAGTCC GTGGACG |
| | | | SEQ ID NO:1147 | SEQ ID NO:9159 | SEQ ID NO:17171 |
| | | AA | KSSQTVLFSSNNNHYLA | WASTRAS | QQYYSSPWT |
| | | | SEQ ID NO:1148 | SEQ ID NO:9160 | SEQ ID NO:17172 |
| **iPS:426114** | **21-225_28H2** | NA | CGGGCAAGTCAGGGCATTAG AAATGATTTAGGC | GCTGCATCCAGTTTGCAA AGT | CTACAACATTATAGTTACCC TCGCAGT |
| | | | SEQ ID NO:1149 | SEQ ID NO:9161 | SEQ ID NO:17173 |
| | | AA | RASQGIRNDLG | AASSLQS | LQHYSYPRS |
| | | | SEQ ID NO:1150 | SEQ ID NO:9162 | SEQ ID NO:17174 |
| **iPS:426116** | **21-225_29E2** | NA | CGGGCAAGTCAGGCCATTAG AAATGATTTAGGC | GCTGCATCCAGTTTGCAA AGT | TTACAGCATTATAATTACCC TCGCAGT |
| | | | SEQ ID NO:1151 | SEQ ID NO:9163 | SEQ ID NO:17175 |
| | | AA | RASQAIRNDLG | AASSLQS | LQHYNYPRS |

FIGURE 49

| | | | | | |
|---|---|---|---|---|---|
| | | | SEQ ID NO:1152 | SEQ ID NO:9164 | SEQ ID NO:17176 |
| **iPS:426118** | **21-225_7A10** | NA | CGGGCAAGTCAGAACATTTA CAGCTATTTAAAT | TCTACATCCAGTTTGCAA AGT | CAACAGAGTTACAGTCCCCC TCTCACT |
| | | | SEQ ID NO:1153 | SEQ ID NO:9165 | SEQ ID NO:17177 |
| | | AA | RASQNIYSYLN | STSSLQS | QQSYSPPLT |
| | | | SEQ ID NO:1154 | SEQ ID NO:9166 | SEQ ID NO:17178 |
| **iPS:426124** | **21-225_32D6** | NA | CGGGCAAGTCAGAACATTAT CAGCTATTTAAAT | GTTGCATCCCGTTTGCAA AGT | CAACAGAGTTACAGTACCCC GTACACT |
| | | | SEQ ID NO:1155 | SEQ ID NO:9167 | SEQ ID NO:17179 |
| | | AA | RASQNIISYLN | VASRLQS | QQSYSTPYT |
| | | | SEQ ID NO:1156 | SEQ ID NO:9168 | SEQ ID NO:17180 |
| **iPS:426126** | **21-225_6G6** | NA | AAGTCCAGCCAGAGTGTTTT ACACAACTCCAACAATTATA ACTATTTAGCT | TGGGCATCTACCCGGGA ATCC | CAGCAATATTATGATACTCC ATTCACT |
| | | | SEQ ID NO:1157 | SEQ ID NO:9169 | SEQ ID NO:17181 |
| | | AA | KSSQSVLHNSNNYNYLA | WASTRES | QQYYDTPFT |
| | | | SEQ ID NO:1158 | SEQ ID NO:9170 | SEQ ID NO:17182 |
| **iPS:433895** | **21-225_43E1** | NA | CGGGCAAGTCAGGGCATTAG AAATGATTTAGGC | GCTGCATCCAGTTTGCAA AGT | CTACAGCATAATAGTTACCC ATTCACT |
| | | | SEQ ID NO:1159 | SEQ ID NO:9171 | SEQ ID NO:17183 |
| | | AA | RASQGIRNDLG | AASSLQS | LQHNSYPFT |
| | | | SEQ ID NO:1160 | SEQ ID NO:9172 | SEQ ID NO:17184 |
| **iPS:433897** | **21-225_43C2** | NA | CGGGCGAGTCAGGGTATTAG CGACTGGTTAGCC | GCTGCATCCAGTTTGCAA AGT | CAACAGACTAACAGTTTCCC GTGGACG |
| | | | SEQ ID NO:1161 | SEQ ID NO:9173 | SEQ ID NO:17185 |
| | | AA | RASQGISDWLA | AASSLQS | QQTNSFPWT |
| | | | SEQ ID NO:1162 | SEQ ID NO:9174 | SEQ ID NO:17186 |
| **iPS:433899** | **21-225_43C3** | NA | CGGGCAAGTCAGGGCATTAG AAATGATTTAGGC | GCTGCATCCAGTTTGCAA AGT | CTACAGCATAATAGTTACCC TCTCACT |
| | | | SEQ ID NO:1163 | SEQ ID NO:9175 | SEQ ID NO:17187 |

FIGURE 49

| | | | | | |
|---|---|---|---|---|---|
| | | AA | RASQGIRNDLG | AASSLQS | LQHNSYPLT |
| | | | SEQ ID NO:1164 | SEQ ID NO:9176 | SEQ ID NO:17188 |
| **iPS:433901** | **21-225_43A4** | NA | CGGGCGAGTCAGGGCATTAA CAATTATTTAGCC | GCTGCATCCAGTTTGCAA AGT | CAACAGTATTATAGTTACCC ATTCACT |
| | | | SEQ ID NO:1165 | SEQ ID NO:9177 | SEQ ID NO:17189 |
| | | AA | RASQGINNYLA | AASSLQS | QQYYSYPFT |
| | | | SEQ ID NO:1166 | SEQ ID NO:9178 | SEQ ID NO:17190 |
| **iPS:433903** | **21-225_43H4** | NA | CGGGCGAGTCAGGGTATTAT CAACTGGTTAGCC | GCTGCATCCAGTTTGCAA AGT | CAACAGACTAACAGTTTCCC GTGGACG |
| | | | SEQ ID NO:1167 | SEQ ID NO:9179 | SEQ ID NO:17191 |
| | | AA | RASQGIINWLA | AASSLQS | QQTNSFPWT |
| | | | SEQ ID NO:1168 | SEQ ID NO:9180 | SEQ ID NO:17192 |
| **iPS:433905** | **21-225_43E5** | NA | CGGGCAAGTCAGGGCATTAG AAATGATTTAGGC | GGTGCATCCAATTTGCA AAGT | CTACAGCATACTAGTTTCCC ATTCACT |
| | | | SEQ ID NO:1169 | SEQ ID NO:9181 | SEQ ID NO:17193 |
| | | AA | RASQGIRNDLG | GASNLQS | LQHTSFPFT |
| | | | SEQ ID NO:1170 | SEQ ID NO:9182 | SEQ ID NO:17194 |
| **iPS:433909** | **21-225_43D8** | NA | AAGTCCAGCCAGAGTGTTTT AATGACCTCCAACGATAAGA ACTACTTAACT | TGGGCTTCTACCCGGGA ATCC | CAGCAATATTATAGTACTCC TCCGACG |
| | | | SEQ ID NO:1171 | SEQ ID NO:9183 | SEQ ID NO:17195 |
| | | AA | KSSQSVLMTSNDKNYLT | WASTRES | QQYYSTPPT |
| | | | SEQ ID NO:1172 | SEQ ID NO:9184 | SEQ ID NO:17196 |
| **iPS:433911** | **21-225_43E8** | NA | CGGGCGAGTCAGGGTATTAG CAACTGGTTAGCC | GCTGCATCCAGTTTGCAA AGT | CAACAGACTAACAGTTTCCC GTGGACG |
| | | | SEQ ID NO:1173 | SEQ ID NO:9185 | SEQ ID NO:17197 |
| | | AA | RASQGISNWLA | AASSLQS | QQTNSFPWT |
| | | | SEQ ID NO:1174 | SEQ ID NO:9186 | SEQ ID NO:17198 |
| **iPS:433913** | | NA | CGGGCAAGTCAGGGCATTAG AAATGATTTAGGC | GCTGCATCCAGTTTGCAA AGT | CTACAGCATAATAGTTTCCC ATTCACT |

FIGURE 49

| | | | | | |
|---|---|---|---|---|---|
| | 21-225_43H8 | | SEQ ID NO:1175 | SEQ ID NO:9187 | SEQ ID NO:17199 |
| | | AA | RASQGIRNDLG | AASSLQS | LQHNSFPFT |
| | | | SEQ ID NO:1176 | SEQ ID NO:9188 | SEQ ID NO:17200 |
| iPS:433915 | 21-225_43H9 | NA | CGGGCGAGTCAGGATATTAG CAGCTGGTTAGCC | GATGCATCCAGTTTGCA AAGT | CAACAGGCTAACAGTCTCCC ATTCACT |
| | | | SEQ ID NO:1177 | SEQ ID NO:9189 | SEQ ID NO:17201 |
| | | AA | RASQDISSWLA | DASSLQS | QQANSLPFT |
| | | | SEQ ID NO:1178 | SEQ ID NO:9190 | SEQ ID NO:17202 |
| iPS:433917 | 21-225_43E11 | NA | AAGTCTAGTCAGAGCCTCCT GCACAGTGATGGAAGGACCT ATTTGTAT | GAAGTTTCCAACCGGTTC TCT | ATGCAAAGTATACAGCTTCC GTGGACG |
| | | | SEQ ID NO:1179 | SEQ ID NO:9191 | SEQ ID NO:17203 |
| | | AA | KSSQSLLHSDGRTYLY | EVSNRFS | MQSIQLPWT |
| | | | SEQ ID NO:1180 | SEQ ID NO:9192 | SEQ ID NO:17204 |
| iPS:433919 | 21-225_44B3 | NA | CGGGCAAGTCAGGGCATTAG AAATGATTTAGGC | GCTGCATCCAGTTTGCAA AGT | CTACTGCATTATAATTACCCT CGGACG |
| | | | SEQ ID NO:1181 | SEQ ID NO:9193 | SEQ ID NO:17205 |
| | | AA | RASQGIRNDLG | AASSLQS | LLHYNYPRT |
| | | | SEQ ID NO:1182 | SEQ ID NO:9194 | SEQ ID NO:17206 |
| iPS:433921 | 21-225_44C3 | NA | CGGGCAAGTCAGGGCATTAG AAATGATTTAGGC | GCTGCATCCAGTTTGCAA AGT | CTACAGCATAGTAGTTACCC TCTCACT |
| | | | SEQ ID NO:1183 | SEQ ID NO:9195 | SEQ ID NO:17207 |
| | | AA | RASQGIRNDLG | AASSLQS | LQHSSYPLT |
| | | | SEQ ID NO:1184 | SEQ ID NO:9196 | SEQ ID NO:17208 |
| iPS:433923 | 21-225_44D3 | NA | CGGGCAAGTCAGGGCATTAG AGATGATTTAGGC | GCTGCATCCAGTTTGCAA AGT | CTACAGCATTATAGTTACCC TCGGACG |
| | | | SEQ ID NO:1185 | SEQ ID NO:9197 | SEQ ID NO:17209 |
| | | AA | RASQGIRDDLG | AASSLQS | LQHYSYPRT |
| | | | SEQ ID NO:1186 | SEQ ID NO:9198 | SEQ ID NO:17210 |

FIGURE 49

| | | | | | |
|---|---|---|---|---|---|
| **iPS:433925** | **21-225_44F3** | NA | CGGGCGAGTCAGGGTATTAGCGACTGGTTAGCC | GCTGCATCCAGTTTACAAAGT | CAACAGGCTAACAGTTTCCCATTCACT |
| | | | SEQ ID NO:1187 | SEQ ID NO:9199 | SEQ ID NO:17211 |
| | | AA | RASQGISDWLA | AASSLQS | QQANSFPFT |
| | | | SEQ ID NO:1188 | SEQ ID NO:9200 | SEQ ID NO:17212 |
| **iPS:433929** | **21-225_44D5** | NA | CGGGCAAGTCAGGACATTAGAAAAGATTTAGGC | GCTGCATCCACTTTGGAAAGT | CTACAGCATTATAGTTTCCCGTGGACG |
| | | | SEQ ID NO:1189 | SEQ ID NO:9201 | SEQ ID NO:17213 |
| | | AA | RASQDIRKDLG | AASTLES | LQHYSFPWT |
| | | | SEQ ID NO:1190 | SEQ ID NO:9202 | SEQ ID NO:17214 |
| **iPS:433931** | **21-225_44F6** | NA | AGGGCCAGTCAGAGTGTTAGTGGAAGCTACTTAGCC | GGTGCATCCAGCAGGGCCACT | CAGCAGTATGGTAGTTCACCGTGGACG |
| | | | SEQ ID NO:1191 | SEQ ID NO:9203 | SEQ ID NO:17215 |
| | | AA | RASQSVSGSYLA | GASSRAT | QQYGSSPWT |
| | | | SEQ ID NO:1192 | SEQ ID NO:9204 | SEQ ID NO:17216 |
| **iPS:433933** | **21-225_44C8** | NA | CGGGCAAGTCAGGGCATTAGAAATGATTTAGGC | GCTGCATCCAATTTGCAAAGT | CTACAGCATAATAGTTACCCATTCACT |
| | | | SEQ ID NO:1193 | SEQ ID NO:9205 | SEQ ID NO:17217 |
| | | AA | RASQGIRNDLG | AASNLQS | LQHNSYPFT |
| | | | SEQ ID NO:1194 | SEQ ID NO:9206 | SEQ ID NO:17218 |
| **iPS:433935** | **21-225_44F9** | NA | CGGGCAAGTCAGGGCATTAGAAATGATTTAGGC | GCTGCATCCAGTTTGCAAAGT | CTCCACCATTATAATTACCCTCGGACG |
| | | | SEQ ID NO:1195 | SEQ ID NO:9207 | SEQ ID NO:17219 |
| | | AA | RASQGIRNDLG | AASSLQS | LHHYNYPRT |
| | | | SEQ ID NO:1196 | SEQ ID NO:9208 | SEQ ID NO:17220 |
| **iPS:433937** | **21-225_44B10** | NA | AAGTCTAGTCAGAGCCTCCTGCATAGTGAGGGAAGGACCTATTTGTAT | GAAATTTCCCACCGGTTCTCT | ATGCAAAGTATCCACCTTCCGTTCACT |
| | | | SEQ ID NO:1197 | SEQ ID NO:9209 | SEQ ID NO:17221 |
| | | AA | KSSQSLLHSEGRTYLY | EISHRFS | MQSIHLPFT |

FIGURE 49

| | | | | | |
|---|---|---|---|---|---|
| | | | SEQ ID NO:1198 | SEQ ID NO:9210 | SEQ ID NO:17222 |
| **iPS:433939** | **21-225_44C10** | NA | CGGGCAAGTCAGGGCATTAG AGATGATTTAGGC | GCTACATCCAGTTTGCAA AGT | CTACAGCATTATAGTTACCC TCGGACG |
| | | | SEQ ID NO:1199 | SEQ ID NO:9211 | SEQ ID NO:17223 |
| | | AA | RASQGIRDDLG | ATSSLQS | LQHYSYPRT |
| | | | SEQ ID NO:1200 | SEQ ID NO:9212 | SEQ ID NO:17224 |
| **iPS:433941** | **21-225_44D10** | NA | CGGGCGAGTCAGGGTATTAG CGACTGGTTAGCC | GCTGCATCCAGTTTGCAA AGT | CAACAGACTAACAGTTTCCC GTGGACG |
| | | | SEQ ID NO:1201 | SEQ ID NO:9213 | SEQ ID NO:17225 |
| | | AA | RASQGISDWLA | AASSLQS | QQTNSFPWT |
| | | | SEQ ID NO:1202 | SEQ ID NO:9214 | SEQ ID NO:17226 |
| **iPS:433943** | **21-225_44E10** | NA | AGGTCTAGTCAGAGCCTCCT GCATAGTAATGGATACAGCT ATTTGGAG | TTGGGTTCTAATCGGGCC TCC | ATGCAAACTCTACAAACTCC ATTCACT |
| | | | SEQ ID NO:1203 | SEQ ID NO:9215 | SEQ ID NO:17227 |
| | | AA | RSSQSLLHSNGYSYLE | LGSNRAS | MQTLQTPFT |
| | | | SEQ ID NO:1204 | SEQ ID NO:9216 | SEQ ID NO:17228 |
| **iPS:433945** | **21-225_44C12** | NA | CGGGCGAGTCAGGGTATTAG CAACTGGTTAGCC | GCTGCATTCAGTTTGCAA AGT | CAACAGTCTAACAGTTTCCC GTGGACG |
| | | | SEQ ID NO:1205 | SEQ ID NO:9217 | SEQ ID NO:17229 |
| | | AA | RASQGISNWLA | AAFSLQS | QQSNSFPWT |
| | | | SEQ ID NO:1206 | SEQ ID NO:9218 | SEQ ID NO:17230 |
| **iPS:433947** | **21-225_44E12** | NA | CGGACAAGTCAGGGCATTAG AAATGATTTAGGC | GCTGCATCCAGTTTGCAA AGT | CTACAACATAATAGTTACCC GCTCACT |
| | | | SEQ ID NO:1207 | SEQ ID NO:9219 | SEQ ID NO:17231 |
| | | AA | RTSQGIRNDLG | AASSLQS | LQHNSYPLT |
| | | | SEQ ID NO:1208 | SEQ ID NO:9220 | SEQ ID NO:17232 |
| **iPS:433949** | **21-225_45H2** | NA | CGGGCAAGTCAGGGCATTAG AAATGATTTAGGC | GGTGCATCCAATTTGCA AAGT | CTACAGCATACTAGTTTCCC ATTCACT |
| | | | SEQ ID NO:1209 | SEQ ID NO:9221 | SEQ ID NO:17233 |

FIGURE 49

| | | | | | |
|---|---|---|---|---|---|
| | | AA | RASQGIRNDLG | GASNLQS | LQHTSFPFT |
| | | | SEQ ID NO:1210 | SEQ ID NO:9222 | SEQ ID NO:17234 |
| **iPS:433951** | **21-225_45B4** | NA | CGGGCAAGTCAGGGCATTAG AGATGATTTAGGC | GCTGCATCCAGTTTGCAA AGT | CTACAGCATTATAGTTACCC TCGGACG |
| | | | SEQ ID NO:1211 | SEQ ID NO:9223 | SEQ ID NO:17235 |
| | | AA | RASQGIRDDLG | AASSLQS | LQHYSYPRT |
| | | | SEQ ID NO:1212 | SEQ ID NO:9224 | SEQ ID NO:17236 |
| **iPS:433953** | **21-225_45H4** | NA | CGGGCGAGTCAGGATATTAG CAGCTGGTTAGCC | GATGCATCCAGTTTGCA AAGT | CAACAGGCTAACAGTCTCCC TTTCACT |
| | | | SEQ ID NO:1213 | SEQ ID NO:9225 | SEQ ID NO:17237 |
| | | AA | RASQDISSWLA | DASSLQS | QQANSLPFT |
| | | | SEQ ID NO:1214 | SEQ ID NO:9226 | SEQ ID NO:17238 |
| **iPS:433955** | **21-225_45B8** | NA | CGGGCAAGTCAGGACATTAG AGATGATTTAGGC | GCTGCATCCAGTTTGCAA AGT | CTACAGCATTATAATTACCC TCGGACG |
| | | | SEQ ID NO:1215 | SEQ ID NO:9227 | SEQ ID NO:17239 |
| | | AA | RASQDIRDDLG | AASSLQS | LQHYNYPRT |
| | | | SEQ ID NO:1216 | SEQ ID NO:9228 | SEQ ID NO:17240 |
| **iPS:433957** | **21-225_45F8** | NA | CGGGCGAGTCAGGGTATTAG CGACTGGTTAGCC | GGTGCATCCAGTTTGCA AAGT | CAACAGACTAACAGTTTCCC GTGGACG |
| | | | SEQ ID NO:1217 | SEQ ID NO:9229 | SEQ ID NO:17241 |
| | | AA | RASQGISDWLA | GASSLQS | QQTNSFPWT |
| | | | SEQ ID NO:1218 | SEQ ID NO:9230 | SEQ ID NO:17242 |
| **iPS:433959** | **21-225_45C9** | NA | CGGGCGAGTCAGGATATTAG CGACTGGTTAGCT | GCTGCATCCAGTTTGGA AAGT | CAACAGGCTAACAGTTTCCC ATTCACT |
| | | | SEQ ID NO:1219 | SEQ ID NO:9231 | SEQ ID NO:17243 |
| | | AA | RASQDISDWLA | AASSLES | QQANSFPFT |
| | | | SEQ ID NO:1220 | SEQ ID NO:9232 | SEQ ID NO:17244 |
| **iPS:433961** | **21-225_45D9** | NA | CGGGCGAGTCAGGGCATTAA CAATTATTTAGCC | GCTGCATCCAGTTTGCAA AGT | CAACACTATTATAGTTACCC ATTCACT |
| | | | SEQ ID NO:1221 | SEQ ID NO:9233 | SEQ ID NO:17245 |

FIGURE 49

| | | | | | |
|---|---|---|---|---|---|
| | | AA | RASQGINNYLA | AASSLQS | QHYYSYPFT |
| | | | SEQ ID NO:1222 | SEQ ID NO:9234 | SEQ ID NO:17246 |
| **iPS:433963** | **21-225_46B1** | NA | CGGGCAAGTCAGGGCATTAGAAATGATTTAGGC | GCTGCATCCAGTTTGCAAGGT | CTACAGCATAATAGTTACCCGCTCACT |
| | | | SEQ ID NO:1223 | SEQ ID NO:9235 | SEQ ID NO:17247 |
| | | AA | RASQGIRNDLG | AASSLQG | LQHNSYPLT |
| | | | SEQ ID NO:1224 | SEQ ID NO:9236 | SEQ ID NO:17248 |
| **iPS:433965** | **21-225_46F2** | NA | AAGTCTAGTCAGAGCCTCCTGCATGGTGATGGAAAGACATATTTGTAT | GAAGTTTCCAATCGGTTCTCT | ATGCAAAGTATACAGCTTCCGTGGACG |
| | | | SEQ ID NO:1225 | SEQ ID NO:9237 | SEQ ID NO:17249 |
| | | AA | KSSQSLLHGDGKTYLY | EVSNRFS | MQSIQLPWT |
| | | | SEQ ID NO:1226 | SEQ ID NO:9238 | SEQ ID NO:17250 |
| **iPS:433967** | **21-225_46C3** | NA | CGGGCAAGTCAGGGCATTAGAGATGATTTAGGC | GCTGCATCCAGTTTGCAAAGT | CTACAGCATTATAGTTACCCTCGGACG |
| | | | SEQ ID NO:1227 | SEQ ID NO:9239 | SEQ ID NO:17251 |
| | | AA | RASQGIRDDLG | AASSLQS | LQHYSYPRT |
| | | | SEQ ID NO:1228 | SEQ ID NO:9240 | SEQ ID NO:17252 |
| **iPS:433969** | **21-225_46F3** | NA | CGGGCAAGTCAGGGCATTAGAAAAGATTTAGGC | GCTGCATCCAGTTTGCAAAGT | CTACAGCATAATAGTTACCCTCTCACT |
| | | | SEQ ID NO:1229 | SEQ ID NO:9241 | SEQ ID NO:17253 |
| | | AA | RASQGIRKDLG | AASSLQS | LQHNSYPLT |
| | | | SEQ ID NO:1230 | SEQ ID NO:9242 | SEQ ID NO:17254 |
| **iPS:433971** | **21-225_46D4** | NA | CGGACAAGTCAGGACATTAGAAAAGATTTAGGC | GCTGCATCCAGTTTGGAAAGT | CTACAGCATTATAGTTTCCCGTGGACG |
| | | | SEQ ID NO:1231 | SEQ ID NO:9243 | SEQ ID NO:17255 |
| | | AA | RTSQDIRKDLG | AASSLES | LQHYSFPWT |
| | | | SEQ ID NO:1232 | SEQ ID NO:9244 | SEQ ID NO:17256 |
| **iPS:433973** | | NA | CGGGCGAGTCAGGGTATTAGCAACTGGTTAGCC | GCTGCATCCAGTTTGCAAAGT | CAACAGACTAACAGTTTCCCGTGGACG |

FIGURE 49

| | | | | | |
|---|---|---|---|---|---|
| | **21-225_46A6** | | SEQ ID NO:1233 | SEQ ID NO:9245 | SEQ ID NO:17257 |
| | | AA | RASQGISNWLA | AASSLQS | QQTNSFPWT |
| | | | SEQ ID NO:1234 | SEQ ID NO:9246 | SEQ ID NO:17258 |
| **iPS:433975** | **21-225_46C6** | NA | CGGGCAAGTCAGGGCATTAG AAATGATTTAGGC | GCTGCATCCAGTTTGCAA AGT | CTACAGCATAATAGTTACCC TCTCACT |
| | | | SEQ ID NO:1235 | SEQ ID NO:9247 | SEQ ID NO:17259 |
| | | AA | RASQGIRNDLG | AASSLQS | LQHNSYPLT |
| | | | SEQ ID NO:1236 | SEQ ID NO:9248 | SEQ ID NO:17260 |
| **iPS:433977** | **21-225_46D8** | NA | CGGGCAAGTCAGGGCATTAG AAAAGATTTAGGC | GCTGCATCCAGTTTGCAA AGT | CTACAGCATAATAGTTACCC TCTCACT |
| | | | SEQ ID NO:1237 | SEQ ID NO:9249 | SEQ ID NO:17261 |
| | | AA | RASQGIRKDLG | AASSLQS | LQHNSYPLT |
| | | | SEQ ID NO:1238 | SEQ ID NO:9250 | SEQ ID NO:17262 |
| **iPS:433979** | **21-225_46B9** | NA | AAGTCTAGTCAGAGCCTCCT GCATAGTGAGGGAAAGACC TATTTGTAT | GAAGTTTCTTACCGGTTC TCT | ATGCACAGTATACAGTATCC GCTCACG |
| | | | SEQ ID NO:1239 | SEQ ID NO:9251 | SEQ ID NO:17263 |
| | | AA | KSSQSLLHSEGKTYLY | EVSYRFS | MHSIQYPLT |
| | | | SEQ ID NO:1240 | SEQ ID NO:9252 | SEQ ID NO:17264 |
| **iPS:433981** | **21-225_46E9** | NA | CGGGCAAGTCAGGGCATTAG AAATGATTTAGGC | GCTGCATCCAGTTTGCAA AGT | CTTCAGCATACTAGTTTCCC ATTCACT |
| | | | SEQ ID NO:1241 | SEQ ID NO:9253 | SEQ ID NO:17265 |
| | | AA | RASQGIRNDLG | AASSLQS | LQHTSFPFT |
| | | | SEQ ID NO:1242 | SEQ ID NO:9254 | SEQ ID NO:17266 |
| **iPS:433983** | **21-225_47A1** | NA | CGGGCAAGTCAGGACATTAG AAATGATTTAGGC | GCTGCATTCAGTTTGCAA AGT | CTGCAACATAATAGTTACCC GCTCACT |
| | | | SEQ ID NO:1243 | SEQ ID NO:9255 | SEQ ID NO:17267 |
| | | AA | RASQDIRNDLG | AAFSLQS | LQHNSYPLT |
| | | | SEQ ID NO:1244 | SEQ ID NO:9256 | SEQ ID NO:17268 |

# FIGURE 49

| | | | | | |
|---|---|---|---|---|---|
| **iPS:433985** | **21-225_47C1** | NA | ACGTCTAGTCAGAGCCTCCTGCATAGTGAAGGAAAGACCTATTTGTAT | GAAGTTTCCAACCGGTTCTCT | ATGCAAAGTATACAGCTTCCGTGGACG |
| | | | SEQ ID NO:1245 | SEQ ID NO:9257 | SEQ ID NO:17269 |
| | | AA | TSSQSLLHSEGKTYLY | EVSNRFS | MQSIQLPWT |
| | | | SEQ ID NO:1246 | SEQ ID NO:9258 | SEQ ID NO:17270 |
| **iPS:433987** | **21-225_47A5** | NA | CGGGCAAGTCAGGGCATTAGAAATGATTTAGGC | GCTGCATCCAGTTTGCAAAGT | CTACAGCATAATAGTTACCCGCTCACT |
| | | | SEQ ID NO:1247 | SEQ ID NO:9259 | SEQ ID NO:17271 |
| | | AA | RASQGIRNDLG | AASSLQS | LQHNSYPLT |
| | | | SEQ ID NO:1248 | SEQ ID NO:9260 | SEQ ID NO:17272 |
| **iPS:433989** | **21-225_47C7** | NA | AGGTCTAGTCAGAGCCTCCTGCATAGTAATGGATACAACTATTTGGAA | TTGGGTTTTAATCGGGCCTCC | ATGCAAGTTCTACAAACTCCATTCACT |
| | | | SEQ ID NO:1249 | SEQ ID NO:9261 | SEQ ID NO:17273 |
| | | AA | RSSQSLLHSNGYNYLE | LGFNRAS | MQVLQTPFT |
| | | | SEQ ID NO:1250 | SEQ ID NO:9262 | SEQ ID NO:17274 |
| **iPS:433991** | **21-225_47E7** | NA | AAGTCTAGTCAGAGCCTCCTGCATAGTGATGGAAGGACCTATTTGTAT | GAAGTTTCCAGCCGGTTCTCT | ATGCAAAGTACACAACTTCCGTGGACG |
| | | | SEQ ID NO:1251 | SEQ ID NO:9263 | SEQ ID NO:17275 |
| | | AA | KSSQSLLHSDGRTYLY | EVSSRFS | MQSTQLPWT |
| | | | SEQ ID NO:1252 | SEQ ID NO:9264 | SEQ ID NO:17276 |
| **iPS:433993** | **21-225_47G7** | NA | CGGGCGAGTCAGGGTATTAGCAACTGGTTAGCC | GCTGCCTCCAATTTGCAAAGT | CAACAGGTTAACAGTTTCCCGTGGACG |
| | | | SEQ ID NO:1253 | SEQ ID NO:9265 | SEQ ID NO:17277 |
| | | AA | RASQGISNWLA | AASNLQS | QQVNSFPWT |
| | | | SEQ ID NO:1254 | SEQ ID NO:9266 | SEQ ID NO:17278 |
| **iPS:433995** | | NA | CGGACAAGTCAGGGCATTAGAAATGATTTAGGC | GCTGCATCCAGTTTGCAAAGT | CTACAACATACTAGTTTCCCATTCACT |

FIGURE 49

| | | | | | |
|---|---|---|---|---|---|
| | **21-225_47H7** | | SEQ ID NO:1255 | SEQ ID NO:9267 | SEQ ID NO:17279 |
| | | AA | RTSQGIRNDLG | AASSLQS | LQHTSFPFT |
| | | | SEQ ID NO:1256 | SEQ ID NO:9268 | SEQ ID NO:17280 |
| **iPS:433997** | **21-225_48C1** | NA | CGGACAAGTCAGGGCATTAG AAATGATTTAGGC | AGAGCATCCAGTTTGCA AAGT | CTACAGCATAATTTTTACCC GTGGACG |
| | | | SEQ ID NO:1257 | SEQ ID NO:9269 | SEQ ID NO:17281 |
| | | AA | RTSQGIRNDLG | RASSLQS | LQHNFYPWT |
| | | | SEQ ID NO:1258 | SEQ ID NO:9270 | SEQ ID NO:17282 |
| **iPS:433999** | **21-225_48D1** | NA | CGGGCAAGTCAGAGCATTAG CAGCTATTTAATT | GCTGCATCCAGTTTGCAA AGT | CAACAGAGTAACAGTATTCC ATTCACT |
| | | | SEQ ID NO:1259 | SEQ ID NO:9271 | SEQ ID NO:17283 |
| | | AA | RASQSISSYLI | AASSLQS | QQSNSIPFT |
| | | | SEQ ID NO:1260 | SEQ ID NO:9272 | SEQ ID NO:17284 |
| **iPS:434001** | **21-225_48F2** | NA | CGGGCAAGTCGGGGCATTAG AGATGATTTAGGC | GCTGCATCCAGTTTGCAA AGT | CTACAGCAATATAGTTATCC TCGGACG |
| | | | SEQ ID NO:1261 | SEQ ID NO:9273 | SEQ ID NO:17285 |
| | | AA | RASRGIRDDLG | AASSLQS | LQQYSYPRT |
| | | | SEQ ID NO:1262 | SEQ ID NO:9274 | SEQ ID NO:17286 |
| **iPS:434003** | **21-225_48C3** | NA | CGGGCAAGTCAGAGCATTAT CAGCTATTTAATT | GCTGCATCCAGTTTGCAA AGT | CAACAGACTAACAGTATTCC ATTCACT |
| | | | SEQ ID NO:1263 | SEQ ID NO:9275 | SEQ ID NO:17287 |
| | | AA | RASQSIISYLI | AASSLQS | QQTNSIPFT |
| | | | SEQ ID NO:1264 | SEQ ID NO:9276 | SEQ ID NO:17288 |
| **iPS:434007** | **21-225_48D7** | NA | CGGGCGAGTCAAAATATTAC CAGCTGGTTAGCC | AGTGCATCCAGTTTGCA AAAT | CAACAGGCTAACAGTTTCCC GTGGACG |
| | | | SEQ ID NO:1265 | SEQ ID NO:9277 | SEQ ID NO:17289 |
| | | AA | RASQNITSWLA | SASSLQN | QQANSFPWT |
| | | | SEQ ID NO:1266 | SEQ ID NO:9278 | SEQ ID NO:17290 |
| **iPS:434009** | | NA | CGGGCAAGTCAGGGCATTAG AAATGATTTAGGC | ATTGCATCCAGTTTGCAA AGT | CTACAGCATAATCGTTACCC GTGGACG |

FIGURE 49

| | | | | | |
|---|---|---|---|---|---|
| | **21-225_48A9** | | SEQ ID NO:1267 | SEQ ID NO:9279 | SEQ ID NO:17291 |
| | | AA | RASQGIRNDLG | IASSLQS | LQHNRYPWT |
| | | | SEQ ID NO:1268 | SEQ ID NO:9280 | SEQ ID NO:17292 |
| **iPS:434011** | **21-225_48B10** | NA | CGGGCAAGTCAGAGCATTAG GAAGTATTTAAAT | GCTGCTTCCAGTTTGCAA AGT | CAACAGACTTACAGTAACCC ACTCACT |
| | | | SEQ ID NO:1269 | SEQ ID NO:9281 | SEQ ID NO:17293 |
| | | AA | RASQSIRKYLN | AASSLQS | QQTYSNPLT |
| | | | SEQ ID NO:1270 | SEQ ID NO:9282 | SEQ ID NO:17294 |
| **iPS:434013** | **21-225_48D12** | NA | CGGGCAAGTCAGGGCATTAG AAATGATTTAGGC | GCTGCATCCACTTTGCAA AGT | CTACAGCATAATAGTTACCC GCTCACT |
| | | | SEQ ID NO:1271 | SEQ ID NO:9283 | SEQ ID NO:17295 |
| | | AA | RASQGIRNDLG | AASTLQS | LQHNSYPLT |
| | | | SEQ ID NO:1272 | SEQ ID NO:9284 | SEQ ID NO:17296 |
| **iPS:434015** | **21-225_48F12** | NA | CGGGCAAGTCAGAGCATTAG GAAGTATTTAAAT | GCTGCTTCCAGTTTGCAA AGT | CAACAGACTTACAGTAACCC GCTCACT |
| | | | SEQ ID NO:1273 | SEQ ID NO:9285 | SEQ ID NO:17297 |
| | | AA | RASQSIRKYLN | AASSLQS | QQTYSNPLT |
| | | | SEQ ID NO:1274 | SEQ ID NO:9286 | SEQ ID NO:17298 |
| **iPS:434017** | **21-225_48G12** | NA | CGGGCAAGTCAGAGCATTAG GAAGTATTTAAAT | GCTGCTTCCAGTTTGCAA AGT | CAACAGACTTACAGTAACCC GCTCACT |
| | | | SEQ ID NO:1275 | SEQ ID NO:9287 | SEQ ID NO:17299 |
| | | AA | RASQSIRKYLN | AASSLQS | QQTYSNPLT |
| | | | SEQ ID NO:1276 | SEQ ID NO:9288 | SEQ ID NO:17300 |
| **iPS:434019** | **21-225_49A1** | NA | CGGGCAAGTCAGGGCATTAG AAATGATTTAGAC | GCTGCATCCAGTTTGCAA AGT | CTACAGCATAATAGTTACCC GCTCACT |
| | | | SEQ ID NO:1277 | SEQ ID NO:9289 | SEQ ID NO:17301 |
| | | AA | RASQGIRNDLD | AASSLQS | LQHNSYPLT |
| | | | SEQ ID NO:1278 | SEQ ID NO:9290 | SEQ ID NO:17302 |

# FIGURE 49

| | | | | | |
|---|---|---|---|---|---|
| **iPS:434021** | **21-225_49C1** | NA | AAGTCTAGTCAGAGCCTCCTGCATAGGGAAGGAAAGACCTATTTGTAC | GAAGTTTCCCACCGGTTCTCT | ATGCAAAGTATACAGATTCCGATCACC |
| | | | SEQ ID NO:1279 | SEQ ID NO:9291 | SEQ ID NO:17303 |
| | | AA | KSSQSLLHREGKTYLY | EVSHRFS | MQSIQIPIT |
| | | | SEQ ID NO:1280 | SEQ ID NO:9292 | SEQ ID NO:17304 |
| **iPS:434023** | **21-225_49F1** | NA | CGGGCGAGTCGGGATATTAACGGCTGGTTAGCC | ACTGTCTCCAGTTTGCAAAGT | CAACAGTCTAACAGTTTCCCATTCACT |
| | | | SEQ ID NO:1281 | SEQ ID NO:9293 | SEQ ID NO:17305 |
| | | AA | RASRDINGWLA | TVSSLQS | QQSNSFPFT |
| | | | SEQ ID NO:1282 | SEQ ID NO:9294 | SEQ ID NO:17306 |
| **iPS:434025** | **21-225_49G3** | NA | AAGTCTAGTCAGAGCCTCCTGCATAGTGAAGGAAAGACCTATTTGTAT | GAAGTTTCCAACCGGCTCTCT | ATGCAAAGTATGCAGCTTCCGATCACC |
| | | | SEQ ID NO:1283 | SEQ ID NO:9295 | SEQ ID NO:17307 |
| | | AA | KSSQSLLHSEGKTYLY | EVSNRLS | MQSMQLPIT |
| | | | SEQ ID NO:1284 | SEQ ID NO:9296 | SEQ ID NO:17308 |
| **iPS:434027** | **21-225_49H5** | NA | CGGGCGAGTCAGGGTTTTAGCACCTGGTTAGCC | GCTGCATCCAGTTTGCAAGAT | CAACAGACTAACAGTTTCCCGTTCACT |
| | | | SEQ ID NO:1285 | SEQ ID NO:9297 | SEQ ID NO:17309 |
| | | AA | RASQGFSTWLA | AASSLQD | QQTNSFPFT |
| | | | SEQ ID NO:1286 | SEQ ID NO:9298 | SEQ ID NO:17310 |
| **iPS:434029** | **21-225_49C6** | NA | CGGGCAAGTCAGGGCATTAGAAATGATTTAGGC | GCTGCATCCAGTTTGCAAAGT | CTACAGCATAATAGTTACCCTCTCACT |
| | | | SEQ ID NO:1287 | SEQ ID NO:9299 | SEQ ID NO:17311 |
| | | AA | RASQGIRNDLG | AASSLQS | LQHNSYPLT |
| | | | SEQ ID NO:1288 | SEQ ID NO:9300 | SEQ ID NO:17312 |
| **iPS:434031** | **21 225 49E7** | NA | AAGTCTAGTCAGATCTTCTTGCATAGTGAAGGAAAGACCTATTTGTAT | GAAGTTTCCAAGCGGCTCTCT | ATGCAAAGTATGCAGCTTCCGATTATC |

FIGURE 49

| | | | | | |
|---|---|---|---|---|---|
| | 21-225_49E7 | | SEQ ID NO:1289 | SEQ ID NO:9301 | SEQ ID NO:17313 |
| | | AA | KSSQIFLHSEGKTYLY | EVSKRLS | MQSMQLPII |
| | | | SEQ ID NO:1290 | SEQ ID NO:9302 | SEQ ID NO:17314 |
| **iPS:434033** | **21-225_49F9** | NA | AAGTCTAATCAGAGCCTCGT GCATAATGAAGGAAAGACC TATTTGTAT | GAAGTTTCCAACCGGTTC TCT | ATGCAAAGTATACAGTATCC GATCACC |
| | | | SEQ ID NO:1291 | SEQ ID NO:9303 | SEQ ID NO:17315 |
| | | AA | KSNQSLVHNEGKTYLY | EVSNRFS | MQSIQYPIT |
| | | | SEQ ID NO:1292 | SEQ ID NO:9304 | SEQ ID NO:17316 |
| **iPS:434035** | **21-225_49F10** | NA | CGGGCGAGTCAGGGTATTAG CAGGTGGTTAGCC | GCTGCATCCACTTTGCAA AGT | CAACAGGCTAACAGTTTCCC ATTCACT |
| | | | SEQ ID NO:1293 | SEQ ID NO:9305 | SEQ ID NO:17317 |
| | | AA | RASQGISRWLA | AASTLQS | QQANSFPFT |
| | | | SEQ ID NO:1294 | SEQ ID NO:9306 | SEQ ID NO:17318 |
| **iPS:434037** | **21-225_49G12** | NA | CGGTCAAGTCAGAGCATTAG TACCTATTTAATG | GCTGCATCCAGTTTGCAA ATT | CAACAGAGTTACAGTATCCC ATTCACT |
| | | | SEQ ID NO:1295 | SEQ ID NO:9307 | SEQ ID NO:17319 |
| | | AA | RSSQSISTYLM | AASSLQI | QQSYSIPFT |
| | | | SEQ ID NO:1296 | SEQ ID NO:9308 | SEQ ID NO:17320 |
| **iPS:434039** | **21-225_43B1** | NA | CGGGCAAGTCAGGGCATTAG AAATGATTTAGGC | GCTGCATCCAGTTTGCAA AGT | CTACAGCATACTAGTTTCCC ATTCACT |
| | | | SEQ ID NO:1297 | SEQ ID NO:9309 | SEQ ID NO:17321 |
| | | AA | RASQGIRNDLG | AASSLQS | LQHTSFPFT |
| | | | SEQ ID NO:1298 | SEQ ID NO:9310 | SEQ ID NO:17322 |
| **iPS:434041** | **21-225_50H8** | NA | CGGGCAAGTCAGAGCATTAG CAGCTATTTAATT | GCTGCATCCAGTCTGCA AAGT | CAACAGAGTAACAGTCTTCC ATTCACT |
| | | | SEQ ID NO:1299 | SEQ ID NO:9311 | SEQ ID NO:17323 |
| | | AA | RASQSISSYLI | AASSLQS | QQSNSLPFT |
| | | | SEQ ID NO:1300 | SEQ ID NO:9312 | SEQ ID NO:17324 |

# FIGURE 49

| | | | | | |
|---|---|---|---|---|---|
| **iPS:434043** | **21-225_50G10** | NA | CGGGCAAGTCAGGGCATTAG AAATAATTTAGGC | GCTGCATCCAGTTTGCAA AGT | CTACAGTATAATAGTTACCC GTTCACT |
| | | | SEQ ID NO:1301 | SEQ ID NO:9313 | SEQ ID NO:17325 |
| | | AA | RASQGIRNNLG | AASSLQS | LQYNSYPFT |
| | | | SEQ ID NO:1302 | SEQ ID NO:9314 | SEQ ID NO:17326 |
| **iPS:434045** | **21-225_50H10** | NA | CGGGCAAGTCAGAGCATTTA CAGCTATTTAATT | GCTGCATCCAGTTTGCAA AGT | CAACAGAGTAACAGTATTCC ATTCACT |
| | | | SEQ ID NO:1303 | SEQ ID NO:9315 | SEQ ID NO:17327 |
| | | AA | RASQSIYSYLI | AASSLQS | QQSNSIPFT |
| | | | SEQ ID NO:1304 | SEQ ID NO:9316 | SEQ ID NO:17328 |
| **iPS:434047** | **21-225_50A12** | NA | CGGGCAAGTCAGGGCATTAG AAATGATTTAGGC | ACTGCATCCAATTTACAA AGT | CTACAGCATAATAGTTACCC GTGGACG |
| | | | SEQ ID NO:1305 | SEQ ID NO:9317 | SEQ ID NO:17329 |
| | | AA | RASQGIRNDLG | TASNLQS | LQHNSYPWT |
| | | | SEQ ID NO:1306 | SEQ ID NO:9318 | SEQ ID NO:17330 |
| **iPS:434049** | **21-225_50B12** | NA | CGGGCAAGTCAGAGCATTAG TAGTTATTTAAAT | GCTGCATCCAGTTTGCAA AGT | CAACAGAGTTACATTGCCCC ATTCACT |
| | | | SEQ ID NO:1307 | SEQ ID NO:9319 | SEQ ID NO:17331 |
| | | AA | RASQSISSYLN | AASSLQS | QQSYIAPFT |
| | | | SEQ ID NO:1308 | SEQ ID NO:9320 | SEQ ID NO:17332 |
| **iPS:434053** | **21-225_51E1** | NA | AAGTCTAGTCAGAGCCTCCT GCATAGTGAGGGAAAGACC TATTTGTAT | GAAGTTTCCAACCGGTTC TCT | ATGCAAAGTATACAGCTTCC ATTCACT |
| | | | SEQ ID NO:1309 | SEQ ID NO:9321 | SEQ ID NO:17333 |
| | | AA | KSSQSLLHSEGKTYLY | EVSNRFS | MQSIQLPFT |
| | | | SEQ ID NO:1310 | SEQ ID NO:9322 | SEQ ID NO:17334 |
| **iPS:434055** | **21-225_51B4** | NA | CAGGCGAGTCGGGACATTAC CTTCTATTTAAAT | GATGCATCCAATTTGGA AACA | CAACAGTATGATAATCTTCC ATTCACT |
| | | | SEQ ID NO:1311 | SEQ ID NO:9323 | SEQ ID NO:17335 |
| | | AA | QASRDITFYLN | DASNLET | QQYDNLPFT |

FIGURE 49

| | | | | | |
|---|---|---|---|---|---|
| | | | SEQ ID NO:1312 | SEQ ID NO:9324 | SEQ ID NO:17336 |
| **iPS:434057** | **21-225_51E4** | NA | CGGGCAAGTCAGGGCATTAG AAATGATTTAGGC | GCTGCATCCAGTTTGCAA AGT | CTACAGCATAATAGTTACCC ATTCACT |
| | | | SEQ ID NO:1313 | SEQ ID NO:9325 | SEQ ID NO:17337 |
| | | AA | RASQGIRNDLG | AASSLQS | LQHNSYPFT |
| | | | SEQ ID NO:1314 | SEQ ID NO:9326 | SEQ ID NO:17338 |
| **iPS:434059** | **21-225_51C5** | NA | CGGGCGAGTCAGGGCATTAG CAATTATTTAGCC | GCTGCATCCAGTTTGCGA AGT | CAACAGTATTATAGTTACCC ATTCACT |
| | | | SEQ ID NO:1315 | SEQ ID NO:9327 | SEQ ID NO:17339 |
| | | AA | RASQGISNYLA | AASSLRS | QQYYSYPFT |
| | | | SEQ ID NO:1316 | SEQ ID NO:9328 | SEQ ID NO:17340 |
| **iPS:434061** | **21-225_51C7** | NA | CGGGCGAGTCAGGATGTTAA CAACTACTTAGCC | GCTGCATCCAGTTTGCAA AAT | CAACAAACTAACAGTTTCCC ATTCACT |
| | | | SEQ ID NO:1317 | SEQ ID NO:9329 | SEQ ID NO:17341 |
| | | AA | RASQDVNNYLA | AASSLQN | QQTNSFPFT |
| | | | SEQ ID NO:1318 | SEQ ID NO:9330 | SEQ ID NO:17342 |
| **iPS:434063** | **21-225_51G7** | NA | CGGGCGAGTCAGGATATTAG CAGTTGGTTAGCC | GCTCCATCCAATTTGCAA AGT | CAACAGGCTCACAGTTTCCC GTGGACG |
| | | | SEQ ID NO:1319 | SEQ ID NO:9331 | SEQ ID NO:17343 |
| | | AA | RASQDISSWLA | APSNLQS | QQAHSFPWT |
| | | | SEQ ID NO:1320 | SEQ ID NO:9332 | SEQ ID NO:17344 |
| **iPS:434065** | **21-225_50D4** | NA | CGGGCGAGTCAGGGTATTAG CAGGTGGTTAGCC | GCTGCATCCACTTTGCAA AGT | CAACAGGCTAACAGTTTCCC ATTCACT |
| | | | SEQ ID NO:1321 | SEQ ID NO:9333 | SEQ ID NO:17345 |
| | | AA | RASQGISRWLA | AASTLQS | QQANSFPFT |
| | | | SEQ ID NO:1322 | SEQ ID NO:9334 | SEQ ID NO:17346 |
| **iPS:434067** | **21-225_51H8** | NA | CGGGCAAGTCAGGGCATTAG AAATGATTTGGGC | GCTGCTTCCAGTTTGCAA AGT | CTACAGCATAATAGTTACCC GTGGACG |
| | | | SEQ ID NO:1323 | SEQ ID NO:9335 | SEQ ID NO:17347 |
| | | AA | RASQGIRNDLG | AASSLQS | LQHNSYPWT |

FIGURE 49

| | | | | | |
|---|---|---|---|---|---|
| | | | SEQ ID NO:1324 | SEQ ID NO:9336 | SEQ ID NO:17348 |
| **iPS:434069** | **21-225_51E9** | NA | CGGGCGAGTCAGGGTATTAG CAGTTGGTTAGCC | GTTGCATCCAGTTTGCAA AGT | CAACAGGCTAAAAGTTTCCC ATTCACT |
| | | | SEQ ID NO:1325 | SEQ ID NO:9337 | SEQ ID NO:17349 |
| | | AA | RASQGISSWLA | VASSLQS | QQAKSFPFT |
| | | | SEQ ID NO:1326 | SEQ ID NO:9338 | SEQ ID NO:17350 |
| **iPS:434071** | **21-225_51F9** | NA | CGGGCAAGTCAGGGCATTAG GACTGATTTAGGC | GCTGCATCCAGTTTGCAA CGT | CTACAGCATAATAGTTACCC ATTCACT |
| | | | SEQ ID NO:1327 | SEQ ID NO:9339 | SEQ ID NO:17351 |
| | | AA | RASQGIRTDLG | AASSLQR | LQHNSYPFT |
| | | | SEQ ID NO:1328 | SEQ ID NO:9340 | SEQ ID NO:17352 |
| **iPS:434073** | **21-225_51H10** | NA | CGGGCAAGTCAGAGCATTAG TACCTATTTAATG | GCTGCATCCAGTTTGCAA AGT | CAACAGAGTTACAGTATCCC ATTCACT |
| | | | SEQ ID NO:1329 | SEQ ID NO:9341 | SEQ ID NO:17353 |
| | | AA | RASQSISTYLM | AASSLQS | QQSYSIPFT |
| | | | SEQ ID NO:1330 | SEQ ID NO:9342 | SEQ ID NO:17354 |
| **iPS:434075** | **21-225_51B11** | NA | CGGACAAGTCAGGGCATTAG AAATGATTTAGGC | GCTGCATCCAGTTTGCAA AGT | CTACAGCATAATAGTTACCC ATTCACT |
| | | | SEQ ID NO:1331 | SEQ ID NO:9343 | SEQ ID NO:17355 |
| | | AA | RTSQGIRNDLG | AASSLQS | LQHNSYPFT |
| | | | SEQ ID NO:1332 | SEQ ID NO:9344 | SEQ ID NO:17356 |
| **iPS:434077** | **21-225_51F11** | NA | CGGGCAAGTCAGGGCATTAG AAATGATTTAGGC | GCTGCATCCAGTTTGCAA AGT | CTACAGCATAATAGTTACCC GTTCACT |
| | | | SEQ ID NO:1333 | SEQ ID NO:9345 | SEQ ID NO:17357 |
| | | AA | RASQGIRNDLG | AASSLQS | LQHNSYPFT |
| | | | SEQ ID NO:1334 | SEQ ID NO:9346 | SEQ ID NO:17358 |
| **iPS:434079** | **21-225_52B1** | NA | CGGGCGAGTCAGGATATTCG CACCTGGTTAGCC | GCTGCATCCAGTTTGCAA AAT | CAACAGGCTAAAAGTTTCCC ATTCACT |
| | | | SEQ ID NO:1335 | SEQ ID NO:9347 | SEQ ID NO:17359 |
| | | AA | RASQDIRTWLA | AASSLQN | QQAKSFPFT |

FIGURE 49

| | | | | | |
|---|---|---|---|---|---|
| | | | SEQ ID NO:1336 | SEQ ID NO:9348 | SEQ ID NO:17360 |
| **iPS:434081** | **21-225_52B2** | NA | CGGGCAAGTCAGGGCATTAG AAATGATTTAGGC | GCTGCATCCTTTTTGCAA AGT | CTGCAGCATAATAGCTACCC TCTCACT |
| | | | SEQ ID NO:1337 | SEQ ID NO:9349 | SEQ ID NO:17361 |
| | | AA | RASQGIRNDLG | AASFLQS | LQHNSYPLT |
| | | | SEQ ID NO:1338 | SEQ ID NO:9350 | SEQ ID NO:17362 |
| **iPS:434083** | **21-225_52H2** | NA | CGGGCGAGTCAGAATATTAC CAACTGGTTAGCC | ACTACATCCAGTTTGCAA AGT | CAACAGACTAACAGTTTCCC GTGGACG |
| | | | SEQ ID NO:1339 | SEQ ID NO:9351 | SEQ ID NO:17363 |
| | | AA | RASQNITNWLA | TTSSLQS | QQTNSFPWT |
| | | | SEQ ID NO:1340 | SEQ ID NO:9352 | SEQ ID NO:17364 |
| **iPS:434085** | **21-225_52E3** | NA | CGGGCGAGTCAGGGCATTAG CAATTATTTAGCC | GCTGCATCCAGTTTACAA AGT | CAACAGTATAATAGTTTCCC TTTCACT |
| | | | SEQ ID NO:1341 | SEQ ID NO:9353 | SEQ ID NO:17365 |
| | | AA | RASQGISNYLA | AASSLQS | QQYNSFPFT |
| | | | SEQ ID NO:1342 | SEQ ID NO:9354 | SEQ ID NO:17366 |
| **iPS:434087** | **21-225_52F6** | NA | CAGGCGAGTCAGGACATTAG TAACTATTTACAT | GATGCATCCACTTTGGG AACA | CAACAGTGTGATAATCTCCC GCTCACT |
| | | | SEQ ID NO:1343 | SEQ ID NO:9355 | SEQ ID NO:17367 |
| | | AA | QASQDISNYLH | DASTLGT | QQCDNLPLT |
| | | | SEQ ID NO:1344 | SEQ ID NO:9356 | SEQ ID NO:17368 |
| **iPS:434091** | **21-225_52B9** | NA | CGGGCAAGTCAGGGCATTAG AAATGATTTAGGC | GCTGCATCCAGTTTGCAA AGT | CTACAGCATAATAGTTACCC ATTCACT |
| | | | SEQ ID NO:1345 | SEQ ID NO:9357 | SEQ ID NO:17369 |
| | | AA | RASQGIRNDLG | AASSLQS | LQHNSYPFT |
| | | | SEQ ID NO:1346 | SEQ ID NO:9358 | SEQ ID NO:17370 |
| **iPS:434093** | **21-225_52D10** | NA | AAGTCTAGTCAGAGCCTCCT GCATAGTGAAGGAAAGACC TATTTGTAT | GAAGTTTCCAACCGGGT CTCT | ATGCAAAGTATACAGTATCC GATCACC |
| | | | SEQ ID NO:1347 | SEQ ID NO:9359 | SEQ ID NO:17371 |

# FIGURE 49

| | | | | | |
|---|---|---|---|---|---|
| | | AA | KSSQSLLHSEGKTYLY | EVSNRVS | MQSIQYPIT |
| | | | SEQ ID NO:1348 | SEQ ID NO:9360 | SEQ ID NO:17372 |
| **iPS:434095** | **21-225_52F10** | NA | CGGGCGAGTCAGGGTATTAG TAATTATTTAGGC | GCTGCATCTAGTTTGCAA AGT | CAACAGTATAATAGTTATCC TCCGACG |
| | | | SEQ ID NO:1349 | SEQ ID NO:9361 | SEQ ID NO:17373 |
| | | AA | RASQGISNYLG | AASSLQS | QQYNSYPPT |
| | | | SEQ ID NO:1350 | SEQ ID NO:9362 | SEQ ID NO:17374 |
| **iPS:434097** | **21-225_52H10** | NA | CGGGCGAGTCAGGATATTAA CAGTTGGTTAGCC | GTTGCATCCAGTTTGCAA AGT | CAACAGGCTAAAAGTTTCCC ATTCACT |
| | | | SEQ ID NO:1351 | SEQ ID NO:9363 | SEQ ID NO:17375 |
| | | AA | RASQDINSWLA | VASSLQS | QQAKSFPFT |
| | | | SEQ ID NO:1352 | SEQ ID NO:9364 | SEQ ID NO:17376 |
| **iPS:434101** | **21-225_52H12** | NA | CGGGCAAGTCAGGGCATAA GAAATAATTTAGGC | GGTGCATCCAGTTTGCA AAGT | CTACAGTATAATAGTTATCC ATTCACT |
| | | | SEQ ID NO:1353 | SEQ ID NO:9365 | SEQ ID NO:17377 |
| | | AA | RASQGIRNNLG | GASSLQS | LQYNSYPFT |
| | | | SEQ ID NO:1354 | SEQ ID NO:9366 | SEQ ID NO:17378 |
| **iPS:434103** | **21-225_53G1** | NA | CGGGCAAGTCAGGGCATTAG AAATGATTTAGGC | CCTGCATCCAGTTTACAA AGT | CTACAGGATAATAGTTACCC ATTCACT |
| | | | SEQ ID NO:1355 | SEQ ID NO:9367 | SEQ ID NO:17379 |
| | | AA | RASQGIRNDLG | PASSLQS | LQDNSYPFT |
| | | | SEQ ID NO:1356 | SEQ ID NO:9368 | SEQ ID NO:17380 |
| **iPS:434105** | **21-225_53D2** | NA | CGGGCAAGTCAGGGCATTAG AAATGATTTAGGC | GCTGCATCCAGTTTGCAA AGT | CTACAACATAATAGATACCC GCTCACT |
| | | | SEQ ID NO:1357 | SEQ ID NO:9369 | SEQ ID NO:17381 |
| | | AA | RASQGIRNDLG | AASSLQS | LQHNRYPLT |
| | | | SEQ ID NO:1358 | SEQ ID NO:9370 | SEQ ID NO:17382 |
| **iPS:434107** | **21-225_53E2** | NA | CGGGCAAGTCAGAGTTTTAG CCACTATTTAAAT | GCTGTATCCAGTTTGCAA AGT | CAACAGAGTTTCAGTACCCC ATTCACT |
| | | | SEQ ID NO:1359 | SEQ ID NO:9371 | SEQ ID NO:17383 |

**FIGURE 49**

| | | | | | |
|---|---|---|---|---|---|
| | | AA | RASQSFSHYLN | AVSSLQS | QQSFSTPFT |
| | | | SEQ ID NO:1360 | SEQ ID NO:9372 | SEQ ID NO:17384 |
| **iPS:434111** | **21-225_53H2** | NA | CAGGCGAGTCAGGACATTAG CAACTATTTACAT | GATGCATCCAATTTGGA AACA | CATCAGTATGATAATCTCCC GCTCACT |
| | | | SEQ ID NO:1361 | SEQ ID NO:9373 | SEQ ID NO:17385 |
| | | AA | QASQDISNYLH | DASNLET | HQYDNLPLT |
| | | | SEQ ID NO:1362 | SEQ ID NO:9374 | SEQ ID NO:17386 |
| **iPS:434115** | **21-225_53E4** | NA | CGGGCGAGTCAGGTCATTAG CAATTATTTAGCC | GCTGCATCCAGTTTGCAA AGT | CAACAGTATCATAGTTACCC ACTCACT |
| | | | SEQ ID NO:1363 | SEQ ID NO:9375 | SEQ ID NO:17387 |
| | | AA | RASQVISNYLA | AASSLQS | QQYHSYPLT |
| | | | SEQ ID NO:1364 | SEQ ID NO:9376 | SEQ ID NO:17388 |
| **iPS:434117** | **21-225_53C6** | NA | CGGGCAAGTCAGTACAGTAG CGACTATTTAAAT | GCTGCATCCAGTTTGAA AAGT | CAACAGAGTTACAGTACCCC GTTCACC |
| | | | SEQ ID NO:1365 | SEQ ID NO:9377 | SEQ ID NO:17389 |
| | | AA | RASQYSSDYLN | AASSLKS | QQSYSTPFT |
| | | | SEQ ID NO:1366 | SEQ ID NO:9378 | SEQ ID NO:17390 |
| **iPS:434119** | **21-225_53E6** | NA | CGGGCAAGTCAGGGCATTAG AAATGATTTAGGC | GCTGCATCCAATTTGCAA AGT | CTACAACATAATCGTTACCC GCTCACT |
| | | | SEQ ID NO:1367 | SEQ ID NO:9379 | SEQ ID NO:17391 |
| | | AA | RASQGIRNDLG | AASNLQS | LQHNRYPLT |
| | | | SEQ ID NO:1368 | SEQ ID NO:9380 | SEQ ID NO:17392 |
| **iPS:434121** | **21-225_53F6** | NA | CAGGCGAGTCAAGACATTAC CAACTATTTAGAT | GATGCATCCAATTTGGG AACA | CAACAGTGTGATAATCTCCC GCTCACT |
| | | | SEQ ID NO:1369 | SEQ ID NO:9381 | SEQ ID NO:17393 |
| | | AA | QASQDITNYLD | DASNLGT | QQCDNLPLT |
| | | | SEQ ID NO:1370 | SEQ ID NO:9382 | SEQ ID NO:17394 |
| **iPS:434123** | **21-225_53F7** | NA | CGGGCGAGTCAGGGTATTAG CAGGTGGTTAGCC | GCTGCATCCAGTTTGCAA AGT | CAGCAGGCTAACAGTTTCCC ATTCACT |
| | | | SEQ ID NO:1371 | SEQ ID NO:9383 | SEQ ID NO:17395 |

**FIGURE 49**

| | | | | | |
|---|---|---|---|---|---|
| | | AA | RASQGISRWLA | AASSLQS | QQANSFPFT |
| | | | SEQ ID NO:1372 | SEQ ID NO:9384 | SEQ ID NO:17396 |
| **iPS:434127** | **21-225_53H8** | NA | AGGGCCAGTCAGAGTATTAC AAGCAGCTACTTAGCC | GGTGCGTCCGGCAGGGC CACT | CAGCAGTTTGAAAGCTCACC CATGTGCAGT |
| | | | SEQ ID NO:1373 | SEQ ID NO:9385 | SEQ ID NO:17397 |
| | | AA | RASQSITSSYLA | GASGRAT | QQFESSPMCS |
| | | | SEQ ID NO:1374 | SEQ ID NO:9386 | SEQ ID NO:17398 |
| **iPS:434129** | **21-225_53B12** | NA | CGGGCAAGTCAGGGCATTAG AAATGATTTAGGC | GCTGCATCCAGTTTGCAA AGT | CTACAGCATAATAGTTACCC ATTCACT |
| | | | SEQ ID NO:1375 | SEQ ID NO:9387 | SEQ ID NO:17399 |
| | | AA | RASQGIRNDLG | AASSLQS | LQHNSYPFT |
| | | | SEQ ID NO:1376 | SEQ ID NO:9388 | SEQ ID NO:17400 |
| **iPS:434131** | **21-225_54D3** | NA | CGGGCAAGTCAGGGCATTAG AAATGATTTAGGC | GCTGCATCCAGTTTGCAA AGT | CTACAACATAATAGTTACCC ATTCACT |
| | | | SEQ ID NO:1377 | SEQ ID NO:9389 | SEQ ID NO:17401 |
| | | AA | RASQGIRNDLG | AASSLQS | LQHNSYPFT |
| | | | SEQ ID NO:1378 | SEQ ID NO:9390 | SEQ ID NO:17402 |
| **iPS:434133** | **21-225_54G3** | NA | CGGGCGAGTCAGGGTATTAG CAGCTGGTTAGCC | GATGCATCCAGTTTGCA AAGT | CAACAGGCTAACAGTTTCCC GTGGACG |
| | | | SEQ ID NO:1379 | SEQ ID NO:9391 | SEQ ID NO:17403 |
| | | AA | RASQGISSWLA | DASSLQS | QQANSFPWT |
| | | | SEQ ID NO:1380 | SEQ ID NO:9392 | SEQ ID NO:17404 |
| **iPS:434135** | **21-225_54H3** | NA | CGGGCAAGTCAGGACATTAG AAATATTTTAGGC | GCTGCATCCAATTTGCAA AGT | CTACAGTATAATAGTTACCC GTGGACG |
| | | | SEQ ID NO:1381 | SEQ ID NO:9393 | SEQ ID NO:17405 |
| | | AA | RASQDIRNILG | AASNLQS | LQYNSYPWT |
| | | | SEQ ID NO:1382 | SEQ ID NO:9394 | SEQ ID NO:17406 |
| **iPS:434137** | **21 225 54D4** | NA | AAGTCTAGTCAGAGCCTCCT GCATAGTGAGGGAAAGACC TATTTGTAT | GAAGTTTCCAACCGGTTC TCT | ATGCAAAGTATACAGTTTCC ATTCACT |

FIGURE 49

| | | | | | |
|---|---|---|---|---|---|
| | 21-225_54D4 | | SEQ ID NO:1383 | SEQ ID NO:9395 | SEQ ID NO:17407 |
| | | AA | KSSQSLLHSEGKTYLY | EVSNRFS | MQSIQFPFT |
| | | | SEQ ID NO:1384 | SEQ ID NO:9396 | SEQ ID NO:17408 |
| iPS:434141 | 21-225_54C6 | NA | CGGGCAAGTCAGGGCATTAG AAATGATTTAGGC | GCTGCATCCAATTTGCAA AGT | CTACAGCATAATCGTTACCC GCTCACT |
| | | | SEQ ID NO:1385 | SEQ ID NO:9397 | SEQ ID NO:17409 |
| | | AA | RASQGIRNDLG | AASNLQS | LQHNRYPLT |
| | | | SEQ ID NO:1386 | SEQ ID NO:9398 | SEQ ID NO:17410 |
| iPS:434143 | 21-225_54G7 | NA | CGGGCAAGTCAGGGCATTAG AAATGATTTAGGC | GCTGCATCCAGTTTGCAA AGT | CTACATCATAATACTTACCC ATTCACT |
| | | | SEQ ID NO:1387 | SEQ ID NO:9399 | SEQ ID NO:17411 |
| | | AA | RASQGIRNDLG | AASSLQS | LHHNTYPFT |
| | | | SEQ ID NO:1388 | SEQ ID NO:9400 | SEQ ID NO:17412 |
| iPS:434145 | 21-225_55B1 | NA | CGGGCGAGTCAGGTTATTAG CCGCTGGTTAGCC | GCTGCATCCAGTTTGCAA AGT | CAACAGGCTAACAGTTTCCC ATTCACT |
| | | | SEQ ID NO:1389 | SEQ ID NO:9401 | SEQ ID NO:17413 |
| | | AA | RASQVISRWLA | AASSLQS | QQANSFPFT |
| | | | SEQ ID NO:1390 | SEQ ID NO:9402 | SEQ ID NO:17414 |
| iPS:434147 | 21-225_55E1 | NA | AGGGCCAGTCAGAGTGTTAG CAGCGACTTAGCC | GATGCATCCGCCAGGGC CACT | CAGCAGTATTATAACTGGCC TCTCACT |
| | | | SEQ ID NO:1391 | SEQ ID NO:9403 | SEQ ID NO:17415 |
| | | AA | RASQSVSSDLA | DASARAT | QQYYNWPLT |
| | | | SEQ ID NO:1392 | SEQ ID NO:9404 | SEQ ID NO:17416 |
| iPS:434149 | 21-225_55H1 | NA | AAATCTAGTCAGAGCCTCCT GCATAGTGAAGGAAAGACC TATTTGTAT | GAAGTTTCCCACCGGTTC TCT | ATGCAAAGTATACAGCTTCC ATTCACT |
| | | | SEQ ID NO:1393 | SEQ ID NO:9405 | SEQ ID NO:17417 |
| | | AA | KSSQSLLHSEGKTYLY | EVSHRFS | MQSIQLPFT |
| | | | SEQ ID NO:1394 | SEQ ID NO:9406 | SEQ ID NO:17418 |

FIGURE 49

| | | | | | |
|---|---|---|---|---|---|
| **iPS:434151** | **21-225_55C2** | NA | AAGTCTAGTCAGAGCCTCGTGCATAGTGAAGGAAAGACCTATTTGTAT | GAAGTTTCCAACCGGGTCTCT | ATGCAAAGTATACTGTATCCGATCACC |
| | | | SEQ ID NO:1395 | SEQ ID NO:9407 | SEQ ID NO:17419 |
| | | AA | KSSQSLVHSEGKTYLY | EVSNRVS | MQSILYPIT |
| | | | SEQ ID NO:1396 | SEQ ID NO:9408 | SEQ ID NO:17420 |
| **iPS:434155** | **21-225_55B3** | NA | CGGGCAAGTCAGGGCATTAGAAATGATTTAGGC | GCTGCATCCAGTTTGCAAAGT | CTACAACATAATAGTTACCCATTCACT |
| | | | SEQ ID NO:1397 | SEQ ID NO:9409 | SEQ ID NO:17421 |
| | | AA | RASQGIRNDLG | AASSLQS | LQHNSYPFT |
| | | | SEQ ID NO:1398 | SEQ ID NO:9410 | SEQ ID NO:17422 |
| **iPS:434157** | **21-225_55D4** | NA | CGGGCGAGTCAGGACATTAGCAATTATTTAATC | ACTGCATCCAGTTTGCAAAGT | CAACAGTATCATAGTTTCCCTCTCACT |
| | | | SEQ ID NO:1399 | SEQ ID NO:9411 | SEQ ID NO:17423 |
| | | AA | RASQDISNYLI | TASSLQS | QQYHSFPLT |
| | | | SEQ ID NO:1400 | SEQ ID NO:9412 | SEQ ID NO:17424 |
| **iPS:434159** | **21-225_55B8** | NA | CGGGCAAGTCAGGCCATTAGAAATGATTTAGGC | GCTGCATTCAGGTTGCAAAGT | CTACAGCATAATAGTTACCCTCTCACT |
| | | | SEQ ID NO:1401 | SEQ ID NO:9413 | SEQ ID NO:17425 |
| | | AA | RASQAIRNDLG | AAFRLQS | LQHNSYPLT |
| | | | SEQ ID NO:1402 | SEQ ID NO:9414 | SEQ ID NO:17426 |
| **iPS:434161** | **21-225_55F9** | NA | AAGTCTAGTCAAAGCCTCCTGCATAGTGAAGGAAAGACCTATTTGTAT | GAAGTTTCCAACCGGTTCTCT | ATACAAAGTATACAACTTCCGATCACC |
| | | | SEQ ID NO:1403 | SEQ ID NO:9415 | SEQ ID NO:17427 |
| | | AA | KSSQSLLHSEGKTYLY | EVSNRFS | IQSIQLPIT |
| | | | SEQ ID NO:1404 | SEQ ID NO:9416 | SEQ ID NO:17428 |
| **iPS:434163** | **21-225_50H1** | NA | CAGGCGAGTCAAGACATTAGCAACTATTTAGAT | GATGCATCCAATTTGGAAACA | CAACAGTGTGATAATCTCCCGCTCACT |
| | | | SEQ ID NO:1405 | SEQ ID NO:9417 | SEQ ID NO:17429 |

FIGURE 49

| | | | | | |
|---|---|---|---|---|---|
| | | AA | QASQDISNYLD | DASNLET | QQCDNLPLT |
| | | | SEQ ID NO:1406 | SEQ ID NO:9418 | SEQ ID NO:17430 |
| **iPS:434165** | **21-225_50F2** | NA | CGGGCAAGTCAGAGCATTCTCAGCTATTTGAAT | GTTGCATCCAGTTTCCAAAGT | CAACAGAGTTACAGTCCCCCTCTCACT |
| | | | SEQ ID NO:1407 | SEQ ID NO:9419 | SEQ ID NO:17431 |
| | | AA | RASQSILSYLN | VASSFQS | QQSYSPPLT |
| | | | SEQ ID NO:1408 | SEQ ID NO:9420 | SEQ ID NO:17432 |
| **iPS:434167** | **21-225_50F3** | NA | CGGGCGAGTCAGGATATTAGCAGCTGGTTGGCC | GCTGCATCCAGTTTGCAAAAT | CAACAGACTAACAGTTTCCCATTCACT |
| | | | SEQ ID NO:1409 | SEQ ID NO:9421 | SEQ ID NO:17433 |
| | | AA | RASQDISSWLA | AASSLQN | QQTNSFPFT |
| | | | SEQ ID NO:1410 | SEQ ID NO:9422 | SEQ ID NO:17434 |
| **iPS:434169** | **21-225_50C4** | NA | CGGGCAAGTCAGGGCATTAGAAATGATTTAGGC | GCTGCATCCAGTTTGCAAAGT | CTACAGCATAATCGTTACCCATTCACT |
| | | | SEQ ID NO:1411 | SEQ ID NO:9423 | SEQ ID NO:17435 |
| | | AA | RASQGIRNDLG | AASSLQS | LQHNRYPFT |
| | | | SEQ ID NO:1412 | SEQ ID NO:9424 | SEQ ID NO:17436 |
| **iPS:434171** | **21-225_50G4** | NA | CAGGCGAGTCAGGACATTACCAACTTTTTAAAT | GATGCCTCCAATTTGGAAACA | CAACAGTATGATAATCTGATCACC |
| | | | SEQ ID NO:1413 | SEQ ID NO:9425 | SEQ ID NO:17437 |
| | | AA | QASQDITNFLN | DASNLET | QQYDNLIT |
| | | | SEQ ID NO:1414 | SEQ ID NO:9426 | SEQ ID NO:17438 |
| **iPS:434175** | **21-225_55A11** | NA | CGGGCGAGTCAGGACATTAACATTTATTTAGCC | GCTGCATCCAGTTTGCAAAGT | CAACAGTATAATAGTTATCCTCTCACT |
| | | | SEQ ID NO:1415 | SEQ ID NO:9427 | SEQ ID NO:17439 |
| | | AA | RASQDINIYLA | AASSLQS | QQYNSYPLT |
| | | | SEQ ID NO:1416 | SEQ ID NO:9428 | SEQ ID NO:17440 |
| **iPS:434177** | **21 225 56A1** | NA | AAGTCCAGCCAGAGTGTTTTACATAGTTCCAACAATAAGAACTACTTAGTT | TGGGCATCTACCCGGGAATCC | CAGCAATATTATAGTACTCCTCCGACG |

**FIGURE 49**

| | | | | | |
|---|---|---|---|---|---|
| | **21-225_56A1** | | SEQ ID NO:1417 | SEQ ID NO:9429 | SEQ ID NO:17441 |
| | | AA | KSSQSVLHSSNNKNYLV | WASTRES | QQYYSTPPT |
| | | | SEQ ID NO:1418 | SEQ ID NO:9430 | SEQ ID NO:17442 |
| **iPS:434179** | **21-225_56F1** | NA | CGGGCAAGTCAGGACATTAG AAATAATTTAGGC | GCTGCATCCAGTTTACAA AGT | CTACAGGATAATAGTCACCC GTTCACT |
| | | | SEQ ID NO:1419 | SEQ ID NO:9431 | SEQ ID NO:17443 |
| | | AA | RASQDIRNNLG | AASSLQS | LQDNSHPFT |
| | | | SEQ ID NO:1420 | SEQ ID NO:9432 | SEQ ID NO:17444 |
| **iPS:434181** | **21-225_56B2** | NA | CGGGCAAGTCAGAGTTTTAG CCACTATTTAAAT | GCTGTATCCAGTTTGCAA AGT | CAACAGAGTTACAGTACCCC ATTCACT |
| | | | SEQ ID NO:1421 | SEQ ID NO:9433 | SEQ ID NO:17445 |
| | | AA | RASQSFSHYLN | AVSSLQS | QQSYSTPFT |
| | | | SEQ ID NO:1422 | SEQ ID NO:9434 | SEQ ID NO:17446 |
| **iPS:434187** | **21-225_56A5** | NA | CGGGCAAGTCAGGACATTAG AAATCTTTTAGGC | GCTGCATCCAGTTTGCAA AGT | CTACAGTATAATAGTTACCC ATTCACT |
| | | | SEQ ID NO:1423 | SEQ ID NO:9435 | SEQ ID NO:17447 |
| | | AA | RASQDIRNLLG | AASSLQS | LQYNSYPFT |
| | | | SEQ ID NO:1424 | SEQ ID NO:9436 | SEQ ID NO:17448 |
| **iPS:434189** | **21-225_56E5** | NA | CGGGCGAGTCAGGGTATTAG GAAGTGGTTAGCC | GCTGCATCCAGTTTGCAA AGT | CAACAGGCTAACAGTTTCCC ATTCACT |
| | | | SEQ ID NO:1425 | SEQ ID NO:9437 | SEQ ID NO:17449 |
| | | AA | RASQGIRKWLA | AASSLQS | QQANSFPFT |
| | | | SEQ ID NO:1426 | SEQ ID NO:9438 | SEQ ID NO:17450 |
| **iPS:434191** | **21-225_56B6** | NA | CGGGCAAGTCAGAGCATTTT CAGATATTTAAAT | GCTGCATCCAGTTTCCAA AGT | CAACAGACTTACAGTCCCCC TCTCACT |
| | | | SEQ ID NO:1427 | SEQ ID NO:9439 | SEQ ID NO:17451 |
| | | AA | RASQSIFRYLN | AASSFQS | QQTYSPPLT |
| | | | SEQ ID NO:1428 | SEQ ID NO:9440 | SEQ ID NO:17452 |
| **iPS:434193** | | NA | CGGGCGAGTCAGGGTATTAG CAGCTGGTTAGCC | GCTGCATCCAGATTGCA AAGT | CAACAGGCTAACAGTTTCCC ATTCACT |

FIGURE 49

| | | | | | |
|---|---|---|---|---|---|
| | **21-225_56C6** | | SEQ ID NO:1429 | SEQ ID NO:9441 | SEQ ID NO:17453 |
| | | AA | RASQGISSWLA | AASRLQS | QQANSFPFT |
| | | | SEQ ID NO:1430 | SEQ ID NO:9442 | SEQ ID NO:17454 |
| **iPS:434195** | **21-225_56F6** | NA | CGGGTGAGTCAGGATATTAG CAAATGGTTAGCC | GTTGCATCCGGTTTGCAA AGT | CAACAGGCTAACAGTTTCCC ATTCACT |
| | | | SEQ ID NO:1431 | SEQ ID NO:9443 | SEQ ID NO:17455 |
| | | AA | RVSQDISKWLA | VASGLQS | QQANSFPFT |
| | | | SEQ ID NO:1432 | SEQ ID NO:9444 | SEQ ID NO:17456 |
| **iPS:434197** | **21-225_56C7** | NA | CGGGCAAGTCAGGGCATTAG AAATGATTTAGGC | ACTGCATCCAATTTACAA AGT | CTACAGCATAATAGTTATCC GTGGACG |
| | | | SEQ ID NO:1433 | SEQ ID NO:9445 | SEQ ID NO:17457 |
| | | AA | RASQGIRNDLG | TASNLQS | LQHNSYPWT |
| | | | SEQ ID NO:1434 | SEQ ID NO:9446 | SEQ ID NO:17458 |
| **iPS:434199** | **21-225_59F11** | NA | CGGGCAAGTCAGGGCATTAG AAATGATTTAGGC | GCTGCATCCAGTTTGCAA AGT | CTACAGCATAATCGTTACCC TTTCACT |
| | | | SEQ ID NO:1435 | SEQ ID NO:9447 | SEQ ID NO:17459 |
| | | AA | RASQGIRNDLG | AASSLQS | LQHNRYPFT |
| | | | SEQ ID NO:1436 | SEQ ID NO:9448 | SEQ ID NO:17460 |
| **iPS:434201** | **21-225_59A12** | NA | AAGTCTAGTCAGAGCCTCCA GCATGGTGAAGGAAAGACC TATTTGTAT | GAAGTTTCCTATCGGTTT TCT | ATGCAAAGTACACAGCTTCC GCTCACC |
| | | | SEQ ID NO:1437 | SEQ ID NO:9449 | SEQ ID NO:17461 |
| | | AA | KSSQSLQHGEGKTYLY | EVSYRFS | MQSTQLPLT |
| | | | SEQ ID NO:1438 | SEQ ID NO:9450 | SEQ ID NO:17462 |
| **iPS:434203** | **21-225_60E2** | NA | CGGGCAAGTCAGGGCATTAG AAAAGATTTAGGC | GCTGCATCCAGTTTGCAA AGT | CTACAGCATAATAGTTACCC GTGGACG |
| | | | SEQ ID NO:1439 | SEQ ID NO:9451 | SEQ ID NO:17463 |
| | | AA | RASQGIRKDLG | AASSLQS | LQHNSYPWT |
| | | | SEQ ID NO:1440 | SEQ ID NO:9452 | SEQ ID NO:17464 |

FIGURE 49

| | | | | | |
|---|---|---|---|---|---|
| **iPS:434205** | **21-225_60G2** | NA | AAGTCTAGTCAGAGCCTCCTGCATAGTGAAGGAAAGACCTATTTGTAT | GAAGTTTCCAACCGGATCTCT | ATGCAAAGTATACAGCTTCCGCTCACT |
| | | | SEQ ID NO:1441 | SEQ ID NO:9453 | SEQ ID NO:17465 |
| | | AA | KSSQSLLHSEGKTYLY | EVSNRIS | MQSIQLPLT |
| | | | SEQ ID NO:1442 | SEQ ID NO:9454 | SEQ ID NO:17466 |
| **iPS:434207** | **21-225_60A3** | NA | CGGGCAAGTCAGGGCATTAGAAATGATTTAGGC | GCTGCATTTAGTTTGCAAAGT | CTACAGCATAATAGTTACCCTTTCACT |
| | | | SEQ ID NO:1443 | SEQ ID NO:9455 | SEQ ID NO:17467 |
| | | AA | RASQGIRNDLG | AAFSLQS | LQHNSYPFT |
| | | | SEQ ID NO:1444 | SEQ ID NO:9456 | SEQ ID NO:17468 |
| **iPS:434209** | **21-225_60C3** | NA | CGGGCAAGTCAGGGCATTAGAAATGATTTAGGC | TCTGCATCCAGTTTACAAAGT | CTACAGCATAATAGTTACCCGTGGACG |
| | | | SEQ ID NO:1445 | SEQ ID NO:9457 | SEQ ID NO:17469 |
| | | AA | RASQGIRNDLG | SASSLQS | LQHNSYPWT |
| | | | SEQ ID NO:1446 | SEQ ID NO:9458 | SEQ ID NO:17470 |
| **iPS:434211** | **21-225_60F3** | NA | AAGTCCAGCCAGAGTGTTTTATACAGCTCCAACAATAAGAACTACTTAGCT | TGGGCATCTACCCGGGAATCC | CAGCAATATTATAGTACTCCGTGCAGT |
| | | | SEQ ID NO:1447 | SEQ ID NO:9459 | SEQ ID NO:17471 |
| | | AA | KSSQSVLYSSNNKNYLA | WASTRES | QQYYSTPCS |
| | | | SEQ ID NO:1448 | SEQ ID NO:9460 | SEQ ID NO:17472 |
| **iPS:434213** | **21-225_60A4** | NA | CGGGCGAGTCAGGGCATTAGCAATTACTTAGCC | GCTGCATCCAGTTTGCAAAGT | CAACAATATAAAAGTCACCCATTCACT |
| | | | SEQ ID NO:1449 | SEQ ID NO:9461 | SEQ ID NO:17473 |
| | | AA | RASQGISNYLA | AASSLQS | QQYKSHPFT |
| | | | SEQ ID NO:1450 | SEQ ID NO:9462 | SEQ ID NO:17474 |
| **iPS:434215** | **21-225_60F7** | NA | CGGGCGAGTCAGGTCATTAAGAATTATTTAGTC | GCTGCGTCCAGTTTGCAAAGT | CTACAGTTTCATAGTTACCCATTCACT |
| | | | SEQ ID NO:1451 | SEQ ID NO:9463 | SEQ ID NO:17475 |

FIGURE 49

| | | | | | |
|---|---|---|---|---|---|
| | | AA | RASQVIKNYLV | AASSLQS | LQFHSYPFT |
| | | | SEQ ID NO:1452 | SEQ ID NO:9464 | SEQ ID NO:17476 |
| **iPS:434217** | **21-225_60E8** | NA | CGGGCAAGTCAGGGCATTAG AAATGATTTAGGC | GCTGCATCCAGTTTGCAA AGT | CTACAGCATAGTAGTTACCC GCTCACT |
| | | | SEQ ID NO:1453 | SEQ ID NO:9465 | SEQ ID NO:17477 |
| | | AA | RASQGIRNDLG | AASSLQS | LQHSSYPLT |
| | | | SEQ ID NO:1454 | SEQ ID NO:9466 | SEQ ID NO:17478 |
| **iPS:434219** | **21-225_60E9** | NA | AGGGCCAGTCAGAGTGTTAG CAGTTCCTTAGCC | GGTGCATCCACCAGGGC CACT | CAGCAGTATAATAACTGGCC ATTCACT |
| | | | SEQ ID NO:1455 | SEQ ID NO:9467 | SEQ ID NO:17479 |
| | | AA | RASQSVSSSLA | GASTRAT | QQYNNWPFT |
| | | | SEQ ID NO:1456 | SEQ ID NO:9468 | SEQ ID NO:17480 |
| **iPS:434221** | **21-225_60A11** | NA | CGGGCGAGTCAGGTTATTAG CAACTGGTTAGCC | ACTGCATCCAGTTTGCAA AGT | CAACAGGCTAACAGTTTCCC GTGGACG |
| | | | SEQ ID NO:1457 | SEQ ID NO:9469 | SEQ ID NO:17481 |
| | | AA | RASQVISNWLA | TASSLQS | QQANSFPWT |
| | | | SEQ ID NO:1458 | SEQ ID NO:9470 | SEQ ID NO:17482 |
| **iPS:434223** | **21-225_60C12** | NA | AAGTCTAGTCAGAGCCTCCT GCATAGTGAGGGAAAGACC TATTTGTAT | GAAGTTTCCAACCGGTTC TCT | ATGCAAAGTATAAAGTATCC GCTCACT |
| | | | SEQ ID NO:1459 | SEQ ID NO:9471 | SEQ ID NO:17483 |
| | | AA | KSSQSLLHSEGKTYLY | EVSNRFS | MQSIKYPLT |
| | | | SEQ ID NO:1460 | SEQ ID NO:9472 | SEQ ID NO:17484 |
| **iPS:434225** | **21-225_60E12** | NA | CGGGCAAGTCAGAGCATTAG CAGCTATTTAAAT | GCTGCATCCAGTTTGCAA AGT | CAACAGAGTTACAATATTTC ATTCACT |
| | | | SEQ ID NO:1461 | SEQ ID NO:9473 | SEQ ID NO:17485 |
| | | AA | RASQSISSYLN | AASSLQS | QQSYNISFT |
| | | | SEQ ID NO:1462 | SEQ ID NO:9474 | SEQ ID NO:17486 |
| **iPS:434227** | | NA | CGGGCAAGTCAGAGCATTAG CAGCTATTTAAAT | GCTGCATCCAGTTTGCAA AGT | CAACAGAGTTACAATATTTC ATTCACT |

**FIGURE 49**

| | | | | | |
|---|---|---|---|---|---|
| | **21-225_61A1** | | SEQ ID NO:1463 | SEQ ID NO:9475 | SEQ ID NO:17487 |
| | | AA | RASQSISSYLN | AASSLQS | QQSYNISFT |
| | | | SEQ ID NO:1464 | SEQ ID NO:9476 | SEQ ID NO:17488 |
| **iPS:434229** | **21-225_61H1** | NA | CGGGCAAGTCAGGGCATTAG AAAAGATTTAGGC | GCTGCATCCAGTTTGCAA AGT | CTACAGCATAATAGTTACCC GTGGACG |
| | | | SEQ ID NO:1465 | SEQ ID NO:9477 | SEQ ID NO:17489 |
| | | AA | RASQGIRKDLG | AASSLQS | LQHNSYPWT |
| | | | SEQ ID NO:1466 | SEQ ID NO:9478 | SEQ ID NO:17490 |
| **iPS:434231** | **21-225_61F2** | NA | CGGGCAAGTCAGGGCATTAG AGATGATTTAGGC | GCTGCATCCAGTTTGCAA AGT | CTACAGCATTATAGTTACCC TCGCAGT |
| | | | SEQ ID NO:1467 | SEQ ID NO:9479 | SEQ ID NO:17491 |
| | | AA | RASQGIRDDLG | AASSLQS | LQHYSYPRS |
| | | | SEQ ID NO:1468 | SEQ ID NO:9480 | SEQ ID NO:17492 |
| **iPS:434233** | **21-225_61B3** | NA | AAGTCTAGTCAGAGCCTCCT GCATAGTGAAGGAAAGACC TATTTGTAT | GAAGTTTCCAACCGGAT CTCT | ATGCAAAGTATACAGCTTCC GCTCACT |
| | | | SEQ ID NO:1469 | SEQ ID NO:9481 | SEQ ID NO:17493 |
| | | AA | KSSQSLLHSEGKTYLY | EVSNRIS | MQSIQLPLT |
| | | | SEQ ID NO:1470 | SEQ ID NO:9482 | SEQ ID NO:17494 |
| **iPS:434235** | **21-225_61E3** | NA | AAGTCCAGCCAGAGTGTTTT ACACATCTCCAACAATAACA ATTACTTAGCT | TGGGCATCTATCCGGGA ATCC | CAGCAATATTATAGTATTCC GTGCAGT |
| | | | SEQ ID NO:1471 | SEQ ID NO:9483 | SEQ ID NO:17495 |
| | | AA | KSSQSVLHISNNNNYLA | WASIRES | QQYYSIPCS |
| | | | SEQ ID NO:1472 | SEQ ID NO:9484 | SEQ ID NO:17496 |
| **iPS:434237** | **21-225_61B5** | NA | AAGTCCAGCCAGAGTGTTTT ATACAGCTCCAACAATAACA ACTCCTTAACT | TGGGCATCTACCCGGGA ATCC | CAGCAATATTATAGTACTCC TCCGACG |
| | | | SEQ ID NO:1473 | SEQ ID NO:9485 | SEQ ID NO:17497 |
| | | AA | KSSQSVLYSSNNNNSLT | WASTRES | QQYYSTPPT |

FIGURE 49

| | | | | | |
|---|---|---|---|---|---|
| | | | SEQ ID NO:1474 | SEQ ID NO:9486 | SEQ ID NO:17498 |
| **iPS:434239** | **21-225_58F1** | NA | CGGGCAAGTCAGAGCATTACCAACTTTTTAAAT | GCTGCATCCAGTTTGCAAAGT | CAACAGAGTTACAGTATCCCGTGGACG |
| | | | SEQ ID NO:1475 | SEQ ID NO:9487 | SEQ ID NO:17499 |
| | | AA | RASQSITNFLN | AASSLQS | QQSYSIPWT |
| | | | SEQ ID NO:1476 | SEQ ID NO:9488 | SEQ ID NO:17500 |
| **iPS:434241** | **21-225_61E6** | NA | CGGGCAAGTCAGGGCATTGGAAATGATTTAGGC | GCTGCATCCAGTTTGCAAAGT | CTACAACATAATAGTTTCCCTCCGTGGACG |
| | | | SEQ ID NO:1477 | SEQ ID NO:9489 | SEQ ID NO:17501 |
| | | AA | RASQGIGNDLG | AASSLQS | LQHNSFPPWT |
| | | | SEQ ID NO:1478 | SEQ ID NO:9490 | SEQ ID NO:17502 |
| **iPS:434243** | **21-225_62C1** | NA | AGGTCTAGTCAGAGCCTCCTACATAGTAATGGATACAACTATTTGGAT | TTGGTTTCTAATCGGGCCTCC | CTGCAAGCTCTACAAACTCCTCTCACC |
| | | | SEQ ID NO:1479 | SEQ ID NO:9491 | SEQ ID NO:17503 |
| | | AA | RSSQSLLHSNGYNYLD | LVSNRAS | LQALQTPLT |
| | | | SEQ ID NO:1480 | SEQ ID NO:9492 | SEQ ID NO:17504 |
| **iPS:434245** | **21-225_62H1** | NA | CGGGCAAGTCAGAACATTTTCAGCTATTTAAAT | GCTGTATTTAGTTTGCAAAGT | CAACAGAGTTACAGTACCCCATTCACT |
| | | | SEQ ID NO:1481 | SEQ ID NO:9493 | SEQ ID NO:17505 |
| | | AA | RASQNIFSYLN | AVFSLQS | QQSYSTPFT |
| | | | SEQ ID NO:1482 | SEQ ID NO:9494 | SEQ ID NO:17506 |
| **iPS:434247** | **21-225_62D2** | NA | CGGGCAAGTCAGAGCATTATCAGTTATTTAAAT | GCTACATCCAGTTTGCAAAGT | CAACAGACTTACAGTCCCCCGCTCACT |
| | | | SEQ ID NO:1483 | SEQ ID NO:9495 | SEQ ID NO:17507 |
| | | AA | RASQSIISYLN | ATSSLQS | QQTYSPPLT |
| | | | SEQ ID NO:1484 | SEQ ID NO:9496 | SEQ ID NO:17508 |
| **iPS:434249** | **21-225_62E2** | NA | CGGGCAAGTCAGGGCATTAGAAATGATTTAGGC | GCTGCATCCCGTTTGCAAAGT | CTACAGCATAGTAATTACCCTCTCACT |
| | | | SEQ ID NO:1485 | SEQ ID NO:9497 | SEQ ID NO:17509 |

FIGURE 49

| | | | | | |
|---|---|---|---|---|---|
| | | AA | RASQGIRNDLG | AASRLQS | LQHSNYPLT |
| | | | SEQ ID NO:1486 | SEQ ID NO:9498 | SEQ ID NO:17510 |
| iPS:434251 | 21-225_62G3 | NA | CGGGCAAGTCAGGACATTAG AAATAATTTAGGC | ACTGCATCCAGTTTGCAA AGT | CTACAGTATAATAGTTACCC ATTCACT |
| | | | SEQ ID NO:1487 | SEQ ID NO:9499 | SEQ ID NO:17511 |
| | | AA | RASQDIRNNLG | TASSLQS | LQYNSYPFT |
| | | | SEQ ID NO:1488 | SEQ ID NO:9500 | SEQ ID NO:17512 |
| iPS:434253 | 21-225_62E4 | NA | CGGGCAAGTCAGGGCATTAG AAATGATTTAGGC | GCTGCATTCAGTTTGCAA AGT | CTACAGCATAATAGTTATCC GCTCACT |
| | | | SEQ ID NO:1489 | SEQ ID NO:9501 | SEQ ID NO:17513 |
| | | AA | RASQGIRNDLG | AAFSLQS | LQHNSYPLT |
| | | | SEQ ID NO:1490 | SEQ ID NO:9502 | SEQ ID NO:17514 |
| iPS:434255 | 21-225_62E6 | NA | AGGGCCAGTCAGAGTGTTAA CAGCAACTTAGCC | GTTGCATCCACCAGGGC CACT | CAGCAGTATAATGACTGGCC GTGTAGT |
| | | | SEQ ID NO:1491 | SEQ ID NO:9503 | SEQ ID NO:17515 |
| | | AA | RASQSVNSNLA | VASTRAT | QQYNDWPCS |
| | | | SEQ ID NO:1492 | SEQ ID NO:9504 | SEQ ID NO:17516 |
| iPS:434257 | 21-225_62F7 | NA | CGGGCAAGTCAGGCCATTAG AAATGATTTAGGC | CCTGCATCCCGTTTGCAA AGT | CTACAGTATAATAGTTACCC TCCGTGGACG |
| | | | SEQ ID NO:1493 | SEQ ID NO:9505 | SEQ ID NO:17517 |
| | | AA | RASQAIRNDLG | PASRLQS | LQYNSYPPWT |
| | | | SEQ ID NO:1494 | SEQ ID NO:9506 | SEQ ID NO:17518 |
| iPS:434259 | 21-225_62G7 | NA | CGGGCGAGTCAGGATATTAG CAGCTGGTTAGCC | GCTGCATCCAGTTTGCAA AGT | CAACAGACTAACAGTTTCCC TCTCACT |
| | | | SEQ ID NO:1495 | SEQ ID NO:9507 | SEQ ID NO:17519 |
| | | AA | RASQDISSWLA | AASSLQS | QQTNSFPLT |
| | | | SEQ ID NO:1496 | SEQ ID NO:9508 | SEQ ID NO:17520 |
| iPS:434261 | 21-225_56F7 | NA | CGGGCGAGTCAGGGCATTAG CACTTATTTAGCC | GCTGCATCCAGTTTGCAA GGT | CATCAGTATAATAGTTTCCC ATTTAAG |
| | | | SEQ ID NO:1497 | SEQ ID NO:9509 | SEQ ID NO:17521 |

FIGURE 49

| | | | | | |
|---|---|---|---|---|---|
| | | AA | RASQGISTYLA | AASSLQG | HQYNSFPFK |
| | | | SEQ ID NO:1498 | SEQ ID NO:9510 | SEQ ID NO:17522 |
| iPS:434263 | 21-225_56H7 | NA | CGGGCAAGTCAGGACATTAG AAATGATTTAGGC | CCTGCATCCAGTTTGCTA AGT | CTACAGGATAATAGTTACCC ATTCACT |
| | | | SEQ ID NO:1499 | SEQ ID NO:9511 | SEQ ID NO:17523 |
| | | AA | RASQDIRNDLG | PASSLLS | LQDNSYPFT |
| | | | SEQ ID NO:1500 | SEQ ID NO:9512 | SEQ ID NO:17524 |
| iPS:434265 | 21-225_57B2 | NA | CGGGCAAGTCAGGGCATTAG AAATGCTTTAGGC | GCTGCATCCAGTTTGCAA AGT | CTACAGCATAATAGTTACCC ATTCACT |
| | | | SEQ ID NO:1501 | SEQ ID NO:9513 | SEQ ID NO:17525 |
| | | AA | RASQGIRNALG | AASSLQS | LQHNSYPFT |
| | | | SEQ ID NO:1502 | SEQ ID NO:9514 | SEQ ID NO:17526 |
| iPS:434267 | 21-225_57F2 | NA | CGGGCAAGTCAGAGCATTAG CAGCTATTTAAAT | GCTGCATCCAGTTTGCAA AGT | CAACAGAGTTACAATATTTC ATTCACT |
| | | | SEQ ID NO:1503 | SEQ ID NO:9515 | SEQ ID NO:17527 |
| | | AA | RASQSISSYLN | AASSLQS | QQSYNISFT |
| | | | SEQ ID NO:1504 | SEQ ID NO:9516 | SEQ ID NO:17528 |
| iPS:434269 | 21-225_57H3 | NA | AGGGCCAGTCAGAGTGTTAG CAGCAGCTTAGCC | GGTGCATCCACCAGGGC CACT | CAGCAGTATAATGACTGGCC GTGCAGT |
| | | | SEQ ID NO:1505 | SEQ ID NO:9517 | SEQ ID NO:17529 |
| | | AA | RASQSVSSSLA | GASTRAT | QQYNDWPCS |
| | | | SEQ ID NO:1506 | SEQ ID NO:9518 | SEQ ID NO:17530 |
| iPS:434271 | 21-225_57A4 | NA | CGGGCAAGTCAGGGCATTAG AAATGATTTAGGC | GCTGCATCCAGTTTGCTA AGT | CTACAGCATAATAGTTACCC ATTCACT |
| | | | SEQ ID NO:1507 | SEQ ID NO:9519 | SEQ ID NO:17531 |
| | | AA | RASQGIRNDLG | AASSLLS | LQHNSYPFT |
| | | | SEQ ID NO:1508 | SEQ ID NO:9520 | SEQ ID NO:17532 |
| iPS:434273 | 21-225_57E4 | NA | CGGGCGAGTCAGGATATTAG CAACTGGTTAGCC | GCTGCATCCAGTTTGCAA AGT | CAACAGGGTAACAGTTTCCC ATTCACT |
| | | | SEQ ID NO:1509 | SEQ ID NO:9521 | SEQ ID NO:17533 |

FIGURE 49

| | | | | | |
|---|---|---|---|---|---|
| | | AA | RASQDISNWLA | AASSLQS | QQGNSFPFT |
| | | | SEQ ID NO:1510 | SEQ ID NO:9522 | SEQ ID NO:17534 |
| **iPS:434275** | **21-225_57F4** | NA | CGGGCAAGTCAGGTCATTAG AAATGATTTAGGC | GCTGCATCCAGTTTGCAA AGT | CTACAGTATGGTAGTTTCCC ATTCACT |
| | | | SEQ ID NO:1511 | SEQ ID NO:9523 | SEQ ID NO:17535 |
| | | AA | RASQVIRNDLG | AASSLQS | LQYGSFPFT |
| | | | SEQ ID NO:1512 | SEQ ID NO:9524 | SEQ ID NO:17536 |
| **iPS:434277** | **21-225_57A7** | NA | CGGGCGAGTCAGGGTATTAG CAGGTGGTTAGCC | GCTGCATCCAATTTGCAA AGT | CAACAGGCTAACAGTTTCCC ATTCACT |
| | | | SEQ ID NO:1513 | SEQ ID NO:9525 | SEQ ID NO:17537 |
| | | AA | RASQGISRWLA | AASNLQS | QQANSFPFT |
| | | | SEQ ID NO:1514 | SEQ ID NO:9526 | SEQ ID NO:17538 |
| **iPS:434279** | **21-225_57F7** | NA | AGGGCCAGTCAGAGTGTTAG CAGCGACTTAGCC | GGTGCATCCACCAGGGC CACT | CAGCAGTATAGTAACTGGCC ATTCACT |
| | | | SEQ ID NO:1515 | SEQ ID NO:9527 | SEQ ID NO:17539 |
| | | AA | RASQSVSSDLA | GASTRAT | QQYSNWPFT |
| | | | SEQ ID NO:1516 | SEQ ID NO:9528 | SEQ ID NO:17540 |
| **iPS:434281** | **21-225_57B8** | NA | CGGGCAAGTCAGGGCATTGG AAATGATTTAGGC | GCTGCATCCAGTTTGCAA AGT | CTACAACATAATAGTTTCCC TCCGTGGACG |
| | | | SEQ ID NO:1517 | SEQ ID NO:9529 | SEQ ID NO:17541 |
| | | AA | RASQGIGNDLG | AASSLQS | LQHNSFPPWT |
| | | | SEQ ID NO:1518 | SEQ ID NO:9530 | SEQ ID NO:17542 |
| **iPS:434283** | **21-225_57F8** | NA | CGGGCGAGTCAGGGTATTAG CAACTGGTTAGCC | ACTGCATCCAGTTTGCAA AGT | CAACAGGCTAACAGTTTCCC GTGGACG |
| | | | SEQ ID NO:1519 | SEQ ID NO:9531 | SEQ ID NO:17543 |
| | | AA | RASQGISNWLA | TASSLQS | QQANSFPWT |
| | | | SEQ ID NO:1520 | SEQ ID NO:9532 | SEQ ID NO:17544 |
| **iPS:434285** | **21 225 57A11** | NA | AAGTCCAGCCAAAGTGTTTT ACACAGCTCCAACAATTATA ACTACTTAGCT | TGGGCATCTACCCGGGC ATCC | CAGCAATATTATAGTACTCC GTGGACG |

FIGURE 49

| | | | | | |
|---|---|---|---|---|---|
| | 21-225_57A11 | | SEQ ID NO:1521 | SEQ ID NO:9533 | SEQ ID NO:17545 |
| | | AA | KSSQSVLHSSNNYNYLA | WASTRAS | QQYYSTPWT |
| | | | SEQ ID NO:1522 | SEQ ID NO:9534 | SEQ ID NO:17546 |
| **iPS:434287** | **21-225_57F12** | NA | AAGTCCAGCCAGAGTGTTTTATTCAGCTCCAACAATTACAATTACTTAGCT | TGGGCATCTACCCGGGAATCC | CAGCAATATTATAGTAATCCGTGTAGT |
| | | | SEQ ID NO:1523 | SEQ ID NO:9535 | SEQ ID NO:17547 |
| | | AA | KSSQSVLFSSNNYNYLA | WASTRES | QQYYSNPCS |
| | | | SEQ ID NO:1524 | SEQ ID NO:9536 | SEQ ID NO:17548 |
| **iPS:434289** | **21-225_57H12** | NA | AGGGCCAGTCAGAGTGTTAGCAGCGACTTAGCC | GCTGCATCTACCAGGGCCACT | CAGCAGTATGATAACTGGCCATTCACT |
| | | | SEQ ID NO:1525 | SEQ ID NO:9537 | SEQ ID NO:17549 |
| | | AA | RASQSVSSDLA | AASTRAT | QQYDNWPFT |
| | | | SEQ ID NO:1526 | SEQ ID NO:9538 | SEQ ID NO:17550 |
| **iPS:434291** | **21-225_58A4** | NA | AGGGCCAGTCAGAGTGTTAGCAGCGACTTAGCC | GCTGCATCTACCAGGGCCACT | CAGCAGTTTAATAACTGGCCATTCACT |
| | | | SEQ ID NO:1527 | SEQ ID NO:9539 | SEQ ID NO:17551 |
| | | AA | RASQSVSSDLA | AASTRAT | QQFNNWPFT |
| | | | SEQ ID NO:1528 | SEQ ID NO:9540 | SEQ ID NO:17552 |
| **iPS:434293** | **21-225_58F5** | NA | CGGGCAAGTCAGGGCATTAGAAATGATTTAGGC | GCTGCATCCAGTTTGCTAAGT | CTACAGCATAATAGTTACCCATTCACT |
| | | | SEQ ID NO:1529 | SEQ ID NO:9541 | SEQ ID NO:17553 |
| | | AA | RASQGIRNDLG | AASSLLS | LQHNSYPFT |
| | | | SEQ ID NO:1530 | SEQ ID NO:9542 | SEQ ID NO:17554 |
| **iPS:434295** | **21-225_58B9** | NA | AAGTCCGGCCAGAGTATTTTATACAGCTCCAACAATAACAACTACTTAGCT | TGGGCATCTACCCGGGATTCC | CAGCAATATTATAGTACTCCTCCGACG |
| | | | SEQ ID NO:1531 | SEQ ID NO:9543 | SEQ ID NO:17555 |
| | | AA | KSGQSILYSSNNNNYLA | WASTRDS | QQYYSTPPT |
| | | | SEQ ID NO:1532 | SEQ ID NO:9544 | SEQ ID NO:17556 |

**FIGURE 49**

| | | | | | |
|---|---|---|---|---|---|
| **iPS:434297** | **21-225_58A10** | NA | AGGGCCAGTCAGAGTGTTAGCAGCTCCTTAGCC | GGTGCATCCACCAGGGCCACT | CAGCAGTATAATAACTGGCCATTCACT |
| | | | SEQ ID NO:1533 | SEQ ID NO:9545 | SEQ ID NO:17557 |
| | | AA | RASQSVSSSLA | GASTRAT | QQYNNWPFT |
| | | | SEQ ID NO:1534 | SEQ ID NO:9546 | SEQ ID NO:17558 |
| **iPS:434299** | **21-225_58D11** | NA | CGGGCAAGTCAGGGCATTAGAAGTGATTTAGAC | GCTGCATCCAGTTTGCAAAGT | CTCCAGCATAATAATTTCCCATTCACT |
| | | | SEQ ID NO:1535 | SEQ ID NO:9547 | SEQ ID NO:17559 |
| | | AA | RASQGIRSDLD | AASSLQS | LQHNNFPFT |
| | | | SEQ ID NO:1536 | SEQ ID NO:9548 | SEQ ID NO:17560 |
| **iPS:434301** | **21-225_58F11** | NA | AGGGCCAGTCAGAGTGTTAGCAGCGACTTAGTC | GGTGTATCCACCAGGGCCACT | CAGCAGTATAATAACTGGCCATTCACT |
| | | | SEQ ID NO:1537 | SEQ ID NO:9549 | SEQ ID NO:17561 |
| | | AA | RASQSVSSDLV | GVSTRAT | QQYNNWPFT |
| | | | SEQ ID NO:1538 | SEQ ID NO:9550 | SEQ ID NO:17562 |
| **iPS:434303** | **21-225_58H11** | NA | AAGTCTAGTCAGAGCCTCCTGCATAGTGAGGGAAAGACCTATTTGTAT | GAAGTTTCCTATCGGTTTTCT | ATGCAAAGTATACAGCTTCCGCTCACT |
| | | | SEQ ID NO:1539 | SEQ ID NO:9551 | SEQ ID NO:17563 |
| | | AA | KSSQSLLHSEGKTYLY | EVSYRFS | MQSIQLPLT |
| | | | SEQ ID NO:1540 | SEQ ID NO:9552 | SEQ ID NO:17564 |
| **iPS:434305** | **21-225_59E1** | NA | AAGTCCAGCCAGAGTGTTTTATACAGCTCCAACAATAACAACTACTTAGCT | TGGTCATCTACCCGGGAATCC | CAGCAATATTTTAGTATTCCGTGCAGT |
| | | | SEQ ID NO:1541 | SEQ ID NO:9553 | SEQ ID NO:17565 |
| | | AA | KSSQSVLYSSNNNNYLA | WSSTRES | QQYFSIPCS |
| | | | SEQ ID NO:1542 | SEQ ID NO:9554 | SEQ ID NO:17566 |
| **iPS:434307** | **21-225_59B2** | NA | TGGGCCAGTCAGAGTGTTTACAGCAGCTTCTTAGCC | GGTGCATCCAGCAGGGCCACT | CAGCAATATGGTACCTCACCGTGGACG |
| | | | SEQ ID NO:1543 | SEQ ID NO:9555 | SEQ ID NO:17567 |

## FIGURE 49

| | | | | | |
|---|---|---|---|---|---|
| | | AA | WASQSVYSSFLA | GASSRAT | QQYGTSPWT |
| | | | SEQ ID NO:1544 | SEQ ID NO:9556 | SEQ ID NO:17568 |
| **iPS:434309** | **21-225_59B5** | NA | CGGGCAAGTCAGAGCATTAT CAGCTATTTAAAT | GGTGCATCCAGTTTGCA GAGT | CAACAGAGTTACAGTACCCC TATGTTCAGT |
| | | | SEQ ID NO:1545 | SEQ ID NO:9557 | SEQ ID NO:17569 |
| | | AA | RASQSIISYLN | GASSLQS | QQSYSTPMFS |
| | | | SEQ ID NO:1546 | SEQ ID NO:9558 | SEQ ID NO:17570 |
| **iPS:434311** | **21-225_59H5** | NA | AGGGCCAGTCAGAGTGTTAG CAGCATCTACTTAGCC | GGTGCATCCAGCAGGGC CACT | CACCAGTATGGTAACTCACC ATTCACT |
| | | | SEQ ID NO:1547 | SEQ ID NO:9559 | SEQ ID NO:17571 |
| | | AA | RASQSVSSIYLA | GASSRAT | HQYGNSPFT |
| | | | SEQ ID NO:1548 | SEQ ID NO:9560 | SEQ ID NO:17572 |
| **iPS:434313** | **21-225_59E6** | NA | CGGGCAAGTCAGGGCATTAG AAATGATTTAGGC | GCTGCATCCAGTTTGCAA AGT | CTACAGCATAATAGTTACCC GCTCACT |
| | | | SEQ ID NO:1549 | SEQ ID NO:9561 | SEQ ID NO:17573 |
| | | AA | RASQGIRNDLG | AASSLQS | LQHNSYPLT |
| | | | SEQ ID NO:1550 | SEQ ID NO:9562 | SEQ ID NO:17574 |
| **iPS:434315** | **21-225_59G7** | NA | CGGGCAAGTCAGGGCATTAG AAATGATTTAGGC | TCTGCATCCAGTTTACAA AGT | CTACAGCATAATAGTTACCC GTGGACG |
| | | | SEQ ID NO:1551 | SEQ ID NO:9563 | SEQ ID NO:17575 |
| | | AA | RASQGIRNDLG | SASSLQS | LQHNSYPWT |
| | | | SEQ ID NO:1552 | SEQ ID NO:9564 | SEQ ID NO:17576 |
| **iPS:434317** | **21-225_59E8** | NA | CGGGCAAGTCAGAGCATTAG CAGCTATTTAAAT | GCTGCATCCAGTTTGCAA AGT | CAACAGAGTTTCAGTAATTC GATCACC |
| | | | SEQ ID NO:1553 | SEQ ID NO:9565 | SEQ ID NO:17577 |
| | | AA | RASQSISSYLN | AASSLQS | QQSFSNSIT |
| | | | SEQ ID NO:1554 | SEQ ID NO:9566 | SEQ ID NO:17578 |
| **iPS:434319** | **21-225_59B9** | NA | CGGGCAAGTCAGGACATTAG AAATGATTTAGGC | GCTGCATCCAGTTTACAA AGT | CTACAGCATAATAGTTACCC GTGGACG |
| | | | SEQ ID NO:1555 | SEQ ID NO:9567 | SEQ ID NO:17579 |

FIGURE 49

| | | | | | |
|---|---|---|---|---|---|
| | | AA | RASQDIRNDLG | AASSLQS | LQHNSYPWT |
| | | | SEQ ID NO:1556 | SEQ ID NO:9568 | SEQ ID NO:17580 |
| **iPS:434321** | **21-225_59F10** | NA | AAGTCCAGCCAGACTGTTTTATACAGGTCCAACAATTACAACTACTTAGCT | TGGGCATCTACCCGGGAATCC | CAGCAATATTTTAGTACTCCTCCGACG |
| | | | SEQ ID NO:1557 | SEQ ID NO:9569 | SEQ ID NO:17581 |
| | | AA | KSSQTVLYRSNNYNYLA | WASTRES | QQYFSTPPT |
| | | | SEQ ID NO:1558 | SEQ ID NO:9570 | SEQ ID NO:17582 |
| **iPS:434323** | **21-225_62H8** | NA | CGGGCAAGTCAGAGCATTTTCAGCTATTTAAAT | GCGTCATCCAGTTTGCAAAGT | CAACAGAGTTACAGTACCCCATTCACT |
| | | | SEQ ID NO:1559 | SEQ ID NO:9571 | SEQ ID NO:17583 |
| | | AA | RASQSIFSYLN | ASSSLQS | QQSYSTPFT |
| | | | SEQ ID NO:1560 | SEQ ID NO:9572 | SEQ ID NO:17584 |
| **iPS:434327** | **21-225_63G6** | NA | CGGGCAAGTCAGAGCATTTTCAGCTATTTAAAT | GATACATCCACTTTGCAAACT | CAACAGAGTTACGGTATCCCCATCACC |
| | | | SEQ ID NO:1561 | SEQ ID NO:9573 | SEQ ID NO:17585 |
| | | AA | RASQSIFSYLN | DTSTLQT | QQSYGIPIT |
| | | | SEQ ID NO:1562 | SEQ ID NO:9574 | SEQ ID NO:17586 |
| **iPS:434331** | **21-225_63H8** | NA | CGGGCGAGTCAGGGCATTAGCAATTATTTAGCC | GCTGCATCCAGTTTGCAAAGT | CAACAATATCATAGTTACCCATTCACT |
| | | | SEQ ID NO:1563 | SEQ ID NO:9575 | SEQ ID NO:17587 |
| | | AA | RASQGISNYLA | AASSLQS | QQYHSYPFT |
| | | | SEQ ID NO:1564 | SEQ ID NO:9576 | SEQ ID NO:17588 |
| **iPS:434333** | **21-225_63C9** | NA | CGGGCGAGTCAGGGTATTAGCAGCTGGTTAGCC | GCTGCATCCAGTTTGCAAAGT | CAACAGATTAACAGTTTCCCTCTCACT |
| | | | SEQ ID NO:1565 | SEQ ID NO:9577 | SEQ ID NO:17589 |
| | | AA | RASQGISSWLA | AASSLQS | QQINSFPLT |
| | | | SEQ ID NO:1566 | SEQ ID NO:9578 | SEQ ID NO:17590 |
| **iPS:434335** | | NA | CGGGCAAGTCAGAGCATTTTCAGCTATTTACAT | GCTGCATCCAGTTTACAAAGT | CAACAGACTTACAGTCCCCCGCTCACT |

FIGURE 49

| | | | | | |
|---|---|---|---|---|---|
| | 21-225_63C10 | | SEQ ID NO:1567 | SEQ ID NO:9579 | SEQ ID NO:17591 |
| | | AA | RASQSIFSYLH | AASSLQS | QQTYSPPLT |
| | | | SEQ ID NO:1568 | SEQ ID NO:9580 | SEQ ID NO:17592 |
| iPS:434337 | 21-225_64E1 | NA | CGGGCAAGTCAGGGCATTAGAAATGATTTAGGC | GCTGCATCCAGTTTGCAAAGT | CTACAGCATAATAGTTACCCGCTCACT |
| | | | SEQ ID NO:1569 | SEQ ID NO:9581 | SEQ ID NO:17593 |
| | | AA | RASQGIRNDLG | AASSLQS | LQHNSYPLT |
| | | | SEQ ID NO:1570 | SEQ ID NO:9582 | SEQ ID NO:17594 |
| iPS:434339 | 21-225_64A4 | NA | CGGGCAAGTCAGGGCATTAGAAATGATTTAGGC | GCTGCATCCAGTTTGCAAAGT | CTACAGCATTATAGTTACCCTCGGACG |
| | | | SEQ ID NO:1571 | SEQ ID NO:9583 | SEQ ID NO:17595 |
| | | AA | RASQGIRNDLG | AASSLQS | LQHYSYPRT |
| | | | SEQ ID NO:1572 | SEQ ID NO:9584 | SEQ ID NO:17596 |
| iPS:434341 | 21-225_64F7 | NA | CGGGCAAGTCAGAACATTAAGAAATATTTAAAT | GGTGCATCCAGTTTGCAAAGT | CAACAGAGTTACAATATTTCGTTCACT |
| | | | SEQ ID NO:1573 | SEQ ID NO:9585 | SEQ ID NO:17597 |
| | | AA | RASQNIKKYLN | GASSLQS | QQSYNISFT |
| | | | SEQ ID NO:1574 | SEQ ID NO:9586 | SEQ ID NO:17598 |
| iPS:434343 | 21-225_64C8 | NA | CGGGCAAGTCAGGGCATTAGAAATGATTTAGGC | GCTGCATCCAGTTTGCAAAGT | CTACACCATTATAGTTACCCTCGGACG |
| | | | SEQ ID NO:1575 | SEQ ID NO:9587 | SEQ ID NO:17599 |
| | | AA | RASQGIRNDLG | AASSLQS | LHHYSYPRT |
| | | | SEQ ID NO:1576 | SEQ ID NO:9588 | SEQ ID NO:17600 |
| iPS:434345 | 21-225_64H9 | NA | AAGTCTAGTCAGAGCCTCCTTCATGGTGATGGAAAGACCTATTTGTTT | GAAGTTTCCAACCGGTTGTGT | ATGCAAAGTATACAGGTTCCGTGGACG |
| | | | SEQ ID NO:1577 | SEQ ID NO:9589 | SEQ ID NO:17601 |
| | | AA | KSSQSLLHGDGKTYLF | EVSNRLC | MQSIQVPWT |
| | | | SEQ ID NO:1578 | SEQ ID NO:9590 | SEQ ID NO:17602 |

# FIGURE 49

| | | | | | |
|---|---|---|---|---|---|
| **iPS:434347** | **21-225_64H10** | NA | CGGGCGAGTCAGGGTATTAG CAGCTGGTTAGCC | GCTGCATCCAGTTTGCAA AGT | CAACAGATTAACAGTTTCCC TCTCACT |
| | | | SEQ ID NO:1579 | SEQ ID NO:9591 | SEQ ID NO:17603 |
| | | AA | RASQGISSWLA | AASSLQS | QQINSFPLT |
| | | | SEQ ID NO:1580 | SEQ ID NO:9592 | SEQ ID NO:17604 |
| **iPS:434351** | **21-225_64A12** | NA | CGGGCAAGTCAGGGCATTAG AAATGATTTAGGC | ACTGCATCCACTTTGCAA AGT | CTACAGCATAATGGTTACCC ATTCACT |
| | | | SEQ ID NO:1581 | SEQ ID NO:9593 | SEQ ID NO:17605 |
| | | AA | RASQGIRNDLG | TASTLQS | LQHNGYPFT |
| | | | SEQ ID NO:1582 | SEQ ID NO:9594 | SEQ ID NO:17606 |
| **iPS:434353** | **21-225_64B12** | NA | CGGGCGAGTCAGGACATTAG CAATTATTTAAAT | GATGCATCCATTTTGGAA ACA | CAACAGAGTGATAATCTCCC GTGCAGT |
| | | | SEQ ID NO:1583 | SEQ ID NO:9595 | SEQ ID NO:17607 |
| | | AA | RASQDISNYLN | DASILET | QQSDNLPCS |
| | | | SEQ ID NO:1584 | SEQ ID NO:9596 | SEQ ID NO:17608 |
| **iPS:434355** | **21-225_64G12** | NA | CGGGCGAGTCAGAATATTAC CACCTGGTTAGCC | GCTGCATCCAGTTTGCAA AGT | CAACAGGCTAACAGTTTTCC ATTCACT |
| | | | SEQ ID NO:1585 | SEQ ID NO:9597 | SEQ ID NO:17609 |
| | | AA | RASQNITTWLA | AASSLQS | QQANSFPFT |
| | | | SEQ ID NO:1586 | SEQ ID NO:9598 | SEQ ID NO:17610 |
| **iPS:434357** | **21-225_65C1** | NA | CGGGCGAGTCAGGTCATTAG CAGTTATTTACAT | AGTGCATCCAATTTGCA ATGT | CAACGGCCTTACAATGCCCC GCTCACT |
| | | | SEQ ID NO:1587 | SEQ ID NO:9599 | SEQ ID NO:17611 |
| | | AA | RASQVISSYLH | SASNLQC | QRPYNAPLT |
| | | | SEQ ID NO:1588 | SEQ ID NO:9600 | SEQ ID NO:17612 |
| **iPS:434359** | **21-225_65G3** | NA | CGGGCGAGTCAGGGTATTAG CAGCTGGTTAGCC | GCTGCATCCAGTTTGCAA AGT | CAACAGGTTAACAGTTTCCC TCTCACT |
| | | | SEQ ID NO:1589 | SEQ ID NO:9601 | SEQ ID NO:17613 |
| | | AA | RASQGISSWLA | AASSLQS | QQVNSFPLT |
| | | | SEQ ID NO:1590 | SEQ ID NO:9602 | SEQ ID NO:17614 |

FIGURE 49

| | | | | | |
|---|---|---|---|---|---|
| **iPS:434361** | **21-225_65D5** | NA | CGGGCGAGTCAGGACATTAA CAATTATTTAGCC | GCTGCATCCAGTTTGCAC AGT | CCACTGTATAAAAGTTATCC ACTCACT |
| | | | SEQ ID NO:1591 | SEQ ID NO:9603 | SEQ ID NO:17615 |
| | | AA | RASQDINNYLA | AASSLHS | PLYKSYPLT |
| | | | SEQ ID NO:1592 | SEQ ID NO:9604 | SEQ ID NO:17616 |
| **iPS:434363** | **21-225_65A6** | NA | AGGGCCAGTCAGAGTGTTAA CAGCAACTTAGCC | GGTGCATCCACCAGGGC CACT | CAGCAGTATAATGACTGGCC GTGCAGT |
| | | | SEQ ID NO:1593 | SEQ ID NO:9605 | SEQ ID NO:17617 |
| | | AA | RASQSVNSNLA | GASTRAT | QQYNDWPCS |
| | | | SEQ ID NO:1594 | SEQ ID NO:9606 | SEQ ID NO:17618 |
| **iPS:434367** | **21-225_65H11** | NA | CGGGCGAGTCAGGACATTAG CACTTATTTAGCC | GCTGCATCCAGTTTGCAA AGT | CAACAGTATAATAGTTTCCC TCTCACT |
| | | | SEQ ID NO:1595 | SEQ ID NO:9607 | SEQ ID NO:17619 |
| | | AA | RASQDISTYLA | AASSLQS | QQYNSFPLT |
| | | | SEQ ID NO:1596 | SEQ ID NO:9608 | SEQ ID NO:17620 |
| **iPS:434369** | **21-225_66B1** | NA | CGGGCGAGTCAGGGTATTAG CAGCTGGTTAGCC | GCTGCATCCAGTTTGCAA AGT | CAACAGACTAACAGTTTCCC TCTCACT |
| | | | SEQ ID NO:1597 | SEQ ID NO:9609 | SEQ ID NO:17621 |
| | | AA | RASQGISSWLA | AASSLQS | QQTNSFPLT |
| | | | SEQ ID NO:1598 | SEQ ID NO:9610 | SEQ ID NO:17622 |
| **iPS:434373** | **21-225_66A7** | NA | CGGGCGAGTCAGGGTATTAG CAGCTGGTTAGCC | GCTGCATCCAGTTTGCAA AGT | CAACAGATTAATAGTTTCCC TCTCACT |
| | | | SEQ ID NO:1599 | SEQ ID NO:9611 | SEQ ID NO:17623 |
| | | AA | RASQGISSWLA | AASSLQS | QQINSFPLT |
| | | | SEQ ID NO:1600 | SEQ ID NO:9612 | SEQ ID NO:17624 |
| **iPS:434375** | **21-225_66C7** | NA | CGGGCGAGTCAGGGCATTAG CAATTATTTACAT | TGTGCATCCAATTTACAA TGT | CAACAGCATAATAATTCCCC GCTCACT |
| | | | SEQ ID NO:1601 | SEQ ID NO:9613 | SEQ ID NO:17625 |
| | | AA | RASQGISNYLH | CASNLQC | QQHNNSPLT |
| | | | SEQ ID NO:1602 | SEQ ID NO:9614 | SEQ ID NO:17626 |

FIGURE 49

| | | | | | |
|---|---|---|---|---|---|
| **iPS:434379** | **21-225_66A9** | NA | CGGGCAAGTCAGAACATTTTCAGCTATTTAAAT | GCTGCATCCAGTTTACAAAGT | CAACAGACTTACAGTGTCCCTTTCACT |
| | | | SEQ ID NO:1603 | SEQ ID NO:9615 | SEQ ID NO:17627 |
| | | AA | RASQNIFSYLN | AASSLQS | QQTYSVPFT |
| | | | SEQ ID NO:1604 | SEQ ID NO:9616 | SEQ ID NO:17628 |
| **iPS:434383** | **21-225_66F9** | NA | CGGGCAAGTCAGGACATTAGAAATGTTTTAGGC | ACTGCATCCAGTTTACAAAGT | CTACAGTATAATAGTTACCCATTCACT |
| | | | SEQ ID NO:1605 | SEQ ID NO:9617 | SEQ ID NO:17629 |
| | | AA | RASQDIRNVLG | TASSLQS | LQYNSYPFT |
| | | | SEQ ID NO:1606 | SEQ ID NO:9618 | SEQ ID NO:17630 |
| **iPS:434385** | **21-225_66C10** | NA | CGGGCAAGTCAGGCCATTAGAAATGATTTAGGC | CCTGCATCCAGTTTGCAAAGT | CTACAGTATAATAGTTACCCTCCGTGGACG |
| | | | SEQ ID NO:1607 | SEQ ID NO:9619 | SEQ ID NO:17631 |
| | | AA | RASQAIRNDLG | PASSLQS | LQYNSYPPWT |
| | | | SEQ ID NO:1608 | SEQ ID NO:9620 | SEQ ID NO:17632 |
| **iPS:434387** | **21-225_66D11** | NA | CGGGCAAGTCAGGGCATTAGAAATGATTTAGGT | GCTGCATCCAGTTGTCAAAGT | ATAGTGCATAATAGTTACCCTCGGACG |
| | | | SEQ ID NO:1609 | SEQ ID NO:9621 | SEQ ID NO:17633 |
| | | AA | RASQGIRNDLG | AASSCQS | IVHNSYPRT |
| | | | SEQ ID NO:1610 | SEQ ID NO:9622 | SEQ ID NO:17634 |
| **iPS:434389** | **21-225_66F11** | NA | CGGGAGAGTCAGGGTATTAGCATCTGGTTAGCC | GCTGCATCCAGTTTGCAAAGT | CAACAGGCTAACAGTTTCCCATTCACT |
| | | | SEQ ID NO:1611 | SEQ ID NO:9623 | SEQ ID NO:17635 |
| | | AA | RESQGISIWLA | AASSLQS | QQANSFPFT |
| | | | SEQ ID NO:1612 | SEQ ID NO:9624 | SEQ ID NO:17636 |
| **iPS:434393** | **21-225_67C3** | NA | AGGGCCAGTCAGAGTGTTAACAGCAACTTAGCC | ATTGCATCCACCAGGGCCACT | CAGCAGTATAATGACTGGCCGTGTAGT |
| | | | SEQ ID NO:1613 | SEQ ID NO:9625 | SEQ ID NO:17637 |
| | | AA | RASQSVNSNLA | IASTRAT | QQYNDWPCS |
| | | | SEQ ID NO:1614 | SEQ ID NO:9626 | SEQ ID NO:17638 |

FIGURE 49

| | | | | | |
|---|---|---|---|---|---|
| **iPS:434397** | **21-225_67H4** | NA | CGGGCGAGTCAGGGTATTAG CAGCTGGTTAGCC | GCTGCATCCAGTTTGCAA AGT | CAACAGATTAACAGTTTCCC TCTCACT |
| | | | SEQ ID NO:1615 | SEQ ID NO:9627 | SEQ ID NO:17639 |
| | | AA | RASQGISSWLA | AASSLQS | QQINSFPLT |
| | | | SEQ ID NO:1616 | SEQ ID NO:9628 | SEQ ID NO:17640 |
| **iPS:434399** | **21-225_67B7** | NA | CGGGCAAGTCAGGGCATTAG AAATGATTTAGGC | GCTGCATCCAGTTTGCAA AGT | CTACACCATAATAGTTATCC ATTCAAA |
| | | | SEQ ID NO:1617 | SEQ ID NO:9629 | SEQ ID NO:17641 |
| | | AA | RASQGIRNDLG | AASSLQS | LHHNSYPFK |
| | | | SEQ ID NO:1618 | SEQ ID NO:9630 | SEQ ID NO:17642 |
| **iPS:434405** | **21-225_68E6** | NA | CGGGCGAGTCAGGGCATTAG CTATTATTTAGCC | GTTGCATCCAGTTTGCAA AGT | CAACAGTATGATAGTTACCC ATTCACT |
| | | | SEQ ID NO:1619 | SEQ ID NO:9631 | SEQ ID NO:17643 |
| | | AA | RASQGISYYLA | VASSLQS | QQYDSYPFT |
| | | | SEQ ID NO:1620 | SEQ ID NO:9632 | SEQ ID NO:17644 |
| **iPS:434407** | **21-225_68G8** | NA | CGGGCAAGTCAGGGCATTAG AAATAATTTAGGC | GCTGCATCCAGTGTGCA AAGT | CTACAGTATAATAGTTACCC ATTCACT |
| | | | SEQ ID NO:1621 | SEQ ID NO:9633 | SEQ ID NO:17645 |
| | | AA | RASQGIRNNLG | AASSVQS | LQYNSYPFT |
| | | | SEQ ID NO:1622 | SEQ ID NO:9634 | SEQ ID NO:17646 |
| **iPS:434411** | **21-225_68F11** | NA | CGGGCAAGTCAGGGCATTAG AAATGATTTAGGC | GCTGCATCCAGTTTGCAA AGT | CTACAGCATAATAGTTATCC GTTCACT |
| | | | SEQ ID NO:1623 | SEQ ID NO:9635 | SEQ ID NO:17647 |
| | | AA | RASQGIRNDLG | AASSLQS | LQHNSYPFT |
| | | | SEQ ID NO:1624 | SEQ ID NO:9636 | SEQ ID NO:17648 |
| **iPS:434413** | **21-225_68D12** | NA | CGGGCAAGTCAGGGCATTAG AAATGATTTAGGC | GCTGCATCCAGTTTGCAA AGT | CTACAGCATAGTACTTACCC GCTCACT |
| | | | SEQ ID NO:1625 | SEQ ID NO:9637 | SEQ ID NO:17649 |
| | | AA | RASQGIRNDLG | AASSLQS | LQHSTYPLT |
| | | | SEQ ID NO:1626 | SEQ ID NO:9638 | SEQ ID NO:17650 |

FIGURE 49

| | | | | | |
|---|---|---|---|---|---|
| **iPS:434417** | **21-225_69C8** | NA | CGGGCAGGTCAGACCATTTA CAATTATTTAAAT | GTTGCGTCCAGTTTGCAA AGT | CAACAGAGTTACAGTACCCC ATTCACT |
| | | | SEQ ID NO:1627 | SEQ ID NO:9639 | SEQ ID NO:17651 |
| | | AA | RAGQTIYNYLN | VASSLQS | QQSYSTPFT |
| | | | SEQ ID NO:1628 | SEQ ID NO:9640 | SEQ ID NO:17652 |
| **iPS:434423** | **21-225_70D1** | NA | CGGGCGAGTCAGGGTGTTAG CAGGTGGTTAGCC | GCTGCATCCAGTTTGCAA AGT | CAACAGGCTAACAGTTTCCC ATTCACT |
| | | | SEQ ID NO:1629 | SEQ ID NO:9641 | SEQ ID NO:17653 |
| | | AA | RASQGVSRWLA | AASSLQS | QQANSFPFT |
| | | | SEQ ID NO:1630 | SEQ ID NO:9642 | SEQ ID NO:17654 |
| **iPS:434425** | **21-225_70A5** | NA | AGGGCCAGTCAGAGTGTTAA CAGCAACTTAGCC | ATTGCATCCACCAGGGC CACT | CAGCAGTATAATGACTGGCC GTGTAGT |
| | | | SEQ ID NO:1631 | SEQ ID NO:9643 | SEQ ID NO:17655 |
| | | AA | RASQSVNSNLA | IASTRAT | QQYNDWPCS |
| | | | SEQ ID NO:1632 | SEQ ID NO:9644 | SEQ ID NO:17656 |
| **iPS:434427** | **21-225_70D6** | NA | CGGGCGAGTCAGGGTATTAG CAAATGGTTAGCC | GCTGCATCCAGTTTGCAA AGT | CAACAGACTAACAGTTTCCC TCTCACT |
| | | | SEQ ID NO:1633 | SEQ ID NO:9645 | SEQ ID NO:17657 |
| | | AA | RASQGISKWLA | AASSLQS | QQTNSFPLT |
| | | | SEQ ID NO:1634 | SEQ ID NO:9646 | SEQ ID NO:17658 |
| **iPS:434429** | **21-225_70H6** | NA | CGGACAAGTCAGAGCATTTT CAACTATTTAAAT | ACTGCATCCAGTTTGCAA AGT | CAACAGAGTTACAGTATCCC GCTCACT |
| | | | SEQ ID NO:1635 | SEQ ID NO:9647 | SEQ ID NO:17659 |
| | | AA | RTSQSIFNYLN | TASSLQS | QQSYSIPLT |
| | | | SEQ ID NO:1636 | SEQ ID NO:9648 | SEQ ID NO:17660 |
| **iPS:434431** | **21-225_70E7** | NA | AAGTCCAGCCAGAGTGTTTT ATACAGCTCCAACAATAACA ACTACTTGGCT | TGGGCATCTACCCGGGA ATCC | CAGCAATATTATAATATTCC TCCGACG |
| | | | SEQ ID NO:1637 | SEQ ID NO:9649 | SEQ ID NO:17661 |
| | | AA | KSSQSVLYSSNNNNYLA | WASTRES | QQYYNIPPT |

FIGURE 49

| | | | | | |
|---|---|---|---|---|---|
| | | | SEQ ID NO:1638 | SEQ ID NO:9650 | SEQ ID NO:17662 |
| **iPS:434433** | **21-225_70E8** | NA | CGGGCAAGTCAGGGCATTAGAAAGGATTTAGGC | GCTGCATCCAGTTTGCAAAGT | CTACAGCATAATCGTTACCCGCTCACT |
| | | | SEQ ID NO:1639 | SEQ ID NO:9651 | SEQ ID NO:17663 |
| | | AA | RASQGIRKDLG | AASSLQS | LQHNRYPLT |
| | | | SEQ ID NO:1640 | SEQ ID NO:9652 | SEQ ID NO:17664 |
| **iPS:434435** | **21-225_70G9** | NA | CGGGCGAGTCAGGATATTAGCAGCTGGTTAGCC | GCTGCATCCAGTTTGCAAAGT | CAACAGACTAACAGTTTCCCTCTCACT |
| | | | SEQ ID NO:1641 | SEQ ID NO:9653 | SEQ ID NO:17665 |
| | | AA | RASQDISSWLA | AASSLQS | QQTNSFPLT |
| | | | SEQ ID NO:1642 | SEQ ID NO:9654 | SEQ ID NO:17666 |
| **iPS:434437** | **21-225_70A12** | NA | CGGGCGAGTCAGGGTATTAGCAGCTGGTTAGCC | GCTGCATCCAGTTTGCAAAGT | CAACAGATTAACAGTTTCCCTCTCACT |
| | | | SEQ ID NO:1643 | SEQ ID NO:9655 | SEQ ID NO:17667 |
| | | AA | RASQGISSWLA | AASSLQS | QQINSFPLT |
| | | | SEQ ID NO:1644 | SEQ ID NO:9656 | SEQ ID NO:17668 |
| **iPS:434439** | **21-225_70E12** | NA | CGGGCAAGTCAGGGCATTAACAATAATTTAAAC | GCTGCATCCAGTTTGCAAAGT | CTACAGCATAATAGTTACCCGCTCACT |
| | | | SEQ ID NO:1645 | SEQ ID NO:9657 | SEQ ID NO:17669 |
| | | AA | RASQGINNNLN | AASSLQS | LQHNSYPLT |
| | | | SEQ ID NO:1646 | SEQ ID NO:9658 | SEQ ID NO:17670 |
| **iPS:434441** | **21-225_71A2** | NA | CGTGCAAGTCAGGGCATTAGAAATGATTTAGGA | ATTGCATTCAGATTGCAAATT | ATACACCATAATAGTTACCCGTGGACG |
| | | | SEQ ID NO:1647 | SEQ ID NO:9659 | SEQ ID NO:17671 |
| | | AA | RASQGIRNDLG | IAFRLQI | IHHNSYPWT |
| | | | SEQ ID NO:1648 | SEQ ID NO:9660 | SEQ ID NO:17672 |
| **iPS:434443** | **21-225_71G3** | NA | AAGTCCAGCCAGAGTGTTTTACACAGCTCCAACAATAACAACTACTTAGAT | TGGGCATCTACCCGGGAATTC | CAACAATATTATATTACTCCGTGCAGT |
| | | | SEQ ID NO:1649 | SEQ ID NO:9661 | SEQ ID NO:17673 |

FIGURE 49

| | | | | | |
|---|---|---|---|---|---|
| | | AA | KSSQSVLHSSNNNNYLD | WASTREF | QQYYITPCS |
| | | | SEQ ID NO:1650 | SEQ ID NO:9662 | SEQ ID NO:17674 |
| **iPS:434447** | **21-225_71B6** | NA | CGGGCAAGTCAGGGCATTAG AAATGATTTAGAT | GCTGCATCCAGTTTGCAA AGT | CTACAGCATAATAGTTACCC ATTCACT |
| | | | SEQ ID NO:1651 | SEQ ID NO:9663 | SEQ ID NO:17675 |
| | | AA | RASQGIRNDLD | AASSLQS | LQHNSYPFT |
| | | | SEQ ID NO:1652 | SEQ ID NO:9664 | SEQ ID NO:17676 |
| **iPS:434449** | **21-225_71H6** | NA | CGGGCAAGTCAGGGCATTAG AAATGTTTTAGGC | GCTGCATCCAGTTTACAA AGT | CTACAGTATAATAGTTACCC ATTCACT |
| | | | SEQ ID NO:1653 | SEQ ID NO:9665 | SEQ ID NO:17677 |
| | | AA | RASQGIRNVLG | AASSLQS | LQYNSYPFT |
| | | | SEQ ID NO:1654 | SEQ ID NO:9666 | SEQ ID NO:17678 |
| **iPS:434451** | **21-225_71B7** | NA | CGGGCGAGTCAGGGTATTAG CAGCTGGTTAGCC | GCTGCATCCAGTTTGCAA GGT | CAACAGACTAATAGTTTCCC TCTCACT |
| | | | SEQ ID NO:1655 | SEQ ID NO:9667 | SEQ ID NO:17679 |
| | | AA | RASQGISSWLA | AASSLQG | QQTNSFPLT |
| | | | SEQ ID NO:1656 | SEQ ID NO:9668 | SEQ ID NO:17680 |
| **iPS:434453** | **21-225_71B11** | NA | CGGGCAAGTCAGGGCATTAG AAATGATTTAGAT | GCTGCATCCAGTTTGCAA AGT | CTACAGCATAATACTTACCC ATTCACT |
| | | | SEQ ID NO:1657 | SEQ ID NO:9669 | SEQ ID NO:17681 |
| | | AA | RASQGIRNDLD | AASSLQS | LQHNTYPFT |
| | | | SEQ ID NO:1658 | SEQ ID NO:9670 | SEQ ID NO:17682 |
| **iPS:434455** | **21-225_72F5** | NA | CGGGCAAGTCAGAACATTAG CAGCTATTTAAAT | GCTGCATCCAGTTTGCAA AGT | CAACAGACTTACAGTACCCC CACC |
| | | | SEQ ID NO:1659 | SEQ ID NO:9671 | SEQ ID NO:17683 |
| | | AA | RASQNISSYLN | AASSLQS | QQTYSTPT |
| | | | SEQ ID NO:1660 | SEQ ID NO:9672 | SEQ ID NO:17684 |
| **iPS:434457** | **21-225_72G12** | NA | CGGGCGAGTCAGGGCATTAG CAGTTATTTAAAT | GGTGCTTCCAATTTGCAA TCT | CAACAGAATTACAATGCCCC GCTCACT |
| | | | SEQ ID NO:1661 | SEQ ID NO:9673 | SEQ ID NO:17685 |

**FIGURE 49**

| | | | | | |
|---|---|---|---|---|---|
| | | AA | RASQGISSYLN<br>SEQ ID NO:1662 | GASNLQS<br>SEQ ID NO:9674 | QQNYNAPLT<br>SEQ ID NO:17686 |
| **iPS:434459** | **21-225_71A7** | NA | CGGGCGAGTCAGGGTATTAG CAGCTGGTTAGCC<br>SEQ ID NO:1663 | GCTGCATCCAGTTTGCAA AGT<br>SEQ ID NO:9675 | CAACAGGTTAACAGTTTCCC TCTCACT<br>SEQ ID NO:17687 |
| | | AA | RASQGISSWLA<br>SEQ ID NO:1664 | AASSLQS<br>SEQ ID NO:9676 | QQVNSFPLT<br>SEQ ID NO:17688 |
| **iPS:434461** | **21-225_73A3** | NA | CGGGCGAGTCAGGGTATTAG CAACTGGTTAGCC<br>SEQ ID NO:1665 | GCTGCATCCAGTTTGCAA AGT<br>SEQ ID NO:9677 | CAACAGGTTAACAGTTTCCC TCTCACT<br>SEQ ID NO:17689 |
| | | AA | RASQGISNWLA<br>SEQ ID NO:1666 | AASSLQS<br>SEQ ID NO:9678 | QQVNSFPLT<br>SEQ ID NO:17690 |
| **iPS:434463** | **21-225_73A6** | NA | CGGGCAAGTCAGGGCATTAG AAATGATTTAGGC<br>SEQ ID NO:1667 | GCTGCATCCAATTTGCAA AGT<br>SEQ ID NO:9679 | CTACAGCATAATAGTTACCC ATTCACT<br>SEQ ID NO:17691 |
| | | AA | RASQGIRNDLG<br>SEQ ID NO:1668 | AASNLQS<br>SEQ ID NO:9680 | LQHNSYPFT<br>SEQ ID NO:17692 |
| **iPS:434467** | **21-225_73H8** | NA | CGGGCAAGTCAGGACATCA GAAATGATTTAGGC<br>SEQ ID NO:1669 | GCTGCATCCAGTTTGCAA AGT<br>SEQ ID NO:9681 | ATACAGCATAATAGTTACCC TCCGATCACC<br>SEQ ID NO:17693 |
| | | AA | RASQDIRNDLG<br>SEQ ID NO:1670 | AASSLQS<br>SEQ ID NO:9682 | IQHNSYPPIT<br>SEQ ID NO:17694 |
| **iPS:434469** | **21-225_73C9** | NA | CGGGCAAGTCAGGGCATTAG AAATGATTTAGGC<br>SEQ ID NO:1671 | GCTGCATCCAGTTTGCAA AGT<br>SEQ ID NO:9683 | CTACAGCATTATAGTTACCC TCGGACG<br>SEQ ID NO:17695 |
| | | AA | RASQGIRNDLG<br>SEQ ID NO:1672 | AASSLQS<br>SEQ ID NO:9684 | LQHYSYPRT<br>SEQ ID NO:17696 |
| **iPS:434471** | **21-225_75G3** | NA | AGGGCCCGTCAGAATGTTGA CAGCAGCTACTTAGCC<br>SEQ ID NO:1673 | GGTGCATCCAGCAGGGC CACT<br>SEQ ID NO:9685 | CAGCAGTATGAACGCTCACC GTGGACG<br>SEQ ID NO:17697 |

FIGURE 49

| | | | | | |
|---|---|---|---|---|---|
| | | AA | RARQNVDSSYLA | GASSRAT | QQYERSPWT |
| | | | SEQ ID NO:1674 | SEQ ID NO:9686 | SEQ ID NO:17698 |
| **iPS:434473** | **21-225_76D1** | NA | AGGGCCAGTCAGAATATTTA CAGCAACTACCTAGCC | GGTGCATCCAGCAGGGC CACT | CAGCAGTATGAAAGTTCACC GTGGACG |
| | | | SEQ ID NO:1675 | SEQ ID NO:9687 | SEQ ID NO:17699 |
| | | AA | RASQNIYSNYLA | GASSRAT | QQYESSPWT |
| | | | SEQ ID NO:1676 | SEQ ID NO:9688 | SEQ ID NO:17700 |
| **iPS:434475** | **21-225_74F9** | NA | AAGTCCAGCCAGAGTGTTTT ATACAGCTCCAACAATTACA ACTACTTAGCT | TGGGCATCTACCCGGGA ATCC | CAGCAATATTATAGTAGTCC TCCGACG |
| | | | SEQ ID NO:1677 | SEQ ID NO:9689 | SEQ ID NO:17701 |
| | | AA | KSSQSVLYSSNNYNYLA | WASTRES | QQYYSSPPT |
| | | | SEQ ID NO:1678 | SEQ ID NO:9690 | SEQ ID NO:17702 |
| **iPS:434477** | **21-225_74A6** | NA | AAGTCCAGCCAGAGTGTTTT ACACAGCTCCAACAATAACA ATTACTTAGCC | TGGGCATCAACCCGGGA ATCC | CAGCAATATTTTAGTACTCC GTGGACG |
| | | | SEQ ID NO:1679 | SEQ ID NO:9691 | SEQ ID NO:17703 |
| | | AA | KSSQSVLHSSNNNNYLA | WASTRES | QQYFSTPWT |
| | | | SEQ ID NO:1680 | SEQ ID NO:9692 | SEQ ID NO:17704 |
| **iPS:434479** | **21-225_76H1** | NA | AGGGCCAGTCAGAGTGTTAG CAGCAGCTACTTAGTC | GGTGCATCCACCAGGGC CACT | CAGCAGTATGGTTGCTCACC GCTCACT |
| | | | SEQ ID NO:1681 | SEQ ID NO:9693 | SEQ ID NO:17705 |
| | | AA | RASQSVSSSYLV | GASTRAT | QQYGCSPLT |
| | | | SEQ ID NO:1682 | SEQ ID NO:9694 | SEQ ID NO:17706 |
| **iPS:434481** | **21-225_74B10** | NA | AAGTCCAGCCAGAGTGTTTT ACACAGCTCCAACAATAAGA ACTACTTAACT | TGGGCATCTACTCGGGA ATCC | CAGCAATATTATAGTATTCC TCCGACG |
| | | | SEQ ID NO:1683 | SEQ ID NO:9695 | SEQ ID NO:17707 |
| | | AA | KSSQSVLHSSNNKNYLT | WASTRES | QQYYSIPPT |
| | | | SEQ ID NO:1684 | SEQ ID NO:9696 | SEQ ID NO:17708 |

FIGURE 49

| | | | | | |
|---|---|---|---|---|---|
| **iPS:434483** | **21-225_74C12** | NA | AAGTCCAGCCAGAGTGTTTTATACAGCTCCAACAATGCGAACTACTTAGCT | TGGGCATCTACCCGGGAATCC | CAGCAATATTATAGTACTCCGTGCAGT |
| | | | SEQ ID NO:1685 | SEQ ID NO:9697 | SEQ ID NO:17709 |
| | | AA | KSSQSVLYSSNNANYLA | WASTRES | QQYYSTPCS |
| | | | SEQ ID NO:1686 | SEQ ID NO:9698 | SEQ ID NO:17710 |
| **iPS:434485** | **21-225_76D2** | NA | AGGGCCAGTGTGAGTGTTGTCAACAGCTTAGCC | GGTGCATCCACCAGGGCCACT | CAGCAATATAATGACTGGCCGTGCAGT |
| | | | SEQ ID NO:1687 | SEQ ID NO:9699 | SEQ ID NO:17711 |
| | | AA | RASVSVVNSLA | GASTRAT | QQYNDWPCS |
| | | | SEQ ID NO:1688 | SEQ ID NO:9700 | SEQ ID NO:17712 |
| **iPS:434487** | **21-225_76G2** | NA | AAGTCCAGCCAGAGTGTTTTACACAGCTCCAACAATTACAACTACTTAGCT | TGGGCATCTACCCGGGAATCC | CAGCAATATTATAGTTCTCCTCCGACG |
| | | | SEQ ID NO:1689 | SEQ ID NO:9701 | SEQ ID NO:17713 |
| | | AA | KSSQSVLHSSNNYNYLA | WASTRES | QQYYSSPPT |
| | | | SEQ ID NO:1690 | SEQ ID NO:9702 | SEQ ID NO:17714 |
| **iPS:434489** | **21-225_74E4** | NA | CGGGCAAGTCAGGGCATTAGAAATGATTTAGGC | GCTGCATCCACTTTGCAAAGT | CTACAGCATAGTAATTACCCGCTCACT |
| | | | SEQ ID NO:1691 | SEQ ID NO:9703 | SEQ ID NO:17715 |
| | | AA | RASQGIRNDLG | AASTLQS | LQHSNYPLT |
| | | | SEQ ID NO:1692 | SEQ ID NO:9704 | SEQ ID NO:17716 |
| **iPS:434493** | **21-225_76F3** | NA | AAGTCCAGCCAGAGTGTTTTATTCAGCTCCAACAATTATAATTACTTAGCT | TGGGCATCTACCCGGGAATCC | CAGCAATATCATAGTTCTCCTCTGACG |
| | | | SEQ ID NO:1693 | SEQ ID NO:9705 | SEQ ID NO:17717 |
| | | AA | KSSQSVLFSSNNYNYLA | WASTRES | QQYHSSPLT |
| | | | SEQ ID NO:1694 | SEQ ID NO:9706 | SEQ ID NO:17718 |
| **iPS:434495** | | NA | AGGGCCAGTCAGAATATTTACAGCAGTTACTTAGCC | GGTGCATCCAGCAGGGCCACT | CAGCAGTATGAAAGCTCACCGTGGACC |

FIGURE 49

| | | | | | |
|---|---|---|---|---|---|
| | **21-225_74B2** | | SEQ ID NO:1695 | SEQ ID NO:9707 | SEQ ID NO:17719 |
| | | AA | RASQNIYSSYLA | GASSRAT | QQYESSPWT |
| | | | SEQ ID NO:1696 | SEQ ID NO:9708 | SEQ ID NO:17720 |
| **iPS:434497** | **21-225_76A4** | NA | AGGGCCAGTCAGAGTGTTTACAGCAGCTACTTAGCC | GGTGCATCCAGCCGGGCCACT | CAGCACTCTGATAACTCACCGTGGACG |
| | | | SEQ ID NO:1697 | SEQ ID NO:9709 | SEQ ID NO:17721 |
| | | AA | RASQSVYSSYLA | GASSRAT | QHSDNSPWT |
| | | | SEQ ID NO:1698 | SEQ ID NO:9710 | SEQ ID NO:17722 |
| **iPS:434501** | **21-225_76G4** | NA | AGGGCCAGTCAGAGTGTTTACAGCAGCTACTTAGCC | GGTGCATCCAGCCGGGCCACT | CAGCACTCTGATAACTCACCGTGGACG |
| | | | SEQ ID NO:1699 | SEQ ID NO:9711 | SEQ ID NO:17723 |
| | | AA | RASQSVYSSYLA | GASSRAT | QHSDNSPWT |
| | | | SEQ ID NO:1700 | SEQ ID NO:9712 | SEQ ID NO:17724 |
| **iPS:434503** | **21-225_74D7** | NA | CGGGCAAGTCAGGGCATTAGAAATGATTTAGGC | GCTGCATCCAGTTTGCAAAGT | CTTCAGCATAGTAATTACCCGCTCACT |
| | | | SEQ ID NO:1701 | SEQ ID NO:9713 | SEQ ID NO:17725 |
| | | AA | RASQGIRNDLG | AASSLQS | LQHSNYPLT |
| | | | SEQ ID NO:1702 | SEQ ID NO:9714 | SEQ ID NO:17726 |
| **iPS:434507** | **21-225_74C5** | NA | AGGGCCAGTCAGAGTGTTAACAGCAACTACTTAGCC | GGTGCATTCAGCAGGGCCACT | CAGCAGTATGAAAGCTCACCGTGGACG |
| | | | SEQ ID NO:1703 | SEQ ID NO:9715 | SEQ ID NO:17727 |
| | | AA | RASQSVNSNYLA | GAFSRAT | QQYESSPWT |
| | | | SEQ ID NO:1704 | SEQ ID NO:9716 | SEQ ID NO:17728 |
| **iPS:434509** | **21-225_76F5** | NA | AAGTCCAGCCAGAGTGTATTACACAGCTCCAACAGTTACAACTACTTAGCT | TGGACATCTACCCGGGAATCC | CAGCAATATTATAGTAGTCCTCCGACG |
| | | | SEQ ID NO:1705 | SEQ ID NO:9717 | SEQ ID NO:17729 |
| | | AA | KSSQSVLHSSNSYNYLA | WTSTRES | QQYYSSPPT |
| | | | SEQ ID NO:1706 | SEQ ID NO:9718 | SEQ ID NO:17730 |

# FIGURE 49

| | | | | | |
|---|---|---|---|---|---|
| **iPS:434511** | **21-225_74B11** | NA | AAGTCCAGCCAGAGTATTTTATACAACTCCAACAATAACAACTACTTAGCT | TGGGCATCTACCCGGGAATCC | CAGCAATATTATAGCACTCCTCCTACT |
| | | | SEQ ID NO:1707 | SEQ ID NO:9719 | SEQ ID NO:17731 |
| | | AA | KSSQSILYNSNNNNYLA | WASTRES | QQYYSTPPT |
| | | | SEQ ID NO:1708 | SEQ ID NO:9720 | SEQ ID NO:17732 |
| **iPS:434513** | **21-225_76A6** | NA | AGGGCCAGTCAGAGTGTTAGCAGCAGCTACTTAGCC | GGTGCATCCACCAGGGCCACT | CAGCAGTATGGTAACTCACCGCTCACT |
| | | | SEQ ID NO:1709 | SEQ ID NO:9721 | SEQ ID NO:17733 |
| | | AA | RASQSVSSSYLA | GASTRAT | QQYGNSPLT |
| | | | SEQ ID NO:1710 | SEQ ID NO:9722 | SEQ ID NO:17734 |
| **iPS:434515** | **21-225_74A5** | NA | AGGGCCAGTCAGAGTGTTAGCAGCAGCTACTTAGTC | GGTGCATCCACCAGGGCCACT | CAGCAGTATGGTTGCTCACCGCTCACT |
| | | | SEQ ID NO:1711 | SEQ ID NO:9723 | SEQ ID NO:17735 |
| | | AA | RASQSVSSSYLV | GASTRAT | QQYGCSPLT |
| | | | SEQ ID NO:1712 | SEQ ID NO:9724 | SEQ ID NO:17736 |
| **iPS:434517** | **21-225_76A7** | NA | AGGGCCAGTCCGAGTGTTGACAGCAGCTACTTAGCC | GGTGCATCCAGCAGGGCCCCT | CAGCAGTATGAAAGTTCACCGTGGACG |
| | | | SEQ ID NO:1713 | SEQ ID NO:9725 | SEQ ID NO:17737 |
| | | AA | RASPSVDSSYLA | GASSRAP | QQYESSPWT |
| | | | SEQ ID NO:1714 | SEQ ID NO:9726 | SEQ ID NO:17738 |
| **iPS:434519** | **21-225_74C7** | NA | AGGACCAGTCCGAATGTTGACAGCAGCTACTTAGCC | GGTGCATCCAGCAGGGCCACT | CAGCAGTATGAACGCTCACCGTGGACG |
| | | | SEQ ID NO:1715 | SEQ ID NO:9727 | SEQ ID NO:17739 |
| | | AA | RTSPNVDSSYLA | GASSRAT | QQYERSPWT |
| | | | SEQ ID NO:1716 | SEQ ID NO:9728 | SEQ ID NO:17740 |
| **iPS:434523** | **21-225_75C3** | NA | AGGGCCAGTCAGAGTGTTAGCAGCAGGTACTTAGCC | GGTGCATCCAGCAGGGCCACT | CAGCATTATGATAGCTCACCGTGGACG |
| | | | SEQ ID NO:1717 | SEQ ID NO:9729 | SEQ ID NO:17741 |
| | | AA | RASQSVSSRYLA | GASSRAT | QHYDSSPWT |

FIGURE 49

| | | | | | |
|---|---|---|---|---|---|
| | | | SEQ ID NO:1718 | SEQ ID NO:9730 | SEQ ID NO:17742 |
| **iPS:434525** | **21-225_76E8** | NA | AAGTCCAGCCAGACTGTTTTACACAGCTCCAACAATTATAACTACTTAGCT | TGGACATCTACCCGGGAATCC | CAGCAATATTTTAGTAGTCCTCTGACG |
| | | | SEQ ID NO:1719 | SEQ ID NO:9731 | SEQ ID NO:17743 |
| | | AA | KSSQTVLHSSNNYNYLA | WTSTRES | QQYFSSPLT |
| | | | SEQ ID NO:1720 | SEQ ID NO:9732 | SEQ ID NO:17744 |
| **iPS:434529** | **21-225_76B9** | NA | AGGGCCAGTCAGAGTGTTAGCAGCAGCTACTTAGTC | GGTGCATCCACCAGGGCCACT | CAGCAGTATGGTTGCTCACCGCTCACT |
| | | | SEQ ID NO:1721 | SEQ ID NO:9733 | SEQ ID NO:17745 |
| | | AA | RASQSVSSSYLV | GASTRAT | QQYGCSPLT |
| | | | SEQ ID NO:1722 | SEQ ID NO:9734 | SEQ ID NO:17746 |
| **iPS:434531** | **21-225_76C9** | NA | AGGGCCAGTCAGAGTGTTAGCAGCAGTTACTTATCC | GGTGCATCCAGCAGGGCCACT | CAGCAGTATGGTAGGTCACGGACG |
| | | | SEQ ID NO:1723 | SEQ ID NO:9735 | SEQ ID NO:17747 |
| | | AA | RASQSVSSSYLS | GASSRAT | QQYGRSRT |
| | | | SEQ ID NO:1724 | SEQ ID NO:9736 | SEQ ID NO:17748 |
| **iPS:434533** | **21-225_85F7** | NA | AGGGCCAGTCAGAATATTTACAGCAACTACTTAGCC | GGTGCATCCAGCAGGGCCACT | CAGCAGTATGAAAGCTCACCGTGGACC |
| | | | SEQ ID NO:1725 | SEQ ID NO:9737 | SEQ ID NO:17749 |
| | | AA | RASQNIYSNYLA | GASSRAT | QQYESSPWT |
| | | | SEQ ID NO:1726 | SEQ ID NO:9738 | SEQ ID NO:17750 |
| **iPS:434535** | **21-225_74C8** | NA | CGGGCGAGTCAGGGCATTGGCAAGTATTTAGCC | ACTACATCCAATTTACAAAGT | CAACAGTACAGTAATTACCCGCTCACT |
| | | | SEQ ID NO:1727 | SEQ ID NO:9739 | SEQ ID NO:17751 |
| | | AA | RASQGIGKYLA | TTSNLQS | QQYSNYPLT |
| | | | SEQ ID NO:1728 | SEQ ID NO:9740 | SEQ ID NO:17752 |
| **iPS:434537** | **21-225_74E11** | NA | AGGGCCAGTCTGAGTGTTGTCAACAGCTTAGCC | GGTGCATCCACCAGGGCCACT | CAGCAGTATAATGACTGGCCGTGCAGT |
| | | | SEQ ID NO:1729 | SEQ ID NO:9741 | SEQ ID NO:17753 |

**FIGURE 49**

| | | | | | |
|---|---|---|---|---|---|
| | | AA | RASLSVVNSLA | GASTRAT | QQYNDWPCS |
| | | | SEQ ID NO:1730 | SEQ ID NO:9742 | SEQ ID NO:17754 |
| **iPS:434539** | **21-225_74A2** | NA | AGGTCTAGTCAGAGCCTCCTGCATAGTAATGGACACAACTATTTGGAT | TTGGGTTCTAATCGGGCCTCC | ATGCAACCTCTACAAACTCCGTTCACT |
| | | | SEQ ID NO:1731 | SEQ ID NO:9743 | SEQ ID NO:17755 |
| | | AA | RSSQSLLHSNGHNYLD | LGSNRAS | MQPLQTPFT |
| | | | SEQ ID NO:1732 | SEQ ID NO:9744 | SEQ ID NO:17756 |
| **iPS:434547** | **21-225_74H5** | NA | AGGGCCAGTCAGAGTGTTAACAGCAACTACTTAGCC | GGTGCATCCAGCAGGGCCACT | CAGCAGTATGAAAGCTCGCCGTGGACG |
| | | | SEQ ID NO:1733 | SEQ ID NO:9745 | SEQ ID NO:17757 |
| | | AA | RASQSVNSNYLA | GASSRAT | QQYESSPWT |
| | | | SEQ ID NO:1734 | SEQ ID NO:9746 | SEQ ID NO:17758 |
| **iPS:434549** | **21-225_76E11** | NA | AAGTCCCGCCAGAGTGTTTTACACAGCTCCAACAATTACAACTACTTAGCT | TGGGCTTCTACCCGGGAATCC | CAGCAATATTATAGTACTCCTCCGACG |
| | | | SEQ ID NO:1735 | SEQ ID NO:9747 | SEQ ID NO:17759 |
| | | AA | KSRQSVLHSSNNYNYLA | WASTRES | QQYYSTPPT |
| | | | SEQ ID NO:1736 | SEQ ID NO:9748 | SEQ ID NO:17760 |
| **iPS:434551** | **21-225_75C4** | NA | AAGTCCAGCCAGAGTATTTTATACAGCTCCAACAATAATAACTACTTAGCT | TGGGCATCTACCCGGGAATCC | CAGCAATATTATATTACTCCTCCGACG |
| | | | SEQ ID NO:1737 | SEQ ID NO:9749 | SEQ ID NO:17761 |
| | | AA | KSSQSILYSSNNNNYLA | WASTRES | QQYYITPPT |
| | | | SEQ ID NO:1738 | SEQ ID NO:9750 | SEQ ID NO:17762 |
| **iPS:434559** | **21-225_74D11** | NA | AGGGCCAGTCAGAGTGTTTACAGCAGCTACTTAGCC | GGTGCATCCAGCCGGGCCACT | CAGCACTATGATAACTCACCGTGGACG |
| | | | SEQ ID NO:1739 | SEQ ID NO:9751 | SEQ ID NO:17763 |
| | | AA | RASQSVYSSYLA | GASSRAT | QHYDNSPWT |
| | | | SEQ ID NO:1740 | SEQ ID NO:9752 | SEQ ID NO:17764 |

FIGURE 49

| | | | | | |
|---|---|---|---|---|---|
| **iPS:434561** | **21-225_77G1** | NA | AGGGCCAGTCAGAGTGTTTACAGCAGCTACTTAGCC | GGTGCATCCAGCCGGGCCACT | CAGCACTATGATAACTCACCGTGGACG |
| | | | SEQ ID NO:1741 | SEQ ID NO:9753 | SEQ ID NO:17765 |
| | | AA | RASQSVYSSYLA | GASSRAT | QHYDNSPWT |
| | | | SEQ ID NO:1742 | SEQ ID NO:9754 | SEQ ID NO:17766 |
| **iPS:434563** | **21-225_75D8** | NA | AGGTCTAGTCAGAGCCTCCTGCATAGTAGTGGATACAACTATTTGGAT | TTGGGTTCTAATCGGGCCTCC | ATGCAAGCTCTACACCCTCCTCTCACT |
| | | | SEQ ID NO:1743 | SEQ ID NO:9755 | SEQ ID NO:17767 |
| | | AA | RSSQSLLHSSGYNYLD | LGSNRAS | MQALHPPLT |
| | | | SEQ ID NO:1744 | SEQ ID NO:9756 | SEQ ID NO:17768 |
| **iPS:434565** | **21-225_75B10** | NA | AGGGCCAGTCCGAGTGTTAACAGCTACTACTTAGCC | GGTGCAACCAGCAGGGCCACT | CAGCAGTATGAAGACTCACCGTGGACG |
| | | | SEQ ID NO:1745 | SEQ ID NO:9757 | SEQ ID NO:17769 |
| | | AA | RASPSVNSYYLA | GATSRAT | QQYEDSPWT |
| | | | SEQ ID NO:1746 | SEQ ID NO:9758 | SEQ ID NO:17770 |
| **iPS:434569** | **21-225_77H5** | NA | AGGGCCAGTCAGAGTGTTAGCAGCAGCTTAGCC | GGTGCATCCACCAGGGCCACT | CAGCAGTATAATGACTGGCCGTGCAGT |
| | | | SEQ ID NO:1747 | SEQ ID NO:9759 | SEQ ID NO:17771 |
| | | AA | RASQSVSSSLA | GASTRAT | QQYNDWPCS |
| | | | SEQ ID NO:1748 | SEQ ID NO:9760 | SEQ ID NO:17772 |
| **iPS:434571** | **21-225_74D2** | NA | AGGGCCAGTCAGAGTGTTGACAGCAACTACTTAGCC | GGTGCATCCAGCAGGGCCCCT | CAGCAGTATGAAAGCTCACCGTGGACG |
| | | | SEQ ID NO:1749 | SEQ ID NO:9761 | SEQ ID NO:17773 |
| | | AA | RASQSVDSNYLA | GASSRAP | QQYESSPWT |
| | | | SEQ ID NO:1750 | SEQ ID NO:9762 | SEQ ID NO:17774 |
| **iPS:434573** | **21-225_77E6** | NA | CGGGCGAGTCAGGGCATTAGCAAGTATTTAGCC | GCTGCATCCAGTTTGCAAGGT | CAACAGTACAGTAATTACCCGCTCACT |
| | | | SEQ ID NO:1751 | SEQ ID NO:9763 | SEQ ID NO:17775 |
| | | AA | RASQGISKYLA | AASSLQG | QQYSNYPLT |

FIGURE 49

| | | | | | |
|---|---|---|---|---|---|
| | | | SEQ ID NO:1752 | SEQ ID NO:9764 | SEQ ID NO:17776 |
| **iPS:434575** | **21-225_77C7** | NA | AAGTCCAGCCAGACTGTTTTACACAGCTCCAACAATTATAACTACTTAGCT | TGGACATCTACCCGGGAATCC | CAGCAATATTTTAGTAGTCCTCCGACG |
| | | | SEQ ID NO:1753 | SEQ ID NO:9765 | SEQ ID NO:17777 |
| | | AA | KSSQTVLHSSNNYNYLA | WTSTRES | QQYFSSPPT |
| | | | SEQ ID NO:1754 | SEQ ID NO:9766 | SEQ ID NO:17778 |
| **iPS:434579** | **21-225_77F7** | NA | AGGGCCAGTCAGAGTGTTTACAGCAGCTACTTAGCG | GGTGCATCCAGCCGGGCCACT | CAGCACTATGATAACTCACCGTGGACG |
| | | | SEQ ID NO:1755 | SEQ ID NO:9767 | SEQ ID NO:17779 |
| | | AA | RASQSVYSSYLA | GASSRAT | QHYDNSPWT |
| | | | SEQ ID NO:1756 | SEQ ID NO:9768 | SEQ ID NO:17780 |
| **iPS:434581** | **21-225_74B12** | NA | AGGGCCAGTCAGAGTGTTTACAGCAGCTACTTAGCC | GGTGCATCCAGCCGGTCCACT | CAGCACTATGATAACTCACCGTGGACG |
| | | | SEQ ID NO:1757 | SEQ ID NO:9769 | SEQ ID NO:17781 |
| | | AA | RASQSVYSSYLA | GASSRST | QHYDNSPWT |
| | | | SEQ ID NO:1758 | SEQ ID NO:9770 | SEQ ID NO:17782 |
| **iPS:434583** | **21-225_74B6** | NA | AGGGCCAGTCAGAGTGTTAGCAGCAGCTACTTAGCC | GGTGCATCCACCAGGGCCACT | CAGCAGTATGGTAACTCACCGCTCACT |
| | | | SEQ ID NO:1759 | SEQ ID NO:9771 | SEQ ID NO:17783 |
| | | AA | RASQSVSSSYLA | GASTRAT | QQYGNSPLT |
| | | | SEQ ID NO:1760 | SEQ ID NO:9772 | SEQ ID NO:17784 |
| **iPS:434585** | **21-225_75A12** | NA | AGGGCCAGTCAGAGTGTTAGCAGCAGGTACTTAGCC | GGTGCATCCAGCAGGGCCACT | CAGCATTATGATAGCTCACCGTGGACG |
| | | | SEQ ID NO:1761 | SEQ ID NO:9773 | SEQ ID NO:17785 |
| | | AA | RASQSVSSRYLA | GASSRAT | QHYDSSPWT |
| | | | SEQ ID NO:1762 | SEQ ID NO:9774 | SEQ ID NO:17786 |
| **iPS:434587** | **21-225_74G3** | NA | AGGGCCAGTCAGAGTGTTAGCAGCAGCTACTTAGTC | GGTGCATCCACCAGGGCCACT | CAGCAGTATGGTTGCTCACCGCTCACT |
| | | | SEQ ID NO:1763 | SEQ ID NO:9775 | SEQ ID NO:17787 |

**FIGURE 49**

| | | | | | |
|---|---|---|---|---|---|
| | | AA | RASQSVSSSYLV | GASTRAT | QQYGCSPLT |
| | | | SEQ ID NO:1764 | SEQ ID NO:9776 | SEQ ID NO:17788 |
| **iPS:434595** | **21-225_77A10** | NA | AGGGCCAGTCAGAGTGTTCA CAGCAGGTACTTAGCC | GGTGCATCCAGCAGGGC CACT | CAGCATTATGATAGCTCACC GTGGACG |
| | | | SEQ ID NO:1765 | SEQ ID NO:9777 | SEQ ID NO:17789 |
| | | AA | RASQSVHSRYLA | GASSRAT | QHYDSSPWT |
| | | | SEQ ID NO:1766 | SEQ ID NO:9778 | SEQ ID NO:17790 |
| **iPS:434597** | **21-225_77C10** | NA | AAGTCCAGCCAGAGTGTTTT ATACACCTCCAACAATAACA ACTACTTAGCT | TGGGCATCTACCCGGGA ATCC | CAGCACTATTATAATACTCC GTGGAAG |
| | | | SEQ ID NO:1767 | SEQ ID NO:9779 | SEQ ID NO:17791 |
| | | AA | KSSQSVLYTSNNNNYLA | WASTRES | QHYYNTPWK |
| | | | SEQ ID NO:1768 | SEQ ID NO:9780 | SEQ ID NO:17792 |
| **iPS:434603** | **21-225_77D11** | NA | AGGGCCAGTCAGAGTGTTAG CAGCAGCTACTTAGTC | GGTGCATCCACCAGGGC CACT | CAGCAGTATGGTTGCTCACC GCTCACT |
| | | | SEQ ID NO:1769 | SEQ ID NO:9781 | SEQ ID NO:17793 |
| | | AA | RASQSVSSSYLV | GASTRAT | QQYGCSPLT |
| | | | SEQ ID NO:1770 | SEQ ID NO:9782 | SEQ ID NO:17794 |
| **iPS:434611** | **21-225_77C12** | NA | AGGGCCAGGCAGAGTGTTG ACAGCAGTTATTTAGCC | GGTGCATCCAGCAGGGC CACT | CAGCAGTATGAAAGCTCACC GTGGACG |
| | | | SEQ ID NO:1771 | SEQ ID NO:9783 | SEQ ID NO:17795 |
| | | AA | RARQSVDSSYLA | GASSRAT | QQYESSPWT |
| | | | SEQ ID NO:1772 | SEQ ID NO:9784 | SEQ ID NO:17796 |
| **iPS:434613** | **21-225_77D12** | NA | AGGTCCAGCCAGAGTGTTTT ATACAGCTCCAACAATTATA ACTACTTAGCT | TGGGCATCTACCCGGGA TTCC | CAGCAATATTATACTACTCC GTGCAGT |
| | | | SEQ ID NO:1773 | SEQ ID NO:9785 | SEQ ID NO:17797 |
| | | AA | RSSQSVLYSSNNYNYLA | WASTRDS | QQYYTTPCS |
| | | | SEQ ID NO:1774 | SEQ ID NO:9786 | SEQ ID NO:17798 |

FIGURE 49

| | | | | | |
|---|---|---|---|---|---|
| **iPS:434615** | **21-225_76C5** | NA | CGGGCGAGTCAGGTCATTAGCAAGTATTTAGCC | GCTGCATCCAGTTTGCAAAGT | CAACAGTACAGTAATTACCCGCTCACT |
| | | | SEQ ID NO:1775 | SEQ ID NO:9787 | SEQ ID NO:17799 |
| | | AA | RASQVISKYLA | AASSLQS | QQYSNYPLT |
| | | | SEQ ID NO:1776 | SEQ ID NO:9788 | SEQ ID NO:17800 |
| **iPS:434617** | **21-225_74B8** | NA | AAGTCCAGCCAGAGTGTTTTACACAGCTCTAATAAAAAGAACTACTTAGCT | TGGGCATCTACCCGGGAATCC | CAGCAATATTATAGGACTCCGTGGACG |
| | | | SEQ ID NO:1777 | SEQ ID NO:9789 | SEQ ID NO:17801 |
| | | AA | KSSQSVLHSSNKKNYLA | WASTRES | QQYYRTPWT |
| | | | SEQ ID NO:1778 | SEQ ID NO:9790 | SEQ ID NO:17802 |
| **iPS:434619** | **21-225_78C1** | NA | AAGTCCAGCCAGAGTGTTTTATACACCTCCAACAATAACAACTACTTAGCT | TGGGCATCTACCCGGGAATCC | CAGCACTATTATAATACTCCGTGGAAG |
| | | | SEQ ID NO:1779 | SEQ ID NO:9791 | SEQ ID NO:17803 |
| | | AA | KSSQSVLYTSNNNNYLA | WASTRES | QHYYNTPWK |
| | | | SEQ ID NO:1780 | SEQ ID NO:9792 | SEQ ID NO:17804 |
| **iPS:434621** | **21-225_74D1** | NA | AGGGCCAGTCAGAGTGTTAGCAGAAATTTAGCC | GGTGCATCCATCAGGGCCACT | CAGCAGTATAATAACTGGCCTCCGCTCACT |
| | | | SEQ ID NO:1781 | SEQ ID NO:9793 | SEQ ID NO:17805 |
| | | AA | RASQSVSRNLA | GASIRAT | QQYNNWPPLT |
| | | | SEQ ID NO:1782 | SEQ ID NO:9794 | SEQ ID NO:17806 |
| **iPS:434629** | **21-225_74C3** | NA | AGGGCCAGTCAGAGTGTTGCCAGCAGCTTAGCC | GGTACATCCACCAGGGCCACT | CAGCAGTATAATGACTGGCCGTGCAGT |
| | | | SEQ ID NO:1783 | SEQ ID NO:9795 | SEQ ID NO:17807 |
| | | AA | RASQSVASSLA | GTSTRAT | QQYNDWPCS |
| | | | SEQ ID NO:1784 | SEQ ID NO:9796 | SEQ ID NO:17808 |
| **iPS:434633** | **21-225_74G8** | NA | AGGGCCAGTCAGAGTTTTAGCAGCGCCTACTTAGCC | GGTACTTCCAGCAGGGCCACT | CAACAGTATGGTAACTCAAGGACG |
| | | | SEQ ID NO:1785 | SEQ ID NO:9797 | SEQ ID NO:17809 |

**FIGURE 49**

| | | | | | |
|---|---|---|---|---|---|
| | | AA | RASQSFSSAYLA | GTSSRAT | QQYGNSRT |
| | | | SEQ ID NO:1786 | SEQ ID NO:9798 | SEQ ID NO:17810 |
| **iPS:434635** | **21-225_78E6** | NA | AAGTCCAGCCAGAGTGTTTT GTACAGCTCCAACAGTCACA ACTACTTAGCT | TGGGCATCTATCCGGGA ATCC | CAGCAATATTATAGTACTCC GTGCAGT |
| | | | SEQ ID NO:1787 | SEQ ID NO:9799 | SEQ ID NO:17811 |
| | | AA | KSSQSVLYSSNSHNYLA | WASIRES | QQYYSTPCS |
| | | | SEQ ID NO:1788 | SEQ ID NO:9800 | SEQ ID NO:17812 |
| **iPS:434637** | **21-225_78E7** | NA | AGGGCCAGTCAGAATGTTGA CAGCAACTACTTAGCC | GGTGCATCCAGCAGGGC CACT | CAGCAGTATGAACGCTCACC GTGGACG |
| | | | SEQ ID NO:1789 | SEQ ID NO:9801 | SEQ ID NO:17813 |
| | | AA | RASQNVDSNYLA | GASSRAT | QQYERSPWT |
| | | | SEQ ID NO:1790 | SEQ ID NO:9802 | SEQ ID NO:17814 |
| **iPS:434639** | **21-225_74B7** | NA | AAGTCCAGCCAGACTGTTTT ACACAGCTCCAACAATTATA ACTACTTAGCT | TGGACATCTACCCGGGA ATCC | CAGCAATATTTTAGTAGTCC TCCGACG |
| | | | SEQ ID NO:1791 | SEQ ID NO:9803 | SEQ ID NO:17815 |
| | | AA | KSSQTVLHSSNNYNYLA | WTSTRES | QQYFSSPPT |
| | | | SEQ ID NO:1792 | SEQ ID NO:9804 | SEQ ID NO:17816 |
| **iPS:434649** | **21-225_78E11** | NA | AAGTCCAGCCAGAGTGTTTT ATACAGCTTCAACAATTACA ACTACTTAGCT | TGGGCATCTACCCGGGA ATCC | CAGCAATATTATAGTAGTCC TCCGACG |
| | | | SEQ ID NO:1793 | SEQ ID NO:9805 | SEQ ID NO:17817 |
| | | AA | KSSQSVLYSFNNYNYLA | WASTRES | QQYYSSPPT |
| | | | SEQ ID NO:1794 | SEQ ID NO:9806 | SEQ ID NO:17818 |
| **iPS:434653** | **21-225_74B5** | NA | AAGTCCAGCCAGAGTGTTTT ATTCAGCTCCAACAATTATA ACTACTTAGCT | TGGGCATCTACCCGGGA ATCC | CAGCAATATTATAGTTCTCC TCCGACG |
| | | | SEQ ID NO:1795 | SEQ ID NO:9807 | SEQ ID NO:17819 |
| | | AA | KSSQSVLFSSNNYNYLA | WASTRES | QQYYSSPPT |

FIGURE 49

| | | | | | |
|---|---|---|---|---|---|
| | | | SEQ ID NO:1796 | SEQ ID NO:9808 | SEQ ID NO:17820 |
| **iPS:434655** | **21-225_78H12** | NA | AAGTCCAGCCAGACTGTTTTACACAGCTTCAACAATTATAACTACTTAGCT | TGGACATCTACCCGGGAATCC | CAGCAATATTTTAGTAGTCCTCCGACG |
| | | | SEQ ID NO:1797 | SEQ ID NO:9809 | SEQ ID NO:17821 |
| | | AA | KSSQTVLHSFNNYNYLA | WTSTRES | QQYFSSPPT |
| | | | SEQ ID NO:1798 | SEQ ID NO:9810 | SEQ ID NO:17822 |
| **iPS:434657** | **21-225_79G1** | NA | AGGGCCAGTCAGAGTGTTTACAGCAGCTACTTAGCC | GGTGCATCCAGCCGGTCCACT | CAGCACTATGATAACTCACCGTGGACG |
| | | | SEQ ID NO:1799 | SEQ ID NO:9811 | SEQ ID NO:17823 |
| | | AA | RASQSVYSSYLA | GASSRST | QHYDNSPWT |
| | | | SEQ ID NO:1800 | SEQ ID NO:9812 | SEQ ID NO:17824 |
| **iPS:434663** | **21-225_79F3** | NA | AGGGCCAGTCAGAGTGTTTACAGCAGCTACTTAGCC | GGTGCATCCAGCCGGTCCACT | CAGCACTATGATAACTCACCGTGGACG |
| | | | SEQ ID NO:1801 | SEQ ID NO:9813 | SEQ ID NO:17825 |
| | | AA | RASQSVYSSYLA | GASSRST | QHYDNSPWT |
| | | | SEQ ID NO:1802 | SEQ ID NO:9814 | SEQ ID NO:17826 |
| **iPS:434665** | **21-225_74G4** | NA | AAGTCCAGCCAGAGTGTTTTATACAGTTCCAACAATAATAACTACTTAGCT | TGGGCATCTACCCGGGAATCC | CAGCAATATTATAGTATTCCTCCGACG |
| | | | SEQ ID NO:1803 | SEQ ID NO:9815 | SEQ ID NO:17827 |
| | | AA | KSSQSVLYSSNNNNYLA | WASTRES | QQYYSIPPT |
| | | | SEQ ID NO:1804 | SEQ ID NO:9816 | SEQ ID NO:17828 |
| **iPS:434669** | **21-225_79F4** | NA | CGGGCGAGTCAGGGCATTAGCAAGTATTTAGCC | GCTGCATCCAGTTTGCAAGGT | CAACAGTACAGTAATTACCCACTCACT |
| | | | SEQ ID NO:1805 | SEQ ID NO:9817 | SEQ ID NO:17829 |
| | | AA | RASQGISKYLA | AASSLQG | QQYSNYPLT |
| | | | SEQ ID NO:1806 | SEQ ID NO:9818 | SEQ ID NO:17830 |
| **iPS:434671** | | NA | AGGGCCAGTCAGATTTTTAGCAGCAGCTACTTAGCC | GGTGCATCCAGCAGGGCCACT | CAGCAGTATGGTAGCTCACGGACG |

FIGURE 49

| | | | | | |
|---|---|---|---|---|---|
| | **21-225_74F4** | | SEQ ID NO:1807 | SEQ ID NO:9819 | SEQ ID NO:17831 |
| | | AA | RASQIFSSSYLA | GASSRAT | QQYGSSRT |
| | | | SEQ ID NO:1808 | SEQ ID NO:9820 | SEQ ID NO:17832 |
| **iPS:434673** | **21-225_74E3** | NA | AGGGCCAGTCTGAGTGTTGT CAACAGCTTAGCC | GGTGCATCCACCAGGGC CACT | CAGCAGTATAATGACTGGCC GTGCAGT |
| | | | SEQ ID NO:1809 | SEQ ID NO:9821 | SEQ ID NO:17833 |
| | | AA | RASLSVVNSLA | GASTRAT | QQYNDWPCS |
| | | | SEQ ID NO:1810 | SEQ ID NO:9822 | SEQ ID NO:17834 |
| **iPS:434675** | **21-225_79G6** | NA | ATGTCCAGCCAGAGTGTTTT ACACAGCTTCAACAATAAGA ACTACTTAACT | TGGGCATCTACTTGGGA ATCC | CAGCAATATTATAGTATTCC TCCGACG |
| | | | SEQ ID NO:1811 | SEQ ID NO:9823 | SEQ ID NO:17835 |
| | | AA | MSSQSVLHSFNNKNYLT | WASTWES | QQYYSIPPT |
| | | | SEQ ID NO:1812 | SEQ ID NO:9824 | SEQ ID NO:17836 |
| **iPS:434679** | **21-225_79G7** | NA | AAGTCCAGCCAGAGTGTTTT GTACAGCTCCAACAGTCACA ACTACTTAGCT | TGGGCATCTATCCGGGA ATCC | CAGCAATATTATAGTACTCC GTGCAGT |
| | | | SEQ ID NO:1813 | SEQ ID NO:9825 | SEQ ID NO:17837 |
| | | AA | KSSQSVLYSSNSHNYLA | WASIRES | QQYYSTPCS |
| | | | SEQ ID NO:1814 | SEQ ID NO:9826 | SEQ ID NO:17838 |
| **iPS:434685** | **21-225_79E9** | NA | AAGTCCAGCCAGAGTATTTT ATACAGCTCCAACAATAATA ACTACTTAGCT | TGGGCATCTACCCGGGA ATCC | CAGCAATATTATATTACTCC TCCGACG |
| | | | SEQ ID NO:1815 | SEQ ID NO:9827 | SEQ ID NO:17839 |
| | | AA | KSSQSILYSSNNNNYLA | WASTRES | QQYYITPPT |
| | | | SEQ ID NO:1816 | SEQ ID NO:9828 | SEQ ID NO:17840 |
| **iPS:434687** | **21-225_75A5** | NA | AGGGCCAGTCAGAGTGTTTA CAGCAGCTACTTAGCC | GGTGCATCCAGCCGGGC CACT | CAGCACTATGATAACTCACC GTGGACG |
| | | | SEQ ID NO:1817 | SEQ ID NO:9829 | SEQ ID NO:17841 |
| | | AA | RASQSVYSSYLA | GASSRAT | QHYDNSPWT |

**FIGURE 49**

| | | | | | |
|---|---|---|---|---|---|
| | | | SEQ ID NO:1818 | SEQ ID NO:9830 | SEQ ID NO:17842 |
| **iPS:434689** | **21-225_79G10** | NA | AAGTCCAGCCAGAGTGTTTTATTCAGCTCCAACAATTATAATTACTTAGCT | TGGGCATCTACCCGGGAATCC | CAGCAATATCATAGTTCTCCTCTGACG |
| | | | SEQ ID NO:1819 | SEQ ID NO:9831 | SEQ ID NO:17843 |
| | | AA | KSSQSVLFSSNNYNYLA | WASTRES | QQYHSSPLT |
| | | | SEQ ID NO:1820 | SEQ ID NO:9832 | SEQ ID NO:17844 |
| **iPS:434691** | **21-225_75G7** | NA | AGGGCCAGGCAGAGTGTTGACAGCAGTTATTTAGCC | GGTGCATCCAGCAGGGCCACT | CAGCAGTATGAAAGCTCACCGTGGACG |
| | | | SEQ ID NO:1821 | SEQ ID NO:9833 | SEQ ID NO:17845 |
| | | AA | RARQSVDSSYLA | GASSRAT | QQYESSPWT |
| | | | SEQ ID NO:1822 | SEQ ID NO:9834 | SEQ ID NO:17846 |
| **iPS:434693** | **21-225_79F11** | NA | AGGGCCAGTCAGAGTGTTTACAGCAGCTACTTAGCC | GGTGCATCCAGCCGGGCCACT | CAGCACTCTGATAACTCACCGTGGACG |
| | | | SEQ ID NO:1823 | SEQ ID NO:9835 | SEQ ID NO:17847 |
| | | AA | RASQSVYSSYLA | GASSRAT | QHSDNSPWT |
| | | | SEQ ID NO:1824 | SEQ ID NO:9836 | SEQ ID NO:17848 |
| **iPS:434697** | **21-225_79F12** | NA | AAGTCCAGCCAGAGTATTTTATACAGCTCCAACAATTACAACTACTTAGCT | TGGGCATCTACCCGGGAATCC | CAGCAATATTATAGTACTCCGTGGACG |
| | | | SEQ ID NO:1825 | SEQ ID NO:9837 | SEQ ID NO:17849 |
| | | AA | KSSQSILYSSNNYNYLA | WASTRES | QQYYSTPWT |
| | | | SEQ ID NO:1826 | SEQ ID NO:9838 | SEQ ID NO:17850 |
| **iPS:434699** | **21-225_79G12** | NA | AGGGCCAGTCAGAGTGTTTACAGCAGCTACTTAGCC | GGTGCATCCAGCCGGTCCACT | CAGCACTCTGATAACTCACCGTGGACG |
| | | | SEQ ID NO:1827 | SEQ ID NO:9839 | SEQ ID NO:17851 |
| | | AA | RASQSVYSSYLA | GASSRST | QHSDNSPWT |
| | | | SEQ ID NO:1828 | SEQ ID NO:9840 | SEQ ID NO:17852 |
| **iPS:434701** | | NA | AGGGCCAGTCAGAGTGTTTACAGCAGCTACTTAGCC | GGTGCATCCAGCCGGTCCACT | CAGCACTCTGATAACTCACCGTGGACG |

**FIGURE 49**

| | | | | | |
|---|---|---|---|---|---|
| | **21-225_80A1** | | SEQ ID NO:1829 | SEQ ID NO:9841 | SEQ ID NO:17853 |
| | | AA | RASQSVYSSYLA | GASSRST | QHSDNSPWT |
| | | | SEQ ID NO:1830 | SEQ ID NO:9842 | SEQ ID NO:17854 |
| **iPS:434703** | **21-225_80C1** | NA | AGGGCCAGTCAGAGTGTTTA CAGCAGCTACTTAGCC | GGTGCATCCAGCCGGTC CACT | CAGCACTCTGATAACTCACC GTGGACG |
| | | | SEQ ID NO:1831 | SEQ ID NO:9843 | SEQ ID NO:17855 |
| | | AA | RASQSVYSSYLA | GASSRST | QHSDNSPWT |
| | | | SEQ ID NO:1832 | SEQ ID NO:9844 | SEQ ID NO:17856 |
| **iPS:434705** | **21-225_80A2** | NA | AGGGCCAGTCAGAGTGTTAG CAGCAGCTACTTAGTC | GGTGCATCCACCAGGGC CACT | CAGCAGTATGGTTGCTCACC GCTCACT |
| | | | SEQ ID NO:1833 | SEQ ID NO:9845 | SEQ ID NO:17857 |
| | | AA | RASQSVSSSYLV | GASTRAT | QQYGCSPLT |
| | | | SEQ ID NO:1834 | SEQ ID NO:9846 | SEQ ID NO:17858 |
| **iPS:434707** | **21-225_80D3** | NA | AAGTCCAGCCAGAGTGTTTT ATACACCTCCAACAATAACA ACTACTTAGCT | TGGGCATCTACCCGGGA ATCC | CAGCACTACAATGAAACTCC AGGGAAG |
| | | | SEQ ID NO:1835 | SEQ ID NO:9847 | SEQ ID NO:17859 |
| | | AA | KSSQSVLYTSNNNNYLA | WASTRES | QHYNETPGK |
| | | | SEQ ID NO:1836 | SEQ ID NO:9848 | SEQ ID NO:17860 |
| **iPS:434709** | **21-225_80E3** | NA | AGGGCCAGTCAGAGTGTTTA CAGCAGCTACTTAGCC | GGTGCATCCAGCCGGTC CACT | CAGCATTCTGATAACTCACC GTGGACG |
| | | | SEQ ID NO:1837 | SEQ ID NO:9849 | SEQ ID NO:17861 |
| | | AA | RASQSVYSSYLA | GASSRST | QHSDNSPWT |
| | | | SEQ ID NO:1838 | SEQ ID NO:9850 | SEQ ID NO:17862 |
| **iPS:434711** | **21-225_80H3** | NA | AAGTCCAGCCAGAGTGTTTT ACACAGGTCCAACAATTACA ACTACTTAGCG | TGGGCATCTACCCGGGA ATCC | CAGCAATATTATAGTACTCC TCCGACG |
| | | | SEQ ID NO:1839 | SEQ ID NO:9851 | SEQ ID NO:17863 |
| | | AA | KSSQSVLHRSNNYNYLA | WASTRES | QQYYSTPPT |
| | | | SEQ ID NO:1840 | SEQ ID NO:9852 | SEQ ID NO:17864 |

FIGURE 49

| | | | | | |
|---|---|---|---|---|---|
| **iPS:434715** | **21-225_80D5** | NA | AGGGCCAGTCAGAATATTTACAGCAGCTACTTAGCC | GGTGCATCCAGCAGGGCCACT | CAGCAGTATGAAAGCTCACCGTGGACC |
| | | | SEQ ID NO:1841 | SEQ ID NO:9853 | SEQ ID NO:17865 |
| | | AA | RASQNIYSSYLA | GASSRAT | QQYESSPWT |
| | | | SEQ ID NO:1842 | SEQ ID NO:9854 | SEQ ID NO:17866 |
| **iPS:434717** | **21-225_80A6** | NA | AGGGCCAGTCAGAGTGTTGACAGCGGCTACTTAGCC | GGTGCATCCAGCAGGGCCCCT | CAGCAGTATGAAAGCTCACCGTGGACG |
| | | | SEQ ID NO:1843 | SEQ ID NO:9855 | SEQ ID NO:17867 |
| | | AA | RASQSVDSGYLA | GASSRAP | QQYESSPWT |
| | | | SEQ ID NO:1844 | SEQ ID NO:9856 | SEQ ID NO:17868 |
| **iPS:434725** | **21-225_80H7** | NA | AGGGCCAGTCAGAGTATTAACAGCAACTACTTAGCC | GGTGCATCCAGCAGGGCCACT | CAGCAGTATGAGAGCTCACCGTGGACG |
| | | | SEQ ID NO:1845 | SEQ ID NO:9857 | SEQ ID NO:17869 |
| | | AA | RASQSINSNYLA | GASSRAT | QQYESSPWT |
| | | | SEQ ID NO:1846 | SEQ ID NO:9858 | SEQ ID NO:17870 |
| **iPS:434729** | **21-225_80B12** | NA | AAGTCCAGACAGAGTGTTTTATACAGCTCCAACAATTACAACTACTTAACT | TGGGCATCTACCCGGGAATCC | CAGCAATATTATAGTTCTCCTCCTACT |
| | | | SEQ ID NO:1847 | SEQ ID NO:9859 | SEQ ID NO:17871 |
| | | AA | KSRQSVLYSSNNYNYLT | WASTRES | QQYYSSPPT |
| | | | SEQ ID NO:1848 | SEQ ID NO:9860 | SEQ ID NO:17872 |
| **iPS:434731** | **21-225_80E9** | NA | AAGTCCAGCCAGAGTGTTTTATACACCTCCAACAATAACAACTACTTAGCT | TGGGCATCTACCCGGGAATCC | CAGCAATATTATAATACTCCGTGGACG |
| | | | SEQ ID NO:1849 | SEQ ID NO:9861 | SEQ ID NO:17873 |
| | | AA | KSSQSVLYTSNNNNYLA | WASTRES | QQYYNTPWT |
| | | | SEQ ID NO:1850 | SEQ ID NO:9862 | SEQ ID NO:17874 |
| **iPS:434735** | **21-225_80B10** | NA | AGGGCCAGTCAGAGTGTTGACAGCAGCTACTTAGCC | GGTGCATCCAGCAGGGCCCCT | CAGCAGTATGAAAGCTCACCGTGGACG |
| | | | SEQ ID NO:1851 | SEQ ID NO:9863 | SEQ ID NO:17875 |

FIGURE 49

| | | | | | |
|---|---|---|---|---|---|
| | | AA | RASQSVDSSYLA | GASSRAP | QQYESSPWT |
| | | | SEQ ID NO:1852 | SEQ ID NO:9864 | SEQ ID NO:17876 |
| **iPS:434737** | **21-225_74G6** | NA | CGGGCGAGTCAGGGCATTGG CAAGTATTTAGCC | ACTACATCCAGTTTGCAA AGT | CAACAGTACAGTAATTACCC GCTCACT |
| | | | SEQ ID NO:1853 | SEQ ID NO:9865 | SEQ ID NO:17877 |
| | | AA | RASQGIGKYLA | TTSSLQS | QQYSNYPLT |
| | | | SEQ ID NO:1854 | SEQ ID NO:9866 | SEQ ID NO:17878 |
| **iPS:434741** | **21-225_80C11** | NA | CGGGCGAGTCAGGGCATTGG CAGGTATTTAGCC | ACTGCATCCAGTTTGCAA AGT | CAACAGTACAGTAATTACCC GCTCACT |
| | | | SEQ ID NO:1855 | SEQ ID NO:9867 | SEQ ID NO:17879 |
| | | AA | RASQGIGRYLA | TASSLQS | QQYSNYPLT |
| | | | SEQ ID NO:1856 | SEQ ID NO:9868 | SEQ ID NO:17880 |
| **iPS:434743** | **21-225_74A4** | NA | AGGGCCAGTCAGAGTGTTTA CAGCAGCTACTTAGCC | GGTGCATCCAGCCGGTC CACT | CAGCACTCTGATAACTCACC GTGGACG |
| | | | SEQ ID NO:1857 | SEQ ID NO:9869 | SEQ ID NO:17881 |
| | | AA | RASQSVYSSYLA | GASSRST | QHSDNSPWT |
| | | | SEQ ID NO:1858 | SEQ ID NO:9870 | SEQ ID NO:17882 |
| **iPS:434747** | **21-225_80C12** | NA | AGGGCCAGTCAGAGTGTTAG CAGCAGCTACTTAGCC | GGTGCATCCACCAGGGC CACT | CAGCAGTATGGTAACTCACC GCTCACT |
| | | | SEQ ID NO:1859 | SEQ ID NO:9871 | SEQ ID NO:17883 |
| | | AA | RASQSVSSSYLA | GASTRAT | QQYGNSPLT |
| | | | SEQ ID NO:1860 | SEQ ID NO:9872 | SEQ ID NO:17884 |
| **iPS:434751** | **21-225_80H12** | NA | AGGGCCAGTCAGAGTGTTTA CAGCAGCTACTTAGCC | GGTGCATCCAGCCGGTC CACT | CAGCACTCTGATAACTCACC GTGGACG |
| | | | SEQ ID NO:1861 | SEQ ID NO:9873 | SEQ ID NO:17885 |
| | | AA | RASQSVYSSYLA | GASSRST | QHSDNSPWT |
| | | | SEQ ID NO:1862 | SEQ ID NO:9874 | SEQ ID NO:17886 |
| **iPS:434759** | **21-225_81C5** | NA | AGGGCCAGTCAGAGTGTTTA CAGCAGCTACTTAGCC | GGTGCATCCAGCCGGTC CACT | CAGCATTCTGATAACTCACC GTGGACG |
| | | | SEQ ID NO:1863 | SEQ ID NO:9875 | SEQ ID NO:17887 |

**FIGURE 49**

| | | | | | |
|---|---|---|---|---|---|
| | | AA | RASQSVYSSYLA | GASSRST | QHSDNSPWT |
| | | | SEQ ID NO:1864 | SEQ ID NO:9876 | SEQ ID NO:17888 |
| **iPS:434761** | **21-225_81E5** | NA | AAGTCCAGCCAGAGTGTTTTATTCAGCTCCAACAATTATAATTACTTAGCT | TGGGCATCTACCCGGGAATCC | CAGCAATATTATAGTTCTCCTCTGACG |
| | | | SEQ ID NO:1865 | SEQ ID NO:9877 | SEQ ID NO:17889 |
| | | AA | KSSQSVLFSSNNYNYLA | WASTRES | QQYYSSPLT |
| | | | SEQ ID NO:1866 | SEQ ID NO:9878 | SEQ ID NO:17890 |
| **iPS:434771** | **21-225_81F9** | NA | AAGTCCAGCCAGAGTGTTTTATACACCTCCAACAATAACAACTACTTAGCT | TGGGCATCTACCCGGGAATCC | CAGCACTACAATGATACTCCAGGGAAG |
| | | | SEQ ID NO:1867 | SEQ ID NO:9879 | SEQ ID NO:17891 |
| | | AA | KSSQSVLYTSNNNNYLA | WASTRES | QHYNDTPGK |
| | | | SEQ ID NO:1868 | SEQ ID NO:9880 | SEQ ID NO:17892 |
| **iPS:434773** | **21-225_75D9** | NA | AGGGCCAGTCAGAGTGTTAGCAGCAGCTACTTAGCC | GGTGCATCCAGCAGGGCCACT | CAGCAGTATGAAAGCTCACCGTGGACG |
| | | | SEQ ID NO:1869 | SEQ ID NO:9881 | SEQ ID NO:17893 |
| | | AA | RASQSVSSSYLA | GASSRAT | QQYESSPWT |
| | | | SEQ ID NO:1870 | SEQ ID NO:9882 | SEQ ID NO:17894 |
| **iPS:434777** | **21-225_81C11** | NA | AGGGCCAGTCAGAGTGTTTACAGCAGCTACTTAGCC | GGTGCATCCAGCCGGTCCACT | CAGCATTCTGATAACTCACCGTGGACG |
| | | | SEQ ID NO:1871 | SEQ ID NO:9883 | SEQ ID NO:17895 |
| | | AA | RASQSVYSSYLA | GASSRST | QHSDNSPWT |
| | | | SEQ ID NO:1872 | SEQ ID NO:9884 | SEQ ID NO:17896 |
| **iPS:434793** | **21-225_82A5** | NA | AGGGCCAGTCAGAGTGTTAGCAGCAGCTACTTAGCC | GGTGCATCCACCAGGGCCACT | CAGCAGTATGGTAACTCACCGCTCACT |
| | | | SEQ ID NO:1873 | SEQ ID NO:9885 | SEQ ID NO:17897 |
| | | AA | RASQSVSSSYLA | GASTRAT | QQYGNSPLT |
| | | | SEQ ID NO:1874 | SEQ ID NO:9886 | SEQ ID NO:17898 |

FIGURE 49

| | | | | | |
|---|---|---|---|---|---|
| **iPS:434797** | **21-225_82G5** | NA | AGGGCCAGTGAGAGTGTTAGCAGCAGCTACTTAGTC | GGTGCATCCACCAGGGCCACT | CAGCAGTATGGTTGCTCACCGCTCACT |
| | | | SEQ ID NO:1875 | SEQ ID NO:9887 | SEQ ID NO:17899 |
| | | AA | RASESVSSSYLV | GASTRAT | QQYGCSPLT |
| | | | SEQ ID NO:1876 | SEQ ID NO:9888 | SEQ ID NO:17900 |
| **iPS:434805** | **21-225_82D9** | NA | AGGGCCAGTGAGAGTGTTAGCAGCAGCTACTTAGTC | GGTGCATCCACCAGGGCCACT | CAGCAGTATGGTTGCTCACCGCTCACT |
| | | | SEQ ID NO:1877 | SEQ ID NO:9889 | SEQ ID NO:17901 |
| | | AA | RASESVSSSYLV | GASTRAT | QQYGCSPLT |
| | | | SEQ ID NO:1878 | SEQ ID NO:9890 | SEQ ID NO:17902 |
| **iPS:434809** | **21-225_74F5** | NA | AGGGCCAGTCAGAGTGTTTACAGCAGCTACTTAGCC | GGTGCATCCAGCCGGTCCACT | CAGCACTCTGATAACTCACCGTGGACG |
| | | | SEQ ID NO:1879 | SEQ ID NO:9891 | SEQ ID NO:17903 |
| | | AA | RASQSVYSSYLA | GASSRST | QHSDNSPWT |
| | | | SEQ ID NO:1880 | SEQ ID NO:9892 | SEQ ID NO:17904 |
| **iPS:434813** | **21-225_82C12** | NA | AGGGCCAGTCAGAGTGTTAGCAGCAGCTACTTAGCC | GGTGCATCCACCAGGGCCTCT | CAGCAGTATGGTAACTCACCGCTCACT |
| | | | SEQ ID NO:1881 | SEQ ID NO:9893 | SEQ ID NO:17905 |
| | | AA | RASQSVSSSYLA | GASTRAS | QQYGNSPLT |
| | | | SEQ ID NO:1882 | SEQ ID NO:9894 | SEQ ID NO:17906 |
| **iPS:434815** | **21-225_74A11** | NA | CGGGCAAGTCAGGACATTAGAAATGATTTAGGC | GCTGCATCCAGTTTGCAAAGT | CTACAGCATAATGATTACCCATTCACT |
| | | | SEQ ID NO:1883 | SEQ ID NO:9895 | SEQ ID NO:17907 |
| | | AA | RASQDIRNDLG | AASSLQS | LQHNDYPFT |
| | | | SEQ ID NO:1884 | SEQ ID NO:9896 | SEQ ID NO:17908 |
| **iPS:434821** | **21-225_83G1** | NA | AGGGCCAGTCAGAGTGTTTACAGCAGCTACTTAGCC | GGTGCATCCAGCCGGTCCACG | CAGCACTCTGATAACTCACCGTGGACG |
| | | | SEQ ID NO:1885 | SEQ ID NO:9897 | SEQ ID NO:17909 |
| | | AA | RASQSVYSSYLA | GASSRST | QHSDNSPWT |
| | | | SEQ ID NO:1886 | SEQ ID NO:9898 | SEQ ID NO:17910 |

FIGURE 49

| | | | | | |
|---|---|---|---|---|---|
| **iPS:434825** | **21-225_83C2** | NA | AGGGCCAGTCAGAGTGTTAG CAGCAGCTACTTAGTC | GGTGCATCCACCAGGGC CACT | CAGCAGTATGGTTGCTCACC GCTCACT |
| | | | SEQ ID NO:1887 | SEQ ID NO:9899 | SEQ ID NO:17911 |
| | | AA | RASQSVSSSYLV | GASTRAT | QQYGCSPLT |
| | | | SEQ ID NO:1888 | SEQ ID NO:9900 | SEQ ID NO:17912 |
| **iPS:434827** | **21-225_83F3** | NA | AAGTCCAGCCAGAGTGTTTT ATACACCTCCAACAATAACA ACTACTTAGCT | TGGGCATCTACCCGGGA ATCC | CAGCACTATAATGATACTCC ATGGAAG |
| | | | SEQ ID NO:1889 | SEQ ID NO:9901 | SEQ ID NO:17913 |
| | | AA | KSSQSVLYTSNNNNYLA | WASTRES | QHYNDTPWK |
| | | | SEQ ID NO:1890 | SEQ ID NO:9902 | SEQ ID NO:17914 |
| **iPS:434829** | **21-225_83G3** | NA | AAGTCCAGCCAGAGTGTTTT ATACACCTCCAACAATAACA ACTACTTAGCT | TGGGCATCTACCCGGGA ATCC | CAGCACTATTATAATACTCC GTGGACG |
| | | | SEQ ID NO:1891 | SEQ ID NO:9903 | SEQ ID NO:17915 |
| | | AA | KSSQSVLYTSNNNNYLA | WASTRES | QHYYNTPWT |
| | | | SEQ ID NO:1892 | SEQ ID NO:9904 | SEQ ID NO:17916 |
| **iPS:434833** | **21-225_83C5** | NA | AGGGCCAGTGAGAGTGTTAG CAGCAGCTACTTAGTC | GGTGCATCCACCAGGGC CACT | CAGCAGTATGGTTGCTCACC GCTCACT |
| | | | SEQ ID NO:1893 | SEQ ID NO:9905 | SEQ ID NO:17917 |
| | | AA | RASESVSSSYLV | GASTRAT | QQYGCSPLT |
| | | | SEQ ID NO:1894 | SEQ ID NO:9906 | SEQ ID NO:17918 |
| **iPS:434835** | **21-225_83B6** | NA | AGGGCCAGTCAGAGTGTTGA CAGCGGCTACTTAGCC | GGTGCATCCAGCAGGAC CCCT | CAGCAGTATGAAAGCTCACC GTGGACG |
| | | | SEQ ID NO:1895 | SEQ ID NO:9907 | SEQ ID NO:17919 |
| | | AA | RASQSVDSGYLA | GASSRTP | QQYESSPWT |
| | | | SEQ ID NO:1896 | SEQ ID NO:9908 | SEQ ID NO:17920 |
| **iPS:434839** | **21-225_83B7** | NA | AGGGCCAGTCAGAGTGTTTA CAGCAGCTACTTAGCG | GGTGCATCCAGCCGGTC CACT | CAGCACTCTGATAACTCACC GTGGACG |
| | | | SEQ ID NO:1897 | SEQ ID NO:9909 | SEQ ID NO:17921 |

# FIGURE 49

| | | | | | |
|---|---|---|---|---|---|
| | | AA | RASQSVYSSYLA | GASSRST | QHSDNSPWT |
| | | | SEQ ID NO:1898 | SEQ ID NO:9910 | SEQ ID NO:17922 |
| **iPS:434841** | **21-225_83G7** | NA | AAGTCCAGCCAGACTGTTTTACACAGCTCCAACAATTATAACTACTTAGCT | TGGACATCTACCCGGGAATCC | CAGCAATATTTAGTAGTCCTCTGACG |
| | | | SEQ ID NO:1899 | SEQ ID NO:9911 | SEQ ID NO:17923 |
| | | AA | KSSQTVLHSSNNYNYLA | WTSTRES | QQYFSSPLT |
| | | | SEQ ID NO:1900 | SEQ ID NO:9912 | SEQ ID NO:17924 |
| **iPS:434849** | **21-225_83C10** | NA | AGGGCCAGTCCGAGTGTTCACAGCAACTACTTAGCC | GGTGCATCCAGCAGGGCCACT | CAGCAGTATGAAAGTTCACCGTGGACG |
| | | | SEQ ID NO:1901 | SEQ ID NO:9913 | SEQ ID NO:17925 |
| | | AA | RASPSVHSNYLA | GASSRAT | QQYESSPWT |
| | | | SEQ ID NO:1902 | SEQ ID NO:9914 | SEQ ID NO:17926 |
| **iPS:434851** | **21-225_75A6** | NA | AAGTCCAGACAGAGTGTTTTACACAGCTCCAACAATTACAACTACTTAGCT | TGGGCATCTACCCGGGAATCC | CAGCAATATTATAGTACTCCTCCTACT |
| | | | SEQ ID NO:1903 | SEQ ID NO:9915 | SEQ ID NO:17927 |
| | | AA | KSRQSVLHSSNNYNYLA | WASTRES | QQYYSTPPT |
| | | | SEQ ID NO:1904 | SEQ ID NO:9916 | SEQ ID NO:17928 |
| **iPS:434863** | **21-225_84G7** | NA | AAGTCCAGCCAGACTGTTTTACACAGCTCCAACAATTATAACTACTTAGCT | TGGACATCTACCCGGGAATCC | CAGCAATATTTAGTAGTCCTCCGACG |
| | | | SEQ ID NO:1905 | SEQ ID NO:9917 | SEQ ID NO:17929 |
| | | AA | KSSQTVLHSSNNYNYLA | WTSTRES | QQYFSSPPT |
| | | | SEQ ID NO:1906 | SEQ ID NO:9918 | SEQ ID NO:17930 |
| **iPS:434867** | **21-225_79A12** | NA | CGGGCGAGTCAGGTCATTAGCAAGTATTTAGCC | GCTGCATCCAGTTTGCAAAGT | CAACAGTACAGTAATTACCCGCTCACT |
| | | | SEQ ID NO:1907 | SEQ ID NO:9919 | SEQ ID NO:17931 |
| | | AA | RASQVISKYLA | AASSLQS | QQYSNYPLT |
| | | | SEQ ID NO:1908 | SEQ ID NO:9920 | SEQ ID NO:17932 |

FIGURE 49

| | | | | | |
|---|---|---|---|---|---|
| **iPS:434869** | **21-225_84E12** | NA | AGGGCCAGTCAGAGTATTAACAGCAACTACTTAGCC | GGTGCATCCAGCAGGGCCACT | CAGCAGTATGAGAGCTCACCGTGGACG |
| | | | SEQ ID NO:1909 | SEQ ID NO:9921 | SEQ ID NO:17933 |
| | | AA | RASQSINSNYLA | GASSRAT | QQYESSPWT |
| | | | SEQ ID NO:1910 | SEQ ID NO:9922 | SEQ ID NO:17934 |
| **iPS:434871** | **21-225_85H1** | NA | AGGGCCAGTCAGGATGTTATCACCTACTTAGCC | GGTGCATCCACCAGGGCCACT | CAGGAGTATAATGACTGGCCGTGCAGT |
| | | | SEQ ID NO:1911 | SEQ ID NO:9923 | SEQ ID NO:17935 |
| | | AA | RASQDVITYLA | GASTRAT | QEYNDWPCS |
| | | | SEQ ID NO:1912 | SEQ ID NO:9924 | SEQ ID NO:17936 |
| **iPS:434877** | **21-225_85H2** | NA | AAGTCCAGCCAGAGTGTTTTACACAGCTCTAATAAAAAGAACTACTTAGCT | TGGGCATCTACCCGGGAATCC | CAGCAATATTATAGGACTCCGTGGACG |
| | | | SEQ ID NO:1913 | SEQ ID NO:9925 | SEQ ID NO:17937 |
| | | AA | KSSQSVLHSSNKKNYLA | WASTRES | QQYYRTPWT |
| | | | SEQ ID NO:1914 | SEQ ID NO:9926 | SEQ ID NO:17938 |
| **iPS:434879** | **21-225_85A3** | NA | AGGGCCAGTCAGAGTGTTTACAGCAGCTACTTAGCC | GGTGCATCCAGCCGGGCCAGT | CAGCACTATGATAACTCACCGTGGACG |
| | | | SEQ ID NO:1915 | SEQ ID NO:9927 | SEQ ID NO:17939 |
| | | AA | RASQSVYSSYLA | GASSRAS | QHYDNSPWT |
| | | | SEQ ID NO:1916 | SEQ ID NO:9928 | SEQ ID NO:17940 |
| **iPS:434881** | **21-225_85B4** | NA | AGGGCCAGTCAGAGTGTTTACAGCAGCTACTTAGCC | GGTGCATCCAGCCGGGCCACT | CAGCACTATGATAACTCACCGTGGACG |
| | | | SEQ ID NO:1917 | SEQ ID NO:9929 | SEQ ID NO:17941 |
| | | AA | RASQSVYSSYLA | GASSRAT | QHYDNSPWT |
| | | | SEQ ID NO:1918 | SEQ ID NO:9930 | SEQ ID NO:17942 |
| **iPS:434883** | **21-225_85B5** | NA | AGGTCCAGTCAGAGTGTTAGCAGCAGCTACTTAGTC | GGTGCATCCACCAGGGCCACT | CAGCAGTATGGTTGCTCACCGCTCACT |
| | | | SEQ ID NO:1919 | SEQ ID NO:9931 | SEQ ID NO:17943 |
| | | AA | RSSQSVSSSYLV | GASTRAT | QQYGCSPLT |

**FIGURE 49**

| | | | | | |
|---|---|---|---|---|---|
| | | | SEQ ID NO:1920 | SEQ ID NO:9932 | SEQ ID NO:17944 |
| **iPS:434887** | **21-225_85D6** | NA | AGGGCCAGTCAGAGTGTTAG CAGCAGGTACTTAGCC | GGTGCATCCAGCAGGGC CACT | CAGCATTATGATAGCTCACC GTGGACG |
| | | | SEQ ID NO:1921 | SEQ ID NO:9933 | SEQ ID NO:17945 |
| | | AA | RASQSVSSRYLA | GASSRAT | QHYDSSPWT |
| | | | SEQ ID NO:1922 | SEQ ID NO:9934 | SEQ ID NO:17946 |
| **iPS:434891** | **21-225_85G6** | NA | AGGGCCAGTCCGAGTGTTGA CAGCAGCTACTTAGCC | GGTGCAGCCAGCAGGGC CCCT | CAGCAGTATGAAAGTTCACC GTGGACG |
| | | | SEQ ID NO:1923 | SEQ ID NO:9935 | SEQ ID NO:17947 |
| | | AA | RASPSVDSSYLA | GAASRAP | QQYESSPWT |
| | | | SEQ ID NO:1924 | SEQ ID NO:9936 | SEQ ID NO:17948 |
| **iPS:434895** | **21-225_74H7** | NA | AGGGCCAGTCAGAATATTTA CAGCAGCTACTTAGCC | GGTGCATCCAGCAGGGC CACT | CAGCAGTATGAAAGCTCACC GTGGACC |
| | | | SEQ ID NO:1925 | SEQ ID NO:9937 | SEQ ID NO:17949 |
| | | AA | RASQNIYSSYLA | GASSRAT | QQYESSPWT |
| | | | SEQ ID NO:1926 | SEQ ID NO:9938 | SEQ ID NO:17950 |
| **iPS:434899** | **21-225_85B9** | NA | AGGGCCAGTCAGAGTGTTAA CAGCAACTACTTAGCC | GGTGCATCCAGCAGGGC CACT | CAGCAGTATGAAAGCTCGCC GTGGACG |
| | | | SEQ ID NO:1927 | SEQ ID NO:9939 | SEQ ID NO:17951 |
| | | AA | RASQSVNSNYLA | GASSRAT | QQYESSPWT |
| | | | SEQ ID NO:1928 | SEQ ID NO:9940 | SEQ ID NO:17952 |
| **iPS:434901** | **21-225_85H9** | NA | AAGTCCAGCCAGAGTGTTTT ACACAGGTCCAACAATTACA ACTACTTAGCG | TGGGCATCTACCCGGGA ATCC | CAGCAATATTATAGTACTCC TCCGACG |
| | | | SEQ ID NO:1929 | SEQ ID NO:9941 | SEQ ID NO:17953 |
| | | AA | KSSQSVLHRSNNYNYLA | WASTRES | QQYYSTPPT |
| | | | SEQ ID NO:1930 | SEQ ID NO:9942 | SEQ ID NO:17954 |
| **iPS:434907** | **21-225_85G10** | NA | AGGGCCAGTCAGAGTGTTTG GAGCGGCTACTTAGCC | GGTGCATCTAGCAGGGC CACT | CAGCAGTATGAGAGTTCACC GTGGACG |
| | | | SEQ ID NO:1931 | SEQ ID NO:9943 | SEQ ID NO:17955 |

FIGURE 49

| | | | | | |
|---|---|---|---|---|---|
| | | AA | RASQSVWSGYLA | GASSRAT | QQYESSPWT |
| | | | SEQ ID NO:1932 | SEQ ID NO:9944 | SEQ ID NO:17956 |
| **iPS:434909** | **21-225_85C11** | NA | AAGTCCAGCCAGAGTGTTTTGTACAGCTCCAACAGTCACAACTTCTTAGCT | TGGGCATTTATCCGGGAATCC | CAGCAATATTATAGTACTCCGTGCAGT |
| | | | SEQ ID NO:1933 | SEQ ID NO:9945 | SEQ ID NO:17957 |
| | | AA | KSSQSVLYSSNSHNFLA | WAFIRES | QQYYSTPCS |
| | | | SEQ ID NO:1934 | SEQ ID NO:9946 | SEQ ID NO:17958 |
| **iPS:434911** | **21-225_85D11** | NA | AGGTCCAGTCAGAGTGTTAGCAGCAGCTACTTAGTC | GGTGCATCCACCAGGGCCACT | CAGCAGTATGGTTGCTCACCGCTCACT |
| | | | SEQ ID NO:1935 | SEQ ID NO:9947 | SEQ ID NO:17959 |
| | | AA | RSSQSVSSSYLV | GASTRAT | QQYGCSPLT |
| | | | SEQ ID NO:1936 | SEQ ID NO:9948 | SEQ ID NO:17960 |
| **iPS:434913** | **21-225_86C1** | NA | AGGGCCAGTCAGAGTGTTTACAGCAGCTACTTAGCC | GGTGCATCCAGCCGGGCCACT | CAGCACTATGATAACTCACCGTGGACG |
| | | | SEQ ID NO:1937 | SEQ ID NO:9949 | SEQ ID NO:17961 |
| | | AA | RASQSVYSSYLA | GASSRAT | QHYDNSPWT |
| | | | SEQ ID NO:1938 | SEQ ID NO:9950 | SEQ ID NO:17962 |
| **iPS:434921** | **21-225_86E4** | NA | AGGGCCAGTCAGAGTGTTTACAGCAGCTACTTAGCC | GGTGCATCCAGCCGGTCCACT | CAGCACTCTGATAACTCACCGTGGACG |
| | | | SEQ ID NO:1939 | SEQ ID NO:9951 | SEQ ID NO:17963 |
| | | AA | RASQSVYSSYLA | GASSRST | QHSDNSPWT |
| | | | SEQ ID NO:1940 | SEQ ID NO:9952 | SEQ ID NO:17964 |
| **iPS:434935** | **21-225_86E9** | NA | AAGTCCAGCCAGAGTGTTTTGCACAGATCCAACAATTATAATTACTTAGCT | TGGGCATCTACCCGGGAATCC | CAGCAATATCATAGTAGTCCACTGACG |
| | | | SEQ ID NO:1941 | SEQ ID NO:9953 | SEQ ID NO:17965 |
| | | AA | KSSQSVLHRSNNYNYLA | WASTRES | QQYHSSPLT |
| | | | SEQ ID NO:1942 | SEQ ID NO:9954 | SEQ ID NO:17966 |

## FIGURE 49

| | | | | | |
|---|---|---|---|---|---|
| **iPS:434939** | **21-225_86C11** | NA | AGGGCCAGTCAGAGTGTTTACAGCAGCTACTTAGCC | GGTGCATCCAGCCGGTCCACT | CAGCACTCTGATAACTCACCGTGGACG |
| | | | SEQ ID NO:1943 | SEQ ID NO:9955 | SEQ ID NO:17967 |
| | | AA | RASQSVYSSYLA | GASSRST | QHSDNSPWT |
| | | | SEQ ID NO:1944 | SEQ ID NO:9956 | SEQ ID NO:17968 |
| **iPS:434943** | **21-225_87H1** | NA | AGGGCCAGTCAGAGTGTTGACAGCAACTACTTAGCC | GGTGCATCTGCCAGGACCACT | CAGCAGTATGAAAGCTCACCGTGGACG |
| | | | SEQ ID NO:1945 | SEQ ID NO:9957 | SEQ ID NO:17969 |
| | | AA | RASQSVDSNYLA | GASARTT | QQYESSPWT |
| | | | SEQ ID NO:1946 | SEQ ID NO:9958 | SEQ ID NO:17970 |
| **iPS:434945** | **21-225_87E5** | NA | AGGGCCAGTCAGAGTGTTTACAGCAGCTACTTAGCC | GGTGCATCCAGCCGGTCCACT | CAGCATTCTGATAACTCACCGTGGACG |
| | | | SEQ ID NO:1947 | SEQ ID NO:9959 | SEQ ID NO:17971 |
| | | AA | RASQSVYSSYLA | GASSRST | QHSDNSPWT |
| | | | SEQ ID NO:1948 | SEQ ID NO:9960 | SEQ ID NO:17972 |
| **iPS:434947** | **21-225_87B7** | NA | CGGGCGAGTCAGGGCATTAGCAATTATTTAGCC | GCTGCATCCAGTTTGCACAGT | CTACTCTATCTTACTTACCCGCTCACC |
| | | | SEQ ID NO:1949 | SEQ ID NO:9961 | SEQ ID NO:17973 |
| | | AA | RASQGISNYLA | AASSLHS | LLYLTYPLT |
| | | | SEQ ID NO:1950 | SEQ ID NO:9962 | SEQ ID NO:17974 |
| **iPS:434955** | **21-225_87C9** | NA | AGGGCCAGTCAGAGTGTTTACAGCAGCTACTTAGCC | GGTGCATCCAGCCGGTCCACT | CAGCATTCTGATAACTCACCGTGGACG |
| | | | SEQ ID NO:1951 | SEQ ID NO:9963 | SEQ ID NO:17975 |
| | | AA | RASQSVYSSYLA | GASSRST | QHSDNSPWT |
| | | | SEQ ID NO:1952 | SEQ ID NO:9964 | SEQ ID NO:17976 |
| **iPS:434957** | **21-225_87A10** | NA | AGGGCCAGTCAGAGTGTTAGCAGCAGCTACTTAGCC | GGTGCATCCACCAGGGCCTCT | CAGCAGTATGGTAACTCACCGCTCACT |
| | | | SEQ ID NO:1953 | SEQ ID NO:9965 | SEQ ID NO:17977 |
| | | AA | RASQSVSSSYLA | GASTRAS | QQYGNSPLT |
| | | | SEQ ID NO:1954 | SEQ ID NO:9966 | SEQ ID NO:17978 |

FIGURE 49

| | | | | | |
|---|---|---|---|---|---|
| **iPS:434959** | **21-225_87E10** | NA | AAGTCCAGCCAGAGTGTTTTACACAGCTCCAACAATATGAACTACTTAGCT | TGGGCATCTACCCGGAAATCC | CAGCAATATTATAGTAGTCCGTGCAGT |
| | | | SEQ ID NO:1955 | SEQ ID NO:9967 | SEQ ID NO:17979 |
| | | AA | KSSQSVLHSSNNMNYLA | WASTRKS | QQYYSSPCS |
| | | | SEQ ID NO:1956 | SEQ ID NO:9968 | SEQ ID NO:17980 |
| **iPS:434961** | **21-225_87A12** | NA | AGGGCCAGTCAGAGTGTTTACAGCAGCTACTTAGCC | GGTGCATCCAGCCGGTCCACT | CAGCACTCTGATAACTCACCGTGGACG |
| | | | SEQ ID NO:1957 | SEQ ID NO:9969 | SEQ ID NO:17981 |
| | | AA | RASQSVYSSYLA | GASSRST | QHSDNSPWT |
| | | | SEQ ID NO:1958 | SEQ ID NO:9970 | SEQ ID NO:17982 |
| **iPS:434965** | **21-225_88A1** | NA | AAGTCCAGCCAGAGTGTTTTACACAGCTCCAACAATTACAACTACTTAACT | TGGGCATCTACCCGGAAATCC | CAGCAATATTATAGTTCTCCTCCGACG |
| | | | SEQ ID NO:1959 | SEQ ID NO:9971 | SEQ ID NO:17983 |
| | | AA | KSSQSVLHSSNNYNYLT | WASTRKS | QQYYSSPPT |
| | | | SEQ ID NO:1960 | SEQ ID NO:9972 | SEQ ID NO:17984 |
| **iPS:434969** | **21-225_88H1** | NA | AGGGCCAGTCAGAGTGTTTACAGCAGCTACTTAGCC | GGTGCATCCAGCCGGTCCACG | CAGCATTCTGATAACTCACCGTGGACG |
| | | | SEQ ID NO:1961 | SEQ ID NO:9973 | SEQ ID NO:17985 |
| | | AA | RASQSVYSSYLA | GASSRST | QHSDNSPWT |
| | | | SEQ ID NO:1962 | SEQ ID NO:9974 | SEQ ID NO:17986 |
| **iPS:434971** | **21-225_88G2** | NA | AAGTCCAGCCAGAGTGTTTTATACACCTCCAACAATAACAACTACTTAGCT | TGGGCATCTACCCGGGAATCC | CAGCAATATTATAATACTCCGTGGACG |
| | | | SEQ ID NO:1963 | SEQ ID NO:9975 | SEQ ID NO:17987 |
| | | AA | KSSQSVLYTSNNNNYLA | WASTRES | QQYYNTPWT |
| | | | SEQ ID NO:1964 | SEQ ID NO:9976 | SEQ ID NO:17988 |

FIGURE 49

| | | | | | |
|---|---|---|---|---|---|
| **iPS:434973** | **21-225_88B4** | NA | AAGTCCAGCCAGAGTGTTTTATACATCTCCAACAATAATAATTACTTAGCT | TGGGCATCTACCCGGGAATCC | CAGCAATATTATAGTACTCCTCCGACG |
| | | | SEQ ID NO:1965 | SEQ ID NO:9977 | SEQ ID NO:17989 |
| | | AA | KSSQSVLYISNNNNYLA | WASTRES | QQYYSTPPT |
| | | | SEQ ID NO:1966 | SEQ ID NO:9978 | SEQ ID NO:17990 |
| **iPS:434977** | **21-225_88A5** | NA | CGGGCAAGTCAGGGCATTAGAAATGATTTAGGC | GCTGCATCCAGTTTGCAAAGT | CTACAGCATAATGATTACCCATTCACT |
| | | | SEQ ID NO:1967 | SEQ ID NO:9979 | SEQ ID NO:17991 |
| | | AA | RASQGIRNDLG | AASSLQS | LQHNDYPFT |
| | | | SEQ ID NO:1968 | SEQ ID NO:9980 | SEQ ID NO:17992 |
| **iPS:434981** | **21-225_88E7** | NA | AGGGCCAGTCAGAGTGTTTACAGCAGCTACTTAGCC | GGTGCATCCAGCCGGTCCACT | CAGCACTCTGATAACTCACCGTGGACG |
| | | | SEQ ID NO:1969 | SEQ ID NO:9981 | SEQ ID NO:17993 |
| | | AA | RASQSVYSSYLA | GASSRST | QHSDNSPWT |
| | | | SEQ ID NO:1970 | SEQ ID NO:9982 | SEQ ID NO:17994 |
| **iPS:434983** | **21-225_88F7** | NA | AGGGCCAGTCAGAGTGTTTACAGCAGCTACTTAGCC | GGTGCATCCAGCCGGTCCACT | CAGCACTCTGATAACTCACCGTGGACG |
| | | | SEQ ID NO:1971 | SEQ ID NO:9983 | SEQ ID NO:17995 |
| | | AA | RASQSVYSSYLA | GASSRST | QHSDNSPWT |
| | | | SEQ ID NO:1972 | SEQ ID NO:9984 | SEQ ID NO:17996 |
| **iPS:434995** | **21-225_88G9** | NA | AGGGCCAGTCAGAGTGTTTACAGCAGCTACTTAGCC | GGTGCATCCAGCCGGTCCACT | CAGCACTCTGATAACTCACCGTGGACG |
| | | | SEQ ID NO:1973 | SEQ ID NO:9985 | SEQ ID NO:17997 |
| | | AA | RASQSVYSSYLA | GASSRST | QHSDNSPWT |
| | | | SEQ ID NO:1974 | SEQ ID NO:9986 | SEQ ID NO:17998 |
| **iPS:434997** | **21-225_88C10** | NA | AAGTCCAGCCAGAGTGTTTTACACAGCTCCAACAATTGGAATTACTTAGCT | TGGGCATTTACTCGGAAATCC | CAGCAATATTATAGAGCTCCTCCGACG |
| | | | SEQ ID NO:1975 | SEQ ID NO:9987 | SEQ ID NO:17999 |

FIGURE 49

| | | | | | |
|---|---|---|---|---|---|
| | | AA | KSSQSVLHSSNNWNYLA | WAFTRKS | QQYYRAPPT |
| | | | SEQ ID NO:1976 | SEQ ID NO:9988 | SEQ ID NO:18000 |
| **iPS:434999** | **21-225_75A8** | NA | AGGGCCAGTCAGAGTGTTTA CAGCAGCTACTTAGCC | GGTGCATCCAGCCGGGC CACT | CAGCACTCTGATAACTCACC GTGGACG |
| | | | SEQ ID NO:1977 | SEQ ID NO:9989 | SEQ ID NO:18001 |
| | | AA | RASQSVYSSYLA | GASSRAT | QHSDNSPWT |
| | | | SEQ ID NO:1978 | SEQ ID NO:9990 | SEQ ID NO:18002 |
| **iPS:435009** | **21-225_89G4** | NA | AGGTCTAGTCAGAGCCTCCT GCATAGTAGTGGATACAACT ATTTGGAT | TTGGGTTCTAATCGGGCC TCC | ATGCAAGCTCTACATATTCC TCTCACT |
| | | | SEQ ID NO:1979 | SEQ ID NO:9991 | SEQ ID NO:18003 |
| | | AA | RSSQSLLHSSGYNYLD | LGSNRAS | MQALHIPLT |
| | | | SEQ ID NO:1980 | SEQ ID NO:9992 | SEQ ID NO:18004 |
| **iPS:435013** | **21-225_89D5** | NA | AGGGCCAGTCAGAGTGTTTA CAGCAGCTACTTAGCC | GGTGCATCCAGCCGGTC CACT | CAGCACTATGATAACTCACC GTGGACG |
| | | | SEQ ID NO:1981 | SEQ ID NO:9993 | SEQ ID NO:18005 |
| | | AA | RASQSVYSSYLA | GASSRST | QHYDNSPWT |
| | | | SEQ ID NO:1982 | SEQ ID NO:9994 | SEQ ID NO:18006 |
| **iPS:435015** | **21-225_89H5** | NA | AGGGCCAGTCAGAGTGTTAA CAGCAACTACTTAGCC | GGTGCATTCAGCAGGGC CACT | CAGCAGTATGAAAGCTCAGT GTGGACG |
| | | | SEQ ID NO:1983 | SEQ ID NO:9995 | SEQ ID NO:18007 |
| | | AA | RASQSVNSNYLA | GAFSRAT | QQYESSVWT |
| | | | SEQ ID NO:1984 | SEQ ID NO:9996 | SEQ ID NO:18008 |
| **iPS:435025** | **21-225_89E10** | NA | AGGGCCAGTCAGAGTGTTTA CAGCAGCTACTTAGCC | GGTGCATCCAGCCGGTC CACT | CAGCATTCTGATAACTCTCC GTGGACG |
| | | | SEQ ID NO:1985 | SEQ ID NO:9997 | SEQ ID NO:18009 |
| | | AA | RASQSVYSSYLA | GASSRST | QHSDNSPWT |
| | | | SEQ ID NO:1986 | SEQ ID NO:9998 | SEQ ID NO:18010 |
| **iPS:435029** | | NA | AGGGCCAGTCAGAGTGTTGA CAGCAACTTCTTAGCC | GGTGCATCTGCCAGGAC CACT | CAGCAGTATGAAATCTCACC GTGGACG |

FIGURE 49

| | | | | | |
|---|---|---|---|---|---|
| | **21-225_89A11** | | SEQ ID NO:1987 | SEQ ID NO:9999 | SEQ ID NO:18011 |
| | | AA | RASQSVDSNFLA | GASARTT | QQYEISPWT |
| | | | SEQ ID NO:1988 | SEQ ID NO:10000 | SEQ ID NO:18012 |
| **iPS:435039** | **21-225_90G4** | NA | AGGGCCAGTCAGAGTGTTTA CAGCAGCTACTTAGCC | GGTGCATCCAGCCGGTC CACT | CAGCACTCTGATAACTCACC GTGGACG |
| | | | SEQ ID NO:1989 | SEQ ID NO:10001 | SEQ ID NO:18013 |
| | | AA | RASQSVYSSYLA | GASSRST | QHSDNSPWT |
| | | | SEQ ID NO:1990 | SEQ ID NO:10002 | SEQ ID NO:18014 |
| **iPS:435041** | **21-225_90A5** | NA | AGGGCCAGTCAGAGTGTTTA CAGCAGCTACTTAGCC | GGTGCATCCAGCCGGGC CACT | CAGCACTATGATAACTCACC GTGGACG |
| | | | SEQ ID NO:1991 | SEQ ID NO:10003 | SEQ ID NO:18015 |
| | | AA | RASQSVYSSYLA | GASSRAT | QHYDNSPWT |
| | | | SEQ ID NO:1992 | SEQ ID NO:10004 | SEQ ID NO:18016 |
| **iPS:435043** | **21-225_90G5** | NA | AGGGCCAGTCAGAGTGTTTA CAGCAGCTACTTAGCC | GGTGCATCCAGCCGGGC CACT | CAGCACTATGATAACTCACC GTGGACG |
| | | | SEQ ID NO:1993 | SEQ ID NO:10005 | SEQ ID NO:18017 |
| | | AA | RASQSVYSSYLA | GASSRAT | QHYDNSPWT |
| | | | SEQ ID NO:1994 | SEQ ID NO:10006 | SEQ ID NO:18018 |
| **iPS:435045** | **21-225_90H5** | NA | CGGGCAAGTCAGGGCATTAG AAATGATTTAGGC | ATTGCATCCAGTTTGCAA AGT | CTACAGCATAATAGTTACCC GATCACC |
| | | | SEQ ID NO:1995 | SEQ ID NO:10007 | SEQ ID NO:18019 |
| | | AA | RASQGIRNDLG | IASSLQS | LQHNSYPIT |
| | | | SEQ ID NO:1996 | SEQ ID NO:10008 | SEQ ID NO:18020 |
| **iPS:435051** | **21-225_90D9** | NA | AAGTCCAGCCAGACTGTTTT ACACAGCTCCAACAATTATA ACTACTTAGCT | TGGACATCTACCCGGGA ATCC | CAGCAATATCTTAGTAGTCC TCTGACG |
| | | | SEQ ID NO:1997 | SEQ ID NO:10009 | SEQ ID NO:18021 |
| | | AA | KSSQTVLHSSNNYNYLA | WTSTRES | QQYLSSPLT |
| | | | SEQ ID NO:1998 | SEQ ID NO:10010 | SEQ ID NO:18022 |

FIGURE 49

| | | | | | |
|---|---|---|---|---|---|
| **iPS:435053** | **21-225_75F9** | NA | AAGTCCAGCCAGAGTGTTTTACACAACTCCAACAATAATAACTACTTGGCT | TGGGCATCTACGCGGGAGTCC | CAACAATATTATAGTAGTCCTCCGACG |
| | | | SEQ ID NO:1999 | SEQ ID NO:10011 | SEQ ID NO:18023 |
| | | AA | KSSQSVLHNSNNNNYLA | WASTRES | QQYYSSPPT |
| | | | SEQ ID NO:2000 | SEQ ID NO:10012 | SEQ ID NO:18024 |
| **iPS:435055** | **21-225_90F10** | NA | AGGGCCAGTCAGAGTGTTTACAGCAGCTACTTAGCC | GGTGCATCCAGCCGGTCCACT | CAGCACTATGATAACTCACCGTGGACG |
| | | | SEQ ID NO:2001 | SEQ ID NO:10013 | SEQ ID NO:18025 |
| | | AA | RASQSVYSSYLA | GASSRST | QHYDNSPWT |
| | | | SEQ ID NO:2002 | SEQ ID NO:10014 | SEQ ID NO:18026 |
| **iPS:435059** | **21-225_90C11** | NA | AGGTATAGTCAGAGCCTCGTGCATAGTAGTGGATACAACTATTTGGAT | TTGGGTTCTAATCGGGCCTCC | ATGCAAGCTCTACACCCTCCTCTCACT |
| | | | SEQ ID NO:2003 | SEQ ID NO:10015 | SEQ ID NO:18027 |
| | | AA | RYSQSLVHSSGYNYLD | LGSNRAS | MQALHPPLT |
| | | | SEQ ID NO:2004 | SEQ ID NO:10016 | SEQ ID NO:18028 |
| **iPS:435071** | **21-225_91F1** | NA | AAGTCCAGCCAGAGTGTTTTATACACCTCCAACAATAACAACTACTTAGCT | TGGGCATCTACCCGGGAATCC | CAGCACTATTATAATACTCCGTGGAAG |
| | | | SEQ ID NO:2005 | SEQ ID NO:10017 | SEQ ID NO:18029 |
| | | AA | KSSQSVLYTSNNNNYLA | WASTRES | QHYYNTPWK |
| | | | SEQ ID NO:2006 | SEQ ID NO:10018 | SEQ ID NO:18030 |
| **iPS:435073** | **21-225_91B2** | NA | AGGGCCAGTCAGAGTGTTTACAGCAGCTACTTAGCC | GGTGCATCCAGCCGGTCCACT | CAGCACTATGATAACTCACCGTGGACG |
| | | | SEQ ID NO:2007 | SEQ ID NO:10019 | SEQ ID NO:18031 |
| | | AA | RASQSVYSSYLA | GASSRST | QHYDNSPWT |
| | | | SEQ ID NO:2008 | SEQ ID NO:10020 | SEQ ID NO:18032 |
| **iPS:435075** | | NA | AGGGCCAGTCAGAGTGTTTACAGCAGCTACTTAGCC | GGTGCATCCAGCCGGTCCACT | CAGCACTATGATAACTCACCGTGGACG |

FIGURE 49

| | | | | | |
|---|---|---|---|---|---|
| | **21-225_91B3** | | SEQ ID NO:2009 | SEQ ID NO:10021 | SEQ ID NO:18033 |
| | | AA | RASQSVYSSYLA | GASSRST | QHYDNSPWT |
| | | | SEQ ID NO:2010 | SEQ ID NO:10022 | SEQ ID NO:18034 |
| **iPS:435077** | **21-225_91F3** | NA | AGGGCCAGTCAGAGTGTTTA CAGCAGCTACTTAGCC | GGTGCATCCAGCCGGTC CACT | CAGCATTCTGATAACTCACC GTGGACG |
| | | | SEQ ID NO:2011 | SEQ ID NO:10023 | SEQ ID NO:18035 |
| | | AA | RASQSVYSSYLA | GASSRST | QHSDNSPWT |
| | | | SEQ ID NO:2012 | SEQ ID NO:10024 | SEQ ID NO:18036 |
| **iPS:435079** | **21-225_91B4** | NA | AGGGCCAGTCAGAGTGTTTA CAGCAGCTACTTAGCC | GGTGCATCCAGCCGGTC CACT | CAGCACTATGATAACTCACC GTGGACG |
| | | | SEQ ID NO:2013 | SEQ ID NO:10025 | SEQ ID NO:18037 |
| | | AA | RASQSVYSSYLA | GASSRST | QHYDNSPWT |
| | | | SEQ ID NO:2014 | SEQ ID NO:10026 | SEQ ID NO:18038 |
| **iPS:435087** | **21-225_91G8** | NA | AAGTCCAGCCAGAGTGTTTT ATACACCTCCAACAATAACA ACTACTTAGCT | TGGGCATCTACCCGGGA ATCC | CAGCAATATTATACTACTCC GTGGACG |
| | | | SEQ ID NO:2015 | SEQ ID NO:10027 | SEQ ID NO:18039 |
| | | AA | KSSQSVLYTSNNNNYLA | WASTRES | QQYYTTPWT |
| | | | SEQ ID NO:2016 | SEQ ID NO:10028 | SEQ ID NO:18040 |
| **iPS:435089** | **21-225_91E9** | NA | AGGGCCAGTCAGAGTGTTTA CAGCAGCTACTTAGCG | GGTGCATCCAGCCGGGC CACG | CAGCACTCTGATAACTCACC GTGGACG |
| | | | SEQ ID NO:2017 | SEQ ID NO:10029 | SEQ ID NO:18041 |
| | | AA | RASQSVYSSYLA | GASSRAT | QHSDNSPWT |
| | | | SEQ ID NO:2018 | SEQ ID NO:10030 | SEQ ID NO:18042 |
| **iPS:435097** | **21-225_92B1** | NA | AGGGCCAGTCAGAGTGTTGG CAGCAACTACTTAGCC | GGTGCATCCAGCAGGGC CACT | CAGCAGTATGAAAGTTCACC GTGGACG |
| | | | SEQ ID NO:2019 | SEQ ID NO:10031 | SEQ ID NO:18043 |
| | | AA | RASQSVGSNYLA | GASSRAT | QQYESSPWT |
| | | | SEQ ID NO:2020 | SEQ ID NO:10032 | SEQ ID NO:18044 |

FIGURE 49

| | | | | | |
|---|---|---|---|---|---|
| **iPS:435103** | **21-225_92B2** | NA | AGGTCTAGTCAGAGCCTCGTGCATAGTAGTGGATACAACTATTTGGAT | TTGGGTTCTAATCGGGCCTCC | ATGCAAGCTCTACATATTCCTCTCACT |
| | | | SEQ ID NO:2021 | SEQ ID NO:10033 | SEQ ID NO:18045 |
| | | AA | RSSQSLVHSSGYNYLD | LGSNRAS | MQALHIPLT |
| | | | SEQ ID NO:2022 | SEQ ID NO:10034 | SEQ ID NO:18046 |
| **iPS:435109** | **21-225_92H5** | NA | CGGGCCAGTCAGGATGTTATCACCTACTTAGCC | GGTGCATCCACCAGGGCCACT | CAGGAGTATAATGACTGGCCGTGCAGT |
| | | | SEQ ID NO:2023 | SEQ ID NO:10035 | SEQ ID NO:18047 |
| | | AA | RASQDVITYLA | GASTRAT | QEYNDWPCS |
| | | | SEQ ID NO:2024 | SEQ ID NO:10036 | SEQ ID NO:18048 |
| **iPS:435111** | **21-225_92D6** | NA | AGGGCCAGTCAGAGTGTTTACAGCAGCTACTTAGCC | GGTGCATCCAGCCGGTCCACT | CAGCATTATGATAACTCTCCGTGGACG |
| | | | SEQ ID NO:2025 | SEQ ID NO:10037 | SEQ ID NO:18049 |
| | | AA | RASQSVYSSYLA | GASSRST | QHYDNSPWT |
| | | | SEQ ID NO:2026 | SEQ ID NO:10038 | SEQ ID NO:18050 |
| **iPS:435113** | **21-225_92E6** | NA | AAGTCCAGTCAGAATATTTTATCCAGCTCCAACAATAAGAACTACTTAACT | TGGACATCTACCCGGGAATCC | CAGCAATATTTTAGTGTTCCTCCGACG |
| | | | SEQ ID NO:2027 | SEQ ID NO:10039 | SEQ ID NO:18051 |
| | | AA | KSSQNILSSSNNKNYLT | WTSTRES | QQYFSVPPT |
| | | | SEQ ID NO:2028 | SEQ ID NO:10040 | SEQ ID NO:18052 |
| **iPS:435115** | **21-225_77C5** | NA | AGGGCCAGTCAGAGTGTTTACAGCAGCTACTTAGCC | GGTGCATCCAGCCGGTCCACT | CAGCACTATGATAACTCACCGTGGACG |
| | | | SEQ ID NO:2029 | SEQ ID NO:10041 | SEQ ID NO:18053 |
| | | AA | RASQSVYSSYLA | GASSRST | QHYDNSPWT |
| | | | SEQ ID NO:2030 | SEQ ID NO:10042 | SEQ ID NO:18054 |
| **iPS:435167** | **21 225 92F12** | NA | AAGTCCAGCCAGAGTGTTTTACACAGGTCCAACAATTACAACTACTTAGCG | TGGGCATCTACCCGGGAATCC | CAGCAATATTATAGTACTCCTCCGACG |

FIGURE 49

| | | | | | |
|---|---|---|---|---|---|
| | 21-225_92F12 | | SEQ ID NO:2031 | SEQ ID NO:10043 | SEQ ID NO:18055 |
| | | AA | KSSQSVLHRSNNYNYLA | WASTRES | QQYYSTPPT |
| | | | SEQ ID NO:2032 | SEQ ID NO:10044 | SEQ ID NO:18056 |
| **iPS:435171** | **21-225_93C2** | NA | AGGGCCAGTCAGAGTGTTTA CAGCAGCTACTTAGCC | GGTGCATCCAGCCGGGC CACT | CAGCACTATGATAACTCACC GTGGACG |
| | | | SEQ ID NO:2033 | SEQ ID NO:10045 | SEQ ID NO:18057 |
| | | AA | RASQSVYSSYLA | GASSRAT | QHYDNSPWT |
| | | | SEQ ID NO:2034 | SEQ ID NO:10046 | SEQ ID NO:18058 |
| **iPS:435177** | **21-225_93E4** | NA | AGGGCCAGTCAGAGTGTTTA CAGCAGCTACTTAGCC | GGTGCATCCAGCCGGTC CACT | CAGCACTCTGATAACTCACC GTGGACG |
| | | | SEQ ID NO:2035 | SEQ ID NO:10047 | SEQ ID NO:18059 |
| | | AA | RASQSVYSSYLA | GASSRST | QHSDNSPWT |
| | | | SEQ ID NO:2036 | SEQ ID NO:10048 | SEQ ID NO:18060 |
| **iPS:435183** | **21-225_93E9** | NA | AGGGCCAGTCAGAGTGTTGA CAGCAGCTACCTAGCC | GGTGCATCCAGCAGGGC CCCT | CAGCAGTATGAAAGCTCACC GTGGACG |
| | | | SEQ ID NO:2037 | SEQ ID NO:10049 | SEQ ID NO:18061 |
| | | AA | RASQSVDSSYLA | GASSRAP | QQYESSPWT |
| | | | SEQ ID NO:2038 | SEQ ID NO:10050 | SEQ ID NO:18062 |
| **iPS:435195** | **21-225_94D3** | NA | AGGGCCAGTCAGAGTGTTAG CAGCAGGTACTTAGCC | GGTGCATCCAGCAGGGC CACT | CAGCATTATGATAGCTCACC GTGGACG |
| | | | SEQ ID NO:2039 | SEQ ID NO:10051 | SEQ ID NO:18063 |
| | | AA | RASQSVSSRYLA | GASSRAT | QHYDSSPWT |
| | | | SEQ ID NO:2040 | SEQ ID NO:10052 | SEQ ID NO:18064 |
| **iPS:435197** | **21-225_94F3** | NA | CGGGCAAGTCAGGCCATTAG AGATGATTTAGGC | GCTGCATCCAGTTTGCAA AGT | CTCCAGCATTATAGTTACCC TCGGACG |
| | | | SEQ ID NO:2041 | SEQ ID NO:10053 | SEQ ID NO:18065 |
| | | AA | RASQAIRDDLG | AASSLQS | LQHYSYPRT |
| | | | SEQ ID NO:2042 | SEQ ID NO:10054 | SEQ ID NO:18066 |

FIGURE 49

| | | | | | |
|---|---|---|---|---|---|
| **iPS:435203** | **21-225_75A7** | NA | AAGTCCAGCCAGACTGTTTTACACAGCTCCAACAATTATAACTACTTAGCT | TGGACATCTACCCGGGAATCC | CAGCAATATTTTAGTAGTCCTCCGACG |
| | | | SEQ ID NO:2043 | SEQ ID NO:10055 | SEQ ID NO:18067 |
| | | AA | KSSQTVLHSSNNYNYLA | WTSTRES | QQYFSSPPT |
| | | | SEQ ID NO:2044 | SEQ ID NO:10056 | SEQ ID NO:18068 |
| **iPS:435209** | **21-225_75A10** | NA | AAGTCCAGCCAGAGTGTTTTACACAACTCCAACAATTACAACTACTTAACT | TGGGCATCTACCCGGAAATCC | CAGCAATATTATAGTTCTCCTCCGACG |
| | | | SEQ ID NO:2045 | SEQ ID NO:10057 | SEQ ID NO:18069 |
| | | AA | KSSQSVLHNSNNYNYLT | WASTRKS | QQYYSSPPT |
| | | | SEQ ID NO:2046 | SEQ ID NO:10058 | SEQ ID NO:18070 |
| **iPS:435211** | **21-225_94E11** | NA | AAGTCCAGCCAGAGTGTTTTATTCAGCTCCAACAATTATAATTACTTAGCT | TGGGCATCTACCCGGGAATCC | CAGCAATATCATAGTTCTCCTCTGACG |
| | | | SEQ ID NO:2047 | SEQ ID NO:10059 | SEQ ID NO:18071 |
| | | AA | KSSQSVLFSSNNYNYLA | WASTRES | QQYHSSPLT |
| | | | SEQ ID NO:2048 | SEQ ID NO:10060 | SEQ ID NO:18072 |
| **iPS:435215** | **21-225_94E12** | NA | AAGTCCAGCCAGAGTGTTTTACACAGGTCCAACAATTACAACTACTTAGCG | TGGGCATCTACCCGGGAATCC | CAGCAATATTATAGTACTCCTCCGACG |
| | | | SEQ ID NO:2049 | SEQ ID NO:10061 | SEQ ID NO:18073 |
| | | AA | KSSQSVLHRSNNYNYLA | WASTRES | QQYYSTPPT |
| | | | SEQ ID NO:2050 | SEQ ID NO:10062 | SEQ ID NO:18074 |
| **iPS:435217** | **21-225_94F12** | NA | AGGGCCAGTCAGAGTGTTTACAGCAGCTACTTAGCG | GGTGCATCCAGCCGGTCCACT | CAGCATTATGATAACTCACCGTGGACG |
| | | | SEQ ID NO:2051 | SEQ ID NO:10063 | SEQ ID NO:18075 |
| | | AA | RASQSVYSSYLA | GASSRST | QHYDNSPWT |
| | | | SEQ ID NO:2052 | SEQ ID NO:10064 | SEQ ID NO:18076 |

FIGURE 49

| | | | | | |
|---|---|---|---|---|---|
| **iPS:435219** | **21-225_95D2** | NA | AGGGCCAGTCAGAGTGTTTACAGCAGCTACTTAGCC | GGTGCATCCAGCCGGGCCACT | CAGCACTATGATAACTCACCGTGGACG |
| | | | SEQ ID NO:2053 | SEQ ID NO:10065 | SEQ ID NO:18077 |
| | | AA | RASQSVYSSYLA | GASSRAT | QHYDNSPWT |
| | | | SEQ ID NO:2054 | SEQ ID NO:10066 | SEQ ID NO:18078 |
| **iPS:435221** | **21-225_95G2** | NA | AGGGCCAGTATGAGTGTTGTCAACAGCTTAGCC | GGTGCATCCACCAGGGCCACT | CAGCAGTATAATGACTGGCCGTGCAGT |
| | | | SEQ ID NO:2055 | SEQ ID NO:10067 | SEQ ID NO:18079 |
| | | AA | RASMSVVNSLA | GASTRAT | QQYNDWPCS |
| | | | SEQ ID NO:2056 | SEQ ID NO:10068 | SEQ ID NO:18080 |
| **iPS:435227** | **21-225_95G4** | NA | AAGTCCAGCCAGAGTGTTTTATTCAGATCCAACAATTATAATTACTTAGCT | TGGGCATCTACCCGGGAATCC | CAGCAATATCATAGTTCTCCTCTGACG |
| | | | SEQ ID NO:2057 | SEQ ID NO:10069 | SEQ ID NO:18081 |
| | | AA | KSSQSVLFRSNNYNYLA | WASTRES | QQYHSSPLT |
| | | | SEQ ID NO:2058 | SEQ ID NO:10070 | SEQ ID NO:18082 |
| **iPS:435235** | **21-225_95F9** | NA | AGGGCCAGTCAGAGTGTTTACAGCAGCTACTTAGCC | GGTGCATCCAGCCGGTCCACT | CAGCACTATGATAACTCACCGTGGACG |
| | | | SEQ ID NO:2059 | SEQ ID NO:10071 | SEQ ID NO:18083 |
| | | AA | RASQSVYSSYLA | GASSRST | QHYDNSPWT |
| | | | SEQ ID NO:2060 | SEQ ID NO:10072 | SEQ ID NO:18084 |
| **iPS:435237** | **21-225_95G9** | NA | AGGGCCAGTCAGAGTGTTTACAGCAGCTACTTAGCG | GGTGCATCCAGCCGGTCCACT | CAGCACTATGATAACTCACCGTGGACG |
| | | | SEQ ID NO:2061 | SEQ ID NO:10073 | SEQ ID NO:18085 |
| | | AA | RASQSVYSSYLA | GASSRST | QHYDNSPWT |
| | | | SEQ ID NO:2062 | SEQ ID NO:10074 | SEQ ID NO:18086 |
| **iPS:435239** | **21-225_95H10** | NA | AGGGCCAGTCAGAGTGTTTACAGCAGCTACTTAGCC | GGTGCATCCAGCCGGGCCACT | CAGCACTATGATAACTCTCCGTGGACG |
| | | | SEQ ID NO:2063 | SEQ ID NO:10075 | SEQ ID NO:18087 |
| | | AA | RASQSVYSSYLA | GASSRAT | QHYDNSPWT |

FIGURE 49

| | | | | | |
|---|---|---|---|---|---|
| | | | SEQ ID NO:2064 | SEQ ID NO:10076 | SEQ ID NO:18088 |
| **iPS:435245** | **21-225_95E12** | NA | AAGTCCAGCCAGAGTGTTTTATACAGCTCCAACAATGCGAACTACTTAGCT | TGGGCATCTACCCGGGAATCC | CAGCAATATTATAGTACTCCGTGCAGT |
| | | | SEQ ID NO:2065 | SEQ ID NO:10077 | SEQ ID NO:18089 |
| | | AA | KSSQSVLYSSNNANYLA | WASTRES | QQYYSTPCS |
| | | | SEQ ID NO:2066 | SEQ ID NO:10078 | SEQ ID NO:18090 |
| **iPS:435247** | **21-225_96G1** | NA | AGGGCCAGTCAGAGCGTTAGCAGCAGCTACTTAGCT | GGTGCATCCACCAGGGCCTCT | CAGCAGTATGGTAACTCACCGCTCACT |
| | | | SEQ ID NO:2067 | SEQ ID NO:10079 | SEQ ID NO:18091 |
| | | AA | RASQSVSSSYLA | GASTRAS | QQYGNSPLT |
| | | | SEQ ID NO:2068 | SEQ ID NO:10080 | SEQ ID NO:18092 |
| **iPS:435249** | **21-225_96E2** | NA | AAGTCCAGCCAGAGTGTTTTACACAGCTCTAATAAAAAGAACTACTTAGCT | TGGGCATCTACCTGGGAATCC | CAGCAATATTATAGGACTCCGTGGACG |
| | | | SEQ ID NO:2069 | SEQ ID NO:10081 | SEQ ID NO:18093 |
| | | AA | KSSQSVLHSSNKKNYLA | WASTWES | QQYYRTPWT |
| | | | SEQ ID NO:2070 | SEQ ID NO:10082 | SEQ ID NO:18094 |
| **iPS:435251** | **21-225_96A3** | NA | CGGGCAAGTCAGGGCATTAGAAATGATTTAGGC | GCTGCATCCACTTTGCAAAGT | CTACAGCATAGTAATTACCCGCTCACT |
| | | | SEQ ID NO:2071 | SEQ ID NO:10083 | SEQ ID NO:18095 |
| | | AA | RASQGIRNDLG | AASTLQS | LQHSNYPLT |
| | | | SEQ ID NO:2072 | SEQ ID NO:10084 | SEQ ID NO:18096 |
| **iPS:435253** | **21-225_96A4** | NA | CGGGCAAGTCAGGACATTAGAAATGATTTAGGC | GGTGTATCCAGTTTGCAAAGT | CTACAGCATAATGATTACCCATTCACT |
| | | | SEQ ID NO:2073 | SEQ ID NO:10085 | SEQ ID NO:18097 |
| | | AA | RASQDIRNDLG | GVSSLQS | LQHNDYPFT |
| | | | SEQ ID NO:2074 | SEQ ID NO:10086 | SEQ ID NO:18098 |

FIGURE 49

| | | | | | |
|---|---|---|---|---|---|
| **iPS:435255** | **21-225_96D5** | NA | AAGTCCAGCCAGAGTGTTTTGCACAGCTCCAACAATTATAATTACTTAGCT | TGGGCATCTACCCGGGAATCC | CAGCAATATTATAGTAGTCCACCGACG |
| | | | SEQ ID NO:2075 | SEQ ID NO:10087 | SEQ ID NO:18099 |
| | | AA | KSSQSVLHSSNNYNYLA | WASTRES | QQYYSSPPT |
| | | | SEQ ID NO:2076 | SEQ ID NO:10088 | SEQ ID NO:18100 |
| **iPS:435257** | **21-225_96H5** | NA | AAGTCCAGCCAGAGTGTTTTGTACAGCTCCAACAGTCACAACTACTTAGCT | TGGGCATCTATCCGGGAATCC | CAGCAATATTATAGTACTCCGTGCAGT |
| | | | SEQ ID NO:2077 | SEQ ID NO:10089 | SEQ ID NO:18101 |
| | | AA | KSSQSVLYSSNSHNYLA | WASIRES | QQYYSTPCS |
| | | | SEQ ID NO:2078 | SEQ ID NO:10090 | SEQ ID NO:18102 |
| **iPS:435259** | **21-225_96C6** | NA | CGGGCGAGTCAGGGCATTAGCAATTATTTAGCC | GCTGCTTCCAGTTTGCAAAGT | CACCAGTATAATGATTACCCATTCACT |
| | | | SEQ ID NO:2079 | SEQ ID NO:10091 | SEQ ID NO:18103 |
| | | AA | RASQGISNYLA | AASSLQS | HQYNDYPFT |
| | | | SEQ ID NO:2080 | SEQ ID NO:10092 | SEQ ID NO:18104 |
| **iPS:435267** | **21-225_96D10** | NA | AAGTCCAGTCAGAATATTTTATCCAGCTCCAACAATAAGAACTACTTAACT | TGGACATCTACCCGGGAATCC | CAGCAATATTTTAGTGTTCCTCCGACG |
| | | | SEQ ID NO:2081 | SEQ ID NO:10093 | SEQ ID NO:18105 |
| | | AA | KSSQNILSSSNNKNYLT | WTSTRES | QQYFSVPPT |
| | | | SEQ ID NO:2082 | SEQ ID NO:10094 | SEQ ID NO:18106 |
| **iPS:435273** | **21-225_97A2** | NA | AGGGCCAGTCAGAGTGTTTACAGCAGCTACTTAGCC | GGTGCATCCAGCCGGTCCACT | CAGCATTCTGATAACTCACCGTGGACG |
| | | | SEQ ID NO:2083 | SEQ ID NO:10095 | SEQ ID NO:18107 |
| | | AA | RASQSVYSSYLA | GASSRST | QHSDNSPWT |
| | | | SEQ ID NO:2084 | SEQ ID NO:10096 | SEQ ID NO:18108 |

FIGURE 49

| | | | | | |
|---|---|---|---|---|---|
| **iPS:435279** | **21-225_97H4** | NA | AAGTCCAGCCAGAGTGTTTTATACACCTCCAACAATAACAACTACTTAGCT | TGGGCATCTACCCGGGAATCC | CAGCACTACAATGATACTCCATGGAAG |
| | | | SEQ ID NO:2085 | SEQ ID NO:10097 | SEQ ID NO:18109 |
| | | AA | KSSQSVLYTSNNNNYLA | WASTRES | QHYNDTPWK |
| | | | SEQ ID NO:2086 | SEQ ID NO:10098 | SEQ ID NO:18110 |
| **iPS:435281** | **21-225_97E5** | NA | AGGGCCAGTCAGAGTGTTTACAGCAGCTACTTAGCC | GGTGCATCCAGCCGGGCCACT | CAGCACTCTGATAACTCACCGTGGACG |
| | | | SEQ ID NO:2087 | SEQ ID NO:10099 | SEQ ID NO:18111 |
| | | AA | RASQSVYSSYLA | GASSRAT | QHSDNSPWT |
| | | | SEQ ID NO:2088 | SEQ ID NO:10100 | SEQ ID NO:18112 |
| **iPS:435291** | **21-225_146E1** | NA | CGGGCGAGTCAGGGTATTAACAACTGGTTAGTC | GCTGCATCCAGTTTGCAAAGT | CAACAGGCTAACAGTTTCCCATTCACT |
| | | | SEQ ID NO:2089 | SEQ ID NO:10101 | SEQ ID NO:18113 |
| | | AA | RASQGINNWLV | AASSLQS | QQANSFPFT |
| | | | SEQ ID NO:2090 | SEQ ID NO:10102 | SEQ ID NO:18114 |
| **iPS:435293** | **21-225_146F1** | NA | CGGGCAAGTCAGGGCATTAGAAATGATTTAGGC | GCTGCATCCAGTTTGCAAAGT | CTTCAGCATAGTACTTACCCGCTCACT |
| | | | SEQ ID NO:2091 | SEQ ID NO:10103 | SEQ ID NO:18115 |
| | | AA | RASQGIRNDLG | AASSLQS | LQHSTYPLT |
| | | | SEQ ID NO:2092 | SEQ ID NO:10104 | SEQ ID NO:18116 |
| **iPS:435295** | **21-225_146H1** | NA | CGGGCAAGTCAGAGCATTAGCGACTATTTAAAT | ACTACATCCAGTTTGCAAAGT | CAACAGAGTTACAGTACCCCCACT |
| | | | SEQ ID NO:2093 | SEQ ID NO:10105 | SEQ ID NO:18117 |
| | | AA | RASQSISDYLN | TTSSLQS | QQSYSTPT |
| | | | SEQ ID NO:2094 | SEQ ID NO:10106 | SEQ ID NO:18118 |
| **iPS:435297** | **21-225_146B3** | NA | AAGTCTAGTCAGAGCCTCCTGCATGGTGATGGAAAGACCTATTTGTAT | GAAGTTTCCAACCGGTTCTCT | ATGCAAAGTATACAGCTTCCGTGGACG |
| | | | SEQ ID NO:2095 | SEQ ID NO:10107 | SEQ ID NO:18119 |

**FIGURE 49**

| | | | | | |
|---|---|---|---|---|---|
| | | AA | KSSQSLLHGDGKTYLY | EVSNRFS | MQSIQLPWT |
| | | | SEQ ID NO:2096 | SEQ ID NO:10108 | SEQ ID NO:18120 |
| **iPS:435299** | **21-225_146D4** | NA | AAGTCCAGCCAGAGTGTTTTATACAGCTCCAACAATAACAACTACTTAGCT | TGGGCATCTACCCGGGAATCC | CAGCAATATTATAGTACTCCGTGCAGT |
| | | | SEQ ID NO:2097 | SEQ ID NO:10109 | SEQ ID NO:18121 |
| | | AA | KSSQSVLYSSNNNNYLA | WASTRES | QQYYSTPCS |
| | | | SEQ ID NO:2098 | SEQ ID NO:10110 | SEQ ID NO:18122 |
| **iPS:435301** | **21-225_146G4** | NA | AGGGCCAGTCAGAATATTATCAGCAGCTATTTAGCC | GGTGTATCTAGTCGGGCCACT | CAACAATATGGTAGGTCACCATTCAAT |
| | | | SEQ ID NO:2099 | SEQ ID NO:10111 | SEQ ID NO:18123 |
| | | AA | RASQNIISSYLA | GVSSRAT | QQYGRSPFN |
| | | | SEQ ID NO:2100 | SEQ ID NO:10112 | SEQ ID NO:18124 |
| **iPS:435303** | **21-225_146A6** | NA | CGGGCGAGTCAGGGTATTAGCAACTGGTTAGCC | GCTGCATCCAGTTTGCAAGGT | CAACAGACTGACAGTTTCCCATTCACT |
| | | | SEQ ID NO:2101 | SEQ ID NO:10113 | SEQ ID NO:18125 |
| | | AA | RASQGISNWLA | AASSLQG | QQTDSFPFT |
| | | | SEQ ID NO:2102 | SEQ ID NO:10114 | SEQ ID NO:18126 |
| **iPS:435305** | **21-225_146C9** | NA | AAGTCCAGCCAGAGTGTTTTACACAGCTCCAACAATTATAATTACTTAGCT | TGGGCATCTACCCGGAAATCC | CAGCAATATTATAGTACTCCGTGCAGT |
| | | | SEQ ID NO:2103 | SEQ ID NO:10115 | SEQ ID NO:18127 |
| | | AA | KSSQSVLHSSNNYNYLA | WASTRKS | QQYYSTPCS |
| | | | SEQ ID NO:2104 | SEQ ID NO:10116 | SEQ ID NO:18128 |
| **iPS:435307** | **21-225_146E9** | NA | CGGGCAAGTCAGAGCATTAGCGACTATTTAAAT | ACTACATCCAGTTTGCAAAGT | CAACAGAGTTACAGTACCCCCACT |
| | | | SEQ ID NO:2105 | SEQ ID NO:10117 | SEQ ID NO:18129 |
| | | AA | RASQSISDYLN | TTSSLQS | QQSYSTPT |
| | | | SEQ ID NO:2106 | SEQ ID NO:10118 | SEQ ID NO:18130 |

FIGURE 49

| | | | | | |
|---|---|---|---|---|---|
| **iPS:435309** | **21-225_146F9** | NA | AAGTCCAGCCAGAATATTTTACACAGCTCCAACAATAACAACTACTTAGCT | TGGGCATCTACCCGGGAATCC | CAGCAATATTATACTACTCCGTGCAGT |
| | | | SEQ ID NO:2107 | SEQ ID NO:10119 | SEQ ID NO:18131 |
| | | AA | KSSQNILHSSNNNNYLA | WASTRES | QQYYTTPCS |
| | | | SEQ ID NO:2108 | SEQ ID NO:10120 | SEQ ID NO:18132 |
| **iPS:435311** | **21-225_146H9** | NA | CGGGCAAGTCAGGGCATTAGAAATGATTTAGGC | GCTGCATCCAGTTTGCAAAGT | CTACAGCATAATAGTTACCCGCTCACT |
| | | | SEQ ID NO:2109 | SEQ ID NO:10121 | SEQ ID NO:18133 |
| | | AA | RASQGIRNDLG | AASSLQS | LQHNSYPLT |
| | | | SEQ ID NO:2110 | SEQ ID NO:10122 | SEQ ID NO:18134 |
| **iPS:435313** | **21-225_146G11** | NA | CGGGCAAGTCAGGACATTAGAAATAATTTTGGC | GCTGCATCCAGTTTGCAAAGT | CTCCAACATGATAGTTACCCGCTCACT |
| | | | SEQ ID NO:2111 | SEQ ID NO:10123 | SEQ ID NO:18135 |
| | | AA | RASQDIRNNFG | AASSLQS | LQHDSYPLT |
| | | | SEQ ID NO:2112 | SEQ ID NO:10124 | SEQ ID NO:18136 |
| **iPS:435315** | **21-225_147B2** | NA | AAGTCTAGTCAGAGCCTCCTGCATAGTGAAGGAAAGACCTATTTGTAT | GAAGTTTCCCACCGGGTCTCT | ATGCAAAGTACACAGTTTCCTCCCACT |
| | | | SEQ ID NO:2113 | SEQ ID NO:10125 | SEQ ID NO:18137 |
| | | AA | KSSQSLLHSEGKTYLY | EVSHRVS | MQSTQFPPT |
| | | | SEQ ID NO:2114 | SEQ ID NO:10126 | SEQ ID NO:18138 |
| **iPS:435317** | **21-225_147D2** | NA | AGGGCCAGTCAGAGTGTTGGCAGCAGCTACTTAGCC | GGTGCATCCAGCAGGGCCACT | CAGCAGTATGGTAGCTTATTCACT |
| | | | SEQ ID NO:2115 | SEQ ID NO:10127 | SEQ ID NO:18139 |
| | | AA | RASQSVGSSYLA | GASSRAT | QQYGSLFT |
| | | | SEQ ID NO:2116 | SEQ ID NO:10128 | SEQ ID NO:18140 |
| **iPS:435319** | **21-225_147E3** | NA | AGGGCCAGTCAGAGTGTTATCAGTAGCTACTTAGCC | GGTGCATCCAGCAGGGCCACT | CAACAATATGGTAGGTCACCATTCAAT |
| | | | SEQ ID NO:2117 | SEQ ID NO:10129 | SEQ ID NO:18141 |

**FIGURE 49**

| | | | | | |
|---|---|---|---|---|---|
| | | AA | RASQSVISSYLA | GASSRAT | QQYGRSPFN |
| | | | SEQ ID NO:2118 | SEQ ID NO:10130 | SEQ ID NO:18142 |
| **iPS:435321** | **21-225_147E4** | NA | AAGTCCAGCCAGAGTGTTTTACACAGCTCCAACAATTACAACTACTTAGCT | TGGGCATCTACCCGGGAATCC | CAGCAATATTATAGTACTCCATCCACT |
| | | | SEQ ID NO:2119 | SEQ ID NO:10131 | SEQ ID NO:18143 |
| | | AA | KSSQSVLHSSNNYNYLA | WASTRES | QQYYSTPST |
| | | | SEQ ID NO:2120 | SEQ ID NO:10132 | SEQ ID NO:18144 |
| **iPS:435323** | **21-225_147D5** | NA | AAGTCCAGCCAGAGTGTTTTATACAGCTCCAACAATAACAACTACTTAGCT | TGGGCATCTACCCGGGAATCC | CACCAATATTATAGTACTCCGTGCAGT |
| | | | SEQ ID NO:2121 | SEQ ID NO:10133 | SEQ ID NO:18145 |
| | | AA | KSSQSVLYSSNNNNYLA | WASTRES | HQYYSTPCS |
| | | | SEQ ID NO:2122 | SEQ ID NO:10134 | SEQ ID NO:18146 |
| **iPS:435325** | **21-225_147H5** | NA | CGGGCAAGTCGGGGCATTAGAGATGATTTAGGC | GCTGCATCCAGTTTGCAGAGT | CTACAGCATTATAGTTATCCTCGGACG |
| | | | SEQ ID NO:2123 | SEQ ID NO:10135 | SEQ ID NO:18147 |
| | | AA | RASRGIRDDLG | AASSLQS | LQHYSYPRT |
| | | | SEQ ID NO:2124 | SEQ ID NO:10136 | SEQ ID NO:18148 |
| **iPS:435327** | **21-225_147G6** | NA | AAGTCCAGCCAGAGTGTTTTATACAGCTCCAACAGTAACAACTACTTAGCT | TGGGCATCTGCCCGGGAATCC | CAGCAATATTATACTACTCCTCCCACT |
| | | | SEQ ID NO:2125 | SEQ ID NO:10137 | SEQ ID NO:18149 |
| | | AA | KSSQSVLYSSNSNNYLA | WASARES | QQYYTTPPT |
| | | | SEQ ID NO:2126 | SEQ ID NO:10138 | SEQ ID NO:18150 |
| **iPS:435329** | **21-225_147A8** | NA | AAGACTAGTCAGAGCCTCCTGCATAGTGAAGGAAAGACCTATTTGTAT | GAAGTTTCCAACCGGTTCTCT | ATGCAAAGTATACAGCTAATCACC |
| | | | SEQ ID NO:2127 | SEQ ID NO:10139 | SEQ ID NO:18151 |
| | | AA | KTSQSLLHSEGKTYLY | EVSNRFS | MQSIQLIT |

FIGURE 49

| | | | | | |
|---|---|---|---|---|---|
| | | | SEQ ID NO:2128 | SEQ ID NO:10140 | SEQ ID NO:18152 |
| **iPS:435331** | **21-225_147G8** | NA | AGGGCCAGTCAGAGAATTTT CAGCAACTACTTAGCC | GGTGCATCCAGCAGGGC CACT | CAGCAGTATGATAGCTCACC GTGGACG |
| | | | SEQ ID NO:2129 | SEQ ID NO:10141 | SEQ ID NO:18153 |
| | | AA | RASQRIFSNYLA | GASSRAT | QQYDSSPWT |
| | | | SEQ ID NO:2130 | SEQ ID NO:10142 | SEQ ID NO:18154 |
| **iPS:435333** | **21-225_147E9** | NA | CGGGCGAGTCAGGACATTAA CAATTATTTAGCC | GCTGCATCCAGTTTGCAA AGT | CAACAGTATAATAGTTACCC TCTCACT |
| | | | SEQ ID NO:2131 | SEQ ID NO:10143 | SEQ ID NO:18155 |
| | | AA | RASQDINNYLA | AASSLQS | QQYNSYPLT |
| | | | SEQ ID NO:2132 | SEQ ID NO:10144 | SEQ ID NO:18156 |
| **iPS:435335** | **21-225_147D10** | NA | CGGGCGAGTCAGAATATTAG CAACTGGTTAACC | GCTGCATCCAGTTTGCAA AGT | CAACAGACTGACAGTTTCCC ATTCACT |
| | | | SEQ ID NO:2133 | SEQ ID NO:10145 | SEQ ID NO:18157 |
| | | AA | RASQNISNWLT | AASSLQS | QQTDSFPFT |
| | | | SEQ ID NO:2134 | SEQ ID NO:10146 | SEQ ID NO:18158 |
| **iPS:435339** | **21-225_147D12** | NA | CGGGCGAGTCAGGGTATTAG CAACTGGTTAGCC | GCTGCATCCAGTTTGCAA AGT | CAACAGACTGACAGTTTCCC ATTCACT |
| | | | SEQ ID NO:2135 | SEQ ID NO:10147 | SEQ ID NO:18159 |
| | | AA | RASQGISNWLA | AASSLQS | QQTDSFPFT |
| | | | SEQ ID NO:2136 | SEQ ID NO:10148 | SEQ ID NO:18160 |
| **iPS:435341** | **21-225_148B2** | NA | AAGTCTAGTCAGAGCCTCCT GCATGGTGATGGAAAGACCT ATTTTTAT | GAAGTTTCCCACCGGTTC TCT | ATGCAAAGTATACAGATTCC GTGGACG |
| | | | SEQ ID NO:2137 | SEQ ID NO:10149 | SEQ ID NO:18161 |
| | | AA | KSSQSLLHGDGKTYFY | EVSHRFS | MQSIQIPWT |
| | | | SEQ ID NO:2138 | SEQ ID NO:10150 | SEQ ID NO:18162 |
| **iPS:435343** | **21-225_148E2** | NA | CGGGCGAGTCAGGGTATTAG CAACTGGTTAGCC | GCTGCATCCAGTTTGCAA AGT | CAACAGACTGACAGTTTCCC ATTCACT |
| | | | SEQ ID NO:2139 | SEQ ID NO:10151 | SEQ ID NO:18163 |

FIGURE 49

| | | | | | |
|---|---|---|---|---|---|
| | | AA | RASQGISNWLA | AASSLQS | QQTDSFPFT |
| | | | SEQ ID NO:2140 | SEQ ID NO:10152 | SEQ ID NO:18164 |
| **iPS:435345** | **21-225_148G3** | NA | AAGTCCAGCCAACGTGTTTT ACACAGCTCCAACAATTATA ACTACTTAGCT | TGGGCATCTACCCGGGA TTCC | CAGCAATATTATAGTACTCC ATTCACT |
| | | | SEQ ID NO:2141 | SEQ ID NO:10153 | SEQ ID NO:18165 |
| | | AA | KSSQRVLHSSNNYNYLA | WASTRDS | QQYYSTPFT |
| | | | SEQ ID NO:2142 | SEQ ID NO:10154 | SEQ ID NO:18166 |
| **iPS:435347** | **21-225_148C4** | NA | CGGGCAAGTCAGAGCATTAT CAACTATTTAAAT | ACTGCATCCAGTTTACAG AGT | CAACAGAGTTACAGTACCCC CACT |
| | | | SEQ ID NO:2143 | SEQ ID NO:10155 | SEQ ID NO:18167 |
| | | AA | RASQSIINYLN | TASSLQS | QQSYSTPT |
| | | | SEQ ID NO:2144 | SEQ ID NO:10156 | SEQ ID NO:18168 |
| **iPS:435349** | **21-225_148F5** | NA | AAGTCTAGTCAGAGCCTCCT GCATAGTGAAGGAAAGACC TATTTGTAT | GAAGTTTCCTACCGGGTC TCT | ATGCAAAGTATACAGCTTCC GCTCACT |
| | | | SEQ ID NO:2145 | SEQ ID NO:10157 | SEQ ID NO:18169 |
| | | AA | KSSQSLLHSEGKTYLY | EVSYRVS | MQSIQLPLT |
| | | | SEQ ID NO:2146 | SEQ ID NO:10158 | SEQ ID NO:18170 |
| **iPS:435351** | **21-225_148B6** | NA | CGGGCGAGTCAGGGCATTAG CAAATATTTAGCC | GCTGCATCCAGTTTGCAA AGT | CAACAGTATAATAGTTTCCC ATTCACT |
| | | | SEQ ID NO:2147 | SEQ ID NO:10159 | SEQ ID NO:18171 |
| | | AA | RASQGISKYLA | AASSLQS | QQYNSFPFT |
| | | | SEQ ID NO:2148 | SEQ ID NO:10160 | SEQ ID NO:18172 |
| **iPS:435353** | **21-225_148F8** | NA | AAGTCCAGCCAGAGTGCTTT ACACAGCTCCAACAATTACA ACTACTTAGCT | TGGGCATCTACCCGGAA ATCC | CAGCAATATTATAGTATTCC TCCGACG |
| | | | SEQ ID NO:2149 | SEQ ID NO:10161 | SEQ ID NO:18173 |
| | | AA | KSSQSALHSSNNYNYLA | WASTRKS | QQYYSIPPT |
| | | | SEQ ID NO:2150 | SEQ ID NO:10162 | SEQ ID NO:18174 |

**FIGURE 49**

| | | | | | |
|---|---|---|---|---|---|
| **iPS:435355** | **21-225_148H9** | NA | CGGGCAAGTCAGAGCATTAG TAACTATTTAAAT | ATTGCATCCAGTTTGCAA AGT | CAACAGAGTTACAGTACCCC CACT |
| | | | SEQ ID NO:2151 | SEQ ID NO:10163 | SEQ ID NO:18175 |
| | | AA | RASQSISNYLN | IASSLQS | QQSYSTPT |
| | | | SEQ ID NO:2152 | SEQ ID NO:10164 | SEQ ID NO:18176 |
| **iPS:435357** | **21-225_148G10** | NA | AAGTCTAGTCAGAGCCTCCT GCATGGTGATGGAAAGACCT ATTTGTAT | GAAGTTTCCAACCGGTTC TCT | ATGCAAAGTATACAGCTTCC GTGGACG |
| | | | SEQ ID NO:2153 | SEQ ID NO:10165 | SEQ ID NO:18177 |
| | | AA | KSSQSLLHGDGKTYLY | EVSNRFS | MQSIQLPWT |
| | | | SEQ ID NO:2154 | SEQ ID NO:10166 | SEQ ID NO:18178 |
| **iPS:435359** | **21-225_148H10** | NA | AAGTCTAGTCAGAGCCTCCT GCATAGTGAGGGAAAGACC TATTTGTAT | GAAGTTTCCTACCGGGTC TCT | ATGCAGAGTATACAGCTTCC GCTCACT |
| | | | SEQ ID NO:2155 | SEQ ID NO:10167 | SEQ ID NO:18179 |
| | | AA | KSSQSLLHSEGKTYLY | EVSYRVS | MQSIQLPLT |
| | | | SEQ ID NO:2156 | SEQ ID NO:10168 | SEQ ID NO:18180 |
| **iPS:435361** | **21-225_148E11** | NA | CGGGCAAGTCAGGGCATTAG AAATGATTTAGGC | GCTGCATCCAGTTTGCAA AGT | CTACAGCATCGTAATTACCC GCTCACT |
| | | | SEQ ID NO:2157 | SEQ ID NO:10169 | SEQ ID NO:18181 |
| | | AA | RASQGIRNDLG | AASSLQS | LQHRNYPLT |
| | | | SEQ ID NO:2158 | SEQ ID NO:10170 | SEQ ID NO:18182 |
| **iPS:435363** | **21-225_148F12** | NA | CGGGCAAGTCAGGGCATTAG AAATGCCTTAGGC | GCTGCATCCAGTTTGCAA AGT | CTACAGCATAATAGTTACCC TCTCATT |
| | | | SEQ ID NO:2159 | SEQ ID NO:10171 | SEQ ID NO:18183 |
| | | AA | RASQGIRNALG | AASSLQS | LQHNSYPLI |
| | | | SEQ ID NO:2160 | SEQ ID NO:10172 | SEQ ID NO:18184 |
| **iPS:435365** | **21 225 149F1** | NA | AAGTCTAGTCAGAGCCTCCT GCATGGTGATGGAAAGACCT ATTTTTAT | GAAGTTTCCCACCGGTTC TCT | ATGCAAAGTATACAGATTCC GTGGACG |

# FIGURE 49

| | | | | | |
|---|---|---|---|---|---|
| | 21-225_149F1 | | SEQ ID NO:2161 | SEQ ID NO:10173 | SEQ ID NO:18185 |
| | | AA | KSSQSLLHGDGKTYFY | EVSHRFS | MQSIQIPWT |
| | | | SEQ ID NO:2162 | SEQ ID NO:10174 | SEQ ID NO:18186 |
| **iPS:435367** | 21-225_149G1 | NA | CGGGCAAGTCAGGGCATTAG AAATGATTTAGGC | GCTGCATCCAATTTGCAA AGT | CTACAGCATAATAGTTACCC GCTCACT |
| | | | SEQ ID NO:2163 | SEQ ID NO:10175 | SEQ ID NO:18187 |
| | | AA | RASQGIRNDLG | AASNLQS | LQHNSYPLT |
| | | | SEQ ID NO:2164 | SEQ ID NO:10176 | SEQ ID NO:18188 |
| **iPS:435369** | 21-225_149A2 | NA | AAGTCCAGCCAGAGTGTTTT ATACAGTCCCAACAATAACA ACTACTTAGCT | TGGGCATCTACCCGGGA ATCC | CAGCAATATTATAGTACTCC GTGCAGT |
| | | | SEQ ID NO:2165 | SEQ ID NO:10177 | SEQ ID NO:18189 |
| | | AA | KSSQSVLYSPNNNNYLA | WASTRES | QQYYSTPCS |
| | | | SEQ ID NO:2166 | SEQ ID NO:10178 | SEQ ID NO:18190 |
| **iPS:435371** | 21-225_149A3 | NA | CGGGCAAGTCAGAGCATTAG CAGTTATTTAAAT | ACTGCATCCAGTTTGCAA AGT | CAACAGAGTTACAGTATCCC CACT |
| | | | SEQ ID NO:2167 | SEQ ID NO:10179 | SEQ ID NO:18191 |
| | | AA | RASQSISSYLN | TASSLQS | QQSYSIPT |
| | | | SEQ ID NO:2168 | SEQ ID NO:10180 | SEQ ID NO:18192 |
| **iPS:435373** | 21-225_149E3 | NA | AAGTCCAGCCAGACTGTTTT ACACAACTCCAATAATCACA ATTACTTTGCT | TGGGCATCTACCCTGAG ATCC | CAGCAATATTATAGTACTCC TCCGACG |
| | | | SEQ ID NO:2169 | SEQ ID NO:10181 | SEQ ID NO:18193 |
| | | AA | KSSQTVLHNSNNHNYFA | WASTLRS | QQYYSTPPT |
| | | | SEQ ID NO:2170 | SEQ ID NO:10182 | SEQ ID NO:18194 |
| **iPS:435375** | 21-225_149H4 | NA | AAGTCCAGCCAGAGTGTTTT ATCCAGCTCCAACGATAACA ACTACTTAGCT | TGGTCATCTACCCGGGA ATCC | CACCAATATTATAGTTATCC TCCGACG |
| | | | SEQ ID NO:2171 | SEQ ID NO:10183 | SEQ ID NO:18195 |
| | | AA | KSSQSVLSSSNDNNYLA | WSSTRES | HQYYSYPPT |

FIGURE 49

| | | | | | |
|---|---|---|---|---|---|
| | | | SEQ ID NO:2172 | SEQ ID NO:10184 | SEQ ID NO:18196 |
| **iPS:435377** | **21-225_149G5** | NA | CGGGCAAGTCAGGGCATTAG AAATGCCTTAGGC | GCTGCATCCAGTTTGCAA AGT | CTACAGCATAATAGTTACCC TCTCACT |
| | | | SEQ ID NO:2173 | SEQ ID NO:10185 | SEQ ID NO:18197 |
| | | AA | RASQGIRNALG | AASSLQS | LQHNSYPLT |
| | | | SEQ ID NO:2174 | SEQ ID NO:10186 | SEQ ID NO:18198 |
| **iPS:435379** | **21-225_149B6** | NA | CGGGCGAGTCAGGGTATTAT CAGTTGGTTAGCC | GCTGCATCCAGTTTGCAA AGT | CAACAGGGTAACAGTTTCCC ATTCACT |
| | | | SEQ ID NO:2175 | SEQ ID NO:10187 | SEQ ID NO:18199 |
| | | AA | RASQGIISWLA | AASSLQS | QQGNSFPFT |
| | | | SEQ ID NO:2176 | SEQ ID NO:10188 | SEQ ID NO:18200 |
| **iPS:435381** | **21-225_149C6** | NA | CGGGCGAGTCAGGGTATTAG CAACTGGTTAGCC | GCTGCATCCAGTTTGCAA GGT | CAACAGACTGACAGTTTCCC ATTCACT |
| | | | SEQ ID NO:2177 | SEQ ID NO:10189 | SEQ ID NO:18201 |
| | | AA | RASQGISNWLA | AASSLQG | QQTDSFPFT |
| | | | SEQ ID NO:2178 | SEQ ID NO:10190 | SEQ ID NO:18202 |
| **iPS:435383** | **21-225_149D7** | NA | AGGGCCAGTCAGAGTATTAT CAGCAACTACTTAGCC | GGTGTATCTAGTAGGGC CACT | CAACAATATGGTCGGTCACC ATTCAAT |
| | | | SEQ ID NO:2179 | SEQ ID NO:10191 | SEQ ID NO:18203 |
| | | AA | RASQSIISNYLA | GVSSRAT | QQYGRSPFN |
| | | | SEQ ID NO:2180 | SEQ ID NO:10192 | SEQ ID NO:18204 |
| **iPS:435391** | **21-225_149F8** | NA | CGGGCGAGTCAGGGTATTAG CAACTGGTTAGCC | GCTGCATCCAGTTTGCAA AGT | CAACAGACTGACAGTTTCCC ATTCACT |
| | | | SEQ ID NO:2181 | SEQ ID NO:10193 | SEQ ID NO:18205 |
| | | AA | RASQGISNWLA | AASSLQS | QQTDSFPFT |
| | | | SEQ ID NO:2182 | SEQ ID NO:10194 | SEQ ID NO:18206 |
| **iPS:435393** | **21-225_149D10** | NA | CGGGCAAGTCGGGGCATTAG AAATGATTTAGGC | GCTGCATCCAGTTTGCAG AGT | CTACAGCATTATAGTTATCC TCGGACG |
| | | | SEQ ID NO:2183 | SEQ ID NO:10195 | SEQ ID NO:18207 |
| | | AA | RASRGIRNDLG | AASSLQS | LQHYSYPRT |

**FIGURE 49**

| | | | | | |
|---|---|---|---|---|---|
| | | | SEQ ID NO:2184 | SEQ ID NO:10196 | SEQ ID NO:18208 |
| **iPS:435395** | **21-225_149D11** | NA | CGGGCGAGTCAGAATATTAGCAACTGGTTAACC | GCTGCATCCAGTTTGCAAAGT | CAACAGACTGACAGTTTCCCATTCACT |
| | | | SEQ ID NO:2185 | SEQ ID NO:10197 | SEQ ID NO:18209 |
| | | AA | RASQNISNWLT | AASSLQS | QQTDSFPFT |
| | | | SEQ ID NO:2186 | SEQ ID NO:10198 | SEQ ID NO:18210 |
| **iPS:435397** | **21-225_149F12** | NA | CGGGCAAGTCAGGGCATTAGAAATGATTTAGGC | GCTGCATCCAATTTGCAAAGT | CTACAGCATAATAGTTACCCGCTCACT |
| | | | SEQ ID NO:2187 | SEQ ID NO:10199 | SEQ ID NO:18211 |
| | | AA | RASQGIRNDLG | AASNLQS | LQHNSYPLT |
| | | | SEQ ID NO:2188 | SEQ ID NO:10200 | SEQ ID NO:18212 |
| **iPS:435399** | **21-225_150D2** | NA | AAGTCCAGCCAGAGTGTTTTATACAGATCCAACAGTAAGAAATACTTAACT | TGGGCATCTACCCGGAAATCC | CAGCAATATTTTAGTACTCCGTACAAT |
| | | | SEQ ID NO:2189 | SEQ ID NO:10201 | SEQ ID NO:18213 |
| | | AA | KSSQSVLYRSNSKKYLT | WASTRKS | QQYFSTPYN |
| | | | SEQ ID NO:2190 | SEQ ID NO:10202 | SEQ ID NO:18214 |
| **iPS:435401** | **21-225_150E2** | NA | CGGGCAAGTCAGGGCATTGGAAATGATTTAGGC | GCTGCATCCAGTTTGCAAAGT | CTACAACATTATAGTTTCCCGTACAGT |
| | | | SEQ ID NO:2191 | SEQ ID NO:10203 | SEQ ID NO:18215 |
| | | AA | RASQGIGNDLG | AASSLQS | LQHYSFPYS |
| | | | SEQ ID NO:2192 | SEQ ID NO:10204 | SEQ ID NO:18216 |
| **iPS:435403** | **21-225_150C5** | NA | CGGGCGAGTCAGGGTATTAACAACTGGTTAGCC | GCTGCATCCAGTTTGCAAGGT | CAACAGACTGACAGTTTCCCATTCACT |
| | | | SEQ ID NO:2193 | SEQ ID NO:10205 | SEQ ID NO:18217 |
| | | AA | RASQGINNWLA | AASSLQG | QQTDSFPFT |
| | | | SEQ ID NO:2194 | SEQ ID NO:10206 | SEQ ID NO:18218 |
| **iPS:435405** | **21 225 150B7** | NA | AAGTCCAGCCAGAATGTTTTATACAGCTCCCACAATAACAACTACTTAGCT | TGGGCATCTACCCGGAAATCC | CAGCAATATTATAGTACTCCATTCACT |

**FIGURE 49**

| | | | | | |
|---|---|---|---|---|---|
| | **21-225_150B7** | | SEQ ID NO:2195 | SEQ ID NO:10207 | SEQ ID NO:18219 |
| | | AA | KSSQNVLYSSHNNNYLA | WASTRKS | QQYYSTPFT |
| | | | SEQ ID NO:2196 | SEQ ID NO:10208 | SEQ ID NO:18220 |
| **iPS:435407** | **21-225_150E7** | NA | CGGGCAAGTCAGGGCATTAG AAATGATTTAGGC | GCTGCATCCAATTTGCAA AGT | CTACAGCATAATAGTTACCC GCTCACT |
| | | | SEQ ID NO:2197 | SEQ ID NO:10209 | SEQ ID NO:18221 |
| | | AA | RASQGIRNDLG | AASNLQS | LQHNSYPLT |
| | | | SEQ ID NO:2198 | SEQ ID NO:10210 | SEQ ID NO:18222 |
| **iPS:435409** | **21-225_150G8** | NA | CGGGCGAGTCAGGGCATTAG CCATTATTTAGCC | GTTGCATCCAGTTTGCAA AAT | CAACAGTATAATAATTACCC GCTCACT |
| | | | SEQ ID NO:2199 | SEQ ID NO:10211 | SEQ ID NO:18223 |
| | | AA | RASQGISHYLA | VASSLQN | QQYNNYPLT |
| | | | SEQ ID NO:2200 | SEQ ID NO:10212 | SEQ ID NO:18224 |
| **iPS:435413** | **21-225_150B11** | NA | AAGTCTAGTCAGAGCCTCGT GCATGGTGATGGAAAGACCT ATTTGTAT | GAAGTTTCCAACCGGTTC TCT | ATGCAAAGTATACAGCTTCC GTGGACG |
| | | | SEQ ID NO:2201 | SEQ ID NO:10213 | SEQ ID NO:18225 |
| | | AA | KSSQSLVHGDGKTYLY | EVSNRFS | MQSIQLPWT |
| | | | SEQ ID NO:2202 | SEQ ID NO:10214 | SEQ ID NO:18226 |
| **iPS:435415** | **21-225_150C11** | NA | CGGGCAAGTCAGAGCATTAG CAGCTATTTAAAT | ACTGCATCCAGTTTGCAA AGT | CAACAGAGTTACAGTATTTA CACT |
| | | | SEQ ID NO:2203 | SEQ ID NO:10215 | SEQ ID NO:18227 |
| | | AA | RASQSISSYLN | TASSLQS | QQSYSIYT |
| | | | SEQ ID NO:2204 | SEQ ID NO:10216 | SEQ ID NO:18228 |
| **iPS:435417** | **21-225_150D11** | NA | AAGTCTAGTCAGAGCCTCCT GCATAGTGAGGGAAAGACC TATTTGTAT | GAAGTTTCCTACCGGTTC TCT | ATGCAAGGTATACAGCTTCC GCTCACT |
| | | | SEQ ID NO:2205 | SEQ ID NO:10217 | SEQ ID NO:18229 |
| | | AA | KSSQSLLHSEGKTYLY | EVSYRFS | MQGIQLPLT |
| | | | SEQ ID NO:2206 | SEQ ID NO:10218 | SEQ ID NO:18230 |

FIGURE 49

| | | | | | |
|---|---|---|---|---|---|
| **iPS:435419** | **21-225_150C12** | NA | CGGGCAAGTCAGAGCATTAGCGACTATTTAAAT | ACTACATCCAGTTTGCAAAGT | CAACAGAGTTTCAGTACCCCCACT |
| | | | SEQ ID NO:2207 | SEQ ID NO:10219 | SEQ ID NO:18231 |
| | | AA | RASQSISDYLN | TTSSLQS | QQSFSTPT |
| | | | SEQ ID NO:2208 | SEQ ID NO:10220 | SEQ ID NO:18232 |
| **iPS:435421** | **21-225_151F1** | NA | AGGGCCAGTCAGAGTATTAACATCAATATAGCC | GGTGCATCCACCAGGGCCACT | CAGCAGTATAATGACTGGCCTCCGTGGACG |
| | | | SEQ ID NO:2209 | SEQ ID NO:10221 | SEQ ID NO:18233 |
| | | AA | RASQSININIA | GASTRAT | QQYNDWPPWT |
| | | | SEQ ID NO:2210 | SEQ ID NO:10222 | SEQ ID NO:18234 |
| **iPS:435423** | **21-225_151G5** | NA | AAGTCTAGTCAGCGCCTCCTGCATGGTGATGGAAAGACCTATTTGTAT | GAAGTTTCCAACCGGTTCTCT | ATGCAAAGTATACAGGTTCCGTGGACG |
| | | | SEQ ID NO:2211 | SEQ ID NO:10223 | SEQ ID NO:18235 |
| | | AA | KSSQRLLHGDGKTYLY | EVSNRFS | MQSIQVPWT |
| | | | SEQ ID NO:2212 | SEQ ID NO:10224 | SEQ ID NO:18236 |
| **iPS:435425** | **21-225_151B12** | NA | CGGGCAAGTCAGAGCATTAGCAACTTTTTAAAT | ACTGCATCCAGTTTGGAAAGT | CAACAGAGTTACAGTACCCCCACT |
| | | | SEQ ID NO:2213 | SEQ ID NO:10225 | SEQ ID NO:18237 |
| | | AA | RASQSISNFLN | TASSLES | QQSYSTPT |
| | | | SEQ ID NO:2214 | SEQ ID NO:10226 | SEQ ID NO:18238 |
| **iPS:435427** | **21-225_151C9** | NA | CGGGCGAGTCAGGGCATTAGCAAGTATTTAGCC | GATGCATCCAGGTTGCAAAGT | CATCAGTATAAACATTACCCGATCACC |
| | | | SEQ ID NO:2215 | SEQ ID NO:10227 | SEQ ID NO:18239 |
| | | AA | RASQGISKYLA | DASRLQS | HQYKHYPIT |
| | | | SEQ ID NO:2216 | SEQ ID NO:10228 | SEQ ID NO:18240 |
| **iPS:435429** | **21-225_151A10** | NA | AAGTCTAGTCAGAGCCTCCTGCATGGTGATGGAAAGACCTATTTGTAT | GAAGTTTCCCACCGGTTCTCT | ATGCAAAGTATACAGATTCCGTGGACG |
| | | | SEQ ID NO:2217 | SEQ ID NO:10229 | SEQ ID NO:18241 |

FIGURE 49

| | | | | | |
|---|---|---|---|---|---|
| | | AA | KSSQSLLHGDGKTYLY | EVSHRFS | MQSIQIPWT |
| | | | SEQ ID NO:2218 | SEQ ID NO:10230 | SEQ ID NO:18242 |
| **iPS:435431** | **21-225_152D2** | NA | CGGGCAAGTCAGAGCATTAG CGACTATTTAAAT | ACTACATCCAGTTTGCAA AGT | CAACAGAGTTACAGTACCCC CACT |
| | | | SEQ ID NO:2219 | SEQ ID NO:10231 | SEQ ID NO:18243 |
| | | AA | RASQSISDYLN | TTSSLQS | QQSYSTPT |
| | | | SEQ ID NO:2220 | SEQ ID NO:10232 | SEQ ID NO:18244 |
| **iPS:435433** | **21-225_152E3** | NA | AAGTCCAGCCAGAGTGTTTT ACACAGCTCCAACAATTACA ACTACTTAGTT | TGGGCATCTACCCGGGA ATCC | CAGCAATATTACAGTACTCC ATTCACT |
| | | | SEQ ID NO:2221 | SEQ ID NO:10233 | SEQ ID NO:18245 |
| | | AA | KSSQSVLHSSNNYNYLV | WASTRES | QQYYSTPFT |
| | | | SEQ ID NO:2222 | SEQ ID NO:10234 | SEQ ID NO:18246 |
| **iPS:435435** | **21-225_152H3** | NA | AAGTCCAGCCAGAGTGTTTT GCACAGCTCCAACAATTACA ACTACTTAACT | TGGGCATCTACCCGGAA ATCC | CAGCAATATTATAGTACTCC GTGCAGT |
| | | | SEQ ID NO:2223 | SEQ ID NO:10235 | SEQ ID NO:18247 |
| | | AA | KSSQSVLHSSNNYNYLT | WASTRKS | QQYYSTPCS |
| | | | SEQ ID NO:2224 | SEQ ID NO:10236 | SEQ ID NO:18248 |
| **iPS:435437** | **21-225_152F4** | NA | AAGTCCAGCCAGAGTGTTTT ATACAGCTCCAACAATTACA ACTACTTAGCT | TGGGCATCTACCCGGAA ATCC | CAGCAATATTTTAATACTCC TCCCACT |
| | | | SEQ ID NO:2225 | SEQ ID NO:10237 | SEQ ID NO:18249 |
| | | AA | KSSQSVLYSSNNYNYLA | WASTRKS | QQYFNTPPT |
| | | | SEQ ID NO:2226 | SEQ ID NO:10238 | SEQ ID NO:18250 |
| **iPS:435439** | **21-225_152G4** | NA | CGGGCAAGTCAGAGCATTAG CGACTATTTAAAT | ACTACATCCAGTTTGCAA AGT | CAACAGAGTTACAGTACCCC CACT |
| | | | SEQ ID NO:2227 | SEQ ID NO:10239 | SEQ ID NO:18251 |
| | | AA | RASQSISDYLN | TTSSLQS | QQSYSTPT |
| | | | SEQ ID NO:2228 | SEQ ID NO:10240 | SEQ ID NO:18252 |

FIGURE 49

| | | | | | |
|---|---|---|---|---|---|
| **iPS:435441** | **21-225_152F6** | NA | AAGTCTAGTCAGAGCCTCCGGCATGGTGATGGAAAGACCTATTTGACT | GAAATTTCCAAGCGGTTCACT | ATGCAAAGTATACAGGTTCCGTGGACG |
| | | | SEQ ID NO:2229 | SEQ ID NO:10241 | SEQ ID NO:18253 |
| | | AA | KSSQSLRHGDGKTYLT | EISKRFT | MQSIQVPWT |
| | | | SEQ ID NO:2230 | SEQ ID NO:10242 | SEQ ID NO:18254 |
| **iPS:435443** | **21-225_152E7** | NA | AGGGCCAGTCAGAGTGTTATCAGCAGCTACTTAGCC | GGTGTATCTAGTAGGGCCACT | CAACAATATGGTAGGTCACCATTCAAT |
| | | | SEQ ID NO:2231 | SEQ ID NO:10243 | SEQ ID NO:18255 |
| | | AA | RASQSVISSYLA | GVSSRAT | QQYGRSPFN |
| | | | SEQ ID NO:2232 | SEQ ID NO:10244 | SEQ ID NO:18256 |
| **iPS:435445** | **21-225_152F7** | NA | CGGGCAAGTCAGGGCATTAGAAATGATTTAGGC | GCTACATCCAGTTTGCAAAGT | CTACAGCATTATAATTTCCCGTACAGT |
| | | | SEQ ID NO:2233 | SEQ ID NO:10245 | SEQ ID NO:18257 |
| | | AA | RASQGIRNDLG | ATSSLQS | LQHYNFPYS |
| | | | SEQ ID NO:2234 | SEQ ID NO:10246 | SEQ ID NO:18258 |
| **iPS:435447** | **21-225_152H7** | NA | CGGGCGAGTCAGGATATTAGCAACTGGTTAGCC | GCTGCATCCAGTTTGCAAGGT | CAACAGACTGACAGTTTCCCATTCACT |
| | | | SEQ ID NO:2235 | SEQ ID NO:10247 | SEQ ID NO:18259 |
| | | AA | RASQDISNWLA | AASSLQG | QQTDSFPFT |
| | | | SEQ ID NO:2236 | SEQ ID NO:10248 | SEQ ID NO:18260 |
| **iPS:435449** | **21-225_152H9** | NA | CGGGCAAGTCAGGGCATTAGAAATGATTTAGGC | GCTGCATCCAGTTTGCAAAGT | CTACAGCATAGTAATTACCCGCTCACT |
| | | | SEQ ID NO:2237 | SEQ ID NO:10249 | SEQ ID NO:18261 |
| | | AA | RASQGIRNDLG | AASSLQS | LQHSNYPLT |
| | | | SEQ ID NO:2238 | SEQ ID NO:10250 | SEQ ID NO:18262 |
| **iPS:435451** | **21-225_152D10** | NA | AAGTCCAGCCAGAGTGTTTTATACAGCTCCAACAATAAGAACTACTTAGCT | TGGGCATCTACCCGGGAATCC | CAGCAATATTATCGTAGTCCTAGT |
| | | | SEQ ID NO:2239 | SEQ ID NO:10251 | SEQ ID NO:18263 |

FIGURE 49

| | | | | | |
|---|---|---|---|---|---|
| | | AA | KSSQSVLYSSNNKNYLA | WASTRES | QQYYRSPS |
| | | | SEQ ID NO:2240 | SEQ ID NO:10252 | SEQ ID NO:18264 |
| **iPS:435453** | **21-225_152G10** | NA | CGGGCGAGTCAGGGTATTAGCAACTGGTTAGCC | GCTGCATCCAGTTTGCAAAGT | CAACAGACTGACAGTTTCCCATTCACT |
| | | | SEQ ID NO:2241 | SEQ ID NO:10253 | SEQ ID NO:18265 |
| | | AA | RASQGISNWLA | AASSLQS | QQTDSFPFT |
| | | | SEQ ID NO:2242 | SEQ ID NO:10254 | SEQ ID NO:18266 |
| **iPS:435455** | **21-225_152B11** | NA | CGGGCAAGTCAGAGCATTAGCGACTATTTAAAT | ACTACATCCAGTTTGCAAAGT | CAACAGAGTTACAGTACCCCCACT |
| | | | SEQ ID NO:2243 | SEQ ID NO:10255 | SEQ ID NO:18267 |
| | | AA | RASQSISDYLN | TTSSLQS | QQSYSTPT |
| | | | SEQ ID NO:2244 | SEQ ID NO:10256 | SEQ ID NO:18268 |
| **iPS:435457** | **21-225_152C11** | NA | AAGTCTAGTCAGAGCCTCCTGCATGGTGATGGAAAGACCTATTTATAT | GAAGTTTCCAACCGGTTCTCT | ATGCAAAGTATACAGATTCCGTGGACG |
| | | | SEQ ID NO:2245 | SEQ ID NO:10257 | SEQ ID NO:18269 |
| | | AA | KSSQSLLHGDGKTYLY | EVSNRFS | MQSIQIPWT |
| | | | SEQ ID NO:2246 | SEQ ID NO:10258 | SEQ ID NO:18270 |
| **iPS:435459** | **21-225_152E12** | NA | ACGTCCAGCCAGAGTATTTTACACAGCTCCAATAATTACAACTACTTAGCT | TGGGCATCTACCCGGGAATCC | CAACAATATTATAGTGGTCCGTGCAGT |
| | | | SEQ ID NO:2247 | SEQ ID NO:10259 | SEQ ID NO:18271 |
| | | AA | TSSQSILHSSNNYNYLA | WASTRES | QQYYSGPCS |
| | | | SEQ ID NO:2248 | SEQ ID NO:10260 | SEQ ID NO:18272 |
| **iPS:435461** | **21-225_153A1** | NA | CGGGCGAGTCAGGTCATTAGCAATTATTTAGCC | GCTGCATCCAGTTTGCGAAGT | CAACAGTATCATAGTTACCCATTCACT |
| | | | SEQ ID NO:2249 | SEQ ID NO:10261 | SEQ ID NO:18273 |
| | | AA | RASQVISNYLA | AASSLRS | QQYHSYPFT |
| | | | SEQ ID NO:2250 | SEQ ID NO:10262 | SEQ ID NO:18274 |

**FIGURE 49**

| | | | | | |
|---|---|---|---|---|---|
| **iPS:435463** | **21-225_153D2** | NA | AAGTCTAGTCAGAGCCTCCGGCATGGTGATGGAAAGACCTATTTGACT | GAAGTTTCCAAGCGGTTCACT | ATGCAAAGTATACAGGTTCCGTGGACG |
| | | | SEQ ID NO:2251 | SEQ ID NO:10263 | SEQ ID NO:18275 |
| | | AA | KSSQSLRHGDGKTYLT | EVSKRFT | MQSIQVPWT |
| | | | SEQ ID NO:2252 | SEQ ID NO:10264 | SEQ ID NO:18276 |
| **iPS:435465** | **21-225_153A6** | NA | AGGGCCAGTCAGAGTGTTATCAGCAGCTACTTAGCC | GGTGTATCTAGTAGGGCCACT | CAACAATATGGTAGGTCACCATTCAAT |
| | | | SEQ ID NO:2253 | SEQ ID NO:10265 | SEQ ID NO:18277 |
| | | AA | RASQSVISSYLA | GVSSRAT | QQYGRSPFN |
| | | | SEQ ID NO:2254 | SEQ ID NO:10266 | SEQ ID NO:18278 |
| **iPS:435467** | **21-225_153B9** | NA | AAGTCCAGCCAGAGTGTTTTATACAGCTCCAACAATAAGAACTACTTAGCT | TGGGCATCTACCCGGGAATTT | CAGCAATATAATCGTAGTCTTAGT |
| | | | SEQ ID NO:2255 | SEQ ID NO:10267 | SEQ ID NO:18279 |
| | | AA | KSSQSVLYSSNNKNYLA | WASTREF | QQYNRSLS |
| | | | SEQ ID NO:2256 | SEQ ID NO:10268 | SEQ ID NO:18280 |
| **iPS:435469** | **21-225_153G9** | NA | AAGTCTAGTCAGAGCCTCCTGCATAGTGATGGAAAGACCTATTTGTAT | GAAGTTTCCAACCGGTTCTCT | ATGCAAAATATAAAGTATCCGCTCACT |
| | | | SEQ ID NO:2257 | SEQ ID NO:10269 | SEQ ID NO:18281 |
| | | AA | KSSQSLLHSDGKTYLY | EVSNRFS | MQNIKYPLT |
| | | | SEQ ID NO:2258 | SEQ ID NO:10270 | SEQ ID NO:18282 |
| **iPS:435471** | **21-225_153F11** | NA | AAGTCCAGCCAGAGTGTTTTATACAGCTCCAACAATTACAAGTACTTAGCT | TGGGCATCTACCCGGAAATCC | CAGCAATATTATAGTACTCCGTGCAGT |
| | | | SEQ ID NO:2259 | SEQ ID NO:10271 | SEQ ID NO:18283 |
| | | AA | KSSQSVLYSSNNYKYLA | WASTRKS | QQYYSTPCS |
| | | | SEQ ID NO:2260 | SEQ ID NO:10272 | SEQ ID NO:18284 |

FIGURE 49

| | | | | | |
|---|---|---|---|---|---|
| **iPS:435475** | **21-225_154H6** | NA | AAGTCCAGCCAGAGTGTTTTACACAGTTCCAACAATTACAACTATTTAGCT | TGGACATCTACCCGGAAATCC | CAGCATTATTATAGTACTCCGTGCAGT |
| | | | SEQ ID NO:2261 | SEQ ID NO:10273 | SEQ ID NO:18285 |
| | | AA | KSSQSVLHSSNNYNYLA | WTSTRKS | QHYYSTPCS |
| | | | SEQ ID NO:2262 | SEQ ID NO:10274 | SEQ ID NO:18286 |
| **iPS:435479** | **21-225_154E9** | NA | CGGGCGAGTCAGGATATTAGCAACTGGTTAGCC | GCTGCATCCAGTTTGCAAAGT | CAACAGGGTAACAGTTTCCCGCTCACT |
| | | | SEQ ID NO:2263 | SEQ ID NO:10275 | SEQ ID NO:18287 |
| | | AA | RASQDISNWLA | AASSLQS | QQGNSFPLT |
| | | | SEQ ID NO:2264 | SEQ ID NO:10276 | SEQ ID NO:18288 |
| **iPS:435481** | **21-225_154A11** | NA | AGGTCAAGCCAGAGTGTTTTACACAGCTCCAACAATTATAACTACTTAGCT | TGGGCATCTAAACGGGATTCC | CAGCAATATTTTAGTTCTCCTCGGACG |
| | | | SEQ ID NO:2265 | SEQ ID NO:10277 | SEQ ID NO:18289 |
| | | AA | RSSQSVLHSSNNYNYLA | WASKRDS | QQYFSSPRT |
| | | | SEQ ID NO:2266 | SEQ ID NO:10278 | SEQ ID NO:18290 |
| **iPS:435483** | **21-225_155A4** | NA | CGGGCGAGTCAGGGTATTAGCAACTGGTTAGCC | GCTGCATCCAGTTTGCAAGGT | CACCAGACTGACAGTTTCCCATTCACT |
| | | | SEQ ID NO:2267 | SEQ ID NO:10279 | SEQ ID NO:18291 |
| | | AA | RASQGISNWLA | AASSLQG | HQTDSFPFT |
| | | | SEQ ID NO:2268 | SEQ ID NO:10280 | SEQ ID NO:18292 |
| **iPS:435485** | **21-225_155B4** | NA | CGGGCGAGTCAGGATATTAGCAACTGGTTAGCC | GCTGCATCCAGTTTGCAAGGT | CACCAGACTGACAGTTTCCCATTCACT |
| | | | SEQ ID NO:2269 | SEQ ID NO:10281 | SEQ ID NO:18293 |
| | | AA | RASQDISNWLA | AASSLQG | HQTDSFPFT |
| | | | SEQ ID NO:2270 | SEQ ID NO:10282 | SEQ ID NO:18294 |
| **iPS:435487** | **21-225_155C4** | NA | CGGGCAAGTCAGAGCATTAGCAGCTATTTAAAT | ACTGCATCCAGTTTGCAAAGT | CAACAGAGTTACAGTACCCCCACT |
| | | | SEQ ID NO:2271 | SEQ ID NO:10283 | SEQ ID NO:18295 |

**FIGURE 49**

| | | | | | |
|---|---|---|---|---|---|
| | | AA | RASQSISSYLN | TASSLQS | QQSYSTPT |
| | | | SEQ ID NO:2272 | SEQ ID NO:10284 | SEQ ID NO:18296 |
| **iPS:435489** | **21-225_155A5** | NA | AAGTCTAGTCAGAGCCTCCTGCATGGTGATGGAAAGACCTATTTGTAT | GAAGTTTCCAATCGGTTCTCT | ATGCAAAGTATACAGGTTCCGTGGACG |
| | | | SEQ ID NO:2273 | SEQ ID NO:10285 | SEQ ID NO:18297 |
| | | AA | KSSQSLLHGDGKTYLY | EVSNRFS | MQSIQVPWT |
| | | | SEQ ID NO:2274 | SEQ ID NO:10286 | SEQ ID NO:18298 |
| **iPS:435491** | **21-225_155E5** | NA | AAGTCCAGCCAGAGTGTTTTATCCAGCTCCAACAATAATAATTATTTAGCT | TGGGCATCTACCCGGAAATCC | CAGCAATATTATAGTACTCCGTGCAGT |
| | | | SEQ ID NO:2275 | SEQ ID NO:10287 | SEQ ID NO:18299 |
| | | AA | KSSQSVLSSSNNNNYLA | WASTRKS | QQYYSTPCS |
| | | | SEQ ID NO:2276 | SEQ ID NO:10288 | SEQ ID NO:18300 |
| **iPS:435495** | **21-225_155B6** | NA | AAGTCCAGCCAGAGTGTTTTACACAGCTCCAATAATTACAACTACTTAGCT | TGGACATCTACCCGGGAATCC | CAACAATATTATAGTACTCCGTGCAGT |
| | | | SEQ ID NO:2277 | SEQ ID NO:10289 | SEQ ID NO:18301 |
| | | AA | KSSQSVLHSSNNYNYLA | WTSTRES | QQYYSTPCS |
| | | | SEQ ID NO:2278 | SEQ ID NO:10290 | SEQ ID NO:18302 |
| **iPS:435497** | **21-225_155H9** | NA | AGGGCCAGTCAGAGTGTTAGTAGTAACTTAGCC | GGTGCATCCACCAGGGCCACT | CAGCAGTATGATGACTGGCCTCCGTGGACG |
| | | | SEQ ID NO:2279 | SEQ ID NO:10291 | SEQ ID NO:18303 |
| | | AA | RASQSVSSNLA | GASTRAT | QQYDDWPPWT |
| | | | SEQ ID NO:2280 | SEQ ID NO:10292 | SEQ ID NO:18304 |
| **iPS:435499** | **21-225_156G1** | NA | CGGGCAAGTCAGGGCATTAGAAATGATTTAGGC | GCTGCATCCAGTTTGCAAAGT | CTACAGCATAGTAATTACCCGCTCACT |
| | | | SEQ ID NO:2281 | SEQ ID NO:10293 | SEQ ID NO:18305 |
| | | AA | RASQGIRNDLG | AASSLQS | LQHSNYPLT |
| | | | SEQ ID NO:2282 | SEQ ID NO:10294 | SEQ ID NO:18306 |

FIGURE 49

| | | | | | |
|---|---|---|---|---|---|
| **iPS:435501** | **21-225_156H1** | NA | AAGTCCAGCCAGAGTGTTTTATACAGCTCCAACAATAACAACTACTTAGCT | TGGGCATCTACCCGGGAATCC | CACCAATATTATAGTACTCCGTGCAGT |
| | | | SEQ ID NO:2283 | SEQ ID NO:10295 | SEQ ID NO:18307 |
| | | AA | KSSQSVLYSSNNNNYLA | WASTRES | HQYYSTPCS |
| | | | SEQ ID NO:2284 | SEQ ID NO:10296 | SEQ ID NO:18308 |
| **iPS:435503** | **21-225_156E4** | NA | CGGGCAAGTCAGAGCATTAGCAGCTATTTAAAT | ACTGCATCCAGTTTGCAAAGT | CAACAGAGTTACAGTGCCCCCACT |
| | | | SEQ ID NO:2285 | SEQ ID NO:10297 | SEQ ID NO:18309 |
| | | AA | RASQSISSYLN | TASSLQS | QQSYSAPT |
| | | | SEQ ID NO:2286 | SEQ ID NO:10298 | SEQ ID NO:18310 |
| **iPS:435505** | **21-225_157C1** | NA | CGGGCGAGTCAGGGCATTAGCAATTATTTAGCC | GCTGCATCCAGTTTGCTAAGT | CAACAGTATAATAGTTTTCCATTCACT |
| | | | SEQ ID NO:2287 | SEQ ID NO:10299 | SEQ ID NO:18311 |
| | | AA | RASQGISNYLA | AASSLLS | QQYNSFPFT |
| | | | SEQ ID NO:2288 | SEQ ID NO:10300 | SEQ ID NO:18312 |
| **iPS:435509** | **21-225_157H1** | NA | CGGGCGAGTCAGGACATTAGCAATTATTTAGTC | GCTGCATCCAGTTTGCAAAGT | CAACAATATCATAGTTACCCATTCACT |
| | | | SEQ ID NO:2289 | SEQ ID NO:10301 | SEQ ID NO:18313 |
| | | AA | RASQDISNYLV | AASSLQS | QQYHSYPFT |
| | | | SEQ ID NO:2290 | SEQ ID NO:10302 | SEQ ID NO:18314 |
| **iPS:435511** | **21-225_157C3** | NA | CGGGCAAGTCAGGGCATTAGAAATGATTTAGGC | GCTGCATCCAGTTTGCAAAGT | CTACAGCATAATAGTTACCCATTCACT |
| | | | SEQ ID NO:2291 | SEQ ID NO:10303 | SEQ ID NO:18315 |
| | | AA | RASQGIRNDLG | AASSLQS | LQHNSYPFT |
| | | | SEQ ID NO:2292 | SEQ ID NO:10304 | SEQ ID NO:18316 |
| **iPS:435513** | **21-225_157F3** | NA | CGGGCAAGTCAGAACATTAGCAGTTATTTAAAT | ACTGCATCCAGTTTGCAAAGT | CAACAGAGTTACAATACCCCCACGTGGACG |
| | | | SEQ ID NO:2293 | SEQ ID NO:10305 | SEQ ID NO:18317 |
| | | AA | RASQNISSYLN | TASSLQS | QQSYNTPTWT |

FIGURE 49

| | | | | | |
|---|---|---|---|---|---|
| | | | SEQ ID NO:2294 | SEQ ID NO:10306 | SEQ ID NO:18318 |
| **iPS:435515** | **21-225_157E4** | NA | CGGGCGAGTCAGGGCATTAGCAATTATTTAGCC | GCTGCATCCAGTTTGCGAAGT | CAACAGTATCATAGTTATCCATTCACT |
| | | | SEQ ID NO:2295 | SEQ ID NO:10307 | SEQ ID NO:18319 |
| | | AA | RASQGISNYLA | AASSLRS | QQYHSYPFT |
| | | | SEQ ID NO:2296 | SEQ ID NO:10308 | SEQ ID NO:18320 |
| **iPS:435521** | **21-225_157H4** | NA | CGGGCAAGTCAGGGCATTAGAAATGATTTAGGC | CCTGCATCCAGTTTACAAACT | CTACAGGATAATAGTTACCCATTCACT |
| | | | SEQ ID NO:2297 | SEQ ID NO:10309 | SEQ ID NO:18321 |
| | | AA | RASQGIRNDLG | PASSLQT | LQDNSYPFT |
| | | | SEQ ID NO:2298 | SEQ ID NO:10310 | SEQ ID NO:18322 |
| **iPS:435523** | **21-225_157G5** | NA | CGGGCGAGTCAGGGCATTAACAATTATTTAGCC | GCTGCATCCAGTTTACAAAGT | CAACAGTATAATAGTTATCCATTCACT |
| | | | SEQ ID NO:2299 | SEQ ID NO:10311 | SEQ ID NO:18323 |
| | | AA | RASQGINNYLA | AASSLQS | QQYNSYPFT |
| | | | SEQ ID NO:2300 | SEQ ID NO:10312 | SEQ ID NO:18324 |
| **iPS:435525** | **21-225_157E7** | NA | CGGGCAAGTCAGAGCTTTAGCAGCTATTTAAAT | GCTGCATCCAGTTTGCAAAGT | CAAGAGAGTTATAGTATCCGCTTCGCC |
| | | | SEQ ID NO:2301 | SEQ ID NO:10313 | SEQ ID NO:18325 |
| | | AA | RASQSFSSYLN | AASSLQS | QESYSIRFA |
| | | | SEQ ID NO:2302 | SEQ ID NO:10314 | SEQ ID NO:18326 |
| **iPS:435527** | **21-225_157G7** | NA | CGGGCAAGTCAGGGCATTAGAAATGATTTAGGC | GTTGCATCCAGTTTGCAAAGT | ATACAGGATAATAGTCACCCATTCACT |
| | | | SEQ ID NO:2303 | SEQ ID NO:10315 | SEQ ID NO:18327 |
| | | AA | RASQGIRNDLG | VASSLQS | IQDNSHPFT |
| | | | SEQ ID NO:2304 | SEQ ID NO:10316 | SEQ ID NO:18328 |
| **iPS:435529** | **21-225_157H7** | NA | CGGGCGAGTCAGGACATTAGCAATTTTTTAGCC | ACTGCATCCAGTTTGCAAAGT | CAACAGTATCATAGTTACCCGATCACC |
| | | | SEQ ID NO:2305 | SEQ ID NO:10317 | SEQ ID NO:18329 |
| | | AA | RASQDISNFLA | TASSLQS | QQYHSYPIT |

**FIGURE 49**

| | | | | | |
|---|---|---|---|---|---|
| | | | SEQ ID NO:2306 | SEQ ID NO:10318 | SEQ ID NO:18330 |
| **iPS:435531** | **21-225_157G8** | NA | AAGTCTAGTCAGAGCCTCCT GCATGGTGATGGAAAGACCT ATTTATAT | GAAATTTCCAAGCGGTT CTCT | ATGCAAAGTATACAGGTTCC GTGGACG |
| | | | SEQ ID NO:2307 | SEQ ID NO:10319 | SEQ ID NO:18331 |
| | | AA | KSSQSLLHGDGKTYLY | EISKRFS | MQSIQVPWT |
| | | | SEQ ID NO:2308 | SEQ ID NO:10320 | SEQ ID NO:18332 |
| **iPS:435533** | **21-225_157H8** | NA | CGGGCAAGTCAGGGCATTAG AAATGATTTAGGC | GCTGCATCCAGTTTGCAA AGT | CTACAGCATAATAGTTACCC ATTCACT |
| | | | SEQ ID NO:2309 | SEQ ID NO:10321 | SEQ ID NO:18333 |
| | | AA | RASQGIRNDLG | AASSLQS | LQHNSYPFT |
| | | | SEQ ID NO:2310 | SEQ ID NO:10322 | SEQ ID NO:18334 |
| **iPS:435535** | **21-225_157H10** | NA | CGGGCGAGTCAGGGCATTAC CAATTATTTAGCC | ACTGCATCCAATTTGCAA AGT | CAACAGTATCATAGTTACCC ATTCACT |
| | | | SEQ ID NO:2311 | SEQ ID NO:10323 | SEQ ID NO:18335 |
| | | AA | RASQGITNYLA | TASNLQS | QQYHSYPFT |
| | | | SEQ ID NO:2312 | SEQ ID NO:10324 | SEQ ID NO:18336 |
| **iPS:435537** | **21-225_157H12** | NA | CGGGCAAGTCAGGGCATTAG AAATGATTTTGGC | GCTGCATCCAGTTTACAG AGT | CTACAGCATTATAGTTACCC ATTCACT |
| | | | SEQ ID NO:2313 | SEQ ID NO:10325 | SEQ ID NO:18337 |
| | | AA | RASQGIRNDFG | AASSLQS | LQHYSYPFT |
| | | | SEQ ID NO:2314 | SEQ ID NO:10326 | SEQ ID NO:18338 |
| **iPS:435539** | **21-225_158G1** | NA | CGGGCAAGTCAGGGCATTAG AAATGATTTAGGC | ACTGCATCCAATTTGCAA AGT | CTACAGCATAATAGTTACCC GTGGACG |
| | | | SEQ ID NO:2315 | SEQ ID NO:10327 | SEQ ID NO:18339 |
| | | AA | RASQGIRNDLG | TASNLQS | LQHNSYPWT |
| | | | SEQ ID NO:2316 | SEQ ID NO:10328 | SEQ ID NO:18340 |
| **iPS:435543** | **21-225_158D4** | NA | CGGGCAAGTCAGGACATTAG AAAGGATTTAGGC | GCTGCATCCAGTTTGCAA AGT | CTACAGCATTATAGTTACCC TCGGACG |
| | | | SEQ ID NO:2317 | SEQ ID NO:10329 | SEQ ID NO:18341 |

FIGURE 49

| | | | | | |
|---|---|---|---|---|---|
| | | AA | RASQDIRKDLG | AASSLQS | LQHYSYPRT |
| | | | SEQ ID NO:2318 | SEQ ID NO:10330 | SEQ ID NO:18342 |
| **iPS:435545** | **21-225_158F4** | NA | CGGGCAAGTCAGAACATTAG AAAGTATTTACAT | ACTGCATCCACTTTACAA AGT | CAACAGAGTTACAATATTTC ATTCACT |
| | | | SEQ ID NO:2319 | SEQ ID NO:10331 | SEQ ID NO:18343 |
| | | AA | RASQNIRKYLH | TASTLQS | QQSYNISFT |
| | | | SEQ ID NO:2320 | SEQ ID NO:10332 | SEQ ID NO:18344 |
| **iPS:435547** | **21-225_158F5** | NA | CGGGCAAGTCAGGGCATTAG AAATGATTTAGGC | GCTGCATCCAGTTTGCAA AGT | CTACAGGATAATAGTCACCC ATTCACT |
| | | | SEQ ID NO:2321 | SEQ ID NO:10333 | SEQ ID NO:18345 |
| | | AA | RASQGIRNDLG | AASSLQS | LQDNSHPFT |
| | | | SEQ ID NO:2322 | SEQ ID NO:10334 | SEQ ID NO:18346 |
| **iPS:435549** | **21-225_158H5** | NA | CGGGCAAGTCAGGGCATGA GAATTGATTTAGGG | CGTGCATCCAGTTTGCAA AGT | GTACAGCATAATAGTTACCC TCTCACT |
| | | | SEQ ID NO:2323 | SEQ ID NO:10335 | SEQ ID NO:18347 |
| | | AA | RASQGMRIDLG | RASSLQS | VQHNSYPLT |
| | | | SEQ ID NO:2324 | SEQ ID NO:10336 | SEQ ID NO:18348 |
| **iPS:435551** | **21-225_158H6** | NA | CGGGCAAGTCAGGGCATTAG AAGTGATTTAGGC | ACTGCATCCAGTTTGCAA AGT | CTACAGCATAATAGTTACCC ATTCACT |
| | | | SEQ ID NO:2325 | SEQ ID NO:10337 | SEQ ID NO:18349 |
| | | AA | RASQGIRSDLG | TASSLQS | LQHNSYPFT |
| | | | SEQ ID NO:2326 | SEQ ID NO:10338 | SEQ ID NO:18350 |
| **iPS:435553** | **21-225_158G8** | NA | CGGGCAAGTCAGGACATTAG AAATGATTTAGGC | ACTGCATCCAGTTTGCAA AGT | CTACAGCATAATAGTTACCC ATTCACT |
| | | | SEQ ID NO:2327 | SEQ ID NO:10339 | SEQ ID NO:18351 |
| | | AA | RASQDIRNDLG | TASSLQS | LQHNSYPFT |
| | | | SEQ ID NO:2328 | SEQ ID NO:10340 | SEQ ID NO:18352 |
| **iPS:435557** | **21 225 158B12** | NA | AAGTCCAGCCAGAATGTTTT ACACAGCTCCAACAATAACA ACTACTTAACT | TGGGCATCTACCCGGGA ATCC | CAGCAATATTATAGTACTCC TCCGACG |

**FIGURE 49**

| | | | | | |
|---|---|---|---|---|---|
| | **21-225_158B12** | | SEQ ID NO:2329 | SEQ ID NO:10341 | SEQ ID NO:18353 |
| | | AA | KSSQNVLHSSNNNNYLT | WASTRES | QQYYSTPPT |
| | | | SEQ ID NO:2330 | SEQ ID NO:10342 | SEQ ID NO:18354 |
| **iPS:435559** | **21-225_158H12** | NA | CGGGCGAGTCAGGGCATTAA CAATTATTTAGCC | GCTGCATCCAGTTTACAA AGT | CAACAGTATCATAGTTACCC ATTCACT |
| | | | SEQ ID NO:2331 | SEQ ID NO:10343 | SEQ ID NO:18355 |
| | | AA | RASQGINNYLA | AASSLQS | QQYHSYPFT |
| | | | SEQ ID NO:2332 | SEQ ID NO:10344 | SEQ ID NO:18356 |
| **iPS:435561** | **21-225_159F1** | NA | CGGGCAAGTCAGCGCGTCAG AAATGATTTAGGC | GATGCATCCAATTTGCA AAGT | CTACAGCATCATAGTTTCCC GATCACC |
| | | | SEQ ID NO:2333 | SEQ ID NO:10345 | SEQ ID NO:18357 |
| | | AA | RASQRVRNDLG | DASNLQS | LQHHSFPIT |
| | | | SEQ ID NO:2334 | SEQ ID NO:10346 | SEQ ID NO:18358 |
| **iPS:435563** | **21-225_159H2** | NA | CGGGCAAGTCAGAGCATTAG CAAATATTTAAAT | GCTACATCCAATTTGCAA AGT | CAACAGAGTTACAGTCTCCC GGTCACT |
| | | | SEQ ID NO:2335 | SEQ ID NO:10347 | SEQ ID NO:18359 |
| | | AA | RASQSISKYLN | ATSNLQS | QQSYSLPVT |
| | | | SEQ ID NO:2336 | SEQ ID NO:10348 | SEQ ID NO:18360 |
| **iPS:435565** | **21-225_159C4** | NA | CAGGCGAGTCAGGACATTAG CGACTATTTAAAT | GATGCCTCCACTTTGGAA ACA | CAACAATATGATAATCTCCC GATCACC |
| | | | SEQ ID NO:2337 | SEQ ID NO:10349 | SEQ ID NO:18361 |
| | | AA | QASQDISDYLN | DASTLET | QQYDNLPIT |
| | | | SEQ ID NO:2338 | SEQ ID NO:10350 | SEQ ID NO:18362 |
| **iPS:435569** | **21-225_159C5** | NA | CGGACAAGTCAGGGCATTAG AAATGATTTAGGC | GCTGCATCCAGTTTGCAA AGT | CTACAGCATAATAGTTATCC ATTCACT |
| | | | SEQ ID NO:2339 | SEQ ID NO:10351 | SEQ ID NO:18363 |
| | | AA | RTSQGIRNDLG | AASSLQS | LQHNSYPFT |
| | | | SEQ ID NO:2340 | SEQ ID NO:10352 | SEQ ID NO:18364 |
| **iPS:435571** | | NA | CGGGCAAGTCAGGACATTAG AAAGGATTTAGGG | GCTGCATCCAGTTTGCAA AGT | CTACAGCATCATAGTTATCC TCGGACG |

FIGURE 49

| | | | | | |
|---|---|---|---|---|---|
| | **21-225_159C8** | | SEQ ID NO:2341 | SEQ ID NO:10353 | SEQ ID NO:18365 |
| | | AA | RASQDIRKDLG | AASSLQS | LQHHSYPRT |
| | | | SEQ ID NO:2342 | SEQ ID NO:10354 | SEQ ID NO:18366 |
| **iPS:435573** | **21-225_159D8** | NA | CGGGCAAGTCGGGACATTGG AAATGATTTAGGC | GCTGCATCCAGTTTGCAA AGT | CTACAGCATTATAGTTACCC TCGGACG |
| | | | SEQ ID NO:2343 | SEQ ID NO:10355 | SEQ ID NO:18367 |
| | | AA | RASRDIGNDLG | AASSLQS | LQHYSYPRT |
| | | | SEQ ID NO:2344 | SEQ ID NO:10356 | SEQ ID NO:18368 |
| **iPS:435575** | **21-225_159H11** | NA | CGGGCGAGTCAGGGCATTAG CAAATATTTAGTC | GCTGCATCCAGTCTGCA AAGT | CAACAGTATAATAGTTACCC ATTCACT |
| | | | SEQ ID NO:2345 | SEQ ID NO:10357 | SEQ ID NO:18369 |
| | | AA | RASQGISKYLV | AASSLQS | QQYNSYPFT |
| | | | SEQ ID NO:2346 | SEQ ID NO:10358 | SEQ ID NO:18370 |
| **iPS:435577** | **21-225_160B1** | NA | AAGTCTAGTCAGAGCCTCCT GCATGGTGATGGGAAGACCT ATTTCTAT | GAAGTATCCAAGCGGTT CTCT | ATGCAAAGTATACAGATTCC GTGGACG |
| | | | SEQ ID NO:2347 | SEQ ID NO:10359 | SEQ ID NO:18371 |
| | | AA | KSSQSLLHGDGKTYFY | EVSKRFS | MQSIQIPWT |
| | | | SEQ ID NO:2348 | SEQ ID NO:10360 | SEQ ID NO:18372 |
| **iPS:435579** | **21-225_160G1** | NA | CGGGCGAGTCAGGACATTAA CAATTATTTAGCC | GCTTCATCCAGTTTGCAA AGT | CAACAATATCATAGTTACCC ATTCACT |
| | | | SEQ ID NO:2349 | SEQ ID NO:10361 | SEQ ID NO:18373 |
| | | AA | RASQDINNYLA | ASSSLQS | QQYHSYPFT |
| | | | SEQ ID NO:2350 | SEQ ID NO:10362 | SEQ ID NO:18374 |
| **iPS:435581** | **21-225_160H1** | NA | CGGGCAAGTCAGGACATTAG AAATGATTTAGGC | GCTGCATCCAGTTTGCAG AAT | CTACAGCATAATAGTTTCCC GTGGACG |
| | | | SEQ ID NO:2351 | SEQ ID NO:10363 | SEQ ID NO:18375 |
| | | AA | RASQDIRNDLG | AASSLQN | LQHNSFPWT |
| | | | SEQ ID NO:2352 | SEQ ID NO:10364 | SEQ ID NO:18376 |

FIGURE 49

| | | | | | |
|---|---|---|---|---|---|
| **iPS:435583** | **21-225_160F2** | NA | CGGGCAAGTCAGGGCATTAG AAATGATTTAGGC | ACTGCATCCAATTTGCAA AGT | CTACAGCATAATAGTTACCC GTGGACG |
| | | | SEQ ID NO:2353 | SEQ ID NO:10365 | SEQ ID NO:18377 |
| | | AA | RASQGIRNDLG | TASNLQS | LQHNSYPWT |
| | | | SEQ ID NO:2354 | SEQ ID NO:10366 | SEQ ID NO:18378 |
| **iPS:435585** | **21-225_160G3** | NA | CGGGCGAGTCAGGACATTAA CAATTATTTAGCC | GCTTCATCCAGTTTGCAA AGT | CAACAATATCATAGTTACCC ATTCACT |
| | | | SEQ ID NO:2355 | SEQ ID NO:10367 | SEQ ID NO:18379 |
| | | AA | RASQDINNYLA | ASSSLQS | QQYHSYPFT |
| | | | SEQ ID NO:2356 | SEQ ID NO:10368 | SEQ ID NO:18380 |
| **iPS:435587** | **21-225_160H3** | NA | CGGGCAAGTCAGGGCATTAG AAATGATTTAGGC | GCTGCATCCAGTTTGCAA AGT | CTACAGCATAGTAATTACCC GCTCACT |
| | | | SEQ ID NO:2357 | SEQ ID NO:10369 | SEQ ID NO:18381 |
| | | AA | RASQGIRNDLG | AASSLQS | LQHSNYPLT |
| | | | SEQ ID NO:2358 | SEQ ID NO:10370 | SEQ ID NO:18382 |
| **iPS:435589** | **21-225_160A4** | NA | AAGTCCAGCCAGAGTGTTTT ACACAGCTCCAACAATAATA ACTACTTAGCT | TGGGCATCTACCCGGGA ATCC | CAGCAATATTATAATAGTCC GTGCAGT |
| | | | SEQ ID NO:2359 | SEQ ID NO:10371 | SEQ ID NO:18383 |
| | | AA | KSSQSVLHSSNNNNYLA | WASTRES | QQYYNSPCS |
| | | | SEQ ID NO:2360 | SEQ ID NO:10372 | SEQ ID NO:18384 |
| **iPS:435591** | **21-225_160C4** | NA | CGGGCAAGTCAGGACATTAG AAAGGATTTAGGG | GCTGCATCCAGTTTGCAA AGT | CTACAGCATTATAGTTATCC TCGGACG |
| | | | SEQ ID NO:2361 | SEQ ID NO:10373 | SEQ ID NO:18385 |
| | | AA | RASQDIRKDLG | AASSLQS | LQHYSYPRT |
| | | | SEQ ID NO:2362 | SEQ ID NO:10374 | SEQ ID NO:18386 |
| **iPS:435593** | **21-225_160F4** | NA | CGGGCAAGTCAGGGCATTAG AAATGATTTAGGC | GTTGCATCCAGTTTGCAA AGT | ATACAGGATAATAGTCACCC ATTCACT |
| | | | SEQ ID NO:2363 | SEQ ID NO:10375 | SEQ ID NO:18387 |
| | | AA | RASQGIRNDLG | VASSLQS | IQDNSHPFT |

FIGURE 49

| | | | | | |
|---|---|---|---|---|---|
| | | | SEQ ID NO:2364 | SEQ ID NO:10376 | SEQ ID NO:18388 |
| **iPS:435595** | **21-225_160H4** | NA | CGGGCGAGTCAGGACATTAGTAATTATTTAGTC | GTTGCATCCAGTTTGCAAAGT | CAACAGTATAATAGTTACCCTCTCACT |
| | | | SEQ ID NO:2365 | SEQ ID NO:10377 | SEQ ID NO:18389 |
| | | AA | RASQDISNYLV | VASSLQS | QQYNSYPLT |
| | | | SEQ ID NO:2366 | SEQ ID NO:10378 | SEQ ID NO:18390 |
| **iPS:435599** | **21-225_160B10** | NA | CGGGCAAGTCAGGGCATTAGAAATGATTTAGGC | GCTGCATCCAGTTTGCAAAGT | CTACAGCATAGTAGTTACCCGCTCACT |
| | | | SEQ ID NO:2367 | SEQ ID NO:10379 | SEQ ID NO:18391 |
| | | AA | RASQGIRNDLG | AASSLQS | LQHSSYPLT |
| | | | SEQ ID NO:2368 | SEQ ID NO:10380 | SEQ ID NO:18392 |
| **iPS:435601** | **21-225_160G10** | NA | AAGTCTAGTCAGAGCCTCCTGCACGGTGATGGAAAGACCTATTTGTAT | GAAGTTTCCAAACGGTTCTCT | ATGCAAAGTATACAGCTTCCGTGGACG |
| | | | SEQ ID NO:2369 | SEQ ID NO:10381 | SEQ ID NO:18393 |
| | | AA | KSSQSLLHGDGKTYLY | EVSKRFS | MQSIQLPWT |
| | | | SEQ ID NO:2370 | SEQ ID NO:10382 | SEQ ID NO:18394 |
| **iPS:435605** | **21-225_161A4** | NA | AGGTCCAGTCAGAGTGTTAACAGCAACTTAGCC | GGTGCATCCATCAGGGCCACT | CAGCAGTATAATAACTGGTGGACG |
| | | | SEQ ID NO:2371 | SEQ ID NO:10383 | SEQ ID NO:18395 |
| | | AA | RSSQSVNSNLA | GASIRAT | QQYNNWWT |
| | | | SEQ ID NO:2372 | SEQ ID NO:10384 | SEQ ID NO:18396 |
| **iPS:435607** | **21-225_161G4** | NA | CAGGCGAGTCAGGACATTTACAATTATTTAAAT | GATGCATCCAATTTGGAAACA | CAACAGTATGATATTCTCCCGATCACC |
| | | | SEQ ID NO:2373 | SEQ ID NO:10385 | SEQ ID NO:18397 |
| | | AA | QASQDIYNYLN | DASNLET | QQYDILPIT |
| | | | SEQ ID NO:2374 | SEQ ID NO:10386 | SEQ ID NO:18398 |
| **iPS:435609** | **21-225_161F7** | NA | CGGGCAAGTCAGGGCATTAGAAATGATTTGGGC | GCTGCATCCACTTTGCAAAGT | CTACTATATATTCGTTACCCATTCACT |
| | | | SEQ ID NO:2375 | SEQ ID NO:10387 | SEQ ID NO:18399 |

**FIGURE 49**

| | | | | | |
|---|---|---|---|---|---|
| | | AA | RASQGIRNDLG | AASTLQS | LLYIRYPFT |
| | | | SEQ ID NO:2376 | SEQ ID NO:10388 | SEQ ID NO:18400 |
| **iPS:435611** | **21-225_161F10** | NA | CAGGCGAGTCAGGACATTTACAACCATTTAAGT | GATGCATCCAATTGGGAAACA | CAACAGTATGAAAATCTCCCGCTCACC |
| | | | SEQ ID NO:2377 | SEQ ID NO:10389 | SEQ ID NO:18401 |
| | | AA | QASQDIYNHLS | DASNWET | QQYENLPLT |
| | | | SEQ ID NO:2378 | SEQ ID NO:10390 | SEQ ID NO:18402 |
| **iPS:435613** | **21-225_161D11** | NA | CGGGCAAGTCAGGGCATTAGAAATGATTTGGGC | GCTGCATCCACTTTGCAAAGT | CTACAATATAATCGTTACCCATTCACT |
| | | | SEQ ID NO:2379 | SEQ ID NO:10391 | SEQ ID NO:18403 |
| | | AA | RASQGIRNDLG | AASTLQS | LQYNRYPFT |
| | | | SEQ ID NO:2380 | SEQ ID NO:10392 | SEQ ID NO:18404 |
| **iPS:435615** | **21-225_161G12** | NA | CGGGCAAGTCAGGACATTAGAAAGGATTTAGGG | GCTGCATCCAGTTTGCAAAGT | CTACAGCATCATAGTTATCCTCGGACG |
| | | | SEQ ID NO:2381 | SEQ ID NO:10393 | SEQ ID NO:18405 |
| | | AA | RASQDIRKDLG | AASSLQS | LQHHSYPRT |
| | | | SEQ ID NO:2382 | SEQ ID NO:10394 | SEQ ID NO:18406 |
| **iPS:435617** | **21-225_162F2** | NA | CGGGCAAGTCAGGGCATTAGAAATGATTTAGGC | GCTGCATCCAGTTTGCAAAGT | ATACAGGATAATAGTCACCCATTCACT |
| | | | SEQ ID NO:2383 | SEQ ID NO:10395 | SEQ ID NO:18407 |
| | | AA | RASQGIRNDLG | AASSLQS | IQDNSHPFT |
| | | | SEQ ID NO:2384 | SEQ ID NO:10396 | SEQ ID NO:18408 |
| **iPS:435621** | **21-225_162H3** | NA | CGGGCAAGTCAGGGCATTAGAAATGATTTAGGC | GCTGCATCCAGTTTGCAAAGT | CTACAGGATAATAGTCACCCATTCACT |
| | | | SEQ ID NO:2385 | SEQ ID NO:10397 | SEQ ID NO:18409 |
| | | AA | RASQGIRNDLG | AASSLQS | LQDNSHPFT |
| | | | SEQ ID NO:2386 | SEQ ID NO:10398 | SEQ ID NO:18410 |
| **iPS:435623** | **21 225 162D5** | NA | AAGTCCAACCATAGTGTTTTATACAGGTCCAACAATAATCAATACTTAGCT | CGGACATCTATCCGGAAATCC | CAGCAATATTATAGTACTCCTCCCACT |

FIGURE 49

| | | | | | |
|---|---|---|---|---|---|
| | 21-225_162D5 | | SEQ ID NO:2387 | SEQ ID NO:10399 | SEQ ID NO:18411 |
| | | AA | KSNHSVLYRSNNNQYLA | RTSIRKS | QQYYSTPPT |
| | | | SEQ ID NO:2388 | SEQ ID NO:10400 | SEQ ID NO:18412 |
| iPS:435627 | 21-225_162F6 | NA | AAGTCCAGCCAGAATGTTTTACACAGCTCCAACAATAACAACTACTTAACT | TGGGCATCTACCCGGGAATCC | CAGCAATATTATAGTACTCCTCCGACG |
| | | | SEQ ID NO:2389 | SEQ ID NO:10401 | SEQ ID NO:18413 |
| | | AA | KSSQNVLHSSNNNNYLT | WASTRES | QQYYSTPPT |
| | | | SEQ ID NO:2390 | SEQ ID NO:10402 | SEQ ID NO:18414 |
| iPS:435629 | 21-225_162H6 | NA | AAGTCTACTCAGAGCCTCCTGCATGGTGATGGAAAGACCTATTTGTAT | GAAGTTTCCAAGCGGTTCTCT | AAGCAAAGTATACAGCTTCCGTGGACG |
| | | | SEQ ID NO:2391 | SEQ ID NO:10403 | SEQ ID NO:18415 |
| | | AA | KSTQSLLHGDGKTYLY | EVSKRFS | KQSIQLPWT |
| | | | SEQ ID NO:2392 | SEQ ID NO:10404 | SEQ ID NO:18416 |
| iPS:435635 | 21-225_163F1 | NA | CGGGCGAGTCAGGACATTAGCAATTATTTAGCC | GCTGCATCCAGTTTGCAAAGT | CAACAGTATAATAGTTACCCATTCACT |
| | | | SEQ ID NO:2393 | SEQ ID NO:10405 | SEQ ID NO:18417 |
| | | AA | RASQDISNYLA | AASSLQS | QQYNSYPFT |
| | | | SEQ ID NO:2394 | SEQ ID NO:10406 | SEQ ID NO:18418 |
| iPS:435637 | 21-225_163E2 | NA | CGGGCAAGTCAGGACATTAGAAATGATTTAGGC | CCTGCATCCAGTTTGCAAAGT | CTACAGCATAATAGTCACCCATTCACT |
| | | | SEQ ID NO:2395 | SEQ ID NO:10407 | SEQ ID NO:18419 |
| | | AA | RASQDIRNDLG | PASSLQS | LQHNSHPFT |
| | | | SEQ ID NO:2396 | SEQ ID NO:10408 | SEQ ID NO:18420 |
| iPS:435639 | 21-225_163G6 | NA | CGGGCGAGTCAGGACATTAGCAATTATTTAGTC | GCTGCATCCAGTTTGCTAAGT | CAACAGTATCATAGTTACCCGCTCACT |
| | | | SEQ ID NO:2397 | SEQ ID NO:10409 | SEQ ID NO:18421 |
| | | AA | RASQDISNYLV | AASSLLS | QQYHSYPLT |
| | | | SEQ ID NO:2398 | SEQ ID NO:10410 | SEQ ID NO:18422 |

FIGURE 49

| | | | | | |
|---|---|---|---|---|---|
| **iPS:435641** | **21-225_163F9** | NA | CGGGCAAGTCAGGACATTAG AGATGATTTAGGC | CCTGCATCCAGTTTGCAA AGT | CTACAGGATAATAGTTACCC ATTCACT |
| | | | SEQ ID NO:2399 | SEQ ID NO:10411 | SEQ ID NO:18423 |
| | | AA | RASQDIRDDLG | PASSLQS | LQDNSYPFT |
| | | | SEQ ID NO:2400 | SEQ ID NO:10412 | SEQ ID NO:18424 |
| **iPS:435643** | **21-225_163G10** | NA | CGGGCAAGTCAGGACATTAG AAATAATTTAGGC | CCTGCATCCAGTTTGCAA AGT | CTACAGGATTATAGTTACCC ATTCACT |
| | | | SEQ ID NO:2401 | SEQ ID NO:10413 | SEQ ID NO:18425 |
| | | AA | RASQDIRNNLG | PASSLQS | LQDYSYPFT |
| | | | SEQ ID NO:2402 | SEQ ID NO:10414 | SEQ ID NO:18426 |
| **iPS:435649** | **21-225_165H2** | NA | AAGTCCAGCCAGAGTGTTTT ACACAGCTCCAACAATAAGA ACTACTTAACT | TGGGCATCTACCCGAGA ATCC | CAGCAATCTTATAGTATTCC TCCCACT |
| | | | SEQ ID NO:2403 | SEQ ID NO:10415 | SEQ ID NO:18427 |
| | | AA | KSSQSVLHSSNNKNYLT | WASTRES | QQSYSIPPT |
| | | | SEQ ID NO:2404 | SEQ ID NO:10416 | SEQ ID NO:18428 |
| **iPS:435653** | **21-225_166H12** | NA | CGGGCGAGTCAGGACATTAG CCATTATTTAGCC | GCTGCATCCAGTTTGCAA AGT | CAACAGTATAATAGTTTCCC GCTCACT |
| | | | SEQ ID NO:2405 | SEQ ID NO:10417 | SEQ ID NO:18429 |
| | | AA | RASQDISHYLA | AASSLQS | QQYNSFPLT |
| | | | SEQ ID NO:2406 | SEQ ID NO:10418 | SEQ ID NO:18430 |
| **iPS:435655** | **21-225_167E2** | NA | AAGTCTAGTCAGAGCCTCCT GCACGGTGATGGGAAGACCT ATTTGTAT | GAAGTTTCCAAACGGTT CTCT | ATGCAAAGCATACAGCTTCC GTGGACG |
| | | | SEQ ID NO:2407 | SEQ ID NO:10419 | SEQ ID NO:18431 |
| | | AA | KSSQSLLHGDGKTYLY | EVSKRFS | MQSIQLPWT |
| | | | SEQ ID NO:2408 | SEQ ID NO:10420 | SEQ ID NO:18432 |
| **iPS:435657** | **21 225 167H10** | NA | AAGTCTAGTCAGAGCCTCCT GCACGGTGATGGAAAGACCT ATTTGTAT | GAAGTTTCCAAACGGTT CTCT | ATGCAAAGCATACAGCTTCC GTGGACG |

**FIGURE 49**

| | | | | | |
|---|---|---|---|---|---|
| | **21-225_167H10** | | SEQ ID NO:2409 | SEQ ID NO:10421 | SEQ ID NO:18433 |
| | | AA | KSSQSLLHGDGKTYLY | EVSKRFS | MQSIQLPWT |
| | | | SEQ ID NO:2410 | SEQ ID NO:10422 | SEQ ID NO:18434 |
| **iPS:435659** | **21-225_167D12** | NA | CGGGCGAGTCAGGGCATTAA CAATTATTTAGCC | GCTGCATCCAGTTTGCAA AGT | CAACAGTATAATAGTTATCC ATTCACT |
| | | | SEQ ID NO:2411 | SEQ ID NO:10423 | SEQ ID NO:18435 |
| | | AA | RASQGINNYLA | AASSLQS | QQYNSYPFT |
| | | | SEQ ID NO:2412 | SEQ ID NO:10424 | SEQ ID NO:18436 |
| **iPS:435663** | **21-225_169B1** | NA | CGGGCTAGTCAGGGCATTAG AAATGATTTAGGC | GCTGAATCCAGTTTGCA AAGT | CTACAGCATTATAGTTACCC GCTCACT |
| | | | SEQ ID NO:2413 | SEQ ID NO:10425 | SEQ ID NO:18437 |
| | | AA | RASQGIRNDLG | AESSLQS | LQHYSYPLT |
| | | | SEQ ID NO:2414 | SEQ ID NO:10426 | SEQ ID NO:18438 |
| **iPS:435665** | **21-225_169F2** | NA | AAGTCCAGCCAGAGTGTTTT ATACATCTCCAACAATAAAA ACTACTTAGCT | TGGGCATCTACCCGGGA ATCC | CAGCAATATTATCGTGCTCC CACC |
| | | | SEQ ID NO:2415 | SEQ ID NO:10427 | SEQ ID NO:18439 |
| | | AA | KSSQSVLYISNNKNYLA | WASTRES | QQYYRAPT |
| | | | SEQ ID NO:2416 | SEQ ID NO:10428 | SEQ ID NO:18440 |
| **iPS:435667** | **21-225_169E3** | NA | AGGTCTAGTCAGAGCCTCCT GCATAATAATGGATACAAGT ATTTGGAT | TTGGGTTCTAATCGGGCC TCC | ATGCAAGTTCTACAAACTCC GTGGACG |
| | | | SEQ ID NO:2417 | SEQ ID NO:10429 | SEQ ID NO:18441 |
| | | AA | RSSQSLLHNNGYKYLD | LGSNRAS | MQVLQTPWT |
| | | | SEQ ID NO:2418 | SEQ ID NO:10430 | SEQ ID NO:18442 |
| **iPS:435669** | **21-225_169F9** | NA | CGGGCAAGTCAGGGCATTAG AAATGATTTAGGC | GCTGCATCCAGTTTGCAA AGT | CTACAGCATTATAGTTACCC GCTCACT |
| | | | SEQ ID NO:2419 | SEQ ID NO:10431 | SEQ ID NO:18443 |
| | | AA | RASQGIRNDLG | AASSLQS | LQHYSYPLT |
| | | | SEQ ID NO:2420 | SEQ ID NO:10432 | SEQ ID NO:18444 |

FIGURE 49

| | | | | | |
|---|---|---|---|---|---|
| **iPS:435671** | **21-225_169H5** | NA | AAGTCCAGCCAGAGTGTTTTATACATCTCCAACAATAAAAACTACTTAGCT | TGGGCATCTACCCGGGAATCC | CAGCAATATTATCGTGCTCCCACC |
| | | | SEQ ID NO:2421 | SEQ ID NO:10433 | SEQ ID NO:18445 |
| | | AA | KSSQSVLYISNNKNYLA | WASTRES | QQYYRAPT |
| | | | SEQ ID NO:2422 | SEQ ID NO:10434 | SEQ ID NO:18446 |
| **iPS:435673** | **21-225_169E6** | NA | AGGTCTAGTCAGAGCCTCCTGCATAATAATGGATACAAGTATTTGGAT | TTGGGTTCTAATCGGGCCTCC | ATGCAAGTTCTACAAACTCCGTGGACG |
| | | | SEQ ID NO:2423 | SEQ ID NO:10435 | SEQ ID NO:18447 |
| | | AA | RSSQSLLHNNGYKYLD | LGSNRAS | MQVLQTPWT |
| | | | SEQ ID NO:2424 | SEQ ID NO:10436 | SEQ ID NO:18448 |
| **iPS:435675** | **21-225_169D7** | NA | CGGGCAAGTCAGGGCATTAGAAATGATTTAGGC | GCTGCATCCAGTTTGCAAAGT | CTACAGCATCATAGTTGCCCGTGGACG |
| | | | SEQ ID NO:2425 | SEQ ID NO:10437 | SEQ ID NO:18449 |
| | | AA | RASQGIRNDLG | AASSLQS | LQHHSCPWT |
| | | | SEQ ID NO:2426 | SEQ ID NO:10438 | SEQ ID NO:18450 |
| **iPS:435677** | **21-225_169C10** | NA | CGGGCGAGTCAGGACATTAGCAATTATTTAGCC | TCTGCATCCAGTTTGCAAAGT | CAACAATCTGATAGTTACCCTCTCACT |
| | | | SEQ ID NO:2427 | SEQ ID NO:10439 | SEQ ID NO:18451 |
| | | AA | RASQDISNYLA | SASSLQS | QQSDSYPLT |
| | | | SEQ ID NO:2428 | SEQ ID NO:10440 | SEQ ID NO:18452 |
| **iPS:435679** | **21-225_169D10** | NA | CGGGCGAGTCAGGACATTAGCAATTATTTAGCC | GCTGCATCCAGTTTGCAAAGT | CAACAGTATCATAGTTACCCATTCACT |
| | | | SEQ ID NO:2429 | SEQ ID NO:10441 | SEQ ID NO:18453 |
| | | AA | RASQDISNYLA | AASSLQS | QQYHSYPFT |
| | | | SEQ ID NO:2430 | SEQ ID NO:10442 | SEQ ID NO:18454 |
| **iPS:435681** | **21-225_169D11** | NA | CGGGCAAGTCAGGGCATTAGAGATGATTTAGGC | GCTGCATCCAGTTTGCAAAGT | CTTCAGCATTATAGTTACCCTCGGACG |
| | | | SEQ ID NO:2431 | SEQ ID NO:10443 | SEQ ID NO:18455 |

FIGURE 49

| | | | | | |
|---|---|---|---|---|---|
| | | AA | RASQGIRDDLG | AASSLQS | LQHYSYPRT |
| | | | SEQ ID NO:2432 | SEQ ID NO:10444 | SEQ ID NO:18456 |
| **iPS:435683** | **21-225_170A1** | NA | AAGTCTAGTCAGAGCCTCCTGCATGGTGATGGAAAGACCTATTTGTTT | GAAGTTTCCAACCGGTTCTCT | ATGCAAAGTATTCAGCTTCCGTGGACG |
| | | | SEQ ID NO:2433 | SEQ ID NO:10445 | SEQ ID NO:18457 |
| | | AA | KSSQSLLHGDGKTYLF | EVSNRFS | MQSIQLPWT |
| | | | SEQ ID NO:2434 | SEQ ID NO:10446 | SEQ ID NO:18458 |
| **iPS:435685** | **21-225_170E1** | NA | CGGGCGAGTCAGGGCATTAGCAATTATTTAGCC | GCTGCATCCAGTTTGCAAAGT | CAACAGTATCATAGTTACCCATTCACT |
| | | | SEQ ID NO:2435 | SEQ ID NO:10447 | SEQ ID NO:18459 |
| | | AA | RASQGISNYLA | AASSLQS | QQYHSYPFT |
| | | | SEQ ID NO:2436 | SEQ ID NO:10448 | SEQ ID NO:18460 |
| **iPS:435687** | **21-225_170H1** | NA | CGGGCAAGTCAGGGCATTAGAAATGATTTAGGC | GCTACATCCAGTTTGCAAAGT | CTACAGCATAGTAGTAACCCGTGGACG |
| | | | SEQ ID NO:2437 | SEQ ID NO:10449 | SEQ ID NO:18461 |
| | | AA | RASQGIRNDLG | ATSSLQS | LQHSSNPWT |
| | | | SEQ ID NO:2438 | SEQ ID NO:10450 | SEQ ID NO:18462 |
| **iPS:435689** | **21-225_170F3** | NA | CGGGCAAGTCGGGGCATTAGAAATGATTTAGGC | GCTGCATCCAGTTTGCAAAGT | CTACAGCATTATAGTTACCCTCGGACG |
| | | | SEQ ID NO:2439 | SEQ ID NO:10451 | SEQ ID NO:18463 |
| | | AA | RASRGIRNDLG | AASSLQS | LQHYSYPRT |
| | | | SEQ ID NO:2440 | SEQ ID NO:10452 | SEQ ID NO:18464 |
| **iPS:435693** | **21-225_170G4** | NA | CGGGCAAGTCAGGGCATTAGAAATGATTTAGGC | GCTGCATCCACTTTGCAAAGT | CTACAGCATTATAGTTACCCGCTCACT |
| | | | SEQ ID NO:2441 | SEQ ID NO:10453 | SEQ ID NO:18465 |
| | | AA | RASQGIRNDLG | AASTLQS | LQHYSYPLT |
| | | | SEQ ID NO:2442 | SEQ ID NO:10454 | SEQ ID NO:18466 |
| **iPS:435695** | | NA | CGGGCAAGTCAGGGCATTAGAAATGATTTAGGC | GCTGCATCCAGTTTGCAAAAT | CTACAGCATTATAGTTTCCCGCTCACT |

FIGURE 49

| | | | | | |
|---|---|---|---|---|---|
| | **21-225_170D5** | | SEQ ID NO:2443 | SEQ ID NO:10455 | SEQ ID NO:18467 |
| | | AA | RASQGIRNDLG | AASSLQN | LQHYSFPLT |
| | | | SEQ ID NO:2444 | SEQ ID NO:10456 | SEQ ID NO:18468 |
| **iPS:435697** | **21-225_170G5** | NA | CGGGCAAGTCAGGGCATTAG AACTGATTTAGGC | ACTGCATCCAGTTTGCAA AGT | CTACAGCATTATAGTTACCC GCTCACT |
| | | | SEQ ID NO:2445 | SEQ ID NO:10457 | SEQ ID NO:18469 |
| | | AA | RASQGIRTDLG | TASSLQS | LQHYSYPLT |
| | | | SEQ ID NO:2446 | SEQ ID NO:10458 | SEQ ID NO:18470 |
| **iPS:435699** | **21-225_170D6** | NA | CGGGCGAGTCAGGACATTGG CAATTGTTTAGCC | TCTGCGTCCAGTTTGCAA AGT | CAACAATCTGATAGTTACCC TCTCACT |
| | | | SEQ ID NO:2447 | SEQ ID NO:10459 | SEQ ID NO:18471 |
| | | AA | RASQDIGNCLA | SASSLQS | QQSDSYPLT |
| | | | SEQ ID NO:2448 | SEQ ID NO:10460 | SEQ ID NO:18472 |
| **iPS:435701** | **21-225_170F6** | NA | AAGTCCAGCCAGAGTGTTTT ACACAGCTCCAACAATTACA ACTACTTAGCT | TGGGCATCTACCCGGAA ATCC | CAGCAATATTATAGTACTCC GTGGACG |
| | | | SEQ ID NO:2449 | SEQ ID NO:10461 | SEQ ID NO:18473 |
| | | AA | KSSQSVLHSSNNYNYLA | WASTRKS | QQYYSTPWT |
| | | | SEQ ID NO:2450 | SEQ ID NO:10462 | SEQ ID NO:18474 |
| **iPS:435703** | **21-225_170D11** | NA | CGGGCAAGTCAGGGCATTAG AAATGATTTAGGC | GCTGCATCCAGTTTGCAA AAT | CTACAGCATTATAGTTTCCC GCTCACT |
| | | | SEQ ID NO:2451 | SEQ ID NO:10463 | SEQ ID NO:18475 |
| | | AA | RASQGIRNDLG | AASSLQN | LQHYSFPLT |
| | | | SEQ ID NO:2452 | SEQ ID NO:10464 | SEQ ID NO:18476 |
| **iPS:435705** | **21-225_171C3** | NA | CGGGCAAGTCAGGGCATTAG AACTGATTTAGGC | ACTGCATCCAGTTTGCAA AGT | CTACAGCATTATAGTTACCC GCTCACT |
| | | | SEQ ID NO:2453 | SEQ ID NO:10465 | SEQ ID NO:18477 |
| | | AA | RASQGIRTDLG | TASSLQS | LQHYSYPLT |
| | | | SEQ ID NO:2454 | SEQ ID NO:10466 | SEQ ID NO:18478 |

FIGURE 49

| | | | | | |
|---|---|---|---|---|---|
| **iPS:435709** | **21-225_171A4** | NA | CGGGCAAGTCAGGGCATTAG AAATGATTTAGGC | ACTGCATCCAGTTTGCAA AGT | CTACAGCATTATAGTTACCC GCTCACT |
| | | | SEQ ID NO:2455 | SEQ ID NO:10467 | SEQ ID NO:18479 |
| | | AA | RASQGIRNDLG | TASSLQS | LQHYSYPLT |
| | | | SEQ ID NO:2456 | SEQ ID NO:10468 | SEQ ID NO:18480 |
| **iPS:435711** | **21-225_171G4** | NA | CGGGCGAGTCAGGGTGTTAA CGACTGGTTAGCC | GATGCATCAAGTTTGCA AAGT | CAACAGGCTAACAGTTTCCC GTGGACG |
| | | | SEQ ID NO:2457 | SEQ ID NO:10469 | SEQ ID NO:18481 |
| | | AA | RASQGVNDWLA | DASSLQS | QQANSFPWT |
| | | | SEQ ID NO:2458 | SEQ ID NO:10470 | SEQ ID NO:18482 |
| **iPS:435713** | **21-225_171D7** | NA | AGGTCTAGTCAGAGCCTCCT GTATCATAATGGATACAACT ATTTGGAT | GTGGGTTCTAATCGGGC CTCC | ATGCAAACTCTACAAACTCC GCTCACT |
| | | | SEQ ID NO:2459 | SEQ ID NO:10471 | SEQ ID NO:18483 |
| | | AA | RSSQSLLYHNGYNYLD | VGSNRAS | MQTLQTPLT |
| | | | SEQ ID NO:2460 | SEQ ID NO:10472 | SEQ ID NO:18484 |
| **iPS:435715** | **21-225_171A8** | NA | CGGGCGAGTCAGGGTATTAG CAACTGGTTAGCC | GCTGCATTCAGTTTGCAA GGT | CAACAGACTAACAGTTTCCC GTGGACG |
| | | | SEQ ID NO:2461 | SEQ ID NO:10473 | SEQ ID NO:18485 |
| | | AA | RASQGISNWLA | AAFSLQG | QQTNSFPWT |
| | | | SEQ ID NO:2462 | SEQ ID NO:10474 | SEQ ID NO:18486 |
| **iPS:435717** | **21-225_171A9** | NA | CGGGCGAGTCAGGATATTAC CACCTGGTTAGCC | GATGCATCCAGTTTGCA AAGT | CTACAGACTAACAGTTTCCC GTGGACG |
| | | | SEQ ID NO:2463 | SEQ ID NO:10475 | SEQ ID NO:18487 |
| | | AA | RASQDITTWLA | DASSLQS | LQTNSFPWT |
| | | | SEQ ID NO:2464 | SEQ ID NO:10476 | SEQ ID NO:18488 |
| **iPS:435719** | **21-225_171A11** | NA | CGGGCAAGTCAGGGCATTAG AAATAATTTAGGC | CCTGCATCCAGTTTGCAA AGT | CTACAGGATCATAGTTACCC ATTCACT |
| | | | SEQ ID NO:2465 | SEQ ID NO:10477 | SEQ ID NO:18489 |
| | | AA | RASQGIRNNLG | PASSLQS | LQDHSYPFT |

# FIGURE 49

| | | | | | |
|---|---|---|---|---|---|
| | | | SEQ ID NO:2466 | SEQ ID NO:10478 | SEQ ID NO:18490 |
| **iPS:435721** | **21-225_172B3** | NA | CGGGCTAGTCAGGGCATTAG AAATGATTTAGGC | GCTGAATCCAGTTTGCA AAGT | CTACAGCATTATAGTTACCC GCTCACT |
| | | | SEQ ID NO:2467 | SEQ ID NO:10479 | SEQ ID NO:18491 |
| | | AA | RASQGIRNDLG | AESSLQS | LQHYSYPLT |
| | | | SEQ ID NO:2468 | SEQ ID NO:10480 | SEQ ID NO:18492 |
| **iPS:435723** | **21-225_172B7** | NA | AAGTCTAGTCAGAGCCTCCT GCATGGTGATGGAAAGACCT ATTTCTAT | GAAGTTTCCCACCGGTTC TCT | ATGCAAAGTATACAGTTTCC GTGGACG |
| | | | SEQ ID NO:2469 | SEQ ID NO:10481 | SEQ ID NO:18493 |
| | | AA | KSSQSLLHGDGKTYFY | EVSHRFS | MQSIQFPWT |
| | | | SEQ ID NO:2470 | SEQ ID NO:10482 | SEQ ID NO:18494 |
| **iPS:435725** | **21-225_172G8** | NA | CGGGCAAGTCAGGGCGTTAG AAATGATTTAGGC | GCTGCATCCAGTTTGCAA AAT | CTACACCATTATAGTTTCCC GCTCACT |
| | | | SEQ ID NO:2471 | SEQ ID NO:10483 | SEQ ID NO:18495 |
| | | AA | RASQGVRNDLG | AASSLQN | LHHYSFPLT |
| | | | SEQ ID NO:2472 | SEQ ID NO:10484 | SEQ ID NO:18496 |
| **iPS:435727** | **21-225_172E11** | NA | AAGTCCAGCCAGAGTGTTTT ACACAGCTCCAACAATAACA ACTACTTAGCT | TGGGCATCTACTCGGGA ATCC | CAGCAATATTTTACTACTCC GTGCAGT |
| | | | SEQ ID NO:2473 | SEQ ID NO:10485 | SEQ ID NO:18497 |
| | | AA | KSSQSVLHSSNNNNYLA | WASTRES | QQYFTTPCS |
| | | | SEQ ID NO:2474 | SEQ ID NO:10486 | SEQ ID NO:18498 |
| **iPS:435729** | **21-225_173E7** | NA | CGTGCAAGTCAGACCATTAG CAACTATTTAAAT | GCTGCATCCAGTTTGCAA ATT | CAACAGAGTTACAGAACCCC TCAGTGGACG |
| | | | SEQ ID NO:2475 | SEQ ID NO:10487 | SEQ ID NO:18499 |
| | | AA | RASQTISNYLN | AASSLQI | QQSYRTPQWT |
| | | | SEQ ID NO:2476 | SEQ ID NO:10488 | SEQ ID NO:18500 |

FIGURE 49

| | | | | | |
|---|---|---|---|---|---|
| **iPS:435731** | **21-225_173A11** | NA | AAGTCTAGTCAGAGCCTCCTGCATGGTGATGGAAAGACCTATTTGTAT | GAAGTTTCCAACCGGTTCTCT | ATGCAAAGTATACAGGTTCCGTGGACG |
| | | | SEQ ID NO:2477 | SEQ ID NO:10489 | SEQ ID NO:18501 |
| | | AA | KSSQSLLHGDGKTYLY | EVSNRFS | MQSIQVPWT |
| | | | SEQ ID NO:2478 | SEQ ID NO:10490 | SEQ ID NO:18502 |
| **iPS:435733** | **21-225_173C11** | NA | AAGTCTAGTCAGAGCCTCCTGCATAGTGAGGGAAAGACCTACTTGTAT | GAAGTTTCCCACCGGTTCTCT | ATGCAAAGTATACAGCTTCTCACT |
| | | | SEQ ID NO:2479 | SEQ ID NO:10491 | SEQ ID NO:18503 |
| | | AA | KSSQSLLHSEGKTYLY | EVSHRFS | MQSIQLLT |
| | | | SEQ ID NO:2480 | SEQ ID NO:10492 | SEQ ID NO:18504 |
| **iPS:435735** | **21-225_173H12** | NA | CGGGCAAGTCAGGGCATTAGAAATGATTTAGGC | ACTACATCCAGTTTGCAAAGT | CTACAGCATTATAGTTTCCCGAACACT |
| | | | SEQ ID NO:2481 | SEQ ID NO:10493 | SEQ ID NO:18505 |
| | | AA | RASQGIRNDLG | TTSSLQS | LQHYSFPNT |
| | | | SEQ ID NO:2482 | SEQ ID NO:10494 | SEQ ID NO:18506 |
| **iPS:435737** | **21-225_174G5** | NA | AAGTCCAGCCAGAGTGTATTACACAGCTCCAACAATTACAACTACTTAACT | TGGGCATCTACCCGGGAATCC | CAGCAATATTATCGTACTCCGTGGACG |
| | | | SEQ ID NO:2483 | SEQ ID NO:10495 | SEQ ID NO:18507 |
| | | AA | KSSQSVLHSSNNYNYLT | WASTRES | QQYYRTPWT |
| | | | SEQ ID NO:2484 | SEQ ID NO:10496 | SEQ ID NO:18508 |
| **iPS:435739** | **21-225_174G7** | NA | CGGGCGAGTCAGGGTATTAGCAACTGGTTAGCC | GCTGCATTCAGTTTGCAAGGT | CAACAGACTAACAGTTTCCCGTGGACG |
| | | | SEQ ID NO:2485 | SEQ ID NO:10497 | SEQ ID NO:18509 |
| | | AA | RASQGISNWLA | AAFSLQG | QQTNSFPWT |
| | | | SEQ ID NO:2486 | SEQ ID NO:10498 | SEQ ID NO:18510 |
| **iPS:435741** | | NA | CGGGCAAGTCAGGGCATTAGAGATGATTTAGGC | GCTGCATCCAGTTTGCAAAGT | CTTCAGCATCATAGTTACCCTCGGACG |

FIGURE 49

| | | | | | |
|---|---|---|---|---|---|
| | **21-225_174G10** | | SEQ ID NO:2487 | SEQ ID NO:10499 | SEQ ID NO:18511 |
| | | AA | RASQGIRDDLG | AASSLQS | LQHHSYPRT |
| | | | SEQ ID NO:2488 | SEQ ID NO:10500 | SEQ ID NO:18512 |
| **iPS:435743** | **21-225_175G1** | NA | CGGGCAAGTCAGGGCATTAG AACTGATTTAGGC | ACTGCATCCAGTTTGCAA AGT | CTACAGCATTATAGTTACCC GCTCACT |
| | | | SEQ ID NO:2489 | SEQ ID NO:10501 | SEQ ID NO:18513 |
| | | AA | RASQGIRTDLG | TASSLQS | LQHYSYPLT |
| | | | SEQ ID NO:2490 | SEQ ID NO:10502 | SEQ ID NO:18514 |
| **iPS:435745** | **21-225_175G3** | NA | CGGGCGAGTCAGGACATTAG CAATGATTTAGCC | TCTGCATCCAGTTTGCAA AGT | CAACAATCTGATAGTTACCC TCTCACT |
| | | | SEQ ID NO:2491 | SEQ ID NO:10503 | SEQ ID NO:18515 |
| | | AA | RASQDISNDLA | SASSLQS | QQSDSYPLT |
| | | | SEQ ID NO:2492 | SEQ ID NO:10504 | SEQ ID NO:18516 |
| **iPS:435747** | **21-225_175C4** | NA | CGGGCGAGTCAGGGCATTGG GAATTATTTAGCC | GCTGCATCCGGTTTGCAA AGT | CAACAGTATTATAGTTACCC ATTCACT |
| | | | SEQ ID NO:2493 | SEQ ID NO:10505 | SEQ ID NO:18517 |
| | | AA | RASQGIGNYLA | AASGLQS | QQYYSYPFT |
| | | | SEQ ID NO:2494 | SEQ ID NO:10506 | SEQ ID NO:18518 |
| **iPS:435749** | **21-225_175C10** | NA | CGGGCGAGTCAGGGTATTAC CGACTGGTTAGCC | GCTGCATCCAGTTTGCAA AGT | CAACAGACTAACAGTTTCCC GTGGACG |
| | | | SEQ ID NO:2495 | SEQ ID NO:10507 | SEQ ID NO:18519 |
| | | AA | RASQGITDWLA | AASSLQS | QQTNSFPWT |
| | | | SEQ ID NO:2496 | SEQ ID NO:10508 | SEQ ID NO:18520 |
| **iPS:435751** | **21-225_175D10** | NA | AAGTCCAGCCAGAGTGTTTT ATACAGCTCCAACAATAACA ACTACTTAGCT | TGGACATCTACCCGGGA ATCC | CAGCAATATTATAGTACTCC TCCGACG |
| | | | SEQ ID NO:2497 | SEQ ID NO:10509 | SEQ ID NO:18521 |
| | | AA | KSSQSVLYSSNNNNYLA | WTSTRES | QQYYSTPPT |
| | | | SEQ ID NO:2498 | SEQ ID NO:10510 | SEQ ID NO:18522 |

FIGURE 49

| | | | | | |
|---|---|---|---|---|---|
| **iPS:435753** | **21-225_175G10** | NA | CGGGCAAGTCAGACCATTGGCAACTATTTAAAT | GCTGCATCCAGTTTGCACAGT | CAACAGAGTTACAGAACCCCTCAGTGGACG |
| | | | SEQ ID NO:2499 | SEQ ID NO:10511 | SEQ ID NO:18523 |
| | | AA | RASQTIGNYLN | AASSLHS | QQSYRTPQWT |
| | | | SEQ ID NO:2500 | SEQ ID NO:10512 | SEQ ID NO:18524 |
| **iPS:435755** | **21-225_176H4** | NA | AAGTCTAGTCAGAGCCTCCTGCATGGTGATGGAAAGACCTATTTGTAT | GAAGTTTCCAACCGGTTCTCT | ATGCAAAGTATACAGATTCCGTGGACG |
| | | | SEQ ID NO:2501 | SEQ ID NO:10513 | SEQ ID NO:18525 |
| | | AA | KSSQSLLHGDGKTYLY | EVSNRFS | MQSIQIPWT |
| | | | SEQ ID NO:2502 | SEQ ID NO:10514 | SEQ ID NO:18526 |
| **iPS:435759** | **21-225_176E6** | NA | AGGTCTAGTCAGAGCCTCCTGCATAATAATGGATACAAGTATTTGGAT | TTGGGTTCTAATCGGGCCTCC | ATGCAAGTTCTACAAACTCCGTGGACG |
| | | | SEQ ID NO:2503 | SEQ ID NO:10515 | SEQ ID NO:18527 |
| | | AA | RSSQSLLHNNGYKYLD | LGSNRAS | MQVLQTPWT |
| | | | SEQ ID NO:2504 | SEQ ID NO:10516 | SEQ ID NO:18528 |
| **iPS:435761** | **21-225_176B11** | NA | CGGGCAAGTCAGGGCATTAGAAATGATTTAGGC | GCTGCATCCAGTTTGCAAAGT | CTACAGCATTATAGTTACCCGCTCACT |
| | | | SEQ ID NO:2505 | SEQ ID NO:10517 | SEQ ID NO:18529 |
| | | AA | RASQGIRNDLG | AASSLQS | LQHYSYPLT |
| | | | SEQ ID NO:2506 | SEQ ID NO:10518 | SEQ ID NO:18530 |
| **iPS:435763** | **21-225_176H12** | NA | CGGGCAAGTCAGGGCATTAGAAATGATTTAGGC | GCTGCATCCAGTTTGCAAAGT | CTACAGCATAATAGTTACCCTCGCAGT |
| | | | SEQ ID NO:2507 | SEQ ID NO:10519 | SEQ ID NO:18531 |
| | | AA | RASQGIRNDLG | AASSLQS | LQHNSYPRS |
| | | | SEQ ID NO:2508 | SEQ ID NO:10520 | SEQ ID NO:18532 |
| **iPS:435765** | **21-225_177D3** | NA | CGGGCGAGTCAGGGCATTACCAATTATTTAGCC | GCTGCATCCAGTTTGCAAAGT | CAACAGTATAATAGTTACCCATTCACT |
| | | | SEQ ID NO:2509 | SEQ ID NO:10521 | SEQ ID NO:18533 |

**FIGURE 49**

| | | | | | |
|---|---|---|---|---|---|
| | | AA | RASQGITNYLA | AASSLQS | QQYNSYPFT |
| | | | SEQ ID NO:2510 | SEQ ID NO:10522 | SEQ ID NO:18534 |
| **iPS:435767** | **21-225_177B4** | NA | CGGGCAAGTCAGGGCATTAG AAATGATTTAGGC | GCTGCATCCAGTTTGCAA AGT | CTACAGCATTATAGTTTCCCT CGCAGT |
| | | | SEQ ID NO:2511 | SEQ ID NO:10523 | SEQ ID NO:18535 |
| | | AA | RASQGIRNDLG | AASSLQS | LQHYSFPRS |
| | | | SEQ ID NO:2512 | SEQ ID NO:10524 | SEQ ID NO:18536 |
| **iPS:435769** | **21-225_177B6** | NA | CGGGCAAGTCAGGACATTAG CAATGATTTAGGC | GCTGCATCCAGTTTGCAA AGT | CTACAGCTTAATAGTTACCC ATTCACT |
| | | | SEQ ID NO:2513 | SEQ ID NO:10525 | SEQ ID NO:18537 |
| | | AA | RASQDISNDLG | AASSLQS | LQLNSYPFT |
| | | | SEQ ID NO:2514 | SEQ ID NO:10526 | SEQ ID NO:18538 |
| **iPS:435771** | **21-225_177B11** | NA | AAGTCTAGTCAGCGCCTCCT GCATGGTGATGGAAAGACCT ATTTGTAT | GAAGTTTCCAACCGGTTC TCT | ATGCAAAGTATACAGATTCC GTGGACG |
| | | | SEQ ID NO:2515 | SEQ ID NO:10527 | SEQ ID NO:18539 |
| | | AA | KSSQRLLHGDGKTYLY | EVSNRFS | MQSIQIPWT |
| | | | SEQ ID NO:2516 | SEQ ID NO:10528 | SEQ ID NO:18540 |
| **iPS:435773** | **21-225_177B12** | NA | AAGTCCAGCCAGAGTGTTTT ACACAGCTCCAACAATAACA ACTACTTAACT | TGGGCATCTACCCGGGA ATCC | CAGCAATATTATAGTAGTCC TCCGACG |
| | | | SEQ ID NO:2517 | SEQ ID NO:10529 | SEQ ID NO:18541 |
| | | AA | KSSQSVLHSSNNNNYLT | WASTRES | QQYYSSPPT |
| | | | SEQ ID NO:2518 | SEQ ID NO:10530 | SEQ ID NO:18542 |
| **iPS:435775** | **21-225_178A5** | NA | CGGGCGAGTCAGGGTATTAG CAACTGGTTAGCC | GCTGCTTCCAGTTTGCAA AGT | CAACAGGCTAACAGTTTACC GTGGACG |
| | | | SEQ ID NO:2519 | SEQ ID NO:10531 | SEQ ID NO:18543 |
| | | AA | RASQGISNWLA | AASSLQS | QQANSLPWT |
| | | | SEQ ID NO:2520 | SEQ ID NO:10532 | SEQ ID NO:18544 |

FIGURE 49

| | | | | | |
|---|---|---|---|---|---|
| **iPS:435777** | **21-225_178F7** | NA | CGGGCGAGTCAGGATATTACTGACTGGTTAGCC | GCTGCATCCAGTTTGCAGAGT | CAACAGGCTAACAGTTTACCGTGGACG |
| | | | SEQ ID NO:2521 | SEQ ID NO:10533 | SEQ ID NO:18545 |
| | | AA | RASQDITDWLA | AASSLQS | QQANSLPWT |
| | | | SEQ ID NO:2522 | SEQ ID NO:10534 | SEQ ID NO:18546 |
| **iPS:435779** | **21-225_178B10** | NA | CGGGCAAGTCAGGGCATTAGAAATGATTTAGGC | GCTGCATCCAGTTTGCAAAAT | CTACACCATTATAGTTTCCCGCTCACT |
| | | | SEQ ID NO:2523 | SEQ ID NO:10535 | SEQ ID NO:18547 |
| | | AA | RASQGIRNDLG | AASSLQN | LHHYSFPLT |
| | | | SEQ ID NO:2524 | SEQ ID NO:10536 | SEQ ID NO:18548 |
| **iPS:435781** | **21-225_178G10** | NA | AAGTCTAGTCAGAGCCTCCTGCATGGTGATGGAAAGACCTATTTGTAT | GAAGTTTCCAACCGGTTTTCT | ATGCAAAGTATACAGGTTCCGTGGACG |
| | | | SEQ ID NO:2525 | SEQ ID NO:10537 | SEQ ID NO:18549 |
| | | AA | KSSQSLLHGDGKTYLY | EVSNRFS | MQSIQVPWT |
| | | | SEQ ID NO:2526 | SEQ ID NO:10538 | SEQ ID NO:18550 |
| **iPS:435783** | **21-225_179G1** | NA | CGGGCGAGTCAGGATATTAGCGACTGGTTAGCC | GCTGCATCCAGTTTGCAAAGT | CAACAGGCTAACAGTTTACCGTGGACG |
| | | | SEQ ID NO:2527 | SEQ ID NO:10539 | SEQ ID NO:18551 |
| | | AA | RASQDISDWLA | AASSLQS | QQANSLPWT |
| | | | SEQ ID NO:2528 | SEQ ID NO:10540 | SEQ ID NO:18552 |
| **iPS:435785** | **21-225_179C2** | NA | AAATCTAGTCAGAGCCTCCTGCATAGTGAGGGAAAGACCTACTTGTAT | GAGGTTTCCCACCGGTTCTCT | ATGCAAAGTATACAGGTTCTCACT |
| | | | SEQ ID NO:2529 | SEQ ID NO:10541 | SEQ ID NO:18553 |
| | | AA | KSSQSLLHSEGKTYLY | EVSHRFS | MQSIQVLT |
| | | | SEQ ID NO:2530 | SEQ ID NO:10542 | SEQ ID NO:18554 |
| **iPS:435787** | **21-225_180A3** | NA | CGGGCGAGTCAGGATATTACCAGCTGGTTAGCC | GCTGCATCCAGTTTGCAAAGT | CAACAGGCTAACAGTATCCCATTCACT |
| | | | SEQ ID NO:2531 | SEQ ID NO:10543 | SEQ ID NO:18555 |

**FIGURE 49**

| | | | | | |
|---|---|---|---|---|---|
| | | AA | RASQDITSWLA | AASSLQS | QQANSIPFT |
| | | | SEQ ID NO:2532 | SEQ ID NO:10544 | SEQ ID NO:18556 |
| **iPS:435789** | **21-225_180C4** | NA | AAGTCTAGTCAGAGCCTCCTGCATGGTGATGGAAAGACCTATTTGTAT | GCAACTTCCAACCGGTTCCCT | ATGCAAAGTATACAGGTTCCGTGGACG |
| | | | SEQ ID NO:2533 | SEQ ID NO:10545 | SEQ ID NO:18557 |
| | | AA | KSSQSLLHGDGKTYLY | ATSNRFP | MQSIQVPWT |
| | | | SEQ ID NO:2534 | SEQ ID NO:10546 | SEQ ID NO:18558 |
| **iPS:435791** | **21-225_180H7** | NA | CGGGCAAGTCAGGGCATTAGAAATGATTTAGGC | ACTGCATCCAGTTTGCAAAGT | CTACAGCATAATAGTTACCCATTCACT |
| | | | SEQ ID NO:2535 | SEQ ID NO:10547 | SEQ ID NO:18559 |
| | | AA | RASQGIRNDLG | TASSLQS | LQHNSYPFT |
| | | | SEQ ID NO:2536 | SEQ ID NO:10548 | SEQ ID NO:18560 |
| **iPS:435793** | **21-225_180F8** | NA | CGGGCAAGTCAGACCATTCTCAGCTATTTAAAT | GGTGTATCCAGTTTACAAAGT | CAGCAGAGTTACAGTACCCCATTCACT |
| | | | SEQ ID NO:2537 | SEQ ID NO:10549 | SEQ ID NO:18561 |
| | | AA | RASQTILSYLN | GVSSLQS | QQSYSTPFT |
| | | | SEQ ID NO:2538 | SEQ ID NO:10550 | SEQ ID NO:18562 |
| **iPS:435795** | **21-225_181C2** | NA | AAGTCTAGTCAGAGCCTCCTGCATGGTGATGGAAAGACCTATTTGTAT | GAAGTTTCCAACCGGTTCTCT | ATGCAAAGTATACAGGTTCCCTGGACG |
| | | | SEQ ID NO:2539 | SEQ ID NO:10551 | SEQ ID NO:18563 |
| | | AA | KSSQSLLHGDGKTYLY | EVSNRFS | MQSIQVPWT |
| | | | SEQ ID NO:2540 | SEQ ID NO:10552 | SEQ ID NO:18564 |
| **iPS:435797** | **21-225_181G2** | NA | CGGGCGAGTCAGGGCATTAGCAATTATTTAGCC | GCTGCATCCAGTTTGCAAAGT | CAACAGTATAATGGTTACCCATTCACT |
| | | | SEQ ID NO:2541 | SEQ ID NO:10553 | SEQ ID NO:18565 |
| | | AA | RASQGISNYLA | AASSLQS | QQYNGYPFT |
| | | | SEQ ID NO:2542 | SEQ ID NO:10554 | SEQ ID NO:18566 |

FIGURE 49

| | | | | | |
|---|---|---|---|---|---|
| **iPS:435799** | **21-225_181G3** | NA | CGGGCAAGTCACAGCATTAGCAACTATTTAAAT | ACTACATTGAATTTGCAAAGT | CAACAGAGTTACAGTTCTCCTCCGTGGACG |
| | | | SEQ ID NO:2543 | SEQ ID NO:10555 | SEQ ID NO:18567 |
| | | AA | RASHSISNYLN | TTLNLQS | QQSYSSPPWT |
| | | | SEQ ID NO:2544 | SEQ ID NO:10556 | SEQ ID NO:18568 |
| **iPS:435801** | **21-225_181E5** | NA | AAGTCCAGCCAGAGTGTTTTACACAGCTCCAACAATTACAACTACTTAACT | TGGGCATCTACCCGGGAATCC | CATCAGTATTTTATTACTCCGTGGACG |
| | | | SEQ ID NO:2545 | SEQ ID NO:10557 | SEQ ID NO:18569 |
| | | AA | KSSQSVLHSSNNYNYLT | WASTRES | HQYFITPWT |
| | | | SEQ ID NO:2546 | SEQ ID NO:10558 | SEQ ID NO:18570 |
| **iPS:435805** | **21-225_181A8** | NA | CGGGCAAGTCAGGGCATTAGAAATGATTTAGGC | ACTGCATCCAGTTTGCAAAGT | CTACAGCATAATAGTTACCCATTCACT |
| | | | SEQ ID NO:2547 | SEQ ID NO:10559 | SEQ ID NO:18571 |
| | | AA | RASQGIRNDLG | TASSLQS | LQHNSYPFT |
| | | | SEQ ID NO:2548 | SEQ ID NO:10560 | SEQ ID NO:18572 |
| **iPS:435807** | **21-225_181C10** | NA | AAGTCTAGTCAGAGCCTCCTGCATGGTGATGGAAAGACCTATTTGTAT | GAAGTTTCCAATCGGTTCTCT | ATGCAAAGTATACAGATTCCCTGGACG |
| | | | SEQ ID NO:2549 | SEQ ID NO:10561 | SEQ ID NO:18573 |
| | | AA | KSSQSLLHGDGKTYLY | EVSNRFS | MQSIQIPWT |
| | | | SEQ ID NO:2550 | SEQ ID NO:10562 | SEQ ID NO:18574 |
| **iPS:435809** | **21-225_182H5** | NA | CGGGCGAGTCAGGATATTACCAGCTGGTTAGCC | GCTGCATCCAGTTTACAAAGT | CAACAGGTTAACAGTTTCCCATTCACT |
| | | | SEQ ID NO:2551 | SEQ ID NO:10563 | SEQ ID NO:18575 |
| | | AA | RASQDITSWLA | AASSLQS | QQVNSFPFT |
| | | | SEQ ID NO:2552 | SEQ ID NO:10564 | SEQ ID NO:18576 |
| **iPS:435811** | **21-225_183H6** | NA | CAGGCGAGTCAGGACATTAGCAACTATTTAAAT | GATGCATCCAATTTGGAAACA | CAACAGTATGATAATCTCCCTCTCACT |
| | | | SEQ ID NO:2553 | SEQ ID NO:10565 | SEQ ID NO:18577 |

# FIGURE 49

| | | | | | |
|---|---|---|---|---|---|
| | | AA | QASQDISNYLN | DASNLET | QQYDNLPLT |
| | | | SEQ ID NO:2554 | SEQ ID NO:10566 | SEQ ID NO:18578 |
| **iPS:435813** | **21-225_183A12** | NA | CGGGCAAGTCGGAACATCA GCAACTATTTAAAT | GTTGTATCCAGTTTGCAA AGT | CAACAGAGTTACAGTTCCCC TCCGTGGACG |
| | | | SEQ ID NO:2555 | SEQ ID NO:10567 | SEQ ID NO:18579 |
| | | AA | RASRNISNYLN | VVSSLQS | QQSYSSPPWT |
| | | | SEQ ID NO:2556 | SEQ ID NO:10568 | SEQ ID NO:18580 |
| **iPS:435815** | **21-225_190G10** | NA | AGGGCCAGTCCGAGTGTTAG CAGCAGATTCTTAGCC | GGTGCATCCAGCAGGGC CACT | CAGCAGTATGGTAGCTCACC TCCGTGGACG |
| | | | SEQ ID NO:2557 | SEQ ID NO:10569 | SEQ ID NO:18581 |
| | | AA | RASPSVSSRFLA | GASSRAT | QQYGSSPPWT |
| | | | SEQ ID NO:2558 | SEQ ID NO:10570 | SEQ ID NO:18582 |
| **iPS:435817** | **21-225_190B11** | NA | CGGGCCAGTCAGAGTATTGG TAGTAACTTACAC | TCTGCTTCCCAGTCCTTC TCA | CAGCAGAGTAGTAGTTTACC GTGGACG |
| | | | SEQ ID NO:2559 | SEQ ID NO:10571 | SEQ ID NO:18583 |
| | | AA | RASQSIGSNLH | SASQSFS | QQSSSLPWT |
| | | | SEQ ID NO:2560 | SEQ ID NO:10572 | SEQ ID NO:18584 |
| **iPS:435819** | **21-225_190C11** | NA | CGGACGAGTCAGGGCATTGG CAATTATTTAGCC | AAAACATCCAGTTTACA AAGT | CAACAGTATATGACTTACCC GCTCACT |
| | | | SEQ ID NO:2561 | SEQ ID NO:10573 | SEQ ID NO:18585 |
| | | AA | RTSQGIGNYLA | KTSSLQS | QQYMTYPLT |
| | | | SEQ ID NO:2562 | SEQ ID NO:10574 | SEQ ID NO:18586 |
| **iPS:435821** | **21-225_190E11** | NA | AGGGCCAGTCAGAGTTTTCG CATCAACTTAGCC | GGTGCATCCACCAGGGC CACT | CAGCAGTATAATAACTGGCC GCTCACT |
| | | | SEQ ID NO:2563 | SEQ ID NO:10575 | SEQ ID NO:18587 |
| | | AA | RASQSFRINLA | GASTRAT | QQYNNWPLT |
| | | | SEQ ID NO:2564 | SEQ ID NO:10576 | SEQ ID NO:18588 |
| **iPS:435823** | **21-225_190F11** | NA | CGGGCCAGTCAGAACATTGG TAGTAGCTTACAC | TATGCTTCCCAGTCCTTC TCA | CATCAGAGTAGTAGTTTCCC TCGGACG |
| | | | SEQ ID NO:2565 | SEQ ID NO:10577 | SEQ ID NO:18589 |

FIGURE 49

| | | | | | |
|---|---|---|---|---|---|
| | | AA | RASQNIGSSLH | YASQSFS | HQSSSFPRT |
| | | | SEQ ID NO:2566 | SEQ ID NO:10578 | SEQ ID NO:18590 |
| **iPS:435825** | **21-225_190G11** | NA | CGGACGAGTCAGGGCATTGG CAATTATTTAGCC | AAAGCATCCAGTTTGCA AAGT | CAACAGTATATGACTTACCC GCTCACT |
| | | | SEQ ID NO:2567 | SEQ ID NO:10579 | SEQ ID NO:18591 |
| | | AA | RTSQGIGNYLA | KASSLQS | QQYMTYPLT |
| | | | SEQ ID NO:2568 | SEQ ID NO:10580 | SEQ ID NO:18592 |
| **iPS:435827** | **21-225_190H11** | NA | AAGTCTAGTCAGAGCCTCCT CCATAGTGATGGAAGGACCT ATTTGTAT | GAGGTTTCCAACCGGTTC GCT | ATGCAAAGTATACAGTTTCC CTGGACG |
| | | | SEQ ID NO:2569 | SEQ ID NO:10581 | SEQ ID NO:18593 |
| | | AA | KSSQSLLHSDGRTYLY | EVSNRFA | MQSIQFPWT |
| | | | SEQ ID NO:2570 | SEQ ID NO:10582 | SEQ ID NO:18594 |
| **iPS:435829** | **21-225_190B12** | NA | CGGGCCAGTCAGAGTATTGG TAGTAGCTTACAC | TATGCTTCCCAGTCCTTC TCA | CATCAGACTAGAAGTTTACC TCTCACT |
| | | | SEQ ID NO:2571 | SEQ ID NO:10583 | SEQ ID NO:18595 |
| | | AA | RASQSIGSSLH | YASQSFS | HQTRSLPLT |
| | | | SEQ ID NO:2572 | SEQ ID NO:10584 | SEQ ID NO:18596 |
| **iPS:435831** | **21-225_190C12** | NA | CGGGCAAGTCAGGGCATTAG AAAAGATTTAGGC | ACTGCATCCAGTTTGCAA AGT | CTACAGCATAATAGTTACCC GTGGACG |
| | | | SEQ ID NO:2573 | SEQ ID NO:10585 | SEQ ID NO:18597 |
| | | AA | RASQGIRKDLG | TASSLQS | LQHNSYPWT |
| | | | SEQ ID NO:2574 | SEQ ID NO:10586 | SEQ ID NO:18598 |
| **iPS:435833** | **21-225_190D12** | NA | CGGGCGAGTCAGGGCATTAG CAATTATTTAGCC | GTTGCATCCACTTTGCAA TCA | CAAAAGTATAACAGTGCCCC ATTCACT |
| | | | SEQ ID NO:2575 | SEQ ID NO:10587 | SEQ ID NO:18599 |
| | | AA | RASQGISNYLA | VASTLQS | QKYNSAPFT |
| | | | SEQ ID NO:2576 | SEQ ID NO:10588 | SEQ ID NO:18600 |
| **iPS:435835** | | NA | CGGGCGAGTCAGGGCATTGG CAAGTATTTAGCC | GCTGCATCCAGTTTGCAA AGT | CAACGATATGATACTTACCC ATTCACT |

FIGURE 49

| | | | | | |
|---|---|---|---|---|---|
| | **21-225_190F12** | | SEQ ID NO:2577 | SEQ ID NO:10589 | SEQ ID NO:18601 |
| | | AA | RASQGIGKYLA | AASSLQS | QRYDTYPFT |
| | | | SEQ ID NO:2578 | SEQ ID NO:10590 | SEQ ID NO:18602 |
| **iPS:435837** | **21-225_198G3** | NA | CGGACGAGTCAGGGCATTGGCAAGTATTTAGCC | AAAGCATCCAGTTTGCAAGGT | CAACAGTATATGACTTACCCGCTCACT |
| | | | SEQ ID NO:2579 | SEQ ID NO:10591 | SEQ ID NO:18603 |
| | | AA | RTSQGIGKYLA | KASSLQG | QQYMTYPLT |
| | | | SEQ ID NO:2580 | SEQ ID NO:10592 | SEQ ID NO:18604 |
| **iPS:435839** | **21-225_191B1** | NA | AGGTCTAGTCAGAGCCTCCTGCATAGTGATGGAAAGACCTATTTGTTT | GAACTTTCCAACCGGTTCTCT | ATGCAAAGTTTCCAGCTTCCCTGGACG |
| | | | SEQ ID NO:2581 | SEQ ID NO:10593 | SEQ ID NO:18605 |
| | | AA | RSSQSLLHSDGKTYLF | ELSNRFS | MQSFQLPWT |
| | | | SEQ ID NO:2582 | SEQ ID NO:10594 | SEQ ID NO:18606 |
| **iPS:435841** | **21-225_191D8** | NA | AGGTCCAGCCAGAGTGTTTTACACAGCTCCAACAATTACAACTACTTAGCT | TGGGCATCTACCCGGGAATCC | CAACAATATTATAGTACTCCTCCGACG |
| | | | SEQ ID NO:2583 | SEQ ID NO:10595 | SEQ ID NO:18607 |
| | | AA | RSSQSVLHSSNNYNYLA | WASTRES | QQYYSTPPT |
| | | | SEQ ID NO:2584 | SEQ ID NO:10596 | SEQ ID NO:18608 |
| **iPS:435843** | **21-225_191F1** | NA | AGGGCCAGTCAGAGTATTAGCCTCAACTTCTTAGCC | GGTGCATCCAGCAGGGCCACT | CAGCAGTATGGTAGGTCACCGTGGACG |
| | | | SEQ ID NO:2585 | SEQ ID NO:10597 | SEQ ID NO:18609 |
| | | AA | RASQSISLNFLA | GASSRAT | QQYGRSPWT |
| | | | SEQ ID NO:2586 | SEQ ID NO:10598 | SEQ ID NO:18610 |
| **iPS:435845** | **21-225_191G1** | NA | CGGGCGAGTCAGGGCATTAGCAATTATTTAGCC | GCTGCATCCAGTTTGCAAAGT | CAACATTATCTTACTTACCCTCTCACT |
| | | | SEQ ID NO:2587 | SEQ ID NO:10599 | SEQ ID NO:18611 |
| | | AA | RASQGISNYLA | AASSLQS | QHYLTYPLT |
| | | | SEQ ID NO:2588 | SEQ ID NO:10600 | SEQ ID NO:18612 |

FIGURE 49

| | | | | | |
|---|---|---|---|---|---|
| **iPS:435847** | **21-225_191A3** | NA | AGGGCCAGTCAGAGTATTCGCAGCAGCTTCTTAGCC | GGTGCATCCAGCAGGGCCACT | CAGCAATATGGTAACTCACCGTGGGCG |
| | | | SEQ ID NO:2589 | SEQ ID NO:10601 | SEQ ID NO:18613 |
| | | AA | RASQSIRSSFLA | GASSRAT | QQYGNSPWA |
| | | | SEQ ID NO:2590 | SEQ ID NO:10602 | SEQ ID NO:18614 |
| **iPS:435849** | **21-225_191C3** | NA | AGGGCCAGTCAGAGTATTCGCAGCAGCTTCTTAGCC | GGTGCATCCAGCAGGGCCACT | CAGCAGTATGGTAACTCACCGTGGGCG |
| | | | SEQ ID NO:2591 | SEQ ID NO:10603 | SEQ ID NO:18615 |
| | | AA | RASQSIRSSFLA | GASSRAT | QQYGNSPWA |
| | | | SEQ ID NO:2592 | SEQ ID NO:10604 | SEQ ID NO:18616 |
| **iPS:435851** | **21-225_191D3** | NA | AGGGCCGGTCAAAGTATTAGAACCAACTTCTTAGCC | GGTGCATCCAGCAGGGCCACT | CAGCAGTATGGTAGCTCACCGTGGACG |
| | | | SEQ ID NO:2593 | SEQ ID NO:10605 | SEQ ID NO:18617 |
| | | AA | RAGQSIRTNFLA | GASSRAT | QQYGSSPWT |
| | | | SEQ ID NO:2594 | SEQ ID NO:10606 | SEQ ID NO:18618 |
| **iPS:435853** | **21-225_191E3** | NA | AAGTCTAGTCAGAGCCTCCTCCATAGTGATGGAAGGACCTATTTGTAT | GAGGTTTCCAACCGGTTCGCT | ATGCAAAGTATACAACTTCCCTGGACG |
| | | | SEQ ID NO:2595 | SEQ ID NO:10607 | SEQ ID NO:18619 |
| | | AA | KSSQSLLHSDGRTYLY | EVSNRFA | MQSIQLPWT |
| | | | SEQ ID NO:2596 | SEQ ID NO:10608 | SEQ ID NO:18620 |
| **iPS:435855** | **21-225_191G3** | NA | AAGTCCAGCCAGAGTGTTTTACACAGCTCCAACAGTTACAACTACTTAGCT | TGGGCATCTACCCGAAAATCC | CAGCAATATTATAGTAGTCCTCCCACT |
| | | | SEQ ID NO:2597 | SEQ ID NO:10609 | SEQ ID NO:18621 |
| | | AA | KSSQSVLHSSNSYNYLA | WASTRKS | QQYYSSPPT |
| | | | SEQ ID NO:2598 | SEQ ID NO:10610 | SEQ ID NO:18622 |
| **iPS:435857** | **21-225_191A4** | NA | CGGGCAAGTCAGGGCATTAGAAAAGATTTAGGC | ACTGCATCCAGTTTGCAAAAT | CTACAGCATAATAGTTACCCGTGGACG |
| | | | SEQ ID NO:2599 | SEQ ID NO:10611 | SEQ ID NO:18623 |

FIGURE 49

| | | | | | |
|---|---|---|---|---|---|
| | | AA | RASQGIRKDLG | TASSLQN | LQHNSYPWT |
| | | | SEQ ID NO:2600 | SEQ ID NO:10612 | SEQ ID NO:18624 |
| **iPS:435859** | **21-225_190E6** | NA | CGGACGAGTCAGGGCATTGG CAATTATTTAGCC | AAAGCATCCAGTTTGCA AAGT | CAACAGTATATGACTTACCC GCTCACT |
| | | | SEQ ID NO:2601 | SEQ ID NO:10613 | SEQ ID NO:18625 |
| | | AA | RTSQGIGNYLA | KASSLQS | QQYMTYPLT |
| | | | SEQ ID NO:2602 | SEQ ID NO:10614 | SEQ ID NO:18626 |
| **iPS:435861** | **21-225_190A5** | NA | CGGGCGAGTCAGGGGATTG GCAATCATTTAGCC | GCTGCATCCAGTTTGCAA AGT | CAACAGTATAGTAATTACCC AGTCACT |
| | | | SEQ ID NO:2603 | SEQ ID NO:10615 | SEQ ID NO:18627 |
| | | AA | RASQGIGNHLA | AASSLQS | QQYSNYPVT |
| | | | SEQ ID NO:2604 | SEQ ID NO:10616 | SEQ ID NO:18628 |
| **iPS:435863** | **21-225_191H4** | NA | CGGGCCAATCAGAGCATTGG TAGTAGCTTACAC | TATGCTTCCCAGTCCCTC TCA | CATCAGACTGGTAGGTTACC TCTCACT |
| | | | SEQ ID NO:2605 | SEQ ID NO:10617 | SEQ ID NO:18629 |
| | | AA | RANQSIGSSLH | YASQSLS | HQTGRLPLT |
| | | | SEQ ID NO:2606 | SEQ ID NO:10618 | SEQ ID NO:18630 |
| **iPS:435865** | **21-225_191A5** | NA | AGGGCCAGTCAGAGTGTTAG CAGCAGGTTCTTAGCC | GGTGCTTCCAACAGGGC CACT | CAGCAGTATGGTGGTTCACC TCCGTGGACG |
| | | | SEQ ID NO:2607 | SEQ ID NO:10619 | SEQ ID NO:18631 |
| | | AA | RASQSVSSRFLA | GASNRAT | QQYGGSPPWT |
| | | | SEQ ID NO:2608 | SEQ ID NO:10620 | SEQ ID NO:18632 |
| **iPS:435867** | **21-225_191E5** | NA | CGGGCCAGTCAGAGCATTGG TAGTAGCTTACAC | TATGCTTCCCAGTCCTTC TCA | CATCAGAGTAGTAGTTTCCC TCGGACG |
| | | | SEQ ID NO:2609 | SEQ ID NO:10621 | SEQ ID NO:18633 |
| | | AA | RASQSIGSSLH | YASQSFS | HQSSSFPRT |
| | | | SEQ ID NO:2610 | SEQ ID NO:10622 | SEQ ID NO:18634 |
| **iPS:435869** | **21-225_190B1** | NA | CGGGCGAGTCAGGGCATTAG AAATTATTTAGCC | GTTGCATCCAGTTTGGAA AGT | CAACAGTATCTTAATTACCC AGTCACT |
| | | | SEQ ID NO:2611 | SEQ ID NO:10623 | SEQ ID NO:18635 |

FIGURE 49

| | | | | | |
|---|---|---|---|---|---|
| | | AA | RASQGIRNYLA | VASSLES | QQYLNYPVT |
| | | | SEQ ID NO:2612 | SEQ ID NO:10624 | SEQ ID NO:18636 |
| **iPS:435871** | **21-225_191E6** | NA | AAGTCTAGTCAGAGCCTCCTCCATAGTGATGGAAGGACCTATTTGTAT | GAGGTTTCCAACCGGTTCGCT | ATGCAAAGTATACATTTTCCCTGGACG |
| | | | SEQ ID NO:2613 | SEQ ID NO:10625 | SEQ ID NO:18637 |
| | | AA | KSSQSLLHSDGRTYLY | EVSNRFA | MQSIHFPWT |
| | | | SEQ ID NO:2614 | SEQ ID NO:10626 | SEQ ID NO:18638 |
| **iPS:435873** | **21-225_190G4** | NA | CGGGCGAGTCAGGACATTGGCAGATATTTAGCC | GCTGCATCCAGTTTGCAAAGT | CAACAATATAGTACTTACCCGCTCACT |
| | | | SEQ ID NO:2615 | SEQ ID NO:10627 | SEQ ID NO:18639 |
| | | AA | RASQDIGRYLA | AASSLQS | QQYSTYPLT |
| | | | SEQ ID NO:2616 | SEQ ID NO:10628 | SEQ ID NO:18640 |
| **iPS:435875** | **21-225_190B9** | NA | CGGGCGAGTCAGGGTATTAACAACTGGTTAGCC | GGTGTTTCCAGTTTGCAGAGT | CAACAGGCTAACAGTTTCCCGTGGACG |
| | | | SEQ ID NO:2617 | SEQ ID NO:10629 | SEQ ID NO:18641 |
| | | AA | RASQGINNWLA | GVSSLQS | QQANSFPWT |
| | | | SEQ ID NO:2618 | SEQ ID NO:10630 | SEQ ID NO:18642 |
| **iPS:435877** | **21-225_184E7** | NA | CGGGCGAGTCAGGGCATTAGCAATTATTTAGCC | GCTGCATCCAGTTTGCAAAGT | CAACAGTATAATGGTTACCCATTCACT |
| | | | SEQ ID NO:2619 | SEQ ID NO:10631 | SEQ ID NO:18643 |
| | | AA | RASQGISNYLA | AASSLQS | QQYNGYPFT |
| | | | SEQ ID NO:2620 | SEQ ID NO:10632 | SEQ ID NO:18644 |
| **iPS:435879** | **21-225_184H10** | NA | CGGGCAAGTCAGGGCATTAGAAATGATTTAGGC | ATTGCATCCAGTTTGCAAAGT | CTACAGCATAATAGTTACCCATTCACT |
| | | | SEQ ID NO:2621 | SEQ ID NO:10633 | SEQ ID NO:18645 |
| | | AA | RASQGIRNDLG | IASSLQS | LQHNSYPFT |
| | | | SEQ ID NO:2622 | SEQ ID NO:10634 | SEQ ID NO:18646 |
| **iPS:435881** | | NA | CGGGCAAGTCAGGGCATTAGAAATGATTTAGGC | ATTGCATCCAGTTTGCAAAGT | CTACAGCATAATAGTTACCCATTCACT |

FIGURE 49

| | | | | | |
|---|---|---|---|---|---|
| | **21-225_184D11** | | SEQ ID NO:2623 | SEQ ID NO:10635 | SEQ ID NO:18647 |
| | | AA | RASQGIRNDLG | IASSLQS | LQHNSYPFT |
| | | | SEQ ID NO:2624 | SEQ ID NO:10636 | SEQ ID NO:18648 |
| **iPS:435883** | **21-225_185A1** | NA | CGGGCGAGTCAGGGCATTAG CAATTATTTAGCC | GTTGCATCCAGTTTGCAA AGT | CGACAATATCATAGTTACCC ATTCACT |
| | | | SEQ ID NO:2625 | SEQ ID NO:10637 | SEQ ID NO:18649 |
| | | AA | RASQGISNYLA | VASSLQS | RQYHSYPFT |
| | | | SEQ ID NO:2626 | SEQ ID NO:10638 | SEQ ID NO:18650 |
| **iPS:435885** | **21-225_185E10** | NA | CGGGCGAGTCAGGGCATTAG CAATTATTTAGCC | GCTGCATCCAGTTTGCAA AGT | CAACAGTATAATGGTTACCC ATTCACT |
| | | | SEQ ID NO:2627 | SEQ ID NO:10639 | SEQ ID NO:18651 |
| | | AA | RASQGISNYLA | AASSLQS | QQYNGYPFT |
| | | | SEQ ID NO:2628 | SEQ ID NO:10640 | SEQ ID NO:18652 |
| **iPS:435887** | **21-225_186F7** | NA | AAGTCTAGTCAGAGCCTCCT GCATGGTGATGGAAAGACCT ATTTGTGT | GAAGTTTCCAAGCGGTT CTCT | ATGCAAAGTATACAGGTTCC CTGGACG |
| | | | SEQ ID NO:2629 | SEQ ID NO:10641 | SEQ ID NO:18653 |
| | | AA | KSSQSLLHGDGKTYLC | EVSKRFS | MQSIQVPWT |
| | | | SEQ ID NO:2630 | SEQ ID NO:10642 | SEQ ID NO:18654 |
| **iPS:435889** | **21-225_186A11** | NA | CGGGCGAGTCAGGATATTAC CAGCTGGTTAGCC | GCTGCATCCAGTTTACAA AGT | CAACAGGTTAACAGTTTCCC ATTCACT |
| | | | SEQ ID NO:2631 | SEQ ID NO:10643 | SEQ ID NO:18655 |
| | | AA | RASQDITSWLA | AASSLQS | QQVNSFPFT |
| | | | SEQ ID NO:2632 | SEQ ID NO:10644 | SEQ ID NO:18656 |
| **iPS:435891** | **21-225_188H5** | NA | CGGGCGAGTCAGGGCATTAG CAATTATTTAGCC | GCTGCTTCCAGTTTGCAA AGT | CAACAGTATAATAGTTATCC ATTCACT |
| | | | SEQ ID NO:2633 | SEQ ID NO:10645 | SEQ ID NO:18657 |
| | | AA | RASQGISNYLA | AASSLQS | QQYNSYPFT |
| | | | SEQ ID NO:2634 | SEQ ID NO:10646 | SEQ ID NO:18658 |

**FIGURE 49**

| | | | | | |
|---|---|---|---|---|---|
| **iPS:435895** | **21-225_188E8** | NA | CGGGCGAATCAGGATATTTC CAGCTGGTTAGCC | GCTGCATCCAATTTGCAA AGT | CAGCAGGCTAACAGTTTCCC GTGGACG |
| | | | SEQ ID NO:2635 | SEQ ID NO:10647 | SEQ ID NO:18659 |
| | | AA | RANQDISSWLA | AASNLQS | QQANSFPWT |
| | | | SEQ ID NO:2636 | SEQ ID NO:10648 | SEQ ID NO:18660 |
| **iPS:435897** | **21-225_188B9** | NA | CGGGCGAGTCAGGGCATTAG CAATTATTTAGCC | GCTGCATCCAGTTTGCAA AGT | CAACAGTATAATAGTTACCC ATTCACT |
| | | | SEQ ID NO:2637 | SEQ ID NO:10649 | SEQ ID NO:18661 |
| | | AA | RASQGISNYLA | AASSLQS | QQYNSYPFT |
| | | | SEQ ID NO:2638 | SEQ ID NO:10650 | SEQ ID NO:18662 |
| **iPS:435899** | **21-225_188G11** | NA | ATGTCTAGTCAGAGCCTCCT GCATGGTGATGGAAAGACCT ATTTATAT | GAAGTTTCCAACCGGTTC TCT | ATGCAAAGTATACAGATTCC TTGGACG |
| | | | SEQ ID NO:2639 | SEQ ID NO:10651 | SEQ ID NO:18663 |
| | | AA | MSSQSLLHGDGKTYLY | EVSNRFS | MQSIQIPWT |
| | | | SEQ ID NO:2640 | SEQ ID NO:10652 | SEQ ID NO:18664 |
| **iPS:435901** | **21-225_189G2** | NA | AAGTCTAGTCAGAGCCTCCT GCATGGTGATGGAAAGACCT ATTTGTTT | GAAGTTTCCAACCGGTTC TCT | ATGCAAAGTATACAGATTCC GTGGACG |
| | | | SEQ ID NO:2641 | SEQ ID NO:10653 | SEQ ID NO:18665 |
| | | AA | KSSQSLLHGDGKTYLF | EVSNRFS | MQSIQIPWT |
| | | | SEQ ID NO:2642 | SEQ ID NO:10654 | SEQ ID NO:18666 |
| **iPS:435903** | **21-225_190E2** | NA | AAGTCCAGCCAGAGTGTTTT ATTCAACTCCAACAATAAGA ACTACTTAGCT | TGGGCATCTACCCGGGA ATCC | CAGCAATATTGTAGTCTTCC ATTCACT |
| | | | SEQ ID NO:2643 | SEQ ID NO:10655 | SEQ ID NO:18667 |
| | | AA | KSSQSVLFNSNNKNYLA | WASTRES | QQYCSLPFT |
| | | | SEQ ID NO:2644 | SEQ ID NO:10656 | SEQ ID NO:18668 |
| **iPS:435905** | | NA | AGGGCCAGTCAGAATATAA GGAGCAACTTCTTAGCC | GGTGCATCCAGCAGGGC CACT | CAGCAGTATGGTAACTCACC GTGGACG |

FIGURE 49

| | | | | | |
|---|---|---|---|---|---|
| | 21-225_190A3 | | SEQ ID NO:2645 | SEQ ID NO:10657 | SEQ ID NO:18669 |
| | | AA | RASQNIRSNFLA | GASSRAT | QQYGNSPWT |
| | | | SEQ ID NO:2646 | SEQ ID NO:10658 | SEQ ID NO:18670 |
| iPS:435907 | 21-225_190G3 | NA | CGGGCAAGTCAGGGCATTAG AAAAGATTTAGGC | ACTGCATCCAGTTTGCAA AGT | CTACAGCATAATAGTTACCC GTGGACG |
| | | | SEQ ID NO:2647 | SEQ ID NO:10659 | SEQ ID NO:18671 |
| | | AA | RASQGIRKDLG | TASSLQS | LQHNSYPWT |
| | | | SEQ ID NO:2648 | SEQ ID NO:10660 | SEQ ID NO:18672 |
| iPS:435909 | 21-225_190H3 | NA | CGGGCGAGTCAGGGTCTTAA CAACTGGTTAGCC | GCTGTGTCCAGTTTGCAA AGT | CAACAGGCTAACAGTCTCCC GTGGACG |
| | | | SEQ ID NO:2649 | SEQ ID NO:10661 | SEQ ID NO:18673 |
| | | AA | RASQGLNNWLA | AVSSLQS | QQANSLPWT |
| | | | SEQ ID NO:2650 | SEQ ID NO:10662 | SEQ ID NO:18674 |
| iPS:435911 | 21-225_190B4 | NA | AGGGCCAGTCAGAGTATTCG CAGCAGCTTCTTAGCC | GGTGCATCCAGCAGGGC CACT | CAGCAGTATGGTAACTCACC GTGGGCG |
| | | | SEQ ID NO:2651 | SEQ ID NO:10663 | SEQ ID NO:18675 |
| | | AA | RASQSIRSSFLA | GASSRAT | QQYGNSPWA |
| | | | SEQ ID NO:2652 | SEQ ID NO:10664 | SEQ ID NO:18676 |
| iPS:435913 | 21-225_190A7 | NA | AGGGCCAGTCAGAGTGTTAG AAGCAACTTCTTAGCC | GGTGCATACCGCAGGGC CACT | CAGCAGTATGGTAACTCACC GTGGACG |
| | | | SEQ ID NO:2653 | SEQ ID NO:10665 | SEQ ID NO:18677 |
| | | AA | RASQSVRSNFLA | GAYRRAT | QQYGNSPWT |
| | | | SEQ ID NO:2654 | SEQ ID NO:10666 | SEQ ID NO:18678 |
| iPS:435915 | 21-225_190H4 | NA | AAGTCCAGCCAGAGTGTTTT ACACAGCTCCAACAATTACA ACTACTTAGCT | TGGGCATCTACCCGGGA ATCC | CAGCAATATTATAGTATTCC TCCCACT |
| | | | SEQ ID NO:2655 | SEQ ID NO:10667 | SEQ ID NO:18679 |
| | | AA | KSSQSVLHSSNNYNYLA | WASTRES | QQYYSIPPT |
| | | | SEQ ID NO:2656 | SEQ ID NO:10668 | SEQ ID NO:18680 |

FIGURE 49

| | | | | | |
|---|---|---|---|---|---|
| **iPS:435917** | **21-225_190D5** | NA | CGGGCCAGTCAGAGTATTGG TAGTAACTTACAC | TCTGCTTCCCAGTCCTTC TCA | CAGCAGAGTAGTAGTTTACC GTGGACG |
| | | | SEQ ID NO:2657 | SEQ ID NO:10669 | SEQ ID NO:18681 |
| | | AA | RASQSIGSNLH | SASQSFS | QQSSSLPWT |
| | | | SEQ ID NO:2658 | SEQ ID NO:10670 | SEQ ID NO:18682 |
| **iPS:435919** | **21-225_190H5** | NA | CGGGCAAGTCAGGGCATTAG AAAAGATTTAGGC | ACTGCATCCAGTTTGCAA AGT | CTACAGCATAATAATTACCC GTGGACG |
| | | | SEQ ID NO:2659 | SEQ ID NO:10671 | SEQ ID NO:18683 |
| | | AA | RASQGIRKDLG | TASSLQS | LQHNNYPWT |
| | | | SEQ ID NO:2660 | SEQ ID NO:10672 | SEQ ID NO:18684 |
| **iPS:435921** | **21-225_190D6** | NA | CGGGCAAGTCAGGGCATTAG AAATGATTTAGGC | GCTGCATCCAGTTTGCAA AGT | CTACAGCATTATAGTTTCCC ATTCACT |
| | | | SEQ ID NO:2661 | SEQ ID NO:10673 | SEQ ID NO:18685 |
| | | AA | RASQGIRNDLG | AASSLQS | LQHYSFPFT |
| | | | SEQ ID NO:2662 | SEQ ID NO:10674 | SEQ ID NO:18686 |
| **iPS:435923** | **21-225_190H6** | NA | AAGTCCAGCCAGAGTGTTTT ATTCAACTCCAACAATAAGA ACTACTTAGCT | TGGGCATCTACCCGGGA ATCC | CAGCAATATTGTAGTCTTCC ATTCACT |
| | | | SEQ ID NO:2663 | SEQ ID NO:10675 | SEQ ID NO:18687 |
| | | AA | KSSQSVLFNSNNKNYLA | WASTRES | QQYCSLPFT |
| | | | SEQ ID NO:2664 | SEQ ID NO:10676 | SEQ ID NO:18688 |
| **iPS:435925** | **21-225_190D7** | NA | AAGTCCAGCCAGAGTGTTTT ATCCAGCTCCAACAATTACA ACTATTTAGTT | TGGGCATCTACCCGGAA ATCC | CAACAATATTATCGTACTCC GTGGACG |
| | | | SEQ ID NO:2665 | SEQ ID NO:10677 | SEQ ID NO:18689 |
| | | AA | KSSQSVLSSSNNYNYLV | WASTRKS | QQYYRTPWT |
| | | | SEQ ID NO:2666 | SEQ ID NO:10678 | SEQ ID NO:18690 |
| **iPS:435927** | **21 225 190E7** | NA | AAGTCTAGTCAGAGCCTCCT CCATAGTGATGGAAGGACCT ATTTGTAT | GAGGTTTCCAACCGGTTC TCT | ATGCAAAGTATACAGCTTCC CTGGACG |

FIGURE 49

| | | | | | |
|---|---|---|---|---|---|
| | 21-225_190E7 | | SEQ ID NO:2667 | SEQ ID NO:10679 | SEQ ID NO:18691 |
| | | AA | KSSQSLLHSDGRTYLY | EVSNRFS | MQSIQLPWT |
| | | | SEQ ID NO:2668 | SEQ ID NO:10680 | SEQ ID NO:18692 |
| **iPS:435929** | 21-225_190D9 | NA | CGGGCCAGTCAGAGCATTGG TAGTAGCTTACAC | TATGCTTCCCAGTCCTTC TCA | CATCAGAGTAGTAGTTTCCC TCGGACG |
| | | | SEQ ID NO:2669 | SEQ ID NO:10681 | SEQ ID NO:18693 |
| | | AA | RASQSIGSSLH | YASQSFS | HQSSSFPRT |
| | | | SEQ ID NO:2670 | SEQ ID NO:10682 | SEQ ID NO:18694 |
| **iPS:435933** | 21-225_190F8 | NA | CGGACGAGTCAGGGCATTGG CAATTATTTAGCC | AAAGCATCCAGTTTACA AAGT | CAACAGTATATGACTTACCC ACTCACT |
| | | | SEQ ID NO:2671 | SEQ ID NO:10683 | SEQ ID NO:18695 |
| | | AA | RTSQGIGNYLA | KASSLQS | QQYMTYPLT |
| | | | SEQ ID NO:2672 | SEQ ID NO:10684 | SEQ ID NO:18696 |
| **iPS:435935** | 21-225_190H8 | NA | CGGGCCAGTCAGAGCATTGG TAGTAGCTTACAC | TATGCTTCCCAGTCCTTC TCA | CATCAGAGTAGTAGTTTCCC TCGGACG |
| | | | SEQ ID NO:2673 | SEQ ID NO:10685 | SEQ ID NO:18697 |
| | | AA | RASQSIGSSLH | YASQSFS | HQSSSFPRT |
| | | | SEQ ID NO:2674 | SEQ ID NO:10686 | SEQ ID NO:18698 |
| **iPS:435937** | 21-225_190H9 | NA | CGGGCGAGTCAGGGCATTGG CAAGTATTTAGCC | GCTGCATCCAGTTTGCAA AGT | CAACGATATGATACTTACCC ATTCACT |
| | | | SEQ ID NO:2675 | SEQ ID NO:10687 | SEQ ID NO:18699 |
| | | AA | RASQGIGKYLA | AASSLQS | QRYDTYPFT |
| | | | SEQ ID NO:2676 | SEQ ID NO:10688 | SEQ ID NO:18700 |
| **iPS:435939** | 21-225_191H7 | NA | AGGGCCGGTCAAAGTATTAG AACCGACTTCTTAGCC | GGTCCATCCAGCAGGGC CACT | CAGCAGTATGGTAGCTCACC GTGGACG |
| | | | SEQ ID NO:2677 | SEQ ID NO:10689 | SEQ ID NO:18701 |
| | | AA | RAGQSIRTDFLA | GPSSRAT | QQYGSSPWT |
| | | | SEQ ID NO:2678 | SEQ ID NO:10690 | SEQ ID NO:18702 |
| **iPS:435941** | | NA | AGGCCCAGTCAGAGTTTTAG CAGAAACTTAGCC | GGTGCATCCACTAGGGC CACT | CAGCAGTATAATAACTGGCC GCTCACT |

FIGURE 49

| | | | | | |
|---|---|---|---|---|---|
| | **21-225_191E8** | | SEQ ID NO:2679 | SEQ ID NO:10691 | SEQ ID NO:18703 |
| | | AA | RPSQSFSRNLA | GASTRAT | QQYNNWPLT |
| | | | SEQ ID NO:2680 | SEQ ID NO:10692 | SEQ ID NO:18704 |
| **iPS:435943** | **21-225_191C9** | NA | CGGGCCAGTCAGAGTATTGG TAGTAGTTTACAC | TATGCTTCCCAGTCCTTC TCA | CATCAGACTAGAAGTTTACC TCTCACT |
| | | | SEQ ID NO:2681 | SEQ ID NO:10693 | SEQ ID NO:18705 |
| | | AA | RASQSIGSSLH | YASQSFS | HQTRSLPLT |
| | | | SEQ ID NO:2682 | SEQ ID NO:10694 | SEQ ID NO:18706 |
| **iPS:435945** | **21-225_191A10** | NA | CGGGCGAGTCAGGGCATTGG CAAGTATTTAGCC | GCTGCATCCAGTTTGCAA AGT | CAACAGTATAGTACTTACCC GCTCACT |
| | | | SEQ ID NO:2683 | SEQ ID NO:10695 | SEQ ID NO:18707 |
| | | AA | RASQGIGKYLA | AASSLQS | QQYSTYPLT |
| | | | SEQ ID NO:2684 | SEQ ID NO:10696 | SEQ ID NO:18708 |
| **iPS:435947** | **21-225_191E10** | NA | CGGGCGAGTCAGGGCATTGG CAAGTATTTAGCC | GCTGCATCCAGTTTGCAA AGT | CAACAGTATAGTACTTACCC GCTCACT |
| | | | SEQ ID NO:2685 | SEQ ID NO:10697 | SEQ ID NO:18709 |
| | | AA | RASQGIGKYLA | AASSLQS | QQYSTYPLT |
| | | | SEQ ID NO:2686 | SEQ ID NO:10698 | SEQ ID NO:18710 |
| **iPS:435953** | **21-225_191B12** | NA | AAGTCCAGCCAGAGTGTTTT ATTCAACTCCAACAATAAGA ACTACTTAGCT | TGGGCCTCTACCCGGGA ATCC | CAGCAATATTCTAGTCTTCC ATTCACT |
| | | | SEQ ID NO:2687 | SEQ ID NO:10699 | SEQ ID NO:18711 |
| | | AA | KSSQSVLFNSNNKNYLA | WASTRES | QQYSSLPFT |
| | | | SEQ ID NO:2688 | SEQ ID NO:10700 | SEQ ID NO:18712 |
| **iPS:435957** | **21-225_191G12** | NA | CGGACGAGTCAGGGCATTGG CAATTATTTAGCC | AAAGCATCCAGTTTGCA AAGT | CAACAGTATATCACTTACCC GCTCACT |
| | | | SEQ ID NO:2689 | SEQ ID NO:10701 | SEQ ID NO:18713 |
| | | AA | RTSQGIGNYLA | KASSLQS | QQYITYPLT |
| | | | SEQ ID NO:2690 | SEQ ID NO:10702 | SEQ ID NO:18714 |

FIGURE 49

| | | | | | |
|---|---|---|---|---|---|
| **iPS:435961** | **21-225_192A2** | NA | CGGGCGAATCAGGGCATTAA CAATTATTTAGCC | GCTGCATCCAGTTTGCAA AGT | CAACAGTATAATAGTTACCC ATTCACT |
| | | | SEQ ID NO:2691 | SEQ ID NO:10703 | SEQ ID NO:18715 |
| | | AA | RANQGINNYLA | AASSLQS | QQYNSYPFT |
| | | | SEQ ID NO:2692 | SEQ ID NO:10704 | SEQ ID NO:18716 |
| **iPS:435963** | **21-225_192D2** | NA | CGGGCGAGTCAGGGCATTAG CAATTATTTAGCC | GCTGCATCCAGTTTGCAA AGT | CAACATTATGTTACTTACCC GAACACT |
| | | | SEQ ID NO:2693 | SEQ ID NO:10705 | SEQ ID NO:18717 |
| | | AA | RASQGISNYLA | AASSLQS | QHYVTYPNT |
| | | | SEQ ID NO:2694 | SEQ ID NO:10706 | SEQ ID NO:18718 |
| **iPS:435965** | **21-225_192H2** | NA | CGGGCGAGTCAGGGTATAA GTAGTTGGATAGCC | GGTGCATCCAGTTTGCA AAGT | CAACAGTCTAACAGTTTCCC ATTCACT |
| | | | SEQ ID NO:2695 | SEQ ID NO:10707 | SEQ ID NO:18719 |
| | | AA | RASQGISSWIA | GASSLQS | QQSNSFPFT |
| | | | SEQ ID NO:2696 | SEQ ID NO:10708 | SEQ ID NO:18720 |
| **iPS:435967** | **21-225_192B3** | NA | AGGGCCAGTCAGAGTGTTCG CAGCAGCTTCCTTGCC | GGTGCATCTAGCAGGGC CACT | CAGCAGTATGGTAACTCACC GTGGGCG |
| | | | SEQ ID NO:2697 | SEQ ID NO:10709 | SEQ ID NO:18721 |
| | | AA | RASQSVRSSFLA | GASSRAT | QQYGNSPWA |
| | | | SEQ ID NO:2698 | SEQ ID NO:10710 | SEQ ID NO:18722 |
| **iPS:435971** | **21-225_192D3** | NA | CGGGCGAGTCAGGGCATTAG CAATTATTTAGCC | GCTGCATCCAGTTTGCAA AGT | CTACATTATCTTACTTACCCT CTCACT |
| | | | SEQ ID NO:2699 | SEQ ID NO:10711 | SEQ ID NO:18723 |
| | | AA | RASQGISNYLA | AASSLQS | LHYLTYPLT |
| | | | SEQ ID NO:2700 | SEQ ID NO:10712 | SEQ ID NO:18724 |
| **iPS:435973** | **21-225_192H3** | NA | AGGGCCAGTCAGAGTGTTAG CAGCAACTTCTTAGCC | GGTGCATCCAGCAGGGC CACT | CAGCAATATGGTATCTCACC GTGGACG |
| | | | SEQ ID NO:2701 | SEQ ID NO:10713 | SEQ ID NO:18725 |
| | | AA | RASQSVSSNFLA | GASSRAT | QQYGISPWT |
| | | | SEQ ID NO:2702 | SEQ ID NO:10714 | SEQ ID NO:18726 |

## FIGURE 49

| | | | | | |
|---|---|---|---|---|---|
| **iPS:435977** | **21-225_192E4** | NA | CGGGCGAGTCAGGGCATTGGCAATTATTTAGCC | GTTGTATCCAGTTTACAAAGT | CAACGGTATGATACTTACCCATTCACT |
| | | | SEQ ID NO:2703 | SEQ ID NO:10715 | SEQ ID NO:18727 |
| | | AA | RASQGIGNYLA | VVSSLQS | QRYDTYPFT |
| | | | SEQ ID NO:2704 | SEQ ID NO:10716 | SEQ ID NO:18728 |
| **iPS:435979** | **21-225_192H4** | NA | CGGGCGAGTCAGGACATTAGCAATTATTTAGCC | GCTGCATCCAGTTTGCAAAGT | CTACATTATCTCAATTACCCGCTCACT |
| | | | SEQ ID NO:2705 | SEQ ID NO:10717 | SEQ ID NO:18729 |
| | | AA | RASQDISNYLA | AASSLQS | LHYLNYPLT |
| | | | SEQ ID NO:2706 | SEQ ID NO:10718 | SEQ ID NO:18730 |
| **iPS:435983** | **21-225_192E5** | NA | CGGGCCAGTCAGAGCATTGGTAGGAGTTTACAC | TATGCTTCCCAGTCATTCTCA | CATCAGAGTAGTCGTTTACCGCTCACT |
| | | | SEQ ID NO:2707 | SEQ ID NO:10719 | SEQ ID NO:18731 |
| | | AA | RASQSIGRSLH | YASQSFS | HQSSRLPLT |
| | | | SEQ ID NO:2708 | SEQ ID NO:10720 | SEQ ID NO:18732 |
| **iPS:435985** | **21-225_192F6** | NA | CGGGCAAGTCAGGGCATTAGAAATGATTTAGGC | GCTGCATCCAGTTTGCAAAGT | CTACAGCATTATAGTTTCCCATTCACT |
| | | | SEQ ID NO:2709 | SEQ ID NO:10721 | SEQ ID NO:18733 |
| | | AA | RASQGIRNDLG | AASSLQS | LQHYSFPFT |
| | | | SEQ ID NO:2710 | SEQ ID NO:10722 | SEQ ID NO:18734 |
| **iPS:435987** | **21-225_192G6** | NA | CGGACGAGTCAGGGCATTGGCAATTATTTAGCC | AAAGCATCCAGTTTGCAAAGT | CAACAGTATATGACTTACCCGCTCACT |
| | | | SEQ ID NO:2711 | SEQ ID NO:10723 | SEQ ID NO:18735 |
| | | AA | RTSQGIGNYLA | KASSLQS | QQYMTYPLT |
| | | | SEQ ID NO:2712 | SEQ ID NO:10724 | SEQ ID NO:18736 |
| **iPS:435989** | **21-225_192F7** | NA | CGGGCAAGTCAGGGCATTAGAAAAGATTTAGGC | ACTGCATCCAGTTTGCAAAGT | CTACAGCATACTAGTTACCCGTGGACG |
| | | | SEQ ID NO:2713 | SEQ ID NO:10725 | SEQ ID NO:18737 |
| | | AA | RASQGIRKDLG | TASSLQS | LQHTSYPWT |
| | | | SEQ ID NO:2714 | SEQ ID NO:10726 | SEQ ID NO:18738 |

FIGURE 49

| | | | | | |
|---|---|---|---|---|---|
| **iPS:435993** | **21-225_192C8** | NA | CGGGCGAGTCAGGGCATTAG CAATTATTTAGCC | GCTGCATCCAGTTTGCAA AGT | CAACATTATCTTACTTACCCT CTCACT |
| | | | SEQ ID NO:2715 | SEQ ID NO:10727 | SEQ ID NO:18739 |
| | | AA | RASQGISNYLA | AASSLQS | QHYLTYPLT |
| | | | SEQ ID NO:2716 | SEQ ID NO:10728 | SEQ ID NO:18740 |
| **iPS:435995** | **21-225_192F8** | NA | AGGGCCAGTCAGAGTATTAG CAGCAGCTACTTAGCC | GGTGCATCCAGCAGGGC CACT | CAGCAGTATGAGAGTTCACC GTGGACG |
| | | | SEQ ID NO:2717 | SEQ ID NO:10729 | SEQ ID NO:18741 |
| | | AA | RASQSISSSYLA | GASSRAT | QQYESSPWT |
| | | | SEQ ID NO:2718 | SEQ ID NO:10730 | SEQ ID NO:18742 |
| **iPS:435997** | **21-225_192G8** | NA | CGGACGAGTCAGGGCATTGG CAATTATTTAGCC | AAAGCATCCAGTTTGCA AAGT | CAACAGTATATCACTTACCC GCTCACT |
| | | | SEQ ID NO:2719 | SEQ ID NO:10731 | SEQ ID NO:18743 |
| | | AA | RTSQGIGNYLA | KASSLQS | QQYITYPLT |
| | | | SEQ ID NO:2720 | SEQ ID NO:10732 | SEQ ID NO:18744 |
| **iPS:435999** | **21-225_192F9** | NA | AAGTCTAGTCAGAGCCTCCT CCATAGTGATGGAAGGACCT ATTTGTAT | GAGGTTTCCAACCGGTTC GCT | ATGCAAAGTATACAGCTTCC CTGGACG |
| | | | SEQ ID NO:2721 | SEQ ID NO:10733 | SEQ ID NO:18745 |
| | | AA | KSSQSLLHSDGRTYLY | EVSNRFA | MQSIQLPWT |
| | | | SEQ ID NO:2722 | SEQ ID NO:10734 | SEQ ID NO:18746 |
| **iPS:436001** | **21-225_192C10** | NA | CGGGCGAGTCAGGGCATTGG CAAGTATTTAGCC | GCTGCATCCAGTTTGCAA AGT | CAACGATATGATACTTACCC ATTCACT |
| | | | SEQ ID NO:2723 | SEQ ID NO:10735 | SEQ ID NO:18747 |
| | | AA | RASQGIGKYLA | AASSLQS | QRYDTYPFT |
| | | | SEQ ID NO:2724 | SEQ ID NO:10736 | SEQ ID NO:18748 |
| **iPS:436003** | **21-225_192G10** | NA | CGGGCAAGTCAGAGCATTAG CAACTATTTAAAT | GCTGAATCCAGTTTACA AAGT | CAACAGAGTTACAGTTCCCC TCCGTGGACG |
| | | | SEQ ID NO:2725 | SEQ ID NO:10737 | SEQ ID NO:18749 |
| | | AA | RASQSISNYLN | AESSLQS | QQSYSSPPWT |

FIGURE 49

| | | | | | |
|---|---|---|---|---|---|
| | | | SEQ ID NO:2726 | SEQ ID NO:10738 | SEQ ID NO:18750 |
| **iPS:436005** | **21-225_192H10** | NA | CGGGCGAGTCAGGGCATTGG CAAGTATTTAGCC | GCTGCATCCAGTTTGCAA AGT | CAACAGTATAGTACTTACCC GCTCACT |
| | | | SEQ ID NO:2727 | SEQ ID NO:10739 | SEQ ID NO:18751 |
| | | AA | RASQGIGKYLA | AASSLQS | QQYSTYPLT |
| | | | SEQ ID NO:2728 | SEQ ID NO:10740 | SEQ ID NO:18752 |
| **iPS:436007** | **21-225_192G12** | NA | AGGGCCAGTCAGAGTGTCAG AAGCGACTTCTTAGCC | GGTGTATCCCGCAGGGC CACT | CAGCAGTATGGTAACTCACC GTGGACG |
| | | | SEQ ID NO:2729 | SEQ ID NO:10741 | SEQ ID NO:18753 |
| | | AA | RASQSVRSDFLA | GVSRRAT | QQYGNSPWT |
| | | | SEQ ID NO:2730 | SEQ ID NO:10742 | SEQ ID NO:18754 |
| **iPS:436009** | **21-225_193A1** | NA | AGGGCCAGTCAGAGTGTTAG AAGCAACTTCTTAGCC | GGTGCATCCCGCAGGGC CACT | CAGCAGTATGGTAACTCACC GTGGACG |
| | | | SEQ ID NO:2731 | SEQ ID NO:10743 | SEQ ID NO:18755 |
| | | AA | RASQSVRSNFLA | GASRRAT | QQYGNSPWT |
| | | | SEQ ID NO:2732 | SEQ ID NO:10744 | SEQ ID NO:18756 |
| **iPS:436011** | **21-225_193B1** | NA | AGGGCCAGTCAGAGTGTTAG AAGCAACTTCTTAGCC | GGTGCATCCCGCAGGGC CACT | CAGCAGTATGGTAACTCACC GTGGACG |
| | | | SEQ ID NO:2733 | SEQ ID NO:10745 | SEQ ID NO:18757 |
| | | AA | RASQSVRSNFLA | GASRRAT | QQYGNSPWT |
| | | | SEQ ID NO:2734 | SEQ ID NO:10746 | SEQ ID NO:18758 |
| **iPS:436013** | **21-225_193F2** | NA | CGGGCGAGTCAGGGTATTAA CAACTGGTTAGCC | GGTGTTTCCAGTTTGCAA AGT | CAACAGGCTAACAGTTTCCC GTGGACG |
| | | | SEQ ID NO:2735 | SEQ ID NO:10747 | SEQ ID NO:18759 |
| | | AA | RASQGINNWLA | GVSSLQS | QQANSFPWT |
| | | | SEQ ID NO:2736 | SEQ ID NO:10748 | SEQ ID NO:18760 |
| **iPS:436015** | **21-225_193D3** | NA | AGGGCCAGTCAGAGTATTCG CAGCAGCTTCTTAGCC | GGTGCATCCAACAGGGC CACT | CAGCAGTATGGTAACTCACC GTGGGCG |
| | | | SEQ ID NO:2737 | SEQ ID NO:10749 | SEQ ID NO:18761 |
| | | AA | RASQSIRSSFLA | GASNRAT | QQYGNSPWA |

**FIGURE 49**

| | | | | | |
|---|---|---|---|---|---|
| | | | SEQ ID NO:2738 | SEQ ID NO:10750 | SEQ ID NO:18762 |
| **iPS:436017** | **21-225_193F3** | NA | AGGGCCGGTCAAAGTATTAGAACCGACTTCTTAGTC | GGTGCATCCAGCAGGGCCACT | CAGCAGTATGGAAGCTCACCGTGGACG |
| | | | SEQ ID NO:2739 | SEQ ID NO:10751 | SEQ ID NO:18763 |
| | | AA | RAGQSIRTDFLV | GASSRAT | QQYGSSPWT |
| | | | SEQ ID NO:2740 | SEQ ID NO:10752 | SEQ ID NO:18764 |
| **iPS:436019** | **21-225_193C4** | NA | CGGCCGAGTCAGGGCATTAGCATTTATTTAGCC | GCTGCATCCACTTTACAATCA | CAAAAGTATAACAGTGCCCCATTCACT |
| | | | SEQ ID NO:2741 | SEQ ID NO:10753 | SEQ ID NO:18765 |
| | | AA | RPSQGISIYLA | AASTLQS | QKYNSAPFT |
| | | | SEQ ID NO:2742 | SEQ ID NO:10754 | SEQ ID NO:18766 |
| **iPS:436021** | **21-225_193G4** | NA | AAGTCCAGCCAGAGTGTTTTATACAGCTCCAACAATTATAACTACTTGACT | TGGGCATCTACCCGAAAATCC | CAGCAATATTATATTACTCCGTGGACG |
| | | | SEQ ID NO:2743 | SEQ ID NO:10755 | SEQ ID NO:18767 |
| | | AA | KSSQSVLYSSNNYNYLT | WASTRKS | QQYYITPWT |
| | | | SEQ ID NO:2744 | SEQ ID NO:10756 | SEQ ID NO:18768 |
| **iPS:436023** | **21-225_193A5** | NA | CGGGCAAGTCAGGGCATTAGAAATGATTTAGGG | GCTGCATCCAGTTTGCAAAGT | CTACAGCATAATGATTTCCCGTTCACT |
| | | | SEQ ID NO:2745 | SEQ ID NO:10757 | SEQ ID NO:18769 |
| | | AA | RASQGIRNDLG | AASSLQS | LQHNDFPFT |
| | | | SEQ ID NO:2746 | SEQ ID NO:10758 | SEQ ID NO:18770 |
| **iPS:436025** | **21-225_193B5** | NA | CGGGCCAGTCAGAGCATTGGTAGTAGCTTACAC | TATGCTTCCCAGTCCTTCTCA | CATCAGAGTAGTCGTTTACCATTCACT |
| | | | SEQ ID NO:2747 | SEQ ID NO:10759 | SEQ ID NO:18771 |
| | | AA | RASQSIGSSLH | YASQSFS | HQSSRLPFT |
| | | | SEQ ID NO:2748 | SEQ ID NO:10760 | SEQ ID NO:18772 |
| **iPS:436027** | **21-225_193E6** | NA | AGGGCCAGTCAGAGTGTTAGGAGCGGTTACTTAGCC | GGTGCATCCAGCAGGGCCACT | CAGCAGTATGAGAGTTCACCGTGGACG |
| | | | SEQ ID NO:2749 | SEQ ID NO:10761 | SEQ ID NO:18773 |

FIGURE 49

| | | | | | |
|---|---|---|---|---|---|
| | | AA | RASQSVRSGYLA | GASSRAT | QQYESSPWT |
| | | | SEQ ID NO:2750 | SEQ ID NO:10762 | SEQ ID NO:18774 |
| iPS:436029 | 21-225_193H6 | NA | AGGGCCGGTCAAAGTATTAGAACCAACTTCTTAGCC | GGTGCATCCAGCAGGGCCACT | CAGCAGTATGGTAGCTCACCGTGGACG |
| | | | SEQ ID NO:2751 | SEQ ID NO:10763 | SEQ ID NO:18775 |
| | | AA | RAGQSIRTNFLA | GASSRAT | QQYGSSPWT |
| | | | SEQ ID NO:2752 | SEQ ID NO:10764 | SEQ ID NO:18776 |
| iPS:436031 | 21-225_193C7 | NA | CGGGCGAGTCAGGGCATTGGCAAGTATTTAGCC | GCTGCATCCAGTTTGCAAAGT | CAACAGTATAGTACTTACCCGCTCACT |
| | | | SEQ ID NO:2753 | SEQ ID NO:10765 | SEQ ID NO:18777 |
| | | AA | RASQGIGKYLA | AASSLQS | QQYSTYPLT |
| | | | SEQ ID NO:2754 | SEQ ID NO:10766 | SEQ ID NO:18778 |
| iPS:436033 | 21-225_193E7 | NA | CGGGCAAGTCAGGGCATTAGAAATGATTTAGGC | TCTGCATCCAGTTTGCAAAGG | CTACAGCATAAAAGGTACCCGCTCACT |
| | | | SEQ ID NO:2755 | SEQ ID NO:10767 | SEQ ID NO:18779 |
| | | AA | RASQGIRNDLG | SASSLQR | LQHKRYPLT |
| | | | SEQ ID NO:2756 | SEQ ID NO:10768 | SEQ ID NO:18780 |
| iPS:436035 | 21-225_193C8 | NA | AGGGCCAGTCAGAGTATTCGCAGCAGCTTCTTAGCC | GGTGCATCCAGCAGGGCCACT | CAGCAATATGGTAACTCACCGTGGGCG |
| | | | SEQ ID NO:2757 | SEQ ID NO:10769 | SEQ ID NO:18781 |
| | | AA | RASQSIRSSFLA | GASSRAT | QQYGNSPWA |
| | | | SEQ ID NO:2758 | SEQ ID NO:10770 | SEQ ID NO:18782 |
| iPS:436037 | 21-225_193D8 | NA | AGGGCCAGTCAGAGTATAAGGACCAACTTCTTAGCC | GGTGCATCCAGCAGGGCCACT | CAGCAGTATGGTAACTCACCGTGGACG |
| | | | SEQ ID NO:2759 | SEQ ID NO:10771 | SEQ ID NO:18783 |
| | | AA | RASQSIRTNFLA | GASSRAT | QQYGNSPWT |
| | | | SEQ ID NO:2760 | SEQ ID NO:10772 | SEQ ID NO:18784 |
| iPS:436039 | 21-225_193F8 | NA | CGGGCGAGTCAGGGCGTTAGCAATCATTTAGCC | GCTGCATCCAGTTTGCAAAGT | CAACAGTATAATAGTTACCCATTCACT |
| | | | SEQ ID NO:2761 | SEQ ID NO:10773 | SEQ ID NO:18785 |

FIGURE 49

| | | | | | |
|---|---|---|---|---|---|
| | | AA | RASQGVSNHLA | AASSLQS | QQYNSYPFT |
| | | | SEQ ID NO:2762 | SEQ ID NO:10774 | SEQ ID NO:18786 |
| **iPS:436041** | **21-225_193G8** | NA | AGGGCCAGTCAGAGTGTTAG AACCAACTTCTTAGCC | GGTGCATCCCGCAGGGC CACT | CAGCAGTATGGTAACTCACC GTGGACG |
| | | | SEQ ID NO:2763 | SEQ ID NO:10775 | SEQ ID NO:18787 |
| | | AA | RASQSVRTNFLA | GASRRAT | QQYGNSPWT |
| | | | SEQ ID NO:2764 | SEQ ID NO:10776 | SEQ ID NO:18788 |
| **iPS:436043** | **21-225_193G9** | NA | CGGGCCAGTCAGAGCATTGG TAGGAGTTTACAC | TATGCTTCCCAGTCATTC TCA | CATCAGAGTAGTCGTTTACC GCTCACT |
| | | | SEQ ID NO:2765 | SEQ ID NO:10777 | SEQ ID NO:18789 |
| | | AA | RASQSIGRSLH | YASQSFS | HQSSRLPLT |
| | | | SEQ ID NO:2766 | SEQ ID NO:10778 | SEQ ID NO:18790 |
| **iPS:436045** | **21-225_193A10** | NA | CGGGCGAGTCAGGGCATTAG CAATTATTTAGCC | GCTGCATCCAGTTTGCAA AGT | CAACATTATCTTACTTACCCT CTCACT |
| | | | SEQ ID NO:2767 | SEQ ID NO:10779 | SEQ ID NO:18791 |
| | | AA | RASQGISNYLA | AASSLQS | QHYLTYPLT |
| | | | SEQ ID NO:2768 | SEQ ID NO:10780 | SEQ ID NO:18792 |
| **iPS:436047** | **21-225_193B10** | NA | AGGGCCAGTCCGAGTGTTAG CAGCAGCTACTTAGCC | GGTGCATCCAGGAGGGC CACT | CAGCAGTATGGTAGCTCACC TCCGTGGACG |
| | | | SEQ ID NO:2769 | SEQ ID NO:10781 | SEQ ID NO:18793 |
| | | AA | RASPSVSSSYLA | GASRRAT | QQYGSSPPWT |
| | | | SEQ ID NO:2770 | SEQ ID NO:10782 | SEQ ID NO:18794 |
| **iPS:436049** | **21-225_193B12** | NA | AGGGCCAGTCAGAGTATTCG CAGCAGCTTCTTAGCC | GATGCATCCAGCAGGGC CACT | CAGCAGTATGGTAACTCACC GTGGGCG |
| | | | SEQ ID NO:2771 | SEQ ID NO:10783 | SEQ ID NO:18795 |
| | | AA | RASQSIRSSFLA | DASSRAT | QQYGNSPWA |
| | | | SEQ ID NO:2772 | SEQ ID NO:10784 | SEQ ID NO:18796 |
| **iPS:436051** | **21-225_193G12** | NA | AGGGCCAGTCAGAGTGTTAG CAGCAGCTTAGCC | GGTGCATCCACCAGGGC CACT | CAGCAGTATAATAACTGGCC GTGCAGT |
| | | | SEQ ID NO:2773 | SEQ ID NO:10785 | SEQ ID NO:18797 |

**FIGURE 49**

| | | | | | |
|---|---|---|---|---|---|
| | | AA | RASQSVSSSLA | GASTRAT | QQYNNWPCS |
| | | | SEQ ID NO:2774 | SEQ ID NO:10786 | SEQ ID NO:18798 |
| **iPS:436054** | **21-225_194C1** | NA | CGGGCGAGTCAGGGCATTAG CAATTATTTAGCC | GCTGCATCCAGTTTGCAA AGT | CTACATTATCTTACTTACCCT CTCACT |
| | | | SEQ ID NO:2775 | SEQ ID NO:10787 | SEQ ID NO:18799 |
| | | AA | RASQGISNYLA | AASSLQS | LHYLTYPLT |
| | | | SEQ ID NO:2776 | SEQ ID NO:10788 | SEQ ID NO:18800 |
| **iPS:436056** | **21-225_194C3** | NA | CGGGCCAGTCAGAGTATTGG TAGTAACTTACAC | TCTGCTTCCCAGTCCTTC TCA | CAGCAGAGTAGTAGTTTACC GTGGACG |
| | | | SEQ ID NO:2777 | SEQ ID NO:10789 | SEQ ID NO:18801 |
| | | AA | RASQSIGSNLH | SASQSFS | QQSSSLPWT |
| | | | SEQ ID NO:2778 | SEQ ID NO:10790 | SEQ ID NO:18802 |
| **iPS:436058** | **21-225_194A4** | NA | AGGGCCAGTCGGGGTGTTAG CAACATCTACTTAGCC | GGTGCTTCCAACAGGGC CACT | CAGCACAATGATTACTCAAT GTTCACT |
| | | | SEQ ID NO:2779 | SEQ ID NO:10791 | SEQ ID NO:18803 |
| | | AA | RASRGVSNIYLA | GASNRAT | QHNDYSMFT |
| | | | SEQ ID NO:2780 | SEQ ID NO:10792 | SEQ ID NO:18804 |
| **iPS:436060** | **21-225_194F4** | NA | AAGTCTAGTCAGAGCCTCCT CCATAGTGATGGAAGGACCT ATTTGTAT | GAGGTTTCCAACCGGTTC TCT | ATGCAAAGTATACAGCTTCC CTGGACG |
| | | | SEQ ID NO:2781 | SEQ ID NO:10793 | SEQ ID NO:18805 |
| | | AA | KSSQSLLHSDGRTYLY | EVSNRFS | MQSIQLPWT |
| | | | SEQ ID NO:2782 | SEQ ID NO:10794 | SEQ ID NO:18806 |
| **iPS:436062** | **21-225_194E5** | NA | AGGGCCGGTCAAAGTATTAG AACCAACTTCTTAGCC | GGTGCATCCAGCAGGGC CACT | CAGCAGTATGGTAGTTCACC GTGGACG |
| | | | SEQ ID NO:2783 | SEQ ID NO:10795 | SEQ ID NO:18807 |
| | | AA | RAGQSIRTNFLA | GASSRAT | QQYGSSPWT |
| | | | SEQ ID NO:2784 | SEQ ID NO:10796 | SEQ ID NO:18808 |
| **iPS:436064** | | NA | AGGGCCAGTCAGAGTATTAG AAGCAACTTCTTAGCC | GGTGCATCCAGCAGGGC CACT | CAGCAGTATGGTAGCTCACC GTGGACG |

FIGURE 49

| | | | | | |
|---|---|---|---|---|---|
| | **21-225_194E6** | | SEQ ID NO:2785 | SEQ ID NO:10797 | SEQ ID NO:18809 |
| | | AA | RASQSIRSNFLA | GASSRAT | QQYGSSPWT |
| | | | SEQ ID NO:2786 | SEQ ID NO:10798 | SEQ ID NO:18810 |
| **iPS:436066** | **21-225_194B7** | NA | CGGGCGAGTCAGGGCATTAG CAAATATTTAGCC | GGTGCATCCAGGTTGCA AAGT | CAACATTATCTTAATTACCCT CTCACC |
| | | | SEQ ID NO:2787 | SEQ ID NO:10799 | SEQ ID NO:18811 |
| | | AA | RASQGISKYLA | GASRLQS | QHYLNYPLT |
| | | | SEQ ID NO:2788 | SEQ ID NO:10800 | SEQ ID NO:18812 |
| **iPS:436068** | **21-225_194F7** | NA | CGGGCGAGTCAGGGTATTAG CAGGTGGTTAGCC | GCTGCATCCAGTTTGCAA AGT | CAACAGGCTAACAGTTTCCC GTGGACG |
| | | | SEQ ID NO:2789 | SEQ ID NO:10801 | SEQ ID NO:18813 |
| | | AA | RASQGISRWLA | AASSLQS | QQANSFPWT |
| | | | SEQ ID NO:2790 | SEQ ID NO:10802 | SEQ ID NO:18814 |
| **iPS:436072** | **21-225_194C10** | NA | AGGGCCAGTCCGAGTGTTAA CAGCGGCTACTTAGCC | GGTGCATCCAGCAGGGC CACT | CAGCAGTATGAAAGCTCACC GTGGACG |
| | | | SEQ ID NO:2791 | SEQ ID NO:10803 | SEQ ID NO:18815 |
| | | AA | RASPSVNSGYLA | GASSRAT | QQYESSPWT |
| | | | SEQ ID NO:2792 | SEQ ID NO:10804 | SEQ ID NO:18816 |
| **iPS:436074** | **21-225_194F10** | NA | CGGGCAAGTCAGGGCATTAG AAATGATTTAGGC | GCTGCATCCAGTTTACAA AGT | CTACAGCATTATAGTTTCCC ATTCACT |
| | | | SEQ ID NO:2793 | SEQ ID NO:10805 | SEQ ID NO:18817 |
| | | AA | RASQGIRNDLG | AASSLQS | LQHYSFPFT |
| | | | SEQ ID NO:2794 | SEQ ID NO:10806 | SEQ ID NO:18818 |
| **iPS:436076** | **21-225_194H11** | NA | CGGGCGAGTCAGGGCATTAG CAATTATTTAGCC | GCTGCATCCAGTTTACAA AGT | CAACATTATCTTACTTACCCT CTCACT |
| | | | SEQ ID NO:2795 | SEQ ID NO:10807 | SEQ ID NO:18819 |
| | | AA | RASQGISNYLA | AASSLQS | QHYLTYPLT |
| | | | SEQ ID NO:2796 | SEQ ID NO:10808 | SEQ ID NO:18820 |
| **iPS:436078** | | NA | CGGGCGAGTCAGGGCATTGG CAAGTATTTAGCA | GCTGCATCCAGTTTGCAA AGT | CAACGATATGATACTTACCC ATTCACT |

FIGURE 49

| | | | | | |
|---|---|---|---|---|---|
| | **21-225_194H12** | | SEQ ID NO:2797 | SEQ ID NO:10809 | SEQ ID NO:18821 |
| | | AA | RASQGIGKYLA | AASSLQS | QRYDTYPFT |
| | | | SEQ ID NO:2798 | SEQ ID NO:10810 | SEQ ID NO:18822 |
| **iPS:436080** | **21-225_195B1** | NA | AGGGCCAGTCCGAGTGTTAACAGTAACTACTTAGCC | GGTGCATCCAACAGGGCCACT | CAGCAGTATGAAAGCTCACCGTGGACG |
| | | | SEQ ID NO:2799 | SEQ ID NO:10811 | SEQ ID NO:18823 |
| | | AA | RASPSVNSNYLA | GASNRAT | QQYESSPWT |
| | | | SEQ ID NO:2800 | SEQ ID NO:10812 | SEQ ID NO:18824 |
| **iPS:436082** | **21-225_195D9** | NA | CGGGCGAGTCAGGGTATTAGCAGGTGGTTAGCC | GCTGCATCCAGTTTGCTAGGT | CAACGGGCTAACAGTTTCCCGTGCAGT |
| | | | SEQ ID NO:2801 | SEQ ID NO:10813 | SEQ ID NO:18825 |
| | | AA | RASQGISRWLA | AASSLLG | QRANSFPCS |
| | | | SEQ ID NO:2802 | SEQ ID NO:10814 | SEQ ID NO:18826 |
| **iPS:436084** | **21-225_195F2** | NA | CGGGCCAGTCAGAGCATTGGTAGTAGCTTACAC | TATGCTTCCCAGTCCTTCTCA | CATCAGAGTAGAACTTTACCGCTCACT |
| | | | SEQ ID NO:2803 | SEQ ID NO:10815 | SEQ ID NO:18827 |
| | | AA | RASQSIGSSLH | YASQSFS | HQSRTLPLT |
| | | | SEQ ID NO:2804 | SEQ ID NO:10816 | SEQ ID NO:18828 |
| **iPS:436086** | **21-225_191G10** | NA | CGGACGAGTCAGGGCATTGGCAAGTATTTAGCC | AAAGTATCCAGTTTGCAAAGT | CAACAGTATATGACTTACCCGCTCACT |
| | | | SEQ ID NO:2805 | SEQ ID NO:10817 | SEQ ID NO:18829 |
| | | AA | RTSQGIGKYLA | KVSSLQS | QQYMTYPLT |
| | | | SEQ ID NO:2806 | SEQ ID NO:10818 | SEQ ID NO:18830 |
| **iPS:436088** | **21-225_195C8** | NA | AGGGCCAGTCAGAGTATTCGCAGCAGCTTCTTAGCC | GGTGCATTTAGTAGGGCCACT | CAGCAGTATGGTAACTCACCGTGGGCG |
| | | | SEQ ID NO:2807 | SEQ ID NO:10819 | SEQ ID NO:18831 |
| | | AA | RASQSIRSSFLA | GAFSRAT | QQYGNSPWA |
| | | | SEQ ID NO:2808 | SEQ ID NO:10820 | SEQ ID NO:18832 |
| **iPS:436090** | | NA | CGGGCGAGTCAGGGCATTAGCAATTATTTAGCC | GCTGCATCCAGTTTGCAAAGT | CAACATTATCTTACTTACCCTCTCACT |

**FIGURE 49**

| | | | | | |
|---|---|---|---|---|---|
| | **21-225_195A9** | | SEQ ID NO:2809 | SEQ ID NO:10821 | SEQ ID NO:18833 |
| | | AA | RASQGISNYLA | AASSLQS | QHYLTYPLT |
| | | | SEQ ID NO:2810 | SEQ ID NO:10822 | SEQ ID NO:18834 |
| **iPS:436092** | **21-225_195B9** | NA | CGGGCAAGTCAGGGCATAA GAAAAGATTTAGGC | GCTGCATCCGATTTGCAA AGT | CTACAGCATTATCGTTACCC ATTCACT |
| | | | SEQ ID NO:2811 | SEQ ID NO:10823 | SEQ ID NO:18835 |
| | | AA | RASQGIRKDLG | AASDLQS | LQHYRYPFT |
| | | | SEQ ID NO:2812 | SEQ ID NO:10824 | SEQ ID NO:18836 |
| **iPS:436094** | **21-225_195B10** | NA | CGGGCCAGTCAGAGCATTGG TAGTAGCTTACAC | TATGCTTCCCAGTCCTTC TCA | CATCAGAGTGGTCGTTTACC GCTCACT |
| | | | SEQ ID NO:2813 | SEQ ID NO:10825 | SEQ ID NO:18837 |
| | | AA | RASQSIGSSLH | YASQSFS | HQSGRLPLT |
| | | | SEQ ID NO:2814 | SEQ ID NO:10826 | SEQ ID NO:18838 |
| **iPS:436096** | **21-225_195E10** | NA | CGGGCCAGTCAGAGCATTGG TAGTAGCTTACAC | TATGCTTCCCAGTCCTTC TCA | CATCAGAGTAGTCGTTTACC ATTCACT |
| | | | SEQ ID NO:2815 | SEQ ID NO:10827 | SEQ ID NO:18839 |
| | | AA | RASQSIGSSLH | YASQSFS | HQSSRLPFT |
| | | | SEQ ID NO:2816 | SEQ ID NO:10828 | SEQ ID NO:18840 |
| **iPS:436098** | **21-225_195G11** | NA | AAGTCCAGCCAGAGTGTTTT ATTCAACTCCAACAATAAGA ACTACTTAGCT | TGGGCATCTACCCTGGA ATCT | CAGCAATATTGTAGTTTTCC ATTCACT |
| | | | SEQ ID NO:2817 | SEQ ID NO:10829 | SEQ ID NO:18841 |
| | | AA | KSSQSVLFNSNNKNYLA | WASTLES | QQYCSFPFT |
| | | | SEQ ID NO:2818 | SEQ ID NO:10830 | SEQ ID NO:18842 |
| **iPS:436100** | **21-225_195G12** | NA | CGGGCGAGTCAGGGTATTAA CAACTGGTTAGCC | GGTGTTTCCAGTTTGCAG AGT | CAACAGGCTAACAGTTTCCC GTGGACG |
| | | | SEQ ID NO:2819 | SEQ ID NO:10831 | SEQ ID NO:18843 |
| | | AA | RASQGINNWLA | GVSSLQS | QQANSFPWT |
| | | | SEQ ID NO:2820 | SEQ ID NO:10832 | SEQ ID NO:18844 |

FIGURE 49

| | | | | | |
|---|---|---|---|---|---|
| **iPS:436102** | **21-225_196B1** | NA | AAGTCCAGCCAGAGTATTTTATTCAGCTCCAACAATAAGAGGTACTTAGCT | TGGGCATCTATCCGGGAATCC | CAGCAATATTCTAGTCTTCCATTCACT |
| | | | SEQ ID NO:2821 | SEQ ID NO:10833 | SEQ ID NO:18845 |
| | | AA | KSSQSILFSSNNKRYLA | WASIRES | QQYSSLPFT |
| | | | SEQ ID NO:2822 | SEQ ID NO:10834 | SEQ ID NO:18846 |
| **iPS:436104** | **21-225_196C1** | NA | AAGTCCAGCCAGAGTGTTTTATTCAACTCCAACAATAAGAACTACTTAGCT | TGGGCATCTACCCTGGAATCT | CAGCAATATTGTAGTTTTCCATTCACT |
| | | | SEQ ID NO:2823 | SEQ ID NO:10835 | SEQ ID NO:18847 |
| | | AA | KSSQSVLFNSNNKNYLA | WASTLES | QQYCSFPFT |
| | | | SEQ ID NO:2824 | SEQ ID NO:10836 | SEQ ID NO:18848 |
| **iPS:436106** | **21-225_196F2** | NA | CGGGCGAGTCAGGGTATTAGCAGCTGGTTAGCC | GCTGCATCCAGTTTGCAAAGT | CAACAGACTAACAGTGTCCCATTCACT |
| | | | SEQ ID NO:2825 | SEQ ID NO:10837 | SEQ ID NO:18849 |
| | | AA | RASQGISSWLA | AASSLQS | QQTNSVPFT |
| | | | SEQ ID NO:2826 | SEQ ID NO:10838 | SEQ ID NO:18850 |
| **iPS:436110** | **21-225_196F4** | NA | CGGGCAAGTCAGCGCATTCACAGCTATTTAAAT | ACTGCATCCAGTTTGCAAGGT | CAACAGAGCTACGGTTCCCCTCTCACT |
| | | | SEQ ID NO:2827 | SEQ ID NO:10839 | SEQ ID NO:18851 |
| | | AA | RASQRIHSYLN | TASSLQG | QQSYGSPLT |
| | | | SEQ ID NO:2828 | SEQ ID NO:10840 | SEQ ID NO:18852 |
| **iPS:436112** | **21-225_196C7** | NA | CGGGCGAATCAGGCCATTAGCAATTATTTAGCC | GCTGCATCCAGTTTGCAAAGT | CAACATTATCTCACTTACCCTCTCACT |
| | | | SEQ ID NO:2829 | SEQ ID NO:10841 | SEQ ID NO:18853 |
| | | AA | RANQAISNYLA | AASSLQS | QHYLTYPLT |
| | | | SEQ ID NO:2830 | SEQ ID NO:10842 | SEQ ID NO:18854 |
| **iPS:436114** | **21 225 196G8** | NA | AAGTCCAGCCAGAGTGTTTTACACAGCTCCAACAATAACAACTACTTAGCT | TGGGCATCTACCCGGGAATCC | CAGCAATATTATAATACTCCTCCGACA |

FIGURE 49

| | | | | | |
|---|---|---|---|---|---|
| | 21-225_196G8 | | SEQ ID NO:2831 | SEQ ID NO:10843 | SEQ ID NO:18855 |
| | | AA | KSSQSVLHSSNNNNYLA | WASTRES | QQYYNTPPT |
| | | | SEQ ID NO:2832 | SEQ ID NO:10844 | SEQ ID NO:18856 |
| **iPS:436116** | 21-225_196B9 | NA | CGGGCGAGTCAGGGTATTAG CAACTGCTTAGCC | GCTGCATCCAGTTTGCAA AGT | CAACAGGGTGACAGTTTCCC TCCGACG |
| | | | SEQ ID NO:2833 | SEQ ID NO:10845 | SEQ ID NO:18857 |
| | | AA | RASQGISNCLA | AASSLQS | QQGDSFPPT |
| | | | SEQ ID NO:2834 | SEQ ID NO:10846 | SEQ ID NO:18858 |
| **iPS:436118** | 21-225_196A10 | NA | CGGGCGAGTCAGGGTATTAG CAGGTGGTTAGCC | GCTGCATCCAGTTTGCTA GGT | CAACGGGATAACAGTTTACC GTGCAGT |
| | | | SEQ ID NO:2835 | SEQ ID NO:10847 | SEQ ID NO:18859 |
| | | AA | RASQGISRWLA | AASSLLG | QRDNSLPCS |
| | | | SEQ ID NO:2836 | SEQ ID NO:10848 | SEQ ID NO:18860 |
| **iPS:436120** | 21-225_196C10 | NA | CGGGCAAGTCAGGGCATTAG AAATGATTTAGGC | GCTGCATCCAGTTTGCAA AGT | CTACAATATAATAGTTACCC TCTCACT |
| | | | SEQ ID NO:2837 | SEQ ID NO:10849 | SEQ ID NO:18861 |
| | | AA | RASQGIRNDLG | AASSLQS | LQYNSYPLT |
| | | | SEQ ID NO:2838 | SEQ ID NO:10850 | SEQ ID NO:18862 |
| **iPS:436122** | 21-225_196G10 | NA | AGGGCCAGTCCGAGTGTTAG CAACAGCTTCTTAGCC | GGTGCATCCAGCAGGGC CACT | CAGCAGTATGGTAGCTCACC TCCGTGGACG |
| | | | SEQ ID NO:2839 | SEQ ID NO:10851 | SEQ ID NO:18863 |
| | | AA | RASPSVSNSFLA | GASSRAT | QQYGSSPPWT |
| | | | SEQ ID NO:2840 | SEQ ID NO:10852 | SEQ ID NO:18864 |
| **iPS:436132** | 21-225_196C12 | NA | CGGGCAAGTCAGGGCATTAG AAATGATCTAGGC | GCTGCATCCAGTTTGCAA AGT | CTACAGCATAATGATTTCCC GTTCACT |
| | | | SEQ ID NO:2841 | SEQ ID NO:10853 | SEQ ID NO:18865 |
| | | AA | RASQGIRNDLG | AASSLQS | LQHNDFPFT |
| | | | SEQ ID NO:2842 | SEQ ID NO:10854 | SEQ ID NO:18866 |
| **iPS:436134** | | NA | AGGGCCAGTCAGAGTGTTAG AAGCAACTTCTTAGCC | GGTGCATACCGCAGGGC CACT | CAGCAGTATGGTAACTCACC GTGGACG |

FIGURE 49

| | | | | | |
|---|---|---|---|---|---|
| | **21-225_196H12** | | SEQ ID NO:2843 | SEQ ID NO:10855 | SEQ ID NO:18867 |
| | | AA | RASQSVRSNFLA | GAYRRAT | QQYGNSPWT |
| | | | SEQ ID NO:2844 | SEQ ID NO:10856 | SEQ ID NO:18868 |
| **iPS:436138** | **21-225_197F2** | NA | CGGACGAGTCAGGGCATTGG CAATTATTTAGCC | AAAACATCCAGTTTGCA AAGT | CAACAATATATCACTTACCC GCTCACT |
| | | | SEQ ID NO:2845 | SEQ ID NO:10857 | SEQ ID NO:18869 |
| | | AA | RTSQGIGNYLA | KTSSLQS | QQYITYPLT |
| | | | SEQ ID NO:2846 | SEQ ID NO:10858 | SEQ ID NO:18870 |
| **iPS:436140** | **21-225_197G3** | NA | CGGGCGAGTCAGGGCATTGG CAATCATTTAGCC | GCTGCATCCAGTTTGCAA AGT | CAACAGTATAGTAATTACCC GGTCACT |
| | | | SEQ ID NO:2847 | SEQ ID NO:10859 | SEQ ID NO:18871 |
| | | AA | RASQGIGNHLA | AASSLQS | QQYSNYPVT |
| | | | SEQ ID NO:2848 | SEQ ID NO:10860 | SEQ ID NO:18872 |
| **iPS:436146** | **21-225_197F4** | NA | AGGGCCAGTCAGAGTATTCG CAGCAGCTTCTTAGCC | GGTGCATCCAGCAGGGC CACT | CAGCAGTATGGAAACTCACC GTGGGCG |
| | | | SEQ ID NO:2849 | SEQ ID NO:10861 | SEQ ID NO:18873 |
| | | AA | RASQSIRSSFLA | GASSRAT | QQYGNSPWA |
| | | | SEQ ID NO:2850 | SEQ ID NO:10862 | SEQ ID NO:18874 |
| **iPS:436150** | **21-225_197H4** | NA | AGGTCCAGCCAGAGTGTTTT ACACAGCTTCAACAATTACA ACTACTTAGCT | TGGGCATCTACCCGGGA ATCC | CAACAATATTATAGTACTCC TCCGACG |
| | | | SEQ ID NO:2851 | SEQ ID NO:10863 | SEQ ID NO:18875 |
| | | AA | RSSQSVLHSFNNYNYLA | WASTRES | QQYYSTPPT |
| | | | SEQ ID NO:2852 | SEQ ID NO:10864 | SEQ ID NO:18876 |
| **iPS:436152** | **21-225_197B6** | NA | CGGGCGAGTCAGGGCATTGG CAAATATTTAGCC | GGTGCATCCAGTTTGCA AAGT | CAACAATATAGTACTTACCC GCTCACT |
| | | | SEQ ID NO:2853 | SEQ ID NO:10865 | SEQ ID NO:18877 |
| | | AA | RASQGIGKYLA | GASSLQS | QQYSTYPLT |
| | | | SEQ ID NO:2854 | SEQ ID NO:10866 | SEQ ID NO:18878 |

FIGURE 49

| | | | | | |
|---|---|---|---|---|---|
| **iPS:436154** | **21-225_197C6** | NA | AGGTCCAGCCAGAGTGTTTTACACAGCTCCAACAATTACAACTACTTAGCT | TGGGCATCTACCCGGGAATCC | CAACAATATTATAGTACTCCTCCGACG |
| | | | SEQ ID NO:2855 | SEQ ID NO:10867 | SEQ ID NO:18879 |
| | | AA | RSSQSVLHSSNNYNYLA | WASTRES | QQYYSTPPT |
| | | | SEQ ID NO:2856 | SEQ ID NO:10868 | SEQ ID NO:18880 |
| **iPS:436156** | **21-225_197C8** | NA | AAGTCCAGCCAGAGTGTTTTACACAGTTCCAACAATAAGAACTACTTAGCT | TGGGCATCTACCCGGGAATCC | CAGCAGTCTTATACTATTCCATTCACT |
| | | | SEQ ID NO:2857 | SEQ ID NO:10869 | SEQ ID NO:18881 |
| | | AA | KSSQSVLHSSNNKNYLA | WASTRES | QQSYTIPFT |
| | | | SEQ ID NO:2858 | SEQ ID NO:10870 | SEQ ID NO:18882 |
| **iPS:436158** | **21-225_197G8** | NA | AAGTCTAGTCAGAACCTCCTGCATAGTGATGGAAAGACCTATTTGTAT | GAAGTTTCCAACCGGTTCTCT | ATGCAAAGTATACAGCTTCCCTGGACG |
| | | | SEQ ID NO:2859 | SEQ ID NO:10871 | SEQ ID NO:18883 |
| | | AA | KSSQNLLHSDGKTYLY | EVSNRFS | MQSIQLPWT |
| | | | SEQ ID NO:2860 | SEQ ID NO:10872 | SEQ ID NO:18884 |
| **iPS:436160** | **21-225_197C9** | NA | CGGGCGAGTCAGGGTATTAGCAACTGGTTAGCC | GCTGCATCCAGTTTGCAAAGT | CAACAGGCTAACAGTTTCCCGTGGACG |
| | | | SEQ ID NO:2861 | SEQ ID NO:10873 | SEQ ID NO:18885 |
| | | AA | RASQGISNWLA | AASSLQS | QQANSFPWT |
| | | | SEQ ID NO:2862 | SEQ ID NO:10874 | SEQ ID NO:18886 |
| **iPS:436164** | **21-225_197G10** | NA | CGGGCAAGTCAGGGCATTAGAAATGATTTAGGC | GGTGCATCCAGTTTACAAAGT | CTACAGCATTATAGGTACCCATTCACT |
| | | | SEQ ID NO:2863 | SEQ ID NO:10875 | SEQ ID NO:18887 |
| | | AA | RASQGIRNDLG | GASSLQS | LQHYRYPFT |
| | | | SEQ ID NO:2864 | SEQ ID NO:10876 | SEQ ID NO:18888 |
| **iPS:436167** | | NA | CGGGCGAGTCAGGGCATTAACAAGTATTTATCC | GCTGCATCCAGTGTGCAAAGT | CAACGATATGACACTTACCCATTCACT |

**FIGURE 49**

| | | | | | |
|---|---|---|---|---|---|
| | **21-225_197E11** | | SEQ ID NO:2865 | SEQ ID NO:10877 | SEQ ID NO:18889 |
| | | AA | RASQGINKYLS | AASSVQS | QRYDTYPFT |
| | | | SEQ ID NO:2866 | SEQ ID NO:10878 | SEQ ID NO:18890 |
| **iPS:436173** | **21-225_197G12** | NA | CGGACGAGTCAGGGCATTGG CAATTATTTAGCC | AAAACATCCAGTTTGCA AAGT | CAACAATATATGACTTACCC GCTCACT |
| | | | SEQ ID NO:2867 | SEQ ID NO:10879 | SEQ ID NO:18891 |
| | | AA | RTSQGIGNYLA | KTSSLQS | QQYMTYPLT |
| | | | SEQ ID NO:2868 | SEQ ID NO:10880 | SEQ ID NO:18892 |
| **iPS:436177** | **21-225_198B1** | NA | AGGGCCGGTCAAAGTATTAG AACCAACTTCTTAGCC | GGTGCATCCAGCAGGGC CACT | CAGCAGTATGGTAGCTCACC GTGGACG |
| | | | SEQ ID NO:2869 | SEQ ID NO:10881 | SEQ ID NO:18893 |
| | | AA | RAGQSIRTNFLA | GASSRAT | QQYGSSPWT |
| | | | SEQ ID NO:2870 | SEQ ID NO:10882 | SEQ ID NO:18894 |
| **iPS:436179** | **21-225_198E1** | NA | AGGGCCAGTCAGAGTATAA GGAGCAACTTCTTAGCC | GGTGCATTCAGTAGGGC CACT | CAGCAGTATGGTAATTCACC GTGGACG |
| | | | SEQ ID NO:2871 | SEQ ID NO:10883 | SEQ ID NO:18895 |
| | | AA | RASQSIRSNFLA | GAFSRAT | QQYGNSPWT |
| | | | SEQ ID NO:2872 | SEQ ID NO:10884 | SEQ ID NO:18896 |
| **iPS:436181** | **21-225_198C2** | NA | AGGGCCAGTCAGAGTGTTAG AAGCAGCTACTTAGCC | GGTGCATTCAGCAGGGC CAGT | CAGCAGTATGGTAACTCACC GTGGACG |
| | | | SEQ ID NO:2873 | SEQ ID NO:10885 | SEQ ID NO:18897 |
| | | AA | RASQSVRSSYLA | GAFSRAS | QQYGNSPWT |
| | | | SEQ ID NO:2874 | SEQ ID NO:10886 | SEQ ID NO:18898 |
| **iPS:436189** | **21-225_198B6** | NA | CGGGCGAGTCAGGGCATTGG CAATTATTTAGCC | GCTGCATCCAGTTTGCAA AGT | CAACAATATAGTACTTACCC GCTCACT |
| | | | SEQ ID NO:2875 | SEQ ID NO:10887 | SEQ ID NO:18899 |
| | | AA | RASQGIGNYLA | AASSLQS | QQYSTYPLT |
| | | | SEQ ID NO:2876 | SEQ ID NO:10888 | SEQ ID NO:18900 |
| **iPS:436191** | | NA | CGGGCAAGTCAGGGCATAA GAAAAGATTTAGGC | GCTGCATCCAGTTTGCAA AGT | CTACAACATTATCGTTACCC TTTCACT |

FIGURE 49

| | | | | | |
|---|---|---|---|---|---|
| | 21-225_198B9 | | SEQ ID NO:2877 | SEQ ID NO:10889 | SEQ ID NO:18901 |
| | | AA | RASQGIRKDLG | AASSLQS | LQHYRYPFT |
| | | | SEQ ID NO:2878 | SEQ ID NO:10890 | SEQ ID NO:18902 |
| iPS:436193 | 21-225_198A10 | NA | AAGTCTAGTCAGAGCCTCCT CTATAGTGATGGAAGGACCT ATTTGTAT | GAGGTTTCCAACCGGTTC TCT | ATGCAAAGTATACAGCTTCC CTGGACG |
| | | | SEQ ID NO:2879 | SEQ ID NO:10891 | SEQ ID NO:18903 |
| | | AA | KSSQSLLYSDGRTYLY | EVSNRFS | MQSIQLPWT |
| | | | SEQ ID NO:2880 | SEQ ID NO:10892 | SEQ ID NO:18904 |
| iPS:436195 | 21-225_198G10 | NA | AGGGCCAGTCAGAGTATTCG CAGCAGCTTCTTAGCC | GGTGCATCCAGCAGGGC CACT | CAGCAGTATGGTAACTCACC GTGGGCG |
| | | | SEQ ID NO:2881 | SEQ ID NO:10893 | SEQ ID NO:18905 |
| | | AA | RASQSIRSSFLA | GASSRAT | QQYGNSPWA |
| | | | SEQ ID NO:2882 | SEQ ID NO:10894 | SEQ ID NO:18906 |
| iPS:436197 | 21-225_199C2 | NA | AGGGCCAGTCAGAGTATTCG CAGCAGCTTCTTAGCC | GGTGCATCCAGCAGGGC CACT | CAGCAGTATGGTAACTCACC GTGGGCG |
| | | | SEQ ID NO:2883 | SEQ ID NO:10895 | SEQ ID NO:18907 |
| | | AA | RASQSIRSSFLA | GASSRAT | QQYGNSPWA |
| | | | SEQ ID NO:2884 | SEQ ID NO:10896 | SEQ ID NO:18908 |
| iPS:436199 | 21-225_199E3 | NA | CGGGCAAGTCAGGGCATTAG AAATGATTTAGGC | TCTGCATCCAGTTTGCAA AGG | CTACAGCATAAAAGGTACCC GCTCACT |
| | | | SEQ ID NO:2885 | SEQ ID NO:10897 | SEQ ID NO:18909 |
| | | AA | RASQGIRNDLG | SASSLQR | LQHKRYPLT |
| | | | SEQ ID NO:2886 | SEQ ID NO:10898 | SEQ ID NO:18910 |
| iPS:436201 | 21-225_199C5 | NA | CGGGCGAGTCAGGGCATTAG CAAGTATTTAGCC | GCTGCATCCAGTTTGCAA AGT | CAACAGTATCTTACTTACCC GCTCACT |
| | | | SEQ ID NO:2887 | SEQ ID NO:10899 | SEQ ID NO:18911 |
| | | AA | RASQGISKYLA | AASSLQS | QQYLTYPLT |
| | | | SEQ ID NO:2888 | SEQ ID NO:10900 | SEQ ID NO:18912 |

FIGURE 49

| | | | | | |
|---|---|---|---|---|---|
| **iPS:436203** | **21-225_199A6** | NA | AGGCCCAGTCAGAGTTTTAG CAGAAACTTAGCC | GGTGCATCCACTAGGGC CACT | CAGCAGTATAATAACTGGCC GCTCACT |
| | | | SEQ ID NO:2889 | SEQ ID NO:10901 | SEQ ID NO:18913 |
| | | AA | RPSQSFSRNLA | GASTRAT | QQYNNWPLT |
| | | | SEQ ID NO:2890 | SEQ ID NO:10902 | SEQ ID NO:18914 |
| **iPS:436205** | **21-225_199A7** | NA | CGGGCAAGTCAGGGCATAA GAAAAGATTTAGGC | GCTGCATCCAGTTTGCAA AGT | CTACAACATTATCGTTACCC TTTCACT |
| | | | SEQ ID NO:2891 | SEQ ID NO:10903 | SEQ ID NO:18915 |
| | | AA | RASQGIRKDLG | AASSLQS | LQHYRYPFT |
| | | | SEQ ID NO:2892 | SEQ ID NO:10904 | SEQ ID NO:18916 |
| **iPS:436207** | **21-225_199C7** | NA | AGGGCCAGTCAGAGTATAA GGACCAACTTCTTAGCC | GGTGCATCCAGCAGGGC CACT | CAGCAGTATGGTAACTCACC GTGGACG |
| | | | SEQ ID NO:2893 | SEQ ID NO:10905 | SEQ ID NO:18917 |
| | | AA | RASQSIRTNFLA | GASSRAT | QQYGNSPWT |
| | | | SEQ ID NO:2894 | SEQ ID NO:10906 | SEQ ID NO:18918 |
| **iPS:436210** | **21-225_199G11** | NA | AGGGCCAGTCAGAGTGTTAG AAGCAGCTACTTAGCC | GGTGCATTCAGCAGGGC CACT | CAGCAGTATGGTAACTCACC GTGGACG |
| | | | SEQ ID NO:2895 | SEQ ID NO:10907 | SEQ ID NO:18919 |
| | | AA | RASQSVRSSYLA | GAFSRAT | QQYGNSPWT |
| | | | SEQ ID NO:2896 | SEQ ID NO:10908 | SEQ ID NO:18920 |
| **iPS:436212** | **21-225_200G1** | NA | CGGGCAAGTCAGAGCATTAG CAGCTATTTAAAT | GCTGAGTCCAGTTTACA AAGT | CAACAGAGTTACAGTTCCCC TCCGTGGACG |
| | | | SEQ ID NO:2897 | SEQ ID NO:10909 | SEQ ID NO:18921 |
| | | AA | RASQSISSYLN | AESSLQS | QQSYSSPPWT |
| | | | SEQ ID NO:2898 | SEQ ID NO:10910 | SEQ ID NO:18922 |
| **iPS:436214** | **21-225_200F6** | NA | CGGGCAAGTCAGGACATTAG AAATGATTTAGGC | GCTGCATCCAGTTTGCAA AGT | CTACAGCATTATCGTTACCC ATTCACT |
| | | | SEQ ID NO:2899 | SEQ ID NO:10911 | SEQ ID NO:18923 |
| | | AA | RASQDIRNDLG | AASSLQS | LQHYRYPFT |
| | | | SEQ ID NO:2900 | SEQ ID NO:10912 | SEQ ID NO:18924 |

FIGURE 49

| | | | | | |
|---|---|---|---|---|---|
| **iPS:436216** | **21-225_200B7** | NA | CGGGCCAGTCAGAACATTGGTAATACCTTGCAC | TATGCTTCCCAGTCCTTCTCA | CATCAGAGTGGTAGTTTACCTCAGACG |
| | | | SEQ ID NO:2901 | SEQ ID NO:10913 | SEQ ID NO:18925 |
| | | AA | RASQNIGNTLH | YASQSFS | HQSGSLPQT |
| | | | SEQ ID NO:2902 | SEQ ID NO:10914 | SEQ ID NO:18926 |
| **iPS:436218** | **21-225_200G7** | NA | AAGTCCAGCCAGAGTGTTTTACACAGCTCCAACAATAACAACTACTTAGCT | TGGGCATCTACCCGGGAATCC | CAGCAATATTATAATACTCCTCCGACA |
| | | | SEQ ID NO:2903 | SEQ ID NO:10915 | SEQ ID NO:18927 |
| | | AA | KSSQSVLHSSNNNNYLA | WASTRES | QQYYNTPPT |
| | | | SEQ ID NO:2904 | SEQ ID NO:10916 | SEQ ID NO:18928 |
| **iPS:436220** | **21-225_200F8** | NA | CGGGCCAGTCAGAGTATTGGTAGTAACTTACAC | TCTGCTTCCCAGTCCTTCTCA | CAGCAGAGTAGTAGTTTACCGTGGACG |
| | | | SEQ ID NO:2905 | SEQ ID NO:10917 | SEQ ID NO:18929 |
| | | AA | RASQSIGSNLH | SASQSFS | QQSSSLPWT |
| | | | SEQ ID NO:2906 | SEQ ID NO:10918 | SEQ ID NO:18930 |
| **iPS:436222** | **21-225_200C9** | NA | CGGGCAACTCAGGGCATTAGAAAAGATTTAGGC | ACTGCATCCAGTTTGCAAAGT | CTACAGCATAATAGTTACCCGTGGACG |
| | | | SEQ ID NO:2907 | SEQ ID NO:10919 | SEQ ID NO:18931 |
| | | AA | RATQGIRKDLG | TASSLQS | LQHNSYPWT |
| | | | SEQ ID NO:2908 | SEQ ID NO:10920 | SEQ ID NO:18932 |
| **iPS:436226** | **21-225_200F10** | NA | AGGGCCAGTCAGAATATTCGCAGCAGCTTCTTAGCC | GGTGCATCCAGCAGGGCCACT | CAGCAGTATGGTAACTCACCGTGGGCG |
| | | | SEQ ID NO:2909 | SEQ ID NO:10921 | SEQ ID NO:18933 |
| | | AA | RASQNIRSSFLA | GASSRAT | QQYGNSPWA |
| | | | SEQ ID NO:2910 | SEQ ID NO:10922 | SEQ ID NO:18934 |
| **iPS:436228** | **21-225_200F12** | NA | CGGGCAAGTCAGGGCATTAGAAATGATTTAGGC | TCTGCATCCAGTTTGCATACT | CTACAGCATAAGAGTTACCCGCTCACT |
| | | | SEQ ID NO:2911 | SEQ ID NO:10923 | SEQ ID NO:18935 |
| | | AA | RASQGIRNDLG | SASSLHT | LQHKSYPLT |

FIGURE 49

| | | | | | |
|---|---|---|---|---|---|
| | | | SEQ ID NO:2912 | SEQ ID NO:10924 | SEQ ID NO:18936 |
| **iPS:436230** | **21-225_201A1** | NA | CGGGCAAGTCAGGGCATTAGGAATGATTTAGGC | TCTGCATCCATTTTACAAAGG | CTACAGCATAAAAGTTACCCTCTCACT |
| | | | SEQ ID NO:2913 | SEQ ID NO:10925 | SEQ ID NO:18937 |
| | | AA | RASQGIRNDLG | SASILQR | LQHKSYPLT |
| | | | SEQ ID NO:2914 | SEQ ID NO:10926 | SEQ ID NO:18938 |
| **iPS:436232** | **21-225_201E1** | NA | AGGGCCAGTCCGAGTATTAACAGCGGCTTCTTAGCC | GGTGCATCCAGCAGGGCCACT | CACCAGTATGAGACCTCACCGTGGACG |
| | | | SEQ ID NO:2915 | SEQ ID NO:10927 | SEQ ID NO:18939 |
| | | AA | RASPSINSGFLA | GASSRAT | HQYETSPWT |
| | | | SEQ ID NO:2916 | SEQ ID NO:10928 | SEQ ID NO:18940 |
| **iPS:436234** | **21-225_51E3** | NA | TCTGGAAGCAACTCCAACATCGGAAGTAATATTGTAACC | AGTAATGATCAGCGGCCCTCA | ACAGCATGGGATGACAGCCTGAATGGTTGGGTG |
| | | | SEQ ID NO:2917 | SEQ ID NO:10929 | SEQ ID NO:18941 |
| | | AA | SGSNSNIGSNIVT | SNDQRPS | TAWDDSLNGWV |
| | | | SEQ ID NO:2918 | SEQ ID NO:10930 | SEQ ID NO:18942 |
| **iPS:436236** | **21-225_201F7** | NA | AGGGCCAGTCAGAATATTAAAAACAACTTAGCC | GGTGCATCCACCAGGGCCACT | CAGCAGTTTTATAACTGGCTGTGCAGT |
| | | | SEQ ID NO:2919 | SEQ ID NO:10931 | SEQ ID NO:18943 |
| | | AA | RASQNIKNNLA | GASTRAT | QQFYNWLCS |
| | | | SEQ ID NO:2920 | SEQ ID NO:10932 | SEQ ID NO:18944 |
| **iPS:436238** | **21-225_201B2** | NA | AGGGCCAGTCAGAGTGTTAGCAGCAACTACTTAGCC | GGTGCATCCAGCAGGGCCACT | CAGCAGTATGAAAACTCACCGTGGACG |
| | | | SEQ ID NO:2921 | SEQ ID NO:10933 | SEQ ID NO:18945 |
| | | AA | RASQSVSSNYLA | GASSRAT | QQYENSPWT |
| | | | SEQ ID NO:2922 | SEQ ID NO:10934 | SEQ ID NO:18946 |
| **iPS:436240** | **21-225_201E8** | NA | CGGGCCAGTCAGAACATTGGTCGTAGTTTACAC | TATGCTTCCCAGTCCTTCTCA | CATCAGAGTCGAAGTTTACCGCTCACT |
| | | | SEQ ID NO:2923 | SEQ ID NO:10935 | SEQ ID NO:18947 |

FIGURE 49

| | | | | | |
|---|---|---|---|---|---|
| | | AA | RASQNIGRSLH | YASQSFS | HQSRSLPLT |
| | | | SEQ ID NO:2924 | SEQ ID NO:10936 | SEQ ID NO:18948 |
| **iPS:436242** | **21-225_201A10** | NA | CGGGCAAGTCAGGGCATTAG AAATGATTTAGGC | TCTACATCCAGTTTGCAT TCT | CTACAGCATAATAGTTACCC GCTCACT |
| | | | SEQ ID NO:2925 | SEQ ID NO:10937 | SEQ ID NO:18949 |
| | | AA | RASQGIRNDLG | STSSLHS | LQHNSYPLT |
| | | | SEQ ID NO:2926 | SEQ ID NO:10938 | SEQ ID NO:18950 |
| **iPS:436244** | **21-225_201H10** | NA | CGGGCAAGTCACAACATTAA CAGCTATTTAAAT | GCTGCATCCAGTTTGCAA AGT | CAACAGAGTTACAGTTTCCC GCTCACT |
| | | | SEQ ID NO:2927 | SEQ ID NO:10939 | SEQ ID NO:18951 |
| | | AA | RASHNINSYLN | AASSLQS | QQSYSFPLT |
| | | | SEQ ID NO:2928 | SEQ ID NO:10940 | SEQ ID NO:18952 |
| **iPS:436246** | **21-225_201G6** | NA | AGGTCTAGTCAGAGCCTCCT CCATAATAATAGATACAACC ATTTGGAT | TTGGGTTCTAATCGGGCC TCC | ATGCAAGCTCTACAAACTCC CACT |
| | | | SEQ ID NO:2929 | SEQ ID NO:10941 | SEQ ID NO:18953 |
| | | AA | RSSQSLLHNNRYNHLD | LGSNRAS | MQALQTPT |
| | | | SEQ ID NO:2930 | SEQ ID NO:10942 | SEQ ID NO:18954 |
| **iPS:436248** | **21-225_202A3** | NA | CGGGCAAGTCAGGGCATTAG AAATGATTTAGGC | GCTGCATCCAGGTTGCA AAGT | CTACAGCATCATGACTACCC ATTCACT |
| | | | SEQ ID NO:2931 | SEQ ID NO:10943 | SEQ ID NO:18955 |
| | | AA | RASQGIRNDLG | AASRLQS | LQHHDYPFT |
| | | | SEQ ID NO:2932 | SEQ ID NO:10944 | SEQ ID NO:18956 |
| **iPS:436250** | **21-225_201A4** | NA | AGGTCCAGTCAGAATATTAA AAGCAACTTAGCC | GGTGCATCCACCAGGGC CACT | CAGCAGTTTTATAACTGGCT GTGCAGT |
| | | | SEQ ID NO:2933 | SEQ ID NO:10945 | SEQ ID NO:18957 |
| | | AA | RSSQNIKSNLA | GASTRAT | QQFYNWLCS |
| | | | SEQ ID NO:2934 | SEQ ID NO:10946 | SEQ ID NO:18958 |
| **iPS:436252** | | NA | AGGGCCAGTCAGAGAATTA ACAACAACTTAGCC | GGTGCATCCACCAGGGC CACT | CAGCAGTATTATAACTGGCT GTGCAGT |

**FIGURE 49**

| | | | | | |
|---|---|---|---|---|---|
| | **21-225_202A8** | | SEQ ID NO:2935 | SEQ ID NO:10947 | SEQ ID NO:18959 |
| | | AA | RASQRINNNLA | GASTRAT | QQYYNWLCS |
| | | | SEQ ID NO:2936 | SEQ ID NO:10948 | SEQ ID NO:18960 |
| **iPS:436254** | **21-225_202C12** | NA | AGGTCTAGTCAGAGCCTCCT GCATAATAATAAATACAACC ATTTGGAT | TTGGGTTCTAATCGGGCC TCC | ATGCAAGCTCTACAAACTCC CACT |
| | | | SEQ ID NO:2937 | SEQ ID NO:10949 | SEQ ID NO:18961 |
| | | AA | RSSQSLLHNNKYNHLD | LGSNRAS | MQALQTPT |
| | | | SEQ ID NO:2938 | SEQ ID NO:10950 | SEQ ID NO:18962 |
| **iPS:436256** | **21-225_202D9** | NA | AGGGCCAGTCAGAGTGTTAA CAGCGGCTACTTAGCC | GGTGCATCCAGCAGGGC CACT | CAACAATATGAGACCTCACC GTGGACG |
| | | | SEQ ID NO:2939 | SEQ ID NO:10951 | SEQ ID NO:18963 |
| | | AA | RASQSVNSGYLA | GASSRAT | QQYETSPWT |
| | | | SEQ ID NO:2940 | SEQ ID NO:10952 | SEQ ID NO:18964 |
| **iPS:436258** | **21-225_202F12** | NA | AGGGCCAGTCAGAGTGTTCT GAACAACTTAGCC | GGTGCATCCACTAGGGC CACT | CAGCAGTATGATAACTGGCC TCCGTGCAGT |
| | | | SEQ ID NO:2941 | SEQ ID NO:10953 | SEQ ID NO:18965 |
| | | AA | RASQSVLNNLA | GASTRAT | QQYDNWPPCS |
| | | | SEQ ID NO:2942 | SEQ ID NO:10954 | SEQ ID NO:18966 |
| **iPS:436260** | **21-225_203H1** | NA | CGGGCGAGTCAGGGCATTGG CAATTATTTAGCC | GTTGCATCCAGGTTGCA AAGT | CAACGGTATCATACTTACCC GCTCACT |
| | | | SEQ ID NO:2943 | SEQ ID NO:10955 | SEQ ID NO:18967 |
| | | AA | RASQGIGNYLA | VASRLQS | QRYHTYPLT |
| | | | SEQ ID NO:2944 | SEQ ID NO:10956 | SEQ ID NO:18968 |
| **iPS:436262** | **21-225_203E3** | NA | CGGGCAAGTCACAACATTAA CAGCTATTTAAAT | GCTGCATCCAGTTTGCAA AGT | CAACAGAGTTACAGTTTCCC GCTCACT |
| | | | SEQ ID NO:2945 | SEQ ID NO:10957 | SEQ ID NO:18969 |
| | | AA | RASHNINSYLN | AASSLQS | QQSYSFPLT |
| | | | SEQ ID NO:2946 | SEQ ID NO:10958 | SEQ ID NO:18970 |

FIGURE 49

| | | | | | |
|---|---|---|---|---|---|
| **iPS:436264** | **21-225_203F7** | NA | CGGGCAAGTCAGGGCATTAGACATGATTTAGGC | GCTGCATCCAGTTTGCAAAGT | CTACAGCATTATAGTTTCCCTCGGACG |
| | | | SEQ ID NO:2947 | SEQ ID NO:10959 | SEQ ID NO:18971 |
| | | AA | RASQGIRHDLG | AASSLQS | LQHYSFPRT |
| | | | SEQ ID NO:2948 | SEQ ID NO:10960 | SEQ ID NO:18972 |
| **iPS:436268** | **21-225_203B9** | NA | CGGGCAAGTCAGGGCATTAGAAATGATTTAGGC | AGGGCATCCAGTGTGCAAAAT | CTACAGCATAATAGTTACCCATTCACT |
| | | | SEQ ID NO:2949 | SEQ ID NO:10961 | SEQ ID NO:18973 |
| | | AA | RASQGIRNDLG | RASSVQN | LQHNSYPFT |
| | | | SEQ ID NO:2950 | SEQ ID NO:10962 | SEQ ID NO:18974 |
| **iPS:436270** | **21-225_203F10** | NA | AAGTCCAGCCAGAGTGTTTTTTTCCACTCGAACAATAAGAACTACTTAGCT | TGGGCATCTACCCGGGAATCC | CAACAATATTTAGTCTTCCATTCACT |
| | | | SEQ ID NO:2951 | SEQ ID NO:10963 | SEQ ID NO:18975 |
| | | AA | KSSQSVFFHSNNKNYLA | WASTRES | QQYFSLPFT |
| | | | SEQ ID NO:2952 | SEQ ID NO:10964 | SEQ ID NO:18976 |
| **iPS:436272** | **21-225_201F5** | NA | AAGTCCAGCCAGAGTGTTTTATACAGTTCCAACAATAAGAACTACTTAGTT | TGGGCATCTACCCGGGAATCC | CAGCAATATTATAGTACTCCTCCGACG |
| | | | SEQ ID NO:2953 | SEQ ID NO:10965 | SEQ ID NO:18977 |
| | | AA | KSSQSVLYSSNNKNYLV | WASTRES | QQYYSTPPT |
| | | | SEQ ID NO:2954 | SEQ ID NO:10966 | SEQ ID NO:18978 |
| **iPS:436274** | **21-225_204H3** | NA | CGGGCAAGTCAGGGCATTAGAAATGATTTAGGC | GCTGCAGCCAGTTTGCAAGGT | CTACAGCATTATAGTTACCCTCGGACG |
| | | | SEQ ID NO:2955 | SEQ ID NO:10967 | SEQ ID NO:18979 |
| | | AA | RASQGIRNDLG | AAASLQG | LQHYSYPRT |
| | | | SEQ ID NO:2956 | SEQ ID NO:10968 | SEQ ID NO:18980 |
| **iPS:436276** | **21-225_204H4** | NA | CGGGCAAGTCACAACATTAACAGCTATTTAAAT | GCTGCATCCAGTTTGCAAAGT | CAACAGAGTTACAGTTTCCCGCTCACT |
| | | | SEQ ID NO:2957 | SEQ ID NO:10969 | SEQ ID NO:18981 |

FIGURE 49

| | | | | | |
|---|---|---|---|---|---|
| | | AA | RASHNINSYLN | AASSLQS | QQSYSFPLT |
| | | | SEQ ID NO:2958 | SEQ ID NO:10970 | SEQ ID NO:18982 |
| **iPS:436278** | **21-225_201F2** | NA | AGGGCCAGTCAGAATATTAA AAGCAACTTAGCC | GGTGCATCCACCAGGGC CACT | CAGCAGTTTATAACTGGCT GTGCAGT |
| | | | SEQ ID NO:2959 | SEQ ID NO:10971 | SEQ ID NO:18983 |
| | | AA | RASQNIKSNLA | GASTRAT | QQFYNWLCS |
| | | | SEQ ID NO:2960 | SEQ ID NO:10972 | SEQ ID NO:18984 |
| **iPS:436280** | **21-225_204D6** | NA | CGGGCAAGTCGGAGCGTTCA CACCTATTTAAAT | GGTGCATCCAGTTTGCA ACGT | CAACAGAGTTACAGTTCCCC GCTCACT |
| | | | SEQ ID NO:2961 | SEQ ID NO:10973 | SEQ ID NO:18985 |
| | | AA | RASRSVHTYLN | GASSLQR | QQSYSSPLT |
| | | | SEQ ID NO:2962 | SEQ ID NO:10974 | SEQ ID NO:18986 |
| **iPS:436282** | **21-225_204G6** | NA | CGGACGAGTCAGGGCATTGG CAATTATTTAGCC | GCTGCATCCAGTTTGCAA AGT | CAACACTATCTTAGTTACCC TCTCACT |
| | | | SEQ ID NO:2963 | SEQ ID NO:10975 | SEQ ID NO:18987 |
| | | AA | RTSQGIGNYLA | AASSLQS | QHYLSYPLT |
| | | | SEQ ID NO:2964 | SEQ ID NO:10976 | SEQ ID NO:18988 |
| **iPS:436284** | **21-225_204G8** | NA | CGGGCGAGTCAGGGCATAA GTAATCATTTAGCC | GCTGCATCCAGTTTGCAA AGT | CAACAGTATAGTAATTACCC GGTCACT |
| | | | SEQ ID NO:2965 | SEQ ID NO:10977 | SEQ ID NO:18989 |
| | | AA | RASQGISNHLA | AASSLQS | QQYSNYPVT |
| | | | SEQ ID NO:2966 | SEQ ID NO:10978 | SEQ ID NO:18990 |
| **iPS:436286** | **21-225_204H8** | NA | CGGGCAAGTCAGGGCATTAG AAATGATTTAGGC | TCTGCATCCAGTTTGCAA AGT | CTACAACATAATAGTTACCC TCTCACT |
| | | | SEQ ID NO:2967 | SEQ ID NO:10979 | SEQ ID NO:18991 |
| | | AA | RASQGIRNDLG | SASSLQS | LQHNSYPLT |
| | | | SEQ ID NO:2968 | SEQ ID NO:10980 | SEQ ID NO:18992 |
| **iPS:436290** | **21-225_205G3** | NA | AGGGCCAGTCAGAATGTTAG TTACAGCTACTTAGCC | GGTGCATCCAGGAGGGC CACT | CAGCAGTATGGTAGCTCACC GTGCAGT |
| | | | SEQ ID NO:2969 | SEQ ID NO:10981 | SEQ ID NO:18993 |

FIGURE 49

| | | | | | |
|---|---|---|---|---|---|
| | | AA | RASQNVSYSYLA | GASRRAT | QQYGSSPCS |
| | | | SEQ ID NO:2970 | SEQ ID NO:10982 | SEQ ID NO:18994 |
| iPS:436292 | 21-225_205H3 | NA | CGGGCGAGTCAGGCCATTAGTAATCATTTAGCC | GCTGCATCCAGTTTGCAAAGT | CAACAATATAGTAATTACCCACTCACT |
| | | | SEQ ID NO:2971 | SEQ ID NO:10983 | SEQ ID NO:18995 |
| | | AA | RASQAISNHLA | AASSLQS | QQYSNYPLT |
| | | | SEQ ID NO:2972 | SEQ ID NO:10984 | SEQ ID NO:18996 |
| iPS:436294 | 21-225_205G4 | NA | AGGGCCAGTCAGAATATTAAAAGCAACTTAGCC | GGTGCATCCACCAGGGCCACT | CAGCAGTTTTATAACTGGCTGTGCAGT |
| | | | SEQ ID NO:2973 | SEQ ID NO:10985 | SEQ ID NO:18997 |
| | | AA | RASQNIKSNLA | GASTRAT | QQFYNWLCS |
| | | | SEQ ID NO:2974 | SEQ ID NO:10986 | SEQ ID NO:18998 |
| iPS:436296 | 21-225_205F5 | NA | CGGGCGAGTCAGGGCATTGGCAATTATTTAGCC | GGTGTCTCCAGTTTGCAAAGT | CAACAATATAGTAATTACCCTCTCACT |
| | | | SEQ ID NO:2975 | SEQ ID NO:10987 | SEQ ID NO:18999 |
| | | AA | RASQGIGNYLA | GVSSLQS | QQYSNYPLT |
| | | | SEQ ID NO:2976 | SEQ ID NO:10988 | SEQ ID NO:19000 |
| iPS:436302 | 21-225_205G7 | NA | AGGGCCAGTCAGAGTGTTTTCAGCAACTACTTAGCC | GGTGCATCCAGCAGGGCCGCT | CAGCAGTATGAAAGTTCACCGTGGACG |
| | | | SEQ ID NO:2977 | SEQ ID NO:10989 | SEQ ID NO:19001 |
| | | AA | RASQSVFSNYLA | GASSRAA | QQYESSPWT |
| | | | SEQ ID NO:2978 | SEQ ID NO:10990 | SEQ ID NO:19002 |
| iPS:436304 | 21-225_201F3 | NA | AGGTCTAGTCAGAGCCTCCTGCATAATAATAGATACAACCATTTGGAT | TTGGGTTCTAATCGGGCCTCC | ATGCAAGCTCTACAAACTCCCACT |
| | | | SEQ ID NO:2979 | SEQ ID NO:10991 | SEQ ID NO:19003 |
| | | AA | RSSQSLLHNNRYNHLD | LGSNRAS | MQALQTPT |
| | | | SEQ ID NO:2980 | SEQ ID NO:10992 | SEQ ID NO:19004 |
| iPS:436306 | | NA | AGGGCCAGTCAGAGTGTTAATAGCTACTTAGCC | GGTGCATCCACCAGGGCCACT | CAAGAGTATAATGACTGGCCGTGCAGT |

FIGURE 49

| | | | | | |
|---|---|---|---|---|---|
| | **21-225_201H4** | | SEQ ID NO:2981 | SEQ ID NO:10993 | SEQ ID NO:19005 |
| | | AA | RASQSVNSYLA | GASTRAT | QEYNDWPCS |
| | | | SEQ ID NO:2982 | SEQ ID NO:10994 | SEQ ID NO:19006 |
| **iPS:436308** | **21-225_205H8** | NA | CGGGCAAGTCAGGGCATTAG AAATGATTTAGGC | TCTGCATCCTTTTTGCAA AGA | CTACAGCATAATAGTTACCC GCTCACT |
| | | | SEQ ID NO:2983 | SEQ ID NO:10995 | SEQ ID NO:19007 |
| | | AA | RASQGIRNDLG | SASFLQR | LQHNSYPLT |
| | | | SEQ ID NO:2984 | SEQ ID NO:10996 | SEQ ID NO:19008 |
| **iPS:436310** | **21-225_202D11** | NA | AGGGCCAGTCAGAGTATTAA CAGCAACTACTTAGCC | GGTGCATCCAGCAGGGC CACT | CAGCAGTATGAAAACTCACC GTGGACG |
| | | | SEQ ID NO:2985 | SEQ ID NO:10997 | SEQ ID NO:19009 |
| | | AA | RASQSINSNYLA | GASSRAT | QQYENSPWT |
| | | | SEQ ID NO:2986 | SEQ ID NO:10998 | SEQ ID NO:19010 |
| **iPS:436312** | **21-225_206A4** | NA | CGGGCAAGTCACAACATTAA CAGCTATTTAAAT | GCTGCATCCAGTTTGCAA AGT | CAACAGAGTTACAGTTTCCC GCTCACT |
| | | | SEQ ID NO:2987 | SEQ ID NO:10999 | SEQ ID NO:19011 |
| | | AA | RASHNINSYLN | AASSLQS | QQSYSFPLT |
| | | | SEQ ID NO:2988 | SEQ ID NO:11000 | SEQ ID NO:19012 |
| **iPS:436314** | **21-225_206G4** | NA | CGGGCCAGTCAGAGCATTGG TCGTAGCTTACAC | TATGCTTCCCAGTCCTTC TCA | CATCAGAGTAGAAGTTTACC GCTCACT |
| | | | SEQ ID NO:2989 | SEQ ID NO:11001 | SEQ ID NO:19013 |
| | | AA | RASQSIGRSLH | YASQSFS | HQSRSLPLT |
| | | | SEQ ID NO:2990 | SEQ ID NO:11002 | SEQ ID NO:19014 |
| **iPS:436316** | **21-225_206A5** | NA | CGGGCAAGTCACAACATTAA CAGCTATTTAAAT | GCTGCATCCAGTTTGCAA AGT | CAACAGAGTTACAGTTTCCC GCTCACT |
| | | | SEQ ID NO:2991 | SEQ ID NO:11003 | SEQ ID NO:19015 |
| | | AA | RASHNINSYLN | AASSLQS | QQSYSFPLT |
| | | | SEQ ID NO:2992 | SEQ ID NO:11004 | SEQ ID NO:19016 |
| **iPS:436324** | | NA | CGGGCGAGTCAGGGCATTAG AAATTATTTAGCC | GCTGCATCCAGTTTGCAA AGT | CAACAGTACAGTAATTACCC TCTCACT |

FIGURE 49

| | | | | | |
|---|---|---|---|---|---|
| | 21-225_207G6 | | SEQ ID NO:2993 | SEQ ID NO:11005 | SEQ ID NO:19017 |
| | | AA | RASQGIRNYLA | AASSLQS | QQYSNYPLT |
| | | | SEQ ID NO:2994 | SEQ ID NO:11006 | SEQ ID NO:19018 |
| iPS:436328 | 21-225_207F12 | NA | CGGGCAAGTCAGGGCATTAG AAATGATTTAGGC | GCTGCATCCAGTTTGCAA AGT | CTACAGCATAATAGTTACCC TCTCACC |
| | | | SEQ ID NO:2995 | SEQ ID NO:11007 | SEQ ID NO:19019 |
| | | AA | RASQGIRNDLG | AASSLQS | LQHNSYPLT |
| | | | SEQ ID NO:2996 | SEQ ID NO:11008 | SEQ ID NO:19020 |
| iPS:436332 | 21-225_208B2 | NA | CGGGCAAGTCAGGGCATTAG ACATGATTTAGGC | GCTGCATCCAGTTTGCAA AGT | CTACAGCATTATAGTTACCC TCGGACG |
| | | | SEQ ID NO:2997 | SEQ ID NO:11009 | SEQ ID NO:19021 |
| | | AA | RASQGIRHDLG | AASSLQS | LQHYSYPRT |
| | | | SEQ ID NO:2998 | SEQ ID NO:11010 | SEQ ID NO:19022 |
| iPS:436334 | 21-225_208G3 | NA | AGGTCTAGTCAGAGCCTCCT GCATAATAATAAATACAACC ATTTGGAT | TTGGGTTCTAATCGGGCC TCC | ATGCAAGCTCTACAAACTCC CACT |
| | | | SEQ ID NO:2999 | SEQ ID NO:11011 | SEQ ID NO:19023 |
| | | AA | RSSQSLLHNNKYNHLD | LGSNRAS | MQALQTPT |
| | | | SEQ ID NO:3000 | SEQ ID NO:11012 | SEQ ID NO:19024 |
| iPS:436336 | 21-225_208B5 | NA | AGGGCCAGTCAGAGTGTTAG CAGCAACTACTTAGCC | GGTGCATCCAGCAGGGC CACT | CAGCACTACGAAAACTCACC GTGGACG |
| | | | SEQ ID NO:3001 | SEQ ID NO:11013 | SEQ ID NO:19025 |
| | | AA | RASQSVSSNYLA | GASSRAT | QHYENSPWT |
| | | | SEQ ID NO:3002 | SEQ ID NO:11014 | SEQ ID NO:19026 |
| iPS:436338 | 21-225_208E8 | NA | CGGGCAAGTCACAACATTAA CAGCTATTTAAAT | GCTGCATCCAGTTTGCAA AGT | CAACAGAGTTACAGTTTCCC GCTCACT |
| | | | SEQ ID NO:3003 | SEQ ID NO:11015 | SEQ ID NO:19027 |
| | | AA | RASHNINSYLN | AASSLQS | QQSYSFPLT |
| | | | SEQ ID NO:3004 | SEQ ID NO:11016 | SEQ ID NO:19028 |

# FIGURE 49

| | | | | | |
|---|---|---|---|---|---|
| **iPS:436340** | **21-225_208A9** | NA | AGGGCCAGTCAGAGTGTTAGCAACAACTACTTAGCC | GGTGCATCCAGCAGGGCCACT | CAGCACTATCATAGCTCACCGTGGACG |
| | | | SEQ ID NO:3005 | SEQ ID NO:11017 | SEQ ID NO:19029 |
| | | AA | RASQSVSNNYLA | GASSRAT | QHYHSSPWT |
| | | | SEQ ID NO:3006 | SEQ ID NO:11018 | SEQ ID NO:19030 |
| **iPS:436344** | **21-225_208B11** | NA | CGGGCAAGTCACAACATTAACAGCTATTTAAAT | GCTGCATCCAGTTTGCAAAGT | CAACAGAGTTACAGTTTCCCGCTCACT |
| | | | SEQ ID NO:3007 | SEQ ID NO:11019 | SEQ ID NO:19031 |
| | | AA | RASHNINSYLN | AASSLQS | QQSYSFPLT |
| | | | SEQ ID NO:3008 | SEQ ID NO:11020 | SEQ ID NO:19032 |
| **iPS:436350** | **21-225_210E4** | NA | CGGGCAAGTCAGGGCATTAGAAATGATTTAGGC | GCTGCATCCAGTTTGCAAAGT | CTACAGCATAATAGTTACCCATTCACT |
| | | | SEQ ID NO:3009 | SEQ ID NO:11021 | SEQ ID NO:19033 |
| | | AA | RASQGIRNDLG | AASSLQS | LQHNSYPFT |
| | | | SEQ ID NO:3010 | SEQ ID NO:11022 | SEQ ID NO:19034 |
| **iPS:436352** | **21-225_210G5** | NA | CGGGCAAGTCAGGGCATTAGACATGATTTAGGC | GCTGCATCCAGTTTGCAAAGT | CTACACCATTATAGTTACCCTCGGACG |
| | | | SEQ ID NO:3011 | SEQ ID NO:11023 | SEQ ID NO:19035 |
| | | AA | RASQGIRHDLG | AASSLQS | LHHYSYPRT |
| | | | SEQ ID NO:3012 | SEQ ID NO:11024 | SEQ ID NO:19036 |
| **iPS:436354** | **21-225_210G10** | NA | CGGACAAGTCAGGGCATTAGAAATGATTTAGGC | GCTGCATCCAGTTTGTTTAGT | CTACAGTATAATAGTTACCCTCCCACC |
| | | | SEQ ID NO:3013 | SEQ ID NO:11025 | SEQ ID NO:19037 |
| | | AA | RTSQGIRNDLG | AASSLFS | LQYNSYPPT |
| | | | SEQ ID NO:3014 | SEQ ID NO:11026 | SEQ ID NO:19038 |
| **iPS:436356** | **21-225_210H10** | NA | AGGGCCAGTCAGAGTGTTAAAAGCAACTTAGCC | GGTGCATCCACCAGGGCCACT | CAGCAGTATTATAACTGGCTGTGCAGT |
| | | | SEQ ID NO:3015 | SEQ ID NO:11027 | SEQ ID NO:19039 |
| | | AA | RASQSVKSNLA | GASTRAT | QQYYNWLCS |
| | | | SEQ ID NO:3016 | SEQ ID NO:11028 | SEQ ID NO:19040 |

# FIGURE 49

| | | | | | |
|---|---|---|---|---|---|
| **iPS:436358** | **21-225_210D11** | NA | CGGGCAAGTCACAACATTAACAGCTATTTAAAT | GCTGCATCCAGTTTGCAAAGT | CAACAGAGTTACAGTTTCCCGCTCACT |
| | | | SEQ ID NO:3017 | SEQ ID NO:11029 | SEQ ID NO:19041 |
| | | AA | RASHNINSYLN | AASSLQS | QQSYSFPLT |
| | | | SEQ ID NO:3018 | SEQ ID NO:11030 | SEQ ID NO:19042 |
| **iPS:436360** | **21-225_210H11** | NA | CGGGCGAGTCAGGGTATTAGCATCTGGTTAGCC | GCTGCATCCAGTTTGCAAAGT | CAACAGGCTAAAAGTTTCCCATTCACT |
| | | | SEQ ID NO:3019 | SEQ ID NO:11031 | SEQ ID NO:19043 |
| | | AA | RASQGISIWLA | AASSLQS | QQAKSFPFT |
| | | | SEQ ID NO:3020 | SEQ ID NO:11032 | SEQ ID NO:19044 |
| **iPS:436362** | **21-225_210C12** | NA | AGGTCTAGTCAGAGCCTCCTGCATTATAATGGACACAACTTTTTGGAT | TTGGTTTCTAATCGGGCCTCC | ATGCAAGCTCTACAAACTCCCATGTGCAGT |
| | | | SEQ ID NO:3021 | SEQ ID NO:11033 | SEQ ID NO:19045 |
| | | AA | RSSQSLLHYNGHNFLD | LVSNRAS | MQALQTPMCS |
| | | | SEQ ID NO:3022 | SEQ ID NO:11034 | SEQ ID NO:19046 |
| **iPS:436364** | **21-225_211A11** | NA | CGGGCGAGTCAGGGCATTGGCAATTATTTAGCC | GATGCATCCAGTTTGGAAAGT | CAACACTATATGACTTACCCGCTCACT |
| | | | SEQ ID NO:3023 | SEQ ID NO:11035 | SEQ ID NO:19047 |
| | | AA | RASQGIGNYLA | DASSLES | QHYMTYPLT |
| | | | SEQ ID NO:3024 | SEQ ID NO:11036 | SEQ ID NO:19048 |
| **iPS:436366** | **21-225_211A3** | NA | CGGGCGAGTCAGGCCATTGGGAAACATTTAGCC | GCTGCATCCAGATTGCAAAGT | CAACAGTATAGTAATTATCCGCTCACT |
| | | | SEQ ID NO:3025 | SEQ ID NO:11037 | SEQ ID NO:19049 |
| | | AA | RASQAIGKHLA | AASRLQS | QQYSNYPLT |
| | | | SEQ ID NO:3026 | SEQ ID NO:11038 | SEQ ID NO:19050 |
| **iPS:436368** | **21-225_211G3** | NA | AGGGCCAGTCAGAGTGTTAGCAGCAGCTTCTTAGCC | GGTGCATCCAGCAGGGCCACT | CAGCAGTATGTTAGCTCACCGCTCACT |
| | | | SEQ ID NO:3027 | SEQ ID NO:11039 | SEQ ID NO:19051 |
| | | AA | RASQSVSSSFLA | GASSRAT | QQYVSSPLT |

**FIGURE 49**

| | | | | | |
|---|---|---|---|---|---|
| | | | SEQ ID NO:3028 | SEQ ID NO:11040 | SEQ ID NO:19052 |
| **iPS:436370** | **21-225_211A6** | NA | CGGGCGAGTCAGGGCATTGGCAAGTATTTAGCC | GCTGCATCCAGTTTGCTAAGT | CAAAAGTATGATACTTACCCATTCACT |
| | | | SEQ ID NO:3029 | SEQ ID NO:11041 | SEQ ID NO:19053 |
| | | AA | RASQGIGKYLA | AASSLLS | QKYDTYPFT |
| | | | SEQ ID NO:3030 | SEQ ID NO:11042 | SEQ ID NO:19054 |
| **iPS:436372** | **21-225_211A8** | NA | CGGGCGAGTCAGGGCATTAGCAGATATTTAGCC | GCTGCATCCAGTTTGCAAAGT | CTACGGTATGATACTTACCCTCTCATT |
| | | | SEQ ID NO:3031 | SEQ ID NO:11043 | SEQ ID NO:19055 |
| | | AA | RASQGISRYLA | AASSLQS | LRYDTYPLI |
| | | | SEQ ID NO:3032 | SEQ ID NO:11044 | SEQ ID NO:19056 |
| **iPS:436374** | **21-225_211C10** | NA | AGGTCTAGTCAGAGCCTCCTCCATAGTAATGGATACAACTATTTGGAT | TTGGGTTCTAATCGGGCCTCC | ATGCAAGCTCTACTAACTCCCGTGTGCAGT |
| | | | SEQ ID NO:3033 | SEQ ID NO:11045 | SEQ ID NO:19057 |
| | | AA | RSSQSLLHSNGYNYLD | LGSNRAS | MQALLTPVCS |
| | | | SEQ ID NO:3034 | SEQ ID NO:11046 | SEQ ID NO:19058 |
| **iPS:436376** | **21-225_212E6** | NA | CGGGCGAGTCAGGGCATTAGCAATCATTTAGCC | GCTGCATCCAGTTTGCAAAGT | CAACAGTATGTTACCTACCCTCTCACT |
| | | | SEQ ID NO:3035 | SEQ ID NO:11047 | SEQ ID NO:19059 |
| | | AA | RASQGISNHLA | AASSLQS | QQYVTYPLT |
| | | | SEQ ID NO:3036 | SEQ ID NO:11048 | SEQ ID NO:19060 |
| **iPS:436378** | **21-225_212D7** | NA | CGGGCGAGTCAGGGCATTAGCAATCATTTAGCC | GCTGCATCCAGTTTGCAAAGT | CAGCAGTATAGTAATTACCCTCTCACT |
| | | | SEQ ID NO:3037 | SEQ ID NO:11049 | SEQ ID NO:19061 |
| | | AA | RASQGISNHLA | AASSLQS | QQYSNYPLT |
| | | | SEQ ID NO:3038 | SEQ ID NO:11050 | SEQ ID NO:19062 |
| **iPS:436380** | **21-225_212H9** | NA | CGGGCGAGTCAGGGCATTAGCAGTTATTTAGCC | GCTGCATCCAGTTTGCAAAGT | CTACGGTATGATACTTACCCTCTCACT |
| | | | SEQ ID NO:3039 | SEQ ID NO:11051 | SEQ ID NO:19063 |

FIGURE 49

| | | | | | |
|---|---|---|---|---|---|
| | | AA | RASQGISSYLA | AASSLQS | LRYDTYPLT |
| | | | SEQ ID NO:3040 | SEQ ID NO:11052 | SEQ ID NO:19064 |
| **iPS:436382** | **21-225_212C10** | NA | AGGGCCAGTCAGAGTGTTGC CAGCAGCTTAGCC | GGTACATCCACCAGGGC CACT | CAGCAGTATAATGACTGGCC GTGCAGT |
| | | | SEQ ID NO:3041 | SEQ ID NO:11053 | SEQ ID NO:19065 |
| | | AA | RASQSVASSLA | GTSTRAT | QQYNDWPCS |
| | | | SEQ ID NO:3042 | SEQ ID NO:11054 | SEQ ID NO:19066 |
| **iPS:436384** | **21-225_212F10** | NA | CGGGCGAGTCAGGGCATTAG CAATTATTTAGAC | TCTGCATCCAATTTGCAA AGT | CAACACTATAGTAATTACCC GCTCACT |
| | | | SEQ ID NO:3043 | SEQ ID NO:11055 | SEQ ID NO:19067 |
| | | AA | RASQGISNYLD | SASNLQS | QHYSNYPLT |
| | | | SEQ ID NO:3044 | SEQ ID NO:11056 | SEQ ID NO:19068 |
| **iPS:436386** | **21-225_212B11** | NA | CGGGCAAGTCAGGGCATTAG AAATGATTTAGGC | GCTGCATCCAGTTTGCAA AGT | CTACTGCATTATAGTTACCCT CGGACG |
| | | | SEQ ID NO:3045 | SEQ ID NO:11057 | SEQ ID NO:19069 |
| | | AA | RASQGIRNDLG | AASSLQS | LLHYSYPRT |
| | | | SEQ ID NO:3046 | SEQ ID NO:11058 | SEQ ID NO:19070 |
| **iPS:436388** | **21-225_212H11** | NA | CGGGCGAGTCAGGCCATTGG GAAACATTTAGCC | GCTGCATCCAGATTGCA AAGT | CAACACTATAGTAATTATCC GCTCACT |
| | | | SEQ ID NO:3047 | SEQ ID NO:11059 | SEQ ID NO:19071 |
| | | AA | RASQAIGKHLA | AASRLQS | QHYSNYPLT |
| | | | SEQ ID NO:3048 | SEQ ID NO:11060 | SEQ ID NO:19072 |
| **iPS:436390** | **21-225_213D2** | NA | CGGGCGAGTCAGGGCATTAG CAATCATTTAGCC | GCTGCATCCAGTTTGCAA AGT | CACCAGTATAGTAATTACCC TCTCACT |
| | | | SEQ ID NO:3049 | SEQ ID NO:11061 | SEQ ID NO:19073 |
| | | AA | RASQGISNHLA | AASSLQS | HQYSNYPLT |
| | | | SEQ ID NO:3050 | SEQ ID NO:11062 | SEQ ID NO:19074 |
| **iPS:436392** | **21-225_213B3** | NA | CGGGCGAGTCAGGGCATTGG CAAGTATTTAGCC | GCTGCATCCAGTGTGCTA AGT | CAAAAGTATGATACTTACCC ATTCACT |
| | | | SEQ ID NO:3051 | SEQ ID NO:11063 | SEQ ID NO:19075 |

FIGURE 49

| | | | | | |
|---|---|---|---|---|---|
| | | AA | RASQGIGKYLA | AASSVLS | QKYDTYPFT |
| | | | SEQ ID NO:3052 | SEQ ID NO:11064 | SEQ ID NO:19076 |
| **iPS:436394** | **21-225_213C4** | NA | CGGGCGAGTCAGGCCATTAGGAATTATTTAGCC | GCTGCATCCAGTTTGCAAAGT | CAACAGTATAGTAATTACCCTCTCACT |
| | | | SEQ ID NO:3053 | SEQ ID NO:11065 | SEQ ID NO:19077 |
| | | AA | RASQAIRNYLA | AASSLQS | QQYSNYPLT |
| | | | SEQ ID NO:3054 | SEQ ID NO:11066 | SEQ ID NO:19078 |
| **iPS:436396** | **21-225_213E5** | NA | CGGGCGAGTCAGGCCATTGGGAAACATTTAGCC | GCTGCATCCAGATTGCAAAGT | CAACACTATAGTAATTATCCGCTCACT |
| | | | SEQ ID NO:3055 | SEQ ID NO:11067 | SEQ ID NO:19079 |
| | | AA | RASQAIGKHLA | AASRLQS | QHYSNYPLT |
| | | | SEQ ID NO:3056 | SEQ ID NO:11068 | SEQ ID NO:19080 |
| **iPS:436398** | **21-225_213B8** | NA | CGGGCGAGTCAGGGCATTAGCAATCATTTAGCC | GCTGCATCCAGTTTGCAAAGT | CAACAGTATAGTAATTACCCTCTCACT |
| | | | SEQ ID NO:3057 | SEQ ID NO:11069 | SEQ ID NO:19081 |
| | | AA | RASQGISNHLA | AASSLQS | QQYSNYPLT |
| | | | SEQ ID NO:3058 | SEQ ID NO:11070 | SEQ ID NO:19082 |
| **iPS:436400** | **21-225_213H7** | NA | AAGTCCAGCCAGAATGTTTTAGACATCTCCAACAATAAGAATTCCTTAGGT | TGGGCATCTACCCGGGAATCC | CAGCAATATTATAACATTCCTCCGACG |
| | | | SEQ ID NO:3059 | SEQ ID NO:11071 | SEQ ID NO:19083 |
| | | AA | KSSQNVLDISNNKNSLG | WASTRES | QQYYNIPPT |
| | | | SEQ ID NO:3060 | SEQ ID NO:11072 | SEQ ID NO:19084 |
| **iPS:436402** | **21-225_213H12** | NA | AAGTCCAGCCAGAATGTTTTAAAGACCTCCAACAATAGGAACTACTTAGCT | TGGGCATCTACCCGGGAATCC | CACCAATATTATAGTATTCCGTGGACC |
| | | | SEQ ID NO:3061 | SEQ ID NO:11073 | SEQ ID NO:19085 |
| | | AA | KSSQNVLKTSNNRNYLA | WASTRES | HQYYSIPWT |
| | | | SEQ ID NO:3062 | SEQ ID NO:11074 | SEQ ID NO:19086 |

FIGURE 49

| | | | | | |
|---|---|---|---|---|---|
| **iPS:436404** | **21-225_214C3** | NA | CGGGCGAGTCAGGGCATTGG CAATTATTTAGCC | GCTGCATCCAGTTTGCAA AGT | CAACAATATATGACTTACCC AATCACT |
| | | | SEQ ID NO:3063 | SEQ ID NO:11075 | SEQ ID NO:19087 |
| | | AA | RASQGIGNYLA | AASSLQS | QQYMTYPIT |
| | | | SEQ ID NO:3064 | SEQ ID NO:11076 | SEQ ID NO:19088 |
| **iPS:436406** | **21-225_214E4** | NA | CGGGCGAGTCAGGGCATTAG CAATTATTTAGCC | GCTGCATCCAGTTTGCAA AGT | CAACAGTATCTTACTTACCC ATTCACT |
| | | | SEQ ID NO:3065 | SEQ ID NO:11077 | SEQ ID NO:19089 |
| | | AA | RASQGISNYLA | AASSLQS | QQYLTYPFT |
| | | | SEQ ID NO:3066 | SEQ ID NO:11078 | SEQ ID NO:19090 |
| **iPS:436408** | **21-225_214H8** | NA | CGGGCCAGTCAGAGCATTGG TGTTAGCTTACAC | TATGCTTCCCAGTCCTTC TCA | CATCAGAGTCGTAGTTTACC ATTCACT |
| | | | SEQ ID NO:3067 | SEQ ID NO:11079 | SEQ ID NO:19091 |
| | | AA | RASQSIGVSLH | YASQSFS | HQSRSLPFT |
| | | | SEQ ID NO:3068 | SEQ ID NO:11080 | SEQ ID NO:19092 |
| **iPS:436410** | **21-225_212E10** | NA | CGGGCGAGTCAGGGCATTAG CAATCATTTAGCC | GCTGCATCCAGTTTGCAA AGT | CAACAGTATAGTAATTACCC TCTCACT |
| | | | SEQ ID NO:3069 | SEQ ID NO:11081 | SEQ ID NO:19093 |
| | | AA | RASQGISNHLA | AASSLQS | QQYSNYPLT |
| | | | SEQ ID NO:3070 | SEQ ID NO:11082 | SEQ ID NO:19094 |
| **iPS:436412** | **21-225_214H9** | NA | CGGGCAAGTCAGGGCATTAG AAATGATTTAGGC | GCTGCATCCAGTTTGCAA AGT | CTACAGCATTATAGTTACCC TCGGACG |
| | | | SEQ ID NO:3071 | SEQ ID NO:11083 | SEQ ID NO:19095 |
| | | AA | RASQGIRNDLG | AASSLQS | LQHYSYPRT |
| | | | SEQ ID NO:3072 | SEQ ID NO:11084 | SEQ ID NO:19096 |
| **iPS:436414** | **21-225_214G10** | NA | CGGGCAAGTCAGGGCATTAG AAATGATTTAGGC | GCTGCATCCAGTTTGCAA AGT | CTACTGCATAATAGTTACCC TCGGACG |
| | | | SEQ ID NO:3073 | SEQ ID NO:11085 | SEQ ID NO:19097 |
| | | AA | RASQGIRNDLG | AASSLQS | LLHNSYPRT |
| | | | SEQ ID NO:3074 | SEQ ID NO:11086 | SEQ ID NO:19098 |

FIGURE 49

| | | | | | |
|---|---|---|---|---|---|
| **iPS:436416** | **21-225_214G12** | NA | CGGGCAAGTCAGGGCATTAG AAATGATTTAGGC | GCTGCATCCAGTTTGCAA AGT | GTAATGCATTATAGTTACCC TCGGACG |
| | | | SEQ ID NO:3075 | SEQ ID NO:11087 | SEQ ID NO:19099 |
| | | AA | RASQGIRNDLG | AASSLQS | VMHYSYPRT |
| | | | SEQ ID NO:3076 | SEQ ID NO:11088 | SEQ ID NO:19100 |
| **iPS:436418** | **21-225_215E3** | NA | CGGGCAAGTCAGGGCATTAG AAATGATTTAGGC | GCTGCATCCAGTTTGCAA AGT | GTAATGCATAATAGTTACCC TCGGACG |
| | | | SEQ ID NO:3077 | SEQ ID NO:11089 | SEQ ID NO:19101 |
| | | AA | RASQGIRNDLG | AASSLQS | VMHNSYPRT |
| | | | SEQ ID NO:3078 | SEQ ID NO:11090 | SEQ ID NO:19102 |
| **iPS:436420** | **21-225_215B5** | NA | CGGGCGAGTCAGGGCATTAG CAATCATTTAGCC | GCTGCATCCAGTTTGCAA AGT | CAGCAGTATATTAATTACCC TCTCACT |
| | | | SEQ ID NO:3079 | SEQ ID NO:11091 | SEQ ID NO:19103 |
| | | AA | RASQGISNHLA | AASSLQS | QQYINYPLT |
| | | | SEQ ID NO:3080 | SEQ ID NO:11092 | SEQ ID NO:19104 |
| **iPS:436422** | **21-225_215D6** | NA | CGGGCGAGTCAGGGCATTAG CAATCATTTAGCC | GCTGCATCCAGTTTGCAT AGT | CAACAGTATGTTACTTACCC TCTCACT |
| | | | SEQ ID NO:3081 | SEQ ID NO:11093 | SEQ ID NO:19105 |
| | | AA | RASQGISNHLA | AASSLHS | QQYVTYPLT |
| | | | SEQ ID NO:3082 | SEQ ID NO:11094 | SEQ ID NO:19106 |
| **iPS:436424** | **21-225_215H6** | NA | CGGGCCAGTCAGAGCATCGG TGTTAGCTTACAC | TATGCTTCCCAGTCCCTC TCA | CATCAGAGTCGCAGTTTACC ATTCACT |
| | | | SEQ ID NO:3083 | SEQ ID NO:11095 | SEQ ID NO:19107 |
| | | AA | RASQSIGVSLH | YASQSLS | HQSRSLPFT |
| | | | SEQ ID NO:3084 | SEQ ID NO:11096 | SEQ ID NO:19108 |
| **iPS:436426** | **21-225_215C7** | NA | AGGGCCAGTCAGAGGATTAC CACCAACTTCTTAGCT | GGTGCATCCAGCAGGGC CACT | CAGCAGTATGTTAGTTCATT GCTCACT |
| | | | SEQ ID NO:3085 | SEQ ID NO:11097 | SEQ ID NO:19109 |
| | | AA | RASQRITTNFLA | GASSRAT | QQYVSSLLT |
| | | | SEQ ID NO:3086 | SEQ ID NO:11098 | SEQ ID NO:19110 |

FIGURE 49

| | | | | | |
|---|---|---|---|---|---|
| **iPS:436428** | **21-225_215E11** | NA | CGGGCAAGTCAGGGCATTAG AAATGATTTAGGC | GCTGCATCCAGTTTGCAA AGT | GTAATGCATTATAGTTACCC TCGGACG |
| | | | SEQ ID NO:3087 | SEQ ID NO:11099 | SEQ ID NO:19111 |
| | | AA | RASQGIRNDLG | AASSLQS | VMHYSYPRT |
| | | | SEQ ID NO:3088 | SEQ ID NO:11100 | SEQ ID NO:19112 |
| **iPS:436430** | **21-225_215A12** | NA | CGGACGAGTCAGGACATTGG CAATTATTTAGCC | GCTGCATCCAGTTTACAG AGT | CAACAGTATGTTACTTACCC GCTCACT |
| | | | SEQ ID NO:3089 | SEQ ID NO:11101 | SEQ ID NO:19113 |
| | | AA | RTSQDIGNYLA | AASSLQS | QQYVTYPLT |
| | | | SEQ ID NO:3090 | SEQ ID NO:11102 | SEQ ID NO:19114 |
| **iPS:436432** | **21-225_215H12** | NA | AGGGCCAGTCAGAGTGTTAG CAGCAGCTTCTTAGCT | GGTGCATCCAGCAGGGC CATT | CAGCAGTATGTTAGCTCACC GCTCACT |
| | | | SEQ ID NO:3091 | SEQ ID NO:11103 | SEQ ID NO:19115 |
| | | AA | RASQSVSSSFLA | GASSRAI | QQYVSSPLT |
| | | | SEQ ID NO:3092 | SEQ ID NO:11104 | SEQ ID NO:19116 |
| **iPS:436434** | **21-225_216B10** | NA | AGGGCCAGTCAGAGTGTTAA CAACAACTTAGCC | GGTGCATCCACCAGGGC CACT | CAGCAGTATAATGACTGGCC GTGCAGT |
| | | | SEQ ID NO:3093 | SEQ ID NO:11105 | SEQ ID NO:19117 |
| | | AA | RASQSVNNNLA | GASTRAT | QQYNDWPCS |
| | | | SEQ ID NO:3094 | SEQ ID NO:11106 | SEQ ID NO:19118 |
| **iPS:436436** | **21-225_216F10** | NA | AGGGCCAGTCAGAGTGTTAG CAGCAGCTTCTTAGCC | GGTACATCCACCAGGGC CACT | CAACAGTATGATAGGTCACC ATTCACT |
| | | | SEQ ID NO:3095 | SEQ ID NO:11107 | SEQ ID NO:19119 |
| | | AA | RASQSVSSSFLA | GTSTRAT | QQYDRSPFT |
| | | | SEQ ID NO:3096 | SEQ ID NO:11108 | SEQ ID NO:19120 |
| **iPS:436438** | **21-225_216E8** | NA | CGGGCAAGTCAGGGCATTAG AAATGATTTAGGC | GCTGCATCCAGTTTGCAA AGT | CTAATGCATTATAGTTACCC TCGGACG |
| | | | SEQ ID NO:3097 | SEQ ID NO:11109 | SEQ ID NO:19121 |
| | | AA | RASQGIRNDLG | AASSLQS | LMHYSYPRT |
| | | | SEQ ID NO:3098 | SEQ ID NO:11110 | SEQ ID NO:19122 |

FIGURE 49

| | | | | | |
|---|---|---|---|---|---|
| **iPS:436440** | **21-225_216H12** | NA | CGGGCAAGTCAGGGCATTAGAAATGATTTAGGC | GGTGCATCCAGTTTGCAAAGT | GTAATGCATAATAGTTACCCTCGGACG |
| | | | SEQ ID NO:3099 | SEQ ID NO:11111 | SEQ ID NO:19123 |
| | | AA | RASQGIRNDLG | GASSLQS | VMHNSYPRT |
| | | | SEQ ID NO:3100 | SEQ ID NO:11112 | SEQ ID NO:19124 |
| **iPS:436448** | **21-225_217A3** | NA | CGGGCCAGTCAGAGCATTGGTAGTAGCTTACAC | TATGCTTCCCAGTCCTTCTCA | CATCAGAGTAGAAGTTTACCGTGGACG |
| | | | SEQ ID NO:3101 | SEQ ID NO:11113 | SEQ ID NO:19125 |
| | | AA | RASQSIGSSLH | YASQSFS | HQSRSLPWT |
| | | | SEQ ID NO:3102 | SEQ ID NO:11114 | SEQ ID NO:19126 |
| **iPS:436450** | **21-225_217E5** | NA | CGGGCAAGTCAGGGCATTAGAAATGATTTAGGC | GCTGCATCCAGTTTGCAAAGT | GTAATGCATAATAGTTACCCTCGGACG |
| | | | SEQ ID NO:3103 | SEQ ID NO:11115 | SEQ ID NO:19127 |
| | | AA | RASQGIRNDLG | AASSLQS | VMHNSYPRT |
| | | | SEQ ID NO:3104 | SEQ ID NO:11116 | SEQ ID NO:19128 |
| **iPS:436452** | **21-225_217G5** | NA | CGGGCGAGTCAGGGCATTGGCAATTATTTAGCC | GCTGCATCCAGTTTGCAAAGT | CAACAGTATGTTAATTACCCTCTCACT |
| | | | SEQ ID NO:3105 | SEQ ID NO:11117 | SEQ ID NO:19129 |
| | | AA | RASQGIGNYLA | AASSLQS | QQYVNYPLT |
| | | | SEQ ID NO:3106 | SEQ ID NO:11118 | SEQ ID NO:19130 |
| **iPS:436454** | **21-225_217B10** | NA | CGGGCGAGTCAGGCCATTGGGAAACATTTAGCC | GCTGCATCCAGATTGCAAAGT | CAACACACCAGTAAATCTCCAGTGCAG |
| | | | SEQ ID NO:3107 | SEQ ID NO:11119 | SEQ ID NO:19131 |
| | | AA | RASQAIGKHLA | AASRLQS | QHTSKSPVQ |
| | | | SEQ ID NO:3108 | SEQ ID NO:11120 | SEQ ID NO:19132 |
| **iPS:436456** | **21-225_217G10** | NA | CGGGCAAGTCAGGGCATTAGAAATGATTTAGGC | GCTGCATCCAGTTTGCAAAGT | GTAATGCATAATAGTTACCCTCGGACG |
| | | | SEQ ID NO:3109 | SEQ ID NO:11121 | SEQ ID NO:19133 |
| | | AA | RASQGIRNDLG | AASSLQS | VMHNSYPRT |
| | | | SEQ ID NO:3110 | SEQ ID NO:11122 | SEQ ID NO:19134 |

FIGURE 49

| | | | | | |
|---|---|---|---|---|---|
| **iPS:436458** | **21-225_217H12** | NA | CGGGCAAGTCAGGGCATTAGAAATGATTTAGGC | GCTGCATCCAGTTTGCAAAGT | CTAATGCATTATAGTTACCCTCGGACG |
| | | | SEQ ID NO:3111 | SEQ ID NO:11123 | SEQ ID NO:19135 |
| | | AA | RASQGIRNDLG | AASSLQS | LMHYSYPRT |
| | | | SEQ ID NO:3112 | SEQ ID NO:11124 | SEQ ID NO:19136 |
| **iPS:436462** | **21-225_218C4** | NA | CGGGCAAGTCAGGGCATTAGAAATGATTTAGGC | GCTGCATCCAGTTTGCAAAGT | GTAATGCATTATAGTTACCCTCGGACG |
| | | | SEQ ID NO:3113 | SEQ ID NO:11125 | SEQ ID NO:19137 |
| | | AA | RASQGIRNDLG | AASSLQS | VMHYSYPRT |
| | | | SEQ ID NO:3114 | SEQ ID NO:11126 | SEQ ID NO:19138 |
| **iPS:436464** | **21-225_219H1** | NA | CGGGCGAGTCAGGGCATTAGCAATTATTTAGAC | TCTGCATCCAATTTGCAAAGT | CAACACTATAGTAATTACCCGCTCACT |
| | | | SEQ ID NO:3115 | SEQ ID NO:11127 | SEQ ID NO:19139 |
| | | AA | RASQGISNYLD | SASNLQS | QHYSNYPLT |
| | | | SEQ ID NO:3116 | SEQ ID NO:11128 | SEQ ID NO:19140 |
| **iPS:436472** | **21-225_220E1** | NA | AGGGCCAGTCAGAGTATTAGCCGCAGCCACTTAGTC | GTTACATCCAGCAGGGCCACT | CAGCAGTATGGTAGCTCACCGTGGACG |
| | | | SEQ ID NO:3117 | SEQ ID NO:11129 | SEQ ID NO:19141 |
| | | AA | RASQSISRSHLV | VTSSRAT | QQYGSSPWT |
| | | | SEQ ID NO:3118 | SEQ ID NO:11130 | SEQ ID NO:19142 |
| **iPS:436480** | **21-225_220F8** | NA | CGGGCAAGTCAGGGCATTAGAAATGATTTAGGC | GGTGCATCCAGTTTGCAAAGT | GTAATGCATAATAGTTACCCTCGGACG |
| | | | SEQ ID NO:3119 | SEQ ID NO:11131 | SEQ ID NO:19143 |
| | | AA | RASQGIRNDLG | GASSLQS | VMHNSYPRT |
| | | | SEQ ID NO:3120 | SEQ ID NO:11132 | SEQ ID NO:19144 |
| **iPS:436488** | **21-225_221A6** | NA | CGGGCGAGTCAGGGTATTAGCAGCTGGTTAGCC | ACTGCATCCAATTTGCAAAGT | CAACAGGCTAACAGTTTCCCATTCACT |
| | | | SEQ ID NO:3121 | SEQ ID NO:11133 | SEQ ID NO:19145 |
| | | AA | RASQGISSWLA | TASNLQS | QQANSFPFT |
| | | | SEQ ID NO:3122 | SEQ ID NO:11134 | SEQ ID NO:19146 |

FIGURE 49

| | | | | | |
|---|---|---|---|---|---|
| **iPS:436490** | **21-225_221F6** | NA | CGGGCGAGTCAGGGCATTAG CAATTATTTAGCC | GCTGCATCCAATTTACAA AGT | CAACAGTATATGACTTACCC GCTCACT |
| | | | SEQ ID NO:3123 | SEQ ID NO:11135 | SEQ ID NO:19147 |
| | | AA | RASQGISNYLA | AASNLQS | QQYMTYPLT |
| | | | SEQ ID NO:3124 | SEQ ID NO:11136 | SEQ ID NO:19148 |
| **iPS:436494** | **21-225_221F12** | NA | CGGGCGAGTCAGGGTATTAG CAGCTGGTTAGCC | ACTGCATCCAATTTGCAA AGT | CAACAGGCTAACAGTTTCCC ATTCACT |
| | | | SEQ ID NO:3125 | SEQ ID NO:11137 | SEQ ID NO:19149 |
| | | AA | RASQGISSWLA | TASNLQS | QQANSFPFT |
| | | | SEQ ID NO:3126 | SEQ ID NO:11138 | SEQ ID NO:19150 |
| **iPS:436496** | **21-225_222E1** | NA | CGGGCGAGTCAGGGTATTAG CAGCTGGTTAGCC | ACTGCATCCAATTTGCAA AGT | CAACAGGCTAACAGTTTCCC ATTCACT |
| | | | SEQ ID NO:3127 | SEQ ID NO:11139 | SEQ ID NO:19151 |
| | | AA | RASQGISSWLA | TASNLQS | QQANSFPFT |
| | | | SEQ ID NO:3128 | SEQ ID NO:11140 | SEQ ID NO:19152 |
| **iPS:436500** | **21-225_222H3** | NA | AAGTCCAGCCAGAGTGTTTT GAAAAGTTCCAACCATAGGA ACTACTTAGCT | TGGGCATCTACCCGGGA AACC | CAGCAATATTCTTCTATTCCG TGGACG |
| | | | SEQ ID NO:3129 | SEQ ID NO:11141 | SEQ ID NO:19153 |
| | | AA | KSSQSVLKSSNHRNYLA | WASTRET | QQYSSIPWT |
| | | | SEQ ID NO:3130 | SEQ ID NO:11142 | SEQ ID NO:19154 |
| **iPS:436502** | **21-225_222A11** | NA | CGGGCGAGTCAGGGCATTAG CAATTATTTGGCC | GCTGCATCCAGTTTGCAA AGT | CTATATTATCTTAATTATCCG CTCACT |
| | | | SEQ ID NO:3131 | SEQ ID NO:11143 | SEQ ID NO:19155 |
| | | AA | RASQGISNYLA | AASSLQS | LYYLNYPLT |
| | | | SEQ ID NO:3132 | SEQ ID NO:11144 | SEQ ID NO:19156 |
| **iPS:436504** | **21-225_222H4** | NA | CGGGCAAGTCAGAACATTAG TAATTATGTTAAT | ACTGCATCGAGTTTGCA AAGT | CAGCAGTATTACTTTACCCC ATTCACT |
| | | | SEQ ID NO:3133 | SEQ ID NO:11145 | SEQ ID NO:19157 |
| | | AA | RASQNISNYVN | TASSLQS | QQYYFTPFT |

**FIGURE 49**

| | | | | | |
|---|---|---|---|---|---|
| | | | SEQ ID NO:3134 | SEQ ID NO:11146 | SEQ ID NO:19158 |
| **iPS:436506** | **21-225_222C7** | NA | AGGGCCAGTCAGAGTGTTTACAGCAACTACTTAGCC | GGTGCATCAAGCAGGGCCACT | CAGCAGTATGAAGACTCACCGTGGACG |
| | | | SEQ ID NO:3135 | SEQ ID NO:11147 | SEQ ID NO:19159 |
| | | AA | RASQSVYSNYLA | GASSRAT | QQYEDSPWT |
| | | | SEQ ID NO:3136 | SEQ ID NO:11148 | SEQ ID NO:19160 |
| **iPS:436508** | **21-225_222F7** | NA | CGGGCGAGTCAGGGTATTAGCAGCTGGTTAGCC | ACTGCATCCAATTTGCAAAGT | CAACAGGCTAACAGTTTCCCATTCACT |
| | | | SEQ ID NO:3137 | SEQ ID NO:11149 | SEQ ID NO:19161 |
| | | AA | RASQGISSWLA | TASNLQS | QQANSFPFT |
| | | | SEQ ID NO:3138 | SEQ ID NO:11150 | SEQ ID NO:19162 |
| **iPS:436510** | **21-225_222H8** | NA | CGGGCAAGTCAGAACATTAGTAATTATGTTAAT | ATTGCATCGAGTTTGCAAAGT | CAGCAGTATTACTTTACCCCATTCACT |
| | | | SEQ ID NO:3139 | SEQ ID NO:11151 | SEQ ID NO:19163 |
| | | AA | RASQNISNYVN | IASSLQS | QQYYFTPFT |
| | | | SEQ ID NO:3140 | SEQ ID NO:11152 | SEQ ID NO:19164 |
| **iPS:436514** | **21-225_222D10** | NA | CGGGCGAGTCAGGGCATTAGCAATTATTTGGCC | GCTGCATCCAGTTTGCAAAGT | CTACATTATCTTAATTACCCGCTCACT |
| | | | SEQ ID NO:3141 | SEQ ID NO:11153 | SEQ ID NO:19165 |
| | | AA | RASQGISNYLA | AASSLQS | LHYLNYPLT |
| | | | SEQ ID NO:3142 | SEQ ID NO:11154 | SEQ ID NO:19166 |
| **iPS:436516** | **21-225_222C12** | NA | CGGGTGAGTCAGGGTATTAGCAGCTGGTTAGCC | ACTGCATCCAATTTGCAAAGT | CAACAGGATAACAGTTTCCCATTCACT |
| | | | SEQ ID NO:3143 | SEQ ID NO:11155 | SEQ ID NO:19167 |
| | | AA | RVSQGISSWLA | TASNLQS | QQDNSFPFT |
| | | | SEQ ID NO:3144 | SEQ ID NO:11156 | SEQ ID NO:19168 |
| **iPS:436520** | **21-225_223G10** | NA | AAGTCCAGCCAGAGTATTTTACTCAGCTCCAACAATAAGAACTACTTAGCT | TGGGCATCTACCCGGGAATCC | CTGCAATATTTTAGTACTCCGTGGACG |
| | | | SEQ ID NO:3145 | SEQ ID NO:11157 | SEQ ID NO:19169 |

## FIGURE 49

| | | | | | |
|---|---|---|---|---|---|
| | | AA | KSSQSILLSSNNKNYLA | WASTRES | LQYFSTPWT |
| | | | SEQ ID NO:3146 | SEQ ID NO:11158 | SEQ ID NO:19170 |
| iPS:436522 | 21-225_223H10 | NA | CGGGCGAGTCAGGGCATTAG TAATTATTTGGCC | GCTGCATCCAATTTGCAA AGT | CTACATTATCTTAATTACCCA CTCACT |
| | | | SEQ ID NO:3147 | SEQ ID NO:11159 | SEQ ID NO:19171 |
| | | AA | RASQGISNYLA | AASNLQS | LHYLNYPLT |
| | | | SEQ ID NO:3148 | SEQ ID NO:11160 | SEQ ID NO:19172 |
| iPS:436526 | 21-225_224A1 | NA | CGGGCAAGTCAGGGCATTGA AAATGATTTAGGC | GCTGCATCCAGTTTGCAA AGT | CTACAGCATAATAGTTACCC GCTCACT |
| | | | SEQ ID NO:3149 | SEQ ID NO:11161 | SEQ ID NO:19173 |
| | | AA | RASQGIENDLG | AASSLQS | LQHNSYPLT |
| | | | SEQ ID NO:3150 | SEQ ID NO:11162 | SEQ ID NO:19174 |
| iPS:436528 | 21-225_224B1 | NA | CGGGCAAGTCAGGGCATTAG TAATGATTTAGGC | GCTGCATCCAGTTTGCAA AGT | CTACAGCATAATAGTTATCC TCCTACT |
| | | | SEQ ID NO:3151 | SEQ ID NO:11163 | SEQ ID NO:19175 |
| | | AA | RASQGISNDLG | AASSLQS | LQHNSYPPT |
| | | | SEQ ID NO:3152 | SEQ ID NO:11164 | SEQ ID NO:19176 |
| iPS:436534 | 21-225_224F1 | NA | CGGGCAAGTCAGGGCATTAG AGATGATTTAGGC | GCTGCATCCAGTTTGCAA AGT | CTACAGCATTATAGTTACCC TCGGACG |
| | | | SEQ ID NO:3153 | SEQ ID NO:11165 | SEQ ID NO:19177 |
| | | AA | RASQGIRDDLG | AASSLQS | LQHYSYPRT |
| | | | SEQ ID NO:3154 | SEQ ID NO:11166 | SEQ ID NO:19178 |
| iPS:436536 | 21-225_224G1 | NA | AAGTCTGGTCAGAGCCTCCT GCATAGTGATGGAAAGACCT TTTTGTCT | GAAATTTCCAACCGGTTC TCT | ATGCAAAGTACACAGCTTCC TCGGACG |
| | | | SEQ ID NO:3155 | SEQ ID NO:11167 | SEQ ID NO:19179 |
| | | AA | KSGQSLLHSDGKTFLS | EISNRFS | MQSTQLPRT |
| | | | SEQ ID NO:3156 | SEQ ID NO:11168 | SEQ ID NO:19180 |
| iPS:436538 | | NA | CGGGCAAGTCAGGACATTAG AAATGATTTAGGC | GCTACATCCAGTTTGCAA AGT | CTACAGCATAATAGTTACCC GCTCACT |

**FIGURE 49**

| | | | | | |
|---|---|---|---|---|---|
| | **21-225_224C3** | | SEQ ID NO:3157 | SEQ ID NO:11169 | SEQ ID NO:19181 |
| | | AA | RASQDIRNDLG | ATSSLQS | LQHNSYPLT |
| | | | SEQ ID NO:3158 | SEQ ID NO:11170 | SEQ ID NO:19182 |
| **iPS:436540** | **21-225_224F3** | NA | CGGGCAAGTCAGGGCATTAG AAATGATTTAGGC | GCTGCATCCAGTTTGCAA AGT | CTTCAGCATTATAATTACCCT CGGGCG |
| | | | SEQ ID NO:3159 | SEQ ID NO:11171 | SEQ ID NO:19183 |
| | | AA | RASQGIRNDLG | AASSLQS | LQHYNYPRA |
| | | | SEQ ID NO:3160 | SEQ ID NO:11172 | SEQ ID NO:19184 |
| **iPS:436544** | **21-225_224H5** | NA | AAGTCCAGCCAGAGTGTTTT ATACAGCTCCAACAATTTCA ACTACTTAACT | TGGGCATCTACCCGGGA ATCC | CAGCAATATTATAGTACTCC TCCGACG |
| | | | SEQ ID NO:3161 | SEQ ID NO:11173 | SEQ ID NO:19185 |
| | | AA | KSSQSVLYSSNNFNYLT | WASTRES | QQYYSTPPT |
| | | | SEQ ID NO:3162 | SEQ ID NO:11174 | SEQ ID NO:19186 |
| **iPS:436546** | **21-225_224D6** | NA | CGGGCGAGTCAGGGTATTAG CAGCTGGTTAGCC | GCTGCATCCAGTTTGCAA AGT | CAACAGGCTAACAGTTTCCC GTGGACG |
| | | | SEQ ID NO:3163 | SEQ ID NO:11175 | SEQ ID NO:19187 |
| | | AA | RASQGISSWLA | AASSLQS | QQANSFPWT |
| | | | SEQ ID NO:3164 | SEQ ID NO:11176 | SEQ ID NO:19188 |
| **iPS:436548** | **21-225_224A7** | NA | AAGTCTAGTCAGAGCCTCCT GCATAGTGATGGAAAGACCT TTTTGTAT | GAAATTTCCAACCGGTTC TCT | ATGCAAAGTACACAGCTTCC TCGGACG |
| | | | SEQ ID NO:3165 | SEQ ID NO:11177 | SEQ ID NO:19189 |
| | | AA | KSSQSLLHSDGKTFLY | EISNRFS | MQSTQLPRT |
| | | | SEQ ID NO:3166 | SEQ ID NO:11178 | SEQ ID NO:19190 |
| **iPS:436550** | **21-225_224D8** | NA | AAGTCCAGCCAGAGTGTTTT ATACAGCTCCAACAATAAGA ACTACTTAGCT | TGGTCGTCTACCCGGAA ATCC | CAGCAATATTTTAGTACTCC TCCGACG |
| | | | SEQ ID NO:3167 | SEQ ID NO:11179 | SEQ ID NO:19191 |
| | | AA | KSSQSVLYSSNNKNYLA | WSSTRKS | QQYFSTPPT |

FIGURE 49

| | | | | | |
|---|---|---|---|---|---|
| | | | SEQ ID NO:3168 | SEQ ID NO:11180 | SEQ ID NO:19192 |
| **iPS:436554** | **21-225_224C10** | NA | AAGTCCAGCCAGAGTGTTTTATACAATTCCAACAATAAGAACTACTTAGCT | TGGGCATCTACCCGGGAGTCC | CAACAATATTATATTAATCCGTGCAGT |
| | | | SEQ ID NO:3169 | SEQ ID NO:11181 | SEQ ID NO:19193 |
| | | AA | KSSQSVLYNSNNKNYLA | WASTRES | QQYYINPCS |
| | | | SEQ ID NO:3170 | SEQ ID NO:11182 | SEQ ID NO:19194 |
| **iPS:436556** | **21-225_224D10** | NA | CGGGCAAGTCAGGGCATTAGAAATGATTTAGGC | GCTGCATCCAGTTTACAAAGT | CTACAACATAATAGTTACCCGCTCACT |
| | | | SEQ ID NO:3171 | SEQ ID NO:11183 | SEQ ID NO:19195 |
| | | AA | RASQGIRNDLG | AASSLQS | LQHNSYPLT |
| | | | SEQ ID NO:3172 | SEQ ID NO:11184 | SEQ ID NO:19196 |
| **iPS:436558** | **21-225_224C11** | NA | AAGTCTAGTCAGAGCCTCCTGCATAGTGATGGAAAGACCTTTTTGTAT | GAAATTTCCAACCGGTTCTCT | ATGCAAAGTACACAGCTTCCTCGGACG |
| | | | SEQ ID NO:3173 | SEQ ID NO:11185 | SEQ ID NO:19197 |
| | | AA | KSSQSLLHSDGKTFLY | EISNRFS | MQSTQLPRT |
| | | | SEQ ID NO:3174 | SEQ ID NO:11186 | SEQ ID NO:19198 |
| **iPS:436560** | **21-225_224F11** | NA | AAGTCCAGCCAGAGTGTTTTATCCAGCTCCAACAATCACAACTACTTAGCT | TGGGCATCTACCCGGGAATCC | CAGCAATATTATACTACTCCGTGCAGT |
| | | | SEQ ID NO:3175 | SEQ ID NO:11187 | SEQ ID NO:19199 |
| | | AA | KSSQSVLSSSNNHNYLA | WASTRES | QQYYTTPCS |
| | | | SEQ ID NO:3176 | SEQ ID NO:11188 | SEQ ID NO:19200 |
| **iPS:436562** | **21-225_224H11** | NA | AAGTCTAGTCAGAGCCTCCTGCATAGTGATGGAAAGACCTTTTTGTAT | GAAATTTCCAACCGGTTCTCT | ATGCAAAGTACACAGCTTCCTCGGACG |
| | | | SEQ ID NO:3177 | SEQ ID NO:11189 | SEQ ID NO:19201 |
| | | AA | KSSQSLLHSDGKTFLY | EISNRFS | MQSTQLPRT |
| | | | SEQ ID NO:3178 | SEQ ID NO:11190 | SEQ ID NO:19202 |

FIGURE 49

| | | | | | |
|---|---|---|---|---|---|
| **iPS:436564** | **21-225_225A1** | NA | CGGGCAAGTCAGGGCATTAGAGATGATTTAGGC | GCTGCATCCAGTTTGCAAAGT | CTGCAGCATTATAGTTACCCTCGGACG |
| | | | SEQ ID NO:3179 | SEQ ID NO:11191 | SEQ ID NO:19203 |
| | | AA | RASQGIRDDLG | AASSLQS | LQHYSYPRT |
| | | | SEQ ID NO:3180 | SEQ ID NO:11192 | SEQ ID NO:19204 |
| **iPS:436568** | **21-225_225B3** | NA | AGGGCCAGTCAGAATCTTAGCAGCAGCTACTTAGGC | GATACATCCAGCAGGGCCACT | CAGGAGTATGGTAGCTCACTCATGTGCAGT |
| | | | SEQ ID NO:3181 | SEQ ID NO:11193 | SEQ ID NO:19205 |
| | | AA | RASQNLSSSYLG | DTSSRAT | QEYGSSLMCS |
| | | | SEQ ID NO:3182 | SEQ ID NO:11194 | SEQ ID NO:19206 |
| **iPS:436570** | **21-225_225F4** | NA | AAGTCCAGCCAGAGTGTTTTATATAGCTCCAACAATAAGAACTACTTAGCT | TGGGCATCTACCCGGGAATCC | CACCAATATCATAATTCTCCTCCCACT |
| | | | SEQ ID NO:3183 | SEQ ID NO:11195 | SEQ ID NO:19207 |
| | | AA | KSSQSVLYSSNNKNYLA | WASTRES | HQYHNSPPT |
| | | | SEQ ID NO:3184 | SEQ ID NO:11196 | SEQ ID NO:19208 |
| **iPS:436572** | **21-225_225G4** | NA | AAGTCTAGTCAGAGCCTCCTGCATAGTGATGGAAAGACCTTTTTGTAT | GAAATTTCCAACCGGTTCTCT | ATGCAAAGTACACAGCTTCCTCGGACG |
| | | | SEQ ID NO:3185 | SEQ ID NO:11197 | SEQ ID NO:19209 |
| | | AA | KSSQSLLHSDGKTFLY | EISNRFS | MQSTQLPRT |
| | | | SEQ ID NO:3186 | SEQ ID NO:11198 | SEQ ID NO:19210 |
| **iPS:436574** | **21-225_225F5** | NA | AAGTCCAGCCAGAATGTTTTATACAACTCCAACAATAACAACTACTTAGCT | TGGGCATCTACCCGGGAATCC | CAGCAATATTATAGTTCTCCTCCGACG |
| | | | SEQ ID NO:3187 | SEQ ID NO:11199 | SEQ ID NO:19211 |
| | | AA | KSSQNVLYNSNNNNYLA | WASTRES | QQYYSSPPT |
| | | | SEQ ID NO:3188 | SEQ ID NO:11200 | SEQ ID NO:19212 |
| **iPS:436576** | | NA | CGGGCAAGTCAGGGCATGAGAAAAGATTTAGGC | GCTGCAACCAGTTTGCAAAGT | CTACAGCATAATAGTTATCCATTCACT |

FIGURE 49

| | | | | | |
|---|---|---|---|---|---|
| | **21-225_225B6** | | SEQ ID NO:3189 | SEQ ID NO:11201 | SEQ ID NO:19213 |
| | | AA | RASQGMRKDLG | AATSLQS | LQHNSYPFT |
| | | | SEQ ID NO:3190 | SEQ ID NO:11202 | SEQ ID NO:19214 |
| **iPS:436578** | **21-225_225D6** | NA | CGGGCAAGTCAGGGCATTAG AAATGATTTAGGC | GCTGCATCCAGTTTACAA AGT | CTTCAGCATAATACTTACCC ATTCACT |
| | | | SEQ ID NO:3191 | SEQ ID NO:11203 | SEQ ID NO:19215 |
| | | AA | RASQGIRNDLG | AASSLQS | LQHNTYPFT |
| | | | SEQ ID NO:3192 | SEQ ID NO:11204 | SEQ ID NO:19216 |
| **iPS:436580** | **21-225_225E7** | NA | AGGGCCAGTCAGAGTGTTTA CAGCAGCTACTTAGCC | GGTGCATCCAGCAGGGC CACT | CAGCAGTATGGTACCTCACC TCGGACG |
| | | | SEQ ID NO:3193 | SEQ ID NO:11205 | SEQ ID NO:19217 |
| | | AA | RASQSVYSSYLA | GASSRAT | QQYGTSPRT |
| | | | SEQ ID NO:3194 | SEQ ID NO:11206 | SEQ ID NO:19218 |
| **iPS:436582** | **21-225_225F8** | NA | CGGGCAAGTCAGGGCATTAG AAATGATTTAGGC | GCTGCATCCAGTTTGCTA GGT | CTACAACATAATAGTTACCC ATTCACT |
| | | | SEQ ID NO:3195 | SEQ ID NO:11207 | SEQ ID NO:19219 |
| | | AA | RASQGIRNDLG | AASSLLG | LQHNSYPFT |
| | | | SEQ ID NO:3196 | SEQ ID NO:11208 | SEQ ID NO:19220 |
| **iPS:436584** | **21-225_225B9** | NA | AAGTCCAGCCAGAGTGTTTT ATACAGCTCCAACAATAACA ACTACTTAGCT | TGGGCATCTACCCGGGA ATCC | CAACACTCCAAGAGTATTCC TGGTAAG |
| | | | SEQ ID NO:3197 | SEQ ID NO:11209 | SEQ ID NO:19221 |
| | | AA | KSSQSVLYSSNNNNYLA | WASTRES | QHSKSIPGK |
| | | | SEQ ID NO:3198 | SEQ ID NO:11210 | SEQ ID NO:19222 |
| **iPS:436586** | **21-225_225F11** | NA | AAGTCCAGCCAGAGTGTTTT ATACAGCTCCAACAATTACA ACTACTTAGCT | TGGGCTTCTACCCGGGA ATCC | CAGCAATATTATACTACTCC TCCGACG |
| | | | SEQ ID NO:3199 | SEQ ID NO:11211 | SEQ ID NO:19223 |
| | | AA | KSSQSVLYSSNNYNYLA | WASTRES | QQYYTTPPT |
| | | | SEQ ID NO:3200 | SEQ ID NO:11212 | SEQ ID NO:19224 |

FIGURE 49

| | | | | | |
|---|---|---|---|---|---|
| **iPS:436588** | **21-225_225F12** | NA | AAGTCCAGCCAGAGTGTTTTATACAGCTCCAACAATCAGAACTACTTAGCT | TGGACATCTACCCGGGAATCC | CAGCAATATTATATTACTCCGTGCAGT |
| | | | SEQ ID NO:3201 | SEQ ID NO:11213 | SEQ ID NO:19225 |
| | | AA | KSSQSVLYSSNNQNYLA | WTSTRES | QQYYITPCS |
| | | | SEQ ID NO:3202 | SEQ ID NO:11214 | SEQ ID NO:19226 |
| **iPS:436590** | **21-225_225H12** | NA | AAGTCCAGCCAGAGTGTTTTATACAACTCCAACAATAACAACTACTTAGCT | TGGGCATCTACCCGGGAATCC | CAGCAATATTATATTACTCCGTGCAGT |
| | | | SEQ ID NO:3203 | SEQ ID NO:11215 | SEQ ID NO:19227 |
| | | AA | KSSQSVLYNSNNNNYLA | WASTRES | QQYYITPCS |
| | | | SEQ ID NO:3204 | SEQ ID NO:11216 | SEQ ID NO:19228 |
| **iPS:436592** | **21-225_226B1** | NA | AAGTCTAGTCAGAGCCTCCTGCATGGTGATGGAAAGACCTATTTGTAT | GAAGTTTCCATCCGGTTCTCT | ATGCAAAGTATACAGATTCCGTGGACG |
| | | | SEQ ID NO:3205 | SEQ ID NO:11217 | SEQ ID NO:19229 |
| | | AA | KSSQSLLHGDGKTYLY | EVSIRFS | MQSIQIPWT |
| | | | SEQ ID NO:3206 | SEQ ID NO:11218 | SEQ ID NO:19230 |
| **iPS:436594** | **21-225_226A5** | NA | AAGTCTAGTCAGAGCCTCCTACATGGTGATGGAAAGACCTATTTGTAT | GAAGTTTCCAACCGGTTCTCT | ATGCAAAGTATACAGATTCCGTGGACG |
| | | | SEQ ID NO:3207 | SEQ ID NO:11219 | SEQ ID NO:19231 |
| | | AA | KSSQSLLHGDGKTYLY | EVSNRFS | MQSIQIPWT |
| | | | SEQ ID NO:3208 | SEQ ID NO:11220 | SEQ ID NO:19232 |
| **iPS:436596** | **21-225_226C6** | NA | CGGGCAAGTCAGGGCATTAGAAATGATTTAGGC | GCTGCATCCAGTTTGCAAAGT | CTACAGCATTATAATTACCCTCGGGCG |
| | | | SEQ ID NO:3209 | SEQ ID NO:11221 | SEQ ID NO:19233 |
| | | AA | RASQGIRNDLG | AASSLQS | LQHYNYPRA |
| | | | SEQ ID NO:3210 | SEQ ID NO:11222 | SEQ ID NO:19234 |

FIGURE 49

| | | | | | |
|---|---|---|---|---|---|
| **iPS:436598** | **21-225_226D6** | NA | AGGTCCAGCCAGAGTATTTTATACATCTCCAACAATAAGAACTACTTAGCT | TGGGCATCTACCCGGGAATCC | CAGCAATATTATAGTTCTCCGTGCAGT |
| | | | SEQ ID NO:3211 | SEQ ID NO:11223 | SEQ ID NO:19235 |
| | | AA | RSSQSILYISNNKNYLA | WASTRES | QQYYSSPCS |
| | | | SEQ ID NO:3212 | SEQ ID NO:11224 | SEQ ID NO:19236 |
| **iPS:436600** | **21-225_226F6** | NA | AAGTCCAGCCAGAGTATTTTATACAGCTCCAACAATTACAACTACTTAGCT | TGGGCTTCTACCCGGGAATCC | CAGCAATATTATACTACTCCTCCGACG |
| | | | SEQ ID NO:3213 | SEQ ID NO:11225 | SEQ ID NO:19237 |
| | | AA | KSSQSILYSSNNYNYLA | WASTRES | QQYYTTPPT |
| | | | SEQ ID NO:3214 | SEQ ID NO:11226 | SEQ ID NO:19238 |
| **iPS:436602** | **21-225_226E7** | NA | AAGTCTAGTCAGAGCCTCCTGCATGGTGATGGAAAGACCTATTTGTAT | GAAGTTTCCAACCGGTTCTCT | ATGCAAAGTATACAGGTTCCGTGGACG |
| | | | SEQ ID NO:3215 | SEQ ID NO:11227 | SEQ ID NO:19239 |
| | | AA | KSSQSLLHGDGKTYLY | EVSNRFS | MQSIQVPWT |
| | | | SEQ ID NO:3216 | SEQ ID NO:11228 | SEQ ID NO:19240 |
| **iPS:436604** | **21-225_226F7** | NA | CGGGCAAGTCAGGGCATTGGGAATGATTTAGGC | GCTGCCTCCAGTTTGCAAAGT | CTACATCATTATAGTTACCCTCGGACG |
| | | | SEQ ID NO:3217 | SEQ ID NO:11229 | SEQ ID NO:19241 |
| | | AA | RASQGIGNDLG | AASSLQS | LHHYSYPRT |
| | | | SEQ ID NO:3218 | SEQ ID NO:11230 | SEQ ID NO:19242 |
| **iPS:436606** | **21-225_226G8** | NA | AAGTCTAGTCAGAGCCTCCTGCATAGTGATGGAAAGACCTTTTTGTAT | GAAATTTCCAACCGGTTCTCT | ATGCAAAGTACACAGCTTCCTCGGACG |
| | | | SEQ ID NO:3219 | SEQ ID NO:11231 | SEQ ID NO:19243 |
| | | AA | KSSQSLLHSDGKTFLY | EISNRFS | MQSTQLPRT |
| | | | SEQ ID NO:3220 | SEQ ID NO:11232 | SEQ ID NO:19244 |

FIGURE 49

| | | | | | |
|---|---|---|---|---|---|
| **iPS:436608** | **21-225_226A9** | NA | CGGGCAAGTCAGGGCATTAGAAATGATTTAGGC | GCTGCATCCAGTTTGCAAAGT | CTACAGCATAATAGTTACCCATTCACT |
| | | | SEQ ID NO:3221 | SEQ ID NO:11233 | SEQ ID NO:19245 |
| | | AA | RASQGIRNDLG | AASSLQS | LQHNSYPFT |
| | | | SEQ ID NO:3222 | SEQ ID NO:11234 | SEQ ID NO:19246 |
| **iPS:436610** | **21-225_226F9** | NA | AAGTCTAGTCAGAGCCTCCTGCATAGTGATGGAAAGACCTTTTTGTAT | GAAATTTCCAACCGGTTCTCT | ATGCAAAGTACACAGCTTCCTCGGACG |
| | | | SEQ ID NO:3223 | SEQ ID NO:11235 | SEQ ID NO:19247 |
| | | AA | KSSQSLLHSDGKTFLY | EISNRFS | MQSTQLPRT |
| | | | SEQ ID NO:3224 | SEQ ID NO:11236 | SEQ ID NO:19248 |
| **iPS:436612** | **21-225_226H9** | NA | AAGTCTAGTCAGAGCCTCCTGCATAGTGATGGAAAGACCTTTTTGTAT | GAAGTTTCCAACCGGTTCTCT | ATGCAAAGTACACAGCTTCCTCGGACG |
| | | | SEQ ID NO:3225 | SEQ ID NO:11237 | SEQ ID NO:19249 |
| | | AA | KSSQSLLHSDGKTFLY | EVSNRFS | MQSTQLPRT |
| | | | SEQ ID NO:3226 | SEQ ID NO:11238 | SEQ ID NO:19250 |
| **iPS:436614** | **21-225_226F10** | NA | AAGTCTAGTCAGAGCCTCCTGCATAGTGATGGAAAGACCTTTTTGTAT | GAAATTTCCAACCGGTTCTCT | ATGCAAAGTACACAGCTTCCTCGGACG |
| | | | SEQ ID NO:3227 | SEQ ID NO:11239 | SEQ ID NO:19251 |
| | | AA | KSSQSLLHSDGKTFLY | EISNRFS | MQSTQLPRT |
| | | | SEQ ID NO:3228 | SEQ ID NO:11240 | SEQ ID NO:19252 |
| **iPS:436616** | **21-225_226D11** | NA | AAGTCCAGCCAGAATGTTTTACACAGCTCCAACAGTAATAACTACTTAGTT | TGGGCATCTACCCGGGAATCC | CAGCAATATTATAAAACTCCGTGGACG |
| | | | SEQ ID NO:3229 | SEQ ID NO:11241 | SEQ ID NO:19253 |
| | | AA | KSSQNVLHSSNSNNYLV | WASTRES | QQYYKTPWT |
| | | | SEQ ID NO:3230 | SEQ ID NO:11242 | SEQ ID NO:19254 |

FIGURE 49

| | | | | | |
|---|---|---|---|---|---|
| **iPS:436618** | **21-225_226E11** | NA | AAGTCTAGTCAGAGCCTCCTGCATAGTGATGGAAAGACCTTTTTGTAT | GAAATTTCCAACCGGTTCTCT | ATGCAAAGTACACAGCTTCCTCGGACG |
| | | | SEQ ID NO:3231 | SEQ ID NO:11243 | SEQ ID NO:19255 |
| | | AA | KSSQSLLHSDGKTFLY | EISNRFS | MQSTQLPRT |
| | | | SEQ ID NO:3232 | SEQ ID NO:11244 | SEQ ID NO:19256 |
| **iPS:436620** | **21-225_226H11** | NA | CGGACAAGTCAGGGCATTAGAAATGATTTAGGC | GCTGCATCCAGTTTGCAAAGT | CTACAGCATTATAGTTACCCTCGGACG |
| | | | SEQ ID NO:3233 | SEQ ID NO:11245 | SEQ ID NO:19257 |
| | | AA | RTSQGIRNDLG | AASSLQS | LQHYSYPRT |
| | | | SEQ ID NO:3234 | SEQ ID NO:11246 | SEQ ID NO:19258 |
| **iPS:436622** | **21-225_226A12** | NA | AAGTCCAGCCAGAATGTTTTATACAGTTCCAACAATAACAACTACTTAGCT | TGGGCATCTACCCGGAAATCC | CAGCAATATTATAGTAGTCCTCCGACG |
| | | | SEQ ID NO:3235 | SEQ ID NO:11247 | SEQ ID NO:19259 |
| | | AA | KSSQNVLYSSNNNNYLA | WASTRKS | QQYYSSPPT |
| | | | SEQ ID NO:3236 | SEQ ID NO:11248 | SEQ ID NO:19260 |
| **iPS:436624** | **21-225_226H12** | NA | AAGTCTAGTAAGACCCTCCTGCATAGTGATGGAAAGACCTTTTTGTAT | GAAATTTCCAACCGGTTCTCT | ATGCAAAGTACACAGCTTCCTCGGACG |
| | | | SEQ ID NO:3237 | SEQ ID NO:11249 | SEQ ID NO:19261 |
| | | AA | KSSKTLLHSDGKTFLY | EISNRFS | MQSTQLPRT |
| | | | SEQ ID NO:3238 | SEQ ID NO:11250 | SEQ ID NO:19262 |
| **iPS:436626** | **21-225_227C1** | NA | AAGTCTAGTCAGAGCCTCCTGCATAGTGATGGAAAGACCTTTTTGTAT | GAAATTTCCAACCGGTTCTCT | ATGCAAAGTACACAGCTTCCTCGGACG |
| | | | SEQ ID NO:3239 | SEQ ID NO:11251 | SEQ ID NO:19263 |
| | | AA | KSSQSLLHSDGKTFLY | EISNRFS | MQSTQLPRT |
| | | | SEQ ID NO:3240 | SEQ ID NO:11252 | SEQ ID NO:19264 |

FIGURE 49

| | | | | | |
|---|---|---|---|---|---|
| **iPS:436628** | **21-225_227F2** | NA | AAGTCTAGTCAGAGCCTCCTGCATAGTGATGGAAAGACCTTTTTGTAT | GAAATTTCCAACCGGTTCTCT | ATGCAAAGTACACAGCTTCCTCGGACG |
| | | | SEQ ID NO:3241 | SEQ ID NO:11253 | SEQ ID NO:19265 |
| | | AA | KSSQSLLHSDGKTFLY | EISNRFS | MQSTQLPRT |
| | | | SEQ ID NO:3242 | SEQ ID NO:11254 | SEQ ID NO:19266 |
| **iPS:436630** | **21-225_227G3** | NA | CGGGCAAGTCAGGGCATTAGAAATGATTTAGGC | GCTGCATCCAGTTTGCAAAGT | CTACACCATAATAGTTACCCATTCACT |
| | | | SEQ ID NO:3243 | SEQ ID NO:11255 | SEQ ID NO:19267 |
| | | AA | RASQGIRNDLG | AASSLQS | LHHNSYPFT |
| | | | SEQ ID NO:3244 | SEQ ID NO:11256 | SEQ ID NO:19268 |
| **iPS:436632** | **21-225_227E4** | NA | CGGGCGAGTCAGGGTATTATCAACTGGTTAGCC | GCTGCATCCAGTTTGCAAAGT | CAACAGGCTAACAGTTTCCCGTGGACG |
| | | | SEQ ID NO:3245 | SEQ ID NO:11257 | SEQ ID NO:19269 |
| | | AA | RASQGIINWLA | AASSLQS | QQANSFPWT |
| | | | SEQ ID NO:3246 | SEQ ID NO:11258 | SEQ ID NO:19270 |
| **iPS:436634** | **21-225_227H5** | NA | CGGGCAAGTCAGGACATTAGAAATGATTTAGGC | GCTGCATCCAGTTTGCAAAGT | CTACAGCATAATAGTTACCCATTCACT |
| | | | SEQ ID NO:3247 | SEQ ID NO:11259 | SEQ ID NO:19271 |
| | | AA | RASQDIRNDLG | AASSLQS | LQHNSYPFT |
| | | | SEQ ID NO:3248 | SEQ ID NO:11260 | SEQ ID NO:19272 |
| **iPS:436636** | **21-225_227E6** | NA | AAGTCCAGCCAGAGTGTTTTATACAGCTCCAACGATAAGAACTACTTAGCT | TGGGCATCTACCCGGGAATCC | CAGCAATATTATATTACTCCGTGCAGT |
| | | | SEQ ID NO:3249 | SEQ ID NO:11261 | SEQ ID NO:19273 |
| | | AA | KSSQSVLYSSNDKNYLA | WASTRES | QQYYITPCS |
| | | | SEQ ID NO:3250 | SEQ ID NO:11262 | SEQ ID NO:19274 |
| **iPS:436638** | **21 225 227C7** | NA | AGGTCCAGCCAGATTGTTTTATCCGACTCCAACAATAACAACTACTTAGCT | TGGGCATCTACCCGGGAATCC | CAGCAATATTATAGTTCTCCTCCGACG |

FIGURE 49

| | | | | | |
|---|---|---|---|---|---|
| | **21-225_227C7** | | SEQ ID NO:3251 | SEQ ID NO:11263 | SEQ ID NO:19275 |
| | | AA | RSSQIVLSDSNNNNYLA | WASTRES | QQYYSSPPT |
| | | | SEQ ID NO:3252 | SEQ ID NO:11264 | SEQ ID NO:19276 |
| **iPS:436640** | **21-225_227A8** | NA | AAGTCTAGTCAGAGCCTCCT GCATAGTGATGGAAAGACCT TTTTGTAT | GAAATTTCCAACCGGTTC TCT | ATGCAAAGTACACAGCTTCC TCGGACG |
| | | | SEQ ID NO:3253 | SEQ ID NO:11265 | SEQ ID NO:19277 |
| | | AA | KSSQSLLHSDGKTFLY | EISNRFS | MQSTQLPRT |
| | | | SEQ ID NO:3254 | SEQ ID NO:11266 | SEQ ID NO:19278 |
| **iPS:436644** | **21-225_227G9** | NA | AAGTCCAGCCAGAGTGTTTT ACACAGCTCCAACAATAACA ACTACTTAGCT | TGGGGATCTACCCGGGA ATCC | CAGCAATATTATAGTGCTCC GTACAGT |
| | | | SEQ ID NO:3255 | SEQ ID NO:11267 | SEQ ID NO:19279 |
| | | AA | KSSQSVLHSSNNNNYLA | WGSTRES | QQYYSAPYS |
| | | | SEQ ID NO:3256 | SEQ ID NO:11268 | SEQ ID NO:19280 |
| **iPS:436646** | **21-225_227D11** | NA | AAGTCCAGCCAGAGTGTTTT ACACAGTTCCAACAATAATA ACTACTTAGCT | TGGGCATCTACCCGGGA ATCC | CAGCAATATTATAATACTCC GTGCAGT |
| | | | SEQ ID NO:3257 | SEQ ID NO:11269 | SEQ ID NO:19281 |
| | | AA | KSSQSVLHSSNNNNYLA | WASTRES | QQYYNTPCS |
| | | | SEQ ID NO:3258 | SEQ ID NO:11270 | SEQ ID NO:19282 |
| **iPS:436648** | **21-225_227F11** | NA | TGGTCTAGTCAGAGCCTCCT GCATAGTAATGGATACAACT ATTTGGAT | TTGGGTTCTAATCGGGCC TCC | ATGCAAGCTCTACAAACTCC TCTCACC |
| | | | SEQ ID NO:3259 | SEQ ID NO:11271 | SEQ ID NO:19283 |
| | | AA | WSSQSLLHSNGYNYLD | LGSNRAS | MQALQTPLT |
| | | | SEQ ID NO:3260 | SEQ ID NO:11272 | SEQ ID NO:19284 |
| **iPS:436650** | **21-225_227C12** | NA | CGGGCAAGTCAGGGCATTAG AAATGATTTAGGC | GCTGCATCCAGTTTGCAA AGT | CTACAGCATAATAGTTACCC ATTCACT |
| | | | SEQ ID NO:3261 | SEQ ID NO:11273 | SEQ ID NO:19285 |

**FIGURE 49**

| | | | | | |
|---|---|---|---|---|---|
| | | AA | RASQGIRNDLG | AASSLQS | LQHNSYPFT |
| | | | SEQ ID NO:3262 | SEQ ID NO:11274 | SEQ ID NO:19286 |
| **iPS:436652** | **21-225_146B11** | NA | TCTGGAGATAAATTGGGGGA TAAATATGCTTCC | CAAGATAGCAAGCGGCC CTCA | CAGGCGTGGGACAGCAGCAC TGTGGTA |
| | | | SEQ ID NO:3263 | SEQ ID NO:11275 | SEQ ID NO:19287 |
| | | AA | SGDKLGDKYAS | QDSKRPS | QAWDSSTVV |
| | | | SEQ ID NO:3264 | SEQ ID NO:11276 | SEQ ID NO:19288 |
| **iPS:436654** | **21-225_146C11** | NA | TCTGGAGATAAATTGGGGGA TAAATATGCTTCC | CAAGATAGCAAGCGGCC CTCA | CAGGCGTGGGACAGCAGCAC TGTGGTA |
| | | | SEQ ID NO:3265 | SEQ ID NO:11277 | SEQ ID NO:19289 |
| | | AA | SGDKLGDKYAS | QDSKRPS | QAWDSSTVV |
| | | | SEQ ID NO:3266 | SEQ ID NO:11278 | SEQ ID NO:19290 |
| **iPS:436658** | **21-225_146A2** | NA | TCTGGAGATAAATTGGGGGA TAAATATGCTTCC | CAAGATAGCAAGCGGCC CTCA | CAGGCGTGGGACAGCAGCAC TGTGGTA |
| | | | SEQ ID NO:3267 | SEQ ID NO:11279 | SEQ ID NO:19291 |
| | | AA | SGDKLGDKYAS | QDSKRPS | QAWDSSTVV |
| | | | SEQ ID NO:3268 | SEQ ID NO:11280 | SEQ ID NO:19292 |
| **iPS:436660** | **21-225_146D8** | NA | TCTGGAAGCAGCTCCTACAT CGGAAGTAATACTGTAGAC | AGTAATAATCAGCGGCC CTCA | GCAGCATGGGATGACAGCCT TAATGGCGTGGTA |
| | | | SEQ ID NO:3269 | SEQ ID NO:11281 | SEQ ID NO:19293 |
| | | AA | SGSSSYIGSNTVD | SNNQRPS | AAWDDSLNGVV |
| | | | SEQ ID NO:3270 | SEQ ID NO:11282 | SEQ ID NO:19294 |
| **iPS:436662** | **21-225_147D7** | NA | TCTGGAGATAAATTGGGGGA TAAATTTGCTTGC | CAAGATAGGAAGCGGCC CTCA | CAGGCGTGGGACAGGAACAC CGCTGTC |
| | | | SEQ ID NO:3271 | SEQ ID NO:11283 | SEQ ID NO:19295 |
| | | AA | SGDKLGDKFAC | QDRKRPS | QAWDRNTAV |
| | | | SEQ ID NO:3272 | SEQ ID NO:11284 | SEQ ID NO:19296 |
| **iPS:436664** | | NA | TCTGGAGATAAATTGGGGGA TAAATATGCTTCC | CAAGATAGCAAGCGCCC CTCA | CAGGCGTGGGACAGCAGCAC TGTGGTA |

**FIGURE 49**

| | | | | | |
|---|---|---|---|---|---|
| | **21-225_147E7** | | SEQ ID NO:3273 | SEQ ID NO:11285 | SEQ ID NO:19297 |
| | | AA | SGDKLGDKYAS | QDSKRPS | QAWDSSTVV |
| | | | SEQ ID NO:3274 | SEQ ID NO:11286 | SEQ ID NO:19298 |
| **iPS:436666** | **21-225_147B8** | NA | TCTGGAGATAAATTGGGGGATAAATATGTTTGC | CAAGATAGGAAGCGGCCCTCA | CAGGCGTGGGGCAGTAACACTGCTGTGGTA |
| | | | SEQ ID NO:3275 | SEQ ID NO:11287 | SEQ ID NO:19299 |
| | | AA | SGDKLGDKYVC | QDRKRPS | QAWGSNTAVV |
| | | | SEQ ID NO:3276 | SEQ ID NO:11288 | SEQ ID NO:19300 |
| **iPS:436668** | **21-225_147B9** | NA | TCTGGAGATAAATTGGGGGATAAATATGTTTCC | CAAGATAGGAAGCGGCCCTCA | CTGGCGTGGGACAGCAGCACTTTTGTGGTA |
| | | | SEQ ID NO:3277 | SEQ ID NO:11289 | SEQ ID NO:19301 |
| | | AA | SGDKLGDKYVS | QDRKRPS | LAWDSSTFVV |
| | | | SEQ ID NO:3278 | SEQ ID NO:11290 | SEQ ID NO:19302 |
| **iPS:436670** | **21-225_147D9** | NA | TCTGGAGATAAATTGGGTAATAAATATGTTTGC | CAAGATAGCAAGCGGCCCTCA | CAGGCGTGGGACAGGAACACTTATGTGGTG |
| | | | SEQ ID NO:3279 | SEQ ID NO:11291 | SEQ ID NO:19303 |
| | | AA | SGDKLGNKYVC | QDSKRPS | QAWDRNTYVV |
| | | | SEQ ID NO:3280 | SEQ ID NO:11292 | SEQ ID NO:19304 |
| **iPS:436672** | **21-225_147F9** | NA | TCTGGAGATGAATTGGGGAATAAATATGCTTGC | CAAGATAGCAAGCGGCCCTCA | CAGGCGTGGCACAGCAGCACTGTGGTA |
| | | | SEQ ID NO:3281 | SEQ ID NO:11293 | SEQ ID NO:19305 |
| | | AA | SGDELGNKYAC | QDSKRPS | QAWHSSTVV |
| | | | SEQ ID NO:3282 | SEQ ID NO:11294 | SEQ ID NO:19306 |
| **iPS:436674** | **21-225_147G9** | NA | TCTGGAGATAAATTGGGGGATAAATATGCTTGC | CAAGATAGGAAGCGGCCCTCA | CAGGCGTGGCACAGCAGTACTGTGGTA |
| | | | SEQ ID NO:3283 | SEQ ID NO:11295 | SEQ ID NO:19307 |
| | | AA | SGDKLGDKYAC | QDRKRPS | QAWHSSTVV |
| | | | SEQ ID NO:3284 | SEQ ID NO:11296 | SEQ ID NO:19308 |
| **iPS:436676** | | NA | TCTGGAGATAAATTGGGGGATAAATATGCTTCC | CAAGATAGCAAGCGGCCCTCA | CAGGCGTGGGACAGCAGCACTGTGGTA |

FIGURE 49

| | | | | | |
|---|---|---|---|---|---|
| | **21-225_147E11** | | SEQ ID NO:3285 | SEQ ID NO:11297 | SEQ ID NO:19309 |
| | | AA | SGDKLGDKYAS | QDSKRPS | QAWDSSTVV |
| | | | SEQ ID NO:3286 | SEQ ID NO:11298 | SEQ ID NO:19310 |
| **iPS:436678** | **21-225_147B12** | NA | TCTGGAGATAAATTGGGGGATAAATATGCTTCC | CAAGATAGCAAGCGGCCCTCA | CAGGCGTGGGACAGCAGCACTGTGGTA |
| | | | SEQ ID NO:3287 | SEQ ID NO:11299 | SEQ ID NO:19311 |
| | | AA | SGDKLGDKYAS | QDSKRPS | QAWDSSTVV |
| | | | SEQ ID NO:3288 | SEQ ID NO:11300 | SEQ ID NO:19312 |
| **iPS:436680** | **21-225_147H12** | NA | TCTGGAAGCAGCTCCAACATCGGAAGTTATGCTGTAAAC | AGTAATAATCACCGGCCCTCA | GAAGCATGGGATGACAGCCTGAATGGTCCGGTA |
| | | | SEQ ID NO:3289 | SEQ ID NO:11301 | SEQ ID NO:19313 |
| | | AA | SGSSSNIGSYAVN | SNNHRPS | EAWDDSLNGPV |
| | | | SEQ ID NO:3290 | SEQ ID NO:11302 | SEQ ID NO:19314 |
| **iPS:436682** | **21-225_146A8** | NA | TCTGGAAGCAGCTCCAACATCGGAAGTAATTCTATAAAC | AGTAATGATCAGCGGCCCTCA | GCAGCATGGGATGACAGCCTGAACGGCGTGGTA |
| | | | SEQ ID NO:3291 | SEQ ID NO:11303 | SEQ ID NO:19315 |
| | | AA | SGSSSNIGSNSIN | SNDQRPS | AAWDDSLNGVV |
| | | | SEQ ID NO:3292 | SEQ ID NO:11304 | SEQ ID NO:19316 |
| **iPS:436684** | **21-225_146B6** | NA | TCTGGAAGCAGCTCCAACATCGGAAGTAATGCTGTAAAC | AGTAATAATCAGCGGCCCTCA | GCAGCATGGGATGACAGCCTGAATGGCGTGGTA |
| | | | SEQ ID NO:3293 | SEQ ID NO:11305 | SEQ ID NO:19317 |
| | | AA | SGSSSNIGSNAVN | SNNQRPS | AAWDDSLNGVV |
| | | | SEQ ID NO:3294 | SEQ ID NO:11306 | SEQ ID NO:19318 |
| **iPS:436686** | **21-225_148G6** | NA | TCTGGAGATAAATTGGGGGATAAATATGCTTCC | CAAGATAGCAAGCGGCCCTCA | CAGGCGTGGGACAGCAGCACTGTGGTA |
| | | | SEQ ID NO:3295 | SEQ ID NO:11307 | SEQ ID NO:19319 |
| | | AA | SGDKLGDKYAS | QDSKRPS | QAWDSSTVV |

FIGURE 49

| | | | | | |
|---|---|---|---|---|---|
| | | | SEQ ID NO:3296 | SEQ ID NO:11308 | SEQ ID NO:19320 |
| **iPS:436688** | **21-225_148C8** | NA | TCTGGAGATAAATTGGGGGA TAAATATGTTTCC | CAAGATAGGAAGCGGCC CTCA | CTGGCGTGGGACAGCAGCAC TTTTGTGGTA |
| | | | SEQ ID NO:3297 | SEQ ID NO:11309 | SEQ ID NO:19321 |
| | | AA | SGDKLGDKYVS | QDRKRPS | LAWDSSTFVV |
| | | | SEQ ID NO:3298 | SEQ ID NO:11310 | SEQ ID NO:19322 |
| **iPS:436690** | **21-225_148A9** | NA | TCTGGAGATAAATTGGGGAA TAAATATGTTTGC | CAAGATAGCAAGCGGCC CTCA | CAGGCGTGGCACAGCAGCAC TGTGGTA |
| | | | SEQ ID NO:3299 | SEQ ID NO:11311 | SEQ ID NO:19323 |
| | | AA | SGDKLGNKYVC | QDSKRPS | QAWHSSTVV |
| | | | SEQ ID NO:3300 | SEQ ID NO:11312 | SEQ ID NO:19324 |
| **iPS:436694** | **21-225_148G11** | NA | TCTGGAGATAAATTGGGGGA TAAATTTGCTTCC | CAAGATAGCAAGCGGCC CTCA | CAGGCGTGGGACAGCAGCAC TGTGGTA |
| | | | SEQ ID NO:3301 | SEQ ID NO:11313 | SEQ ID NO:19325 |
| | | AA | SGDKLGDKFAS | QDSKRPS | QAWDSSTVV |
| | | | SEQ ID NO:3302 | SEQ ID NO:11314 | SEQ ID NO:19326 |
| **iPS:436696** | **21-225_149A1** | NA | TCTGGAAGCAGCTCCAACAT CGGAAGTAATGCTGTAAAC | AGTAATAATCAGCGGCC CTCA | GCAGCATGGGATGACAGCCT GAATGGCGTGGTA |
| | | | SEQ ID NO:3303 | SEQ ID NO:11315 | SEQ ID NO:19327 |
| | | AA | SGSSSNIGSNAVN | SNNQRPS | AAWDDSLNGVV |
| | | | SEQ ID NO:3304 | SEQ ID NO:11316 | SEQ ID NO:19328 |
| **iPS:436698** | **21-225_149B5** | NA | TCTGGATATAAATTGGGGTA TAAATATGTTTGC | CAAAATAACCAGCGGCC CTCA | CAGGCGTGGGACAGCAGCAC TGTGGTA |
| | | | SEQ ID NO:3305 | SEQ ID NO:11317 | SEQ ID NO:19329 |
| | | AA | SGYKLGYKYVC | QNNQRPS | QAWDSSTVV |
| | | | SEQ ID NO:3306 | SEQ ID NO:11318 | SEQ ID NO:19330 |
| **iPS:436700** | **21-225_149C7** | NA | TCTGGAAATAAATTGGGGGA TAAATATGCTTCC | CAAGATAGCAAGCGGCC CTCA | CAGGCGTGGGACAGCAGCAC TGTGGTA |
| | | | SEQ ID NO:3307 | SEQ ID NO:11319 | SEQ ID NO:19331 |

FIGURE 49

| | | | | | |
|---|---|---|---|---|---|
| | | AA | SGNKLGDKYAS | QDSKRPS | QAWDSSTVV |
| | | | SEQ ID NO:3308 | SEQ ID NO:11320 | SEQ ID NO:19332 |
| **iPS:436702** | **21-225_149E8** | NA | ACCTTACGCAGTGGCATCACTGTTACTACCTATAGGATATAC | TACACATCAGACTCAGATAAACACCAGGGCTCT | ATGATTTGGCACAGCAGCGCTTGGGTG |
| | | | SEQ ID NO:3309 | SEQ ID NO:11321 | SEQ ID NO:19333 |
| | | AA | TLRSGITVTTYRIY | YTSDSDKHQGS | MIWHSSAWV |
| | | | SEQ ID NO:3310 | SEQ ID NO:11322 | SEQ ID NO:19334 |
| **iPS:436704** | **21-225_149C10** | NA | TCTGGAGATAAATTGGGGGATAAATATGCTTCC | CAAGATAACAAGCGGCCCTCA | CAGGCGTGGGACAGCAGCACTGTGGTA |
| | | | SEQ ID NO:3311 | SEQ ID NO:11323 | SEQ ID NO:19335 |
| | | AA | SGDKLGDKYAS | QDNKRPS | QAWDSSTVV |
| | | | SEQ ID NO:3312 | SEQ ID NO:11324 | SEQ ID NO:19336 |
| **iPS:436706** | **21-225_149A11** | NA | TCTGGAGATAAATTGGGGAATAAATATGTTTCC | CAAGATAGCAGGCGGCCCTCA | CTGGCGTGGGACAGCAGCACTTTTGTGGTC |
| | | | SEQ ID NO:3313 | SEQ ID NO:11325 | SEQ ID NO:19337 |
| | | AA | SGDKLGNKYVS | QDSRRPS | LAWDSSTFVV |
| | | | SEQ ID NO:3314 | SEQ ID NO:11326 | SEQ ID NO:19338 |
| **iPS:436708** | **21-225_150D3** | NA | TCTGGAGATGAATTGGGGAATAAATATGCTTGC | CAAGATAACAAGCGGCCCTCA | CAGGCGTGGCACAGCAGCACTGTGGTA |
| | | | SEQ ID NO:3315 | SEQ ID NO:11327 | SEQ ID NO:19339 |
| | | AA | SGDELGNKYAC | QDNKRPS | QAWHSSTVV |
| | | | SEQ ID NO:3316 | SEQ ID NO:11328 | SEQ ID NO:19340 |
| **iPS:436710** | **21-225_150F6** | NA | TCTGGAGATAAATTGGGGGATAAATATGCTTCC | CAAGATAGCAAGCGGCCCTCA | CAGGCGTGGGACAGCAGCACTGTGGTA |
| | | | SEQ ID NO:3317 | SEQ ID NO:11329 | SEQ ID NO:19341 |
| | | AA | SGDKLGDKYAS | QDSKRPS | QAWDSSTVV |
| | | | SEQ ID NO:3318 | SEQ ID NO:11330 | SEQ ID NO:19342 |

FIGURE 49

| | | | | | |
|---|---|---|---|---|---|
| **iPS:436712** | **21-225_150F9** | NA | TCTGGAAGCAGCTCCAACAT CGGAAGTAATGCTGTAAAC | AGTAATAGTCAGCGGCC CTCA | GCAGCATGGGATGACAGCCT GAATGGCGTGGTA |
| | | | SEQ ID NO:3319 | SEQ ID NO:11331 | SEQ ID NO:19343 |
| | | AA | SGSSSNIGSNAVN | SNSQRPS | AAWDDSLNGVV |
| | | | SEQ ID NO:3320 | SEQ ID NO:11332 | SEQ ID NO:19344 |
| **iPS:436714** | **21-225_150H11** | NA | TCTGGAGATAAATTGGGGGA TAAATATGCTTCC | CAAGATAGCAAGCGGCC CTCA | CAGGCGTGGGACAGCAGCAC TGTGGTA |
| | | | SEQ ID NO:3321 | SEQ ID NO:11333 | SEQ ID NO:19345 |
| | | AA | SGDKLGDKYAS | QDSKRPS | QAWDSSTVV |
| | | | SEQ ID NO:3322 | SEQ ID NO:11334 | SEQ ID NO:19346 |
| **iPS:436716** | **21-225_151F3** | NA | TCTGGAGATAAATTGGGGGA TAAATATGTTTGC | CAAGATCGCAAGCGGCC CTCA | CAGGCGTGGCACAGCAGCAC TGTGGTA |
| | | | SEQ ID NO:3323 | SEQ ID NO:11335 | SEQ ID NO:19347 |
| | | AA | SGDKLGDKYVC | QDRKRPS | QAWHSSTVV |
| | | | SEQ ID NO:3324 | SEQ ID NO:11336 | SEQ ID NO:19348 |
| **iPS:436718** | **21-225_151H5** | NA | TCTGGAGATAAATTGGGGGA TAAATATGCTTCC | CAAGATAGCAAGCGGCC CTCA | CAGGCGTGGGACAGCAGCAC TGTGGTA |
| | | | SEQ ID NO:3325 | SEQ ID NO:11337 | SEQ ID NO:19349 |
| | | AA | SGDKLGDKYAS | QDSKRPS | QAWDSSTVV |
| | | | SEQ ID NO:3326 | SEQ ID NO:11338 | SEQ ID NO:19350 |
| **iPS:436720** | **21-225_151H6** | NA | TCTGGAGATAAATTGGGGGA TAAATATGCTTGC | CAAGATACCAAGCGGCC CTCA | CAGGCGTGGGACAGCAGCAC TTATGTC |
| | | | SEQ ID NO:3327 | SEQ ID NO:11339 | SEQ ID NO:19351 |
| | | AA | SGDKLGDKYAC | QDTKRPS | QAWDSSTYV |
| | | | SEQ ID NO:3328 | SEQ ID NO:11340 | SEQ ID NO:19352 |
| **iPS:436722** | **21-225_151H7** | NA | TCTGGAGATAAATTGGGGGA TAAATATGCTTCC | CAAGATAGCAAGCGGCC CTCA | CAGGCGTGGGACAGCAGCAC TGTGGTA |
| | | | SEQ ID NO:3329 | SEQ ID NO:11341 | SEQ ID NO:19353 |
| | | AA | SGDKLGDKYAS | QDSKRPS | QAWDSSTVV |

FIGURE 49

| | | | | | |
|---|---|---|---|---|---|
| | | | SEQ ID NO:3330 | SEQ ID NO:11342 | SEQ ID NO:19354 |
| **iPS:436724** | **21-225_151B9** | NA | TCTGGAGATAATTTGGGGGATAAATATGCTTCC | CAAGATAGCAAGCGGCCCTCA | CAGGCGTGGGACAGCAGCACTGTGGTA |
| | | | SEQ ID NO:3331 | SEQ ID NO:11343 | SEQ ID NO:19355 |
| | | AA | SGDNLGDKYAS | QDSKRPS | QAWDSSTVV |
| | | | SEQ ID NO:3332 | SEQ ID NO:11344 | SEQ ID NO:19356 |
| **iPS:436726** | **21-225_152G5** | NA | TCTGGAGATAAATTGGGGGATAAATATGCTTGC | CAAGATTCCAAGCGGCCCTCA | CAGGCGTGGGACAGCAGCACTTATGTC |
| | | | SEQ ID NO:3333 | SEQ ID NO:11345 | SEQ ID NO:19357 |
| | | AA | SGDKLGDKYAC | QDSKRPS | QAWDSSTYV |
| | | | SEQ ID NO:3334 | SEQ ID NO:11346 | SEQ ID NO:19358 |
| **iPS:436728** | **21-225_152G6** | NA | TCTGGAGATAAATTGGGGGATAAATATGCTTCC | CAAGATAGCAAGCGGCCCTCA | CAGGCGTGGGACAACAGCACTGTGGTA |
| | | | SEQ ID NO:3335 | SEQ ID NO:11347 | SEQ ID NO:19359 |
| | | AA | SGDKLGDKYAS | QDSKRPS | QAWDNSTVV |
| | | | SEQ ID NO:3336 | SEQ ID NO:11348 | SEQ ID NO:19360 |
| **iPS:436730** | **21-225_152D7** | NA | TCTGGAGATAAATTGGGGGATAAATATGCTTCC | CAAGATAGCAAGCGGCCCTCA | CAGGCGTGGGACAGCAGCACTGTGGTA |
| | | | SEQ ID NO:3337 | SEQ ID NO:11349 | SEQ ID NO:19361 |
| | | AA | SGDKLGDKYAS | QDSKRPS | QAWDSSTVV |
| | | | SEQ ID NO:3338 | SEQ ID NO:11350 | SEQ ID NO:19362 |
| **iPS:436732** | **21-225_152B12** | NA | TCTGGCGATAAATTGGGAAATAAATATGCTTGC | CAAGATACCAAGCGGCCCTCA | CAGGCGTGGGACAGCAGCACTTATGTC |
| | | | SEQ ID NO:3339 | SEQ ID NO:11351 | SEQ ID NO:19363 |
| | | AA | SGDKLGNKYAC | QDTKRPS | QAWDSSTYV |
| | | | SEQ ID NO:3340 | SEQ ID NO:11352 | SEQ ID NO:19364 |
| **iPS:436734** | **21-225_153A8** | NA | TCTGGAGATAAATTGGGGGATAAATATGCTTGC | CAAGATACCAAGCGGCCCTCA | CAGGCGTGGGACAGCAGCACTTATGTC |
| | | | SEQ ID NO:3341 | SEQ ID NO:11353 | SEQ ID NO:19365 |
| | | AA | SGDKLGDKYAC | QDTKRPS | QAWDSSTYV |

FIGURE 49

| | | | | | |
|---|---|---|---|---|---|
| | | | SEQ ID NO:3342 | SEQ ID NO:11354 | SEQ ID NO:19366 |
| **iPS:436736** | **21-225_153E8** | NA | TCTGGAAGTAAATTGGGTAA TAAATATGTTTGC | CAAGATAACAAGCGGCC CTCA | CAGGCGTGGGACAGCAGCAC TTATGTGATA |
| | | | SEQ ID NO:3343 | SEQ ID NO:11355 | SEQ ID NO:19367 |
| | | AA | SGSKLGNKYVC | QDNKRPS | QAWDSSTYVI |
| | | | SEQ ID NO:3344 | SEQ ID NO:11356 | SEQ ID NO:19368 |
| **iPS:436738** | **21-225_153D9** | NA | TCTGGAGATAAATTGGGGGA TAAATATGCTTGC | CAAGATAGGAAGCGGCC CTCA | CAGGCGTGGCACAGCAGTAC TGTGGTA |
| | | | SEQ ID NO:3345 | SEQ ID NO:11357 | SEQ ID NO:19369 |
| | | AA | SGDKLGDKYAC | QDRKRPS | QAWHSSTVV |
| | | | SEQ ID NO:3346 | SEQ ID NO:11358 | SEQ ID NO:19370 |
| **iPS:436740** | **21-225_154C3** | NA | TCTGGAGATAAATTGGGGGA TAAATATGTTTGC | CAAGATAGGAAGCGGCC CTCA | CAGGCGTGGCACAGCAGCAC TGTGGTA |
| | | | SEQ ID NO:3347 | SEQ ID NO:11359 | SEQ ID NO:19371 |
| | | AA | SGDKLGDKYVC | QDRKRPS | QAWHSSTVV |
| | | | SEQ ID NO:3348 | SEQ ID NO:11360 | SEQ ID NO:19372 |
| **iPS:436742** | **21-225_154C4** | NA | TCTGGAGATAAATTGGGGGA TAAATATGCTTCC | CAAGATAGCAAGCGGCC CTCA | CAGGCGTGGGACAGCAGCAC TGTGGTA |
| | | | SEQ ID NO:3349 | SEQ ID NO:11361 | SEQ ID NO:19373 |
| | | AA | SGDKLGDKYAS | QDSKRPS | QAWDSSTVV |
| | | | SEQ ID NO:3350 | SEQ ID NO:11362 | SEQ ID NO:19374 |
| **iPS:436744** | **21-225_154F4** | NA | TCTGGAGATAAATTGGGAAA TAAATATGTTTGT | AAAGATAGTAAGCGGCC CTCA | CAGGCGTGGGACAACAGTAC TTTAGTA |
| | | | SEQ ID NO:3351 | SEQ ID NO:11363 | SEQ ID NO:19375 |
| | | AA | SGDKLGNKYVC | KDSKRPS | QAWDNSTLV |
| | | | SEQ ID NO:3352 | SEQ ID NO:11364 | SEQ ID NO:19376 |
| **iPS:436746** | **21-225_154E10** | NA | TCTGGAGATAAATTGGGGGA TAAATATGCTTCC | CAAGATAGCAAGCGGCC CTCA | CAGGCGTGGGACAACAGCAC TGTGGTA |
| | | | SEQ ID NO:3353 | SEQ ID NO:11365 | SEQ ID NO:19377 |
| | | AA | SGDKLGDKYAS | QDSKRPS | QAWDNSTVV |

**FIGURE 49**

| | | | | | |
|---|---|---|---|---|---|
| | | | SEQ ID NO:3354 | SEQ ID NO:11366 | SEQ ID NO:19378 |
| **iPS:436748** | **21-225_154D11** | NA | TCTGGAGATAAATTGGGGGATAAATATGCTTGC | CAAGATAAGAAGCGGCCCTCA | CAGGCGTGGCACAGCAGTATTGTGGTA |
| | | | SEQ ID NO:3355 | SEQ ID NO:11367 | SEQ ID NO:19379 |
| | | AA | SGDKLGDKYAC | QDKKRPS | QAWHSSIVV |
| | | | SEQ ID NO:3356 | SEQ ID NO:11368 | SEQ ID NO:19380 |
| **iPS:436750** | **21-225_154G12** | NA | TCTGGAAGCAGCTCCAACATCGGAAATAATGCTGTAAGC | AGTAATGATCACCGGCCCTCA | GCAGCATGGGATGACAGCCTGAAGGGTCCGGTA |
| | | | SEQ ID NO:3357 | SEQ ID NO:11369 | SEQ ID NO:19381 |
| | | AA | SGSSSNIGNNAVS | SNDHRPS | AAWDDSLKGPV |
| | | | SEQ ID NO:3358 | SEQ ID NO:11370 | SEQ ID NO:19382 |
| **iPS:436752** | **21-225_155H1** | NA | ACTGGGAGCAGCTCCAATATCGGGGCAGGTTATGATGTACAC | GGTAACAGCAATCGGCCCTCA | CAGTCCTATGACAGCAGCCTGAGTGGTCCTGTGATA |
| | | | SEQ ID NO:3359 | SEQ ID NO:11371 | SEQ ID NO:19383 |
| | | AA | TGSSSNIGAGYDVH | GNSNRPS | QSYDSSLSGPVI |
| | | | SEQ ID NO:3360 | SEQ ID NO:11372 | SEQ ID NO:19384 |
| **iPS:436754** | **21-225_155G3** | NA | TCTGGAGATAAGTTGGGGGATAAATATGTTTGC | CAAGATAGTAAGCGGCCCTCA | CAGGCGTGGGACAATAGTATTTATGTC |
| | | | SEQ ID NO:3361 | SEQ ID NO:11373 | SEQ ID NO:19385 |
| | | AA | SGDKLGDKYVC | QDSKRPS | QAWDNSIYV |
| | | | SEQ ID NO:3362 | SEQ ID NO:11374 | SEQ ID NO:19386 |
| **iPS:436756** | **21-225_146A10** | NA | TCTGGAGATAAATTGGGGGATAAATATGTTTGC | CAAGATAGAAAGCGGCCCTCA | CAGGCGTGGGACAGCAGCACTGTGGTG |
| | | | SEQ ID NO:3363 | SEQ ID NO:11375 | SEQ ID NO:19387 |
| | | AA | SGDKLGDKYVC | QDRKRPS | QAWDSSTVV |
| | | | SEQ ID NO:3364 | SEQ ID NO:11376 | SEQ ID NO:19388 |
| **iPS:436758** | | NA | TCTGGAGATAAATTGGGGGATAAATATGTTTCC | CAAGATAGCAAGCGGCCCTCA | CAGGCGTGGGACAGCAGCACTGTGGTA |

FIGURE 49

| | | | | | |
|---|---|---|---|---|---|
| | **21-225_155C10** | | SEQ ID NO:3365 | SEQ ID NO:11377 | SEQ ID NO:19389 |
| | | AA | SGDKLGDKYVS | QDSKRPS | QAWDSSTVV |
| | | | SEQ ID NO:3366 | SEQ ID NO:11378 | SEQ ID NO:19390 |
| **iPS:436760** | **21-225_155E10** | NA | TCTGGAGATAAATTGGGGGA TAAATATGTTTCC | CAAGATAGGAAGCGGCC CTCA | CTGGCGTGGGACAGCAGCAC TTTTGTGGTA |
| | | | SEQ ID NO:3367 | SEQ ID NO:11379 | SEQ ID NO:19391 |
| | | AA | SGDKLGDKYVS | QDRKRPS | LAWDSSTFVV |
| | | | SEQ ID NO:3368 | SEQ ID NO:11380 | SEQ ID NO:19392 |
| **iPS:436762** | **21-225_156H2** | NA | TCTGGAAGCAGCTCCAACAT CGGAAGTAATACTGTAAAT | AGTAGTAATCAGCGGCC CTCA | GCAGCATGGGATGACAGCCT GAATGGCGTGGTA |
| | | | SEQ ID NO:3369 | SEQ ID NO:11381 | SEQ ID NO:19393 |
| | | AA | SGSSSNIGSNTVN | SSNQRPS | AAWDDSLNGVV |
| | | | SEQ ID NO:3370 | SEQ ID NO:11382 | SEQ ID NO:19394 |
| **iPS:436764** | **21-225_158E9** | NA | TCTGGAGATAAATTGGGGGA TAAATATGTTTGC | CAAGATAGTAAGCGGCC CTCA | CAGGCGTGGGACAACAGCAG CTTTGTGCTA |
| | | | SEQ ID NO:3371 | SEQ ID NO:11383 | SEQ ID NO:19395 |
| | | AA | SGDKLGDKYVC | QDSKRPS | QAWDNSSFVL |
| | | | SEQ ID NO:3372 | SEQ ID NO:11384 | SEQ ID NO:19396 |
| **iPS:436766** | **21-225_158D10** | NA | TCTGGAGATAAATTGGGGGA TAAATATGTTTGC | CAAGATCGCAAGCGGCC CTCA | CAGGCGTGGGGCAACAGCAG CTTTGTGGTA |
| | | | SEQ ID NO:3373 | SEQ ID NO:11385 | SEQ ID NO:19397 |
| | | AA | SGDKLGDKYVC | QDRKRPS | QAWGNSSFVV |
| | | | SEQ ID NO:3374 | SEQ ID NO:11386 | SEQ ID NO:19398 |
| **iPS:436768** | **21-225_159H8** | NA | TCTGGAGATAAATTGGGGGA TAAATATGTTTGC | CAAGATAGGAAGCGGCC CTCA | CAGGCGTGGGGCAACAGCAG CTTTGTGGTA |
| | | | SEQ ID NO:3375 | SEQ ID NO:11387 | SEQ ID NO:19399 |
| | | AA | SGDKLGDKYVC | QDRKRPS | QAWGNSSFVV |
| | | | SEQ ID NO:3376 | SEQ ID NO:11388 | SEQ ID NO:19400 |

**FIGURE 49**

| | | | | | |
|---|---|---|---|---|---|
| **iPS:436770** | **21-225_160B12** | NA | TCTGGAGATAAATTGGGGGA TAAATATGTTTGC | CAAGATAGCAAGCGGCC CTCA | CAGGCGTGGGGCAACAGCAG CTTTGTGGTA |
| | | | SEQ ID NO:3377 | SEQ ID NO:11389 | SEQ ID NO:19401 |
| | | AA | SGDKLGDKYVC | QDSKRPS | QAWGNSSFVV |
| | | | SEQ ID NO:3378 | SEQ ID NO:11390 | SEQ ID NO:19402 |
| **iPS:436772** | **21-225_161H3** | NA | TCTGGAGATAGATTGGGGGA TAAATATGTTTGC | CAAGATAACAAGCGGCC CTCA | CAGGCGTGGGTCAACAACAC TGCAGTGGTT |
| | | | SEQ ID NO:3379 | SEQ ID NO:11391 | SEQ ID NO:19403 |
| | | AA | SGDRLGDKYVC | QDNKRPS | QAWVNNTAVV |
| | | | SEQ ID NO:3380 | SEQ ID NO:11392 | SEQ ID NO:19404 |
| **iPS:436774** | **21-225_161E10** | NA | TCTGGAGATAAATTGGGGGA TAAATATGTTTGC | CAAGATAGCAAGCGGCC CTCA | CAGACGTGGGACAACAGTAG TTTTGCGCTT |
| | | | SEQ ID NO:3381 | SEQ ID NO:11393 | SEQ ID NO:19405 |
| | | AA | SGDKLGDKYVC | QDSKRPS | QTWDNSSFAL |
| | | | SEQ ID NO:3382 | SEQ ID NO:11394 | SEQ ID NO:19406 |
| **iPS:436776** | **21-225_161F12** | NA | TCTGGAGATAAATTGGGTGA TAAATATGCTTGC | CAAGATACCAAGCGGCC CTCA | CAGGCGTGGGACAGCACCAC TCTGGTT |
| | | | SEQ ID NO:3383 | SEQ ID NO:11395 | SEQ ID NO:19407 |
| | | AA | SGDKLGDKYAC | QDTKRPS | QAWDSTTLV |
| | | | SEQ ID NO:3384 | SEQ ID NO:11396 | SEQ ID NO:19408 |
| **iPS:436780** | **21-225_165H3** | NA | TCTGGAGATAAATTGGGTGA TAAATATGCTTGC | CAAGATAGCAAGCGGCC CTCA | CAGGCGTGGGACAGCACCAC TCTGGTT |
| | | | SEQ ID NO:3385 | SEQ ID NO:11397 | SEQ ID NO:19409 |
| | | AA | SGDKLGDKYAC | QDSKRPS | QAWDSTTLV |
| | | | SEQ ID NO:3386 | SEQ ID NO:11398 | SEQ ID NO:19410 |
| **iPS:436782** | **21-225_166G11** | NA | TCTGGAGATAAATTGGGGGA TAAATATGTTCAC | CAAGATAGCAAGCGGCC CTCA | CAGGCGTGGGACAACAGCAC TGCGGTA |
| | | | SEQ ID NO:3387 | SEQ ID NO:11399 | SEQ ID NO:19411 |
| | | AA | SGDKLGDKYVH | QDSKRPS | QAWDNSTAV |
| | | | SEQ ID NO:3388 | SEQ ID NO:11400 | SEQ ID NO:19412 |

FIGURE 49

| | | | | | |
|---|---|---|---|---|---|
| **iPS:436784** | **21-225_169C1** | NA | TCTGGAGATAAATTGGGGGA TAAATATGTTTGT | AAAGATATCAAGCGGCC CTCA | CAGGCGTGGGACACCAACAC TGTGATA |
| | | | SEQ ID NO:3389 | SEQ ID NO:11401 | SEQ ID NO:19413 |
| | | AA | SGDKLGDKYVC | KDIKRPS | QAWDTNTVI |
| | | | SEQ ID NO:3390 | SEQ ID NO:11402 | SEQ ID NO:19414 |
| **iPS:436786** | **21-225_169A6** | NA | TCTGGAGATAAATTGGGGGA TAAATATGTTTGT | CAGGATTACAAGCGGCC CTCA | CAGGCGTGGGACACCAACAC TGTGCTT |
| | | | SEQ ID NO:3391 | SEQ ID NO:11403 | SEQ ID NO:19415 |
| | | AA | SGDKLGDKYVC | QDYKRPS | QAWDTNTVL |
| | | | SEQ ID NO:3392 | SEQ ID NO:11404 | SEQ ID NO:19416 |
| **iPS:436788** | **21-225_169B7** | NA | TCTGGAGATAAATTGGGGGG AAAATATGCTTCC | CAAGATAGGAAGCGGCC CTCA | CAGGCGTGGGACAAGAACAC TGTGGTA |
| | | | SEQ ID NO:3393 | SEQ ID NO:11405 | SEQ ID NO:19417 |
| | | AA | SGDKLGGKYAS | QDRKRPS | QAWDKNTVV |
| | | | SEQ ID NO:3394 | SEQ ID NO:11406 | SEQ ID NO:19418 |
| **iPS:436790** | **21-225_169G11** | NA | TCTGGAGATAAATTGGGGGA TAAATATGCTTGC | CAAGATAGTAAGCGGCC CTCA | CAGGCGTGGGACAACAGCAC TGCGGTA |
| | | | SEQ ID NO:3395 | SEQ ID NO:11407 | SEQ ID NO:19419 |
| | | AA | SGDKLGDKYAC | QDSKRPS | QAWDNSTAV |
| | | | SEQ ID NO:3396 | SEQ ID NO:11408 | SEQ ID NO:19420 |
| **iPS:436792** | **21-225_169D12** | NA | ACCCGCAGCAGTGGCAGCAT TACCGGCAACTATGTGCAG | GAGGATAAAAAAAGACC CTCT | CAGTCTTATTATAGCGGCAA TTGGGTG |
| | | | SEQ ID NO:3397 | SEQ ID NO:11409 | SEQ ID NO:19421 |
| | | AA | TRSSGSITGNYVQ | EDKKRPS | QSYYSGNWV |
| | | | SEQ ID NO:3398 | SEQ ID NO:11410 | SEQ ID NO:19422 |
| **iPS:436794** | **21-225_170F1** | NA | TCTGGAGATAAATTGGGGGA TAAATATTCTTGC | CAAGATAGTAAGCGGCC CTCA | CAGGCGTGGGACAGCAACAC TGCGGTA |
| | | | SEQ ID NO:3399 | SEQ ID NO:11411 | SEQ ID NO:19423 |
| | | AA | SGDKLGDKYSC | QDSKRPS | QAWDSNTAV |

**FIGURE 49**

| | | | | | |
|---|---|---|---|---|---|
| | | | SEQ ID NO:3400 | SEQ ID NO:11412 | SEQ ID NO:19424 |
| **iPS:436796** | **21-225_170A5** | NA | TCTGGAGATAAATTGGGGGATAAATATGCTTGT | CAAGATTACAAGCGGCCCTCA | CAGGCGTGGGACAACAGCACTATGGTA |
| | | | SEQ ID NO:3401 | SEQ ID NO:11413 | SEQ ID NO:19425 |
| | | AA | SGDKLGDKYAC | QDYKRPS | QAWDNSTMV |
| | | | SEQ ID NO:3402 | SEQ ID NO:11414 | SEQ ID NO:19426 |
| **iPS:436798** | **21-225_171F5** | NA | TCTGGAGATAAATTGGGGGGAAAATATGCTTCC | CAAGATAGGAAGCGGCCCTCA | CAGGCGTGGGACAAGAACACTGTGGTA |
| | | | SEQ ID NO:3403 | SEQ ID NO:11415 | SEQ ID NO:19427 |
| | | AA | SGDKLGGKYAS | QDRKRPS | QAWDKNTVV |
| | | | SEQ ID NO:3404 | SEQ ID NO:11416 | SEQ ID NO:19428 |
| **iPS:436800** | **21-225_171D12** | NA | CAAGGAGACAGCCTCAGAAGCTATTATGCAAGC | GCTAAAAACAACCGGCCCTCA | AACTCCCGGGACAGCAGTGGCAGCCATGTGGTA |
| | | | SEQ ID NO:3405 | SEQ ID NO:11417 | SEQ ID NO:19429 |
| | | AA | QGDSLRSYYAS | AKNNRPS | NSRDSSGSHVV |
| | | | SEQ ID NO:3406 | SEQ ID NO:11418 | SEQ ID NO:19430 |
| **iPS:436802** | **21-225_171E12** | NA | TCTGGAGATAAATTGGGGGATAAATATGCTTGC | CAAGATCGCAAGCGGCCCTCA | CAGGCGTGGGACATCAGCACTTATGTGGTA |
| | | | SEQ ID NO:3407 | SEQ ID NO:11419 | SEQ ID NO:19431 |
| | | AA | SGDKLGDKYAC | QDRKRPS | QAWDISTYVV |
| | | | SEQ ID NO:3408 | SEQ ID NO:11420 | SEQ ID NO:19432 |
| **iPS:436804** | **21-225_172C3** | NA | CAAGGAGACAGCCTCAGAAACTATTATGTAAGC | ACTAAAAACAGCCGGCCCTCA | AACTCCCGGGACAGCAGTGGCAACCATGTGGTA |
| | | | SEQ ID NO:3409 | SEQ ID NO:11421 | SEQ ID NO:19433 |
| | | AA | QGDSLRNYYVS | TKNSRPS | NSRDSSGNHVV |
| | | | SEQ ID NO:3410 | SEQ ID NO:11422 | SEQ ID NO:19434 |
| **iPS:436806** | **21-225_172B12** | NA | CAAGGAGACAGCCTCAGAAACTATTATGCAAGC | ACTAAAAACAGCCGGCCCTCA | AACTCCCGGGACAGCAGTGGCAACCATGTGGTA |
| | | | SEQ ID NO:3411 | SEQ ID NO:11423 | SEQ ID NO:19435 |
| | | AA | QGDSLRNYYAS | TKNSRPS | NSRDSSGNHVV |

FIGURE 49

| | | | | | |
|---|---|---|---|---|---|
| | | | SEQ ID NO:3412 | SEQ ID NO:11424 | SEQ ID NO:19436 |
| **iPS:436808** | **21-225_173F8** | NA | TCTGGAAATAAATTGGGGAA TAAATATGTTTGC | CAAGATAGCAGGCGGCC CTCA | CAGGCGTGGGACAGCTTCAC TGTGGTA |
| | | | SEQ ID NO:3413 | SEQ ID NO:11425 | SEQ ID NO:19437 |
| | | AA | SGNKLGNKYVC | QDSRRPS | QAWDSFTVV |
| | | | SEQ ID NO:3414 | SEQ ID NO:11426 | SEQ ID NO:19438 |
| **iPS:436810** | **21-225_175F4** | NA | ACTGGAACCAGCAGTGATGT TGGACGTTTTAACCTTGTCT CC | GAGGTCAGTAAGCGGCC CTCA | TGCTCATATGCAGGTAGTAG CACCTATGTGGTA |
| | | | SEQ ID NO:3415 | SEQ ID NO:11427 | SEQ ID NO:19439 |
| | | AA | TGTSSDVGRFNLVS | EVSKRPS | CSYAGSSTYVV |
| | | | SEQ ID NO:3416 | SEQ ID NO:11428 | SEQ ID NO:19440 |
| **iPS:436812** | **21-225_175C6** | NA | TCTGGAGATAAATTGGGGGA TAAATATGCTTGT | CAAGATTACAAGCGGCC CTCA | CAGGCGTGGGACAACAGCAC TATGGTA |
| | | | SEQ ID NO:3417 | SEQ ID NO:11429 | SEQ ID NO:19441 |
| | | AA | SGDKLGDKYAC | QDYKRPS | QAWDNSTMV |
| | | | SEQ ID NO:3418 | SEQ ID NO:11430 | SEQ ID NO:19442 |
| **iPS:436814** | **21-225_178H10** | NA | ACTGGAACCAGCAGTGATGT TGGACGTTTTAACCTTGTCT CC | GAAGTCAGTAAGCGGCC CTCA | TGCTCATATGCAGGTAGTAG CACCTTTGTAGTA |
| | | | SEQ ID NO:3419 | SEQ ID NO:11431 | SEQ ID NO:19443 |
| | | AA | TGTSSDVGRFNLVS | EVSKRPS | CSYAGSSTFVV |
| | | | SEQ ID NO:3420 | SEQ ID NO:11432 | SEQ ID NO:19444 |
| **iPS:436816** | **21-225_179H5** | NA | CAAGGAGACAGTCTCAGAA ACTATTATGCAAGC | GGTAAAAACAACCGGCC CTCA | AACTCCCGGGACAGCAGTGG TAACCATTGGGTG |
| | | | SEQ ID NO:3421 | SEQ ID NO:11433 | SEQ ID NO:19445 |
| | | AA | QGDSLRNYYAS | GKNNRPS | NSRDSSGNHWV |
| | | | SEQ ID NO:3422 | SEQ ID NO:11434 | SEQ ID NO:19446 |
| **iPS:436818** | | NA | TCTGGAGATAAATTGGGGGA TAAATATGTTTGC | CAGGATAGTAAGCGGCC CTCA | CAGGCGTGGGACAGCAACAC TGCAGTGGTA |

**FIGURE 49**

| | | | | | |
|---|---|---|---|---|---|
| | **21-225_179C7** | | SEQ ID NO:3423 | SEQ ID NO:11435 | SEQ ID NO:19447 |
| | | AA | SGDKLGDKYVC | QDSKRPS | QAWDSNTAVV |
| | | | SEQ ID NO:3424 | SEQ ID NO:11436 | SEQ ID NO:19448 |
| **iPS:436820** | **21-225_179D10** | NA | ACTGGGAGCAGCTCCAACTTCGGGACAGATTATGATGTACAC | GGTCACAGCAACCGGCCCTCA | CAGTCCTATGATAGAAGCCTGAATGTGGTC |
| | | | SEQ ID NO:3425 | SEQ ID NO:11437 | SEQ ID NO:19449 |
| | | AA | TGSSSNFGTDYDVH | GHSNRPS | QSYDRSLNVV |
| | | | SEQ ID NO:3426 | SEQ ID NO:11438 | SEQ ID NO:19450 |
| **iPS:436822** | **21-225_180D4** | NA | TCTGGAGATAGATTGGGGGATAAATATGCTTGC | GAAGATAGGAAGCGGCCCTCA | CAGGCGTGGGACAGTAGGAAAGTGGTA |
| | | | SEQ ID NO:3427 | SEQ ID NO:11439 | SEQ ID NO:19451 |
| | | AA | SGDRLGDKYAC | EDRKRPS | QAWDSRKVV |
| | | | SEQ ID NO:3428 | SEQ ID NO:11440 | SEQ ID NO:19452 |
| **iPS:436824** | **21-225_180C5** | NA | TCTGGAGATAAATTGGGGGAAAAATATGCTTGC | CAAGATAGAAAGCGGCCCTCA | CAGGCGTGGGACAGCAGCACTGCGGTA |
| | | | SEQ ID NO:3429 | SEQ ID NO:11441 | SEQ ID NO:19453 |
| | | AA | SGDKLGEKYAC | QDRKRPS | QAWDSSTAV |
| | | | SEQ ID NO:3430 | SEQ ID NO:11442 | SEQ ID NO:19454 |
| **iPS:436826** | **21-225_180G5** | NA | TCTGGAGATAAATTGGGGGATAAATATGTTAGC | CAAGATAGCAAGCGGCCCTCA | CAGGCGTGGGACATCACCACTGCGGTA |
| | | | SEQ ID NO:3431 | SEQ ID NO:11443 | SEQ ID NO:19455 |
| | | AA | SGDKLGDKYVS | QDSKRPS | QAWDITTAV |
| | | | SEQ ID NO:3432 | SEQ ID NO:11444 | SEQ ID NO:19456 |
| **iPS:436828** | **21-225_181H1** | NA | TCTGGAGATAAATTGGGGGATAAATATGCTTGC | GAAGATAGGAAGCGGCCCTCA | CAGGCGTGGGACAGCAGGAAAGTGGTA |
| | | | SEQ ID NO:3433 | SEQ ID NO:11445 | SEQ ID NO:19457 |
| | | AA | SGDKLGDKYAC | EDRKRPS | QAWDSRKVV |
| | | | SEQ ID NO:3434 | SEQ ID NO:11446 | SEQ ID NO:19458 |

## FIGURE 49

| | | | | | |
|---|---|---|---|---|---|
| **iPS:436830** | **21-225_51F4** | NA | TCTGGAAGCAGCTCCAACATCGGAAGTAATATTGTGACC | AGTAATGATCAGCGGCCCTCA | ACAGCATGGGATGACAGCCTGAATGGTTGGGTG |
| | | | SEQ ID NO:3435 | SEQ ID NO:11447 | SEQ ID NO:19459 |
| | | AA | SGSSSNIGSNIVT | SNDQRPS | TAWDDSLNGWV |
| | | | SEQ ID NO:3436 | SEQ ID NO:11448 | SEQ ID NO:19460 |
| **iPS:436832** | **21-225_51D8** | NA | ACTGGGAGCAGCTCCAACATCGGGGCAGGTTTTGAAGTACAC | GGTAACAGCAATCGGCCCTCA | CAGTCCTATGACAGCAGCCTGAGTGGTTATGTC |
| | | | SEQ ID NO:3437 | SEQ ID NO:11449 | SEQ ID NO:19461 |
| | | AA | TGSSSNIGAGFEVH | GNSNRPS | QSYDSSLSGYV |
| | | | SEQ ID NO:3438 | SEQ ID NO:11450 | SEQ ID NO:19462 |
| **iPS:436834** | **21-225_52F1** | NA | TCTGGAAGCAGCTCCAACATCGGAAGTAATATTGTGACC | AGTAATGATCAGCGGCCCTCA | GCAGCATGGGATGACAGCCTGAATGGTTGGGTG |
| | | | SEQ ID NO:3439 | SEQ ID NO:11451 | SEQ ID NO:19463 |
| | | AA | SGSSSNIGSNIVT | SNDQRPS | AAWDDSLNGWV |
| | | | SEQ ID NO:3440 | SEQ ID NO:11452 | SEQ ID NO:19464 |
| **iPS:436836** | **21-225_52H1** | NA | TCTGGAGATAAATTGGGGGATAAATATGTTTCC | CAAGATAGAAAGCGGCCCTCA | CAGGCGTGGGACAACAGCACTGTGGTA |
| | | | SEQ ID NO:3441 | SEQ ID NO:11453 | SEQ ID NO:19465 |
| | | AA | SGDKLGDKYVS | QDRKRPS | QAWDNSTVV |
| | | | SEQ ID NO:3442 | SEQ ID NO:11454 | SEQ ID NO:19466 |
| **iPS:436838** | **21-225_52H4** | NA | ACTGGAACCAGCAGTGACGTTGGTGGTTATAACTATGTCTCC | GAGGTCAGTAATCGGCCCTCA | AACTCATATACAAGCAACATCACTTGGGTG |
| | | | SEQ ID NO:3443 | SEQ ID NO:11455 | SEQ ID NO:19467 |
| | | AA | TGTSSDVGGYNYVS | EVSNRPS | NSYTSNITWV |
| | | | SEQ ID NO:3444 | SEQ ID NO:11456 | SEQ ID NO:19468 |

## FIGURE 49

| | | | | | |
|---|---|---|---|---|---|
| **iPS:436840** | **21-225_53E9** | NA | TCTGGAACTAAATTGGGGGATAAATATGTTTGC | CAAGATACAATGCGGCCCTCA | CAGACGTGGGACAGCAGCACTGCGGTT |
| | | | SEQ ID NO:3445 | SEQ ID NO:11457 | SEQ ID NO:19469 |
| | | AA | SGTKLGDKYVC | QDTMRPS | QTWDSSTAV |
| | | | SEQ ID NO:3446 | SEQ ID NO:11458 | SEQ ID NO:19470 |
| **iPS:436842** | **21-225_54E9** | NA | TCTGGAAGCAACTCCAACATCGGAAATAATATTGTCACC | GTTAATGATCAGCGGCCCTCA | GCAGCATGGGATGACAGCCTGAATGGTTGGGTG |
| | | | SEQ ID NO:3447 | SEQ ID NO:11459 | SEQ ID NO:19471 |
| | | AA | SGSNSNIGNNIVT | VNDQRPS | AAWDDSLNGWV |
| | | | SEQ ID NO:3448 | SEQ ID NO:11460 | SEQ ID NO:19472 |
| **iPS:436844** | **21-225_56G1** | NA | TCTGGAAGCAGCTCCAACATCGGAAGTCATATTGTTACC | AGTAATGATCAGCGGCCCTCA | GCAGTATGGGATGACAGCCTGATTGGTTGGGTG |
| | | | SEQ ID NO:3449 | SEQ ID NO:11461 | SEQ ID NO:19473 |
| | | AA | SGSSSNIGSHIVT | SNDQRPS | AVWDDSLIGWV |
| | | | SEQ ID NO:3450 | SEQ ID NO:11462 | SEQ ID NO:19474 |
| **iPS:436846** | **21-225_56E3** | NA | TCTGGAAGCAGCTCCAACATCGGAAGTAATATTGTTACC | AGTAATAATCAGCGGCCCTCA | GCAGCATGGGATGACAGCCTGAATGGTTGGGTG |
| | | | SEQ ID NO:3451 | SEQ ID NO:11463 | SEQ ID NO:19475 |
| | | AA | SGSSSNIGSNIVT | SNNQRPS | AAWDDSLNGWV |
| | | | SEQ ID NO:3452 | SEQ ID NO:11464 | SEQ ID NO:19476 |
| **iPS:436848** | **21-225_57F1** | NA | TCTGGAGATAAACTGGGGGAAAAATATGCTTGC | CAAGATAGGAAGCGGCCCTCA | CAGGCGTGGGACAGCAGCACTGTGGTA |
| | | | SEQ ID NO:3453 | SEQ ID NO:11465 | SEQ ID NO:19477 |
| | | AA | SGDKLGEKYAC | QDRKRPS | QAWDSSTVV |
| | | | SEQ ID NO:3454 | SEQ ID NO:11466 | SEQ ID NO:19478 |
| **iPS:436850** | | NA | TCTGGAGAGAAATTGGGGGAAAAATTTGCTTGC | CAAGATAGCAAGCGGCCCTCA | CAGGCGTGGGACAGCAGCACTGTGGTA |

FIGURE 49

| | | | | | |
|---|---|---|---|---|---|
| | 21-225_57D9 | | SEQ ID NO:3455 | SEQ ID NO:11467 | SEQ ID NO:19479 |
| | | AA | SGEKLGEKFAC | QDSKRPS | QAWDSSTVV |
| | | | SEQ ID NO:3456 | SEQ ID NO:11468 | SEQ ID NO:19480 |
| iPS:436852 | 21-225_57H11 | NA | TCTGGAGATAAACTGGGGGAAAAATATGCTTGC | CAAGATAGGAAGCGGCCCTCA | CAGGCGTGGGACAGCAGCACTGTGGTA |
| | | | SEQ ID NO:3457 | SEQ ID NO:11469 | SEQ ID NO:19481 |
| | | AA | SGDKLGEKYAC | QDRKRPS | QAWDSSTVV |
| | | | SEQ ID NO:3458 | SEQ ID NO:11470 | SEQ ID NO:19482 |
| iPS:436854 | 21-225_58C1 | NA | TCTGGAGATAAATTGGGGAATAAATATGCTTGC | CAAGATAGGAAGCGGCCCTCA | CAGGCGTGGGACAGCAGCACTGCA |
| | | | SEQ ID NO:3459 | SEQ ID NO:11471 | SEQ ID NO:19483 |
| | | AA | SGDKLGNKYAC | QDRKRPS | QAWDSSTA |
| | | | SEQ ID NO:3460 | SEQ ID NO:11472 | SEQ ID NO:19484 |
| iPS:436856 | 21-225_58C5 | NA | TCTGGAAGCAGCTCCAACATTGGGAATAATTATGTATCC | GACAATAATAAGCGACCCTCA | GGAACATGGGATATCAGTCTGAGTGTTGGGGTA |
| | | | SEQ ID NO:3461 | SEQ ID NO:11473 | SEQ ID NO:19485 |
| | | AA | SGSSSNIGNNYVS | DNNKRPS | GTWDISLSVGV |
| | | | SEQ ID NO:3462 | SEQ ID NO:11474 | SEQ ID NO:19486 |
| iPS:436858 | 21-225_58E7 | NA | TCTGGAGATAAATTGGGGGATAAATATACTTGC | CAAGATAACAAGCGGCCCTCA | CAGGCGTGGAACAACTACACTGTGGTA |
| | | | SEQ ID NO:3463 | SEQ ID NO:11475 | SEQ ID NO:19487 |
| | | AA | SGDKLGDKYTC | QDNKRPS | QAWNNYTVV |
| | | | SEQ ID NO:3464 | SEQ ID NO:11476 | SEQ ID NO:19488 |
| iPS:436860 | 21-225_58F7 | NA | TCTGGAGATAAATTGGGGGATAAATATGCTTGC | CAAGATAGGAAGCGGCCCTCA | CAGGCGTGGGACAGCAGCACTGTGGTA |
| | | | SEQ ID NO:3465 | SEQ ID NO:11477 | SEQ ID NO:19489 |
| | | AA | SGDKLGDKYAC | QDRKRPS | QAWDSSTVV |
| | | | SEQ ID NO:3466 | SEQ ID NO:11478 | SEQ ID NO:19490 |

# FIGURE 49

| | | | | | |
|---|---|---|---|---|---|
| **iPS:436862** | **21-225_58F8** | NA | TCTGGAGATAAATTGGGAAATAAATATGCTTGC | CAAGATAGCAAGCGGCCCTCA | CAGGCGTGGGACAGCAGCACTGTGGTA |
| | | | SEQ ID NO:3467 | SEQ ID NO:11479 | SEQ ID NO:19491 |
| | | AA | SGDKLGNKYAC | QDSKRPS | QAWDSSTVV |
| | | | SEQ ID NO:3468 | SEQ ID NO:11480 | SEQ ID NO:19492 |
| **iPS:436864** | **21-225_58G11** | NA | TCTGGAGATAAATTGGGGATAAATATGCTTCC | CAAGATAACAAGCGGCCCTCA | CAGGCGTGGAACAACAACACTGTAATG |
| | | | SEQ ID NO:3469 | SEQ ID NO:11481 | SEQ ID NO:19493 |
| | | AA | SGDKLGDKYAS | QDNKRPS | QAWNNNTVM |
| | | | SEQ ID NO:3470 | SEQ ID NO:11482 | SEQ ID NO:19494 |
| **iPS:436866** | **21-225_59F2** | NA | TCTGGAGATAAATTGGGGATAAATATGCTTCT | CAAGATAACAAGCGGCCCTCA | CAGGCGTGGGACAACAACACTGTGGTC |
| | | | SEQ ID NO:3471 | SEQ ID NO:11483 | SEQ ID NO:19495 |
| | | AA | SGDKLGDKYAS | QDNKRPS | QAWDNNTVV |
| | | | SEQ ID NO:3472 | SEQ ID NO:11484 | SEQ ID NO:19496 |
| **iPS:436868** | **21-225_59B11** | NA | TCTGGAGATAAATTGGGGATAAATATGCTTGC | CAAGATAGCAAGCGGCCCTCA | CAGGCGTGGGACAGCAGCACTTATGTGGTA |
| | | | SEQ ID NO:3473 | SEQ ID NO:11485 | SEQ ID NO:19497 |
| | | AA | SGDKLGDKYAC | QDSKRPS | QAWDSSTYVV |
| | | | SEQ ID NO:3474 | SEQ ID NO:11486 | SEQ ID NO:19498 |
| **iPS:436870** | **21-225_60B1** | NA | TCTGGAGATAAACTGGGGAAAAATATGCTTGC | CAAGATAGGAAGCGGCCCTCA | CAGGCGTGGGACAGCAGCACTGTGGTA |
| | | | SEQ ID NO:3475 | SEQ ID NO:11487 | SEQ ID NO:19499 |
| | | AA | SGDKLGEKYAC | QDRKRPS | QAWDSSTVV |
| | | | SEQ ID NO:3476 | SEQ ID NO:11488 | SEQ ID NO:19500 |
| **iPS:436872** | **21-225_60D2** | NA | TCTGGAAATAAATTGGGGATAAATATGCTTCT | CAAGATAACAAGCGGCCCTCA | CAGGCGTGGGACAACAACACTGTGGTC |
| | | | SEQ ID NO:3477 | SEQ ID NO:11489 | SEQ ID NO:19501 |
| | | AA | SGNKLGDKYAS | QDNKRPS | QAWDNNTVV |
| | | | SEQ ID NO:3478 | SEQ ID NO:11490 | SEQ ID NO:19502 |

**FIGURE 49**

| | | | | | |
|---|---|---|---|---|---|
| **iPS:436874** | **21-225_60A12** | NA | TCTGGAGATAAATTGGGGAA TAAATATGCTTGC | CAAGATAGGAAGCGGCC CTCA | CAGGCGTGGGACAGCAGCAC TGCT |
| | | | SEQ ID NO:3479 | SEQ ID NO:11491 | SEQ ID NO:19503 |
| | | AA | SGDKLGNKYAC | QDRKRPS | QAWDSSTA |
| | | | SEQ ID NO:3480 | SEQ ID NO:11492 | SEQ ID NO:19504 |
| **iPS:436876** | **21-225_61F5** | NA | TCTGGAGATAAATTGGGGGA AAAATATGCTTGC | CAAGATAGCAAGCGGCC CTCA | CAGGCGTGGGACAGCAGCAC TGTGGTT |
| | | | SEQ ID NO:3481 | SEQ ID NO:11493 | SEQ ID NO:19505 |
| | | AA | SGDKLGEKYAC | QDSKRPS | QAWDSSTVV |
| | | | SEQ ID NO:3482 | SEQ ID NO:11494 | SEQ ID NO:19506 |
| **iPS:436878** | **21-225_62E3** | NA | TCTGGAGATAAATTGGGGAA TAAATATGCTTGC | CAAGATAGGAAGCGGCC CTCA | CAGGCGTGGGACAGCAGCAC TGCGGTA |
| | | | SEQ ID NO:3483 | SEQ ID NO:11495 | SEQ ID NO:19507 |
| | | AA | SGDKLGNKYAC | QDRKRPS | QAWDSSTAV |
| | | | SEQ ID NO:3484 | SEQ ID NO:11496 | SEQ ID NO:19508 |
| **iPS:436880** | **21-225_62E8** | NA | TCTGGAGATAGATTGGGGAA TAAATATGCTTCC | CAGGATAGGAAGCGGCC CTCA | CAGGCGTGGGACAGCAGCAC TGCGGTA |
| | | | SEQ ID NO:3485 | SEQ ID NO:11497 | SEQ ID NO:19509 |
| | | AA | SGDRLGNKYAS | QDRKRPS | QAWDSSTAV |
| | | | SEQ ID NO:3486 | SEQ ID NO:11498 | SEQ ID NO:19510 |
| **iPS:436882** | **21-225_62D10** | NA | TCTGGAGATAAATTGGGGAA TAAATATGCTTGC | CAAGATAGGAAGCGGCC CTCA | CAGGCGTGGGACAGCAGCAC TGCGGTA |
| | | | SEQ ID NO:3487 | SEQ ID NO:11499 | SEQ ID NO:19511 |
| | | AA | SGDKLGNKYAC | QDRKRPS | QAWDSSTAV |
| | | | SEQ ID NO:3488 | SEQ ID NO:11500 | SEQ ID NO:19512 |
| **iPS:436884** | **21-225_62A12** | NA | TCTGGAGATAAATTGGGGAA TAAATATGCTTGC | CAAGATAGGAAGCGGCC CTCA | CAGGCGTGGGACAGCAGCAC TGCGGTA |
| | | | SEQ ID NO:3489 | SEQ ID NO:11501 | SEQ ID NO:19513 |
| | | AA | SGDKLGNKYAC | QDRKRPS | QAWDSSTAV |
| | | | SEQ ID NO:3490 | SEQ ID NO:11502 | SEQ ID NO:19514 |

FIGURE 49

| | | | | | |
|---|---|---|---|---|---|
| **iPS:436886** | **21-225_62B12** | NA | TCTGGAGATAAATTGGGGAA TAAATATACTTGC | CAAGATAGGAAGCGGCC CTCA | CAGGCGTGGGACAGCAGCAC TGCGGTA |
| | | | SEQ ID NO:3491 | SEQ ID NO:11503 | SEQ ID NO:19515 |
| | | AA | SGDKLGNKYTC | QDRKRPS | QAWDSSTAV |
| | | | SEQ ID NO:3492 | SEQ ID NO:11504 | SEQ ID NO:19516 |
| **iPS:436888** | **21-225_63G7** | NA | ACCCGCAGCAATGGCAGCAT TGTCAGCAACTATGTGCAG | GAGGATAGCCGAAGACC CTCT | CAGTCTTATGATGGCATCAA TGTGGTA |
| | | | SEQ ID NO:3493 | SEQ ID NO:11505 | SEQ ID NO:19517 |
| | | AA | TRSNGSIVSNYVQ | EDSRRPS | QSYDGINVV |
| | | | SEQ ID NO:3494 | SEQ ID NO:11506 | SEQ ID NO:19518 |
| **iPS:436890** | **21-225_63A10** | NA | ACCCGCAGCAATGGCAGCAT TGTCAGCAACTATGTGCAG | GAGGATAAAAGAAGACC CTCA | CAGTCTTATGATAGCATCAA TGTGGTA |
| | | | SEQ ID NO:3495 | SEQ ID NO:11507 | SEQ ID NO:19519 |
| | | AA | TRSNGSIVSNYVQ | EDKRRPS | QSYDSINVV |
| | | | SEQ ID NO:3496 | SEQ ID NO:11508 | SEQ ID NO:19520 |
| **iPS:436892** | **21-225_65E9** | NA | TCTGGAGATAAATTGGGGAA TAAATATGATTAC | CAAGATAGAAAGCGGCC CTCA | CAGGCGTGGGACAACAGCAC TGTGGTA |
| | | | SEQ ID NO:3497 | SEQ ID NO:11509 | SEQ ID NO:19521 |
| | | AA | SGDKLGNKYDY | QDRKRPS | QAWDNSTVV |
| | | | SEQ ID NO:3498 | SEQ ID NO:11510 | SEQ ID NO:19522 |
| **iPS:436894** | **21-225_66G9** | NA | TCTGGAGATAAATTGGGGAA TAAATATGCTTGC | CAAGATAGGAAGCGGCC CTCA | CAGGCGTGGGACATCAACAC TGCGGTA |
| | | | SEQ ID NO:3499 | SEQ ID NO:11511 | SEQ ID NO:19523 |
| | | AA | SGDKLGNKYAC | QDRKRPS | QAWDINTAV |
| | | | SEQ ID NO:3500 | SEQ ID NO:11512 | SEQ ID NO:19524 |
| **iPS:436896** | **21-225_67F10** | NA | TCTGGAGATAAATTGGGGTA TAAATATGCTTGG | GAAGATAGGAAGCGGCC CTCA | CAGGCGTGGGACAACAGCAC TGTGGTA |
| | | | SEQ ID NO:3501 | SEQ ID NO:11513 | SEQ ID NO:19525 |

FIGURE 49

| | | | | | |
|---|---|---|---|---|---|
| | | AA | SGDKLGYKYAW | EDRKRPS | QAWDNSTVV |
| | | | SEQ ID NO:3502 | SEQ ID NO:11514 | SEQ ID NO:19526 |
| **iPS:436898** | **21-225_68D8** | NA | TCTGGAGATAAATTGGGGGA TAAATATGCTTGC | CAAGATAGCAAGCGGCC CTCA | CAGGCGTGGGACAACAGCAC TGTGGTA |
| | | | SEQ ID NO:3503 | SEQ ID NO:11515 | SEQ ID NO:19527 |
| | | AA | SGDKLGDKYAC | QDSKRPS | QAWDNSTVV |
| | | | SEQ ID NO:3504 | SEQ ID NO:11516 | SEQ ID NO:19528 |
| **iPS:436900** | **21-225_69B9** | NA | TCTGGAGATAAATTGGGGAA TAAATATGATTAC | CAAGATAGAAAGCGGCC CTCA | CAGGCGTGGGACAACAGCAC TGTGGTA |
| | | | SEQ ID NO:3505 | SEQ ID NO:11517 | SEQ ID NO:19529 |
| | | AA | SGDKLGNKYDY | QDRKRPS | QAWDNSTVV |
| | | | SEQ ID NO:3506 | SEQ ID NO:11518 | SEQ ID NO:19530 |
| **iPS:436902** | **21-225_69B11** | NA | TCTGGAGATAAATTGGGGGA TAAATATGCTTGG | GAAGATAGGAAGCGGCC CTCA | CAGGCGTGGGACAACAGCAC TGTGGTA |
| | | | SEQ ID NO:3507 | SEQ ID NO:11519 | SEQ ID NO:19531 |
| | | AA | SGDKLGDKYAW | EDRKRPS | QAWDNSTVV |
| | | | SEQ ID NO:3508 | SEQ ID NO:11520 | SEQ ID NO:19532 |
| **iPS:436904** | **21-225_71D4** | NA | TCTGGAGATAAATTGGGGGA TAAATATGCTTAC | CAAGATAGGAAGCGGCC CTCA | CAGGCGTGGGTCAACAGCAC TGTGGTA |
| | | | SEQ ID NO:3509 | SEQ ID NO:11521 | SEQ ID NO:19533 |
| | | AA | SGDKLGDKYAY | QDRKRPS | QAWVNSTVV |
| | | | SEQ ID NO:3510 | SEQ ID NO:11522 | SEQ ID NO:19534 |
| **iPS:436906** | **21-225_72B4** | NA | TCTGGAGATAAATTGGGGGA TAAATATGCTTGG | GAAGATAGGAAGCGGCC CTCA | CAGGCGTGGGACAACAGCAC TGTGGTA |
| | | | SEQ ID NO:3511 | SEQ ID NO:11523 | SEQ ID NO:19535 |
| | | AA | SGDKLGDKYAW | EDRKRPS | QAWDNSTVV |
| | | | SEQ ID NO:3512 | SEQ ID NO:11524 | SEQ ID NO:19536 |
| **iPS:436908** | **21-225_72D5** | NA | TCTGGAGATAAATTGGGGAA TAAATATGCTTGC | CAAGATAGGAAGCGGCC CTCA | CAGGCGTGGGACAGCAGCAC TGCGGTA |
| | | | SEQ ID NO:3513 | SEQ ID NO:11525 | SEQ ID NO:19537 |

**FIGURE 49**

| | | | | | |
|---|---|---|---|---|---|
| | | AA | SGDKLGNKYAC | QDRKRPS | QAWDSSTAV |
| | | | SEQ ID NO:3514 | SEQ ID NO:11526 | SEQ ID NO:19538 |
| **iPS:436910** | **21-225_73G1** | NA | GGCTTGAGCTCTGGCTCAGTCTCTACTAGTTACTACCCCAGC | AACACAAACACTCGCTCTTCT | GTTCTATATATGGGTAGTGCCATTTGGGTG |
| | | | SEQ ID NO:3515 | SEQ ID NO:11527 | SEQ ID NO:19539 |
| | | AA | GLSSGSVSTSYYPS | NTNTRSS | VLYMGSAIWV |
| | | | SEQ ID NO:3516 | SEQ ID NO:11528 | SEQ ID NO:19540 |
| **iPS:436912** | **21-225_73C4** | NA | TCTGGAGATAAATTGGGGAATAAATATGCTTGC | CAAGATATGAAGCGGCCCTCA | CAGGCGTGGGACAGCAGCACTGCGGTA |
| | | | SEQ ID NO:3517 | SEQ ID NO:11529 | SEQ ID NO:19541 |
| | | AA | SGDKLGNKYAC | QDMKRPS | QAWDSSTAV |
| | | | SEQ ID NO:3518 | SEQ ID NO:11530 | SEQ ID NO:19542 |
| **iPS:436914** | **21-225_76B4** | NA | TCTGGAGATAGATTGGGGACTAAATTTGCTTGC | CAAGATAGCAAGCGGCCCTCA | CAGGCGTGGGACAGCAGCACTGTA |
| | | | SEQ ID NO:3519 | SEQ ID NO:11531 | SEQ ID NO:19543 |
| | | AA | SGDRLGTKFAC | QDSKRPS | QAWDSSTV |
| | | | SEQ ID NO:3520 | SEQ ID NO:11532 | SEQ ID NO:19544 |
| **iPS:436916** | **21-225_74A9** | NA | TCTGGAGATAAATTGGGTAATAAATATGTTTGT | CAAGATAACAGGCGGCCCTCA | CAGGCGTGGGACAGCAGTCCTGTGATA |
| | | | SEQ ID NO:3521 | SEQ ID NO:11533 | SEQ ID NO:19545 |
| | | AA | SGDKLGNKYVC | QDNRRPS | QAWDSSPVI |
| | | | SEQ ID NO:3522 | SEQ ID NO:11534 | SEQ ID NO:19546 |
| **iPS:436918** | **21-225_77A2** | NA | TCTGGAGATAGATTGGGGGATAAATATGCTTGC | CAAGATAGGAAGCGGCCCTCA | CAGGCGTGGGACAGCAGTACTGCGGTA |
| | | | SEQ ID NO:3523 | SEQ ID NO:11535 | SEQ ID NO:19547 |
| | | AA | SGDRLGDKYAC | QDRKRPS | QAWDSSTAV |
| | | | SEQ ID NO:3524 | SEQ ID NO:11536 | SEQ ID NO:19548 |

FIGURE 49

| | | | | | |
|---|---|---|---|---|---|
| **iPS:436920** | **21-225_74E5** | NA | GCTTCCAGCACTGAAACAGTCACCAGTGGTTCTTATCCGAAC | AGTACAAGCAACAAACACTCC | CTGCTCTACTATGGTGGTGCTCAACTGGTA |
| | | | SEQ ID NO:3525 | SEQ ID NO:11537 | SEQ ID NO:19549 |
| | | AA | ASSTETVTSGSYPN | STSNKHS | LLYYGGAQLV |
| | | | SEQ ID NO:3526 | SEQ ID NO:11538 | SEQ ID NO:19550 |
| **iPS:436922** | **21-225_78E9** | NA | TCAGGAGATAAATTGGGGAATAAATATGTTTCC | CAAGATAACAGGCGGCCGTCA | CAGGCGTGGGACAGCAGCCCTGTGATA |
| | | | SEQ ID NO:3527 | SEQ ID NO:11539 | SEQ ID NO:19551 |
| | | AA | SGDKLGNKYVS | QDNRRPS | QAWDSSPVI |
| | | | SEQ ID NO:3528 | SEQ ID NO:11540 | SEQ ID NO:19552 |
| **iPS:436924** | **21-225_74B3** | NA | TCTGGAGATAAATTGGGGGATAAATATGCTTGC | CAAGATAGCAAGCGGCCCTCA | CAGGCGTGGGACAGCACCACTGTGGTA |
| | | | SEQ ID NO:3529 | SEQ ID NO:11541 | SEQ ID NO:19553 |
| | | AA | SGDKLGDKYAC | QDSKRPS | QAWDSTTVV |
| | | | SEQ ID NO:3530 | SEQ ID NO:11542 | SEQ ID NO:19554 |
| **iPS:436926** | **21-225_78D10** | NA | GCTTCCAGCACTGGAGCAGTCACCAGTGGTTACTTTCCAAAC | AGTACAGACAACAAACACTCC | CTCCTCTACTATGGTGGTGCTCAGCTGATG |
| | | | SEQ ID NO:3531 | SEQ ID NO:11543 | SEQ ID NO:19555 |
| | | AA | ASSTGAVTSGYFPN | STDNKHS | LLYYGGAQLM |
| | | | SEQ ID NO:3532 | SEQ ID NO:11544 | SEQ ID NO:19556 |
| **iPS:436928** | **21-225_79E7** | NA | TCAGGAGATAAATTGGGGAATAAATATGTTTCC | CAAGATAACAGGCGGCCCTCA | CAGGCGTGGGACAGCAGCCCTGTGATA |
| | | | SEQ ID NO:3533 | SEQ ID NO:11545 | SEQ ID NO:19557 |
| | | AA | SGDKLGNKYVS | QDNRRPS | QAWDSSPVI |
| | | | SEQ ID NO:3534 | SEQ ID NO:11546 | SEQ ID NO:19558 |
| **iPS:436932** | **21-225_92A4** | NA | TCTGGAGATAAATTGGGGAATAAATATGTTTGC | CAAGATAACAGGCGGCCCTCA | CAGGCGTGGGACAGCAGCCCTGTGATA |
| | | | SEQ ID NO:3535 | SEQ ID NO:11547 | SEQ ID NO:19559 |

**FIGURE 49**

| | | | | | |
|---|---|---|---|---|---|
| | | AA | SGDKLGNKYVC | QDNRRPS | QAWDSSPVI |
| | | | SEQ ID NO:3536 | SEQ ID NO:11548 | SEQ ID NO:19560 |
| **iPS:436934** | **21-225_96B5** | NA | TCTGGAGATAGATTGGGGACTAAATTTGCTTGC | CAAGATAGCAAGCGGCCCTCA | CAGGCGTGGGACAGCAGCACTGTA |
| | | | SEQ ID NO:3537 | SEQ ID NO:11549 | SEQ ID NO:19561 |
| | | AA | SGDRLGTKFAC | QDSKRPS | QAWDSSTV |
| | | | SEQ ID NO:3538 | SEQ ID NO:11550 | SEQ ID NO:19562 |
| **iPS:436936** | **21-225_97E6** | NA | TCTGGAGATAAATTGGGGAATAAATATGTTTCC | CAAGATAACAGGCGGCCGTCA | CAGGCGTGGGACAGCACCCCTGTGATA |
| | | | SEQ ID NO:3539 | SEQ ID NO:11551 | SEQ ID NO:19563 |
| | | AA | SGDKLGNKYVS | QDNRRPS | QAWDSTPVI |
| | | | SEQ ID NO:3540 | SEQ ID NO:11552 | SEQ ID NO:19564 |
| **iPS:436938** | **21-225_146A3** | NA | TCTGGAAATAAATTGGGGAATAGATATGCTTGC | CAAGATAGCAAGCGGCCCTCA | CAGGCGTGGGACAGCAGCACTGTGGTA |
| | | | SEQ ID NO:3541 | SEQ ID NO:11553 | SEQ ID NO:19565 |
| | | AA | SGNKLGNRYAC | QDSKRPS | QAWDSSTVV |
| | | | SEQ ID NO:3542 | SEQ ID NO:11554 | SEQ ID NO:19566 |
| **iPS:436940** | **21-225_146B8** | NA | TCTGGAGATAAATTGGGGGATAAATATGTTTGC | CAAGATAGGAAGCGGCCCTCA | CAGGCGTGGCACAGCAGCACTGTGGTA |
| | | | SEQ ID NO:3543 | SEQ ID NO:11555 | SEQ ID NO:19567 |
| | | AA | SGDKLGDKYVC | QDRKRPS | QAWHSSTVV |
| | | | SEQ ID NO:3544 | SEQ ID NO:11556 | SEQ ID NO:19568 |
| **iPS:436942** | **21-225_146H8** | NA | TCTGGAGATAAATTGGGGGATAAATATGCTTGC | CAAGATAAGAAGCGGCCCTCA | CAGGCGTGGGACATCAGAACTGTGGTA |
| | | | SEQ ID NO:3545 | SEQ ID NO:11557 | SEQ ID NO:19569 |
| | | AA | SGDKLGDKYAC | QDKKRPS | QAWDIRTVV |
| | | | SEQ ID NO:3546 | SEQ ID NO:11558 | SEQ ID NO:19570 |
| **iPS:436944** | **21-225_182D12** | NA | TCTGGAGATAAATTGGGGGAGAAATATGCTTGC | CAAGATAGAAAGCGGCCCTCA | CAGGCGTGGGACAGTAGAACTGCGGTA |
| | | | SEQ ID NO:3547 | SEQ ID NO:11559 | SEQ ID NO:19571 |

FIGURE 49

| | | | | | |
|---|---|---|---|---|---|
| | | AA | SGDKLGEKYAC | QDRKRPS | QAWDSRTAV |
| | | | SEQ ID NO:3548 | SEQ ID NO:11560 | SEQ ID NO:19572 |
| **iPS:436946** | **21-225_183F4** | NA | TCTGGAGATAAATTGGGGGATAAATATGCTTGT | CAAGATAAGAAACGGCCCTCA | CAGGCGTGGGACAACAGCACTGCTGTGGTA |
| | | | SEQ ID NO:3549 | SEQ ID NO:11561 | SEQ ID NO:19573 |
| | | AA | SGDKLGDKYAC | QDKKRPS | QAWDNSTAVV |
| | | | SEQ ID NO:3550 | SEQ ID NO:11562 | SEQ ID NO:19574 |
| **iPS:436948** | **21-225_183F5** | NA | GGCTTGAGCTCTGGCTCAGTCTCTACTACTTTCTACCCCAGC | AACACAAACACTCGCTCTTCT | GTGCTTTATATGGGTAGTGGCATTTGGGTG |
| | | | SEQ ID NO:3551 | SEQ ID NO:11563 | SEQ ID NO:19575 |
| | | AA | GLSSGSVSTTFYPS | NTNTRSS | VLYMGSGIWV |
| | | | SEQ ID NO:3552 | SEQ ID NO:11564 | SEQ ID NO:19576 |
| **iPS:436950** | **21-225_184G4** | NA | TCTGGAGATAAATTGGGGGATAAATTTGCTTGC | GAAGATAGGAAGCGGCCCTCA | CAGGCGTGGGACAGCCGCACTGTGGTA |
| | | | SEQ ID NO:3553 | SEQ ID NO:11565 | SEQ ID NO:19577 |
| | | AA | SGDKLGDKFAC | EDRKRPS | QAWDSRTVV |
| | | | SEQ ID NO:3554 | SEQ ID NO:11566 | SEQ ID NO:19578 |
| **iPS:436952** | **21-225_185D2** | NA | TCTGGAGATAAATTGGGGGATAAATATGCTTGC | GAAGATAGGAAGCGGCCCTCA | CAGGCGTGGGACAGCAGGAAAGTGGTA |
| | | | SEQ ID NO:3555 | SEQ ID NO:11567 | SEQ ID NO:19579 |
| | | AA | SGDKLGDKYAC | EDRKRPS | QAWDSRKVV |
| | | | SEQ ID NO:3556 | SEQ ID NO:11568 | SEQ ID NO:19580 |
| **iPS:436954** | **21-225_185G7** | NA | TCTGGAGATAAATTGGGGCATAAATTTGTTTGC | CAAGATCGCAAGCGGCCCTCA | CAGGCGTGGGACAGCAGCACGGTA |
| | | | SEQ ID NO:3557 | SEQ ID NO:11569 | SEQ ID NO:19581 |
| | | AA | SGDKLGHKFVC | QDRKRPS | QAWDSSTV |
| | | | SEQ ID NO:3558 | SEQ ID NO:11570 | SEQ ID NO:19582 |
| **iPS:436956** | | NA | TCTGGAGATAAAATGGGGGAAAAATATGCTTGC | CAAGATAGAAAGCGGCCCTCA | CAGGCGTGGGACAGCAGCACTGCGGTA |

FIGURE 49

| | | | | | |
|---|---|---|---|---|---|
| | **21-225_186H6** | | SEQ ID NO:3559 | SEQ ID NO:11571 | SEQ ID NO:19583 |
| | | AA | SGDKMGEKYAC | QDRKRPS | QAWDSSTAV |
| | | | SEQ ID NO:3560 | SEQ ID NO:11572 | SEQ ID NO:19584 |
| **iPS:436958** | **21-225_190D1** | NA | GCTTCCAGCACTGGAGCAGTCACCAGTGGTTCCTATCCAAAC | AGTACAAGTAACAAACACTCC | CTGCTCTACTATGGTGGTGCTCAGGTGGCA |
| | | | SEQ ID NO:3561 | SEQ ID NO:11573 | SEQ ID NO:19585 |
| | | AA | ASSTGAVTSGSYPN | STSNKHS | LLYYGGAQVA |
| | | | SEQ ID NO:3562 | SEQ ID NO:11574 | SEQ ID NO:19586 |
| **iPS:436960** | **21-225_198D2** | NA | TCTGGAAGCAGCTCCAACATTGGGAGTAATTATGTTTCC | GACAATAATAAGCGACCCTCA | GGAACATGGGATAGCAGACTGAATGTTGGGGTA |
| | | | SEQ ID NO:3563 | SEQ ID NO:11575 | SEQ ID NO:19587 |
| | | AA | SGSSSNIGSNYVS | DNNKRPS | GTWDSRLNVGV |
| | | | SEQ ID NO:3564 | SEQ ID NO:11576 | SEQ ID NO:19588 |
| **iPS:436962** | **21-225_190H1** | NA | TCTGGAGATAAATTGGGGGATAGATTTGCTTAC | CAAGATAGCAAGCGGCCCTCA | AAGGCGTGGGACAGCAGCACTGTGGTA |
| | | | SEQ ID NO:3565 | SEQ ID NO:11577 | SEQ ID NO:19589 |
| | | AA | SGDKLGDRFAY | QDSKRPS | KAWDSSTVV |
| | | | SEQ ID NO:3566 | SEQ ID NO:11578 | SEQ ID NO:19590 |
| **iPS:436964** | **21-225_190B3** | NA | CAAGGAGACAAACTCAGAACCTATTATGCAAGC | GGAAAAAACAACCGGCCCTCA | AACTCCCGGGACAGCAGTGGTAACCATCTTGTACTA |
| | | | SEQ ID NO:3567 | SEQ ID NO:11579 | SEQ ID NO:19591 |
| | | AA | QGDKLRTYYAS | GKNNRPS | NSRDSSGNHLVL |
| | | | SEQ ID NO:3568 | SEQ ID NO:11580 | SEQ ID NO:19592 |
| **iPS:436966** | **21-225_190C3** | NA | TCTGGAAGCAGCTCCAACATTGGAAATAATTATGTATCC | GACAGTAATAAGCGACCCTCA | GGAACATGGGATAGCAGCCTGAGTACTGTGGTA |
| | | | SEQ ID NO:3569 | SEQ ID NO:11581 | SEQ ID NO:19593 |
| | | AA | SGSSSNIGNNYVS | DSNKRPS | GTWDSSLSTVV |
| | | | SEQ ID NO:3570 | SEQ ID NO:11582 | SEQ ID NO:19594 |

# FIGURE 49

| | | | | | |
|---|---|---|---|---|---|
| **iPS:436968** | **21-225_190B10** | NA | TCTGGAAGCAGCTCCAACATTGGGAATAATTATGTATCC | GACAATAATAAGCGACCCTCA | GGAACATGGGATAGCAGCCTGAGTGCTGGGGTT |
| | | | SEQ ID NO:3571 | SEQ ID NO:11583 | SEQ ID NO:19595 |
| | | AA | SGSSSNIGNNYVS | DNNKRPS | GTWDSSLSAGV |
| | | | SEQ ID NO:3572 | SEQ ID NO:11584 | SEQ ID NO:19596 |
| **iPS:436970** | **21-225_190B8** | NA | CAAGGAGACACCCTCAGACCCTATTATGTAAGC | GGTAAAAACAACCGGCCCTCA | AACTCCCGGGACAGCAGTGGTAACCATCTTGTGGTA |
| | | | SEQ ID NO:3573 | SEQ ID NO:11585 | SEQ ID NO:19597 |
| | | AA | QGDTLRPYYVS | GKNNRPS | NSRDSSGNHLVV |
| | | | SEQ ID NO:3574 | SEQ ID NO:11586 | SEQ ID NO:19598 |
| **iPS:436972** | **21-225_190C7** | NA | TCTGGAGGCAGCTCCAACATTGGGAATAATTATGTATCC | GACAATAATAAGCGACCCTCA | GGAACATGGGATCGCACCCTGAGTGATTGGGTA |
| | | | SEQ ID NO:3575 | SEQ ID NO:11587 | SEQ ID NO:19599 |
| | | AA | SGGSSNIGNNYVS | DNNKRPS | GTWDRTLSDWV |
| | | | SEQ ID NO:3576 | SEQ ID NO:11588 | SEQ ID NO:19600 |
| **iPS:436974** | **21-225_190H7** | NA | TCTGGAAGCAGCTCCAACATTGGGAGTAATTATGTTTCC | GACAATAATAAGCGACCCTCA | GGAACATGGGATGGCAGACTGAATGTTGGGGTA |
| | | | SEQ ID NO:3577 | SEQ ID NO:11589 | SEQ ID NO:19601 |
| | | AA | SGSSSNIGSNYVS | DNNKRPS | GTWDGRLNVGV |
| | | | SEQ ID NO:3578 | SEQ ID NO:11590 | SEQ ID NO:19602 |
| **iPS:436976** | **21-225_190D8** | NA | TCTGGAAGCAGCTCCAACATTGGGAATCATTATGTCTCC | GACAGTAGTAAGCGACCCTCA | GGAACATGGGATAGTAGTCTGAGTACTGTGGTA |
| | | | SEQ ID NO:3579 | SEQ ID NO:11591 | SEQ ID NO:19603 |
| | | AA | SGSSSNIGNHYVS | DSSKRPS | GTWDSSLSTVV |
| | | | SEQ ID NO:3580 | SEQ ID NO:11592 | SEQ ID NO:19604 |
| **iPS:436978** | **21-225_190G9** | NA | TCTGGAGATAAATTGGGGGATAGATTTGCTTAC | CAAGATAACAAGCGGCCCTCA | CAGGCGTGGGACAGCAGCACTGTGGTA |
| | | | SEQ ID NO:3581 | SEQ ID NO:11593 | SEQ ID NO:19605 |

FIGURE 49

| | | | | | |
|---|---|---|---|---|---|
| | | AA | SGDKLGDRFAY | QDNKRPS | QAWDSSTVV |
| | | | SEQ ID NO:3582 | SEQ ID NO:11594 | SEQ ID NO:19606 |
| **iPS:436980** | **21-225_190C10** | NA | CAAGGAGACAGCCTCAGACCCTATTATGCAAGC | GGTAAAAACAACCGGCCCTCA | AACTCCCGGGACAGCAGTGGTAACCATCTTGTGGTA |
| | | | SEQ ID NO:3583 | SEQ ID NO:11595 | SEQ ID NO:19607 |
| | | AA | QGDSLRPYYAS | GKNNRPS | NSRDSSGNHLVV |
| | | | SEQ ID NO:3584 | SEQ ID NO:11596 | SEQ ID NO:19608 |
| **iPS:436982** | **21-225_190D10** | NA | TCTGGAAGCAGCTCCAACATTGGGAGTAATTATGTTTCC | GACAATAATAAGCGACCCTCA | GGAACATGGGATAGCAGACTGAATGTTGGGGTA |
| | | | SEQ ID NO:3585 | SEQ ID NO:11597 | SEQ ID NO:19609 |
| | | AA | SGSSSNIGSNYVS | DNNKRPS | GTWDSRLNVGV |
| | | | SEQ ID NO:3586 | SEQ ID NO:11598 | SEQ ID NO:19610 |
| **iPS:436984** | **21-225_190F10** | NA | GTTTTTAGCACTGGAGCAGTCACCAGTGGTTCCTTTCCAAAC | AGTACAAGCAACAAACACTCC | CTGCTCTACTGTGGTGGTGCTCAGCTGGTG |
| | | | SEQ ID NO:3587 | SEQ ID NO:11599 | SEQ ID NO:19611 |
| | | AA | VFSTGAVTSGSFPN | STSNKHS | LLYCGGAQLV |
| | | | SEQ ID NO:3588 | SEQ ID NO:11600 | SEQ ID NO:19612 |
| **iPS:436986** | **21-225_191A1** | NA | TCTGGAAGCAGCTCCAACCTTGGAAATAATTTTGTATCC | GACAATTATAAGCGACCCTCA | GGAACATGGGATAGCAGCCTGAATACTGGGGTA |
| | | | SEQ ID NO:3589 | SEQ ID NO:11601 | SEQ ID NO:19613 |
| | | AA | SGSSSNLGNNFVS | DNYKRPS | GTWDSSLNTGV |
| | | | SEQ ID NO:3590 | SEQ ID NO:11602 | SEQ ID NO:19614 |
| **iPS:436988** | **21-225_191A2** | NA | GTTCTTAGCACTGGAGCAGTCACCAGTGGTTCCTTTCCAAAC | AGTACAAGCAACAAACACTCC | ATGCTCTACTGTGGTGGTGCTCAGCTGGTG |
| | | | SEQ ID NO:3591 | SEQ ID NO:11603 | SEQ ID NO:19615 |
| | | AA | VLSTGAVTSGSFPN | STSNKHS | MLYCGGAQLV |
| | | | SEQ ID NO:3592 | SEQ ID NO:11604 | SEQ ID NO:19616 |

FIGURE 49

| | | | | | |
|---|---|---|---|---|---|
| **iPS:436992** | **21-225_191B8** | NA | CAAGGAGACACCCTCAGACCCTATTATGCAAGT | GGTAAAAACAACCGGCCCTCA | AACTCCCGGGACAGCAGTGGTAACCATCTTGTGGTA |
| | | | SEQ ID NO:3593 | SEQ ID NO:11605 | SEQ ID NO:19617 |
| | | AA | QGDTLRPYYAS | GKNNRPS | NSRDSSGNHLVV |
| | | | SEQ ID NO:3594 | SEQ ID NO:11606 | SEQ ID NO:19618 |
| **iPS:436994** | **21-225_191A9** | NA | CAAGGAGACAGCCTCAGACCCTATTATGCAAGC | GGTAAAAACAACCGGCCCTCA | AACTCCCGGGACAGCTGTGGTAACCATCTTGTGGTA |
| | | | SEQ ID NO:3595 | SEQ ID NO:11607 | SEQ ID NO:19619 |
| | | AA | QGDSLRPYYAS | GKNNRPS | NSRDSCGNHLVV |
| | | | SEQ ID NO:3596 | SEQ ID NO:11608 | SEQ ID NO:19620 |
| **iPS:436996** | **21-225_191B9** | NA | TCTGGAAGCAGCTCCAACATCGGGAATAATTATGTATCC | GACAATAAAAAGCGACCCTCA | GGAACATGGGATAGCAGCCTGAGTGTTTGTGTC |
| | | | SEQ ID NO:3597 | SEQ ID NO:11609 | SEQ ID NO:19621 |
| | | AA | SGSSSNIGNNYVS | DNKKRPS | GTWDSSLSVCV |
| | | | SEQ ID NO:3598 | SEQ ID NO:11610 | SEQ ID NO:19622 |
| **iPS:437000** | **21-225_191G9** | NA | ACCTTACGCAGTGGCATCAATGTTGGTACCTACAGGATATAC | TACAAATCAGACTCAGATAAGCAGCAGGGCTCT | ATGATTTGGCACAGCAGCGCTGTGGTA |
| | | | SEQ ID NO:3599 | SEQ ID NO:11611 | SEQ ID NO:19623 |
| | | AA | TLRSGINVGTYRIY | YKSDSDKQQGS | MIWHSSAVV |
| | | | SEQ ID NO:3600 | SEQ ID NO:11612 | SEQ ID NO:19624 |
| **iPS:437002** | **21-225_191H9** | NA | GCTTCCAGCACTGGAGCAGTCACCAGTGCTTACTATCCAAAC | AGTACAAACAACAAACACTCC | CTGATCTTCTATGGTGGTGTACATGTGATA |
| | | | SEQ ID NO:3601 | SEQ ID NO:11613 | SEQ ID NO:19625 |
| | | AA | ASSTGAVTSAYYPN | STNNKHS | LIFYGGVHVI |
| | | | SEQ ID NO:3602 | SEQ ID NO:11614 | SEQ ID NO:19626 |

FIGURE 49

| | | | | | |
|---|---|---|---|---|---|
| **iPS:437006** | **21-225_192G2** | NA | TCTGGAAGCAGCTCCAACATTGGGAATAATTATGTATCC | GACAATAATAAGCGACCCTCA | GGAACATGGGATAGCAGCCTGAGTGCTGGGGTA |
| | | | SEQ ID NO:3603 | SEQ ID NO:11615 | SEQ ID NO:19627 |
| | | AA | SGSSSNIGNNYVS | DNNKRPS | GTWDSSLSAGV |
| | | | SEQ ID NO:3604 | SEQ ID NO:11616 | SEQ ID NO:19628 |
| **iPS:437008** | **21-225_192E3** | NA | GCTTTCAGCACTGGATCAGTCACCAGTGGTTCCTATCCAAAC | AGTACAAACAACAAACACTCC | CTGCTATACTATGGTGGTGCTCAGCTGGTG |
| | | | SEQ ID NO:3605 | SEQ ID NO:11617 | SEQ ID NO:19629 |
| | | AA | AFSTGSVTSGSYPN | STNNKHS | LLYYGGAQLV |
| | | | SEQ ID NO:3606 | SEQ ID NO:11618 | SEQ ID NO:19630 |
| **iPS:437010** | **21-225_192G3** | NA | TCTGGAAGCAGCTCCAACATCGGAAGTAATACTGTAAAC | GGTAATAAGCAGCGGCCCTCA | GCAGCGTGGGATGACAGCCTGAATGGTTGGGTG |
| | | | SEQ ID NO:3607 | SEQ ID NO:11619 | SEQ ID NO:19631 |
| | | AA | SGSSSNIGSNTVN | GNKQRPS | AAWDDSLNGWV |
| | | | SEQ ID NO:3608 | SEQ ID NO:11620 | SEQ ID NO:19632 |
| **iPS:437012** | **21-225_192G7** | NA | GCTTCCAGCACTGGAGCAGTCACCAGTGGTAACTATCCACAG | AGTACAACCAACAGACATTCC | CTGTTCTACTATGGTGGTGCTCAGGTGATA |
| | | | SEQ ID NO:3609 | SEQ ID NO:11621 | SEQ ID NO:19633 |
| | | AA | ASSTGAVTSGNYPQ | STTNRHS | LFYYGGAQVI |
| | | | SEQ ID NO:3610 | SEQ ID NO:11622 | SEQ ID NO:19634 |
| **iPS:437014** | **21-225_192H8** | NA | GCTTTCAGCACTGGAACAGTCACCAGTGGTTTCTATCCAAAC | AATACAAGCAACAGACACTCC | CTGCTGTACTATGGTGGTGCTCAGCTGATG |
| | | | SEQ ID NO:3611 | SEQ ID NO:11623 | SEQ ID NO:19635 |
| | | AA | AFSTGTVTSGFYPN | NTSNRHS | LLYYGGAQLM |
| | | | SEQ ID NO:3612 | SEQ ID NO:11624 | SEQ ID NO:19636 |

FIGURE 49

| | | | | | |
|---|---|---|---|---|---|
| **iPS:437016** | **21-225_193A6** | NA | CAAGGAGACAGCCTCAGAAGCTATTATGCAAAC | GCTAAGAACAACCGGCCCTCA | AATTCCCGGGACAGCAGTGGTAACCATCTGGTA |
| | | | SEQ ID NO:3613 | SEQ ID NO:11625 | SEQ ID NO:19637 |
| | | AA | QGDSLRSYYAN | AKNNRPS | NSRDSSGNHLV |
| | | | SEQ ID NO:3614 | SEQ ID NO:11626 | SEQ ID NO:19638 |
| **iPS:437018** | **21-225_193H5** | NA | TCTGGAGATAAATTGGGGGATAGATTTGCTTGC | CAAGATAGCAAGCGGCCCTCA | CAGGCGTGGGACAGCAGCACTGCGGTA |
| | | | SEQ ID NO:3615 | SEQ ID NO:11627 | SEQ ID NO:19639 |
| | | AA | SGDKLGDRFAC | QDSKRPS | QAWDSSTAV |
| | | | SEQ ID NO:3616 | SEQ ID NO:11628 | SEQ ID NO:19640 |
| **iPS:437020** | **21-225_193F11** | NA | TTCGGAGGCAGCTCCAACATTGGGAATAATTATGTATCC | GACAATAATAAGCGACCCTCA | GGAACATGGGATCGCACCATGAGTGATTGGGTA |
| | | | SEQ ID NO:3617 | SEQ ID NO:11629 | SEQ ID NO:19641 |
| | | AA | FGGSSNIGNNYVS | DNNKRPS | GTWDRTMSDWV |
| | | | SEQ ID NO:3618 | SEQ ID NO:11630 | SEQ ID NO:19642 |
| **iPS:437022** | **21-225_194G5** | NA | GCTTCCAGCACTGGAGCAGTCACCAGTGGTAACTATCCAAAC | AGTACAAGCAACAAACACTCC | CTGATCTACTATGGTGGTGCTCAGCTGATG |
| | | | SEQ ID NO:3619 | SEQ ID NO:11631 | SEQ ID NO:19643 |
| | | AA | ASSTGAVTSGNYPN | STSNKHS | LIYYGGAQLM |
| | | | SEQ ID NO:3620 | SEQ ID NO:11632 | SEQ ID NO:19644 |
| **iPS:437024** | **21-225_194F11** | NA | TCTGGAAGCAGCTCCAACATTGGGAATAATTATGTATCC | GACAATAATAAGCGACCCTCA | GGAACATGGGATAGCAGCCTGAGTGCTGGGGTT |
| | | | SEQ ID NO:3621 | SEQ ID NO:11633 | SEQ ID NO:19645 |
| | | AA | SGSSSNIGNNYVS | DNNKRPS | GTWDSSLSAGV |
| | | | SEQ ID NO:3622 | SEQ ID NO:11634 | SEQ ID NO:19646 |

FIGURE 49

| | | | | | |
|---|---|---|---|---|---|
| **iPS:437026** | **21-225_194D12** | NA | GCTTCCAGCACTGGAGCAGTCACCAGTGGTTCCTTTCCAAGC | AGTACAAGCAACAGACACTCC | CTGATCTACTATGGTGGTGCTCAGCTGGCA |
| | | | SEQ ID NO:3623 | SEQ ID NO:11635 | SEQ ID NO:19647 |
| | | AA | ASSTGAVTSGSFPS | STSNRHS | LIYYGGAQLA |
| | | | SEQ ID NO:3624 | SEQ ID NO:11636 | SEQ ID NO:19648 |
| **iPS:437028** | **21-225_194G12** | NA | TCTGGAAGCAGCTCCAACATTGGGAATAATTATGTATCC | GACAATAATAAGCGACCCTCA | GGAACATGGGATAGCAGCCTGAGTGTTGGGGTA |
| | | | SEQ ID NO:3625 | SEQ ID NO:11637 | SEQ ID NO:19649 |
| | | AA | SGSSSNIGNNYVS | DNNKRPS | GTWDSSLSVGV |
| | | | SEQ ID NO:3626 | SEQ ID NO:11638 | SEQ ID NO:19650 |
| **iPS:437030** | **21-225_195E3** | NA | TCTGGAGATAAATTGGGGTATAGATCTGTTTGC | GAAGATAGCAAGCGACCCTCA | CAGGCGTGGGACAGTGTCACTGTGGTA |
| | | | SEQ ID NO:3627 | SEQ ID NO:11639 | SEQ ID NO:19651 |
| | | AA | SGDKLGYRSVC | EDSKRPS | QAWDSVTVV |
| | | | SEQ ID NO:3628 | SEQ ID NO:11640 | SEQ ID NO:19652 |
| **iPS:437032** | **21-225_195H6** | NA | TCTGGAAGCAGCTCCAACATCGGAAGTCATACTGTAAAC | AATAATTATCAGCGGCCCTCA | GCAACATGGGATGACAGCCTGAGTGTTTGGGTG |
| | | | SEQ ID NO:3629 | SEQ ID NO:11641 | SEQ ID NO:19653 |
| | | AA | SGSSSNIGSHTVN | NNYQRPS | ATWDDSLSVWV |
| | | | SEQ ID NO:3630 | SEQ ID NO:11642 | SEQ ID NO:19654 |
| **iPS:437034** | **21-225_195E9** | NA | TCAGGAGATAAATTGGGGAATAAATATGCTTAC | CAAGATAGGAAGCGGCCCTCA | CAGGCGTGGGACAGAGGAATTGTGGTA |
| | | | SEQ ID NO:3631 | SEQ ID NO:11643 | SEQ ID NO:19655 |
| | | AA | SGDKLGNKYAY | QDRKRPS | QAWDRGIVV |
| | | | SEQ ID NO:3632 | SEQ ID NO:11644 | SEQ ID NO:19656 |

FIGURE 49

| | | | | | |
|---|---|---|---|---|---|
| **iPS:437036** | **21-225_195H9** | NA | TCTGGAGGCAGCTCCAACATTGGGAATAATTATGTATCC | GACAATAATAAGCGACCCTCA | GGAACATGGGATCGCACCATGAGTGATTGGGTA |
| | | | SEQ ID NO:3633 | SEQ ID NO:11645 | SEQ ID NO:19657 |
| | | AA | SGGSSNIGNNYVS | DNNKRPS | GTWDRTMSDWV |
| | | | SEQ ID NO:3634 | SEQ ID NO:11646 | SEQ ID NO:19658 |
| **iPS:437040** | **21-225_196E7** | NA | GCTTCCAGCACTGGAGCAGTCACCAGTGCTTACTATCCAAAC | AGTACAAACAACAAACACTCC | CTGATTTTCTATGGTGGTGTACATGTGATA |
| | | | SEQ ID NO:3635 | SEQ ID NO:11647 | SEQ ID NO:19659 |
| | | AA | ASSTGAVTSAYYPN | STNNKHS | LIFYGGVHVI |
| | | | SEQ ID NO:3636 | SEQ ID NO:11648 | SEQ ID NO:19660 |
| **iPS:437042** | **21-225_197E8** | NA | TCTGGAAGCAGCTCCAACATTGGGAATAAATATGTATCC | GACAATAATAAGCGACCCTCA | GGAATATGGGATCGCAGTCTGAGTGTTATGGTG |
| | | | SEQ ID NO:3637 | SEQ ID NO:11649 | SEQ ID NO:19661 |
| | | AA | SGSSSNIGNKYVS | DNNKRPS | GIWDRSLSVMV |
| | | | SEQ ID NO:3638 | SEQ ID NO:11650 | SEQ ID NO:19662 |
| **iPS:437044** | **21-225_197F9** | NA | TCTGGAAGCAGCTCCAACATCGGAAGTAATACTGTAAAC | AGTAATAATCAGCGGCCCTCA | GCAGCATGGGATGACAGTATGAATGGTCCGGTG |
| | | | SEQ ID NO:3639 | SEQ ID NO:11651 | SEQ ID NO:19663 |
| | | AA | SGSSSNIGSNTVN | SNNQRPS | AAWDDSMNGPV |
| | | | SEQ ID NO:3640 | SEQ ID NO:11652 | SEQ ID NO:19664 |
| **iPS:437048** | **21-225_197B11** | NA | GCTTTCAGCACTGGATCAGTCACCAGTGGTTCCTATCCAAAC | AGTACAAACAACAAACACTCC | CTGCTCTACTATGGTGGTGCTCAGCTGGTG |
| | | | SEQ ID NO:3641 | SEQ ID NO:11653 | SEQ ID NO:19665 |
| | | AA | AFSTGSVTSGSYPN | STNNKHS | LLYYGGAQLV |
| | | | SEQ ID NO:3642 | SEQ ID NO:11654 | SEQ ID NO:19666 |

FIGURE 49

| | | | | | |
|---|---|---|---|---|---|
| **iPS:437050** | **21-225_197C11** | NA | GCTTCCAGCACTGGAGCAGTCACCAGTGCTTACTATCCAAAC | AGTACAAGCAACAAACACTCC | CTGATCTTCTATGGTGGTGTACATGTGATA |
| | | | SEQ ID NO:3643 | SEQ ID NO:11655 | SEQ ID NO:19667 |
| | | AA | ASSTGAVTSAYYPN | STSNKHS | LIFYGGVHVI |
| | | | SEQ ID NO:3644 | SEQ ID NO:11656 | SEQ ID NO:19668 |
| **iPS:437054** | **21-225_194G3** | NA | TCTGGAAGCAGCTCCAACATCGGGAATAATTATATATCC | GACAATAAAAAGCGACCCTCA | GGAACATGGGATAGCAGCCTGAGTGTTTGTGTC |
| | | | SEQ ID NO:3645 | SEQ ID NO:11657 | SEQ ID NO:19669 |
| | | AA | SGSSSNIGNNYIS | DNKKRPS | GTWDSSLSVCV |
| | | | SEQ ID NO:3646 | SEQ ID NO:11658 | SEQ ID NO:19670 |
| **iPS:437056** | **21-225_198B8** | NA | GTTCTTAGCACTGGAGCAGTCACCAGTGGTTCCTTTCCAAAC | AGTACAAGCAACAAACATTCC | ATGCTTTACAGTGGTGGAGCTCAGATGGTG |
| | | | SEQ ID NO:3647 | SEQ ID NO:11659 | SEQ ID NO:19671 |
| | | AA | VLSTGAVTSGSFPN | STSNKHS | MLYSGGAQMV |
| | | | SEQ ID NO:3648 | SEQ ID NO:11660 | SEQ ID NO:19672 |
| **iPS:437058** | **21-225_199F3** | NA | TCTGGAAGCAGCTCCAACATTGGGAATAATTATGTATCC | GACAATAATAAGCGCCCCTCA | GGAACATGGGATAGCAGCCTGAGTGCTTGTGTC |
| | | | SEQ ID NO:3649 | SEQ ID NO:11661 | SEQ ID NO:19673 |
| | | AA | SGSSSNIGNNYVS | DNNKRPS | GTWDSSLSACV |
| | | | SEQ ID NO:3650 | SEQ ID NO:11662 | SEQ ID NO:19674 |
| **iPS:437060** | **21-225_199C3** | NA | TCTGGAAGCAGCTCCAACATCGGAAGTAATACTGTAAAC | AGTAATAATCAGCGGCCCTCA | GCAGCATGGGATGACAGCCTGAATGGTCCGGTG |
| | | | SEQ ID NO:3651 | SEQ ID NO:11663 | SEQ ID NO:19675 |
| | | AA | SGSSSNIGSNTVN | SNNQRPS | AAWDDSLNGPV |
| | | | SEQ ID NO:3652 | SEQ ID NO:11664 | SEQ ID NO:19676 |

FIGURE 49

| | | | | | |
|---|---|---|---|---|---|
| **iPS:437062** | **21-225_200H1** | NA | GCTTCCAACACTGGAGCAGTCACCAGTGGTTCCTATCCAAAC | CATACAAACAACAAACACTCC | CTGATCTACTATGGTGGTGCTCAGCTGGTG |
| | | | SEQ ID NO:3653 | SEQ ID NO:11665 | SEQ ID NO:19677 |
| | | AA | ASNTGAVTSGSYPN | HTNNKHS | LIYYGGAQLV |
| | | | SEQ ID NO:3654 | SEQ ID NO:11666 | SEQ ID NO:19678 |
| **iPS:437064** | **21-225_200G8** | NA | TCTGGAAGCAGCTCCAACCTTGGAAATAATTTTGTATCC | GACAATTATAAGCGACCCTCA | GGAACTTGGGATAGCAGCCTGAATACTGGGGTA |
| | | | SEQ ID NO:3655 | SEQ ID NO:11667 | SEQ ID NO:19679 |
| | | AA | SGSSSNLGNNFVS | DNYKRPS | GTWDSSLNTGV |
| | | | SEQ ID NO:3656 | SEQ ID NO:11668 | SEQ ID NO:19680 |
| **iPS:437066** | **21-225_200G9** | NA | GCTTCCAACACTGGAGCAGTCACCAGTGGTTCCTATCCAAAT | CATACAGACAACAAACACTCC | CTGATCTACTATGGTGGTGCTCAGCTGGTG |
| | | | SEQ ID NO:3657 | SEQ ID NO:11669 | SEQ ID NO:19681 |
| | | AA | ASNTGAVTSGSYPN | HTDNKHS | LIYYGGAQLV |
| | | | SEQ ID NO:3658 | SEQ ID NO:11670 | SEQ ID NO:19682 |
| **iPS:437068** | **21-225_200A11** | NA | GCTTCCAGCACTGGAGCAGTCACCAGTGGTTACTATCCAAAC | AGTACAAACAACAAACACTCC | CTGCTCTATTATGGTGGTGCTCACCTGGCA |
| | | | SEQ ID NO:3659 | SEQ ID NO:11671 | SEQ ID NO:19683 |
| | | AA | ASSTGAVTSGYYPN | STNNKHS | LLYYGGAHLA |
| | | | SEQ ID NO:3660 | SEQ ID NO:11672 | SEQ ID NO:19684 |
| **iPS:437070** | **21-225_201G11** | NA | TCTGGAGATAAATTGGGGGATAGATTTGCTTGC | CAAGATAGCAAGCGGCCCTCA | CAGGCGTGGGACAGCAGCACTGTGGTA |
| | | | SEQ ID NO:3661 | SEQ ID NO:11673 | SEQ ID NO:19685 |
| | | AA | SGDKLGDRFAC | QDSKRPS | QAWDSSTVV |
| | | | SEQ ID NO:3662 | SEQ ID NO:11674 | SEQ ID NO:19686 |

FIGURE 49

| | | | | | |
|---|---|---|---|---|---|
| **iPS:437074** | **21-225_203B2** | NA | GGGGGAAACAACATTGGAAGAAAAAATGTGCAC | AGGGATAGCGACCGGCCCTCT | CAGGTGTGGGACAGCGGCACTGCGGTA |
| | | | SEQ ID NO:3663 | SEQ ID NO:11675 | SEQ ID NO:19687 |
| | | AA | GGNNIGRKNVH | RDSDRPS | QVWDSGTAV |
| | | | SEQ ID NO:3664 | SEQ ID NO:11676 | SEQ ID NO:19688 |
| **iPS:437076** | **21-225_203G6** | NA | TCTGGAGATAAATTGGGGATAGATTTGCTTGC | CAAGATAACAAGCGGCCCTCA | CAGGCGTGGGACAGCAGCACTGTGGTA |
| | | | SEQ ID NO:3665 | SEQ ID NO:11677 | SEQ ID NO:19689 |
| | | AA | SGDKLGDRFAC | QDNKRPS | QAWDSSTVV |
| | | | SEQ ID NO:3666 | SEQ ID NO:11678 | SEQ ID NO:19690 |
| **iPS:437082** | **21-225_205E12** | NA | GGGGGAAACAACATTGGAAGAAAAAATGTGCAC | AGGGATAGCGACCGGCCCTCT | CAGGTGTGGGACAGCGGCACTGCGGTA |
| | | | SEQ ID NO:3667 | SEQ ID NO:11679 | SEQ ID NO:19691 |
| | | AA | GGNNIGRKNVH | RDSDRPS | QVWDSGTAV |
| | | | SEQ ID NO:3668 | SEQ ID NO:11680 | SEQ ID NO:19692 |
| **iPS:437084** | **21-225_206B5** | NA | GGGGGAAACAACATTGGAAGAAAAAATGTGCAC | AGGGATAGCTACCGATCTTCT | CAGGATTGGGACAGCAGCACTGTGGTG |
| | | | SEQ ID NO:3669 | SEQ ID NO:11681 | SEQ ID NO:19693 |
| | | AA | GGNNIGRKNVH | RDSYRSS | QDWDSSTVV |
| | | | SEQ ID NO:3670 | SEQ ID NO:11682 | SEQ ID NO:19694 |
| **iPS:437086** | **21-225_209A8** | NA | TCTGGAAGCAGCTCCAACATTGGGAGTAATTTTTTATCC | GACAATAATAAGCGACCCTCA | GGAACATGGGATAGCAGCCTGAGTGCTGGGGTA |
| | | | SEQ ID NO:3671 | SEQ ID NO:11683 | SEQ ID NO:19695 |
| | | AA | SGSSSNIGSNFLS | DNNKRPS | GTWDSSLSAGV |
| | | | SEQ ID NO:3672 | SEQ ID NO:11684 | SEQ ID NO:19696 |
| **iPS:437088** | **21-225_209H10** | NA | GGGGGAAACAACATTGGAAGAAAAAATGTGCAC | AGGGATAGCTACCGGTCTTCT | CAGGATTGGGACAGCAGCACTGTGGTG |
| | | | SEQ ID NO:3673 | SEQ ID NO:11685 | SEQ ID NO:19697 |
| | | AA | GGNNIGRKNVH | RDSYRSS | QDWDSSTVV |
| | | | SEQ ID NO:3674 | SEQ ID NO:11686 | SEQ ID NO:19698 |

FIGURE 49

| | | | | | |
|---|---|---|---|---|---|
| **iPS:437090** | **21-225_210F11** | NA | GCTTTCAGCACTGGAGCAGTCACCAGTGGTAATTATCCAAAC | AGTACAAGCAACAAACACTCC | CTGCTCTACTATGGTGGTGCTCAGCTGGTG |
| | | | SEQ ID NO:3675 | SEQ ID NO:11687 | SEQ ID NO:19699 |
| | | AA | AFSTGAVTSGNYPN | STSNKHS | LLYYGGAQLV |
| | | | SEQ ID NO:3676 | SEQ ID NO:11688 | SEQ ID NO:19700 |
| **iPS:437092** | **21-225_210B12** | NA | ACTGGAACCAGCAGTGACGTTGGTGGTTATAACTATGTCTCC | GAGGTCAGGAATCGGCCCTCA | AGCTCATATACCAGCAGCCGCACTCTGGTA |
| | | | SEQ ID NO:3677 | SEQ ID NO:11689 | SEQ ID NO:19701 |
| | | AA | TGTSSDVGGYNYVS | EVRNRPS | SSYTSSRTLV |
| | | | SEQ ID NO:3678 | SEQ ID NO:11690 | SEQ ID NO:19702 |
| **iPS:437094** | **21-225_210D12** | NA | ACTGGAACCAGCAGTGACGTTGGTGGTTATAATTATGTCTCC | GAGGTCAGTAATCGGCCCTCA | AACTCATATACAAGCAGCATCACTTGGGTG |
| | | | SEQ ID NO:3679 | SEQ ID NO:11691 | SEQ ID NO:19703 |
| | | AA | TGTSSDVGGYNYVS | EVSNRPS | NSYTSSITWV |
| | | | SEQ ID NO:3680 | SEQ ID NO:11692 | SEQ ID NO:19704 |
| **iPS:437096** | **21-225_210E12** | NA | ACTGGAACCAGCAGTGACGTTGGTGGTTATAACTATGTCTCC | GAGGTCAGTAATCGGCCCTCA | GGCTCATATGTAAAAGGCATCACTTGGGTG |
| | | | SEQ ID NO:3681 | SEQ ID NO:11693 | SEQ ID NO:19705 |
| | | AA | TGTSSDVGGYNYVS | EVSNRPS | GSYVKGITWV |
| | | | SEQ ID NO:3682 | SEQ ID NO:11694 | SEQ ID NO:19706 |
| **iPS:437098** | **21-225_211C1** | NA | ACTGGAACCAGTAGTGACGTTGGTAGTTATAACTATGTCTCC | GAGGTCAGTAATCGGCCCTCA | AACTCATATACAAGCAGCATCACTTGGGTG |
| | | | SEQ ID NO:3683 | SEQ ID NO:11695 | SEQ ID NO:19707 |
| | | AA | TGTSSDVGSYNYVS | EVSNRPS | NSYTSSITWV |
| | | | SEQ ID NO:3684 | SEQ ID NO:11696 | SEQ ID NO:19708 |

FIGURE 49

| | | | | | |
|---|---|---|---|---|---|
| **iPS:437100** | **21-225_211H2** | NA | GGGGGAAACAACATTGGACGTAGAAATGTGCAC | AGAGATCGCGACCGGCCCTCT | CAGGTGTGGGACAGCAGTACTGCGGTG |
| | | | SEQ ID NO:3685 | SEQ ID NO:11697 | SEQ ID NO:19709 |
| | | AA | GGNNIGRRNVH | RDRDRPS | QVWDSSTAV |
| | | | SEQ ID NO:3686 | SEQ ID NO:11698 | SEQ ID NO:19710 |
| **iPS:437102** | **21-225_211E5** | NA | TCTGGAGATGCATTGCCAAAGCAATATGCTTAT | AAAGACAGTGCGAGGCCCTCA | CAATTAGTGTACAGCAGTGATACTTATGTC |
| | | | SEQ ID NO:3687 | SEQ ID NO:11699 | SEQ ID NO:19711 |
| | | AA | SGDALPKQYAY | KDSARPS | QLVYSSDTYV |
| | | | SEQ ID NO:3688 | SEQ ID NO:11700 | SEQ ID NO:19712 |
| **iPS:437104** | **21-225_211G5** | NA | ACTGGAACCAGCAGTGACGTTGGTGGTTATAACTATGTCTCC | GAGGTCAGTAATCGGCCCTCA | AACTCATATACAAGAAGCATCACTTGGGTG |
| | | | SEQ ID NO:3689 | SEQ ID NO:11701 | SEQ ID NO:19713 |
| | | AA | TGTSSDVGGYNYVS | EVSNRPS | NSYTRSITWV |
| | | | SEQ ID NO:3690 | SEQ ID NO:11702 | SEQ ID NO:19714 |
| **iPS:437106** | **21-225_211H7** | NA | GCTTTCAGCACTGGAGCAGTCACCAGTGGTAACTATCCAAGT | AGTACAAGCAACAGACACTCC | CTGCTCTACTATGGTGGTGCTCAGCTGGTG |
| | | | SEQ ID NO:3691 | SEQ ID NO:11703 | SEQ ID NO:19715 |
| | | AA | AFSTGAVTSGNYPS | STSNRHS | LLYYGGAQLV |
| | | | SEQ ID NO:3692 | SEQ ID NO:11704 | SEQ ID NO:19716 |
| **iPS:437108** | **21-225_211C9** | NA | GGTTCCAGCACTGGATCAGTCACCAGTGGTTACTTTCCAAAC | AGTACAAACAACAAGCACTCC | CTGCTCTACTATGGTGGTGCTCAGCTGGCA |
| | | | SEQ ID NO:3693 | SEQ ID NO:11705 | SEQ ID NO:19717 |
| | | AA | GSSTGSVTSGYFPN | STNNKHS | LLYYGGAQLA |
| | | | SEQ ID NO:3694 | SEQ ID NO:11706 | SEQ ID NO:19718 |

FIGURE 49

| | | | | | |
|---|---|---|---|---|---|
| **iPS:437110** | **21-225_211E9** | NA | GCTTCCAGCACTGGAGCAGTCACCAGTGGTAACTATCCAAAC | AGTACAATCAACAAACACTCC | CTGCTCTACTATGGTGGTGCTCAGCTGGCA |
| | | | SEQ ID NO:3695 | SEQ ID NO:11707 | SEQ ID NO:19719 |
| | | AA | ASSTGAVTSGNYPN | STINKHS | LLYYGGAQLA |
| | | | SEQ ID NO:3696 | SEQ ID NO:11708 | SEQ ID NO:19720 |
| **iPS:437112** | **21-225_212C2** | NA | ACTGGAACCAGCAGTGACGTTGGTGGTTATAACTATGTCTCC | GAGGTCAGAAATCGGCCCTCA | AGCTCATATACACGCAGCATCACTTGGGTG |
| | | | SEQ ID NO:3697 | SEQ ID NO:11709 | SEQ ID NO:19721 |
| | | AA | TGTSSDVGGYNYVS | EVRNRPS | SSYTRSITWV |
| | | | SEQ ID NO:3698 | SEQ ID NO:11710 | SEQ ID NO:19722 |
| **iPS:437114** | **21-225_212A4** | NA | ACTGGAACCAGCAGTGACGTTGGTGGTTATAACTATGTCTCC | GAGGTCAGTAATCGGCCCTCA | GGCTCATATGTAAAAGGCATCACTTGGGTG |
| | | | SEQ ID NO:3699 | SEQ ID NO:11711 | SEQ ID NO:19723 |
| | | AA | TGTSSDVGGYNYVS | EVSNRPS | GSYVKGITWV |
| | | | SEQ ID NO:3700 | SEQ ID NO:11712 | SEQ ID NO:19724 |
| **iPS:437116** | **21-225_212F6** | NA | ACTGGAACCAGTAGTGACGTTGGTGGTTATAACTATGTCTCC | GAGGTCAGTAATCGGCCCTCA | AACTCATATACAAGCAGCATCACTTGGGTG |
| | | | SEQ ID NO:3701 | SEQ ID NO:11713 | SEQ ID NO:19725 |
| | | AA | TGTSSDVGGYNYVS | EVSNRPS | NSYTSSITWV |
| | | | SEQ ID NO:3702 | SEQ ID NO:11714 | SEQ ID NO:19726 |
| **iPS:437118** | **21-225_212G7** | NA | ACTGGAACCAGCAGTGACGTTGGTGGTTATAATTATGTCTCC | GAGGTCAGTAATCGGCCCTCA | AACTCATATACAAGCAGCATCACTTGGGTG |
| | | | SEQ ID NO:3703 | SEQ ID NO:11715 | SEQ ID NO:19727 |
| | | AA | TGTSSDVGGYNYVS | EVSNRPS | NSYTSSITWV |
| | | | SEQ ID NO:3704 | SEQ ID NO:11716 | SEQ ID NO:19728 |

FIGURE 49

| | | | | | |
|---|---|---|---|---|---|
| **iPS:437120** | **21-225_212A9** | NA | GCTTCCAGCACTGGAGCAGTCACCAGTGGTTACTATCCAAAC | AGTACAAACAACAAACACTCC | CTGCTCTACTATGGTGGTGCTCAGGTGGGA |
| | | | SEQ ID NO:3705 | SEQ ID NO:11717 | SEQ ID NO:19729 |
| | | AA | ASSTGAVTSGYYPN | STNNKHS | LLYYGGAQVG |
| | | | SEQ ID NO:3706 | SEQ ID NO:11718 | SEQ ID NO:19730 |
| **iPS:437124** | **21-225_212H12** | NA | GCTTCCAGCACTGGAGCAGTCACCAGTGGTTACTATCCAAAC | AGTACAAGCAACAAACACTCC | CTGCTCTACTATGGTGGTGCTCATGTGGTA |
| | | | SEQ ID NO:3707 | SEQ ID NO:11719 | SEQ ID NO:19731 |
| | | AA | ASSTGAVTSGYYPN | STSNKHS | LLYYGGAHVV |
| | | | SEQ ID NO:3708 | SEQ ID NO:11720 | SEQ ID NO:19732 |
| **iPS:437128** | **21-225_213G3** | NA | ACTGGAACCAGCAGTGACGTTGGTGGTTATAACTATGTCTCC | GAGGTCAGGAATCGGCCCTCA | AACTCATATACACGCAGCATCACTTGGGTG |
| | | | SEQ ID NO:3709 | SEQ ID NO:11721 | SEQ ID NO:19733 |
| | | AA | TGTSSDVGGYNYVS | EVRNRPS | NSYTRSITWV |
| | | | SEQ ID NO:3710 | SEQ ID NO:11722 | SEQ ID NO:19734 |
| **iPS:437130** | **21-225_213D5** | NA | ACTGGAACCAGCAGTGACGTTGGTGGTTATAACTATGTCTCC | GAGGTCCGTAATCGGCCCTCA | TGCTCATATACAAGAAGAATCACTTGGGTG |
| | | | SEQ ID NO:3711 | SEQ ID NO:11723 | SEQ ID NO:19735 |
| | | AA | TGTSSDVGGYNYVS | EVRNRPS | CSYTRRITWV |
| | | | SEQ ID NO:3712 | SEQ ID NO:11724 | SEQ ID NO:19736 |
| **iPS:437132** | **21-225_213F5** | NA | GGTTCCAGCACTGGATCAGTCACCAGTGGTTACTTTCCAAAC | AGTACAAACAACAAGCACTCC | CTGCTCTACTTTGGTGGTGCTCAGCTGGCA |
| | | | SEQ ID NO:3713 | SEQ ID NO:11725 | SEQ ID NO:19737 |
| | | AA | GSSTGSVTSGYFPN | STNNKHS | LLYFGGAQLA |
| | | | SEQ ID NO:3714 | SEQ ID NO:11726 | SEQ ID NO:19738 |

## FIGURE 49

| | | | | | |
|---|---|---|---|---|---|
| **iPS:437134** | **21-225_213A7** | NA | ACTGGAACCAGCAGTGACGTTGGTGGTTATAACTATGTCTCC | GAGGTCAGGAATCGGCCCTCA | AGCTCATATACCAGCAGCCGCACTCTGGTA |
| | | | SEQ ID NO:3715 | SEQ ID NO:11727 | SEQ ID NO:19739 |
| | | AA | TGTSSDVGGYNYVS | EVRNRPS | SSYTSSRTLV |
| | | | SEQ ID NO:3716 | SEQ ID NO:11728 | SEQ ID NO:19740 |
| **iPS:437136** | **21-225_214H3** | NA | GCTTCCAGCACTGGAGCAGTCACCAGTGGTTACTATCCAAAC | AGTACAAGCAACAAACACTCC | CTGCTCTACTATGGTGGTGCTCATGTGGTA |
| | | | SEQ ID NO:3717 | SEQ ID NO:11729 | SEQ ID NO:19741 |
| | | AA | ASSTGAVTSGYYPN | STSNKHS | LLYYGGAHVV |
| | | | SEQ ID NO:3718 | SEQ ID NO:11730 | SEQ ID NO:19742 |
| **iPS:437138** | **21-225_214D8** | NA | TCTGGAAGCAGCTCCAACATTGGGAATAATTATGTATCC | GACAATAATAAGCGACCCTCA | GGAGCATGGGATAGCAGCCTGAGTGCTGTGGTA |
| | | | SEQ ID NO:3719 | SEQ ID NO:11731 | SEQ ID NO:19743 |
| | | AA | SGSSSNIGNNYVS | DNNKRPS | GAWDSSLSAVV |
| | | | SEQ ID NO:3720 | SEQ ID NO:11732 | SEQ ID NO:19744 |
| **iPS:437140** | **21-225_214E12** | NA | GCTTCCAGCACTGGAGCAGTCACCAGTGGCTACTATCCAAAC | AGTACAAGCAATAAACACTCC | CTGCTCTACTGTGATGGTGCCCAGCTGGTG |
| | | | SEQ ID NO:3721 | SEQ ID NO:11733 | SEQ ID NO:19745 |
| | | AA | ASSTGAVTSGYYPN | STSNKHS | LLYCDGAQLV |
| | | | SEQ ID NO:3722 | SEQ ID NO:11734 | SEQ ID NO:19746 |
| **iPS:437142** | **21-225_215A3** | NA | GCTTCCAGCACTGAAGCCGTCACCAGTGGTAACTATCCAAGC | AGTACAAGCAACAAACACTCC | CTGCTCTACTATGGTGGCGCTCAGCTGGCA |
| | | | SEQ ID NO:3723 | SEQ ID NO:11735 | SEQ ID NO:19747 |
| | | AA | ASSTEAVTSGNYPS | STSNKHS | LLYYGGAQLA |
| | | | SEQ ID NO:3724 | SEQ ID NO:11736 | SEQ ID NO:19748 |

FIGURE 49

| | | | | | |
|---|---|---|---|---|---|
| **iPS:437144** | **21-225_215B3** | NA | TCTGGAGATAAATTGGGGGATAAATTTGCTTGC | CAAGATAGCAAGCGGCCCTCA | CAGGCGTGGGACAGCAGCACTGTGGTA |
| | | | SEQ ID NO:3725 | SEQ ID NO:11737 | SEQ ID NO:19749 |
| | | AA | SGDKLGDKFAC | QDSKRPS | QAWDSSTVV |
| | | | SEQ ID NO:3726 | SEQ ID NO:11738 | SEQ ID NO:19750 |
| **iPS:437146** | **21-225_215D3** | NA | ACTGGAACCAGCAGTGACATTGGTGGTTATAACTATGTCTCC | GAGGTCAGTAATCGGCCCTCA | AACTCATATAAAAGGGGCAGCACTTGGGTG |
| | | | SEQ ID NO:3727 | SEQ ID NO:11739 | SEQ ID NO:19751 |
| | | AA | TGTSSDIGGYNYVS | EVSNRPS | NSYKRGSTWV |
| | | | SEQ ID NO:3728 | SEQ ID NO:11740 | SEQ ID NO:19752 |
| **iPS:437148** | **21-225_215H3** | NA | GCTTCCAGCACTGGAGCAGTCACCAGTGGTTACTATCCAAAC | AGTACAAACAACAAACACTCC | CTGCTCTACTATGGTGGTGCTCAGGTGGGA |
| | | | SEQ ID NO:3729 | SEQ ID NO:11741 | SEQ ID NO:19753 |
| | | AA | ASSTGAVTSGYYPN | STNNKHS | LLYYGGAQVG |
| | | | SEQ ID NO:3730 | SEQ ID NO:11742 | SEQ ID NO:19754 |
| **iPS:437150** | **21-225_216A3** | NA | ACTGGAACCAGCAGTGACGTTGGTGGTTATAATTATGTCTCC | GAGGTCAGTAATCGGCCCTCA | AACTCATATACAAGCAGCATCACTTGGGTG |
| | | | SEQ ID NO:3731 | SEQ ID NO:11743 | SEQ ID NO:19755 |
| | | AA | TGTSSDVGGYNYVS | EVSNRPS | NSYTSSITWV |
| | | | SEQ ID NO:3732 | SEQ ID NO:11744 | SEQ ID NO:19756 |
| **iPS:437154** | **21-225_216A7** | NA | GCTTCCAGCACTGGAGCAGTCACCAGTGGTTACTATCCAAAC | AGTACAAACAACAAACACTCC | CTGCTCTACTATGGTGGTGCTCAGGTGGGA |
| | | | SEQ ID NO:3733 | SEQ ID NO:11745 | SEQ ID NO:19757 |
| | | AA | ASSTGAVTSGYYPN | STNNKHS | LLYYGGAQVG |
| | | | SEQ ID NO:3734 | SEQ ID NO:11746 | SEQ ID NO:19758 |

FIGURE 49

| | | | | | |
|---|---|---|---|---|---|
| **iPS:437158** | **21-225_216H11** | NA | GCTTCCAGCACTGGAGCAGTCACCAGTGGCTACTATCCAAAC | AGTACAAGCAATAAACACTCC | CTGCTCTACTGTGATGGTGCTCAGCTGGTG |
| | | | SEQ ID NO:3735 | SEQ ID NO:11747 | SEQ ID NO:19759 |
| | | AA | ASSTGAVTSGYYPN | STSNKHS | LLYCDGAQLV |
| | | | SEQ ID NO:3736 | SEQ ID NO:11748 | SEQ ID NO:19760 |
| **iPS:437160** | **21-225_216B12** | NA | GGGGGAGACAACATTAGAAGAAGAAATGTGCAC | AGGGATAGCAACCGGCCCTCT | CAGGTGTGGGACAGCAGCACTGGGGTG |
| | | | SEQ ID NO:3737 | SEQ ID NO:11749 | SEQ ID NO:19761 |
| | | AA | GGDNIRRRNVH | RDSNRPS | QVWDSSTGV |
| | | | SEQ ID NO:3738 | SEQ ID NO:11750 | SEQ ID NO:19762 |
| **iPS:437162** | **21-225_217B2** | NA | ACTGGAACCAGCAGTGACGTTGGTGGTTATAACTATGTCTCC | GAGGTCAGTAATCGGCCCTCA | GGCTCATATGTAAAAGGCATCACTTGGGTG |
| | | | SEQ ID NO:3739 | SEQ ID NO:11751 | SEQ ID NO:19763 |
| | | AA | TGTSSDVGGYNYVS | EVSNRPS | GSYVKGITWV |
| | | | SEQ ID NO:3740 | SEQ ID NO:11752 | SEQ ID NO:19764 |
| **iPS:437164** | **21-225_217C6** | NA | TCTGGAGATGCATTGCCAAAGCAATATGCTTAT | AAAGACAGTGAGAGGCCCTCA | CAATTAATAGTCAGCAGTGATACTTATGTC |
| | | | SEQ ID NO:3741 | SEQ ID NO:11753 | SEQ ID NO:19765 |
| | | AA | SGDALPKQYAY | KDSERPS | QLIVSSDTYV |
| | | | SEQ ID NO:3742 | SEQ ID NO:11754 | SEQ ID NO:19766 |
| **iPS:437166** | **21-225_217G11** | NA | TCTGGAGATGCATTGCCAAAACAATATGCTTAT | AAAGACAGTGAGAGGCCCTCA | CAATTAGTGTACAGCAGTGATACTTATGTC |
| | | | SEQ ID NO:3743 | SEQ ID NO:11755 | SEQ ID NO:19767 |
| | | AA | SGDALPKQYAY | KDSERPS | QLVYSSDTYV |
| | | | SEQ ID NO:3744 | SEQ ID NO:11756 | SEQ ID NO:19768 |
| **iPS:437168** | **21 225 218G4** | NA | TCTGGAAGCAGCTCCAACATTGGGAATAATTATGTATCC | GACAGTAATAAGCGACCCTCA | GGAACATGGGATAGCAGCCTGAATACTGTGGTA |

FIGURE 49

| | | | | | |
|---|---|---|---|---|---|
| | **21-225_218G4** | | SEQ ID NO:3745 | SEQ ID NO:11757 | SEQ ID NO:19769 |
| | | AA | SGSSSNIGNNYVS | DSNKRPS | GTWDSSLNTVV |
| | | | SEQ ID NO:3746 | SEQ ID NO:11758 | SEQ ID NO:19770 |
| **iPS:437170** | **21-225_218E5** | NA | TCTAGAGATGTATTGCCGAA GCAATATGCTTAT | AAAGACAGTGAGAGGCC CTCA | CAATTAGTTGTCAGCAGTGA TACTTATGTC |
| | | | SEQ ID NO:3747 | SEQ ID NO:11759 | SEQ ID NO:19771 |
| | | AA | SRDVLPKQYAY | KDSERPS | QLVVSSDTYV |
| | | | SEQ ID NO:3748 | SEQ ID NO:11760 | SEQ ID NO:19772 |
| **iPS:437172** | **21-225_219A7** | NA | ACTGGAACCAGCAGTGACGT TGGTGGTTATAATTATGTCT CC | GAGGTCAGAAATCGGCC CTCA | TGCTCATATACAAGGAGCAT CACTTGGGTG |
| | | | SEQ ID NO:3749 | SEQ ID NO:11761 | SEQ ID NO:19773 |
| | | AA | TGTSSDVGGYNYVS | EVRNRPS | CSYTRSITWV |
| | | | SEQ ID NO:3750 | SEQ ID NO:11762 | SEQ ID NO:19774 |
| **iPS:437182** | **21-225_221H2** | NA | ACTGGAACCAGCAGTGACGT TGGTGGTTATAACTATGTCT CC | GAGGTCAGGAATCGGCC CTCA | AACTCATATACACGCAGCAT CACTTGGGTG |
| | | | SEQ ID NO:3751 | SEQ ID NO:11763 | SEQ ID NO:19775 |
| | | AA | TGTSSDVGGYNYVS | EVRNRPS | NSYTRSITWV |
| | | | SEQ ID NO:3752 | SEQ ID NO:11764 | SEQ ID NO:19776 |
| **iPS:437184** | **21-225_221G4** | NA | ACTGGAACCAGCAGTGACGT TGGTGGTTATAACTATGTCT CC | GAGGTCAGGAATCGGCC CTCA | AACTCATATACACGCAGCAT CACTTGGGTG |
| | | | SEQ ID NO:3753 | SEQ ID NO:11765 | SEQ ID NO:19777 |
| | | AA | TGTSSDVGGYNYVS | EVRNRPS | NSYTRSITWV |
| | | | SEQ ID NO:3754 | SEQ ID NO:11766 | SEQ ID NO:19778 |
| **iPS:437186** | **21-225_224H2** | NA | TCTGGAGATAATTTGGGGGT TAAATATACTTAC | CAAGATAGCAAGCGGCC CTCA | CAGGCGTGGGACAGCAGCAC TGTGGTA |
| | | | SEQ ID NO:3755 | SEQ ID NO:11767 | SEQ ID NO:19779 |
| | | AA | SGDNLGVKYTY | QDSKRPS | QAWDSSTVV |

FIGURE 49

| | | | | | |
|---|---|---|---|---|---|
| | | | SEQ ID NO:3756 | SEQ ID NO:11768 | SEQ ID NO:19780 |
| **iPS:437188** | **21-225_224B11** | NA | ACTGGGAGCAGCTCCAACATCGGGGCAGGTTATGATGTACAC | GGTAACAGCAATCGGCCCTCA | CAGTCCTATGACAACAGCCTGAGTGGTGTG |
| | | | SEQ ID NO:3757 | SEQ ID NO:11769 | SEQ ID NO:19781 |
| | | AA | TGSSSNIGAGYDVH | GNSNRPS | QSYDNSLSGV |
| | | | SEQ ID NO:3758 | SEQ ID NO:11770 | SEQ ID NO:19782 |
| **iPS:437190** | **21-225_225A9** | NA | TCTGGAGATAAATTGGGGAATAAATATGCTTGC | CAAGATAGCAAGCGGCCCTCA | CAGGCGTGGGACAGCAACACTGCATGTGTC |
| | | | SEQ ID NO:3759 | SEQ ID NO:11771 | SEQ ID NO:19783 |
| | | AA | SGDKLGNKYAC | QDSKRPS | QAWDSNTACV |
| | | | SEQ ID NO:3760 | SEQ ID NO:11772 | SEQ ID NO:19784 |
| **iPS:437192** | **21-225_225E9** | NA | TCTGGAGATAATTTGGGGAATAGATATGCTTGC | CAAGATCGCAAGCGGCCCTCA | CAGGCGTGGGACAGCAGAACTGCTGTGGTA |
| | | | SEQ ID NO:3761 | SEQ ID NO:11773 | SEQ ID NO:19785 |
| | | AA | SGDNLGNRYAC | QDRKRPS | QAWDSRTAVV |
| | | | SEQ ID NO:3762 | SEQ ID NO:11774 | SEQ ID NO:19786 |
| **iPS:437194** | **21-225_226B2** | NA | TCTGGAGATACATTGGGGGGTAAATATGCTTGG | CAAGATCGCAAGCGGCCCTCA | CAGGCGTGGGACAACGGCGCTGCGGTT |
| | | | SEQ ID NO:3763 | SEQ ID NO:11775 | SEQ ID NO:19787 |
| | | AA | SGDTLGGKYAW | QDRKRPS | QAWDNGAAV |
| | | | SEQ ID NO:3764 | SEQ ID NO:11776 | SEQ ID NO:19788 |
| **iPS:437196** | **21-225_226B7** | NA | TCTGGAGATGCATTGCCAAGGCATTATGTTTAT | AAAGACAGTGAGAGGCCCTCA | CAATCAGCAGACAGCAGTGGTACTTATGTC |
| | | | SEQ ID NO:3765 | SEQ ID NO:11777 | SEQ ID NO:19789 |
| | | AA | SGDALPRHYVY | KDSERPS | QSADSSGTYV |
| | | | SEQ ID NO:3766 | SEQ ID NO:11778 | SEQ ID NO:19790 |
| **iPS:437198** | **21 225 226F8** | NA | ACTGGGAGCAGCTCCAACATCGGGGCAGGTTATGATGTACAC | GGTAACAGCAATCGGCCCTCA | CAGTCCTATGACAACAGCCTGAGTGGTGTG |

## FIGURE 49

| | | | | | |
|---|---|---|---|---|---|
| | 21-225_226F8 | | SEQ ID NO:3767 | SEQ ID NO:11779 | SEQ ID NO:19791 |
| | | AA | TGSSSNIGAGYDVH | GNSNRPS | QSYDNSLSGV |
| | | | SEQ ID NO:3768 | SEQ ID NO:11780 | SEQ ID NO:19792 |
| **iPS:437200** | 21-225_226A10 | NA | TCTGGAGATACATTGGGGGGTAAATATGCTTGG | CAAGATCGCAAGCGGCCCTCA | CAGGCGTGGGACAACGGCGCTGCGGTT |
| | | | SEQ ID NO:3769 | SEQ ID NO:11781 | SEQ ID NO:19793 |
| | | AA | SGDTLGGKYAW | QDRKRPS | QAWDNGAAV |
| | | | SEQ ID NO:3770 | SEQ ID NO:11782 | SEQ ID NO:19794 |
| **iPS:437202** | 21-225_227D3 | NA | ACTGGGAGCAGCTCCAACATCGGGGCAGGTTATGATGTACAC | GGTAACAGCAATCGGCCCTCA | CAGTCCTATGACAACAGCCTGAGTGGTGTG |
| | | | SEQ ID NO:3771 | SEQ ID NO:11783 | SEQ ID NO:19795 |
| | | AA | TGSSSNIGAGYDVH | GNSNRPS | QSYDNSLSGV |
| | | | SEQ ID NO:3772 | SEQ ID NO:11784 | SEQ ID NO:19796 |
| **iPS:437204** | 21-225_227E5 | NA | TCTGGAGATAAATTGGGGGAAAAATATGCTTGC | CAAGATAGGAAGCGGCCCTCA | CAGGCGTGGGTCAACAACACTATGATA |
| | | | SEQ ID NO:3773 | SEQ ID NO:11785 | SEQ ID NO:19797 |
| | | AA | SGDKLGEKYAC | QDRKRPS | QAWVNNTMI |
| | | | SEQ ID NO:3774 | SEQ ID NO:11786 | SEQ ID NO:19798 |
| **iPS:437208** | 21-225_227C10 | NA | ACTGGGAGCAGCTCCAACATCGGGGCAGGTTATGATGTACAC | GGTAACAGCAATCGGCCCTCA | CAGTCCTATGACAACAACCTGAGTGGTGTG |
| | | | SEQ ID NO:3775 | SEQ ID NO:11787 | SEQ ID NO:19799 |
| | | AA | TGSSSNIGAGYDVH | GNSNRPS | QSYDNNLSGV |
| | | | SEQ ID NO:3776 | SEQ ID NO:11788 | SEQ ID NO:19800 |
| **iPS:437210** | 21-225_227E12 | NA | TCTGGAGATAAATTGGGGGATAAATATGTTTGT | CAAGATAGCAAGCGGCCCTCA | CAGGCGTGGAACAGCAGCAATGTGGTA |
| | | | SEQ ID NO:3777 | SEQ ID NO:11789 | SEQ ID NO:19801 |
| | | AA | SGDKLGDKYVC | QDSKRPS | QAWNSSNVV |
| | | | SEQ ID NO:3778 | SEQ ID NO:11790 | SEQ ID NO:19802 |

FIGURE 49

| | | | | | |
|---|---|---|---|---|---|
| **iPS:437214** | **21-225_48B12** | NA | TCTGGAGATGCATTGCCAAAAAAATATGCTTAT | GAGGACAGCAAACGACCCTCC | AACTCAACAGACAGCAGTGGTAATCATGTGGTA |
| | | | SEQ ID NO:3779 | SEQ ID NO:11791 | SEQ ID NO:19803 |
| | | AA | SGDALPKKYAY | EDSKRPS | NSTDSSGNHVV |
| | | | SEQ ID NO:3780 | SEQ ID NO:11792 | SEQ ID NO:19804 |
| **iPS:437216** | **21-225_51D5** | NA | CGGGCGAGTCAGGGCATTAACAATTATTTAGCC | GCTGCATCCAGTTTGCGAAGT | CAACAGTATTATAGTTACCCATTCACT |
| | | | SEQ ID NO:3781 | SEQ ID NO:11793 | SEQ ID NO:19805 |
| | | AA | RASQGINNYLA | AASSLRS | QQYYSYPFT |
| | | | SEQ ID NO:3782 | SEQ ID NO:11794 | SEQ ID NO:19806 |
| **iPS:437220** | **21-225_55H6** | NA | CGGGCAAGTCAGGGCATTAGAAACGATTTAGGC | GGTGCATCCAGTTTGCAAAGT | CTACAGCGTGATAGTTACCCGTTCACT |
| | | | SEQ ID NO:3783 | SEQ ID NO:11795 | SEQ ID NO:19807 |
| | | AA | RASQGIRNDLG | GASSLQS | LQRDSYPFT |
| | | | SEQ ID NO:3784 | SEQ ID NO:11796 | SEQ ID NO:19808 |
| **iPS:437224** | **21-225_56H1** | NA | CGGGCGAGTCAGGGCATTAGCCATTATTTAGCC | GCTGCATCCGGTTTGCAAAGT | CAACAATATCAGAATTACCCCTTCACT |
| | | | SEQ ID NO:3785 | SEQ ID NO:11797 | SEQ ID NO:19809 |
| | | AA | RASQGISHYLA | AASGLQS | QQYQNYPFT |
| | | | SEQ ID NO:3786 | SEQ ID NO:11798 | SEQ ID NO:19810 |
| **iPS:437226** | **21-225_57C2** | NA | AGGTCTAGTCAAAGCCTCGTATACAGTGATGGAAACACCTACTTGAAT | GAGGTTTCTAACTGGGACTCT | GTGCAAGGTACACACTGGCCTCGGACG |
| | | | SEQ ID NO:3787 | SEQ ID NO:11799 | SEQ ID NO:19811 |
| | | AA | RSSQSLVYSDGNTYLN | EVSNWDS | VQGTHWPRT |
| | | | SEQ ID NO:3788 | SEQ ID NO:11800 | SEQ ID NO:19812 |
| **iPS:437228** | **21-225_60C11** | NA | AGGGCCAGTCAGAGTGTTAGCAACGACTTAGCC | GGTGCATCCACCAGGGCCACT | CAGCAGTATAGTAACTGGCCATTCACT |
| | | | SEQ ID NO:3789 | SEQ ID NO:11801 | SEQ ID NO:19813 |
| | | AA | RASQSVSNDLA | GASTRAT | QQYSNWPFT |

FIGURE 49

| | | | | | |
|---|---|---|---|---|---|
| | | | SEQ ID NO:3790 | SEQ ID NO:11802 | SEQ ID NO:19814 |
| **iPS:437230** | **21-225_62H10** | NA | CGGGCAAGTCAGAGCATTACCAGCTATTTAAAT | ACTGCATCCAGTTTGCAAAGT | CAACAGAGTCACAGTTTCCCATTCACT |
| | | | SEQ ID NO:3791 | SEQ ID NO:11803 | SEQ ID NO:19815 |
| | | AA | RASQSITSYLN | TASSLQS | QQSHSFPFT |
| | | | SEQ ID NO:3792 | SEQ ID NO:11804 | SEQ ID NO:19816 |
| **iPS:437232** | **21-225_63E1** | NA | CGTGCGAGTCAGGGTATTAGCAGCTACTTAGCC | GCTGCATCCAGTTTGCAAAGT | CAACAGGCTAACAGTTTCCCGCTCACT |
| | | | SEQ ID NO:3793 | SEQ ID NO:11805 | SEQ ID NO:19817 |
| | | AA | RASQGISSYLA | AASSLQS | QQANSFPLT |
| | | | SEQ ID NO:3794 | SEQ ID NO:11806 | SEQ ID NO:19818 |
| **iPS:437234** | **21-225_64E2** | NA | CGGGCGAGTCAGGGTATTAGCAGGTGGTTAGCC | GCTGCATCCAGTTTGCAAAGT | CAACAGGCTAACAGTTTCCCATTCACT |
| | | | SEQ ID NO:3795 | SEQ ID NO:11807 | SEQ ID NO:19819 |
| | | AA | RASQGISRWLA | AASSLQS | QQANSFPFT |
| | | | SEQ ID NO:3796 | SEQ ID NO:11808 | SEQ ID NO:19820 |
| **iPS:437248** | **21-225_97H3** | NA | AGGTCTAGTCAGAGCCTCCTGCATAGTAATGGACACAACTATTTGGAT | TTGGGTTCTAATCGGGCCTCC | ATGCAACCTCTACAAACTCCGTTCACT |
| | | | SEQ ID NO:3797 | SEQ ID NO:11809 | SEQ ID NO:19821 |
| | | AA | RSSQSLLHSNGHNYLD | LGSNRAS | MQPLQTPFT |
| | | | SEQ ID NO:3798 | SEQ ID NO:11810 | SEQ ID NO:19822 |
| **iPS:437250** | **21-225_148C6** | NA | AGGTCTAGTCAAAGCCTCGTATACAGTGATGGAAACACCTACTTGAAT | AAGGTTTCTAACTGGGACTCT | ATGCAAGGTACACACTGGTCGCTCACT |
| | | | SEQ ID NO:3799 | SEQ ID NO:11811 | SEQ ID NO:19823 |
| | | AA | RSSQSLVYSDGNTYLN | KVSNWDS | MQGTHWSLT |
| | | | SEQ ID NO:3800 | SEQ ID NO:11812 | SEQ ID NO:19824 |

FIGURE 49

| | | | | | |
|---|---|---|---|---|---|
| **iPS:437252** | **21-225_148H11** | NA | AGGTCTAGTCAAAGCCTCGTATACAGTGATGGAAACACCTACTTGAAT | AAGGTTTCTAACTGGGACTCT | ATGCAAGGTACACACTGGTTGCTCACT |
| | | | SEQ ID NO:3801 | SEQ ID NO:11813 | SEQ ID NO:19825 |
| | | AA | RSSQSLVYSDGNTYLN | KVSNWDS | MQGTHWLLT |
| | | | SEQ ID NO:3802 | SEQ ID NO:11814 | SEQ ID NO:19826 |
| **iPS:437254** | **21-225_149F2** | NA | AGGTCTAGTCAAAGCCTCGTATACAGTGATGGAAACACCTCCTTGAAT | AAGGTTTCTAACTGGGACTCT | ATGCAAGGTACACACTGGCCTCCCACT |
| | | | SEQ ID NO:3803 | SEQ ID NO:11815 | SEQ ID NO:19827 |
| | | AA | RSSQSLVYSDGNTSLN | KVSNWDS | MQGTHWPPT |
| | | | SEQ ID NO:3804 | SEQ ID NO:11816 | SEQ ID NO:19828 |
| **iPS:437256** | **21-225_150F11** | NA | AGGTCTAGTCAAAGCCTCGTATACAGTGATGGAAACACCTCCTTGAAT | AAGGTTTCTAACTGGGACTAT | ATGCAAGGTACACACTGGCCTCCCACT |
| | | | SEQ ID NO:3805 | SEQ ID NO:11817 | SEQ ID NO:19829 |
| | | AA | RSSQSLVYSDGNTSLN | KVSNWDY | MQGTHWPPT |
| | | | SEQ ID NO:3806 | SEQ ID NO:11818 | SEQ ID NO:19830 |
| **iPS:437258** | **21-225_153F9** | NA | CGGGCGAGTCAGGGCATTAGCAATTATTTAGCC | GCTGCATCCAGTTTGCAAAGT | CAACAGTATAATAGTTACCCGCTCAGT |
| | | | SEQ ID NO:3807 | SEQ ID NO:11819 | SEQ ID NO:19831 |
| | | AA | RASQGISNYLA | AASSLQS | QQYNSYPLS |
| | | | SEQ ID NO:3808 | SEQ ID NO:11820 | SEQ ID NO:19832 |
| **iPS:437260** | **21-225_170D1** | NA | CGGGCGAGTCAGGACATTAGCAATTATTTAGCC | GCTGCATCCAGTTTGCAAAGT | CAACAGTGTGATAGTTTCCCTCTCACT |
| | | | SEQ ID NO:3809 | SEQ ID NO:11821 | SEQ ID NO:19833 |
| | | AA | RASQDISNYLA | AASSLQS | QQCDSFPLT |
| | | | SEQ ID NO:3810 | SEQ ID NO:11822 | SEQ ID NO:19834 |
| **iPS:437262** | | NA | CGGGCAAGTCAGGGCATTAGAAATGATTTAGGC | GTTGCATCCGGTTTGCAAAGT | CTACAGCACAATAGTTACCCTCCGTGGACG |

FIGURE 49

| | | | | | |
|---|---|---|---|---|---|
| | **21-225_170E4** | | SEQ ID NO:3811 | SEQ ID NO:11823 | SEQ ID NO:19835 |
| | | AA | RASQGIRNDLG | VASGLQS | LQHNSYPPWT |
| | | | SEQ ID NO:3812 | SEQ ID NO:11824 | SEQ ID NO:19836 |
| **iPS:437264** | **21-225_171H12** | NA | CGGGCGAGTCAGGACATTAG CAATTATTTAGCC | TCTGCATCCAGTTTGCAA AGT | CAACAATCTGATAGTTACCC TCTCACT |
| | | | SEQ ID NO:3813 | SEQ ID NO:11825 | SEQ ID NO:19837 |
| | | AA | RASQDISNYLA | SASSLQS | QQSDSYPLT |
| | | | SEQ ID NO:3814 | SEQ ID NO:11826 | SEQ ID NO:19838 |
| **iPS:437266** | **21-225_177A5** | NA | CGGGCGAGTCAGGACATTAG CAATTATTTAGCC | TCTGCATCCAGTTTGCAA AGT | CAACAATCTGATAGTTACCC TCTCACT |
| | | | SEQ ID NO:3815 | SEQ ID NO:11827 | SEQ ID NO:19839 |
| | | AA | RASQDISNYLA | SASSLQS | QQSDSYPLT |
| | | | SEQ ID NO:3816 | SEQ ID NO:11828 | SEQ ID NO:19840 |
| **iPS:437268** | **21-225_177D2** | NA | AGGTCTAGTCAAAGCCTCGT ATACAGTGATGGAAACACCT ACTTGAAT | AAGGTTTCTAACTGGGA CTCT | ATGCAAGGTACACACTGGCC TCTCACT |
| | | | SEQ ID NO:3817 | SEQ ID NO:11829 | SEQ ID NO:19841 |
| | | AA | RSSQSLVYSDGNTYLN | KVSNWDS | MQGTHWPLT |
| | | | SEQ ID NO:3818 | SEQ ID NO:11830 | SEQ ID NO:19842 |
| **iPS:437270** | **21-225_178H4** | NA | CGGGCGAGTCAGGACATTAG CAATTATTTAGCC | TCTGCATCCAGTTTGCAA AGT | CAACAATCTAATAGTTACCC TCTCACT |
| | | | SEQ ID NO:3819 | SEQ ID NO:11831 | SEQ ID NO:19843 |
| | | AA | RASQDISNYLA | SASSLQS | QQSNSYPLT |
| | | | SEQ ID NO:3820 | SEQ ID NO:11832 | SEQ ID NO:19844 |
| **iPS:437274** | **21-225_196D4** | NA | CGGGCGAGTCAGGGCATTAG CAATTATTTAGCC | GCTGCATCCAGTTTGCAA AGT | CAACATTATCTTAATTACCCT CTCACC |
| | | | SEQ ID NO:3821 | SEQ ID NO:11833 | SEQ ID NO:19845 |
| | | AA | RASQGISNYLA | AASSLQS | QHYLNYPLT |
| | | | SEQ ID NO:3822 | SEQ ID NO:11834 | SEQ ID NO:19846 |

FIGURE 49

| | | | | | |
|---|---|---|---|---|---|
| **iPS:437280** | **21-225_203C10** | NA | CGGGCAAGTCAGGACATTAG AAATGATTTAGGC | AGAGCATCCAGTTTGCA AAGT | CTACAGCATAATAGTTACCC GTGGACG |
| | | | SEQ ID NO:3823 | SEQ ID NO:11835 | SEQ ID NO:19847 |
| | | AA | RASQDIRNDLG | RASSLQS | LQHNSYPWT |
| | | | SEQ ID NO:3824 | SEQ ID NO:11836 | SEQ ID NO:19848 |
| **iPS:437282** | **21-225_207C9** | NA | CGGGCAAGTCAGAGGTTTAG TAACTATTTAAAT | ACTGCATCCAGTTTGCAA AGT | CAACAGAGTTACAGTATTCC GCTCACT |
| | | | SEQ ID NO:3825 | SEQ ID NO:11837 | SEQ ID NO:19849 |
| | | AA | RASQRFSNYLN | TASSLQS | QQSYSIPLT |
| | | | SEQ ID NO:3826 | SEQ ID NO:11838 | SEQ ID NO:19850 |
| **iPS:437286** | **21-225_208F1** | NA | CGGGCAAGTCAGGGCATTAG ACATGATTTAGGC | GCTGCATCCAGTTTGCAA AGT | CTACAGCATTATAGTTTCCCT CGGACG |
| | | | SEQ ID NO:3827 | SEQ ID NO:11839 | SEQ ID NO:19851 |
| | | AA | RASQGIRHDLG | AASSLQS | LQHYSFPRT |
| | | | SEQ ID NO:3828 | SEQ ID NO:11840 | SEQ ID NO:19852 |
| **iPS:437290** | **21-225_210G6** | NA | CGGGCAAGTCAGGGCATTAG ACATGATTTAGGC | GCTGCATCCAGTTCGCA AAGT | GTACAGCATTATAGTTTCCC TCGGACG |
| | | | SEQ ID NO:3829 | SEQ ID NO:11841 | SEQ ID NO:19853 |
| | | AA | RASQGIRHDLG | AASSSQS | VQHYSFPRT |
| | | | SEQ ID NO:3830 | SEQ ID NO:11842 | SEQ ID NO:19854 |
| **iPS:437294** | **21-225_216D5** | NA | AGGTCTAGTCAAAGCCTCGT ATACAGTGATGGAAACACCT ACTTGAAT | AAGGTTTCTAACTGGGA CTCT | ATGCAAGGTGCACACTGGTT CACC |
| | | | SEQ ID NO:3831 | SEQ ID NO:11843 | SEQ ID NO:19855 |
| | | AA | RSSQSLVYSDGNTYLN | KVSNWDS | MQGAHWFT |
| | | | SEQ ID NO:3832 | SEQ ID NO:11844 | SEQ ID NO:19856 |
| **iPS:437302** | **21-225_225B11** | NA | CAGGCGAGTCAGGACATTTT CAACTATTTAAAT | GATGCATCCACTTTGGA AACA | CAACAGTATGATAATCTCCC GATCACC |
| | | | SEQ ID NO:3833 | SEQ ID NO:11845 | SEQ ID NO:19857 |
| | | AA | QASQDIFNYLN | DASTLET | QQYDNLPIT |

FIGURE 49

| | | | | | |
|---|---|---|---|---|---|
| | | | SEQ ID NO:3834 | SEQ ID NO:11846 | SEQ ID NO:19858 |
| **iPS:437320** | **21-225_75A1** | NA | AGGTCTAGTCAGAGCCTCCTGCATAGTAATGGACACAACTATTTGGAT | TTGGGTTCTAATCGGGCCTCC | ATGCAACCTCTACAAACTCCGTTCACT |
| | | | SEQ ID NO:3835 | SEQ ID NO:11847 | SEQ ID NO:19859 |
| | | AA | RSSQSLLHSNGHNYLD | LGSNRAS | MQPLQTPFT |
| | | | SEQ ID NO:3836 | SEQ ID NO:11848 | SEQ ID NO:19860 |
| **iPS:437322** | **21-225_75B1** | NA | AGGGCCAGTCAGAGTGTTAGCAGCAGCTACTTAGTC | GGTGCATCCACCAGGGCCACT | CAGCAGTATGGTTGCTCACCGCTCACT |
| | | | SEQ ID NO:3837 | SEQ ID NO:11849 | SEQ ID NO:19861 |
| | | AA | RASQSVSSSYLV | GASTRAT | QQYGCSPLT |
| | | | SEQ ID NO:3838 | SEQ ID NO:11850 | SEQ ID NO:19862 |
| **iPS:437324** | **21-225_75C2** | NA | AGGGCCAGTCAGAGTGTTTACAGCAGCTACTTAGCC | GGTGCATCCAGCCGGGCCACT | CAGCACTATGATAACTCACCGTGGACG |
| | | | SEQ ID NO:3839 | SEQ ID NO:11851 | SEQ ID NO:19863 |
| | | AA | RASQSVYSSYLA | GASSRAT | QHYDNSPWT |
| | | | SEQ ID NO:3840 | SEQ ID NO:11852 | SEQ ID NO:19864 |
| **iPS:437326** | **21-225_75C10** | NA | CGGGCGAGTCAGGGCATTAGCATCTGGTTAGCC | GCTGCATCCAGTTTGCAAAGT | CAACAGGCTAAAAGTTTCCCGCTCACT |
| | | | SEQ ID NO:3841 | SEQ ID NO:11853 | SEQ ID NO:19865 |
| | | AA | RASQGISIWLA | AASSLQS | QQAKSFPLT |
| | | | SEQ ID NO:3842 | SEQ ID NO:11854 | SEQ ID NO:19866 |
| **iPS:437328** | **21-225_75D3** | NA | AGGGCCAGTCAGAGTGTTTACAGCAGCTACTTAGCC | GGTGCATCCAGCCGGTCCACT | CAGCACTATGATAACTCACCGTGGACG |
| | | | SEQ ID NO:3843 | SEQ ID NO:11855 | SEQ ID NO:19867 |
| | | AA | RASQSVYSSYLA | GASSRST | QHYDNSPWT |
| | | | SEQ ID NO:3844 | SEQ ID NO:11856 | SEQ ID NO:19868 |
| **iPS:437332** | **21-225_75F3** | NA | AGGGCCAGTCAGAGTGTTTACAGCAGCTACTTAGCC | GGTGCATCCAGCCGGGCCACT | CAGCACTATGATAACTCACCGTGGACG |
| | | | SEQ ID NO:3845 | SEQ ID NO:11857 | SEQ ID NO:19869 |

## FIGURE 49

| | | | | | |
|---|---|---|---|---|---|
| | | AA | RASQSVYSSYLA | GASSRAT | QHYDNSPWT |
| | | | SEQ ID NO:3846 | SEQ ID NO:11858 | SEQ ID NO:19870 |
| **iPS:437334** | **21-225_75F11** | NA | AGGGCCAGTCAGAGTGTTAGCAGAAATTTAGCC | GGTGCATCCATCAGGGCCACT | CAGCAGTATAATAACTGGCCTCCGCTCACT |
| | | | SEQ ID NO:3847 | SEQ ID NO:11859 | SEQ ID NO:19871 |
| | | AA | RASQSVSRNLA | GASIRAT | QQYNNWPPLT |
| | | | SEQ ID NO:3848 | SEQ ID NO:11860 | SEQ ID NO:19872 |
| **iPS:437340** | **21-225_75G9** | NA | AGGGCCAGTCCGAGTGTTGACAGCAGCTACTTAGCC | GGTGCATCCAGCAGGGCCCCT | CAGCAGTATGAAAGTTCACCGTGGACG |
| | | | SEQ ID NO:3849 | SEQ ID NO:11861 | SEQ ID NO:19873 |
| | | AA | RASPSVDSSYLA | GASSRAP | QQYESSPWT |
| | | | SEQ ID NO:3850 | SEQ ID NO:11862 | SEQ ID NO:19874 |
| **iPS:437344** | **21-225_75G12** | NA | AGGGCCAGTCAGAGTGTTTACAGCAGCTACTTAGCC | GGTGCATCCAGCCGGGCCACT | CAGCACTATGATAACTCACCGTGGACG |
| | | | SEQ ID NO:3851 | SEQ ID NO:11863 | SEQ ID NO:19875 |
| | | AA | RASQSVYSSYLA | GASSRAT | QHYDNSPWT |
| | | | SEQ ID NO:3852 | SEQ ID NO:11864 | SEQ ID NO:19876 |
| **iPS:437346** | **21-225_75H7** | NA | CGGGCAAGTCAGGGCATAAGAAATGATTTAGGC | GATGCATCCAGTTTGCAAAGT | ATACAGCATAGTAATTACCCGCTCACT |
| | | | SEQ ID NO:3853 | SEQ ID NO:11865 | SEQ ID NO:19877 |
| | | AA | RASQGIRNDLG | DASSLQS | IQHSNYPLT |
| | | | SEQ ID NO:3854 | SEQ ID NO:11866 | SEQ ID NO:19878 |
| **iPS:437350** | **21-225_74A3** | NA | AGGGCCAGTCAGAGTGTTTACAGCAGCTACTTAGCC | GGTGCATCCAGCCGGTCCACT | CAGCACTCTGATAACTCACCGTGGACG |
| | | | SEQ ID NO:3855 | SEQ ID NO:11867 | SEQ ID NO:19879 |
| | | AA | RASQSVYSSYLA | GASSRST | QHSDNSPWT |
| | | | SEQ ID NO:3856 | SEQ ID NO:11868 | SEQ ID NO:19880 |
| **iPS:437356** | **21 225 74B1** | NA | AAGTCCAGCCAGAGTGTTTTACACAGGTCCAACAATTACAACTACTTAGCG | TGGGCATCTACCCGGGAATCC | CAGCAATATTATAGTACTCCTCCGACG |

FIGURE 49

| | | | | | |
|---|---|---|---|---|---|
| | 21-225_74B1 | | SEQ ID NO:3857 | SEQ ID NO:11869 | SEQ ID NO:19881 |
| | | AA | KSSQSVLHRSNNYNYLA | WASTRES | QQYYSTPPT |
| | | | SEQ ID NO:3858 | SEQ ID NO:11870 | SEQ ID NO:19882 |
| **iPS:437361** | 21-225_74C1 | NA | AAGTCCAGCCAGAGTATTTTACACAGCTCCAACAATTACAATTACTTAGCT | TGGGCATCTACCCGGGAATCC | CAGCAATATTATAGTACTCCGTGGACG |
| | | | SEQ ID NO:3859 | SEQ ID NO:11871 | SEQ ID NO:19883 |
| | | AA | KSSQSILHSSNNYNYLA | WASTRES | QQYYSTPWT |
| | | | SEQ ID NO:3860 | SEQ ID NO:11872 | SEQ ID NO:19884 |
| **iPS:437363** | 21-225_74C10 | NA | AAGTCCAGCCAGAGTGTTTTATACAGCTCCAACAATGCGAACTACTTAGCT | TGGGCATCTACCCGGGAATCC | CAGCAATATTATAGTACTCCGTGCAGT |
| | | | SEQ ID NO:3861 | SEQ ID NO:11873 | SEQ ID NO:19885 |
| | | AA | KSSQSVLYSSNNANYLA | WASTRES | QQYYSTPCS |
| | | | SEQ ID NO:3862 | SEQ ID NO:11874 | SEQ ID NO:19886 |
| **iPS:437369** | 21-225_74D6 | NA | AGGGCCAGTCAGAGTGTTTACAGCAGCTACTTAGCC | GGTGCATCCAGCCGGGCCACT | CAGCATTATGATAACTCACCGTGGACG |
| | | | SEQ ID NO:3863 | SEQ ID NO:11875 | SEQ ID NO:19887 |
| | | AA | RASQSVYSSYLA | GASSRAT | QHYDNSPWT |
| | | | SEQ ID NO:3864 | SEQ ID NO:11876 | SEQ ID NO:19888 |
| **iPS:437371** | 21-225_74D8 | NA | AGGTCTAGTCAGAGCCTCGTGCATAGTAGTGGATACAACTATTTGGAT | TTGGGTTCTAATCGGGCCTCC | ATGCAAGCTCTACACCCTCCTCTCACT |
| | | | SEQ ID NO:3865 | SEQ ID NO:11877 | SEQ ID NO:19889 |
| | | AA | RSSQSLVHSSGYNYLD | LGSNRAS | MQALHPPLT |
| | | | SEQ ID NO:3866 | SEQ ID NO:11878 | SEQ ID NO:19890 |
| **iPS:437377** | 21-225_74G9 | NA | AGGGCCAGTCAGAGTGTTAGCAGCAGCTACTTAGTC | GGTGCATCCACCAGGGCCACT | CAGCAGTATGGTTGCTCACCGCTCACT |
| | | | SEQ ID NO:3867 | SEQ ID NO:11879 | SEQ ID NO:19891 |
| | | AA | RASQSVSSSYLV | GASTRAT | QQYGCSPLT |

FIGURE 49

| | | | | | |
|---|---|---|---|---|---|
| | | | SEQ ID NO:3868 | SEQ ID NO:11880 | SEQ ID NO:19892 |
| **iPS:437379** | **21-225_74H2** | NA | AAGTCCAGCCAGAGTGTTTTACACAGCTCCAACAATAAGAACTACTTAACT | TGGGCATCTACTCGGGAATCC | CAGCAATATTATAGTATTCCTCCGACG |
| | | | SEQ ID NO:3869 | SEQ ID NO:11881 | SEQ ID NO:19893 |
| | | AA | KSSQSVLHSSNNKNYLT | WASTRES | QQYYSIPPT |
| | | | SEQ ID NO:3870 | SEQ ID NO:11882 | SEQ ID NO:19894 |
| **iPS:437383** | **21-225_74H8** | NA | AGGGCCAGTCAGAGTTTTAGCAGCAGCTACTTAGCC | GGTGCATCCAGCAGGGCCACT | CAGCAGTATGGTAGCTCAAGGACG |
| | | | SEQ ID NO:3871 | SEQ ID NO:11883 | SEQ ID NO:19895 |
| | | AA | RASQSFSSSYLA | GASSRAT | QQYGSSRT |
| | | | SEQ ID NO:3872 | SEQ ID NO:11884 | SEQ ID NO:19896 |
| **iPS:438664** | **21-225_216G1** | NA | CGGGCGAGTCAGGGCATTAGCAGTTATTTAGCC | GCTGCATCCAGTTTGCAAAGT | CTACGGTATGATACTTACCCTCTCACT |
| | | | SEQ ID NO:3873 | SEQ ID NO:11885 | SEQ ID NO:19897 |
| | | AA | RASQGISSYLA | AASSLQS | LRYDTYPLT |
| | | | SEQ ID NO:3874 | SEQ ID NO:11886 | SEQ ID NO:19898 |
| **iPS:441468** | **21-225_25A4.001.001** | NA | AAGTCCAGCCAGAGTGTTTTATACAGCTCCCACAATAACAACTACTTAGCT | TGGGCATCTACCCGGGAATCC | CAGCAGTATTATAGTACTCCTCCGACG |
| | | | SEQ ID NO:3875 | SEQ ID NO:11887 | SEQ ID NO:19899 |
| | | AA | KSSQSVLYSSHNNNYLA | WASTRES | QQYYSTPPT |
| | | | SEQ ID NO:3876 | SEQ ID NO:11888 | SEQ ID NO:19900 |
| **iPS:441475** | **21-225_25A4.001.002** | NA | AAGTCCAGCCAGAGTGTTTTATACAGCTCCCACAATAACAACTACTTAGCT | TGGGCATCTACCCGGGAATCC | CAGCAGTATTATAGTACTCCTCCGACG |
| | | | SEQ ID NO:3877 | SEQ ID NO:11889 | SEQ ID NO:19901 |
| | | AA | KSSQSVLYSSHNNNYLA | WASTRES | QQYYSTPPT |
| | | | SEQ ID NO:3878 | SEQ ID NO:11890 | SEQ ID NO:19902 |

FIGURE 49

| | | | | | |
|---|---|---|---|---|---|
| **iPS:441482** | **21-225_25A4.001.003** | NA | AAGTCCAGCCAGAGTGTTTTATACAGCTCCCACAATAACAACTACTTAGCT | TGGGCATCTACCCGGGAATCC | CAGCAGTATTATAGTACTCCTCCGACG |
| | | | SEQ ID NO:3879 | SEQ ID NO:11891 | SEQ ID NO:19903 |
| | | AA | KSSQSVLYSSHNNNYLA | WASTRES | QQYYSTPPT |
| | | | SEQ ID NO:3880 | SEQ ID NO:11892 | SEQ ID NO:19904 |
| **iPS:441489** | **21-225_25A4.001.004** | NA | AAGTCCAGCCAGAGTGTTTTATACAGCTCCCACAATAACAACTACTTAGCT | TGGGCATCTACCCGGGAATCC | CAGCAGTATTATAGTACTCCTCCGACG |
| | | | SEQ ID NO:3881 | SEQ ID NO:11893 | SEQ ID NO:19905 |
| | | AA | KSSQSVLYSSHNNNYLA | WASTRES | QQYYSTPPT |
| | | | SEQ ID NO:3882 | SEQ ID NO:11894 | SEQ ID NO:19906 |
| **iPS:441496** | **21-225_25A4.001.005** | NA | AAGTCCAGCCAGAGTGTTTTATACAGCTCCCACAATAACAACTACTTAGCT | TGGGCATCTACCCGGGAATCC | CAGCAGTATTATAGTACTCCTCCGACG |
| | | | SEQ ID NO:3883 | SEQ ID NO:11895 | SEQ ID NO:19907 |
| | | AA | KSSQSVLYSSHNNNYLA | WASTRES | QQYYSTPPT |
| | | | SEQ ID NO:3884 | SEQ ID NO:11896 | SEQ ID NO:19908 |
| **iPS:441505** | **21-225_25A4.001.006** | NA | AAGTCCAGCCAGAGTGTTTTATACAGCTCCCACAATAACAACTACTTAGCT | TGGGCATCTACCCGGGAATCC | CAGCAGTATTATAGTACTCCTCCGACG |
| | | | SEQ ID NO:3885 | SEQ ID NO:11897 | SEQ ID NO:19909 |
| | | AA | KSSQSVLYSSHNNNYLA | WASTRES | QQYYSTPPT |
| | | | SEQ ID NO:3886 | SEQ ID NO:11898 | SEQ ID NO:19910 |
| **iPS:441512** | **21-225_25A4.001.007** | NA | AAGTCCAGCCAGAGTGTTTTATACAGCTCCCACAATAACAACTACTTAGCT | TGGGCATCTACCCGGGAATCC | CAGCAGTATTATAGTACTCCTCCGACG |
| | | | SEQ ID NO:3887 | SEQ ID NO:11899 | SEQ ID NO:19911 |
| | | AA | KSSQSVLYSSHNNNYLA | WASTRES | QQYYSTPPT |
| | | | SEQ ID NO:3888 | SEQ ID NO:11900 | SEQ ID NO:19912 |

FIGURE 49

| | | | | | |
|---|---|---|---|---|---|
| **iPS:441519** | **21-225_25A4.001.008** | NA | AAGTCCAGCCAGAGTGTTTTATACAGCTCCCACAATAACAACTACTTAGCT | TGGGCATCTACCCGGGAATCC | CAGCAGTATTATAGTACTCCTCCGACG |
| | | | SEQ ID NO:3889 | SEQ ID NO:11901 | SEQ ID NO:19913 |
| | | AA | KSSQSVLYSSHNNNYLA | WASTRES | QQYYSTPPT |
| | | | SEQ ID NO:3890 | SEQ ID NO:11902 | SEQ ID NO:19914 |
| **iPS:441554** | **21-225_25A4.001.013** | NA | AAGTCCAGCCAGAGTGTTTTATACAGCTCCCACAATAACAACTACTTAGCT | TGGGCATCTACCCGGGAATCC | CAGCAGTATTATAGTACTCCTCCGACG |
| | | | SEQ ID NO:3891 | SEQ ID NO:11903 | SEQ ID NO:19915 |
| | | AA | KSSQSVLYSSHNNNYLA | WASTRES | QQYYSTPPT |
| | | | SEQ ID NO:3892 | SEQ ID NO:11904 | SEQ ID NO:19916 |
| **iPS:441595** | **21-225_25A4.001.019** | NA | AAGTCCAGCCAGAGTGTTTTATACAGCTCCCACAATAACAACTACTTAGCT | TGGGCATCTACCCGGGAATCC | CAGCAGTATTATAGTACTCCTCCGACG |
| | | | SEQ ID NO:3893 | SEQ ID NO:11905 | SEQ ID NO:19917 |
| | | AA | KSSQSVLYSSHNNNYLA | WASTRES | QQYYSTPPT |
| | | | SEQ ID NO:3894 | SEQ ID NO:11906 | SEQ ID NO:19918 |
| **iPS:441604** | **21-225_25A4.001.020** | NA | AAGTCCAGCCAGAGTGTTTTATACAGCTCCCACAATAACAACTACTTAGCT | TGGGCATCTACCCGGGAATCC | CAGCAGTATTATAGTACTCCTCCGACG |
| | | | SEQ ID NO:3895 | SEQ ID NO:11907 | SEQ ID NO:19919 |
| | | AA | KSSQSVLYSSHNNNYLA | WASTRES | QQYYSTPPT |
| | | | SEQ ID NO:3896 | SEQ ID NO:11908 | SEQ ID NO:19920 |
| **iPS:441613** | **21-225_25A4.001.021** | NA | AAGTCCAGCCAGAGTGTTTTATACAGCTCCCACAATAACAACTACTTAGCT | TGGGCATCTACCCGGGAATCC | CAGCAGTATTATAGTACTCCTCCGACG |
| | | | SEQ ID NO:3897 | SEQ ID NO:11909 | SEQ ID NO:19921 |
| | | AA | KSSQSVLYSSHNNNYLA | WASTRES | QQYYSTPPT |
| | | | SEQ ID NO:3898 | SEQ ID NO:11910 | SEQ ID NO:19922 |

FIGURE 49

| | | | | | |
|---|---|---|---|---|---|
| **iPS:441841** | **21-225_4A2.001.001** | NA | AAGTCCAGCCAGAGTATTTTACACAGCTCCAACAATAACAACTACTTAGCT | TGGGCATCTACCCGGGAATCC | CAGCAATATTATAGTACTCCAGTCACT |
| | | | SEQ ID NO:3899 | SEQ ID NO:11911 | SEQ ID NO:19923 |
| | | AA | KSSQSILHSSNNNNYLA | WASTRES | QQYYSTPVT |
| | | | SEQ ID NO:3900 | SEQ ID NO:11912 | SEQ ID NO:19924 |
| **iPS:441847** | **21-225_4A2.001.002** | NA | AAGTCCAGCCAGAGTATTTTACACAGCTCCAACAATAACAACTACTTAGCT | TGGGCATCTACCCGGGAATCC | CAGCAATATTATAATGCTCCAGTCACT |
| | | | SEQ ID NO:3901 | SEQ ID NO:11913 | SEQ ID NO:19925 |
| | | AA | KSSQSILHSSNNNNYLA | WASTRES | QQYYNAPVT |
| | | | SEQ ID NO:3902 | SEQ ID NO:11914 | SEQ ID NO:19926 |
| **iPS:441853** | **21-225_4A2.001.003** | NA | AAGTCCAGCCAGAGTATTTTACACAGCTCCAACAATAACAACTACTTAGCT | TGGGCATCTACCCGGGAATCC | CAGCAATATTATCAAACTCCAGTCACT |
| | | | SEQ ID NO:3903 | SEQ ID NO:11915 | SEQ ID NO:19927 |
| | | AA | KSSQSILHSSNNNNYLA | WASTRES | QQYYQTPVT |
| | | | SEQ ID NO:3904 | SEQ ID NO:11916 | SEQ ID NO:19928 |
| **iPS:441859** | **21-225_4A2.001.004** | NA | AAGTCCAGCCAGAGTATTTTACACAGCTCCAACAATAACAACTACTTAGCT | TGGGCATCTACCCGGGAATCC | CAGCAATATTATAATACTCCAGTCACT |
| | | | SEQ ID NO:3905 | SEQ ID NO:11917 | SEQ ID NO:19929 |
| | | AA | KSSQSILHSSNNNNYLA | WASTRES | QQYYNTPVT |
| | | | SEQ ID NO:3906 | SEQ ID NO:11918 | SEQ ID NO:19930 |
| **iPS:441866** | **21-225_4A2.001.005** | NA | AAGTCCAGCCAGAGTATTTTACACAGCTCCAACAATAACAACTACTTAGCT | TGGGCATCTACCCGGGAATCC | CAGCAATATTATAATACTCCAGTCACT |
| | | | SEQ ID NO:3907 | SEQ ID NO:11919 | SEQ ID NO:19931 |
| | | AA | KSSQSILHSSNNNNYLA | WASTRES | QQYYNTPVT |
| | | | SEQ ID NO:3908 | SEQ ID NO:11920 | SEQ ID NO:19932 |

FIGURE 49

| | | | | | |
|---|---|---|---|---|---|
| **iPS:441873** | **21-225_4A2.001.006** | NA | AAGTCCAGCCAGAGTATTTTACACAGCTCCAACAATAACAACTACTTAGCT | TGGGCATCTACCCGGGAATCC | CAGCAATATTATAATACTCCAGTCACT |
| | | | SEQ ID NO:3909 | SEQ ID NO:11921 | SEQ ID NO:19933 |
| | | AA | KSSQSILHSSNNNNYLA | WASTRES | QQYYNTPVT |
| | | | SEQ ID NO:3910 | SEQ ID NO:11922 | SEQ ID NO:19934 |
| **iPS:441880** | **21-225_4A2.001.007** | NA | AAGTCCAGCCAGAGTATTTTACACAGCTCCAACAATAACAACTACTTAGCT | TGGGCATCTACCCGGGAATCC | CAGCAATATTATAGTACTCCAGTCACT |
| | | | SEQ ID NO:3911 | SEQ ID NO:11923 | SEQ ID NO:19935 |
| | | AA | KSSQSILHSSNNNNYLA | WASTRES | QQYYSTPVT |
| | | | SEQ ID NO:3912 | SEQ ID NO:11924 | SEQ ID NO:19936 |
| **iPS:441884** | **21-225_4A2.001.008** | NA | AAGTCCAGCCAGAGTATTTTACACAGCTCCAACAATAACAACTACTTAGCT | TGGGCATCTACCCGGGAATCC | CAGCAATATTATAGTACTCCAGTCACT |
| | | | SEQ ID NO:3913 | SEQ ID NO:11925 | SEQ ID NO:19937 |
| | | AA | KSSQSILHSSNNNNYLA | WASTRES | QQYYSTPVT |
| | | | SEQ ID NO:3914 | SEQ ID NO:11926 | SEQ ID NO:19938 |
| **iPS:441888** | **21-225_4A2.001.009** | NA | AAGTCCAGCCAGAGTATTTTACACAGCTCCAACAATAACAACTACTTAGCT | TGGGCATCTACCCGGGAATCC | CAGCAATATTATAATGCTCCAGTCACT |
| | | | SEQ ID NO:3915 | SEQ ID NO:11927 | SEQ ID NO:19939 |
| | | AA | KSSQSILHSSNNNNYLA | WASTRES | QQYYNAPVT |
| | | | SEQ ID NO:3916 | SEQ ID NO:11928 | SEQ ID NO:19940 |
| **iPS:441892** | **21-225_4A2.001.010** | NA | AAGTCCAGCCAGAGTATTTTACACAGCTCCAACAATAACAACTACTTAGCT | TGGGCATCTACCCGGGAATCC | CAGCAATATTATCAAACTCCAGTCACT |
| | | | SEQ ID NO:3917 | SEQ ID NO:11929 | SEQ ID NO:19941 |
| | | AA | KSSQSILHSSNNNNYLA | WASTRES | QQYYQTPVT |
| | | | SEQ ID NO:3918 | SEQ ID NO:11930 | SEQ ID NO:19942 |

FIGURE 49

| | | | | | |
|---|---|---|---|---|---|
| **iPS:441896** | **21-225_4A2.001.011** | NA | AAGTCCAGCCAGAGTATTTTACACAGCTCCAACAATAACAACTACTTAGCT | TGGGCATCTACCCGGGAATCC | CAGCAATATTATCAAACTCCAGTCACT |
| | | | SEQ ID NO:3919 | SEQ ID NO:11931 | SEQ ID NO:19943 |
| | | AA | KSSQSILHSSNNNNYLA | WASTRES | QQYYQTPVT |
| | | | SEQ ID NO:3920 | SEQ ID NO:11932 | SEQ ID NO:19944 |
| **iPS:441900** | **21-225_4A2.001.012** | NA | AAGTCCAGCCAGAGTATTTTACACAGCTCCAACAATAACAACTACTTAGCT | TGGGCATCTACCCGGGAATCC | CAGCAATATTATCAAACTCCAGTCACT |
| | | | SEQ ID NO:3921 | SEQ ID NO:11933 | SEQ ID NO:19945 |
| | | AA | KSSQSILHSSNNNNYLA | WASTRES | QQYYQTPVT |
| | | | SEQ ID NO:3922 | SEQ ID NO:11934 | SEQ ID NO:19946 |
| **iPS:441955** | **21-225_4A2.001.022** | NA | AAGTCCAGCCAGAGTATTTTACACAGCTCCAACAATAACAACTACTTAGCT | TGGGCATCTACCCGGGAATCC | CAGCAATATTATAGTACTCCAGTCACT |
| | | | SEQ ID NO:3923 | SEQ ID NO:11935 | SEQ ID NO:19947 |
| | | AA | KSSQSILHSSNNNNYLA | WASTRES | QQYYSTPVT |
| | | | SEQ ID NO:3924 | SEQ ID NO:11936 | SEQ ID NO:19948 |
| **iPS:441962** | **21-225_4A2.001.023** | NA | AAGTCCAGCCAGAGTATTTTACACAGCTCCAACAATAACAACTACTTAGCT | TGGGCATCTACCCGGGAATCC | CAGCAATATTATAGTACTCCAGTCACT |
| | | | SEQ ID NO:3925 | SEQ ID NO:11937 | SEQ ID NO:19949 |
| | | AA | KSSQSILHSSNNNNYLA | WASTRES | QQYYSTPVT |
| | | | SEQ ID NO:3926 | SEQ ID NO:11938 | SEQ ID NO:19950 |
| **iPS:441971** | **21-225_4A2.001.024** | NA | AAGTCCAGCCAGAGTATTTTACACAGCTCCAACAATAACAACTACTTAGCT | TGGGCATCTACCCGGGAATCC | CAGCAATATTATAGTACTCCAGTCACT |
| | | | SEQ ID NO:3927 | SEQ ID NO:11939 | SEQ ID NO:19951 |
| | | AA | KSSQSILHSSNNNNYLA | WASTRES | QQYYSTPVT |
| | | | SEQ ID NO:3928 | SEQ ID NO:11940 | SEQ ID NO:19952 |

FIGURE 49

| | | | | | |
|---|---|---|---|---|---|
| **iPS:441999** | **21-225_4A2.001.028** | NA | AAGTCCAGCCAGAGTATTTTACACAGCTCCAACAATAACAACTACTTAGCT | TGGGCATCTACCCGGGAATCC | CAGCAATATTATAGTACTCCAGTCACT |
| | | | SEQ ID NO:3929 | SEQ ID NO:11941 | SEQ ID NO:19953 |
| | | AA | KSSQSILHSSNNNNYLA | WASTRES | QQYYSTPVT |
| | | | SEQ ID NO:3930 | SEQ ID NO:11942 | SEQ ID NO:19954 |
| **iPS:442006** | **21-225_4A2.001.029** | NA | AAGTCCAGCCAGAGTATTTTACACAGCTCCAACAATAACAACTACTTAGCT | TGGGCATCTACCCGGGAATCC | CAGCAATATTATAGTACTCCAGTCACT |
| | | | SEQ ID NO:3931 | SEQ ID NO:11943 | SEQ ID NO:19955 |
| | | AA | KSSQSILHSSNNNNYLA | WASTRES | QQYYSTPVT |
| | | | SEQ ID NO:3932 | SEQ ID NO:11944 | SEQ ID NO:19956 |
| **iPS:442020** | **21-225_4A2.001.031** | NA | AAGTCCAGCCAGAGTATTTTACACAGCTCCAACAATAACAACTACTTAGCT | TGGGCATCTACCCGGGAATCC | CAGCAATATTATAATACTCCAGTCACT |
| | | | SEQ ID NO:3933 | SEQ ID NO:11945 | SEQ ID NO:19957 |
| | | AA | KSSQSILHSSNNNNYLA | WASTRES | QQYYNTPVT |
| | | | SEQ ID NO:3934 | SEQ ID NO:11946 | SEQ ID NO:19958 |
| **iPS:442050** | **21-225_4H6.004** | NA | CGGGCGAGTCAGGGTATTAGCAGGTGGTTAGCC | GGTGCATCCAGTTTGCAAAGT | CAACAGGCTAACAGTTTCCCATTCACT |
| | | | SEQ ID NO:3935 | SEQ ID NO:11947 | SEQ ID NO:19959 |
| | | AA | RASQGISRWLA | GASSLQS | QQANSFPFT |
| | | | SEQ ID NO:3936 | SEQ ID NO:11948 | SEQ ID NO:19960 |
| **iPS:442059** | **21-225_4H6.005** | NA | CGGGCGAGTCAGGGTATTAGCAGGTGGTTAGCC | GGTGCATCCAGTTTGCAAAGT | CAACAGGCTAACAGTTTCCCATTCACT |
| | | | SEQ ID NO:3937 | SEQ ID NO:11949 | SEQ ID NO:19961 |
| | | AA | RASQGISRWLA | GASSLQS | QQANSFPFT |
| | | | SEQ ID NO:3938 | SEQ ID NO:11950 | SEQ ID NO:19962 |
| **iPS:442065** | | NA | CGGGCGAGTCAGGGTATTAGCAGGTGGTTAGCC | GGTGCATCCAGTTTGCAAAGT | CAACAGGCTAACAGTTTCCCATTCACT |

FIGURE 49

| | | | | | |
|---|---|---|---|---|---|
| | **21-225_4H6.006** | | SEQ ID NO:3939 | SEQ ID NO:11951 | SEQ ID NO:19963 |
| | | AA | RASQGISRWLA | GASSLQS | QQANSFPFT |
| | | | SEQ ID NO:3940 | SEQ ID NO:11952 | SEQ ID NO:19964 |
| **iPS:442071** | **21-225_4H6.007** | NA | CGGGCGAGTCAGGGTATTAGCAGGTGGTTAGCC | GGTGCATCCAGTTTGCAAAGT | CAACAGGCTAACAGTTTCCCATTCACT |
| | | | SEQ ID NO:3941 | SEQ ID NO:11953 | SEQ ID NO:19965 |
| | | AA | RASQGISRWLA | GASSLQS | QQANSFPFT |
| | | | SEQ ID NO:3942 | SEQ ID NO:11954 | SEQ ID NO:19966 |
| **iPS:442078** | **21-225_4H6.008** | NA | CGGGCGAGTCAGGGTATTAGCAGGTGGTTAGCC | GGTGCATCCAGTTTGCAAAGT | CAACAGGCTAACAGTTTCCCATTCACT |
| | | | SEQ ID NO:3943 | SEQ ID NO:11955 | SEQ ID NO:19967 |
| | | AA | RASQGISRWLA | GASSLQS | QQANSFPFT |
| | | | SEQ ID NO:3944 | SEQ ID NO:11956 | SEQ ID NO:19968 |
| **iPS:442085** | **21-225_4H6.009** | NA | CGGGCGAGTCAGGGTATTAGCAGGTGGTTAGCC | GGTGCATCCAGTTTGCAAAGT | CAACAGGCTAACAGTTTCCCATTCACT |
| | | | SEQ ID NO:3945 | SEQ ID NO:11957 | SEQ ID NO:19969 |
| | | AA | RASQGISRWLA | GASSLQS | QQANSFPFT |
| | | | SEQ ID NO:3946 | SEQ ID NO:11958 | SEQ ID NO:19970 |
| **iPS:442089** | **21-225_4H6.010** | NA | CGGGCGAGTCAGGGTATTAGCAGGTGGTTAGCC | GGTGCATCCAGTTTGCAAAGT | CAACAGGCTAACAGTTTCCCATTCACT |
| | | | SEQ ID NO:3947 | SEQ ID NO:11959 | SEQ ID NO:19971 |
| | | AA | RASQGISRWLA | GASSLQS | QQANSFPFT |
| | | | SEQ ID NO:3948 | SEQ ID NO:11960 | SEQ ID NO:19972 |
| **iPS:442093** | **21-225_4H6.011** | NA | CGGGCGAGTCAGGGTATTAGCAGGTGGTTAGCC | GGTGCATCCAGTTTGCAAAGT | CAACAGGCTAACAGTTTCCCATTCACT |
| | | | SEQ ID NO:3949 | SEQ ID NO:11961 | SEQ ID NO:19973 |
| | | AA | RASQGISRWLA | GASSLQS | QQANSFPFT |
| | | | SEQ ID NO:3950 | SEQ ID NO:11962 | SEQ ID NO:19974 |
| **iPS:442115** | | NA | CGGGCAAGTCAGGGCATTAGAAATGATTTAGGC | GCTGCATCCAGTTTGCAAAGT | CTACAGCATTATAGTTACCCTCGGACG |

FIGURE 49

| | | | | | |
|---|---|---|---|---|---|
| | **21-225_5E5.003** | | SEQ ID NO:3951 | SEQ ID NO:11963 | SEQ ID NO:19975 |
| | | AA | RASQGIRNDLG | AASSLQS | LQHYSYPRT |
| | | | SEQ ID NO:3952 | SEQ ID NO:11964 | SEQ ID NO:19976 |
| **iPS:442122** | | NA | CGGGCAAGTCAGGGCATTAG AAATGATTTAGGC | GCTGCATCCAGTTTGCAA AGT | CTACAGCATTATAGTTACCC TCGGACG |
| | **21-225_5E5.004** | | SEQ ID NO:3953 | SEQ ID NO:11965 | SEQ ID NO:19977 |
| | | AA | RASQGIRNDLG | AASSLQS | LQHYSYPRT |
| | | | SEQ ID NO:3954 | SEQ ID NO:11966 | SEQ ID NO:19978 |
| **iPS:442129** | | NA | CGGGCAAGTCAGGGCATTAG AAATGATTTAGGC | GCTGCATCCAGTTTGCAA AGT | CTACAGCATTATAGTTACCC TCGGACG |
| | **21-225_5E5.005** | | SEQ ID NO:3955 | SEQ ID NO:11967 | SEQ ID NO:19979 |
| | | AA | RASQGIRNDLG | AASSLQS | LQHYSYPRT |
| | | | SEQ ID NO:3956 | SEQ ID NO:11968 | SEQ ID NO:19980 |
| **iPS:442136** | | NA | CGGGCAAGTCAGGGCATTAG AAATGATTTAGGC | GCTGCATCCAGTTTGCAA AGT | CTACAGCATTATAGTTACCC TCGGACG |
| | **21-225_5E5.006** | | SEQ ID NO:3957 | SEQ ID NO:11969 | SEQ ID NO:19981 |
| | | AA | RASQGIRNDLG | AASSLQS | LQHYSYPRT |
| | | | SEQ ID NO:3958 | SEQ ID NO:11970 | SEQ ID NO:19982 |
| **iPS:442171** | | NA | CGGGCAAGTCAGGGCATTAG AAATGATTTAGGC | GCTGCATCCAGTTTGCAA AGT | CTACAGCATTATAGTTACCC TCGGACG |
| | **21-225_5E5.011** | | SEQ ID NO:3959 | SEQ ID NO:11971 | SEQ ID NO:19983 |
| | | AA | RASQGIRNDLG | AASSLQS | LQHYSYPRT |
| | | | SEQ ID NO:3960 | SEQ ID NO:11972 | SEQ ID NO:19984 |
| **iPS:442178** | | NA | CGGGCAAGTCAGGGCATTAG AAATGATTTAGGC | GCTGCATCCAGTTTGCAA AGT | CTACAGCATTATAGTTACCC TCGGACG |
| | **21-225_5E5.012** | | SEQ ID NO:3961 | SEQ ID NO:11973 | SEQ ID NO:19985 |
| | | AA | RASQGIRNDLG | AASSLQS | LQHYSYPRT |
| | | | SEQ ID NO:3962 | SEQ ID NO:11974 | SEQ ID NO:19986 |
| **iPS:442199** | | NA | CGGGCAAGTCAGGGCATTAG AAATGATTTAGGC | GCTGCATCCAGTTTGCAA AGT | CTACAGCATTATAGTTACCC TCGGACG |

FIGURE 49

| | | | | | |
|---|---|---|---|---|---|
| | **21-225_5E5.015** | | SEQ ID NO:3963 | SEQ ID NO:11975 | SEQ ID NO:19987 |
| | | AA | RASQGIRNDLG | AASSLQS | LQHYSYPRT |
| | | | SEQ ID NO:3964 | SEQ ID NO:11976 | SEQ ID NO:19988 |
| **iPS:442206** | | NA | CGGGCAAGTCAGGGCATTAG AAATGATTTAGGC | GCTGCATCCAGTTTGCAA AGT | CTACAGCATTATAGTTACCC TCGGACG |
| | **21-225_5E5.016** | | SEQ ID NO:3965 | SEQ ID NO:11977 | SEQ ID NO:19989 |
| | | AA | RASQGIRNDLG | AASSLQS | LQHYSYPRT |
| | | | SEQ ID NO:3966 | SEQ ID NO:11978 | SEQ ID NO:19990 |
| **iPS:442213** | | NA | CGGGCAAGTCAGGGCATTAG AAATGATTTAGGC | GCTGCATCCAGTTTGCAA AGT | CTACAGCATTATAGTTACCC TCGGACG |
| | **21-225_5E5.017** | | SEQ ID NO:3967 | SEQ ID NO:11979 | SEQ ID NO:19991 |
| | | AA | RASQGIRNDLG | AASSLQS | LQHYSYPRT |
| | | | SEQ ID NO:3968 | SEQ ID NO:11980 | SEQ ID NO:19992 |
| **iPS:442220** | | NA | CGGGCAAGTCAGGGCATTAG AAATGATTTAGGC | GCTGCATCCAGTTTGCAA AGT | CTACAGCATTATAGTTACCC TCGGACG |
| | **21-225_5E5.018** | | SEQ ID NO:3969 | SEQ ID NO:11981 | SEQ ID NO:19993 |
| | | AA | RASQGIRNDLG | AASSLQS | LQHYSYPRT |
| | | | SEQ ID NO:3970 | SEQ ID NO:11982 | SEQ ID NO:19994 |
| **iPS:442227** | | NA | CGGGCAAGTCAGGGCATTAG AAATGATTTAGGC | GCTGCATCCAGTTTGCAA AGT | CTACAGCATTATAGTTACCC TCGGACG |
| | **21-225_5E5.019** | | SEQ ID NO:3971 | SEQ ID NO:11983 | SEQ ID NO:19995 |
| | | AA | RASQGIRNDLG | AASSLQS | LQHYSYPRT |
| | | | SEQ ID NO:3972 | SEQ ID NO:11984 | SEQ ID NO:19996 |
| **iPS:442255** | | NA | CGGGCAAGTCAGGGCATTAG AAATGATTTAGGC | GCTGCATCCAGTTTGCAA AGT | CTACAGCATTATAGTTACCC TCGGACG |
| | **21-225_5E5.023** | | SEQ ID NO:3973 | SEQ ID NO:11985 | SEQ ID NO:19997 |
| | | AA | RASQGIRNDLG | AASSLQS | LQHYSYPRT |
| | | | SEQ ID NO:3974 | SEQ ID NO:11986 | SEQ ID NO:19998 |
| **iPS:442262** | | NA | CGGGCAAGTCAGGGCATTAG AAATGATTTAGGC | GCTGCATCCAGTTTGCAA AGT | CTACAGCATTATAGTTACCC TCGGACG |

FIGURE 49

| | | | | | |
|---|---|---|---|---|---|
| | **21-225_5E5.024** | | SEQ ID NO:3975 | SEQ ID NO:11987 | SEQ ID NO:19999 |
| | | AA | RASQGIRNDLG | AASSLQS | LQHYSYPRT |
| | | | SEQ ID NO:3976 | SEQ ID NO:11988 | SEQ ID NO:20000 |
| **iPS:442269** | **21-225_5E5.025** | NA | CGGGCAAGTCAGGGCATTAG AAATGATTTAGGC | GCTGCATCCAGTTTGCAA AGT | CTACAGCATTATAGTTACCC TCGGACG |
| | | | SEQ ID NO:3977 | SEQ ID NO:11989 | SEQ ID NO:20001 |
| | | AA | RASQGIRNDLG | AASSLQS | LQHYSYPRT |
| | | | SEQ ID NO:3978 | SEQ ID NO:11990 | SEQ ID NO:20002 |
| **iPS:442311** | **21-225_7E11.001.001** | NA | CGGGCAAGTCAAAACATTAT CAGCTATTTAAAT | ACTGCATCCAGTTTGCAA AGT | CAACAGACTTACAGTACCCC GCTCACT |
| | | | SEQ ID NO:3979 | SEQ ID NO:11991 | SEQ ID NO:20003 |
| | | AA | RASQNIISYLN | TASSLQS | QQTYSTPLT |
| | | | SEQ ID NO:3980 | SEQ ID NO:11992 | SEQ ID NO:20004 |
| **iPS:442317** | **21-225_7E11.001.002** | NA | CGGGCAAGTCAAAACATTAT CAGCTATTTAAAT | ACTGCATCCAGTTTGCAA AGT | CAACAGACTTACAGTACCCC GCTCACT |
| | | | SEQ ID NO:3981 | SEQ ID NO:11993 | SEQ ID NO:20005 |
| | | AA | RASQNIISYLN | TASSLQS | QQTYSTPLT |
| | | | SEQ ID NO:3982 | SEQ ID NO:11994 | SEQ ID NO:20006 |
| **iPS:442323** | **21-225_7E11.001.003** | NA | CGGGCAAGTCAAAACATTAT CAGCTATTTAAAT | ACTGCATCCAGTTTGCAA AGT | CAACAGACTTACAGTACCCC GCTCACT |
| | | | SEQ ID NO:3983 | SEQ ID NO:11995 | SEQ ID NO:20007 |
| | | AA | RASQNIISYLN | TASSLQS | QQTYSTPLT |
| | | | SEQ ID NO:3984 | SEQ ID NO:11996 | SEQ ID NO:20008 |
| **iPS:442330** | **21-225_7E11.001.004** | NA | CGGGCAAGTCAAAACATTAT CAGCTATTTAAAT | ACTGCATCCAGTTTGCAA AGT | CAACAGACTTACAGTACCCC GCTCACT |
| | | | SEQ ID NO:3985 | SEQ ID NO:11997 | SEQ ID NO:20009 |
| | | AA | RASQNIISYLN | TASSLQS | QQTYSTPLT |
| | | | SEQ ID NO:3986 | SEQ ID NO:11998 | SEQ ID NO:20010 |
| **iPS:442337** | | NA | CGGGCAAGTCAAAACATTAT CAGCTATTTAAAT | ACTGCATCCAGTTTGCAA AGT | CAACAGACTTACAGTACCCC GCTCACT |

**FIGURE 49**

| | | | | | |
|---|---|---|---|---|---|
| | **21-225_7E11.001.005** | | SEQ ID NO:3987 | SEQ ID NO:11999 | SEQ ID NO:20011 |
| | | AA | RASQNIISYLN | TASSLQS | QQTYSTPLT |
| | | | SEQ ID NO:3988 | SEQ ID NO:12000 | SEQ ID NO:20012 |
| **iPS:442344** | | NA | CGGGCAAGTCAAAACATTAT CAGCTATTTAAAT | ACTGCATCCAGTTTGCAA AGT | CAACAGACTTACAGTACCCC GCTCACT |
| | **21-225_7E11.001.006** | | SEQ ID NO:3989 | SEQ ID NO:12001 | SEQ ID NO:20013 |
| | | AA | RASQNIISYLN | TASSLQS | QQTYSTPLT |
| | | | SEQ ID NO:3990 | SEQ ID NO:12002 | SEQ ID NO:20014 |
| **iPS:442351** | | NA | CGGGCAAGTCAAAACATTAT CAGCTATTTAAAT | ACTGCATCCAGTTTGCAA AGT | CAACAGACTTACAGTACCCC GCTCACT |
| | **21-225_7E11.001.007** | | SEQ ID NO:3991 | SEQ ID NO:12003 | SEQ ID NO:20015 |
| | | AA | RASQNIISYLN | TASSLQS | QQTYSTPLT |
| | | | SEQ ID NO:3992 | SEQ ID NO:12004 | SEQ ID NO:20016 |
| **iPS:442358** | | NA | CGGGCAAGTCAAAACATTAT CAGCTATTTAAAT | ACTGCATCCAGTTTGCAA AGT | CAACAGACTTACAGTACCCC GCTCACT |
| | **21-225_7E11.001.008** | | SEQ ID NO:3993 | SEQ ID NO:12005 | SEQ ID NO:20017 |
| | | AA | RASQNIISYLN | TASSLQS | QQTYSTPLT |
| | | | SEQ ID NO:3994 | SEQ ID NO:12006 | SEQ ID NO:20018 |
| **iPS:442365** | | NA | CGGGCAAGTCAAAACATTAT CAGCTATTTAAAT | ACTGCATCCAGTTTGCAA AGT | CAACAGACTTACAGTACCCC GCTCACT |
| | **21-225_7E11.001.009** | | SEQ ID NO:3995 | SEQ ID NO:12007 | SEQ ID NO:20019 |
| | | AA | RASQNIISYLN | TASSLQS | QQTYSTPLT |
| | | | SEQ ID NO:3996 | SEQ ID NO:12008 | SEQ ID NO:20020 |
| **iPS:442372** | | NA | CGGGCAAGTCAAAACATTAT CAGCTATTTAAAT | ACTGCATCCAGTTTGCAA AGT | CAACAGACTTACAGTACCCC GCTCACT |
| | **21-225_7E11.001.010** | | SEQ ID NO:3997 | SEQ ID NO:12009 | SEQ ID NO:20021 |
| | | AA | RASQNIISYLN | TASSLQS | QQTYSTPLT |
| | | | SEQ ID NO:3998 | SEQ ID NO:12010 | SEQ ID NO:20022 |
| **iPS:442379** | | NA | CGGGCAAGTCAAAACATTAT CAGCTATTTAAAT | ACTGCATCCAGTTTGCAA AGT | CAACAGACTTACAGTACCCC GCTCACT |

FIGURE 49

| | | | | | |
|---|---|---|---|---|---|
| | **21-225_7E11.001.011** | | SEQ ID NO:3999 | SEQ ID NO:12011 | SEQ ID NO:20023 |
| | | AA | RASQNIISYLN | TASSLQS | QQTYSTPLT |
| | | | SEQ ID NO:4000 | SEQ ID NO:12012 | SEQ ID NO:20024 |
| **iPS:442386** | | NA | CGGGCAAGTCAAAACATTAT CAGCTATTTAAAT | ACTGCATCCAGTTTGCAA AGT | CAACAGACTTACAGTACCCC GCTCACT |
| | **21-225_7E11.001.012** | | SEQ ID NO:4001 | SEQ ID NO:12013 | SEQ ID NO:20025 |
| | | AA | RASQNIISYLN | TASSLQS | QQTYSTPLT |
| | | | SEQ ID NO:4002 | SEQ ID NO:12014 | SEQ ID NO:20026 |
| **iPS:442390** | | NA | CGGGCAAGTCAAAACATTAT CAGCTATTTAAAT | ACTGCATCCAGTTTGCAA AGT | CAACAGACTTACAGTACCCC GCTCACT |
| | **21-225_7E11.001.013** | | SEQ ID NO:4003 | SEQ ID NO:12015 | SEQ ID NO:20027 |
| | | AA | RASQNIISYLN | TASSLQS | QQTYSTPLT |
| | | | SEQ ID NO:4004 | SEQ ID NO:12016 | SEQ ID NO:20028 |
| **iPS:442394** | | NA | CGGGCAAGTCAAAACATTAT CAGCTATTTAAAT | ACTGCATCCAGTTTGCAA AGT | CAACAGACTTACAGTACCCC GCTCACT |
| | **21-225_7E11.001.014** | | SEQ ID NO:4005 | SEQ ID NO:12017 | SEQ ID NO:20029 |
| | | AA | RASQNIISYLN | TASSLQS | QQTYSTPLT |
| | | | SEQ ID NO:4006 | SEQ ID NO:12018 | SEQ ID NO:20030 |
| **iPS:442398** | | NA | CGGGCAAGTCAAAACATTAT CAGCTATTTAAAT | ACTGCATCCAGTTTGCAA AGT | CAACAGACTTACAGTACCCC GCTCACT |
| | **21-225_7E11.001.015** | | SEQ ID NO:4007 | SEQ ID NO:12019 | SEQ ID NO:20031 |
| | | AA | RASQNIISYLN | TASSLQS | QQTYSTPLT |
| | | | SEQ ID NO:4008 | SEQ ID NO:12020 | SEQ ID NO:20032 |
| **iPS:442402** | | NA | CGGGCAAGTCAAAACATTAT CAGCTATTTAAAT | ACTGCATCCAGTTTGCAA AGT | CAACAGACTTACAGTACCCC GCTCACT |
| | **21-225_7E11.001.016** | | SEQ ID NO:4009 | SEQ ID NO:12021 | SEQ ID NO:20033 |
| | | AA | RASQNIISYLN | TASSLQS | QQTYSTPLT |
| | | | SEQ ID NO:4010 | SEQ ID NO:12022 | SEQ ID NO:20034 |
| **iPS:442406** | | NA | CGGGCAAGTCAAAACATTAT CAGCTATTTAAAT | ACTGCATCCAGTTTGCAA AGT | CAACAGACTTACAGTACCCC GCTCACT |

FIGURE 49

| | | | | | |
|---|---|---|---|---|---|
| | **21-225_7E11.001.017** | | SEQ ID NO:4011 | SEQ ID NO:12023 | SEQ ID NO:20035 |
| | | AA | RASQNIISYLN | TASSLQS | QQTYSTPLT |
| | | | SEQ ID NO:4012 | SEQ ID NO:12024 | SEQ ID NO:20036 |
| **iPS:442410** | **21-225_7E11.001.018** | NA | CGGGCAAGTCAAAACATTAT CAGCTATTTAAAT | ACTGCATCCAGTTTGCAA AGT | CAACAGACTTACAGTACCCC GCTCACT |
| | | | SEQ ID NO:4013 | SEQ ID NO:12025 | SEQ ID NO:20037 |
| | | AA | RASQNIISYLN | TASSLQS | QQTYSTPLT |
| | | | SEQ ID NO:4014 | SEQ ID NO:12026 | SEQ ID NO:20038 |
| **iPS:442417** | **21-225_7E11.001.019** | NA | CGGGCAAGTCAAAACATTAT CAGCTATTTAAAT | ACTGCATCCAGTTTGCAA AGT | CAACAGACTTACAGTACCCC GCTCACT |
| | | | SEQ ID NO:4015 | SEQ ID NO:12027 | SEQ ID NO:20039 |
| | | AA | RASQNIISYLN | TASSLQS | QQTYSTPLT |
| | | | SEQ ID NO:4016 | SEQ ID NO:12028 | SEQ ID NO:20040 |
| **iPS:442431** | **21-225_7E11.001.021** | NA | CGGGCAAGTCAAAACATTAT CAGCTATTTAAAT | ACTGCATCCAGTTTGCAA AGT | CAACAGACTTACAGTACCCC GCTCACT |
| | | | SEQ ID NO:4017 | SEQ ID NO:12029 | SEQ ID NO:20041 |
| | | AA | RASQNIISYLN | TASSLQS | QQTYSTPLT |
| | | | SEQ ID NO:4018 | SEQ ID NO:12030 | SEQ ID NO:20042 |
| **iPS:442438** | **21-225_7E11.001.022** | NA | CGGGCAAGTCAAAACATTAT CAGCTATTTAAAT | ACTGCATCCAGTTTGCAA AGT | CAACAGACTTACAGTACCCC GCTCACT |
| | | | SEQ ID NO:4019 | SEQ ID NO:12031 | SEQ ID NO:20043 |
| | | AA | RASQNIISYLN | TASSLQS | QQTYSTPLT |
| | | | SEQ ID NO:4020 | SEQ ID NO:12032 | SEQ ID NO:20044 |
| **iPS:442568** | **21-225_149D8** | NA | AGGGCCAGTCAGAGTGTGAT CAGCAGCTACTTAGCC | GGTGTATCTAGTTGGGCC ACT | CAACAATATGGTAGGTCACC ATTCAAT |
| | | | SEQ ID NO:4021 | SEQ ID NO:12033 | SEQ ID NO:20045 |
| | | AA | RASQSVISSYLA | GVSSWAT | QQYGRSPFN |
| | | | SEQ ID NO:4022 | SEQ ID NO:12034 | SEQ ID NO:20046 |

FIGURE 49

| | | | | | |
|---|---|---|---|---|---|
| **iPS:443003** | **21-225_43F11_LC2** | NA | ACTGGGAGCAGCTCCAACATCGGGGCAGGTTATGATGTACAC | GGTAACAGCAATCGGCCCTCA | CAGTCCTATGACAACAGCCTGAGTGGTTCGGTA |
| | | | SEQ ID NO:4023 | SEQ ID NO:12035 | SEQ ID NO:20047 |
| | | AA | TGSSSNIGAGYDVH | GNSNRPS | QSYDNSLSGSV |
| | | | SEQ ID NO:4024 | SEQ ID NO:12036 | SEQ ID NO:20048 |
| **iPS:443005** | **21-225_43F11_LC1** | NA | AGGTCTAGTCAAAGCCTCGTATACAGTGATGGAAACACCTACTTGAAT | AAGGTTTCTAACTGGGACTCT | ATGCAAGGTACACACTGGCCGCTCACT |
| | | | SEQ ID NO:4025 | SEQ ID NO:12037 | SEQ ID NO:20049 |
| | | AA | RSSQSLVYSDGNTYLN | KVSNWDS | MQGTHWPLT |
| | | | SEQ ID NO:4026 | SEQ ID NO:12038 | SEQ ID NO:20050 |
| **iPS:443006** | **21-225_25A4.001.029** | NA | AAGTCCAGCCAGAGTGTTTTATACAGCTCCCACAATAACAACTACTTAGCT | TGGGCATCTACCCGGGAATCC | CAGCAGTATTATAGTACTCCTCCGACG |
| | | | SEQ ID NO:4027 | SEQ ID NO:12039 | SEQ ID NO:20051 |
| | | AA | KSSQSVLYSSHNNNYLA | WASTRES | QQYYSTPPT |
| | | | SEQ ID NO:4028 | SEQ ID NO:12040 | SEQ ID NO:20052 |
| **iPS:443016** | **21-225_4H6.014** | NA | CGGGCGAGTCAGGGTATTAGCAGGTGGTTAGCC | GGTGCATCCAGTTTGCAAAGT | CAACAGGCTAACAGTTTCCCATTCACT |
| | | | SEQ ID NO:4029 | SEQ ID NO:12041 | SEQ ID NO:20053 |
| | | AA | RASQGISRWLA | GASSLQS | QQANSFPFT |
| | | | SEQ ID NO:4030 | SEQ ID NO:12042 | SEQ ID NO:20054 |
| **iPS:443027** | **21-225_7E11.001.023** | NA | CGGGCAAGTCAAAACATTATCAGCTATTTAAAT | ACTGCATCCAGTTTGCAAAGT | CAACAGACTTACAGTACCCCGCTCACT |
| | | | SEQ ID NO:4031 | SEQ ID NO:12043 | SEQ ID NO:20055 |
| | | AA | RASQNIISYLN | TASSLQS | QQTYSTPLT |
| | | | SEQ ID NO:4032 | SEQ ID NO:12044 | SEQ ID NO:20056 |

FIGURE 49

| | | | | | |
|---|---|---|---|---|---|
| **iPS:446086** | **21-225_94D8** | NA | AAGTCCAGACAGAGTGTTTTATACAGCTCCAACAATTACAACTACTTAACT | TGGGCATCTACCCGGGAATCC | CAGCAATATTATAGTTCTCCTCCTACT |
| | | | SEQ ID NO:4033 | SEQ ID NO:12045 | SEQ ID NO:20057 |
| | | AA | KSRQSVLYSSNNYNYLT | WASTRES | QQYYSSPPT |
| | | | SEQ ID NO:4034 | SEQ ID NO:12046 | SEQ ID NO:20058 |
| **iPS:446094** | **21-225_77E1** | NA | AAGTCCAGCCAGACTGTCTTACACAGCTCCAACAATTATAACTACTTAGCT | TGGACATCTACCCGGGAATCC | CACCAATATCTTAGTAGTCCTCTGACG |
| | | | SEQ ID NO:4035 | SEQ ID NO:12047 | SEQ ID NO:20059 |
| | | AA | KSSQTVLHSSNNYNYLA | WTSTRES | HQYLSSPLT |
| | | | SEQ ID NO:4036 | SEQ ID NO:12048 | SEQ ID NO:20060 |
| **iPS:448904** | **21-225_65C12** | NA | AGGGCCAGTCAGAGTGTTAGCATCAACTTAGCC | GGTGCATCCACCAGGGCCACT | CAGCAGTATAATACCTGGCCTCTCACT |
| | | | SEQ ID NO:4037 | SEQ ID NO:12049 | SEQ ID NO:20061 |
| | | AA | RASQSVSINLA | GASTRAT | QQYNTWPLT |
| | | | SEQ ID NO:4038 | SEQ ID NO:12050 | SEQ ID NO:20062 |
| **iPS:448906** | **21-225_72G9** | NA | CGGGCAAGTCAGAGCATTACCAGCTATTTAAAT | ACTGCATCCAGTTTGCAAAGT | CAACAGAGTCACAGTTTCCCATTCACT |
| | | | SEQ ID NO:4039 | SEQ ID NO:12051 | SEQ ID NO:20063 |
| | | AA | RASQSITSYLN | TASSLQS | QQSHSFPFT |
| | | | SEQ ID NO:4040 | SEQ ID NO:12052 | SEQ ID NO:20064 |
| **iPS:448908** | **21-225_50G9** | NA | TCTGGAGATAAATTGGGGGATAAATATGCTTGC | CAAGATAGCAAGCGGCCCTCA | CAGGCGCGGAACAGCCGCAGAGGGGTA |
| | | | SEQ ID NO:4041 | SEQ ID NO:12053 | SEQ ID NO:20065 |
| | | AA | SGDKLGDKYAC | QDSKRPS | QARNSRRGV |
| | | | SEQ ID NO:4042 | SEQ ID NO:12054 | SEQ ID NO:20066 |
| **iPS:451102** | **21-225_45F6** | NA | TCTGGAGATAAATTGGGGGATAAATATGCTTCC | CAAGATAGTAAGCGGCCCTCA | CAGGCGTGGGACAACAGAACTATGGTA |
| | | | SEQ ID NO:4043 | SEQ ID NO:12055 | SEQ ID NO:20067 |

FIGURE 49

| | | | | | |
|---|---|---|---|---|---|
| | | AA | SGDKLGDKYAS | QDSKRPS | QAWDNRTMV |
| | | | SEQ ID NO:4044 | SEQ ID NO:12056 | SEQ ID NO:20068 |
| **iPS:451104** | **21-225_49C5** | NA | TCTGGAAGCAGCTCCAACAT CGGAAGTAATATTGTGACC | AGTAATGATCAGCGGCC CTCA | ACAGCATGGGATGACAGCCT GAATGGTTGGGTG |
| | | | SEQ ID NO:4045 | SEQ ID NO:12057 | SEQ ID NO:20069 |
| | | AA | SGSSSNIGSNIVT | SNDQRPS | TAWDDSLNGWV |
| | | | SEQ ID NO:4046 | SEQ ID NO:12058 | SEQ ID NO:20070 |
| **iPS:451106** | **21-225_49D10** | NA | TCTGGAAGCAACTCCAACAT CGGAAGTAATATTGTAACC | AGTAATGATCAGCGGCC CTCA | GCAGCATGGGATGACAGCCT GAATGGTTGGGTG |
| | | | SEQ ID NO:4047 | SEQ ID NO:12059 | SEQ ID NO:20071 |
| | | AA | SGSNSNIGSNIVT | SNDQRPS | AAWDDSLNGWV |
| | | | SEQ ID NO:4048 | SEQ ID NO:12060 | SEQ ID NO:20072 |
| **iPS:451108** | **21-225_53E8** | NA | TCTGGAAGCTGCTCCAACAT CGGAAGTAATATTGTGACC | AGTAATGATCAGCGGCC CTCA | ACAGCATGGGATGACAGCCT GAATGATTGGGTG |
| | | | SEQ ID NO:4049 | SEQ ID NO:12061 | SEQ ID NO:20073 |
| | | AA | SGSCSNIGSNIVT | SNDQRPS | TAWDDSLNDWV |
| | | | SEQ ID NO:4050 | SEQ ID NO:12062 | SEQ ID NO:20074 |
| **iPS:451110** | **21-225_74C9** | NA | TCAGGAGATAAATCGGGGA ATAAATATGTTTCC | CAAGATAACAGGCGGCC GTCA | CAGGCGTGGGACAGCACCCC TGTGATA |
| | | | SEQ ID NO:4051 | SEQ ID NO:12063 | SEQ ID NO:20075 |
| | | AA | SGDKSGNKYVS | QDNRRPS | QAWDSTPVI |
| | | | SEQ ID NO:4052 | SEQ ID NO:12064 | SEQ ID NO:20076 |
| **iPS:451112** | **21-225_53D10** | NA | TCTGGAGATAAATTGGGGAA TAAATATGCTTGC | CAAGATCGCAAGCGGCC CTCA | CAGGCGTGGGACAGCAGCAC TGTGGTA |
| | | | SEQ ID NO:4053 | SEQ ID NO:12065 | SEQ ID NO:20077 |
| | | AA | SGDKLGNKYAC | QDRKRPS | QAWDSSTVV |
| | | | SEQ ID NO:4054 | SEQ ID NO:12066 | SEQ ID NO:20078 |

FIGURE 49

| | | | | | |
|---|---|---|---|---|---|
| **iPS:451114** | **21-225_159A3** | NA | CGGGCAAGTCAGGACATTAGAAAGGATTTAGGC | GCTGCATCCAGTTTGCAAAGT | CTACAGCATCATAGTTATCCTCGGACG |
| | | | SEQ ID NO:4055 | SEQ ID NO:12067 | SEQ ID NO:20079 |
| | | AA | RASQDIRKDLG | AASSLQS | LQHHSYPRT |
| | | | SEQ ID NO:4056 | SEQ ID NO:12068 | SEQ ID NO:20080 |
| **iPS:451116** | **21-225_164A4** | NA | AAGTCCAGCCAGAGTGTTTTATACAGCTCCAACAATAAGAACTACTTAACT | TGGGCATCTACCCGGGAATCC | CAGCAATATTTTAGTACTCCGTGGACG |
| | | | SEQ ID NO:4057 | SEQ ID NO:12069 | SEQ ID NO:20081 |
| | | AA | KSSQSVLYSSNNKNYLT | WASTRES | QQYFSTPWT |
| | | | SEQ ID NO:4058 | SEQ ID NO:12070 | SEQ ID NO:20082 |
| **iPS:451118** | **21-225_191C8** | NA | AGGGCCAGTCAGAGTGTTCGCAGTAACTTAGCC | GGTGCATCCACCAGGGCCACT | CAGCAGTCTTTTACCTGGCTCCGGACG |
| | | | SEQ ID NO:4059 | SEQ ID NO:12071 | SEQ ID NO:20083 |
| | | AA | RASQSVRSNLA | GASTRAT | QQSFTWLRT |
| | | | SEQ ID NO:4060 | SEQ ID NO:12072 | SEQ ID NO:20084 |
| **iPS:451120** | **21-225_197D3** | NA | CGGGCGAGTCAGGGCATTAGAAATTATTTAGCC | GCTGCATCCAGTTTGCAAAGT | CAACATTATCTTACTTACCCGCTCACT |
| | | | SEQ ID NO:4061 | SEQ ID NO:12073 | SEQ ID NO:20085 |
| | | AA | RASQGIRNYLA | AASSLQS | QHYLTYPLT |
| | | | SEQ ID NO:4062 | SEQ ID NO:12074 | SEQ ID NO:20086 |
| **iPS:451122** | **21-225_200A1** | NA | AGGGCCAGTCAGAGTGTTAACAGCAACTATTTAGCC | GGGGCATCCAGCAGGGCCACT | CAGCAGTATGAGATCTCACCGTGGACG |
| | | | SEQ ID NO:4063 | SEQ ID NO:12075 | SEQ ID NO:20087 |
| | | AA | RASQSVNSNYLA | GASSRAT | QQYEISPWT |
| | | | SEQ ID NO:4064 | SEQ ID NO:12076 | SEQ ID NO:20088 |
| **iPS:451124** | **21-225_74F6** | NA | AAGTCCAGTCAGAATATTTTATCCAGCTCCAACAATAAGAACTACTTAACT | TGGACATCTACCCGGGAATCC | CAGCAATATTTTAGTGTTCCTCTGACG |
| | | | SEQ ID NO:4065 | SEQ ID NO:12077 | SEQ ID NO:20089 |

FIGURE 49

| | | | | | |
|---|---|---|---|---|---|
| | | AA | KSSQNILSSSNNKNYLT | WTSTRES | QQYFSVPLT |
| | | | SEQ ID NO:4066 | SEQ ID NO:12078 | SEQ ID NO:20090 |
| **iPS:451127** | **21-225_164A7** | NA | AAGTCCAGCCAGAGTGTTTTACACAGCTCCAACAATAACAACTACTTAGCT | TGGACATCTACCCGGGAATCC | CAGCAATATTATAGTATTCCTCTGACG |
| | | | SEQ ID NO:4067 | SEQ ID NO:12079 | SEQ ID NO:20091 |
| | | AA | KSSQSVLHSSNNNNYLA | WTSTRES | QQYYSIPLT |
| | | | SEQ ID NO:4068 | SEQ ID NO:12080 | SEQ ID NO:20092 |
| **iPS:451129** | **21-225_94D2** | NA | AAGTCCAGCCAGAGTGTTTTACACAGCTCCAACAATAAGAACTACTTAACT | TGGGCATCTACTCGGGAATCC | CAGCAATATCATAGTATTCCTCCGACG |
| | | | SEQ ID NO:4069 | SEQ ID NO:12081 | SEQ ID NO:20093 |
| | | AA | KSSQSVLHSSNNKNYLT | WASTRES | QQYHSIPPT |
| | | | SEQ ID NO:4070 | SEQ ID NO:12082 | SEQ ID NO:20094 |
| **iPS:451131** | **21-225_160A7** | NA | AAGTCCAGCCAGAGTGTTTTATCCAACTCCCACAATAACAACTACTTAGCT | TGGGCATCTACCCGGGAATCC | CAGCAATATTATAGTACTCCGTGCAGT |
| | | | SEQ ID NO:4071 | SEQ ID NO:12083 | SEQ ID NO:20095 |
| | | AA | KSSQSVLSNSHNNNYLA | WASTRES | QQYYSTPCS |
| | | | SEQ ID NO:4072 | SEQ ID NO:12084 | SEQ ID NO:20096 |
| **iPS:451133** | **21-225_95H4** | NA | AAGTCCAGCCAGAGTGTTTTATTCAGCTCCAACAATTATAATTACTTAGCT | TGGGCATCTACCCGGGAATCC | CAGCAATATCATAGTTCTCCTCTGACG |
| | | | SEQ ID NO:4073 | SEQ ID NO:12085 | SEQ ID NO:20097 |
| | | AA | KSSQSVLFSSNNYNYLA | WASTRES | QQYHSSPLT |
| | | | SEQ ID NO:4074 | SEQ ID NO:12086 | SEQ ID NO:20098 |
| **iPS:437240** | **21-225_84H12** | NA | CGGGCAAGTCAGGGCATTAGAAATGATTTAGGC | GCTGCATCCAGTTTGCAAAGT | TTACAGCATAATGATTACCCATTCACT |
| | | | SEQ ID NO:4075 | SEQ ID NO:12087 | SEQ ID NO:20099 |
| | | AA | RASQGIRNDLG | AASSLQS | LQHNDYPFT |

# FIGURE 49

| | | | | | |
|---|---|---|---|---|---|
| | | | SEQ ID NO:4076 | SEQ ID NO:12088 | SEQ ID NO:20100 |
| **iPS:434577** | **21-225_75C11** | NA | CGGGCAAGTCAGGGCATTAG AAATGATTTAGGC | GCTGCATCCAGTTTGCAA AGT | CTACAGCATAATGATTACCC ATTCACT |
| | | | SEQ ID NO:4077 | SEQ ID NO:12089 | SEQ ID NO:20101 |
| | | AA | RASQGIRNDLG | AASSLQS | LQHNDYPFT |
| | | | SEQ ID NO:4078 | SEQ ID NO:12090 | SEQ ID NO:20102 |
| **iPS:435477** | **21-225_154E8** | NA | CGGGCGAGTCAGTTTATTAG CAGCTGGTTAGCC | ACTGCATCCAGTTTGCAA AGT | CAACAGGCTAACAGTTTCCC GTGGACG |
| | | | SEQ ID NO:4079 | SEQ ID NO:12091 | SEQ ID NO:20103 |
| | | AA | RASQFISSWLA | TASSLQS | QQANSFPWT |
| | | | SEQ ID NO:4080 | SEQ ID NO:12092 | SEQ ID NO:20104 |
| **iPS:434553** | **21-225_76H12** | NA | CGGGCAAGTCAGGGCATTAG AAATGATTTAGGC | GCTGCATCCAGATTGCA AAGT | CTACAGCATAATGATTACCC ATTCACT |
| | | | SEQ ID NO:4081 | SEQ ID NO:12093 | SEQ ID NO:20105 |
| | | AA | RASQGIRNDLG | AASRLQS | LQHNDYPFT |
| | | | SEQ ID NO:4082 | SEQ ID NO:12094 | SEQ ID NO:20106 |
| **iPS:434927** | **21-225_86E5** | NA | CGGGCAAGTCAGGGCATTAG AAATGATTTAGGC | GCTGCATCCAGTTTGCAA AGT | CTACAGCATAATGATTACCC ATTCACT |
| | | | SEQ ID NO:4083 | SEQ ID NO:12095 | SEQ ID NO:20107 |
| | | AA | RASQGIRNDLG | AASSLQS | LQHNDYPFT |
| | | | SEQ ID NO:4084 | SEQ ID NO:12096 | SEQ ID NO:20108 |
| **iPS:435385** | **21-225_149G7** | NA | CGGGCGAGTCAGTTTATTAG CAGCTGGTTAGCC | GCTGCATCCAGTTTGCAA AGT | CAACAGGCTAACAGTTTCCC GTGGACG |
| | | | SEQ ID NO:4085 | SEQ ID NO:12097 | SEQ ID NO:20109 |
| | | AA | RASQFISSWLA | AASSLQS | QQANSFPWT |
| | | | SEQ ID NO:4086 | SEQ ID NO:12098 | SEQ ID NO:20110 |

**Table 2B**

## FIGURE 49

**Standard**
**IgG Antibody**
**VH CDRs**

| iPS# | Ab | Type | CDR1 | CDR2 | CDR3 |
|---|---|---|---|---|---|
| **iPS:451135** | **21-225_64A11** | NA | AACTATGGCATGCAC | GTTATATCATATGTTGGAAGTAATAAATACTATGCAGACTCCGTGAAGGGC | AGAGGAGCAGTGGCTCCGTACTACGGTATGGACGTC |
| | | | SEQ ID NO:4087 | SEQ ID NO:12099 | SEQ ID NO:20111 |
| | | AA | NYGMH | VISYVGSNKYYADSVKG | RGAVAPYYGMDV |
| | | | SEQ ID NO:4088 | SEQ ID NO:12100 | SEQ ID NO:20112 |
| **iPS:451141** | **21-225_164B11** | NA | AATTATGATATCAAC | TGGATGACCCCTAACAGTGGTAACACAGGCTATGCACAGAAGTTCCAGGGC | AGCAGTGGCTGGTACATGTTTGACTAC |
| | | | SEQ ID NO:4089 | SEQ ID NO:12101 | SEQ ID NO:20113 |
| | | AA | NYDIN | WMTPNSGNTGYAQKFQG | SSGWYMFDY |
| | | | SEQ ID NO:4090 | SEQ ID NO:12102 | SEQ ID NO:20114 |
| **iPS:451137** | **21-225_74A7** | NA | AATTATGATATCAAC | TGGATGAACCCTAACAGTGGTAACACAGGCTATGCACAGAAGTTCCAGGGC | TCCAGTGGCTGGAACTGGTTCGACCCC |
| | | | SEQ ID NO:4091 | SEQ ID NO:12103 | SEQ ID NO:20115 |
| | | AA | NYDIN | WMNPNSGNTGYAQKFQG | SSGWNWFDP |
| | | | SEQ ID NO:4092 | SEQ ID NO:12104 | SEQ ID NO:20116 |
| **iPS:451139** | **21-225_71A6** | NA | AACTATGGCATGCAC | GTTATATCATATGATGGAAGTAATGAATACTATGCAGACTCCGTGAAGGGC | GATCACAGATATGGGGTTCGGGGAGGCTTTGACTAC |
| | | | SEQ ID NO:4093 | SEQ ID NO:12105 | SEQ ID NO:20117 |
| | | AA | NYGMH | VISYDGSNEYYADSVKG | DHRYGVRGGFDY |

**FIGURE 49**

| | | | | | |
|---|---|---|---|---|---|
| | | | SEQ ID NO:4094 | SEQ ID NO:12106 | SEQ ID NO:20118 |
| **iPS:451143** | **21-225_66H11** | NA | ACCTATGGTATCAGC | TGGATCAGCGCTTACAATGGTAACACAAACTATGCACAGAAGCTCCAGGGC | GGGGAAGCAGTGGCTGTCTTCGACCCC |
| | | | SEQ ID NO:4095 | SEQ ID NO:12107 | SEQ ID NO:20119 |
| | | AA | TYGIS | WISAYNGNTNYAQKLQG | GEAVAVFDP |
| | | | SEQ ID NO:4096 | SEQ ID NO:12108 | SEQ ID NO:20120 |
| **iPS:453445** | **21-225_148E10** | NA | AGCTATGGCATGCAC | GTTATATGGTTTGATGGCAGTAATAAATACTATGTAGACTCCGTGAAGGAC | GATCGGGTGGAGGGTTCGGGGACTCCCTACTACTACTACGGTATGGACGTC |
| | | | SEQ ID NO:4097 | SEQ ID NO:12109 | SEQ ID NO:20121 |
| | | AA | SYGMH | VIWFDGSNKYYVDSVKD | DRVEGSGTPYYYYGMDV |
| | | | SEQ ID NO:4098 | SEQ ID NO:12110 | SEQ ID NO:20122 |
| **iPS:453447** | **21-225_65F10** | NA | GGCTACCATATGCAC | TGGATCAACCCTAACAATGGTGGCACAAGCTATGCACAGAAGTTTCAGGAC | GATAGTAGGTCGTCCTGGGACTAC |
| | | | SEQ ID NO:4099 | SEQ ID NO:12111 | SEQ ID NO:20123 |
| | | AA | GYHMH | WINPNNGGTSYAQKFQD | DSRSSWDY |
| | | | SEQ ID NO:4100 | SEQ ID NO:12112 | SEQ ID NO:20124 |
| **iPS:453449** | **21-225_208A2** | NA | AGTTACTACTGGAGC | CGTATCTATACCAGTGGGAGCACCGACTACAACCCCTCCCTCAAGAGT | GGGTTCGGTGACTGGGACTAC |
| | | | SEQ ID NO:4101 | SEQ ID NO:12113 | SEQ ID NO:20125 |
| | | AA | SYYWS | RIYTSGSTDYNPSLKS | GFGDWDY |
| | | | SEQ ID NO:4102 | SEQ ID NO:12114 | SEQ ID NO:20126 |

FIGURE 49

| | | | | | |
|---|---|---|---|---|---|
| **iPS:453451** | **21-225_52G11** | NA | GGCTACTATTTGCAC | TGGATCAACCCTAATAGAAATGGCACAAACTATGCACAGAAGTTTCAGGGC | GACGGTACCAGCAGCTTTGACTAC |
| | | | SEQ ID NO:4103 | SEQ ID NO:12115 | SEQ ID NO:20127 |
| | | AA | GYYLH | WINPNRNGTNYAQKFQG | DGTSSFDY |
| | | | SEQ ID NO:4104 | SEQ ID NO:12116 | SEQ ID NO:20128 |
| **iPS:453453** | **21-225_53F2** | NA | GGCTACTATTTGCAC | TGGATCAACCCTAACAGAAATGGCACAAACTATGCACAGAATTTTCAGGGC | GACGGTACCAGTAGCTTTGACTAC |
| | | | SEQ ID NO:4105 | SEQ ID NO:12117 | SEQ ID NO:20129 |
| | | AA | GYYLH | WINPNRNGTNYAQNFQG | DGTSSFDY |
| | | | SEQ ID NO:4106 | SEQ ID NO:12118 | SEQ ID NO:20130 |
| **iPS:468810** | **21-225_74D5** | NA | GGTTGCTACTGGAGC | GAAATCAATTATAGTGGAAGGACCAACTTCAACCCGTCCCTCAAGAGT | GACTACGGCGGTATGGACGTC |
| | | | SEQ ID NO:4107 | SEQ ID NO:12119 | SEQ ID NO:20131 |
| | | AA | GCYWS | EINYSGRTNFNPSLKS | DYGGMDV |
| | | | SEQ ID NO:4108 | SEQ ID NO:12120 | SEQ ID NO:20132 |
| **iPS:468812** | **21-225_48H4** | NA | GACTCTCTCATGCAC | GTTATATGGTATGATGGAAGTAATAAATACTATACAGACTCCGTTAAGGGC | GAAAACTATAGCAGTGGCTGGTACGGGTACGGTATGGACGTC |
| | | | SEQ ID NO:4109 | SEQ ID NO:12121 | SEQ ID NO:20133 |
| | | AA | DSLMH | VIWYDGSNKYYTDSVKG | ENYSSGWYGYGMDV |
| | | | SEQ ID NO:4110 | SEQ ID NO:12122 | SEQ ID NO:20134 |

FIGURE 49

| | | | | | |
|---|---|---|---|---|---|
| **iPS:468816** | **21-225_52G8** | NA | AGCTATGGCATGCAC | GTTATATGGTATGATGGAAGTAATAAATACTATGCAGACTCCGTGAAGGGC | AGGTATAGCAGCAGCTGGTCGGGCGGTATGGACGTC |
| | | | SEQ ID NO:4111 | SEQ ID NO:12123 | SEQ ID NO:20135 |
| | | AA | SYGMH | VIWYDGSNKYYADSVKG | RYSSSWSGGMDV |
| | | | SEQ ID NO:4112 | SEQ ID NO:12124 | SEQ ID NO:20136 |
| **iPS:468814** | **21-225_223D11** | NA | AACTATGGCATGGAC | GTTATATGGTATGATGGAAGTAATGACTACTATGCAGACTCCGTGAAGGGC | GATCGGGGGATCGGGTACAACGATATGGACGTC |
| | | | SEQ ID NO:4113 | SEQ ID NO:12125 | SEQ ID NO:20137 |
| | | AA | NYGMD | VIWYDGSNDYYADSVKG | DRGIGYNDMDV |
| | | | SEQ ID NO:4114 | SEQ ID NO:12126 | SEQ ID NO:20138 |
| **iPS:468822** | **21-225_147E10** | NA | AACTATGGCTTACAC | ATTATATGGTATGATGGAAGTAATAAATACTATGCAGACTCCGTGAAGGGC | GATCATTACGATTTTTGGAGTGGTCACTTTGACTAC |
| | | | SEQ ID NO:4115 | SEQ ID NO:12127 | SEQ ID NO:20139 |
| | | AA | NYGLH | IIWYDGSNKYYADSVKG | DHYDFWSGHFDY |
| | | | SEQ ID NO:4116 | SEQ ID NO:12128 | SEQ ID NO:20140 |
| **iPS:468824** | **21-225_73G6** | NA | AGCTATGGCATGCAC | GTTATATGGTATGATGTAAGTAATAAATACTATGGAGACTCCGTGAAGGGC | GAAGTGGGGATGACTTCTGACTAC |
| | | | SEQ ID NO:4117 | SEQ ID NO:12129 | SEQ ID NO:20141 |
| | | AA | SYGMH | VIWYDVSNKYYGDSVKG | EVGMTSDY |
| | | | SEQ ID NO:4118 | SEQ ID NO:12130 | SEQ ID NO:20142 |

FIGURE 49

| | | | | | |
|---|---|---|---|---|---|
| **iPS:468818** | **21-225_190C8** | NA | AGTTATGATATCAAC | TGGATGAACCCTAAAAGGGGTAACACAGGCTATGCACAGAAGTTCCAGGGC | GGAGACCCGTATAACTGGAACTCCTACGCTATGGACGTC |
| | | | SEQ ID NO:4119 | SEQ ID NO:12131 | SEQ ID NO:20143 |
| | | AA | SYDIN | WMNPKRGNTGYAQKFQG | GDPYNWNSYAMDV |
| | | | SEQ ID NO:4120 | SEQ ID NO:12132 | SEQ ID NO:20144 |
| **iPS:468826** | **21-225_201C5** | NA | GACTATGTCATGCAC | GTTATATGGTATGATGGAAGTAATAAATACTATGTAGACTCCGTGAAGGGC | GAACGGTATAGCAGTGGCTTGTACGACTACGGTATGGACGTC |
| | | | SEQ ID NO:4121 | SEQ ID NO:12133 | SEQ ID NO:20145 |
| | | AA | DYVMH | VIWYDGSNKYYVDSVKG | ERYSSGLYDYGMDV |
| | | | SEQ ID NO:4122 | SEQ ID NO:12134 | SEQ ID NO:20146 |
| **iPS:468828** | **21-225_162A10** | NA | AGCTGTGGCATGCAC | GCTATATGGTATGATGGAAGCAATAAATACTATGCAGACTCCGTGAAGGGC | GACAAAAATATAATGGGAGATACTTGGTTTGACTTC |
| | | | SEQ ID NO:4123 | SEQ ID NO:12135 | SEQ ID NO:20147 |
| | | AA | SCGMH | AIWYDGSNKYYADSVKG | DKNIMGDTWFDF |
| | | | SEQ ID NO:4124 | SEQ ID NO:12136 | SEQ ID NO:20148 |
| **iPS:468830** | **21-225_191G11** | NA | GGCTACTATATGCAC | TGGATCAACCCTAATAGTGGTGGCACAAACTTTGCACAGAAGTTTCAGGGC | GGAAAGAACTATGGCTCCTACTTTGACTAC |
| | | | SEQ ID NO:4125 | SEQ ID NO:12137 | SEQ ID NO:20149 |
| | | AA | GYYMH | WINPNSGGTNFAQKFQG | GKNYGSYFDY |
| | | | SEQ ID NO:4126 | SEQ ID NO:12138 | SEQ ID NO:20150 |

FIGURE 49

| | | | | | |
|---|---|---|---|---|---|
| **iPS:468832** | **21-225_76H10** | NA | GGTTGCTACTGGAGC | GAAATCAATTATAGTGGAAGGACCAACTTCAACCCGTCCCTCAAGAGT | GACTACGGCGGTATGGACGTC |
| | | | SEQ ID NO:4127 | SEQ ID NO:12139 | SEQ ID NO:20151 |
| | | AA | GCYWS | EINYSGRTNFNPSLKS | DYGGMDV |
| | | | SEQ ID NO:4128 | SEQ ID NO:12140 | SEQ ID NO:20152 |
| **iPS:468834** | **21-225_94G10** | NA | GGTTGCTACTGGAGC | GAAATCAATTATAGTGGAAGGACCAACTTCAACCCGTCCCTCAAGAGT | GACTACGGCGGTATGGACGTC |
| | | | SEQ ID NO:4129 | SEQ ID NO:12141 | SEQ ID NO:20153 |
| | | AA | GCYWS | EINYSGRTNFNPSLKS | DYGGMDV |
| | | | SEQ ID NO:4130 | SEQ ID NO:12142 | SEQ ID NO:20154 |
| **iPS:468836** | **21-225_198E3** | NA | AGCTATGGCATGCAC | GTTATATCATATGATGGAGGTTATAAAAACTATGCAGACTCCGTGAAGGGC | GGTACCCACGGGTACTACTACGGTGTGGACGTC |
| | | | SEQ ID NO:4131 | SEQ ID NO:12143 | SEQ ID NO:20155 |
| | | AA | SYGMH | VISYDGGYKNYADSVKG | GTHGYYYGVDV |
| | | | SEQ ID NO:4132 | SEQ ID NO:12144 | SEQ ID NO:20156 |
| **iPS:468838** | **21-225_80E12** | NA | GGTTGCTACTGGAGC | GAAATCAATTATAGTGGAAGGACCAACTTCAACCCGTCCCTCAAGAGT | GACTACGGCGGTATGGACGTC |
| | | | SEQ ID NO:4133 | SEQ ID NO:12145 | SEQ ID NO:20157 |
| | | AA | GCYWS | EINYSGRTNFNPSLKS | DYGGMDV |
| | | | SEQ ID NO:4134 | SEQ ID NO:12146 | SEQ ID NO:20158 |
| **iPS:468840** | **21-225_200H9** | NA | AGTGGTGGTGACTACTGGAGC | TACATCTATTACAGTGGGAGCACCTACTACAACCCGTCCCTCAAGAGT | ATGGACTACAGTAACTACTACTACGGTATGGACGTC |
| | | | SEQ ID NO:4135 | SEQ ID NO:12147 | SEQ ID NO:20159 |
| | | AA | SGGDYWS | YIYYSGSTYYNPSLKS | MDYSNYYYGMDV |
| | | | SEQ ID NO:4136 | SEQ ID NO:12148 | SEQ ID NO:20160 |

FIGURE 49

| | | | | | |
|---|---|---|---|---|---|
| **iPS:468820** | **21-225_76E10** | NA | GGTTCCTACTGGAGC | GAAATCAATTATAGTGGAAGGACCAACTTCAACCCGTCCCTCAAGAGT | GACTACGGCGGTATGGACGTC |
| | | | SEQ ID NO:4137 | SEQ ID NO:12149 | SEQ ID NO:20161 |
| | | AA | GSYWS | EINYSGRTNFNPSLKS | DYGGMDV |
| | | | SEQ ID NO:4138 | SEQ ID NO:12150 | SEQ ID NO:20162 |
| **iPS:468842** | **21-225_50H4** | NA | AGCTATGTCATGCAC | GCTATATGGTATGATGGAAGTAATAAATACTATGCAGACTCCGTGAAGGGC | GAGTTGTATAGCAGCAACTGGTACGACTACGGTATGGACGTC |
| | | | SEQ ID NO:4139 | SEQ ID NO:12151 | SEQ ID NO:20163 |
| | | AA | SYVMH | AIWYDGSNKYYADSVKG | ELYSSNWYDYGMDV |
| | | | SEQ ID NO:4140 | SEQ ID NO:12152 | SEQ ID NO:20164 |
| **iPS:468844** | **21-225_48E10** | NA | AGCTATAACATGAAC | TCCATTAGTGGTAGTAGTAGTTACATATACTACGCAGACTCAGTGAAGGGC | AGCCTCGACCTC |
| | | | SEQ ID NO:4141 | SEQ ID NO:12153 | SEQ ID NO:20165 |
| | | AA | SYNMN | SISGSSSYIYYADSVKG | SLDL |
| | | | SEQ ID NO:4142 | SEQ ID NO:12154 | SEQ ID NO:20166 |
| **iPS:468846** | **21-225_53B10** | NA | AGCTATAGCATGAAC | TCCATTAGTGGTAGTAGCAGTTACATATACTACGTAGACTCAGTGAAGGGC | GTCAACTCTTTTGACTCC |
| | | | SEQ ID NO:4143 | SEQ ID NO:12155 | SEQ ID NO:20167 |
| | | AA | SYSMN | SISGSSSYIYYVDSVKG | VNSFDS |
| | | | SEQ ID NO:4144 | SEQ ID NO:12156 | SEQ ID NO:20168 |
| **iPS:468848** | **21-225_54B1** | NA | AGCTATGCCATGAGC | GTTCTTAGTGGTAGTGGTGGTAGCACATTCTACGCAGACTCCGTGAAGGGC | AGAGGCCGTGAATATAGTGGCTACGATTACTTTGACTAC |
| | | | SEQ ID NO:4145 | SEQ ID NO:12157 | SEQ ID NO:20169 |

FIGURE 49

| | | | | | |
|---|---|---|---|---|---|
| | | AA | SYAMS | VLSGSGGSTFYADSVKG | RGREYSGYDYFDY |
| | | | SEQ ID NO:4146 | SEQ ID NO:12158 | SEQ ID NO:20170 |
| **iPS:468850** | **21-225_63F4** | NA | AATTATGATGTCAAC | TGGATGCACCCTAACAG TGGTAACACAGGCTATG CACAGAAGTTCCGGGGC | AGCAGTGGCTGGTACGTTTT TGACTAC |
| | | | SEQ ID NO:4147 | SEQ ID NO:12159 | SEQ ID NO:20171 |
| | | AA | NYDVN | WMHPNSGNTGYAQKFRG | SSGWYVFDY |
| | | | SEQ ID NO:4148 | SEQ ID NO:12160 | SEQ ID NO:20172 |
| **iPS:468852** | **21-225_71F3** | NA | AATTATGATGTCAAC | TGGATGCACCCTAACAG TGGTAACACAGGCTATG CACAGAAGTTCCAGGGC | AGCAGTGGCTGGTACGTTTT TGACTCC |
| | | | SEQ ID NO:4149 | SEQ ID NO:12161 | SEQ ID NO:20173 |
| | | AA | NYDVN | WMHPNSGNTGYAQKFQG | SSGWYVFDS |
| | | | SEQ ID NO:4150 | SEQ ID NO:12162 | SEQ ID NO:20174 |
| **iPS:468854** | **21-225_72C4** | NA | AATTATGATATCAAC | TGGATGCACCCTAACAG TGGTAACACAGGCTATG CACAGAAGTTCCAGGGC | AGCAGTGGCTGGTACCTCTT TGACTAC |
| | | | SEQ ID NO:4151 | SEQ ID NO:12163 | SEQ ID NO:20175 |
| | | AA | NYDIN | WMHPNSGNTGYAQKFQG | SSGWYLFDY |
| | | | SEQ ID NO:4152 | SEQ ID NO:12164 | SEQ ID NO:20176 |
| **iPS:468856** | **21-225_77C9** | NA | AGGAGTAGTTACTACTGGGG C | AGTATCTATTATAGTGGG AGCGCCTACTCCAACCC GTCCCTCAAGAGT | CTTGACTCTAACTGGGGTCT TGACTAC |
| | | | SEQ ID NO:4153 | SEQ ID NO:12165 | SEQ ID NO:20177 |
| | | AA | RSSYYWG | SIYYSGSAYSNPSLKS | LDSNWGLDY |
| | | | SEQ ID NO:4154 | SEQ ID NO:12166 | SEQ ID NO:20178 |

FIGURE 49

| | | | | | |
|---|---|---|---|---|---|
| **iPS:468858** | **21-225_148C9** | NA | GACTATGGCATGCAC | GTTATATGGTATGATGGAAGTAATAAGTACTATGCAGACTCCGTGAAGGGC | GAGAGGTATAGCAGTGGCTGGTACGACTACGGTTTGGACGTC |
| | | | SEQ ID NO:4155 | SEQ ID NO:12167 | SEQ ID NO:20179 |
| | | AA | DYGMH | VIWYDGSNKYYADSVKG | ERYSSGWYDYGLDV |
| | | | SEQ ID NO:4156 | SEQ ID NO:12168 | SEQ ID NO:20180 |
| **iPS:468860** | **21-225_224E7** | NA | AGCTATGTCATGCAC | GTTATATGGTATGATGGAAGTAATAAATACTATGCAGACTCCGTGAAGGGC | GAACAGTATAGCAGCAGCTGGTACGACTTCGGTCTGGACGTC |
| | | | SEQ ID NO:4157 | SEQ ID NO:12169 | SEQ ID NO:20181 |
| | | AA | SYVMH | VIWYDGSNKYYADSVKG | EQYSSSWYDFGLDV |
| | | | SEQ ID NO:4158 | SEQ ID NO:12170 | SEQ ID NO:20182 |
| **iPS:468862** | **21-225_178H8** | NA | GACTATTATATGCAC | TGGATCAACCCTAACAGAGGTGGCACAAACTATGCTCAGAAGTTTCAGGGC | GAGGAGGATCGCAGTGGCTGGTACTACTACTACGGTATGGACGTC |
| | | | SEQ ID NO:4159 | SEQ ID NO:12171 | SEQ ID NO:20183 |
| | | AA | DYYMH | WINPNRGGTNYAQKFQG | EEDRSGWYYYYGMDV |
| | | | SEQ ID NO:4160 | SEQ ID NO:12172 | SEQ ID NO:20184 |
| **iPS:468864** | **21-225_60D6** | NA | ACTAGTGGAGTGGGTGTGGGC | CTCATTTATTGGAAAGATGATGAGCGCTACAGCCCATCTCTGAAGAGC | GCAGTGGCTGTCTCCTTTGACTAC |
| | | | SEQ ID NO:4161 | SEQ ID NO:12173 | SEQ ID NO:20185 |
| | | AA | TSGVGVG | LIYWKDDERYSPSLKS | AVAVSFDY |
| | | | SEQ ID NO:4162 | SEQ ID NO:12174 | SEQ ID NO:20186 |
| **iPS:468866** | **21 225 190C1** | NA | GGCTACTATATGCAC | TGGATCAACCCTTACAGTGGTGGCACAAACTATGCACAGAAGTTTCAGGGC | GATAGAGCAGTGGCTGGAAACTACTTCTACTACGGTATGGACGTC |

**FIGURE 49**

| | | | | | |
|---|---|---|---|---|---|
| | **21-225_190C1** | | SEQ ID NO:4163 | SEQ ID NO:12175 | SEQ ID NO:20187 |
| | | AA | GYYMH | WINPYSGGTNYAQKFQG | DRAVAGNYFYYGMDV |
| | | | SEQ ID NO:4164 | SEQ ID NO:12176 | SEQ ID NO:20188 |
| **iPS:468868** | **21-225_74A1** | NA | GGTAGTAGTTACTACTGGGG C | AATATCTATTATAGTGGG AGCACCTACCACAACCC GTCCCTCAAGAGT | CATGATTTACTTTGGTCCCTT GACTTC |
| | | | SEQ ID NO:4165 | SEQ ID NO:12177 | SEQ ID NO:20189 |
| | | AA | GSSYYWG | NIYYSGSTYHNPSLKS | HDLLWSLDF |
| | | | SEQ ID NO:4166 | SEQ ID NO:12178 | SEQ ID NO:20190 |
| **iPS:468870** | **21-225_74A8** | NA | AATTATGATATCAAC | TGGATGCACCCTAACAG TGGTAACACAGGCTATG CACAGAAGTTCCAGGGC | AGTAGTGGCTGGTACAAATT TGACTAC |
| | | | SEQ ID NO:4167 | SEQ ID NO:12179 | SEQ ID NO:20191 |
| | | AA | NYDIN | WMHPNSGNTGYAQKFQG | SSGWYKFDY |
| | | | SEQ ID NO:4168 | SEQ ID NO:12180 | SEQ ID NO:20192 |
| **iPS:472730** | **21-225_14B1_LC1** | NA | AGCTATACCATGAAC | TCCATAAGTGGTAGTAG TAGTTACTTATACTACCC AGACTCAGTGAAGGGC | GATAGAGGCAGCAGC |
| | | | SEQ ID NO:4169 | SEQ ID NO:12181 | SEQ ID NO:20193 |
| | | AA | SYTMN | SISGSSSYLYYPDSVKG | DRGSS |
| | | | SEQ ID NO:4170 | SEQ ID NO:12182 | SEQ ID NO:20194 |
| **iPS:472731** | **21-225_14B1_LC2** | NA | AGCTATACCATGAAC | TCCATAAGTGGTAGTAG TAGTTACTTATACTACCC AGACTCAGTGAAGGGC | GATAGAGGCAGCAGC |
| | | | SEQ ID NO:4171 | SEQ ID NO:12183 | SEQ ID NO:20195 |
| | | AA | SYTMN | SISGSSSYLYYPDSVKG | DRGSS |
| | | | SEQ ID NO:4172 | SEQ ID NO:12184 | SEQ ID NO:20196 |

FIGURE 49

| | | | | | |
|---|---|---|---|---|---|
| **iPS:472732** | **21-225_2B10_LC1** | NA | AGCTATGTCATGCAC | GTTATATGGTATGATGGAAGTAATAAATACTATGCAGACTCCGTGAAGGTC | GAGAGATATACCAGTGGCTGGTATGACTACGGTATGGACGTC |
| | | | SEQ ID NO:4173 | SEQ ID NO:12185 | SEQ ID NO:20197 |
| | | AA | SYVMH | VIWYDGSNKYYADSVKV | ERYTSGWYDYGMDV |
| | | | SEQ ID NO:4174 | SEQ ID NO:12186 | SEQ ID NO:20198 |
| **iPS:472733** | **21-225_2B10_LC2** | NA | AGCTATGTCATGCAC | GTTATATGGTATGATGGAAGTAATAAATACTATGCAGACTCCGTGAAGGTC | GAGAGATATACCAGTGGCTGGTATGACTACGGTATGGACGTC |
| | | | SEQ ID NO:4175 | SEQ ID NO:12187 | SEQ ID NO:20199 |
| | | AA | SYVMH | VIWYDGSNKYYADSVKV | ERYTSGWYDYGMDV |
| | | | SEQ ID NO:4176 | SEQ ID NO:12188 | SEQ ID NO:20200 |
| **iPS:473253** | **21-225_7C3_LC1** | NA | GACTACTATTTGCAC | TGGATCCACCCTAACAGTGGTGGCACAAACTATGCACAGAAGTTTCAGGGC | GATGGTACCAGCTCGTTTGACTAC |
| | | | SEQ ID NO:4177 | SEQ ID NO:12189 | SEQ ID NO:20201 |
| | | AA | DYYLH | WIHPNSGGTNYAQKFQG | DGTSSFDY |
| | | | SEQ ID NO:4178 | SEQ ID NO:12190 | SEQ ID NO:20202 |
| **iPS:473254** | **21-225_7C3_LC2** | NA | GACTACTATTTGCAC | TGGATCCACCCTAACAGTGGTGGCACAAACTATGCACAGAAGTTTCAGGGC | GATGGTACCAGCTCGTTTGACTAC |
| | | | SEQ ID NO:4179 | SEQ ID NO:12191 | SEQ ID NO:20203 |
| | | AA | DYYLH | WIHPNSGGTNYAQKFQG | DGTSSFDY |
| | | | SEQ ID NO:4180 | SEQ ID NO:12192 | SEQ ID NO:20204 |

FIGURE 49

| | | | | | |
|---|---|---|---|---|---|
| **iPS:473255** | **21-225_9F12_LC1** | NA | GACTACTATTTGCAC | TGGATCCACCCTAACAGTGGTGGCACAAACTTTGCACAGAAGTTTCAGGGC | GATGGTACCAGCTCGTTTGACTAC |
| | | | SEQ ID NO:4181 | SEQ ID NO:12193 | SEQ ID NO:20205 |
| | | AA | DYYLH | WIHPNSGGTNFAQKFQG | DGTSSFDY |
| | | | SEQ ID NO:4182 | SEQ ID NO:12194 | SEQ ID NO:20206 |
| **iPS:473256** | **21-225_9F12_LC2** | NA | GACTACTATTTGCAC | TGGATCCACCCTAACAGTGGTGGCACAAACTTTGCACAGAAGTTTCAGGGC | GATGGTACCAGCTCGTTTGACTAC |
| | | | SEQ ID NO:4183 | SEQ ID NO:12195 | SEQ ID NO:20207 |
| | | AA | DYYLH | WIHPNSGGTNFAQKFQG | DGTSSFDY |
| | | | SEQ ID NO:4184 | SEQ ID NO:12196 | SEQ ID NO:20208 |
| **iPS:472742** | **21-225_30D9_LC2** | NA | GGCTACTATCTGCAC | TGGATCAACCCTAACAGTGGTGGCACAAACTATGCACAGAAGTTTCAGGGC | GTGTATTACTATGGTTCGGGGAGTTATTATAACGAGTTTGACAAC |
| | | | SEQ ID NO:4185 | SEQ ID NO:12197 | SEQ ID NO:20209 |
| | | AA | GYYLH | WINPNSGGTNYAQKFQG | VYYYGSGSYYNEFDN |
| | | | SEQ ID NO:4186 | SEQ ID NO:12198 | SEQ ID NO:20210 |
| **iPS:472741** | **21-225_30D9_LC1** | NA | GGCTACTATCTGCAC | TGGATCAACCCTAACAGTGGTGGCACAAACTATGCACAGAAGTTTCAGGGC | GTGTATTACTATGGTTCGGGGAGTTATTATAACGAGTTTGACAAC |
| | | | SEQ ID NO:4187 | SEQ ID NO:12199 | SEQ ID NO:20211 |
| | | AA | GYYLH | WINPNSGGTNYAQKFQG | VYYYGSGSYYNEFDN |
| | | | SEQ ID NO:4188 | SEQ ID NO:12200 | SEQ ID NO:20212 |
| **iPS:472743** | **21-225_68G6** | NA | GGYTACTATATGCAC | TCGATCTACCGTAACAGTGGTGGCACAAATTATGCACAGAAGTTTCAGGGC | GCCTTTTACTATGGTTCGGGGACTTATTATAACGAATTTGACTAC |
| | | | SEQ ID NO:4189 | SEQ ID NO:12201 | SEQ ID NO:20213 |
| | | AA | GYYMH | SIYRNSGGTNYAQKFQG | AFYYGSGTYYNEFDY |
| | | | SEQ ID NO:4190 | SEQ ID NO:12202 | SEQ ID NO:20214 |

**FIGURE 49**

| | | | | | |
|---|---|---|---|---|---|
| **iPS:392573** | **21-225_15G2** | NA | ACTAGTGGAGTGGGTGTGGGC | CTCATTTATTGGAATGATGATAAGCGCTACAGCCCATCTCTGAAGAGC | ACCGGTGTCAGCTGCTGCTATTTTCACTAT |
| | | | SEQ ID NO:4191 | SEQ ID NO:12203 | SEQ ID NO:20215 |
| | | AA | TSGVGVG | LIYWNDDKRYSPSLKS | TGVSCCYFHY |
| | | | SEQ ID NO:4192 | SEQ ID NO:12204 | SEQ ID NO:20216 |
| **iPS:392583** | **21-225_10B10** | NA | ACTGGTGGAGTGGGTGTGGGC | TTCATTTATTGGAGTGATGATAAGCGCTACAGCCCATCTCTGAAGAGC | ATAGCAGCAGTTGCCTTTGACTAC |
| | | | SEQ ID NO:4193 | SEQ ID NO:12205 | SEQ ID NO:20217 |
| | | AA | TGGVGVG | FIYWSDDKRYSPSLKS | IAAVAFDY |
| | | | SEQ ID NO:4194 | SEQ ID NO:12206 | SEQ ID NO:20218 |
| **iPS:392585** | **21-225_14H11** | NA | GGCCACTATATGTGC | TGGATCAACCCTAATAGTGGTGGCACAAACTATGCACAGAAGTTTCAGGGC | GGATATTGTAGTAGTTCCAGCTGCTATTTGCAACCGGGTTATTACGGTATGGACGTC |
| | | | SEQ ID NO:4195 | SEQ ID NO:12207 | SEQ ID NO:20219 |
| | | AA | GHYMC | WINPNSGGTNYAQKFQG | GYCSSSSCYLQPGYYGMDV |
| | | | SEQ ID NO:4196 | SEQ ID NO:12208 | SEQ ID NO:20220 |
| **iPS:392587** | **21-225_18G5** | NA | ACTAGTGGAGTGGGTGTGGGC | CTCATTTATTGGAATGATGATAAGGTCTACAGCCCATCTCTGAAGAGC | AGGGGACAGCAGCTGGCCCTCGACTAC |
| | | | SEQ ID NO:4197 | SEQ ID NO:12209 | SEQ ID NO:20221 |
| | | AA | TSGVGVG | LIYWNDDKVYSPSLKS | RGQQLALDY |
| | | | SEQ ID NO:4198 | SEQ ID NO:12210 | SEQ ID NO:20222 |
| **iPS:392589** | **21-225_27H2** | NA | GGCTATGGCATGCAC | GTTATATGGTATGATGGAAGTAATAAATACTATGCAGACTCCGTGAAGGGC | GATAGGGTATATTGTAGTAGTACCAGCTGCTCCCCTTACTACTACTACTACGGTATGGACGTC |
| | | | SEQ ID NO:4199 | SEQ ID NO:12211 | SEQ ID NO:20223 |

FIGURE 49

| | | | | | |
|---|---|---|---|---|---|
| | | AA | GYGMH | VIWYDGSNKYYADSVKG | DRVYCSSTSCSPYYYYYGMDV |
| | | | SEQ ID NO:4200 | SEQ ID NO:12212 | SEQ ID NO:20224 |
| **iPS:392593** | **21-225_3E10** | NA | ACTGGTGGAGTGGGTGTGGGC | CTCATTTATTGGAATGATGATAAGCGCCACAGCCCATCTCTGAAGAGC | CTTATAGAAGTGGCCTTTGACTAT |
| | | | SEQ ID NO:4201 | SEQ ID NO:12213 | SEQ ID NO:20225 |
| | | AA | TGGVGVG | LIYWNDDKRHSPSLKS | LIEVAFDY |
| | | | SEQ ID NO:4202 | SEQ ID NO:12214 | SEQ ID NO:20226 |
| **iPS:392596** | **21-225_12D8** | NA | AGCTATGTCATGAGC | ACTATTAGTGTTGGTGGTGGTAGCACATACTACGCAGACTCCGTGAAGGGC | TGGGGACGTGGATACAGCTATGAATACTACTACGGTATGGACGTC |
| | | | SEQ ID NO:4203 | SEQ ID NO:12215 | SEQ ID NO:20227 |
| | | AA | SYVMS | TISVGGGSTYYADSVKG | WGRGYSYEYYYGMDV |
| | | | SEQ ID NO:4204 | SEQ ID NO:12216 | SEQ ID NO:20228 |
| **iPS:392598** | **21-225_18E10** | NA | GGCTACTATATGCAC | TGGATCAACCCTAACAGTGGTGGCACAAACTATGCACAGAAGTTTCAGGGC | TCGTACTACTATGGTTCGGGGAGTTATTATAACGAGTTTGACTAC |
| | | | SEQ ID NO:4205 | SEQ ID NO:12217 | SEQ ID NO:20229 |
| | | AA | GYYMH | WINPNSGGTNYAQKFQG | SYYYGSGSYYNEFDY |
| | | | SEQ ID NO:4206 | SEQ ID NO:12218 | SEQ ID NO:20230 |
| **iPS:392618** | **21-225_16F10** | NA | GACTATGGCATGCAC | GTCATATGGTATGATGGAAATAATAAATACTATGTAGACTCCGTGAAGGGC | GAGCTTGCCTGGTACGAGGACTAC |
| | | | SEQ ID NO:4207 | SEQ ID NO:12219 | SEQ ID NO:20231 |
| | | AA | DYGMH | VIWYDGNNKYYVDSVKG | ELAWYEDY |
| | | | SEQ ID NO:4208 | SEQ ID NO:12220 | SEQ ID NO:20232 |

**FIGURE 49**

| | | | | | |
|---|---|---|---|---|---|
| **iPS:392620** | **21-225_17H5** | NA | AGCTATAGCATGAAC | TCCATTAGTAGTAGTAGTACTTACATATACTACGCAGACTCAGTGAAGGGC | GTGGCTTCATTTGACTAC |
| | | | SEQ ID NO:4209 | SEQ ID NO:12221 | SEQ ID NO:20233 |
| | | AA | SYSMN | SISSSSTYIYYADSVKG | VASFDY |
| | | | SEQ ID NO:4210 | SEQ ID NO:12222 | SEQ ID NO:20234 |
| **iPS:392622** | **21-225_17H8** | NA | AGAAGTAGTTACTACTGGGGC | AATATCTATTATGGTGGGAACACCTACTACAACCCGTCCCTCAAGAGT | CATGGAAAAGACTGGGGCCTTGACTAC |
| | | | SEQ ID NO:4211 | SEQ ID NO:12223 | SEQ ID NO:20235 |
| | | AA | RSSYYWG | NIYYGGNTYYNPSLKS | HGKDWGLDY |
| | | | SEQ ID NO:4212 | SEQ ID NO:12224 | SEQ ID NO:20236 |
| **iPS:392624** | **21-225_17H12** | NA | AGCTATACCATGAAC | TCCATTAGTGGTAGTAGTAGTTACATATACTACGCAGACTCAGTGAAGGGC | GATCGAGGCTCCATC |
| | | | SEQ ID NO:4213 | SEQ ID NO:12225 | SEQ ID NO:20237 |
| | | AA | SYTMN | SISGSSSYIYYADSVKG | DRGSI |
| | | | SEQ ID NO:4214 | SEQ ID NO:12226 | SEQ ID NO:20238 |
| **iPS:392626** | **21-225_18A5** | NA | GACTATGGCATGCAC | GTTATATGGTTTGATGGAAGTAATAAATACTATGCAGACTCCGTGAAGGGC | GATCTTGGCTGGACGGAAGAGTAC |
| | | | SEQ ID NO:4215 | SEQ ID NO:12227 | SEQ ID NO:20239 |
| | | AA | DYGMH | VIWFDGSNKYYADSVKG | DLGWTEEY |
| | | | SEQ ID NO:4216 | SEQ ID NO:12228 | SEQ ID NO:20240 |
| **iPS:392628** | **21-225_20C2** | NA | AGAAGTAGTTACTACTGGGGC | AATATCTATTATAGTGGGACTGCCTACTGTAATTCGTCCCTCAAGAGT | CATAGTAGCAGCTGGTCCCTTGACAAC |
| | | | SEQ ID NO:4217 | SEQ ID NO:12229 | SEQ ID NO:20241 |
| | | AA | RSSYYWG | NIYYSGTAYCNSSLKS | HSSSWSLDN |

FIGURE 49

| | | | | | |
|---|---|---|---|---|---|
| | | | SEQ ID NO:4218 | SEQ ID NO:12230 | SEQ ID NO:20242 |
| **iPS:392630** | **21-225_20E5** | NA | GACTATGGCATGCAC | GTTATTTGGTATGAAGAAAATAATCAATACTATGCAGACTCCGTGAAGGGC | GAACTGGGGTTCCGGTCTGACTAC |
| | | | SEQ ID NO:4219 | SEQ ID NO:12231 | SEQ ID NO:20243 |
| | | AA | DYGMH | VIWYEENNQYYADSVKG | ELGFRSDY |
| | | | SEQ ID NO:4220 | SEQ ID NO:12232 | SEQ ID NO:20244 |
| **iPS:392632** | **21-225_16A11** | NA | AGCTATAGCATGAAC | TCCATTAGTGGTAGTAGTAGTCTCATATACTACGCAGACTCAGTGAAGGGC | GTAGCAGCCTTTGACTAC |
| | | | SEQ ID NO:4221 | SEQ ID NO:12233 | SEQ ID NO:20245 |
| | | AA | SYSMN | SISGSSSLIYYADSVKG | VAAFDY |
| | | | SEQ ID NO:4222 | SEQ ID NO:12234 | SEQ ID NO:20246 |
| **iPS:392634** | **21-225_17H3** | NA | AACTGTGTCATGCAC | GTTATATGGTATGATGGAAGTAATAAATACTATGCAGACTCCGTGAAGGGC | GAAAAGTATAGCAGCAGCTGGTACGACTACGGTATGGACGTC |
| | | | SEQ ID NO:4223 | SEQ ID NO:12235 | SEQ ID NO:20247 |
| | | AA | NCVMH | VIWYDGSNKYYADSVKG | EKYSSSWYDYGMDV |
| | | | SEQ ID NO:4224 | SEQ ID NO:12236 | SEQ ID NO:20248 |
| **iPS:392636** | **21-225_17A6** | NA | CGCAACACTGCTGCTTGGAGC | AGGACATACTACAGGTCCAAGTGGTATAATGATTATGCAGTATCTGTGAAAAGT | GTAAGCAGTGGCTGGTCCCATCACTACTACTACTACGGTATGGACGTC |
| | | | SEQ ID NO:4225 | SEQ ID NO:12237 | SEQ ID NO:20249 |
| | | AA | RNTAAWS | RTYYRSKWYNDYAVSVKS | VSSGWSHHYYYYGMDV |

**FIGURE 49**

| | | | | | |
|---|---|---|---|---|---|
| | | | SEQ ID NO:4226 | SEQ ID NO:12238 | SEQ ID NO:20250 |
| **iPS:392638** | **21-225_17F9** | NA | AGAAGTAGTTACTATTGGGGC | AATATCTATTATAGTGGGAGCACCTACTACAATCCGTCCCTCAAGAGT | CATGGAAAAGACTGGGGCCTTGACTAC |
| | | | SEQ ID NO:4227 | SEQ ID NO:12239 | SEQ ID NO:20251 |
| | | AA | RSSYYWG | NIYYSGSTYYNPSLKS | HGKDWGLDY |
| | | | SEQ ID NO:4228 | SEQ ID NO:12240 | SEQ ID NO:20252 |
| **iPS:392640** | **21-225_18A1** | NA | AGCTATGGCATGCAT | GTTATATGGTATGAGGAAAATAATAAATATTATGTAGACTCCGTGAAGGGC | GAGCTAGGCTTCCAGTCTGACTAC |
| | | | SEQ ID NO:4229 | SEQ ID NO:12241 | SEQ ID NO:20253 |
| | | AA | SYGMH | VIWYEENNKYYVDSVKG | ELGFQSDY |
| | | | SEQ ID NO:4230 | SEQ ID NO:12242 | SEQ ID NO:20254 |
| **iPS:392642** | **21-225_18C6** | NA | AGGAGTAGTTATTACTGGGGC | AATATCTATTATAGTGGGTACACCTACTACAACCCGTCCCTCAAGAGT | CATAGTAGCAGTTGGTCCCTTGACGAC |
| | | | SEQ ID NO:4231 | SEQ ID NO:12243 | SEQ ID NO:20255 |
| | | AA | RSSYYWG | NIYYSGYTYYNPSLKS | HSSSWSLDD |
| | | | SEQ ID NO:4232 | SEQ ID NO:12244 | SEQ ID NO:20256 |
| **iPS:392644** | **21-225_19E1** | NA | AACTATGGCATGCAC | GTTATTTGGTATGAAGAAAATAATCAATACTATGCAGACTCCGTGAAGGGC | GAACTGGGGTTCCGGTCTGACTAC |
| | | | SEQ ID NO:4233 | SEQ ID NO:12245 | SEQ ID NO:20257 |
| | | AA | NYGMH | VIWYEENNQYYADSVKG | ELGFRSDY |
| | | | SEQ ID NO:4234 | SEQ ID NO:12246 | SEQ ID NO:20258 |

FIGURE 49

| | | | | | |
|---|---|---|---|---|---|
| **iPS:392646** | **21-225_20G2** | NA | AGCGATGACATGCAC | GTTATATGGTTTGATGGAAGTAATAAATACTATGCAGACTCCGTGAAGGGC | GATCTGATAGCAGCAGCTGGTACGGTTGACTAC |
| | | | SEQ ID NO:4235 | SEQ ID NO:12247 | SEQ ID NO:20259 |
| | | AA | SDDMH | VIWFDGSNKYYADSVKG | DLIAAAGTVDY |
| | | | SEQ ID NO:4236 | SEQ ID NO:12248 | SEQ ID NO:20260 |
| **iPS:392648** | **21-225_16D11** | NA | CGCAACACTGCTGCTTGGAGC | AGGACATACTACAGGTCCAAGTGGTATAATGATTATGCAGTATCTGTGAAAAGT | GTAAACAGTGGCTGGTCCCATCACTACTACTACTACGGTATGGACGTC |
| | | | SEQ ID NO:4237 | SEQ ID NO:12249 | SEQ ID NO:20261 |
| | | AA | RNTAAWS | RTYYRSKWYNDYAVSVKS | VNSGWSHHYYYYGMDV |
| | | | SEQ ID NO:4238 | SEQ ID NO:12250 | SEQ ID NO:20262 |
| **iPS:392650** | **21-225_17A4** | NA | GACTACTACATGAGC | CACATTAGTAGTAGTGGTAGTACCATATATTACGCAGACTCTGTGAAGGGC | TATCGGAATAACCGGGGATACTTCGATCTC |
| | | | SEQ ID NO:4239 | SEQ ID NO:12251 | SEQ ID NO:20263 |
| | | AA | DYYMS | HISSSGSTIYYADSVKG | YRNNRGYFDL |
| | | | SEQ ID NO:4240 | SEQ ID NO:12252 | SEQ ID NO:20264 |
| **iPS:392652** | **21-225_17C6** | NA | AGCTATGCCATGAAC | GTTATTAGTGGTCGTGGTGGTAACACATTCTACGCAGACTCCGTGAAGGGC | CGTTTGGCAGTGGCTGGCTCGGAGGCTTTTGATATC |
| | | | SEQ ID NO:4241 | SEQ ID NO:12253 | SEQ ID NO:20265 |
| | | AA | SYAMN | VISGRGGNTFYADSVKG | RLAVAGSEAFDI |
| | | | SEQ ID NO:4242 | SEQ ID NO:12254 | SEQ ID NO:20266 |

## FIGURE 49

| | | | | | |
|---|---|---|---|---|---|
| **iPS:392654** | **21-225_17A10** | NA | AACTATGGCATGCAC | GTTATTTGGTATGAAGAAAATAATCAATACTATGCAGACTCCGTGAAGGGC | GAACTGGGGTTCCGGTCTGACTAC |
| | | | SEQ ID NO:4243 | SEQ ID NO:12255 | SEQ ID NO:20267 |
| | | AA | NYGMH | VIWYEENNQYYADSVKG | ELGFRSDY |
| | | | SEQ ID NO:4244 | SEQ ID NO:12256 | SEQ ID NO:20268 |
| **iPS:392656** | **21-225_1F2** | NA | AGGAGTAGTTACTACTGGGGC | AATATTTATTATAGTGGGAGCGCCTACAACAACCCGTCCCTCAAGGGT | CATGGAAAAGACTGGGGCCTTGACTAC |
| | | | SEQ ID NO:4245 | SEQ ID NO:12257 | SEQ ID NO:20269 |
| | | AA | RSSYYWG | NIYYSGSAYNNPSLKG | HGKDWGLDY |
| | | | SEQ ID NO:4246 | SEQ ID NO:12258 | SEQ ID NO:20270 |
| **iPS:392658** | **21-225_18E8** | NA | AACTATGGCATGCAC | GTTATTTGGTATGAAGAAAATAATCAATACTATGCAGACTCCGTGAAGGGC | GAACTGGGGTTCCGGTCTGACTAC |
| | | | SEQ ID NO:4247 | SEQ ID NO:12259 | SEQ ID NO:20271 |
| | | AA | NYGMH | VIWYEENNQYYADSVKG | ELGFRSDY |
| | | | SEQ ID NO:4248 | SEQ ID NO:12260 | SEQ ID NO:20272 |
| **iPS:392660** | **21-225_19B3** | NA | AGTTATGACATGCAC | GTTATATGGTATGACGGAAGTGATAAATACTATGCAGACTCCGTGAAGGGC | GATCGGGCCTATAGCAGCTCGTCTGACTAC |
| | | | SEQ ID NO:4249 | SEQ ID NO:12261 | SEQ ID NO:20273 |
| | | AA | SYDMH | VIWYDGSDKYYADSVKG | DRAYSSSSDY |
| | | | SEQ ID NO:4250 | SEQ ID NO:12262 | SEQ ID NO:20274 |

FIGURE 49

| | | | | | |
|---|---|---|---|---|---|
| **iPS:392664** | **21-225_20F6** | NA | AGCTATGGCATGCAC | GTTATATGGCATGATGGAAGTAATAAATACTATGCAGACTCCGTGAAGGGC | GATCTGAGTATGGGCGGAATGGACGTC |
| | | | SEQ ID NO:4251 | SEQ ID NO:12263 | SEQ ID NO:20275 |
| | | AA | SYGMH | VIWHDGSNKYYADSVKG | DLSMGGMDV |
| | | | SEQ ID NO:4252 | SEQ ID NO:12264 | SEQ ID NO:20276 |
| **iPS:392666** | **21-225_16F11** | NA | AGCTATGGCATGCAC | GTTATATGGTATGAGGAAAATAATAAATACTATGTAGACTCCGTGAAGGGC | GAACTAGGCTTCCAGTCTGACTAC |
| | | | SEQ ID NO:4253 | SEQ ID NO:12265 | SEQ ID NO:20277 |
| | | AA | SYGMH | VIWYEENNKYYVDSVKG | ELGFQSDY |
| | | | SEQ ID NO:4254 | SEQ ID NO:12266 | SEQ ID NO:20278 |
| **iPS:392668** | **21-225_17B4** | NA | AGCTATGCCATGAAC | GTTATTAGTGGCCGTGGTGGTAACACATTCTACGCAGACTCCGTGAAGGGC | CGTTTGGCAGTGGCTGGCTCGGAGGCTTTTGATATC |
| | | | SEQ ID NO:4255 | SEQ ID NO:12267 | SEQ ID NO:20279 |
| | | AA | SYAMN | VISGRGGNTFYADSVKG | RLAVAGSEAFDI |
| | | | SEQ ID NO:4256 | SEQ ID NO:12268 | SEQ ID NO:20280 |
| **iPS:392674** | **21-225_18C2** | NA | GACTATGGCATGCAC | GTTATATGGTATGATGTAACTAATAAATACTATGCAGACTCCGTGAAGGGC | GAGCTTGGCTGGTACGAGGACTAC |
| | | | SEQ ID NO:4257 | SEQ ID NO:12269 | SEQ ID NO:20281 |
| | | AA | DYGMH | VIWYDVTNKYYADSVKG | ELGWYEDY |
| | | | SEQ ID NO:4258 | SEQ ID NO:12270 | SEQ ID NO:20282 |

## FIGURE 49

| | | | | | |
|---|---|---|---|---|---|
| **iPS:392676** | **21-225_19F3** | NA | GGTAGTAGTTACTACTGGGGC | AATATCTATTATAGTGGGAGCACCTACTACAACCCGTCCTTCAAGAGT | CATTCCAGTAGCTGGTCCCTTGACTAC |
| | | | SEQ ID NO:4259 | SEQ ID NO:12271 | SEQ ID NO:20283 |
| | | AA | GSSYYWG | NIYYSGSTYYNPSFKS | HSSSWSLDY |
| | | | SEQ ID NO:4260 | SEQ ID NO:12272 | SEQ ID NO:20284 |
| **iPS:392678** | **21-225_20F3** | NA | AGCTATGCCATGAAC | GTTATTAGTGGTAGTGGTGGTAGCACATACTACGCAGACTCCGTGAAGGGC | AGGATAGCAGCAGCTGGTACGGAGTACTTCGATCTC |
| | | | SEQ ID NO:4261 | SEQ ID NO:12273 | SEQ ID NO:20285 |
| | | AA | SYAMN | VISGSGGSTYYADSVKG | RIAAAGTEYFDL |
| | | | SEQ ID NO:4262 | SEQ ID NO:12274 | SEQ ID NO:20286 |
| **iPS:392680** | **21-225_20A7** | NA | AACTATGGCATGCAC | GTTATTTGGTATGAAGAAAATAATCAATACTATGCAGACTCCGTGAAGGGC | GAACTGGGGTTCCGGTCTGACTAC |
| | | | SEQ ID NO:4263 | SEQ ID NO:12275 | SEQ ID NO:20287 |
| | | AA | NYGMH | VIWYEENNQYYADSVKG | ELGFRSDY |
| | | | SEQ ID NO:4264 | SEQ ID NO:12276 | SEQ ID NO:20288 |
| **iPS:392682** | **21-225_16A12** | NA | AGCTATAGAATGAAC | TCCATTAGTGGTAGTAGTACTGACATATACTACGCAGACTCAGTGAAGGGC | AGGGACTTC |
| | | | SEQ ID NO:4265 | SEQ ID NO:12277 | SEQ ID NO:20289 |
| | | AA | SYRMN | SISGSSTDIYYADSVKG | RDF |
| | | | SEQ ID NO:4266 | SEQ ID NO:12278 | SEQ ID NO:20290 |
| **iPS:392684** | **21 225 17F4** | NA | AACTATGGCATGAAC | GTTATATGGTATGATGGAAATAATAAACACTATGCAGACTCCGTGAAGGGC | AGTGGGAGCTACTTCTTTGACTAC |

FIGURE 49

| | | | | | |
|---|---|---|---|---|---|
| | **21-225_17F4** | | SEQ ID NO:4267 | SEQ ID NO:12279 | SEQ ID NO:20291 |
| | | AA | NYGMN | VIWYDGNNKHYADSVKG | SGSYFFDY |
| | | | SEQ ID NO:4268 | SEQ ID NO:12280 | SEQ ID NO:20292 |
| **iPS:392686** | **21-225_17C7** | NA | GACTATGGCATGCAC | GTTATATGGTTTGATGGAAGTAATAAATACTATGCAGACTCCGTGAAGGGC | GATCTTGGCTGGACGGAAGAATAC |
| | | | SEQ ID NO:4269 | SEQ ID NO:12281 | SEQ ID NO:20293 |
| | | AA | DYGMH | VIWFDGSNKYYADSVKG | DLGWTEEY |
| | | | SEQ ID NO:4270 | SEQ ID NO:12282 | SEQ ID NO:20294 |
| **iPS:392690** | **21-225_18F2** | NA | GACTATGGCATGCAC | GTTATATGGTTTGATGGAAGTAATAAATACTATGTAGACTCCGTGAAGGGC | GATCTTGGCTGGACGGAAGAGTAC |
| | | | SEQ ID NO:4271 | SEQ ID NO:12283 | SEQ ID NO:20295 |
| | | AA | DYGMH | VIWFDGSNKYYVDSVKG | DLGWTEEY |
| | | | SEQ ID NO:4272 | SEQ ID NO:12284 | SEQ ID NO:20296 |
| **iPS:392692** | **21-225_18G10** | NA | ACCTATAGCATGAAC | TACATTAGTAGGAGTAGTAGTACCATAGACTACGCAGACTCTGTGAAGGGC | GGAGGTGGGAGCCCTTTTGACTAC |
| | | | SEQ ID NO:4273 | SEQ ID NO:12285 | SEQ ID NO:20297 |
| | | AA | TYSMN | YISRSSSTIDYADSVKG | GGGSPFDY |
| | | | SEQ ID NO:4274 | SEQ ID NO:12286 | SEQ ID NO:20298 |
| **iPS:392694** | **21-225_19A5** | NA | AGCTATGCCATGCAC | GTTATATGGTTTGATGGAAGTGATAAATACTATGCAGACTCCGTGAAGGGC | GATCGGGCCTATAGTAGCTCGTCTGACTAC |
| | | | SEQ ID NO:4275 | SEQ ID NO:12287 | SEQ ID NO:20299 |
| | | AA | SYAMH | VIWFDGSDKYYADSVKG | DRAYSSSSDY |
| | | | SEQ ID NO:4276 | SEQ ID NO:12288 | SEQ ID NO:20300 |

FIGURE 49

| | | | | | |
|---|---|---|---|---|---|
| **iPS:392696** | **21-225_20A4** | NA | AGCTATGCCATGACC | GTTATAAGTGGTAGTGGTGGTTACACATACAACGCGGACTCCGTGAAGGGC | CGTATAGCAGTGGCTGGCTCGGAGGCTTTTGATATC |
| | | | SEQ ID NO:4277 | SEQ ID NO:12289 | SEQ ID NO:20301 |
| | | AA | SYAMT | VISGSGGYTYNADSVKG | RIAVAGSEAFDI |
| | | | SEQ ID NO:4278 | SEQ ID NO:12290 | SEQ ID NO:20302 |
| **iPS:392700** | **21-225_16E12** | NA | AACTATGGCATGCAC | GTTATATGGTATGAGGGAAGTAATAAATATTATGTAGACTCCGTGAGGGGC | GAGCTAGGCTTCCAGTCTGATCAC |
| | | | SEQ ID NO:4279 | SEQ ID NO:12291 | SEQ ID NO:20303 |
| | | AA | NYGMH | VIWYEGSNKYYVDSVRG | ELGFQSDH |
| | | | SEQ ID NO:4280 | SEQ ID NO:12292 | SEQ ID NO:20304 |
| **iPS:392702** | **21-225_17F7** | NA | AGCTATGCCATGAAC | ATTATTAGTGGTCGTGGTGGTAACACATTCTACGCAGACTCCGTGAAGGGC | CGTTTGGCAGTGGCTGGCTCGGAGGCTTTTGATATC |
| | | | SEQ ID NO:4281 | SEQ ID NO:12293 | SEQ ID NO:20305 |
| | | AA | SYAMN | IISGRGGNTFYADSVKG | RLAVAGSEAFDI |
| | | | SEQ ID NO:4282 | SEQ ID NO:12294 | SEQ ID NO:20306 |
| **iPS:392704** | **21-225_17F11** | NA | AGCTATGCCATGAGC | GTTATTAGTGGTAGAGGTGGTAACACATACTCCGCAGACTCCGTGAAGGGC | CGTTTAGCAGTGGCTGGCTCGGAGGCTTTTCATATC |
| | | | SEQ ID NO:4283 | SEQ ID NO:12295 | SEQ ID NO:20307 |
| | | AA | SYAMS | VISGRGGNTYSADSVKG | RLAVAGSEAFHI |
| | | | SEQ ID NO:4284 | SEQ ID NO:12296 | SEQ ID NO:20308 |
| **iPS:392706** | **21-225_18A3** | NA | AGAAGTAGTTATTACTGGGGC | AATATCTATTATAGTGGGTATACCTACTACACTCCGTCCCTCAAGAGT | CATAGCACCAGCTGGTCCCTTGACTAC |
| | | | SEQ ID NO:4285 | SEQ ID NO:12297 | SEQ ID NO:20309 |
| | | AA | RSSYYWG | NIYYSGYTYYTPSLKS | HSTSWSLDY |
| | | | SEQ ID NO:4286 | SEQ ID NO:12298 | SEQ ID NO:20310 |

FIGURE 49

| | | | | | |
|---|---|---|---|---|---|
| **iPS:392708** | **21-225_18D11** | NA | AGCTATAGCATGAAC | TACATTAGTAGTAGTAGTGGTACCATATACTACGCAGACTCTGTGAAGGGC | GGAGGTGGGAGCCCTTTTGACTAC |
| | | | SEQ ID NO:4287 | SEQ ID NO:12299 | SEQ ID NO:20311 |
| | | AA | SYSMN | YISSSSGTIYYADSVKG | GGGSPFDY |
| | | | SEQ ID NO:4288 | SEQ ID NO:12300 | SEQ ID NO:20312 |
| **iPS:392710** | **21-225_19A10** | NA | AGCTATGGCATGCAC | GTTATATGGTATGATGAAAGTAATAAATACTATGCAGACTCCGTGAAGGGC | GAGCTTGCCTGGTACGAGGACTCC |
| | | | SEQ ID NO:4289 | SEQ ID NO:12301 | SEQ ID NO:20313 |
| | | AA | SYGMH | VIWYDESNKYYADSVKG | ELAWYEDS |
| | | | SEQ ID NO:4290 | SEQ ID NO:12302 | SEQ ID NO:20314 |
| **iPS:392714** | **21-225_16G12** | NA | AGCTATGCCATGACC | ACTATTAGTGGTCGTGGTGGTCACACATACTACGCAGACTCCGTGAGGGGC | CAGGACTGC |
| | | | SEQ ID NO:4291 | SEQ ID NO:12303 | SEQ ID NO:20315 |
| | | AA | SYAMT | TISGRGGHTYYADSVRG | QDC |
| | | | SEQ ID NO:4292 | SEQ ID NO:12304 | SEQ ID NO:20316 |
| **iPS:392716** | **21-225_17B5** | NA | GACTATGGCATGCAC | GTTATATGGTATGATGAAAGTAATAAACACTATATAGACTCCGTGAAGGGC | GAACTGGGGTTCCGGTTTGACTAC |
| | | | SEQ ID NO:4293 | SEQ ID NO:12305 | SEQ ID NO:20317 |
| | | AA | DYGMH | VIWYDESNKHYIDSVKG | ELGFRFDY |
| | | | SEQ ID NO:4294 | SEQ ID NO:12306 | SEQ ID NO:20318 |
| **iPS:392718** | | NA | AGCTATGCTATCAAC | TGGATGAACCCTAACACTGGTAACACAGGCTATGCACAGAAGTTCCAGGGC | AAGGCTGGGTTTGACTAC |

FIGURE 49

| | | | | | |
|---|---|---|---|---|---|
| | **21-225_17B8** | | SEQ ID NO:4295 | SEQ ID NO:12307 | SEQ ID NO:20319 |
| | | AA | SYAIN | WMNPNTGNTGYAQKFQG | KAGFDY |
| | | | SEQ ID NO:4296 | SEQ ID NO:12308 | SEQ ID NO:20320 |
| **iPS:392720** | **21-225_17A12** | NA | AGCTATGCCATGAGC | ATTATTAGTGGTCGTGGG GGAAACGCATTCTACGC AGACTCCGTGAAGGGC | CGTATAGCAGTGGCTGGCTC GGAGGCTTTTGATATC |
| | | | SEQ ID NO:4297 | SEQ ID NO:12309 | SEQ ID NO:20321 |
| | | AA | SYAMS | IISGRGGNAFYADSVKG | RIAVAGSEAFDI |
| | | | SEQ ID NO:4298 | SEQ ID NO:12310 | SEQ ID NO:20322 |
| **iPS:392722** | **21-225_18E12** | NA | AGCTATGCCATGAGC | ATTATTAGTGGTCGTGGT GGTAACACATTCTACGC AGACTCCGTGAAGGGC | CGTCTGGCAGTGGCTGGCTC GGAGGCTTTTGATATC |
| | | | SEQ ID NO:4299 | SEQ ID NO:12311 | SEQ ID NO:20323 |
| | | AA | SYAMS | IISGRGGNTFYADSVKG | RLAVAGSEAFDI |
| | | | SEQ ID NO:4300 | SEQ ID NO:12312 | SEQ ID NO:20324 |
| **iPS:392726** | **21-225_20B5** | NA | AGCTATAGCATGAAC | TCCATTAGTGGGAGTAG TAGTTACATATACTACGC AGACTCAGTGAAGGGC | GATCGTGGGAGCTAC |
| | | | SEQ ID NO:4301 | SEQ ID NO:12313 | SEQ ID NO:20325 |
| | | AA | SYSMN | SISGSSSYIYYADSVKG | DRGSY |
| | | | SEQ ID NO:4302 | SEQ ID NO:12314 | SEQ ID NO:20326 |
| **iPS:392728** | **21-225_20F7** | NA | GACTATTACATGAGC | CACATTAGTAGTAGTGG TAGTACCATATACTACGC AGACTCTGTGAAGGGC | TATCGGAATAACCGGGGTA CTTCGATCTC |
| | | | SEQ ID NO:4303 | SEQ ID NO:12315 | SEQ ID NO:20327 |
| | | AA | DYYMS | HISSSGSTIYYADSVKG | YRNNRGYFDL |
| | | | SEQ ID NO:4304 | SEQ ID NO:12316 | SEQ ID NO:20328 |

FIGURE 49

| | | | | | |
|---|---|---|---|---|---|
| **iPS:392730** | **21-225_17A1** | NA | AGCTATGCCATGAAC | GTTATTAGTGGTAGTGGTAGTAACACATACTACGCAGACTCCGTGAAGGGC | AGATATACCAGTGACTGGCATGATGCTTTTGATATC |
| | | | SEQ ID NO:4305 | SEQ ID NO:12317 | SEQ ID NO:20329 |
| | | AA | SYAMN | VISGSGSNTYYADSVKG | RYTSDWHDAFDI |
| | | | SEQ ID NO:4306 | SEQ ID NO:12318 | SEQ ID NO:20330 |
| **iPS:392732** | **21-225_17E5** | NA | GACTATGGCATGCAC | CTTATATGGTATGATGTAACTAATAAATACTATGGAGACTCCGTGAAGGGC | GAGCTTGGCTGGTACGAGGACTAC |
| | | | SEQ ID NO:4307 | SEQ ID NO:12319 | SEQ ID NO:20331 |
| | | AA | DYGMH | LIWYDVTNKYYGDSVKG | ELGWYEDY |
| | | | SEQ ID NO:4308 | SEQ ID NO:12320 | SEQ ID NO:20332 |
| **iPS:392734** | **21-225_17D8** | NA | AGCTATGGCTTGAAC | TCCATTAGTGGTAGTGGTAGTCACATATCCTACGCGGACTCAGTGAAGGGC | GATCGGGGCAGTGGC |
| | | | SEQ ID NO:4309 | SEQ ID NO:12321 | SEQ ID NO:20333 |
| | | AA | SYGLN | SISGSGSHISYADSVKG | DRGSG |
| | | | SEQ ID NO:4310 | SEQ ID NO:12322 | SEQ ID NO:20334 |
| **iPS:392736** | **21-225_17B12** | NA | AGCTATGCCATGAAC | GTTATTAGTGGTAGTGGTGGTAGCACATACTACGCAGACTCCGTGAAGGGC | AGGTATACCAGTGACTGGCATGATGCTTTTGATATC |
| | | | SEQ ID NO:4311 | SEQ ID NO:12323 | SEQ ID NO:20335 |
| | | AA | SYAMN | VISGSGGSTYYADSVKG | RYTSDWHDAFDI |
| | | | SEQ ID NO:4312 | SEQ ID NO:12324 | SEQ ID NO:20336 |
| **iPS:392738** | **21-225_18G4** | NA | AGCTATGGCATGCAC | ATTATATGGTATGATGGAAGTAATAAATACTATGCAGACTCCGTGAAGGGC | GATCTGAGTATGGGCGGTATGGACGTC |

FIGURE 49

| | | | | | |
|---|---|---|---|---|---|
| | | | SEQ ID NO:4313 | SEQ ID NO:12325 | SEQ ID NO:20337 |
| | | AA | SYGMH | IIWYDGSNKYYADSVKG | DLSMGGMDV |
| | | | SEQ ID NO:4314 | SEQ ID NO:12326 | SEQ ID NO:20338 |
| **iPS:392740** | **21-225_18H12** | NA | GACTATGGCATGCAC | GTTATATGGTATGATGTAACTAATAAATACTATGCAGACTCCGTGAAGGGC | GAGGTTGGCTGGTACGAGGACTAC |
| | | | SEQ ID NO:4315 | SEQ ID NO:12327 | SEQ ID NO:20339 |
| | | AA | DYGMH | VIWYDVTNKYYADSVKG | EVGWYEDY |
| | | | SEQ ID NO:4316 | SEQ ID NO:12328 | SEQ ID NO:20340 |
| **iPS:392742** | **21-225_20B2** | NA | AATTATGTCATTCAC | GTTATATGGTATGATGGAAGTAATAAATACTATGCAGACTCCGTGAAGGGC | GAGAAGTATAGCAGCAGCTGGTACGACTACGGTATGGACGTC |
| | | | SEQ ID NO:4317 | SEQ ID NO:12329 | SEQ ID NO:20341 |
| | | AA | NYVIH | VIWYDGSNKYYADSVKG | EKYSSSWYDYGMDV |
| | | | SEQ ID NO:4318 | SEQ ID NO:12330 | SEQ ID NO:20342 |
| **iPS:392744** | **21-225_20D5** | NA | AGCTATGGCATGCAC | GTTATTTGGTATGAAGAAAATAATCAATACTATGCAGACTCCGTGAAGGGC | GAACTGGGGTTCCGGTCTGACTAC |
| | | | SEQ ID NO:4319 | SEQ ID NO:12331 | SEQ ID NO:20343 |
| | | AA | SYGMH | VIWYEENNQYYADSVKG | ELGFRSDY |
| | | | SEQ ID NO:4320 | SEQ ID NO:12332 | SEQ ID NO:20344 |
| **iPS:392746** | **21 225 20H7** | NA | AGCTATAGCATGAAC | TCCATTAGTGGTAGTAGTAGTTTCATATACTACGCAGACTCAGTGAAGGGC | GTAGCAGCTCTTGACTAC |

## FIGURE 49

| | | | | | |
|---|---|---|---|---|---|
| | 21-225_20H7 | | SEQ ID NO:4321 | SEQ ID NO:12333 | SEQ ID NO:20345 |
| | | AA | SYSMN | SISGSSSFIYYADSVKG | VAALDY |
| | | | SEQ ID NO:4322 | SEQ ID NO:12334 | SEQ ID NO:20346 |
| iPS:392748 | 21-225_20A8 | NA | AGCTATAGCGTGAAC | TCCATTAGTAGTAGTAGTAGTTTCCTATACTACGCAGACTCAGTGAAGGGC | AACTGGGACTAC |
| | | | SEQ ID NO:4323 | SEQ ID NO:12335 | SEQ ID NO:20347 |
| | | AA | SYSVN | SISSSSSFLYYADSVKG | NWDY |
| | | | SEQ ID NO:4324 | SEQ ID NO:12336 | SEQ ID NO:20348 |
| iPS:392750 | 21-225_20A10 | NA | AGCGATGACATGCAC | GTTATATGGTTTGATGGAAGTAATAAATACTATGCAGACTCCGTGAAGGGC | GATCTAATAGCAGCAGCTGGTACGGTTGACTAC |
| | | | SEQ ID NO:4325 | SEQ ID NO:12337 | SEQ ID NO:20349 |
| | | AA | SDDMH | VIWFDGSNKYYADSVKG | DLIAAAGTVDY |
| | | | SEQ ID NO:4326 | SEQ ID NO:12338 | SEQ ID NO:20350 |
| iPS:392754 | 21-225_21D3 | NA | AGCTATGGCATGCAC | GTTATATCATATGATGGAAGTAATAAATACTATGCAGACTCCGTGAAGGGC | GGGGTATGGTTCGGGGACCTC |
| | | | SEQ ID NO:4327 | SEQ ID NO:12339 | SEQ ID NO:20351 |
| | | AA | SYGMH | VISYDGSNKYYADSVKG | GVWFGDL |
| | | | SEQ ID NO:4328 | SEQ ID NO:12340 | SEQ ID NO:20352 |
| iPS:392758 | 21-225_21G11 | NA | GACTATGGCATGCAC | GTTATATGGTATGATGTAACTAATGAATACTATGCAGACTCCGTGAAGGGC | GAGCTTGGCTGGTACGAGGACTAC |
| | | | SEQ ID NO:4329 | SEQ ID NO:12341 | SEQ ID NO:20353 |
| | | AA | DYGMH | VIWYDVTNEYYADSVKG | ELGWYEDY |
| | | | SEQ ID NO:4330 | SEQ ID NO:12342 | SEQ ID NO:20354 |

FIGURE 49

| | | | | | |
|---|---|---|---|---|---|
| **iPS:392760** | **21-225_22G3** | NA | AGCTATGCCATGAAC | ATTATTAGTGGTCGTGGTGTTAACACATTCTACGCAGACTCCGTGAAGGGC | CGTTTGGCAGTGGCTGGCTCGGAGGCTTTTGATATC |
| | | | SEQ ID NO:4331 | SEQ ID NO:12343 | SEQ ID NO:20355 |
| | | AA | SYAMN | IISGRGVNTFYADSVKG | RLAVAGSEAFDI |
| | | | SEQ ID NO:4332 | SEQ ID NO:12344 | SEQ ID NO:20356 |
| **iPS:392762** | **21-225_22G5** | NA | AGCTATGCCATGAAC | GTTATTAGTCGTAGTGGTGGTTACACATACTACGCGGACTCCGTGAAGGGC | CGTTTAGCAGTGGCTGGCTCGGAGGCTTTTGATATC |
| | | | SEQ ID NO:4333 | SEQ ID NO:12345 | SEQ ID NO:20357 |
| | | AA | SYAMN | VISRSGGYTYYADSVKG | RLAVAGSEAFDI |
| | | | SEQ ID NO:4334 | SEQ ID NO:12346 | SEQ ID NO:20358 |
| **iPS:392764** | **21-225_22G10** | NA | AGCTATGCCATGAAC | GTTATTAGTGGTAGTGGTGGTAACACATTCTACGCAGACTCCGTGAAGGGC | CGTATGGCAGTGGCTGGCTCGGAGGCTTTTGATATC |
| | | | SEQ ID NO:4335 | SEQ ID NO:12347 | SEQ ID NO:20359 |
| | | AA | SYAMN | VISGSGGNTFYADSVKG | RMAVAGSEAFDI |
| | | | SEQ ID NO:4336 | SEQ ID NO:12348 | SEQ ID NO:20360 |
| **iPS:392766** | **21-225_23H4** | NA | AGCTATGCCATGAAC | GTTATTAGTGGTAGTGGTGGTACCACATACTTCGCAGACTCCGTGAAGGGC | AGAAATAGCAGTGGCTGGCATGATGTTTTTGATATC |
| | | | SEQ ID NO:4337 | SEQ ID NO:12349 | SEQ ID NO:20361 |
| | | AA | SYAMN | VISGSGGTTYFADSVKG | RNSSGWHDVFDI |
| | | | SEQ ID NO:4338 | SEQ ID NO:12350 | SEQ ID NO:20362 |
| **iPS:392768** | **21-225_20B8** | NA | AGTTATAGCATGAAC | TCCATCAGTGGCAGTGGTAGTCACATATACTACGCGGACTCAGTGAAGGGC | GATCGGGGCAGTGGC |
| | | | SEQ ID NO:4339 | SEQ ID NO:12351 | SEQ ID NO:20363 |
| | | AA | SYSMN | SISGSGSHIYYADSVKG | DRGSG |
| | | | SEQ ID NO:4340 | SEQ ID NO:12352 | SEQ ID NO:20364 |

FIGURE 49

| | | | | | |
|---|---|---|---|---|---|
| **iPS:392770** | **21-225_20C10** | NA | AGCTATGCCATGAAC | GTTATTAGTGGTAGTGGTGGTACCACATACTACGCAGACTCCGTGAAGGGC | AGGTATACCAGTGACTGGCATGATGCTTTTGATATC |
| | | | SEQ ID NO:4341 | SEQ ID NO:12353 | SEQ ID NO:20365 |
| | | AA | SYAMN | VISGSGGTTYYADSVKG | RYTSDWHDAFDI |
| | | | SEQ ID NO:4342 | SEQ ID NO:12354 | SEQ ID NO:20366 |
| **iPS:392772** | **21-225_20E12** | NA | AGCTATGGCATGCAC | GTTATGTGGTATGATGAAAGTAATAAACACTATGCAGACTCCGTGAAGGGC | GAACTGGGGTTCCGGTTTGACTAC |
| | | | SEQ ID NO:4343 | SEQ ID NO:12355 | SEQ ID NO:20367 |
| | | AA | SYGMH | VMWYDESNKHYADSVKG | ELGFRFDY |
| | | | SEQ ID NO:4344 | SEQ ID NO:12356 | SEQ ID NO:20368 |
| **iPS:392774** | **21-225_21F3** | NA | AGAAGTAGTTACTACTGGGGC | AGCATCTATTATAGTGGGAGCACCTACTACAACCCGTCCCTCAAGAGT | CTTAGCAGCAGCTGGGACTTCCAGCAC |
| | | | SEQ ID NO:4345 | SEQ ID NO:12357 | SEQ ID NO:20369 |
| | | AA | RSSYYWG | SIYYSGSTYYNPSLKS | LSSSWDFQH |
| | | | SEQ ID NO:4346 | SEQ ID NO:12358 | SEQ ID NO:20370 |
| **iPS:392776** | **21-225_21A12** | NA | AGCTATAGCATGAAC | TCCATTAGTGGTAGTAGTAGTTACATATACTACGCAGACTCAGTGAAGGGC | GCGGCTGGCTTTGACTAC |
| | | | SEQ ID NO:4347 | SEQ ID NO:12359 | SEQ ID NO:20371 |
| | | AA | SYSMN | SISGSSSYIYYADSVKG | AAGFDY |
| | | | SEQ ID NO:4348 | SEQ ID NO:12360 | SEQ ID NO:20372 |
| **iPS:392778** | **21-225_22H3** | NA | AGCTATAGCATGAAC | TCCATTAGTAGTAGTAGTAGTTACATATACTACGCAGACTCAGTGAAGGGC | GATAGGGGCAGCCTC |
| | | | SEQ ID NO:4349 | SEQ ID NO:12361 | SEQ ID NO:20373 |

FIGURE 49

| | | | | | |
|---|---|---|---|---|---|
| | | AA | SYSMN | SISSSSSYIYYADSVKG | DRGSL |
| | | | SEQ ID NO:4350 | SEQ ID NO:12362 | SEQ ID NO:20374 |
| **iPS:392780** | **21-225_22B7** | NA | AGCTATGGCATGCAC | GTTATTTGGTATGAAGAAAATAATCAATACTATGCAGACTCCGTGAAGGGC | GAAGTGGGGTTCCGGTCTGACTAC |
| | | | SEQ ID NO:4351 | SEQ ID NO:12363 | SEQ ID NO:20375 |
| | | AA | SYGMH | VIWYEENNQYYADSVKG | EVGFRSDY |
| | | | SEQ ID NO:4352 | SEQ ID NO:12364 | SEQ ID NO:20376 |
| **iPS:392782** | **21-225_22B12** | NA | AGCTATAGCATGAAC | TCCATTAGTGGTAGTAGTAGTTACACATACTACGCAGACTCAGTGAAGGGC | GTAGCAGCCCTTGACTCC |
| | | | SEQ ID NO:4353 | SEQ ID NO:12365 | SEQ ID NO:20377 |
| | | AA | SYSMN | SISGSSSYTYYADSVKG | VAALDS |
| | | | SEQ ID NO:4354 | SEQ ID NO:12366 | SEQ ID NO:20378 |
| **iPS:392784** | **21-225_23C7** | NA | AGCTATGTTATGAGC | GCTATGAGTGGTAGTGGTGGTAGCACATATTATGTAGACTCCGTGAAGGGC | ACTGGGGTCTTTGACTAC |
| | | | SEQ ID NO:4355 | SEQ ID NO:12367 | SEQ ID NO:20379 |
| | | AA | SYVMS | AMSGSGGSTYYVDSVKG | TGVFDY |
| | | | SEQ ID NO:4356 | SEQ ID NO:12368 | SEQ ID NO:20380 |
| **iPS:392786** | **21-225_24E1** | NA | AATTATGATATCAAC | TGGATGCACCCTAACAGTGGTAACACAGGCTATGCACAGAGGTTCCAGGGC | AGCAGTGGCTGGGAGGTCTTTGACTAC |
| | | | SEQ ID NO:4357 | SEQ ID NO:12369 | SEQ ID NO:20381 |
| | | AA | NYDIN | WMHPNSGNTGYAQRFQG | SSGWEVFDY |
| | | | SEQ ID NO:4358 | SEQ ID NO:12370 | SEQ ID NO:20382 |

**FIGURE 49**

| | | | | | |
|---|---|---|---|---|---|
| **iPS:392788** | **21-225_20C8** | NA | AGCTATAGCATGAAC | TCCATTAGTGGCAGTAGTAGTTACATATACTACGCAGACTCAGTGAAGGCC | GACAGAGGCAGTCTC |
| | | | SEQ ID NO:4359 | SEQ ID NO:12371 | SEQ ID NO:20383 |
| | | AA | SYSMN | SISGSSSYIYYADSVKA | DRGSL |
| | | | SEQ ID NO:4360 | SEQ ID NO:12372 | SEQ ID NO:20384 |
| **iPS:392790** | **21-225_20D10** | NA | GACTATGGCATGCAC | GTTATATGGTTTGATGGAAGTAATAAATACTATGTAGACTCCGTGAAGGGC | GATCTTGGCTGGACGGAAGAGTAC |
| | | | SEQ ID NO:4361 | SEQ ID NO:12373 | SEQ ID NO:20385 |
| | | AA | DYGMH | VIWFDGSNKYYVDSVKG | DLGWTEEY |
| | | | SEQ ID NO:4362 | SEQ ID NO:12374 | SEQ ID NO:20386 |
| **iPS:392792** | **21-225_20G12** | NA | AGCTATAGCATGAAC | TCCATTAGCGGTAGTAGTAGTTACATCTACTACGCAGACTCACTGAAGGGC | GATCGTGGGAGCTAC |
| | | | SEQ ID NO:4363 | SEQ ID NO:12375 | SEQ ID NO:20387 |
| | | AA | SYSMN | SISGSSSYIYYADSLKG | DRGSY |
| | | | SEQ ID NO:4364 | SEQ ID NO:12376 | SEQ ID NO:20388 |
| **iPS:392794** | **21-225_21H3** | NA | AGGAGTAGTTACTACTGGGGC | AATATTTATTATAGTGGGAGCACCTACGACAACCCGTCCCTCAAGAGT | CATGGAAAAGACTGGGGCCTTGACTAC |
| | | | SEQ ID NO:4365 | SEQ ID NO:12377 | SEQ ID NO:20389 |
| | | AA | RSSYYWG | NIYYSGSTYDNPSLKS | HGKDWGLDY |
| | | | SEQ ID NO:4366 | SEQ ID NO:12378 | SEQ ID NO:20390 |
| **iPS:392796** | **21-225_22A4** | NA | GACTATGGCATACAC | GTTATATGGTTTGATGGAAGTAATAAATACTATGCAGACTCCGTGAAGGGC | GATCTTGGCTGGACGGAAGAGTAC |
| | | | SEQ ID NO:4367 | SEQ ID NO:12379 | SEQ ID NO:20391 |

FIGURE 49

| | | | | | |
|---|---|---|---|---|---|
| | | AA | DYGIH | VIWFDGSNKYYADSVKG | DLGWTEEY |
| | | | SEQ ID NO:4368 | SEQ ID NO:12380 | SEQ ID NO:20392 |
| **iPS:392798** | **21-225_22C7** | NA | AGCTATGGCATGCAC | GTTATATGGCATGATGGAAGTAATAAATACTATGCAGACTCCGTGAAGGGC | GATCTGAGTATGGGCGGTATGGACGTC |
| | | | SEQ ID NO:4369 | SEQ ID NO:12381 | SEQ ID NO:20393 |
| | | AA | SYGMH | VIWHDGSNKYYADSVKG | DLSMGGMDV |
| | | | SEQ ID NO:4370 | SEQ ID NO:12382 | SEQ ID NO:20394 |
| **iPS:392800** | **21-225_22D12** | NA | AGGAGTAGTTACTACTGGGGC | AATATCTATTATAGTGGGACCACCTCCTACAACCCGTCCCTCAAGAGT | CTCAGCAGCAGCTGGTCCGTTGACTAC |
| | | | SEQ ID NO:4371 | SEQ ID NO:12383 | SEQ ID NO:20395 |
| | | AA | RSSYYWG | NIYYSGTTSYNPSLKS | LSSSWSVDY |
| | | | SEQ ID NO:4372 | SEQ ID NO:12384 | SEQ ID NO:20396 |
| **iPS:392802** | **21-225_23E7** | NA | AGCTATGCCATGAAC | GCTATTAGTGGTAGTGGTGGTTTCACATACTACGCAGACTCCGTGAAGGGC | ACCAGTGGCTTTGACTAC |
| | | | SEQ ID NO:4373 | SEQ ID NO:12385 | SEQ ID NO:20397 |
| | | AA | SYAMN | AISGSGGFTYYADSVKG | TSGFDY |
| | | | SEQ ID NO:4374 | SEQ ID NO:12386 | SEQ ID NO:20398 |
| **iPS:392806** | **21-225_24H3** | NA | AGCTATGGCATGCAC | GTTATATGGTATGATGGAAGTAATAAATACTATGCAGACTCCGTGAAGGGC | GTAGCAGTGGCAGGCGGTATGGACGTC |
| | | | SEQ ID NO:4375 | SEQ ID NO:12387 | SEQ ID NO:20399 |
| | | AA | SYGMH | VIWYDGSNKYYADSVKG | VAVAGGMDV |
| | | | SEQ ID NO:4376 | SEQ ID NO:12388 | SEQ ID NO:20400 |

FIGURE 49

| | | | | | |
|---|---|---|---|---|---|
| **iPS:392808** | **21-225_20F8** | NA | AGCTATGCCATGAGC | GTTATTAGTGGTAGTGGTGGTAGCACATATTACGCAGACTCCGTGAAGGGC | AGGTATAACAGTGGCTGGCATGATGTTTTTGATATC |
| | | | SEQ ID NO:4377 | SEQ ID NO:12389 | SEQ ID NO:20401 |
| | | AA | SYAMS | VISGSGGSTYYADSVKG | RYNSGWHDVFDI |
| | | | SEQ ID NO:4378 | SEQ ID NO:12390 | SEQ ID NO:20402 |
| **iPS:392810** | **21-225_20H12** | NA | AACTATGGCATGCAC | GTTATATGGTATGATGAAAATAATAAATACTATGTAGACTCCGTGAAGGGC | GAGTTGGGGTTCCGGTCTGACTAC |
| | | | SEQ ID NO:4379 | SEQ ID NO:12391 | SEQ ID NO:20403 |
| | | AA | NYGMH | VIWYDENNKYYVDSVKG | ELGFRSDY |
| | | | SEQ ID NO:4380 | SEQ ID NO:12392 | SEQ ID NO:20404 |
| **iPS:392812** | **21-225_21F4** | NA | AGCTATGCCATGAAC | GTTATTAGTGGTCGTGGTGGTAACACATTCTACGCAGACTCCGTGAAGGGC | CGTTTGGCAGTGGCTGGCTCGGAGGCTTGTGATATC |
| | | | SEQ ID NO:4381 | SEQ ID NO:12393 | SEQ ID NO:20405 |
| | | AA | SYAMN | VISGRGGNTFYADSVKG | RLAVAGSEACDI |
| | | | SEQ ID NO:4382 | SEQ ID NO:12394 | SEQ ID NO:20406 |
| **iPS:392814** | **21-225_22A1** | NA | AGCTATGGCATGCAC | GTTATGTGGTATGATGGAAGTAATAAATATTATGCAGACTCCGTGAAGGGC | GATGGGGGATTTTTGGAGTGGTTAGACTAC |
| | | | SEQ ID NO:4383 | SEQ ID NO:12395 | SEQ ID NO:20407 |
| | | AA | SYGMH | VMWYDGSNKYYADSVKG | DGGFLEWLDY |
| | | | SEQ ID NO:4384 | SEQ ID NO:12396 | SEQ ID NO:20408 |
| **iPS:392816** | **21 225 22E4** | NA | AGCTATGCCATGAGC | ATTATTAGTGGTCGTGGTACTAACACATTCTACGCAGACTCCGTGAAGGGC | CGTATAGCAGTGGCTGGCTCGGAGGCTTTTGATATC |

**FIGURE 49**

| | | | | | |
|---|---|---|---|---|---|
| | **21-225_22E4** | | SEQ ID NO:4385 | SEQ ID NO:12397 | SEQ ID NO:20409 |
| | | AA | SYAMS | IISGRGTNTFYADSVKG | RIAVAGSEAFDI |
| | | | SEQ ID NO:4386 | SEQ ID NO:12398 | SEQ ID NO:20410 |
| **iPS:392818** | **21-225_22D8** | NA | AGCTATGGCATGCAC | ATTATATCATATGATGGA AGTAATAAATACTATGC AGACTCCGTGAAGGGC | GGGGTTTGGTTCGGGGACTT C |
| | | | SEQ ID NO:4387 | SEQ ID NO:12399 | SEQ ID NO:20411 |
| | | AA | SYGMH | IISYDGSNKYYADSVKG | GVWFGDF |
| | | | SEQ ID NO:4388 | SEQ ID NO:12400 | SEQ ID NO:20412 |
| **iPS:392820** | **21-225_23D1** | NA | AGAAGTAGTTACTACTGGGG C | AGTATCTATTATAGTGGG AGCGCCCAGTACAACCC GTCCCTCAAGAGT | CTGAGCAGCAGCTGGTCTTT TGACTAC |
| | | | SEQ ID NO:4389 | SEQ ID NO:12401 | SEQ ID NO:20413 |
| | | AA | RSSYYWG | SIYYSGSAQYNPSLKS | LSSSWSFDY |
| | | | SEQ ID NO:4390 | SEQ ID NO:12402 | SEQ ID NO:20414 |
| **iPS:392822** | **21-225_23C8** | NA | AGGAGTAGTTACTACTGGGG C | AATATTTATTATAGTGGG ACCACTTACAACAACCC GTCCCTCAAGAGT | CATGGAAAAGACTGGGGCCT TGACTAC |
| | | | SEQ ID NO:4391 | SEQ ID NO:12403 | SEQ ID NO:20415 |
| | | AA | RSSYYWG | NIYYSGTTYNNPSLKS | HGKDWGLDY |
| | | | SEQ ID NO:4392 | SEQ ID NO:12404 | SEQ ID NO:20416 |
| **iPS:392824** | **21-225_24E5** | NA | AGGAGTAGTTACTACTGGGG C | AGTATCTATTATAGTGGG AGCGCCAACTACAACCC GTCCCTCAAGAGT | CTCAGCAGCAGCTGGTCCAT TGACAAC |
| | | | SEQ ID NO:4393 | SEQ ID NO:12405 | SEQ ID NO:20417 |
| | | AA | RSSYYWG | SIYYSGSANYNPSLKS | LSSSWSIDN |
| | | | SEQ ID NO:4394 | SEQ ID NO:12406 | SEQ ID NO:20418 |

FIGURE 49

| | | | | | |
|---|---|---|---|---|---|
| **iPS:392826** | **21-225_20B9** | NA | AGCTATAGCATGAAC | TACATTAGTAGTAGTAGTAGTACCATATACTATGCAGACTCTGTGAAGGGC | TCACTATGGTCCCCCTTTGACTAC |
| | | | SEQ ID NO:4395 | SEQ ID NO:12407 | SEQ ID NO:20419 |
| | | AA | SYSMN | YISSSSSTIYYADSVKG | SLWSPFDY |
| | | | SEQ ID NO:4396 | SEQ ID NO:12408 | SEQ ID NO:20420 |
| **iPS:392830** | **21-225_21A5** | NA | AGTTACTTCTGGAGC | CGTATCTATACCAGTGGGATCACCAACTACAACCCCTCCCTCAAGAGT | GGCCCGACTTCGGGGTGGTTCGACCCC |
| | | | SEQ ID NO:4397 | SEQ ID NO:12409 | SEQ ID NO:20421 |
| | | AA | SYFWS | RIYTSGITNYNPSLKS | GPTSGWFDP |
| | | | SEQ ID NO:4398 | SEQ ID NO:12410 | SEQ ID NO:20422 |
| **iPS:392832** | **21-225_21H8** | NA | GACTATGGCATGCAC | GTTATATGGTTTGATGGAAGTAATAAATACTATGCAGACTCCGTGAAGGGC | GATCTTGGCTGGACGGAAGAGTAC |
| | | | SEQ ID NO:4399 | SEQ ID NO:12411 | SEQ ID NO:20423 |
| | | AA | DYGMH | VIWFDGSNKYYADSVKG | DLGWTEEY |
| | | | SEQ ID NO:4400 | SEQ ID NO:12412 | SEQ ID NO:20424 |
| **iPS:392834** | **21-225_22C1** | NA | AGGAGTAGTTACTACTGGGGC | AATATCTATTATAGTGGGGCCACCTATTATAATTCGTCCCTCAAGAGT | CATAGCGGCAGCTGGTCCCTTGACTAC |
| | | | SEQ ID NO:4401 | SEQ ID NO:12413 | SEQ ID NO:20425 |
| | | AA | RSSYYWG | NIYYSGATYYNSSLKS | HSGSWSLDY |
| | | | SEQ ID NO:4402 | SEQ ID NO:12414 | SEQ ID NO:20426 |
| **iPS:392836** | **21-225_22F4** | NA | GACTATGGCATGCAC | GTTATATGGTATGATGGAAGTAATAAATACTATGTAGACTCCGTGAAGGGC | GAAAAGTATAGCAGCAGCTGGTACGACTACGGTATGGACGTC |
| | | | SEQ ID NO:4403 | SEQ ID NO:12415 | SEQ ID NO:20427 |

**FIGURE 49**

| | | | | | |
|---|---|---|---|---|---|
| | | AA | DYGMH | VIWYDGSNKYYVDSVKG | EKYSSSWYDYGMDV |
| | | | SEQ ID NO:4404 | SEQ ID NO:12416 | SEQ ID NO:20428 |
| **iPS:392838** | **21-225_22G8** | NA | AGGAGTAGTTACTACTGGGG C | AATATCTATTATAGTGGG AGCACCTACTACAACCC GTCCGTCAAGAGT | CATGGAAAAGACTGGGGCCT TGACTAC |
| | | | SEQ ID NO:4405 | SEQ ID NO:12417 | SEQ ID NO:20429 |
| | | AA | RSSYYWG | NIYYSGSTYYNPSVKS | HGKDWGLDY |
| | | | SEQ ID NO:4406 | SEQ ID NO:12418 | SEQ ID NO:20430 |
| **iPS:392840** | **21-225_23G1** | NA | AGCTATGCCATGAGC | GTTATTAGTGGTAGTGGT GGTACCACATATAACAC AGACTCCGTGAAGGGC | AGCTCCTTGTTTGACTAC |
| | | | SEQ ID NO:4407 | SEQ ID NO:12419 | SEQ ID NO:20431 |
| | | AA | SYAMS | VISGSGGTTYNTDSVKG | SSLFDY |
| | | | SEQ ID NO:4408 | SEQ ID NO:12420 | SEQ ID NO:20432 |
| **iPS:392842** | **21-225_23G8** | NA | AGCTATGCCATGAGC | GCTATTAGTGGTAGTGGT GGTAGCACATACTACGC AGACTCCGTGAAGGGC | AGCAGTGGCTGGTTCGCC |
| | | | SEQ ID NO:4409 | SEQ ID NO:12421 | SEQ ID NO:20433 |
| | | AA | SYAMS | AISGSGGSTYYADSVKG | SSGWFA |
| | | | SEQ ID NO:4410 | SEQ ID NO:12422 | SEQ ID NO:20434 |
| **iPS:392844** | **21-225_23E11** | NA | TCCTATACCATGAAC | TCCATTAGTGGTAGTAGT AGTTACATATGGTATGTA GACTCAGTGAAGGGC | GATCGGGGCAGTCTC |
| | | | SEQ ID NO:4411 | SEQ ID NO:12423 | SEQ ID NO:20435 |
| | | AA | SYTMN | SISGSSSYIWYVDSVKG | DRGSL |
| | | | SEQ ID NO:4412 | SEQ ID NO:12424 | SEQ ID NO:20436 |

FIGURE 49

| | | | | | |
|---|---|---|---|---|---|
| **iPS:392846** | **21-225_24B6** | NA | AACTATGTCATGCAC | GTTATATGGTATGATGGAAGTAATAAATACTATGCAGACTCCGTGAAGGGC | GAGGAATATAGTAGTGGCTGGTACGACTACGGTATGGACGTC |
| | | | SEQ ID NO:4413 | SEQ ID NO:12425 | SEQ ID NO:20437 |
| | | AA | NYVMH | VIWYDGSNKYYADSVKG | EEYSSGWYDYGMDV |
| | | | SEQ ID NO:4414 | SEQ ID NO:12426 | SEQ ID NO:20438 |
| **iPS:392848** | **21-225_20F9** | NA | AGCTATAGCATGAAC | TCCATTAGTAGTAGTAGTAGTTACATATACTACGCAGACTCAGTGAAGGGC | GATCGTGGGAGCTGC |
| | | | SEQ ID NO:4415 | SEQ ID NO:12427 | SEQ ID NO:20439 |
| | | AA | SYSMN | SISSSSSYIYYADSVKG | DRGSC |
| | | | SEQ ID NO:4416 | SEQ ID NO:12428 | SEQ ID NO:20440 |
| **iPS:392850** | **21-225_20H10** | NA | ACCTATAGCATGAAC | TCCATTAGTAGTAGTAGTAGTTACATATACTACGCAGACTCAGTGAAGGGC | GATCGTGGGAGCCTC |
| | | | SEQ ID NO:4417 | SEQ ID NO:12429 | SEQ ID NO:20441 |
| | | AA | TYSMN | SISSSSSYIYYADSVKG | DRGSL |
| | | | SEQ ID NO:4418 | SEQ ID NO:12430 | SEQ ID NO:20442 |
| **iPS:392852** | **21-225_21A2** | NA | AGCTATGCCATGAAC | ATTATTAGTGGTCGTGGTGGTAACACATTCTACGCAGACTCCGTGAAGGGC | CGTTTGGCAGTGGCTGGCTCGGAGGCTTTTGATATC |
| | | | SEQ ID NO:4419 | SEQ ID NO:12431 | SEQ ID NO:20443 |
| | | AA | SYAMN | IISGRGGNTFYADSVKG | RLAVAGSEAFDI |
| | | | SEQ ID NO:4420 | SEQ ID NO:12432 | SEQ ID NO:20444 |
| **iPS:392854** | **21 225 21E5** | NA | GACTATGGCATGCAC | GTTATATGGTATGATGAAAGTAATAAATACTATGCAGACTCCGTGAAGGGC | GAACTGGGGTTCCGGTCTGACTAT |

FIGURE 49

| | | | | | |
|---|---|---|---|---|---|
| | **21-225_21E5** | | SEQ ID NO:4421 | SEQ ID NO:12433 | SEQ ID NO:20445 |
| | | AA | DYGMH | VIWYDESNKYYADSVKG | ELGFRSDY |
| | | | SEQ ID NO:4422 | SEQ ID NO:12434 | SEQ ID NO:20446 |
| **iPS:392856** | **21-225_22A2** | NA | AGCTATGCCATGAGC | GGTATTAGTGGTAGTGG AGGTAACACACCCTACG CAGACTCCGTGAAGGGC | GTAGTGGGAGCTGTCCAC |
| | | | SEQ ID NO:4423 | SEQ ID NO:12435 | SEQ ID NO:20447 |
| | | AA | SYAMS | GISGSGGNTPYADSVKG | VVGAVH |
| | | | SEQ ID NO:4424 | SEQ ID NO:12436 | SEQ ID NO:20448 |
| **iPS:392858** | **21-225_22H4** | NA | AGGAGTAGTTACTACTGGGG C | AATATTTATTATAGTGGG AGCACCTACCACAACCC GTCTCTCAAGAGT | CATGGAAAAGACTGGGGCCT TGACTAC |
| | | | SEQ ID NO:4425 | SEQ ID NO:12437 | SEQ ID NO:20449 |
| | | AA | RSSYYWG | NIYYSGSTYHNPSLKS | HGKDWGLDY |
| | | | SEQ ID NO:4426 | SEQ ID NO:12438 | SEQ ID NO:20450 |
| **iPS:392860** | **21-225_22H8** | NA | AGCTATGGCATGCAC | GTTATCTGGTATGATGGA AATAATAAATACTATGC AGACTCCGTGAAGGGC | GAGCTTGCCTGGTACGAAGA CTAC |
| | | | SEQ ID NO:4427 | SEQ ID NO:12439 | SEQ ID NO:20451 |
| | | AA | SYGMH | VIWYDGNNKYYADSVKG | ELAWYEDY |
| | | | SEQ ID NO:4428 | SEQ ID NO:12440 | SEQ ID NO:20452 |
| **iPS:392864** | **21-225_23B9** | NA | AGTGGTGGTTACTACTGGAG C | TACATCTATTACAGTGGG AGCACCTACTACAACCC GTCCCTCAAGAGT | GAGGACGGTGCCTTCGGCTA CTACGGTATGGACGTC |
| | | | SEQ ID NO:4429 | SEQ ID NO:12441 | SEQ ID NO:20453 |
| | | AA | SGGYYWS | YIYYSGSTYYNPSLKS | EDGAFGYYGMDV |

FIGURE 49

| | | | | | |
|---|---|---|---|---|---|
| | | | SEQ ID NO:4430 | SEQ ID NO:12442 | SEQ ID NO:20454 |
| **iPS:392866** | **21-225_23H11** | NA | AGCTATGGCATGCAC | GTTATATGGTATGATGAAAATAATAAATACTATGTAGACTCCGTGAAGGGC | GAATTGGGGTTCCGGTCTGACTAC |
| | | | SEQ ID NO:4431 | SEQ ID NO:12443 | SEQ ID NO:20455 |
| | | AA | SYGMH | VIWYDENNKYYVDSVKG | ELGFRSDY |
| | | | SEQ ID NO:4432 | SEQ ID NO:12444 | SEQ ID NO:20456 |
| **iPS:392868** | **21-225_24D6** | NA | AGCTATGGCATGCAC | ATTATATCATATGCTGGAAGTAATAAATCCTATGCAGACTCCGTGAAGGGC | CGGGGATACAGCTATGGCGGGTACGGTATGGACGTC |
| | | | SEQ ID NO:4433 | SEQ ID NO:12445 | SEQ ID NO:20457 |
| | | AA | SYGMH | IISYAGSNKSYADSVKG | RGYSYGGYGMDV |
| | | | SEQ ID NO:4434 | SEQ ID NO:12446 | SEQ ID NO:20458 |
| **iPS:392870** | **21-225_20G9** | NA | AGGAGTAGTTACTACTGGGGC | AGTATCTATTATAGTGGGAGCGCCTCTTACAACCCGTCCCTCAAGAGT | CTGAGCAGCAGCTGGTCTTTTGACTAC |
| | | | SEQ ID NO:4435 | SEQ ID NO:12447 | SEQ ID NO:20459 |
| | | AA | RSSYYWG | SIYYSGSASYNPSLKS | LSSSWSFDY |
| | | | SEQ ID NO:4436 | SEQ ID NO:12448 | SEQ ID NO:20460 |
| **iPS:392872** | **21-225_20B11** | NA | AGCTATGTCATGCAC | GTTATATGGTATGATGGAAGTAATAAATACTATGCAGACTCCGTGAAGGTC | GAGAGATATACCAGTGGCTGGTATGACTACGGTATGGACGTC |
| | | | SEQ ID NO:4437 | SEQ ID NO:12449 | SEQ ID NO:20461 |
| | | AA | SYVMH | VIWYDGSNKYYADSVKV | ERYTSGWYDYGMDV |
| | | | SEQ ID NO:4438 | SEQ ID NO:12450 | SEQ ID NO:20462 |

FIGURE 49

| | | | | | |
|---|---|---|---|---|---|
| **iPS:392874** | **21-225_21D2** | NA | AACTATGCCATGAGC | GTTCTTAGTGGTAGTGGTGGTAGCACATTCTACGCAGACTCCGTGAAGGGC | TATTGTAGTAGTGCCAGGTGCCCTTATGATGCCTTTGATATC |
| | | | SEQ ID NO:4439 | SEQ ID NO:12451 | SEQ ID NO:20463 |
| | | AA | NYAMS | VLSGSGGSTFYADSVKG | YCSSARCPYDAFDI |
| | | | SEQ ID NO:4440 | SEQ ID NO:12452 | SEQ ID NO:20464 |
| **iPS:392876** | **21-225_21F7** | NA | GACTATGGCATGCAC | GTTATATGGTTTGATGGAAATAATAAATACTATGTAGACTCCGTGAAGGGC | GATCTTGGCTGGACGGAAGAGTAC |
| | | | SEQ ID NO:4441 | SEQ ID NO:12453 | SEQ ID NO:20465 |
| | | AA | DYGMH | VIWFDGNNKYYVDSVKG | DLGWTEEY |
| | | | SEQ ID NO:4442 | SEQ ID NO:12454 | SEQ ID NO:20466 |
| **iPS:392878** | **21-225_22C5** | NA | AGCTATGCCATGAGC | ATTATTAGTGGTAGTGGTGGTTACACATACTACGCGGACTCCGTGAAGGGC | CGTATAGCAGTGGCTGGCTCGGAGGCTTTTGATATC |
| | | | SEQ ID NO:4443 | SEQ ID NO:12455 | SEQ ID NO:20467 |
| | | AA | SYAMS | IISGSGGYTYYADSVKG | RIAVAGSEAFDI |
| | | | SEQ ID NO:4444 | SEQ ID NO:12456 | SEQ ID NO:20468 |
| **iPS:392880** | **21-225_22F9** | NA | AGCTATGGCATGCAC | GTTATATGGTATGAGGAAAATAATAAAGACTATGTAGACTCCGTGAAGGGC | GAGTTAGGCTTCCAGTCTGACTAC |
| | | | SEQ ID NO:4445 | SEQ ID NO:12457 | SEQ ID NO:20469 |
| | | AA | SYGMH | VIWYEENNKDYVDSVKG | ELGFQSDY |
| | | | SEQ ID NO:4446 | SEQ ID NO:12458 | SEQ ID NO:20470 |

FIGURE 49

| | | | | | |
|---|---|---|---|---|---|
| **iPS:392882** | **21-225_23A3** | NA | AGGAGTAGTTACTACTGGGGC | AATATTTATTATAGTGGGAGCACCTACAACAACCCGTCCCTCAAGAGT | CATGGAAAAGACTGGGGCCTTGACTTC |
| | | | SEQ ID NO:4447 | SEQ ID NO:12459 | SEQ ID NO:20471 |
| | | AA | RSSYYWG | NIYYSGSTYNNPSLKS | HGKDWGLDF |
| | | | SEQ ID NO:4448 | SEQ ID NO:12460 | SEQ ID NO:20472 |
| **iPS:392884** | **21-225_23A10** | NA | AGCTATGGCATGCAC | GTTATATGGTATGATGGAAGTAATAAATACTATGCAGACTCCGTGAAGGGC | GAAAGGTATAGCAGTGGCTGGCACGACTACGGTATGGACGTC |
| | | | SEQ ID NO:4449 | SEQ ID NO:12461 | SEQ ID NO:20473 |
| | | AA | SYGMH | VIWYDGSNKYYADSVKG | ERYSSGWHDYGMDV |
| | | | SEQ ID NO:4450 | SEQ ID NO:12462 | SEQ ID NO:20474 |
| **iPS:392886** | **21-225_23A12** | NA | AATTATGATATCAAC | TGGATGCACCCTAACAGTGGTAACACAGGCTATGCACAGAAGTTCCAGGGC | AGCAGTGGCTGGTACTACTTTGACTAC |
| | | | SEQ ID NO:4451 | SEQ ID NO:12463 | SEQ ID NO:20475 |
| | | AA | NYDIN | WMHPNSGNTGYAQKFQG | SSGWYYFDY |
| | | | SEQ ID NO:4452 | SEQ ID NO:12464 | SEQ ID NO:20476 |
| **iPS:392888** | **21-225_25A2** | NA | AGCTATGGCATGCAC | GTTATATGGTATGATGGAAGTAATAAATACTATGCAGACTCCGTGAAGGGC | AGGTATAGCAGCAGCTGGTCAGGGGGTATGGACGTC |
| | | | SEQ ID NO:4453 | SEQ ID NO:12465 | SEQ ID NO:20477 |
| | | AA | SYGMH | VIWYDGSNKYYADSVKG | RYSSSWSGGMDV |
| | | | SEQ ID NO:4454 | SEQ ID NO:12466 | SEQ ID NO:20478 |

FIGURE 49

| | | | | | |
|---|---|---|---|---|---|
| **iPS:392890** | **21-225_20H9** | NA | AGCTATGCCATGAGC | GCTATTAGTGGTAGTGGTGGTTACACATACTACGCAGACTCCGTGAAGGGC | GGGGGGTCCCTCTTCTAC |
| | | | SEQ ID NO:4455 | SEQ ID NO:12467 | SEQ ID NO:20479 |
| | | AA | SYAMS | AISGSGGYTYYADSVKG | GGSLFY |
| | | | SEQ ID NO:4456 | SEQ ID NO:12468 | SEQ ID NO:20480 |
| **iPS:392892** | **21-225_20C11** | NA | AGCTATGCCATGAGC | ACTATTAGTGGTCGTGGTGGTCACACATACTACGCAGACTCCGTGAAGGGC | CAGGACTGC |
| | | | SEQ ID NO:4457 | SEQ ID NO:12469 | SEQ ID NO:20481 |
| | | AA | SYAMS | TISGRGGHTYYADSVKG | QDC |
| | | | SEQ ID NO:4458 | SEQ ID NO:12470 | SEQ ID NO:20482 |
| **iPS:392894** | **21-225_21G2** | NA | GACTATGGCATGCAC | GTTATATGGTATGATGTAACTAATAAATACTATGCAGACTCCGTGAAGGGC | GAGCTTGGCTGGTACGAGGACTAC |
| | | | SEQ ID NO:4459 | SEQ ID NO:12471 | SEQ ID NO:20483 |
| | | AA | DYGMH | VIWYDVTNKYYADSVKG | ELGWYEDY |
| | | | SEQ ID NO:4460 | SEQ ID NO:12472 | SEQ ID NO:20484 |
| **iPS:392896** | **21-225_21G7** | NA | AGAAGTAGTTACTACTGGGGC | AATATCTATTATAGTGGGTATAGTTACTACAATCCGTCCCTCAAGAGT | CATAGCACCAGCTGGTCCCTTGACTAC |
| | | | SEQ ID NO:4461 | SEQ ID NO:12473 | SEQ ID NO:20485 |
| | | AA | RSSYYWG | NIYYSGYSYYNPSLKS | HSTSWSLDY |
| | | | SEQ ID NO:4462 | SEQ ID NO:12474 | SEQ ID NO:20486 |
| **iPS:392898** | **21 225 21H10** | NA | AACGCCTGGATGAAC | CGTATTAAAAGCAAAACTGATGGTGGGACAACAGACTACGCTGCACCCGTGAAAGGC | GAAGGCTGGAACACGGACTAC |

**FIGURE 49**

| | | | | | |
|---|---|---|---|---|---|
| | | | SEQ ID NO:4463 | SEQ ID NO:12475 | SEQ ID NO:20487 |
| | **21-225_21H10** | AA | NAWMN | RIKSKTDGGTTDYAAPVKG | EGWNTDY |
| | | | SEQ ID NO:4464 | SEQ ID NO:12476 | SEQ ID NO:20488 |
| **iPS:392900** | **21-225_22F2** | NA | GACTATGGCATGCAC | GTTATATGGTATGAGGGAAGTAATAAATACTATGTAGACTCCGTGAGGGGC | GAGCTAGGCTTCCAGTCTGACTAC |
| | | | SEQ ID NO:4465 | SEQ ID NO:12477 | SEQ ID NO:20489 |
| | | AA | DYGMH | VIWYEGSNKYYVDSVRG | ELGFQSDY |
| | | | SEQ ID NO:4466 | SEQ ID NO:12478 | SEQ ID NO:20490 |
| **iPS:392902** | **21-225_22D5** | NA | AGCTATGCCATGAGC | GTTATTAGTGGTCGTGGTGGTAACACATTCTACGCAGACTCCGTGAAGGGC | CGTATAGCAGTGGCTGGCTCGGAGGCTTTTGATATC |
| | | | SEQ ID NO:4467 | SEQ ID NO:12479 | SEQ ID NO:20491 |
| | | AA | SYAMS | VISGRGGNTFYADSVKG | RIAVAGSEAFDI |
| | | | SEQ ID NO:4468 | SEQ ID NO:12480 | SEQ ID NO:20492 |
| **iPS:392904** | **21-225_22G9** | NA | GGTAGTAATTACTACTGGGGC | AATATCTATTATAGTGGGAGCACCTACTACAACCCGTCCCTCAAGAGT | CATAGCAGTAGCTGGTCCCTTGACTAC |
| | | | SEQ ID NO:4469 | SEQ ID NO:12481 | SEQ ID NO:20493 |
| | | AA | GSNYYWG | NIYYSGSTYYNPSLKS | HSSSWSLDY |
| | | | SEQ ID NO:4470 | SEQ ID NO:12482 | SEQ ID NO:20494 |
| **iPS:392908** | **21-225_23F12** | NA | AGCTATGGCATGCAC | GTTATATGGTATGATGAAACTAATAAATACTATGCAGACTCCGTGAAGGGC | GAGCTTGCCTGGTACGAGGACTAC |
| | | | SEQ ID NO:4471 | SEQ ID NO:12483 | SEQ ID NO:20495 |
| | | AA | SYGMH | VIWYDETNKYYADSVKG | ELAWYEDY |

**FIGURE 49**

| | | | | | |
|---|---|---|---|---|---|
| | | | SEQ ID NO:4472 | SEQ ID NO:12484 | SEQ ID NO:20496 |
| **iPS:392912** | **21-225_25A9** | NA | AGCTATGGCATGCAC | GTTATATGGTATGATGTAACTAATAAATACTATACAGGCTCCGTGAAGGGC | GAAATTGGCTGGTTAGATGACTAC |
| | | | SEQ ID NO:4473 | SEQ ID NO:12485 | SEQ ID NO:20497 |
| | | AA | SYGMH | VIWYDVTNKYYTGSVKG | EIGWLDDY |
| | | | SEQ ID NO:4474 | SEQ ID NO:12486 | SEQ ID NO:20498 |
| **iPS:392914** | **21-225_25D12** | NA | AGCGATGGCATGCAC | GTTATATGGTATGATGGAAGTAATAAATACTATGCAGACTCCGTGAAGGGC | GAGAGGTATAGCAGCAGCTGGTACGACTACGGTATGGACGTC |
| | | | SEQ ID NO:4475 | SEQ ID NO:12487 | SEQ ID NO:20499 |
| | | AA | SDGMH | VIWYDGSNKYYADSVKG | ERYSSSWYDYGMDV |
| | | | SEQ ID NO:4476 | SEQ ID NO:12488 | SEQ ID NO:20500 |
| **iPS:392916** | **21-225_27C5** | NA | AGCTATAGCATGAAC | TCCACTAGTAGTAGTGATAGTTATATATACTACGCAGACTCAGTGAAGGGC | GTGGCGTCCTTTGACTGC |
| | | | SEQ ID NO:4477 | SEQ ID NO:12489 | SEQ ID NO:20501 |
| | | AA | SYSMN | STSSSDSYIYYADSVKG | VASFDC |
| | | | SEQ ID NO:4478 | SEQ ID NO:12490 | SEQ ID NO:20502 |
| **iPS:392918** | **21-225_28F5** | NA | AGCTATGGCATGCAC | GTTATATGGTATGATGAAAATAATAAATACTATGCAGACTCCGTGAAGGGC | GAATTAGGCTGGTACGACGACTAC |
| | | | SEQ ID NO:4479 | SEQ ID NO:12491 | SEQ ID NO:20503 |
| | | AA | SYGMH | VIWYDENNKYYADSVKG | ELGWYDDY |

**FIGURE 49**

| | | | | | |
|---|---|---|---|---|---|
| | | | SEQ ID NO:4480 | SEQ ID NO:12492 | SEQ ID NO:20504 |
| **iPS:392920** | **21-225_29G4** | NA | GACTATGGCATACAC | GTTATATGGTATGATGAAAGTAATAAATACTATGCAGACTCCATGAAGGGC | GAACTGGGAATGACGGGTGACTAC |
| | | | SEQ ID NO:4481 | SEQ ID NO:12493 | SEQ ID NO:20505 |
| | | AA | DYGIH | VIWYDESNKYYADSMKG | ELGMTGDY |
| | | | SEQ ID NO:4482 | SEQ ID NO:12494 | SEQ ID NO:20506 |
| **iPS:392922** | **21-225_30G4** | NA | AGCTATGGCATGCAC | GTTATATGGTATGATGGAACTGATAAATACTATGTAGACTCCGTGAAGGGC | GAAAATAGCAGCTCGTACTACTTTGACTAC |
| | | | SEQ ID NO:4483 | SEQ ID NO:12495 | SEQ ID NO:20507 |
| | | AA | SYGMH | VIWYDGTDKYYVDSVKG | ENSSSYYFDY |
| | | | SEQ ID NO:4484 | SEQ ID NO:12496 | SEQ ID NO:20508 |
| **iPS:392924** | **21-225_32H2** | NA | AGCTATGGCATGCAC | GTTATATGGTATGATGGAAGTAATAAATACTATGCAGACTCCGTGAAGGGC | AGATATAGCAGCAGCTGGACGGGGGTATGGACGTC |
| | | | SEQ ID NO:4485 | SEQ ID NO:12497 | SEQ ID NO:20509 |
| | | AA | SYGMH | VIWYDGSNKYYADSVKG | RYSSSWTGGMDV |
| | | | SEQ ID NO:4486 | SEQ ID NO:12498 | SEQ ID NO:20510 |
| **iPS:392928** | **21-225_25A4** | NA | AATTATGATATTAAT | TGGATGTACCCTAACAGTGGTAACACAGGCTATGCACAGAAATTCCAGGGC | AGCAGTGGCTGGTACTACTTTGACTAC |
| | | | SEQ ID NO:4487 | SEQ ID NO:12499 | SEQ ID NO:20511 |

FIGURE 49

| | | | | | |
|---|---|---|---|---|---|
| | | AA | NYDIN | WMYPNSGNTGYAQKFQG | SSGWYYFDY |
| | | | SEQ ID NO:4488 | SEQ ID NO:12500 | SEQ ID NO:20512 |
| **iPS:392930** | **21-225_25H9** | NA | AACTATGGCATGCAC | ATTATATGGTATGATGG AAGTTATAAATACTATG CAGACTCCGTGAAGGGC | GAGGGATACGATTTTTGGAG TGGCTTCTTTGACTCC |
| | | | SEQ ID NO:4489 | SEQ ID NO:12501 | SEQ ID NO:20513 |
| | | AA | NYGMH | IIWYDGSYKYYADSVKG | EGYDFWSGFFDS |
| | | | SEQ ID NO:4490 | SEQ ID NO:12502 | SEQ ID NO:20514 |
| **iPS:392934** | **21-225_27D5** | NA | AGCTATGGCATGCAC | GTTATATGGTATGATGA AAGTAATAAATACTATG GAGACTCCGTGAAGGGC | GAACTGGGGTTCCTCTCTGA CTAC |
| | | | SEQ ID NO:4491 | SEQ ID NO:12503 | SEQ ID NO:20515 |
| | | AA | SYGMH | VIWYDESNKYYGDSVKG | ELGFLSDY |
| | | | SEQ ID NO:4492 | SEQ ID NO:12504 | SEQ ID NO:20516 |
| **iPS:392936** | **21-225_28B6** | NA | AATTATGATATTAAT | TGGATGCACCCTGACAG TGGTAACACAGGCTATG CACAGAAGTTCCAGGGC | AGCAGTGGCTGGTACTACTT TGACTAC |
| | | | SEQ ID NO:4493 | SEQ ID NO:12505 | SEQ ID NO:20517 |
| | | AA | NYDIN | WMHPDSGNTGYAQKFQG | SSGWYYFDY |
| | | | SEQ ID NO:4494 | SEQ ID NO:12506 | SEQ ID NO:20518 |
| **iPS:392938** | **21-225_29H4** | NA | AGCTATGGCATACAC | GTTATATGGTATGATGG AAGTAATAAATACTATG CAGACTCCGTGAAGGGC | AGGTATAGCAGCAGCTGGTC AGGGGGTATGGACGTC |
| | | | SEQ ID NO:4495 | SEQ ID NO:12507 | SEQ ID NO:20519 |

## FIGURE 49

| | | | | | |
|---|---|---|---|---|---|
| | | AA | SYGIH | VIWYDGSNKYYADSVKG | RYSSSWSGGMDV |
| | | | SEQ ID NO:4496 | SEQ ID NO:12508 | SEQ ID NO:20520 |
| **iPS:392940** | **21-225_29D9** | NA | GACTATGGCATTCAC | GTTATATGGTATGATGAAAGTAATAACTACTATGCAGACTCCGTGAAGGGC | GAAATTGGCTGGTTAGATGACTAC |
| | | | SEQ ID NO:4497 | SEQ ID NO:12509 | SEQ ID NO:20521 |
| | | AA | DYGIH | VIWYDESNNYYADSVKG | EIGWLDDY |
| | | | SEQ ID NO:4498 | SEQ ID NO:12510 | SEQ ID NO:20522 |
| **iPS:392942** | **21-225_30E9** | NA | AGCTGTGCCATGAAC | GCTATTAGTGGTCGTGGTGGTAGCACATTCTACGCAGACTCCGTGAAGGGC | GGGGAGCTACTAGAGGACTACTACTACTACGGAATGGACGTC |
| | | | SEQ ID NO:4499 | SEQ ID NO:12511 | SEQ ID NO:20523 |
| | | AA | SCAMN | AISGRGGSTFYADSVKG | GELLEDYYYYGMDV |
| | | | SEQ ID NO:4500 | SEQ ID NO:12512 | SEQ ID NO:20524 |
| **iPS:392944** | **21-225_31H5** | NA | AGCTATGCCATGAGC | GCTATTAGTGGTCGTGGTGGAAGCATATTCCACGCAGACTCCGTGAAGGGC | GGGGAGCTACTAGAGGACTACTACTTCTACGGTATGGACGTC |
| | | | SEQ ID NO:4501 | SEQ ID NO:12513 | SEQ ID NO:20525 |
| | | AA | SYAMS | AISGRGGSIFHADSVKG | GELLEDYYFYGMDV |
| | | | SEQ ID NO:4502 | SEQ ID NO:12514 | SEQ ID NO:20526 |
| **iPS:392948** | **21-225_25G5** | NA | GACTATGGCATACAC | GTTATTTGGTATGATGGAAATAATAAATATTATGCAGACTCCGTGAAGGGC | GAAATTGGCTGGTTAGATGACTAC |
| | | | SEQ ID NO:4503 | SEQ ID NO:12515 | SEQ ID NO:20527 |
| | | AA | DYGIH | VIWYDGNNKYYADSVKG | EIGWLDDY |
| | | | SEQ ID NO:4504 | SEQ ID NO:12516 | SEQ ID NO:20528 |

FIGURE 49

| | | | | | |
|---|---|---|---|---|---|
| **iPS:392950** | **21-225_25C10** | NA | AGCTATAGGATGAAC | TCCATTAGTAGTAGTAGT AGTACCATATACTACGC AGACTCTGTGAAGGGC | ACGGCTGGTTTTGACTAC |
| | | | SEQ ID NO:4505 | SEQ ID NO:12517 | SEQ ID NO:20529 |
| | | AA | SYRMN | SISSSSSTIYYADSVKG | TAGFDY |
| | | | SEQ ID NO:4506 | SEQ ID NO:12518 | SEQ ID NO:20530 |
| **iPS:392952** | **21-225_26G1** | NA | AGCTATGGCATGAAC | TCCATTAGTGGTAGTAGT AGTTACATATACTACGC GGACTCAGTGAAGGGC | CTGACTACCTTTGACTTC |
| | | | SEQ ID NO:4507 | SEQ ID NO:12519 | SEQ ID NO:20531 |
| | | AA | SYGMN | SISGSSSYIYYADSVKG | LTTFDF |
| | | | SEQ ID NO:4508 | SEQ ID NO:12520 | SEQ ID NO:20532 |
| **iPS:392954** | **21-225_26A10** | NA | AGCTATGCCATGAGC | GTTATTAGTGGTAGTGGT GTTAACACATTCTACGCA GACTCCGTGAAGGGC | AAGATAGCAGTGGCTGGTAC TCACTACTTTGACTAC |
| | | | SEQ ID NO:4509 | SEQ ID NO:12521 | SEQ ID NO:20533 |
| | | AA | SYAMS | VISGSGVNTFYADSVKG | KIAVAGTHYFDY |
| | | | SEQ ID NO:4510 | SEQ ID NO:12522 | SEQ ID NO:20534 |
| **iPS:392956** | **21-225_27A11** | NA | AGCTATGGCATGCAC | GTTATATGGTATGATGG AAGTAATAAATACTATG CAGACTCCGTGAAGGGC | GATTCCTCCCCCTACGGTAT GGACGTC |
| | | | SEQ ID NO:4511 | SEQ ID NO:12523 | SEQ ID NO:20535 |
| | | AA | SYGMH | VIWYDGSNKYYADSVKG | DSSPYGMDV |
| | | | SEQ ID NO:4512 | SEQ ID NO:12524 | SEQ ID NO:20536 |
| **iPS:392958** | **21 225 28C7** | NA | AGCTATGGCATGCAC | GTTATATGGTATGATGA AAGTAATAAATACTATG CAGACTCCGTGAAGGGC | GAATTAGGCTGGTACGACGA CTAC |

FIGURE 49

| | | | | | |
|---|---|---|---|---|---|
| | **21-225_28C7** | | SEQ ID NO:4513 | SEQ ID NO:12525 | SEQ ID NO:20537 |
| | | AA | SYGMH | VIWYDESNKYYADSVKG | ELGWYDDY |
| | | | SEQ ID NO:4514 | SEQ ID NO:12526 | SEQ ID NO:20538 |
| **iPS:392960** | **21-225_29E6** | NA | AATTATGATATTAAT | TGGATGCACCCTAACAG TGGTAACACAGGCTATG CACAGAAATTCCAGGGC | AGCAGTGGCTGGTACTACTT TGACTAC |
| | | | SEQ ID NO:4515 | SEQ ID NO:12527 | SEQ ID NO:20539 |
| | | AA | NYDIN | WMHPNSGNTGYAQKFQG | SSGWYYFDY |
| | | | SEQ ID NO:4516 | SEQ ID NO:12528 | SEQ ID NO:20540 |
| **iPS:392962** | **21-225_30A1** | NA | AGCTATGTCATGAAC | GCTATTAGTGGTAGTGGT GGTAGCACATACTACGC AGACTCCGTGAAGGGC | ACTGGGGTCTTTGACTAC |
| | | | SEQ ID NO:4517 | SEQ ID NO:12529 | SEQ ID NO:20541 |
| | | AA | SYVMN | AISGSGGSTYYADSVKG | TGVFDY |
| | | | SEQ ID NO:4518 | SEQ ID NO:12530 | SEQ ID NO:20542 |
| **iPS:392964** | **21-225_31A8** | NA | AGCTATGCCATGAGC | GCTATTAGTGGTCGTGGT GGTAGCACATTCCACGC AGACTCCGTGAAGGGC | GGGGAGCTACTAGAGGACTA CTACTTCTACGGTATGGACG TC |
| | | | SEQ ID NO:4519 | SEQ ID NO:12531 | SEQ ID NO:20543 |
| | | AA | SYAMS | AISGRGGSTFHADSVKG | GELLEDYYFYGMDV |
| | | | SEQ ID NO:4520 | SEQ ID NO:12532 | SEQ ID NO:20544 |
| **iPS:392966** | **21-225_32G3** | NA | AGCTATAGCATGAAC | TCCATTAGTGGTAGTAGT AGTTACATATACTACGC AGACTCAGTGAAGGGC | GGCAATATAGCAAGGGACTA C |
| | | | SEQ ID NO:4521 | SEQ ID NO:12533 | SEQ ID NO:20545 |
| | | AA | SYSMN | SISGSSSYIYYADSVKG | GNIARDY |
| | | | SEQ ID NO:4522 | SEQ ID NO:12534 | SEQ ID NO:20546 |

## FIGURE 49

| | | | | | |
|---|---|---|---|---|---|
| **iPS:392968** | **21-225_25B6** | NA | AACTATGGCATGCAC | GTTATATGGTATGAGGAAAGTAATAAATACTATACAGAGTCCGTGAAGGGC | GAACTGGGGTTCCTCTCTGACTAC |
| | | | SEQ ID NO:4523 | SEQ ID NO:12535 | SEQ ID NO:20547 |
| | | AA | NYGMH | VIWYEESNKYYTESVKG | ELGFLSDY |
| | | | SEQ ID NO:4524 | SEQ ID NO:12536 | SEQ ID NO:20548 |
| **iPS:392972** | **21-225_26A2** | NA | AGCTATGGCATGCAC | GTTATATGGTATGAAGGAAGTAATAAATACTATGTAGACTCCGTGAAGGGC | GAATTAGGCTGGTACGACGACTAC |
| | | | SEQ ID NO:4525 | SEQ ID NO:12537 | SEQ ID NO:20549 |
| | | AA | SYGMH | VIWYEGSNKYYVDSVKG | ELGWYDDY |
| | | | SEQ ID NO:4526 | SEQ ID NO:12538 | SEQ ID NO:20550 |
| **iPS:392974** | **21-225_26A11** | NA | AACTGTGTCATGCAC | GTTATATGGTATGATGGAAGTAATAAATACTATGCAGACTCCGTGAAGGGC | GAGGAGTATAGCAGTGGCTGGTACGACTACGGTATGGACGTC |
| | | | SEQ ID NO:4527 | SEQ ID NO:12539 | SEQ ID NO:20551 |
| | | AA | NCVMH | VIWYDGSNKYYADSVKG | EEYSSGWYDYGMDV |
| | | | SEQ ID NO:4528 | SEQ ID NO:12540 | SEQ ID NO:20552 |
| **iPS:392976** | **21-225_27H12** | NA | AGCTATAGCCTGAAC | TCCATTAGTGGTAGTAGTAGTAACATATACTACACAGACTCAGTGAAGGGC | GTGGCGTCCTTTGACTAC |
| | | | SEQ ID NO:4529 | SEQ ID NO:12541 | SEQ ID NO:20553 |
| | | AA | SYSLN | SISGSSSNIYYTDSVKG | VASFDY |
| | | | SEQ ID NO:4530 | SEQ ID NO:12542 | SEQ ID NO:20554 |

FIGURE 49

| | | | | | |
|---|---|---|---|---|---|
| **iPS:392978** | **21-225_28B8** | NA | GACTATGGCATGCAC | GTTATATGGTATGATGCAAATAATAAATACTATGCAGACTCCGTGAAGGGC | GAAATTGGCTGGTTAGATGACTAC |
| | | | SEQ ID NO:4531 | SEQ ID NO:12543 | SEQ ID NO:20555 |
| | | AA | DYGMH | VIWYDANNKYYADSVKG | EIGWLDDY |
| | | | SEQ ID NO:4532 | SEQ ID NO:12544 | SEQ ID NO:20556 |
| **iPS:392980** | **21-225_29H6** | NA | GACTATGGCATGCAC | GTTATATGGTATAATGAAAATAATAAATACTATGCAGACTCCGTGAAGGGC | GAACTGGGGATGACGGGTGACTCC |
| | | | SEQ ID NO:4533 | SEQ ID NO:12545 | SEQ ID NO:20557 |
| | | AA | DYGMH | VIWYNENNKYYADSVKG | ELGMTGDS |
| | | | SEQ ID NO:4534 | SEQ ID NO:12546 | SEQ ID NO:20558 |
| **iPS:392982** | **21-225_30D1** | NA | AGCTATGCCATGAGC | GCTATTAGTGGTCGTGGTGGTAGCACATTCCACGCAGACTCCGTGAAGGGC | GGGGAGCTACTAGAGGACTACTACTTCTACGGTTTGGACGTC |
| | | | SEQ ID NO:4535 | SEQ ID NO:12547 | SEQ ID NO:20559 |
| | | AA | SYAMS | AISGRGGSTFHADSVKG | GELLEDYYFYGLDV |
| | | | SEQ ID NO:4536 | SEQ ID NO:12548 | SEQ ID NO:20560 |
| **iPS:392984** | **21-225_30E11** | NA | ATCTATGCCATGAGC | GTTATTAGTGGTAGTGGTGGTAGCTCATTCTACGCAGACTCCGTGAAGGGC | GATCGGGTGAAAGCTCATGATGGTTTTGATATC |
| | | | SEQ ID NO:4537 | SEQ ID NO:12549 | SEQ ID NO:20561 |
| | | AA | IYAMS | VISGSGGSSFYADSVKG | DRVKAHDGFDI |
| | | | SEQ ID NO:4538 | SEQ ID NO:12550 | SEQ ID NO:20562 |

# FIGURE 49

| | | | | | |
|---|---|---|---|---|---|
| **iPS:392986** | **21-225_31B8** | NA | AGCTATGCCATGAGC | GCTATTAGTGGTCGTGGTGGTAGCACATTCCACGCAGACTCCGTGAAGGGC | GGGGAGCTACTAGAGGACTACTACTTCTACGGTATGGACGTC |
| | | | SEQ ID NO:4539 | SEQ ID NO:12551 | SEQ ID NO:20563 |
| | | AA | SYAMS | AISGRGGSTFHADSVKG | GELLEDYYFYGMDV |
| | | | SEQ ID NO:4540 | SEQ ID NO:12552 | SEQ ID NO:20564 |
| **iPS:392988** | **21-225_25E6** | NA | GACTATGGCATGCAC | GTTATATGGTATGATGAAAATAATAAATACTATGCAGACTCCGTGAAGGGC | GAACTGGGGATGACGGGTGACTCC |
| | | | SEQ ID NO:4541 | SEQ ID NO:12553 | SEQ ID NO:20565 |
| | | AA | DYGMH | VIWYDENNKYYADSVKG | ELGMTGDS |
| | | | SEQ ID NO:4542 | SEQ ID NO:12554 | SEQ ID NO:20566 |
| **iPS:392990** | **21-225_25H10** | NA | GACTATGGCATGCAC | GTTATATGGTATGATGAAAGTAATAAATACTATGCAGACTCCATGAAGGGC | GAACTGGGGATGACGGGTGACTAC |
| | | | SEQ ID NO:4543 | SEQ ID NO:12555 | SEQ ID NO:20567 |
| | | AA | DYGMH | VIWYDESNKYYADSMKG | ELGMTGDY |
| | | | SEQ ID NO:4544 | SEQ ID NO:12556 | SEQ ID NO:20568 |
| **iPS:392992** | **21-225_26C4** | NA | AATTATGATATCAAC | TGGATGAACCCTAACAGTGGTAACACAGGCTATGCACAGAGATTCCAGGGC | AGCAGTGGCTGGTACTACTTTGACTAC |
| | | | SEQ ID NO:4545 | SEQ ID NO:12557 | SEQ ID NO:20569 |
| | | AA | NYDIN | WMNPNSGNTGYAQRFQG | SSGWYYFDY |
| | | | SEQ ID NO:4546 | SEQ ID NO:12558 | SEQ ID NO:20570 |

## FIGURE 49

| | | | | | |
|---|---|---|---|---|---|
| **iPS:392994** | **21-225_26G11** | NA | AGCTATGGCATGCAC | GTTATATGGTATGATGGAAGTAATAAATACTATGCAGACTCCGTGAAGGGC | AGGTATAGCAACAGCTGGTCAGGGGGTATGGACGTC |
| | | | SEQ ID NO:4547 | SEQ ID NO:12559 | SEQ ID NO:20571 |
| | | AA | SYGMH | VIWYDGSNKYYADSVKG | RYSNSWSGGMDV |
| | | | SEQ ID NO:4548 | SEQ ID NO:12560 | SEQ ID NO:20572 |
| **iPS:392996** | **21-225_28B1** | NA | AGCTATGCCATGAGC | GCTATTAGTGGTAGTGGAGGTAACACATTCTACGCAGACTCCGTGAAGGGC | TTGGGGCGTATAGCAGTGACTGGTCCTTACTTTGACTAC |
| | | | SEQ ID NO:4549 | SEQ ID NO:12561 | SEQ ID NO:20573 |
| | | AA | SYAMS | AISGSGGNTFYADSVKG | LGRIAVTGPYFDY |
| | | | SEQ ID NO:4550 | SEQ ID NO:12562 | SEQ ID NO:20574 |
| **iPS:392998** | **21-225_28A9** | NA | AGCTATGGCATACAC | GTTATATGGTTTGATGGAAGTAATAAATACTATGCAGACTCCGTGAAGGGC | GAAATTGGCTGGTTAGATGACTAC |
| | | | SEQ ID NO:4551 | SEQ ID NO:12563 | SEQ ID NO:20575 |
| | | AA | SYGIH | VIWFDGSNKYYADSVKG | EIGWLDDY |
| | | | SEQ ID NO:4552 | SEQ ID NO:12564 | SEQ ID NO:20576 |
| **iPS:393000** | **21-225_29D7** | NA | AGCTATGGCATGCAC | GTTATATGGTATGATGAAAGTAATAAATACTATGGAGACTCCGTGAAGGGC | GAACTGGGGTTCCTCTCTGACTAC |
| | | | SEQ ID NO:4553 | SEQ ID NO:12565 | SEQ ID NO:20577 |
| | | AA | SYGMH | VIWYDESNKYYGDSVKG | ELGFLSDY |
| | | | SEQ ID NO:4554 | SEQ ID NO:12566 | SEQ ID NO:20578 |

FIGURE 49

| | | | | | |
|---|---|---|---|---|---|
| **iPS:393002** | **21-225_30G1** | NA | TACTATGGCATGCAC | GTTATATGGCATGATGG AAGTAATAAATACTATG TAGACTCCGTGAAGGGC | GAAAATAGCAGTTCGTACTA CTTTGACTAC |
| | | | SEQ ID NO:4555 | SEQ ID NO:12567 | SEQ ID NO:20579 |
| | | AA | YYGMH | VIWHDGSNKYYVDSVKG | ENSSSYYFDY |
| | | | SEQ ID NO:4556 | SEQ ID NO:12568 | SEQ ID NO:20580 |
| **iPS:393004** | **21-225_30G11** | NA | AGCTATGCCATGAGC | GCTATTAGTGGTCGTGGT GGTAGCACATTCAACGC AGACTCCGTGAAGGGC | GGGGAGCTACTAGAGGACTA CTACTTCTACGGTATGGACG TC |
| | | | SEQ ID NO:4557 | SEQ ID NO:12569 | SEQ ID NO:20581 |
| | | AA | SYAMS | AISGRGGSTFNADSVKG | GELLEDYYFYGMDV |
| | | | SEQ ID NO:4558 | SEQ ID NO:12570 | SEQ ID NO:20582 |
| **iPS:393006** | **21-225_31G9** | NA | AGCTATAGCATGAAC | TCAATTAGTAGTAGTAGT AGTTACATATACTACGC AGACTCAGTGAAGGGC | GATCGAGGCAGCAGC |
| | | | SEQ ID NO:4559 | SEQ ID NO:12571 | SEQ ID NO:20583 |
| | | AA | SYSMN | SISSSSSYIYYADSVKG | DRGSS |
| | | | SEQ ID NO:4560 | SEQ ID NO:12572 | SEQ ID NO:20584 |
| **iPS:393010** | **21-225_25E11** | NA | AGCTATGCCATGAGC | GTTATTAGTGGTGGTGGT GGTAGCACATACTACGC AGACTCCGTGAAGGGC | CGTGGATATAGTGGCTACGA GGACCTCCTCTACTTTGACT GC |
| | | | SEQ ID NO:4561 | SEQ ID NO:12573 | SEQ ID NO:20585 |
| | | AA | SYAMS | VISGGGGSTYYADSVKG | RGYSGYEDLLYFDC |
| | | | SEQ ID NO:4562 | SEQ ID NO:12574 | SEQ ID NO:20586 |
| **iPS:393012** | **21-225_26G7** | NA | AGCTATGGCATGCAC | GTTATATGGTATGATGG AAGTAATAAATATTATG CAGACTCCGTGAAGGGC | AGGTATAGCAGCAGCTGGTC AGGGGGTATGGACGTC |
| | | | SEQ ID NO:4563 | SEQ ID NO:12575 | SEQ ID NO:20587 |

FIGURE 49

| | | | | | |
|---|---|---|---|---|---|
| | | AA | SYGMH | VIWYDGSNKYYADSVKG | RYSSSWSGGMDV |
| | | | SEQ ID NO:4564 | SEQ ID NO:12576 | SEQ ID NO:20588 |
| **iPS:393014** | **21-225_26D12** | NA | AGTTATGGCATGCAC | GTTATATGGTATGATGG AAGTAATGAATACTATG CAGACTCCGTGAAGGGC | GATTCCTCCCCCTACGGTAT GGACGTC |
| | | | SEQ ID NO:4565 | SEQ ID NO:12577 | SEQ ID NO:20589 |
| | | AA | SYGMH | VIWYDGSNEYYADSVKG | DSSPYGMDV |
| | | | SEQ ID NO:4566 | SEQ ID NO:12578 | SEQ ID NO:20590 |
| **iPS:393016** | **21-225_28F11** | NA | AGCTATGCCATGAGC | GTTACTAGTGGTAGTGGT GGTACCACATTCTACGC AGACTCCGTGAAGGGC | CGGACCCAGTTTGATGATTT TGATATC |
| | | | SEQ ID NO:4567 | SEQ ID NO:12579 | SEQ ID NO:20591 |
| | | AA | SYAMS | VTSGSGGTTFYADSVKG | RTQFDDFDI |
| | | | SEQ ID NO:4568 | SEQ ID NO:12580 | SEQ ID NO:20592 |
| **iPS:393018** | **21-225_29B8** | NA | GACTATGGCATGCAC | GTTATATGGTATGATGA AAATAATAAATACTATG CAGACTCCGTGAAGGGC | GAACTGGGGATGACGGGTGA CTCC |
| | | | SEQ ID NO:4569 | SEQ ID NO:12581 | SEQ ID NO:20593 |
| | | AA | DYGMH | VIWYDENNKYYADSVKG | ELGMTGDS |
| | | | SEQ ID NO:4570 | SEQ ID NO:12582 | SEQ ID NO:20594 |
| **iPS:393020** | **21-225_30E2** | NA | AGCTATGGCATGCAC | GTTATATCATATGGTGGA AGTAATAAATTCTATGC AGTCTCCGTGAAGGGC | AGGGGGTATAGCAGTGGAGG CTACGGTATGGACGTC |
| | | | SEQ ID NO:4571 | SEQ ID NO:12583 | SEQ ID NO:20595 |
| | | AA | SYGMH | VISYGGSNKFYAVSVKG | RGYSSGGYGMDV |
| | | | SEQ ID NO:4572 | SEQ ID NO:12584 | SEQ ID NO:20596 |

FIGURE 49

| | | | | | |
|---|---|---|---|---|---|
| **iPS:393022** | **21-225_30H11** | NA | AGCTATAGCATGAAC | TCCATTAGTGGTAGTAGTAGTTACATATACTACGCAGACTCAGTGAAGGGC | GATAGGGGGAGCCTC |
| | | | SEQ ID NO:4573 | SEQ ID NO:12585 | SEQ ID NO:20597 |
| | | AA | SYSMN | SISGSSSYIYYADSVKG | DRGSL |
| | | | SEQ ID NO:4574 | SEQ ID NO:12586 | SEQ ID NO:20598 |
| **iPS:393024** | **21-225_31H9** | NA | AGCTGTGCCATGAAC | GCTATTAGTGGTAGTGGTGGTAGCTCATTCTACGCAGACTCCGTGAAGGGC | CGGACTCCCTATGATGTCTTTGATATC |
| | | | SEQ ID NO:4575 | SEQ ID NO:12587 | SEQ ID NO:20599 |
| | | AA | SCAMN | AISGSGGSSFYADSVKG | RTPYDVFDI |
| | | | SEQ ID NO:4576 | SEQ ID NO:12588 | SEQ ID NO:20600 |
| **iPS:393026** | **21-225_32B6** | NA | GACTATGGCATGCAC | GTTATATGGTATGATGAAAATACTAAATACTATGCAGACTCCGTGAAGGGC | GAGTGGGGGGACTAC |
| | | | SEQ ID NO:4577 | SEQ ID NO:12589 | SEQ ID NO:20601 |
| | | AA | DYGMH | VIWYDENTKYYADSVKG | EWGDY |
| | | | SEQ ID NO:4578 | SEQ ID NO:12590 | SEQ ID NO:20602 |
| **iPS:393028** | **21-225_25D7** | NA | AGCTATGCCATGAGC | GCTATTAGTGGTAGAGGTGGTACCACATTCTACGCAGACTCCGTGAAGGGC | GACGGGTACGGTGGTAACTCCTTCTTTGACTAC |
| | | | SEQ ID NO:4579 | SEQ ID NO:12591 | SEQ ID NO:20603 |
| | | AA | SYAMS | AISGRGGTTFYADSVKG | DGYGGNSFFDY |
| | | | SEQ ID NO:4580 | SEQ ID NO:12592 | SEQ ID NO:20604 |
| **iPS:393030** | **21 225 25H11** | NA | GACTATGGCATGCAC | GTTATATGGTATGATGAAAATAATGAATACTATGCAGACTCCGTGAAGGGC | GAACTGGGGATGACGGGTGACTCC |

**FIGURE 49**

| | | | | | |
|---|---|---|---|---|---|
| | **21-225_25H11** | | SEQ ID NO:4581 | SEQ ID NO:12593 | SEQ ID NO:20605 |
| | | AA | DYGMH | VIWYDENNEYYADSVKG | ELGMTGDS |
| | | | SEQ ID NO:4582 | SEQ ID NO:12594 | SEQ ID NO:20606 |
| **iPS:393032** | **21-225_26F8** | NA | GGCTATGGCATGCAC | ATTATATGGTATGATGGAAGTAATAAATACTATGCAGACTCCGTGAAGGGC | GAGAGGTACGATTTTTGGAGTGGTTGTATGGACGTC |
| | | | SEQ ID NO:4583 | SEQ ID NO:12595 | SEQ ID NO:20607 |
| | | AA | GYGMH | IIWYDGSNKYYADSVKG | ERYDFWSGCMDV |
| | | | SEQ ID NO:4584 | SEQ ID NO:12596 | SEQ ID NO:20608 |
| **iPS:393034** | **21-225_27F2** | NA | GACTATGGCATGCAC | GTTATATGGTATGATGAAAATAATAAATACTATGTAGACTCCGTGAGGGGC | GAACTGGGGATGACGGGTGACTCC |
| | | | SEQ ID NO:4585 | SEQ ID NO:12597 | SEQ ID NO:20609 |
| | | AA | DYGMH | VIWYDENNKYYVDSVRG | ELGMTGDS |
| | | | SEQ ID NO:4586 | SEQ ID NO:12598 | SEQ ID NO:20610 |
| **iPS:393036** | **21-225_28G3** | NA | ACCTATGGCATGCAC | GTTATATGGTATGATGGAAGTAATAAATACTATGCAGACTCCGTGAAGGGC | GATCGATACGATTTTTGGAGTGGTTATTTTGACTAC |
| | | | SEQ ID NO:4587 | SEQ ID NO:12599 | SEQ ID NO:20611 |
| | | AA | TYGMH | VIWYDGSNKYYADSVKG | DRYDFWSGYFDY |
| | | | SEQ ID NO:4588 | SEQ ID NO:12600 | SEQ ID NO:20612 |

FIGURE 49

| | | | | | |
|---|---|---|---|---|---|
| **iPS:393038** | **21-225_29D8** | NA | GACTATGGCATACAC | GTTATATGGTTTGATGGAACTAATAAATACTATGCAGACTCCGTGAAGGGC | GAAATTGGCTGGTTAGATGACTAC |
| | | | SEQ ID NO:4589 | SEQ ID NO:12601 | SEQ ID NO:20613 |
| | | AA | DYGIH | VIWFDGTNKYYADSVKG | EIGWLDDY |
| | | | SEQ ID NO:4590 | SEQ ID NO:12602 | SEQ ID NO:20614 |
| **iPS:393040** | **21-225_30E3** | NA | AGCTATGCCATGAAC | GCTATTAGTGGTCGTGGTGGTAGCACATTCTACGCAGACTCCGAGAAGGGC | GGGGAGCTATTAGAGGACTACTACTACTACGGAATGGACGTC |
| | | | SEQ ID NO:4591 | SEQ ID NO:12603 | SEQ ID NO:20615 |
| | | AA | SYAMN | AISGRGGSTFYADSEKG | GELLEDYYYYGMDV |
| | | | SEQ ID NO:4592 | SEQ ID NO:12604 | SEQ ID NO:20616 |
| **iPS:393042** | **21-225_31F1** | NA | GACTACTATATGCAC | TGGATCAACCCTAACAGTGGTGGCACAAACTATGCACAGAAGTTTCAGGGC | GATAGTAGCAATTTCAGCAACTGGTACGATTACTACGGTATGGACGTC |
| | | | SEQ ID NO:4593 | SEQ ID NO:12605 | SEQ ID NO:20617 |
| | | AA | DYYMH | WINPNSGGTNYAQKFQG | DSSNFSNWYDYYGMDV |
| | | | SEQ ID NO:4594 | SEQ ID NO:12606 | SEQ ID NO:20618 |
| **iPS:393044** | **21-225_25B8** | NA | AGCTATGGTATCAGC | TGGATCAGCGCTTACAATGGTAACACAACCTATGCACAGAAGCTCCGGGGC | ACCGCTGCTGGGTATAGCAGCAGCTGGTTTGACTAC |
| | | | SEQ ID NO:4595 | SEQ ID NO:12607 | SEQ ID NO:20619 |
| | | AA | SYGIS | WISAYNGNTTYAQKLRG | TAAGYSSSWFDY |
| | | | SEQ ID NO:4596 | SEQ ID NO:12608 | SEQ ID NO:20620 |

FIGURE 49

| | | | | | |
|---|---|---|---|---|---|
| **iPS:393046** | **21-225_25A12** | NA | AACTGTGTCATGCAC | GTTATATGGTATGATGGAAGTAATAAATACTATGCAGACTCCGTGAAGGGC | GAGGAGTATAGCAGTGGCTGGTACGACTACGGTATGGACGTC |
| | | | SEQ ID NO:4597 | SEQ ID NO:12609 | SEQ ID NO:20621 |
| | | AA | NCVMH | VIWYDGSNKYYADSVKG | EEYSSGWYDYGMDV |
| | | | SEQ ID NO:4598 | SEQ ID NO:12610 | SEQ ID NO:20622 |
| **iPS:393048** | **21-225_27C3** | NA | GACTATGGCATGCAC | GTTATATGGTATGATGAAAATAATAAATCCTATGCAGACTCCGTGAAGGGC | GAACTGGGGATGACGGGTGACTCC |
| | | | SEQ ID NO:4599 | SEQ ID NO:12611 | SEQ ID NO:20623 |
| | | AA | DYGMH | VIWYDENNKSYADSVKG | ELGMTGDS |
| | | | SEQ ID NO:4600 | SEQ ID NO:12612 | SEQ ID NO:20624 |
| **iPS:393050** | **21-225_28C5** | NA | AGCTATGGTATCAGC | TGGATCAGCGCTTACAATGGTAACACAACCTATGCACAGAAGCTCCGGGGC | ACCGCTGCTGGGTATAGCAGCAGCTGGTTTGACTAC |
| | | | SEQ ID NO:4601 | SEQ ID NO:12613 | SEQ ID NO:20625 |
| | | AA | SYGIS | WISAYNGNTTYAQKLRG | TAAGYSSSWFDY |
| | | | SEQ ID NO:4602 | SEQ ID NO:12614 | SEQ ID NO:20626 |
| **iPS:393054** | **21-225_29G8** | NA | GACTATGGCATGCAC | GTTATATGGTATGATGAAACTAATAAATACTATGCAGACTCCGTGAAGGGC | GAACTGGGGATGACGAGTGACTAC |
| | | | SEQ ID NO:4603 | SEQ ID NO:12615 | SEQ ID NO:20627 |
| | | AA | DYGMH | VIWYDETNKYYADSVKG | ELGMTSDY |
| | | | SEQ ID NO:4604 | SEQ ID NO:12616 | SEQ ID NO:20628 |

FIGURE 49

| | | | | | |
|---|---|---|---|---|---|
| **iPS:393056** | **21-225_30F3** | NA | AGCTATGGCATGCAC | GTTATATGGTATGATGTAAGTAATAAATACTATGCAGACTCCGTGAAGGGC | GAAATGGGCTGGTACGATGACTAC |
| | | | SEQ ID NO:4605 | SEQ ID NO:12617 | SEQ ID NO:20629 |
| | | AA | SYGMH | VIWYDVSNKYYADSVKG | EMGWYDDY |
| | | | SEQ ID NO:4606 | SEQ ID NO:12618 | SEQ ID NO:20630 |
| **iPS:393058** | **21-225_31H3** | NA | AGCTATGCCATGAAC | GCTATTAGTGGTCGTGGTGGTAACACATTCTACGCAGACTCCGTGAAGGGC | GGGGAGTTACTAGAGGACTACTACTACTACGGAATGGACGTC |
| | | | SEQ ID NO:4607 | SEQ ID NO:12619 | SEQ ID NO:20631 |
| | | AA | SYAMN | AISGRGGNTFYADSVKG | GELLEDYYYYGMDV |
| | | | SEQ ID NO:4608 | SEQ ID NO:12620 | SEQ ID NO:20632 |
| **iPS:393060** | **21-225_32G12** | NA | AGCTATGCCATGAGC | TCTATTAGTGGTCGTGGTGGTAGCACATTCCACGCAGACTCCGTGAAGGGC | GGGGAGCTACTAGAGGACTACTACTTCTACGGTATGGACGTC |
| | | | SEQ ID NO:4609 | SEQ ID NO:12621 | SEQ ID NO:20633 |
| | | AA | SYAMS | SISGRGGSTFHADSVKG | GELLEDYYFYGMDV |
| | | | SEQ ID NO:4610 | SEQ ID NO:12622 | SEQ ID NO:20634 |
| **iPS:393062** | **21-225_33H3** | NA | AGCTATGGCATGCAC | ATTATATCATATGGTGGAAGTAATAACTTCTATGCAGACTCCGTGAAGGGC | AGGGGGTATAGCAGTGGAGGCTACGGTATGGACGTC |
| | | | SEQ ID NO:4611 | SEQ ID NO:12623 | SEQ ID NO:20635 |
| | | AA | SYGMH | IISYGGSNNFYADSVKG | RGYSSGGYGMDV |
| | | | SEQ ID NO:4612 | SEQ ID NO:12624 | SEQ ID NO:20636 |
| **iPS:393064** | **21-225_33A9** | NA | AGTTATGATATCAAC | TGGATGAACCCTAACAGTGGTAACACAGGCTATGCACAGAAGTTCCAGGGC | AAGAAGGCTAACGACTAC |
| | | | SEQ ID NO:4613 | SEQ ID NO:12625 | SEQ ID NO:20637 |

FIGURE 49

| | | | | | |
|---|---|---|---|---|---|
| | | AA | SYDIN | WMNPNSGNTGYAQKFQG | KKANDY |
| | | | SEQ ID NO:4614 | SEQ ID NO:12626 | SEQ ID NO:20638 |
| **iPS:393066** | **21-225_34D3** | NA | TACTATGGCATGCAC | GTTATATGGCATGATGGAAGTAATAAATACTATGTAGACTCCGTGAAGGGC | GAAAATAGCAGTTCGTTCTACTTTGACTAC |
| | | | SEQ ID NO:4615 | SEQ ID NO:12627 | SEQ ID NO:20639 |
| | | AA | YYGMH | VIWHDGSNKYYVDSVKG | ENSSSFYFDY |
| | | | SEQ ID NO:4616 | SEQ ID NO:12628 | SEQ ID NO:20640 |
| **iPS:393068** | **21-225_34G9** | NA | AGCTATGCCATGAGC | GCTATTAGTGGTCGTGGTGGTAGCACATTCCACGCAGACTCCGTGAAGGGC | GGGGAGCTACTAGAGGACTACTACTTCTACGGTATGGACGTC |
| | | | SEQ ID NO:4617 | SEQ ID NO:12629 | SEQ ID NO:20641 |
| | | AA | SYAMS | AISGRGGSTFHADSVKG | GELLEDYYFYGMDV |
| | | | SEQ ID NO:4618 | SEQ ID NO:12630 | SEQ ID NO:20642 |
| **iPS:393072** | **21-225_36C5** | NA | AGCTATGCCATGAAC | GCTATTAGTGGTCGTGGTGGTAGCACATTCCACGCAGACTCCGTGAAGGGC | GGGGAGCTATTAGAGGACTACTACTTCTACGGTATGGACGTC |
| | | | SEQ ID NO:4619 | SEQ ID NO:12631 | SEQ ID NO:20643 |
| | | AA | SYAMN | AISGRGGSTFHADSVKG | GELLEDYYFYGMDV |
| | | | SEQ ID NO:4620 | SEQ ID NO:12632 | SEQ ID NO:20644 |
| **iPS:393074** | **21-225_33B1** | NA | GACTATGGCATGCAC | GTTATATGGTATGATAGAAATAATAAATACTATGCAGACTCCGTGAAGGGC | GAAATGGGCTGGTACGATGACTAC |
| | | | SEQ ID NO:4621 | SEQ ID NO:12633 | SEQ ID NO:20645 |
| | | AA | DYGMH | VIWYDRNNKYYADSVKG | EMGWYDDY |

## FIGURE 49

| | | | | | |
|---|---|---|---|---|---|
| | | | SEQ ID NO:4622 | SEQ ID NO:12634 | SEQ ID NO:20646 |
| **iPS:393076** | **21-225_33A4** | NA | AGCTATGCCATGAAC | GCTATTAGTCGTCGTGGTGGTAGCACATTCTACGCAGACTCCGTGAAGGGC | GGGGAACTACTAGAGGACTACTCCTACTACGGTATCGACGTC |
| | | | SEQ ID NO:4623 | SEQ ID NO:12635 | SEQ ID NO:20647 |
| | | AA | SYAMN | AISRRGGSTFYADSVKG | GELLEDYSYYGIDV |
| | | | SEQ ID NO:4624 | SEQ ID NO:12636 | SEQ ID NO:20648 |
| **iPS:393078** | **21-225_33H11** | NA | AGCTATAGCATGAAC | TCCATTAGTGGTAGTAGTAGTTACATATACTACGCAGACTCAGTGAAGGGC | ACAAACGGTATGGACGTC |
| | | | SEQ ID NO:4625 | SEQ ID NO:12637 | SEQ ID NO:20649 |
| | | AA | SYSMN | SISGSSSYIYYADSVKG | TNGMDV |
| | | | SEQ ID NO:4626 | SEQ ID NO:12638 | SEQ ID NO:20650 |
| **iPS:393080** | **21-225_34F3** | NA | GGCTACCATATGCAC | TGGATCAACCCTAACAGTGGTGGCACAAACTATGCACAGAAGTTTCAGGGC | GACGGTACCAGCTCCTTTGACTAC |
| | | | SEQ ID NO:4627 | SEQ ID NO:12639 | SEQ ID NO:20651 |
| | | AA | GYHMH | WINPNSGGTNYAQKFQG | DGTSSFDY |
| | | | SEQ ID NO:4628 | SEQ ID NO:12640 | SEQ ID NO:20652 |
| **iPS:393082** | **21-225_34C11** | NA | AGTTATACCATGAGC | TCCATTAGTGGTAGTAGTAATTACATATACTACGCAGACTCAGTGAAGGGC | TTAACTGGTTTTGACTAC |
| | | | SEQ ID NO:4629 | SEQ ID NO:12641 | SEQ ID NO:20653 |
| | | AA | SYTMS | SISGSSNYIYYADSVKG | LTGFDY |
| | | | SEQ ID NO:4630 | SEQ ID NO:12642 | SEQ ID NO:20654 |
| **iPS:393084** | **21-225_35C6** | NA | GGCGATTATATGCAC | TGGATCAGCCCTAAAAATGGTGGCACAAACTATGCACAGAAGTTTCAGGGC | GATGGAACTGGGTCCTTTGACTAC |
| | | | SEQ ID NO:4631 | SEQ ID NO:12643 | SEQ ID NO:20655 |

**FIGURE 49**

| | | | | | |
|---|---|---|---|---|---|
| | | AA | GDYMH | WISPKNGGTNYAQKFQG | DGTGSFDY |
| | | | SEQ ID NO:4632 | SEQ ID NO:12644 | SEQ ID NO:20656 |
| **iPS:393086** | **21-225_36H5** | NA | GACTATCATATGCAC | TGGATCAACCCTAATAG GGGTGGCACAAACTATG CACAGAAGTTTCAGGAC | GATGGAACTGGGTCCTTTGA CTAC |
| | | | SEQ ID NO:4633 | SEQ ID NO:12645 | SEQ ID NO:20657 |
| | | AA | DYHMH | WINPNRGGTNYAQKFQD | DGTGSFDY |
| | | | SEQ ID NO:4634 | SEQ ID NO:12646 | SEQ ID NO:20658 |
| **iPS:393088** | **21-225_33D1** | NA | AATTATGACATTAAC | TGGATGCACCCTAACAG TGGTAACACAGGCTTTG CACAGAAGTTCCGGGGC | AGCAGTGGCTGGTACTTTTT TGACTAC |
| | | | SEQ ID NO:4635 | SEQ ID NO:12647 | SEQ ID NO:20659 |
| | | AA | NYDIN | WMHPNSGNTGFAQKFRG | SSGWYFFDY |
| | | | SEQ ID NO:4636 | SEQ ID NO:12648 | SEQ ID NO:20660 |
| **iPS:393090** | **21-225_33A5** | NA | AGCTATGTCATAAAC | GCTATTAGTGGTAGTGGT GTTAGCACATACTACGC AGACTCCGTGAAGGGC | ACTTCCCTCTTTGACTAC |
| | | | SEQ ID NO:4637 | SEQ ID NO:12649 | SEQ ID NO:20661 |
| | | AA | SYVIN | AISGSGVSTYYADSVKG | TSLFDY |
| | | | SEQ ID NO:4638 | SEQ ID NO:12650 | SEQ ID NO:20662 |
| **iPS:393092** | **21-225_33C12** | NA | CACTATGGCATGCAC | GTTATATGGTATGATGG AAGTAATAAATACTATG CAGACTCCGTGAAGGGC | GAAAATAGCAGCTCGTACTA CTTTGACTAC |
| | | | SEQ ID NO:4639 | SEQ ID NO:12651 | SEQ ID NO:20663 |
| | | AA | HYGMH | VIWYDGSNKYYADSVKG | ENSSSYYFDY |
| | | | SEQ ID NO:4640 | SEQ ID NO:12652 | SEQ ID NO:20664 |

FIGURE 49

| | | | | | |
|---|---|---|---|---|---|
| **iPS:393094** | **21-225_34C4** | NA | AGAAGTAGTTACTACTGGGGC | AGTATCTATTATAGTGGGAGCACCGCCTACAATCCGTCCCTCAAGAGT | CTGAGCAGCAGCTGGTCTTTTGACTAC |
| | | | SEQ ID NO:4641 | SEQ ID NO:12653 | SEQ ID NO:20665 |
| | | AA | RSSYYWG | SIYYSGSTAYNPSLKS | LSSSWSFDY |
| | | | SEQ ID NO:4642 | SEQ ID NO:12654 | SEQ ID NO:20666 |
| **iPS:393096** | **21-225_34D11** | NA | AGCTATGCCATGAAC | GCTATTAGTGGTCGTGGTGGTAGCACATTCCACGCAGACTCCGTGAAGGGC | GGGGAGTTAGTAGAGGACTACTACTTCTACGGTATGGACGTC |
| | | | SEQ ID NO:4643 | SEQ ID NO:12655 | SEQ ID NO:20667 |
| | | AA | SYAMN | AISGRGGSTFHADSVKG | GELVEDYYFYGMDV |
| | | | SEQ ID NO:4644 | SEQ ID NO:12656 | SEQ ID NO:20668 |
| **iPS:393098** | **21-225_35G6** | NA | GACTACCATATACAC | TGGATCAACCCTAACAATGGTGGCACACACTATGCACAGGAGTTTCAGGGC | GATGGAACTGGGTCCTTTGACTAC |
| | | | SEQ ID NO:4645 | SEQ ID NO:12657 | SEQ ID NO:20669 |
| | | AA | DYHIH | WINPNNGGTHYAQEFQG | DGTGSFDY |
| | | | SEQ ID NO:4646 | SEQ ID NO:12658 | SEQ ID NO:20670 |
| **iPS:393100** | **21-225_36B8** | NA | AACTATGGCATGCAC | GTTATATGGCATGATGGAAGTAATAAATACTATGGAGACTCCGTGAAGGGC | GAAAATAGCAGCTCGTACTACTTTGACTAC |
| | | | SEQ ID NO:4647 | SEQ ID NO:12659 | SEQ ID NO:20671 |
| | | AA | NYGMH | VIWHDGSNKYYGDSVKG | ENSSSYYFDY |
| | | | SEQ ID NO:4648 | SEQ ID NO:12660 | SEQ ID NO:20672 |
| **iPS:393102** | **21 225 33F1** | NA | AGCTATGCCATGAGC | TCTATTAGTGGTCGTGGTGGTAGCACATTCCACGCAGACTCCGTGAAGGGC | GGGGAGCTACTTGAGGACTACTACTTCTACGGTATGGACGTC |

**FIGURE 49**

| | | | | | |
|---|---|---|---|---|---|
| | **21-225_33F1** | | SEQ ID NO:4649 | SEQ ID NO:12661 | SEQ ID NO:20673 |
| | | AA | SYAMS | SISGRGGSTFHADSVKG | GELLEDYYFYGMDV |
| | | | SEQ ID NO:4650 | SEQ ID NO:12662 | SEQ ID NO:20674 |
| **iPS:393104** | **21-225_33A7** | NA | AGCTGTGCCATGAGC | GCTATTAGTGGTCGTGGTGGTAGCACATTCCACGCAGACTCCGTGAAGGGC | GGGGAACTACTAGAGGACTACTACTTCTACGGTATGGACGTC |
| | | | SEQ ID NO:4651 | SEQ ID NO:12663 | SEQ ID NO:20675 |
| | | AA | SCAMS | AISGRGGSTFHADSVKG | GELLEDYYFYGMDV |
| | | | SEQ ID NO:4652 | SEQ ID NO:12664 | SEQ ID NO:20676 |
| **iPS:393106** | **21-225_34A6** | NA | AACTATGCCATGAAC | GCTATTAGTCGTCGTGGTGGTAGCACATTCTACGCAGACTCCGTGAAGGGC | GGGGAGCTACTAGAGGACTACTACTACTTCGCTATGGACGTC |
| | | | SEQ ID NO:4653 | SEQ ID NO:12665 | SEQ ID NO:20677 |
| | | AA | NYAMN | AISRRGGSTFYADSVKG | GELLEDYYYFAMDV |
| | | | SEQ ID NO:4654 | SEQ ID NO:12666 | SEQ ID NO:20678 |
| **iPS:393108** | **21-225_34G11** | NA | GGCTACTATATGCAC | TGGATCAACCCTAACAGTGGTGGCACAAACTATGCACAGAAGTTTCAGGGC | GATATTAGTAATTTCAGCAGCTGGTACGATTACTACGCTATGGACGTC |
| | | | SEQ ID NO:4655 | SEQ ID NO:12667 | SEQ ID NO:20679 |
| | | AA | GYYMH | WINPNSGGTNYAQKFQG | DISNFSSWYDYYAMDV |
| | | | SEQ ID NO:4656 | SEQ ID NO:12668 | SEQ ID NO:20680 |
| **iPS:393110** | **21-225_35B7** | NA | AGCTATGCCATGAGC | GCTATTAGTGGTCGTGGTGGTAGCACATTCCACGCAGACTCCGTGAAGGGC | GGGGAGCTACTAGAGGACTACTACTTCTACGGTATGGACGTC |
| | | | SEQ ID NO:4657 | SEQ ID NO:12669 | SEQ ID NO:20681 |
| | | AA | SYAMS | AISGRGGSTFHADSVKG | GELLEDYYFYGMDV |
| | | | SEQ ID NO:4658 | SEQ ID NO:12670 | SEQ ID NO:20682 |
| **iPS:393112** | **21 225 33G1** | NA | GGCTACTATATGCAC | TGGATCAGCCCTAACAATGGTGGCACAAACTATGCACAGAAGTTTCAGGGC | GATGGAACTGGGTCCTTTGACTAC |

FIGURE 49

| | | | | | |
|---|---|---|---|---|---|
| | 21-225_33G1 | | SEQ ID NO:4659 | SEQ ID NO:12671 | SEQ ID NO:20683 |
| | | AA | GYYMH | WISPNNGGTNYAQKFQG | DGTGSFDY |
| | | | SEQ ID NO:4660 | SEQ ID NO:12672 | SEQ ID NO:20684 |
| **iPS:393114** | **21-225_33G12** | NA | AGCTATGCCATGAGC | GTTATTAGTGGTAGTGGTGGTAGCTCATTCTACGCAGACTCCGTGAAGGGC | GATCGGGTGAGAGCTCATGATGGTTTTGATATC |
| | | | SEQ ID NO:4661 | SEQ ID NO:12673 | SEQ ID NO:20685 |
| | | AA | SYAMS | VISGSGGSSFYADSVKG | DRVRAHDGFDI |
| | | | SEQ ID NO:4662 | SEQ ID NO:12674 | SEQ ID NO:20686 |
| **iPS:393116** | **21-225_34G7** | NA | GACTACCATATTCAC | TGGATCAACCCTAACAATGGTGGCACACACTATGCACAGAAGTTTCAGGGC | GATGGAACTGGGTCCTTTGACTAC |
| | | | SEQ ID NO:4663 | SEQ ID NO:12675 | SEQ ID NO:20687 |
| | | AA | DYHIH | WINPNNGGTHYAQKFQG | DGTGSFDY |
| | | | SEQ ID NO:4664 | SEQ ID NO:12676 | SEQ ID NO:20688 |
| **iPS:393118** | **21-225_34H11** | NA | AGCTATGCCATGAAC | GCTATTAGTGGTCGTGGTGGTAGCACATTCTACGCAGACTCCGTGAAGGGC | GGGGAGCTACTAGAGGACTACTACTACTACGGTATGGACGTC |
| | | | SEQ ID NO:4665 | SEQ ID NO:12677 | SEQ ID NO:20689 |
| | | AA | SYAMN | AISGRGGSTFYADSVKG | GELLEDYYYYGMDV |
| | | | SEQ ID NO:4666 | SEQ ID NO:12678 | SEQ ID NO:20690 |
| **iPS:393120** | **21-225_35H8** | NA | AGTTATAGCATGAAC | TCCATTAGTGGTACTGGTAGTTTCATATACTACGCAGACTCAGTGAAGGGC | GTCTCTGGCTTTGACTAC |
| | | | SEQ ID NO:4667 | SEQ ID NO:12679 | SEQ ID NO:20691 |
| | | AA | SYSMN | SISGTGSFIYYADSVKG | VSGFDY |
| | | | SEQ ID NO:4668 | SEQ ID NO:12680 | SEQ ID NO:20692 |

FIGURE 49

| | | | | | |
|---|---|---|---|---|---|
| **iPS:393122** | **21-225_33B2** | NA | AACTATGGCATGCAC | GTTATATGGCATGATGG AAGTAATAAATACTATG CAGACTCCGTGAAGGGC | GAAAATAGCAGCTCGTACTA CTTTGACTAC |
| | | | SEQ ID NO:4669 | SEQ ID NO:12681 | SEQ ID NO:20693 |
| | | AA | NYGMH | VIWHDGSNKYYADSVKG | ENSSSYYFDY |
| | | | SEQ ID NO:4670 | SEQ ID NO:12682 | SEQ ID NO:20694 |
| **iPS:393124** | **21-225_33G7** | NA | AACTATGCCATGAGC | GCTATTAGTCGTCGTGGT GGTAGCACATTCTACGC AGACTCCGTGAAGGGC | GGGGAGCTACTAGAGGACTA CTCCTACTACGGTATGGACG TC |
| | | | SEQ ID NO:4671 | SEQ ID NO:12683 | SEQ ID NO:20695 |
| | | AA | NYAMS | AISRRGGSTFYADSVKG | GELLEDYSYYGMDV |
| | | | SEQ ID NO:4672 | SEQ ID NO:12684 | SEQ ID NO:20696 |
| **iPS:393126** | **21-225_35D1** | NA | AGCTATGCCATGAGC | GCTATTAGTGGTCGTGGT GGTAGCACATTCCACGC AGACTCCGTGAAGGGC | GGGGAGTTACTAGAGGACTA CTACTTCTACGGTATGGACG TC |
| | | | SEQ ID NO:4673 | SEQ ID NO:12685 | SEQ ID NO:20697 |
| | | AA | SYAMS | AISGRGGSTFHADSVKG | GELLEDYYFYGMDV |
| | | | SEQ ID NO:4674 | SEQ ID NO:12686 | SEQ ID NO:20698 |
| **iPS:393128** | **21-225_35F11** | NA | AGCTATGCCATGAGC | GCTATTAGTGGTCGTGGT GGTAGCACATTCCACGC AGACTCCATGAAGGGC | GGGGAGCTACTAGAGGACTA CTACTTCTACGGTATGGACG TC |
| | | | SEQ ID NO:4675 | SEQ ID NO:12687 | SEQ ID NO:20699 |
| | | AA | SYAMS | AISGRGGSTFHADSMKG | GELLEDYYFYGMDV |
| | | | SEQ ID NO:4676 | SEQ ID NO:12688 | SEQ ID NO:20700 |
| **iPS:393130** | **21-225_33C2** | NA | AGCTATACCATGAAC | TCCATTAGTGGTAGTAGT AGTTACATATACTACGC GGACTCAGTGAAGGGC | GATCGGGGGGGACC |
| | | | SEQ ID NO:4677 | SEQ ID NO:12689 | SEQ ID NO:20701 |

**FIGURE 49**

| | | | | | |
|---|---|---|---|---|---|
| | | AA | SYTMN | SISGSSSYIYYADSVKG | DRGGT |
| | | | SEQ ID NO:4678 | SEQ ID NO:12690 | SEQ ID NO:20702 |
| **iPS:393132** | **21-225_33H7** | NA | GACTACCATATACAC | TGGATCAACCCTAACAATGGTGGCACACACTATGCACAGGAGTTTCAGGGC | GATGGAACTGGGTCCTTTGACTAC |
| | | | SEQ ID NO:4679 | SEQ ID NO:12691 | SEQ ID NO:20703 |
| | | AA | DYHIH | WINPNNGGTHYAQEFQG | DGTGSFDY |
| | | | SEQ ID NO:4680 | SEQ ID NO:12692 | SEQ ID NO:20704 |
| **iPS:393134** | **21-225_34C2** | NA | AACTATGTCATGCAC | CTTATATGGTATGATGGAAGTAATAAATACTATGCAGACTCCGTGAAGGGC | GAAAATAGCAGCTCGTACTACTTTGACTAC |
| | | | SEQ ID NO:4681 | SEQ ID NO:12693 | SEQ ID NO:20705 |
| | | AA | NYVMH | LIWYDGSNKYYADSVKG | ENSSSYYFDY |
| | | | SEQ ID NO:4682 | SEQ ID NO:12694 | SEQ ID NO:20706 |
| **iPS:393136** | **21-225_34D8** | NA | AACTATGGCATGCAC | GTTATATGGTATGATGGAAGTAATAAATACTATGCAGACTCCGTGAAGGGC | GAAAATAGCAGCTCGTACTACTTTGACTAC |
| | | | SEQ ID NO:4683 | SEQ ID NO:12695 | SEQ ID NO:20707 |
| | | AA | NYGMH | VIWYDGSNKYYADSVKG | ENSSSYYFDY |
| | | | SEQ ID NO:4684 | SEQ ID NO:12696 | SEQ ID NO:20708 |
| **iPS:393138** | **21-225_35E3** | NA | AGCTATGGCATGCAC | GTTATATCATATGGTGGAAGTAATAAATACTATGCAGACTCCGTGAAGGGC | AGGGGGTATAGCAGTGGAGGCTACGGTATGGACGTC |
| | | | SEQ ID NO:4685 | SEQ ID NO:12697 | SEQ ID NO:20709 |

**FIGURE 49**

| | | | | | |
|---|---|---|---|---|---|
| | | AA | SYGMH | VISYGGSNKYYADSVKG | RGYSSGGYGMDV |
| | | | SEQ ID NO:4686 | SEQ ID NO:12698 | SEQ ID NO:20710 |
| **iPS:393140** | **21-225_35H12** | NA | GACTACTATATACAC | TGGATCAACCCTAATAGGGGTGGCACAAACTATGCACAGAAGTTTCAGGGC | GATGGAACTGGGTCCTTTGACTAC |
| | | | SEQ ID NO:4687 | SEQ ID NO:12699 | SEQ ID NO:20711 |
| | | AA | DYYIH | WINPNRGGTNYAQKFQG | DGTGSFDY |
| | | | SEQ ID NO:4688 | SEQ ID NO:12700 | SEQ ID NO:20712 |
| **iPS:393142** | **21-225_33A3** | NA | AGCTATGGCATGAAC | TCCATTAGTGGTAGTAGTACTTACATATACTACGCAGACTCAGTGAAGGGC | ACAAACGGTATGGACGTC |
| | | | SEQ ID NO:4689 | SEQ ID NO:12701 | SEQ ID NO:20713 |
| | | AA | SYGMN | SISGSSTYIYYADSVKG | TNGMDV |
| | | | SEQ ID NO:4690 | SEQ ID NO:12702 | SEQ ID NO:20714 |
| **iPS:393144** | **21-225_34D2** | NA | AATTATGACATTAAC | TGGCTGCACCCTAACAGTGGTACCACAGGCTTTGCACAGAAGTTCCGGGGC | AGCAGTGGCTGGTACTTTTTTGACTAC |
| | | | SEQ ID NO:4691 | SEQ ID NO:12703 | SEQ ID NO:20715 |
| | | AA | NYDIN | WLHPNSGTTGFAQKFRG | SSGWYFFDY |
| | | | SEQ ID NO:4692 | SEQ ID NO:12704 | SEQ ID NO:20716 |
| **iPS:393146** | **21-225_34G8** | NA | AGCTATGCCATGAGC | GCTATTAGTGGTCGTGGTGGTAGCACATTCCACGCAGACTCCGTGAAGGGC | GGGGAGCTACTAGAGGACTACTACTTCTACGGTATGGACGTC |
| | | | SEQ ID NO:4693 | SEQ ID NO:12705 | SEQ ID NO:20717 |
| | | AA | SYAMS | AISGRGGSTFHADSVKG | GELLEDYYFYGMDV |
| | | | SEQ ID NO:4694 | SEQ ID NO:12706 | SEQ ID NO:20718 |
| **iPS:393148** | **21-225_35E5** | NA | AGTTATGATATCAAC | TGGATGAACCCTAACAGTGGTAACACAGGCTATGCACAGAAGTTCCAGGGC | AAGAAGTCTAACGACTAC |
| | | | SEQ ID NO:4695 | SEQ ID NO:12707 | SEQ ID NO:20719 |

**FIGURE 49**

| | | | | | |
|---|---|---|---|---|---|
| | | AA | SYDIN | WMNPNSGNTGYAQKFQG | KKSNDY |
| | | | SEQ ID NO:4696 | SEQ ID NO:12708 | SEQ ID NO:20720 |
| **iPS:393150** | **21-225_36A5** | NA | AACTATGCCATGAAC | GCTATTAGTCGTCGTGGTGGTAACACATTCTACGCAGACTCCGTGAAGGGC | GGGGAGCTACTAGAGGACTACTACTACTACGCTATGGACGTC |
| | | | SEQ ID NO:4697 | SEQ ID NO:12709 | SEQ ID NO:20721 |
| | | AA | NYAMN | AISRRGGNTFYADSVKG | GELLEDYYYYAMDV |
| | | | SEQ ID NO:4698 | SEQ ID NO:12710 | SEQ ID NO:20722 |
| **iPS:393152** | **21-225_25B3** | NA | AGCTATGGCATGCAC | GTTATATGGTATGATGGAAGTAATAAATATTATGCAGACTCCGTGAAGGGC | GATTCCTCCCCCTACGGTATGGACGTC |
| | | | SEQ ID NO:4699 | SEQ ID NO:12711 | SEQ ID NO:20723 |
| | | AA | SYGMH | VIWYDGSNKYYADSVKG | DSSPYGMDV |
| | | | SEQ ID NO:4700 | SEQ ID NO:12712 | SEQ ID NO:20724 |
| **iPS:393166** | **21-225_27G6** | NA | GGCTATGGCATGCAC | ATTATATGGTATGATGGAAGTAAAAAATACAATGCAGACTCCGTGAAGGGC | GATAGGGTATATTGTAGTAGTACCAGCTGCTCCCCTTACTACTACTACTACGGTATGGACGTC |
| | | | SEQ ID NO:4701 | SEQ ID NO:12713 | SEQ ID NO:20725 |
| | | AA | GYGMH | IIWYDGSKKYNADSVKG | DRVYCSSTSCSPYYYYYGMDV |
| | | | SEQ ID NO:4702 | SEQ ID NO:12714 | SEQ ID NO:20726 |
| **iPS:393168** | **21-225_32B11** | NA | GGCTACTATATGCAC | TGGATCAACCCTAACAGTGATGGCACTAACTATGCACAGAAGTTTCAGGGC | GGGTTTTACTATGGTTCGGGGAGTTATTATAACGACCTCGACCCC |
| | | | SEQ ID NO:4703 | SEQ ID NO:12715 | SEQ ID NO:20727 |
| | | AA | GYYMH | WINPNSDGTNYAQKFQG | GFYYGSGSYYNDLDP |
| | | | SEQ ID NO:4704 | SEQ ID NO:12716 | SEQ ID NO:20728 |

# FIGURE 49

| | | | | | |
|---|---|---|---|---|---|
| **iPS:393172** | **21-225_3B12** | NA | TACTATGGCATGCAC | GTTATATCATATGATGGAAGTAATAAATACTATGCAGACTCCGTGAAGGGC | GATCGGAGGGGGGCTATGGAGTCCCCGATGCTTTTGATATC |
| | | | SEQ ID NO:4705 | SEQ ID NO:12717 | SEQ ID NO:20729 |
| | | AA | YYGMH | VISYDGSNKYYADSVKG | DRRGGYGVPDAFDI |
| | | | SEQ ID NO:4706 | SEQ ID NO:12718 | SEQ ID NO:20730 |
| **iPS:393174** | **21-225_15D8** | NA | GGCTATGGCATGCAC | GTTATATCATATGATGGAAGTAATAAATACTATGCAGACTCCGTGAAGGGC | GATCGGGTCTATTGTAGTAGTACCAGCTGCGTCCCTTACTACGACTACTACGGTATGGACGTC |
| | | | SEQ ID NO:4707 | SEQ ID NO:12719 | SEQ ID NO:20731 |
| | | AA | GYGMH | VISYDGSNKYYADSVKG | DRVYCSSTSCVPYYDYYGMDV |
| | | | SEQ ID NO:4708 | SEQ ID NO:12720 | SEQ ID NO:20732 |
| **iPS:393176** | **21-225_27E7** | NA | AGCTATGGCATGCAC | GTTATATGGTATGATGGAAGTAATAAATACTATGCAGACTCCGTGAAGGGC | GAGGATTCCTATTGTAGTAGTACCAGCTGCCCTTACTACTACTACTACGGTATGGACGTC |
| | | | SEQ ID NO:4709 | SEQ ID NO:12721 | SEQ ID NO:20733 |
| | | AA | SYGMH | VIWYDGSNKYYADSVKG | EDSYCSSTSCPYYYYYGMDV |
| | | | SEQ ID NO:4710 | SEQ ID NO:12722 | SEQ ID NO:20734 |
| **iPS:393178** | **21-225_34D7** | NA | GGCTACTATATGCAC | TGGATCAACCCTAACAGTGGTGGCACAAACTATGCACAGAAGTTTCAGGGC | GGGTATTACTATGGTTCGGGGAGTTATTATAACGACCTCGACCCC |
| | | | SEQ ID NO:4711 | SEQ ID NO:12723 | SEQ ID NO:20735 |
| | | AA | GYYMH | WINPNSGGTNYAQKFQG | GYYYGSGSYYNDLDP |
| | | | SEQ ID NO:4712 | SEQ ID NO:12724 | SEQ ID NO:20736 |

**FIGURE 49**

| | | | | | |
|---|---|---|---|---|---|
| **iPS:393180** | **21-225_4G12** | NA | AGCTATGCCATGAGC | ACTCTTAGTGGTCGTGGTGGTAGCACATACTACGCAGACTCCGTGAAGGGC | TGGGGACGTGGATACAGCTATGAGTACTACTACGGTATGGACGTC |
| | | | SEQ ID NO:4713 | SEQ ID NO:12725 | SEQ ID NO:20737 |
| | | AA | SYAMS | TLSGRGGSTYYADSVKG | WGRGYSYEYYYGMDV |
| | | | SEQ ID NO:4714 | SEQ ID NO:12726 | SEQ ID NO:20738 |
| **iPS:393182** | **21-225_4B3** | NA | GGCTACTATATGCAC | TGGATCAACCCTAACAGTGGTGGCACAAACTCTGCACAGAAGTTTCAGGGC | TCCTATTACTATGGTTCGGGGAGTTATTATAACGAGTTTGACTAC |
| | | | SEQ ID NO:4715 | SEQ ID NO:12727 | SEQ ID NO:20739 |
| | | AA | GYYMH | WINPNSGGTNSAQKFQG | SYYYGSGSYYNEFDY |
| | | | SEQ ID NO:4716 | SEQ ID NO:12728 | SEQ ID NO:20740 |
| **iPS:393184** | **21-225_15H11** | NA | ACTGGTGGAGTGGGTGTGGGC | CTCATTTATTGGCATGATGATAAGCGCTACAGTCCATCTCTGAGGAGC | ATAGTAGCAGTTGCCTTTGACTAC |
| | | | SEQ ID NO:4717 | SEQ ID NO:12729 | SEQ ID NO:20741 |
| | | AA | TGGVGVG | LIYWHDDKRYSPSLRS | IVAVAFDY |
| | | | SEQ ID NO:4718 | SEQ ID NO:12730 | SEQ ID NO:20742 |
| **iPS:393186** | **21-225_27D9** | NA | GGCTACTATATGCAC | TGGATCAACCCTAACAGTGGTGGCACAAAGTATGCACAGAAGTTTCAGGGC | GAGAGGTGTAGTACTACCAGTTGCTATTTAGGAATTACGGGCTACTACGGTATGGACGTC |
| | | | SEQ ID NO:4719 | SEQ ID NO:12731 | SEQ ID NO:20743 |
| | | AA | GYYMH | WINPNSGGTKYAQKFQG | ERCSTTSCYLGITGYYGMDV |
| | | | SEQ ID NO:4720 | SEQ ID NO:12732 | SEQ ID NO:20744 |
| **iPS:393188** | **21-225_34B9** | NA | ACTAGTGGAGTGGGTGTGGGC | CTCATTTATTGGAATGATGATAAGCGCTACAGCCCATCTCTGAAGAGC | TTAATAGCAGTGACTTTTGACTCC |
| | | | SEQ ID NO:4721 | SEQ ID NO:12733 | SEQ ID NO:20745 |
| | | AA | TSGVGVG | LIYWNDDKRYSPSLKS | LIAVTFDS |

**FIGURE 49**

| | | | | | |
|---|---|---|---|---|---|
| | | | SEQ ID NO:4722 | SEQ ID NO:12734 | SEQ ID NO:20746 |
| **iPS:393192** | **21-225_12B1** | NA | AGCTATGGCATGCAC | GTTATATGGAATGATGGAAGTAATAAATACTATGCAGACTCCGTGAAGGGC | GATCGGGTAGCAGCAGCTGGTACCCCCTACTACTACTACGGTATGGACGTC |
| | | | SEQ ID NO:4723 | SEQ ID NO:12735 | SEQ ID NO:20747 |
| | | AA | SYGMH | VIWNDGSNKYYADSVKG | DRVAAAGTPYYYYGMDV |
| | | | SEQ ID NO:4724 | SEQ ID NO:12736 | SEQ ID NO:20748 |
| **iPS:393194** | **21-225_16D2** | NA | AACGCCTGGATGAAC | CGTATTAAAAGCAAAACTGATGGTGGGACAACAGACTACGCTGCACCCGTGAAAGGC | GATACGGGTCCTATAGCAGCTCGTCTCGCTTACTACTACTACTACGCTATGGACGTC |
| | | | SEQ ID NO:4725 | SEQ ID NO:12737 | SEQ ID NO:20749 |
| | | AA | NAWMN | RIKSKTDGGTTDYAAPVKG | DTGPIAARLAYYYYYAMDV |
| | | | SEQ ID NO:4726 | SEQ ID NO:12738 | SEQ ID NO:20750 |
| **iPS:393196** | **21-225_16G8** | NA | AGCTATGCCATGAGC | GGTATTAGTGGTAGTGGTGGTAGCACATACTACGCAGACTCCGTGAAGGGC | GAATATTGTGGTGGTGACTGCTATTCCCCTTACTACTACTACTACGGTATGGACGTC |
| | | | SEQ ID NO:4727 | SEQ ID NO:12739 | SEQ ID NO:20751 |
| | | AA | SYAMS | GISGSGGSTYYADSVKG | EYCGGDCYSPYYYYYGMDV |
| | | | SEQ ID NO:4728 | SEQ ID NO:12740 | SEQ ID NO:20752 |
| **iPS:393198** | **21-225_28A11** | NA | GGCTATGGCCTGCAC | CTTATATGGTATGATGGAAATAATACATACTATGCAGACTCCGTGAAGGGC | GATAGGGTATATTGTAGTAGTACCAGCTGCTCCCCTTACTACTACTACTACGGTATGGACGTC |
| | | | SEQ ID NO:4729 | SEQ ID NO:12741 | SEQ ID NO:20753 |

**FIGURE 49**

| | | | | | |
|---|---|---|---|---|---|
| | | AA | GYGLH | LIWYDGNNTYYADSVKG | DRVYCSSTSCSPYYYYYGMDV |
| | | | SEQ ID NO:4730 | SEQ ID NO:12742 | SEQ ID NO:20754 |
| **iPS:393200** | **21-225_35E1** | NA | GGCTACTATATGCAC | TGGATCAACCCTAAAAGTGGTGGCACAAATTATGCACAGAAGTTTCAGGGC | GTGTATTACCATGGTTCGGGGAGTTATTATAACGAGTTTGATTAT |
| | | | SEQ ID NO:4731 | SEQ ID NO:12743 | SEQ ID NO:20755 |
| | | AA | GYYMH | WINPKSGGTNYAQKFQG | VYYHGSGSYYNEFDY |
| | | | SEQ ID NO:4732 | SEQ ID NO:12744 | SEQ ID NO:20756 |
| **iPS:393202** | **21-225_6B4** | NA | AGCTATGCCATGAGC | GCTATTAGTGGTAGTGGTAGTAGCACATACTACGCAGACTCCGTGAAGGGC | GAATATTGTGGTGGTGACTGCTATTCCCCTTACTACTACTACTACGGTATGGACGTC |
| | | | SEQ ID NO:4733 | SEQ ID NO:12745 | SEQ ID NO:20757 |
| | | AA | SYAMS | AISGSGSSTYYADSVKG | EYCGGDCYSPYYYYYGMDV |
| | | | SEQ ID NO:4734 | SEQ ID NO:12746 | SEQ ID NO:20758 |
| **iPS:393204** | **21-225_8C12** | NA | AGCTATGGCATGCAC | CTTATATGGTATGATGGAAGTAATAAATACTATGCAGACTCCGTGAAGGGC | GATCGGGTATCTTGTAGTAGTTCCAGCTGCTATCCTTACTACTACTACTACGGTATGGACGTC |
| | | | SEQ ID NO:4735 | SEQ ID NO:12747 | SEQ ID NO:20759 |
| | | AA | SYGMH | LIWYDGSNKYYADSVKG | DRVSCSSSSCYPYYYYYGMDV |
| | | | SEQ ID NO:4736 | SEQ ID NO:12748 | SEQ ID NO:20760 |
| **iPS:393206** | **21-225_13F6** | NA | GGCTACTATATGCAC | TGGATCAACCCTAACAGTGGTGGCGCAAACTATGCACAGAAGTTTCAGGGC | TCGTTTTACTATGGTTCGGGGACTTATTATAACGAGTTTGACTAC |
| | | | SEQ ID NO:4737 | SEQ ID NO:12749 | SEQ ID NO:20761 |
| | | AA | GYYMH | WINPNSGGANYAQKFQG | SFYYGSGTYYNEFDY |

**FIGURE 49**

| | | | | | |
|---|---|---|---|---|---|
| | | | SEQ ID NO:4738 | SEQ ID NO:12750 | SEQ ID NO:20762 |
| **iPS:393208** | **21-225_16F3** | NA | GGGCACTATATGCAC | TGGATCAACCCTAACAG TGGTGGCACAAACTATG CACAGAAGTTTCAGGGC | TCGTATTACTATGGTTCGGG GACTTATTATAACGAGTTTG ACTAC |
| | | | SEQ ID NO:4739 | SEQ ID NO:12751 | SEQ ID NO:20763 |
| | | AA | GHYMH | WINPNSGGTNYAQKFQG | SYYYGSGTYYNEFDY |
| | | | SEQ ID NO:4740 | SEQ ID NO:12752 | SEQ ID NO:20764 |
| **iPS:393210** | **21-225_17D3** | NA | GGCTACTATATGCAC | TGGATCAACCCTAACAG TGGTGGCACAAACTATG CACAGAAGTTTCAGGGC | GCGAATTACTATGGTTCGGG GAGTTATTATAACGACTTTG ACTAC |
| | | | SEQ ID NO:4741 | SEQ ID NO:12753 | SEQ ID NO:20765 |
| | | AA | GYYMH | WINPNSGGTNYAQKFQG | ANYYGSGSYYNDFDY |
| | | | SEQ ID NO:4742 | SEQ ID NO:12754 | SEQ ID NO:20766 |
| **iPS:393212** | **21-225_30H6** | NA | ACTGGTGGAGTGGGTGTGGG C | CTCATTTATTGGCATGAT GATAAGCGCTACAGTCC CTCTCTGAAGAGC | TTAATAGCAGTGGCTTTTGA CTAT |
| | | | SEQ ID NO:4743 | SEQ ID NO:12755 | SEQ ID NO:20767 |
| | | AA | TGGVGVG | LIYWHDDKRYSPSLKS | LIAVAFDY |
| | | | SEQ ID NO:4744 | SEQ ID NO:12756 | SEQ ID NO:20768 |
| **iPS:393214** | **21-225_33A1** | NA | GGCTACTATATGCAC | TGGATCAACCCTAACAA TGGTGGCACACACTATG CACAGAAGTTTCAGGGC | GGATATTATTATGCTTCGGG GAGTTATTATAACGACCTCG ACCCC |
| | | | SEQ ID NO:4745 | SEQ ID NO:12757 | SEQ ID NO:20769 |
| | | AA | GYYMH | WINPNNGGTHYAQKFQG | GYYYASGSYYNDLDP |
| | | | SEQ ID NO:4746 | SEQ ID NO:12758 | SEQ ID NO:20770 |
| **iPS:393218** | **21 225 14G3** | NA | GGCTACTATATGTAC | TGGATCAACCCTAACAG TGGTGGCACAAACTATG CACAGAAGTTTCAGGGC | TCGTATTTTTATGGTTCGGGG AGTTATTATAACGAGTTTGA CTAC |

FIGURE 49

| | | | | | |
|---|---|---|---|---|---|
| | **21-225_14G3** | | SEQ ID NO:4747 | SEQ ID NO:12759 | SEQ ID NO:20771 |
| | | AA | GYYMY | WINPNSGGTNYAQKFQG | SYFYGSGSYYNEFDY |
| | | | SEQ ID NO:4748 | SEQ ID NO:12760 | SEQ ID NO:20772 |
| **iPS:393222** | **21-225_17F5** | NA | ACTAGTGGAGTGGGTGTGGGC | CTCATTTATTGGGATGATGATAAGCGCTACAGCCCATCTCTGAAGAGC | ATTATAGCAGTGGCTTTTGACTAC |
| | | | SEQ ID NO:4749 | SEQ ID NO:12761 | SEQ ID NO:20773 |
| | | AA | TSGVGVG | LIYWDDDKRYSPSLKS | IIAVAFDY |
| | | | SEQ ID NO:4750 | SEQ ID NO:12762 | SEQ ID NO:20774 |
| **iPS:393224** | **21-225_31C2** | NA | ACTGGTGGAGTGGGTGTGGGC | CTCATTTATTGGAATGATGATGAGCGCTACAGCCCATCTCTGAAGAGC | TTAATAGCAGTTTCCTTTGACTAC |
| | | | SEQ ID NO:4751 | SEQ ID NO:12763 | SEQ ID NO:20775 |
| | | AA | TGGVGVG | LIYWNDDERYSPSLKS | LIAVSFDY |
| | | | SEQ ID NO:4752 | SEQ ID NO:12764 | SEQ ID NO:20776 |
| **iPS:393226** | **21-225_33E6** | NA | GGCTACTATATGCAC | TGGATCAACCCTAACAGTGGTGGCACAAACTATGCACAGAAGTTTCAGGGC | GTGTATTACTATGGTTCGGGGAGTTATTATAACGAGTTTGACTAC |
| | | | SEQ ID NO:4753 | SEQ ID NO:12765 | SEQ ID NO:20777 |
| | | AA | GYYMH | WINPNSGGTNYAQKFQG | VYYYGSGSYYNEFDY |
| | | | SEQ ID NO:4754 | SEQ ID NO:12766 | SEQ ID NO:20778 |
| **iPS:393230** | **21-225_9G9** | NA | GGCTACTATATACAC | TGGATCAACCCTAACAGTGGTGGCACAGACTATGCACAGAAGTTTCAGGGC | TCGTATTACTATGGTTCGGGGACTTATTATAACGAGTTTGACTAC |
| | | | SEQ ID NO:4755 | SEQ ID NO:12767 | SEQ ID NO:20779 |
| | | AA | GYYIH | WINPNSGGTDYAQKFQG | SYYYGSGTYYNEFDY |
| | | | SEQ ID NO:4756 | SEQ ID NO:12768 | SEQ ID NO:20780 |
| **iPS:393232** | **21 225 17F12** | NA | AGCTATGCCATGAGC | GCTATTAGTGGTGGTGGTGGTAGCACATACTACGCAGACTCCGTGAAGGGC | TGGGGACGTGGATACAACTATGAGTACTACTACGGTATGGACGTC |

FIGURE 49

| | | | | | |
|---|---|---|---|---|---|
| | 21-225_17F12 | | SEQ ID NO:4757 | SEQ ID NO:12769 | SEQ ID NO:20781 |
| | | AA | SYAMS | AISGGGGSTYYADSVKG | WGRGYNYEYYYGMDV |
| | | | SEQ ID NO:4758 | SEQ ID NO:12770 | SEQ ID NO:20782 |
| **iPS:393234** | 21-225_26C10 | NA | GGCTACTATGTGCAC | TGGATCAACCCTAACAG TGGTGGCACAAACTATG CACAGAAGTTTCAGGGC | GAGAGGTGTAGTACTACCAG CTGCTATTTAGGAATTACGG GCTACTACGGTATGGACGTC |
| | | | SEQ ID NO:4759 | SEQ ID NO:12771 | SEQ ID NO:20783 |
| | | AA | GYYVH | WINPNSGGTNYAQKFQG | ERCSTTSCYLGITGYYGMDV |
| | | | SEQ ID NO:4760 | SEQ ID NO:12772 | SEQ ID NO:20784 |
| **iPS:393345** | 21-225_5G7 | NA | AGCTATGCCATGAGC | GCTATTAGTGGTAGTGGT GGTAGCACATACTACGC AGACTCCGTGAAGGGC | GAATATTGTGGTGGTGACTG CTATTCCCCTTACTACTACTA CTACGGTATGGACGTC |
| | | | SEQ ID NO:4761 | SEQ ID NO:12773 | SEQ ID NO:20785 |
| | | AA | SYAMS | AISGSGGSTYYADSVKG | EYCGGDCYSPYYYYYGMDV |
| | | | SEQ ID NO:4762 | SEQ ID NO:12774 | SEQ ID NO:20786 |
| **iPS:393368** | 21-225_29H8 | NA | AATTATGATATCAAC | TGGATGAACCCTAACAG TGGTAACACAGGCTATG CACAGAGGTTCCAGGGC | AGCAGTGGCTGGTACTACTT TGACTAC |
| | | | SEQ ID NO:4763 | SEQ ID NO:12775 | SEQ ID NO:20787 |
| | | AA | NYDIN | WMNPNSGNTGYAQRFQG | SSGWYYFDY |
| | | | SEQ ID NO:4764 | SEQ ID NO:12776 | SEQ ID NO:20788 |
| **iPS:393565** | 21-225_34B11 | NA | GGCTACTATATGCAC | TGGATCAACCCTAACAG TGGTGGCACAAACTATG CACAGAAGTTTCAGGGC | GTGTATTTCTATGGTTCGGG GAGTTATTATAACGAGTTTG ACTAC |
| | | | SEQ ID NO:4765 | SEQ ID NO:12777 | SEQ ID NO:20789 |
| | | AA | GYYMH | WINPNSGGTNYAQKFQG | VYFYGSGSYYNEFDY |
| | | | SEQ ID NO:4766 | SEQ ID NO:12778 | SEQ ID NO:20790 |

FIGURE 49

| | | | | | |
|---|---|---|---|---|---|
| **iPS:393802** | **21-225_3D12** | NA | ACCGCCTGGATGAAC | CGGATTAAAAACAAAATTGATGGTGGGACAACAGACTACGTTGCACCCGTGAAAGGC | GAAGGCTGGAACACGGACTAC |
| | | | SEQ ID NO:4767 | SEQ ID NO:12779 | SEQ ID NO:20791 |
| | | AA | TAWMN | RIKNKIDGGTTDYVAPVKG | EGWNTDY |
| | | | SEQ ID NO:4768 | SEQ ID NO:12780 | SEQ ID NO:20792 |
| **iPS:393804** | **21-225_5H7** | NA | AGAAGTAGTTATTACTGGGGC | AATATCTATTATAGTGGGACCACCTACTACAACCCGTCCCTCAAGAGT | CATGGAAAAGACTGGGGCCTTGACTAC |
| | | | SEQ ID NO:4769 | SEQ ID NO:12781 | SEQ ID NO:20793 |
| | | AA | RSSYYWG | NIYYSGTTYYNPSLKS | HGKDWGLDY |
| | | | SEQ ID NO:4770 | SEQ ID NO:12782 | SEQ ID NO:20794 |
| **iPS:393806** | **21-225_6A6** | NA | AGAAGTAGTTACTACTGGGGC | AATATCTATTATAGTGGGATCCCCTACTACAACCCGTCCCTCAAGAGT | CACAGCAGCAGCTGGTCTCTTGACTAC |
| | | | SEQ ID NO:4771 | SEQ ID NO:12783 | SEQ ID NO:20795 |
| | | AA | RSSYYWG | NIYYSGIPYYNPSLKS | HSSSWSLDY |
| | | | SEQ ID NO:4772 | SEQ ID NO:12784 | SEQ ID NO:20796 |
| **iPS:393808** | **21-225_1A2** | NA | AGCTATACCATGAAC | TCCATTAGTGGTAGTGGTAGTTACATATACTACGCAGACTCAGTGAAGGGC | GGGAGCAGCTCGTCCGGGTTTGACTAC |
| | | | SEQ ID NO:4773 | SEQ ID NO:12785 | SEQ ID NO:20797 |
| | | AA | SYTMN | SISGSGSYIYYADSVKG | GSSSSGFDY |
| | | | SEQ ID NO:4774 | SEQ ID NO:12786 | SEQ ID NO:20798 |
| **iPS:393810** | **21-225_5A4** | NA | AGCTCTGCCATGAGC | GCTATTAGTGGTCGTGGTGGTAACACATTCTACACAGACTCCGTGAAGGGC | CTGGGGAAAGACTACTACTACTACGGTATGGACGTC |
| | | | SEQ ID NO:4775 | SEQ ID NO:12787 | SEQ ID NO:20799 |

FIGURE 49

| | | | | | |
|---|---|---|---|---|---|
| | | AA | SSAMS | AISGRGGNTFYTDSVKG | LGKDYYYYGMDV |
| | | | SEQ ID NO:4776 | SEQ ID NO:12788 | SEQ ID NO:20800 |
| **iPS:393812** | **21-225_6A11** | NA | GACTATGGCATGCAC | GTTATATGGTTTGATGGAAGTAATAAATACTATGCAGACTCCGTGAAGGGC | GATCTTGGCTGGACGGAAGAGTAC |
| | | | SEQ ID NO:4777 | SEQ ID NO:12789 | SEQ ID NO:20801 |
| | | AA | DYGMH | VIWFDGSNKYYADSVKG | DLGWTEEY |
| | | | SEQ ID NO:4778 | SEQ ID NO:12790 | SEQ ID NO:20802 |
| **iPS:393814** | **21-225_7F4** | NA | AGGAGTAGTTACTACTGGGGC | AATATCTATTATAGTGGGGCCACCTACTACAACCCGTCCCTCAAGAGT | CATAGCGGCAGCTGGTCCCTTGACTAC |
| | | | SEQ ID NO:4779 | SEQ ID NO:12791 | SEQ ID NO:20803 |
| | | AA | RSSYYWG | NIYYSGATYYNPSLKS | HSGSWSLDY |
| | | | SEQ ID NO:4780 | SEQ ID NO:12792 | SEQ ID NO:20804 |
| **iPS:393816** | **21-225_6D4** | NA | AGAAGTAGTTCCTACTGGGGC | AATATCTATTATAGTGGGAGCGCCTACTACATTCCGTCCCTCAAGAGT | CACAGCAGCAGCTGGTCTCTTGACTGC |
| | | | SEQ ID NO:4781 | SEQ ID NO:12793 | SEQ ID NO:20805 |
| | | AA | RSSSYWG | NIYYSGSAYYIPSLKS | HSSSWSLDC |
| | | | SEQ ID NO:4782 | SEQ ID NO:12794 | SEQ ID NO:20806 |
| **iPS:393818** | **21-225_6G12** | NA | AGCTATGGCATGCAC | GTTATTTGGTATGATAGAAGTAATAACTACTATGCAGACTCCGTGAAGGGC | GAACTGGGGTTCCGGTCTGACTAC |
| | | | SEQ ID NO:4783 | SEQ ID NO:12795 | SEQ ID NO:20807 |
| | | AA | SYGMH | VIWYDRSNNYYADSVKG | ELGFRSDY |
| | | | SEQ ID NO:4784 | SEQ ID NO:12796 | SEQ ID NO:20808 |

FIGURE 49

| | | | | | |
|---|---|---|---|---|---|
| **iPS:393820** | **21-225_8H7** | NA | GACTTTGGCATGCAC | GTTATTTGGTATGAAGAAAATAATCAATACTATGCAGACTCCGTGAAGGGC | GAGCTGGGGTTCCGGTCTGACTAC |
| | | | SEQ ID NO:4785 | SEQ ID NO:12797 | SEQ ID NO:20809 |
| | | AA | DFGMH | VIWYEENNQYYADSVKG | ELGFRSDY |
| | | | SEQ ID NO:4786 | SEQ ID NO:12798 | SEQ ID NO:20810 |
| **iPS:393822** | **21-225_15B11** | NA | AACTATGGCATGCAC | GTTATATGGTATGAGGAAAGTAATAAATACTATACAGACTCCGTGAAGGGC | GAAGTGGGATTCACTGAGGACTAC |
| | | | SEQ ID NO:4787 | SEQ ID NO:12799 | SEQ ID NO:20811 |
| | | AA | NYGMH | VIWYEESNKYYTDSVKG | EVGFTEDY |
| | | | SEQ ID NO:4788 | SEQ ID NO:12800 | SEQ ID NO:20812 |
| **iPS:393824** | **21-225_10F12** | NA | AGCTATGCCATGAGC | ATTATTAGTGGTCGTGGTGGTAACACATTCTACGCAGACTCCGTGAAGGGC | CGTGTAGCAGTGGCTGGCTCGGAGGCTTTTGCTATC |
| | | | SEQ ID NO:4789 | SEQ ID NO:12801 | SEQ ID NO:20813 |
| | | AA | SYAMS | IISGRGGNTFYADSVKG | RVAVAGSEAFAI |
| | | | SEQ ID NO:4790 | SEQ ID NO:12802 | SEQ ID NO:20814 |
| **iPS:393826** | **21-225_10G5** | NA | AACTATGGCATGCAC | GTTATTTGGTATGAAGAAAATAATCAATACTATGCAGACTCCGTGAAGGGC | GAACTGGGGTTCCGGTCTGACTAC |
| | | | SEQ ID NO:4791 | SEQ ID NO:12803 | SEQ ID NO:20815 |
| | | AA | NYGMH | VIWYEENNQYYADSVKG | ELGFRSDY |
| | | | SEQ ID NO:4792 | SEQ ID NO:12804 | SEQ ID NO:20816 |

FIGURE 49

| | | | | | |
|---|---|---|---|---|---|
| **iPS:393828** | **21-225_10H12** | NA | GACTATGGCATGCAC | GTTATTTGGTATGAAGACAATAATCAATACTATGCAGACTCCGTGAAGGGC | GAACTGGGGTTCCGGTCTGACTAC |
| | | | SEQ ID NO:4793 | SEQ ID NO:12805 | SEQ ID NO:20817 |
| | | AA | DYGMH | VIWYEDNNQYYADSVKG | ELGFRSDY |
| | | | SEQ ID NO:4794 | SEQ ID NO:12806 | SEQ ID NO:20818 |
| **iPS:393830** | **21-225_12A1** | NA | AGCTATGGCATGCAC | GTTATTTGGTATGAAGAAAATAATCAATACTATGCAGACTCCGTGAAGGGC | GAACTGGGGTTCCGGTCTGACTAC |
| | | | SEQ ID NO:4795 | SEQ ID NO:12807 | SEQ ID NO:20819 |
| | | AA | SYGMH | VIWYEENNQYYADSVKG | ELGFRSDY |
| | | | SEQ ID NO:4796 | SEQ ID NO:12808 | SEQ ID NO:20820 |
| **iPS:393832** | **21-225_14B2** | NA | AGAAGTAGTTATTACTGGGGT | AATATCTATTATAGTGGGACCACCTACTACAACCCGTCCCTCAAGAGT | CATGGAAAAGACTGGGGCCTTGACTAC |
| | | | SEQ ID NO:4797 | SEQ ID NO:12809 | SEQ ID NO:20821 |
| | | AA | RSSYYWG | NIYYSGTTYYNPSLKS | HGKDWGLDY |
| | | | SEQ ID NO:4798 | SEQ ID NO:12810 | SEQ ID NO:20822 |
| **iPS:393836** | **21-225_15A2** | NA | AGCTATACCATGAAC | TCCATTAGTGGTAGTGGTAGTTACATATACTACGCAGACTCAGTGAAGGGC | GTGGCTTCATTTGACTAC |
| | | | SEQ ID NO:4799 | SEQ ID NO:12811 | SEQ ID NO:20823 |
| | | AA | SYTMN | SISGSGSYIYYADSVKG | VASFDY |
| | | | SEQ ID NO:4800 | SEQ ID NO:12812 | SEQ ID NO:20824 |

**FIGURE 49**

| | | | | | |
|---|---|---|---|---|---|
| **iPS:393838** | **21-225_6G2** | NA | GACTATGGCATACAC | GTCATTTGGTTTGATGGAAGTAATAAATACTATGCAGACTCCGTGAAGGGC | GATCTTGGCTGGACGGAAGAGTAC |
| | | | SEQ ID NO:4801 | SEQ ID NO:12813 | SEQ ID NO:20825 |
| | | AA | DYGIH | VIWFDGSNKYYADSVKG | DLGWTEEY |
| | | | SEQ ID NO:4802 | SEQ ID NO:12814 | SEQ ID NO:20826 |
| **iPS:393840** | **21-225_3F8** | NA | AGCTATGGCATGCAC | GTTATATGGCATGATGGAAGTAATAAATACTATGCAGACTCCGTGAAGGGC | GATCTGAGTATGGGCGGTATGGACGTC |
| | | | SEQ ID NO:4803 | SEQ ID NO:12815 | SEQ ID NO:20827 |
| | | AA | SYGMH | VIWHDGSNKYYADSVKG | DLSMGGMDV |
| | | | SEQ ID NO:4804 | SEQ ID NO:12816 | SEQ ID NO:20828 |
| **iPS:393844** | **21-225_3G7** | NA | AACTATGGCATGCAC | GTCATATGGCATGATGGAAGTAATAAATACTATGTAGACTCCGTGAAGGGC | GATGAGAGGCTGGGGATTTTTGACTAC |
| | | | SEQ ID NO:4805 | SEQ ID NO:12817 | SEQ ID NO:20829 |
| | | AA | NYGMH | VIWHDGSNKYYVDSVKG | DERLGIFDY |
| | | | SEQ ID NO:4806 | SEQ ID NO:12818 | SEQ ID NO:20830 |
| **iPS:393848** | **21-225_4H2** | NA | GGCTATGCCATGAAC | GTTATTAGTCGTAGTGGTGGTTACACATACTACGCGGACTCCGTGAAGGGC | CGTTTAGCAGTGGCTGGCTCGGAGGCTTTTGATATC |
| | | | SEQ ID NO:4807 | SEQ ID NO:12819 | SEQ ID NO:20831 |
| | | AA | GYAMN | VISRSGGYTYYADSVKG | RLAVAGSEAFDI |
| | | | SEQ ID NO:4808 | SEQ ID NO:12820 | SEQ ID NO:20832 |

**FIGURE 49**

| | | | | | |
|---|---|---|---|---|---|
| **iPS:393852** | **21-225_12A10** | NA | AGCTATGGCATGCAC | GTTATATGGCATGATGAAAGTAATAAATACTATACAGACTCCGTGAAGGGC | GACGAGAGGCTGGGGATTTTTGACTAC |
| | | | SEQ ID NO:4809 | SEQ ID NO:12821 | SEQ ID NO:20833 |
| | | AA | SYGMH | VIWHDESNKYYTDSVKG | DERLGIFDY |
| | | | SEQ ID NO:4810 | SEQ ID NO:12822 | SEQ ID NO:20834 |
| **iPS:393854** | **21-225_7H11** | NA | GACTATGGCATGCAC | GTTATATGGTATGATGAAAATAATAAATACTATGCAGACTCCGTGAAGGGC | GAACTGGGGTTCCGGTCTGACTAC |
| | | | SEQ ID NO:4811 | SEQ ID NO:12823 | SEQ ID NO:20835 |
| | | AA | DYGMH | VIWYDENNKYYADSVKG | ELGFRSDY |
| | | | SEQ ID NO:4812 | SEQ ID NO:12824 | SEQ ID NO:20836 |
| **iPS:393856** | **21-225_14C2** | NA | GACTATGGCATGCAC | GTTATATGGTATGACGAAAGTAATAAATACTATGAAGACTCCGTGAAGGGC | GAAGTGGGATTCCGGTCTGACTAC |
| | | | SEQ ID NO:4813 | SEQ ID NO:12825 | SEQ ID NO:20837 |
| | | AA | DYGMH | VIWYDESNKYYEDSVKG | EVGFRSDY |
| | | | SEQ ID NO:4814 | SEQ ID NO:12826 | SEQ ID NO:20838 |
| **iPS:393862** | **21-225_5G2** | NA | AGCTATGCCATGAGC | GTTATTAGTGGTCGTGGTGTTAACACATTCTACGCAGACTCCGTGAAGGGC | CGTATAGCAGTGGCTGGCTCGGAGGCTTTTGATATC |
| | | | SEQ ID NO:4815 | SEQ ID NO:12827 | SEQ ID NO:20839 |
| | | AA | SYAMS | VISGRGVNTFYADSVKG | RIAVAGSEAFDI |
| | | | SEQ ID NO:4816 | SEQ ID NO:12828 | SEQ ID NO:20840 |

# FIGURE 49

| | | | | | |
|---|---|---|---|---|---|
| **iPS:393864** | **21-225_4C5** | NA | AGCTATGTCCTGCAC | GTTATATGGTATGATGGAAGTAATAAATACTATGCAGACTCCGTGAAGGGC | GAAAAGTATACCAGCAGCTGGTACGACTACGGTATGGACGTC |
| | | | SEQ ID NO:4817 | SEQ ID NO:12829 | SEQ ID NO:20841 |
| | | AA | SYVLH | VIWYDGSNKYYADSVKG | EKYTSSWYDYGMDV |
| | | | SEQ ID NO:4818 | SEQ ID NO:12830 | SEQ ID NO:20842 |
| **iPS:393866** | **21-225_14E3** | NA | GACTTTGGCATGCAC | GTTATTTGGTATGAAGAAAATAATCAATACTATGCAGACTCCGTGAAGGGC | GAGCTGGGGTTCCGGTCTGACTAC |
| | | | SEQ ID NO:4819 | SEQ ID NO:12831 | SEQ ID NO:20843 |
| | | AA | DFGMH | VIWYEENNQYYADSVKG | ELGFRSDY |
| | | | SEQ ID NO:4820 | SEQ ID NO:12832 | SEQ ID NO:20844 |
| **iPS:393868** | **21-225_9C11** | NA | AGCTATGGCATGCAC | GTTATATGGCATGATGAAACTAATAAATACTATGCAGATTCCGTGAAGGGC | GACGAGAGGCTGGGGATTTTTGACTAC |
| | | | SEQ ID NO:4821 | SEQ ID NO:12833 | SEQ ID NO:20845 |
| | | AA | SYGMH | VIWHDETNKYYADSVKG | DERLGIFDY |
| | | | SEQ ID NO:4822 | SEQ ID NO:12834 | SEQ ID NO:20846 |
| **iPS:393870** | **21-225_7B1** | NA | AGCTATGACATGAGC | ACTATTAGTGGTAGTGGTGGTATCACATACTACGCAGACTCCGTGAAGGGC | GATCGGGGCAGCGTC |
| | | | SEQ ID NO:4823 | SEQ ID NO:12835 | SEQ ID NO:20847 |
| | | AA | SYDMS | TISGSGGITYYADSVKG | DRGSV |
| | | | SEQ ID NO:4824 | SEQ ID NO:12836 | SEQ ID NO:20848 |

FIGURE 49

| | | | | | |
|---|---|---|---|---|---|
| **iPS:393872** | **21-225_2A11** | NA | AGGAGTAGTTACTACTGGGGC | AATATCTATTATAGTGGGAGCACCTACTACAACCCGTCCGTCAAGAGT | CATGGAAAAGACTGGGGCCTTGAAGAC |
| | | | SEQ ID NO:4825 | SEQ ID NO:12837 | SEQ ID NO:20849 |
| | | AA | RSSYYWG | NIYYSGSTYYNPSVKS | HGKDWGLED |
| | | | SEQ ID NO:4826 | SEQ ID NO:12838 | SEQ ID NO:20850 |
| **iPS:393874** | **21-225_4C8** | NA | AACTATGGCATGCAC | GTTATTTGGTATGAAGAAAATAATCAATACTATGCAGACTCCGTGAAGGGC | GAAATGGGGTTCCTGTCTGACTAC |
| | | | SEQ ID NO:4827 | SEQ ID NO:12839 | SEQ ID NO:20851 |
| | | AA | NYGMH | VIWYEENNQYYADSVKG | EMGFLSDY |
| | | | SEQ ID NO:4828 | SEQ ID NO:12840 | SEQ ID NO:20852 |
| **iPS:393876** | **21-225_9A1** | NA | GACTATGGCATGCAC | GTTATATGGTTTGATGGAAGTAATAAATACTATGCAGACTCCGTGAAGGGC | GATCTTGGCTGGACGGAAGAGTAC |
| | | | SEQ ID NO:4829 | SEQ ID NO:12841 | SEQ ID NO:20853 |
| | | AA | DYGMH | VIWFDGSNKYYADSVKG | DLGWTEEY |
| | | | SEQ ID NO:4830 | SEQ ID NO:12842 | SEQ ID NO:20854 |
| **iPS:393878** | **21-225_7G12** | NA | AGCTATGCCATGAAC | GTTATTAGTGGTAGTGGTGGTAGCACATACTACGCAGACTCCGTGAAGGGC | AGGTATACCAGTGACTGGCATGATGCTTTTGATATC |
| | | | SEQ ID NO:4831 | SEQ ID NO:12843 | SEQ ID NO:20855 |
| | | AA | SYAMN | VISGSGGSTYYADSVKG | RYTSDWHDAFDI |
| | | | SEQ ID NO:4832 | SEQ ID NO:12844 | SEQ ID NO:20856 |
| **iPS:393880** | **21 225 15A1** | NA | AGAAGTAGTTACTACTGGGGC | AGTATCTATTATAGTGGGAGCGCCCAGTACAACCCGTCCCTCAAGAGT | CTGAGCAGCAGCTGGTCTTTTGACTAC |

FIGURE 49

| | | | | | |
|---|---|---|---|---|---|
| | **21-225_15A1** | | SEQ ID NO:4833 | SEQ ID NO:12845 | SEQ ID NO:20857 |
| | | AA | RSSYYWG | SIYYSGSAQYNPSLKS | LSSSWSFDY |
| | | | SEQ ID NO:4834 | SEQ ID NO:12846 | SEQ ID NO:20858 |
| **iPS:393882** | **21-225_15E3** | NA | AGCTATGGCATGCAC | GTTATATGGTATGAGGAAAATAATAAACACTATGCAGACTCCGTGAAGGGC | GAGCTGGGGTTCCTCTCTGACTAC |
| | | | SEQ ID NO:4835 | SEQ ID NO:12847 | SEQ ID NO:20859 |
| | | AA | SYGMH | VIWYEENNKHYADSVKG | ELGFLSDY |
| | | | SEQ ID NO:4836 | SEQ ID NO:12848 | SEQ ID NO:20860 |
| **iPS:393884** | **21-225_16F4** | NA | AACTATGGCATGCAC | GTTATATGGTATGAAGGAAGTAATCAATACTATGGAGACTCCGTGAAGGGC | GAGCTGGGGTTCCTCTCTGACTAC |
| | | | SEQ ID NO:4837 | SEQ ID NO:12849 | SEQ ID NO:20861 |
| | | AA | NYGMH | VIWYEGSNQYYGDSVKG | ELGFLSDY |
| | | | SEQ ID NO:4838 | SEQ ID NO:12850 | SEQ ID NO:20862 |
| **iPS:393886** | **21-225_2G9** | NA | CCTAATTACTACTGGGGC | AGTATCTATTATAGTGGAAGCACCTCCTACAACCCGTCCCTCAACAGT | CTAAGCAGCAACTGGGACTTTGACAAC |
| | | | SEQ ID NO:4839 | SEQ ID NO:12851 | SEQ ID NO:20863 |
| | | AA | PNYYWG | SIYYSGSTSYNPSLNS | LSSNWDFDN |
| | | | SEQ ID NO:4840 | SEQ ID NO:12852 | SEQ ID NO:20864 |
| **iPS:393888** | **21-225_3E3** | NA | AGCTATGTCATGAGC | ATTATTAGTGGTCGTGGTGGTAACACATTCTACGCAGACTCCGTGAAGGGC | CGTTTAGCAGTGGCTGGCTCGGAGGCTTTTGATATC |
| | | | SEQ ID NO:4841 | SEQ ID NO:12853 | SEQ ID NO:20865 |
| | | AA | SYVMS | IISGRGGNTFYADSVKG | RLAVAGSEAFDI |
| | | | SEQ ID NO:4842 | SEQ ID NO:12854 | SEQ ID NO:20866 |

FIGURE 49

| | | | | | |
|---|---|---|---|---|---|
| **iPS:393890** | **21-225_4B1** | NA | AGTTACTCCTGGAGC | CGTATCTATACCAGTGGGAGCACCAACTACATCCCCTCCCTCAAGAGT | GATTTGAAGAGCAGTGGCTGCCTTTTCTTTGACTAC |
| | | | SEQ ID NO:4843 | SEQ ID NO:12855 | SEQ ID NO:20867 |
| | | AA | SYSWS | RIYTSGSTNYIPSLKS | DLKSSGCLFFDY |
| | | | SEQ ID NO:4844 | SEQ ID NO:12856 | SEQ ID NO:20868 |
| **iPS:393892** | **21-225_6G7** | NA | AGCTATGGCATGCAC | ATTATATCATATGTTGGAAAGAATAAATATTATGCAGACTCCGTGAAGGGC | CGGGGAAACAGCTATGGCGGGTACGGTATGGACGTC |
| | | | SEQ ID NO:4845 | SEQ ID NO:12857 | SEQ ID NO:20869 |
| | | AA | SYGMH | IISYVGKNKYYADSVKG | RGNSYGGYGMDV |
| | | | SEQ ID NO:4846 | SEQ ID NO:12858 | SEQ ID NO:20870 |
| **iPS:393894** | **21-225_5E11** | NA | AGCTATAGCATGAAC | TCCATTAGTGGTAGTAGTACTTACATATACTACGCAGACTCAGTGAAGGGC | GTGGCTTCATTTGACTAC |
| | | | SEQ ID NO:4847 | SEQ ID NO:12859 | SEQ ID NO:20871 |
| | | AA | SYSMN | SISGSSTYIYYADSVKG | VASFDY |
| | | | SEQ ID NO:4848 | SEQ ID NO:12860 | SEQ ID NO:20872 |
| **iPS:393896** | **21-225_2A4** | NA | AGCTATAGCATGAAC | TCCATTAGTGGTAGTAGTACTTACATATACTACGCAGACTCAGTGAAGGGC | GTGGCTTCATTTGACTAC |
| | | | SEQ ID NO:4849 | SEQ ID NO:12861 | SEQ ID NO:20873 |
| | | AA | SYSMN | SISGSSTYIYYADSVKG | VASFDY |
| | | | SEQ ID NO:4850 | SEQ ID NO:12862 | SEQ ID NO:20874 |
| **iPS:393898** | **21-225_5F7** | NA | AGCTATGCCATGAGC | ATTATTAGTGGTCGTGGTGGTAACACATTCTACGCAGACTCCGTGAAGGGC | CGTATAGCAGTGGCTGGCTCGGAGGCTTTTGCTATC |
| | | | SEQ ID NO:4851 | SEQ ID NO:12863 | SEQ ID NO:20875 |
| | | AA | SYAMS | IISGRGGNTFYADSVKG | RIAVAGSEAFAI |

FIGURE 49

| | | | | | |
|---|---|---|---|---|---|
| | | | SEQ ID NO:4852 | SEQ ID NO:12864 | SEQ ID NO:20876 |
| **iPS:393900** | **21-225_10E12** | NA | AACTATGGCATGCAC | GTTATATGGCATGATGGAAGTAATAAATACTATGTAGACTCCGTGAAGGGC | GATGAGAGGCTGGGGATTTTTGACTAC |
| | | | SEQ ID NO:4853 | SEQ ID NO:12865 | SEQ ID NO:20877 |
| | | AA | NYGMH | VIWHDGSNKYYVDSVKG | DERLGIFDY |
| | | | SEQ ID NO:4854 | SEQ ID NO:12866 | SEQ ID NO:20878 |
| **iPS:393902** | **21-225_14E10** | NA | GACTATGGCATGCAC | GTTATATGGTATGATGGAAGTAATAAATACTATGCAGACTCCGTGAAGGGC | GAGAAGTATAGCAGCAGCTGGTACGACTACGGTATGGACGTC |
| | | | SEQ ID NO:4855 | SEQ ID NO:12867 | SEQ ID NO:20879 |
| | | AA | DYGMH | VIWYDGSNKYYADSVKG | EKYSSSWYDYGMDV |
| | | | SEQ ID NO:4856 | SEQ ID NO:12868 | SEQ ID NO:20880 |
| **iPS:393904** | **21-225_8H11** | NA | ACCTATAACATGCAC | GTTATATGGTATGATGGAAGTGATAGATACTCTGCAGACTCCGTGAAGGGC | GATCGGGCCTATAGCAGCTCGTCTGACTTC |
| | | | SEQ ID NO:4857 | SEQ ID NO:12869 | SEQ ID NO:20881 |
| | | AA | TYNMH | VIWYDGSDRYSADSVKG | DRAYSSSSDF |
| | | | SEQ ID NO:4858 | SEQ ID NO:12870 | SEQ ID NO:20882 |
| **iPS:393906** | **21-225_13D3** | NA | AGTTATACCATGAAC | TCCATTAGTGGTAGTAGTAGTTACATATACTACGCGGACTCAGTGAAGGGC | GATCGGGGCAGTGGC |
| | | | SEQ ID NO:4859 | SEQ ID NO:12871 | SEQ ID NO:20883 |
| | | AA | SYTMN | SISGSSSYIYYADSVKG | DRGSG |
| | | | SEQ ID NO:4860 | SEQ ID NO:12872 | SEQ ID NO:20884 |

FIGURE 49

| | | | | | |
|---|---|---|---|---|---|
| **iPS:393908** | **21-225_10E9** | NA | AACTATGTCATACAC | GTTATATGGTATGATGGAAGTAATAAATACTATGCAGACTCCGTGAAGGGC | GAAAAGTATAGCAGCAGCTGGTACGACTACGGTATGGACGTC |
| | | | SEQ ID NO:4861 | SEQ ID NO:12873 | SEQ ID NO:20885 |
| | | AA | NYVIH | VIWYDGSNKYYADSVKG | EKYSSSWYDYGMDV |
| | | | SEQ ID NO:4862 | SEQ ID NO:12874 | SEQ ID NO:20886 |
| **iPS:393910** | **21-225_15F10** | NA | AGCTATGGCATGCAC | GTTATATCATATGGTGGAAGTAATAATTACTATGCAGACTCCGTGAAGGGC | CGGGGATACAGCTATGGCGGGTACGGTATGGACGTC |
| | | | SEQ ID NO:4863 | SEQ ID NO:12875 | SEQ ID NO:20887 |
| | | AA | SYGMH | VISYGGSNNYYADSVKG | RGYSYGGYGMDV |
| | | | SEQ ID NO:4864 | SEQ ID NO:12876 | SEQ ID NO:20888 |
| **iPS:393912** | **21-225_16F6** | NA | AACTATGGCATGCAC | GTTATATGGTATGAAGGAAGTAATCAATACTATGGAGACTCCGTGAAGGGC | GAGCTGGGGTTCCTCTCTGATTAC |
| | | | SEQ ID NO:4865 | SEQ ID NO:12877 | SEQ ID NO:20889 |
| | | AA | NYGMH | VIWYEGSNQYYGDSVKG | ELGFLSDY |
| | | | SEQ ID NO:4866 | SEQ ID NO:12878 | SEQ ID NO:20890 |
| **iPS:393914** | **21-225_16B8** | NA | AGCTATAGCATGAAC | TCCATTAGTGGTAGTAGTACTTACATATACTACGCAGACTCAGTGAAGGGC | GTGGCTTCATTCGACTAC |
| | | | SEQ ID NO:4867 | SEQ ID NO:12879 | SEQ ID NO:20891 |
| | | AA | SYSMN | SISGSSTYIYYADSVKG | VASFDY |
| | | | SEQ ID NO:4868 | SEQ ID NO:12880 | SEQ ID NO:20892 |
| **iPS:393916** | **21 225 2G4** | NA | AGCTATGTCATGCAC | GTTATATGGTATGATGGAAGTAATAAATACTATGCAGACTCCGTGAAGGGC | GAGAAGTATAGCAGCAGCTGGTACGACTACGGTATGGACGTC |

FIGURE 49

| | | | | | |
|---|---|---|---|---|---|
| | 21-225_2G4 | | SEQ ID NO:4869 | SEQ ID NO:12881 | SEQ ID NO:20893 |
| | | AA | SYVMH | VIWYDGSNKYYADSVKG | EKYSSSWYDYGMDV |
| | | | SEQ ID NO:4870 | SEQ ID NO:12882 | SEQ ID NO:20894 |
| iPS:393920 | 21-225_1H12 | NA | AGCTATGGCATGCAC | ATTATATGGCATGATGG AAGTAATAAATACTATG TAGACTCCGTGAAGGGC | GATGAGAGGCTGGGGATTTT TGACTAC |
| | | | SEQ ID NO:4871 | SEQ ID NO:12883 | SEQ ID NO:20895 |
| | | AA | SYGMH | IIWHDGSNKYYVDSVKG | DERLGIFDY |
| | | | SEQ ID NO:4872 | SEQ ID NO:12884 | SEQ ID NO:20896 |
| iPS:393922 | 21-225_2B2 | NA | AGCTATGGCATGCAC | GTTATATGGTATGAGGA AAATAATAAATACTATG TAGACTCCGTGAAGGGC | GAACTAGGCTTCCAGTCTGA CTAC |
| | | | SEQ ID NO:4873 | SEQ ID NO:12885 | SEQ ID NO:20897 |
| | | AA | SYGMH | VIWYEENNKYYVDSVKG | ELGFQSDY |
| | | | SEQ ID NO:4874 | SEQ ID NO:12886 | SEQ ID NO:20898 |
| iPS:393926 | 21-225_4G4 | NA | AGCTATGGCATGCAC | GTTATATGGCATGATGG AAGTAATAAATACTATG CAGACTCCGTGAAGGGC | GATCTGAGAATGGGCGGTAT GGACGTC |
| | | | SEQ ID NO:4875 | SEQ ID NO:12887 | SEQ ID NO:20899 |
| | | AA | SYGMH | VIWHDGSNKYYADSVKG | DLRMGGMDV |
| | | | SEQ ID NO:4876 | SEQ ID NO:12888 | SEQ ID NO:20900 |
| iPS:393928 | 21 225 4E10 | NA | CGTAGTAGTTACTACTGGGG C | AGTGTCTATTATAGTGGG GCCACCTCCTACAACCC GTCCCTCAAGAGT | CTAAGCAGCAACTGGGACTT TGACTAC |

FIGURE 49

| | | | | | |
|---|---|---|---|---|---|
| | 21-225_4E10 | | SEQ ID NO:4877 | SEQ ID NO:12889 | SEQ ID NO:20901 |
| | | AA | RSSYYWG | SVYYSGATSYNPSLKS | LSSNWDFDY |
| | | | SEQ ID NO:4878 | SEQ ID NO:12890 | SEQ ID NO:20902 |
| iPS:393930 | 21-225_7E11 | NA | AGCTTTGGCATGCAC | ATTATATGGCATGATGGAAGTAATAAATACTATGCAGACTCCGTGAAGGGC | GATCTGAGTATGGGCGGTATGGACGTC |
| | | | SEQ ID NO:4879 | SEQ ID NO:12891 | SEQ ID NO:20903 |
| | | AA | SFGMH | IIWHDGSNKYYADSVKG | DLSMGGMDV |
| | | | SEQ ID NO:4880 | SEQ ID NO:12892 | SEQ ID NO:20904 |
| iPS:393932 | 21-225_10F5 | NA | AGCTATGGCATGCAC | ATTATATGGCATGATGGAAGTAATAAATATTATGTAGACTCCGTGAAGGGC | GATGAGAGGCTGGGGATTTTTGACTAC |
| | | | SEQ ID NO:4881 | SEQ ID NO:12893 | SEQ ID NO:20905 |
| | | AA | SYGMH | IIWHDGSNKYYVDSVKG | DERLGIFDY |
| | | | SEQ ID NO:4882 | SEQ ID NO:12894 | SEQ ID NO:20906 |
| iPS:393934 | 21-225_13E6 | NA | AACTATGGCATGCAC | GTTATATGGTATGATGAAAATAATAAATATTATATAGACTCCGTGAAGGGC | GAATTGGGGTTCCGGTCTGACTAC |
| | | | SEQ ID NO:4883 | SEQ ID NO:12895 | SEQ ID NO:20907 |
| | | AA | NYGMH | VIWYDENNKYYIDSVKG | ELGFRSDY |
| | | | SEQ ID NO:4884 | SEQ ID NO:12896 | SEQ ID NO:20908 |
| iPS:393936 | 21-225_14A11 | NA | AGCTATGCCATGAAC | GTTATTAGTGGTAGAGGTGGTAGTACATACTACGCAGACTCCGTGAAGGGC | AGGATAGCAGCTGGTATGGAGTACTTCGATCTC |
| | | | SEQ ID NO:4885 | SEQ ID NO:12897 | SEQ ID NO:20909 |
| | | AA | SYAMN | VISGRGGSTYYADSVKG | RIAAGMEYFDL |
| | | | SEQ ID NO:4886 | SEQ ID NO:12898 | SEQ ID NO:20910 |

FIGURE 49

| | | | | | |
|---|---|---|---|---|---|
| **iPS:393940** | **21-225_16B2** | NA | AGCTATGCCATGACC | GTTATTAGTGGTAGTGGTGGTAGCACATTCTACGCAGACTCCGTGAAGGGC | TATTGTAGTAGTACCAGGTGCCCTTATGATGCCTTTGATATC |
| | | | SEQ ID NO:4887 | SEQ ID NO:12899 | SEQ ID NO:20911 |
| | | AA | SYAMT | VISGSGGSTFYADSVKG | YCSSTRCPYDAFDI |
| | | | SEQ ID NO:4888 | SEQ ID NO:12900 | SEQ ID NO:20912 |
| **iPS:393942** | **21-225_11E5** | NA | AATTATGATATCAAC | TGGATGCACCCTAACAGTGGTGCCACCGGCTATGCACAGAGGTTCCAGGGC | AGCAGTGGCTGGGAGGTCTTTGACTAC |
| | | | SEQ ID NO:4889 | SEQ ID NO:12901 | SEQ ID NO:20913 |
| | | AA | NYDIN | WMHPNSGATGYAQRFQG | SSGWEVFDY |
| | | | SEQ ID NO:4890 | SEQ ID NO:12902 | SEQ ID NO:20914 |
| **iPS:393944** | **21-225_14D6** | NA | AGTTATACCATGAAC | TCCATTAGTGGTAGTGGTAGTTACATATACTACGCAGACTCAGTGAAGGGC | GTAGCAGCCTTTGACTCC |
| | | | SEQ ID NO:4891 | SEQ ID NO:12903 | SEQ ID NO:20915 |
| | | AA | SYTMN | SISGSGSYIYYADSVKG | VAAFDS |
| | | | SEQ ID NO:4892 | SEQ ID NO:12904 | SEQ ID NO:20916 |
| **iPS:393946** | **21-225_16A4** | NA | AGCTATAGCATGAAC | TCCATTAGTGGTAGTAGTACTTACAAATACTACGCAGACTCAGTGAAGGGC | GATCGTGGGAGCTAC |
| | | | SEQ ID NO:4893 | SEQ ID NO:12905 | SEQ ID NO:20917 |
| | | AA | SYSMN | SISGSSTYKYYADSVKG | DRGSY |
| | | | SEQ ID NO:4894 | SEQ ID NO:12906 | SEQ ID NO:20918 |
| **iPS:393948** | **21-225_16A5** | NA | GACTATGGCATGCAC | GTTATATGGTTTGATGGAAGTAATAAATACTATGTAGACTCCGTGAAGGGC | GATCTTGGCTGGACGGAAGAGTAC |
| | | | SEQ ID NO:4895 | SEQ ID NO:12907 | SEQ ID NO:20919 |

FIGURE 49

| | | | | | |
|---|---|---|---|---|---|
| | | AA | DYGMH | VIWFDGSNKYYVDSVKG | DLGWTEEY |
| | | | SEQ ID NO:4896 | SEQ ID NO:12908 | SEQ ID NO:20920 |
| **iPS:393950** | **21-225_3H10** | NA | AGCTATGTCATGCAC | GTTATATGGTATGATGGAAGTAATAAATACTATGCAGACTCCGTGAAGGGC | GAGAGATATAGCAGTGGCTGGTATGACTACGGTTTGGACGTC |
| | | | SEQ ID NO:4897 | SEQ ID NO:12909 | SEQ ID NO:20921 |
| | | AA | SYVMH | VIWYDGSNKYYADSVKG | ERYSSGWYDYGLDV |
| | | | SEQ ID NO:4898 | SEQ ID NO:12910 | SEQ ID NO:20922 |
| **iPS:393952** | **21-225_1F1** | NA | AGCTATAACATGAAC | TCCATTAGTAGTAGTAGTAGTTACATATACTACGCAGACTCAGTGAAGGGC | GTTAACCTCTTTGACTAC |
| | | | SEQ ID NO:4899 | SEQ ID NO:12911 | SEQ ID NO:20923 |
| | | AA | SYNMN | SISSSSSYIYYADSVKG | VNLFDY |
| | | | SEQ ID NO:4900 | SEQ ID NO:12912 | SEQ ID NO:20924 |
| **iPS:393954** | **21-225_4H6** | NA | GACTACTATTTGCAC | TGGATCCACCCTAACAGTGGTGGCACAAACTATGCACAGAAGTTTCAGGGC | GATGGTACCAGCTCGTTTGACTAC |
| | | | SEQ ID NO:4901 | SEQ ID NO:12913 | SEQ ID NO:20925 |
| | | AA | DYYLH | WIHPNSGGTNYAQKFQG | DGTSSFDY |
| | | | SEQ ID NO:4902 | SEQ ID NO:12914 | SEQ ID NO:20926 |
| **iPS:393956** | **21-225_4D7** | NA | AGCTATGCCATGAGC | GTTCTTAGTGGTAGTGGTGGTAGCACATTCTACGCAGACTCCGTGAAGGGC | TATTGTAGTAGTGCCAGGTGCCCTTATGATGCCTTTGATATC |
| | | | SEQ ID NO:4903 | SEQ ID NO:12915 | SEQ ID NO:20927 |
| | | AA | SYAMS | VLSGSGGSTFYADSVKG | YCSSARCPYDAFDI |
| | | | SEQ ID NO:4904 | SEQ ID NO:12916 | SEQ ID NO:20928 |

FIGURE 49

| | | | | | |
|---|---|---|---|---|---|
| **iPS:393958** | **21-225_5H2** | NA | AGCTATACCATGAAC | TCCATTAGTGGTAGTAGTAGTTACATATACTACGCAGACTCAGTGAAGGGC | GGGAGCAGCTCGTCCGGCTTTGACTAC |
| | | | SEQ ID NO:4905 | SEQ ID NO:12917 | SEQ ID NO:20929 |
| | | AA | SYTMN | SISGSSSYIYYADSVKG | GSSSSGFDY |
| | | | SEQ ID NO:4906 | SEQ ID NO:12918 | SEQ ID NO:20930 |
| **iPS:393960** | **21-225_7G2** | NA | GACTATGGCATGCAC | GTTATATGGTATGATGTAACTAATAAATACTATGCAGACTCCGTGAAGGGC | GAACTTGGCTGGTACGAGGACTAC |
| | | | SEQ ID NO:4907 | SEQ ID NO:12919 | SEQ ID NO:20931 |
| | | AA | DYGMH | VIWYDVTNKYYADSVKG | ELGWYEDY |
| | | | SEQ ID NO:4908 | SEQ ID NO:12920 | SEQ ID NO:20932 |
| **iPS:393962** | **21-225_7H7** | NA | AGAAGTAGTTACTACTGGGGC | AATATCTATTATAGTGGGAGCACCTACTACATCCCGTCCCTCAAGAGT | CACAGTACCAGCTGGTCTCTTGACCAC |
| | | | SEQ ID NO:4909 | SEQ ID NO:12921 | SEQ ID NO:20933 |
| | | AA | RSSYYWG | NIYYSGSTYYIPSLKS | HSTSWSLDH |
| | | | SEQ ID NO:4910 | SEQ ID NO:12922 | SEQ ID NO:20934 |
| **iPS:393964** | **21-225_6G1** | NA | AGCTATGGCATGCAC | ATTATATGGTATGATGGAAGTAATAAATACTATGCAGACTCCGTGAAGGGC | GATCTGAGTATGGGCGGTATGGACGTC |
| | | | SEQ ID NO:4911 | SEQ ID NO:12923 | SEQ ID NO:20935 |
| | | AA | SYGMH | IIWYDGSNKYYADSVKG | DLSMGGMDV |
| | | | SEQ ID NO:4912 | SEQ ID NO:12924 | SEQ ID NO:20936 |

FIGURE 49

| | | | | | |
|---|---|---|---|---|---|
| **iPS:393966** | **21-225_7F8** | NA | AGCTGTGTCATGCAC | GTTATATGGTATGATGGAAGTAATAAATACTATGCAGACTCCGTGAAGGGC | GAAAGGTATACCAGTGGCTGGCACGACTACGGTATGGACGTC |
| | | | SEQ ID NO:4913 | SEQ ID NO:12925 | SEQ ID NO:20937 |
| | | AA | SCVMH | VIWYDGSNKYYADSVKG | ERYTSGWHDYGMDV |
| | | | SEQ ID NO:4914 | SEQ ID NO:12926 | SEQ ID NO:20938 |
| **iPS:393968** | **21-225_5A5** | NA | AGCTATGCCATGAAC | GCTATTAGTGGTAGTGGTGGTTACACATACTACGCAGACTCCGTGAAGGGC | GGGGGGTCCCTCTTCTAC |
| | | | SEQ ID NO:4915 | SEQ ID NO:12927 | SEQ ID NO:20939 |
| | | AA | SYAMN | AISGSGGYTYYADSVKG | GGSLFY |
| | | | SEQ ID NO:4916 | SEQ ID NO:12928 | SEQ ID NO:20940 |
| **iPS:393972** | **21-225_7C9** | NA | AACTATGTCATGCAC | GTTATATGGTATGATGGAAGTAATAAATACTATGCAGACTCCGTGAAGGGC | GAGAAGTATAGCAGCAGCTGGTACGACTACGGTATGGACGTC |
| | | | SEQ ID NO:4917 | SEQ ID NO:12929 | SEQ ID NO:20941 |
| | | AA | NYVMH | VIWYDGSNKYYADSVKG | EKYSSSWYDYGMDV |
| | | | SEQ ID NO:4918 | SEQ ID NO:12930 | SEQ ID NO:20942 |
| **iPS:393974** | **21-225_7C4** | NA | AACTATGGCATGCAC | GTTATTTGGTATGAAGAAAATAATCAATACTATGCAGACTCCGTGAAGGGC | GAACTGGGGTTCCGGTCTGACTAC |
| | | | SEQ ID NO:4919 | SEQ ID NO:12931 | SEQ ID NO:20943 |
| | | AA | NYGMH | VIWYEENNQYYADSVKG | ELGFRSDY |
| | | | SEQ ID NO:4920 | SEQ ID NO:12932 | SEQ ID NO:20944 |

**FIGURE 49**

| | | | | | |
|---|---|---|---|---|---|
| **iPS:393976** | **21-225_7E9** | NA | GACTATGGCATGCAC | GTTATATGGTATGATGAAAATAATAAATACTATGTAGACTCCGTGAAGGGC | GAATTGGGGTTCCGGTCTGACTAC |
| | | | SEQ ID NO:4921 | SEQ ID NO:12933 | SEQ ID NO:20945 |
| | | AA | DYGMH | VIWYDENNKYYVDSVKG | ELGFRSDY |
| | | | SEQ ID NO:4922 | SEQ ID NO:12934 | SEQ ID NO:20946 |
| **iPS:393978** | **21-225_4C12** | NA | AGCTATGTCATGCAC | GTTATATGGTATGATGGAAGTAATAAATACTATGCAGACTCCGTGAAGGGC | GAGAAGTATAGCAGCAGCTGGTACGACTACGGTATGGACGTC |
| | | | SEQ ID NO:4923 | SEQ ID NO:12935 | SEQ ID NO:20947 |
| | | AA | SYVMH | VIWYDGSNKYYADSVKG | EKYSSSWYDYGMDV |
| | | | SEQ ID NO:4924 | SEQ ID NO:12936 | SEQ ID NO:20948 |
| **iPS:393980** | **21-225_6D3** | NA | AGCTATGCCATGAAC | GTTATTAGTGGTCGTGGTGGTAACACATTCTACGCAGACTCCGTGAAGGGC | CGTTTGGCAGTGGCTGGCTCGGAGGCTTTTGATATC |
| | | | SEQ ID NO:4925 | SEQ ID NO:12937 | SEQ ID NO:20949 |
| | | AA | SYAMN | VISGRGGNTFYADSVKG | RLAVAGSEAFDI |
| | | | SEQ ID NO:4926 | SEQ ID NO:12938 | SEQ ID NO:20950 |
| **iPS:393982** | **21-225_6C12** | NA | AGCTATAGCATGAAC | TCCATTAGTAGTAGTAGTAGTTACATATACTACGCAGACTCAGTGAAGGGC | GATCGTGGGAGCCTC |
| | | | SEQ ID NO:4927 | SEQ ID NO:12939 | SEQ ID NO:20951 |
| | | AA | SYSMN | SISSSSSYIYYADSVKG | DRGSL |
| | | | SEQ ID NO:4928 | SEQ ID NO:12940 | SEQ ID NO:20952 |

**FIGURE 49**

| | | | | | |
|---|---|---|---|---|---|
| **iPS:393984** | **21-225_4F12** | NA | AGCTATGGCATGCAC | GTTATATGGTATGATGTTACTAATAAAAGTATGCAGACTCCGTGAAGGGC | GAAAAGGGGGGTCTATTTGACTAC |
| | | | SEQ ID NO:4929 | SEQ ID NO:12941 | SEQ ID NO:20953 |
| | | AA | SYGMH | VIWYDVTNKKYADSVKG | EKGGLFDY |
| | | | SEQ ID NO:4930 | SEQ ID NO:12942 | SEQ ID NO:20954 |
| **iPS:393986** | **21-225_7G4** | NA | AACTATGTCATGCAC | GTTATATGGTATGATGGAAGTAATAAATACTATGCAGACTCCGTGAAGGGC | GAGAAGTATAGCAGCAACTGGTACGACTACGGTATGGACGTC |
| | | | SEQ ID NO:4931 | SEQ ID NO:12943 | SEQ ID NO:20955 |
| | | AA | NYVMH | VIWYDGSNKYYADSVKG | EKYSSNWYDYGMDV |
| | | | SEQ ID NO:4932 | SEQ ID NO:12944 | SEQ ID NO:20956 |
| **iPS:393988** | **21-225_7F10** | NA | AGCTATAGCATGAAC | TCCATTAGTAGTAGTAGTAGTTACATATACTACGCAGACTCAGTGAAGGGC | GCTAACCTCTTTGACTAC |
| | | | SEQ ID NO:4933 | SEQ ID NO:12945 | SEQ ID NO:20957 |
| | | AA | SYSMN | SISSSSSYIYYADSVKG | ANLFDY |
| | | | SEQ ID NO:4934 | SEQ ID NO:12946 | SEQ ID NO:20958 |
| **iPS:393990** | **21-225_11G7** | NA | AGGAGTACTTACTACTGGGGC | AGTATCTATTATAGTGGGAGCACCTCCTACAGCCCGTCCCTCAAGAGT | CTGAACAGCAGCTGGTCTTTTGACTAC |
| | | | SEQ ID NO:4935 | SEQ ID NO:12947 | SEQ ID NO:20959 |
| | | AA | RSTYYWG | SIYYSGSTSYSPSLKS | LNSSWSFDY |
| | | | SEQ ID NO:4936 | SEQ ID NO:12948 | SEQ ID NO:20960 |

## FIGURE 49

| | | | | | |
|---|---|---|---|---|---|
| **iPS:393992** | **21-225_14H8** | NA | AGCAATAGCATGAAC | TACATTAGTAGTAGTAGTAGTACCATATACTACGCAGACTCTGTGAAGGGC | GGAGGTGGGAGCCCTTTTGACTAC |
| | | | SEQ ID NO:4937 | SEQ ID NO:12949 | SEQ ID NO:20961 |
| | | AA | SNSMN | YISSSSSTIYYADSVKG | GGGSPFDY |
| | | | SEQ ID NO:4938 | SEQ ID NO:12950 | SEQ ID NO:20962 |
| **iPS:393994** | **21-225_8C9** | NA | AGCTATGGCATGCAC | GTTATATGGTATGATGAAAATAATAAATACTATGTAGACTCCGTGAAGGGC | GAATTGGGGTTCCGGTCTGACTAC |
| | | | SEQ ID NO:4939 | SEQ ID NO:12951 | SEQ ID NO:20963 |
| | | AA | SYGMH | VIWYDENNKYYVDSVKG | ELGFRSDY |
| | | | SEQ ID NO:4940 | SEQ ID NO:12952 | SEQ ID NO:20964 |
| **iPS:393996** | **21-225_15C11** | NA | GACTATGTCATGCAC | GTTATATGGTATGATGGAAGTAATAAATACTATGCAGACTCCGTGAAGGGC | GAGAAGTATAGCAGCAGCTGGTACGACTACGGTTTGGACGTC |
| | | | SEQ ID NO:4941 | SEQ ID NO:12953 | SEQ ID NO:20965 |
| | | AA | DYVMH | VIWYDGSNKYYADSVKG | EKYSSSWYDYGLDV |
| | | | SEQ ID NO:4942 | SEQ ID NO:12954 | SEQ ID NO:20966 |
| **iPS:393998** | **21-225_12B12** | NA | GACTATGGCATGCAC | GTTATATGGTATGATGTAACTAATAAATACTATGCAGACTCCGTGAAGGGC | GAGCTTGGCTGGTACGAGGACTAC |
| | | | SEQ ID NO:4943 | SEQ ID NO:12955 | SEQ ID NO:20967 |
| | | AA | DYGMH | VIWYDVTNKYYADSVKG | ELGWYEDY |
| | | | SEQ ID NO:4944 | SEQ ID NO:12956 | SEQ ID NO:20968 |

**FIGURE 49**

| | | | | | |
|---|---|---|---|---|---|
| **iPS:394000** | **21-225_11A2** | NA | AGCTATGGCATGCAC | GTTATATCATATGGTGGAAGTAATAAAGACTCTGCAGACTCCGTGAAGGGC | CGGGGATACAGCTATGGCGGGTACGGTATGGACGTC |
| | | | SEQ ID NO:4945 | SEQ ID NO:12957 | SEQ ID NO:20969 |
| | | AA | SYGMH | VISYGGSNKDSADSVKG | RGYSYGGYGMDV |
| | | | SEQ ID NO:4946 | SEQ ID NO:12958 | SEQ ID NO:20970 |
| **iPS:394002** | **21-225_15G7** | NA | AGGAGTAGTTCCTACTGGGGC | AGTATCTATTATAGTGGGTACACCTATTACACCCCGTCCCTCAAGAGT | CTGAGCAGCAGTTGGTCTTTTGACTTC |
| | | | SEQ ID NO:4947 | SEQ ID NO:12959 | SEQ ID NO:20971 |
| | | AA | RSSSYWG | SIYYSGYTYYTPSLKS | LSSSWSFDF |
| | | | SEQ ID NO:4948 | SEQ ID NO:12960 | SEQ ID NO:20972 |
| **iPS:394004** | **21-225_13A1** | NA | AGCTATGGCATGCAC | GTTATATCATATGCGGGAACTAATCAATACTATGCAGACTCCGTGAAGGGC | CGGGGATACAGCTATGGCGGGTACGGTATGGACGTC |
| | | | SEQ ID NO:4949 | SEQ ID NO:12961 | SEQ ID NO:20973 |
| | | AA | SYGMH | VISYAGTNQYYADSVKG | RGYSYGGYGMDV |
| | | | SEQ ID NO:4950 | SEQ ID NO:12962 | SEQ ID NO:20974 |
| **iPS:394006** | **21-225_15C2** | NA | AGCTATGGCATGCAC | ATAATATCATATGGTGGACGTAATAATCACTATGCAGACTCCGTGAAGGGC | CGGGGATACAGCTATGGCGGGTACGGTATGGACGTC |
| | | | SEQ ID NO:4951 | SEQ ID NO:12963 | SEQ ID NO:20975 |
| | | AA | SYGMH | IISYGGRNNHYADSVKG | RGYSYGGYGMDV |
| | | | SEQ ID NO:4952 | SEQ ID NO:12964 | SEQ ID NO:20976 |
| **iPS:394008** | **21-225_15H8** | NA | AGCTATGTCATGAAC | TCCATTAGTGGTAGTAGTACTTACATATACTGCGCAGACTCAATCAAGGGC | GATCGAGGCTCCATC |
| | | | SEQ ID NO:4953 | SEQ ID NO:12965 | SEQ ID NO:20977 |

# FIGURE 49

| | | | | | |
|---|---|---|---|---|---|
| | | AA | SYVMN | SISGSSTYIYCADSIKG | DRGSI |
| | | | SEQ ID NO:4954 | SEQ ID NO:12966 | SEQ ID NO:20978 |
| **iPS:394010** | **21-225_12G5** | NA | AACTATGGCATCTAC | GTTATATCATATGATGGAAGTAATAAATACTATGCAGACTCCGTGAAGGGC | GATCGGGGAGCAGTGGCTGCTTACTACTACGGTATAGACGTC |
| | | | SEQ ID NO:4955 | SEQ ID NO:12967 | SEQ ID NO:20979 |
| | | AA | NYGIY | VISYDGSNKYYADSVKG | DRGAVAAYYYYGIDV |
| | | | SEQ ID NO:4956 | SEQ ID NO:12968 | SEQ ID NO:20980 |
| **iPS:394012** | **21-225_15A3** | NA | AGCTATGGCATTCAC | GTTATATGGCATGATGGAAGTAATAAATACTATGCAGACTCCGTGAAGGGC | GATCTGAGTATGGGCGGTATGGACGTC |
| | | | SEQ ID NO:4957 | SEQ ID NO:12969 | SEQ ID NO:20981 |
| | | AA | SYGIH | VIWHDGSNKYYADSVKG | DLSMGGMDV |
| | | | SEQ ID NO:4958 | SEQ ID NO:12970 | SEQ ID NO:20982 |
| **iPS:394014** | **21-225_8G6** | NA | AGCTATGTCATGAAC | GTTATTAGTGGTCGTGGTGGTAACACATTCTACGCAGACTCCGTGAAGGGC | CGTTTGGCAGTGGCTGGCTCGGAGGCTTTTGATATC |
| | | | SEQ ID NO:4959 | SEQ ID NO:12971 | SEQ ID NO:20983 |
| | | AA | SYVMN | VISGRGGNTFYADSVKG | RLAVAGSEAFDI |
| | | | SEQ ID NO:4960 | SEQ ID NO:12972 | SEQ ID NO:20984 |
| **iPS:394016** | **21-225_13D4** | NA | AGCTATGGCATGCAC | GTTATATGGCATGATGGAAGTAATAAATACTATGCAGACTCCGTGAAGGGC | GATCTGAGTATGGGCGGTATGGACGTC |
| | | | SEQ ID NO:4961 | SEQ ID NO:12973 | SEQ ID NO:20985 |
| | | AA | SYGMH | VIWHDGSNKYYADSVKG | DLSMGGMDV |

**FIGURE 49**

| | | | | | |
|---|---|---|---|---|---|
| | | | SEQ ID NO:4962 | SEQ ID NO:12974 | SEQ ID NO:20986 |
| **iPS:394018** | **21-225_15B1** | NA | AGCTATGTCATGAGC | GGTATTAGTGGTAGTGGTGGTAGCACAAACAACGCAGACTCCGTGAAGGGC | AGCTCCTTGTTTGACTAC |
| | | | SEQ ID NO:4963 | SEQ ID NO:12975 | SEQ ID NO:20987 |
| | | AA | SYVMS | GISGSGGSTNNADSVKG | SSLFDY |
| | | | SEQ ID NO:4964 | SEQ ID NO:12976 | SEQ ID NO:20988 |
| **iPS:394020** | **21-225_15H10** | NA | AACTATGGCATGCAC | GTTATATGGTATGATGAAAGTAATAAATACTATGAAGACTCCGTGAAGGGC | GAAGTGGGGTTTCTTTCTGACTAC |
| | | | SEQ ID NO:4965 | SEQ ID NO:12977 | SEQ ID NO:20989 |
| | | AA | NYGMH | VIWYDESNKYYEDSVKG | EVGFLSDY |
| | | | SEQ ID NO:4966 | SEQ ID NO:12978 | SEQ ID NO:20990 |
| **iPS:394022** | **21-225_16H6** | NA | AGCTATGCCATGAAC | GTTATTAGTCGTAGTGGTGGTTACACATACTACGCGGACTCCGTGAAGGGC | CGTTTAGCAGTGGCTGGCTCGGAGGCTTTTGATATC |
| | | | SEQ ID NO:4967 | SEQ ID NO:12979 | SEQ ID NO:20991 |
| | | AA | SYAMN | VISRSGGYTYYADSVKG | RLAVAGSEAFDI |
| | | | SEQ ID NO:4968 | SEQ ID NO:12980 | SEQ ID NO:20992 |
| **iPS:394024** | **21-225_16B7** | NA | AGCTATGGCATGCAC | GTTATATGGTATGATGAAAGTAATAAATACTATGCAGACTCCGTGAAGGGC | GAACTGGGGTTTCTCTCTGACTAC |
| | | | SEQ ID NO:4969 | SEQ ID NO:12981 | SEQ ID NO:20993 |
| | | AA | SYGMH | VIWYDESNKYYADSVKG | ELGFLSDY |
| | | | SEQ ID NO:4970 | SEQ ID NO:12982 | SEQ ID NO:20994 |

## FIGURE 49

| | | | | | |
|---|---|---|---|---|---|
| **iPS:394026** | **21-225_16C7** | NA | AGCTATGTCATGACC | ACTATTAGTGGTAGTGGTGGTTGGACATATTATGCAGACTCCGTGAAGGGC | AGCTCCTTGTTTGACTAT |
| | | | SEQ ID NO:4971 | SEQ ID NO:12983 | SEQ ID NO:20995 |
| | | AA | SYVMT | TISGSGGWTYYADSVKG | SSLFDY |
| | | | SEQ ID NO:4972 | SEQ ID NO:12984 | SEQ ID NO:20996 |
| **iPS:394029** | **21-225_1B12** | NA | AGCTATGGCATGCAC | ATTATATCATATGCTGGAAGTAATAAATCCTATGCAGACTCCGTGAAGGGC | CGGGGATACAGCTATGGCGGGTACGGTATGGACGTC |
| | | | SEQ ID NO:4973 | SEQ ID NO:12985 | SEQ ID NO:20997 |
| | | AA | SYGMH | IISYAGSNKSYADSVKG | RGYSYGGYGMDV |
| | | | SEQ ID NO:4974 | SEQ ID NO:12986 | SEQ ID NO:20998 |
| **iPS:394033** | **21-225_5F4** | NA | AGCTATAGCATGAAC | TCCATTAGTGGTAGTAGTAGTTACATATACTACGCAGACTCAGTGAAGGGC | GTTAACAACTTTGACTAC |
| | | | SEQ ID NO:4975 | SEQ ID NO:12987 | SEQ ID NO:20999 |
| | | AA | SYSMN | SISGSSSYIYYADSVKG | VNNFDY |
| | | | SEQ ID NO:4976 | SEQ ID NO:12988 | SEQ ID NO:21000 |
| **iPS:394035** | **21-225_5G9** | NA | AATTATGGCATGCAC | ATTATATCATATGCTGGAAGTAATAAATACTATGCAGACTCCGTGAAGGGC | CGTATAACAGCTCGTCTCTACTACGGTATGGACGTC |
| | | | SEQ ID NO:4977 | SEQ ID NO:12989 | SEQ ID NO:21001 |
| | | AA | NYGMH | IISYAGSNKYYADSVKG | RITARLYYGMDV |
| | | | SEQ ID NO:4978 | SEQ ID NO:12990 | SEQ ID NO:21002 |
| **iPS:394037** | **21-225_4F4** | NA | AGGAGTAGTTACTACTGGGGC | AATATTTATTATAGTGGGAGCACCTACGACAACCCGTCCCTCAAGAGT | CATGGAAAAGACTGGGGCCTTGACTAC |
| | | | SEQ ID NO:4979 | SEQ ID NO:12991 | SEQ ID NO:21003 |
| | | AA | RSSYYWG | NIYYSGSTYDNPSLKS | HGKDWGLDY |

**FIGURE 49**

| | | | | | |
|---|---|---|---|---|---|
| | | | SEQ ID NO:4980 | SEQ ID NO:12992 | SEQ ID NO:21004 |
| **iPS:394041** | **21-225_5E5** | NA | AACTATGTCATGCAC | GTTATATGGTATGATGGAAGTAATAAATACTATGCAGACTCCGTGAAGGGC | GAGGTATATAGCAGTGGCTGGTACGACTACGGTATGGACGTC |
| | | | SEQ ID NO:4981 | SEQ ID NO:12993 | SEQ ID NO:21005 |
| | | AA | NYVMH | VIWYDGSNKYYADSVKG | EVYSSGWYDYGMDV |
| | | | SEQ ID NO:4982 | SEQ ID NO:12994 | SEQ ID NO:21006 |
| **iPS:394043** | **21-225_3B1** | NA | AGCTATGCCATGAAC | GTTATTAGTGGTCGTGGTATTAACACATTCTACGCAGACTCCGTGAAGGGC | CGTTTAGCAGTGGCTGGCTCGGAGGCTTTTGATATC |
| | | | SEQ ID NO:4983 | SEQ ID NO:12995 | SEQ ID NO:21007 |
| | | AA | SYAMN | VISGRGINTFYADSVKG | RLAVAGSEAFDI |
| | | | SEQ ID NO:4984 | SEQ ID NO:12996 | SEQ ID NO:21008 |
| **iPS:394045** | **21-225_4H4** | NA | AGGAGTAGTTACTACTGGGGC | AATATTTATTATAGTGGGAACACCTACAACAACCCGTCCCTCAAGAGT | CATGGAAAAGACTGGGGCCTTGACTAC |
| | | | SEQ ID NO:4985 | SEQ ID NO:12997 | SEQ ID NO:21009 |
| | | AA | RSSYYWG | NIYYSGNTYNNPSLKS | HGKDWGLDY |
| | | | SEQ ID NO:4986 | SEQ ID NO:12998 | SEQ ID NO:21010 |
| **iPS:394047** | **21-225_5E6** | NA | AGCTATGGCATGCAC | ATTATATCATATGTTGGAAATAATAAATACTATGCAGACTCCGTGAAGGGC | CGGGGATACAGCTATGGCGGGTACGGTATGGACGTC |
| | | | SEQ ID NO:4987 | SEQ ID NO:12999 | SEQ ID NO:21011 |
| | | AA | SYGMH | IISYVGNNKYYADSVKG | RGYSYGGYGMDV |
| | | | SEQ ID NO:4988 | SEQ ID NO:13000 | SEQ ID NO:21012 |

FIGURE 49

| | | | | | |
|---|---|---|---|---|---|
| **iPS:394049** | **21-225_13H5** | NA | AGAAGTAGTTACTACTGGGGC | AGTATCTATTATAGTGGGAGCACCTACTACAACCCGTCCCTCAAGAGT | CTTAGCAGCAGCTGGGACTTCCAGCAC |
| | | | SEQ ID NO:4989 | SEQ ID NO:13001 | SEQ ID NO:21013 |
| | | AA | RSSYYWG | SIYYSGSTYYNPSLKS | LSSSWDFQH |
| | | | SEQ ID NO:4990 | SEQ ID NO:13002 | SEQ ID NO:21014 |
| **iPS:394051** | **21-225_9E5** | NA | AACTATGCCATGAAC | GCTATTAGTGGTGGTGGTGGTAACACATTCTACGCAGACTCCGTGAAGGGC | CGTATAGCAGTGGCTGGCTCGGAGGCTTTTGCTATC |
| | | | SEQ ID NO:4991 | SEQ ID NO:13003 | SEQ ID NO:21015 |
| | | AA | NYAMN | AISGGGGNTFYADSVKG | RIAVAGSEAFAI |
| | | | SEQ ID NO:4992 | SEQ ID NO:13004 | SEQ ID NO:21016 |
| **iPS:394053** | **21-225_11F10** | NA | AGAAGTAGTTACTACTGGGGC | AGTATTTATTATAGTGGGAGCGCCCAGTACAACCCGTCCCTCAAGAGT | CTGAGCAGCAGCTGGTCTTTTGACTAC |
| | | | SEQ ID NO:4993 | SEQ ID NO:13005 | SEQ ID NO:21017 |
| | | AA | RSSYYWG | SIYYSGSAQYNPSLKS | LSSSWSFDY |
| | | | SEQ ID NO:4994 | SEQ ID NO:13006 | SEQ ID NO:21018 |
| **iPS:394055** | **21-225_9C8** | NA | AGCCAGAGCATGAAC | TACATTAGTATTAGTAGTACCATATACTATGCAGACTCTGTGAAGGGC | GGAGGTGGGAGCCCTTTTGACTCC |
| | | | SEQ ID NO:4995 | SEQ ID NO:13007 | SEQ ID NO:21019 |
| | | AA | SQSMN | YISISSTIYYADSVKG | GGGSPFDS |
| | | | SEQ ID NO:4996 | SEQ ID NO:13008 | SEQ ID NO:21020 |
| **iPS:394057** | **21-225_15H1** | NA | AGAAGTAGTTACTACTGGGGC | AATATCTATTATAGTGGGTATCCCTACTACAATCCGTCCCTCAAGAGT | CATAGCACCAGCTGGTCCCTTGACTAC |
| | | | SEQ ID NO:4997 | SEQ ID NO:13009 | SEQ ID NO:21021 |
| | | AA | RSSYYWG | NIYYSGYPYYNPSLKS | HSTSWSLDY |
| | | | SEQ ID NO:4998 | SEQ ID NO:13010 | SEQ ID NO:21022 |

**FIGURE 49**

| | | | | | |
|---|---|---|---|---|---|
| **iPS:394059** | **21-225_9E8** | NA | AGCTATGGCATGCAC | GTTATTTGGTATGAAGAAAATAATCAATACTATGCAGACTCCGTGAAGGGC | GAACTGGGGTTCCGGTCTGACTAC |
| | | | SEQ ID NO:4999 | SEQ ID NO:13011 | SEQ ID NO:21023 |
| | | AA | SYGMH | VIWYEENNQYYADSVKG | ELGFRSDY |
| | | | SEQ ID NO:5000 | SEQ ID NO:13012 | SEQ ID NO:21024 |
| **iPS:394061** | **21-225_12D2** | NA | AGCTATAGCATGAAC | TCCATTAGTAGTAGTAGTAGTTACATATACTACGCAGACTCAGTGAAGGGC | TTAGGGGACTAC |
| | | | SEQ ID NO:5001 | SEQ ID NO:13013 | SEQ ID NO:21025 |
| | | AA | SYSMN | SISSSSSYIYYADSVKG | LGDY |
| | | | SEQ ID NO:5002 | SEQ ID NO:13014 | SEQ ID NO:21026 |
| **iPS:394063** | **21-225_16A1** | NA | AGGAGTAGTTACTACTGGGGC | AGTATCTATTATAGTGGGAGCGCCTATCACAACCCGTCCCTCAAGAGT | CTGAGCAGCAGCTGGTCCTTTGACTAC |
| | | | SEQ ID NO:5003 | SEQ ID NO:13015 | SEQ ID NO:21027 |
| | | AA | RSSYYWG | SIYYSGSAYHNPSLKS | LSSSWSFDY |
| | | | SEQ ID NO:5004 | SEQ ID NO:13016 | SEQ ID NO:21028 |
| **iPS:394065** | **21-225_11E2** | NA | AATTATGATATCAAC | TGGATGAACACTAACAGTGGTAACACAGGCTATGCACAGAAGTTCCAGGGC | AGTCATGGCTGGTTCCTCTTTGACTAC |
| | | | SEQ ID NO:5005 | SEQ ID NO:13017 | SEQ ID NO:21029 |
| | | AA | NYDIN | WMNTNSGNTGYAQKFQG | SHGWFLFDY |
| | | | SEQ ID NO:5006 | SEQ ID NO:13018 | SEQ ID NO:21030 |

FIGURE 49

| | | | | | |
|---|---|---|---|---|---|
| **iPS:394067** | **21-225_12F2** | NA | GACTATGGCATGCAC | GTTATATGGTTTGATGGAAATAATAAATACTATGTAGATTCCGTGAAGGGC | GAGCTTGCCTGGTCCGAGGACTAC |
| | | | SEQ ID NO:5007 | SEQ ID NO:13019 | SEQ ID NO:21031 |
| | | AA | DYGMH | VIWFDGNNKYYVDSVKG | ELAWSEDY |
| | | | SEQ ID NO:5008 | SEQ ID NO:13020 | SEQ ID NO:21032 |
| **iPS:394069** | **21-225_16H1** | NA | AGCTATAGCATGAAC | TCCATTAGTGGTAGTAGTACTTACATATACTACGCAGACTCAGTGAAGGGC | GTAGCAGCCTTTGACTAC |
| | | | SEQ ID NO:5009 | SEQ ID NO:13021 | SEQ ID NO:21033 |
| | | AA | SYSMN | SISGSSTYIYYADSVKG | VAAFDY |
| | | | SEQ ID NO:5010 | SEQ ID NO:13022 | SEQ ID NO:21034 |
| **iPS:394071** | **21-225_10C7** | NA | AGCTATAGCATGAAC | TCCATTAGTAGTAGTAATAATTACATATACTACGCAGACTCAGTGAAGGGC | TTAGGGGTCTAC |
| | | | SEQ ID NO:5011 | SEQ ID NO:13023 | SEQ ID NO:21035 |
| | | AA | SYSMN | SISSSNNYIYYADSVKG | LGVY |
| | | | SEQ ID NO:5012 | SEQ ID NO:13024 | SEQ ID NO:21036 |
| **iPS:394073** | **21-225_15C9** | NA | AGAAGTAGTTACTACTGGGGC | AATATCTATTATAGTGGGAGCACCTACAACAACCCGTCCCTCAAGAGT | CAGGGCAGTGGCTGGGAGGTTGACTAC |
| | | | SEQ ID NO:5013 | SEQ ID NO:13025 | SEQ ID NO:21037 |
| | | AA | RSSYYWG | NIYYSGSTYNNPSLKS | QGSGWEVDY |
| | | | SEQ ID NO:5014 | SEQ ID NO:13026 | SEQ ID NO:21038 |
| **iPS:394075** | **21-225_8D12** | NA | AGAAGTAGTTACTACTGGGGC | AATATCTATTATAGTGGGTATCCCTACTACAATCCGTCCCTCAAGAGT | CATAGCACCAGCTGGTCCCTTGACTAC |
| | | | SEQ ID NO:5015 | SEQ ID NO:13027 | SEQ ID NO:21039 |

**FIGURE 49**

| | | | | | |
|---|---|---|---|---|---|
| | | AA | RSSYYWG | NIYYSGYPYYNPSLKS | HSTSWSLDY |
| | | | SEQ ID NO:5016 | SEQ ID NO:13028 | SEQ ID NO:21040 |
| **iPS:394077** | **21-225_8E12** | NA | AGCTATGCCATGAGC | ATTATTAGTGGTCGTGGTGGTAACACATTCTACGCAGACTCCGTGAAGGGC | CGTATGGCAGTGGCTGGCTCGGAGGCTTTTGATATC |
| | | | SEQ ID NO:5017 | SEQ ID NO:13029 | SEQ ID NO:21041 |
| | | AA | SYAMS | IISGRGGNTFYADSVKG | RMAVAGSEAFDI |
| | | | SEQ ID NO:5018 | SEQ ID NO:13030 | SEQ ID NO:21042 |
| **iPS:394079** | **21-225_11F5** | NA | AGGAGTAGTTACTACTGGGGC | AATATTTATTATAGTGGGAGCACCTACACCAACCCGTCCCTCAAGAGT | CATGGAAAAGACTGGGGCCTTGACAAC |
| | | | SEQ ID NO:5019 | SEQ ID NO:13031 | SEQ ID NO:21043 |
| | | AA | RSSYYWG | NIYYSGSTYTNPSLKS | HGKDWGLDN |
| | | | SEQ ID NO:5020 | SEQ ID NO:13032 | SEQ ID NO:21044 |
| **iPS:394081** | **21-225_16B3** | NA | AGCTATGGCATGCAC | GTTATATCATATGCTGGAATTAATAAATCCTATGCAGACTCCGTGAAGGGC | CGGGGATACAGCTATGGCGGGTATGGTATGGACGTC |
| | | | SEQ ID NO:5021 | SEQ ID NO:13033 | SEQ ID NO:21045 |
| | | AA | SYGMH | VISYAGINKSYADSVKG | RGYSYGGYGMDV |
| | | | SEQ ID NO:5022 | SEQ ID NO:13034 | SEQ ID NO:21046 |
| **iPS:394083** | **21-225_16E6** | NA | AGCTATGGCATGCAC | GTTATATGGCATGATGGAAGTAATAAATACTATGTAGACTCCGTGAAGGGC | GATCTGAGTATGGGCGGTATGGACGTC |
| | | | SEQ ID NO:5023 | SEQ ID NO:13035 | SEQ ID NO:21047 |
| | | AA | SYGMH | VIWHDGSNKYYVDSVKG | DLSMGGMDV |
| | | | SEQ ID NO:5024 | SEQ ID NO:13036 | SEQ ID NO:21048 |

FIGURE 49

| | | | | | |
|---|---|---|---|---|---|
| **iPS:394085** | **21-225_8B11** | NA | AATTATGATATCAAC | TGGATGCACCCTAACAGTGGTAACACAGGCTATGCACAGAAGTTCCAGGGC | AGCAGTGGCTGGTACTTCTTTGACTAC |
| | | | SEQ ID NO:5025 | SEQ ID NO:13037 | SEQ ID NO:21049 |
| | | AA | NYDIN | WMHPNSGNTGYAQKFQG | SSGWYFFDY |
| | | | SEQ ID NO:5026 | SEQ ID NO:13038 | SEQ ID NO:21050 |
| **iPS:394087** | **21-225_11A5** | NA | AGCTATGCCATGAGC | GTTATTAGTGGTCGTGGTGTTAACACATTCTACGCAGACTCCGTGAAGGGC | CGTATCGCAGTGGCTGGCTCGGAGGCTTTTGATATC |
| | | | SEQ ID NO:5027 | SEQ ID NO:13039 | SEQ ID NO:21051 |
| | | AA | SYAMS | VISGRGVNTFYADSVKG | RIAVAGSEAFDI |
| | | | SEQ ID NO:5028 | SEQ ID NO:13040 | SEQ ID NO:21052 |
| **iPS:394089** | **21-225_12E6** | NA | GACTATGGCATGCAC | GTTATATGGTATGATGAAAGTAATAAATACTATGCAGACTCCGTGAAGGGC | GAGCTTGCCTGGTACGAGGACTAC |
| | | | SEQ ID NO:5029 | SEQ ID NO:13041 | SEQ ID NO:21053 |
| | | AA | DYGMH | VIWYDESNKYYADSVKG | ELAWYEDY |
| | | | SEQ ID NO:5030 | SEQ ID NO:13042 | SEQ ID NO:21054 |
| **iPS:394091** | **21-225_13H3** | NA | AGCTATGGCATGCAC | GTTATTTGGTATGAGGAAAGTAATAAATACTATGTAGACTCCGTGAGGGGC | GAACTAGGCTTCCAGTCTGACTTC |
| | | | SEQ ID NO:5031 | SEQ ID NO:13043 | SEQ ID NO:21055 |
| | | AA | SYGMH | VIWYEESNKYYVDSVRG | ELGFQSDF |
| | | | SEQ ID NO:5032 | SEQ ID NO:13044 | SEQ ID NO:21056 |

**FIGURE 49**

| | | | | | |
|---|---|---|---|---|---|
| **iPS:394093** | **21-225_9D12** | NA | GACTATGGCATGCAC | GTTATATGGTATGATGGAAATAATAATTACTATGCAGACTCCGTGAAGGGC | GAGCTTGCCTGGTACGAGGACTTC |
| | | | SEQ ID NO:5033 | SEQ ID NO:13045 | SEQ ID NO:21057 |
| | | AA | DYGMH | VIWYDGNNNYYADSVKG | ELAWYEDF |
| | | | SEQ ID NO:5034 | SEQ ID NO:13046 | SEQ ID NO:21058 |
| **iPS:394095** | **21-225_16H4** | NA | AACTATGGCATGCAC | GTTATATGGTATGATGTAAGTAATAAATACTATGCAGACTCCGTGAAGGGC | GAAATGGGCTGGACCGATGACTGC |
| | | | SEQ ID NO:5035 | SEQ ID NO:13047 | SEQ ID NO:21059 |
| | | AA | NYGMH | VIWYDVSNKYYADSVKG | EMGWTDDC |
| | | | SEQ ID NO:5036 | SEQ ID NO:13048 | SEQ ID NO:21060 |
| **iPS:394097** | **21-225_16G7** | NA | GACTATGGCATGCAC | GTTATTTGGTATGATGAAAATAATGAATACTATGCAGACTCCGTGAAGGGC | GAGCTTGCCTGGTACGAGGACTAC |
| | | | SEQ ID NO:5037 | SEQ ID NO:13049 | SEQ ID NO:21061 |
| | | AA | DYGMH | VIWYDENNEYYADSVKG | ELAWYEDY |
| | | | SEQ ID NO:5038 | SEQ ID NO:13050 | SEQ ID NO:21062 |
| **iPS:398470** | **21-225_14B7** | NA | GGCTACTATATGCAC | TGGATCAACCCTAACAGTGGTGGCACAAACTATGTACAGAAGTTTCAGGGC | TCGTTTTTCTATGGTTCGGGGAGTTATTATAACGAATTTGACTAC |
| | | | SEQ ID NO:5039 | SEQ ID NO:13051 | SEQ ID NO:21063 |
| | | AA | GYYMH | WINPNSGGTNYVQKFQG | SFFYGSGSYYNEFDY |
| | | | SEQ ID NO:5040 | SEQ ID NO:13052 | SEQ ID NO:21064 |

**FIGURE 49**

| | | | | | |
|---|---|---|---|---|---|
| **iPS:398472** | **21-225_16E4** | NA | AGCTATGTCATGAGC | ACTATTAGTGTTGGTGGTGGTACCACATACTACGCAGACTCCGTGAAGGGC | TGGGGACGTGGCAACAGCTATGAGTACTACTACGGTATGGACGTC |
| | | | SEQ ID NO:5041 | SEQ ID NO:13053 | SEQ ID NO:21065 |
| | | AA | SYVMS | TISVGGGTTYYADSVKG | WGRGNSYEYYYGMDV |
| | | | SEQ ID NO:5042 | SEQ ID NO:13054 | SEQ ID NO:21066 |
| **iPS:398474** | **21-225_17B10** | NA | AGCTATGCCATGAGC | GTTATTAGTGGTAGTGGTGGTAACACATACTTCGCAGACTCCGTGAAGGGC | AGGGGTATACCAGAGGCTGATGCTTTTGATATC |
| | | | SEQ ID NO:5043 | SEQ ID NO:13055 | SEQ ID NO:21067 |
| | | AA | SYAMS | VISGSGGNTYFADSVKG | RGIPEADAFDI |
| | | | SEQ ID NO:5044 | SEQ ID NO:13056 | SEQ ID NO:21068 |
| **iPS:398476** | **21-225_17C1** | NA | AGCTATGCCATGAGC | GTTATTAGTGGTAGTGGTGGTACCACATTCTACGCAGACTCCGTGAAGGGC | TATTGTAGTAGTACCAGGTGTCCTTATGATGCCTTTGATATC |
| | | | SEQ ID NO:5045 | SEQ ID NO:13057 | SEQ ID NO:21069 |
| | | AA | SYAMS | VISGSGGTTFYADSVKG | YCSSTRCPYDAFDI |
| | | | SEQ ID NO:5046 | SEQ ID NO:13058 | SEQ ID NO:21070 |
| **iPS:398478** | **21-225_17C10** | NA | AGCTATAGCATGAAC | TCCATTAGTGGTAGTAGTAGTTACATGTACTACGCAGACTCAGTGAAGGGC | GATCGTGGGAGCTCC |
| | | | SEQ ID NO:5047 | SEQ ID NO:13059 | SEQ ID NO:21071 |
| | | AA | SYSMN | SISGSSSYMYYADSVKG | DRGSS |
| | | | SEQ ID NO:5048 | SEQ ID NO:13060 | SEQ ID NO:21072 |
| **iPS:398480** | **21-225_17G4** | NA | GACTATTATATGCAC | TGGATCAACCCTAACAGTGGTGGCACAAACTATGAACAGAAGTTTCAGGGC | GGATACAGCTATGGGTACAACTGGTTCGACCCC |
| | | | SEQ ID NO:5049 | SEQ ID NO:13061 | SEQ ID NO:21073 |
| | | AA | DYYMH | WINPNSGGTNYEQKFQG | GYSYGYNWFDP |
| | | | SEQ ID NO:5050 | SEQ ID NO:13062 | SEQ ID NO:21074 |

FIGURE 49

| | | | | | |
|---|---|---|---|---|---|
| **iPS:398482** | **21-225_17H6** | NA | AGCTATAGCATGAAC | TCCATTAGTGGTAGTAGTAGTTACATATACTACGCAGACTCAGTGAAGGGC | GTGGCTTCATTTGACTAC |
| | | | SEQ ID NO:5051 | SEQ ID NO:13063 | SEQ ID NO:21075 |
| | | AA | SYSMN | SISGSSSYIYYADSVKG | VASFDY |
| | | | SEQ ID NO:5052 | SEQ ID NO:13064 | SEQ ID NO:21076 |
| **iPS:398484** | **21-225_18D4** | NA | GGCTACTATTTGCAC | TGGATCAACCCTAACAGTAATGGCACAATCTCTGCACAGAAGTTTCAGGGC | GATGGTACCAGCTCGCTTGACTAC |
| | | | SEQ ID NO:5053 | SEQ ID NO:13065 | SEQ ID NO:21077 |
| | | AA | GYYLH | WINPNSNGTISAQKFQG | DGTSSLDY |
| | | | SEQ ID NO:5054 | SEQ ID NO:13066 | SEQ ID NO:21078 |
| **iPS:398486** | **21-225_19A1** | NA | GGCTACTATATGCAC | TGGATCAATCCTAACAGTGGTGGCACAAACTATGCACAGAAGTTTCAGGGC | GGATACAGCTATGGGTACAACTGGTTCGACCCC |
| | | | SEQ ID NO:5055 | SEQ ID NO:13067 | SEQ ID NO:21079 |
| | | AA | GYYMH | WINPNSGGTNYAQKFQG | GYSYGYNWFDP |
| | | | SEQ ID NO:5056 | SEQ ID NO:13068 | SEQ ID NO:21080 |
| **iPS:398488** | **21-225_19F6** | NA | AACGCCTGGATGAAC | CGTATTAAAAGCAAAACTGATGGTGGGACAACAGACTACGCTGCACCCGTGAAAGGC | GATACGGGTCCTATAGCAGCTCGTCTCGCTTACTACTACTATTACGCTATGGACGTC |
| | | | SEQ ID NO:5057 | SEQ ID NO:13069 | SEQ ID NO:21081 |
| | | AA | NAWMN | RIKSKTDGGTTDYAAPVKG | DTGPIAARLAYYYYYAMDV |
| | | | SEQ ID NO:5058 | SEQ ID NO:13070 | SEQ ID NO:21082 |
| **iPS:398490** | **21-225_21D12** | NA | GACTACTATATTCAC | TGGATCAACCCTAACAGTGGTGGGACAAACAATGCACAGAAGTTTCAGGGC | TCGTATTACTATGGTTCGGGGACTTATTATAACGAATTTGACTAC |
| | | | SEQ ID NO:5059 | SEQ ID NO:13071 | SEQ ID NO:21083 |

**FIGURE 49**

| | | | | | |
|---|---|---|---|---|---|
| | | AA | DYYIH | WINPNSGGTNNAQKFQG | SYYYGSGTYYNEFDY |
| | | | SEQ ID NO:5060 | SEQ ID NO:13072 | SEQ ID NO:21084 |
| **iPS:398494** | **21-225_21H4** | NA | AGCTATGCCATGAGC | GCTCTTAGTGGTCGTGGTGGTAGCACATACTACGCAGACTCCGTGAAGGGC | TGGGGACGTGGATACAGCTATGAGTACTACTACGGTATGGACGTC |
| | | | SEQ ID NO:5061 | SEQ ID NO:13073 | SEQ ID NO:21085 |
| | | AA | SYAMS | ALSGRGGSTYYADSVKG | WGRGYSYEYYYGMDV |
| | | | SEQ ID NO:5062 | SEQ ID NO:13074 | SEQ ID NO:21086 |
| **iPS:398496** | **21-225_22D2** | NA | AATTATGATATCAAC | TGGATGCACCCTGACAGTGGTAACACAGGCTATGCACAGAAGTTCCAGGGC | AGTAGTGGCTGGTACTACTTTGACTAC |
| | | | SEQ ID NO:5063 | SEQ ID NO:13075 | SEQ ID NO:21087 |
| | | AA | NYDIN | WMHPDSGNTGYAQKFQG | SSGWYYFDY |
| | | | SEQ ID NO:5064 | SEQ ID NO:13076 | SEQ ID NO:21088 |
| **iPS:398498** | **21-225_22E6** | NA | ACTGGTGGAGTGGGTGTGGGC | CTCATTTATTGGAATGATGATAAGCGCTACAGCCCATCTCTGAAGAGC | ACTATAGCAGTTCGTGGCTTTGACTAC |
| | | | SEQ ID NO:5065 | SEQ ID NO:13077 | SEQ ID NO:21089 |
| | | AA | TGGVGVG | LIYWNDDKRYSPSLKS | TIAVRGFDY |
| | | | SEQ ID NO:5066 | SEQ ID NO:13078 | SEQ ID NO:21090 |
| **iPS:398500** | **21-225_23A11** | NA | AGTTATAGCATGAAC | TCCATTAGTGGTAGTAGTACTTACATATACTACGCAGACTCAGTGAAGGGC | GTGGCTTCATTTGACTAC |
| | | | SEQ ID NO:5067 | SEQ ID NO:13079 | SEQ ID NO:21091 |
| | | AA | SYSMN | SISGSSTYIYYADSVKG | VASFDY |
| | | | SEQ ID NO:5068 | SEQ ID NO:13080 | SEQ ID NO:21092 |

FIGURE 49

| | | | | | |
|---|---|---|---|---|---|
| **iPS:398502** | **21-225_23B11** | NA | GGCTACTATCTGCAC | TGGATCAACCCTAACAATAATGGCACAAACTATGCACAGAAGTTTCAGGGC | GATGGTACCAGCTCGTTTGACTAC |
| | | | SEQ ID NO:5069 | SEQ ID NO:13081 | SEQ ID NO:21093 |
| | | AA | GYYLH | WINPNNNGTNYAQKFQG | DGTSSFDY |
| | | | SEQ ID NO:5070 | SEQ ID NO:13082 | SEQ ID NO:21094 |
| **iPS:398504** | **21-225_23D7** | NA | ACTAGTGGAGTGGGTGTGGGC | CTCATTTATTGGAATAATGATAAGGTCTACAGCCCATCTCTGAAGAGC | AGGGGACAGCAGCTGGCCCTCGACTAC |
| | | | SEQ ID NO:5071 | SEQ ID NO:13083 | SEQ ID NO:21095 |
| | | AA | TSGVGVG | LIYWNNDKVYSPSLKS | RGQQLALDY |
| | | | SEQ ID NO:5072 | SEQ ID NO:13084 | SEQ ID NO:21096 |
| **iPS:398506** | **21-225_23G12** | NA | AATTATGATATCAAC | TGGATGTACCCTAACAGTGGTAACACGGGCTATGCACAGAAGTTCCAGGGC | AGCGGTGGCTGGTACTACTTTGACTAC |
| | | | SEQ ID NO:5073 | SEQ ID NO:13085 | SEQ ID NO:21097 |
| | | AA | NYDIN | WMYPNSGNTGYAQKFQG | SGGWYYFDY |
| | | | SEQ ID NO:5074 | SEQ ID NO:13086 | SEQ ID NO:21098 |
| **iPS:398508** | **21-225_24B1** | NA | AGCTATGCCATGAGC | GCTATTAGTGGTCGTGGTGGTAGCACATACTACGCAGACTCCGTGAAGGGC | TGGGGACGTGGATACAGCTATGAGTACTACTACGGTATGGACGTC |
| | | | SEQ ID NO:5075 | SEQ ID NO:13087 | SEQ ID NO:21099 |
| | | AA | SYAMS | AISGRGGSTYYADSVKG | WGRGYSYEYYYGMDV |
| | | | SEQ ID NO:5076 | SEQ ID NO:13088 | SEQ ID NO:21100 |
| **iPS:398510** | | NA | AATTATGATATCAAC | TGGATGCACCCTAACAGTGGTAACACAGGCTATGCACAGAAGTTCCAGGGC | AGCAGTGGCTGGTATTGGTTCGACCCC |

FIGURE 49

| | | | | | |
|---|---|---|---|---|---|
| | 21-225_25A3 | | SEQ ID NO:5077 | SEQ ID NO:13089 | SEQ ID NO:21101 |
| | | AA | NYDIN | WMHPNSGNTGYAQKFQG | SSGWYWFDP |
| | | | SEQ ID NO:5078 | SEQ ID NO:13090 | SEQ ID NO:21102 |
| iPS:398512 | | NA | AATTATGATATCAAC | TGGATGAACCCTAACAG TGGTAACACAGGCTATG CACAGAAGTTCCAGGGC | AGCAATGGCTGGTACTACTT TGACTAC |
| | 21-225_25E12 | | SEQ ID NO:5079 | SEQ ID NO:13091 | SEQ ID NO:21103 |
| | | AA | NYDIN | WMNPNSGNTGYAQKFQG | SNGWYYFDY |
| | | | SEQ ID NO:5080 | SEQ ID NO:13092 | SEQ ID NO:21104 |
| iPS:398516 | | NA | AATTATGATATCAAC | TGGATGCACCCTAACAG TGGTAACACAGGCTGTG CACAGAAGTTCCAGGGC | AGCAGTGGCTGGTACTGGTT CGACCCC |
| | 21-225_26A9 | | SEQ ID NO:5081 | SEQ ID NO:13093 | SEQ ID NO:21105 |
| | | AA | NYDIN | WMHPNSGNTGCAQKFQG | SSGWYWFDP |
| | | | SEQ ID NO:5082 | SEQ ID NO:13094 | SEQ ID NO:21106 |
| iPS:398520 | | NA | GGCGATTATATGCAC | TGGATCAGCCCTAAAAA TGGTGGCACAAACTATG CACAGAAGTTTCAGGGC | GATGGAACTGGGTCCTTTGA CTAC |
| | 21-225_31C4 | | SEQ ID NO:5083 | SEQ ID NO:13095 | SEQ ID NO:21107 |
| | | AA | GDYMH | WISPKNGGTNYAQKFQG | DGTGSFDY |
| | | | SEQ ID NO:5084 | SEQ ID NO:13096 | SEQ ID NO:21108 |
| iPS:398522 | | NA | AACTATGATATTAAC | TGGATGAACCCTAACAG TGGTAACACAGGCTATG CACAGAAGTTCCAGGGC | AGCAGTGGCTGGTACTTTTT TGACTAC |
| | 21-225_32A1 | | SEQ ID NO:5085 | SEQ ID NO:13097 | SEQ ID NO:21109 |

FIGURE 49

| | | | | | |
|---|---|---|---|---|---|
| | | AA | NYDIN | WMNPNSGNTGYAQKFQG | SSGWYFFDY |
| | | | SEQ ID NO:5086 | SEQ ID NO:13098 | SEQ ID NO:21110 |
| **iPS:398524** | **21-225_32A5** | NA | AATTATGACATCAAC | TGGATGCACCCTAACAG TGGTAACACAGGCTTTG CACAGAAGTTCCGGGGC | AGCAGTGGCTGGTACTTTTT TGACTAC |
| | | | SEQ ID NO:5087 | SEQ ID NO:13099 | SEQ ID NO:21111 |
| | | AA | NYDIN | WMHPNSGNTGFAQKFRG | SSGWYFFDY |
| | | | SEQ ID NO:5088 | SEQ ID NO:13100 | SEQ ID NO:21112 |
| **iPS:398526** | **21-225_32B3** | NA | AGCTATAGCATGAAC | TCCATTAGTGGTAGTAGT ACTTACATATACTACGCA GACTCAGTGAAGGGC | GTGGCTGGCTTTGACTAC |
| | | | SEQ ID NO:5089 | SEQ ID NO:13101 | SEQ ID NO:21113 |
| | | AA | SYSMN | SISGSSTYIYYADSVKG | VAGFDY |
| | | | SEQ ID NO:5090 | SEQ ID NO:13102 | SEQ ID NO:21114 |
| **iPS:398528** | **21-225_32G1** | NA | AGCTATGCCATGAGC | GCTATTAGTGGTCGTGGT GGTAGCACATTCCACGC AGACTCCGTGAAGGGC | GGGGAGCTACTAGAGGACTA CTACTTCTACGGTATGGACG TC |
| | | | SEQ ID NO:5091 | SEQ ID NO:13103 | SEQ ID NO:21115 |
| | | AA | SYAMS | AISGRGGSTFHADSVKG | GELLEDYYFYGMDV |
| | | | SEQ ID NO:5092 | SEQ ID NO:13104 | SEQ ID NO:21116 |
| **iPS:398530** | **21-225_32G4** | NA | AGTTATGATATCAAC | TGGATGAACCCTAACAG TGGTAACACAGGCTATG CACAGAAGTTCCAGGGC | AAGAAGGCTAACGACTAC |
| | | | SEQ ID NO:5093 | SEQ ID NO:13105 | SEQ ID NO:21117 |
| | | AA | SYDIN | WMNPNSGNTGYAQKFQG | KKANDY |
| | | | SEQ ID NO:5094 | SEQ ID NO:13106 | SEQ ID NO:21118 |

**FIGURE 49**

| | | | | | |
|---|---|---|---|---|---|
| **iPS:398532** | **21-225_33B7** | NA | AGCTATAACATGAAC | TCCATTAGTGGTAGTAGTAGTTACATATACTACGCAGACTCAGTGAAGGGC | TTAAATGGTTTTGACTAC |
| | | | SEQ ID NO:5095 | SEQ ID NO:13107 | SEQ ID NO:21119 |
| | | AA | SYNMN | SISGSSSYIYYADSVKG | LNGFDY |
| | | | SEQ ID NO:5096 | SEQ ID NO:13108 | SEQ ID NO:21120 |
| **iPS:398534** | **21-225_33B8** | NA | AGCTATGCCATGAGC | GCTATTAGTGGTCGTGGTGGTAGCACATTCCACGCAGACTCCGTGAAGGGC | GGGGAGCTACTAGAGGACTACTACTTCTACGGTATGGACGTC |
| | | | SEQ ID NO:5097 | SEQ ID NO:13109 | SEQ ID NO:21121 |
| | | AA | SYAMS | AISGRGGSTFHADSVKG | GELLEDYYFYGMDV |
| | | | SEQ ID NO:5098 | SEQ ID NO:13110 | SEQ ID NO:21122 |
| **iPS:398536** | **21-225_33D12** | NA | AGTTATGATATCAGC | TGGATGAACCCTAACAGTGGTAACACAGGCTATGCACAGAAGTTCCAGGGC | AAGAGGGCTAACGACTAC |
| | | | SEQ ID NO:5099 | SEQ ID NO:13111 | SEQ ID NO:21123 |
| | | AA | SYDIS | WMNPNSGNTGYAQKFQG | KRANDY |
| | | | SEQ ID NO:5100 | SEQ ID NO:13112 | SEQ ID NO:21124 |
| **iPS:398538** | **21-225_34H7** | NA | AACTATGATATTAAC | TGGATGAACCCTAACAGTGGTAACACAGGCTATGCACAGAAGTTCCAGGGC | AGCAGTGGCTGGTACTTTTTTGACTAC |
| | | | SEQ ID NO:5101 | SEQ ID NO:13113 | SEQ ID NO:21125 |
| | | AA | NYDIN | WMNPNSGNTGYAQKFQG | SSGWYFFDY |
| | | | SEQ ID NO:5102 | SEQ ID NO:13114 | SEQ ID NO:21126 |
| **iPS:398540** | **21-225_35A6** | NA | AGCTATGCCATGAGC | ACTATTAGTGGTCGTGGTGGTAGCACATTCCACGCAGACTCCGTGAAGGGC | GGGGAGCTACTAGAGGACTACTACTTCTACGGTATGGACGTC |
| | | | SEQ ID NO:5103 | SEQ ID NO:13115 | SEQ ID NO:21127 |

FIGURE 49

| | | | | | |
|---|---|---|---|---|---|
| | | AA | SYAMS | TISGRGGSTFHADSVKG | GELLEDYYFYGMDV |
| | | | SEQ ID NO:5104 | SEQ ID NO:13116 | SEQ ID NO:21128 |
| **iPS:398544** | **21-225_7C8** | NA | AACGCCCGGATGAAC | CGTATTAAAAGCAAAAC TGATGGTGGGACAACAG ACTACGCTGCACCCGTG AAAGGC | GATACGGGTCCTATAGCAGC TCGTCTCGCTTACTACTACTA CTACGCTATGGACGTC |
| | | | SEQ ID NO:5105 | SEQ ID NO:13117 | SEQ ID NO:21129 |
| | | AA | NARMN | RIKSKTDGGTTDYAAPVK G | DTGPIAARLAYYYYYAMDV |
| | | | SEQ ID NO:5106 | SEQ ID NO:13118 | SEQ ID NO:21130 |
| **iPS:398546** | **21-225_9H10** | NA | ACTAGTGGAGTGGGTGTGGG C | CTCATTTATTGGAGTGAT GATAAGCGCTACAGCCC ATCTCTGAAGAGC | ACCGGTTCTAGCTGCTGCTA TTTTGACTAC |
| | | | SEQ ID NO:5107 | SEQ ID NO:13119 | SEQ ID NO:21131 |
| | | AA | TSGVGVG | LIYWSDDKRYSPSLKS | TGSSCCYFDY |
| | | | SEQ ID NO:5108 | SEQ ID NO:13120 | SEQ ID NO:21132 |
| **iPS:402219** | **21-225_1C12** | NA | AACTATGGCATGCAC | GTTATATGGTATGATGA AAATAATAAATACTATG TAGACTCCGTGAAGGGC | GAATTGGGGTTCCGGTCTGA CTAC |
| | | | SEQ ID NO:5109 | SEQ ID NO:13121 | SEQ ID NO:21133 |
| | | AA | NYGMH | VIWYDENNKYYVDSVKG | ELGFRSDY |
| | | | SEQ ID NO:5110 | SEQ ID NO:13122 | SEQ ID NO:21134 |
| **iPS:402221** | **21-225_2C12** | NA | AGCTATAGCATGAAC | TCCATTAGTGGTAGTAGT AGTTACATGTACTACGC AGACTCAGTGAAGGGC | GTGAATCTCTTTGACTAC |
| | | | SEQ ID NO:5111 | SEQ ID NO:13123 | SEQ ID NO:21135 |
| | | AA | SYSMN | SISGSSSYMYYADSVKG | VNLFDY |
| | | | SEQ ID NO:5112 | SEQ ID NO:13124 | SEQ ID NO:21136 |

FIGURE 49

| | | | | | |
|---|---|---|---|---|---|
| **iPS:402223** | **21-225_30A11** | NA | GACTATCATATGCAC | TGGATCAACCCTAATAGGGGTGGCACAAACTATGCACAGAAGTTTCAGGAC | GATGGAACTGGGTCCTTTGACTAC |
| | | | SEQ ID NO:5113 | SEQ ID NO:13125 | SEQ ID NO:21137 |
| | | AA | DYHMH | WINPNRGGTNYAQKFQD | DGTGSFDY |
| | | | SEQ ID NO:5114 | SEQ ID NO:13126 | SEQ ID NO:21138 |
| **iPS:402225** | **21-225_2B1** | NA | AGCTATAGCATGAAC | TCCATTAGTAGTAGTAGTAGTTACATATACTACGCAGACTCAGTGAAGGGC | CTGGGGAACTAC |
| | | | SEQ ID NO:5115 | SEQ ID NO:13127 | SEQ ID NO:21139 |
| | | AA | SYSMN | SISSSSSYIYYADSVKG | LGNY |
| | | | SEQ ID NO:5116 | SEQ ID NO:13128 | SEQ ID NO:21140 |
| **iPS:402229** | **21-225_16H9** | NA | AGCTATAGCATGAAC | TCCATTAGTGGTAGTAGTAGTTACATATACTACGCAGACTCAGTGAAGGGC | GTCAACGGTATGGACGTC |
| | | | SEQ ID NO:5117 | SEQ ID NO:13129 | SEQ ID NO:21141 |
| | | AA | SYSMN | SISGSSSYIYYADSVKG | VNGMDV |
| | | | SEQ ID NO:5118 | SEQ ID NO:13130 | SEQ ID NO:21142 |
| **iPS:402231** | **21-225_6D9** | NA | AACGCCTGGATGAAC | CGTATTAAAAGCAAAACTGATGGTGGGACAACAGACTACGCTGCACCCGTGAAAGGC | GATACGGGTCCTATAGCAGCTCGTCTCGCTTACTACTACTACTACGCTATGGACGTC |
| | | | SEQ ID NO:5119 | SEQ ID NO:13131 | SEQ ID NO:21143 |
| | | AA | NAWMN | RIKSKTDGGTTDYAAPVKG | DTGPIAARLAYYYYYAMDV |
| | | | SEQ ID NO:5120 | SEQ ID NO:13132 | SEQ ID NO:21144 |
| **iPS:402233** | **21 225 16D10** | NA | ACCTATAACTTGAAC | TCCATTAGTGGTGGTGCCGGTCACATATATTACTCAGACTCAGTGAAGGGC | ACTAATGGGTTTGACTTC |

**FIGURE 49**

| | | | | | |
|---|---|---|---|---|---|
| | **21-225_16D10** | | SEQ ID NO:5121 | SEQ ID NO:13133 | SEQ ID NO:21145 |
| | | AA | TYNLN | SISGGAGHIYYSDSVKG | TNGFDF |
| | | | SEQ ID NO:5122 | SEQ ID NO:13134 | SEQ ID NO:21146 |
| **iPS:402235** | **21-225_20F10** | NA | AGCTATAGCATGAAC | TCCATTAGTACTAGTACTTTCATATACTACGCAGATTCAGTGAAGGGC | AAGGCTGGGCTTGATATC |
| | | | SEQ ID NO:5123 | SEQ ID NO:13135 | SEQ ID NO:21147 |
| | | AA | SYSMN | SISTSTFIYYADSVKG | KAGLDI |
| | | | SEQ ID NO:5124 | SEQ ID NO:13136 | SEQ ID NO:21148 |
| **iPS:402237** | **21-225_23D11** | NA | AGCTATAACATAAAC | TCCATTAGTGGTAATAGTGGTTACATATACTACGCAGACTCAGTGAAGGGC | ACTAACCTCTTTGACTAC |
| | | | SEQ ID NO:5125 | SEQ ID NO:13137 | SEQ ID NO:21149 |
| | | AA | SYNIN | SISGNSGYIYYADSVKG | TNLFDY |
| | | | SEQ ID NO:5126 | SEQ ID NO:13138 | SEQ ID NO:21150 |
| **iPS:403868** | **21-225_19D11** | NA | AGAAGTAGTTATTACTGGGGC | AGTATCTATTATAGTGGGAGCGCCAACTACAACCCGTCCCTCAAGAGT | CTGGACAGGGGCTGGTCCTTTGACTAC |
| | | | SEQ ID NO:5127 | SEQ ID NO:13139 | SEQ ID NO:21151 |
| | | AA | RSSYYWG | SIYYSGSANYNPSLKS | LDRGWSFDY |
| | | | SEQ ID NO:5128 | SEQ ID NO:13140 | SEQ ID NO:21152 |
| **iPS:403870** | **21-225_23G4** | NA | AGCTATGCCATGAGC | GTTATTAGTGGCGTGGTGGTAGCACATACTACGCAGACTCCGTGAAGGGC | AGGGGGATAGTGGGAGCTACTGAGGCTTTTGATATC |
| | | | SEQ ID NO:5129 | SEQ ID NO:13141 | SEQ ID NO:21153 |
| | | AA | SYAMS | VISGRGGSTYYADSVKG | RGIVGATEAFDI |
| | | | SEQ ID NO:5130 | SEQ ID NO:13142 | SEQ ID NO:21154 |
| **iPS:403872** | **21 225 8F11** | NA | AGGACTAGTTACTACTGGGGC | AATATTTATTATAGTGGGAGCGCCTACAACAACCCGTCCCTCAAGAGT | CATGGACAAGACTGGGGCCTTGACTAC |

FIGURE 49

| | | | | | |
|---|---|---|---|---|---|
| | **21-225_8F11** | | SEQ ID NO:5131 | SEQ ID NO:13143 | SEQ ID NO:21155 |
| | | AA | RTSYYWG | NIYYSGSAYNNPSLKS | HGQDWGLDY |
| | | | SEQ ID NO:5132 | SEQ ID NO:13144 | SEQ ID NO:21156 |
| **iPS:404090** | **21-225_8D8** | NA | AGCTATAGCATGAAC | TCCATTAGTAGTAGTAGT AGTTACATATACTACGC AGACTCAGTGAAGGGC | CTGGGTAACTAC |
| | | | SEQ ID NO:5133 | SEQ ID NO:13145 | SEQ ID NO:21157 |
| | | AA | SYSMN | SISSSSSYIYYADSVKG | LGNY |
| | | | SEQ ID NO:5134 | SEQ ID NO:13146 | SEQ ID NO:21158 |
| **iPS:412232** | **21-225_4A2** | NA | AATTATGATATCAAC | TGGATGCACCCTAACAG TGGTAACACAGGCTATG CACAGAAGTTCCAGGGC | AGCAGTGGCTGGTACTACTT TGACTAC |
| | | | SEQ ID NO:5135 | SEQ ID NO:13147 | SEQ ID NO:21159 |
| | | AA | NYDIN | WMHPNSGNTGYAQKFQG | SSGWYYFDY |
| | | | SEQ ID NO:5136 | SEQ ID NO:13148 | SEQ ID NO:21160 |
| **iPS:422894** | **21-225_4A2.001** | NA | AATTATGATATCAAC | TGGATGCACCCTAACAG TGGTAACACAGGCTATG CACAGAAGTTCCAGGGC | AGCAGTGGCTGGTACTACTT TGACTAC |
| | | | SEQ ID NO:5137 | SEQ ID NO:13149 | SEQ ID NO:21161 |
| | | AA | NYDIN | WMHPNSGNTGYAQKFQG | SSGWYYFDY |
| | | | SEQ ID NO:5138 | SEQ ID NO:13150 | SEQ ID NO:21162 |
| **iPS:423018** | **21-225_31D12_LC2** | NA | GGCTACTATATGCAC | TGGATCAACCCTAACAG TGGTGGCACAAACTATG TACAGAAGTTTCAGGGC | GTGTATTACTATGGTTCGGG GAGTTATTATAACGAGTTTG ACTAC |
| | | | SEQ ID NO:5139 | SEQ ID NO:13151 | SEQ ID NO:21163 |
| | | AA | GYYMH | WINPNSGGTNYVQKFQG | VYYYGSGSYYNEFDY |
| | | | SEQ ID NO:5140 | SEQ ID NO:13152 | SEQ ID NO:21164 |

FIGURE 49

| | | | | | |
|---|---|---|---|---|---|
| **iPS:423019** | **21-225_31D12_LC1** | NA | GGCTACTATATGCAC | TGGATCAACCCTAACAGTGGTGGCACAAACTATGTACAGAAGTTTCAGGGC | GTGTATTACTATGGTTCGGGGAGTTATTATAACGAGTTTGACTAC |
| | | | SEQ ID NO:5141 | SEQ ID NO:13153 | SEQ ID NO:21165 |
| | | AA | GYYMH | WINPNSGGTNYVQKFQG | VYYYGSGSYYNEFDY |
| | | | SEQ ID NO:5142 | SEQ ID NO:13154 | SEQ ID NO:21166 |
| **iPS:423314** | **21-225_12F11** | NA | AATTATGATATCAAC | TGGATGCACCCTAACAGTGGTAACACAGGCTATGCAAAGAAGTTCCAGGGC | AGCAGTGGCTGGTACTACTTTGACTAC |
| | | | SEQ ID NO:5143 | SEQ ID NO:13155 | SEQ ID NO:21167 |
| | | AA | NYDIN | WMHPNSGNTGYAKKFQG | SSGWYYFDY |
| | | | SEQ ID NO:5144 | SEQ ID NO:13156 | SEQ ID NO:21168 |
| **iPS:424419** | **21-225_25A4.001** | NA | AATTATGATATTAAT | TGGATGTACCCTAACAGTGGTAACACAGGCTATGCACAGAAATTCCAGGGC | AGCAGTGGCTGGTACTACTTTGACTAC |
| | | | SEQ ID NO:5145 | SEQ ID NO:13157 | SEQ ID NO:21169 |
| | | AA | NYDIN | WMYPNSGNTGYAQKFQG | SSGWYYFDY |
| | | | SEQ ID NO:5146 | SEQ ID NO:13158 | SEQ ID NO:21170 |
| **iPS:424460** | **21-225_7E11.001** | NA | AGCTTTGGCATGCAC | ATTATCTGGCATGATGGAAGTAATAAATACTATGCAGACTCCGTGAAGGGC | GATCTGAGTATGGGCGGTATGGACGTC |
| | | | SEQ ID NO:5147 | SEQ ID NO:13159 | SEQ ID NO:21171 |
| | | AA | SFGMH | IIWHDGSNKYYADSVKG | DLSMGGMDV |
| | | | SEQ ID NO:5148 | SEQ ID NO:13160 | SEQ ID NO:21172 |

FIGURE 49

| | | | | | |
|---|---|---|---|---|---|
| **iPS:426108** | **21-225_10G6** | NA | GCCTACCATATGCAC | TGGATCAACCCTAACAATAATGGCACAAACTATGCACAGAAGTTTCAGGGC | GATGTTACCAGCTCGTTTGACTAT |
| | | | SEQ ID NO:5149 | SEQ ID NO:13161 | SEQ ID NO:21173 |
| | | AA | AYHMH | WINPNNNGTNYAQKFQG | DVTSSFDY |
| | | | SEQ ID NO:5150 | SEQ ID NO:13162 | SEQ ID NO:21174 |
| **iPS:426110** | **21-225_12E9** | NA | GACTACTATTTGCAC | TGGGTCCACCCTAACAGTGGTGGCACAAACTTTGCACAGAAGTTTCAGGAC | GATGGTACCAGCTCGTTTGACTAC |
| | | | SEQ ID NO:5151 | SEQ ID NO:13163 | SEQ ID NO:21175 |
| | | AA | DYYLH | WVHPNSGGTNFAQKFQD | DGTSSFDY |
| | | | SEQ ID NO:5152 | SEQ ID NO:13164 | SEQ ID NO:21176 |
| **iPS:426112** | **21-225_12F12** | NA | AATTATGATATCAAC | TGGATGTACCCTAACAGTGGTAACACGGGCTATGCACAGAAGTTCCAGGGC | AGCAGTGGCTGGTACTACTTTGACTTC |
| | | | SEQ ID NO:5153 | SEQ ID NO:13165 | SEQ ID NO:21177 |
| | | AA | NYDIN | WMYPNSGNTGYAQKFQG | SSGWYYFDF |
| | | | SEQ ID NO:5154 | SEQ ID NO:13166 | SEQ ID NO:21178 |
| **iPS:426114** | **21-225_28H2** | NA | AACTATGTCATGCAC | GTTATATGGTATGATGGAAGTAATAAGTACTATGCAGACTCCGTGAAGGGC | GAGGAGTATAGCAGTGGCTGGTACGACTACGGTATGGACGTC |
| | | | SEQ ID NO:5155 | SEQ ID NO:13167 | SEQ ID NO:21179 |
| | | AA | NYVMH | VIWYDGSNKYYADSVKG | EEYSSGWYDYGMDV |
| | | | SEQ ID NO:5156 | SEQ ID NO:13168 | SEQ ID NO:21180 |

# FIGURE 49

| | | | | | |
|---|---|---|---|---|---|
| **iPS:426116** | **21-225_29E2** | NA | AACTGTGTCATGCAC | GTTATATGGTATGATGGAAGTAATAAATACTATGCAGACTCCGTGAAGGGC | GAGGAGTATAGCAGTGGCTGGTACGACTACGGTATGGACGTC |
| | | | SEQ ID NO:5157 | SEQ ID NO:13169 | SEQ ID NO:21181 |
| | | AA | NCVMH | VIWYDGSNKYYADSVKG | EEYSSGWYDYGMDV |
| | | | SEQ ID NO:5158 | SEQ ID NO:13170 | SEQ ID NO:21182 |
| **iPS:426118** | **21-225_7A10** | NA | AGCTATGGCATGCAC | GTTATATGGTATGATGGAAGTAATAAATACTATGCAGACTCCGTGAAGGGC | GATGAGAGGCTGGGGATTTTTGACTAC |
| | | | SEQ ID NO:5159 | SEQ ID NO:13171 | SEQ ID NO:21183 |
| | | AA | SYGMH | VIWYDGSNKYYADSVKG | DERLGIFDY |
| | | | SEQ ID NO:5160 | SEQ ID NO:13172 | SEQ ID NO:21184 |
| **iPS:426124** | **21-225_32D6** | NA | AGCTATGGCATGCAC | GTTATATGGCATGATGGAAGTAATGCATACTATGCAGACTCCGTGAAGGGC | GAAAATAGCAGCTCGTACTACTTTGACTAC |
| | | | SEQ ID NO:5161 | SEQ ID NO:13173 | SEQ ID NO:21185 |
| | | AA | SYGMH | VIWHDGSNAYYADSVKG | ENSSSYYFDY |
| | | | SEQ ID NO:5162 | SEQ ID NO:13174 | SEQ ID NO:21186 |
| **iPS:426126** | **21-225_6G6** | NA | AATTATGATATCAAC | TGGATGCACCCTAACAGTGGTAACACAGGCTATGCAAAGAAGTTCCAGGGC | AGCAGTGGCTGGTACTACTTTGACTAC |
| | | | SEQ ID NO:5163 | SEQ ID NO:13175 | SEQ ID NO:21187 |
| | | AA | NYDIN | WMHPNSGNTGYAKKFQG | SSGWYYFDY |

FIGURE 49

| | | | | | |
|---|---|---|---|---|---|
| | | | SEQ ID NO:5164 | SEQ ID NO:13176 | SEQ ID NO:21188 |
| **iPS:433895** | **21-225_43E1** | NA | AGCTATAGCATGAAC | GCCATTAGTGGTAATAGTACTTACATATACTACGCAGACTCGTTGAAGGGC | GATCGGGGCAGTGAA |
| | | | SEQ ID NO:5165 | SEQ ID NO:13177 | SEQ ID NO:21189 |
| | | AA | SYSMN | AISGNSTYIYYADSLKG | DRGSE |
| | | | SEQ ID NO:5166 | SEQ ID NO:13178 | SEQ ID NO:21190 |
| **iPS:433897** | **21-225_43C2** | NA | AGCTATGCCATGAGC | GCTATTAGTGGTCGTGGTGTTAACACATTCGACGCAGACTCCGTGAAGGGC | GAAAGGAGTGGGAGCTATTTTGACTAC |
| | | | SEQ ID NO:5167 | SEQ ID NO:13179 | SEQ ID NO:21191 |
| | | AA | SYAMS | AISGRGVNTFDADSVKG | ERSGSYFDY |
| | | | SEQ ID NO:5168 | SEQ ID NO:13180 | SEQ ID NO:21192 |
| **iPS:433899** | **21-225_43C3** | NA | AGCTATGGCATACAC | GTTATATGGTATGATGAAAATAATAAATACTATGCAGACTCCGTGAAGGGC | GAGCTAGGATTTTCCAATGACTAC |
| | | | SEQ ID NO:5169 | SEQ ID NO:13181 | SEQ ID NO:21193 |
| | | AA | SYGIH | VIWYDENNKYYADSVKG | ELGFSNDY |
| | | | SEQ ID NO:5170 | SEQ ID NO:13182 | SEQ ID NO:21194 |
| **iPS:433901** | **21-225_43A4** | NA | AGCTATACCATGAAC | TCCATTAGTGGAAGTAGTACTTACATATACTACGCAGACTCAGTGAAGGGC | GTGACCTCTTTTGACTAC |
| | | | SEQ ID NO:5171 | SEQ ID NO:13183 | SEQ ID NO:21195 |
| | | AA | SYTMN | SISGSSTYIYYADSVKG | VTSFDY |
| | | | SEQ ID NO:5172 | SEQ ID NO:13184 | SEQ ID NO:21196 |
| **iPS:433903** | **21 225 43H4** | NA | AGCTATGCCATGAGC | GCTATTAGTGGTCGTGGTATTAACACATTCGACGCAGACTCCGTGAAGGGC | GAAAGGAGTGGGAGCTATTTTGACTAC |

FIGURE 49

| | | | | | |
|---|---|---|---|---|---|
| | **21-225_43H4** | | SEQ ID NO:5173 | SEQ ID NO:13185 | SEQ ID NO:21197 |
| | | AA | SYAMS | AISGRGINTFDADSVKG | ERSGSYFDY |
| | | | SEQ ID NO:5174 | SEQ ID NO:13186 | SEQ ID NO:21198 |
| **iPS:433905** | **21-225_43E5** | NA | GACTACTACATGATC | TACATTAGTAGTAGTGGT ATTACCAAATACTACGC AGACTCTATGAAGGGC | GATACAATCTAC |
| | | | SEQ ID NO:5175 | SEQ ID NO:13187 | SEQ ID NO:21199 |
| | | AA | DYYMI | YISSSGITKYYADSMKG | DTIY |
| | | | SEQ ID NO:5176 | SEQ ID NO:13188 | SEQ ID NO:21200 |
| **iPS:433909** | **21-225_43D8** | NA | AATTATGATATCAAC | TGGATGCACCCTAACAG TGGTAACACAGGCTATG CACAGAAGTTCCAGGGC | AGCAGTGGCTGGACCCTCTT TGACTAC |
| | | | SEQ ID NO:5177 | SEQ ID NO:13189 | SEQ ID NO:21201 |
| | | AA | NYDIN | WMHPNSGNTGYAQKFQG | SSGWTLFDY |
| | | | SEQ ID NO:5178 | SEQ ID NO:13190 | SEQ ID NO:21202 |
| **iPS:433911** | **21-225_43E8** | NA | AGCTATGCCATGAGC | GCTATTAGTGGTCGTGGT ATTAACACATTCGACGC AGACTCCGTGAAGGGC | GAAAGGAGTGGGAGCTATTT TGACTAC |
| | | | SEQ ID NO:5179 | SEQ ID NO:13191 | SEQ ID NO:21203 |
| | | AA | SYAMS | AISGRGINTFDADSVKG | ERSGSYFDY |
| | | | SEQ ID NO:5180 | SEQ ID NO:13192 | SEQ ID NO:21204 |
| **iPS:433913** | **21-225_43H8** | NA | GACTACTACATGAAC | TACATTAGTAGTAGTGGT AGAACCATATTCTACGC AGACTCTTTGAAGGGC | GATACAATCTAC |
| | | | SEQ ID NO:5181 | SEQ ID NO:13193 | SEQ ID NO:21205 |
| | | AA | DYYMN | YISSSGRTIFYADSLKG | DTIY |
| | | | SEQ ID NO:5182 | SEQ ID NO:13194 | SEQ ID NO:21206 |

FIGURE 49

| | | | | | |
|---|---|---|---|---|---|
| **iPS:433915** | **21-225_43H9** | NA | AGCTATGCCATGAGT | GCTATTAGTGGTAGTGGTAGTAACACATTCTACGCAGACTCCGTGAAGGGC | CGAACGCCCTCTGATGTTTTTGATATC |
| | | | SEQ ID NO:5183 | SEQ ID NO:13195 | SEQ ID NO:21207 |
| | | AA | SYAMS | AISGSGSNTFYADSVKG | RTPSDVFDI |
| | | | SEQ ID NO:5184 | SEQ ID NO:13196 | SEQ ID NO:21208 |
| **iPS:433917** | **21-225_43E11** | NA | GACTATGGCATGCAC | GTTATATGGTATGATGGAAGTAATAAATACTATGCAGACTCCGTGAAGGGC | CGGTATGTCAGAAGCTGGGTGGGAGGTATGGACGTC |
| | | | SEQ ID NO:5185 | SEQ ID NO:13197 | SEQ ID NO:21209 |
| | | AA | DYGMH | VIWYDGSNKYYADSVKG | RYVRSWVGGMDV |
| | | | SEQ ID NO:5186 | SEQ ID NO:13198 | SEQ ID NO:21210 |
| **iPS:433919** | **21-225_44B3** | NA | GACTATGGCATGCAC | GTTATATGGTATGATGGAAGTAATAAATACTATGCAGACTCCGTGAAGGGC | GAGAGGTATAGCAGTGGCTGGTACGACTACGGTATGGACGTC |
| | | | SEQ ID NO:5187 | SEQ ID NO:13199 | SEQ ID NO:21211 |
| | | AA | DYGMH | VIWYDGSNKYYADSVKG | ERYSSGWYDYGMDV |
| | | | SEQ ID NO:5188 | SEQ ID NO:13200 | SEQ ID NO:21212 |
| **iPS:433921** | **21-225_44C3** | NA | AGCTATGGCATGCAC | GTTATATGGTTTGAAGGAAGTAATAAATACTATGCAGATTCCGTGAAGGGC | GAACTAGGATTTTCCACCGACTAC |
| | | | SEQ ID NO:5189 | SEQ ID NO:13201 | SEQ ID NO:21213 |
| | | AA | SYGMH | VIWFEGSNKYYADSVKG | ELGFSTDY |
| | | | SEQ ID NO:5190 | SEQ ID NO:13202 | SEQ ID NO:21214 |

**FIGURE 49**

| | | | | | |
|---|---|---|---|---|---|
| **iPS:433923** | **21-225_44D3** | NA | AGCTATGTCATGCAC | GTTATATGGTATGATGGAAGTAATAAATACTATGCTGACTCCGTGAAGGGC | GAAAGGTATAGCAGTGGCTTGTACGACTACGGTATGGACGTC |
| | | | SEQ ID NO:5191 | SEQ ID NO:13203 | SEQ ID NO:21215 |
| | | AA | SYVMH | VIWYDGSNKYYADSVKG | ERYSSGLYDYGMDV |
| | | | SEQ ID NO:5192 | SEQ ID NO:13204 | SEQ ID NO:21216 |
| **iPS:433925** | **21-225_44F3** | NA | AGCTATGCCATGAGC | ATTCTCAGTGGTGGTGGTAAGACCACATACTACGCAGACTCCGTGAAGGGC | CGAACGCCCTCTGATGCTTTTGATATC |
| | | | SEQ ID NO:5193 | SEQ ID NO:13205 | SEQ ID NO:21217 |
| | | AA | SYAMS | ILSGGGKTTYYADSVKG | RTPSDAFDI |
| | | | SEQ ID NO:5194 | SEQ ID NO:13206 | SEQ ID NO:21218 |
| **iPS:433929** | **21-225_44D5** | NA | AGCTATGTCATGCAC | GTTATATGGTATGATGGAAGTAATAAATACTATGCAGACTCCGTGAAGGGC | GTCCCGTATAGCAGCAGCTGGTACGACTACGGTATGGACGTC |
| | | | SEQ ID NO:5195 | SEQ ID NO:13207 | SEQ ID NO:21219 |
| | | AA | SYVMH | VIWYDGSNKYYADSVKG | VPYSSSWYDYGMDV |
| | | | SEQ ID NO:5196 | SEQ ID NO:13208 | SEQ ID NO:21220 |
| **iPS:433931** | **21-225_44F6** | NA | AGTTACTACTGGAGC | TATATCTATTACAGTGGAAACACCAACTACAACCCCTCCCTCAAGAGT | GGGGTGGCTATAAAGAACTAC |
| | | | SEQ ID NO:5197 | SEQ ID NO:13209 | SEQ ID NO:21221 |
| | | AA | SYYWS | YIYYSGNTNYNPSLKS | GVAIKNY |
| | | | SEQ ID NO:5198 | SEQ ID NO:13210 | SEQ ID NO:21222 |

FIGURE 49

| | | | | | |
|---|---|---|---|---|---|
| **iPS:433933** | **21-225_44C8** | NA | AACTATGGCATGCAC | GTTATATGGTATGAAGGAAGTAATAAATACTATGCAGACTCCGTGAAGGGC | GAACTGGGGTTCCTCTCTGACTAC |
| | | | SEQ ID NO:5199 | SEQ ID NO:13211 | SEQ ID NO:21223 |
| | | AA | NYGMH | VIWYEGSNKYYADSVKG | ELGFLSDY |
| | | | SEQ ID NO:5200 | SEQ ID NO:13212 | SEQ ID NO:21224 |
| **iPS:433935** | **21-225_44F9** | NA | AGCTATGTCATGCAC | GTTATATGGTATGATGGAAGTAATAAATACTATGCAGACTCCGTGAAGGGC | GAACCATATAGCAGCAGCTGGTACGACTACGGTATGGACGTC |
| | | | SEQ ID NO:5201 | SEQ ID NO:13213 | SEQ ID NO:21225 |
| | | AA | SYVMH | VIWYDGSNKYYADSVKG | EPYSSSWYDYGMDV |
| | | | SEQ ID NO:5202 | SEQ ID NO:13214 | SEQ ID NO:21226 |
| **iPS:433937** | **21-225_44B10** | NA | AGCTATGGCATGCAC | GTTATATGGTATGATGGAAGTAATAAATACTATGCAGACTCCGTGAAGGGC | CGGTATAGCAGCAGCTGGGTGGGGGGTATGGACGTC |
| | | | SEQ ID NO:5203 | SEQ ID NO:13215 | SEQ ID NO:21227 |
| | | AA | SYGMH | VIWYDGSNKYYADSVKG | RYSSSWVGGMDV |
| | | | SEQ ID NO:5204 | SEQ ID NO:13216 | SEQ ID NO:21228 |
| **iPS:433939** | **21-225_44C10** | NA | GACTGTGTCATGCAC | GTTATATGGTATGATGGAAGTAATAAATACTATGCTGACTCCGTGAAGGGC | GAAAGGTATAGCAGTGGCTTGTACGACTACGGTATGGACGTC |
| | | | SEQ ID NO:5205 | SEQ ID NO:13217 | SEQ ID NO:21229 |

FIGURE 49

| | | | | | |
|---|---|---|---|---|---|
| | | AA | DCVMH | VIWYDGSNKYYADSVKG | ERYSSGLYDYGMDV |
| | | | SEQ ID NO:5206 | SEQ ID NO:13218 | SEQ ID NO:21230 |
| **iPS:433941** | **21-225_44D10** | NA | AGCTATGCCATGAGC | GCTATTAGTGGTCGTGGT GTTAACACATTCGACGC AGACTCCGTGAAGGGC | GAAAGGAGTGGGAGCTATTT TGACTAC |
| | | | SEQ ID NO:5207 | SEQ ID NO:13219 | SEQ ID NO:21231 |
| | | AA | SYAMS | AISGRGVNTFDADSVKG | ERSGSYFDY |
| | | | SEQ ID NO:5208 | SEQ ID NO:13220 | SEQ ID NO:21232 |
| **iPS:433943** | **21-225_44E10** | NA | AGCTATGCCATGAAC | GGTGTTGTTGGTAGTGGT GGTAGAACATACTACGC AGACTCCGTGAAGGGC | GATCGGGGGCAGTGGCTCCT AGGCGGTATGGACGTC |
| | | | SEQ ID NO:5209 | SEQ ID NO:13221 | SEQ ID NO:21233 |
| | | AA | SYAMN | GVVGSGGRTYYADSVKG | DRGQWLLGGMDV |
| | | | SEQ ID NO:5210 | SEQ ID NO:13222 | SEQ ID NO:21234 |
| **iPS:433945** | **21-225_44C12** | NA | AGCTATAGCGTGAAC | TACATTAGTAGTAGTAGT AGTACCATATACTACGC AGACTCTGTGAAGGGC | AGTGGATACAGCTATGCTTA CTACTACTACTACGGTATGG ACGTC |
| | | | SEQ ID NO:5211 | SEQ ID NO:13223 | SEQ ID NO:21235 |
| | | AA | SYSVN | YISSSSSTIYYADSVKG | SGYSYAYYYYYGMDV |
| | | | SEQ ID NO:5212 | SEQ ID NO:13224 | SEQ ID NO:21236 |
| **iPS:433947** | **21-225_44E12** | NA | AGCGATGACACGCAC | GTTATATGGTTTGATGAA TATAATAAATACTATGC AGACTCCGTGAAGGGC | GATCTAATAGCAGCAGCTGG GACGGGAGACTAC |
| | | | SEQ ID NO:5213 | SEQ ID NO:13225 | SEQ ID NO:21237 |
| | | AA | SDDTH | VIWFDEYNKYYADSVKG | DLIAAAGTGDY |
| | | | SEQ ID NO:5214 | SEQ ID NO:13226 | SEQ ID NO:21238 |

FIGURE 49

| | | | | | |
|---|---|---|---|---|---|
| **iPS:433949** | **21-225_45H2** | NA | GACTACTACATGAAC | TACATTAGTAGTAGTGGTATTACCAAATACTACGCAGACTCTGTGAAGGGC | GATACAATCTAC |
| | | | SEQ ID NO:5215 | SEQ ID NO:13227 | SEQ ID NO:21239 |
| | | AA | DYYMN | YISSSGITKYYADSVKG | DTIY |
| | | | SEQ ID NO:5216 | SEQ ID NO:13228 | SEQ ID NO:21240 |
| **iPS:433951** | **21-225_45B4** | NA | GACTGTGTCATGCAC | GTTATATGGTATGATGGAAGTAATAAATACTATGCTGACTCCGTGAAGGGC | GAAAGGTATAGCAGTGGCTTGTACGACTACGGTATGGACGTC |
| | | | SEQ ID NO:5217 | SEQ ID NO:13229 | SEQ ID NO:21241 |
| | | AA | DCVMH | VIWYDGSNKYYADSVKG | ERYSSGLYDYGMDV |
| | | | SEQ ID NO:5218 | SEQ ID NO:13230 | SEQ ID NO:21242 |
| **iPS:433953** | **21-225_45H4** | NA | AGCTATGCCATGAGT | GCTATTAGTGGTAGTGGTAGTAACACATTCTACGCAGACTCCGTGAAGGGC | CGAACGCCCTCTGATGTTTTTGATATC |
| | | | SEQ ID NO:5219 | SEQ ID NO:13231 | SEQ ID NO:21243 |
| | | AA | SYAMS | AISGSGSNTFYADSVKG | RTPSDVFDI |
| | | | SEQ ID NO:5220 | SEQ ID NO:13232 | SEQ ID NO:21244 |
| **iPS:433955** | **21-225_45B8** | NA | GACTGTGTCATGCAC | GTTATATGGTATGATGGAAGTAATAAATACTATGCTGACTCCGTGAAGGGC | GAAAGGTATAGCAGTGGCTTGTACGACTACGGTATGGACGTC |
| | | | SEQ ID NO:5221 | SEQ ID NO:13233 | SEQ ID NO:21245 |
| | | AA | DCVMH | VIWYDGSNKYYADSVKG | ERYSSGLYDYGMDV |
| | | | SEQ ID NO:5222 | SEQ ID NO:13234 | SEQ ID NO:21246 |

FIGURE 49

| | | | | | |
|---|---|---|---|---|---|
| **iPS:433957** | **21-225_45F8** | NA | AGCTATGCCATGAGC | GCTATTAGTGGTCGTGGTGTTAACACATTCGACGCAGACTCCGTGAAGGGC | GAAAGGAGTGGGAGCTATTTTGACTAC |
| | | | SEQ ID NO:5223 | SEQ ID NO:13235 | SEQ ID NO:21247 |
| | | AA | SYAMS | AISGRGVNTFDADSVKG | ERSGSYFDY |
| | | | SEQ ID NO:5224 | SEQ ID NO:13236 | SEQ ID NO:21248 |
| **iPS:433959** | **21-225_45C9** | NA | AGCTATGCCATGAGC | GTTATTAGTGGTCGTGGTGGTACCACATACTACGCAGACTCCGTGAAGGGC | CGAACGCCCTCTGATGCTTTTGATATC |
| | | | SEQ ID NO:5225 | SEQ ID NO:13237 | SEQ ID NO:21249 |
| | | AA | SYAMS | VISGRGGTTYYADSVKG | RTPSDAFDI |
| | | | SEQ ID NO:5226 | SEQ ID NO:13238 | SEQ ID NO:21250 |
| **iPS:433961** | **21-225_45D9** | NA | AGCTATACCATGAAC | TCCATTAGTGGAAGTAGTACTTACATATACTACGCAGACTCAGTGAAGGGC | GTGACCTCTTTTGACTAC |
| | | | SEQ ID NO:5227 | SEQ ID NO:13239 | SEQ ID NO:21251 |
| | | AA | SYTMN | SISGSSTYIYYADSVKG | VTSFDY |
| | | | SEQ ID NO:5228 | SEQ ID NO:13240 | SEQ ID NO:21252 |
| **iPS:433963** | **21-225_46B1** | NA | AGCGATGACTCGCAC | GTTATATGGTTTGATGAATATACTAAATACTATGCAGACTCCGTGAAGGGC | GATCTAATAGCAGCAACTGGGACGGGAGACTAC |
| | | | SEQ ID NO:5229 | SEQ ID NO:13241 | SEQ ID NO:21253 |
| | | AA | SDDSH | VIWFDEYTKYYADSVKG | DLIAATGTGDY |
| | | | SEQ ID NO:5230 | SEQ ID NO:13242 | SEQ ID NO:21254 |
| **iPS:433965** | **21-225_46F2** | NA | AGCTATGGCATGCAC | ATTATATGGTATGATGGAAGTAATAAATACTATGTAGACTCCGTGAAGGGC | GATCGATACGATTTTTGGAGTGGTTACTTTGACTAC |
| | | | SEQ ID NO:5231 | SEQ ID NO:13243 | SEQ ID NO:21255 |
| | | AA | SYGMH | IIWYDGSNKYYVDSVKG | DRYDFWSGYFDY |

FIGURE 49

| | | | | | |
|---|---|---|---|---|---|
| | | | SEQ ID NO:5232 | SEQ ID NO:13244 | SEQ ID NO:21256 |
| **iPS:433967** | **21-225_46C3** | NA | AGCTATGTCATGCAC | GTTATATGGTATGATGGAAGTAATAAATACTATGCTGACTCCGTGAAGGGC | GAAAGGTATAGCAGTGGCTTGTACGACTACGGTATGGACGTC |
| | | | SEQ ID NO:5233 | SEQ ID NO:13245 | SEQ ID NO:21257 |
| | | AA | SYVMH | VIWYDGSNKYYADSVKG | ERYSSGLYDYGMDV |
| | | | SEQ ID NO:5234 | SEQ ID NO:13246 | SEQ ID NO:21258 |
| **iPS:433969** | **21-225_46F3** | NA | GATTATGGCATGCAC | GTTATATGGTTTGAAGGAAGTAATAAATACTATGCAGATTCCGTGAAGGGC | GAACTAGGATTTTCCAATGACTAC |
| | | | SEQ ID NO:5235 | SEQ ID NO:13247 | SEQ ID NO:21259 |
| | | AA | DYGMH | VIWFEGSNKYYADSVKG | ELGFSNDY |
| | | | SEQ ID NO:5236 | SEQ ID NO:13248 | SEQ ID NO:21260 |
| **iPS:433971** | **21-225_46D4** | NA | AGCTATGTCATGCAC | GTTATATGGTATGATGGAAGTAATAAATACTATGCAGACTCCGTGAAGGGC | GTCCCGTATAGCAGCAGTTGGTACGACTACGGTATGGACGTC |
| | | | SEQ ID NO:5237 | SEQ ID NO:13249 | SEQ ID NO:21261 |
| | | AA | SYVMH | VIWYDGSNKYYADSVKG | VPYSSSWYDYGMDV |
| | | | SEQ ID NO:5238 | SEQ ID NO:13250 | SEQ ID NO:21262 |
| **iPS:433973** | **21-225_46A6** | NA | AGCTATGCCATGAGC | GCTATTAGTGGTCGTGGTATTAACACATTCGACGCAGACTCCGTGAAGGGC | GAAAGGAGTGGGAGCTATTTTGACTAC |
| | | | SEQ ID NO:5239 | SEQ ID NO:13251 | SEQ ID NO:21263 |
| | | AA | SYAMS | AISGRGINTFDADSVKG | ERSGSYFDY |
| | | | SEQ ID NO:5240 | SEQ ID NO:13252 | SEQ ID NO:21264 |

## FIGURE 49

| | | | | | |
|---|---|---|---|---|---|
| **iPS:433975** | **21-225_46C6** | NA | AGCTATGGCATACAC | GTTATATGGTATGATGAAAATAATAAATACTATGCAGACTCCGTGAAGGGC | GAGCTAGGATTTTCCAATGACTAC |
| | | | SEQ ID NO:5241 | SEQ ID NO:13253 | SEQ ID NO:21265 |
| | | AA | SYGIH | VIWYDENNKYYADSVKG | ELGFSNDY |
| | | | SEQ ID NO:5242 | SEQ ID NO:13254 | SEQ ID NO:21266 |
| **iPS:433977** | **21-225_46D8** | NA | GATTATGGCATACAC | GTTATATGGTTTGAAGGAAGTAATAAATACTATGCAGATTCCGTGAAGGGC | GAACTAGGATTTTCCAATGACTAC |
| | | | SEQ ID NO:5243 | SEQ ID NO:13255 | SEQ ID NO:21267 |
| | | AA | DYGIH | VIWFEGSNKYYADSVKG | ELGFSNDY |
| | | | SEQ ID NO:5244 | SEQ ID NO:13256 | SEQ ID NO:21268 |
| **iPS:433979** | **21-225_46B9** | NA | AGCTATGGCATGCAC | GTTATATGGTATGATGGAAGAAATAAATACTATGCAGACTCCGTGAAGGGC | CGGTATAGCAGCAGCTGGATGGGAGGTATGGACGTC |
| | | | SEQ ID NO:5245 | SEQ ID NO:13257 | SEQ ID NO:21269 |
| | | AA | SYGMH | VIWYDGRNKYYADSVKG | RYSSSWMGGMDV |
| | | | SEQ ID NO:5246 | SEQ ID NO:13258 | SEQ ID NO:21270 |
| **iPS:433981** | **21-225_46E9** | NA | GACTACTACATGAAC | TACATTAATAGTAATGGTTTTACCATATACTACGCAGACTCTGTGAAGGGC | GATACAATCTAC |
| | | | SEQ ID NO:5247 | SEQ ID NO:13259 | SEQ ID NO:21271 |
| | | AA | DYYMN | YINSNGFTIYYADSVKG | DTIY |
| | | | SEQ ID NO:5248 | SEQ ID NO:13260 | SEQ ID NO:21272 |

## FIGURE 49

| | | | | | |
|---|---|---|---|---|---|
| **iPS:433983** | **21-225_47A1** | NA | GACTATGGCATGCAC | GTTATATGGTATGATGACTATAATAAAAAGTATGCAGACTCCGTGAAGGGC | GAACTGGGGATGCTCTTTGACTAC |
| | | | SEQ ID NO:5249 | SEQ ID NO:13261 | SEQ ID NO:21273 |
| | | AA | DYGMH | VIWYDDYNKKYADSVKG | ELGMLFDY |
| | | | SEQ ID NO:5250 | SEQ ID NO:13262 | SEQ ID NO:21274 |
| **iPS:433985** | **21-225_47C1** | NA | GACTATGGCATGCAC | GTTATATGGTATGATGGAAGTAATAAATACTATGCAGACTCCGTGAAGGGC | CGGTATAGCCGCAGCTGGGTGGGAGGTATGGACGTC |
| | | | SEQ ID NO:5251 | SEQ ID NO:13263 | SEQ ID NO:21275 |
| | | AA | DYGMH | VIWYDGSNKYYADSVKG | RYSRSWVGGMDV |
| | | | SEQ ID NO:5252 | SEQ ID NO:13264 | SEQ ID NO:21276 |
| **iPS:433987** | **21-225_47A5** | NA | GACGATGACACACAC | GTTATATGGTTTGATGGAAGTAATAAATACTATGCAGACTCCGTGAAGGGC | GATCTTATAGCAGCAGCTGGTACAGTTGACTAC |
| | | | SEQ ID NO:5253 | SEQ ID NO:13265 | SEQ ID NO:21277 |
| | | AA | DDDTH | VIWFDGSNKYYADSVKG | DLIAAAGTVDY |
| | | | SEQ ID NO:5254 | SEQ ID NO:13266 | SEQ ID NO:21278 |
| **iPS:433989** | **21-225_47C7** | NA | AACTATGCCATGAGC | GGTATTAGTGGTAGTGGTAGTCGCACATACTACGCAGACTCCGTGAAGGGC | GATCGGGGGCAGTGGCTCATAGGCGGTATGGACGTC |
| | | | SEQ ID NO:5255 | SEQ ID NO:13267 | SEQ ID NO:21279 |
| | | AA | NYAMS | GISGSGSRTYYADSVKG | DRGQWLIGGMDV |
| | | | SEQ ID NO:5256 | SEQ ID NO:13268 | SEQ ID NO:21280 |

FIGURE 49

| | | | | | |
|---|---|---|---|---|---|
| **iPS:433991** | **21-225_47E7** | NA | ATCTATGGCATGCAC | GTTATATGGTATGATGGAAGTAATAAATACTATGCAGACTCCGTGAAGGGC | CGGTATAGCAGAAGCTGGGTGGGAGGTATGGACGTC |
| | | | SEQ ID NO:5257 | SEQ ID NO:13269 | SEQ ID NO:21281 |
| | | AA | IYGMH | VIWYDGSNKYYADSVKG | RYSRSWVGGMDV |
| | | | SEQ ID NO:5258 | SEQ ID NO:13270 | SEQ ID NO:21282 |
| **iPS:433993** | **21-225_47G7** | NA | AGCTATGCCATGAGT | GCTATTAGTGGTCGTGGTGGTAACACATTCTACGCAGAGTCCGTGAGGGGC | ATTATCCGGGAGCAGTGGGCCTTTGACTAC |
| | | | SEQ ID NO:5259 | SEQ ID NO:13271 | SEQ ID NO:21283 |
| | | AA | SYAMS | AISGRGGNTFYAESVRG | IIREQWAFDY |
| | | | SEQ ID NO:5260 | SEQ ID NO:13272 | SEQ ID NO:21284 |
| **iPS:433995** | **21-225_47H7** | NA | GACTACTACATGATC | TACATTAATAGTAATGGTTTTACCAAATACTACGCAGACTCTGTGAAGGGC | GATACAGTCTAC |
| | | | SEQ ID NO:5261 | SEQ ID NO:13273 | SEQ ID NO:21285 |
| | | AA | DYYMI | YINSNGFTKYYADSVKG | DTVY |
| | | | SEQ ID NO:5262 | SEQ ID NO:13274 | SEQ ID NO:21286 |
| **iPS:433997** | **21-225_48C1** | NA | AGCTATGGCATGCAC | GTTGTATGGTATGATGAAATTAATAAAAAGTATGCAGACTCCGTGAAGGGC | GAATTAGGGTGGGAGGCTGACTAC |
| | | | SEQ ID NO:5263 | SEQ ID NO:13275 | SEQ ID NO:21287 |
| | | AA | SYGMH | VVWYDEINKKYADSVKG | ELGWEADY |
| | | | SEQ ID NO:5264 | SEQ ID NO:13276 | SEQ ID NO:21288 |

FIGURE 49

| | | | | | |
|---|---|---|---|---|---|
| **iPS:433999** | **21-225_48D1** | NA | AGTTATGCCATGAGC | GTTATTAGTGGTCGTGGTGGTAGCACATTCTACGCAGACTCCGTGAAGGGC | CGTATAGCAGTGGCTGGGAATGAGGCTTTTGATATC |
| | | | SEQ ID NO:5265 | SEQ ID NO:13277 | SEQ ID NO:21289 |
| | | AA | SYAMS | VISGRGGSTFYADSVKG | RIAVAGNEAFDI |
| | | | SEQ ID NO:5266 | SEQ ID NO:13278 | SEQ ID NO:21290 |
| **iPS:434001** | **21-225_48F2** | NA | AACTATGTCATGCAC | GTTATATGGTATGATGGAAGTAATAAATACTATGCAGACTCCGTGAAGGGC | GAGAGGTATAGCAGCAGCTGGTACGACTACGGTCTGGACGTC |
| | | | SEQ ID NO:5267 | SEQ ID NO:13279 | SEQ ID NO:21291 |
| | | AA | NYVMH | VIWYDGSNKYYADSVKG | ERYSSSWYDYGLDV |
| | | | SEQ ID NO:5268 | SEQ ID NO:13280 | SEQ ID NO:21292 |
| **iPS:434003** | **21-225_48C3** | NA | AGTTATGCCATGAGC | GTTATTAGTGGTCGTGGTGGTAGCACATTCTACGCAGACTCCGTGAAGGGC | CGTATAGCAGTGGCTGGGAATGAGGCTTTTGATATC |
| | | | SEQ ID NO:5269 | SEQ ID NO:13281 | SEQ ID NO:21293 |
| | | AA | SYAMS | VISGRGGSTFYADSVKG | RIAVAGNEAFDI |
| | | | SEQ ID NO:5270 | SEQ ID NO:13282 | SEQ ID NO:21294 |
| **iPS:434007** | **21-225_48D7** | NA | AACTCTGCCATGAAC | GCTATTAGTGGTAGTGGTGGTACCACATTCTACGCAGACTCCGTGAAGGGC | TGTGGGCGGGAGCAGTGGCTTGACTAC |
| | | | SEQ ID NO:5271 | SEQ ID NO:13283 | SEQ ID NO:21295 |
| | | AA | NSAMN | AISGSGGTTFYADSVKG | CGREQWLDY |
| | | | SEQ ID NO:5272 | SEQ ID NO:13284 | SEQ ID NO:21296 |
| **iPS:434009** | **21 225 48A9** | NA | AGCTATGGCATGCAC | GTTATATGGTATGAGGAAAATAAGAAATACTATGCAGACTCCGTGAAGGGC | GAACTTGCCTGGTACGAGGACTAC |

FIGURE 49

| | | | | | |
|---|---|---|---|---|---|
| | 21-225_48A9 | | SEQ ID NO:5273 | SEQ ID NO:13285 | SEQ ID NO:21297 |
| | | AA | SYGMH | VIWYEENKKYYADSVKG | ELAWYEDY |
| | | | SEQ ID NO:5274 | SEQ ID NO:13286 | SEQ ID NO:21298 |
| **iPS:434011** | 21-225_48B10 | NA | GACTACTTCATGACC | TACATTAGTAGTGCTGGTGGTGCCATATACTACGCAGACTCTGTGAAGGGC | GCAGTGGCTGCCCCTGGTGTTTTTGATATC |
| | | | SEQ ID NO:5275 | SEQ ID NO:13287 | SEQ ID NO:21299 |
| | | AA | DYFMT | YISSAGGAIYYADSVKG | AVAAPGVFDI |
| | | | SEQ ID NO:5276 | SEQ ID NO:13288 | SEQ ID NO:21300 |
| **iPS:434013** | 21-225_48D12 | NA | GACTATGGCATGCAC | GTTATATGGTATGATGTAAGTAATAAATACTATGTAGACTCCGTGAAGGGC | GAACTGGGGATGAGATCTGACTAC |
| | | | SEQ ID NO:5277 | SEQ ID NO:13289 | SEQ ID NO:21301 |
| | | AA | DYGMH | VIWYDVSNKYYVDSVKG | ELGMRSDY |
| | | | SEQ ID NO:5278 | SEQ ID NO:13290 | SEQ ID NO:21302 |
| **iPS:434015** | 21-225_48F12 | NA | GACTACTTCATGACC | TACATTAGTAGTGCTGGTGGTGCCATATACTACGCAGACTCTGTGAAGGGC | GCAGTGGCTGCCCCTGGTGCTTTTGATATC |
| | | | SEQ ID NO:5279 | SEQ ID NO:13291 | SEQ ID NO:21303 |
| | | AA | DYFMT | YISSAGGAIYYADSVKG | AVAAPGAFDI |
| | | | SEQ ID NO:5280 | SEQ ID NO:13292 | SEQ ID NO:21304 |
| **iPS:434017** | 21-225_48G12 | NA | GACTACTTCATGACC | TACATTAGTAGTGCTGGTGGTGCCATATACTACGCAGACTCTGTGAAGGGC | GCAGTGGCTGCCCCTGGTGCTTTTGATATC |
| | | | SEQ ID NO:5281 | SEQ ID NO:13293 | SEQ ID NO:21305 |
| | | AA | DYFMT | YISSAGGAIYYADSVKG | AVAAPGAFDI |
| | | | SEQ ID NO:5282 | SEQ ID NO:13294 | SEQ ID NO:21306 |

**FIGURE 49**

| | | | | | |
|---|---|---|---|---|---|
| **iPS:434019** | **21-225_49A1** | NA | GACTATGGCATGCAC | GTTATATGGTATGATGAAGATAATAAATATTATGTAGACTCCGTGAAGGGC | GAACTGGGGTTCCTCTCTGACTAC |
| | | | SEQ ID NO:5283 | SEQ ID NO:13295 | SEQ ID NO:21307 |
| | | AA | DYGMH | VIWYDEDNKYYVDSVKG | ELGFLSDY |
| | | | SEQ ID NO:5284 | SEQ ID NO:13296 | SEQ ID NO:21308 |
| **iPS:434021** | **21-225_49C1** | NA | AGCTATGGCATGCAC | GTTATATGGTATGATGGAAGTAATAAATACTATGCAGACTCCGTGAAGGGC | AGGTATAGCAGCAGCTGGTCGGGCGGTATGGACGTC |
| | | | SEQ ID NO:5285 | SEQ ID NO:13297 | SEQ ID NO:21309 |
| | | AA | SYGMH | VIWYDGSNKYYADSVKG | RYSSSWSGGMDV |
| | | | SEQ ID NO:5286 | SEQ ID NO:13298 | SEQ ID NO:21310 |
| **iPS:434023** | **21-225_49F1** | NA | AGCTATGCCATGAGC | GTTATTAGTGGTAGTGGTGGTAGCACATTCTACGCAGACTCCGTGAAGGGC | GCTATAGCAGCGGCTGGTGCCCACTATTTTGACTAC |
| | | | SEQ ID NO:5287 | SEQ ID NO:13299 | SEQ ID NO:21311 |
| | | AA | SYAMS | VISGSGGSTFYADSVKG | AIAAAGAHYFDY |
| | | | SEQ ID NO:5288 | SEQ ID NO:13300 | SEQ ID NO:21312 |
| **iPS:434025** | **21-225_49G3** | NA | AGTTATGGCATGCAC | GTTATATGGTATGATGGAAGTAATAAATACTATGCAGACTCCGTGAAGGGC | AGGTATAGCAGCAGCTGGTCGGGCGGAATGGACGTC |
| | | | SEQ ID NO:5289 | SEQ ID NO:13301 | SEQ ID NO:21313 |
| | | AA | SYGMH | VIWYDGSNKYYADSVKG | RYSSSWSGGMDV |
| | | | SEQ ID NO:5290 | SEQ ID NO:13302 | SEQ ID NO:21314 |

FIGURE 49

| | | | | | |
|---|---|---|---|---|---|
| **iPS:434027** | **21-225_49H5** | NA | AGCTATGCCATGACC | GCTATTAGTGGTAGTGGTGGTAACTCATTCTACGCAGACTCCGTGAAGGGC | GCAAGGGCAGTGGCTGGGTCACACTGGTTCGACCCC |
| | | | SEQ ID NO:5291 | SEQ ID NO:13303 | SEQ ID NO:21315 |
| | | AA | SYAMT | AISGSGGNSFYADSVKG | ARAVAGSHWFDP |
| | | | SEQ ID NO:5292 | SEQ ID NO:13304 | SEQ ID NO:21316 |
| **iPS:434029** | **21-225_49C6** | NA | AACTATGGCATGCAC | GTTATATGGTTTGATGTAAGTAATAAAAAGTATGTAGACTCCGTGAAGGGC | GATCTGGGGATGATCGAGGACTAC |
| | | | SEQ ID NO:5293 | SEQ ID NO:13305 | SEQ ID NO:21317 |
| | | AA | NYGMH | VIWFDVSNKKYVDSVKG | DLGMIEDY |
| | | | SEQ ID NO:5294 | SEQ ID NO:13306 | SEQ ID NO:21318 |
| **iPS:434031** | **21-225_49E7** | NA | AGCTATGGCATGCAC | GTTATATGGTATGATGGAAGTAATAAATACTATGCAGACTCCGTGAAGGGC | AGGTATAGCAGCAGCTGGTCGGGCGGTATGGACGTC |
| | | | SEQ ID NO:5295 | SEQ ID NO:13307 | SEQ ID NO:21319 |
| | | AA | SYGMH | VIWYDGSNKYYADSVKG | RYSSSWSGGMDV |
| | | | SEQ ID NO:5296 | SEQ ID NO:13308 | SEQ ID NO:21320 |
| **iPS:434033** | **21-225_49F9** | NA | AGTTATGGCATGCAC | CTTATATGGTATGATGGAAGGAATAAATACTATGCAGACTCCGTGAAGGGC | AGGTATAGCAGCAGCTGGTCGGGCGGTATGGACGTC |
| | | | SEQ ID NO:5297 | SEQ ID NO:13309 | SEQ ID NO:21321 |
| | | AA | SYGMH | LIWYDGRNKYYADSVKG | RYSSSWSGGMDV |
| | | | SEQ ID NO:5298 | SEQ ID NO:13310 | SEQ ID NO:21322 |

FIGURE 49

| | | | | | |
|---|---|---|---|---|---|
| **iPS:434035** | **21-225_49F10** | NA | GGCTACCATATGCAC | TGGATCAACCCTAATAACAATGCCACAAACTATGCTCAGAACTTTCAGGGC | GACGGTACCAGCAGCTTTGACTTC |
| | | | SEQ ID NO:5299 | SEQ ID NO:13311 | SEQ ID NO:21323 |
| | | AA | GYHMH | WINPNNNATNYAQNFQG | DGTSSFDF |
| | | | SEQ ID NO:5300 | SEQ ID NO:13312 | SEQ ID NO:21324 |
| **iPS:434037** | **21-225_49G12** | NA | AGCTATGCCATGAGC | GTTATTAGTGGTAGTGGTGGTACCACATTCTACGCAGACTCCGTGAAGGGC | CGTATAGCAGTGGCTGGGAATGATGCTTTTGATATC |
| | | | SEQ ID NO:5301 | SEQ ID NO:13313 | SEQ ID NO:21325 |
| | | AA | SYAMS | VISGSGGTTFYADSVKG | RIAVAGNDAFDI |
| | | | SEQ ID NO:5302 | SEQ ID NO:13314 | SEQ ID NO:21326 |
| **iPS:434039** | **21-225_43B1** | NA | GACTACTACATGAAC | TACATTAATAGTAATGGTTTTACCATATACTACGCAGACTCTGTGAAGGGC | GATACAATCTAC |
| | | | SEQ ID NO:5303 | SEQ ID NO:13315 | SEQ ID NO:21327 |
| | | AA | DYYMN | YINSNGFTIYYADSVKG | DTIY |
| | | | SEQ ID NO:5304 | SEQ ID NO:13316 | SEQ ID NO:21328 |
| **iPS:434041** | **21-225_50H8** | NA | AGTTATGCCATGAGC | GTTATTAGTGGTCGTGGTGGTACCACATTCTACGCAGACTCCGTGAAGGGC | CGTATAGCAGTGGCTGGGAATGAGGCTTTTGATATC |
| | | | SEQ ID NO:5305 | SEQ ID NO:13317 | SEQ ID NO:21329 |
| | | AA | SYAMS | VISGRGGTTFYADSVKG | RIAVAGNEAFDI |
| | | | SEQ ID NO:5306 | SEQ ID NO:13318 | SEQ ID NO:21330 |
| **iPS:434043** | **21-225_50G10** | NA | AGCTATAGCATGAAC | TCCATTAGTGGTAGTAGTAGTTACATATACTACGCAGACTCAGTGAAGGGC | GTAGCAACTTTTGACTAC |
| | | | SEQ ID NO:5307 | SEQ ID NO:13319 | SEQ ID NO:21331 |

# FIGURE 49

| | | | | | |
|---|---|---|---|---|---|
| | | AA | SYSMN | SISGSSSYIYYADSVKG | VATFDY |
| | | | SEQ ID NO:5308 | SEQ ID NO:13320 | SEQ ID NO:21332 |
| **iPS:434045** | **21-225_50H10** | NA | AGTTATGCCATGAGC | GTTATTAGTGGTCGTGGTGGTAGCACATTCTACGCAGACTCCGTGAAGGGC | CGTATAGCAGTGGCTGGGAATGAGGCTTTTGATATC |
| | | | SEQ ID NO:5309 | SEQ ID NO:13321 | SEQ ID NO:21333 |
| | | AA | SYAMS | VISGRGGSTFYADSVKG | RIAVAGNEAFDI |
| | | | SEQ ID NO:5310 | SEQ ID NO:13322 | SEQ ID NO:21334 |
| **iPS:434047** | **21-225_50A12** | NA | GGCCACTATATAAAC | TGGGTCAACCCTAACAGTGGTGGCACAAACTCTGCACAGAAGTTTCAGGGC | GGAGGGCAGCTCGGCGGGTTTAACTACTACTACTACGGTATGGACGTC |
| | | | SEQ ID NO:5311 | SEQ ID NO:13323 | SEQ ID NO:21335 |
| | | AA | GHYIN | WVNPNSGGTNSAQKFQG | GGQLGGFNYYYYGMDV |
| | | | SEQ ID NO:5312 | SEQ ID NO:13324 | SEQ ID NO:21336 |
| **iPS:434049** | **21-225_50B12** | NA | AGCCATAGCATGAAC | TCCATCAGTAGTAGTAGTAATTACATATACTACGCAGACTCAGTGAAGGGC | GATCGGAGCATAGTAGTGGCTGGTCCCTGGGACTACTACGGTATGGACGTC |
| | | | SEQ ID NO:5313 | SEQ ID NO:13325 | SEQ ID NO:21337 |
| | | AA | SHSMN | SISSSSNYIYYADSVKG | DRSIVVAGPWDYYGMDV |
| | | | SEQ ID NO:5314 | SEQ ID NO:13326 | SEQ ID NO:21338 |
| **iPS:434053** | **21-225_51E1** | NA | AGCTATGGCATGCAC | GTTATATGGTATGATGGAAGTAGTAAATACTATGCAGACTCCGTGAAGGGC | AGGTATAGCAGCAGTTGGTCGGGCGGTATGGACGTC |
| | | | SEQ ID NO:5315 | SEQ ID NO:13327 | SEQ ID NO:21339 |
| | | AA | SYGMH | VIWYDGSSKYYADSVKG | RYSSSWSGGMDV |
| | | | SEQ ID NO:5316 | SEQ ID NO:13328 | SEQ ID NO:21340 |
| **iPS:434055** | **21 225 51B4** | NA | AGCTATGTCATGAGC | GCTATTAGTGGTCGTGGTAGTAACACATTCTACACAGACTCCGTGAAGGGC | GGGATAACTGGATCACACGGTGCTTTTGATATC |

FIGURE 49

| | | | | | |
|---|---|---|---|---|---|
| | 21-225_51B4 | | SEQ ID NO:5317 | SEQ ID NO:13329 | SEQ ID NO:21341 |
| | | AA | SYVMS | AISGRGSNTFYTDSVKG | GITGSHGAFDI |
| | | | SEQ ID NO:5318 | SEQ ID NO:13330 | SEQ ID NO:21342 |
| iPS:434057 | 21-225_51E4 | NA | AGCTATGGCATGCAC | GTTATATGGTATGATGA AAGTAATAAATACTATG CAGACTCCGTGAAGGGC | GAACTGGGATTTCTCTCTGA CTAC |
| | | | SEQ ID NO:5319 | SEQ ID NO:13331 | SEQ ID NO:21343 |
| | | AA | SYGMH | VIWYDESNKYYADSVKG | ELGFLSDY |
| | | | SEQ ID NO:5320 | SEQ ID NO:13332 | SEQ ID NO:21344 |
| iPS:434059 | 21-225_51C5 | NA | AGCTATGTCATGAGC | ACTATGAGTGGTAGTGG TGGTCGCACATACTACG CAGACTCCGTGAACGGC | GTGACTGCTTTTGACTAC |
| | | | SEQ ID NO:5321 | SEQ ID NO:13333 | SEQ ID NO:21345 |
| | | AA | SYVMS | TMSGSGGRTYYADSVNG | VTAFDY |
| | | | SEQ ID NO:5322 | SEQ ID NO:13334 | SEQ ID NO:21346 |
| iPS:434061 | 21-225_51C7 | NA | AACTATGCCATGACC | GTTATTAGTGCTAGTGGT GGTAACTCATTCTACGCA GACTCCGTGAAGGGC | GCAAGGGCAGTGGCTGGGTC ACACTGGTTCGACCCC |
| | | | SEQ ID NO:5323 | SEQ ID NO:13335 | SEQ ID NO:21347 |
| | | AA | NYAMT | VISASGGNSFYADSVKG | ARAVAGSHWFDP |
| | | | SEQ ID NO:5324 | SEQ ID NO:13336 | SEQ ID NO:21348 |
| iPS:434063 | 21-225_51G7 | NA | AGCTATAGCATGAAC | TCCATTAGTAGTAGTAGT AGTTACATATACTACGC AGACTCAGTGAAGGGC | GCTCGCCTTGACTAC |
| | | | SEQ ID NO:5325 | SEQ ID NO:13337 | SEQ ID NO:21349 |
| | | AA | SYSMN | SISSSSYIYYADSVKG | ARLDY |
| | | | SEQ ID NO:5326 | SEQ ID NO:13338 | SEQ ID NO:21350 |

FIGURE 49

| | | | | | |
|---|---|---|---|---|---|
| **iPS:434065** | **21-225_50D4** | NA | GGCTACCATATGCAC | TGGATCAACCCTAATAATAATGCCACAAACTATGCTCAGAGCTTTCAGGGC | GACGGTACCAGCAGCTTTGACTTC |
| | | | SEQ ID NO:5327 | SEQ ID NO:13339 | SEQ ID NO:21351 |
| | | AA | GYHMH | WINPNNNATNYAQSFQG | DGTSSFDF |
| | | | SEQ ID NO:5328 | SEQ ID NO:13340 | SEQ ID NO:21352 |
| **iPS:434067** | **21-225_51H8** | NA | GGCCACTATATGAAC | TGGGTCAACCCTAACAGTGGTGGCTCAAACTCTGCACAGCAGTTTCAGGGC | GGAGGGCAGCTCGGCGGCTTTAACTTCTACTACTACGGTATGGACGTC |
| | | | SEQ ID NO:5329 | SEQ ID NO:13341 | SEQ ID NO:21353 |
| | | AA | GHYMN | WVNPNSGGSNSAQQFQG | GGQLGGFNFYYYGMDV |
| | | | SEQ ID NO:5330 | SEQ ID NO:13342 | SEQ ID NO:21354 |
| **iPS:434069** | **21-225_51E9** | NA | GGCTACCATATACAC | TGGATCAACCCTAACACTAATGGCACACAGTATGCACAGAAGTTTCAGGGC | GATGGCACCTCGTCCTTTGACTAC |
| | | | SEQ ID NO:5331 | SEQ ID NO:13343 | SEQ ID NO:21355 |
| | | AA | GYHIH | WINPNTNGTQYAQKFQG | DGTSSFDY |
| | | | SEQ ID NO:5332 | SEQ ID NO:13344 | SEQ ID NO:21356 |
| **iPS:434071** | **21-225_51F9** | NA | GACTATGGCATGCAC | GTTATATGGTTTGATGGAAATAATAAATACTATGCAGACTCCGTGAAGGGC | GAGCTGGGATTTCTCTCTGACTAC |
| | | | SEQ ID NO:5333 | SEQ ID NO:13345 | SEQ ID NO:21357 |
| | | AA | DYGMH | VIWFDGNNKYYADSVKG | ELGFLSDY |
| | | | SEQ ID NO:5334 | SEQ ID NO:13346 | SEQ ID NO:21358 |
| **iPS:434073** | **21-225_51H10** | NA | AGCTATGCCATGAGC | GTTATTAGTGGTAGTGGTGGTACCACATTCTACGCAGACTCCGTGAAGGGC | CGTATAGCAGTGGCTGGGAATGATGCTTTTGATATC |
| | | | SEQ ID NO:5335 | SEQ ID NO:13347 | SEQ ID NO:21359 |

**FIGURE 49**

| | | | | | |
|---|---|---|---|---|---|
| | | AA | SYAMS | VISGSGGTTFYADSVKG | RIAVAGNDAFDI |
| | | | SEQ ID NO:5336 | SEQ ID NO:13348 | SEQ ID NO:21360 |
| **iPS:434075** | **21-225_51B11** | NA | GACTATGGCATGCAC | GTTATATGGTTTGGTGGAAATAATAAATACTATGGAGACTCCGTGAAGGGC | GAGCTGGGATTTCTCTCTGACTAC |
| | | | SEQ ID NO:5337 | SEQ ID NO:13349 | SEQ ID NO:21361 |
| | | AA | DYGMH | VIWFGGNNKYYGDSVKG | ELGFLSDY |
| | | | SEQ ID NO:5338 | SEQ ID NO:13350 | SEQ ID NO:21362 |
| **iPS:434077** | **21-225_51F11** | NA | AACTTTGGCATGCAC | GTTATATGGTATGAGGAAAGTAATAAATACTATGCAGACTCCGTGAAGGGC | GAACTGGGGTTCCTCTCTGACTTC |
| | | | SEQ ID NO:5339 | SEQ ID NO:13351 | SEQ ID NO:21363 |
| | | AA | NFGMH | VIWYEESNKYYADSVKG | ELGFLSDF |
| | | | SEQ ID NO:5340 | SEQ ID NO:13352 | SEQ ID NO:21364 |
| **iPS:434079** | **21-225_52B1** | NA | GGCTATCATATGCAG | TGGATCAACCCTAACAGTGGTGCCACAAACTATGCACAGAACTTTCAGGGC | GATGGCACCTCGTCCTTTGACTAC |
| | | | SEQ ID NO:5341 | SEQ ID NO:13353 | SEQ ID NO:21365 |
| | | AA | GYHMQ | WINPNSGATNYAQNFQG | DGTSSFDY |
| | | | SEQ ID NO:5342 | SEQ ID NO:13354 | SEQ ID NO:21366 |
| **iPS:434081** | **21-225_52B2** | NA | AACTATGGCATGCAC | GTTACATGGTTTGATGGAAGTAATCAACGCTATGCAGACTCCGTGAAGGGC | GATCTGGGGATGATCGAGGACTTC |
| | | | SEQ ID NO:5343 | SEQ ID NO:13355 | SEQ ID NO:21367 |
| | | AA | NYGMH | VTWFDGSNQRYADSVKG | DLGMIEDF |
| | | | SEQ ID NO:5344 | SEQ ID NO:13356 | SEQ ID NO:21368 |

FIGURE 49

| | | | | | |
|---|---|---|---|---|---|
| **iPS:434083** | **21-225_52H2** | NA | AGAAATGCCATGAGC | GCTATTAGTGGTCGTGGTGGTAATACATTCTACGCAGACTCCGTGAAGGGC | AATGGGCGAGAGCAGTGGCTTGACTAC |
| | | | SEQ ID NO:5345 | SEQ ID NO:13357 | SEQ ID NO:21369 |
| | | AA | RNAMS | AISGRGGNTFYADSVKG | NGREQWLDY |
| | | | SEQ ID NO:5346 | SEQ ID NO:13358 | SEQ ID NO:21370 |
| **iPS:434085** | **21-225_52E3** | NA | AGCTATAAAATGAAC | TCCATTAGTAGTGGTAATAGTTCCATATACTACGCAGACTCAGTGAAGGGC | GTTAGCAGTAATGACTAC |
| | | | SEQ ID NO:5347 | SEQ ID NO:13359 | SEQ ID NO:21371 |
| | | AA | SYKMN | SISSGNSSIYYADSVKG | VSSNDY |
| | | | SEQ ID NO:5348 | SEQ ID NO:13360 | SEQ ID NO:21372 |
| **iPS:434087** | **21-225_52F6** | NA | AGCTATGGCATGCAC | ATTATATCATATGGAGGAAGTAATAAATACTATGCAGACTCCGTGAAGGGC | AGGTCAGCAGCTCGGCCGGGCTACGGTATGGACGTC |
| | | | SEQ ID NO:5349 | SEQ ID NO:13361 | SEQ ID NO:21373 |
| | | AA | SYGMH | IISYGGSNKYYADSVKG | RSAARPGYGMDV |
| | | | SEQ ID NO:5350 | SEQ ID NO:13362 | SEQ ID NO:21374 |
| **iPS:434091** | **21-225_52B9** | NA | GACTATGGCATGCAC | GTTATATGGTTTGATGGAAATAATAAATACTATGCAGACTCCGTGAAGGGC | GAGCTGGGATTTCTCTCTGACTAC |
| | | | SEQ ID NO:5351 | SEQ ID NO:13363 | SEQ ID NO:21375 |
| | | AA | DYGMH | VIWFDGNNKYYADSVKG | ELGFLSDY |
| | | | SEQ ID NO:5352 | SEQ ID NO:13364 | SEQ ID NO:21376 |

## FIGURE 49

| | | | | | |
|---|---|---|---|---|---|
| **iPS:434093** | **21-225_52D10** | NA | AGTTATGGCATGCAC | CTTATATGGTATGATGGAAGTAATAAATACCATGCAGACTCCGTGAAGGGC | AGGTATAGCAGCAGCTGGTCGGGCGGTATGGACGTC |
| | | | SEQ ID NO:5353 | SEQ ID NO:13365 | SEQ ID NO:21377 |
| | | AA | SYGMH | LIWYDGSNKYHADSVKG | RYSSSWSGGMDV |
| | | | SEQ ID NO:5354 | SEQ ID NO:13366 | SEQ ID NO:21378 |
| **iPS:434095** | **21-225_52F10** | NA | TTCTATGGCATGCAC | GTTATATGGGACGATGGAAGTAATAAATACTATGCAGACTCCGTGAAGGGC | GATTCTCTGTATAGCAGCAGCTGGTTGTTTGACTAC |
| | | | SEQ ID NO:5355 | SEQ ID NO:13367 | SEQ ID NO:21379 |
| | | AA | FYGMH | VIWDDGSNKYYADSVKG | DSLYSSSWLFDY |
| | | | SEQ ID NO:5356 | SEQ ID NO:13368 | SEQ ID NO:21380 |
| **iPS:434097** | **21-225_52H10** | NA | GGCTACCATATGCAG | TGGATCAACCCTAACAATGGTGGCACACAGTATGCACAGAAGTTTCAGGGC | GATGGCACCTCGTCCTTTGACTAC |
| | | | SEQ ID NO:5357 | SEQ ID NO:13369 | SEQ ID NO:21381 |
| | | AA | GYHMQ | WINPNNGGTQYAQKFQG | DGTSSFDY |
| | | | SEQ ID NO:5358 | SEQ ID NO:13370 | SEQ ID NO:21382 |
| **iPS:434101** | **21-225_52H12** | NA | AGCTATAGCATGAAC | TCCATTAGTGGTAGTAGTACTTACATATACTACGCAGACTCAGTGAAGGGC | GTCAACTCCTTTGACTAC |
| | | | SEQ ID NO:5359 | SEQ ID NO:13371 | SEQ ID NO:21383 |
| | | AA | SYSMN | SISGSSTYIYYADSVKG | VNSFDY |
| | | | SEQ ID NO:5360 | SEQ ID NO:13372 | SEQ ID NO:21384 |

**FIGURE 49**

| | | | | | |
|---|---|---|---|---|---|
| **iPS:434103** | **21-225_53G1** | NA | AGCTATAGCATGAAC | TCCATTAGTGGTAGTAGT AGTTACATATACTACGC AGACTCAGTGAAGGGC | GATCGGGGCAGCACC |
| | | | SEQ ID NO:5361 | SEQ ID NO:13373 | SEQ ID NO:21385 |
| | | AA | SYSMN | SISGSSSYIYYADSVKG | DRGST |
| | | | SEQ ID NO:5362 | SEQ ID NO:13374 | SEQ ID NO:21386 |
| **iPS:434105** | **21-225_53D2** | NA | AACTATGGCATGCAC | GTTGTATGGGATGATGG AAGTAATAAATACTATG CAGACTCCGTGAAGGGC | GGCCTTGGCTTTACGGGAGA CTAC |
| | | | SEQ ID NO:5363 | SEQ ID NO:13375 | SEQ ID NO:21387 |
| | | AA | NYGMH | VVWDDGSNKYYADSVKG | GLGFTGDY |
| | | | SEQ ID NO:5364 | SEQ ID NO:13376 | SEQ ID NO:21388 |
| **iPS:434107** | **21-225_53E2** | NA | AGCTATGCCATGAGC | GCTATTAGTGGTAGTGGT GGTAACACATTCTACGC AGACTCCGTGAAGGGC | AAGGTCGTGGATACAGCCAT GGCTCTTGACTAC |
| | | | SEQ ID NO:5365 | SEQ ID NO:13377 | SEQ ID NO:21389 |
| | | AA | SYAMS | AISGSGGNTFYADSVKG | KVVDTAMALDY |
| | | | SEQ ID NO:5366 | SEQ ID NO:13378 | SEQ ID NO:21390 |
| **iPS:434111** | **21-225_53H2** | NA | AGCTATGGCATGCAC | GTTATATCATATGGTGGA AGTAATAAATACCATGC AGACTCCGTGAAGGGC | CGGGGAGCAGCTCGTCCTGG CTACGGTATGGACGTC |
| | | | SEQ ID NO:5367 | SEQ ID NO:13379 | SEQ ID NO:21391 |
| | | AA | SYGMH | VISYGGSNKYHADSVKG | RGAARPGYGMDV |
| | | | SEQ ID NO:5368 | SEQ ID NO:13380 | SEQ ID NO:21392 |
| **iPS:434115** | **21 225 53E4** | NA | AGCTATGTCATGAGC | GGTATTAGTGGTAGTGG TGGTCGCACATACTACG CAGACTCCGTGAAGGGC | GTGGCCCTTTTTGACTAC |

FIGURE 49

| | | | | | |
|---|---|---|---|---|---|
| | **21-225_53E4** | | SEQ ID NO:5369 | SEQ ID NO:13381 | SEQ ID NO:21393 |
| | | AA | SYVMS | GISGSGGRTYYADSVKG | VALFDY |
| | | | SEQ ID NO:5370 | SEQ ID NO:13382 | SEQ ID NO:21394 |
| **iPS:434117** | **21-225_53C6** | NA | AGCTATGCCATGAAC | GCTATTAGTGGTAGTGGTGGTGCCACATACTACGCAGACTCCGTGAAGGGC | CCTCTAGTGGGAGCCCATGATGCTTTTGAAATC |
| | | | SEQ ID NO:5371 | SEQ ID NO:13383 | SEQ ID NO:21395 |
| | | AA | SYAMN | AISGSGGATYYADSVKG | PLVGAHDAFEI |
| | | | SEQ ID NO:5372 | SEQ ID NO:13384 | SEQ ID NO:21396 |
| **iPS:434119** | **21-225_53E6** | NA | GACTATGGCATCCAC | GTTATATGGTATGATGAAAGTAATAAATACTATGGAGACTCCGTGAAGGGC | GAACTGGGGATGACGTCTGACTAC |
| | | | SEQ ID NO:5373 | SEQ ID NO:13385 | SEQ ID NO:21397 |
| | | AA | DYGIH | VIWYDESNKYYGDSVKG | ELGMTSDY |
| | | | SEQ ID NO:5374 | SEQ ID NO:13386 | SEQ ID NO:21398 |
| **iPS:434121** | **21-225_53F6** | NA | AGCTATGGCATGCAC | GTTATATCATATGGTGGAAGTAATAAATACGATGCAGACTCCGTGAAGGGC | CGACGGGCAGCTCGTCCAGGGTACGGTATGGACGTC |
| | | | SEQ ID NO:5375 | SEQ ID NO:13387 | SEQ ID NO:21399 |
| | | AA | SYGMH | VISYGGSNKYDADSVKG | RRAARPGYGMDV |
| | | | SEQ ID NO:5376 | SEQ ID NO:13388 | SEQ ID NO:21400 |
| **iPS:434123** | **21-225_53F7** | NA | GGCTACCATATGCAC | TGGATCAACCCTAACAATAACGGCACAAACTATGCACAGAAGTTTCAGGGC | GACGGTACCAGCAGCTTTGACTAC |
| | | | SEQ ID NO:5377 | SEQ ID NO:13389 | SEQ ID NO:21401 |

FIGURE 49

| | | | | | |
|---|---|---|---|---|---|
| | | AA | GYHMH | WINPNNNGTNYAQKFQG | DGTSSFDY |
| | | | SEQ ID NO:5378 | SEQ ID NO:13390 | SEQ ID NO:21402 |
| **iPS:434127** | **21-225_53H8** | NA | AGCTATGGCATGCAC | GTTATATGGTATGATGGAAGTAATAAATACTATGCAGACTCCGTGAAGGGC | GCCCGTATTGGGTACTTTGACTCC |
| | | | SEQ ID NO:5379 | SEQ ID NO:13391 | SEQ ID NO:21403 |
| | | AA | SYGMH | VIWYDGSNKYYADSVKG | ARIGYFDS |
| | | | SEQ ID NO:5380 | SEQ ID NO:13392 | SEQ ID NO:21404 |
| **iPS:434129** | **21-225_53B12** | NA | AACTTTGGCATGCAC | GTTGTATGGTATGATGGAAATAATAGATACTATGCAGACTCCGTGAAGGGC | GAACTGGGATTTCTCTCTGACTTC |
| | | | SEQ ID NO:5381 | SEQ ID NO:13393 | SEQ ID NO:21405 |
| | | AA | NFGMH | VVWYDGNNRYYADSVKG | ELGFLSDF |
| | | | SEQ ID NO:5382 | SEQ ID NO:13394 | SEQ ID NO:21406 |
| **iPS:434131** | **21-225_54D3** | NA | AACTATGGCATGCAC | GTTACATGGTTTGATGGAAATAATAACTACTATGCAGACTCCGTGAAGGGC | GAACTGGGGTTCCTTTCTGATTAT |
| | | | SEQ ID NO:5383 | SEQ ID NO:13395 | SEQ ID NO:21407 |
| | | AA | NYGMH | VTWFDGNNNYYADSVKG | ELGFLSDY |
| | | | SEQ ID NO:5384 | SEQ ID NO:13396 | SEQ ID NO:21408 |
| **iPS:434133** | **21 225 54G3** | NA | ACCTATGCCATGAGT | GCTATTAGTGGTAGTGGTGTTAACACATTCTACGCAGACTCCGTGAAGGGC | CTGGGGAAGGACTACTACTACTACGGTATGGACGTC |

**FIGURE 49**

| | | | | | |
|---|---|---|---|---|---|
| | **21-225_54G3** | | SEQ ID NO:5385 | SEQ ID NO:13397 | SEQ ID NO:21409 |
| | | AA | TYAMS | AISGSGVNTFYADSVKG | LGKDYYYYGMDV |
| | | | SEQ ID NO:5386 | SEQ ID NO:13398 | SEQ ID NO:21410 |
| **iPS:434135** | **21-225_54H3** | NA | AGCTATAGCATGATC | TCCATTAGTGGTACTAGTAGTTACATATACTACGCAGACTCAGTGAAGGGC | ATGACTACAGTAATT |
| | | | SEQ ID NO:5387 | SEQ ID NO:13399 | SEQ ID NO:21411 |
| | | AA | SYSMI | SISGTSSYIYYADSVKG | MTTVI |
| | | | SEQ ID NO:5388 | SEQ ID NO:13400 | SEQ ID NO:21412 |
| **iPS:434137** | **21-225_54D4** | NA | AGCTATGGCATGCAC | GTTATATGGTATGATGGAAGTAATAAATACTATGCAGACTCCGTGAAGGGC | AGGTATAGCAGCAGCTGGTCGGGCGGTATGGACGTC |
| | | | SEQ ID NO:5389 | SEQ ID NO:13401 | SEQ ID NO:21413 |
| | | AA | SYGMH | VIWYDGSNKYYADSVKG | RYSSSWSGGMDV |
| | | | SEQ ID NO:5390 | SEQ ID NO:13402 | SEQ ID NO:21414 |
| **iPS:434141** | **21-225_54C6** | NA | GACTATGGCATCCAC | GTTATATGGTATGATGAAAATAATAAATACTATGCAGACTCCGTGAAGGGC | GAACTGGGGATGACGTCTGACTAC |
| | | | SEQ ID NO:5391 | SEQ ID NO:13403 | SEQ ID NO:21415 |
| | | AA | DYGIH | VIWYDENNKYYADSVKG | ELGMTSDY |
| | | | SEQ ID NO:5392 | SEQ ID NO:13404 | SEQ ID NO:21416 |
| **iPS:434143** | **21-225_54G7** | NA | AACTATGGCATGCAC | GTTATATGGTATGAGGAAAGTAATAAATACTATGGAGACTCCGTGAAGGGC | GAATTGGGGTTCCTCTCTGACTAC |
| | | | SEQ ID NO:5393 | SEQ ID NO:13405 | SEQ ID NO:21417 |

**FIGURE 49**

| | | | | | |
|---|---|---|---|---|---|
| | | AA | NYGMH | VIWYEESNKYYGDSVKG | ELGFLSDY |
| | | | SEQ ID NO:5394 | SEQ ID NO:13406 | SEQ ID NO:21418 |
| **iPS:434145** | **21-225_55B1** | NA | GGCTACTATTTCCAC | TGGATCCACCCTAACAATAATGCCACAAACTATGCACAGAAGTTTCAGGGC | GATGGCAGATCGTCCTTTGACTAC |
| | | | SEQ ID NO:5395 | SEQ ID NO:13407 | SEQ ID NO:21419 |
| | | AA | GYYFH | WIHPNNNATNYAQKFQG | DGRSSFDY |
| | | | SEQ ID NO:5396 | SEQ ID NO:13408 | SEQ ID NO:21420 |
| **iPS:434147** | **21-225_55E1** | NA | AGCTATGCCATGAGC | GCTATTAGTGGTCGTGGTAGTAGCACATTCTACGCAGACTCCGTGAAGGGC | GATCACGGTATAGTGGGAACTATTTACTTTGACTAC |
| | | | SEQ ID NO:5397 | SEQ ID NO:13409 | SEQ ID NO:21421 |
| | | AA | SYAMS | AISGRGSSTFYADSVKG | DHGIVGTIYFDY |
| | | | SEQ ID NO:5398 | SEQ ID NO:13410 | SEQ ID NO:21422 |
| **iPS:434149** | **21-225_55H1** | NA | AGCTATGGCATGCAC | GTTATATGGTATGATGGAAGTAATAAATACTATGCAGACTCCGTGAAGGGC | AGGTATAGCAGCAGCTGGTCGGGCGGTATGGACGTC |
| | | | SEQ ID NO:5399 | SEQ ID NO:13411 | SEQ ID NO:21423 |
| | | AA | SYGMH | VIWYDGSNKYYADSVKG | RYSSSWSGGMDV |
| | | | SEQ ID NO:5400 | SEQ ID NO:13412 | SEQ ID NO:21424 |
| **iPS:434151** | **21-225_55C2** | NA | AGTTATGGCATGCAC | CTTATATGGTATGATGGAAGTAATAAATACCATGCAGACTCCGTGAAGGGC | AGGTATAGCAGCAGCTGGTCGGGCGGTATGGACGTC |
| | | | SEQ ID NO:5401 | SEQ ID NO:13413 | SEQ ID NO:21425 |
| | | AA | SYGMH | LIWYDGSNKYHADSVKG | RYSSSWSGGMDV |

FIGURE 49

| | | | | | |
|---|---|---|---|---|---|
| | | | SEQ ID NO:5402 | SEQ ID NO:13414 | SEQ ID NO:21426 |
| **iPS:434155** | **21-225_55B3** | NA | AGCTATGGCATGCAC | GTTATATGGTTTGATGGAAATAATAAATACTATGAAGACTCCGTGAAGGGC | GAACTGGGATTTCTCTCTGACTAC |
| | | | SEQ ID NO:5403 | SEQ ID NO:13415 | SEQ ID NO:21427 |
| | | AA | SYGMH | VIWFDGNNKYYEDSVKG | ELGFLSDY |
| | | | SEQ ID NO:5404 | SEQ ID NO:13416 | SEQ ID NO:21428 |
| **iPS:434157** | **21-225_55D4** | NA | AGTTATAGGATGAAC | TCCATTAGTAGTAGTAGTAATCACATAGACTACGCAGACTCAGTGAAGGGC | GGGACTGACTAC |
| | | | SEQ ID NO:5405 | SEQ ID NO:13417 | SEQ ID NO:21429 |
| | | AA | SYRMN | SISSSSNHIDYADSVKG | GTDY |
| | | | SEQ ID NO:5406 | SEQ ID NO:13418 | SEQ ID NO:21430 |
| **iPS:434159** | **21-225_55B8** | NA | GACTATGGCATGCAC | GTTATATGGTATGATGAAAATAATAAATACTATGCAGACTCCGTGAAGGGC | GAATGGTTTGACTAC |
| | | | SEQ ID NO:5407 | SEQ ID NO:13419 | SEQ ID NO:21431 |
| | | AA | DYGMH | VIWYDENNKYYADSVKG | EWFDY |
| | | | SEQ ID NO:5408 | SEQ ID NO:13420 | SEQ ID NO:21432 |
| **iPS:434161** | **21-225_55F9** | NA | AGCTATGGCATGCAC | GTTATATGGTATGATGGAAATGGCAAATATTATGCAGACTCCGTGAAGGGC | AGGTATAGCAGCAGCTGGTCGGGCGGTATGGACGTC |
| | | | SEQ ID NO:5409 | SEQ ID NO:13421 | SEQ ID NO:21433 |
| | | AA | SYGMH | VIWYDGNGKYYADSVKG | RYSSSWSGGMDV |
| | | | SEQ ID NO:5410 | SEQ ID NO:13422 | SEQ ID NO:21434 |

**FIGURE 49**

| | | | | | |
|---|---|---|---|---|---|
| **iPS:434163** | **21-225_50H1** | NA | AGCTATGGCATGCAC | ATTATATCATATGGTGGAAGTAATAAATACGATGCAGACTCCGTGAAGGGC | CGACGGGCAGCTCGTCCAGGGTACGGTATGGACGTC |
| | | | SEQ ID NO:5411 | SEQ ID NO:13423 | SEQ ID NO:21435 |
| | | AA | SYGMH | IISYGGSNKYDADSVKG | RRAARPGYGMDV |
| | | | SEQ ID NO:5412 | SEQ ID NO:13424 | SEQ ID NO:21436 |
| **iPS:434165** | **21-225_50F2** | NA | AGCTATGGCATGCAC | GTTATATGGCATGATGGAAGTAATAAATACTATGCAGACTCCGTGAAGGGC | GACGAGCAGCTCGGGACCTTTGACTAC |
| | | | SEQ ID NO:5413 | SEQ ID NO:13425 | SEQ ID NO:21437 |
| | | AA | SYGMH | VIWHDGSNKYYADSVKG | DEQLGTFDY |
| | | | SEQ ID NO:5414 | SEQ ID NO:13426 | SEQ ID NO:21438 |
| **iPS:434167** | **21-225_50F3** | NA | ACCTATGCCATGACC | GCTATCAGTGGTAGTGGTGTTAACTCATTCTACGCAGACTCCGTGAAGGGC | GCAAGGGCAGTGGCTGGGTCACACTGGTTCGACCCC |
| | | | SEQ ID NO:5415 | SEQ ID NO:13427 | SEQ ID NO:21439 |
| | | AA | TYAMT | AISGSGVNSFYADSVKG | ARAVAGSHWFDP |
| | | | SEQ ID NO:5416 | SEQ ID NO:13428 | SEQ ID NO:21440 |
| **iPS:434169** | **21-225_50C4** | NA | GACTATGGCATGCAC | GTTATATGGTATGAAGAAACTAATAAATACTATGCAGACTCCGTGAAGGGC | GAAGTGGGGTTCCTGAATGACTAC |
| | | | SEQ ID NO:5417 | SEQ ID NO:13429 | SEQ ID NO:21441 |
| | | AA | DYGMH | VIWYEETNKYYADSVKG | EVGFLNDY |
| | | | SEQ ID NO:5418 | SEQ ID NO:13430 | SEQ ID NO:21442 |

FIGURE 49

| | | | | | |
|---|---|---|---|---|---|
| **iPS:434171** | **21-225_50G4** | NA | AGTTACTATATACAC | GTAATCAACCCTAGTAATGGTAGAACAAGCTACGCACAGAAGTTCCAGGGC | GATCGAGGAGATGGTTACTACTTCTACTACGGTATGGACGTC |
| | | | SEQ ID NO:5419 | SEQ ID NO:13431 | SEQ ID NO:21443 |
| | | AA | SYYIH | VINPSNGRTSYAQKFQG | DRGDGYYFYYGMDV |
| | | | SEQ ID NO:5420 | SEQ ID NO:13432 | SEQ ID NO:21444 |
| **iPS:434175** | **21-225_55A11** | NA | AGCTATGGCATGCAC | GTTATATCATATGTTGGAAGTACTAAATACTATGCAGACTCCGTGAGGGGC | GGGAGAGGTCGATATAGTGACTACGGTCATGATGCTTTTGATATC |
| | | | SEQ ID NO:5421 | SEQ ID NO:13433 | SEQ ID NO:21445 |
| | | AA | SYGMH | VISYVGSTKYYADSVRG | GRGRYSDYGHDAFDI |
| | | | SEQ ID NO:5422 | SEQ ID NO:13434 | SEQ ID NO:21446 |
| **iPS:434177** | **21-225_56A1** | NA | AATTATGATATCAAC | TGGATGCACCCTAACAGTGGTAACACAGGCTATGCACAGAAGTTCCAGGGC | AGCAGTGGCTGGTACGTTTTTGACTAC |
| | | | SEQ ID NO:5423 | SEQ ID NO:13435 | SEQ ID NO:21447 |
| | | AA | NYDIN | WMHPNSGNTGYAQKFQG | SSGWYVFDY |
| | | | SEQ ID NO:5424 | SEQ ID NO:13436 | SEQ ID NO:21448 |
| **iPS:434179** | **21-225_56F1** | NA | AGCTATAGCATGAAC | TCCATTAGTAGTAGTAGTACTTACATATACTACGGAGACTCAGTGAAGGGC | GATCGGGGCAGCAGC |
| | | | SEQ ID NO:5425 | SEQ ID NO:13437 | SEQ ID NO:21449 |
| | | AA | SYSMN | SISSSSTYIYYGDSVKG | DRGSS |
| | | | SEQ ID NO:5426 | SEQ ID NO:13438 | SEQ ID NO:21450 |
| **iPS:434181** | **21-225_56B2** | NA | AGCTATGCCATGAGC | GCTATTAGTGGTAGTGGTGGTAACACATTCTACGCAGACTCCGTGAAGGGC | AAGGTCGTGGATACAGCCATGGCTCTTGACTAC |
| | | | SEQ ID NO:5427 | SEQ ID NO:13439 | SEQ ID NO:21451 |
| | | AA | SYAMS | AISGSGGNTFYADSVKG | KVVDTAMALDY |

**FIGURE 49**

| | | | | | |
|---|---|---|---|---|---|
| | | | SEQ ID NO:5428 | SEQ ID NO:13440 | SEQ ID NO:21452 |
| **iPS:434187** | **21-225_56A5** | NA | AGCTATAGCATGAAC | TCCATTAGTGGTAGTAGT AGTTACATATACTACGC AGACTCAGTGAAGGGC | GTGGCTACTTTTGACTAC |
| | | | SEQ ID NO:5429 | SEQ ID NO:13441 | SEQ ID NO:21453 |
| | | AA | SYSMN | SISGSSSYIYYADSVKG | VATFDY |
| | | | SEQ ID NO:5430 | SEQ ID NO:13442 | SEQ ID NO:21454 |
| **iPS:434189** | **21-225_56E5** | NA | GGCTACCATATGCAC | TGGATCAACCCTAACAA TAATGCCACAAACTATG CACAGAAGTTTCAGGGC | GATGGCACCTCGTCTTTTGA CTAC |
| | | | SEQ ID NO:5431 | SEQ ID NO:13443 | SEQ ID NO:21455 |
| | | AA | GYHMH | WINPNNNATNYAQKFQG | DGTSSFDY |
| | | | SEQ ID NO:5432 | SEQ ID NO:13444 | SEQ ID NO:21456 |
| **iPS:434191** | **21-225_56B6** | NA | AGCTATGGCATGCAC | GTTATATGGCATGATGG AAGTAATAAATATTATG TAGACTCCGTGAAGGGC | GACGAGCAGCTCGGGACCTT TGACTAC |
| | | | SEQ ID NO:5433 | SEQ ID NO:13445 | SEQ ID NO:21457 |
| | | AA | SYGMH | VIWHDGSNKYYVDSVKG | DEQLGTFDY |
| | | | SEQ ID NO:5434 | SEQ ID NO:13446 | SEQ ID NO:21458 |
| **iPS:434193** | **21-225_56C6** | NA | GACTACCATATGCAC | TGGATCAACCCTAACAG AGGTGGCACAAATTATG TACAGAAGTTTCAGGGT | GATGGCACCTCGTCTTTTGA CTAT |
| | | | SEQ ID NO:5435 | SEQ ID NO:13447 | SEQ ID NO:21459 |
| | | AA | DYHMH | WINPNRGGTNYVQKFQG | DGTSSFDY |
| | | | SEQ ID NO:5436 | SEQ ID NO:13448 | SEQ ID NO:21460 |

## FIGURE 49

| | | | | | |
|---|---|---|---|---|---|
| **iPS:434195** | **21-225_56F6** | NA | GACTACTATATGCAC | TGGATCAACCCTAACAGTGGTGGCACAAACTATGCACAGAGGTTTCAGGGC | GAGGGAGCAACTCGTCCGACGGGTTTTGACTAC |
| | | | SEQ ID NO:5437 | SEQ ID NO:13449 | SEQ ID NO:21461 |
| | | AA | DYYMH | WINPNSGGTNYAQRFQG | EGATRPTGFDY |
| | | | SEQ ID NO:5438 | SEQ ID NO:13450 | SEQ ID NO:21462 |
| **iPS:434197** | **21-225_56C7** | NA | GGCCACTATATAAAC | TGGGTCAACCCTAACAGTGGTGGCACAAACTCTGCACAGAAGTTTCAGGGC | GGAGGGCAGCTCGGCGGGTTTAACTACTACTACTACGGTATGGACGTC |
| | | | SEQ ID NO:5439 | SEQ ID NO:13451 | SEQ ID NO:21463 |
| | | AA | GHYIN | WVNPNSGGTNSAQKFQG | GGQLGGFNYYYYGMDV |
| | | | SEQ ID NO:5440 | SEQ ID NO:13452 | SEQ ID NO:21464 |
| **iPS:434199** | **21-225_59F11** | NA | AACTATGGCATGCAC | GTTATATGGTATGATGAAAGTAATAAACACTATGCAGACTCCGTGAAGGGC | GAACTGGGGATGAACGGAGACTAC |
| | | | SEQ ID NO:5441 | SEQ ID NO:13453 | SEQ ID NO:21465 |
| | | AA | NYGMH | VIWYDESNKHYADSVKG | ELGMNGDY |
| | | | SEQ ID NO:5442 | SEQ ID NO:13454 | SEQ ID NO:21466 |
| **iPS:434201** | **21-225_59A12** | NA | AGCTATGGCATGCAC | GTTATATGGTATGATGGAAGTAATAAATACTATGCAGACTCCGTGAAGGGC | CGGTATAGCAGCAGCTGGGACGGGGGTATGGACGTC |
| | | | SEQ ID NO:5443 | SEQ ID NO:13455 | SEQ ID NO:21467 |
| | | AA | SYGMH | VIWYDGSNKYYADSVKG | RYSSSWDGGMDV |
| | | | SEQ ID NO:5444 | SEQ ID NO:13456 | SEQ ID NO:21468 |

**FIGURE 49**

| | | | | | |
|---|---|---|---|---|---|
| **iPS:434203** | **21-225_60E2** | NA | GACTATGGCATGCAC | ATTATATGGTATGATGAAAATAATAAATACTATGCAGACTCCGTGAAGGGC | GATGTTCTGGACCCTTTTGACTAC |
| | | | SEQ ID NO:5445 | SEQ ID NO:13457 | SEQ ID NO:21469 |
| | | AA | DYGMH | IIWYDENNKYYADSVKG | DVLDPFDY |
| | | | SEQ ID NO:5446 | SEQ ID NO:13458 | SEQ ID NO:21470 |
| **iPS:434205** | **21-225_60G2** | NA | AGTTATGGCATGCAC | GTTATATGGTATGATGGAAGTAATAAATACTATGCAGACTCCGTGAAGGGC | AGGTATAGCAGAAGCTGGACGGGAGGCATGGACGTC |
| | | | SEQ ID NO:5447 | SEQ ID NO:13459 | SEQ ID NO:21471 |
| | | AA | SYGMH | VIWYDGSNKYYADSVKG | RYSRSWTGGMDV |
| | | | SEQ ID NO:5448 | SEQ ID NO:13460 | SEQ ID NO:21472 |
| **iPS:434207** | **21-225_60A3** | NA | AGCTATGGCATGCAC | GTTATATGGTATGAAGAAAGTAATAAATACTATGCAGACTCCGTGAAGGGC | GAACTGGGGATGACCGGAGACTAC |
| | | | SEQ ID NO:5449 | SEQ ID NO:13461 | SEQ ID NO:21473 |
| | | AA | SYGMH | VIWYEESNKYYADSVKG | ELGMTGDY |
| | | | SEQ ID NO:5450 | SEQ ID NO:13462 | SEQ ID NO:21474 |
| **iPS:434209** | **21-225_60C3** | NA | GGCCACTATATACAC | TGGATCAACCCTAACAGCGGTGGCACAAACTATGTACAGAAATTTCAGGGC | GGGGGCCTACTGGGAGCTACCAACTACTATTATTACGGTATGGACGTC |
| | | | SEQ ID NO:5451 | SEQ ID NO:13463 | SEQ ID NO:21475 |
| | | AA | GHYIH | WINPNSGGTNYVQKFQG | GGLLGATNYYYYGMDV |
| | | | SEQ ID NO:5452 | SEQ ID NO:13464 | SEQ ID NO:21476 |

FIGURE 49

| | | | | | |
|---|---|---|---|---|---|
| **iPS:434211** | **21-225_60F3** | NA | AATTATGATATCAAC | TGGATGCACCCTAACAGTGGTAACACAGGCTATGCACAGAAGTTCCAGGGC | AGCAGTGGCTGGTACTTCTTTGACTAC |
| | | | SEQ ID NO:5453 | SEQ ID NO:13465 | SEQ ID NO:21477 |
| | | AA | NYDIN | WMHPNSGNTGYAQKFQG | SSGWYFFDY |
| | | | SEQ ID NO:5454 | SEQ ID NO:13466 | SEQ ID NO:21478 |
| **iPS:434213** | **21-225_60A4** | NA | AGCTATGTCATGAGC | TCTATTAGTGGTAGTGGTGGTTGGACAAACTACGCAGACTCCGTGAAGGGC | CTAACTGGATTTGACTAT |
| | | | SEQ ID NO:5455 | SEQ ID NO:13467 | SEQ ID NO:21479 |
| | | AA | SYVMS | SISGSGGWTNYADSVKG | LTGFDY |
| | | | SEQ ID NO:5456 | SEQ ID NO:13468 | SEQ ID NO:21480 |
| **iPS:434215** | **21-225_60F7** | NA | AGCTATGTCATGAGC | GGTATTAGTGGTAGTGGTAATAGAACATACTACGCAGACTCCGTGAAGGGC | TTGGGGATTGAC |
| | | | SEQ ID NO:5457 | SEQ ID NO:13469 | SEQ ID NO:21481 |
| | | AA | SYVMS | GISGSGNRTYYADSVKG | LGID |
| | | | SEQ ID NO:5458 | SEQ ID NO:13470 | SEQ ID NO:21482 |
| **iPS:434217** | **21-225_60E8** | NA | AGGAGTAGTTACTACTGGGGC | AGTATCTATTATAGTGGGAGCGCCTCCTACAACCCGTCCCTCAAGAGT | CTGGACAGTGGCTGGTCGTTTGACTAC |
| | | | SEQ ID NO:5459 | SEQ ID NO:13471 | SEQ ID NO:21483 |
| | | AA | RSSYYWG | SIYYSGSASYNPSLKS | LDSGWSFDY |
| | | | SEQ ID NO:5460 | SEQ ID NO:13472 | SEQ ID NO:21484 |
| **iPS:434219** | **21-225_60E9** | NA | AGCTATGCCATGAGC | GCTATTAGTGGTAGTGGTGGTAACACATTCTACGCAGACTCCGTGAAGGGC | TTTTTCGGTATAGTGGGAGCCGGGTACTTTGACTAC |
| | | | SEQ ID NO:5461 | SEQ ID NO:13473 | SEQ ID NO:21485 |

**FIGURE 49**

| | | | | | |
|---|---|---|---|---|---|
| | | AA | SYAMS | AISGSGGNTFYADSVKG | FFGIVGAGYFDY |
| | | | SEQ ID NO:5462 | SEQ ID NO:13474 | SEQ ID NO:21486 |
| **iPS:434221** | **21-225_60A11** | NA | AACTATGCCATGACC | GCTATTAGTGGTAGTGGTGGTAACACATTCTACGCAGACTCCGTGACGGGC | CTGGGGAAGGACTACCACTACTACGGTATGGACGTC |
| | | | SEQ ID NO:5463 | SEQ ID NO:13475 | SEQ ID NO:21487 |
| | | AA | NYAMT | AISGSGGNTFYADSVTG | LGKDYHYYGMDV |
| | | | SEQ ID NO:5464 | SEQ ID NO:13476 | SEQ ID NO:21488 |
| **iPS:434223** | **21-225_60C12** | NA | AGCTATGGCATGCAC | GTTATATGGTATGATGGAAGTAATAAATACTATGTAGACTCCGTGAAGGGC | AGGTATAGCAGAAGCTGGACGGGAGGTATGGACGTC |
| | | | SEQ ID NO:5465 | SEQ ID NO:13477 | SEQ ID NO:21489 |
| | | AA | SYGMH | VIWYDGSNKYYVDSVKG | RYSRSWTGGMDV |
| | | | SEQ ID NO:5466 | SEQ ID NO:13478 | SEQ ID NO:21490 |
| **iPS:434225** | **21-225_60E12** | NA | AGTTACTTCTGGAGC | CGCATCTATACCAGGGGGAGCACCAACTACAACCCCTCCCTCAAGAGT | GAGGGAAAAACTGGGGGGGTTTCTTACTTTGACTAC |
| | | | SEQ ID NO:5467 | SEQ ID NO:13479 | SEQ ID NO:21491 |
| | | AA | SYFWS | RIYTRGSTNYNPSLKS | EGKTGGVSYFDY |
| | | | SEQ ID NO:5468 | SEQ ID NO:13480 | SEQ ID NO:21492 |
| **iPS:434227** | **21-225_61A1** | NA | AGTCACTTCTGGAGC | CGCATCTATATCAGGGGGAGCACCAACTACAACCCCTCCCTCAAGAGT | GAGGGAAAAACTGGGGGGGTTTCTTACTTTGACTAC |
| | | | SEQ ID NO:5469 | SEQ ID NO:13481 | SEQ ID NO:21493 |
| | | AA | SHFWS | RIYIRGSTNYNPSLKS | EGKTGGVSYFDY |
| | | | SEQ ID NO:5470 | SEQ ID NO:13482 | SEQ ID NO:21494 |

**FIGURE 49**

| | | | | | |
|---|---|---|---|---|---|
| **iPS:434229** | **21-225_61H1** | NA | GACTATGGCATGCAC | ATTATATGGTATGATGAAAGTAATAAATACTATGCAGACTCCGTGAAGGGC | GATGTTCTGGACCCTTTTGACTAC |
| | | | SEQ ID NO:5471 | SEQ ID NO:13483 | SEQ ID NO:21495 |
| | | AA | DYGMH | IIWYDESNKYYADSVKG | DVLDPFDY |
| | | | SEQ ID NO:5472 | SEQ ID NO:13484 | SEQ ID NO:21496 |
| **iPS:434231** | **21-225_61F2** | NA | AGCTATGGCATGCAC | GTTATATGGTATGATGGAAGTAATAAATACTATGCAGACTCCGTGAAGGGC | GAGAGGTATAGCAGTGGCTGGTACGACTACGGTATGGACGTC |
| | | | SEQ ID NO:5473 | SEQ ID NO:13485 | SEQ ID NO:21497 |
| | | AA | SYGMH | VIWYDGSNKYYADSVKG | ERYSSGWYDYGMDV |
| | | | SEQ ID NO:5474 | SEQ ID NO:13486 | SEQ ID NO:21498 |
| **iPS:434233** | **21-225_61B3** | NA | AGCTATGGCATGCAC | GTTATATGGTATGATGGAAGTAATAAATACTATGCAGACTCCGTGAAGGGC | AGGTATAGCAGAAGCTGGGCGGGAGGCATGGACGTC |
| | | | SEQ ID NO:5475 | SEQ ID NO:13487 | SEQ ID NO:21499 |
| | | AA | SYGMH | VIWYDGSNKYYADSVKG | RYSRSWAGGMDV |
| | | | SEQ ID NO:5476 | SEQ ID NO:13488 | SEQ ID NO:21500 |
| **iPS:434235** | **21-225_61E3** | NA | AATTATGATATCAAC | TGGATGACCCCTAACAGTGGTAACACAGGCTATGCACAGAAGTTCCAGGGC | AGCAGTGGCTGGTACCGCTTTGACTAC |
| | | | SEQ ID NO:5477 | SEQ ID NO:13489 | SEQ ID NO:21501 |
| | | AA | NYDIN | WMTPNSGNTGYAQKFQG | SSGWYRFDY |
| | | | SEQ ID NO:5478 | SEQ ID NO:13490 | SEQ ID NO:21502 |

FIGURE 49

| | | | | | |
|---|---|---|---|---|---|
| **iPS:434237** | **21-225_61B5** | NA | AATTATGATATCAAC | TGGATGCACCCTAACAGTGGTAGCACAGGCTATGCACAGAAGTTCCAGGGC | AGCAGTGGCTGGTACTACTTTGACTAC |
| | | | SEQ ID NO:5479 | SEQ ID NO:13491 | SEQ ID NO:21503 |
| | | AA | NYDIN | WMHPNSGSTGYAQKFQG | SSGWYYFDY |
| | | | SEQ ID NO:5480 | SEQ ID NO:13492 | SEQ ID NO:21504 |
| **iPS:434239** | **21-225_58F1** | NA | AGCTATGCCATGAGT | GCTATTAGTACTGGTGGTGGTAACACATACTACGCAGACTCCGTGAAGGGC | CGGGGGGTCTACGGTGACTTTGATGCTTTTGATATC |
| | | | SEQ ID NO:5481 | SEQ ID NO:13493 | SEQ ID NO:21505 |
| | | AA | SYAMS | AISTGGGNTYYADSVKG | RGVYGDFDAFDI |
| | | | SEQ ID NO:5482 | SEQ ID NO:13494 | SEQ ID NO:21506 |
| **iPS:434241** | **21-225_61E6** | NA | AGCTATGCCATGAGC | GCTACTAGTGGTAGTGGTGTTAACACATTCTACGCAGACTCCGTGAAGGGC | TTGGAACTGGGGATCTTTGACTAC |
| | | | SEQ ID NO:5483 | SEQ ID NO:13495 | SEQ ID NO:21507 |
| | | AA | SYAMS | ATSGSGVNTFYADSVKG | LELGIFDY |
| | | | SEQ ID NO:5484 | SEQ ID NO:13496 | SEQ ID NO:21508 |
| **iPS:434243** | **21-225_62C1** | NA | AGCTATAGCATGAAC | TCCATTAGTAGTAGTAGTAGTTACATATACTACGCAGACTCAGTGAAGGGC | TTTGGAGTGGAC |
| | | | SEQ ID NO:5485 | SEQ ID NO:13497 | SEQ ID NO:21509 |
| | | AA | SYSMN | SISSSSSYIYYADSVKG | FGVD |
| | | | SEQ ID NO:5486 | SEQ ID NO:13498 | SEQ ID NO:21510 |
| **iPS:434245** | **21-225_62H1** | NA | AGCTATGGCATGCAC | ATTATATGGTATGATGGAAGTAATAAATACTATGCAGACTCCGTGAAGGGC | GAAGACCCGCGTACCAGCTGCTCTGACTAC |
| | | | SEQ ID NO:5487 | SEQ ID NO:13499 | SEQ ID NO:21511 |

FIGURE 49

| | | | | | |
|---|---|---|---|---|---|
| | | AA | SYGMH | IIWYDGSNKYYADSVKG | EDPRTSCSDY |
| | | | SEQ ID NO:5488 | SEQ ID NO:13500 | SEQ ID NO:21512 |
| **iPS:434247** | **21-225_62D2** | NA | AGCTATGGCATGCAC | GTTATATGGTATGATGG AAGTAATAAATACTCTG CAGACTCCGTGAAGGGC | GATAATGGTAACTGGAACTA CCTTGACTAC |
| | | | SEQ ID NO:5489 | SEQ ID NO:13501 | SEQ ID NO:21513 |
| | | AA | SYGMH | VIWYDGSNKYSADSVKG | DNGNWNYLDY |
| | | | SEQ ID NO:5490 | SEQ ID NO:13502 | SEQ ID NO:21514 |
| **iPS:434249** | **21-225_62E2** | NA | AGAAGTAGTTACTACTGGGG C | AGCATCTATTATAGTGG GATCGCCTCCTATAATCC GTCCCTCAAGAGT | CTGAGCAGTGGCTGGTCCTT TGACTAC |
| | | | SEQ ID NO:5491 | SEQ ID NO:13503 | SEQ ID NO:21515 |
| | | AA | RSSYYWG | SIYYSGIASYNPSLKS | LSSGWSFDY |
| | | | SEQ ID NO:5492 | SEQ ID NO:13504 | SEQ ID NO:21516 |
| **iPS:434251** | **21-225_62G3** | NA | AGCTATAGCATGAAC | TCCATTAGTAGTAGTAGT AGTTACATATACTACGC AGACTCAGTGAAGGGC | GTTAACTCTTTTGACTCC |
| | | | SEQ ID NO:5493 | SEQ ID NO:13505 | SEQ ID NO:21517 |
| | | AA | SYSMN | SISSSSSYIYYADSVKG | VNSFDS |
| | | | SEQ ID NO:5494 | SEQ ID NO:13506 | SEQ ID NO:21518 |
| **iPS:434253** | **21-225_62E4** | NA | GACTATGGCATGCAC | GTTATATGGTATGATAG AAGTAATAAATACTATG CAGACTCCGTGAAGGGC | GAGCTTGGGTTCAGCAGTGA CTAC |
| | | | SEQ ID NO:5495 | SEQ ID NO:13507 | SEQ ID NO:21519 |
| | | AA | DYGMH | VIWYDRSNKYYADSVKG | ELGFSSDY |
| | | | SEQ ID NO:5496 | SEQ ID NO:13508 | SEQ ID NO:21520 |

**FIGURE 49**

| | | | | | |
|---|---|---|---|---|---|
| **iPS:434255** | **21-225_62E6** | NA | TCCTATGGCATGCAC | GCTATATGGTATGATGGAAGTAATAAATACTATGGAGACTCCGTGAAGGGC | GATCAGGGCATAGTGGGAGCTACTTGGTTTGACTAC |
| | | | SEQ ID NO:5497 | SEQ ID NO:13509 | SEQ ID NO:21521 |
| | | AA | SYGMH | AIWYDGSNKYYGDSVKG | DQGIVGATWFDY |
| | | | SEQ ID NO:5498 | SEQ ID NO:13510 | SEQ ID NO:21522 |
| **iPS:434257** | **21-225_62F7** | NA | AGCTATGTTATGAGC | GGTATTAGTGGTAGTGGTGCTAAAACATACTATGCAGACTCCGTGAAGGGC | CTGGGGATAGACTACTACTACGGTATGGACGTC |
| | | | SEQ ID NO:5499 | SEQ ID NO:13511 | SEQ ID NO:21523 |
| | | AA | SYVMS | GISGSGAKTYYADSVKG | LGIDYYYGMDV |
| | | | SEQ ID NO:5500 | SEQ ID NO:13512 | SEQ ID NO:21524 |
| **iPS:434259** | **21-225_62G7** | NA | GGCTACTATATGCAC | TGGATCAAACCTAAAAGTGGTGGCACAAACCAAGCACAGAAGTTTCAGGGC | GCTCCGGGTATAGCAGCAGCTGGTACATGGGGATACTTTGACTAC |
| | | | SEQ ID NO:5501 | SEQ ID NO:13513 | SEQ ID NO:21525 |
| | | AA | GYYMH | WIKPKSGGTNQAQKFQG | APGIAAAGTWGYFDY |
| | | | SEQ ID NO:5502 | SEQ ID NO:13514 | SEQ ID NO:21526 |
| **iPS:434261** | **21-225_56F7** | NA | AGCTATGTCTTAAAC | GCTATGAGTGGTAGTGGTGGTAGAACATACTACGCAGACTCCGTGAAGGGC | ACTACGCACTTTGACTAC |
| | | | SEQ ID NO:5503 | SEQ ID NO:13515 | SEQ ID NO:21527 |
| | | AA | SYVLN | AMSGSGGRTYYADSVKG | TTHFDY |
| | | | SEQ ID NO:5504 | SEQ ID NO:13516 | SEQ ID NO:21528 |
| **iPS:434263** | **21 225 56H7** | NA | AGCTATAGCATGAAC | TCCATTAGTAGTAGTAGTACTTACATATACTACGGAGACTCAGTGAAGGGC | GATCGGGGCAGCAGC |

**FIGURE 49**

| | | | | | |
|---|---|---|---|---|---|
| | | | SEQ ID NO:5505 | SEQ ID NO:13517 | SEQ ID NO:21529 |
| | **21-225_56H7** | AA | SYSMN | SISSSSTYIYYGDSVKG | DRGSS |
| | | | SEQ ID NO:5506 | SEQ ID NO:13518 | SEQ ID NO:21530 |
| **iPS:434265** | **21-225_57B2** | NA | AGCTATAGCATGAAC | TCCATTAGTGGTAGTAGTAGTTACATAAACTACACAGACTCAGTGAAGGGC | GTGGCTGGCTTTGACTAC |
| | | | SEQ ID NO:5507 | SEQ ID NO:13519 | SEQ ID NO:21531 |
| | | AA | SYSMN | SISGSSSYINYTDSVKG | VAGFDY |
| | | | SEQ ID NO:5508 | SEQ ID NO:13520 | SEQ ID NO:21532 |
| **iPS:434267** | **21-225_57F2** | NA | AGTTACTACTGGAGC | CGCATCTATACCAGGGGGAGCACCAACTACAACCCCTCCCTCAAGAGT | GAGGGAAAAACTGGGGGGGTTTCTTACTTTGACTAC |
| | | | SEQ ID NO:5509 | SEQ ID NO:13521 | SEQ ID NO:21533 |
| | | AA | SYYWS | RIYTRGSTNYNPSLKS | EGKTGGVSYFDY |
| | | | SEQ ID NO:5510 | SEQ ID NO:13522 | SEQ ID NO:21534 |
| **iPS:434269** | **21-225_57H3** | NA | AGCTATGGCATGCAC | GCTATATGGTATGATGGAAGTAATAAATACTATGCAGACTCCGTGAAGGGC | GATTATGGTATAGTGGGAGCTACATATTTTGACTAC |
| | | | SEQ ID NO:5511 | SEQ ID NO:13523 | SEQ ID NO:21535 |
| | | AA | SYGMH | AIWYDGSNKYYADSVKG | DYGIVGATYFDY |
| | | | SEQ ID NO:5512 | SEQ ID NO:13524 | SEQ ID NO:21536 |
| **iPS:434271** | **21-225_57A4** | NA | GACTATGGCATGCAC | GTTATATGGTATGCTGGAAGTAATAAATACTATGTAGACTCCGTGAAGGGC | GAACTGGGGATGAGGTCTGACTAC |
| | | | SEQ ID NO:5513 | SEQ ID NO:13525 | SEQ ID NO:21537 |
| | | AA | DYGMH | VIWYAGSNKYYVDSVKG | ELGMRSDY |

**FIGURE 49**

| | | | | | |
|---|---|---|---|---|---|
| | | | SEQ ID NO:5514 | SEQ ID NO:13526 | SEQ ID NO:21538 |
| **iPS:434273** | **21-225_57E4** | NA | AGCTATGCCATGAGC | GTTATTAGTGGTAGTGGTGGTAGTACATTCTACGCAGACTCCGTGAAGGGC | AGGGACTGGAACGACGTTTTTGACTAC |
| | | | SEQ ID NO:5515 | SEQ ID NO:13527 | SEQ ID NO:21539 |
| | | AA | SYAMS | VISGSGGSTFYADSVKG | RDWNDVFDY |
| | | | SEQ ID NO:5516 | SEQ ID NO:13528 | SEQ ID NO:21540 |
| **iPS:434275** | **21-225_57F4** | NA | GACTACTACATGAAC | TACATTAGTAGTAGTGGTAGTACCATATACTACGCAGACTCTGTGAAGGGC | GATATGATTACG |
| | | | SEQ ID NO:5517 | SEQ ID NO:13529 | SEQ ID NO:21541 |
| | | AA | DYYMN | YISSSGSTIYYADSVKG | DMIT |
| | | | SEQ ID NO:5518 | SEQ ID NO:13530 | SEQ ID NO:21542 |
| **iPS:434277** | **21-225_57A7** | NA | GGCTACCATATACAC | TGGATCAACCCTAACAATAATGGCACAAACTATGCACAGAAGTTTCAGGGC | GATGGGAGAAGTGGTTTTGACTAC |
| | | | SEQ ID NO:5519 | SEQ ID NO:13531 | SEQ ID NO:21543 |
| | | AA | GYHIH | WINPNNNGTNYAQKFQG | DGRSGFDY |
| | | | SEQ ID NO:5520 | SEQ ID NO:13532 | SEQ ID NO:21544 |
| **iPS:434279** | **21-225_57F7** | NA | AGCTATGCCATGAGC | GCTATTAGTGGTAGTGGTGGTAACACATTCTACGCAGACTCCGTGAAGGGC | TTTTTCGGTGTAGTGGGAGTCGGGTGCTTTGACTAC |
| | | | SEQ ID NO:5521 | SEQ ID NO:13533 | SEQ ID NO:21545 |
| | | AA | SYAMS | AISGSGGNTFYADSVKG | FFGVVGVGCFDY |
| | | | SEQ ID NO:5522 | SEQ ID NO:13534 | SEQ ID NO:21546 |
| **iPS:434281** | **21 225 57B8** | NA | AGCTATGCCATGAGC | GCTATTAGTGGTAGTGGTGGTAACACATTCTACGCAGACTCCGTGAAGGGC | TTGGAACTGGGGATCTTTGACTAC |

FIGURE 49

| | | | | | |
|---|---|---|---|---|---|
| | **21-225_57B8** | | SEQ ID NO:5523 | SEQ ID NO:13535 | SEQ ID NO:21547 |
| | | AA | SYAMS | AISGSGGNTFYADSVKG | LELGIFDY |
| | | | SEQ ID NO:5524 | SEQ ID NO:13536 | SEQ ID NO:21548 |
| **iPS:434283** | **21-225_57F8** | NA | AACTATGCCATGAGC | GCTAGCAGTGGTAGTGG TGGTAACACATTCTACGC AGACTCCGTGACGGGC | CTGGGGAAGGACTACCACTA CTACGGTATGGACGTC |
| | | | SEQ ID NO:5525 | SEQ ID NO:13537 | SEQ ID NO:21549 |
| | | AA | NYAMS | ASSGSGGNTFYADSVTG | LGKDYHYYGMDV |
| | | | SEQ ID NO:5526 | SEQ ID NO:13538 | SEQ ID NO:21550 |
| **iPS:434285** | **21-225_57A11** | NA | AATTATGATATCAAC | TGGATGAACCCTAACAG TGTTAACACAGGCTATG CACAGAAGTTCCAGGGC | AGCAGTGGCTGGAACTGGTT CGACCCC |
| | | | SEQ ID NO:5527 | SEQ ID NO:13539 | SEQ ID NO:21551 |
| | | AA | NYDIN | WMNPNSVNTGYAQKFQG | SSGWNWFDP |
| | | | SEQ ID NO:5528 | SEQ ID NO:13540 | SEQ ID NO:21552 |
| **iPS:434287** | **21-225_57F12** | NA | AATTATGATATCAAC | TGGATGAACCCTAACAG TGGTAACACAGGCTATG CACAGAAGTTCCAGGGC | AGCAGTGGCTGGTACCGGTT CGACCCC |
| | | | SEQ ID NO:5529 | SEQ ID NO:13541 | SEQ ID NO:21553 |
| | | AA | NYDIN | WMNPNSGNTGYAQKFQG | SSGWYRFDP |
| | | | SEQ ID NO:5530 | SEQ ID NO:13542 | SEQ ID NO:21554 |
| **iPS:434289** | **21-225_57H12** | NA | AGCTACGCCATGAGC | GCTATTAGTGGTAGTGGT GGTAACACATTCTACGG AGACTCCGTGAAGGGC | TTTTTCGGTATAGTGGGTGC CGGGTACTTTGACTAC |
| | | | SEQ ID NO:5531 | SEQ ID NO:13543 | SEQ ID NO:21555 |
| | | AA | SYAMS | AISGSGGNTFYGDSVKG | FFGIVGAGYFDY |
| | | | SEQ ID NO:5532 | SEQ ID NO:13544 | SEQ ID NO:21556 |

FIGURE 49

| | | | | | |
|---|---|---|---|---|---|
| **iPS:434291** | **21-225_58A4** | NA | AGCTACGCCATGAGC | GCTATTAGTGGTAGTGGTGGTAACACATTCTACGGAGACTCCGTGAAGGGC | TTTTTCGGTATAGTGGGAGCCGGGTTCTTTGACTCC |
| | | | SEQ ID NO:5533 | SEQ ID NO:13545 | SEQ ID NO:21557 |
| | | AA | SYAMS | AISGSGGNTFYGDSVKG | FFGIVGAGFFDS |
| | | | SEQ ID NO:5534 | SEQ ID NO:13546 | SEQ ID NO:21558 |
| **iPS:434293** | **21-225_58F5** | NA | GACTATGGCATGCAC | GTTATATGGTATGCTGGAAGTAATAAATACCATGTAGACTCCGTGAAGGGC | GAACTGGGGATGAGGTCTGACTAC |
| | | | SEQ ID NO:5535 | SEQ ID NO:13547 | SEQ ID NO:21559 |
| | | AA | DYGMH | VIWYAGSNKYHVDSVKG | ELGMRSDY |
| | | | SEQ ID NO:5536 | SEQ ID NO:13548 | SEQ ID NO:21560 |
| **iPS:434295** | **21-225_58B9** | NA | AATTATGATATCAAC | TGGATGAACCCTAACAGTGGTAGCACAGGCTATGCACAGAAGTTCCAGGGC | AGCAGTGGCTGGTACTACTTTGACTAC |
| | | | SEQ ID NO:5537 | SEQ ID NO:13549 | SEQ ID NO:21561 |
| | | AA | NYDIN | WMNPNSGSTGYAQKFQG | SSGWYYFDY |
| | | | SEQ ID NO:5538 | SEQ ID NO:13550 | SEQ ID NO:21562 |
| **iPS:434297** | **21-225_58A10** | NA | AGCTATGCCATGAGC | GCTATTAGTGGTAGTGGTGGTAACACATTCTACGCAGACTCCGTGAAGGGC | TTTTTCGGTATAGTGGGAGCCGGGTACTTTGACTAC |
| | | | SEQ ID NO:5539 | SEQ ID NO:13551 | SEQ ID NO:21563 |
| | | AA | SYAMS | AISGSGGNTFYADSVKG | FFGIVGAGYFDY |
| | | | SEQ ID NO:5540 | SEQ ID NO:13552 | SEQ ID NO:21564 |

# FIGURE 49

| | | | | | |
|---|---|---|---|---|---|
| **iPS:434299** | **21-225_58D11** | NA | GACTATGACATACAC | GTTATATGGTATGATGGAAGTAAAAAATATTATGCAGACTCCGTGAAGGGC | GATCGGGTCACTTTTGACTAC |
| | | | SEQ ID NO:5541 | SEQ ID NO:13553 | SEQ ID NO:21565 |
| | | AA | DYDIH | VIWYDGSKKYYADSVKG | DRVTFDY |
| | | | SEQ ID NO:5542 | SEQ ID NO:13554 | SEQ ID NO:21566 |
| **iPS:434301** | **21-225_58F11** | NA | AGCTATGCCATGAGC | GCTATTAGTGGTAGTGGTGGTAACACATTCTACGCAGACTCCGTGAAGGGC | TTTTTCGGTATGGTGGGAGCCGGATTCTTTGACTAC |
| | | | SEQ ID NO:5543 | SEQ ID NO:13555 | SEQ ID NO:21567 |
| | | AA | SYAMS | AISGSGGNTFYADSVKG | FFGMVGAGFFDY |
| | | | SEQ ID NO:5544 | SEQ ID NO:13556 | SEQ ID NO:21568 |
| **iPS:434303** | **21-225_58H11** | NA | AGCTATGGCATGCAC | GTTATATGGTATGATGGAAGTAATAAATACCATGCAGACTCCGTGAAGGGC | CGGTATAGCAGCAGCTGGGACGGGGGTATGGACGTC |
| | | | SEQ ID NO:5545 | SEQ ID NO:13557 | SEQ ID NO:21569 |
| | | AA | SYGMH | VIWYDGSNKYHADSVKG | RYSSSWDGGMDV |
| | | | SEQ ID NO:5546 | SEQ ID NO:13558 | SEQ ID NO:21570 |
| **iPS:434305** | **21-225_59E1** | NA | AATTATGATATCAAC | TGGATGACTCCTAACAGTGGTAACACAGGCTATGCACAGAAGTTCCAGGGC | AGCAGTGGCTGGTACTTCTTTGACTAC |
| | | | SEQ ID NO:5547 | SEQ ID NO:13559 | SEQ ID NO:21571 |
| | | AA | NYDIN | WMTPNSGNTGYAQKFQG | SSGWYFFDY |
| | | | SEQ ID NO:5548 | SEQ ID NO:13560 | SEQ ID NO:21572 |

FIGURE 49

| | | | | | |
|---|---|---|---|---|---|
| **iPS:434307** | **21-225_59B2** | NA | GGCTACTATATACAC | TGGATCAACCCTAACAGTGGTGGCACAAACTATGCACAGAAGTTTCAGGGC | GATCCGGGGCCCTTTGACTAC |
| | | | SEQ ID NO:5549 | SEQ ID NO:13561 | SEQ ID NO:21573 |
| | | AA | GYYIH | WINPNSGGTNYAQKFQG | DPGPFDY |
| | | | SEQ ID NO:5550 | SEQ ID NO:13562 | SEQ ID NO:21574 |
| **iPS:434309** | **21-225_59B5** | NA | AGCTATGCCATGAAC | GCTATTAGTGGTAGTGGTGGTAACACATTCTACGCAGACTCCGTGAAGGGC | AGGGGGGTCTACGGTGACTACGAGGCTTTTGATATC |
| | | | SEQ ID NO:5551 | SEQ ID NO:13563 | SEQ ID NO:21575 |
| | | AA | SYAMN | AISGSGGNTFYADSVKG | RGVYGDYEAFDI |
| | | | SEQ ID NO:5552 | SEQ ID NO:13564 | SEQ ID NO:21576 |
| **iPS:434311** | **21-225_59H5** | NA | AGCTATGGCATGCAC | GTTATATGGTATGATGGAAGTAATAAATACTATGCAGACTCCGTGAAGGGC | GAGAGGGGTATAGCAGTGGGGTACTACGGTATGGACGTC |
| | | | SEQ ID NO:5553 | SEQ ID NO:13565 | SEQ ID NO:21577 |
| | | AA | SYGMH | VIWYDGSNKYYADSVKG | ERGIAVGYYGMDV |
| | | | SEQ ID NO:5554 | SEQ ID NO:13566 | SEQ ID NO:21578 |
| **iPS:434313** | **21-225_59E6** | NA | AGAAGTAGTTACTACTGGGGC | AATATCTATTATAGTGGGAGCACCTACTACAACCCGTCCCTCAAGAGT | CATAGCAGCAGCTGGTCCCTTGACTAC |
| | | | SEQ ID NO:5555 | SEQ ID NO:13567 | SEQ ID NO:21579 |
| | | AA | RSSYYWG | NIYYSGSTYYNPSLKS | HSSSWSLDY |
| | | | SEQ ID NO:5556 | SEQ ID NO:13568 | SEQ ID NO:21580 |
| **iPS:434315** | **21-225_59G7** | NA | GGCCACTATATACAC | TGGATCAACCCGAACAGTGGTGGCACAAACTATGTACAGAAATTTCAGGGC | GGGGGCCTACTGGGAGCTACCAACTACTACTACTACGGTATGGACGTC |

FIGURE 49

| | | | | | |
|---|---|---|---|---|---|
| | | | SEQ ID NO:5557 | SEQ ID NO:13569 | SEQ ID NO:21581 |
| | | AA | GHYIH | WINPNSGGTNYVQKFQG | GGLLGATNYYYYGMDV |
| | | | SEQ ID NO:5558 | SEQ ID NO:13570 | SEQ ID NO:21582 |
| **iPS:434317** | **21-225_59E8** | NA | AGCTATAGCATGAAT | TACATTAGTAGTAGTAGTGGGACCATATACTACGCAGACTCTGTGAAGGGC | GAATGGGGAATGGCAGTGGCTGGCCCGTTTGACTAC |
| | | | SEQ ID NO:5559 | SEQ ID NO:13571 | SEQ ID NO:21583 |
| | | AA | SYSMN | YISSSSGTIYYADSVKG | EWGMAVAGPFDY |
| | | | SEQ ID NO:5560 | SEQ ID NO:13572 | SEQ ID NO:21584 |
| **iPS:434319** | **21-225_59B9** | NA | GGCAATTATATACAC | TGGATCAACCCTAACAGTGGTGGCACAAACTATGTACAGAAGTTTCAGGGC | GGGGGCCTACTGGGAGCTACCTACTACTACTACTACGGTATGGACGTC |
| | | | SEQ ID NO:5561 | SEQ ID NO:13573 | SEQ ID NO:21585 |
| | | AA | GNYIH | WINPNSGGTNYVQKFQG | GGLLGATYYYYYGMDV |
| | | | SEQ ID NO:5562 | SEQ ID NO:13574 | SEQ ID NO:21586 |
| **iPS:434321** | **21-225_59F10** | NA | AATTATGATATCAAC | TGGATGAACCCTAACAGTGGTAACACAGGCTATGCACAGAAGTTCCAGGGC | AGCAGTGGCTGGTACTACTTTGACTAC |
| | | | SEQ ID NO:5563 | SEQ ID NO:13575 | SEQ ID NO:21587 |
| | | AA | NYDIN | WMNPNSGNTGYAQKFQG | SSGWYYFDY |
| | | | SEQ ID NO:5564 | SEQ ID NO:13576 | SEQ ID NO:21588 |
| **iPS:434323** | **21-225_62H8** | NA | ACCTATGGCATGCAC | GTTATATGGCATGATGGAAGTGATAAATATTATGTAGACTCCGTGAAGGGC | GAAGACCCGCGTACCAGCTGCTCTGACTAC |
| | | | SEQ ID NO:5565 | SEQ ID NO:13577 | SEQ ID NO:21589 |
| | | AA | TYGMH | VIWHDGSDKYYVDSVKG | EDPRTSCSDY |
| | | | SEQ ID NO:5566 | SEQ ID NO:13578 | SEQ ID NO:21590 |

FIGURE 49

| | | | | | |
|---|---|---|---|---|---|
| **iPS:434327** | **21-225_63G6** | NA | AGCTATGGCATGCAC | GTTATATGGTATGATGGAAGTAATAAATACTATGCAGACTCCGTGAAGGGC | GATAGCCTCTCGGGTATAGCAGCAGCTTTTGACTAC |
| | | | SEQ ID NO:5567 | SEQ ID NO:13579 | SEQ ID NO:21591 |
| | | AA | SYGMH | VIWYDGSNKYYADSVKG | DSLSGIAAAFDY |
| | | | SEQ ID NO:5568 | SEQ ID NO:13580 | SEQ ID NO:21592 |
| **iPS:434331** | **21-225_63H8** | NA | AGCTATAACATGAAC | TCCATTAGTGGTAGTAGCACTTACATGAACTACACAGACTCAGTGAAGGGC | CTACGTAATTTTGACTAC |
| | | | SEQ ID NO:5569 | SEQ ID NO:13581 | SEQ ID NO:21593 |
| | | AA | SYNMN | SISGSSTYMNYTDSVKG | LRNFDY |
| | | | SEQ ID NO:5570 | SEQ ID NO:13582 | SEQ ID NO:21594 |
| **iPS:434333** | **21-225_63C9** | NA | GGCTACTATATGCAC | TGGATCAACCCTAACAGTGGTGGCACAAACTTTGCACAGAAGTTTCAGGGC | GCTCCGGGTGTAGCAGCAGCTGGTTCATGGGGATACTTTGACTAC |
| | | | SEQ ID NO:5571 | SEQ ID NO:13583 | SEQ ID NO:21595 |
| | | AA | GYYMH | WINPNSGGTNFAQKFQG | APGVAAAGSWGYFDY |
| | | | SEQ ID NO:5572 | SEQ ID NO:13584 | SEQ ID NO:21596 |
| **iPS:434335** | **21-225_63C10** | NA | AGCTATGGCATGCAC | GTCATATGGTATGATGGAAGTAATAAATACTATGCAGACTCCGTGAAGGGC | GATGATCCCAGATCCTCGGCCGGGGACTAC |
| | | | SEQ ID NO:5573 | SEQ ID NO:13585 | SEQ ID NO:21597 |
| | | AA | SYGMH | VIWYDGSNKYYADSVKG | DDPRSSAGDY |
| | | | SEQ ID NO:5574 | SEQ ID NO:13586 | SEQ ID NO:21598 |

FIGURE 49

| | | | | | |
|---|---|---|---|---|---|
| **iPS:434337** | **21-225_64E1** | NA | AGCTATGGCATGCAC | GTTATATGGTTTGATGAAACTAATAAATACTATGGAGACTCCGTGAAGGGC | GAGCTTGGGTTCAGCAGTGACTAT |
| | | | SEQ ID NO:5575 | SEQ ID NO:13587 | SEQ ID NO:21599 |
| | | AA | SYGMH | VIWFDETNKYYGDSVKG | ELGFSSDY |
| | | | SEQ ID NO:5576 | SEQ ID NO:13588 | SEQ ID NO:21600 |
| **iPS:434339** | **21-225_64A4** | NA | GATTATGTCATGCAC | GTCATATGGTATGATGGAAGTAATAAATACTATGCAGACTCCGTGAAGGGC | GAAAGGTATAGCAGCAGCTGGTACGACTACGGTATGGACGTC |
| | | | SEQ ID NO:5577 | SEQ ID NO:13589 | SEQ ID NO:21601 |
| | | AA | DYVMH | VIWYDGSNKYYADSVKG | ERYSSSWYDYGMDV |
| | | | SEQ ID NO:5578 | SEQ ID NO:13590 | SEQ ID NO:21602 |
| **iPS:434341** | **21-225_64F7** | NA | AGTTACTTCTGGAGC | CGTATCTATACCAGTGGGATCTCCAACTACAATCCCTCCCTCAAGAGT | TTTAGCAGTGGCTTTTTTGACTAC |
| | | | SEQ ID NO:5579 | SEQ ID NO:13591 | SEQ ID NO:21603 |
| | | AA | SYFWS | RIYTSGISNYNPSLKS | FSSGFFDY |
| | | | SEQ ID NO:5580 | SEQ ID NO:13592 | SEQ ID NO:21604 |
| **iPS:434343** | **21-225_64C8** | NA | GACTATGTCATGCAC | GTTATATGGTATGATGGAAGTAATAAATACTATGCAGACTCCATGAAGGGC | GAACGGTATAGCAGTGGCTGGTACGACTACGGTATGGACGTC |
| | | | SEQ ID NO:5581 | SEQ ID NO:13593 | SEQ ID NO:21605 |
| | | AA | DYVMH | VIWYDGSNKYYADSMKG | ERYSSGWYDYGMDV |
| | | | SEQ ID NO:5582 | SEQ ID NO:13594 | SEQ ID NO:21606 |

FIGURE 49

| | | | | | |
|---|---|---|---|---|---|
| **iPS:434345** | **21-225_64H9** | NA | ACCTATGGCATGCAC | ATTATATGGTATGATGGAAGTAATAAATACTATGCAGACTCCGTGAAGGGC | GATACATACGATTTTTGGAGTGGTTATTTGGGCTAC |
| | | | SEQ ID NO:5583 | SEQ ID NO:13595 | SEQ ID NO:21607 |
| | | AA | TYGMH | IIWYDGSNKYYADSVKG | DTYDFWSGYLGY |
| | | | SEQ ID NO:5584 | SEQ ID NO:13596 | SEQ ID NO:21608 |
| **iPS:434347** | **21-225_64H10** | NA | GGCTACTATATGCAC | TGGATCAAACCAAACAGTGGTGGCACAAACCAAGCACAGAAGTTTCAGGGC | GCTCCGGGTACTGCAGCAACTGGTACATGGGGATACTTTGACTAC |
| | | | SEQ ID NO:5585 | SEQ ID NO:13597 | SEQ ID NO:21609 |
| | | AA | GYYMH | WIKPNSGGTNQAQKFQG | APGTAATGTWGYFDY |
| | | | SEQ ID NO:5586 | SEQ ID NO:13598 | SEQ ID NO:21610 |
| **iPS:434351** | **21-225_64A12** | NA | AGCTATGGCATGCAC | GTTATATGGTATGATGAAAGTAATAAATACTATGTAGACTCCGTGAAGGGC | GAACTCGGGTTCCTCTCTGACCAC |
| | | | SEQ ID NO:5587 | SEQ ID NO:13599 | SEQ ID NO:21611 |
| | | AA | SYGMH | VIWYDESNKYYVDSVKG | ELGFLSDH |
| | | | SEQ ID NO:5588 | SEQ ID NO:13600 | SEQ ID NO:21612 |
| **iPS:434353** | **21-225_64B12** | NA | AGAAGTAGTTACTACTGGGGC | AGTATCTATTACAGTGGGAGCACCTCCTACAACCCGTCCCTCAAGAGT | CTGGACAGTGGCTGGTCCTTTGACTAC |
| | | | SEQ ID NO:5589 | SEQ ID NO:13601 | SEQ ID NO:21613 |
| | | AA | RSSYYWG | SIYYSGSTSYNPSLKS | LDSGWSFDY |
| | | | SEQ ID NO:5590 | SEQ ID NO:13602 | SEQ ID NO:21614 |

**FIGURE 49**

| | | | | | |
|---|---|---|---|---|---|
| **iPS:434355** | **21-225_64G12** | NA | AGTAATGCCATGAGC | GTTATTAGTGGTAGTGGTGGTAGCACATACTACGCAGACTCCGTGAAGGGC | AGGAACTACGACGATGCTTTTGATATC |
| | | | SEQ ID NO:5591 | SEQ ID NO:13603 | SEQ ID NO:21615 |
| | | AA | SNAMS | VISGSGGSTYYADSVKG | RNYDDAFDI |
| | | | SEQ ID NO:5592 | SEQ ID NO:13604 | SEQ ID NO:21616 |
| **iPS:434357** | **21-225_65C1** | NA | GACTATGGCATGCAC | GTGATATGGTTTGAGGGAAGTAATAAACACTATACAGACTCCGTGAAGGGC | GAACTTGGGTTCAGCAGTGACTAC |
| | | | SEQ ID NO:5593 | SEQ ID NO:13605 | SEQ ID NO:21617 |
| | | AA | DYGMH | VIWFEGSNKHYTDSVKG | ELGFSSDY |
| | | | SEQ ID NO:5594 | SEQ ID NO:13606 | SEQ ID NO:21618 |
| **iPS:434359** | **21-225_65G3** | NA | GGCTACTATATACAC | TGGATCAACCCTAACAGTGGTGGCACAAACTCTGCACAGAAGTTTCAGGGC | GCTCCGGGTAAAGCAGCAGCTGGTACATGGGGATACTTTGACTAC |
| | | | SEQ ID NO:5595 | SEQ ID NO:13607 | SEQ ID NO:21619 |
| | | AA | GYYIH | WINPNSGGTNSAQKFQG | APGKAAAGTWGYFDY |
| | | | SEQ ID NO:5596 | SEQ ID NO:13608 | SEQ ID NO:21620 |
| **iPS:434361** | **21-225_65D5** | NA | AGCTATGGTATCAGT | TGGATCAGCGCTTACAGTGGTAACACAAACTATGCACAGAAGCTCCAGGGC | GGGGAAGCAGTGGCTGTCTTCGACCCC |
| | | | SEQ ID NO:5597 | SEQ ID NO:13609 | SEQ ID NO:21621 |
| | | AA | SYGIS | WISAYSGNTNYAQKLQG | GEAVAVFDP |
| | | | SEQ ID NO:5598 | SEQ ID NO:13610 | SEQ ID NO:21622 |
| **iPS:434363** | **21 225 65A6** | NA | AACTATGGCATGCAC | GTTATATGGTATGATGGAAGTAATAAATACTATGGAGACTCCGTGAAGGGC | GATCAGGGCATAGTGGGAGCTACTTGGTTTGACTAC |

FIGURE 49

| | | | | | |
|---|---|---|---|---|---|
| | **21-225_65A6** | | SEQ ID NO:5599 | SEQ ID NO:13611 | SEQ ID NO:21623 |
| | | AA | NYGMH | VIWYDGSNKYYGDSVKG | DQGIVGATWFDY |
| | | | SEQ ID NO:5600 | SEQ ID NO:13612 | SEQ ID NO:21624 |
| **iPS:434367** | **21-225_65H11** | NA | AGCTATAGGATGAAC | TCCATTAGTAGTAGTAATAGTTCCATATACTACGCAGACTCAGTGAAGGGC | ACAAGTGGGAGC |
| | | | SEQ ID NO:5601 | SEQ ID NO:13613 | SEQ ID NO:21625 |
| | | AA | SYRMN | SISSSNSSIYYADSVKG | TSGS |
| | | | SEQ ID NO:5602 | SEQ ID NO:13614 | SEQ ID NO:21626 |
| **iPS:434369** | **21-225_66B1** | NA | GGCTACTATATGCAC | TGGATCAACCCTAACAGTGGTGGCACAAACAATGCACAGAAGTTTCAGGGC | GCTCCGGGTACAGCAGCAGCTGGTACATGGGGATACTTTGACTAC |
| | | | SEQ ID NO:5603 | SEQ ID NO:13615 | SEQ ID NO:21627 |
| | | AA | GYYMH | WINPNSGGTNNAQKFQG | APGTAAAGTWGYFDY |
| | | | SEQ ID NO:5604 | SEQ ID NO:13616 | SEQ ID NO:21628 |
| **iPS:434373** | **21-225_66A7** | NA | GGCTACTATATGCAC | TGGATCAAACCAAACAGTGGTGGCACAAACCAAGCACAGAAGTTCCAGGGC | GCTCCGGGCACAGTAGCAGCTGGTACATGGGGATACTTTGACTAT |
| | | | SEQ ID NO:5605 | SEQ ID NO:13617 | SEQ ID NO:21629 |
| | | AA | GYYMH | WIKPNSGGTNQAQKFQG | APGTVAAGTWGYFDY |
| | | | SEQ ID NO:5606 | SEQ ID NO:13618 | SEQ ID NO:21630 |
| **iPS:434375** | **21-225_66C7** | NA | GACTATGGCATGCAC | GTTATATGGTTTGAGGGAAGTCATAAATACTATACAGACTCCGTGAAGGGC | GAACTTGGGTTCAGCAGTGACTAC |
| | | | SEQ ID NO:5607 | SEQ ID NO:13619 | SEQ ID NO:21631 |
| | | AA | DYGMH | VIWFEGSHKYYTDSVKG | ELGFSSDY |
| | | | SEQ ID NO:5608 | SEQ ID NO:13620 | SEQ ID NO:21632 |

**FIGURE 49**

| | | | | | |
|---|---|---|---|---|---|
| **iPS:434379** | **21-225_66A9** | NA | AGCTATGGCATGCAC | GTTATATGGCATGATGGAAGTGATAAATACTATGCAGACTCCGTGAAGGGC | GAAGACCCGCGTACCAGTTGTTCTGACTAC |
| | | | SEQ ID NO:5609 | SEQ ID NO:13621 | SEQ ID NO:21633 |
| | | AA | SYGMH | VIWHDGSDKYYADSVKG | EDPRTSCSDY |
| | | | SEQ ID NO:5610 | SEQ ID NO:13622 | SEQ ID NO:21634 |
| **iPS:434383** | **21-225_66F9** | NA | AGCTATAGCATGAAC | TCCATTAGTGGTACTAGTAGTTACATATACTACGCAGACTCAGTGAAGGGC | ACCAATGCTTTTGATATC |
| | | | SEQ ID NO:5611 | SEQ ID NO:13623 | SEQ ID NO:21635 |
| | | AA | SYSMN | SISGTSSYIYYADSVKG | TNAFDI |
| | | | SEQ ID NO:5612 | SEQ ID NO:13624 | SEQ ID NO:21636 |
| **iPS:434385** | **21-225_66C10** | NA | AGCTATGTTATGAGC | GGTATTAGTGGTAGTGGTGCTAGAACATACTACGCAGACTCCGTGAAGGGC | CTGGGGATAGACTACTACTACGGTATGGACGTC |
| | | | SEQ ID NO:5613 | SEQ ID NO:13625 | SEQ ID NO:21637 |
| | | AA | SYVMS | GISGSGARTYYADSVKG | LGIDYYYGMDV |
| | | | SEQ ID NO:5614 | SEQ ID NO:13626 | SEQ ID NO:21638 |
| **iPS:434387** | **21-225_66D11** | NA | AGTTATGGCATGCAC | GTTATATGGTATGATGGAAGTAATAAATACTATGCAGACTCCGTGAAGGGC | GAGATGTATAGCAGCAACTGGTACGACTACGGTTTGGACGTC |
| | | | SEQ ID NO:5615 | SEQ ID NO:13627 | SEQ ID NO:21639 |
| | | AA | SYGMH | VIWYDGSNKYYADSVKG | EMYSSNWYDYGLDV |
| | | | SEQ ID NO:5616 | SEQ ID NO:13628 | SEQ ID NO:21640 |

**FIGURE 49**

| | | | | | |
|---|---|---|---|---|---|
| **iPS:434389** | **21-225_66F11** | NA | GGCTACCATATGCAC | TGGATCAACCCTAACAATGGTGGCACACACTATGCACAGAAGTTTCAGGAC | GATAGTAGAAGTTCGTGGGACTAC |
| | | | SEQ ID NO:5617 | SEQ ID NO:13629 | SEQ ID NO:21641 |
| | | AA | GYHMH | WINPNNGGTHYAQKFQD | DSRSSWDY |
| | | | SEQ ID NO:5618 | SEQ ID NO:13630 | SEQ ID NO:21642 |
| **iPS:434393** | **21-225_67C3** | NA | AGTTATGGCATGCAC | GCTATATGGTATGATGGAAGTAATAAATATTATGGAGACTCCGTGAAGGGC | GATCAGGGCATAGTGGGAGCTACTTGGTTTGACTAC |
| | | | SEQ ID NO:5619 | SEQ ID NO:13631 | SEQ ID NO:21643 |
| | | AA | SYGMH | AIWYDGSNKYYGDSVKG | DQGIVGATWFDY |
| | | | SEQ ID NO:5620 | SEQ ID NO:13632 | SEQ ID NO:21644 |
| **iPS:434397** | **21-225_67H4** | NA | GGCTACTATATGCAC | TGGATCAAACCAAACAGTGGTGGCACAAACCAAGCACAGAAGTTTCAGGGC | GCTCCGGGTACTGCAGCAACTGGTACATGGGGATACTTTGACTAC |
| | | | SEQ ID NO:5621 | SEQ ID NO:13633 | SEQ ID NO:21645 |
| | | AA | GYYMH | WIKPNSGGTNQAQKFQG | APGTAATGTWGYFDY |
| | | | SEQ ID NO:5622 | SEQ ID NO:13634 | SEQ ID NO:21646 |
| **iPS:434399** | **21-225_67B7** | NA | AACTATGGCATGCAC | GTTATATTATATGATGGAAGTAAGAAATACTATGCAGACTCCGTGAAGGGC | AGTATCCCGGAATTTGACTAT |
| | | | SEQ ID NO:5623 | SEQ ID NO:13635 | SEQ ID NO:21647 |
| | | AA | NYGMH | VILYDGSKKYYADSVKG | SIPEFDY |
| | | | SEQ ID NO:5624 | SEQ ID NO:13636 | SEQ ID NO:21648 |

**FIGURE 49**

| | | | | | |
|---|---|---|---|---|---|
| **iPS:434405** | **21-225_68E6** | NA | AGCTTTGGCATGAAC | TACATTAGTAGAAGTAGTAGTCACATATACTACGCAGACTCAGTGAAGGGC | TCTAGTGGGAGCCCCTTTGACTAC |
| | | | SEQ ID NO:5625 | SEQ ID NO:13637 | SEQ ID NO:21649 |
| | | AA | SFGMN | YISRSSSHIYYADSVKG | SSGSPFDY |
| | | | SEQ ID NO:5626 | SEQ ID NO:13638 | SEQ ID NO:21650 |
| **iPS:434407** | **21-225_68G8** | NA | AGCTATAGCATGAAC | TCCATTAGTGGTAGTAGTAGTTACATATATTACGCAGACTCAGTGATGGGC | GTCAACAGCTTTGACTCC |
| | | | SEQ ID NO:5627 | SEQ ID NO:13639 | SEQ ID NO:21651 |
| | | AA | SYSMN | SISGSSSYIYYADSVMG | VNSFDS |
| | | | SEQ ID NO:5628 | SEQ ID NO:13640 | SEQ ID NO:21652 |
| **iPS:434411** | **21-225_68F11** | NA | GACTATGGCATGCAC | GTTATATGGTATGATGTAAGTAATAAATACTATGCAGACTCCGTGAAGGGC | GAACTGGGGATGACCTCTGACTGC |
| | | | SEQ ID NO:5629 | SEQ ID NO:13641 | SEQ ID NO:21653 |
| | | AA | DYGMH | VIWYDVSNKYYADSVKG | ELGMTSDC |
| | | | SEQ ID NO:5630 | SEQ ID NO:13642 | SEQ ID NO:21654 |
| **iPS:434413** | **21-225_68D12** | NA | AGAAGTAGTTACTACTGGGGC | AATATCTATTATAGTGGGAGCACCTATTACATCCCGTCCCTCAAGAGT | CATAGCACCAGCTGGTCCATTGACTAC |
| | | | SEQ ID NO:5631 | SEQ ID NO:13643 | SEQ ID NO:21655 |
| | | AA | RSSYYWG | NIYYSGSTYYIPSLKS | HSTSWSIDY |
| | | | SEQ ID NO:5632 | SEQ ID NO:13644 | SEQ ID NO:21656 |
| **iPS:434417** | **21-225_69C8** | NA | AACTATGCCATGCAC | GTTATATGGTATGATGGAAGTGATAAATACTATGCAGACTCCGTGAAGGGC | GATATCCCTAGCAACTCGGCGGGGGACTAC |
| | | | SEQ ID NO:5633 | SEQ ID NO:13645 | SEQ ID NO:21657 |

**FIGURE 49**

| | | | | | |
|---|---|---|---|---|---|
| | | AA | NYAMH | VIWYDGSDKYYADSVKG | DIPSNSAGDY |
| | | | SEQ ID NO:5634 | SEQ ID NO:13646 | SEQ ID NO:21658 |
| **iPS:434423** | **21-225_70D1** | NA | GGCTACCATATGCAC | TGGATCAACCCTAACAGTAATGCCACAAACTATGCACAGAAGTTTCAGGGC | GATAGCATATCGTCGTGGGACTAC |
| | | | SEQ ID NO:5635 | SEQ ID NO:13647 | SEQ ID NO:21659 |
| | | AA | GYHMH | WINPNSNATNYAQKFQG | DSISSWDY |
| | | | SEQ ID NO:5636 | SEQ ID NO:13648 | SEQ ID NO:21660 |
| **iPS:434425** | **21-225_70A5** | NA | AGCTATGGCATGCAC | GTTATATGGTATGATGGAAGTAATAAATACTATGCAGACTCCGTGAAGGGC | GATCAGGGCATAGTGGGAGCTACTTGGTTTGACTAC |
| | | | SEQ ID NO:5637 | SEQ ID NO:13649 | SEQ ID NO:21661 |
| | | AA | SYGMH | VIWYDGSNKYYADSVKG | DQGIVGATWFDY |
| | | | SEQ ID NO:5638 | SEQ ID NO:13650 | SEQ ID NO:21662 |
| **iPS:434427** | **21-225_70D6** | NA | GGCTACTATATGCAC | TGGATCAACCCTAAGAGTGGTGGCACAAACTCTGCACAGAAGTTTCAGGGC | GCTCCGGGTAAAGCAGCAGCTGGTACATGGGGATTCTTTGACTAC |
| | | | SEQ ID NO:5639 | SEQ ID NO:13651 | SEQ ID NO:21663 |
| | | AA | GYYMH | WINPKSGGTNSAQKFQG | APGKAAAGTWGFFDY |
| | | | SEQ ID NO:5640 | SEQ ID NO:13652 | SEQ ID NO:21664 |
| **iPS:434429** | **21-225_70H6** | NA | AGTTATGGCATGCAC | GTTATATGGCATGATGGAAGTAATAAATACTATGCAGACTCCGTGAAGGGC | GATGATCCCAGATCCTCGGCCGGGGACTAC |
| | | | SEQ ID NO:5641 | SEQ ID NO:13653 | SEQ ID NO:21665 |
| | | AA | SYGMH | VIWHDGSNKYYADSVKG | DDPRSSAGDY |

**FIGURE 49**

| | | | | | |
|---|---|---|---|---|---|
| | | | SEQ ID NO:5642 | SEQ ID NO:13654 | SEQ ID NO:21666 |
| **iPS:434431** | **21-225_70E7** | NA | AATTATGATATCAAC | TGGATGCACCCTAACAG TGGTAACACAGGCTATG CACAGAAGTTCCAGGGC | AGCAGTGGCTGGTACGTCTT TGACTAC |
| | | | SEQ ID NO:5643 | SEQ ID NO:13655 | SEQ ID NO:21667 |
| | | AA | NYDIN | WMHPNSGNTGYAQKFQG | SSGWYVFDY |
| | | | SEQ ID NO:5644 | SEQ ID NO:13656 | SEQ ID NO:21668 |
| **iPS:434433** | **21-225_70E8** | NA | AGCTATGGCATGCTC | ATTATATGGTATGATGA AAGTAATAAATACTATG CAGACTCCGTGAAGGGC | GACCTACTGGACCCACGGGA CTAC |
| | | | SEQ ID NO:5645 | SEQ ID NO:13657 | SEQ ID NO:21669 |
| | | AA | SYGML | IIWYDESNKYYADSVKG | DLLDPRDY |
| | | | SEQ ID NO:5646 | SEQ ID NO:13658 | SEQ ID NO:21670 |
| **iPS:434435** | **21-225_70G9** | NA | GGCTACTATATGCAC | TGGATCAAACCTAACAG TGGTGGCACAAACCAAG CACAGAAGTTTCAGGGC | GCTCCGGGTATAGCAGCAGC TGGTACATGGGGATACTTTG ACTAC |
| | | | SEQ ID NO:5647 | SEQ ID NO:13659 | SEQ ID NO:21671 |
| | | AA | GYYMH | WIKPNSGGTNQAQKFQG | APGIAAAGTWGYFDY |
| | | | SEQ ID NO:5648 | SEQ ID NO:13660 | SEQ ID NO:21672 |
| **iPS:434437** | **21-225_70A12** | NA | GGCTACTATATGCAC | TGGATCAAACCAAACAG TGGTGGCACAAACCAAG CACAGAAGTTTCAGGGC | GCTCCGGGTACTGCAGCAAC TGGTACATGGGGATACTTTG ACTAC |
| | | | SEQ ID NO:5649 | SEQ ID NO:13661 | SEQ ID NO:21673 |
| | | AA | GYYMH | WIKPNSGGTNQAQKFQG | APGTAATGTWGYFDY |
| | | | SEQ ID NO:5650 | SEQ ID NO:13662 | SEQ ID NO:21674 |

**FIGURE 49**

| | | | | | |
|---|---|---|---|---|---|
| **iPS:434439** | **21-225_70E12** | NA | AGCTATAGCATGAAC | TCCATTAGTGGTAATAGTACTTACATATACTACACAGACTCAGTGAAGGGC | GTGGCCGCCTTTGACTGC |
| | | | SEQ ID NO:5651 | SEQ ID NO:13663 | SEQ ID NO:21675 |
| | | AA | SYSMN | SISGNSTYIYYTDSVKG | VAAFDC |
| | | | SEQ ID NO:5652 | SEQ ID NO:13664 | SEQ ID NO:21676 |
| **iPS:434441** | **21-225_71A2** | NA | GACTATGGCATGCAC | GTGATATGGTATGATGAAAGTAATAAATACTATGCAGACTCCGTGAAGGGC | GAATTGGGGTGGCAGGATGATTAC |
| | | | SEQ ID NO:5653 | SEQ ID NO:13665 | SEQ ID NO:21677 |
| | | AA | DYGMH | VIWYDESNKYYADSVKG | ELGWQDDY |
| | | | SEQ ID NO:5654 | SEQ ID NO:13666 | SEQ ID NO:21678 |
| **iPS:434443** | **21-225_71G3** | NA | AATTATGATATCAAC | TGGATGCACCCTAACAGTGGTAGCACAGGCTATGCACAGAAGTTCCAGGGC | AGCAGTGGCTGGTACTACTTTGACTAC |
| | | | SEQ ID NO:5655 | SEQ ID NO:13667 | SEQ ID NO:21679 |
| | | AA | NYDIN | WMHPNSGSTGYAQKFQG | SSGWYYFDY |
| | | | SEQ ID NO:5656 | SEQ ID NO:13668 | SEQ ID NO:21680 |
| **iPS:434447** | **21-225_71B6** | NA | AACTATGGCATGCAC | GTTATTTGGTATGATAGAACAAATAAATACTATGCAGACTCCGTGAAGGGC | GAACTGGGGATGTTGTCTGACTAC |
| | | | SEQ ID NO:5657 | SEQ ID NO:13669 | SEQ ID NO:21681 |
| | | AA | NYGMH | VIWYDRTNKYYADSVKG | ELGMLSDY |
| | | | SEQ ID NO:5658 | SEQ ID NO:13670 | SEQ ID NO:21682 |

**FIGURE 49**

| | | | | | |
|---|---|---|---|---|---|
| **iPS:434449** | **21-225_71H6** | NA | AGCTATAGCATGAAC | TCCATTAGTGGTACTAGTAGTTACATATACTACGCAGACTCAGTGAAGGGC | ACCAATGCTTTTGATATC |
| | | | SEQ ID NO:5659 | SEQ ID NO:13671 | SEQ ID NO:21683 |
| | | AA | SYSMN | SISGTSSYIYYADSVKG | TNAFDI |
| | | | SEQ ID NO:5660 | SEQ ID NO:13672 | SEQ ID NO:21684 |
| **iPS:434451** | **21-225_71B7** | NA | GGCTACTATATGCAC | TGGATCAACCCTAACAGTGGTGGCACAAACTCTGCACAGAAGTTTCAGGGC | GCTCCGGGTAAAGCAGCAGCTGGTACATGGGGATTCTTTGACTAC |
| | | | SEQ ID NO:5661 | SEQ ID NO:13673 | SEQ ID NO:21685 |
| | | AA | GYYMH | WINPNSGGTNSAQKFQG | APGKAAAGTWGFFDY |
| | | | SEQ ID NO:5662 | SEQ ID NO:13674 | SEQ ID NO:21686 |
| **iPS:434453** | **21-225_71B11** | NA | GACTATGGCATGCAC | GTTATATGGTATGATAGAAATAATAAATACTATGGAGACTCCGTGAAGGGC | GAACTGGGGATGTTGTCTGACTAC |
| | | | SEQ ID NO:5663 | SEQ ID NO:13675 | SEQ ID NO:21687 |
| | | AA | DYGMH | VIWYDRNNKYYGDSVKG | ELGMLSDY |
| | | | SEQ ID NO:5664 | SEQ ID NO:13676 | SEQ ID NO:21688 |
| **iPS:434455** | **21-225_72F5** | NA | AGCTATGCCATGATC | ACTATTAGTGGTAGTGGTGGTTACACATACTCCGCAGACTCCGTGAAGGGC | CGTATAGCAGTGACTGGGACGGAATGGTACGACCCC |
| | | | SEQ ID NO:5665 | SEQ ID NO:13677 | SEQ ID NO:21689 |
| | | AA | SYAMI | TISGSGGYTYSADSVKG | RIAVTGTEWYDP |
| | | | SEQ ID NO:5666 | SEQ ID NO:13678 | SEQ ID NO:21690 |
| **iPS:434457** | **21-225_72G12** | NA | AGCTATGGCATGCAC | GTTATATGGTTTGATGAAAGTAATAAATACTATGCAGACTCCGTGAAGGGC | GAGCTTGGTTTCAGCAGTGACTAC |

FIGURE 49

| | | | | | |
|---|---|---|---|---|---|
| | | | SEQ ID NO:5667 | SEQ ID NO:13679 | SEQ ID NO:21691 |
| | | AA | SYGMH | VIWFDESNKYYADSVKG | ELGFSSDY |
| | | | SEQ ID NO:5668 | SEQ ID NO:13680 | SEQ ID NO:21692 |
| **iPS:434459** | **21-225_71A7** | NA | GGCTACTATATGCAC | TGGATCAACCCTAAAAGTGGTGGCACAAATTATGTACAGAAGTTTCAGGGC | GCTCCGGGTACAGCACCAGCTGGGTCATGGGGATACTTTGACTAC |
| | | | SEQ ID NO:5669 | SEQ ID NO:13681 | SEQ ID NO:21693 |
| | | AA | GYYMH | WINPKSGGTNYVQKFQG | APGTAPAGSWGYFDY |
| | | | SEQ ID NO:5670 | SEQ ID NO:13682 | SEQ ID NO:21694 |
| **iPS:434461** | **21-225_73A3** | NA | GGCTACTATATGCAC | TGGATCAACCCTAAAAGTGGTGGCACGAATCATGTCCAGAAGTTTCAGGGC | GCTCCGGGTACAGCAGCAGCTGGGTCATGGGGATGCTTTGACTAC |
| | | | SEQ ID NO:5671 | SEQ ID NO:13683 | SEQ ID NO:21695 |
| | | AA | GYYMH | WINPKSGGTNHVQKFQG | APGTAAAGSWGCFDY |
| | | | SEQ ID NO:5672 | SEQ ID NO:13684 | SEQ ID NO:21696 |
| **iPS:434463** | **21-225_73A6** | NA | TACTATGGCATGCAC | GTTATATTATATGATGGAAGTAAGAAATACTATGCAGCCTCCGTGAAGGGC | AGTATCCCGGACTTTGACTAC |
| | | | SEQ ID NO:5673 | SEQ ID NO:13685 | SEQ ID NO:21697 |
| | | AA | YYGMH | VILYDGSKKYYAASVKG | SIPDFDY |
| | | | SEQ ID NO:5674 | SEQ ID NO:13686 | SEQ ID NO:21698 |
| **iPS:434467** | **21-225_73H8** | NA | AGCAATGCCATGAGC | GACATTAGTCGTAGTGGTGGTACCACATTCTACGCAGACTCCGTGAAGGGC | TGGGATAGCAGCAGCTGGTACGACGTGACTCCCTTTGACTAC |
| | | | SEQ ID NO:5675 | SEQ ID NO:13687 | SEQ ID NO:21699 |
| | | AA | SNAMS | DISRSGGTTFYADSVKG | WDSSSWYDVTPFDY |
| | | | SEQ ID NO:5676 | SEQ ID NO:13688 | SEQ ID NO:21700 |

FIGURE 49

| | | | | | |
|---|---|---|---|---|---|
| **iPS:434469** | **21-225_73C9** | NA | AATTATGTCATGCAC | GTTATATGGTATGATGGAAGTAATAAATACTATGCAGACTCCGTGAAGGGC | GAAAGGTATAGCAGCAGCTGGTTCGACTACGGTATGGACGTC |
| | | | SEQ ID NO:5677 | SEQ ID NO:13689 | SEQ ID NO:21701 |
| | | AA | NYVMH | VIWYDGSNKYYADSVKG | ERYSSSWFDYGMDV |
| | | | SEQ ID NO:5678 | SEQ ID NO:13690 | SEQ ID NO:21702 |
| **iPS:434471** | **21-225_75G3** | NA | GGTTCCTACTGGAGC | GAAATCAATCTTAGTGGAAGTACCAACTACAACCCGTCCCTCAAGAGT | GACTACGGTGGCCTTGACTAC |
| | | | SEQ ID NO:5679 | SEQ ID NO:13691 | SEQ ID NO:21703 |
| | | AA | GSYWS | EINLSGSTNYNPSLKS | DYGGLDY |
| | | | SEQ ID NO:5680 | SEQ ID NO:13692 | SEQ ID NO:21704 |
| **iPS:434473** | **21-225_76D1** | NA | GGTTGCTACTGGAGC | GAAATCAATCATAGTGGAAGAACCAACTACAACCCGTCCCTCAAGAGT | GACTACGGCGGTATGGACGTC |
| | | | SEQ ID NO:5681 | SEQ ID NO:13693 | SEQ ID NO:21705 |
| | | AA | GCYWS | EINHSGRTNYNPSLKS | DYGGMDV |
| | | | SEQ ID NO:5682 | SEQ ID NO:13694 | SEQ ID NO:21706 |
| **iPS:434475** | **21-225_74F9** | NA | AATTATGATATCAAC | TGGATGAACCCTAACAGTGGTAACACAGGCTGTGCACAGAAGTTCCAGGGC | AGCAGTGGCTGGAACTTCTTTGACTAC |
| | | | SEQ ID NO:5683 | SEQ ID NO:13695 | SEQ ID NO:21707 |
| | | AA | NYDIN | WMNPNSGNTGCAQKFQG | SSGWNFFDY |
| | | | SEQ ID NO:5684 | SEQ ID NO:13696 | SEQ ID NO:21708 |
| **iPS:434477** | | NA | AATTATGATATCAAC | TGGATGCACCCTAACAGTGGTAACACAGGCTATGCACAGAAGTTCCGGGGC | AGCAGTGGCTGGTACTACTTTGACTAC |

**FIGURE 49**

| | | | | | |
|---|---|---|---|---|---|
| | **21-225_74A6** | | SEQ ID NO:5685 | SEQ ID NO:13697 | SEQ ID NO:21709 |
| | | AA | NYDIN | WMHPNSGNTGYAQKFRG | SSGWYYFDY |
| | | | SEQ ID NO:5686 | SEQ ID NO:13698 | SEQ ID NO:21710 |
| **iPS:434479** | | NA | AATTATGATATCAAC | TGGATGCACCCTAACAG TGGTAACACAGGCTATG CACAGAAGTTCCAGGGC | AGTAGTGGCTGGTACTGGTT CGACCCC |
| | **21-225_76H1** | | SEQ ID NO:5687 | SEQ ID NO:13699 | SEQ ID NO:21711 |
| | | AA | NYDIN | WMHPNSGNTGYAQKFQG | SSGWYWFDP |
| | | | SEQ ID NO:5688 | SEQ ID NO:13700 | SEQ ID NO:21712 |
| **iPS:434481** | | NA | AATTATGATATCAAC | TGGATGCACCCTAACAG TGGTAACACAGGCTTTG CACAGAAGTTCCAGGGC | TCCAGTGGCTGGTACTGGTT CGACCCC |
| | **21-225_74B10** | | SEQ ID NO:5689 | SEQ ID NO:13701 | SEQ ID NO:21713 |
| | | AA | NYDIN | WMHPNSGNTGFAQKFQG | SSGWYWFDP |
| | | | SEQ ID NO:5690 | SEQ ID NO:13702 | SEQ ID NO:21714 |
| **iPS:434483** | | NA | AATTATGATATCAAC | TGGATGAACCCTAACAG TGGTAACACAGGCTATG CACAGAAGTTCCAGGGC | AGCAGTGGCTGGTACTGGTT CGACCCC |
| | **21-225_74C12** | | SEQ ID NO:5691 | SEQ ID NO:13703 | SEQ ID NO:21715 |
| | | AA | NYDIN | WMNPNSGNTGYAQKFQG | SSGWYWFDP |
| | | | SEQ ID NO:5692 | SEQ ID NO:13704 | SEQ ID NO:21716 |
| **iPS:434485** | | NA | AGCTATGGCATGCAC | GTTATTTGGTATGATGGA AGTAATAAATACTATGC AGACTCCGTGAAGGGC | GATCGCAATATAGTGGGAGC TACTTACTTTGAGTCC |
| | **21-225_76D2** | | SEQ ID NO:5693 | SEQ ID NO:13705 | SEQ ID NO:21717 |

FIGURE 49

| | | | | | |
|---|---|---|---|---|---|
| | | AA | SYGMH | VIWYDGSNKYYADSVKG | DRNIVGATYFES |
| | | | SEQ ID NO:5694 | SEQ ID NO:13706 | SEQ ID NO:21718 |
| **iPS:434487** | **21-225_76G2** | NA | AATTATGATATCAAC | TGGATGAACCCTAACAG TGGTAACACAGGCTATG CACAGAAGTTCCAGGGC | AGCAGTGGCTGGTACATGTT TGACTAC |
| | | | SEQ ID NO:5695 | SEQ ID NO:13707 | SEQ ID NO:21719 |
| | | AA | NYDIN | WMNPNSGNTGYAQKFQG | SSGWYMFDY |
| | | | SEQ ID NO:5696 | SEQ ID NO:13708 | SEQ ID NO:21720 |
| **iPS:434489** | **21-225_74E4** | NA | AGTAGTAATTACTACTGGGG C | AGTATCTATTATAGTGGA TACACCTCCTACAACCCG TCCCTCAAGAGT | CTTGACTCTAACTGGGGTCT TGACTAC |
| | | | SEQ ID NO:5697 | SEQ ID NO:13709 | SEQ ID NO:21721 |
| | | AA | SSNYYWG | SIYYSGYTSYNPSLKS | LDSNWGLDY |
| | | | SEQ ID NO:5698 | SEQ ID NO:13710 | SEQ ID NO:21722 |
| **iPS:434493** | **21-225_76F3** | NA | AATTATGATATCAAC | TGGATGAACCCTAACAG TGGTAACACAGGCTATG CACAGAAGTTCCAGGGC | TCCAGTGGCTGGAACTGGTT CGACCCC |
| | | | SEQ ID NO:5699 | SEQ ID NO:13711 | SEQ ID NO:21723 |
| | | AA | NYDIN | WMNPNSGNTGYAQKFQG | SSGWNWFDP |
| | | | SEQ ID NO:5700 | SEQ ID NO:13712 | SEQ ID NO:21724 |
| **iPS:434495** | **21-225_74B2** | NA | GGTCCCTACTGGAGC | GAAATCAATCATAGTGG AAGCACCAACTACAACC CGTCCCTCACGAGT | GACTACGGGGGTTTGGACGT C |
| | | | SEQ ID NO:5701 | SEQ ID NO:13713 | SEQ ID NO:21725 |
| | | AA | GPYWS | EINHSGSTNYNPSLTS | DYGGLDV |
| | | | SEQ ID NO:5702 | SEQ ID NO:13714 | SEQ ID NO:21726 |

**FIGURE 49**

| | | | | | |
|---|---|---|---|---|---|
| **iPS:434497** | **21-225_76A4** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5703 | SEQ ID NO:13715 | SEQ ID NO:21727 |
| | | AA | GCYWS | EINHSGSTNYNPSLKS | DYGGMDV |
| | | | SEQ ID NO:5704 | SEQ ID NO:13716 | SEQ ID NO:21728 |
| **iPS:434501** | **21-225_76G4** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5705 | SEQ ID NO:13717 | SEQ ID NO:21729 |
| | | AA | GCYWS | EINHSGSTNYNPSLKS | DYGGMDV |
| | | | SEQ ID NO:5706 | SEQ ID NO:13718 | SEQ ID NO:21730 |
| **iPS:434503** | **21-225_74D7** | NA | AGAAGTAGTTACTACTGGGGC | GGTATCTATTATAGTGGGAGCACCTCCTACAACCCGTCCCTCAAGAGT | CTGCGACCTAACTGGGACTTTGACTAC |
| | | | SEQ ID NO:5707 | SEQ ID NO:13719 | SEQ ID NO:21731 |
| | | AA | RSSYYWG | GIYYSGSTSYNPSLKS | LRPNWDFDY |
| | | | SEQ ID NO:5708 | SEQ ID NO:13720 | SEQ ID NO:21732 |
| **iPS:434507** | **21-225_74C5** | NA | GGTTGCTACTGGAGC | GAAATCAATCATAGTGGATGCACCAACTTCAACCCGTCCCTCAAGAGT | GACTACGGCGGTATGGACGTC |
| | | | SEQ ID NO:5709 | SEQ ID NO:13721 | SEQ ID NO:21733 |
| | | AA | GCYWS | EINHSGCTNFNPSLKS | DYGGMDV |
| | | | SEQ ID NO:5710 | SEQ ID NO:13722 | SEQ ID NO:21734 |
| **iPS:434509** | **21-225_76F5** | NA | AATTATGATATCAAC | TGGATGAACCCTAACAGTGGTAACACAGGCTATGCACAGAAGTTCCAGGGC | AGCAGTGGCTGGTACTGGTTCGACCCC |
| | | | SEQ ID NO:5711 | SEQ ID NO:13723 | SEQ ID NO:21735 |
| | | AA | NYDIN | WMNPNSGNTGYAQKFQG | SSGWYWFDP |

**FIGURE 49**

| | | | | | |
|---|---|---|---|---|---|
| | | | SEQ ID NO:5712 | SEQ ID NO:13724 | SEQ ID NO:21736 |
| **iPS:434511** | **21-225_74B11** | NA | AATTATGATATCAAC | TGGATGCACCCTAACAG TGGTAACACAGGCTATG CACAGAAGTTCCAGGGC | AGCAGTGGCTGGTACTACTT TGACTAC |
| | | | SEQ ID NO:5713 | SEQ ID NO:13725 | SEQ ID NO:21737 |
| | | AA | NYDIN | WMHPNSGNTGYAQKFQG | SSGWYYFDY |
| | | | SEQ ID NO:5714 | SEQ ID NO:13726 | SEQ ID NO:21738 |
| **iPS:434513** | **21-225_76A6** | NA | AATTATGATATCAAC | TGGATGCACCCTAACAA TGGTAACACAGGCTATG CACAGAAGTTCCAGGGC | AGCAGTGGCTGGTACTGGTT CGACCCC |
| | | | SEQ ID NO:5715 | SEQ ID NO:13727 | SEQ ID NO:21739 |
| | | AA | NYDIN | WMHPNNGNTGYAQKFQG | SSGWYWFDP |
| | | | SEQ ID NO:5716 | SEQ ID NO:13728 | SEQ ID NO:21740 |
| **iPS:434515** | **21-225_74A5** | NA | AATTATGATATCAAC | TGGATGCACCCTAACAG TGGTAACACAGGCTATG CACAGAAGTTCCAGGGC | AGTAGTGGCTGGTACTGGTT CGACCCC |
| | | | SEQ ID NO:5717 | SEQ ID NO:13729 | SEQ ID NO:21741 |
| | | AA | NYDIN | WMHPNSGNTGYAQKFQG | SSGWYWFDP |
| | | | SEQ ID NO:5718 | SEQ ID NO:13730 | SEQ ID NO:21742 |
| **iPS:434517** | **21-225_76A7** | NA | GGTTGCTACTGGAGC | GAAATCAATCATAGTGG AAGGACCAACTACAACC CGTCCCTCAAGAGT | GACTACGGTGGGCTTGACTA C |
| | | | SEQ ID NO:5719 | SEQ ID NO:13731 | SEQ ID NO:21743 |
| | | AA | GCYWS | EINHSGRTNYNPSLKS | DYGGLDY |
| | | | SEQ ID NO:5720 | SEQ ID NO:13732 | SEQ ID NO:21744 |

**FIGURE 49**

| | | | | | |
|---|---|---|---|---|---|
| **iPS:434519** | **21-225_74C7** | NA | GGTTCCTACTGGAGC | GAAATCAATCTTAGTGGAAGCACCAACTACAACCCGTCCCTCAAGAGT | GACTACGGTGGCCTTGACTAC |
| | | | SEQ ID NO:5721 | SEQ ID NO:13733 | SEQ ID NO:21745 |
| | | AA | GSYWS | EINLSGSTNYNPSLKS | DYGGLDY |
| | | | SEQ ID NO:5722 | SEQ ID NO:13734 | SEQ ID NO:21746 |
| **iPS:434523** | **21-225_75C3** | NA | GGTTGCTACTGGAGC | GAAATCAATTATAGTGGAAGAACCAACTACAACCCGTCCCTCAAGAGT | GACTACGGCGGTATGGACGTC |
| | | | SEQ ID NO:5723 | SEQ ID NO:13735 | SEQ ID NO:21747 |
| | | AA | GCYWS | EINYSGRTNYNPSLKS | DYGGMDV |
| | | | SEQ ID NO:5724 | SEQ ID NO:13736 | SEQ ID NO:21748 |
| **iPS:434525** | **21-225_76E8** | NA | AATTATGATATCAAC | TGGATGAACCCTAACAGTGGTAACACAGGCTATGCACAGAAGTTCCAGGGC | TCCAGTGGCTGGCACTGGTTCGACCCC |
| | | | SEQ ID NO:5725 | SEQ ID NO:13737 | SEQ ID NO:21749 |
| | | AA | NYDIN | WMNPNSGNTGYAQKFQG | SSGWHWFDP |
| | | | SEQ ID NO:5726 | SEQ ID NO:13738 | SEQ ID NO:21750 |
| **iPS:434529** | **21-225_76B9** | NA | AATTATGATATCAAC | TGGATGCACCCTAACAGTGGTAACACAGGCTATGCACAGAAGTTCCAGGGC | AGTAGTGGCTGGTACTGGTTCGACCCC |
| | | | SEQ ID NO:5727 | SEQ ID NO:13739 | SEQ ID NO:21751 |
| | | AA | NYDIN | WMHPNSGNTGYAQKFQG | SSGWYWFDP |
| | | | SEQ ID NO:5728 | SEQ ID NO:13740 | SEQ ID NO:21752 |
| **iPS:434531** | **21 225 76C9** | NA | AACGCCTGGATGAAC | CGTATTAAAAACAAAGCTGATGGTGGGACAACAGACTTCGCTGCACCCGTGAAAGGC | GTGGGACCTACTACGGACTAC |

**FIGURE 49**

| | | | | | |
|---|---|---|---|---|---|
| | 21-225_76C9 | | SEQ ID NO:5729 | SEQ ID NO:13741 | SEQ ID NO:21753 |
| | | AA | NAWMN | RIKNKADGGTTDFAAPVKG | VGPTTDY |
| | | | SEQ ID NO:5730 | SEQ ID NO:13742 | SEQ ID NO:21754 |
| **iPS:434533** | 21-225_85F7 | NA | GGTCCCTACTGGAGC | GAAATCAATCATAGTGGAAGCACCAACTACAACCCGTCCCTCAAGAGT | GACTACGGGGGTTTGGACGTC |
| | | | SEQ ID NO:5731 | SEQ ID NO:13743 | SEQ ID NO:21755 |
| | | AA | GPYWS | EINHSGSTNYNPSLKS | DYGGLDV |
| | | | SEQ ID NO:5732 | SEQ ID NO:13744 | SEQ ID NO:21756 |
| **iPS:434535** | 21-225_74C8 | NA | AGCTATGGCATGCAC | GTTATATGGTATGATGGAAGTAATAAAAACTATGCAGACTCCGTGAAGGGC | GATGGCAGCTATGGTTATGACGGCCTTGACTAC |
| | | | SEQ ID NO:5733 | SEQ ID NO:13745 | SEQ ID NO:21757 |
| | | AA | SYGMH | VIWYDGSNKNYADSVKG | DGSYGYDGLDY |
| | | | SEQ ID NO:5734 | SEQ ID NO:13746 | SEQ ID NO:21758 |
| **iPS:434537** | 21-225_74E11 | NA | AGCTATGGCATGCAC | GTTATTTGGTATGATGGAAGTAATAAATACTATGCAGACTCCGTGAAGGGC | GATCGCAATATAGTGGGAGCTACTTACTTTGAGTCC |
| | | | SEQ ID NO:5735 | SEQ ID NO:13747 | SEQ ID NO:21759 |
| | | AA | SYGMH | VIWYDGSNKYYADSVKG | DRNIVGATYFES |
| | | | SEQ ID NO:5736 | SEQ ID NO:13748 | SEQ ID NO:21760 |
| **iPS:434539** | 21-225_74A2 | NA | GATTACTACTGGAGC | GAAATCAATCATAGTGGAGACACCAACTACAACCCGTCCCTCAAGAGT | GAGTTTCCATATAGTGGAAGCTACCTCTACTACTACGGTATGGACGTC |
| | | | SEQ ID NO:5737 | SEQ ID NO:13749 | SEQ ID NO:21761 |

FIGURE 49

| | | | | | |
|---|---|---|---|---|---|
| | | AA | DYYWS | EINHSGDTNYNPSLKS | EFPYSGSYLYYYGMDV |
| | | | SEQ ID NO:5738 | SEQ ID NO:13750 | SEQ ID NO:21762 |
| **iPS:434547** | **21-225_74H5** | NA | GGTTGCTACTGGAGC | GAAATCAATCATAGTGGAAGGACCAACTTCAACCCGTCCCTCAAGAGT | GACTACGGCGGTATGGACGTC |
| | | | SEQ ID NO:5739 | SEQ ID NO:13751 | SEQ ID NO:21763 |
| | | AA | GCYWS | EINHSGRTNFNPSLKS | DYGGMDV |
| | | | SEQ ID NO:5740 | SEQ ID NO:13752 | SEQ ID NO:21764 |
| **iPS:434549** | **21-225_76E11** | NA | AATTATGATATCAAC | TGGATGAACCCTAACAGTGGTAACACAGGCTATGCACAGAAGTTCCAGGGC | AGCAGTGGCTGGTACTACTTTGACTAC |
| | | | SEQ ID NO:5741 | SEQ ID NO:13753 | SEQ ID NO:21765 |
| | | AA | NYDIN | WMNPNSGNTGYAQKFQG | SSGWYYFDY |
| | | | SEQ ID NO:5742 | SEQ ID NO:13754 | SEQ ID NO:21766 |
| **iPS:434551** | **21-225_75C4** | NA | AATTATGATATCAAC | TGGATGCATCCTAACAGTGGTAACACAGGCTATGCACAGAAGTTCCAGGGC | AGCAGTGGCTGGTACTACTTTGACTAC |
| | | | SEQ ID NO:5743 | SEQ ID NO:13755 | SEQ ID NO:21767 |
| | | AA | NYDIN | WMHPNSGNTGYAQKFQG | SSGWYYFDY |
| | | | SEQ ID NO:5744 | SEQ ID NO:13756 | SEQ ID NO:21768 |
| **iPS:434559** | **21-225_74D11** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5745 | SEQ ID NO:13757 | SEQ ID NO:21769 |
| | | AA | GCYWS | EINHSGSTNYNPSLKS | DYGGMDV |
| | | | SEQ ID NO:5746 | SEQ ID NO:13758 | SEQ ID NO:21770 |

FIGURE 49

| | | | | | |
|---|---|---|---|---|---|
| **iPS:434561** | **21-225_77G1** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5747 | SEQ ID NO:13759 | SEQ ID NO:21771 |
| | | AA | GCYWS | EINHSGSTNYNPSLKS | DYGGMDV |
| | | | SEQ ID NO:5748 | SEQ ID NO:13760 | SEQ ID NO:21772 |
| **iPS:434563** | **21-225_75D8** | NA | AACTACGACATGCAC | GCTATTGGTACTGCTGGTGACACATACTATCCAGGCTCCGTGAAGGGC | GTTCTTGACTACGGTGACTCCTTGGGCTACTACTACTACGGTATGGACGTC |
| | | | SEQ ID NO:5749 | SEQ ID NO:13761 | SEQ ID NO:21773 |
| | | AA | NYDMH | AIGTAGDTYYPGSVKG | VLDYGDSLGYYYYGMDV |
| | | | SEQ ID NO:5750 | SEQ ID NO:13762 | SEQ ID NO:21774 |
| **iPS:434565** | **21-225_75B10** | NA | GGTTACTACTGGAGC | GAAATCAATCACAGTGGAAGCACCAACTACAACCCGTCCCTCAAGAGT | GACTACGGTGGTTTGGACGTC |
| | | | SEQ ID NO:5751 | SEQ ID NO:13763 | SEQ ID NO:21775 |
| | | AA | GYYWS | EINHSGSTNYNPSLKS | DYGGLDV |
| | | | SEQ ID NO:5752 | SEQ ID NO:13764 | SEQ ID NO:21776 |
| **iPS:434569** | **21-225_77H5** | NA | AATTATGGCATGCAC | GTTATATGGTATGATGGAAGAAATAAATACTATGCAGACTCCGTGAAGGGC | GATCGGAGTATATTGGGAGCTACTTTCTTTGACTAC |
| | | | SEQ ID NO:5753 | SEQ ID NO:13765 | SEQ ID NO:21777 |
| | | AA | NYGMH | VIWYDGRNKYYADSVKG | DRSILGATFFDY |
| | | | SEQ ID NO:5754 | SEQ ID NO:13766 | SEQ ID NO:21778 |
| **iPS:434571** | **21-225_74D2** | NA | GGTTGCTACTGGAGC | GAAATCAATCATAGTGGAAGGACCAACTACAACCCGTCCCTCAAGAGT | GACTACGGTGGGCTTGACTAC |
| | | | SEQ ID NO:5755 | SEQ ID NO:13767 | SEQ ID NO:21779 |

FIGURE 49

| | | | | | |
|---|---|---|---|---|---|
| | | AA | GCYWS | EINHSGRTNYNPSLKS | DYGGLDY |
| | | | SEQ ID NO:5756 | SEQ ID NO:13768 | SEQ ID NO:21780 |
| **iPS:434573** | **21-225_77E6** | NA | AGCTATGGCATGCAC | GTTATATGGTATGATGGAAGTAATCAAAACTATGCAGACTCCGTGAAGGGC | GATGGCAGCTATGGTTACGACGGCCTTGACTAC |
| | | | SEQ ID NO:5757 | SEQ ID NO:13769 | SEQ ID NO:21781 |
| | | AA | SYGMH | VIWYDGSNQNYADSVKG | DGSYGYDGLDY |
| | | | SEQ ID NO:5758 | SEQ ID NO:13770 | SEQ ID NO:21782 |
| **iPS:434575** | **21-225_77C7** | NA | AATTATGATATCAAC | TGGATGAACCCTAACAGTGGTAACACAGGCTATGCACAGAAGTTCCAGGGC | TCCAGTGGCTGGCACTGGTTCGACCCC |
| | | | SEQ ID NO:5759 | SEQ ID NO:13771 | SEQ ID NO:21783 |
| | | AA | NYDIN | WMNPNSGNTGYAQKFQG | SSGWHWFDP |
| | | | SEQ ID NO:5760 | SEQ ID NO:13772 | SEQ ID NO:21784 |
| **iPS:434579** | **21-225_77F7** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5761 | SEQ ID NO:13773 | SEQ ID NO:21785 |
| | | AA | GCYWS | EINHSGSTNYNPSLKS | DYGGMDV |
| | | | SEQ ID NO:5762 | SEQ ID NO:13774 | SEQ ID NO:21786 |
| **iPS:434581** | **21-225_74B12** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5763 | SEQ ID NO:13775 | SEQ ID NO:21787 |
| | | AA | GCYWS | EINHSGSTNYNPSLKS | DYGGMDV |
| | | | SEQ ID NO:5764 | SEQ ID NO:13776 | SEQ ID NO:21788 |

**FIGURE 49**

| | | | | | |
|---|---|---|---|---|---|
| **iPS:434583** | **21-225_74B6** | NA | AATTATGATATCAAC | TGGATGCACCCTAACAA TGGTAACACAGGCTATG CACAGAAGTTCCAGGGC | AGCAGTGGCTGGTACTGGTT CGACCCC |
| | | | SEQ ID NO:5765 | SEQ ID NO:13777 | SEQ ID NO:21789 |
| | | AA | NYDIN | WMHPNNGNTGYAQKFQG | SSGWYWFDP |
| | | | SEQ ID NO:5766 | SEQ ID NO:13778 | SEQ ID NO:21790 |
| **iPS:434585** | **21-225_75A12** | NA | GGTTGCTACTGGAGC | GAAATCAATTATAGTGG AAGAACCAACTACAACC CGTCCCTCAAGAGT | GACTACGGCGGTATGGACGT C |
| | | | SEQ ID NO:5767 | SEQ ID NO:13779 | SEQ ID NO:21791 |
| | | AA | GCYWS | EINYSGRTNYNPSLKS | DYGGMDV |
| | | | SEQ ID NO:5768 | SEQ ID NO:13780 | SEQ ID NO:21792 |
| **iPS:434587** | **21-225_74G3** | NA | AATTATGATATCAAC | TGGATGCACCCTAACAG TGGTAACACAGGCTATG CACAGAAGTTCCAGGGC | AGTAGTGGCTGGTACTGGTT CGACCCC |
| | | | SEQ ID NO:5769 | SEQ ID NO:13781 | SEQ ID NO:21793 |
| | | AA | NYDIN | WMHPNSGNTGYAQKFQG | SSGWYWFDP |
| | | | SEQ ID NO:5770 | SEQ ID NO:13782 | SEQ ID NO:21794 |
| **iPS:434595** | **21-225_77A10** | NA | GGTTGCTACTGGAGC | GAAATCAATTATAGTGG AAGAACCAACTACAACC CGTCCCTCAAGAGT | GACTACGGCGGTATGGACGT C |
| | | | SEQ ID NO:5771 | SEQ ID NO:13783 | SEQ ID NO:21795 |
| | | AA | GCYWS | EINYSGRTNYNPSLKS | DYGGMDV |
| | | | SEQ ID NO:5772 | SEQ ID NO:13784 | SEQ ID NO:21796 |
| **iPS:434597** | **21-225_77C10** | NA | AATTATGATATCAAC | TGGATGCACCCTAACAG TGGTAACACAGGCTATG CACAGAAGTTCCAGGGC | AGCAGTGGCTGGTACTGGTT CGACCCC |
| | | | SEQ ID NO:5773 | SEQ ID NO:13785 | SEQ ID NO:21797 |

FIGURE 49

| | | | | | |
|---|---|---|---|---|---|
| | | AA | NYDIN | WMHPNSGNTGYAQKFQG | SSGWYWFDP |
| | | | SEQ ID NO:5774 | SEQ ID NO:13786 | SEQ ID NO:21798 |
| **iPS:434603** | **21-225_77D11** | NA | AATTATGATATCAAC | TGGATGCACCCTAACAG TGGTAACACAGGCTATG CACAGAAGTTCCAGGGC | AGTAGTGGCTGGTACTGGTT CGACCCC |
| | | | SEQ ID NO:5775 | SEQ ID NO:13787 | SEQ ID NO:21799 |
| | | AA | NYDIN | WMHPNSGNTGYAQKFQG | SSGWYWFDP |
| | | | SEQ ID NO:5776 | SEQ ID NO:13788 | SEQ ID NO:21800 |
| **iPS:434611** | **21-225_77C12** | NA | GGTTCCTACTGGAGC | GAAATCAATTATAGGGG AAGCACCAACTACAACC CGTCCCTCAAGAGT | GACTACGGCGGTATGGACGT C |
| | | | SEQ ID NO:5777 | SEQ ID NO:13789 | SEQ ID NO:21801 |
| | | AA | GSYWS | EINYRGSTNYNPSLKS | DYGGMDV |
| | | | SEQ ID NO:5778 | SEQ ID NO:13790 | SEQ ID NO:21802 |
| **iPS:434613** | **21-225_77D12** | NA | AATTATGATATCAAC | TGGATGAACCCTAACAG TGGTAACACAGGCTATG CACAGAAGTTCCAGGGC | AGCAGTGGCTGGTACTGGTT CGACCCC |
| | | | SEQ ID NO:5779 | SEQ ID NO:13791 | SEQ ID NO:21803 |
| | | AA | NYDIN | WMNPNSGNTGYAQKFQG | SSGWYWFDP |
| | | | SEQ ID NO:5780 | SEQ ID NO:13792 | SEQ ID NO:21804 |
| **iPS:434615** | **21-225_76C5** | NA | AGCTATGGCATGCAC | GTTATATGGTATGATGG AAGTAATAAAAACTATG CAGACTCCGTGAAGGGC | GATGGCAGCTATGGTTACGA CGGCCTTGACTAC |
| | | | SEQ ID NO:5781 | SEQ ID NO:13793 | SEQ ID NO:21805 |
| | | AA | SYGMH | VIWYDGSNKNYADSVKG | DGSYGYDGLDY |

FIGURE 49

| | | | | | |
|---|---|---|---|---|---|
| | | | SEQ ID NO:5782 | SEQ ID NO:13794 | SEQ ID NO:21806 |
| **iPS:434617** | **21-225_74B8** | NA | AATTATGATATCAAC | TGGATGCACCCTAATAGTGGTAACACAGGCTATGCACAGAAGTTCCAGGGC | TCCAGTGGCTGGTACTGGTTCGACCCC |
| | | | SEQ ID NO:5783 | SEQ ID NO:13795 | SEQ ID NO:21807 |
| | | AA | NYDIN | WMHPNSGNTGYAQKFQG | SSGWYWFDP |
| | | | SEQ ID NO:5784 | SEQ ID NO:13796 | SEQ ID NO:21808 |
| **iPS:434619** | **21-225_78C1** | NA | AATTATGATATCAAC | TGGATGCACCCTAACAGTGGTAACACAGGCTATGCACAGAAGTTCCAGGGC | AGCAGTGGCTGGTACTGGTTCGACCCC |
| | | | SEQ ID NO:5785 | SEQ ID NO:13797 | SEQ ID NO:21809 |
| | | AA | NYDIN | WMHPNSGNTGYAQKFQG | SSGWYWFDP |
| | | | SEQ ID NO:5786 | SEQ ID NO:13798 | SEQ ID NO:21810 |
| **iPS:434621** | **21-225_74D1** | NA | AGCTATGGCATGCAC | GTTATATGGTATGATGGAAGTAATAAATACCATGCAGACTCCGTGAAGGGC | GATGAGGGGTTCGGGGAGTTCGACTACTACAACTACGGTATGGACGTC |
| | | | SEQ ID NO:5787 | SEQ ID NO:13799 | SEQ ID NO:21811 |
| | | AA | SYGMH | VIWYDGSNKYHADSVKG | DEGFGEFDYYNYGMDV |
| | | | SEQ ID NO:5788 | SEQ ID NO:13800 | SEQ ID NO:21812 |
| **iPS:434629** | **21-225_74C3** | NA | AGCTTTGGCATGCAC | GCTATTTGGTATGATGGAAGTAATAAATACTGTGCAGACTCCGTGAAGGGC | GATCGGAGTATACTGGGAGCTGCTTTCTTTGACTAC |
| | | | SEQ ID NO:5789 | SEQ ID NO:13801 | SEQ ID NO:21813 |
| | | AA | SFGMH | AIWYDGSNKYCADSVKG | DRSILGAAFFDY |
| | | | SEQ ID NO:5790 | SEQ ID NO:13802 | SEQ ID NO:21814 |

**FIGURE 49**

| | | | | | |
|---|---|---|---|---|---|
| **iPS:434633** | **21-225_74G8** | NA | AACGCCTGGATGAAC | CGTATTAAAAACAAAGCTGATGGTGGGACAACAGACTACGCTGCACCCGTGAAAGGC | GTGGGAGCTACTACGGACTAC |
| | | | SEQ ID NO:5791 | SEQ ID NO:13803 | SEQ ID NO:21815 |
| | | AA | NAWMN | RIKNKADGGTTDYAAPVKG | VGATTDY |
| | | | SEQ ID NO:5792 | SEQ ID NO:13804 | SEQ ID NO:21816 |
| **iPS:434635** | **21-225_78E6** | NA | AATTATGATATCAAC | TGGATGCACCCTAACAGTGGTAACACAGGCTATGCACAGAAGTTCCAGGGC | AGTAGTGGCTGGTACAAATTTGACTAC |
| | | | SEQ ID NO:5793 | SEQ ID NO:13805 | SEQ ID NO:21817 |
| | | AA | NYDIN | WMHPNSGNTGYAQKFQG | SSGWYKFDY |
| | | | SEQ ID NO:5794 | SEQ ID NO:13806 | SEQ ID NO:21818 |
| **iPS:434637** | **21-225_78E7** | NA | GGTTCCTACTGGAGC | GAAATCAATCTTAGTGGAAGCACCAACTACAACCCGTCCCTCAAGAGT | GACTACGGTGGCCTTGACTAC |
| | | | SEQ ID NO:5795 | SEQ ID NO:13807 | SEQ ID NO:21819 |
| | | AA | GSYWS | EINLSGSTNYNPSLKS | DYGGLDY |
| | | | SEQ ID NO:5796 | SEQ ID NO:13808 | SEQ ID NO:21820 |
| **iPS:434639** | **21-225_74B7** | NA | AATTATGATATCAAC | TGGATGAACCCTAACAGTGGTAACACAGGCTATGCACAGAAGTTCCAGGGC | TCCAGTGGCTGGCACTGGTTCGACCCC |
| | | | SEQ ID NO:5797 | SEQ ID NO:13809 | SEQ ID NO:21821 |
| | | AA | NYDIN | WMNPNSGNTGYAQKFQG | SSGWHWFDP |
| | | | SEQ ID NO:5798 | SEQ ID NO:13810 | SEQ ID NO:21822 |

FIGURE 49

| | | | | | |
|---|---|---|---|---|---|
| **iPS:434649** | **21-225_78E11** | NA | AATTATGATATCAAC | TGGATGAACCCTAACAGTGGTAACACAGGCTGTGCACAGAAGTTCCAGGGC | AGCAGTGGCTGGAACTTCTTTGACTAC |
| | | | SEQ ID NO:5799 | SEQ ID NO:13811 | SEQ ID NO:21823 |
| | | AA | NYDIN | WMNPNSGNTGCAQKFQG | SSGWNFFDY |
| | | | SEQ ID NO:5800 | SEQ ID NO:13812 | SEQ ID NO:21824 |
| **iPS:434653** | **21-225_74B5** | NA | AATTATGATATCAAC | TGGATGAACCCTAACAGTGGTAACACAGGCTATGCACAGAAGTTCCAGGGC | TCCAGTGGCTGGAACTGGTTCGACCCC |
| | | | SEQ ID NO:5801 | SEQ ID NO:13813 | SEQ ID NO:21825 |
| | | AA | NYDIN | WMNPNSGNTGYAQKFQG | SSGWNWFDP |
| | | | SEQ ID NO:5802 | SEQ ID NO:13814 | SEQ ID NO:21826 |
| **iPS:434655** | **21-225_78H12** | NA | AATTATGATATCAAC | TGGATGAACCCTAACAGTGGTAACACAGGCTATGCACAGAAGTTCCAGGGC | TCCAGTGGCTGGCACTGGTTCGACCCC |
| | | | SEQ ID NO:5803 | SEQ ID NO:13815 | SEQ ID NO:21827 |
| | | AA | NYDIN | WMNPNSGNTGYAQKFQG | SSGWHWFDP |
| | | | SEQ ID NO:5804 | SEQ ID NO:13816 | SEQ ID NO:21828 |
| **iPS:434657** | **21-225_79G1** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5805 | SEQ ID NO:13817 | SEQ ID NO:21829 |
| | | AA | GCYWS | EINHSGSTNYNPSLKS | DYGGMDV |
| | | | SEQ ID NO:5806 | SEQ ID NO:13818 | SEQ ID NO:21830 |
| **iPS:434663** | **21 225 79F3** | NA | GGTTGCTACTGGAGC | GAAATCAATCATAGTGGAAGCACCAACTACAACCCGTCCCTCAAGAGT | GACTACGGCGGTATGGACGTC |

**FIGURE 49**

| | | | | | |
|---|---|---|---|---|---|
| | **21-225_79F3** | | SEQ ID NO:5807 | SEQ ID NO:13819 | SEQ ID NO:21831 |
| | | AA | GCYWS | EINHSGSTNYNPSLKS | DYGGMDV |
| | | | SEQ ID NO:5808 | SEQ ID NO:13820 | SEQ ID NO:21832 |
| **iPS:434665** | **21-225_74G4** | NA | AATTATGATGTCAAC | TGGATGCACCCTAACAG TGGTAACACAGGCTATG CACAGAAGTTCCAGGGC | AGCAGTGGCTGGTACCATTT TGACTAC |
| | | | SEQ ID NO:5809 | SEQ ID NO:13821 | SEQ ID NO:21833 |
| | | AA | NYDVN | WMHPNSGNTGYAQKFQG | SSGWYHFDY |
| | | | SEQ ID NO:5810 | SEQ ID NO:13822 | SEQ ID NO:21834 |
| **iPS:434669** | **21-225_79F4** | NA | AACTATGGCATGCAC | GTTATATGGTATGATGG AAGTAATCAAAACTATG CAGACTCCGTGAAGGGC | GATGGCAGCTATGGTTATGA CGGCCTTGACTAC |
| | | | SEQ ID NO:5811 | SEQ ID NO:13823 | SEQ ID NO:21835 |
| | | AA | NYGMH | VIWYDGSNQNYADSVKG | DGSYGYDGLDY |
| | | | SEQ ID NO:5812 | SEQ ID NO:13824 | SEQ ID NO:21836 |
| **iPS:434671** | **21-225_74F4** | NA | AACGCCTGGATGAAC | CGAATTAAAAACAAAAT TGATGGTGGGACAACAG ACTACGCTGCACCCGTG AAAGGC | GTGGGAGCTACTACGGACTA C |
| | | | SEQ ID NO:5813 | SEQ ID NO:13825 | SEQ ID NO:21837 |
| | | AA | NAWMN | RIKNKIDGGTTDYAAPVK G | VGATTDY |
| | | | SEQ ID NO:5814 | SEQ ID NO:13826 | SEQ ID NO:21838 |
| **iPS:434673** | **21 225 74E3** | NA | AGCTATGGCATGCAC | GTTATTTGGTATGATGGA AGTAATAAATACTATGC AGACTCCGTGAAGGGC | GATCGCAATATAGTGGGAGC TACTTACTTTGAGTCC |

**FIGURE 49**

| | | | | | |
|---|---|---|---|---|---|
| | **21-225_74E3** | | SEQ ID NO:5815 | SEQ ID NO:13827 | SEQ ID NO:21839 |
| | | AA | SYGMH | VIWYDGSNKYYADSVKG | DRNIVGATYFES |
| | | | SEQ ID NO:5816 | SEQ ID NO:13828 | SEQ ID NO:21840 |
| **iPS:434675** | **21-225_79G6** | NA | AATTATGATATCAAC | TGGATGCACCCTAACAG TGGTAACACAGGCTTTG CACAGAAGTTCCAGGGC | TCCAGTGGCTGGTACTGGTT CGACCCC |
| | | | SEQ ID NO:5817 | SEQ ID NO:13829 | SEQ ID NO:21841 |
| | | AA | NYDIN | WMHPNSGNTGFAQKFQG | SSGWYWFDP |
| | | | SEQ ID NO:5818 | SEQ ID NO:13830 | SEQ ID NO:21842 |
| **iPS:434679** | **21-225_79G7** | NA | AATTATGATATCAAC | TGGATGCACCCTAACAG TGGTAACACAGGCTATG CACAGAAGTTCCAGGGC | AGTAGTGGCTGGTACAAATT TGACTAC |
| | | | SEQ ID NO:5819 | SEQ ID NO:13831 | SEQ ID NO:21843 |
| | | AA | NYDIN | WMHPNSGNTGYAQKFQG | SSGWYKFDY |
| | | | SEQ ID NO:5820 | SEQ ID NO:13832 | SEQ ID NO:21844 |
| **iPS:434685** | **21-225_79E9** | NA | AATTATGATATCAAC | TGGATGCATCCTAACAG TGGTAACACAGGCTATG CACAGAAGTTCCAGGGC | AGCAGTGGCTGGTACTACTT TGACTAC |
| | | | SEQ ID NO:5821 | SEQ ID NO:13833 | SEQ ID NO:21845 |
| | | AA | NYDIN | WMHPNSGNTGYAQKFQG | SSGWYYFDY |
| | | | SEQ ID NO:5822 | SEQ ID NO:13834 | SEQ ID NO:21846 |
| **iPS:434687** | **21-225_75A5** | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:5823 | SEQ ID NO:13835 | SEQ ID NO:21847 |
| | | AA | GCYWS | EINHSGSTNYNPSLKS | DYGGMDV |

FIGURE 49

| | | | | | |
|---|---|---|---|---|---|
| | | | SEQ ID NO:5824 | SEQ ID NO:13836 | SEQ ID NO:21848 |
| **iPS:434689** | **21-225_79G10** | NA | AATTATGATATCAAC | TGGATGAACCCTAACAG TGGTAACACAGGCTATG CACAGAAGTTCCAGGGC | TCCAGTGGCTGGAACTGGTT CGACCCC |
| | | | SEQ ID NO:5825 | SEQ ID NO:13837 | SEQ ID NO:21849 |
| | | AA | NYDIN | WMNPNSGNTGYAQKFQG | SSGWNWFDP |
| | | | SEQ ID NO:5826 | SEQ ID NO:13838 | SEQ ID NO:21850 |
| **iPS:434691** | **21-225_75G7** | NA | GGTTCCTACTGGAGC | GAAATCAATTATAGGGG AAGCACCAACTACAACC CGTCCCTCAAGAGT | GACTACGGCGGTATGGACGT C |
| | | | SEQ ID NO:5827 | SEQ ID NO:13839 | SEQ ID NO:21851 |
| | | AA | GSYWS | EINYRGSTNYNPSLKS | DYGGMDV |
| | | | SEQ ID NO:5828 | SEQ ID NO:13840 | SEQ ID NO:21852 |
| **iPS:434693** | **21-225_79F11** | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:5829 | SEQ ID NO:13841 | SEQ ID NO:21853 |
| | | AA | GCYWS | EINHSGSTNYNPSLKS | DYGGMDV |
| | | | SEQ ID NO:5830 | SEQ ID NO:13842 | SEQ ID NO:21854 |
| **iPS:434697** | **21-225_79F12** | NA | AATTATGATATCAAC | TGGATGAACCCTAACAG TGGTAACACAGGCTATG CACAGAAGTTCCAGGGC | AGCAGTGGCTGGTACTTCTT TGACTAC |
| | | | SEQ ID NO:5831 | SEQ ID NO:13843 | SEQ ID NO:21855 |
| | | AA | NYDIN | WMNPNSGNTGYAQKFQG | SSGWYFFDY |
| | | | SEQ ID NO:5832 | SEQ ID NO:13844 | SEQ ID NO:21856 |
| **iPS:434699** | **21 225 79G12** | NA | GGTTGCTACTGGAGC | GAAATCAATCATAGTGG AAGCACCAACTACAACC CGTCCCTCAAGAGT | GACTACGGCGGTATGGACGT C |

FIGURE 49

| | | | | | |
|---|---|---|---|---|---|
| | 21-225_79G12 | | SEQ ID NO:5833 | SEQ ID NO:13845 | SEQ ID NO:21857 |
| | | AA | GCYWS | EINHSGSTNYNPSLKS | DYGGMDV |
| | | | SEQ ID NO:5834 | SEQ ID NO:13846 | SEQ ID NO:21858 |
| **iPS:434701** | **21-225_80A1** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5835 | SEQ ID NO:13847 | SEQ ID NO:21859 |
| | | AA | GCYWS | EINHSGSTNYNPSLKS | DYGGMDV |
| | | | SEQ ID NO:5836 | SEQ ID NO:13848 | SEQ ID NO:21860 |
| **iPS:434703** | **21-225_80C1** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5837 | SEQ ID NO:13849 | SEQ ID NO:21861 |
| | | AA | GCYWS | EINHSGSTNYNPSLKS | DYGGMDV |
| | | | SEQ ID NO:5838 | SEQ ID NO:13850 | SEQ ID NO:21862 |
| **iPS:434705** | **21-225_80A2** | NA | AATTATGATATCAAC | TGGATGCACCCTAACAGTGGTAACACAGGCTATGCACAGAAGTTCCAGGGC | AGTAGTGGCTGGTACTGGTTCGACCCC |
| | | | SEQ ID NO:5839 | SEQ ID NO:13851 | SEQ ID NO:21863 |
| | | AA | NYDIN | WMHPNSGNTGYAQKFQG | SSGWYWFDP |
| | | | SEQ ID NO:5840 | SEQ ID NO:13852 | SEQ ID NO:21864 |
| **iPS:434707** | **21-225_80D3** | NA | AATTATGATATCAAC | TGGATGCACCCTAACAGTGGTAACACAGGCTATGCACAGAAGTTCCAGGGC | AGCAGTGGCTGGTACTGGTTCGACCCC |
| | | | SEQ ID NO:5841 | SEQ ID NO:13853 | SEQ ID NO:21865 |
| | | AA | NYDIN | WMHPNSGNTGYAQKFQG | SSGWYWFDP |
| | | | SEQ ID NO:5842 | SEQ ID NO:13854 | SEQ ID NO:21866 |

**FIGURE 49**

| | | | | | |
|---|---|---|---|---|---|
| **iPS:434709** | **21-225_80E3** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5843 | SEQ ID NO:13855 | SEQ ID NO:21867 |
| | | AA | GCYWS | EINHSGSTNYNPSLKS | DYGGMDV |
| | | | SEQ ID NO:5844 | SEQ ID NO:13856 | SEQ ID NO:21868 |
| **iPS:434711** | **21-225_80H3** | NA | AATTATGATATCAAC | TGGATGAACCCTAACAGTGGTAACACAGGCTATGCACAGAAGTTCCAGGGC | ACCAGTGGCTGGAACTTCTTTGACTAC |
| | | | SEQ ID NO:5845 | SEQ ID NO:13857 | SEQ ID NO:21869 |
| | | AA | NYDIN | WMNPNSGNTGYAQKFQG | TSGWNFFDY |
| | | | SEQ ID NO:5846 | SEQ ID NO:13858 | SEQ ID NO:21870 |
| **iPS:434715** | **21-225_80D5** | NA | GGTCCCTACTGGAGC | GAAATCAATCATAGTGGAAGCACCAACTACAACCCGTCCCTCAAGAGT | GACTACGGGGGTTTGGACGTC |
| | | | SEQ ID NO:5847 | SEQ ID NO:13859 | SEQ ID NO:21871 |
| | | AA | GPYWS | EINHSGSTNYNPSLKS | DYGGLDV |
| | | | SEQ ID NO:5848 | SEQ ID NO:13860 | SEQ ID NO:21872 |
| **iPS:434717** | **21-225_80A6** | NA | GGTTGCTACTGGAGC | GAAATCAATCATAGTGGAAGGACCAACTACAACCCGTCCCTCAAGAGT | GACTACGGTGGGCTTGACTAC |
| | | | SEQ ID NO:5849 | SEQ ID NO:13861 | SEQ ID NO:21873 |
| | | AA | GCYWS | EINHSGRTNYNPSLKS | DYGGLDY |
| | | | SEQ ID NO:5850 | SEQ ID NO:13862 | SEQ ID NO:21874 |
| **iPS:434725** | **21-225_80H7** | NA | GGTTCCTACTGGAGC | GAAATCAATCAAAGTGGACGCACCAACTACAACCCGTCCCTCAAGAGT | GACTACGGGGGTATAGACGTC |
| | | | SEQ ID NO:5851 | SEQ ID NO:13863 | SEQ ID NO:21875 |
| | | AA | GSYWS | EINQSGRTNYNPSLKS | DYGGIDV |

FIGURE 49

| | | | | | |
|---|---|---|---|---|---|
| | | | SEQ ID NO:5852 | SEQ ID NO:13864 | SEQ ID NO:21876 |
| **iPS:434729** | **21-225_80B12** | NA | AATTATGATATCAAC | TGGATGAACCCTAACAGTGGTAACACAGGCTATGCACAGAAGTTCCAGGTC | AGCAGTGGCTGGTACATCTTTGACTAC |
| | | | SEQ ID NO:5853 | SEQ ID NO:13865 | SEQ ID NO:21877 |
| | | AA | NYDIN | WMNPNSGNTGYAQKFQV | SSGWYIFDY |
| | | | SEQ ID NO:5854 | SEQ ID NO:13866 | SEQ ID NO:21878 |
| **iPS:434731** | **21-225_80E9** | NA | AATTATGATATCAAC | TGGATGCACCCTAACAGTGGTAACACAGGCTATGCACAGAAGTTCCAGGGC | AGCAGTGGCTGGTACTGGTTCGACCCC |
| | | | SEQ ID NO:5855 | SEQ ID NO:13867 | SEQ ID NO:21879 |
| | | AA | NYDIN | WMHPNSGNTGYAQKFQG | SSGWYWFDP |
| | | | SEQ ID NO:5856 | SEQ ID NO:13868 | SEQ ID NO:21880 |
| **iPS:434735** | **21-225_80B10** | NA | GGTTGCTACTGGAGC | GAAATCAATCATAGTGGAAGCACCAACTACAACCCGTCCCTCAAGAGT | GACTACGGTGGGCTTGACTAC |
| | | | SEQ ID NO:5857 | SEQ ID NO:13869 | SEQ ID NO:21881 |
| | | AA | GCYWS | EINHSGSTNYNPSLKS | DYGGLDY |
| | | | SEQ ID NO:5858 | SEQ ID NO:13870 | SEQ ID NO:21882 |
| **iPS:434737** | **21-225_74G6** | NA | AGCTATGGCATGCAC | GTTATATGGTATGATGGAAGTAATAAAAACTATGCAGACTCCGTGAAGGGC | GATGGCAGCTATGGTTATGACGGCCTTGACTAC |
| | | | SEQ ID NO:5859 | SEQ ID NO:13871 | SEQ ID NO:21883 |
| | | AA | SYGMH | VIWYDGSNKNYADSVKG | DGSYGYDGLDY |
| | | | SEQ ID NO:5860 | SEQ ID NO:13872 | SEQ ID NO:21884 |

## FIGURE 49

| | | | | | |
|---|---|---|---|---|---|
| **iPS:434741** | **21-225_80C11** | NA | AGCTATGGCATGCAC | GTTATATGGTATGATGG AAGTAATAAAAACTATG CAGACTCCGTGAAGGGC | GATGGCAGCTATGGGTATGA CGGCCTTGACTAC |
| | | | SEQ ID NO:5861 | SEQ ID NO:13873 | SEQ ID NO:21885 |
| | | AA | SYGMH | VIWYDGSNKNYADSVKG | DGSYGYDGLDY |
| | | | SEQ ID NO:5862 | SEQ ID NO:13874 | SEQ ID NO:21886 |
| **iPS:434743** | **21-225_74A4** | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:5863 | SEQ ID NO:13875 | SEQ ID NO:21887 |
| | | AA | GCYWS | EINHSGSTNYNPSLKS | DYGGMDV |
| | | | SEQ ID NO:5864 | SEQ ID NO:13876 | SEQ ID NO:21888 |
| **iPS:434747** | **21-225_80C12** | NA | AATTATGATATCAAC | TGGATGCACCCTAACAA TGGTAACACAGGCTATG CACAGAAGTTCCAGGGC | AGCAGTGGCTGGTACTGGTT CGACCCC |
| | | | SEQ ID NO:5865 | SEQ ID NO:13877 | SEQ ID NO:21889 |
| | | AA | NYDIN | WMHPNNGNTGYAQKFQG | SSGWYWFDP |
| | | | SEQ ID NO:5866 | SEQ ID NO:13878 | SEQ ID NO:21890 |
| **iPS:434751** | **21-225_80H12** | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:5867 | SEQ ID NO:13879 | SEQ ID NO:21891 |
| | | AA | GCYWS | EINHSGSTNYNPSLKS | DYGGMDV |
| | | | SEQ ID NO:5868 | SEQ ID NO:13880 | SEQ ID NO:21892 |
| **iPS:434759** | **21 225 81C5** | NA | GGTTGCTACTGGAGC | GAAATCAATCATAGTGG AAGCACCAACTACAACC CGTCCCTCAAGAGT | GACTACGGCGGTATGGACGT C |

FIGURE 49

| | | | | | |
|---|---|---|---|---|---|
| | 21-225_81C5 | | SEQ ID NO:5869 | SEQ ID NO:13881 | SEQ ID NO:21893 |
| | | AA | GCYWS | EINHSGSTNYNPSLKS | DYGGMDV |
| | | | SEQ ID NO:5870 | SEQ ID NO:13882 | SEQ ID NO:21894 |
| **iPS:434761** | **21-225_81E5** | NA | AATTATGATATCAAC | TGGATGAACCCTAACAG TGGTAACACAGGCTATG CACAGAAGTTCCAGGGC | TCCAGTGGCTGGAACTGGTT CGACCCC |
| | | | SEQ ID NO:5871 | SEQ ID NO:13883 | SEQ ID NO:21895 |
| | | AA | NYDIN | WMNPNSGNTGYAQKFQG | SSGWNWFDP |
| | | | SEQ ID NO:5872 | SEQ ID NO:13884 | SEQ ID NO:21896 |
| **iPS:434771** | **21-225_81F9** | NA | AATTATGATATCAAC | TGGATGCACCCTAACAG TGGTAACACAGGCTATG CACAGAAGTTCCAGGGC | AGCAGTGGCTGGTACTGGTT CGACCCC |
| | | | SEQ ID NO:5873 | SEQ ID NO:13885 | SEQ ID NO:21897 |
| | | AA | NYDIN | WMHPNSGNTGYAQKFQG | SSGWYWFDP |
| | | | SEQ ID NO:5874 | SEQ ID NO:13886 | SEQ ID NO:21898 |
| **iPS:434773** | **21-225_75D9** | NA | GGTCCCTACTGGAGC | GAAATCAATTATAGGGG AAGCACCAACTACAACC CGTCCCTCAAGAGT | GACTACGGCGGTATGGACGT C |
| | | | SEQ ID NO:5875 | SEQ ID NO:13887 | SEQ ID NO:21899 |
| | | AA | GPYWS | EINYRGSTNYNPSLKS | DYGGMDV |
| | | | SEQ ID NO:5876 | SEQ ID NO:13888 | SEQ ID NO:21900 |
| **iPS:434777** | **21-225_81C11** | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:5877 | SEQ ID NO:13889 | SEQ ID NO:21901 |
| | | AA | GCYWS | EINHSGSTNYNPSLKS | DYGGMDV |
| | | | SEQ ID NO:5878 | SEQ ID NO:13890 | SEQ ID NO:21902 |

FIGURE 49

| | | | | | |
|---|---|---|---|---|---|
| **iPS:434793** | **21-225_82A5** | NA | AATTATGATATCAAC | TGGATGCACCCTAACAATGGTAACACAGGCTATGCACAGAAGTTCCAGGGC | AGCAGTGGCTGGTACTGGTTCGACCCC |
| | | | SEQ ID NO:5879 | SEQ ID NO:13891 | SEQ ID NO:21903 |
| | | AA | NYDIN | WMHPNNGNTGYAQKFQG | SSGWYWFDP |
| | | | SEQ ID NO:5880 | SEQ ID NO:13892 | SEQ ID NO:21904 |
| **iPS:434797** | **21-225_82G5** | NA | AATTATGATATCAAC | TGGATGCACCCTAACAGTGGTAACACAGGCTATGCACAGAAGTTCCAGGGC | AGTAGTGGCTGGTACTGGTTCGACCCC |
| | | | SEQ ID NO:5881 | SEQ ID NO:13893 | SEQ ID NO:21905 |
| | | AA | NYDIN | WMHPNSGNTGYAQKFQG | SSGWYWFDP |
| | | | SEQ ID NO:5882 | SEQ ID NO:13894 | SEQ ID NO:21906 |
| **iPS:434805** | **21-225_82D9** | NA | AATTATGATATCAAC | TGGATGCACCCTAACAGTGGTAACACAGGCTATGCACAGAAGTTCCAGGGC | AGTAGTGGCTGGTACTGGTTCGACCCC |
| | | | SEQ ID NO:5883 | SEQ ID NO:13895 | SEQ ID NO:21907 |
| | | AA | NYDIN | WMHPNSGNTGYAQKFQG | SSGWYWFDP |
| | | | SEQ ID NO:5884 | SEQ ID NO:13896 | SEQ ID NO:21908 |
| **iPS:434809** | **21-225_74F5** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5885 | SEQ ID NO:13897 | SEQ ID NO:21909 |
| | | AA | GCYWS | EINHSGSTNYNPSLKS | DYGGMDV |
| | | | SEQ ID NO:5886 | SEQ ID NO:13898 | SEQ ID NO:21910 |
| **iPS:434813** | | NA | AATTATGATATCAAC | TGGATGCACCCTAACAATGGTAACACAGGCTATGCACAGAAGTTCCAGGGC | AGCAGTGGCTGGTACTGGTTCGACCCC |

FIGURE 49

| | | | | | |
|---|---|---|---|---|---|
| | **21-225_82C12** | | SEQ ID NO:5887 | SEQ ID NO:13899 | SEQ ID NO:21911 |
| | | AA | NYDIN | WMHPNNGNTGYAQKFQG | SSGWYWFDP |
| | | | SEQ ID NO:5888 | SEQ ID NO:13900 | SEQ ID NO:21912 |
| **iPS:434815** | **21-225_74A11** | NA | AGTTATGATATCAAC | TGGATGAACCCTAACAG TGGTAACACAGGCTATG CACAGAAGTTCCAGGGC | GGCTTTTACGATACTTTGACT GGTTCCGGCTACTACTACGT TATGGACGTC |
| | | | SEQ ID NO:5889 | SEQ ID NO:13901 | SEQ ID NO:21913 |
| | | AA | SYDIN | WMNPNSGNTGYAQKFQG | GFYDTLTGSGYYYVMDV |
| | | | SEQ ID NO:5890 | SEQ ID NO:13902 | SEQ ID NO:21914 |
| **iPS:434821** | **21-225_83G1** | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:5891 | SEQ ID NO:13903 | SEQ ID NO:21915 |
| | | AA | GCYWS | EINHSGSTNYNPSLKS | DYGGMDV |
| | | | SEQ ID NO:5892 | SEQ ID NO:13904 | SEQ ID NO:21916 |
| **iPS:434825** | **21-225_83C2** | NA | AATTATGATATCAAC | TGGATGCACCCTAACAG TGGTAACACAGGCTATG CACAGAAGTTCCAGGGC | AGTAGTGGCTGGTACTGGTT CGACCCC |
| | | | SEQ ID NO:5893 | SEQ ID NO:13905 | SEQ ID NO:21917 |
| | | AA | NYDIN | WMHPNSGNTGYAQKFQG | SSGWYWFDP |
| | | | SEQ ID NO:5894 | SEQ ID NO:13906 | SEQ ID NO:21918 |
| **iPS:434827** | **21-225_83F3** | NA | AATTATGATATCAAC | TGGATGCACCCTAACAG TGGTAACACAGGCTATG CACAGAAGTTCCAGGGC | AGCAGTGGCTGGTACTGGTT CGACCCC |
| | | | SEQ ID NO:5895 | SEQ ID NO:13907 | SEQ ID NO:21919 |
| | | AA | NYDIN | WMHPNSGNTGYAQKFQG | SSGWYWFDP |

FIGURE 49

| | | | | | |
|---|---|---|---|---|---|
| | | | SEQ ID NO:5896 | SEQ ID NO:13908 | SEQ ID NO:21920 |
| **iPS:434829** | **21-225_83G3** | NA | AATTATGATATCAAC | TGGATGCACCCTAACAGTGGTAACACAGGCTATGCACAGAAGTTCCAGGGC | AGCAGTGGCTGGTACTGGTTCGACCCC |
| | | | SEQ ID NO:5897 | SEQ ID NO:13909 | SEQ ID NO:21921 |
| | | AA | NYDIN | WMHPNSGNTGYAQKFQG | SSGWYWFDP |
| | | | SEQ ID NO:5898 | SEQ ID NO:13910 | SEQ ID NO:21922 |
| **iPS:434833** | **21-225_83C5** | NA | AATTATGATATCAAC | TGGATGCACCCTAACAGTGGTAACACAGGCTATGCACAGAAGTTCCAGGGC | AGTAGTGGCTGGTACTGGTTCGACCCC |
| | | | SEQ ID NO:5899 | SEQ ID NO:13911 | SEQ ID NO:21923 |
| | | AA | NYDIN | WMHPNSGNTGYAQKFQG | SSGWYWFDP |
| | | | SEQ ID NO:5900 | SEQ ID NO:13912 | SEQ ID NO:21924 |
| **iPS:434835** | **21-225_83B6** | NA | GGTTGTTACTGGAGC | GAAATCAATCATAGTGGAAGGACCAACTACAACCCCTCCCTCAAGAGT | GACTACGGTGGGCTTGACTAC |
| | | | SEQ ID NO:5901 | SEQ ID NO:13913 | SEQ ID NO:21925 |
| | | AA | GCYWS | EINHSGRTNYNPSLKS | DYGGLDY |
| | | | SEQ ID NO:5902 | SEQ ID NO:13914 | SEQ ID NO:21926 |
| **iPS:434839** | **21-225_83B7** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5903 | SEQ ID NO:13915 | SEQ ID NO:21927 |
| | | AA | GCYWS | EINHSGSTNYNPSLKS | DYGGMDV |
| | | | SEQ ID NO:5904 | SEQ ID NO:13916 | SEQ ID NO:21928 |
| **iPS:434841** | | NA | AATTATGATATCAAC | TGGATGAACCCTAACAGTGGTAACACAGGCTATGCACAGAAGTTCCAGGGC | TCCAGTGGCTGGCACTGGTTCGACCCC |

**FIGURE 49**

| | | | | | |
|---|---|---|---|---|---|
| | **21-225_83G7** | | SEQ ID NO:5905 | SEQ ID NO:13917 | SEQ ID NO:21929 |
| | | AA | NYDIN | WMNPNSGNTGYAQKFQG | SSGWHWFDP |
| | | | SEQ ID NO:5906 | SEQ ID NO:13918 | SEQ ID NO:21930 |
| **iPS:434849** | **21-225_83C10** | NA | GGTTACTACTGGAGC | GAAATCAATCATAGTGG AAGCACCAACTTCAACC CGTCCCTCAAGAGT | GACTACGGTGGGCTTGACTA C |
| | | | SEQ ID NO:5907 | SEQ ID NO:13919 | SEQ ID NO:21931 |
| | | AA | GYYWS | EINHSGSTNFNPSLKS | DYGGLDY |
| | | | SEQ ID NO:5908 | SEQ ID NO:13920 | SEQ ID NO:21932 |
| **iPS:434851** | **21-225_75A6** | NA | AATTATGATATCAAC | TGGATGAACCCTAACAG TGGTAACACAGGCTATG CACAGAAGTTCCAGGGC | AGCAGTGGCTGGTACATCTT TGACTAC |
| | | | SEQ ID NO:5909 | SEQ ID NO:13921 | SEQ ID NO:21933 |
| | | AA | NYDIN | WMNPNSGNTGYAQKFQG | SSGWYIFDY |
| | | | SEQ ID NO:5910 | SEQ ID NO:13922 | SEQ ID NO:21934 |
| **iPS:434863** | **21-225_84G7** | NA | AATTATGATATCAAC | TGGATGAACCCTAACAG TGGTAACACAGGCTATG CACAGAAGTTCCAGGAC | TCCAGTGGCTGGCACTGGTT CGACCCC |
| | | | SEQ ID NO:5911 | SEQ ID NO:13923 | SEQ ID NO:21935 |
| | | AA | NYDIN | WMNPNSGNTGYAQKFQD | SSGWHWFDP |
| | | | SEQ ID NO:5912 | SEQ ID NO:13924 | SEQ ID NO:21936 |
| **iPS:434867** | **21-225_79A12** | NA | AGCTATGGCATGCAC | GTTATATGGTATGATGG AAGTAATAAAAACTATG CAGACTCCGTGAAGGGC | GATGGCAGCTATGGTTACGA CGGCCTTGACTAC |
| | | | SEQ ID NO:5913 | SEQ ID NO:13925 | SEQ ID NO:21937 |

**FIGURE 49**

| | | | | | |
|---|---|---|---|---|---|
| | | AA | SYGMH | VIWYDGSNKNYADSVKG | DGSYGYDGLDY |
| | | | SEQ ID NO:5914 | SEQ ID NO:13926 | SEQ ID NO:21938 |
| **iPS:434869** | **21-225_84E12** | NA | GGTTCCTACTGGAGC | GAAATCAATCAAAGTGGACGCACCAACTACAACCCGTCCCTCAAGAGT | GACTACGGGGGTATAGACGTC |
| | | | SEQ ID NO:5915 | SEQ ID NO:13927 | SEQ ID NO:21939 |
| | | AA | GSYWS | EINQSGRTNYNPSLKS | DYGGIDV |
| | | | SEQ ID NO:5916 | SEQ ID NO:13928 | SEQ ID NO:21940 |
| **iPS:434871** | **21-225_85H1** | NA | AGCTATGGCATACAC | GTTATATGGTATGATGGAAGTAATAAATACTATGCAGACTCCGTGAAGGGC | GATCCCTTTATAGTGGGAGCTACTTACTTTGACTAC |
| | | | SEQ ID NO:5917 | SEQ ID NO:13929 | SEQ ID NO:21941 |
| | | AA | SYGIH | VIWYDGSNKYYADSVKG | DPFIVGATYFDY |
| | | | SEQ ID NO:5918 | SEQ ID NO:13930 | SEQ ID NO:21942 |
| **iPS:434877** | **21-225_85H2** | NA | AATTATGATATCAAC | TGGATGCACCCTAATAGTGGTAACACAGGCTATGCACAGAAGTTCCAGGGC | TCCAGTGGCTGGTACTGGTTCGACCCC |
| | | | SEQ ID NO:5919 | SEQ ID NO:13931 | SEQ ID NO:21943 |
| | | AA | NYDIN | WMHPNSGNTGYAQKFQG | SSGWYWFDP |
| | | | SEQ ID NO:5920 | SEQ ID NO:13932 | SEQ ID NO:21944 |
| **iPS:434879** | **21-225_85A3** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5921 | SEQ ID NO:13933 | SEQ ID NO:21945 |
| | | AA | GCYWS | EINHSGSTNYNPSLKS | DYGGMDV |
| | | | SEQ ID NO:5922 | SEQ ID NO:13934 | SEQ ID NO:21946 |

**FIGURE 49**

| | | | | | |
|---|---|---|---|---|---|
| **iPS:434881** | **21-225_85B4** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5923 | SEQ ID NO:13935 | SEQ ID NO:21947 |
| | | AA | GCYWS | EINHSGSTNYNPSLKS | DYGGMDV |
| | | | SEQ ID NO:5924 | SEQ ID NO:13936 | SEQ ID NO:21948 |
| **iPS:434883** | **21-225_85B5** | NA | AATTATGATATCAAC | TGGATGCACCCTAACAGTGGTAACACAGGCTATGCACAGAAGTTCCAGGGC | AGTAGTGGCTGGTACTGGTTCGACCCC |
| | | | SEQ ID NO:5925 | SEQ ID NO:13937 | SEQ ID NO:21949 |
| | | AA | NYDIN | WMHPNSGNTGYAQKFQG | SSGWYWFDP |
| | | | SEQ ID NO:5926 | SEQ ID NO:13938 | SEQ ID NO:21950 |
| **iPS:434887** | **21-225_85D6** | NA | GGTTGCTACTGGAGC | GAAATCAATTATAGTGGAAGAACCAACTACAACCCGTCCCTCAAGAGT | GACTACGGCGGTATGGACGTC |
| | | | SEQ ID NO:5927 | SEQ ID NO:13939 | SEQ ID NO:21951 |
| | | AA | GCYWS | EINYSGRTNYNPSLKS | DYGGMDV |
| | | | SEQ ID NO:5928 | SEQ ID NO:13940 | SEQ ID NO:21952 |
| **iPS:434891** | **21-225_85G6** | NA | GATTGCTACTGGAGC | GAAATCAATCATAGTGGAAGGACCAACTACAACCCGTCCCTCAAGAGT | GACTACGGTGGGCTTGACTAC |
| | | | SEQ ID NO:5929 | SEQ ID NO:13941 | SEQ ID NO:21953 |
| | | AA | DCYWS | EINHSGRTNYNPSLKS | DYGGLDY |
| | | | SEQ ID NO:5930 | SEQ ID NO:13942 | SEQ ID NO:21954 |
| **iPS:434895** | **21-225_74H7** | NA | GGTCCCTACTGGAGC | GAAATCAATCATAGTGGAAGCACCAACTACAACCCGTCCCTCAAGAGT | GACTACGGGGGTTTGGACGTC |
| | | | SEQ ID NO:5931 | SEQ ID NO:13943 | SEQ ID NO:21955 |
| | | AA | GPYWS | EINHSGSTNYNPSLKS | DYGGLDV |

FIGURE 49

| | | | | | |
|---|---|---|---|---|---|
| | | | SEQ ID NO:5932 | SEQ ID NO:13944 | SEQ ID NO:21956 |
| **iPS:434899** | **21-225_85B9** | NA | GGTTGCTACTGGAGC | GAAATCAATCATAGTGGAAGGACCAACTTCAACCCGTCCCTCAAGAGT | GACTACGGCGGTATGGACGTC |
| | | | SEQ ID NO:5933 | SEQ ID NO:13945 | SEQ ID NO:21957 |
| | | AA | GCYWS | EINHSGRTNFNPSLKS | DYGGMDV |
| | | | SEQ ID NO:5934 | SEQ ID NO:13946 | SEQ ID NO:21958 |
| **iPS:434901** | **21-225_85H9** | NA | AATTATGATATCAAC | TGGATGAACCCTAACAGTGGTAACACAGGCTATGCACAGAAGTTCCAGGGC | ACCAGTGGCTGGAACTTCTTTGACTAC |
| | | | SEQ ID NO:5935 | SEQ ID NO:13947 | SEQ ID NO:21959 |
| | | AA | NYDIN | WMNPNSGNTGYAQKFQG | TSGWNFFDY |
| | | | SEQ ID NO:5936 | SEQ ID NO:13948 | SEQ ID NO:21960 |
| **iPS:434907** | **21-225_85G10** | NA | GGTTGTTACTGGAGC | GAAATCAATCATAGTGGAATCACCAACTACAACCCGTCCCTCAAGAGT | GACTACGGTGGTTTGGACGTC |
| | | | SEQ ID NO:5937 | SEQ ID NO:13949 | SEQ ID NO:21961 |
| | | AA | GCYWS | EINHSGITNYNPSLKS | DYGGLDV |
| | | | SEQ ID NO:5938 | SEQ ID NO:13950 | SEQ ID NO:21962 |
| **iPS:434909** | **21-225_85C11** | NA | AATTATGATATCAAC | TGGATGCACCCTAACAGTGGTAACACAGGCTATGCACAGAAGTTCCAGGGC | AGTAGTGGCTGGTACAAATTTGACTAC |
| | | | SEQ ID NO:5939 | SEQ ID NO:13951 | SEQ ID NO:21963 |
| | | AA | NYDIN | WMHPNSGNTGYAQKFQG | SSGWYKFDY |
| | | | SEQ ID NO:5940 | SEQ ID NO:13952 | SEQ ID NO:21964 |
| **iPS:434911** | | NA | AATTATGATATCAAC | TGGATGCACCCTAACAGTGGTAACACAGGCTATGCACAGAAGTTCCAGGGC | AGTAGTGGCTGGTACTGGTTCGACCCC |

FIGURE 49

| | | | | | |
|---|---|---|---|---|---|
| | **21-225_85D11** | | SEQ ID NO:5941 | SEQ ID NO:13953 | SEQ ID NO:21965 |
| | | AA | NYDIN | WMHPNSGNTGYAQKFQG | SSGWYWFDP |
| | | | SEQ ID NO:5942 | SEQ ID NO:13954 | SEQ ID NO:21966 |
| **iPS:434913** | **21-225_86C1** | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:5943 | SEQ ID NO:13955 | SEQ ID NO:21967 |
| | | AA | GCYWS | EINHSGSTNYNPSLKS | DYGGMDV |
| | | | SEQ ID NO:5944 | SEQ ID NO:13956 | SEQ ID NO:21968 |
| **iPS:434921** | **21-225_86E4** | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:5945 | SEQ ID NO:13957 | SEQ ID NO:21969 |
| | | AA | GCYWS | EINHSGSTNYNPSLKS | DYGGMDV |
| | | | SEQ ID NO:5946 | SEQ ID NO:13958 | SEQ ID NO:21970 |
| **iPS:434935** | **21-225_86E9** | NA | AATTATGATATCAAC | TGGATGAACCCTAACAG TGGTAACACAGGCTATG CACAGAAGTTCCAGGGC | TCCAGTGGCTGGTCCTGGTT CGACCCC |
| | | | SEQ ID NO:5947 | SEQ ID NO:13959 | SEQ ID NO:21971 |
| | | AA | NYDIN | WMNPNSGNTGYAQKFQG | SSGWSWFDP |
| | | | SEQ ID NO:5948 | SEQ ID NO:13960 | SEQ ID NO:21972 |
| **iPS:434939** | **21-225_86C11** | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:5949 | SEQ ID NO:13961 | SEQ ID NO:21973 |
| | | AA | GCYWS | EINHSGSTNYNPSLKS | DYGGMDV |
| | | | SEQ ID NO:5950 | SEQ ID NO:13962 | SEQ ID NO:21974 |

**FIGURE 49**

| | | | | | |
|---|---|---|---|---|---|
| **iPS:434943** | **21-225_87H1** | NA | GGTTACTACTGGAGC | GAAATCAATCATAGTGGACGCACCAACTACAACCCGTCCCTCAAGAGT | GACTACGGTGGTTTGGACGTC |
| | | | SEQ ID NO:5951 | SEQ ID NO:13963 | SEQ ID NO:21975 |
| | | AA | GYYWS | EINHSGRTNYNPSLKS | DYGGLDV |
| | | | SEQ ID NO:5952 | SEQ ID NO:13964 | SEQ ID NO:21976 |
| **iPS:434945** | **21-225_87E5** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5953 | SEQ ID NO:13965 | SEQ ID NO:21977 |
| | | AA | GCYWS | EINHSGSTNYNPSLKS | DYGGMDV |
| | | | SEQ ID NO:5954 | SEQ ID NO:13966 | SEQ ID NO:21978 |
| **iPS:434947** | **21-225_87B7** | NA | AGCTATGGCATGCAC | GTTATATGGTATGATGGAAGTAATAAAAACTATGCAGACTCCGTGAAGGGC | GATTTTGGAGTGGGCTACTACGGTATGGACGTC |
| | | | SEQ ID NO:5955 | SEQ ID NO:13967 | SEQ ID NO:21979 |
| | | AA | SYGMH | VIWYDGSNKNYADSVKG | DFGVGYYGMDV |
| | | | SEQ ID NO:5956 | SEQ ID NO:13968 | SEQ ID NO:21980 |
| **iPS:434955** | **21-225_87C9** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5957 | SEQ ID NO:13969 | SEQ ID NO:21981 |
| | | AA | GCYWS | EINHSGSTNYNPSLKS | DYGGMDV |
| | | | SEQ ID NO:5958 | SEQ ID NO:13970 | SEQ ID NO:21982 |
| **iPS:434957** | **21-225_87A10** | NA | AATTATGATATCAAC | TGGATGCACCCTAACAATGGTAACACAGGCTATGCACAGAAGTTCCAGGGC | AGCAGTGGCTGGTACTGGTTCGACCCC |
| | | | SEQ ID NO:5959 | SEQ ID NO:13971 | SEQ ID NO:21983 |

**FIGURE 49**

| | | | | | |
|---|---|---|---|---|---|
| | | AA | NYDIN | WMHPNNGNTGYAQKFQG | SSGWYWFDP |
| | | | SEQ ID NO:5960 | SEQ ID NO:13972 | SEQ ID NO:21984 |
| **iPS:434959** | **21-225_87E10** | NA | AATTATGATATCAAC | TGGATGAACCCTAACAGTGGTAACACAGGCTATGCACAGAAGTTCCAGGGC | AGCAGTGGCTGGTACTTCTTTGACTAC |
| | | | SEQ ID NO:5961 | SEQ ID NO:13973 | SEQ ID NO:21985 |
| | | AA | NYDIN | WMNPNSGNTGYAQKFQG | SSGWYFFDY |
| | | | SEQ ID NO:5962 | SEQ ID NO:13974 | SEQ ID NO:21986 |
| **iPS:434961** | **21-225_87A12** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5963 | SEQ ID NO:13975 | SEQ ID NO:21987 |
| | | AA | GCYWS | EINHSGSTNYNPSLKS | DYGGMDV |
| | | | SEQ ID NO:5964 | SEQ ID NO:13976 | SEQ ID NO:21988 |
| **iPS:434965** | **21-225_88A1** | NA | AATTATGATATCAAC | TGGATGAACCCTAACAGTGGTAACACAGGCTATGCACAGAAGTTCCAGGGC | AGCAGTGGCTGGTACTACTTTGACTAC |
| | | | SEQ ID NO:5965 | SEQ ID NO:13977 | SEQ ID NO:21989 |
| | | AA | NYDIN | WMNPNSGNTGYAQKFQG | SSGWYYFDY |
| | | | SEQ ID NO:5966 | SEQ ID NO:13978 | SEQ ID NO:21990 |
| **iPS:434969** | **21-225_88H1** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5967 | SEQ ID NO:13979 | SEQ ID NO:21991 |
| | | AA | GCYWS | EINHSGSTNYNPSLKS | DYGGMDV |
| | | | SEQ ID NO:5968 | SEQ ID NO:13980 | SEQ ID NO:21992 |

FIGURE 49

| | | | | | |
|---|---|---|---|---|---|
| **iPS:434971** | **21-225_88G2** | NA | AATTATGATATCAAC | TGGATGCACCCTAACAGTGGTAACACAGGCTATGCACAGAAGTTCCAGGGC | AGCAGTGGCTGGTACTGGTTCGACCCC |
| | | | SEQ ID NO:5969 | SEQ ID NO:13981 | SEQ ID NO:21993 |
| | | AA | NYDIN | WMHPNSGNTGYAQKFQG | SSGWYWFDP |
| | | | SEQ ID NO:5970 | SEQ ID NO:13982 | SEQ ID NO:21994 |
| **iPS:434973** | **21-225_88B4** | NA | AATTATGATATCAAC | TGGATGAACCCTAACAGTGGTGACACAGGCTATGCACAGAAGTTCCAGGGC | AGCAGTGGCTGGTACTACTTTGACTAC |
| | | | SEQ ID NO:5971 | SEQ ID NO:13983 | SEQ ID NO:21995 |
| | | AA | NYDIN | WMNPNSGDTGYAQKFQG | SSGWYYFDY |
| | | | SEQ ID NO:5972 | SEQ ID NO:13984 | SEQ ID NO:21996 |
| **iPS:434977** | **21-225_88A5** | NA | AGTTATGATATCAAC | TGGATGAACCCTAACAGTGGTAACACAGGCTATGCACAGAAGTTCCAGGGC | GGGTTTTACGATTTTTGACTGGTTATTCCCCCACCTACTACTACTACGATATGGACGTC |
| | | | SEQ ID NO:5973 | SEQ ID NO:13985 | SEQ ID NO:21997 |
| | | AA | SYDIN | WMNPNSGNTGYAQKFQG | GFYDFLTGYSPTYYYYDMDV |
| | | | SEQ ID NO:5974 | SEQ ID NO:13986 | SEQ ID NO:21998 |
| **iPS:434981** | **21-225_88E7** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5975 | SEQ ID NO:13987 | SEQ ID NO:21999 |
| | | AA | GCYWS | EINHSGSTNYNPSLKS | DYGGMDV |
| | | | SEQ ID NO:5976 | SEQ ID NO:13988 | SEQ ID NO:22000 |

**FIGURE 49**

| | | | | | |
|---|---|---|---|---|---|
| **iPS:434983** | **21-225_88F7** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5977 | SEQ ID NO:13989 | SEQ ID NO:22001 |
| | | AA | GCYWS | EINHSGSTNYNPSLKS | DYGGMDV |
| | | | SEQ ID NO:5978 | SEQ ID NO:13990 | SEQ ID NO:22002 |
| **iPS:434995** | **21-225_88G9** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5979 | SEQ ID NO:13991 | SEQ ID NO:22003 |
| | | AA | GCYWS | EINHSGSTNYNPSLKS | DYGGMDV |
| | | | SEQ ID NO:5980 | SEQ ID NO:13992 | SEQ ID NO:22004 |
| **iPS:434997** | **21-225_88C10** | NA | AATTATGATATCAAC | TGGATGACCCCTAACAGTGGTAACACAGGCTATGCACAGAAGTTCCAGGGC | AGCAGTGGCTGGTACTACTTTGACTCC |
| | | | SEQ ID NO:5981 | SEQ ID NO:13993 | SEQ ID NO:22005 |
| | | AA | NYDIN | WMTPNSGNTGYAQKFQG | SSGWYYFDS |
| | | | SEQ ID NO:5982 | SEQ ID NO:13994 | SEQ ID NO:22006 |
| **iPS:434999** | **21-225_75A8** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5983 | SEQ ID NO:13995 | SEQ ID NO:22007 |
| | | AA | GCYWS | EINHSGSTNYNPSLKS | DYGGMDV |
| | | | SEQ ID NO:5984 | SEQ ID NO:13996 | SEQ ID NO:22008 |
| **iPS:435009** | **21-225_89G4** | NA | AGCTACGACATGCAC | GCTATTGGTACTGCTGGTGACACATACTATCCAGGCTCCGTGAAGGGC | GCTCTTGACTACGGTGACTCCTTGGGCTACTACTACTACGGTATGGACGTC |
| | | | SEQ ID NO:5985 | SEQ ID NO:13997 | SEQ ID NO:22009 |
| | | AA | SYDMH | AIGTAGDTYYPGSVKG | ALDYGDSLGYYYYGMDV |

**FIGURE 49**

| | | | | | |
|---|---|---|---|---|---|
| | | | SEQ ID NO:5986 | SEQ ID NO:13998 | SEQ ID NO:22010 |
| **iPS:435013** | **21-225_89D5** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5987 | SEQ ID NO:13999 | SEQ ID NO:22011 |
| | | AA | GCYWS | EINHSGSTNYNPSLKS | DYGGMDV |
| | | | SEQ ID NO:5988 | SEQ ID NO:14000 | SEQ ID NO:22012 |
| **iPS:435015** | **21-225_89H5** | NA | GGTTGCTACTGGAGC | GAAATCAATTATAGTGGAAGCACCAACTTCAACCCGTCCCTCAAGAGT | GACTACGGCGGTATGGACGTC |
| | | | SEQ ID NO:5989 | SEQ ID NO:14001 | SEQ ID NO:22013 |
| | | AA | GCYWS | EINYSGSTNFNPSLKS | DYGGMDV |
| | | | SEQ ID NO:5990 | SEQ ID NO:14002 | SEQ ID NO:22014 |
| **iPS:435025** | **21-225_89E10** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5991 | SEQ ID NO:14003 | SEQ ID NO:22015 |
| | | AA | GCYWS | EINHSGSTNYNPSLKS | DYGGMDV |
| | | | SEQ ID NO:5992 | SEQ ID NO:14004 | SEQ ID NO:22016 |
| **iPS:435029** | **21-225_89A11** | NA | GGTTACTACTGGAGC | GAAATCAATCATAGTGGACGCACCAGCTACAACCCGTCCCTCAAGAGT | GACTACGGTGGTTTGGACGTC |
| | | | SEQ ID NO:5993 | SEQ ID NO:14005 | SEQ ID NO:22017 |
| | | AA | GYYWS | EINHSGRTSYNPSLKS | DYGGLDV |
| | | | SEQ ID NO:5994 | SEQ ID NO:14006 | SEQ ID NO:22018 |
| **iPS:435039** | **21-225_90G4** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5995 | SEQ ID NO:14007 | SEQ ID NO:22019 |
| | | AA | GCYWS | EINHSGSTNYNPSLKS | DYGGMDV |

**FIGURE 49**

| | | | | | |
|---|---|---|---|---|---|
| | | | SEQ ID NO:5996 | SEQ ID NO:14008 | SEQ ID NO:22020 |
| **iPS:435041** | **21-225_90A5** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5997 | SEQ ID NO:14009 | SEQ ID NO:22021 |
| | | AA | GCYWS | EINHSGSTNYNPSLKS | DYGGMDV |
| | | | SEQ ID NO:5998 | SEQ ID NO:14010 | SEQ ID NO:22022 |
| **iPS:435043** | **21-225_90G5** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:5999 | SEQ ID NO:14011 | SEQ ID NO:22023 |
| | | AA | GCYWS | EINHSGSTNYNPSLKS | DYGGMDV |
| | | | SEQ ID NO:6000 | SEQ ID NO:14012 | SEQ ID NO:22024 |
| **iPS:435045** | **21-225_90H5** | NA | GACTATGGCATGCAC | GTTATATGGTATGAAGGAAGTAATACATACTATGCAGACTCCGTGAAGGGC | GAGATGGGGTGGTTAGATGACTAC |
| | | | SEQ ID NO:6001 | SEQ ID NO:14013 | SEQ ID NO:22025 |
| | | AA | DYGMH | VIWYEGSNTYYADSVKG | EMGWLDDY |
| | | | SEQ ID NO:6002 | SEQ ID NO:14014 | SEQ ID NO:22026 |
| **iPS:435051** | **21-225_90D9** | NA | AATTATGATATCAAC | TGGATGAACCCTAACAGTGGTAACACAGGCTATGCACAGAAGTTCCAGGGC | TCCAGTGGCTGGCACTGGTTCGACCCC |
| | | | SEQ ID NO:6003 | SEQ ID NO:14015 | SEQ ID NO:22027 |
| | | AA | NYDIN | WMNPNSGNTGYAQKFQG | SSGWHWFDP |
| | | | SEQ ID NO:6004 | SEQ ID NO:14016 | SEQ ID NO:22028 |
| **iPS:435053** | **21-225_75F9** | NA | AATTATGATATCAAC | TGGATGCACCCTAACAGTGGTAACACAGGCTATGCACAGAAGTTCCAGGGC | AGCAGTGGCTGGTACATCTTTGACTAC |
| | | | SEQ ID NO:6005 | SEQ ID NO:14017 | SEQ ID NO:22029 |

# FIGURE 49

| | | | | | |
|---|---|---|---|---|---|
| | | AA | NYDIN | WMHPNSGNTGYAQKFQG | SSGWYIFDY |
| | | | SEQ ID NO:6006 | SEQ ID NO:14018 | SEQ ID NO:22030 |
| **iPS:435055** | **21-225_90F10** | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:6007 | SEQ ID NO:14019 | SEQ ID NO:22031 |
| | | AA | GCYWS | EINHSGSTNYNPSLKS | DYGGMDV |
| | | | SEQ ID NO:6008 | SEQ ID NO:14020 | SEQ ID NO:22032 |
| **iPS:435059** | **21-225_90C11** | NA | AACTACGACATGCAC | GCTATTGGTACTGCTGGT GACACATACTATCCAGG CTCCGTGAAGGGC | GTTCTTGACTACGGTGACTC CTTGGGCTACTACTACTACG GTATGGACGTC |
| | | | SEQ ID NO:6009 | SEQ ID NO:14021 | SEQ ID NO:22033 |
| | | AA | NYDMH | AIGTAGDTYYPGSVKG | VLDYGDSLGYYYYGMDV |
| | | | SEQ ID NO:6010 | SEQ ID NO:14022 | SEQ ID NO:22034 |
| **iPS:435071** | **21-225_91F1** | NA | AATTATGATATCAAC | TGGATGCACCCTAACAG TGGTAACACAGGCTATG CACAGAAGTTCCAGGGC | AGCAGTGGCTGGTACTGGTT CGACCCC |
| | | | SEQ ID NO:6011 | SEQ ID NO:14023 | SEQ ID NO:22035 |
| | | AA | NYDIN | WMHPNSGNTGYAQKFQG | SSGWYWFDP |
| | | | SEQ ID NO:6012 | SEQ ID NO:14024 | SEQ ID NO:22036 |
| **iPS:435073** | **21-225_91B2** | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:6013 | SEQ ID NO:14025 | SEQ ID NO:22037 |
| | | AA | GCYWS | EINHSGSTNYNPSLKS | DYGGMDV |
| | | | SEQ ID NO:6014 | SEQ ID NO:14026 | SEQ ID NO:22038 |

FIGURE 49

| | | | | | |
|---|---|---|---|---|---|
| **iPS:435075** | **21-225_91B3** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:6015 | SEQ ID NO:14027 | SEQ ID NO:22039 |
| | | AA | GCYWS | EINHSGSTNYNPSLKS | DYGGMDV |
| | | | SEQ ID NO:6016 | SEQ ID NO:14028 | SEQ ID NO:22040 |
| **iPS:435077** | **21-225_91F3** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:6017 | SEQ ID NO:14029 | SEQ ID NO:22041 |
| | | AA | GCYWS | EINHSGSTNYNPSLKS | DYGGMDV |
| | | | SEQ ID NO:6018 | SEQ ID NO:14030 | SEQ ID NO:22042 |
| **iPS:435079** | **21-225_91B4** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:6019 | SEQ ID NO:14031 | SEQ ID NO:22043 |
| | | AA | GCYWS | EINHSGSTNYNPSLKS | DYGGMDV |
| | | | SEQ ID NO:6020 | SEQ ID NO:14032 | SEQ ID NO:22044 |
| **iPS:435087** | **21-225_91G8** | NA | AATTATGATATCAAC | TGGATGCACCCTAACAGTGGTAACACAGGCTATGCACAGAAGTTCCAGGGC | AGCAGTGGCTGGTACTGGTTCGACCCC |
| | | | SEQ ID NO:6021 | SEQ ID NO:14033 | SEQ ID NO:22045 |
| | | AA | NYDIN | WMHPNSGNTGYAQKFQG | SSGWYWFDP |
| | | | SEQ ID NO:6022 | SEQ ID NO:14034 | SEQ ID NO:22046 |
| **iPS:435089** | **21-225_91E9** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:6023 | SEQ ID NO:14035 | SEQ ID NO:22047 |
| | | AA | GCYWS | EINHSGSTNYNPSLKS | DYGGMDV |

FIGURE 49

| | | | | | |
|---|---|---|---|---|---|
| | | | SEQ ID NO:6024 | SEQ ID NO:14036 | SEQ ID NO:22048 |
| **iPS:435097** | **21-225_92B1** | NA | GGTTCCTACTGGAGC | GAAATCAATTATAGGGGAAGCACCAACTACAACCCGTCCCTCAAGAGT | GACTACGGCGGTTTGGACGTC |
| | | | SEQ ID NO:6025 | SEQ ID NO:14037 | SEQ ID NO:22049 |
| | | AA | GSYWS | EINYRGSTNYNPSLKS | DYGGLDV |
| | | | SEQ ID NO:6026 | SEQ ID NO:14038 | SEQ ID NO:22050 |
| **iPS:435103** | **21-225_92B2** | NA | AACTACGACATGCAC | GCTATTGGTACTGCTGGTGACACATACTATCCAGGCTCCGTGAAGGGC | GCTCTTGACTACGGTGACTCCTTGGGCTACTACTACTACGGTATGGACGTC |
| | | | SEQ ID NO:6027 | SEQ ID NO:14039 | SEQ ID NO:22051 |
| | | AA | NYDMH | AIGTAGDTYYPGSVKG | ALDYGDSLGYYYYGMDV |
| | | | SEQ ID NO:6028 | SEQ ID NO:14040 | SEQ ID NO:22052 |
| **iPS:435109** | **21-225_92H5** | NA | AGCTATGGCATGCAC | GTTATATGGTATGATGGAAGTAATAAATACTATGCAGACTCCGTGAAGGGC | GATCCCTTTATAGTGGGAGCTACTTACTTTGACTAC |
| | | | SEQ ID NO:6029 | SEQ ID NO:14041 | SEQ ID NO:22053 |
| | | AA | SYGMH | VIWYDGSNKYYADSVKG | DPFIVGATYFDY |
| | | | SEQ ID NO:6030 | SEQ ID NO:14042 | SEQ ID NO:22054 |
| **iPS:435111** | **21-225_92D6** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:6031 | SEQ ID NO:14043 | SEQ ID NO:22055 |
| | | AA | GCYWS | EINHSGSTNYNPSLKS | DYGGMDV |
| | | | SEQ ID NO:6032 | SEQ ID NO:14044 | SEQ ID NO:22056 |
| **iPS:435113** | | NA | AATTATGATATCAAC | TGGATGCACCCTAACAGTGGTAACACAGGCTATGCACAGAAGTTCCAGGGC | AGCAGTGGCTGGTACTTTTTTGACTAC |

**FIGURE 49**

| | | | | | |
|---|---|---|---|---|---|
| | **21-225_92E6** | | SEQ ID NO:6033 | SEQ ID NO:14045 | SEQ ID NO:22057 |
| | | AA | NYDIN | WMHPNSGNTGYAQKFQG | SSGWYFFDY |
| | | | SEQ ID NO:6034 | SEQ ID NO:14046 | SEQ ID NO:22058 |
| **iPS:435115** | **21-225_77C5** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:6035 | SEQ ID NO:14047 | SEQ ID NO:22059 |
| | | AA | GCYWS | EINHSGSTNYNPSLKS | DYGGMDV |
| | | | SEQ ID NO:6036 | SEQ ID NO:14048 | SEQ ID NO:22060 |
| **iPS:435167** | **21-225_92F12** | NA | AATTATGATATCAAC | TGGATGAACCCTAACAGTGGTAACACAGGCTATGCACAGAAGTTCCAGGGC | ACCAGTGGCGGGAAGTTCTTCGACTAC |
| | | | SEQ ID NO:6037 | SEQ ID NO:14049 | SEQ ID NO:22061 |
| | | AA | NYDIN | WMNPNSGNTGYAQKFQG | TSGGKFFDY |
| | | | SEQ ID NO:6038 | SEQ ID NO:14050 | SEQ ID NO:22062 |
| **iPS:435171** | **21-225_93C2** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:6039 | SEQ ID NO:14051 | SEQ ID NO:22063 |
| | | AA | GCYWS | EINHSGSTNYNPSLKS | DYGGMDV |
| | | | SEQ ID NO:6040 | SEQ ID NO:14052 | SEQ ID NO:22064 |
| **iPS:435177** | **21-225_93E4** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:6041 | SEQ ID NO:14053 | SEQ ID NO:22065 |
| | | AA | GCYWS | EINHSGSTNYNPSLKS | DYGGMDV |
| | | | SEQ ID NO:6042 | SEQ ID NO:14054 | SEQ ID NO:22066 |

FIGURE 49

| | | | | | |
|---|---|---|---|---|---|
| **iPS:435183** | **21-225_93E9** | NA | GGTTGCTACTGGAGC | GAAATCAATCATAGTGGAAGGACCAACTACAACCCGTCCCTCAAGAGT | GACTACGGTGGGCTTGACTAC |
| | | | SEQ ID NO:6043 | SEQ ID NO:14055 | SEQ ID NO:22067 |
| | | AA | GCYWS | EINHSGRTNYNPSLKS | DYGGLDY |
| | | | SEQ ID NO:6044 | SEQ ID NO:14056 | SEQ ID NO:22068 |
| **iPS:435195** | **21-225_94D3** | NA | GGTTGCTACTGGAGC | GAAATCAATTATAGTGGAAGAACCAACTACAACCCGTCCCTCAAGAGT | GACTACGGCGGTATGGACGTC |
| | | | SEQ ID NO:6045 | SEQ ID NO:14057 | SEQ ID NO:22069 |
| | | AA | GCYWS | EINYSGRTNYNPSLKS | DYGGMDV |
| | | | SEQ ID NO:6046 | SEQ ID NO:14058 | SEQ ID NO:22070 |
| **iPS:435197** | **21-225_94F3** | NA | AACGATATCATGCAC | GTTATATGGTATGATGGAAGTAATAAATACTATGCAGACTCCGTGAAGGGC | GAAAAATATAGCAGTGGCTGGTACGACTACGGTATGGACGTC |
| | | | SEQ ID NO:6047 | SEQ ID NO:14059 | SEQ ID NO:22071 |
| | | AA | NDIMH | VIWYDGSNKYYADSVKG | EKYSSGWYDYGMDV |
| | | | SEQ ID NO:6048 | SEQ ID NO:14060 | SEQ ID NO:22072 |
| **iPS:435203** | **21-225_75A7** | NA | AATTATGATATCAAC | TGGATGAACCCTAACAGTGGTAACACAGGCTATGCACAGAAGTTCCAGGAC | TCCAGTGGCTGGCACTGGTTCGACCCC |
| | | | SEQ ID NO:6049 | SEQ ID NO:14061 | SEQ ID NO:22073 |
| | | AA | NYDIN | WMNPNSGNTGYAQKFQD | SSGWHWFDP |
| | | | SEQ ID NO:6050 | SEQ ID NO:14062 | SEQ ID NO:22074 |

FIGURE 49

| | | | | | |
|---|---|---|---|---|---|
| **iPS:435209** | **21-225_75A10** | NA | AATTATGATATCAAC | TGGATGAACCCTAACAGTGGTAACACAGGCTATGCACAGAAGTTCCAGGGC | AGCAGTGGCTGGTACTACTTTGACTAC |
| | | | SEQ ID NO:6051 | SEQ ID NO:14063 | SEQ ID NO:22075 |
| | | AA | NYDIN | WMNPNSGNTGYAQKFQG | SSGWYYFDY |
| | | | SEQ ID NO:6052 | SEQ ID NO:14064 | SEQ ID NO:22076 |
| **iPS:435211** | **21-225_94E11** | NA | AATTATGATATCAAC | TGGATGAACCCTAACAGTGGTAACACAGGCTATGCACAGAAGTTCCAGGGC | TCCAGTGGCTGGAAGTGGTTCGACCCC |
| | | | SEQ ID NO:6053 | SEQ ID NO:14065 | SEQ ID NO:22077 |
| | | AA | NYDIN | WMNPNSGNTGYAQKFQG | SSGWKWFDP |
| | | | SEQ ID NO:6054 | SEQ ID NO:14066 | SEQ ID NO:22078 |
| **iPS:435215** | **21-225_94E12** | NA | AATTATGATATCAAC | TGGATGAACCCTAACAGTGGTAACACAGGCTATGCACAGAAGTTCCAGGGC | ACCAGTGGCTGGAAGTTCTTTGACTAC |
| | | | SEQ ID NO:6055 | SEQ ID NO:14067 | SEQ ID NO:22079 |
| | | AA | NYDIN | WMNPNSGNTGYAQKFQG | TSGWKFFDY |
| | | | SEQ ID NO:6056 | SEQ ID NO:14068 | SEQ ID NO:22080 |
| **iPS:435217** | **21-225_94F12** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:6057 | SEQ ID NO:14069 | SEQ ID NO:22081 |
| | | AA | GCYWS | EINHSGSTNYNPSLKS | DYGGMDV |
| | | | SEQ ID NO:6058 | SEQ ID NO:14070 | SEQ ID NO:22082 |
| **iPS:435219** | **21 225 95D2** | NA | GGTTGCTACTGGAGC | GAAATCAATCATAGTGGAAGCACCAACTACAACCCGTCCCTCAAGAGT | GACTACGGCGGTATGGACGTC |

FIGURE 49

| | | | | | |
|---|---|---|---|---|---|
| | **21-225_95D2** | | SEQ ID NO:6059 | SEQ ID NO:14071 | SEQ ID NO:22083 |
| | | AA | GCYWS | EINHSGSTNYNPSLKS | DYGGMDV |
| | | | SEQ ID NO:6060 | SEQ ID NO:14072 | SEQ ID NO:22084 |
| **iPS:435221** | **21-225_95G2** | NA | AGCTATGGCATGCAC | GTTATTTGGTATGATGGAAGTAATAAATACTATGCAGACTCCGTGAAGGGC | GATCGCAATATAGTGGGAGCTACTTACTTTGAGTCC |
| | | | SEQ ID NO:6061 | SEQ ID NO:14073 | SEQ ID NO:22085 |
| | | AA | SYGMH | VIWYDGSNKYYADSVKG | DRNIVGATYFES |
| | | | SEQ ID NO:6062 | SEQ ID NO:14074 | SEQ ID NO:22086 |
| **iPS:435227** | **21-225_95G4** | NA | AATTATGATATCAAC | TGGATGAACCCTAACAGTGGTAACACAGGCTATGCACAGAAGTTCCAGGGC | TCCAGTGGCTGGAACTGGTTCGACCCC |
| | | | SEQ ID NO:6063 | SEQ ID NO:14075 | SEQ ID NO:22087 |
| | | AA | NYDIN | WMNPNSGNTGYAQKFQG | SSGWNWFDP |
| | | | SEQ ID NO:6064 | SEQ ID NO:14076 | SEQ ID NO:22088 |
| **iPS:435235** | **21-225_95F9** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:6065 | SEQ ID NO:14077 | SEQ ID NO:22089 |
| | | AA | GCYWS | EINHSGSTNYNPSLKS | DYGGMDV |
| | | | SEQ ID NO:6066 | SEQ ID NO:14078 | SEQ ID NO:22090 |
| **iPS:435237** | **21-225_95G9** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:6067 | SEQ ID NO:14079 | SEQ ID NO:22091 |
| | | AA | GCYWS | EINHSGSTNYNPSLKS | DYGGMDV |
| | | | SEQ ID NO:6068 | SEQ ID NO:14080 | SEQ ID NO:22092 |

FIGURE 49

| | | | | | |
|---|---|---|---|---|---|
| **iPS:435239** | **21-225_95H10** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:6069 | SEQ ID NO:14081 | SEQ ID NO:22093 |
| | | AA | GCYWS | EINHSGSTNYNPSLKS | DYGGMDV |
| | | | SEQ ID NO:6070 | SEQ ID NO:14082 | SEQ ID NO:22094 |
| **iPS:435245** | **21-225_95E12** | NA | AATTATGATATCAAC | TGGATGAACCCTAACAGTGGTAACACAGGCTATGCACAGAAGTTCCAGGGC | AGCAGTGGCTGGTACTGGTTCGACCCC |
| | | | SEQ ID NO:6071 | SEQ ID NO:14083 | SEQ ID NO:22095 |
| | | AA | NYDIN | WMNPNSGNTGYAQKFQG | SSGWYWFDP |
| | | | SEQ ID NO:6072 | SEQ ID NO:14084 | SEQ ID NO:22096 |
| **iPS:435247** | **21-225_96G1** | NA | AATTATGATATCAAC | TGGATGCACCCTAACAATGGTAACACAGGCTATGCACAGAAGTTCCAGGGC | AGCAGTGGCTGGTACTGGTTCGACCCC |
| | | | SEQ ID NO:6073 | SEQ ID NO:14085 | SEQ ID NO:22097 |
| | | AA | NYDIN | WMHPNNGNTGYAQKFQG | SSGWYWFDP |
| | | | SEQ ID NO:6074 | SEQ ID NO:14086 | SEQ ID NO:22098 |
| **iPS:435249** | **21-225_96E2** | NA | AATTATGATATCAAC | TGGATGCACCCTAATAGTGGTAACACAGGCTATGCACAGAAGTTCCAGGGC | TCCAGTGGCTGGTACTGGTTCGACCCC |
| | | | SEQ ID NO:6075 | SEQ ID NO:14087 | SEQ ID NO:22099 |
| | | AA | NYDIN | WMHPNSGNTGYAQKFQG | SSGWYWFDP |
| | | | SEQ ID NO:6076 | SEQ ID NO:14088 | SEQ ID NO:22100 |
| **iPS:435251** | **21 225 96A3** | NA | AGTAGTAATTACTACTGGGGC | AGTATCTATTATAGTGGATACACCTCCTACAACCCGTCCCTCAAGAGT | CTTGACTCTAACTGGGGTCTTGACTAC |

FIGURE 49

| | | | | | |
|---|---|---|---|---|---|
| | 21-225_96A3 | | SEQ ID NO:6077 | SEQ ID NO:14089 | SEQ ID NO:22101 |
| | | AA | SSNYYWG | SIYYSGYTSYNPSLKS | LDSNWGLDY |
| | | | SEQ ID NO:6078 | SEQ ID NO:14090 | SEQ ID NO:22102 |
| iPS:435253 | | NA | AGTTATGATATCAAC | TGGATGAACCCTAACAG TGGTAACACAGGCTATG CACAGAAGTTCCAGGGC | GGCTTTTACGATACTTTGACT GGTTCCGGCTACTACTACGT TATGGACGTC |
| | 21-225_96A4 | | SEQ ID NO:6079 | SEQ ID NO:14091 | SEQ ID NO:22103 |
| | | AA | SYDIN | WMNPNSGNTGYAQKFQG | GFYDTLTGSGYYYVMDV |
| | | | SEQ ID NO:6080 | SEQ ID NO:14092 | SEQ ID NO:22104 |
| iPS:435255 | | NA | AATTATGATATCAAC | TGGATGAACCCTAACAG TGGTAACACAGGCTATG CACAGAAGTTCCAGGGC | TCCAGTGGCTGGTCCTGGTT CGACCCC |
| | 21-225_96D5 | | SEQ ID NO:6081 | SEQ ID NO:14093 | SEQ ID NO:22105 |
| | | AA | NYDIN | WMNPNSGNTGYAQKFQG | SSGWSWFDP |
| | | | SEQ ID NO:6082 | SEQ ID NO:14094 | SEQ ID NO:22106 |
| iPS:435257 | | NA | AATTATGATATCAAC | TGGATGCACCCTAACAG TGGTAACACAGGCTATG CACAGAAGTTCCAGGGC | AGTAGTGGCTGGTACAAATT TGACTAC |
| | 21-225_96H5 | | SEQ ID NO:6083 | SEQ ID NO:14095 | SEQ ID NO:22107 |
| | | AA | NYDIN | WMHPNSGNTGYAQKFQG | SSGWYKFDY |
| | | | SEQ ID NO:6084 | SEQ ID NO:14096 | SEQ ID NO:22108 |
| iPS:435259 | | NA | AGTTATGATATCAAC | TGGATGAACCCTAACAG TCGTAACACAGGCTATG CACAGAAGTTCCAGGGC | GGCGGCTACGATGTTTTGCC TGGGAATAACTACTACTACG ATATGGACGTC |
| | 21-225_96C6 | | SEQ ID NO:6085 | SEQ ID NO:14097 | SEQ ID NO:22109 |
| | | AA | SYDIN | WMNPNSRNTGYAQKFQG | GGYDVLPGNNYYYDMDV |

FIGURE 49

| | | | | | |
|---|---|---|---|---|---|
| | | | SEQ ID NO:6086 | SEQ ID NO:14098 | SEQ ID NO:22110 |
| **iPS:435267** | **21-225_96D10** | NA | AATTATGATATCAAC | TGGATGCACCCTAACAGTGGTAACACAGGCTATGCACAGAAGTTCCAGGGC | AGCAGTGGCTGGTACTTTTTTGACTAC |
| | | | SEQ ID NO:6087 | SEQ ID NO:14099 | SEQ ID NO:22111 |
| | | AA | NYDIN | WMHPNSGNTGYAQKFQG | SSGWYFFDY |
| | | | SEQ ID NO:6088 | SEQ ID NO:14100 | SEQ ID NO:22112 |
| **iPS:435273** | **21-225_97A2** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:6089 | SEQ ID NO:14101 | SEQ ID NO:22113 |
| | | AA | GCYWS | EINHSGSTNYNPSLKS | DYGGMDV |
| | | | SEQ ID NO:6090 | SEQ ID NO:14102 | SEQ ID NO:22114 |
| **iPS:435279** | **21-225_97H4** | NA | AATTATGATATCAAC | TGGATGCACCCTAACAGTGGTAACACAGGCTATGCACAGAAGTTCCAGGGC | AGCAGTGGCTGGTACTGGTTCGACCCC |
| | | | SEQ ID NO:6091 | SEQ ID NO:14103 | SEQ ID NO:22115 |
| | | AA | NYDIN | WMHPNSGNTGYAQKFQG | SSGWYWFDP |
| | | | SEQ ID NO:6092 | SEQ ID NO:14104 | SEQ ID NO:22116 |
| **iPS:435281** | **21-225_97E5** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:6093 | SEQ ID NO:14105 | SEQ ID NO:22117 |
| | | AA | GCYWS | EINHSGSTNYNPSLKS | DYGGMDV |
| | | | SEQ ID NO:6094 | SEQ ID NO:14106 | SEQ ID NO:22118 |

FIGURE 49

| | | | | | |
|---|---|---|---|---|---|
| **iPS:435291** | **21-225_146E1** | NA | AGCTATGGCATGCAC | ATTATATGGTATGATGGAAGTAATAAATACTATGCAGACTCCGTGAAGGGC | GATCGTTTAGTGGGAGCTACCGCTGATGCTTTTGATATC |
| | | | SEQ ID NO:6095 | SEQ ID NO:14107 | SEQ ID NO:22119 |
| | | AA | SYGMH | IIWYDGSNKYYADSVKG | DRLVGATADAFDI |
| | | | SEQ ID NO:6096 | SEQ ID NO:14108 | SEQ ID NO:22120 |
| **iPS:435293** | **21-225_146F1** | NA | AGAAGTAGTTACTACTGGGGC | AGTATATATTATAGTGGGAGTACCTCCTACAACCCGTCCCTCAAGAGT | CTTGATCTCCTGTGGAGTTTTGACTAC |
| | | | SEQ ID NO:6097 | SEQ ID NO:14109 | SEQ ID NO:22121 |
| | | AA | RSSYYWG | SIYYSGSTSYNPSLKS | LDLLWSFDY |
| | | | SEQ ID NO:6098 | SEQ ID NO:14110 | SEQ ID NO:22122 |
| **iPS:435295** | **21-225_146H1** | NA | AGCTATGCCATGAGC | GCTATTAGTGGTCGTGGTGGTAACACATTCTACGCAGACTCCGTGAAGGGC | CGGGTGACGGACTACGGTGGTAACGACTGGTTCGACCCC |
| | | | SEQ ID NO:6099 | SEQ ID NO:14111 | SEQ ID NO:22123 |
| | | AA | SYAMS | AISGRGGNTFYADSVKG | RVTDYGGNDWFDP |
| | | | SEQ ID NO:6100 | SEQ ID NO:14112 | SEQ ID NO:22124 |
| **iPS:435297** | **21-225_146B3** | NA | AGCTATGGCATGCAC | GTTATATGGTATGATGGAAGTTATAAATACTATGCAGACTCCGTGAAGGGC | ATGGGTATAGAAGTGGCTGTGGACTACTACTACGGTATGGACGTC |
| | | | SEQ ID NO:6101 | SEQ ID NO:14113 | SEQ ID NO:22125 |
| | | AA | SYGMH | VIWYDGSYKYYADSVKG | MGIEVAVDYYYGMDV |
| | | | SEQ ID NO:6102 | SEQ ID NO:14114 | SEQ ID NO:22126 |
| **iPS:435299** | **21-225_146D4** | NA | AATTATGATATCAAC | TGGGTGCACCCTAACAGTGGTAACACAGGCTATGCACAGAAGTTCCAGGGC | AGCAGTGGCTGGTACTACTTTGACTAC |
| | | | SEQ ID NO:6103 | SEQ ID NO:14115 | SEQ ID NO:22127 |

FIGURE 49

| | | | | | |
|---|---|---|---|---|---|
| | | AA | NYDIN | WVHPNSGNTGYAQKFQG | SSGWYYFDY |
| | | | SEQ ID NO:6104 | SEQ ID NO:14116 | SEQ ID NO:22128 |
| **iPS:435301** | **21-225_146G4** | NA | AACAGTGGTTACTACTGGAG C | TACAGCTATTACAGTGG GAGCACCTACTACAACC CGTCCCTCAAGAGT | GGGAAATATAACTGGAACCA TGCTTTTGATATC |
| | | | SEQ ID NO:6105 | SEQ ID NO:14117 | SEQ ID NO:22129 |
| | | AA | NSGYYWS | YSYYSGSTYYNPSLKS | GKYNWNHAFDI |
| | | | SEQ ID NO:6106 | SEQ ID NO:14118 | SEQ ID NO:22130 |
| **iPS:435303** | **21-225_146A6** | NA | AGCTATGCCATGAAC | GCTATTAGTGGTAGTGGT GGTAACACATTCTACGC AGACTCCGTGAAGGGC | AAGGATAATGATTACGTTTG GGGGAGTCCTTACTTTGACT AC |
| | | | SEQ ID NO:6107 | SEQ ID NO:14119 | SEQ ID NO:22131 |
| | | AA | SYAMN | AISGSGGNTFYADSVKG | KDNDYVWGSPYFDY |
| | | | SEQ ID NO:6108 | SEQ ID NO:14120 | SEQ ID NO:22132 |
| **iPS:435305** | **21-225_146C9** | NA | AATTATGATATCAAC | TGGATGCACCCTAACAG TGGTAACACAGGCTATG CACAGAAGTTCCAGGGC | AGCAGTGGCTGGTACTCCTT TGACTAC |
| | | | SEQ ID NO:6109 | SEQ ID NO:14121 | SEQ ID NO:22133 |
| | | AA | NYDIN | WMHPNSGNTGYAQKFQG | SSGWYSFDY |
| | | | SEQ ID NO:6110 | SEQ ID NO:14122 | SEQ ID NO:22134 |
| **iPS:435307** | **21-225_146E9** | NA | AGCTATGCCATGAGC | GCTATTAGTGGTCGTGGT GGTAACACATTCTACGC AGACTCCGTGAAGGGC | CGGGTGACGGACTACGGTGG TAACGACTGGTTCGACCCC |
| | | | SEQ ID NO:6111 | SEQ ID NO:14123 | SEQ ID NO:22135 |
| | | AA | SYAMS | AISGRGGNTFYADSVKG | RVTDYGGNDWFDP |
| | | | SEQ ID NO:6112 | SEQ ID NO:14124 | SEQ ID NO:22136 |

**FIGURE 49**

| | | | | | |
|---|---|---|---|---|---|
| **iPS:435309** | **21-225_146F9** | NA | AATTATGATATCAAC | TGGATGCACCCTAACAGTGGTAACACAGGCTATGCACAGAAGTTCCAGGGC | AGCAGTGGCTGGTACTTTTTTGACTAC |
| | | | SEQ ID NO:6113 | SEQ ID NO:14125 | SEQ ID NO:22137 |
| | | AA | NYDIN | WMHPNSGNTGYAQKFQG | SSGWYFFDY |
| | | | SEQ ID NO:6114 | SEQ ID NO:14126 | SEQ ID NO:22138 |
| **iPS:435311** | **21-225_146H9** | NA | AGCTATGGCATGCAC | GTGATATGGTTTGATGAAAGTAATAAACACTATGGAGACTCCGTGAAGGGC | GAATTGGGATTTCTCTCTGACTAT |
| | | | SEQ ID NO:6115 | SEQ ID NO:14127 | SEQ ID NO:22139 |
| | | AA | SYGMH | VIWFDESNKHYGDSVKG | ELGFLSDY |
| | | | SEQ ID NO:6116 | SEQ ID NO:14128 | SEQ ID NO:22140 |
| **iPS:435313** | **21-225_146G11** | NA | AGCTATAGCATGAAC | TCCATTAGTGGTAGTGGTAGCTACACATACTACGCAGACTCAGTGAAGGGC | GGTAGCAGCTCGTCCGGGTTTGACTAC |
| | | | SEQ ID NO:6117 | SEQ ID NO:14129 | SEQ ID NO:22141 |
| | | AA | SYSMN | SISGSGSYTYYADSVKG | GSSSSGFDY |
| | | | SEQ ID NO:6118 | SEQ ID NO:14130 | SEQ ID NO:22142 |
| **iPS:435315** | **21-225_147B2** | NA | AGCTATGGCATGCAC | GTTATATGGTATGATGGAAGTAATAAATACTATGCAGACTCCGTGAAGGGC | AGGTATAGCAGCAGCTGGTCGGGGGGTATGGACGTC |
| | | | SEQ ID NO:6119 | SEQ ID NO:14131 | SEQ ID NO:22143 |
| | | AA | SYGMH | VIWYDGSNKYYADSVKG | RYSSSWSGGMDV |
| | | | SEQ ID NO:6120 | SEQ ID NO:14132 | SEQ ID NO:22144 |

FIGURE 49

| | | | | | |
|---|---|---|---|---|---|
| **iPS:435317** | **21-225_147D2** | NA | AGTGGTGATTACTACTGGAAC | TTCATCTATTACACTGGGAGCACCTACTACAACCCGTCCCTCAAGAGT | GGGGGAGCTTACTACTCCTACTACGGTATGGACGTC |
| | | | SEQ ID NO:6121 | SEQ ID NO:14133 | SEQ ID NO:22145 |
| | | AA | SGDYYWN | FIYYTGSTYYNPSLKS | GGAYYSYYGMDV |
| | | | SEQ ID NO:6122 | SEQ ID NO:14134 | SEQ ID NO:22146 |
| **iPS:435319** | **21-225_147E3** | NA | AATAGTGGTTACTACTATAGC | TACATCTATTACAGTGGGGGCACCTACTACAACCCGTCCCTCAAGAGT | GGGGGATATAACTGGAACCATGCTTTTGATTTC |
| | | | SEQ ID NO:6123 | SEQ ID NO:14135 | SEQ ID NO:22147 |
| | | AA | NSGYYYS | YIYYSGGTYYNPSLKS | GGYNWNHAFDF |
| | | | SEQ ID NO:6124 | SEQ ID NO:14136 | SEQ ID NO:22148 |
| **iPS:435321** | **21-225_147E4** | NA | AATTATGATATCAAC | TGGATGAACCCTAACAGTGGTAACACAGGCTATGCACAGAAGTTCCAGGGC | AGCAGTGGCTGGTACTTTTTTGACTAC |
| | | | SEQ ID NO:6125 | SEQ ID NO:14137 | SEQ ID NO:22149 |
| | | AA | NYDIN | WMNPNSGNTGYAQKFQG | SSGWYFFDY |
| | | | SEQ ID NO:6126 | SEQ ID NO:14138 | SEQ ID NO:22150 |
| **iPS:435323** | **21-225_147D5** | NA | AATTATGATATCAAC | TGGGTGCACCCTAACAGTGGTAACACAGGCTATGCACAGAAGTTCCAGGGC | AGCAGTGGCTGGTACTACTTTGACTAC |
| | | | SEQ ID NO:6127 | SEQ ID NO:14139 | SEQ ID NO:22151 |
| | | AA | NYDIN | WVHPNSGNTGYAQKFQG | SSGWYYFDY |
| | | | SEQ ID NO:6128 | SEQ ID NO:14140 | SEQ ID NO:22152 |
| **iPS:435325** | **21-225_147H5** | NA | GACTATGGCATGCAC | GTTATATGGTATGATGGAAGTAATAAGTACTATGCAGACTCCGTGAAGGGC | GAGAGGTATAGCAGTGGCTGGTACGACTACGGTTTGGACGTC |
| | | | SEQ ID NO:6129 | SEQ ID NO:14141 | SEQ ID NO:22153 |

**FIGURE 49**

| | | | | | |
|---|---|---|---|---|---|
| | | AA | DYGMH | VIWYDGSNKYYADSVKG | ERYSSGWYDYGLDV |
| | | | SEQ ID NO:6130 | SEQ ID NO:14142 | SEQ ID NO:22154 |
| **iPS:435327** | **21-225_147G6** | NA | AATTATGATATCAAC | TGGATGCACCCCAACAGTGGTAACACAGGCTATGCACAGAAGTTCCAGGGC | AGCAGTGGCTGGTACTGGTTCGACCCC |
| | | | SEQ ID NO:6131 | SEQ ID NO:14143 | SEQ ID NO:22155 |
| | | AA | NYDIN | WMHPNSGNTGYAQKFQG | SSGWYWFDP |
| | | | SEQ ID NO:6132 | SEQ ID NO:14144 | SEQ ID NO:22156 |
| **iPS:435329** | **21-225_147A8** | NA | AGCTATGGCATGCAC | GTTATATGGTATGATGGAAGTAATAAATACTATGCAGACTCCGTGAAGGGC | AGGTATAGCAGCAGCTGGACGGGGGGTATGGACGTC |
| | | | SEQ ID NO:6133 | SEQ ID NO:14145 | SEQ ID NO:22157 |
| | | AA | SYGMH | VIWYDGSNKYYADSVKG | RYSSSWTGGMDV |
| | | | SEQ ID NO:6134 | SEQ ID NO:14146 | SEQ ID NO:22158 |
| **iPS:435331** | **21-225_147G8** | NA | GCTTACTACTGGAGC | GAAATCAATCATAGTGGAAGTACCAACTACAAACCGTCCCTCAAGAGT | GACTACGGTGTTTTTGACTAC |
| | | | SEQ ID NO:6135 | SEQ ID NO:14147 | SEQ ID NO:22159 |
| | | AA | AYYWS | EINHSGSTNYKPSLKS | DYGVFDY |
| | | | SEQ ID NO:6136 | SEQ ID NO:14148 | SEQ ID NO:22160 |
| **iPS:435333** | **21-225_147E9** | NA | AGCTATAGCATGAAC | TCCATTAGTGGTAGAAACACTACCATATACTATGCAGACTCTGTGAAGGGC | GATCGGGGCAGTTGC |
| | | | SEQ ID NO:6137 | SEQ ID NO:14149 | SEQ ID NO:22161 |
| | | AA | SYSMN | SISGRNTTIYYADSVKG | DRGSC |
| | | | SEQ ID NO:6138 | SEQ ID NO:14150 | SEQ ID NO:22162 |

**FIGURE 49**

| | | | | | |
|---|---|---|---|---|---|
| **iPS:435335** | **21-225_147D10** | NA | AGCTATGCCATGAGC | GCTATTAGTGGTCGTGGTGGTAACACATTCTACGCAGACTCCGTGAAGGGC | AAGGATTATGATTACGTTTGGGGGAGTCCTTACTTTGACTAC |
| | | | SEQ ID NO:6139 | SEQ ID NO:14151 | SEQ ID NO:22163 |
| | | AA | SYAMS | AISGRGGNTFYADSVKG | KDYDYVWGSPYFDY |
| | | | SEQ ID NO:6140 | SEQ ID NO:14152 | SEQ ID NO:22164 |
| **iPS:435339** | **21-225_147D12** | NA | AGCTATGCCATGAGC | GCTATTAGTGGTCGTGGTGGTAACACATTCTACGCAGACTCCGTGAAGGGC | AAGGATTATGATTACGTTTGGGGGAGTCCTTACTTTGACTAC |
| | | | SEQ ID NO:6141 | SEQ ID NO:14153 | SEQ ID NO:22165 |
| | | AA | SYAMS | AISGRGGNTFYADSVKG | KDYDYVWGSPYFDY |
| | | | SEQ ID NO:6142 | SEQ ID NO:14154 | SEQ ID NO:22166 |
| **iPS:435341** | **21-225_148B2** | NA | AGCTATGGCATGCAC | ATTATCTGGTATGATGGAAGTTATAAATACTATGCAGACTCCGTGAAGGGC | GATCATTTCGATTTTTGGAGTGGTCACTTTGACTAC |
| | | | SEQ ID NO:6143 | SEQ ID NO:14155 | SEQ ID NO:22167 |
| | | AA | SYGMH | IIWYDGSYKYYADSVKG | DHFDFWSGHFDY |
| | | | SEQ ID NO:6144 | SEQ ID NO:14156 | SEQ ID NO:22168 |
| **iPS:435343** | **21-225_148E2** | NA | AGCTATGCCATGAGC | GCTATTAGTGGTCGTGGTGGTAACACATTCTACGCAGACTCCGTGAAGGGC | AAGGATTATGATTACGTTTGGGGGAGTCCTTACTTTGACTAC |
| | | | SEQ ID NO:6145 | SEQ ID NO:14157 | SEQ ID NO:22169 |
| | | AA | SYAMS | AISGRGGNTFYADSVKG | KDYDYVWGSPYFDY |
| | | | SEQ ID NO:6146 | SEQ ID NO:14158 | SEQ ID NO:22170 |
| **iPS:435345** | **21-225_148G3** | NA | AATTATGATATCAAC | TGGATGCACCCTAACAGTGGTAACACAGGCTATGCACAGAACTTCCAGGGC | AGCAGTGGCTGGTACTTTTTTGACTAC |
| | | | SEQ ID NO:6147 | SEQ ID NO:14159 | SEQ ID NO:22171 |
| | | AA | NYDIN | WMHPNSGNTGYAQNFQG | SSGWYFFDY |

FIGURE 49

| | | | | | |
|---|---|---|---|---|---|
| | | | SEQ ID NO:6148 | SEQ ID NO:14160 | SEQ ID NO:22172 |
| **iPS:435347** | **21-225_148C4** | NA | AGCTATGCCATGAAC | GCTATTAGTGGTAGTGGTGGTAACACATTCTACGCAGACTCCGTGAAGGGC | CGGGTGACGGACTACGGTGGTAACGACTGGTTCGACCCC |
| | | | SEQ ID NO:6149 | SEQ ID NO:14161 | SEQ ID NO:22173 |
| | | AA | SYAMN | AISGSGGNTFYADSVKG | RVTDYGGNDWFDP |
| | | | SEQ ID NO:6150 | SEQ ID NO:14162 | SEQ ID NO:22174 |
| **iPS:435349** | **21-225_148F5** | NA | AGCTATGGCATGCAC | GTTATATGGTATGATGGAAGTAATAAATACTATGCAGACTCCGTGAAGGGC | AGGTATAGCAGCAGCTGGTCGGGGGGTATGGACGTC |
| | | | SEQ ID NO:6151 | SEQ ID NO:14163 | SEQ ID NO:22175 |
| | | AA | SYGMH | VIWYDGSNKYYADSVKG | RYSSSWSGGMDV |
| | | | SEQ ID NO:6152 | SEQ ID NO:14164 | SEQ ID NO:22176 |
| **iPS:435351** | **21-225_148B6** | NA | GGCTACTATATGCAC | TGGATCCACCCTAACAATGGTGGCACAAACTATGCACAGACGTTTCAGGGC | GATCCTGTAGTAGTACCAGCTGCCCCCTTTGACTAC |
| | | | SEQ ID NO:6153 | SEQ ID NO:14165 | SEQ ID NO:22177 |
| | | AA | GYYMH | WIHPNNGGTNYAQTFQG | DPVVVPAAPFDY |
| | | | SEQ ID NO:6154 | SEQ ID NO:14166 | SEQ ID NO:22178 |
| **iPS:435353** | **21-225_148F8** | NA | AATTATGATATCAAC | TGGATGAACCCTAACAGTGGTAACACAGGCTATGCACAGAAGTTCCAGGGC | AGCAGTGGCTGGTACTACTTTGACTAC |
| | | | SEQ ID NO:6155 | SEQ ID NO:14167 | SEQ ID NO:22179 |
| | | AA | NYDIN | WMNPNSGNTGYAQKFQG | SSGWYYFDY |
| | | | SEQ ID NO:6156 | SEQ ID NO:14168 | SEQ ID NO:22180 |

## FIGURE 49

| | | | | | |
|---|---|---|---|---|---|
| **iPS:435355** | **21-225_148H9** | NA | AGCTATGCCATGAGC | GCTATTAGTGGTCGTGGTGGTAACACATTCTACGCAGACTCCGTGAAGGGC | CGGGTGACGGACTACGGTGGTAACGACTGGTTCGACCCC |
| | | | SEQ ID NO:6157 | SEQ ID NO:14169 | SEQ ID NO:22181 |
| | | AA | SYAMS | AISGRGGNTFYADSVKG | RVTDYGGNDWFDP |
| | | | SEQ ID NO:6158 | SEQ ID NO:14170 | SEQ ID NO:22182 |
| **iPS:435357** | **21-225_148G10** | NA | AGCTATGGCATGCAC | ATTATATGGTATGATGGAAGTAATAAATACTATGCAGACTCCGTGAAGGGC | GATCGTTACGATTTTTGGAGTGGTCACTTTGACTAC |
| | | | SEQ ID NO:6159 | SEQ ID NO:14171 | SEQ ID NO:22183 |
| | | AA | SYGMH | IIWYDGSNKYYADSVKG | DRYDFWSGHFDY |
| | | | SEQ ID NO:6160 | SEQ ID NO:14172 | SEQ ID NO:22184 |
| **iPS:435359** | **21-225_148H10** | NA | AGCTATGGCATGCAC | GTTATATGGTATGATGGAAGTAATAAATACTATGGAGACTCCGTGAAGGGC | AGGTATAGCAGCAGCTGGTCGGGGGGTATGGACGTC |
| | | | SEQ ID NO:6161 | SEQ ID NO:14173 | SEQ ID NO:22185 |
| | | AA | SYGMH | VIWYDGSNKYYGDSVKG | RYSSSWSGGMDV |
| | | | SEQ ID NO:6162 | SEQ ID NO:14174 | SEQ ID NO:22186 |
| **iPS:435361** | **21-225_148E11** | NA | AGAAGTAGTTACTACTGGGGC | AGTATCTATTATAGTGGGAGCACCTCCTACAACCCGTCCCTCAAGAGT | CTTGATCCCCAGTGGAGTTTTGACTAC |
| | | | SEQ ID NO:6163 | SEQ ID NO:14175 | SEQ ID NO:22187 |
| | | AA | RSSYYWG | SIYYSGSTSYNPSLKS | LDPQWSFDY |
| | | | SEQ ID NO:6164 | SEQ ID NO:14176 | SEQ ID NO:22188 |
| **iPS:435363** | **21 225 148F12** | NA | AATGGTGGTTACTACTGGAAC | TACATCTATTACAGTGGGAGCACCTACTACAACCCGTCCCTCAAGAGT | TACAGTACCTACGACTACTACTACGGTATGGACGTC |

FIGURE 49

| | | | | | |
|---|---|---|---|---|---|
| | **21-225_148F12** | | SEQ ID NO:6165 | SEQ ID NO:14177 | SEQ ID NO:22189 |
| | | AA | NGGYYWN | YIYYSGSTYYNPSLKS | YSTYDYYYGMDV |
| | | | SEQ ID NO:6166 | SEQ ID NO:14178 | SEQ ID NO:22190 |
| **iPS:435365** | **21-225_149F1** | NA | AGCTATGGCATGCAC | ATTATCTGGTATGATGGAAGTTATAAATACTATGCAGACTCCGTGAAGGGC | GATCATTTCGATTTTTGGAGTGGTCACTTTGACTAC |
| | | | SEQ ID NO:6167 | SEQ ID NO:14179 | SEQ ID NO:22191 |
| | | AA | SYGMH | IIWYDGSYKYYADSVKG | DHFDFWSGHFDY |
| | | | SEQ ID NO:6168 | SEQ ID NO:14180 | SEQ ID NO:22192 |
| **iPS:435367** | **21-225_149G1** | NA | GACTATGGCATGCAC | GTTATATGGTATGAAGGAAGTAATAAATACTATGCAGACTCCGTGAAGGGC | GAAATAGGATTCAGTGAGGACTAC |
| | | | SEQ ID NO:6169 | SEQ ID NO:14181 | SEQ ID NO:22193 |
| | | AA | DYGMH | VIWYEGSNKYYADSVKG | EIGFSEDY |
| | | | SEQ ID NO:6170 | SEQ ID NO:14182 | SEQ ID NO:22194 |
| **iPS:435369** | **21-225_149A2** | NA | AATTATGATATCAAC | TGGGTGCACCCTAACAGTGGTAACACAGGCTATGCACAGAAGTTCCAGGGC | AGCAGTGGCTGGTACTACTTTGACTAC |
| | | | SEQ ID NO:6171 | SEQ ID NO:14183 | SEQ ID NO:22195 |
| | | AA | NYDIN | WVHPNSGNTGYAQKFQG | SSGWYYFDY |
| | | | SEQ ID NO:6172 | SEQ ID NO:14184 | SEQ ID NO:22196 |
| **iPS:435371** | **21-225_149A3** | NA | AACTATGCCATGACC | GCTATTAGTGGTCGTGGTGGTAACACATTCTACGCAGACTCCGTGAAGGGC | CGGGTGACGGACTACGGTGGTAACGACTGGTTCGACCCC |
| | | | SEQ ID NO:6173 | SEQ ID NO:14185 | SEQ ID NO:22197 |
| | | AA | NYAMT | AISGRGGNTFYADSVKG | RVTDYGGNDWFDP |
| | | | SEQ ID NO:6174 | SEQ ID NO:14186 | SEQ ID NO:22198 |

FIGURE 49

| | | | | | |
|---|---|---|---|---|---|
| **iPS:435373** | **21-225_149E3** | NA | AATTATGATATCAAC | TGGATGCACCCTAACAGTGGTAACACAGGCTATGCACAGAAGTTCCAGGGC | AGCAGTGGCTGGTACTGGTTTGACTAC |
| | | | SEQ ID NO:6175 | SEQ ID NO:14187 | SEQ ID NO:22199 |
| | | AA | NYDIN | WMHPNSGNTGYAQKFQG | SSGWYWFDY |
| | | | SEQ ID NO:6176 | SEQ ID NO:14188 | SEQ ID NO:22200 |
| **iPS:435375** | **21-225_149H4** | NA | AATTATGATATCAAC | TGGATGCACCCTAACAGTGGTAACACAGACTATGCACAGAAGTTCCAGGGC | AGCAGTGGCTGGTACTACTTTGACTAC |
| | | | SEQ ID NO:6177 | SEQ ID NO:14189 | SEQ ID NO:22201 |
| | | AA | NYDIN | WMHPNSGNTDYAQKFQG | SSGWYYFDY |
| | | | SEQ ID NO:6178 | SEQ ID NO:14190 | SEQ ID NO:22202 |
| **iPS:435377** | **21-225_149G5** | NA | AATGGTGGTTACTACTGGAAC | TACATCTATTACAGTGGGAGCACCTACTACAACCCGTCCCTCAAGAGT | TACAGTACCTACGACTACTACTACGGTATGGACGTC |
| | | | SEQ ID NO:6179 | SEQ ID NO:14191 | SEQ ID NO:22203 |
| | | AA | NGGYYWN | YIYYSGSTYYNPSLKS | YSTYDYYYGMDV |
| | | | SEQ ID NO:6180 | SEQ ID NO:14192 | SEQ ID NO:22204 |
| **iPS:435379** | **21-225_149B6** | NA | AGCTATGCCATGAGC | GTTATTAGTGGTAGTGGTGGTAGCACATTCTACGCAGACTCCGTGAAGGGC | CGAACTCCGGAAGATGTTTTTGATATC |
| | | | SEQ ID NO:6181 | SEQ ID NO:14193 | SEQ ID NO:22205 |
| | | AA | SYAMS | VISGSGGSTFYADSVKG | RTPEDVFDI |
| | | | SEQ ID NO:6182 | SEQ ID NO:14194 | SEQ ID NO:22206 |
| **iPS:435381** | **21-225_149C6** | NA | AGCTATGCCATGAGC | GCTATTAGTGGTAGTGGTGGTAACACATTCTACGCAGACTCCGTGAAGGGC | AAGGATTATGATTACGTTTGGGGGAGTCCTTACTTTGACTAC |
| | | | SEQ ID NO:6183 | SEQ ID NO:14195 | SEQ ID NO:22207 |

FIGURE 49

| | | | | | |
|---|---|---|---|---|---|
| | | AA | SYAMS | AISGSGGNTFYADSVKG | KDYDYVWGSPYFDY |
| | | | SEQ ID NO:6184 | SEQ ID NO:14196 | SEQ ID NO:22208 |
| **iPS:435383** | **21-225_149D7** | NA | AACAGTGGTTACTACTGGAGC | TACAGCTATTACAGTGGGAGCACCTACTACAACCCGTCCCTCAAGAGT | GGGGGATATAACTGGAACCATGCTTTTGATATC |
| | | | SEQ ID NO:6185 | SEQ ID NO:14197 | SEQ ID NO:22209 |
| | | AA | NSGYYWS | YSYYSGSTYYNPSLKS | GGYNWNHAFDI |
| | | | SEQ ID NO:6186 | SEQ ID NO:14198 | SEQ ID NO:22210 |
| **iPS:435391** | **21-225_149F8** | NA | AGCTATGCCATGAGC | GCTATTAGTGGTCGTGGTGGTAACACATTCTACGCAGACTCCGTGAAGGGC | AAGGATTATGATTACGTTTGGGGGAGTCCTTACTTTGACTAC |
| | | | SEQ ID NO:6187 | SEQ ID NO:14199 | SEQ ID NO:22211 |
| | | AA | SYAMS | AISGRGGNTFYADSVKG | KDYDYVWGSPYFDY |
| | | | SEQ ID NO:6188 | SEQ ID NO:14200 | SEQ ID NO:22212 |
| **iPS:435393** | **21-225_149D10** | NA | GACTATGGCATGCAC | GTTATATGGTATGATGGAAGTAATAAGTACTATGCAGACTCCGTGAAGGGC | GAGAGGTATAGCAGTGGCTGGTACGACTACGGTATGGACGTC |
| | | | SEQ ID NO:6189 | SEQ ID NO:14201 | SEQ ID NO:22213 |
| | | AA | DYGMH | VIWYDGSNKYYADSVKG | ERYSSGWYDYGMDV |
| | | | SEQ ID NO:6190 | SEQ ID NO:14202 | SEQ ID NO:22214 |
| **iPS:435395** | **21-225_149D11** | NA | AGCTATGCCATGAGC | GCTATTAGTGGTCGTGGTGGTAACACATTCTACGCAGACTCCGTGAAGGGC | AAGGATTATGATTACGTTTGGGGGAGTCCTTACTTTGACTAC |
| | | | SEQ ID NO:6191 | SEQ ID NO:14203 | SEQ ID NO:22215 |
| | | AA | SYAMS | AISGRGGNTFYADSVKG | KDYDYVWGSPYFDY |
| | | | SEQ ID NO:6192 | SEQ ID NO:14204 | SEQ ID NO:22216 |

FIGURE 49

| | | | | | |
|---|---|---|---|---|---|
| **iPS:435397** | **21-225_149F12** | NA | GACTATGGCATGCAC | GTTATATGGTATGAAGAAAATAATAAATACTATGCAGACTCCGTGAAGGGC | GAAATAGGGTTCAGTGAGGACTAC |
| | | | SEQ ID NO:6193 | SEQ ID NO:14205 | SEQ ID NO:22217 |
| | | AA | DYGMH | VIWYEENNKYYADSVKG | EIGFSEDY |
| | | | SEQ ID NO:6194 | SEQ ID NO:14206 | SEQ ID NO:22218 |
| **iPS:435399** | **21-225_150D2** | NA | AATTATGATATCAAC | TGGATGCACCCTAACAGTGGTAACACAGGCTATGCACAGAAGTTCCAGGGC | AGCAGTGGCTGGTACTACTTTGACTAC |
| | | | SEQ ID NO:6195 | SEQ ID NO:14207 | SEQ ID NO:22219 |
| | | AA | NYDIN | WMHPNSGNTGYAQKFQG | SSGWYYFDY |
| | | | SEQ ID NO:6196 | SEQ ID NO:14208 | SEQ ID NO:22220 |
| **iPS:435401** | **21-225_150E2** | NA | AGCTATGTCATGCAC | GTTATATGGTATGATGGAAGTAATAAATACTATGCAGACTCCGTGAAGGGC | GAGGAGTATAGCAGCAGCTGGTACGGGTACGGTATGGACGTC |
| | | | SEQ ID NO:6197 | SEQ ID NO:14209 | SEQ ID NO:22221 |
| | | AA | SYVMH | VIWYDGSNKYYADSVKG | EEYSSSWYGYGMDV |
| | | | SEQ ID NO:6198 | SEQ ID NO:14210 | SEQ ID NO:22222 |
| **iPS:435403** | **21-225_150C5** | NA | AGCTATGCCATGAGC | GCTATTAGTGGTAGTGGTGGTAACACATTCTACGCAGACTCCGTGAAGGGC | AAGGATTATGATTACGTTTGGGGGAGTCCTTACTTTGACTAC |
| | | | SEQ ID NO:6199 | SEQ ID NO:14211 | SEQ ID NO:22223 |
| | | AA | SYAMS | AISGSGGNTFYADSVKG | KDYDYVWGSPYFDY |
| | | | SEQ ID NO:6200 | SEQ ID NO:14212 | SEQ ID NO:22224 |

FIGURE 49

| | | | | | |
|---|---|---|---|---|---|
| **iPS:435405** | **21-225_150B7** | NA | AATTATGATATCAAC | TGGATGCACCCTAACAGTGGTAACACAGGCTATGCACAGAAGTTCCAGGGC | AGCAGTGGCTGGTACTTTTTTGACTAC |
| | | | SEQ ID NO:6201 | SEQ ID NO:14213 | SEQ ID NO:22225 |
| | | AA | NYDIN | WMHPNSGNTGYAQKFQG | SSGWYFFDY |
| | | | SEQ ID NO:6202 | SEQ ID NO:14214 | SEQ ID NO:22226 |
| **iPS:435407** | **21-225_150E7** | NA | GACTATGGCATGCAC | GTTATATGGTATGAAGAAAATAATAAATACTATGCAGACTCCGTGAAGGGC | GAAATAGGGTTCAGTGAGGACTAC |
| | | | SEQ ID NO:6203 | SEQ ID NO:14215 | SEQ ID NO:22227 |
| | | AA | DYGMH | VIWYEENNKYYADSVKG | EIGFSEDY |
| | | | SEQ ID NO:6204 | SEQ ID NO:14216 | SEQ ID NO:22228 |
| **iPS:435409** | **21-225_150G8** | NA | ACCTATAGCATGACT | TACATTAGTAGGAGTAGTAGTACCATATACTACGCAGACTCTGTGAAGGGC | TCGGCATTTAGCCCTTTTGATTAC |
| | | | SEQ ID NO:6205 | SEQ ID NO:14217 | SEQ ID NO:22229 |
| | | AA | TYSMT | YISRSSSTIYYADSVKG | SAFSPFDY |
| | | | SEQ ID NO:6206 | SEQ ID NO:14218 | SEQ ID NO:22230 |
| **iPS:435413** | **21-225_150B11** | NA | AGCTATGGCATGCAC | ATTATATGGTATGATGGAAGTAATAAATACTATGCAGACTCCGTGAAGGGC | GATCGTTACGATTTTTGGAGTGGTCACTTTGACTAC |
| | | | SEQ ID NO:6207 | SEQ ID NO:14219 | SEQ ID NO:22231 |
| | | AA | SYGMH | IIWYDGSNKYYADSVKG | DRYDFWSGHFDY |
| | | | SEQ ID NO:6208 | SEQ ID NO:14220 | SEQ ID NO:22232 |

FIGURE 49

| | | | | | |
|---|---|---|---|---|---|
| **iPS:435415** | **21-225_150C11** | NA | AGCTATGCCATGAAC | GCTATTAGTGGTAGTGGTGGTAACACATTCTACGCAGACTCCGTGAAGGGC | CGGGTGACGGACTACGGTGGTAACGACTGGTTCGACCCC |
| | | | SEQ ID NO:6209 | SEQ ID NO:14221 | SEQ ID NO:22233 |
| | | AA | SYAMN | AISGSGGNTFYADSVKG | RVTDYGGNDWFDP |
| | | | SEQ ID NO:6210 | SEQ ID NO:14222 | SEQ ID NO:22234 |
| **iPS:435417** | **21-225_150D11** | NA | AGCTATGGCATGCAC | GTTATATTCTATGATGGAAGTAATAAACACTATGCAGACTCCGTGAAGGGC | AGGTTTAGCAGCAGCTGGTCGGGGGGTATGGACGTC |
| | | | SEQ ID NO:6211 | SEQ ID NO:14223 | SEQ ID NO:22235 |
| | | AA | SYGMH | VIFYDGSNKHYADSVKG | RFSSSWSGGMDV |
| | | | SEQ ID NO:6212 | SEQ ID NO:14224 | SEQ ID NO:22236 |
| **iPS:435419** | **21-225_150C12** | NA | AGCTATGCCATGAGC | GCTATTAGTGGTCGTGGTGGTAACACATTCTACGCAGACTCCGTGAAGGGC | CGGGTGACGGACTACGGTGGTAACGACTGGTTCGACCCC |
| | | | SEQ ID NO:6213 | SEQ ID NO:14225 | SEQ ID NO:22237 |
| | | AA | SYAMS | AISGRGGNTFYADSVKG | RVTDYGGNDWFDP |
| | | | SEQ ID NO:6214 | SEQ ID NO:14226 | SEQ ID NO:22238 |
| **iPS:435421** | **21-225_151F1** | NA | AGCTTTAGCATGAAC | TCCATTAGTAGTAGTAGTTATTACATATACTACGCAGACTCAGTGAAGGGC | GATACACCACTGGTTTAC |
| | | | SEQ ID NO:6215 | SEQ ID NO:14227 | SEQ ID NO:22239 |
| | | AA | SFSMN | SISSSSYYIYYADSVKG | DTPLVY |
| | | | SEQ ID NO:6216 | SEQ ID NO:14228 | SEQ ID NO:22240 |
| **iPS:435423** | **21-225_151G5** | NA | AGCTATGGCATGCAC | ATTATATGGTATGATGGAAGTAATAAATACTATGCAGACTCCGTGAAGGGC | GATCGATACGATTTTTGGAGTGGTCACTTTGACTAC |
| | | | SEQ ID NO:6217 | SEQ ID NO:14229 | SEQ ID NO:22241 |

# FIGURE 49

| | | | | | |
|---|---|---|---|---|---|
| | | AA | SYGMH | IIWYDGSNKYYADSVKG | DRYDFWSGHFDY |
| | | | SEQ ID NO:6218 | SEQ ID NO:14230 | SEQ ID NO:22242 |
| **iPS:435425** | **21-225_151B12** | NA | AGCTATGCCATGAAC | GCTATTAGTGGTAGTGGTAAAAACACATTCTACGCAGACTCCGTGAAGGGC | CGGGTGACGGACTACGGTGGTAACGACTGGTTCGACCCC |
| | | | SEQ ID NO:6219 | SEQ ID NO:14231 | SEQ ID NO:22243 |
| | | AA | SYAMN | AISGSGKNTFYADSVKG | RVTDYGGNDWFDP |
| | | | SEQ ID NO:6220 | SEQ ID NO:14232 | SEQ ID NO:22244 |
| **iPS:435427** | **21-225_151C9** | NA | AGCTATGGCATGCAC | GTTATATCATATGATGGAAGTAATAAATACTATGCAGACTCCGTGAAGGGC | GGGGTATTACTATGGTTCGGGGAGCTAGAAGATGACTGGTTCGACCCC |
| | | | SEQ ID NO:6221 | SEQ ID NO:14233 | SEQ ID NO:22245 |
| | | AA | SYGMH | VISYDGSNKYYADSVKG | GVLLWFGELEDDWFDP |
| | | | SEQ ID NO:6222 | SEQ ID NO:14234 | SEQ ID NO:22246 |
| **iPS:435429** | **21-225_151A10** | NA | AACTATGGCATGCAC | ATTATATGGTATGATGGAAGTAATAAATACTATGCAGACTCCGTGAAGGGC | GATCATTACGATTTTTGGAGTGGTCACTTTGACTAC |
| | | | SEQ ID NO:6223 | SEQ ID NO:14235 | SEQ ID NO:22247 |
| | | AA | NYGMH | IIWYDGSNKYYADSVKG | DHYDFWSGHFDY |
| | | | SEQ ID NO:6224 | SEQ ID NO:14236 | SEQ ID NO:22248 |
| **iPS:435431** | **21-225_152D2** | NA | AGCTATGCCATGAGC | GCTATTAGTGGTCGTGGTGGTAACACATTCTACGCAGACTCCGTGAAGGGC | CGGGTGACGGACTACGGTGGTAACGACTGGTTCGACCCC |
| | | | SEQ ID NO:6225 | SEQ ID NO:14237 | SEQ ID NO:22249 |
| | | AA | SYAMS | AISGRGGNTFYADSVKG | RVTDYGGNDWFDP |
| | | | SEQ ID NO:6226 | SEQ ID NO:14238 | SEQ ID NO:22250 |

FIGURE 49

| | | | | | |
|---|---|---|---|---|---|
| **iPS:435433** | **21-225_152E3** | NA | AATTATGATATCAAC | TGGATGCACCCTAACAG TGGTAACACAGGCTATG CACAGAAGTTCCAGGGC | AGCAGTGGCTGGTACTTTTT TGACTAC |
| | | | SEQ ID NO:6227 | SEQ ID NO:14239 | SEQ ID NO:22251 |
| | | AA | NYDIN | WMHPNSGNTGYAQKFQG | SSGWYFFDY |
| | | | SEQ ID NO:6228 | SEQ ID NO:14240 | SEQ ID NO:22252 |
| **iPS:435435** | **21-225_152H3** | NA | AATTATGATATCAAC | TGGATGCACCCTAACAG TGGTAACACAGGCTATG CACAGAAGTTCCAGGGC | AGCAGTGGCTGGTACTGGTT TGACTAC |
| | | | SEQ ID NO:6229 | SEQ ID NO:14241 | SEQ ID NO:22253 |
| | | AA | NYDIN | WMHPNSGNTGYAQKFQG | SSGWYWFDY |
| | | | SEQ ID NO:6230 | SEQ ID NO:14242 | SEQ ID NO:22254 |
| **iPS:435437** | **21-225_152F4** | NA | AATTATGATATCAAC | TGGATGCACCCTAACAG TGGTAACACAGGCTATG CACAGAAGTTCCAGGGC | AGCAGTGGCTGGTACTGGTT CGACCCC |
| | | | SEQ ID NO:6231 | SEQ ID NO:14243 | SEQ ID NO:22255 |
| | | AA | NYDIN | WMHPNSGNTGYAQKFQG | SSGWYWFDP |
| | | | SEQ ID NO:6232 | SEQ ID NO:14244 | SEQ ID NO:22256 |
| **iPS:435439** | **21-225_152G4** | NA | AGCTATGCCATGAGC | GCTATTAGTGGTCGTGGT GGTAACACATTCTACGC AGACTCCGTGAAGGGC | CGGGTGACGGACTACGGTGG TAACGACTGGTTCGACCCC |
| | | | SEQ ID NO:6233 | SEQ ID NO:14245 | SEQ ID NO:22257 |
| | | AA | SYAMS | AISGRGGNTFYADSVKG | RVTDYGGNDWFDP |
| | | | SEQ ID NO:6234 | SEQ ID NO:14246 | SEQ ID NO:22258 |
| **iPS:435441** | **21 225 152F6** | NA | AGCTATGGCATGCAC | ATTATATGGTATGATGG AAGTTATAAATACTATG CAGACTCCGTGAAGGGC | GAGGGGTACGATTTTTGGAG TGGTTACCTTGGCTAC |

## FIGURE 49

| | | | | | |
|---|---|---|---|---|---|
| | 21-225_152F6 | | SEQ ID NO:6235 | SEQ ID NO:14247 | SEQ ID NO:22259 |
| | | AA | SYGMH | IIWYDGSYKYYADSVKG | EGYDFWSGYLGY |
| | | | SEQ ID NO:6236 | SEQ ID NO:14248 | SEQ ID NO:22260 |
| **iPS:435443** | 21-225_152E7 | NA | AACAGTGGTTACTACTGGAG C | TACAGTTATTACAGTGG GAGCACCTACTACAACC CGTCCCTCAAGAGT | GGGGGATATAACTGGAACCA TGCTTTTGATATC |
| | | | SEQ ID NO:6237 | SEQ ID NO:14249 | SEQ ID NO:22261 |
| | | AA | NSGYYWS | YSYYSGSTYYNPSLKS | GGYNWNHAFDI |
| | | | SEQ ID NO:6238 | SEQ ID NO:14250 | SEQ ID NO:22262 |
| **iPS:435445** | 21-225_152F7 | NA | AGCTATATCATGCAC | GTTATATGGTATGATGG AAGTAATAAATACTATG CAGACTCCGTGAAGGGC | GAGGAGTATAGCAGCAGCTG GTACGGGTACGGTATGGACG TC |
| | | | SEQ ID NO:6239 | SEQ ID NO:14251 | SEQ ID NO:22263 |
| | | AA | SYIMH | VIWYDGSNKYYADSVKG | EEYSSSWYGYGMDV |
| | | | SEQ ID NO:6240 | SEQ ID NO:14252 | SEQ ID NO:22264 |
| **iPS:435447** | 21-225_152H7 | NA | AGCTATGCCATGAAC | GCTATTAGTGGTAGTGGT GGTAACACATTCTACGC AGACTCCGTGAAGGGC | AAGGATAATGATTACGTTTG GGGGAGTCCTTACTTTGACT AC |
| | | | SEQ ID NO:6241 | SEQ ID NO:14253 | SEQ ID NO:22265 |
| | | AA | SYAMN | AISGSGGNTFYADSVKG | KDNDYVWGSPYFDY |
| | | | SEQ ID NO:6242 | SEQ ID NO:14254 | SEQ ID NO:22266 |
| **iPS:435449** | 21-225_152H9 | NA | AGAAGTAGTTACTACTGGGG C | AGTATCTATTATAGTGGG AGCGCCTCCTACAACCC GTCCCTCAAGAGT | CTTGATCTCCAGTGGAGTTTT GACTTC |
| | | | SEQ ID NO:6243 | SEQ ID NO:14255 | SEQ ID NO:22267 |
| | | AA | RSSYYWG | SIYYSGSASYNPSLKS | LDLQWSFDF |
| | | | SEQ ID NO:6244 | SEQ ID NO:14256 | SEQ ID NO:22268 |

# FIGURE 49

| | | | | | |
|---|---|---|---|---|---|
| **iPS:435451** | **21-225_152D10** | NA | AATTACTACTGGAGC | CGTATCGATACCAGTGGGATCACCAACTACAACCCCTCCCTCAAGAGT | GAGGGGGGATTGGGAGCTACCTTCTTTGACTAC |
| | | | SEQ ID NO:6245 | SEQ ID NO:14257 | SEQ ID NO:22269 |
| | | AA | NYYWS | RIDTSGITNYNPSLKS | EGGLGATFFDY |
| | | | SEQ ID NO:6246 | SEQ ID NO:14258 | SEQ ID NO:22270 |
| **iPS:435453** | **21-225_152G10** | NA | AGCTATGCCATGAGC | GCTATTAGTGGTCGTGGTGGTAACACATTCTACGCAGACTCCGTGAAGGGC | AAGGATTATGATTACGTTTGGGGGAGTCCTTACTTTGACTAC |
| | | | SEQ ID NO:6247 | SEQ ID NO:14259 | SEQ ID NO:22271 |
| | | AA | SYAMS | AISGRGGNTFYADSVKG | KDYDYVWGSPYFDY |
| | | | SEQ ID NO:6248 | SEQ ID NO:14260 | SEQ ID NO:22272 |
| **iPS:435455** | **21-225_152B11** | NA | AGCTATGCCATGAAC | GCTATTAGTGGTCGTGGTGGTAACACATTCTACGCAGACTCCGTGAAGGGC | CGGGTGACGGACTACGGTGGTAACGACTGGTTCGACCCC |
| | | | SEQ ID NO:6249 | SEQ ID NO:14261 | SEQ ID NO:22273 |
| | | AA | SYAMN | AISGRGGNTFYADSVKG | RVTDYGGNDWFDP |
| | | | SEQ ID NO:6250 | SEQ ID NO:14262 | SEQ ID NO:22274 |
| **iPS:435457** | **21-225_152C11** | NA | AACTATGGCATGCAC | ATTATATGGTATGATGGAAGTAATAAATACTATGCAGACTCCGTGAAGGGC | GAGGCTTACGATTTTTGGAGTGGTTATTTTGACTAC |
| | | | SEQ ID NO:6251 | SEQ ID NO:14263 | SEQ ID NO:22275 |
| | | AA | NYGMH | IIWYDGSNKYYADSVKG | EAYDFWSGYFDY |
| | | | SEQ ID NO:6252 | SEQ ID NO:14264 | SEQ ID NO:22276 |
| **iPS:435459** | **21-225_152E12** | NA | AATTATGATATCAAC | TGGATGAACCCTAACAGTGGTAACACAGGCTATGCACAGAAGTTCCAGGGC | AGCAGTGGCTGGTACTACTTTGACTAC |
| | | | SEQ ID NO:6253 | SEQ ID NO:14265 | SEQ ID NO:22277 |

FIGURE 49

| | | | | | |
|---|---|---|---|---|---|
| | | AA | NYDIN | WMNPNSGNTGYAQKFQG | SSGWYYFDY |
| | | | SEQ ID NO:6254 | SEQ ID NO:14266 | SEQ ID NO:22278 |
| **iPS:435461** | **21-225_153A1** | NA | AGCTATGTCATGAGT | GCTATTAGTGGAAGTGG TGATAGAACATACTACG CAGACTCCGTGAAGGGC | ACGGCGACTAAGGACTAC |
| | | | SEQ ID NO:6255 | SEQ ID NO:14267 | SEQ ID NO:22279 |
| | | AA | SYVMS | AISGSGDRTYYADSVKG | TATKDY |
| | | | SEQ ID NO:6256 | SEQ ID NO:14268 | SEQ ID NO:22280 |
| **iPS:435463** | **21-225_153D2** | NA | AGCTATGGCATGCAC | ATTATATGGTATGATGG AAGTTATAAATACTATG CAGACTCCGTGAAGGGC | GAGGGGTACGATTTTTGGAG TGGTTACCTTGGCTAC |
| | | | SEQ ID NO:6257 | SEQ ID NO:14269 | SEQ ID NO:22281 |
| | | AA | SYGMH | IIWYDGSYKYYADSVKG | EGYDFWSGYLGY |
| | | | SEQ ID NO:6258 | SEQ ID NO:14270 | SEQ ID NO:22282 |
| **iPS:435465** | **21-225_153A6** | NA | AACAGTGGTTACTACTGGAG C | TACAGCTATTACAGTGG GAGCACCTACTACAACC CGTCCCTCAAGAGT | GGGGGATATAACTGGAACCA TGCTTTTGATATC |
| | | | SEQ ID NO:6259 | SEQ ID NO:14271 | SEQ ID NO:22283 |
| | | AA | NSGYYWS | YSYYSGSTYYNPSLKS | GGYNWNHAFDI |
| | | | SEQ ID NO:6260 | SEQ ID NO:14272 | SEQ ID NO:22284 |
| **iPS:435467** | **21-225_153B9** | NA | AGTTACTACTGGAGC | CGTATCGATACCAGTGG GATCACCAACTACAACC CCTCCCTCAAGAGT | GAGGGGGGAGTGGGAGCTA CGTACTTTGACTAC |
| | | | SEQ ID NO:6261 | SEQ ID NO:14273 | SEQ ID NO:22285 |
| | | AA | SYYWS | RIDTSGITNYNPSLKS | EGGVGATYFDY |
| | | | SEQ ID NO:6262 | SEQ ID NO:14274 | SEQ ID NO:22286 |

FIGURE 49

| | | | | | |
|---|---|---|---|---|---|
| **iPS:435469** | **21-225_153G9** | NA | AGCTATGGCATGCAC | CTTATATTCTATGATGGAAGTAATAAATACTATGCAGACTCCGTGAAGGGC | CGCTATAGCCGCAGCTGGGCCGGGGGTATGGACGTC |
| | | | SEQ ID NO:6263 | SEQ ID NO:14275 | SEQ ID NO:22287 |
| | | AA | SYGMH | LIFYDGSNKYYADSVKG | RYSRSWAGGMDV |
| | | | SEQ ID NO:6264 | SEQ ID NO:14276 | SEQ ID NO:22288 |
| **iPS:435471** | **21-225_153F11** | NA | AATTATGATATCAAC | TGGATGCACCCTAACAGTGGTAACACAGGCTATGCACAGAAGTTCCAGGGC | AGCAGTGGCTGGTACTTCTTTGACAAC |
| | | | SEQ ID NO:6265 | SEQ ID NO:14277 | SEQ ID NO:22289 |
| | | AA | NYDIN | WMHPNSGNTGYAQKFQG | SSGWYFFDN |
| | | | SEQ ID NO:6266 | SEQ ID NO:14278 | SEQ ID NO:22290 |
| **iPS:435475** | **21-225_154H6** | NA | AATTATGATATCAAC | TGGATGAACCCTAACAGTGGTAACACAGGCTATGCACAGAAGTTCCAGGGC | AGCAGTGGCTGGTACATCTTTGACTAC |
| | | | SEQ ID NO:6267 | SEQ ID NO:14279 | SEQ ID NO:22291 |
| | | AA | NYDIN | WMNPNSGNTGYAQKFQG | SSGWYIFDY |
| | | | SEQ ID NO:6268 | SEQ ID NO:14280 | SEQ ID NO:22292 |
| **iPS:435479** | **21-225_154E9** | NA | AGCTATGCCATGAGC | GCTATTAGTGGTCGTGGTGGTAACACATTCTACGCAGACTCCGTGAAGGGC | AGGGGATTTCGATTTTTGGAGTGGTTGGGGGGCTTTGACTAC |
| | | | SEQ ID NO:6269 | SEQ ID NO:14281 | SEQ ID NO:22293 |
| | | AA | SYAMS | AISGRGGNTFYADSVKG | RGFRFLEWLGGFDY |
| | | | SEQ ID NO:6270 | SEQ ID NO:14282 | SEQ ID NO:22294 |
| **iPS:435481** | | NA | AATTATGATATCAAC | TGGATGAACCCTAACAGTGGTAACACAGGCTATGCACAGAAGTTCCAGGGC | AGCAGTGGCTGGTACTACTTCGACTAC |

FIGURE 49

| | | | | | |
|---|---|---|---|---|---|
| | **21-225_154A11** | | SEQ ID NO:6271 | SEQ ID NO:14283 | SEQ ID NO:22295 |
| | | AA | NYDIN | WMNPNSGNTGYAQKFQG | SSGWYYFDY |
| | | | SEQ ID NO:6272 | SEQ ID NO:14284 | SEQ ID NO:22296 |
| **iPS:435483** | **21-225_155A4** | NA | AGCTATGCCATGAGC | GCTATTAGTGGTAGTGGT GGTAACACATTCTACGC AGACTCCGTGAAGGGC | AAGGATTATGATTACGTTTG GGGGAGTCCTTACTTTGACT AC |
| | | | SEQ ID NO:6273 | SEQ ID NO:14285 | SEQ ID NO:22297 |
| | | AA | SYAMS | AISGSGGNTFYADSVKG | KDYDYVWGSPYFDY |
| | | | SEQ ID NO:6274 | SEQ ID NO:14286 | SEQ ID NO:22298 |
| **iPS:435485** | **21-225_155B4** | NA | AGCTATGCCATGAGC | GCTATTAGTGGTAGTGGT GGTAACACATTCTACGC AGACTCCGTGAAGGGC | AAGGATTATGATTACGTTTG GGGGAGTCCTTACTTTGACT AC |
| | | | SEQ ID NO:6275 | SEQ ID NO:14287 | SEQ ID NO:22299 |
| | | AA | SYAMS | AISGSGGNTFYADSVKG | KDYDYVWGSPYFDY |
| | | | SEQ ID NO:6276 | SEQ ID NO:14288 | SEQ ID NO:22300 |
| **iPS:435487** | **21-225_155C4** | NA | AGCTATGCCATGAGC | GCTATTAGTGGTCGTGGT GGTAACACATTCTACGC AGACTCCGTGAAGGGC | CGGGTGACGGACTACGGTGG TAACGACTGGTTCGACCCC |
| | | | SEQ ID NO:6277 | SEQ ID NO:14289 | SEQ ID NO:22301 |
| | | AA | SYAMS | AISGRGGNTFYADSVKG | RVTDYGGNDWFDP |
| | | | SEQ ID NO:6278 | SEQ ID NO:14290 | SEQ ID NO:22302 |
| **iPS:435489** | **21-225_155A5** | NA | AGCTATGGCATGCAC | ATTATATGGTATGATGG AAGTAGTAAATACTATG CAGACTCCGTGAAGGGC | GATCGATACGATTTTTGGAG TGGTCACTTTGACTAC |
| | | | SEQ ID NO:6279 | SEQ ID NO:14291 | SEQ ID NO:22303 |
| | | AA | SYGMH | IIWYDGSSKYYADSVKG | DRYDFWSGHFDY |
| | | | SEQ ID NO:6280 | SEQ ID NO:14292 | SEQ ID NO:22304 |

FIGURE 49

| | | | | | |
|---|---|---|---|---|---|
| **iPS:435491** | **21-225_155E5** | NA | AATTATGATATCAAC | TGGATGCACCCTAACAGTGGTAGTACAGGCTATGCACAGAGGTTCCAGGGC | AGCAGTGGCTGGTACTATTTTGACTAC |
| | | | SEQ ID NO:6281 | SEQ ID NO:14293 | SEQ ID NO:22305 |
| | | AA | NYDIN | WMHPNSGSTGYAQRFQG | SSGWYYFDY |
| | | | SEQ ID NO:6282 | SEQ ID NO:14294 | SEQ ID NO:22306 |
| **iPS:435495** | **21-225_155B6** | NA | AATTATGATATCAAC | TGGATGAACCCTAACAGTGGTAACACAGGCTATGCACAGAAGTTCCAGGGC | AGCAGTGGCTGGTACTACTTTGACTAC |
| | | | SEQ ID NO:6283 | SEQ ID NO:14295 | SEQ ID NO:22307 |
| | | AA | NYDIN | WMNPNSGNTGYAQKFQG | SSGWYYFDY |
| | | | SEQ ID NO:6284 | SEQ ID NO:14296 | SEQ ID NO:22308 |
| **iPS:435497** | **21-225_155H9** | NA | AGCTATGCCATGAAC | ACTATTAGTGGTAGAGGTCTTGGCACATACTACGCAGACTCCGTGAAGGGC | GACCATGACTACGGTGACTACAATATCTACTTTGACTAC |
| | | | SEQ ID NO:6285 | SEQ ID NO:14297 | SEQ ID NO:22309 |
| | | AA | SYAMN | TISGRGLGTYYADSVKG | DHDYGDYNIYFDY |
| | | | SEQ ID NO:6286 | SEQ ID NO:14298 | SEQ ID NO:22310 |
| **iPS:435499** | **21-225_156G1** | NA | AGAAGTAGTTACTACTGGGGC | AGTATCTATTATAGTGGGAGCGCCTCCTACAACCCGTCCCTCAAGAGT | CTTGATCTCCAGTGGAGTTTTGACTTC |
| | | | SEQ ID NO:6287 | SEQ ID NO:14299 | SEQ ID NO:22311 |
| | | AA | RSSYYWG | SIYYSGSASYNPSLKS | LDLQWSFDF |
| | | | SEQ ID NO:6288 | SEQ ID NO:14300 | SEQ ID NO:22312 |
| **iPS:435501** | **21-225_156H1** | NA | AATTATGATATCAAC | TGGGTGCACCCTAACAGTGGTAACACAGGCTATGCACAGAAGTTCCAGGGC | AGCAGTGGCTGGTACTACTTTGACTAC |
| | | | SEQ ID NO:6289 | SEQ ID NO:14301 | SEQ ID NO:22313 |

FIGURE 49

| | | | | | |
|---|---|---|---|---|---|
| | | AA | NYDIN | WVHPNSGNTGYAQKFQG | SSGWYYFDY |
| | | | SEQ ID NO:6290 | SEQ ID NO:14302 | SEQ ID NO:22314 |
| **iPS:435503** | **21-225_156E4** | NA | AGCTATGCCATGAGC | GCTATTAGTGGTCGTGGTGGTAACACATTCTACGCAGACTCCGTGAAGGGC | CGGGTGACGGACTACGGTGGTAACGACTGGTTCGACCCC |
| | | | SEQ ID NO:6291 | SEQ ID NO:14303 | SEQ ID NO:22315 |
| | | AA | SYAMS | AISGRGGNTFYADSVKG | RVTDYGGNDWFDP |
| | | | SEQ ID NO:6292 | SEQ ID NO:14304 | SEQ ID NO:22316 |
| **iPS:435505** | **21-225_157C1** | NA | AGCTATAGAATGAAC | TCCATGAGTAATAGTAGTAGTTCCATATACTACGCAGACTCAGTGAAGGGC | CAGGCAGCCCAGGACTAC |
| | | | SEQ ID NO:6293 | SEQ ID NO:14305 | SEQ ID NO:22317 |
| | | AA | SYRMN | SMSNSSSSIYYADSVKG | QAAQDY |
| | | | SEQ ID NO:6294 | SEQ ID NO:14306 | SEQ ID NO:22318 |
| **iPS:435509** | **21-225_157H1** | NA | AGTTATGCCATGAGG | GATATTAGTGGTAGTGGTGGTACCACATACTACGCAGACTCCGTGAAGGGC | ACCTACCTC |
| | | | SEQ ID NO:6295 | SEQ ID NO:14307 | SEQ ID NO:22319 |
| | | AA | SYAMR | DISGSGGTTYYADSVKG | TYL |
| | | | SEQ ID NO:6296 | SEQ ID NO:14308 | SEQ ID NO:22320 |
| **iPS:435511** | **21-225_157C3** | NA | ACCTATGGCATGCAC | GTTATATGGTATGATGTAAATAATAAATACTATGCAGACTCCGTGAAGGGC | GAGCTGGGGTTCCTCTCTGACTAT |
| | | | SEQ ID NO:6297 | SEQ ID NO:14309 | SEQ ID NO:22321 |
| | | AA | TYGMH | VIWYDVNNKYYADSVKG | ELGFLSDY |
| | | | SEQ ID NO:6298 | SEQ ID NO:14310 | SEQ ID NO:22322 |

# FIGURE 49

| | | | | | |
|---|---|---|---|---|---|
| **iPS:435513** | **21-225_157F3** | NA | ACCTATGCCATGAGC | GTTATTAGTGGTAGTGGTGGTAGCACATACTACGCAGACTCCGTGAAGGGC | AGGAGCAGTGGCTGGTACGAGGATGCTCTTGATATC |
| | | | SEQ ID NO:6299 | SEQ ID NO:14311 | SEQ ID NO:22323 |
| | | AA | TYAMS | VISGSGGSTYYADSVKG | RSSGWYEDALDI |
| | | | SEQ ID NO:6300 | SEQ ID NO:14312 | SEQ ID NO:22324 |
| **iPS:435515** | **21-225_157E4** | NA | AGCTATACCATGAAC | TCCATTAGTGGTAGTAGTAGTTACATATACTACGCAGACTCAGTGAAGGGC | GTAGCTACCTTTGACTAC |
| | | | SEQ ID NO:6301 | SEQ ID NO:14313 | SEQ ID NO:22325 |
| | | AA | SYTMN | SISGSSSYIYYADSVKG | VATFDY |
| | | | SEQ ID NO:6302 | SEQ ID NO:14314 | SEQ ID NO:22326 |
| **iPS:435521** | **21-225_157H4** | NA | AGTTATAGCATGAAC | TCCATTAGTAGTAGTAGTACTTACATATACTACGCAGACTCAGTGAAGGGC | GATAGAGGGTCCATC |
| | | | SEQ ID NO:6303 | SEQ ID NO:14315 | SEQ ID NO:22327 |
| | | AA | SYSMN | SISSSSTYIYYADSVKG | DRGSI |
| | | | SEQ ID NO:6304 | SEQ ID NO:14316 | SEQ ID NO:22328 |
| **iPS:435523** | **21-225_157G5** | NA | AGCTATGTCATGAGC | GCTATGAGTGGTAGTGGTGGTAGAACATACTACGCAGACTCCGTGAAGGGC | TATACCTGGAACGGCTAC |
| | | | SEQ ID NO:6305 | SEQ ID NO:14317 | SEQ ID NO:22329 |
| | | AA | SYVMS | AMSGSGGRTYYADSVKG | YTWNGY |
| | | | SEQ ID NO:6306 | SEQ ID NO:14318 | SEQ ID NO:22330 |
| **iPS:435525** | **21-225_157E7** | NA | AGTGGTAGTTACTACTGGGGC | AGTATCTACTATAGTGGGAGCACCTACTACAATCCGTCCCTCAAGAGT | CATAAAGTGGCTGGTCCCTTTGACTAC |
| | | | SEQ ID NO:6307 | SEQ ID NO:14319 | SEQ ID NO:22331 |
| | | AA | SGSYYWG | SIYYSGSTYYNPSLKS | HKVAGPFDY |
| | | | SEQ ID NO:6308 | SEQ ID NO:14320 | SEQ ID NO:22332 |

## FIGURE 49

| | | | | | |
|---|---|---|---|---|---|
| **iPS:435527** | **21-225_157G7** | NA | AGTTATAGCATGAAC | TCCATTAGTGGTAGTAGTACGTACATATACTACGCAGACTCAGTGAAGGGC | GATCGGGGCAGCAGC |
| | | | SEQ ID NO:6309 | SEQ ID NO:14321 | SEQ ID NO:22333 |
| | | AA | SYSMN | SISGSSTYIYYADSVKG | DRGSS |
| | | | SEQ ID NO:6310 | SEQ ID NO:14322 | SEQ ID NO:22334 |
| **iPS:435529** | **21-225_157H7** | NA | AGCTATAGCATGAAC | TGCATTAGTGGTAGTAGTAGTTACATATATTATGCAGACTCAGTGAAGGGC | GATCGAGGGGGCTAT |
| | | | SEQ ID NO:6311 | SEQ ID NO:14323 | SEQ ID NO:22335 |
| | | AA | SYSMN | CISGSSSYIYYADSVKG | DRGGY |
| | | | SEQ ID NO:6312 | SEQ ID NO:14324 | SEQ ID NO:22336 |
| **iPS:435531** | **21-225_157G8** | NA | AATTATGGCATGCAC | ATTATATGGTATGATGGAAGTTATAAATACTATGCAGACTCCGTGAAGGGC | GAGGGATACGATTTTTGGAGTGGTTTCTTTGACTCC |
| | | | SEQ ID NO:6313 | SEQ ID NO:14325 | SEQ ID NO:22337 |
| | | AA | NYGMH | IIWYDGSYKYYADSVKG | EGYDFWSGFFDS |
| | | | SEQ ID NO:6314 | SEQ ID NO:14326 | SEQ ID NO:22338 |
| **iPS:435533** | **21-225_157H8** | NA | AGCTATGGCATGCAC | GTTATATGGTATGATGTAAATAATAAATACTATGCAGACTCCGTGAAGGGC | GAGCTGGGGTTCCTCTCTGACTAC |
| | | | SEQ ID NO:6315 | SEQ ID NO:14327 | SEQ ID NO:22339 |
| | | AA | SYGMH | VIWYDVNNKYYADSVKG | ELGFLSDY |
| | | | SEQ ID NO:6316 | SEQ ID NO:14328 | SEQ ID NO:22340 |
| **iPS:435535** | **21-225_157H10** | NA | AGCTATACCATGAAC | TCCATTAGTGGTAGCAGTAGTTACATAAACTACGCAGACTCAGTGAAGGGC | GTGGCTCACTTTGACTAC |
| | | | SEQ ID NO:6317 | SEQ ID NO:14329 | SEQ ID NO:22341 |

FIGURE 49

| | | | | | |
|---|---|---|---|---|---|
| | | AA | SYTMN | SISGSSSYINYADSVKG | VAHFDY |
| | | | SEQ ID NO:6318 | SEQ ID NO:14330 | SEQ ID NO:22342 |
| iPS:435537 | 21-225_157H12 | NA | AGCTATAGCATGAAC | TCCATTAGTGGTAGTGGTAGTTACATAAACTACGCAGACTCAGTGAAGGGC | TCCAAGTTTGACTCC |
| | | | SEQ ID NO:6319 | SEQ ID NO:14331 | SEQ ID NO:22343 |
| | | AA | SYSMN | SISGSGSYINYADSVKG | SKFDS |
| | | | SEQ ID NO:6320 | SEQ ID NO:14332 | SEQ ID NO:22344 |
| iPS:435539 | 21-225_158G1 | NA | AGTTATGGCATGAAC | TCCATTAGTGGTAGTGGTAGTTACATATACTACGCAGACTCAGTGAAGGGC | AGCAGTGGCTGGTCT |
| | | | SEQ ID NO:6321 | SEQ ID NO:14333 | SEQ ID NO:22345 |
| | | AA | SYGMN | SISGSGSYIYYADSVKG | SSGWS |
| | | | SEQ ID NO:6322 | SEQ ID NO:14334 | SEQ ID NO:22346 |
| iPS:435543 | 21-225_158D4 | NA | GACTATGTCATGCAC | GTTATATGGTATGATGGAAGTAATAAATACTATGCAGACTCCGTGAAGGGC | GAACCGTATACTAGTGGCTGGTACGACTACGGTATGGACGTC |
| | | | SEQ ID NO:6323 | SEQ ID NO:14335 | SEQ ID NO:22347 |
| | | AA | DYVMH | VIWYDGSNKYYADSVKG | EPYTSGWYDYGMDV |
| | | | SEQ ID NO:6324 | SEQ ID NO:14336 | SEQ ID NO:22348 |
| iPS:435545 | 21-225_158F4 | NA | AGTCACTTCTGGAGC | CGTATCTATACCAGTGGGACCACCAACTACACCCCCTCCCTCAAGAGT | TTGAGCAGTGGCTGGTTTGACTAC |
| | | | SEQ ID NO:6325 | SEQ ID NO:14337 | SEQ ID NO:22349 |
| | | AA | SHFWS | RIYTSGTTNYTPSLKS | LSSGWFDY |
| | | | SEQ ID NO:6326 | SEQ ID NO:14338 | SEQ ID NO:22350 |

## FIGURE 49

| | | | | | |
|---|---|---|---|---|---|
| **iPS:435547** | **21-225_158F5** | NA | AGTTATAGCATGAAC | TCCATTAGTGGTAGTAGTACGTACATATACTACGCAGACTCAGTGAAGGGC | GATCGGGGCAGCAGC |
| | | | SEQ ID NO:6327 | SEQ ID NO:14339 | SEQ ID NO:22351 |
| | | AA | SYSMN | SISGSSTYIYYADSVKG | DRGSS |
| | | | SEQ ID NO:6328 | SEQ ID NO:14340 | SEQ ID NO:22352 |
| **iPS:435549** | **21-225_158H5** | NA | AGCTATAGCATGAAC | TCCATCAGTGGTAGTAGTACTTACATATACTACGCAGACTCAGTGAAGGGC | GATCGGGGCAGCAGC |
| | | | SEQ ID NO:6329 | SEQ ID NO:14341 | SEQ ID NO:22353 |
| | | AA | SYSMN | SISGSSTYIYYADSVKG | DRGSS |
| | | | SEQ ID NO:6330 | SEQ ID NO:14342 | SEQ ID NO:22354 |
| **iPS:435551** | **21-225_158H6** | NA | AGCTATGGCATGCAC | GTTATATGGTATGATGTAACTAATAAATACTATGCAGACTCCGTGAAGGGC | GAACTGGGATGGGCGGAGGACTAC |
| | | | SEQ ID NO:6331 | SEQ ID NO:14343 | SEQ ID NO:22355 |
| | | AA | SYGMH | VIWYDVTNKYYADSVKG | ELGWAEDY |
| | | | SEQ ID NO:6332 | SEQ ID NO:14344 | SEQ ID NO:22356 |
| **iPS:435553** | **21-225_158G8** | NA | AGCTATACCATGAAC | TTGATTAGTGGCAGTAGTAGTTACATATACTACGCAGACTCAGTGAAGGGC | GATCGAGGCAGCCTC |
| | | | SEQ ID NO:6333 | SEQ ID NO:14345 | SEQ ID NO:22357 |
| | | AA | SYTMN | LISGSSSYIYYADSVKG | DRGSL |
| | | | SEQ ID NO:6334 | SEQ ID NO:14346 | SEQ ID NO:22358 |
| **iPS:435557** | **21-225_158B12** | NA | AATTATGATATCAAC | TGGATGCACCCTAACAGTGGTAACACAGGCTATGCACAGAAGTTCCAGGGC | AGCAGTGGCTGGTACCGCTTTGACTAC |
| | | | SEQ ID NO:6335 | SEQ ID NO:14347 | SEQ ID NO:22359 |

**FIGURE 49**

| | | | | | |
|---|---|---|---|---|---|
| | | AA | NYDIN<br>SEQ ID NO:6336 | WMHPNSGNTGYAQKFQG<br>SEQ ID NO:14348 | SSGWYRFDY<br>SEQ ID NO:22360 |
| **iPS:435559** | **21-225_158H12** | NA | AGCTATGTCATGAGC<br>SEQ ID NO:6337 | GCTATTAGTGGTAGTGGT GGTAGGACAGACTACGC AGACTCCGTAAAGGGC<br>SEQ ID NO:14349 | GGGGGCTGGAACCACGAC<br>SEQ ID NO:22361 |
| | | AA | SYVMS<br>SEQ ID NO:6338 | AISGSGGRTDYADSVKG<br>SEQ ID NO:14350 | GGWNHD<br>SEQ ID NO:22362 |
| **iPS:435561** | **21-225_159F1** | NA | AGCTATAGAATGAAC<br>SEQ ID NO:6339 | TCCATAAGTGGTAGTGG TAATTACATAGACTACG CAGACTCAGTGAAGGGC<br>SEQ ID NO:14351 | GGTTGGGACGTC<br>SEQ ID NO:22363 |
| | | AA | SYRMN<br>SEQ ID NO:6340 | SISGSGNYIDYADSVKG<br>SEQ ID NO:14352 | GWDV<br>SEQ ID NO:22364 |
| **iPS:435563** | **21-225_159H2** | NA | AGTTATGATATCAAC<br>SEQ ID NO:6341 | TGGATGAACCCTAACAG TGGTAACACAGGCTATG TACAGAAGTTCCAGGGC<br>SEQ ID NO:14353 | AAGAAAACTGGGGACTAC<br>SEQ ID NO:22365 |
| | | AA | SYDIN<br>SEQ ID NO:6342 | WMNPNSGNTGYVQKFQG<br>SEQ ID NO:14354 | KKTGDY<br>SEQ ID NO:22366 |
| **iPS:435565** | **21-225_159C4** | NA | AACTATGGCATGCAC<br>SEQ ID NO:6343 | GTTATATCATATTCTGGA AACAATAAATACTATGC AGACTCCGTGAAGGGC<br>SEQ ID NO:14355 | CGGAGCAGCTCGTGGGGGG CTACGGTATGGACGTC<br>SEQ ID NO:22367 |
| | | AA | NYGMH<br>SEQ ID NO:6344 | VISYSGNNKYYADSVKG<br>SEQ ID NO:14356 | RSSSWGGYGMDV<br>SEQ ID NO:22368 |

FIGURE 49

| | | | | | |
|---|---|---|---|---|---|
| **iPS:435569** | **21-225_159C5** | NA | AGCTATGGCATACAC | GTTGTATGGTATGATGTAAATAATAAATACTATGCAGACTCCGTGAAGGGC | GAGCTGGGGTTCCTCTCTGACTAC |
| | | | SEQ ID NO:6345 | SEQ ID NO:14357 | SEQ ID NO:22369 |
| | | AA | SYGIH | VVWYDVNNKYYADSVKG | ELGFLSDY |
| | | | SEQ ID NO:6346 | SEQ ID NO:14358 | SEQ ID NO:22370 |
| **iPS:435571** | **21-225_159C8** | NA | GACTATGTCATGCAG | GTTATATGGTATGATGGAAGTAATAAATACTATGCAGACTCCGTGAAGGGC | GAACCGTATAATAGTGGCTGGTACGACTACGGTATGGACGTC |
| | | | SEQ ID NO:6347 | SEQ ID NO:14359 | SEQ ID NO:22371 |
| | | AA | DYVMQ | VIWYDGSNKYYADSVKG | EPYNSGWYDYGMDV |
| | | | SEQ ID NO:6348 | SEQ ID NO:14360 | SEQ ID NO:22372 |
| **iPS:435573** | **21-225_159D8** | NA | AACTATGTCATGCAC | GTTATATGGTATGATGGAAGTAATAAATACTATGCAGACTCCGTGAAGGGC | GAACCGTATAGTAGTGGCTGGTACGACTACGGTATGGACGTC |
| | | | SEQ ID NO:6349 | SEQ ID NO:14361 | SEQ ID NO:22373 |
| | | AA | NYVMH | VIWYDGSNKYYADSVKG | EPYSSGWYDYGMDV |
| | | | SEQ ID NO:6350 | SEQ ID NO:14362 | SEQ ID NO:22374 |
| **iPS:435575** | **21-225_159H11** | NA | AGCTATACCATGAAC | TCCATTAGTGGTAGTAGTAGTTACATATACTACGCAGACTCAGTGAAGGGC | GTGAGCTGGGCTGACTGC |
| | | | SEQ ID NO:6351 | SEQ ID NO:14363 | SEQ ID NO:22375 |
| | | AA | SYTMN | SISGSSSYIYYADSVKG | VSWADC |
| | | | SEQ ID NO:6352 | SEQ ID NO:14364 | SEQ ID NO:22376 |

FIGURE 49

| | | | | | |
|---|---|---|---|---|---|
| **iPS:435577** | **21-225_160B1** | NA | AGCTATGGCATGCAC | GTTATATGGTATGATGGAAGTTATAAATACTATGCAGACTCCGTGAAGGGC | GAGGCCTACGATTTTTGGAGTGGTTATTATGACTAC |
| | | | SEQ ID NO:6353 | SEQ ID NO:14365 | SEQ ID NO:22377 |
| | | AA | SYGMH | VIWYDGSYKYYADSVKG | EAYDFWSGYYDY |
| | | | SEQ ID NO:6354 | SEQ ID NO:14366 | SEQ ID NO:22378 |
| **iPS:435579** | **21-225_160G1** | NA | AGCTATGTCATGAGC | GCTATGAGTGGTAGTGGTGGTCACACATACTACGCAGACTCCGTGAAGGGC | CATGGATACAGC |
| | | | SEQ ID NO:6355 | SEQ ID NO:14367 | SEQ ID NO:22379 |
| | | AA | SYVMS | AMSGSGGHTYYADSVKG | HGYS |
| | | | SEQ ID NO:6356 | SEQ ID NO:14368 | SEQ ID NO:22380 |
| **iPS:435581** | **21-225_160H1** | NA | AGCTATCGCATGAAC | TCCATTAGTAGTAGTACTGGTTACATGTACTACGCAGACTCAGTGAAGGGC | GATAAAGATTAC |
| | | | SEQ ID NO:6357 | SEQ ID NO:14369 | SEQ ID NO:22381 |
| | | AA | SYRMN | SISSSTGYMYYADSVKG | DKDY |
| | | | SEQ ID NO:6358 | SEQ ID NO:14370 | SEQ ID NO:22382 |
| **iPS:435583** | **21-225_160F2** | NA | AGTTATGGCATGAAC | TCCATTAGTGGTAGTGGTAGTTACATATACTACGCAGACTCAGTGAAGGGC | AGCAGTGGCTGGTCT |
| | | | SEQ ID NO:6359 | SEQ ID NO:14371 | SEQ ID NO:22383 |
| | | AA | SYGMN | SISGSGSYIYYADSVKG | SSGWS |
| | | | SEQ ID NO:6360 | SEQ ID NO:14372 | SEQ ID NO:22384 |
| **iPS:435585** | **21-225_160G3** | NA | AGCTATGTCATGAGC | GCTATGAGTGGTAGTGGTGGTCACACATACTACGCAGACTCCGTGAAGGGC | CATGGATACAGC |
| | | | SEQ ID NO:6361 | SEQ ID NO:14373 | SEQ ID NO:22385 |

**FIGURE 49**

| | | | | | |
|---|---|---|---|---|---|
| | | AA | SYVMS | AMSGSGGHTYYADSVKG | HGYS |
| | | | SEQ ID NO:6362 | SEQ ID NO:14374 | SEQ ID NO:22386 |
| **iPS:435587** | **21-225_160H3** | NA | AGAAGTAGTTACTACTGGGGC | AGTATCTATTATAGTGGGAGTACCTCCTACAACCCGTCTCTCGAGAGT | CTCTCTCAACGGTGGGACTTTGACTAC |
| | | | SEQ ID NO:6363 | SEQ ID NO:14375 | SEQ ID NO:22387 |
| | | AA | RSSYYWG | SIYYSGSTSYNPSLES | LSQRWDFDY |
| | | | SEQ ID NO:6364 | SEQ ID NO:14376 | SEQ ID NO:22388 |
| **iPS:435589** | **21-225_160A4** | NA | AATTATGATATCAAC | TGGATGCACCCTAACAGTGGTAACACAGGCTATCCACAGAAGTTCCAGGGC | AGCAGCGGCTGGTACATTTTTGACTAC |
| | | | SEQ ID NO:6365 | SEQ ID NO:14377 | SEQ ID NO:22389 |
| | | AA | NYDIN | WMHPNSGNTGYPQKFQG | SSGWYIFDY |
| | | | SEQ ID NO:6366 | SEQ ID NO:14378 | SEQ ID NO:22390 |
| **iPS:435591** | **21-225_160C4** | NA | GACTATGTCATGCAG | GTTATATGGTATGATGGAAGTAATAAATACTATGCAGACTCCGTGAAGGGC | GAACCGTATAATAGTGGCTGGTACGACTACGGTATGGACGTC |
| | | | SEQ ID NO:6367 | SEQ ID NO:14379 | SEQ ID NO:22391 |
| | | AA | DYVMQ | VIWYDGSNKYYADSVKG | EPYNSGWYDYGMDV |
| | | | SEQ ID NO:6368 | SEQ ID NO:14380 | SEQ ID NO:22392 |
| **iPS:435593** | **21-225_160F4** | NA | AGTTATAGCATGAAC | TCCATTAGTGGTAGTAGTACGTACATATACTACGCAGACTCAGTGAAGGGC | GATCGGGGCAGCAGC |
| | | | SEQ ID NO:6369 | SEQ ID NO:14381 | SEQ ID NO:22393 |
| | | AA | SYSMN | SISGSSTYIYYADSVKG | DRGSS |
| | | | SEQ ID NO:6370 | SEQ ID NO:14382 | SEQ ID NO:22394 |

FIGURE 49

| | | | | | |
|---|---|---|---|---|---|
| **iPS:435595** | **21-225_160H4** | NA | AGCTATAGGATGAAC | TCCATTAGTGGTAGTAGTAGTTACATAGACTACGCAGACTCAGTGAAGGGC | AAGAGTTGGTTTGACTAC |
| | | | SEQ ID NO:6371 | SEQ ID NO:14383 | SEQ ID NO:22395 |
| | | AA | SYRMN | SISGSSSYIDYADSVKG | KSWFDY |
| | | | SEQ ID NO:6372 | SEQ ID NO:14384 | SEQ ID NO:22396 |
| **iPS:435599** | **21-225_160B10** | NA | AGAAGTAGCTACTACTGGGGC | AATATCTATTATAGTGGGAGCGCCTACCACATTCCGTCCCTCAAGAGT | CATGACCCAAACTGGGGAGTTGACTAC |
| | | | SEQ ID NO:6373 | SEQ ID NO:14385 | SEQ ID NO:22397 |
| | | AA | RSSYYWG | NIYYSGSAYHIPSLKS | HDPNWGVDY |
| | | | SEQ ID NO:6374 | SEQ ID NO:14386 | SEQ ID NO:22398 |
| **iPS:435601** | **21-225_160G10** | NA | AGCTTTGGCATGCAC | GTCATATGGTATGATGGAAGTTATAAATACTATGCAGACTCCGTGAAGGGC | GTTGGTATAGAAGTGGCTGGTGACTACTACTTCGGTATGGAAGTC |
| | | | SEQ ID NO:6375 | SEQ ID NO:14387 | SEQ ID NO:22399 |
| | | AA | SFGMH | VIWYDGSYKYYADSVKG | VGIEVAGDYYFGMEV |
| | | | SEQ ID NO:6376 | SEQ ID NO:14388 | SEQ ID NO:22400 |
| **iPS:435605** | **21-225_161A4** | NA | AGCAACTACATGAGC | GTTATTTATACCGGTGGTAGCACATACAACGCAGACTCCGTGAAGGGC | AATTGGGGAATGGCTGGCCCCTTTGACTAC |
| | | | SEQ ID NO:6377 | SEQ ID NO:14389 | SEQ ID NO:22401 |
| | | AA | SNYMS | VIYTGGSTYNADSVKG | NWGMAGPFDY |
| | | | SEQ ID NO:6378 | SEQ ID NO:14390 | SEQ ID NO:22402 |
| **iPS:435607** | **21-225_161G4** | NA | AGCTATGGCATGCAC | GTTATATCATATGGTGGAAGTAATAAATACCATGCAGACTCCGTGAAGGGC | CGGAGCAGCTCGTCGGGGGGCTACGGTATGGACGTC |
| | | | SEQ ID NO:6379 | SEQ ID NO:14391 | SEQ ID NO:22403 |

**FIGURE 49**

| | | | | | |
|---|---|---|---|---|---|
| | | AA | SYGMH | VISYGGSNKYHADSVKG | RSSSSGGYGMDV |
| | | | SEQ ID NO:6380 | SEQ ID NO:14392 | SEQ ID NO:22404 |
| **iPS:435609** | **21-225_161F7** | NA | GACTTTGGCTTGCAC | GTTATATGGTTTGATGGAAGTAATAAATACTATGCAGACTCCGTGAAGGGC | GAGATTGGCTGGCTCTCTGACTAC |
| | | | SEQ ID NO:6381 | SEQ ID NO:14393 | SEQ ID NO:22405 |
| | | AA | DFGLH | VIWFDGSNKYYADSVKG | EIGWLSDY |
| | | | SEQ ID NO:6382 | SEQ ID NO:14394 | SEQ ID NO:22406 |
| **iPS:435611** | **21-225_161F10** | NA | AGCTATGGCATGCAC | ATTATATCATATTCTGGAAGAAATGATTTCTATGCAGACTCCGTGAAGGGC | CGTATAGCAGCAGCTGGTCACTACGGTATGGACGTC |
| | | | SEQ ID NO:6383 | SEQ ID NO:14395 | SEQ ID NO:22407 |
| | | AA | SYGMH | IISYSGRNDFYADSVKG | RIAAAGHYGMDV |
| | | | SEQ ID NO:6384 | SEQ ID NO:14396 | SEQ ID NO:22408 |
| **iPS:435613** | **21-225_161D11** | NA | GACTTTGGCTTGCAC | GTTATATGGTTTGATGGAAGTAATAAATACTATGCAGACTCCGTGAAGGGC | GAGATTGGCTGGCTCTCTGACTAC |
| | | | SEQ ID NO:6385 | SEQ ID NO:14397 | SEQ ID NO:22409 |
| | | AA | DFGLH | VIWFDGSNKYYADSVKG | EIGWLSDY |
| | | | SEQ ID NO:6386 | SEQ ID NO:14398 | SEQ ID NO:22410 |
| **iPS:435615** | **21-225_161G12** | NA | GACTATGTCATGCAG | GTTATATGGTATGATGGAAGTAATAAATACTATGCAGACTCCGTGAAGGGC | GAACCGTATAATAGTGGCTGGTACGACTACGGTATGGACGTC |
| | | | SEQ ID NO:6387 | SEQ ID NO:14399 | SEQ ID NO:22411 |
| | | AA | DYVMQ | VIWYDGSNKYYADSVKG | EPYNSGWYDYGMDV |
| | | | SEQ ID NO:6388 | SEQ ID NO:14400 | SEQ ID NO:22412 |

# FIGURE 49

| | | | | | |
|---|---|---|---|---|---|
| **iPS:435617** | **21-225_162F2** | NA | AGTTATAGCATGAAC | TCCATTAGTGGTAGTAGT ACGTACATATACTACGC AGACTCAGTGAAGGGC | GATCGGGGCAGCAGC |
| | | | SEQ ID NO:6389 | SEQ ID NO:14401 | SEQ ID NO:22413 |
| | | AA | SYSMN | SISGSSTYIYYADSVKG | DRGSS |
| | | | SEQ ID NO:6390 | SEQ ID NO:14402 | SEQ ID NO:22414 |
| **iPS:435621** | **21-225_162H3** | NA | AGTTATAGCATGAAC | TCCATTAGTGGTAGTAGT ACGTACATATACTACGC AGACTCAGTGAAGGGC | GATCGGGGCAGCAGC |
| | | | SEQ ID NO:6391 | SEQ ID NO:14403 | SEQ ID NO:22415 |
| | | AA | SYSMN | SISGSSTYIYYADSVKG | DRGSS |
| | | | SEQ ID NO:6392 | SEQ ID NO:14404 | SEQ ID NO:22416 |
| **iPS:435623** | **21-225_162D5** | NA | AATTATGATATCAAC | TGGATGCACCCTAACAG TGGTAACACAGGCTATG CACAGAAGTTCCAGGGC | AGCAGTGGCTGGTACTTCTT TGACTAC |
| | | | SEQ ID NO:6393 | SEQ ID NO:14405 | SEQ ID NO:22417 |
| | | AA | NYDIN | WMHPNSGNTGYAQKFQG | SSGWYFFDY |
| | | | SEQ ID NO:6394 | SEQ ID NO:14406 | SEQ ID NO:22418 |
| **iPS:435627** | **21-225_162F6** | NA | AATTATGATATCAAC | TGGATGCACCCTAACAG TGGTAACACAGGCTATG CACAGAAGTTCCAGGGC | AGCAGTGGCTGGTACCGCTT TGACTAC |
| | | | SEQ ID NO:6395 | SEQ ID NO:14407 | SEQ ID NO:22419 |
| | | AA | NYDIN | WMHPNSGNTGYAQKFQG | SSGWYRFDY |
| | | | SEQ ID NO:6396 | SEQ ID NO:14408 | SEQ ID NO:22420 |
| **iPS:435629** | **21 225 162H6** | NA | AACTATGGCATGCAC | GTTATATGGTATGATGG AAGTAATAAATACTATG CAGACTCCGTGAAGGGC | AAAGGTATAGCAGCAGTTGG AGACTACTACTACGGTATGG ACGTC |

FIGURE 49

| | | | | | |
|---|---|---|---|---|---|
| | | | SEQ ID NO:6397 | SEQ ID NO:14409 | SEQ ID NO:22421 |
| | **21-225_162H6** | AA | NYGMH | VIWYDGSNKYYADSVKG | KGIAAVGDYYYGMDV |
| | | | SEQ ID NO:6398 | SEQ ID NO:14410 | SEQ ID NO:22422 |
| **iPS:435635** | **21-225_163F1** | NA | AGCTATAGCATGAAC | TCCATTAGTGGTAGTGGT AGTTACATATACTACGC AGACTCAGTGAAGGGC | TATAGCAGCTCGCACTAT |
| | | | SEQ ID NO:6399 | SEQ ID NO:14411 | SEQ ID NO:22423 |
| | | AA | SYSMN | SISGSGSYIYYADSVKG | YSSSHY |
| | | | SEQ ID NO:6400 | SEQ ID NO:14412 | SEQ ID NO:22424 |
| **iPS:435637** | **21-225_163E2** | NA | AGCTATAGCATGAAC | TCCACTAGTGGGAGTTCT ACTTACATATACTACGCA GACTCAGTGAAGGGC | GATCGAGGCAGCCTC |
| | | | SEQ ID NO:6401 | SEQ ID NO:14413 | SEQ ID NO:22425 |
| | | AA | SYSMN | STSGSSTYIYYADSVKG | DRGSL |
| | | | SEQ ID NO:6402 | SEQ ID NO:14414 | SEQ ID NO:22426 |
| **iPS:435639** | **21-225_163G6** | NA | AGTTATAGCATGAGC | TCCATTAGTGGTAGTAGT GCTTACATATACTACGCA GACTCAGTGAAGGGC | TTGAGCGGTATGGACGTC |
| | | | SEQ ID NO:6403 | SEQ ID NO:14415 | SEQ ID NO:22427 |
| | | AA | SYSMS | SISGSSAYIYYADSVKG | LSGMDV |
| | | | SEQ ID NO:6404 | SEQ ID NO:14416 | SEQ ID NO:22428 |
| **iPS:435641** | **21-225_163F9** | NA | AGCTATAGCATGAAC | TCCATTAGTGGTAGTAGT ACTTACATATACTACGCA GACTCAGTGAAGGGC | GATCGAGGCAGCCTC |
| | | | SEQ ID NO:6405 | SEQ ID NO:14417 | SEQ ID NO:22429 |
| | | AA | SYSMN | SISGSSTYIYYADSVKG | DRGSL |
| | | | SEQ ID NO:6406 | SEQ ID NO:14418 | SEQ ID NO:22430 |

## FIGURE 49

| | | | | | |
|---|---|---|---|---|---|
| **iPS:435643** | **21-225_163G10** | NA | AGCTATAGCATGAAC | TCCATTAGTGGTAGTAGTACTTACATATACTACGCAGACTCAGTGAAGGGC | GCCCGTATGGACGTC |
| | | | SEQ ID NO:6407 | SEQ ID NO:14419 | SEQ ID NO:22431 |
| | | AA | SYSMN | SISGSSTYIYYADSVKG | ARMDV |
| | | | SEQ ID NO:6408 | SEQ ID NO:14420 | SEQ ID NO:22432 |
| **iPS:435649** | **21-225_165H2** | NA | CATTATGATATCAAC | TGGATGCACCCTAACAGTCATAAGACAGGCTATGCACAGAAGTTCCAGGGC | AGCAGTGGCTGGTACATGTTTGACTAC |
| | | | SEQ ID NO:6409 | SEQ ID NO:14421 | SEQ ID NO:22433 |
| | | AA | HYDIN | WMHPNSHKTGYAQKFQG | SSGWYMFDY |
| | | | SEQ ID NO:6410 | SEQ ID NO:14422 | SEQ ID NO:22434 |
| **iPS:435653** | **21-225_166H12** | NA | AGCTATAGCATGAGC | TCCATTAGTGGGAGTAGTAGTTACAGTTACTACGCAGACTCAGTGAAGGGC | CTAACTGGCTTTGACTAC |
| | | | SEQ ID NO:6411 | SEQ ID NO:14423 | SEQ ID NO:22435 |
| | | AA | SYSMS | SISGSSSYSYYADSVKG | LTGFDY |
| | | | SEQ ID NO:6412 | SEQ ID NO:14424 | SEQ ID NO:22436 |
| **iPS:435655** | **21-225_167E2** | NA | AGCTTTGGCATGCAC | GTCATATGGTATGATGGAACTTATAAATACTATGCAGACTCCGTGAAGGGC | GTTGGTATTGAAGTGGCTGGTGACTACTACTACGGTATGGAAGTC |
| | | | SEQ ID NO:6413 | SEQ ID NO:14425 | SEQ ID NO:22437 |
| | | AA | SFGMH | VIWYDGTYKYYADSVKG | VGIEVAGDYYYGMEV |
| | | | SEQ ID NO:6414 | SEQ ID NO:14426 | SEQ ID NO:22438 |
| **iPS:435657** | **21-225_167H10** | NA | AGCTTTGGCATGCAC | GTCATATGGTATGATGGAAGTTATAAGTACCATGCAGACTCCGTGAAGGGC | GTTGGTATAGAAGTGGCTGGTGACTACTACTACGGTATGGAAGTC |
| | | | SEQ ID NO:6415 | SEQ ID NO:14427 | SEQ ID NO:22439 |

**FIGURE 49**

| | | | | | |
|---|---|---|---|---|---|
| | | AA | SFGMH | VIWYDGSYKYHADSVKG | VGIEVAGDYYYGMEV |
| | | | SEQ ID NO:6416 | SEQ ID NO:14428 | SEQ ID NO:22440 |
| **iPS:435659** | **21-225_167D12** | NA | AGCTATGTCATGAGC | GCTATGAGTGGTAGTGG TGGTAGAACATACTACG CAGACTCCGTGAAGGGC | TATACCTGGAACGGCTAC |
| | | | SEQ ID NO:6417 | SEQ ID NO:14429 | SEQ ID NO:22441 |
| | | AA | SYVMS | AMSGSGGRTYYADSVKG | YTWNGY |
| | | | SEQ ID NO:6418 | SEQ ID NO:14430 | SEQ ID NO:22442 |
| **iPS:435663** | **21-225_169B1** | NA | AGCTATGGCATGCAC | GTTATATGGTATGATGG AAGTAATAAATACTATG CAGACTCCGTGAGGGGC | GATCCCTTACGTGGATACAA TGACCCGGTTATGGACTAC |
| | | | SEQ ID NO:6419 | SEQ ID NO:14431 | SEQ ID NO:22443 |
| | | AA | SYGMH | VIWYDGSNKYYADSVRG | DPLRGYNDPVMDY |
| | | | SEQ ID NO:6420 | SEQ ID NO:14432 | SEQ ID NO:22444 |
| **iPS:435665** | **21-225_169F2** | NA | AGTTACTACTGGAGT | CGTATCGATACCAGTGG GATCACCAACTACAACC CCTCCCTCAAGAGT | GAGGGAGGAGTGGGAGCTA CCTACTTTGACTAC |
| | | | SEQ ID NO:6421 | SEQ ID NO:14433 | SEQ ID NO:22445 |
| | | AA | SYYWS | RIDTSGITNYNPSLKS | EGGVGATYFDY |
| | | | SEQ ID NO:6422 | SEQ ID NO:14434 | SEQ ID NO:22446 |
| **iPS:435667** | **21-225_169E3** | NA | GGCCATAGCATGAAC | TACATTAGCCTTAGTGGT AGTACCATAAAGTACGC AGACTCTGTGAAGGGC | AGGGGGATTACTGTGGTTCG GAATGAGGACGGTTTGGACG TC |
| | | | SEQ ID NO:6423 | SEQ ID NO:14435 | SEQ ID NO:22447 |
| | | AA | GHSMN | YISLSGSTIKYADSVKG | RGITVVRNEDGLDV |
| | | | SEQ ID NO:6424 | SEQ ID NO:14436 | SEQ ID NO:22448 |

FIGURE 49

| | | | | | |
|---|---|---|---|---|---|
| **iPS:435669** | **21-225_169F9** | NA | ACCTATGGCATGCAC | ATTATATGGTATGATGGAACTAATAAATACTATGCAGACTCCGTGAAGGGC | GATCCCTTACGTGGATACAATGACCCGGTTATGGACTAC |
| | | | SEQ ID NO:6425 | SEQ ID NO:14437 | SEQ ID NO:22449 |
| | | AA | TYGMH | IIWYDGTNKYYADSVKG | DPLRGYNDPVMDY |
| | | | SEQ ID NO:6426 | SEQ ID NO:14438 | SEQ ID NO:22450 |
| **iPS:435671** | **21-225_169H5** | NA | AGTTACTACTGGAGT | CGTATCGATACCAGTGGGATCACCAACTACAACCCCTCCCTCAAGAGT | GAGGGAGGAGTGGGAGCTACCTACTTTGACTAC |
| | | | SEQ ID NO:6427 | SEQ ID NO:14439 | SEQ ID NO:22451 |
| | | AA | SYYWS | RIDTSGITNYNPSLKS | EGGVGATYFDY |
| | | | SEQ ID NO:6428 | SEQ ID NO:14440 | SEQ ID NO:22452 |
| **iPS:435673** | **21-225_169E6** | NA | GGCCATAGCATGAAC | TACATTAGCATTAGTAGTAGTACCATAAAGTACGCAGACTCTGTGAAGGGC | AGGGGGATTACTGTGGTTCGGAATGAGGACGGTTTGGACGTC |
| | | | SEQ ID NO:6429 | SEQ ID NO:14441 | SEQ ID NO:22453 |
| | | AA | GHSMN | YISISSSTIKYADSVKG | RGITVVRNEDGLDV |
| | | | SEQ ID NO:6430 | SEQ ID NO:14442 | SEQ ID NO:22454 |
| **iPS:435675** | **21-225_169D7** | NA | AGTTACTACTGGACC | CGTATCTATACCAGTGGGAGCACCAACTACAACCCCTCCCTCAAGAGT | GTCGGGAGGTACTACTACGGTATGGACGTC |
| | | | SEQ ID NO:6431 | SEQ ID NO:14443 | SEQ ID NO:22455 |
| | | AA | SYYWT | RIYTSGSTNYNPSLKS | VGRYYYGMDV |
| | | | SEQ ID NO:6432 | SEQ ID NO:14444 | SEQ ID NO:22456 |
| **iPS:435677** | **21-225_169C10** | NA | GGCTACTTTATGCAC | TGGATCAAGCCTAAGAGTGGTGGCACAAACTCTGCACAGAGGTTTCAGGGC | GGGGGGACTACGGTGGCTACGTGGGGGGTCTTTGACTAC |
| | | | SEQ ID NO:6433 | SEQ ID NO:14445 | SEQ ID NO:22457 |
| | | AA | GYFMH | WIKPKSGGTNSAQRFQG | GGTTVATWGVFDY |

FIGURE 49

| | | | | | |
|---|---|---|---|---|---|
| | | | SEQ ID NO:6434 | SEQ ID NO:14446 | SEQ ID NO:22458 |
| **iPS:435679** | **21-225_169D10** | NA | AGCTATGTCATGAGT | GCTATTAGTGGTAGTGGTAGTAGAATATACTACGCGGACTCCGTGAAGGGC | GTGGCTTTCTTTGACTAT |
| | | | SEQ ID NO:6435 | SEQ ID NO:14447 | SEQ ID NO:22459 |
| | | AA | SYVMS | AISGSGSRIYYADSVKG | VAFFDY |
| | | | SEQ ID NO:6436 | SEQ ID NO:14448 | SEQ ID NO:22460 |
| **iPS:435681** | **21-225_169D11** | NA | GACTATGTCATGCAC | GTTATATGGTATGATGGAAGTAATAAATACTATGCAGACTCCGTGAAGGGC | GAAAGGTATAGCAGTGGCTGGTACGACTACGGTATGGACGTC |
| | | | SEQ ID NO:6437 | SEQ ID NO:14449 | SEQ ID NO:22461 |
| | | AA | DYVMH | VIWYDGSNKYYADSVKG | ERYSSGWYDYGMDV |
| | | | SEQ ID NO:6438 | SEQ ID NO:14450 | SEQ ID NO:22462 |
| **iPS:435683** | **21-225_170A1** | NA | AGCTATGGCATGCAC | ATTATATGGTATGATGGAAGTTATAAATACTATGCAGATTCCGTGAAGGGC | GATGCCCACGATTTTTGGAGTGGTTACTTTGACTCC |
| | | | SEQ ID NO:6439 | SEQ ID NO:14451 | SEQ ID NO:22463 |
| | | AA | SYGMH | IIWYDGSYKYYADSVKG | DAHDFWSGYFDS |
| | | | SEQ ID NO:6440 | SEQ ID NO:14452 | SEQ ID NO:22464 |
| **iPS:435685** | **21-225_170E1** | NA | AGCTATGTCATGAGT | GCTATTAGTGGTAGTGGTAATAGAATATACTACGCAGACTCCGTGAAGGGC | GTGGCTTTCTTTGACTAT |
| | | | SEQ ID NO:6441 | SEQ ID NO:14453 | SEQ ID NO:22465 |
| | | AA | SYVMS | AISGSGNRIYYADSVKG | VAFFDY |
| | | | SEQ ID NO:6442 | SEQ ID NO:14454 | SEQ ID NO:22466 |
| **iPS:435687** | **21 225 170H1** | NA | AGTTATTACTGGAGC | CGTATCTATACCAGTGGGAGCACCAACTACAACCCCTCCCTCAAGAGT | GTCGGGAGGTACTACTATGGTATGGACGTC |

FIGURE 49

| | | | | | |
|---|---|---|---|---|---|
| | **21-225_170H1** | | SEQ ID NO:6443 | SEQ ID NO:14455 | SEQ ID NO:22467 |
| | | AA | SYYWS | RIYTSGSTNYNPSLKS | VGRYYYGMDV |
| | | | SEQ ID NO:6444 | SEQ ID NO:14456 | SEQ ID NO:22468 |
| **iPS:435689** | **21-225_170F3** | NA | GACTATGTCATGCAC | GTTATATGGTATGATGG AAGTAATAAATACTATG AAGACTCCGTGAAGGGC | GAGACGTATAGCAGCAGCTG GTACGACTACGGTATGGACG TC |
| | | | SEQ ID NO:6445 | SEQ ID NO:14457 | SEQ ID NO:22469 |
| | | AA | DYVMH | VIWYDGSNKYYEDSVKG | ETYSSSWYDYGMDV |
| | | | SEQ ID NO:6446 | SEQ ID NO:14458 | SEQ ID NO:22470 |
| **iPS:435693** | **21-225_170G4** | NA | ACCTATGGCATGCAC | ATTATATGGTATGATGG AACTAATAAATACTATG CAGACTCCGTGAAGGGC | GATCCCTTACGTGGATACAA TGACCCGGTTATGGACTAC |
| | | | SEQ ID NO:6447 | SEQ ID NO:14459 | SEQ ID NO:22471 |
| | | AA | TYGMH | IIWYDGTNKYYADSVKG | DPLRGYNDPVMDY |
| | | | SEQ ID NO:6448 | SEQ ID NO:14460 | SEQ ID NO:22472 |
| **iPS:435695** | **21-225_170D5** | NA | AGCTATGGCATGCAC | ATTATATGGTATGATGG AACTAATAAATACTATG CAGACTCCGTGAAGGGC | GATCCCTTACGTGGATACAA TGACCCGGTTATGGACTAC |
| | | | SEQ ID NO:6449 | SEQ ID NO:14461 | SEQ ID NO:22473 |
| | | AA | SYGMH | IIWYDGTNKYYADSVKG | DPLRGYNDPVMDY |
| | | | SEQ ID NO:6450 | SEQ ID NO:14462 | SEQ ID NO:22474 |
| **iPS:435697** | **21-225_170G5** | NA | ACCTATGGCATGCAC | ATTATATGGTATGATGG GACTAATAAATACTATG CAGACTCCGTGAAGGGC | GATCCCTTACGTGGATACAA TGACCCGGTTATGGACTAC |
| | | | SEQ ID NO:6451 | SEQ ID NO:14463 | SEQ ID NO:22475 |
| | | AA | TYGMH | IIWYDGTNKYYADSVKG | DPLRGYNDPVMDY |

FIGURE 49

| | | | | | |
|---|---|---|---|---|---|
| | | | SEQ ID NO:6452 | SEQ ID NO:14464 | SEQ ID NO:22476 |
| **iPS:435699** | **21-225_170D6** | NA | GGCTACTTTATACAC | TGGATCAAGCCTAACAG TGGTGGCACAAACTCTG CACAGAGGTTTCAGGGC | GGGGGGACTACGGTGGCTAC GTGGGGGGTCTTTGACTAC |
| | | | SEQ ID NO:6453 | SEQ ID NO:14465 | SEQ ID NO:22477 |
| | | AA | GYFIH | WIKPNSGGTNSAQRFQG | GGTTVATWGVFDY |
| | | | SEQ ID NO:6454 | SEQ ID NO:14466 | SEQ ID NO:22478 |
| **iPS:435701** | **21-225_170F6** | NA | AATTATGATATCAAC | TGGATGAACCCTAACAG TGGTAACACAGGCTATG CACAGAAGTTCCAGGAC | AGCAGTGGCTGGTACTGGTT CGACCCC |
| | | | SEQ ID NO:6455 | SEQ ID NO:14467 | SEQ ID NO:22479 |
| | | AA | NYDIN | WMNPNSGNTGYAQKFQD | SSGWYWFDP |
| | | | SEQ ID NO:6456 | SEQ ID NO:14468 | SEQ ID NO:22480 |
| **iPS:435703** | **21-225_170D11** | NA | AGCTATGGCATGCAC | ATTATATGGTATGATGG AACTAATAAATACTATG CAGACTCCGTGAAGGGC | GATCCCTTACGTGGATACAA TGACCCGGTTATGGACTAC |
| | | | SEQ ID NO:6457 | SEQ ID NO:14469 | SEQ ID NO:22481 |
| | | AA | SYGMH | IIWYDGTNKYYADSVKG | DPLRGYNDPVMDY |
| | | | SEQ ID NO:6458 | SEQ ID NO:14470 | SEQ ID NO:22482 |
| **iPS:435705** | **21-225_171C3** | NA | ACCTATGGCATGCAC | ATTATATGGTATGATGG GACTAATAAATACTATG CAGACTCCGTGAAGGGC | GATCCCTTACGTGGATACAA TGACCCGGTTATGGACTAC |
| | | | SEQ ID NO:6459 | SEQ ID NO:14471 | SEQ ID NO:22483 |
| | | AA | TYGMH | IIWYDGTNKYYADSVKG | DPLRGYNDPVMDY |
| | | | SEQ ID NO:6460 | SEQ ID NO:14472 | SEQ ID NO:22484 |

FIGURE 49

| | | | | | |
|---|---|---|---|---|---|
| **iPS:435709** | **21-225_171A4** | NA | ACCTATGGCATGCAC | ATTATATGGTATGATGGAAGTAATAAATACTATGCAGACTCCGTGAAGGGC | GATCCCTTACGTGGATACAATGACCCGGTTATGGACTAC |
| | | | SEQ ID NO:6461 | SEQ ID NO:14473 | SEQ ID NO:22485 |
| | | AA | TYGMH | IIWYDGSNKYYADSVKG | DPLRGYNDPVMDY |
| | | | SEQ ID NO:6462 | SEQ ID NO:14474 | SEQ ID NO:22486 |
| **iPS:435711** | **21-225_171G4** | NA | AGCTGTGCCATGACC | GCTATTAGTGGTCGTGGTGGTACCACGTTCTACGCAGACTCCGTGAGGGGC | GATCTTATTGGGGGAGCTACTTACTTTGACTAC |
| | | | SEQ ID NO:6463 | SEQ ID NO:14475 | SEQ ID NO:22487 |
| | | AA | SCAMT | AISGRGGTTFYADSVRG | DLIGGATYFDY |
| | | | SEQ ID NO:6464 | SEQ ID NO:14476 | SEQ ID NO:22488 |
| **iPS:435713** | **21-225_171D7** | NA | AGCTATGGCATGCAC | GTTATATCATATGACGGAAACAATAGACACTATGCAGACTCCGTGCAGGGC | GATCGTCACCGTTTGGACTACTACGCTTTGGACGTC |
| | | | SEQ ID NO:6465 | SEQ ID NO:14477 | SEQ ID NO:22489 |
| | | AA | SYGMH | VISYDGNNRHYADSVQG | DRHRLDYYALDV |
| | | | SEQ ID NO:6466 | SEQ ID NO:14478 | SEQ ID NO:22490 |
| **iPS:435715** | **21-225_171A8** | NA | AGCTCTGCCATGAGC | GTTATTAGTGGTAGTGGTGGTAGCACATTCTACACAGACTCCGTGAAGGGC | TCGAATAGCAGTGGCTGGTTTGACTAC |
| | | | SEQ ID NO:6467 | SEQ ID NO:14479 | SEQ ID NO:22491 |
| | | AA | SSAMS | VISGSGGSTFYTDSVKG | SNSSGWFDY |
| | | | SEQ ID NO:6468 | SEQ ID NO:14480 | SEQ ID NO:22492 |
| **iPS:435717** | **21-225_171A9** | NA | AGCTATGCCATGACT | GCTATTAGTGGTAGTGGTGGTAACACATTCAACGCAGACTCCGTGAAGGGC | CTGGGGATCGACTACTACTACTACGGTATGGACGTC |
| | | | SEQ ID NO:6469 | SEQ ID NO:14481 | SEQ ID NO:22493 |

FIGURE 49

| | | | | | |
|---|---|---|---|---|---|
| | | AA | SYAMT | AISGSGGNTFNADSVKG | LGIDYYYYGMDV |
| | | | SEQ ID NO:6470 | SEQ ID NO:14482 | SEQ ID NO:22494 |
| **iPS:435719** | **21-225_171A11** | NA | AGCTATAGCATGAAC | TCCATTAGTGGTAGTAGTAGTTACATATACTACGCAGACTCAGTGAAGGGC | GATAGGGGCAGCTCC |
| | | | SEQ ID NO:6471 | SEQ ID NO:14483 | SEQ ID NO:22495 |
| | | AA | SYSMN | SISGSSSYIYYADSVKG | DRGSS |
| | | | SEQ ID NO:6472 | SEQ ID NO:14484 | SEQ ID NO:22496 |
| **iPS:435721** | **21-225_172B3** | NA | ACCTATGGCATGCAC | GTTATATGGTATGATGGAAGTAATAAATACTATGCAGACTCCGTGAAGGGC | GATCCCTTACGTGGATACAATGACCCGGTTATGGACTAC |
| | | | SEQ ID NO:6473 | SEQ ID NO:14485 | SEQ ID NO:22497 |
| | | AA | TYGMH | VIWYDGSNKYYADSVKG | DPLRGYNDPVMDY |
| | | | SEQ ID NO:6474 | SEQ ID NO:14486 | SEQ ID NO:22498 |
| **iPS:435723** | **21-225_172B7** | NA | AACTATGGCATGCAC | ATTATATGGTATGATGGAAGTAATAAATACTATGTAGACTCCGTGAAGGGC | GAGGCGTACGATTTTTGGAGTGGTTATTGGGACTAC |
| | | | SEQ ID NO:6475 | SEQ ID NO:14487 | SEQ ID NO:22499 |
| | | AA | NYGMH | IIWYDGSNKYYVDSVKG | EAYDFWSGYWDY |
| | | | SEQ ID NO:6476 | SEQ ID NO:14488 | SEQ ID NO:22500 |
| **iPS:435725** | **21-225_172G8** | NA | ACCTATGGCATGCAC | ATTATATGGTATGATGGAACTAATAAATACTATGCAGACTCCGTGAAGGGC | GATCCCTTACGTGGATACAATGACCCGGTTATGGACTAC |
| | | | SEQ ID NO:6477 | SEQ ID NO:14489 | SEQ ID NO:22501 |
| | | AA | TYGMH | IIWYDGTNKYYADSVKG | DPLRGYNDPVMDY |
| | | | SEQ ID NO:6478 | SEQ ID NO:14490 | SEQ ID NO:22502 |

**FIGURE 49**

| | | | | | |
|---|---|---|---|---|---|
| **iPS:435727** | **21-225_172E11** | NA | AATTATGATATCAAC | TGGATGCACCCTAACAGTGGTAACACAGGCTATGCACAGAAGTTCCAGGGC | AGCAGTGGCTGGTACCGGTTTGACTAC |
| | | | SEQ ID NO:6479 | SEQ ID NO:14491 | SEQ ID NO:22503 |
| | | AA | NYDIN | WMHPNSGNTGYAQKFQG | SSGWYRFDY |
| | | | SEQ ID NO:6480 | SEQ ID NO:14492 | SEQ ID NO:22504 |
| **iPS:435729** | **21-225_173E7** | NA | AGCTATGCCATGAGC | TTTATTAGTGGTAGTGGTGGTAACACATTCTACGCAGACTCCGTGAAGGGC | AGGGATACCTACAACGGTTGGGATGCTTTTGATATC |
| | | | SEQ ID NO:6481 | SEQ ID NO:14493 | SEQ ID NO:22505 |
| | | AA | SYAMS | FISGSGGNTFYADSVKG | RDTYNGWDAFDI |
| | | | SEQ ID NO:6482 | SEQ ID NO:14494 | SEQ ID NO:22506 |
| **iPS:435731** | **21-225_173A11** | NA | AGCTATGGCATGCAC | ATTATATGGTATGATGGAAGTAATAAATACTATGCAGACTCCGTGAAGGGC | GAGGCCTACGATTTTTGGAGTGGTTTCTTTGACTCC |
| | | | SEQ ID NO:6483 | SEQ ID NO:14495 | SEQ ID NO:22507 |
| | | AA | SYGMH | IIWYDGSNKYYADSVKG | EAYDFWSGFFDS |
| | | | SEQ ID NO:6484 | SEQ ID NO:14496 | SEQ ID NO:22508 |
| **iPS:435733** | **21-225_173C11** | NA | AGCTATGGCATACAC | CTTATATTTTATGATGGAAGTAATAAATACTATGCAGACTCCGTGAAGGGC | CGGTATAGCAGCAGCTGGTCCGGTGGTATGGACGTC |
| | | | SEQ ID NO:6485 | SEQ ID NO:14497 | SEQ ID NO:22509 |
| | | AA | SYGIH | LIFYDGSNKYYADSVKG | RYSSSWSGGMDV |
| | | | SEQ ID NO:6486 | SEQ ID NO:14498 | SEQ ID NO:22510 |

FIGURE 49

| | | | | | |
|---|---|---|---|---|---|
| **iPS:435735** | **21-225_173H12** | NA | AGCTATGGCATGCAC | ATTATATGGTATGATGGAACTAACAAATACTATGCAGACTCCGTGAAGGGC | GATCCCTTACGTGGATACAATGACCCGGTTATGGACTAC |
| | | | SEQ ID NO:6487 | SEQ ID NO:14499 | SEQ ID NO:22511 |
| | | AA | SYGMH | IIWYDGTNKYYADSVKG | DPLRGYNDPVMDY |
| | | | SEQ ID NO:6488 | SEQ ID NO:14500 | SEQ ID NO:22512 |
| **iPS:435737** | **21-225_174G5** | NA | AATTATGATATCAAC | TGGATGCACCCTAACAGTGGTAACACAGGCTATGCACAGAAGTTCCAGGGC | AGCAGTGGCTGGTACTGGTTCGACCCC |
| | | | SEQ ID NO:6489 | SEQ ID NO:14501 | SEQ ID NO:22513 |
| | | AA | NYDIN | WMHPNSGNTGYAQKFQG | SSGWYWFDP |
| | | | SEQ ID NO:6490 | SEQ ID NO:14502 | SEQ ID NO:22514 |
| **iPS:435739** | **21-225_174G7** | NA | AGCTCTGCCATGAGC | GTTATTAGTGGTAGTGGTGGTAGCACATTCTACACAGACTCCGTGAAGGGC | TCGAATAGCAGTGGCTGGTTTGACTAC |
| | | | SEQ ID NO:6491 | SEQ ID NO:14503 | SEQ ID NO:22515 |
| | | AA | SSAMS | VISGSGGSTFYTDSVKG | SNSSGWFDY |
| | | | SEQ ID NO:6492 | SEQ ID NO:14504 | SEQ ID NO:22516 |
| **iPS:435741** | **21-225_174G10** | NA | GACTATGTCATGCAC | GTTATATGGTATGATGGAAGTAATAAATACTATGCAGACTCCGTGAAGGGC | GAAAGGTATAGCAGTGGCTGGTACGACTACGGTATGGACGTC |
| | | | SEQ ID NO:6493 | SEQ ID NO:14505 | SEQ ID NO:22517 |
| | | AA | DYVMH | VIWYDGSNKYYADSVKG | ERYSSGWYDYGMDV |
| | | | SEQ ID NO:6494 | SEQ ID NO:14506 | SEQ ID NO:22518 |

FIGURE 49

| | | | | | |
|---|---|---|---|---|---|
| **iPS:435743** | **21-225_175G1** | NA | ACCTATGGCATGCAC | GTTATATGGTATGATGGAAGTAATAAATACTATGCAGACTCCGTGAAGGGC | GATCCCTTACGTGGATACAATGACCCGGTTATGGACTAC |
| | | | SEQ ID NO:6495 | SEQ ID NO:14507 | SEQ ID NO:22519 |
| | | AA | TYGMH | VIWYDGSNKYYADSVKG | DPLRGYNDPVMDY |
| | | | SEQ ID NO:6496 | SEQ ID NO:14508 | SEQ ID NO:22520 |
| **iPS:435745** | **21-225_175G3** | NA | GGCTACTTTATGCAC | TGGATCAAGCCTAAAAGTGGTGGCACAAACTGTGCACAGAGGTTTCAGGGC | GGGGGGACTACGGTGACTACGTGGGGGGTCTTTGACTAC |
| | | | SEQ ID NO:6497 | SEQ ID NO:14509 | SEQ ID NO:22521 |
| | | AA | GYFMH | WIKPKSGGTNCAQRFQG | GGTTVTTWGVFDY |
| | | | SEQ ID NO:6498 | SEQ ID NO:14510 | SEQ ID NO:22522 |
| **iPS:435747** | **21-225_175C4** | NA | AGCTATGTCATGAGC | GCTATTAGTGGTAGTGGTGATAGAACATACTACGCAGACTCCGTGAAGGGC | ACAGCGGGCTTTGACTAC |
| | | | SEQ ID NO:6499 | SEQ ID NO:14511 | SEQ ID NO:22523 |
| | | AA | SYVMS | AISGSGDRTYYADSVKG | TAGFDY |
| | | | SEQ ID NO:6500 | SEQ ID NO:14512 | SEQ ID NO:22524 |
| **iPS:435749** | **21-225_175C10** | NA | AGCTATGCCATGAGC | TCTATTAGTGGTCGTGGTGGTAGCACGTTCTACGCAGACTCCGTGAAGGGC | TCGAATAGCAGTGGCTGGTTTGACTAC |
| | | | SEQ ID NO:6501 | SEQ ID NO:14513 | SEQ ID NO:22525 |
| | | AA | SYAMS | SISGRGGSTFYADSVKG | SNSSGWFDY |
| | | | SEQ ID NO:6502 | SEQ ID NO:14514 | SEQ ID NO:22526 |
| **iPS:435751** | | NA | AATTATGATCTCAAC | TGGATGCACCCTAACAGTGGTAACACAGGCTATGCACAGAAGTTCCAGGGC | AGCAGTGGCTGGTACTACTTTGACTAC |

FIGURE 49

| | | | | | |
|---|---|---|---|---|---|
| | 21-225_175D10 | | SEQ ID NO:6503 | SEQ ID NO:14515 | SEQ ID NO:22527 |
| | | AA | NYDLN | WMHPNSGNTGYAQKFQG | SSGWYYFDY |
| | | | SEQ ID NO:6504 | SEQ ID NO:14516 | SEQ ID NO:22528 |
| iPS:435753 | 21-225_175G10 | NA | AGCTATGCCATGAGC | ATTATTAGTGGTAGTGGT GGTAACACATACTACGC AGACTCCGTGAAGGGC | AGGGATACCTGGAACGGTTG GGATGCTTTTGATATC |
| | | | SEQ ID NO:6505 | SEQ ID NO:14517 | SEQ ID NO:22529 |
| | | AA | SYAMS | IISGSGGNTYYADSVKG | RDTWNGWDAFDI |
| | | | SEQ ID NO:6506 | SEQ ID NO:14518 | SEQ ID NO:22530 |
| iPS:435755 | 21-225_176H4 | NA | AGCTATGGCATGCAC | ATTATATGGTATGATGG AAGTTATAAATACTATG CAGACTCCGTGAAGGGC | GATGCCCACGATTTTTGGAG TGGTTACTTTGCCTAC |
| | | | SEQ ID NO:6507 | SEQ ID NO:14519 | SEQ ID NO:22531 |
| | | AA | SYGMH | IIWYDGSYKYYADSVKG | DAHDFWSGYFAY |
| | | | SEQ ID NO:6508 | SEQ ID NO:14520 | SEQ ID NO:22532 |
| iPS:435759 | 21-225_176E6 | NA | GGCCATAGTATGAAC | TACATTAGCATTAGTGGT AGTACCATAAAGTACGC AGACTCTGTGAAGGGC | AGGGGGATTACTGTGGTTCG GAATGAGGACGGTTTGGACG TC |
| | | | SEQ ID NO:6509 | SEQ ID NO:14521 | SEQ ID NO:22533 |
| | | AA | GHSMN | YISISGSTIKYADSVKG | RGITVVRNEDGLDV |
| | | | SEQ ID NO:6510 | SEQ ID NO:14522 | SEQ ID NO:22534 |
| iPS:435761 | 21-225_176B11 | NA | ACCTATGGCATGCAC | ATTATATGGTATGATGG AACTAATAAATACTATG CAGACTCCGTGAAGGGC | GATCCCTTACGTGGATACAA TGACCCGGTTTTGGACTAC |
| | | | SEQ ID NO:6511 | SEQ ID NO:14523 | SEQ ID NO:22535 |
| | | AA | TYGMH | IIWYDGTNKYYADSVKG | DPLRGYNDPVLDY |
| | | | SEQ ID NO:6512 | SEQ ID NO:14524 | SEQ ID NO:22536 |

**FIGURE 49**

| | | | | | |
|---|---|---|---|---|---|
| **iPS:435763** | **21-225_176H12** | NA | GACTATGTCATGCAC | GTTATATGGTATGATGGAAGTAATAAATACTATGCCGACTCCGTGAAGGGC | GAAAAGTATAGCAGCAACTGGTACGACTACGGTATGGACGTC |
| | | | SEQ ID NO:6513 | SEQ ID NO:14525 | SEQ ID NO:22537 |
| | | AA | DYVMH | VIWYDGSNKYYADSVKG | EKYSSNWYDYGMDV |
| | | | SEQ ID NO:6514 | SEQ ID NO:14526 | SEQ ID NO:22538 |
| **iPS:435765** | **21-225_177D3** | NA | AGCTATGTCATGAAC | GGTATGAGCGGTAGTGGTGGTAGAACATACTACGCAGACTCCGTGAAGGAC | GTGACTTTCTTTGACTAT |
| | | | SEQ ID NO:6515 | SEQ ID NO:14527 | SEQ ID NO:22539 |
| | | AA | SYVMN | GMSGSGGRTYYADSVKD | VTFFDY |
| | | | SEQ ID NO:6516 | SEQ ID NO:14528 | SEQ ID NO:22540 |
| **iPS:435767** | **21-225_177B4** | NA | GATTATGTCATGCAC | GTTATATGGTATGATGGAAGTAATAAATACTATGCAGACTCCGTGAAGGGC | GAAAAGTATAGCAGCAGCTGGTACGACTACGGTTTGGACGTC |
| | | | SEQ ID NO:6517 | SEQ ID NO:14529 | SEQ ID NO:22541 |
| | | AA | DYVMH | VIWYDGSNKYYADSVKG | EKYSSSWYDYGLDV |
| | | | SEQ ID NO:6518 | SEQ ID NO:14530 | SEQ ID NO:22542 |
| **iPS:435769** | **21-225_177B6** | NA | AGTTATGCCATGAGC | GTTATTAGTGGTAGTGGTAGTAACACATACTACGTAGACTCCGTGAAGGGC | GGTTACTATGATAGTAGTGGTTATTACTACCCTTTTGACTTC |
| | | | SEQ ID NO:6519 | SEQ ID NO:14531 | SEQ ID NO:22543 |
| | | AA | SYAMS | VISGSGSNTYYVDSVKG | GYYDSSGYYYPFDF |
| | | | SEQ ID NO:6520 | SEQ ID NO:14532 | SEQ ID NO:22544 |

FIGURE 49

| | | | | | |
|---|---|---|---|---|---|
| **iPS:435771** | **21-225_177B11** | NA | AGCTATGGCATGCAC | ATTATATGGTATGATGGAAGTTATAAATACTATACAGACTCCGTGAAGGGC | GAGACTTACGATTTTTGGAGTGGTTATTTTGTCTTC |
| | | | SEQ ID NO:6521 | SEQ ID NO:14533 | SEQ ID NO:22545 |
| | | AA | SYGMH | IIWYDGSYKYYTDSVKG | ETYDFWSGYFVF |
| | | | SEQ ID NO:6522 | SEQ ID NO:14534 | SEQ ID NO:22546 |
| **iPS:435773** | **21-225_177B12** | NA | AATTATGATATCAAC | TGGATGCACCCTAACAGTGGTAACACAGGCTATGCACAGAAGTTCCAGGGC | AGCAGTGGCTGGTACTACTTTGACTTC |
| | | | SEQ ID NO:6523 | SEQ ID NO:14535 | SEQ ID NO:22547 |
| | | AA | NYDIN | WMHPNSGNTGYAQKFQG | SSGWYYFDF |
| | | | SEQ ID NO:6524 | SEQ ID NO:14536 | SEQ ID NO:22548 |
| **iPS:435775** | **21-225_178A5** | NA | AGCTATGCCATGACC | GTTATTAGTGGTAGTGGTGGTAATACATTCTACGCAGACTCCGTGAAGGGC | CGGGACGGTGACTACTTTGACTAC |
| | | | SEQ ID NO:6525 | SEQ ID NO:14537 | SEQ ID NO:22549 |
| | | AA | SYAMT | VISGSGGNTFYADSVKG | RDGDYFDY |
| | | | SEQ ID NO:6526 | SEQ ID NO:14538 | SEQ ID NO:22550 |
| **iPS:435777** | **21-225_178F7** | NA | AGCTATGCCATGACC | GTTATTAGTGGTAGTGGTGGTAACACATTCTACGCAGACTCCGTGAAGGGC | CGGTACGGTGACTACTTTGACTAC |
| | | | SEQ ID NO:6527 | SEQ ID NO:14539 | SEQ ID NO:22551 |
| | | AA | SYAMT | VISGSGGNTFYADSVKG | RYGDYFDY |
| | | | SEQ ID NO:6528 | SEQ ID NO:14540 | SEQ ID NO:22552 |
| **iPS:435779** | **21-225_178B10** | NA | ACCTATGGCATGCAC | ATTATATGGTATGATGGAACTAATAAATACTATGCAGACTCCGTGAAGGGC | GATCCCTTACGTGGATACAATGACCCGGTTATGGACTAC |
| | | | SEQ ID NO:6529 | SEQ ID NO:14541 | SEQ ID NO:22553 |

FIGURE 49

| | | | | | |
|---|---|---|---|---|---|
| | | AA | TYGMH | IIWYDGTNKYYADSVKG | DPLRGYNDPVMDY |
| | | | SEQ ID NO:6530 | SEQ ID NO:14542 | SEQ ID NO:22554 |
| **iPS:435781** | **21-225_178G10** | NA | ACCTATGGCATGCAC | GTTATATGGTATGATGG AAGTAATAAATACTACG CAGACTCCGTGAAGGGC | GAACGGTACGATTTTTGGAG TGGTCATTTTGACTAC |
| | | | SEQ ID NO:6531 | SEQ ID NO:14543 | SEQ ID NO:22555 |
| | | AA | TYGMH | VIWYDGSNKYYADSVKG | ERYDFWSGHFDY |
| | | | SEQ ID NO:6532 | SEQ ID NO:14544 | SEQ ID NO:22556 |
| **iPS:435783** | **21-225_179G1** | NA | AGCTATGCCATGACC | GTTATTAGTGGTTTTGGT GGTAACACATTCTACGC AGACTCCGTGAAGGGC | CGGTACGGTGACTACTTTGA CTAC |
| | | | SEQ ID NO:6533 | SEQ ID NO:14545 | SEQ ID NO:22557 |
| | | AA | SYAMT | VISGFGGNTFYADSVKG | RYGDYFDY |
| | | | SEQ ID NO:6534 | SEQ ID NO:14546 | SEQ ID NO:22558 |
| **iPS:435785** | **21-225_179C2** | NA | AGCTATGGCATGCAC | CTTATATTTTATGATGGA AGTAATAAATACTATGC AGACTCCGTGAAGGGC | CGGTATAGCGGCAGCTGGTC CGGTGGTATGGACGTC |
| | | | SEQ ID NO:6535 | SEQ ID NO:14547 | SEQ ID NO:22559 |
| | | AA | SYGMH | LIFYDGSNKYYADSVKG | RYSGSWSGGMDV |
| | | | SEQ ID NO:6536 | SEQ ID NO:14548 | SEQ ID NO:22560 |
| **iPS:435787** | **21-225_180A3** | NA | AGCTTTGCCATGAAC | GTTATTAGCGGTCGCGGT GGTAACACATTCTACGC AGACTCCGTGAAGGGC | CGGACTGGGGATGATGTTTT TGATGTC |
| | | | SEQ ID NO:6537 | SEQ ID NO:14549 | SEQ ID NO:22561 |
| | | AA | SFAMN | VISGRGGNTFYADSVKG | RTGDDVFDV |
| | | | SEQ ID NO:6538 | SEQ ID NO:14550 | SEQ ID NO:22562 |

FIGURE 49

| | | | | | |
|---|---|---|---|---|---|
| **iPS:435789** | **21-225_180C4** | NA | GCCTATGGCATGCAC | ATTATTTGGTATGATGGAAGTTATAAATACTATGCAGACTCCGTGAAGGGC | ACCGGTGTGGATCCCTGGGACTACTACAACGGAATGGACGTC |
| | | | SEQ ID NO:6539 | SEQ ID NO:14551 | SEQ ID NO:22563 |
| | | AA | AYGMH | IIWYDGSYKYYADSVKG | TGVDPWDYYNGMDV |
| | | | SEQ ID NO:6540 | SEQ ID NO:14552 | SEQ ID NO:22564 |
| **iPS:435791** | **21-225_180H7** | NA | GACTATGGCATGCAC | GTTATATGGTATGATGAAAATAATAAACACTATGCAGACTCCGCGAAGGGC | GAGGTTGGCTGGTCCGATGACTAC |
| | | | SEQ ID NO:6541 | SEQ ID NO:14553 | SEQ ID NO:22565 |
| | | AA | DYGMH | VIWYDENNKHYADSAKG | EVGWSDDY |
| | | | SEQ ID NO:6542 | SEQ ID NO:14554 | SEQ ID NO:22566 |
| **iPS:435793** | **21-225_180F8** | NA | AGCTATGGCATGCAC | GTTATATGGTATGATGGAAGTGATAAATACTATGAAGACTCCGTGAAGGGC | GATCATCCCCGGTGGAGCTACGGAGACTAC |
| | | | SEQ ID NO:6543 | SEQ ID NO:14555 | SEQ ID NO:22567 |
| | | AA | SYGMH | VIWYDGSDKYYEDSVKG | DHPRWSYGDY |
| | | | SEQ ID NO:6544 | SEQ ID NO:14556 | SEQ ID NO:22568 |
| **iPS:435795** | **21-225_181C2** | NA | AGCTATGGCATGCAC | ATTATATGGTATGATGGAAGTTATAAATACTATGCAGACTCCGTGAAGGGC | GATCATTACGATTTTTGGAGTGGGCACTTTGACTTC |
| | | | SEQ ID NO:6545 | SEQ ID NO:14557 | SEQ ID NO:22569 |
| | | AA | SYGMH | IIWYDGSYKYYADSVKG | DHYDFWSGHFDF |
| | | | SEQ ID NO:6546 | SEQ ID NO:14558 | SEQ ID NO:22570 |

FIGURE 49

| | | | | | |
|---|---|---|---|---|---|
| **iPS:435797** | **21-225_181G2** | NA | AGCTACAATATGCAC | TGGATCAACCCTAACAATGGTGGCTCAAACTATACACAGAAGTTTCAGGGC | AAGTTTGGGGAC |
| | | | SEQ ID NO:6547 | SEQ ID NO:14559 | SEQ ID NO:22571 |
| | | AA | SYNMH | WINPNNGGSNYTQKFQG | KFGD |
| | | | SEQ ID NO:6548 | SEQ ID NO:14560 | SEQ ID NO:22572 |
| **iPS:435799** | **21-225_181G3** | NA | AGCTATGCCATGAGT | GTTATTAGTGGTAGTGGTGGTAACACATTCTACGGAGACTCCGTGAAGGGC | CGGGAGACCTACGACTGGGGATCCGATGCTTTTGATATC |
| | | | SEQ ID NO:6549 | SEQ ID NO:14561 | SEQ ID NO:22573 |
| | | AA | SYAMS | VISGSGGNTFYGDSVKG | RETYDWGSDAFDI |
| | | | SEQ ID NO:6550 | SEQ ID NO:14562 | SEQ ID NO:22574 |
| **iPS:435801** | **21-225_181E5** | NA | AATTATGATATCAAC | TGGATGAACCCTAACAGTGGTAACACAGGCTATGCACAGAAGTTCCAGGGC | AGTAGTGGCTGGTACATCTTTGACTAC |
| | | | SEQ ID NO:6551 | SEQ ID NO:14563 | SEQ ID NO:22575 |
| | | AA | NYDIN | WMNPNSGNTGYAQKFQG | SSGWYIFDY |
| | | | SEQ ID NO:6552 | SEQ ID NO:14564 | SEQ ID NO:22576 |
| **iPS:435805** | **21-225_181A8** | NA | GACTATGGCATGCAC | GTTATATGGTATGATGAAAATAATAAACACTATGCAGACTCCGCGAAGGGC | GAGGTTGGCTGGTCCGATGACTAC |
| | | | SEQ ID NO:6553 | SEQ ID NO:14565 | SEQ ID NO:22577 |
| | | AA | DYGMH | VIWYDENNKHYADSAKG | EVGWSDDY |
| | | | SEQ ID NO:6554 | SEQ ID NO:14566 | SEQ ID NO:22578 |

FIGURE 49

| | | | | | |
|---|---|---|---|---|---|
| **iPS:435807** | **21-225_181C10** | NA | AGCTATGGCATGCAC | ATTATATGGTATGATGGAAGTTATAAATACTATGCAGACTCCGTGAAGGGC | GATCATTACGATTTTTGGAGTGGGCACTTTGACTAC |
| | | | SEQ ID NO:6555 | SEQ ID NO:14567 | SEQ ID NO:22579 |
| | | AA | SYGMH | IIWYDGSYKYYADSVKG | DHYDFWSGHFDY |
| | | | SEQ ID NO:6556 | SEQ ID NO:14568 | SEQ ID NO:22580 |
| **iPS:435809** | **21-225_182H5** | NA | AGCTATGCCATGAGT | GTTATTAGTGGTAGAGGTGGTACCACATTCTACGCAGACTCCGTGAAGGGC | CGGACTGGGGATGATGTTTTTGATATC |
| | | | SEQ ID NO:6557 | SEQ ID NO:14569 | SEQ ID NO:22581 |
| | | AA | SYAMS | VISGRGGTTFYADSVKG | RTGDDVFDI |
| | | | SEQ ID NO:6558 | SEQ ID NO:14570 | SEQ ID NO:22582 |
| **iPS:435811** | **21-225_183H6** | NA | AGCTATGGCATGCAC | ATTATATCATATGCTGGAAGTACTAAATTCTATGCAGACTCCGTGAAGGGC | AGGCCCCCGCAGTGGCTGGTAGAGGGCTACGGTATGGACGTC |
| | | | SEQ ID NO:6559 | SEQ ID NO:14571 | SEQ ID NO:22583 |
| | | AA | SYGMH | IISYAGSTKFYADSVKG | RPPQWLVEGYGMDV |
| | | | SEQ ID NO:6560 | SEQ ID NO:14572 | SEQ ID NO:22584 |
| **iPS:435813** | **21-225_183A12** | NA | AGCTATGGCATGCAC | GTTATATCATCTGCTGGAAGTAATAAATACTATGCAGACTCCGTGAAGGGC | AGGTATAGCAGTGGCTGGGACTGGTTCGACCCC |
| | | | SEQ ID NO:6561 | SEQ ID NO:14573 | SEQ ID NO:22585 |
| | | AA | SYGMH | VISSAGSNKYYADSVKG | RYSSGWDWFDP |
| | | | SEQ ID NO:6562 | SEQ ID NO:14574 | SEQ ID NO:22586 |
| **iPS:435815** | **21-225_190G10** | NA | GACTATAGCATGAAC | TCTATTAGTAGTGGTAGTGGTTACATACACTACGCAGACTCAGTGAAGGGC | GCAACTATGGCCCTTGACTAC |
| | | | SEQ ID NO:6563 | SEQ ID NO:14575 | SEQ ID NO:22587 |
| | | AA | DYSMN | SISSGSGYIHYADSVKG | ATMALDY |

FIGURE 49

| | | | | | |
|---|---|---|---|---|---|
| | | | SEQ ID NO:6564 | SEQ ID NO:14576 | SEQ ID NO:22588 |
| **iPS:435817** | **21-225_190B11** | NA | AATTACTACTGGAGC | CGTATCTATACCAGTGGGAGCACCAACTACAACCCCTCCCTCAAGAGT | GATCGGGGATACTATGGCTACTACGGTATGGACGTC |
| | | | SEQ ID NO:6565 | SEQ ID NO:14577 | SEQ ID NO:22589 |
| | | AA | NYYWS | RIYTSGSTNYNPSLKS | DRGYYGYYGMDV |
| | | | SEQ ID NO:6566 | SEQ ID NO:14578 | SEQ ID NO:22590 |
| **iPS:435819** | **21-225_190C11** | NA | AGCTATGGCATGCAC | GTTATATGGTATGATGGAAGTAATAAAAACTATGCAGACTCCGTGAAGGGC | GATCAGGGCGTGGGCTACGACGGTTTGGACGTC |
| | | | SEQ ID NO:6567 | SEQ ID NO:14579 | SEQ ID NO:22591 |
| | | AA | SYGMH | VIWYDGSNKNYADSVKG | DQGVGYDGLDV |
| | | | SEQ ID NO:6568 | SEQ ID NO:14580 | SEQ ID NO:22592 |
| **iPS:435821** | **21-225_190E11** | NA | AACTATGGCATGCAC | ATTATATGGTTTGATGGAAGTAATAAATACTATGCAGACTCCGTGAAGGGC | GCCCAGGGGGTCTACTACTACGTTATGGACGTC |
| | | | SEQ ID NO:6569 | SEQ ID NO:14581 | SEQ ID NO:22593 |
| | | AA | NYGMH | IIWFDGSNKYYADSVKG | AQGVYYYVMDV |
| | | | SEQ ID NO:6570 | SEQ ID NO:14582 | SEQ ID NO:22594 |
| **iPS:435823** | **21-225_190F11** | NA | AGCTATGCCATGAAC | ACTATTAGTGGTACTGGTCGTAGGACATACTACGCAGACTCCGTGAAGGGC | GAGGAGGATTACTATGATAGTAGTGGCCCGGGGTTCGACCCC |
| | | | SEQ ID NO:6571 | SEQ ID NO:14583 | SEQ ID NO:22595 |
| | | AA | SYAMN | TISGTGRRTYYADSVKG | EEDYYDSSGPGFDP |
| | | | SEQ ID NO:6572 | SEQ ID NO:14584 | SEQ ID NO:22596 |

FIGURE 49

| | | | | | |
|---|---|---|---|---|---|
| **iPS:435825** | **21-225_190G11** | NA | ATTTATGGCATGCAC | GTTATATGGTATGATGGAAGTAATAAAAACTATGCAGACTCCGTGAAGGGC | GATCAGGGCGTGGGCTACGACGGTTTGGACGTC |
| | | | SEQ ID NO:6573 | SEQ ID NO:14585 | SEQ ID NO:22597 |
| | | AA | IYGMH | VIWYDGSNKNYADSVKG | DQGVGYDGLDV |
| | | | SEQ ID NO:6574 | SEQ ID NO:14586 | SEQ ID NO:22598 |
| **iPS:435827** | **21-225_190H11** | NA | AGCTACCACTGGAGC | CTTATCTATACCAGTAGGAGCACCATTTACAACCCCTCCCTCAAGAGT | CTCCGGTATAACTGGAACTTCCCTTACTTTGACTAC |
| | | | SEQ ID NO:6575 | SEQ ID NO:14587 | SEQ ID NO:22599 |
| | | AA | SYHWS | LIYTSRSTIYNPSLKS | LRYNWNFPYFDY |
| | | | SEQ ID NO:6576 | SEQ ID NO:14588 | SEQ ID NO:22600 |
| **iPS:435829** | **21-225_190B12** | NA | AGTGGTGGTTACTACTGGAAC | TATATCTATTACAGTGGGAGCACCTACTACAACCCGTCCCTCAAGAGT | TCCGGGTATAATTGGGACGCCGGGGTCGACCCC |
| | | | SEQ ID NO:6577 | SEQ ID NO:14589 | SEQ ID NO:22601 |
| | | AA | SGGYYWN | YIYYSGSTYYNPSLKS | SGYNWDAGVDP |
| | | | SEQ ID NO:6578 | SEQ ID NO:14590 | SEQ ID NO:22602 |
| **iPS:435831** | **21-225_190C12** | NA | AGCTATGGCATGCAC | GTTATATCATATGATGGAGGTTATAAAAACTATGTAGACTCCGTGAAGGGC | GGTACCCACGGGTACTACTACGGTGTGGACGTC |
| | | | SEQ ID NO:6579 | SEQ ID NO:14591 | SEQ ID NO:22603 |
| | | AA | SYGMH | VISYDGGYKNYVDSVKG | GTHGYYYGVDV |
| | | | SEQ ID NO:6580 | SEQ ID NO:14592 | SEQ ID NO:22604 |
| **iPS:435833** | **21-225_190D12** | NA | AGCTATGCCATGAGC | GCTATTATTGGTAATGGTGGTAGGACATACTACGCAGACTCCGTGAAGGGC | GATATGGGTAGATACAGCTATGGTTTCTTTGACTAC |
| | | | SEQ ID NO:6581 | SEQ ID NO:14593 | SEQ ID NO:22605 |

FIGURE 49

| | | | | | |
|---|---|---|---|---|---|
| | | AA | SYAMS | AIIGNGGRTYYADSVKG | DMGRYSYGFFDY |
| | | | SEQ ID NO:6582 | SEQ ID NO:14594 | SEQ ID NO:22606 |
| **iPS:435835** | **21-225_190F12** | NA | AACTATGGCATGCAC | GTTATATGGTTTGATGGAAGTAATGACTACTATGCAGACTCCGTGAAGGGC | GATAGAAGCGTCGGCTACGACGGTTTAGATGTC |
| | | | SEQ ID NO:6583 | SEQ ID NO:14595 | SEQ ID NO:22607 |
| | | AA | NYGMH | VIWFDGSNDYYADSVKG | DRSVGYDGLDV |
| | | | SEQ ID NO:6584 | SEQ ID NO:14596 | SEQ ID NO:22608 |
| **iPS:435837** | **21-225_198G3** | NA | ACCTATGGCATGCAC | GTTATATGGTATGATGGAACTAATAAAAACTATGCAGACTCCGTGAAGGGC | GATCAGGGCGTGGGCTACGACGGTTTGGACGTC |
| | | | SEQ ID NO:6585 | SEQ ID NO:14597 | SEQ ID NO:22609 |
| | | AA | TYGMH | VIWYDGTNKNYADSVKG | DQGVGYDGLDV |
| | | | SEQ ID NO:6586 | SEQ ID NO:14598 | SEQ ID NO:22610 |
| **iPS:435839** | **21-225_191B1** | NA | AGTTATCACTGGAGC | CATATCTATACCAGTGGGAGCACCAAGTACAACCCCTCCCTCAAGAGT | CTCCGGTATAACTGGAACTTCCCTTTCTTTGACTAT |
| | | | SEQ ID NO:6587 | SEQ ID NO:14599 | SEQ ID NO:22611 |
| | | AA | SYHWS | HIYTSGSTKYNPSLKS | LRYNWNFPFFDY |
| | | | SEQ ID NO:6588 | SEQ ID NO:14600 | SEQ ID NO:22612 |
| **iPS:435841** | **21-225_191D8** | NA | AATTATGATATCAAC | TGGATGCACCCTAACAGTGGTAACACAGGCTATGCACAGAAGTTCCAGGGC | AGCAGTGGCTGGTACTACTTTGACTAC |
| | | | SEQ ID NO:6589 | SEQ ID NO:14601 | SEQ ID NO:22613 |
| | | AA | NYDIN | WMHPNSGNTGYAQKFQG | SSGWYYFDY |
| | | | SEQ ID NO:6590 | SEQ ID NO:14602 | SEQ ID NO:22614 |

FIGURE 49

| | | | | | |
|---|---|---|---|---|---|
| **iPS:435843** | **21-225_191F1** | NA | AGTGGTGGTTACTACTGGAAC | TACATCTTTTACAGTGGGAGCACCTACTACAACCCGTCCCTCAAGAGT | GGGGATTACGATGGTTCGGGGAGTTATCACTACTATTACGGTATGGACGTC |
| | | | SEQ ID NO:6591 | SEQ ID NO:14603 | SEQ ID NO:22615 |
| | | AA | SGGYYWN | YIFYSGSTYYNPSLKS | GDYDGSGSYHYYYGMDV |
| | | | SEQ ID NO:6592 | SEQ ID NO:14604 | SEQ ID NO:22616 |
| **iPS:435845** | **21-225_191G1** | NA | AGCTATGGCATGCAC | GTTATATGGTATGATGGAAGTAATGAACACTATGCAGACTCCGTGAAGGGC | GATAGGGGGGTGGGTTACTACGGTTTGGACGTC |
| | | | SEQ ID NO:6593 | SEQ ID NO:14605 | SEQ ID NO:22617 |
| | | AA | SYGMH | VIWYDGSNEHYADSVKG | DRGVGYYGLDV |
| | | | SEQ ID NO:6594 | SEQ ID NO:14606 | SEQ ID NO:22618 |
| **iPS:435847** | **21-225_191A3** | NA | AGTGGTGATTACTACTGGAAC | TACATCTTTTACAGTGGGAGCACCTACTACAACCCGTCCCTCAAGAGT | GGGGATTACGATGGTTCGGGGAGTTATCACTACTACTACGGTATGGACGTC |
| | | | SEQ ID NO:6595 | SEQ ID NO:14607 | SEQ ID NO:22619 |
| | | AA | SGDYYWN | YIFYSGSTYYNPSLKS | GDYDGSGSYHYYYGMDV |
| | | | SEQ ID NO:6596 | SEQ ID NO:14608 | SEQ ID NO:22620 |
| **iPS:435849** | **21-225_191C3** | NA | AGTGGTGATTACTACTGGAAC | TACATCTTTTACAGTGGGAGCACCTACTACAACCCGTCCCTCAAGAGT | GGGGATTACGATGGTTCGGGGAGTTATCACTTCTACTACGGTATGGACGTC |
| | | | SEQ ID NO:6597 | SEQ ID NO:14609 | SEQ ID NO:22621 |
| | | AA | SGDYYWN | YIFYSGSTYYNPSLKS | GDYDGSGSYHFYYGMDV |
| | | | SEQ ID NO:6598 | SEQ ID NO:14610 | SEQ ID NO:22622 |
| **iPS:435851** | **21-225_191D3** | NA | AGTGGTGATTACTACTGGAAC | TACATCTTTTACAGTGGGAGCACCTACTACAACCCGTCCCTCAAGAGT | GGGGATTACGATGGTTCGGGGAGTTATCACTACTATTACGGTATGGACGTC |
| | | | SEQ ID NO:6599 | SEQ ID NO:14611 | SEQ ID NO:22623 |

**FIGURE 49**

| | | | | | |
|---|---|---|---|---|---|
| | | AA | SGDYYWN | YIFYSGSTYYNPSLKS | GDYDGSGSYHYYYGMDV |
| | | | SEQ ID NO:6600 | SEQ ID NO:14612 | SEQ ID NO:22624 |
| **iPS:435853** | **21-225_191E3** | NA | AGCTACCACTGGAGC | CTTATCTATACCAGTAGGAGCACCAATTACAACCCCTCCCTCAAGAGT | CTCCGGTATAACTGGAACTTCCCTTACTTTGACTAC |
| | | | SEQ ID NO:6601 | SEQ ID NO:14613 | SEQ ID NO:22625 |
| | | AA | SYHWS | LIYTSRSTNYNPSLKS | LRYNWNFPYFDY |
| | | | SEQ ID NO:6602 | SEQ ID NO:14614 | SEQ ID NO:22626 |
| **iPS:435855** | **21-225_191G3** | NA | AATTATGATATCAAC | CGGATGAACCCTAACAGTGGTAACACAGGCTATGCACAGAAGTTCCAGGGC | AGCAGTGGCTGGTACATCTTTGACTAC |
| | | | SEQ ID NO:6603 | SEQ ID NO:14615 | SEQ ID NO:22627 |
| | | AA | NYDIN | RMNPNSGNTGYAQKFQG | SSGWYIFDY |
| | | | SEQ ID NO:6604 | SEQ ID NO:14616 | SEQ ID NO:22628 |
| **iPS:435857** | **21-225_191A4** | NA | AGCTATGGCATGCAC | GTTATATCATATGATGGAGGTTATAAAAACTATGCAGACTCCGTGAAGGGC | GGTACCCACGGGTACTACTACGGTGTGGACGTC |
| | | | SEQ ID NO:6605 | SEQ ID NO:14617 | SEQ ID NO:22629 |
| | | AA | SYGMH | VISYDGGYKNYADSVKG | GTHGYYYGVDV |
| | | | SEQ ID NO:6606 | SEQ ID NO:14618 | SEQ ID NO:22630 |
| **iPS:435859** | **21-225_190E6** | NA | AGCTATGGCATGCAC | GTTATATGGTATGATGGAAGTAATAAAAACTATGCAGACTCCGTGAAGGGC | GATCAGGGCGTGGGCTACGACGGTTTGGACGTC |
| | | | SEQ ID NO:6607 | SEQ ID NO:14619 | SEQ ID NO:22631 |
| | | AA | SYGMH | VIWYDGSNKNYADSVKG | DQGVGYDGLDV |
| | | | SEQ ID NO:6608 | SEQ ID NO:14620 | SEQ ID NO:22632 |

**FIGURE 49**

| | | | | | |
|---|---|---|---|---|---|
| **iPS:435861** | **21-225_190A5** | NA | AGTTATGGCATGCAC | GTTATATGGTATGATGGAAGTAATAAAAACTATGCAGACTCCGTGAAGGGC | GATTTCTCTGTAGGGTACGACGGTATGGACGTC |
| | | | SEQ ID NO:6609 | SEQ ID NO:14621 | SEQ ID NO:22633 |
| | | AA | SYGMH | VIWYDGSNKNYADSVKG | DFSVGYDGMDV |
| | | | SEQ ID NO:6610 | SEQ ID NO:14622 | SEQ ID NO:22634 |
| **iPS:435863** | **21-225_191H4** | NA | AGTGGTGGTTACTACTGGAAC | TACATCTTTTACAGTGGGAGCACCTACTACAACCCGTCCCTCAGGAGT | TCCGGGTATAACTGGGACAACGGGGTCGACCCC |
| | | | SEQ ID NO:6611 | SEQ ID NO:14623 | SEQ ID NO:22635 |
| | | AA | SGGYYWN | YIFYSGSTYYNPSLRS | SGYNWDNGVDP |
| | | | SEQ ID NO:6612 | SEQ ID NO:14624 | SEQ ID NO:22636 |
| **iPS:435865** | **21-225_191A5** | NA | GACTATAGCATGAAC | TCCATTAGTAGTGGTAGTGGTTACATATATTATGCAGACTCAGTGAAGGGC | GCTACTATGGCCCTTGACTAC |
| | | | SEQ ID NO:6613 | SEQ ID NO:14625 | SEQ ID NO:22637 |
| | | AA | DYSMN | SISSGSGYIYYADSVKG | ATMALDY |
| | | | SEQ ID NO:6614 | SEQ ID NO:14626 | SEQ ID NO:22638 |
| **iPS:435867** | **21-225_191E5** | NA | AGCTATGCCATGAAC | ACTATTAGTGGTACTGGTCGTAGGACATACTACGCAGACTCCGTGAAGGGC | GAGGAGGATTACTATGATAGTAGTGGCCCGGGGTTCGACCCC |
| | | | SEQ ID NO:6615 | SEQ ID NO:14627 | SEQ ID NO:22639 |
| | | AA | SYAMN | TISGTGRRTYYADSVKG | EEDYYDSSGPGFDP |
| | | | SEQ ID NO:6616 | SEQ ID NO:14628 | SEQ ID NO:22640 |
| **iPS:435869** | **21 225 190B1** | NA | AGCTATGGCATGCAC | GTTATATGGTATGATGGAAGTAATAAACATTATGCAGACTCCGTGAAGGGC | GATAGAACAGTGGGATACTCCGGTATGGACGTC |

FIGURE 49

| | | | | | |
|---|---|---|---|---|---|
| | 21-225_190B1 | | SEQ ID NO:6617 | SEQ ID NO:14629 | SEQ ID NO:22641 |
| | | AA | SYGMH | VIWYDGSNKHYADSVKG | DRTVGYSGMDV |
| | | | SEQ ID NO:6618 | SEQ ID NO:14630 | SEQ ID NO:22642 |
| iPS:435871 | 21-225_191E6 | NA | AGCTACCACTGGAGC | CTTATCTATACCAGTAGG AGCACCAATTACAACCC CTCCCTCAAGAGT | CTCCGGTATAACTGGAACTT CCCTTACTTTGACTTC |
| | | | SEQ ID NO:6619 | SEQ ID NO:14631 | SEQ ID NO:22643 |
| | | AA | SYHWS | LIYTSRSTNYNPSLKS | LRYNWNFPYFDF |
| | | | SEQ ID NO:6620 | SEQ ID NO:14632 | SEQ ID NO:22644 |
| iPS:435873 | 21-225_190G4 | NA | AGCTATGGCATGCAC | GTTATATGGTATGATGG AAGTAATAAAAACTACG CAGACTCCGTGAAGGGC | GATCAGGGCGTGGGCTACGA CGGTTTGGACGTC |
| | | | SEQ ID NO:6621 | SEQ ID NO:14633 | SEQ ID NO:22645 |
| | | AA | SYGMH | VIWYDGSNKNYADSVKG | DQGVGYDGLDV |
| | | | SEQ ID NO:6622 | SEQ ID NO:14634 | SEQ ID NO:22646 |
| iPS:435875 | 21-225_190B9 | NA | ACCTATGCCATGAGT | GCTATTAGTCGTAGTGGT GGTAACACACACTACGC AGACTCCGTGAAGGGC | GATGGATTCGGTGGGAGCTC CTACTTTGACTAC |
| | | | SEQ ID NO:6623 | SEQ ID NO:14635 | SEQ ID NO:22647 |
| | | AA | TYAMS | AISRSGGNTHYADSVKG | DGFGGSSYFDY |
| | | | SEQ ID NO:6624 | SEQ ID NO:14636 | SEQ ID NO:22648 |
| iPS:435877 | 21-225_184E7 | NA | AGCTACAATATGCAC | TGGATCAACCCTAACAA TGGTGGCTCAAACTATA CACAGAAGTTTCAGGGC | AAGTTTGGGGAC |
| | | | SEQ ID NO:6625 | SEQ ID NO:14637 | SEQ ID NO:22649 |
| | | AA | SYNMH | WINPNNGGSNYTQKFQG | KFGD |

FIGURE 49

| | | | | | |
|---|---|---|---|---|---|
| | | | SEQ ID NO:6626 | SEQ ID NO:14638 | SEQ ID NO:22650 |
| **iPS:435879** | **21-225_184H10** | NA | GACTATGGCATGCAC | GTTATTTGGTATGATGAAACTAATAAACACTATGGAGACTCCGTGAAGGGC | GAGGTTGGCTGGCACGATGACTAT |
| | | | SEQ ID NO:6627 | SEQ ID NO:14639 | SEQ ID NO:22651 |
| | | AA | DYGMH | VIWYDETNKHYGDSVKG | EVGWHDDY |
| | | | SEQ ID NO:6628 | SEQ ID NO:14640 | SEQ ID NO:22652 |
| **iPS:435881** | **21-225_184D11** | NA | GACTATGGCATGCAC | GTTATTTGGTATGATGAAACTAATAAACACTATGGAGACTCCGTGAAGGGC | GAGGTTGGCTGGCACGATGACTAT |
| | | | SEQ ID NO:6629 | SEQ ID NO:14641 | SEQ ID NO:22653 |
| | | AA | DYGMH | VIWYDETNKHYGDSVKG | EVGWHDDY |
| | | | SEQ ID NO:6630 | SEQ ID NO:14642 | SEQ ID NO:22654 |
| **iPS:435883** | **21-225_185A1** | NA | AGCTATAGCATGAAC | TCCATTAGCAGTAGTGGTAGTTACATATATTACGCAGACTCAGTGAAGGGC | AGCAACCTTTTTGACTGC |
| | | | SEQ ID NO:6631 | SEQ ID NO:14643 | SEQ ID NO:22655 |
| | | AA | SYSMN | SISSSGSYIYYADSVKG | SNLFDC |
| | | | SEQ ID NO:6632 | SEQ ID NO:14644 | SEQ ID NO:22656 |
| **iPS:435885** | **21-225_185E10** | NA | AGCTACAATATGCAC | TGGATCAACCCTAACAATGGTGGCTCAAACTATACACAGAAGTTTCAGGGC | AAGTTTGGGGAC |
| | | | SEQ ID NO:6633 | SEQ ID NO:14645 | SEQ ID NO:22657 |
| | | AA | SYNMH | WINPNNGGSNYTQKFQG | KFGD |
| | | | SEQ ID NO:6634 | SEQ ID NO:14646 | SEQ ID NO:22658 |

**FIGURE 49**

| | | | | | |
|---|---|---|---|---|---|
| **iPS:435887** | **21-225_186F7** | NA | ACCTATGGCATGCAC | ATTATATGGTATGATGGAAGTTATAAATATTATGCAGACTCCGTGAAGGGC | GATCATTACGATTTTTGGAGTGGGCACTTTGACTAC |
| | | | SEQ ID NO:6635 | SEQ ID NO:14647 | SEQ ID NO:22659 |
| | | AA | TYGMH | IIWYDGSYKYYADSVKG | DHYDFWSGHFDY |
| | | | SEQ ID NO:6636 | SEQ ID NO:14648 | SEQ ID NO:22660 |
| **iPS:435889** | **21-225_186A11** | NA | AGCTATGCCATGAGT | GTTATTAGTGGTAGAGGTGGTACCACATTCTACGCAGACTCCGTGAAGGGC | CGGACTGGGGATGATGTTTTTGATATC |
| | | | SEQ ID NO:6637 | SEQ ID NO:14649 | SEQ ID NO:22661 |
| | | AA | SYAMS | VISGRGGTTFYADSVKG | RTGDDVFDI |
| | | | SEQ ID NO:6638 | SEQ ID NO:14650 | SEQ ID NO:22662 |
| **iPS:435891** | **21-225_188H5** | NA | AGCTACAATATGCAC | TGGATCAACCCTAACAGTGGTGGCTCAAACTATACACAGAAGTTTCAGGGC | AAGTTTGGGGAC |
| | | | SEQ ID NO:6639 | SEQ ID NO:14651 | SEQ ID NO:22663 |
| | | AA | SYNMH | WINPNSGGSNYTQKFQG | KFGD |
| | | | SEQ ID NO:6640 | SEQ ID NO:14652 | SEQ ID NO:22664 |
| **iPS:435895** | **21-225_188E8** | NA | AGCTCTGCCATGAAC | GTTATTAGTGGTAGTGGTGGTTACACATACTACGCAGACTCCGTGAAGGGC | AGGAACACCGATGATGCTTTTGATATC |
| | | | SEQ ID NO:6641 | SEQ ID NO:14653 | SEQ ID NO:22665 |
| | | AA | SSAMN | VISGSGGYTYYADSVKG | RNTDDAFDI |
| | | | SEQ ID NO:6642 | SEQ ID NO:14654 | SEQ ID NO:22666 |
| **iPS:435897** | **21-225_188B9** | NA | AGCTACAATATGCAC | TGGATCAACCCTAACAGTGGTGGCTCAAACTATACACAGAAGTTTCAGGGC | AAGTTTGGGGAC |
| | | | SEQ ID NO:6643 | SEQ ID NO:14655 | SEQ ID NO:22667 |
| | | AA | SYNMH | WINPNSGGSNYTQKFQG | KFGD |
| | | | SEQ ID NO:6644 | SEQ ID NO:14656 | SEQ ID NO:22668 |

**FIGURE 49**

| | | | | | |
|---|---|---|---|---|---|
| **iPS:435899** | **21-225_188G11** | NA | AGTTATGGCATGCAC | ATTATATGGTATGATGGAAGTTATAAATACTATGCAGACTCCGTGAAGGGC | GAGAGATACGATTTTTGGAGTGGTCATTTTGACTAC |
| | | | SEQ ID NO:6645 | SEQ ID NO:14657 | SEQ ID NO:22669 |
| | | AA | SYGMH | IIWYDGSYKYYADSVKG | ERYDFWSGHFDY |
| | | | SEQ ID NO:6646 | SEQ ID NO:14658 | SEQ ID NO:22670 |
| **iPS:435901** | **21-225_189G2** | NA | AACTATGGCATGCAC | ATTATATGGTATGATGGAAGTTATAAATACTATGCAGACTCCGTGAAGGGC | GATCGATTCGATTTTTGGAGTGGTTATTCCGACTAC |
| | | | SEQ ID NO:6647 | SEQ ID NO:14659 | SEQ ID NO:22671 |
| | | AA | NYGMH | IIWYDGSYKYYADSVKG | DRFDFWSGYSDY |
| | | | SEQ ID NO:6648 | SEQ ID NO:14660 | SEQ ID NO:22672 |
| **iPS:435903** | **21-225_190E2** | NA | GACTACTACATGAGC | TACATTAGTAGTAGTGGTACTACCGTATTCTACGCAGACTCTGTGAAGGGC | GAATGGGTGGGAGCCGACTAC |
| | | | SEQ ID NO:6649 | SEQ ID NO:14661 | SEQ ID NO:22673 |
| | | AA | DYYMS | YISSSGTTVFYADSVKG | EWVGADY |
| | | | SEQ ID NO:6650 | SEQ ID NO:14662 | SEQ ID NO:22674 |
| **iPS:435905** | **21-225_190A3** | NA | AGTGGTGGTTACTACTGGAAC | TTCATCTTTTATAGTGGGAGCACCTACTACAACCCGTCCCTCAAGAGT | GGGGATTACGATGGTTCGGGGAGTTATCACTACTATTACGGTATGGACGTC |
| | | | SEQ ID NO:6651 | SEQ ID NO:14663 | SEQ ID NO:22675 |
| | | AA | SGGYYWN | FIFYSGSTYYNPSLKS | GDYDGSGSYHYYYGMDV |
| | | | SEQ ID NO:6652 | SEQ ID NO:14664 | SEQ ID NO:22676 |
| **iPS:435907** | **21-225_190G3** | NA | AGCTATGGCATGCAC | GTTATATCATATGATGGAGGTTATAAAAACTATGTAGACTCCGTGAAGGGC | GGTACCCACGGGTACTACTACGGTGTGGACGTC |
| | | | SEQ ID NO:6653 | SEQ ID NO:14665 | SEQ ID NO:22677 |
| | | AA | SYGMH | VISYDGGYKNYVDSVKG | GTHGYYYGVDV |
| | | | SEQ ID NO:6654 | SEQ ID NO:14666 | SEQ ID NO:22678 |

**FIGURE 49**

| | | | | | |
|---|---|---|---|---|---|
| **iPS:435909** | **21-225_190H3** | NA | AGCTATGCCATGAGC | GCTATTAGTGGTCGTGGTGGTAACACATACTACGCAGACTCCGTGAAGGGC | GATGGATTCGGTGGGAGCTCCTATTTTGACTAC |
| | | | SEQ ID NO:6655 | SEQ ID NO:14667 | SEQ ID NO:22679 |
| | | AA | SYAMS | AISGRGGNTYYADSVKG | DGFGGSSYFDY |
| | | | SEQ ID NO:6656 | SEQ ID NO:14668 | SEQ ID NO:22680 |
| **iPS:435911** | **21-225_190B4** | NA | AGTGGTGATTACTACTGGAAC | TACATCTTTTACAGTGGGAGCACCTACTACAACCCGTCCCTCAAGAGT | GGGGATTACGATGGTTCGGGGAGTTATCACTACTACTACGGTATGGACGTC |
| | | | SEQ ID NO:6657 | SEQ ID NO:14669 | SEQ ID NO:22681 |
| | | AA | SGDYYWN | YIFYSGSTYYNPSLKS | GDYDGSGSYHYYYGMDV |
| | | | SEQ ID NO:6658 | SEQ ID NO:14670 | SEQ ID NO:22682 |
| **iPS:435913** | **21-225_190A7** | NA | AGTGGTGTTTACTACTGGAGC | AACATCTATTACAGTGGGAGCACCTACAACAACCCGTCCCTCAAGAGT | GGGGATTACGATGGTTCGGGGAGTTATCACTACTACTACGGTTTGGACGTC |
| | | | SEQ ID NO:6659 | SEQ ID NO:14671 | SEQ ID NO:22683 |
| | | AA | SGVYYWS | NIYYSGSTYNNPSLKS | GDYDGSGSYHYYYGLDV |
| | | | SEQ ID NO:6660 | SEQ ID NO:14672 | SEQ ID NO:22684 |
| **iPS:435915** | **21-225_190H4** | NA | AATTATGATATCAAC | TGGATGAACCCTAACAGTGGTAACACAGGCTATGCACAGAAGTTCCAGGGC | AGCAGTGGCTGGTACATCTTTGACTAC |
| | | | SEQ ID NO:6661 | SEQ ID NO:14673 | SEQ ID NO:22685 |
| | | AA | NYDIN | WMNPNSGNTGYAQKFQG | SSGWYIFDY |
| | | | SEQ ID NO:6662 | SEQ ID NO:14674 | SEQ ID NO:22686 |
| **iPS:435917** | **21-225_190D5** | NA | AATTACTACTGGAGC | CGTATCTATGCCAGTGGGAGCACCAACTACAACCCCTCCCTCAAGAGT | GATCGGGGATACTATGGCTACTACGGTATGGACGTC |
| | | | SEQ ID NO:6663 | SEQ ID NO:14675 | SEQ ID NO:22687 |
| | | AA | NYYWS | RIYASGSTNYNPSLKS | DRGYYGYYGMDV |

**FIGURE 49**

| | | | | | |
|---|---|---|---|---|---|
| | | | SEQ ID NO:6664 | SEQ ID NO:14676 | SEQ ID NO:22688 |
| **iPS:435919** | **21-225_190H5** | NA | AGCTATGGCATGCAC | GTTATATCATATGATGGAGGTTATAAAAACTATGCAGACTCCGTGAAGGGC | GGTACCCACGGGTACTACTACGGTGTGGACGTC |
| | | | SEQ ID NO:6665 | SEQ ID NO:14677 | SEQ ID NO:22689 |
| | | AA | SYGMH | VISYDGGYKNYADSVKG | GTHGYYYGVDV |
| | | | SEQ ID NO:6666 | SEQ ID NO:14678 | SEQ ID NO:22690 |
| **iPS:435921** | **21-225_190D6** | NA | AGCTTTATCATGCAC | GTTATATGGTATGATGGAAGTAATAAATACTATGCAGACTCCGTGAAGGGC | GAGGAGTATAGCAGTGGCTGGTTCGGGTACGGTATGGACGTC |
| | | | SEQ ID NO:6667 | SEQ ID NO:14679 | SEQ ID NO:22691 |
| | | AA | SFIMH | VIWYDGSNKYYADSVKG | EEYSSGWFGYGMDV |
| | | | SEQ ID NO:6668 | SEQ ID NO:14680 | SEQ ID NO:22692 |
| **iPS:435923** | **21-225_190H6** | NA | GACTACTACATGAGC | TACATTAGTAGTAGTGGTACTACCGTATTCTACGCAGACTCTGTGAAGGGC | GAATGGGTGGGAGCCGACTAC |
| | | | SEQ ID NO:6669 | SEQ ID NO:14681 | SEQ ID NO:22693 |
| | | AA | DYYMS | YISSSGTTVFYADSVKG | EWVGADY |
| | | | SEQ ID NO:6670 | SEQ ID NO:14682 | SEQ ID NO:22694 |
| **iPS:435925** | **21-225_190D7** | NA | AATTATGATATCAAC | TGGATGAACCCTAATAGTGGTAATACAGGCTATGCACAGAAGTTCCAGGGC | AGCAGTGGCTGGTACTTCTTTGACTAC |
| | | | SEQ ID NO:6671 | SEQ ID NO:14683 | SEQ ID NO:22695 |
| | | AA | NYDIN | WMNPNSGNTGYAQKFQG | SSGWYFFDY |
| | | | SEQ ID NO:6672 | SEQ ID NO:14684 | SEQ ID NO:22696 |

FIGURE 49

| | | | | | |
|---|---|---|---|---|---|
| **iPS:435927** | **21-225_190E7** | NA | AGTTACCACTGGAGT | CATATCTATACCAGTAGGAGCACCAACTACAACCCCTCCCTCAAGAGT | CTCCGGTATAACTGGAACTTCCCTTACTTTGACTAC |
| | | | SEQ ID NO:6673 | SEQ ID NO:14685 | SEQ ID NO:22697 |
| | | AA | SYHWS | HIYTSRSTNYNPSLKS | LRYNWNFPYFDY |
| | | | SEQ ID NO:6674 | SEQ ID NO:14686 | SEQ ID NO:22698 |
| **iPS:435929** | **21-225_190D9** | NA | AGCTATGCCATGAGT | ACTATTAGTGGTACTGGTCGTAGGACATACTACGCAGACTCCGTGAAGGGC | GAGGAGGATTACTATGATAGTAGTGGCCCGGGGTTCGACCCC |
| | | | SEQ ID NO:6675 | SEQ ID NO:14687 | SEQ ID NO:22699 |
| | | AA | SYAMS | TISGTGRRTYYADSVKG | EEDYYDSSGPGFDP |
| | | | SEQ ID NO:6676 | SEQ ID NO:14688 | SEQ ID NO:22700 |
| **iPS:435933** | **21-225_190F8** | NA | ACTTATGGCATGCAC | GTTATATGGTATGATGGAAGTAATAAAAACTATGCAGACTCCGTGAAGGGC | GATCAGGGCGTGGGCTACGACGGTTTGGACGTC |
| | | | SEQ ID NO:6677 | SEQ ID NO:14689 | SEQ ID NO:22701 |
| | | AA | TYGMH | VIWYDGSNKNYADSVKG | DQGVGYDGLDV |
| | | | SEQ ID NO:6678 | SEQ ID NO:14690 | SEQ ID NO:22702 |
| **iPS:435935** | **21-225_190H8** | NA | AGCTATGCCATGAGC | ACTATTAGTGGTACTGGTCGTAGGACATATTACGCAGACTCCGTGAAGGGC | GAGGAGGATTACTATGATAGTAGTGGCCCGGGGTTCGACCCC |
| | | | SEQ ID NO:6679 | SEQ ID NO:14691 | SEQ ID NO:22703 |
| | | AA | SYAMS | TISGTGRRTYYADSVKG | EEDYYDSSGPGFDP |
| | | | SEQ ID NO:6680 | SEQ ID NO:14692 | SEQ ID NO:22704 |
| **iPS:435937** | **21-225_190H9** | NA | AACTATGGCATGCAC | GTTATATGGTTTGATGGAAGTAATGACTACTATGCAGACTCCGTGAAGGGC | GATAGAAGCGTCGGCTACGACGGTTTAGATGTC |
| | | | SEQ ID NO:6681 | SEQ ID NO:14693 | SEQ ID NO:22705 |

**FIGURE 49**

| | | | | | |
|---|---|---|---|---|---|
| | | AA | NYGMH | VIWFDGSNDYYADSVKG | DRSVGYDGLDV |
| | | | SEQ ID NO:6682 | SEQ ID NO:14694 | SEQ ID NO:22706 |
| **iPS:435939** | **21-225_191H7** | NA | AGTGGTGATTACTACTGGAA C | TACATCTTTTACAGTGGG AGCACCTACTACAACCC GTCCCTCAAGAGT | GGGGATTACGATGGTTCGGG GAGTTATCACTACTATTACG GTATGGACGTC |
| | | | SEQ ID NO:6683 | SEQ ID NO:14695 | SEQ ID NO:22707 |
| | | AA | SGDYYWN | YIFYSGSTYYNPSLKS | GDYDGSGSYHYYYGMDV |
| | | | SEQ ID NO:6684 | SEQ ID NO:14696 | SEQ ID NO:22708 |
| **iPS:435941** | **21-225_191E8** | NA | AACTATGGCATGCAC | ATTATATGGTTTGATGGA AGTAATCAATACTATGC CGACTCCGTGAAGGGC | GCCCACGGGGTCTACTACTA CGCTATGGACGTC |
| | | | SEQ ID NO:6685 | SEQ ID NO:14697 | SEQ ID NO:22709 |
| | | AA | NYGMH | IIWFDGSNQYYADSVKG | AHGVYYYAMDV |
| | | | SEQ ID NO:6686 | SEQ ID NO:14698 | SEQ ID NO:22710 |
| **iPS:435943** | **21-225_191C9** | NA | AGTGGTGGTTACTACTGGAA C | TATATCTATTACAGTGGG AGCACCTACTACAACCC GTCCCTCAAGAGT | TCCGGGTATAATTGGGACGC CGGGGTCGACCCC |
| | | | SEQ ID NO:6687 | SEQ ID NO:14699 | SEQ ID NO:22711 |
| | | AA | SGGYYWN | YIYYSGSTYYNPSLKS | SGYNWDAGVDP |
| | | | SEQ ID NO:6688 | SEQ ID NO:14700 | SEQ ID NO:22712 |
| **iPS:435945** | **21-225_191A10** | NA | AGCTATGGCATGCAC | GTTATATGGTATGATGG AAGTAATAAAAACTACG CAGACTCCGTGAAGGGC | GATCAGGGCGTGGGCTACGA CGGTTTGGACGTC |
| | | | SEQ ID NO:6689 | SEQ ID NO:14701 | SEQ ID NO:22713 |
| | | AA | SYGMH | VIWYDGSNKNYADSVKG | DQGVGYDGLDV |
| | | | SEQ ID NO:6690 | SEQ ID NO:14702 | SEQ ID NO:22714 |

# FIGURE 49

| | | | | | |
|---|---|---|---|---|---|
| **iPS:435947** | **21-225_191E10** | NA | AGCTATGGCATGCAC | GTTATATGGTATGATGGAAGTAATAAAAACTACGCAGACTCCGTGAAGGGC | GATCAGGGCGTGGGCTACGACGGTTTGGACGTC |
| | | | SEQ ID NO:6691 | SEQ ID NO:14703 | SEQ ID NO:22715 |
| | | AA | SYGMH | VIWYDGSNKNYADSVKG | DQGVGYDGLDV |
| | | | SEQ ID NO:6692 | SEQ ID NO:14704 | SEQ ID NO:22716 |
| **iPS:435953** | **21-225_191B12** | NA | GACTACTACATGAGC | TACATTAGTAGTAGTGGTACTACCGTATTCTACGCAGACTCTGTGAAGGGC | GAATGGGTGGGAGCCGACTAC |
| | | | SEQ ID NO:6693 | SEQ ID NO:14705 | SEQ ID NO:22717 |
| | | AA | DYYMS | YISSSGTTVFYADSVKG | EWVGADY |
| | | | SEQ ID NO:6694 | SEQ ID NO:14706 | SEQ ID NO:22718 |
| **iPS:435957** | **21-225_191G12** | NA | AGCTATGGCATGCAC | GTTATATGGTATGATGGAAGTAATAAAAACTATGCAGACTCCGTGAAGGGC | GATCAGGGCGTGGGCTACGACGGTTTGGACGTC |
| | | | SEQ ID NO:6695 | SEQ ID NO:14707 | SEQ ID NO:22719 |
| | | AA | SYGMH | VIWYDGSNKNYADSVKG | DQGVGYDGLDV |
| | | | SEQ ID NO:6696 | SEQ ID NO:14708 | SEQ ID NO:22720 |
| **iPS:435961** | **21-225_192A2** | NA | AGCTATGGCATGCAC | GTTATATGGTATGATGGAAGTTATAAATACTATGCAGACTCCGTGAAGGGC | GAGGATTCCCCTTATAGTGGCTACGCCTTGGACTACTTCTACGGTATGGACGTC |
| | | | SEQ ID NO:6697 | SEQ ID NO:14709 | SEQ ID NO:22721 |
| | | AA | SYGMH | VIWYDGSYKYYADSVKG | EDSPYSGYALDYFYGMDV |
| | | | SEQ ID NO:6698 | SEQ ID NO:14710 | SEQ ID NO:22722 |

FIGURE 49

| | | | | | |
|---|---|---|---|---|---|
| **iPS:435963** | **21-225_192D2** | NA | AGCTATGGCATGCAC | GTTATATGGTATGATGGAAGTAATAAAAACTATGCAGACTCCGTGAAGGGC | GATCGTGGGGTTGGCTACTACGGTATGGACGTC |
| | | | SEQ ID NO:6699 | SEQ ID NO:14711 | SEQ ID NO:22723 |
| | | AA | SYGMH | VIWYDGSNKNYADSVKG | DRGVGYYGMDV |
| | | | SEQ ID NO:6700 | SEQ ID NO:14712 | SEQ ID NO:22724 |
| **iPS:435965** | **21-225_192H2** | NA | AGCTCTGCCATGAGC | GCCATTAGTGGTAGTGGTGGTAACACATTCTACGCAGACTCCGTGAAGGGC | CTCATAGCAGTAGTTGGGTCCCACTACTTTGACTAC |
| | | | SEQ ID NO:6701 | SEQ ID NO:14713 | SEQ ID NO:22725 |
| | | AA | SSAMS | AISGSGGNTFYADSVKG | LIAVVGSHYFDY |
| | | | SEQ ID NO:6702 | SEQ ID NO:14714 | SEQ ID NO:22726 |
| **iPS:435967** | **21-225_192B3** | NA | AGTGGTGATTACTACTGGAAC | TTCATCTTTTACAGTGGGAGCACCTACTACAACCCGTCCCTCAAGAGT | GGGGATTACGATGGTTCGGGGAGTTATCACCACTACTACGGTATGGACGTC |
| | | | SEQ ID NO:6703 | SEQ ID NO:14715 | SEQ ID NO:22727 |
| | | AA | SGDYYWN | FIFYSGSTYYNPSLKS | GDYDGSGSYHHYYGMDV |
| | | | SEQ ID NO:6704 | SEQ ID NO:14716 | SEQ ID NO:22728 |
| **iPS:435971** | **21-225_192D3** | NA | AGCTATGGCATGCAC | GTTATATGGTATGATGGAAGTAATGAACACTATGCAGACTCCGTGAAGGGC | GATAGGGGGGTGGGTTACTACGGTTTGGACGTC |
| | | | SEQ ID NO:6705 | SEQ ID NO:14717 | SEQ ID NO:22729 |
| | | AA | SYGMH | VIWYDGSNEHYADSVKG | DRGVGYYGLDV |
| | | | SEQ ID NO:6706 | SEQ ID NO:14718 | SEQ ID NO:22730 |

**FIGURE 49**

| | | | | | |
|---|---|---|---|---|---|
| **iPS:435973** | **21-225_192H3** | NA | AGTGTTAGTTACTACTGGAGC | AACCTCTATTACAGTGGGAGCACCTACTACAACCCGTCCCTCAGGAGT | GGGGATTACGATGGTTCGGGGAGTTATCACTACTACCACGGTATGGACGTC |
| | | | SEQ ID NO:6707 | SEQ ID NO:14719 | SEQ ID NO:22731 |
| | | AA | SVSYYWS | NLYYSGSTYYNPSLRS | GDYDGSGSYHYYHGMDV |
| | | | SEQ ID NO:6708 | SEQ ID NO:14720 | SEQ ID NO:22732 |
| **iPS:435977** | **21-225_192E4** | NA | AGTTATGGCATGCAC | GTTATATGGTATGATGGAAGTAACAAAAACTATGTAGACTCCGTGAGGGGC | GATAGAAGCGTCGGCTACGACGGTATGGACGTC |
| | | | SEQ ID NO:6709 | SEQ ID NO:14721 | SEQ ID NO:22733 |
| | | AA | SYGMH | VIWYDGSNKNYVDSVRG | DRSVGYDGMDV |
| | | | SEQ ID NO:6710 | SEQ ID NO:14722 | SEQ ID NO:22734 |
| **iPS:435979** | **21-225_192H4** | NA | AGCTATGGCATGCAC | GTTATATGGTATGATGGAAGCAATAAAAACTATGCAGACTCCGTGAAGGGC | GATCAAGGTGTGGGGTACTACGGTATGGACGTC |
| | | | SEQ ID NO:6711 | SEQ ID NO:14723 | SEQ ID NO:22735 |
| | | AA | SYGMH | VIWYDGSNKNYADSVKG | DQGVGYYGMDV |
| | | | SEQ ID NO:6712 | SEQ ID NO:14724 | SEQ ID NO:22736 |
| **iPS:435983** | **21-225_192E5** | NA | AATGGTGGATACTACTGGAGC | TACATCTTTTACAGCGGGAGCACCTACTACAACCCGTCCCTCAAGAGT | GCGGGATATAACTGGGACAACGGGTTTGACTAC |
| | | | SEQ ID NO:6713 | SEQ ID NO:14725 | SEQ ID NO:22737 |
| | | AA | NGGYYWS | YIFYSGSTYYNPSLKS | AGYNWDNGFDY |
| | | | SEQ ID NO:6714 | SEQ ID NO:14726 | SEQ ID NO:22738 |

FIGURE 49

| | | | | | |
|---|---|---|---|---|---|
| **iPS:435985** | **21-225_192F6** | NA | AGCTTTATCATGCAC | GTTATATGGTATGATGGAAGTAATAAATACTATGTAGACTCCGTGAAGGGC | GAGGAGTATAGTAGCGGCTGGTTCGGGTACGGTATGGACGTC |
| | | | SEQ ID NO:6715 | SEQ ID NO:14727 | SEQ ID NO:22739 |
| | | AA | SFIMH | VIWYDGSNKYYVDSVKG | EEYSSGWFGYGMDV |
| | | | SEQ ID NO:6716 | SEQ ID NO:14728 | SEQ ID NO:22740 |
| **iPS:435987** | **21-225_192G6** | NA | AGCTATGGCATGCAC | GTTTTATGGTATGATGGAACTAATAAAAACTATGCAGACTCCGTGAAGGGC | GATCAGGGCGTGGGCTACGACGGTTTGGACGTC |
| | | | SEQ ID NO:6717 | SEQ ID NO:14729 | SEQ ID NO:22741 |
| | | AA | SYGMH | VLWYDGTNKNYADSVKG | DQGVGYDGLDV |
| | | | SEQ ID NO:6718 | SEQ ID NO:14730 | SEQ ID NO:22742 |
| **iPS:435989** | **21-225_192F7** | NA | AGCTATGGCATGCAC | GTTATATCATATGATGGAGGTTATAAAAACTATGCAGACTCCGTGAAGGGC | GGTACCCACGGGTACTACTACGGTGTGGACGTC |
| | | | SEQ ID NO:6719 | SEQ ID NO:14731 | SEQ ID NO:22743 |
| | | AA | SYGMH | VISYDGGYKNYADSVKG | GTHGYYYGVDV |
| | | | SEQ ID NO:6720 | SEQ ID NO:14732 | SEQ ID NO:22744 |
| **iPS:435993** | **21-225_192C8** | NA | AGCTATGGCATGCAC | GTTATATGGTATGATGGAAGTAATGAACACTATGCAGACTCCGTGAAGGGC | GATAGGGGAGTGGGTTACTACGGTATGGACGTC |
| | | | SEQ ID NO:6721 | SEQ ID NO:14733 | SEQ ID NO:22745 |
| | | AA | SYGMH | VIWYDGSNEHYADSVKG | DRGVGYYGMDV |
| | | | SEQ ID NO:6722 | SEQ ID NO:14734 | SEQ ID NO:22746 |

FIGURE 49

| | | | | | |
|---|---|---|---|---|---|
| **iPS:435995** | **21-225_192F8** | NA | GGTTGCTACTGGAGC | GAAATCAATCAAAGTGGAAGGTCCAACTACAACCCGTCCCTCAAGAGT | GACTACGGTGTCTTTGACTAC |
| | | | SEQ ID NO:6723 | SEQ ID NO:14735 | SEQ ID NO:22747 |
| | | AA | GCYWS | EINQSGRSNYNPSLKS | DYGVFDY |
| | | | SEQ ID NO:6724 | SEQ ID NO:14736 | SEQ ID NO:22748 |
| **iPS:435997** | **21-225_192G8** | NA | AGCTATGGCATGCAC | GTTATATGGTATGATGGAAGTAATAAAAACTATGCAGACTCCGTGAAGGGC | GATCAGGGCGTGGGCTACGACGGTTTGGACGTC |
| | | | SEQ ID NO:6725 | SEQ ID NO:14737 | SEQ ID NO:22749 |
| | | AA | SYGMH | VIWYDGSNKNYADSVKG | DQGVGYDGLDV |
| | | | SEQ ID NO:6726 | SEQ ID NO:14738 | SEQ ID NO:22750 |
| **iPS:435999** | **21-225_192F9** | NA | AGCTACCACTGGAGC | CTTATCTATACCAGTAGGAGCACCAATTACAACCCCTCCCTCAAGAGT | CTCCGGTATAACTGGAACTTCCCTTACTTTGACTAC |
| | | | SEQ ID NO:6727 | SEQ ID NO:14739 | SEQ ID NO:22751 |
| | | AA | SYHWS | LIYTSRSTNYNPSLKS | LRYNWNFPYFDY |
| | | | SEQ ID NO:6728 | SEQ ID NO:14740 | SEQ ID NO:22752 |
| **iPS:436001** | **21-225_192C10** | NA | AACTATGGCATGCAC | GTTATATGGTTTGATGGAAGTAATGACTACTATGCAGACTCCGTGAAGGGC | GATAGAAGCGTCGGCTACGACGGTTTAGATGTC |
| | | | SEQ ID NO:6729 | SEQ ID NO:14741 | SEQ ID NO:22753 |
| | | AA | NYGMH | VIWFDGSNDYYADSVKG | DRSVGYDGLDV |
| | | | SEQ ID NO:6730 | SEQ ID NO:14742 | SEQ ID NO:22754 |
| **iPS:436003** | **21-225_192G10** | NA | AGCTATGCCATGAGC | GCTATTAGTGGTAGAGGCGGTAGTACATTCTACGCAGACTCCGTGAAGGGC | CGTTTAGCACTGGATGGCTATGATGCTTTTGATATC |
| | | | SEQ ID NO:6731 | SEQ ID NO:14743 | SEQ ID NO:22755 |

FIGURE 49

| | | | | | |
|---|---|---|---|---|---|
| | | AA | SYAMS | AISGRGGSTFYADSVKG | RLALDGYDAFDI |
| | | | SEQ ID NO:6732 | SEQ ID NO:14744 | SEQ ID NO:22756 |
| **iPS:436005** | **21-225_192H10** | NA | AGCTATGGCATGCAC | GTTATATGGTATGATGGAAGTAATAAAAACTACGCAGACTCCGTGAAGGGC | GATCAGGGCGTGGGCTACGACGGTTTGGACGTC |
| | | | SEQ ID NO:6733 | SEQ ID NO:14745 | SEQ ID NO:22757 |
| | | AA | SYGMH | VIWYDGSNKNYADSVKG | DQGVGYDGLDV |
| | | | SEQ ID NO:6734 | SEQ ID NO:14746 | SEQ ID NO:22758 |
| **iPS:436007** | **21-225_192G12** | NA | AGTGGTGTTTACCACTGGAGC | AACATCCATTACAGCGGGAGCACCTACAACAACCCGTCCCTCAAGAGT | GGGGATTACGATGGTTCGGGGAGTTATCACTACTACTACGGTATGGACGTC |
| | | | SEQ ID NO:6735 | SEQ ID NO:14747 | SEQ ID NO:22759 |
| | | AA | SGVYHWS | NIHYSGSTYNNPSLKS | GDYDGSGSYHYYYGMDV |
| | | | SEQ ID NO:6736 | SEQ ID NO:14748 | SEQ ID NO:22760 |
| **iPS:436009** | **21-225_193A1** | NA | AGTGGTGTTTACTACTGGAGC | AACATCTATTACAGTGGGAGCACCTACAACAACCCGTCCCTCAAGAGT | GGGGATTACGATGGTTCGGGGAGTTATCACTACTACTACGGTATGGACGTC |
| | | | SEQ ID NO:6737 | SEQ ID NO:14749 | SEQ ID NO:22761 |
| | | AA | SGVYYWS | NIYYSGSTYNNPSLKS | GDYDGSGSYHYYYGMDV |
| | | | SEQ ID NO:6738 | SEQ ID NO:14750 | SEQ ID NO:22762 |
| **iPS:436011** | **21-225_193B1** | NA | AGTGGTGTTTACTACTGGAGC | AACATCTATTACAGTGGGAGCACCTACAACAACCCGTCCCTCAAGAGT | GGGGATTACGATGGTTCGGGGAGTTATCACTACTACTACGGTATGGACGTC |
| | | | SEQ ID NO:6739 | SEQ ID NO:14751 | SEQ ID NO:22763 |
| | | AA | SGVYYWS | NIYYSGSTYNNPSLKS | GDYDGSGSYHYYYGMDV |
| | | | SEQ ID NO:6740 | SEQ ID NO:14752 | SEQ ID NO:22764 |

**FIGURE 49**

| | | | | | |
|---|---|---|---|---|---|
| **iPS:436013** | **21-225_193F2** | NA | ACCTTTGCCATGAGT | GCTATTAGTCGTAGTGGTGGTAACACACACTACGCAGACTCCGTGAAGGGC | GATGGATTCGGTGGGAGCTCCTACTTTGACTAC |
| | | | SEQ ID NO:6741 | SEQ ID NO:14753 | SEQ ID NO:22765 |
| | | AA | TFAMS | AISRSGGNTHYADSVKG | DGFGGSSYFDY |
| | | | SEQ ID NO:6742 | SEQ ID NO:14754 | SEQ ID NO:22766 |
| **iPS:436015** | **21-225_193D3** | NA | AGTGGTGATTACTACTGGAAC | TACATCTTTTACAGTGGGAGCACCTACTACAACCCGTCCCTCAAGAGT | GGGGATTACGATGGTTCGGGGAGTTATCACTTCTACTACGGTATGGACGTC |
| | | | SEQ ID NO:6743 | SEQ ID NO:14755 | SEQ ID NO:22767 |
| | | AA | SGDYYWN | YIFYSGSTYYNPSLKS | GDYDGSGSYHFYYGMDV |
| | | | SEQ ID NO:6744 | SEQ ID NO:14756 | SEQ ID NO:22768 |
| **iPS:436017** | **21-225_193F3** | NA | AGTGGTGATTACTACTGGAAC | TACATCTTTTACAGTGGGAGCACCTACTACAACCCGTCCCTCAAGAGT | GGGGATTACGATGGTTCGGGGAGTTATCACTACTATTACGGTATGGACGTC |
| | | | SEQ ID NO:6745 | SEQ ID NO:14757 | SEQ ID NO:22769 |
| | | AA | SGDYYWN | YIFYSGSTYYNPSLKS | GDYDGSGSYHYYYGMDV |
| | | | SEQ ID NO:6746 | SEQ ID NO:14758 | SEQ ID NO:22770 |
| **iPS:436019** | **21-225_193C4** | NA | AGCTATGCCATGAAC | GCTATTATTGGTAATGGTGGTAGAACATACTACGCAGACTCCGTGAAGGGC | GATCTGGGTAGATACAGCTATGGTTTCTTTGACTAC |
| | | | SEQ ID NO:6747 | SEQ ID NO:14759 | SEQ ID NO:22771 |
| | | AA | SYAMN | AIIGNGGRTYYADSVKG | DLGRYSYGFFDY |
| | | | SEQ ID NO:6748 | SEQ ID NO:14760 | SEQ ID NO:22772 |
| **iPS:436021** | **21-225_193G4** | NA | AATTATGATATCAAC | TGGATGAACCCTAACAGTGGTAACACAGGCTATGCACAGAAGTTCCAGGGC | AGCAGTGGCTGGTACTTCTTTGACTAC |
| | | | SEQ ID NO:6749 | SEQ ID NO:14761 | SEQ ID NO:22773 |
| | | AA | NYDIN | WMNPNSGNTGYAQKFQG | SSGWYFFDY |

FIGURE 49

| | | | | | |
|---|---|---|---|---|---|
| | | | SEQ ID NO:6750 | SEQ ID NO:14762 | SEQ ID NO:22774 |
| **iPS:436023** | **21-225_193A5** | NA | AGTTATGATATCAAC | TGGATGAACCCTAAAAGGGGTAACACAGGCTATGCACAGAAGTTCCAGGGC | GGAGACCCGTATAACTGGAACTCCTACGCTATGGACGTC |
| | | | SEQ ID NO:6751 | SEQ ID NO:14763 | SEQ ID NO:22775 |
| | | AA | SYDIN | WMNPKRGNTGYAQKFQG | GDPYNWNSYAMDV |
| | | | SEQ ID NO:6752 | SEQ ID NO:14764 | SEQ ID NO:22776 |
| **iPS:436025** | **21-225_193B5** | NA | AGTGGTGGTTACTACTGGAGC | TACATCTATTACAGTGGGAGCACCTACTACAACCCGTCCCTCAAGAGT | GGAGAGTATAACTGGAACCACGGTATGGACGTC |
| | | | SEQ ID NO:6753 | SEQ ID NO:14765 | SEQ ID NO:22777 |
| | | AA | SGGYYWS | YIYYSGSTYYNPSLKS | GEYNWNHGMDV |
| | | | SEQ ID NO:6754 | SEQ ID NO:14766 | SEQ ID NO:22778 |
| **iPS:436027** | **21-225_193E6** | NA | GGTCCCTACTGGAGT | GAATCCAATCATAGTGGACGCACCAACTACAACCCGTCCCTCAAGAGT | GACTACGGTGGTTTGGACTAC |
| | | | SEQ ID NO:6755 | SEQ ID NO:14767 | SEQ ID NO:22779 |
| | | AA | GPYWS | ESNHSGRTNYNPSLKS | DYGGLDY |
| | | | SEQ ID NO:6756 | SEQ ID NO:14768 | SEQ ID NO:22780 |
| **iPS:436029** | **21-225_193H6** | NA | AGTGGTGATTACTACTGGAAC | TACATCTTTTACAGTGGGAGCACCTACTACAACCCGTCCCTCAAGAGT | GGGGATTACGATGGTTCGGGGAGTTATCACTACTATTACGGTATGGACGTC |
| | | | SEQ ID NO:6757 | SEQ ID NO:14769 | SEQ ID NO:22781 |
| | | AA | SGDYYWN | YIFYSGSTYYNPSLKS | GDYDGSGSYHYYYGMDV |
| | | | SEQ ID NO:6758 | SEQ ID NO:14770 | SEQ ID NO:22782 |

FIGURE 49

| | | | | | |
|---|---|---|---|---|---|
| **iPS:436031** | **21-225_193C7** | NA | AGCTATGGCATGCAC | GTTATATGGTATGATGGAAGTAATAAAAACTACGCAGACTCCGTGAAGGGC | GATCAGGGCGTGGGCTACGACGGCTTGGACGTC |
| | | | SEQ ID NO:6759 | SEQ ID NO:14771 | SEQ ID NO:22783 |
| | | AA | SYGMH | VIWYDGSNKNYADSVKG | DQGVGYDGLDV |
| | | | SEQ ID NO:6760 | SEQ ID NO:14772 | SEQ ID NO:22784 |
| **iPS:436033** | **21-225_193E7** | NA | GGTTACTTCTGGACC | GAAATCAATCATAGTGGAAGCACCAACTACAACCCGTCCCTCAAGAGT | GACTACGGTGCTGACTAC |
| | | | SEQ ID NO:6761 | SEQ ID NO:14773 | SEQ ID NO:22785 |
| | | AA | GYFWT | EINHSGSTNYNPSLKS | DYGADY |
| | | | SEQ ID NO:6762 | SEQ ID NO:14774 | SEQ ID NO:22786 |
| **iPS:436035** | **21-225_193C8** | NA | AGTGGTGATTACTACTGGAAC | TACATCTTTTACAGTGGGAGCACCTACTACAACCCGTCCCTCAAGAGT | GGGGATTACGATGGTTCGGGGAGTTATCACTACTACTACGGTATGGACGTC |
| | | | SEQ ID NO:6763 | SEQ ID NO:14775 | SEQ ID NO:22787 |
| | | AA | SGDYYWN | YIFYSGSTYYNPSLKS | GDYDGSGSYHYYYGMDV |
| | | | SEQ ID NO:6764 | SEQ ID NO:14776 | SEQ ID NO:22788 |
| **iPS:436037** | **21-225_193D8** | NA | AGTGGTGGTTACTACTGGAAC | TTCATCTTTTACAGTGGGAGCACCTACTACAACCCGTCCCTCAAGAGT | GGGGATTACGATGGTTCGGGGAGTTATCACTACTATTACGGTATGGACGTC |
| | | | SEQ ID NO:6765 | SEQ ID NO:14777 | SEQ ID NO:22789 |
| | | AA | SGGYYWN | FIFYSGSTYYNPSLKS | GDYDGSGSYHYYYGMDV |
| | | | SEQ ID NO:6766 | SEQ ID NO:14778 | SEQ ID NO:22790 |
| **iPS:436039** | **21-225_193F8** | NA | ATCTATGGCATGGAC | GTTATATGGTATGATGGAAGTTATAAATACTATGCAGACTCCGTGAAGGGC | GAGGATTCCCCTTATAGTGGCTACGGCTTGGACTACTACTACGGTATGGACGTC |
| | | | SEQ ID NO:6767 | SEQ ID NO:14779 | SEQ ID NO:22791 |

# FIGURE 49

| | | | | | |
|---|---|---|---|---|---|
| | | AA | IYGMD | VIWYDGSYKYYADSVKG | EDSPYSGYGLDYYYGMDV |
| | | | SEQ ID NO:6768 | SEQ ID NO:14780 | SEQ ID NO:22792 |
| **iPS:436041** | **21-225_193G8** | NA | AGTGGTGTTTACTACTGGAGC | AACATCTATTACAGTGGGAGCACCTACAACAACCCGTCCCTCAAGAGT | GGGGATTACGATGGTTCGGGGAGTTATCACTTCTACTACGGTTTGGACGTC |
| | | | SEQ ID NO:6769 | SEQ ID NO:14781 | SEQ ID NO:22793 |
| | | AA | SGVYYWS | NIYYSGSTYNNPSLKS | GDYDGSGSYHFYYGLDV |
| | | | SEQ ID NO:6770 | SEQ ID NO:14782 | SEQ ID NO:22794 |
| **iPS:436043** | **21-225_193G9** | NA | AATGGTGGATACTACTGGAGC | TACATCTTTTACAGCGGGAGCACCTACTACAACCCGTCCCTCAAGAGT | GCGGGATATAACTGGGACAACGGGTTTGACTAC |
| | | | SEQ ID NO:6771 | SEQ ID NO:14783 | SEQ ID NO:22795 |
| | | AA | NGGYYWS | YIFYSGSTYYNPSLKS | AGYNWDNGFDY |
| | | | SEQ ID NO:6772 | SEQ ID NO:14784 | SEQ ID NO:22796 |
| **iPS:436045** | **21-225_193A10** | NA | AGCTATGGCATGCAC | GTTATATGGTATGATGGAAGTAATGAACACTATGCAGACTCCGTGAAGGGC | GATAGGGGGGTGGGTTACTACGGTTTGGACGTC |
| | | | SEQ ID NO:6773 | SEQ ID NO:14785 | SEQ ID NO:22797 |
| | | AA | SYGMH | VIWYDGSNEHYADSVKG | DRGVGYYGLDV |
| | | | SEQ ID NO:6774 | SEQ ID NO:14786 | SEQ ID NO:22798 |
| **iPS:436047** | **21-225_193B10** | NA | GACTATAGCATGAAC | TCCATTAGTAGTGCTGGTGGTTACATATACTACGCAGACTCACTGAAGGGC | GCAACTATGGCCCTTGACTAC |
| | | | SEQ ID NO:6775 | SEQ ID NO:14787 | SEQ ID NO:22799 |
| | | AA | DYSMN | SISSAGGYIYYADSLKG | ATMALDY |
| | | | SEQ ID NO:6776 | SEQ ID NO:14788 | SEQ ID NO:22800 |

## FIGURE 49

| | | | | | |
|---|---|---|---|---|---|
| **iPS:436049** | **21-225_193B12** | NA | AGTGCTGATTACTACTGGAAC | TACATCTTTTACAGTGGGAGCACCTACTACAACCCGTCCCTCAAGAGT | GGGGATTACGATGGTTCGGGGAGTTATCACTTCTACTACGGTATGGACGTC |
| | | | SEQ ID NO:6777 | SEQ ID NO:14789 | SEQ ID NO:22801 |
| | | AA | SADYYWN | YIFYSGSTYYNPSLKS | GDYDGSGSYHFYYGMDV |
| | | | SEQ ID NO:6778 | SEQ ID NO:14790 | SEQ ID NO:22802 |
| **iPS:436051** | **21-225_193G12** | NA | AGCTATGCCATGCAC | GTTATATGGTATGATGGAACTAATAAATACTATGGAGACTCCGTGAAGGGC | GATTTCACTATAACTGGAGCTACATATTTTGACTAC |
| | | | SEQ ID NO:6779 | SEQ ID NO:14791 | SEQ ID NO:22803 |
| | | AA | SYAMH | VIWYDGTNKYYGDSVKG | DFTITGATYFDY |
| | | | SEQ ID NO:6780 | SEQ ID NO:14792 | SEQ ID NO:22804 |
| **iPS:436054** | **21-225_194C1** | NA | AGCTATGGCATGCAC | GTTATATGGTATGATGGAAGTAATGAACACTATGCAGACTCCGTGAAGGGC | AATAGGGGGGTGGGTTACTACGGTTTGGACGTC |
| | | | SEQ ID NO:6781 | SEQ ID NO:14793 | SEQ ID NO:22805 |
| | | AA | SYGMH | VIWYDGSNEHYADSVKG | NRGVGYYGLDV |
| | | | SEQ ID NO:6782 | SEQ ID NO:14794 | SEQ ID NO:22806 |
| **iPS:436056** | **21-225_194C3** | NA | AATTACTACTGGAGC | CGTATCTATGCCAGTGGGAGCACCAACTACAACCCCTCCCTCAAGAGT | GATCGGGGATACTATGGCTACTACGGTATGGACGTC |
| | | | SEQ ID NO:6783 | SEQ ID NO:14795 | SEQ ID NO:22807 |
| | | AA | NYYWS | RIYASGSTNYNPSLKS | DRGYYGYYGMDV |
| | | | SEQ ID NO:6784 | SEQ ID NO:14796 | SEQ ID NO:22808 |

FIGURE 49

| | | | | | |
|---|---|---|---|---|---|
| **iPS:436058** | **21-225_194A4** | NA | GTCTACTATTTGAAC | TGGATCAACCCTAACAGTGGTGGCACAAACTATGCACAGAAGTTTCAGGGC | GGCTACGATATTTTGACTGGT |
| | | | SEQ ID NO:6785 | SEQ ID NO:14797 | SEQ ID NO:22809 |
| | | AA | VYYLN | WINPNSGGTNYAQKFQG | GYDILTG |
| | | | SEQ ID NO:6786 | SEQ ID NO:14798 | SEQ ID NO:22810 |
| **iPS:436060** | **21-225_194F4** | NA | AGTTACCACTGGAGC | CTTATCTATACCAGTAGGAGCACCAACTACAACCCCTCCCTCAAGAGT | CTCCGGTATAACTGGAACTTCCCTTACTTTGACTAC |
| | | | SEQ ID NO:6787 | SEQ ID NO:14799 | SEQ ID NO:22811 |
| | | AA | SYHWS | LIYTSRSTNYNPSLKS | LRYNWNFPYFDY |
| | | | SEQ ID NO:6788 | SEQ ID NO:14800 | SEQ ID NO:22812 |
| **iPS:436062** | **21-225_194E5** | NA | AGTGGTGATTACTACTGGAAC | TACATCTTTTACAGTGGGAGCACCTACTACAACCCGTCCCTCAAGAGT | GGGGATTACGATGGTTCGGGGAGTTATCACTACTATTACGGTATGGACGTC |
| | | | SEQ ID NO:6789 | SEQ ID NO:14801 | SEQ ID NO:22813 |
| | | AA | SGDYYWN | YIFYSGSTYYNPSLKS | GDYDGSGSYHYYYGMDV |
| | | | SEQ ID NO:6790 | SEQ ID NO:14802 | SEQ ID NO:22814 |
| **iPS:436064** | **21-225_194E6** | NA | AGTGGTGATTACTACTGGAAC | TTCATCTTTTACAGTGGGAGCACCTACTACAACCCGTCCCTCAAGAGT | GGGGATTACGATGGTTCGGGGAGTTATCACTACTATTACGGTATGGACGTC |
| | | | SEQ ID NO:6791 | SEQ ID NO:14803 | SEQ ID NO:22815 |
| | | AA | SGDYYWN | FIFYSGSTYYNPSLKS | GDYDGSGSYHYYYGMDV |
| | | | SEQ ID NO:6792 | SEQ ID NO:14804 | SEQ ID NO:22816 |
| **iPS:436066** | **21-225_194B7** | NA | AGCTATGGCATGCAC | GTTATATGGTATGATGGAAGTAATAAAAACTATGCAGACTCCGTGAAGGGC | GATCGGTCTAAGGGTTACGACGGTATGGACGTC |
| | | | SEQ ID NO:6793 | SEQ ID NO:14805 | SEQ ID NO:22817 |

**FIGURE 49**

| | | | | | |
|---|---|---|---|---|---|
| | | AA | SYGMH | VIWYDGSNKNYADSVKG | DRSKGYDGMDV |
| | | | SEQ ID NO:6794 | SEQ ID NO:14806 | SEQ ID NO:22818 |
| **iPS:436068** | **21-225_194F7** | NA | GACTACTACATACAC | TGGATCAACCCTAACAA TGGTGGCACAAACTATG CACAGAAATTTCAGGGC | GAACCCCTTGGTTACTATGG TTCGGGGAGTTATGGGGCCT ACGGTATGGACGTC |
| | | | SEQ ID NO:6795 | SEQ ID NO:14807 | SEQ ID NO:22819 |
| | | AA | DYYIH | WINPNNGGTNYAQKFQG | EPLGYYGSGSYGAYGMDV |
| | | | SEQ ID NO:6796 | SEQ ID NO:14808 | SEQ ID NO:22820 |
| **iPS:436072** | **21-225_194C10** | NA | TATTACTACTGGAGC | GAAATCAATCATAGTGG AAGCACCAACTACAACC CGTCCCTCAAGAGT | GACTACGGTGCTTTTGATAT C |
| | | | SEQ ID NO:6797 | SEQ ID NO:14809 | SEQ ID NO:22821 |
| | | AA | YYYWS | EINHSGSTNYNPSLKS | DYGAFDI |
| | | | SEQ ID NO:6798 | SEQ ID NO:14810 | SEQ ID NO:22822 |
| **iPS:436074** | **21-225_194F10** | NA | AGCTTTATCATGCAC | GTTATATGGTATGATGG AAGTAATAAATACTATG CAGACTCCGTGAAGGGC | GAGGAGTATAGCAGTGGCTG GTTCGGGTACGGTATGGACG TC |
| | | | SEQ ID NO:6799 | SEQ ID NO:14811 | SEQ ID NO:22823 |
| | | AA | SFIMH | VIWYDGSNKYYADSVKG | EEYSSGWFGYGMDV |
| | | | SEQ ID NO:6800 | SEQ ID NO:14812 | SEQ ID NO:22824 |
| **iPS:436076** | **21-225_194H11** | NA | AGCTATGGCATGCAC | GTTATATGGTATGATGG AAGTAATGAACACTATG CAGACTCCGTGAAGGGC | GATAGGGGGGTGGGTTATTA CGGTTTGGACGTC |
| | | | SEQ ID NO:6801 | SEQ ID NO:14813 | SEQ ID NO:22825 |

**FIGURE 49**

| | | | | | |
|---|---|---|---|---|---|
| | | AA | SYGMH | VIWYDGSNEHYADSVKG | DRGVGYYGLDV |
| | | | SEQ ID NO:6802 | SEQ ID NO:14814 | SEQ ID NO:22826 |
| **iPS:436078** | **21-225_194H12** | NA | AACTATGGCATGCAC | GTTATATGGTTTGATGGAAGTAATGACTACTATGCAGACTCCGTGAAGGGC | GATAGAAGCGTCGGCTACGACGGTTTAGATGTC |
| | | | SEQ ID NO:6803 | SEQ ID NO:14815 | SEQ ID NO:22827 |
| | | AA | NYGMH | VIWFDGSNDYYADSVKG | DRSVGYDGLDV |
| | | | SEQ ID NO:6804 | SEQ ID NO:14816 | SEQ ID NO:22828 |
| **iPS:436080** | **21-225_195B1** | NA | TATTACTTCTGGAGC | GAAATCAATCATAGTGGACGCACCAACTACAACCCGTCCCTCAAGAGT | GACTACGGTGCTTTTGATATC |
| | | | SEQ ID NO:6805 | SEQ ID NO:14817 | SEQ ID NO:22829 |
| | | AA | YYFWS | EINHSGRTNYNPSLKS | DYGAFDI |
| | | | SEQ ID NO:6806 | SEQ ID NO:14818 | SEQ ID NO:22830 |
| **iPS:436082** | **21-225_195D9** | NA | CACTATGTCATGCAC | GTTATTTGGTATGATGGAACTAATAAATACTATGCAGACTCCGTGAAGGGC | GATTGGTTCGGGGAGGGGAACTACTACGGTATGGACGTC |
| | | | SEQ ID NO:6807 | SEQ ID NO:14819 | SEQ ID NO:22831 |
| | | AA | HYVMH | VIWYDGTNKYYADSVKG | DWFGEGNYYGMDV |
| | | | SEQ ID NO:6808 | SEQ ID NO:14820 | SEQ ID NO:22832 |
| **iPS:436084** | **21-225_195F2** | NA | AGCGGTGGTTACTACTGGAGC | TACAGCTATTACAGTGGGAGCACCAACTATAACCCGTCCCTCAAGAGT | GGGGGGTATAACTGGAACAACGGGTTTGACTAC |
| | | | SEQ ID NO:6809 | SEQ ID NO:14821 | SEQ ID NO:22833 |
| | | AA | SGGYYWS | YSYYSGSTNYNPSLKS | GGYNWNNGFDY |
| | | | SEQ ID NO:6810 | SEQ ID NO:14822 | SEQ ID NO:22834 |

FIGURE 49

| | | | | | |
|---|---|---|---|---|---|
| **iPS:436086** | **21-225_191G10** | NA | AGCTATGGCATGCAC | GTTATATGGTATGATGGAAGTAATAAAAACTATGCAGACTCCGTGAAGGGC | GATCAGGGCGTGGGCTACGACGGTTTGGACGTC |
| | | | SEQ ID NO:6811 | SEQ ID NO:14823 | SEQ ID NO:22835 |
| | | AA | SYGMH | VIWYDGSNKNYADSVKG | DQGVGYDGLDV |
| | | | SEQ ID NO:6812 | SEQ ID NO:14824 | SEQ ID NO:22836 |
| **iPS:436088** | **21-225_195C8** | NA | AGTGGTGATTACTACTGGAAC | TACATCTTTTACAGTGGGAGCACCTACTACAACCCGTCCCTCAAGAGT | GGGGATTACGATGGTTCGGGGAGTTATCACTTCTACTACGGTATGGACGTC |
| | | | SEQ ID NO:6813 | SEQ ID NO:14825 | SEQ ID NO:22837 |
| | | AA | SGDYYWN | YIFYSGSTYYNPSLKS | GDYDGSGSYHFYYGMDV |
| | | | SEQ ID NO:6814 | SEQ ID NO:14826 | SEQ ID NO:22838 |
| **iPS:436090** | **21-225_195A9** | NA | AGCTATGGCATGCAC | GTTATATGGTATGATGGAAGTAATGAACACTATGCAGACTCCGTGAAGGGC | GATAGGGGGGTGGGTTACTACGGTTTGGACGTC |
| | | | SEQ ID NO:6815 | SEQ ID NO:14827 | SEQ ID NO:22839 |
| | | AA | SYGMH | VIWYDGSNEHYADSVKG | DRGVGYYGLDV |
| | | | SEQ ID NO:6816 | SEQ ID NO:14828 | SEQ ID NO:22840 |
| **iPS:436092** | **21-225_195B9** | NA | AGCTATGGCATGCAC | GTTATATGGTATGATGGAAGTAATAAATACTATGCAGACTCCGTGAAGGGC | GAATGGCTACAATTCAGGTACTACTACGGTATGGACGTC |
| | | | SEQ ID NO:6817 | SEQ ID NO:14829 | SEQ ID NO:22841 |
| | | AA | SYGMH | VIWYDGSNKYYADSVKG | EWLQFRYYYGMDV |
| | | | SEQ ID NO:6818 | SEQ ID NO:14830 | SEQ ID NO:22842 |

FIGURE 49

| | | | | | |
|---|---|---|---|---|---|
| **iPS:436094** | **21-225_195B10** | NA | AATAGTGGTTACTACTGGAGC | TACATGTATTACAGTGGGAGCACCTACTACAACCCGTCCCTCAAGAGT | GGGGGGTATAACTGGAACAATGGGTTTGACTGT |
| | | | SEQ ID NO:6819 | SEQ ID NO:14831 | SEQ ID NO:22843 |
| | | AA | NSGYYWS | YMYYSGSTYYNPSLKS | GGYNWNNGFDC |
| | | | SEQ ID NO:6820 | SEQ ID NO:14832 | SEQ ID NO:22844 |
| **iPS:436096** | **21-225_195E10** | NA | AGTGGTGGTTACTACTGGAGC | TACATCTATTACAGTGGGAGCACCTACTACAACCCGTCCCTCAAGAGT | GGGGGGTATAACTGGAACCACGGTATGGACGTC |
| | | | SEQ ID NO:6821 | SEQ ID NO:14833 | SEQ ID NO:22845 |
| | | AA | SGGYYWS | YIYYSGSTYYNPSLKS | GGYNWNHGMDV |
| | | | SEQ ID NO:6822 | SEQ ID NO:14834 | SEQ ID NO:22846 |
| **iPS:436098** | **21-225_195G11** | NA | GACTACTACATGAGC | TACATTAGTAGTAGTGGTACTACCGTATTCTACGCAGACTCTGTGAAGGGC | GAATGGGTGGGAGCCGACTAC |
| | | | SEQ ID NO:6823 | SEQ ID NO:14835 | SEQ ID NO:22847 |
| | | AA | DYYMS | YISSSGTTVFYADSVKG | EWVGADY |
| | | | SEQ ID NO:6824 | SEQ ID NO:14836 | SEQ ID NO:22848 |
| **iPS:436100** | **21-225_195G12** | NA | ACCTATGCCATGAGT | GCTATTAGTCGTAGTGGTGGTAACACACACTACGCAGACTCCGTGAAGGGC | GATGGATTCGGTGGGAGCTCCTACTTTGACTAC |
| | | | SEQ ID NO:6825 | SEQ ID NO:14837 | SEQ ID NO:22849 |
| | | AA | TYAMS | AISRSGGNTHYADSVKG | DGFGGSSYFDY |
| | | | SEQ ID NO:6826 | SEQ ID NO:14838 | SEQ ID NO:22850 |
| **iPS:436102** | **21-225_196B1** | NA | GACTACTACATGAGC | TACATTAGTAGTAGTGGTATTACCATGTACTACGCAGACTCTGTGAAGGGC | GAATGGGTGGGAGCCGACTAC |
| | | | SEQ ID NO:6827 | SEQ ID NO:14839 | SEQ ID NO:22851 |
| | | AA | DYYMS | YISSSGITMYYADSVKG | EWVGADY |
| | | | SEQ ID NO:6828 | SEQ ID NO:14840 | SEQ ID NO:22852 |

FIGURE 49

| | | | | | |
|---|---|---|---|---|---|
| **iPS:436104** | **21-225_196C1** | NA | GACTACTACATGAGC | TACATTAGTAGTAGTGGTACTACCGTATTCTACGCAGACTCTGTGAAGGGC | GAATGGGTGGGAGCCGACTAC |
| | | | SEQ ID NO:6829 | SEQ ID NO:14841 | SEQ ID NO:22853 |
| | | AA | DYYMS | YISSSGTTVFYADSVKG | EWVGADY |
| | | | SEQ ID NO:6830 | SEQ ID NO:14842 | SEQ ID NO:22854 |
| **iPS:436106** | **21-225_196F2** | NA | AGCTTTGGCATGCAC | GTTATATTAAATGATGGAAGTAATAAAAAGTGTGCAGACTCCGTGAAGGGC | GGACAGCAGTGGCTGGTAAACGGTGTGGACGTC |
| | | | SEQ ID NO:6831 | SEQ ID NO:14843 | SEQ ID NO:22855 |
| | | AA | SFGMH | VILNDGSNKKCADSVKG | GQQWLVNGVDV |
| | | | SEQ ID NO:6832 | SEQ ID NO:14844 | SEQ ID NO:22856 |
| **iPS:436110** | **21-225_196F4** | NA | AGCTGTGCCATGACC | GCTATTAGTGGTAGTGGTGGTAGCACATACTACGCAGACTCCGTGAAGGGC | GTGGGGGGTTTGACTGGCTCCTACTACTACTACGGTATGGACGTC |
| | | | SEQ ID NO:6833 | SEQ ID NO:14845 | SEQ ID NO:22857 |
| | | AA | SCAMT | AISGSGGSTYYADSVKG | VGGLTGSYYYYGMDV |
| | | | SEQ ID NO:6834 | SEQ ID NO:14846 | SEQ ID NO:22858 |
| **iPS:436112** | **21-225_196C7** | NA | AGCTATGGCATGCAC | GTTATATGGTATGATGGAAGTAATGAACACTATGCAGACTCCGTGAAGGGC | GATAGGGGGGTGGGTTACTACGGTTTGGACGTC |
| | | | SEQ ID NO:6835 | SEQ ID NO:14847 | SEQ ID NO:22859 |
| | | AA | SYGMH | VIWYDGSNEHYADSVKG | DRGVGYYGLDV |
| | | | SEQ ID NO:6836 | SEQ ID NO:14848 | SEQ ID NO:22860 |
| **iPS:436114** | | NA | AATTATGATATCAAC | TGGATGCACCTTAACAGTGGTAACACAGGCTATGCACCGAAGTTCCAGGGC | AGCGGTGGCTGGTACGTGTTCGACCCC |

FIGURE 49

| | | | | | |
|---|---|---|---|---|---|
| | **21-225_196G8** | | SEQ ID NO:6837 | SEQ ID NO:14849 | SEQ ID NO:22861 |
| | | AA | NYDIN | WMHLNSGNTGYAPKFQG | SGGWYVFDP |
| | | | SEQ ID NO:6838 | SEQ ID NO:14850 | SEQ ID NO:22862 |
| **iPS:436116** | **21-225_196B9** | NA | GGCTACTATATGCAC | TGGATCAACCCTAACAGTGGTGGCACAAACTTTGCACAGAAGTTTCGGGGC | GGGGGGGTTCGGGGAGTTCCCAACTACTACTACGTTATGGACGTC |
| | | | SEQ ID NO:6839 | SEQ ID NO:14851 | SEQ ID NO:22863 |
| | | AA | GYYMH | WINPNSGGTNFAQKFRG | GGVRGVPNYYYVMDV |
| | | | SEQ ID NO:6840 | SEQ ID NO:14852 | SEQ ID NO:22864 |
| **iPS:436118** | **21-225_196A10** | NA | CACTATGTCATGCAC | GTTATTTGGTATGATGGAACTAATAAATACTATGCAGACTCCGTGAAGGGC | GATTGGTTCGGGGAGGGGAACTACTACGGTATGGACGTC |
| | | | SEQ ID NO:6841 | SEQ ID NO:14853 | SEQ ID NO:22865 |
| | | AA | HYVMH | VIWYDGTNKYYADSVKG | DWFGEGNYYGMDV |
| | | | SEQ ID NO:6842 | SEQ ID NO:14854 | SEQ ID NO:22866 |
| **iPS:436120** | **21-225_196C10** | NA | AGTGGTGGTGACTACTGGAGC | TTCATCTATTACAGTGGGAGCACCTACTACAATCCGTCCCTCAAGAGT | ATGGACTACAGTAACTACTACTACGGTATGGACGTC |
| | | | SEQ ID NO:6843 | SEQ ID NO:14855 | SEQ ID NO:22867 |
| | | AA | SGGDYWS | FIYYSGSTYYNPSLKS | MDYSNYYYGMDV |
| | | | SEQ ID NO:6844 | SEQ ID NO:14856 | SEQ ID NO:22868 |
| **iPS:436122** | **21-225_196G10** | NA | GACTATAGCATGAAC | TCTATTAGTAGTGGTAGTGGTTACATACACTACGCAGACTCAGTGAAGGGC | GCAACTATGGCCCTTGACTAC |
| | | | SEQ ID NO:6845 | SEQ ID NO:14857 | SEQ ID NO:22869 |
| | | AA | DYSMN | SISSGSGYIHYADSVKG | ATMALDY |
| | | | SEQ ID NO:6846 | SEQ ID NO:14858 | SEQ ID NO:22870 |

FIGURE 49

| | | | | | |
|---|---|---|---|---|---|
| **iPS:436132** | **21-225_196C12** | NA | AGTTATGATATCAAC | TGGATGAACCCTAAAAGGGGTAACACAGGCTATGCACAGAAGTTCCAGGGC | GGAGACCCGTATAACTGGAACTCCTACGCTATGGACGTC |
| | | | SEQ ID NO:6847 | SEQ ID NO:14859 | SEQ ID NO:22871 |
| | | AA | SYDIN | WMNPKRGNTGYAQKFQG | GDPYNWNSYAMDV |
| | | | SEQ ID NO:6848 | SEQ ID NO:14860 | SEQ ID NO:22872 |
| **iPS:436134** | **21-225_196H12** | NA | AGTGGTGTTTACTACTGGAGC | AACATCTATTACAGTGGGAGCACCTACAACAACCCGTCCCTCAAGAGT | GGGGATTACGATGGTTCGGGGAGTTATCACTACTACTACGGTTTGGACGTC |
| | | | SEQ ID NO:6849 | SEQ ID NO:14861 | SEQ ID NO:22873 |
| | | AA | SGVYYWS | NIYYSGSTYNNPSLKS | GDYDGSGSYHYYYGLDV |
| | | | SEQ ID NO:6850 | SEQ ID NO:14862 | SEQ ID NO:22874 |
| **iPS:436138** | **21-225_197F2** | NA | AGCTATGGCATGCAC | GTTATATGGTATGATGGAAGTAATAAAAACTATGCAGACTCCGTGAAGGGC | GATCAGGGCGTGGGCTACGACGGTTTGGACGTC |
| | | | SEQ ID NO:6851 | SEQ ID NO:14863 | SEQ ID NO:22875 |
| | | AA | SYGMH | VIWYDGSNKNYADSVKG | DQGVGYDGLDV |
| | | | SEQ ID NO:6852 | SEQ ID NO:14864 | SEQ ID NO:22876 |
| **iPS:436140** | **21-225_197G3** | NA | AGCCATGGCATGCAC | GTTATATGGTATGATGGAAGTAATAAAAACTATGCAGACTCCGTGAAGGGC | GATCCCTCTGTAGGGTACGACGGTATGGACGTC |
| | | | SEQ ID NO:6853 | SEQ ID NO:14865 | SEQ ID NO:22877 |
| | | AA | SHGMH | VIWYDGSNKNYADSVKG | DPSVGYDGMDV |
| | | | SEQ ID NO:6854 | SEQ ID NO:14866 | SEQ ID NO:22878 |

FIGURE 49

| | | | | | |
|---|---|---|---|---|---|
| **iPS:436146** | **21-225_197F4** | NA | AGTGGTGATTACTACTGGAAC | TACATCTTTTACAGTGGGAGCACCTACTACAACCCGTCCCTCAAGAGT | GGGGATTACGATGGTTCGGGGAGTTATCACTACTACTACGGTTTGGACGTC |
| | | | SEQ ID NO:6855 | SEQ ID NO:14867 | SEQ ID NO:22879 |
| | | AA | SGDYYWN | YIFYSGSTYYNPSLKS | GDYDGSGSYHYYYGLDV |
| | | | SEQ ID NO:6856 | SEQ ID NO:14868 | SEQ ID NO:22880 |
| **iPS:436150** | **21-225_197H4** | NA | AATTATGATATCAAC | TGGATGCACCCTAACAGTGGTAACACAGGCTATGCACAGAAGTTCCAGGGC | AGCAGTGGCTGGTACTACTTTGACTAC |
| | | | SEQ ID NO:6857 | SEQ ID NO:14869 | SEQ ID NO:22881 |
| | | AA | NYDIN | WMHPNSGNTGYAQKFQG | SSGWYYFDY |
| | | | SEQ ID NO:6858 | SEQ ID NO:14870 | SEQ ID NO:22882 |
| **iPS:436152** | **21-225_197B6** | NA | AGCTATGGCATGCAC | GTTATATGGTATGATGGAAGTAATAAAAACTACGCAGACTCCGTGAAGGGC | GATCAGGGCGTGGGCTACGACGGTTTGGACGTC |
| | | | SEQ ID NO:6859 | SEQ ID NO:14871 | SEQ ID NO:22883 |
| | | AA | SYGMH | VIWYDGSNKNYADSVKG | DQGVGYDGLDV |
| | | | SEQ ID NO:6860 | SEQ ID NO:14872 | SEQ ID NO:22884 |
| **iPS:436154** | **21-225_197C6** | NA | AATTATGATATCAAC | TGGATGCACCCTAACAGTGGTAACACAGGCTATGCACAGAAGTTCCAGGGC | AGCAGTGGCTGGTACTACTTTGACTAC |
| | | | SEQ ID NO:6861 | SEQ ID NO:14873 | SEQ ID NO:22885 |
| | | AA | NYDIN | WMHPNSGNTGYAQKFQG | SSGWYYFDY |
| | | | SEQ ID NO:6862 | SEQ ID NO:14874 | SEQ ID NO:22886 |

FIGURE 49

| | | | | | |
|---|---|---|---|---|---|
| **iPS:436156** | **21-225_197C8** | NA | AGCTCTGCCATGACC | GCTATCATTGGTAATGGTGGTAGAGCATACTACGCAGACTCCGTGAAGGGC | GATCGGGGATATAGCAGGATAGCAGTGGCTGGTACCTTTGACTAC |
| | | | SEQ ID NO:6863 | SEQ ID NO:14875 | SEQ ID NO:22887 |
| | | AA | SSAMT | AIIGNGGRAYYADSVKG | DRGYSRIAVAGTFDY |
| | | | SEQ ID NO:6864 | SEQ ID NO:14876 | SEQ ID NO:22888 |
| **iPS:436158** | **21-225_197G8** | NA | GCTTACTCCTGGAGC | CGTCTCTCTCCTGGTGGGAGCACCAACTTCAACCCCTCCCTCAAGAGT | CTCCGGTATAACTGGAACTTCCCTTACTTTGACTAC |
| | | | SEQ ID NO:6865 | SEQ ID NO:14877 | SEQ ID NO:22889 |
| | | AA | AYSWS | RLSPGGSTNFNPSLKS | LRYNWNFPYFDY |
| | | | SEQ ID NO:6866 | SEQ ID NO:14878 | SEQ ID NO:22890 |
| **iPS:436160** | **21-225_197C9** | NA | AGCTATGCCATGAGC | GTTATTAGTGGTAGAGGTGGTAACACATACTACGCAGACTCCGTGAAGGGC | GGCATAGCAGTGGCTGGCTCGCACTACTTTGACTAC |
| | | | SEQ ID NO:6867 | SEQ ID NO:14879 | SEQ ID NO:22891 |
| | | AA | SYAMS | VISGRGGNTYYADSVKG | GIAVAGSHYFDY |
| | | | SEQ ID NO:6868 | SEQ ID NO:14880 | SEQ ID NO:22892 |
| **iPS:436164** | **21-225_197G10** | NA | AGCTATGGCATGCAC | GTTACATGGTATGATGGAAGTAATAAATACTATGCAGACTCCGTGAAGGGC | GAATGGCTACAATTTAGGTACTACTACGGTATAGACGTC |
| | | | SEQ ID NO:6869 | SEQ ID NO:14881 | SEQ ID NO:22893 |
| | | AA | SYGMH | VTWYDGSNKYYADSVKG | EWLQFRYYYGIDV |
| | | | SEQ ID NO:6870 | SEQ ID NO:14882 | SEQ ID NO:22894 |
| **iPS:436167** | **21-225_197E11** | NA | AACTATGGCATGCAC | GTTATATGGTTTGATGGAAGTAATGACTACTATGCAGACTCCGTGAAGGGC | GATAGAAGCGTCGGCTACGACGGTTTAGATGTC |
| | | | SEQ ID NO:6871 | SEQ ID NO:14883 | SEQ ID NO:22895 |

FIGURE 49

| | | | | | |
|---|---|---|---|---|---|
| | | AA | NYGMH | VIWFDGSNDYYADSVKG | DRSVGYDGLDV |
| | | | SEQ ID NO:6872 | SEQ ID NO:14884 | SEQ ID NO:22896 |
| iPS:436173 | 21-225_197G12 | NA | AGCTATGGCATGCAC | GTTATATGGTATGATGG AAGTAATAAAAACTATG CAGACTCCGTGAAGGGC | GATCAGGGCGTGGGCTACGA CGGTTTGGACGTC |
| | | | SEQ ID NO:6873 | SEQ ID NO:14885 | SEQ ID NO:22897 |
| | | AA | SYGMH | VIWYDGSNKNYADSVKG | DQGVGYDGLDV |
| | | | SEQ ID NO:6874 | SEQ ID NO:14886 | SEQ ID NO:22898 |
| iPS:436177 | 21-225_198B1 | NA | AGTGGTGATTACTACTGGAA C | TACATCTTCCACAGTGGG AGCACCTACTACAACCC GTCCCTCAAGAGT | GGGGATTACGATGGTTCGGG GAGTTATCACTACTATTACG GTATGGACGTC |
| | | | SEQ ID NO:6875 | SEQ ID NO:14887 | SEQ ID NO:22899 |
| | | AA | SGDYYWN | YIFHSGSTYYNPSLKS | GDYDGSGSYHYYYGMDV |
| | | | SEQ ID NO:6876 | SEQ ID NO:14888 | SEQ ID NO:22900 |
| iPS:436179 | 21-225_198E1 | NA | AGTGGTGGTTACTACTGGAA C | TTCATCTTTTACAGTGGG AGCACCTACTACAACCC GTCCCTCAAGAGT | GGGGATTACGATGGTTCGGG GAGTTATCACTACTATTACG GTATGGACGTC |
| | | | SEQ ID NO:6877 | SEQ ID NO:14889 | SEQ ID NO:22901 |
| | | AA | SGGYYWN | FIFYSGSTYYNPSLKS | GDYDGSGSYHYYYGMDV |
| | | | SEQ ID NO:6878 | SEQ ID NO:14890 | SEQ ID NO:22902 |
| iPS:436181 | 21-225_198C2 | NA | AGTGGTGGTTACTACTGGAG C | AACATCTATTACAGTGG GAGCACCTACTACAACC CGTCCCTCAAGAGT | GGGGATTACTATGGTTCGGG GAGTTATCACAACTACTACG GTTTGGACGTC |
| | | | SEQ ID NO:6879 | SEQ ID NO:14891 | SEQ ID NO:22903 |
| | | AA | SGGYYWS | NIYYSGSTYYNPSLKS | GDYYGSGSYHNYYGLDV |
| | | | SEQ ID NO:6880 | SEQ ID NO:14892 | SEQ ID NO:22904 |

FIGURE 49

| | | | | | |
|---|---|---|---|---|---|
| **iPS:436189** | **21-225_198B6** | NA | AGCTATGGCATGCAC | GTTATATGGTATGATGGAAGTAATAAACACTATGCAGACTCCGTGAAGGGC | GATCAGGGCGTGGGCTACTACGGTATGGACGTC |
| | | | SEQ ID NO:6881 | SEQ ID NO:14893 | SEQ ID NO:22905 |
| | | AA | SYGMH | VIWYDGSNKHYADSVKG | DQGVGYYGMDV |
| | | | SEQ ID NO:6882 | SEQ ID NO:14894 | SEQ ID NO:22906 |
| **iPS:436191** | **21-225_198B9** | NA | AACTATGGCATGCAC | ATTATATGGTTTGATGGAAGTAATAAATACTATGTAGACTCCGTGAAGGGC | GAATGGCTACAATTCAGGTACTACTACGGTATGGACGTC |
| | | | SEQ ID NO:6883 | SEQ ID NO:14895 | SEQ ID NO:22907 |
| | | AA | NYGMH | IIWFDGSNKYYVDSVKG | EWLQFRYYYGMDV |
| | | | SEQ ID NO:6884 | SEQ ID NO:14896 | SEQ ID NO:22908 |
| **iPS:436193** | **21-225_198A10** | NA | AGTTACCACTGGAGT | CATATCTATACCAGTAGGAGCACCAACTACAACCCCTCCCTCAAGAGT | CTCCGGTATAACTGGAACTTCCCTTACTTTGACTAC |
| | | | SEQ ID NO:6885 | SEQ ID NO:14897 | SEQ ID NO:22909 |
| | | AA | SYHWS | HIYTSRSTNYNPSLKS | LRYNWNFPYFDY |
| | | | SEQ ID NO:6886 | SEQ ID NO:14898 | SEQ ID NO:22910 |
| **iPS:436195** | **21-225_198G10** | NA | AGTGGTGATTACTACTGGAAC | TACATCTTTTACAGTGGGAGCACCTACTACAACCCGTCCCTCAAGAGT | GGGGATTACGATGGTTCGGGGAGTTATCACTTCTACTACGGTATGGACGTC |
| | | | SEQ ID NO:6887 | SEQ ID NO:14899 | SEQ ID NO:22911 |
| | | AA | SGDYYWN | YIFYSGSTYYNPSLKS | GDYDGSGSYHFYYGMDV |
| | | | SEQ ID NO:6888 | SEQ ID NO:14900 | SEQ ID NO:22912 |
| **iPS:436197** | **21 225 199C2** | NA | AGTGGTGATTACTACTGGAAC | TACATCTTTTACAGTGGGAGCACCTACTACAACCCGTCCCTCAAGAGT | GGGGATTACGATGGTTCGGGGAGTTATCACTACTACTACGGTATGGACGTC |

FIGURE 49

| | | | | | |
|---|---|---|---|---|---|
| | **21-225_199C2** | | SEQ ID NO:6889 | SEQ ID NO:14901 | SEQ ID NO:22913 |
| | | AA | SGDYYWN | YIFYSGSTYYNPSLKS | GDYDGSGSYHYYYGMDV |
| | | | SEQ ID NO:6890 | SEQ ID NO:14902 | SEQ ID NO:22914 |
| **iPS:436199** | **21-225_199E3** | NA | GGTTACTTCTGGACC | GAAATCAATCATAGTGG AAGCACCAACTACAACC CGTCCCTCAAGAGT | GACTACGGTGCTGACTAC |
| | | | SEQ ID NO:6891 | SEQ ID NO:14903 | SEQ ID NO:22915 |
| | | AA | GYFWT | EINHSGSTNYNPSLKS | DYGADY |
| | | | SEQ ID NO:6892 | SEQ ID NO:14904 | SEQ ID NO:22916 |
| **iPS:436201** | **21-225_199C5** | NA | AGCTATGGCATGCAC | GTTATATGGTATGATGG AAGTAATAAAAACTATG CAGACTCCGTGAAGGGC | GATCAGGGCGTGGGCTACTA CGGTATGGACGTC |
| | | | SEQ ID NO:6893 | SEQ ID NO:14905 | SEQ ID NO:22917 |
| | | AA | SYGMH | VIWYDGSNKNYADSVKG | DQGVGYYGMDV |
| | | | SEQ ID NO:6894 | SEQ ID NO:14906 | SEQ ID NO:22918 |
| **iPS:436203** | **21-225_199A6** | NA | AACTATGGCATGCAC | ATTATATGGTTTGATGGA AGTAATCAATACTATGC CGACTCCGTGAAGGGC | GCCCACGGGGTCTACTACTA CGCTATGGACGTC |
| | | | SEQ ID NO:6895 | SEQ ID NO:14907 | SEQ ID NO:22919 |
| | | AA | NYGMH | IIWFDGSNQYYADSVKG | AHGVYYYAMDV |
| | | | SEQ ID NO:6896 | SEQ ID NO:14908 | SEQ ID NO:22920 |
| **iPS:436205** | **21-225_199A7** | NA | AACTATGGCATGCAC | ATTATATGGTTTGATGGA AGTAATAAATACTATGC AGACTCCGTGAAGGGC | GAATGGCTACAATTCAGGTA CTACTACGGTATGGACGTC |
| | | | SEQ ID NO:6897 | SEQ ID NO:14909 | SEQ ID NO:22921 |
| | | AA | NYGMH | IIWFDGSNKYYADSVKG | EWLQFRYYYGMDV |
| | | | SEQ ID NO:6898 | SEQ ID NO:14910 | SEQ ID NO:22922 |

FIGURE 49

| | | | | | |
|---|---|---|---|---|---|
| **iPS:436207** | **21-225_199C7** | NA | AGTGGTGGTTACTACTGGAAC | TTCATCTTTTACAGTGGGAGCACCTACTACAACCCGTCCCTCAAGAGT | GGGGATTACGATGGTTCGGGGAGTTATCACTACTATTACGGTATGGACGTC |
| | | | SEQ ID NO:6899 | SEQ ID NO:14911 | SEQ ID NO:22923 |
| | | AA | SGGYYWN | FIFYSGSTYYNPSLKS | GDYDGSGSYHYYYGMDV |
| | | | SEQ ID NO:6900 | SEQ ID NO:14912 | SEQ ID NO:22924 |
| **iPS:436210** | **21-225_199G11** | NA | AGTGGTGGTTACTACTGGAGC | AACATCTATTACAGTGGGAGCACCTACTACAACCCGTCCCTCAAGAGT | GGGGATTACTATGGTTCGGGGAGTTATCACAACTACTACGGTTTGGACGTC |
| | | | SEQ ID NO:6901 | SEQ ID NO:14913 | SEQ ID NO:22925 |
| | | AA | SGGYYWS | NIYYSGSTYYNPSLKS | GDYYGSGSYHNYYGLDV |
| | | | SEQ ID NO:6902 | SEQ ID NO:14914 | SEQ ID NO:22926 |
| **iPS:436212** | **21-225_200G1** | NA | AGCTATGCCATGAGC | GCTATTAGTGGTAGAGGCGGTAATACATTCTACGCAGACTCCGTGAGGGGC | CGTATAGCAGTGGATGGCTATGATGCTTTTGATGTC |
| | | | SEQ ID NO:6903 | SEQ ID NO:14915 | SEQ ID NO:22927 |
| | | AA | SYAMS | AISGRGGNTFYADSVRG | RIAVDGYDAFDV |
| | | | SEQ ID NO:6904 | SEQ ID NO:14916 | SEQ ID NO:22928 |
| **iPS:436214** | **21-225_200F6** | NA | AGTTATGGCATGCAC | GTTATATGGTATGATGGAAGTAATAAATACTATGCAGACTCCGTGAAGGGC | GAATGGCTACAATTTAGGTATTACTACGGTATGGACGTC |
| | | | SEQ ID NO:6905 | SEQ ID NO:14917 | SEQ ID NO:22929 |
| | | AA | SYGMH | VIWYDGSNKYYADSVKG | EWLQFRYYYGMDV |
| | | | SEQ ID NO:6906 | SEQ ID NO:14918 | SEQ ID NO:22930 |
| **iPS:436216** | **21-225_200B7** | NA | AGTGGTGGTTACTACTGGAGC | TACATCTTTTACAGTGGGAGCACCAACTACAACCCGTCCCTCAGGAGT | GCCGGGTATAACTGGAACAACGGTATGGACGTC |
| | | | SEQ ID NO:6907 | SEQ ID NO:14919 | SEQ ID NO:22931 |

FIGURE 49

| | | | | | |
|---|---|---|---|---|---|
| | | AA | SGGYYWS | YIFYSGSTNYNPSLRS | AGYNWNNGMDV |
| | | | SEQ ID NO:6908 | SEQ ID NO:14920 | SEQ ID NO:22932 |
| **iPS:436218** | **21-225_200G7** | NA | AATTATGATATCAAC | TGGATGCACCTTAACAGTGGTAACACAGGCTATGCACCGAAGTTCCAGGGC | AGCGGTGGCTGGTACGTGTTCGACCCC |
| | | | SEQ ID NO:6909 | SEQ ID NO:14921 | SEQ ID NO:22933 |
| | | AA | NYDIN | WMHLNSGNTGYAPKFQG | SGGWYVFDP |
| | | | SEQ ID NO:6910 | SEQ ID NO:14922 | SEQ ID NO:22934 |
| **iPS:436220** | **21-225_200F8** | NA | AATTACTACTGGAGC | CGTATCTATACCAGTGGGAGCACCAACTACAACCCCTCCCTCAAGAGT | GATCGGGGATACTATGGCTACTACGGTATGGACGTC |
| | | | SEQ ID NO:6911 | SEQ ID NO:14923 | SEQ ID NO:22935 |
| | | AA | NYYWS | RIYTSGSTNYNPSLKS | DRGYYGYYGMDV |
| | | | SEQ ID NO:6912 | SEQ ID NO:14924 | SEQ ID NO:22936 |
| **iPS:436222** | **21-225_200C9** | NA | AGCTATGGCATGCAC | GTTATATCATATGATGGAGGTTATAAAAACTATATAGACTCCGTGAAGGGC | GGTACCCACGGGTACTACTACGGTGTGGACGTC |
| | | | SEQ ID NO:6913 | SEQ ID NO:14925 | SEQ ID NO:22937 |
| | | AA | SYGMH | VISYDGGYKNYIDSVKG | GTHGYYYGVDV |
| | | | SEQ ID NO:6914 | SEQ ID NO:14926 | SEQ ID NO:22938 |
| **iPS:436226** | **21-225_200F10** | NA | AGTGGTGATTACTACTGGAAC | TACATCTTTTACAGTGGGAGCACCTACTACAACCCGTCCCTCAAGAGT | GGGGATTACGATGGTTCGGGGAGTTATCACTACTACTACGGTATGGACGTC |
| | | | SEQ ID NO:6915 | SEQ ID NO:14927 | SEQ ID NO:22939 |
| | | AA | SGDYYWN | YIFYSGSTYYNPSLKS | GDYDGSGSYHYYYGMDV |
| | | | SEQ ID NO:6916 | SEQ ID NO:14928 | SEQ ID NO:22940 |

FIGURE 49

| | | | | | |
|---|---|---|---|---|---|
| **iPS:436228** | **21-225_200F12** | NA | GGTTACTTCTGGACC | GAAATCAGTCATAGTGGAAGCACCAACTACAACCCGTCCCTCAAGAGT | GACTACGGGGCGGACTAC |
| | | | SEQ ID NO:6917 | SEQ ID NO:14929 | SEQ ID NO:22941 |
| | | AA | GYFWT | EISHSGSTNYNPSLKS | DYGADY |
| | | | SEQ ID NO:6918 | SEQ ID NO:14930 | SEQ ID NO:22942 |
| **iPS:436230** | **21-225_201A1** | NA | GGTTACTTCTGGACC | GAAATCAGTCATAGTGGACGCACCAACTACAACCCGTCCCTCAAGAGT | GACTACGGGGCGGACTAC |
| | | | SEQ ID NO:6919 | SEQ ID NO:14931 | SEQ ID NO:22943 |
| | | AA | GYFWT | EISHSGRTNYNPSLKS | DYGADY |
| | | | SEQ ID NO:6920 | SEQ ID NO:14932 | SEQ ID NO:22944 |
| **iPS:436232** | **21-225_201E1** | NA | CCTTACTACTGGAGC | GAAGTCAATCATAGTGGAAGCACCAACTACAACCCGTCCCTCAAGAGT | GACTACGGGGGTTTAGACTAC |
| | | | SEQ ID NO:6921 | SEQ ID NO:14933 | SEQ ID NO:22945 |
| | | AA | PYYWS | EVNHSGSTNYNPSLKS | DYGGLDY |
| | | | SEQ ID NO:6922 | SEQ ID NO:14934 | SEQ ID NO:22946 |
| **iPS:436234** | **21-225_51E3** | NA | AGCTATGGTATCAGC | TGGATCAGCGCTTATAATGGTAACACAAAGAATGCACAGAGGTTCCAGGGC | CACGATTTTTGGAGTGGTTATTATAAGGGTATGGACGTC |
| | | | SEQ ID NO:6923 | SEQ ID NO:14935 | SEQ ID NO:22947 |
| | | AA | SYGIS | WISAYNGNTKNAQRFQG | HDFWSGYYKGMDV |
| | | | SEQ ID NO:6924 | SEQ ID NO:14936 | SEQ ID NO:22948 |
| **iPS:436236** | **21-225_201F7** | NA | AGCAACAGTGCTGCTTGGAAC | AGGACATACTACAGGTCCAAGTGGTATAATTATTATGAAGTATCTGTGAGAAGT | GATCAACGGTACTACGGTATGGACGTC |
| | | | SEQ ID NO:6925 | SEQ ID NO:14937 | SEQ ID NO:22949 |

FIGURE 49

| | | | | | |
|---|---|---|---|---|---|
| | | AA | SNSAAWN | RTYYRSKWYNYYEVSVRS | DQRYYGMDV |
| | | | SEQ ID NO:6926 | SEQ ID NO:14938 | SEQ ID NO:22950 |
| **iPS:436238** | **21-225_201B2** | NA | GTTTACTACTGGACC | GAAATCAATCATAGTGGAAGCACCAACTACAACCCGTCCCTCAAGAGT | GACTATGGTGTCTTTGATTAC |
| | | | SEQ ID NO:6927 | SEQ ID NO:14939 | SEQ ID NO:22951 |
| | | AA | VYYWT | EINHSGSTNYNPSLKS | DYGVFDY |
| | | | SEQ ID NO:6928 | SEQ ID NO:14940 | SEQ ID NO:22952 |
| **iPS:436240** | **21-225_201E8** | NA | GGCTACTATATGCAC | TGGATCGACCCTAACAGTGGTGGCACAAACTATCCACAGAAGTTTCAGGGC | GATCAAGGGTATAACTGGAACTCTTTTGACTAC |
| | | | SEQ ID NO:6929 | SEQ ID NO:14941 | SEQ ID NO:22953 |
| | | AA | GYYMH | WIDPNSGGTNYPQKFQG | DQGYNWNSFDY |
| | | | SEQ ID NO:6930 | SEQ ID NO:14942 | SEQ ID NO:22954 |
| **iPS:436242** | **21-225_201A10** | NA | GGTTACTTCTGGACC | GAAATCAGTCATAGTGGAAGCACCAACTACAACCCGTCCCTCAAGAGT | GACTACGGGGCGGACTAC |
| | | | SEQ ID NO:6931 | SEQ ID NO:14943 | SEQ ID NO:22955 |
| | | AA | GYFWT | EISHSGSTNYNPSLKS | DYGADY |
| | | | SEQ ID NO:6932 | SEQ ID NO:14944 | SEQ ID NO:22956 |
| **iPS:436244** | **21-225_201H10** | NA | GGCTACTATATCCAC | TGGATCAACCCTAATAGTGGTGGCACAAACTATGCACAGAAGTTTCAGGGC | GGATACAGCTATGGTTACAACTGGTTCGACCCC |
| | | | SEQ ID NO:6933 | SEQ ID NO:14945 | SEQ ID NO:22957 |
| | | AA | GYYIH | WINPNSGGTNYAQKFQG | GYSYGYNWFDP |
| | | | SEQ ID NO:6934 | SEQ ID NO:14946 | SEQ ID NO:22958 |
| **iPS:436246** | **21 225 201G6** | NA | AGCTATGCCATGAGC | ACTATTAGTGGTAGTGGTGTTAGAACATACTACGCAGACTCCGTGAAGGGC | GGGGGAGCTAGGAGCAGTGGCTGGTTCCACTTTGACTAC |

FIGURE 49

| | | | | | |
|---|---|---|---|---|---|
| | 21-225_201G6 | | SEQ ID NO:6935 | SEQ ID NO:14947 | SEQ ID NO:22959 |
| | | AA | SYAMS | TISGSGVRTYYADSVKG | GGARSSGWFHFDY |
| | | | SEQ ID NO:6936 | SEQ ID NO:14948 | SEQ ID NO:22960 |
| iPS:436248 | 21-225_202A3 | NA | AGTTATGATATCAAC | TGGATGAACCCTAAGAG AGGTAACACAGGCTATG CACAGAAGTTCCAGGGC | GGAAGGTATAGCAGGGAGG ATTACTACTACTATTATGAT ATGGACGTC |
| | | | SEQ ID NO:6937 | SEQ ID NO:14949 | SEQ ID NO:22961 |
| | | AA | SYDIN | WMNPKRGNTGYAQKFQG | GRYSREDYYYYYDMDV |
| | | | SEQ ID NO:6938 | SEQ ID NO:14950 | SEQ ID NO:22962 |
| iPS:436250 | 21-225_201A4 | NA | AGCAACAGTGCTGCTTGGAA C | AGGACATACTACAGGTC CAAGTGGTATAATTATTA TGAAGTATCTGTGAAAA GT | GATCAACGGTACTACGGTAT GGACGTC |
| | | | SEQ ID NO:6939 | SEQ ID NO:14951 | SEQ ID NO:22963 |
| | | AA | SNSAAWN | RTYYRSKWYNYYEVSVK S | DQRYYGMDV |
| | | | SEQ ID NO:6940 | SEQ ID NO:14952 | SEQ ID NO:22964 |
| iPS:436252 | 21-225_202A8 | NA | AGCAACAGTGCTGCTTGGAA C | AGGACATACTACAGGTC CAAGTGGTATAATGAGT ATGCAGTATCTGTGAGA AGT | GATCAACGGTACTACGGTAT GGACGTC |
| | | | SEQ ID NO:6941 | SEQ ID NO:14953 | SEQ ID NO:22965 |
| | | AA | SNSAAWN | RTYYRSKWYNEYAVSVRS | DQRYYGMDV |
| | | | SEQ ID NO:6942 | SEQ ID NO:14954 | SEQ ID NO:22966 |
| iPS:436254 | 21-225_202C12 | NA | AGCTATGCCATGAGC | ACTATTAGTGGTAGTGGT GTTAGAACATACTACGC AGACTCCGTGAAGGGC | GGGGGAGCTAGGAGCAGTG GCTGGTTCCACTTTGACTAC |
| | | | SEQ ID NO:6943 | SEQ ID NO:14955 | SEQ ID NO:22967 |

**FIGURE 49**

| | | | | | |
|---|---|---|---|---|---|
| | | AA | SYAMS | TISGSGVRTYYADSVKG | GGARSSGWFHFDY |
| | | | SEQ ID NO:6944 | SEQ ID NO:14956 | SEQ ID NO:22968 |
| **iPS:436256** | **21-225_202D9** | NA | CCTTACTACTGGAGC | GAAATCAATCATAGTGG AAGCACCAACTACAATC CGTCCCTCAAGAGT | GACTACGGGGGTTTAGACTA C |
| | | | SEQ ID NO:6945 | SEQ ID NO:14957 | SEQ ID NO:22969 |
| | | AA | PYYWS | EINHSGSTNYNPSLKS | DYGGLDY |
| | | | SEQ ID NO:6946 | SEQ ID NO:14958 | SEQ ID NO:22970 |
| **iPS:436258** | **21-225_202F12** | NA | TACTATGGCATGCAC | ATTATATGGTATGATGG AAGTAATAAATTCTATG CAGACTCCGTGAAGGGC | AATATAGCAGCAGCTGCCCC TTACTTTGACTAC |
| | | | SEQ ID NO:6947 | SEQ ID NO:14959 | SEQ ID NO:22971 |
| | | AA | YYGMH | IIWYDGSNKFYADSVKG | NIAAAAPYFDY |
| | | | SEQ ID NO:6948 | SEQ ID NO:14960 | SEQ ID NO:22972 |
| **iPS:436260** | **21-225_203H1** | NA | AGCTATGGCATGCAC | GTTATATGGTATGATGG AAGTAATAAAAACTATG CAGACTCCGTGAAGGGC | GATAGAACAGTTGGCTACAA CGGTATGGACGTC |
| | | | SEQ ID NO:6949 | SEQ ID NO:14961 | SEQ ID NO:22973 |
| | | AA | SYGMH | VIWYDGSNKNYADSVKG | DRTVGYNGMDV |
| | | | SEQ ID NO:6950 | SEQ ID NO:14962 | SEQ ID NO:22974 |
| **iPS:436262** | **21-225_203E3** | NA | GGCTACTATATCCAC | TGGATCAACCCTAATAG TGGTGGCACAAACTATG CACAGAAGTTTCAGGGC | GGATACAGCTATGGTTACAA CTGGTTCGACCCC |
| | | | SEQ ID NO:6951 | SEQ ID NO:14963 | SEQ ID NO:22975 |
| | | AA | GYYIH | WINPNSGGTNYAQKFQG | GYSYGYNWFDP |
| | | | SEQ ID NO:6952 | SEQ ID NO:14964 | SEQ ID NO:22976 |

## FIGURE 49

| | | | | | |
|---|---|---|---|---|---|
| **iPS:436264** | **21-225_203F7** | NA | GACTATGTCATGCAC | GTTATATGGTATGATGGAAGTAATAAATACTATGTAGACTCCGTGAAGGGC | GAACGGTATAGCAGTGGCTTGTACGACTACGGTATGGACGTC |
| | | | SEQ ID NO:6953 | SEQ ID NO:14965 | SEQ ID NO:22977 |
| | | AA | DYVMH | VIWYDGSNKYYVDSVKG | ERYSSGLYDYGMDV |
| | | | SEQ ID NO:6954 | SEQ ID NO:14966 | SEQ ID NO:22978 |
| **iPS:436268** | **21-225_203B9** | NA | AGTTTTGGCATGCAC | GTTATATGGTATGATGTAAATAATAAATACTATGCAGACTCCGTGAAGGGC | GAACTGGGGTTCCTCTCTGACTAC |
| | | | SEQ ID NO:6955 | SEQ ID NO:14967 | SEQ ID NO:22979 |
| | | AA | SFGMH | VIWYDVNNKYYADSVKG | ELGFLSDY |
| | | | SEQ ID NO:6956 | SEQ ID NO:14968 | SEQ ID NO:22980 |
| **iPS:436270** | **21-225_203F10** | NA | GACTACTACATGAGC | TACATTAGTGGTAGTGGTACTACCACATACTACGCAGACTCTGTGAAGGGC | GATAGGGGGGGTTTGGACGTC |
| | | | SEQ ID NO:6957 | SEQ ID NO:14969 | SEQ ID NO:22981 |
| | | AA | DYYMS | YISGSGTTTYYADSVKG | DRGGLDV |
| | | | SEQ ID NO:6958 | SEQ ID NO:14970 | SEQ ID NO:22982 |
| **iPS:436272** | **21-225_201F5** | NA | AATTATGATATCAAC | TGGATGCACCCTAACAGTGGTAACACAGGCTATGCACAGAAGTTCCAGGGC | AGCAGTGGCTGGTACTACTTTGACTAC |
| | | | SEQ ID NO:6959 | SEQ ID NO:14971 | SEQ ID NO:22983 |
| | | AA | NYDIN | WMHPNSGNTGYAQKFQG | SSGWYYFDY |
| | | | SEQ ID NO:6960 | SEQ ID NO:14972 | SEQ ID NO:22984 |

FIGURE 49

| | | | | | |
|---|---|---|---|---|---|
| **iPS:436274** | **21-225_204H3** | NA | AGCTATGTCATGCAC | GTTATATGGTATGATGGAAGTAATAAATATAATGCAGACTCCGTGAAGGGC | GAACCGTATAGCAGCAGCTGGTACGACTACGGTATGGACGTC |
| | | | SEQ ID NO:6961 | SEQ ID NO:14973 | SEQ ID NO:22985 |
| | | AA | SYVMH | VIWYDGSNKYNADSVKG | EPYSSSWYDYGMDV |
| | | | SEQ ID NO:6962 | SEQ ID NO:14974 | SEQ ID NO:22986 |
| **iPS:436276** | **21-225_204H4** | NA | GGCTACTATATCCAC | TGGATCAACCCTAATAGTGGTGGCACAAACTATGCACAGAAGTTTCAGGGC | GGATACAGCTATGGTTACAACTGGTTCGACCCC |
| | | | SEQ ID NO:6963 | SEQ ID NO:14975 | SEQ ID NO:22987 |
| | | AA | GYYIH | WINPNSGGTNYAQKFQG | GYSYGYNWFDP |
| | | | SEQ ID NO:6964 | SEQ ID NO:14976 | SEQ ID NO:22988 |
| **iPS:436278** | **21-225_201F2** | NA | AGCAACAGTGCTGCTTGGAAC | AGGACATACTACAGGTCCAAGTGGTATAATTATTATGAAGTATCTGTGAGAAGT | GATCAACGGTACTACGGTATGGACGTC |
| | | | SEQ ID NO:6965 | SEQ ID NO:14977 | SEQ ID NO:22989 |
| | | AA | SNSAAWN | RTYYRSKWYNYYEVSVRS | DQRYYGMDV |
| | | | SEQ ID NO:6966 | SEQ ID NO:14978 | SEQ ID NO:22990 |
| **iPS:436280** | **21-225_204D6** | NA | ACCTATGCCATGAGC | GCTATTAGTGGTAGTGGTGGTAGCACATATTACGCAGACTCCGTGAAGGGC | GGGATAAGTGGAACCGGCTCCTACTACTACTACGGTGTGGACGTC |
| | | | SEQ ID NO:6967 | SEQ ID NO:14979 | SEQ ID NO:22991 |
| | | AA | TYAMS | AISGSGGSTYYADSVKG | GISGTGSYYYYGVDV |
| | | | SEQ ID NO:6968 | SEQ ID NO:14980 | SEQ ID NO:22992 |

FIGURE 49

| | | | | | |
|---|---|---|---|---|---|
| **iPS:436282** | **21-225_204G6** | NA | AGCTATGGCATGCAC | GTTATATGGTATGATGGAAGTAATAAAAACTATGCAGACTCCGTGAAGGGC | GATCGAGGTGTCGGCTACGACGGTATGGACGTC |
| | | | SEQ ID NO:6969 | SEQ ID NO:14981 | SEQ ID NO:22993 |
| | | AA | SYGMH | VIWYDGSNKNYADSVKG | DRGVGYDGMDV |
| | | | SEQ ID NO:6970 | SEQ ID NO:14982 | SEQ ID NO:22994 |
| **iPS:436284** | **21-225_204G8** | NA | AGCTATGGCATGCAC | GTTATATGGTATGATGGTAGCAATGAAAATTATGTAGCCTCCGTGAAGGGC | GATCTGGGGATAGGGTATTACGGTATGGACGTC |
| | | | SEQ ID NO:6971 | SEQ ID NO:14983 | SEQ ID NO:22995 |
| | | AA | SYGMH | VIWYDGSNENYVASVKG | DLGIGYYGMDV |
| | | | SEQ ID NO:6972 | SEQ ID NO:14984 | SEQ ID NO:22996 |
| **iPS:436286** | **21-225_204H8** | NA | GGTTACTTCTGGACC | GAAATCAGTCATAGTGGAAGCACCAGTTACAACCCGTCCCTCAAGAGT | GACTACGGGGCCGACTAC |
| | | | SEQ ID NO:6973 | SEQ ID NO:14985 | SEQ ID NO:22997 |
| | | AA | GYFWT | EISHSGSTSYNPSLKS | DYGADY |
| | | | SEQ ID NO:6974 | SEQ ID NO:14986 | SEQ ID NO:22998 |
| **iPS:436290** | **21-225_205G3** | NA | GGTCACTACTGGAGC | GAAATGTATCATTTTGGAAACACCAACTACAACCCGTCCCTCAAGAGT | GTGGGGCAGTGGCTGGCTTTTGATATC |
| | | | SEQ ID NO:6975 | SEQ ID NO:14987 | SEQ ID NO:22999 |
| | | AA | GHYWS | EMYHFGNTNYNPSLKS | VGQWLAFDI |
| | | | SEQ ID NO:6976 | SEQ ID NO:14988 | SEQ ID NO:23000 |

FIGURE 49

| | | | | | |
|---|---|---|---|---|---|
| **iPS:436292** | **21-225_205H3** | NA | AGCTATGGCATGCAC | GTTATATGGTATGATGGAAGTAATAAATACTATGCAGACTCCGTGAAGGGC | GATAGATCAGTTGGCTACGACGGTACGGACGTC |
| | | | SEQ ID NO:6977 | SEQ ID NO:14989 | SEQ ID NO:23001 |
| | | AA | SYGMH | VIWYDGSNKYYADSVKG | DRSVGYDGTDV |
| | | | SEQ ID NO:6978 | SEQ ID NO:14990 | SEQ ID NO:23002 |
| **iPS:436294** | **21-225_205G4** | NA | AGCAACAGTGCTGCTTGGAAC | AGGACATATTACAGGTCCAAGTGGTATAATTATTATGAAGTATCTGTGAGAAGT | GATCAACGGTACTACGGTATGGACGTC |
| | | | SEQ ID NO:6979 | SEQ ID NO:14991 | SEQ ID NO:23003 |
| | | AA | SNSAAWN | RTYYRSKWYNYYEVSVRS | DQRYYGMDV |
| | | | SEQ ID NO:6980 | SEQ ID NO:14992 | SEQ ID NO:23004 |
| **iPS:436296** | **21-225_205F5** | NA | AGATATGGCATGCAC | GTTATATGGTATGATGGAAGTAATGAGAATTATGTAGACTCCGTGAAGGGC | GATATGGGGATAGGGTATTATGGTATGGACGTC |
| | | | SEQ ID NO:6981 | SEQ ID NO:14993 | SEQ ID NO:23005 |
| | | AA | RYGMH | VIWYDGSNENYVDSVKG | DMGIGYYGMDV |
| | | | SEQ ID NO:6982 | SEQ ID NO:14994 | SEQ ID NO:23006 |
| **iPS:436302** | **21-225_205G7** | NA | GTTTATTACTGGAGC | GAAAGCAATCAGAGTGGACGCACCACCTACAACCCGTCCCTCAAGAGT | GACTACGGTGTCTTTGACTAC |
| | | | SEQ ID NO:6983 | SEQ ID NO:14995 | SEQ ID NO:23007 |
| | | AA | VYYWS | ESNQSGRTTYNPSLKS | DYGVFDY |
| | | | SEQ ID NO:6984 | SEQ ID NO:14996 | SEQ ID NO:23008 |

FIGURE 49

| | | | | | |
|---|---|---|---|---|---|
| **iPS:436304** | **21-225_201F3** | NA | AGCTATGCCATGAGC | ACTATTAGTGGTAGTGGTGTTAGAACATACTACGCAGACTCCGTGAAGGGC | GGGGGAGCTAGGAGCAGTGGCTGGTTCCACTTTGACTAC |
| | | | SEQ ID NO:6985 | SEQ ID NO:14997 | SEQ ID NO:23009 |
| | | AA | SYAMS | TISGSGVRTYYADSVKG | GGARSSGWFHFDY |
| | | | SEQ ID NO:6986 | SEQ ID NO:14998 | SEQ ID NO:23010 |
| **iPS:436306** | **21-225_201H4** | NA | AGCTATGCCATGCAC | GCTATATGGTATGATGGAAGTAATAAATACAATGCAGACTCCGTGAAGGGC | GATGTGGGTACAGTGGGAGCTACCTACTTTGACTGC |
| | | | SEQ ID NO:6987 | SEQ ID NO:14999 | SEQ ID NO:23011 |
| | | AA | SYAMH | AIWYDGSNKYNADSVKG | DVGTVGATYFDC |
| | | | SEQ ID NO:6988 | SEQ ID NO:15000 | SEQ ID NO:23012 |
| **iPS:436308** | **21-225_205H8** | NA | GGTTACTTCTGGAGC | GAAATCAGTCATAGTGGACGCACCAACTACAACCCGTCCCTCAAGAGC | GACTACGGGGCGGACTAC |
| | | | SEQ ID NO:6989 | SEQ ID NO:15001 | SEQ ID NO:23013 |
| | | AA | GYFWS | EISHSGRTNYNPSLKS | DYGADY |
| | | | SEQ ID NO:6990 | SEQ ID NO:15002 | SEQ ID NO:23014 |
| **iPS:436310** | **21-225_202D11** | NA | GGTCCCTACTGGAGC | GAAATCAATCATAGTGGAAGCACCAACTACAACCCGTCCCTCAAGAGT | GACTACGGTGTCCTTGACTAC |
| | | | SEQ ID NO:6991 | SEQ ID NO:15003 | SEQ ID NO:23015 |
| | | AA | GPYWS | EINHSGSTNYNPSLKS | DYGVLDY |
| | | | SEQ ID NO:6992 | SEQ ID NO:15004 | SEQ ID NO:23016 |
| **iPS:436312** | **21-225_206A4** | NA | GGCTACTATATCCAC | TGGATCAACCCTAATAGTGGTGGCACAAACTATGCACAGAAGTTTCAGGGC | GGATACAGCTATGGTTACAACTGGTTCGACCCC |
| | | | SEQ ID NO:6993 | SEQ ID NO:15005 | SEQ ID NO:23017 |

FIGURE 49

| | | | | | |
|---|---|---|---|---|---|
| | | AA | GYYIH | WINPNSGGTNYAQKFQG | GYSYGYNWFDP |
| | | | SEQ ID NO:6994 | SEQ ID NO:15006 | SEQ ID NO:23018 |
| iPS:436314 | 21-225_206G4 | NA | GGCTACTATATACAC | TGGATCGACCCTAATAG TGGTGGCACAAACTATG CACAGAAGTTTCAGGGC | GATCAAGGGTATAACTGGAA CTCTTTTGACTAC |
| | | | SEQ ID NO:6995 | SEQ ID NO:15007 | SEQ ID NO:23019 |
| | | AA | GYYIH | WIDPNSGGTNYAQKFQG | DQGYNWNSFDY |
| | | | SEQ ID NO:6996 | SEQ ID NO:15008 | SEQ ID NO:23020 |
| iPS:436316 | 21-225_206A5 | NA | GGCTACTATATCCAC | TGGATCAACCCTAATAG TGGTGGCACAAACTATG CACAGAAGTTTCAGGGC | GGATACAGCTATGGTTACAA CTGGTTCGACCCC |
| | | | SEQ ID NO:6997 | SEQ ID NO:15009 | SEQ ID NO:23021 |
| | | AA | GYYIH | WINPNSGGTNYAQKFQG | GYSYGYNWFDP |
| | | | SEQ ID NO:6998 | SEQ ID NO:15010 | SEQ ID NO:23022 |
| iPS:436324 | 21-225_207G6 | NA | AGCTATGGCATGCAC | GTTATATGGTATGATGG AAGTAATAAAAACTATG CAGAGTCCGTGAAGGGC | GATGCGGGTATTGGATACTA CGGTATAGACGTC |
| | | | SEQ ID NO:6999 | SEQ ID NO:15011 | SEQ ID NO:23023 |
| | | AA | SYGMH | VIWYDGSNKNYAESVKG | DAGIGYYGIDV |
| | | | SEQ ID NO:7000 | SEQ ID NO:15012 | SEQ ID NO:23024 |
| iPS:436328 | 21-225_207F12 | NA | AACTATGGCATGCAC | GTTATATGGTATGATAG AAATAATAAATACTATG GTGACTCCGTGAAGGGC | GAACTGGGGTTCCTCTTTGA CTAC |
| | | | SEQ ID NO:7001 | SEQ ID NO:15013 | SEQ ID NO:23025 |
| | | AA | NYGMH | VIWYDRNNKYYGDSVKG | ELGFLFDY |
| | | | SEQ ID NO:7002 | SEQ ID NO:15014 | SEQ ID NO:23026 |

FIGURE 49

| | | | | | |
|---|---|---|---|---|---|
| **iPS:436332** | **21-225_208B2** | NA | GACTGTGTCATGCAC | GTTATATGGTATGATGGAAGTAATAAATACTATGTAGACTCCGTGAAGGGC | GAACGGTATAGCAGTGGCTTGTACGACTACGGTTTGGACGTC |
| | | | SEQ ID NO:7003 | SEQ ID NO:15015 | SEQ ID NO:23027 |
| | | AA | DCVMH | VIWYDGSNKYYVDSVKG | ERYSSGLYDYGLDV |
| | | | SEQ ID NO:7004 | SEQ ID NO:15016 | SEQ ID NO:23028 |
| **iPS:436334** | **21-225_208G3** | NA | AGCTATGCCATGAGC | ACTATTAGTGGTAGTGGTGTTAGAACATACTACGCAGACTCCGTGAAGGGC | GGGGGAGCTAGGAGCAGTGGCTGGTTCCACTTTGACTAC |
| | | | SEQ ID NO:7005 | SEQ ID NO:15017 | SEQ ID NO:23029 |
| | | AA | SYAMS | TISGSGVRTYYADSVKG | GGARSSGWFHFDY |
| | | | SEQ ID NO:7006 | SEQ ID NO:15018 | SEQ ID NO:23030 |
| **iPS:436336** | **21-225_208B5** | NA | GTTTACTACTGGACC | GAAATCAATCATAGTGGAAGCACCAACTACAACCCGTCCCTCAAGAGT | GACTATGGTGTCTTTGATTAC |
| | | | SEQ ID NO:7007 | SEQ ID NO:15019 | SEQ ID NO:23031 |
| | | AA | VYYWT | EINHSGSTNYNPSLKS | DYGVFDY |
| | | | SEQ ID NO:7008 | SEQ ID NO:15020 | SEQ ID NO:23032 |
| **iPS:436338** | **21-225_208E8** | NA | GGCTACTATATCCAC | TGGATCAACCCTAATAGTGGTGGCACAAACTATGCACAGAAGTTTCAGGGC | GGATACAGCTATGGTTACAACTGGTTCGACCCC |
| | | | SEQ ID NO:7009 | SEQ ID NO:15021 | SEQ ID NO:23033 |
| | | AA | GYYIH | WINPNSGGTNYAQKFQG | GYSYGYNWFDP |
| | | | SEQ ID NO:7010 | SEQ ID NO:15022 | SEQ ID NO:23034 |
| **iPS:436340** | **21-225_208A9** | NA | GTTTCCTACTGGAGC | GAAATCAATCATAGTGGACGCGCCAACTACAACCCGTCCCTCAAGAGT | GACTACGGTGTCCTTGACTAC |
| | | | SEQ ID NO:7011 | SEQ ID NO:15023 | SEQ ID NO:23035 |

FIGURE 49

| | | | | | |
|---|---|---|---|---|---|
| | | AA | VSYWS | EINHSGRANYNPSLKS | DYGVLDY |
| | | | SEQ ID NO:7012 | SEQ ID NO:15024 | SEQ ID NO:23036 |
| **iPS:436344** | **21-225_208B11** | NA | GGCTACTATATCCAC | TGGATCAACCCTAATAGTGGTGGCACAAACTATGCACAGAAGTTTCAGGGC | GGATACAGCTATGGTTACAACTGGTTCGACCCC |
| | | | SEQ ID NO:7013 | SEQ ID NO:15025 | SEQ ID NO:23037 |
| | | AA | GYYIH | WINPNSGGTNYAQKFQG | GYSYGYNWFDP |
| | | | SEQ ID NO:7014 | SEQ ID NO:15026 | SEQ ID NO:23038 |
| **iPS:436350** | **21-225_210E4** | NA | AACTATGGCATGCAC | GTTATATGGTATGATGAAAATAATAAATACTATGTAGACTCCGTGAAGGGC | GAGACGGGTTTCTTGAGCGACTAC |
| | | | SEQ ID NO:7015 | SEQ ID NO:15027 | SEQ ID NO:23039 |
| | | AA | NYGMH | VIWYDENNKYYVDSVKG | ETGFLSDY |
| | | | SEQ ID NO:7016 | SEQ ID NO:15028 | SEQ ID NO:23040 |
| **iPS:436352** | **21-225_210G5** | NA | GACTGTGTCATGCAC | GTTATATGGTATGATGGAAGTAATAAATACTATGTAGACTCCGTGAAGGGC | GAACGGTATAGCAGTGGCTTGTACGACTACGGTTTGGACGTC |
| | | | SEQ ID NO:7017 | SEQ ID NO:15029 | SEQ ID NO:23041 |
| | | AA | DCVMH | VIWYDGSNKYYVDSVKG | ERYSSGLYDYGLDV |
| | | | SEQ ID NO:7018 | SEQ ID NO:15030 | SEQ ID NO:23042 |
| **iPS:436354** | **21-225_210G10** | NA | AGTTACTACTGGAGC | CGTATCTATACCAGTGGGAGCACCGACTACAACCCCTCCCTCAAGAGT | GGGTTCGGTGACTGGGACTAC |
| | | | SEQ ID NO:7019 | SEQ ID NO:15031 | SEQ ID NO:23043 |
| | | AA | SYYWS | RIYTSGSTDYNPSLKS | GFGDWDY |
| | | | SEQ ID NO:7020 | SEQ ID NO:15032 | SEQ ID NO:23044 |

FIGURE 49

| | | | | | |
|---|---|---|---|---|---|
| **iPS:436356** | **21-225_210H10** | NA | AGCAACAGTGCTGCTTGGAAC | AGGACATACTACAGGTCCAAGTGGTATAATTATTATCCAGTATCTGTGAGAAGT | GATCAACGGTACTACGGTATGGACGTC |
| | | | SEQ ID NO:7021 | SEQ ID NO:15033 | SEQ ID NO:23045 |
| | | AA | SNSAAWN | RTYYRSKWYNYYPVSVRS | DQRYYGMDV |
| | | | SEQ ID NO:7022 | SEQ ID NO:15034 | SEQ ID NO:23046 |
| **iPS:436358** | **21-225_210D11** | NA | GGCTACTATATCCAC | TGGATCAACCCTAATAGTGGTGGCACAAACTATGCACAGAAGTTTCAGGGC | GGATACAGCTATGGTTACAACTGGTTCGACCCC |
| | | | SEQ ID NO:7023 | SEQ ID NO:15035 | SEQ ID NO:23047 |
| | | AA | GYYIH | WINPNSGGTNYAQKFQG | GYSYGYNWFDP |
| | | | SEQ ID NO:7024 | SEQ ID NO:15036 | SEQ ID NO:23048 |
| **iPS:436360** | **21-225_210H11** | NA | AGCCATGGCATGCAC | GTTACATGGTATGATGGAAGTGATAAATACTATGCAGACTCCGTGAAGGGC | GACCGGCTAGTGGGAGCTACTACCGATGCTTTTGATATC |
| | | | SEQ ID NO:7025 | SEQ ID NO:15037 | SEQ ID NO:23049 |
| | | AA | SHGMH | VTWYDGSDKYYADSVKG | DRLVGATTDAFDI |
| | | | SEQ ID NO:7026 | SEQ ID NO:15038 | SEQ ID NO:23050 |
| **iPS:436362** | **21-225_210C12** | NA | AACAATGGTATCAGC | TGGATCAACGCTTACAATGGTCACACAAACTATGCACAGAAGTTCCAGGGC | GATCCTACGGTGACCCACTACTATTACTACGGTATGGACGTC |
| | | | SEQ ID NO:7027 | SEQ ID NO:15039 | SEQ ID NO:23051 |
| | | AA | NNGIS | WINAYNGHTNYAQKFQG | DPTVTHYYYYGMDV |
| | | | SEQ ID NO:7028 | SEQ ID NO:15040 | SEQ ID NO:23052 |

FIGURE 49

| | | | | | |
|---|---|---|---|---|---|
| **iPS:436364** | **21-225_211A11** | NA | AGCTATGGCATGCAC | GTTCTTTGGTTTGATGGAAGTAATAGAAACTATGCAGACTCCGTGAAGGGC | GATCGGGGAGTGGGCTACTACGGTACGGACGTC |
| | | | SEQ ID NO:7029 | SEQ ID NO:15041 | SEQ ID NO:23053 |
| | | AA | SYGMH | VLWFDGSNRNYADSVKG | DRGVGYYGTDV |
| | | | SEQ ID NO:7030 | SEQ ID NO:15042 | SEQ ID NO:23054 |
| **iPS:436366** | **21-225_211A3** | NA | AGTTATGGCATGCAC | GTTATATGGTATGATGGAAGTAATAAAAACTATGAAGACTCCGTGAAGGGC | GATGGGAGTTATGGTTACGACGGTATGGACGTC |
| | | | SEQ ID NO:7031 | SEQ ID NO:15043 | SEQ ID NO:23055 |
| | | AA | SYGMH | VIWYDGSNKNYEDSVKG | DGSYGYDGMDV |
| | | | SEQ ID NO:7032 | SEQ ID NO:15044 | SEQ ID NO:23056 |
| **iPS:436368** | **21-225_211G3** | NA | AACTATGGCATGCAC | GTTATATGGCATGATGGAAGTAATAAATACTATGCAGACTCCGTGAAGGGC | TTAGACTACAGTAACTACGGGTGGTTCGACCCC |
| | | | SEQ ID NO:7033 | SEQ ID NO:15045 | SEQ ID NO:23057 |
| | | AA | NYGMH | VIWHDGSNKYYADSVKG | LDYSNYGWFDP |
| | | | SEQ ID NO:7034 | SEQ ID NO:15046 | SEQ ID NO:23058 |
| **iPS:436370** | **21-225_211A6** | NA | AGCTATGGCATGCAC | GTTATATGGTATGATGGAAGTAATAAAAACTATGCAGACTCCGTGAAGGGC | GATAGGACGGTGGGCTATGATGGTTTTGATATC |
| | | | SEQ ID NO:7035 | SEQ ID NO:15047 | SEQ ID NO:23059 |
| | | AA | SYGMH | VIWYDGSNKNYADSVKG | DRTVGYDGFDI |

FIGURE 49

| | | | | | |
|---|---|---|---|---|---|
| | | | SEQ ID NO:7036 | SEQ ID NO:15048 | SEQ ID NO:23060 |
| **iPS:436372** | **21-225_211A8** | NA | AGCTATGGCATGCAC | GTTATATGGTATGATGGAAGTAATGAACACTATGCAGACTCCGTGAAGGGC | GACCACGGTGTCGGGTACTACGGTATGGACGTC |
| | | | SEQ ID NO:7037 | SEQ ID NO:15049 | SEQ ID NO:23061 |
| | | AA | SYGMH | VIWYDGSNEHYADSVKG | DHGVGYYGMDV |
| | | | SEQ ID NO:7038 | SEQ ID NO:15050 | SEQ ID NO:23062 |
| **iPS:436374** | **21-225_211C10** | NA | AGGCATGGTATCAGC | TGGATCAGCGCTTACAATGGTCTCACAAACTATGCACAGAAGTTCCAGGGC | GATCCTACGGTGACCCACTACTACTACTACGGTATGGACGTC |
| | | | SEQ ID NO:7039 | SEQ ID NO:15051 | SEQ ID NO:23063 |
| | | AA | RHGIS | WISAYNGLTNYAQKFQG | DPTVTHYYYYGMDV |
| | | | SEQ ID NO:7040 | SEQ ID NO:15052 | SEQ ID NO:23064 |
| **iPS:436376** | **21-225_212E6** | NA | AGCTATGGCATGCAC | GTTATATGGTATGATGGAAGTAATAAAAACTATGTAGACTCCGTGAAGGGC | GACTACGGTGTCGGGTACTACGGTACGGACGTC |
| | | | SEQ ID NO:7041 | SEQ ID NO:15053 | SEQ ID NO:23065 |
| | | AA | SYGMH | VIWYDGSNKNYVDSVKG | DYGVGYYGTDV |
| | | | SEQ ID NO:7042 | SEQ ID NO:15054 | SEQ ID NO:23066 |
| **iPS:436378** | **21-225_212D7** | NA | AGCTATGGCATGCAC | GTTATATGGTATGATGGAAGTAATAAAAATTATGCAGACTCCGTGAAGGGC | GACTACGGTGTCGGGTACTACGGTACGGACGTC |
| | | | SEQ ID NO:7043 | SEQ ID NO:15055 | SEQ ID NO:23067 |
| | | AA | SYGMH | VIWYDGSNKNYADSVKG | DYGVGYYGTDV |

FIGURE 49

| | | | | | |
|---|---|---|---|---|---|
| | | | SEQ ID NO:7044 | SEQ ID NO:15056 | SEQ ID NO:23068 |
| iPS:436380 | 21-225_212H9 | NA | AGCTATGGCATGCAC | GTTATATGGTATGATGGAAGTAATGAACACTATGCAGACTCCGTGAAGGGC | GACCACGGTGTCGGGTACTACGGTATGGACGTC |
| | | | SEQ ID NO:7045 | SEQ ID NO:15057 | SEQ ID NO:23069 |
| | | AA | SYGMH | VIWYDGSNEHYADSVKG | DHGVGYYGMDV |
| | | | SEQ ID NO:7046 | SEQ ID NO:15058 | SEQ ID NO:23070 |
| iPS:436382 | 21-225_212C10 | NA | AGCTATGGCATGCAC | GCTATATGGTATGATGGAAGTCATAAATACTATACAGATTCCGTGAAGGGC | GATCGGAGTATAGTGGGAGCTACCTACTTTGACTAC |
| | | | SEQ ID NO:7047 | SEQ ID NO:15059 | SEQ ID NO:23071 |
| | | AA | SYGMH | AIWYDGSHKYYTDSVKG | DRSIVGATYFDY |
| | | | SEQ ID NO:7048 | SEQ ID NO:15060 | SEQ ID NO:23072 |
| iPS:436384 | 21-225_212F10 | NA | AGATATGGCATGCAC | GTTATATGGTATGATGGAAGTAATAAACACTATGCAGACTCCGTGAAGGGC | GATCGGGGAGTGGGCTACAACGGTATGGACGTC |
| | | | SEQ ID NO:7049 | SEQ ID NO:15061 | SEQ ID NO:23073 |
| | | AA | RYGMH | VIWYDGSNKHYADSVKG | DRGVGYNGMDV |
| | | | SEQ ID NO:7050 | SEQ ID NO:15062 | SEQ ID NO:23074 |
| iPS:436386 | 21-225_212B11 | NA | GACTATGTCATGCAC | GTTATATGGTATGATGGAAGTAATAAATATTATGCAGACTCCGTGAAGGGC | GAACGTTATAGCAGTGGCTGGTACGACTACGGTATGGACGTC |
| | | | SEQ ID NO:7051 | SEQ ID NO:15063 | SEQ ID NO:23075 |

FIGURE 49

| | | | | | |
|---|---|---|---|---|---|
| | | AA | DYVMH | VIWYDGSNKYYADSVKG | ERYSSGWYDYGMDV |
| | | | SEQ ID NO:7052 | SEQ ID NO:15064 | SEQ ID NO:23076 |
| **iPS:436388** | **21-225_212H11** | NA | AGTTATGGCATGCAC | GTTATATGGTATGATGGAAGTAATAAAAACTATGAAGACTCCGTGAAGGGC | GATGGGAGTTATGGTTACGACGGTATGGACGTC |
| | | | SEQ ID NO:7053 | SEQ ID NO:15065 | SEQ ID NO:23077 |
| | | AA | SYGMH | VIWYDGSNKNYEDSVKG | DGSYGYDGMDV |
| | | | SEQ ID NO:7054 | SEQ ID NO:15066 | SEQ ID NO:23078 |
| **iPS:436390** | **21-225_213D2** | NA | AGCTATGGCATGCAC | GTTATATGGTATGATGGAAGTAATAAAAATTATGCAGACTCCGTGAAGGGC | GACTACGGTGTCGGGTACTACGGTACGGACGTC |
| | | | SEQ ID NO:7055 | SEQ ID NO:15067 | SEQ ID NO:23079 |
| | | AA | SYGMH | VIWYDGSNKNYADSVKG | DYGVGYYGTDV |
| | | | SEQ ID NO:7056 | SEQ ID NO:15068 | SEQ ID NO:23080 |
| **iPS:436392** | **21-225_213B3** | NA | AGCTATGGCATGCAC | GTTATATGGTATGATGGAAGTAATAAAAACTATGCAGACTCCGTGAAGGGC | GATAGGACGGTGGGCTATGATGGTTTTGATATC |
| | | | SEQ ID NO:7057 | SEQ ID NO:15069 | SEQ ID NO:23081 |
| | | AA | SYGMH | VIWYDGSNKNYADSVKG | DRTVGYDGFDI |
| | | | SEQ ID NO:7058 | SEQ ID NO:15070 | SEQ ID NO:23082 |

# FIGURE 49

| | | | | | |
|---|---|---|---|---|---|
| **iPS:436394** | **21-225_213C4** | NA | AGCTATGGCATGCAC | GTTATATGGTATGATGGAAGTAATAAACACTATGCAGACTCCGTGAAGGGC | GACTACGGTGTCGGGTACGACGGTATGGACGTC |
| | | | SEQ ID NO:7059 | SEQ ID NO:15071 | SEQ ID NO:23083 |
| | | AA | SYGMH | VIWYDGSNKHYADSVKG | DYGVGYDGMDV |
| | | | SEQ ID NO:7060 | SEQ ID NO:15072 | SEQ ID NO:23084 |
| **iPS:436396** | **21-225_213E5** | NA | AGTTATGGCATGCAC | GTTATATGGTATGATGGAAGTAATAAAAACTATGAAGACTCCGTGAAGGGC | GATGGGAGTTATGGTTACGACGGTATGGACGTC |
| | | | SEQ ID NO:7061 | SEQ ID NO:15073 | SEQ ID NO:23085 |
| | | AA | SYGMH | VIWYDGSNKNYEDSVKG | DGSYGYDGMDV |
| | | | SEQ ID NO:7062 | SEQ ID NO:15074 | SEQ ID NO:23086 |
| **iPS:436398** | **21-225_213B8** | NA | AGCTATGGCATGCAC | GTTATATGGTATGATGGAAGTAATAAACACTATGCAGACTCCGTGAAGGGC | GACTACGGTGTCGGGTACTACGGTACGGACGTC |
| | | | SEQ ID NO:7063 | SEQ ID NO:15075 | SEQ ID NO:23087 |
| | | AA | SYGMH | VIWYDGSNKHYADSVKG | DYGVGYYGTDV |
| | | | SEQ ID NO:7064 | SEQ ID NO:15076 | SEQ ID NO:23088 |
| **iPS:436400** | **21-225_213H7** | NA | GGCTACCATATGCAC | TGGATCAATCCTAAGAGTGATGGCACAAACTATGCACAGAAGTTTCAGGGC | GAAAAGCCTGGGAGCTACTACAAATAC |
| | | | SEQ ID NO:7065 | SEQ ID NO:15077 | SEQ ID NO:23089 |
| | | AA | GYHMH | WINPKSDGTNYAQKFQG | EKPGSYYKY |
| | | | SEQ ID NO:7066 | SEQ ID NO:15078 | SEQ ID NO:23090 |

FIGURE 49

| | | | | | |
|---|---|---|---|---|---|
| **iPS:436402** | **21-225_213H12** | NA | AGCTATGGTATCAAC | TGGATCAGCGTTCACAATGGTAACACAGACTATGCACAGAAGTTCCAGGGC | GACTACTACTACGGTATGGACGTC |
| | | | SEQ ID NO:7067 | SEQ ID NO:15079 | SEQ ID NO:23091 |
| | | AA | SYGIN | WISVHNGNTDYAQKFQG | DYYYGMDV |
| | | | SEQ ID NO:7068 | SEQ ID NO:15080 | SEQ ID NO:23092 |
| **iPS:436404** | **21-225_214C3** | NA | AGCTATGGCATGCAC | GTTATATGGTATGATGGAAGTAATAAAAACTATGGAGACTCCGTGAAGGGC | GATCGGGGAGTGGGCTACGACGGAATGGACGTC |
| | | | SEQ ID NO:7069 | SEQ ID NO:15081 | SEQ ID NO:23093 |
| | | AA | SYGMH | VIWYDGSNKNYGDSVKG | DRGVGYDGMDV |
| | | | SEQ ID NO:7070 | SEQ ID NO:15082 | SEQ ID NO:23094 |
| **iPS:436406** | **21-225_214E4** | NA | AGCTATGGCATGCAC | GTTATATGGTATGATGGAAGTAATGAAAACTATGCAGACTCCGTGAAGGGC | GATAGGACGGTGGGCTATGATGGTTGTGATATC |
| | | | SEQ ID NO:7071 | SEQ ID NO:15083 | SEQ ID NO:23095 |
| | | AA | SYGMH | VIWYDGSNENYADSVKG | DRTVGYDGCDI |
| | | | SEQ ID NO:7072 | SEQ ID NO:15084 | SEQ ID NO:23096 |
| **iPS:436408** | **21-225_214H8** | NA | GGCCACTATATACAC | TGGATCAACTCTAACAGTGGTGGCACAAACTATGCACAGAAGTTTCAGGGC | GACGGGAGATACAGCTATGGTTACGACTGGTTCGACCCC |
| | | | SEQ ID NO:7073 | SEQ ID NO:15085 | SEQ ID NO:23097 |
| | | AA | GHYIH | WINSNSGGTNYAQKFQG | DGRYSYGYDWFDP |
| | | | SEQ ID NO:7074 | SEQ ID NO:15086 | SEQ ID NO:23098 |

FIGURE 49

| | | | | | |
|---|---|---|---|---|---|
| **iPS:436410** | **21-225_212E10** | NA | AGCTATGGCATGCAC | GTTATATGGTATGATGGAAGTAATAAATACTATGCAGACTCCGTGAAGGGC | GACTACGGTGTCGGGTACTACGGTACGGACGTC |
| | | | SEQ ID NO:7075 | SEQ ID NO:15087 | SEQ ID NO:23099 |
| | | AA | SYGMH | VIWYDGSNKYYADSVKG | DYGVGYYGTDV |
| | | | SEQ ID NO:7076 | SEQ ID NO:15088 | SEQ ID NO:23100 |
| **iPS:436412** | **21-225_214H9** | NA | AGCTATGTCATGCAC | GTTATATGGTATGATGGAAGTAATAAATACTATGCAGACTCCGTGAAGGGC | GAGAGGTATACCAGCAGCTGGTACGACTACGGTATGGACGTC |
| | | | SEQ ID NO:7077 | SEQ ID NO:15089 | SEQ ID NO:23101 |
| | | AA | SYVMH | VIWYDGSNKYYADSVKG | ERYTSSWYDYGMDV |
| | | | SEQ ID NO:7078 | SEQ ID NO:15090 | SEQ ID NO:23102 |
| **iPS:436414** | **21-225_214G10** | NA | GACTATGTCATGCAC | GTTATATGGTATGATGGAAGTAATAAATATTATGCAGACTCCGTGAAGGGC | GAACGTTATAGCAGTGGCTGGTACGACTACGGTATGGACGTC |
| | | | SEQ ID NO:7079 | SEQ ID NO:15091 | SEQ ID NO:23103 |
| | | AA | DYVMH | VIWYDGSNKYYADSVKG | ERYSSGWYDYGMDV |
| | | | SEQ ID NO:7080 | SEQ ID NO:15092 | SEQ ID NO:23104 |
| **iPS:436416** | **21-225_214G12** | NA | GACTATGTCATGCAC | GTTATATGGTATGATGGAAGTAATAAATATTATGCAGACTCCGTGAAGGGC | GAACGTTATAGCAGTGGCTGGTACGACTACGGTATGGACGTC |
| | | | SEQ ID NO:7081 | SEQ ID NO:15093 | SEQ ID NO:23105 |
| | | AA | DYVMH | VIWYDGSNKYYADSVKG | ERYSSGWYDYGMDV |
| | | | SEQ ID NO:7082 | SEQ ID NO:15094 | SEQ ID NO:23106 |

FIGURE 49

| | | | | | |
|---|---|---|---|---|---|
| **iPS:436418** | **21-225_215E3** | NA | GACTATGTCATACAC | GTTATATGGTATGATGGAAGTAATAAATATTATGCAGACTCCGTGAAGGGC | GAACGTTATAGCAGTGGCTGGTACGACTACGGTATGGACGTC |
| | | | SEQ ID NO:7083 | SEQ ID NO:15095 | SEQ ID NO:23107 |
| | | AA | DYVIH | VIWYDGSNKYYADSVKG | ERYSSGWYDYGMDV |
| | | | SEQ ID NO:7084 | SEQ ID NO:15096 | SEQ ID NO:23108 |
| **iPS:436420** | **21-225_215B5** | NA | AGCTATGGCATGCAC | GTTATATGGTATGATGGAAGTAATAAAAATTATGCAGACTCCGTGAAGGGC | GACTACGGTGTCGGGTACTACGGTACGGACGTC |
| | | | SEQ ID NO:7085 | SEQ ID NO:15097 | SEQ ID NO:23109 |
| | | AA | SYGMH | VIWYDGSNKNYADSVKG | DYGVGYYGTDV |
| | | | SEQ ID NO:7086 | SEQ ID NO:15098 | SEQ ID NO:23110 |
| **iPS:436422** | **21-225_215D6** | NA | AGCTATGGCATGCAC | GTTATATGGTATGATGGAAGTAATAAAAACTATGCAGATTCCGTGAAGGGC | GACTGCGGTGTCGGATACTACGGTACGGACGTC |
| | | | SEQ ID NO:7087 | SEQ ID NO:15099 | SEQ ID NO:23111 |
| | | AA | SYGMH | VIWYDGSNKNYADSVKG | DCGVGYYGTDV |
| | | | SEQ ID NO:7088 | SEQ ID NO:15100 | SEQ ID NO:23112 |
| **iPS:436424** | **21-225_215H6** | NA | GGCCACTATATACAC | TGGATCAACTCTAACAGTGGTGGCACAAATTATGCAGAGAAGTTTCAGGGC | GACGGGAGATACAGCTATGGTCACGACTGGTTCGACCCC |
| | | | SEQ ID NO:7089 | SEQ ID NO:15101 | SEQ ID NO:23113 |
| | | AA | GHYIH | WINSNSGGTNYAEKFQG | DGRYSYGHDWFDP |
| | | | SEQ ID NO:7090 | SEQ ID NO:15102 | SEQ ID NO:23114 |

FIGURE 49

| | | | | | |
|---|---|---|---|---|---|
| **iPS:436426** | **21-225_215C7** | NA | TACTATGGCATGCAC | GTTATATGGCATGATGGAAGTAATAAATACTATGCAGACTCCGTGAAGGGC | TTAGACTACAGTAATTACGGGTGGTTCGACCCC |
| | | | SEQ ID NO:7091 | SEQ ID NO:15103 | SEQ ID NO:23115 |
| | | AA | YYGMH | VIWHDGSNKYYADSVKG | LDYSNYGWFDP |
| | | | SEQ ID NO:7092 | SEQ ID NO:15104 | SEQ ID NO:23116 |
| **iPS:436428** | **21-225_215E11** | NA | GACTATGTCATGCAC | GTTATATGGTATGATGGAAGTAATAAATATTATGCAGACTCCGTGAAGGGC | GAACGTTATAGCAGTGGCTGGTACGACTACGGTATGGACGTC |
| | | | SEQ ID NO:7093 | SEQ ID NO:15105 | SEQ ID NO:23117 |
| | | AA | DYVMH | VIWYDGSNKYYADSVKG | ERYSSGWYDYGMDV |
| | | | SEQ ID NO:7094 | SEQ ID NO:15106 | SEQ ID NO:23118 |
| **iPS:436430** | **21-225_215A12** | NA | AGCTATGGCATGCAC | GTTATATGGTATGATGGAAGTAATGAACACTATGCAGACTCCGTGAAGGGC | GATCGGGGAGTGGGCTACTACGGTATGGACGTC |
| | | | SEQ ID NO:7095 | SEQ ID NO:15107 | SEQ ID NO:23119 |
| | | AA | SYGMH | VIWYDGSNEHYADSVKG | DRGVGYYGMDV |
| | | | SEQ ID NO:7096 | SEQ ID NO:15108 | SEQ ID NO:23120 |
| **iPS:436432** | **21-225_215H12** | NA | AACTATGGCATGCAC | GTTATATGGCATGATGGAAGTAATAAATACTATGCAGACTCCGTGAAGGGC | TTAGACTACAGTAACTACGGGTGGTTCGACCCC |
| | | | SEQ ID NO:7097 | SEQ ID NO:15109 | SEQ ID NO:23121 |
| | | AA | NYGMH | VIWHDGSNKYYADSVKG | LDYSNYGWFDP |

**FIGURE 49**

| | | | | | |
|---|---|---|---|---|---|
| | | | SEQ ID NO:7098 | SEQ ID NO:15110 | SEQ ID NO:23122 |
| **iPS:436434** | **21-225_216B10** | NA | AGCTATGGCATGCAC | GCTATATGGTATGATGGAAGTAATAAATACTATGCAGACTCCGTGAAGGGC | GATCCCAACATAGTGGGAGCTACTTGGTTTGACTAC |
| | | | SEQ ID NO:7099 | SEQ ID NO:15111 | SEQ ID NO:23123 |
| | | AA | SYGMH | AIWYDGSNKYYADSVKG | DPNIVGATWFDY |
| | | | SEQ ID NO:7100 | SEQ ID NO:15112 | SEQ ID NO:23124 |
| **iPS:436436** | **21-225_216F10** | NA | AGCTATAGCATGAAC | TACATTACTGGTAGTAGTAGTACCATATACTACGCAGACTCTGTGAAGGGC | TCGGGTTTAGCAGTGGAGGACTAC |
| | | | SEQ ID NO:7101 | SEQ ID NO:15113 | SEQ ID NO:23125 |
| | | AA | SYSMN | YITGSSSTIYYADSVKG | SGLAVEDY |
| | | | SEQ ID NO:7102 | SEQ ID NO:15114 | SEQ ID NO:23126 |
| **iPS:436438** | **21-225_216E8** | NA | GACTATGTCATGCAC | GTTATATGGTATGATGGAAGTAATAAATATTATGCAGACTCCGTGAAGGGC | GAACGTTATAGCAGTGGCTGGTACGACTACGGTATGGACGTC |
| | | | SEQ ID NO:7103 | SEQ ID NO:15115 | SEQ ID NO:23127 |
| | | AA | DYVMH | VIWYDGSNKYYADSVKG | ERYSSGWYDYGMDV |
| | | | SEQ ID NO:7104 | SEQ ID NO:15116 | SEQ ID NO:23128 |
| **iPS:436440** | **21-225_216H12** | NA | GACTATGTCATGCAC | GTTATATGGTATGATGGAAGTAATAAATATTATGCAGACTCCGTGAAGGGC | GAACGTTATAGCAGTGGCTGGTACGACTACGGTATGGACGTC |
| | | | SEQ ID NO:7105 | SEQ ID NO:15117 | SEQ ID NO:23129 |
| | | AA | DYVMH | VIWYDGSNKYYADSVKG | ERYSSGWYDYGMDV |
| | | | SEQ ID NO:7106 | SEQ ID NO:15118 | SEQ ID NO:23130 |

FIGURE 49

| | | | | | |
|---|---|---|---|---|---|
| **iPS:436448** | **21-225_217A3** | NA | AGCTATAATATGAAC | TACATTAGTAGTAGTCGTAATATCATATATTACGCAGACTCTGTGAAGGGC | GATGGCTCTTATAGCAGTGGCTGGTACTGGGGTTTTGACTAC |
| | | | SEQ ID NO:7107 | SEQ ID NO:15119 | SEQ ID NO:23131 |
| | | AA | SYNMN | YISSSRNIIYYADSVKG | DGSYSSGWYWGFDY |
| | | | SEQ ID NO:7108 | SEQ ID NO:15120 | SEQ ID NO:23132 |
| **iPS:436450** | **21-225_217E5** | NA | GACTATGTCATGCAC | GTTATATGGTATGATGGAAGTAATAAATATTATGCAGACTCCGTGAAGGGC | GAACGTTATAGCAGTGGCTGGTACGACTACGGTATGGACGTC |
| | | | SEQ ID NO:7109 | SEQ ID NO:15121 | SEQ ID NO:23133 |
| | | AA | DYVMH | VIWYDGSNKYYADSVKG | ERYSSGWYDYGMDV |
| | | | SEQ ID NO:7110 | SEQ ID NO:15122 | SEQ ID NO:23134 |
| **iPS:436452** | **21-225_217G5** | NA | AGCAATGGCATGCAC | GTTATATGGTATGATGGAAGTAATAAAAACTATGCAGACTCCGTGAAGGGC | GACTACGGTGTCGGGTACTACGGTCTGGACGTC |
| | | | SEQ ID NO:7111 | SEQ ID NO:15123 | SEQ ID NO:23135 |
| | | AA | SNGMH | VIWYDGSNKNYADSVKG | DYGVGYYGLDV |
| | | | SEQ ID NO:7112 | SEQ ID NO:15124 | SEQ ID NO:23136 |
| **iPS:436454** | **21-225_217B10** | NA | AGTTATGGCATGCAC | GTTATATGGTATGATGGAAGTAATAAAAACTATGAAGACTCCGTGAAGGGC | GATGGGAGTTATGGTTACGACGGTATGGACGTC |
| | | | SEQ ID NO:7113 | SEQ ID NO:15125 | SEQ ID NO:23137 |
| | | AA | SYGMH | VIWYDGSNKNYEDSVKG | DGSYGYDGMDV |
| | | | SEQ ID NO:7114 | SEQ ID NO:15126 | SEQ ID NO:23138 |

FIGURE 49

| | | | | | |
|---|---|---|---|---|---|
| **iPS:436456** | **21-225_217G10** | NA | GACTATGTCATGCAC | GTTATATGGTATGATGGAAGTAATAAATATTATGCAGACTCCGTGAAGGGC | GAACGTTATAGCAGTGGCTGGTACGACTACGGTATGGACGTC |
| | | | SEQ ID NO:7115 | SEQ ID NO:15127 | SEQ ID NO:23139 |
| | | AA | DYVMH | VIWYDGSNKYYADSVKG | ERYSSGWYDYGMDV |
| | | | SEQ ID NO:7116 | SEQ ID NO:15128 | SEQ ID NO:23140 |
| **iPS:436458** | **21-225_217H12** | NA | GACTATGTCATGCAC | GTTATATGGTATGATGGAAGTAATAAATATTATGCAGACTCCGTGAAGGGC | GAACGTTATAGCAGTGGCTGGTACGACTACGGTATGGACGTC |
| | | | SEQ ID NO:7117 | SEQ ID NO:15129 | SEQ ID NO:23141 |
| | | AA | DYVMH | VIWYDGSNKYYADSVKG | ERYSSGWYDYGMDV |
| | | | SEQ ID NO:7118 | SEQ ID NO:15130 | SEQ ID NO:23142 |
| **iPS:436462** | **21-225_218C4** | NA | GACTATGTCATGCAC | GTTATATGGTATGATGGAAGTAATAAATATTATGCAGACTCCGTGAAGGGC | GAACGTTATAGCAGTGGCTGGTACGACTACGGTATGGACGTC |
| | | | SEQ ID NO:7119 | SEQ ID NO:15131 | SEQ ID NO:23143 |
| | | AA | DYVMH | VIWYDGSNKYYADSVKG | ERYSSGWYDYGMDV |
| | | | SEQ ID NO:7120 | SEQ ID NO:15132 | SEQ ID NO:23144 |
| **iPS:436464** | **21-225_219H1** | NA | AGATATGGCATGCAC | GTTATATGGTATGATGGAAGTAATAAACACTATGCAGACTCCGTGAAGGGC | GATCGGGGAGTGGGCTACAACGGTATGGACGTC |
| | | | SEQ ID NO:7121 | SEQ ID NO:15133 | SEQ ID NO:23145 |
| | | AA | RYGMH | VIWYDGSNKHYADSVKG | DRGVGYNGMDV |
| | | | SEQ ID NO:7122 | SEQ ID NO:15134 | SEQ ID NO:23146 |

FIGURE 49

| | | | | | |
|---|---|---|---|---|---|
| **iPS:436472** | **21-225_220E1** | NA | ACTTACTACTGGAGC | TATATCTATTACAGTGGGACCACCAACTACAACCCCTCCCTCAAGAGT | GACCAGCAGTGGCTGGTACGTGGGAGGGACAACTACTACTACGGTATGGACGTC |
| | | | SEQ ID NO:7123 | SEQ ID NO:15135 | SEQ ID NO:23147 |
| | | AA | TYYWS | YIYYSGTTNYNPSLKS | DQQWLVRGRDNYYYGMDV |
| | | | SEQ ID NO:7124 | SEQ ID NO:15136 | SEQ ID NO:23148 |
| **iPS:436480** | **21-225_220F8** | NA | GACTATGTCATGCAC | GTTATATGGTATGATGGAAGTAATAAATATTATGCAGACTCCGTGAAGGGC | GAACGTTATAGCAGTGGCTGGTACGACTACGGTATGGACGTC |
| | | | SEQ ID NO:7125 | SEQ ID NO:15137 | SEQ ID NO:23149 |
| | | AA | DYVMH | VIWYDGSNKYYADSVKG | ERYSSGWYDYGMDV |
| | | | SEQ ID NO:7126 | SEQ ID NO:15138 | SEQ ID NO:23150 |
| **iPS:436488** | **21-225_221A6** | NA | GGCTACTATATGCAC | TGGATCCACCCTAACAGTGGTGGCACAAACTATGCACAGAAATTTCAGGGC | GATGGGACCAGCTCGTTTGACTAC |
| | | | SEQ ID NO:7127 | SEQ ID NO:15139 | SEQ ID NO:23151 |
| | | AA | GYYMH | WIHPNSGGTNYAQKFQG | DGTSSFDY |
| | | | SEQ ID NO:7128 | SEQ ID NO:15140 | SEQ ID NO:23152 |
| **iPS:436490** | **21-225_221F6** | NA | AGCAATGGCATGCAC | GTTATATGGTACGATGGAAGTAATGAAAACTATGCAGACTCCGTGAAGGGC | GATCGGACAGTGGGCTACAACGGTATGGACGTC |
| | | | SEQ ID NO:7129 | SEQ ID NO:15141 | SEQ ID NO:23153 |
| | | AA | SNGMH | VIWYDGSNENYADSVKG | DRTVGYNGMDV |
| | | | SEQ ID NO:7130 | SEQ ID NO:15142 | SEQ ID NO:23154 |
| **iPS:436496** | **21 225 222E1** | NA | GGCTACTATATGCAC | TGGATCCACCCTAACAGTGGTGGCACAAACTATGCACAGAAATTTCAGGGC | GATGGGACCAGCTCGTTTGACTAC |

FIGURE 49

| | | | | | |
|---|---|---|---|---|---|
| | 21-225_222E1 | | SEQ ID NO:7131 | SEQ ID NO:15143 | SEQ ID NO:23155 |
| | | AA | GYYMH | WIHPNSGGTNYAQKFQG | DGTSSFDY |
| | | | SEQ ID NO:7132 | SEQ ID NO:15144 | SEQ ID NO:23156 |
| iPS:436500 | 21-225_222H3 | NA | AGCTATGGTATCAAC | TGGATCAGCGTTTACAATGGTAACACAAACTATGCACAGAAGCTCCAGGGC | GACTACTACTACGGTTTTGACGTC |
| | | | SEQ ID NO:7133 | SEQ ID NO:15145 | SEQ ID NO:23157 |
| | | AA | SYGIN | WISVYNGNTNYAQKLQG | DYYYGFDV |
| | | | SEQ ID NO:7134 | SEQ ID NO:15146 | SEQ ID NO:23158 |
| iPS:436502 | 21-225_222A11 | NA | AGCTATGGCATGCAC | GTTATATGGTATGATGGAAGTAATAAAAACTATGCAGACTCCGTGAAGGGC | GATCGGGATGTCGGGTACAACGGTATGGACGTC |
| | | | SEQ ID NO:7135 | SEQ ID NO:15147 | SEQ ID NO:23159 |
| | | AA | SYGMH | VIWYDGSNKNYADSVKG | DRDVGYNGMDV |
| | | | SEQ ID NO:7136 | SEQ ID NO:15148 | SEQ ID NO:23160 |
| iPS:436504 | 21-225_222H4 | NA | AACTTTGCCATGAGT | AGTATTGTTGGTAGTGGTGGTCGCACGTACTACGCAGACTCCGTGAAGGGC | GACCCTTATCGTGTAGCAGTGGCTGGGGCCTTTGACTAC |
| | | | SEQ ID NO:7137 | SEQ ID NO:15149 | SEQ ID NO:23161 |
| | | AA | NFAMS | SIVGSGGRTYYADSVKG | DPYRVAVAGAFDY |
| | | | SEQ ID NO:7138 | SEQ ID NO:15150 | SEQ ID NO:23162 |
| iPS:436506 | 21-225_222C7 | NA | GGTCGCTACTGGAGC | GAAATCAATCATAGTGGAAGCGCCAACTACAACCCGTCCCTCAAGAGT | GACTACGGCGCCCTTGATTTC |
| | | | SEQ ID NO:7139 | SEQ ID NO:15151 | SEQ ID NO:23163 |
| | | AA | GRYWS | EINHSGSANYNPSLKS | DYGALDF |

FIGURE 49

| | | | | | |
|---|---|---|---|---|---|
| | | | SEQ ID NO:7140 | SEQ ID NO:15152 | SEQ ID NO:23164 |
| **iPS:436508** | **21-225_222F7** | NA | GGCTACTATATGCAC | TGGATCCACCCTAACAGTGGTGGCACAAACTATGCACAGAAATTTCAGGGC | GATGGGACCAGCTCGTTTGACTAC |
| | | | SEQ ID NO:7141 | SEQ ID NO:15153 | SEQ ID NO:23165 |
| | | AA | GYYMH | WIHPNSGGTNYAQKFQG | DGTSSFDY |
| | | | SEQ ID NO:7142 | SEQ ID NO:15154 | SEQ ID NO:23166 |
| **iPS:436510** | **21-225_222H8** | NA | AACTTTGCCATGAGT | AGTATTGTTGGTAGTGGTGGTCGCACGTACTACGCAGACTCCGTGAAGGGC | GACCCTTATCGTGTAGCAGTGGCTGGGGCCTTTGACTAC |
| | | | SEQ ID NO:7143 | SEQ ID NO:15155 | SEQ ID NO:23167 |
| | | AA | NFAMS | SIVGSGGRTYYADSVKG | DPYRVAVAGAFDY |
| | | | SEQ ID NO:7144 | SEQ ID NO:15156 | SEQ ID NO:23168 |
| **iPS:436514** | **21-225_222D10** | NA | AGCTATGGCATGCAC | GTTATATGGTATGATGGAAGTAATAAAAACTATGCAGACTCCGTGAAGGGC | GATCGGGATGTCGGGTACAACGGTATGGACGTC |
| | | | SEQ ID NO:7145 | SEQ ID NO:15157 | SEQ ID NO:23169 |
| | | AA | SYGMH | VIWYDGSNKNYADSVKG | DRDVGYNGMDV |
| | | | SEQ ID NO:7146 | SEQ ID NO:15158 | SEQ ID NO:23170 |
| **iPS:436516** | **21-225_222C12** | NA | GGCTACTATATGCAC | TGGATCCACCCTAACAGTGGTGGCACAAACTATGCACAGAAATTTCAGGGC | GATGGGACCAGCTCGTTTGACTAC |
| | | | SEQ ID NO:7147 | SEQ ID NO:15159 | SEQ ID NO:23171 |
| | | AA | GYYMH | WIHPNSGGTNYAQKFQG | DGTSSFDY |
| | | | SEQ ID NO:7148 | SEQ ID NO:15160 | SEQ ID NO:23172 |

**FIGURE 49**

| | | | | | |
|---|---|---|---|---|---|
| **iPS:436520** | **21-225_223G10** | NA | AGCTATGGTATCAAC | TGGATCAGCGTTTACAGTGGTAACACAAACTATGCACAGAAGCTCCAGGGC | GACTACTACTACGGTATGGACGTC |
| | | | SEQ ID NO:7149 | SEQ ID NO:15161 | SEQ ID NO:23173 |
| | | AA | SYGIN | WISVYSGNTNYAQKLQG | DYYYGMDV |
| | | | SEQ ID NO:7150 | SEQ ID NO:15162 | SEQ ID NO:23174 |
| **iPS:436522** | **21-225_223H10** | NA | AGCTATGGCATGCAC | GTTATATGGTATGATGGAAGTAATAAAAACTATGCAGACTCCGTGAAGGGC | GATCGGGATGTCGGGTACAACGGTATGGACGTC |
| | | | SEQ ID NO:7151 | SEQ ID NO:15163 | SEQ ID NO:23175 |
| | | AA | SYGMH | VIWYDGSNKNYADSVKG | DRDVGYNGMDV |
| | | | SEQ ID NO:7152 | SEQ ID NO:15164 | SEQ ID NO:23176 |
| **iPS:436526** | **21-225_224A1** | NA | AGCTATGCCATGAGC | GCTATTAGTGGCAGAGGCGGCAGCACATACTACGCAGACGCCGTGAAGGGC | GGCTCCTACGATAGTAGTGGTTATTACCACTACTTAGACCAC |
| | | | SEQ ID NO:7153 | SEQ ID NO:15165 | SEQ ID NO:23177 |
| | | AA | SYAMS | AISGRGGSTYYADAVKG | GSYDSSGYYHYLDH |
| | | | SEQ ID NO:7154 | SEQ ID NO:15166 | SEQ ID NO:23178 |
| **iPS:436528** | **21-225_224B1** | NA | AGCTATGCCATGAGC | GCTATTAGTGGTAGTGGTGGTAACACATACTACGCAGACTCCGTGAAGGGC | GAGGGGGGCTACTACTATTACTACGGTGTGGACGTC |
| | | | SEQ ID NO:7155 | SEQ ID NO:15167 | SEQ ID NO:23179 |
| | | AA | SYAMS | AISGSGGNTYYADSVKG | EGGYYYYYGVDV |
| | | | SEQ ID NO:7156 | SEQ ID NO:15168 | SEQ ID NO:23180 |

FIGURE 49

| | | | | | |
|---|---|---|---|---|---|
| **iPS:436534** | **21-225_224F1** | NA | AGCTATATCATGCAC | GTTATATGGTATGATGGAAGTAATAAATACTATGCAGACTCCGTGAAGGGC | GAGAGGTATAGCAGCAACTGGTACGACTACGGTATGGACGTC |
| | | | SEQ ID NO:7157 | SEQ ID NO:15169 | SEQ ID NO:23181 |
| | | AA | SYIMH | VIWYDGSNKYYADSVKG | ERYSSNWYDYGMDV |
| | | | SEQ ID NO:7158 | SEQ ID NO:15170 | SEQ ID NO:23182 |
| **iPS:436536** | **21-225_224G1** | NA | GGCTACTATATACAC | TGGATCAACCCTTACAGTGGTGACACAAACTATGCACAGAAGTTTCAGGGC | GATTGGGGTGGCTACAGTTCTTACTACTACGGTATGGACGTC |
| | | | SEQ ID NO:7159 | SEQ ID NO:15171 | SEQ ID NO:23183 |
| | | AA | GYYIH | WINPYSGDTNYAQKFQG | DWGGYSSYYYGMDV |
| | | | SEQ ID NO:7160 | SEQ ID NO:15172 | SEQ ID NO:23184 |
| **iPS:436538** | **21-225_224C3** | NA | AGAAGTAGTTACTACTGGGGC | AATATCTATTATAGTGGGAGCACCTACTACAACCCGTCCCTCAAGAGT | CAGGGTCGGGACTGGGGTGTTGACTAC |
| | | | SEQ ID NO:7161 | SEQ ID NO:15173 | SEQ ID NO:23185 |
| | | AA | RSSYYWG | NIYYSGSTYYNPSLKS | QGRDWGVDY |
| | | | SEQ ID NO:7162 | SEQ ID NO:15174 | SEQ ID NO:23186 |
| **iPS:436540** | **21-225_224F3** | NA | AGCTATGGCATGCAC | GTTATATGGTATGATGGAAGTAATAAATACTATGCAGACTCCGTGAAGGGC | GAGAGGTATAGCAGCAGCTGGTACGACTACGGTATGGACGTC |
| | | | SEQ ID NO:7163 | SEQ ID NO:15175 | SEQ ID NO:23187 |
| | | AA | SYGMH | VIWYDGSNKYYADSVKG | ERYSSSWYDYGMDV |
| | | | SEQ ID NO:7164 | SEQ ID NO:15176 | SEQ ID NO:23188 |

FIGURE 49

| | | | | | |
|---|---|---|---|---|---|
| **iPS:436544** | **21-225_224H5** | NA | AATTATGATATCAAC | TGGATGAACCCTAACAGTGGTAACACAGGCTATGCACAGAAGTTCCAGGGC | TCCAGTGGCTGGAACTGGTTCGACCCC |
| | | | SEQ ID NO:7165 | SEQ ID NO:15177 | SEQ ID NO:23189 |
| | | AA | NYDIN | WMNPNSGNTGYAQKFQG | SSGWNWFDP |
| | | | SEQ ID NO:7166 | SEQ ID NO:15178 | SEQ ID NO:23190 |
| **iPS:436546** | **21-225_224D6** | NA | AGCGATGCCATGAGC | GCTATTAGTGGTAGTGGTGATAACACATTCTACGCAGACTCCGTGAAGGGC | GTCTATAGTGCCTACGATTCTCACTGGTTCGACCCC |
| | | | SEQ ID NO:7167 | SEQ ID NO:15179 | SEQ ID NO:23191 |
| | | AA | SDAMS | AISGSGDNTFYADSVKG | VYSAYDSHWFDP |
| | | | SEQ ID NO:7168 | SEQ ID NO:15180 | SEQ ID NO:23192 |
| **iPS:436548** | **21-225_224A7** | NA | GGCTACTATATACAC | TGGATCAACCCTTACAGTGGTGACACAAACTATGCACAGAAGTTTCAGGGC | GATTGGGGTGGCTACAGTTCTTACTACTACGGTATGGACGTC |
| | | | SEQ ID NO:7169 | SEQ ID NO:15181 | SEQ ID NO:23193 |
| | | AA | GYYIH | WINPYSGDTNYAQKFQG | DWGGYSSYYYGMDV |
| | | | SEQ ID NO:7170 | SEQ ID NO:15182 | SEQ ID NO:23194 |
| **iPS:436550** | **21-225_224D8** | NA | AATTATGATATCAAC | TGGTTGTACCCTAACAGTGGTAACACAGGCTATGCACAGAAGTTCCAGGGC | AGCAGTGGCTGGTACTACTTTGACTAC |
| | | | SEQ ID NO:7171 | SEQ ID NO:15183 | SEQ ID NO:23195 |
| | | AA | NYDIN | WLYPNSGNTGYAQKFQG | SSGWYYFDY |
| | | | SEQ ID NO:7172 | SEQ ID NO:15184 | SEQ ID NO:23196 |
| **iPS:436554** | **21-225_224C10** | NA | AATTATGATATCAAC | TGGATGCACCCTAACAGTGGTAACACAGGCTATGCACAGAAGTTCCAGGGC | AGCAGTGGCTGGTACAAGTTTGACTAC |
| | | | SEQ ID NO:7173 | SEQ ID NO:15185 | SEQ ID NO:23197 |

FIGURE 49

| | | | | | |
|---|---|---|---|---|---|
| | | AA | NYDIN | WMHPNSGNTGYAQKFQG | SSGWYKFDY |
| | | | SEQ ID NO:7174 | SEQ ID NO:15186 | SEQ ID NO:23198 |
| **iPS:436556** | **21-225_224D10** | NA | AGCTATGGCATGCAC | GTTATATGGTATGAGGGAAGTAATAAATACTATGTAGACTCCGTGAGGGGC | GAGCTAGGCTTCCAGTCTGACTAC |
| | | | SEQ ID NO:7175 | SEQ ID NO:15187 | SEQ ID NO:23199 |
| | | AA | SYGMH | VIWYEGSNKYYVDSVRG | ELGFQSDY |
| | | | SEQ ID NO:7176 | SEQ ID NO:15188 | SEQ ID NO:23200 |
| **iPS:436558** | **21-225_224C11** | NA | GGCTACTATATACAC | TGGATCAACCCTTACAGTGGTGACACAAACTATGCACAGAAGTTTCAGGGC | GATTGGGGTGGCTACAGTTCTTACTACTTCGGTATGGACGTC |
| | | | SEQ ID NO:7177 | SEQ ID NO:15189 | SEQ ID NO:23201 |
| | | AA | GYYIH | WINPYSGDTNYAQKFQG | DWGGYSSYYFGMDV |
| | | | SEQ ID NO:7178 | SEQ ID NO:15190 | SEQ ID NO:23202 |
| **iPS:436560** | **21-225_224F11** | NA | AATTATGATATCAAC | TGGATGAACCCTAACAGTGGTAACACAGGCTATGCACAGAAGTTCCAGGGC | AGCAGTGGCTGGTACAAGTTTGACTAC |
| | | | SEQ ID NO:7179 | SEQ ID NO:15191 | SEQ ID NO:23203 |
| | | AA | NYDIN | WMNPNSGNTGYAQKFQG | SSGWYKFDY |
| | | | SEQ ID NO:7180 | SEQ ID NO:15192 | SEQ ID NO:23204 |
| **iPS:436562** | **21-225_224H11** | NA | GGCTACTATATACAC | TGGATCAACCCTTACAGTGGTGACACAAACTATGCACAGAAGTTTCAGGGC | GATTGGGGTGGCTACAGTTCTTACTACTACGGTATGGACGTC |
| | | | SEQ ID NO:7181 | SEQ ID NO:15193 | SEQ ID NO:23205 |
| | | AA | GYYIH | WINPYSGDTNYAQKFQG | DWGGYSSYYYGMDV |
| | | | SEQ ID NO:7182 | SEQ ID NO:15194 | SEQ ID NO:23206 |

FIGURE 49

| | | | | | |
|---|---|---|---|---|---|
| **iPS:436564** | **21-225_225A1** | NA | GACTATGTCATCCAC | GTTATATGGTATGATGGAAGTAATAAATACTATGCAGACTCCGTGAAGGGC | GAGAGGTATAGCAGTGGCTGGTACGACTACGGTATGGACGTC |
| | | | SEQ ID NO:7183 | SEQ ID NO:15195 | SEQ ID NO:23207 |
| | | AA | DYVIH | VIWYDGSNKYYADSVKG | ERYSSGWYDYGMDV |
| | | | SEQ ID NO:7184 | SEQ ID NO:15196 | SEQ ID NO:23208 |
| **iPS:436568** | **21-225_225B3** | NA | AGCTACTATATGCAC | ATAATCAACCCTAGTGGTGGTAGCACAAGCTACGCACAGAAGTTCCAGGGC | GATTTAGCAGCTCGTTCTTACTACTACTACTTCGGTATGGACGTC |
| | | | SEQ ID NO:7185 | SEQ ID NO:15197 | SEQ ID NO:23209 |
| | | AA | SYYMH | IINPSGGSTSYAQKFQG | DLAARSYYYYFGMDV |
| | | | SEQ ID NO:7186 | SEQ ID NO:15198 | SEQ ID NO:23210 |
| **iPS:436570** | **21-225_225F4** | NA | AATTATGATATCAAC | TGGATGCACCCTAACAGTGGTAGCACAGGCTATGCACAGAAGTTCCAGGGC | AGCAGTGGCTGGTACTGGTTCGACCCC |
| | | | SEQ ID NO:7187 | SEQ ID NO:15199 | SEQ ID NO:23211 |
| | | AA | NYDIN | WMHPNSGSTGYAQKFQG | SSGWYWFDP |
| | | | SEQ ID NO:7188 | SEQ ID NO:15200 | SEQ ID NO:23212 |
| **iPS:436572** | **21-225_225G4** | NA | GGCTACTATATACAC | TGGATCAACCCTTACAGTGGTGACACAAACTATGCACAGAAGTTTCAGGGC | GATTGGGGTGGCTACAGTTCTTACTACTACGGTATGGACGTC |
| | | | SEQ ID NO:7189 | SEQ ID NO:15201 | SEQ ID NO:23213 |
| | | AA | GYYIH | WINPYSGDTNYAQKFQG | DWGGYSSYYYGMDV |
| | | | SEQ ID NO:7190 | SEQ ID NO:15202 | SEQ ID NO:23214 |
| **iPS:436574** | | NA | AATTATGATATCAAC | TGGATGCATCCTAACAGTGGTAACACAGGCTTTGCACAGAAGTTCCAGGGC | AGCAGTGGCTGGTACCGCTTTGACTAC |

FIGURE 49

| | | | | | |
|---|---|---|---|---|---|
| | 21-225_225F5 | | SEQ ID NO:7191 | SEQ ID NO:15203 | SEQ ID NO:23215 |
| | | AA | NYDIN | WMHPNSGNTGFAQKFQG | SSGWYRFDY |
| | | | SEQ ID NO:7192 | SEQ ID NO:15204 | SEQ ID NO:23216 |
| iPS:436576 | 21-225_225B6 | NA | GACTATGGCATGCAC | GTTATATGGTATGATGA AAATAATAAATACTATG CAGACTCCGTGAAGGGC | GAAGTGGGATTCACTGAGGA CTAC |
| | | | SEQ ID NO:7193 | SEQ ID NO:15205 | SEQ ID NO:23217 |
| | | AA | DYGMH | VIWYDENNKYYADSVKG | EVGFTEDY |
| | | | SEQ ID NO:7194 | SEQ ID NO:15206 | SEQ ID NO:23218 |
| iPS:436578 | 21-225_225D6 | NA | AACTATGGCATGCAC | GTTATATGGTATGATGA AAATAATAAATACTATG CAGACTCCGTGAAGGGC | GAAGTGGGATTCACTGAGGA CTAC |
| | | | SEQ ID NO:7195 | SEQ ID NO:15207 | SEQ ID NO:23219 |
| | | AA | NYGMH | VIWYDENNKYYADSVKG | EVGFTEDY |
| | | | SEQ ID NO:7196 | SEQ ID NO:15208 | SEQ ID NO:23220 |
| iPS:436580 | 21-225_225E7 | NA | AGTGGTCATTACTACTGGAG C | TTCATCTATTACACTGGG AGCACCTACTACAACCC GTCCCTCAAGAGT | GAGGCCGGTGACTACGGCTA CTACGGTATGGACGTC |
| | | | SEQ ID NO:7197 | SEQ ID NO:15209 | SEQ ID NO:23221 |
| | | AA | SGHYYWS | FIYYTGSTYYNPSLKS | EAGDYGYYGMDV |
| | | | SEQ ID NO:7198 | SEQ ID NO:15210 | SEQ ID NO:23222 |
| iPS:436582 | 21 225 225F8 | NA | AGCTATGGCATGCAC | GTTATATGGTATGATGA AAATAATAAATACTATG TAGACTCCGTGAAGGGC | GAAGTGGGATTTACTGAGGA CTAC |

**FIGURE 49**

| | | | | | |
|---|---|---|---|---|---|
| | **21-225_225F8** | | SEQ ID NO:7199 | SEQ ID NO:15211 | SEQ ID NO:23223 |
| | | AA | SYGMH | VIWYDENNKYYVDSVKG | EVGFTEDY |
| | | | SEQ ID NO:7200 | SEQ ID NO:15212 | SEQ ID NO:23224 |
| **iPS:436584** | **21-225_225B9** | NA | AATTATGATATCAAC | TGGATGCACCCTAACAG TGGTAACACAGGCTATG CACAGAAGTTCCGGGGC | AGCAGTGGCTGGACCCTTTT TGACTAC |
| | | | SEQ ID NO:7201 | SEQ ID NO:15213 | SEQ ID NO:23225 |
| | | AA | NYDIN | WMHPNSGNTGYAQKFRG | SSGWTLFDY |
| | | | SEQ ID NO:7202 | SEQ ID NO:15214 | SEQ ID NO:23226 |
| **iPS:436586** | **21-225_225F11** | NA | AATTATGATATCAAC | TGGATGCACCCTAACAG TGGTAACACAGGCTATG CACAGAAGTTCCAGGGC | AGCAGTGGCTGGTACCGCTT TGACTAC |
| | | | SEQ ID NO:7203 | SEQ ID NO:15215 | SEQ ID NO:23227 |
| | | AA | NYDIN | WMHPNSGNTGYAQKFQG | SSGWYRFDY |
| | | | SEQ ID NO:7204 | SEQ ID NO:15216 | SEQ ID NO:23228 |
| **iPS:436588** | **21-225_225F12** | NA | CATTATGATATCAAC | TGGATGCACCCAAACAG TGGTAACACAGGCTATG CACAGAAGTTCCAGGGC | AGCAGTGGCTGGTACAAGTT TGACTAC |
| | | | SEQ ID NO:7205 | SEQ ID NO:15217 | SEQ ID NO:23229 |
| | | AA | HYDIN | WMHPNSGNTGYAQKFQG | SSGWYKFDY |
| | | | SEQ ID NO:7206 | SEQ ID NO:15218 | SEQ ID NO:23230 |
| **iPS:436590** | **21-225_225H12** | NA | AATTATGATATCAAC | TGGATGCACCCTAACAG TGGTAACACAGGCTATG CACAGAAGTTCCAGGGC | AGCAGTGGCTGGTACAAGTT TGACTAC |
| | | | SEQ ID NO:7207 | SEQ ID NO:15219 | SEQ ID NO:23231 |

FIGURE 49

| | | | | | |
|---|---|---|---|---|---|
| | | AA | NYDIN | WMHPNSGNTGYAQKFQG | SSGWYKFDY |
| | | | SEQ ID NO:7208 | SEQ ID NO:15220 | SEQ ID NO:23232 |
| iPS:436592 | 21-225_226B1 | NA | ACCTATGGCATGCAC | ATTATATGGTATGATGG AGGTTATAAATACTATG CAGACTCCGTGAAGGGC | GATCACTACGATTTTTGGAG TGGTTATCTTACCCAC |
| | | | SEQ ID NO:7209 | SEQ ID NO:15221 | SEQ ID NO:23233 |
| | | AA | TYGMH | IIWYDGGYKYYADSVKG | DHYDFWSGYLTH |
| | | | SEQ ID NO:7210 | SEQ ID NO:15222 | SEQ ID NO:23234 |
| iPS:436594 | 21-225_226A5 | NA | AACTATGGCATGCAC | ATTATATGGTATGATGG AACTAATAAATACTATA CAGACTCCGTGAAGGGC | GAGGGTCACGATTTTTGGAG TGGCTTTTTTTGTTAC |
| | | | SEQ ID NO:7211 | SEQ ID NO:15223 | SEQ ID NO:23235 |
| | | AA | NYGMH | IIWYDGTNKYYTDSVKG | EGHDFWSGFFCY |
| | | | SEQ ID NO:7212 | SEQ ID NO:15224 | SEQ ID NO:23236 |
| iPS:436596 | 21-225_226C6 | NA | GACTATGGCATGCAC | GTTATATGGTATGATGG AAGTAATAAATACTATG CAGACTCCGTGAAGGGC | GAGAGGTATAGCAGCAGCTG GTACGACTACGGTATGGACG TC |
| | | | SEQ ID NO:7213 | SEQ ID NO:15225 | SEQ ID NO:23237 |
| | | AA | DYGMH | VIWYDGSNKYYADSVKG | ERYSSSWYDYGMDV |
| | | | SEQ ID NO:7214 | SEQ ID NO:15226 | SEQ ID NO:23238 |
| iPS:436598 | 21-225_226D6 | NA | AATTATGATATCAAC | TGGATGCACCCTAACAG TGGTAACACAGGCTATG CACAGAAGTTCCAGGGC | AGCAGTGGCTGGTACAAGTT TGACTAC |
| | | | SEQ ID NO:7215 | SEQ ID NO:15227 | SEQ ID NO:23239 |
| | | AA | NYDIN | WMHPNSGNTGYAQKFQG | SSGWYKFDY |

FIGURE 49

| | | | | | |
|---|---|---|---|---|---|
| | | | SEQ ID NO:7216 | SEQ ID NO:15228 | SEQ ID NO:23240 |
| **iPS:436600** | **21-225_226F6** | NA | AATTATGATATCAAC | TGGATGCACCCTAACAGTGGTAACACAGGCTATGCACAGAAGTTCCAGGGC | AGCAGTGGCTGGTACCGCTTTGACTAC |
| | | | SEQ ID NO:7217 | SEQ ID NO:15229 | SEQ ID NO:23241 |
| | | AA | NYDIN | WMHPNSGNTGYAQKFQG | SSGWYRFDY |
| | | | SEQ ID NO:7218 | SEQ ID NO:15230 | SEQ ID NO:23242 |
| **iPS:436602** | **21-225_226E7** | NA | ACCTATGGCATGCAC | ATTATATGGTATGATGGAAGTAATAAATACTATGCAGACTCCGTGAAGGGC | GAGAATTACGATTTTTGGAGTGGTTATTATGGCTAC |
| | | | SEQ ID NO:7219 | SEQ ID NO:15231 | SEQ ID NO:23243 |
| | | AA | TYGMH | IIWYDGSNKYYADSVKG | ENYDFWSGYYGY |
| | | | SEQ ID NO:7220 | SEQ ID NO:15232 | SEQ ID NO:23244 |
| **iPS:436604** | **21-225_226F7** | NA | AGCTATGGCATGCAC | ATTATATGGTATGATGGAAGTAATAAATACTATGCAGACTCCGTGAAGGGC | GAGAGGTATAACAGCGGCTGGTACGACTACGGTTTGGACGTC |
| | | | SEQ ID NO:7221 | SEQ ID NO:15233 | SEQ ID NO:23245 |
| | | AA | SYGMH | IIWYDGSNKYYADSVKG | ERYNSGWYDYGLDV |
| | | | SEQ ID NO:7222 | SEQ ID NO:15234 | SEQ ID NO:23246 |
| **iPS:436606** | **21-225_226G8** | NA | GGCTACTATATACAC | TGGATCAACCCTTACAGTGGTGACACAAAGTATGCACAGAAGTTTCAGGGC | GATTGGGGTGGCTACAGTTCTTACTACTACGGTATGGACGTC |
| | | | SEQ ID NO:7223 | SEQ ID NO:15235 | SEQ ID NO:23247 |
| | | AA | GYYIH | WINPYSGDTKYAQKFQG | DWGGYSSYYYGMDV |
| | | | SEQ ID NO:7224 | SEQ ID NO:15236 | SEQ ID NO:23248 |

# FIGURE 49

| | | | | | |
|---|---|---|---|---|---|
| **iPS:436608** | **21-225_226A9** | NA | AACTATGGCATGCAC | GTTATATGGTATGAGGAAAGTAATAAATACTATACAGACTCCGTGAAGGGC | GAAGTGGGATTCACTGAGGACTAC |
| | | | SEQ ID NO:7225 | SEQ ID NO:15237 | SEQ ID NO:23249 |
| | | AA | NYGMH | VIWYEESNKYYTDSVKG | EVGFTEDY |
| | | | SEQ ID NO:7226 | SEQ ID NO:15238 | SEQ ID NO:23250 |
| **iPS:436610** | **21-225_226F9** | NA | GGCTACTATATACAC | TGGATCAACCCTTACAGTGGTGACACAAAGTATGCACAGAAGTTTCAGGGC | GATTGGGGTGGCTACAGTTCTTACTACTACGGTATGGACGTC |
| | | | SEQ ID NO:7227 | SEQ ID NO:15239 | SEQ ID NO:23251 |
| | | AA | GYYIH | WINPYSGDTKYAQKFQG | DWGGYSSYYYGMDV |
| | | | SEQ ID NO:7228 | SEQ ID NO:15240 | SEQ ID NO:23252 |
| **iPS:436612** | **21-225_226H9** | NA | GGCTACTATATACAC | TGGATCAACCCTTACAGTGGTGACACAAACTCTGCACAGAAGTTTCAGGGC | GATTGGGGTGGCTACAGTTCTTACTACTACGGTATGGACGTC |
| | | | SEQ ID NO:7229 | SEQ ID NO:15241 | SEQ ID NO:23253 |
| | | AA | GYYIH | WINPYSGDTNSAQKFQG | DWGGYSSYYYGMDV |
| | | | SEQ ID NO:7230 | SEQ ID NO:15242 | SEQ ID NO:23254 |
| **iPS:436614** | **21-225_226F10** | NA | GGCTATTATATACAC | TGGATCAACCCATACAGTGGTGACACAAACTATGCACAGAAGTTTCAGGGC | GATTGGGGTGGCTACAGTTCTTACTACTACGGTATGGACGTC |
| | | | SEQ ID NO:7231 | SEQ ID NO:15243 | SEQ ID NO:23255 |
| | | AA | GYYIH | WINPYSGDTNYAQKFQG | DWGGYSSYYYGMDV |
| | | | SEQ ID NO:7232 | SEQ ID NO:15244 | SEQ ID NO:23256 |
| **iPS:436616** | **21-225_226D11** | NA | AATTATGATATCAAC | TGGATGCACCCTAACAGTGGTAACACAGGCTATGCACAGAAGTTCCAGGGC | AGCAGTGGCTGGTACTACTTTGACTAC |
| | | | SEQ ID NO:7233 | SEQ ID NO:15245 | SEQ ID NO:23257 |

FIGURE 49

| | | | | | |
|---|---|---|---|---|---|
| | | AA | NYDIN | WMHPNSGNTGYAQKFQG | SSGWYYFDY |
| | | | SEQ ID NO:7234 | SEQ ID NO:15246 | SEQ ID NO:23258 |
| **iPS:436618** | **21-225_226E11** | NA | GGCTACTATATACAC | TGGATCAACCCTTACAGTGGTGACACAAACTATGCACAGAAGTTTCAGGGC | GATTGGGGTGGCTACAGTTCTTACTACTACGGTATGGACGTC |
| | | | SEQ ID NO:7235 | SEQ ID NO:15247 | SEQ ID NO:23259 |
| | | AA | GYYIH | WINPYSGDTNYAQKFQG | DWGGYSSYYYGMDV |
| | | | SEQ ID NO:7236 | SEQ ID NO:15248 | SEQ ID NO:23260 |
| **iPS:436620** | **21-225_226H11** | NA | AACTGTGGCATGCAC | GTTATATGGTATGATGGAAGTAATAAATACTATGCAGACTCCGTGAAGGGC | GAGCTGTATAGCAGCAGCTGGTACGACTACGGTTTGGACGTC |
| | | | SEQ ID NO:7237 | SEQ ID NO:15249 | SEQ ID NO:23261 |
| | | AA | NCGMH | VIWYDGSNKYYADSVKG | ELYSSSWYDYGLDV |
| | | | SEQ ID NO:7238 | SEQ ID NO:15250 | SEQ ID NO:23262 |
| **iPS:436622** | **21-225_226A12** | NA | AATTATGATATCAAC | TGGATGCACCCTAACAGTGGTAACACAGGCTATGCACAGAAGTTCCAGGGC | AGCAGTGGCTGGTACTACTTTGACTAC |
| | | | SEQ ID NO:7239 | SEQ ID NO:15251 | SEQ ID NO:23263 |
| | | AA | NYDIN | WMHPNSGNTGYAQKFQG | SSGWYYFDY |
| | | | SEQ ID NO:7240 | SEQ ID NO:15252 | SEQ ID NO:23264 |
| **iPS:436624** | **21-225_226H12** | NA | GGCTACTATATACAC | TGGATCAACCCTTACAGTGGTGACACAAACTATGCACAGAAGTTTCAGGGC | GATTGGGGTGGCTACAGTTCTTACTACTACGGTATGGACGTC |
| | | | SEQ ID NO:7241 | SEQ ID NO:15253 | SEQ ID NO:23265 |
| | | AA | GYYIH | WINPYSGDTNYAQKFQG | DWGGYSSYYYGMDV |
| | | | SEQ ID NO:7242 | SEQ ID NO:15254 | SEQ ID NO:23266 |

FIGURE 49

| | | | | | |
|---|---|---|---|---|---|
| **iPS:436626** | **21-225_227C1** | NA | GGCTACTATACACAC | TGGATCAACCCTTACAGTGGTGGCACAAACTATGCACAGAAGTTTCAGGGC | GATTGGGGTGGCTACAGTTCTTACTACTACGGTATGGACGTC |
| | | | SEQ ID NO:7243 | SEQ ID NO:15255 | SEQ ID NO:23267 |
| | | AA | GYYTH | WINPYSGGTNYAQKFQG | DWGGYSSYYYGMDV |
| | | | SEQ ID NO:7244 | SEQ ID NO:15256 | SEQ ID NO:23268 |
| **iPS:436628** | **21-225_227F2** | NA | GGCTACTATATACAC | TGGATCAACCCTTACAGTGGTGACACAAAGTATGCACAGAAGTTTCAGGGC | GATTGGGGTGGCTACAGTTCTTACTACTACGGTATGGACGTC |
| | | | SEQ ID NO:7245 | SEQ ID NO:15257 | SEQ ID NO:23269 |
| | | AA | GYYIH | WINPYSGDTKYAQKFQG | DWGGYSSYYYGMDV |
| | | | SEQ ID NO:7246 | SEQ ID NO:15258 | SEQ ID NO:23270 |
| **iPS:436630** | **21-225_227G3** | NA | AGCTATGGCATGCAC | GTTATATGGTATGTTGGAAGTAATCAATACTATGCAGACTCCGTGAAGGGC | GAAGTGGGATTCACTGAGGACTAC |
| | | | SEQ ID NO:7247 | SEQ ID NO:15259 | SEQ ID NO:23271 |
| | | AA | SYGMH | VIWYVGSNQYYADSVKG | EVGFTEDY |
| | | | SEQ ID NO:7248 | SEQ ID NO:15260 | SEQ ID NO:23272 |
| **iPS:436632** | **21-225_227E4** | NA | ACTTTTGCCATGACC | GTTATTAGTGGTAGAGGTGGTAGCTCATTCTACGCAGACTCCGTGAAGGGC | GATCAACTATGGTTTGACTAC |
| | | | SEQ ID NO:7249 | SEQ ID NO:15261 | SEQ ID NO:23273 |
| | | AA | TFAMT | VISGRGGSSFYADSVKG | DQLWFDY |
| | | | SEQ ID NO:7250 | SEQ ID NO:15262 | SEQ ID NO:23274 |
| **iPS:436634** | **21-225_227H5** | NA | AACTATGGCATGCAC | GTTATATGGTATGAAGAAAGTAATAAATACTATGCAGACTCCGTGAAGGGC | GAAGTGGGATTCACTGAGGACTAC |
| | | | SEQ ID NO:7251 | SEQ ID NO:15263 | SEQ ID NO:23275 |

FIGURE 49

| | | | | | |
|---|---|---|---|---|---|
| | | AA | NYGMH | VIWYEESNKYYADSVKG | EVGFTEDY |
| | | | SEQ ID NO:7252 | SEQ ID NO:15264 | SEQ ID NO:23276 |
| **iPS:436636** | **21-225_227E6** | NA | AATTATGATATCAAC | TGGATGCACCCTAACAGTGGTAACACAGGCTATGCACAGAAGTTCCAGGGC | AGCAGTGGCTGGTACAAGTTTGACTAC |
| | | | SEQ ID NO:7253 | SEQ ID NO:15265 | SEQ ID NO:23277 |
| | | AA | NYDIN | WMHPNSGNTGYAQKFQG | SSGWYKFDY |
| | | | SEQ ID NO:7254 | SEQ ID NO:15266 | SEQ ID NO:23278 |
| **iPS:436638** | **21-225_227C7** | NA | AATTATGATATCAAC | TGGATGCATCCTAACAGTGGTAACACAGGCTATGCACAGAAGTTCCAGGGC | AGCAGTGGCTGGTACCGCTTTGACTAC |
| | | | SEQ ID NO:7255 | SEQ ID NO:15267 | SEQ ID NO:23279 |
| | | AA | NYDIN | WMHPNSGNTGYAQKFQG | SSGWYRFDY |
| | | | SEQ ID NO:7256 | SEQ ID NO:15268 | SEQ ID NO:23280 |
| **iPS:436640** | **21-225_227A8** | NA | GGCTACTATATACAC | TGGATCAACCCTTACAGTGGTGACACAAACTATGCACAGAAGTTTCAGGGC | GATTGGGGTGGCTACAGTTCTTACTACTACGGTATGGACGTC |
| | | | SEQ ID NO:7257 | SEQ ID NO:15269 | SEQ ID NO:23281 |
| | | AA | GYYIH | WINPYSGDTNYAQKFQG | DWGGYSSYYYGMDV |
| | | | SEQ ID NO:7258 | SEQ ID NO:15270 | SEQ ID NO:23282 |
| **iPS:436644** | **21-225_227G9** | NA | AATTATGATATCAAC | TGGATGTACCCTAACAGTGGTAACACAGGCTATGCACAGAAGTTCCAGGGC | AGCAGTGGCTGGTACTGGTTCGACCCC |
| | | | SEQ ID NO:7259 | SEQ ID NO:15271 | SEQ ID NO:23283 |
| | | AA | NYDIN | WMYPNSGNTGYAQKFQG | SSGWYWFDP |
| | | | SEQ ID NO:7260 | SEQ ID NO:15272 | SEQ ID NO:23284 |

FIGURE 49

| | | | | | |
|---|---|---|---|---|---|
| **iPS:436646** | **21-225_227D11** | NA | AATTATGATATCAAC | TGGATGCACCCTAACAGTGGTAACACAGGCTATGCACAGAAGTTCCAGGGC | AGCAGTGGCTGGTACTACTTTGACTAC |
| | | | SEQ ID NO:7261 | SEQ ID NO:15273 | SEQ ID NO:23285 |
| | | AA | NYDIN | WMHPNSGNTGYAQKFQG | SSGWYYFDY |
| | | | SEQ ID NO:7262 | SEQ ID NO:15274 | SEQ ID NO:23286 |
| **iPS:436648** | **21-225_227F11** | NA | ACCTATAGCATGAAC | TCCATTAGTAGTAGTATTAATTACATGTACTACGCAGACTCAGTGAAGGGC | TTAGGGGTCTAC |
| | | | SEQ ID NO:7263 | SEQ ID NO:15275 | SEQ ID NO:23287 |
| | | AA | TYSMN | SISSSINYMYYADSVKG | LGVY |
| | | | SEQ ID NO:7264 | SEQ ID NO:15276 | SEQ ID NO:23288 |
| **iPS:436650** | **21-225_227C12** | NA | AACTATGGCATGCAC | GTTATATGGTATATTGGAAGTAATCAATACTATGCGGACTCCGTGAAGGGC | GAAGTGGGATTCACTGAGGACTAC |
| | | | SEQ ID NO:7265 | SEQ ID NO:15277 | SEQ ID NO:23289 |
| | | AA | NYGMH | VIWYIGSNQYYADSVKG | EVGFTEDY |
| | | | SEQ ID NO:7266 | SEQ ID NO:15278 | SEQ ID NO:23290 |
| **iPS:436652** | **21-225_146B11** | NA | AGCTATGCCATGAGC | GTTATTAGTGGTGGTGGTAGTAGCACATACTACGCAGACTCCGTGAAGGGC | TGGCGAGGTAACCCCACTGACTACGGTATGGACGTC |
| | | | SEQ ID NO:7267 | SEQ ID NO:15279 | SEQ ID NO:23291 |
| | | AA | SYAMS | VISGGGSSTYYADSVKG | WRGNPTDYGMDV |
| | | | SEQ ID NO:7268 | SEQ ID NO:15280 | SEQ ID NO:23292 |
| **iPS:436654** | **21-225_146C11** | NA | AGCTATGCCATGAGC | GTTATTAGTGGTGGTGGTAGTAGCACATACTACGCAGACTCCGTGAAGGGC | TGGCGAGGTAACCCCACTGACTACGGTATGGACGTC |
| | | | SEQ ID NO:7269 | SEQ ID NO:15281 | SEQ ID NO:23293 |
| | | AA | SYAMS | VISGGGSSTYYADSVKG | WRGNPTDYGMDV |

FIGURE 49

| | | | | | |
|---|---|---|---|---|---|
| | | | SEQ ID NO:7270 | SEQ ID NO:15282 | SEQ ID NO:23294 |
| **iPS:436658** | **21-225_146A2** | NA | AGCTATGCCATGAGC | GTTATTAGTGGTGGTGGT AGTAGCACATACTACGC AGACTCCGTGAAGGGC | TGGCGAGGTAACCCCACTGA CTACGGTATGGACGTC |
| | | | SEQ ID NO:7271 | SEQ ID NO:15283 | SEQ ID NO:23295 |
| | | AA | SYAMS | VISGGGSSTYYADSVKG | WRGNPTDYGMDV |
| | | | SEQ ID NO:7272 | SEQ ID NO:15284 | SEQ ID NO:23296 |
| **iPS:436660** | **21-225_146D8** | NA | AACTATAACATGAAC | TACATTAGTAGAAGTAG TAATACCAAATACTATGT AGACTCTGTGAAGGGC | GATAGGAGTGGGAGCTACGG GTACTTCTACTACTACGGTTT GGACGTC |
| | | | SEQ ID NO:7273 | SEQ ID NO:15285 | SEQ ID NO:23297 |
| | | AA | NYNMN | YISRSSNTKYYVDSVKG | DRSGSYGYFYYYGLDV |
| | | | SEQ ID NO:7274 | SEQ ID NO:15286 | SEQ ID NO:23298 |
| **iPS:436662** | **21-225_147D7** | NA | AGTTATGATATCAAC | TGGATGAACCCTAATAG TGGTAACACAGGCTATG CACAGAAGTTCCAGGGC | GCGGATATTGTATTAGTACC AGCTGCTATCCCTTATAATT ACTACTTCGCTATGGACGTC |
| | | | SEQ ID NO:7275 | SEQ ID NO:15287 | SEQ ID NO:23299 |
| | | AA | SYDIN | WMNPNSGNTGYAQKFQG | ADIVLVPAAIPYNYYFAMDV |
| | | | SEQ ID NO:7276 | SEQ ID NO:15288 | SEQ ID NO:23300 |
| **iPS:436664** | **21-225_147E7** | NA | AGCTATGCCATGAGC | GTTATTAGTGGTGGTGGT AGTAGTACATACTACGC AGACTCCGTGAAGGGC | TGGCGAGGTAACCCCACTGA CTACGGTATGGACGTC |
| | | | SEQ ID NO:7277 | SEQ ID NO:15289 | SEQ ID NO:23301 |
| | | AA | SYAMS | VISGGGSSTYYADSVKG | WRGNPTDYGMDV |
| | | | SEQ ID NO:7278 | SEQ ID NO:15290 | SEQ ID NO:23302 |
| **iPS:436666** | **21-225_147B8** | NA | GACTACTATTTGCAC | TGGATCAACCCTAACAG TGGTGACACAAACTATG CACAGAAGTTTCAGGGC | GATCGGGACTCTGGTTCGGG GAGTTACCCCTACTACTACT ACTACGGTATGGACGTC |
| | | | SEQ ID NO:7279 | SEQ ID NO:15291 | SEQ ID NO:23303 |

**FIGURE 49**

| | | | | | |
|---|---|---|---|---|---|
| | | AA | DYYLH | WINPNSGDTNYAQKFQG | DRDSGSGSYPYYYYYGMDV |
| | | | SEQ ID NO:7280 | SEQ ID NO:15292 | SEQ ID NO:23304 |
| **iPS:436668** | **21-225_147B9** | NA | AGCTATGGCATGCAC | GTTATATGGTATGATGG AAGTAATAAATACTATG CAGACTCCGTGAAGGGC | GATCGTGACTACGGTGACCC CCCCTACTACTACTACTACG GTATGGACGTC |
| | | | SEQ ID NO:7281 | SEQ ID NO:15293 | SEQ ID NO:23305 |
| | | AA | SYGMH | VIWYDGSNKYYADSVKG | DRDYGDPPYYYYYGMDV |
| | | | SEQ ID NO:7282 | SEQ ID NO:15294 | SEQ ID NO:23306 |
| **iPS:436670** | **21-225_147D9** | NA | AGCTATGGCATGCAC | GATATATGGTTTGATGGC AGTAATAAATACTATGT AGACTCCGTGAAGGAC | GATCGGGTGGAGGGTTCGGG GACTCCCTACTACTACTACG GTATGGACGTC |
| | | | SEQ ID NO:7283 | SEQ ID NO:15295 | SEQ ID NO:23307 |
| | | AA | SYGMH | DIWFDGSNKYYVDSVKD | DRVEGSGTPYYYYGMDV |
| | | | SEQ ID NO:7284 | SEQ ID NO:15296 | SEQ ID NO:23308 |
| **iPS:436672** | **21-225_147F9** | NA | ACCTATGGCATGCAC | GTTATATGGTATGGTGG AAGTGATAAAGACTATG CAGACTCCGTGAAGGGC | GATCGGGATTATTGTAGTGG TGGTACTTGTCCTTACTACTA CTACTACGGTATGGACGTC |
| | | | SEQ ID NO:7285 | SEQ ID NO:15297 | SEQ ID NO:23309 |
| | | AA | TYGMH | VIWYGGSDKDYADSVKG | DRDYCSGGTCPYYYYYGMDV |
| | | | SEQ ID NO:7286 | SEQ ID NO:15298 | SEQ ID NO:23310 |
| **iPS:436674** | **21-225_147G9** | NA | ACCTATGGCATGCAC | GTTATATGGTATGGTGG AAGTGATAAAGACTATG CAGACTCCGTGAAGGGC | GATCGGGATTATTGTAGTGG TGGTAGCTGCCCTTACTACT ACTACTACGGTATGGACGTC |
| | | | SEQ ID NO:7287 | SEQ ID NO:15299 | SEQ ID NO:23311 |

FIGURE 49

| | | | | | |
|---|---|---|---|---|---|
| | | AA | TYGMH | VIWYGGSDKDYADSVKG | DRDYCSGGSCPYYYYYGMDV |
| | | | SEQ ID NO:7288 | SEQ ID NO:15300 | SEQ ID NO:23312 |
| **iPS:436676** | **21-225_147E11** | NA | AACTATGTCATGAGC | GTTATTAGTGGTGGTGGTAGTAGTACATACTACGCAGACTCCGTGAAGGGC | TGGCGAGGTAACCCCACTGACTACGGTATGGACGTC |
| | | | SEQ ID NO:7289 | SEQ ID NO:15301 | SEQ ID NO:23313 |
| | | AA | NYVMS | VISGGGSSTYYADSVKG | WRGNPTDYGMDV |
| | | | SEQ ID NO:7290 | SEQ ID NO:15302 | SEQ ID NO:23314 |
| **iPS:436678** | **21-225_147B12** | NA | AGCTATGCCATGAGC | GTTATTAGTGGTGGTGGTAGTAGCACATACTACGCAGACTCCGTGAAGGGC | TGGCGAGGTAACCCCACTGACTACGGTATGGACGTC |
| | | | SEQ ID NO:7291 | SEQ ID NO:15303 | SEQ ID NO:23315 |
| | | AA | SYAMS | VISGGGSSTYYADSVKG | WRGNPTDYGMDV |
| | | | SEQ ID NO:7292 | SEQ ID NO:15304 | SEQ ID NO:23316 |
| **iPS:436680** | **21-225_147H12** | NA | AGTGGTTATTACCACTGGAGC | TACATCTATTACAGTGGGAGCACCTACTACAACCCGTCCCTCAAGAGT | GATTGGGGTGGCTACGATTCGAGTGGCTGGTTCGACCCC |
| | | | SEQ ID NO:7293 | SEQ ID NO:15305 | SEQ ID NO:23317 |
| | | AA | SGYYHWS | YIYYSGSTYYNPSLKS | DWGGYDSSGWFDP |
| | | | SEQ ID NO:7294 | SEQ ID NO:15306 | SEQ ID NO:23318 |
| **iPS:436682** | **21-225_146A8** | NA | AACTATAACATGAAC | TACATTAGTAGAAGTAGTAATACCAAATACTACGCAGACTCTGTGAGGGGC | GATAGGAGTGGGAGCTACGGGTACTTCTACTACTACGGTTTGGACGTC |
| | | | SEQ ID NO:7295 | SEQ ID NO:15307 | SEQ ID NO:23319 |
| | | AA | NYNMN | YISRSSNTKYYADSVRG | DRSGSYGYFYYYGLDV |
| | | | SEQ ID NO:7296 | SEQ ID NO:15308 | SEQ ID NO:23320 |

**FIGURE 49**

| | | | | | |
|---|---|---|---|---|---|
| **iPS:436684** | **21-225_146B6** | NA | AGCTATAACATGAAC | TACATTAGTAGAAGTAGTAATACCAAACACTACGCAGACTCTGTGAAGGGC | GATAGGAGTGGGAGCTACGGGTACTTCTACTACTACGGTTTGGACGTC |
| | | | SEQ ID NO:7297 | SEQ ID NO:15309 | SEQ ID NO:23321 |
| | | AA | SYNMN | YISRSSNTKHYADSVKG | DRSGSYGYFYYYGLDV |
| | | | SEQ ID NO:7298 | SEQ ID NO:15310 | SEQ ID NO:23322 |
| **iPS:436686** | **21-225_148G6** | NA | AGCTATGCCATGAGC | GTTATTAGTGGTGGTGGTAGTAGCACATACTACGCAGACTCCGTGAAGGGC | TGGCGAGGTAACCCCACTGACTACGGTATGGACGTC |
| | | | SEQ ID NO:7299 | SEQ ID NO:15311 | SEQ ID NO:23323 |
| | | AA | SYAMS | VISGGGSSTYYADSVKG | WRGNPTDYGMDV |
| | | | SEQ ID NO:7300 | SEQ ID NO:15312 | SEQ ID NO:23324 |
| **iPS:436688** | **21-225_148C8** | NA | AGCTATGGCATGCAC | GTTATATGGTATGATGGAAGTAATAAATACTATGCAGACTCCGTGAAGGGC | GATCGTGACTACGGTGACCCCCCCTACTACTACTACTACGGTATGGACGTC |
| | | | SEQ ID NO:7301 | SEQ ID NO:15313 | SEQ ID NO:23325 |
| | | AA | SYGMH | VIWYDGSNKYYADSVKG | DRDYGDPPYYYYYGMDV |
| | | | SEQ ID NO:7302 | SEQ ID NO:15314 | SEQ ID NO:23326 |
| **iPS:436690** | **21-225_148A9** | NA | ACCTATGGCATGCAC | GTTATATGGTATGGTGGAAGTGATAAAGACTATGCAGACTCCGTGAAGGGC | GATCGGGATTATTGTAGTGGTGGTACTTGTCCTTACTACTACTACTACGGTATGGACGTC |
| | | | SEQ ID NO:7303 | SEQ ID NO:15315 | SEQ ID NO:23327 |
| | | AA | TYGMH | VIWYGGSDKDYADSVKG | DRDYCSGGTCPYYYYYGMDV |
| | | | SEQ ID NO:7304 | SEQ ID NO:15316 | SEQ ID NO:23328 |

FIGURE 49

| | | | | | |
|---|---|---|---|---|---|
| **iPS:436694** | **21-225_148G11** | NA | AGCTATCCCATGAGC | GTTATTAGTGGTGGTGGTAGTAGTGCATACTACGCAGACTCCGTGAAGGGC | TGGCGAGGTAACCCCACTGACTACGGTATGGACGTC |
| | | | SEQ ID NO:7305 | SEQ ID NO:15317 | SEQ ID NO:23329 |
| | | AA | SYPMS | VISGGGSSAYYADSVKG | WRGNPTDYGMDV |
| | | | SEQ ID NO:7306 | SEQ ID NO:15318 | SEQ ID NO:23330 |
| **iPS:436696** | **21-225_149A1** | NA | AGCTATAACATGAAC | TACATTAGTAGAAGTAGTAATACCAAACACTACGCAGACTCTGTGAAGGGC | GATAGGAGTGGGAGCTACGGGTACTTCTACTACTACGGTTTGGACGTC |
| | | | SEQ ID NO:7307 | SEQ ID NO:15319 | SEQ ID NO:23331 |
| | | AA | SYNMN | YISRSSNTKHYADSVKG | DRSGSYGYFYYYGLDV |
| | | | SEQ ID NO:7308 | SEQ ID NO:15320 | SEQ ID NO:23332 |
| **iPS:436698** | **21-225_149B5** | NA | GGCTATTGGATGAAC | AACATAAAGCAAGATGGAAGTGAGAAATACTATGTGGACTCTGTGAAGGGC | GGGATGTATAGCAGTGGCTGGTACGTCTTTGACTAC |
| | | | SEQ ID NO:7309 | SEQ ID NO:15321 | SEQ ID NO:23333 |
| | | AA | GYWMN | NIKQDGSEKYYVDSVKG | GMYSSGWYVFDY |
| | | | SEQ ID NO:7310 | SEQ ID NO:15322 | SEQ ID NO:23334 |
| **iPS:436700** | **21-225_149C7** | NA | AGCTATGCCATGAGC | GTTATTAGTGGTGGTGGTAGTAGCACATACTACGCAGACTCCGTGAAGGGC | TGGCGAGGTAACCCCACTGACTACGGTATGGACGTC |
| | | | SEQ ID NO:7311 | SEQ ID NO:15323 | SEQ ID NO:23335 |
| | | AA | SYAMS | VISGGGSSTYYADSVKG | WRGNPTDYGMDV |
| | | | SEQ ID NO:7312 | SEQ ID NO:15324 | SEQ ID NO:23336 |
| **iPS:436702** | **21-225_149E8** | NA | AGTTATAGCATGAAC | GCCATTAGTAGTACTGGTAGTTACATATATTACGCAGACTCAGTGAAGGGC | ACGGCAGTGGCTGGTACTGGGTGGTTCGACCCC |
| | | | SEQ ID NO:7313 | SEQ ID NO:15325 | SEQ ID NO:23337 |

FIGURE 49

| | | | | | |
|---|---|---|---|---|---|
| | | AA | SYSMN | AISSTGSYIYYADSVKG | TAVAGTGWFDP |
| | | | SEQ ID NO:7314 | SEQ ID NO:15326 | SEQ ID NO:23338 |
| **iPS:436704** | **21-225_149C10** | NA | AGCCACGCCATGAGC | GTTATAAGTGGAGGTGGTAGTAGCACATATTACGCAGACTCCGTGAAGGGC | TGGCGAGGTAACCCCACTGACTACGGTATGGACGTC |
| | | | SEQ ID NO:7315 | SEQ ID NO:15327 | SEQ ID NO:23339 |
| | | AA | SHAMS | VISGGGSSTYYADSVKG | WRGNPTDYGMDV |
| | | | SEQ ID NO:7316 | SEQ ID NO:15328 | SEQ ID NO:23340 |
| **iPS:436706** | **21-225_149A11** | NA | AGCTATGGCATGCAC | GTTATATGGTATGATGGAAGTAATAAATACTATGCAGACTCCGTGAAGGGC | GATCGTGACTACGGTGACCCCCCCTACTACTACTACTACGGTATGGACGTC |
| | | | SEQ ID NO:7317 | SEQ ID NO:15329 | SEQ ID NO:23341 |
| | | AA | SYGMH | VIWYDGSNKYYADSVKG | DRDYGDPPYYYYYGMDV |
| | | | SEQ ID NO:7318 | SEQ ID NO:15330 | SEQ ID NO:23342 |
| **iPS:436708** | **21-225_150D3** | NA | ACCTATGGCATGCAC | GTTATATGGTATGGTGGAAGTAATAAAGACTATGCAGACTCCGTGAAGGGC | GATCGGGATTATTGTAGTGGTGGTACCTGCCCTTACTACTACTACTACGGTATGGACGTC |
| | | | SEQ ID NO:7319 | SEQ ID NO:15331 | SEQ ID NO:23343 |
| | | AA | TYGMH | VIWYGGSNKDYADSVKG | DRDYCSGGTCPYYYYYGMDV |
| | | | SEQ ID NO:7320 | SEQ ID NO:15332 | SEQ ID NO:23344 |
| **iPS:436710** | **21-225_150F6** | NA | AGCTATGCCATGAGC | GTTATTAGTGGTGGTGGTAGTAGCACATACTACGCAGACTCCGTGAAGGGC | TGGCGAGGTAACCCCACTGACTACGGTATGGACGTC |
| | | | SEQ ID NO:7321 | SEQ ID NO:15333 | SEQ ID NO:23345 |
| | | AA | SYAMS | VISGGGSSTYYADSVKG | WRGNPTDYGMDV |
| | | | SEQ ID NO:7322 | SEQ ID NO:15334 | SEQ ID NO:23346 |

**FIGURE 49**

| | | | | | |
|---|---|---|---|---|---|
| **iPS:436712** | **21-225_150F9** | NA | AGCTATAACATGAAC | TACATTAGTAGAAGTAGTAATACCAAACACTACGCAGACTCTGTGAAGGGC | GATAGGAGTGGGAGCTACGGGTACTTCTACTACTACGGTTTGGACGTC |
| | | | SEQ ID NO:7323 | SEQ ID NO:15335 | SEQ ID NO:23347 |
| | | AA | SYNMN | YISRSSNTKHYADSVKG | DRSGSYGYFYYYGLDV |
| | | | SEQ ID NO:7324 | SEQ ID NO:15336 | SEQ ID NO:23348 |
| **iPS:436714** | **21-225_150H11** | NA | ACCTATGCCATGAGC | ATTATTAGTGGTGGTGGTAGTAGCACATACTACGCAGACTCCGTGAAGGGC | TGGCGAGGTAACCCCACTGACTACGGTATGGACGTC |
| | | | SEQ ID NO:7325 | SEQ ID NO:15337 | SEQ ID NO:23349 |
| | | AA | TYAMS | IISGGGSSTYYADSVKG | WRGNPTDYGMDV |
| | | | SEQ ID NO:7326 | SEQ ID NO:15338 | SEQ ID NO:23350 |
| **iPS:436716** | **21-225_151F3** | NA | ACCTATGGCATGCAC | GTTATATGGTATGGTGGAAGTAATACAGACTATGCAGACTCCGTGAAGGGC | GATCGGGATTATTGTAGTGGTACTAGCTGCCCTTACTACTACTACTACGGTATGGACGTC |
| | | | SEQ ID NO:7327 | SEQ ID NO:15339 | SEQ ID NO:23351 |
| | | AA | TYGMH | VIWYGGSNTDYADSVKG | DRDYCSGTSCPYYYYYGMDV |
| | | | SEQ ID NO:7328 | SEQ ID NO:15340 | SEQ ID NO:23352 |
| **iPS:436718** | **21-225_151H5** | NA | AGCTATGCCATGAGC | GTTATTAGTGGTGGTGGTAGTAGCACATACTACGCAGACTCCGTGAAGGGC | TGGCGAGGTAACCCCACTGACTACGGTATGGACGTC |
| | | | SEQ ID NO:7329 | SEQ ID NO:15341 | SEQ ID NO:23353 |
| | | AA | SYAMS | VISGGGSSTYYADSVKG | WRGNPTDYGMDV |
| | | | SEQ ID NO:7330 | SEQ ID NO:15342 | SEQ ID NO:23354 |

**FIGURE 49**

| | | | | | |
|---|---|---|---|---|---|
| **iPS:436720** | **21-225_151H6** | NA | GACTATGGCATGCAC | CTTATATGGTATGATGGAAGTAATAAATACTATGCAGACTCCGTGAAGGGC | GATGACCGATCTTGTAGTAGAACCAGCTGCCCTTACTACTACTACTACGGTTTGGACGTC |
| | | | SEQ ID NO:7331 | SEQ ID NO:15343 | SEQ ID NO:23355 |
| | | AA | DYGMH | LIWYDGSNKYYADSVKG | DDRSCSRTSCPYYYYYGLDV |
| | | | SEQ ID NO:7332 | SEQ ID NO:15344 | SEQ ID NO:23356 |
| **iPS:436722** | **21-225_151H7** | NA | AGCTATGCCATGAGC | GTTATTAGTGGTGGTGGTAGTAGCACATACTACGCAGACTCCGTGAAGGGC | TGGCGAGGTAACCCCACTGACTACGGTATGGACGTC |
| | | | SEQ ID NO:7333 | SEQ ID NO:15345 | SEQ ID NO:23357 |
| | | AA | SYAMS | VISGGGSSTYYADSVKG | WRGNPTDYGMDV |
| | | | SEQ ID NO:7334 | SEQ ID NO:15346 | SEQ ID NO:23358 |
| **iPS:436724** | **21-225_151B9** | NA | AGCTATGCCATGAGC | GTTATTAGTGGTGGTGGTAGTAGCACATACTACGCAGACTCCGTGAAGGGC | TGGCGAGGTAACCCCACTGACTACGGTATGGACGTC |
| | | | SEQ ID NO:7335 | SEQ ID NO:15347 | SEQ ID NO:23359 |
| | | AA | SYAMS | VISGGGSSTYYADSVKG | WRGNPTDYGMDV |
| | | | SEQ ID NO:7336 | SEQ ID NO:15348 | SEQ ID NO:23360 |
| **iPS:436726** | **21-225_152G5** | NA | GACTATGGCATGCAC | CTTATATGGTATGATGGAAGTAATAAATACTATGCAGACTCCGTGAAGGGC | GATGACCGATCTTGTAGTAGAACCAGCTGCCCTTACTACTACTACTACGGTTTGGACGTC |
| | | | SEQ ID NO:7337 | SEQ ID NO:15349 | SEQ ID NO:23361 |
| | | AA | DYGMH | LIWYDGSNKYYADSVKG | DDRSCSRTSCPYYYYYGLDV |
| | | | SEQ ID NO:7338 | SEQ ID NO:15350 | SEQ ID NO:23362 |

FIGURE 49

| | | | | | |
|---|---|---|---|---|---|
| **iPS:436728** | **21-225_152G6** | NA | AGCTATGCCATGAGC | GTTATTAGTGGTGGTGGTAGTAGCACATACTACGCAGACTCCGTGAAGGGC | TGGCGAGGTAACCCCACTGACTACGGTATGGACGTC |
| | | | SEQ ID NO:7339 | SEQ ID NO:15351 | SEQ ID NO:23363 |
| | | AA | SYAMS | VISGGGSSTYYADSVKG | WRGNPTDYGMDV |
| | | | SEQ ID NO:7340 | SEQ ID NO:15352 | SEQ ID NO:23364 |
| **iPS:436730** | **21-225_152D7** | NA | AGCTATGCCATGAGC | GTTATTAGTGGTGGTGGTAGTAGCACATACTACGCAGACTCCGTGAAGGGC | TGGCGAGGTAACCCCACTGACTCCGGTATGGACGTC |
| | | | SEQ ID NO:7341 | SEQ ID NO:15353 | SEQ ID NO:23365 |
| | | AA | SYAMS | VISGGGSSTYYADSVKG | WRGNPTDSGMDV |
| | | | SEQ ID NO:7342 | SEQ ID NO:15354 | SEQ ID NO:23366 |
| **iPS:436732** | **21-225_152B12** | NA | GACTATGGCATGCAC | GTTATATGGTATGATGGAAGTAATAAATACTATGCAGACTCCGTGAAGGGC | GATGACCGATCTTGTAGTAGTACCAGCTGCCCTTACTACTACTACTACGGTTTGGACGTC |
| | | | SEQ ID NO:7343 | SEQ ID NO:15355 | SEQ ID NO:23367 |
| | | AA | DYGMH | VIWYDGSNKYYADSVKG | DDRSCSSTSCPYYYYYGLDV |
| | | | SEQ ID NO:7344 | SEQ ID NO:15356 | SEQ ID NO:23368 |
| **iPS:436734** | **21-225_153A8** | NA | GACTATGGCATGCAC | CTTATATGGTATGATGGAAGTAATAAATACTATGCAGACTCCGTGAAGGGC | GATGACCGATCTTGTAGTAGAACCAGCTGCCCTTACTACTACTACTACGGTTTGGACGTC |
| | | | SEQ ID NO:7345 | SEQ ID NO:15357 | SEQ ID NO:23369 |
| | | AA | DYGMH | LIWYDGSNKYYADSVKG | DDRSCSRTSCPYYYYYGLDV |
| | | | SEQ ID NO:7346 | SEQ ID NO:15358 | SEQ ID NO:23370 |

FIGURE 49

| | | | | | |
|---|---|---|---|---|---|
| **iPS:436736** | **21-225_153E8** | NA | AACTATGGCATGCAC | GTTATATGGTTTGATGGCAGTAATAAATACTATGTTGACTCCGTGAAGGAC | GATCGGGTGGAGGGTTCGGGGACTCCCTACTACTACTACGGTATGGACGTC |
| | | | SEQ ID NO:7347 | SEQ ID NO:15359 | SEQ ID NO:23371 |
| | | AA | NYGMH | VIWFDGSNKYYVDSVKD | DRVEGSGTPYYYYGMDV |
| | | | SEQ ID NO:7348 | SEQ ID NO:15360 | SEQ ID NO:23372 |
| **iPS:436738** | **21-225_153D9** | NA | ACCTATGGCATGCAC | GTTATATGGTATGGTGGAAGTAATAAAGACTATGCAGACTCCGTGAAGGGC | GATCGGGATTATTGTAGTGGTGGTAGCTGTCCTTACTACTACTACTACGGTATGGACGTC |
| | | | SEQ ID NO:7349 | SEQ ID NO:15361 | SEQ ID NO:23373 |
| | | AA | TYGMH | VIWYGGSNKDYADSVKG | DRDYCSGGSCPYYYYYGMDV |
| | | | SEQ ID NO:7350 | SEQ ID NO:15362 | SEQ ID NO:23374 |
| **iPS:436740** | **21-225_154C3** | NA | ACCTATGGCATGCAC | GTTGTATGGTATGGTGGAAATAATAAAGACTATGCAGACTCCGTGAAGGGC | GATCGGGATTATTGTAGTGGTGGTAGCTGCCCTTACTACTACTACTACGGTATGGACGTC |
| | | | SEQ ID NO:7351 | SEQ ID NO:15363 | SEQ ID NO:23375 |
| | | AA | TYGMH | VVWYGGNNKDYADSVKG | DRDYCSGGSCPYYYYYGMDV |
| | | | SEQ ID NO:7352 | SEQ ID NO:15364 | SEQ ID NO:23376 |
| **iPS:436742** | **21-225_154C4** | NA | AGCTATGCCATGAGC | GTTATTAGTGGTGGTGGTAGTAGCACATACTACGCAGACTCCGTGAAGGGC | TGGCGAGGTAACCCCACTGACTACGGTATGGACGTC |
| | | | SEQ ID NO:7353 | SEQ ID NO:15365 | SEQ ID NO:23377 |
| | | AA | SYAMS | VISGGGSSTYYADSVKG | WRGNPTDYGMDV |
| | | | SEQ ID NO:7354 | SEQ ID NO:15366 | SEQ ID NO:23378 |

## FIGURE 49

| | | | | | |
|---|---|---|---|---|---|
| **iPS:436744** | **21-225_154F4** | NA | AGCTATGGCATGCAC | GTTATATGGTATGATGGAAGTAATAAATACTATGCAGACTCCGTGAAGGGC | GAGGATTCCTATTGTAGTGGTACCAGCTGCCCCTACTACTACTACTACGGTATGGACGTC |
| | | | SEQ ID NO:7355 | SEQ ID NO:15367 | SEQ ID NO:23379 |
| | | AA | SYGMH | VIWYDGSNKYYADSVKG | EDSYCSGTSCPYYYYYGMDV |
| | | | SEQ ID NO:7356 | SEQ ID NO:15368 | SEQ ID NO:23380 |
| **iPS:436746** | **21-225_154E10** | NA | AGCTATGCCATGAGC | GTTATTAGTGGTGGTGGTAGTAGCACATACTACGCAGACTCCGTGAAGGGC | TGGCGAGGTAACCCCACTGACTACGGTATGGACGTC |
| | | | SEQ ID NO:7357 | SEQ ID NO:15369 | SEQ ID NO:23381 |
| | | AA | SYAMS | VISGGGSSTYYADSVKG | WRGNPTDYGMDV |
| | | | SEQ ID NO:7358 | SEQ ID NO:15370 | SEQ ID NO:23382 |
| **iPS:436748** | **21-225_154D11** | NA | ACCTATGGCATGCAC | GTTATATGGTATGGTGGAAGTAATAAAGACTATGCAGACTCTGTGAAGGGC | GATCGGGATTATTGTAGTGGTGGTAGTTGCCCTTACTACTACTACTACGGTATGGACGTC |
| | | | SEQ ID NO:7359 | SEQ ID NO:15371 | SEQ ID NO:23383 |
| | | AA | TYGMH | VIWYGGSNKDYADSVKG | DRDYCSGGSCPYYYYYGMDV |
| | | | SEQ ID NO:7360 | SEQ ID NO:15372 | SEQ ID NO:23384 |
| **iPS:436750** | **21-225_154G12** | NA | AGTGGTTATTACTACTGGAGC | TACATCTATTATAGTGGGAGCACCTACTACAACCCGTCCCTCAAGAGT | GATTGGGGTGGCTACGATTCGAGTGGCTGGTTCGACCCC |
| | | | SEQ ID NO:7361 | SEQ ID NO:15373 | SEQ ID NO:23385 |
| | | AA | SGYYYWS | YIYYSGSTYYNPSLKS | DWGGYDSSGWFDP |
| | | | SEQ ID NO:7362 | SEQ ID NO:15374 | SEQ ID NO:23386 |

# FIGURE 49

| | | | | | |
|---|---|---|---|---|---|
| **iPS:436752** | **21-225_155H1** | NA | AGCTACTGGATCGGC | CTCATCTATCCTGGTGCCTCTGATACCAGATACAGCCCGTCCTTCCAAGGC | CAGGCCATAGCAAGTCGAGGGAGGTACTACTACTACGGTATGGACGTC |
| | | | SEQ ID NO:7363 | SEQ ID NO:15375 | SEQ ID NO:23387 |
| | | AA | SYWIG | LIYPGASDTRYSPSFQG | QAIASRGRYYYYGMDV |
| | | | SEQ ID NO:7364 | SEQ ID NO:15376 | SEQ ID NO:23388 |
| **iPS:436754** | **21-225_155G3** | NA | AGCTATGGCATGCAC | GTTATATCATATGATGGAAGTAATAAATACTATGCAGACTCCGTGAAGGGC | GATACGGAGAGATGGCTACCATACTCCTACGGTATGGACGTC |
| | | | SEQ ID NO:7365 | SEQ ID NO:15377 | SEQ ID NO:23389 |
| | | AA | SYGMH | VISYDGSNKYYADSVKG | DTERWLPYSYGMDV |
| | | | SEQ ID NO:7366 | SEQ ID NO:15378 | SEQ ID NO:23390 |
| **iPS:436756** | **21-225_146A10** | NA | GGCTATGGCATGCAC | CTTATACGGTATGATGGAAGCGATAAAAACTATGCAGACTCCGTGAAGGGC | GATCGGGTTTTTGTAGTAGTACCAGCTGCCTCTCTTACTACTACTACTACGGTATGGACGTC |
| | | | SEQ ID NO:7367 | SEQ ID NO:15379 | SEQ ID NO:23391 |
| | | AA | GYGMH | LIRYDGSDKNYADSVKG | DRVFCSSTSCLSYYYYYGMDV |
| | | | SEQ ID NO:7368 | SEQ ID NO:15380 | SEQ ID NO:23392 |
| **iPS:436758** | **21-225_155C10** | NA | AGCTATGCCATGAGC | GTTATTAGTGGTGGTGGTAGTAGCACATACTACGCAGACTCCGTGAAGGGC | TGGCGAGGTAACCCCACTGACTACGGTATGGACGTC |
| | | | SEQ ID NO:7369 | SEQ ID NO:15381 | SEQ ID NO:23393 |
| | | AA | SYAMS | VISGGGSSTYYADSVKG | WRGNPTDYGMDV |
| | | | SEQ ID NO:7370 | SEQ ID NO:15382 | SEQ ID NO:23394 |

FIGURE 49

| | | | | | |
|---|---|---|---|---|---|
| **iPS:436760** | **21-225_155E10** | NA | AGCTATGGCATGCAC | GTTATATGGTATGATGGAAGTAATAAATACTATGCAGACTCCGTGAAGGGC | GATCGTGACTACGGTGACCCCCCCTACTACTACTACTACGGTATGGACGTC |
| | | | SEQ ID NO:7371 | SEQ ID NO:15383 | SEQ ID NO:23395 |
| | | AA | SYGMH | VIWYDGSNKYYADSVKG | DRDYGDPPYYYYYGMDV |
| | | | SEQ ID NO:7372 | SEQ ID NO:15384 | SEQ ID NO:23396 |
| **iPS:436762** | **21-225_156H2** | NA | AACTATAACATGAAC | TACATTAGTAGAAGTAGTAATACCAAATACTACGCAGACTCTGTGAAGGGC | GATAGGAGTGGGAGCTACGGGTACTTCTACTACTACGGTATGGACGTC |
| | | | SEQ ID NO:7373 | SEQ ID NO:15385 | SEQ ID NO:23397 |
| | | AA | NYNMN | YISRSSNTKYYADSVKG | DRSGSYGYFYYYGMDV |
| | | | SEQ ID NO:7374 | SEQ ID NO:15386 | SEQ ID NO:23398 |
| **iPS:436764** | **21-225_158E9** | NA | AGCTATGGCATGCAC | GTTATATGGTATGATGGAAGTAGTAAATACTATGCAGACTCCGTGAAGGGC | GATCGGGTTTTTTGTAGTGGTACCAGCTGCCCTTACTACTACTACTACGGTATGGACGTC |
| | | | SEQ ID NO:7375 | SEQ ID NO:15387 | SEQ ID NO:23399 |
| | | AA | SYGMH | VIWYDGSSKYYADSVKG | DRVFCSGTSCPYYYYYGMDV |
| | | | SEQ ID NO:7376 | SEQ ID NO:15388 | SEQ ID NO:23400 |
| **iPS:436766** | **21-225_158D10** | NA | AGCTATGGCATGCAC | GTTATATGGTATGATGGAAGTAATAAATACTATGCAGACTCCGTGAAGGGC | GATCGGGTTTCTTGTAGTAGTACCAGCTGCCCTTACTACTACTACTACGGTATGGACGTC |
| | | | SEQ ID NO:7377 | SEQ ID NO:15389 | SEQ ID NO:23401 |
| | | AA | SYGMH | VIWYDGSNKYYADSVKG | DRVSCSSTSCPYYYYYGMDV |

FIGURE 49

| | | | | | |
|---|---|---|---|---|---|
| | | | SEQ ID NO:7378 | SEQ ID NO:15390 | SEQ ID NO:23402 |
| **iPS:436768** | **21-225_159H8** | NA | ACCTATGGCATGCAC | GTTATATGGTATGATGG AAGTAATAAATACTATG CAGACTCCGTGAAGGGC | GATCGGGTTTCTTGTAGTAG TACCAGCTGCCCTTACTACT ACTACTACGGTATGGACGTC |
| | | | SEQ ID NO:7379 | SEQ ID NO:15391 | SEQ ID NO:23403 |
| | | AA | TYGMH | VIWYDGSNKYYADSVKG | DRVSCSSTSCPYYYYYGMDV |
| | | | SEQ ID NO:7380 | SEQ ID NO:15392 | SEQ ID NO:23404 |
| **iPS:436770** | **21-225_160B12** | NA | AGCTATGGCATGCAC | GTTATATGGTATGATGG AAGTAATAAATACTATG CAGACTCCGTGAAGGGC | GATCGGGTTTCTTGTAGTAG TACCAGCTGCCCTTACTACT ACTACTACGGTATGGACGTC |
| | | | SEQ ID NO:7381 | SEQ ID NO:15393 | SEQ ID NO:23405 |
| | | AA | SYGMH | VIWYDGSNKYYADSVKG | DRVSCSSTSCPYYYYYGMDV |
| | | | SEQ ID NO:7382 | SEQ ID NO:15394 | SEQ ID NO:23406 |
| **iPS:436772** | **21-225_161H3** | NA | AGCTATGGCATGCAC | GTTATATGGTATGATGG AAGTAATAAATACTATG CAGACTCCGTGAAGGGC | GTCGGGTATAGCGGTGGCTG GTACATCTTTGACTAC |
| | | | SEQ ID NO:7383 | SEQ ID NO:15395 | SEQ ID NO:23407 |
| | | AA | SYGMH | VIWYDGSNKYYADSVKG | VGYSGGWYIFDY |
| | | | SEQ ID NO:7384 | SEQ ID NO:15396 | SEQ ID NO:23408 |
| **iPS:436774** | **21-225_161E10** | NA | AGCTATGGCATGCAC | GTTATATGGTATGATGG AAGTAATAAATACTATG TAGACTCCGTGAAGGGC | GATCGGGTTTTTTGTAGTGG TACCAGCTGCCCTTACTACT ACTACTACGGTATGGACGTC |
| | | | SEQ ID NO:7385 | SEQ ID NO:15397 | SEQ ID NO:23409 |

FIGURE 49

| | | | | | |
|---|---|---|---|---|---|
| | | AA | SYGMH | VIWYDGSNKYYVDSVKG | DRVFCSGTSCPYYYYYGMDV |
| | | | SEQ ID NO:7386 | SEQ ID NO:15398 | SEQ ID NO:23410 |
| **iPS:436776** | **21-225_161F12** | NA | AGTGGTGGTTACTACTGGAGC | TACATCTATTACAGTGGGAGCCCCTACTACAACCCGTCCCTCAAGAGT | TCGAATTGTAGTAGTGCCAACTGCTATACGGTGGGGTTCTACTACTACGGTTTGGACGTC |
| | | | SEQ ID NO:7387 | SEQ ID NO:15399 | SEQ ID NO:23411 |
| | | AA | SGGYYWS | YIYYSGSPYYNPSLKS | SNCSSANCYTVGFYYYGLDV |
| | | | SEQ ID NO:7388 | SEQ ID NO:15400 | SEQ ID NO:23412 |
| **iPS:436780** | **21-225_165H3** | NA | AGTGGTGGTTACTACTGGAGC | TACATCTATTACAGTGGGAGCCCCTACTACAATCCGTCCCTCAAGAGT | TCGAATTGTAGTAGTGCCAACTGCTATACGGTGGGGTTCTACTACTACGGTATGGACGTC |
| | | | SEQ ID NO:7389 | SEQ ID NO:15401 | SEQ ID NO:23413 |
| | | AA | SGGYYWS | YIYYSGSPYYNPSLKS | SNCSSANCYTVGFYYYGMDV |
| | | | SEQ ID NO:7390 | SEQ ID NO:15402 | SEQ ID NO:23414 |
| **iPS:436782** | **21-225_166G11** | NA | GGCTATGGCATGCAC | GTTATATGGTATGATGGAAGTAATAAATACTATGCAGACTCCGTGAAGGGC | GATGATAGATATTGTAGTAGTCCCACCTGCCATCCTTACTACTACTACTACGGTCTGGACGTC |
| | | | SEQ ID NO:7391 | SEQ ID NO:15403 | SEQ ID NO:23415 |
| | | AA | GYGMH | VIWYDGSNKYYADSVKG | DDRYCSSPTCHPYYYYYGLDV |
| | | | SEQ ID NO:7392 | SEQ ID NO:15404 | SEQ ID NO:23416 |

FIGURE 49

| | | | | | |
|---|---|---|---|---|---|
| **iPS:436784** | **21-225_169C1** | NA | AGCAATGGCATGCAC | GTTATATGGTATGATGGAAGTAATAAATACTATGCAGACTCCGTGAAGGGC | GATCAGTACAACCGGAACGACGGACCACCAGCTTACTACTACTACTACGGTTTGGACGTC |
| | | | SEQ ID NO:7393 | SEQ ID NO:15405 | SEQ ID NO:23417 |
| | | AA | SNGMH | VIWYDGSNKYYADSVKG | DQYNRNDGPPAYYYYYGLDV |
| | | | SEQ ID NO:7394 | SEQ ID NO:15406 | SEQ ID NO:23418 |
| **iPS:436786** | **21-225_169A6** | NA | AGCAATGGCATGCAC | GTTATATGGTATGATGGAAGTAATAAATACTATGCAGACTCCGTGAAGGGC | GATCAGTACAACCGGAACGACGGACCACCAGCTTACTACTACTACTACGGTATGGACGTC |
| | | | SEQ ID NO:7395 | SEQ ID NO:15407 | SEQ ID NO:23419 |
| | | AA | SNGMH | VIWYDGSNKYYADSVKG | DQYNRNDGPPAYYYYYGMDV |
| | | | SEQ ID NO:7396 | SEQ ID NO:15408 | SEQ ID NO:23420 |
| **iPS:436788** | **21-225_169B7** | NA | AGCTATAGCTTGAAC | TACATTGGTAGTAGTGGCAGTATCATATTCTACGCAGACTCTGTGAAGGGC | GGGGATACAGCTGGGGTTACCTATTACTACGGTATGGACGTC |
| | | | SEQ ID NO:7397 | SEQ ID NO:15409 | SEQ ID NO:23421 |
| | | AA | SYSLN | YIGSSGSIIFYADSVKG | GDTAGVTYYYGMDV |
| | | | SEQ ID NO:7398 | SEQ ID NO:15410 | SEQ ID NO:23422 |
| **iPS:436790** | **21-225_169G11** | NA | AGCTATGGCATGCAC | ATTATATGGTATGATGGAAGTAATAAATACTATGCAGACTCCGTGAAGGGC | GAGGGGGCTACGTATTACCATGGTTCGGGGAGTTATTATCCGGCTACTAACTACGGTATGGACGTC |
| | | | SEQ ID NO:7399 | SEQ ID NO:15411 | SEQ ID NO:23423 |
| | | AA | SYGMH | IIWYDGSNKYYADSVKG | EGATYYHGSGSYYPATNYGMDV |
| | | | SEQ ID NO:7400 | SEQ ID NO:15412 | SEQ ID NO:23424 |

FIGURE 49

| | | | | | |
|---|---|---|---|---|---|
| **iPS:436792** | **21-225_169D12** | NA | AGCTATGGCATGCAC | GTTATATGGTATGATGGAAGTAATAAATATTATGCAGACTCCGTGAAGGGC | CCCCTTTACGATATGGGACTCTACTACGATATGGACGTC |
| | | | SEQ ID NO:7401 | SEQ ID NO:15413 | SEQ ID NO:23425 |
| | | AA | SYGMH | VIWYDGSNKYYADSVKG | PLYDMGLYYDMDV |
| | | | SEQ ID NO:7402 | SEQ ID NO:15414 | SEQ ID NO:23426 |
| **iPS:436794** | **21-225_170F1** | NA | GGCTATGGCATGAAC | ATTATATGGTATGATGGAAATAATAAATACTATGCAGACTCCGTGAAGGGC | GATCGGGTTTATTGTAGTAGTACCAGCTGCCATCCCTATTACTACTACTACGCTATGGACGTC |
| | | | SEQ ID NO:7403 | SEQ ID NO:15415 | SEQ ID NO:23427 |
| | | AA | GYGMN | IIWYDGNNKYYADSVKG | DRVYCSSTSCHPYYYYYAMDV |
| | | | SEQ ID NO:7404 | SEQ ID NO:15416 | SEQ ID NO:23428 |
| **iPS:436796** | **21-225_170A5** | NA | AACTGTGGCATGCAC | ATTATATGGTATGATGGAAGTAATAAATACTATGCAGACTCCGTGAAGGGC | GATCAGTACAACAGGAACGACGGACCACCAGCTTACTACTACTACTACGGTTTGGACGTC |
| | | | SEQ ID NO:7405 | SEQ ID NO:15417 | SEQ ID NO:23429 |
| | | AA | NCGMH | IIWYDGSNKYYADSVKG | DQYNRNDGPPAYYYYYGLDV |
| | | | SEQ ID NO:7406 | SEQ ID NO:15418 | SEQ ID NO:23430 |
| **iPS:436798** | **21-225_171F5** | NA | AGCTATAGCTTGAAC | TACATTGGTAGTAGTGGCAGTATCATATTCTACGCAGACTCTGTGAAGGGC | GGGGATACAGCTGGGGTTACCTATTACTACGGTATGGACGTC |
| | | | SEQ ID NO:7407 | SEQ ID NO:15419 | SEQ ID NO:23431 |
| | | AA | SYSLN | YIGSSGSIIFYADSVKG | GDTAGVTYYYGMDV |
| | | | SEQ ID NO:7408 | SEQ ID NO:15420 | SEQ ID NO:23432 |

**FIGURE 49**

| | | | | | |
|---|---|---|---|---|---|
| **iPS:436800** | **21-225_171D12** | NA | AGTTACTATATGTAT | ATAATCAACCCTAGTGGTGGTAGCACAAACTACGCACAGAAGTTCCAGGGC | GGTTGGGAGTTAAACTAC |
| | | | SEQ ID NO:7409 | SEQ ID NO:15421 | SEQ ID NO:23433 |
| | | AA | SYYMY | IINPSGGSTNYAQKFQG | GWELNY |
| | | | SEQ ID NO:7410 | SEQ ID NO:15422 | SEQ ID NO:23434 |
| **iPS:436802** | **21-225_171E12** | NA | AGTTATGGCATGCAC | GTTATATGGAATGATGGAGGTAATAAATATAATGGAGACTCCGTGAAGGGC | GACCGTACGTATTACTCTGGTTCGGGGAGCCCCCCCTACTACTACTACTACGGTATGGACGTC |
| | | | SEQ ID NO:7411 | SEQ ID NO:15423 | SEQ ID NO:23435 |
| | | AA | SYGMH | VIWNDGGNKYNGDSVKG | DRTYYSGSGSPPYYYYYGMDV |
| | | | SEQ ID NO:7412 | SEQ ID NO:15424 | SEQ ID NO:23436 |
| **iPS:436804** | **21-225_172C3** | NA | AGTTACTATATGTAT | ACAATCAACCCTAGTGGTGGTAGCACAAACTACGCACAGAAGTTCCAGGGC | GGCTGGGAGTTAAACTAC |
| | | | SEQ ID NO:7413 | SEQ ID NO:15425 | SEQ ID NO:23437 |
| | | AA | SYYMY | TINPSGGSTNYAQKFQG | GWELNY |
| | | | SEQ ID NO:7414 | SEQ ID NO:15426 | SEQ ID NO:23438 |
| **iPS:436806** | **21-225_172B12** | NA | AGTTACTATATGTAT | ACAATCAACCCTAGTGGTGGTAGCACAGACTACGCACAGAAGTTCCAGGGC | GGCTGGGAATTAAACTAC |
| | | | SEQ ID NO:7415 | SEQ ID NO:15427 | SEQ ID NO:23439 |
| | | AA | SYYMY | TINPSGGSTDYAQKFQG | GWELNY |
| | | | SEQ ID NO:7416 | SEQ ID NO:15428 | SEQ ID NO:23440 |
| **iPS:436808** | **21-225_173F8** | NA | AGCTATGGCATGCAC | GTTATATCATATGATGGAAGTCCTAAATACTGTGCAGACTCCGTGAAGGGC | GATGAAAGGCAGTGGCTGCCGGCCCCCTACGGTATGGACGTC |
| | | | SEQ ID NO:7417 | SEQ ID NO:15429 | SEQ ID NO:23441 |

## FIGURE 49

| | | | | | |
|---|---|---|---|---|---|
| | | AA | SYGMH | VISYDGSPKYCADSVKG | DERQWLPAPYGMDV |
| | | | SEQ ID NO:7418 | SEQ ID NO:15430 | SEQ ID NO:23442 |
| iPS:436810 | 21-225_175F4 | NA | AGCAACAGTGCTGCTTGGAAC | AGGACATACTACAGGTCCAAGTGGTATAATGCTTATCCAGTATCTATGGAAAGT | GATAAGGCAGCTGGGAGGAATGACTTCTACTACTACGGTATGGACGTC |
| | | | SEQ ID NO:7419 | SEQ ID NO:15431 | SEQ ID NO:23443 |
| | | AA | SNSAAWN | RTYYRSKWYNAYPVSMES | DKAAGRNDFYYYGMDV |
| | | | SEQ ID NO:7420 | SEQ ID NO:15432 | SEQ ID NO:23444 |
| iPS:436812 | 21-225_175C6 | NA | AACTGTGGCATGCAC | ATTATATGGTATGATGGAAGTAATAAATACTATGCAGACTCCGTGAAGGGC | GATCAGTACAACAGGAACGACGGACCACCAGCTTACTACTACTACTACGGTTTGGACGTC |
| | | | SEQ ID NO:7421 | SEQ ID NO:15433 | SEQ ID NO:23445 |
| | | AA | NCGMH | IIWYDGSNKYYADSVKG | DQYNRNDGPPAYYYYYGLDV |
| | | | SEQ ID NO:7422 | SEQ ID NO:15434 | SEQ ID NO:23446 |
| iPS:436814 | 21-225_178H10 | NA | AGCAACAGTGCTGCTTGGAAC | AGGACATACTACAGGTCCAAGTGGTATAGTGCTTATCCAGTATCTATGGAAAGT | GATAAGGCAGCTGGGAGGAATGACTTCTACTACTACGGTATGGACGTC |
| | | | SEQ ID NO:7423 | SEQ ID NO:15435 | SEQ ID NO:23447 |
| | | AA | SNSAAWN | RTYYRSKWYSAYPVSMES | DKAAGRNDFYYYGMDV |
| | | | SEQ ID NO:7424 | SEQ ID NO:15436 | SEQ ID NO:23448 |
| iPS:436816 | 21-225_179H5 | NA | AGCTATGGCATGCAC | GTTATATGGTATGATGGAAGTAATGAATACTATGCAGACTCCGTGAAGGGC | GATATCCGGAACTACTACTACGGTTTGGACGTC |
| | | | SEQ ID NO:7425 | SEQ ID NO:15437 | SEQ ID NO:23449 |

FIGURE 49

| | | | | | |
|---|---|---|---|---|---|
| | | AA | SYGMH | VIWYDGSNEYYADSVKG | DIRNYYYGLDV |
| | | | SEQ ID NO:7426 | SEQ ID NO:15438 | SEQ ID NO:23450 |
| **iPS:436818** | **21-225_179C7** | NA | AACTCTGGCATGCAC | ATTATATATTATGATGGAAGTTATAAATACAATGCAGACTCCGTGAAGGGC | GACCGTCATTACGATTTCCACGTTCCCTACTATTACTATTACGGTATGGACGTC |
| | | | SEQ ID NO:7427 | SEQ ID NO:15439 | SEQ ID NO:23451 |
| | | AA | NSGMH | IIYYDGSYKYNADSVKG | DRHYDFHVPYYYYYGMDV |
| | | | SEQ ID NO:7428 | SEQ ID NO:15440 | SEQ ID NO:23452 |
| **iPS:436820** | **21-225_179D10** | NA | AGCTATAGCATGAAC | TACATTAGTAGTAGTGGAAGTACCACATACTACGCAGACTCTGTGCAGGGC | GATAGTAGGAAGGGGTTCTACTACGGTCTGGACGTC |
| | | | SEQ ID NO:7429 | SEQ ID NO:15441 | SEQ ID NO:23453 |
| | | AA | SYSMN | YISSSGSTTYYADSVQG | DSRKGFYYGLDV |
| | | | SEQ ID NO:7430 | SEQ ID NO:15442 | SEQ ID NO:23454 |
| **iPS:436822** | **21-225_180D4** | NA | AACTTTGGCATGCAC | ATTATATGGTATGATGGAAGTGATAAATACTATGCAGACTCCGTGAAGGGC | GGGGGGCCCCCGTTCTCTACGGTGACTATGTACTTTGACTAC |
| | | | SEQ ID NO:7431 | SEQ ID NO:15443 | SEQ ID NO:23455 |
| | | AA | NFGMH | IIWYDGSDKYYADSVKG | GGPPFSTVTMYFDY |
| | | | SEQ ID NO:7432 | SEQ ID NO:15444 | SEQ ID NO:23456 |
| **iPS:436824** | **21-225_180C5** | NA | ACTAGTGGAGTGGGTGTGGGC | TTCATTTCTTGGAATGATGATAAGCGCTACAGCCCATCTCTGAAGAGC | AAAGCAGCAGCTGTTGCTTTTGATATC |
| | | | SEQ ID NO:7433 | SEQ ID NO:15445 | SEQ ID NO:23457 |
| | | AA | TSGVGVG | FISWNDDKRYSPSLKS | KAAAVAFDI |
| | | | SEQ ID NO:7434 | SEQ ID NO:15446 | SEQ ID NO:23458 |

FIGURE 49

| | | | | | |
|---|---|---|---|---|---|
| **iPS:436826** | **21-225_180G5** | NA | AGTTATGATATCAAC | TGGATGAACCCTAACAGTGGTAACACAGGCTATGCACAGAAGTTCCAGGGC | GGGTTTTATTACTATGGTTCGGGGAGTCATGTCCCTTACCACTACTACTACGGTTTGGACGTC |
| | | | SEQ ID NO:7435 | SEQ ID NO:15447 | SEQ ID NO:23459 |
| | | AA | SYDIN | WMNPNSGNTGYAQKFQG | GFYYYGSGSHVPYHYYYGLDV |
| | | | SEQ ID NO:7436 | SEQ ID NO:15448 | SEQ ID NO:23460 |
| **iPS:436828** | **21-225_181H1** | NA | AACTATGGCATGCAC | ATTATATGGTATGATGGAAGTGATAAATACTATGCAGACTCCGTGAAGGGC | GGGGGGCCCCCGTTTTCTACGGTGACTATGTACTTCGACTAC |
| | | | SEQ ID NO:7437 | SEQ ID NO:15449 | SEQ ID NO:23461 |
| | | AA | NYGMH | IIWYDGSDKYYADSVKG | GGPPFSTVTMYFDY |
| | | | SEQ ID NO:7438 | SEQ ID NO:15450 | SEQ ID NO:23462 |
| **iPS:436830** | **21-225_51F4** | NA | AGCTATGGTATCAGC | TGGATCAGCGCTTATAATGGTAACACAAAGTATGCACAGAAGCTCCAGGGC | CACGATTTTTGGAGTGGTTATTATAAGGGTATGGACGTC |
| | | | SEQ ID NO:7439 | SEQ ID NO:15451 | SEQ ID NO:23463 |
| | | AA | SYGIS | WISAYNGNTKYAQKLQG | HDFWSGYYKGMDV |
| | | | SEQ ID NO:7440 | SEQ ID NO:15452 | SEQ ID NO:23464 |
| **iPS:436832** | **21-225_51D8** | NA | AGTAACAGTGCTGCTTGGAAC | AGGACATACTACAGGTCCAAGTGGTATAATGATTATGCAGTATCTGTGAAAAGT | GACCGCTATAACTGGAACTACCCCTACTGGTACTTCGATCTC |
| | | | SEQ ID NO:7441 | SEQ ID NO:15453 | SEQ ID NO:23465 |
| | | AA | SNSAAWN | RTYYRSKWYNDYAVSVKS | DRYNWNYPYWYFDL |
| | | | SEQ ID NO:7442 | SEQ ID NO:15454 | SEQ ID NO:23466 |

FIGURE 49

| | | | | | |
|---|---|---|---|---|---|
| **iPS:436834** | **21-225_52F1** | NA | AGCTATGGTGTCAGC | TGGATCAGCGCTTATAATGGTAACAGAAAGTATGCACAGAAGCTCCAGGGC | CACGATTTTTGGAGTGGTTATTATAAGGGTATGGACGTC |
| | | | SEQ ID NO:7443 | SEQ ID NO:15455 | SEQ ID NO:23467 |
| | | AA | SYGVS | WISAYNGNRKYAQKLQG | HDFWSGYYKGMDV |
| | | | SEQ ID NO:7444 | SEQ ID NO:15456 | SEQ ID NO:23468 |
| **iPS:436836** | **21-225_52H1** | NA | GGCTATGGCATGCAC | GTTATATGGTATGATGGAAGTAATAAATACTATGCAGACTCCGTGAAGGGC | GATCGGGTCTATTGTAGTAGTTCCAGCTGCTCATATTACTACTACTACTACGGTATGGACGTC |
| | | | SEQ ID NO:7445 | SEQ ID NO:15457 | SEQ ID NO:23469 |
| | | AA | GYGMH | VIWYDGSNKYYADSVKG | DRVYCSSSSCSYYYYYYGMDV |
| | | | SEQ ID NO:7446 | SEQ ID NO:15458 | SEQ ID NO:23470 |
| **iPS:436838** | **21-225_52H4** | NA | CACTTTGGCATGCAC | GTTATTTGGTATGATGGAAGTAATAAATACTATGCAGACTCCGTGAAGGGC | GGGGACTGGAACTACGAGGGTTTTGACTAC |
| | | | SEQ ID NO:7447 | SEQ ID NO:15459 | SEQ ID NO:23471 |
| | | AA | HFGMH | VIWYDGSNKYYADSVKG | GDWNYEGFDY |
| | | | SEQ ID NO:7448 | SEQ ID NO:15460 | SEQ ID NO:23472 |
| **iPS:436840** | **21-225_53E9** | NA | GGCTACTATATGCAC | TGGATCATCCCTAACAGTGGTGACACAAACTATGCACAGAAGTTTCAGGGC | GATGGGTATAGCAGTGGCTGGTTCAACTGGTTCGACCCC |
| | | | SEQ ID NO:7449 | SEQ ID NO:15461 | SEQ ID NO:23473 |
| | | AA | GYYMH | WIIPNSGDTNYAQKFQG | DGYSSGWFNWFDP |
| | | | SEQ ID NO:7450 | SEQ ID NO:15462 | SEQ ID NO:23474 |

**FIGURE 49**

| | | | | | |
|---|---|---|---|---|---|
| **iPS:436842** | **21-225_54E9** | NA | AGCTATGGTATCAGC | TGGATTAGTGCTTATAATGGTAACACAAAGAATGCACAGAAGCTCCAGGGC | CACGATTTTTGGAGTGGTTATTATAAGGGTATGGACGTC |
| | | | SEQ ID NO:7451 | SEQ ID NO:15463 | SEQ ID NO:23475 |
| | | AA | SYGIS | WISAYNGNTKNAQKLQG | HDFWSGYYKGMDV |
| | | | SEQ ID NO:7452 | SEQ ID NO:15464 | SEQ ID NO:23476 |
| **iPS:436844** | **21-225_56G1** | NA | AGCTATGGTATCAGC | TGGATCAGCGCTTATAATGGTAACACAAAGTATGCACAGAAGTTCCAGGGC | CACGATTTTTGGAGTGGTTATTATAAGGGTATGGACGTC |
| | | | SEQ ID NO:7453 | SEQ ID NO:15465 | SEQ ID NO:23477 |
| | | AA | SYGIS | WISAYNGNTKYAQKFQG | HDFWSGYYKGMDV |
| | | | SEQ ID NO:7454 | SEQ ID NO:15466 | SEQ ID NO:23478 |
| **iPS:436846** | **21-225_56E3** | NA | AGCTATGGTTTCAGC | TGGATCAGCGCTTATAATGGTAACACAAAGGAAGCACAGAAGTTCCAGGGC | CACGATTTTTGGAGTGGTTATTATAAGGGTATGGACGTC |
| | | | SEQ ID NO:7455 | SEQ ID NO:15467 | SEQ ID NO:23479 |
| | | AA | SYGFS | WISAYNGNTKEAQKFQG | HDFWSGYYKGMDV |
| | | | SEQ ID NO:7456 | SEQ ID NO:15468 | SEQ ID NO:23480 |
| **iPS:436848** | **21-225_57F1** | NA | ACTAGTGGAGTGGGTGTGGGC | CTCATTTATTGGCATGAAGATAAGCGCTACAGCCCATCTCTGAAGAGC | GTCACAGGTATAGCAGCTCCCTAC |
| | | | SEQ ID NO:7457 | SEQ ID NO:15469 | SEQ ID NO:23481 |
| | | AA | TSGVGVG | LIYWHEDKRYSPSLKS | VTGIAAPY |
| | | | SEQ ID NO:7458 | SEQ ID NO:15470 | SEQ ID NO:23482 |

FIGURE 49

| | | | | | |
|---|---|---|---|---|---|
| **iPS:436850** | **21-225_57D9** | NA | ACTAGTGGAGTGGGTGTGGGC | CTCATTTATTGGAATGATGATAAGCGCTACAGTCCATCTCTGAAGAGC | GCAGTGGCTGTCTCCTTTGACTAC |
| | | | SEQ ID NO:7459 | SEQ ID NO:15471 | SEQ ID NO:23483 |
| | | AA | TSGVGVG | LIYWNDDKRYSPSLKS | AVAVSFDY |
| | | | SEQ ID NO:7460 | SEQ ID NO:15472 | SEQ ID NO:23484 |
| **iPS:436852** | **21-225_57H11** | NA | ACTAGTGGAGTGGGTGTGGGC | CTCATTTATTGGCATGAAGATAGGCGCTACAGCCCATCTCTGAAGAGC | GTCACAGGTATAGCAGCTCCCTAC |
| | | | SEQ ID NO:7461 | SEQ ID NO:15473 | SEQ ID NO:23485 |
| | | AA | TSGVGVG | LIYWHEDRRYSPSLKS | VTGIAAPY |
| | | | SEQ ID NO:7462 | SEQ ID NO:15474 | SEQ ID NO:23486 |
| **iPS:436854** | **21-225_58C1** | NA | ACTAGTGGAGTGGGTGTGGGC | CTCATTTATTGGGATGATGATAAGCGCTACAGCCCATCTCTGAAGAGC | CTTATAGCAGTGGCCTTTGACTCC |
| | | | SEQ ID NO:7463 | SEQ ID NO:15475 | SEQ ID NO:23487 |
| | | AA | TSGVGVG | LIYWDDDKRYSPSLKS | LIAVAFDS |
| | | | SEQ ID NO:7464 | SEQ ID NO:15476 | SEQ ID NO:23488 |
| **iPS:436856** | **21-225_58C5** | NA | AGCTATAGCATGAAC | TCCATTAGTAGTAGTAGTTATTACTTATACTACGCAGACTCAGTGAAGGGC | ACCTATAGTGGGAGTTTTGACTAC |
| | | | SEQ ID NO:7465 | SEQ ID NO:15477 | SEQ ID NO:23489 |
| | | AA | SYSMN | SISSSSYYLYYADSVKG | TYSGSFDY |
| | | | SEQ ID NO:7466 | SEQ ID NO:15478 | SEQ ID NO:23490 |
| **iPS:436858** | **21-225_58E7** | NA | TTCTATGGCATGCAC | GTTACATCATATGATGGAAGTGATAAATACTATGCAGACTCCGTGAAGGGC | GATGACTATGGTTCGGGGAGTCCCCTATACTACGGTATGGACGTC |
| | | | SEQ ID NO:7467 | SEQ ID NO:15479 | SEQ ID NO:23491 |
| | | AA | FYGMH | VTSYDGSDKYYADSVKG | DDYGSGSPLYYGMDV |

**FIGURE 49**

| | | | | | |
|---|---|---|---|---|---|
| | | | SEQ ID NO:7468 | SEQ ID NO:15480 | SEQ ID NO:23492 |
| **iPS:436860** | **21-225_58F7** | NA | AGCTTTTGGATGAGC | CACATAAAGCAAGATGGAAGTGAGAAATACTATGTGGACTCTGTGAAGGGC | GGGGACCTCCCATACAGCTCGGGCTACTACTACGGTATGGACGTC |
| | | | SEQ ID NO:7469 | SEQ ID NO:15481 | SEQ ID NO:23493 |
| | | AA | SFWMS | HIKQDGSEKYYVDSVKG | GDLPYSSGYYYGMDV |
| | | | SEQ ID NO:7470 | SEQ ID NO:15482 | SEQ ID NO:23494 |
| **iPS:436862** | **21-225_58F8** | NA | AGCTATGGCATGCAC | GTTATATCATATGATGGAAGTAATAAATACTATGCAGACTCCGTGAAGGGC | GATGAGGGACGTGGATATGGTGGCTACGAGAGGGGATATTACTACTACTACTACGGTATGGACGTC |
| | | | SEQ ID NO:7471 | SEQ ID NO:15483 | SEQ ID NO:23495 |
| | | AA | SYGMH | VISYDGSNKYYADSVKG | DEGRGYGGYERGYYYYYGMDV |
| | | | SEQ ID NO:7472 | SEQ ID NO:15484 | SEQ ID NO:23496 |
| **iPS:436864** | **21-225_58G11** | NA | AGCTATAGCATGAAC | TACATTAGTACTAGTAGTAGTACCATATTCTACGCAGACTCTGTGAAGGGC | GGGGATACAGCTATGGTCCTCTACTACTACGGTATGGACGTC |
| | | | SEQ ID NO:7473 | SEQ ID NO:15485 | SEQ ID NO:23497 |
| | | AA | SYSMN | YISTSSSTIFYADSVKG | GDTAMVLYYYGMDV |
| | | | SEQ ID NO:7474 | SEQ ID NO:15486 | SEQ ID NO:23498 |
| **iPS:436866** | **21-225_59F2** | NA | AGCTATAGCATGAAC | TACATTAGTGGGAGTAGTAATATCATATACTACACAGACTCTGTGAAGGGC | GCGGATACACCTATGGTCCTTTACTTCTACGGTATGGACGTC |
| | | | SEQ ID NO:7475 | SEQ ID NO:15487 | SEQ ID NO:23499 |
| | | AA | SYSMN | YISGSSNIIYYTDSVKG | ADTPMVLYFYGMDV |
| | | | SEQ ID NO:7476 | SEQ ID NO:15488 | SEQ ID NO:23500 |

## FIGURE 49

| | | | | | |
|---|---|---|---|---|---|
| **iPS:436868** | **21-225_59B11** | NA | AGTTATGGCGTGCAC | GCTATATGGTATGATGGAAGTAATAAATACTATGCAGACTCCGTGAAGGGC | GATAGGGACTATTGTAGTAGTTCCAGCTGCCCTTACTACTACTACTACGGTATGGACGTC |
| | | | SEQ ID NO:7477 | SEQ ID NO:15489 | SEQ ID NO:23501 |
| | | AA | SYGVH | AIWYDGSNKYYADSVKG | DRDYCSSSSCPYYYYYGMDV |
| | | | SEQ ID NO:7478 | SEQ ID NO:15490 | SEQ ID NO:23502 |
| **iPS:436870** | **21-225_60B1** | NA | ACTAGTGGAGTGGGTGTGGGC | CTCATTTATTGGCATGAAGATAAGCGCTACAGCCCATCTCTGAAGAGC | GTCACATATATAGCAGCTCCCTAC |
| | | | SEQ ID NO:7479 | SEQ ID NO:15491 | SEQ ID NO:23503 |
| | | AA | TSGVGVG | LIYWHEDKRYSPSLKS | VTYIAAPY |
| | | | SEQ ID NO:7480 | SEQ ID NO:15492 | SEQ ID NO:23504 |
| **iPS:436872** | **21-225_60D2** | NA | AGCTATAGCATGAAC | TACATTAGTGAGAGTAGTAATATCATATACTACACAGACTCTGTGAAGGGC | GCGGATACACCTATGGTCCTTTACTTCTACGGTATGGACGTC |
| | | | SEQ ID NO:7481 | SEQ ID NO:15493 | SEQ ID NO:23505 |
| | | AA | SYSMN | YISESSNIIYYTDSVKG | ADTPMVLYFYGMDV |
| | | | SEQ ID NO:7482 | SEQ ID NO:15494 | SEQ ID NO:23506 |
| **iPS:436874** | **21-225_60A12** | NA | ACTAGTGGAGTGGGTGTGGGC | CTCATTTATTGGGATGATGATAAGCGCTACAGCCCATCTCTGAAGAGC | CTTATAGCAGTGGCCTTTGACTCC |
| | | | SEQ ID NO:7483 | SEQ ID NO:15495 | SEQ ID NO:23507 |
| | | AA | TSGVGVG | LIYWDDDKRYSPSLKS | LIAVAFDS |
| | | | SEQ ID NO:7484 | SEQ ID NO:15496 | SEQ ID NO:23508 |
| **iPS:436876** | **21-225_61F5** | NA | ACTAGTGGGTTGGGTGTGGGC | CTCATTTATTCACATGAAGATAAGCGCTACAGCCCATCTCTGAAGAGC | GTCACAGGTATAGCAGCTCCCTAC |
| | | | SEQ ID NO:7485 | SEQ ID NO:15497 | SEQ ID NO:23509 |

FIGURE 49

| | | | | | |
|---|---|---|---|---|---|
| | | AA | TSGLGVG | LIYSHEDKRYSPSLKS | VTGIAAPY |
| | | | SEQ ID NO:7486 | SEQ ID NO:15498 | SEQ ID NO:23510 |
| **iPS:436878** | **21-225_62E3** | NA | ACTAGTGGAGTGGGTGTGGGC | CTCATTAATTGGAATGATGATAAGCGCTACAGCCCATCTCTGAAGAGC | AAAGCTACCTGGGTGGCTTTTGATATC |
| | | | SEQ ID NO:7487 | SEQ ID NO:15499 | SEQ ID NO:23511 |
| | | AA | TSGVGVG | LINWNDDKRYSPSLKS | KATWVAFDI |
| | | | SEQ ID NO:7488 | SEQ ID NO:15500 | SEQ ID NO:23512 |
| **iPS:436880** | **21-225_62E8** | NA | ACTAGTGGAGTGGGTGTGGGC | CTCATTAATTGGAATGATGATAAGCGCTACAGCCCATCTCTGAAGAGC | AAAGCTACCTGGGTGGCTTTTGATATC |
| | | | SEQ ID NO:7489 | SEQ ID NO:15501 | SEQ ID NO:23513 |
| | | AA | TSGVGVG | LINWNDDKRYSPSLKS | KATWVAFDI |
| | | | SEQ ID NO:7490 | SEQ ID NO:15502 | SEQ ID NO:23514 |
| **iPS:436882** | **21-225_62D10** | NA | ACTAGTGGAGTGGGTGTGGGC | CTCATTAATTGGAATGATGATAAGCGCTACAGCCCATCTCTGAAGAGC | AAAGCTACCTGGGTGGCTTTTGATATC |
| | | | SEQ ID NO:7491 | SEQ ID NO:15503 | SEQ ID NO:23515 |
| | | AA | TSGVGVG | LINWNDDKRYSPSLKS | KATWVAFDI |
| | | | SEQ ID NO:7492 | SEQ ID NO:15504 | SEQ ID NO:23516 |
| **iPS:436884** | **21-225_62A12** | NA | ACTAGTGGAGTGGGTGTGGGC | CTCATTAATTGGAATGATGATAAACGCTACAGCCCATCTCTGAAGAGC | AAAACTACCTGGGTGGCTTTTGATATC |
| | | | SEQ ID NO:7493 | SEQ ID NO:15505 | SEQ ID NO:23517 |
| | | AA | TSGVGVG | LINWNDDKRYSPSLKS | KTTWVAFDI |
| | | | SEQ ID NO:7494 | SEQ ID NO:15506 | SEQ ID NO:23518 |
| **iPS:436886** | **21-225_62B12** | NA | ACTAGTGGAGTGGGTGTGGGC | CTCATTAATTGGAATGATGATAAGCGCTACAGCCCGTCTCTGAAGAGC | AAAGCTACCTGGGTGGCTTTTGATATC |
| | | | SEQ ID NO:7495 | SEQ ID NO:15507 | SEQ ID NO:23519 |

FIGURE 49

| | | | | | |
|---|---|---|---|---|---|
| | | AA | TSGVGVG | LINWNDDKRYSPSLKS | KATWVAFDI |
| | | | SEQ ID NO:7496 | SEQ ID NO:15508 | SEQ ID NO:23520 |
| **iPS:436888** | **21-225_63G7** | NA | AGCTATAGCATGAAC | TACATTAGTAGTAGTACTAGTACCATATACTACGCAGCCTCTGTGAAGGGC | GATCACCGTTACTATGATAGTAGTGGTTATTACTCTGATGCTTTTGATATC |
| | | | SEQ ID NO:7497 | SEQ ID NO:15509 | SEQ ID NO:23521 |
| | | AA | SYSMN | YISSSTSTIYYAASVKG | DHRYYDSSGYYSDAFDI |
| | | | SEQ ID NO:7498 | SEQ ID NO:15510 | SEQ ID NO:23522 |
| **iPS:436890** | **21-225_63A10** | NA | AGCTATAGCATGAAC | TACATTAGTAGTAGTACTAGTACCATATACTACGCAGCCTCTGTGAAGGGC | GATCACCGTTACTATGATAGTAGTGGTTATTACTCTGATGCTTTTGATATC |
| | | | SEQ ID NO:7499 | SEQ ID NO:15511 | SEQ ID NO:23523 |
| | | AA | SYSMN | YISSSTSTIYYAASVKG | DHRYYDSSGYYSDAFDI |
| | | | SEQ ID NO:7500 | SEQ ID NO:15512 | SEQ ID NO:23524 |
| **iPS:436892** | **21-225_65E9** | NA | GGCTACTATATGCAC | TGGATCAACCCTAACAGTGGTGGCACAAATTATGCACAGAAGTTTCAGGGC | GCGTATTATTATGGTTCGGGGAGTTATTATAATGAATTTGATATG |
| | | | SEQ ID NO:7501 | SEQ ID NO:15513 | SEQ ID NO:23525 |
| | | AA | GYYMH | WINPNSGGTNYAQKFQG | AYYYGSGSYYNEFDM |
| | | | SEQ ID NO:7502 | SEQ ID NO:15514 | SEQ ID NO:23526 |
| **iPS:436894** | **21-225_66G9** | NA | ACTAGTGGAGTGGGTGTGGGC | CTCATTAATTGGAATGATGATAAGCGCTTCAGCCCATCTCTGAAGAGC | AAAGCTACCTGGGTGGCTTTTGATATC |
| | | | SEQ ID NO:7503 | SEQ ID NO:15515 | SEQ ID NO:23527 |
| | | AA | TSGVGVG | LINWNDDKRFSPSLKS | KATWVAFDI |
| | | | SEQ ID NO:7504 | SEQ ID NO:15516 | SEQ ID NO:23528 |
| **iPS:436896** | **21-225_67F10** | NA | GGCTACTATATGCAC | TGGATCAACCCTAACAGTGGTGGCACAAACTATGGACAGAAGTTTCAGGGC | ACGTATTTCTATGGTTCGGGGAGTTATTATAACGGCTTTGACTAC |
| | | | SEQ ID NO:7505 | SEQ ID NO:15517 | SEQ ID NO:23529 |

**FIGURE 49**

| | | | | | |
|---|---|---|---|---|---|
| | | AA | GYYMH | WINPNSGGTNYGQKFQG | TYFYGSGSYYNGFDY |
| | | | SEQ ID NO:7506 | SEQ ID NO:15518 | SEQ ID NO:23530 |
| **iPS:436898** | **21-225_68D8** | NA | AGCAACAGTGCTGCTTGGAAC | AGGACATACTACAGGTCCGAGTGCTATAATGATTATGCAGTATCTGTGCAGAGT | GATAGAGGGCATAGAGGGTTCTACGGTATGGACGTC |
| | | | SEQ ID NO:7507 | SEQ ID NO:15519 | SEQ ID NO:23531 |
| | | AA | SNSAAWN | RTYYRSECYNDYAVSVQS | DRGHRGFYGMDV |
| | | | SEQ ID NO:7508 | SEQ ID NO:15520 | SEQ ID NO:23532 |
| **iPS:436900** | **21-225_69B9** | NA | GGCTACCATATGCAC | TGGATCAACCCTAACAGTGGTGGCACAAATTATGCACAGAAGTTTCAGGGC | GCGTATTATTATGGTTCGGGGAGTTATTATAATGAATCTGATATG |
| | | | SEQ ID NO:7509 | SEQ ID NO:15521 | SEQ ID NO:23533 |
| | | AA | GYHMH | WINPNSGGTNYAQKFQG | AYYYGSGSYYNESDM |
| | | | SEQ ID NO:7510 | SEQ ID NO:15522 | SEQ ID NO:23534 |
| **iPS:436902** | **21-225_69B11** | NA | GGCTACTATATGCAC | TGGATCAACCCTAACAGTGGTGGCACAAACTATGGACAGAAGTTTCAGGAC | ACGTATTACTATGGGTCGGGGAGTTATTATAACGGCTTTGACTAC |
| | | | SEQ ID NO:7511 | SEQ ID NO:15523 | SEQ ID NO:23535 |
| | | AA | GYYMH | WINPNSGGTNYGQKFQD | TYYYGSGSYYNGFDY |
| | | | SEQ ID NO:7512 | SEQ ID NO:15524 | SEQ ID NO:23536 |
| **iPS:436904** | **21-225_71D4** | NA | GGCTACTGTATGCAC | TGGATCAACCCTAACAGTGGTGGCACAAACTATGCACAGAAGTTTCAGGTC | GCGTATTACTATGGTTCGGGGACTTATCATAACGAATTTGACTAC |
| | | | SEQ ID NO:7513 | SEQ ID NO:15525 | SEQ ID NO:23537 |
| | | AA | GYCMH | WINPNSGGTNYAQKFQV | AYYYGSGTYHNEFDY |
| | | | SEQ ID NO:7514 | SEQ ID NO:15526 | SEQ ID NO:23538 |

**FIGURE 49**

| | | | | | |
|---|---|---|---|---|---|
| **iPS:436906** | **21-225_72B4** | NA | GGCTACTATATGCAC | TGGATCAACCCTAACAGTGGTGGCACAAACTATGGACAGAAGTTTCAGGGC | ACGTATTACTATGGTTCGGGGAGTTATTATAACGGCTTTGACTAC |
| | | | SEQ ID NO:7515 | SEQ ID NO:15527 | SEQ ID NO:23539 |
| | | AA | GYYMH | WINPNSGGTNYGQKFQG | TYYYGSGSYYNGFDY |
| | | | SEQ ID NO:7516 | SEQ ID NO:15528 | SEQ ID NO:23540 |
| **iPS:436908** | **21-225_72D5** | NA | ACTAGTGGAGTGGGTGTGGGC | CTCATTAATTGGAATGATGATAAGCGCTACAGCCCATCTCTGAAGAGC | AAAGCTACCTGGGTGGCTTTTGATATC |
| | | | SEQ ID NO:7517 | SEQ ID NO:15529 | SEQ ID NO:23541 |
| | | AA | TSGVGVG | LINWNDDKRYSPSLKS | KATWVAFDI |
| | | | SEQ ID NO:7518 | SEQ ID NO:15530 | SEQ ID NO:23542 |
| **iPS:436910** | **21-225_73G1** | NA | TACTATGGCATGCAC | GTTACAACATATGATGGAAGTAATAAATACTATGCAGACTCCGTGAAGGGC | GAGACTGGAACCTGGGCTTTTGATATC |
| | | | SEQ ID NO:7519 | SEQ ID NO:15531 | SEQ ID NO:23543 |
| | | AA | YYGMH | VTTYDGSNKYYADSVKG | ETGTWAFDI |
| | | | SEQ ID NO:7520 | SEQ ID NO:15532 | SEQ ID NO:23544 |
| **iPS:436912** | **21-225_73C4** | NA | ACTAGTGGAGTGGGTGTGGGC | CTCATTAATTGGAATGATGATAAGCGCTACAGCCCATCTCTGAAGAGC | AAAACTACCTGGGTGGCTTTTGATATC |
| | | | SEQ ID NO:7521 | SEQ ID NO:15533 | SEQ ID NO:23545 |
| | | AA | TSGVGVG | LINWNDDKRYSPSLKS | KTTWVAFDI |
| | | | SEQ ID NO:7522 | SEQ ID NO:15534 | SEQ ID NO:23546 |
| **iPS:436914** | **21-225_76B4** | NA | ACTGGTGGAGTGGGTGTGGGC | CTCATTTATTGGGATGATGATAAGCGCTACAGCCCATCTCTGAAGAGC | CTTATAGCAGTGGCCTTTGACTAC |
| | | | SEQ ID NO:7523 | SEQ ID NO:15535 | SEQ ID NO:23547 |

**FIGURE 49**

| | | | | | |
|---|---|---|---|---|---|
| | | AA | TGGVGVG | LIYWDDDKRYSPSLKS | LIAVAFDY |
| | | | SEQ ID NO:7524 | SEQ ID NO:15536 | SEQ ID NO:23548 |
| **iPS:436916** | **21-225_74A9** | NA | AGCTATGGCATGCAC | GTTATATGGTATGATGGAAATAATAAATCCTATGCAGACTCCGTGAAGGGC | GATCGAGATTATTGTAGTGGTACCAGCTGCCCTTATTATTACTACTACGGTATGGACGTC |
| | | | SEQ ID NO:7525 | SEQ ID NO:15537 | SEQ ID NO:23549 |
| | | AA | SYGMH | VIWYDGNNKSYADSVKG | DRDYCSGTSCPYYYYYGMDV |
| | | | SEQ ID NO:7526 | SEQ ID NO:15538 | SEQ ID NO:23550 |
| **iPS:436918** | **21-225_77A2** | NA | ACTAGTGGAGTGGGTGTGGGC | TTCATTTATTGGGATGATGATAAGCGCTACAGCCCATCTCTGAAGAGC | CTTATAGCAGTGGCCTTTGACTAC |
| | | | SEQ ID NO:7527 | SEQ ID NO:15539 | SEQ ID NO:23551 |
| | | AA | TSGVGVG | FIYWDDDKRYSPSLKS | LIAVAFDY |
| | | | SEQ ID NO:7528 | SEQ ID NO:15540 | SEQ ID NO:23552 |
| **iPS:436920** | **21-225_74E5** | NA | AGTGGTGGTTACTACTGGAGC | TACATCTATTACAGTGGGAGCACCTACTACAACCCGTCCCTCAGGAGT | GATTCACCAGTGGCTGGTACTGACTAC |
| | | | SEQ ID NO:7529 | SEQ ID NO:15541 | SEQ ID NO:23553 |
| | | AA | SGGYYWS | YIYYSGSTYYNPSLRS | DSPVAGTDY |
| | | | SEQ ID NO:7530 | SEQ ID NO:15542 | SEQ ID NO:23554 |
| **iPS:436922** | **21-225_78E9** | NA | AGCTATGGCATGCAC | GTTATATGGTATGATGGAAATAATAAATCCTATGCAGACTCCGTGAAGGGC | GATCGAGATTATTGTAGTAGTACCAGCTGCCCTTATTATTACTACTACGGTATGGACGTC |
| | | | SEQ ID NO:7531 | SEQ ID NO:15543 | SEQ ID NO:23555 |
| | | AA | SYGMH | VIWYDGNNKSYADSVKG | DRDYCSSTSCPYYYYYGMDV |
| | | | SEQ ID NO:7532 | SEQ ID NO:15544 | SEQ ID NO:23556 |

## FIGURE 49

| | | | | | |
|---|---|---|---|---|---|
| **iPS:436924** | **21-225_74B3** | NA | CGATATGGCATGCAC | GTTTTTGGTATGATGGAAGTAATAAAGACTATGCAGACTCCGTGAAGGGC | GATCGAGATTATTGTAGTAGTACCAGCTGCCCTTACTACTACTACTACGGTATGGACGTC |
| | | | SEQ ID NO:7533 | SEQ ID NO:15545 | SEQ ID NO:23557 |
| | | AA | RYGMH | VFWYDGSNKDYADSVKG | DRDYCSSTSCPYYYYYGMDV |
| | | | SEQ ID NO:7534 | SEQ ID NO:15546 | SEQ ID NO:23558 |
| **iPS:436926** | **21-225_78D10** | NA | AGTGGTGGTTACTACTGGAGC | TACATCTATTACATTGGGAGTGTTTACTACAACCCGTCCCTCAAGAGT | GATGCCCCCGACTTCGGTATGGACGTC |
| | | | SEQ ID NO:7535 | SEQ ID NO:15547 | SEQ ID NO:23559 |
| | | AA | SGGYYWS | YIYYIGSVYYNPSLKS | DAPDFGMDV |
| | | | SEQ ID NO:7536 | SEQ ID NO:15548 | SEQ ID NO:23560 |
| **iPS:436928** | **21-225_79E7** | NA | AGCTATGGCATGCAC | GTTATATGGTATGATGGAAATAATAAATCCTATGCAGACTCCGTGAAGGGC | GATCGAGATTATTGTAGTAGTACCAGCTGCCCTTATTATTACTACTACGGTATGGACGTC |
| | | | SEQ ID NO:7537 | SEQ ID NO:15549 | SEQ ID NO:23561 |
| | | AA | SYGMH | VIWYDGNNKSYADSVKG | DRDYCSSTSCPYYYYYGMDV |
| | | | SEQ ID NO:7538 | SEQ ID NO:15550 | SEQ ID NO:23562 |
| **iPS:436932** | **21-225_92A4** | NA | AGCTATGGCATGCAC | GTTATATGGTATGATGGAAATAATAAATCCTATGCAGACTCCGTGAAGGGC | GATCGAGATTATTGTAGTAGTACCAGCTGCCCTTATTATTACTACTACGGTATGGACGTC |
| | | | SEQ ID NO:7539 | SEQ ID NO:15551 | SEQ ID NO:23563 |
| | | AA | SYGMH | VIWYDGNNKSYADSVKG | DRDYCSSTSCPYYYYYGMDV |
| | | | SEQ ID NO:7540 | SEQ ID NO:15552 | SEQ ID NO:23564 |

FIGURE 49

| | | | | | |
|---|---|---|---|---|---|
| **iPS:436934** | **21-225_96B5** | NA | ACTGGTGGAGTGGGTGTGGGC | CTCATTTATTGGGATGATGATAAGCGCTACAGCCCATCTCTGAAGAGC | CTTATAGCAGTGGCCTGTGACTAC |
| | | | SEQ ID NO:7541 | SEQ ID NO:15553 | SEQ ID NO:23565 |
| | | AA | TGGVGVG | LIYWDDDKRYSPSLKS | LIAVACDY |
| | | | SEQ ID NO:7542 | SEQ ID NO:15554 | SEQ ID NO:23566 |
| **iPS:436936** | **21-225_97E6** | NA | AGCTATGGCATGCAC | GTTATATGGTATGATGGAAATAATAAATCCTATGCAGACTCCGTGAAGGGC | GATCGAGATTATTGTAGTAGTACCAGCTGCCCTTATTATTACTACTACGGTATGGACGTC |
| | | | SEQ ID NO:7543 | SEQ ID NO:15555 | SEQ ID NO:23567 |
| | | AA | SYGMH | VIWYDGNNKSYADSVKG | DRDYCSSTSCPYYYYYGMDV |
| | | | SEQ ID NO:7544 | SEQ ID NO:15556 | SEQ ID NO:23568 |
| **iPS:436938** | **21-225_146A3** | NA | AGCTATGCCATGAGC | GTTATTAGTGGTGGTGGTACTACCACATACTACGCAGACTCCGTGAAGGGC | TGGCGAGGTAACCCCACTGACTACGGTATGGACGTC |
| | | | SEQ ID NO:7545 | SEQ ID NO:15557 | SEQ ID NO:23569 |
| | | AA | SYAMS | VISGGGTTTYYADSVKG | WRGNPTDYGMDV |
| | | | SEQ ID NO:7546 | SEQ ID NO:15558 | SEQ ID NO:23570 |
| **iPS:436940** | **21-225_146B8** | NA | ACCTATGGCATGCAC | GTTGTATGGTATGGTGGAAATGATAAAGACTTTGCAGACTCCGTGACGGGC | GATCGGGATTATTGTAGTGGTGGTAGCTGCCCTTACTACTACTACTACGGTATGGACGTC |
| | | | SEQ ID NO:7547 | SEQ ID NO:15559 | SEQ ID NO:23571 |
| | | AA | TYGMH | VVWYGGNDKDFADSVTG | DRDYCSGGSCPYYYYYGMDV |
| | | | SEQ ID NO:7548 | SEQ ID NO:15560 | SEQ ID NO:23572 |

# FIGURE 49

| | | | | | |
|---|---|---|---|---|---|
| **iPS:436942** | **21-225_146H8** | NA | AGTTATGATATCAAT | TGGATGAACCCTAACAGTGGTAACACAGGCTATGCACAGAAGTTCCAGGGC | GGAGATTATTACTATGATAGTAGTGGTCACCAGCCTTACTACTACTACTACGGTATGGACGTC |
| | | | SEQ ID NO:7549 | SEQ ID NO:15561 | SEQ ID NO:23573 |
| | | AA | SYDIN | WMNPNSGNTGYAQKFQG | GDYYYDSSGHQPYYYYYGMDV |
| | | | SEQ ID NO:7550 | SEQ ID NO:15562 | SEQ ID NO:23574 |
| **iPS:436944** | **21-225_182D12** | NA | ACTACTGGAGTGGGTGTGGGC | ATCCTTTTTGGAATGATGATGAGCGCTACAGCCCATCTCTGAAGAGC | AAATCGCAGCTCGTCTACTTTGACTAC |
| | | | SEQ ID NO:7551 | SEQ ID NO:15563 | SEQ ID NO:23575 |
| | | AA | TTGVGVG | ILFWNDDERYSPSLKS | KSQLVYFDY |
| | | | SEQ ID NO:7552 | SEQ ID NO:15564 | SEQ ID NO:23576 |
| **iPS:436946** | **21-225_183F4** | NA | AGCTATGGCATGCAC | GTTATATGGTATGATGGAAGTAATAAATACTATGCAGACTCCGTGAAGGGC | GAAAGGACGTATTGTAGTGGTACCACCTGCCCCTACTACTACTACTACGGTCTGGGCGTC |
| | | | SEQ ID NO:7553 | SEQ ID NO:15565 | SEQ ID NO:23577 |
| | | AA | SYGMH | VIWYDGSNKYYADSVKG | ERTYCSGTTCPYYYYYGLGV |
| | | | SEQ ID NO:7554 | SEQ ID NO:15566 | SEQ ID NO:23578 |
| **iPS:436948** | **21-225_183F5** | NA | AGTTATGGCATGCTC | GTTATATGGTATGATGGAAGTGGTAAATACTATGCAGACTCCGTGAAGGGC | GAGAATTTTTGGAGTGGTGACTAC |
| | | | SEQ ID NO:7555 | SEQ ID NO:15567 | SEQ ID NO:23579 |
| | | AA | SYGML | VIWYDGSGKYYADSVKG | ENFWSGDY |
| | | | SEQ ID NO:7556 | SEQ ID NO:15568 | SEQ ID NO:23580 |

FIGURE 49

| | | | | | |
|---|---|---|---|---|---|
| **iPS:436950** | **21-225_184G4** | NA | AGCTATGGCATGCAC | ATTATATGGTATGATGGAAGTAATAAATACTATGCAGACTCCGTGAAGGGC | GGGGGGCCCCGTTCTCTACGGTGACTATGTACTTTGACTAC |
| | | | SEQ ID NO:7557 | SEQ ID NO:15569 | SEQ ID NO:23581 |
| | | AA | SYGMH | IIWYDGSNKYYADSVKG | GGPPFSTVTMYFDY |
| | | | SEQ ID NO:7558 | SEQ ID NO:15570 | SEQ ID NO:23582 |
| **iPS:436952** | **21-225_185D2** | NA | AACTATGGCATGCAC | ATTATATGGTATGATGGAAGTGATAAATACTATGCAGACTCCGTGAAGGGC | GGGGGGCCCCGTTCTCTACGGTGACTATGTACTTTGACTAC |
| | | | SEQ ID NO:7559 | SEQ ID NO:15571 | SEQ ID NO:23583 |
| | | AA | NYGMH | IIWYDGSDKYYADSVKG | GGPPFSTVTMYFDY |
| | | | SEQ ID NO:7560 | SEQ ID NO:15572 | SEQ ID NO:23584 |
| **iPS:436954** | **21-225_185G7** | NA | ACTGGTGGAGTGGGTGTGGGC | CTCATTTATTGGAATGATGATGAGCGCTACAGCCCATCTCTGAAGAGC | ATTATAGCAGTGGCCTTCCAGCAT |
| | | | SEQ ID NO:7561 | SEQ ID NO:15573 | SEQ ID NO:23585 |
| | | AA | TGGVGVG | LIYWNDDERYSPSLKS | IIAVAFQH |
| | | | SEQ ID NO:7562 | SEQ ID NO:15574 | SEQ ID NO:23586 |
| **iPS:436956** | **21-225_186H6** | NA | ACTAGTGGAGTGGGTGTGGGC | TTCATTTCTTGGAATGATGATAAGCGCTACAGCCCATCTCTGAAGAGC | AAAGCAGCAGCTGTTGCTTTTGATATC |
| | | | SEQ ID NO:7563 | SEQ ID NO:15575 | SEQ ID NO:23587 |
| | | AA | TSGVGVG | FISWNDDKRYSPSLKS | KAAAVAFDI |
| | | | SEQ ID NO:7564 | SEQ ID NO:15576 | SEQ ID NO:23588 |
| **iPS:436958** | **21-225_190D1** | NA | AGTGGTGGTTACTACTGGAGC | TACATCTATTACAGTGGGAGCACCTACTACAACCCGTCCCTCAAGAGT | GATTCCCCACTACGAGGCTTTGACTAC |
| | | | SEQ ID NO:7565 | SEQ ID NO:15577 | SEQ ID NO:23589 |
| | | AA | SGGYYWS | YIYYSGSTYYNPSLKS | DSPLRGFDY |

# FIGURE 49

| | | | | | |
|---|---|---|---|---|---|
| | | | SEQ ID NO:7566 | SEQ ID NO:15578 | SEQ ID NO:23590 |
| **iPS:436960** | **21-225_198D2** | NA | AGCTATGGCATGCAT | GTTATAATATATGATGGAAGTTATAAGTACTATGCAGACTCCGTGAAGGGC | ACGTATAGCGGGGGTATGGACGTC |
| | | | SEQ ID NO:7567 | SEQ ID NO:15579 | SEQ ID NO:23591 |
| | | AA | SYGMH | VIIYDGSYKYYADSVKG | TYSGGMDV |
| | | | SEQ ID NO:7568 | SEQ ID NO:15580 | SEQ ID NO:23592 |
| **iPS:436962** | **21-225_190H1** | NA | AGGAAAAGTGCTACTTGGAAC | AAGACATACTACAGGTCCAAGTGGTATAATGATTATGCAGTATCTGTGAAAAGT | GATCCGGGTGGCCTCTTTGACTAC |
| | | | SEQ ID NO:7569 | SEQ ID NO:15581 | SEQ ID NO:23593 |
| | | AA | RKSATWN | KTYYRSKWYNDYAVSVKS | DPGGLFDY |
| | | | SEQ ID NO:7570 | SEQ ID NO:15582 | SEQ ID NO:23594 |
| **iPS:436964** | **21-225_190B3** | NA | AACTATGGCATACAC | GTTATATGGTTTGATGGAGATAATAAATACTATGCAGACTCCGTGAAGGGC | GATAACTGGAACTACGGCGATCACTACTACTACTTCGGTATGGACGTC |
| | | | SEQ ID NO:7571 | SEQ ID NO:15583 | SEQ ID NO:23595 |
| | | AA | NYGIH | VIWFDGDNKYYADSVKG | DNWNYGDHYYYFGMDV |
| | | | SEQ ID NO:7572 | SEQ ID NO:15584 | SEQ ID NO:23596 |
| **iPS:436966** | **21-225_190C3** | NA | AGCTATGGCATGCAC | GTTATATGGTATGATGGAAGTAATAAATACTATGCAGACTCCGTGAAGGGC | TGGTACTACTACTACTACGGTATGGACGTC |
| | | | SEQ ID NO:7573 | SEQ ID NO:15585 | SEQ ID NO:23597 |
| | | AA | SYGMH | VIWYDGSNKYYADSVKG | WYYYYYGMDV |

FIGURE 49

| | | | | | |
|---|---|---|---|---|---|
| | | | SEQ ID NO:7574 | SEQ ID NO:15586 | SEQ ID NO:23598 |
| **iPS:436968** | **21-225_190B10** | NA | AGCTATGGCATGCAC | GTTATATGGAATGATGGAAGTAAAAAATACCATGTAGACTCCGTGAAGGGC | GATCTGGATAAGAGGAACTTTCCTTATTACTACTACTACGGTATGGACGTC |
| | | | SEQ ID NO:7575 | SEQ ID NO:15587 | SEQ ID NO:23599 |
| | | AA | SYGMH | VIWNDGSKKYHVDSVKG | DLDKRNFPYYYYYGMDV |
| | | | SEQ ID NO:7576 | SEQ ID NO:15588 | SEQ ID NO:23600 |
| **iPS:436970** | **21-225_190B8** | NA | AGCTATGGCATGCAC | GTTATATGGTTTGATGGAAGTAATAAATACTATACAGACTCCGTGAAGGGC | GATAACTGGAACTACGGCGATTACTACTACTACTACGGTATGGACGTC |
| | | | SEQ ID NO:7577 | SEQ ID NO:15589 | SEQ ID NO:23601 |
| | | AA | SYGMH | VIWFDGSNKYYTDSVKG | DNWNYGDYYYYYGMDV |
| | | | SEQ ID NO:7578 | SEQ ID NO:15590 | SEQ ID NO:23602 |
| **iPS:436972** | **21-225_190C7** | NA | GGCTACTATATGCAC | TGGATCAACCCTAACAGTGGTGGCACAAACTATGCACAGAAGTTTCAGGGC | GATAGAGCAGTGGCTGGAAACTACTTCTACTACGGTATGGACGTC |
| | | | SEQ ID NO:7579 | SEQ ID NO:15591 | SEQ ID NO:23603 |
| | | AA | GYYMH | WINPNSGGTNYAQKFQG | DRAVAGNYFYYGMDV |
| | | | SEQ ID NO:7580 | SEQ ID NO:15592 | SEQ ID NO:23604 |
| **iPS:436974** | **21-225_190H7** | NA | AGCTATGGCATGCAT | GTTATAATATATGATGGAAGTTATAAGTACTATGCAGACTCCGTGAAGGGC | ACGTATAGCGGGGGTATGGACGTC |
| | | | SEQ ID NO:7581 | SEQ ID NO:15593 | SEQ ID NO:23605 |
| | | AA | SYGMH | VIIYDGSYKYYADSVKG | TYSGGMDV |
| | | | SEQ ID NO:7582 | SEQ ID NO:15594 | SEQ ID NO:23606 |

# FIGURE 49

| | | | | | |
|---|---|---|---|---|---|
| **iPS:436976** | **21-225_190D8** | NA | AGCTATGGCCTGCAC | GTTATATGGTATGATGGAAGTAATAAATACTATGCAGACTCCGTGAAGGGC | TGGTACTACTACTACTACGGTATGGACGTC |
| | | | SEQ ID NO:7583 | SEQ ID NO:15595 | SEQ ID NO:23607 |
| | | AA | SYGLH | VIWYDGSNKYYADSVKG | WYYYYYGMDV |
| | | | SEQ ID NO:7584 | SEQ ID NO:15596 | SEQ ID NO:23608 |
| **iPS:436978** | **21-225_190G9** | NA | AGGAAAAGTGCTACTTGGAAC | AGGACATACTACAGGTCCAAGTGGTATAATGATTATGCAGTATCTGTGAAAAGT | GATCCGGGTGGCCTCTTTGACTAC |
| | | | SEQ ID NO:7585 | SEQ ID NO:15597 | SEQ ID NO:23609 |
| | | AA | RKSATWN | RTYYRSKWYNDYAVSVKS | DPGGLFDY |
| | | | SEQ ID NO:7586 | SEQ ID NO:15598 | SEQ ID NO:23610 |
| **iPS:436980** | **21-225_190C10** | NA | AACTATGGCATGCAC | GTTATATGGTTTGGTGGAGATAATAAATACTATGCAGACTCCGTGAGGGGC | GATAACTGGAACTACGGCGATCACTACTACTATTACGGAATGGACGTC |
| | | | SEQ ID NO:7587 | SEQ ID NO:15599 | SEQ ID NO:23611 |
| | | AA | NYGMH | VIWFGGDNKYYADSVRG | DNWNYGDHYYYYGMDV |
| | | | SEQ ID NO:7588 | SEQ ID NO:15600 | SEQ ID NO:23612 |
| **iPS:436982** | **21-225_190D10** | NA | AGCTATGGCATGCAT | GTTATAATATATGATGGAAGTTATAAGTACTATGCAGACTCCGTGAAGGGC | ACGTATAGCGGGGGTATGGACGTC |
| | | | SEQ ID NO:7589 | SEQ ID NO:15601 | SEQ ID NO:23613 |
| | | AA | SYGMH | VIIYDGSYKYYADSVKG | TYSGGMDV |
| | | | SEQ ID NO:7590 | SEQ ID NO:15602 | SEQ ID NO:23614 |

FIGURE 49

| | | | | | |
|---|---|---|---|---|---|
| **iPS:436984** | **21-225_190F10** | NA | AGTGGTGGTGACTACTGGAGC | TACATCTATTACAGTGGGATCACCTACTACAATCCGTCCCTCAAGAGT | GATAGCAGCTCGCGGGGTATGGACGTC |
| | | | SEQ ID NO:7591 | SEQ ID NO:15603 | SEQ ID NO:23615 |
| | | AA | SGGDYWS | YIYYSGITYYNPSLKS | DSSSRGMDV |
| | | | SEQ ID NO:7592 | SEQ ID NO:15604 | SEQ ID NO:23616 |
| **iPS:436986** | **21-225_191A1** | NA | AGTTACTACTGGATC | TATATCTATTACAGTGGGAGTACTAAGTACAACCCCTCCCTCAAGAGT | AAGGGAGTGGGAACCATCCACTTTGACTAC |
| | | | SEQ ID NO:7593 | SEQ ID NO:15605 | SEQ ID NO:23617 |
| | | AA | SYYWI | YIYYSGSTKYNPSLKS | KGVGTIHFDY |
| | | | SEQ ID NO:7594 | SEQ ID NO:15606 | SEQ ID NO:23618 |
| **iPS:436988** | **21-225_191A2** | NA | AGTGGTGGTGACTACTGGAGC | TACATCTATTACAGTGGGATCACCTACTACAATCCGTCCCTCAAGAGT | GATAGCAGCTCGCGGGGTATGGACGTC |
| | | | SEQ ID NO:7595 | SEQ ID NO:15607 | SEQ ID NO:23619 |
| | | AA | SGGDYWS | YIYYSGITYYNPSLKS | DSSSRGMDV |
| | | | SEQ ID NO:7596 | SEQ ID NO:15608 | SEQ ID NO:23620 |
| **iPS:436992** | **21-225_191B8** | NA | AGCTATGGCATGCAC | GTTATATGGTTTGATGGAAGTAATAAATACTATGCAGACTCCGTGAAGGGC | GATAACTGGAACTACGGCGATCACTACTACTACTACGGTATGGACGTC |
| | | | SEQ ID NO:7597 | SEQ ID NO:15609 | SEQ ID NO:23621 |
| | | AA | SYGMH | VIWFDGSNKYYADSVKG | DNWNYGDHYYYYGMDV |
| | | | SEQ ID NO:7598 | SEQ ID NO:15610 | SEQ ID NO:23622 |
| **iPS:436994** | **21-225_191A9** | NA | AATTATGGCATGCAC | GTTATATGGTTTGGTGGAGATAATAAATACTATGCAGACTCCGTGAAGGGC | GATAACTGGAACTACGGCGATCACTACTACTATTACGGTATGGACGTC |
| | | | SEQ ID NO:7599 | SEQ ID NO:15611 | SEQ ID NO:23623 |

# FIGURE 49

| | | | | | |
|---|---|---|---|---|---|
| | | AA | NYGMH | VIWFGGDNKYYADSVKG | DNWNYGDHYYYYGMDV |
| | | | SEQ ID NO:7600 | SEQ ID NO:15612 | SEQ ID NO:23624 |
| iPS:436996 | 21-225_191B9 | NA | TTCCATGGCATGCAC | GTTATATGGTATGATGG AAGTAAAAAATACTATG CAGACTCCGTGAAGGGC | GAAGGGTATAGCAGTGGCTT TTACAGGGGGTTTGACAAC |
| | | | SEQ ID NO:7601 | SEQ ID NO:15613 | SEQ ID NO:23625 |
| | | AA | FHGMH | VIWYDGSKKYYADSVKG | EGYSSGFYRGFDN |
| | | | SEQ ID NO:7602 | SEQ ID NO:15614 | SEQ ID NO:23626 |
| iPS:437000 | 21-225_191G9 | NA | ACCTATGGCATGCAC | CTTATATGGTTTGATGGA AGTAATAAATACTATGC AGACTCCGTGAAGGGC | GATCGGGTGGGAGGTACTAG TCCTCCTTACTACTACTACTA CGGTATGGACGTC |
| | | | SEQ ID NO:7603 | SEQ ID NO:15615 | SEQ ID NO:23627 |
| | | AA | TYGMH | LIWFDGSNKYYADSVKG | DRVGGTSPPYYYYYGMDV |
| | | | SEQ ID NO:7604 | SEQ ID NO:15616 | SEQ ID NO:23628 |
| iPS:437002 | 21-225_191H9 | NA | GGCTACAATATGCAC | TGGATCAACCCTAATAG TGGTGGCACAAACTATG CACACAAGTTTCAGGGC | GATTTCTATGATAGTGGTGG AGAAGGGTGGTTCGACCCC |
| | | | SEQ ID NO:7605 | SEQ ID NO:15617 | SEQ ID NO:23629 |
| | | AA | GYNMH | WINPNSGGTNYAHKFQG | DFYDSGGEGWFDP |
| | | | SEQ ID NO:7606 | SEQ ID NO:15618 | SEQ ID NO:23630 |
| iPS:437006 | 21-225_192G2 | NA | AGCTATGGCATGCAC | GTTATATGGAATGATGG AAGTAATAAATACTATG CAGACTCCGTGAAGGGC | GATCTGGATAAGAGGAACTT TCCTTATTACTACTACTACGG TATGGACGTC |
| | | | SEQ ID NO:7607 | SEQ ID NO:15619 | SEQ ID NO:23631 |

FIGURE 49

| | | | | | |
|---|---|---|---|---|---|
| | | AA | SYGMH | VIWNDGSNKYYADSVKG | DLDKRNFPYYYYYGMDV |
| | | | SEQ ID NO:7608 | SEQ ID NO:15620 | SEQ ID NO:23632 |
| **iPS:437008** | **21-225_192E3** | NA | AGTGGTGGTTACTACTGGAGC | TACATCTATTACACTGGGAGCACCTACTACAACCCGTCCCTCAAGAGT | GACGATCCCCTCTACGGAATGGACGTC |
| | | | SEQ ID NO:7609 | SEQ ID NO:15621 | SEQ ID NO:23633 |
| | | AA | SGGYYWS | YIYYTGSTYYNPSLKS | DDPLYGMDV |
| | | | SEQ ID NO:7610 | SEQ ID NO:15622 | SEQ ID NO:23634 |
| **iPS:437010** | **21-225_192G3** | NA | AATTACTACTGGAGC | CGGATCTATTCCAGTGGGAGCACCAACTACAACCCCTCCCTCAAGAGT | GGGTGGGAGCTAAACTAC |
| | | | SEQ ID NO:7611 | SEQ ID NO:15623 | SEQ ID NO:23635 |
| | | AA | NYYWS | RIYSSGSTNYNPSLKS | GWELNY |
| | | | SEQ ID NO:7612 | SEQ ID NO:15624 | SEQ ID NO:23636 |
| **iPS:437012** | **21-225_192G7** | NA | AGTGGTGGTTACTACTGGAGC | TACATCTATTACAGAGGGAGTACCTACTACAATCCGTCCCTCAAGAGT | GACTCCCCGGTGACAGGATTTGACTAT |
| | | | SEQ ID NO:7613 | SEQ ID NO:15625 | SEQ ID NO:23637 |
| | | AA | SGGYYWS | YIYYRGSTYYNPSLKS | DSPVTGFDY |
| | | | SEQ ID NO:7614 | SEQ ID NO:15626 | SEQ ID NO:23638 |
| **iPS:437014** | **21-225_192H8** | NA | AGTGGTGGTGACTACTGGAGC | TACATCTATTACAGTGGGCCCACCTACTACAACCCGTCCCTCAAGAGT | GATAGCTCCCTCTACGGTATGGACGTC |
| | | | SEQ ID NO:7615 | SEQ ID NO:15627 | SEQ ID NO:23639 |
| | | AA | SGGDYWS | YIYYSGPTYYNPSLKS | DSSLYGMDV |
| | | | SEQ ID NO:7616 | SEQ ID NO:15628 | SEQ ID NO:23640 |
| **iPS:437016** | **21 225 193A6** | NA | AGTTACTACTGGAGC | TATATCTATTACAGTGGGAGCACCAACTACAACCCCTCCCTCAAGAGT | GGATGGGAGCTAAACTAC |

**FIGURE 49**

| | | | | | |
|---|---|---|---|---|---|
| | **21-225_193A6** | | SEQ ID NO:7617 | SEQ ID NO:15629 | SEQ ID NO:23641 |
| | | AA | SYYWS | YIYYSGSTNYNPSLKS | GWELNY |
| | | | SEQ ID NO:7618 | SEQ ID NO:15630 | SEQ ID NO:23642 |
| **iPS:437018** | **21-225_193H5** | NA | AACGCCTACATGACC | CGTATTAAAAGCAAAACTGATGGTGGGACAACAGACTACGCTGCACCCGTGAAAGGC | GATCCCGGTGGTATCTTTGACTAC |
| | | | SEQ ID NO:7619 | SEQ ID NO:15631 | SEQ ID NO:23643 |
| | | AA | NAYMT | RIKSKTDGGTTDYAAPVKG | DPGGIFDY |
| | | | SEQ ID NO:7620 | SEQ ID NO:15632 | SEQ ID NO:23644 |
| **iPS:437020** | **21-225_193F11** | NA | GGCTACTATATGCAC | TGGATCAACCCTTACAGTGGTGGCACAAACTATGCACAGAAGTTTCAGGGC | GATAGAGCAGTGGCTGGAAACTACTTCTACTACGGTATGGACGTC |
| | | | SEQ ID NO:7621 | SEQ ID NO:15633 | SEQ ID NO:23645 |
| | | AA | GYYMH | WINPYSGGTNYAQKFQG | DRAVAGNYFYYGMDV |
| | | | SEQ ID NO:7622 | SEQ ID NO:15634 | SEQ ID NO:23646 |
| **iPS:437022** | **21-225_194G5** | NA | AGTGGTGGTGACTACTGGAGC | TACATCTATTACAGTGGGAGCACCTACTACAACCCGTCTCTCAAGAGT | GATCACTCCCTCTACGGTATGGACGTC |
| | | | SEQ ID NO:7623 | SEQ ID NO:15635 | SEQ ID NO:23647 |
| | | AA | SGGDYWS | YIYYSGSTYYNPSLKS | DHSLYGMDV |
| | | | SEQ ID NO:7624 | SEQ ID NO:15636 | SEQ ID NO:23648 |
| **iPS:437024** | **21-225_194F11** | NA | AGCTATGGCATGCAC | GTTATATGGAATGATGGAAGTAAAAAATACCATGTAGACTCCGTGAAGGGC | GATCTGGATAAGAGGAACTTTCCTTATTACTACTACTACGGTATGGACGTC |
| | | | SEQ ID NO:7625 | SEQ ID NO:15637 | SEQ ID NO:23649 |
| | | AA | SYGMH | VIWNDGSKKYHVDSVKG | DLDKRNFPYYYYYGMDV |

FIGURE 49

| | | | | | |
|---|---|---|---|---|---|
| | | | SEQ ID NO:7626 | SEQ ID NO:15638 | SEQ ID NO:23650 |
| **iPS:437026** | **21-225_194D12** | NA | AGTGGTGGTGACTACTGGAGC | TACATCTATTACAGTGGGAGTACCTACTACAACCCGTCCCTCAAGAGT | GATGGGGCTCGGCACGGTATGGACGTC |
| | | | SEQ ID NO:7627 | SEQ ID NO:15639 | SEQ ID NO:23651 |
| | | AA | SGGDYWS | YIYYSGSTYYNPSLKS | DGARHGMDV |
| | | | SEQ ID NO:7628 | SEQ ID NO:15640 | SEQ ID NO:23652 |
| **iPS:437028** | **21-225_194G12** | NA | AGCTATGGCATGCAC | GTTATATGGAATGATGGAAGTAATAAATACTATGCAGACTCCGTGAAGGGC | GATCTGGATAAGAGGAACTTTCCTTATTACTACTACTACGGTATGGACGTC |
| | | | SEQ ID NO:7629 | SEQ ID NO:15641 | SEQ ID NO:23653 |
| | | AA | SYGMH | VIWNDGSNKYYADSVKG | DLDKRNFPYYYYYGMDV |
| | | | SEQ ID NO:7630 | SEQ ID NO:15642 | SEQ ID NO:23654 |
| **iPS:437030** | **21-225_195E3** | NA | GACTACTACATGAGC | TATATTACTAGTAGTGGTAATACCATATACTACGCAGACTCTGTGAAGGGC | GATAGTCGATATTTTGACTGGTTTGACTAC |
| | | | SEQ ID NO:7631 | SEQ ID NO:15643 | SEQ ID NO:23655 |
| | | AA | DYYMS | YITSSGNTIYYADSVKG | DSRYFDWFDY |
| | | | SEQ ID NO:7632 | SEQ ID NO:15644 | SEQ ID NO:23656 |
| **iPS:437032** | **21-225_195H6** | NA | AATTACTACTGGAGC | CGTATCTATAGCAGTGGGAGCACCAACTACAACCCCTCCCTCAAGAGT | GGGTGGGAGCTAAACAAC |
| | | | SEQ ID NO:7633 | SEQ ID NO:15645 | SEQ ID NO:23657 |
| | | AA | NYYWS | RIYSSGSTNYNPSLKS | GWELNN |
| | | | SEQ ID NO:7634 | SEQ ID NO:15646 | SEQ ID NO:23658 |
| **iPS:437034** | **21 225 195E9** | NA | GGCTACTATATGCAC | TGGATCAACCCTAACAGTGGTGCCACAAACTATGCACAGAAGTTTCAGGGC | GCCTATTACTATGGTTCGGGGACTTATTATAACGAGTTCGACTAC |

FIGURE 49

| | | | | | |
|---|---|---|---|---|---|
| | 21-225_195E9 | | SEQ ID NO:7635 | SEQ ID NO:15647 | SEQ ID NO:23659 |
| | | AA | GYYMH | WINPNSGATNYAQKFQG | AYYYGSGTYYNEFDY |
| | | | SEQ ID NO:7636 | SEQ ID NO:15648 | SEQ ID NO:23660 |
| iPS:437036 | 21-225_195H9 | NA | GGCTACTATATGCAC | TGGATCAACCCTTACAGTGGTGGCACAAACTATGCACAGAAGTTTCAGGAC | GATAGAGCAGTGGCTGGAAACTACTTCTACTACGGTATGGACGTC |
| | | | SEQ ID NO:7637 | SEQ ID NO:15649 | SEQ ID NO:23661 |
| | | AA | GYYMH | WINPYSGGTNYAQKFQD | DRAVAGNYFYYGMDV |
| | | | SEQ ID NO:7638 | SEQ ID NO:15650 | SEQ ID NO:23662 |
| iPS:437040 | 21-225_196E7 | NA | GGCTACAATATGCAC | TGGATCAACCCTAATAGTGGTGGCACAAACTATGCACACAAGTTTCAGGGC | GATTACTATGATACTAGTGGAGAAGGGTGGTTCGACCCC |
| | | | SEQ ID NO:7639 | SEQ ID NO:15651 | SEQ ID NO:23663 |
| | | AA | GYNMH | WINPNSGGTNYAHKFQG | DYYDTSGEGWFDP |
| | | | SEQ ID NO:7640 | SEQ ID NO:15652 | SEQ ID NO:23664 |
| iPS:437042 | 21-225_197E8 | NA | GGCTACTATATACAC | TGGATCAACCCTAACAGTGGTGGCACAAACTATGCACAGAGGTTTCAGGGC | GAGATAGCAGTGGCTGGGAACTACTTCTACTACGGTATGGGCGTC |
| | | | SEQ ID NO:7641 | SEQ ID NO:15653 | SEQ ID NO:23665 |
| | | AA | GYYIH | WINPNSGGTNYAQRFQG | EIAVAGNYFYYGMGV |
| | | | SEQ ID NO:7642 | SEQ ID NO:15654 | SEQ ID NO:23666 |
| iPS:437044 | 21-225_197F9 | NA | ATTTACTACTGGAGC | TATGTCTATTACAGTGGGAGCACCACCTACAACCCCTCCCTCAAGAGT | GAAAGGGGGAGTAGCCACAGATGGGGGACTACTACGGAATGGACGTC |
| | | | SEQ ID NO:7643 | SEQ ID NO:15655 | SEQ ID NO:23667 |
| | | AA | IYYWS | YVYYSGSTTYNPSLKS | ERGSSHRWGDYYGMDV |
| | | | SEQ ID NO:7644 | SEQ ID NO:15656 | SEQ ID NO:23668 |
| iPS:437048 | 21 225 197B11 | NA | AGTGGTGGTTACTACTGGAGC | TACATCTATTACACTGGGAGCACCTACTACAACCCGTCCCTCAAGAGT | GACGATCCCCTCTACGGAATGGACGTC |

FIGURE 49

| | | | | | |
|---|---|---|---|---|---|
| | 21-225_197B11 | | SEQ ID NO:7645 | SEQ ID NO:15657 | SEQ ID NO:23669 |
| | | AA | SGGYYWS | YIYYTGSTYYNPSLKS | DDPLYGMDV |
| | | | SEQ ID NO:7646 | SEQ ID NO:15658 | SEQ ID NO:23670 |
| iPS:437050 | 21-225_197C11 | NA | GGCTACAATATGCAC | TGGATCAACCCTAATAGTGGTGGCACAAACTATGCACACAAGTTTCAGGGC | GATTACTATGATAGTAGTGGAGAAGGGTGGTTCGACCCC |
| | | | SEQ ID NO:7647 | SEQ ID NO:15659 | SEQ ID NO:23671 |
| | | AA | GYNMH | WINPNSGGTNYAHKFQG | DYYDSSGEGWFDP |
| | | | SEQ ID NO:7648 | SEQ ID NO:15660 | SEQ ID NO:23672 |
| iPS:437054 | 21-225_194G3 | NA | TTCCATGGCATGCAC | GTTATATGGTATGATGGAAGTAAAAAATACTATGCAGACTCCGTGAAGGGC | GAAGGGTTTAGCAGTGGCTTTTACAGGGGGTTTGACAAC |
| | | | SEQ ID NO:7649 | SEQ ID NO:15661 | SEQ ID NO:23673 |
| | | AA | FHGMH | VIWYDGSKKYYADSVKG | EGFSSGFYRGFDN |
| | | | SEQ ID NO:7650 | SEQ ID NO:15662 | SEQ ID NO:23674 |
| iPS:437056 | 21-225_198B8 | NA | AGTGGTGGTGACTACTGGAGC | TACATCTATTACAGTGGGATCACCTACTACAATCCGTCCCTCAAGAGT | GATAGCAGCTCGCGGGGTATGGACGTC |
| | | | SEQ ID NO:7651 | SEQ ID NO:15663 | SEQ ID NO:23675 |
| | | AA | SGGDYWS | YIYYSGITYYNPSLKS | DSSSRGMDV |
| | | | SEQ ID NO:7652 | SEQ ID NO:15664 | SEQ ID NO:23676 |
| iPS:437058 | 21-225_199F3 | NA | TTCTATGGCATGCAC | GTTATTTGGTATGATGGAAGTAGTAAATACTATGCAGACTCCGTGAAGGGC | GAAGGGTATAGCAGTGGCTTTTACAGGGGATTTGCCAAC |
| | | | SEQ ID NO:7653 | SEQ ID NO:15665 | SEQ ID NO:23677 |
| | | AA | FYGMH | VIWYDGSSKYYADSVKG | EGYSSGFYRGFAN |
| | | | SEQ ID NO:7654 | SEQ ID NO:15666 | SEQ ID NO:23678 |

FIGURE 49

| | | | | | |
|---|---|---|---|---|---|
| **iPS:437060** | **21-225_199C3** | NA | ATTTACTACTGGAGC | TATATCTATTACAGTGGGAGCACCACCTACAACCCCTCCCTCAAGAGT | GAAAGGGGGAGTAGCCACAGATGGGGGACTACTACGGAATGGACGTC |
| | | | SEQ ID NO:7655 | SEQ ID NO:15667 | SEQ ID NO:23679 |
| | | AA | IYYWS | YIYYSGSTTYNPSLKS | ERGSSHRWGDYYGMDV |
| | | | SEQ ID NO:7656 | SEQ ID NO:15668 | SEQ ID NO:23680 |
| **iPS:437062** | **21-225_200H1** | NA | AGTGGTGGTGACTATTGGAGC | TACATCTATTATAGTGGGAGCACCTACTACAACCCGTCCCTCAAGAGT | GATGGAGCAGCTCTGGGTATGGACGTC |
| | | | SEQ ID NO:7657 | SEQ ID NO:15669 | SEQ ID NO:23681 |
| | | AA | SGGDYWS | YIYYSGSTYYNPSLKS | DGAALGMDV |
| | | | SEQ ID NO:7658 | SEQ ID NO:15670 | SEQ ID NO:23682 |
| **iPS:437064** | **21-225_200G8** | NA | AGTTACTACTGGAGC | TATATCTATTACAGTGGGAGTACTAAGTACAACCCCTCCCTCAAGAGT | AAGGGAGTGGGAACCATCCACTTTGACTAC |
| | | | SEQ ID NO:7659 | SEQ ID NO:15671 | SEQ ID NO:23683 |
| | | AA | SYYWS | YIYYSGSTKYNPSLKS | KGVGTIHFDY |
| | | | SEQ ID NO:7660 | SEQ ID NO:15672 | SEQ ID NO:23684 |
| **iPS:437066** | **21-225_200G9** | NA | AGTGGTGGTGACTACTGGAGC | TACATCTATTACAGTGGGAGCACCTACTACAACCCGTCCCTCAAGAGT | GATGGAGCAGCTCTGGGTATGGACGTC |
| | | | SEQ ID NO:7661 | SEQ ID NO:15673 | SEQ ID NO:23685 |
| | | AA | SGGDYWS | YIYYSGSTYYNPSLKS | DGAALGMDV |
| | | | SEQ ID NO:7662 | SEQ ID NO:15674 | SEQ ID NO:23686 |
| **iPS:437068** | **21-225_200A11** | NA | AGTGGTGGTGACTACTGGAGC | TACATCTATTACAGAGGGAGCACCTACTACAACCCGTCCCTCAAGAGT | GATGCAGCAGCCCACGGCATGGACGTC |
| | | | SEQ ID NO:7663 | SEQ ID NO:15675 | SEQ ID NO:23687 |
| | | AA | SGGDYWS | YIYYRGSTYYNPSLKS | DAAAHGMDV |
| | | | SEQ ID NO:7664 | SEQ ID NO:15676 | SEQ ID NO:23688 |

FIGURE 49

| | | | | | |
|---|---|---|---|---|---|
| **iPS:437070** | **21-225_201G11** | NA | CGCATCAATCCTACTTGGAAC | AGGACATACTACAGGTCCAAGTGGTATCATGTTTATGCAGTATCTGTGAAAAGT | GATCCTGGGGGGCTCTTTGACTAC |
| | | | SEQ ID NO:7665 | SEQ ID NO:15677 | SEQ ID NO:23689 |
| | | AA | RINPTWN | RTYYRSKWYHVYAVSVKS | DPGGLFDY |
| | | | SEQ ID NO:7666 | SEQ ID NO:15678 | SEQ ID NO:23690 |
| **iPS:437074** | **21-225_203B2** | NA | AACTATGGCATGCAC | ATTATATGGTTTGATGGAAGTAATGAATACTATGCAGACTCCGTGAAGGGC | GAAAGTGGGAGCTATGCTCTTTATATC |
| | | | SEQ ID NO:7667 | SEQ ID NO:15679 | SEQ ID NO:23691 |
| | | AA | NYGMH | IIWFDGSNEYYADSVKG | ESGSYALYI |
| | | | SEQ ID NO:7668 | SEQ ID NO:15680 | SEQ ID NO:23692 |
| **iPS:437076** | **21-225_203G6** | NA | CGCACCAATCCTACTTGGAAC | AGGACATACTACAGGTCCAAGTGGTATCATGTTTATGCACTATCTGTGAAAAGT | GATCCTGGGGGCCTCTTTGACTAC |
| | | | SEQ ID NO:7669 | SEQ ID NO:15681 | SEQ ID NO:23693 |
| | | AA | RTNPTWN | RTYYRSKWYHVYALSVKS | DPGGLFDY |
| | | | SEQ ID NO:7670 | SEQ ID NO:15682 | SEQ ID NO:23694 |
| **iPS:437082** | **21-225_205E12** | NA | AACTATGGCATGCAC | ATTATATGGTTTGATGGAAGTAATGAATACTATGCAGACTCCGTGAAGGGC | GAAAGTGGGAGCTATGCTCTTTATATC |
| | | | SEQ ID NO:7671 | SEQ ID NO:15683 | SEQ ID NO:23695 |
| | | AA | NYGMH | IIWFDGSNEYYADSVKG | ESGSYALYI |
| | | | SEQ ID NO:7672 | SEQ ID NO:15684 | SEQ ID NO:23696 |

FIGURE 49

| | | | | | |
|---|---|---|---|---|---|
| **iPS:437084** | **21-225_206B5** | NA | AGCTATGGCATGCAC | GTTATATGGTATGATGGAAGTAATAAATACTATGCAGACTCCGTGAAGGGC | GAGGGTGGGAGCTACCACCTTGACTAC |
| | | | SEQ ID NO:7673 | SEQ ID NO:15685 | SEQ ID NO:23697 |
| | | AA | SYGMH | VIWYDGSNKYYADSVKG | EGGSYHLDY |
| | | | SEQ ID NO:7674 | SEQ ID NO:15686 | SEQ ID NO:23698 |
| **iPS:437086** | **21-225_209A8** | NA | AGTTATAGCATGAAC | TACATTAGTAGTAGTAGTAGTATCAAAAAGTACGCAGACTCTGTGAAGGGC | GATGATGGGAGCTACTACTTTGACTAC |
| | | | SEQ ID NO:7675 | SEQ ID NO:15687 | SEQ ID NO:23699 |
| | | AA | SYSMN | YISSSSSIKKYADSVKG | DDGSYYFDY |
| | | | SEQ ID NO:7676 | SEQ ID NO:15688 | SEQ ID NO:23700 |
| **iPS:437088** | **21-225_209H10** | NA | AGCTATGGCATGCAC | GTTATATGGTATGATGGAAGTAATAAATACTATGCAGACTCCGTGAAGGGC | GAGGGTGGGAGCTACCACCTTGACTAC |
| | | | SEQ ID NO:7677 | SEQ ID NO:15689 | SEQ ID NO:23701 |
| | | AA | SYGMH | VIWYDGSNKYYADSVKG | EGGSYHLDY |
| | | | SEQ ID NO:7678 | SEQ ID NO:15690 | SEQ ID NO:23702 |
| **iPS:437090** | **21-225_210F11** | NA | AGTGGTGGTTCCTACTGGAGC | TACATCTATTACATTGGGACCACCTACTACAACCCGTCCCTCAAGAGT | GATGAGCCATTGACCGGTATGGACGTC |
| | | | SEQ ID NO:7679 | SEQ ID NO:15691 | SEQ ID NO:23703 |
| | | AA | SGGSYWS | YIYYIGTTYYNPSLKS | DEPLTGMDV |
| | | | SEQ ID NO:7680 | SEQ ID NO:15692 | SEQ ID NO:23704 |

# FIGURE 49

| | | | | | |
|---|---|---|---|---|---|
| **iPS:437092** | **21-225_210B12** | NA | GACTACTATATGAAC | TGGATCAACCCTAACAGTGGTGGCACAAACTATGCACAGAAGTTTCAGGGC | GGGTATGACTCGTTCGCCCCC |
| | | | SEQ ID NO:7681 | SEQ ID NO:15693 | SEQ ID NO:23705 |
| | | AA | DYYMN | WINPNSGGTNYAQKFQG | GYDSFAP |
| | | | SEQ ID NO:7682 | SEQ ID NO:15694 | SEQ ID NO:23706 |
| **iPS:437094** | **21-225_210D12** | NA | CACTATGGCATGCAC | GTTATATGGTATGATGGAAGTAATAAATACTATGCAGACTCCGTGAAGGGC | GGGGACTGGAACCCCGAGGGTTTGGACGTC |
| | | | SEQ ID NO:7683 | SEQ ID NO:15695 | SEQ ID NO:23707 |
| | | AA | HYGMH | VIWYDGSNKYYADSVKG | GDWNPEGLDV |
| | | | SEQ ID NO:7684 | SEQ ID NO:15696 | SEQ ID NO:23708 |
| **iPS:437096** | **21-225_210E12** | NA | AACTATGGCATGCAC | GTTATATGGTATGATGGAAGTAATAAATACTATGCAGACTCCGTGAAGGGC | GGGGACTGGAACCCCGAGGGTATGGACGTC |
| | | | SEQ ID NO:7685 | SEQ ID NO:15697 | SEQ ID NO:23709 |
| | | AA | NYGMH | VIWYDGSNKYYADSVKG | GDWNPEGMDV |
| | | | SEQ ID NO:7686 | SEQ ID NO:15698 | SEQ ID NO:23710 |
| **iPS:437098** | **21-225_211C1** | NA | CACTATGGCATGCAC | GTTATATGGTATGATGGAAGTAATAAATACTATGCAGACTCCGTGAAGGGC | GGGGACTGGAACCCCGAGGGTTTGGACGTC |
| | | | SEQ ID NO:7687 | SEQ ID NO:15699 | SEQ ID NO:23711 |
| | | AA | HYGMH | VIWYDGSNKYYADSVKG | GDWNPEGLDV |
| | | | SEQ ID NO:7688 | SEQ ID NO:15700 | SEQ ID NO:23712 |

FIGURE 49

| | | | | | |
|---|---|---|---|---|---|
| **iPS:437100** | **21-225_211H2** | NA | AGCTATGCCATGCAC | GTTATATGGTATGATGGAAGTAATAAATACTATGCAGACTCCGTGAAGGGC | GATCCTGGGAGCTACGGGTTCGACCCC |
| | | | SEQ ID NO:7689 | SEQ ID NO:15701 | SEQ ID NO:23713 |
| | | AA | SYAMH | VIWYDGSNKYYADSVKG | DPGSYGFDP |
| | | | SEQ ID NO:7690 | SEQ ID NO:15702 | SEQ ID NO:23714 |
| **iPS:437102** | **21-225_211E5** | NA | AACTATGGCATGCAC | ATTATATGGTTTGATGGAAGTGATCAATACTATGCAGACTCCGTGAAGGGC | GGCCTCTCTGTCTACTACTACGGTATGGGCGTC |
| | | | SEQ ID NO:7691 | SEQ ID NO:15703 | SEQ ID NO:23715 |
| | | AA | NYGMH | IIWFDGSDQYYADSVKG | GLSVYYYGMGV |
| | | | SEQ ID NO:7692 | SEQ ID NO:15704 | SEQ ID NO:23716 |
| **iPS:437104** | **21-225_211G5** | NA | CACTATGGCATGCAC | GTTATATGGTATGATGGAAGTAATAAATACTATGCAGACTCCGTGAAGGGC | GGGGACTGGAACCCCGAGGGTTTGGACGTC |
| | | | SEQ ID NO:7693 | SEQ ID NO:15705 | SEQ ID NO:23717 |
| | | AA | HYGMH | VIWYDGSNKYYADSVKG | GDWNPEGLDV |
| | | | SEQ ID NO:7694 | SEQ ID NO:15706 | SEQ ID NO:23718 |
| **iPS:437106** | **21-225_211H7** | NA | AGTGGTGGTTACTACTGGAGC | TACATCTATTACGTTGGGAGCACCTACTACAACCCGTCCCTCAAGAGT | GATGGGCCATTGAGCGGTATGGACGTC |
| | | | SEQ ID NO:7695 | SEQ ID NO:15707 | SEQ ID NO:23719 |
| | | AA | SGGYYWS | YIYYVGSTYYNPSLKS | DGPLSGMDV |
| | | | SEQ ID NO:7696 | SEQ ID NO:15708 | SEQ ID NO:23720 |

FIGURE 49

| | | | | | |
|---|---|---|---|---|---|
| **iPS:437108** | **21-225_211C9** | NA | AGTGGTGGTGACTACTGGAGC | TACATCTATTATAGTGGGAGCACCTACTACAACCCGTCCCTCAAGAGT | GATTCAGCAGTGTACAATATGGACGTC |
| | | | SEQ ID NO:7697 | SEQ ID NO:15709 | SEQ ID NO:23721 |
| | | AA | SGGDYWS | YIYYSGSTYYNPSLKS | DSAVYNMDV |
| | | | SEQ ID NO:7698 | SEQ ID NO:15710 | SEQ ID NO:23722 |
| **iPS:437110** | **21-225_211E9** | NA | AGTGGTGGTGACTACTGGAGC | TACATCTATTACACTGGGAGCAACTACTACAACCCGTCCCTCAAGAGT | GATTCAGCAGTGTACGGTATGGACGTC |
| | | | SEQ ID NO:7699 | SEQ ID NO:15711 | SEQ ID NO:23723 |
| | | AA | SGGDYWS | YIYYTGSNYYNPSLKS | DSAVYGMDV |
| | | | SEQ ID NO:7700 | SEQ ID NO:15712 | SEQ ID NO:23724 |
| **iPS:437112** | **21-225_212C2** | NA | CACTATGGCATGCAC | GTTATATGGTATGATGGAAGTAATAAATACTATGCAGACTCCGTGAAGGGC | GGGGACTGGAACCCCGAGGGTATGGACGTC |
| | | | SEQ ID NO:7701 | SEQ ID NO:15713 | SEQ ID NO:23725 |
| | | AA | HYGMH | VIWYDGSNKYYADSVKG | GDWNPEGMDV |
| | | | SEQ ID NO:7702 | SEQ ID NO:15714 | SEQ ID NO:23726 |
| **iPS:437114** | **21-225_212A4** | NA | AACTATGGCATGCAC | GTTATATGGTATGATGGAAGTAATAAATACTATGCAGACTCCGTGAAGGGC | GGGGACTGGAACCCCGAGGGTATGGACGTC |
| | | | SEQ ID NO:7703 | SEQ ID NO:15715 | SEQ ID NO:23727 |
| | | AA | NYGMH | VIWYDGSNKYYADSVKG | GDWNPEGMDV |
| | | | SEQ ID NO:7704 | SEQ ID NO:15716 | SEQ ID NO:23728 |

**FIGURE 49**

| | | | | | |
|---|---|---|---|---|---|
| **iPS:437116** | **21-225_212F6** | NA | CACTATGGCATGCAC | GTTATATGGTATGATGG AAGTAATAAATACTATG CGGACTCCGTGAAGGGC | GGGGACTGGAACCCCGAGGG TTTGGACGTC |
| | | | SEQ ID NO:7705 | SEQ ID NO:15717 | SEQ ID NO:23729 |
| | | AA | HYGMH | VIWYDGSNKYYADSVKG | GDWNPEGLDV |
| | | | SEQ ID NO:7706 | SEQ ID NO:15718 | SEQ ID NO:23730 |
| **iPS:437118** | **21-225_212G7** | NA | CACTATGGCATGCAC | GTTATATGGTATGATGG AAGTAATAAATACTGTG CAGACTCCGTGAAGGGC | GGAGACTGGAACCCCGAGGG TTTGGACGTC |
| | | | SEQ ID NO:7707 | SEQ ID NO:15719 | SEQ ID NO:23731 |
| | | AA | HYGMH | VIWYDGSNKYCADSVKG | GDWNPEGLDV |
| | | | SEQ ID NO:7708 | SEQ ID NO:15720 | SEQ ID NO:23732 |
| **iPS:437120** | **21-225_212A9** | NA | AGTGGTGGTGACTACTGGAG C | TATATGTATTACAGTGGG AGCACCTACTACAACCC GTCCCTCAAGAGT | GATTCAGCAGTGTACGGTAT GGACGTC |
| | | | SEQ ID NO:7709 | SEQ ID NO:15721 | SEQ ID NO:23733 |
| | | AA | SGGDYWS | YMYYSGSTYYNPSLKS | DSAVYGMDV |
| | | | SEQ ID NO:7710 | SEQ ID NO:15722 | SEQ ID NO:23734 |
| **iPS:437124** | **21-225_212H12** | NA | AGTGGTGGTGACTACTGGAG T | TACATCTATTACAGTGGG AGCACCTACTACAACCC GTCCCTCAAGAGT | GATAGCAGCTCCTACGGTAT GGACGTC |
| | | | SEQ ID NO:7711 | SEQ ID NO:15723 | SEQ ID NO:23735 |
| | | AA | SGGDYWS | YIYYSGSTYYNPSLKS | DSSSYGMDV |
| | | | SEQ ID NO:7712 | SEQ ID NO:15724 | SEQ ID NO:23736 |

# FIGURE 49

| | | | | | |
|---|---|---|---|---|---|
| **iPS:437128** | **21-225_213G3** | NA | CACTATGGCATGCAC | GTTATATGGTATGATGG AAGTAATAAATACTATG CAGACTCCGTGAAGGGC | GGGGACTGGAACCCCGAGGG TATGGACGTC |
| | | | SEQ ID NO:7713 | SEQ ID NO:15725 | SEQ ID NO:23737 |
| | | AA | HYGMH | VIWYDGSNKYYADSVKG | GDWNPEGMDV |
| | | | SEQ ID NO:7714 | SEQ ID NO:15726 | SEQ ID NO:23738 |
| **iPS:437130** | **21-225_213D5** | NA | CACTATGGCATGCAC | GTTATATGGTATGATGG AAGTAATAAATACTATG CAGACTCCGTGAAGGGC | GGGGACTGGAACCCCGAGGG TATGGACGTC |
| | | | SEQ ID NO:7715 | SEQ ID NO:15727 | SEQ ID NO:23739 |
| | | AA | HYGMH | VIWYDGSNKYYADSVKG | GDWNPEGMDV |
| | | | SEQ ID NO:7716 | SEQ ID NO:15728 | SEQ ID NO:23740 |
| **iPS:437132** | **21-225_213F5** | NA | AGTGGTGGTGACTACTGGAG C | TACATCTATTATAGTGGG AGCACCTACTACAACCC GTCCCTCAAGAGT | GATTCAGCAGTGTACAATAT GGACGTC |
| | | | SEQ ID NO:7717 | SEQ ID NO:15729 | SEQ ID NO:23741 |
| | | AA | SGGDYWS | YIYYSGSTYYNPSLKS | DSAVYNMDV |
| | | | SEQ ID NO:7718 | SEQ ID NO:15730 | SEQ ID NO:23742 |
| **iPS:437134** | **21-225_213A7** | NA | GACTACTATATGAAC | TGGATCAACCCTAAGAA TGGTGGCACAAACTATG CACAGAAGTTTCAGGGC | GGGTATGATTCGTTCGCCCC C |
| | | | SEQ ID NO:7719 | SEQ ID NO:15731 | SEQ ID NO:23743 |
| | | AA | DYYMN | WINPKNGGTNYAQKFQG | GYDSFAP |
| | | | SEQ ID NO:7720 | SEQ ID NO:15732 | SEQ ID NO:23744 |

**FIGURE 49**

| | | | | | |
|---|---|---|---|---|---|
| **iPS:437136** | **21-225_214H3** | NA | AGTGGTGGTGACTACTGGAGT | TACATCTATTACAGTGGGAGCACCTACTACAACCCGTCCCTCAAGAGT | GATAGCAGCTCCTACGGTATGGACGTC |
| | | | SEQ ID NO:7721 | SEQ ID NO:15733 | SEQ ID NO:23745 |
| | | AA | SGGDYWS | YIYYSGSTYYNPSLKS | DSSSYGMDV |
| | | | SEQ ID NO:7722 | SEQ ID NO:15734 | SEQ ID NO:23746 |
| **iPS:437138** | **21-225_214D8** | NA | ACTGCTTTTTACTACTGGAGC | TACATCTATTTCAGTGGGAGCACCTACTACAACCCGTCCCTCAAGAGT | GCAAGGGGATATCACTACAGTATCTTTGACTAC |
| | | | SEQ ID NO:7723 | SEQ ID NO:15735 | SEQ ID NO:23747 |
| | | AA | TAFYYWS | YIYFSGSTYYNPSLKS | ARGYHYSIFDY |
| | | | SEQ ID NO:7724 | SEQ ID NO:15736 | SEQ ID NO:23748 |
| **iPS:437140** | **21-225_214E12** | NA | AGTGGTGGTGATTACTGGAGC | TACATCTATTACAGTGGGCCCACCTACTACAACCCGTCCCTCAAGAGT | GATGGGGCTGCGGAGGGTATGGACGTC |
| | | | SEQ ID NO:7725 | SEQ ID NO:15737 | SEQ ID NO:23749 |
| | | AA | SGGDYWS | YIYYSGPTYYNPSLKS | DGAAEGMDV |
| | | | SEQ ID NO:7726 | SEQ ID NO:15738 | SEQ ID NO:23750 |
| **iPS:437142** | **21-225_215A3** | NA | AGTGGTGGTGACTACTGGAGC | TACATCTATTACACTGGGAGCAACTACTACAACCCGTCCCTCAAGAGT | GATTCAGCAGTGTACGGTATGGACGTC |
| | | | SEQ ID NO:7727 | SEQ ID NO:15739 | SEQ ID NO:23751 |
| | | AA | SGGDYWS | YIYYTGSNYYNPSLKS | DSAVYGMDV |
| | | | SEQ ID NO:7728 | SEQ ID NO:15740 | SEQ ID NO:23752 |
| **iPS:437144** | **21-225_215B3** | NA | AACGCCTGGATGCAC | CGTATTAAAAGCAAAACTAATGGTGGGACAACAGACTACGCTGCACCCGTGAAAGGC | GATCCGGGGGGGATCTTTGACTAC |
| | | | SEQ ID NO:7729 | SEQ ID NO:15741 | SEQ ID NO:23753 |

FIGURE 49

| | | | | | |
|---|---|---|---|---|---|
| | | AA | NAWMH | RIKSKTNGGTTDYAAPVKG | DPGGIFDY |
| | | | SEQ ID NO:7730 | SEQ ID NO:15742 | SEQ ID NO:23754 |
| **iPS:437146** | **21-225_215D3** | NA | CACTATGGCATGCAC | GTTATATGGTATGATGGAAGTAATGAGTACTATGCAGACTCCGTGAAGGGC | GGGGACTGGAACCCCGAGGGTATGGACGTC |
| | | | SEQ ID NO:7731 | SEQ ID NO:15743 | SEQ ID NO:23755 |
| | | AA | HYGMH | VIWYDGSNEYYADSVKG | GDWNPEGMDV |
| | | | SEQ ID NO:7732 | SEQ ID NO:15744 | SEQ ID NO:23756 |
| **iPS:437148** | **21-225_215H3** | NA | AGTGGTGGTGACTACTGGAGC | TATATGTATTACAGTGGGAGCACCTACTACAACCCGTCCCTCAAGAGT | GATTCAGCAGTGTACGGTATGGACGTC |
| | | | SEQ ID NO:7733 | SEQ ID NO:15745 | SEQ ID NO:23757 |
| | | AA | SGGDYWS | YMYYSGSTYYNPSLKS | DSAVYGMDV |
| | | | SEQ ID NO:7734 | SEQ ID NO:15746 | SEQ ID NO:23758 |
| **iPS:437150** | **21-225_216A3** | NA | CACTATGGCATGCAC | GTTATATGGTATGATGGAAGTAATAAATACTATGCAGACTCCGTGAAGGGC | GGGGACTGGAACCCCGAGGGTTTGGACGTC |
| | | | SEQ ID NO:7735 | SEQ ID NO:15747 | SEQ ID NO:23759 |
| | | AA | HYGMH | VIWYDGSNKYYADSVKG | GDWNPEGLDV |
| | | | SEQ ID NO:7736 | SEQ ID NO:15748 | SEQ ID NO:23760 |
| **iPS:437154** | **21-225_216A7** | NA | AGTGGTGGTGACTACTGGAGC | TATATGTATTACAGTGGGAGCACCTACTACAACCCGTCCCTCAAGAGT | GATTCAGCAGTGTACGGTATGGACGTC |
| | | | SEQ ID NO:7737 | SEQ ID NO:15749 | SEQ ID NO:23761 |
| | | AA | SGGDYWS | YMYYSGSTYYNPSLKS | DSAVYGMDV |
| | | | SEQ ID NO:7738 | SEQ ID NO:15750 | SEQ ID NO:23762 |

FIGURE 49

| | | | | | |
|---|---|---|---|---|---|
| **iPS:437158** | **21-225_216H11** | NA | AGTGGTGGTGATTACTGGAGC | TACATCTATTACAGTGGGCCCACCTACTACAACCCGTCCCTCAAGAGT | GATGGGGCTGCGGAGGGTTTGGACGTC |
| | | | SEQ ID NO:7739 | SEQ ID NO:15751 | SEQ ID NO:23763 |
| | | AA | SGGDYWS | YIYYSGPTYYNPSLKS | DGAAEGLDV |
| | | | SEQ ID NO:7740 | SEQ ID NO:15752 | SEQ ID NO:23764 |
| **iPS:437160** | **21-225_216B12** | NA | AGCTATGCCATGCAC | GTTATATGGTATGATGGAAGTAATAAATACTATGCAGACTCCGTGAAGGGC | GATCTGGGACTGGGATACTTCTTTGACTAC |
| | | | SEQ ID NO:7741 | SEQ ID NO:15753 | SEQ ID NO:23765 |
| | | AA | SYAMH | VIWYDGSNKYYADSVKG | DLGLGYFFDY |
| | | | SEQ ID NO:7742 | SEQ ID NO:15754 | SEQ ID NO:23766 |
| **iPS:437162** | **21-225_217B2** | NA | AACTATGGCATGCAC | GTTATATGGTATGATGGAAGTAATAAATACTATGCAGACTCCGTGAAGGGC | GGGGACTGGAACCCCGAGGGTATGGACGTC |
| | | | SEQ ID NO:7743 | SEQ ID NO:15755 | SEQ ID NO:23767 |
| | | AA | NYGMH | VIWYDGSNKYYADSVKG | GDWNPEGMDV |
| | | | SEQ ID NO:7744 | SEQ ID NO:15756 | SEQ ID NO:23768 |
| **iPS:437164** | **21-225_217C6** | NA | AACTATGGCATGCAC | ATTATATGGTTTGATGGAAGTGATGAATACTATGCAGACTCCGTGAAGGGC | GGCCTATCTGTCTACTACTACGGTATGGACGTC |
| | | | SEQ ID NO:7745 | SEQ ID NO:15757 | SEQ ID NO:23769 |
| | | AA | NYGMH | IIWFDGSDEYYADSVKG | GLSVYYYGMDV |
| | | | SEQ ID NO:7746 | SEQ ID NO:15758 | SEQ ID NO:23770 |

FIGURE 49

| | | | | | |
|---|---|---|---|---|---|
| **iPS:437166** | **21-225_217G11** | NA | AACTATGGCATGCAC | ATTATATGGTTTGATGGAAGTGATCAGTACTATGCAGACTCCGTGAAGGGC | GGCCTCTCTGTCTACTACTACGGTATGGACGTC |
| | | | SEQ ID NO:7747 | SEQ ID NO:15759 | SEQ ID NO:23771 |
| | | AA | NYGMH | IIWFDGSDQYYADSVKG | GLSVYYYGMDV |
| | | | SEQ ID NO:7748 | SEQ ID NO:15760 | SEQ ID NO:23772 |
| **iPS:437168** | **21-225_218G4** | NA | AGCTATGGCTTGCAC | GTTATATGGTATGATGGAAGTAATAAATACTATGCAGACTCCGTGAAGGGC | TGGTACTACTATTACTACGGTATGGACGTC |
| | | | SEQ ID NO:7749 | SEQ ID NO:15761 | SEQ ID NO:23773 |
| | | AA | SYGLH | VIWYDGSNKYYADSVKG | WYYYYYGMDV |
| | | | SEQ ID NO:7750 | SEQ ID NO:15762 | SEQ ID NO:23774 |
| **iPS:437170** | **21-225_218E5** | NA | AACTATGGCATGCAC | ATTATATGGTTTGATGGAAGTGATGAATACTATGCAGACTCCGTGAAGGGC | GGCCTATCTGTCTACTACTACGGTATGGACGTC |
| | | | SEQ ID NO:7751 | SEQ ID NO:15763 | SEQ ID NO:23775 |
| | | AA | NYGMH | IIWFDGSDEYYADSVKG | GLSVYYYGMDV |
| | | | SEQ ID NO:7752 | SEQ ID NO:15764 | SEQ ID NO:23776 |
| **iPS:437172** | **21-225_219A7** | NA | CACTATGGCATGCAC | GTCATATGGTATGATGGAAGTAATAAATACTATGCAGACTCCGTGAAGGGC | GGGGACTGGAACCCCGAGGGTATGGACGTC |
| | | | SEQ ID NO:7753 | SEQ ID NO:15765 | SEQ ID NO:23777 |
| | | AA | HYGMH | VIWYDGSNKYYADSVKG | GDWNPEGMDV |
| | | | SEQ ID NO:7754 | SEQ ID NO:15766 | SEQ ID NO:23778 |

FIGURE 49

| | | | | | |
|---|---|---|---|---|---|
| **iPS:437182** | **21-225_221H2** | NA | CACTATGGCATGCAC | GTTATATGGTATGATGGAAGTAATAAATACTATGCAGACTCCGTGAAGGGC | GGGGACTGGAACCCCGAGGGTATGGACGTC |
| | | | SEQ ID NO:7755 | SEQ ID NO:15767 | SEQ ID NO:23779 |
| | | AA | HYGMH | VIWYDGSNKYYADSVKG | GDWNPEGMDV |
| | | | SEQ ID NO:7756 | SEQ ID NO:15768 | SEQ ID NO:23780 |
| **iPS:437184** | **21-225_221G4** | NA | CACTATGGCATGCAC | GTTATATGGTATGATGGAAGTAATAAATACTATGCAGACTCCGTGAAGGGC | GGGGACTGGAACCCCGAGGGTATGGACGTC |
| | | | SEQ ID NO:7757 | SEQ ID NO:15769 | SEQ ID NO:23781 |
| | | AA | HYGMH | VIWYDGSNKYYADSVKG | GDWNPEGMDV |
| | | | SEQ ID NO:7758 | SEQ ID NO:15770 | SEQ ID NO:23782 |
| **iPS:437186** | **21-225_224H2** | NA | AGCAACAGTGCTGCTTGGAAC | AGGACATACTACAGGTCCAAGTGGTATAATGATTATGCAGTATCTGTGAAAAGT | GAGGGGGGCCTAGGATATTGTAGTAGTACCAGCTGCTATGGAGGCTGGTTCGACCCC |
| | | | SEQ ID NO:7759 | SEQ ID NO:15771 | SEQ ID NO:23783 |
| | | AA | SNSAAWN | RTYYRSKWYNDYAVSVKS | EGGLGYCSSTSCYGGWFDP |
| | | | SEQ ID NO:7760 | SEQ ID NO:15772 | SEQ ID NO:23784 |
| **iPS:437188** | **21-225_224B11** | NA | GGCTACTATATACAC | TGGATCAACCCTAAAAATGGTGGCACAAACTATGCACAGAAGTTTCAGGGC | GGAGCGTTTGATTACTTCTACTACTACGCTATGGACGTC |
| | | | SEQ ID NO:7761 | SEQ ID NO:15773 | SEQ ID NO:23785 |

**FIGURE 49**

| | | | | | |
|---|---|---|---|---|---|
| | | AA | GYYIH | WINPKNGGTNYAQKFQG | GAFDYFYYYAMDV |
| | | | SEQ ID NO:7762 | SEQ ID NO:15774 | SEQ ID NO:23786 |
| **iPS:437190** | **21-225_225A9** | NA | ACCTATGGCATGCAC | GTTATATGGTATGATGGAAGTAATAAATACTATGCAGACTCCGTGAAGGGC | GATAACCACTATTGTAGTAGTACCAGCTGCTCCCCATACTACTACTACTTCGGTATGGACGTC |
| | | | SEQ ID NO:7763 | SEQ ID NO:15775 | SEQ ID NO:23787 |
| | | AA | TYGMH | VIWYDGSNKYYADSVKG | DNHYCSSTSCSPYYYYFGMDV |
| | | | SEQ ID NO:7764 | SEQ ID NO:15776 | SEQ ID NO:23788 |
| **iPS:437192** | **21-225_225E9** | NA | AGCTATGGCATGCAC | GTTATGTGGTATGATGGAGGTAATAAAGACTATGCAGACTCCGTGAAGGGC | GATCGGGAATATTGTACTAGTACCAGCTGCCCTTACTACTACTACTACGGTATGGACGTC |
| | | | SEQ ID NO:7765 | SEQ ID NO:15777 | SEQ ID NO:23789 |
| | | AA | SYGMH | VMWYDGGNKDYADSVKG | DREYCTSTSCPYYYYYGMDV |
| | | | SEQ ID NO:7766 | SEQ ID NO:15778 | SEQ ID NO:23790 |
| **iPS:437194** | **21-225_226B2** | NA | GGCTACTTTATGCAC | TGGATCAACCCTAACAGTGGTGACACAAACTATGCACAGAAGTTTCAGGGC | GGGACTTACTATGGTTCGGGGAGTTATTTTAACGAACTTGACTCC |
| | | | SEQ ID NO:7767 | SEQ ID NO:15779 | SEQ ID NO:23791 |
| | | AA | GYFMH | WINPNSGDTNYAQKFQG | GTYYGSGSYFNELDS |
| | | | SEQ ID NO:7768 | SEQ ID NO:15780 | SEQ ID NO:23792 |
| **iPS:437196** | **21-225_226B7** | NA | GGCTACTATATGCAC | TGGATCAACCCTAACAGTGGAGGCACAAACTATGCACAGAAGTTTCAGGAC | GGATATTACTATGGTTCGGGGAGTTATTATAACTGGTTCGACTCC |
| | | | SEQ ID NO:7769 | SEQ ID NO:15781 | SEQ ID NO:23793 |
| | | AA | GYYMH | WINPNSGGTNYAQKFQD | GYYYGSGSYYNWFDS |

FIGURE 49

| | | | | | |
|---|---|---|---|---|---|
| | | | SEQ ID NO:7770 | SEQ ID NO:15782 | SEQ ID NO:23794 |
| **iPS:437198** | **21-225_226F8** | NA | GGCTACTATATACAC | TGGATCAACCCTAACAGTGGTGGCACAAACTATGCACGGAAGTTTCAGGGC | GGAGCGTTTGATTACTACTACTACTACGCTTTGGACGTC |
| | | | SEQ ID NO:7771 | SEQ ID NO:15783 | SEQ ID NO:23795 |
| | | AA | GYYIH | WINPNSGGTNYARKFQG | GAFDYYYYYALDV |
| | | | SEQ ID NO:7772 | SEQ ID NO:15784 | SEQ ID NO:23796 |
| **iPS:437200** | **21-225_226A10** | NA | GGCTACTTTATGCAC | TGGATCAACCCTAACAGTGGTGACACAAACTATGCACAGAAGTTTCAGGGC | GGGACTTACTATGGTTCGGGGAGTTATTTTAACGAACTTGACTCC |
| | | | SEQ ID NO:7773 | SEQ ID NO:15785 | SEQ ID NO:23797 |
| | | AA | GYFMH | WINPNSGDTNYAQKFQG | GTYYGSGSYFNELDS |
| | | | SEQ ID NO:7774 | SEQ ID NO:15786 | SEQ ID NO:23798 |
| **iPS:437202** | **21-225_227D3** | NA | GGCTACTATATGCAC | TGGATCAACCCTAAGAGTGGTGGCACAAACTTTGCACAGAAGTTTCAGGGC | GGAGCGTTTGATTACTTCTACTACTACGGTATGGACGTC |
| | | | SEQ ID NO:7775 | SEQ ID NO:15787 | SEQ ID NO:23799 |
| | | AA | GYYMH | WINPKSGGTNFAQKFQG | GAFDYFYYYGMDV |
| | | | SEQ ID NO:7776 | SEQ ID NO:15788 | SEQ ID NO:23800 |
| **iPS:437204** | **21-225_227E5** | NA | AGCTATGCCATGAGC | GCTATTAGTGGTAGTGGTGGTAGCACATACTACGCAGACTCCGTGAAGGGC | GAATATTGTGGTGGTGACTGCTATTCCCCTTACTACTACTACTACGGTATGGACGTC |
| | | | SEQ ID NO:7777 | SEQ ID NO:15789 | SEQ ID NO:23801 |
| | | AA | SYAMS | AISGSGGSTYYADSVKG | EYCGGDCYSPYYYYYGMDV |
| | | | SEQ ID NO:7778 | SEQ ID NO:15790 | SEQ ID NO:23802 |
| **iPS:437208** | **21-225_227C10** | NA | GGCTACTATATGCAC | TGGATCAACCCTAAGAGTGGTGGCACAAACTATGCACAGAAGTTTCAGGGC | GGAGCGTTTGATTACTTCTACTACTACGGTATGGACGTC |
| | | | SEQ ID NO:7779 | SEQ ID NO:15791 | SEQ ID NO:23803 |

**FIGURE 49**

| | | | | | |
|---|---|---|---|---|---|
| | | AA | GYYMH | WINPKSGGTNYAQKFQG | GAFDYFYYYGMDV |
| | | | SEQ ID NO:7780 | SEQ ID NO:15792 | SEQ ID NO:23804 |
| **iPS:437210** | **21-225_227E12** | NA | ACTAGTGGAGTGGGTGTGGGC | CTCATTTATTGGAATGATGATAAGGTCTACAGCCCATCTCTGAAGAGC | AGGGGACAGCAGCTGGCCCTCGACTAC |
| | | | SEQ ID NO:7781 | SEQ ID NO:15793 | SEQ ID NO:23805 |
| | | AA | TSGVGVG | LIYWNDDKVYSPSLKS | RGQQLALDY |
| | | | SEQ ID NO:7782 | SEQ ID NO:15794 | SEQ ID NO:23806 |
| **iPS:437214** | **21-225_48B12** | NA | AGCTATGCCATGAAC | GCTATTAGTGGTCGTGGTGGTAACACATTCTACGCAGACTCCGTGAAGGGC | AGAGAGACGTATAACTGGAACTACGAAGGGTTTGACTAC |
| | | | SEQ ID NO:7783 | SEQ ID NO:15795 | SEQ ID NO:23807 |
| | | AA | SYAMN | AISGRGGNTFYADSVKG | RETYNWNYEGFDY |
| | | | SEQ ID NO:7784 | SEQ ID NO:15796 | SEQ ID NO:23808 |
| **iPS:437216** | **21-225_51D5** | NA | AGCTATGTCATGAGC | ACTATGAGTGGTAGTGGTGGTCGCACATACTACGCAGACTCCGTGAACGGC | GTGACTGCTTTTGACTAC |
| | | | SEQ ID NO:7785 | SEQ ID NO:15797 | SEQ ID NO:23809 |
| | | AA | SYVMS | TMSGSGGRTYYADSVNG | VTAFDY |
| | | | SEQ ID NO:7786 | SEQ ID NO:15798 | SEQ ID NO:23810 |
| **iPS:437220** | **21-225_55H6** | NA | AGCTATAGCATGAAC | TCCATTAGTGGTAGTAGTACTTACATATACTACGCAGACTCAGTGAAGGGC | ACTGGGGTCTTTGACTAC |
| | | | SEQ ID NO:7787 | SEQ ID NO:15799 | SEQ ID NO:23811 |
| | | AA | SYSMN | SISGSSTYIYYADSVKG | TGVFDY |
| | | | SEQ ID NO:7788 | SEQ ID NO:15800 | SEQ ID NO:23812 |
| **iPS:437224** | **21-225_56H1** | NA | AACTATAGAATGAAC | TCCATTAGTGGTAGTAGTACTGACATATACTACGCAGACTCAGTGAAGGGC | GTGGCCTCCTTTGACTAC |
| | | | SEQ ID NO:7789 | SEQ ID NO:15801 | SEQ ID NO:23813 |

FIGURE 49

| | | | | | |
|---|---|---|---|---|---|
| | | AA | NYRMN | SISGSSTDIYYADSVKG | VASFDY |
| | | | SEQ ID NO:7790 | SEQ ID NO:15802 | SEQ ID NO:23814 |
| **iPS:437226** | **21-225_57C2** | NA | AGCTTTGGCATGAAC | TCTATTAGTAGTAGTACTGGTTACATATACAACGCAGACTCAGTGAAGGGC | ACCTATAGTGGGAGCCTGGACGTC |
| | | | SEQ ID NO:7791 | SEQ ID NO:15803 | SEQ ID NO:23815 |
| | | AA | SFGMN | SISSSTGYIYNADSVKG | TYSGSLDV |
| | | | SEQ ID NO:7792 | SEQ ID NO:15804 | SEQ ID NO:23816 |
| **iPS:437228** | **21-225_60C11** | NA | AGCTATGCCATGAGC | GCTATTAGTGGTAGTGGTGGTAACACATTCTACGCAGACTCCGTGAAGGGC | TTTTTCGGTGTAGTGGGAGTCGGGTGCTTTGACTAC |
| | | | SEQ ID NO:7793 | SEQ ID NO:15805 | SEQ ID NO:23817 |
| | | AA | SYAMS | AISGSGGNTFYADSVKG | FFGVVGVGCFDY |
| | | | SEQ ID NO:7794 | SEQ ID NO:15806 | SEQ ID NO:23818 |
| **iPS:437230** | **21-225_62H10** | NA | AGCTATAGCATGAAC | TCCATTAGTAGTAGTAGTAGTTACATATACTACGCAGACTCAGTGAAGGGC | GGGGGTTCGAGGGGGTTCGACCCC |
| | | | SEQ ID NO:7795 | SEQ ID NO:15807 | SEQ ID NO:23819 |
| | | AA | SYSMN | SISSSSSYIYYADSVKG | GGSRGFDP |
| | | | SEQ ID NO:7796 | SEQ ID NO:15808 | SEQ ID NO:23820 |
| **iPS:437232** | **21-225_63E1** | NA | ACTTCTGCCATGAGC | GCTATTAGTGGTAGTGGTGCTAACACATTCTACGCAGACTCCGTGAAGGGC | GTTATAGCAGTGGCTGGAGGGCACTTTTTCGACCCC |
| | | | SEQ ID NO:7797 | SEQ ID NO:15809 | SEQ ID NO:23821 |
| | | AA | TSAMS | AISGSGANTFYADSVKG | VIAVAGGHFFDP |
| | | | SEQ ID NO:7798 | SEQ ID NO:15810 | SEQ ID NO:23822 |
| **iPS:437234** | **21 225 64E2** | NA | GGCTACTATATGCAC | TGGATCAACCCTAACAATAATGGCACAAACTATGCACAGAAGTTTCAGGGC | GATGGGAGCAGTGGCTTTGACTAC |

FIGURE 49

| | | | | | |
|---|---|---|---|---|---|
| | 21-225_64E2 | | SEQ ID NO:7799 | SEQ ID NO:15811 | SEQ ID NO:23823 |
| | | AA | GYYMH | WINPNNNGTNYAQKFQG | DGSSGFDY |
| | | | SEQ ID NO:7800 | SEQ ID NO:15812 | SEQ ID NO:23824 |
| iPS:437248 | 21-225_97H3 | NA | GATTACTACTGGAGC | GAAATCAATCATAGTGG AGACACCAACTACAACC CGTCCCTCAAGAGT | GAGTTTCCATATAGTGGAAG CTACCTCTACTACTACGGTA TGGACGTC |
| | | | SEQ ID NO:7801 | SEQ ID NO:15813 | SEQ ID NO:23825 |
| | | AA | DYYWS | EINHSGDTNYNPSLKS | EFPYSGSYLYYYGMDV |
| | | | SEQ ID NO:7802 | SEQ ID NO:15814 | SEQ ID NO:23826 |
| iPS:437250 | 21-225_148C6 | NA | AGCTATGCCATGAGC | GTTATTAGTGGTGGTGGT AGTAGTACATACTACGC AGACTCCGTGAAGGGC | TGGCGAGGTAACCCCACTGA CTACGGTATGGACGTC |
| | | | SEQ ID NO:7803 | SEQ ID NO:15815 | SEQ ID NO:23827 |
| | | AA | SYAMS | VISGGGSSTYYADSVKG | WRGNPTDYGMDV |
| | | | SEQ ID NO:7804 | SEQ ID NO:15816 | SEQ ID NO:23828 |
| iPS:437252 | 21-225_148H11 | NA | AGCTATGCCATGAGC | GTTATTAGTGGTGGTGGT AGTAGCACATACTACGC AGACTCCGTGAAGGGC | TGGCGAGGTAACCCCACTGA CTACGGTATGGACGTC |
| | | | SEQ ID NO:7805 | SEQ ID NO:15817 | SEQ ID NO:23829 |
| | | AA | SYAMS | VISGGGSSTYYADSVKG | WRGNPTDYGMDV |
| | | | SEQ ID NO:7806 | SEQ ID NO:15818 | SEQ ID NO:23830 |
| iPS:437254 | 21-225_149F2 | NA | CGCTATGGCATGCAC | TTTATATGGTATGATGGA AGTGAGAACTACTATGC AGACTCCGTGAAGGGC | GATCGGGTGGAGGGTTCGGG GACTCCCTACTACTACTACG GTATGGACGTC |
| | | | SEQ ID NO:7807 | SEQ ID NO:15819 | SEQ ID NO:23831 |
| | | AA | RYGMH | FIWYDGSENYYADSVKG | DRVEGSGTPYYYYGMDV |
| | | | SEQ ID NO:7808 | SEQ ID NO:15820 | SEQ ID NO:23832 |

FIGURE 49

| | | | | | |
|---|---|---|---|---|---|
| **iPS:437256** | **21-225_150F11** | NA | CGCTATGGCATGCAC | TTTATATGGTATGATGGAAGTGAGAACTACTATGCAGACTCCGTGAAGGGC | GATCGGGTGGAGGGTTCGGGGACTCCCTACTACTACTACGGTATGGACGTC |
| | | | SEQ ID NO:7809 | SEQ ID NO:15821 | SEQ ID NO:23833 |
| | | AA | RYGMH | FIWYDGSENYYADSVKG | DRVEGSGTPYYYYGMDV |
| | | | SEQ ID NO:7810 | SEQ ID NO:15822 | SEQ ID NO:23834 |
| **iPS:437258** | **21-225_153F9** | NA | ACCTATGGCATGCAC | GTTATATGGTATGGTGGAAGTGATACAGACTATGCAGACTCCGTGAGGGGC | GATCGGGATTATTGTAGTGGTGGTAACTGCCCTTACTACTACTACTACGGTATGGACGTC |
| | | | SEQ ID NO:7811 | SEQ ID NO:15823 | SEQ ID NO:23835 |
| | | AA | TYGMH | VIWYGGSDTDYADSVRG | DRDYCSGGNCPYYYYYGMDV |
| | | | SEQ ID NO:7812 | SEQ ID NO:15824 | SEQ ID NO:23836 |
| **iPS:437260** | **21-225_170D1** | NA | GGCTACTTTATGCAC | TGGATCAAGCCTAAAAGCGGTGGCACAAACTGTGCACAGAAGTTTCAGGGC | GGGGGGGCTACGGTGACTACGTGGGGGGTCTTTGACTAC |
| | | | SEQ ID NO:7813 | SEQ ID NO:15825 | SEQ ID NO:23837 |
| | | AA | GYFMH | WIKPKSGGTNCAQKFQG | GGATVTTWGVFDY |
| | | | SEQ ID NO:7814 | SEQ ID NO:15826 | SEQ ID NO:23838 |
| **iPS:437262** | **21-225_170E4** | NA | AGCTATAGCATGAAC | TACATTAGCAGTAGTGGTAGTACCAAATACTACGCAGACTCTGTGGAGGGC | GATAGTAGGAAGGGGTTCTACTACGGTCTGGACGTC |
| | | | SEQ ID NO:7815 | SEQ ID NO:15827 | SEQ ID NO:23839 |
| | | AA | SYSMN | YISSSGSTKYYADSVEG | DSRKGFYYGLDV |
| | | | SEQ ID NO:7816 | SEQ ID NO:15828 | SEQ ID NO:23840 |
| **iPS:437264** | **21 225 171H12** | NA | GGCTACTTTATGCAC | TGGATCAAGCCTAAGAGTGGTGGCACAAACTCTGCACAGAGGTTTCAGGGC | GGGGGGACTACGGTGGCTACGTGGGGGGTCTTTGACTAC |

**FIGURE 49**

| | | | | | |
|---|---|---|---|---|---|
| | **21-225_171H12** | | SEQ ID NO:7817 | SEQ ID NO:15829 | SEQ ID NO:23841 |
| | | AA | GYFMH | WIKPKSGGTNSAQRFQG | GGTTVATWGVFDY |
| | | | SEQ ID NO:7818 | SEQ ID NO:15830 | SEQ ID NO:23842 |
| **iPS:437266** | **21-225_177A5** | NA | GGCTACTTTATGCAC | TGGATCAAGCCTAAGAG TGGTGGCACAAACTCTG CACAGAGGTTTCAGGGC | GGGGGGACTACGGTGGCTAC GTGGGGGGTCTTTGACTAC |
| | | | SEQ ID NO:7819 | SEQ ID NO:15831 | SEQ ID NO:23843 |
| | | AA | GYFMH | WIKPKSGGTNSAQRFQG | GGTTVATWGVFDY |
| | | | SEQ ID NO:7820 | SEQ ID NO:15832 | SEQ ID NO:23844 |
| **iPS:437268** | **21-225_177D2** | NA | AGCTATGGCATGGAC | ATTATATGGTTTGATGGA AGTAATAAATACTATGC AGACTCCGTGAAGGGC | GCATATTGTGGTGGTGACTG CTATTTCCCCCATCTCCATTA CTACGGTATGGACGTC |
| | | | SEQ ID NO:7821 | SEQ ID NO:15833 | SEQ ID NO:23845 |
| | | AA | SYGMD | IIWFDGSNKYYADSVKG | AYCGGDCYFPHLHYYGMDV |
| | | | SEQ ID NO:7822 | SEQ ID NO:15834 | SEQ ID NO:23846 |
| **iPS:437270** | **21-225_178H4** | NA | GGCTACTTTATGCAC | TGGATCAAGCCTAAAAG TGGTGGCACAAACTGTG CACAGAAGTTTCAGGGC | GGGGGGACTACGGTGACTAC GTGGGGGGTCTTTGACTAC |
| | | | SEQ ID NO:7823 | SEQ ID NO:15835 | SEQ ID NO:23847 |
| | | AA | GYFMH | WIKPKSGGTNCAQKFQG | GGTTVTTWGVFDY |
| | | | SEQ ID NO:7824 | SEQ ID NO:15836 | SEQ ID NO:23848 |
| **iPS:437274** | **21-225_196D4** | NA | AGCTATGGCATGCAC | GTTATATGGTATGATGG AAGTAATAGAAACTATG CAGACTCCGTGAAGGGC | GATCGGTCTAAGGGTTACGA CGGTATGGACGTC |
| | | | SEQ ID NO:7825 | SEQ ID NO:15837 | SEQ ID NO:23849 |

**FIGURE 49**

| | | | | | |
|---|---|---|---|---|---|
| | | AA | SYGMH | VIWYDGSNRNYADSVKG | DRSKGYDGMDV |
| | | | SEQ ID NO:7826 | SEQ ID NO:15838 | SEQ ID NO:23850 |
| **iPS:437280** | **21-225_203C10** | NA | GACTATGGCATGCAC | GTTATATGGTATGATGG AGGTAATACACATTATA CAGACTCCGTGAAGGGC | GAAGTGGGTTGGCTTGATGA CTAC |
| | | | SEQ ID NO:7827 | SEQ ID NO:15839 | SEQ ID NO:23851 |
| | | AA | DYGMH | VIWYDGGNTHYTDSVKG | EVGWLDDY |
| | | | SEQ ID NO:7828 | SEQ ID NO:15840 | SEQ ID NO:23852 |
| **iPS:437282** | **21-225_207C9** | NA | AGCTATGCCATGAGC | GCTATTAGTGGTAGTGGT GGTAGCACATACTACGC AGACTCCGTGAAGGGC | GCTGGTGGAACTACGGGGAG CTACTACTACAACGGTATGG ACGTC |
| | | | SEQ ID NO:7829 | SEQ ID NO:15841 | SEQ ID NO:23853 |
| | | AA | SYAMS | AISGSGGSTYYADSVKG | AGGTTGSYYYNGMDV |
| | | | SEQ ID NO:7830 | SEQ ID NO:15842 | SEQ ID NO:23854 |
| **iPS:437286** | **21-225_208F1** | NA | GACTATGTCATGCAC | GTTATATGGTATGATGG AAGTAATAAATACTATG TAGACTCCGTGAAGGGC | GAACGGTATAGCAGTGGCTT GTACGACTACGGTATGGACG TC |
| | | | SEQ ID NO:7831 | SEQ ID NO:15843 | SEQ ID NO:23855 |
| | | AA | DYVMH | VIWYDGSNKYYVDSVKG | ERYSSGLYDYGMDV |
| | | | SEQ ID NO:7832 | SEQ ID NO:15844 | SEQ ID NO:23856 |
| **iPS:437290** | **21-225_210G6** | NA | GACTATGTCATGCAC | GTTATATGGTATGATGG AAGTAATAAATACTATG TAGACTCCGTGAAGGGC | GAACGGTATAGCAGTGGCTT GTACGACTACGGTATGGACG TC |
| | | | SEQ ID NO:7833 | SEQ ID NO:15845 | SEQ ID NO:23857 |

FIGURE 49

| | | | | | |
|---|---|---|---|---|---|
| | | AA | DYVMH | VIWYDGSNKYYVDSVKG | ERYSSGLYDYGMDV |
| | | | SEQ ID NO:7834 | SEQ ID NO:15846 | SEQ ID NO:23858 |
| **iPS:437294** | **21-225_216D5** | NA | AGTGGTGGTTACTACTGGAGC | TACATCTATTACAGTGGGAGCACCTACTACAACCCGTCCCTCAAGAGT | GATTCCCCTGACAGGGGGTTTGACTAC |
| | | | SEQ ID NO:7835 | SEQ ID NO:15847 | SEQ ID NO:23859 |
| | | AA | SGGYYWS | YIYYSGSTYYNPSLKS | DSPDRGFDY |
| | | | SEQ ID NO:7836 | SEQ ID NO:15848 | SEQ ID NO:23860 |
| **iPS:437302** | **21-225_225B11** | NA | AGCTATGGCATGCAC | ATTATATCATATAGTGGAAGTAATAAATACTATGCAGACTCCGTGAAGGGC | CGGGGATACAGCTATGGCGGGTACGGTATGGACGTC |
| | | | SEQ ID NO:7837 | SEQ ID NO:15849 | SEQ ID NO:23861 |
| | | AA | SYGMH | IISYSGSNKYYADSVKG | RGYSYGGYGMDV |
| | | | SEQ ID NO:7838 | SEQ ID NO:15850 | SEQ ID NO:23862 |
| **iPS:437320** | **21-225_75A1** | NA | GATTACTACTGGAGC | GAAATCAATCATAGTGGAGACACCAACTACAACCCGTCCCTCAAGAGT | GAGTTTCCATATAGTGGAAGCTACCTCTACTACTACGGTATGGACGTC |
| | | | SEQ ID NO:7839 | SEQ ID NO:15851 | SEQ ID NO:23863 |
| | | AA | DYYWS | EINHSGDTNYNPSLKS | EFPYSGSYLYYYGMDV |
| | | | SEQ ID NO:7840 | SEQ ID NO:15852 | SEQ ID NO:23864 |
| **iPS:437322** | **21-225_75B1** | NA | AATTATGATATCAAC | TGGATGCACCCTAACAGTGGTAACACAGGCTATGCACAGAAGTTCCAGGGC | AGTAGTGGCTGGTACTGGTTCGACCCC |
| | | | SEQ ID NO:7841 | SEQ ID NO:15853 | SEQ ID NO:23865 |
| | | AA | NYDIN | WMHPNSGNTGYAQKFQG | SSGWYWFDP |
| | | | SEQ ID NO:7842 | SEQ ID NO:15854 | SEQ ID NO:23866 |

FIGURE 49

| | | | | | |
|---|---|---|---|---|---|
| **iPS:437324** | **21-225_75C2** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:7843 | SEQ ID NO:15855 | SEQ ID NO:23867 |
| | | AA | GCYWS | EINHSGSTNYNPSLKS | DYGGMDV |
| | | | SEQ ID NO:7844 | SEQ ID NO:15856 | SEQ ID NO:23868 |
| **iPS:437326** | **21-225_75C10** | NA | AGTTATGGCATGCAT | GTTATATGGTATGATGGAAGTGATAAATACTATGCAGACTCCGTGAAGGGC | GATCGGTTAGTGGGAGCTACGGTTGATGCTTTTGATATC |
| | | | SEQ ID NO:7845 | SEQ ID NO:15857 | SEQ ID NO:23869 |
| | | AA | SYGMH | VIWYDGSDKYYADSVKG | DRLVGATVDAFDI |
| | | | SEQ ID NO:7846 | SEQ ID NO:15858 | SEQ ID NO:23870 |
| **iPS:437328** | **21-225_75D3** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:7847 | SEQ ID NO:15859 | SEQ ID NO:23871 |
| | | AA | GCYWS | EINHSGSTNYNPSLKS | DYGGMDV |
| | | | SEQ ID NO:7848 | SEQ ID NO:15860 | SEQ ID NO:23872 |
| **iPS:437332** | **21-225_75F3** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:7849 | SEQ ID NO:15861 | SEQ ID NO:23873 |
| | | AA | GCYWS | EINHSGSTNYNPSLKS | DYGGMDV |
| | | | SEQ ID NO:7850 | SEQ ID NO:15862 | SEQ ID NO:23874 |
| **iPS:437334** | **21-225_75F11** | NA | ACTGGTGGAGTGGGTGTGGGC | CTCATTTATTGGGATGATGATAAGCGCTACAGCCCATCTCTGAAGAGC | CTTATAGCAGTGGCCTTTGACTAC |
| | | | SEQ ID NO:7851 | SEQ ID NO:15863 | SEQ ID NO:23875 |
| | | AA | TGGVGVG | LIYWDDDKRYSPSLKS | LIAVAFDY |

FIGURE 49

| | | | | | |
|---|---|---|---|---|---|
| | | | SEQ ID NO:7852 | SEQ ID NO:15864 | SEQ ID NO:23876 |
| **iPS:437340** | **21-225_75G9** | NA | GGTTGCTACTGGAGC | GAAATCAATCATAGTGGAAGGACCAACTACAACCCGTCCCTCAAGAGT | GACTACGGTGGGCTTGACTAC |
| | | | SEQ ID NO:7853 | SEQ ID NO:15865 | SEQ ID NO:23877 |
| | | AA | GCYWS | EINHSGRTNYNPSLKS | DYGGLDY |
| | | | SEQ ID NO:7854 | SEQ ID NO:15866 | SEQ ID NO:23878 |
| **iPS:437344** | **21-225_75G12** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:7855 | SEQ ID NO:15867 | SEQ ID NO:23879 |
| | | AA | GCYWS | EINHSGSTNYNPSLKS | DYGGMDV |
| | | | SEQ ID NO:7856 | SEQ ID NO:15868 | SEQ ID NO:23880 |
| **iPS:437346** | **21-225_75H7** | NA | AGGAGTAGTTACTACTGGGGC | AGTATCTATTATAGTGGGAGCGCCTACTCCAACCCGTCCCTCAAGAGT | CTTGACTCTAACTGGGGTCTTGACTAC |
| | | | SEQ ID NO:7857 | SEQ ID NO:15869 | SEQ ID NO:23881 |
| | | AA | RSSYYWG | SIYYSGSAYSNPSLKS | LDSNWGLDY |
| | | | SEQ ID NO:7858 | SEQ ID NO:15870 | SEQ ID NO:23882 |
| **iPS:437350** | **21-225_74A3** | NA | GGTTGCTACTGGAGC | GAAATCAATCATAGTGGAAGCACCAACTACAACCCGTCCCTCAAGAGT | GACTACGGCGGTATGGACGTC |
| | | | SEQ ID NO:7859 | SEQ ID NO:15871 | SEQ ID NO:23883 |
| | | AA | GCYWS | EINHSGSTNYNPSLKS | DYGGMDV |
| | | | SEQ ID NO:7860 | SEQ ID NO:15872 | SEQ ID NO:23884 |
| **iPS:437356** | **21-225_74B1** | NA | AATTATGATATCAAC | TGGATGAACCCTAACAGTGGTAACACAGGCTATGCACAGAAGTTCCAGGGC | ACCAGTGGCTGGAACTTCTTTGACTAC |
| | | | SEQ ID NO:7861 | SEQ ID NO:15873 | SEQ ID NO:23885 |

FIGURE 49

| | | | | | |
|---|---|---|---|---|---|
| | | AA | NYDIN | WMNPNSGNTGYAQKFQG | TSGWNFFDY |
| | | | SEQ ID NO:7862 | SEQ ID NO:15874 | SEQ ID NO:23886 |
| **iPS:437361** | **21-225_74C1** | NA | AATTATGATATCAAC | TGGATGAACCCTGACAG TGGTAACACAGGCTTTG CACAGAAGTTCCAGGGC | TCCAGTGGCTGGTACTGGTT CGACCCC |
| | | | SEQ ID NO:7863 | SEQ ID NO:15875 | SEQ ID NO:23887 |
| | | AA | NYDIN | WMNPDSGNTGFAQKFQG | SSGWYWFDP |
| | | | SEQ ID NO:7864 | SEQ ID NO:15876 | SEQ ID NO:23888 |
| **iPS:437363** | **21-225_74C10** | NA | AATTATGATATCAAC | TGGATGAACCCTAACAG TGGTAACATAGGCTATG CACAGAAGTTCCAGGGC | AGCAGTGGCTGGTACTGGTT CGACCCC |
| | | | SEQ ID NO:7865 | SEQ ID NO:15877 | SEQ ID NO:23889 |
| | | AA | NYDIN | WMNPNSGNIGYAQKFQG | SSGWYWFDP |
| | | | SEQ ID NO:7866 | SEQ ID NO:15878 | SEQ ID NO:23890 |
| **iPS:437369** | **21-225_74D6** | NA | GGTTGCTACTGGAGC | GAAATCAATCATAGTGG AAGCACCAACTACAACC CGTCCCTCAAGAGT | GACTACGGCGGTATGGACGT C |
| | | | SEQ ID NO:7867 | SEQ ID NO:15879 | SEQ ID NO:23891 |
| | | AA | GCYWS | EINHSGSTNYNPSLKS | DYGGMDV |
| | | | SEQ ID NO:7868 | SEQ ID NO:15880 | SEQ ID NO:23892 |
| **iPS:437371** | **21-225_74D8** | NA | AACTACGACATGCAC | GCTATTGGTACTGCTGGT GACACATACTATCCAGG CTCCGTGAAGGGC | GTTCTTGACTACGGTGACTC CTTGGGCTACTACTACTACG GTATGGACGTC |
| | | | SEQ ID NO:7869 | SEQ ID NO:15881 | SEQ ID NO:23893 |
| | | AA | NYDMH | AIGTAGDTYYPGSVKG | VLDYGDSLGYYYYGMDV |
| | | | SEQ ID NO:7870 | SEQ ID NO:15882 | SEQ ID NO:23894 |

## FIGURE 49

| | | | | | |
|---|---|---|---|---|---|
| **iPS:437377** | **21-225_74G9** | NA | ACTGGTGGAGTGGGTGTGGGC | CTCATTTATTGGGATGATGATAAGCGCTACAGCCCATCTCTGAAGAGC | CTTATAGCAGTGGCCTTTGACTAC |
| | | | SEQ ID NO:7871 | SEQ ID NO:15883 | SEQ ID NO:23895 |
| | | AA | TGGVGVG | LIYWDDDKRYSPSLKS | LIAVAFDY |
| | | | SEQ ID NO:7872 | SEQ ID NO:15884 | SEQ ID NO:23896 |
| **iPS:437379** | **21-225_74H2** | NA | AATTATGATATCAAC | TGGATGCACCCTAACAGTGGTAACACAGGCTTTGCACAGAAGTTCCAGGGC | TCCAGTGGCTGGTACTGGTTCGACCCC |
| | | | SEQ ID NO:7873 | SEQ ID NO:15885 | SEQ ID NO:23897 |
| | | AA | NYDIN | WMHPNSGNTGFAQKFQG | SSGWYWFDP |
| | | | SEQ ID NO:7874 | SEQ ID NO:15886 | SEQ ID NO:23898 |
| **iPS:437383** | **21-225_74H8** | NA | AACGCCTGGATGAAC | CGTATTAAAAGCAAAACTGATGGTGGGACAACAGACTACGCTGCACCCGTGAAAGGC | GTGGGAGCTACTACGGACTAC |
| | | | SEQ ID NO:7875 | SEQ ID NO:15887 | SEQ ID NO:23899 |
| | | AA | NAWMN | RIKSKTDGGTTDYAAPVKG | VGATTDY |
| | | | SEQ ID NO:7876 | SEQ ID NO:15888 | SEQ ID NO:23900 |
| **iPS:438664** | **21-225_216G1** | NA | AACTATGGCATGCAC | GTTATATGGTATGATGGAAGTAATAAATACTATGCAGACTCCGTGAAGGGC | GGGGACTGGAACCCCGAGGGTATGGACGTC |
| | | | SEQ ID NO:7877 | SEQ ID NO:15889 | SEQ ID NO:23901 |
| | | AA | NYGMH | VIWYDGSNKYYADSVKG | GDWNPEGMDV |
| | | | SEQ ID NO:7878 | SEQ ID NO:15890 | SEQ ID NO:23902 |

**FIGURE 49**

| | | | | | |
|---|---|---|---|---|---|
| **iPS:441468** | **21-225_25A4.001.001** | NA | AATTATGATATTAAT | TGGATGTACCCTAACAGTGGTAGCACAGGCTATGCACAGAAATTCCAGGGC | AGCAGTGGCTGGTACTACTTTGACTAC |
| | | | SEQ ID NO:7879 | SEQ ID NO:15891 | SEQ ID NO:23903 |
| | | AA | NYDIN | WMYPNSGSTGYAQKFQG | SSGWYYFDY |
| | | | SEQ ID NO:7880 | SEQ ID NO:15892 | SEQ ID NO:23904 |
| **iPS:441475** | **21-225_25A4.001.002** | NA | AATTATGATATTAAT | TGGATGTACCCTAACAGTGGTAACGCAGGCTATGCACAGAAATTCCAGGGC | AGCAGTGGCTGGTACTACTTTGACTAC |
| | | | SEQ ID NO:7881 | SEQ ID NO:15893 | SEQ ID NO:23905 |
| | | AA | NYDIN | WMYPNSGNAGYAQKFQG | SSGWYYFDY |
| | | | SEQ ID NO:7882 | SEQ ID NO:15894 | SEQ ID NO:23906 |
| **iPS:441482** | **21-225_25A4.001.003** | NA | AATTATGATATTAAT | TGGATGTACCCTAACAGTGGTAACGTAGGCTATGCACAGAAATTCCAGGGC | AGCAGTGGCTGGTACTACTTTGACTAC |
| | | | SEQ ID NO:7883 | SEQ ID NO:15895 | SEQ ID NO:23907 |
| | | AA | NYDIN | WMYPNSGNVGYAQKFQG | SSGWYYFDY |
| | | | SEQ ID NO:7884 | SEQ ID NO:15896 | SEQ ID NO:23908 |
| **iPS:441489** | **21-225_25A4.001.004** | NA | AATTATGATATTAAT | TGGATGTACCCTAACAGTGGTCAAACAGGCTATGCACAGAAATTCCAGGGC | AGCAGTGGCTGGTACTACTTTGACTAC |
| | | | SEQ ID NO:7885 | SEQ ID NO:15897 | SEQ ID NO:23909 |

**FIGURE 49**

| | | | | | |
|---|---|---|---|---|---|
| | | AA | NYDIN | WMYPNSGQTGYAQKFQG | SSGWYYFDY |
| | | | SEQ ID NO:7886 | SEQ ID NO:15898 | SEQ ID NO:23910 |
| **iPS:441496** | **21-225_25A4.001.005** | NA | AATTATGATATTAAT | TGGATGTACCCTAACAG TGGTAGCACAGGCTATG CACAGAAATTCCAGGGC | AGCAGTGGCTGGTACTACTT TGACTAC |
| | | | SEQ ID NO:7887 | SEQ ID NO:15899 | SEQ ID NO:23911 |
| | | AA | NYDIN | WMYPNSGSTGYAQKFQG | SSGWYYFDY |
| | | | SEQ ID NO:7888 | SEQ ID NO:15900 | SEQ ID NO:23912 |
| **iPS:441505** | **21-225_25A4.001.006** | NA | AATTATGATATTAAT | TGGATGTACCCTAACAG TGGTAACGCAGGCTATG CACAGAAATTCCAGGGC | AGCAGTGGCTGGTACTACTT TGACTAC |
| | | | SEQ ID NO:7889 | SEQ ID NO:15901 | SEQ ID NO:23913 |
| | | AA | NYDIN | WMYPNSGNAGYAQKFQG | SSGWYYFDY |
| | | | SEQ ID NO:7890 | SEQ ID NO:15902 | SEQ ID NO:23914 |
| **iPS:441512** | **21-225_25A4.001.007** | NA | AATTATGATATTAAT | TGGATGTACCCTAACAG TGGTAACGTAGGCTATG CACAGAAATTCCAGGGC | AGCAGTGGCTGGTACTACTT TGACTAC |
| | | | SEQ ID NO:7891 | SEQ ID NO:15903 | SEQ ID NO:23915 |
| | | AA | NYDIN | WMYPNSGNVGYAQKFQG | SSGWYYFDY |
| | | | SEQ ID NO:7892 | SEQ ID NO:15904 | SEQ ID NO:23916 |

## FIGURE 49

| | | | | | |
|---|---|---|---|---|---|
| **iPS:441519** | **21-225_25A4.001.008** | NA | AATTATGATATTAAT | TGGATGTACCCTAACAGTGGTCAAACAGGCTATGCACAGAAATTCCAGGGC | AGCAGTGGCTGGTACTACTTTGACTAC |
| | | | SEQ ID NO:7893 | SEQ ID NO:15905 | SEQ ID NO:23917 |
| | | AA | NYDIN | WMYPNSGQTGYAQKFQG | SSGWYYFDY |
| | | | SEQ ID NO:7894 | SEQ ID NO:15906 | SEQ ID NO:23918 |
| **iPS:441554** | **21-225_25A4.001.013** | NA | AATTATGATATTAAT | TGGATGTACCCTAACAGTGGTAGCACAGGCTATGCACAGAAATTCCAGGGC | AGCAGTGGCTGGTACTACTTTGACTAC |
| | | | SEQ ID NO:7895 | SEQ ID NO:15907 | SEQ ID NO:23919 |
| | | AA | NYDIN | WMYPNSGSTGYAQKFQG | SSGWYYFDY |
| | | | SEQ ID NO:7896 | SEQ ID NO:15908 | SEQ ID NO:23920 |
| **iPS:441595** | **21-225_25A4.001.019** | NA | AATTATGATATTAAT | TGGATGTACCCTAACAGTGGTAGCACAGGCTATGCACAGAAATTCCAGGGC | AGCAGTGGCTGGTACTACTTTGACTAC |
| | | | SEQ ID NO:7897 | SEQ ID NO:15909 | SEQ ID NO:23921 |
| | | AA | NYDIN | WMYPNSGSTGYAQKFQG | SSGWYYFDY |
| | | | SEQ ID NO:7898 | SEQ ID NO:15910 | SEQ ID NO:23922 |
| **iPS:441604** | **21-225_25A4.001.020** | NA | AATTATGATATTAAT | TGGATGTACCCTAACAGTGGTCAAACAGGCTATGCACAGAAATTCCAGGGC | AGCAGTGGCTGGTACTACTTTGACTAC |
| | | | SEQ ID NO:7899 | SEQ ID NO:15911 | SEQ ID NO:23923 |

## FIGURE 49

| | | | | | |
|---|---|---|---|---|---|
| | | AA | NYDIN | WMYPNSGQTGYAQKFQG | SSGWYYFDY |
| | | | SEQ ID NO:7900 | SEQ ID NO:15912 | SEQ ID NO:23924 |
| **iPS:441613** | **21-225_25A4.001.021** | NA | AATTATGATATTAAT | TGGATGTACCCTAACAGTGGTAACGCAGGCTATGCACAGAAATTCCAGGGC | AGCAGTGGCTGGTACTACTTTGACTAC |
| | | | SEQ ID NO:7901 | SEQ ID NO:15913 | SEQ ID NO:23925 |
| | | AA | NYDIN | WMYPNSGNAGYAQKFQG | SSGWYYFDY |
| | | | SEQ ID NO:7902 | SEQ ID NO:15914 | SEQ ID NO:23926 |
| **iPS:441841** | **21-225_4A2.001.001** | NA | AATTATGATATCAAC | TGGATGCACCCTAACAGTGGTAACACAGGCTATGCACAGAAGTTCCAGGGC | AGCAGTGGCTGGTACTACTTTGACTAC |
| | | | SEQ ID NO:7903 | SEQ ID NO:15915 | SEQ ID NO:23927 |
| | | AA | NYDIN | WMHPNSGNTGYAQKFQG | SSGWYYFDY |
| | | | SEQ ID NO:7904 | SEQ ID NO:15916 | SEQ ID NO:23928 |
| **iPS:441847** | **21-225_4A2.001.002** | NA | AATTATGATATCAAC | TGGATGCACCCTAACAGTGGTAACACAGGCTATGCACAGAAGTTCCAGGGC | AGCAGTGGCTGGTACTACTTTGACTAC |
| | | | SEQ ID NO:7905 | SEQ ID NO:15917 | SEQ ID NO:23929 |
| | | AA | NYDIN | WMHPNSGNTGYAQKFQG | SSGWYYFDY |
| | | | SEQ ID NO:7906 | SEQ ID NO:15918 | SEQ ID NO:23930 |
| **iPS:441853** | **21-225_4A2.001.003** | NA | AATTATGATATCAAC | TGGATGCACCCTAACAGTGGTAACACAGGCTATGCACAGAAGTTCCAGGGC | AGCAGTGGCTGGTACTACTTTGACTAC |
| | | | SEQ ID NO:7907 | SEQ ID NO:15919 | SEQ ID NO:23931 |

FIGURE 49

| | | | | | |
|---|---|---|---|---|---|
| | | AA | NYDIN | WMHPNSGNTGYAQKFQG | SSGWYYFDY |
| | | | SEQ ID NO:7908 | SEQ ID NO:15920 | SEQ ID NO:23932 |
| **iPS:441859** | **21-225_4A2.001.004** | NA | AATTATGATATCAAC | TGGATGCACCCTAACAG TGGTAACGCAGGCTATG CACAGAAGTTCCAGGGC | AGCAGTGGCTGGTACTACTT TGACTAC |
| | | | SEQ ID NO:7909 | SEQ ID NO:15921 | SEQ ID NO:23933 |
| | | AA | NYDIN | WMHPNSGNAGYAQKFQG | SSGWYYFDY |
| | | | SEQ ID NO:7910 | SEQ ID NO:15922 | SEQ ID NO:23934 |
| **iPS:441866** | **21-225_4A2.001.005** | NA | AATTATGATATCAAC | TGGATGCACCCTAACAG TGGTAGCACAGGCTATG CACAGAAGTTCCAGGGC | AGCAGTGGCTGGTACTACTT TGACTAC |
| | | | SEQ ID NO:7911 | SEQ ID NO:15923 | SEQ ID NO:23935 |
| | | AA | NYDIN | WMHPNSGSTGYAQKFQG | SSGWYYFDY |
| | | | SEQ ID NO:7912 | SEQ ID NO:15924 | SEQ ID NO:23936 |
| **iPS:441873** | **21-225_4A2.001.006** | NA | AATTATGATATCAAC | TGGATGCACCCTAACAG TGGTCAAACAGGCTATG CACAGAAGTTCCAGGGC | AGCAGTGGCTGGTACTACTT TGACTAC |
| | | | SEQ ID NO:7913 | SEQ ID NO:15925 | SEQ ID NO:23937 |
| | | AA | NYDIN | WMHPNSGQTGYAQKFQG | SSGWYYFDY |
| | | | SEQ ID NO:7914 | SEQ ID NO:15926 | SEQ ID NO:23938 |
| **iPS:441880** | **21-225_4A2.001.007** | NA | AATTATGATATCAAC | TGGATGCACCCTAACAG TGGTCAAACAGGCTATG CACAGAAGTTCCAGGGC | AGCAGTGGCTGGTACTACTT TGACTAC |
| | | | SEQ ID NO:7915 | SEQ ID NO:15927 | SEQ ID NO:23939 |
| | | AA | NYDIN | WMHPNSGQTGYAQKFQG | SSGWYYFDY |

FIGURE 49

| | | | | | |
|---|---|---|---|---|---|
| | | | SEQ ID NO:7916 | SEQ ID NO:15928 | SEQ ID NO:23940 |
| **iPS:441884** | **21-225_4A2.001.008** | NA | AATTATGATATCAAC | TGGATGCACCCTAACAG TGGTAACGCAGGCTATG CACAGAAGTTCCAGGGC | AGCAGTGGCTGGTACTACTT TGACTAC |
| | | | SEQ ID NO:7917 | SEQ ID NO:15929 | SEQ ID NO:23941 |
| | | AA | NYDIN | WMHPNSGNAGYAQKFQG | SSGWYYFDY |
| | | | SEQ ID NO:7918 | SEQ ID NO:15930 | SEQ ID NO:23942 |
| **iPS:441888** | **21-225_4A2.001.009** | NA | AATTATGATATCAAC | TGGATGCACCCTAACAG TGGTCAAACAGGCTATG CACAGAAGTTCCAGGGC | AGCAGTGGCTGGTACTACTT TGACTAC |
| | | | SEQ ID NO:7919 | SEQ ID NO:15931 | SEQ ID NO:23943 |
| | | AA | NYDIN | WMHPNSGQTGYAQKFQG | SSGWYYFDY |
| | | | SEQ ID NO:7920 | SEQ ID NO:15932 | SEQ ID NO:23944 |
| **iPS:441892** | **21-225_4A2.001.010** | NA | AATTATGATATCAAC | TGGATGCACCCTAACAG TGGTAACGCAGGCTATG CACAGAAGTTCCAGGGC | AGCAGTGGCTGGTACTACTT TGACTAC |
| | | | SEQ ID NO:7921 | SEQ ID NO:15933 | SEQ ID NO:23945 |
| | | AA | NYDIN | WMHPNSGNAGYAQKFQG | SSGWYYFDY |
| | | | SEQ ID NO:7922 | SEQ ID NO:15934 | SEQ ID NO:23946 |
| **iPS:441896** | **21-225_4A2.001.011** | NA | AATTATGATATCAAC | TGGATGCACCCTAACAG TGGTCAAACAGGCTATG CACAGAAGTTCCAGGGC | AGCAGTGGCTGGTACTACTT TGACTAC |
| | | | SEQ ID NO:7923 | SEQ ID NO:15935 | SEQ ID NO:23947 |
| | | AA | NYDIN | WMHPNSGQTGYAQKFQG | SSGWYYFDY |
| | | | SEQ ID NO:7924 | SEQ ID NO:15936 | SEQ ID NO:23948 |

**FIGURE 49**

| | | | | | |
|---|---|---|---|---|---|
| **iPS:441900** | **21-225_4A2.001.012** | NA | AATTATGATATCAAC | TGGATGCACCCTAACAGTGGTAGCACAGGCTATGCACAGAAGTTCCAGGGC | AGCAGTGGCTGGTACTACTTTGACTAC |
| | | | SEQ ID NO:7925 | SEQ ID NO:15937 | SEQ ID NO:23949 |
| | | AA | NYDIN | WMHPNSGSTGYAQKFQG | SSGWYYFDY |
| | | | SEQ ID NO:7926 | SEQ ID NO:15938 | SEQ ID NO:23950 |
| **iPS:441955** | **21-225_4A2.001.022** | NA | AATTATGATATCAAC | TGGATGCACCCTAACAGTGGTAACACAGGCTATGCACAGAAGTTCCAGGGC | AGCAGTGGCTGGTACTACTTTGACTAC |
| | | | SEQ ID NO:7927 | SEQ ID NO:15939 | SEQ ID NO:23951 |
| | | AA | NYDIN | WMHPNSGNTGYAQKFQG | SSGWYYFDY |
| | | | SEQ ID NO:7928 | SEQ ID NO:15940 | SEQ ID NO:23952 |
| **iPS:441962** | **21-225_4A2.001.023** | NA | AATTATGATATCAAC | TGGATGCACCCTAACAGTGGTAACACAGGCTATGCACAGAAGTTCCAGGGC | AGCAGTGGCTGGTACTACTTTGACTAC |
| | | | SEQ ID NO:7929 | SEQ ID NO:15941 | SEQ ID NO:23953 |
| | | AA | NYDIN | WMHPNSGNTGYAQKFQG | SSGWYYFDY |
| | | | SEQ ID NO:7930 | SEQ ID NO:15942 | SEQ ID NO:23954 |
| **iPS:441971** | **21-225_4A2.001.024** | NA | AATTATGATATCAAC | TGGATGCACCCTAACAGTGGTCAAACAGGCTATGCACAGAAGTTCCAGGGC | AGCAGTGGCTGGTACTACTTTGACTAC |
| | | | SEQ ID NO:7931 | SEQ ID NO:15943 | SEQ ID NO:23955 |
| | | AA | NYDIN | WMHPNSGQTGYAQKFQG | SSGWYYFDY |
| | | | SEQ ID NO:7932 | SEQ ID NO:15944 | SEQ ID NO:23956 |

FIGURE 49

| | | | | | |
|---|---|---|---|---|---|
| **iPS:441999** | **21-225_4A2.001.028** | NA | AATTATGATATCAAC | TGGATGCACCCTAACAGTGGTCAAACAGGCTATGCACAGAAGTTCCAGGGC | AGCAGTGGCTGGTACTACTTTGACTAC |
| | | | SEQ ID NO:7933 | SEQ ID NO:15945 | SEQ ID NO:23957 |
| | | AA | NYDIN | WMHPNSGQTGYAQKFQG | SSGWYYFDY |
| | | | SEQ ID NO:7934 | SEQ ID NO:15946 | SEQ ID NO:23958 |
| **iPS:442006** | **21-225_4A2.001.029** | NA | AATTATGATATCAAC | TGGATGCACCCTAACAGTGGTAGCACAGGCTATGCACAGAAGTTCCAGGGC | AGCAGTGGCTGGTACTACTTTGACTAC |
| | | | SEQ ID NO:7935 | SEQ ID NO:15947 | SEQ ID NO:23959 |
| | | AA | NYDIN | WMHPNSGSTGYAQKFQG | SSGWYYFDY |
| | | | SEQ ID NO:7936 | SEQ ID NO:15948 | SEQ ID NO:23960 |
| **iPS:442020** | **21-225_4A2.001.031** | NA | AATTATGATATCAAC | TGGATGCACCCTAACAGTGGTAACGAAGGCTATGCACAGAAGTTCCAGGGC | AGCAGTGGCTGGTACTACTTTGACTAC |
| | | | SEQ ID NO:7937 | SEQ ID NO:15949 | SEQ ID NO:23961 |
| | | AA | NYDIN | WMHPNSGNEGYAQKFQG | SSGWYYFDY |
| | | | SEQ ID NO:7938 | SEQ ID NO:15950 | SEQ ID NO:23962 |
| **iPS:442050** | **21-225_4H6.004** | NA | GACTACTATTTGCAC | TGGATCCACCCTAACAGTGGTGGCACAAACTATGCACAGAAGTTTCAGGGC | GATGGTACCAGCTCGTTTGACTAC |
| | | | SEQ ID NO:7939 | SEQ ID NO:15951 | SEQ ID NO:23963 |
| | | AA | DYYLH | WIHPNSGGTNYAQKFQG | DGTSSFDY |
| | | | SEQ ID NO:7940 | SEQ ID NO:15952 | SEQ ID NO:23964 |
| **iPS:442059** | **21 225 4H6 005** | NA | GACTACTATTTGCAC | TGGATCCACCCTAACAGTGGTGGCACAAACTATGCACAGAAGTTTCAGGGC | GATGGTACCAGCTCGTTTGACTAC |

FIGURE 49

| | | | | | |
|---|---|---|---|---|---|
| | **21-225_4H6.005** | | SEQ ID NO:7941 | SEQ ID NO:15953 | SEQ ID NO:23965 |
| | | AA | DYYLH | WIHPNSGGTNYAQKFQG | DGTSSFDY |
| | | | SEQ ID NO:7942 | SEQ ID NO:15954 | SEQ ID NO:23966 |
| **iPS:442065** | **21-225_4H6.006** | NA | GACTACTATTTGCAC | TGGATCCACCCTAACAGTGGTGGCACAAACTATGCACAGAAGTTTCAGGGC | GATGGTACCAGCTCGTTTGACTAC |
| | | | SEQ ID NO:7943 | SEQ ID NO:15955 | SEQ ID NO:23967 |
| | | AA | DYYLH | WIHPNSGGTNYAQKFQG | DGTSSFDY |
| | | | SEQ ID NO:7944 | SEQ ID NO:15956 | SEQ ID NO:23968 |
| **iPS:442071** | **21-225_4H6.007** | NA | GACTACTATTTGCAC | TGGATCCACCCTAACAGTGGTGGCACAAACTATGCACAGAAGTTTCAGGGC | GATGCTACCAGCTCGTTTGACTAC |
| | | | SEQ ID NO:7945 | SEQ ID NO:15957 | SEQ ID NO:23969 |
| | | AA | DYYLH | WIHPNSGGTNYAQKFQG | DATSSFDY |
| | | | SEQ ID NO:7946 | SEQ ID NO:15958 | SEQ ID NO:23970 |
| **iPS:442078** | **21-225_4H6.008** | NA | GACTACTATTTGCAC | TGGATCCACCCTAACAGTGGTGGCACAAACTATGCACAGAAGTTTCAGGGC | AGTGGTACCAGCTCGTTTGACTAC |
| | | | SEQ ID NO:7947 | SEQ ID NO:15959 | SEQ ID NO:23971 |
| | | AA | DYYLH | WIHPNSGGTNYAQKFQG | SGTSSFDY |
| | | | SEQ ID NO:7948 | SEQ ID NO:15960 | SEQ ID NO:23972 |
| **iPS:442085** | **21-225_4H6.009** | NA | GACTACTATTTGCAC | TGGATCCACCCTAACAGTGGTGGCACAAACTATGCACAGAAGTTTCAGGGC | AGTGGTACCAGCTCGTTTGACTAC |
| | | | SEQ ID NO:7949 | SEQ ID NO:15961 | SEQ ID NO:23973 |
| | | AA | DYYLH | WIHPNSGGTNYAQKFQG | SGTSSFDY |
| | | | SEQ ID NO:7950 | SEQ ID NO:15962 | SEQ ID NO:23974 |
| **iPS:442089** | **21 225 4H6 010** | NA | GACTACTATTTGCAC | TGGATCCACCCTAACAGTGGTGGCACAAACTATGCACAGAAGTTTCAGGGC | GATGCTACCAGCTCGTTTGACTAC |

**FIGURE 49**

| | | | | | |
|---|---|---|---|---|---|
| | **21-225_4H6.010** | | SEQ ID NO:7951 | SEQ ID NO:15963 | SEQ ID NO:23975 |
| | | AA | DYYLH | WIHPNSGGTNYAQKFQG | DATSSFDY |
| | | | SEQ ID NO:7952 | SEQ ID NO:15964 | SEQ ID NO:23976 |
| **iPS:442093** | **21-225_4H6.011** | NA | GACTACTATTTGCAC | TGGATCCACCCTAACAG TGGTGGCACAAACTATG CACAGAAGTTTCAGGGC | GATGCTACCAGCTCGTTTGA CTAC |
| | | | SEQ ID NO:7953 | SEQ ID NO:15965 | SEQ ID NO:23977 |
| | | AA | DYYLH | WIHPNSGGTNYAQKFQG | DATSSFDY |
| | | | SEQ ID NO:7954 | SEQ ID NO:15966 | SEQ ID NO:23978 |
| **iPS:442115** | **21-225_5E5.003** | NA | AACTATGTCATGCAC | GTTATCTGGTATGATGCA AGTAATAAATACTATGC AGACTCCGTGAAGGGC | GAGGTATATAGCAGTGGCTG GTACGACTACGGTATGGACG TC |
| | | | SEQ ID NO:7955 | SEQ ID NO:15967 | SEQ ID NO:23979 |
| | | AA | NYVMH | VIWYDASNKYYADSVKG | EVYSSGWYDYGMDV |
| | | | SEQ ID NO:7956 | SEQ ID NO:15968 | SEQ ID NO:23980 |
| **iPS:442122** | **21-225_5E5.004** | NA | AACTATGTCATGCAC | GTTATCTGGTATGATGGA AGTAATAAATACTATGC AGAATCCGTGAAGGGC | GAGGTATATAGCAGTGGCTG GTACGACTACGGTATGGACG TC |
| | | | SEQ ID NO:7957 | SEQ ID NO:15969 | SEQ ID NO:23981 |
| | | AA | NYVMH | VIWYDGSNKYYAESVKG | EVYSSGWYDYGMDV |
| | | | SEQ ID NO:7958 | SEQ ID NO:15970 | SEQ ID NO:23982 |
| **iPS:442129** | **21-225_5E5.005** | NA | AACTATGTCATGCAC | GTTATCTGGTATGATGGA AGTAATAAATACTATGC AGGCTCCGTGAAGGGC | GAGGTATATAGCAGTGGCTG GTACGACTACGGTATGGACG TC |
| | | | SEQ ID NO:7959 | SEQ ID NO:15971 | SEQ ID NO:23983 |

FIGURE 49

| | | | | | |
|---|---|---|---|---|---|
| | | AA | NYVMH<br>SEQ ID NO:7960 | VIWYDGSNKYYAGSVKG<br>SEQ ID NO:15972 | EVYSSGWYDYGMDV<br>SEQ ID NO:23984 |
| **iPS:442136** | **21-225_5E5.006** | NA | AACTATGTCATGCAC<br>SEQ ID NO:7961 | GTTATCTGGTATGATGGAAGTAATAAATACTATGCAGACGCCGTGAAGGGC<br>SEQ ID NO:15973 | GAGGTATATAGCAGTGGCTGGTACGACTACGGTATGGACGTC<br>SEQ ID NO:23985 |
| | | AA | NYVMH<br>SEQ ID NO:7962 | VIWYDGSNKYYADAVKG<br>SEQ ID NO:15974 | EVYSSGWYDYGMDV<br>SEQ ID NO:23986 |
| **iPS:442171** | **21-225_5E5.011** | NA | AACTATGTCATGCAC<br>SEQ ID NO:7963 | GTTATCTGGTATGATGCAAGTAATAAATACTATGCAGAATCCGTGAAGGGC<br>SEQ ID NO:15975 | GAGGTATATAGCAGTGGCTGGTACGACTACGGTATGGACGTC<br>SEQ ID NO:23987 |
| | | AA | NYVMH<br>SEQ ID NO:7964 | VIWYDASNKYYAESVKG<br>SEQ ID NO:15976 | EVYSSGWYDYGMDV<br>SEQ ID NO:23988 |
| **iPS:442178** | **21-225_5E5.012** | NA | AACTATGTCATGCAC<br>SEQ ID NO:7965 | GTTATCTGGTATGATGCAAGTAATAAATACTATGCAGACGCCGTGAAGGGC<br>SEQ ID NO:15977 | GAGGTATATAGCAGTGGCTGGTACGACTACGGTATGGACGTC<br>SEQ ID NO:23989 |
| | | AA | NYVMH<br>SEQ ID NO:7966 | VIWYDASNKYYADAVKG<br>SEQ ID NO:15978 | EVYSSGWYDYGMDV<br>SEQ ID NO:23990 |

**FIGURE 49**

| | | | | | |
|---|---|---|---|---|---|
| **iPS:442199** | **21-225_5E5.015** | NA | AACTATGTCATGCAC | GTTATCTGGTATGATGCAAGTAATAAATACTATGCAGACTCCGTGAAGGGC | GAGGTATATAGCAGTGGCTATTACGACTACGGTATGGACGTC |
| | | | SEQ ID NO:7967 | SEQ ID NO:15979 | SEQ ID NO:23991 |
| | | AA | NYVMH | VIWYDASNKYYADSVKG | EVYSSGYYDYGMDV |
| | | | SEQ ID NO:7968 | SEQ ID NO:15980 | SEQ ID NO:23992 |
| **iPS:442206** | **21-225_5E5.016** | NA | AACTATGTCATGCAC | GTTATCTGGTATGATGCAAGTAATAAATACTATGCAGACTCCGTGAAGGGC | GAGGTATATAGCAGTGGCTTCTACGACTACGGTATGGACGTC |
| | | | SEQ ID NO:7969 | SEQ ID NO:15981 | SEQ ID NO:23993 |
| | | AA | NYVMH | VIWYDASNKYYADSVKG | EVYSSGFYDYGMDV |
| | | | SEQ ID NO:7970 | SEQ ID NO:15982 | SEQ ID NO:23994 |
| **iPS:442213** | **21-225_5E5.017** | NA | AACTATGTCATGCAC | GTTATCTGGTATGATGGAAGTAATAAATACTATGCAGAATCCGTGAAGGGC | GAGGTATATAGCAGTGGCTATTACGACTACGGTATGGACGTC |
| | | | SEQ ID NO:7971 | SEQ ID NO:15983 | SEQ ID NO:23995 |
| | | AA | NYVMH | VIWYDGSNKYYAESVKG | EVYSSGYYDYGMDV |
| | | | SEQ ID NO:7972 | SEQ ID NO:15984 | SEQ ID NO:23996 |
| **iPS:442220** | **21-225_5E5.018** | NA | AACTATGTCATGCAC | GTTATCTGGTATGATGGAAGTAATAAATACTATGCAGACTCCGTGAAGGGC | GAGGTATATAGCAGTGGCTATTACGACTACGGTATGGACGTC |
| | | | SEQ ID NO:7973 | SEQ ID NO:15985 | SEQ ID NO:23997 |

**FIGURE 49**

| | | | | | |
|---|---|---|---|---|---|
| | | AA | NYVMH | VIWYDGSNKYYADSVKG | EVYSSGYYDYGMDV |
| | | | SEQ ID NO:7974 | SEQ ID NO:15986 | SEQ ID NO:23998 |
| **iPS:442227** | **21-225_5E5.019** | NA | AACTATGTCATGCAC | GTTATCTGGTATGATGGAAGTAATAAATACTATGCAGACTCCGTGAAGGGC | GAGGTATATAGCAGTGGCTTCTACGACTACGGTATGGACGTC |
| | | | SEQ ID NO:7975 | SEQ ID NO:15987 | SEQ ID NO:23999 |
| | | AA | NYVMH | VIWYDGSNKYYADSVKG | EVYSSGFYDYGMDV |
| | | | SEQ ID NO:7976 | SEQ ID NO:15988 | SEQ ID NO:24000 |
| **iPS:442255** | **21-225_5E5.023** | NA | AACTATGTCATGCAC | GTTATCTGGTATGATGCAAGTAATAAATACTATGCAGAATCCGTGAAGGGC | GAGGTATATAGCAGTGGCTATTACGACTACGGTATGGACGTC |
| | | | SEQ ID NO:7977 | SEQ ID NO:15989 | SEQ ID NO:24001 |
| | | AA | NYVMH | VIWYDASNKYYAESVKG | EVYSSGYYDYGMDV |
| | | | SEQ ID NO:7978 | SEQ ID NO:15990 | SEQ ID NO:24002 |
| **iPS:442262** | **21-225_5E5.024** | NA | AACTATGTCATGCAC | GTTATCTGGTATGATGCAAGTAATAAATACTATGCAGACGCCGTGAAGGGC | GAGGTATATAGCAGTGGCTATTACGACTACGGTATGGACGTC |
| | | | SEQ ID NO:7979 | SEQ ID NO:15991 | SEQ ID NO:24003 |
| | | AA | NYVMH | VIWYDASNKYYADAVKG | EVYSSGYYDYGMDV |
| | | | SEQ ID NO:7980 | SEQ ID NO:15992 | SEQ ID NO:24004 |

FIGURE 49

| | | | | | |
|---|---|---|---|---|---|
| **iPS:442269** | **21-225_5E5.025** | NA | AACTATGTCATGCAC | GTTATCTGGTATGATGGAAGTAATAAATACTATGCAGAATCCGTGAAGGGC | GAGGTATATAGCAGTGGCTGGTACGACTACGGTATGGACGTC |
| | | | SEQ ID NO:7981 | SEQ ID NO:15993 | SEQ ID NO:24005 |
| | | AA | NYVMH | VIWYDGSNKYYAESVKG | EVYSSGWYDYGMDV |
| | | | SEQ ID NO:7982 | SEQ ID NO:15994 | SEQ ID NO:24006 |
| **iPS:442311** | **21-225_7E11.001.001** | NA | AGCTTTGGCATGCAC | ATTATCTGGCATGATGGAAGTAATAAATACTATGCAGACTCCGTGAAGGGC | GATCTGAGTATGGGCGGTATGGACGTC |
| | | | SEQ ID NO:7983 | SEQ ID NO:15995 | SEQ ID NO:24007 |
| | | AA | SFGMH | IIWHDGSNKYYADSVKG | DLSMGGMDV |
| | | | SEQ ID NO:7984 | SEQ ID NO:15996 | SEQ ID NO:24008 |
| **iPS:442317** | **21-225_7E11.001.002** | NA | AGCTTTGGCATGCAC | ATTATCTGGCATGATGGAAGTAATAAATACTATGCAGACTCCGTGAAGGGC | GATCTGAGTATGGGCGGTATGGACGTC |
| | | | SEQ ID NO:7985 | SEQ ID NO:15997 | SEQ ID NO:24009 |
| | | AA | SFGMH | IIWHDGSNKYYADSVKG | DLSMGGMDV |
| | | | SEQ ID NO:7986 | SEQ ID NO:15998 | SEQ ID NO:24010 |
| **iPS:442323** | **21-225_7E11.001.003** | NA | AGCTTTGGCATGCAC | ATTATCTGGCATAGTGGAAGTAATAAATACTATGCAGACTCCGTGAAGGGC | GATCTGAGTATGGGCGGTATGGACGTC |
| | | | SEQ ID NO:7987 | SEQ ID NO:15999 | SEQ ID NO:24011 |
| | | AA | SFGMH | IIWHSGSNKYYADSVKG | DLSMGGMDV |
| | | | SEQ ID NO:7988 | SEQ ID NO:16000 | SEQ ID NO:24012 |

FIGURE 49

| | | | | | |
|---|---|---|---|---|---|
| **iPS:442330** | **21-225_7E11.001.004** | NA | AGCTTTGGCATGCAC | ATTATCTGGCATGAAGGAAGTAATAAATACTATGCAGACTCCGTGAAGGGC | GATCTGAGTATGGGCGGTATGGACGTC |
| | | | SEQ ID NO:7989 | SEQ ID NO:16001 | SEQ ID NO:24013 |
| | | AA | SFGMH | IIWHEGSNKYYADSVKG | DLSMGGMDV |
| | | | SEQ ID NO:7990 | SEQ ID NO:16002 | SEQ ID NO:24014 |
| **iPS:442337** | **21-225_7E11.001.005** | NA | AGCTTTGGCATGCAC | ATTATCTGGCATGATGCAAGTAATAAATACTATGCAGACTCCGTGAAGGGC | GATCTGAGTATGGGCGGTATGGACGTC |
| | | | SEQ ID NO:7991 | SEQ ID NO:16003 | SEQ ID NO:24015 |
| | | AA | SFGMH | IIWHDASNKYYADSVKG | DLSMGGMDV |
| | | | SEQ ID NO:7992 | SEQ ID NO:16004 | SEQ ID NO:24016 |
| **iPS:442344** | **21-225_7E11.001.006** | NA | AGCTTTGGCATGCAC | ATTATCTGGCATGATGGAAGTAATAAATACTATGCAGAATCCGTGAAGGGC | GATCTGAGTATGGGCGGTATGGACGTC |
| | | | SEQ ID NO:7993 | SEQ ID NO:16005 | SEQ ID NO:24017 |
| | | AA | SFGMH | IIWHDGSNKYYAESVKG | DLSMGGMDV |
| | | | SEQ ID NO:7994 | SEQ ID NO:16006 | SEQ ID NO:24018 |
| **iPS:442351** | **21-225_7E11.001.007** | NA | AGCTTTGGCATGCAC | ATTATCTGGCATGATGGAAGTAATAAATACTATGCAGACGCCGTGAAGGGC | GATCTGAGTATGGGCGGTATGGACGTC |
| | | | SEQ ID NO:7995 | SEQ ID NO:16007 | SEQ ID NO:24019 |
| | | AA | SFGMH | IIWHDGSNKYYADAVKG | DLSMGGMDV |
| | | | SEQ ID NO:7996 | SEQ ID NO:16008 | SEQ ID NO:24020 |

FIGURE 49

| | | | | | |
|---|---|---|---|---|---|
| **iPS:442358** | **21-225_7E11.001.008** | NA | AGCTTTGGCATGCAC | ATTATCTGGCATGAAGGAAGTAATAAATACTATGCAGACGCCGTGAAGGGC | GATCTGAGTATGGGCGGTATGGACGTC |
| | | | SEQ ID NO:7997 | SEQ ID NO:16009 | SEQ ID NO:24021 |
| | | AA | SFGMH | IIWHEGSNKYYADAVKG | DLSMGGMDV |
| | | | SEQ ID NO:7998 | SEQ ID NO:16010 | SEQ ID NO:24022 |
| **iPS:442365** | **21-225_7E11.001.009** | NA | AGCTTTGGCATGCAC | ATTATCTGGCATGAAGGAAGTAATAAATACTATGCAGAATCCGTGAAGGGC | GATCTGAGTATGGGCGGTATGGACGTC |
| | | | SEQ ID NO:7999 | SEQ ID NO:16011 | SEQ ID NO:24023 |
| | | AA | SFGMH | IIWHEGSNKYYAESVKG | DLSMGGMDV |
| | | | SEQ ID NO:8000 | SEQ ID NO:16012 | SEQ ID NO:24024 |
| **iPS:442372** | **21-225_7E11.001.010** | NA | AGCTTTGGCATGCAC | ATTATCTGGCATAGTGGAAGTAATAAATACTATGCAGAATCCGTGAAGGGC | GATCTGAGTATGGGCGGTATGGACGTC |
| | | | SEQ ID NO:8001 | SEQ ID NO:16013 | SEQ ID NO:24025 |
| | | AA | SFGMH | IIWHSGSNKYYAESVKG | DLSMGGMDV |
| | | | SEQ ID NO:8002 | SEQ ID NO:16014 | SEQ ID NO:24026 |
| **iPS:442379** | **21-225_7E11.001.011** | NA | AGCTTTGGCATGCAC | ATTATCTGGCATAGTGGAAGTAATAAATACTATGCAGACGCCGTGAAGGGC | GATCTGAGTATGGGCGGTATGGACGTC |
| | | | SEQ ID NO:8003 | SEQ ID NO:16015 | SEQ ID NO:24027 |
| | | AA | SFGMH | IIWHSGSNKYYADAVKG | DLSMGGMDV |
| | | | SEQ ID NO:8004 | SEQ ID NO:16016 | SEQ ID NO:24028 |

FIGURE 49

| | | | | | |
|---|---|---|---|---|---|
| **iPS:442386** | **21-225_7E11.001.012** | NA | AGCTTTGGCATGCAC | ATTATCTGGCATAGTGGAAGTAATAAATACTATGCAGACTCCGTGAAGGGC | GATCTGAGTATGGGCGGTATGGACGTC |
| | | | SEQ ID NO:8005 | SEQ ID NO:16017 | SEQ ID NO:24029 |
| | | AA | SFGMH | IIWHSGSNKYYADSVKG | DLSMGGMDV |
| | | | SEQ ID NO:8006 | SEQ ID NO:16018 | SEQ ID NO:24030 |
| **iPS:442390** | **21-225_7E11.001.013** | NA | AGCTTTGGCATGCAC | ATTATCTGGCATGATGGAAGTAATAAATACTATGCAGAATCCGTGAAGGGC | GATCTGAGTATGGGCGGTATGGACGTC |
| | | | SEQ ID NO:8007 | SEQ ID NO:16019 | SEQ ID NO:24031 |
| | | AA | SFGMH | IIWHDGSNKYYAESVKG | DLSMGGMDV |
| | | | SEQ ID NO:8008 | SEQ ID NO:16020 | SEQ ID NO:24032 |
| **iPS:442394** | **21-225_7E11.001.014** | NA | AGCTTTGGCATGCAC | ATTATCTGGCATAGTGGAAGTAATAAATACTATGCAGAATCCGTGAAGGGC | GATCTGAGTATGGGCGGTATGGACGTC |
| | | | SEQ ID NO:8009 | SEQ ID NO:16021 | SEQ ID NO:24033 |
| | | AA | SFGMH | IIWHSGSNKYYAESVKG | DLSMGGMDV |
| | | | SEQ ID NO:8010 | SEQ ID NO:16022 | SEQ ID NO:24034 |
| **iPS:442398** | **21-225_7E11.001.015** | NA | AGCTTTGGCATGCAC | ATTATCTGGCATAGTGGAAGTAATAAATACTATGCAGACTCCGTGAAGGGC | GATCTGAGTATGGGCGGTATGGACGTC |
| | | | SEQ ID NO:8011 | SEQ ID NO:16023 | SEQ ID NO:24035 |
| | | AA | SFGMH | IIWHSGSNKYYADSVKG | DLSMGGMDV |
| | | | SEQ ID NO:8012 | SEQ ID NO:16024 | SEQ ID NO:24036 |

FIGURE 49

| | | | | | |
|---|---|---|---|---|---|
| **iPS:442402** | **21-225_7E11.001.016** | NA | AGCTTTGGCATGCAC | ATTATCTGGCATAGTGGAAGTAATAAATACTATGCAGAATCCGTGAAGGGC | GATCTGAGTATGGGCGGTATGGACGTC |
| | | | SEQ ID NO:8013 | SEQ ID NO:16025 | SEQ ID NO:24037 |
| | | AA | SFGMH | IIWHSGSNKYYAESVKG | DLSMGGMDV |
| | | | SEQ ID NO:8014 | SEQ ID NO:16026 | SEQ ID NO:24038 |
| **iPS:442406** | **21-225_7E11.001.017** | NA | AGCTTTGGCATGCAC | ATTATCTGGCATGAAGGAAGTAATAAATACTATGCAGACGCCGTGAAGGGC | GATCTGAGTATGGGCGGTATGGACGTC |
| | | | SEQ ID NO:8015 | SEQ ID NO:16027 | SEQ ID NO:24039 |
| | | AA | SFGMH | IIWHEGSNKYYADAVKG | DLSMGGMDV |
| | | | SEQ ID NO:8016 | SEQ ID NO:16028 | SEQ ID NO:24040 |
| **iPS:442410** | **21-225_7E11.001.018** | NA | AGCTTTGGCATGCAC | ATTATCTGGCATGATGCAAGTAATAAATACTATGCAGAATCCGTGAAGGGC | GATCTGAGTATGGGCGGTATGGACGTC |
| | | | SEQ ID NO:8017 | SEQ ID NO:16029 | SEQ ID NO:24041 |
| | | AA | SFGMH | IIWHDASNKYYAESVKG | DLSMGGMDV |
| | | | SEQ ID NO:8018 | SEQ ID NO:16030 | SEQ ID NO:24042 |
| **iPS:442417** | **21-225_7E11.001.019** | NA | AGCTTTGGCATGCAC | ATTATCTGGCATGATGGAAGTAATAAATACTATGCAGAATCCGTGAAGGGC | GATCTGAGTATGGGCGGTATGGACGTC |
| | | | SEQ ID NO:8019 | SEQ ID NO:16031 | SEQ ID NO:24043 |
| | | AA | SFGMH | IIWHDGSNKYYAESVKG | DLSMGGMDV |
| | | | SEQ ID NO:8020 | SEQ ID NO:16032 | SEQ ID NO:24044 |

FIGURE 49

| | | | | | |
|---|---|---|---|---|---|
| **iPS:442431** | **21-225_7E11.001.021** | NA | AGCTTTGGCATGCAC | ATTATCTGGCATAGTGGAAGTAATAAATACTATGCAGAATCCGTGAAGGGC | GATCTGAGTATGGGCGGTATGGACGTC |
| | | | SEQ ID NO:8021 | SEQ ID NO:16033 | SEQ ID NO:24045 |
| | | AA | SFGMH | IIWHSGSNKYYAESVKG | DLSMGGMDV |
| | | | SEQ ID NO:8022 | SEQ ID NO:16034 | SEQ ID NO:24046 |
| **iPS:442438** | **21-225_7E11.001.022** | NA | AGCTTTGGCATGCAC | ATTATCTGGCATGAAGGAAGTAATAAATACTATGCAGAATCCGTGAAGGGC | GATCTGAGTATGGGCGGTATGGACGTC |
| | | | SEQ ID NO:8023 | SEQ ID NO:16035 | SEQ ID NO:24047 |
| | | AA | SFGMH | IIWHEGSNKYYAESVKG | DLSMGGMDV |
| | | | SEQ ID NO:8024 | SEQ ID NO:16036 | SEQ ID NO:24048 |
| **iPS:442568** | **21-225_149D8** | NA | AACAGTGGTTACTACTGGAGC | TACAGCTATTACAGTGGGAGCACCTACTACAACCCGTCCCTCAAGAGT | GGGGGATATAACTGGAACCATGCTTTTGATATC |
| | | | SEQ ID NO:8025 | SEQ ID NO:16037 | SEQ ID NO:24049 |
| | | AA | NSGYYWS | YSYYSGSTYYNPSLKS | GGYNWNHAFDI |
| | | | SEQ ID NO:8026 | SEQ ID NO:16038 | SEQ ID NO:24050 |
| **iPS:443003** | **21-225_43F11_LC2** | NA | GGCTACTATATACAC | TGGATCAACCCTAACAGTGGTGGCACAAACTATGCACAGAAGTTTCAGGGC | GGAGGGAATTACTTCTACAACCACGTTATGGACGTC |
| | | | SEQ ID NO:8027 | SEQ ID NO:16039 | SEQ ID NO:24051 |
| | | AA | GYYIH | WINPNSGGTNYAQKFQG | GGNYFYNHVMDV |
| | | | SEQ ID NO:8028 | SEQ ID NO:16040 | SEQ ID NO:24052 |
| **iPS:443005** | **21-225_43F11_LC1** | NA | GGCTACTATATACAC | TGGATCAACCCTAACAGTGGTGGCACAAACTATGCACAGAAGTTTCAGGGC | GGAGGGAATTACTTCTACAACCACGTTATGGACGTC |
| | | | SEQ ID NO:8029 | SEQ ID NO:16041 | SEQ ID NO:24053 |

FIGURE 49

| | | | | | |
|---|---|---|---|---|---|
| | | AA | GYYIH | WINPNSGGTNYAQKFQG | GGNYFYNHVMDV |
| | | | SEQ ID NO:8030 | SEQ ID NO:16042 | SEQ ID NO:24054 |
| **iPS:443006** | **21-225_25A4.001.029** | NA | AATTATGATATTAAT | TGGATGTACCCTAACAGTGGTCAAACAGGCTATGCACAGAAATTCCAGGGC | AGCAGTGGCTGGTACTACTTTGACTAC |
| | | | SEQ ID NO:8031 | SEQ ID NO:16043 | SEQ ID NO:24055 |
| | | AA | NYDIN | WMYPNSGQTGYAQKFQG | SSGWYYFDY |
| | | | SEQ ID NO:8032 | SEQ ID NO:16044 | SEQ ID NO:24056 |
| **iPS:443016** | **21-225_4H6.014** | NA | GACTACTATTTGCAC | TGGATCCACCCTAACAGTGGTGGCACAAACTATGCACAGAAGTTTCAGGGC | GATGCTACCAGCTCGTTTGACTAC |
| | | | SEQ ID NO:8033 | SEQ ID NO:16045 | SEQ ID NO:24057 |
| | | AA | DYYLH | WIHPNSGGTNYAQKFQG | DATSSFDY |
| | | | SEQ ID NO:8034 | SEQ ID NO:16046 | SEQ ID NO:24058 |
| **iPS:443027** | **21-225_7E11.001.023** | NA | AGCTTTGGCATGCAC | ATTATCTGGCATGATGCAAGTAATAAATACTATGCAGACGCCGTGAAGGGC | GATCTGAGTATGGGCGGTATGGACGTC |
| | | | SEQ ID NO:8035 | SEQ ID NO:16047 | SEQ ID NO:24059 |
| | | AA | SFGMH | IIWHDASNKYYADAVKG | DLSMGGMDV |
| | | | SEQ ID NO:8036 | SEQ ID NO:16048 | SEQ ID NO:24060 |
| **iPS:446086** | **21-225_94D8** | NA | AATTATGATATCAAC | TGGATGAACCCTAACAGTGGTAACACAGGCTATGCACAGAAGTTCCAGGTC | AGCAGTGGCTGGTACATCTTTGACTAC |
| | | | SEQ ID NO:8037 | SEQ ID NO:16049 | SEQ ID NO:24061 |
| | | AA | NYDIN | WMNPNSGNTGYAQKFQV | SSGWYIFDY |

FIGURE 49

| | | | | | |
|---|---|---|---|---|---|
| | | | SEQ ID NO:8038 | SEQ ID NO:16050 | SEQ ID NO:24062 |
| **iPS:446094** | **21-225_77E1** | NA | AATTATGATATCAAC | TGGATGAACCCTAACAG TGGTAACACAGGCTATG CACAGAAGTTCCAGGGC | TCCAGTGGCTGGCACTGGTT CGACCCC |
| | | | SEQ ID NO:8039 | SEQ ID NO:16051 | SEQ ID NO:24063 |
| | | AA | NYDIN | WMNPNSGNTGYAQKFQG | SSGWHWFDP |
| | | | SEQ ID NO:8040 | SEQ ID NO:16052 | SEQ ID NO:24064 |
| **iPS:448904** | **21-225_65C12** | NA | AGCTTTAGCTTGAAC | TCCATTAGTAGTAGTAGT AGTTACATATACTACGC AGACTCAGTGAAGGGC | GATGCGTATAGCCACTAC |
| | | | SEQ ID NO:8041 | SEQ ID NO:16053 | SEQ ID NO:24065 |
| | | AA | SFSLN | SISSSSSYIYYADSVKG | DAYSHY |
| | | | SEQ ID NO:8042 | SEQ ID NO:16054 | SEQ ID NO:24066 |
| **iPS:448906** | **21-225_72G9** | NA | AGTTATAGCATGAAC | TCCATTAGTGGTAGTAGT AGTTACATATACTACGC AGACTCAGTGAAGGGC | GGGGGTTCGAGGGGGTTCGA CCCC |
| | | | SEQ ID NO:8043 | SEQ ID NO:16055 | SEQ ID NO:24067 |
| | | AA | SYSMN | SISGSSSYIYYADSVKG | GGSRGFDP |
| | | | SEQ ID NO:8044 | SEQ ID NO:16056 | SEQ ID NO:24068 |
| **iPS:448908** | **21-225_50G9** | NA | AGCTATGGCATGCAC | GTTATATCACAAGATGG AATTATTAGATACTATGC AGACTCCGTGAAGGGC | GATGTGAAGCAGTGGCTGGT ACGGACCTACGGTATGGACG TC |
| | | | SEQ ID NO:8045 | SEQ ID NO:16057 | SEQ ID NO:24069 |
| | | AA | SYGMH | VISQDGIIRYYADSVKG | DVKQWLVRTYGMDV |
| | | | SEQ ID NO:8046 | SEQ ID NO:16058 | SEQ ID NO:24070 |
| **iPS:451102** | **21 225 45F6** | NA | TACTATGGCTTGCAC | GTTATATCATATGATGGA AGTAATAAATATTATGC AGACTCCGTGAAGGGC | GAGGATCGATATTGTAGTGG TACCAGCTGCCCCTACTACT ACTACTACGGTATGGACGTC |

**FIGURE 49**

| | | | | | |
|---|---|---|---|---|---|
| | | | SEQ ID NO:8047 | SEQ ID NO:16059 | SEQ ID NO:24071 |
| | **21-225_45F6** | AA | YYGLH | VISYDGSNKYYADSVKG | EDRYCSGTSCPYYYYYGMDV |
| | | | SEQ ID NO:8048 | SEQ ID NO:16060 | SEQ ID NO:24072 |
| **iPS:451104** | **21-225_49C5** | NA | AGCTATGGTATCAGC | TGGATCAGCGCTTATAATGGTAACACAAAGTATGCACAGAAGCTCCAGGGC | CACGATTTTTGGAGTGGTTATTATAAGGGTATGGACGTC |
| | | | SEQ ID NO:8049 | SEQ ID NO:16061 | SEQ ID NO:24073 |
| | | AA | SYGIS | WISAYNGNTKYAQKLQG | HDFWSGYYKGMDV |
| | | | SEQ ID NO:8050 | SEQ ID NO:16062 | SEQ ID NO:24074 |
| **iPS:451106** | **21-225_49D10** | NA | AGCTATGGTATCAGC | TGGATCAGCGCTTATAATGGTAACACAAAGAATGCACAGAAGCTCCAGGGC | CACGATTTTTGGAGTGGTTATTATAAGGGTATGGACGTC |
| | | | SEQ ID NO:8051 | SEQ ID NO:16063 | SEQ ID NO:24075 |
| | | AA | SYGIS | WISAYNGNTKNAQKLQG | HDFWSGYYKGMDV |
| | | | SEQ ID NO:8052 | SEQ ID NO:16064 | SEQ ID NO:24076 |
| **iPS:451108** | **21-225_53E8** | NA | AGCTATGGTATCAGC | TGGATCAGCGCTTATAATGGTAACACAAAGTTTGCACAGAAGCTCCAGGGC | CACGATTTTTGGAGTGGTTATTATAAGGGTATGGACGTC |
| | | | SEQ ID NO:8053 | SEQ ID NO:16065 | SEQ ID NO:24077 |
| | | AA | SYGIS | WISAYNGNTKFAQKLQG | HDFWSGYYKGMDV |
| | | | SEQ ID NO:8054 | SEQ ID NO:16066 | SEQ ID NO:24078 |

**FIGURE 49**

| | | | | | |
|---|---|---|---|---|---|
| **iPS:451110** | **21-225_74C9** | NA | AGCTATGGCATGCAC | GTTATATGGTATGATGGAAATAATAAATCCTATGCAGACTCCGTGAAGGGC | GATCGAGATTATTGTAGTAGTACCAGCTGCCCTTATTATTACTACTACGGTATGGACGTC |
| | | | SEQ ID NO:8055 | SEQ ID NO:16067 | SEQ ID NO:24079 |
| | | AA | SYGMH | VIWYDGNNKSYADSVKG | DRDYCSSTSCPYYYYYGMDV |
| | | | SEQ ID NO:8056 | SEQ ID NO:16068 | SEQ ID NO:24080 |
| **iPS:451112** | **21-225_53D10** | NA | GGCTACTATATACAC | TGGATCAACCCTAACAGTGGTGGCACAAACTATGCACAGAAGTTTCAGGGC | GAAAACGAAAGTCTAGCAACTCGTCCTTTCTACGACTACTACGGTATGGACGTC |
| | | | SEQ ID NO:8057 | SEQ ID NO:16069 | SEQ ID NO:24081 |
| | | AA | GYYIH | WINPNSGGTNYAQKFQG | ENESLATRPFYDYYGMDV |
| | | | SEQ ID NO:8058 | SEQ ID NO:16070 | SEQ ID NO:24082 |
| **iPS:451114** | **21-225_159A3** | NA | GACTATGTCATGCAG | GTTATATGGTATGATGGAAGTAATAAATACTATGCAGACTCCGTGAAGGGC | GAACCGTATAATAGTGGCTGGTACGACTACGGTATGGACGTC |
| | | | SEQ ID NO:8059 | SEQ ID NO:16071 | SEQ ID NO:24083 |
| | | AA | DYVMQ | VIWYDGSNKYYADSVKG | EPYNSGWYDYGMDV |
| | | | SEQ ID NO:8060 | SEQ ID NO:16072 | SEQ ID NO:24084 |
| **iPS:451116** | **21-225_164A4** | NA | AATTATGATATCAAC | TGGATGCACCCTAACAGTGGTAACACAGGCTATGCACAGAAGTTCCAGGGC | AGCAGTGGCTGGTACTTCTTTGACTAC |
| | | | SEQ ID NO:8061 | SEQ ID NO:16073 | SEQ ID NO:24085 |
| | | AA | NYDIN | WMHPNSGNTGYAQKFQG | SSGWYFFDY |
| | | | SEQ ID NO:8062 | SEQ ID NO:16074 | SEQ ID NO:24086 |

## FIGURE 49

| | | | | | |
|---|---|---|---|---|---|
| **iPS:451118** | **21-225_191C8** | NA | AGTGGTGGTTACTACTGGAGC | TATATCTATTACAGTGGGACCACCATTTACAACCCCTCCCTCAAGAGT | GACACGTTTTGCTTTGATGGTTGTGGTTATTTCTTTGACTCC |
| | | | SEQ ID NO:8063 | SEQ ID NO:16075 | SEQ ID NO:24087 |
| | | AA | SGGYYWS | YIYYSGTTIYNPSLKS | DTFCFDGCGYFFDS |
| | | | SEQ ID NO:8064 | SEQ ID NO:16076 | SEQ ID NO:24088 |
| **iPS:451120** | **21-225_197D3** | NA | AGCCATGGCATGCAC | GTTATATGGTATGATGGAAGTAATGAACACTATGCAGACTCCGTGAAGGGC | GATCAAGGTGTGGGGTACTACGGTATGGACGTC |
| | | | SEQ ID NO:8065 | SEQ ID NO:16077 | SEQ ID NO:24089 |
| | | AA | SHGMH | VIWYDGSNEHYADSVKG | DQGVGYYGMDV |
| | | | SEQ ID NO:8066 | SEQ ID NO:16078 | SEQ ID NO:24090 |
| **iPS:451122** | **21-225_200A1** | NA | GTTTACTATTGGAGC | GAAATCAATCATAGTGGAAGCACCAACTACAACCCGTCCCTCAAGAGT | GACTACGGTGTCTTTGACTAC |
| | | | SEQ ID NO:8067 | SEQ ID NO:16079 | SEQ ID NO:24091 |
| | | AA | VYYWS | EINHSGSTNYNPSLKS | DYGVFDY |
| | | | SEQ ID NO:8068 | SEQ ID NO:16080 | SEQ ID NO:24092 |
| **iPS:451124** | **21-225_74F6** | NA | AATTATGATATCAAC | TGGATGCACCCTAACAGTGGTAACACAGGCTATGCACAGAAGTTCCAGGGC | AGCAGTGGCTGGTACTTTTTTGACTAC |
| | | | SEQ ID NO:8069 | SEQ ID NO:16081 | SEQ ID NO:24093 |
| | | AA | NYDIN | WMHPNSGNTGYAQKFQG | SSGWYFFDY |
| | | | SEQ ID NO:8070 | SEQ ID NO:16082 | SEQ ID NO:24094 |
| **iPS:451127** | | NA | AATTATGATGTCAAC | TGGATGCACCCTAACAGTGGTAACACAGGCTATGCACAGAAGTTCCAGGGC | AGCAGTGGCTGGTACCTCTTTGACTAC |

FIGURE 49

| | | | | | |
|---|---|---|---|---|---|
| | 21-225_164A7 | | SEQ ID NO:8071 | SEQ ID NO:16083 | SEQ ID NO:24095 |
| | | AA | NYDVN | WMHPNSGNTGYAQKFQG | SSGWYLFDY |
| | | | SEQ ID NO:8072 | SEQ ID NO:16084 | SEQ ID NO:24096 |
| **iPS:451129** | | NA | AATTATGATATCAAC | TGGATGCACCCTAACAG TGGTAACACAGGCTTTG CACAGAAGTTCCAGGGC | TCCAGTGGCTGGTACTGGTT CGACCCC |
| | 21-225_94D2 | | SEQ ID NO:8073 | SEQ ID NO:16085 | SEQ ID NO:24097 |
| | | AA | NYDIN | WMHPNSGNTGFAQKFQG | SSGWYWFDP |
| | | | SEQ ID NO:8074 | SEQ ID NO:16086 | SEQ ID NO:24098 |
| **iPS:451131** | | NA | AATTATGATATCAAC | TGGATGCACCCTCACAG TGGTAACACAGGCTATG CACAGAAGTTCCAGGGC | AGCAGTGGCTGGTACTACTT TGACTAC |
| | 21-225_160A7 | | SEQ ID NO:8075 | SEQ ID NO:16087 | SEQ ID NO:24099 |
| | | AA | NYDIN | WMHPHSGNTGYAQKFQG | SSGWYYFDY |
| | | | SEQ ID NO:8076 | SEQ ID NO:16088 | SEQ ID NO:24100 |
| **iPS:451133** | | NA | AATTATGATATCAAC | TGGATGAACCCTAACAG TGGTAACACAGGCTATG CACAGAAGTTCCAGGGC | TCCAGTGGCTGGAACTGGTT CGACCCC |
| | 21-225_95H4 | | SEQ ID NO:8077 | SEQ ID NO:16089 | SEQ ID NO:24101 |
| | | AA | NYDIN | WMNPNSGNTGYAQKFQG | SSGWNWFDP |
| | | | SEQ ID NO:8078 | SEQ ID NO:16090 | SEQ ID NO:24102 |
| **iPS:437240** | | NA | AGTTATGATATCAAC | TGGCTGAACCCTCACAG TGGTAACACAGGCTATG CACAGAAGTTCCAGGGC | GGGTTTTACGATATTTTGACT GGTTATTCCCCCACCTACTA CTACTACGATATGGACGTC |
| | 21-225_84H12 | | SEQ ID NO:8079 | SEQ ID NO:16091 | SEQ ID NO:24103 |

FIGURE 49

| | | | | | |
|---|---|---|---|---|---|
| | | AA | SYDIN | WLNPHSGNTGYAQKFQG | GFYDILTGYSPTYYYYDMDV |
| | | | SEQ ID NO:8080 | SEQ ID NO:16092 | SEQ ID NO:24104 |
| **iPS:434577** | **21-225_75C11** | NA | AGTTATGATATCAAC | TGGCTGAACCCTCACAG TGGTAACACAGGCTATG CACAGAAGTTCCAGGGC | GGGTTTTACGATATTTTGACT GGTTATTCCCCCACCTACTA CTACTACGATATGGACGTC |
| | | | SEQ ID NO:8081 | SEQ ID NO:16093 | SEQ ID NO:24105 |
| | | AA | SYDIN | WLNPHSGNTGYAQKFQG | GFYDILTGYSPTYYYYDMDV |
| | | | SEQ ID NO:8082 | SEQ ID NO:16094 | SEQ ID NO:24106 |
| **iPS:435477** | **21-225_154E8** | NA | AGCTATGCCATGAGC | GCTATTAGTGGTAGTGGT GGTAACACATTCTACGC AGACTCCGTGAAGGGC | CACGGTATAGCAGTGGCTGG TACTGGGGCTCACTACTTTG ACTAC |
| | | | SEQ ID NO:8083 | SEQ ID NO:16095 | SEQ ID NO:24107 |
| | | AA | SYAMS | AISGSGGNTFYADSVKG | HGIAVAGTGAHYFDY |
| | | | SEQ ID NO:8084 | SEQ ID NO:16096 | SEQ ID NO:24108 |
| **iPS:434553** | **21-225_76H12** | NA | AGTTATGATATCAAC | TGGCTGAACCCTCACAG TGGTAACACAGGCTATG CACAGAAGTTCCAGGGC | GGGTTTTACGATATTTTGACT GGTTATTCCCCCACCTACTA CTACTACGATATGGACGTC |
| | | | SEQ ID NO:8085 | SEQ ID NO:16097 | SEQ ID NO:24109 |
| | | AA | SYDIN | WLNPHSGNTGYAQKFQG | GFYDILTGYSPTYYYYDMDV |
| | | | SEQ ID NO:8086 | SEQ ID NO:16098 | SEQ ID NO:24110 |
| **iPS:434927** | **21-225_86E5** | NA | AGTTATGATATCAAC | TGGATGAACCCTAACAG TGGTAACACAGGCTATG CACAGAAGTTCCAGGGC | GGGTTTTACGATATTTTGACT GGTTATTCCCCCACCTACTA CTACTACGATATGGACGTC |
| | | | SEQ ID NO:8087 | SEQ ID NO:16099 | SEQ ID NO:24111 |

FIGURE 49

| | | | | | |
|---|---|---|---|---|---|
| | | AA | SYDIN | WMNPNSGNTGYAQKFQG | GFYDILTGYSPTYYYYDMDV |
| | | | SEQ ID NO:8088 | SEQ ID NO:16100 | SEQ ID NO:24112 |
| **iPS:435385** | **21-225_149G7** | NA | AGCTATGCCATGAGC | GCTATTAGTGGTAGTGGTGGTAACACATTCTACGCAGACTCCGTGAAGGGC | CACGGTATAGCAGTGGCTGGTACTGGGGCTCACTACTTTGACTAC |
| | | | SEQ ID NO:8089 | SEQ ID NO:16101 | SEQ ID NO:24113 |
| | | AA | SYAMS | AISGSGGNTFYADSVKG | HGIAVAGTGAHYFDY |
| | | | SEQ ID NO:8090 | SEQ ID NO:16102 | SEQ ID NO:24114 |

FIGURE 50

**Table 3**
**Standard IgG Antibody Variable Region Sequences**

| iPS# | Ab | Type | LC V-region | HC V-region |
|---|---|---|---|---|
| **iPS:451135** | **21-225_64A11** | NA | GACATCCAGATGACCCAGTCTCCATTCTCCCTGTCTGCATCTGTAGGCGACAGAGTCACCATCACTTGCCGGGCAAGTCGGAGCGTTAGCAGATATTTAAATTGGTATCAGCAGACACTGGGGAAAGCCCTTAAGCTCTTGATATCTGTTGCATCCCGTTTGCAAAGTGGGGTCCCATCGAGGTTCAGTGGCAGTGGATCTGGGACAGATTTCACTCTCACCATCAGCAGTGTGCAACGTGAAGATTTTGCAACTTACTTCTGTCAACAGAGTGACAGTTTCCCTCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAACTATGGCATGCACTGGGTCCGCCAGGCTCCAGGCAAGGGGCTGGAGTGGGTGGCAGTTATATCATATGTTGGAAGTAATAAATACTATGCAGACTCCGTGAAGGGCCGATTCACCATCTCCAGAGACAATTCCAAGAACACGCTGTATCTGCAAATGAACACCCTGAGAGCTGAGGACACGGCTGTGTATTACTGTGCGAGGAGAGGAGCAGTGGCTCCGTACTACGGTATGGACGTCTGGGGCCAAGGGACCACGGTCACCGTCTCCTCA |
| | | | SEQ ID NO: 24115 | SEQ ID NO: 28121 |
| | | AA | DIQMTQSPFSLSASVGDRVTITCRASRSVSRYLNWYQQTLGKALKLLISVASRLQSGVPSRFSGSGSGTDFTLTISSVQREDFATYFCQQSDSFPLTFGGGTKVEIK | QVQLVESGGGVVQPGRSLRLSCAASGFTFSNYGMHWVRQAPGKGLEWVAVISYVGSNKYYADSVKGRFTISRDNSKNTLYLQMNTLRAEDTAVYYCARRGAVAPYYGMDVWGQGTTVTVSS |
| | | | SEQ ID NO: 24116 | SEQ ID NO: 28122 |

FIGURE 50

| | | | | |
|---|---|---|---|---|
| **iPS:451141** | **21-225_164B11** | NA | GACATCGTGATGACCCAGTCTCCAGACTCCCTGGCTGTGTCTCTGGGCGAGAGGGCCACCATCAACTGCAAGTCCAGCCAGAGTCTTTTAAAGAGCTCCAACAATAAGAGCTACTTAGCTTCGTACCAGCAGAAGCCAGGACAGCTTCCTAAACTGCTCATTTACTGGGCATCTTCCCGGGAATCCGGGGTCCCTGACCGATTCAGTGGCAGCGGGTCTGGGACAGATTTCACTCTCACCATCAGCAGCCTGCAGGCTGAAGATGTGGCACTTTATTACTGTCAGCAATATTATAGTATTCCTCCCACTTTCGGCCATGGGACCAATGTGGATATCACG | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGACCCCTAACAGTGGTAACACAGGCTATGCACAGAAGTTCCAGGGCAGAGTCACCATGACCAGGAACACTTCCATGAGCACCGCCTACATGGAGCTGAGCAGCCTGAGATCTGAGGACTCGGCCGTGTATTACTGTTCCTATAGCAGTGGCTGGTACATGTTTGACTACTGGGGCCAGGGAACCCTGGTCACCGTCTCCTCA |
| | | | SEQ ID NO: 24117 | SEQ ID NO: 28123 |
| | | AA | DIVMTQSPDSLAVSLGERATINCKSSQSLLKSSNNKSYLASYQQKPGQLPKLLIYWASSRESGVPDRFSGSGSGTDFTLTISSLQAEDVALYYCQQYYSIPPTFGHGTNVDIT | QVQLVQSGAEVKKPGASVKVSCKASGYTFTNYDINWVRQATGQGLEWMGWMTPNSGNTGYAQKFQGRVTMTRNTSMSTAYMELSSLRSEDSAVYYCSYSSGWYMFDYWGQGTLVTVSS |
| | | | SEQ ID NO: 24118 | SEQ ID NO: 28124 |
| **iPS:451137** | **21-225_74A7** | NA | GACATCGTTATGACCCAGTCTCCAGACTCCCTGGCTGTGTCTCTGGGCGAGAGGGCCACCATCAACTGCAAGTCCAGCCAGAGTGTTTTATTCAGCTCCAACAATTATAACTACTTAGCTTGGTACCAGCAGAGACCAGGACAGCCTCCTAACCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGCTGATGTGGCAGTTTATTACTGTCAGCAATATCATAGTTCTCCTCCGACGTTCGGCCAAGGGACCACGGTGCAAATCAAA | CAGGTGCAGTTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCACAAGCACAGCCCACATGGAGCTGAGCAGCCTGAGATCTGAGGACACGGCCGTGTATTACTGTGCGGTCTCCAGTGGCTGGAACTGGTTCGACCCCTGGGGCCAGGGAACCCTGGTCACCGTCTCCTCA |
| | | | SEQ ID NO: 24119 | SEQ ID NO: 28125 |

FIGURE 50

| | | | | |
|---|---|---|---|---|
| | | AA | DIVMTQSPDSLAVSLGERATINCKSSQSVLFSSNNYNYLAWYQQRPGQPPNLLIYWASTRESGVPDRFSGSGSGTDFTLTISSLQAADVAVYYCQQYHSSPPTFGQGTTVQIK | QVQLVQSGAEVKKPGASVKVSCKASGYTFTNYDINWVRQATGQGLEWMGWMNPNSGNTGYAQKFQGRVTMTRNTSTSTAHMELSSLRSEDTAVYYCAVSSGWNWFDPWGQGTLVTVSS |
| | | | SEQ ID NO: 24120 | SEQ ID NO: 28126 |
| iPS:451139 | 21-225_71A6 | NA | GATATTGTGATGACCCAGACTCCACTCTCTCTCTCTGTCACACCTGGACAGCCGGCCTCCATCTCCTGCAAGTCTAGTCAGAGCCTCCTGCGTAGTGATGGAAAGACCCATTTGTATTGGTACCTGCAGAAGCCAGGCCAGCCTCCACAGCTCTTAATCTATGAAGTTTCCAACCGGTTCTCTGGAGTGTCAGATAGGTTCAGTGGCAGCGGGTCAGGGACAGATTTCACACTGAAAATCAGCCGGGTGGAGGCTGAGGATGTTGGGGTTTATTACTGCATGCAAAGTAAACAGCTTCCTCTCACTTTCGGCGGAGGGACCAAGGTGGAGTTCAAA | CAGGTGCAACTGGTGGAGTCTGGGGGAGGCGTGGTCCAGCCTGGGAGGTCCCTGAGACTCTCCTGTGCAGCCTCTGGATTCACCTTCAGTAACTATGGCATGCACTGGGTCCGCCAGGCTCCAGGCAAGGGGCTGGAGTGGGTGGCAGTTATATCATATGATGGAAGTAATGAATACTATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTACTGTGCGAGAGATCACAGATATGGGGTTCGGGGAGGCTTTGACTACTGGGGCCAGGGAACCCTGGTCACCGTCTCCTCA |
| | | | SEQ ID NO: 24121 | SEQ ID NO: 28127 |
| | | AA | DIVMTQTPLSLSVTPGQPASISCKSSQSLLRSDGKTHLYWYLQKPGQPPQLLIYEVSNRFSGVSDRFSGSGSGTDFTLKISRVEAEDVGVYYCMQSKQLPLTFGGGTKVEFK | QVQLVESGGGVVQPGRSLRLSCAASGFTFSNYGMHWVRQAPGKGLEWVAVISYDGSNEYYADSVKGRFTISRDNSKNTLYLQMNSLRAEDTAVYYCARDHRYGVRGGFDYWGQGTLVTVSS |
| | | | SEQ ID NO: 24122 | SEQ ID NO: 28128 |

FIGURE 50

| | | | | |
|---|---|---|---|---|
| **iPS:451143** | **21-225_66H11** | NA | GACATCCAGATGACCCAGTTTCCATCCTCACTGTTTGCATTTGTAGGAGACAGAGTCACCATCACTTGTCCGGCGAGTCAGGGCATTAGCAATTATTTAGCTTGGTTTCAGCAGAAACCAGGGAAAGCCCCTAAGTCCCTTATTTATGGTGCATTCAATTTGCACAGTGGGGTCCCATCAAAGTTCAGCGGCAGTGGATTTGGGACAGATTTCACTCTCACCATCAACAGCCTGCAGCCTGAAGATTTTGCAAATTATTACTGCCAACAGTATAGTTGTTACCCATTCACTTTCGGCCATGGGACCAAAGTGGATATCAAA | CAGGTTCAGCTGGTCCAGTCTGGAGCTGAGGTGAAGAAGCCTGGGGCCTCAGTGAAGGTCTCCTGCAAGGCTTCTGGTTACACCTTTGCCACCTATGGTATCAGCTGGGTGCGACAGGCCCCTGGACAGGGGCTTGAGTGGATGGGATGGATCAGCGCTTACAATGGTAACACAAACTATGCACAGAAGCTCCAGGGCAGAGTCACCATGACCACAGACACATCCACGAGCACAGCCTACATGGAGCTGAGGAGCCTGAGATCTGACGACACGGCCGTGTATTACTGTGCGAGAGGGGAAGCAGTGGCTGTCTTCGACCCCTGGGGCCAGGGAACCCTGGTCACCGTCTCCTCA |
| | | | SEQ ID NO: 24123 | SEQ ID NO: 28129 |
| | | AA | DIQMTQFPSSLFAFVGDRVTITCPASQGISNYLAWFQQKPGKAPKSLIYGAFNLHSGVPSKFSGSGFGTDFTLTINSLQPEDFANYYCQQYSCYPFTFGHGTKVDIK | QVQLVQSGAEVKKPGASVKVSCKASGYTFATYGISWVRQAPGQGLEWMGWISAYNGNTNYAQKLQGRVTMTTDTSTSTAYMELRSLRSDDTAVYYCARGEAVAVFDPWGQGTLVTVSS |
| | | | SEQ ID NO: 24124 | SEQ ID NO: 28130 |
| **iPS:453445** | **21-225_148E10** | NA | TCCTATGAGCTGACTCAGCCACCCTCAGTGTCCGTGTCCCCAGGACAGACAGCCAGCATCACCTGCTCTGGAGATAAATTGGGTAATAAATATGTTTGTTGGTATCAGCAGAGGCCAGGCCATGCTGCTGTGCTGATCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTTCTGTCAGGCGTGGGACAGGAACACTTATGTGGTGTTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTTTGATGGCAGTAATAAATACTATGTAGACTCCGTGAAGGACCGATTCACCATCTCCAGAGACAATTCCAAGAACACGCTGTATCTGCAAATGAACAGCCTGAGAGCCGAGGACACGGCTGTTTATTACTGTGCGAGAGATCGGGTGGAGGGTTCGGGGACTCCCTACTACTACTACGGTATGGACGTCTGGGGCCAAGGGACCACGGTCACCGTCTCCTCA |
| | | | SEQ ID NO: 24125 | SEQ ID NO: 28131 |

FIGURE 50

| | | | | |
|---|---|---|---|---|
| | | AA | SYELTQPPSVSVSPGQTASITCSGDKLGNKYVCWYQQRPGHAAVLIIYQDSKRPSGIPERFSGSNSGNTATLTISGTQAMDEADYFCQAWDRNTYVVFGGGTKLTVL | QVQLVESGGGVVQPGRSLRLSCAASGFTFSSYGMHWVRQAPGKGLEWVAVIWFDGSNKYYVDSVKDRFTISRDNSKNTLYLQMNSLRAEDTAVYYCARDRVEGSGTPYYYYGMDVWGQGTTVTVSS |
| | | | SEQ ID NO: 24126 | SEQ ID NO: 28132 |
| iPS:453447 | 21-225_65F10 | NA | GACATCCAGATGACCCAGTCTCCATCTTCCGTGTCTGCATCTGTAGGAGACAGAGTCACCATCACTTGTCGGGGGGGTCAGGGTATTAGCACATGGTTAGCATGGTATCAGCAGAAACCAGGGAAAGCCCCTAAGCTCCTGATCTATGCTGCATCCATTTTGCAAAGTGGGGTCCCATCAAGGTTCAGAGGCAGGGGATCTGGGACAGATTTCACTCTCACCATCAGCAGCCTGCAGCCTGAAGATTTTGCAACTTATTATTGTCAACAGGGTAACATTTTCCCATTCACTTTCGGCCGAGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACCATATGCACTGGGTGCGACAGGCCCCTGGACAAGGGCTTGAGTGGATGGGATGGATCAACCCTAACAATGGTGGCACAAGCTATGCACAGAAGTTTCAGGACAGGGTCAACATGACCAGGGACACGTCCATCAGCACAGCCTACATGGAGCTGAGCAGGCTGAGATCTGACGACACGGCCGTGTACTACTGTGCGAGAGATAGTAGGTCGTCCTGGGACTACTGGGGCCAGGGAACCCTGGTCACCGTCTCCTCA |
| | | | SEQ ID NO: 24127 | SEQ ID NO: 28133 |
| | | AA | DIQMTQSPSSVSASVGDRVTITCRGGQGISTWLAWYQQKPGKAPKLLIYAASILQSGVPSRFRGRGSGTDFTLTISSLQPEDFATYYCQQGNIFPFTFGRGTKVDIK | QVQLVQSGAEVKKPGASVKVSCKASGYTFTGYHMHWVRQAPGQGLEWMGWINPNNGGTSYAQKFQDRVNMTRDTSISTAYMELSRLRSDDTAVYYCARDSRSSWDYWGQGTLVTVSS |
| | | | SEQ ID NO: 24128 | SEQ ID NO: 28134 |

FIGURE 50

| | | | | |
|---|---|---|---|---|
| **iPS:453449** | **21-225_208A2** | NA | GACATCCAGATGACCCAGTCTCCATCCTCCCTGTCTGCATCTGTAGGAGACAGAGTCACCATCACTTGCCGGACAAGTCAGGGCATTAGAAATGATTTAGGCTGGTATCAGCAGCAACCAGGGAAAACCCCTAAGCGCCTGATCTATGCTGCATCCAGTTTGCTTAGTGGGGTCCCATCAAGGTTCAGCGGCAGTAGATCTGGGACAGATTTCACTCTCACAATCAGCAGCCTGCAGCCTGAAGATTTTGCAACTTATTACTGTCTACAGTATAATAGTTACCCTCCCACCTTCGGCCAAGGGACACGACTGGAGATTAAA | CAGGTGCAGCTGCAGGAGTCGGGCCCAGGACTGGTGAAGCCTTCGGAGACCCTGTCCCTCAACTGCACTGTCTCTGGTGGCTCCATCAGGAGTTACTACTGGAGCTGGATCCGGCAGCCCGCCGGGAAGGGACTGGAGTGGATTGGGCGTATCTATACCAGTGGGAGCACCGACTACAACCCCTCCCTCAAGAGTCGAATCACCATGTCAGTAGACACGTCCAAGAACCAGTTCTCTTTGAAGCTGAGCTCTGTGACCGCCGCGGACACGGCCGTGTATTACTGTGCGAGAGGGTTCGGTGACTGGGACTACTGGGGCCAGGGAACCCTGGTCACCGTCTCCTCA |
| | | | SEQ ID NO: 24129 | SEQ ID NO: 28135 |
| | | AA | DIQMTQSPSSLSASVGDRVTITCRTSQGIRNDLGWYQQQPGKTPKRLIYAASSLLSGVPSRFSGSRSGTDFTLTISSLQPEDFATYYCLQYNSYPPTFGQGTRLEIK | QVQLQESGPGLVKPSETLSLNCTVSGGSIRSYYWSWIRQPAGKGLEWIGRIYTSGSTDYNPSLKSRITMSVDTSKNQFSLKLSSVTAADTAVYYCARGFGDWDYWGQGTLVTVSS |
| | | | SEQ ID NO: 24130 | SEQ ID NO: 28136 |
| **iPS:453451** | **21-225_52G11** | NA | GACATCCAGATGACCCAGTCTCCATCTTCCGTGTCTGCATCTGTAGGAGACAGAGTCACCATCACTTGTCGGGCGAGTCAGGGTATTAGCAAATGGTTAGCCTGGTATCAGCAGAAACCAGGGAAAGCCCCTAAGCTCCTGCTCTATGCTGCATCCAGTTTGCAAAGTGGGGTCCCATCAAGATTCAGCGCCAGTGGATCTGGGACAGATTTCACTCTCACCATCAGCAGTTTGCAGCCTGAAGATTTTGCAACTTACTATTGTCAACAGGCTAACAGTTTCCCATTCACTTTCGGCCCTGGGACCAAAGTGGATGTCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATTTGCACTGGGTGCGACAGGCCCCTGGACAAGGGCTTGAGTGGATGGGATGGATCAACCCTAATAGAAATGGCACAAACTATGCACAGAAGTTTCAGGGCAGGGTCACCATGACCAGGGACACGTCCATCAGCACAGCCTTCATGGAGCTGAGCAGGCTGAGATCTGACGACACGGCCGTGTATTACTGTGCGAGAGACGGTACCAGCAGCTTTGACTACTGGGGCCAGGGAACCCTGGTCACCGTCTCCTCA |
| | | | SEQ ID NO: 24131 | SEQ ID NO: 28137 |

FIGURE 50

| | | | | |
|---|---|---|---|---|
| | | AA | DIQMTQSPSSVSASVGDRVTITCRASQGISKWLAWYQQKPGKAPKLLLYAASSLQSGVPSRFSASGSGTDFTLTISSLQPEDFATYYCQQANSFPFTFGPGTKVDVK | QVQLVQSGAEVKKPGASVKVSCKASGYTFTGYYLHWVRQAPGQGLEWMGWINPNRNGTNYAQKFQGRVTMTRDTSISTAFMELSRLRSDDTAVYYCARDGTSSFDYWGQGTLVTVSS |
| | | | SEQ ID NO: 24132 | SEQ ID NO: 28138 |
| iPS:453453 | 21-225_53F2 | NA | GACATCCAGATGACCCAGTCTCCATCTTCCGTGTCTGCATCTGTAGGAGACAGAGTCACCATCACTTGTCGGGCGAGTCAGGGTATTAGCAAGTGGTTAGCCTGGTATCAGCAGAAACCAGGGAAAGCCCCTAACCTCCTTCTCTATGCTGCATCCAGTTTGCAAAGTGGGGTCCCATCAAAATTCAGCGGCAGTGGATCTGGGACAGATTTCACTCTCACCATCAGCAGCCTGCAGCCTGAAGATTTTGCAACTTACTATTGTCAACAGGCTAACAGTTTCCCATTCACTTTCGGCCCTGGGACCAAAGTGGATGTCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATTTGCACTGGGTGCGACAGGCCCCTGGACAAGGGCTTGAGTGGATGGGATGGATCAACCCTAACAGAAATGGCACAAACTATGCACAGAATTTTCAGGGCAGGGTCACCATGACCAGGGACACGTCCATCAGCACAGCCTACATGGAGCTGAGCAGACTGAAATCTGACGACACGGCCGTGTATTACTGTGCGAGAGACGGTACCAGTAGCTTTGACTACTGGGGCCAGGGAACCCTGGTCACCGTCTCCTCA |
| | | | SEQ ID NO: 24133 | SEQ ID NO: 28139 |
| | | AA | DIQMTQSPSSVSASVGDRVTITCRASQGISKWLAWYQQKPGKAPNLLLYAASSLQSGVPSKFSGSGSGTDFTLTISSLQPEDFATYYCQQANSFPFTFGPGTKVDVK | QVQLVQSGAEVKKPGASVKVSCKASGYTFTGYYLHWVRQAPGQGLEWMGWINPNRNGTNYAQNFQGRVTMTRDTSISTAYMELSRLKSDDTAVYYCARDGTSSFDYWGQGTLVTVSS |
| | | | SEQ ID NO: 24134 | SEQ ID NO: 28140 |

FIGURE 50

| | | | | |
|---|---|---|---|---|
| **iPS:468810** | **21-225_74D5** | NA | GAAATTGTGTTGACGCAGTCTCCAGGCACCCT<br>GTCTTTGTCTCCAGGGGAGAGAGCCACCCTCT<br>CCTGCAGGGCCAGTCAGAGTGTTAACAGCAAC<br>TACTTAGCCTGGTACCGGCAGAAACCTGGCCA<br>GGCTCCCAGGCTCCTCATCTATGGTGCATCCA<br>GCAGGGCCACTGGCATCCCAGACAGGTTCAGT<br>GGCAGTGGGTCTGGGACAGACTTCATTCTCAT<br>CATCAGCAGACTGGAGCCTGAGGATTTTGCAG<br>TGTATTACTGTCAGCAGTATGAAAGCTCGCCG<br>TGGACGTTCGGCCAAGGGACCAAGGTGGAAA<br>TCAAA | CAGGTGCAGCTACAACAGTGGGGCGCAGGACTG<br>TTGAAGCCTTCGGAGACCCTGTCCCTCACTTGCG<br>CTGTCAATGGTGGGCCCTTCAGCGGTTGCTACTG<br>GAGCTGGATCCGCCAGCCCCCAGGGAAGGGGCT<br>GGAGTGGATTGGGGAAATCAATTATAGTGGAAG<br>GACCAACTTCAACCCGTCCCTCAAGAGTCGAGTC<br>ACCATATCAGTTGACACGTCCAAGAACCAGTTCT<br>CCCTGAAGCTGAGCTCTGTGACCGCCGCGGACAC<br>GGCTGTGTATTACTGTGCGAGAGACTACGGCGGT<br>ATGGACGTCTGGGGCCAAGGGACCACGGTCACC<br>GTCTCCTCA |
| | | | SEQ ID NO: 24135 | SEQ ID NO: 28141 |
| | | AA | EIVLTQSPGTLSLSPGERATLSCRASQSVNSNYLA<br>WYRQKPGQAPRLLIYGASSRATGIPDRFSGSGSG<br>TDFILIISRLEPEDFAVYYCQQYESSPWTFGQGTK<br>VEIK | QVQLQQWGAGLLKPSETLSLTCAVNGGPFSGCYW<br>SWIRQPPGKGLEWIGEINYSGRTNFNPSLKSRVTISV<br>DTSKNQFSLKLSSVTAADTAVYYCARDYGGMDV<br>WGQGTTVTVSS |
| | | | SEQ ID NO: 24136 | SEQ ID NO: 28142 |
| **iPS:468812** | **21-225_48H4** | NA | GACATCCAGATGACCCAGTTTCCATCCTCCCT<br>GTCTGCATCTGTAGGAGACAGAGTCACCATCA<br>CTTGCCGGGCAAGTCGAGACATTAGAAATGAT<br>TTAGGCTGGTATCAGCAGAAACCAGGAAAAG<br>CCCCTAAGCGCCTGATCTATGCTGCATCCAGT<br>TTGCAAAGTGGGGTCCCATCAAGGTTCAGCGG<br>CAGTGGATCTGGGACAGAATTCACTCTCACAA<br>TCAGCAGCCTGCAGCCTGAAGATTTTGCAACT<br>TATTACTGTCTACAACATTATAGTTACCCGCTC<br>ACTTTCGGCGGAGGGACCAAGGTGGAGATCA<br>AA | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTGACTCTCTCAT<br>GCACTGGGTCCGCCAGGCTCCAGGCAAGGGGCT<br>GGAGTGGGTGGCAGTTATATGGTATGATGGAAGT<br>AATAAATACTATACAGACTCCGTTAAGGGCCGAT<br>TCACCATCTCCAGAGACAATTCCAAGAACACGCT<br>GTATCTGCAAATGAACAGCCTGAGAGCCGAGGA<br>CACGGCTGTGTATTACTGTGCGAGAGAAAACTAT<br>AGCAGTGGCTGGTACGGGTACGGTATGGACGTCT<br>GGGGCCAAGGGACCACGGTCACCGTCTCCTCA |
| | | | SEQ ID NO: 24137 | SEQ ID NO: 28143 |

FIGURE 50

| | | | | |
|---|---|---|---|---|
| | | AA | DIQMTQFPSSLSASVGDRVTITCRASRDIRNDLG WYQQKPGKAPKRLIYAASSLQSGVPSRFSGSGS GTEFTLTISSLQPEDFATYYCLQHYSYPLTFGGG TKVEIK | QVQLVESGGGVVQPGRSLRLSCAASGFTFSDSLMH WVRQAPGKGLEWVAVIWYDGSNKYYTDSVKGRF TISRDNSKNTLYLQMNSLRAEDTAVYYCARENYSS GWYGYGMDVWGQGTTVTVSS |
| | | | SEQ ID NO: 24138 | SEQ ID NO: 28144 |
| **iPS:468816** | **21-225_52G8** | NA | GATATTGTGATGACCCAGACTCCACTCTCTCT GTCCGTCACCCCTGGACAGCCGGCCTCCATGT CCTGCAAGTCTAGTCAGAGCCTCCTGCATAGT GAAGGAAAGACCTATTTGTATTGGTACCTGCA GAAGACAGGCCAGCCTCCACACCTCCTGATCT ATGAAGTTTCCAAGCGGCTCTCTGGCGTGCCA GATAGGTTCAGTGGCAGCGGGTCAGGGACAG ATTTCACACTGAAAATCAGCCGGATGGAGGCT GAGGATGTTGGGGTTTATTACTGCATGCAAAG TATGCAGCTTCCGATTATCTTCGGCCAGGGGA CACGACTGGAGATTAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTCTGTATTACTGTGCGAGAAGGTATAG CAGCAGCTGGTCGGGCGGTATGGACGTCTGGGG CCAAGGGACCACGGTCACCGTCTCCTCA |
| | | | SEQ ID NO: 24139 | SEQ ID NO: 28145 |
| | | AA | DIVMTQTPLSLSVTPGQPASMSCKSSQSLLHSEG KTYLYWYLQKTGQPPHLLIYEVSKRLSGVPDRF SGSGSGTDFTLKISRMEAEDVGVYYCMQSMQLP IIFGQGTRLEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTALYYCARRYSSS WSGGMDVWGQGTTVTVSS |
| | | | SEQ ID NO: 24140 | SEQ ID NO: 28146 |

FIGURE 50

| | | | | |
|---|---|---|---|---|
| **iPS:468814** | **21-225_223D11** | NA | GACATCCAGATGACCCAGTCTCCCTCCTCACTGTCTGCATCTGTAGGAGACAGAGTCACCATCACTTGTCGGGCGAGTCAGGGCATTAGCAATTATTTAGCCTGGTTTCAGCAGAAACCAGGGAAAGCCCCTAAGTCCCTGATCTATGCTGCATCCACTTTGCAAAGTGGGGTCCCATCAAAGTTCAGCGGCAGTAGATCTGGGACAGATTTTAATCTCACCATCAGCAACCTGCAGCCTGAAGATTTTGCAACTTATTACTGCCAACAGTATAGTGGTTACCCATTCACTTTCGGCCCTGGGACCAAAGTGGATACCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGGACTGGGTCCGCCAGGCTCCAGGCAAGGGGCTGGAGTGGGTGGCAGTTATATGGTATGATGGAAGTAATGACTACTATGCAGACTCCGTGAAGGGCCGATTCACCATCTCCAGAGACAATTCCAAGAACACGCTGTTTCTGCAAATGAACAGCCTGAGAGCCGAGGACACGGCTGTGTATTACTGTGCGAGAGATCGGGGGATCGGGTACAACGATATGGACGTCTGGGGCCAAGGGACCACGGTCACCGTCTCCTCA |
| | | | SEQ ID NO: 24141 | SEQ ID NO: 28147 |
| | | AA | DIQMTQSPSSLSASVGDRVTITCRASQGISNYLAWFQQKPGKAPKSLIYAASTLQSGVPSKFSGSRSGTDFNLTISNLQPEDFATYYCQQYSGYPFTFGPGTKVDTK | QVQLVESGGGVVQPGRSLRLSCAASGFTFSNYGMDWVRQAPGKGLEWVAVIWYDGSNDYYADSVKGRFTISRDNSKNTLFLQMNSLRAEDTAVYYCARDRGIGYNDMDVWGQGTTVTVSS |
| | | | SEQ ID NO: 24142 | SEQ ID NO: 28148 |
| **iPS:468822** | **21-225_147E10** | NA | GCTATTGTGATGACCCAGACTCCACTCTCTCTGTCCGTCACCCCTGGACAGCCGGCCTCCATCTCCTGCAAGTCTAGTCAGCGCCTCCTGCATGGTGATGGAAAGACCTATTTGTATTGGTACCTGCAGAAGCCAGGCCAGCCTCCACACCTCCTGATCTCTGAAGTTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGGTTTATTACTGCATGCAAAGTATACAGGTTCCGTGGACGTTCGGCCAAGGGACCAAGGTGGAAATCAAA | CAGGTGCAGCTGGTGGAGTCTGGGGGAGGCGTGGTCCAGCCTGGGAAGTCCCTGAGACTCTCCTGTGCTGCGTCTGGATTCACCTTCAGTAACTATGGCTTACACTGGGTCCGCCAGGCTCCAGGCAAGGGGCTGGAGTGGGTGGCAATTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCATTACGATTTTTGGAGTGGTCACTTTGACTACTGGGGCCAGGGAACCCTGGTCACCGTCTCCTCA |
| | | | SEQ ID NO: 24143 | SEQ ID NO: 28149 |

FIGURE 50

| | | | | |
|---|---|---|---|---|
| | | AA | AIVMTQTPLSLSVTPGQPASISCKSSQRLLHGDGKTYLYWYLQKPGQPPHLLISEVSNRFSGVPDRFSGSGSGTDFTLKISRVEAEDVGVYYCMQSIQVPWTFGQGTKVEIK | QVQLVESGGGVVQPGKSLRLSCAASGFTFSNYGLHWVRQAPGKGLEWVAIIWYDGSNKYYADSVKGRFTISRDNSKNTLYLQMNSLRAEDTAVYYCARDHYDFWSGHFDYWGQGTLVTVSS |
| | | | SEQ ID NO: 24144 | SEQ ID NO: 28150 |
| iPS:468824 | 21-225_73G6 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCGTCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGTAAGTAATAAATACTATGGAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGGAAGTGGGGATGACTTCTGACTACTGGGGCCAGGGAACCCTGGTCACCGTCTCCTCA |
| | | | SEQ ID NO: 24145 | SEQ ID NO: 28151 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVEIK | QVQLVESGGGVVQPGRSLRLSCAASGFTFSSYGMHWVRQAPGKGLEWVAVIWYDVSNKYYGDSVKGRFTISRDNSKNTLYLQMNSLRAEDTAVYYCAREVGMTSDYWGQGTLVTVSS |
| | | | SEQ ID NO: 24146 | SEQ ID NO: 28152 |

FIGURE 50

| | | | | |
|---|---|---|---|---|
| **iPS:468818** | **21-225_190C8** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGAAACTTATTACTGTCTACAGCATAATGATTACCCGTTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCATCAGTTATGATATCAACTGGGTGCGACAGGCCACTGGACAAGGGCTTGAGTGGATGGGATGGATGAACCCTAAAAGGGGTAACACAGGCTATGCACAGAAGTTCCAGGGCAGAGTCACCATGACCAGGGACACCTCCATAAGCACAGCCTACATGGAGCTGAGCAGCCTGAGATCTGAGGACACGGCCGTTTATTACTGTGCGAGAGGAGACCCGTATAACTGGAACTCCTACGCTATGGACGTCTGGGGCCAAGGGGCCACGGTCACCGTCTCCTCA |
| | | | SEQ ID NO: 24147 | SEQ ID NO: 28153 |
| | | AA | DIQMTQSPSSLSASVGDRVTITCRASQGIRNDLGWYQQKPGKAPKRLIYAASSLQSGVPSRFSGSGSGTEFTLTISSLQPEDFETYYCLQHNDYPFTFGGGTKVEIK | QVQLVQSGAEVKKPGASVKVSCKASGYTFISYDINWVRQATGQGLEWMGWMNPKRGNTGYAQKFQGRVTMTRDTSISTAYMELSSLRSEDTAVYYCARGDPYNWNSYAMDVWGQGATVTVSS |
| | | | SEQ ID NO: 24148 | SEQ ID NO: 28154 |
| **iPS:468826** | **21-225_201C5** | NA | GACATCCAGATGACCCAGTCTCCATCCTCCCTGTCTGCATCTGTAGGAGACAGAGTCACCATCACTTGCCGGGCAAGTCAGGGCATTAGACATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTTCCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGTCATGCACTGGGTCCGCCAGGCTCCAGGCAAGGGGCTGGAGTGGGTGGCAGTTATATGGTATGATGGAAGTAATAAATACTATGTAGACTCCGTGAAGGGCCGATTCACCATCTCCAGAGACAATTCCAGGAACACGCTGTATCTGCAAATGAACAGCCTGAGAGCCGAGGACACGGCTGTGTATTACTGTGCGAGAGAACGGTATAGCAGTGGCTTGTACGACTACGGTATGGACGTCTGGGGCCAAGGGACCACGGTCACCGTCTCCTCA |
| | | | SEQ ID NO: 24149 | SEQ ID NO: 28155 |

FIGURE 50

| | | | | |
|---|---|---|---|---|
| | | AA | DIQMTQSPSSLSASVGDRVTITCRASQGIRHDLGWYQQKPGKAPKRLIYAASSLQSGVPSRFSGSGSGTEFTLTISSLQPEDFATYYCLQHYSFPRTFGQGTKVEIK | QVQLVESGGGVVQPGRSLRLSCAASGFTFSDYVMHWVRQAPGKGLEWVAVIWYDGSNKYYVDSVKGRFTISRDNSRNTLYLQMNSLRAEDTAVYYCARERYSSGLYDYGMDVWGQGTTVTVSS |
| | | | SEQ ID NO: 24150 | SEQ ID NO: 28156 |
| iPS:468828 | 21-225_162A10 | NA | GAAATAGTGATGACGCAGTCTCCAGCCACCCTGTCTGTGTCTCCAGGGGAAAGAGCCACCCTCTCCTGCAGGGCCAGTCAGACTGTTAACAGCAACTTAGCCTGGTACCAGCAGAAATCTGGCCAGGCTCCCAGGCTCCTCATCTTTGGTGCATCCACCAGGGCCACTGTTATCCCAGCCAGGATCAATGGCAGTGGGTCTGGGACAGAGTTCACTCTCACCATCAGCAGCCTGCGGTCTGAAGATTTTGCAGTTTATTTCTGTCAGCAGTATAATGACTGGCCGTGCAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTGTGGCATGCACTGGGTCCGCCAGGCTCCAGGCAAGGGGCTGGAATGGGTGGCAGCTATATGGTATGATGGAAGCAATAAATACTATGCAGACTCCGTGAAGGGCCGATTCACCATCTCCAGAGACAATTCCAAGAACACGCTGTTTCTGCAAATGAACAGCCTGAGAGCCGAGGACACGGCTGTGTATTACTGTGCGAGAGACAAAAATATAATGGGAGATACTTGGTTTGACTTCTGGGGCCAGGGAACCCTGGTCACCGTCTCCTCA |
| | | | SEQ ID NO: 24151 | SEQ ID NO: 28157 |
| | | AA | EIVMTQSPATLSVSPGERATLSCRASQTVNSNLAWYQQKSGQAPRLLIFGASTRATVIPARINGSGSGTEFTLTISSLRSEDFAVYFCQQYNDWPCSFGQGTKLEIK | QVQLVESGGGVVQPGRSLRLSCAASGFTFSSCGMHWVRQAPGKGLEWVAAIWYDGSNKYYADSVKGRFTISRDNSKNTLFLQMNSLRAEDTAVYYCARDKNIMGDTWFDFWGQGTLVTVSS |
| | | | SEQ ID NO: 24152 | SEQ ID NO: 28158 |

FIGURE 50

| | | | | |
|---|---|---|---|---|
| **iPS:468830** | **21-225_191G11** | NA | GAAATAGTGATGACGCAGTCTCCAGCCACCCTGTCTGTGTCTCCAGGGGAAAGAGCCAACCTCTCCTGCAGGACCAGTCAGAGTGTTTGGATTAGCGTAGCCTGGTACCACCAGAAACCTGGCCAGGCTCCCAGGCTCCTCATCTATGGTGCAGCCACCAGGGCCACTGGTATCCCAGCCAGGTTTAGTGGCAGTGGGTCTGGGACAGAGTTCACTCTCACCATCAGCAGCCTGCAGTCTGAAGATTTTGCAGTTTATTACTGTCAGCAGTATAATTACTGGCCGCTCACTTTCGGCGGAGGGACCAAGGTGGAGATCAAA | CAGGTGCAACTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGACTTGAGTGGATGGGATGGATCAACCCTAATAGTGGTGGCACAAACTTTGCACAGAAGTTTCAGGGCAGGGTCACCTTGACCAGGGACACGTCCATCAACACAGCCTACATGGAGCTGAGCTGGCTGCGATCTGACGACACGGCCGTATATTACTGTGCGCGTGGAAAGAACTATGGCTCCTACTTTGACTACTGGGGCCAGGGAACCCTGGTCACCGTCTCCTCG |
| | | | SEQ ID NO: 24153 | SEQ ID NO: 28159 |
| | | AA | EIVMTQSPATLSVSPGERANLSCRTSQSVWISVAWYHQKPGQAPRLLIYGAATRATGIPARFSGSGSGTEFTLTISSLQSEDFAVYYCQQYNYWPLTFGGGTKVEIK | QVQLVQSGAEVKKPGASVKVSCKASGYTFTGYYMHWVRQAPGQGLEWMGWINPNSGGTNFAQKFQGRVTLTRDTSINTAYMELSWLRSDDTAVYYCARGKNYGSYFDYWGQGTLVTVSS |
| | | | SEQ ID NO: 24154 | SEQ ID NO: 28160 |
| **iPS:468832** | **21-225_76H10** | NA | GACATCCAGATGACCCAGTCTCCATCCTCCCTGTCTGCCTCTGCAGGAGACAGAGTCACCATCACTTGCCGGGCAAGTCAGGACATTAGAAATTATTTAGGCTGGTATCAGCAGAAACCAGGGAAAGCCCCTAAGCGACTGATCTATGGTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTTTTACTGTCTACAGTATAATAGTTACCCATTCACTTTCGGCCCTGGGACCAAAGTGGATATCAAA | CAGGTGCAGCTACAACAGTGGGGCGCAGGACTGTTGAAGCCTTCGGAGACCCTGTCCCTCACTTGCGCTGTCAATGGTGGGCCCTTCAGCGGTTGCTACTGGAGCTGGATCCGCCAGCCCCCAGGGAAGGGGCTGGAGTGGATTGGGGAAATCAATTATAGTGGAAGGACCAACTTCAACCCGTCCCTCAAGAGTCGAGTCACCATATCAGTTGACACGTCCAAGAACCAGTTCTCCCTGAAGCTGAGCTCTGTGACCGCCGCGGACACGGCTGTGTATTACTGTGCGAGAGACTACGGCGGTATGGACGTCTGGGGCCAAGGGACCACGGTCACCGTCTCCTCA |
| | | | SEQ ID NO: 24155 | SEQ ID NO: 28161 |

FIGURE 50

| | | | | |
|---|---|---|---|---|
| | | AA | DIQMTQSPSSLSASAGDRVTITCRASQDIRNYLGWYQQKPGKAPKRLIYGASSLQSGVPSRFSGSGSGTEFTLTISSLQPEDFATFYCLQYNSYPFTFGPGTKVDIK | QVQLQQWGAGLLKPSETLSLTCAVNGGPFSGCYWSWIRQPPGKGLEWIGEINYSGRTNFNPSLKSRVTISVDTSKNQFSLKLSSVTAADTAVYYCARDYGGMDVWGQGTTVTVSS |
| | | | SEQ ID NO: 24156 | SEQ ID NO: 28162 |
| iPS:468834 | 21-225_94G10 | NA | GAAATTGTGTTGACGCAGTCTCCAGGCACCCTGTCTTTGTCTCCAGGGGAGAGAGCCACCCTCTCCTGCAGGGCCAGTCAGAGTGTTAACAGCAACTACTTAGCCTGGTACCGGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCATTCTCATCATCAGCAGACTGGAGCCTGAGGATTTTGCAGTGTATTACTGTCAGCAGTATGAAAGCTCGCCGTGGACGTTCGGCCAAGGGACCAAGGTGGAAATCAAA | CAGGTGCAGCTACAACAGTGGGGCGCAGGACTGTTGAAGCCTTCGGAGACCCTGTCCCTCACTTGCGCTGTCAATGGTGGGCCCTTCAGCGGTTGCTACTGGAGCGGGATCCGCCAGCCCCCAGGGAAGGGGCGGGAGTGGATTGGGGAAATCAATTATAGTGGAAGGACCAACTTCAACCCGTCCCTCAAGAGTCGAGTCACCATATCAGTTGACACGTCCAAGAACCAGTTCTCCCTGAAGCTGAGCTCTGTGACCGCCGCGGACACGGCTGTGTATTACTGTGCGAGAGACTACGGCGGTATGGACGTCTGGGGCCAAGGGACCACGGTCACCGTCTCCTCA |
| | | | SEQ ID NO: 24157 | SEQ ID NO: 28163 |
| | | AA | EIVLTQSPGTLSLSPGERATLSCRASQSVNSNYLAWYRQKPGQAPRLLIYGASSRATGIPDRFSGSGSGTDFILIISRLEPEDFAVYYCQQYESSPWTFGQGTKVEIK | QVQLQQWGAGLLKPSETLSLTCAVNGGPFSGCYWSGIRQPPGKGREWIGEINYSGRTNFNPSLKSRVTISVDTSKNQFSLKLSSVTAADTAVYYCARDYGGMDVWGQGTTVTVSS |
| | | | SEQ ID NO: 24158 | SEQ ID NO: 28164 |

FIGURE 50

| | | | | |
|---|---|---|---|---|
| **iPS:468836** | **21-225_198E3** | NA | GACATCCAGATGACCCAGTCTCCATCCTCCCTGTCTGCATCTGTAGGAGACAGAGTCACCATCACTTGCCGGGCAAGTCAGGGCATAAGAAAAGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATGTCTGTCTACAACATTATCGTTACCCTTTCACTTTCGGCCCTGGGACCAAAGTGGATTTCAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAGTGGGTGGCAGTTATATCATATGATGGAGGTTATAAAAACTATGCAGACTCCGTGAAGGGCCGATTCACCATCTCCAGAGACAATTCCAAGAACACGCTGTATCTGCAAATGAATAGCCTGAGAGCTGAGGACACGGCTGTGTATTACTGTGCGAGAGGTACCCACGGGTACTACTACGGTGTGGACGTCTGGGGCCAAGGGACCACGGTCACCGTCTCCTCA |
| | | | SEQ ID NO: 24159 | SEQ ID NO: 28165 |
| | | AA | DIQMTQSPSSLSASVGDRVTITCRASQGIRKDLGWYQQKPGKAPKRLIYAASSLQSGVPSRFSGSGSGTEFTLTISSLQPEDFATYVCLQHYRYPFTFGPGTKVDFK | QVQLVESGGGVVQPGRSLRLSCAASGFTFSSYGMHWVRQAPGKGLEWVAVISYDGGYKNYADSVKGRFTISRDNSKNTLYLQMNSLRAEDTAVYYCARGTHGYYYGVDVWGQGTTVTVSS |
| | | | SEQ ID NO: 24160 | SEQ ID NO: 28166 |
| **iPS:468838** | **21-225_80E12** | NA | GAAATTGTGTTGACGCAGTGTCCAGGCACCCTGTCTTTGTCTCCAGGGGAGAGAGCCACCGTCTCCTGCAGGGCCAGTCAGAGCGTTAACAGCAACTACTTAGCCTGGTACCGGCAGAAACCTGACCAGGCTCCCAGGCTCCTCATCTATGGTGCATCCAGCAGGGCCACTGGCATCCCAGACAGGTTCAGTGGCAGTGGGTCTGGGACAGACTTCATTCTCATCATCAGCAGACTGGAGCCTGAGGATTTTGCAGTGTATTACTGTCAGCAGTATGAAAGCTCGCCGTGGACGTTCGGCCAAGGGACCAAGGTGGAAATCAAA | CAGGTGCAGCTACAACAGTGGGGCGCAGGACTGTTGAAGCCTTCGGAGACCCTGTCCCTCACTTGCGCTGTCAATGGTGGGCCCTTCAGCGGTTGCTACTGGAGCTGGATCCGCCAGCCCCCAGGGAAGGGGCTGGAGTGGATTGGGGAAATCAATTATAGTGGAAGGACCAACTTCAACCCGTCCCTCAAGAGTCGAGTCACCATATCAGTTGACACGTCCAAGAACCAGTTCTCCCTGAAGCTGAGCTCTGTGACCGCCGCGGACACGGCTGTGTATTACTGTGCGAGAGACTACGGCGGTATGGACGTCTGGGGCCAAGGGACCACGGTCACCGTCTCCTCA |
| | | | SEQ ID NO: 24161 | SEQ ID NO: 28167 |

FIGURE 50

| | | | | |
|---|---|---|---|---|
| | | AA | EIVLTQCPGTLSLSPGERATVSCRASQSVNSNYLAWYRQKPDQAPRLLIYGASSRATGIPDRFSGSGSGTDFILIISRLEPEDFAVYYCQQYESSPWTFGQGTKVEIK | QVQLQQWGAGLLKPSETLSLTCAVNGGPFSGCYWSWIRQPPGKGLEWIGEINYSGRTNFNPSLKSRVTISVDTSKNQFSLKLSSVTAADTAVYYCARDYGGMDVWGQGTTVTVSS |
| | | | SEQ ID NO: 24162 | SEQ ID NO: 28168 |
| **iPS:468840** | **21-225_200H9** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGATCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCTCTCACTTTCGGCGGAGGGACCAAGGTGGAGATCAAA | CAGGTGCAGCTGCAGGAGTCGGGCCCAGGACTGGTGAAGCCTTCACAGACCCTGTCCCTCACCTGCATTGTCTCTGGTGGCTCCATGAGGAGTGGTGGTGACTACTGGAGCTGGATCCGCCAGCACCCAGGGAAGGGCCTGGAGTGGTTTGGGTACATCTATTACAGTGGGAGCACCTACTACAACCCGTCCCTCAAGAGTCGAGTTACCTTATCAGTAGACACGTCTAAGAACCAGTTCTCCCTGAAGCTGAGCTCTGTGACTGCCGCGGACACGGCCGTGTATTACTGTGCGAGAATGGACTACAGTAACTACTACTACGGTATGGACGTCTGGGGCCAAGGGACCTCGGTCACCGTCTCCTCA |
| | | | SEQ ID NO: 24163 | SEQ ID NO: 28169 |
| | | AA | DIQMTQSPSSLSASVGDRVTITCRASQGIRNDLGWYQQKPGKAPKRLIYAASSLQSGIPSRFSGSGSGTEFTLTISSLQPEDFATYYCLQHNSYPLTFGGGTKVEIK | QVQLQESGPGLVKPSQTLSLTCIVSGGSMRSGGDYWSWIRQHPGKGLEWFGYIYYSGSTYYNPSLKSRVTLSVDTSKNQFSLKLSSVTAADTAVYYCARMDYSNYYYGMDVWGQGTSVTVSS |
| | | | SEQ ID NO: 24164 | SEQ ID NO: 28170 |

FIGURE 50

| | | | | |
|---|---|---|---|---|
| **iPS:468820** | **21-225_76E10** | NA | GAAATTGTGTTGACGCAGTCTCCAGGCACCCTGTCTTTGTCTCCAGGGGAGAGAGCCACCCTCTCCTGCAGGGCCAGTCAGAGTGTTAACAGCAACTACTTAGCCTGGTACCGGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCATTCTCATCATCAGCAGACTGGAGCCTGAGGATTTTGCAGTGTATTACTGTCAGCAGTATGAAAGCTCGCCGTGGACGTTCGGCCAAGGGACCAAGGTGGAAATCAAA | CAGGTGCAGCTCCAACAGTGGGGCGCAGGACTGTTGAAGCCTTCGGAGACCCTGTCCCTCACTTGCGCTGTCAATGGTGGGCCCTTCAGCGGTTCCTACTGGAGCTGGATCCGCCAGCCCCCAGGGAAGGGGCTGGAGTGGATTGGGGAAATCAATTATAGTGGAAGGACCAACTTCAACCCGTCCCTCAAGAGTCGAGTCACCATATCAGTTGACACGTCCAAGAACCAGTTCTCCCTGAAGCTGAGCTCTGTGACCGCCGCGGACACGGCTGTGTATTACTGTGCGAGAGACTACGGCGGTATGGACGTCTGGGGCCAAGGGACCACGGTCACCGTCTCCTCA |
| | | | SEQ ID NO: 24165 | SEQ ID NO: 28171 |
| | | AA | EIVLTQSPGTLSLSPGERATLSCRASQSVNSNYLAWYRQKPGQAPRLLIYGASSRATGIPDRFSGSGSGTDFILIISRLEPEDFAVYYCQQYESSPWTFGQGTKVEIK | QVQLQQWGAGLLKPSETLSLTCAVNGGPFSGSYWSWIRQPPGKGLEWIGEINYSGRTNFNPSLKSRVTISVDTSKNQFSLKLSSVTAADTAVYYCARDYGGMDVWGQGTTVTVSS |
| | | | SEQ ID NO: 24166 | SEQ ID NO: 28172 |
| **iPS:468842** | **21-225_50H4** | NA | GACATCCAGATGACCCAGTCTCCATCCTCCCTGTCTGCATCTGTTGGAGACAGAGTCACCATCACTTGCCGGGCAAGTCAGGGCATTAGAAATGATTTAGGCTGGTATCAGCAGAAACCAGGGAAGGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTACAGCCTGAAGATTTTGCAACTTATTACTGTCTACAACATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGTCATGCACTGGGTCCGCCAGGCTCCAGGCAAGGGGCTGGAGTGGGTGGCAGCTATATGGTATGATGGAAGTAATAAATACTATGCAGACTCCGTGAAGGGCCGATTCACCATCTCCAGAGACAATTCCAAGAACACGCTGGATCTGCAAATGAACAGCCTGAGAGCCGAGGACACGGCTGTGTATTACTGTGCGAGAGAGTTGTATAGCAGCAACTGGTACGACTACGGTATGGACGTCTGGGGCCAAGGGACCACGGTCACCGTCTCCTCA |
| | | | SEQ ID NO: 24167 | SEQ ID NO: 28173 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPRTFGQGTKVEIK | QVQLVESGGGVVQPGRSLRLSCAASGFTFSSYVMHWVRQAPGKGLEWVAAIWYDGSNKYYADSVKGRFTISRDNSKNTLDLQMNSLRAEDTAVYYCARELYSSNWYDYGMDVWGQGTTVTVSS |
| | | | SEQ ID NO: 24168 | SEQ ID NO: 28174 |
| iPS:468844 | 21-225_48E10 | NA | GACATCCAGATGACCCAGTCTCCATCCTCCCTGTCTGCATCTGTAGGAGACAGAGTCACCATCACTTGCCGGGCAAGTCAGGGCATTAGAAGTGATTTAGGCTGGTATCAGCAGAAACCAGGGAAAGCCCCTAAGCGCCTGATCTATACTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGTATAATAGTTACCCATTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAACATGAACTGGGTCCGCCAGGCTCCAGGGAAGGGGCTGGAGTGGGTCTCATCCATTAGTGGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAAGCCTCGACCTCTGGGGCCAGGGAACCCTGGTCACCGTCTCCTCA |
| | | | SEQ ID NO: 24169 | SEQ ID NO: 28175 |
| | | AA | DIQMTQSPSSLSASVGDRVTITCRASQGIRSDLGWYQQKPGKAPKRLIYTASSLQSGVPSRFSGSGSGTEFTLTISSLQPEDFATYYCLQYNSYPFTFGPGTKVDIK | EVQLVESGGGLVKPGGSLRLSCAASGFTFSSYNMNWVRQAPGKGLEWVSSISGSSSYIYYADSVKGRFTISRDNAKNSLYLQMNSLRAEDTAVYYCARSLDLWGQGTLVTVSS |
| | | | SEQ ID NO: 24170 | SEQ ID NO: 28176 |

FIGURE 50

| | | | | |
|---|---|---|---|---|
| **iPS:468846** | **21-225_53B10** | NA | GACATCCAGATGACCCAGTCTCCATCCTCCCTGTCTGCCTCTGCAGGAGACAGAGTCACCATCACTTGCCGGGCAAGTCAGGACATTAGAAATTATTTAGGCTGGTATCAGCAGAAACCAGGGAAAGCCCCTAAGCGACTGATCTATGGTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTTTTACTGTCTACAGTATAATAGTTACCCATTCACTTTCGGCCCTGGGACCAAAGTGGATATCAAA | GAGGTGCAGTTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGCAGTTACATATACTACGTAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGTCAACTCTTTTGACTCCTGGGGCCAGGGAACCCTGGTCACCGTCTCCTCA |
| | | | SEQ ID NO: 24171 | SEQ ID NO: 28177 |
| | | AA | DIQMTQSPSSLSASAGDRVTITCRASQDIRNYLGWYQQKPGKAPKRLIYGASSLQSGVPSRFSGSGSGTEFTLTISSLQPEDFATFYCLQYNSYPFTFGPGTKVDIK | EVQLVESGGGLVKPGGSLRLSCAASGFTFSSYSMNWVRQAPGKGLEWVSSISGSSSYIYYVDSVKGRFTISRDNAKNSLYLQMNSLRAEDTAVYYCARVNSFDSWGQGTLVTVSS |
| | | | SEQ ID NO: 24172 | SEQ ID NO: 28178 |
| **iPS:468848** | **21-225_54B1** | NA | GACATCCAGATGACCCAGTCTCCATCCTCCCTGTCTGCTTCTATTGGAGACAGAGTCACCATCACTTGCCGGGCAAGTCAGAACATTAGCAGCTATTTAAATTGGTATCAGCAGAAACCAGGGAAGGCCCCCAAGTTCCTGATCTATGCTGCATCCAGTTTGCATAGTGGGGTCCCACCAAGGTTCAGTGGCAGTGGATCTGGGACAGATTTCACTCTCACCATCAGCAGTCTGCAACCTGAAGATTTTGCAATTTACTACTGTCAACAGAGTTACAGAACCCCTCTGTGGACGTTCGGCCAAGGGACCAAGGTGGAAATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTCTTAGTGGTAGTGGTGGTAGCACATTCTACGCAGACTCCGTGAAGGGCCGGTTCACCATCTCCAGAGAGAATTCCAAGAACACGCTGTATCTGCAAATGAGCAGCCTGAGAGCCGAGGACACGGCCGTATATTACTGTGCCCGAAGAGGCCGTGAATATAGTGGCTACGATTACTTTGACTACTGGGGCCAGGGAACCCTGGTCACCGTCTCCTCA |
| | | | SEQ ID NO: 24173 | SEQ ID NO: 28179 |

FIGURE 50

| | | | | |
|---|---|---|---|---|
| | | AA | DIQMTQSPSSLSASIGDRVTITCRASQNISSYLNWYQQKPGKAPKFLIYAASSLHSGVPPRFSGSGSGTDFTLTISSLQPEDFAIYYCQQSYRTPLWTFGQGTKVEIK | EVQLLESGGGLVQPGGSLRLSCAASGFTFSSYAMSWVRQAPGKGLEWVSVLSGSGGSTFYADSVKGRFTISRENSKNTLYLQMSSLRAEDTAVYYCARRGREYSGYDYFDYWGQGTLVTVSS |
| | | | SEQ ID NO: 24174 | SEQ ID NO: 28180 |
| **iPS:468850** | **21-225_63F4** | NA | GACATCGTGATGACCCAATCTCCAGACTCCCTGGCTGTGTCTCTGGGCGAGAGGGCCACCATCAACTGCAAGTCCAGCCAGAGTGTTTTATCCAGCTCCAACAATAACAACTACTTAGCTTGGTACCAGCAGAAACCAGGACAGCCTCCTAAGCTGCTCATTTACTGGGCATCTACCCGGGAATCCGGGATCCCTGACCGATTCAGTGGCAGCGGGTCTGGGACAGGTTTCACTCTCACCATCAGCAGCCTGCAGGCTGAGGATGTGGCAGTTTATTACTGTCAGCAATATTATACTACTCCGTGCAGTTTTGGCCAGGGGACCAAGCTGGAGATCAAT | CAGGTGCAGCTGGTGCAATCTGGGGCTGAGGTGAAGAAGCCTGGGGCCTCAGTGAAGGTCTCCTGCAAGGCTTCTGGATACACCTTCACCAATTATGATGTCAACTGGGTGCGACAGGCCACTGGACAAGGGCTTGAGTGGATGGGATGGATGCACCCTAACAGTGGTAACACAGGCTATGCACAGAAGTTCCGGGGCAGAGTCACCATGACCAGGAACACCTCCCTAAGCACAGTCTACATGGAGCTGAGCAGCCTGCGATCTGAGGACACGGCCGTGTATTACTGTGCCTATAGCAGTGGCTGGTACGTTTTTGACTACTGGGGCCAGGGAACCCTGGTCACCGTCTCCTCA |
| | | | SEQ ID NO: 24175 | SEQ ID NO: 28181 |
| | | AA | DIVMTQSPDSLAVSLGERATINCKSSQSVLSSSNNNNYLAWYQQKPGQPPKLLIYWASTRESGIPDRFSGSGSGTGFTLTISSLQAEDVAVYYCQQYYTTPCSFGQGTKLEIN | QVQLVQSGAEVKKPGASVKVSCKASGYTFTNYDVNWVRQATGQGLEWMGWMHPNSGNTGYAQKFRGRVTMTRNTSLSTVYMELSSLRSEDTAVYYCAYSSGWYVFDYWGQGTLVTVSS |
| | | | SEQ ID NO: 24176 | SEQ ID NO: 28182 |

FIGURE 50

| | | | | |
|---|---|---|---|---|
| **iPS:468852** | **21-225_71F3** | NA | GACATCGTGATGACCCAATCTCCAGACTCCCTGGCTGTGTCTCTGGGCGCGAGGGCCACCATCAACTGCAAGTCCAGCCAGAGTGTTTTATCCAACTCCAACAATAACAACTACTTAGCTTGGTACCAGCAGAAACCAGGACAGCCTCCTAAGCTGCTCATTTACTGGGCATCTACCCGGGAATCCGGGATCCCTGACCGATTCAGTGGCAGCGGGTCTGGGACAGATTTCACTCTCACCATCAACAGCCTGCAGGCTGAAGATGTGGCAGTTTATTACTGTCAGCAATATTATACTACTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATGTCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGCGATCTGAGGACACGGCCGTGTATTACTGTGCCTATAGCAGTGGCTGGTACGTTTTTGACTCCTGGGGCCAGGGAACCCTGGTCACCGTCTCCTCA |
| | | | SEQ ID NO: 24177 | SEQ ID NO: 28183 |
| | | AA | DIVMTQSPDSLAVSLGARATINCKSSQSVLSNSNNNNYLAWYQQKPGQPPKLLIYWASTRESGIPDRFSGSGSGTDFTLTINSLQAEDVAVYYCQQYYTTPCSFGQGTKLEIK | QVQLVQSGAEVKKPGASVKVSCKASGYTFTNYDVNWVRQATGQGLEWMGWMHPNSGNTGYAQKFQGRVTMTRNTSISTAYMELSSLRSEDTAVYYCAYSSGWYVFDSWGQGTLVTVSS |
| | | | SEQ ID NO: 24178 | SEQ ID NO: 28184 |
| **iPS:468854** | **21-225_72C4** | NA | GATGTTGTAATGACTCAGTCTCCGCTCTCCCTGCCCGTCACCCTTGGACAGCCGGCCTCCATCTCCTGCAGGTCTGGTCAAAGCCTCGTATACAGTGATGGAAACACCTACTTGAATTGGTTTCAGCAGAGGCCAGGCCAATCTCCAAGGCGCCTAATTTATGAGGTTTCTAAGTGGGACTCTGGGGTCCCAGACAGATTCAGTGGCAGTGGGTCAGGCACTAATTTCACACTGAAAATCAGCAGGGTGGAGGCTGAGGATGTTGGGGTCTTTTACTGCATGCAAGGTACACACTGGCCGCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGTCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAACTGAACAGCCTGAGATCTGAGGACACGGCCGTGTATTACTGTTCGCATAGCAGTGGCTGGTACCTCTTTGACTACTGGGGCCAGGGAACCCTGGTCACCGTCTCCTCA |
| | | | SEQ ID NO: 24179 | SEQ ID NO: 28185 |

FIGURE 50

| | | | | |
|---|---|---|---|---|
| | | AA | DVVMTQSPLSLPVTLGQPASISCRSGQSLVYSDGNTYLNWFQQRPGQSPRRLIYEVSKWDSGVPDRFSGSGSGTNFTLKISRVEAEDVGVFYCMQGTHWPLTFGGGTKVEIK | QVQLVQSGAEVKKPGASVKVSCKASGYTFTNYDINWVRQVTGQGLEWMGWMHPNSGNTGYAQKFQGRVTMTRNTSISTAYMELNSLRSEDTAVYYCSHSSGWYLFDYWGQGTLVTVSS |
| | | | SEQ ID NO: 24180 | SEQ ID NO: 28186 |
| **iPS:468856** | **21-225_77C9** | NA | GATGTTGTGATGACTCAGTCTCCACTCTCCCTGCCCGTCACCCTTGGACAGCCGGCCTCCATCTCCTGCAGGTCTAGTCAAAGCCTCGTTTACAGTGTTGGAAACACCTCCTTGAGTTGGTTTCAGCAGAGGCCAGGCCAATCTCCAAGGCGCCTAATTTATAAGGTTTCTAACTGGGACTCTGGGGTCCCAGACAGATTCAGCGGCAGTGGGTCAGGCACTGATTTCACACTGAAAATCAGCAGGGTGGAGGCTGAGGATGTTGGGGTTTATTACCGCATGCAAGGTACACACTGGCCATTCACTTTCGGCCCTGGGACCAAAGTGGATATCAAA | CAGCTGCAGCTGCAGGAGTCGGGCCCAGGACTGGTGACGCCTTCGGAGACCCTGTCCCTCACCTGCACTGTCTCTGGCGGCTCCATCAGCAGGAGTAGTTACTACTGGGGCTGGATCCGCCAGCCCCCAGGGAAGGGGCTGGAGTGGATTGGGAGTATCTATTATAGTGGGAGCGCCTACTCCAACCCGTCCCTCAAGAGTCGAGTCACCATATCCGTAGACACGTCCAAGAACCAGTTCTCCCTGAAGCTGAGCTCTGTGACCGCCGCGGACACGGCTCTGTTTTACTGTGCGAGACTTGACTCTAACTGGGGTCTTGACTACTGGGGCCAGGGAACCCTGGTCACCGTCTCCTCA |
| | | | SEQ ID NO: 24181 | SEQ ID NO: 28187 |
| | | AA | DVVMTQSPLSLPVTLGQPASISCRSSQSLVYSVGNTSLSWFQQRPGQSPRRLIYKVSNWDSGVPDRFSGSGSGTDFTLKISRVEAEDVGVYYRMQGTHWPFTFGPGTKVDIK | QLQLQESGPGLVTPSETLSLTCTVSGGSISRSSYYWGWIRQPPGKGLEWIGSIYYSGSAYSNPSLKSRVTISVDTSKNQFSLKLSSVTAADTALFYCARLDSNWGLDYWGQGTLVTVSS |
| | | | SEQ ID NO: 24182 | SEQ ID NO: 28188 |

FIGURE 50

| | | | | |
|---|---|---|---|---|
| **iPS:468858** | **21-225_148C9** | NA | GACATCCAGATGACCCAGTCTCCATCCTCCCTGTCTGCATCTGTAGGAGACAGAGTCACCATCACTTGCCGGGCAAGTCGGGGCATTAGAGATGATTTAGGCTGGTATCAGCAGAAACCAGGGAAAGCCCCTAAGCGCCTGATCTATGCTGCATCCAGTTTGCAGAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTATCCTCGGACGTTCGGCCAAGGGACCAAGGTGGAAATCAAA | CAGGTGCAGCTGGTGGAGTCTGGGGGAGGCGTGGCCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGGAAGTAATAAGTACTATGCAGACTCCGTGAAGGGCCGACTCACCATCTCCAGAGACAATTCCAAGAACACGCTGTATCTGCAAATGAACAGCCTGAGAGCCGAGGACACGGCTGTGTATTACTGTGCGAGAGAGAGGTATAGCAGTGGCTGGTACGACTACGGTTTGGACGTCTGGGGCCAAGGGACCACGGTCACCGTCTCCTCA |
| | | | SEQ ID NO: 24183 | SEQ ID NO: 28189 |
| | | AA | DIQMTQSPSSLSASVGDRVTITCRASRGIRDDLGWYQQKPGKAPKRLIYAASSLQSGVPSRFSGSGSGTEFTLTISSLQPEDFATYYCLQHYSYPRTFGQGTKVEIK | QVQLVESGGGVAQPGRSLRLSCAASGFTFSDYGMHWVRQAPGKGLEWVAVIWYDGSNKYYADSVKGRLTISRDNSKNTLYLQMNSLRAEDTAVYYCARERYSSGWYDYGLDVWGQGTTVTVSS |
| | | | SEQ ID NO: 24184 | SEQ ID NO: 28190 |
| **iPS:468860** | **21-225_224E7** | NA | GACATCCAGATGACCCAGTCTCCTTCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTACGCGCCTGATCTATGCTGCATCCAGTTTGCAAAGTGGGGTCCCATCGAGGTTCAGCGGCAGTGGATCTGGGACAGAATTCACTCTCACAATCAGCAGCCTGCAGCCTGAAGATTTTGCAACTTATTACTGTCTACAACATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGCCTCTCCTGTGCAGCGTCTGGATTCACCTTCAGTAGCTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACAGTATAGCAGCAGCTGGTACGACTTCGGTCTGGACGTCTGGGGCCAAGGGACCACGGTCACCGTCTCCTCA |
| | | | SEQ ID NO: 24185 | SEQ ID NO: 28191 |

FIGURE 50

| | | | | |
|---|---|---|---|---|
| | | AA | DIQMTQSPSSLSASVGDRVTITCRASQGIRNDLGWYQQKPGKAPTRLIYAASSLQSGVPSRFSGSGSGTEFTLTISSLQPEDFATYYCLQHYSYPRTFGQGTKVEIK | QVQLVESGGGVVQPGRSLSLSCAASGFTFSSYVMHWVRQAPGKGLEWVAVIWYDGSNKYYADSVKGRFTISRDNSKNTLYLQMNSLRAEDTAVYYCAREQYSSSWYDFGLDVWGQGTTVTVSS |
| | | | SEQ ID NO: 24186 | SEQ ID NO: 28192 |
| iPS:468862 | 21-225_178H8 | NA | CAGTCTGCCCTGACTCAGTCTGCCTCCGTGTCGGGGTCTCCTGGACAGTCGATCACCATCTCCTGCACTGGAACCAGCAGTGACGTTGGTGGTTATAATTTTGTCTCCTGGTACCAACAGCACCCAGGCAAAGTCCCCAAATTCATGATTTATGAGGTCAGTAATCGGCCCTCAGGGGTTCCTAATCGTTTTTCTGGCTCCAAGTCTGGCAACACGGCCTCCCTGACCATCTCTGGCCTCCAGGCTGAGGACGAGGCTGATTATTACTGCAGCTCATATACAAGCAGCTACACTTGGGTGTTCGGCGGAGGGACCAAACTGACCGTCCTA | CAGGTGCAGCTGGTGCAGTCTGGGGCTGAGGTGAGGACGCCTGGGGCCTCAGTGAAGGTCTCCTGCAAGGCTTCTGGATACACCTTCACCGACTATTATATGCACTGGGTGCGACAGGCCCCTGGACAAGGGCTTGAGTGGATGGGATGGATCAACCCTAACAGAGGTGGCACAAACTATGCTCAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGAGAGGAGGATCGCAGTGGCTGGTACTACTACTACGGTATGGACGTCTGGGGCCAAGGGACCACGGTCACCGTCTCCTCA |
| | | | SEQ ID NO: 24187 | SEQ ID NO: 28193 |
| | | AA | QSALTQSASVSGSPGQSITISCTGTSSDVGGYNFVSWYQQHPGKVPKFMIYEVSNRPSGVPNRFSGSKSGNTASLTISGLQAEDEADYYCSSYTSSYTWVFGGGTKLTVL | QVQLVQSGAEVRTPGASVKVSCKASGYTFTDYYMHWVRQAPGQGLEWMGWINPNRGGTNYAQKFQGRVTMTRDTSISTAYMELSRLRSDDTAVYYCAREEDRSGWYYYYGMDVWGQGTTVTVSS |
| | | | SEQ ID NO: 24188 | SEQ ID NO: 28194 |

FIGURE 50

| | | | | |
|---|---|---|---|---|
| **iPS:468864** | **21-225_60D6** | NA | CAGTCTGTGCTGACTCAGCCACCCTCAGCGTCTGGGACCCCCGGGCAGAGGGTCACCATCTCTTGTTCTGGAAGCAGCTCCAACATCGGAAGTAATACTGTAAACTGGTACCAGCAGCTCCCAGGAACGGCCCCCAAACTCCTCATCTATAGTAATAATCAGCGGCCCTCAGGGGTCCCTGACCGATTCTCTGGCTCCAAGTCTGGCACCTCAGCCTCCCTGGCCATCAGTGGGCTCCAGTCTGAGGATGAGGCTGATTATTACTGTGCAGCATGGGATGACAGCCTGAATGGTCCGGTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGGTTCTCACTCAGCACTAGTGGAGTGGGTGTGGGCTGGATCCGTCAGCCCCCAGGAAAGGCCCTGGAGTGGCTTGCACTCATTTATTGGAAAGATGATGAGCGCTACAGCCCATCTCTGAAGAGCAGGCTCACCATCACCAAGGACACTTCCAAAAACCAGGTGGTCCTTACAATGACCAACATGGACCCTGTGGACACAGCCACATATTACTGTGCACATGCAGTGGCTGTCTCCTTTGACTACTGGGGCCAGGGAACCCTGGTCACCGTCTCCTCA |
| | | | SEQ ID NO: 24189 | SEQ ID NO: 28195 |
| | | AA | QSVLTQPPSASGTPGQRVTISCSGSSSNIGSNTVNWYQQLPGTAPKLLIYSNNQRPSGVPDRFSGSKSGTSASLAISGLQSEDEADYYCAAWDDSLNGPVGGGTKLTVL | QITLKESGPTLVKPTQTLTLTCTFSGFSLSTSGVGVGWIRQPPGKALEWLALIYWKDDERYSPSLKSRLTITKDTSKNQVVLTMTNMDPVDTATYYCAHAVAVSFDYWGQGTLVTVSS |
| | | | SEQ ID NO: 24190 | SEQ ID NO: 28196 |
| **iPS:468866** | **21-225_190C1** | NA | TCCTATGAGCTGACACAGCCACCCTCGGTGTCAGTGTCCCCAGGACAAACGGCCAGGATCACCTGCACTGGAGATGCAATGCCGAAAAAATATGCTTATTGGGACCAGCAGAAGTCAGGCCAGGCCCCTGTGCTGGTCATCTCTGAGGACAGCAAGCGACCCTCCGGGATCCCTGAGAGATTCTCTGGCTCCAGCTCAGGGACAATGGCCCCCTTGACTATCAGTGGGGCCCAGGTGGAGGATGAAACTGACTACGACTGTAACTCAACAGACAGCAGTGGTAATCGGGTGTTCGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGACTTGAGTGGATGGGATGGATCAACCCTTACAGTGGTGGCACAAACTATGCACAGAAGTTTCAGGGCAGGGTCACCATGACCAGGGACACGTCCATCAGCACAGCCTACATGGAACTGAGCAGGCTGAGATCTGACGACACGGCCGTGTATTACTGTGCGAGAGATAGAGCAGTGGCTGGAAACTACTTCTACTACGGTATGGACGTCTGGGGCCAAGGGACCACGGTCACCGTCTCCTCA |
| | | | SEQ ID NO: 24191 | SEQ ID NO: 28197 |

FIGURE 50

| | | | | |
|---|---|---|---|---|
| | | AA | SYELTQPPSVSVSPGQTARITCTGDAMPKKYAYWDQQKSGQAPVLVISEDSKRPSGIPERFSGSSSGTMAPLTISGAQVEDETDYDCNSTDSSGNRVFGGGTKLTVL | QVQLVQSGAEVKKPGASVKVSCKASGYTFTGYYMHWVRQAPGQGLEWMGWINPYSGGTNYAQKFQGRVTMTRDTSISTAYMELSRLRSDDTAVYYCARDRAVAGNYFYYGMDVWGQGTTVTVSS |
| | | | SEQ ID NO: 24192 | SEQ ID NO: 28198 |
| **iPS:468868** | **21-225_74A1** | NA | GACATCCAGATGACCCAGTCTCCATCCTCCCTGTCGGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTACGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATGATAGTTACCCTCTCACTTTCGGCGGAGGGGCCAAGGTGGAGATCAAA | CAACTGCAGCTGCAGGAGTCGGGCCCGGGACTGGTGAAGCCTTCGGAGACCCTGTCCCTCACCTGCACTGTCTCTGGTGGCTCCATCAGCGGTAGTAGTTACTACTGGGGCTGGATCCGCCAGCCCCCGGGGAAGGGGCTGGAGTGGATTGGGAATATCTATTATAGTGGGAGCACCTACCACAACCCGTCCCTCAAGAGTCGAGTCACCATATCCGTGGACACGTCCAAGAACCAGTTCTCCCTGAAGCTGACCTCTGTGACCGCCGCAGACACGGCTGTGTTTTACTGTGCGAGACATGATTTACTTTGGTCCCTTGACTTCTGGGGCCAGGGAATTCTGGTCACCGTCTCCTCA |
| | | | SEQ ID NO: 24193 | SEQ ID NO: 28199 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHDSYPLTFGGGAKVEIK | QLQLQESGPGLVKPSETLSLTCTVSGGSISGSSYYWGWIRQPPGKGLEWIGNIYYSGSTYHNPSLKSRVTISVDTSKNQFSLKLTSVTAADTAVFYCARHDLLWSLDFWGQGILVTVSS |
| | | | SEQ ID NO: 24194 | SEQ ID NO: 28200 |

FIGURE 50

| | | | | |
|---|---|---|---|---|
| **iPS:468870** | **21-225_74A8** | NA | GACATCGTGATGACCCAGTCTCCAGACTCCCTGGCTGTGTCTCTGGGCGAGAGGGCCACCATCAACTGCAAGTCCAGCCAGAGTGTTTTGTACAGCTCCAACAGTCACAACTACTTAGCTTGGTACCAGCAGAAACCAGGACAGCCTCCTAAACTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCTCTGGACAAGGACTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACTCGGCCGTATATTACTGTGCGTATAGTAGTGGCTGGTACAAATTTGACTACTGGAGCCAGGGAACCCTGGTCACCGTCTCCTCA |
| | | | SEQ ID NO: 24195 | SEQ ID NO: 28201 |
| | | AA | DIVMTQSPDSLAVSLGERATINCKSSQSVLYSSNSHNYLAWYQQKPGQPPKLLIYWASTRESGVPDRFSGSGSGTDFTLTISSLQAEDVAVYYCQQYYSTPCSFGQGTKLEIK | QVQLVQSGAEVKKPGASVKVSCKASGYTFTNYDINWVRQASGQGLEWMGWMHPNSGNTGYAQKFQGRVTMTRNTSISTAYMELSSLRSEDSAVYYCAYSSGWYKFDYWSQGTLVTVSS |
| | | | SEQ ID NO: 24196 | SEQ ID NO: 28202 |
| **iPS:472730** | **21-225_14B1_LC1** | NA | GACATCCAGATGACCCAGTCTCCATCCTACCTGTCTGCATCTGTAGGAGACAGAGTCACCATCACTTGCCGGGCAAGTCAGGACATTAGAGATAATTTAGGCTGGTATCAGCAGAAACCAGGGAAAGCCCCTAAACGCCTGATCTATACTGCATACAGTTTGCAAAGTGGGGTCCCATCAAGGTTCAGCGGCAGTGGATCTGGGACAGAATTCACTCTCACAATCAGCAGCCTGCAGCCTGAGGATTTTGCAACTTATTACTGTCTACAACATTATAATTACCCGCTCACTTTCGGCGGAGGGACCAAGGTGGAGATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATACCATGAACTGGGTCCGCCAGGCTCCAGGGAAGGGGCTGGAGTGGGTCTCATCCATAAGTGGTAGTAGTAGTTACTTATACTACCCAGACTCAGTGAAGGGCCGATTCACCATCTCCAGAGACAACGCCAAGAACTCTCTGTATCTGCAAATGAACAGCCTGAGAGCCGAGGACACGGCTGTGTATTACTGTGCGAGAGATAGAGGCAGCAGCTGGGGCCAGGGAACCCTGGTCACCGTCTCCTCA |
| | | | SEQ ID NO: 24197 | SEQ ID NO: 28203 |

FIGURE 50

| | | | | |
|---|---|---|---|---|
| | | AA | DIQMTQSPSYLSASVGDRVTITCRASQDIRDNLG WYQQKPGKAPKRLIYTAYSLQSGVPSRFSGSGS GTEFTLTISSLQPEDFATYYCLQHYNYPLTFGGG TKVEIK | EVQLVESGGGLVKPGGSLRLSCAASGFTFSSYTMN WVRQAPGKGLEWVSSISGSSSYLYYPDSVKGRFTIS RDNAKNSLYLQMNSLRAEDTAVYYCARDRGSSW GQGTLVTVSS |
| | | | SEQ ID NO: 24198 | SEQ ID NO: 28204 |
| iPS:472731 | 21-225_14B1_LC2 | NA | TCCTTTGAGCTGACTCAGCCACCCTCAGTGTCC GTGTCCCCAGGACAGACAGCCAGCATCACCTG CTCTGGAGATAAATTGGGGGATAAATATGCTT ACTGGTATCAGCAGAAGCCAGGCCAGTCCCCT GTGTTGGTCATCTATCAAGATAGGAAGCGCCC CTCAGGGATCCCTGAGCGATTCTCTGGCTCCA ACTCTGGGAACACAGCCACTCTGACCATCAGC GGGACCCAGGCTATGGATGAGGCTGACTATTA CTGTCAGGCGTGGGACAACAGCACTGTGGTGT TCGGCGGAGGGACCAAGCTGACCGTCCTA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATACCAT GAACTGGGTCCGCCAGGCTCCAGGGAAGGGGCT GGAGTGGGTCTCATCCATAAGTGGTAGTAGTAGT TACTTATACTACCCAGACTCAGTGAAGGGCCGAT TCACCATCTCCAGAGACAACGCCAAGAACTCTCT GTATCTGCAAATGAACAGCCTGAGAGCCGAGGA CACGGCTGTGTATTACTGTGCGAGAGATAGAGGC AGCAGCTGGGGCCAGGGAACCCTGGTCACCGTC TCCTCA |
| | | | SEQ ID NO: 24199 | SEQ ID NO: 28205 |
| | | AA | SFELTQPPSVSVSPGQTASITCSGDKLGDKYAYW YQQKPGQSPVLVIYQDRKRPSGIPERFSGSNSGN TATLTISGTQAMDEADYYCQAWDNSTVVFGGG TKLTVL | EVQLVESGGGLVKPGGSLRLSCAASGFTFSSYTMN WVRQAPGKGLEWVSSISGSSSYLYYPDSVKGRFTIS RDNAKNSLYLQMNSLRAEDTAVYYCARDRGSSW GQGTLVTVSS |
| | | | SEQ ID NO: 24200 | SEQ ID NO: 28206 |

FIGURE 50

| | | | | |
|---|---|---|---|---|
| iPS:472732 | 21-225_2B10_LC1 | NA | GATGTTGTGATGACTCAGTCTCCACTCTCCCTG<br>CCCGTCACCCTTGGACAGCCGGCCTCCATCTC<br>CTGCAGGTCTAGTCAAAGCCTCGTATACAGTG<br>ATGGAAACACCTTCTTGAATTGGTTTCAGCAG<br>AGGCCAGGCCAATCTCCAAGGCGCCTAATTTA<br>TAAGGTTTCTAACTGGGACTCTGGGGTCCCAG<br>ACAGATTCAGCGGCAGTGGGTCAGGCACTGGT<br>TTCACACTGAAAATCAGCAGGGTGGAGGCTGA<br>GGATGTTGGGGTTTATTACTGCATACAAGGTA<br>CGCACTGGCCTTTCCCCTTCGGCCAAGGGACA<br>CGACTGGAGATTAAA | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTAGCTATGTCAT<br>GCACTGGGTCCGCCAGGCTCCAGGCAAGGGGCT<br>GGAGTGGGTGGCGGTTATATGGTATGATGGAAGT<br>AATAAATACTATGCAGACTCCGTGAAGGTCCGAT<br>TCACCGTCTCCAGAGACAATTCCAAGAACACGCT<br>GTCTCTGCAAATGAACAGCCTGAGAGCCGAGGA<br>CACGGCTGTGTATTACTGTGCGAGAGAGAGATAT<br>ACCAGTGGCTGGTATGACTACGGTATGGACGTCT<br>GGGGCCAAGGGACCACGGTCACCGTCTCCTCA |
| | | | SEQ ID NO: 24201 | SEQ ID NO: 28207 |
| | | AA | DVVMTQSPLSLPVTLGQPASISCRSSQSLVYSDG<br>NTFLNWFQQRPGQSPRRLIYKVSNWDSGVPDRF<br>SGSGSGTGFTLKISRVEAEDVGVYYCIQGTHWPF<br>PFGQGTRLEIK | QVQLVESGGGVVQPGRSLRLSCAASGFTFSSYVMH<br>WVRQAPGKGLEWVAVIWYDGSNKYYADSVKVRF<br>TVSRDNSKNTLSLQMNSLRAEDTAVYYCARERYTS<br>GWYDYGMDVWGQGTTVTVSS |
| | | | SEQ ID NO: 24202 | SEQ ID NO: 28208 |
| iPS:472733 | 21-225_2B10_LC2 | NA | CAGTCTGCCCTGACTCAGCCTGCCTCCGTGTCT<br>GGGTCTCCTGGACAGTCGATCACCATCTCCTG<br>CACTGGAACCAGCAGTGACGTTGGTGGTTATA<br>ATTTTGTCTCCTGGTACCAACAGCACCCAGAC<br>AAAGCCCCCAAACTCATGATTTATGAGGTCAG<br>TAATCGGCCCTCAGGGGTTTCTAATCGCTTCTC<br>TGGCTCCAAGTCTGGCAACACGGCCTCCCTGA<br>CCATCTCTGGGCTCCAGGCTGAGGACGAGGCT<br>GATTATTACTGCAGCTCATATACAAGCACCGG<br>CACTGTGGTAATCGGCGGAGGGACCAAACTG<br>ACCGTCCTA | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTAGCTATGTCAT<br>GCACTGGGTCCGCCAGGCTCCAGGCAAGGGGCT<br>GGAGTGGGTGGCGGTTATATGGTATGATGGAAGT<br>AATAAATACTATGCAGACTCCGTGAAGGTCCGAT<br>TCACCGTCTCCAGAGACAATTCCAAGAACACGCT<br>GTCTCTGCAAATGAACAGCCTGAGAGCCGAGGA<br>CACGGCTGTGTATTACTGTGCGAGAGAGAGATAT<br>ACCAGTGGCTGGTATGACTACGGTATGGACGTCT<br>GGGGCCAAGGGACCACGGTCACCGTCTCCTCA |
| | | | SEQ ID NO: 24203 | SEQ ID NO: 28209 |

FIGURE 50

| | | | | |
|---|---|---|---|---|
| | | AA | QSALTQPASVSGSPGQSITISCTGTSSDVGGYNFVSWYQQHPDKAPKLMIYEVSNRPSGVSNRFSGSKSGNTASLTISGLQAEDEADYYCSSYTSTGTVVIGGGTKLTVL | QVQLVESGGGVVQPGRSLRLSCAASGFTFSSYVMHWVRQAPGKGLEWVAVIWYDGSNKYYADSVKVRFTVSRDNSKNTLSLQMNSLRAEDTAVYYCARERYTSGWYDYGMDVWGQGTTVTVSS |
| | | | SEQ ID NO: 24204 | SEQ ID NO: 28210 |
| **iPS:473253** | **21-225_7C3_LC1** | NA | GACATCCAGATGACCCAGTCTCCATCCTCCCTGTCTGCATCTGTAGGAGACAGAGTCACCATCACTTGCCGGGCAAGTCAGGGCATTAGAAGTGATTTAGGCTGGTATCAGCAGAATCCAGTCAAAGCCCCTAAGCGCCTGATCTATGCTGCATCCAGTTTGCAAAGTGGGGTCCCATCGAGGTTCAGCGGCAGTGGATCTGGGACAGAATTCACTCTCACAGTCAGCAGCCTGCAGCCTGAAGATTTTGCATTTTATTACTGTCTACAGCATAATAGTTACCTCCCCATCACCTTCGGCCAAGGGACACGACTGGAAATTAAA | CAGGTGCAGCTGGTACAGTCTGGGGCTGAGGTGAAGAAGCCTGGGGCCTCAGTGAAGGTCTCCTGCAAGGCTTCTGGATACACCTTCAACGACTACTATTTGCACTGGGTGCGACAGGCCCCTGGACAAGGTCTTGAGTGGATGGGATGGATCCACCCTAACAGTGGTGGCACAAACTATGCACAGAAGTTTCAGGGCAGGGTCACCATGACCAGGGACACGTCCATCAGCACAGCCTACATGGAGCTGAGCAGCCTGAGATCTGACGACACGGCCGTGTATTCCTGTGCGAGAGATGGTACCAGCTCGTTTGACTACTGGGGCCAGGGAACCCTGGTCACCGTCTCCTCA |
| | | | SEQ ID NO: 24205 | SEQ ID NO: 28211 |
| | | AA | DIQMTQSPSSLSASVGDRVTITCRASQGIRSDLGWYQQNPVKAPKRLIYAASSLQSGVPSRFSGSGSGTEFTLTVSSLQPEDFAFYYCLQHNSYLPITFGQGTRLEIK | QVQLVQSGAEVKKPGASVKVSCKASGYTFNDYYLHWVRQAPGQGLEWMGWIHPNSGGTNYAQKFQGRVTMTRDTSISTAYMELSSLRSDDTAVYSCARDGTSSFDYWGQGTLVTVSS |
| | | | SEQ ID NO: 24206 | SEQ ID NO: 28212 |

FIGURE 50

| | | | | |
|---|---|---|---|---|
| iPS:473254 | 21-225_7C3_LC2 | NA | GACATCCAGATGACCCAGTCTCCATCTTCCGTGTCTGCATCTCTAGGAGACAGAGTCACCATCACTTGTCGGGCGAGTCAGGGTATTAGCAGCTGGTTAGCCTGGTATCAGCAGAAACAAGGGAAAGCCCCTAAGCTCCTGATCTTTGCTGCATCCAGGTTGCAAAGTGGGGCCCCATCAAGGTTCAGCGGCAGTGGATCTGGGACAGATTTCACTCTCACCATCAGCAGCCTGCAGCCTGAAGATTTTGCAACTTACTATTGTCAACAGGCTAACAGTTTCCCATTCACTTTCGGCCCTGGGACCAAAGTGGATATCAAA | CAGGTGCAGCTGGTACAGTCTGGGGCTGAGGTGAAGAAGCCTGGGGCCTCAGTGAAGGTCTCCTGCAAGGCTTCTGGATACACCTTCAACGACTACTATTTGCACTGGGTGCGACAGGCCCCTGGACAAGGTCTTGAGTGGATGGGATGGATCCACCCTAACAGTGGTGGCACAAACTATGCACAGAAGTTTCAGGGCAGGGTCACCATGACCAGGGACACGTCCATCAGCACAGCCTACATGGAGCTGAGCAGCCTGAGATCTGACGACACGGCCGTGTATTCCTGTGCGAGAGATGGTACCAGCTCGTTTGACTACTGGGGCCAGGGAACCCTGGTCACCGTCTCCTCA |
| | | | SEQ ID NO: 24207 | SEQ ID NO: 28213 |
| | | AA | DIQMTQSPSSVSASLGDRVTITCRASQGISSWLAWYQQKQGKAPKLLIFAASRLQSGAPSRFSGSGSGTDFTLTISSLQPEDFATYYCQQANSFPFTFGPGTKVDIK | QVQLVQSGAEVKKPGASVKVSCKASGYTFNDYYLHWVRQAPGQGLEWMGWIHPNSGGTNYAQKFQGRVTMTRDTSISTAYMELSSLRSDDTAVYSCARDGTSSFDYWGQGTLVTVSS |
| | | | SEQ ID NO: 24208 | SEQ ID NO: 28214 |
| iPS:473255 | 21-225_9F12_LC1 | NA | GACATCCAGATGACCCAGTCTCCATCTTCCGTGTCTGCATCTGTAGGAGACAGAGTCACCATCACTTGTCGGGCGAGTCAGGGTATTAGCAGGTGGTTAGCCTGGTATCAGCAGAAACCAGGGAAAGCCCCTAAGCTCCTGATCTATGCTGCATCCAGATTGCAAAGTGGGGTCCCATCAAGGTTCAGCGGCAGTGGATCTGGGACAGATTTCACTCTCACCATCAGCGGCCTGCTGCCTGAAGATTTTGCAATTTATTATTGTCAACAGGCTAACAGTTTCCCATTCACTTTCGGCCCTGGGACCAAAGTGGATTTCAAA | CAGGTGCAGCTGGTACAGTCTGGGGCTGAGGTGAAGAAGCCTGGGGCCTCAGTGAAGGTCTCCTGCAAGGCTTCTGGATACACCTTCGCCGACTACTATTTGCACTGGGTGCGACAGGCCCCTGGACAAGGTCTTGAGTGGATGGGATGGATCCACCCTAACAGTGGTGGCACAAACTTTGCACAGAAGTTTCAGGGCAGGGTCACCATGACCAGGGACACGTCCATCAGCACAGCCTACCTGGAACTGAGCAGTCTGAGATCTGACGACACGGCCTTCTATTACTGTGCGAGAGATGGTACCAGCTCGTTTGACTACTGGGGCCAGGGAACCCTGGTCACCGTCTCCTCA |
| | | | SEQ ID NO: 24209 | SEQ ID NO: 28215 |

FIGURE 50

| | | | | |
|---|---|---|---|---|
| | | AA | DIQMTQSPSSVSASVGDRVTITCRASQGISRWLAWYQQKPGKAPKLLIYAASRLQSGVPSRFSGSGSGTDFTLTISGLLPEDFAIYYCQQANSFPFTFGPGTKVDFK | QVQLVQSGAEVKKPGASVKVSCKASGYTFADYYLHWVRQAPGQGLEWMGWIHPNSGGTNFAQKFQGRVTMTRDTSISTAYLELSSLRSDDTAFYYCARDGTSSFDYWGQGTLVTVSS |
| | | | SEQ ID NO: 24210 | SEQ ID NO: 28216 |
| iPS:473256 | 21-225_9F12_LC2 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGTCAAAGCCCCTAAGCGCCTTATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCTCCCCATCACCTTCGGCCAAGGGACACGACTGGAGATTAAA | CAGGTGCAGCTGGTACAGTCTGGGGCTGAGGTGAAGAAGCCTGGGGCCTCAGTGAAGGTCTCCTGCAAGGCTTCTGGATACACCTTCGCCGACTACTATTTGCACTGGGTGCGACAGGCCCCTGGACAAGGTCTTGAGTGGATGGGATGGATCCACCCTAACAGTGGTGGCACAAACTTTGCACAGAAGTTTCAGGGCAGGGTCACCATGACCAGGGACACGTCCATCAGCACAGCCTACCTGGAACTGAGCAGTCTGAGATCTGACGACACGGCCTTCTATTACTGTGCGAGAGATGGTACCAGCTCGTTTGACTACTGGGGCCAGGGAACCCTGGTCACCGTCTCCTCA |
| | | | SEQ ID NO: 24211 | SEQ ID NO: 28217 |
| | | AA | DIQMTQSPSSLSASVGDRVTITCRASQGIRNDLGWYQQKPVKAPKRLIYAASSLQSGVPSRFSGSGSGTEFTLTISSLQPEDFATYYCLQHNSYLPITFGQGTRLEIK | QVQLVQSGAEVKKPGASVKVSCKASGYTFADYYLHWVRQAPGQGLEWMGWIHPNSGGTNFAQKFQGRVTMTRDTSISTAYLELSSLRSDDTAFYYCARDGTSSFDYWGQGTLVTVSS |
| | | | SEQ ID NO: 24212 | SEQ ID NO: 28218 |

FIGURE 50

| | | | | |
|---|---|---|---|---|
| iPS:472742 | 21-225_30D9_LC2 | NA | TCCTATGAGCTGACTCAGCCACCCTCAGTGTC<br>CGTGTCCCCAGGACAGACAGCCAGCATCACCT<br>GCTCTGGAGATAAATTGGGGGATAAATATGTT<br>TACTGGTTTCAGCAGAAGCCAGGCCAGTCCCC<br>TGTGCTAGTCATCTATCAAGATAGGAAGCGGC<br>CCTCAGGGATCCCTGAGCGATTCTCTGGCTCC<br>AACTCTGGGAACACAGCCACTCTGACCATCAG<br>CGGGACGCAGGCTCTGGATGAGGCTGACTATT<br>ACTGTCAGGCGTGGGACAACAGCACTGCGGTA<br>TTCGGCGGAGGGACCAAGCTGACCGTCCTA | CAGGTGAAACTGGTGCAGTCTGGGGCTGAGGTG<br>GAGAAGCCTGGGGCCTCAGTGAAGGTCTCCTGC<br>AAGGCTTCAGGATACACCTTCACCGGCTACTATC<br>TGCACTGGGTGCGACAGGCCCCTGGACAAGGGC<br>TTGAGTGGATGGGATGGATCAACCCTAACAGTGG<br>TGGCACAAACTATGCACAGAAGTTTCAGGGCAG<br>GGTCACCATGACCAGGGACACGTCCATCAGCAC<br>AGCCTACATGGAGCTGAGCAGGCTGAGATCTGA<br>CGACACGGCCGTGTATTACTGTGCGAGAGTGTAT<br>TACTATGGTTCGGGGAGTTATTATAACGAGTTTG<br>ACAACTGGGGCCAGGGAACCCTGGTCACCGTCTC<br>CTCA |
| | | | SEQ ID NO: 24213 | SEQ ID NO: 28219 |
| | | AA | SYELTQPPSVSVSPGQTASITCSGDKLGDKYVY<br>WFQQKPGQSPVLVIYQDRKRPSGIPERFSGSNSG<br>NTATLTISGTQALDEADYYCQAWDNSTAVFGG<br>GTKLTVL | QVKLVQSGAEVEKPGASVKVSCKASGYTFTGYYL<br>HWVRQAPGQGLEWMGWINPNSGGTNYAQKFQGR<br>VTMTRDTSISTAYMELSRLRSDDTAVYYCARVYYY<br>GSGSYYNEFDNWGQGTLVTVSS |
| | | | SEQ ID NO: 24214 | SEQ ID NO: 28220 |
| iPS:472741 | 21-225_30D9_LC1 | NA | GATGTCGTGATGACTCAGTCTCCACTCTCCCTG<br>CCCGTCACCCTTGGACAGCCGGCCTCCATCTC<br>CTGCAGGTCTAGTCAAAGCCTCGTATCCAGTG<br>ATGGAAACACCTTCTTGAATTGGTTTCAGCAG<br>AGGCCAGGCCAATCTCCAAGGCGCCTAATTTA<br>TAAGGTTTCTAACTGGGACTCTGGGGTCCCAG<br>ACAGATTCAGCGGCAGTGGGTCAGGCACTGAT<br>TTCACACTGAAAATCAGCAGGTTGGAGGCTGA<br>GGATGTTGGGGTTTATTACTGCTTGCAAGGTA<br>CACACTGGCCTCTCACCTTCGGCCAAGGGACA<br>CGACTGGAGATTAAA | CAGGTGAAACTGGTGCAGTCTGGGGCTGAGGTG<br>GAGAAGCCTGGGGCCTCAGTGAAGGTCTCCTGC<br>AAGGCTTCAGGATACACCTTCACCGGCTACTATC<br>TGCACTGGGTGCGACAGGCCCCTGGACAAGGGC<br>TTGAGTGGATGGGATGGATCAACCCTAACAGTGG<br>TGGCACAAACTATGCACAGAAGTTTCAGGGCAG<br>GGTCACCATGACCAGGGACACGTCCATCAGCAC<br>AGCCTACATGGAGCTGAGCAGGCTGAGATCTGA<br>CGACACGGCCGTGTATTACTGTGCGAGAGTGTAT<br>TACTATGGTTCGGGGAGTTATTATAACGAGTTTG<br>ACAACTGGGGCCAGGGAACCCTGGTCACCGTCTC<br>CTCA |
| | | | SEQ ID NO: 24215 | SEQ ID NO: 28221 |

FIGURE 50

| | | | | |
|---|---|---|---|---|
| | | AA | DVVMTQSPLSLPVTLGQPASISCRSSQSLVSSDGNTFLNWFQQRPGQSPRRLIYKVSNWDSGVPDRFSGSGSGTDFTLKISRLEAEDVGVYYCLQGTHWPLTFGQGTRLEIK | QVKLVQSGAEVEKPGASVKVSCKASGYTFTGYYLHWVRQAPGQGLEWMGWINPNSGGTNYAQKFQGRVTMTRDTSISTAYMELSRLRSDDTAVYYCARVYYYGSGSYYNEFDNWGQGTLVTVSS |
| | | | SEQ ID NO: 24216 | SEQ ID NO: 28222 |
| iPS:472743 | 21-225_68G6 | NA | TCCTATGAGGTGACTCAGCCACCCTCAGTGTCCGTGTCCCCAGGACAGACAGCCAGCATCACCTGCTCTGGAGATAAATTGGGGGATAAATATACTTACTGGTATCAGCAGAAGGCAGGCCAGTCCCCTTTCCTGGTCATCTATCAAGATAGGAAGCGGCCCTCAGGGATCCCTGACCGATTCTCTGGCTCCAACTCTGGGAACACAGCCACTCTGACCATCAGCGGGACCCAGGCTATGGATGCGGCTGACTTTTACTGTCAGGCGTGGGACAATAGTACTGCGGTATTCGGCGGAGGGACCAAGCTGACCGTCCTA | CAGGTGCAGTTGGTGCAGTTTGGGGGTGAGGTGAAGAAGCCTGGGTCCTCAGTGAAGGTNTCCTGCAAGGCTTCAGGATACACCTTCACCGGYTACTATATGCACTGGGTGCGACAGGCCCCTGGACAAGGGCTTGAGTGGATGGGATCGATCTACCGTAACAGTGGTGGCACAAATTATGCACAGAAGTTTCAGGGCAGGGTCACCATGACCAGGGACAAGTCCATCAGCACCGCCTACATGGAGAAGAGCAGGATCAGATCTGATGACACGGCCGTGTATTACTGTGCGAGAGCCTTTTACTATGGTTCGGGGACTTATTATAACGAATTTGACTACTGGGGCCAGGGAACCCTGGTCACCGTCTCCTCA |
| | | | SEQ ID NO: 24217 | SEQ ID NO: 28223 |
| | | AA | SYEVTQPPSVSVSPGQTASITCSGDKLGDKYTYWYQQKAGQSPFLVIYQDRKRPSGIPDRFSGSNSGNTATLTISGTQAMDAADFYCQAWDNSTAVFGGGTKLTVL | QVQLVQFGGEVKKPGSSVKVSCKASGYTFTGYYMHWVRQAPGQGLEWMGSIYRNSGGTNYAQKFQGRVTMTRDKSISTAYMEKSRIRSDDTAVYYCARAFYYGSGTYYNEFDYWGQGTLVTVSS |
| | | | SEQ ID NO: 24218 | SEQ ID NO: 28224 |

FIGURE 50

| | | | | |
|---|---|---|---|---|
| **iPS:392573** | **21-225_15G2** | NA | CAGTCTGCCCTGACTCAGCCTGCCTCCGTGTCTGGGTCTCCTGGACAGTCGATCACCATCTCCTGCACTGGAACCAGCAGTGACGTTGGTGGTTATAACTATGTCTCCTGGTACCAACAGCACCCAGGCAAAGCCCCCAAACTCATGATTTATGAGGTCAGTAATCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCACCTCATATACAAGCACCAGCACTGTGGTCTTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGATTCTCACTCAGCACTAGTGGAGTGGGTGTGGGCTGGATCCGTCAGCCCCCAGGAAAGGCCCTGGAGTGGCTTGCACTCATTTATTGGAATGATGATAAGCGCTACAGCCCATCTCTGAAGAGCAGGCTCACCATCACCAAGGACACCTCCAAAAACCAGGTGGTCCTTACAATGACCAACATGGACCCTGTGGACACAGCCACATATTACTGTGCAGACACCGGTGTCAGCTGCTGCTATTTTCACTATTGGGGCCAGGGAACCCTGGTCACCGTCTCCTCA |
| | | | SEQ ID NO: 24219 | SEQ ID NO: 28225 |
| | | AA | QSALTQPASVSGSPGQSITISCTGTSSDVGGYNYVSWYQQHPGKAPKLMIYEVSNRPSGVSNRFSGSKSGNTASLTISGLQAEDEADYYCTSYTSTSTVVFGGGTKLTVL | QITLKESGPTLVKPTQTLTLTCTFSGFSLSTSGVGVGWIRQPPGKALEWLALIYWNDDKRYSPSLKSRLTITKDTSKNQVVLTMTNMDPVDTATYYCADTGVSCCYFHYWGQGTLVTVSS |
| | | | SEQ ID NO: 24220 | SEQ ID NO: 28226 |
| **iPS:392583** | **21-225_10B10** | NA | TCCTATGAGCTGACTCAGCCACCCTCAGTGTCCGTGTCCCCAGGACAGACAGCCAGCATCACCTGCTCTGGAGATAAATTGGGGAATAAATATGCTTGGTGGTATCAGCAGAAGCCAGGCCAGTCCCCTGTGCTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAACAGCACTGTGGTTTTCGGCGGAGGGACCAAACTGACCGTCCTA | CAGATCACCTTGAAGGAGTCTGGTCCTACGCTGGTGAAACCCACACAGACCCTCACGCTGACCTGCACCTTCTCTGGGTTCTCACTCAGCACTGGTGGAGTGGGTGTGGGCTGGATCCGTCAGCCCCCAGGAAAGGCCCTGGAGTGGCTTGTATTCATTTATTGGAGTGATGATAAGCGCTACAGCCCATCTCTGAAGAGCAGGCTCTCCATCACCAAGGACACCTCCAAAAACCAGGTGGTCCTTACAATGACCAACATGGACCCTGTGGACACAGCCACATATTACTGTGCACGTATAGCAGCAGTTGCCTTTGACTACTGGGGCCAGGGAACCCTGGTCACCGTCTCCTCA |
| | | | SEQ ID NO: 24221 | SEQ ID NO: 28227 |

FIGURE 50

| | | | | |
|---|---|---|---|---|
| | | AA | SYELTQPPSVSVSPGQTASITCSGDKLGNKYAWWYQQKPGQSPVLVIYQDRKRPSGIPERFSGSNSGNTATLTISGTQAMDEADYYCQAWDNSTVVFGGGTKLTVL | QITLKESGPTLVKPTQTLTLTCTFSGFSLSTGGVGVGWIRQPPGKALEWLVFIYWSDDKRYSPSLKSRLSITKDTSKNQVVLTMTNMDPVDTATYYCARIAAVAFDYWGQGTLVTVSS |
| | | | SEQ ID NO: 24222 | SEQ ID NO: 28228 |
| **iPS:392585** | **21-225_14H11** | NA | ACCTATGAGCTGACTCAGCCATCCTCAGTGTCCGTGTCCCCAGGACAGACAGCCAGCATCACCTGCTCTGGAGATAAATTGGGGGAAAAATATGTTTGCTGGTATCAGCAGAAGCCAGGCCAGTCCCCTGTGCTGGTCATATATCAAGATACCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTATATTCGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGCCAGGGTTCTGGATACACCTTCACCGGCCACTATATGTGCTGGGTGCGACAGGCCCCTGGACAAGGGCTTGAGTGGATGGGATGGATCAACCCTAATAGTGGTGGCACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGGCTGGATATTGTAGTAGTTCCAGCTGCTATTTGCAACCGGGTTATTACGGTATGGACGTCTGGGGCCAAGGGACCACGGTCACCGTCTCCTCA |
| | | | SEQ ID NO: 24223 | SEQ ID NO: 28229 |
| | | AA | TYELTQPSSVSVSPGQTASITCSGDKLGEKYVCWYQQKPGQSPVLVIYQDTKRPSGIPERFSGSNSGNTATLTISGTQAMDEADYYCQAWDSSTIFGGGTKLTVL | QVQLVQSGAEVKKPGASVKVSCQGSGYTFTGHYMCWVRQAPGQGLEWMGWINPNSGGTNYAQKFQGRVTMTRDTSISTAYMELSRLRSDDTAVYYCAAGYCSSSSCYLQPGYYGMDVWGQGTTVTVSS |
| | | | SEQ ID NO: 24224 | SEQ ID NO: 28230 |

FIGURE 50

| | | | | |
|---|---|---|---|---|
| **iPS:392587** | **21-225_18G5** | NA | TCCTATGAGCTGACTCAGCCACCCTCAGTGTCCGTGTCCCCAGGACAGACAGCCAGCATCACCTGCTCTGGAGAGAAATTGGGGGATAAATATGTTTGTTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGAACAGCAGCAATGTGGTATTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGGTTCTCACTCAGCACTAGTGGAGTGGGTGTGGGCTGGATCCGTCAGCCCCCAGGAAAGGCCCTGGAGTGCCTTTCACTCATTTATTGGAATGATGATAAGGTCTACAGCCCATCTCTGAAGAGCAGGCTCACCATCACCAAGTACACCTCCAAAAACCAGGTGGTCCTTACAATGACCAACATGGACCCTGTGGACACAGCCACATATTACTGTGCACACAGGGGACAGCAGCTGGCCCTCGACTACTGGGGCCAGGGAACCCTGGTCACCGTCTCCTCA |
| | | | SEQ ID NO: 24225 | SEQ ID NO: 28231 |
| | | AA | SYELTQPPSVSVSPGQTASITCSGEKLGDKYVCWYQQKPGQSPVLVIYQDSKRPSGIPERFSGSNSGNTATLTISGTQAMDEADYYCQAWNSSNVVFGGGTKLTVL | QITLKESGPTLVKPTQTLTLTCTFSGFSLSTSGVGVGWIRQPPGKALECLSLIYWNDDKVYSPSLKSRLTITKYTSKNQVVLTMTNMDPVDTATYYCAHRGQQLALDYWGQGTLVTVSS |
| | | | SEQ ID NO: 24226 | SEQ ID NO: 28232 |
| **iPS:392589** | **21-225_27H2** | NA | TCCTATGAGCTGACTCAGCCACCCTCAGTGTCCGTATCCCCAGGACAGACAGCCAGCATCACCTGCTCTGGAGATAAATTGGGGGATAAATATGCTTCCTGGTATCAGCAGAAGCCAGGCCAGTCCCCTGTTTTGGTCATCTATCAAGATGGCAAGCGGCCCTCAGGGATCCCTGAGCGATTCTCTGGCTCCAACTCTGGGAACACAGCCACTCTGACCCTCAGCGGGACCCAGGCTATGGATGAGGCTGACTATTACTGTCAGGCGTGGGACAGCAGCACTTATGTGGTATTCGGCGGAGGGACCAAGCTGACCGTCCTA | CAGGTGCAGTTGGTGGAGTCTGGGGGAGGCGTGGTCCAGCCTGGGAGGTCCCTGAGACTCTCCTGTGCAGCGTCTGGATTCACCTTCAGTGGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTTTCTGCAAATGAACAGCCTGAGAGCCGAGGACACGGCTGTGTATTACTGTGCGAGAGATAGGGTATATTGTAGTAGTACCAGCTGCTCCCCTTACTACTACTACTACGGTATGGACGTCTGGGGCCAAGGGACCACGGTCACCGTCTCCTCA |

FIGURE 50

| | | | | |
|---|---|---|---|---|
| | | | SEQ ID NO: 24227 | SEQ ID NO: 28233 |
| | | AA | SYELTQPPSVSVSPGQTASITCSGDKLGDKYASWYQQKPGQSPVLVIYQDGKRPSGIPERFSGSNSGNTATLTLSGTQAMDEADYYCQAWDSSTYVVFGGGTKLTVL | QVQLVESGGGVVQPGRSLRLSCAASGFTFSGYGMHWVRQAPGKGLEWVAVIWYDGSNKYYADSVKGRFTISRDNSKNTLFLQMNSLRAEDTAVYYCARDRVYCSSTSCSPYYYYYGMDVWGQGTTVTVSS |
| | | | SEQ ID NO: 24228 | SEQ ID NO: 28234 |
| **iPS:392593** | **21-225_3E10** | NA | TCCTATGAGCTGACTCAGCCACACTCAGTGTCAGTGGCCACAGCACAGATGGCCAGGATCACCTGTGGGGGAAACAACATTGGAAGTAAAGCTGTGCACTGGTACCAGCAAAAGCCAGGCCAGGACCCTGTGCTGGTCATCTATAGCGATAGCAACCGGCCCTCAGGGATCCCTGAGCGATTCTCTGGCTCCAACCCAGGGAACACCGCCACCCTAACCATCAGCAGGATCGAGGCTGGGGATGAGGCTGACTATTACTGTCAGGTGTGGGACAGTAGTAGTGATCATGTGGTATTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGGTTCTCACTCAATACTGGTGGAGTGGGTGTGGGCTGGATCCGTCAGCCCCCAGGAAAGGCCCTGGAGTGGCTTGCACTCATTTATTGGAATGATGATAAGCGCCACAGCCCATCTCTGAAGAGCAGGCTCACCATCACCAAAGACACCTCCAAAAACCAGGTGGTCCTTACAATGACCCACATGGCCCCTGTGGACACAGCCACATATTACTGTGCACACCTTATAGAAGTGGCCTTTGACTATTGGGGCCAGGGAACCCTGGTCACCGTCTCCTCA |
| | | | SEQ ID NO: 24229 | SEQ ID NO: 28235 |
| | | AA | SYELTQPHSVSVATAQMARITCGGNNIGSKAVHWYQQKPGQDPVLVIYSDSNRPSGIPERFSGSNPGNTATLTISRIEAGDEADYYCQVWDSSSDHVVFGGGTKLTVL | QITLKESGPTLVKPTQTLTLTCTFSGFSLNTGGVGVGWIRQPPGKALEWLALIYWNDDKRHSPSLKSRLTITKDTSKNQVVLTMTHMAPVDTATYYCAHLIEVAFDYWGQGTLVTVSS |
| | | | SEQ ID NO: 24230 | SEQ ID NO: 28236 |

FIGURE 50

| | | | | |
|---|---|---|---|---|
| **iPS:392596** | **21-225_12D8** | NA | CTGCCTGTGCTGACTCAGCCCCCGTCTGCATCTGCCTTGCTGGGAGCCTCGATCAAGCTCACCTGCACCCTAAGCAGTGAGCACAGCACCTACACCATCGAATGGTATCAACAGAGACCAGGGAGGTCCCCCCAGTATATAATGAAGGTTAAGAGTGATGGCAGCCACAGCAAGGGGGACGGGATCCCCGATCGCTTCATGGGCTCCAGTTCTGGGGCTGACCGCTACCTCACCTTCTCCAACCTCCAGTCTGACGATGAGGATGAGTATCACTGTGGAGAGAGCCACACGATTGATGGCCAAGTCGGTGTGGTATTCGGCGGAGGGACCAAGCTGACCGTCCTA | GAGGTGCAGTTGTTGGAATCTGGGGGAGGCTTGGTACAGCCTGGGGGGTCCCTGAGACTCTCCTGTGCAGCCTCTGGATTCACCTTTAGCAGCTATGTCATGAGCTGGGTCCGCCAGGCTCCAGGGAAGGGGCTGGAGTGGGTCTCAACTATTAGTGTTGGTGGTGGTAGCACATACTACGCAGACTCCGTGAAGGGCCGGTTCACCATCTCCAGAGACAATTCCAAGACCACGCTGTATCTGCAAATGAACAGCCTGAGAGCCGAGGACACGGCCGTATATTACTGTGCGAAATGGGGACGTGGATACAGCTATGAATACTACTACGGTATGGACGTCTGGGGCCAAGGGACCACGGTCACCGTCTCCTCA |
| | | | SEQ ID NO: 24231 | SEQ ID NO: 28237 |
| | | AA | LPVLTQPPSASALLGASIKLTCTLSSEHSTYTIEWYQQRPGRSPQYIMKVKSDGSHSKGDGIPDRFMGSSSGADRYLTFSNLQSDDEDEYHCGESHTIDGQVGVVFGGGTKLTVL | EVQLLESGGGLVQPGGSLRLSCAASGFTFSSYVMSWVRQAPGKGLEWVSTISVGGGSTYYADSVKGRFTISRDNSKTTLYLQMNSLRAEDTAVYYCAKWGRGYSYEYYYGMDVWGQGTTVTVSS |
| | | | SEQ ID NO: 24232 | SEQ ID NO: 28238 |
| **iPS:392598** | **21-225_18E10** | NA | TCCTATGAACTGACGCAGCCACCCTCAGTGTCCGTGTCCCCAGGACAGACAGCCAGCATCACCTGCTCTGGAGATAGATTGGGGGATAAATATGCTTGGTGGTATCAGCAGAAGCCAGGCCAGTCCCCTGTGCTGGTCATATATCAAGATCGCAAGCGGCCCTCAGGGATCCCTGAGCGATTCTCTGGCTCCAATTCAGGGAACACAGCCACTCTGACCATCAGCGGGACCCAGGCTATGGATGAGGCTGACTATTACTGTCAGGCGTGGGACAGCAGCACAGTGGTATTCGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGGTGGATCAACCCTAACAGTGGTGGCACAAACTATGCACAGAAGTTTCAGGGCAGGGTCACCATGACCAGGGACTCGTCCATCAACACAGCCTACATGGAGCTGAGCAGGCTGAGATCTGACGACACGGCCGTGTATTACTGTGCGAGGTCGTACTACTATGGTTCGGGGAGTTATTATAACGAGTTTGACTACTGGGGCCAGGGAACCCTGGTCACCGTCTCCTCA |
| | | | SEQ ID NO: 24233 | SEQ ID NO: 28239 |

FIGURE 50

| | | | | |
|---|---|---|---|---|
| | | AA | SYELTQPPSVSVSPGQTASITCSGDRLGDKYAWWYQQKPGQSPVLVIYQDRKRPSGIPERFSGSNSGNTATLTISGTQAMDEADYYCQAWDSSTVVFGGGTKLTVL | QVQLVQSGAEVKKPGASVKVSCKASGYTFTGYYMHWVRQAPGQGLEWMGWINPNSGGTNYAQKFQGRVTMTRDSSINTAYMELSRLRSDDTAVYYCARSYYYGSGSYYNEFDYWGQGTLVTVSS |
| | | | SEQ ID NO: 24234 | SEQ ID NO: 28240 |
| **iPS:392618** | **21-225_16F10** | NA | GATATTGTGATGACCCAGACTCCACTCTCTCTGTCCGTCATTCCTGGACAGCCGGCCTCCATCTCCTGCAAGTCTAGTCAGAGCCTCCTGCATAGTGATGGAAAGACCCATTTGAATTGGTACCTGCAGAAGCCAGGCCAGCCTCCACAGCTCCTGATCTATGAAGTTTCCTACCGGTTCTCTGGAGTGCCAGATAGGTTCAGTGGCAGCGGGTCAGGGACAGTTTTCACACTGGAGATCAGCCGGGTGGAGGCTGCGGATGTTGGGGTTTATTACTGCTTTCAAAGTATACAGCTTCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTCATATGGTATGATGGAAATAATAAATACTATGTAGACTCCGTGAAGGGCCGATTCACCATCTCCAGAGACAATTCCAAGAATACGCTGTATCTGCAAATGAACAGCCTGAGAGCCGAGGACACGGCTGTGTATTACTGTGCGAGAGAGCTTGCCTGGTACGAGGACTACTGGGGCCAGGGAACCCTGGTCACCGTCTCCTCA |
| | | | SEQ ID NO: 24235 | SEQ ID NO: 28241 |
| | | AA | DIVMTQTPLSLSVIPGQPASISCKSSQSLLHSDGKTHLNWYLQKPGQPPQLLIYEVSYRFSGVPDRFSGSGSGTVFTLEISRVEAADVGVYYCFQSIQLPLTFGGGTKVEIK | QVQLVESGGGVVQPGRSLRLSCAASGFTFSDYGMHWVRQAPGKGLEWVAVIWYDGNNKYYVDSVKGRFTISRDNSKNTLYLQMNSLRAEDTAVYYCARELAWYEDYWGQGTLVTVSS |
| | | | SEQ ID NO: 24236 | SEQ ID NO: 28242 |

FIGURE 50

| | | | | |
|---|---|---|---|---|
| **iPS:392620** | **21-225_17H5** | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAACCAGGGAAAGCCCCTAAGTCCCTGATCAATGCTGCATCCAGTTTACAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATCATAGTTACCCATTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTAGTAGTAGTACTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGTGGCTTCATTTGACTACTGGGGCCAGGGAACCCTGGTCACCGTCTCCTCA |
| | | | SEQ ID NO: 24237 | SEQ ID NO: 28243 |
| | | AA | DIQMTQSPSSLSASVGDRVTITCRASQGISNYLAWFQQKPGKAPKSLINAASSLQSGVPSKFSGSGSGTDFTLTISSLQPEDFATYYCQQYHSYPFTFGPGTKVDIK | EVQLVESGGGLVKPGGSLRLSCAASGFTFSSYSMNWVRQAPGKGLEWVSSISSSSTYIYYADSVKGRFTISRDNAKNSLYLQMNSLRAEDTAVYYCARVASFDYWGQGTLVTVSS |
| | | | SEQ ID NO: 24238 | SEQ ID NO: 28244 |
| **iPS:392622** | **21-225_17H8** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGGTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTACAACTTATTACTGTCTACAGCATAATAGTTACCCACTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGTAGAAGTAGTTACTACTGGGGCTGGATCCGCCAGCCCCCAGGGAGGGGGCTGGAGTGGATTGGGAATATCTATTATGGTGGGAACACCTACTACAACCCGTCCCTCAAGAGTCGAGTCACCATATCCGTAGACACGTCCAAGAACCAGTTCTCCCTGAAGCTGAGCTCTGTGACCGCCGCAGACACGGCTGTGTATTACTGTGCGAGACATGGAAAAGACTGGGGCCTTGACTACTGGGGCCAGGGAACCCTGGTCACCGTCTCCTCA |
| | | | SEQ ID NO: 24239 | SEQ ID NO: 28245 |

FIGURE 50

| | | | | |
|---|---|---|---|---|
| | | AA | DIQMTQSPSSLSASVGDRVTITCRASQGIRNDLGWYQQKPGKAPKRLIYGASSLQSGVPSRFSGSGSGTEFTLTISSLQPEDFTTYYCLQHNSYPLTFGGGTKVEIK | QLQLQESGPGLVKPSETLSLTCTVSGGSISRSSYYWGWIRQPPGRGLEWIGNIYYGGNTYYNPSLKSRVTISVDTSKNQFSLKLSSVTAADTAVYYCARHGKDWGLDYWGQGTLVTVSS |
| | | | SEQ ID NO: 24240 | SEQ ID NO: 28246 |
| **iPS:392624** | **21-225_17H12** | NA | GACATCCAGATGACCCAGTCTCCATCCTCCCTGTCTGCATCTGTAGGAGACAGAGTCACCATCACTTGCCGGGCAAGTCAGGACATTAGAAATGATTTAGGCTGGTATCAGCAGAAAGCAGGGAAAGCCCCTAAGCGCCTGATCAATGCTGCATCCAGTTTGCAAAGTGGGGTCCCATCAAGGTTCAGCGGCATTGGCTCTGGGACAGAATTCACTCTCACAATCACCGGCCTGCAGCCTGAAGATTTTGCAACTTATTACTGTCTACAGCATTATAGTTATATGTTCACTTTCGGCGGAGGGACCAAGGTGGAGATCAAA | GAGGTGCAACTGGTGGAGTCTGGGGGAGGCCTGGTCAAGCCTGGGGGGTCCCTGAGACTCTCCTGTGCAGCCTCTGGATTCACCTTCAGTAGCTATACCATGAACTGGGTCCGCCAGGCTCCAGGGAAGGGGCTGGAGTGGGTCTCATCCATTAGTGGTAGTAGTAGTTACATATACTACGCAGACTCAGTGAAGGGCCGATTCACCATCTCCAGAGACAACGCCAAGAACTCACTGTATTTGCAAATGAACAGCCTGAGAGCCGAGGACACGGCTGTATATTACTGTGCGAGAGATCGAGGCTCCATCTGGGGCCAAGGGACAATGGTCACCGTCTCTTCA |
| | | | SEQ ID NO: 24241 | SEQ ID NO: 28247 |
| | | AA | DIQMTQSPSSLSASVGDRVTITCRASQDIRNDLGWYQQKAGKAPKRLINAASSLQSGVPSRFSGIGSGTEFTLTITGLQPEDFATYYCLQHYSYMFTFGGGTKVEIK | EVQLVESGGGLVKPGGSLRLSCAASGFTFSSYTMNWVRQAPGKGLEWVSSISGSSSYIYYADSVKGRFTISRDNAKNSLYLQMNSLRAEDTAVYYCARDRGSIWGQGTMVTVSS |
| | | | SEQ ID NO: 24242 | SEQ ID NO: 28248 |

FIGURE 50

| | | | | |
|---|---|---|---|---|
| iPS:392626 | 21-225_18A5 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCTTGCAGCCTGAAGATTTTGCAACTTATTACTGTCTACAGCATAATAGTTACCCGTGGACGTTCGGCCAAGGGACCAAGGTGGAAATCAAA | CAGGTGCAGTTGGTGGAGTCTGGGGGAGGCGTGGTCCAGCCTGGGAGGTCCCTGAGACTCTCCTATACAGCGTCTGGATTCACCTTCAGTGACTATGGCATGCACTGGGTCCGCCAGGCTCCAGGCAAGGGGCTGGAGTGGGTGGCAGTTATATGGTTTGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCTTGGCTGGACGGAAGAGTACTGGGGCCAGGGAACCCTGGTCACCGTCTCCTCA |
| | | | SEQ ID NO: 24243 | SEQ ID NO: 28249 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPWTFGQGTKVEIK | QVQLVESGGGVVQPGRSLRLSYTASGFTFSDYGMHWVRQAPGKGLEWVAVIWFDGSNKYYADSVKGRFTISRDNSKNTLYLQMNSLRAEDTAVYYCARDLGWTEEYWGQGTLVTVSS |
| | | | SEQ ID NO: 24244 | SEQ ID NO: 28250 |
| iPS:392628 | 21-225_20C2 | NA | GACATCCAGATGACCCAGTCTCCATCCTCCCTGTCTGCATCTGTACAAGACAGAGTCACCATCACTTGCCGGGCAAGTCAGGGCATTAGAAATGATTTAGGCTGGTATCAGCAGAAACCAGGGAAAGCCCCTAAGCGCCTGATCTTTGCTGCATCCAGTTTGCAAAGTGGGGTCCCGTCAAGGTTCAGCGGCAGTGGATCTGGGACAGAATTCACTCTCACAATCAGCAGCCTGCAGCCTGAAGATTTTGCAACTTATTACTGTCTTCAACATGCTAGTTACCCGCTCACTTTCGGCGGAGGGACCAAGGTGGAGATCGAA | CAGCTGCAGCTGCAGGAGTCGGGCCCAGGACTGGTGAAGCCTTCGGAGACCCTGTCCCTCACCTGCACTGTCTCTGGTGGCTCCATCAGCAGAAGTAGTTACTACTGGGGCTGGATCCGCCAGCCCCCAGGGAAGGGGCTGGAGTGGATTGGGAATATCTATTATAGTGGGACTGCCTACTGTAATTCGTCCCTCAAGAGTCGAGTCATTATATCCGTAGACACGTCCAAGAACCAGTTCTCCCTGAAGCTGAGCTCTGTGACCGCCACAGACACGGCTGTGTATTACTGTGCGAGACATAGTAGCAGCTGGTCCCTTGACAACTGGGGCCAGGGAACCCTGGTCACCGTCTCCTCA |
| | | | SEQ ID NO: 24245 | SEQ ID NO: 28251 |

FIGURE 50

| | | | | |
|---|---|---|---|---|
| | | AA | DIQMTQSPSSLSASVQDRVTITCRASQGIRNDLGWYQQKPGKAPKRLIFAASSLQSGVPSRFSGSGSGTEFTLTISSLQPEDFATYYCLQHASYPLTFGGGTKVEIE | QLQLQESGPGLVKPSETLSLTCTVSGGSISRSSYYWGWIRQPPGKGLEWIGNIYYSGTAYCNSSLKSRVIISVDTSKNQFSLKLSSVTATDTAVYYCARHSSSWSLDNWGQGTLVTVSS |
| | | | SEQ ID NO: 24246 | SEQ ID NO: 28252 |
| iPS:392630 | 21-225_20E5 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATTTGGTATGAAGAAAATAATCAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGTTCCGGTCTGACTACTGGGGCCAGGGAACCCTGGTCACCGTCTCCTCA |
| | | | SEQ ID NO: 24247 | SEQ ID NO: 28253 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVEIK | QVQLVESGGGVVQPGRSLRLSCAASGFTFSDYGMHWVRQAPGKGLEWVAVIWYEENNQYYADSVKGRFTISRDNSKNTLYLQMNSLRAEDTAVYYCARELGFRSDYWGQGTLVTVSS |
| | | | SEQ ID NO: 24248 | SEQ ID NO: 28254 |

FIGURE 50

| | | | | |
|---|---|---|---|---|
| **iPS:392632** | **21-225_16A11** | NA | GACATCCAGATGACCCAGTCTCCATCCTCCCTGTCTGCATCTGTCGGAGACAGTGTCACCATCTCTTGCCGGGCAAGTCAGGACATTAGAAATCATTTAGGCTGGTATCAGCGTAACCCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGACTTTGCAACTTATTACTGTCTACAGTATAATAGTTATCCATTCACTTTCGGCCCTGGGACCAAAGTGGACATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTAGTCTCATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGTAGCAGCCTTTGACTACTGGGGCCAGGGAACCCTGGTCACCGTCTCCTCA |
| | | | SEQ ID NO: 24249 | SEQ ID NO: 28255 |
| | | AA | DIQMTQSPSSLSASVGDSVTISCRASQDIRNHLGWYQRNPGKAPKRLIYAASSLQSGVPSRFSGSGSGTEFTLTISSLQPEDFATYYCLQYNSYPFTFGPGTKVDIK | EVQLVESGGGLVKPGGSLRLSCAASGFTFSSYSMNWVRQAPGKGLEWVSSISGSSSLIYYADSVKGRFTISRDNAKNSLYLQMNSLRAEDTAVYYCARVAAFDYWGQGTLVTVSS |
| | | | SEQ ID NO: 24250 | SEQ ID NO: 28256 |
| **iPS:392634** | **21-225_17H3** | NA | GACATCCAGATGACCCAGTCTCCATCCTCCCTGTCTGCATCTGTAGGAGACAGAGTCACCTTCACTTGCCGGGCAAGTCAGGGCATTAGAAGTGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCAATATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTGTGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAAGTATAGCAGCAGCTGGTACGACTACGGTATGGACGTCTGGGGCCAAGGGACCACGGTCACCGTCTCCTCA |
| | | | SEQ ID NO: 24251 | SEQ ID NO: 28257 |

FIGURE 50

| | | | | |
|---|---|---|---|---|
| | | AA | DIQMTQSPSSLSASVGDRVTFTCRASQGIRSDLGWYQQKPGKAPKRLIYAASSLQSGVPSRFSGSGSGTEFTLTISSLQPEDFATYYCLQQYSYPRTFGQGTKVEIK | QVQLVESGGGVVQPGRSLRLSCAASGFTFSNCVMHWVRQAPGKGLEWVAVIWYDGSNKYYADSVKGRFTISRDNSKNTLYLQMNSLRAEDTAVYYCAREKYSSSWYDYGMDVWGQGTTVTVSS |
| | | | SEQ ID NO: 24252 | SEQ ID NO: 28258 |
| **iPS:392636** | **21-225_17A6** | NA | GACATCCAGATGACCCAGTCTCCATCCTCCCTGTCTGCATCTGTCGGAGACAGAGTCACCATCACTTGCCGGGCAAGTCAGACCATTAGCAACTATTTAAATTGGTATCATCAGAAACCAGGGAAAGCCCCTAAGCTCCTGATCTATGCTGCTTCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTCACACTTCCCCGCTCACTTTCGGCGGAGGGACCAAGGTGGAGATCAAA | CAGGTACAGCTGCAGCAGTCAGGTCCAGGACTGGTGAAGCCCTCGCAGACCCTCTCACTCACCTGTGCCATCTCCGGGGACAGTGTCTCTCGCAACACTGCTGCTTGGAGCTGGATCAGGCAGTCCCCATCGAGAGGCCTTGAGTGGCTGGGAAGGACATACTACAGGTCCAAGTGGTATAATGATTATGCAGTATCTGTGAAAAGTCGAGTAACCATCAACCCAGACACATCCAAGAACCAGTTCTCCCTGCAGCTGAACTCTGTGACTCCCGAGGACACGGCTGTGTATTACTGTGCAAGAGTAAGCAGTGGCTGGTCCCATCACTACTACTACTACGGTATGGACGTCTGGGGCCAAGGGACCACGGTCACCGTCTCCTCA |
| | | | SEQ ID NO: 24253 | SEQ ID NO: 28259 |
| | | AA | DIQMTQSPSSLSASVGDRVTITCRASQTISNYLNWYHQKPGKAPKLLIYAASSLQSGVPSRFSGSGSGTDFTLTISSLQPEDFATYYCQQSHTSPLTFGGGTKVEIK | QVQLQQSGPGLVKPSQTLSLTCAISGDSVSRNTAAWSWIRQSPSRGLEWLGRTYYRSKWYNDYAVSVKSRVTINPDTSKNQFSLQLNSVTPEDTAVYYCARVSSGWSHHYYYYGMDVWGQGTTVTVSS |
| | | | SEQ ID NO: 24254 | SEQ ID NO: 28260 |

FIGURE 50

| | | | | |
|---|---|---|---|---|
| **iPS:392638** | **21-225_17F9** | NA | GACATCCAGATGACCCAGTCTCCATCCTCCCTGTCTGCATCTGTAGGAGACAGAGTCACCATCACTTGCCGGGCAAGTCAGGTCATTAGAAATGATTTAGGCTGGTATCAGCAGAAACCAGGGAAAGCCCCTAAGCGCCTGATCTATGCTGTATCCAGTTTGCAAAGTGGGGTCCCATCAAGGTTCAGCGGCAGTGGATCTGGGACAGAATTCACTCTCACAATCAACAGCCTGCAGCCTGAAGATTTTGCAACTTATTACTGTCTACAACATAATACTTATCCGCTCACTTTCGGCGGCGGGACCAAGGTGGAGTTCAAA | CAGCTGCAGCTGCAGGAGTCGGGCCCAGGACTGGTGAAGCCTTCGGAGACCCTGTCCCTCACCTGCACTGTCTCTGGCGGCTCCATCAGCAGAAGTAGTTACTATTGGGGCTGGATCCGCCAGCCCCCAGGGAAGGGCCTGGAGTGGATTGGGAATATCTATTATAGTGGGAGCACCTACTACAATCCGTCCCTCAAGAGTCGAGTCACCATATCCGTAGACTCGTCCAAGAACCAGTTCTCCCTGAACCTGAACTCTGTGACCGCCGCAGACACGGCTGTGTATTCCTGTGCGAGACATGGAAAAGACTGGGGCCTTGACTACTGGGGCCAGGGAACCCTGGTCACCGTCTCCTCA |
| | | | SEQ ID NO: 24255 | SEQ ID NO: 28261 |
| | | AA | DIQMTQSPSSLSASVGDRVTITCRASQVIRNDLGWYQQKPGKAPKRLIYAVSSLQSGVPSRFSGSGSGTEFTLTINSLQPEDFATYYCLQHNTYPLTFGGGTKVEFK | QLQLQESGPGLVKPSETLSLTCTVSGGSISRSSYYWGWIRQPPGKGLEWIGNIYYSGSTYYNPSLKSRVTISVDSSKNQFSLNLNSVTAADTAVYSCARHGKDWGLDYWGQGTLVTVSS |
| | | | SEQ ID NO: 24256 | SEQ ID NO: 28262 |
| **iPS:392640** | **21-225_18A1** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTACAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGTGTCTGGATTCACCTTCAGTAGCTATGGCATGCATTGGGTCCGCCAGGCTCCAGGCAAGGGGCTGGAGTGGGTGGCAGTTATATGGTATGAGGAAAATAATAAATATTATGTAGACTCCGTGAAGGGCCGATTCACCATCTCCAGAGACAATTCCAAGAGCACGCTGTATCTGCAAATGAACAGCCTGAGAGCCGAGGACACGGCTGTGTATTACTGTGTGAGAGAGCTAGGCTTCCAGTCTGACTACTGGGGCCAGGGAACCCCGGTCACCGTCTCCTCA |
| | | | SEQ ID NO: 24257 | SEQ ID NO: 28263 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVEIK | QVQLVESGGGVVQPGRSLRLSCAVSGFTFSSYGMHWVRQAPGKGLEWVAVIWYEENNKYYVDSVKGRFTISRDNSKSTLYLQMNSLRAEDTAVYYCVRELGFQSDYWGQGTPVTVSS |
| | | | SEQ ID NO: 24258 | SEQ ID NO: 28264 |
| **iPS:392642** | **21-225_18C6** | NA | GACATCCAGATGACCCAGTCTCCATCCTCCCTGTCTGCATCTGTAGGAGACAGAGTCACCATCACTTGCCGGACAAGTCAGGGCATTAGAAATGATTTAGGCTGGTATCAGCAGAAACCGGGGAAAGCCCCTAAGCGCCTGATCTATGCTGCATCCAGTTTGCAAAGTGGGGTCCCGTCAAGGTTCAGCGGCAGTGGATCTGGGACAGAATTCACTCTCACAATCAGCAGCCTGCAGCCTGAAGATTTTGCAACTTATTACTGTCTACAGCATAGTTCTTACCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGTAGGAGTAGTTATTACTGGGGCTGGATCCGCCAGCCCCCAGGGAAGGGGCTGGAGTGGATTGGGAATATCTATTATAGTGGGTACACCTACTACAACCCGTCCCTCAAGAGTCGAGTCATCATATCCGTAGACACGTCCAAGAACCAGTTCTCCCTGAAGCTGAGCTCTGTGACCGCCGCAGACACGGCTCTGTATTACTGTGCGAGACATAGTAGCAGTTGGTCCCTTGACGACTGGGGCCAGGGAACCCTGGTCACCGTCTCCTCT |
| | | | SEQ ID NO: 24259 | SEQ ID NO: 28265 |
| | | AA | DIQMTQSPSSLSASVGDRVTITCRTSQGIRNDLGWYQQKPGKAPKRLIYAASSLQSGVPSRFSGSGSGTEFTLTISSLQPEDFATYYCLQHSSYPLTFGGGTKVEIK | QLQLQESGPGLVKPSETLSLTCTVSGGSISRSSYYWGWIRQPPGKGLEWIGNIYYSGYTYYNPSLKSRVIISVDTSKNQFSLKLSSVTAADTALYYCARHSSSWSLDDWGQGTLVTVSS |
| | | | SEQ ID NO: 24260 | SEQ ID NO: 28266 |

FIGURE 50

| | | | | |
|---|---|---|---|---|
| **iPS:392644** | **21-225_19E1** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAATTTACAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTTCCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCCGGCAAGGGGCTGGAGTGGGTGGCAGTTATTTGGTATGAAGAAAATAATCAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGTTCCGGTCTGACTACTGGGGCCAGGGAACCCTGGTCACCGTCTCCTCA |
| | | | SEQ ID NO: 24261 | SEQ ID NO: 28267 |
| | | AA | DIQMTQSPSSLSASVGDRVTITCRASQGIRNDLGWYQQKPGKAPKRLIYAASNLQSGVPSRFSGSGSGTEFTLTISSLQPEDFATYYCLQHNSFPLTFGGGTKVEIK | QVQLVESGGGVVQPGRSLRLSCAASGFTFSNYGMHWVRQAPGKGLEWVAVIWYEENNQYYADSVKGRFTISRDNSKNTLYLQMNSLRAEDTAVYYCARELGFRSDYWGQGTLVTVSS |
| | | | SEQ ID NO: 24262 | SEQ ID NO: 28268 |
| **iPS:392646** | **21-225_20G2** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTCCAAAGTGGGGTCCCATCAAGGTTCAGCGGCAGTGGATCTGGGACAGAATTCACTCTCACAATCAGCAGCCTGCAGCCTGAAGATTTTGCAAGTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCCTCAGTAGCGATGACATGCACTGGGTCCGCCAGGAACCAGGCAAGGGGCTGGAGTGGGTGGCAGTTATATGGTTTGATGGAAGTAATAAATACTATGCAGACTCCGTGAAGGGCCGATTCATCATGTCCAGAGACAATTCCAAGAACACCCTGTATCTGCAAATGAACAGCCTGAGAGCCGGGGACACGGCTGTGTATTACTGTGCGAGAGATCTGATAGCAGCAGCTGGTACGGTTGACTACTGGGGCCAGGGAACCCTGGTCACCGTCTCCTCA |
| | | | SEQ ID NO: 24263 | SEQ ID NO: 28269 |

FIGURE 50

| | | | | |
|---|---|---|---|---|
| | | AA | DIQMTQSPSSLSASVGDRVTITCRASQGIRNDLGWYQQKPGKAPKRLIYAASSFQSGVPSRFSGSGSGTEFTLTISSLQPEDFASYYCLQHNSYPLTFGGGTKVEIK | QVQLVESGGGVVQPGRSLRLSCAASGFTLSSDDMHWVRQEPGKGLEWVAVIWFDGSNKYYADSVKGRFIMSRDNSKNTLYLQMNSLRAGDTAVYYCARDLIAAAGTVDYWGQGTLVTVSS |
| | | | SEQ ID NO: 24264 | SEQ ID NO: 28270 |
| iPS:392648 | 21-225_16D11 | NA | GACATCCAGATGACCCAGTCTCCATCCTCCCTGTCTGCATCTGTCGGAGACAGAGTCACCATCACTTGCCGGGCAAGTCAGACCATTAGCAACTATTTAAATTGGTATCATCAGAAACCAGGGAAAGCCCCTAAGCTCCTGATCTATGCTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTCACAGTTCCCCGCTCACTTTCGGCGGAGGGACCAAGGTGGAGATCAAA | CAGGTACAGCTGCAGCAGTCAGGTCCAGGACTGGTGAAGCCCTCGCAGACCCTCTCACTCACCTGTGCCATCTCCGGGGACAGTGTCTCTCGCAACACTGCTGCTTGGAGCTGGATCAGGCAGTCCCCATCGAGAGGCCTTGAGTGGCTGGGAAGGACATACTACAGGTCCAAGTGGTATAATGATTATGCAGTATCTGTGAAAAGTCGAATAACCATCAACCCAGACACATCCAAGAACCAGTTCTCCCTGCAGCTGAACTCTGTGACTCCCGAGGACACGGCTGTGTATTACTGTGCAAGAGTAAACAGTGGCTGGTCCCATCACTACTACTACTACGGTATGGACGTCTGGGGCCAAGGGACCACGGTCACCGTCTCCTCA |
| | | | SEQ ID NO: 24265 | SEQ ID NO: 28271 |
| | | AA | DIQMTQSPSSLSASVGDRVTITCRASQTISNYLNWYHQKPGKAPKLLIYAASSLQSGVPSRFSGSGSGTDFTLTISSLQPEDFATYYCQQSHSSPLTFGGGTKVEIK | QVQLQQSGPGLVKPSQTLSLTCAISGDSVSRNTAAWSWIRQSPSRGLEWLGRTYYRSKWYNDYAVSVKSRITINPDTSKNQFSLQLNSVTPEDTAVYYCARVNSGWSHHYYYYGMDVWGQGTTVTVSS |
| | | | SEQ ID NO: 24266 | SEQ ID NO: 28272 |

FIGURE 50

| | | | | |
|---|---|---|---|---|
| **iPS:392650** | **21-225_17A4** | NA | GACATCCAGATGACCCAGTCTCCATCTTCCGTGTCTGCATCTGTAGGAGACAGAGTCACCATCACTTGTCGGGCGAGTCAGGGTATTGGCAACTGGTTAGCCTGGTATCAGCAGAAACCAGGGAAAGCCCCTAAACTCCTGATCTTTGCTGCATCCAGTTTGCAAAGTGGGGTCCCATCACGGTTCAGCGGCAGTGGATCTGGGACAGATTTCACTCTCACCATCAGCAGCCTGCAGCCTGAAGATTTTGTAACTTACTATTGTCAACAGGCTAACAGTTTCCCTCGGACGTTCGGCCAAGGGACCAAGGTGGAAATCAAA | CAGGTGCAGCTGGTGGAGTCTGGGGGAGGCTTGGTCAAGCCTGGAGGGTCCCTGAGACTCTCCTGTGCAGCCTCTGGATTCACCTTCAGTGACTACTACATGAGCTGGATCCGCCAGGCTCCAGGGAAGGGGCTGGAGTGGGTTTCACACATTAGTAGTAGTGGTAGTACCATATATTACGCAGACTCTGTGAAGGGCCGATTCACCATCTCCAGGGACAACGCCAAGAACTCACTGTATCTGCAAATGAACAGCCTGAGAGCCGAGGACACGGCCGTGTATTACTGTGCGAGATATCGGAATAACCGGGGATACTTCGATCTCTGGGGCCGTGGCACCCTGGTCACTGTCTCTTCA |
| | | | SEQ ID NO: 24267 | SEQ ID NO: 28273 |
| | | AA | DIQMTQSPSSVSASVGDRVTITCRASQGIGNWLAWYQQKPGKAPKLLIFAASSLQSGVPSRFSGSGSGTDFTLTISSLQPEDFVTYYCQQANSFPRTFGQGTKVEIK | QVQLVESGGGLVKPGGSLRLSCAASGFTFSDYYMSWIRQAPGKGLEWVSHISSSGSTIYYADSVKGRFTISRDNAKNSLYLQMNSLRAEDTAVYYCARYRNNRGYFDLWGRGTLVTVSS |
| | | | SEQ ID NO: 24268 | SEQ ID NO: 28274 |
| **iPS:392652** | **21-225_17C6** | NA | GACATCCAGATGACCCAGTCTCCATCCTCCCTGTCTGCATCTGTAGGAGACAGAGTCACCATCACTTGCCGGGCAAGTCAGAGCATTAATACTTATTTAAATTGGTATCAGCAAAAACCAGGGAAAGCCCCTAAGCTCCTGATCTATGCTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTTCTGTCAACAGAGTTACAGAACCCCCTTTTTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGAATTCACCTTTAGCAGCTATGCCATGAACTGGGTCCGCCAGGCTCCAGGGAAGGGGCTGGAGTGGGTCTCAGTTATTAGTGGTCGTGGTGGTAACACATTCTACGCAGACTCCGTGAAGGGCCGGTTCACCATCTCCAGAGACAATTCCAAGAACACGCTCTTTCTGCAAATGAACAGCCTGAGAGCCGAGGACACGGCCGTATATTATTGTGCGTCCCGTTTGGCAGTGGCTGGCTCGGAGGCTTTTGATATCTGGGGCCAAGGGACAATGGTCACCGTCTCTTCA |
| | | | SEQ ID NO: 24269 | SEQ ID NO: 28275 |

FIGURE 50

| | | | | |
|---|---|---|---|---|
| | | AA | DIQMTQSPSSLSASVGDRVTITCRASQSINTYLN WYQQKPGKAPKLLIYAASSLQSGVPSRFSGSGS GTDFTLTISSLQPEDFATYFCQQSYRTPFFTFGPG TKVDIK | EVQLLESGGGLVQPGGSLRLSCAASEFTFSSYAMN WVRQAPGKGLEWVSVISGRGGNTFYADSVKGRFTI SRDNSKNTLFLQMNSLRAEDTAVYYCASRLAVAG SEAFDIWGQGTMVTVSS |
| | | | SEQ ID NO: 24270 | SEQ ID NO: 28276 |
| iPS:392654 | 21-225_17A10 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTACAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATAATAGTTACCCGCTC ACTTTCGGCGGAGGGACCAAGGTGGAGATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGCCAAGGGGCT GGAGTGGGTGGCAGTTATTTGGTATGAAGAAAAT AATCAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACTGGGG TTCCGGTCTGACTACTGGGGCCAGGGAACCCTGG TCACCGTCTCCTCA |
| | | | SEQ ID NO: 24271 | SEQ ID NO: 28277 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPLTFGGG TKVEIK | QVQLVESGGGVVQPGRSLRLSCAASGFTFSNYGM HWVRQAPAKGLEWVAVIWYEENNQYYADSVKGR FTISRDNSKNTLYLQMNSLRAEDTAVYYCARELGF RSDYWGQGTLVTVSS |
| | | | SEQ ID NO: 24272 | SEQ ID NO: 28278 |

FIGURE 50

| | | | | |
|---|---|---|---|---|
| **iPS:392656** | **21-225_1F2** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTGTGCAAAGTGGGGTCCCATCAAGGTTCAGCGGCAGTGGATCTGGGACAGAATTCACTCTCACAATCAGCAGCCTGCAGCCTGAAGATTTTGCAATTTATTACTGTCTACAGCATAATAGTTACCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGTAGGAGTAGTTACTACTGGGGCTGGATCCGCCAGCCCCCAGGGAAGGGGCTGGAGTGGATTGGGAATATTTATTATAGTGGGAGCGCCTACAACAACCCGTCCCTCAAGGGTCGAGTCACCATATCCGTAGACACGTCCAAGAACCAGTTCTCCCTGAAGCTGAACTCTGTGACCGCCGCAGACACGGCTGTGTATTACTGTGGGAGACATGGAAAAGACTGGGGCCTTGACTACTGGGGCCAGGGAACCCTGGTCACCGTCTCCTCA |
| | | | SEQ ID NO: 24273 | SEQ ID NO: 28279 |
| | | AA | DIQMTQSPSSLSASVGDRVTITCRASQGIRNDLGWYQQKPGKAPKRLIYAASSVQSGVPSRFSGSGSGTEFTLTISSLQPEDFAIYYCLQHNSYPLTFGGGTKVEIK | QLQLQESGPGLVKPSETLSLTCTVSGGSISRSSYYWGWIRQPPGKGLEWIGNIYYSGSAYNNPSLKGRVTISVDTSKNQFSLKLNSVTAADTAVYYCGRHGKDWGLDYWGQGTLVTVSS |
| | | | SEQ ID NO: 24274 | SEQ ID NO: 28280 |
| **iPS:392658** | **21-225_18E8** | NA | GACATCCAGATGACCCAGGCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATTTGGTATGAAGAAAATAATCAATACTATGCAGACTCCGTGAAGGGCCGATTCACCGTCTCCAGAGACAATTCCAAGAACACGCTGTTTCTGCAAATGAACAGCCTGAGAGCCGAGGACACGGCTGTGTATTACTGTGCGAGAGAACTGGGGTTCCGGTCTGACTACTGGGGCCAGGGAACCCTGGTCACCGTCTCCTCA |
| | | | SEQ ID NO: 24275 | SEQ ID NO: 28281 |

FIGURE 50

| | | | | |
|---|---|---|---|---|
| | | AA | DIQMTQAPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVEIK | QVQLVESGGGVVQPGRSLRLSCAASGFTFSNYGMHWVRQAPGKGLEWVAVIWYEENNQYYADSVKGRFTVSRDNSKNTLFLQMNSLRAEDTAVYYCARELGFRSDYWGQGTLVTVSS |
| | | | SEQ ID NO: 24276 | SEQ ID NO: 28282 |
| **iPS:392660** | **21-225_19B3** | NA | GACATCCAGATGACCCAGTCTCCATCCTCCCTGTCTGCTTCTGTCGGAGACAGAATCACCATCACTTGCCGGGCAGGTCAGAACATTATCAACTATTTAAATTGGTATCAGCAGAAACCAGGGAAAGCCCCTAACCTCCTGATATATGTTGCATCCAGTTTGCAAAGTGGGGTCCCATCAAGGTTCAATGGCAGTGGATCTGGGACAGATTTCACTCTCACCATCAGCAGTCTGCAACCTGAAGATTTTGCAACTTACTACTGTCAACAGAGTTACAGTACCCCTTTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGTTATGACATGCACTGGGTCCGCCAGGCTCCAGGCAAGGGGCTGGAGTGGGTGGCAGTTATATGGTATGACGGAAGTGATAAATACTATGCAGACTCCGTGAAGGGCCGATTCACCATCTCCAGAGACAATTCCAAGAACACGCTCTTTCTGCAAATGAACAGCCTGAGAGCCGAGGACACGGCTGTGTATTACTGTGCGAGAGATCGGGCCTATAGCAGCTCGTCTGACTACTGGGGCCAGGGAACCCTGGTCACCGTCTCCTCA |
| | | | SEQ ID NO: 24277 | SEQ ID NO: 28283 |
| | | AA | DIQMTQSPSSLSASVGDRITITCRAGQNIINYLNWYQQKPGKAPNLLIYVASSLQSGVPSRFNGSGSGTDFTLTISSLQPEDFATYYCQQSYSTPFTFGPGTKVDIK | QVQLVESGGGVVQPGRSLRLSCAASGFTFSSYDMHWVRQAPGKGLEWVAVIWYDGSDKYYADSVKGRFTISRDNSKNTLFLQMNSLRAEDTAVYYCARDRAYSSSDYWGQGTLVTVSS |
| | | | SEQ ID NO: 24278 | SEQ ID NO: 28284 |

FIGURE 50

| | | | | |
|---|---|---|---|---|
| **iPS:392664** | **21-225_20F6** | NA | GACATCCAGATGACCCAGTCTCCATCCTCCCTGTCTGCATCTGTAGGAGACAGAGTCACCATCACTTGCCGGGCAAGTCAGAGCATTATCACCTATTTAAATTGGTATCAGCAGAAACCAGGGAAAGCCCCTAAGGTCCTGATCCATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGACTTACAGTCCCCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGTAGCGTCTGGATTCACCTTCAGTAGCTATGGCATGCACTGGGTCCGCCAGGCTCCAGGCAAGGGGCTGGAGTGGGGGGCAGTTATATGGCATGATGGAAGTAATAAATACTATGCAGACTCCGTGAAGGGCCGATTCACCATCTCCAGAGACAATGCCAAGAACACGCTGTATCTGCAAATGAACAGCCTGAGAGCCGAGGACACGGCTGTGTATTACTGTGCGAGAGATCTGAGTATGGGCGGAATGGACGTCTGGGGCCAAGGGACCACGGTCACCGTCTCCTCA |
| | | | SEQ ID NO: 24279 | SEQ ID NO: 28285 |
| | | AA | DIQMTQSPSSLSASVGDRVTITCRASQSIITYLNWYQQKPGKAPKVLIHTASSLQSGVPSRFSGSGSGTDFTLTISSLQPEDFATYYCQQTYSPPLTFGGGTKVEIK | QVQLVESGGGVVQPGRSLRLSCVASGFTFSSYGMHWVRQAPGKGLEWGAVIWHDGSNKYYADSVKGRFTISRDNAKNTLYLQMNSLRAEDTAVYYCARDLSMGGMDVWGQGTTVTVSS |
| | | | SEQ ID NO: 24280 | SEQ ID NO: 28286 |
| **iPS:392666** | **21-225_16F11** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTGTACAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGATGGTGGAGTCTGGGGGAGGCGTGGTCCAGCCTGGGAGGTCCCTGAGACTCTCTTGTGAAGCGTCTGGATTCACCTTCAGTAGCTATGGCATGCACTGGGTCCGCCAGGCTCCAGGCAAGGGGCTGGAGTGGGTGGCAGTTATATGGTATGAGGAAAATAATAAATACTATGTAGACTCCGTGAAGGGCCGATTCACCATCTCCAGAGACAATTCCAAGAGCACGCTGTATCTGCAAATGAATAGCCTGAGAGCCGAGGACACGGCTGTGTATTACTGTGTGAGAGAACTAGGCTTCCAGTCTGACTACTGGGGCCAGGGAACCCCGGTCACCGTCTCCTCA |
| | | | SEQ ID NO: 24281 | SEQ ID NO: 28287 |

FIGURE 50

| | | | | |
|---|---|---|---|---|
| | | AA | DIQMTQSPSSLSASVGDRVTITCRASQGIRNDLGWYQQKPGKAPKRLIYAASSVQSGVPSRFSGSGSGTEFTLTISSLQPEDFATYYCLQHNSYPLTFGGGTKVEIK | QVQMVESGGGVVQPGRSLRLSCEASGFTFSSYGMHWVRQAPGKGLEWVAVIWYEENNKYYVDSVKGRFTISRDNSKSTLYLQMNSLRAEDTAVYYCVRELGFQSDYWGQGTPVTVSS |
| | | | SEQ ID NO: 24282 | SEQ ID NO: 28288 |
| **iPS:392668** | **21-225_17B4** | NA | GACATCCAGATGACCCAGTCTCCATCCTCCCTGTCTGCATCTGTAGGCGACAGAGTCACCATCACTTGCCGGGCAAGTCAGAACATTAGTAGTTATTTAAATTGGTATCAGCAGAAACCAGGGAAAGCCCCTAAGCTCCTGATCTTTGGTGCATCCAGTTTGCAAACTGGGGTCCCATCAAGGTTCAGTGGCAGTGGATCTGGGACAGATTTCACTCTCACCATCAACAGTCTGCAACCTGAAGATTTTGCAACTTACTTCTGTCAACAGAGTTACAGAACCCCCTTTTTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGAATTTACCTTTAGCAGCTATGCCATGAACTGGGTCCGCCAGGCTCCAGGGAAGGGGCTGGAGTGGGTCTCAGTTATTAGTGGCCGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGTCCCGTTTGGCAGTGGCTGGCTCGGAGGCTTTTGATATCTGGGGCCAAGGGACAATGGTCACCGTCTCTTCA |
| | | | SEQ ID NO: 24283 | SEQ ID NO: 28289 |
| | | AA | DIQMTQSPSSLSASVGDRVTITCRASQNISSYLNWYQQKPGKAPKLLIFGASSLQTGVPSRFSGSGSGTDFTLTINSLQPEDFATYFCQQSYRTPFFTFGPGTKVDIK | EVQLLESGGGLVQPGGSLRLSCAASEFTFSSYAMNWVRQAPGKGLEWVSVISGRGGNTFYADSVKGRFTISRDNSKNTLYLQMNSLRAEDTAVYYCASRLAVAGSEAFDIWGQGTMVTVSS |
| | | | SEQ ID NO: 24284 | SEQ ID NO: 28290 |

FIGURE 50

| | | | | |
|---|---|---|---|---|
| **iPS:392674** | **21-225_18C2** | NA | GACATCCAGATGACCCAGTCTCCATCCTCCCTGTCTGCGTCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATACTGCATCCAGTTTGCAAAGTGGGGTCCCATCAAGATTCAGCGGCAGTGGATTTGGGACAGAATTCACTCTCACAATCAGCAGCCTGCAGCCTGAAGATTTTGCAACTTATTACTGTCTACAACATAATAGTTACCCGTGGACGTTCGGCCTGGGGACCAAGGTGGTCATCAAA | CAGGTGCAACTGGTGGAGTCTGGGGGAGGCGTGGTCCAGCCTGGGAGGTCCCTGAGACTCTCCTGTGTAGCGTCTGGATTCACCTTCAGTGACTATGGCATGCACTGGGTCCGCCAGGCTCCAGGCAAGGGGCTGGAGTGGATGGCAGTTATATGGTATGATGTAACTAATAAATACTATGCAGACTCCGTGAAGGGCCGATTCACCATCTCCAGAGACAATTCCAAGAACACGCTGTATCTGCAAATGAACAGCCTGAGCGCCGAGGACACGGCTGTTTATTACTGTGCGAGAGAGCTTGGCTGGTACGAGGACTACTGGGGCCAGGGAACCCTGGTCACCGTCTCCTCA |
| | | | SEQ ID NO: 24285 | SEQ ID NO: 28291 |
| | | AA | DIQMTQSPSSLSASVGDRVTITCRASQGIRNDLGWYQQKPGKAPKRLIYTASSLQSGVPSRFSGSGFGTEFTLTISSLQPEDFATYYCLQHNSYPWTFGLGTKVVIK | QVQLVESGGGVVQPGRSLRLSCVASGFTFSDYGMHWVRQAPGKGLEWMAVIWYDVTNKYYADSVKGRFTISRDNSKNTLYLQMNSLSAEDTAVYYCARELGWYEDYWGQGTLVTVSS |
| | | | SEQ ID NO: 24286 | SEQ ID NO: 28292 |
| **iPS:392676** | **21-225_19F3** | NA | GACATCCAGATGACCCAGTCTCCATCCTCCCTGTCTGCATCTGTACGAGACAGAGTCACCATCACTTGCCGGGCAAGTCAGGGCATAAGAAATGATTTAGGCTGGTATCAGCAGAAACCAGGGAAAGCCCCTAAGCGCCTGATCTATGCTGTTTCCAGTTTGCAAAGTGGGGTCCCATCAAGGTTCAGCGGCAGTGGATCTGGGACAGAGTTCACTCTCACAATCAGCAGCCTGCAGCCTGAAGATTTTGCAACTTATTACTGTCTACAGCATGCCAGTTACCCGCTCACTTTCGGCGGAGGGACCAAGGTGGAGATCAAA | CAGGTGCAGCTGCAGGAGTCGGGCCCAGGACTGGTGAAGCCTTCGGAGACCCTGTCCCTCACCTGCACTGTCTCCGGTGGCGCCATCAGCGGTAGTAGTTACTACTGGGGCTGGATCCGCCAGCCCCCAGGGAAACAACTGGAGTGGATTGGGAATATCTATTATAGTGGGAGCACCTACTACAACCCGTCCTTCAAGAGTCGAGTCACCATATCCGTAGACACGTCCAAGAACCAGTTCTCCCTGAAGCTGAGCTCTGTGACCGCCGAAGACACGGCTGTCTATTACTGTGCGAGACATTCCAGTAGCTGGTCCCTTGACTACTGGGGCCAGGGAACCCTGGTCACCGTCTCCTCT |
| | | | SEQ ID NO: 24287 | SEQ ID NO: 28293 |

FIGURE 50

| | | | | |
|---|---|---|---|---|
| | | AA | DIQMTQSPSSLSASVRDRVTITCRASQGIRNDLGWYQQKPGKAPKRLIYAVSSLQSGVPSRFSGSGSGTEFTLTISSLQPEDFATYYCLQHASYPLTFGGGTKVEIK | QVQLQESGPGLVKPSETLSLTCTVSGGAISGSSYYWGWIRQPPGKQLEWIGNIYYSGSTYYNPSFKSRVTISVDTSKNQFSLKLSSVTAEDTAVYYCARHSSSWSLDYWGQGTLVTVSS |
| | | | SEQ ID NO: 24288 | SEQ ID NO: 28294 |
| iPS:392678 | 21-225_20F3 | NA | GACATCCAGATGACCCAGTCTCCATCCTCCCTGTCTGCATCTGTAGGAGACAGAGTCACCATCACTTGCCGGGCAAGTCAGAGCATTAGTAGTTATTTATATTGGTATCAGCAGAAACCAGGGAAAGCCCCTAAGCTCCTGATCTATGCTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACAGTGCCCCTCCATTCACTTTCGGCCCTGGGACCAAAGTGGATATCAAA | GAGGTGCAGCTGTTGGAGTCTGGGGGAGGCTTGGTACAGCCGGGGGGGTCCCTGAGACTCTCCTGTGCAGCCTCTGGATTCACCTTTAGCAGCTATGCCATGAACTGGGTCCGCCAGGCTCCAGGGAAGGGGCTGGAGTGGGTCTCAGTTATTAGTGGTAGTGGTGGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAAAGGATAGCAGCAGCTGGTACGGAGTACTTCGATCTCTGGGGCCGTGGCACCCTGGTCACTGTCTCCTCA |
| | | | SEQ ID NO: 24289 | SEQ ID NO: 28295 |
| | | AA | DIQMTQSPSSLSASVGDRVTITCRASQSISSYLYWYQQKPGKAPKLLIYAASSLQSGVPSRFSGSGSGTDFTLTISSLQPEDFATYYCQQSYSAPPFTFGPGTKVDIK | EVQLLESGGGLVQPGGSLRLSCAASGFTFSSYAMNWVRQAPGKGLEWVSVISGSGGSTYYADSVKGRFTISRDNSKNTLYLQMNSLRAEDTAVYYCAKRIAAAGTEYFDLWGRGTLVTVSS |
| | | | SEQ ID NO: 24290 | SEQ ID NO: 28296 |

FIGURE 50

| | | | | |
|---|---|---|---|---|
| **iPS:392680** | **21-225_20A7** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAAAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCTCTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATTTGGTATGAAGAAAATAATCAATACTATGCAGACTCCGTGAAGGGCCGATTCACCATCTCCAGAGACAATTCCAAGAACACGCTGTATCTGCAAATGAACAGCCTGAGAGCCGAGGACACGGCTATTTATTACTGTGCGAGAGAACTGGGGTTCCGGTCTGACTACTGGGGCCAGGGAACCCTGGTCACCGTCTCCTCA |
| | | | SEQ ID NO: 24291 | SEQ ID NO: 28297 |
| | | AA | DIQMTQSPSSLSASVGDRVTITCRASQGIRNDLGWYQQKPGKAPKRLIYAASSLQSGVPSRFSGSGSGTEFSLTISSLQPEDFATYYCLQHNSYPLTFGGGTKVEIK | QVQLVESGGGVVQPGRSLRLSCAASGFTFSNYGMHWVRQAPGKGLEWVAVIWYEENNQYYADSVKGRFTISRDNSKNTLYLQMNSLRAEDTAIYYCARELGFRSDYWGQGTLVTVSS |
| | | | SEQ ID NO: 24292 | SEQ ID NO: 28298 |
| **iPS:392682** | **21-225_16A12** | NA | GACATCCAGATGACCCAGTCTCCATCCTCACTGTCTGCATCTGTAGGAGACAGAGTCACCATCACTTGTCGGGCGAGTCAGGCCATTAACACTTATTTAGCCTGGTTTCAGCAGAAACCAGGGAAAGCCCCTAAGTCCCTGATTTCTGCTGCATCCAGTTTGCAAAGTGGGGTCCCATCAAAGTTCAGCGGCAGTGGATCTGGGACAGAATTCACTCTCACCATCAGCAGCCTGCAGCCTGAAGATTTTGCAACTTATTACTGCCAACAGTATTATAGTTATCCGCTCACTTTCGGCGGAGGGACCAAGGTGGAGATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGAATGAACTGGTTCCGCCAGGCTCCAGGGAAGGGGCTGGAGTGGGTCTCATCCATTAGTGGTAGTAGTACTGACATATACTACGCAGACTCAGTGAAGGGCCGATTCACCATCTCCAGAGACAACGCCGAGAACTCACTGTATCTGCAAATGAACAGCCTGAGAGCCGAGGACACGGCTGTGTATTACTGTGCGAGAAGGGACTTCTGGGGCCAGGGAACCCTGGTCACCGTCTCCTCA |
| | | | SEQ ID NO: 24293 | SEQ ID NO: 28299 |

FIGURE 50

| | | | | |
|---|---|---|---|---|
| | | AA | DIQMTQSPSSLSASVGDRVTITCRASQAINTYLAWFQQKPGKAPKSLISAASSLQSGVPSKFSGSGSGTEFTLTISSLQPEDFATYYCQQYYSYPLTFGGGTKVEIK | EVQLVESGGGLVKPGGSLRLSCAASGFTFSSYRMNWFRQAPGKGLEWVSSISGSSTDIYYADSVKGRFTISRDNAENSLYLQMNSLRAEDTAVYYCARRDFWGQGTLVTVSS |
| | | | SEQ ID NO: 24294 | SEQ ID NO: 28300 |
| iPS:392684 | 21-225_17F4 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGAACTGGGTCCGCCAGGCTCCAGGCAAGGGGCTGGAGTGGGTGGCAGTTATATGGTATGATGGAAATAATAAACACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGTAGTGGGAGCTACTTCTTTGACTACTGGGGCCAGGGAACCCTGGTCACCGTCTCCTCA |
| | | | SEQ ID NO: 24295 | SEQ ID NO: 28301 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPFTFGPGTKVDIK | QVQLVESGGGVVQPGRSLRLSCAASGFTFSNYGMNWVRQAPGKGLEWVAVIWYDGNNKHYADSVKGRFTISRDNSKNTLYLQMNSLRAEDTAVYYCASSGSYFFDYWGQGTLVTVSS |
| | | | SEQ ID NO: 24296 | SEQ ID NO: 28302 |

FIGURE 50

| | | | | |
|---|---|---|---|---|
| **iPS:392686** | **21-225_17C7** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGATTCAGCGGCAGTGGATCTGGGACAGAATTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | CAGGTGCAGCTGGTGGAGTCTGGGGGAGGCGTGGTCCAGCCTGGGAAGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTTTGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGGCACGGCTGTGTATTACTGTGCGAGAGATCTTGGCTGGACGGAAGAATACTGGGGCCAGGGAACCCTGGTCACCGTCTCCTCA |
| | | | SEQ ID NO: 24297 | SEQ ID NO: 28303 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPWTFGQGTKVEIK | QVQLVESGGGVVQPGKSLRLSCAASGFTFSDYGMHWVRQAPGKGLEWVAVIWFDGSNKYYADSVKGRFTISRDNSKNTLYLQMNSLRAEGTAVYYCARDLGWTEEYWGQGTLVTVSS |
| | | | SEQ ID NO: 24298 | SEQ ID NO: 28304 |
| **iPS:392690** | **21-225_18F2** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTTTGATGGAAGTAATAAATACTATGTAGACTCCGTGAAGGGCCGATTCACCATCTCCAGAGACAATTCCAAGAACACGCTGTATCTGCAAATGAACAGCCTGAGAGTCGAGGACACGGCTGTGTATTACTGTGCGAGAGATCTTGGCTGGACGGAAGAGTACTGGGGCCAGGGAACCCTGGTCACCGTCTCCTCA |
| | | | SEQ ID NO: 24299 | SEQ ID NO: 28305 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPWTFGQGTKVEIK | QVQLVESGGGVVQPGRSLRLSCAASGFTFSDYGMHWVRQAPGKGLEWVAVIWFDGSNKYYVDSVKGRFTISRDNSKNTLYLQMNSLRVEDTAVYYCARDLGWTEEYWGQGTLVTVSS |
| | | | SEQ ID NO: 24300 | SEQ ID NO: 28306 |
| **iPS:392692** | **21-225_18G10** | NA | GACATCCAGATGACCCAGTCTCCATCCTCACTGTCTGCATCTGTAGGAGACAGAGTCACCATCACTTGTCGGGCGAGTCAGGACATTAGCTATTATTTAGCCTGGTTTCAGCAGAAACCAGGGAAAGCCCCTAAGTCCCTGATTTATGTTGCATCCAGTTTGCAAAGTGGGGTCCCATCAAAGTTCGGCGGCAGTGGATTTGGGACAGATTTCACTCTCACCATCAGCAGCCTGCAGCCTGAAGATTTTGCAACTTATTACTGCTTACAGTATAATAGTTACCCATTCACTTTCGGCCCTGGGACCAAAGTGGATATCAAA | GAGGTGCAGCTGGTGGAGTCTGGGGGAGGCTTGGTACAGCCTGGGGGGTCCCTGAGACTCCTCTGTGCAGCCTCTGGAATCACCTTCAGTACCTATAGCATGAACTGGGTCCGCCAGGCTCCAGGGAAGGGGCTGGAGTGGGTTTCATACATTAGTAGGAGTAGTAGTACCATAGACTACGCAGACTCTGTGAAGGGCCGATTCACCATCTCCAGAGACAATGCCAAGAACTCACTGTATCTGCAAATGAACAGCCTGAGAGACGAGGACACGGCTGTGTATTACTGTGCGAGAGGAGGTGGGAGCCCTTTTGACTACTGGGGCCAGGGAACCCTGGTCACCGTCTCCTCA |
| | | | SEQ ID NO: 24301 | SEQ ID NO: 28307 |
| | | AA | DIQMTQSPSSLSASVGDRVTITCRASQDISYYLAWFQQKPGKAPKSLIYVASSLQSGVPSKFGGSGFGTDFTLTISSLQPEDFATYYCLQYNSYPFTFGPGTKVDIK | EVQLVESGGGLVQPGGSLRLLCAASGITFSTYSMNWVRQAPGKGLEWVSYISRSSSTIDYADSVKGRFTISRDNAKNSLYLQMNSLRDEDTAVYYCARGGGSPFDYWGQGTLVTVSS |
| | | | SEQ ID NO: 24302 | SEQ ID NO: 28308 |

FIGURE 50

| | | | | |
|---|---|---|---|---|
| iPS:392694 | 21-225_19A5 | NA | GACATCCAGATGACCCAGTCTCCATCCTCCCTGTCTGCACCTGTAGGAGACAGAGTCTCCATCACTTGCCGGGCAAGTCAGAACATTATCAACTATTTAAATTGGTATCAGCAGAAACCAGGGAAAGCCCCTAAGCTCCTGATAGATGTTGCATCCAATTTACAAGGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACAGTACCCCTTTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGCCATGCACTGGGTCCGCCAGGCTCCAGGCAAGGGGCTGGAGTGGGTGGCAGTTATATGGTTTGATGGAAGTGATAAATACTATGCAGACTCCGTGAAGGGCCGATTCACCATCTCCAGAGACAATTCCAAGAACACGCTGTATCTACAAATGAACAGCCTGAGAGCCGAGGACACGGCTGTGTATTACTGTGCGAGAGATCGGGCCTATAGTAGCTCGTCTGACTACTGGGGCCAGGGAACCCTGGTCACCGTCTCCTCA |
| | | | SEQ ID NO: 24303 | SEQ ID NO: 28309 |
| | | AA | DIQMTQSPSSLSAPVGDRVSITCRASQNIINYLNWYQQKPGKAPKLLIDVASNLQGGVPSRFSGSGSGTDFTLTISSLQPEDFATYYCQQSYSTPFTFGPGTKVDIK | QVQLVESGGGVVQPGRSLRLSCAASGFTFSSYAMHWVRQAPGKGLEWVAVIWFDGSDKYYADSVKGRFTISRDNSKNTLYLQMNSLRAEDTAVYYCARDRAYSSSSDYWGQGTLVTVSS |
| | | | SEQ ID NO: 24304 | SEQ ID NO: 28310 |
| iPS:392696 | 21-225_20A4 | NA | GACATCCAGATGACCCAGTCTCCAGCCTCCCTGTCTGCATCTGTAGGAGACAGAGTCACCATCACTTGCCGGGCAAGTCAGAGCATTATCAACTATTTAAATTGGTATCAGCAGAGACCAGGGAAATCCCCTAAGCTCCTGATCTATGCTGCATCCAGTTTGCACAGTGGGGTCCCATCAAGGTTCAGTGGCAGAGGATCTGGGACAGATTTCACTCTCACCATCAGTAGTCTGCAACCTGAAGATTTTGCAACTTACTTCTGTCAACAGAGTTACAGAACCCCCTTATTCACTTTCGGCCCTGGGACCAAAGTAGATTTCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGACCTGGGTCCGCCAGGGTCCAGGGATGGGGCTGGAGTGGGTCTCAGTTATAAGTGGTAGTGGTGGTTACACATACAACGCGGACTCCGTGAAGGGCCGGTTCACCATCTCCAGAGACAATTCCAAGAACACGCTGTATCTGCAAATGAACAGCCTGAGAGTCGAGGACACGGCCGTATATTACTGTGCGTCCCGTATAGCAGTGGCTGGCTCGGAGGCTTTTGATATCTGGGGCCAAGGGACAATGGTCACCGTCTCTTCA |
| | | | SEQ ID NO: 24305 | SEQ ID NO: 28311 |

FIGURE 50

| | | | | |
|---|---|---|---|---|
| | | AA | DIQMTQSPASLSASVGDRVTITCRASQSIINYLNWYQQRPGKSPKLLIYAASSLHSGVPSRFSGRGSGTDFTLTISSLQPEDFATYFCQQSYRTPLFTFGPGTKVDFK | EVQLLESGGGLVQPGGSLRLSCAASGFTFSSYAMTWVRQGPGMGLEWVSVISGSGGYTYNADSVKGRFTISRDNSKNTLYLQMNSLRVEDTAVYYCASRIAVAGSEAFDIWGQGTMVTVSS |
| | | | SEQ ID NO: 24306 | SEQ ID NO: 28312 |
| **iPS:392700** | **21-225_16E12** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTACAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCCTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGAGGGAAGTAATAAATATTATGTAGACTCCGTGAGGGGCCGATTCACCATCTCCAGAGACAATTCCAAGAGCACGCTGTATCTGCAAATGAACAGCCTGAGAGCCGAGGACACGGCTGTGTATTACTGTGTGAGAGAGCTAGGCTTCCAGTCTGATCACTGGGGCCAGGGAACCCCGGTCACCGTCTCCTCA |
| | | | SEQ ID NO: 24307 | SEQ ID NO: 28313 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVEIK | QVQLVESGGGLVQPGRSLRLSCAASGFTFSNYGMHWVRQAPGKGLEWVAVIWYEGSNKYYVDSVRGRFTISRDNSKSTLYLQMNSLRAEDTAVYYCVRELGFQSDHWGQGTPVTVSS |
| | | | SEQ ID NO: 24308 | SEQ ID NO: 28314 |

FIGURE 50

| | | | | |
|---|---|---|---|---|
| **iPS:392702** | **21-225_17F7** | NA | GACATCCAGATGACCCAGTCTCCATCCTCCCTGTCTGCATCTGTTGGAGACAGAGTCACCATCACTTGCCGGGCAAGTCAGACCATTAGTAGTTTTTTAAATTGGTATCAGCAGAAACCAGGGAAAGCCCCTAAACTCCTGATCTATGCTGCTTCCAGTTTGCAAAGTGGGGTCCCGTCAAGGTTCAGTGGCAGTGGATCTGGGACAGATTTCACTCTCACCATCAGCGGTCTGCAACCTGAAGATTTTGCAACTTACTTCTGTCAACAGAGTTACAGAACCCCCTTTTTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGAATTCACCTTTAGCAGCTATGCCATGAACTGGGTCCGCCAGGCTCCAGGGAAGGGGCTGGAGTGGGTCTCAATTATTAGTGGTCGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGTCCCGTTTGGCAGTGGCTGGCTCGGAGGCTTTTGATATCTGGGGCCAAGGGACAATGGTCACCGTCTCTTCA |
| | | | SEQ ID NO: 24309 | SEQ ID NO: 28315 |
| | | AA | DIQMTQSPSSLSASVGDRVTITCRASQTISSFLNWYQQKPGKAPKLLIYAASSLQSGVPSRFSGSGSGTDFTLTISGLQPEDFATYFCQQSYRTPFFTFGPGTKVDIK | EVQLLESGGGLVQPGGSLRLSCAASEFTFSSYAMNWVRQAPGKGLEWVSIISGRGGNTFYADSVKGRFTISRDNSKNTLYLQMNSLRAEDTAVYYCASRLAVAGSEAFDIWGQGTMVTVSS |
| | | | SEQ ID NO: 24310 | SEQ ID NO: 28316 |
| **iPS:392704** | **21-225_17F11** | NA | GACATCCAGATGACCCAGTCTCCATCCTCCCTGTCTGCATCTATAGGAGACAGAGTCTCCATCACTTGCCGGGCAAGTCGGACCATTAACAACTATTTAAATTGGTATCAGCAGAAACCAGGGAAAGCCCCTAAACTCCTGATCTTTGCTACATCCAGTTTACAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGACTTACAGTACCCCCTTATTCGCTTTCGGCCCTGGGACCAAAGTGGATATCAAA | GAGGCGCAGCTGTTGGAGTCTGGGGGAGGCTTGGAACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTATTAGTGGTAGAGGTGGTAACACATACTCCGCAGACTCCGTGAAGGGCCGGTTCACCATCTCCAGAGACAATTCCAAGAACACGCTATATCTACAAATGAACAGCCTGAGAGCCGAGGACACGGCCGTATATTACTGTGCGTCCCGTTTAGCAGTGGCTGGCTCGGAGGCTTTTCATATCTGGGGCCAAGGGACAATGGTCACCGTCTCTTCA |
| | | | SEQ ID NO: 24311 | SEQ ID NO: 28317 |

FIGURE 50

| | | | | |
|---|---|---|---|---|
| | | AA | DIQMTQSPSSLSASIGDRVSITCRASRTINNYLNWYQQKPGKAPKLLIFATSSLQSGVPSRFSGSGSGTDFTLTISSLQPEDFATYYCQQTYSTPLFAFGPGTKVDIK | EAQLLESGGGLEQPGGSLRLSCAASGFTFSSYAMSWVRQAPGKGLEWVSVISGRGGNTYSADSVKGRFTISRDNSKNTLYLQMNSLRAEDTAVYYCASRLAVAGSEAFHIWGQGTMVTVSS |
| | | | SEQ ID NO: 24312 | SEQ ID NO: 28318 |
| **iPS:392706** | **21-225_18A3** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGTTGCATCCAGTTTGCAAAGTGGGGTCCCATCAAGGTTCAACGGCAGTGGATCTGGGACAGAATTCACTCTCACAATCAGCAGCCTGCAGCCTGAAGATTTTGCAACTTATTACTGTCTACAGCATAGTAGTTACCCGCTCACTTTCGGCGGAGGGACCAAGGTGGAGATCAAA | CAGCTGCAGCTGCAGGAGTCGGGCCCAGGACTGGTGAAGTTTTCGGAGACCCTGTCCCTCACCTGCACTGTCTCTGGTGGCTCCATCAGCAGAAGTAGTTATTACTGGGGCTGGATCCGCCAGCCCCCAGGGAAGGGGCTGGAGTGGATAGGGAATATCTATTATAGTGGGTATACCTACTACACTCCGTCCCTCAAGAGTCGAGTCACCATATCCGTAGACACGTCCAAGAACCAGTTCTCCCTGAAGCTGAGCTCTGTGACCGCCGCAGACACGGCTGTGTATTACTGTGCGAGACATAGCACCAGCTGGTCCCTTGACTACTGGGGCCAGGGAACCCTGGTCACCGTCTCCTCA |
| | | | SEQ ID NO: 24313 | SEQ ID NO: 28319 |
| | | AA | DIQMTQSPSSLSASVGDRVTITCRASQGIRNDLGWYQQKPGKAPKRLIYVASSLQSGVPSRFNGSGSGTEFTLTISSLQPEDFATYYCLQHSSYPLTFGGGTKVEIK | QLQLQESGPGLVKFSETLSLTCTVSGGSISRSSYYWGWIRQPPGKGLEWIGNIYYSGYTYYTPSLKSRVTISVDTSKNQFSLKLSSVTAADTAVYYCARHSTSWSLDYWGQGTLVTVSS |
| | | | SEQ ID NO: 24314 | SEQ ID NO: 28320 |

FIGURE 50

| | | | | |
|---|---|---|---|---|
| **iPS:392708** | **21-225_18D11** | NA | GACATCCAGATGACCCAGTCTCCTTCCTCACTGTTTGCATTTGTAGGAGACAGAGTCACCATCACTTGTCGGGCGAGTCAGGGCATTAGCTATTATTTAGCCTGGTTTCAGCAGAAACCAGGGAAAGCCCCTAAGTCCCTGATTTATGTTGCATCCAGTTTGCAAAGTGGGGTCCCATCAAAGTTCAGCGGCAGTGGATTTGGGACAGATTTCACTCTCACCATCAGCAGCCTGCAGCCTGAAGATTTTGCAACTTATTACTGCCAACAGTATAATACTTACCCATTCACTTTCGGCCCTGGGACCACAGTGGATATCAAG | GAGGTGCAGCTGGTGGAGTCTGGGGGAGGCTTGGTACAGCCTGGGGGGTCCCTGAGACTCTCCTGTGCAGCCTCTGGATTCACCTTCAGAAGCTATAGCATGAACTGGGTCCGCCAGGCTCCAGGGAAGGGGCTGGAGTGGCTTTCATACATTAGTAGTAGTAGTGGTACCATATACTACGCAGACTCTGTGAAGGGCCGATTCACCATCTCCAGAGACAACGCCAGGAACTCACTGAATCTGCAAATGAACAGCCTGAGAGACGAGGACACGGCTGTGTATTACTGTGCGAGAGGAGGTGGGAGCCCTTTTGACTACTGGGGCCAGGGAATCCTGGTCACCGTCTCCTCA |
| | | | SEQ ID NO: 24315 | SEQ ID NO: 28321 |
| | | AA | DIQMTQSPSSLFAFVGDRVTITCRASQGISYYLAWFQQKPGKAPKSLIYVASSLQSGVPSKFSGSGFGTDFTLTISSLQPEDFATYYCQQYNTYPFTFGPGTTVDIK | EVQLVESGGGLVQPGGSLRLSCAASGFTFRSYSMNWVRQAPGKGLEWLSYISSSSGTIYYADSVKGRFTISRDNARNSLNLQMNSLRDEDTAVYYCARGGGSPFDYWGQGILVTVSS |
| | | | SEQ ID NO: 24316 | SEQ ID NO: 28322 |
| **iPS:392710** | **21-225_19A10** | NA | GACATCCAGATGACCCAGTCTCCATCCTCCCTGTCTGCATCTGTAGGAGACAGAGTCACCATCACTTGCCGGGCAAGTCAGGGCATTAGAACTGATTTAGGCTGGTATCAGCAGAGACCAGGGAAAGCCCCTAAGCGCCTGATCTATACTGCATCCAGTTTGCAAAGTGGGGTCCCATCAAGGTTCAGCGGCAGTGGATCTGGGACAGAATTCACTCTCACAATCAGCAGGCTGCAGCCTGAAGATTTTGCAACTTATTACTGTCTACAGCATAATGGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGAAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTATATTACTGTGCGAGAGAGCTTGCCTGGTACGAGGACTCCTGGGGCCAGGGAACCCTGGTCACCGTCTCCTCA |
| | | | SEQ ID NO: 24317 | SEQ ID NO: 28323 |

FIGURE 50

| | | | | |
|---|---|---|---|---|
| | | AA | DIQMTQSPSSLSASVGDRVTITCRASQGIRTDLGWYQQRPGKAPKRLIYTASSLQSGVPSRFSGSGSGTEFTLTISRLQPEDFATYYCLQHNGYPWTFGQGTKVEIK | QVQLVESGGGVVQPGRSLRLSCAASGFTFSSYGMHWVRQAPGKGLEWVAVIWYDESNKYYADSVKGRFTISRDNSKNTLYLQMNSLRAEDTAVYYCARELAWYEDSWGQGTLVTVSS |
| | | | SEQ ID NO: 24318 | SEQ ID NO: 28324 |
| **iPS:392714** | **21-225_16G12** | NA | GACATCCAGATGACCCAGTCTCCATCCTCACTGTCTGCATCTGTTGGAGACAGAGTCACCATCACTTGTCGGGCGAGTCAGGACATTAGCAATTATTTAGCCTGGTTTCAGCAGAAACCAGGGAAAGCCCCTAAGTCCCTGATCTATGCTGCATCCAGTTTGCAAAGTGGGGTCCCATCAAAGTTCAGAGGCAGTGGATCTGGGACAGATTTCACTCTCACCATCAGCAACCTGCAGCCTGAAGATTTTGCATCTTATTACTGCCAACAGTATCATAGTTTCCCATTCACTTTCGGCCCTGGGACCAAAGTGGATATCAAA | GAGGTGCAACTGTTGGAGTCGGGGGGAGGCTTGGTACAGCCTGGGGGGTCCCTGAGACTCTCCTGTGCAGCCTCTGGATTCTCCTTTAGTAGCTATGCCATGACCTGGGTCCGCCAGGCTCCAGGGAAGGGGCTGGAGTGGGTCTCAACTATTAGTGGTCGTGGTGGTCACACATACTACGCAGACTCCGTGAGGGGCCGGTTCGCCATCTCCAGAGACAGTTCCAAGAACACGCTGTATCTGCAAATGAACAGCCTGAGAGCCGAGGACACGGCCGTATATTACTGTGCGAAACAGGACTGCTGGGGCCAGGGAACCCTGGTCACCGTCTCCTCA |
| | | | SEQ ID NO: 24319 | SEQ ID NO: 28325 |
| | | AA | DIQMTQSPSSLSASVGDRVTITCRASQDISNYLAWFQQKPGKAPKSLIYAASSLQSGVPSKFRGSGSGTDFTLTISNLQPEDFASYYCQQYHSFPFTFGPGTKVDIK | EVQLLESGGGLVQPGGSLRLSCAASGFSFSSYAMTWVRQAPGKGLEWVSTISGRGGHTYYADSVRGRFAISRDSSKNTLYLQMNSLRAEDTAVYYCAKQDCWGQGTLVTVSS |
| | | | SEQ ID NO: 24320 | SEQ ID NO: 28326 |

FIGURE 50

| | | | | |
|---|---|---|---|---|
| **iPS:392716** | **21-225_17B5** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAATTTGCAAAGTGGGGTCCCATCAAGGTTCAGCGGCAGTGGATCTGGGACAGAATTCAGTTTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGAAAGTAATAAACACTATATAGACTCCGTGAAGGGCCGATTCACCATCTCCAGAGACAATTCCAAGAACACGCTGTATCTGCAAATGAACAGCCTGAGAGCCGAGGACACGGCTGTGTATTATTGCGTGAGAGAACTGGGGTTCCGGTTTGACTACTGGGGCCAGGGAACCCTGGTCACCGTCTCCTCA |
| | | | SEQ ID NO: 24321 | SEQ ID NO: 28327 |
| | | AA | DIQMTQSPSSLSASVGDRVTITCRASQGIRNDLGWYQQKPGKAPKRLIYAASNLQSGVPSRFSGSGSGTEFSFTISSLQPEDFATYYCLQHNSYPLTFGGGTKVEIK | QVQLVESGGGVVQPGRSLRLSCAASGFTFSDYGMHWVRQAPGKGLEWVAVIWYDESNKHYIDSVKGRFTISRDNSKNTLYLQMNSLRAEDTAVYYCVRELGFRFDYWGQGTLVTVSS |
| | | | SEQ ID NO: 24322 | SEQ ID NO: 28328 |
| **iPS:392718** | **21-225_17B8** | NA | GATATTGTGATGACTCAGTCTCCACTCTCCCTGCCCGTCACCCCTGGAGAGCCGGCCTCCATCTCCTGTAGGTCTAGTCAGAGCCTCCTGCATAGTAATGGGAACAACTCTTTGGATTGGTACCTGCAGAAGCCAGGGCAGTCTCCACAGCTCCTGATCTATTTGGGTTCTCATCGGGCCTCCGGGGTCCCTGACAGGTTCAGTGACAGTGGATCAGGCACAGATTTTACACTGAAAATCAGCAGAGTGGAGGCTGAGGATGTTGGGGTTTATTACTGCATGCAAGTTCTACAAACTCCTCCCCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCGTCTGGATACACCTTCACCAGCTATGCTATCAACTGGGTGCGACAGGCCACTGGACAAGGGCTTGAGTGGATGGGATGGATGAACCCTAACACTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTATATTTCTGTACGAGAAAGGCTGGGTTTGACTACTGGGGCCAGGGAACCCTGGTCACCGTCTCCTCA |
| | | | SEQ ID NO: 24323 | SEQ ID NO: 28329 |

FIGURE 50

| | | | | |
|---|---|---|---|---|
| | | AA | DIVMTQSPLSLPVTPGEPASISCRSSQSLLHSNGNNSLDWYLQKPGQSPQLLIYLGSHRASGVPDRFSDSGSGTDFTLKISRVEAEDVGVYYCMQVLQTPPLTFGGGTKVEIK | QVQLVQSGAEVKKPGASVKVSCKASGYTFTSYAINWVRQATGQGLEWMGWMNPNTGNTGYAQKFQGRVTMTRNTSISTAYMELSSLRSEDTAVYFCTRKAGFDYWGQGTLVTVSS |
| | | | SEQ ID NO: 24324 | SEQ ID NO: 28330 |
| iPS:392720 | 21-225_17A12 | NA | GACATCCAGATGACCCAGTCTCCATCCTCCCTGTCTGCATCTGTAGGAGACAGAGTCACCATCACTTGCCGGGCAAGTCAGAGCATTAGTAGTTATTTAAATTGGTATCACCAGAAACCAGGGAAAGCCCCTAAGCTCCTGATCTATGCTGCATCCAGTTTGCAAAGTGGGGTCCCATCAAGGTTCAGTGGCAGTGGATCTGGGACAGATTTCACTCTCACCATCAGCAATCTGCAACCTGAAGATTTTGCAACTTACTACTGTCAACAGAGTTACAATACCCCCTTATTCACTTTCGGCCCTGGGACCAAAGTGGATATCAAA | GAGGTGCAGCTGTTGGAGTCTGGGGGAGGCTTGATACAGCCTGGGGGGTCCCTGAGACTCTCCTGTGCAGCCTCTGAATTCACCTTTAGCAGCTATGCCATGAGCTGGGTCCGCCAGGATCCAGGGAAGGGGCTGGAGTGGGTCTCAATTATTAGTGGTCGTGGGGGAAACGCATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGTCCCGTATAGCAGTGGCTGGCTCGGAGGCTTTTGATATCTGGGGCCAAGGGACAATGGTCACCGTCTCTTCA |
| | | | SEQ ID NO: 24325 | SEQ ID NO: 28331 |
| | | AA | DIQMTQSPSSLSASVGDRVTITCRASQSISSYLNWYHQKPGKAPKLLIYAASSLQSGVPSRFSGSGSGTDFTLTISNLQPEDFATYYCQQSYNTPLFTFGPGTKVDIK | EVQLLESGGGLIQPGGSLRLSCAASEFTFSSYAMSWVRQDPGKGLEWVSIISGRGGNAFYADSVKGRFTISRDNSKNTLYLQMNSLRAEDTAVYYCASRIAVAGSEAFDIWGQGTMVTVSS |
| | | | SEQ ID NO: 24326 | SEQ ID NO: 28332 |

FIGURE 50

| | | | | |
|---|---|---|---|---|
| **iPS:392722** | **21-225_18E12** | NA | GACATCCAGATGACCCAGTCTCCATCCTCCCTGTCTGCATCTGTAGGAGACAGAGTCACCATCACTTGCCGGGCAAGTCAGAGCATTAGTAGTTATTTAAATTGGTATCAGCAGAAACCAGGGAAAGCCCCTACGCTCCTGATCTATGCTGCATCCAGTTTACAAAGTGGGGTCCCATCAAGGTTCAGTGGCAGTGGATCTGGGACAGATTTCACTCTCACCATCAACAGTCTGCAACCTGAAGATTTTGCAACTTACTTCTGTCAACAGAGTTACAGAACCCCCTTTTTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGAATTCACCTTTAGCAGCTATGCCATGAGCTGGGTCCGCCAGGCTCCAGGGACGGGGCTGGAGTGGGTCTCAATTATTAGTGGTCGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGTCCCGTCTGGCAGTGGCTGGCTCGGAGGCTTTTGATATCTGGGGCCAAGGGACGATGGTCACCGTCTCTTCA |
| | | | SEQ ID NO: 24327 | SEQ ID NO: 28333 |
| | | AA | DIQMTQSPSSLSASVGDRVTITCRASQSISSYLNWYQQKPGKAPTLLIYAASSLQSGVPSRFSGSGSGTDFTLTINSLQPEDFATYFCQQSYRTPFFTFGPGTKVDIK | EVQLLESGGGLVQPGGSLRLSCAASEFTFSSYAMSWVRQAPGTGLEWVSIISGRGGNTFYADSVKGRFTISRDNSKNTLYLQMNSLRAEDTAVYYCASRLAVAGSEAFDIWGQGTMVTVSS |
| | | | SEQ ID NO: 24328 | SEQ ID NO: 28334 |
| **iPS:392726** | **21-225_20B5** | NA | GACATCCAGATGACCCAGTCTCCATCCTCCCTGTCTGCATCTGTAGGAGACAGAGTCACCATCACTTGCCGGGCGAGTCAGGGCATTAACAATTATTTAGCCTGGTATCAGCAGAAACCAGGGAAAATTCCTAAGCTCCTGATCTATGCTGCATCCACTTTGCAATCAGGGGTCCCCTCTCGGTTCAGTGGCAGTGGATCTGGGACAGATTTCACTCTCACCATCAGCAGCCTGCAGCCTGAAGATGTTGCAACTTATTGCTGTCAAAAGTATAACAGTGCCCCTCCGATCACCTTCGGCCAAGGGACACGACTGGAGATTAAA | GAGGTGCAGCTGGTGGAGGCCGGGGGAGGCCTGGTCAAGCCGGGGGGGTCCCTGAGACTCTCCTGTGCAGCCTCTGGATTCACCTTCACCAGCTATAGCATGAACTGGGTCCGCCAGGCTCCAGGGAAGGGGCTGGAGTGGGTCTCATCCATTAGTGGGAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGATCGTGGGAGCTACTGGGGCCAGGGTACCCTGGTCACCGTCTCCTCA |
| | | | SEQ ID NO: 24329 | SEQ ID NO: 28335 |

FIGURE 50

| | | | | |
|---|---|---|---|---|
| | | AA | DIQMTQSPSSLSASVGDRVTITCRASQGINNYLAWYQQKPGKIPKLLIYAASTLQSGVPSRFSGSGSGTDFTLTISSLQPEDVATYCCQKYNSAPPITFGQGTRLEIK | EVQLVEAGGGLVKPGGSLRLSCAASGFTFTSYSMNWVRQAPGKGLEWVSSISGSSSYIYYADSVKGRFTISRDNAKNSLYLQMNSLRAEDTAVYYCARDRGSYWGQGTLVTVSS |
| | | | SEQ ID NO: 24330 | SEQ ID NO: 28336 |
| iPS:392728 | 21-225_20F7 | NA | GACATCCAGATGACCCAGTCTCCATCTTCCGTGTCTGCATCTGTAGGAGACGGAGTCACCATCACTTGTCGGGCGAGTCAGGGTATTAGCAGCTGGTTAGCCTGGTATCAGCAGAAACCAGGGAAAGCCCCTAAACTCTTGATTTATGCTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGGCTAACAGTTTCCCTCGGACGTTCGGCCAAGGGACCAAGGTGGAAATCAAA | CAGGTGCAGCTGGTGGAGTCTGGGGGAGGCTTGGTCAAGCCTGGAGGGTCCCTGAGACTCTCCTGTGCAGCCTCTGGATTCACCTTCAGTGACTATTACATGAGCTGGATCCGCCAGGCTCCAGGGAAGGGGCTGGAGTGGGTTTCACACATTAGTAGTAGTGGTAGTACCATATACTACGCAGACTCTGTGAAGGGCCGATTCACCATCTCCAGGGACAACGGCGAGAACTCACTGTATCTGCAAATGAACAGCCTGAGAGCCGAGGACACGGCCGTGTATTACTGTGCGAGATATCGGAATAACCGGGGGTACTTCGATCTCTGGGGCCGTGGCTCCCTGGTCACTGTCTCCTCA |
| | | | SEQ ID NO: 24331 | SEQ ID NO: 28337 |
| | | AA | DIQMTQSPSSVSASVGDGVTITCRASQGISSWLAWYQQKPGKAPKLLIYAASSLQSGVPSRFSGSGSGTDFTLTISSLQPEDFATYYCQQANSFPRTFGQGTKVEIK | QVQLVESGGGLVKPGGSLRLSCAASGFTFSDYYMSWIRQAPGKGLEWVSHISSSGSTIYYADSVKGRFTISRDNGENSLYLQMNSLRAEDTAVYYCARYRNNRGYFDLWGRGSLVTVSS |
| | | | SEQ ID NO: 24332 | SEQ ID NO: 28338 |

FIGURE 50

| | | | | |
|---|---|---|---|---|
| **iPS:392730** | **21-225_17A1** | NA | GACATCCAGATGACCCAGTCTCCATCCTCCCTGTCTGCATCTGTAGGAGACAGAGTCACCATCACCTGCCGGGCAAGTCAGAACATTAACAATTATTTAAATTGGTATCAGCAGAAACCAGGGAAAGGCCCTAAGGTCCTGATCTTTACTACATCTAGTTTACAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACACTACCCCCACGTGGACGTTCGGCCAAGGGACCAAGGTGGAAATCAAA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATGCCATGAACTGGGTCCGCCAGGCTCCAGGGAAGGGGCTGGAGTGGGTCTCAGTTATTAGTGGTAGTGGTAGTAACACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAAAGATATACCAGTGACTGGCATGATGCTTTTGATATCTGGGGCCAAGGGACAATGGTCACCGTCTCTTCA |
| | | | SEQ ID NO: 24333 | SEQ ID NO: 28339 |
| | | AA | DIQMTQSPSSLSASVGDRVTITCRASQNINNYLNWYQQKPGKGPKVLIFTTSSLQSGVPSRFSGSGSGTDFTLTISSLQPEDFATYYCQQSYTTPTWTFGQGTKVEIK | EVQLLESGGGLVQPGGSLRLSCAASGFTFSSYAMNWVRQAPGKGLEWVSVISGSGSNTYYADSVKGRFTISRDNSKNTLYLQMNSLRAEDTAVYYCAKRYTSDWHDAFDIWGQGTMVTVSS |
| | | | SEQ ID NO: 24334 | SEQ ID NO: 28340 |
| **iPS:392732** | **21-225_17E5** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATACTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAACATAATAGTTACCCGTTGACGTTCGGCCCAGGGACCAAGGTGGTCATCAAA | CAGGTGCAACTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCACTTATATGGTATGATGTAACTAATAAATACTATGGAGACTCCGTGAAGGGCCGATTCACCATCTCCAGAGACAATTCCCAGAACACGCTGTATCTGCAACTGAACAGCCTGAGAGCCGAGGACACGGCTGTGTATTACTGTGCGAGAGAGCTTGGCTGGTACGAGGACTACTGGGGCCAGGGAACCCTGGTCACCGTCTCCTCA |
| | | | SEQ ID NO: 24335 | SEQ ID NO: 28341 |

FIGURE 50

| | | | | |
|---|---|---|---|---|
| | | AA | DIQMTQSPSSLSASVGDRVTITCRASQGIRNDLGWYQQKPGKAPKRLIYTASSLQSGVPSRFSGSGSGTEFTLTISSLQPEDFATYYCLQHNSYPLTFGPGTKVVIK | QVQLVESGGGVVQPGRSLRLSCAASGFTFSDYGMHWVRQAPGKGLEWVALIWYDVTNKYYGDSVKGRFTISRDNSQNTLYLQLNSLRAEDTAVYYCARELGWYEDYWGQGTLVTVSS |
| | | | SEQ ID NO: 24336 | SEQ ID NO: 28342 |
| **iPS:392734** | **21-225_17D8** | NA | GAAATAGTGATGACGCAGTCTCCATCCACCCTGTCTGTGTCTCCAGGGGAAAGAGCCACCCTCTCCTGCAGGGCCAGTCAGAGTGTTAGCAGCAACTTAGCCTGGTTCCAGCAGAAACCTGGCCAGGCTCCCAGGCTCCTCATCAATGGTGCATCCACCAGGGCCAGTGGTATCCCAGCCAGGTTCAGTGGCAGTGGGTCTGGGACAGAGTTCACTCTCACCATCAGCAGCCTGCAGTCTGAAGATTTTGCAGTTTATTACTGTCAGCAGTATAATAACTGGCCTCTGACGTTCGGCCAAGGGACCAAGGTGGAAATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATGGCTTGAACTGGGTCCGCCAGGCTCCAGGGAAGGGGCTGGAGTGGGTCTCATCCATTAGTGGTAGTGGTAGTCACATATCCTACGCGGACTCAGTGAAGGGCCGATTCACCATCTCCAGAGACAACGCCAAGAACTCACTGTATCTGCAACTGAACAGCCTGAGAGCCGAGGACACGGCTGTGTATTACTGTGCGAGAGATCGGGGCAGTGGCTGGGGCCAGGGAACCCTGGTCACCGTCTCCTCA |
| | | | SEQ ID NO: 24337 | SEQ ID NO: 28343 |
| | | AA | EIVMTQSPSTLSVSPGERATLSCRASQSVSSNLAWFQQKPGQAPRLLINGASTRASGIPARFSGSGSGTEFTLTISSLQSEDFAVYYCQQYNNWPLTFGQGTKVEIK | EVQLVESGGGLVKPGGSLRLSCAASGFTFSSYGLNWVRQAPGKGLEWVSSISGSGSHISYADSVKGRFTISRDNAKNSLYLQLNSLRAEDTAVYYCARDRGSGWGQGTLVTVSS |
| | | | SEQ ID NO: 24338 | SEQ ID NO: 28344 |

FIGURE 50

| | | | | |
|---|---|---|---|---|
| **iPS:392736** | **21-225_17B12** | NA | GACATCCAGATGACCCAGTCTCCATCCTCCCTGTCTGCATCTGTAGGAGACAGAGTCTCCATCACTTGCCGGGCAAGTCAGAATATTAACAACTATTTAAATTGGTATCAGCAGAAACCAGGGAAAGGCCCTAAGGTCCTGATCCTTACTGCATCCAGTTTGCAAAGTGGGGTCCCATCAAGGTTCAGTGGCAGTGGATCTGGGACAGATTTCACTCTCACCATCAGCAGTCTACAACCTGAAGATTTTGCAACTTACTACTGTCAACAGACTTACACTACCCCCACGTGGACGTTCGGCCAAGGGACCAAGGTGGAAATCAAA | GAGGTGCAGCTGTTGGAGTCTGGGGGAGGCTTGGTACAGCCTGGGGGGTCCCTGAGACTCTCCTGTGTAGCCTCTGGATTCACCTTTAGCAGCTATGCCATGAACTGGGTCCGCCAGGCTCCAGGGAAGGGGCTGGAGTGGGTCTCAGTTATTAGTGGTAGTGGTGGTAGCACATACTACGCAGACTCCGTGAAGGGCCGGTTCACCATCTCCAGAGACAATTCCAAGAACACGCTGTATCTGCAAATGAACACCCTGAGAGCCGAGGACACGGCCGTATATTACTGTGCGAAAAGGTATACCAGTGACTGGCATGATGCTTTTGATATCTGGGGCCAAGGGACAATGGTCACCGTCTCTTCA |
| | | | SEQ ID NO: 24339 | SEQ ID NO: 28345 |
| | | AA | DIQMTQSPSSLSASVGDRVSITCRASQNINNYLNWYQQKPGKGPKVLILTASSLQSGVPSRFSGSGSGTDFTLTISSLQPEDFATYYCQQTYTTPWTFGQGTKVEIK | EVQLLESGGGLVQPGGSLRLSCVASGFTFSSYAMNWVRQAPGKGLEWVSVISGSGGSTYYADSVKGRFTISRDNSKNTLYLQMNTLRAEDTAVYYCAKRYTSDWHDAFDIWGQGTMVTVSS |
| | | | SEQ ID NO: 24340 | SEQ ID NO: 28346 |
| **iPS:392738** | **21-225_18G4** | NA | GACATCCACATGACCCAGTCTCCATCCTCCCTGTCTGCATCTGTAGGAGACAGAGTCACCATCACTTGCCGGGCAAGTCAGAGCATTATCAGCTATTTAAATTGGTATCAGCAGAAACCAGGGAAAGCCCCTAAGGTCCTGATCTATACTGCATCCAGTTTGCAAACTGGGGTCCCATCAGGGTTCAGTGGCAGTGGATCTGGGACAGATTTCACTCTCACCATCAGCAGTCTGCAACCTGAAGATTTTGCAACTTACTACTGTCAACAGACTTACAGTCCCCCGCTCACTTTCGGCGGTGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAATTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCTGAGTATGGGCGGTATGGACGTCTGGGGCCAAGGGACCACGGTCACCGTCTCCTCA |
| | | | SEQ ID NO: 24341 | SEQ ID NO: 28347 |

FIGURE 50

| | | | | |
|---|---|---|---|---|
| | | AA | DIHMTQSPSSLSASVGDRVTITCRASQSIISYLNWYQQKPGKAPKVLIYTASSLQTGVPSGFSGSGSGTDFTLTISSLQPEDFATYYCQQTYSPPLTFGGGTKVEIK | QVQLVESGGGVVQPGRSLRLSCAASGFTFSSYGMHWVRQAPGKGLEWVAIIWYDGSNKYYADSVKGRFTISRDNSKNTLYLQMNSLRAEDTAVYYCARDLSMGGMDVWGQGTTVTVSS |
| | | | SEQ ID NO: 24342 | SEQ ID NO: 28348 |
| **iPS:392740** | **21-225_18H12** | NA | GACATCCAGATGACCCAGTCTCCATCCTCCCTGTCTGCGTCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATACTGCATCCAATTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAACATAATAATTACCCGTGGACGTTCGGCCTAGGGACCAAGGTGGTCATCAAA | CAGGTGCAACTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGATGGCAGTTATATGGTATGATGTAACTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTTTATTACTGTGCGAGAGAGGTTGGCTGGTACGAGGACTACTGGGGCCAGGGAACCCTGGTCACCGTCTCCTCA |
| | | | SEQ ID NO: 24343 | SEQ ID NO: 28349 |
| | | AA | DIQMTQSPSSLSASVGDRVTITCRASQGIRNDLGWYQQKPGKAPKRLIYTASNLQSGVPSRFSGSGSGTEFTLTISSLQPEDFATYYCLQHNNYPWTFGLGTKVVIK | QVQLVESGGGVVQPGRSLRLSCAASGFTFSDYGMHWVRQAPGKGLEWMAVIWYDVTNKYYADSVKGRFTISRDNSKNTLYLQMNSLRAEDTAVYYCAREVGWYEDYWGQGTLVTVSS |
| | | | SEQ ID NO: 24344 | SEQ ID NO: 28350 |

FIGURE 50

| | | | | |
|---|---|---|---|---|
| **iPS:392742** | **21-225_20B2** | NA | GACATCCAGATGACCCAGTCTCCATCCTCCCTGTCTGCCTCTGTAGGAGACAGAGTCAATATCACTTGCCGGGCAAGTCAGGACATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACGATCAGCAGCCTGCAGCCTGAAGATTTTGCAACTTATTACTGTCTACAGCATTATAATTACCCTCGGGCGTTCGGCCAGGGGACCAAGGTGGATATCAAA | CAGGTGCAACTGGTGGAGTCTGGGGGAGGCGTGGTCCAGCCTGGGAGGTCCCTGAGACTCTCCTGTGCAGCGTCTGGATTCACCTTCAGTAATTATGTCATTCACTGGGTCCGCCAGGCTCCAGGCAAGGGACTGGAGTGGGTGGCAGTTATATGGTATGATGGAAGTAATAAATACTATGCAGACTCCGTGAAGGGCCGCTTCACCATCTCCAGAGACAATTCCAAGAACACGCTGTATCTGCAAATGAACAGCCTGAGAGCCGAGGACACGGCTGTGTATTCCTGTGCGAGAGAGAAGTATAGCAGCAGCTGGTACGACTACGGTATGGACGTCTGGGGCCAAGGGACCACGGTCACCGTCTCCTCA |
| | | | SEQ ID NO: 24345 | SEQ ID NO: 28351 |
| | | AA | DIQMTQSPSSLSASVGDRVNITCRASQDIRNDLGWYQQKPGKAPKRLIYAASSLQSGVPSRFSGSGSGTEFTLTISSLQPEDFATYYCLQHYNYPRAFGQGTKVDIK | QVQLVESGGGVVQPGRSLRLSCAASGFTFSNYVIHWVRQAPGKGLEWVAVIWYDGSNKYYADSVKGRFTISRDNSKNTLYLQMNSLRAEDTAVYSCAREKYSSSWYDYGMDVWGQGTTVTVSS |
| | | | SEQ ID NO: 24346 | SEQ ID NO: 28352 |
| **iPS:392744** | **21-225_20D5** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGTTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATTTGGTATGAAGAAAATAATCAATACTATGCAGACTCCGTGAAGGGCCGATTCACCATCTCCAGAGACAATTCCAAGAACACGCTGTATCTGCAAATGAACAGCCTGAGAGCCGACGACACGGCTGTGTATTACTGTGCGAGAGAACTGGGGTTCCGGTCTGACTACTGGGGCCAGGGAACCCTGGTCACCGTCTCCTCA |
| | | | SEQ ID NO: 24347 | SEQ ID NO: 28353 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPFTFGGGTKVEIK | QVQLVESGGGVVQPGRSLRLSCAASGFTFSSYGMHWVRQAPGKGLEWVAVIWYEENNQYYADSVKGRFTISRDNSKNTLYLQMNSLRADDTAVYYCARELGFRSDYWGQGTLVTVSS |
| | | | SEQ ID NO: 24348 | SEQ ID NO: 28354 |
| iPS:392746 | 21-225_20H7 | NA | GACATCCAGATGACCCAGTCTCCATCCTCACTGTCTGCATCTGTAGGAGACAGAGTCACCATCACTTGTCGGACGAGTCAGGGCATTAACAATTATTTAGTCTGGTTTCAGCAGAAACCAGGGAAAGCCCCTAAGCGCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATTATAGTTACCCATTCACTTTCGGCCCTGGGACCAAAATGGATTTCAAA | GAGGTGCACCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTAGTTTCATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGTAGCAGCTCTTGACTACTGGGGCCAGGGAACCCTGGTCACCGTCTCCTCA |
| | | | SEQ ID NO: 24349 | SEQ ID NO: 28355 |
| | | AA | DIQMTQSPSSLSASVGDRVTITCRTSQGINNYLVWFQQKPGKAPKRLIYAASSLQSGVPSKFSGSGSGTDFTLTISSLQPEDFATYYCQQYYSYPFTFGPGTKMDFK | EVHLVESGGGLVKPGGSLRLSCAASGFTFSSYSMNWVRQAPGKGLEWVSSISGSSSFIYYADSVKGRFTISRDNAKNSLYLQMNSLRAEDTAVYYCARVAALDYWGQGTLVTVSS |
| | | | SEQ ID NO: 24350 | SEQ ID NO: 28356 |

FIGURE 50

| | | | | |
|---|---|---|---|---|
| **iPS:392748** | **21-225_20A8** | NA | GACATCCAGATGACCCAGTCTCCATCCTCACTGTCTGCATCTGTAGGAGACAGAGTCACCATCACTTGTCGGGCGAGTCAGGGCATTAATAATTATTTAGTCTGGTTTCAGCAGAAACCAGGGAAAGCCCCTAAGTCCCTGATCTATGCTGCATCCAGTTTGCTGAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATAATAGTTACCCGATCACCTTCGGCCAAGGGACACGACTGGAGATTAAA | GAGGTGCAGCTGGTGGAGTCTGGGGGAGGCCTGGTCAAGCCTGGGGGGTCCCTAAGACTCTCCTGTGCAGCCTCTGGATTCACCTTCAGTAGCTATAGCGTGAACTGGGTCCGCCGGGCTCCAGGGAAGGGGCTGGAGTGGGTCTCATCCATTAGTAGTAGTAGTAGTTTCCTATACTACGCAGACTCAGTGAAGGGCCGATTCACCATCTCCAGAGACAACGCCAAGAACTCAGTGTATCTGCAAATGAACAGCCTGAGAGCCGAGGACACGGCTGTATATTACTGTGCGAGAAACTGGGACTACTGGGGCCAGGGAACCCTGGTCACCGTCTCCTCA |
| | | | SEQ ID NO: 24351 | SEQ ID NO: 28357 |
| | | AA | DIQMTQSPSSLSASVGDRVTITCRASQGINNYLVWFQQKPGKAPKSLIYAASSLLSGVPSKFSGSGSGTDFTLTISSLQPEDFATYYCQQYNSYPITFGQGTRLEIK | EVQLVESGGGLVKPGGSLRLSCAASGFTFSSYSVNWVRRAPGKGLEWVSSISSSSSFLYYADSVKGRFTISRDNAKNSVYLQMNSLRAEDTAVYYCARNWDYWGQGTLVTVSS |
| | | | SEQ ID NO: 24352 | SEQ ID NO: 28358 |
| **iPS:392750** | **21-225_20A10** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACAAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACCATCAGCAGCCTGCAGCCTGAAGATTTTGCAACTTATTACTGTCTACAGCATAATAGATACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCCTCAGTAGCGATGACATGCACTGGGTCCGCCAGGCTCCAGGCAAGGGGCTGGAGTGGGTGGCAGTTATATGGTTTGATGGAAGTAATAAATACTATGCAGACTCCGTGAAGGGCCGATTCATCATCTCCAGAGACAATTCCAAGAACACGCTGTATCTGCAAATGAACAGCCTGAGAGCCGAGGACACGGCTATGTATTACTGTGCGAGAGATCTAATAGCAGCAGCTGGTACGGTTGACTACTGGGGCCAGGGAACCCTGGTCACCGTCTCCTCA |
| | | | SEQ ID NO: 24353 | SEQ ID NO: 28359 |

FIGURE 50

| | | | | |
|---|---|---|---|---|
| | | AA | DIQMTQSPSSLSASVGDRVTITCRASQGIRNDLGWYQQKQGKAPKRLIYAASSLQSGVPSRFSGSGSGTEFTLTISSLQPEDFATYYCLQHNRYPLTFGGGTKVEIK | QVQLVESGGGVVQPGRSLRLSCAASGFTLSSDDMHWVRQAPGKGLEWVAVIWFDGSNKYYADSVKGRFIISRDNSKNTLYLQMNSLRAEDTAMYYCARDLIAAAGTVDYWGQGTLVTVSS |
| | | | SEQ ID NO: 24354 | SEQ ID NO: 28360 |
| **iPS:392754** | **21-225_21D3** | NA | GACATCCAGATGACCCAGTCTCCATCCTCCCTGTCTGCATCTGTAGGAGACAGAGTCACCATCACTTGCCGGGCAAGTCAGAGCATTACTGGTTATTCAAATTGGTATCAGCAGAAGCCAGGGAAAACCCCTAAACTCCTGATCTTTGCTACATACAGTTTGGAAAGTGGGGTCCCATCAAGGTTCAGTGGCAGTGGATTTGGGACAAATTTCACTCTCACCATCACCAGTCTGCAACCTGAAGATTTTGCAACTTACTACTGTCAACAGAGTTACAGTACCTCGATCACCTTCGGCCAAGGGACACGACTGGAGATTAAA | CAGGTGCAGCTGGTGGAGTCTGGGGGAGGCGTGGTCCAGCCTGGGAGGTCCCTGAGACTCTCCTGTGTTGCCTCTGGATTCACCTTCAGTAGCTATGGCATGCACTGGGTCCGCCAGGCTCCAGGCAAGGGGCTGGAGTGGGTGACAGTTATATCATATGATGGAAGTAATAAATACTATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTACTGTGCGAGAGGGGTATGGTTCGGGGACCTCTGGGGCCAGGGAACCCTGGTCACCGTCTCCTCA |
| | | | SEQ ID NO: 24355 | SEQ ID NO: 28361 |
| | | AA | DIQMTQSPSSLSASVGDRVTITCRASQSITGYSNWYQQKPGKTPKLLIFATYSLESGVPSRFSGSGFGTNFTLTITSLQPEDFATYYCQQSYSTSITFGQGTRLEIK | QVQLVESGGGVVQPGRSLRLSCVASGFTFSSYGMHWVRQAPGKGLEWVTVISYDGSNKYYADSVKGRFTISRDNSKNTLYLQMNSLRAEDTAVYYCARGVWFGDLWGQGTLVTVSS |
| | | | SEQ ID NO: 24356 | SEQ ID NO: 28362 |

FIGURE 50

| | | | | |
|---|---|---|---|---|
| **iPS:392758** | **21-225_21G11** | NA | GACATCCAGATGACCCAGTCTCCATCCTCCCTGTCTGCGTCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATACTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAACATAATAATTACCCGTGGACGTTCGGCCTAGGGACCAAGGTGGTCATCAAA | CAGGTGCAACTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGATGGCAGTTATATGGTATGATGTAACTAATGAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTTTATTACTGTGCGAGAGAGCTTGGCTGGTACGAGGACTACTGGGGCCAGGGAACCCTGGTCACCGTCTCCTCA |
| | | | SEQ ID NO: 24357 | SEQ ID NO: 28363 |
| | | AA | DIQMTQSPSSLSASVGDRVTITCRASQGIRNDLGWYQQKPGKAPKRLIYTASSLQSGVPSRFSGSGSGTEFTLTISSLQPEDFATYYCLQHNNYPWTFGLGTKVVIK | QVQLVESGGGVVQPGRSLRLSCAASGFTFSDYGMHWVRQAPGKGLEWMAVIWYDVTNEYYADSVKGRFTISRDNSKNTLYLQMNSLRAEDTAVYYCARELGWYEDYWGQGTLVTVSS |
| | | | SEQ ID NO: 24358 | SEQ ID NO: 28364 |
| **iPS:392760** | **21-225_22G3** | NA | GACATCCAGATGACCCAGTCTCCATCCTCCCTGTCTGCATCTGTAGGAGACAGAGTCACCATCACTTGCCGGGCAAGTCAGAGCATTAGTAATTATTTAAATTGGTATCAGCAGAAACCAGGGAAAGCCCCTAAGCTCCTGATCTTTGCTGCTTCCAGTTTGCAAAGTGGGGTCCCATCAAGGTTCAGTGGCAGTGGATCTGGGACAGATTTCACTCTCACCATCAGCGGTCTACAACCTGAAGATTTTGCAACTTACTTCTGTCAACAGAGTTTCAGAACCCCCTTTTTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGAATTCACCTTTAGCAGCTATGCCATGAACTGGGTCCGCCAGGCTCCAGGGAAGGGGCTGGAGTGGGTCTCAATTATTAGTGGTCGTGGTGTTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGTCCCGTTTGGCAGTGGCTGGCTCGGAGGCTTTTGATATCTGGGGCCAAGGGACAATGGTCACCGTCTCTTCA |
| | | | SEQ ID NO: 24359 | SEQ ID NO: 28365 |

FIGURE 50

| | | | | |
|---|---|---|---|---|
| | | AA | DIQMTQSPSSLSASVGDRVTITCRASQSISNYLNWYQQKPGKAPKLLIFAASSLQSGVPSRFSGSGSGTDFTLTISGLQPEDFATYFCQQSFRTPFFTFGPGTKVDIK | EVQLLESGGGLVQPGGSLRLSCAASEFTFSSYAMNWVRQAPGKGLEWVSIISGRGVNTFYADSVKGRFTISRDNSKNTLYLQMNSLRAEDTAVYYCASRLAVAGSEAFDIWGQGTMVTVSS |
| | | | SEQ ID NO: 24360 | SEQ ID NO: 28366 |
| **iPS:392762** | **21-225_22G5** | NA | GACATCCAGATGACCCAGTCTCCATCCTCCCTGTCTGCATCTGTAGGAGACAGAGTCACCATCACTTGCCGGGCAAGTCAGAACATTAGCAGCTATTTAAATTGGTATCAGCAGAAACAAGGGAAAGCCCCTAAGCTCCTGATCTATGCTGCATCCAGTTTGCAAAATGGGGTCCCATCAAGGTTCAGTGGCAGAGGATCTGGGACAGATTTCACTCTCACCATCAGTAGTCTGCAACCTGAAGATTTTGCAACTTACTACTGTCAACAGAGTTACAGAACCCCCTTATTCACTTTCGGCCCTGGGACCAAGGTTGATTTCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAACTGGGTCCGCCAGGCTCCAGGGATGGGGCTGGAGTGGGTCTCAGTTATTAGTCGTAGTGGTGGTTACACATACTACGCGGACTCCGTGAAGGGCCGGTTCACCATCTCCAGAGACAATTCCAAGAACACGCTGTATCTGCAAATGAACAGCCTGAGAGCCGAGGACACGGCCGTCTATTACTGTGCGTCCCGTTTAGCAGTGGCTGGCTCGGAGGCTTTTGATATCTGGGGCCAAGGGACACTGGTCACCGTCTCTTCA |
| | | | SEQ ID NO: 24361 | SEQ ID NO: 28367 |
| | | AA | DIQMTQSPSSLSASVGDRVTITCRASQNISSYLNWYQQKQGKAPKLLIYAASSLQNGVPSRFSGRGSGTDFTLTISSLQPEDFATYYCQQSYRTPLFTFGPGTKVDFK | EVQLLESGGGLVQPGGSLRLSCAASGFTFSSYAMNWVRQAPGMGLEWVSVISRSGGYTYYADSVKGRFTISRDNSKNTLYLQMNSLRAEDTAVYYCASRLAVAGSEAFDIWGQGTLVTVSS |
| | | | SEQ ID NO: 24362 | SEQ ID NO: 28368 |

FIGURE 50

| | | | | |
|---|---|---|---|---|
| **iPS:392764** | **21-225_22G10** | NA | GACATCCAGATGACCCAGTCTCCATCCTCCCTGTCTGCATCTGTAGGAGACAGAGTCACCATCACTTGCCGGGCAAGTCAGAGCATTTTCAGCTATTTAAATTGGTATCAGCAGAAACCAGGGAAAGCCCCTAAACTCCTGATCTATGCTGCATCCAGTTTACAAAGTGGGGTCCCATCAAGGTTCAGTGGCAGTGGATCTGGGACAGATTTCACTCTCACCATCAGCAGTCTCCAACCTGAAGATTTTGCAACTTACTTCTGTCAACAGAGTTTCAGAACCCCCTTATTCACTTTCGGCCCTGGGACCAAGGTGGATTTCAAA | GAGGTGCAGCTGTTGGAGTCTGGGGGAGACTTGGTACAGCCTGGGGGGTCCCTGAGACTCTCCTGTACAGCCTCTGGATTCACCTTTAGCAGCTATGCCATGAACTGGGTCCGCCAGGCTCCAGGGAAGGGGCTGGAGTGGGTCTCAGTTATTAGTGGTAGTGGTGGTAACACATTCTACGCAGACTCCGTGAAGGGCCGATTCACCATCTCCAGAGACAATTCCAAGAACACGCTGTTTCTGCACATGAACAGCCTGAGAGCCGAGGACACGGCCGTTTATTACTGTGCGTCCCGTATGGCAGTGGCTGGCTCGGAGGCTTTTGATATCTGGGGCCAAGGGACAATGGTCACCGTCTCTTCA |
| | | | SEQ ID NO: 24363 | SEQ ID NO: 28369 |
| | | AA | DIQMTQSPSSLSASVGDRVTITCRASQSIFSYLNWYQQKPGKAPKLLIYAASSLQSGVPSRFSGSGSGTDFTLTISSLQPEDFATYFCQQSFRTPLFTFGPGTKVDFK | EVQLLESGGDLVQPGGSLRLSCTASGFTFSSYAMNWVRQAPGKGLEWVSVISGSGGNTFYADSVKGRFTISRDNSKNTLFLHMNSLRAEDTAVYYCASRMAVAGSEAFDIWGQGTMVTVSS |
| | | | SEQ ID NO: 24364 | SEQ ID NO: 28370 |
| **iPS:392766** | **21-225_23H4** | NA | GACATCCAGATGACCCAGTCTCCATCCTCCCTGTCTGCATCTGTAGGAGACAGAGTCACCATCACTTGCCGGGCAAGTCAGAGCATTAGCAGGTATTTAAATTGGTATCAGCAGAAACCAGGGAGAGCCCCTAAACTCCTGATCTGTTCTACATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTATTACTGTCAACAGAGTTACAGTACCCCCACGTGGACGTTCGGCCAAGGGACCAAGGTGGAAATCAGA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAACTGGGTCCGCCAGGCTCCAGGGAAGGGGCTGGAGTGGGTCTCAGTTATTAGTGGTAGTGGTGGTACCACATACTTCGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAAAGAAATAGCAGTGGCTGGCATGATGTTTTTGATATCTGGGGCCAAGGGACAAAGGTCACCGTCTCTTCA |
| | | | SEQ ID NO: 24365 | SEQ ID NO: 28371 |

FIGURE 50

| | | | | |
|---|---|---|---|---|
| | | AA | DIQMTQSPSSLSASVGDRVTITCRASQSISRYLNWYQQKPGRAPKLLICSTSSLQSGVPSRFSGSGSGTDFTLTISSLQPEDFATYYCQQSYSTPTWTFGQGTKVEIR | EVQLLESGGGLVQPGGSLRLSCAASGFTFSSYAMNWVRQAPGKGLEWVSVISGSGGTTYFADSVKGRFTISRDNSKNTLYLQMNSLRAEDTAVYYCAKRNSSGWHDVFDIWGQGTKVTVSS |
| | | | SEQ ID NO: 24366 | SEQ ID NO: 28372 |
| iPS:392768 | 21-225_20B8 | NA | GAAATAGTGATGACGCAGTCTCCATCCACCCTGTCTGTGTCTCCAGGGGAAAGAGTCACCCTCTCTTGCAGGGCCAGTCAGAGTGTTAGCAGCAACTTAGCCTGGTTTCAGCAGAAACCTGGCCAGGCTCCCAGGCTCCTCATCAATGGTGCATCCACCAGGGCCAGTGGTATCCCAGCCAGGTTCAGTGGCAGTGGGTCTGGGACAGAGTTCACTCTCACCATCAGCAGCCTGCAGTCTGAAGATTTTGCAGTTTATTACTGTCAGCAGTATAATAACTGTCCTCTGACGTTCGGCCAAGGGACCAAGGTGGAAATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGTTATAGCATGAACTGGGTCCGCCAGGCTCCAGGGAAGGGGCTGGAGTGGGTCTCATCCATCAGTGGCAGTGGTAGTCACATATACTACGCGGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGATCGGGGCAGTGGCTGGGGCCAGGGAACCCTGGTCACCGTCTCCTCA |
| | | | SEQ ID NO: 24367 | SEQ ID NO: 28373 |
| | | AA | EIVMTQSPSTLSVSPGERVTLSCRASQSVSSNLAWFQQKPGQAPRLLINGASTRASGIPARFSGSGSGTEFTLTISSLQSEDFAVYYCQQYNNCPLTFGQGTKVEIK | EVQLVESGGGLVKPGGSLRLSCAASGFTFSSYSMNWVRQAPGKGLEWVSSISGSGSHIYYADSVKGRFTISRDNAKNSLYLQMNSLRAEDTAVYYCARDRGSGWGQGTLVTVSS |
| | | | SEQ ID NO: 24368 | SEQ ID NO: 28374 |

FIGURE 50

| | | | | |
|---|---|---|---|---|
| **iPS:392770** | **21-225_20C10** | NA | GACATCCACATGACCCAGTCTCCATCCTCCCTGTCTGCATCTGTGGGAGACAAAGTCTCCATCACTTGCCGGGCAAGTCACCATATTAGCAACTATTTAAATTGGTATCAGCAGAAACCAGGGAAAGGCCCTAAGGTCCTGATCCTTACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACACTACCCCCACGTGGACGTTCGGCCAAGGGACCAAGGTGGAAATCAAA | GAGGTGCAGCTGTTGGAGTCTGGGGGAGGCTTGGTACAGCCTGGGGGGTCCCTGAGACTCTCCTGTGCAGCCTCTGGATTCACTTTTAGCAGCTATGCCATGAACTGGGTCCGCCAGGCTCCAGGGAAGGGGCTGGAGTGGGTCTCAGTTATTAGTGGTAGTGGTGGTACCACATACTACGCAGACTCCGTGAAGGGCCGGTTCACCATCTCCAGAGACAATTCCAAGAACACGCTGTATCTGCAAATGAACACCCTGAGAGCCGAGGACACGGCCGTATATTACTGTGCGAAAAGGTATACCAGTGACTGGCATGATGCTTTTGATATCTGGGGCCAAGGGACAATGGTCACCGTCTCTTCA |
| | | | SEQ ID NO: 24369 | SEQ ID NO: 28375 |
| | | AA | DIHMTQSPSSLSASVGDKVSITCRASHHISNYLNWYQQKPGKGPKVLILTASSLQSGVPSRFSGSGSGTDFTLTISSLQPEDFATYYCQQSYTTPWTFGQGTKVEIK | EVQLLESGGGLVQPGGSLRLSCAASGFTFSSYAMNWVRQAPGKGLEWVSVISGSGGTTYYADSVKGRFTISRDNSKNTLYLQMNTLRAEDTAVYYCAKRYTSDWHDAFDIWGQGTMVTVSS |
| | | | SEQ ID NO: 24370 | SEQ ID NO: 28376 |
| **iPS:392772** | **21-225_20E12** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTTTGCTGCATCCAGTTTACAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGTTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATGTGGTATGATGAAAGTAATAAACACTATGCAGACTCCGTGAAGGGCCGATTCACCATCTCCAGAGACAATTCCAGGAACACGCTGTATCTGCAAATGAACAGCCTGAGAGCCGAGGACACGGCTGTGTATTACTGTGCGAGAGAACTGGGGTTCCGGTTTGACTACTGGGGCCAGGGAACCCTGGTCACCGTCTCCTCA |
| | | | SEQ ID NO: 24371 | SEQ ID NO: 28377 |

FIGURE 50

| | | | | |
|---|---|---|---|---|
| | | AA | DIQMTQSPSSLSASVGDRVTITCRASQGIRNDLGWYQQKPGKAPKRLIFAASSLQSGVPSRFSGSGSGTEFTLTISSLQPEDFATYYCLQHNSYPFTFGGGTKVEIK | QVQLVESGGGVVQPGRSLRLSCAASGFTFSSYGMHWVRQAPGKGLEWVAVMWYDESNKHYADSVKGRFTISRDNSRNTLYLQMNSLRAEDTAVYYCARELGFRFDYWGQGTLVTVSS |
| | | | SEQ ID NO: 24372 | SEQ ID NO: 28378 |
| **iPS:392774** | **21-225_21F3** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGATTTCATTCTCACAATCAGCAGCCTGCAGCCTGAAGATTTTGCAACTTATTACTGTCTACAGCATAGTAGTTACCCCCTCACTTTCGGCGGAGGGACCAAGGTGGAGATCAAA | CAGCTGCAGCTGCAGGAGTCGGGCCCAGGACTGGTGAAGCCTGCGGAGACCCTGTCCCTCACCTGCACTGTCTCTGGTGGCTCCATCAGCAGAAGTAGTTACTACTGGGGCTGGATCCGCCAGCCCCCAGGGAAGGTGCTGGAGTGGATTGGGAGCATCTATTATAGTGGGAGCACCTACTACAACCCGTCCCTCAAGAGTCGAGTCACCATATCCGTCGACACGTCCAAGAACCAGTTCTCCCTGAAGCTGAGCTCTGTGACCGCCGCAGACACGGCTGAGTATTACTGTGCGAGCCTTAGCAGCAGCTGGGACTTCCAGCACTGGGGCCAGGGCACCCTGGTCACCGTCTCCTCA |
| | | | SEQ ID NO: 24373 | SEQ ID NO: 28379 |
| | | AA | DIQMTQSPSSLSASVGDRVTITCRASQGIRNDLGWYQQKPGKAPKRLIYAASSLQSGVPSRFSGSGSGTDFILTISSLQPEDFATYYCLQHSSYPLTFGGGTKVEIK | QLQLQESGPGLVKPAETLSLTCTVSGGSISRSSYYWGWIRQPPGKVLEWIGSIYYSGSTYYNPSLKSRVTISVDTSKNQFSLKLSSVTAADTAEYYCASLSSSWDFQHWGQGTLVTVSS |
| | | | SEQ ID NO: 24374 | SEQ ID NO: 28380 |

FIGURE 50

| | | | | |
|---|---|---|---|---|
| **iPS:392776** | **21-225_21A12** | NA | GACATCCAGATGACCCAGTCTCCATCCTCACTGTCTGCATCTGTGGGAGACAGAGTCACCATCACTTGTCGGGCGAGTCAAGGCATTAGCAAATATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATAATAGTTACCCGTTCAGGTTTGGCCAGGGGACCAAGCTGGAGATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAATAGCTATAGCATGAACTGGGTCCGCCAGGCTCCAGGGAAGGGGCTGGAGTGGGTCTCATCCATTAGTGGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTCTGTATTACTGTGCGAGAGCGGCTGGCTTTGACTACTGGGGCCAGGGAACCCTGGTCACCGTCTCCTCA |
| | | | SEQ ID NO: 24375 | SEQ ID NO: 28381 |
| | | AA | DIQMTQSPSSLSASVGDRVTITCRASQGISKYLAWFQQKPGKAPKSLIYAASSLQSGVPSKFSGSGSGTDFTLTISSLQPEDFATYYCQQYNSYPFRFGQGTKLEIK | EVQLVESGGGLVKPGGSLRLSCAASGFTFNSYSMNWVRQAPGKGLEWVSSISGSSSYIYYADSVKGRFTISRDNAKNSLYLQMNSLRAEDTALYYCARAAGFDYWGQGTLVTVSS |
| | | | SEQ ID NO: 24376 | SEQ ID NO: 28382 |
| **iPS:392778** | **21-225_22H3** | NA | GACATCCAGATGACCCAGTCTCCATCCTCCCTGTCTGCATCTGTAGGAGACAGAGTCACCATCACTTGCCGGGCAAGTCAGGACATTAGAAATAATTTAGGCTGGTATCAGCAGAAACCAGGGAAAGCCCCTAAGCGCCTGATCTATCCTGCATCCAGTTTGCAAACTGGGGTCCCATCAAGGTTCAGCGGCAGTGGATCTGGGACAGAATTCACTCTCACAATCAGCAGCCTGCAGCCTGAAGATTTTGCAACTTATTTCTGTCTACAGGATAATAGTTACCCATTCACTTTCGGCCCTGGGACCAAAGTGGATATCAAA | GCGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTAGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTTTTACTGTGCGAGAGATAGGGGCAGCCTCTGGGGCCAGGGAACCCTGGTCACCGTCTCCTCT |
| | | | SEQ ID NO: 24377 | SEQ ID NO: 28383 |

FIGURE 50

| | | | | |
|---|---|---|---|---|
| | | AA | DIQMTQSPSSLSASVGDRVTITCRASQDIRNNLGWYQQKPGKAPKRLIYPASSLQTGVPSRFSGSGSGTEFTLTISSLQPEDFATYFCLQDNSYPFTFGPGTKVDIK | AVQLVESGGGLVKPGGSLRLSCAASGFTFSSYSMNWVRQAPGKGLEWVSSISSSSSYIYYADSVKGRFTISRDNAKNSLYLQMNSLRAEDTAVFYCARDRGSLWGQGTLVTVSS |
| | | | SEQ ID NO: 24378 | SEQ ID NO: 28384 |
| iPS:392780 | 21-225_22B7 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAACGCCTGATCTATACTGCATCCAGTTTGCAAAGTGGGGTCCCATCAAGGTTCAGCGGCAGTGGTTCTGGGACAGAATTCACTCTCACAATCAGCAGCCTGCAGCCTGAAGATTTTGCAACTTATTACTGTCTACAGCATAATACTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATTTGGTATGAAGAAAATAATCAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAGTGGGGTTCCGGTCTGACTACTGGGGCCAGGGAACCCTGGTCACCGTCTCCTCA |
| | | | SEQ ID NO: 24379 | SEQ ID NO: 28385 |
| | | AA | DIQMTQSPSSLSASVGDRVTITCRASQGIRNDLGWYQQKPGKAPKRLIYTASSLQSGVPSRFSGSGSGTEFTLTISSLQPEDFATYYCLQHNTYPLTFGGGTKVEIK | QVQLVESGGGVVQPGRSLRLSCAASGFTFSSYGMHWVRQAPGKGLEWVAVIWYEENNQYYADSVKGRFTISRDNSKNTLYLQMNSLRAEDTAVYYCAREVGFRSDYWGQGTLVTVSS |
| | | | SEQ ID NO: 24380 | SEQ ID NO: 28386 |

FIGURE 50

| | | | | |
|---|---|---|---|---|
| **iPS:392782** | **21-225_22B12** | NA | GACATCCAGATGACCCAGTCTCCATCCTCACTGTCTGCGTCTGTAGGAGACAGAGTCACCATCATTTGTCGGGCGAGTCAGGACATTAGCAATTATTTAGCCTGGTTTCAGGAGAAACCAGGGAAAGCCCATAAGTCCCTGATCTATGGTGCATCCAGTTTGCGGAGTGGGGTCCCATCAAAGTTCAGCGGCAGTGGATCTGGGACAGATTTCAATCTCACCATCAGCAGCCTGCAGCCTGAAGATCTTGCAACTTATTACTGCCAACAGTATCATAGTTACCCATTCACTTTCGGCCCTGGGACCAAAGTGGATTTCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGCAGCTATAGCATGAACTGGGTCCGCCAGGCTCCAGGGAAGGGGCTGGAGTGGGTCTCATCCATTAGTGGTAGTAGTAGTTACACATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGTAGCAGCCCTTGACTCCTGGGGCCAGGGAACCCTGGTCACCGTCTCCTCA |
| | | | SEQ ID NO: 24381 | SEQ ID NO: 28387 |
| | | AA | DIQMTQSPSSLSASVGDRVTIICRASQDISNYLAWFQEKPGKAHKSLIYGASSLRSGVPSKFSGSGSGTDFNLTISSLQPEDLATYYCQQYHSYPFTFGPGTKVDFK | EVQLVESGGGLVKPGGSLRLSCAASGFTFSSYSMNWVRQAPGKGLEWVSSISGSSSYTYYADSVKGRFTISRDNAKNSLYLQMNSLRAEDTAVYYCARVAALDSWGQGTLVTVSS |
| | | | SEQ ID NO: 24382 | SEQ ID NO: 28388 |
| **iPS:392784** | **21-225_23C7** | NA | GACATCCAGATGACCCAGTCTCCATCCTCACTGTCTGCATCTGTAGGAGACAGAGTCACCATCACTTGCCGGGCGAGTCAGGGCATTGGCATTTATTTAGCCTGGTTTCAGCAGAAACCAGGGAAAGCCCCTAAGTCCCTGATCTCTGCTGCATCCAGTTTGCAAAGTGGGGTCCCATCAAAGTTCAGCGGCAGTGGATCTGGGACAGAGTTCACTCTCACCATCAGCAGCCTGCAGCCTGAAGATTTTGCAACTTATTACTGCCAACAGTATAATAGTTACCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGTTATGAGCTGGGTCCGCCAGGCTCCAGGGAAGGGGCTGGAGTGGGTCTCAGCTATGAGTGGTAGTGGTGGTAGCACATATTATGTAGACTCCGTGAAGGGCCGGTTCACCATCTCCAGAGACAATTCCAAGAACACGCTGTATCTGCAAATGAACAGCCTGAGAGCCGAGGACACGGCCGTATATTATTGTGCGCGAACTGGGGTCTTTGACTACTGGGGCCAGGGAACCCTGGTCATCGTCTCCTCA |
| | | | SEQ ID NO: 24383 | SEQ ID NO: 28389 |

FIGURE 50

| | | | | |
|---|---|---|---|---|
| | | AA | DIQMTQSPSSLSASVGDRVTITCRASQGIGIYLAWFQQKPGKAPKSLISAASSLQSGVPSKFSGSGSGTEFTLTISSLQPEDFATYYCQQYNSYPFTFGPGTKVDIK | EVQLLESGGGLVQPGGSLRLSCAASGFTFSSYVMSWVRQAPGKGLEWVSAMSGSGGSTYYVDSVKGRFTISRDNSKNTLYLQMNSLRAEDTAVYYCARTGVFDYWGQGTLVIVSS |
| | | | SEQ ID NO: 24384 | SEQ ID NO: 28390 |
| **iPS:392786** | **21-225_24E1** | NA | GACATCGTGATGACCCAGTCTCCAGACTCCCTGGCTGTGTCTCTGGGCGAGAGGGCCACCATCAACTGCAAGTCCAGCCAGAGTGTTTTATACACCTCCAACAATAACAACTACTTAACTTGGTACCAGCAGAAACCTGGACAGCGTCCTAACCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATTTTATAGTACTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAGGTTCCAGGGCAGAGTCACCATGACCAGGAACACCTCCATAAGCACAGCCTACATGGAGCTGAGCAGCCTGAGATCTGAGGACACGGCCGTGTATTACTGTGCGACAAGCAGTGGCTGGGAGGTCTTTGACTACTGGGGCCAGGGAACCCTGGTCACCGTCTCCTCA |
| | | | SEQ ID NO: 24385 | SEQ ID NO: 28391 |
| | | AA | DIVMTQSPDSLAVSLGERATINCKSSQSVLYTSNNNNYLTWYQQKPGQRPNLLIYWASTRESGVPDRFSGSGSGTDFTLTISSLQAEDVAVYYCQQFYSTPPTFGQGTKVEIK | QVQLVQSGAEVKKPGASVKVSCKASGYTFTNYDINWVRQATGQGLEWMGWMHPNSGNTGYAQRFQGRVTMTRNTSISTAYMELSSLRSEDTAVYYCATSSGWEVFDYWGQGTLVTVSS |
| | | | SEQ ID NO: 24386 | SEQ ID NO: 28392 |

FIGURE 50

| | | | | |
|---|---|---|---|---|
| **iPS:392788** | **21-225_20C8** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGAAGAAACCAGGGAAAGCCCCTAAGCGCCTGATCAATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAAGATAATAGTTACCCGTTCACTTTCGGCGGAGGGACCAAGGTGGAGATCACA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGCCTGGAGTGGGTCTCATCCATTAGTGGCAGTAGTAGTTACATATACTACGCAGACTCAGTGAAGGCCCGATTCACCATCTCCAGAGACAACGCCAAGAACTCACTGTATCTGCAAATGAACAGCCTGAGAGCCGAGGACACGGCTGTGTATTACTGTGCGAGAGACAGAGGCAGTCTCTGGGGCCAGGGAACCCTGGTCACCGTCTCCTCA |
| | | | SEQ ID NO: 24387 | SEQ ID NO: 28393 |
| | | AA | DIQMTQSPSSLSASVGDRVTITCRASQGIRNDLGWYQKKPGKAPKRLINAASSLQSGVPSRFSGSGSGTEFTLTISSLQPEDFATYYCLQDNSYPFTFGGGTKVEIT | EVQLVESGGGLVKPGGSLRLSCAASGFTFSSYSMNWVRQAPGKGLEWVSSISGSSSYIYYADSVKARFTISRDNAKNSLYLQMNSLRAEDTAVYYCARDRGSLWGQGTLVTVSS |
| | | | SEQ ID NO: 24388 | SEQ ID NO: 28394 |
| **iPS:392790** | **21-225_20D10** | NA | GACATCCAGATGACCCAGTCTCCATCCTCCCTGTCTGCATTTGTAGGAGACAGAGTCACCATCACTTGCCGGGCAAGTCAGGGCATTAGAAATGATTTAGGCTGGTATCAGCAGAAACCAGGGAAAGCCCGTAAGCGCCTGATCTATGTTGCATACAGTTTGCAAAGTGGGGTCCCATCAAGGTTCAGCGGCAGTGGATATGGGACAGAATTCACTCTCACAATCAGCAGCTTGCAGCCTGAAGATTTTGCAACTTATTACTGTATACAGCAAAATAGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTTTGATGGAAGTAATAAATACTATGTAGACTCCGTGAAGGGCCGATTCACCATCTCCAGAGACGATTCCAAGAACACTCTGTATCTGCAAATGAACAGCCTGAGAGCCGAGGACACGGCTGTGTATTACTGCGCGAGAGATCTTGGCTGGACGGAAGAGTACTGGGGCCAGGGAACCCTGGTCACCGTCTCCTCA |
| | | | SEQ ID NO: 24389 | SEQ ID NO: 28395 |

FIGURE 50

| | | | | |
|---|---|---|---|---|
| | | AA | DIQMTQSPSSLSAFVGDRVTITCRASQGIRNDLGWYQQKPGKARKRLIYVAYSLQSGVPSRFSGSGYGTEFTLTISSLQPEDFATYYCIQQNSYPWTFGQGTKVEIK | QVQLVESGGGVVQPGRSLRLSCAASGFTFSDYGMHWVRQAPGKGLEWVAVIWFDGSNKYYVDSVKGRFTISRDDSKNTLYLQMNSLRAEDTAVYYCARDLGWTEEYWGQGTLVTVSS |
| | | | SEQ ID NO: 24390 | SEQ ID NO: 28396 |
| **iPS:392792** | **21-225_20G12** | NA | GACATCCAGATGACCCAGTCTCCATCCTCCCTGTCTGCATCTGTAGGAGACAGAGTCACCATCACTTGCCGGGCGAGTCAGGGCATTAGCAATTATTTAGCCTGGTATCAGCAGAAACCAGGGAAAGTTCCTAAGGTCTTGATCTATACTGCATCCACTTTGCAATCAGGGGTCCCATCTCGGTTCAGTGGCAGTGGATCTGGGACAGATTTCACTCTCACCGTCAGCAGCCTGCAGCCTGAAGATGTTGCAACTTATTACTGTCAAAAGTATAACAGTGCCCCTCCGATCACCTTCGGCCAAGGGACACGACTGGAGATTAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGCGGTAGTAGTAGTTACATCTACTACGCAGACTCACTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGATCGTGGGAGCTACTGGGGCCAGGGAACCCTGGTCACCGTCTCCTCA |
| | | | SEQ ID NO: 24391 | SEQ ID NO: 28397 |
| | | AA | DIQMTQSPSSLSASVGDRVTITCRASQGISNYLAWYQQKPGKVPKVLIYTASTLQSGVPSRFSGSGSGTDFTLTVSSLQPEDVATYYCQKYNSAPPITFGQGTRLEIK | EVQLVESGGGLVKPGGSLRLSCAASGFTFSSYSMNWVRQAPGKGLEWVSSISGSSSYIYYADSLKGRFTISRDNAKNSLYLQMNSLRAEDTAVYYCARDRGSYWGQGTLVTVSS |
| | | | SEQ ID NO: 24392 | SEQ ID NO: 28398 |

FIGURE 50

| | | | | |
|---|---|---|---|---|
| **iPS:392794** | **21-225_21H3** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCGTCCAGTGTGCAAACTGGGGTCCCATCAAGGTTCAGCGGCAGTGGATCTGGGACAGAATTCACTCTCACAATCAGCAGCCTGCAGGCTGAAGATTTGGCAATTTATTACTGTCTACAGCATAATAGTTATCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGTAGGAGTAGTTACTACTGGGGCTGGATCCGCCAGCCCCCAGGGAAGGGGCTGGAGTGGATTGGGAATATTTATTATAGTGGGAGCACCTACGACAACCCGTCCCTCAAGAGTCGAGTCACCATATCCGTAGACACGTCCAAGAACCAGTTCTCCCTGAAGCTGAGCTCTGTGACCGCCGCGGACACGGCTGTTTATTACTGTGGGAGACATGGAAAAGACTGGGGCCTTGACTACTGGGGCCAGGGAACCCTGGTCACCGTCTCCTCA |
| | | | SEQ ID NO: 24393 | SEQ ID NO: 28399 |
| | | AA | DIQMTQSPSSLSASVGDRVTITCRASQGIRNDLGWYQQKPGKAPKRLIYAASSVQTGVPSRFSGSGSGTEFTLTISSLQAEDLAIYYCLQHNSYPLTFGGGTKVEIK | QLQLQESGPGLVKPSETLSLTCTVSGGSISRSSYYWGWIRQPPGKGLEWIGNIYYSGSTYDNPSLKSRVTISVDTSKNQFSLKLSSVTAADTAVYYCGRHGKDWGLDYWGQGTLVTVSS |
| | | | SEQ ID NO: 24394 | SEQ ID NO: 28400 |
| **iPS:392796** | **21-225_22A4** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTTCAGCATAATAGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATACACTGGGTCCGCCAGGCTCCAGGCAAGGGGCTGGAGTGGGTGGCAGTTATATGGTTTGATGGAAGTAATAAATACTATGCAGACTCCGTGAAGGGCCGAATCACCATCTCTAGAGACAATTCCAAGAACACGCTGTATCTGCAAATGAACAGCCTGAGAGCCGAGGACACGGCTGTGTATTACTGTGCGAGAGATCTTGGCTGGACGGAAGAGTACTGGGGCCAGGGAACCCTGGTCACCGTCTCCTCA |
| | | | SEQ ID NO: 24395 | SEQ ID NO: 28401 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPWTFGQGTKVEIK | QVQLVESGGGVVQPGRSLRLSCAASGFTFSDYGIHWVRQAPGKGLEWVAVIWFDGSNKYYADSVKGRITISRDNSKNTLYLQMNSLRAEDTAVYYCARDLGWTEEYWGQGTLVTVSS |
| | | | SEQ ID NO: 24396 | SEQ ID NO: 28402 |
| iPS:392798 | 21-225_22C7 | NA | GACATCCAGATGACCCAGTCTCCATCCTCCCTGTCTGCATCTGTAGGAGACAGAGTCACCATCACTTGCCGGGCAAGTCAGAACATTATCAGCTATTTAAATTGGTATCAGCAGAAACCAGAGAAAGCCCCTAAACTCCTGATCCATATTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGACTTACAGTACCCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGGGGCAGTTATATGGCATGATGGAAGTAATAAATACTATGCAGACTCCGTGAAGGGCCGATTCACCATCTCCAGAGACAATGCCAAGAACACGCTGTATCTGCAAATGAACAGCCTGAGAGCCGAGGACACGGCTGTGTATTACTGTGCGAGAGATCTGAGTATGGGCGGTATGGACGTCTGGGGCCAAGGGACCACGGTCACCGTCTCCTCA |
| | | | SEQ ID NO: 24397 | SEQ ID NO: 28403 |
| | | AA | DIQMTQSPSSLSASVGDRVTITCRASQNIISYLNWYQQKPEKAPKLLIHIASSLQSGVPSRFSGSGSGTDFTLTISSLQPEDFATYYCQQTYSTPLTFGGGTKVEIK | QVQLVESGGGVVQPGRSLRLSCAASGFTFSSYGMHWVRQAPGKGLEWGAVIWHDGSNKYYADSVKGRFTISRDNAKNTLYLQMNSLRAEDTAVYYCARDLSMGGMDVWGQGTTVTVSS |
| | | | SEQ ID NO: 24398 | SEQ ID NO: 28404 |

FIGURE 50

| | | | | |
|---|---|---|---|---|
| **iPS:392800** | **21-225_22D12** | NA | GACATCCAGATGACCCAGTCTCCATCCTCCCTGTCTGCATCTGTAGGAGACAGAGTCACCATCACTTGTCGGGCAAGTCAGGGCATTAGAAATGATTTAGGCTGGTATCAGCAGAAACCAGGGAAAGCCCCTAAGCGCCTGATCTATGCTGCATCCAGTTTGCAGAGTGGGGTCCCATCTAGGTTCAGCGGCAGTGGATCTGGGACAGATTTCACTCTCACAATCAGCAGCCTGCAGCCTGAAGATTTTGCAACTTATTACTGTCTACAGCATAGTACTTACCCTCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGCAGGAGTAGTTACTACTGGGGCTGGATCCGCCAGCCCCCAGGGAAGGGGCTGGAGTGGATTGGAAATATCTATTATAGTGGGACCACCTCCTACAACCCGTCCCTCAAGAGTCGAGTCACCATATCCGTTGACACGTCCAGGAACCAGTTCTCCCTGAAGCTGAGCTCTGTGACCGCCGCAGACACGGCTGTGTTTTACTGTGCGAGACTCAGCAGCAGCTGGTCCGTTGACTACTGGGGCCAGGGAACCCTGGTCACCGTCTCCTCA |
| | | | SEQ ID NO: 24399 | SEQ ID NO: 28405 |
| | | AA | DIQMTQSPSSLSASVGDRVTITCRASQGIRNDLGWYQQKPGKAPKRLIYAASSLQSGVPSRFSGSGSGTDFTLTISSLQPEDFATYYCLQHSTYPLTFGGGTKVEIK | QLQLQESGPGLVKPSETLSLTCTVSGGSISRSSYYWGWIRQPPGKGLEWIGNIYYSGTTSYNPSLKSRVTISVDTSRNQFSLKLSSVTAADTAVFYCARLSSSWSVDYWGQGTLVTVSS |
| | | | SEQ ID NO: 24400 | SEQ ID NO: 28406 |
| **iPS:392802** | **21-225_23E7** | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGATACCAGGGAAAGCCCCTAAGTCCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTTTTATAGTTACCCATTCACTTTCGGCCCTGGGACCAAAGTGGATATCAAT | GAGGTGCAGCTGTTGGAGTCTGGGGGAGGCTTGGTACAGCCTGGGGGGTCCCTGAGACTCTCCTGTACAGCCTCTGGATTCACCTTTAGCAGCTATGCCATGAACTGGGTCCGCCAGGCTCCAGGGAAGGGGCTGGAGTGGGTCTCAGCTATTAGTGGTAGTGGTGGTTTCACATACTACGCAGACTCCGTGAAGGGCCGGTTCACCATCTCCAGAGACAATTCCAGGAACACGCTGTATCTGCAAATGAACAGCCTGAGAGCCGAGGACACGGCCGTATATTACTGTGCGCGTACCAGTGGCTTTGACTACTGGGGCCAGGGAACCCTGGTCACCGTCTCCTCA |
| | | | SEQ ID NO: 24401 | SEQ ID NO: 28407 |

FIGURE 50

| | | | | |
|---|---|---|---|---|
| | | AA | DIQMTQSPSSLSASVGDRVTITCRASQGISNYLAWFQQIPGKAPKSLIYAASSLQSGVPSKFSGSGSGTDFTLTISSLQPEDFATYYCQQFYSYPFTFGPGTKVDIN | EVQLLESGGGLVQPGGSLRLSCTASGFTFSSYAMNWVRQAPGKGLEWVSAISGSGGFTYYADSVKGRFTISRDNSRNTLYLQMNSLRAEDTAVYYCARTSGFDYWGQGTLVTVSS |
| | | | SEQ ID NO: 24402 | SEQ ID NO: 28408 |
| iPS:392806 | 21-225_24H3 | NA | GAAATAGTGATGACGCAGTCTCCAGCCACCCTGTCTGTGTCTCCAGGGGAAAGAGCCACCCTCTCCTGCAGGGCCAGTCAGAGTGTTAGCAGCAACTTAGCCTGGTACCAGCAGAAACCTGGCCAGGCTCCCAGGCTCCTCATCTATTTTGCATCCATCAGGGCCACTGGTATCCCAGCCAGGTTCAGTGGCAGTGGGTCTGGGACAGAGTTCACTCTCACCATCAGCAGCCTGCAGTCTGAAGATTTTGCAGTTTATTACTGTCAGCAGTATAATAACTGGCCCATGTGCAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTGGAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGTAGCAGTGGCAGGCGGTATGGACGTCTGGGGCCAAGGGACCACGGTCACCGTCTCCTCA |
| | | | SEQ ID NO: 24403 | SEQ ID NO: 28409 |
| | | AA | EIVMTQSPATLSVSPGERATLSCRASQSVSSNLAWYQQKPGQAPRLLIYFASIRATGIPARFSGSGSGTEFTLTISSLQSEDFAVYYCQQYNNWPMCSFGQGTKLEIK | QVQLVESGGGVVQPGRSLRLSCGASGFTFSSYGMHWVRQAPGKGLEWVAVIWYDGSNKYYADSVKGRFTISRDNSKNTLYLQMNSLRAEDTAVYYCARVAVAGGMDVWGQGTTVTVSS |
| | | | SEQ ID NO: 24404 | SEQ ID NO: 28410 |

FIGURE 50

| | | | | |
|---|---|---|---|---|
| **iPS:392808** | **21-225_20F8** | NA | GACATCCAGATGACCCAGTCTCCATCCTCCCTGTCTGCATCTGTAGGAGACAGAGTCACCATCACTTGCCGGGCAAGTCAGAGCATTAGCAGGTATTTAAATTGGTATCAGCAGAAACCAGGGAAAGCCCCTGAGCTCCTGATCTATGCTGCTTCCAGTTTGCAAAGTGGGGTCCCATCAAGGTTCAGTGGCAGTGGATCTGGGACAGATTTCATTCTCACCATCAGCAGTCTACAACCTGAAGATTTTGCAACTTACTACTGTCAACAGAGTTACAATACCCCCACGTGGACGTTCGGCCAAGGGACCAAGGTGGAAATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGAAGCTATGCCATGAGCTGGATCCGCCAGGCTCCAGGGAGGGGGCTGGAGTGGTCTTCAGTTATTAGTGGTAGTGGTGGTAGCACATATTACGCAGACTCCGTGAAGGGCCGGTTCACCATCTCCAGAGACAATTCCAAGAACACGCTGTATCTGCAAATGAGCAGCCTGAGAGCCGAAGACACGGCCGTATATTACTGTGCGAAAAGGTATAACAGTGGCTGGCATGATGTTTTTGATATCTGGGGCCAAGGGACAATGGTCACCGTCTCTTCA |
| | | | SEQ ID NO: 24405 | SEQ ID NO: 28411 |
| | | AA | DIQMTQSPSSLSASVGDRVTITCRASQSISRYLNWYQQKPGKAPELLIYAASSLQSGVPSRFSGSGSGTDFILTISSLQPEDFATYYCQQSYNTPTWTFGQGTKVEIK | EVQLLESGGGLVQPGGSLRLSCAASGFTFRSYAMSWIRQAPGRGLEWSSVISGSGGSTYYADSVKGRFTISRDNSKNTLYLQMSSLRAEDTAVYYCAKRYNSGWHDVFDIWGQGTMVTVSS |
| | | | SEQ ID NO: 24406 | SEQ ID NO: 28412 |
| **iPS:392810** | **21-225_20H12** | NA | GACATCCAGATGACCCAGTCTCCATCCTCCCTGTCTGCTTCTGTAGGAGACAGAGTCACCATCACTTGCCGGGCAAGTCAGGGCATTAGAAATGATTTAGACTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGATGAAAATAATAAATACTATGTAGACTCCGTGAAGGGCCGATTCACCATCTCCAGAGACAACTCCAAGAACACGCTGTATCTGCAAATGAACAGCCTGAGAGCCGAGGACACGGCTGTGTATTACTGTGCGAGAGAGTTGGGGTTCCGGTCTGACTACTGGGGCCAGGGAACCCTGGTCACCGTCTCCTCA |
| | | | SEQ ID NO: 24407 | SEQ ID NO: 28413 |

FIGURE 50

| | | | | |
|---|---|---|---|---|
| | | AA | DIQMTQSPSSLSASVGDRVTITCRASQGIRNDLDWYQQKPGKAPKRLIYAASSLQSGVPSRFSGSGSGTEFTLTISSLQPEDFATYYCLQHNSYPLTFGGGTKVEIK | QVQLVESGGGVVQPGRSLRLSCAASGFTFSNYGMHWVRQAPGKGLEWVAVIWYDENNKYYVDSVKGRFTISRDNSKNTLYLQMNSLRAEDTAVYYCARELGFRSDYWGQGTLVTVSS |
| | | | SEQ ID NO: 24408 | SEQ ID NO: 28414 |
| iPS:392812 | 21-225_21F4 | NA | GACATCCAGATGACCCAGTCTCCATCCTCCCTGTCTGCATCTGTAGGAGACAGAGTCACCATCACTTGCCGGGCAAGTCAGAACATTGGTAGTTATTTAAATTGGTATCAGCAGAAACCAGGGAAAGCCCCTAAGCTCCTGATCTTTGCTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTTCTTCTGTCAACAGAGTTACAGAACCCCCTTTTTCACTTTCGGCCCTGGGACCAAAGTGGATTTCAAA | GAGGTGCAGCTGTTGGAGTCTGGGGGAGGCTTGGTACAGCCTGGGGGGTCCCTGAGACTCTCCTGTGCAGCCTCTGAATTCACCTTTAGCAGCTATGCCATGAACTGGGTCCGCCAGGCTCCAGGGAAGGGGCTGGAGTGGGTCTCAGTTATTAGTGGTCGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGTCCCGTTTGGCAGTGGCTGGCTCGGAGGCTTGTGATATCTGGGGCCAAGGGACAATGGTCACCGTCTCTTCA |
| | | | SEQ ID NO: 24409 | SEQ ID NO: 28415 |
| | | AA | DIQMTQSPSSLSASVGDRVTITCRASQNIGSYLNWYQQKPGKAPKLLIFAASSLQSGVPSRFSGSGSGTDFTLTISSLQPEDFATFFCQQSYRTPFFTFGPGTKVDFK | EVQLLESGGGLVQPGGSLRLSCAASEFTFSSYAMNWVRQAPGKGLEWVSVISGRGGNTFYADSVKGRFTISRDNSKNTLYLQMNSLRAEDTAVYYCASRLAVAGSEACDIWGQGTMVTVSS |
| | | | SEQ ID NO: 24410 | SEQ ID NO: 28416 |

FIGURE 50

| | | | | |
|---|---|---|---|---|
| **iPS:392814** | **21-225_22A1** | NA | GATATTGTGATGACCCAGACTCCACTCTCTCTGTCCGTCACCCCTGGACAGCCGGCCTCCATCTCCTGCAAGTCTAGTCAGAGCCTCCTGCATAGTGGTGGAAAGACCTATTTATATTGGTACCTGCAGAAGCCAGGCCAGCCTCCACAGCTCCTGATCTATGAAGTTTCCAACCGGTTCTCTGGACTGCCAGATAGGTTCAGTGGCAGCGGGTCAGGGACAGATTTCACACTGAAAATCAGCCGGGTGGAGGCTGCGGATGTTGGGGTTTATTACTGCATGCAAACTTTACACCTTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATGTGGTATGATGGAAGTAATAAATATTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATGGGGGATTTTTGGAGTGGTTAGACTACTGGGGCCAGGGAACCCTGGTCACCGTCTCCTCA |
| | | | SEQ ID NO: 24411 | SEQ ID NO: 28417 |
| | | AA | DIVMTQTPLSLSVTPGQPASISCKSSQSLLHSGGKTYLYWYLQKPGQPPQLLIYEVSNRFSGLPDRFSGSGSGTDFTLKISRVEAADVGVYYCMQTLHLPWTFGQGTKVEIK | QVQLVESGGGVVQPGRSLRLSCAASGFTFSSYGMHWVRQAPGKGLEWVAVMWYDGSNKYYADSVKGRFTISRDNSKNTLYLQMNSLRAEDTAVYYCARDGGFLEWLDYWGQGTLVTVSS |
| | | | SEQ ID NO: 24412 | SEQ ID NO: 28418 |
| **iPS:392816** | **21-225_22E4** | NA | GACATCCAGATGACCCAGTCTCCATCCTCCCTGTCTGCATCTGTAGGAGACAGAGTCACCATCACTTGCCGGGCAAGTCAGAACATTAGTAGTTATTTAAATTGGTATCAGCAGAAACCAGGGAAAGCCCCTAAGCTCCTGATCTATGCTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACAATACCCCCTTATTCACTTTCGGCCCTGGGACCAAAGTGGATATCAAA | GAGGTGCAGCTGTTGGAGTCTGGGGGAGGCTTGGTGCAGCCTGGGGGGTCCCTGAGACTCTCCTGTGCAGCCTCTGAATTCACCTTTAGCAGCTATGCCATGAGCTGGGTCCGCCAGGCTCCAGGGAAGGGGCTGGAGTGGGTCTCAATTATTAGTGGTCGTGGTACTAACACATTCTACGCAGACTCCGTGAAGGGCCGGTTCACCATCTCCAGAGTCAATTCCAAGAACACGCTGTATCTGCAAATGAACAGCCTGAGAGCCGAGGACACGGCCGTATATTACTGTGCGTCCCGTATAGCAGTGGCTGGCTCGGAGGCTTTTGATATCTGGGGCCAAGGGACAATGGTCACCGTCTCTTCA |
| | | | SEQ ID NO: 24413 | SEQ ID NO: 28419 |

FIGURE 50

| | | | | |
|---|---|---|---|---|
| | | AA | DIQMTQSPSSLSASVGDRVTITCRASQNISSYLNWYQQKPGKAPKLLIYAASSLQSGVPSRFSGSGSGTDFTLTISSLQPEDFATYYCQQSYNTPLFTFGPGTKVDIK | EVQLLESGGGLVQPGGSLRLSCAASEFTFSSYAMSWVRQAPGKGLEWVSIISGRGTNTFYADSVKGRFTISRVNSKNTLYLQMNSLRAEDTAVYYCASRIAVAGSEAFDIWGQGTMVTVSS |
| | | | SEQ ID NO: 24414 | SEQ ID NO: 28420 |
| iPS:392818 | 21-225_22D8 | NA | GACATCCAGATGACCCAGTCTCCATCCTCCCTGTCTGCATCTGTAGGAGACAGAGTCACCATCACTTGCCGGACAAGTCAGAACAGTAACAGTTATTTAAATTGGTATCAGCAGAAACCAGGGAAAGCCCCTAAGCTCCAGATCTTTGCTGCATACAGTTTGGAAAGTGGGGTCCCATCAAGGTTCAGTGGCAATAGATCTGGGACAGAGTTCACTCTCACCATCAGCAGTCTGCAACCTGAAGATTTTGCAACTTACTACTGTCAACAGACTTACGGTACCTCGATCACCTTCGGCCAAGGGACACGACTGGAGATTAAA | CAGGTGCAGCTGGTGGAGTCTGGGGGAGGCGTGGTCCAGCCTGGGAGGTCCCTGAGACTCTCCTGTGTAGCCTCTGGATTCACCTTCAGGAGCTATGGCATGCACTGGGTCCGCCAGGCTCCAGGCAAGGGGCTGGAGTGGGTGACAATTATATCATATGATGGAAGTAATAAATACTATGCAGACTCCGTGAAGGGCCGATTCACCATCTCCAGAGACAATTCCAAGAACACGCTGTATCTGCAAATGAACAGCCTGAGAGCTGAGGACACGGCTGTATATTTCTGTGCGAGAGGGGTTTGGTTCGGGGACTTCTGGGGCCAGGGAACCCTGGTCACCGTCTCCTCA |
| | | | SEQ ID NO: 24415 | SEQ ID NO: 28421 |
| | | AA | DIQMTQSPSSLSASVGDRVTITCRTSQNSNSYLNWYQQKPGKAPKLQIFAAYSLESGVPSRFSGNRSGTEFTLTISSLQPEDFATYYCQQTYGTSITFGQGTRLEIK | QVQLVESGGGVVQPGRSLRLSCVASGFTFRSYGMHWVRQAPGKGLEWVTIISYDGSNKYYADSVKGRFTISRDNSKNTLYLQMNSLRAEDTAVYFCARGVWFGDFWGQGTLVTVSS |
| | | | SEQ ID NO: 24416 | SEQ ID NO: 28422 |

FIGURE 50

| | | | | |
|---|---|---|---|---|
| **iPS:392820** | **21-225_23D1** | NA | GACATCCAGATGACCCAGTCTCCATCCTCCCTGTCTGCATCTGTAGGAGACAGAGTCACCATCACTTGCCGGGCAAGTCAGGGCATCAGAAATGATTTAGGCTGGTATCAGCAGAAACCAGGGAAAGCCCCTAAGCGCCTGATCTATGCTGCATCCAGTTTACAAAGTGGGGTCCCATCAAGGTTCAGCGGCAGTGGTTCTGGGACAGAATTCACTCTCACAGTCAGCAGCCTGCAGCCTGAAGATTTTGCAACTTATTACTGTCTACAGCATAGTAGTTACCCTCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGCAGAAGTAGTTACTACTGGGGCTGGATCCGCCAGCCCCCAGGGAAGGGACTGGAGTGGATTGGGAGTATCTATTATAGTGGGAGCGCCCAGTACAACCCGTCCCTCAAGAGTCGAGTCACCATATCCGTCGACACGTCCAAGAACCAGTTCTCCCTGACGCTGAGCTCTGTGACCGCCGCAGACACGGCTTTATATTACTGTGCGAGACTGAGCAGCAGCTGGTCTTTTGACTACTGGGGCCAGGGAACCCTGGTCACCGTCTCCTCA |
| | | | SEQ ID NO: 24417 | SEQ ID NO: 28423 |
| | | AA | DIQMTQSPSSLSASVGDRVTITCRASQGIRNDLGWYQQKPGKAPKRLIYAASSLQSGVPSRFSGSGSGTEFTLTVSSLQPEDFATYYCLQHSSYPLTFGGGTKVEIK | QLQLQESGPGLVKPSETLSLTCTVSGGSISRSSYYWGWIRQPPGKGLEWIGSIYYSGSAQYNPSLKSRVTISVDTSKNQFSLTLSSVTAADTALYYCARLSSSWSFDYWGQGTLVTVSS |
| | | | SEQ ID NO: 24418 | SEQ ID NO: 28424 |
| **iPS:392822** | **21-225_23C8** | NA | GACATCCAGATGACCCAGTCTCCATCCTCCCGGTCTGCATCTGTAGGGGACAGAGTCACCATCACTTGCCGGGCAAGTCAGGACATTAGAAATGATTTAGGCTGGTATCAGCAGAAACCAGGGAGAGCCCCTAAGCGCCTGATCAATGGTGCATCCAGTGTGCAAAGTGGGGTCCCATCAAGGTTCAGCGGCAGTGGATCTGGGACAGAATTCACTCTCACAATCAGCAGCCTGCAGCCTGAAGATTTTGTAATTTATTACTGTCTACAGCATAATAGTTACCCGCTCACTTTCGGCGGAGGGACCAAGGTGGAGATCAAA | CAGTTGCAACTGCAGGAGTCGGGCCCAGGACTGGTGAAGCCTTCGGAGACCCTGTCCCTCACCTGCACTGTCTCTGGTGGCTCCATCAGTAGGAGTAGTTACTACTGGGGCTGGATCCGCCAGCCCCCAGGGAAGGGGCTGGAGTGGATTGGGAATATTTATTATAGTGGGACCACTTACAACAACCCGTCCCTCAAGAGTCGAGTCACCATATCCGTAGACACGTCCAAGAACCACTTCTCCCTGAAGCTGAGCTCTGTGACCGCCGCAGACACGGCTGTGTATTACTGTGGGAGACATGGAAAAGACTGGGGCCTTGACTACTGGGGCCAGGGAACCCTGGTCACCGTCTCCTCA |
| | | | SEQ ID NO: 24419 | SEQ ID NO: 28425 |

FIGURE 50

| | | | | |
|---|---|---|---|---|
| | | AA | DIQMTQSPSSRSASVGDRVTITCRASQDIRNDLGWYQQKPGRAPKRLINGASSVQSGVPSRFSGSGSGTEFTLTISSLQPEDFVIYYCLQHNSYPLTFGGGTKVEIK | QLQLQESGPGLVKPSETLSLTCTVSGGSISRSSYYWGWIRQPPGKGLEWIGNIYYSGTTYNNPSLKSRVTISVDTSKNHFSLKLSSVTAADTAVYYCGRHGKDWGLDYWGQGTLVTVSS |
| | | | SEQ ID NO: 24420 | SEQ ID NO: 28426 |
| iPS:392824 | 21-225_24E5 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAGTAATTACCCGCTCACTTTCGGCGGAGGGACCAAGGTGGAGATCAAA | CAGCTGCAGCTGCAGGAGTCGGGCCCAGGACTGGTGAAGCCCTCGGAGACCCTGTCCCTCACCTGCACTGTCTCTGGTGGCGCCATCAGCAGGAGTAGTTACTACTGGGGCTGGATCCGCCAGCCCCCAGGGAAGGGGCTGGAGTGGATTGGGAGTATCTATTATAGTGGGAGCGCCAACTACAACCCGTCCCTCAAGAGTCGAGTCACCATATCCGTAGACACGTCCAAGAACCAGTTCTCCCTGAAGCTGAGCTCTGTGACCGCCGCAGACACGGCTGTGTATTACTGTGCGAGACTCAGCAGCAGCTGGTCCATTGACAACTGGGGCCAGGGAACCCTGGTCACCGTCTCCTCA |
| | | | SEQ ID NO: 24421 | SEQ ID NO: 28427 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHSNYPLTFGGGTKVEIK | QLQLQESGPGLVKPSETLSLTCTVSGGAISRSSYYWGWIRQPPGKGLEWIGSIYYSGSANYNPSLKSRVTISVDTSKNQFSLKLSSVTAADTAVYYCARLSSSWSIDNWGQGTLVTVSS |
| | | | SEQ ID NO: 24422 | SEQ ID NO: 28428 |

FIGURE 50

| | | | | |
|---|---|---|---|---|
| **iPS:392826** | **21-225_20B9** | NA | GACATCCAGATGACCCAGTCTCCATCCTCACT<br>GTCTGCATCTGTAGGAGACAGAGTCACCATCA<br>CTTGTCGGGCGAGTCAGGGCATTAGCAATTAT<br>TTAGCCTGGTTTCAGCAGAAACCAGGGAAAGC<br>CCCTAAGTCCCTGATCTATGTTGCATCCAGTTT<br>GCAAAGTGGGGTCCCATCAAAGTTCAGCGGCA<br>GTGGATCTGGGACAGATTTCACTCTCACCATC<br>AGCAGCCTGCAGCCTGAAGATTTTGCAACTTA<br>TTACTGCCAACAGTATAATACTTATCCATTCAC<br>TTTCGGCCCTGGGACCAAAGTGGATATCAAA | GAGGTGCAGCTGGTGGAGTCTGGGGGAGGCTTG<br>GTACAGCCTGGGGGGTCCCTGAGACTCTCCTGTG<br>CAGCCTCTGGATTCACCTTCAGTAGCTATAGCAT<br>GAACTGGGTCCGCCAGGCTCCAGGGAAGGGGCT<br>GGAGTGGGTTTCATACATTAGTAGTAGTAGTAGT<br>ACCATATACTATGCAGACTCTGTGAAGGGCCGAT<br>TCACCATCTCCAGAGACAATGCCAAGAACTCACT<br>GTACCTGCAAATGAACAGCCTGAGAGACGAGGA<br>CACGGCTGTGTATTACTGTGCGAGGTCACTATGG<br>TCCCCCTTTGACTACTGGGGCCAGGGAACCCTGG<br>TCACCGTCTCCTCA |
| | | | SEQ ID NO: 24423 | SEQ ID NO: 28429 |
| | | AA | DIQMTQSPSSLSASVGDRVTITCRASQGISNYLA<br>WFQQKPGKAPKSLIYVASSLQSGVPSKFSGSGSG<br>TDFTLTISSLQPEDFATYYCQQYNTYPFTFGPGT<br>KVDIK | EVQLVESGGGLVQPGGSLRLSCAASGFTFSSYSMN<br>WVRQAPGKGLEWVSYISSSSSTIYYADSVKGRFTIS<br>RDNAKNSLYLQMNSLRDEDTAVYYCARSLWSPFD<br>YWGQGTLVTVSS |
| | | | SEQ ID NO: 24424 | SEQ ID NO: 28430 |
| **iPS:392830** | **21-225_21A5** | NA | GACATCCAGATGACCCAGTCTCCATCCTCCCT<br>GTGTGCATCTGTAGGAGACAGAGTCACCATCA<br>CTTGCCGGGCAAGTCAGACCATTAGCAGCCAT<br>TTAAATTGGTATCAGCGGAAACCAGGGAAAG<br>CCCCTAAACTCCTGATCTATGCTGCATCCAGTT<br>TGCAAAGTGGGGTCCCATCACGGTTCAGTGGC<br>AGTGGATCTGGGACAGATTTCACTCTCACCAT<br>CAGCAGTGTGCAACCTGAAGATTTTGCAACTT<br>ATTACTGTCAACAGAGTTACAATATCTCATTC<br>ACTTTCGGCCCTGGGACCAAAGTGGATATCAA<br>A | CAGGTGCAGCTGCAGGAGTCGGGCCCAGGACTG<br>GTGAAGCCTTCGGAGACCCTGTCCCTCACCTGCA<br>CTGTCTCTGGTGGCTCCATCAGTAGTTACTTCTGG<br>AGCTGGATCCGGCAGCCCGCCGGGAAGGGACTG<br>GAGTGGATTGGGCGTATCTATACCAGTGGGATCA<br>CCAACTACAACCCCTCCCTCAAGAGTCGAGTCAC<br>CATGTCAGTAGACACGTCCAAGAACCAGTTCTCC<br>CTGAAACTGAGTTCTGTGACCGCCGCGGACACGG<br>CCATATATTACTGTGCGAGAGGCCCGACTTCGGG<br>GTGGTTCGACCCCTGGGGCCAGGGAACCCTGGTC<br>ACCGTCTCCTCA |
| | | | SEQ ID NO: 24425 | SEQ ID NO: 28431 |

FIGURE 50

| | | | | |
|---|---|---|---|---|
| | | AA | DIQMTQSPSSLCASVGDRVTITCRASQTISSHLNWYQRKPGKAPKLLIYAASSLQSGVPSRFSGSGSGTDFTLTISSVQPEDFATYYCQQSYNISFTFGPGTKVDIK | QVQLQESGPGLVKPSETLSLTCTVSGGSISSYFWSWIRQPAGKGLEWIGRIYTSGITNYNPSLKSRVTMSVDTSKNQFSLKLSSVTAADTAIYYCARGPTSGWFDPWGQGTLVTVSS |
| | | | SEQ ID NO: 24426 | SEQ ID NO: 28432 |
| **iPS:392832** | **21-225_21H8** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTACAAAGTGGGGTCCCATCAAGGTTCAGAGGCAGTGGATCTGGGACAGAATTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | CAGGTGCAGCTGGTGGAGTCTGGGGGAGGCGTGGTCCAGTCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTTTGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCTTGGCTGGACGGAAGAGTACTGGGGCCAGGGAACCCTGGTCACCGTCTCCTCA |
| | | | SEQ ID NO: 24427 | SEQ ID NO: 28433 |
| | | AA | DIQMTQSPSSLSASVGDRVTITCRASQGIRNDLGWYQQKPGKAPKRLIYAASSLQSGVPSRFRGSGSGTEFTLTISSLQPEDFATYYCLQHNSYPWTFGQGTKVEIK | QVQLVESGGGVVQSGRSLRLSCAASGFTFSDYGMHWVRQAPGKGLEWVAVIWFDGSNKYYADSVKGRFTISRDNSKNTLYLQMNSLRAEDTAVYYCARDLGWTEEYWGQGTLVTVSS |
| | | | SEQ ID NO: 24428 | SEQ ID NO: 28434 |

FIGURE 50

| | | | | |
|---|---|---|---|---|
| **iPS:392834** | **21-225_22C1** | NA | GACATCCAGATGACCCAGTCTCCATCCTCCCTGTCTGCATCTGTAGGAGACAGAGTCACCATCACTTGCCGGACAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAGTACTTACCCGCTCACTTTCGGCGGAGGGACCAAGGTGGAGATCAAA | CAACTGCAGCTGCAGGAGTCGGGCCCAGGACTGGTGAAGCCTTCGGAGACCCTGTCCCTCACCTGTACTGTTTCTGGTGGCTCCATCAACAGGAGTAGTTACTACTGGGGCTGGATCCGCCAGCCCCCAGGGAAGGGGCTGGAGTGGATTGGGAATATCTATTATAGTGGGGCCACCTATTATAATTCGTCCCTCAAGAGTCGAGTCACCATCTCCGTAGACACGTCCACGAACCAGTTCTCCCTGAAGCTGAGCTCTGTGACCGCCGCAGACACGGCTGTGTATTACTGTGCGAGACATAGCGGCAGCTGGTCCCTTGACTACTGGGGCCAGGGAACCCTGGTCACCGTCTCCTCA |
| | | | SEQ ID NO: 24429 | SEQ ID NO: 28435 |
| | | AA | DIQMTQSPSSLSASVGDRVTITCRTSQGIRNDLGWYQQKPGKAPKRLIYAASSLQSGVPSRFSGSGSGTEFTLTISSLQPEDFATYYCLQHSTYPLTFGGGTKVEIK | QLQLQESGPGLVKPSETLSLTCTVSGGSINRSSYYWGWIRQPPGKGLEWIGNIYYSGATYYNSSLKSRVTISVDTSTNQFSLKLSSVTAADTAVYYCARHSGSWSLDYWGQGTLVTVSS |
| | | | SEQ ID NO: 24430 | SEQ ID NO: 28436 |
| **iPS:392836** | **21-225_22F4** | NA | GACATCCAGATGACCCAGTCTCCATCCTCCCTGTCTGCTTCTGTTGGAGACAGAGTCACCATCACTTGCCGGGCAAGTCAGGTCATTAGAGATGATTTAGGCTGGTATCAGCAGAAACCAGGGAAAGCCCCTAAGCGCCTGATCTATGCTGCATCCAGTTTGCAAAGTGGGGTCCCATCGAGGTTCAGCGGCAGTAGATCTGGGACAGACTTCACTCTCACAATCAGCAGCCTGCAGCCTGAAGATTTTGCAACTTATTACTGTCTACACCACTATAGTTATCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGCGACTATGGCATGCACTGGGTCCGCCAGGCTCCAGGCAAGGGGCTGGAGTGGGTGGCAGTTATATGGTATGATGGAAGTAATAAATACTATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAAGTATAGCAGCAGCTGGTACGACTACGGTATGGACGTCTGGGGCCAAGGGACCACGGTCACCGTCTCCTCA |
| | | | SEQ ID NO: 24431 | SEQ ID NO: 28437 |

FIGURE 50

| | | | | |
|---|---|---|---|---|
| | | AA | DIQMTQSPSSLSASVGDRVTITCRASQVIRDDLGWYQQKPGKAPKRLIYAASSLQSGVPSRFSGSRSGTDFTLTISSLQPEDFATYYCLHHYSYPRTFGQGTKVEIK | QVQLVESGGGVVQPGRSLRLSCAASGFTFSDYGMHWVRQAPGKGLEWVAVIWYDGSNKYYVDSVKGRFTISRDNSKNTLYLQMNSLRAEDTAVYYCAREKYSSSWYDYGMDVWGQGTTVTVSS |
| | | | SEQ ID NO: 24432 | SEQ ID NO: 28438 |
| **iPS:392838** | **21-225_22G8** | NA | GACATCCAGATGATCCAGTCTCCATCCTCCCTGTTCGCATCTGTAGGAGACAGAGTCACCATCACTTGCCGGACAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAAATTCAGCGGCAGTGGATCTGGGACAGAATTCACTCTCTCAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAACAGGAGTAGTTACTACTGGGGCTGGATCCGCCAGCCCCCAGGGAAGGGGCTGGACTGGATTGGGAATATCTATTATAGTGGGAGCACCTACTACAACCCGTCCGTCAAGAGTCGATTCACCATATCCGTAGACACGTCCAAGAACCAGTTCTCCCTGAAGCTGAGCTCTGTGACCGCCGCAGACACGGCTGTGTATTACTGTGCGAGACATGGAAAAGACTGGGGCCTTGACTACTGGGGCCAGGGAACCCTGGTCACCGTCTCCTCA |
| | | | SEQ ID NO: 24433 | SEQ ID NO: 28439 |
| | | AA | DIQMIQSPSSLFASVGDRVTITCRTSQGIRNDLGWYQQKPGKAPKRLIYAASSLQSGVPSKFSGSGSGTEFTLSISSLQPEDFATYYCLQHNSYPLTFGGGTKVEIK | QLQLQESGPGLVKPSETLSLTCTVSGGSINRSSYYWGWIRQPPGKGLDWIGNIYYSGSTYYNPSVKSRFTISVDTSKNQFSLKLSSVTAADTAVYYCARHGKDWGLDYWGQGTLVTVSS |
| | | | SEQ ID NO: 24434 | SEQ ID NO: 28440 |

FIGURE 50

| | | | | |
|---|---|---|---|---|
| iPS:392840 | 21-225_23G1 | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAACCAGGGAAAGCCCCTAAGTCCCTGATCTCTGCTGCATCCAGTTTACAGAGTGGGGTCCCATCACAGTTCAGCGGCAGTGGATTTGGGACAGATTTCACTCTCACCATCAGCAGCCTGCAGCCTGAGGATTTTGCAACTTATTACTGCCAACAGTATAATAGTTACCCATTCACTTTCGGCCCTGGGACCAAAGTGGATATCAAA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAGGGGGCTGGAGTGGGTCTCAGTTATTAGTGGTAGTGGTGGTACCACATATAACACAGACTCCGTGAAGGGCCGGTTCACCATCTCCAGAGACAATTCCAAGAACACGCTATATCTGCAAATGAACAGCCTGAGAGCCGAGGACACGGCCGTATATTACTGTGCCCGCAGCTCCTTGTTTGACTACTGGGGCCAGGGAACCCTGGTCACCGTCTCCTCA |
| | | | SEQ ID NO: 24435 | SEQ ID NO: 28441 |
| | | AA | DIQMTQSPSSLSASVGDRVTITCRASQGISNYLAWFQQKPGKAPKSLISAASSLQSGVPSQFSGSGFGTDFTLTISSLQPEDFATYYCQQYNSYPFTFGPGTKVDIK | EVQLLESGGGLVQPGGSLRLSCAASGFTFSSYAMSWVRQAPGRGLEWVSVISGSGGTTYNTDSVKGRFTISRDNSKNTLYLQMNSLRAEDTAVYYCARSSLFDYWGQGTLVTVSS |
| | | | SEQ ID NO: 24436 | SEQ ID NO: 28442 |
| iPS:392842 | 21-225_23G8 | NA | GACATCCAGATGACCCAGTCTCCATCCTCACTGTCTGCATCTGTAGGAGACAGAGTCACCATCACTTGTCGGGCGAGTCAGGGCATTAGAAATTATTTAGCCTGGTTTCAGCAGAAACCAGGGAAAGCCCCTAAGTCCCTGATCTATGCTGCATCCAGTTTGCAAAGTGGGGTCCCATCAAACTTCAGCGGCAGTGGATCTGGGACAGATTTCACTCTCACCATCAGTAGCCTGCAGCCTGAAGATTTTGCAACTTATTACTGCCAACAGTATAATAGTTACCCTTTCACTATCGGCCCTGGGACCAAGGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTAGTGGTGGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGGTTAGCAGTGGCTGGTTCGCCTGGGGCCAGGGAACCCTGGTCACCGTCTCCTCA |
| | | | SEQ ID NO: 24437 | SEQ ID NO: 28443 |

FIGURE 50

| | | | | |
|---|---|---|---|---|
| | | AA | DIQMTQSPSSLSASVGDRVTITCRASQGIRNYLAWFQQKPGKAPKSLIYAASSLQSGVPSNFSGSGSGTDFTLTISSLQPEDFATYYCQQYNSYPFTIGPGTKVDIK | EVQLLESGGGLVQPGGSLRLSCAASGFTFSSYAMSWVRQAPGKGLEWVSAISGSGGSTYYADSVKGRFTISRDNSKNTLYLQMNSLRAEDTAVYYCAVSSGWFAWGQGTLVTVSS |
| | | | SEQ ID NO: 24438 | SEQ ID NO: 28444 |
| iPS:392844 | 21-225_23E11 | NA | GACATCCAGATGACCCAGTCTCCATCCTCCCTGTCTGCATCTGTAGGAGACAGAATCACCATCACTTGCCGGGCAAGTCAGGGCATTAGAAATGATTTAGGCTGGTATCAGCAGAAACCAGGGAAAGCCCCTAAGCGCCTGATCTATCCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | GAGGTGCAACTGGTGGAGTCTGGGGGAGGCCTGGTCAAGCCTGGGGGGTCCCTGAGACTCTCCTGTGCAGCCTCTGGATTCACCTTCAGTTCCTATACCATGAACTGGGTCCGCCAGGCTCCAGGGAAGGGGCTGGAGTGGGTCTCATCCATTAGTGGTAGTAGTAGTTACATATGGTATGTAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGATCGGGGCAGTCTCTGGGGCCAGGGAACCCTGGTCACCGTCTCCTCA |
| | | | SEQ ID NO: 24439 | SEQ ID NO: 28445 |
| | | AA | DIQMTQSPSSLSASVGDRITITCRASQGIRNDLGWYQQKPGKAPKRLIYPASSLQSGVPSRFSGSGSGTEFTLTISSLQPEDFATYYCLQHNSYPWTFGQGTKVEIK | EVQLVESGGGLVKPGGSLRLSCAASGFTFSSYTMNWVRQAPGKGLEWVSSISGSSSYIWYVDSVKGRFTISRDNAKNSLYLQMNSLRAEDTAVYYCARDRGSLWGQGTLVTVSS |
| | | | SEQ ID NO: 24440 | SEQ ID NO: 28446 |

FIGURE 50

| | | | | |
|---|---|---|---|---|
| iPS:392846 | 21-225_24B6 | NA | GACATCCAGATGACCCAGTCTCCATCCTCCCTGTCTGCATCTGTCGGAGACAGAGTCACCATCACTTGCCGGGCAAGTCAGGACATTAGAAATGATTTAGGCTGGTATCAGCAGAAGCCAGGGAAAGCCCCTAAGCGCCTGATCTATGCTGCATCCAGTTTGCACAGTGGGGTCCCATCAAGGTTCAGCGGCAGTGGATCTGGGACAGAATTCACTCTCACAATCAGCAGCCTGCAGACTGAAGATTTTGCAACTTATTACTGTCTACAGCATTATAGTTACCCTCGGACGTTCGGCCAAGGGACCAAGGTGGAAGTCAAG | CAGGTGCAGCTGGTGGAGTCTGGGGGAGGCGTGGTCCAGCCTGGGAGGTCCCCGAGACTCTCCTGTGCAGCGTCTGGATTCATCTTCAGTAACTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGGAATATAGTAGTGGCTGGTACGACTACGGTATGGACGTCTGGGGCCAAGGGACCACGGTCACCGTCTCCTCA |
| | | | SEQ ID NO: 24441 | SEQ ID NO: 28447 |
| | | AA | DIQMTQSPSSLSASVGDRVTITCRASQDIRNDLGWYQQKPGKAPKRLIYAASSLHSGVPSRFSGSGSGTEFTLTISSLQTEDFATYYCLQHYSYPRTFGQGTKVEVK | QVQLVESGGGVVQPGRSPRLSCAASGFIFSNYVMHWVRQAPGKGLEWVAVIWYDGSNKYYADSVKGRFTISRDNSKNTLYLQMNSLRAEDTAVYYCAREEYSSGWYDYGMDVWGQGTTVTVSS |
| | | | SEQ ID NO: 24442 | SEQ ID NO: 28448 |
| iPS:392848 | 21-225_20F9 | NA | GACATCCAGATGACCCAGTCTCCATCCTCACTGTCTGCATCTGTAGGAGACAGAATCACCATCACTTGTCGGGCGAGTCAGGGCATTAGCAATTATTTAGCCTGGTTTCAGCAGAAACCAGGGAAAGCCCCTAAGTCCCTGATCTCTGCTGCATCCAGTTTGCAAAGTGGGGTCCCTTCAAAGTTCAGCGGCAGTGGATCCGGGACAGATTTCACTCTCACCATCAGCAGCCTGCAGCCTGAAGATTTTGCAACTTATTGCTGCCAACAGTATCATAGTTACCCGTGGACGTTCGGCCAAGGGACCAAGGTGGAAATCAAA | GAAGTGCAGCTGGTGGAATCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTAGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTATTGTGCGAGAGATCGTGGGAGCTGCTGGGGCCAGGGAACCCTGGTCACCATCTCCTCA |
| | | | SEQ ID NO: 24443 | SEQ ID NO: 28449 |

FIGURE 50

| | | | | |
|---|---|---|---|---|
| | | AA | DIQMTQSPSSLSASVGDRITITCRASQGISNYLAWFQQKPGKAPKSLISAASSLQSGVPSKFSGSGSGTDFTLTISSLQPEDFATYCCQQYHSYPWTFGQGTKVEIK | EVQLVESGGGLVKPGGSLRLSCAASGFTFSSYSMNWVRQAPGKGLEWVSSISSSSSYIYYADSVKGRFTISRDNAKNSLYLQMNSLRAEDTAVYYCARDRGSCWGQGTLVTISS |
| | | | SEQ ID NO: 24444 | SEQ ID NO: 28450 |
| iPS:392850 | 21-225_20H10 | NA | GGCATCCAGATGACCCAGTCTCCATCCTCCCTGTCTGCATCTGTAGGAGACAGAGTCACCATCACTTGCCGGGCAAGTCAGGGCATTAAAAATAATTTAGGCTGGTATCAGCAGAAACCAGGGAAAGGCCCTAAGTGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTACCTATAGCATGAACTGGGTCCGCCAGGCTCCAGGGAAGGGGCTGGAGTGGGTCTCATCCATTAGTAGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTATTTATTACTGTGCGAGAGATCGTGGGAGCCTCTGGGGCCAGGGAACCCTGGTCACCGTCTCCTCA |
| | | | SEQ ID NO: 24445 | SEQ ID NO: 28451 |
| | | AA | GIQMTQSPSSLSASVGDRVTITCRASQGIKNNLGWYQQKPGKGPKCLIYAASSLQSGVPSRFSGSGSGTEFTLTISSLQPEDFATYYCLQHNSYPLTFGGGTKVEIK | EVQLVESGGGLVKPGGSLRLSCAASGFTFSTYSMNWVRQAPGKGLEWVSSISSSSSYIYYADSVKGRFTISRDNAKNSLYLQMNSLRAEDTAIYYCARDRGSLWGQGTLVTVSS |
| | | | SEQ ID NO: 24446 | SEQ ID NO: 28452 |

FIGURE 50

| | | | | |
|---|---|---|---|---|
| **iPS:392852** | **21-225_21A2** | NA | GACATCCAGATGACCCAGTCTCCATCCTCCCTGTCTGCATCTGTAGGAGACAGAGTCACCATCACTTGCCGGGCAAGTCAGAGTATTAGTAGTTATTTAAATTGGTATCAGCAGAAACCAGGGAAAGCCCCTAAGCTCCTGATCTATGCTGCATCCAGTTTGCAAAGTGGGGTCCCATCAAGGTTCAGTGGCAGTGGATCTGGGACAGACTTCACTCTCACCATCAGTAGTCTGCAACCTGAAGATTTTGCAACTTACTTCTGTCAACAGAGTTACAGAACCCCCTTTTTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTAAATTCACCTTTAGCAGCTATGCCATGAACTGGGTCCGCCAGGCTCCAGGGAAGGGGCTGGAGTGGATCTCAATTATTAGTGGTCGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGTCCCGTTTGGCAGTGGCTGGCTCGGAGGCTTTTGATATCTGGGGCCAAGGGACAATGGTCACCGTCTCTTCA |
| | | | SEQ ID NO: 24447 | SEQ ID NO: 28453 |
| | | AA | DIQMTQSPSSLSASVGDRVTITCRASQSISSYLNWYQQKPGKAPKLLIYAASSLQSGVPSRFSGSGSGTDFTLTISSLQPEDFATYFCQQSYRTPFFTFGPGTKVDIK | EVQLLESGGGLVQPGGSLRLSCAASKFTFSSYAMNWVRQAPGKGLEWISIISGRGGNTFYADSVKGRFTISRDNSKNTLYLQMNSLRAEDTAVYYCASRLAVAGSEAFDIWGQGTMVTVSS |
| | | | SEQ ID NO: 24448 | SEQ ID NO: 28454 |
| **iPS:392854** | **21-225_21E5** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTACATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCCCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTTCAGCGTCTGGATTCACCTTCAGTGACTATGGCATGCACTGGGTCCGCCAGGCTCCAGGCAAGGGGCTGGAGTGGGTGGCAGTTATATGGTATGATGAAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTATGTATTACTGTACGAGAGAACTGGGGTTCCGGTCTGACTATTGGGGCCAGGGAACCCTGGTCACCGTCTCCTCA |
| | | | SEQ ID NO: 24449 | SEQ ID NO: 28455 |

FIGURE 50

| | | | | |
|---|---|---|---|---|
| | | AA | DIQMTQSPSSLSASVGDRVTITCRASQGIRNDLGWYQQKPGKAPKRLIYATSSLQSGVPSRFSGSGSGTEFTLTISSLQPEDFATYYCLQHNSYPLTFGGGTKVEIK | QVQLVESGGGVVQPGRSLRLSCSASGFTFSDYGMHWVRQAPGKGLEWVAVIWYDESNKYYADSVKGRFTISRDNSKNTLYLQMNSLRAEDTAMYYCTRELGFRSDYWGQGTLVTVSS |
| | | | SEQ ID NO: 24450 | SEQ ID NO: 28456 |
| iPS:392856 | 21-225_22A2 | NA | GACATCCAGATGACCCAGTCTCCATCCTCACTGTCTGCATCTGTCGGAGACAGAGTCACCATCACTTGTCGGGCGAGTCAGGACATTAGCAATTATTTAGCCTGGGTTCAGCAGAAACCAGGGAAAGCCCCTAAGTCCCTGATCTACGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAAGTTATTACTGCCAACAGTATAATAGTTTCCCGCTCACTTTCGGCGGAGGGACCAAGGTGGAGATCAAA | GAGGTGCAGCTGTTGGAGTCTGGGGGAGGCTTGGTCCAACCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGGTATTAGTGGTAGTGGAGGTAACACACCCTACGCAGACTCCGTGAAGGGCCGGTTCACCATCTCCAGAGACATTTCCAAGAACACGCTGTATCTGCAAATGAACAGCCTGAGAGCCGAAGACACGGCCGTATATTACTGTGCGAAAGTAGTGGGAGCTGTCCACTGGGGCCGGGGAACCCTGGTCACCGTCTCCTCA |
| | | | SEQ ID NO: 24451 | SEQ ID NO: 28457 |
| | | AA | DIQMTQSPSSLSASVGDRVTITCRASQDISNYLAWVQQKPGKAPKSLIYAASSLQSGVPSKFSGSGSGTDFTLTISSLQPEDFASYYCQQYNSFPLTFGGGTKVEIK | EVQLLESGGGLVQPGGSLRLSCAASGFTFSSYAMSWVRQAPGKGLEWVSGISGSGGNTPYADSVKGRFTISRDISKNTLYLQMNSLRAEDTAVYYCAKVVGAVHWGRGTLVTVSS |
| | | | SEQ ID NO: 24452 | SEQ ID NO: 28458 |

FIGURE 50

| | | | | |
|---|---|---|---|---|
| **iPS:392858** | **21-225_22H4** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTGTGCAAAGTGGGGTCCCATCAAGGTTCAGCGGCAGTGGATCTGGGACAGAATTCACTCTCACAATCAGCAGCCTGCAGCCTGAAGATTTTGCACTTTATTACTGTCTACAGCATAATAGTTACCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGTAGGAGTAGTTACTACTGGGGCTGGATCCGCCAGCCCCCAGGGAAGGGGCTGGAGTGGATTGGGAATATTTATTATAGTGGGAGCACCTACCACAACCCGTCTCTCAAGAGTCGAGTCACCATATCCGTAGACACGTCCATGAACCAGTTCTCCCTGAAGTTGACCTCTGTGACCGCCGCAGACACGGCTGTGTATTTCTGTGGGAGACATGGAAAAGACTGGGGCCTTGACTACTGGGGCCAGGGAACCCTGGTCACCGTCTCCTCA |
| | | | SEQ ID NO: 24453 | SEQ ID NO: 28459 |
| | | AA | DIQMTQSPSSLSASVGDRVTITCRASQGIRNDLGWYQQKPGKAPKRLIYAASSVQSGVPSRFSGSGSGTEFTLTISSLQPEDFALYYCLQHNSYPLTFGGGTKVEIK | QLQLQESGPGLVKPSETLSLTCTVSGGSISRSSYYWGWIRQPPGKGLEWIGNIYYSGSTYHNPSLKSRVTISVDTSMNQFSLKLTSVTAADTAVYFCGRHGKDWGLDYWGQGTLVTVSS |
| | | | SEQ ID NO: 24454 | SEQ ID NO: 28460 |
| **iPS:392860** | **21-225_22H8** | NA | GATATTGTGATGACCCAGACTCCACTCTCTCTGTCCGTCACCCCTGGACAGCCGGCCTCCATCTCCTGCAAGTCTAGTCAGAGCCTCCTGCATAGTGATGGAAAGACCTTTTTGTATTGGTACCTGCAGAAGCCAGGCCATCCTCCACAGCTCCTGATCTATGAAGTTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGAATTTATTACTGCATGCAAAGTATACAGCTTCCGCTCTCATTCGGCGGAGGGACCAAGGTGGAGATCAAC | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATCTGGTATGATGGAAATAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGCTTGCCTGGTACGAAGACTACTGGGGCCAGGGAACCCTGGTCACCGTCTCCTCA |
| | | | SEQ ID NO: 24455 | SEQ ID NO: 28461 |

FIGURE 50

| | | | | |
|---|---|---|---|---|
| | | AA | DIVMTQTPLSLSVTPGQPASISCKSSQSLLHSDGKTFLYWYLQKPGHPPQLLIYEVSNRFSGVPDRFSGSGSGTDFTLKISRVEAEDVGIYYCMQSIQLPLSFGGGTKVEIN | QVQLVESGGGVVQPGRSLRLSCAASGFTFSSYGMHWVRQAPGKGLEWVAVIWYDGNNKYYADSVKGRFTISRDNSKNTLYLQMNSLRAEDTAVYYCARELAWYEDYWGQGTLVTVSS |
| | | | SEQ ID NO: 24456 | SEQ ID NO: 28462 |
| **iPS:392864** | **21-225_23B9** | NA | GAAATTGTGTTGACGCAGTCTCCAGGCACCCTGTCTTTGTCTCCAGGAGAAAGAGCCACCCTCTCCTGCAGGGCCAGTCAGAATGTTTACAGCAGCTACTTAGCCTGGTACCAGCAGAAACCTGGCCAGACTCCCAGGCTCCTCATCTATGGTGCATCCAGCAGGGCCAGTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGGTAGCTCACCTCGGACGTTCGGCCAAGGGACCAAGGTGGAAATCAAA | CAGGTGCAGCTGCAGGCGTCGGGCCCAGGACTGGTGAAGCCTTCACAGACCCTGTCCCTCACCTGCACTGTCTCTGATGGCTCCATCAGCAGTGGTGGTTACTACTGGAGCTGGATCCGCCAGCACCCAGGGAAGGGCCTGGAGTGGATTGGGTACATCTATTACAGTGGGAGCACCTACTACAACCCGTCCCTCAAGAGTCGAGTTACCATATCAGTAGACACGTCTAAGAACCAGTTCTCCCTGAAGCTGAGTTCTGTGACTGCCGCGGACACGGCCGTATATTACTGTGCGAGAGAGGACGGTGCCTTCGGCTACTACGGTATGGACGTCTGGGGCCAAGGGACCACGGTCACCGTCTCCTCA |
| | | | SEQ ID NO: 24457 | SEQ ID NO: 28463 |
| | | AA | EIVLTQSPGTLSLSPGERATLSCRASQNVYSSYLAWYQQKPGQTPRLLIYGASSRASGIPDRFSGSGSGTDFTLTISRLEPEDFAVYYCQQYGSSPRTFGQGTKVEIK | QVQLQASGPGLVKPSQTLSLTCTVSDGSISSGGYYWSWIRQHPGKGLEWIGYIYYSGSTYYNPSLKSRVTISVDTSKNQFSLKLSSVTAADTAVYYCAREDGAFGYYGMDVWGQGTTVTVSS |
| | | | SEQ ID NO: 24458 | SEQ ID NO: 28464 |

FIGURE 50

| | | | | |
|---|---|---|---|---|
| iPS:392866 | 21-225_23H11 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGACTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATCGTTACCCGCTCACTTTCGGCGGAGGGACCAAGGTGGAGATCAAA | CAGGAGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGAAAATAATAAATACTATGTAGACTCCGTGAAGGGCCGATTCACCATCTCCAGAGACAACTCCAAGAACACGCTGTATCTGCAAATGAACAGCCTGAGAGCCGAGGACACGGCTGTGTATTACTGTGCGAGAGAATTGGGGTTCCGGTCTGACTACTGGGGCCAGGGAACCCTGGTCACCGTCTCCTCA |
| | | | SEQ ID NO: 24459 | SEQ ID NO: 28465 |
| | | AA | DIQMTQSPSSLSASVGDRVTITCRASQGIRNDLDWYQQKPGKAPKRLIYAASSLQSGVPSRFSGSGSGTEFTLTISSLQPEDFATYYCLQHNRYPLTFGGGTKVEIK | QEQLVESGGGVVQPGRSLRLSCAASGFTFSSYGMHWVRQAPGKGLEWVAVIWYDENNKYYVDSVKGRFTISRDNSKNTLYLQMNSLRAEDTAVYYCARELGFRSDYWGQGTLVTVSS |
| | | | SEQ ID NO: 24460 | SEQ ID NO: 28466 |
| iPS:392868 | 21-225_24D6 | NA | GACATCCAGATGACCCAGTCTCCATCCTCCCTGTCTGCATCTGTAGGAGACAGAGTCACCATCACTTGCCAGGCGAGTCAGGACATTAACAACTATTTAAATTGGTATCAGCAGAAACCAGGGAAAGCCCTTAAGCTCTTGATATACGATGCATCCGATTTGGAAACAGGGGTCCCATCAAGGTTCAGTGGAAGTGGATCTGGGACAGATTTTACTTTCACCATCAGCAGTCTGCAGCCTGAAGATATTGCAATATATTACTGTCAACAGTATGAAAATCTCCCGATCACCTTCGGCCAAGGGACACGACTGGAGATTAAA | CAGGTGCAACTGGTAGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGTTCCAGGCAAGGGGCTGGAGTGGGTGGCAATTATATCATATGCTGGAAGTAATAAATCCTATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTACTGTGTGAGACGGGGATACAGCTATGGCGGGTACGGTATGGACGTCTGGGGCCAAGGGGCCACGGTCACCGTCTCCTCA |
| | | | SEQ ID NO: 24461 | SEQ ID NO: 28467 |

FIGURE 50

| | | | | |
|---|---|---|---|---|
| | | AA | DIQMTQSPSSLSASVGDRVTITCQASQDINNYLNWYQQKPGKALKLLIYDASDLETGVPSRFSGSGSGTDFTFTISSLQPEDIAIYYCQQYENLPITFGQGTRLEIK | QVQLVESGGGVVQPGRSLRLSCAASGFTFSSYGMHWVRQVPGKGLEWVAIISYAGSNKSYADSVKGRFTISRDNSKNTLYLQMNSLRAEDTAVYYCVRRGYSYGGYGMDVWGQGATVTVSS |
| | | | SEQ ID NO: 24462 | SEQ ID NO: 28468 |
| iPS:392870 | 21-225_20G9 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAGTACTTACCCTCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGCAGGAGTAGTTACTACTGGGGCTGGATCCGCCAGCCCCCAGGGAAGGGACTGGAGTGGATTGGGAGTATCTATTATAGTGGGAGCGCCTCTTACAACCCGTCCCTCAAGAGTCGAGTCACCATATCCGTAGACACGTCCAGGAACCAGTTCTCCCTGAAGCTGAGCTCTGTGACCGCCGCAGACACGGCTGCGTATTACTGTGCGAGACTGAGCAGCAGCTGGTCTTTTGACTACTGGGGCCAGGGAACCCTGGTCACCGTCTCCTCA |
| | | | SEQ ID NO: 24463 | SEQ ID NO: 28469 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHSTYPLTFGGGTKVEIK | QLQLQESGPGLVKPSETLSLTCTVSGGSISRSSYYWGWIRQPPGKGLEWIGSIYYSGSASYNPSLKSRVTISVDTSRNQFSLKLSSVTAADTAAYYCARLSSSWSFDYWGQGTLVTVSS |
| | | | SEQ ID NO: 24464 | SEQ ID NO: 28470 |

FIGURE 50

| | | | | |
|---|---|---|---|---|
| **iPS:392872** | **21-225_20B11** | NA | GACATCCAGATGACCCAGTCTCCATCCTCCCTGTCTGCATCTGTAGGAGACAGAGTCACCATCACTTGCCGGGCAAGTCAGGGCATTGGAAATGATTTAGGCTGGTATCAGCAGAAACCAGAGAAAGCCCCTAAGCGCCTGATCTATGCTGCATCCAGTTTGCATAGTGGGGTCCCATCCAGGTTCAGCGGCAGTGGCTCTGGGACAGAATTCACTCTCACAATCAGCAGCCTGCAGCCTGAAGATTTTGCAACTTATTACTGTCTACAGCATTATAGTTACCCTCGGACGTTTGGCCAAGGGACCAAGGTGGAAATCAAA<br>SEQ ID NO: 24465 | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGTCCGATTCACCGTCTCCAGAGACAATTCCAAGAACACGCTGTCTCTGCAAATGAACAGCCTGAGAGCCGAGGACACGGCTGTGTATTACTGTGCGAGAGAGAGATATACCAGTGGCTGGTATGACTACGGTATGGACGTCTGGGGCCAAGGGACCACGGTCACCGTCTCCTCA<br>SEQ ID NO: 28471 |
| | | AA | DIQMTQSPSSLSASVGDRVTITCRASQGIGNDLGWYQQKPEKAPKRLIYAASSLHSGVPSRFSGSGSGTEFTLTISSLQPEDFATYYCLQHYSYPRTFGQGTKVEIK<br>SEQ ID NO: 24466 | QVQLVESGGGVVQPGRSLRLSCAASGFTFSSYVMHWVRQAPGKGLEWVAVIWYDGSNKYYADSVKVRFTVSRDNSKNTLSLQMNSLRAEDTAVYYCARERYTSGWYDYGMDVWGQGTTVTVSS<br>SEQ ID NO: 28472 |
| **iPS:392874** | **21-225_21D2** | NA | GACATCCAGATGACCCAGTCTCCATCCTCCCTGTTTGCATCTGTAGGAGACAGAGTCACCATCACTTGCCGGGCAAGTCAGAGCATTAGCGACTATTTAAATTGGTATCAGCAGAAACCAGGGAGAGCCCCTAAACTCCTGATCTATGATACATCCAGTTTGCAAAGTGGGGTCCCATCAAGGTTCAGTGGCAGTGGATCTGGGACAGATTTCACTCTCACCATCAACAGTCTGCAACCTGAAGATTTTGCAACTTACTACTGTCAACAGACTTACAATATTCTTCCGGAGCGCAGTTTTGGCCGGGGGACCAAGCTGGAGATCAAA<br>SEQ ID NO: 24467 | GAGGTGAAGCTGTTGGAGTCTGGGGGAGGCTTGGTACAGCCGGGGGGGTCCCTGAGACTCTCCTGTGCAGCCTCTGGATTCACCTTTAACAACTATGCCATGAGCTGGGTCCGCCAGGCTCCAGGGAAGGGGCTGGAGTGGGTCTCAGTTCTTAGTGGTAGTGGTGGTAGCACATTCTACGCAGACTCCGTGAAGGGCCGGTTCACCATCTCCAGAGACAATTCCAAGAACACGCTGTATCTGCAAATGAGCAGCCTGAGAGCCGGGGACACGGCCGTATATTTCTGTGCCCGATATTGTAGTAGTGCCAGGTGCCCTTATGATGCCTTTGATATCTGGGGCCAAGGGACAATGGTCACCGTCTCCTCA<br>SEQ ID NO: 28473 |

FIGURE 50

| | | | | |
|---|---|---|---|---|
| | | AA | DIQMTQSPSSLFASVGDRVTITCRASQSISDYLNWYQQKPGRAPKLLIYDTSSLQSGVPSRFSGSGSGTDFTLTINSLQPEDFATYYCQQTYNILPERSFGRGTKLEIK | EVKLLESGGGLVQPGGSLRLSCAASGFTFNNYAMSWVRQAPGKGLEWVSVLSGSGGSTFYADSVKGRFTISRDNSKNTLYLQMSSLRAGDTAVYFCARYCSSARCPYDAFDIWGQGTMVTVSS |
| | | | SEQ ID NO: 24468 | SEQ ID NO: 28474 |
| iPS:392876 | 21-225_21F7 | NA | GACATCCAGATGACCCAGTCTCCATCCTCCCTGTCTGCATCTGTAGGAGACAGAGTCACCATCACTTGCCGGGCAAGTCAGGACATTAGAAATGATTTAGGCTGGTATCAGCAGAAACCAGGGAAAGCCCCTAAGCGCCTGATCTATGCTGCATCCAATTTTCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAATTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTTTGATGGAAATAATAAATACTATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCTTGGCTGGACGGAAGAGTACTGGGGCCAGGGAACCCTGGTCACCGTCTCCTCA |
| | | | SEQ ID NO: 24469 | SEQ ID NO: 28475 |
| | | AA | DIQMTQSPSSLSASVGDRVTITCRASQDIRNDLGWYQQKPGKAPKRLIYAASNFQSGVPSRFSGSGSGTEFTLTISSLQPEDFATYYCLQHNNYPWTFGQGTKVEIK | QVQLVESGGGVVQPGRSLRLSCAASGFTFSDYGMHWVRQAPGKGLEWVAVIWFDGNNKYYVDSVKGRFTISRDNSKNTLYLQMNSLRAEDTAVYYCARDLGWTEEYWGQGTLVTVSS |
| | | | SEQ ID NO: 24470 | SEQ ID NO: 28476 |

FIGURE 50

| | | | | |
|---|---|---|---|---|
| **iPS:392878** | **21-225_22C5** | NA | GACATCCAGATGACCCAGTCTCCAGCCTCCCTGTCTGCGTCTGTTGGAGACAGAGTCACCATCACTTGCCGGGCAAGTCAGAACATTAGCAGCTATTTAAATTGGTATCAGCAGAAACCAGGGAAGGCCCCTAAACTCCTGATCTATGCTGCATCCGTTTTGCAACATGGGATCCCATCAAGGTTCAGTGGCAGGGGATCTGGGACAGATTTCACTCTCATCATCAGTAGTCTGCAACCTGAAGATTTTGCAACTTACTACTGTCAACAGAGTTACAGAACCCCCTTATTCACTTTCGGCCCTGGGACCAAAGTAGATTTCAAA | GAGGTGCAGCTGTTGGAGTCTGGGGGAGGCTTGGTACAGGTTGGGGGGTCCCTGAGACTCTCCTGTGCAGCCTCTGGATTCACTTTTAGCAGCTATGCCATGAGCTGGGTCCGCCAGGCTCCAGGGATGGGGCTGGAGTGGGTCTCAATTATTAGTGGTAGTGGTGGTTACACATACTACGCGGACTCCGTGAAGGGCCGATTCACCATCTCCAGAGACAATTCCAAGAACACGCTGTATCTGCAAATGAACAGCCTGAGAGCCGAGGACACGGCCGTATATTATTGTGCGTCCCGTATAGCAGTGGCTGGCTCGGAGGCTTTTGATATCTGGGGCCAAGGGACAATGGTCACCGTCTCTTCA |
| | | | SEQ ID NO: 24471 | SEQ ID NO: 28477 |
| | | AA | DIQMTQSPASLSASVGDRVTITCRASQNISSYLNWYQQKPGKAPKLLIYAASVLQHGIPSRFSGRGSGTDFTLIISSLQPEDFATYYCQQSYRTPLFTFGPGTKVDFK | EVQLLESGGGLVQVGGSLRLSCAASGFTFSSYAMSWVRQAPGMGLEWVSIISGSGGYTYYADSVKGRFTISRDNSKNTLYLQMNSLRAEDTAVYYCASRIAVAGSEAFDIWGQGTMVTVSS |
| | | | SEQ ID NO: 24472 | SEQ ID NO: 28478 |
| **iPS:392880** | **21-225_22F9** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTACAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGATGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGAGGAAAATAATAAAGACTATGTAGACTCCGTGAAGGGCCGATTCACCATCTCCAGAGACAATTCCAAGAGCACGCTGTATCTGCAAATGAATAGTCTGAGAGCCGAGGACACGGCTGTGTATTACTGTGTGAGAGAGTTAGGCTTCCAGTCTGACTACTGGGGCCAGGGAACCCCGGTCACCGTCTCCTCA |
| | | | SEQ ID NO: 24473 | SEQ ID NO: 28479 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVEIK | QVQMVESGGGVVQPGRSLRLSCAASGFTFSSYGMHWVRQAPGKGLEWVAVIWYEENNKDYVDSVKGRFTISRDNSKSTLYLQMNSLRAEDTAVYYCVRELGFQSDYWGQGTPVTVSS |
| | | | SEQ ID NO: 24474 | SEQ ID NO: 28480 |
| **iPS:392882** | **21-225_23A3** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTGTGCAAAGTGGGGTCCCATCAAGGTTCAGCGGCAGTGGATCTGGGACAGAATTCACTCTCACAATCAGCAGCCTGCAGCCTGAAGATTTTGCAATTTATTTCTGTCTACAGCATAATAGTTACCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGTAGGAGTAGTTACTACTGGGGCTGGATCCGCCAGCCCCCAGGGCAGGGGCTGGAGTGGATTGGGAATATTTATTATAGTGGGAGCACCTACAACAACCCGTCCCTCAAGAGTCGAGTCTCCATATCCGTTGACACGTCCAAGAACCAGTTCTCCCTGAACCTGAGCTCTGTGACCGCCGCAGACACGGCTGTGTATTACTGTGGGAGACATGGAAAAGACTGGGGCCTTGACTTCTGGGGCCAGGGAACCCTGGTCACCGTCTCCTCA |
| | | | SEQ ID NO: 24475 | SEQ ID NO: 28481 |
| | | AA | DIQMTQSPSSLSASVGDRVTITCRASQGIRNDLGWYQQKPGKAPKRLIYAASSVQSGVPSRFSGSGSGTEFTLTISSLQPEDFAIYFCLQHNSYPLTFGGGTKVEIK | QLQLQESGPGLVKPSETLSLTCTVSGGSISRSSYYWGWIRQPPGQGLEWIGNIYYSGSTYNNPSLKSRVSISVDTSKNQFSLNLSSVTAADTAVYYCGRHGKDWGLDFWGQGTLVTVSS |
| | | | SEQ ID NO: 24476 | SEQ ID NO: 28482 |

FIGURE 50

| | | | | |
|---|---|---|---|---|
| iPS:392884 | 21-225_23A10 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCGGCAGCCTGCAGCCTGAAGATTTTGCAACTTATTACTGTCTGCAACATTATAGTTACCCTCGGACGTTCGGCCTAGGGACCAAGGTGGAAATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAGGTATAGCAGTGGCTGGCACGACTACGGTATGGACGTCTGGGGCCAAGGGACCACGGTCACCGTCTCCTCA |
| | | | SEQ ID NO: 24477 | SEQ ID NO: 28483 |
| | | AA | DIQMTQSPSSLSASVGDRVTITCRASQGIRNDLGWYQQKPGKAPKRLIYAASSLQSGVPSRFSGSGSGTEFTLTIGSLQPEDFATYYCLQHYSYPRTFGLGTKVEIK | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARERYSSGWHDYGMDVWGQGTTVTVSS |
| | | | SEQ ID NO: 24478 | SEQ ID NO: 28484 |
| iPS:392886 | 21-225_23A12 | NA | GACATCGTGATGACCCAGTCTCCAGACTCCCTGGCTGTGTCTCTGGGCGAGAGGGCCACCATCAACTGCAAGTCCAGCCAGAGTGTTTTATACAGCTCCAACAATAACAATTATTTAGCTTGGTACCAGCAGAAACCAGGACAGCCTCCTAAGCTGCTCATTTACTGGACATCTACCCGGGAATCCGGGGTCCCTGACCGATTCAGTGGCAGCGGGTCTGGGACAGATTTCACTCTCACCATCAGCAGCCTGCAGGCTGAAGATGTGGCCGTTTATTACTGTCAGCAGTATTATGATACTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACCCCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAACACAGCCTACATGGAACTGAGCAGCCTGAGATCTGAGGACACGGCCGTGTATTACTGTGCGGGTAGCAGTGGCTGGTACTACTTTGACTACTGGGGCCAGGGAACCCTGGTCACCGTCTCCTCA |
| | | | SEQ ID NO: 24479 | SEQ ID NO: 28485 |

FIGURE 50

| | | | | |
|---|---|---|---|---|
| | | AA | DIVMTQSPDSLAVSLGERATINCKSSQSVLYSSNNNNYLAWYQQKPGQPPKLLIYWTSTRESGVPDRFSGSGSGTDFTLTISSLQAEDVAVYYCQQYYDTPPTFGQGTKVEIK | QVQLVQSGAEVKKPGASVKVSCKASGYPFTNYDINWVRQATGQGLEWMGWMHPNSGNTGYAQKFQGRVTMTRNTSINTAYMELSSLRSEDTAVYYCAGSSGWYYFDYWGQGTLVTVSS |
| | | | SEQ ID NO: 24480 | SEQ ID NO: 28486 |
| iPS:392888 | 21-225_25A2 | NA | GATATTGTGATGAACCAGACTCCACTCTCTCTGTCCGTCACCCCTGGACAGCCGGCCTCCATCTCCTGCAAGTCTAGTCAGAGCCTCCTACATAGTGAAGGAAAGACCTATTTGTATTGGTATCTGCAGAAGCCAGGCCAGCCTCCACAACTCCTGATCTATGAAATTTCCAACCGGTTCTCTGGAGTGCCAGCTAGGTTAAGTGGCAGCGGGTCAGGGACAGATTTCACACTGAAAATCAGCCGGGTGGAGGCTGAGGATGTTGGGGTTTATTACTGCATGCAAAGTACACAGTTTCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAAGGTATAGCAGCAGCTGGTCAGGGGGTATGGACGTCTGGGGCCAAGGGACCACGGTCACCGTCTCCTCA |
| | | | SEQ ID NO: 24481 | SEQ ID NO: 28487 |
| | | AA | DIVMNQTPLSLSVTPGQPASISCKSSQSLLHSEGKTYLYWYLQKPGQPPQLLIYEISNRFSGVPARLSGSGSGTDFTLKISRVEAEDVGVYYCMQSTQFPLTFGGGTKVEIK | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARRYSSSWSGGMDVWGQGTTVTVSS |
| | | | SEQ ID NO: 24482 | SEQ ID NO: 28488 |

FIGURE 50

| | | | | |
|---|---|---|---|---|
| **iPS:392890** | **21-225_20H9** | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAACCAGGGAAAGCCCCTAAGTCCCTGATCTCTGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATAATAGTTACCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCCGGATTCACCTTTAGCAGCTATGCCATGAGCTGGGTCCGCCAGGCTCCAGGGAAGGGGCTGGAGTGGGTCTCAGCTATTAGTGGTAGTGGTGGTTACACATACTACGCAGACTCCGTGAAGGGCCGGTTCACCATCTCCAGAGACAATTCCGAGAACACGCTGTATCTGCAAATGAGCAGCCTGAGAGCCGAGGACACGGCCGTTTATTACTGTGCGAAAGGGGGGTCCCTCTTCTACTGGGGCCAGGGAACCCTGGTCACCGTCTCCTCA |
| | | | SEQ ID NO: 24483 | SEQ ID NO: 28489 |
| | | AA | DIQMTQSPSSLSASVGDRVTITCRASQGISNYLAWFQQKPGKAPKSLISAASSLQSGVPSKFSGSGSGTDFTLTISSLQPEDFATYYCQQYNSYPFTFGPGTKVDIK | EVQLLESGGGLVQPGGSLRLSCAASGFTFSSYAMSWVRQAPGKGLEWVSAISGSGGYTYYADSVKGRFTISRDNSENTLYLQMSSLRAEDTAVYYCAKGGSLFYWGQGTLVTVSS |
| | | | SEQ ID NO: 24484 | SEQ ID NO: 28490 |
| **iPS:392892** | **21-225_20C11** | NA | GACATCCAGATGACCCAGTCTCCATCCTCACTGTCTGCATCTGTTGGAGACAGAGTCACCATCACTTGTCGGGCGAGTCAGGACATTAGCAATTATTTAGCCTGGTTTCAGCAGAAACCAGGGAAAGCCCCTAAGTCCCTGATCTATGCTGCATCCAGTTTGCAAAGTGGGGTCCCATCAAAGTTCAGAGGCAGTGGATCTGGGACAGATTTCACTCTCACCATCAGCAGCCTGCAGCCTGAAGATTTTGCAACTTATTACTGCCAACAGTATCATAGTTTCCCATTCACTTTCGGCCCTGGGACCAAAGTGGATGTCAAA | GAGGTGCAGCTGTTGGAGTCGGGGGGAGGCTTGGTACAGCCTGGGGGGTCCCTGAGACTCTCCTGTGCAGCCTCTGGATTCTCCTTTAGTAGCTATGCCATGAGCTGGGTCCGCCAGGCTCCAGGGAAGGGGCTGGAGTGGGTCTCAACTATTAGTGGTCGTGGTGGTCACACATACTACGCAGACTCCGTGAAGGGCCGGTTCGCCATCTCCAGAGACAGTTCCAAGAACACGCTGTATCTGCAAATGAACAGCCTGAGAGCCGAGGACACGGCCGTATATTACTGTGCGAAACAGGACTGCTGGGGCCAGGGAACCCTGGTCACCGTCTCCTCA |
| | | | SEQ ID NO: 24485 | SEQ ID NO: 28491 |

FIGURE 50

| | | | | |
|---|---|---|---|---|
| | | AA | DIQMTQSPSSLSASVGDRVTITCRASQDISNYLAWFQQKPGKAPKSLIYAASSLQSGVPSKFRGSGSGTDFTLTISSLQPEDFATYYCQQYHSFPFTFGPGTKVDVK | EVQLLESGGGLVQPGGSLRLSCAASGFSFSSYAMSWVRQAPGKGLEWVSTISGRGGHTYYADSVKGRFAISRDSSKNTLYLQMNSLRAEDTAVYYCAKQDCWGQGTLVTVSS |
| | | | SEQ ID NO: 24486 | SEQ ID NO: 28492 |
| iPS:392894 | 21-225_21G2 | NA | GACATCCAGATGACCCAGTCTCCATCCTCCCTGTCTGCGTCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATACTTCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAACATAATAGTTACCCGTGGACGTTCGGCCTAGGGACCAAGGTGGTCATCAAA | CAGGTGCAACTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGATGGCAGTTATATGGTATGATGTAACTAATAAATACTATGCAGACTCCGTGAAGGGCCGATTCACCATCTCCAGAGACAATTCCAAGAACACGCTGTATCTGGAAATGAACAGCCTGAGAGCCGAGGACACGGCTGTTTATTACTGTGCGAGAGAGCTTGGCTGGTACGAGGACTACTGGGGCCAGGGAACCCTGGTCACCGTCTCCTCA |
| | | | SEQ ID NO: 24487 | SEQ ID NO: 28493 |
| | | AA | DIQMTQSPSSLSASVGDRVTITCRASQGIRNDLGWYQQKPGKAPKRLIYTSSSLQSGVPSRFSGSGSGTEFTLTISSLQPEDFATYYCLQHNSYPWTFGLGTKVVIK | QVQLVESGGGVVQPGRSLRLSCAASGFTFSDYGMHWVRQAPGKGLEWMAVIWYDVTNKYYADSVKGRFTISRDNSKNTLYLEMNSLRAEDTAVYYCARELGWYEDYWGQGTLVTVSS |
| | | | SEQ ID NO: 24488 | SEQ ID NO: 28494 |

FIGURE 50

| | | | | |
|---|---|---|---|---|
| **iPS:392896** | **21-225_21G7** | NA | GACATCCAGATGACCCAGTCTCCATCCTCCCTGTCTGCATCTGTAGGAGACAGAGTCACCATCACTTGCCGGGCAAGTCAGGGCGTTAGAAATGATTTAGGCTGGTATCAGCAGAAACCAGGGAAAGCCCCTCAGCGCCTGATCTATGCTGCATCCAGTTTGCAAAGTGGGGTCCCATCAAGGTTCAACGGCAGTGGATCTGGGACAGAATTCACTCTCACAATCAGCAGCCTGCAGCCTGAAGATTTTGCAACTTATTACTGTCTACAGCATAGTAGTTACCCGCTCACTTTCGGCGGAGGGACCAAGGTGGAGATCAAA | CAGCTGCAGCTGCAGGAGTCGGGCCCAGGACTGGTGAAGTTTTCGGAGACCCTGTCCCTCACCTGCACTGTCTCTGGTGGCTCCATCAGCAGAAGTAGTTACTACTGGGGCTGGATCCGCCAGCCCCCAGGGAAGGGGCAGGAGTGGATAGGGAATATCTATTATAGTGGGTATAGTTACTACAATCCGTCCCTCAAGAGTCGAGTCACCATATCCGTAGACACGTCCAAGAACCAGTTCTCCCTGAAGCTGAGCTCTGTGACCGCCGCAGACACGGCTGTGTATTACTGTGCGAGACATAGCACCAGCTGGTCCCTTGACTACTGGGGCCAGGGAACCCTGGTCACCGTCTCCTCA |
| | | | SEQ ID NO: 24489 | SEQ ID NO: 28495 |
| | | AA | DIQMTQSPSSLSASVGDRVTITCRASQGVRNDLGWYQQKPGKAPQRLIYAASSLQSGVPSRFNGSGSGTEFTLTISSLQPEDFATYYCLQHSSYPLTFGGGTKVEIK | QLQLQESGPGLVKFSETLSLTCTVSGGSISRSSYYWGWIRQPPGKGQEWIGNIYYSGYSYYNPSLKSRVTISVDTSKNQFSLKLSSVTAADTAVYYCARHSTSWSLDYWGQGTLVTVSS |
| | | | SEQ ID NO: 24490 | SEQ ID NO: 28496 |
| **iPS:392898** | **21-225_21H10** | NA | GAAATTGTGTTGACGCAGTCTCCAGGCACCCTGTCTTTGTCTCCAGGGGAAAGAGCCACCCTCTCCTGCAGGGCCAGTCAGAGTTTTAGCAGCAGCTACTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGGTAGCTCACGCAGTTTTGGCCAGGGGACCAAGCTGGAGATCAAA | GAGGTGCAGCTGGTGGAGTCTGGGGGAGGCTTGGTAAAGCCTGGGGGGTCCCTTAGACTCTCCTGTGCAGCCTCTGGATTCACTTTCAGTAACGCCTGGATGAACTGGGTCCGCCAGGCTCCAGGGAAGGGGCTGGAGTGGGTTGGCCGTATTAAAAGCAAAACTGATGGTGGGACAACAGACTACGCTGCACCCGTGAAAGGCAGATTCACCATCTCAAGAGATGATTCAAAAAACACGCTGTATCTGCAAATGAACAGCCTGAAAACCGAGGACACAGCCGTGTATTACTGTACCACAGAAGGCTGGAACACGGACTACTGGGGCCAGGGAACCCTGGTCACCGTCTCCTCA |
| | | | SEQ ID NO: 24491 | SEQ ID NO: 28497 |

FIGURE 50

| | | | | |
|---|---|---|---|---|
| | | AA | EIVLTQSPGTLSLSPGERATLSCRASQSFSSSYLAWYQQKPGQAPRLLIYGASSRATGIPDRFSGSGSGTDFTLTISRLEPEDFAVYYCQQYGSSRSFGQGTKLEIK | EVQLVESGGGLVKPGGSLRLSCAASGFTFSNAWMNWVRQAPGKGLEWVGRIKSKTDGGTTDYAAPVKGRFTISRDDSKNTLYLQMNSLKTEDTAVYYCTTEGWNTDYWGQGTLVTVSS |
| | | | SEQ ID NO: 24492 | SEQ ID NO: 28498 |
| iPS:392900 | 21-225_22F2 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTACAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGAGGGAAGTAATAAATACTATGTAGACTCCGTGAGGGGCCGATTCACCATCTCCAGAGACAATTCCAAGAGCACGCTGTATCTGCAAATGAACAGCCTGAGAGCCGAGGACACGGCTGTTTATTACTGTGTGAGAGAGCTAGGCTTCCAGTCTGACTACTGGGGCCAGGGAACCCCGGTCACCGTCTCCTCA |
| | | | SEQ ID NO: 24493 | SEQ ID NO: 28499 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVEIK | QVQLVESGGGVVQPGRSLRLSCAASGFTFSDYGMHWVRQAPGKGLEWVAVIWYEGSNKYYVDSVRGRFTISRDNSKSTLYLQMNSLRAEDTAVYYCVRELGFQSDYWGQGTPVTVSS |
| | | | SEQ ID NO: 24494 | SEQ ID NO: 28500 |

FIGURE 50

| | | | | |
|---|---|---|---|---|
| **iPS:392902** | **21-225_22D5** | NA | GACATCCAGATGACCCAGTCTCCATCCTCCCTGTCTGCATCTGTAGGAGACAGAGTCACCATCACTTGCCGGGCAAGTCAGAACATTTTTAGTTATTTAAATTGGTATCATCAGAAACCAGGGAAAGCCCCTAAGCTCCTGATCTATGCTGCATCCAGTTTGCAAAGTGGGGTCCCATCAAGGTTCAGTGGCAGTGGATCTGGGACAGATTTCACTCTCACCATCAGCAGTCTACAACCTGAAGATTTTGCAACTTACTACTGTCAACAGAGTTACAGTACCCCCTTATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGAATTCACCTTTAGCAGCTATGCCATGAGCTGGGTCCGCCAGGCTCCAGGGAAGGGGCTGGAGTGGGTCTCAGTTATTAGTGGTCGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGTCCCGTATAGCAGTGGCTGGCTCGGAGGCTTTTGATATCTGGGGCCAAGGGACAATGGTCACCGTCTCTTCA |
| | | | SEQ ID NO: 24495 | SEQ ID NO: 28501 |
| | | AA | DIQMTQSPSSLSASVGDRVTITCRASQNIFSYLNWYHQKPGKAPKLLIYAASSLQSGVPSRFSGSGSGTDFTLTISSLQPEDFATYYCQQSYSTPLFTFGPGTKVDIK | EVQLLESGGGLVQPGGSLRLSCAASEFTFSSYAMSWVRQAPGKGLEWVSVISGRGGNTFYADSVKGRFTISRDNSKNTLYLQMNSLRAEDTAVYYCASRIAVAGSEAFDIWGQGTMVTVSS |
| | | | SEQ ID NO: 24496 | SEQ ID NO: 28502 |
| **iPS:392904** | **21-225_22G9** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGAAAAGCCCCTAAGCGCCTGATCTATGCTGCTTCCAGTTTGCAAAGTGGGGTCCCATCAAGGTTCAGCGGCAGTGGATCTGGGACAGAGTTCACTCTCACAATCAGCAGCCTGCAGCCTGAAGATTTTGCAACTTATTACTGTCTACAGCATGCCAGTTACCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCCGGTGGCGCCATCAGCGGTAGTAATTACTACTGGGGCTGGATCCGCCAGCCCCCAGGGAAGGAACTGGAGTGGATTGGAAATATCTATTATAGTGGGAGCACCTACTACAACCCGTCCCTCAAGAGTCGAGTCACCATATCCGTAGACACGTCCAAGAACCAGTTCTCCCTGAAGCTGAGGTCTGTGACCGCCGAAGACACGGCTGTGTATTACTGTGCGAGACATAGCAGTAGCTGGTCCCTTGACTACTGGGGCCAGGGAACCCTGGTCACCGTCTCCTCA |
| | | | SEQ ID NO: 24497 | SEQ ID NO: 28503 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHASYPLTFGGGTKVEIK | QLQLQESGPGLVKPSETLSLTCTVSGGAISGSNYYWGWIRQPPGKELEWIGNIYYSGSTYYNPSLKSRVTISVDTSKNQFSLKLRSVTAEDTAVYYCARHSSSWSLDYWGQGTLVTVSS |
| | | | SEQ ID NO: 24498 | SEQ ID NO: 28504 |
| **iPS:392908** | **21-225_23F12** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGTTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAAGTTATTACTGTCTACAGCATAATAGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGTAGCGTCTGGATTCACCTTCAGTAGCTATGGCATGCACTGGGTCCGCCAGGCTCCAGGCAAGGGGCTGGAGTGGGTGGCAGTTATATGGTATGATGAAACTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTATTTATTACTGTGCGAGAGAGCTTGCCTGGTACGAGGACTACTGGGGCCAGGGATCCCTGGTCACCGTCTCCTCA |
| | | | SEQ ID NO: 24499 | SEQ ID NO: 28505 |
| | | AA | DIQMTQSPSSLSASVGDRVTITCRASQGIRNDLGWYQQKPGKAPKRLIYVASSLQSGVPSRFSGSGSGTEFTLTISSLQPEDFASYYCLQHNSYPWTFGQGTKVEIK | QVQLVESGGGVVQPGRSLRLSCVASGFTFSSYGMHWVRQAPGKGLEWVAVIWYDETNKYYADSVKGRFTISRDNSKNTLYLQMNSLRAEDTAIYYCARELAWYEDYWGQGSLVTVSS |
| | | | SEQ ID NO: 24500 | SEQ ID NO: 28506 |

FIGURE 50

| | | | | |
|---|---|---|---|---|
| **iPS:392912** | **21-225_25A9** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGTGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGTAACTAATAAATACTATACAGGCTCCGTGAAGGGCCGATTCACCATCTCCAGAGACAATTCCAAGAACACGCTGTATCTGCAAATGAACAGCCTGAGAGCCGAGGACACGGCTGTATATTACTGTGCGAGAGAAATTGGCTGGTTAGATGACTACTGGGGCCAGGGAACCCTGGTCACCGTCTCCTCA |
| | | | SEQ ID NO: 24501 | SEQ ID NO: 28507 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPFTFGPGTKVDIK | QVQLVESGGGVVQPGRSLRLSCAASGFTFSSYGMHWVRQAPGKGLEWVAVIWYDVTNKYYTGSVKGRFTISRDNSKNTLYLQMNSLRAEDTAVYYCAREIGWLDDYWGQGTLVTVSS |
| | | | SEQ ID NO: 24502 | SEQ ID NO: 28508 |
| **iPS:392914** | **21-225_25D12** | NA | GACATCCAGATGACCCAGTCTCCATCCTCCCTGTCTGCATCTGTAGGAGACAGAGTCACCATCACTTGCCGGACAAGTCAGGGCATTAGAAATGATTTAGGCTGGTATCAGCAGAAACCAGGGAAAGCCCCTAAGCGCCTGATCTATGCTGCATCCAGTTTACAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTTCCCTCGGACGTTCGGCCAAGGGACCAAAGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCGATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGAGGTATAGCAGCAGCTGGTACGACTACGGTATGGACGTCTGGGGCCAAGGGACCACGGTCACCGTCTCCTCA |
| | | | SEQ ID NO: 24503 | SEQ ID NO: 28509 |

FIGURE 50

| | | | | |
|---|---|---|---|---|
| | | AA | DIQMTQSPSSLSASVGDRVTITCRTSQGIRNDLG WYQQKPGKAPKRLIYAASSLQSGVPSRFSGSGS GTEFTLTISSLQPEDFATYYCLQHYSFPRTFGQGT KVEIK | QVQLVESGGGVVQPGRSLRLSCAASGFTFSSDGMH WVRQAPGKGLEWVAVIWYDGSNKYYADSVKGRF TISRDNSKNTLYLQMNSLRAEDTAVYYCARERYSS SWYDYGMDVWGQGTTVTVSS |
| | | | SEQ ID NO: 24504 | SEQ ID NO: 28510 |
| **iPS:392916** | **21-225_27C5** | NA | GACATCCAGATGACCCAGTCTCCCTCTTCCGT GTCTGCATCTGTAGGAGACAGAGTCACCATCA CTTGTCGGGCGAGTCAGGGTATTAGCAGCTGG TTAGCCTGGTATCAGCAGAAACCAGGGAAAG CCCCTAAGTTCCTGATCTATGGTGCATCCAGTT TGCAAAGTGGGGTCCCATCAAGGTTCAGCGCC AGTGGATCTGGGACAGAGTTCACTCTCACCAT CAGCAGCCTGCAGCCTGAAGATTTTGCAACTT ACTGTTGTCAACAGTATGACAGTTTCCCTCGG ACGTTCGGCCAAGGGACCAAGGTGGAAATCA AA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCACTAGTAGTAGTGATAGT TATATATACTACGCAGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGAGTGGCGTCC TTTGACTGCTGGGGCCAGGGAACCCTGGTCACCG TCTCCTCA |
| | | | SEQ ID NO: 24505 | SEQ ID NO: 28511 |
| | | AA | DIQMTQSPSSVSASVGDRVTITCRASQGISSWLA WYQQKPGKAPKFLIYGASSLQSGVPSRFSASGSG TEFTLTISSLQPEDFATYCCQQYDSFPRTFGQGTK VEIK | EVQLVESGGGLVKPGGSLRLSCAASGFTFSSYSMN WVRQAPGKGLEWVSSTSSSDSYIYYADSVKGRFTI SRDNAKNSLYLQMNSLRAEDTAVYYCARVASFDC WGQGTLVTVSS |
| | | | SEQ ID NO: 24506 | SEQ ID NO: 28512 |

FIGURE 50

| | | | | |
|---|---|---|---|---|
| **iPS:392918** | **21-225_28F5** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATACTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATACTTACCCGTGGACGTTCGGCCAAGGGACCAAGGTGGAAGTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGAAAATAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAATTAGGCTGGTACGACGACTACTGGGGCCAGGGAACCCTGGTCACCGTCTCCTCA |
| | | | SEQ ID NO: 24507 | SEQ ID NO: 28513 |
| | | AA | DIQMTQSPSSLSASVGDRVTITCRASQGIRNDLGWYQQKPGKAPKRLIYTASSLQSGVPSRFSGSGSGTEFTLTISSLQPEDFATYYCLQHNTYPWTFGQGTKVEVK | QVQLVESGGGVVQPGRSLRLSCAASGFTFSSYGMHWVRQAPGKGLEWVAVIWYDENNKYYADSVKGRFTISRDNSKNTLYLQMNSLRAEDTAVYYCARELGWYDDYWGQGTLVTVSS |
| | | | SEQ ID NO: 24508 | SEQ ID NO: 28514 |
| **iPS:392920** | **21-225_29G4** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCACA | CAGGTGCAGCTGGTGGAGTCTGGGGGAGGCGTGGTCCAGCCTGGGAGGTCCCTGAGACTCACCTGTGCAGCGTCTGGATTCACCTTCAGTGACTATGGCATACACTGGGTCCGCCAGGCTCCAGGCAAGGGGCTGGAGTGGGTGGCAGTTATATGGTATGATGAAAGTAATAAATACTATGCAGACTCCATGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGAATGACGGGTGACTACTGGGGCCAGGGAACCCTGGTCACCGTCTCCTCA |
| | | | SEQ ID NO: 24509 | SEQ ID NO: 28515 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVEIT | QVQLVESGGGVVQPGRSLRLTCAASGFTFSDYGIHWVRQAPGKGLEWVAVIWYDESNKYYADSMKGRFTISRDNSKNTLYLQMNSLRAEDTAVYYCARELGMTGDYWGQGTLVTVSS |
| | | | SEQ ID NO: 24510 | SEQ ID NO: 28516 |
| **iPS:392922** | **21-225_30G4** | NA | GACATCCAGATGACCCAGTCTCCACCCTCCCTGTCTACATCTGTAGGAGACAGAGTCACCATCACTTGCCGGGCAACTCAGAACATTTTCAGCTATTTAAATTGGCATCAGCAGAAACCAGGGAAAGCCCCTAAGCTCCTGATCCATACTGCATCCAGTTTGCAAGGTGGGGTCCCATCAAGGTTCAGTGGCAGTGGATCTGGGACAGATTTCACTCTCACCATCATCAGTATGCAACCTGAAGATTTTTCCACTTACTACTGTCAACTCAGCTACAGTCCCCCGTACACTTTTGGCGGAGGGACCAAGGTGGAGATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCCGGGGGCTGGAATGGGTGGCAGTTATATGGTATGATGGAACTGATAAATACTATGTAGACTCCGTGAAGGGCCGATTCACCATCTCCAGAGACAATTCCAAGAACACGCTGTATCTGCAAATGAACAGCCTGAGAGCCGAGGACTCGGCTGTGTATTACTGTGCGAGAGAAAATAGCAGCTCGTACTACTTTGACTACTGGGGCCAGGGAACCCTGGTCACCGTCTCCTCA |
| | | | SEQ ID NO: 24511 | SEQ ID NO: 28517 |
| | | AA | DIQMTQSPPSLSTSVGDRVTITCRATQNIFSYLNWHQQKPGKAPKLLIHTASSLQGGVPSRFSGSGSGTDFTLTIISMQPEDFSTYYCQLSYSPPYTFGGGTKVEIK | QVQLVESGGGVVQPGRSLRLSCAASGFTFSSYGMHWVRQAPGRGLEWVAVIWYDGTDKYYVDSVKGRFTISRDNSKNTLYLQMNSLRAEDSAVYYCARENSSSYYFDYWGQGTLVTVSS |
| | | | SEQ ID NO: 24512 | SEQ ID NO: 28518 |

FIGURE 50

| | | | | |
|---|---|---|---|---|
| **iPS:392924** | **21-225_32H2** | NA | GATATTGTGATGACCCAGACTCCACTCTCTCTGTCCGTCACCCCTGGACAGCCGGCCTCCATCTCCTGCAGGTCTAGTCAGAGCCTCCTCCATAGTGATGGAAGGACCTATTTGTATTGGTACCTGCAGAAGCCAGGCCAGCCTCCACAGCTCCTGATCTATGAACTTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGATTTATTCCTGCTTGCAAAGTATACAATATCCCATCACCTTCGGCCAAGGGACACGACTGGAGATTAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGAATCTGCAAATGAACAGCCTGAGAGCCGAGGACACGGGTGTGTATTACTGTGCGAGAAGATATAGCAGCAGCTGGACGGGGGGTATGGACGTCTGGGGCCAAGGGACCACGGTCACCGTCTCCTCA |
| | | | SEQ ID NO: 24513 | SEQ ID NO: 28519 |
| | | AA | DIVMTQTPLSLSVTPGQPASISCRSSQSLLHSDGRTYLYWYLQKPGQPPQLLIYELSNRFSGVPDRFSGSGSGTDFTLKISRVEAEDVGIYSCLQSIQYPITFGQGTRLEIK | QVQLVESGGGVVQPGRSLRLSCAASGFTFSSYGMHWVRQAPGKGLEWVAVIWYDGSNKYYADSVKGRFTISRDNSKNTLNLQMNSLRAEDTGVYYCARRYSSSWTGGMDVWGQGTTVTVSS |
| | | | SEQ ID NO: 24514 | SEQ ID NO: 28520 |
| **iPS:392928** | **21-225_25A4** | NA | GACATCGTGATGACCCAGTTTCCAGACTCCCTGGCTGTGTCTCTGGGCGAGAGGGCCACCATCAAGTGTAAGTCCAGCCAGAGTGTTTTATACAGCTCCCACAATAACAACTACTTAGCTTGGTACCAGCAGAAACCAGGACAGCCTCCTAAGTTGCTCCTTTACTGGGCATCTACCCGGGAATCCGGGGTCCCTGACCGATTCAGTGGCAGCGGGTCTGGGACAGAATTCACTCTCACCATCAGCAGCCTGGAGGCTGAAGATGTGGCAGTTTATTACTGTCAGCAGTATTATAGTACTCCTCCGACGTTCGGCCAAGGGACCAAGGTGGAAATCAAA | CAGGTGCTCCTGGTGCAGTCTGGGGCTGAGGTGAAGAGGCCTGGGGCCTCAGTGAAGGTCTCCTGCAAGGCTTCTGGATACACCTTCACCAATTATGATATTAATTGGGTGCGACAGGCCACTGGACAAGGGCTTGAGTGGATGGGATGGATGTACCCTAACAGTGGTAACACAGGCTATGCACAGAAATTCCAGGGCAGAGTCACCATGACCAGGAACACCTCCATAAGCACAGCCTACATGGAGCTGAGCAGCCTGAGATCTGAGGACACGGCCGTCTATTACTGTGCGAGTAGCAGTGGCTGGTACTACTTTGACTACTGGGGCCAGGGAACCCTGGTCACCGTCTCCTCA |
| | | | SEQ ID NO: 24515 | SEQ ID NO: 28521 |

FIGURE 50

| | | | | |
|---|---|---|---|---|
| | | AA | DIVMTQFPDSLAVSLGERATIKCKSSQSVLYSSHNNNYLAWYQQKPGQPPKLLLYWASTRESGVPDRFSGSGSGTEFTLTISSLEAEDVAVYYCQQYYSTPPTFGQGTKVEIK | QVLLVQSGAEVKRPGASVKVSCKASGYTFTNYDINWVRQATGQGLEWMGWMYPNSGNTGYAQKFQGRVTMTRNTSISTAYMELSSLRSEDTAVYYCASSSGWYYFDYWGQGTLVTVSS |
| | | | SEQ ID NO: 24516 | SEQ ID NO: 28522 |
| iPS:392930 | 21-225_25H9 | NA | GATATTGTGATGACCCAGACTCCACTCTCTCTGTCCGTCACCCCTGGACAGCCGGCCTCCATCTCCTGCAAGTCTAGTCAGAGCCTCCTGCATGGTGATGGAAAGACCTATTTGTTTTGGTACCTGCAGAAGCCAGGCCAGCCTCCACAGCTCCTGATCTATGAAGTTTCCAATCGGTTCTCTGGAGTGCCAGATAGGTTCAGTGGCAGCGGGTCAGGGACAGATTTCACACTGAAAATCAGCCGGGTGGAGGCTGAGGATGTTGGGGTTTATTACTGCATGCAAAGTATACAGCTTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCCCCTTCAATAACTATGGCATGCACTGGGTCCGCCAGGCTCCAGGCAAGGGGCTGGAGTGGGTGTCAATTATATGGTATGATGGAAGTTATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGGGATACGATTTTTGGAGTGGCTTCTTTGACTCCTGGGGCCAGGGAACCCTGGTCACCGTCTCCTCA |
| | | | SEQ ID NO: 24517 | SEQ ID NO: 28523 |
| | | AA | DIVMTQTPLSLSVTPGQPASISCKSSQSLLHGDGKTYLFWYLQKPGQPPQLLIYEVSNRFSGVPDRFSGSGSGTDFTLKISRVEAEDVGVYYCMQSIQLPWTFGQGTKVEIK | QVQLVESGGGVVQPGRSLRLSCAASGFPFNNYGMHWVRQAPGKGLEWVSIIWYDGSYKYYADSVKGRFTISRDNSKNTLYLQMNSLRAEDTAVYYCAREGYDFWSGFFDSWGQGTLVTVSS |
| | | | SEQ ID NO: 24518 | SEQ ID NO: 28524 |

FIGURE 50

| | | | | |
|---|---|---|---|---|
| **iPS:392934** | **21-225_27D5** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAACAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | CAGGTGCAACTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGAAAGTAATAAATACTATGGAGACTCCGTGAAGGGCCGATTCACCATCTCCAGAGACAATTCCAAGAATATGCTGTATCTGCAAATGAACAGCCTGAGAGGCGAGGACACGGCTCTGTATTACTGTGCGAGAGAACTGGGGTTCCTCTCTGACTACTGGGGCCAGGGAACCCTGGTCACCGTCTCCTCA |
| | | | SEQ ID NO: 24519 | SEQ ID NO: 28525 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPFTFGPGTKVDIK | QVQLVESGGGVVQPGRSLRLSCAASGFTFSSYGMHWVRQAPGKGLEWVAVIWYDESNKYYGDSVKGRFTISRDNSKNMLYLQMNSLRGEDTALYYCARELGFLSDYWGQGTLVTVSS |
| | | | SEQ ID NO: 24520 | SEQ ID NO: 28526 |
| **iPS:392936** | **21-225_28B6** | NA | GATGTTGTGATGACTCAGTCTCCACTCTCCCTGCCCGTCACCCTTGGACAGCCGGCCTCCATCTCCTGCCGGTCTAGTCAAAGCCTCGTATATAGTGATGGAGACACCTACTTGAATTGGTTTCAGCAGAGGCCAGGCCAATCTCCAAGGCGCCAAATTTATAAGGTTTCTAACTGGGACTCTGGGGTCCCAGACAGATTCAGCGGCAGTGGGTCAGGCACTGATTTCACACTGAACATCAGCAGGGTGGAGGCTGAGGATGTTGGGATTTATTTCTGCATGCATTGTACACACTGGCTCCTTTTCGGCCCTGGGACCAAAGTGGATATCAAA | CAGGTGCTCCTGGTGCAGTCTGGGGCTGAGGTGAAGAGGCCTGGGGCCTCAGTGAAGGTCTCCTGCAAGGCTTCTGGATACACCTTCACCAATTATGATATTAATTGGGTGCGACAGGCCACTGGACAAGGTCTTGAGTGGATGGGATGGATGCACCCTGACAGTGGTAACACAGGCTATGCACAGAAGTTCCAGGGCAGAGTCACCATGACCAGGAACACCTCCATAAGCACGGCCTACATGGAGCTGAGCAGCCTGAGATCTGAGGACACGGCCGTGTATTACTGTGCGAGTAGCAGTGGCTGGTACTACTTTGACTACTGGGGCCAGGGAACCCTGGTCACCGTCTCCTCA |
| | | | SEQ ID NO: 24521 | SEQ ID NO: 28527 |

FIGURE 50

| | | | | |
|---|---|---|---|---|
| | | AA | DVVMTQSPLSLPVTLGQPASISCRSSQSLVYSDGDTYLNWFQQRPGQSPRRQIYKVSNWDSGVPDRFSGSGSGTDFTLNISRVEAEDVGIYFCMHCTHWLLFGPGTKVDIK | QVLLVQSGAEVKRPGASVKVSCKASGYTFTNYDINWVRQATGQGLEWMGWMHPDSGNTGYAQKFQGRVTMTRNTSISTAYMELSSLRSEDTAVYYCASSSGWYYFDYWGQGTLVTVSS |
| | | | SEQ ID NO: 24522 | SEQ ID NO: 28528 |
| **iPS:392938** | **21-225_29H4** | NA | GATATTGTGATGACCCAGACTCCACTCTCTCTGTCCGTCACCCCTGGACAGCCGGCCTCCTTCTCCTGCAAGTCTAGTCAGAGCCTCCTGCATAGTGATGGAAAGACCTATTTGTATTGGTACCTGCAGAAGCCAGGCCAGCCTCCACAGCTCCTGATCTTTGAGGTTTCCCACCGGTTCTCTGGACTGCCAGATAGGTTCAGTGGCAGCGGGTCAGGGACAGATTTCACACTGAAAATCAGCCGGGTGGAGGCTGAGGATGTTGGGGTTTATTACTGCATGCAAAGTATACAGCATCCGTTCACTTTCGGCGGAGGGACCAGGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATACACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAAGGTATAGCAGCAGCTGGTCAGGGGGTATGGACGTCTGGGGCCAAGGGACCACGGTCACCGTCTCCTCA |
| | | | SEQ ID NO: 24523 | SEQ ID NO: 28529 |
| | | AA | DIVMTQTPLSLSVTPGQPASFSCKSSQSLLHSDGKTYLYWYLQKPGQPPQLLIFEVSHRFSGLPDRFSGSGSGTDFTLKISRVEAEDVGVYYCMQSIQHPFTFGGGTRVEIK | QVQLVESGGGVVQPGRSLRLSCAASGFTFSSYGIHWVRQAPGKGLEWVAVIWYDGSNKYYADSVKGRFTISRDNSKNTLYLQMNSLRAEDTAVYYCARRYSSSWSGGMDVWGQGTTVTVSS |
| | | | SEQ ID NO: 24524 | SEQ ID NO: 28530 |

FIGURE 50

| | | | | |
|---|---|---|---|---|
| iPS:392940 | 21-225_29D9 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTTTCAGCAGAAACCGGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATACTTACCCATTCACTTTCGGCCCTGGGACCAAAGTGGATTTCAAA | CAGGTGCAGCTGGTGGAGTCTGGGGGAGGCGTGGTCCAGCCTGGGAGGTCCCTGAAACTCTCCTGTTCAGCGTCTGGATTCACCTTCAGTGACTATGGCATTCACTGGGTCCGCCAGGCTCCAGGCAAGGGGCTGGAGTGGGTGGCAGTTATATGGTATGATGAAAGTAATAACTACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTACTACTGTGCGAGAGAAATTGGCTGGTTAGATGACTACTGGGGCCAGGGAACCCAGGTCACCGTCTCCTCA |
| | | | SEQ ID NO: 24525 | SEQ ID NO: 28531 |
| | | AA | DIQMTQSPSSLSASVGDRVTITCRASQGIRNDLGWFQQKPGKAPKRLIYAASSLQSGVPSRFSGSGSGTEFTLTISSLQPEDFATYYCLQHNTYPFTFGPGTKVDFK | QVQLVESGGGVVQPGRSLKLSCSASGFTFSDYGIHWVRQAPGKGLEWVAVIWYDESNNYYADSVKGRFTISRDNSKNTLYLQMNSLRAEDTAVYYCAREIGWLDDYWGQGTQVTVSS |
| | | | SEQ ID NO: 24526 | SEQ ID NO: 28532 |
| iPS:392942 | 21-225_30E9 | NA | GACATCCAGATGACCCAGTCTCCATCCTCCCTGTCTGCATCTGTAGGAGACAGAGTCACCATCACTTGCCGGGCAAGTCAGGACATTAGAGATGATTTAGGCTGGTATCAGCAGAAACCAGGGAAAGCCCCTAGGCGCCTGATCTATGGTGCATTCAGCTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATACTAGTTACCCTCCTACTTTCGGCGGAGGGACCAAGGTGGAGATCAAA | GAGGTGCAGGTGTTGGAGTCTGGGGGAGGCTTGGTACAGCCTGGGGGGTCCCTGAGACTCTCCTGTGCAGCCTCTGGATTCACCTTTAGCAGCTGTGCCATGAACTGGGTCCGCCAGGCTCCAGGGAAGGGGCTGGAGTGGGTCTCCGCTATTAGTGGTCGTGGTGGTAGCACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAAGGGGAGCTACTAGAGGACTACTACTACTACGGAATGGACGTCTGGGGCCAAGGGACCACGGTCACCGTCTCCTCA |
| | | | SEQ ID NO: 24527 | SEQ ID NO: 28533 |

FIGURE 50

| | | | | |
|---|---|---|---|---|
| | | AA | DIQMTQSPSSLSASVGDRVTITCRASQDIRDDLGWYQQKPGKAPRRLIYGAFSLQSGVPSRFSGSGSGTEFTLTISSLQPEDFATYYCLQHTSYPPTFGGGTKVEIK | EVQVLESGGGLVQPGGSLRLSCAASGFTFSSCAMNWVRQAPGKGLEWVSAISGRGGSTFYADSVKGRFTISRDNSKNTLYLQMNSLRAEDTAVYYCAKGELLEDYYYYGMDVWGQGTTVTVSS |
| | | | SEQ ID NO: 24528 | SEQ ID NO: 28534 |
| iPS:392944 | 21-225_31H5 | NA | GACATCCAGATGACCCAGTCTCCATCCTCCCTGTCTGCATCTGTAGGAGACAGATTCACCATCTCTTTCCGGGCAAGTCAGGACATTAGAAGTGATTTAGGCTGGTATCAGCAGAAACCAGGGAAAGCCCATAAGCGCATCATCTATGCTGCATCCAGTTTGCAAAGTGGGGTCCCATCAAGGTTCAGCGTCAGTGGATCTGGGACAGAATTCACTTTCACAATCAGCAGCATGCAGCCTGACGATTTTTCAAATTATTACTGTATACAACATATTATTTACCCTCCTACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCCGCTATTAGTGGTCGTGGTGGAAGCATATTCCACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGTGAAAGGGGAGCTACTAGAGGACTACTACTTCTACGGTATGGACGTCTGGGGCCAAGGGACCACGGTCACCGTCTCCTCA |
| | | | SEQ ID NO: 24529 | SEQ ID NO: 28535 |
| | | AA | DIQMTQSPSSLSASVGDRFTISFRASQDIRSDLGWYQQKPGKAHKRIIYAASSLQSGVPSRFSVSGSGTEFTFTISSMQPDDFSNYYCIQHIIYPPTFGGGTKVEIK | EVQLLESGGGLVQPGGSLRLSCAASGFTFSSYAMSWVRQAPGKGLEWVSAISGRGGSIFHADSVKGRFTISRDNSKNTLYLQMNSLRAEDTAVYYCVKGELLEDYYFYGMDVWGQGTTVTVSS |
| | | | SEQ ID NO: 24530 | SEQ ID NO: 28536 |

FIGURE 50

| | | | | |
|---|---|---|---|---|
| **iPS:392948** | **21-225_25G5** | NA | GACATCCAGATGACCCAGTCTCCATCCTCACTGTCTGCATCTGTTGGAGACAGAGTCACCATCACTTGCCGGGCAAGTCAGGACATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAGTCAGCAGCCTGCAGCCTGAAGATTTTGCAACTTATTACTGTCTACAGCATAATAGTTACCCCTTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATACACTGGGTCCGCCAGGCTCCAGGCAAGGGGCTGGAGTGGGTGGCAGTTATTTGGTATGATGGAAATAATAAATATTATGCAGACTCCGTGAAGGGCCGATTCACCATCTCCAGAGACAATTCCAAGAACACGCTGTATCTGCAAATGAACAACCTGAGAGCCGAGGACACGGCTGTGTATTACTGTGCGAGAGAAATTGGCTGGTTAGATGACTACTGGGGCCAGGGAACCCTGGTCACCGTCTCCTCA |
| | | | SEQ ID NO: 24531 | SEQ ID NO: 28537 |
| | | AA | DIQMTQSPSSLSASVGDRVTITCRASQDIRNDLGWYQQKPGKAPKRLIYAASSLQSGVPSRFSGSGSGTEFTLTVSSLQPEDFATYYCLQHNSYPFTFGPGTKVDIK | QVQLVESGGGVVQPGRSLRLSCAASGFTFSDYGIHWVRQAPGKGLEWVAVIWYDGNNKYYADSVKGRFTISRDNSKNTLYLQMNNLRAEDTAVYYCAREIGWLDDYWGQGTLVTVSS |
| | | | SEQ ID NO: 24532 | SEQ ID NO: 28538 |
| **iPS:392950** | **21-225_25C10** | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAACCAGGGAAAGCCCCTAAGTCCCTGATCTATGCTGCATCCAGTTTGCAAAGTGGGTTCCCATCAAAGTTCAGCGGCAGTGGATCTGGGACAGATTTCACTCTCACCATCAGCAGCCTGCAGCCTGAAGATTTTGCAACTTATTACTGCCAACAGTATCATAGTTACCCATTCACTTTCGGCCCTGGGACCAAAGTGGATATCAAA | GAGGTGCAGTTGGTGGAGTCTGGGGGAGGCTTGGTACAGCCTGGGGGGTCCCTGAGACTCTCCTGTGCAGCCTCTGGATTCACCTTCAGTAGCTATAGGATGAACTGGGTCCGCCAGGCTCCAGGGAAGGGGCTGGAGTGGGTTTCATCCATTAGTAGTAGTAGTAGTACCATATACTACGCAGACTCTGTGAAGGGCCGATTCACCATCTCCAGAGACAATGCCAAGAACTCACTGTATCTGCAAATGAACAGCCTGAGAGACGAGGACACGGCTGTGTATTACTGTGCGAGAACGGCTGGTTTTGACTACTGGGGCCAGGGAACCCTGGTCACCGTCTCCTCA |
| | | | SEQ ID NO: 24533 | SEQ ID NO: 28539 |

FIGURE 50

| | | | | |
|---|---|---|---|---|
| | | AA | DIQMTQSPSSLSASVGDRVTITCRASQGISNYLA WFQQKPGKAPKSLIYAASSLQSGFPSKFSGSGSG TDFTLTISSLQPEDFATYYCQQYHSYPFTFGPGT KVDIK | EVQLVESGGGLVQPGGSLRLSCAASGFTFSSYRMN WVRQAPGKGLEWVSSISSSSSTIYYADSVKGRFTIS RDNAKNSLYLQMNSLRDEDTAVYYCARTAGFDY WGQGTLVTVSS |
| | | | SEQ ID NO: 24534 | SEQ ID NO: 28540 |
| iPS:392952 | 21-225_26G1 | NA | GACATCCAGATGACCCAGTCTCCATCCTCACT GTCTGCATCTGTAGGAGACAGAGTCACCATCA CTTGTCGGGCGAGTCAGGACATTAGCAATTAT TTAGCCTGGTTTCAGCAGAAACCAGGGAAAGC CCCTAAGTCCCTGATCTATGCTGCATCCAGTTT GCGAAGTGGGGTCCCATCAAACTTCAGCGGCA GTGGATCTGGGACAGATTTCACTCTCACCATC AGCAGCCTGCAGCCTGAAAATTTTGCAACTTA TTACTGCCAACAGTATCATAGTTACCCATTCA CTTTCGGCCCTGGGACCAAAGTGGATATCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATGGCAT GAACTGGGTCCGCCAGGCTCCAGGGAAGGGGCT GGAGTGGGTCTCATCCATTAGTGGTAGTAGTAGT TACATATACTACGCGGACTCAGTGAAGGGCCGAT TCACCATCTCCAGAGACAACGCCAAGAACTCACT GTATCTGCAAATGAACAGCCTGAGAGCCGAGGA CACGGCTGTGTATTACTGTGCGAGACTGACTACC TTTGACTTCTGGGGCCAGGGAACCCTGGTCACCG TCTCCTCA |
| | | | SEQ ID NO: 24535 | SEQ ID NO: 28541 |
| | | AA | DIQMTQSPSSLSASVGDRVTITCRASQDISNYLA WFQQKPGKAPKSLIYAASSLRSGVPSNFSGSGSG TDFTLTISSLQPENFATYYCQQYHSYPFTFGPGT KVDIK | EVQLVESGGGLVKPGGSLRLSCAASGFTFSSYGMN WVRQAPGKGLEWVSSISGSSSYIYYADSVKGRFTIS RDNAKNSLYLQMNSLRAEDTAVYYCARLTTFDFW GQGTLVTVSS |
| | | | SEQ ID NO: 24536 | SEQ ID NO: 28542 |

FIGURE 50

| | | | | |
|---|---|---|---|---|
| **iPS:392954** | **21-225_26A10** | NA | GACATCCAGATGACCCAGTCTCCATCCTCCCTGTCTGCATCTGTAGGAGACAGAATCACCATCACTTGCCGGGCAAGTCAGAGCATTAGCAGCTATTTAAATTGGTATCAGCAGAAACCAGGGAAAGCCCCTAAGGTCCTGATCTATGCTGCATCCAGCTTGCAAAGTGGGGTCCCATCAAGATTCAGTGGCAGTGGATCTGGGACAGATTTCACTCTCACCATCAGCAGTCTGCAACCTGAAGATTTTGCAACTTACTACTGTCAACAGAGTTACAGTACCCCTACGTGGACGTTCGGCCAAGGGACCAAGGTGGAAATCAAA | GAGGTGCAGCTGCTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGTCTGGAGTGGGTCTCAGTTATTAGTGGTAGTGGTGTTAACACATTCTACGCAGACTCCGTGAAGGGCCGGTTCACCATCTCCAGAGACAATTCCAAGAACACGCTGTATCTGCTAATGAACAGCCTGAGAGCCGAGGACACGGCCGTATATTACTGTGCGAAAAAGATAGCAGTGGCTGGTACTCACTACTTTGACTACTGGGGCCAGGGAACCCTGGTCACCGTCTCCTCA |
| | | | SEQ ID NO: 24537 | SEQ ID NO: 28543 |
| | | AA | DIQMTQSPSSLSASVGDRITITCRASQSISSYLNWYQQKPGKAPKVLIYAASSLQSGVPSRFSGSGSGTDFTLTISSLQPEDFATYYCQQSYSTPTWTFGQGTKVEIK | EVQLLESGGGLVQPGGSLRLSCAASGFTFSSYAMSWVRQAPGKGLEWVSVISGSGVNTFYADSVKGRFTISRDNSKNTLYLLMNSLRAEDTAVYYCAKKIAVAGTHYFDYWGQGTLVTVSS |
| | | | SEQ ID NO: 24538 | SEQ ID NO: 28544 |
| **iPS:392956** | **21-225_27A11** | NA | GACATCCAGATGACCCAGTCTCCCTCTTCCGTGTCTGCATCTGTAGGAGACAGAGTCACCATCACTTGTCGGGCGAGTCAGGGTATTAGTAGTTGGTTAGCCTGGTATCAGCAGAAACCAGGGAAAGCCCCTAAGCTCCTGATCTATGGTGCATCCAGTTTGCAAAGTGGGGTCCCATCAAGGTTCAGCGCCAGTGGATCTGGGACAGATTTCACTCTCACCATCAGCAGCCTGCAGCCTGAAGATTTTGCAACTTACTGTTGTCAACAGTCTGACAGTTTCCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGCAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTATATCTACAAATGAACAGCCTGAGAGCCGAGGACACGGCTGTGTATCACTGTGCGAGAGATTCCTCCCCCTACGGTATGGACGTCTGGGGCCAAGGGACCACGGTCACCGTCTCCTCA |
| | | | SEQ ID NO: 24539 | SEQ ID NO: 28545 |

FIGURE 50

| | | | | |
|---|---|---|---|---|
| | | AA | DIQMTQSPSSVSASVGDRVTITCRASQGISSWLAWYQQKPGKAPKLLIYGASSLQSGVPSRFSASGSGTDFTLTISSLQPEDFATYCCQQSDSFPRTFGQGTKVEIK | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYHCARDSSPYGMDVWGQGTTVTVSS |
| | | | SEQ ID NO: 24540 | SEQ ID NO: 28546 |
| **iPS:392958** | **21-225_28C7** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATATTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATACTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGAAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAATTAGGCTGGTACGACGACTACTGGGGCCAGGGAACCCTGGTCACCGTCTCCTCT |
| | | | SEQ ID NO: 24541 | SEQ ID NO: 28547 |
| | | AA | DIQMTQSPSSLSASVGDRVTITCRASQGIRNDLGWYQQKPGKAPKRLIYIASSLQSGVPSRFSGSGSGTEFTLTISSLQPEDFATYYCLQHNTYPWTFGQGTKVEIK | QVQLVESGGGVVQPGRSLRLSCAASGFTFSSYGMHWVRQAPGKGLEWVAVIWYDESNKYYADSVKGRFTISRDNSKNTLYLQMNSLRAEDTAVYYCARELGWYDDYWGQGTLVTVSS |
| | | | SEQ ID NO: 24542 | SEQ ID NO: 28548 |

FIGURE 50

| | | | | |
|---|---|---|---|---|
| **iPS:392960** | **21-225_29E6** | NA | GACATCGTGATGACCCAGTTTCCAGACTCCCTGGCTGTGTCTCTGGGCGAGAGGGCCACCATCAACTGTAAGTCCAGCCAGAGTGTTTTATACAGCTCCCACAATAACTACTACTTAACTTGGTACCAGCAGAAACCAGGACAGCCTCATAAGTTGCTCCTTTACTGGGCATCTTCCCGGGAATCCGGGGTCCCTGACCGATTCAGTGGCAGCGGGTCTGGGACAGAATTCACTCTCACCATCAGCAGCCTGCAGGCTGAAGATGTGGCAGTTTATTACTGTCAGCAGTATTATAGTACTCCTCCGACGTTCGGCCAAGGGACCAAGGTGGAAATCAAA | CAGGTGCTCCTGGTGCAGTCTGGGGCTGAGGTGAAGAGGCCTGGGGCCTCAGTGAAGGTCTCCTGCAAGGCTTCTGGATACACCTTCACCAATTATGATATTAATTGGGTGCGACAGGCCACTGGACAAGGGCTTGAGTGGATGGGATGGATGCACCCTAACAGTGGTAACACAGGCTATGCACAGAAATTCCAGGGCAGAGTCACCATGACCAGGAACACCTCCATAAGCACAGCCTACATGGAGCTGAGCAGCCTGAGATCTGAGGACACGGCCGTCTATTACTGTGCGAGTAGCAGTGGCTGGTACTACTTTGACTACTGGGGCCAGGGAACCCTGGTCACCGTCTCCTCA |
| | | | SEQ ID NO: 24543 | SEQ ID NO: 28549 |
| | | AA | DIVMTQFPDSLAVSLGERATINCKSSQSVLYSSHNNYYLTWYQQKPGQPHKLLLYWASSRESGVPDRFSGSGSGTEFTLTISSLQAEDVAVYYCQQYYSTPPTFGQGTKVEIK | QVLLVQSGAEVKRPGASVKVSCKASGYTFTNYDINWVRQATGQGLEWMGWMHPNSGNTGYAQKFQGRVTMTRNTSISTAYMELSSLRSEDTAVYYCASSSGWYYFDYWGQGTLVTVSS |
| | | | SEQ ID NO: 24544 | SEQ ID NO: 28550 |
| **iPS:392962** | **21-225_30A1** | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAACCAGGGAAAGCCCCTAAGTCCCTGATCTCTGCTGCATCCAGTTTGCAAACTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATAATAGTTACCCATTCACTTTCGGCCCTGGGACCAAAGTGGATATCAAA | GAGGTGCAGCTGTTGGAGTCTGGGGGAGGCCTGGTACAGCCGGGGGGGTCCCTGAGACTCTCCTGTGCAGCCTCTGGATTCACCTTTAGCAGCTATGTCATGAACTGGGTCCGCCAGGCTCCAGGGAAGGGGCTGGAGTGGGTCTCAGCTATTAGTGGTAGTGGTGGTAGCACATACTACGCAGACTCCGTGAAGGGCCGGTTCACCATCTCCAGAAACAATTCCAAGAACACGCTGTATCTGCAAATGAACAGCCTGAGAGCCGAGGACACGGCCGTATATTACTGTGCGCGAACTGGGGTCTTTGACTACTGGGGCCAGGGAACCCTGGTCACCGTCTCCTCA |
| | | | SEQ ID NO: 24545 | SEQ ID NO: 28551 |

FIGURE 50

| | | | | |
|---|---|---|---|---|
| | | AA | DIQMTQSPSSLSASVGDRVTITCRASQGISNYLA<br>WFQQKPGKAPKSLISAASSLQTGVPSKFSGSGSG<br>TDFTLTISSLQPEDFATYYCQQYNSYPFTFGPGT<br>KVDIK | EVQLLESGGGLVQPGGSLRLSCAASGFTFSSYVMN<br>WVRQAPGKGLEWVSAISGSGGSTYYADSVKGRFTI<br>SRNNSKNTLYLQMNSLRAEDTAVYYCARTGVFDY<br>WGQGTLVTVSS |
| | | | SEQ ID NO: 24546 | SEQ ID NO: 28552 |
| **iPS:392964** | **21-225_31A8** | NA | GACATCCAGATGACCCAGTCTCCATCCTCCCT<br>GTCTGCATCTGTAGGAGACAGAGTCACCATCA<br>CTTGCCGGGCAAGTCAGGACATTAGAAGTGAT<br>TTAGGCTGGTATCAGCAGAAACCAGGGAAAG<br>CCCCTAAGCGCCTGATCTATGCTGTATCCAGTT<br>TGCAAAGTGGGGTCCCATCAAGGTTCAGCGGC<br>AGTGGCTCTGGGACAGAATTCACTCTCACAAT<br>CAGCAGCCTGCAGCCTGAAGATTTTGCCACTT<br>ATTACTGTCTACAGCATACTATTTACCCTCCTA<br>CTTTCGGCGGAGGGACCAAGGTGGAGATCAA<br>A | GAGGTGCAGCTGTTGGAGTCTGGGGGAGGCTTG<br>GTACAGCCTGGGGGGTCCCTGAGACTCTCCTGTG<br>CAGCCTCTGGATTCACCTTTAGCAGCTATGCCAT<br>GAGCTGGGTCCGCCAGGCTCCAGGGAAGGGGCT<br>GGAGTGGGTCTCCGCTATTAGTGGTCGTGGTGGT<br>AGCACATTCCACGCAGACTCCGTGAAGGGCCGG<br>TTCACCATCTCCAGAGACAATTCCAAGAACACGC<br>TGTATCTGCAAATGAACAGCCTGAGAGACGAGG<br>ACACGGCCGTATATTACTGTGTGAAAGGGGAGCT<br>ACTAGAGGACTACTACTTCTACGGTATGGACGTC<br>TGGGGCCAAGGGACCACGGTCACCGTCTCCTCA |
| | | | SEQ ID NO: 24547 | SEQ ID NO: 28553 |
| | | AA | DIQMTQSPSSLSASVGDRVTITCRASQDIRSDLG<br>WYQQKPGKAPKRLIYAVSSLQSGVPSRFSGSGS<br>GTEFTLTISSLQPEDFATYYCLQHTIYPPTFGGGT<br>KVEIK | EVQLLESGGGLVQPGGSLRLSCAASGFTFSSYAMS<br>WVRQAPGKGLEWVSAISGRGGSTFHADSVKGRFTI<br>SRDNSKNTLYLQMNSLRDEDTAVYYCVKGELLED<br>YYFYGMDVWGQGTTVTVSS |
| | | | SEQ ID NO: 24548 | SEQ ID NO: 28554 |

FIGURE 50

| | | | | |
|---|---|---|---|---|
| **iPS:392966** | **21-225_32G3** | NA | GACATCCAGATGACCCAGTCTCCAACCTCACTGTCTGCATCTGTCGGAGACAGAGTCACCATCACTTGTCGGGCGAGTCAGGCCATTAGCAATTATTTAGCCTGGTTTCAGCAGAAACCAGGGAAAGCCCCTAAGTCCCTGATCTATGATACATCCAGTTTGCAAAGTGGGGTCCCATCAAAATTCAGCGGCAGTGGGTCTGGGACAGATTTCACTCTCACCATCAGCACCCTACAGCCTGAAGATTTTGCAACTTATTACTGCCAACAGTATCATAGTTACCCGCTCACTTTCGGCGGAGGGACCAAGGTGGAGATCAAG | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTAGTTACATATACTACGCAGACTCAGTGAAGGGCCGATTCACCATCTCCAGAGACAACGCCAAGAACTCACTGTATCTGCAAATCAACAGCCTGAGAGCCGAGGACACGGCTGTGTATTACTGTGCGAGAGGCAATATAGCAAGGGACTACTGGGGCCAGGGAACCCTGGTCACCGTCTCCTCA |
| | | | SEQ ID NO: 24549 | SEQ ID NO: 28555 |
| | | AA | DIQMTQSPTSLSASVGDRVTITCRASQAISNYLAWFQQKPGKAPKSLIYDTSSLQSGVPSKFSGSGSGTDFTLTISTLQPEDFATYYCQQYHSYPLTFGGGTKVEIK | EVQLVESGGGLVKPGGSLRLSCAASGFTFSSYSMNWVRQAPGKGLEWVSSISGSSSYIYYADSVKGRFTISRDNAKNSLYLQINSLRAEDTAVYYCARGNIARDYWGQGTLVTVSS |
| | | | SEQ ID NO: 24550 | SEQ ID NO: 28556 |
| **iPS:392968** | **21-225_25B6** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGAAAAGCCCCTAAGCGCCTGATCTATCGTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGAGTCTCCTGTACAGCGTCTGGATTCACCCTCAGAAACTATGGCATGCACTGGGTCCGCCAGGCTCCAGGCAAGGGGCTGGAGTGGGTGGCAGTTATATGGTATGAGGAAAGTAATAAATACTATACAGAGTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGTTCCTCTCTGACTACTGGGGCCAGGGAACCCTGGTCACCGTCTCCTCA |
| | | | SEQ ID NO: 24551 | SEQ ID NO: 28557 |

FIGURE 50

| | | | | |
|---|---|---|---|---|
| | | AA | DIQMTQSPSSLSASVGDRVTITCRASQGIRNDLGWYQQKPGKAPKRLIYRASSLQSGVPSRFSGSGSGTEFTLTISSLQPEDFATYYCLQHNSYPFTFGPGTKVDIK | QVQLVESGGGVVQPGRSLRVSCTASGFTLRNYGMHWVRQAPGKGLEWVAVIWYEESNKYYTESVKGRFTISRDNSKNTLYLQMNSLRAEDTAVYYCARELGFLSDYWGQGTLVTVSS |
| | | | SEQ ID NO: 24552 | SEQ ID NO: 28558 |
| iPS:392972 | 21-225_26A2 | NA | GACATCCAGATGACCCAGTCTCCATCCTCCCTGTCTGCATCTGTAGGAGACAGAGTCACCATCACTTGCCGGGCAAGTCAGGGCATTAGAAGTGATTTAGGCTGGTATCAGCAGAAACCAGGGAAAGCCCCTAAGCGCCTGATCTATACTGCATCCAGTTTGCAGAGTGGGGTCCCATCAAGATTCAGCGGCAGTGGATCTGGGACAGAATTCACTCTCACATTCAGCAGCCTGCAGCCTGAAGATTTTGCAACGTATTACTGTCTACAGCATAATCGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGAAGGAAGTAATAAATACTATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAATTAGGCTGGTACGACGACTACTGGGGCCAGGGAACCCTGGTCACCGTCTCCTCA |
| | | | SEQ ID NO: 24553 | SEQ ID NO: 28559 |
| | | AA | DIQMTQSPSSLSASVGDRVTITCRASQGIRSDLGWYQQKPGKAPKRLIYTASSLQSGVPSRFSGSGSGTEFTLTFSSLQPEDFATYYCLQHNRYPWTFGQGTKVEIK | QVQLVESGGGVVQPGRSLRLSCAASGFTFSSYGMHWVRQAPGKGLEWVAVIWYEGSNKYYVDSVKGRFTISRDNSKNTLYLQMNSLRAEDTAVYYCARELGWYDDYWGQGTLVTVSS |
| | | | SEQ ID NO: 24554 | SEQ ID NO: 28560 |

FIGURE 50

| | | | | |
|---|---|---|---|---|
| **iPS:392974** | **21-225_26A11** | NA | GACATCCAGATGACCCAGTCTCCAATTTCCCTGTCTGCATCTGTAGGAGACAGAGTCACCATCACTTGCCGGGCAAGTCAGGCCATTAGAAATGATTTAGGCTGGTATCAGCAGAGACCAGGGAAAGCCCCTAAGCGCCTGATCTATGCTGCATCCAGTTTGCAAAGTGGGGTCCCATCAAGGTTCAGCGGCAGTGGATCTGGGACAGAATTTATTCTCACAATCAGCAGCCTGCAGCCTGAAGATTTTGCAACTTATTACTGTCTACAGCATTATAATTACCCTCGCAGTTTTGGCCAGGGGACCAAGCTGGAGATCAGA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTGTGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGGAGTATAGCAGTGGCTGGTACGACTACGGTATGGACGTCTGGGGCCAAGGGACCACGGTCACCGTCTCCTCA |
| | | | SEQ ID NO: 24555 | SEQ ID NO: 28561 |
| | | AA | DIQMTQSPISLSASVGDRVTITCRASQAIRNDLGWYQQRPGKAPKRLIYAASSLQSGVPSRFSGSGSGTEFILTISSLQPEDFATYYCLQHYNYPRSFGQGTKLEIR | QVQLVESGGGVVQPGRSLRLSCAASGFTFSNCVMHWVRQAPGKGLEWVAVIWYDGSNKYYADSVKGRFTISRDNSKNTLYLQMNSLRAEDTAVYYCAREEYSSGWYDYGMDVWGQGTTVTVSS |
| | | | SEQ ID NO: 24556 | SEQ ID NO: 28562 |
| **iPS:392976** | **21-225_27H12** | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAACCAGGGAAAGCCCCTAAGTCCCTGATCAATGGTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATTATAGTTACCCATTCACTTTCGGCCCTGGGACCAAAGTGAATATCAAT | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCCTGAACTGGGTCCGCCAGGCTCCAGGGAAGGGGCTGGAGTGGGTCTCATCCATTAGTGGTAGTAGTAGTAACATATACTACACAGACTCAGTGAAGGGCCGATTCACCATCTCCAGAGACAACGCCAAGAACTCACTGTTTCTGCAAATGAACAGCCTGAGAGCCGAGGACACGGCTGTGTATTACTGTGCGAGAGTGGCGTCCTTTGACTACTGGGGCCAGGGAACCCTGGTCACCGTCTCCTCA |
| | | | SEQ ID NO: 24557 | SEQ ID NO: 28563 |

FIGURE 50

| | | | | |
|---|---|---|---|---|
| | | AA | DIQMTQSPSSLSASVGDRVTITCRASQGISNYLAWFQQKPGKAPKSLINGASSLQSGVPSKFSGSGSGTDFTLTISSLQPEDFATYYCQQYYSYPFTFGPGTKVNIN | EVQLVESGGGLVKPGGSLRLSCAASGFTFSSYSLNWVRQAPGKGLEWVSSISGSSSNIYYTDSVKGRFTISRDNAKNSLFLQMNSLRAEDTAVYYCARVASFDYWGQGTLVTVSS |
| | | | SEQ ID NO: 24558 | SEQ ID NO: 28564 |
| iPS:392978 | 21-225_28B8 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCACAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAT | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGACAGTTATATGGTATGATGCAAATAATAAATACTATGCAGACTCCGTGAAGGGCCGATTCACCATCTCCAGAGACAATTTCAAAAACACGGTGTATCTGCAAATGAACAGCCTGAGAGCCGAGGACACGGCTGTGTATTACTGTGCGAGAGAAATTGGCTGGTTAGATGACTACTGGGGCCAGGGAACCCTGGTCACCGTCTCCTCA |
| | | | SEQ ID NO: 24559 | SEQ ID NO: 28565 |
| | | AA | DIQMTQSPSSLSASVGDRVTITCRASQGIRNDLGWYQHKPGKAPKRLIYAASSLQSGVPSRFSGSGSGTEFTLTISSLQPEDFATYYCLQHNSYPFTFGPGTKVDIN | QVQLVESGGGVVQPGRSLRLSCAASGFTFSDYGMHWVRQAPGKGLEWVTVIWYDANNKYYADSVKGRFTISRDNFKNTVYLQMNSLRAEDTAVYYCAREIGWLDDYWGQGTLVTVSS |
| | | | SEQ ID NO: 24560 | SEQ ID NO: 28566 |

FIGURE 50

| | | | | |
|---|---|---|---|---|
| iPS:392980 | 21-225_29H6 | NA | GACATCCAGATGACCCAGTCTCCATCCTCCCT<br>GTCTGCATCTGTAGGAGACAGAGTCACCATCA<br>CTTGCCGGGCAAGTCAGGGCATTAGAAATGAT<br>TTAGGCTGGTATCAGCAGAAATCAGGGAAAA<br>CCCCTAAGCGCCTGATCTATGCTGCATCCAGT<br>TTGCAAAGTGGGGTCCCATCAAGGTTCAGCGG<br>CAGTGGATCTGGGACAGAATTCACTCTCACAA<br>TCAGCAGTCTGCAGCCTGAAGATTTTGCAACC<br>TATTACTGTCTACAGCATAATAGTTACCCGCTC<br>ACTTTCGGCGGAGGGACCAAGGTGGAGATCA<br>AA | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTGACTATGGCAT<br>GCACTGGGTCCGCCAGGCTCCAGGCAAGGGGCT<br>GGAGTGGGTGACAGTTATATGGTATAATGAAAAT<br>AATAAATACTATGCAGACTCCGTGAAGGGCCGA<br>TTCACCATCTCCAGAGACAATTCCAAGAACACGC<br>TGTATCTGCAAATGAACAGCCTGAGAGCCGAGG<br>ACACGGCTGTGTATTACTGTGCGAGAGAACTGGG<br>GATGACGGGTGACTCCTGGGGCCAGGGAACCCT<br>GGTCACCGTCTCCTCA |
| | | | SEQ ID NO: 24561 | SEQ ID NO: 28567 |
| | | AA | DIQMTQSPSSLSASVGDRVTITCRASQGIRNDLG<br>WYQQKSGKTPKRLIYAASSLQSGVPSRFSGSGSG<br>TEFTLTISSLQPEDFATYYCLQHNSYPLTFGGGT<br>KVEIK | QVQLVESGGGVVQPGRSLRLSCAASGFTFSDYGM<br>HWVRQAPGKGLEWVTVIWYNENNKYYADSVKGR<br>FTISRDNSKNTLYLQMNSLRAEDTAVYYCARELGM<br>TGDSWGQGTLVTVSS |
| | | | SEQ ID NO: 24562 | SEQ ID NO: 28568 |
| iPS:392982 | 21-225_30D1 | NA | GACATCCAGATGACCCAGTCTCCATCCTCCCT<br>GTCTGCATCTGTAGGAGACAGAGTCACCATCA<br>CTTGCCGGGCAAGTCAGGACATTAGAAGTGAT<br>TTAGGCTGGTATCAACAGAAACCAGGGAAAG<br>CCCCTGAGCGCCTGATCTATGCTGCATCCAGT<br>TTGCAAAGTGGGGTCCCATCAAGGTTCAGCGG<br>CAGTGGATCTGGGACAGAATTCACTCTCACAA<br>TCAGCAGCCTGCAGCCTGAAGATTTTACAACT<br>TATTACTGTCTACAGCATACTATTTACCCTCCT<br>ACTTTCGGCGGAGGGACCAAGGTGGAGATCA<br>AA | GAGGTGCAGGTGTTGGAGTCTGGGGGAGGCTTG<br>GTACAGCCTGGGGGGTCCCTGAGACTCTCCTGTG<br>CAGCCTCTGGATTCACCTTTAGCAGCTATGCCAT<br>GAGCTGGGTCCGCCAGGCTCCAGGGAAGGGGCT<br>GGAGTGGGTCTCCGCTATTAGTGGTCGTGGTGGT<br>AGCACATTCCACGCAGACTCCGTGAAGGGCCGG<br>TTCACCATCTCCAGAGACAATTCCAAGAACACGC<br>TGTATCTGCAAATGAACAGCCTGAGAGCCGAGG<br>ACACGGCCGTATATTACTGTGTGAAAGGGGAGCT<br>ACTAGAGGACTACTACTTCTACGGTTTGGACGTC<br>TGGGGCCAAGGGACCACGGTCACCGTCTCCTCA |
| | | | SEQ ID NO: 24563 | SEQ ID NO: 28569 |

FIGURE 50

| | | | | |
|---|---|---|---|---|
| | | AA | DIQMTQSPSSLSASVGDRVTITCRASQDIRSDLGWYQQKPGKAPERLIYAASSLQSGVPSRFSGSGSGTEFTLTISSLQPEDFTTYYCLQHTIYPPTFGGGTKVEIK | EVQVLESGGGLVQPGGSLRLSCAASGFTFSSYAMSWVRQAPGKGLEWVSAISGRGGSTFHADSVKGRFTISRDNSKNTLYLQMNSLRAEDTAVYYCVKGELLEDYYFYGLDVWGQGTTVTVSS |
| | | | SEQ ID NO: 24564 | SEQ ID NO: 28570 |
| **iPS:392984** | **21-225_30E11** | NA | GACATCCAGATGACCCAGTCTCCATCCTCCCTGTCTGCATCTGTAGGAGACAGAGTCACCATCACTTGCCGGGCAAGTCAGAGCATTAGCAACTATTTAAATTGGTATCAACAGCAAACAGGGAAAGCCCCTAAGTTCCTGATCTATGCTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACAGTACCCCATTCACTTTCGGCCCTGGGACCAAAGTGGATATCAAA | GAGGTGCAGCTGTTGGAGTCTGGGGGAGACATGGTCCAGCCTGGGGGGTCCCTGAGACTCTCCTGTGCAGCCTCCGGATTCACCTTTAGCATCTATGCCATGAGCTGGGTCCGCCAGGCTCCAGGGAAGGGGCTGGAGTGGGTCTCAGTTATTAGTGGTAGTGGTGGTAGCTCATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTACGAAAGATCGGGTGAAAGCTCATGATGGTTTTGATATCTGGGGCCAAGGGACAATGGTCACCGTCTCTTCA |
| | | | SEQ ID NO: 24565 | SEQ ID NO: 28571 |
| | | AA | DIQMTQSPSSLSASVGDRVTITCRASQSISNYLNWYQQQTGKAPKFLIYAASSLQSGVPSRFSGSGSGTDFTLTISSLQPEDFATYYCQQSYSTPFTFGPGTKVDIK | EVQLLESGGDMVQPGGSLRLSCAASGFTFSIYAMSWVRQAPGKGLEWVSVISGSGGSSFYADSVKGRFTISRDNSKNTLYLQMNSLRAEDTAVYYCTKDRVKAHDGFDIWGQGTMVTVSS |
| | | | SEQ ID NO: 24566 | SEQ ID NO: 28572 |

FIGURE 50

| | | | | |
|---|---|---|---|---|
| **iPS:392986** | **21-225_31B8** | NA | GACATCCAGATGATCCAGTCTCCATCCTCCCTGTCTGCATCTGTAGGAGACAGAGTCACCATCACTTGCCGGGCAAGTCAGGACATTAGAAGTGATTTAGGCTGGTATCAGCAGAAACCAGGGAAAGCCCCTAAGCGCCTGATCTATGGTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATATTATTTACCCTCCTACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCCGCTATTAGTGGTCGTGGTGGTAGCACATTCCACGCAGACTCCGTGAAGGGCCGGTTCACCATCTCCAGAGACAATTCCAAGAACACGCTGTATCTACAACTGAACAGCCTGAGAGCCGATGACACGGCCGTATATTACTGTGTGAAAGGGGAGCTACTAGAGGACTACTACTTCTACGGTATGGACGTCTGGGGCCAAGGGACCACGGTCACCGTCTCCTCA |
| | | | SEQ ID NO: 24567 | SEQ ID NO: 28573 |
| | | AA | DIQMIQSPSSLSASVGDRVTITCRASQDIRSDLGWYQQKPGKAPKRLIYGASSLQSGVPSRFSGSGSGTEFTLTISSLQPEDFATYYCLQHIIYPPTFGGGTKVEIK | EVQLLESGGGLVQPGGSLRLSCAASGFTFSSYAMSWVRQAPGKGLEWVSAISGRGGSTFHADSVKGRFTISRDNSKNTLYLQLNSLRADDTAVYYCVKGELLEDYYFYGMDVWGQGTTVTVSS |
| | | | SEQ ID NO: 24568 | SEQ ID NO: 28574 |
| **iPS:392988** | **21-225_25E6** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCGGAAACCAGGGAAAGCCCCTAAGCGCCTGATCTATGCTGCATCCAGTTTGCAAAGTGGGGTCCCATCAAGGTTCCGCGGCAGTGGATCTGGGACAGAATTCACTCTCACAATCAACAGTCTGCAGCCTGAAGATTTTGCAACCTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGAAAATAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTCTGTATTACTGTGCGACAGAACTGGGGATGACGGGTGACTCCTGGGGCCAGGGAACCCTGGTCACCGTCTCCTCA |
| | | | SEQ ID NO: 24569 | SEQ ID NO: 28575 |

FIGURE 50

| | | | | |
|---|---|---|---|---|
| | | AA | DIQMTQSPSSLSASVGDRVTITCRASQGIRNDLGWYQRKPGKAPKRLIYAASSLQSGVPSRFRGSGSGTEFTLTINSLQPEDFATYYCLQHNSYPLTFGGGTKVEIK | QVQLVESGGGVVQPGRSLRLSCAASGFTFSDYGMHWVRQAPGKGLEWVAVIWYDENNKYYADSVKGRFTISRDNSKNTLYLQMNSLRAEDTALYYCATELGMTGDSWGQGTLVTVSS |
| | | | SEQ ID NO: 24570 | SEQ ID NO: 28576 |
| **iPS:392990** | **21-225_25H10** | NA | GACATCCAGATGACCCAGTCTCCATCCTCCCTGTCTGCATCTGTAGGAGACAGAGTCACCATCACTTGCCGGGCAAGTCGGGGCATTAGAAATGATTTAGGCTGGTATCAGCAGAAACCAGGGAAAGCCCCTAAGCGCCTGATCTATGCTGCATTCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGAAAGTAATAAATACTATGCAGACTCCATGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGATGACGGGTGACTACTGGGGCCAGGGAACCCTGGTCACCGTCTCCTCA |
| | | | SEQ ID NO: 24571 | SEQ ID NO: 28577 |
| | | AA | DIQMTQSPSSLSASVGDRVTITCRASRGIRNDLGWYQQKPGKAPKRLIYAAFSLQSGVPSRFSGSGSGTEFTLTISSLQPEDFATYYCLQHNSYPLTFGGGTKVEIK | QVQLVESGGGVVQPGRSLRLSCAASGFTFSDYGMHWVRQAPGKGLEWVAVIWYDESNKYYADSMKGRFTISRDNSKNTLYLQMNSLRAEDTAVYYCARELGMTGDYWGQGTLVTVSS |
| | | | SEQ ID NO: 24572 | SEQ ID NO: 28578 |

FIGURE 50

| | | | | |
|---|---|---|---|---|
| iPS:392992 | 21-225_26C4 | NA | GACATCGTGATGACCCAGTCTCCAGACTCCCT GGCTGTGTCTCTGGGCGAGAGGGCCACCATCA ACTGCAAGTCCAGCCAGAGTGTTTTATACCGC TCCAACAATTACAACTACTTAGCTTGGTACCA GCACAAACCAGGACAGCCTCCTAAGCTGCTCA TTTACTGGGCATCTACCCGGGAATCCGGGGTC CCTGACCGCTTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCTCCGACGTTCGGCCAAGG GACCAAGGTGGAATTCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCTCTGGACAAGGGC TTGAGTGGATGGGATGGATGAACCCTAACAGTG GTAACACAGGCTATGCACAGAGATTCCAGGGCA GAGTCACCATGACCAGGAACACCTCCATAAACA CAGCCTACATGGAGCTGAGCAGCCTGAGATCTG AGGACACGGCCGTATATTACTGTGCGAGTAGCA GTGGCTGGTACTACTTTGACTACTGGGGCCAGGG AACCCTGGTCACCGTCTCCTCA |
| | | | SEQ ID NO: 24573 | SEQ ID NO: 28579 |
| | | AA | DIVMTQSPDSLAVSLGERATINCKSSQSVLYRSN NYNYLAWYQHKPGQPPKLLIYWASTRESGVPD RFSGSGSGTDFTLTISSLQAEDVAVYYCQQYYST PPTFGQGTKVEFK | QVQLVQSGAEVKKPGASVKVSCKASGYTFTNYDIN WVRQASGQGLEWMGWMNPNSGNTGYAQRFQGR VTMTRNTSINTAYMELSSLRSEDTAVYYCASSSGW YYFDYWGQGTLVTVSS |
| | | | SEQ ID NO: 24574 | SEQ ID NO: 28580 |
| iPS:392994 | 21-225_26G11 | NA | GATATTGTGATGACCCAGACTCCACTCTCTCT GTCCGTCACCCCTGGACAGCCGGCCTCCATCT CCTGCAAGTCTAGTCAGACCCTCCTGCATGGT GAAGGAAAGACCTATTTGTATTGGTACCTGCA GAAGCCAGGCCAGCCTCCACACCTCCTGATCT ATGAAGTTTCCAACCGGTTCTCTGGAGTGCCA GATAGGTTCAGTGGCAGCGGGTCAGGGACAG ATTTCACACTGAAAATCAGCCGGGTGGAGGCT GAGGATGTTGGGATTTATTACTGCATGCAAAG TATAAAGCTTCCGCTCACTTTCGGCGGAGGGA CCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAAGGTATAG CAACAGCTGGTCAGGGGGTATGGACGTCTGGGG CCAAGGGACCACGGTCACCGTCTCCTCA |
| | | | SEQ ID NO: 24575 | SEQ ID NO: 28581 |

FIGURE 50

| | | | | |
|---|---|---|---|---|
| | | AA | DIVMTQTPLSLSVTPGQPASISCKSSQTLLHGEGKTYLYWYLQKPGQPPHLLIYEVSNRFSGVPDRFSGSGSGTDFTLKISRVEAEDVGIYYCMQSIKLPLTFGGGTKVEIK | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARRYSNSWSGGMDVWGQGTTVTVSS |
| | | | SEQ ID NO: 24576 | SEQ ID NO: 28582 |
| iPS:392996 | 21-225_28B1 | NA | GACATCCAGATGACCCAGTCTCCATCTTCCGTGTCTGCATCTGTAGGAGACAGAGTCACCATCACTTGTCGGGCGAGTCAGGCTATCAATGACTGGTTAGCCTGGTATCAGCAGAAACCAGGGAAAGCCCCTAAGCTCCTGATCTATGCTGCATCCAGTTTCCAAAGTGGGGTCCCATCAAGGTTCAGCGGCAGTGGATCTGGGACAGATTTCACTCTCACCATCACCAGCCTGCAGCCTGAAGATTTTGCAACTTACTATTGTCAACAGGCTAGCAGTTTCCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGATTGTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTAGTGGAGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATTGGGGCGTATAGCAGTGACTGGTCCTTACTTTGACTACTGGGGCCAGGGAACCCTGGTCACCGTCTCCTCA |
| | | | SEQ ID NO: 24577 | SEQ ID NO: 28583 |
| | | AA | DIQMTQSPSSVSASVGDRVTITCRASQAINDWLAWYQQKPGKAPKLLIYAASSFQSGVPSRFSGSGSGTDFTLTITSLQPEDFATYYCQQASSFPFTFGPGTKVDIK | EVQLLESGGGLVQPGGSLRLSCAASGFTFSSYAMSWVRQAPGKGLEWVSAISGSGGNTFYADSVKGRFTISRDNSKNTLYLQMNSLRAEDTAVYYCAKLGRIAVTGPYFDYWGQGTLVTVSS |
| | | | SEQ ID NO: 24578 | SEQ ID NO: 28584 |

FIGURE 50

| | | | | |
|---|---|---|---|---|
| **iPS:392998** | **21-225_28A9** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCTTCTAGTTTGCAAAATGGAGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATCGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATACACTGGGTCCGCCAGGCTCCAGGCAAGGGGCTGGAGTGGGTGGCAGTTATATGGTTTGATGGAAGTAATAAATACTATGCAGACTCCGTGAAGGGCCGATTCACCATCTCCAGAGACAATTCCAAGAACACGCTGTTTCTGCAAATGAACAGCCTGAGAGTCGAGGACACGGCTGTGTATTACTGTGCGAGAGAAATTGGCTGGTTAGATGACTACTGGGGCCAGGGAACCCTGGTCACCGTCTCCTCA |
| | | | SEQ ID NO: 24579 | SEQ ID NO: 28585 |
| | | AA | DIQMTQSPSSLSASVGDRVTITCRASQGIRNDLGWYQQKPGKAPKRLIYAASSLQNGVPSRFSGSGSGTEFTLTISSLQPEDFATYYCLQHNRYPFTFGPGTKVDIK | QVQLVESGGGVVQPGRSLRLSCAASGFTFSSYGIHWVRQAPGKGLEWVAVIWFDGSNKYYADSVKGRFTISRDNSKNTLFLQMNSLRVEDTAVYYCAREIGWLDDYWGQGTLVTVSS |
| | | | SEQ ID NO: 24580 | SEQ ID NO: 28586 |
| **iPS:393000** | **21-225_29D7** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAACAGAAACCAGGGAAAGCCCCTAAGCGCCTGATCTATGCTGCATCCAGTTTACAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | CAGGTGCAACTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGAAAGTAATAAATACTATGGAGACTCCGTGAAGGGCCGATTCACCATCTCCAGAGACAATTCCAAGAACACGCTGTATCTGCAAATGAACAGCCTGAGAGGCGAGGACACGGCTCTGTATTACTGTGCGAGAGAACTGGGGTTCCTCTCTGACTACTGGGGCCAGGGAACCCTGGTCACCGTCTCCTCA |
| | | | SEQ ID NO: 24581 | SEQ ID NO: 28587 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPFTFGPGTKVDIK | QVQLVESGGGVVQPGRSLRLSCAASGFTFSSYGMHWVRQAPGKGLEWVAVIWYDESNKYYGDSVKGRFTISRDNSKNTLYLQMNSLRGEDTALYYCARELGFLSDYWGQGTLVTVSS |
| | | | SEQ ID NO: 24582 | SEQ ID NO: 28588 |
| **iPS:393002** | **21-225_30G1** | NA | GACATCCAGATGACCCAGTCTCCATCCTCCCTGTCTGCATCTGTAGGAGACAGAGTCACCATCACTTGCCGGGCAAGTCAGAACATTTACAGCTATTTAAATTGGTATCAGCAGAAACCAGGGAAAGACCCTAAGCTCCTGATCTATGCTGCATCCAGTTTGCATAGTGGGGTCCCGTCACGGTTCAGTGGCAGTGGTTCTGGGACAGATTTCACTCTCACCATCAGCAGTCTGCAACCTGAAGATTTTGCAACTTACTACTGTCAACAGAGTTACAGTACTCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTTACTATGGCATGCACTGGGTCCGCCAGGCTCCAGGCAAGGGGCTGGAGTGGGTGGCAGTTATATGGCATGATGGAAGTAATAAATACTATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAATAGCAGTTCGTACTACTTTGACTACTGGGGCCAGGGAACCCTGGTCACCGTCTCCTCA |
| | | | SEQ ID NO: 24583 | SEQ ID NO: 28589 |
| | | AA | DIQMTQSPSSLSASVGDRVTITCRASQNIYSYLNWYQQKPGKDPKLLIYAASSLHSGVPSRFSGSGSGTDFTLTISSLQPEDFATYYCQQSYSTPLTFGGGTKVEIK | QVQLVESGGGVVQPGRSLRLSCAASGFTFSYYGMHWVRQAPGKGLEWVAVIWHDGSNKYYVDSVKGRFTISRDNSKNTLYLQMNSLRAEDTAVYYCARENSSSYYFDYWGQGTLVTVSS |
| | | | SEQ ID NO: 24584 | SEQ ID NO: 28590 |

FIGURE 50

| | | | | |
|---|---|---|---|---|
| **iPS:393004** | **21-225_30G11** | NA | GACATCCAGATGACCCAGTCTCCATCCTCCCTGTCTGCATCTGTAGGAGACAGAGTCACCATCACTTGCCGGGCAAGTCAGGACATTAGAAGTGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCGGCAGCCTGCAGCCTGAAGATTTTGCAACTTATTTCTGTCTACAGCATACTATTTACCCTCCTACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCCGCTATTAGTGGTCGTGGTGGTAGCACATTCAACGCAGACTCCGTGAAGGGCCGGTTCACCATCTCCAGAGACAATTCCAAGACCACGCTGTATCTGCAAATGAACAGCCTGAGAGCCGAGGACACGGCCGTATATTACTGTGTGAAAGGGGAGCTACTAGAGGACTACTACTTCTACGGTATGGACGTCTGGGGCCAAGGGACCACGGTCACCGTCTCCTCA |
| | | | SEQ ID NO: 24585 | SEQ ID NO: 28591 |
| | | AA | DIQMTQSPSSLSASVGDRVTITCRASQDIRSDLGWYQQKPGKAPKRLIYAASSLQSGVPSRFSGSGSGTEFTLTIGSLQPEDFATYFCLQHTIYPPTFGGGTKVEIK | EVQLLESGGGLVQPGGSLRLSCAASGFTFSSYAMSWVRQAPGKGLEWVSAISGRGGSTFNADSVKGRFTISRDNSKTTLYLQMNSLRAEDTAVYYCVKGELLEDYYFYGMDVWGQGTTVTVSS |
| | | | SEQ ID NO: 24586 | SEQ ID NO: 28592 |
| **iPS:393006** | **21-225_31G9** | NA | GACATCCAGATGACCCAGTCTCCATCCTCCCTGTCTGCATCTATAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATCCTGCATCCAGTTTGCAAAGTGGGGTCCCATCAAGGTTCAGCGGCAGTGGATCTGGGACAGAGTTCACTCTCACAATCAGCAGCCTGCAGCCTGAAGATTTTGCAACTTATTACTGTCTACAGGATAATAGTTACCCTTTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCAATTAGTAGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGATCGAGGCAGCAGCTGGGGCCAGGGAACCCTGGTCACCGTCTCCTCA |
| | | | SEQ ID NO: 24587 | SEQ ID NO: 28593 |

FIGURE 50

| | | | | |
|---|---|---|---|---|
| | | AA | DIQMTQSPSSLSASIGDRVTITCRASQGIRNDLGWYQQKPGKAPKRLIYPASSLQSGVPSRFSGSGSGTEFTLTISSLQPEDFATYYCLQDNSYPFTFGPGTKVDIK | EVQLVESGGGLVKPGGSLRLSCAASGFTFSSYSMNWVRQAPGKGLEWVSSISSSSSYIYYADSVKGRFTISRDNAKNSLYLQMNSLRAEDTAVYYCARDRGSSWGQGTLVTVSS |
| | | | SEQ ID NO: 24588 | SEQ ID NO: 28594 |
| **iPS:393010** | **21-225_25E11** | NA | GACATCCAGATGACCCAGTCTCCATCTTCCGTGTCTGCATCTGTAGGAGACAGAGTCACCATCACTTGTCGGGCGAGTCAGGGTATTAGCAACTGGTTAGCCTGGTATCAGCAGAAACCAGGGAAAGCCCCTAAGCTCCTGATCTATACTGCATCCAGTTTGCAAGGTGGGGTCCCATCAAGGTTCAGCGGCAGTGGATCTGGGACAGATTTCACTATCAGCATCAGCAGCCTGCAGCCTGAAGATTTTGCAACTTACTATTGTCAACAGGCTAACAGTTTCCCAATCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTATTAGTGGTGGTGGTGGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAACGTGGATATAGTGGCTACGAGGACCTCCTCTACTTTGACTGCTGGGGCCAGGGAACCCTGGTCACCGTCTCCTCA |
| | | | SEQ ID NO: 24589 | SEQ ID NO: 28595 |
| | | AA | DIQMTQSPSSVSASVGDRVTITCRASQGISNWLAWYQQKPGKAPKLLIYTASSLQGGVPSRFSGSGSGTDFTISISSLQPEDFATYYCQQANSFPITFGPGTKVDIK | EVQLLESGGGLVQPGGSLRLSCAASGFTFSSYAMSWVRQAPGKGLEWVSVISGGGGSTYYADSVKGRFTISRDNSKNTLYLQMNSLRAEDTAVYYCAKRGYSGYEDLLYFDCWGQGTLVTVSS |
| | | | SEQ ID NO: 24590 | SEQ ID NO: 28596 |

FIGURE 50

| | | | | |
|---|---|---|---|---|
| **iPS:393012** | **21-225_26G7** | NA | GATATCTTGATGACCCAGACTCCACTCTCTCTGTCCGTCACCCCTGGACAGCCGGCCTCCATCTCCTGCAAGTCTAGTCAGAGCCTCCTGCATAGTGAGGGAAAGACCTATTTGTATTGGTACCTGCAGAAGCCAGGCCAGCCTCCACAGTTCCTGATCTATGAAGTTTCCCACCGGCTCTCTGGAGTGCCAGATAGGTTCAGTGGCAGCGGGTCAGGGACAGATTTCACACTGAAAATCAGCCGGGTTGAGGCTGAGGATGTTGGGGTTTATTACTGCATGCAAAGTATACAGCTTCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTGTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATATTATGCAGACTCCGTGAAGGGCCGATTCACCATCTCCAGAGACAATTCCAATAACACGCTGTATCTGCAAATGAACAGCCTGAGAGCCGAGGACACGGCTGTGTATTACTGTGCGAGAAGGTATAGCAGCAGCTGGTCAGGGGGTATGGACGTCTGGGGCCAAGGGACCACGGTCACCGTCTCCTCA |
| | | | SEQ ID NO: 24591 | SEQ ID NO: 28597 |
| | | AA | DILMTQTPLSLSVTPGQPASISCKSSQSLLHSEGKTYLYWYLQKPGQPPQFLIYEVSHRLSGVPDRFSGSGSGTDFTLKISRVEAEDVGVYYCMQSIQLPLTFGGGTKVEIK | QVQLVESGGGVVQPGRSLRLSCAASGFTFSSYGMHWVRQAPGKGLEWVAVIWYDGSNKYYADSVKGRFTISRDNSNNTLYLQMNSLRAEDTAVYYCARRYSSSWSGGMDVWGQGTTVTVSS |
| | | | SEQ ID NO: 24592 | SEQ ID NO: 28598 |
| **iPS:393014** | **21-225_26D12** | NA | GACATCCAGATGACCCAGTCTCCCTCTTCCGTGTCTGCATCTGTAGGAGACAGAGTCACCATCACTTGTCGGGCGAGTCAGGGTATTAGTAGTTGGTTAGCCTGGTATCAGCAGAAACCAGGGAAAGCCCCTAAGCTCCTGATCTATGGTGCATCCAGTTTGCAAAGTGGGGTCCCATCAAGGTTCAGCGCCAGTGGATCTGGGACAGATTTCACTCTCACCATCAGCAGCCTGCAGCCTGAAGATTTTGCAACTTACTGTTGTCAACAGTCTGACAGTTTCCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGCAGTTATGGCATGCACTGGGTCCGCCAGGCTCCAGGCAAGGGGCTGGAGTGGGTGGCAGTTATATGGTATGATGGAAGTAATGAATACTATGCAGACTCCGTGAAGGGCCGATTCACCATCTCCAGAGACAATTCCAAGAACACGCTATATCTACAAATGAACAGCCTGAGAGCCGCGGACACGGCTGTGTATTACTGTGCGAGAGATTCCTCCCCCTACGGTATGGACGTCTGGGGCCAAGGGACCACGGTCACCGTCTCCTCA |
| | | | SEQ ID NO: 24593 | SEQ ID NO: 28599 |

FIGURE 50

| | | | | |
|---|---|---|---|---|
| | | AA | DIQMTQSPSSVSASVGDRVTITCRASQGISSWLAWYQQKPGKAPKLLIYGASSLQSGVPSRFSASGSGTDFTLTISSLQPEDFATYCCQQSDSFPRTFGQGTKVEIK | QVQLVESGGGVVQPGRSLRLSCAASGFTFSSYGMHWVRQAPGKGLEWVAVIWYDGSNEYYADSVKGRFTISRDNSKNTLYLQMNSLRAADTAVYYCARDSSPYGMDVWGQGTTVTVSS |
| | | | SEQ ID NO: 24594 | SEQ ID NO: 28600 |
| iPS:393016 | 21-225_28F11 | NA | GACATCCAGATGACCCAGTCTCCATCTTCCGTGTCTGCATCTGTAGGAGACAGAGTCACCATCACTTGTCGGGCGAGTCAGGGTATTAGCAACTGGTTAGCCTGGTATCAGCAGAAACCAGGGAAAGCCCCTAAGCTCCTGATCTCTGCTGCATCCAATTTGCAAAGTGGGGTCCCATCAAGGTTCAGAGGCAGTGGATCTGGGACAGATTTCACTCTCACCATCACCAGCCTGCAGCCTGAAGATTTTGCAACTTACTGTTGTCAACAGGCTAACAGTCTCCCATTCACTTTCGGCCCTGGGACCAAAGTGGATATCAAA | GAGGTGCAGCTGTTGGAGTCTGGGGGAGGCTTGGTACAGCCGGGGGGGTCCCTGAGACTCTCCTGTGCAGCCTCTGGATTCATCTTTAGCAGCTATGCCATGAGCTGGGTCCGCCAGGCTCCAGGGAAGGGGCTGGAGTGGGTCTCAGTTACTAGTGGTAGTGGTGGTACCACATTCTACGCAGACTCCGTGAAGGGCCGGTTCACCATCTCCAGAGACAATTCCAAGAACACGCTTTATCTGCAAATGAACAGCCTGAGAGCCGAGGACACGGCCGTTTATTACTGTGCGAAACGGACCCAGTTTGATGATTTTGATATCTGGGGCCAAGGGACAATGGTCACCGTCTCTTCA |
| | | | SEQ ID NO: 24595 | SEQ ID NO: 28601 |
| | | AA | DIQMTQSPSSVSASVGDRVTITCRASQGISNWLAWYQQKPGKAPKLLISAASNLQSGVPSRFRGSGSGTDFTLTITSLQPEDFATYCCQQANSLPFTFGPGTKVDIK | EVQLLESGGGLVQPGGSLRLSCAASGFIFSSYAMSWVRQAPGKGLEWVSVTSGSGGTTFYADSVKGRFTISRDNSKNTLYLQMNSLRAEDTAVYYCAKRTQFDDFDIWGQGTMVTVSS |
| | | | SEQ ID NO: 24596 | SEQ ID NO: 28602 |

FIGURE 50

| | | | | |
|---|---|---|---|---|
| iPS:393018 | 21-225_29B8 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTAGATCTGGGACAGAATTCACTCTCACAATCAGCAGTCTGCAGCCTGAAGATTTTGCAACCTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGAAAATAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGATGACGGGTGACTCCTGGGGCCAGGGAACCCTGGTCACCGTCTCCTCA |
| | | | SEQ ID NO: 24597 | SEQ ID NO: 28603 |
| | | AA | DIQMTQSPSSLSASVGDRVTITCRASQGIRNDLGWYQQKPGKAPKRLIYAASSLQSGVPSRFSGSRSGTEFTLTISSLQPEDFATYYCLQHNSYPLTFGGGTKVEIK | QVQLVESGGGVVQPGRSLRLSCAASGFTFSDYGMHWVRQAPGKGLEWVAVIWYDENNKYYADSVKGRFTISRDNSKNTLYLQMNSLRAEDTAVYYCARELGMTGDSWGQGTLVTVSS |
| | | | SEQ ID NO: 24598 | SEQ ID NO: 28604 |
| iPS:393020 | 21-225_30E2 | NA | GACATCCAGATGACCCAGTCTCCACATTCCCTGTCTGCATCTGTAGGAGACAGAGTCACCATCACTTGCCAGGCGAGTCAGTACATTAGCAACTATTTAAATTGGTATCAGCAGAAATCAGGAAAAGCCCCTAAGCTCCTGATCTACGATGGATCCAGTTTGGAAACAGGGGTCCCATCAAGGTTCAGTGGAAGTGGATCTGGGACAGATTTTACTTTCACCATCAGCAGCCTGCAGCCTGAAGATCTTGCAACATATTACTGTCAACAGTATGATAATCTCCCGATCACCTTCGGCCAAGGGACACGACTGGAGATTAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAGTGGGTGGCAGTTATATCATATGGTGGAAGTAATAAATTCTATGCAGTCTCCGTGAAGGGCCGATTCAACATCTCCAGAGACAATTCCAAGAACACGCTGTATCTGCAAATGAACAGCCTGAGAGCTGAGGACACGGCTGTGTATTACTGTGTGAGAAGGGGGTATAGCAGTGGAGGCTACGGTATGGACGTCTGGGGCCAAGGGACCACGGTCACCGTCTCCTCA |
| | | | SEQ ID NO: 24599 | SEQ ID NO: 28605 |

FIGURE 50

| | | | | |
|---|---|---|---|---|
| | | AA | DIQMTQSPHSLSASVGDRVTITCQASQYISNYLNWYQQKSGKAPKLLIYDGSSLETGVPSRFSGSGSGTDFTFTISSLQPEDLATYYCQQYDNLPITFGQGTRLEIK | QVQLVESGGGVVQPGRSLRLSCAASGFTFSSYGMHWVRQAPGKGLEWVAVISYGGSNKFYAVSVKGRFNISRDNSKNTLYLQMNSLRAEDTAVYYCVRRGYSSGGYGMDVWGQGTTVTVSS |
| | | | SEQ ID NO: 24600 | SEQ ID NO: 28606 |
| iPS:393022 | 21-225_30H11 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGGTCTATCCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGGATAATAGTCATCCATTCACTTTCGGCCCTGGGACCAAAGTGGATATCACA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGATAGGGGGAGCCTCTGGGGCCAGGGAACCCTGGTCACCGTCTCCTCA |
| | | | SEQ ID NO: 24601 | SEQ ID NO: 28607 |
| | | AA | DIQMTQSPSSLSASVGDRVTITCRASQGIRNDLGWYQQKPGKAPKRLVYPASSLQSGVPSRFSGSGSGTEFTLTISSLQPEDFATYYCLQDNSHPFTFGPGTKVDIT | EVQLVESGGGLVKPGGSLRLSCAASGFTFSSYSMNWVRQAPGKGLEWVSSISGSSSYIYYADSVKGRFTISRDNAKNSLYLQMNSLRAEDTAVYYCARDRGSLWGQGTLVTVSS |
| | | | SEQ ID NO: 24602 | SEQ ID NO: 28608 |

FIGURE 50

| | | | | |
|---|---|---|---|---|
| **iPS:393024** | **21-225_31H9** | NA | GACATCCAGATGACCCAGTCTCCATCTTCCGTGTCTGCATCTGTAGGAGACAGAGTCACCATCACTTGTCGGGCGAGTCAGGGTATTACCAGCTGGTTAACTTGGTATCAGCAGAGACCAGGGAAAGCCCCTAAACTCCTGATCTATGATACATCCAGTTTGCAAAGTGGGGTCCCATCAAGGTTCAGCGGCAGTGGATCTGGGACAGATTTCATTTTCACCATCAGCAGCCTGCAGCCTGAAGATTTTGCCACTTATTATTGTCAACAGGGTAACAGTTTCCCATTCACTTTCGGCCAAGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTGTGCCATGAACTGGGTCCGCCAGGCTCCAGGGAAGGGGCTGGAGTGGGTCTCAGCTATTAGTGGTAGTGGTGGTAGCTCATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAACGGACTCCCTATGATGTCTTTGATATCTGGGGCCAAGGGACAATGGTCACCGTCTCTTCA |
| | | | SEQ ID NO: 24603 | SEQ ID NO: 28609 |
| | | AA | DIQMTQSPSSVSASVGDRVTITCRASQGITSWLTWYQQRPGKAPKLLIYDTSSLQSGVPSRFSGSGSGTDFIFTISSLQPEDFATYYCQQGNSFPFTFGQGTKVDIK | EVQLLESGGGLVQPGGSLRLSCAASGFTFSSCAMNWVRQAPGKGLEWVSAISGSGGSSFYADSVKGRFTISRDNSKNTLYLQMNSLRAEDTAVYYCAKRTPYDVFDIWGQGTMVTVSS |
| | | | SEQ ID NO: 24604 | SEQ ID NO: 28610 |
| **iPS:393026** | **21-225_32B6** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATATTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGACAAGGGGCTGGAGTGGGTGGCAGTTATATGGTATGATGAAAATACTAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGTGGGGGGACTACTGGGGCCAGGGAACCCTGGTCACCGTCTCCTCA |
| | | | SEQ ID NO: 24605 | SEQ ID NO: 28611 |

FIGURE 50

| | | | | |
|---|---|---|---|---|
| | | AA | DIQMTQSPSSLSASVGDRVTITCRASQGIRNDLGWYQQKPGKAPKRLIYIASSLQSGVPSRFSGSGSGTEFTLTISSLQPEDFATYYCLQHNSYPWTFGQGTKVEIK | QVQLVESGGGVVQPGRSLRLSCAASGFTFSDYGMHWVRQAPDKGLEWVAVIWYDENTKYYADSVKGRFTISRDNSKNTLYLQMNSLRAEDTAVYYCAREWGDYWGQGTLVTVSS |
| | | | SEQ ID NO: 24606 | SEQ ID NO: 28612 |
| iPS:393028 | 21-225_25D7 | NA | GACATCCAGATGACCCAGTCTCCATCTTCCGTGTCTGCATCTGTAGGAGACAGAGTCACCTTCACTTGTCGGGCGAGTCAGGATATTTTCGACTGGTTAGCCTGGTATCAGCAGAAACCCGGGACAGCCCCTAAGCTCCTGATCTATGCTGCATCCAGTTTGCAAAGTGGGGTCCCATCAAGGTTCAGCGGCAGTGGATCTGGGACAAATTTCACTCTCACCGTCAGCGGCCTGCAGCCTGAAGATTTTGCTACTTACTATTGTCAACAGGCTTACAGTTTCCCGTGGACGTTCGGCCAAGGGACCAAAGTGGAAATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGACTCCAGGGCAGGGGCTGGAGTGGGTCTCAGCTATTAGTGGTAGAGGTGGTACCACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAAGACGGGTACGGTGGTAACTCCTTCTTTGACTACTGGGGCCAGGGAACCCTGGTCACCGTCTCCTCA |
| | | | SEQ ID NO: 24607 | SEQ ID NO: 28613 |
| | | AA | DIQMTQSPSSVSASVGDRVTFTCRASQDIFDWLAWYQQKPGTAPKLLIYAASSLQSGVPSRFSGSGSGTNFTLTVSGLQPEDFATYYCQQAYSFPWTFGQGTKVEIK | EVQLLESGGGLVQPGGSLRLSCAASGFTFSSYAMSWVRQTPGQGLEWVSAISGRGGTTFYADSVKGRFTISRDNSKNTLYLQMNSLRAEDTAVYYCAKDGYGGNSFFDYWGQGTLVTVSS |
| | | | SEQ ID NO: 24608 | SEQ ID NO: 28614 |

FIGURE 50

| | | | | |
|---|---|---|---|---|
| **iPS:393030** | **21-225_25H11** | NA | GACATCCAGATGACCCAGTCTCCATCCTCCCTGTCTGCGTCTGTAGGAGACAGAGTCACCATCACTTGCCGGGCAAGTCAGGGCATTAGAACTGATTTAGGCTGGTATCAGCAGAAACCAGGGAAAGCCCCTAAGCGCCTGATCTATGCTGCATCCAGTTTACAAAGTGGGGTCCCATCAAGGTTCAGCGGCAGTGGATCTGGGACAGAATTCACTCTCACAATCAGTAGTCTGCAGCCTGAAGATTTTGCAACCTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGAAAATAATGAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGATGACGGGTGACTCCTGGGGCCAGGGAACCCTGGTCACCGTCTCCTCA |
| | | | SEQ ID NO: 24609 | SEQ ID NO: 28615 |
| | | AA | DIQMTQSPSSLSASVGDRVTITCRASQGIRTDLGWYQQKPGKAPKRLIYAASSLQSGVPSRFSGSGSGTEFTLTISSLQPEDFATYYCLQHNSYPLTFGGGTKVEIK | QVQLVESGGGVVQPGRSLRLSCAASGFTFSDYGMHWVRQAPGKGLEWVAVIWYDENNEYYADSVKGRFTISRDNSKNTLYLQMNSLRAEDTAVYYCARELGMTGDSWGQGTLVTVSS |
| | | | SEQ ID NO: 24610 | SEQ ID NO: 28616 |
| **iPS:393032** | **21-225_26F8** | NA | GATATTGTGATGACCCAGACTCCACTCTCTCTGTCCGTCACCCCTGGACAGCCGGCCTCCATCTCCTGCAAGTCTAGTCAGAGCCTCCTGCATGGTGATGGAAAGACCTATTTGTATTGGTACCTGCAGAAGCCAGGCCAGCCTCCACAGCTCCTGATCTATGAAGTTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGGTTTATTACTGCATGCAAAGTATACAGCTTCCGTGGACGTTCGGCCAGGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGGCTATGGCATGCACTGGGTCCGCCAGGCTCCAGGCAAGGGGCTGGAGTGGGTGGCAATTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGAGGTACGATTTTTGGAGTGGTTGTATGGACGTCTGGGGCCAAGGGACCACGGTCACCGTCTCCTCA |
| | | | SEQ ID NO: 24611 | SEQ ID NO: 28617 |

FIGURE 50

| | | | | |
|---|---|---|---|---|
| | | AA | DIVMTQTPLSLSVTPGQPASISCKSSQSLLHGDGKTYLYWYLQKPGQPPQLLIYEVSNRFSGVPDRFSGSGSGTDFTLKISRVEAEDVGVYYCMQSIQLPWTFGQGTKVEIK | QVQLVESGGGVVQPGRSLRLSCAASGFTFSGYGMHWVRQAPGKGLEWVAIIWYDGSNKYYADSVKGRFTISRDNSKNTLYLQMNSLRAEDTAVYYCARERYDFWSGCMDVWGQGTTVTVSS |
| | | | SEQ ID NO: 24612 | SEQ ID NO: 28618 |
| iPS:393034 | 21-225_27F2 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGTTGCATCCAGTTTGCAAAGTGGGGTCCCATCAAGGTTCAGCGGCAGTGGATCTGGGACAGAATTCACTCTCACAATCAGCAGTCTGCAGCCTGAAGATTTTGCAACCTATTACTGTCTACAGCATAATAGTTACCCGCTCACTTTCGGCGGAGGGACCAAGGTGGAGATCAAA | CAGGAGCAGCTGGTGGAGTCTGGGGGAGGCGTGGTCCAGCCTGGGAGGTCCCTGAGACTCTCCTGTGCAGCGTCTGGATTCATCTTCAGTGACTATGGCATGCACTGGGTCCGCCAGGCTCCAGGCAAGGGGCTGGAGTGGGTGGCAGTTATATGGTATGATGAAAATAATAAATACTATGTAGACTCCGTGAGGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGATGACGGGTGACTCCTGGGGCCAGGGAACCCTGGTCACCGTCTCCTCA |
| | | | SEQ ID NO: 24613 | SEQ ID NO: 28619 |
| | | AA | DIQMTQSPSSLSASVGDRVTITCRASQGIRNDLGWYQQKPGKAPKRLIYVASSLQSGVPSRFSGSGSGTEFTLTISSLQPEDFATYYCLQHNSYPLTFGGGTKVEIK | QEQLVESGGGVVQPGRSLRLSCAASGFIFSDYGMHWVRQAPGKGLEWVAVIWYDENNKYYVDSVRGRFTISRDNSKNTLYLQMNSLRAEDTAVYYCARELGMTGDSWGQGTLVTVSS |
| | | | SEQ ID NO: 24614 | SEQ ID NO: 28620 |

FIGURE 50

| | | | | |
|---|---|---|---|---|
| **iPS:393036** | **21-225_28G3** | NA | GAAATTGTGATGACCCAGACTCCACTCTCTCTGTCCGTCACCCCTGGACAGCCGGCCTCCATCTCCTGCAAGTCTAGTCAGAGCCTCCTACATGGTGATGGAAAGACCTATTTGTATTGGTACCTGCAGAAGCCAGGCCAGCCTCCACAGCTCCTGATCTATGAAGTTTCCAACCGGTTCTCTGGAGTGCCAGAAAGGTTCAGTGGTAGCGGGTCAGGGACAGATTTCACACTGAAAATCAGCCGGGTGGAGGCTGAGGATGTTGGGGTTTATTACTGTATGCAAAGTATACAGATTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCATCTTCAGTACCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGATACGATTTTTGGAGTGGTTATTTTGACTACTGGGGCCAGGGAACCCTGGTCACCGTCTCCTCA |
| | | | SEQ ID NO: 24615 | SEQ ID NO: 28621 |
| | | AA | EIVMTQTPLSLSVTPGQPASISCKSSQSLLHGDGKTYLYWYLQKPGQPPQLLIYEVSNRFSGVPERFSGSGSGTDFTLKISRVEAEDVGVYYCMQSIQIPWTFGQGTKVEIK | QVQLVESGGGVVQPGRSLRLSCAASGFIFSTYGMHWVRQAPGKGLEWVAVIWYDGSNKYYADSVKGRFTISRDNSKNTLYLQMNSLRAEDTAVYYCARDRYDFWSGYFDYWGQGTLVTVSS |
| | | | SEQ ID NO: 24616 | SEQ ID NO: 28622 |
| **iPS:393038** | **21-225_29D8** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATACTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGGGACTATGGCATACACTGGGTCCGCCAGGCTCCAGGCAAGGGGCTGGAGTGGGTGGCAGTTATATGGTTTGATGGAACTAATAAATACTATGCAGACTCCGTGAAGGGCCGATTCACCATCTCCAGAGACAATTCCAAGAACACGCTGTATCTGCAAATGAACAGCCTGAGAGCCGAAGACACGGCTGTGTATTACTGTGGGAGAGAAATTGGCTGGTTAGATGACTACTGGGGCCAGGGAACCCTGGTCACCGTCTCCTCA |
| | | | SEQ ID NO: 24617 | SEQ ID NO: 28623 |

FIGURE 50

| | | | | |
|---|---|---|---|---|
| | | AA | DIQMTQSPSSLSASVGDRVTITCRASQGIRNDLGWYQQKPGKAPKRLIYTASSLQSGVPSRFSGSGSGTEFTLTISSLQPEDFATYYCLQHNSYPFTFGPGTKVDIK | QVQLVESGGGVVQPGRSLRLSCAASGFTFRDYGIHWVRQAPGKGLEWVAVIWFDGTNKYYADSVKGRFTISRDNSKNTLYLQMNSLRAEDTAVYYCGREIGWLDDYWGQGTLVTVSS |
| | | | SEQ ID NO: 24618 | SEQ ID NO: 28624 |
| **iPS:393040** | **21-225_30E3** | NA | GACATCCAGATGACCCAGTCTCCATCCTCCCTGTCTGCATCTGTAGGAGACAGAGTCACCATCACTTGCCGGGCAAGTCAGGACATTAGAGATGATTTAGGCTGGTATCAGCAGAAACCAGGGAAAGCCCCTAGGCGCCTGATCTATGCTGCATTCAGCTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATACTAGTTACCCTCCTACTTTCGGCGGAGGGACCAAGGTGGAGATCAAA | GAGGTGCAGGTGTTGGAGTCTGGGGGAGGCTTGGTACAGCCTGGGGGGTCCCTGAGACTCTCCTGTGCAGCCTCTGGATTCACCTTTAGCAGCTATGCCATGAACTGGGTCCGCCAGGCTCCAGGGAAGGGGCTGGAGTGGGTCTCCGCTATTAGTGGTCGTGGTGGTAGCACATTCTACGCAGACTCCGAGAAGGGCCGGTTCACCATCTCCAGAGACAATTCCAAGAACACGCTGTATCTGCAAATGAACAGCCTGAGAGCCGAGGACACGGCCCTATATTACTGTGCGAAAGGGGAGCTATTAGAGGACTACTACTACTACGGAATGGACGTCTGGGGCCAAGGGACCACGGTCACCGTCTCCTCA |
| | | | SEQ ID NO: 24619 | SEQ ID NO: 28625 |
| | | AA | DIQMTQSPSSLSASVGDRVTITCRASQDIRDDLGWYQQKPGKAPRRLIYAAFSLQSGVPSRFSGSGSGTEFTLTISSLQPEDFATYYCLQHTSYPPTFGGGTKVEIK | EVQVLESGGGLVQPGGSLRLSCAASGFTFSSYAMNWVRQAPGKGLEWVSAISGRGGSTFYADSEKGRFTISRDNSKNTLYLQMNSLRAEDTALYYCAKGELLEDYYYYGMDVWGQGTTVTVSS |
| | | | SEQ ID NO: 24620 | SEQ ID NO: 28626 |

FIGURE 50

| | | | | |
|---|---|---|---|---|
| **iPS:393042** | **21-225_31F1** | NA | GACATCCAGATGACCCAGTCTCCATCCTCCCT<br>GTCTGCATCTGTAGGAGACAGAGTCACCATCA<br>CTTGCCGGGCAAGTCAGAGGATTAGCAGCTAT<br>TTAAATTGGTATCAGCAGAAACCAGGGAAAG<br>CCCCTAAGCTCCTGATCTTTGCTGCATCCAGTT<br>CGCAAAGTGGGGTCCCATCAAGGTTCAGTGGC<br>AGTGGATCTGGGACAGATTTCACTCTCACCAT<br>CAGCAGTCTGCAACCTGAAGATTTTGCAACTT<br>ACTACTGTCAACAGAGTTACATTACCCCGCTC<br>ACTTTCGGCGGAGGGACCACGGTGGAGATCA<br>GA | CAGGTGCAGCTGGTGCAGTCTGGGGCTGAGGTG<br>AAGAAGCCTGGGGCCTCAGTGAAGGTCTCCTGC<br>AAGGCTTCTGGATACACCTTCACCGACTACTATA<br>TGCACTGGGTGCGACAGGCCCCTGGACAGGGGC<br>TTGAGTGGATGGGATGGATCAACCCTAACAGTGG<br>TGGCACAAACTATGCACAGAAGTTTCAGGGCAG<br>GGTCACCATGACCAGGGACACGTCCATTATGACA<br>GCCTATATGGAGCTGAGCAGGCTGAGATCTGAC<br>GACACGGCCGTGTATTACTGTGCGAGAGATAGTA<br>GCAATTTCAGCAACTGGTACGATTACTACGGTAT<br>GGACGTCTGGGGCCAAGGGACCACGGTCACCGT<br>CTCCTCA |
| | | | SEQ ID NO: 24621 | SEQ ID NO: 28627 |
| | | AA | DIQMTQSPSSLSASVGDRVTITCRASQRISSYLN<br>WYQQKPGKAPKLLIFAASSSQSGVPSRFSGSGSG<br>TDFTLTISSLQPEDFATYYCQQSYITPLTFGGGTT<br>VEIR | QVQLVQSGAEVKKPGASVKVSCKASGYTFTDYYM<br>HWVRQAPGQGLEWMGWINPNSGGTNYAQKFQGR<br>VTMTRDTSIMTAYMELSRLRSDDTAVYYCARDSSN<br>FSNWYDYYGMDVWGQGTTVTVSS |
| | | | SEQ ID NO: 24622 | SEQ ID NO: 28628 |
| **iPS:393044** | **21-225_25B8** | NA | GAAATAGTGATGACGCAGTCTCCAGCCACCCT<br>GTCTGTGTCTCCAGGGGAAAGAGCCACCCTCT<br>CCTGCAGGGCCAGTCAGAGTGTTAGAAGTAAT<br>TTAGCCTGGTACCAGCAGAAACCTGGCCAGGC<br>TCCCAGGCTCCTCATCTATGGTGCATCCACCA<br>GGGCCACTGGTATCCCAGCCAGGTTCAGTGGC<br>AGTGGGTCTGGGACAGAGTTCACTCTCACCAT<br>CAGCAGCCTGCAGTCTGAAGATTTTGCACTTT<br>ATTACTGTCAGCAGTATAATAATTGGCCTCCG<br>TGGCCGTTCGGCCAAGGGACCAAGGTGGAAA<br>TCAAA | CAGGTTCAGTTGGTGCAGTCTGGAGCTGAGGTGA<br>AGAAGCCTGGGGCCTCAGTGAAGGTCTCCTGCA<br>AGGCTTCTGGTTACACCTTCACCAGCTATGGTAT<br>CAGCTGGGTGCGACAGGCCCCTGGACAAGGGCT<br>TGAGTGGATGGGATGGATCAGCGCTTACAATGGT<br>AACACAACCTATGCACAGAAGCTCCGGGGCAGA<br>GTCACCATGACCACAGACACATCCACGAGCACA<br>GCCTACATGGATCTGAGGAGCCTGAGATCTGACG<br>ACACGGCCGTGTATTACTGTGCGAGAACCGCTGC<br>TGGGTATAGCAGCAGCTGGTTTGACTACTGGGGC<br>CAGGGAACCCTGGTCACCGTCTCCTCA |
| | | | SEQ ID NO: 24623 | SEQ ID NO: 28629 |

FIGURE 50

| | | | | |
|---|---|---|---|---|
| | | AA | EIVMTQSPATLSVSPGERATLSCRASQSVRSNLAWYQQKPGQAPRLLIYGASTRATGIPARFSGSGSGTEFTLTISSLQSEDFALYYCQQYNNWPPWPFGQGTKVEIK | QVQLVQSGAEVKKPGASVKVSCKASGYTFTSYGISWVRQAPGQGLEWMGWISAYNGNTTYAQKLRGRVTMTTDTSTSTAYMDLRSLRSDDTAVYYCARTAAGYSSSWFDYWGQGTLVTVSS |
| | | | SEQ ID NO: 24624 | SEQ ID NO: 28630 |
| iPS:393046 | 21-225_25A12 | NA | GACATCCAGATGACCCAGTCTCCATCCTCCCTGTCTGCATCTGTAGGAGACAGAGTCACCGTCACTTGCCGGGCAAGTCAGGCCATTAGAGATGATTTAGGCTGGTATCAGCAGAGACCAGGGAAAGCCCCTAAGCGCCTGATCTATGCTGCATCCAGTTTGCAAAGTGGGGTCCCATCAAGGTTCAGCGGCAGTGGATCTGGGACAGAATTTATTCTCACAATCAGCAGCCTGCAGCCTGAAGATTTTGCAACTTATTACTGTCTACAGCATTATAATTACCCTCGCAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTGTGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGGAGTATAGCAGTGGCTGGTACGACTACGGTATGGACGTCTGGGGCCAAGGGACCATGGTCACCGTCTCCTCA |
| | | | SEQ ID NO: 24625 | SEQ ID NO: 28631 |
| | | AA | DIQMTQSPSSLSASVGDRVTVTCRASQAIRDDLGWYQQRPGKAPKRLIYAASSLQSGVPSRFSGSGSGTEFILTISSLQPEDFATYYCLQHYNYPRSFGQGTKLEIK | QVQLVESGGGVVQPGRSLRLSCAASGFTFSNCVMHWVRQAPGKGLEWVAVIWYDGSNKYYADSVKGRFTISRDNSKNTLYLQMNSLRAEDTAVYYCAREEYSSGWYDYGMDVWGQGTMVTVSS |
| | | | SEQ ID NO: 24626 | SEQ ID NO: 28632 |

FIGURE 50

| | | | | |
|---|---|---|---|---|
| **iPS:393048** | **21-225_27C3** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGTCTGCAGCCTGAAGATTTTGCAACCTATTACTGTCTACAGCATAATCGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGAAAATAATAAATCCTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGATGACGGGTGACTCCTGGGGCCAGGGAACCCTGGTCACCGTCTCCTCA |
| | | | SEQ ID NO: 24627 | SEQ ID NO: 28633 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNRYPLTFGGGTKVEIK | QVQLVESGGGVVQPGRSLRLSCAASGFTFSDYGMHWVRQAPGKGLEWVAVIWYDENNKSYADSVKGRFTISRDNSKNTLYLQMNSLRAEDTAVYYCARELGMTGDSWGQGTLVTVSS |
| | | | SEQ ID NO: 24628 | SEQ ID NO: 28634 |
| **iPS:393050** | **21-225_28C5** | NA | GAAATAGTGATGACGCAGTCTCCAGCCACCCTGTCTGTGTCTCCAGGGGAAAGAGCCACCCTCTCCTGCAGGGCCAGTCAGAGTGTTAGCAGCAACTTAGCCTGGTACCATCAGAAACCTGGCCAGGCTCCCAGGCTCCTCATCTATGGTGCATCCACCAGGGCCACTGGTATCCCAGCCAGGTTCAGTGGCAGTGGGTCTGGGACAGAGTTCACTCTCACCATCAGCAGCCTGCAGTCTGAAGATTTTGCACTTTATTACTGTCAGCAGTATAATAATTGGCCTCCGTGGCCGTTCGGCCAAGGGACCAAGGTGGAAATCAAA | CAGGTTCAGTTGGTGCAGTCTGGAGCTGAGGTGAAGAAGCCTGGGGCCTCAGTGAAGGTCTCCTGCAAGGCTTCTGATTACACCTTCACCAGCTATGGTATCAGCTGGGTGCGACAGGCCCCTGGACAAGGGCTTGAGTGGATGGGATGGATCAGCGCTTACAATGGTAACACAAACTATGCACAGAAGCTCCGGGGCAGAGTCACCATGACCACAGACACATCCACGAGCACAGCCTACATGGATCTGAGGAGCCTGAGATCTGACGACACGGCCGTGTATTACTGTGCGAGAACCGCTGCTGGGTATAGCAGCAGCTGGTTTGACTACTGGGGCCAGGGAACCCTGGTCACCGTCTCCTCA |
| | | | SEQ ID NO: 24629 | SEQ ID NO: 28635 |

FIGURE 50

| | | | | |
|---|---|---|---|---|
| | | AA | EIVMTQSPATLSVSPGERATLSCRASQSVSSNLAWYHQKPGQAPRLLIYGASTRATGIPARFSGSGSGTEFTLTISSLQSEDFALYYCQQYNNWPPWPFGQGTKVEIK | QVQLVQSGAEVKKPGASVKVSCKASDYTFTSYGISWVRQAPGQGLEWMGWISAYNGNTTYAQKLRGRVTMTTDTSTSTAYMDLRSLRSDDTAVYYCARTAAGYSSSWFDYWGQGTLVTVSS |
| | | | SEQ ID NO: 24630 | SEQ ID NO: 28636 |
| **iPS:393054** | **21-225_29G8** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCTGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATAACTGTCTACAGCATAATAGTTATCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGAAACTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGATGACGAGTGACTACTGGGGCCAGGGAACCCTGGTCACCGTCTCCTCA |
| | | | SEQ ID NO: 24631 | SEQ ID NO: 28637 |
| | | AA | DIQMTQSPSSLSASVGDRVTITCRASQGIRNDLGWYQLKPGKAPKRLIYAASSLQSGVPSRFSGSGSGTEFTLTISSLQPEDFATYNCLQHNSYPLTFGGGTKVEIK | QVQLVESGGGVVQPGRSLRLSCAASGFTFSDYGMHWVRQAPGKGLEWVAVIWYDETNKYYADSVKGRFTISRDNSKNTLYLQMNSLRAEDTAVYYCARELGMTSDYWGQGTLVTVSS |
| | | | SEQ ID NO: 24632 | SEQ ID NO: 28638 |

FIGURE 50

| | | | | |
|---|---|---|---|---|
| **iPS:393056** | **21-225_30F3** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATACTGCATCCAGTTTACAAAGTGGGGTCCCATCAAGGTTCAGCGGCAGTGGATCTGGGACAGATTTCACTCTCACAATCAGCAGCCTGCAGCCTGAAGATTTTGCAAGTTATTACTGTCTACAGCATAATAGTTACCCGTTCACTTTCGGCGGAGGGACCAAGGTGGAGATCAAA | CAGGTGCAGCTGGTGGAATCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGTAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAATGGGCTGGTACGATGACTACTGGGGCCAGGGAACCCTGGTCACCGTCTCCTCA |
| | | | SEQ ID NO: 24633 | SEQ ID NO: 28639 |
| | | AA | DIQMTQSPSSLSASVGDRVTITCRASQGIRNDLGWYQQKPGKAPKRLIYTASSLQSGVPSRFSGSGSGTDFTLTISSLQPEDFASYYCLQHNSYPFTFGGGTKVEIK | QVQLVESGGGVVQPGRSLRLSCAASGFTFSSYGMHWVRQAPGKGLEWVAVIWYDVSNKYYADSVKGRFTISRDNSKNTLYLQMNSLRAEDTAVYYCAREMGWYDDYWGQGTLVTVSS |
| | | | SEQ ID NO: 24634 | SEQ ID NO: 28640 |
| **iPS:393058** | **21-225_31H3** | NA | GACATCCAGATGACACAGTCTCCATCCTCCCTGTCTGCATCTGTAGGAGACAGAGTCACCATCACTTGCCGGGCAAGTCAGGACATTAGAGATGATTTAGGCTGGTATCAGCAGAAACCAGGGAAAGCCCCTAGGCGCCTGATCTATGCTGCATTCAGCTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATACTAGTTACCCTCCTACTTTCGGCGGAGGGACCAAGGTGGAGATCAAA | GAGGTGCAGGTGTTGGAGTCTGGGGGAGGCTTGGTACAGCCTGGGGGGTCCCTGAGACTCTCCTGTGCAGCCTCTGGATTCACCTTTAGCAGCTATGCCATGAACTGGGTCCGCCAGGCTCCAGGGAAGGGGCTGGAGTGGGTCTCCGCTATTAGTGGTCGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAAGGGGAGTTACTAGAGGACTACTACTACTACGGAATGGACGTCTGGGGCCAAGGGACCACGGTCACCGTCTCCTCA |
| | | | SEQ ID NO: 24635 | SEQ ID NO: 28641 |

FIGURE 50

| | | | | |
|---|---|---|---|---|
| | | AA | DIQMTQSPSSLSASVGDRVTITCRASQDIRDDLGWYQQKPGKAPRRLIYAAFSLQSGVPSRFSGSGSGTEFTLTISSLQPEDFATYYCLQHTSYPPTFGGGTKVEIK | EVQVLESGGGLVQPGGSLRLSCAASGFTFSSYAMNWVRQAPGKGLEWVSAISGRGGNTFYADSVKGRFTISRDNSKNTLYLQMNSLRAEDTAVYYCAKGELLEDYYYYGMDVWGQGTTVTVSS |
| | | | SEQ ID NO: 24636 | SEQ ID NO: 28642 |
| **iPS:393060** | **21-225_32G12** | NA | GACATCCAGATGACCCAGTCTCCATCCTCCCTGTCTGCATCTGTAGGAGACAGAGTCACCATCACTTGCCGGGCAAGTCAGGACATTAGAAGTGATTTAGGCTGGTATCAGCAGAGACCAGGAAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTACAGCCTGAAGATTTTGCAACTTATTACTGTCTGCAGCATACTATTTACCCTCCTACTTTCGGCGGAGGGACCAAGGTGGAGATCAAA | GAGGTGCAGCTGTTGGAGTCTGGGGGAGGCTTGCTACAGCCTGGGGGGTCCCTGAGACTCTCCTGTGCAGCCTCTGGATTCACCTTTAATAGCTATGCCATGAGCTGGGTCCGCCAGGCTCCAGGGAAGGGGCTGGAGTGGGTCTCCTCTATTAGTGGTCGTGGTGGTAGCACATTCCACGCAGACTCCGTGAAGGGCCGGTTCACCATCTCCAGAGACAATTCCAGGAACACGCTGTATCTGCAAATGAACAGCCTGAGAGCCGAGGACACGGCCGTATATTACTGTGTGAAAGGGGAGCTACTAGAGGACTACTACTTCTACGGTATGGACGTCTGGGGCCAAGGGACCACGGTCACCGTCTCCTCA |
| | | | SEQ ID NO: 24637 | SEQ ID NO: 28643 |
| | | AA | DIQMTQSPSSLSASVGDRVTITCRASQDIRSDLGWYQQRPGKAPKRLIYAASSLQSGVPSRFSGSGSGTEFTLTISSLQPEDFATYYCLQHTIYPPTFGGGTKVEIK | EVQLLESGGGLLQPGGSLRLSCAASGFTFNSYAMSWVRQAPGKGLEWVSSISGRGGSTFHADSVKGRFTISRDNSRNTLYLQMNSLRAEDTAVYYCVKGELLEDYYFYGMDVWGQGTTVTVSS |
| | | | SEQ ID NO: 24638 | SEQ ID NO: 28644 |

FIGURE 50

| | | | | |
|---|---|---|---|---|
| **iPS:393062** | **21-225_33H3** | NA | GACATCCAGATGACCCAGTCTCCATCCTCCCTGTCTGCATCTGTTGGAGACAGAGTCACCATCACTTGCCAGGCGAGTCAGGACATTTCCAACTTTTTAAATTGGTTTCGGCAGAAACCAGGAAAAGCCCCTAACTCCCTGATCTACGATGCATCCAATTTGGTAACAGGGGTCCCATCAAGGTTCAGTGGACGTGGATCTGGGACAGATTTTACTTTCACCATCAGCAGCCTGCAGCCTGAAGATTTTGCAACATATTACTGTCAACAGTATGATAATCTCCCGATCACCTTCGGCCAAGGGACACGGCTGGAGATTAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCGGGCTCCAGGCAAGGGGCTGGAGTGGGTGGCAATTATATCATATGGTGGAAGTAATAACTTCTATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTACTGTGCGAGAAGGGGGTATAGCAGTGGAGGCTACGGTATGGACGTCTGGGGCCAAGGGACCACGGTCACCGTCTCCTCA |
| | | | SEQ ID NO: 24639 | SEQ ID NO: 28645 |
| | | AA | DIQMTQSPSSLSASVGDRVTITCQASQDISNFLNWFRQKPGKAPNSLIYDASNLVTGVPSRFSGRGSGTDFTFTISSLQPEDFATYYCQQYDNLPITFGQGTRLEIK | QVQLVESGGGVVQPGRSLRLSCAASGFTFSSYGMHWVRRAPGKGLEWVAIISYGGSNNFYADSVKGRFTISRDNSKNTLYLQMNSLRAEDTAVYYCARRGYSSGGYGMDVWGQGTTVTVSS |
| | | | SEQ ID NO: 24640 | SEQ ID NO: 28646 |
| **iPS:393064** | **21-225_33A9** | NA | GACATCCAGATGACCCAGTCTCCATCCTCCCTGTCTGCATCTGTAGGAGACAGAGTCACCATCACTTGCCGGGCAAGTCAGAGCATTAGCAGGTATTTAAGTTGGTATCAGCAGAAACCAGGGAGAGCCCCTAACCTCCAGATCTATGCTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACAATATCCCGATCACCTTCGGCCAAGGGACACGACTGGAGATTAAA | CAGGTGCAGCTGGTGCAGTCTGGGGCTGAGGTGAAGAAGCCTGGGGCCTCAGTGAAGGTCTCCTGTAAGGCTTCTGGATACACCTTCACCAGTTATGATATCAACTGGGTGCGACAGGCCACTGGTCAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGGGATCTGAGGACACGGCCGTGTATTACTGTGCGAGAAAGAAGGCTAACGACTACTGGGGCCAGGGAACCCTGGTCACCGTCTCCTCA |
| | | | SEQ ID NO: 24641 | SEQ ID NO: 28647 |

FIGURE 50

| | | | | |
|---|---|---|---|---|
| | | AA | DIQMTQSPSSLSASVGDRVTITCRASQSISRYLSWYQQKPGRAPNLQIYAASSLQSGVPSRFSGSGSGTDFTLTISSLQPEDFATYYCQQSYNIPITFGQGTRLEIK | QVQLVQSGAEVKKPGASVKVSCKASGYTFTSYDINWVRQATGQGLEWMGWMNPNSGNTGYAQKFQGRVTMTRNTSISTAYMELSSLGSEDTAVYYCARKKANDYWGQGTLVTVSS |
| | | | SEQ ID NO: 24642 | SEQ ID NO: 28648 |
| iPS:393066 | 21-225_34D3 | NA | GACATCCAGATGACCCAGTCTCCATCCTCCCTGTCTGCATCTGTTGGAGACAGAGTCACCATCACTTGCCGGGCAAGTCAGAACATTTACAGCTATTTAAATTGGTATCAGCAGAAACCAGGGAAAGACCCTAAACTCCTGATCTATGCTGCATCCAGTTTGCATAGTGGGGTCCCGTCACGGTTCAGTGGCAGTGGTTCTGGGACAGATTTCACTCTCACCATCAGCAGTCTGCAACCTGAAGATTTTGCAACTTTCTACTGTCAACAGAGTTACAGTACTCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTTACTATGGCATGCACTGGGTCCGCCAGGCTCCAGGCAAGGGGCTGGAGTGGGTGGCAGTTATATGGCATGATGGAAGTAATAAATACTATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAATAGCAGTTCGTTCTACTTTGACTACTGGGGCCAGGGAACCCTGGTCACCGTCTCCTCA |
| | | | SEQ ID NO: 24643 | SEQ ID NO: 28649 |
| | | AA | DIQMTQSPSSLSASVGDRVTITCRASQNIYSYLNWYQQKPGKDPKLLIYAASSLHSGVPSRFSGSGSGTDFTLTISSLQPEDFATFYCQQSYSTPLTFGGGTKVEIK | QVQLVESGGGVVQPGRSLRLSCAASGFTFSYYGMHWVRQAPGKGLEWVAVIWHDGSNKYYVDSVKGRFTISRDNSKNTLYLQMNSLRAEDTAVYYCARENSSSFYFDYWGQGTLVTVSS |
| | | | SEQ ID NO: 24644 | SEQ ID NO: 28650 |

FIGURE 50

| | | | | |
|---|---|---|---|---|
| **iPS:393068** | **21-225_34G9** | NA | GACATCCAGATGACCCAGTCTCCATCCTCCCT<br>GTCTGCATCTGTAGGAGACAGAGTCACCATCA<br>CTTGCCGGGCAAGTCAGGACATTAGAAGTGAT<br>TTAGGCTGGTATCAGCAGAAACCAGGGAAAG<br>CCCCTAAGCGCCTGATCTATGCTGCATCCAGT<br>TTGCAAAGTGGGGTCCCATCAAGGTTCACCGG<br>CAGTGGCTCTGGGACAGAATTCACTCTCACAA<br>TCAGCAGCCTGCAGCCTGAAGCTTTTGCAATT<br>TATTACTGTCTCCAGCATACTATTTACCCTCCT<br>ACTTTCGGCGGAGGGACCAAGGTGTGGATCAA<br>A | GAGGTGCAGCTGGTGGAGTCTGGGGGAGGCTTG<br>GTACAGCCTGGGGGGTCCCTGAGACTCTCCTGTG<br>CAGCCTCTGGATTCACCTTTAGCAGCTATGCCAT<br>GAGCTGGGTCCGCCAGGCTCCAGGGAAGGGGCT<br>GGAGTGGGTCTCCGCTATTAGTGGTCGTGGTGGT<br>AGCACATTCCACGCAGACTCCGTGAAGGGCCGG<br>TTCACCATCTCCAGAGACAATTCCAAGAACACGC<br>TGTATCTGCAAATGAACAGCCTGAGAGCCGAGG<br>ACACGGCCGTATATTACTGTGTGAAAGGGGAGCT<br>ACTAGAGGACTACTACTTCTACGGTATGGACGTC<br>TGGGGCCAAGGGACCACGGTCACCGTCTCCTCA |
| | | | SEQ ID NO: 24645 | SEQ ID NO: 28651 |
| | | AA | DIQMTQSPSSLSASVGDRVTITCRASQDIRSDLG<br>WYQQKPGKAPKRLIYAASSLQSGVPSRFTGSGS<br>GTEFTLTISSLQPEAFAIYYCLQHTIYPPTFGGGT<br>KVWIK | EVQLVESGGGLVQPGGSLRLSCAASGFTFSSYAMS<br>WVRQAPGKGLEWVSAISGRGGSTFHADSVKGRFTI<br>SRDNSKNTLYLQMNSLRAEDTAVYYCVKGELLED<br>YYFYGMDVWGQGTTVTVSS |
| | | | SEQ ID NO: 24646 | SEQ ID NO: 28652 |
| **iPS:393072** | **21-225_36C5** | NA | GACATCCAGATGACCCAGTCTCCATCCTCCCT<br>GTCTGCATCTGTAGGAGACAGAGTCACCATCA<br>CTTGCCGGGCAAGTCAGGACATTAGAAGTGAT<br>TTAGGCTGGTATCAGCAGAAACCAGGGAAAG<br>CCCCTAAGCGCCTGATCTATGCTGCATCCAGT<br>TTGCAAAGTGGGGTCCCATCAAGGTTCACCGG<br>CAGTGGATCTGGGACAGAATTCACTCTCACAA<br>TCAGCAGCGTGCAGCCTGAAGATTTTGCAACT<br>TATTACTGTCTCCATCATCCTATTTACCCTCCT<br>ACTTTCGGCGGAGGGACCAAGGTGTGGATCAA<br>A | GAGGTGCAGCTGGTGGAGTCTGGGGGAGGCTTG<br>GTACAGCCTGGGGGGTCCCTGAGACTCTCCTGTG<br>CAGCCTCTGGATTCACCTTTAGCAGCTATGCCAT<br>GAACTGGGTCCGCCAGGCTCCAGGGAAGGGGCT<br>GGAGTGGGTCTCCGCTATTAGTGGTCGTGGTGGT<br>AGCACATTCCACGCAGACTCCGTGAAGGGCCGG<br>TTCACCATCTCCAGAGACAATTCCAAGAACACGC<br>TGTATCTGCAAATGAACAGCCTGAGAGCCGAGG<br>ACACGGCCGTATATTACTGTGTGAAAGGGGAGCT<br>ATTAGAGGACTACTACTTCTACGGTATGGACGTC<br>TGGGGCCAAGGGACCACGGTCACCGTCTCCTCA |
| | | | SEQ ID NO: 24647 | SEQ ID NO: 28653 |

FIGURE 50

| | | | | |
|---|---|---|---|---|
| | | AA | DIQMTQSPSSLSASVGDRVTITCRASQDIRSDLGWYQQKPGKAPKRLIYAASSLQSGVPSRFTGSGSGTEFTLTISSVQPEDFATYYCLHHPIYPPTFGGGTKVWIK | EVQLVESGGGLVQPGGSLRLSCAASGFTFSSYAMNWVRQAPGKGLEWVSAISGRGGSTFHADSVKGRFTISRDNSKNTLYLQMNSLRAEDTAVYYCVKGELLEDYYFYGMDVWGQGTTVTVSS |
| | | | SEQ ID NO: 24648 | SEQ ID NO: 28654 |
| iPS:393074 | 21-225_33B1 | NA | GACATCCAGATGACCCAGTCTCCATCCTCCCTGTCTGCATCTGTAGGAGACAGAGTCACCATCACTTGCCGGACAAGTCAGGGCATTAGAAATGATTTAGGCTGGTTTCAGCAGAAACCAGGGAAAGCCCCTAAGCGCCTGATCTATACTGCATCCAGTTTGCAAAGTGGGGTCCCATCAAGGTTCAGCGGCAGTGGATCTGGGACAGAATTCACTCTCACAATTAGCAGCCTGCAGCCTGAAGATTTTGCAACTTATTACTGTCTACAGCATAATAGTTACCCGCTCACTTTCGGCGGAGGGACCAAGGTGGAGGTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGCCAAGGGGCTGGAGTGGGTGGCAGTTATATGGTATGATAGAAATAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTATATTACTGTGCGAGAGAAATGGGCTGGTACGATGACTACTGGGGCCAGGGAACCCTGGTCACCGTCTCCTCA |
| | | | SEQ ID NO: 24649 | SEQ ID NO: 28655 |
| | | AA | DIQMTQSPSSLSASVGDRVTITCRTSQGIRNDLGWFQQKPGKAPKRLIYTASSLQSGVPSRFSGSGSGTEFTLTISSLQPEDFATYYCLQHNSYPLTFGGGTKVEVK | QVQLVESGGGVVQPGRSLRLSCAASGFTFSDYGMHWVRQAPAKGLEWVAVIWYDRNNKYYADSVKGRFTISRDNSKNTLYLQMNSLRAEDTAVYYCAREMGWYDDYWGQGTLVTVSS |
| | | | SEQ ID NO: 24650 | SEQ ID NO: 28656 |

FIGURE 50

| | | | | |
|---|---|---|---|---|
| **iPS:393076** | **21-225_33A4** | NA | GACATCCAGATGACCCAGTCTCCATCCTCCCTGTCTGCATCTGTAGGAGACAGAGTCACCATCACTTGCCGGGCAAGTCAGGACATTAGAAATGATGTAGGCTGGTATCAGCAGAAACCAGGGAAAGCCCCTGAGCGCCTGATCTATGCTGCATCCAGTTTGCAACGTGGGGTCCCATCAAGGTTCAGCGGCAGTGGATCTGGGACAGAATTCACTCTCACAATCAGCAGCCTGCAGCCTGAAGATTTTGCAAGATATTACTGTCTACAGCATTATAGTTACCCTCCTACTTTCGGCGGAGGGACCAAGGTGGAGATCAAA | GAGGTGCAGTTGTTGGAGTCTGGGGGAGGCTTGGTACAGTCTGGGGGGTCCCTGAGACTCTCCTGTGAAGCCTCAGGATTCATCTTTAGCAGCTATGCCATGAACTGGGTCCGCCAGGTTCCAGGGAAGGGGCTGGAGTGGGTCTCAGCTATTAGTCGTCGTGGTGGTAGCACATTCTACGCAGACTCCGTGAAGGGCCGGTTCACCATTTCCAGAGACAATTCCAAGAACACGCTGTATCTGCAAATGAACAGCCTGAGAGCCGAGGACACGGCCGTATATTTCTGTGCGAAAGGGGAACTACTAGAGGACTACTCCTACTACGGTATCGACGTCTGGGGCCAGGGGACCACGGTCACCGTCTCCTCA |
| | | | SEQ ID NO: 24651 | SEQ ID NO: 28657 |
| | | AA | DIQMTQSPSSLSASVGDRVTITCRASQDIRNDVGWYQQKPGKAPERLIYAASSLQRGVPSRFSGSGSGTEFTLTISSLQPEDFARYYCLQHYSYPPTFGGGTKVEIK | EVQLLESGGGLVQSGGSLRLSCEASGFIFSSYAMNWVRQVPGKGLEWVSAISRRGGSTFYADSVKGRFTISRDNSKNTLYLQMNSLRAEDTAVYFCAKGELLEDYSYYGIDVWGQGTTVTVSS |
| | | | SEQ ID NO: 24652 | SEQ ID NO: 28658 |
| **iPS:393078** | **21-225_33H11** | NA | GACATCCAGATGACCCAGTCTCCATCCTCACTGTCTGCATTTGTAGGAGACAGAGTCACCATCACTTGTTGGGCGAGTCAGGGCATTAACAGTTATTTAGCCTGGTTTCAGCAGAGACCAGGGAAAGCCCATAAGTCCTTGATCTATGCTGCATCCAGTTTGCAAGGTGGGGTCCCATCAAAGTTCAGCGGCAGTGGATCTGGGACAGATTTCATTCTCACCATCAGCAGCCTGCAGCGTGAAGATTTTGCAACTTATTACTGCCAACAGTTTAATAGTTACCCTCTGACGTTCGGCCAAGGGACCAAGGTGGAAATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTAGTTACATATACTACGCAGACTCAGTGAAGGGCCGATTCACCATCTCCAGAGACAACGCCAAGAACTCACTGTATCTGCAAATGAGCAGCCTGAGAGCCGAGGACACGGCTGTGTACTACTGTGCGAGAACAAACGGTATGGACGTCTGGGGCCAAGGGACCACGGTTACCGTCTCCTCA |
| | | | SEQ ID NO: 24653 | SEQ ID NO: 28659 |

FIGURE 50

| | | | | |
|---|---|---|---|---|
| | | AA | DIQMTQSPSSLSAFVGDRVTITCWASQGINSYLAWFQQRPGKAHKSLIYAASSLQGGVPSKFSGSGSGTDFILTISSLQREDFATYYCQQFNSYPLTFGQGTKVEIK | EVQLVESGGGLVKPGGSLRLSCAASGFTFSSYSMNWVRQAPGKGLEWVSSISGSSSYIYYADSVKGRFTISRDNAKNSLYLQMSSLRAEDTAVYYCARTNGMDVWGQGTTVTVSS |
| | | | SEQ ID NO: 24654 | SEQ ID NO: 28660 |
| iPS:393080 | 21-225_34F3 | NA | GACATCCAGATGACCCAGTCTCCGTCTTCCGTGTCTGCAACTGTAGGAGACAGAGTCACCAGCACTTGTCGGGCGAGTCAGGGTATTAGTAAGTGGTTAGCCTGGTATCAGCAGAAGCCAGGGAAAGCCCCTAAACTCCTGATCTATGCTGCATCCAGTTTGCAAAGTGGGGTCCCATCAAGGTTCAGCGGCAGTGGATCTGGGACAGATTTCACTCTCACCATCAGCAGCCTGCAGCCTGAAGACTCTGCAACTTACTATTGTCAACAGGCTAACAGTTTCCCTTTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACCATATGCACTGGGTGCGACAGGCCCCTGGACAAGGGCTTGACTGGATGGGATGGATCAACCCTAACAGTGGTGGCACAAACTATGCACAGAAGTTTCAGGGCAGGGTCACCATGACCAGGGACACGTCCATCAGCACAGCCTATATGGAGCTGAGCAGGCTGAGATCTGACGACACGGCCGTGTATTACTGTGCGAGAGACGGTACCAGCTCCTTTGACTACTGGGGCCAGGGAACCCTGGTCACCGTCTCCTCA |
| | | | SEQ ID NO: 24655 | SEQ ID NO: 28661 |
| | | AA | DIQMTQSPSSVSATVGDRVTSTCRASQGISKWLAWYQQKPGKAPKLLIYAASSLQSGVPSRFSGSGSGTDFTLTISSLQPEDSATYYCQQANSFPFTFGPGTKVDIK | QVQLVQSGAEVKKPGASVKVSCKASGYTFTGYHMHWVRQAPGQGLDWMGWINPNSGGTNYAQKFQGRVTMTRDTSISTAYMELSRLRSDDTAVYYCARDGTSSFDYWGQGTLVTVSS |
| | | | SEQ ID NO: 24656 | SEQ ID NO: 28662 |

FIGURE 50

| | | | | |
|---|---|---|---|---|
| **iPS:393082** | **21-225_34C11** | NA | GACATCCAGATGACCCAGTTTCCATCTTCCCTGTCTACATCTGTAGGAGACAGAGTCACCAGCACTTGCCGGGCAAGTCAGAACATTAGGAACTTTTTAAATTGGTATCAGCAGAAACCTGAGAAAGACCCTAAGCTCCAGATCTATGGTGCATCCACTTTGCAAAGTGGGGTCCCATCAAGGTTCAGTGGCAGTGGATTTGGGACAGATTTCACTCTCACCATCAGTAGTCTGCAACCTAAAGATTTTGCAACTTACTACTGTCAACAGACTTGCAGTACCCCGCTCACTTTCGGCGGAGGGACCAAGGTGGAGATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGTTATACCATGAGCTGGGTCCGCCAGGCTCCAGGGAAGGGGCTGGAGTGGGTCTCATCCATTAGTGGTAGTAGTAATTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGATTAACTGGTTTTGACTACTGGGGCCAGGGAACCCTGGTCACCGTCTCCTCA |
| | | | SEQ ID NO: 24657 | SEQ ID NO: 28663 |
| | | AA | DIQMTQFPSSLSTSVGDRVTSTCRASQNIRNFLNWYQQKPEKDPKLQIYGASTLQSGVPSRFSGSGFGTDFTLTISSLQPKDFATYYCQQTCSTPLTFGGGTKVEIK | EVQLVESGGGLVKPGGSLRLSCAASGFTFSSYTMSWVRQAPGKGLEWVSSISGSSNYIYYADSVKGRFTISRDNAKNSLYLQMNSLRAEDTAVYYCARLTGFDYWGQGTLVTVSS |
| | | | SEQ ID NO: 24658 | SEQ ID NO: 28664 |
| **iPS:393084** | **21-225_35C6** | NA | GACATCCAGATGACCCAGTCTCCATCTTCCGTGTCTGCATCTGTAGGAGACAGAGTCACCATCACTTGTCGGGCGAGTCAGGGTATTAGCAAATGGTTAGCCTGGTATCAGCAGAAACCAGGGAAAGCCCCTAAACCCCTGATCTATGCTGCATCCAGTTTGCAGAGTGGGGTCCCAACAAGGTTCAGCGGCAGTGGATCTGGGACAGATTTCACTCTCACCATCAGCAGCCTGCAGCCTGAAGATTTTGCAATTTACTATTGTCAACAGGCTAACAGTTTCCCATTCACTTTCGGCCCTGGGACCAAAGTGGATATCAAA | CAGGTGCAGTTGGTGCAGTCTGGGACTGAGGTGAAGAAGCCTGGGGCCTCAGTGAAGGTCTCCTGCAAGGCTTCTGGATACACCTTCACCGGCGATTATATGCACTGGGTGCGACAGGCCCCTGGACAAGGGCTTGAGTGGATGGGATGGATCAGCCCTAAAAATGGTGGCACAAACTATGCACAGAAGTTTCAGGGCAGGGTCACCATGACCAGGGACACGTCCATCAGCACAGCCTACATGGAGCTGAACAGGCTGAGATCTGACGACACGGCCGTGTATTACTGTGCGAGAGATGGAACTGGGTCCTTTGACTACTGGGGCCAGGGAACCCTGGTCACCGTCTCCTCA |
| | | | SEQ ID NO: 24659 | SEQ ID NO: 28665 |

FIGURE 50

| | | | | |
|---|---|---|---|---|
| | | AA | DIQMTQSPSSVSASVGDRVTITCRASQGISKWLA WYQQKPGKAPKPLIYAASSLQSGVPTRFSGSGS GTDFTLTISSLQPEDFAIYYCQQANSFPFTFGPGT KVDIK | QVQLVQSGTEVKKPGASVKVSCKASGYTFTGDYM HWVRQAPGQGLEWMGWISPKNGGTNYAQKFQGR VTMTRDTSISTAYMELNRLRSDDTAVYYCARDGT GSFDYWGQGTLVTVSS |
| | | | SEQ ID NO: 24660 | SEQ ID NO: 28666 |
| iPS:393086 | 21-225_36H5 | NA | GACATCCAGATGACCCAGTCTCCATCTTCCGT GTCTGCATCTGTAGGAGACAGAGTCACCATCA CTTGTCGGGCGAGTCAGGGTATTAGCAGATGG TTAGCCTGGTATCAGCAGAAACCAGGGAAAG CCCCTGAACTCCTGATCTATGCTGCATCCCGTT TGCAAAGTGGGATCCCATCCAGGTTCAGCGGC AGTGGATCTGGGACAGACTTCACTCTCACCAT CAGCAGCCTGCAGCCTGAAGATTTTGCAACTT ACTATTGTCAACAGGCTAACAGTTTCCCTTTCA CTTTCGGCCCTGGGACCAAAGTGGATATCAAA | CAGGTGCAGCTGGTGCAGTCTGGGGCTGAGGTG AAGAAGCCTGGGGCCTCAATGAAGGTCTCCTGC AAGGCTTCTGGATACACCTTCACCGACTATCATA TGCACTGGGTGCGACAGGCCCCTGGACAAGGGC TTGAGTGGATGGGATGGATCAACCCTAATAGGG GTGGCACAAACTATGCACAGAAGTTTCAGGACA GGGTCACCATGACCAGGGACACGTCCATCAGCA CAGCCTACATGGAACTGAGCAGGCTGAGATCTG ACGACACGGCCGTGTATTTCTGTGCGAGAGATGG AACTGGGTCCTTTGACTACTGGGGCCAGGGAACC CTGGTCACCGTCTCCTCA |
| | | | SEQ ID NO: 24661 | SEQ ID NO: 28667 |
| | | AA | DIQMTQSPSSVSASVGDRVTITCRASQGISRWLA WYQQKPGKAPELLIYAASRLQSGIPSRFSGSGSG TDFTLTISSLQPEDFATYYCQQANSFPFTFGPGTK VDIK | QVQLVQSGAEVKKPGASMKVSCKASGYTFTDYHM HWVRQAPGQGLEWMGWINPNRGGTNYAQKFQDR VTMTRDTSISTAYMELSRLRSDDTAVYFCARDGTG SFDYWGQGTLVTVSS |
| | | | SEQ ID NO: 24662 | SEQ ID NO: 28668 |

FIGURE 50

| | | | | |
|---|---|---|---|---|
| **iPS:393088** | **21-225_33D1** | NA | GACATCGTGATGACCCAGTCTCCAGACTCCCTGTCTGTGTCTTTGGGCGAGAGGGCCACCATCAACTGCAAGTCCATCCAGAGTGTTTTATACAGATCCAACAATAAGAACTACTTAACTTGGTATCAGCAGAAACCAGGACAGCCTCGTAAACTGTTCATTTATTGGGCATCTACCCGGGAATCCGGGGTCCTTGACCGATTCAGTGGCAGCGGGTGTGGGACAGATTTCACTCTCACCATCATCAGCCTGCAGGCTGAAGATGTGGCACTTTATTACTGTCAGCAATATTATAGTTCTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGACATTAACTGGGTGCGACAGGCCACTGGACAAGGGCTTGAGTGGATGGGATGGATGCACCCTAACAGTGGTAACACAGGCTTTGCACAGAAGTTCCGGGGCAGAGTCACCATGACCAGAAACACCTCCATAAGCACAGCCTACATGGAACTGAGCAGCCTGAGATCTGAGGACACGGCCGTGTATTACTGTGCGAGCAGCAGTGGCTGGTACTTTTTTGACTACTGGGGCCAGGGAACCCTGGTCACCGTCTCCTCA |
| | | | SEQ ID NO: 24663 | SEQ ID NO: 28669 |
| | | AA | DIVMTQSPDSLSVSLGERATINCKSIQSVLYRSNNKNYLTWYQQKPGQPRKLFIYWASTRESGVLDRFSGSGCGTDFTLTIISLQAEDVALYYCQQYYSSPCSFGQGTKLEIK | QVQLVQSGAEVKKPGASVKVSCKASGYTFTNYDINWVRQATGQGLEWMGWMHPNSGNTGFAQKFRGRVTMTRNTSISTAYMELSSLRSEDTAVYYCASSSGWYFFDYWGQGTLVTVSS |
| | | | SEQ ID NO: 24664 | SEQ ID NO: 28670 |
| **iPS:393090** | **21-225_33A5** | NA | GACATCCAGATGACCCAGTCTCCATCCTCACTGTCTGCATCTGTAGGAGACAGAGTCACCATCACTTGTCGGGCGAGTCAGGGCATTAGCAATTACTTAGCCTGGTTTCAGCAGAAACCAGGGAAAGCCCCTAAGTCCCTGATCTCTGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATAATAGTTACCCATTCACTTTCGGCCCTGGGACCAACGTGGATATCAAA | GAGGTGCAGCTGTTAGAGTCTGGGGGAGGCTTGGTACAGCCTGGGGGGTCCCTGAGACTCTCCTGTGCAGCCTCTGGATTCACCTTTAGCAGCTATGTCATAAACTGGGTCCGCCAGGCTCCAGGGAAGGGGCTGGAGTGGGTCTCAGCTATTAGTGGTAGTGGTGTTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAGAACTTCCCTCTTTGACTACTGGGGCCAGGGAACCCTGGTCACCGTCTCCTCA |
| | | | SEQ ID NO: 24665 | SEQ ID NO: 28671 |

FIGURE 50

| | | | | |
|---|---|---|---|---|
| | | AA | DIQMTQSPSSLSASVGDRVTITCRASQGISNYLAWFQQKPGKAPKSLISAASSLQSGVPSKFSGSGSGTDFTLTISSLQPEDFATYYCQQYNSYPFTFGPGTNVDIK | EVQLLESGGGLVQPGGSLRLSCAASGFTFSSYVINWVRQAPGKGLEWVSAISGSGVSTYYADSVKGRFTISRDNSKNTLYLQMNSLRAEDTAVYYCARTSLFDYWGQGTLVTVSS |
| | | | SEQ ID NO: 24666 | SEQ ID NO: 28672 |
| iPS:393092 | 21-225_33C12 | NA | GACATCCAGATGACCCAGTCTCCATCCTCCCTGTCTACATCTGTAGGAGACAGAGTCACCATCACTTGCCGGGCAAGTCAGAGCATTATCAGCTATTTAAATTGGTATCAGCAGAAACCAGGGAAAGCCCCTAAACTCCTGATCTATGTTGCATCCAGTTTGCAAGGTGGGGTCCCATCAAGGTTCAATGGCAGTGGATCTGGGACAGATTTCACTCTCACCATCAGCAGTCTGCAACCTGAAGATTTTGCAACTTACTACTGTCAACAGAGTTACAGTACCCCGTACACTTTCGGCGGGGGGACCAAGGTGGAGATCAAA | CAGGTGCAGTTGGTGGAATCTGGGGGAGGCGTGGTCCAGCCTGGGAGGTCCCTGAGACTCTCCTGTGCAGCGTCTGGATTCACCTTCAGTCACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAATAGCAGCTCGTACTACTTTGACTACTGGGGCCAGGGAACCCTGGTCACCGTCTCCTCA |
| | | | SEQ ID NO: 24667 | SEQ ID NO: 28673 |
| | | AA | DIQMTQSPSSLSTSVGDRVTITCRASQSIISYLNWYQQKPGKAPKLLIYVASSLQGGVPSRFNGSGSGTDFTLTISSLQPEDFATYYCQQSYSTPYTFGGGTKVEIK | QVQLVESGGGVVQPGRSLRLSCAASGFTFSHYGMHWVRQAPGKGLEWVAVIWYDGSNKYYADSVKGRFTISRDNSKNTLYLQMNSLRAEDTAVYYCARENSSSYYFDYWGQGTLVTVSS |
| | | | SEQ ID NO: 24668 | SEQ ID NO: 28674 |

FIGURE 50

| | | | | |
|---|---|---|---|---|
| **iPS:393094** | **21-225_34C4** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAACAGAAACCAGGGAAAGCCCCTAAGCGCCTGATCTATACTGCATCCAATTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAACATAGTTCTTACCCCATCACCTTCGGCCAAGGGACACGACTGGAGATTAAA | CAGCTGCAGCTGCAGGAGTCGGGCCCAGGACTGGTGAAGCCTTCGGAGACCCTGTCCCTCACCTGCACTGTCTCTGGTGGCTCCATCAGTAGAAGTAGTTACTACTGGGGCTGGATCCGCCAGTCCCCAGGGAAGGGGCTGGAGTGGATTGGGAGTATCTATTATAGTGGGAGCACCGCCTACAATCCGTCCCTCAAGAGTCGAGTCACCATATCCGTAGACACGTCCAAGAACCAGGTCTCCCTGAAGCTGAGCTCCGTGACCGCCGCAGACACGGCTGTGTATTACTGTGCGAGACTGAGCAGCAGCTGGTCTTTTGACTACTGGGGCCAGGGAACCCTGGTCACCGTCTCCTCA |
| | | | SEQ ID NO: 24669 | SEQ ID NO: 28675 |
| | | AA | DIQMTQSPSSLSASVGDRVTITCRASQGIRNDLGWYQQKPGKAPKRLIYTASNLQSGVPSRFSGSGSGTEFTLTISSLQPEDFATYYCLQHSSYPITFGQGTRLEIK | QLQLQESGPGLVKPSETLSLTCTVSGGSISRSSYYWGWIRQSPGKGLEWIGSIYYSGSTAYNPSLKSRVTISVDTSKNQVSLKLSSVTAADTAVYYCARLSSSWSFDYWGQGTLVTVSS |
| | | | SEQ ID NO: 24670 | SEQ ID NO: 28676 |
| **iPS:393096** | **21-225_34D11** | NA | GACATCCAGATGACCCAGTCTCCATCCTCCCTGTCTGCATCTGTAGGAGACAGAGTCACCATCACTTGCCGGGCAAGTCAGGACATTAGAAGTGATTTAGGCTGGTATCAGCAGAAACCAGGGAAAGCCCCTAAGCGCCTGATCTATGCTGCATCCAGTTTGCAAAGTGGGGTCCCATCCAGGTTCAGCGGCAGTGGATCTGGGACAGAATTCACTCTCACAGTCATCAGCCTGCAGCCTGAAGATTTTGCAACTTATTACTGTCTACAGCATACTATTTACCCTCCTACTTTCGGCGGAGGGACCAAGGTGGGGATCAAA | GAGGTGCAGCTGTCGGAGTCTGGGGGAGGCTTGGTACAGCCTGGGGGGTCCCTGAGACTCTCCTGTGCAGCCTCTGGATTCACCTTTAGCAGCTATGCCATGAACTGGGTCCGCCAGGCTCCAGGGAAGGGGCTGGAGTGGGTCTCCGCTATTAGTGGTCGTGGTGGTAGCACATTCCACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGTGAAAGGGGAGTTAGTAGAGGACTACTACTTCTACGGTATGGACGTCTGGGGCCAAGGGACCACGGTCACCGTCTCCTCA |
| | | | SEQ ID NO: 24671 | SEQ ID NO: 28677 |

FIGURE 50

| | | | | |
|---|---|---|---|---|
| | | AA | DIQMTQSPSSLSASVGDRVTITCRASQDIRSDLGWYQQKPGKAPKRLIYAASSLQSGVPSRFSGSGSGTEFTLTVISLQPEDFATYYCLQHTIYPPTFGGGTKVGIK | EVQLSESGGGLVQPGGSLRLSCAASGFTFSSYAMNWVRQAPGKGLEWVSAISGRGGSTFHADSVKGRFTISRDNSKNTLYLQMNSLRAEDTAVYYCVKGELVEDYYFYGMDVWGQGTTVTVSS |
| | | | SEQ ID NO: 24672 | SEQ ID NO: 28678 |
| iPS:393098 | 21-225_35G6 | NA | GACATCCAGATGACCCAGTCTCCATCTTCCGTGTCTGCATCTGTAGGCGACAGACTCACCATCACTTGTCGGGCGAGTCAGGGTATTAGCCGGTGGTTAGCCTGGTATCAGCAGAAAGTGGGGAAAGTCCCCAAACTCCTGATCTATGCTGCATCCAGGTTGCAAAGTGGGGTCCCATCAAGGTTCAGCGGCAGTGGATCTGGGACAGCTTTCACTCTCACCATCGGCAGCCTGCAGCCTGAAGATTTTGCAACTTACTATTGTCAACAGGCTAACAGTTTCCCGTTCACTTTCGGCCCTGGGACCAAAGTGGATCTCAAA | CAGGTGCAGCTGGTGCAGTCTGGGGCTGACGTGAAGAAGCCTGGGGCCTCAGTGAAGGTCTCCTGCAAGGCTTCTGGATACACCTTCACCGACTACCATATACACTGGGTGCGACAGGCCCCTGGACAAGGGCTTGAGTGGATGGGATGGATCAACCCTAACAATGGTGGCACACACTATGCACAGGAGTTTCAGGGCAGGGTCACCATGACCAGGGACACGTCCATCAGCACAGCCTACATGGAGCTGAGTAGCCTGAGATCTGACGACACGGCCGTGTATTACTGTGCGAGAGATGGAACTGGGTCCTTTGACTACTGGGGCCAGGGAACCCTGGTCACCGTCTCCTCA |
| | | | SEQ ID NO: 24673 | SEQ ID NO: 28679 |
| | | AA | DIQMTQSPSSVSASVGDRLTITCRASQGISRWLAWYQQKVGKVPKLLIYAASRLQSGVPSRFSGSGSGTAFTLTIGSLQPEDFATYYCQQANSFPFTFGPGTKVDLK | QVQLVQSGADVKKPGASVKVSCKASGYTFTDYHIHWVRQAPGQGLEWMGWINPNNGGTHYAQEFQGRVTMTRDTSISTAYMELSSLRSDDTAVYYCARDGTGSFDYWGQGTLVTVSS |
| | | | SEQ ID NO: 24674 | SEQ ID NO: 28680 |

FIGURE 50

| | | | | |
|---|---|---|---|---|
| **iPS:393100** | **21-225_36B8** | NA | GACATCCAGATGACCCAGTCTCCATCCTCCCTGTCTACTTCTGTAGGAGACAGAGTCACCATCACTTGCCGGGCAAGTCAGAGCATTATCAGCTATTTAAATTGGTATCAGCAGAAACCAGGGAAAGCCCCTAAACTCCTGATCTATGTTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCGACTTACTACTGTCAACAGAGTTACAGTACCCCGTACACTTTCGGCGGAGGGACCAAGATGGAGATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGCATGATGGAAGTAATAAATACTATGGAGACTCCGTGAAGGGCCGATTCACCATCTCCAGAGACAATTCCAAGAACACACTGTATCTGCAAATGAACAGCCTGAGAGCCGAGGACACGGCTGTGTATTACTGTGCGAGAGAAAATAGCAGCTCGTACTACTTTGACTACTGGGGCCAGGGAACCCTGGTCACCGTCTCCTCA |
| | | | SEQ ID NO: 24675 | SEQ ID NO: 28681 |
| | | AA | DIQMTQSPSSLSTSVGDRVTITCRASQSIISYLNWYQQKPGKAPKLLIYVASSLQSGVPSRFSGSGSGTDFTLTISSLQPEDFATYYCQQSYSTPYTFGGGTKMEIK | QVQLVESGGGVVQPGRSLRLSCAASGFTFSNYGMHWVRQAPGKGLEWVAVIWHDGSNKYYGDSVKGRFTISRDNSKNTLYLQMNSLRAEDTAVYYCARENSSSYYFDYWGQGTLVTVSS |
| | | | SEQ ID NO: 24676 | SEQ ID NO: 28682 |
| **iPS:393102** | **21-225_33F1** | NA | GACATCCAGATGACCCAGTCTCCATCCTCCCTGTCTGCATCTGTAGGAGACAGAGTCACCATCACTTGCCGGACAAGTCAGGACATTAGAAGTGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAATTTATTACTGTCTACAGCATACTATTTACCCTCCTACTTTCGGCGGAGGGACCAAGGTGGAGATCAAA | GAGGTGCAGCTGTTGGAGTCTGGGGGAGGCTTGCTACAGCCGGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCTTCTATTAGTGGTCGTGGTGGTAGCACATTCCACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGTGAAGGGGGAGCTACTTGAGGACTACTACTTCTACGGTATGGACGTCTGGGGCCAAGGGACCACGGTCACCGTCTCCTCA |
| | | | SEQ ID NO: 24677 | SEQ ID NO: 28683 |

FIGURE 50

| | | | | |
|---|---|---|---|---|
| | | AA | DIQMTQSPSSLSASVGDRVTITCRTSQDIRSDLGWYQQKPGKAPKRLIYAASSLQSGVPSRFSGSGSGTEFTLTISSLQPEDFAIYYCLQHTIYPPTFGGGTKVEIK | EVQLLESGGGLLQPGGSLRLSCAASGFTFSSYAMSWVRQAPGKGLEWVSSISGRGGSTFHADSVKGRFTISRDNSKNTLYLQMNSLRAEDTAVYYCVKGELLEDYYFYGMDVWGQGTTVTVSS |
| | | | SEQ ID NO: 24678 | SEQ ID NO: 28684 |
| iPS:393104 | 21-225_33A7 | NA | GACATCCAGATGACCCAGTCTCCATCCTCCCTGTCTGCATCTGTAGGAGACAGAGTCACCATCACTTGCCGGGCAAGTCAGGACATTAGAAGTGATTTAGGCTGGTATCAGCAGAAACCAGGGAAAGCCCCTAAGCGCCTGATCTATGTTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAGCATATTACTGTCTACAGCATACTATTTACCCTCCTACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTGTGCCATGAGCTGGGTCCGCCAGGCTCCAGGGAAGGGGCTGGAGTGGGTCTCCGCTATTAGTGGTCGTGGTGGTAGCACATTCCACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGTGAAAGGGGAACTACTAGAGGACTACTACTTCTACGGTATGGACGTCTGGGGCCAAGGGACCACGGTCACCGTCTCCTCA |
| | | | SEQ ID NO: 24679 | SEQ ID NO: 28685 |
| | | AA | DIQMTQSPSSLSASVGDRVTITCRASQDIRSDLGWYQQKPGKAPKRLIYVASSLQSGVPSRFSGSGSGTEFTLTISSLQPEDFAAYYCLQHTIYPPTFGGGTKVEIK | EVQLLESGGGLVQPGGSLRLSCAASGFTFSSCAMSWVRQAPGKGLEWVSAISGRGGSTFHADSVKGRFTISRDNSKNTLYLQMNSLRAEDTAVYYCVKGELLEDYYFYGMDVWGQGTTVTVSS |
| | | | SEQ ID NO: 24680 | SEQ ID NO: 28686 |

FIGURE 50

| | | | | |
|---|---|---|---|---|
| **iPS:393106** | **21-225_34A6** | NA | GACATCCAGATGACCCAGTCTCCATCCTCCCTGTCTGCATCTGTAGGAGACAGAGTCACCATCACTTGCCGGACAAGTCAGGACATCAGAAATGATTTAGGCTGGTATCAGCAGAAACCAGGGAAAGCCCCTAAGCGCCTGATCTTTGCTGCATCCAGTTTGCAAAGTGGGGTCCCATCAAGATTCAGCGGCAGTGGATCTGGGACAGAATTCACTCTCACAGTCAGCAGCCTGCAGCCTGAAGATTTTGCAACTTATTACTGTCTACAGCATAATAGTTACCCTCCTACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAACAACTATGCCATGAACTGGGTCCGCCAGGCTCCAGGGAAGGGGCTGGAGTGGGTCTCAGCTATTAGTCGTCGTGGTGGTAGCACATTCTACGCAGACTCCGTGAAGGGCCGGTTCACCATCTCCAGAGACAATTCCAAGAACACTCTGTATCTCCAAATGAACAGCCTGAGAGCCGAGGACACGGCCGTATATTACTGTGCGAAAGGGGAGCTACTAGAGGACTACTACTACTTCGCTATGGACGTCTGGGGCCAAGGGACCACGGTCACCGTCTCCTCA |
| | | | SEQ ID NO: 24681 | SEQ ID NO: 28687 |
| | | AA | DIQMTQSPSSLSASVGDRVTITCRTSQDIRNDLGWYQQKPGKAPKRLIFAASSLQSGVPSRFSGSGSGTEFTLTVSSLQPEDFATYYCLQHNSYPPTFGGGTKVEIK | EVQLLESGGGLVQPGGSLRLSCAASGFTFNNYAMNWVRQAPGKGLEWVSAISRRGGSTFYADSVKGRFTISRDNSKNTLYLQMNSLRAEDTAVYYCAKGELLEDYYYFAMDVWGQGTTVTVSS |
| | | | SEQ ID NO: 24682 | SEQ ID NO: 28688 |
| **iPS:393108** | **21-225_34G11** | NA | GACATCCAGATGACCCAGTCTCCATCCTCCCTGTCTGCATCTGTGGGAGACAGAGTCACCATCACTTGCCGGGCAAGTCAGAACATTAACAGGTATTTAAATTGGTATCAGCAGAAACCAGGGAAAGCCCCTAAGCTCCTGATCTATGGTGCATCCAGTTTGCAAAGTGGGGTCCCATCAAGGTTCAGTGGCAGTGGATCTGGGACAGATTTCACTCTCACCATCAGCACTCTGCAACCTGAAGATTTTGCAACTTACTACTGTCAACAGACTTACATTACCCCGCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAGGGGCTTGAGTGGATGGGATGGATCAACCCTAACAGTGGTGGCACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGAGATATTAGTAATTTCAGCAGCTGGTACGATTACTACGCTATGGACGTCTGGGGCCAAGGGACCACGGTCACCGTCTCCTCA |
| | | | SEQ ID NO: 24683 | SEQ ID NO: 28689 |

FIGURE 50

| | | | | |
|---|---|---|---|---|
| | | AA | DIQMTQSPSSLSASVGDRVTITCRASQNINRYLNWYQQKPGKAPKLLIYGASSLQSGVPSRFSGSGSGTDFTLTISTLQPEDFATYYCQQTYITPLTFGGGTKVEIK | QVQLVQSGAEVKKPGASVKVSCKASGYTFTGYYMHWVRQAPGQGLEWMGWINPNSGGTNYAQKFQGRVTMTRDTSISTAYMELSRLRSDDTAVYYCARDISNFSSWYDYYAMDVWGQGTTVTVSS |
| | | | SEQ ID NO: 24684 | SEQ ID NO: 28690 |
| **iPS:393110** | **21-225_35B7** | NA | GACATCCAGATGACCCAGTCTCCATCCTCCCTGTCTGCATCTGTAGGAGACAGAATCACCATCACTTGCCGGGCAAGTCAGGACATTAGAAGTGATTTAGGCTGGTATCAGCAGAAACCAGGGAAAGCCCCTGAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATACTATTTACCCTCCTACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCCGCTATTAGTGGTCGTGGTGGTAGCACATTCCACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGTGAAAGGGGAGCTACTAGAGGACTACTACTTCTACGGTATGGACGTCTGGGGCCAAGGGGCCACGGTCACCGTCTCCTCA |
| | | | SEQ ID NO: 24685 | SEQ ID NO: 28691 |
| | | AA | DIQMTQSPSSLSASVGDRITITCRASQDIRSDLGWYQQKPGKAPERLIYAASSLQSGVPSRFSGSGSGTEFTLTISSLQPEDFATYYCLQHTIYPPTFGGGTKVEIK | EVQLLESGGGLVQPGGSLRLSCAASGFTFSSYAMSWVRQAPGKGLEWVSAISGRGGSTFHADSVKGRFTISRDNSKNTLYLQMNSLRAEDTAVYYCVKGELLEDYYFYGMDVWGQGATVTVSS |
| | | | SEQ ID NO: 24686 | SEQ ID NO: 28692 |

FIGURE 50

| | | | | |
|---|---|---|---|---|
| **iPS:393112** | **21-225_33G1** | NA | GACATCCAGATGACCCAGTCTCCATCTTCCGTGTCTGTGTCTGTCGGAGACAGAGTCACCATCACTTGTCGGGCGAGTCAGGGTATTAGCAGGTGGTTAGCCTGGTATCAGCAGAAACCTGGGAAAGCCCCAAAGCTCCTGATCTATGGTGCATACAGTCTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGGCTAACAGTTTCCCATTCACTTTCGGCCCTGGGACCAAAGTGGATATCAAA | CAGGTGCAGTTGGCGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGGTGGATCAGCCCTAACAATGGTGGCACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGAGATGGAACTGGGTCCTTTGACTACTGGGGCCAGGGAACCCTGGTCACCGTCTCCTCA |
| | | | SEQ ID NO: 24687 | SEQ ID NO: 28693 |
| | | AA | DIQMTQSPSSVSVSVGDRVTITCRASQGISRWLAWYQQKPGKAPKLLIYGAYSLQSGVPSRFSGSGSGTDFTLTISSLQPEDFATYYCQQANSFPFTFGPGTKVDIK | QVQLAQSGAEVKKPGASVKVSCKASGYTFTGYYMHWVRQAPGQGLEWMGWISPNNGGTNYAQKFQGRVTMTRDTSISTAYMELSRLRSDDTAVYYCARDGTGSFDYWGQGTLVTVSS |
| | | | SEQ ID NO: 24688 | SEQ ID NO: 28694 |
| **iPS:393114** | **21-225_33G12** | NA | GACATCCAGATGACCCAGTCTCCATCCTCCCTGTCTGCATCTGTAGGAGACAGAGTCACCATCACTTGCCGGGCAAGTCAGAGCATTAGCAACTATTTAAATTGGTATCAACAGCAAACAGGGAAAGCCCCTAAGTTCCTGATTTATGCTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACAGTACCCCATTCACTTTCGGCCCTGGGACCAAAGTGGATATCAAA | GAGGTGCAGCTGTTGGAGTCTGGGGGAGACATGGTCCAGCCTGGGGGGTCCCTGAGACTCTCCTGTGCAGCCTCCGGATTCACCTTTAGCAGCTATGCCATGAGCTGGGTCCGCCAGGCTCCAGGGAAGGGGCTGGAGTGGGTCTCAGTTATTAGTGGTAGTGGTGGTAGCTCATTCTACGCAGACTCCGTGAAGGGCCGGTTCACCATCTCCAGAGACAATTCCAAGAACACGCTGTATCTGCAAATGAACAGCCTGAGGGCCGAGGACACGGCCGTATATTACTGTGCGAAAGATCGGGTGAGAGCTCATGATGGTTTTGATATCTGGGGCCAAGGGACAATGGTCACCGTCTCTTCA |
| | | | SEQ ID NO: 24689 | SEQ ID NO: 28695 |

FIGURE 50

| | | | | |
|---|---|---|---|---|
| | | AA | DIQMTQSPSSLSASVGDRVTITCRASQSISNYLN WYQQQTGKAPKFLIYAASSLQSGVPSRFSGSGS GTDFTLTISSLQPEDFATYYCQQSYSTPFTFGPGT KVDIK | EVQLLESGGDMVQPGGSLRLSCAASGFTFSSYAMS WVRQAPGKGLEWVSVISGSGGSSFYADSVKGRFTI SRDNSKNTLYLQMNSLRAEDTAVYYCAKDRVRAH DGFDIWGQGTMVTVSS |
| | | | SEQ ID NO: 24690 | SEQ ID NO: 28696 |
| iPS:393116 | 21-225_34G7 | NA | GACATCCAGATGACCCAGTCTCCATCTTCCGT GTCTGCATCTGTAGGAGACAGAGTCACCATCA CTTGTCGGGCGAGTCAGCTTATTAGCAAGTGG TTAGCCTGGTATCAGCAGAAACCAGGGAAAG CCCCTAAACTCCTGATCTATGCTGCATCCAGTT TGCAAAGTGGGGTCCCATTAAGGTTCAGCGGC AGTGGATCTGGGACAGATTTCACTCTCACCAT CAGCAGCCTGCAGCCTGAAGATTTTGCAACTT ACTATTGTCAACAGGCTAACAGTTTCCCATTC ACTTTCGGCCCTGGGACCAAAGTGGATATCAA A | CAGGTGCAGCTGGTGCAGTCTGGGGCTGAGGTG AAGAAGCCTGGGGCCTCAGTGAAGGTCTCCTGC AGGGCTTCTGGATACACCTTCACCGACTACCATA TTCACTGGGTGCGACAGGCCCCTGGACAAGGGCT TGAGTGGATGGGATGGATCAACCCTAACAATGGT GGCACACACTATGCACAGAAGTTTCAGGGCAGG GTCACCATGACCAGGGACACGTCCATCAGCACA GCCTACATGGAGCTGAGCAGGCTGAGATCTGAC GACACGGCCGTGTATTATTGTGCGAGAGATGGAA CTGGGTCCTTTGACTACTGGGGCCAGGGAAACCT GGTCACCGTCTCCTCA |
| | | | SEQ ID NO: 24691 | SEQ ID NO: 28697 |
| | | AA | DIQMTQSPSSVSASVGDRVTITCRASQLISKWLA WYQQKPGKAPKLLIYAASSLQSGVPLRFSGSGS GTDFTLTISSLQPEDFATYYCQQANSFPFTFGPGT KVDIK | QVQLVQSGAEVKKPGASVKVSCRASGYTFTDYHIH WVRQAPGQGLEWMGWINPNNGGTHYAQKFQGRV TMTRDTSISTAYMELSRLRSDDTAVYYCARDGTGS FDYWGQGNLVTVSS |
| | | | SEQ ID NO: 24692 | SEQ ID NO: 28698 |

FIGURE 50

| | | | | |
|---|---|---|---|---|
| **iPS:393118** | **21-225_34H11** | NA | GACATCCAGATGACCCAGTCTCCATCCTCCCTGTCTGCATCTGTCGGAGACAGAGTCACCATCACTTGCCGGGCAAGTCAGGACATTAGAAATGATTTAGGCTGGTATCAGCAGAAACCAGGGAAAGCCCCTAAGCGCCTGATCTATGCTACATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCTCCTACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAACTGGGTCCGCCAGGCTCCAGGGGAGGGGCTGGAGTGGGTCTCCGCTATTAGTGGTCGTGGTGGTAGCACATTCTACGCAGACTCCGTGAAGGGCCGGTTCACCATCTCCAGAGACAATTCCAAGAACACGCTGTTTCTGCAAATGAACAGCCTGAGAGCCGAGGACACGGCCGTTTATTACTGTGCGAAAGGGGAGCTACTAGAGGACTACTACTACTACGGTATGGACGTCTGGGGCCAAGGGACCACGGTCACCGTCTCCTCA |
| | | | SEQ ID NO: 24693 | SEQ ID NO: 28699 |
| | | AA | DIQMTQSPSSLSASVGDRVTITCRASQDIRNDLGWYQQKPGKAPKRLIYATSSLQSGVPSRFSGSGSGTEFTLTISSLQPEDFATYYCLQHNSYPPTFGGGTKVEIK | EVQLLESGGGLVQPGGSLRLSCAASGFTFSSYAMNWVRQAPGEGLEWVSAISGRGGSTFYADSVKGRFTISRDNSKNTLFLQMNSLRAEDTAVYYCAKGELLEDYYYYGMDVWGQGTTVTVSS |
| | | | SEQ ID NO: 24694 | SEQ ID NO: 28700 |
| **iPS:393120** | **21-225_35H8** | NA | GACATCCAGATGACCCAGTCTCCATCCTCACTGTCTGCATCTGTAGGAGACAGAGTCACCATCACTTGTCGGGCGAGTCAGGCCATTAGTAATTATTTAGCCTGGTTTCAGCAGAAACCAGGGAAAGCCCCTAAGTCCCTGATCTATGGTGCGTCCGGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATAATAGTTACCCATTCACTTTCGGCCCTGGGACCAAAGTGGATTTCAAA | GAGGTTCAACTGGTGGAGTCTGGGGGGGGCCTGGTCAAGCCTGGGGGGTCCCTGAGACTCTCCTGTGCAGCCTCTGGATTCACCTTCAGTAGTTATAGCATGAACTGGGTCCGCCAGGCTCCAGGGAAGGGGCTGGAGTGGGTCTCGTCCATTAGTGGTACTGGTAGTTTCATATACTACGCAGACTCAGTGAAGGGCCGATTCACCATCTCCAGAGACAACGCCAAGAAATCAGTGTATCTGCAAATGAACAGCCTGAGAGCCGAGGACACGGCTGTGTATTACTGTGCGAGAGTCTCTGGCTTTGACTACTGGGGCCAGGGAACCCTGGTCACCGTCTCCTCA |
| | | | SEQ ID NO: 24695 | SEQ ID NO: 28701 |

FIGURE 50

| | | | | |
|---|---|---|---|---|
| | | AA | DIQMTQSPSSLSASVGDRVTITCRASQAISNYLAWFQQKPGKAPKSLIYGASGLQSGVPSKFSGSGSGTDFTLTISSLQPEDFATYYCQQYNSYPFTFGPGTKVDFK | EVQLVESGGGLVKPGGSLRLSCAASGFTFSSYSMNWVRQAPGKGLEWVSSISGTGSFIYYADSVKGRFTISRDNAKKSVYLQMNSLRAEDTAVYYCARVSGFDYWGQGTLVTVSS |
| | | | SEQ ID NO: 24696 | SEQ ID NO: 28702 |
| **iPS:393122** | **21-225_33B2** | NA | GACATCCAGATGACCCAGTCTCCATCCTCCCTGTCTACATCTGTCGGAGACAAAGTCACCATCACTTGCCGGGCAAGTCAGAGCATTATCAGCTATTTAAATTGGTATCAGCAGAAACCAGGGAAAGCCCCTAAACTCCTGATCTATGTTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACAGTACCCCGTACACTTTCGGCGGGGGGACTAAGGTGGAGATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGCATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAATAGCAGCTCGTACTACTTTGACTACTGGGGCCAGGGAACCCTGGTCACCGTCTCCTCA |
| | | | SEQ ID NO: 24697 | SEQ ID NO: 28703 |
| | | AA | DIQMTQSPSSLSTSVGDKVTITCRASQSIISYLNWYQQKPGKAPKLLIYVASSLQSGVPSRFSGSGSGTDFTLTISSLQPEDFATYYCQQSYSTPYTFGGGTKVEIK | QVQLVESGGGVVQPGRSLRLSCAASGFTFSNYGMHWVRQAPGKGLEWVAVIWHDGSNKYYADSVKGRFTISRDNSKNTLYLQMNSLRAEDTAVYYCARENSSSYYFDYWGQGTLVTVSS |
| | | | SEQ ID NO: 24698 | SEQ ID NO: 28704 |

FIGURE 50

| | | | | |
|---|---|---|---|---|
| **iPS:393124** | **21-225_33G7** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACCCTCACAATCAGCAGCCTGCAGCCTGAAGATTTTGCAACATATTACTGTCTACAGCATTATAGTTACCCTCCTACTTTCGGCGGAGGGACCAAGGTGGAGATCAAA | GAGGTTCAATTGTTGGAGTCTGGGGGAGGCTTGGTACAGCCTGGGGGGTCCCTGAGACTCTCCTGTGCAGCCTCTGGATTCACCTTTAGCAACTATGCCATGAGCTGGGTCCGCCAGGCTCCAGGGAAGGGGCTGGAGTGGGTCTCAGCTATTAGTCGTCGTGGTGGTAGCACATTCTACGCAGACTCCGTGAAGGGCCAGTTCACCATTTCCAGAGACAATTCCAAGAACACGCTGTATCTGCAAATGAACAGCCTGAGAGCCGAGGACACGGCCGTGTATTACTGTGCGAAAGGGGAGCTACTAGAGGACTACTCCTACTACGGTATGGACGTCTGGGGCCAGGGGACCACGGTCACCGTCTCCTCA |
| | | | SEQ ID NO: 24699 | SEQ ID NO: 28705 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPPTFGGGTKVEIK | EVQLLESGGGLVQPGGSLRLSCAASGFTFSNYAMSWVRQAPGKGLEWVSAISRRGGSTFYADSVKGQFTISRDNSKNTLYLQMNSLRAEDTAVYYCAKGELLEDYSYYGMDVWGQGTTVTVSS |
| | | | SEQ ID NO: 24700 | SEQ ID NO: 28706 |
| **iPS:393126** | **21-225_35D1** | NA | GACATCCAGATGACCCAGTCTCCATCCTCCCTGTCTGCATCTGTAGGAGACAGAGTCACCATCACTTGCCGGGCAAGTCAGGACATTAGAAGTGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATACTATTTACCCTCCCACTTTCGGCGGGGGGACCAAGGTGGAGATCAAA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCCGCTATTAGTGGTCGTGGTGGTAGCACATTCCACGCAGACTCCGTGAAGGGCCGGTTCACCATCTCCAGAGACAATTCCAACAACACGCTGTATCTGCAAATGAACAGCCTGAGAGCCGAGGACACGGCCGTATATTACTGTGTGAAAGGGGAGTTACTAGAGGACTACTACTTCTACGGTATGGACGTCTGGGGCCAAGGGGCCACGGTCACCGTCTCCTCA |
| | | | SEQ ID NO: 24701 | SEQ ID NO: 28707 |

FIGURE 50

| | | | | |
|---|---|---|---|---|
| | | AA | DIQMTQSPSSLSASVGDRVTITCRASQDIRSDLGWYQQKPGKAPKRLIYAASSLQSGVPSRFSGSGSGTEFTLTISSLQPEDFATYYCLQHTIYPPTFGGGTKVEIK | EVQLLESGGGLVQPGGSLRLSCAASGFTFSSYAMSWVRQAPGKGLEWVSAISGRGGSTFHADSVKGRFTISRDNSNNTLYLQMNSLRAEDTAVYYCVKGELLEDYYFYGMDVWGQGATVTVSS |
| | | | SEQ ID NO: 24702 | SEQ ID NO: 28708 |
| **iPS:393128** | **21-225_35F11** | NA | GACATCCAGATGACCCAGTCTCCATCCTCCCTGTCTGCATCTGTAGGAGACAGAGTCACCATCACTTGCCGGGCAAGTCAGGACATTAGAAGTGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATACTGTTTACCCTCCTACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCCGCTATTAGTGGTCGTGGTGGTAGCACATTCCACGCAGACTCCATGAAGGGCCGGTTCACCATCTCCAGAGACAATTCCAAGAACACGCTGTATCTGCAAATGAACAGCCTGAGAGCCGAGGACACGGCCGTATATTACTGTGTGAAAGGGGAGCTACTAGAGGACTACTACTTCTACGGTATGGACGTCTGGGGCCAAGGGACCACGGTCACCGTCTCCTCA |
| | | | SEQ ID NO: 24703 | SEQ ID NO: 28709 |
| | | AA | DIQMTQSPSSLSASVGDRVTITCRASQDIRSDLGWYQQKPGKAPKRLIYAASSLQSGVPSRFSGSGSGTEFTLTISSLQPEDFATYYCLQHTVYPPTFGGGTKVEIK | EVQLLESGGGLVQPGGSLRLSCAASGFTFSSYAMSWVRQAPGKGLEWVSAISGRGGSTFHADSMKGRFTISRDNSKNTLYLQMNSLRAEDTAVYYCVKGELLEDYYFYGMDVWGQGTTVTVSS |
| | | | SEQ ID NO: 24704 | SEQ ID NO: 28710 |

FIGURE 50

| | | | | |
|---|---|---|---|---|
| iPS:393130 | 21-225_33C2 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGTGAAAGCCCCTAAGCGCCTGATCTATGCTGCACCCAGTTTGCAAAGTGGGGTCCCATCAAGGTTCAGCGGCAGTGGATCTGGGACAGAATTCACTCTCACAATCAGCAGCCTGCAGCCTGAACATTTTGCAACTTATTACTGTCTACAGCATAATAGTTACCCGTGGACGTTCGGCCAGGGGACCAAGGTGGAAATCAAA | GAGGTGCAACTGGTGGAGTCTGGGGGAGGCCTGGTCAAGCCGGGGGGGTCCCTGAGACTCTCCTGTGCAGCCTCTGGATTCACCTTCAGTAGCTATACCATGAACTGGGTCCGCCAGGCTCCAGGGAAGGGGCTGCAGTGGGTCTCATCCATTAGTGGTAGTAGTAGTTACATATACTACGCGGACTCAGTGAAGGGCCGATTCACCATCTCCAGAGACAACGCCAAGAACTCACTGTTTCTGCAAATGAACAGCCTGAGAGCCGAGGACACGGCTATCTTTTACTGTGCGCGAGATCGGGGGGGGACCTGGGGCCAGGGAACCCTGGTCACCGTCTCCTCA |
| | | | SEQ ID NO: 24705 | SEQ ID NO: 28711 |
| | | AA | DIQMTQSPSSLSASVGDRVTITCRASQGIRNDLGWYQQKPVKAPKRLIYAAPSLQSGVPSRFSGSGSGTEFTLTISSLQPEHFATYYCLQHNSYPWTFGQGTKVEIK | EVQLVESGGGLVKPGGSLRLSCAASGFTFSSYTMNWVRQAPGKGLQWVSSISGSSSYIYYADSVKGRFTISRDNAKNSLFLQMNSLRAEDTAIFYCARDRGGTWGQGTLVTVSS |
| | | | SEQ ID NO: 24706 | SEQ ID NO: 28712 |
| iPS:393132 | 21-225_33H7 | NA | GACATCCAGATGACCCAGTCTCCATCTTCCGTGTCTGCATCTGTAGGCGACAGAGTCACCATCACTTGTCGGGCGAGTCAGGGTATTAGCCGGTGGTTAGCCTGGTATCAGCAGAAAGTGGGGAAAGTCCCCAAACTCCTGATCTATGCTGCATCCAGGTTGCAAAGTGGGGTCCCATCAAGGTTCAGCGGCAGTGGATCTGGGACAGATTTCACTCTCACCATCGGCAGCCTGCAGCCTGAAGATTTTGCAACTTACTATTGTCAACAGGCTAACATTTTCCCGTTCACTTTCGGCCCTGGGACCAAAGTGGATCTCAAA | CAGGTGCAGCTGGTGCAGTCTGGGGCTGACGTGAAGAAGCCTGGGGCCTCAGTGAAGGTCTCCTGCAAGGCTTCTGGATACACCTTCACCGACTACCATATACACTGGGTGCGACAGGCCCCTGGACAAGGGCTTGAGTGGATGGGATGGATCAACCCTAACAATGGTGGCACACACTATGCACAGGAGTTTCAGGGCAGGGTCACCATGACCAGGGACACGTCCATCAGCACAGCCTACATGGAACTGAGTAGCCTGAGATCTGACGACACGGCCGTGTATCACTGTGCGAGAGATGGAACTGGGTCCTTTGACTACTGGGGCCAGGGAACCCTGGTCACCGTCTCCTCA |
| | | | SEQ ID NO: 24707 | SEQ ID NO: 28713 |

FIGURE 50

| | | | | |
|---|---|---|---|---|
| | | AA | DIQMTQSPSSVSASVGDRVTITCRASQGISRWLAWYQQKVGKVPKLLIYAASRLQSGVPSRFSGSGSGTDFTLTIGSLQPEDFATYYCQQANIFPFTFGPGTKVDLK | QVQLVQSGADVKKPGASVKVSCKASGYTFTDYHIHWVRQAPGQGLEWMGWINPNNGGTHYAQEFQGRVTMTRDTSISTAYMELSSLRSDDTAVYHCARDGTGSFDYWGQGTLVTVSS |
| | | | SEQ ID NO: 24708 | SEQ ID NO: 28714 |
| iPS:393134 | 21-225_34C2 | NA | GACATCCAGATGACCCAGTCTCCATCCTCCCTGTCTACATTTGTAGGAGACAGAGTCACCATTACTTGTCGGGCAAGTCAGAGAATTATCAGCTATTTAAATTGGTTTCAGCAGAAACCAGGGAAAGCCCCTAAACTCCTGATCTATGTTGCATCCAGTTTGCAAAGTGGGGTCCCGTCAAGGTTCAGTGGCAGTGGATCTGGGACATATTTCACTCTCACCATCAGCAGTCTGCAACCTGAAGATTTTGCGACTTACTACTGTCAACAGAGTTACAGTACCCCGTACACTTTCGGCGGAGGGACCAAGATGGAGATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAACTATGTCATGCACTGGGTCCGCCAGGCTCCAGGCAAGGGGCTGGAGTGGGTGGCACTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAATAGCAGCTCGTACTACTTTGACTACTGGGGCCAGGGAACCCTGGTCACCGTCTCCTCA |
| | | | SEQ ID NO: 24709 | SEQ ID NO: 28715 |
| | | AA | DIQMTQSPSSLSTFVGDRVTITCRASQRIISYLNWFQQKPGKAPKLLIYVASSLQSGVPSRFSGSGSGTYFTLTISSLQPEDFATYYCQQSYSTPYTFGGGTKMEIK | QVQLVESGGGVVQPGRSLRLSCAASGFTFSNYVMHWVRQAPGKGLEWVALIWYDGSNKYYADSVKGRFTISRDNSKNTLYLQMNSLRAEDTAVYYCARENSSSYYFDYWGQGTLVTVSS |
| | | | SEQ ID NO: 24710 | SEQ ID NO: 28716 |

FIGURE 50

| | | | | |
|---|---|---|---|---|
| **iPS:393136** | **21-225_34D8** | NA | GACATCCAGATGACCCAGTCTCCATCCTCCCTGTCTACATCTGTAGGAGACAGAGTCACCATCACTTGCCGGGCAAGTCAGATCATTATCAGCTATTTAAATTGGTATCAGCAGAAACCAGGGAAAGCCCCTAAACTCCTGATCTTTGTTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACAGTACCCCGTACACTTTCGGCGGGGGGACCAAGGTGGAGATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAATAGCAGCTCGTACTACTTTGACTACTGGGGCCAGGGAACCCTGGTCACCGTCTCCTCA |
| | | | SEQ ID NO: 24711 | SEQ ID NO: 28717 |
| | | AA | DIQMTQSPSSLSTSVGDRVTITCRASQIIISYLNWYQQKPGKAPKLLIFVASSLQSGVPSRFSGSGSGTDFTLTISSLQPEDFATYYCQQSYSTPYTFGGGTKVEIK | QVQLVESGGGVVQPGRSLRLSCAASGFTFSNYGMHWVRQAPGKGLEWVAVIWYDGSNKYYADSVKGRFTISRDNSKNTLYLQMNSLRAEDTAVYYCARENSSSYYFDYWGQGTLVTVSS |
| | | | SEQ ID NO: 24712 | SEQ ID NO: 28718 |
| **iPS:393138** | **21-225_35E3** | NA | GACATCCAGATGACCCAGTCTCCATCCTCCCTGTCTGCATCTGTAGGAGACAGAGTCACCATCACTTGCCAGGCGAGTCAGGACATTTTCAACTATTTAAATTGGTATCAGCAGAAACCAGGAAAAGCCCCTAACCTCCTGATCTACGATGCCTCCAATTTGGAAACAGGGGTCCCATCAAGGTTCAGTGGAAGTGGATCTGGGACAGATTTTACTTTCACCATCAGCAGCCTGCAGCCTGAAGATATTGCGACATATTTCTGTCAACAGTATGATAATCTCCCGATCACCTTCGGCCAAGGGACACGACTGGATATTAGA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAGTGGGTGGCAGTTATATCATATGGTGGAAGTAATAAATACTATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTACTGTGTGAGAAGGGGGTATAGCAGTGGAGGCTACGGTATGGACGTCTGGGGCCAAGGGACCACGGTCACCGTCTCCTCA |
| | | | SEQ ID NO: 24713 | SEQ ID NO: 28719 |

FIGURE 50

| | | | | |
|---|---|---|---|---|
| | | AA | DIQMTQSPSSLSASVGDRVTITCQASQDIFNYLNWYQQKPGKAPNLLIYDASNLETGVPSRFSGSGSGTDFTFTISSLQPEDIATYFCQQYDNLPITFGQGTRLDIR | QVQLVESGGGVVQPGRSLRLSCAASGFTFSSYGMHWVRQAPGKGLEWVAVISYGGSNKYYADSVKGRFTISRDNSKNTLYLQMNSLRAEDTAVYYCVRRGYSSGGYGMDVWGQGTTVTVSS |
| | | | SEQ ID NO: 24714 | SEQ ID NO: 28720 |
| iPS:393140 | 21-225_35H12 | NA | GACATCCAGATGACCCAGTCTCCATCTTCCGTGTCTGCATCTGTAGGAGACAGAGTCACCATCACTTGTCGGGCGAGTCAGGGTATTAGCAGATGGTTAGCCTGGTATCAGCAGAAACCAGGGAAAGCCCCTGAACTCCTGATCTATGCTGCATCCCGTTTGCAAAGTGGGATCCCATCCAGGTTCAGCGGCAGTGGATCTGGGACAGACTTCACTCTCACCATCAGCAGCCTGCAGCCTGAAGATTTTGCAACTTACTATTGTCAACAGGCTAACAGTTTCCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGACTACTATATACACTGGGTGCGACAGGCCCCTGGACAAGGGCTTGAGTGGATGGGATGGATCAACCCTAATAGGGGTGGCACAAACTATGCACAGAAGTTTCAGGGCAGGGTCACCATGACCAGGGACACGTCCATCAGCACAGCCTACATGGAACTGAGCAGGCTGAGATCTGACGACACGGCCGTGTATTACTGTGCGAGAGATGGAACTGGGTCCTTTGACTACTGGGGCCAGGGAACCCTGGTCACCGTCTCCTCA |
| | | | SEQ ID NO: 24715 | SEQ ID NO: 28721 |
| | | AA | DIQMTQSPSSVSASVGDRVTITCRASQGISRWLAWYQQKPGKAPELLIYAASRLQSGIPSRFSGSGSGTDFTLTISSLQPEDFATYYCQQANSFPFTFGPGTKVDIK | QVQLVQSGAEVKKPGASVKVSCKASGYTFTDYYIHWVRQAPGQGLEWMGWINPNRGGTNYAQKFQGRVTMTRDTSISTAYMELSRLRSDDTAVYYCARDGTGSFDYWGQGTLVTVSS |
| | | | SEQ ID NO: 24716 | SEQ ID NO: 28722 |

FIGURE 50

| | | | | |
|---|---|---|---|---|
| **iPS:393142** | **21-225_33A3** | NA | GACATCCAGATGACCCAGTCTCCATCCTCACTGTCTGCATCTGTAGGAGACAGAGTCACCATCACTTGTCGGGCGAGTCAGGGCATTAACAATTATTTAGCCTGGTTTCAACAGAAACCAGGGAAAGCCCCTAAGTCCCTGATCTACGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTTTAATAGTTACCCTCCGACGTTCGGCCAAGGGACCAAGGTGGAAATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATGGCATGAACTGGGTCCGCCAGGCTCCAGGGAAGGGGCTGGAGTGGGTCTCATCCATTAGTGGTAGTAGTACTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAACAAACGGTATGGACGTCTGGGGCCAAGGGACCACGGTCACCGTCTCCTCA |
| | | | SEQ ID NO: 24717 | SEQ ID NO: 28723 |
| | | AA | DIQMTQSPSSLSASVGDRVTITCRASQGINNYLAWFQQKPGKAPKSLIYAASSLQSGVPSKFSGSGSGTDFTLTISSLQPEDFATYYCQQFNSYPPTFGQGTKVEIK | EVQLVESGGGLVKPGGSLRLSCAASGFTFSSYGMNWVRQAPGKGLEWVSSISGSSTYIYYADSVKGRFTISRDNAKNSLYLQMNSLRAEDTAVYYCARTNGMDVWGQGTTVTVSS |
| | | | SEQ ID NO: 24718 | SEQ ID NO: 28724 |
| **iPS:393144** | **21-225_34D2** | NA | GATATTGTGATGACCCAGACTCCACTCTCTCTGTCCGTCACCCCTGGACAGCCGGCCTCCATCTCCTGCAAGTCTAGTCAGAGCCTCCTGCATAGTGATGGAAAGACCTATTTGTATTGGTACCTGCAGAAGCCAGGCCAGCCTCCACAGCTCCTGATCTATGAAGTTTCCAACCGGTTCTCTGGAGTGCCAGATAGGTTCAGTGGCAGCGGGTCAGGGACAGATTTCACACTAAAAATCAGCCGGGTGGAGGCTGAGGATGTTGGGGTTTATTACTGCATGCAAAGTAAACAGCTTCCTCCTTTCGGCGGAGGGACCAAGGTGGAGATCAGA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGACATTAACTGGGTGCGACAGGCCACTGGACAAGGGCTTGAGTGGGTGGGATGGCTGCACCCTAACAGTGGTACCACAGGCTTTGCACAGAAGTTCCGGGGCAGAGTCACCATGACCAGAAACACCTCCATAAGCACAGCCTACTTGGAACTGAGCAGCCTGAGATCTGAGGACACGGCCGTGTATTACTGTGCGAGCAGCAGTGGCTGGTACTTTTTTGACTACTGGGGCCAGGGAACCCTGGTCACCGTCTCCTCA |
| | | | SEQ ID NO: 24719 | SEQ ID NO: 28725 |

FIGURE 50

| | | | | |
|---|---|---|---|---|
| | | AA | DIVMTQTPLSLSVTPGQPASISCKSSQSLLHSDGKTYLYWYLQKPGQPPQLLIYEVSNRFSGVPDRFSGSGSGTDFTLKISRVEAEDVGVYYCMQSKQLPPFGGGTKVEIR | QVQLVQSGAEVKKPGASVKVSCKASGYTFTNYDINWVRQATGQGLEWVGWLHPNSGTTGFAQKFRGRVTMTRNTSISTAYLELSSLRSEDTAVYYCASSSGWYFFDYWGQGTLVTVSS |
| | | | SEQ ID NO: 24720 | SEQ ID NO: 28726 |
| **iPS:393146** | **21-225_34G8** | NA | GACATCCAGATGACCCAGTCTCCATCCTCCCTGTCTGCATCTGTAGGAGACAGAGTCACCATCACTTGCCGGGCAAGTCAGGACATTAGAAGTGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATACTATTTACCCTCCTACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCCGCTATTAGTGGTCGTGGTGGTAGCACATTCCACGCAGACTCCGTGAAGGGCCGGTTCACCATCTCCAGAGACAATTCCAAGAACACGCTGTATCTGCAAATTAACAGCCTGAGAGCCGAGGACACGGCCGTATATTACTGTGTGAAAGGGGAGCTACTAGAGGACTACTACTTCTACGGTATGGACGTCTGGGGCCAAGGGACCACGGTCACCGTCTCCTCA |
| | | | SEQ ID NO: 24721 | SEQ ID NO: 28727 |
| | | AA | DIQMTQSPSSLSASVGDRVTITCRASQDIRSDLGWYQQKPGKAPKRLIYAASSLQSGVPSRFSGSGSGTEFTLTISSLQPEDFATYYCLQHTIYPPTFGGGTKVEIK | EVQLLESGGGLVQPGGSLRLSCAASGFTFSSYAMSWVRQAPGKGLEWVSAISGRGGSTFHADSVKGRFTISRDNSKNTLYLQINSLRAEDTAVYYCVKGELLEDYYFYGMDVWGQGTTVTVSS |
| | | | SEQ ID NO: 24722 | SEQ ID NO: 28728 |

FIGURE 50

| | | | | |
|---|---|---|---|---|
| **iPS:393148** | **21-225_35E5** | NA | GACATCCAGATGATCCAGTCTCCATCCTCCCTGTCTGCATCTGTAGGAGACAGAGTCACCATCACTTACCGGGCAAGTCAGAGCATTAGCAGCTATTTAAATTGGTATCAGCAGAAACCAGCGAAAGCCCCTAAGCTCCACATCTATGGTGCATCCAGTTTCCAAAGTTGGGTCCCATCAAGGTTCAGTGGCAGTGGATCTTCGACAGATTTCACGCTCACCATCATCAGTATGCAACCTGGAGATTATGCAACTTACTACTGTCACCAGAGTTACAATCTCCCGATCACCTTCGGCCAAGGGACCCGACTGGAGATTAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAGTTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGAGAAAGAAGTCTAACGACTACTGGGGCCAGGGAACCCTGGTCACCGTCTCCTCA |
| | | | SEQ ID NO: 24723 | SEQ ID NO: 28729 |
| | | AA | DIQMIQSPSSLSASVGDRVTITYRASQSISSYLNWYQQKPAKAPKLHIYGASSFQSWVPSRFSGSGSSTDFTLTIISMQPGDYATYYCHQSYNLPITFGQGTRLEIK | QVQLVQSGAEVKKPGASVKVSCKASGYTFTSYDINWVRQATGQGLEWMGWMNPNSGNTGYAQKFQGRVTMTRNTSISTAYMELSSLRSEDTAVYYCARKKSNDYWGQGTLVTVSS |
| | | | SEQ ID NO: 24724 | SEQ ID NO: 28730 |
| **iPS:393150** | **21-225_36A5** | NA | GACATCCAGATGACCCAGTCTCCATCCTCCCTGTCTGCATCTGTAGGAGACAGAGTCACCATCACTTGCCGGACAAGTCAGGACATTAGAAATGATTTAGGCTGGTATCAGCAGAAACCAGGGAAAGCCCCTAAGCGCCTGATCTTTGCTGCATCCAGTTTGCAAAGTGGGGTCCCATCAAGATTCAGCGGCAGTGGATCTGGGACAGAATTCACTCTCACAATCAGCAGCCTGCAGCCTGAAGATTTTGCAACTTATTACTGTCTACACCACAATAGTTACCCTCCTAAGTTTGGCGGAGGGATCAAGGTGGAGATCACA | GAGGTGCAGCTGTTGGAGTCTGGGGGAGGCTTGGTACAGCCTGGGGGGTCCCTGAGACTCTCCTGTGCAGCCTCTGGATTCACCTTTAACAACTATGCCATGAACTGGGTCCGCCAGGCTCCAGGGAAGGGGCTGGAGTGGGTCTCAGCTATTAGTCGTCGTGGTGGTAACACATTCTACGCAGACTCCGTGAAGGGCCGGTTCACCATCTCCAGAGACAATTCCAAGAACACGCTGTATCTCCAAATGAACAGCCTGAGAGCCGAGGACACGGCCGTATATTACTGTGCGAAAGGGGAGCTACTAGAGGACTACTACTACTACGCTATGGACGTCTGGGGCCAAGGGACCACGGTCACCGTCTCCTCA |
| | | | SEQ ID NO: 24725 | SEQ ID NO: 28731 |

FIGURE 50

| | | | | |
|---|---|---|---|---|
| | | AA | DIQMTQSPSSLSASVGDRVTITCRTSQDIRNDLGWYQQKPGKAPKRLIFAASSLQSGVPSRFSGSGSGTEFTLTISSLQPEDFATYYCLHHNSYPPKFGGGIKVEIT | EVQLLESGGGLVQPGGSLRLSCAASGFTFNNYAMNWVRQAPGKGLEWVSAISRRGGNTFYADSVKGRFTISRDNSKNTLYLQMNSLRAEDTAVYYCAKGELLEDYYYYAMDVWGQGTTVTVSS |
| | | | SEQ ID NO: 24726 | SEQ ID NO: 28732 |
| iPS:393152 | 21-225_25B3 | NA | GACATCCAGATGACCCAGTCTCCCTCTTCCGTGTCTGCATCTGTAGGAGACAGAGTCACCATCACTTGTCGGGCGAGTCAGGGTATTAGCAGCTGGTTAGCCTGGTATCAGCAGAAACCAGGGAAAGCCCCTAAGCTCCTGATCTATGGTGCATCCAGTTTGCAAAGTGGGGTCCCATCAAGGTTCAGCGCCAGTGGATCTGGGACAGATTTCACTCTCACCATCAGCAGCCTGCAGCCTGAAGATTTTGCGACTTACTGTTGTCAACAGTCTGACAGTTTCCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGCAGCTATGGCATGCACTGGGTCCGCCAGGCTCCAGGCAAGGGGCTGGAGTGGGTGGCAGTTATATGGTATGATGGAAGTAATAAATATTATGCAGACTCCGTGAAGGGCCGATTCACCATCTCCAGAGACAATTCCAAGAACACGCTATATCTACAAATGAACAGCCTGAGAGCCGAGGACACGGCTGTGTATTACTGTGCGAGAGATTCCTCCCCCTACGGTATGGACGTCTGGGGCCAAGGGACCACGGTCACCGTCTCCTCA |
| | | | SEQ ID NO: 24727 | SEQ ID NO: 28733 |
| | | AA | DIQMTQSPSSVSASVGDRVTITCRASQGISSWLAWYQQKPGKAPKLLIYGASSLQSGVPSRFSASGSGTDFTLTISSLQPEDFATYCCQQSDSFPRTFGQGTKVEIK | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARDSSPYGMDVWGQGTTVTVSS |
| | | | SEQ ID NO: 24728 | SEQ ID NO: 28734 |

FIGURE 50

| | | | | |
|---|---|---|---|---|
| **iPS:393166** | **21-225_27G6** | NA | TCCTATGAGCTGACTCAGCCACCCTCAGTGTCCGTGTCCCCAGGACAGACAGCCAGCATCACCTGCTCTGGAGATAAATTGGGGGATAAATATGCTTGCTGGTATCAGCAGAAGCCAGGCCAGTCCCCTGTGTTGGTCATCTATCAAGATAGGAAGCGGCCCTCAGGGATCCCTGAGCGATTCTCTGGCTCCAACTCTGGGAACACAGCCACTCTGACCATCAGCGGGACCCAGACTATGGATGAGGCTGACTATTACTGTCAGGCGTGGGACAGCAGCTCTTATGTGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGGCTATGGCATGCACTGGGTCCGCCAGGCTCCAGGCAAGGGGCTGGAGTGGGTGGCAATTATATGGTATGATGGAAGTAAAAAATACAATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATAGGGTATATTGTAGTAGTACCAGCTGCTCCCCTTACTACTACTACTACGGTATGGACGTCTGGGGCCAAGGGACCACGGTCACCGTCTCCTCA |
| | | | SEQ ID NO: 24729 | SEQ ID NO: 28735 |
| | | AA | SYELTQPPSVSVSPGQTASITCSGDKLGDKYACWYQQKPGQSPVLVIYQDRKRPSGIPERFSGSNSGNTATLTISGTQTMDEADYYCQAWDSSSYVVFGGGTKLTVL | QVQLVESGGGVVQPGRSLRLSCAASGFTFSGYGMHWVRQAPGKGLEWVAIIWYDGSKKYNADSVKGRFTISRDNSKNTLYLQMNSLRAEDTAVYYCARDRVYCSSTSCSPYYYYYGMDVWGQGTTVTVSS |
| | | | SEQ ID NO: 24730 | SEQ ID NO: 28736 |

FIGURE 50

| | | | | |
|---|---|---|---|---|
| **iPS:393168** | **21-225_32B11** | NA | TCCTATGAACTGACTCAGCCACCCTCAGTGTCCGTGTCCCCAGGACAGACAGCCAGCATCACCTGCTCTGGAGATAAATTGGGGGATAAATATGCTTACTGGTATCAGCAGAAGCCAGGCCAGTCCCCTGTGTTGGTCATCTATCAAGATAGTAAGCGGTCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAACAGCACTGTGGTATTCGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAACAGTGATGGCACTAACTATGCACAGAAGTTTCAGGGCAGGGTCACCATGACCAGGGACACGTCCATCAGCACAGCCTACATGGAGCTGAACAGGCTGAGATCTGACGACACGGCCGTGTATTACTGTGCGAGGGGGTTTTACTATGGTTCGGGGAGTTATTATAACGACCTCGACCCCTGGGGCCAGGGAACCCTGGTCACCGTCTCCTCA |
| | | | SEQ ID NO: 24731 | SEQ ID NO: 28737 |
| | | AA | SYELTQPPSVSVSPGQTASITCSGDKLGDKYAYWYQQKPGQSPVLVIYQDSKRSSGIPERFSGSNSGNTATLTISGTQAMDEADYYCQAWDNSTVVFGGGTKLTVL | QVQLVQSGAEVKKPGASVKVSCKASGYTFTGYYMHWVRQAPGQGLEWMGWINPNSDGTNYAQKFQGRVTMTRDTSISTAYMELNRLRSDDTAVYYCARGFYYGSGSYYNDLDPWGQGTLVTVSS |
| | | | SEQ ID NO: 24732 | SEQ ID NO: 28738 |
| **iPS:393172** | **21-225_3B12** | NA | TCCTATGAGCTGAGTCAGCCACCCTCAGTGTCCGTGTCCCCAGGACAGACAGCCAGCATCACCTGCTCTGGAGATAAATTGGGGGAAAAATATGCTTGCTGGTATCAGCAGAAGCCAGGCCAGTCCCCTGTGCTGGTCATCTATCAAGATAGGAAGCGGCCCTCAGGGATCCCTGAGCGATTCTCTGGCTCCAACTCTGGGAACAAAGCCACTCTGACCATCAGCGGGACCCAGGCTATGGATGAGGCTGACTATTACTGTCAGGCGTGGGTCAACAACACTATGATATTCGGCGGAGGGACCAAGCTGACCGTCCTA | CAGGTGCACCTGGTGGAGTCTGGGGGAGGCGTGGTCCAGCCTGGGAGGTCCCTGAGACTCTCCTGTGCAGCCTCTGGATTCACCTTCAGTTACTATGGCATGCACTGGGTCCGCCAGGCTCCAGGCAAGGGGCTGGAGTGGGTGGCAGTTATATCATATGATGGAAGTAATAAATACTATGCAGACTCCGTGAAGGGCCGATTCACCATCTCCAGAGACAATTCCAAGAACACGCTGCATCTGCAAATGAACAGCCTGAGAGCTGAGGACACGGCTGTGTATTACTGTGCGAGAGATCGGAGGGGGGGCTATGGAGTCCCCGATGCTTTTGATATCTGGGGCCAAGGGACAATGGTCACCGTCTCTTCA |
| | | | SEQ ID NO: 24733 | SEQ ID NO: 28739 |

FIGURE 50

| | | | | |
|---|---|---|---|---|
| | | AA | SYELSQPPSVSVSPGQTASITCSGDKLGEKYACWYQQKPGQSPVLVIYQDRKRPSGIPERFSGSNSGNKATLTISGTQAMDEADYYCQAWVNNTMIFGGGTKLTVL | QVHLVESGGGVVQPGRSLRLSCAASGFTFSYYGMHWVRQAPGKGLEWVAVISYDGSNKYYADSVKGRFTISRDNSKNTLHLQMNSLRAEDTAVYYCARDRRGGYGVPDAFDIWGQGTMVTVSS |
| | | | SEQ ID NO: 24734 | SEQ ID NO: 28740 |
| iPS:393174 | 21-225_15D8 | NA | CTGCCTGTGCTGACTCAGCCCCCGTCTGCATCTGCCTTGCTGGGAGCCTCGATCAAGCTCACCTGCACCCTAAGCAGTGAGCACAGCACCTACACCATCGAATGGTATCAACAGAGACCAGGGAGGTCCCCCCAGTATATAATGAAGGTTAAGAGTGATGGCAGCCACAGCAAGGGGGACGGGATCCCCGATCGCTTCATGGGCTCCAGTTCTGGGGCTGACCGCTACATCACCTTCTCCAACCTCCAGTCTGACGATGAGGAGGAGTATCACTGTGGAGAGAGCCACACGATCGATGGCCAAGTCGGTGTGGTATTCGGCGGAGGGACCAAGCTGACCGTCCTA | CAGGTGCAGTTGGTGGAGTCTGGGGGAGGCGTGGTCCAGACTGGGAGGTCCCTGAGACTCTCCTGTGCAGCCTCTGGATTCACCTTCAGTGGCTATGGCATGCACTGGGTCCGCCAGGCTCCAGGCAAGGGGCTGGAGTGGGTGTCAGTTATATCATATGATGGAAGTAATAAATACTATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTACTGTGCGAGAGATCGGGTCTATTGTAGTAGTACCAGCTGCGTCCCTTACTACGACTACTACGGTATGGACGTCTGGGGCCAAGGGACCACGGTCACCGTCTCCTCA |
| | | | SEQ ID NO: 24735 | SEQ ID NO: 28741 |
| | | AA | LPVLTQPPSASALLGASIKLTCTLSSEHSTYTIEWYQQRPGRSPQYIMKVKSDGSHSKGDGIPDRFMGSSSGADRYITFSNLQSDDEEEYHCGESHTIDGQVGVVFGGGTKLTVL | QVQLVESGGGVVQTGRSLRLSCAASGFTFSGYGMHWVRQAPGKGLEWVSVISYDGSNKYYADSVKGRFTISRDNSKNTLYLQMNSLRAEDTAVYYCARDRVYCSSTSCVPYYDYYGMDVWGQGTTVTVSS |
| | | | SEQ ID NO: 24736 | SEQ ID NO: 28742 |

FIGURE 50

| | | | | |
|---|---|---|---|---|
| **iPS:393176** | **21-225_27E7** | NA | TCCTATGAGCTGACTCAGCCACCCTCAGTGTCCGTGTCCCCAGGACAGACAGCCAGCATCACCTGCTCTGGAGATAAATTGGGGAATAAATATGCTTGCTGGTATCAGCAGAAGCCAGGCCAGTCCCCTGTGGAGGTCATCTATCAAGATAGCAAGCGGCCCTTAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGTAGTACTGTGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGGATTCCTATTGTAGTAGTACCAGCTGCCCTTACTACTACTACTACGGTATGGACGTCTGGGGCCAAGGGACCACGGTCACCGTCTCCTCA |
| | | | SEQ ID NO: 24737 | SEQ ID NO: 28743 |
| | | AA | SYELTQPPSVSVSPGQTASITCSGDKLGNKYACWYQQKPGQSPVEVIYQDSKRPLGIPERFSGSNSGNTATLTISGTQAMDEADYYCQAWDSSTVVFGGGTKLTVL | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCAREDSYCSSTSCPYYYYYGMDVWGQGTTVTVSS |
| | | | SEQ ID NO: 24738 | SEQ ID NO: 28744 |

FIGURE 50

| | | | | |
|---|---|---|---|---|
| **iPS:393178** | **21-225_34D7** | NA | TCCTATGAGCTGACTCAGCCACCCTCAGTGTCCGTGTCCCCAGGACAGACAGCCAGCATCACCTGCTCTGGAGATAAATTGGGGGAGAAATATGCTTACTGGTATCAGCAGAAGCCAGGCCAGTCCCCTGTGCTGGTCCTCTATCAAGATAGCAAGCGGCCCTCAGGGATCCCTGAGCGATTCTCTGGCTCCAACTCTGGGAACACAGCCACTCTGACCATCAGCGGGACCCAGACTATGGATGAGGCTGACTTTTACTGTCAGGCGTGGGACAACACCACTGTGGTATTCGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAACAGTGGTGGCACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGGGGGTATTACTATGGTTCGGGGAGTTATTATAACGACCTCGACCCCTGGGGTCAGGGAACCCTGGTCACCGTCTCCTCA |
| | | | SEQ ID NO: 24739 | SEQ ID NO: 28745 |
| | | AA | SYELTQPPSVSVSPGQTASITCSGDKLGEKYAYWYQQKPGQSPVLVLYQDSKRPSGIPERFSGSNSGNTATLTISGTQTMDEADFYCQAWDNTTVVFGGGTKLTVL | QVQLVQSGAEVKKPGASVKVSCKASGYTFTGYYMHWVRQAPGQGLEWMGWINPNSGGTNYAQKFQGRVTMTRDTSISTAYMELSRLRSDDTAVYYCARGYYYGSGSYYNDLDPWGQGTLVTVSS |
| | | | SEQ ID NO: 24740 | SEQ ID NO: 28746 |
| **iPS:393180** | **21-225_4G12** | NA | CAGTCTGTGCTGACTCAGCCACCCTCAGCGTCTGGGACCCCCGGGCAGAGGGTCAACATGTCTTGTTCTGGAACCAACTCCAACATCGGAAGTTATACTGTAAACTGGTACCAGCAGCTCCCAGGAACGGCCCCCAAACTCCTCATCTATATTAATAATCAGCGGCCCTCAGGGGTCCCTGACCGATTCTCTGGCTCCAAGTCTGGCACCTCAGCCTCCCTGGCCATCAGTGGGCTCCAGTCTGAGGATGAGGCTGATTATTACTGTGCAGCATGGGATGACAGCCTGAATGGTCATGTGGTATTCGGCGGAAGGACCAAGCTGACCGTCCTA | GAGGTGCAGCTGTTGGAGTCTGGGGGAGGCTTGGTACAGCCGGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAACTCTTAGTGGTCGTGGTGGTAGCACATACTACGCAGACTCCGTGAAGGGCCGGTTCACCATCTCCAGAGACAATTCCAAGAACACGCTGTATCTGCAAATGAGCAGCCTGAGAGCCGAGGACACGGCCGTATATTACTGTGCGAAATGGGGACGTGGATACAGCTATGAGTACTACTACGGTATGGACGTCTGGGGCCAAGGGACCACGGTCACCGTCTCCTCA |
| | | | SEQ ID NO: 24741 | SEQ ID NO: 28747 |

FIGURE 50

| | | | | |
|---|---|---|---|---|
| | | AA | QSVLTQPPSASGTPGQRVNMSCSGTNSNIGSYTVNWYQQLPGTAPKLLIYINNQRPSGVPDRFSGSKSGTSASLAISGLQSEDEADYYCAAWDDSLNGHVVFGGRTKLTVL | EVQLLESGGGLVQPGGSLRLSCAASGFTFSSYAMSWVRQAPGKGLEWVSTLSGRGGSTYYADSVKGRSTISRDNSKNTLYLQMSSLRAEDTAVYYCAKWGRGYSYEYYYGMDVWGQGTTVTVSS |
| | | | SEQ ID NO: 24742 | SEQ ID NO: 28748 |
| iPS:393182 | 21-225_4B3 | NA | TCCTATGAGCTGACTCAGCCACCCTCAGTGTCCGTGTCCCCAAGACAGACAGTCAGCATCACCTGCTCTGGAGATAAATTGGGGGATAAATATGCTTGCTGGTATCAGCAGAAGCCAGGCCAGTCCCCTGTGCTGGTCATCTATCAAGATCGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAACAACACTGTGATATTCGGCGGAGGGACCAAGCTGACCGTCCTA | CAGGTGCAACTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAACAGTGGTGGCACAAACTCTGCACAGAAGTTTCAGGGCAGGGTCACCATGACCAGGGACACGTCCATCAGCACAGCCTACATGGAGCTGAGCAGGCTGAGATCTGACGACACGGCCGTATATTACTGTGCGAGGTCCTATTACTATGGTTCGGGGAGTTATTATAACGAGTTTGACTACTGGGGCCAGGGAACCCTGGTCACCGTCTCCTCA |
| | | | SEQ ID NO: 24743 | SEQ ID NO: 28749 |
| | | AA | SYELTQPPSVSVSPRQTVSITCSGDKLGDKYACWYQQKPGQSPVLVIYQDRKRPSGIPERFSGSNSGNTATLTISGTQAMDEADYYCQAWDNNTVIFGGGTKLTVL | QVQLVQSGAEVKKPGASVKVSCKASGYTFTGYYMHWVRQAPGQGLEWMGWINPNSGGTNSAQKFQGRVTMTRDTSISTAYMELSRLRSDDTAVYYCARSYYYGSGSYYNEFDYWGQGTLVTVSS |
| | | | SEQ ID NO: 24744 | SEQ ID NO: 28750 |

FIGURE 50

| | | | | |
|---|---|---|---|---|
| **iPS:393184** | **21-225_15H11** | NA | TCCTATGAGCTGACTCAGCCACCCTCAGTGTCCGTGTCCCCAGGACAGACAGCCAGCATCACCTGCTCTGGAGATAAATTGGGGGAGAAATATGCTTGCTGGTATCAGCAGAAGCCAGGCCAGTCCCCTGTGGTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATAGATGAGGCTGACTATTACTGTCAGGCGTGGGACAGCAGCACTGCGGTATTCGGCGGAGGGACCAAGTTGACCGTCCTA | CAGATCACCTTGAAGGAGTCTGGTCTTACGCTGATGAAACCCACACAGACCCTCACGCTGACCTGCACCTTCTCTGGGTTCTCACTCAGCACTGGTGGAGTGGGTGTGGGCTGGATCCGTCAGCCCCCAGGAAAGGCCCTGGAGTGGCTTGCACTCATTTATTGGCATGATGATAAGCGCTACAGTCCATCTCTGAGGAGCAGGCTCACCATCACCAAGGACACCTCCAAAAACCAGGTGGTCCTTACAATGACCAACATGGACCCTGTGGACACAGCCACATATTACTGTGCACGTATAGTAGCAGTTGCCTTTGACTACTGGGGCCAGGGAACCCTGATCACCGTCTCCTCA |
| | | | SEQ ID NO: 24745 | SEQ ID NO: 28751 |
| | | AA | SYELTQPPSVSVSPGQTASITCSGDKLGEKYACWYQQKPGQSPVVVIYQDRKRPSGIPERFSGSNSGNTATLTISGTQAIDEADYYCQAWDSSTAVFGGGTKLTVL | QITLKESGLTLMKPTQTLTLTCTFSGFSLSTGGVGVGWIRQPPGKALEWLALIYWHDDKRYSPSLRSRLTITKDTSKNQVVLTMTNMDPVDTATYYCARIVAVAFDYWGQGTLITVSS |
| | | | SEQ ID NO: 24746 | SEQ ID NO: 28752 |
| **iPS:393186** | **21-225_27D9** | NA | TCCTATGAGCTGACTCAGCCACCCTCAATGTCCGTGTCCCCAGGACAGACAGCCAGCATCACCTGCTCTGGATATAAATTGGGGGATAAATATGCTTGCTGGTTTCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGTCAACAACACTGTATTCGGCGGAGGGACCAAGCTGACCGTCCTA | CAGGTGCAGTTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAACAGTGGTGGCACAAAGTATGCACAGAAGTTTCAGGGCAGGGTCACCATGACCAGGGACACGTCCATCAGCACAGCCTACATGGAGCTGAACAGGCTGAGATCTGACGACACGGCCGTGTATTACTGTGCGAGAGAGAGGTGTAGTACTACCAGTTGCTATTTAGGAATTACGGGCTACTACGGTATGGACGTCTGGGGCCAAGGGACCACGGTCACCGTCTCCTCA |

FIGURE 50

| | | | | |
|---|---|---|---|---|
| | | | SEQ ID NO: 24747 | SEQ ID NO: 28753 |
| | | AA | SYELTQPPSMSVSPGQTASITCSGYKLGDKYAC WFQQKPGQSPVLVIYQDSKRPSGIPERFSGSNSG NTATLTISGTQAMDEADYYCQAWVNNTVFGGG TKLTVL | QVQLVQSGAEVKKPGASVKVSCKASGYTFTGYYM HWVRQAPGQGLEWMGWINPNSGGTKYAQKFQGR VTMTRDTSISTAYMELNRLRSDDTAVYYCARERCS TTSCYLGITGYYGMDVWGQGTTVTVSS |
| | | | SEQ ID NO: 24748 | SEQ ID NO: 28754 |
| **iPS:393188** | **21-225_34B9** | NA | TCCTATGAGCTGACTCAGGCACCCTCAGTGTC CGTGTCCCCAGGACAGACAGCCAGCATCACCT GCTCTGGAGATAAATTGGGGGAGAAATATGTT TCCTGGTATCAGGAGAAGCCAGGCCAGTCCCC TGTGCTGGTCATCTATCAAGATAGTAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGTATTC GGCGGAGGGACCAAGCTGACCGTCCTA | CAGATCACCTTGAGGGAGTCTGGTCCTACGCTGG TGAAACCCACACAGACCCTCACGCTGACCTGCAC CTTCTCTGGGTTCTCACTCAGCACTAGTGGAGTG GGTGTGGGCTGGATCCGTCAGCCCCCAGGAAAG GCCCTGGAGTGGCTTGCACTCATTTATTGGAATG ATGATAAGCGCTACAGCCCATCTCTGAAGAGCA GACTCACCATCACCAAGGACACCTCCAAAAACC AGGTGGTCCTTACAATGACCAACATGGACCCTGT GGACACAGCCACATATTACTGTGCACACTTAATA GCAGTGACTTTTGACTCCTGGGGCCAGGGATCCC TGGTCACCGTCTCCTCA |
| | | | SEQ ID NO: 24749 | SEQ ID NO: 28755 |
| | | AA | SYELTQAPSVSVSPGQTASITCSGDKLGEKYVSW YQEKPGQSPVLVIYQDSKRPSGIPERFSGSNSGNT ATLTISGTQAMDEADYYCQAWDSSTVFGGGTK LTVL | QITLRESGPTLVKPTQTLTLTCTFSGFSLSTSGVGVG WIRQPPGKALEWLALIYWNDDKRYSPSLKSRLTIT KDTSKNQVVLTMTNMDPVDTATYYCAHLIAVTFD SWGQGSLVTVSS |
| | | | SEQ ID NO: 24750 | SEQ ID NO: 28756 |

FIGURE 50

| | | | | |
|---|---|---|---|---|
| iPS:393192 | 21-225_12B1 | NA | TCCTATGAGCTGACTCAGCCACCCTCAGTGTCCGTGTCCCCAAGACAGACAGTCAGCATCACCTGCTCTGGAGATAAATTGGGGGATAAATATGCTTGCTGGTATCAGCAGAAGCCAGGCCAGTCCCCTGTGCTGGTCATCTATCAAGATCGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAACAACACTGTGATATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGAATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGGGTAGCAGCAGCTGGTACCCCCTACTACTACTACGGTATGGACGTCTGGGGCCAAGGGACCACGGTCACCGTCTCCTCA |
| | | | SEQ ID NO: 24751 | SEQ ID NO: 28757 |
| | | AA | SYELTQPPSVSVSPRQTVSITCSGDKLGDKYACWYQQKPGQSPVLVIYQDRKRPSGIPERFSGSNSGNTATLTISGTQAMDEADYYCQAWDNNTVIFGGGTKLTVL | QVQLVESGGGVVQPGRSLRLSCAASGFTFSSYGMHWVRQAPGKGLEWVAVIWNDGSNKYYADSVKGRFTISRDNSKNTLYLQMNSLRAEDTAVYYCARDRVAAAGTPYYYYGMDVWGQGTTVTVSS |
| | | | SEQ ID NO: 24752 | SEQ ID NO: 28758 |
| iPS:393194 | 21-225_16D2 | NA | TCCTATGAGCTGACTCAGCCACCCTCAGTGTCCGTGTCCCCAGGACAGACAGCCAGCATCACCTGCTCTGGAGATAAATTGGGGGATAAATATGCTTGCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTTATGTGGTATTCGGCGGAGGGACCAAGCTGACCGTCCTA | GAGGTGCAGCTGGTGGAGTCTGGGGGAGGCTTGGTAAAGCCTGGGGGGTCCCTTAGACTCTCCTGTGCAGCCTCTGGATTCACTTTCAGTAACGCCTGGATGAACTGGGTCCGCCAGGCTCCAGGGAAGGGGCTGGAGTGGGTTGGCCGTATTAAAAGCAAAACTGATGGTGGGACAACAGACTACGCTGCACCCGTGAAAGGCAGATTCACCATCTCAAGAGATGATTCAAAAAACACGCTGTATCTGCAAATGCACAGCCTGAAAACCGAGGACACAGCCGTGTATTACTGTACCACAGATACGGGTCCTATAGCAGCTCGTCTCGCTTACTACTACTACTACGCTATGGACGTCTGGGGCCAAGGGACCACGGTCACCGTCTCCTCA |

FIGURE 50

| | | | | |
|---|---|---|---|---|
| | | | SEQ ID NO: 24753 | SEQ ID NO: 28759 |
| | | AA | SYELTQPPSVSVSPGQTASITCSGDKLGDKYACWYQQKPGQSPVLVIYQDSKRPSGIPERFSGSNSGNTATLTISGTQAMDEADYYCQAWDSSTYVVFGGGTKLTVL | EVQLVESGGGLVKPGGSLRLSCAASGFTFSNAWMNWVRQAPGKGLEWVGRIKSKTDGGTTDYAAPVKGRFTISRDDSKNTLYLQMHSLKTEDTAVYYCTTDTGPIAARLAYYYYYAMDVWGQGTTVTVSS |
| | | | SEQ ID NO: 24754 | SEQ ID NO: 28760 |
| iPS:393196 | 21-225_16G8 | NA | TCCTATGAGCTGAGTCAGCCACCCTCAGTGTCCGTGTCCCCAGGACAGACAGCCAGCATCACCTGCTCTGGAGATAAATTGGGGGAAAAATATGCTTGCTGGTATCAGCAGAAGCCAGGCCAGTCCCCTGTGCTGGTCATCTATCAAGATAGGAAGCGGCCCTCAGGGATCCCTGAGCGATTCTCTGGCTCCAACTCTGGGAACAAAGCCACTCTGACCATCAGCGGGACCCAGGCTATGGATGAGGCTGACTATTACTGTCAGGCGTGGGTCAATAACACTATGATATTCGGCGGAGGGACCAAGCTGACCGTCCTA | GAGGTGCAGCTGTTGGAGTCTGGGGGAGGCTTGGTACAGCCTGAGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGGTATTAGTGGTAGTGGTGGTAGCACATACTACGCAGACTCCGTGAAGGGCCGGTTCACCATCTCCAGAGACAATTCCAAGAACACGCTGTGTCTGCACATGAACAGCCTGAGAGCCGAGGACACGGCCGTATATTACTGTGCGAAAGAATATTGTGGTGGTGACTGCTATTCCCCTTACTACTACTACTACGGTATGGACGTCTGGGGCCAAGGGACCACGGTCACCGTCTCCTCA |
| | | | SEQ ID NO: 24755 | SEQ ID NO: 28761 |
| | | AA | SYELSQPPSVSVSPGQTASITCSGDKLGEKYACWYQQKPGQSPVLVIYQDRKRPSGIPERFSGSNSGNKATLTISGTQAMDEADYYCQAWVNNTMIFGGGTKLTVL | EVQLLESGGGLVQPEGSLRLSCAASGFTFSSYAMSWVRQAPGKGLEWVSGISGSGGSTYYADSVKGRFTISRDNSKNTLCLHMNSLRAEDTAVYYCAKEYCGGDCYSPYYYYYGMDVWGQGTTVTVSS |
| | | | SEQ ID NO: 24756 | SEQ ID NO: 28762 |

FIGURE 50

| | | | | |
|---|---|---|---|---|
| **iPS:393198** | **21-225_28A11** | NA | TCCTATGAGTTGACTCAGCCACCCTCAGTGTCCGTGTCCCCAGGACAGACAGCCAGCATCACCTGCTCTGGAGATAAATTGGGGGATAAATATGCTTGCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGTAGCACTTATGTGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGGCTATGGCCTGCACTGGGTCCGCCAGGCTCCAGGCAAGGGGCTGGAGTGGGTGGCACTTATATGGTATGATGGAAATAATACATACTATGCAGACTCCGTGAAGGGCCGATTCACCATCTCCAGAGACAATTCCAAGAACACGCTGTATCTGCAAATGAACAGCCTGAGAGCCGAAGACACGGCTGTGTATTACTGTGCGAGAGATAGGGTATATTGTAGTAGTACCAGCTGCTCCCCTTACTACTACTACTACGGTATGGACGTCTGGGGCCAAGGGACCACGGTCACCGTCTCCTCA |
| | | | SEQ ID NO: 24757 | SEQ ID NO: 28763 |
| | | AA | SYELTQPPSVSVSPGQTASITCSGDKLGDKYACWYQQKPGQSPVLVIYQDSKRPSGIPERFSGSNSGNTATLTISGTQAMDEADYYCQAWDSSTYVVFGGGTKLTVL | QVQLVESGGGVVQPGRSLRLSCAASGFTFSGYGLHWVRQAPGKGLEWVALIWYDGNNTYYADSVKGRFTISRDNSKNTLYLQMNSLRAEDTAVYYCARDRVYCSSTSCSPYYYYYGMDVWGQGTTVTVSS |
| | | | SEQ ID NO: 24758 | SEQ ID NO: 28764 |

FIGURE 50

| | | | | |
|---|---|---|---|---|
| iPS:393200 | 21-225_35E1 | NA | TCCTATGAGCTGACTCAGCCACCCTCAGTGTCCGTGTCCCCAGGACAGACAGCCAGCATCACCTGCTCTGGAGATAAATTGGGGGAAAAATATGCTTACTGGTTTCAGCAGAAGCCAGGCCAGTCCCCTGTGATAGTCATCTATCAAGATAGGAAGCGGCCCTCAGGGATCCCTGAGCGATTCTCTGGCTCCAACTCTGGGAACACAGCCACTCTGACCATCAGCGGGACGCAGGCTATGGATGAGGCTGACTATTACTGTCAGGCGTGGGACAACAGCACTGCGGTATTCGGCGGAGGGACCAAGCTGACCGTCCTA | CAGGTGAAGCTGGTGCAGTCTGGGGCTGAGGTGAAGAAGCCTGGGGCCTCAGTGAAGGTCTCCTGCAAGGCTTCAGGATACACCTTCACCGGCTACTATATGCACTGGGTGCGACAGGCCCCTGGACAAGGGCTTGAGTGGATGGGATGGATCAACCCTAAAAGTGGTGGCACAAATTATGCACAGAAGTTTCAGGGCAGGGTCACCATGACCAGGGACACGTCCATCAGCACAGTCTACATGGAGCCGAGCAGGCTGAGATCTGACGACACGGCCGTGTATTACTGTGCGAGAGTGTATTACCATGGTTCGGGGAGTTATTATAACGAGTTTGATTATTGGGGCCAGGGAACCCTGGTCACCGTCTCCTCA |
| | | | SEQ ID NO: 24759 | SEQ ID NO: 28765 |
| | | AA | SYELTQPPSVSVSPGQTASITCSGDKLGEKYAYWFQQKPGQSPVIVIYQDRKRPSGIPERFSGSNSGNTATLTISGTQAMDEADYYCQAWDNSTAVFGGGTKLTVL | QVKLVQSGAEVKKPGASVKVSCKASGYTFTGYYMHWVRQAPGQGLEWMGWINPKSGGTNYAQKFQGRVTMTRDTSISTVYMEPSRLRSDDTAVYYCARVYYHGSGSYYNEFDYWGQGTLVTVSS |
| | | | SEQ ID NO: 24760 | SEQ ID NO: 28766 |
| iPS:393202 | 21-225_6B4 | NA | TCCTATGAGCTGACTCAGCCACCCTCAGTGTCCGTGTCCCCAAGACAGACAGTCAGCATCACCTGCTCTGGAGATAAATTGGGGGATAAATATGCTTGCTGGTATCAGCAGAAGCCAGGCCAGTCCCCTGTGCTGGTCATCTATCAAGATCGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAACAACACTGTGATATTCGGCGGAGGGACCAAGCTGACCGTCCTA | GAGGTGCAGCTGTTGGAGTCTGGGGGAGGCTTGGTACAGCCTGAGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTAGTGGTAGTAGCACATACTACGCAGACTCCGTGAAGGGCCGGTTCACCATCTCCAGAGACAATTCCAAGAACACGCTGTGTCTGCAAATGAACAGCCTGAGAGCCGAGGACACGGCCGTATATTACTGTGCGAAAGAATATTGTGGTGGTGACTGCTATTCCCCTTACTACTACTACTACGGTATGGACGTCTGGGGCCAAGGGACCACGGTCACCGTCTCCTCA |
| | | | SEQ ID NO: 24761 | SEQ ID NO: 28767 |

FIGURE 50

| | | | | |
|---|---|---|---|---|
| | | AA | SYELTQPPSVSVSPRQTVSITCSGDKLGDKYACWYQQKPGQSPVLVIYQDRKRPSGIPERFSGSNSGNTATLTISGTQAMDEADYYCQAWDNNTVIFGGGTKLTVL | EVQLLESGGGLVQPEGSLRLSCAASGFTFSSYAMSWVRQAPGKGLEWVSAISGSGSSTYYADSVKGRFTISRDNSKNTLCLQMNSLRAEDTAVYYCAKEYCGGDCYSPYYYYYGMDVWGQGTTVTVSS |
| | | | SEQ ID NO: 24762 | SEQ ID NO: 28768 |
| iPS:393204 | 21-225_8C12 | NA | TCCTATGAGCTGACTCAGCCACACTCAGTGTCAGTGGCCACAGCACAGATGGCCAGGATCACCTGTGGGGGAAACAACATTGGAAGTAAAGCTGTGCACTGGTACCAGCAAAAGCCAGGCCAGGACCCTGTGCTGGTCATCTATAGCGATAGCAACCGGCCCTCAGGGATCCCTGAGCGATTCTCTGGCTCCAACCCAGGGAACACCGCCACCCTAACCATCAGCAGGATCGAGGCTGGGGATGAGGCTGACTATTACTGTCAGGTGTGGGACAGTAGTAGTGATCATGTGGTATTCGGCGGAGGGACCAAGCTGACCGTCCTA | CAGGTGCAGTTGGTGGAGTCTGGGGGAGGCGTGGTCCAGCCTGGGAGGTCCCTGAGACTCTCCTGTGCAGCGTCTGGATTCACCTTCGGTAGCTATGGCATGCACTGGGTCCGCCAGGCTCCAGGCAAGGGGCTGGAGTGGGTGGCACTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGGGTATCTTGTAGTAGTTCCAGCTGCTATCCTTACTACTACTACTACGGTATGGACGTCTGGGGCCAAGGGACCACGGTCACCGTCTCCTCA |
| | | | SEQ ID NO: 24763 | SEQ ID NO: 28769 |
| | | AA | SYELTQPHSVSVATAQMARITCGGNNIGSKAVHWYQQKPGQDPVLVIYSDSNRPSGIPERFSGSNPGNTATLTISRIEAGDEADYYCQVWDSSSDHVVFGGGTKLTVL | QVQLVESGGGVVQPGRSLRLSCAASGFTFGSYGMHWVRQAPGKGLEWVALIWYDGSNKYYADSVKGRFTISRDNSKNTLYLQMNSLRAEDTAVYYCARDRVSCSSSCYPYYYYYGMDVWGQGTTVTVSS |
| | | | SEQ ID NO: 24764 | SEQ ID NO: 28770 |

FIGURE 50

| | | | | |
|---|---|---|---|---|
| **iPS:393206** | **21-225_13F6** | NA | TCCTATGAGCTGACTCAGCCACCCTCAGTGTCCGTGTCCCCAGGACAGACAGCCAGCATCACCTGCTCTGGAGATAAATTGGGGGATAAATATGCTTGCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGGCAACAGCACTGCTGTGGTATTCGGCGGAGGGACCAAGCTGACCGTCCTG | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAACAGTGGTGGCGCAAACTATGCACAGAAGTTTCAGGGCAGGGTCACCATGACCAGGGACACGTCCATCAGCACAGCCTACATGGAGCTGAGCAGGCTGAGATCTGACGACACGGCCGTGTATTTCTGTGCGAGGTCGTTTTACTATGGTTCGGGGACTTATTATAACGAGTTTGACTACTGGGGCCAGGGAACCCTGGTCACCGTCTCCTCA |
| | | | SEQ ID NO: 24765 | SEQ ID NO: 28771 |
| | | AA | SYELTQPPSVSVSPGQTASITCSGDKLGDKYACWYQQKPGQSPVLVIYQDSKRPSGIPERFSGSNSGNTATLTISGTQAMDEADYYCQAWGNSTAVVFGGGTKLTVL | QVQLVQSGAEVKKPGASVKVSCKASGYTFTGYYMHWVRQAPGQGLEWMGWINPNSGGANYAQKFQGRVTMTRDTSISTAYMELSRLRSDDTAVYFCARSFYYGSGTYYNEFDYWGQGTLVTVSS |
| | | | SEQ ID NO: 24766 | SEQ ID NO: 28772 |
| **iPS:393208** | **21-225_16F3** | NA | TCCTATGTGCTGACTCAGCCACCCTCGGTGTCAGTGGCCCCAGGACAGACGGCCAGGATTACCTGTGGGGGAAACAACATTGGAAGTAAAAGTGTGCACTGGTACCAGCAGAAGCCAGGCCAGGCCCCTGTGCTGGTCGTCTATGATGATACCGACCGGCCCTCAGGGATCCCTGAGCGATTCTCTGGCTCCAACTCTGGGAACACGGCCACCCTGACCATCAGCAGGGTCGAAGCCGGGGATGAGGCCGACTATTACTGTCAGGTGTGGGATAGTAGCAGTGATCATGTGGTATTCGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGGCACTATATGCACTGGGTGCGACAGGCCCCTGGACAAGGACTTGAGTGGATGGGATGGATCAACCCTAACAGTGGTGGCACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGGTCGTATTACTATGGTTCGGGGACTTATTATAACGAGTTTGACTACTGGGGCCAGGGAACCCTGGTCACCGTCTCCTCA |
| | | | SEQ ID NO: 24767 | SEQ ID NO: 28773 |

FIGURE 50

| | | | | |
|---|---|---|---|---|
| | | AA | SYVLTQPPSVSVAPGQTARITCGGNNIGSKSVHWYQQKPGQAPVLVVYDDTDRPSGIPERFSGSNSGNTATLTISRVEAGDEADYYCQVWDSSSDHVVFGGGTKLTVL | QVQLVQSGAEVKKPGASVKVSCKASGYTFTGHYMHWVRQAPGQGLEWMGWINPNSGGTNYAQKFQGRVTMTRDTSISTAYMELSRLRSDDTAVYYCARSYYYGSGTYYNEFDYWGQGTLVTVSS |
| | | | SEQ ID NO: 24768 | SEQ ID NO: 28774 |
| iPS:393210 | 21-225_17D3 | NA | TCCTATGAGCTGACGCAGTCACCCTCAGTGTCCGTGTCCCCAGGACAGACAGCCAGCATCACCTGCTCTGGAGATAAATTGGGGGATAAATATGTTTACTGGTATCAGCAGAAGCCAGGCCAGTCCCCTGTGGTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCATCACTGCAGTATTCGGCGGAGGGACCAAGCTGACCGTCCGA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAACAGTGGTGGCACAAACTATGCACAGAAGTTTCAGGGCAGGGTCACCATGACCAGGGACACGTCCATCAGCACAGCCTACATGGAGCTGAGCAGGCTGAGATCGGACGACACGGCCGTGTATTACTGTGCGAGAGCGAATTACTATGGTTCGGGGAGTTATTATAACGACTTTGACTACTGGGGCCAGGGAACCCTGGTCACCGTCTCCTCA |
| | | | SEQ ID NO: 24769 | SEQ ID NO: 28775 |
| | | AA | SYELTQSPSVSVSPGQTASITCSGDKLGDKYVYWYQQKPGQSPVVVIYQDRKRPSGIPERFSGSNSGNTATLTISGTQAMDEADYYCQAWDSITAVFGGGTKLTVR | QVQLVQSGAEVKKPGASVKVSCKASGYTFTGYYMHWVRQAPGQGLEWMGWINPNSGGTNYAQKFQGRVTMTRDTSISTAYMELSRLRSDDTAVYYCARANYYGSGSYYNDFDYWGQGTLVTVSS |
| | | | SEQ ID NO: 24770 | SEQ ID NO: 28776 |

FIGURE 50

| | | | | |
|---|---|---|---|---|
| **iPS:393212** | **21-225_30H6** | NA | TCCTATGAGCTGACTCAGCCACCCTCAGTGTCCGTGTCCCCAGGACAGACAGCCAGCATCACCTGCTCTGGAGATAAATTGGGTAATAAATATGCTTGCTGGTATCAGCAGAAGCCAGGCCAGTCCCCTGTGTTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTGTTTTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGATTCTCACTCAGCACTGGTGGAGTGGGTGTGGGCTGGATCCGTCAGCCCCCAGGAAAGGCCCTGGAGTGGCTTGCACTCATTTATTGGCATGATGATAAGCGCTACAGTCCCTCTCTGAAGAGCAGGCTCGCCATCACCAAGGACACCTCCAAAAACCAGGTGGTCCTTACAATTACCAACATGGACCCTGTGGACACAGCCACATATTACTGTGCTCACTTAATAGCAGTGGCTTTTGACTATTGGGGCCAGGGAACCCTGGTCACCGTCTCCTCA |
| | | | SEQ ID NO: 24771 | SEQ ID NO: 28777 |
| | | AA | SYELTQPPSVSVSPGQTASITCSGDKLGNKYACWYQQKPGQSPVLVIYQDSKRPSGIPERFSGSNSGNTATLTISGTQAMDEADYYCQAWDSSTVFGGGTKLTVL | QITLKESGPTLVKPTQTLTLTCTFSGFSLSTGGVGVGWIRQPPGKALEWLALIYWHDDKRYSPSLKSRLAITKDTSKNQVVLTITNMDPVDTATYYCAHLIAVAFDYWGQGTLVTVSS |
| | | | SEQ ID NO: 24772 | SEQ ID NO: 28778 |
| **iPS:393214** | **21-225_33A1** | NA | TCCTATGAGCTGACTCAGCCACCCTCAGTGTCCGTGTCCCCAGGACAGACAGCCAGCATCACCTGCTCTGGAGATAAATTGGGGGATAAATTTGTTTATTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCACCACCGTGGTATTCGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCAGCGGCTACTATATGCACTGGGTGCGACAGGCCCCTGGACAAGGACTTGAGTGGATGGGATGGATCAACCCTAACAATGGTGGCACACACTATGCACAGAAGTTTCAGGGCAGGGTCACCATGACCAGGGACACGTCCATCCGCACAGCCTCCATGGAGCTGAGCAGGCTGAGATCTGACGACACGGCCGTGTATTTCTGTGCGAGGGGATATTATTATGCTTCGGGGAGTTATTATAACGACCTCGACCCCTGGGGCCAGGGAACCCTGGTCACCGTCTCCTCA |
| | | | SEQ ID NO: 24773 | SEQ ID NO: 28779 |

FIGURE 50

| | | | | |
|---|---|---|---|---|
| | | AA | SYELTQPPSVSVSPGQTASITCSGDKLGDKFVYWYQQKPGQSPVLVIYQDSKRPSGIPERFSGSNSGNTATLTISGTQAMDEADYYCQAWDSTTVVFGGGTKLTVL | QVQLVQSGAEVKKPGASVKVSCKASGYTFSGYYMHWVRQAPGQGLEWMGWINPNNGGTHYAQKFQGRVTMTRDTSIRTASMELSRLRSDDTAVYFCARGYYYASGSYYNDLDPWGQGTLVTVSS |
| | | | SEQ ID NO: 24774 | SEQ ID NO: 28780 |
| **iPS:393218** | **21-225_14G3** | NA | TCCTATGAGCTGACTCAGCCACCCTCAGTGTCCGTGTCCCCAGGACAGACAGCCAGCATCACCTGCTCTGGAGATAAATTGGGGGATAAATATGTTTGCTGGTATCAGCAGAAGCCAGGCCAGTCCCCTGTGCTGGTCATCTATCAAGATCGCAAGCGGCCCTCAGGGATCCCTGAGCGATTCTCTGGCTCCAACTCTGGGAACACAGCCACTCTGACCATCAGCGGGACCCAGGCTATGGATGAGGCTGATTATTACTGTCAGGCGTGGGGCAACAGCACTGCTGTGGTATTCGGCGGAGGGACCAAGCTGACCGTCCTG | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGTACTGGGTGCGACAGGCCCCTGGACAAGGGCTTGAGTGGATGGGATGGATCAACCCTAACAGTGGTGGCACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTTTATTACTGTGCGAGGTCGTATTTTTATGGTTCGGGGAGTTATTATAACGAGTTTGACTACTGGGGCCAGGGAACCCTGGTCACCGTCTCCTCA |
| | | | SEQ ID NO: 24775 | SEQ ID NO: 28781 |
| | | AA | SYELTQPPSVSVSPGQTASITCSGDKLGDKYVCWYQQKPGQSPVLVIYQDRKRPSGIPERFSGSNSGNTATLTISGTQAMDEADYYCQAWGNSTAVVFGGGTKLTVL | QVQLVQSGAEVKKPGASVKVSCKASGYTFTGYYMYWVRQAPGQGLEWMGWINPNSGGTNYAQKFQGRVTMTRDTSISTAYMELSRLRSDDTAVYYCARSYFYGSGSYYNEFDYWGQGTLVTVSS |
| | | | SEQ ID NO: 24776 | SEQ ID NO: 28782 |

FIGURE 50

| | | | | |
|---|---|---|---|---|
| **iPS:393222** | **21-225_17F5** | NA | TCCTATGAGCTGACTCAGCCACCCTCAGTGTC CGTGTCCCCAGGACAGACAGCCAGCATCACCT GCTCTGGAGATAAATTGGGGGAAAAATATGCT TGCTGGTATCAGCAGAAGCCAGGCCAGTCCCC TGTGCTGGTCATCTATCAAGATAGAAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACGGTATTC GGCGGAGGGACCAAACTGACCGTCCTA | CAGATCACCTTGAAGGAGTCTGGTCCTTCGCTGG TGAAGCCCACACAGACCCTCACGCTGACCTGCAC CTTCTCTGGATTCTCACTCAGCACTAGTGGAGTG GGTGTGGGCTGGATCCGTCAGCCCCCGGAAAG GCCCTGGAGTGGCTTGCACTCATTTATTGGGATG ATGATAAGCGCTACAGCCCATCTCTGAAGAGCA GGCTCACCATCACCAAGGACACCTCCAAAAACC AGGTGGTCCTGACAATGACCAACATGGACCCTGT GGACACAGCCACATATTCCTGTGCACACATTATA GCAGTGGCTTTTGACTACTGGGGCCAGGGAACCC TGGTCACCGTCTCCTCA |
| | | | SEQ ID NO: 24777 | SEQ ID NO: 28783 |
| | | AA | SYELTQPPSVSVSPGQTASITCSGDKLGEKYACW YQQKPGQSPVLVIYQDRKRPSGIPERFSGSNSGN TATLTISGTQAMDEADYYCQAWDSSTVFGGGT KLTVL | QITLKESGPSLVKPTQTLTLTCTFSGFSLSTSGVGVG WIRQPPGKALEWLALIYWDDDKRYSPSLKSRLTIT KDTSKNQVVLTMTNMDPVDTATYSCAHIIAVAFD YWGQGTLVTVSS |
| | | | SEQ ID NO: 24778 | SEQ ID NO: 28784 |
| **iPS:393224** | **21-225_31C2** | NA | TCCTATGAGCTGACTCAGCCACCCTCAGTGTC CGTGTCCCCAGGGCAGACAGCCAGCATCACCT GCTCTGGAGATAAATTGGGAAATAAATATGCT TGCTGGTATCAGCAGAAGCCAGGCCAGTCCCC TGTGCTGGTCATCTATCAAGATTCCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGTATTC GGCGGAGGGACCAAACTGACCGTCCTA | CAGATCACCTTGAAGGAGTCTGGTCCTACGCTGG TGAAACCCACACAGACCCTCACGCTGACCTGCAC CTTCTCTGGGTTCTCCCTCAACACTGGTGGAGTG GGTGTGGGCTGGATCCGTCAGCCCCCAGGAAAG GCCCTGGAGTGGCTTGCACTCATTTATTGGAATG ATGATGAGCGCTACAGCCCATCTCTGAAGAGCA GGCTCACCATCACCAAGGACACCTCCAAAAACC AGGTGGTCCTTACAATGACCAACATGGACCCTTT GGACACAGCCTCATATTACTGTGCACACTTAATA GCAGTTTCCTTTGACTACTGGGGCCAGGGAGCCC TGGTCACCGTCTCCTCA |
| | | | SEQ ID NO: 24779 | SEQ ID NO: 28785 |

FIGURE 50

| | | | | |
|---|---|---|---|---|
| | | AA | SYELTQPPSVSVSPGQTASITCSGDKLGNKYACWYQQKPGQSPVLVIYQDSKRPSGIPERFSGSNSGNTATLTISGTQAMDEADYYCQAWDSSTVFGGGTKLTVL | QITLKESGPTLVKPTQTLTLTCTFSGFSLNTGGVGVGWIRQPPGKALEWLALIYWNDDERYSPSLKSRLTITKDTSKNQVVLTMTNMDPLDTASYYCAHLIAVSFDYWGQGALVTVSS |
| | | | SEQ ID NO: 24780 | SEQ ID NO: 28786 |
| **iPS:393226** | **21-225_33E6** | NA | TCCTATGAGCTGACTCAGCCACCCTCAGTGTCCGTGTCCCCAGGACAGACAGCCAGCATCACCTGCTCTGGAGATAAATTGGGGGATAAATATGCTTACTGGTTTCAGCAGAAGCCAGGCCAGTCCCCTGTGATAGTCATCTATCAAGATAGGAAGCGGCCCTCAGGGATCCCTGAGCGATTCTCTGGCTCCAACTCTGGGAACACAGCCACTCTGACCATCAGCGGGACGCAGGCTATGGATGAGGCTGACTATTACTGTCAGGCGTGGGACAACAGCACTGCGGTATTCGGCGGAGGGACCAAGCTGACCGTCCTA | CAGGTGAAGCTGGTGCAGTCTGGGGCTGAGGTGAAGAAGCCTGGGGCCTCAGTGAAGGTCTCCTGCAAGGCTTCAGGATACACCTTCACCGGCTACTATATGCACTGGGTGCGACAGGCCCCTGGACAAGGGCTTGAGTGGATGGGATGGATCAACCCTAACAGTGGTGGCACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGAGTGTATTACTATGGTTCGGGGAGTTATTATAACGAGTTTGACTACTGGGGCCAGGGAACCCTGGTCACCGTCTCCTCA |
| | | | SEQ ID NO: 24781 | SEQ ID NO: 28787 |
| | | AA | SYELTQPPSVSVSPGQTASITCSGDKLGDKYAYWFQQKPGQSPVIVIYQDRKRPSGIPERFSGSNSGNTATLTISGTQAMDEADYYCQAWDNSTAVFGGGTKLTVL | QVKLVQSGAEVKKPGASVKVSCKASGYTFTGYYMHWVRQAPGQGLEWMGWINPNSGGTNYAQKFQGRVTMTRDTSISTAYMELSRLRSDDTAVYYCARVYYYGSGSYYNEFDYWGQGTLVTVSS |
| | | | SEQ ID NO: 24782 | SEQ ID NO: 28788 |

FIGURE 50

| | | | | |
|---|---|---|---|---|
| **iPS:393230** | **21-225_9G9** | NA | CAGTCTGTGCTGACTCAGCCACCCTCAGCGTCTGGGACCCCCGGGCAGAGGGTCAACATGTCTTGTTCTGGAACCAACTCCAACATCGGAAGTTATACTGTAAACTGGTACCAGCAGCTCCCAGGAACGGCCCCCAAACTCCTCATCTATATTAATAATCAGCGGCCCTCAGGGGTCCCTGACCGATTCTCTGGCTCCAAGTCTGGCACCTCAGCCTCCCTGGCCATCAGTGGGCTCCAGTCTGAGGATGAGGCTGATTATTACTGTGCAGCATGGGATGACAGCCTGAATGGTCATGTGGTATTCGGCGGAAGGACCAAGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATACACTGGGTGCGACAGGCCCCTGGACAAGGACTTGAGTGGATGGGATGGATCAACCCTAACAGTGGTGGCACAGACTATGCACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGGTCGTATTACTATGGTTCGGGGACTTATTATAACGAGTTTGACTACTGGGGCCAGGGAACCCTGGTCACCGTCTCCTCA |
| | | | SEQ ID NO: 24783 | SEQ ID NO: 28789 |
| | | AA | QSVLTQPPSASGTPGQRVNMSCSGTNSNIGSYTVNWYQQLPGTAPKLLIYINNQRPSGVPDRFSGSKSGTSASLAISGLQSEDEADYYCAAWDDSLNGHVVFGGRTKLTVL | QVQLVQSGAEVKKPGASVKVSCKASGYTFTGYYIHWVRQAPGQGLEWMGWINPNSGGTDYAQKFQGRVTMTRDTSISTAYMELSRLRSDDTAVYYCARSYYYGSGTYYNEFDYWGQGTLVTVSS |
| | | | SEQ ID NO: 24784 | SEQ ID NO: 28790 |
| **iPS:393232** | **21-225_17F12** | NA | CAGTCTGCCCTGACTCAGCCTGCCTCCGTGTCTGGGTCTCCTGGACAGTCGATCACCATCTCCTGCACTGGAGCCAGCAGTGACGTTGGTGATTATAACTCTGTCTCCTGGTACCAACAGCACCCAGGCAAAGCCCCCAAACTCATGATTTATGAGGTCAGTAATCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCAGCTCATATACAAGCAGCATCACTGTGGTATTCGGCGGAGGGACCAAACTGACCGTCCTA | GAGGTGCAGTTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTGGTGGTGGTAGCACATACTACGCAGACTCCGTGAAGGGCCGGGTCACCATCTCCAGAGACAATTCCAAGAACACGCTGTATCTGCAAATGAACAGCCTGAGAGCCGAGGACACGGCCGTATATTACTGTGCGAAATGGGGACGTGGATACAACTATGAGTACTACTACGGTATGGACGTCTGGGGCCAAGGGACCACGGTCACCGTCTCCTCA |
| | | | SEQ ID NO: 24785 | SEQ ID NO: 28791 |

FIGURE 50

| | | | | |
|---|---|---|---|---|
| | | AA | QSALTQPASVSGSPGQSITISCTGASSDVGDYNSVSWYQQHPGKAPKLMIYEVSNRPSGVSNRFSGSKSGNTASLTISGLQAEDEADYYCSSYTSSITVVFGGGTKLTVL | EVQLLESGGGLVQPGGSLRLSCAASGFTFSSYAMSWVRQAPGKGLEWVSAISGGGGSTYYADSVKGRVTISRDNSKNTLYLQMNSLRAEDTAVYYCAKWGRGYNYEYYYGMDVWGQGTTVTVSS |
| | | | SEQ ID NO: 24786 | SEQ ID NO: 28792 |
| **iPS:393234** | **21-225_26C10** | NA | TCCTATGAAGTGACTCAGCCACCCTCAATGTCCGTGTCCCCAGGACAGACAGCCAGCATCACCTGCTCTGGAGATAAATTGGGGGATAAATATGTTTGCTGGTTTCAGCAGAAGCCAGGCCAGTCCCCTGTGTTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGTCAACAACACTGTATTCGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATGTGCACTGGGTGCGACAGGCCCCTGGACAAGGGCTTGAGTGGATGGGATGGATCAACCCTAACAGTGGTGGCACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGAGAGAGGTGTAGTACTACCAGCTGCTATTTAGGAATTACGGGCTACTACGGTATGGACGTCTGGGGCCAAGGGACCACGGTCACCGTCTCCTCA |
| | | | SEQ ID NO: 24787 | SEQ ID NO: 28793 |
| | | AA | SYEVTQPPSMSVSPGQTASITCSGDKLGDKYVCWFQQKPGQSPVLVIYQDSKRPSGIPERFSGSNSGNTATLTISGTQAMDEADYYCQAWVNNTVFGGGTKLTVL | QVQLVQSGAEVKKPGASVKVSCKASGYTFTGYYVHWVRQAPGQGLEWMGWINPNSGGTNYAQKFQGRVTMTRDTSISTAYMELSRLRSDDTAVYYCARERCSTTSCYLGITGYYGMDVWGQGTTVTVSS |
| | | | SEQ ID NO: 24788 | SEQ ID NO: 28794 |

FIGURE 50

| | | | | |
|---|---|---|---|---|
| **iPS:393345** | **21-225_5G7** | NA | TCCTATGAGCTGACTCAGCCACCCTCAGTGTCCGTGTCCCCAGGACAGACAGCCAGCATCACCTGCTCTGGAGATAAATTGGGGAATAAATATGCTTGGTGGTATCAGCAGAAGCCAGGCCAGTCCCCTGTGCTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAACAGCACTGTGGTTTTCGGCGGAGGGACCAAACTGACCGTCCTA | GAGGTGCAGCTGTTGGAGTCTGGGGGAGGCTTGGTACAGCCTGAGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTAGTGGTGGTAGCACATACTACGCAGACTCCGTGAAGGGCCGGTTCACCATCTCCAGAGACAATTCCAAGAACACGCTGTGTCTGCAAATGAACAGCCTGAGAGCCGAGGACACGGCCGTATATTACTGTGCGAAAGAATATTGTGGTGGTGACTGCTATTCCCCTTACTACTACTACTACGGTATGGACGTCTGGGGCCAAGGGACCACGGTCACCGTCTCCTCA |
| | | | SEQ ID NO: 24789 | SEQ ID NO: 28795 |
| | | AA | SYELTQPPSVSVSPGQTASITCSGDKLGNKYAWWYQQKPGQSPVLVIYQDRKRPSGIPERFSGSNSGNTATLTISGTQAMDEADYYCQAWDNSTVVFGGGTKLTVL | EVQLLESGGGLVQPEGSLRLSCAASGFTFSSYAMSWVRQAPGKGLEWVSAISGSGGSTYYADSVKGRFTISRDNSKNTLCLQMNSLRAEDTAVYYCAKEYCGGDCYSPYYYYYGMDVWGQGTTVTVSS |
| | | | SEQ ID NO: 24790 | SEQ ID NO: 28796 |
| **iPS:393368** | **21-225_29H8** | NA | GACATCGTGATGACCCAGTCTCCAGACTCCCTGGCTGTGTCTCTGGGCGAGAGGGCCACCATCAACTGCAGGTCCAGCCAGACTATTTTACACAGCTCCAACAATTACAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATTGTACTCCTCCGACGTTCGGCCAAGGGACCAAGGTGGAAATCAAA | CAGGTGCAGCTGGTGCAGTCTGGGGCTGAGGTGCAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGCTGGATGAACCCTAACAGTGGTAACACAGGCTATGCACAGAGGTTCCAGGGCAGAGTCACCATGACCAGGAACACCTCCATAAGCGCAGCCTACATGGAGCTGAGCAGCCTGAGATCTGAGGACACGGCCGTATATTACTGTGCGAGTAGCAGTGGCTGGTACTACTTTGACTACTGGGGCCAGGGAACCCTGGTCACCGTCTCCTCA |
| | | | SEQ ID NO: 24791 | SEQ ID NO: 28797 |

FIGURE 50

| | | | | |
|---|---|---|---|---|
| | | AA | DIVMTQSPDSLAVSLGERATINCRSSQTILHSSNNYNYLAWYQQKPGQPPKLLIYWASTRESGVPDRFSGSGSGTDFTLTISSLQAEDVAVYYCQQYYCTPPTFGQGTKVEIK | QVQLVQSGAEVQKPGASVKVSCKASGYTFTNYDINWVRQATGQGLEWMGWMNPNSGNTGYAQRFQGRVTMTRNTSISAAYMELSSLRSEDTAVYYCASSSGWYYFDYWGQGTLVTVSS |
| | | | SEQ ID NO: 24792 | SEQ ID NO: 28798 |
| iPS:393565 | 21-225_34B11 | NA | TCCTATGAGCTGACTCAGCCACCCTCAGTGTCCGTGTCCCCAGGACAGACAGCCAGCATCACCTGCTCTGGAGATAAATTGGGGGATAAATATGCTTGCTGGTATCAGCAGAAGCCAGGCCAGTCCCCTGTGGTGGTCATCTATCAAGATATGAAGCGGCCCTCAGGGATCCCTGAGCGATTCTCTGGCTCCAACTCTGGGAACACAGCCACTCTGACCATCAGCGGGACGCAGGCTATGGATGAGGCTGACTATTACTGTCAGGCGTGGGACAACAGCACTGCGGTATTCGGCGGAGGGACCAAGCTGACCGTCCTA | CAGGTGAAACTGGTGCAGTCTGGGGCTGAGGTGAAGAAGCCTGGGGCCTCAGTGAAGGTCTCCTGCAAGGCTTCAGGATACACCTTCACCGGCTACTATATGCACTGGGTGCGACAGGCCCCTGGACAAGGGCTTGAGTGGATGGGATGGATCAACCCTAACAGTGGTGGCACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGAGTGTATTTCTATGGTTCGGGGAGTTATTATAACGAGTTTGACTACTGGGGCCAGGGAACCCTGGTCACCGTCTCCTCA |
| | | | SEQ ID NO: 24793 | SEQ ID NO: 28799 |
| | | AA | SYELTQPPSVSVSPGQTASITCSGDKLGDKYACWYQQKPGQSPVVVIYQDMKRPSGIPERFSGSNSGNTATLTISGTQAMDEADYYCQAWDNSTAVFGGGTKLTVL | QVKLVQSGAEVKKPGASVKVSCKASGYTFTGYYMHWVRQAPGQGLEWMGWINPNSGGTNYAQKFQGRVTMTRDTSISTAYMELSRLRSDDTAVYYCARVYFYGSGSYYNEFDYWGQGTLVTVSS |
| | | | SEQ ID NO: 24794 | SEQ ID NO: 28800 |

FIGURE 50

| | | | | |
|---|---|---|---|---|
| **iPS:393802** | **21-225_3D12** | NA | GAAATTGTGTTGACGCAGTCTCCAGGCACCCTGTCTTTGTCTCCAGGGGAAAGAGCCACCCTCTCCTGCAGGGCCAGTCAGAGTGTTAGCAGCAGCTACTTAGCCTGGTACCAGCAGAAACCTGGCCAGGCTCCCAGGCTCCTCATCTATGGTACATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGGTAGTTCACGCAGTTTTGGCCAGGGGACCAAGCTGGAGATCAAA | GAGGTGCAGCTGGTGGAGTCTGGGGGAGGCTTGGTAAAGCCTGGGGGGTCCCTTAGACTCTCCTGTGCAGCCTCTGGAGTCACTTTCAGTACCGCCTGGATGAACTGGGTCCGCCAGGCTCCAGGGAAGGGGCTGGAGTGGGTTGGCCGGATTAAAAACAAAATTGATGGTGGGACAACAGACTACGTTGCACCCGTGAAAGGCAGATTCACCATCTCAAGAGATGATTCAAAAAACACGCTGTATCTGCAAATGAACAGCCTGAAAACCGAGGACACAGCCGTATATTACTGTACCACAGAAGGCTGGAACACGGACTACTGGGGCCAGGGAACCCTGGTCACCGTCTCCTCA |
| | | | SEQ ID NO: 24795 | SEQ ID NO: 28801 |
| | | AA | EIVLTQSPGTLSLSPGERATLSCRASQSVSSSYLAWYQQKPGQAPRLLIYGTSSRATGIPDRFSGSGSGTDFTLTISRLEPEDFAVYYCQQYGSSRSFGQGTKLEIK | EVQLVESGGGLVKPGGSLRLSCAASGVTFSTAWMNWVRQAPGKGLEWVGRIKNKIDGGTTDYVAPVKGRFTISRDDSKNTLYLQMNSLKTEDTAVYYCTTEGWNTDYWGQGTLVTVSS |
| | | | SEQ ID NO: 24796 | SEQ ID NO: 28802 |
| **iPS:393804** | **21-225_5H7** | NA | GACATCCAGATGACCCAGTCTCCATCCTCCCTGTCTGCATCTGTTGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGTTACATCCAGTTTGCAAGGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAACATAATAGTTACCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAACAGAAGTAGTTATTACTGGGGCTGGATCCGCCAGCCCCCAGGGAAGGGGCTGGAGTGGATTGGGAATATCTATTATAGTGGGACCACCTACTACAACCCGTCCCTCAAGAGTCGAGTCACCATATCCGTAGACACGTCCAAGAACCAGTTCTCCCTGAACCTGAGCTCTGTGACCGCCGCAGACACGGCTGTATATTCCTGTGCGAGACATGGAAAAGACTGGGGCCTTGACTACTGGGGCCAGGGAACCCTGGTCACCGTCTCCTCA |
| | | | SEQ ID NO: 24797 | SEQ ID NO: 28803 |

FIGURE 50

| | | | | |
|---|---|---|---|---|
| | | AA | DIQMTQSPSSLSASVGDRVTITCRASQGIRNDLGWYQQKPGKAPKRLIYVTSSLQGGVPSRFSGSGSGTEFTLTISSLQPEDFATYYCLQHNSYPLTFGGGTKVEIK | QLQLQESGPGLVKPSETLSLTCTVSGGSINRSSYYWGWIRQPPGKGLEWIGNIYYSGTTYYNPSLKSRVTISVDTSKNQFSLNLSSVTAADTAVYSCARHGKDWGLDYWGQGTLVTVSS |
| | | | SEQ ID NO: 24798 | SEQ ID NO: 28804 |
| iPS:393806 | 21-225_6A6 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAGTAGTTACCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGCAGAAGTAGTTACTACTGGGGCTGGATCCGCCAGCCCCCAGGGAAGGGGCTGGAGTGGATTGGGAATATCTATTATAGTGGGATCCCCTACTACAACCCGTCCCTCAAGAGTCGGGTCAACATATCCGTAGACACGTCCAAGAACCAGTTCTCCCTGAAGCTGAACTCTGTGACCGCCGCAGACACGGCTGTGTATTACTGTGCGAGACACAGCAGCAGCTGGTCTCTTGACTACTGGGGCCAGGGAACCCTAGTCACCGTCTCCTCA |
| | | | SEQ ID NO: 24799 | SEQ ID NO: 28805 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHSSYPLTFGGGTKVEIK | QLQLQESGPGLVKPSETLSLTCTVSGGSISRSSYYWGWIRQPPGKGLEWIGNIYYSGIPYYNPSLKSRVNISVDTSKNQFSLKLNSVTAADTAVYYCARHSSSWSLDYWGQGTLVTVSS |
| | | | SEQ ID NO: 24800 | SEQ ID NO: 28806 |

FIGURE 50

| | | | | |
|---|---|---|---|---|
| **iPS:393808** | **21-225_1A2** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTCACCCTCTCACTTTCGGCGGAGGGACCAAGGTGGAGATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATACCATGAACTGGGTCCGCCAGGCTCCAGGGAAGGGGCTGGAGTGGGTCTCATCCATTAGTGGTAGTGGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGGGAGCAGCTCGTCCGGGTTTGACTACTGGGGCCAGGGAACCCTGGTCACCGTCTCCTCA |
| | | | SEQ ID NO: 24801 | SEQ ID NO: 28807 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSHPLTFGGGTKVEIK | EVQLVESGGGLVKPGGSLRLSCAASGFTFSSYTMNWVRQAPGKGLEWVSSISGSGSYIYYADSVKGRFTISRDNAKNSLYLQMNSLRAEDTAVYYCARGSSSSGFDYWGQGTLVTVSS |
| | | | SEQ ID NO: 24802 | SEQ ID NO: 28808 |
| **iPS:393810** | **21-225_5A4** | NA | GACATCCAGATGACCCAGTCTCCATCTTCCGTGTCTGCATCTGTAGGAGACAGAGTCACCATCACTTGTCGGGCGAGTCAGGGTATTAGCACCTGGTTAGCCTGGTATCAACAGAAACCAGGGAAAGCCCCTAAACTCCTGATCTATGATGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGGCTAACAGTTTCCCGTGGACGTTCGGCCAAGGGACCAAGGTGGAAATCAAA | GAGGTGCAGGTGTTGGAGTCTGGGGGAGGCTTGGTACAGCCGGGGGGGTCCCTGAGACTCTCCTGTGCAGCCTCTGGACTCACCTTTAGCAGCTCTGCCATGAGCTGGGTCCGCCAGGCTCCAGGGAAGGGGCTGGAGTGGGTCTCAGCTATTAGTGGTCGTGGTGGTAACACATTCTACACAGACTCCGTGAAGGGCCGGTTCACCATTTCCAGAGACAATTCCAAGAACACGCTGTATCTGCAAATGAACAGCCTGAGAGCCGAGGACACGGCCGTATATTACTGTGCGAAACTGGGGAAAGACTACTACTACTACGGTATGGACGTCTGGGGCCAAGGGACCACGGTCACCGTCTCCTCA |
| | | | SEQ ID NO: 24803 | SEQ ID NO: 28809 |

FIGURE 50

| | | | | |
|---|---|---|---|---|
| | | AA | DIQMTQSPSSVSASVGDRVTITCRASQGISTWLAWYQQKPGKAPKLLIYDASSLQSGVPSRFSGSGSGTDFTLTISSLQPEDFATYYCQQANSFPWTFGQGTKVEIK | EVQVLESGGGLVQPGGSLRLSCAASGLTFSSSAMSWVRQAPGKGLEWVSAISGRGGNTFYTDSVKGRFTISRDNSKNTLYLQMNSLRAEDTAVYYCAKLGKDYYYGMDVWGQGTTVTVSS |
| | | | SEQ ID NO: 24804 | SEQ ID NO: 28810 |
| **iPS:393812** | **21-225_6A11** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTTTGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATGACTGTGCGAGAGATCTTGGCTGGACGGAAGAGTACTGGGGCCAGGGAACCCTGGTCACCGTCTCCTCA |
| | | | SEQ ID NO: 24805 | SEQ ID NO: 28811 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPWTFGQGTKVEIK | QVQLVESGGGVVQPGRSLRLSCAASGFTFSDYGMHWVRQAPGKGLEWVAVIWFDGSNKYYADSVKGRFTISRDNSKNTLYLQMNSLRAEDTAVYDCARDLGWTEEYWGQGTLVTVSS |
| | | | SEQ ID NO: 24806 | SEQ ID NO: 28812 |

FIGURE 50

| | | | | |
|---|---|---|---|---|
| **iPS:393814** | **21-225_7F4** | NA | GACATCCAGATGACCCAGTCTCCATCCTCCCTGTCTGCATCTGTAGGAGACAGAGTCACCATCACTTGCCGGACAAGTCAGGGCATTAGAAATGATTTAGGCTGGTATCAGCAGAAACCAGGGAAAGCCCCTAAGCGCCTGATTTATGCTGCATCCAATTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAGTGCTTACCCGCTCACTTTCGGCGGAGGGACCAAGGTGGAGATCAAA | CAACTGCAGCTGCAGGAGTCGGGCCCAGGACTGGTGAAGCCTTCGGAGACCCTGTCCCTCACCTGCACTGTCTCTGGTGGCTCCATCAGCAGGAGTAGTTACTACTGGGGCTGGATCCGCCAGCCCCCAGGGAAGGGGCTGGAGTGGATTGGGAATATCTATTATAGTGGGGCCACCTACTACAACCCGTCCCTCAAGAGTCGAGTCACCATCTCCGTAGACACGTCCACGAACCAGTTCTCCCTGAAGCTGAGCTCTGTGACCGCCGCAGACACGGCTGTGTATTACTGTGCGAGACATAGCGGCAGCTGGTCCCTTGACTACTGGGGCCAGGGAACCCTGGTCACCGTCTCCTCA |
| | | | SEQ ID NO: 24807 | SEQ ID NO: 28813 |
| | | AA | DIQMTQSPSSLSASVGDRVTITCRTSQGIRNDLGWYQQKPGKAPKRLIYAASNLQSGVPSRFSGSGSGTEFTLTISSLQPEDFATYYCLQHSAYPLTFGGGTKVEIK | QLQLQESGPGLVKPSETLSLTCTVSGGSISRSSYYWGWIRQPPGKGLEWIGNIYYSGATYYNPSLKSRVTISVDTSTNQFSLKLSSVTAADTAVYYCARHSGSWSLDYWGQGTLVTVSS |
| | | | SEQ ID NO: 24808 | SEQ ID NO: 28814 |
| **iPS:393816** | **21-225_6D4** | NA | GACATCCAGATGACCCAGTCTCCATCCTCCCTGTCTGCTTCTGTAGGAGACAGAGTCACCATCACTTGCCGGGCAAGTCAGGCCATTAGAAATGATTTAGGCTGGTATCAGCAGAAACCAGGGAAAGCCCCTAAGCGTCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAGTAGTTACCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGCAGAAGTAGTTCCTACTGGGGCTGGATCCGCCAGCCCCCAGGGAAGGGGCTGGAGTGGATTGGAAATATCTATTATAGTGGGAGCGCCTACTACATTCCGTCCCTCAAGAGTCGAGTCACCATATCCGTAGCCACGTCCAAGAACCAGTTCTCCCTGAACCTGACCTCTGTGACCGCCGCAGACACGGCTGTGTATTACTGTGCGCGACACAGCAGCAGCTGGTCTCTTGACTGCTGGGGCCAGGGAACCCTGGTCACCGTCTCCTCA |
| | | | SEQ ID NO: 24809 | SEQ ID NO: 28815 |

FIGURE 50

| | | | | |
|---|---|---|---|---|
| | | AA | DIQMTQSPSSLSASVGDRVTITCRASQAIRNDLGWYQQKPGKAPKRLIYAASSLQSGVPSRFSGSGSGTEFTLTISSLQPEDFATYYCLQHSSYPLTFGGGTKVEIK | QLQLQESGPGLVKPSETLSLTCTVSGGSISRSSSYWGWIRQPPGKGLEWIGNIYYSGSAYYIPSLKSRVTISVATSKNQFSLNLTSVTAADTAVYYCARHSSSWSLDCWGQGTLVTVSS |
| | | | SEQ ID NO: 24810 | SEQ ID NO: 28816 |
| iPS:393818 | 21-225_6G12 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCACTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATTTGGTATGATAGAAGTAATAACTACTATGCAGACTCCGTGAAGGGCCGATTCACCATCTCCAGAGACAATTCCAAGAATACGCTGTATCTGCAAATGAACAGCCTGAGAGCCGAGGACACGGCTGTGTATTACTGTGCGAGAGAACTGGGGTTCCGGTCTGACTACTGGGGCCAGGGAACCCTGGTCACCGTCTCCTCA |
| | | | SEQ ID NO: 24811 | SEQ ID NO: 28817 |
| | | AA | DIQMTQSPSSLSASVGDRVTITCRASQGIRNDLGWYQQKPGKAPKRLIYAASTLQSGVPSRFSGSGSGTEFTLTISSLQPEDFATYYCLQHNSYPLTFGGGTKVEIK | QVQLVESGGGVVQPGRSLRLSCAASGFTFSSYGMHWVRQAPGKGLEWVAVIWYDRSNNYYADSVKGRFTISRDNSKNTLYLQMNSLRAEDTAVYYCARELGFRSDYWGQGTLVTVSS |
| | | | SEQ ID NO: 24812 | SEQ ID NO: 28818 |

FIGURE 50

| | | | | |
|---|---|---|---|---|
| **iPS:393820** | **21-225_8H7** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATTTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGTTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTCCAGCGTCTGGATTCACCTTCAGTGACTTTGGCATGCACTGGGTCCGCCAGGCTCCAGGCAAGGGGCTGGAGTGGGTGGCAGTTATTTGGTATGAAGAAAATAATCAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGCTGGGGTTCCGGTCTGACTACTGGGGCCAGGGAACCCTGGTCACCGTCTCCTCA |
| | | | SEQ ID NO: 24813 | SEQ ID NO: 28819 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPFTFGGGTKVEIK | QVQLVESGGGVVQPGRSLRLSCPASGFTFSDFGMHWVRQAPGKGLEWVAVIWYEENNQYYADSVKGRFTISRDNSKNTLYLQMNSLRAEDTAVYYCARELGFRSDYWGQGTLVTVSS |
| | | | SEQ ID NO: 24814 | SEQ ID NO: 28820 |
| **iPS:393822** | **21-225_15B11** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAATGGGTGGCAGTTATATGGTATGAGGAAAGTAATAAATACTATACAGACTCCGTGAAGGGCCGATTCACCATCTCCAGAGACAATTCCAAGAACACGCTGTATCTGCAAATGAACAGCCTGAGACCCGAGGACACGGCTGTGTATTACTGTGCGAGAGAAGTGGGATTCACTGAGGACTACTGGGGCCAGGGAACCCTGGTCACCGTCTCCTCA |
| | | | SEQ ID NO: 24815 | SEQ ID NO: 28821 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPFTFGPGTKVDIK | QVQLVESGGGVVQPGRSLRLSCAASGFTFSNYGMHWVRQAPGKGLEWVAVIWYEESNKYYTDSVKGRFTISRDNSKNTLYLQMNSLRPEDTAVYYCAREVGFTEDYWGQGTLVTVSS |
| | | | SEQ ID NO: 24816 | SEQ ID NO: 28822 |
| **iPS:393824** | **21-225_10F12** | NA | GACATCCAGATGACCCAGTCTCCATCCTCCCTGTCTGCATCTGTAGGAGACAGAGTCACCATCACTTGCCGGGCAAGTCAGAACATTAGTAGTTATTTAAATTGGTATCAGCAGAAACCAGGGAAAGCCCCTAAACTCCTGATCTATGCTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACAATACCCCCTTCTTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGAATTCACCTTTAGCAGCTATGCCATGAGCTGGGTCCGCCAGGCTCCAGGGAAGGGGCTGGAGTGGGTCTCAATTATTAGTGGTCGTGGTGGTAACACATTCTACGCAGACTCCGTGAAGGGCCGGTTCACCATCTCCAGAGACAATTCCAAGAACACTCTGTATCTGCAAATGAACAGCCTGAGAGCCGAGGACACGGCCGTATATTACTGTGCGTCCCGTGTAGCAGTGGCTGGCTCGGAGGCTTTTGCTATCTGGGGCCAAGGGACAATGGTCACCGTCTCTTCA |
| | | | SEQ ID NO: 24817 | SEQ ID NO: 28823 |
| | | AA | DIQMTQSPSSLSASVGDRVTITCRASQNISSYLNWYQQKPGKAPKLLIYAASSLQSGVPSRFSGSGSGTDFTLTISSLQPEDFATYYCQQSYNTPFFTFGPGTKVDIK | EVQLLESGGGLVQPGGSLRLSCAASEFTFSSYAMSWVRQAPGKGLEWVSIISGRGGNTFYADSVKGRFTISRDNSKNTLYLQMNSLRAEDTAVYYCASRVAVAGSEAFAIWGQGTMVTVSS |
| | | | SEQ ID NO: 24818 | SEQ ID NO: 28824 |

FIGURE 50

| | | | | |
|---|---|---|---|---|
| **iPS:393826** | **21-225_10G5** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATTTGGTATGAAGAAAATAATCAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTTTATTACTGTACGAGAGAACTGGGGTTCCGGTCTGACTACTGGGGCCAGGGAACCCTGGTCACCGTCTCCTCA |
| | | | SEQ ID NO: 24819 | SEQ ID NO: 28825 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVEIK | QVQLVESGGGVVQPGRSLRLSCAASGFTFSNYGMHWVRQAPGKGLEWVAVIWYEENNQYYADSVKGRFTISRDNSKNTLYLQMNSLRAEDTAVYYCTRELGFRSDYWGQGTLVTVSS |
| | | | SEQ ID NO: 24820 | SEQ ID NO: 28826 |
| **iPS:393828** | **21-225_10H12** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGGTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATTTGGTATGAAGACAATAATCAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGTTCCGGTCTGACTACTGGGGCCAGGGAACCCTGGTCACCGTCTCCTCA |
| | | | SEQ ID NO: 24821 | SEQ ID NO: 28827 |

FIGURE 50

| | | | | |
|---|---|---|---|---|
| | | AA | DIQMTQSPSSLSASVGDRVTITCRASQGIRNDLGWYQQKPGKAPKRLIYGASSLQSGVPSRFSGSGSGTEFTLTISSLQPEDFATYYCLQHNSYPLTFGGGTKVEIK | QVQLVESGGGVVQPGRSLRLSCAASGFTFSDYGMHWVRQAPGKGLEWVAVIWYEDNNQYYADSVKGRFTISRDNSKNTLYLQMNSLRAEDTAVYYCARELGFRSDYWGQGTLVTVSS |
| | | | SEQ ID NO: 24822 | SEQ ID NO: 28828 |
| **iPS:393830** | **21-225_12A1** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATTTGGTATGAAGAAAATAATCAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGTTCCGGTCTGACTACTGGGGCCAGGGAACCCTGGTCACCGTCTCCTCA |
| | | | SEQ ID NO: 24823 | SEQ ID NO: 28829 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVEIK | QVQLVESGGGVVQPGRSLRLSCAASGFTFSSYGMHWVRQAPGKGLEWVAVIWYEENNQYYADSVKGRFTISRDNSKNTLYLQMNSLRAEDTAVYYCARELGFRSDYWGQGTLVTVSS |
| | | | SEQ ID NO: 24824 | SEQ ID NO: 28830 |

FIGURE 50

| | | | | |
|---|---|---|---|---|
| **iPS:393832** | **21-225_14B2** | NA | GACATCCAGATGACCCAGTCTCCATCCTCCCTGTCTGCATCTGTTGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGTTACATCCAGTTTGCAAGGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAACATAATAGTTACCCGCTCACTTTCGGCGGAGGGACCAAGGTGGAGATCAAA | CAGCTGCAGCTGCAGGAGTCGGGGCCAGGACTGGTGAAGCCTTCGGAGACCCTGTCCCTCACCTGCACTGTCTCTGGTGGCTCCATCAACAGAAGTAGTTATTACTGGGGTTGGATCCGCCAGCCCCCAGGGAAGGGGCTGGAGTGGATTGGGAATATCTATTATAGTGGGACCACCTACTACAACCCGTCCCTCAAGAGTCGAGTCACCATATCCGTAGACACGTCCAAGAACCAGTTCTCCCTGAACCTGAGCTCTGTGACCGCCGCAGACACGGCTGTATATTCCTGTGCGAGACATGGAAAAGACTGGGGCCTTGACTACTGGGGCCAGGGAGCCCTGGTCACCGTCTCCTCA |
| | | | SEQ ID NO: 24825 | SEQ ID NO: 28831 |
| | | AA | DIQMTQSPSSLSASVGDRVTITCRASQGIRNDLGWYQQKPGKAPKRLIYVTSSLQGGVPSRFSGSGSGTEFTLTISSLQPEDFATYYCLQHNSYPLTFGGGTKVEIK | QLQLQESGPGLVKPSETLSLTCTVSGGSINRSSYYWGWIRQPPGKGLEWIGNIYYSGTTYYNPSLKSRVTISVDTSKNQFSLNLSSVTAADTAVYSCARHGKDWGLDYWGQGALVTVSS |
| | | | SEQ ID NO: 24826 | SEQ ID NO: 28832 |
| **iPS:393836** | **21-225_15A2** | NA | GACATCCAGATGACCCAGTCTCCATCCTCACTGTTTGCATTTATAGGAGACAGAGTCACCATCACTTGTCGGGCGAGTCAGGGCATTAGCAATTATTTAGCCTGGTTTCAGCAGAAACCTGGAAAAGCCCCTAAGTCCCTGATTTTTGCTGCATCCAGTTTGCAAAGTGGGGTCCCATCAAAGTTTAGCGGCAGTGGATTTGGGACAGATTTCACTTTTCCCATCAGCAGCCTGCAGCCTGAAGATTTTGCAAATTATTACTGCCAACAGTATTATAGTTACCCATTCACTTTCGGCCCTGGGACCCAAGTGGATGTCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATACCATGAACTGGGTCCGCCAGGCTCCAGGGAAGGGGCTGGAGTGGGTCTCATCCATTAGTGGTAGTGGTAGTTACATATACTACGCAGACTCAGTGAAGGGCCGATTCACCATCTCCAGAGACAACGCCAAGAACTCACTGTATCTGCAAATGAACGCCCTGAGAGCCGAGGACACGGCTGTGTATTACTGTGCGAGAGTGGCTTCATTTGACTACTGGGGCCAGGGAACCCTGGTCACCGTCTCCTCA |
| | | | SEQ ID NO: 24827 | SEQ ID NO: 28833 |

FIGURE 50

| | | | | |
|---|---|---|---|---|
| | | AA | DIQMTQSPSSLFAFIGDRVTITCRASQGISNYLAWFQQKPGKAPKSLIFAASSLQSGVPSKFSGSGFGTDFTFPISSLQPEDFANYYCQQYYSYPFTFGPGTQVDVK | EVQLVESGGGLVKPGGSLRLSCAASGFTFSSYTMNWVRQAPGKGLEWVSSISGSGSYIYYADSVKGRFTISRDNAKNSLYLQMNALRAEDTAVYYCARVASFDYWGQGTLVTVSS |
| | | | SEQ ID NO: 24828 | SEQ ID NO: 28834 |
| iPS:393838 | 21-225_6G2 | NA | GACATCCAGATGACCCAGTCTCCATCCTCCCGGTCTGCATTTGTTGGAGACAGAGTCACCATCACTTACCGGGCAAGTCAGGGCATTAGAAATGATTTAGGCTGGTATCAGCAGAAACCAGGGAAAGCCCCTCAGCGCCTGATCTATGCTGCATCCAGTCTGCAAAGTGGGGTCCCATCAAGGTTCAGCGGCAGTGGATATGGGACTGAATTCAATATCACAATCAGCAGCTTGCAGCCTGAAGATTTTGCAATTTATTACTGTATACAGCATAATAGTTACCTGTGGACGTTCGGCCAAGGGACCAAGGTGGAAATCACA | CTGGTGCAGCTGGTGGAGTCTGGGGGAGGCGTGGTCCAGCCTGGGAAGTCCCTGAGACTCTCCTGTGCAGCGTCTGGATTCACCTTCAGTGACTATGGCATACACTGGGTCCGCCAGGCTCCAGGCAAGGGGCTGGAGTGGGTGGCAGTCATTTGGTTTGATGGAAGTAATAAATACTATGCAGACTCCGTGAAGGGCCGATTCACCATCTCCAGCGACAATTCCAAAAACACGCTGTATCTGCAAATGAACAGCCTGAGAGCCGAGGACACGGCTGTGTATTACTGTGCGAGAGATCTTGGCTGGACGGAAGAGTACTGGGGCCAGGGAACCCTGGTCACCGTCTCCTCA |
| | | | SEQ ID NO: 24829 | SEQ ID NO: 28835 |
| | | AA | DIQMTQSPSSRSAFVGDRVTITYRASQGIRNDLGWYQQKPGKAPQRLIYAASSLQSGVPSRFSGSGYGTEFNITISSLQPEDFAIYYCIQHNSYLWTFGQGTKVEIT | LVQLVESGGGVVQPGKSLRLSCAASGFTFSDYGIHWVRQAPGKGLEWVAVIWFDGSNKYYADSVKGRFTISSDNSKNTLYLQMNSLRAEDTAVYYCARDLGWTEEYWGQGTLVTVSS |
| | | | SEQ ID NO: 24830 | SEQ ID NO: 28836 |

FIGURE 50

| | | | | |
|---|---|---|---|---|
| **iPS:393840** | **21-225_3F8** | NA | GACTTCCAGATGACCCAGTCTCCATCCTCCCTGTCTGCATCTGTAGGAGACAGAGTCACCATCACTTGCCGGGCAAGTCAGAGTATTCTCAGCTATTTAAATTGGTATCGGCAGAAACCAGGGAGAGCCCCTCAGGTCCTGATCCATACTACATCCAGTTTGCAAAGTGGGGTCCCATCAAGGTTCAGTGGCAGTGGATCTGGGACAGTTTTCACTCTCACCATCAGCAGTCTGCAACCTGAAGATTTTGCAACTTACTACTGTCAACAGACTTACAGTACCCCGCTCACTTTCGGCGGAGGGACCAGGGTGGAGATCAAC | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGGGGCAGTTATATGGCATGATGGAAGTAATAAATACTATGCAGACTCCGTGAAGGGCCGATTCACCATCTCCAGAGACAATGCCAAGAACACGCTGTATCTGCAAATGAACAGCCTGAGAGCCGAGGACACGGCTGTGTATTACTGTGCGAGAGATCTGAGTATGGGCGGTATGGACGTCTGGGGCCAAGGGACCACGGTCACCGTCTCCTCA |
| | | | SEQ ID NO: 24831 | SEQ ID NO: 28837 |
| | | AA | DFQMTQSPSSLSASVGDRVTITCRASQSILSYLNWYRQKPGRAPQVLIHTTSSLQSGVPSRFSGSGSGTVFTLTISSLQPEDFATYYCQQTYSTPLTFGGGTRVEIN | QVQLVESGGGVVQPGRSLRLSCAASGFTFSSYGMHWVRQAPGKGLEWGAVIWHDGSNKYYADSVKGRFTISRDNAKNTLYLQMNSLRAEDTAVYYCARDLSMGGMDVWGQGTTVTVSS |
| | | | SEQ ID NO: 24832 | SEQ ID NO: 28838 |
| **iPS:393844** | **21-225_3G7** | NA | GACATCCAGATGACCCAGTCTCCATCCTCCCTGTCTGCATCTGTCGGAGACAGAGTCACCGTCACTTGCCGGGCAAGTCAGAACATTTACAGGTATTTAAATTGGTATCAGGGGAGACCAGGGAGAGCCCCTAAACTCATGATCTATGCTGCATCCAGTTCGCAAAGTGGGGTCCCATCAAGATTCAGTGGCAGTGGATCTGGGACAGATTTCACTGTCACCATCAGTAGTCTTCAACCTGAAGATTTTGCAACTTATTACTGTCAACAGAGTTACAGTCCCCCTTTCACTTTCGGCGGAGGGGCCAAGGTGGAGATCGAC | CAGGTGCAGCTGGTGGAGTCTGGGGGAGGCGTGGTCCAGCCTGGGAGGTCCCTGAGACTCTCCTGTGCAGCGTCTGGATTCAACTTCAGTAACTATGGCATGCACTGGGTCCGCCAGGCTCCAGGCAAGGGGCTGGAGTGGGTGGCAGTCATATGGCATGATGGAAGTAATAAATACTATGTAGACTCCGTGAAGGGCCGATTCACCATCTCCAGAGACAATTCCAAGAACACGGTGTATCTGCAAATGAACAGCCTGAGAGCCGAGGACACGGCTGTGTATTACTGTGCGAGAGATGAGAGGCTGGGGATTTTTGACTACTGGGGCCAGGGAACCCTGGTCACCGTCTCCTCA |
| | | | SEQ ID NO: 24833 | SEQ ID NO: 28839 |

FIGURE 50

| | | | | |
|---|---|---|---|---|
| | | AA | DIQMTQSPSSLSASVGDRVTVTCRASQNIYRYLNWYQGRPGRAPKLMIYAASSSQSGVPSRFSGSGSGTDFTVTISSLQPEDFATYYCQQSYSPPFTFGGGAKVEID | QVQLVESGGGVVQPGRSLRLSCAASGFNFSNYGMHWVRQAPGKGLEWVAVIWHDGSNKYYVDSVKGRFTISRDNSKNTVYLQMNSLRAEDTAVYYCARDERLGIFDYWGQGTLVTVSS |
| | | | SEQ ID NO: 24834 | SEQ ID NO: 28840 |
| **iPS:393848** | **21-225_4H2** | NA | GACATCCAGATGACCCTGTCTCCATCCTCCCTGTCTGCATCTGTAGGAGACAGAGTCACCATCACTTGCCGGGCAATTCAGAACATTAGCAGCTATTTAAATTGGTATCAGCAGAAACCAGGGAAAGCCCCTAAACTCGTGATCTATGCTGCATCCAGCTTGCAAAGTGGGGTCCCATCAAGGTTCAGTGGCAGAGGATCTGGGACAGATTTCACTCTCACCATCGGTTGTGTGCAACGTGAAGATTTTGCAACTTACTACTGTCAACAGAGTTACAGAACCCCCTTATTCACTTTCGGCCCTGGGACCAAGGTTGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCGGCTATGCCATGAACTGGGTCCGCCAGGCTCCAGGGATGGGGCTGGAGTGGGTCTCAGTTATTAGTCGTAGTGGTGGTTACACATACTACGCGGACTCCGTGAAGGGCCGGTTCACCATCTCCAGAGACAATTCCAAGAACACGCTGTATCTGCAAATGAACAGCCTGAGAGCCGAGGACACGGCCGTCTATTACTGTGCGTCCCGTTTAGCAGTGGCTGGCTCGGAGGCTTTTGATATCTGGGGCCAAGGGACAATGGTCACCGTCTCTTCA |
| | | | SEQ ID NO: 24835 | SEQ ID NO: 28841 |
| | | AA | DIQMTLSPSSLSASVGDRVTITCRAIQNISSYLNWYQQKPGKAPKLVIYAASSLQSGVPSRFSGRGSGTDFTLTIGCVQREDFATYYCQQSYRTPLFTFGPGTKVDIK | EVQLLESGGGLVQPGGSLRLSCAASGFTFSGYAMNWVRQAPGMGLEWVSVISRSGGYTYYADSVKGRFTISRDNSKNTLYLQMNSLRAEDTAVYYCASRLAVAGSEAFDIWGQGTMVTVSS |
| | | | SEQ ID NO: 24836 | SEQ ID NO: 28842 |

FIGURE 50

| | | | | |
|---|---|---|---|---|
| **iPS:393852** | **21-225_12A10** | NA | GACGTCCAGATGACCCAGTCTCCATCCTCCCTGTCTGCATCTGCAGGAGACAGAGTCACCATCACTTGCCGGGCAAGTCAGAACATTTACAGCTATTTAAATTGGTATCAGCAGAAACCAGGGAGAGCCCCTAAGGTCCTGATCCATACTGCATCCAGTTTGCAAAGTGGGGTCCCATCAAGGTTCAGTGGCAGTGGATCTGGGGCAGATTTCACTCTCACCATCAACAGTCTGCAACCTGAAGATTTTGCAACTTACTACTGTCAGCAGAGTTACAGTCCCCCTCTCACTTTCGGCGGAGGGACCAAGGTAGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGCATGATGAAAGTAATAAATACTATACAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGACGAGAGGCTGGGGATTTTTGACTACTGGGGCCAGGGAACCCTGGTCACCGTCTCCTCA |
| | | | SEQ ID NO: 24837 | SEQ ID NO: 28843 |
| | | AA | DVQMTQSPSSLSASAGDRVTITCRASQNIYSYLNWYQQKPGRAPKVLIHTASSLQSGVPSRFSGSGSGADFTLTINSLQPEDFATYYCQQSYSPPLTFGGGTKVEIK | QVQLVESGGGVVQPGRSLRLSCAASGFTFSSYGMHWVRQAPGKGLEWVAVIWHDESNKYYTDSVKGRFTISRDNSKNTLYLQMNSLRAEDTAVYYCARDERLGIFDYWGQGTLVTVSS |
| | | | SEQ ID NO: 24838 | SEQ ID NO: 28844 |
| **iPS:393854** | **21-225_7H11** | NA | GACATCCAGATGACCCAGTCTCCATCCTCCGCATCTGCATCTGTAGGAGTCAGAGTCACCATCATTTGCCTGGCAAGTCAGGGCATTAGAAATGATTTAGGCTGGTATCAGCAGAAACCAGGGAAAGCCCCTAAGCGCATAATCTATGTTGCATGTAGTTTCCAAAGTGGGGTCCCATCAAGGTTCAGCGGCAGTGGATATGGGACAGAATTCACTCTCACAATCAGCATCATGCAGCCTGAAGATTTCGCAACTTATTACTGTCTACAACATAATCTTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTTCAGCGTCTGGATTCACCTTCAGTGACTATGGCATGCACTGGGTCCGCCAGGCTCCAGGCAAGGGGCTGGAGTGGGTGGCAGTTATATGGTATGATGAAAATAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGTTCCGGTCTGACTACTGGGGCCAGGGAACCCTGGTCACCGTCTCCTCA |
| | | | SEQ ID NO: 24839 | SEQ ID NO: 28845 |

FIGURE 50

| | | | | |
|---|---|---|---|---|
| | | AA | DIQMTQSPSSASASVGVRVTIICLASQGIRNDLGWYQQKPGKAPKRIIYVACSFQSGVPSRFSGSGYGTEFTLTISIMQPEDFATYYCLQHNLYPLTFGGGTKVEIK | QVQLVESGGGVVQPGRSLRLSCSASGFTFSDYGMHWVRQAPGKGLEWVAVIWYDENNKYYADSVKGRFTISRDNSKNTLYLQMNSLRAEDTAVYYCARELGFRSDYWGQGTLVTVSS |
| | | | SEQ ID NO: 24840 | SEQ ID NO: 28846 |
| **iPS:393856** | **21-225_14C2** | NA | GACATCCAGATGATCCAGTCTCCATCCTCCCTGTTTGCATGTGTAGGAGACAGAGTCATCATCACTTGCCGGGCAAGTCAGGGCATTAGAAATGATTTAGACTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCGTGCAGCGTGAAGATTTTGCAACTTATTACTGTGTACAGCATAATAGTTACCCGCTCACTTTCGGCGGAGGGACCAAGGTGGAGATCAGA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGACGAAAGTAATAAATACTATGAAGACTCCGTGAAGGGCCGATTCACCATCTCCAGAGACAATTCCAAGAACACGCTGTATCTGCAAATGAAAAGCCTGAGAGCCGAGGACACGGGTGTGTATTACTGTGCGAGAGAAGTGGGATTCCGGTCTGACTACTGGGGCCAGGGAACCCTGGTCACCGTCTCCTCA |
| | | | SEQ ID NO: 24841 | SEQ ID NO: 28847 |
| | | AA | DIQMIQSPSSLFACVGDRVIITCRASQGIRNDLDWYQQKPGKAPKRLIYAASSLQSGVPSRFSGSGSGTEFTLTISSVQREDFATYYCVQHNSYPLTFGGGTKVEIR | QVQLVESGGGVVQPGRSLRLSCAASGFTFSDYGMHWVRQAPGKGLEWVAVIWYDESNKYYEDSVKGRFTISRDNSKNTLYLQMKSLRAEDTGVYYCAREVGFRSDYWGQGTLVTVSS |
| | | | SEQ ID NO: 24842 | SEQ ID NO: 28848 |

FIGURE 50

| | | | | |
|---|---|---|---|---|
| **iPS:393862** | **21-225_5G2** | NA | GACATCCAGATGACCCAGTCTCCATCCTCCCCGTCTGCATCTGTAGGAGACAGAGTCACCATCACTTTCCGGGCAAGTCAGAACATTATTAGTTATTTAAATTGGTATCAGCAGAAACCAGGGAAAGCCCGTAAGCTCGTGATCTATGGTGCATCCAGTTTGCAAAGTGGGGTCCCATCAAGGTTCAGTGGCAGTGGATCTGGGACAGATTTCACTCTCAACATCAGAAGTCTGCAACCTGAAGATTTTGCAACTTACTACTGTCAACAGAGTTACAGTACTCCCTTATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGAATTCACCTTTAGCAGCTATGCCATGAGCTGGGTCCGCCAGGCTCCAGGGAAGGGGCTGGAGTGGGTCTCAGTTATTAGTGGTCGTGGTGTTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGTCCCGTATAGCAGTGGCTGGCTCGGAGGCTTTTGATATCTGGGGCCAAGGGACAATGGTCACCGTCTCTTCA |
| | | | SEQ ID NO: 24843 | SEQ ID NO: 28849 |
| | | AA | DIQMTQSPSSPSASVGDRVTITFRASQNIISYLNWYQQKPGKARKLVIYGASSLQSGVPSRFSGSGSGTDFTLNIRSLQPEDFATYYCQQSYSTPLFTFGPGTKVDIK | EVQLLESGGGLVQPGGSLRLSCAASEFTFSSYAMSWVRQAPGKGLEWVSVISGRGVNTFYADSVKGRFTISRDNSKNTLYLQMNSLRAEDTAVYYCASRIAVAGSEAFDIWGQGTMVTVSS |
| | | | SEQ ID NO: 24844 | SEQ ID NO: 28850 |
| **iPS:393864** | **21-225_4C5** | NA | GACATCCAGATGACCCAGTCTCCATCCTCCCTGTCTGCATCTGTAGGACACAGAGTCCACCTCACTTGCCGGGCAAGTCGGGGCATCAGAGGTGATTTAGGTTGGTATCGCCAGAAACCAGGGAAAGCCCCTAAGCGCTTGATCTATGCTGCATCCAATTTGCAAAGTGGGGTCCCATCAAGGTTCAGCGGCAGTGGATATGGGACAGAATTCACTCTCACAATCGGCAGCCTGCAGCCTGAAGATTTTGCAACTTATTACTGTCTACAGCATTATAGTTACCCTCGGACGTTCGGCCAAGGGACCAAGGTGGAAATCAAA | CAGGTGCAACTGGTGGAGTCTGGGGGAGGCGTGGTCCAGCCTGGGAGGTCCCTGAGACTCTCCTGTGCAGCGTCTGGATTCACCTTCAGTAGCTATGTCCTGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTATATCTGCAAATGAACAGCCTGAGAGCCGAGGACACGGCTGTTTATTACTGTGCGCGAGAAAAGTATACCAGCAGCTGGTACGACTACGGTATGGACGTCTGGGGCCAAGGGACCACGGTCACCGTCTCCTCA |
| | | | SEQ ID NO: 24845 | SEQ ID NO: 28851 |

FIGURE 50

| | | | | |
|---|---|---|---|---|
| | | AA | DIQMTQSPSSLSASVGHRVHLTCRASRGIRGDLGWYRQKPGKAPKRLIYAASNLQSGVPSRFSGSGYGTEFTLTIGSLQPEDFATYYCLQHYSYPRTFGQGTKVEIK | QVQLVESGGGVVQPGRSLRLSCAASGFTFSSYVLHWVRQAPGKGLEWVAVIWYDGSNKYYADSVKGRFTISRDNSKNTLYLQMNSLRAEDTAVYYCAREKYTSSWYDYGMDVWGQGTTVTVSS |
| | | | SEQ ID NO: 24846 | SEQ ID NO: 28852 |
| iPS:393866 | 21-225_14E3 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAACAAAAACCAGGGAAAGCCCCTAAGCGCATTATTTATTCTGCATCCAGTTTGCAAAGTGGGGTCCCATCAAGGTTCATCGGCAGTGGATGTGGGACTGAATTCACTCTCACAATCAGCAGCCTGCAGCGTGAAGATTTTGCAGCTTATTACAGTGTACAGCATTATAGTTACCCGTTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTTTGGCATGCACTGGGTCCGCCAGGCTCCAGGCAAGGGGCTGGAGTGGGTGGCAGTTATTTGGTATGAAGAAAATAATCAATACTATGCAGACTCCGTGAAGGGCCGATTCACCATCTCCAGAGACAATTCCAAGAACACGCTGTATCTGCAAATGAACAGCCTGAGAGCCGAGGACACGGCTTTTTATTACTGTGCGAGAGAGCTGGGGTTCCGGTCTGACTACTGGGGCCAGGGAACCCTGGTCACCGTCTCCTCA |
| | | | SEQ ID NO: 24847 | SEQ ID NO: 28853 |
| | | AA | DIQMTQSPSSLSASVGDRVTITCRASQGIRNDLGWYQQKPGKAPKRIIYSASSLQSGVPSRFIGSGCGTEFTLTISSLQREDFAAYYSVQHYSYPFTFGGGTKVEIK | QVQLVESGGGVVQPGRSLRLSCAASGFTFSDFGMHWVRQAPGKGLEWVAVIWYEENNQYYADSVKGRFTISRDNSKNTLYLQMNSLRAEDTAFYYCARELGFRSDYWGQGTLVTVSS |
| | | | SEQ ID NO: 24848 | SEQ ID NO: 28854 |

FIGURE 50

| | | | | |
|---|---|---|---|---|
| **iPS:393868** | **21-225_9C11** | NA | GACATCCAGATGACCCAGTCTCCATCCTCCCTGTCTGCATCTGTAGGAGACAGAGTCACCATCACTTGCCGGGCAAGTCAGAACATTAGAAATTATTTAAATTGGTATCAGCAGAAATCAGGGAGAGCCCCTAAGCTCCTGATCTATGTTGCATCCAGTTTGCAAAGTGGGGTCCCATCAAGGTTCAGTGGCAGTGGATCTGGGACAGAATTCACTCTCACCATCAACAGTCTGCAACCTGAAGATTTTGCAATTTATCACTGTCATCAGAGTAACAGTACTCCTCTCACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGCATGATGAAACTAATAAATACTATGCAGATTCCGTGAAGGGCCGATTCACCATCTCCAGAGACAATTCCAAGAACACGCTGTCTCTGCAAATGAACAGCCTGAGAGCCGAGGACACGGCTGTGTATTACTGTGCGAGAGACGAGAGGCTGGGGATTTTTGACTACTGGGGCCAGGGAACCCTGGTCACCGTCTCCTCA |
| | | | SEQ ID NO: 24849 | SEQ ID NO: 28855 |
| | | AA | DIQMTQSPSSLSASVGDRVTITCRASQNIRNYLNWYQQKSGRAPKLLIYVASSLQSGVPSRFSGSGSGTEFTLTINSLQPEDFAIYHCHQSNSTPLTFGQGTKVEIK | QVQLVESGGGVVQPGRSLRLSCAASGFTFSSYGMHWVRQAPGKGLEWVAVIWHDETNKYYADSVKGRFTISRDNSKNTLSLQMNSLRAEDTAVYYCARDERLGIFDYWGQGTLVTVSS |
| | | | SEQ ID NO: 24850 | SEQ ID NO: 28856 |
| **iPS:393870** | **21-225_7B1** | NA | GATATCCAGATGACCCAGGCTCCATCCTCACTGTCTGCTTCTGTAGGAGACAGAGTCACCATCACTTGTCGGGCGAGTCAGGACATTAGCAATCATTTAGTCTGGTTTCAGCAGAAACCAGGGAAAGCCCCTAAGTCCCTGATCTTTGCTGCATCCAGTTTACAAAGTGGGGTCCCATCACAGTTCAGCGGCAGTGGATCTGGAACAGATTTCACTCTCACCATCAGCATCCTGCAGCCTGAGGATTTTGCAACTTATTACTGCCACCAGTATAATAGTTACCCCTTCACTTTCGGCCCTGGGACCAAAGTGGATTTCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGACATGAGCTGGGTCCGCCAGGCTCCAGGAAAGGGGCTGGAGTGGGTCTCAACTATTAGTGGTAGTGGTGGTATCACATACTACGCAGACTCCGTGAAGGGCCGGTTCACCATCTCCAGAGACAATTCCAAGAAAACGCTGTATCTGCAAATGAACAGCCTGAGAGCCGAGGACACGGCCGTATATTTCTGTGCGAGAGATCGGGGCAGCGTCTGGGGCCAGGGAACCCTGGTCACCGTCTCCTCA |
| | | | SEQ ID NO: 24851 | SEQ ID NO: 28857 |

FIGURE 50

| | | | | |
|---|---|---|---|---|
| | | AA | DIQMTQAPSSLSASVGDRVTITCRASQDISNHLVWFQQKPGKAPKSLIFAASSLQSGVPSQFSGSGSGTDFTLTISILQPEDFATYYCHQYNSYPFTFGPGTKVDFK | EVQLLESGGGLVQPGGSLRLSCAASGFTFSSYDMSWVRQAPGKGLEWVSTISGSGGITYYADSVKGRFTISRDNSKKTLYLQMNSLRAEDTAVYFCARDRGSVWGQGTLVTVSS |
| | | | SEQ ID NO: 24852 | SEQ ID NO: 28858 |
| iPS:393872 | 21-225_2A11 | NA | GACATCCAGATGACCCAGTCTCCATCCTCCCTGTCTCCATCTGTAGGAGACAGAGTCACCATCACTTGCCGGGCAAGTCAGGGCATTAGAAATGATTTAGGCTGGTATCAGCAGAAACCAGGGAAAGCCCCTCAGCGCCTGATCTCTGCTGCATCCAGTTTGCAAAGTGGGGTCCCATCAAGGTTCAGCGGCAGTGGATCTGGGACAGATTTCACTCTCACAATCAGCAGTTTGCAGCCTGAAGATTTTGCAACTTATTACTGTCTACAGCATAATAGTTACCCGCTCACTTTCGGCGGAGGGACCAAGATGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGTAGGAGTAGTTACTACTGGGGCTGGATCCGGCAGCCCCCAGGGAAGGGGCTGGAGTGGATTGGGAATATCTATTATAGTGGGAGCACCTACTACAACCCGTCCGTCAAGAGTCGAGTCACCATATCCGTAGACACGTCCAAGAACCAGTTCTCCCTGAAGCTGAGTACTGTGACCGCCGCAGACACGGCTGTGTATTACTGTGCGAGACATGGAAAAGACTGGGGCCTTGAAGACTGGGGCCAGGGAACCCTGGTCACCGTCTCCTCA |
| | | | SEQ ID NO: 24853 | SEQ ID NO: 28859 |
| | | AA | DIQMTQSPSSLSPSVGDRVTITCRASQGIRNDLGWYQQKPGKAPQRLISAASSLQSGVPSRFSGSGSGTDFTLTISSLQPEDFATYYCLQHNSYPLTFGGGTKMEIK | QLQLQESGPGLVKPSETLSLTCTVSGGSISRSSYYWGWIRQPPGKGLEWIGNIYYSGSTYYNPSVKSRVTISVDTSKNQFSLKLSTVTAADTAVYYCARHGKDWGLEDWGQGTLVTVSS |
| | | | SEQ ID NO: 24854 | SEQ ID NO: 28860 |

FIGURE 50

| | | | | |
|---|---|---|---|---|
| **iPS:393874** | **21-225_4C8** | NA | GACATCCAGATGATCCAGTCTCCATCCTTCCTGTTTGCATGTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATATATGCTGCATCCAGTTTGCAAAGTGGGGTCCCATCAAGGTTCAGCGGCAGTGTATCTGGGACAGAATTCACTCTCACAATCAGCAGCCTGCAGCCTGAAGATTTCGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATTTGGTATGAAGAAAATAATCAATACTATGCAGACTCCGTGAAGGGCCGATTCACCATCTCCAGAGACAATTCCAAGAACACGCTGTATCTGCAAATGAACAGCCTGACAGCCGAGGACACGGCTGTTTATTACAGTCCGAGAGAAATGGGGTTCCTGTCTGACTACTGGGGCCAGGGAACCCTGGTCACCGTCTCCTCA |
| | | | SEQ ID NO: 24855 | SEQ ID NO: 28861 |
| | | AA | DIQMIQSPSFLFACVGDRVTITCRASQGIRNDLGWYQQKPGKAPKRLIYAASSLQSGVPSRFSGSVSGTEFTLTISSLQPEDFATYYCLQHNSYPLTFGGGTKVEIK | QVQLVESGGGVVQPGRSLRLSCAASGFTFSNYGMHWVRQAPGKGLEWVAVIWYEENNQYYADSVKGRFTISRDNSKNTLYLQMNSLTAEDTAVYYSPREMGFLSDYWGQGTLVTVSS |
| | | | SEQ ID NO: 24856 | SEQ ID NO: 28862 |
| **iPS:393876** | **21-225_9A1** | NA | GACATCCAGATGACCCAGTCTCCATCCTCCCGGTCTGCATTTGTTGGAGACAGAGTCACCATCACTTGCCGGGCAAGTCAGGGCATTAGAAATGATTTAGGGTGGTATCAGCAGAAACCAGGGAAAGCCCGTAAGCGCCTGATCTATACTGCATCCAGTTTGCAAAGTGGGGTCCCATCAAGGTTCAGCGGCAGTGGATATGGGACTGAATTCACTATCACAATCAGCAGCTTGCAGCCTGAAGATTTTGCAACTTATTACTGTATACAGCATAATAGTTACCTGTGGACGTTCGGCCAAGGGACCAAGGTGGAAATCAAA | CAGGTGCAGGTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTTTGATGGAAGTAATAAATACTATGCAGACTCCGTGAAGGGCCGATTCACCATCTCCAGAGACAATTCCAAGAACACGCTGTATCTGCAAATGAACAGTCTGAGAGCCGAGGACACGGCTGTGTATTACTGTGCGAGAGATCTTGGCTGGACGGAAGAGTACTGGGGCCAGGGAACCCCGGTCACCGTCTCCTCA |
| | | | SEQ ID NO: 24857 | SEQ ID NO: 28863 |

FIGURE 50

| | | | | |
|---|---|---|---|---|
| | | AA | DIQMTQSPSSRSAFVGDRVTITCRASQGIRNDLGWYQQKPGKARKRLIYTASSLQSGVPSRFSGSGYGTEFTITISSLQPEDFATYYCIQHNSYLWTFGQGTKVEIK | QVQVVESGGGVVQPGRSLRLSCAASGFTFSDYGMHWVRQAPGKGLEWVAVIWFDGSNKYYADSVKGRFTISRDNSKNTLYLQMNSLRAEDTAVYYCARDLGWTEEYWGQGTPVTVSS |
| | | | SEQ ID NO: 24858 | SEQ ID NO: 28864 |
| **iPS:393878** | **21-225_7G12** | NA | GACATCCAGATGACCCAGTCTCCATCCTCCCTGTCTGCATCTGTAGGAGACAGAGTCTCCATCACTTGCCGGGCAAGTCAGAATATTAACAACTATTTAAATTGGTATCAGCAGAAACCAGGGAAAGGCCCTAAGGTCCTGATCCTTACTGCATCCAGTTTGCAAAGTGGGGTCCCATCAAGGTTCAGTGGCAGTGGATCTGGGACAGATTTCACTCTCACCATCAACAGTCTGCAACCTGAAGATTTTGCAACTTACTACTGTCAACAGAGTTACACTACCCCCACGTGGACGTTCGGCCAAGGGACCAAGGTGGAAATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAACTGGGTCCGCCAGGCTCCAGGGAAGGGGCTGGAGTGGGTCTCAGTTATTAGTGGTAGTGGTGGTAGCACATACTACGCAGACTCCGTGAAGGGCCGGTTCACCATCTCCAGAGACAATTCCAAGAACACGCTGTATTTGCAAATGAACACCCTGAGAGCCGAGGACACGGCCGTATATTACTGTGCGAAAAGGTATACCAGTGACTGGCATGATGCTTTTGATATCTGGGGCCAAGGGACAATGGTCACCGTCTCTTCA |
| | | | SEQ ID NO: 24859 | SEQ ID NO: 28865 |
| | | AA | DIQMTQSPSSLSASVGDRVSITCRASQNINNYLNWYQQKPGKGPKVLILTASSLQSGVPSRFSGSGSGTDFTLTINSLQPEDFATYYCQQSYTTPTWTFGQGTKVEIK | EVQLLESGGGLVQPGGSLRLSCAASGFTFSSYAMNWVRQAPGKGLEWVSVISGSGGSTYYADSVKGRFTISRDNSKNTLYLQMNTLRAEDTAVYYCAKRYTSDWHDAFDIWGQGTMVTVSS |
| | | | SEQ ID NO: 24860 | SEQ ID NO: 28866 |

FIGURE 50

| | | | | |
|---|---|---|---|---|
| **iPS:393880** | **21-225_15A1** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTACAAAGTGGGGTCCCATCAAGGTTCAGCGGCAGTGGTTCTGGGACAGAATTCACTCTCACAGTCAGCAGCCTGCAGCCTGAAGATTTTGCAAGTTATTACTGTCTACACAACAGTAGTTACCCTGTTAAGTTTGGGGGAGGGATAAAGGTGGAGATCACA | CAGCTGCATCTGCAGGAGTCGGGCCCAGGACTGGTGAAGCCTTCGGAGACCCTGTCCCTCACCTGCACTGTCTCTGGTGGCTCCATCAGCAGAAGTAGTTACTACTGGGGCTGGATCCGCCAGCCCCCAGGGAAGGGGCTGGAGTGGATTGGGAGTATCTATTATAGTGGGAGCGCCCAGTACAACCCGTCCCTCAAGAGTCGAGTCACCATATCCGTAGACACGACCAAGAACCAGTTCTCCCTGACGCTGAGCTCTGTGACCGCCGCAGACACGGCTGTATATTACTGTGCGAGACTGAGCAGCAGCTGGTCTTTTGACTACTGGGGCCAGGGAACCCTGGTCACCGTCTCCTCA |
| | | | SEQ ID NO: 24861 | SEQ ID NO: 28867 |
| | | AA | DIQMTQSPSSLSASVGDRVTITCRASQGIRNDLGWYQQKPGKAPKRLIYAASSLQSGVPSRFSGSGSGTEFTLTVSSLQPEDFASYYCLHNSSYPVKFGGGIKVEIT | QLHLQESGPGLVKPSETLSLTCTVSGGSISRSSYYWGWIRQPPGKGLEWIGSIYYSGSAQYNPSLKSRVTISVDTTKNQFSLTLSSVTAADTAVYYCARLSSSWSFDYWGQGTLVTVSS |
| | | | SEQ ID NO: 24862 | SEQ ID NO: 28868 |
| **iPS:393882** | **21-225_15E3** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTCGCAAAGTGGTGTCCCATCAAGGTTCAGCGGCAGTCGATCTGGGACAGAATTCACTCTCACAATCAGCAGCCTGCAGCCTGAAGATCTTGCAGCTTATTACTGTCTACAGCATCATAGTTACCCGCTCACTTTCGGCGGAGGGACCGAGGTGGAGATCTAC | CAGGTGCAGTTGGTGGAGTCTGGGGGAACCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGAGGAAAATAATAAACACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGCTGGGGTTCCTCTCTGACTACTGGGGCCAGGGAACCCTGGTCACCGTCTCCTCA |
| | | | SEQ ID NO: 24863 | SEQ ID NO: 28869 |

FIGURE 50

| | | | | |
|---|---|---|---|---|
| | | AA | DIQMTQSPSSLSASVGDRVTITCRASQGIRNDLGWYQQKPGKAPKRLIYAASSSQSGVPSRFSGSRSGTEFTLTISSLQPEDLAAYYCLQHHSYPLTFGGGTEVEIY | QVQLVESGGTVVQPGRSLRLSCAASGFTFSSYGMHWVRQAPGKGLEWVAVIWYEENNKHYADSVKGRFTISRDNSKNTLYLQMNSLRAEDTAVYYCARELGFLSDYWGQGTLVTVSS |
| | | | SEQ ID NO: 24864 | SEQ ID NO: 28870 |
| iPS:393884 | 21-225_16F4 | NA | GACATCCAGATGATCCAGTCTCCATCCTCCCCGTCTGCATCTGTAGGAGACAGAGTCACCATCACTTGCCGGGCAAGTCAGGGCATTAGAAATGATTTAGGCTGGTATCAGCAGAAATCAGGGAAAGCCCCTAAGCGCCTGATATATGTTGCATCCAGTTTGCAAAGTGGGGTCCCATCAAGGTTCAGCGGCAGTGGATCTGGGACAGAATTCACTATCACAATCAGCAGCGTGCAGCCTGAAGATTTTGCAACTTATTACTGTATACAGCATAATAGTTATCCGTTCACTTTCGGCGGAGGGACCAAGGTGGAGATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGAAGGAAGTAATCAATACTATGGAGACTCCGTGAAGGGCCGATTCACCATCTCCAGAGACAATTCCAAGAACACGGTGTATCTGCAAATGCACAGCCTGAGAGCCGAGGACACGGCTGTGTATTACTGTGCGAGAGAGCTGGGGTTCCTCTCTGACTACTGGGGCCAGGGAACCCTGGTCACCGTCTCCTCA |
| | | | SEQ ID NO: 24865 | SEQ ID NO: 28871 |
| | | AA | DIQMIQSPSSPSASVGDRVTITCRASQGIRNDLGWYQQKSGKAPKRLIYVASSLQSGVPSRFSGSGSGTEFTITISSVQPEDFATYYCIQHNSYPFTFGGGTKVEIK | QVQLVESGGGVVQPGRSLRLSCAASGFTFSNYGMHWVRQAPGKGLEWVAVIWYEGSNQYYGDSVKGRFTISRDNSKNTVYLQMHSLRAEDTAVYYCARELGFLSDYWGQGTLVTVSS |
| | | | SEQ ID NO: 24866 | SEQ ID NO: 28872 |

FIGURE 50

| | | | | |
|---|---|---|---|---|
| **iPS:393886** | **21-225_2G9** | NA | GACATCCAGATGACCCAGTCTCCATCCTCCCTGTTTGCATCTGTCGGAGACAGAGTCACCATCACTTGCCGGGCAAGTCAGGGCATTAGAAATGATTTAGGTTGGTATCAGCAGAAACCAGGGAAAGCCCCTCAGCGCCTGATCTATGCTGCATCCAGTTTGCAAAGTGGAGTCCCATCAAGGTTCAGCGGCAGTGGATTTGGGACAGAATTCACTCTCACAATCAGCAGCCTGCAGCTTGAAGATTTTGCAACTTATTACTGTTTACAGCATGAAAGTTACCCTCTCACTTTCGGCGGAGGGACCAAGGTGGAAATCAAA | CAACTGCAGCTGCAGGAGTCGGGCCCAGGACTGGTGAAGCCTTCGGAGACCCTGTCCCTCACCTGCACTGTCTCTGGTGGCTCCATCAGACCTAATTACTACTGGGGCTGGATCCGCCAGCCCCCAGGGAAGGGGCTGGAGTGGATTGGTAGTATCTATTATAGTGGAAGCACCTCCTACAACCCGTCCCTCAACAGTCGAGTCACCATATCCGTGGACACGTCCAAGAACCAGTTCTCCCTGAAGCTGAACTCTGTGACCGCCGCAGACACGGCTGTGTATTACTGTGCGAGACTAAGCAGCAACTGGGACTTTGACAACTGGGGCCAGGGAACCCTGGTCACCGTCTCCTCA |
| | | | SEQ ID NO: 24867 | SEQ ID NO: 28873 |
| | | AA | DIQMTQSPSSLFASVGDRVTITCRASQGIRNDLGWYQQKPGKAPQRLIYAASSLQSGVPSRFSGSGFGTEFTLTISSLQLEDFATYYCLQHESYPLTFGGGTKVEIK | QLQLQESGPGLVKPSETLSLTCTVSGGSIRPNYYWGWIRQPPGKGLEWIGSIYYSGSTSYNPSLNSRVTISVDTSKNQFSLKLNSVTAADTAVYYCARLSSNWDFDNWGQGTLVTVSS |
| | | | SEQ ID NO: 24868 | SEQ ID NO: 28874 |
| **iPS:393888** | **21-225_3E3** | NA | GACATCCAGATGACCCAGTCTCCATCCTCCCCGTCTGCATCTGTAGGAGACAGAGTCACCATCACTTTCCGGGCAAGTCAGAGCATTAGAAGTTATTTAAATTGGTATCAGCAGAAACCAGGGAAAGCCCATAAACTCGTGATCTATGGTACATCCAGTTTGCAAAGTGGGGTCCCATCAAGGTTCAGTGGCAGTGGATCTGGGACAGACTTCACTCTCACCATCAGCAGTCTGCAACCTGAAGATTTTGCAACTTACTACTGTCAACAGAGTTACAGTACCCCCTTGTTCACTTTCGGCCCTGGGACCAAAGTGGATATCAAA | GAGGTGCAGCTGTTGGAGTCTGGGGGAACCTTGGTACAGCCTGGGGGGTCCCTGAGACTCTCCTGTGCAGCCTCTGAATTCACCTTTAGCAGCTATGTCATGAGCTGGGTCCGCCAGGCTCCAGGGAAGGGGCTGGAGTGGGTCTCAATTATTAGTGGTCGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCTTATATTACTGTGCGTCCCGTTTAGCAGTGGCTGGCTCGGAGGCTTTTGATATCTGGGGCCAAGGGACAATGGTCACCGTCTCTTCA |
| | | | SEQ ID NO: 24869 | SEQ ID NO: 28875 |

FIGURE 50

| | | | | |
|---|---|---|---|---|
| | | AA | DIQMTQSPSSPSASVGDRVTITFRASQSIRSYLNWYQQKPGKAHKLVIYGTSSLQSGVPSRFSGSGSGTDFTLTISSLQPEDFATYYCQQSYSTPLFTFGPGTKVDIK | EVQLLESGGTLVQPGGSLRLSCAASEFTFSSYVMSWVRQAPGKGLEWVSIISGRGGNTFYADSVKGRFTISRDNSKNTLYLQMNSLRAEDTALYYCASRLAVAGSEAFDIWGQGTMVTVSS |
| | | | SEQ ID NO: 24870 | SEQ ID NO: 28876 |
| **iPS:393890** | **21-225_4B1** | NA | GACATCCAGATGACCCAGTCTCCATCCTCCCTGTCTGCATCTGTAGGCGACAGAGTCACCATCACTTGCCGGGCAAGTCATACCATTAGAACCTATTTAAACTGGTATCAGCAGAAACCAGGGAAAGCCCCTAAGCTCCTGATCTATGCTGCATCCAGTTTGCAAAGTGGGGTCCCATCAAGGATCAATGGCAGTGGATCTGGGACAGATTTCACTCTCACCATCACCAATCTACAGCCTGAAGATTTTGCAACTTACTACTGTCAACAGAGTTACAATATCTCATTCACTTTCGGCCCTGGGACCAAAGTGGATATCAAA | CAGGTGCAGCTGCAGGAGTCGGGCCCAGGACTGGTGAAGCCTTCGGAGACCCTGTCCCTCACCTGTAGTGTCTCTGGTGACTCCATCAGTAGTTACTCCTGGAGCTGGATCCGGCAGCCCGCCGGGAAGGGACTGGAATGGATTGGGCGTATCTATACCAGTGGGAGCACCAACTACATCCCCTCCCTCAAGAGTCGAATCACCATGTCAGTAGACACGTCCAAGAAGCAGTTCTCCCTGAAGCTGAGCTCTGTGACCGCCGCGGACACGGCCGTGTATTACTGTGCGAGAGATTTGAAGAGCAGTGGCTGCCTTTTCTTTGACTACTGGGGCCAGGGAACCCTGGTCACCGTCTCCTCA |
| | | | SEQ ID NO: 24871 | SEQ ID NO: 28877 |
| | | AA | DIQMTQSPSSLSASVGDRVTITCRASHTIRTYLNWYQQKPGKAPKLLIYAASSLQSGVPSRINGSGSGTDFTLTITNLQPEDFATYYCQQSYNISFTFGPGTKVDIK | QVQLQESGPGLVKPSETLSLTCSVSGDSISSYSWSWIRQPAGKGLEWIGRIYTSGSTNYIPSLKSRITMSVDTSKKQFSLKLSSVTAADTAVYYCARDLKSSGCLFFDYWGQGTLVTVSS |
| | | | SEQ ID NO: 24872 | SEQ ID NO: 28878 |

FIGURE 50

| | | | | |
|---|---|---|---|---|
| iPS:393892 | 21-225_6G7 | NA | GACATCCAGATGACCCAGTCTCCATCCTCCCGGTCTGCATCTGTAGGAGACAGAGTCACCATCACTTGCCAGGCGAGTCAGGACATTAGCAACTATTTAAATTGGTGTCAACAGAAACCAGGGAAAGCCCTTAAGCTCTTGATATACGATGCATCCACTTTGGAAACAGGGGTCCCCTCAAGGTTCAGTGGAAGTGGATCTGGGACAGATTTTACTTTCACCATCAGCAGCGTGCAGCCTGAAGATATTGCAACATATTACTGCCAACAGTATGATAATGTCCCGATCACCTTCGGCCAAGGGACACGACTGGAGATTAAA | CAGGTGCAGCTGGTGGAGTCTGGGGGAGGCGTGGTCCAGCCTGGGAGGTCCCTGAGACTCTCCTGTGCAGCCTCTGGATTTACCTTCAGTAGCTATGGCATGCACTGGGTCCGCCAGACTCCAGGCAAGGGGCTGGAGTGGGTGGCAATTATATCATATGTTGGAAAGAATAAATATTATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTACTGTGCGAGACGGGGAAACAGCTATGGCGGGTACGGTATGGACGTCTGGGGCCAAGGGACTACGGTCACCGTCTCCTCA |
| | | | SEQ ID NO: 24873 | SEQ ID NO: 28879 |
| | | AA | DIQMTQSPSSRSASVGDRVTITCQASQDISNYLNWCQQKPGKALKLLIYDASTLETGVPSRFSGSGSGTDFTFTISSVQPEDIATYYCQQYDNVPITFGQGTRLEIK | QVQLVESGGGVVQPGRSLRLSCAASGFTFSSYGMHWVRQTPGKGLEWVAIISYVGKNKYYADSVKGRFTISRDNSKNTLYLQMNSLRAEDTAVYYCARRGNSYGGYGMDVWGQGTTVTVSS |
| | | | SEQ ID NO: 24874 | SEQ ID NO: 28880 |
| iPS:393894 | 21-225_5E11 | NA | GTCATCCAGATGACCCAGTCTCCATCCTCACTGTCTGCCTCTGTAGGAGACAGAGTCACCATCACTTGTCGGGCGAGTCAGGGCATTAGCAATTATTTAGCCTGGTTTCAGCAGAAACCAGGGAAAGCCCCTAAGTCCCTGATCAATGCTGCATCCAGTGTGCAGAGTGGGGTCCCATCAAAATTCAGCGGCAATGGATCTGGGACAGACTTCACTCTCACCATCAGCAGCCTGCAGCCTGAGGATTTTGCAACTTATTACTGCCACCAGTATCACAGTTACCCATTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTACTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTTTTACTGTGCGAGAGTGGCTTCATTTGACTACTGGGGCCAGGGAACCCTGGTCACCGTCTCCTCA |
| | | | SEQ ID NO: 24875 | SEQ ID NO: 28881 |

FIGURE 50

| | | | | |
|---|---|---|---|---|
| | | AA | VIQMTQSPSSLSASVGDRVTITCRASQGISNYLAWFQQKPGKAPKSLINAASSVQSGVPSKFSGNGSGTDFTLTISSLQPEDFATYYCHQYHSYPFTFGPGTKVDIK | EVQLVESGGGLVKPGGSLRLSCAASGFTFSSYSMNWVRQAPGKGLEWVSSISGSSTYIYYADSVKGRFTISRDNAKNSLYLQMNSLRAEDTAVFYCARVASFDYWGQGTLVTVSS |
| | | | SEQ ID NO: 24876 | SEQ ID NO: 28882 |
| iPS:393896 | 21-225_2A4 | NA | GACATCCAGATGACCCAGTCTCCATCCTCACTGTTTGCATCTGTAGGAGACAGAGTCACCATCACTTGTCGGGCGAGTCAGGGCATTAGCAATTATTTAGCCTGGTTTCAGCAGAAACCAGGGAAAGCCCCTAAGCGCCTGATCTATACTGCATCCAGTTTGCAAAGTGGGGTCCCATCAAAGTTCAGCGGCAGTGGATTTGGGACAGATTTCACTCTCACCATCAGCAGCCTGCAGCCTGAAGATTTTGCAACTTATTACTGCCAACAGTATAATAGTTACCCATTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTACTTACATATACTACGCAGACTCAGTGAAGGGCCGAATCGCCATCTCCAGAGACAACGCCAAGAACTCGCTGTATCTGCAAATGAACAGCCTGAGAGCCGAGGACACGGCTGTATATTACTGTGCGAGAGTGGCTTCATTTGACTACTGGGGCCAGGGAACCCTGGTCACCGTCTCCTCA |
| | | | SEQ ID NO: 24877 | SEQ ID NO: 28883 |
| | | AA | DIQMTQSPSSLFASVGDRVTITCRASQGISNYLAWFQQKPGKAPKRLIYTASSLQSGVPSKFSGSGFGTDFTLTISSLQPEDFATYYCQQYNSYPFTFGPGTKVDIK | EVQLVESGGGLVKPGGSLRLSCAASGFTFSSYSMNWVRQAPGKGLEWVSSISGSSTYIYYADSVKGRIAISRDNAKNSLYLQMNSLRAEDTAVYYCARVASFDYWGQGTLVTVSS |
| | | | SEQ ID NO: 24878 | SEQ ID NO: 28884 |

FIGURE 50

| | | | | |
|---|---|---|---|---|
| iPS:393898 | 21-225_5F7 | NA | GACATTCAGATGACCCAGTCTCCATCCTCCCCGTCTGCATCTGTAGGAGACAGAGTCACCATCACTTTCCGGGCAAGTCAGACCATTAGTAGTTATTTAAATTGGTATCAGCAGAAACCAGGGAAAGCCCCTAAACTCCTGATCTCTGCTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACAATACCCCCTTATTCACTTTCGGCCCTGGGACCAAAGTGGTTATCAAA | GAGGTGCAGTTGTTGGAGTCTGGGGGAGGCTTGGTACAGCCTGGGGGGTCCCTGAGACTCTCCTGTGCAGCCTCTGAATTCACCTTTAGCAGCTATGCCATGAGCTGGGTCCGCCAGGCTCCAGGGAAGGGGCTGGAGTGGGTCTCAATTATTAGTGGTCGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGTCCCGTATAGCAGTGGCTGGCTCGGAGGCTTTTGCTATCTGGGGCCAAGGGACAATGGTCACCGTCTCTTCA |
| | | | SEQ ID NO: 24879 | SEQ ID NO: 28885 |
| | | AA | DIQMTQSPSSPSASVGDRVTITFRASQTISSYLNWYQQKPGKAPKLLISAASSLQSGVPSRFSGSGSGTDFTLTISSLQPEDFATYYCQQSYNTPLFTFGPGTKVVIK | EVQLLESGGGLVQPGGSLRLSCAASEFTFSSYAMSWVRQAPGKGLEWVSIISGRGGNTFYADSVKGRFTISRDNSKNTLYLQMNSLRAEDTAVYYCASRIAVAGSEAFAIWGQGTMVTVSS |
| | | | SEQ ID NO: 24880 | SEQ ID NO: 28886 |
| iPS:393900 | 21-225_10E12 | NA | GACATCCAGATGACCCAGTCTCCATCCTCCCTGTCTGCATCTGTAGGAGACAGAGTCACCATCACTTGCCGGGCAAGTCAGAACATTTACAGTTATTTAAATTGGTATCAGGAGAAACCAGGGAGAGCCCCTAAGCTCCTGATCTATGCTACATCCAGTTTGCAAAGTGGGGTCCCATCAAGGTTCGGTGGCAGTGGATCTGGGACAGATTTCACTCTCACCATCAGCAGTCTACAACCTGAAGATTTTGCAACTTACTACTGTCAACAGAATTACAGTCCCCCTCTCACTTTCGGCGGAGGGACCAAGGTGGAGATCGAA | CAGGTGCAGCTGGTGGAGTCGGGGGGAGGCGTGGTCCAGCCTGGGAGGTCCCTGAGACTCTCCTGTGCAGCGTCTGGATTCAACTTCAGTAACTATGGCATGCACTGGGTCCGCCAGGTTCCAGGCAAGGGGCTGGAGTGGGTGGCAGTTATATGGCATGATGGAAGTAATAAATACTATGTAGACTCCGTGAAGGGCCGATTCACCATCTCCAGAGACAATTCCAAGAACACGCTGTCTCTGCAAATGAACAGCCTGAGAGCCGAGGACACGGCTGTGTATTGCTGTGCGAGAGATGAGAGGCTGGGGATTTTTGACTACTGGGGCCAGGGAACCCTGGTCACCGTCTCCTCA |
| | | | SEQ ID NO: 24881 | SEQ ID NO: 28887 |

FIGURE 50

| | | | | |
|---|---|---|---|---|
| | | AA | DIQMTQSPSSLSASVGDRVTITCRASQNIYSYLNWYQEKPGRAPKLLIYATSSLQSGVPSRFGGSGSGTDFTLTISSLQPEDFATYYCQQNYSPPLTFGGGTKVEIE | QVQLVESGGGVVQPGRSLRLSCAASGFNFSNYGMHWVRQVPGKGLEWVAVIWHDGSNKYYVDSVKGRFTISRDNSKNTLSLQMNSLRAEDTAVYCCARDERLGIFDYWGQGTLVTVSS |
| | | | SEQ ID NO: 24882 | SEQ ID NO: 28888 |
| iPS:393902 | 21-225_14E10 | NA | GACATCCAGATGACCCAGTCTCCATCCTCCCTGTCTGCATCTGTAGGAGACCGAGTCACCATCACTTGCCGGACAAGTCAGGGCATTAGAAATGATTTAGGCTGGTATCAGCACAAACCAGGGCAAGCCCCTAAGCGCCTGATCTATGCTGCATCCAGTTTACAAAGTGGGGTCCCATCAAGGTTCAGCGGCAGTGGATCTGGGACAGAATTCACTCTCACAATCAGCAGCCTGCAGCCTGAAGATTTTACAGCTTATTACTGTCTGCAGCATTATAGTTACCCTCGGACGTTCGGCCAAGGGACCAAGGTGGAAATCAAG | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTCTATCTGCAAATGAACAGCCTGAGAGCCGAGGACACGGCTGTGTATTACTGTGCGAGAGAGAAGTATAGCAGCAGCTGGTACGACTACGGTATGGACGTCTGGGGCCAAGGGACCACGGTCACCGTCTCCTCA |
| | | | SEQ ID NO: 24883 | SEQ ID NO: 28889 |
| | | AA | DIQMTQSPSSLSASVGDRVTITCRTSQGIRNDLGWYQHKPGQAPKRLIYAASSLQSGVPSRFSGSGSGTEFTLTISSLQPEDFTAYYCLQHYSYPRTFGQGTKVEIK | QVQLVESGGGVVQPGRSLRLSCAASGFTFSDYGMHWVRQAPGKGLEWVAVIWYDGSNKYYADSVKGRFTISRDNSKNTLYLQMNSLRAEDTAVYYCAREKYSSSWYDYGMDVWGQGTTVTVSS |
| | | | SEQ ID NO: 24884 | SEQ ID NO: 28890 |

FIGURE 50

| | | | | |
|---|---|---|---|---|
| **iPS:393904** | **21-225_8H11** | NA | GACATCCAGATGACCCAGTCTCCATCCTCCCTGTCTGCATTTGTAGGAGACAGAGTCACCATCACTTGCCGGGCAAGTCAGAACATTATCAGCTATTTAAATTGGTATCAACAGAAACCAGGGAAAGCCCCTAACCTCATGATATATGTTACATCCAGTTTGCACAGTGGGGTCCCATCAAGGTTCAGTGGCAGTGGATCTGGGACAGATTTCTCTCTCACCATCAGCAGTCTCCAACCTGAGGATTTTGCAACTTACTACTGTCAACAGAGTTACAGTACCCCTTTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTACCTATAACATGCACTGGGTCCGCCAGGCTCCAGGCAAGGGGCTGGAGTGGGTGGCAGTTATATGGTATGATGGAAGTGATAGATACTCTGCAGACTCCGTGAAGGGCCGATTCACCATCTCCCGAGACAATTCCAAGAACACGCTGTATCTGCAAATGAACAGCCTGAGAGCCGAGGACACGGCTGTGTATTACTGTGCGAGAGATCGGGCCTATAGCAGCTCGTCTGACTTCTGGGGCCAGGGAACCCTGGTCACCGTCTCCTCA |
| | | | SEQ ID NO: 24885 | SEQ ID NO: 28891 |
| | | AA | DIQMTQSPSSLSAFVGDRVTITCRASQNIISYLNWYQQKPGKAPNLMIYVTSSLHSGVPSRFSGSGSGTDFSLTISSLQPEDFATYYCQQSYSTPFTFGPGTKVDIK | QVQLVESGGGVVQPGRSLRLSCAASGFTFSTYNMHWVRQAPGKGLEWVAVIWYDGSDRYSADSVKGRFTISRDNSKNTLYLQMNSLRAEDTAVYYCARDRAYSSSSDFWGQGTLVTVSS |
| | | | SEQ ID NO: 24886 | SEQ ID NO: 28892 |
| **iPS:393906** | **21-225_13D3** | NA | GAAATAGTGATGACGCAGTCTCCAGCCACCCTGTCTGTGTCTCCAGGGGAAAGCGCCACCCTCTCCTGCAGGGCCAGTCAGACTGTTAGCAGCAACTTAGCCTGGTTCCAGCAGAAGCCTGGCCAGGCTCCCAGGCTCCTCATCAATGGTGCATCCACCAGGGCCACTGGTATCCCAGCCAGGTTCAGTGGCAGTGGGTCTGGGACAGAGTTCACTCTCACCATCAGCAGCCTGCAGTCTGAAGATTTTGCAGTTTATTTCTGTCAGCAGTATCATGACTGGCCTCCGACGTTCGGCCAAGGGACCAAGGTGGAAATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGTTATACCATGAACTGGGTCCGCCAGGCTCCAGGGAAGGGGCTGGACTGGGTCTCATCCATTAGTGGTAGTAGTAGTTACATATACTACGCGGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGATCGGGGCAGTGGCTGGGGCCAGGGAACCCTGGTCACCGTCTCCTCA |
| | | | SEQ ID NO: 24887 | SEQ ID NO: 28893 |

FIGURE 50

| | | | | |
|---|---|---|---|---|
| | | AA | EIVMTQSPATLSVSPGESATLSCRASQTVSSNLAWFQQKPGQAPRLLINGASTRATGIPARFSGSGSGTEFTLTISSLQSEDFAVYFCQQYHDWPPTFGQGTKVEIK | EVQLVESGGGLVKPGGSLRLSCAASGFTFSSYTMNWVRQAPGKGLDWVSSISGSSSYIYYADSVKGRFTISRDNAKNSLYLQMNSLRAEDTAVYYCARDRGSGWGQGTLVTVSS |
| | | | SEQ ID NO: 24888 | SEQ ID NO: 28894 |
| iPS:393908 | 21-225_10E9 | NA | GACATCCAGATGACCCAGTCTCCATCCTCCCTGTCTGCATCTGTAGGAGACAGAGTCACCTTCACTTGCCGGGCAAGTCAGGACATTAGAAGTGATTTAGGCTGGTATCAGCAGAAACCAGGGAAAGCCCCTACGCGCCTGATCTTTGCTGCATCCAGTTTGCAAAGTGGGGTCCCATCAAGGTTCAGCGGCAGTGGATCTGGGACAGAATTCACTCTCACAATCAACAGCCTGCAGCCTGAAGATTTTGCAACTTATTACTGTCTACAGCATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGTCATACACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAAGTATAGCAGCAGCTGGTACGACTACGGTATGGACGTCTGGGGCCAAGGGACCACGGTCACCGTCTCCTCA |
| | | | SEQ ID NO: 24889 | SEQ ID NO: 28895 |
| | | AA | DIQMTQSPSSLSASVGDRVTFTCRASQDIRSDLGWYQQKPGKAPTRLIFAASSLQSGVPSRFSGSGSGTEFTLTINSLQPEDFATYYCLQHYSYPRTFGQGTKVEIK | QVQLVESGGGVVQPGRSLRLSCAASGFTFSNYVIHWVRQAPGKGLEWVAVIWYDGSNKYYADSVKGRFTISRDNSKNTLYLQMNSLRAEDTAVYYCAREKYSSSWYDYGMDVWGQGTTVTVSS |
| | | | SEQ ID NO: 24890 | SEQ ID NO: 28896 |

FIGURE 50

| | | | | |
|---|---|---|---|---|
| **iPS:393910** | **21-225_15F10** | NA | GACATCCAGATGACCCAGTCTCCATCCTCCCTGTCTGCTTCTGTAGGAGACAGAGTCACCATCACTTGCCAGGCGAATCAGGACATTACCAACTTTTTAAATTGGTATCAGCTGAAACCAGGGAAAGCCCCTAACCTCCTGATCTCCGATGCATCCAATTTGGAAACAGGGGTCCCATCAAGGTTCAGTGGAAGTGGATCTGGGACAGATTTTACTTTCACCATCAGCAGCCTGCAGCCTGAAGATGTTGCGACATATTACTGTCAACAGTATGATAATCTCCCGATCACCTTCGGCCAAGGGACACGACTGGAGATTAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAGTGGGTGTCAGTTATATCATATGGTGGAAGTAATAATTACTATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTACTGTGTGAGACGGGGATACAGCTATGGCGGGTACGGTATGGACGTCTGGGGCCAAGGGACCACGGTCACCGTCTCCTCA |
| | | | SEQ ID NO: 24891 | SEQ ID NO: 28897 |
| | | AA | DIQMTQSPSSLSASVGDRVTITCQANQDITNFLNWYQLKPGKAPNLLISDASNLETGVPSRFSGSGSGTDFTFTISSLQPEDVATYYCQQYDNLPITFGQGTRLEIK | QVQLVESGGGVVQPGRSLRLSCAASGFTFSSYGMHWVRQAPGKGLEWVSVISYGGSNNYYADSVKGRFTISRDNSKNTLYLQMNSLRAEDTAVYYCVRRGYSYGGYGMDVWGQGTTVTVSS |
| | | | SEQ ID NO: 24892 | SEQ ID NO: 28898 |
| **iPS:393912** | **21-225_16F6** | NA | GACATCCAGATGACCCAGTCTCCATCCTCCCTGTCTGCTTCTGTAGGAGACAGAGTCACCATCACTTGCCAGGCGAATCAGGACATTACCAACTTTTTAAATTGGTATCAGCTGAAACCAGGGAAAGCCCCTAACCTCCTGATCTCCGATGCATCCAATTTGGAAACAGGGGTCCCATCAAGGTTCAGTGGAAGTGGATCTGGGACAGATTTTACTTTCACCATCAGCAGCCTGCAGCCTGAAGATGTTGCGACATATTACTGTCAACAGTATGATAATCTCCCGATCACCTTCGGCCAAGGGACACGACTGGAGATTAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAACTATGGCATGCACCGGGTCCGCCAGGCTCCAGGCAAGGGGCTGGAGTGGGTGGCAGTTATATGGTATGAAGGAAGTAATCAATACTATGGAGACTCCGTGAAGGGCCGATTCACCATCTCCAGAGACAATTCCAAGAACACGGTGTATCTGCAAATGCACAGCCTGAGAGCCGAGGACACGGCTGTGTATTACTGTGCGAGAGAGCTGGGGTTCCTCTCTGATTACTGGGGCCAGGGAACCCTGGTCACCGTCTCCTCA |
| | | | SEQ ID NO: 24893 | SEQ ID NO: 28899 |

FIGURE 50

| | | | | |
|---|---|---|---|---|
| | | AA | DIQMTQSPSSLSASVGDRVTITCQANQDITNFLNWYQLKPGKAPNLLISDASNLETGVPSRFSGSGSGTDFTFTISSLQPEDVATYYCQQYDNLPITFGQGTRLEIK | QVQLVESGGGVVQPGRSLRLSCAASGFTFSNYGMHRVRQAPGKGLEWVAVIWYEGSNQYYGDSVKGRFTISRDNSKNTVYLQMHSLRAEDTAVYYCARELGFLSDYWGQGTLVTVSS |
| | | | SEQ ID NO: 24894 | SEQ ID NO: 28900 |
| iPS:393914 | 21-225_16B8 | NA | GACATCCAGATGACCCAGTCTCCATCCTCACTGTCTGCCTCTGTAGGAGACAGAGTCACCATCACTTGTCGGGCGAGTCAGGGCATTAACAATTATTTAGCCTGGTTTCAGCAGAAACCAGGGAAAGCCCTTAAGTCCCTGATCAATGCTGCATCCAGTGTGCAGAGTGGGGTCCCATCAAAGTTCAGCGGCAGTGGATCTGGGACAGACTTCACTCTCACCATCAGCAGCCTGCAGCCTGAAGATTTTGCAACTTATTACTGCCACCAGTATCACAGTTACCCATTCACTTTCGGCCCTGGGACCAAAGTGGATATCGTA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGATCTCATCCATTAGTGGTAGTAGTACTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGTGGCTTCATTCGACTACTGGGGCCAGGGAACCCTGGTCACCGTCTCCTCA |
| | | | SEQ ID NO: 24895 | SEQ ID NO: 28901 |
| | | AA | DIQMTQSPSSLSASVGDRVTITCRASQGINNYLAWFQQKPGKALKSLINAASSVQSGVPSKFSGSGSGTDFTLTISSLQPEDFATYYCHQYHSYPFTFGPGTKVDIV | EVQLVESGGGLVKPGGSLRLSCAASGFTFSSYSMNWVRQAPGKGLEWISSISGSSTYIYYADSVKGRFTISRDNAKNSLYLQMNSLRAEDTAVYYCARVASFDYWGQGTLVTVSS |
| | | | SEQ ID NO: 24896 | SEQ ID NO: 28902 |

FIGURE 50

| | | | | |
|---|---|---|---|---|
| iPS:393916 | 21-225_2G4 | NA | GACATCCAGATGACCCAGTCTCCATCCTCCCTGTCTGCTTCTGTAGGCGACAGAGTCACCATCACTTGCCGGGCAAGTCAGGGCATTAGAAATGATTTAGGCTGGTATCAGCAGAAACCAGGGAAAGCCCCTACGCGCCTGATCTATGCTGCATCCAGTTTGCACAGTGGGGTCCCATCACGGTTCAGCGGCAGTGGATCTGGGACAGAATTCACTCTCACAATCAGCAGCCTGCAGCCTGAAGATCTTGCAACTTATTACTGTCTACAACATTATAGTTTCCCTCGGACGTTCGGCCGAGGGACCAAGGTGGAAATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAGCTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGAAGTATAGCAGCAGCTGGTACGACTACGGTATGGACGTCTGGGGCCACGGGACCACGGTCACCGTCTCCTCA |
| | | | SEQ ID NO: 24897 | SEQ ID NO: 28903 |
| | | AA | DIQMTQSPSSLSASVGDRVTITCRASQGIRNDLGWYQQKPGKAPTRLIYAASSLHSGVPSRFSGSGSGTEFTLTISSLQPEDLATYYCLQHYSFPRTFGRGTKVEIK | QVQLVESGGGVVQPGRSLRLSCAASGFTFSSYVMHWVRQAPGKGLEWVAVIWYDGSNKYYADSVKGRFTISRDNSKNTLYLQMNSLRAEDTAVYYCAREKYSSSWYDYGMDVWGHGTTVTVSS |
| | | | SEQ ID NO: 24898 | SEQ ID NO: 28904 |
| iPS:393920 | 21-225_1H12 | NA | GACATCCAGATGACCCAGTCTCCATCCTCCCTGTCTGCATCTGTTGGAGACAGAGTCACCATCACTTGCCGGGCAAGTCAGAACATTTACAGGTATTTAAATTGGTATCAGGAGAAACCAGGGAGAGCCCCTAAGCTCCTGATCTATACTGCATCCAGTTTACAAAGTGGGGTCCCATCAAGGTTCAGTGGCAGTGACTCTGGGACAGATTTCACTCTCACCATCAGCAGTCTGCAACCTGAAGATTTTGCAACTTACTACTGTCAACAGAGTTACAGTCCCCCTCTCACTTTCGGCGGAGGGACCAAGGTGGAGATCAAA | CAGGTGCAGCTGGTGGAGTCTGGGGGAGGCGTGGTCCAGTCTGGGAGGTCCCTGAGACTCTCCTGTGCAGCGTCTGGATTCAACTTCAGTAGCTATGGCATGCACTGGGTCCGCCAGGCTCCAGGCAAGGGGCTGGAGTGGGTGGCAATTATATGGCATGATGGAAGTAATAAATACTATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATGAGAGGCTGGGGATTTTTGACTACTGGGGCCAGGGAACCCTGGTCACCGTCTCCTCA |
| | | | SEQ ID NO: 24899 | SEQ ID NO: 28905 |

FIGURE 50

| | | | | |
|---|---|---|---|---|
| | | AA | DIQMTQSPSSLSASVGDRVTITCRASQNIYRYLNWYQEKPGRAPKLLIYTASSLQSGVPSRFSGSDSGTDFTLTISSLQPEDFATYYCQQSYSPPLTFGGGTKVEIK | QVQLVESGGGVVQSGRSLRLSCAASGFNFSSYGMHWVRQAPGKGLEWVAIIWHDGSNKYYVDSVKGRFTISRDNSKNTLYLQMNSLRAEDTAVYYCARDERLGIFDYWGQGTLVTVSS |
| | | | SEQ ID NO: 24900 | SEQ ID NO: 28906 |
| **iPS:393922** | **21-225_2B2** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGCAAGCCCCTAAGCGCCTGATCTATGCTGCATCCAGTTTACAAAGTGGGGTCCCATCAAGGTTCAGCGGCAGTGGATCTGGGACAGAATTCACTCTCACAATCAGCAGCCTGCAGCCTGAAGATCTTGCAACTTATCACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGAGGAAAATAATAAATACTATGTAGACTCCGTGAAGGGCCGATTCACCATCTCCAGAGACAATTCCAAGAGCACGCTGTATCTGCAAATGAACAGCCTGAGAGCCGAGGACACGGCTGTGTATTACTGTGTGAGAGAACTAGGCTTCCAGTCTGACTACTGGGGCCAGGGAACCCCGGTCACCGTCTCCTCA |
| | | | SEQ ID NO: 24901 | SEQ ID NO: 28907 |
| | | AA | DIQMTQSPSSLSASVGDRVTITCRASQGIRNDLGWYQQKPGQAPKRLIYAASSLQSGVPSRFSGSGSGTEFTLTISSLQPEDLATYHCLQHNSYPLTFGGGTKVEIK | QVQLVESGGGVVQPGRSLRLSCAASGFTFSSYGMHWVRQAPGKGLEWVAVIWYEENNKYYVDSVKGRFTISRDNSKSTLYLQMNSLRAEDTAVYYCVRELGFQSDYWGQGTPVTVSS |
| | | | SEQ ID NO: 24902 | SEQ ID NO: 28908 |

FIGURE 50

| | | | | |
|---|---|---|---|---|
| **iPS:393926** | **21-225_4G4** | NA | GACATCCAGATGACCCAGTCTCCATCCTCCCTGGCTGCATCTGTAGGAGACAGAGTCACCATCACTTGCCGGGCAAGTCAGACCATTATCAGCTATTTAAATTGGTATCAGCAGAAACCAGGGAAAGCCCCTAAACTTCTGATCCATACTGCATCCAGTTTGCAAAGTGGGGTCCCATCAAGGTTCAGTGGCAGTGGATCTGGGACAGATTTCACTCTCTCCATCAGCAGTCTGCAACCTGAAGATTTTGCAACTTACTACTGTCAACAGACTTACAGTACTCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCCGGATTCACCTTCAGTAGCTATGGCATGCACTGGGTCCGCCAGGCTCCAGGCAAGGGGCTGGAGTGGGGGCAGTTATATGGCATGATGGAAGTAATAAATACTATGCAGACTCCGTGAAGGGCCGATTCACCATCTCCAGAGACAATGCCAAGAACACGCTGTATCTGCAAATGAACAGCCTGAGAGCCGAGGACACGGCTGTGTATTACTGTGCGAGAGATCTGAGAATGGGCGGTATGGACGTCTGGGGCCAAGGGACCACGGTCACCGTCTCCTCA |
| | | | SEQ ID NO: 24903 | SEQ ID NO: 28909 |
| | | AA | DIQMTQSPSSLAASVGDRVTITCRASQTIISYLNWYQQKPGKAPKLLIHTASSLQSGVPSRFSGSGSGTDFTLSISSLQPEDFATYYCQQTYSTPLTFGGGTKVEIK | QVQLVESGGGVVQPGRSLRLSCAASGFTFSSYGMHWVRQAPGKGLEWGAVIWHDGSNKYYADSVKGRFTISRDNAKNTLYLQMNSLRAEDTAVYYCARDLRMGGMDVWGQGTTVTVSS |
| | | | SEQ ID NO: 24904 | SEQ ID NO: 28910 |
| **iPS:393928** | **21-225_4E10** | NA | GACATCCAGATGACCCAGTCTCCATCCTCCCTGTTTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATTTGGGACAGAATTCACTCTCACAATCAGCAGCCTGCAGCTTGAAGATTTTGCAACTTATTACTGTTTACAGCATGATAATTACCCTCTCACTTTCGGCGGAGGGACCAAGGTGGAGATCAAA | CAGCTGCAGCTACAGGAGTCGGGCCCAGGACTGGTGAAGCCTTCGGAGACCCTGTCCCTCACCTGCACTGTCTCTGGTGGCTCCATCAGCCGTAGTAGTTACTACTGGGGCTGGATCCGCCAGCCCCCAGGGAAGGGGCTGGAGTGGATTGGGAGTGTCTATTATAGTGGGGCCACCTCCTACAACCCGTCCCTCAAGAGTCGAGTCACCATATCCGTAGACACGTCCAAGAACCAGTTTTCCCTGAAGCTGAACTCTGTGACCGCCGCAGACACGGCTTTGTATTACTGTGTGAGACTAAGCAGCAACTGGGACTTTGACTACTGGGGCCAGGGAACCCTGTTCACCGTCTCCTCA |
| | | | SEQ ID NO: 24905 | SEQ ID NO: 28911 |

FIGURE 50

| | | | | |
|---|---|---|---|---|
| | | AA | DIQMTQSPSSLFASVGDRVTITCRASQGIRNDLGWYQQKPGKAPKRLIYAASSLQSGVPSRFSGSGFGTEFTLTISSLQLEDFATYYCLQHDNYPLTFGGGTKVEIK | QLQLQESGPGLVKPSETLSLTCTVSGGSISRSSYYWGWIRQPPGKGLEWIGSVYYSGATSYNPSLKSRVTISVDTSKNQFSLKLNSVTAADTALYYCVRLSSNWDFDYWGQGTLFTVSS |
| | | | SEQ ID NO: 24906 | SEQ ID NO: 28912 |
| **iPS:393930** | **21-225_7E11** | NA | GACATCCAGATGACCCAGTCTCCATCCTCCCTGTCTGCATCTGTAGGAGACAGAGTCACCATCGCTTGCCGGGCAAGTCAAAACATTATCAGCTATTTAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATATGGCATGATGGAAGTAATAAATACTATGCAGACTCCGTGAAGGGCCGATTCACCATCTCCAGAGACAATTCCAACAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTCTCCTCA |
| | | | SEQ ID NO: 24907 | SEQ ID NO: 28913 |
| | | AA | DIQMTQSPSSLSASVGDRVTIACRASQNIISYLNWYQQKPGKAPKFLIYTASSLQSGVPSRFSGSGSGTDFTLTISSLQPEDFAIYYCQQTYSTPLTFGGGTKVEIK | QVQLVESGGGVVQPGRSLRLSCAASGFTFSSFGMHWVRQAPGKGLEWVAIIWHDGSNKYYADSVKGRFTISRDNSNNTLYLQMSSLRAEDTAVYYCARDLSMGGMDVWGQGTTVTVSS |
| | | | SEQ ID NO: 24908 | SEQ ID NO: 28914 |

FIGURE 50

| | | | | |
|---|---|---|---|---|
| **iPS:393932** | **21-225_10F5** | NA | GACATCCAGATGACCCAGTCTCCATCCTCCCTGTCTGCATCTGTTGGAGACAGAGTCACCATCACTTGCCGGGCAAGTCAGAACATTTACAGGTATTTAAATTGGTATCAGGAGAAACCAGGGAGAGCCCCTAAACTCCTGATCTATACTGCATCCAGTTTGCAAAGTGGGGTCCCATCAAGGTTCAGTGGCAGTGACTCTGGGACAGATTTCACTCTCACCATCAGCAGTCTGCAACCTGAAGATTTTGCAACTTACTACTGTCAACAGAGTTACAGTCCCCCTCTCACTTTCGGCGGAGGGACCAAGGTGGAGATCAAA<br>SEQ ID NO: 24909 | CAGGTGCAGCTGGTGGAGTCTGGGGGAGGCGTGGTCCAGCCTGGGAGGTCCCTGAGACTCTCCTGTGCAGCGTCTGGATTCAACTTCAGTAGCTATGGCATGCACTGGGTCCGCCAGGCTCCAGGCAAGGGGCTGGAGTGGGTGTCAATTATATGGCATGATGGAAGTAATAAATATTATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATGAGAGGCTGGGGATTTTTGACTACTGGGGCCAGGGAACCCTGGTCACCGTCTCCTCG<br>SEQ ID NO: 28915 |
| | | AA | DIQMTQSPSSLSASVGDRVTITCRASQNIYRYLNWYQEKPGRAPKLLIYTASSLQSGVPSRFSGSDSGTDFTLTISSLQPEDFATYYCQQSYSPPLTFGGGTKVEIK<br>SEQ ID NO: 24910 | QVQLVESGGGVVQPGRSLRLSCAASGFNFSSYGMHWVRQAPGKGLEWVSIIWHDGSNKYYVDSVKGRFTISRDNSKNTLYLQMNSLRAEDTAVYYCARDERLGIFDYWGQGTLVTVSS<br>SEQ ID NO: 28916 |
| **iPS:393934** | **21-225_13E6** | NA | GACATCCAGGTGACCCAGTCTCCATCTTCCCTGTCTGCATCTGTAGGAGACAGAGTCACCATCACTAGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTGTACAGCATAATAGTTACCCGCTCACTTTCGGCGGAGGGACCAAGGTGGCGATCAAA<br>SEQ ID NO: 24911 | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGATGAAAATAATAAATATTATATAGACTCCGTGAAGGGCCGATTCACCATCTCCAGAGACAACTCCAAGAACACGCTGTATCTGCAAATGAACAGCCTGAGAGCCGAGGACACGGCTGTGTATTACTGTGCGAGAGAATTGGGGTTCCGGTCTGACTACTGGGGCCAGGGAACCCTGGTCACCGTCTCCTCA<br>SEQ ID NO: 28917 |

FIGURE 50

| | | | | |
|---|---|---|---|---|
| | | AA | DIQVTQSPSSLSASVGDRVTITSRASQGIRNDLGWYQQKPGKAPKRLIYAASSLQSGVPSRFSGSGSGTEFTLTISSLQPEDFATYYCVQHNSYPLTFGGGTKVAIK | QVQLVESGGGVVQPGRSLRLSCAASGFTFSNYGMHWVRQAPGKGLEWVAVIWYDENNKYYIDSVKGRFTISRDNSKNTLYLQMNSLRAEDTAVYYCARELGFRSDYWGQGTLVTVSS |
| | | | SEQ ID NO: 24912 | SEQ ID NO: 28918 |
| **iPS:393936** | **21-225_14A11** | NA | GACATCCAGATGACCCAGTCTCCATCCTCCCTGTCTGCATCTATAGGAGACAGAGTCACCATCACTTGCCGGGCAAGTCAGAGCATTAGCAGTTATTTAAATTGGTATCAGCAGAAACCAGGGAAAGCCCCTAAGCTCCTGATCTTTGCTGCATCCAGTTTGCAAAATGGGGTCCCATCAAGGTTCAGTGGCAGTGGATCTGGGACAGATTTCACTCTCACCATCAGCAGTCTGCAACCTGAAGATTTTGCAACTTACTACTGTCAACAGACTTACAGTAGCCCTCCATTCACTTTCGCCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAACTGGGTCCGCCAGGCTCCAGGGAAGGGGCTGGAGTGGGTCTCAGTTATTAGTGGTAGAGGTGGTAGTACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAAAGGATAGCAGCTGGTATGGAGTACTTCGATCTCTGGGGCCGTGGCACCCTGGTCACTGTCTCCTCA |
| | | | SEQ ID NO: 24913 | SEQ ID NO: 28919 |
| | | AA | DIQMTQSPSSLSASIGDRVTITCRASQSISSYLNWYQQKPGKAPKLLIFAASSLQNGVPSRFSGSGSGTDFTLTISSLQPEDFATYYCQQTYSSPPFTFAPGTKVDIK | EVQLLESGGGLVQPGGSLRLSCAASGFTFSSYAMNWVRQAPGKGLEWVSVISGRGGSTYYADSVKGRFTISRDNSKNTLYLQMNSLRAEDTAVYYCAKRIAAGMEYFDLWGRGTLVTVSS |
| | | | SEQ ID NO: 24914 | SEQ ID NO: 28920 |

FIGURE 50

| | | | | |
|---|---|---|---|---|
| **iPS:393940** | **21-225_16B2** | NA | GGCGTCCAGATGACCCAGTCTCCATCCTCCCTGTCTGCATCTGTAGGAGACAGAGTCACCATCACTTGCCGGGCAAGTCAGAGCATTAGCGGCTATTTAAATTGGTATCAGCAGAAACCAGGGAAAGCCCCTAAGCTCCTGATCTATGCTGCATCCAGTTTGCAAAGTGGGGTCCCATCAAGGTTCAGTGGCGGTGGATCTGGGACAGATTTCACTCTCACCATCAGCAGTCTACAACCTGAAGATTTTGCAACTTACTACTGTCAACAGACTTACAATACCCCTCCGGAGCGCAGTTTTGGCCAGGGGACCAAGCTGGAGATCAAA | GAGGTGCAGTTGTTGGAGTCTGGGGGAGGCTTGGTACAGCCGGGGGGGTCCCTGAGACTCTCCTGTGCAGCCTCTGGATTCACCTTTAGCAGCTATGCCATGACCTGGGTCCGCCAGGCTCCAGGGAAGGGGCCGGAGTGGGTCTCAGTTATTAGTGGTAGTGGTGGTAGCACATTCTACGCAGACTCCGTGAAGGGCCGGTTCACCATCTCCAGAGACAATTCCAAGAACACGCTTTATCTGCAAATGAGCAGCCTGAGAGCCGAGGACACGGCCGTTTATTTCTGTGCCCGATATTGTAGTAGTACCAGGTGCCCTTATGATGCCTTTGATATCTGGGGCCAAGGGACAATGGTCACCGTCTCCTCA |
| | | | SEQ ID NO: 24915 | SEQ ID NO: 28921 |
| | | AA | GVQMTQSPSSLSASVGDRVTITCRASQSISGYLNWYQQKPGKAPKLLIYAASSLQSGVPSRFSGGGSGTDFTLTISSLQPEDFATYYCQQTYNTPPERSFGQGTKLEIK | EVQLLESGGGLVQPGGSLRLSCAASGFTFSSYAMTWVRQAPGKGPEWVSVISGSGGSTFYADSVKGRFTISRDNSKNTLYLQMSSLRAEDTAVYFCARYCSSTRCPYDAFDIWGQGTMVTVSS |
| | | | SEQ ID NO: 24916 | SEQ ID NO: 28922 |
| **iPS:393942** | **21-225_11E5** | NA | GACATCGTGATGACCCAGTCTCCAGACTCCCTGGCTGTGTCTCTGGGCGAGAGGGCCACCCTCAACTGCAAGTCCAGCCAGAGTGTTTTATACAGCTCCAACAATAACAACTACTTAACTTGGTACCAGCAGAAACCTGGACAGCGACCTAAGTTGCTCATTTACTGGGCATCTACCCGGGAATCCGGGGTCCCTGACCGATTCAGTGGCAGCGGGTCTGGGACAAATTTCACTCTCACCATCAGCAGCCTGCAGGCTGAAGATGTGGCAGTTTATTACTGTCAGCAATATTATAGTACTCCTCCGACGTTCGGCCAAGGGACCAAGGTGGAAATCAAA | CAGGTGCAACTGGTGCAGTCTGGGCCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTGCCACCGGCTATGCACAGAGGTTCCAGGGCAGAGTCACCATGACCAGGAACACCTCCATAAGCACAGCCTACATGGAGCTGAGCAGCCTGAGATCTGAGGACACGGCCGTGTATTACTGTGCGACAAGCAGTGGCTGGGAGGTCTTTGACTACTGGGGCCAGGGAACCCTGGTCACCGTCTCCTCA |
| | | | SEQ ID NO: 24917 | SEQ ID NO: 28923 |

FIGURE 50

| | | | | |
|---|---|---|---|---|
| | | AA | DIVMTQSPDSLAVSLGERATLNCKSSQSVLYSSNNNNYLTWYQQKPGQRPKLLIYWASTRESGVPDRFSGSGSGTNFTLTISSLQAEDVAVYYCQQYYSTPPTFGQGTKVEIK | QVQLVQSGPEVKKPGASVKVSCKASGYTFTNYDINWVRQATGQGLEWMGWMHPNSGATGYAQRFQGRVTMTRNTSISTAYMELSSLRSEDTAVYYCATSSGWEVFDYWGQGTLVTVSS |
| | | | SEQ ID NO: 24918 | SEQ ID NO: 28924 |
| **iPS:393944** | **21-225_14D6** | NA | GACATCCAAATGACCCAGTCTCCATCCTCCCTGTCTGCATCTGTCGGAGACAGTGTCACCATCACTTGCCGGGCAAGTCAGGACATTAGAAATCATTTAGGCTGGTATCAGCATAAACCAGGGAAAGCCCCTAAGCGCCTGATCTATGCTGCATCCAGTTTGCAAAGTGGGGTCCCATCTAGGTTCAGCGGCAGTGGATCTGGGACAGAATTCACTCTCACAATCAGCAGCCTGCAGCCTGAAGATTTTGCAACTTATTACTGTCTACAGTATAATAGTTACCCATTCACTTTCGGCCCTGGGACCAAAGTGGATATCAAC | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGTTATACCATGAACTGGGTCCGCCAGGCTCCAGGGAAGGGGCTGGAGTGGGTCTCATCCATTAGTGGTAGTGGTAGTTACATATACTACGCAGACTCAGTGAAGGGCCGATTCACCATGTCCAGAGACAACGCCAAGAACTCACTGTATCTGCAAATGAACAGCCTGAGAGCCGAGGACACGGCTGTGTATTACTGTGCGAGAGTAGCAGCCTTTGACTCCTGGGGCCAGGGAACCCTGGTCTCCGTCTCCTCA |
| | | | SEQ ID NO: 24919 | SEQ ID NO: 28925 |
| | | AA | DIQMTQSPSSLSASVGDSVTITCRASQDIRNHLGWYQHKPGKAPKRLIYAASSLQSGVPSRFSGSGSGTEFTLTISSLQPEDFATYYCLQYNSYPFTFGPGTKVDIN | EVQLVESGGGLVKPGGSLRLSCAASGFTFSSYTMNWVRQAPGKGLEWVSSISGSGSYIYYADSVKGRFTMSRDNAKNSLYLQMNSLRAEDTAVYYCARVAAFDSWGQGTLVSVSS |
| | | | SEQ ID NO: 24920 | SEQ ID NO: 28926 |

FIGURE 50

| | | | | |
|---|---|---|---|---|
| **iPS:393946** | **21-225_16A4** | NA | GACATCCAGATGACCCAGTCTCCATCCTCACTGTCTGCATCTGTAGGAGACAGAGTCACCATCACTTGTCGGGCGAGTCAGGACATTAGTAATTATTTAGCCTGGTTTCAGCAGAAACCAGGGAAAGCCCCTAAGTCCCTGATCTCTGCTGCATCCAGTTTGCAAAGTGGGGTCCCATCAAAATTCAGCGGCAGTGGATCTGGGACAGATTTCACTCTCACCATCAGCAGCCTGCAGCCTGAAGATTTTGCAACTTATTACTGCCAACAGTATCATAGTTACCCGTGGACGTTCGGCCAAGGGACCAAGGTGGAAATCAAT | GAGGTGCAGCTGGTGGAGTCTGGGGGAGGCCTGGTCAAGCCTGGGGGGTCCCTGAGACTCTCCTGTGCAGCCTCTGGATTCACCTTCAGCAGCTATAGCATGAACTGGGTCCGCCAGGCTCCAGGGAAGGGGCTGGAGTGGGTCTCATCCATTAGTGGTAGTAGTACTTACAAATACTACGCAGACTCAGTGAAGGGCCGATTCACCATCTCCAGAGACAACGCCAAGAACTCACTGTATCTGCAAATGAACAGCCTGAGAACCGAGGACACGGCTGTGTATTACTGTGCGAGAGATCGTGGGAGCTACTGGGGCCAGGGAACCCAGGTCACCGTCTCCTCA |
| | | | SEQ ID NO: 24921 | SEQ ID NO: 28927 |
| | | AA | DIQMTQSPSSLSASVGDRVTITCRASQDISNYLAWFQQKPGKAPKSLISAASSLQSGVPSKFSGSGSGTDFTLTISSLQPEDFATYYCQQYHSYPWTFGQGTKVEIN | EVQLVESGGGLVKPGGSLRLSCAASGFTFSSYSMNWVRQAPGKGLEWVSSISGSSTYKYYADSVKGRFTISRDNAKNSLYLQMNSLRTEDTAVYYCARDRGSYWGQGTQVTVSS |
| | | | SEQ ID NO: 24922 | SEQ ID NO: 28928 |
| **iPS:393948** | **21-225_16A5** | NA | GACATCCAGATGACCCAGTCTCCTTCCTCCCTGTCTGCATCTGTAGGAGACAGAGTCACCATCACTTGCCGGGCAAGTCAGGGCATTAGAAATGATTTAGGCTGCTATCAGCAGAAACCAGGGAAAGCCCCTAAGCGCCTGATCTATGCTGCATCCAGTTTGCAAAGTGGGGTCCCATCAAGGTTCAGCGGCAATGGATATGGGACAGAATTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTAAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTTTGATGGAAGTAATAAATACTATGTAGACTCCGTGAAGGGCCGATTCACCATCTCCAGAGACAATTCCAAGAACACGCTGTATCTGCAAATGAGCAGCCTGAGAGCCGAGGACACGGCTGTGTATTACTGTGCGAGAGATCTTGGCTGGACGGAAGAGTACTGGGGCCAGGGAACCCTGGTCACCGTCTCCTCA |
| | | | SEQ ID NO: 24923 | SEQ ID NO: 28929 |

FIGURE 50

| | | | | |
|---|---|---|---|---|
| | | AA | DIQMTQSPSSLSASVGDRVTITCRASQGIRNDLGCYQQKPGKAPKRLIYAASSLQSGVPSRFSGNGYGTEFTLTISSLQPEDFATYYCLQHNSYPWTFGQGTKVEIK | QVQLVESGGGVVQPGRSLRLSCAASGFTFSDYGMHWVRQAPGKGLEWVAVIWFDGSNKYYVDSVKGRFTISRDNSKNTLYLQMSSLRAEDTAVYYCARDLGWTEEYWGQGTLVTVSS |
| | | | SEQ ID NO: 24924 | SEQ ID NO: 28930 |
| **iPS:393950** | **21-225_3H10** | NA | TCCTATGAGCTGAGTCAGCCACCCTCAGTGTCCGTGTCCCCAGGACAGACAGCCAGCATCACCTGCTCTGGAGATAAATTGGGGGAAAAATATGCTTGCTGGTATCAGCAGAAGCCAGGCCAGTCCCCTGTGCTGGTCATCTATCAAGATAGGAAGCGGCCCTCAGGGATCCCTGAGCGATTCTCTGGCTCCAACTCTGGGAACAAAGCCACTCTGACCATCAGCGGGACCCAGGCTATGGATGAGGCTGACTATTACTGTCAGGCGTGGGTCAACAACACTATGATATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGAGATATAGCAGTGGCTGGTATGACTACGGTTTGGACGTCTGGGGCCAAGGGACCACGGTCACCGTCTCCTCA |
| | | | SEQ ID NO: 24925 | SEQ ID NO: 28931 |
| | | AA | SYELSQPPSVSVSPGQTASITCSGDKLGEKYACWYQQKPGQSPVLVIYQDRKRPSGIPERFSGSNSGNKATLTISGTQAMDEADYYCQAWVNNTMIFGGGTKLTVL | QVQLVESGGGVVQPGRSLRLSCAASGFTFSSYVMHWVRQAPGKGLEWVAVIWYDGSNKYYADSVKGRFTISRDNSKNTLYLQMNSLRAEDTAVYYCARERYSSGWYDYGLDVWGQGTTVTVSS |
| | | | SEQ ID NO: 24926 | SEQ ID NO: 28932 |

FIGURE 50

| | | | | |
|---|---|---|---|---|
| **iPS:393952** | **21-225_1F1** | NA | GACATCCAGATGACCCAGTCTCCATCCTCACTGTCTGCATCTGTAGGAGACAGAGTCACCATCACTTGTCGGGCGAGTCAGGGCATTAACAATTATTTAGCCTGGTTTCAGCAGAAATCAGGGAAAGCCCCTAAGTCCCTGATCTCTGTTGCATCCAGTTTGCAAACTGGGGTCCCATCAAGGTTCAGCGGCAGTGGATCTGGGACAGATTTCACTCTCACCATCAGCAGCCTGCAGCCTGAAGATTTTGCAACTTATTACTGCCAACAGTATAATAGTTACCCTCTCACTTTCGGCGGAGGGACCAAGGTGGAGATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAACATGAACTGGGTCCGCCAGGCTCCAGGGAAGGGGCTGGAGTGGGTCTCATCCATTAGTAGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGTTAACCTCTTTGACTACTGGGGCCAGGGAACCCTGGTCACCGTCTCCTCA |
| | | | SEQ ID NO: 24927 | SEQ ID NO: 28933 |
| | | AA | DIQMTQSPSSLSASVGDRVTITCRASQGINNYLAWFQQKSGKAPKSLISVASSLQTGVPSRFSGSGSGTDFTLTISSLQPEDFATYYCQQYNSYPLTFGGGTKVEIK | EVQLVESGGGLVKPGGSLRLSCAASGFTFSSYNMNWVRQAPGKGLEWVSSISSSSSYIYYADSVKGRFTISRDNAKNSLYLQMNSLRAEDTAVYYCARVNLFDYWGQGTLVTVSS |
| | | | SEQ ID NO: 24928 | SEQ ID NO: 28934 |
| **iPS:393954** | **21-225_4H6** | NA | GACATCCAGATGACCCAGTCTCCATCTTCCGTGTCTGCATCTGTAGGAGACAGAGTCACCATCACTTGTCGGGCGAGTCAGGGTATTAGCAGGTGGTTAGCCTGGTATCAGCAGAAACCAGGGAAAGCCCCTAAGCTCCTGATCTATGGTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAATTTATTATTGTCAACAGGCTAACAGTTTCCCATTCACTTTCGGCCCTGGGACCAAAGTGGATATCAAA | CAGGTGCAGCTGGTACAGTCTGGGGCTGAGGTGAAGAAGCCTGGGGCCTCAGTGAAGGTCTCCTGCAAGGCTTCTGGATACACCTTCACCGACTACTATTTGCACTGGGTGCGACAGGCCCCTGGACAAGGTCTTGAGTGGATGGGATGGATCCACCCTAACAGTGGTGGCACAAACTATGCACAGAAGTTTCAGGGCAGGGTCACCATGACCAGGGACACGTCCATCAGCACAGCCTACATGGGGCTGAGCAGTCTGAGATCTGACGACACGGCCGTGTATTACTGTGCGAGAGATGGTACCAGCTCGTTTGACTACTGGGGCCAGGGAACCCTGGTCACCGTCTCCTCA |
| | | | SEQ ID NO: 24929 | SEQ ID NO: 28935 |

FIGURE 50

| | | | | |
|---|---|---|---|---|
| | | AA | DIQMTQSPSSVSASVGDRVTITCRASQGISRWLAWYQQKPGKAPKLLIYGASSLQSGVPSRFSGSGSGTDFTLTISSLQPEDFAIYYCQQANSFPFTFGPGTKVDIK | QVQLVQSGAEVKKPGASVKVSCKASGYTFTDYYLHWVRQAPGQGLEWMGWIHPNSGGTNYAQKFQGRVTMTRDTSISTAYMGLSSLRSDDTAVYYCARDGTSSFDYWGQGTLVTVSS |
| | | | SEQ ID NO: 24930 | SEQ ID NO: 28936 |
| iPS:393956 | 21-225_4D7 | NA | GACATCCAGATGACCCAGTCTCCATCCTCCCTGTCTGCATCTGTAGGAGACAGAGTCACCATCACTTGCCGGGCAAGTCAGAGCATTAGCGACTATTTAAATTGGTATCAGCAGAAACCAGGGAAAGCCCCTAAACTCCTGATCTTTGATACAACCAGTTTGCAAAGTGGGGTCCCATCAAGGTTCAGTGGCAGTGGATCTGGGACAGATTTCACTCTCACCATCAACAGTCTGCAACCTGAAGATTTTGCAACTTACTACTGTCAACAGACTTACAATACCCCTCCGGAGCGCAGTTTTGGCCAGGGGACCAAGCTGGAGATCAAA | GAGGTGAAGCTGTTGGAGTCTGGGGGAGGCTTGGTACAGCCGGGGGGGTCCCTGAGACTCTCCTGTGCAGCCTCTGGATTCACCTTCAGCAGCTATGCCATGAGCTGGGTCCGCCAGGCTCCAGGGAAGGGGCTGGAGTGGGTCTCAGTTCTTAGTGGTAGTGGTGGTAGCACATTCTACGCAGACTCCGTGAAGGGCCGGTTCACCATCTCCAGAGACAATTCCAAGAACACGCTGTATCTGCAAATGAGCAGCCTGAGAGCCGGGGACACGGCCGTATATTTCTGTGCCCGATATTGTAGTAGTGCCAGGTGCCCTTATGATGCCTTTGATATCTGGGGCCAAGGGACAATGGTCACCGTCTCCTCA |
| | | | SEQ ID NO: 24931 | SEQ ID NO: 28937 |
| | | AA | DIQMTQSPSSLSASVGDRVTITCRASQSISDYLNWYQQKPGKAPKLLIFDTTSLQSGVPSRFSGSGSGTDFTLTINSLQPEDFATYYCQQTYNTPPERSFGQGTKLEIK | EVKLLESGGGLVQPGGSLRLSCAASGFTFSSYAMSWVRQAPGKGLEWVSVLSGSGGSTFYADSVKGRFTISRDNSKNTLYLQMSSLRAGDTAVYFCARYCSSARCPYDAFDIWGQGTMVTVSS |
| | | | SEQ ID NO: 24932 | SEQ ID NO: 28938 |

FIGURE 50

| | | | | |
|---|---|---|---|---|
| **iPS:393958** | **21-225_5H2** | NA | GACATCCAGATGACCCAGTCTCCATCCTCCCTGTCTGCATCTGTAACAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCAGTCTCACAATCAGCAGCCTGCAGCCTGAAGATTTTGCAACTTATTACTGTCTACAGCATAATAGTTACCCTCTCACTTTCGGCGGAGGGACCAAGGTGGAGATCAAA | GAGGTGCAGCTGGTGGAGTCTGGGGGAGGCCTGGTCAAGCCTGGGGGGTCCTTGAGACTCTCCTGTGCAGCCTCTGGATTCACATTCAGTAGCTATACCATGAACTGGGTCCGCCAGGCTCCAGGGAAGGGGCTGGAGTGGGTCTCATCCATTAGTGGTAGTAGTAGTTACATATACTACGCAGACTCAGTGAAGGGCCGATTCACCATCTCCAGAGCCAACGCCAAGAACTCATTGTATCTGCAAATGAACAGCCTGAGAGCCGAGGACACGGCTGTGTATTACTGTGCGAGAGGGAGCAGCTCGTCCGGCTTTGACTACTGGGGCCAGGGAACCCTGGTCACCGTCTCCTCA |
| | | | SEQ ID NO: 24933 | SEQ ID NO: 28939 |
| | | AA | DIQMTQSPSSLSASVTDRVTITCRASQGIRNDLGWYQQKPGKAPKRLIYAASSLQSGVPSRFSGSGSGTEFSLTISSLQPEDFATYYCLQHNSYPLTFGGGTKVEIK | EVQLVESGGGLVKPGGSLRLSCAASGFTFSSYTMNWVRQAPGKGLEWVSSISGSSSYIYYADSVKGRFTISRANAKNSLYLQMNSLRAEDTAVYYCARGSSSSGFDYWGQGTLVTVSS |
| | | | SEQ ID NO: 24934 | SEQ ID NO: 28940 |
| **iPS:393960** | **21-225_7G2** | NA | GACATCCAGATGACCCAGTCTCCATCCTCCCTGTCTGCGTCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATACTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAACATAATAGTTACCCGTGGACGTTCGGCCTAGGGACCAAGGTGGTCATCAAA | CAGGTGCAACTGGTGGAGTCTGGGGGAGGCGTGGTCCAGCCTGGGAGGTCCCTGAGACTCTCCTGTGCAGCGTCAGGATTCACCTTCAGTGACTATGGCATGCACTGGGTCCGCCAGGCTCCAGGCAAGGGGCTGGAGTGGATGGCAGTTATATGGTATGATGTAACTAATAAATACTATGCAGACTCCGTGAAGGGCCGATTCACCATCTCCAGAGACAATTCCAAGAACACGCTGTATCTGCAACTGAACAGCCTGAGAGCCGAGGACACGGCTGTTTATTACTGTGCGAGAGAACTTGGCTGGTACGAGGACTACTGGGGCCAGGGAACCCTGGTCACCGTCTCCTCA |
| | | | SEQ ID NO: 24935 | SEQ ID NO: 28941 |

FIGURE 50

| | | | | |
|---|---|---|---|---|
| | | AA | DIQMTQSPSSLSASVGDRVTITCRASQGIRNDLG WYQQKPGKAPKRLIYTASSLQSGVPSRFSGSGSG TEFTLTISSLQPEDFATYYCLQHNSYPWTFGLGT KVVIK | QVQLVESGGGVVQPGRSLRLSCAASGFTFSDYGM HWVRQAPGKGLEWMAVIWYDVTNKYYADSVKG RFTISRDNSKNTLYLQLNSLRAEDTAVYYCARELG WYEDYWGQGTLVTVSS |
| | | | SEQ ID NO: 24936 | SEQ ID NO: 28942 |
| iPS:393962 | 21-225_7H7 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCACATCAAGGTTCAGCGG CAGTGGATCTGGGACAGAATTCACTCTCACAA TCAGCAGCCTGCAGCCTGAAGATTTTGCAACT TATTACTGTCTACAGCATAGTAGTTACCCGTTC ACTTTCGTCGGAGGGACCAAAGTGGAGATCAA A | CAGCTGCAGCTGCAGGAGTCGGGCCCAGGACTG GTGAAGCCTTCGGAGACCCTGTCCCTCACCTGCA CTGTCTCTGGTGGCTCCATCAGCAGAAGTAGTTA CTACTGGGGCTGGATCCGCCAGCCCCCAGGGAA GGGGCTGGAGTGGATTGGGAATATCTATTATAGT GGGAGCACCTACTACATCCCGTCCCTCAAGAGTC GAGTCACCATATCCGTTGACACGTCCAAGAACCA GTTCTCCCTGAAACTGAGCTCTGTGACCGCCGCA GACACGGCTGTGTATTACTGTGCGAGACACAGTA CCAGCTGGTCTCTTGACCACTGGGGCCAGGGAAC CCTGGTCACCGTCTCCTCA |
| | | | SEQ ID NO: 24937 | SEQ ID NO: 28943 |
| | | AA | DIQMTQSPSSLSASVGDRVTITCRASQGIRNDLG WYQQKPGKAPKRLIYAASSLQSGVTSRFSGSGS GTEFTLTISSLQPEDFATYYCLQHSSYPFTFVGGT KVEIK | QLQLQESGPGLVKPSETLSLTCTVSGGSISRSSYYW GWIRQPPGKGLEWIGNIYYSGSTYYIPSLKSRVTISV DTSKNQFSLKLSSVTAADTAVYYCARHSTSWSLDH WGQGTLVTVSS |
| | | | SEQ ID NO: 24938 | SEQ ID NO: 28944 |

FIGURE 50

| | | | | |
|---|---|---|---|---|
| **iPS:393964** | **21-225_6G1** | NA | GACATCCAGATGACCCAGTCTCCATCCTCCCTGTCTGCATCTGTAGGAGACAGAGTCACCATCACTTGCCGGACAAGTCAGAACATTATCAGCTATTTAAATTGGTATCAGCAGAAACCAGGGAAAGCCCCTAAGGTCCTGATCTATACTGCATCCAATTTGCAAACTGGGGTCCCATCAGGGTTCAGTGGCAGTGGATCTGGGACAGATTTCACTCTCACCATCAGCAGTCTGCAACCTGAAGATTTTGCAACTTACTACTGTCAACAGCCTCACAGTCCCCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCATCTTCAGTAGCTATGGCATGCACTGGGTCCGCCAGGCTCCAGGCAAGGGGCTGGAGTGGGTGGCAATTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTATGTATTACTGTGCGAGAGATCTGAGTATGGGCGGTATGGACGTCTGGGGCCAAGGGACCACGGTCACCGTCTCCTCA |
| | | | SEQ ID NO: 24939 | SEQ ID NO: 28945 |
| | | AA | DIQMTQSPSSLSASVGDRVTITCRTSQNIISYLNWYQQKPGKAPKVLIYTASNLQTGVPSGFSGSGSGTDFTLTISSLQPEDFATYYCQQPHSPPLTFGGGTKVEIK | QVQLVESGGGVVQPGRSLRLSCAASGFIFSSYGMHWVRQAPGKGLEWVAIIWYDGSNKYYADSVKGRFTISRDNSKNTLYLQMNSLRAEDTAMYYCARDLSMGGMDVWGQGTTVTVSS |
| | | | SEQ ID NO: 24940 | SEQ ID NO: 28946 |
| **iPS:393966** | **21-225_7F8** | NA | GACATCCAGATGACCCAGTCTCCATCCTCCCTGTCTGCATCTGTGGGAGACAGAGTCACCATCACTTGCCGGGCAAGTCAGGGCATTGGAAATGATTTAGGCTGGTATCAGCAGAAACCAGGGAAAGCCCCTAAGCGCCTGATCTATGCTGCATCCAGTTTGCAAAGTGGGGTCCCATCACGGTTCAGCGGCAGTGTATCTGGGACAGAATTCACTCTCCCAATCAGCAGCCTGCAGCCTGAAGATTTTGCAACTTATTACTGTCTACAACATTATACTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTGTGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAATAACACGCTGTATCTGCAAATGAACAGCCTGAGAGCCGAGGACACGGCTGTGTATTACTGTGCGAGAGAAAGGTATACCAGTGGCTGGCACGACTACGGTATGGACGTCTGGGGCCAAGGGACCACGGTCACCGTCTCCTCG |
| | | | SEQ ID NO: 24941 | SEQ ID NO: 28947 |

FIGURE 50

| | | | | |
|---|---|---|---|---|
| | | AA | DIQMTQSPSSLSASVGDRVTITCRASQGIGNDLG WYQQKPGKAPKRLIYAASSLQSGVPSRFSGSVS GTEFTLPISSLQPEDFATYYCLQHYTYPRTFGQG TKVEIK | QVQLVESGGGVVQPGRSLRLSCAASGFTFSSCVMH WVRQAPGKGLEWVAVIWYDGSNKYYADSVKGRF TISRDNSNNTLYLQMNSLRAEDTAVYYCARERYTS GWHDYGMDVWGQGTTVTVSS |
| | | | SEQ ID NO: 24942 | SEQ ID NO: 28948 |
| iPS:393968 | 21-225_5A5 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTCTGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAATAGTTACCCATTCA CTTTCGGCCCTGGGACCAAAGTGGATATCAAA | GAGGTGCAGCTGTGGGAGTCTGGGGGAGGCTTG GTACAGCCTGGGGGGTCCCTGAGACTCTCCTGTG CAGCCTCCGGATTCACCTTTAGCAGCTATGCCAT GAACTGGGTCCGCCAGGCTCCAGGGAAGGGGCT GGAGTGGGTCTCAGCTATTAGTGGTAGTGGTGGT TACACATACTACGCAGACTCCGTGAAGGGCCGGT TCACCATCTCCCGAGACAATTCCAAGAACACGCT GTATCTGCAAATGAGCAGCCTGAGAGCCGAGGA CACGGCCGTTTATTACTGTGCGAAAGGGGGGTCC CTCTTCTACTGGGGCCAGGGAACCTTGGTCACCG TCTCTTCA |
| | | | SEQ ID NO: 24943 | SEQ ID NO: 28949 |
| | | AA | DIQMTQSPSSLSASVGDRVTITCRASQGISNYLA WFQQKPGKAPKSLISAASSLQSGVPSKFSGSGSG TDFTLTISSLQPEDFATYYCQQYNSYPFTFGPGT KVDIK | EVQLWESGGGLVQPGGSLRLSCAASGFTFSSYAMN WVRQAPGKGLEWVSAISGSGGYTYYADSVKGRFT ISRDNSKNTLYLQMSSLRAEDTAVYYCAKGGSLFY WGQGTLVTVSS |
| | | | SEQ ID NO: 24944 | SEQ ID NO: 28950 |

FIGURE 50

| | | | | |
|---|---|---|---|---|
| **iPS:393972** | **21-225_7C9** | NA | GACATCCAGATGACCCAGTCTCCATCCTCCCTGTCTGCATCTGGAGGAGACAGAGTCACCATCACTTGCCGGGCAAGTCAGGGCATTAGAAACGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCTTTATAGTTACCCTCGGACGTTCGGCCAGGGGACCAAGGTGGATATCAAA | CAGGTGCAGCTGGTGGAGTCTGGGGGAGGCGTGGTCCAGCCTGGGAGGTCCCTGAGACTCTCCTGTGCAGCGTCTGGACTCACCTTCAGTAACTATGTCATGCACTGGGTCCGCCAGGCTCCAGGCAAGGGGCTGGAGTGGGTGGCTGTTATATGGTATGATGGAAGTAATAAATACTATGCAGACTCCGTGAAGGGCCGATTCACCATCTCCAGAGACAATTCCAAGAACACGTTGTATCTGCAAATGAACAGCCTGAGAGCCGAGGACACGGCTGTGTATTACTGTGCGAGAGAGAAGTATAGCAGCAGCTGGTACGACTACGGTATGGACGTCTGGGGCCAAGGGACCACGGTCACCGTCTCCTCA |
| | | | SEQ ID NO: 24945 | SEQ ID NO: 28951 |
| | | AA | DIQMTQSPSSLSASGGDRVTITCRASQGIRNDLGWYQQKPGKAPKRLIYAASSLQSGVPSRFSGSGSGTEFTLTISSLQPEDFATYYCLQLYSYPRTFGQGTKVDIK | QVQLVESGGGVVQPGRSLRLSCAASGLTFSNYVMHWVRQAPGKGLEWVAVIWYDGSNKYYADSVKGRFTISRDNSKNTLYLQMNSLRAEDTAVYYCAREKYSSSWYDYGMDVWGQGTTVTVSS |
| | | | SEQ ID NO: 24946 | SEQ ID NO: 28952 |
| **iPS:393974** | **21-225_7C4** | NA | GACATCCAGATGACCCAGTCTCCATCCTCCCTGTTTGCATCTGTAGGAGACAGAGTCACCATCACTTGCCGGGCAAGTCAGGGCATTAGAAATGATTTAGGCTGGTATCAGCAGAAACCAGGGAAAGCCCATAAGCGCCTTATATATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAAGTCCCTGAGACTCTCCTGTGCAGCGTCTGGATTCACCTTCAGTAACTATGGCATGCACTGGGTCCGCCAGGCTCCAGGCAAGGGGCTGGAATGGGTGGCAGTTATTTGGTATGAAGAAAATAATCAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGTTCCGGTCTGACTACTGGGGCCAGGGAACCCTGGTCACCGTCTCCTCA |
| | | | SEQ ID NO: 24947 | SEQ ID NO: 28953 |

FIGURE 50

| | | | | |
|---|---|---|---|---|
| | | AA | DIQMTQSPSSLFASVGDRVTITCRASQGIRNDLGWYQQKPGKAHKRLIYAASSLQSGVPSRFSGSGSGTEFTLTISSLQPEDFATYYCLQHNSYPLTFGGGTKVEIK | QVQLVESGGGVVQPGKSLRLSCAASGFTFSNYGMHWVRQAPGKGLEWVAVIWYEENNQYYADSVKGRFTISRDNSKNTLYLQMNSLRAEDTAVYYCARELGFRSDYWGQGTLVTVSS |
| | | | SEQ ID NO: 24948 | SEQ ID NO: 28954 |
| **iPS:393976** | **21-225_7E9** | NA | GACATCCAGATGACCCAGTCTCCATCCTCCCTGTTTGCATCTGTAGGAGACAGAGTCACCATCACTTGCCGGGCAAGTCAGGGCATTAGAAATGATTTAGGCTGGTATGAGCAGAAACCAGGGAAAGCCCCTAAGCGCCTGATCTATACTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGAAAATAATAAATACTATGTAGACTCCGTGAAGGGCCGATTCACCATCTCCAGAGACAACTCCAAGAACACGCTGTATCTGCAAATGAACAGCCTGAGAGCCGAGGACACGGCTGTATATTATTGTGCGAGAGAATTGGGGTTCCGGTCTGACTACTGGGGCCAGGGAACCCTGGTCACCGTCTCCTCA |
| | | | SEQ ID NO: 24949 | SEQ ID NO: 28955 |
| | | AA | DIQMTQSPSSLFASVGDRVTITCRASQGIRNDLGWYEQKPGKAPKRLIYTASSLQSGVPSRFSGSGSGTEFTLTISSLQPEDFATYYCLQHNSYPLTFGGGTKVEIK | QVQLVESGGGVVQPGRSLRLSCAASGFTFSDYGMHWVRQAPGKGLEWVAVIWYDENNKYYVDSVKGRFTISRDNSKNTLYLQMNSLRAEDTAVYYCARELGFRSDYWGQGTLVTVSS |
| | | | SEQ ID NO: 24950 | SEQ ID NO: 28956 |

FIGURE 50

| | | | | |
|---|---|---|---|---|
| **iPS:393978** | **21-225_4C12** | NA | GACATCCAGATGACCCAGTCTCCATCCTCCCTGTCTGCTTCTGTAGGCGACAGAGTCACCATCACTTGCCGGGCAAGTCAGGGCATTAGAAATGATTTAGGCTGGTATCAGCAGAAACCAGGGAAAGCCCCTACGCGCCTGATCTATGCTGCATCCAGTTTGCACAGTGGGGTCCCATCACGGTTCAGCGGCAGTGGATCTGGGACAGAATTCACTCTCACAATCAGCAGCCTGCAGCCTGAAGATCTTGCAACTTATTACTGTCTACAACATTATAGTTTCCCTCGGACGTTCGGCCAAGGGACCAAGGTGGAAATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAGCTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGAAGTATAGCAGCAGCTGGTACGACTACGGTATGGACGTCTGGGGCCACGGGACCACGGTCACCGTCTCCTCA |
| | | | SEQ ID NO: 24951 | SEQ ID NO: 28957 |
| | | AA | DIQMTQSPSSLSASVGDRVTITCRASQGIRNDLGWYQQKPGKAPTRLIYAASSLHSGVPSRFSGSGSGTEFTLTISSLQPEDLATYYCLQHYSFPRTFGQGTKVEIK | QVQLVESGGGVVQPGRSLRLSCAASGFTFSSYVMHWVRQAPGKGLEWVAVIWYDGSNKYYADSVKGRFTISRDNSKNTLYLQMNSLRAEDTAVYYCAREKYSSSWYDYGMDVWGHGTTVTVSS |
| | | | SEQ ID NO: 24952 | SEQ ID NO: 28958 |
| **iPS:393980** | **21-225_6D3** | NA | GACATCCAGATGACCCAGTCTCCATCCTCCCTGTCTGCATCTGTAGGAGACAGAGTCACCATCACTTGCCGGACAAGTCAGAGCATTAGTACTTATTTAAATTGGTATCAGCAGAAACCAGGGAAAGCCCCTAAGCTCCTGATCTTTGCTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTTCTGTCAACAGAGTTACAGAACCCCCTTTTTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGAATTCACCTTTAGCAGCTATGCCATGAACTGGGTCCGCCAGGCTCCAGGGAAGGGGCTGGAGTGGGTCTCAGTTATTAGTGGTCGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTTCTGTGCGTCCCGTTTGGCAGTGGCTGGCTCGGAGGCTTTTGATATCTGGGGCCAAGGGACAATGGTCACCGTCTCTTCA |
| | | | SEQ ID NO: 24953 | SEQ ID NO: 28959 |

FIGURE 50

| | | | | |
|---|---|---|---|---|
| | | AA | DIQMTQSPSSLSASVGDRVTITCRTSQSISTYLNWYQQKPGKAPKLLIFAASSLQSGVPSRFSGSGSGTDFTLTISSLQPEDFATYFCQQSYRTPFFTFGPGTKVDIK | EVQLLESGGGLVQPGGSLRLSCAASEFTFSSYAMNWVRQAPGKGLEWVSVISGRGGNTFYADSVKGRFTISRDNSKNTLYLQMNSLRAEDTAVYFCASRLAVAGSEAFDIWGQGTMVTVSS |
| | | | SEQ ID NO: 24954 | SEQ ID NO: 28960 |
| iPS:393982 | 21-225_6C12 | NA | GACATCCAGATGACCCAGTCTCCATCCTCCCTGTCTGCATCTGTAGGAGACAGAGGCACCATCACTTGCCGGGCAAGTCAGGGCATTAGAAGTAATTTAGGCTGGTATCAGCAGAAACCAGGGAAAGCCCCTAAGCGCCTGATCTATGCTGCATCCAGTTTGGAAAGTGGGGTCCCATCAAGGTTCAGCGGCAGTGGATCTGGGACAGAATTCACTCTCACAATCAGCAGCCTGCAGCCTGAAGATTTTGCAACTTATTTCTGTCTACAGGATAATAGTTATCCGTTCACTTTCGGCGGAGGGACCAAGGTGGAGATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTAGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGATCGTGGGAGCCTCTGGGGCCAGGGAACCCTGGTCACCGTCTCCTCA |
| | | | SEQ ID NO: 24955 | SEQ ID NO: 28961 |
| | | AA | DIQMTQSPSSLSASVGDRGTITCRASQGIRSNLGWYQQKPGKAPKRLIYAASSLESGVPSRFSGSGSGTEFTLTISSLQPEDFATYFCLQDNSYPFTFGGGTKVEIK | EVQLVESGGGLVKPGGSLRLSCAASGFTFSSYSMNWVRQAPGKGLEWVSSISSSSSYIYYADSVKGRFTISRDNAKNSLYLQMNSLRAEDTAVYYCARDRGSLWGQGTLVTVSS |
| | | | SEQ ID NO: 24956 | SEQ ID NO: 28962 |

FIGURE 50

| | | | | |
|---|---|---|---|---|
| **iPS:393984** | **21-225_4F12** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCGGTGGATCTGGGACAATATTCACTCTCACAATCAGCAGCCTGCAGCCTGAAGATTTTGCAACTTATTACTGTCTACAGCATAATAGTTACGCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGTTACTAATAAAAAGTATGCAGACTCCGTGAAGGGCCGATTCACCATCTCCAGAGACAATTCCAAGAACACGCTGTATCTGCAAATGAACAGCCTGACACCAGAGAACACGGGTGGATATGAGAACCAGAGAGAAAAGGGGGGTCTATTTGACTACTGGGGCCAGGGAACCCTGGTCACCGTCTCCTCA |
| | | | SEQ ID NO: 24957 | SEQ ID NO: 28963 |
| | | AA | DIQMTQSPSSLSASVGDRVTITCRASQGIRNDLGWYQQKPGKAPKRLIYAASSLQSGVPSRFSGGGSGTIFTLTISSLQPEDFATYYCLQHNSYALTFGGGTKVEIK | QVQLVESGGGVVQPGRSLRLSCAASGFTFSSYGMHWVRQAPGKGLEWVAVIWYDVTNKKYADSVKGRFTISRDNSKNTLYLQMNSLTPENTGGYENQREKGGLFDYWGQGTLVTVSS |
| | | | SEQ ID NO: 24958 | SEQ ID NO: 28964 |
| **iPS:393986** | **21-225_7G4** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAGACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTCTGCAACTTATTACTGTCTACATCAATATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAATAACTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGACCAGCCTGAGAGCCGAGGACACGGCTGTGTATTACTGTGCGAGAGAGAAGTATAGCAGCAACTGGTACGACTACGGTATGGACGTCTGGGGCCAAGGGACCACGGTCACCGTCTCCTCA |
| | | | SEQ ID NO: 24959 | SEQ ID NO: 28965 |

FIGURE 50

| | | | | |
|---|---|---|---|---|
| | | AA | DIQMTQSPSSLSASVGDRVTITCRASQGIRNDLGWYQQRPGKAPKRLIYAASSLQSGVPSRFSGSGSGTEFTLTISSLQPEDSATYYCLHQYSYPRTFGQGTKVEIK | QVQLVESGGGVVQPGRSLRLSCAASGFTFNNYVMHWVRQAPGKGLEWVAVIWYDGSNKYYADSVKGRFTISRDNSKNTLYLQMTSLRAEDTAVYYCAREKYSSNWYDYGMDVWGQGTTVTVSS |
| | | | SEQ ID NO: 24960 | SEQ ID NO: 28966 |
| **iPS:393988** | **21-225_7F10** | NA | GACATCCAGATGACCCAGTCGCCATCCGCACTGTCTGCATCTGTAGGAGACAGAGTCACCATCACTTGTCGGGCGAGTCAGGACATTAGGAATTATTTAGCCTGGTTTCAGCAGAAACCAGGGAAAGCCCCTAAGTCCCTGATCTCTGTTGCATCCAGTTTGCAAAGTGGGGTCCCATCAAGGTTCAGCGGGAGTGGATCTGGGACAGATTTCACTCTCACCATCAGCAGCCTGCAGCCTGAAGATTTTGCAACTTATTATTGCCAACAGTATAATAGTTACCCTCTCACTTTCGGCGGAGGGACCAAGGTGGAGATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTAGTAGTAGTAGTTACATATACTACGCAGACTCAGTGAAGGGCCGATTCACCATCTCCAGAGACAACGCCAAGAACTCACTGTATCTGCAAATGAACAGCCTGAGAACCGAGGACACGGCTGTGTATTACTGTGCGAGAGCTAACCTCTTTGACTACTGGGGCCAGGGAACCCTGGTCACCGTCTCCTCA |
| | | | SEQ ID NO: 24961 | SEQ ID NO: 28967 |
| | | AA | DIQMTQSPSALSASVGDRVTITCRASQDIRNYLAWFQQKPGKAPKSLISVASSLQSGVPSRFSGSGSGTDFTLTISSLQPEDFATYYCQQYNSYPLTFGGGTKVEIK | EVQLVESGGGLVKPGGSLRLSCAASGFTFSSYSMNWVRQAPGKGLEWVSSISSSSSYIYYADSVKGRFTISRDNAKNSLYLQMNSLRTEDTAVYYCARANLFDYWGQGTLVTVSS |
| | | | SEQ ID NO: 24962 | SEQ ID NO: 28968 |

FIGURE 50

| | | | | |
|---|---|---|---|---|
| **iPS:393990** | **21-225_11G7** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAGTAATTACCCTCTCACTTTCGGCGGAGGGACCATGGTGGAGATCAGA | CAGCTGCAGCTGCAGGAGTCGGGCCCAGGACTGGTGAAGCCTTCGGAGACCCTGTCCCTCACCTGCACTGTCTCTGGTGGCTTCATCAGCAGGAGTACTTACTACTGGGGCTGGATCCGCCAGCCCCCAGGGAAGGGGCTGGAGTGGATTGGGAGTATCTATTATAGTGGGAGCACCTCCTACAGCCCGTCCCTCAAGAGTCGAGTCACCATATCCGTAGACACGTCCAAAAACCAGTTCTCCCTGAAGTTGAGCTCTGTGACCGCCGCAGACACGGCTGTGTATTACTGTGCGAGACTGAACAGCAGCTGGTCTTTTGACTACTGGGGCCAGGGAACCCTGGTCACCGTCTCCTCA |
| | | | SEQ ID NO: 24963 | SEQ ID NO: 28969 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHSNYPLTFGGGTMVEIR | QLQLQESGPGLVKPSETLSLTCTVSGGFISRSTYYWGWIRQPPGKGLEWIGSIYYSGSTSYSPSLKSRVTISVDTSKNQFSLKLSSVTAADTAVYYCARLNSSWSFDYWGQGTLVTVSS |
| | | | SEQ ID NO: 24964 | SEQ ID NO: 28970 |
| **iPS:393992** | **21-225_14H8** | NA | GACATCCAGATGACCCAGTCTCCATCCTCACTGTCTGCATCTGTAGGAGACAGAGTCACCATCACTTGTCGGGCGAGTCAGGGCATTAGCTATTATTTAGCCTGGTTTCAGCAGAAACCAGGGAAAGCCCCTAAGTCCCTGATCTATGTTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATAATAGTTACCCATTCACTTTCGGCCCTGGGACCAAAGTGGATATCAAT | GAGGTGCAGCTGGTGGAGTCTGGGGGAGGCTTGGTACAGCCTGGGGGGTCCCTGAGACTCTCCTGTGCAGCCTCTGGATTCACCTTCAGTAGCAATAGCATGAACTGGGTCCGCCAGGCTCCAGGGAAGGGGCTGGAGTGGGTTTCATACATTAGTAGTAGTAGTAGTACCATATACTACGCAGACTCTGTGAAGGGCCGATTCACCATCGCCAGAGACAATGCCAAGAACTCACTGTATCTGCAAATGAACAGCCTGAGAGACGAGGACACGGCTGTGTATTACTGTGCGAGAGGAGGTGGGAGCCCTTTTGACTACTGGGGCCAGGGAACCCTGGTCACCGTCTCCTCA |
| | | | SEQ ID NO: 24965 | SEQ ID NO: 28971 |

FIGURE 50

| | | | | |
|---|---|---|---|---|
| | | AA | DIQMTQSPSSLSASVGDRVTITCRASQGISYYLAWFQQKPGKAPKSLIYVASSLQSGVPSKFSGSGSGTDFTLTISSLQPEDFATYYCQQYNSYPFTFGPGTKVDIN | EVQLVESGGGLVQPGGSLRLSCAASGFTFSSNSMNWVRQAPGKGLEWVSYISSSSSTIYYADSVKGRFTIARDNAKNSLYLQMNSLRDEDTAVYYCARGGGSPFDYWGQGTLVTVSS |
| | | | SEQ ID NO: 24966 | SEQ ID NO: 28972 |
| iPS:393994 | 21-225_8C9 | NA | GACATCCAGATGACCCAGTCTCCATCCTCCCTGTCTGCATCTATAGGAGACAGAGTCACCATCACTTGCCGGGCAAGTCAGGCCATTAGAAATGATTTAGACTGGTATCAGCAGAAACCAGGGAAAGCCCCTAAGCGCCTTATCTATGCTGCATCCAGTTTGCAAAGTGGGGTCCCATCAAGGTTCAGCGGCAGTGGATCTGGGACAGAATTCACTCTCACAATCAGCAGCCTGCAGACTGAAGATTTTGCAACTTATTACTGTCTACAGCATAATAGTTATCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGAAAATAATAAATACTATGTAGACTCCGTGAAGGGCCGATTCACCATCTCCAGAGACAACTCCAAGAACACGCTGTATCTGCAAATGAACAGCCTGAGAGCCGAGGACACGGCTGTGTATTACTGTGCGAGAGAATTGGGGTTCCGGTCTGACTACTGGGGCCAGGGAACCCTGGTCACCGTCTCCTCA |
| | | | SEQ ID NO: 24967 | SEQ ID NO: 28973 |
| | | AA | DIQMTQSPSSLSASIGDRVTITCRASQAIRNDLDWYQQKPGKAPKRLIYAASSLQSGVPSRFSGSGSGTEFTLTISSLQTEDFATYYCLQHNSYPLTFGGGTKVEIK | QVQLVESGGGVVQPGRSLRLSCAASGFTFSSYGMHWVRQAPGKGLEWVAVIWYDENNKYYVDSVKGRFTISRDNSKNTLYLQMNSLRAEDTAVYYCARELGFRSDYWGQGTLVTVSS |
| | | | SEQ ID NO: 24968 | SEQ ID NO: 28974 |

FIGURE 50

| | | | | |
|---|---|---|---|---|
| **iPS:393996** | **21-225_15C11** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAGCCAGGGAAAGTCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACTGCATTATAGTTACCCTCGGACGTTCGGCCGAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAAGACACGGCTGTGTATTACTGTGCGAGAGAGAAGTATAGCAGCAGCTGGTACGACTACGGTTTGGACGTCTGGGGCCAAGGGACCACGGTCACCGTCTCCTCA |
| | | | SEQ ID NO: 24969 | SEQ ID NO: 28975 |
| | | AA | DIQMTQSPSSLSASVGDRVTITCRASQGIRNDLGWYQQKPGKVPKRLIYAASSLQSGVPSRFSGSGSGTEFTLTISSLQPEDFATYYCLLHYSYPRTFGRGTKVEIK | QVQLVESGGGVVQPGRSLRLSCAASGFTFSDYVMHWVRQAPGKGLEWVAVIWYDGSNKYYADSVKGRFTISRDNSKNTLYLQMNSLRAEDTAVYYCAREKYSSSWYDYGLDVWGQGTTVTVSS |
| | | | SEQ ID NO: 24970 | SEQ ID NO: 28976 |
| **iPS:393998** | **21-225_12B12** | NA | GACATCCAGATGACCCAGTCTCCATCCTCCCTGTCTGCGTCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATACTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAACATAATAGTTACCCGTGGACGTTCGGCCTAGGGACCAAGGTGGTCATCAAA | CAGGTGCAACTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGATGGCAGTTATATGGTATGATGTAACTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTTTATTACTGTGCGAGAGAGCTTGGCTGGTACGAGGACTACTGGGGCCAGGGAACCCTGGTCACCGTCTCCTCA |
| | | | SEQ ID NO: 24971 | SEQ ID NO: 28977 |

FIGURE 50

| | | | | |
|---|---|---|---|---|
| | | AA | DIQMTQSPSSLSASVGDRVTITCRASQGIRNDLG WYQQKPGKAPKRLIYTASSLQSGVPSRFSGSGSG TEFTLTISSLQPEDFATYYCLQHNSYPWTFGLGT KVVIK | QVQLVESGGGVVQPGRSLRLSCAASGFTFSDYGM HWVRQAPGKGLEWMAVIWYDVTNKYYADSVKG RFTISRDNSKNTLYLQMNSLRAEDTAVYYCARELG WYEDYWGQGTLVTVSS |
| | | | SEQ ID NO: 24972 | SEQ ID NO: 28978 |
| **iPS:394000** | **21-225_11A2** | NA | GACATCCAGATGACCCAGTCTCCATCCTCCCT GTCTGCATCTGTAGGAGACAGAGTCACCATCA CTTGCCAGGCGAGTCAGGACATTAGCAACTAT TTAAATTGGTATCAGCAGAAACCAGGGAAAG CCCCTAAGCTCCTGATCTACGATGCATCCAAT TTGGAAACAGGGGTCCCCTCAAGATTCAGTGG AAGTGGATCTGGGACAGATTTTACTTTCACCA TCAGCAGCCTGCAACCTGAGGATGTTGCAACA TATTACTGTCAACAGTATGATAATCTCCCGAT CACCTTCGGCCAAGGGACACGACTGGACATTA AA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAGTTATATCATATGGTGGAAGT AATAAAGACTCTGCAGACTCCGTGAAGGGCCGA TTCATCATCTCCAGAGACAATTCCAAGAACACGC TGTATCTGCAAATGAACAGCCTGAGAGCTGAGG ACACGGCTGTGTATTACTGTGTGAGACGGGGATA CAGCTATGGCGGGTACGGTATGGACGTCTGGGG CCAAGGGACCACGGTCACCGTCTCCTCA |
| | | | SEQ ID NO: 24973 | SEQ ID NO: 28979 |
| | | AA | DIQMTQSPSSLSASVGDRVTITCQASQDISNYLN WYQQKPGKAPKLLIYDASNLETGVPSRFSGSGS GTDFTFTISSLQPEDVATYYCQQYDNLPITFGQG TRLDIK | QVQLVESGGGVVQPGRSLRLSCAASGFTFSSYGMH WVRQAPGKGLEWVAVISYGGSNKDSADSVKGRFII SRDNSKNTLYLQMNSLRAEDTAVYYCVRRGYSYG GYGMDVWGQGTTVTVSS |
| | | | SEQ ID NO: 24974 | SEQ ID NO: 28980 |

FIGURE 50

| | | | | |
|---|---|---|---|---|
| **iPS:394002** | **21-225_15G7** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGATCCCATCAAGGTTCAGCGGCAGTGGATTTGGGACAGAATTCACTCTCACAATCAGCAGCCTGCAGCCTGAAGATTTTGCAACTTATTACTGTCTACAGCATAGTAATTACCCTCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGCAGGAGTAGTTCCTACTGGGGCTGGATCCGCCAGCCCCCAGGGAAGGGACTGGAGTGGATTGGGAGTATCTATTATAGTGGGTACACCTATTACACCCCGTCCCTCAAGAGTCGAGTCACCATATCCGTGGACACGTCCAAGAACCAGTTCTCCCTGAGGCTGAGCTCTGTGACCGCCGCAGACACGGCTTCGTATTACTGTGCGAGACTGAGCAGCAGTTGGTCTTTTGACTTCTGGGGCCAGGGAACCCTGGTCACCGTCTCCTCA |
| | | | SEQ ID NO: 24975 | SEQ ID NO: 28981 |
| | | AA | DIQMTQSPSSLSASVGDRVTITCRASQGIRNDLGWYQQKPGKAPKRLIYAASSLQSGIPSRFSGSGFGTEFTLTISSLQPEDFATYYCLQHSNYPLTFGGGTKVEIK | QLQLQESGPGLVKPSETLSLTCTVSGGSISRSSSYWGWIRQPPGKGLEWIGSIYYSGYTYYTPSLKSRVTISVDTSKNQFSLRLSSVTAADTASYYCARLSSSWSFDFWGQGTLVTVSS |
| | | | SEQ ID NO: 24976 | SEQ ID NO: 28982 |
| **iPS:394004** | **21-225_13A1** | NA | GACATCCAGATGACCCAGTCTCCATCCTCCCTGTCTGCATCTGTAGGAGACAGAGTCACCATCACTTGCCAGGCGAGTCAGGACATTAACAACTATTTAAATTGGTATCAGCAGAAACTAGGGACAGCCCCTAAGCTCCTGATCTACGATGGATCCAATTTGGAAACAGGGGTCCCATCAAGGTTCAGTGGAAGTGGATCTGGGACAGATTTTACTTTCACCATCAGCAGCCTGCAGCCTGAAGATATTGCAACATATTACTGTCAACAGTATGAAAATCTCCCGATCACTTTCGGCCAAGGGACACGACTGGAGATTAAA | CAGGTGCAGTTGGTGGAGTCTGGGGGAGGCGTGGTCCAGCCTGGGAGGTCCCTGAGACTCTCCTGTGCAGCCTCTGGATTCACCTTCAGCAGCTATGGCATGCACTGGGTCCGCCAGGCTCCAGGCAAGGGGCTGGAGTGGGTGGCAGTTATATCATATGCGGAACTAATCAATACTATGCAGACTCCGTGAAGGGCCGATTCACCATTTCCAGAGACAATTCCAAGAACACGCTCTATCTGCAAATGAACAGCCTGAGAGCTGAGGACACGGCTGTTTATTATTGTGTGAGACGGGGATACAGCTATGGCGGGTACGGTATGGACGTCTGGGGCCAAGGGACCACGGTCACCGTCTCCTCA |
| | | | SEQ ID NO: 24977 | SEQ ID NO: 28983 |

FIGURE 50

| | | | | |
|---|---|---|---|---|
| | | AA | DIQMTQSPSSLSASVGDRVTITCQASQDINNYLNWYQQKLGTAPKLLIYDGSNLETGVPSRFSGSGSGTDFTFTISSLQPEDIATYYCQQYENLPITFGQGTRLEIK | QVQLVESGGGVVQPGRSLRLSCAASGFTFSSYGMHWVRQAPGKGLEWVAVISYAGTNQYYADSVKGRFTISRDNSKNTLYLQMNSLRAEDTAVYYCVRRGYSYGGYGMDVWGQGTTVTVSS |
| | | | SEQ ID NO: 24978 | SEQ ID NO: 28984 |
| iPS:394006 | 21-225_15C2 | NA | GACATCCAGATGACCCAGTCTCCATCCTCCCTGTCTGCATCTGTAGGAGACAGAGTCACCATCACTTGCCAGGCGAGTCAGGACATTACCAACTATTTAAATTGGTATCAGCAGAAACCAGGGAAAGCCCCTAACCTCCTGATCTACGATGCATCCAATTTGGAAACAGGGGTCCCATCAAGGTTCAGTGGAAGTGGATCTGGGACAGATTTTACTTTCACCATCAGCAGCCTGCAGCCTGAAGATATTGCGACATATTACTGTCAACAGTATGATAATCTCCCGATCACCTTCGCCCAAGGGACACGACTGGAGATTAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAGTGGGTGTCAATAATATCATATGGTGGACGTAATAATCACTATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTACTGTGTGAGACGGGGATACAGCTATGGCGGGTACGGTATGGACGTCTGGGGCCAAGGGACCACGGTCACCGTCTCCTCA |
| | | | SEQ ID NO: 24979 | SEQ ID NO: 28985 |
| | | AA | DIQMTQSPSSLSASVGDRVTITCQASQDITNYLNWYQQKPGKAPNLLIYDASNLETGVPSRFSGSGSGTDFTFTISSLQPEDIATYYCQQYDNLPITFAQGTRLEIK | QVQLVESGGGVVQPGRSLRLSCAASGFTFSSYGMHWVRQAPGKGLEWVSIISYGGRNNHYADSVKGRFTISRDNSKNTLYLQMNSLRAEDTAVYYCVRRGYSYGGYGMDVWGQGTTVTVSS |
| | | | SEQ ID NO: 24980 | SEQ ID NO: 28986 |

FIGURE 50

| | | | | |
|---|---|---|---|---|
| **iPS:394008** | **21-225_15H8** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTTTGCTGCATCCAGTTTGCAAAGTGGCGTCCCATCACGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAGA | GAGGTGCAGCTGGTGGAGTCTGGGGGAGGCCTGGTCAAGCCTGGGGGGTCCCTGAGACTCTCCTGTGCAGCCTCTGGATTCACCTTCAGCAGCTATGTCATGAACTGGGTCCGCCAGGCTCCAGGGAAGGGGCTGGAGTGGGTCTCATCCATTAGTGGTAGTAGTACTTACATATACTGCGCAGACTCAATCAAGGGCCGATTCACCATCTCCCGAGACAACGCCAAGAACTCTCTGTATCTGCAAATGAACAGCCTGAGAGCCGATGACACGGCTGTGTATTACTGTGCGAGAGATCGAGGCTCCATCTGGGGCCAAGGGACAATGGTCACCGTCTCTTCA |
| | | | SEQ ID NO: 24981 | SEQ ID NO: 28987 |
| | | AA | DIQMTQSPSSLSASVGDRVTITCRASQGIRNDLGWYQQKPGKAPKRLIFAASSLQSGVPSRFSGSGSGTEFTLTISSLQPEDFATYYCLQHNSYPLTFGGGTKVEIR | EVQLVESGGGLVKPGGSLRLSCAASGFTFSSYVMNWVRQAPGKGLEWVSSISGSSTYIYCADSIKGRFTISRDNAKNSLYLQMNSLRADDTAVYYCARDRGSIWGQGTMVTVSS |
| | | | SEQ ID NO: 24982 | SEQ ID NO: 28988 |
| **iPS:394010** | **21-225_12G5** | NA | GACATCCAGATGACCCAGTCTCCATCCTCCCTGTCTGCCTCTGTAGGAGACAGAGTCACCATCCCTTGCCGGGCGAGTCAGGACATTAGCAATTATTTAGCCTGGTATCAGCAGAAACCAGGGAAAGTTCCTAAGCTCCTGATCTATGCTGCATACATTTTGCAATCAGGGGTCCCATCTCGGTTCAGTGGCAGTGGATCTGGGACAGATTTCACTCTCACCATCAGCAGCCTGCAGCCTGAAGATGTTGCAGCTTATTACTGTCAAAAGTATGACAGTGCCCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAACTATGGCATCTACTGGGTCCGCCAGGCTCCAGGCAAGGGGCTGGAGTGGGTGGCAGTTATATCATATGATGGAAGTAATAAATACTATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTACTGTGCGAGAGATCGGGGAGCAGTGGCTGCTTACTACTACTACGGTATAGACGTCTGGGGCCAAGGGACCACGGTCACCGTCTCCTCA |
| | | | SEQ ID NO: 24983 | SEQ ID NO: 28989 |

FIGURE 50

| | | | | |
|---|---|---|---|---|
| | | AA | DIQMTQSPSSLSASVGDRVTIPCRASQDISNYLAWYQQKPGKVPKLLIYAAYILQSGVPSRFSGSGSGTDFTLTISSLQPEDVAAYYCQKYDSAPFTFGPGTKVDIK | QVQLVESGGGVVQPGRSLRLSCAASGFTFSNYGIYWVRQAPGKGLEWVAVISYDGSNKYYADSVKGRFTISRDNSKNTLYLQMNSLRAEDTAVYYCARDRGAVAAYYYYGIDVWGQGTTVTVSS |
| | | | SEQ ID NO: 24984 | SEQ ID NO: 28990 |
| **iPS:394012** | **21-225_15A3** | NA | GACATCCAGATGACCCAGTCTCCATCCTCCCTGTCTGCATCTGTAGGAGACAGAGTCACCATCACTTGCCGGGCAAGTCAGAGCATTATCAGCTATTTAAATTGGTATCTGCAGAAACCAGGGAAAGCCCCTAAGTTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGACTTACAGTACCCCGCTCACTTTCGGCGGAGGGACCAAGGTGGAGATCAAG | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATTCACTGGGTCCGCCAGGCTCCAGGCAAGGGGCTGGAGTGGGTGGCAGTTATATGGCATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCTGAGTATGGGCGGTATGGACGTCTGGGGCCAAGGGACCACGGTCACCGTCTCCTCA |
| | | | SEQ ID NO: 24985 | SEQ ID NO: 28991 |
| | | AA | DIQMTQSPSSLSASVGDRVTITCRASQSIISYLNWYLQKPGKAPKFLIYTASSLQSGVPSRFSGSGSGTDFTLTISSLQPEDFATYYCQQTYSTPLTFGGGTKVEIK | QVQLVESGGGVVQPGRSLRLSCAASGFTFSSYGIHWVRQAPGKGLEWVAVIWHDGSNKYYADSVKGRFTISRDNSKNTLYLQMNSLRAEDTAVYYCARDLSMGGMDVWGQGTTVTVSS |
| | | | SEQ ID NO: 24986 | SEQ ID NO: 28992 |

FIGURE 50

| | | | | |
|---|---|---|---|---|
| **iPS:394014** | **21-225_8G6** | NA | GACATCCAGATGACCCAGTCTCCATCCTCCCTGTCTGCATCTGTAGGAGACAGAGTCACCATCACTTGCCGGGCAAGTCAGAGCATTAGTAGTTATTTAAATTGGTATCAGCAGAAACCAGGGAAAGCCCCCAAGCTCCTGATCTTTGCTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTTCTGTCAACAGAGTTACAGAACCCCCTTTTTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGTAGCCTCTGAATTCACCTTTAGCAGCTATGTCATGAACTGGGTCCGCCAGGCTCCAGGGAAGGGGCTGGAGTGGGTCTCAGTTATTAGTGGTCGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGTCCCGTTTGGCAGTGGCTGGCTCGGAGGCTTTTGATATCTGGGGCCAAGGGACAATGGTCAGCGTCTCTTCA |
| | | | SEQ ID NO: 24987 | SEQ ID NO: 28993 |
| | | AA | DIQMTQSPSSLSASVGDRVTITCRASQSISSYLNWYQQKPGKAPKLLIFAASSLQSGVPSRFSGSGSGTDFTLTISSLQPEDFATYFCQQSYRTPFFTFGPGTKVDIK | EVQLLESGGGLVQPGGSLRLSCVASEFTFSSYVMNWVRQAPGKGLEWVSVISGRGGNTFYADSVKGRFTISRDNSKNTLYLQMNSLRAEDTAVYYCASRLAVAGSEAFDIWGQGTMVSVSS |
| | | | SEQ ID NO: 24988 | SEQ ID NO: 28994 |
| **iPS:394016** | **21-225_13D4** | NA | GACCTCCAGATGACCCAGTCTCCATCCTCCCTGTCTGCATCTGTAGGAGACCGAGTCACCATCACTTGCCGGGCAAGTCAGAGCATTTTCAGCTACTTAAATTGGTATCAGCAGAAACCAGGGAAAGCCCCTAAGCTCCTGATCTGTACTGCATCCAGTTTGCAAAATGGGGTCCCATCAAGGTTCAGTGGCAGTGGATCTGGGACAGATTTCACTCTCACCATCAGCAGTCTACAGCCTGAGGATTTTGCAACTTACTACTGTCAACAGACTTACAGTCTTCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGCATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCTGAGTATGGGCGGTATGGACGTCTGGGGCCAAGGGACCACGGTCACCGTCTCCTCA |
| | | | SEQ ID NO: 24989 | SEQ ID NO: 28995 |

FIGURE 50

| | | | | |
|---|---|---|---|---|
| | | AA | DLQMTQSPSSLSASVGDRVTITCRASQSIFSYLNWYQQKPGKAPKLLICTASSLQNGVPSRFSGSGSGTDFTLTISSLQPEDFATYYCQQTYSLPLTFGGGTKVEIK | QVQLVESGGGVVQPGRSLRLSCAASGFTFSSYGMHWVRQAPGKGLEWVAVIWHDGSNKYYADSVKGRFTISRDNSKNTLYLQMNSLRAEDTAVYYCARDLSMGGMDVWGQGTTVTVSS |
| | | | SEQ ID NO: 24990 | SEQ ID NO: 28996 |
| iPS:394018 | 21-225_15B1 | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAACCAGGGAAAGCCCCTAAGTCCCTAATCTCTGCTGCATCCAGTTTGCAAAGTGGGGTCCCATCAAACTTCAGCGGCAGTGGATCTGGGACAGATTTCACTCTCACCATCAGCAGCCTGCAGCCTGAAGATTTTGCAACTTATTACTGCCAACAGTATCATAGTTACCCATTCACTTTCGGCCCTGGGACCAAAGTGGATATCAAA | GAGGTGCAGCTGTTGGAGTCTGGGGGAGGCTTGGTACAGCCGGGGGGGTCCCTGAGACTCTCCTGTGCAACCTCAGGATTCACCTTTAGCAGCTATGTCATGAGCTGGGTCCGCCAGGCTCCAGGGAAGGGGCTGGAGTGGGTCTCAGGTATTAGTGGTAGTGGTGGTAGCACAAACAACGCAGACTCCGTGAAGGGCCGGTTCACCATCTCCAGAGACAATTCCAAGAACACGCTGTATCTGCAAGTGAACAGCCTGAGAGCCGAGGACACGGCCGTATATTACTGTGCCCGCAGCTCCTTGTTTGACTACTGGGGCCAGGGAACCCTGGTCACCGTCTCCTCA |
| | | | SEQ ID NO: 24991 | SEQ ID NO: 28997 |
| | | AA | DIQMTQSPSSLSASVGDRVTITCRASQGISNYLAWFQQKPGKAPKSLISAASSLQSGVPSNFSGSGSGTDFTLTISSLQPEDFATYYCQQYHSYPFTFGPGTKVDIK | EVQLLESGGGLVQPGGSLRLSCATSGFTFSSYVMSWVRQAPGKGLEWVSGISGSGGSTNNADSVKGRFTISRDNSKNTLYLQVNSLRAEDTAVYYCARSSLFDYWGQGTLVTVSS |
| | | | SEQ ID NO: 24992 | SEQ ID NO: 28998 |

FIGURE 50

| | | | | |
|---|---|---|---|---|
| **iPS:394020** | **21-225_15H10** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAAGTTATTACTGTCTACAGCATAATAGTTACCCGCTCACTTTCGGCGGAGGGACCAAGGTGGAGATCAAT | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGATGAAAGTAATAAATACTATGAAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGTGAGGGAAGTGGGGTTTCTTTCTGACTACTGGGGCCAGGGAATCCTGGTCACCGTCTCCTCA |
| | | | SEQ ID NO: 24993 | SEQ ID NO: 28999 |
| | | AA | DIQMTQSPSSLSASVGDRVTITCRASQGIRNDLGWYQQKPGKAPKRLIYAASSLQSGVPSRFSGSGSGTEFTLTISSLQPEDFASYYCLQHNSYPLTFGGGTKVEIN | QVQLVESGGGVVQPGRSLRLSCAASGFTFSNYGMHWVRQAPGKGLEWVAVIWYDESNKYYEDSVKGRFTISRDNSKNTLYLQMNSLRAEDTAVYYCVREVGFLSDYWGQGILVTVSS |
| | | | SEQ ID NO: 24994 | SEQ ID NO: 29000 |
| **iPS:394022** | **21-225_16H6** | NA | GACATCCAGATGACCCAGTCTCCATCCTCCCTGTCTGCATCTGTAGGAGACAGAGTCACCATCACTTGCCGGGCAAGTCAGAACATTAGCAGCTATTTAAATTGGTATCAGCAGAAACCAGGGAAAGCCCCTAAGCTCCTGATCTATGCTGCATCCAGTTTGCAAAGTGGGGTCCCATCAAGGTTCAGTGGCAGAGGATCTGGGACAGATTTCACTCTCACCATCAGTAGTCTGCAACCTGAAGATTTTGCAACTTACTACTGTCAACAGAGTTACAGAACCCCCTTATTCACTTTCGGCCCTGGGACCAAGGTTGATTTCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAACTGGGTCCGCCAGGCTCCAGGGATGGGGCTGGAGTGGGTCTCAGTTATTAGTCGTAGTGGTGGTTACACATACTACGCGGACTCCGTGAAGGGCCGGTTCACCATCTCCAGAGACAATTCCAAGAACACGCTGTATCTGCAAATGAACAGCCTGAGAGCCGAGGACACGGCCGTCTATTACTGTGCGTCCCGTTTAGCAGTGGCTGGCTCGGAGGCTTTTGATATCTGGGGCCAAGGGACAATGGTCACCGTCTCTTCA |
| | | | SEQ ID NO: 24995 | SEQ ID NO: 29001 |

FIGURE 50

| | | | | |
|---|---|---|---|---|
| | | AA | DIQMTQSPSSLSASVGDRVTITCRASQNISSYLNWYQQKPGKAPKLLIYAASSLQSGVPSRFSGRGSGTDFTLTISSLQPEDFATYYCQQSYRTPLFTFGPGTKVDFK | EVQLLESGGGLVQPGGSLRLSCAASGFTFSSYAMNWVRQAPGMGLEWVSVISRSGGYTYYADSVKGRFTISRDNSKNTLYLQMNSLRAEDTAVYYCASRLAVAGSEAFDIWGQGTMVTVSS |
| | | | SEQ ID NO: 24996 | SEQ ID NO: 29002 |
| iPS:394024 | 21-225_16B7 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGTTCAAT | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGAAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGGAACTGGGGTTTCTCTCTGACTACTGGGGCCAGGGAACCCTGGTCACCGTCTCCTCA |
| | | | SEQ ID NO: 24997 | SEQ ID NO: 29003 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVEFN | QVQLVESGGGVVQPGRSLRLSCAASGFTFSSYGMHWVRQAPGKGLEWVAVIWYDESNKYYADSVKGRFTISRDNSKNTLYLQMNSLRAEDTAVYYCARELGFLSDYWGQGTLVTVSS |
| | | | SEQ ID NO: 24998 | SEQ ID NO: 29004 |

FIGURE 50

| | | | | |
|---|---|---|---|---|
| **iPS:394026** | **21-225_16C7** | NA | GACATCCAGATGACCCAGTCTCCATCCTCACT GTCTGCTTCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTCTGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAATAGTTACCCATTCA CTTTCGGCCCTGGGACCAAAGTGGATATCAAA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGTCAT GACCTGGGTCCGCCAGGCTCCAGGGAAGGGGCT GGAGTGGGTCTCAACTATTAGTGGTAGTGGTGGT TGGACATATTATGCAGACTCCGTGAAGGGCCGGT TCACCATTTCCAGAGACAATTCCAAGAACACGCT GTATCTGCAAATGAACAGCCTGAGAGCCGAGGA CACGGCCGAATATTACTGTGCCCGCAGCTCCTTG TTTGACTATTGGGGCCAGGGAACCCTGGTCACCG TCTCCTCA |
| | | | SEQ ID NO: 24999 | SEQ ID NO: 29005 |
| | | AA | DIQMTQSPSSLSASVGDRVTITCRASQGISNYLA WFQQKPGKAPKSLISAASSLQSGVPSKFSGSGSG TDFTLTISSLQPEDFATYYCQQYNSYPFTFGPGT KVDIK | EVQLLESGGGLVQPGGSLRLSCAASGFTFSSYVMT WVRQAPGKGLEWVSTISGSGGWTYYADSVKGRFT ISRDNSKNTLYLQMNSLRAEDTAEYYCARSSLFDY WGQGTLVTVSS |
| | | | SEQ ID NO: 25000 | SEQ ID NO: 29006 |
| **iPS:394029** | **21-225_1B12** | NA | GACATCCAGATGACCCAGTCTCCATCCTCCCT GTCTGCATCTGTAGGAGACAGAGTCACCATCA CTTGCCAGGCGAGTCAGGACATTAACAACTAT TTAAATTGGTATCAACAGAAACCAGGGAAAG CCCCTAAGCTCCTGATATACGATGCATCCAAT TTGGAAACAGGGGTCCCATCAAGGTTCAGTGG AAGTGGATCTGGGACAGATTTTACTTTCACCA TCAGCAGTCTGCAGCCTGAAGATATTACAACA TATTACTGTCAACAGTATGAAAATCTCCCGAT CACCTTCGGCCAAGGGACACGACTGGAGATTA AA | CAGGTGCAGCTGGTAGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAATTATATCATATGCTGGAAGT AATAAATCCTATGCAGACTCCGTGAAGGGCCGAT TCACCATCTCCAGAGACAATTCCAAGAACATGCT GTATCTGCAAATGAATAGCCTGAGAGCTGAGGA CACGGCTGTGTATTACTGTGTGAGACGGGGATAC AGCTATGGCGGGTACGGTATGGACGTCTGGGGC CAAGGGGCCACGGTCACCGTCTCCTCA |
| | | | SEQ ID NO: 25001 | SEQ ID NO: 29007 |

FIGURE 50

| | | | | |
|---|---|---|---|---|
| | | AA | DIQMTQSPSSLSASVGDRVTITCQASQDINNYLN WYQQKPGKAPKLLIYDASNLETGVPSRFSGSGS GTDFTFTISSLQPEDITTYYCQQYENLPITFGQGT RLEIK | QVQLVESGGGVVQPGRSLRLSCAASGFTFSSYGMH WVRQAPGKGLEWVAIISYAGSNKSYADSVKGRFTI SRDNSKNMLYLQMNSLRAEDTAVYYCVRRGYSY GGYGMDVWGQGATVTVSS |
| | | | SEQ ID NO: 25002 | SEQ ID NO: 29008 |
| iPS:394033 | 21-225_5F4 | NA | GACATCCAGATGACCCAGTCTCCATCCTCCCT GTCTGCATCTGTAGGAGACAGAGTCACCATCA CTTGCCGGGCAAGTCAGGGCATTCGAAATCAT TTAGGCTGGTATCAGCAGAAACCAGGGAAAG CCCCTAAGCGCCTGATCTATGCTGCCTCCAGTT TGCAAAGTGGGGTCCCATCAAGGTTCAGCGGC AGTGGATCTGGGACAGAATTCACTCTCACAGT CAGCAGCCTGCAGCCTGAAGATTTTGCAACTT ATTACTGTCTACAGTATAATGGTTACCCATTCA CTTTCGGCCCTGGGACCAAAGTGGATATCAAA | GAGGTGCAGTTGGTGGAGTCTGGGGGAGGCCTG GTCAAGCCTGGGGGGTCCCTGAGACTCTCCTGTG TAGCCTCTGGATTCACCTTCAGTAGCTATAGCAT GAACTGGGTCCGCCAGGCTCCAGGGAAGGGGCT GGAGTGGGTCTCATCCATTAGTGGTAGTAGTAGT TACATATACTACGCAGACTCAGTGAAGGGCCGAT TCACCATCTCCAGAGACAACGCCAAGAACTCACT GTTTCTGCAAATGAACAGCCTGAGAGCCGAGGA CACGGCTGTGTATTACTGTGCGAGAGTTAACAAC TTTGACTACTGGGGCCAGGGAACCCTGGTCACCG TCTCCTCA |
| | | | SEQ ID NO: 25003 | SEQ ID NO: 29009 |
| | | AA | DIQMTQSPSSLSASVGDRVTITCRASQGIRNHLG WYQQKPGKAPKRLIYAASSLQSGVPSRFSGSGS GTEFTLTVSSLQPEDFATYYCLQYNGYPFTFGPG TKVDIK | EVQLVESGGGLVKPGGSLRLSCVASGFTFSSYSMN WVRQAPGKGLEWVSSISGSSSYIYYADSVKGRFTIS RDNAKNSLFLQMNSLRAEDTAVYYCARVNNFDY WGQGTLVTVSS |
| | | | SEQ ID NO: 25004 | SEQ ID NO: 29010 |

FIGURE 50

| | | | | |
|---|---|---|---|---|
| iPS:394035 | 21-225_5G9 | NA | GACATCCAGATGACCCAGTCTCCATCCTCCCTGTCTGCATCTGTAGGAGACAGAGTCACCATCACTTGCCAGGCGAGTCAGGGCATTAGCAACTCTTTAAATTGGTATCAGCAGAAACCAGGAAAAGCCCCTAAACTCCTGATCTACGATGCATCCAATTTGGAAACAGGGGTCCCATCAAGGTTCAGTGGAAGTGGAGCTGGGACAGATTTTACTTTCACCATCAGCAGCCTGCAGCCTGAAGATATTGCAACATATTACTGTCAACAATATGATAATCTCCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAATTATGGCATGCACTGGGTCCGCCAGGCTCCAGGCAAGGGGCTGGAGTGGGTGGCAATTATATCATATGCTGGAAGTAATAAATACTATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTACTGTGTGAGACGTATAACAGCTCGTCTCTACTACGGTATGGACGTCTGGGGCCAAGGGACCACGGTCACCGTCTCCTCA |
| | | | SEQ ID NO: 25005 | SEQ ID NO: 29011 |
| | | AA | DIQMTQSPSSLSASVGDRVTITCQASQGISNSLNWYQQKPGKAPKLLIYDASNLETGVPSRFSGSGAGTDFTFTISSLQPEDIATYYCQQYDNLPLTFGGGTKVEIK | QVQLVESGGGVVQPGRSLRLSCAASGFTFSNYGMHWVRQAPGKGLEWVAIISYAGSNKYYADSVKGRFTISRDNSKNTLYLQMNSLRAEDTAVYYCVRRITARLYYGMDVWGQGTTVTVSS |
| | | | SEQ ID NO: 25006 | SEQ ID NO: 29012 |
| iPS:394037 | 21-225_4F4 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCGTCCAGTGTGCAAACTGGGGTCCCATCAAGGTTCAGCGGCAGTGGATCTGGGACAGAATTCACTCTCACAATCAGCAGCCTGCAGCCTGAAGATTTTGCAATTTATTACTGTCTACAGCATAATAGTTACCCGCTCACTTTCGGCGGAGGGACCAAGGTGGAGATCAAA | CAGCTGCAGCTGCAGGAGTCGGGCCCAGGACTGGTGAGGCCTTCGGAGACCCTGTCCCTCACCTGCACTGTCTCTGGTGGCTCCATCAGTAGGAGTAGTTACTACTGGGGCTGGATCCGCCAGCCCCCAGGAAAGGGGCTGGAGTGGATTGGGAATATTTATTATAGTGGGAGCACCTACGACAACCCGTCCCTCAAGAGTCGAGTCACCATATCCGTAGACACGTCCAAGAACCAGTTCTCCCTGAAGCTGAGCTCTGTGACCGCCGCGGACACGGCTGTTTATTACTGTGGGAGACATGGAAAAGACTGGGGCCTTGACTACTGGGGCCAGGGAACCCTGGTCACCGTCTCCTCA |
| | | | SEQ ID NO: 25007 | SEQ ID NO: 29013 |

FIGURE 50

| | | | | |
|---|---|---|---|---|
| | | AA | DIQMTQSPSSLSASVGDRVTITCRASQGIRNDLGWYQQKPGKAPKRLIYAASSVQTGVPSRFSGSGSGTEFTLTISSLQPEDFAIYYCLQHNSYPLTFGGGTKVEIK | QLQLQESGPGLVRPSETLSLTCTVSGGSISRSSYYWGWIRQPPGKGLEWIGNIYYSGSTYDNPSLKSRVTISVDTSKNQFSLKLSSVTAADTAVYYCGRHGKDWGLDYWGQGTLVTVSS |
| | | | SEQ ID NO: 25008 | SEQ ID NO: 29014 |
| iPS:394041 | 21-225_5E5 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTACCCTCGGACGTTCGGCCAAGGGACCAAGGTGGAAGTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTACGAGAGAGGTATATAGCAGTGGCTGGTACGACTACGGTATGGACGTCTGGGGCCAAGGGACCACGGTCACCGTCTCCTCA |
| | | | SEQ ID NO: 25009 | SEQ ID NO: 29015 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPRTFGQGTKVEVK | QVQLVESGGGVVQPGRSLRLSCAASGFTFSNYVMHWVRQAPGKGLEWVAVIWYDGSNKYYADSVKGRFTISRDNSKNTLYLQMNSLRAEDTAVYYCTREVYSSGWYDYGMDVWGQGTTVTVSS |
| | | | SEQ ID NO: 25010 | SEQ ID NO: 29016 |

FIGURE 50

| | | | | |
|---|---|---|---|---|
| **iPS:394043** | **21-225_3B1** | NA | GACATCCAGATGACCCAGTCTCCATCCTCCCTGTCTGCATCTGTAGGAGACAGAGTCACCATCACTTGCCGGGCAAGTCAGAGTATTAATAATTATTTAAATTGGTATCAGCAGAAACCAGGGAAAGCCCCTAAGCTCCTGATCTATGCTACATCCAGTTTGCAAAATGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACAGTACCCCCTTATTCACTTTCGGCCCTGGGACCAAAGTGGATATCAAT | GAGGTGCAGCTGTTGGAGTCTGGGGGAGGCTTGGTACAGCCTGGGGGGTCCCTGAGACTCTCCTGTGCAGCCTCTGAATTCACCTTTAGCAGCTATGCCATGAACTGGGTCCGCCAGGCTCCAGGGAAGGGGCTGGAGTGGGTCTCAGTTATTAGTGGTCGTGGTATTAACACATTCTACGCAGACTCCGTGAAGGGCCGGTTCACCATTTCCAGAGACAATTCCAAGAACACGCTGTATCTGCAAATGAACAGCCTGAGAGCCGAGGACACGGCCGTATATTACTGTGCGTCCCGTTTAGCAGTGGCTGGCTCGGAGGCTTTTGATATCTGGGGCCAAGGGACAATGGTCACCGTCTCTTCA |
| | | | SEQ ID NO: 25011 | SEQ ID NO: 29017 |
| | | AA | DIQMTQSPSSLSASVGDRVTITCRASQSINNYLNWYQQKPGKAPKLLIYATSSLQNGVPSRFSGSGSGTDFTLTISSLQPEDFATYYCQQSYSTPLFTFGPGTKVDIN | EVQLLESGGGLVQPGGSLRLSCAASEFTFSSYAMNWVRQAPGKGLEWVSVISGRGINTFYADSVKGRFTISRDNSKNTLYLQMNSLRAEDTAVYYCASRLAVAGSEAFDIWGQGTMVTVSS |
| | | | SEQ ID NO: 25012 | SEQ ID NO: 29018 |
| **iPS:394045** | **21-225_4H4** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCGGGGAAAGCCCCTAAGCGCCTGATCTATGCTGCATCCAGTGTGCAAAGTGGGGTCCCATCAAGGTTCAGCGGCAGTGGATCTGGGACAGAATTCACTCTCACAATCAGCAGCCTGCAGCCTGAAGATTTTGCAATTTATTACTGTCTACAGCATAATAGTTACCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGTAGGAGTAGTTACTACTGGGGCTGGATCCGCCAGCCCCCAGGGATGGGGCTGGAATGGATTGGGAATATTTATTATAGTGGGAACACCTACAACAACCCGTCCCTCAAGAGTCGAGTCACCATATCCGTAGACACGTCCAAGAACCAGTTCTCCCTGAAGCTGAACTCTGTGACCGCCGCAGACACGGCTGTGTATTACTGTGGGAGACATGGAAAAGACTGGGGCCTTGACTACTGGGGCCAGGGAACCCTGGTCATCGTCTCCTCA |
| | | | SEQ ID NO: 25013 | SEQ ID NO: 29019 |

FIGURE 50

| | | | | |
|---|---|---|---|---|
| | | AA | DIQMTQSPSSLSASVGDRVTITCRASQGIRNDLGWYQQKPGKAPKRLIYAASSVQSGVPSRFSGSGSGTEFTLTISSLQPEDFAIYYCLQHNSYPLTFGGGTKVEIK | QLQLQESGPGLVKPSETLSLTCTVSGGSISRSSYYWGWIRQPPGMGLEWIGNIYYSGNTYNNPSLKSRVTISVDTSKNQFSLKLNSVTAADTAVYYCGRHGKDWGLDYWGQGTLVIVSS |
| | | | SEQ ID NO: 25014 | SEQ ID NO: 29020 |
| **iPS:394047** | **21-225_5E6** | NA | GACATCCAGATGACCCAGTCTCCATCCTCCCTGTCTGCATCTGTAGGAGACAGAGTCACCATCACTTGCCAGGCGAGTCAGGACATTAACAACTATTTAAATTGGTGTCAACAGAAACCAGGGAAAGCCCCTAAGCTCTTGATATACGATGCATCCAATTTGGAAACAGGGGTCCCATCAAGGTTCAGTGGAAGTGGATCTGGGACAGATTTTACTTTCACCATCAGCAGCCTGCAGCCTGAAGATATTGCAACATATTACTGTCAACAGTATGATAATCTCCCGATCACCTTCGGCCAAGGGACACGACTGGAGATTAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAGTGGGTGGCAATTATATCATATGTTGGAAATAATAAATACTATGCAGACTCCGTGAAGGGCCGATTCACCATCTCCAGAGACAATTCCAAGAACACGCTGTATCTGCAAATGAACAGCCTGAGAGTTGAGGACACGGCTGTGTATTACTGTGCGAGACGGGGATACAGCTATGGCGGGTACGGTATGGACGTCTGGGGCCAAGGGACCACGGTCACCGTCTCCTCA |
| | | | SEQ ID NO: 25015 | SEQ ID NO: 29021 |
| | | AA | DIQMTQSPSSLSASVGDRVTITCQASQDINNYLNWCQQKPGKAPKLLIYDASNLETGVPSRFSGSGSGTDFTFTISSLQPEDIATYYCQQYDNLPITFGQGTRLEIK | QVQLVESGGGVVQPGRSLRLSCAASGFTFSSYGMHWVRQAPGKGLEWVAIISYVGNNKYYADSVKGRFTISRDNSKNTLYLQMNSLRVEDTAVYYCARRGYSYGGYGMDVWGQGTTVTVSS |
| | | | SEQ ID NO: 25016 | SEQ ID NO: 29022 |

FIGURE 50

| | | | | |
|---|---|---|---|---|
| iPS:394049 | 21-225_13H5 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAACTGGGGTCCCATCAAGATTCAGCGGCAGTGGATCTGGGACAGAATTCATTCTCACAATCAGCAGCCTGCAGCCTGAAGATTTTGCAACTTATTACTGTCTACAGCATAATAGTTACCCTCTCACTTTCGGCGGAGGGACCAAGGTGGAGATCAAA | CAGCTGCAGCTGCAGGAGTCGGGCCCAGGACTGGTGAAGCCTGCGGAGACCCTGTCCCTCACCTGCACTGTCTCTGGTGGCTCCATCAGCAGAAGTAGTTACTACTGGGGCTGGATCCGCCAGCCCCCAGGGAAGGGGCTGGAGTGGATTGGGAGTATCTATTATAGTGGGAGCACCTACTACAACCCGTCCCTCAAGAGTCGAGTCACCATATCCGTGGACACGTCCAAGAACCAGTTCTCCCTGAAGCTGAGCTCTGTGACCGCCGCAGACACGGCTGTGTATTACTGTGCGAGCCTTAGCAGCAGCTGGGACTTCCAGCACTGGGGCCAGGGCACCCTGGTCACCGTCTCCTCA |
| | | | SEQ ID NO: 25017 | SEQ ID NO: 29023 |
| | | AA | DIQMTQSPSSLSASVGDRVTITCRASQGIRNDLGWYQQKPGKAPKRLIYAASSLQTGVPSRFSGSGSGTEFILTISSLQPEDFATYYCLQHNSYPLTFGGGTKVEIK | QLQLQESGPGLVKPAETLSLTCTVSGGSISRSSYYWGWIRQPPGKGLEWIGSIYYSGSTYYNPSLKSRVTISVDTSKNQFSLKLSSVTAADTAVYYCASLSSSWDFQHWGQGTLVTVSS |
| | | | SEQ ID NO: 25018 | SEQ ID NO: 29024 |
| iPS:394051 | 21-225_9E5 | NA | GACATCCAGATGACCCAGTCTCAATCCTCCCTGTCTGCATCTGTAGGAGACAGAGTCACCATCACTTGCCGGGCAAGTCAGAGCATTGCCAGTTATTTAAATTGGTATCAGCAGAAACCAGGGAAAGCCCCTAAACTCCTGATCTATGGTGCATCCAGTTTGCAAAGTGGGGTCCCATCAAGGTTCAGTGGCAGTGGATCTGGGACAGATTTCACTCTCACCATCAGCAGTCTCCAACCTGAAGATTTTGCAACTTACTACTGTCAACAGAGTTACAGTACCCCCTTATTCAGTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAACTATGCCATGAACTGGGTCCGCCAGGCTCCAGGGAAGGGGCTGGAGTGGGTCTCAGCTATTAGTGGTGGTGGTGGTAACACATTCTACGCAGACTCCGTGAAGGGCCGGTTCACCATCTCCAGAGACAATTCCAAGAACACGCTGTATCTGCAAATGAACGGCCTGAGAGCCGAGGACACGGCCGTATATTACTGTGCGTCCCGTATAGCAGTGGCTGGCTCGGAGGCTTTTGCTATCTGGGGCCAAGGGACATTGGTCACCGTCTCTTCA |
| | | | SEQ ID NO: 25019 | SEQ ID NO: 29025 |

FIGURE 50

| | | | | |
|---|---|---|---|---|
| | | AA | DIQMTQSQSSLSASVGDRVTITCRASQSIASYLNWYQQKPGKAPKLLIYGASSLQSGVPSRFSGSGSGTDFTLTISSLQPEDFATYYCQQSYSTPLFSFGPGTKVDIK | EVQLLESGGGLVQPGGSLRLSCAASGFTFSNYAMNWVRQAPGKGLEWVSAISGGGGNTFYADSVKGRFTISRDNSKNTLYLQMNGLRAEDTAVYYCASRIAVAGSEAFAIWGQGTLVTVSS |
| | | | SEQ ID NO: 25020 | SEQ ID NO: 29026 |
| **iPS:394053** | **21-225_11F10** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTACAAAGTGGGGTCCCATCAAGGTTCAGCGGCAGTGGTTCTGGGACAGAATTCACTCTCACAGTCAGCAGCCTGCAGCCTGAAGATTTTGCAACTTATTACTGTCTACAGCATAGTAGTTACCCTCTCACTTTCGGCGGAGGGACCAAGGTGGAGATCAAA | CAGCTGCATCTGCAGGAGTCGGGCCCAGGACTGGTGAAGCCTTCGGAGACCCTGTCCCTCACCTGCACTGTCTCTGGTGCCTCCATCAGCAGAAGTAGTTACTACTGGGGCTGGATCCGCCAGCCCCCAGGGAAGGGGCTGGAGTGGATTGGGAGTATTTATTATAGTGGGAGCGCCCAGTACAACCCGTCCCTCAAGAGTCGAGTCACCATATCCGTAGACACGTCCAAGAACCAGTTCTCCCTGACGCTGAGCTCTGTGACCGCCGCAGACACGGCTGTATATTACTGTGCGAGACTGAGCAGCAGCTGGTCTTTTGACTACTGGGGCCAGGGAACCCTGGTCACCGTCTCCTCA |
| | | | SEQ ID NO: 25021 | SEQ ID NO: 29027 |
| | | AA | DIQMTQSPSSLSASVGDRVTITCRASQGIRNDLGWYQQKPGKAPKRLIYAASSLQSGVPSRFSGSGSGTEFTLTVSSLQPEDFATYYCLQHSSYPLTFGGGTKVEIK | QLHLQESGPGLVKPSETLSLTCTVSGASISRSSYYWGWIRQPPGKGLEWIGSIYYSGSAQYNPSLKSRVTISVDTSKNQFSLTLSSVTAADTAVYYCARLSSSWSFDYWGQGTLVTVSS |
| | | | SEQ ID NO: 25022 | SEQ ID NO: 29028 |

FIGURE 50

| | | | | |
|---|---|---|---|---|
| iPS:394055 | 21-225_9C8 | NA | GACATCCAGATGACCCAGTCTCCATCCTCACTGTCTGCATCTGTAGGAGACAGAGTCACCATCACTTGTCGGGCGAGTCAGGGCATTAGCTATTATTTAGCCTGGTTTCAGCAGAAACCAGGGAAAGCCCCTAAGTCCCTGATCTATGTTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATGATAGTTACCCATTCACTTTCGGCCCTGGGACCAAAGTGGATATCAAA | GAGGTGCAACTGGTGGAGTCTGGGGGAGGCTTGGTACAGCCTGGGGGGTCCCTGAGACTCTCCTGTGTAGTCTCTGGATTCACCTTCAGTAGCCAGAGCATGAACTGGGTCCGCCAGGCTCCAGGGAAGGGGCTGGAGTGGGTCTCATACATTAGTATTAGTAGTACCATATACTATGCAGACTCTGTGAAGGGCCGATTCACCATCTCCAGAGACAATGCCAAGAACTCACTGTATCTGCAAATGAACAGCCTGAGAGACGAGGACACGGCTGTGTATTACTGTGCGAGAGGAGGTGGGAGCCCTTTTGACTCCTGGGGCCAGGGAACCCTGGTCACCGTCTCCTCA |
| | | | SEQ ID NO: 25023 | SEQ ID NO: 29029 |
| | | AA | DIQMTQSPSSLSASVGDRVTITCRASQGISYYLAWFQQKPGKAPKSLIYVASSLQSGVPSKFSGSGSGTDFTLTISSLQPEDFATYYCQQYDSYPFTFGPGTKVDIK | EVQLVESGGGLVQPGGSLRLSCVVSGFTFSSQSMNWVRQAPGKGLEWVSYISISSTIYYADSVKGRFTISRDNAKNSLYLQMNSLRDEDTAVYYCARGGGSPFDSWGQGTLVTVSS |
| | | | SEQ ID NO: 25024 | SEQ ID NO: 29030 |
| iPS:394057 | 21-225_15H1 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATACTGCATCCAGTTTGCAAAGTGGGGTCCCATCAAGGTTCAACGGCAGTGGATCTGGGACAGAATTCACTCTCACAATCAGCAGCCTGCAGCCTGAAGATTTTGCAACTTATTACTGTCTACAGCATAGTAGTTACCCGCTCACTTTCGGCGGAGGGACCAAGGTGGAGATCAAA | CAGCTGCAGTTGCAGGAGTCGGGCCCAGGACTGGTGAAGTTTTCGGAGACCCTGTCCCTCACCTGCACTGTCTCTGGTGGCTCCATCAGCAGAAGTAGTTACTACTGGGGCTGGATCCGCCAGCCCCCAGGGAAGGGGCTGGAGTGGATAGGGAATATCTATTATAGTGGGTATCCCTACTACAATCCGTCCCTCAAGAGTCGAGTCACCATATCCGTAGACACGTCCAAGAACCAGTTCTCCCTGAAGCTGAGCTCTGTGACCGCCGCAGACACGGCTGTGTATTACTGTGCGAGACATAGCACCAGCTGGTCCCTTGACTACTGGGGCCAGGGAACCCTGGTCACCGTCTCCTCA |
| | | | SEQ ID NO: 25025 | SEQ ID NO: 29031 |

FIGURE 50

| | | | | |
|---|---|---|---|---|
| | | AA | DIQMTQSPSSLSASVGDRVTITCRASQGIRNDLGWYQQKPGKAPKRLIYTASSLQSGVPSRFNGSGSGTEFTLTISSLQPEDFATYYCLQHSSYPLTFGGGTKVEIK | QLQLQESGPGLVKFSETLSLTCTVSGGSISRSSYYWGWIRQPPGKGLEWIGNIYYSGYPYYNPSLKSRVTISVDTSKNQFSLKLSSVTAADTAVYYCARHSTSWSLDYWGQGTLVTVSS |
| | | | SEQ ID NO: 25026 | SEQ ID NO: 29032 |
| iPS:394059 | 21-225_9E8 | NA | GACATCCAGATGACCCAGTCTCCATCCTCCCTGTTTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTTATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGAAGCGTCTGGATTCACCTTCAGTAGCTATGGCATGCACTGGGTCCGCCAGGCTCCAGGCAAGGGGCTGGAGTGGGTGGCAGTTATTTGGTATGAAGAAAATAATCAATACTATGCAGACTCCGTGAAGGGCCGATTCACCATCTCCAGAGACAATTCCAAGAACACGCTGTATCTACAAATGAACAGCCTGAGAGCCGAGGACACGGCTGTGTATTACTGTGCGAGAGAACTGGGGTTCCGGTCTGACTACTGGGGCCAGGGAACCCTGGTCACCGTCTCCTCA |
| | | | SEQ ID NO: 25027 | SEQ ID NO: 29033 |
| | | AA | DIQMTQSPSSLFASVGDRVTITCRASQGIRNDLGWYQQKPGKAPKRLIYAASSLQSGVPSRFSGSGSGTEFTLTISSLQPEDFATYYCLQHNSYPLTFGGGTKVEIK | QVQLVESGGGVVQPGRSLRLSCEASGFTFSSYGMHWVRQAPGKGLEWVAVIWYEENNQYYADSVKGRFTISRDNSKNTLYLQMNSLRAEDTAVYYCARELGFRSDYWGQGTLVTVSS |
| | | | SEQ ID NO: 25028 | SEQ ID NO: 29034 |

FIGURE 50

| | | | | |
|---|---|---|---|---|
| **iPS:394061** | **21-225_12D2** | NA | GATATTGTGATGACTCAGTCTCCACTCTCCCTGCCCGTCACCCCTGGTGAGCCGGCCTCCATCTCCTGCAGGTCTAGTCAGAGCCTCCTCCATAGTAATGGATACAACTATTTGGATTGGTACCTGCAGAAGCCAGGGCAGTCTCCACAGGTCCTGATCTATTTGGGTTCTAATCGGGCCTCCGGGGTCCCTGACAGGTTCAGTGGCAGTGGATCAGGCACAGATTTTACTCTGAAAATCAGCAGAGTGGAGGCTGAGGATGTTGGGGTTTATTACTGCATGCAAGCTCTACAAACTCCTATCACCTTCGGCCAAGGGACACGACTGGAGATTAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTAGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGATTAGGGGACTACTGGGGCCAGGGAACCCTGGTCGCCGTCTCCTCA |
| | | | SEQ ID NO: 25029 | SEQ ID NO: 29035 |
| | | AA | DIVMTQSPLSLPVTPGEPASISCRSSQSLLHSNGYNYLDWYLQKPGQSPQVLIYLGSNRASGVPDRFSGSGSGTDFTLKISRVEAEDVGVYYCMQALQTPITFGQGTRLEIK | EVQLVESGGGLVKPGGSLRLSCAASGFTFSSYSMNWVRQAPGKGLEWVSSISSSSSYIYYADSVKGRFTISRDNAKNSLYLQMNSLRAEDTAVYYCARLGDYWGQGTLVAVSS |
| | | | SEQ ID NO: 25030 | SEQ ID NO: 29036 |
| **iPS:394063** | **21-225_16A1** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCGTCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACACCATAGTAATTACCCTCTCACTTTCGGCGGAGGGACCAAGGTGGAGATCGAA | CAGCTGCAGCTGCAGGAGTCGGGCCCAGGACTGGTGAAGCCTTCGGAGACCCTGTCCCTCATTTGTACTGTCTCTGGTGGCTCCATCGACAGGAGTAGTTACTACTGGGGCTGGATCCGCCAGCCCCCAGGGAAGGGGCTGGAGTGGATTGGGAGTATCTATTATAGTGGGAGCGCCTATCACAACCCGTCCCTCAAGAGTCGAGGCACCATATCCGTAGATACGTCCAAGAACCAGTTCTCCCTGAAGCTGAGCTCTGTGACCGCCGCAGACACGGCTGCGTATTACTGTGCGAGACTGAGCAGCAGCTGGTCCTTTGACTACTGGGGCCAGGGAACCCTGGTCACCGTCTCCTCT |
| | | | SEQ ID NO: 25031 | SEQ ID NO: 29037 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLHHSNYPLTFGGGTKVEIE | QLQLQESGPGLVKPSETLSLICTVSGGSIDRSSYYWGWIRQPPGKGLEWIGSIYYSGSAYHNPSLKSRGTISVDTSKNQFSLKLSSVTAADTAAYYCARLSSSWSFDYWGQGTLVTVSS |
| | | | SEQ ID NO: 25032 | SEQ ID NO: 29038 |
| iPS:394065 | 21-225_11E2 | NA | GACATCGTGATGACCCAGTCTCCAGACTCCCTGGCTGTGTCTCTGGGCGAGAGGGCCACCATCAACTGCAAGTCCAACCAGAGAGTTTTATCCAGCTCCAACAATCACAACTACTTAGCTTGGTACCAGCAGAGACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTTTAGTACTCCATTCACTTTCGGCCCTGGGACCAAAGTGGATATCAAA | CAGGTGCAGGTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACACTAACAGTGGTAACACAGGCTATGCACAGAAGTTCCAGGGCAGAGTCACCATGACCAGGAACACCTCCATAAGCACAGCCTACATGGACCTGAGCAGCCTGAGATCTGAGGACACGGCCGTATATTACTGTGCGTATAGTCATGGCTGGTTCCTCTTTGACTACTGGGGCCAGGGAATCCTGGTCACCGTCTCCTCA |
| | | | SEQ ID NO: 25033 | SEQ ID NO: 29039 |
| | | AA | DIVMTQSPDSLAVSLGERATINCKSNQRVLSSSNNHNYLAWYQQRPGQPPKLLIYWASTRESGVPDRFSGSGSGTDFTLTISSLQAEDVAVYYCQQYFSTPFTFGPGTKVDIK | QVQVVQSGAEVKKPGASVKVSCKASGYTFTNYDINWVRQATGQGLEWMGWMNTNSGNTGYAQKFQGRVTMTRNTSISTAYMDLSSLRSEDTAVYYCAYSHGWFLFDYWGQGILVTVSS |
| | | | SEQ ID NO: 25034 | SEQ ID NO: 29040 |

FIGURE 50

| | | | | |
|---|---|---|---|---|
| **iPS:394067** | **21-225_12F2** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTACCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGTAACTTATTACTGTCTACAGCATAATAGTTATCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTTTGATGGAAATAATAAATACTATGTAGATTCCGTGAAGGGCCGATTCACCATCTCCAGAGACAATTCCAAGAACACGCTGTTTCTGCAAATGAACAGCCTGAGAGCCGAGGACACGGCTGTGTATTACTGTGTGAGAGAGCTTGCCTGGTCCGAGGACTACTGGGGCCAGGGAACCCTGGTCACCGTCTCCTCA |
| | | | SEQ ID NO: 25035 | SEQ ID NO: 29041 |
| | | AA | DIQMTQSPSSLSASVGDRVTITCRASQGIRNDLGWYQQKPGKAPKRLIYAASSLQSGVPSRFSGSGSGTEFTLTISSLQPEDFVTYYCLQHNSYPWTFGQGTKVEIK | QVQLVESGGGVVQPGRSLRLSCAASGFTFSDYGMHWVRQAPGKGLEWVAVIWFDGNNKYYVDSVKGRFTISRDNSKNTLFLQMNSLRAEDTAVYYCVRELAWSEDYWGQGTLVTVSS |
| | | | SEQ ID NO: 25036 | SEQ ID NO: 29042 |
| **iPS:394069** | **21-225_16H1** | NA | GACATCCAGATGACCCAGTCTCCATCCTCCCTGTCTGCATCTGTAGGAGACAGAGTCACCATCACTTGCCGGGCAAGTCAGGGCATTAGAGATATTTTAGGCTGGTATCAGCAGAAACCAGGGAAAGCCCCTAAGCGCCTGATCTATGCTGCATCCAGTTTGCAAAATGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGTATCATAGTTACCCATTCACTTTCGGCCCTGGGACCAAAGTGGATGTCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTACTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGTAGCAGCCTTTGACTACTGGGGCCAGGGAACCCTGGTCACCGTCTCCTCA |
| | | | SEQ ID NO: 25037 | SEQ ID NO: 29043 |

FIGURE 50

| | | | | |
|---|---|---|---|---|
| | | AA | DIQMTQSPSSLSASVGDRVTITCRASQGIRDILGWYQQKPGKAPKRLIYAASSLQNGVPSRFSGSGSGTEFTLTISSLQPEDFATYYCLQYHSYPFTFGPGTKVDVK | EVQLVESGGGLVKPGGSLRLSCAASGFTFSSYSMNWVRQAPGKGLEWVSSISGSSTYIYYADSVKGRFTISRDNAKNSLYLQMNSLRAEDTAVYYCARVAAFDYWGQGTLVTVSS |
| | | | SEQ ID NO: 25038 | SEQ ID NO: 29044 |
| iPS:394071 | 21-225_10C7 | NA | GATATTGTGATGACTCAGTCTCCACTCTCCCTGCCCGTCACCCCTGGAGAGCCGGCCTCCATCTCCTGCAGGTCTAGTCAGAGCCTCCTGCATAGTAAGGGATACAACTATTTGGATTGGTACCTGCAGAAGCCAGGGCAGTCTCCACAGGTCCTGATCTATTTGGGTTCTAATCGGGCCTCCGGGGTCCCTGACAGGTTCAGTGGCAGTGGATCAGGCACAGATTTTACACTGAAAATCAGCAGAGTGGAGGCTGAGGATGTTGGGGTTTATTACTGCATGCAAGCTCTACAAACTCCTCTCACCTTCGGCCAAGGGACACGACTGGAGATTAAA | GAGGTGCAGCTGGTGGAGTCTGGGGGAGGCCTGGTCAAGCCGGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTAGTAGTAATAATTACATATACTACGCAGACTCAGTGAAGGGCCGATTCACCATCTCCAGAGACAACGCCAAGAACTCACTGTATCTGCAAATGAACAGCCTGAGAGCCGAGGACTCGGCTGTGTATTACTGTGCGAGATTAGGGGTCTACTGGGGCCAGGGAACCCTGGTCACCGTCTCCTCA |
| | | | SEQ ID NO: 25039 | SEQ ID NO: 29045 |
| | | AA | DIVMTQSPLSLPVTPGEPASISCRSSQSLLHSKGYNYLDWYLQKPGQSPQVLIYLGSNRASGVPDRFSGSGSGTDFTLKISRVEAEDVGVYYCMQALQTPLTFGQGTRLEIK | EVQLVESGGGLVKPGGSLRLSCAASGFTFSSYSMNWVRQAPGKGLEWVSSISSSNNYIYYADSVKGRFTISRDNAKNSLYLQMNSLRAEDSAVYYCARLGVYWGQGTLVTVSS |
| | | | SEQ ID NO: 25040 | SEQ ID NO: 29046 |

FIGURE 50

| | | | | |
|---|---|---|---|---|
| **iPS:394073** | **21-225_15C9** | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAACAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAG TCAGCAGCCTGCAGCCTGAAGATTTTGCTACT TATTACTGTCTACAACATACTAGTTACCCGCTC ACTTTCGGCGGAGGGACCAAGGTGGAGATCA AA | CAGCTGCAGCTGCAGGAGTCGGGCCCAGGACTG GTGAAGCCTTCGGAGACCCTGTCCCTCACCTGCA CTGTCTCTGGTGGCTCCATCAGCAGAAGTAGTTA CTACTGGGGCTGGATCCGCCAGCCCCCAGGGAA GGGGCTGGAGTGGATTGGGAATATCTATTATAGT GGGAGCACCTACAACAACCCGTCCCTCAAGAGT CGAGTCACCATATCCGTAGACACGTCCAAGAACC AGTTCTCCCTGAAGCTGAGCTCTGTGACCGCCGC AGACACGGCTGTGTATTACTGTGCGAGACAGGG CAGTGGCTGGGAGGTTGACTACTGGGGCCAGGG AACCCTGGTCACCGTCTCCTCA |
| | | | SEQ ID NO: 25041 | SEQ ID NO: 29047 |
| | | AA | DIQMTQSPSSLSASVGDRVTITCRASQGIRNDLG WYQQKPGKAPKRLIYAASSLQSGVPSRFSGSGS GTEFTLTVSSLQPEDFATYYCLQHTSYPLTFGGG TKVEIK | QLQLQESGPGLVKPSETLSLTCTVSGGSISRSSYYW GWIRQPPGKGLEWIGNIYYSGSTYNNPSLKSRVTIS VDTSKNQFSLKLSSVTAADTAVYYCARQGSGWEV DYWGQGTLVTVSS |
| | | | SEQ ID NO: 25042 | SEQ ID NO: 29048 |
| **iPS:394075** | **21-225_8D12** | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAACAGAAACCAGGGAAAG CCCCTAAGCGCCTGATCTATGCTGCATCCAGT TTGCAAAGTGGGGTCCCATCAAGGTTCAACGG CAGTGGATCTGGGACAGAATTCACTCTCACAA TCAGCAGCCTGCAGCCTGAAGATTTTGCAACT TATTACTGTCTACAGCATAGTAGTTACCCGCTC ACTTTCGGCGGAGGGACCAAGGTGGAGATCA AA | CAGCTGCAGTTGCAGGAGTCGGGCCCAGGACTG GTGAAGTTTTCGGAGACCCTGTCCCTCACCTGCA CTGTCTCTGGTGGCTCCATCAGCAGAAGTAGTTA CTACTGGGGCTGGATCCGCCAGCCCCCAGGGAA GGGGCTGGAGTGGATAGGGAATATCTATTATAGT GGGTATCCCTACTACAATCCGTCCCTCAAGAGTC GAGTCACCATATCCATAGACACGTCCAAGAACC AGTTCTCCCTGAAGCTGAGCTCTGTGACCGCCGC AGACACGGCTGTGTATTACTGTGCGAGACATAGC ACCAGCTGGTCCCTTGACTACTGGGGCCAGGGAA CCCTGGTCACCGTCTCCTCA |
| | | | SEQ ID NO: 25043 | SEQ ID NO: 29049 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFNGSGSGTEFTLTISSLQPEDFATYYCLQHSSYPLTFGGGTKVEIK | QLQLQESGPGLVKFSETLSLTCTVSGGSISRSSYYWGWIRQPPGKGLEWIGNIYYSGYPYYNPSLKSRVTISIDTSKNQFSLKLSSVTAADTAVYYCARHSTSWSLDYWGQGTLVTVSS |
| | | | SEQ ID NO: 25044 | SEQ ID NO: 29050 |
| **iPS:394077** | **21-225_8E12** | NA | GACATCCAGATGACCCAGTCTCCATCCTCCCTGTCTGCATCTGTAGGAGACAGAGTCACCATCACTTGCCGGGCAAGTCAGAGCATTAGTAATTATTTAAATTGGTATCAGCAGAAACCAGGGAAAGCCCCTAAGCTCCTGATCTATGCTGCATCCAGTTTACAAAGTGGGGTCCCATCAAGGTTCAGTGGCAGTGGATCTGGGACAGATTTCACTCTCACCATCAGCAGTCTGCAACCTGAAGATTTTGCAACTTACTTCTGTCAACAGAGTTACAGAACCCCCTTTTTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGAATTCACCTTTAGCAGCTATGCCATGAGCTGGGTCCGCCAGGCTCCAGGGAAGGGGCTGGAGTGGGTCTCAATTATTAGTGGTCGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGTCCCGTATGGCAGTGGCTGGCTCGGAGGCTTTTGATATCTGGGGCCAAGGGACAATGGTCACCGTCTCTTCA |
| | | | SEQ ID NO: 25045 | SEQ ID NO: 29051 |
| | | AA | DIQMTQSPSSLSASVGDRVTITCRASQSISNYLNWYQQKPGKAPKLLIYAASSLQSGVPSRFSGSGSGTDFTLTISSLQPEDFATYFCQQSYRTPFFTFGPGTKVDIK | EVQLLESGGGLVQPGGSLRLSCAASEFTFSSYAMSWVRQAPGKGLEWVSIISGRGGNTFYADSVKGRFTISRDNSKNTLYLQMNSLRAEDTAVYYCASRMAVAGSEAFDIWGQGTMVTVSS |
| | | | SEQ ID NO: 25046 | SEQ ID NO: 29052 |

FIGURE 50

| | | | | |
|---|---|---|---|---|
| **iPS:394079** | **21-225_11F5** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGTCTGATCTATGCTGCATCCAGTGTGCAAAGTGGGGTCCCATCAAGGTTCAGCGGCAGTGGATCTGGGACAGAATTCACTCTCACAATCAGCAGCCTGCAGCCTGAAGATTTTGCAATTTATTACTGTCTACAGCATAATAGTTACCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGTAGGAGTAGTTACTACTGGGGCTGGATCCGCCAGCCCCCAGGGAAGGGGCTGGAATGGATTGGAAATATTTATTATAGTGGGAGCACCTACACCAACCCGTCCCTCAAGAGTCGAGTCACCATATCCGTAGACACGTCCAAGAACCACTTCTCCCTGAAGCTGAGCTCTGTGACCGCCGCAGACACGGCTGTGTATTACTGTGGGAGACATGGAAAAGACTGGGGCCTTGACAACTGGGGCCAGGGAACCCTGGTCACCGTCTCCTCA |
| | | | SEQ ID NO: 25047 | SEQ ID NO: 29053 |
| | | AA | DIQMTQSPSSLSASVGDRVTITCRASQGIRNDLGWYQQKPGKAPKRLIYAASSVQSGVPSRFSGSGSGTEFTLTISSLQPEDFAIYYCLQHNSYPLTFGGGTKVEIK | QLQLQESGPGLVKPSETLSLTCTVSGGSISRSSYYWGWIRQPPGKGLEWIGNIYYSGSTYTNPSLKSRVTISVDTSKNHFSLKLSSVTAADTAVYYCGRHGKDWGLDNWGQGTLVTVSS |
| | | | SEQ ID NO: 25048 | SEQ ID NO: 29054 |
| **iPS:394081** | **21-225_16B3** | NA | GACATCCAGATGACCCAGTCTCCATCCTCCCTGTCTGCATCTGTAGGAGACAGAGTCACCATCACTTGCCAGGCGAGTCAGGACATTAACAACTATTTAAATTGGTATCAGCAGAAACCAGGGAGAGCCCCTAAGCTCCTGATCTACGATGCATCCAATTTGGAAACAGGGGTCCCATCAAGGTTCAGTGGAAGTGGCTCTGGGACAGATTTTACTTTCACCATCAGCAGTCTGCAGCCTGAAGATATTGCAACATATTACTGTCAACAGTTTGATAATCTCCCGATCACCTTCGGCCAAGGGACACGACTGGAGATTAAA | CAGGTGCAGCTGGTAGAGTCTGGGGGAGGCGTGGTCCAGCCTGGGAGGTCCCTGAGACTCTCCTGTGTAGCCTCTGGATTCACCTTCAGTAGCTATGGCATGCACTGGGTCCGCCAGGCTCCAGGCAAGGGGCTGGAGTGGGTGGCAGTTATATCATATGCTGGAATTAATAAATCCTATGCAGACTCCGTGAAGGGCCGATTCACCATCTCCAGAGACAATTCCAATAACACGCTGTATCTGCAAATGAACAGCCTGAGAGCTGAGGACACGGCTGTGTATTACTGTGTGAGACGGGGATACAGCTATGGCGGGTATGGTATGGACGTCTGGGGCCAAGGGGCCACGGTCACCGTCTCCTCA |
| | | | SEQ ID NO: 25049 | SEQ ID NO: 29055 |

FIGURE 50

| | | | | |
|---|---|---|---|---|
| | | AA | DIQMTQSPSSLSASVGDRVTITCQASQDINNYLNWYQQKPGRAPKLLIYDASNLETGVPSRFSGSGSGTDFTFTISSLQPEDIATYYCQQFDNLPITFGQGTRLEIK | QVQLVESGGGVVQPGRSLRLSCVASGFTFSSYGMHWVRQAPGKGLEWVAVISYAGINKSYADSVKGRFTISRDNSNNTLYLQMNSLRAEDTAVYYCVRRGYSYGGYGMDVWGQGATVTVSS |
| | | | SEQ ID NO: 25050 | SEQ ID NO: 29056 |
| iPS:394083 | 21-225_16E6 | NA | GACATCCAGATGACCCAGTCTCCATCCTCCCTGTCTGCATCTGTAGGAGACAGAGTCACCATCACTTGCCGGGCAAGTCAGAGCATTATCAGCTATTTAAATTGGTATCAGCAGAAACCAGGGAAAGCCCCTAAGTTCCTGATCTATACTACATCCAGTTTGCAAAGTGGGGTCCCATCAAGGTTCAGTGGCAGTGGATCTGGGACAGATTTCACTCTCACCATCAGTAGTCTGCAACCTGAAGATTTTGCAACTTACTACTGTCAGCAGACTTACAGTACCCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGCATGATGGAAGTAATAAATACTATGTAGACTCCGTGAAGGGCCGATTCACCATCTCCAGAGACAATTCCAAGAACACGCTGAATCTGCAAATGAACAGCCTGAGAGCCGAGGACACGGCTGTGTATTACTGTGCGAGAGATCTGAGTATGGGCGGTATGGACGTCTGGGGCCAAGGGACCACGGTCACCGTCTCCTCA |
| | | | SEQ ID NO: 25051 | SEQ ID NO: 29057 |
| | | AA | DIQMTQSPSSLSASVGDRVTITCRASQSIISYLNWYQQKPGKAPKFLIYTTSSLQSGVPSRFSGSGSGTDFTLTISSLQPEDFATYYCQQTYSTPLTFGGGTKVEIK | QVQLVESGGGVVQPGRSLRLSCAASGFTFSSYGMHWVRQAPGKGLEWVAVIWHDGSNKYYVDSVKGRFTISRDNSKNTLNLQMNSLRAEDTAVYYCARDLSMGGMDVWGQGTTVTVSS |
| | | | SEQ ID NO: 25052 | SEQ ID NO: 29058 |

FIGURE 50

| | | | | |
|---|---|---|---|---|
| **iPS:394085** | **21-225_8B11** | NA | GACATCGTGATGACCCAGTCTCCAGACTCCCTGGCTGTGTCTCTGGGCGAGAGGGCCACCATCAACTGCAAGTCCAGCCAGAATGTTTTATACAACTCCAACAATAACAACTACTTAGCTTGGTACCAGCAGAAACCAGGACAGCCTCCTAAGCTGCTCATTTACTGGGCATCTACCCGGAAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATACTACTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCGCTGGACAAGGGCTTGAGTGGATGGGATGGATGCACCCTAACAGTGGTAACACAGGCTATGCACAGAAGTTCCAGGGCAGAGTCACCATGACCAGGAACACCTCCAGAAGCACAGCCTACATGGAGCTGAGCAGCCTGAGATCTGAGGACACGGCCGTGTACTACTGTGCGTATAGCAGTGGCTGGTACTTCTTTGACTACTGGGGCCAGGGAACCCTGGTCACCGTCTCCTCA |
| | | | SEQ ID NO: 25053 | SEQ ID NO: 29059 |
| | | AA | DIVMTQSPDSLAVSLGERATINCKSSQNVLYNSNNNNYLAWYQQKPGQPPKLLIYWASTRKSGVPDRFSGSGSGTDFTLTISSLQAEDVAVYYCQQYYTTPCSFGQGTKLEIK | QVQLVQSGAEVKKPGASVKVSCKASGYTFTNYDINWVRQAAGQGLEWMGWMHPNSGNTGYAQKFQGRVTMTRNTSRSTAYMELSSLRSEDTAVYYCAYSSGWYFFDYWGQGTLVTVSS |
| | | | SEQ ID NO: 25054 | SEQ ID NO: 29060 |
| **iPS:394087** | **21-225_11A5** | NA | GACATCCAGATGACCCAGTCTCCATCCTCCCTGTCTGCATCTGTAGGAGACAGAGTCACCATCACTTGCCGGGCAAGTCAGAACATTTATAGTTATTTAAATTGGTATCAGCAGAAACCAGGGAAAGCCCCTAAGCTCCTGATCTATGCTGCATCCAGTTTGCAAAGTGGGGTCCCATCAAGGTTCAGTGGCAGTGGATCTGGGACAGATTTCACTCTCACCATCAGCAGTCTACAACCTGAAGATTTTGCAACTTACTACTGTCAACAGAGTTACAATACCCCCTTATTCACTTTCGGCCCTGGGACCAAAGTGGATATCAAA | GAGGTGCAGCTGTTGGAGTCTGGGGGAGACTTGGTTCAGCCTGGGGGGTCCCTGAGACTCTCCTGTGCAGCCTCTGAATTCACCTTTAGCAGCTATGCCATGAGCTGGGTCCGCCAGGCTCCAGGGAAGGGGCTGGAGTGGATCTCAGTTATTAGTGGTCGTGGTGTTAACACATTCTACGCAGACTCCGTGAAGGGCCGGTTCACCATCTCCAGAGACAATTCCAAGAACACGCTCTATCTACAAATGAACAGCCTGAGAGCCGAGGACACGGCCGTATATTACTGTGCGTCCCGTATCGCAGTGGCTGGCTCGGAGGCTTTTGATATCTGGGGCCAAGGGACAATGGTCACCGTCTCTTCA |
| | | | SEQ ID NO: 25055 | SEQ ID NO: 29061 |

FIGURE 50

| | | | | |
|---|---|---|---|---|
| | | AA | DIQMTQSPSSLSASVGDRVTITCRASQNIYSYLNWYQQKPGKAPKLLIYAASSLQSGVPSRFSGSGSGTDFTLTISSLQPEDFATYYCQQSYNTPLFTFGPGTKVDIK | EVQLLESGGDLVQPGGSLRLSCAASEFTFSSYAMSWVRQAPGKGLEWISVISGRGVNTFYADSVKGRFTISRDNSKNTLYLQMNSLRAEDTAVYYCASRIAVAGSEAFDIWGQGTMVTVSS |
| | | | SEQ ID NO: 25056 | SEQ ID NO: 29062 |
| iPS:394089 | 21-225_12E6 | NA | GACATCCAGATGACCCAGTCTCCATCCTCCCTGTCTGCATCTGTAGGAGACAGAGTCACCATCACTTGCCGGGCAAGTCAGGGCATTAGAAGTGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGACAGTTATATGGTATGATGAAAGTAATAAATACTATGCAGACTCCGTGAAGGGCCGATTCACCATCTCCAGAGACGATTCCAAAAACACGCTGTATCTGCAAATGAACAGTCTGAGAGCCGAGGACACGGCTGTGTATTACTGTGCGAGAGAGCTTGCCTGGTACGAGGACTACTGGGGCCAGGGAACCCTGGTCACCGTCTCCTCA |
| | | | SEQ ID NO: 25057 | SEQ ID NO: 29063 |
| | | AA | DIQMTQSPSSLSASVGDRVTITCRASQGIRSDLGWYQQKPGKAPKRLIYAASSLQSGVPSRFSGSGSGTEFTLTISSLQPEDFATYYCLQHNSYPWTFGQGTKVEIK | QVQLVESGGGVVQPGRSLRLSCAASGFTFSDYGMHWVRQAPGKGLEWVTVIWYDESNKYYADSVKGRFTISRDDSKNTLYLQMNSLRAEDTAVYYCARELAWYEDYWGQGTLVTVSS |
| | | | SEQ ID NO: 25058 | SEQ ID NO: 29064 |

FIGURE 50

| | | | | |
|---|---|---|---|---|
| **iPS:394091** | **21-225_13H3** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTACAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCCTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATTTGGTATGAGGAAAGTAATAAATACTATGTAGACTCCGTGAGGGGCCGATTCACCATCTCCAGAGACAATTCCAAGAGCACGCTGTATCTGCAAATGAACAGCCTGAGAGCCGAGGACACGGCTGTGTATTACTGTGTGAGAGAACTAGGCTTCCAGTCTGACTTCTGGGGCCAGGGAACCCCGGTCACCGTCTCCTCA |
| | | | SEQ ID NO: 25059 | SEQ ID NO: 29065 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVEIK | QVQLVESGGGLVQPGRSLRLSCAASGFTFSSYGMHWVRQAPGKGLEWVAVIWYEESNKYYVDSVRGRFTISRDNSKSTLYLQMNSLRAEDTAVYYCVRELGFQSDFWGQGTPVTVSS |
| | | | SEQ ID NO: 25060 | SEQ ID NO: 29066 |
| **iPS:394093** | **21-225_9D12** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGGAAATAATAATTACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGCTTGCCTGGTACGAGGACTTCTGGGGCCAGGGAACCCTGGTCACCGTCTCCTCA |
| | | | SEQ ID NO: 25061 | SEQ ID NO: 29067 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPWTFGQGTKVEIK | QVQLVESGGGVVQPGRSLRLSCAASGFTFSDYGMHWVRQAPGKGLEWVAVIWYDGNNNYYADSVKGRFTISRDNSKNTLYLQMNSLRAEDTAVYYCARELAWYEDFWGQGTLVTVSS |
| | | | SEQ ID NO: 25062 | SEQ ID NO: 29068 |
| iPS:394095 | 21-225_16H4 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTTATCTATACTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGATGTAAGTAATAAATACTATGCAGACTCCGTGAAGGGCCGATTCACCATCTCCAGAGACAATTCCAAGAACATGTTGTATCTGCAAATGAACAGCCTGAGAGCCGAGGACACGGCTGTGTATTACTGTGCGCGAGAAATGGGCTGGACCGATGACTGCTGGGGCCAGGGAACCCTGGTCACCGTCTCCTCA |
| | | | SEQ ID NO: 25063 | SEQ ID NO: 29069 |
| | | AA | DIQMTQSPSSLSASVGDRVTITCRASQGIRNDLGWYQQKPGKAPKRLIYTASSLQSGVPSRFSGSGSGTEFTLTISSLQPEDFATYYCLQHNSYPWTFGQGTKVEIK | QVQLVESGGGVVQPGRSLRLSCAASGFTFSNYGMHWVRQAPGKGLEWVAVIWYDVSNKYYADSVKGRFTISRDNSKNMLYLQMNSLRAEDTAVYYCAREMGWTDDCWGQGTLVTVSS |
| | | | SEQ ID NO: 25064 | SEQ ID NO: 29070 |

FIGURE 50

| | | | | |
|---|---|---|---|---|
| **iPS:394097** | **21-225_16G7** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAACATAATAGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCACTGACTATGGCATGCACTGGGTCCGCCAGGCTCCAGGCAAGGGGCTGGAGTGGGTGGCAGTTATTTGGTATGATGAAAATAATGAATACTATGCAGACTCCGTGAAGGGCCGATTCACCATCTCCAGAGCCAATTCCAAGAACACGCTGTATCTGCAAATGAACAGCCTGAGAGCCGAGGACACGGCTGTGTATTACTGTGCGAGAGAGCTTGCCTGGTACGAGGACTACTGGGGCCAGGGAACCCTGGTCACCGTCTCCTCA |
| | | | SEQ ID NO: 25065 | SEQ ID NO: 29071 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPWTFGQGTKVEIK | QVQLVESGGGVVQPGRSLRLSCAASGFTFTDYGMHWVRQAPGKGLEWVAVIWYDENNEYYADSVKGRFTISRANSKNTLYLQMNSLRAEDTAVYYCARELAWYEDYWGQGTLVTVSS |
| | | | SEQ ID NO: 25066 | SEQ ID NO: 29072 |
| **iPS:398470** | **21-225_14B7** | NA | TCCTATGAGCTGACTCAGCCACCCTCAGTGTCCGTGTCCCCAGGACGGACAGCCAGCATCACCTGCTCTGGAGATAAATTGGGGAATAAATATGCTTACTGGTATCAGCAGAAGCCAGGCCAGTCCCCTGTGCTGGTCATCTATCAAGATAGAAAGCGGCCCTCAGGGATCCCTGAGCGATTCTCTGGCTCCAACTCTGGGAACACAGCCACTCTGACCATCAGCGGGACCCAGACTATGGATGAGGCTGACTATTACTGTCAGGCGTGGAACAACAGCACTGTGGTATTCGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAACAGTGGTGGCACAAACTATGTACAGAAGTTTCAGGGCAGGGTCACCATGACCAGGGACACGTCCATCAGCACAGCCTGCATGGAGCTGAGCAGGCTGAAATCTGACGACACGGCCGTGTATTTCTGTGCGAGGTCGTTTTTCTATGGTTCGGGGAGTTATTATAACGAATTTGACTACTGGGGCCAGGGAACCCTGGTCACCGTCTCCTCA |
| | | | SEQ ID NO: 25067 | SEQ ID NO: 29073 |

FIGURE 50

| | | | | |
|---|---|---|---|---|
| | | AA | SYELTQPPSVSVSPGRTASITCSGDKLGNKYAYWYQQKPGQSPVLVIYQDRKRPSGIPERFSGSNSGNTATLTISGTQTMDEADYYCQAWNNSTVVFGGGTKLTVL | QVQLVQSGAEVKKPGASVKVSCKASGYTFTGYYMHWVRQAPGQGLEWMGWINPNSGGTNYVQKFQGRVTMTRDTSISTACMELSRLKSDDTAVYFCARSFFYGSGSYYNEFDYWGQGTLVTVSS |
| | | | SEQ ID NO: 25068 | SEQ ID NO: 29074 |
| iPS:398472 | 21-225_16E4 | NA | CCCTATGAGCTGACTCAGCCACCCTCAGTGTCCGTGTCCCCAGGACAGACAGCCAGCATCACCTGCTCTGGAGATAAATTGGGGGATAAATATGTTTACTGGTATCAGCAGAAGTCAGGCCAGTCCCCTGTGCTGGTCATCTATCAAGATAGCAAGCGGCCCTCAGGGATCCCTGAGCGATTCTCTGGCTCCAACTCTGGAAACACAGCCGCTCTGACCATCAGCGGGACCCAGGCTATGGATGAGGCTGACTATTACTGTCAGGCGTGGGACAGCAGCACTGTGGTTTTCGGCGGAGGGACCAAGCTGACCGTCCTA | GAGGTGCAGCTGTTGGAGTCTGGGGGAGGCTTGATACAGCCTGGGGGGTCCCTGAGACTCTCCTGTGCAGCCTCTGGATTCACCTTTAGTAGCTATGTCATGAGCTGGGTCCGCCAGGCTCCAGGGAAGGGGCTGGAGTGGGTCTCCACTATTAGTGTTGGTGGTGGTACCACATACTACGCAGACTCCGTGAAGGGCCGGTTCACCATCTCCAGAGACAATTCCAAGAATACGCTGTATCTGCAAATGAACAGCCTGAGAGCCGAGGACACGGCCGTATATTACTGTGCGAAATGGGGACGTGGCAACAGCTATGAGTACTACTACGGTATGGACGTCTGGGGCCAAGGGACCACGGTCACCGTCTCCTCA |
| | | | SEQ ID NO: 25069 | SEQ ID NO: 29075 |
| | | AA | PYELTQPPSVSVSPGQTASITCSGDKLGDKYVYWYQQKSGQSPVLVIYQDSKRPSGIPERFSGSNSGNTAALTISGTQAMDEADYYCQAWDSSTVVFGGGTKLTVL | EVQLLESGGGLIQPGGSLRLSCAASGFTFSSYVMSWVRQAPGKGLEWVSTISVGGGTTYYADSVKGRFTISRDNSKNTLYLQMNSLRAEDTAVYYCAKWGRGNSYEYYYGMDVWGQGTTVTVSS |
| | | | SEQ ID NO: 25070 | SEQ ID NO: 29076 |

FIGURE 50

| | | | | |
|---|---|---|---|---|
| **iPS:398474** | **21-225_17B10** | NA | GACATCCAGATGACCCAGTCTCCATCCTCCCT ATCTGCATCTGTAGGAGACAGAGTCACCATCA CTTGCCGGTCAAGTCAGAGCATTAACAGCTAT TTAAATTGGTATCAGCAGAAACCTGGGAAAGC CCCTAAGCTCCTGATCTTTGCTGCATCCAGTTT GCACAGTGGGGTCCCATCAAGGTTCAGTGGCA GTGGATCTGGGACGGATTTCACTCTCACCATC AGCAGTCTGCAACCTGAAGATTTTGCAACTTA CTACTGTCAACAGGGTTACAATACCCCCACGT GGACGTTCGGCCAAGGGACCAAGGTGGAAAT CAAT | GAGGTGCAGCTGTTGGAGTCTGGGGGAGGCTTG GTACAGCCTGGGGGGTCCCTGAGACTCTCCTGTG CAGCCTCTGGATTCACCTTTAGAAGCTATGCCAT GAGCTGGGTCCGCCAGGCTCCAGGGAAGGGGCT GGAGTGGGTCTCAGTTATTAGTGGTAGTGGTGGT AACACATACTTCGCAGACTCCGTGAAGGGCCGGT TCACCATCTCCAGAGACAATTCCAAGAACACGCT GTATCTGCAAATGGACAGCCTGAGAGCCGAGGA CACGGCCGTATATTACTGTGCGAAAAGGGGTATA CCAGAGGCTGATGCTTTTGATATCTGGGGCCAAG GGACAATGGTCACTGTCTCTTCA |
| | | | SEQ ID NO: 25071 | SEQ ID NO: 29077 |
| | | AA | DIQMTQSPSSLSASVGDRVTITCRSSQSINSYLNW YQQKPGKAPKLLIFAASSLHSGVPSRFSGSGSGT DFTLTISSLQPEDFATYYCQQGYNTPTWTFGQGT KVEIN | EVQLLESGGGLVQPGGSLRLSCAASGFTFRSYAMS WVRQAPGKGLEWVSVISGSGGNTYFADSVKGRFTI SRDNSKNTLYLQMDSLRAEDTAVYYCAKRGIPEAD AFDIWGQGTMVTVSS |
| | | | SEQ ID NO: 25072 | SEQ ID NO: 29078 |
| **iPS:398476** | **21-225_17C1** | NA | GACATCCAGATGACCCAGTCTCCATCCTCCCT GTCTGCATCTGTAGGAGACAGAGTCACCATCA CTTGCCGGGCAAGTCAGAACATTAACGACTAT TTAAATTGGTATCAGCAGAAACCAGGGAAAG CCCCTAAGCTCCTGATCTATGCTGCATCCAATT TGCAAAGTGGGGTCCCAGCAAGGTTCAGTGGC AGTCGATCTGGGACAGATTTCACTCTCACCAT CAGCAGTCTGCAACCTGAAGATTTTGCAACTT ACTACTGTCAACAGACTTACAATACCCCTCCG GAGCGCAGTTTTGGCCAGGGGACCAAGCTGG AGATCAAA | GAGGTGCAGCTGTTGGAGTCTGGGGGAGGCTTG GAACAGCCGGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGTTATTAGTGGTAGTGGTGGT ACCACATTCTACGCAGACTCCGTGAAGGGCCGGT TCACCATCTCCAGAGACAATTCCAAGAACACGCT GTATCTGCAAATGAGCAGCCTGAGAGCCGAGGA CACGGCCGTATATTTCTGTGCCCGATATTGTAGT AGTACCAGGTGTCCTTATGATGCCTTTGATATCT GGGGCCAAGGGACAATGGTCACCGTCTCCTCA |
| | | | SEQ ID NO: 25073 | SEQ ID NO: 29079 |

FIGURE 50

| | | | | |
|---|---|---|---|---|
| | | AA | DIQMTQSPSSLSASVGDRVTITCRASQNINDYLNWYQQKPGKAPKLLIYAASNLQSGVPARFSGSRSGTDFTLTISSLQPEDFATYYCQQTYNTPPERSFGQGTKLEIK | EVQLLESGGGLEQPGGSLRLSCAASGFTFSSYAMSWVRQAPGKGLEWVSVISGSGGTTFYADSVKGRFTISRDNSKNTLYLQMSSLRAEDTAVYFCARYCSSTRCPYDAFDIWGQGTMVTVSS |
| | | | SEQ ID NO: 25074 | SEQ ID NO: 29080 |
| iPS:398478 | 21-225_17C10 | NA | GACATCCAGATGACCCAGTCTCCATCCTCCCAGTCTGCATCTGTAGGAGACAGAGTCACCATCACTTGCCGGGCGAGTCAGGGCATTAGCAATTATTTAGCCTGGTATCAGCAGAAACCAGGGAGAGTTCCTAAGCTCCTGATCTATGCTGCATCCACTTTGCAATCAGGGGTCCCATCTCGGTTCAGTGGCAGTGGATCTGGGACAGATTTCACTCTCACCATCAGCAGCCTGCAGCCTGACGATGTTGCAACTTATTACTGTCAAAAGTATAACAGTGCCCCTCCGCTCACCTTCGGCCAAGGGACACGACTGGAGATTAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTAGTTACATGTACTACGCAGACTCAGTGAAGGGCCGATTCACCATCTCCAGAGACAACGCCAAGAACTCACTGTATCTGCAAATGAACAGCCTGCGAGCCGAGGACACGGCTCTGTATTACTGTGCGAGAGATCGTGGGAGCTCCTGGGGCCAGGGAACCCTGGTCACCGTCTCCTCA |
| | | | SEQ ID NO: 25075 | SEQ ID NO: 29081 |
| | | AA | DIQMTQSPSSQSASVGDRVTITCRASQGISNYLAWYQQKPGRVPKLLIYAASTLQSGVPSRFSGSGSGTDFTLTISSLQPDDVATYYCQKYNSAPPLTFGQGTRLEIK | EVQLVESGGGLVKPGGSLRLSCAASGFTFSSYSMNWVRQAPGKGLEWVSSISGSSSYMYYADSVKGRFTISRDNAKNSLYLQMNSLRAEDTALYYCARDRGSSWGQGTLVTVSS |
| | | | SEQ ID NO: 25076 | SEQ ID NO: 29082 |

FIGURE 50

| | | | | |
|---|---|---|---|---|
| **iPS:398480** | **21-225_17G4** | NA | GACATCCAGATGACCCAGTCTCCATCCTCCCTGTCTGCATCTGCAGGAGACAGAGTCACCATCACTTGCCGGACAAGTCAGAATATTAGCAACTATTTAAATTGGTATCAGCAGAAACCAGGAAAAGCCCCTAAGCTCCTGATCTATGTTGCGTCCAGTTTCCCAAGTGGGGTCCCATCAAGGTTCAGTGGCAGTGGATCTGGGACAGAGTTCACTCTCACCATCAGCAGTCTGCAACCTGAAGATTTTGCAACTTACTACTGTCAACAGAGTAACTTTTTCCCGCTCACTTTCGGCGGAGGGACCAAGGTGGAGATCATA | CAGGTGCAGTTGGTGCAGTCTGGGGCTGAGGTGAAGAAGCCTGGGGCCTCAGTGAAGGTCTCCTGCAAGACTTCTGGATACACCTTCACCGACTATTATATGCACTGGGTGCGACAGGCCCCTGGACAAGGGCTTGAGTGGATGGGATGGATCAACCCTAACAGTGGTGGCACAAACTATGAACAGAAGTTTCAGGGCAGGGTCACCATGACCAGGGACACGTCCATCAGCACAGCCTACATGGAACTGAGTAGGCTGAGATCTGACGACACGGCCGTGTATTACTGTGCGAGTGGATACAGCTATGGGTACAACTGGTTCGACCCCTGGGGCCAGGGAACCCTGGTCACCGTCTCCTCA |
| | | | SEQ ID NO: 25077 | SEQ ID NO: 29083 |
| | | AA | DIQMTQSPSSLSASAGDRVTITCRTSQNISNYLNWYQQKPGKAPKLLIYVASSFPSGVPSRFSGSGSGTEFTLTISSLQPEDFATYYCQQSNFFPLTFGGGTKVEII | QVQLVQSGAEVKKPGASVKVSCKTSGYTFTDYYMHWVRQAPGQGLEWMGWINPNSGGTNYEQKFQGRVTMTRDTSISTAYMELSRLRSDDTAVYYCASGYSYGYNWFDPWGQGTLVTVSS |
| | | | SEQ ID NO: 25078 | SEQ ID NO: 29084 |
| **iPS:398482** | **21-225_17H6** | NA | GACATCCAGATGACCCAGTCTCCATCCTCACTGTCTGCATCTGTAGGAGACAGAGTCACCATCACTTGTCGGGCGAGTCGGGACATTAGCAATTATTTAGCCTGGTTTCAGCAGAAACCAGGGAAAGCCCCTAAGTCCCTGATCTCTACTGCATCCAGTTTGCAAAGTGGGGTCCCATCAAAGTTCAGTGGCAGTGGATCTGGGACAGATTTCACTCTCACCATCAGCAGCCTGCAGCCTGAAGATTTTGCAATTTATTACTGCCAACAGTATCATAGTTACCCATTCACTTTCGGCCCTGGGACCAAAGTGGATATCCAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAGGGGGCTGGAGTGGGTCTCATCCATTAGTGGTAGTAGTAGTTACATATACTACGCAGACTCAGTGAAGGGCCGATTCACCATCTTCAGAGACAACGCCAAGAACTCACTGTATCTGCAAATGAACAGCCTGAGAGCCGAGGACACGGCTGTGTATTACTGTGCGAGAGTGGCTTCATTTGACTACTGGGGCCAGGGAACCCTGGTCACCGTCTCCTCA |
| | | | SEQ ID NO: 25079 | SEQ ID NO: 29085 |

FIGURE 50

| | | | | |
|---|---|---|---|---|
| | | AA | DIQMTQSPSSLSASVGDRVTITCRASRDISNYLAWFQQKPGKAPKSLISTASSLQSGVPSKFSGSGSGTDFTLTISSLQPEDFAIYYCQQYHSYPFTFGPGTKVDIQ | EVQLVESGGGLVKPGGSLRLSCAASGFTFSSYSMNWVRQAPGRGLEWVSSISGSSSYIYYADSVKGRFTIFRDNAKNSLYLQMNSLRAEDTAVYYCARVASFDYWGQGTLVTVSS |
| | | | SEQ ID NO: 25080 | SEQ ID NO: 29086 |
| **iPS:398484** | **21-225_18D4** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGGAAAGTGGGGTCCCATCAAGGTTCAGCGGCAGTGGATCTGGGACAGAATTCACTCTCACAGTCAGCAGCCTGCAGCCTGAAGATTTTGCAACTTATTACTGTCTACATCATAATAATTACCTCCCCATCACCTTCGGCCAAGGGACACGACTGGAGATTAAA | CAGGTGCAACTGGTGCAGTCTGGGGCTGAGGTGAAGAAGCCTGGGGCCTCCGTGAAGGTCTCCTGCAAGGCTTCTGGATACACCTTCACCGGCTACTATTTGCACTGGGTGCGACAGGCCCCTGGACAAGGGCTTGAGTGGCTGGGATGGATCAACCCTAACAGTAATGGCACAATCTCTGCACAGAAGTTTCAGGGCAGGGTCACCATGACCAGGGACACGTCCATCAGCACAGCCTACATGGAGCTGAGCAGGCTGATATCTGACGACACGGCCGTATATTACTGTGCGAGAGATGGTACCAGCTCGCTTGACTACTGGGGCCAGGGAACCCTGGTCACCGTCTCCTCA |
| | | | SEQ ID NO: 25081 | SEQ ID NO: 29087 |
| | | AA | DIQMTQSPSSLSASVGDRVTITCRASQGIRNDLGWYQQKPGKAPKRLIYAASSLESGVPSRFSGSGSGTEFTLTVSSLQPEDFATYYCLHHNNYLPITFGQGTRLEIK | QVQLVQSGAEVKKPGASVKVSCKASGYTFTGYYLHWVRQAPGQGLEWLGWINPNSNGTISAQKFQGRVTMTRDTSISTAYMELSRLISDDTAVYYCARDGTSSLDYWGQGTLVTVSS |
| | | | SEQ ID NO: 25082 | SEQ ID NO: 29088 |

FIGURE 50

| | | | | |
|---|---|---|---|---|
| **iPS:398486** | **21-225_19A1** | NA | GACATCCAGATGACCCAGTCTCCATCCTCCCTGTCTGCATCTGTAGGAGACAGAGTCACCATCACTTGCCGGGCAAGTCATACCATTACCAGCTATTTAAATTGGTATCAGCAGAAACCAGGGAAAGCCCCTAAGTTCCTGATCTATGCTACATCCAATCTCCAAAGTGGGGTCCCATCAAGGTTCAGTGGCAGTGGATCTGGGACAGATTTCACTTTTACCATCAGCAGTCTGCAACCTGAAGATTTTGCAATTTACTACTGTCAACAGAGTTACAACTTCCCGCTCACTTTCGGCGGAGGGACCAAGGTGGAGATCAAA | CAGGTGCAACTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAATCCTAACAGTGGTGGCACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGTGGATACAGCTATGGGTACAACTGGTTCGACCCCTGGGGCCAGGGAACCCTGGTCACCGTCTCCTCA |
| | | | SEQ ID NO: 25083 | SEQ ID NO: 29089 |
| | | AA | DIQMTQSPSSLSASVGDRVTITCRASHTITSYLNWYQQKPGKAPKFLIYATSNLQSGVPSRFSGSGSGTDFTFTISSLQPEDFAIYYCQQSYNFPLTFGGGTKVEIK | QVQLVQSGAEVKKPGASVKVSCKASGYTFTGYYMHWVRQAPGQGLEWMGWINPNSGGTNYAQKFQGRVTMTRDTSISTAYMELSRLRSDDTAVYYCASGYSYGYNWFDPWGQGTLVTVSS |
| | | | SEQ ID NO: 25084 | SEQ ID NO: 29090 |
| **iPS:398488** | **21-225_19F6** | NA | TCCTATGAGCTGACTCAGCCACCCTCAGTGTCCGTGTCCCCAGGACAGACAGTCAGCATCACCTGCTCTGGAGATAAATTGGGGGATAAATATGCTTGCTGGTATCAGCAGAAGCCAGGCCAGTCCCCTGTGCTGGTCATCTATCAAGATAGCAAGCGGCCCTCAGGGACCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAACAACACTGTGGTATTCGGCGGAGGGACCAAGCTGACCGTCCTA | GAGGTGCAGCTGGTGGAGTCTGGGGGAGGCTTGGTGAAGCCTGGGGGGTCCCTTAGACTCTCCTGTGCAGCCTCTGGATTCACTTTCAGTAACGCCTGGATGAACTGGGTCCGCCAGGCTCCAGGGAAGGGGCTGGAGTGGGTTGGCCGTATTAAAAGCAAAACTGATGGTGGGACAACAGACTACGCTGCACCCGTGAAAGGCAGATTCACCATCTCAAGAGATGATTCAAAAAACACGCTGTATCTGCAAATGAACAGCCTGAAAACCGAGGACACAGCCGTGTATTACTGTACCACAGATACGGGTCCTATAGCAGCTCGTCTCGCTTACTACTACTATTACGCTATGGACGTCTGGGGCCACGGGACCACGGTCACCGTCTCCTCA |

FIGURE 50

| | | | | |
|---|---|---|---|---|
| | | | SEQ ID NO: 25085 | SEQ ID NO: 29091 |
| | | AA | SYELTQPPSVSVSPGQTVSITCSGDKLGDKYACWYQQKPGQSPVLVIYQDSKRPSGTPERFSGSNSGNTATLTISGTQAMDEADYYCQAWDNNTVVFGGGTKLTVL | EVQLVESGGGLVKPGGSLRLSCAASGFTFSNAWMNWVRQAPGKGLEWVGRIKSKTDGGTTDYAAPVKGRFTISRDDSKNTLYLQMNSLKTEDTAVYYCTTDTGPIAARLAYYYYYAMDVWGHGTTVTVSS |
| | | | SEQ ID NO: 25086 | SEQ ID NO: 29092 |
| iPS:398490 | 21-225_21D12 | NA | TCCTATGAGCTGACTCAGCCACCCTCAGTGTCCGTGTCCCCAGGACAGACAGCCAGCATCACCTGCTCTGGAGATAAATTGGGGAATAAATATGCTTACTGGTATCAGCAGAAGCCAGGCCAGTCCCCTGTGTTGGTCATCTATCAAGATAGAAAGAGGCCCTCAGGGATCCCTGAGCGATTCTCTGGCTCCAACTCTGGGAACACAGCCACTCTGACCATCAGCGGGACCCAGGCTATGGATGAGGCTGACTTTTACTGTCAGGCGTGGGACAACAGCACTGTGGTATTCGGCGGAGGGACCAGGCTGACCGTCCTA | CAGGTGCAGCTGGTGCAGTCTGGGGCTGAGGTGAAGAAACCTGGGGCCTCAGTGAAGGTCTCCTGCAAGGCTTCTGGATACACCTTCACCGACTACTATATTCACTGGGTGCGACAGGCCCCTGGACAAGGGCTTGAGTGGATGGGATGGATCAACCCTAACAGTGGTGGGACAAACAATGCACAGAAGTTTCAGGGCAGGGTCACCATGACCAGGGACACGTCCATCAGTACAGCCTACATGGAGCTGAGCAGGCTGAGATCTGACGATACGGCCGTGTATTCCTGTGCGAGGTCGTATTACTATGGTTCGGGGACTTATTATAACGAATTTGACTACTGGGGCCAGGGAACCCTGGTCACCGTCTCCTCA |
| | | | SEQ ID NO: 25087 | SEQ ID NO: 29093 |
| | | AA | SYELTQPPSVSVSPGQTASITCSGDKLGNKYAYWYQQKPGQSPVLVIYQDRKRPSGIPERFSGSNSGNTATLTISGTQAMDEADFYCQAWDNSTVVFGGGTRLTVL | QVQLVQSGAEVKKPGASVKVSCKASGYTFTDYYIHWVRQAPGQGLEWMGWINPNSGGTNNAQKFQGRVTMTRDTSISTAYMELSRLRSDDTAVYSCARSYYYGSGTYYNEFDYWGQGTLVTVSS |
| | | | SEQ ID NO: 25088 | SEQ ID NO: 29094 |

FIGURE 50

| | | | | |
|---|---|---|---|---|
| **iPS:398494** | **21-225_21H4** | NA | CAGTCTGCCCTGACTCAGCCTGCCTCCGTGTCTGGGTCTCCTGGACAGTCGATCACCATCTCCTGCACTGGAACCAGCAGTGACGTTGGTGGTTATAACTCTGTCTCCTGGTACCAACAGCACCCAGACAAAGCCCCCAAACTCATGATTTATGAGGTCAGTAATCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCAGCTCATATACAAGGAGCAGCACTGTGGTATTCGGCGGAGGGACCAAGCTGACCGTCCTA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTCTTAGTGGTCGTGGTGGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATGGGGACGTGGATACAGCTATGAGTACTACTACGGTATGGACGTCTGGGGCCAAGGGACCACGGTCACCGTCTCCTCA |
| | | | SEQ ID NO: 25089 | SEQ ID NO: 29095 |
| | | AA | QSALTQPASVSGSPGQSITISCTGTSSDVGGYNSVSWYQQHPDKAPKLMIYEVSNRPSGVSNRFSGSKSGNTASLTISGLQAEDEADYYCSSYTRSSTVVFGGGTKLTVL | EVQLLESGGGLVQPGGSLRLSCAASGFTFSSYAMSWVRQAPGKGLEWVSALSGRGGSTYYADSVKGRFTISRDNSKNTLYLQMNSLRAEDTAVYYCAKWGRGYSYEYYYGMDVWGQGTTVTVSS |
| | | | SEQ ID NO: 25090 | SEQ ID NO: 29096 |
| **iPS:398496** | **21-225_22D2** | NA | GACATCGTGATGACCCAGTCTCCAGACTCCCTGGCTGTGTCTCTGGGCGAGAGGGCCACCATCACCTGCAAGTCCAGCCAGAGTGTTTTACACAGCTCCAACAATAACAACTACTTAGCTTGGTACCAGCAGAAACCAGGACAGCCTCCTAAGCTACTCATTTACTGGGCATCTACCCGGAAATCCGGGGTCCCTGACCGATTCAGTGGCAGCGGGTCTGGGACAGATTTCACTCTCACCATCAGCAGCCTGCAGGCTGAAGATGTGGCAGTTTATTTCTGTCAGCAATATTATAGTACTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCATAAATTATGATATCAACTGGGTGCGACAGGCCACTGGACAAGGGCTTGAGTGGATGGGATGGATGCACCCTGACAGTGGTAACACAGGCTATGCACAGAAGTTCCAGGGCAGAGTCACCATGACCAGGAACACCTCCATAAGCACAGCCTACATGGAGCTGAGCAGTTTGAGATCTGAGGACACGGCCGTGTATTATTGTGCGTATAGTAGTGGCTGGTACTACTTTGACTACTGGGGCCAGGGAACCCTGGTCACCGTCTCCTCA |
| | | | SEQ ID NO: 25091 | SEQ ID NO: 29097 |

FIGURE 50

| | | | | |
|---|---|---|---|---|
| | | AA | DIVMTQSPDSLAVSLGERATITCKSSQSVLHSSNNNNYLAWYQQKPGQPPKLLIYWASTRKSGVPDRFSGSGSGTDFTLTISSLQAEDVAVYFCQQYYSTPCSFGQGTKLEIK | QVQLVQSGAEVKKPGASVKVSCKASGYTFINYDINWVRQATGQGLEWMGWMHPDSGNTGYAQKFQGRVTMTRNTSISTAYMELSSLRSEDTAVYYCAYSSGWYYFDYWGQGTLVTVSS |
| | | | SEQ ID NO: 25092 | SEQ ID NO: 29098 |
| iPS:398498 | 21-225_22E6 | NA | TCCTATGAGCTGACTCAGCCACCCTCAGTGTCCGTGTCCCCAGGACAGACAGCCAGCATCACCTGCTCTGGAGATAAATTGGGGGAGAAATATGCTTGCTGGTATCAGCAGAAGCCAGGCCAGTCCCCTGTGTTGGTCATCTATCAAGATAGAAAGCGGCCCTCAGGGATCCCTGAGCGATACTCTGGCTCCAACACTGGGAACACAGCCACTCTGACCATCAGCGGGACCCAGGCTATGGATGAGGCTGACTATTACTGTCAGGCGTGGGACAGCAGCACTGCGGTATTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGGTTCTCACTCAGCACTGGTGGAGTGGGTGTGGGCTGGATCCGTCAGCCCCCAGGAAAGGCCCTGGAGTGGCTTGCACTCATTTATTGGAATGATGATAAGCGCTACAGCCCATCTCTGAAGAGCAGGCTCACCATCACCAGGGACACCTCCAAAAACCAGGTGGTCCTTACAATGACCAACATGGACCCTGTGGACACAGCCACATATTACTGTGCACACACTATAGCAGTTCGTGGCTTTGACTACTGGGGCCAGGGAACCCTGGTCACCGTCTCCTCA |
| | | | SEQ ID NO: 25093 | SEQ ID NO: 29099 |
| | | AA | SYELTQPPSVSVSPGQTASITCSGDKLGEKYACWYQQKPGQSPVLVIYQDRKRPSGIPERYSGSNTGNTATLTISGTQAMDEADYYCQAWDSSTAVFGGGTKLTVL | QITLKESGPTLVKPTQTLTLTCTFSGFSLSTGGVGVGWIRQPPGKALEWLALIYWNDDKRYSPSLKSRLTITRDTSKNQVVLTMTNMDPVDTATYYCAHTIAVRGFDYWGQGTLVTVSS |
| | | | SEQ ID NO: 25094 | SEQ ID NO: 29100 |

FIGURE 50

| | | | | |
|---|---|---|---|---|
| iPS:398500 | 21-225_23A11 | NA | GACATCCAGATGACCCAGTCTCCATCCTCACTGTCTACATCTGTAGGAGACAGAGTCACCATCACTTGTCGGGCGAGTCAGGACATTAGCAATTATTTAGCCTGGTTTCAGCAGAAACCAGGGAAAGCCCCTAAGCGCCTGATCTATGCTGCATCCACTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATAATAGTTACCCATTCACTTTCGGCCCTGGGACCAAAGTGGATCTCAAG | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGTTATAGCATGAACTGGATCCGCCAGGCTCCAGGGAAGGGGCTGGAGTGGGTCTCATCCATTAGTGGTAGTAGTACTTACATATACTACGCAGACTCAGTGAAGGGCCGAATCGCCATCTCCAGAGACAACGCCAAGAACTCACTGTATCTGCAAATGAACAGCCTGAGAGCCGAGGACACGGCTGTGTATTACTGTGCGAGAGTGGCTTCATTTGACTACTGGGGCCAGGGAACCCTGGTCACCGTCTCCTCA |
| | | | SEQ ID NO: 25095 | SEQ ID NO: 29101 |
| | | AA | DIQMTQSPSSLSTSVGDRVTITCRASQDISNYLAWFQQKPGKAPKRLIYAASTLQSGVPSKFSGSGSGTDFTLTISSLQPEDFATYYCQQYNSYPFTFGPGTKVDLK | EVQLVESGGGLVKPGGSLRLSCAASGFTFSSYSMNWIRQAPGKGLEWVSSISGSSTYIYYADSVKGRIAISRDNAKNSLYLQMNSLRAEDTAVYYCARVASFDYWGQGTLVTVSS |
| | | | SEQ ID NO: 25096 | SEQ ID NO: 29102 |
| iPS:398502 | 21-225_23B11 | NA | GACATCCAGATGACCCAGTCTCCATCTTCCGTGTCTGCATCTGTAGGAGACAGAGTCACCATCACTTGTCGGGCGAGTCAGGGTATTACCAAGTGGTTAGCCTGGTATCAGCAGAAACCAGGGAAAGCCCCTAAAGTCCTGATCTATGCTGCATCCAGTTTGCAAAGTCGGGTCCCATCAAGGTTCAGCGGCAGTAGATCTGGGACAGATTTCACTCTCACCATCAGCAGCCTGCAGCCTGAAGATTTTGCAACTTACTATTGTCAACAGGCTAACAGTTTCCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATCTGCACTGGGTGCGACAGGCCCCTGGACAAGGGCTTGAGTGGATGGGATGGATCAACCCTAACAATAATGGCACAAACTATGCACAGAAGTTTCAGGGCAGGGTCACCATGACCAGGGACACGTCCATCAGCACAGCCTACATGGAGCTGAGCCGGCTGAGATCTGACGACACGGCCGTGTATTACTGTGCGAGAGATGGTACCAGCTCGTTTGACTACTGGGGCCAGGGAACCCTGGTCACCGTCTCCTCA |
| | | | SEQ ID NO: 25097 | SEQ ID NO: 29103 |

FIGURE 50

| | | | | |
|---|---|---|---|---|
| | | AA | DIQMTQSPSSVSASVGDRVTITCRASQGITKWLAWYQQKPGKAPKVLIYAASSLQSRVPSRFSGSRSGTDFTLTISSLQPEDFATYYCQQANSFPFTFGPGTKVDIK | QVQLVQSGAEVKKPGASVKVSCKASGYTFTGYYLHWVRQAPGQGLEWMGWINPNNNGTNYAQKFQGRVTMTRDTSISTAYMELSRLRSDDTAVYYCARDGTSSFDYWGQGTLVTVSS |
| | | | SEQ ID NO: 25098 | SEQ ID NO: 29104 |
| **iPS:398504** | **21-225_23D7** | NA | TCCTATGAACTGACTCAGCCACCCTCAGTGTCCGTGTCCCCAGGACAGACAGCCAGCATCACCTGCTCTGGAGAAAAATTGGGGGATAAATATGTTTGTTGGTATCAGCAGAAGCCAGGCCAGTCCCCTGTGGTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGAACAGCAGCAATGTGGTATTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGGTTCTCACTCAGCACTAGTGGAGTGGGTGTGGGCTGGATCCGTCAGCCCCCAGGAAAGGCCCTGGAGTGCCTTTCACTCATTTATTGGAATAATGATAAGGTCTACAGCCCATCTCTGAAGAGCAGGCTCACCATCACCAAGTACACCTCCAAAAACCAGGTGGTCCTTACAATGTCCAACATGGACCCTGTGGACACAGCCACATATTACTGTGCACACAGGGGACAGCAGCTGGCCCTCGACTACTGGGGCCAGGGAACCCTGGTCACCGTCTCCTCA |
| | | | SEQ ID NO: 25099 | SEQ ID NO: 29105 |
| | | AA | SYELTQPPSVSVSPGQTASITCSGEKLGDKYVCWYQQKPGQSPVVVIYQDSKRPSGIPERFSGSNSGNTATLTISGTQAMDEADYYCQAWNSSNVVFGGGTKLTVL | QITLKESGPTLVKPTQTLTLTCTFSGFSLSTSGVGVGWIRQPPGKALECLSLIYWNNDKVYSPSLKSRLTITKYTSKNQVVLTMSNMDPVDTATYYCAHRGQQLALDYWGQGTLVTVSS |
| | | | SEQ ID NO: 25100 | SEQ ID NO: 29106 |

FIGURE 50

| | | | | |
|---|---|---|---|---|
| **iPS:398506** | **21-225_23G12** | NA | GACATCGTGATGACCCAGTCTCCAGACTCCCT<br>GGCTGTGTCTCTGGGCGAGAGGGCCACCATCA<br>ACTGCAAGTCCAGCCAGAGTATTTTATTCAGC<br>TCCAACAATAACAACTACTTAGCTTGGTACCA<br>GCAGAAACCAGGACAACCTCCTAAGTTGCTCA<br>TTTACTGGGCATCTACCCGGGAATCCGGGGTC<br>CCTGACCGATTCAGTGGCAGCGGGTCTGGGAC<br>AGATTTCACTCTCACCATCAGCAGCCTGCAGG<br>CTGAAGATGTGGCAGTTTATTTCTGTCAGCAA<br>TATTCTAGTACTCCGTGGACGTTCGGCCAAGG<br>GACCAAGGTGGAAATCAAA | CAGGTGCAGCTGCTGCAGTCTGGGGCTGAGGTG<br>AAGAAGCCTGGGGCCTCAGTGAAGGTCTCCTGC<br>AAGGCTTCTGGATACACCTTCACCAATTATGATA<br>TCAACTGGGTGCGACAGGCCACTGGACAAGGGC<br>TTGAGTGGATGGGATGGATGTACCCTAACAGTGG<br>TAACACGGGCTATGCACAGAAGTTCCAGGGCAG<br>AGTCACCATGACCATGAACACCTCCATAAGCACA<br>GCCTACATGGAGTTGAGCAGCCTGAGATCTGAG<br>GACACGGCCGTGTATTACTGTGCGATTAGCGGTG<br>GCTGGTACTACTTTGACTACTGGGGCCAGGGAAC<br>CCTGGTCACCGTCTCCTCT |
| | | | SEQ ID NO: 25101 | SEQ ID NO: 29107 |
| | | AA | DIVMTQSPDSLAVSLGERATINCKSSQSILFSSNN<br>NNYLAWYQQKPGQPPKLLIYWASTRESGVPDRF<br>SGSGSGTDFTLTISSLQAEDVAVYFCQQYSSTPW<br>TFGQGTKVEIK | QVQLLQSGAEVKKPGASVKVSCKASGYTFTNYDIN<br>WVRQATGQGLEWMGWMYPNSGNTGYAQKFQGR<br>VTMTMNTSISTAYMELSSLRSEDTAVYYCAISGGW<br>YYFDYWGQGTLVTVSS |
| | | | SEQ ID NO: 25102 | SEQ ID NO: 29108 |
| **iPS:398508** | **21-225_24B1** | NA | GATGTTGTGATGACTCAGTCTCCACTCTCCCTG<br>CCCGTCACCCTTGGACAGCCGGCCTCCATCTC<br>CTGCAGGTCTAGTCAAAGCCTCGTATACAGTG<br>ATGGAAACACCTACTTGAATTGGTTTCAGCAG<br>AGGCCAGGCCAATCTCCAAGGCGCCTAATTTA<br>TAAGGTTTCTAACTGGGACTCTGGGGTCCCAG<br>ACAGATTCAGCGGCAGTGGGTCAGGCACTGAT<br>TTCACACTGAAAATCAGCTGGGTGGAGGCTGA<br>GGATGTTGGGGTTTGTTACTGCATGCAAGGTG<br>CACACTGGCCTCCGATCACCTTCGGCCAAGGG<br>ACACGACTGGAGATTAAA | GAGGTGCAGCTGTTGGAGTCTGGGGGAGGCTTG<br>GTACAGCCTGGGGGGTCCCTGAGACTCTCCTGTG<br>CAGCCTCTGGATTCACCTTTAGCAGCTATGCCAT<br>GAGCTGGGTCCGCCAGGCTCCAGGGAAGGGGCT<br>GGAGTGGGTCTCAGCTATTAGTGGTCGTGGTGGT<br>AGCACATACTACGCAGACTCCGTGAAGGGCCGG<br>TTCACCATCTCCAGAGACAATTCCAAGAACACGC<br>TGTATCTGCAAATGAACAGCCTGAGAGCCGAGG<br>ACACGGCCGTATATTACTGTGCGAAATGGGGAC<br>GTGGATACAGCTATGAGTACTACTACGGTATGGA<br>CGTCTGGGGCCAGGGGACCACGGTCACCGTCTCC<br>TCA |
| | | | SEQ ID NO: 25103 | SEQ ID NO: 29109 |

FIGURE 50

| | | | | |
|---|---|---|---|---|
| | | AA | DVVMTQSPLSLPVTLGQPASISCRSSQSLVYSDGNTYLNWFQQRPGQSPRRLIYKVSNWDSGVPDRFSGSGSGTDFTLKISWVEAEDVGVCYCMQGAHWPPITFGQGTRLEIK | EVQLLESGGGLVQPGGSLRLSCAASGFTFSSYAMSWVRQAPGKGLEWVSAISGRGGSTYYADSVKGRFTISRDNSKNTLYLQMNSLRAEDTAVYYCAKWGRGYSYEYYYGMDVWGQGTTVTVSS |
| | | | SEQ ID NO: 25104 | SEQ ID NO: 29110 |
| iPS:398510 | 21-225_25A3 | NA | GACATCGTGATGACCCAGTCTCCAGACTCCCTGACTGTGTCTCTGGGCGAGAGGGCCACCATCAACTGCAAGTCCAGCCAGAGTGTTTTATACAGCTCCAACAATAAGAACTACTTAGCTTGGTACCAGCAGAAACCTGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCGTGCAGTTTTGGCCAGGGGACCAGGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACATCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCTGGAACACCTCCATAAGCACAGCCAACATGGAGCTGAGCAGCCTGAGATCTGAGGACACGGCCGTGTATTACTGTGCGAGTAGCAGTGGCTGGTATTGGTTCGACCCCTGGGGCCAGGGAACCCTGGTCACCGTCTCCTCA |
| | | | SEQ ID NO: 25105 | SEQ ID NO: 29111 |
| | | AA | DIVMTQSPDSLTVSLGERATINCKSSQSVLYSSNNKNYLAWYQQKPGQPPKLLIYWASTRESGVPDRFSGSGSGTDFTLTISSLQAEDVAVYYCQQYYSTPCSFGQGTRLEIK | QVQLVQSGAEVKKPGASVKVSCKASGYIFTNYDINWVRQATGQGLEWMGWMHPNSGNTGYAQKFQGRVTMTWNTSISTANMELSSLRSEDTAVYYCASSSGWYWFDPWGQGTLVTVSS |
| | | | SEQ ID NO: 25106 | SEQ ID NO: 29112 |

FIGURE 50

| | | | | |
|---|---|---|---|---|
| **iPS:398512** | **21-225_25E12** | NA | GACATCGTGCTGACCCAGTCTCCAGACTTCCTGGCTATGTCTCTGGGCGAGAGGGCCACCATCAACTGCAAGTCCAGCCAGAGTGTTTTATACCACTCCAACAATTACAACTACTTAGCTTGGTACCAGCAGAAACCAAAACAGCCTCCTAAGCTGCTCATTTACTGGGCATCTACCCGGGAGTCCGGGGTCCCTGACCGATTCAGTGGCAGCGGGTCTGGGACATATTTCACTCTCACCATCAGCAGCCTCCAGGCTGAAGATGTGGCAGTTTATTACTGTCAGCAATATTACAGTACTCCGTGCAGTTTTGGCCAGGGGACCAACCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACCTGGAGCTGAGCAGCCTGAGATCTGAGGACACGGCCGTGTATTACTGTGCGGGGAGCAATGGCTGGTACTACTTTGACTACTGGGGCCAGGGAACCCTGGTCACCGTCTCCTCA |
| | | | SEQ ID NO: 25107 | SEQ ID NO: 29113 |
| | | AA | DIVLTQSPDFLAMSLGERATINCKSSQSVLYHSNNYNYLAWYQQKPKQPPKLLIYWASTRESGVPDRFSGSGSGTYFTLTISSLQAEDVAVYYCQQYYSTPCSFGQGTNLEIK | QVQLVQSGAEVKKPGASVKVSCKASGYTFTNYDINWVRQATGQGLEWMGWMNPNSGNTGYAQKFQGRVTMTRNTSISTAYLELSSLRSEDTAVYYCAGSNGWYYFDYWGQGTLVTVSS |
| | | | SEQ ID NO: 25108 | SEQ ID NO: 29114 |
| **iPS:398516** | **21-225_26A9** | NA | GACATCGTGATGACCCAGTCTCCAGACTCCCTGGCTGTGTCTCTGGGCGAGAGGGCCACCATCAACTGCAAGTCCAGCCAGAGTGTTTTATACAGCTCCAACAATAAGAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGACTTCACTCTCACTATCAGCAGCCTGCAGGCTGAAGATGTGGCAGTTTATTACTGTCAGCAATATTATAGTAGTCCGTGCAGTTTTGGCCAGGGGACCAGGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAATGGATGGGATGGATGCACCCTAACAGTGGTAACACAGGCTGTGCACAGAAGTTCCAGGGCAGAGTCACCATGACCTGGAACATGTCCATAAGCACAGCCTACATGGAGCTGAGCAGCCTGAGATCTGAGGACACGGCCGTATATTACTGTGCGAGTAGCAGTGGCTGGTACTGGTTCGACCCCTGGGGCCAGGGAACCCTGGTCACCGTCTCCTCA |
| | | | SEQ ID NO: 25109 | SEQ ID NO: 29115 |

FIGURE 50

| | | | | |
|---|---|---|---|---|
| | | AA | DIVMTQSPDSLAVSLGERATINCKSSQSVLYSSNNKNYLAWYQQKPGQPPKLLIYWASTRESGVPDRFSGSGSGTDFTLTISSLQAEDVAVYYCQQYYSSPCSFGQGTRLEIK | QVQLVQSGAEVKKPGASVKVSCKASGYTFTNYDINWVRQATGQGLEWMGWMHPNSGNTGCAQKFQGRVTMTWNMSISTAYMELSSLRSEDTAVYYCASSSGWYWFDPWGQGTLVTVSS |
| | | | SEQ ID NO: 25110 | SEQ ID NO: 29116 |
| iPS:398520 | 21-225_31C4 | NA | GACATCCAGATGACCCAGTCTCCATCTTCCGTGTCTGCATCTGTAGGAGACAGAGTCACCATCACTTGTCGGGCGAGTCAGGGTATTAGCAAATGGTTAGCCTGGTATCAGCAGAAACCAGGGAAAGCCCCTAAACCCCTGATCTATGCTGCATCCAGTTTGCAGAGTGGGGTCCCAACAAGGTTCAGCGGCAGTGGATCTGGGACAGATTTCACTCTCACCATCAGCAGCCTGCAGCCTGAAGATTTTGCAATTTACTATTGTCAACAGGCTAACAGTTTCCCATTCACTTTCGGCCCTGGGACCAAAGTGGATATCAAA | CAGGTGCAGTTGGTGCAGTCTGGGACTGAGGTGAAGAAGCCTGGGGCCTCAGTGAAGGTCTCCTGCAAGGCTTCTGGATACACCTTCACCGGCGATTATATGCACTGGGTGCGACAGGCCCCTGGACAAGGGCTTGAGTGGATGGGATGGATCAGCCCTAAAAATGGTGGCACAAACTATGCACAGAAGTTTCAGGGCAGGGTCACCATGACCAGGGACACGTCCATCAGCACAGTCTACATGGAGCTGAACAGGCTGAGATCTGACGACACGGCCGTGTATTACTGTGCGAGAGATGGAACTGGGTCCTTTGACTACTGGGGCCAGGGAACCCTAGTCACCGTCTCCTCA |
| | | | SEQ ID NO: 25111 | SEQ ID NO: 29117 |
| | | AA | DIQMTQSPSSVSASVGDRVTITCRASQGISKWLAWYQQKPGKAPKPLIYAASSLQSGVPTRFSGSGSGTDFTLTISSLQPEDFAIYYCQQANSFPFTFGPGTKVDIK | QVQLVQSGTEVKKPGASVKVSCKASGYTFTGDYMHWVRQAPGQGLEWMGWISPKNGGTNYAQKFQGRVTMTRDTSISTVYMELNRLRSDDTAVYYCARDGTGSFDYWGQGTLVTVSS |
| | | | SEQ ID NO: 25112 | SEQ ID NO: 29118 |

FIGURE 50

| | | | | |
|---|---|---|---|---|
| **iPS:398522** | **21-225_32A1** | NA | GACATCGTGATGACCCAGTCTCCAGACTCCCT<br>GGCTGTGTCTCTGGGCGAGAGGGCCACCATCA<br>ACTGCAAGTCCAGCCAGAGTGTTTTATACAGC<br>TCCAACAATTACAACTACTTAGCTTGGTACCA<br>GCTGAAACCAGGACAGCCTCCTAAGCTGCTCA<br>TTTACTGGGCATCTACCCGGAAATCCGGGGTC<br>CCTGACCGATTCAGTGGCAGCGGGTCTGGGAC<br>AGATTTCACTCTCACCATCAGCAGCCTGCAGA<br>CTGAAGATGTGGCACTTTATTACTGTCAACAA<br>TATTATACTTCTCCGTGCAGTTTTGGCCAGGGG<br>ACCAAGCTGGAGATCAAA | CAGGTGCAGCTGGTGCAGTCTGGGGCTGAGGTG<br>AAGAAGCCTGGGGCCTCAGTGAAGGTCTCCTGC<br>AAGGCTTCTGGATACACCTTCACCAACTATGATA<br>TTAACTGGGTGCGACAGGCCACTGGACAAGGGC<br>TTGAGTGGATGGGATGGATGAACCCTAACAGTG<br>GTAACACAGGCTATGCACAGAAGTTCCAGGGCC<br>GAGTCACCATGACCAGGAACACCTCCATAAGCA<br>CAGCCTACATGGAGCTGAGCAGCCTGAGATCTG<br>AGGACACGGCCGTGTATTACTGTGCGAGCAGCA<br>GTGGCTGGTACTTTTTGACTACTGGGGCCAGGG<br>AACCCTGGTCACCGTCTCCTCA |
| | | | SEQ ID NO: 25113 | SEQ ID NO: 29119 |
| | | AA | DIVMTQSPDSLAVSLGERATINCKSSQSVLYSSN<br>NYNYLAWYQLKPGQPPKLLIYWASTRKSGVPD<br>RFSGSGSGTDFTLTISSLQTEDVALYYCQQYYTS<br>PCSFGQGTKLEIK | QVQLVQSGAEVKKPGASVKVSCKASGYTFTNYDIN<br>WVRQATGQGLEWMGWMNPNSGNTGYAQKFQGR<br>VTMTRNTSISTAYMELSSLRSEDTAVYYCASSSGW<br>YFFDYWGQGTLVTVSS |
| | | | SEQ ID NO: 25114 | SEQ ID NO: 29120 |
| **iPS:398524** | **21-225_32A5** | NA | GACATCGTGATGACCCAGTCGCCAGACTCCCT<br>GGCTGTGTCTCTGGGCGAGAGGGCCACCATCA<br>ACTGCAAGTCCAGCCAGAGTGTTTTACACAGC<br>TCCAACAATAAGAACTACTTAGCTTGGTATCA<br>GCAGAAACCAGGACAGCCTCCTAAGCTGCTCA<br>TTTACTGGGCATCTACCCGGGAATCCGGGGTC<br>CCTGACCGCTTCAGTGGCAGCGGGTCTGGGAC<br>AGATTTCACTCTCGCCATCAGCAGCCTGCAGG<br>CTGAAGATGTGGCACTTTATCACTGTCAGCAA<br>TATTATAGTTCTCCGTGCAGTTTTGGCCAGGG<br>GACCGGGCTGGAGATCAAA | CAGGTGCAGCTGGTGCAGTCTGGGGCTGAGGTG<br>AAGAAGCCTGGGGCCTCAGTGAAGGTCTCCTGC<br>AAGGCTTCTGGATACACCTTCACCAATTATGACA<br>TCAACTGGGTGCGACAGGCCACTGGACAAGGGC<br>TTGAGTGGATGGGATGGATGCACCCTAACAGTGG<br>TAACACAGGCTTTGCACAGAAGTTCCGGGGCAG<br>AGTCACCATGACCAGAAACACCTCCATAAGCAC<br>AGCCTACATGGAACTGAGCAGCCTGAGATCTGA<br>GGACACGGCCGTGTATTACTGTTCGAGCAGCAGT<br>GGCTGGTACTTTTTGACTACTGGGGCCAGGGAA<br>CCCTGGTCACCGTCTCCTCA |
| | | | SEQ ID NO: 25115 | SEQ ID NO: 29121 |

FIGURE 50

| | | | | |
|---|---|---|---|---|
| | | AA | DIVMTQSPDSLAVSLGERATINCKSSQSVLHSSNNKNYLAWYQQKPGQPPKLLIYWASTRESGVPDRFSGSGSGTDFTLAISSLQAEDVALYHCQQYYSSPCSFGQGTGLEIK | QVQLVQSGAEVKKPGASVKVSCKASGYTFTNYDINWVRQATGQGLEWMGWMHPNSGNTGFAQKFRGRVTMTRNTSISTAYMELSSLRSEDTAVYYCSSSSGWYFFDYWGQGTLVTVSS |
| | | | SEQ ID NO: 25116 | SEQ ID NO: 29122 |
| iPS:398526 | 21-225_32B3 | NA | GACATCCAGATGACCCAGTCTCCATCCTCACTGTCTGCATCTATAGGAGACAGAGTCACCATCACTTGTCGGGCGAGTCAGGGCATTAGCAATTATTTAGCCTGGTTTCAGCAGAAACCAGGGAAAGCCCCTAGGTCCCTGATCTATGCTGCATCCAGTTTACAAAGTGGGGTCCCATCAACGTTCAGCGGCAGTGGATCTGGGACAGATTTCACTCTCACCATCAGCAGCCTGCAGCCTGAAGATTTTGCAACTTATTACTGCCAACAGTATAATAGTTATCCATTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTACTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGTGGCTGGCTTTGACTACTGGGGCCAGGGAACCCTGGTCACCGTCTCCTCA |
| | | | SEQ ID NO: 25117 | SEQ ID NO: 29123 |
| | | AA | DIQMTQSPSSLSASIGDRVTITCRASQGISNYLAWFQQKPGKAPRSLIYAASSLQSGVPSTFSGSGSGTDFTLTISSLQPEDFATYYCQQYNSYPFTFGPGTKVDIK | EVQLVESGGGLVKPGGSLRLSCAASGFTFSSYSMNWVRQAPGKGLEWVSSISGSSTYIYYADSVKGRFTISRDNAKNSLYLQMNSLRAEDTAVYYCARVAGFDYWGQGTLVTVSS |
| | | | SEQ ID NO: 25118 | SEQ ID NO: 29124 |

FIGURE 50

| | | | | |
|---|---|---|---|---|
| **iPS:398528** | **21-225_32G1** | NA | GACATCCAGATGACCCAGTCTCCATCCTCCCTGTCTGCATCTGTAGGAGACAGAGTCACCATCACTTGCCGGGCAAGTCAGGACATGAGAAGTGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATACTATTTCCCCTCCTACTTTCGGCGGAGGGACCAAGGTGGAGATCAAA | GAGGTGCAGCTGTTGGAGTCTGGGGGAGGCTTGGCACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCCGCTATTAGTGGTCGTGGTGGTAGCACATTCCACGCAGACTCCGTGAAGGGCCGGTTCACGATCTCCAGAGACAATTCCAAGAACACGCTGTATCTGCAAATGAACAGCCTGAGAGCCGAGGACACGGCCGTATATTACTGTGTGAAAGGGGAGCTACTAGAGGACTACTACTTCTACGGTATGGACGTCTGGGGCCAAGGGACCACGGTCACCGTCTCCTCA |
| | | | SEQ ID NO: 25119 | SEQ ID NO: 29125 |
| | | AA | DIQMTQSPSSLSASVGDRVTITCRASQDMRSDLGWYQQKPGKAPKRLIYAASSLQSGVPSRFSGSGSGTEFTLTISSLQPEDFATYYCLQHTISPPTFGGGTKVEIK | EVQLLESGGGLAQPGGSLRLSCAASGFTFSSYAMSWVRQAPGKGLEWVSAISGRGGSTFHADSVKGRFTISRDNSKNTLYLQMNSLRAEDTAVYYCVKGELLEDYYFYGMDVWGQGTTVTVSS |
| | | | SEQ ID NO: 25120 | SEQ ID NO: 29126 |
| **iPS:398530** | **21-225_32G4** | NA | GATGTTGTGATGACTCAGTCTCCACTCTCCCTGTCCGTCACCCTTGGACAGCCGGCCTCCATCTCCTGCAGGTCAAGTCAAAGCCTCGTATACAGTGATGGAAACACCTACTTGAATTGGTTTCAGCAGAGGCCAGGCCAATCTCCAAGGCGCCTAATTTATAAGGTTTCTAACTGGGACTCTGGGGTCCCAGACAGATTCAGCGGCAGTGGGTCAGGCACAGATTTCACACTGAAAATCAGCAGGGTGGAGGCTGAGGATGTTGGGGTTTATTACTGCATGCAAGGTATACACTGGCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAGTTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGCGAAAGAAGGCTAACGACTACTGGGGCCAGGGAACCCTGGTCACCGTCTCCTCA |
| | | | SEQ ID NO: 25121 | SEQ ID NO: 29127 |

FIGURE 50

| | | | | |
|---|---|---|---|---|
| | | AA | DVVMTQSPLSLSVTLGQPASISCRSSQSLVYSDGNTYLNWFQQRPGQSPRRLIYKVSNWDSGVPDRFSGSGSGTDFTLKISRVEAEDVGVYYCMQGIHWLTFGGGTKVEIK | QVQLVQSGAEVKKPGASVKVSCKASGYTFTSYDINWVRQATGQGLEWMGWMNPNSGNTGYAQKFQGRVTMTRNTSISTAYMELSSLRSEDTAVYYCARKKANDYWGQGTLVTVSS |
| | | | SEQ ID NO: 25122 | SEQ ID NO: 29128 |
| iPS:398532 | 21-225_33B7 | NA | GACATCCAGATGACCCAGTCTCCATCCTCACTGTCTGCATCTGTAGGAGACAGAGTCACCATCACTTGTCGGGCGAGTCAGGACATTAGCAATTATTTGGCCTGGTTTCAGCAGAAACCAGGGAAAGCCCCTACGTCCCTGATCTATGCTGCATCTAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTTTTACTGCCAACAGTATCATAGTTACCCGCTCACCTTCGGCCAAGGGACACGACTGGAAATTAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAACATGAACTGGGTCCGCCAGGCTCCAGGGAAGGGGCTGGAGTGGGTCTCATCCATTAGTGGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGGTTAAATGGTTTTGACTACTGGGGCCAGGGAACCCTGGTCACCGTCTCCTCA |
| | | | SEQ ID NO: 25123 | SEQ ID NO: 29129 |
| | | AA | DIQMTQSPSSLSASVGDRVTITCRASQDISNYLAWFQQKPGKAPTSLIYAASSLQSGVPSKFSGSGSGTDFTLTISSLQPEDFATFYCQQYHSYPLTFGQGTRLEIK | EVQLVESGGGLVKPGGSLRLSCAASGFTFSSYNMNWVRQAPGKGLEWVSSISGSSSYIYYADSVKGRFTISRDNAKNSLYLQMNSLRAEDTAVYYCARLNGFDYWGQGTLVTVSS |
| | | | SEQ ID NO: 25124 | SEQ ID NO: 29130 |

FIGURE 50

| | | | | |
|---|---|---|---|---|
| **iPS:398534** | **21-225_33B8** | NA | GACATCCAGATGACCCAGTCTCCATCCTCCCTGTCTGCATCTGTAGGAGACAGAGTCACCATCACTTGCCGGGCAAGTCAGGACATTAGAAGTGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTTCTGTCTACAGCATACTATTTACCCTCCTACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCCGCTATTAGTGGTCGTGGTGGTAGCACATTCCACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGTGAAAGGGGAGCTACTAGAGGACTACTACTTCTACGGTATGGACGTCTGGGGCCAAGGGACCACGGTCACCGTCTCCTCA |
| | | | SEQ ID NO: 25125 | SEQ ID NO: 29131 |
| | | AA | DIQMTQSPSSLSASVGDRVTITCRASQDIRSDLGWYQQKPGKAPKRLIYAASSLQSGVPSRFSGSGSGTEFTLTISSLQPEDFATYFCLQHTIYPPTFGGGTKVEIK | EVQLLESGGGLVQPGGSLRLSCAASGFTFSSYAMSWVRQAPGKGLEWVSAISGRGGSTFHADSVKGRFTISRDNSKNTLYLQMNSLRAEDTAVYYCVKGELLEDYYFYGMDVWGQGTTVTVSS |
| | | | SEQ ID NO: 25126 | SEQ ID NO: 29132 |
| **iPS:398536** | **21-225_33D12** | NA | GACGTCCAGATGACCCAGTCTCCATCCTCCCTGTCTGCATCTTTAGGAGACAGAGTCACCATCACTTGCCGGGCAAGTCAGAGCATTAGAAGCTATTTAAATTGGTATCAGCAGAAACCAGGGAAAGCCCCTAACCTCCTGATCTATAGTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAAATTACTACTGTCAACAGAGTTACAGTATCCCGATCACCTTCGGCCAAGGGACACGACTGGAGATTAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAGTTATGATATCAGCTGGGTGCGACTGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGAGAAAGAGGGCTAACGACTACTGGGGCCAGGGAACCCTGGTCACCGTCTCCTCA |
| | | | SEQ ID NO: 25127 | SEQ ID NO: 29133 |

FIGURE 50

| | | | | |
|---|---|---|---|---|
| | | AA | DVQMTQSPSSLSASLGDRVTITCRASQSIRSYLNWYQQKPGKAPNLLIYSASSLQSGVPSRFSGSGSGTDFTLTISSLQPEDFANYYCQQSYSIPITFGQGTRLEIK | QVQLVQSGAEVKKPGASVKVSCKASGYTFTSYDISWVRLATGQGLEWMGWMNPNSGNTGYAQKFQGRVTMTRNTSISTAYMELSSLRSEDTAVYYCARKRANDYWGQGTLVTVSS |
| | | | SEQ ID NO: 25128 | SEQ ID NO: 29134 |
| **iPS:398538** | **21-225_34H7** | NA | GACATCGTGATGACCCAGTCTCCAGACTCCCTGGCTGTGTCTCTGGGCGAGAGGGCCACCATCAACTGCAAGTCCAGCCAGAGTGTTTTATACAGCTCCAACAATTACAACTACTTAGCTTGGTACCAGCTGAAACCAGGACAGCCTCCTAAGCTGCTCATTTACTGGGCATCTACCCGGAAATCCGGGGTCCCTGACCGATTCAGTGGCAGCGGGTCTGGGACAGATTTCACTCTCACCATCAGCAGCCTGCAGACTGAAGATGTGGCACTTTATTACTGTCAGCAATATTATACTTCTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAACTATGATATTAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAACAGCCTGAGATCTGAGGACACGGCCGTGTATTACTGTGCGAGCAGCAGTGGCTGGTACTTTTTTGACTACTGGGGCCAGGGAACCCTGGTCACCGTCTCCTCA |
| | | | SEQ ID NO: 25129 | SEQ ID NO: 29135 |
| | | AA | DIVMTQSPDSLAVSLGERATINCKSSQSVLYSSNNYNYLAWYQLKPGQPPKLLIYWASTRKSGVPDRFSGSGSGTDFTLTISSLQTEDVALYYCQQYYTSPCSFGQGTKLEIK | QVQLVQSGAEVKKPGASVKVSCKASGYTFTNYDINWVRQATGQGLEWMGWMNPNSGNTGYAQKFQGRVTMTRNTSISTAYMELNSLRSEDTAVYYCASSSGWYFFDYWGQGTLVTVSS |
| | | | SEQ ID NO: 25130 | SEQ ID NO: 29136 |

FIGURE 50

| | | | | |
|---|---|---|---|---|
| **iPS:398540** | **21-225_35A6** | NA | GACATCCAGATGACCCAGTCTCCATCCTCCCTGTCCGCATCTGTAGGAGACAGAGTCACCATCACTTGCCGGGCAAGTCAGGACATTAGAAGTGATTTAGGCTGGTATCAGCAGAAACCAGGGAAAGCCCCTAAGCGCCTGATCTATGCTACATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATACTATTTACCCTCCTACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCCACTATTAGTGGTCGTGGTGGTAGCACATTCCACGCAGACTCCGTGAAGGGCCGGTTCACCATCTCCAGAGACAATTCCAAGAACACGCTGTATCTGCAAATGAACAGCCTGAGAGCCGAAGACACGGCCGTATATTACTGTGTGAAAGGGGAGCTACTAGAGGACTACTACTTCTACGGTATGGACGTCTGGGGCCAAGGGACCACGGTCACCGTCTCCTCA |
| | | | SEQ ID NO: 25131 | SEQ ID NO: 29137 |
| | | AA | DIQMTQSPSSLSASVGDRVTITCRASQDIRSDLGWYQQKPGKAPKRLIYATSSLQSGVPSRFSGSGSGTEFTLTISSLQPEDFATYYCLQHTIYPPTFGGGTKVEIK | EVQLLESGGGLVQPGGSLRLSCAASGFTFSSYAMSWVRQAPGKGLEWVSTISGRGGSTFHADSVKGRFTISRDNSKNTLYLQMNSLRAEDTAVYYCVKGELLEDYYFYGMDVWGQGTTVTVSS |
| | | | SEQ ID NO: 25132 | SEQ ID NO: 29138 |
| **iPS:398544** | **21-225_7C8** | NA | CTGCCTGTGCTGACTCAGCCCCCGTCTGCATCTGCCTTGCTGGGAGCCTCGATCAAGCTCACCTGCACCCTAAGCAGTGAGCACAGCACCTACACCATCGAATGGTATCAACAGAGACCAGGGAGGTCCCCCCAGTATATAATGAAGGTTAAGAGTGATGGCAGCCACAGCAAGGGGGACGGGATCCCCGATCGCTTCATGGGCTCCAGTTCTGGGGGTGACCGCTACCTCACCTTCTCCAACCTCCAGTCTGACGATGAGGATGAGTATCACTGTGGAGAGAGCCACCCGATTGATGGCCAAGTCGGTGTGGTATTCGGCGGAGGGACCAAGCTGACCGTCCTA | GAGGTGCAGCTGGTGGAGTCTGGGGGAGGCTTGGTAAAGCCTGGGGGGTCCCTTAGACTCTCCTGTGCAGCCTCTGGATTCACTTTCAGTAACGCCCGGATGAACTGGGTCCGCCTGGCTCCAGGGAAGGGGCTGGAGTGGGTTGGCCGTATTAAAAGCAAAACTGATGGTGGGACAACAGACTACGCTGCACCCGTGAAAGGCAGATTCACCATCTCAAGAGATGAATCAGAAAACACGCTGTATCTGCAAATGAACAGCCTGAAAACCGAGGACACAGGTGTGTATTACTGTTCCACAGATACGGGTCCTATAGCAGCTCGTCTCGCTTACTACTACTACTACGCTATGGACGTCTGGGGCCAAGGGACCACGGTCACCGTCTCCTCA |

FIGURE 50

| | | | | |
|---|---|---|---|---|
| | | | SEQ ID NO: 25133 | SEQ ID NO: 29139 |
| | | AA | LPVLTQPPSASALLGASIKLTCTLSSEHSTYTIEWYQQRPGRSPQYIMKVKSDGSHSKGDGIPDRFMGSSSGGDRYLTFSNLQSDDEDEYHCGESHPIDGQVGVVFGGGTKLTVL | EVQLVESGGGLVKPGGSLRLSCAASGFTFSNARMNWVRLAPGKGLEWVGRIKSKTDGGTTDYAAPVKGRFTISRDESENTLYLQMNSLKTEDTGVYYCSTDTGPIAARLAYYYYYAMDVWGQGTTVTVSS |
| | | | SEQ ID NO: 25134 | SEQ ID NO: 29140 |
| iPS:398546 | 21-225_9H10 | NA | TCCTATGAGCTGACTCAGCCACCCTCAGTGTCCGTGTCCCCAGGACAGACAGCCAGCATCACCTGCTCTGGAGATAAATTGGGGGATAAATATGCTTGCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTTATGTGGTATTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGGTTCTCACTCACCACTAGTGGAGTGGGTGTGGGCTGGATCCGTCAGCCCCCAGGAAAGGCCCTGGAATGGCTTGCACTCATTTATTGGAGTGATGATAAGCGCTACAGCCCATCTCTGAAGAGCAGGCTCACCATCACCAAGGACACCTCCAAAAACCAGGTGGTCCTTACAATGACCAACATGGCCCCTGTGGACACAGCCACATATTACTGTGCACACACCGGTTCTAGCTGCTGCTATTTTGACTACTGGGGCCAGGGAACCCTGGTCACCGTCTCCTCA |
| | | | SEQ ID NO: 25135 | SEQ ID NO: 29141 |
| | | AA | SYELTQPPSVSVSPGQTASITCSGDKLGDKYACWYQQKPGQSPVLVIYQDSKRPSGIPERFSGSNSGNTATLTISGTQAMDEADYYCQAWDSSTYVVFGGGTKLTVL | QITLKESGPTLVKPTQTLTLTCTFSGFSLTTSGVGVGWIRQPPGKALEWLALIYWSDDKRYSPSLKSRLTITKDTSKNQVVLTMTNMAPVDTATYYCAHTGSSCCYFDYWGQGTLVTVSS |
| | | | SEQ ID NO: 25136 | SEQ ID NO: 29142 |

FIGURE 50

| | | | | |
|---|---|---|---|---|
| **iPS:402219** | **21-225_1C12** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAACATAATAGTTACCCGCTCACTTTCGGCGGAGGGACCAAGGTGGCGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGATGAAAATAATAAATACTATGTAGACTCCGTGAAGGGCCGATTCACCATCTCCAGAGACAACTCCAAGAACACGCTGTATCTGCAAATGAACAGCCTGAGAGCCGAGGACACGGCTGTGTATTACTGTGCGAGAGAATTGGGGTTCCGGTCTGACTACTGGGGCCAGGGAACCCTGGTCACCGTCTCCTCA |
| | | | SEQ ID NO: 25137 | SEQ ID NO: 29143 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVAIK | QVQLVESGGGVVQPGRSLRLSCAASGFTFSNYGMHWVRQAPGKGLEWVAVIWYDENNKYYVDSVKGRFTISRDNSKNTLYLQMNSLRAEDTAVYYCARELGFRSDYWGQGTLVTVSS |
| | | | SEQ ID NO: 25138 | SEQ ID NO: 29144 |
| **iPS:402221** | **21-225_2C12** | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAATCAGGGAAAGCCCCTAAGTCTCTGATCTCTGCTGCAACCAGTTTGCAAAGTGGGGTCCCATCACAGTTCAGCGGCAGTGGATCTGGGACAGATTTCACTCTCACCATCAGCAGCCTGCAGCCTGAAGATTTTGCAACTTATTACTGCCAACAGTATTATAGTTACCCGATCACCTTCGGCCAAGGGACACGACTGGAGATTAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTAGTTACATGTACTACGCAGACTCAGTGAAGGGCCGATTCACCATCTCCAGAGACAACGCCAAGGACTCACTGTATCTGCAAATGAACAGCCTGAGAGCCGAGGACACGGCTGTGTATTACTGTGCGAGAGTGAATCTCTTTGACTACTGGGGCCAGGGAACCCTGGTCACCGTCTCCTCA |
| | | | SEQ ID NO: 25139 | SEQ ID NO: 29145 |

FIGURE 50

| | | | | |
|---|---|---|---|---|
| | | AA | DIQMTQSPSSLSASVGDRVTITCRASQGISNYLAWFQQKSGKAPKSLISAATSLQSGVPSQFSGSGSGTDFTLTISSLQPEDFATYYCQQYYSYPITFGQGTRLEIK | EVQLVESGGGLVKPGGSLRLSCAASGFTFSSYSMNWVRQAPGKGLEWVSSISGSSSYMYYADSVKGRFTISRDNAKDSLYLQMNSLRAEDTAVYYCARVNLFDYWGQGTLVTVSS |
| | | | SEQ ID NO: 25140 | SEQ ID NO: 29146 |
| **iPS:402223** | **21-225_30A11** | NA | GACATCCAGATGACCCAGTCTCCATCTTCCGTGTCTGCATCTGTAGGAGACAGAGTCACCATCACTTGTCGGGCGAGTCAGGGTATTAGCAGATGGTTAGCCTGGTATCAGCAGAAACCAGGGAGAGCCCCTGAACTCCTGATCTATGCTGCATCCCGTTTGCAAAGTGGGATCCCATCCAGGTTCAGCGGCAGTGGATCTGGGACAGACTTCACTCTCACCATCAGCAGCCTGCAGCCTGAAGATTTTGCAACTTACTATTGTCAACAGGCTAACAGTTTCCCATTCACTTTCGGCCCTGGGACCAAAGTGGATAACAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGACTATCATATGCACTGGGTGCGACAGGCCCCTGGACAAGGGCTTGAGTGGATGGGATGGATCAACCCTAATAGGGGTGGCACAAACTATGCACAGAAGTTTCAGGACAGGGTCACCATGACCAGGGACACGTCCATCAGCACAGCCTACATGGAACTGAGCAGGCTGAGATCTGACGACACGGCCGTCTATTTCTGTGCGAGAGATGGAACTGGGTCCTTTGACTACTGGGGCCAGGGAACCCTGGTCACCGTCTCCTCA |
| | | | SEQ ID NO: 25141 | SEQ ID NO: 29147 |
| | | AA | DIQMTQSPSSVSASVGDRVTITCRASQGISRWLAWYQQKPGRAPELLIYAASRLQSGIPSRFSGSGSGTDFTLTISSLQPEDFATYYCQQANSFPFTFGPGTKVDNK | QVQLVQSGAEVKKPGASVKVSCKASGYTFTDYHMHWVRQAPGQGLEWMGWINPNRGGTNYAQKFQDRVTMTRDTSISTAYMELSRLRSDDTAVYFCARDGTGSFDYWGQGTLVTVSS |
| | | | SEQ ID NO: 25142 | SEQ ID NO: 29148 |

FIGURE 50

| | | | | |
|---|---|---|---|---|
| **iPS:402225** | **21-225_2B1** | NA | TCCTATGAGCTGACTCAGCCACCCTCAGTGTCCGTGTCCCCAGGACAGACAGTCAGCATCACCTGCTCTGGAGATAAATTGGGGGATAAATATGCTTGCTGGTATCAGCAGAAGCCAGGCCAGTCCCCTGTGCTGGTCATCTATCAAGATCGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAACAACACTGTGGTATTCGGCGGAGGGACCAAGCTGACCGTCCTA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTAGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGCGTCTGGGGAACTACTGGGGCCAGGGAACCCTGGTCACCGTCTCCTCA |
| | | | SEQ ID NO: 25143 | SEQ ID NO: 29149 |
| | | AA | SYELTQPPSVSVSPGQTVSITCSGDKLGDKYACWYQQKPGQSPVLVIYQDRKRPSGIPERFSGSNSGNTATLTISGTQAMDEADYYCQAWDNNTVVFGGGTKLTVL | EVQLVESGGGLVKPGGSLRLSCAASGFTFSSYSMNWVRQAPGKGLEWVSSISSSSSYIYYADSVKGRFTISRDNAKNSLYLQMNSLRAEDTAVYYCARLGNYWGQGTLVTVSS |
| | | | SEQ ID NO: 25144 | SEQ ID NO: 29150 |
| **iPS:402229** | **21-225_16H9** | NA | GACATCCAGATGACCCAGTCTCCATCCTCCCTGTCTGCATCTGTAGGAGACAGAGTCACCATCACTTGCCGGGCAAGTCAGGGCATTAGAAATTATTTAGGCTGGTTTCAGCAGAAACCAGGGAAAGCCCCTAAGCGCCTGATCTATGGTGCATCCAGTTTGCAAAGTGGGGTCCCATCAAGGATCAGCGGCAGTGGATCTGGGACAGAATTCACTCTCACAATCAGCAGCCTGCAGCCTGAAGATTTTGCAACTTATTACTGTCTACAGTATCATAGTTATCTATTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGTCAACGGTATGGACGTCTGGGGCCAAGGGACCACGGTCACCGTCTCCTCA |
| | | | SEQ ID NO: 25145 | SEQ ID NO: 29151 |

FIGURE 50

| | | | | |
|---|---|---|---|---|
| | | AA | DIQMTQSPSSLSASVGDRVTITCRASQGIRNYLGWFQQKPGKAPKRLIYGASSLQSGVPSRISGSGSGTEFTLTISSLQPEDFATYYCLQYHSYLFTFGPGTKVDIK | EVQLVESGGGLVKPGGSLRLSCAASGFTFSSYSMNWVRQAPGKGLEWVSSISGSSSYIYYADSVKGRFTISRDNAKNSLYLQMNSLRAEDTAVYYCARVNGMDVWGQGTTVTVSS |
| | | | SEQ ID NO: 25146 | SEQ ID NO: 29152 |
| iPS:402231 | 21-225_6D9 | NA | TCCTATGAGCTGACTCAGCCACCCTCAGTGTCCGTGTCCCCAGGACAGACAGCCAGCATCACCTGCTCTGGAGATAAATTGGGGGAAAAATATGCTTGCTGGTATCAGCAGAAGCCAGGCCAGTCCCCTGTACTGGTCATCTATCAAGATAAGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTGTATTCGGCGGAGGGACCAAACTGACCGTCCTA | GAGGTGCAGCTGGTGGAGTCTGGGGGAGGCTTGGTAAAGCCTGGGGGGTCCCTTAGACTCTCCTGTGCAGCCTCTGGATTCACTTTCAGTAACGCCTGGATGAACTGGGTCCGCCTGGCTCCAGGGAAGGGGCTGGAGTGGGTTGGCCGTATTAAAAGCAAAACTGATGGTGGGACAACAGACTACGCTGCACCCGTGAAAGGCAGATTCACCATCTCAAGAGATGATTCAGAAAACACGTTGTATCTGCAAATGAACAGCCTGAAAACCGAGGACACAGCCGTGTATTACTGTTCCACAGATACGGGTCCTATAGCAGCTCGTCTCGCTTACTACTACTACTACGCTATGGACGTCTGGGGCCAAGGGACCACGGTCACCGTCTCCTCA |
| | | | SEQ ID NO: 25147 | SEQ ID NO: 29153 |
| | | AA | SYELTQPPSVSVSPGQTASITCSGDKLGEKYACWYQQKPGQSPVLVIYQDKKRPSGIPERFSGSNSGNTATLTISGTQAMDEADYYCQAWDSSTVFGGGTKLTVL | EVQLVESGGGLVKPGGSLRLSCAASGFTFSNAWMNWVRLAPGKGLEWVGRIKSKTDGGTTDYAAPVKGRFTISRDDSENTLYLQMNSLKTEDTAVYYCSTDTGPIAARLAYYYYYAMDVWGQGTTVTVSS |
| | | | SEQ ID NO: 25148 | SEQ ID NO: 29154 |

FIGURE 50

| | | | | |
|---|---|---|---|---|
| **iPS:402233** | **21-225_16D10** | NA | GACATCCAGATGACCCAGTCTCCATCCTCACTGTCTGCATCTGTAGGAGACAGAGTCACCATCACTTGTCGGGCGAGTCAGGACATAAGTAATTATTTAGCCTGGTTTCAGCAGAAACCAGGGAAAGCCCCTAAGTCCCTGATCTATGCTACACCCAGTTTGCAGAGTGGGGTCCCATCAAAGTTCAGCGGCAGTGGATCTGGGACAGAGTTCACTCTCACCATCAGCAGCCTGCAGCCTGAAGATTTTGCAACTTATTACTGCCAACAGTATAATAGTTACCCGCTCACTTTCGGCGGAGGGACCAAGGTGGAGATCAAA | GAGGTGCAACTGGTGGAGTCTGGGGGAGGCCTGGTCAAGCCTGGGGGGTCCCTGAGACTCTCCTGTGCAGCCTCTGGATTCACCTTCAGTACCTATAACTTGAACTGGGTCCGCCAGGCTCCAGGGAAGGGGCTGGAGTGGGTCTCATCCATTAGTGGTGGTGCCGGTCACATATATTACTCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGGACTAATGGGTTTGACTTCTGGGGCCAGGGAACCCTGGTCACCGTCTCCTCA |
| | | | SEQ ID NO: 25149 | SEQ ID NO: 29155 |
| | | AA | DIQMTQSPSSLSASVGDRVTITCRASQDISNYLAWFQQKPGKAPKSLIYATPSLQSGVPSKFSGSGSGTEFTLTISSLQPEDFATYYCQQYNSYPLTFGGGTKVEIK | EVQLVESGGGLVKPGGSLRLSCAASGFTFSTYNLNWVRQAPGKGLEWVSSISGGAGHIYYSDSVKGRFTISRDNAKNSLYLQMNSLRAEDTAVYYCARTNGFDFWGQGTLVTVSS |
| | | | SEQ ID NO: 25150 | SEQ ID NO: 29156 |
| **iPS:402235** | **21-225_20F10** | NA | GACATCCAGTTGACCCAGTCTCCATCCTCACTGTCTGCATCTGTCGGAGACAGAGTCACCATCACTTGTCGGGCGAGTCAGGGCATTAACAATTATTTAGCCTGGTTTCAGCAGAAACCAGGGAAAGCCCCTAAGTCCCTGATTTATGCTGCATCCAGTTTGCAAAGTGGGGTCCCATCAAGGTTCAGCGGCAGTGGATTTGGGACAGATTTCACTCTCACCATCAGCAGCCTGCAGCCTGAAGATTTTGCAACTTTTTACTGCCAGCAATATAATAGTTACCCATTCACTTTTGGCCCTGGGACCAAAGTGGATAACAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTACTAGTACTTTCATATACTACGCAGATTCAGTGAAGGGCCGATTCACCATCTCAAGAGACAACGCCAAGAACTCACTGTATCTGCAAATGAACAGCCTGAGAGCCGAGGACACGGCTGTGTATTACTGTGCGAGAAAGGCTGGGCTTGATATCTGGGGCCAAGGGACAATGGTCACCGTCTCTTCA |
| | | | SEQ ID NO: 25151 | SEQ ID NO: 29157 |

FIGURE 50

| | | | | |
|---|---|---|---|---|
| | | AA | DIQLTQSPSSLSASVGDRVTITCRASQGINNYLAWFQQKPGKAPKSLIYAASSLQSGVPSRFSGSGFGTDFTLTISSLQPEDFATFYCQQYNSYPFTFGPGTKVDNK | EVQLVESGGGLVKPGGSLRLSCAASGFTFSSYSMNWVRQAPGKGLEWVSSISTSTFIYYADSVKGRFTISRDNAKNSLYLQMNSLRAEDTAVYYCARKAGLDIWGQGTMVTVSS |
| | | | SEQ ID NO: 25152 | SEQ ID NO: 29158 |
| **iPS:402237** | **21-225_23D11** | NA | GACATCCAGATGACCCAGTCTCCATCCTCACTGTCTGCATCTGTAGGGGACAGAGTCACCATCACTTGTCGGGCGAGTCAGGGCATTGCCAATTATTTAGCCTGGTTTCAGCAGAGACCAGGGAAAGCCCCTAAGTCCCTGATCTCTGCTGCATCCAGTTTGCAAAGTGGGGTCCCATCAAAGTTCAGCGGCAGTGGATCTGGGACAGAGTTCACTCTCACCATCAGCAGCCTGCAGCCTGAAGATTTTGCAACTTATTACTGCCAACAGTATCATAGTTACCCGCTCACTTTCGGCGGAGGGTCCAAGGTGGAGATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAACATAAACTGGGTCCGCCAGGCTCCAGGGAAGGGGCTGGAGTGGGTCTCATCCATTAGTGGTAATAGTGGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGCGAACTAACCTCTTTGACTACTGGGGCCAGGGAACCCTGGTCACCGTCTCCTCA |
| | | | SEQ ID NO: 25153 | SEQ ID NO: 29159 |
| | | AA | DIQMTQSPSSLSASVGDRVTITCRASQGIANYLAWFQQRPGKAPKSLISAASSLQSGVPSKFSGSGSGTEFTLTISSLQPEDFATYYCQQYHSYPLTFGGGSKVEIK | EVQLVESGGGLVKPGGSLRLSCAASGFTFSSYNINWVRQAPGKGLEWVSSISGNSGYIYYADSVKGRFTISRDNAKNSLYLQMNSLRAEDTAVYYCARTNLFDYWGQGTLVTVSS |
| | | | SEQ ID NO: 25154 | SEQ ID NO: 29160 |

FIGURE 50

| | | | | |
|---|---|---|---|---|
| iPS:403868 | 21-225_19D11 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTACATCCAGTTTGCAAAGTGGGGTCCCATCAAGGTTCAGCGGCAGTGGATCTGGGACAGAATTCACTCTCACAATCAGCAGCCTGCAGCCTGAGGATTTTGCAACTTATTACTGTCTACAGTATTATAGTTACCCGCTCACTTTCGGCGGAGGGACCGAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGCAGAAGTAGTTATTACTGGGGCTGGATCCGCCAGCCCCCCGGGAAGGGGCTGGACTGGATTGGGAGTATCTATTATAGTGGGAGCGCCAACTACAACCCGTCCCTCAAGAGTCGAGTCACCATATCCGTAGACACGTCCAAGAACCAGTTCTCCCTGAAACTGAGTTCCGTGACCGCCGCAGACGCGGCTGTGTATTACTGTGCGAGACTGGACAGGGGCTGGTCCTTTGACTACTGGGGCCAGGGAACCCTGGTCACCGTCTCCTCA |
| | | | SEQ ID NO: 25155 | SEQ ID NO: 29161 |
| | | AA | DIQMTQSPSSLSASVGDRVTITCRASQGIRNDLGWYQQKPGKAPKRLIYATSSLQSGVPSRFSGSGSGTEFTLTISSLQPEDFATYYCLQYYSYPLTFGGGTEVEIK | QLQLQESGPGLVKPSETLSLTCTVSGGSISRSSYYWGWIRQPPGKGLDWIGSIYYSGSANYNPSLKSRVTISVDTSKNQFSLKLSSVTAADAVYYCARLDRGWSFDYWGQGTLVTVSS |
| | | | SEQ ID NO: 25156 | SEQ ID NO: 29162 |
| iPS:403870 | 21-225_23G4 | NA | GACATCCAGATGACCCAGTCTCCATCCTCCCTGTCTGCATCTGTGGGAGACAGAGTCACCATCACTTGCCGGGCAAGTCAGAACATTTACAGCTATTTAAATTGGTATCAGCAGAAACCTGGGAAAGCCCCTAAGCTCCTGATCTATGCTGCATCCAGTTTGCAAAGTGGGGTTCCATCAAGGTTCAGTGGCAGTGGATCTGGGACAGAATTCACTCTCACCATCAGCATTCTGCAACCTGAAGATTTTGCAACTTACTACTGTCAACAGAGTTACAATACCCCTCCGGAGTGCAATTTTGGCCAGGGGACCAAGCTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTATTAGTGGCGTGGTGGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAAAGGGGGATAGTGGGAGCTACTGAGGCTTTTGATATCTGGGGCCAAGGGACAATGGTCACCGTCTCTTCA |
| | | | SEQ ID NO: 25157 | SEQ ID NO: 29163 |

FIGURE 50

| | | | | |
|---|---|---|---|---|
| | | AA | DIQMTQSPSSLSASVGDRVTITCRASQNIYSYLNWYQQKPGKAPKLLIYAASSLQSGVPSRFSGSGSGTEFTLTISILQPEDFATYYCQQSYNTPPECNFGQGTKLEIK | EVQLLESGGGLVQPGGSLRLSCAASGFTFSSYAMSWVRQAPGKGLEWVSVISGRGGSTYYADSVKGRFTISRDNSKNTLYLQMNSLRAEDTAVYYCAKRGIVGATEAFDIWGQGTMVTVSS |
| | | | SEQ ID NO: 25158 | SEQ ID NO: 29164 |
| iPS:403872 | 21-225_8F11 | NA | GACATCCAGATGACCCAGTCTCCATCCTCCCTGTCTGCATCTGTAGGAGACAGAGTCACCATCACTTGCCGGGCAAGTCAGGGCATTAGGAGTGATTTAGGCTGGTTTCAGCAGAAACCAGGGAAAGCCCCTAAGCGCCTGATCTTTGATGCATCCAGTGTGCAAAGTGGGGTCCCATCAAGGTTCAGCGGCAGTGGATCTGGGACAGAATTCACTCTCACAATCAGCAGCCTGCAGCCTGAAGATTTTGCAATTTATTACTGTCTACAACATTATACTTACCCGCTCACTTTCGGCGGAGGGACCAAGGTGGAGATCAAG | CAGCTGCAGCTGCAGGAGTCGGGCCCAGGACTGGTGCAGCCTTCGGAGACCCTGTCCCTCACCTGCACTGTCTCTGGTGTCTCCATCAGTAGGACTAGTTACTACTGGGGCTGGCTCCGCCAGCCCCCAGGGAAGGGGCTGGAGTGGATTGGCAATATTTATTATAGTGGGAGCGCCTACAACAACCCGTCCCTCAAGAGTCGAGTCACCATATCCGTTGACACGTCCAAGAACCAGTTCTCCCTGAAGCTGAGTTCTGTGACCGCCGCAGACACGGCTGTGTATTACTGTGGGAGACATGGACAAGACTGGGGCCTTGACTACTGGGGCCAGGGAACCCTGGTCACCGTCTCCTCA |
| | | | SEQ ID NO: 25159 | SEQ ID NO: 29165 |
| | | AA | DIQMTQSPSSLSASVGDRVTITCRASQGIRSDLGWFQQKPGKAPKRLIFDASSVQSGVPSRFSGSGSGTEFTLTISSLQPEDFAIYYCLQHYTYPLTFGGGTKVEIK | QLQLQESGPGLVQPSETLSLTCTVSGVSISRTSYYWGWLRQPPGKGLEWIGNIYYSGSAYNNPSLKSRVTISVDTSKNQFSLKLSSVTAADTAVYYCGRHGQDWGLDYWGQGTLVTVSS |
| | | | SEQ ID NO: 25160 | SEQ ID NO: 29166 |

FIGURE 50

| | | | | |
|---|---|---|---|---|
| **iPS:404090** | **21-225_8D8** | NA | TCCTATGAGCTGACTCAGCCACCCTCAGTGTCCGTGTCCCCAGGACAGACAGCCAGCATCACCTGCTCTGGAGATAAATTGGGGGAGAAATATGCTTGCTGGTATCAGCAGAAGCCAGGCCAGTCCCCTGTGGTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATAGATGAGGCTGACTATTACTGTCAGGCGTGGGACAGCAGCACTGCGGTATTCGGCGGAGGGACCAAGTTGACCGTCCTA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTAGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGCGTCTGGGTAACTACTGGGGCCAGGGAACCCTGGTCACCGTCTCCTCA |
| | | | SEQ ID NO: 25161 | SEQ ID NO: 29167 |
| | | AA | SYELTQPPSVSVSPGQTASITCSGDKLGEKYACWYQQKPGQSPVVVIYQDRKRPSGIPERFSGSNSGNTATLTISGTQAIDEADYYCQAWDSSTAVFGGGTKLTVL | EVQLVESGGGLVKPGGSLRLSCAASGFTFSSYSMNWVRQAPGKGLEWVSSISSSSSYIYYADSVKGRFTISRDNAKNSLYLQMNSLRAEDTAVYYCARLGNYWGQGTLVTVSS |
| | | | SEQ ID NO: 25162 | SEQ ID NO: 29168 |
| **iPS:412232** | **21-225_4A2** | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATAATACTCCAGTCACTTTCGGCCCTGGGACCAAAGTGGGTATCAAA | CAGGTGCAGCTGGTGCAGTCTGGGACTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCTTGACCAGGAACACCTCCATAAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTCTCCTCA |
| | | | SEQ ID NO: 25163 | SEQ ID NO: 29169 |

FIGURE 50

| | | | | |
|---|---|---|---|---|
| | | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYNTPVTFGPGTKVGIK | QVQLVQSGTEVKKPGASVKVSCKASGYTFTNYDINWVRQATGQGLEWMGWMHPNSGNTGYAQKFQGRVTLTRNTSISTAYMELSSLRSEDTAVYYCASSSGWYYFDYWGQGTLVTVSS |
| | | | SEQ ID NO: 25164 | SEQ ID NO: 29170 |
| iPS:422894 | 21-225_4A2.001 | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATAATACTCCAGTCACTTTCGGCCCTGGGACCAAAGTGGGTATCAAA | CAGGTGCAGCTGGTGCAGTCTGGGACTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCTTGACCAGGGACACCTCCATAAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTGTCCTCA |
| | | | SEQ ID NO: 25165 | SEQ ID NO: 29171 |
| | | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYNTPVTFGPGTKVGIK | QVQLVQSGTEVKKPGASVKVSCKASGYTFTNYDINWVRQATGQGLEWMGWMHPNSGNTGYAQKFQGRVTLTRDTSISTAYMELSSLRSEDTAVYYCASSSGWYYFDYWGQGTLVTVSS |
| | | | SEQ ID NO: 25166 | SEQ ID NO: 29172 |

FIGURE 50

| | | | | |
|---|---|---|---|---|
| **iPS:423018** | **21-225_31D12_LC2** | NA | TCCTATGAGCTGACTCAGCCACCCTCAGTGTCCGTGTCCCCAGGACAGACAGCCAGCATCACCTGCTCTGGAGATAAATTGGGGGATAAATATGCTTACTGGTTTCAGCAGAAGCCAGGCCAGTCCCCTGTGATAGTCATCTATCAAGATAGGAAGCGGCCCTCAGGGATCCCTGAGCGATTCTCTGGCTCCAACTCTGGGAACACAGCCACTCTGACCATCAGCGGGACGCAGGCTATGGATGAGGCTGACTATTACTGTCAGGCGTGGGACAACAGCACTGCGGTATTCGGCGGAGGGACCAAGCTGACCGTCCTA | CAGGTGAAGCTGGTGCAGTCTGGGGCTGAGGTGAAGAAGCCTGGGGCCTCAGTGAAGGTCTCCTGCAAGGCTTCAGGATACACCTTCACCGGCTACTATATGCACTGGGTGCGACAGGCCCCTGGACAAGGGCTTGAGTGGATGGGATGGATCAACCCTAACAGTGGTGGCACAAACTATGTACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGAGTGTATTACTATGGTTCGGGGAGTTATTATAACGAGTTTGACTACTGGGGCCAGGGAACCCTGGTCACCGTCTCCTCA |
| | | | SEQ ID NO: 25167 | SEQ ID NO: 29173 |
| | | AA | SYELTQPPSVSVSPGQTASITCSGDKLGDKYAYWFQQKPGQSPVIVIYQDRKRPSGIPERFSGSNSGNTATLTISGTQAMDEADYYCQAWDNSTAVFGGGTKLTVL | QVKLVQSGAEVKKPGASVKVSCKASGYTFTGYYMHWVRQAPGQGLEWMGWINPNSGGTNYVQKFQGRVTMTRDTSISTAYMELSRLRSDDTAVYYCARVYYYGSGSYYNEFDYWGQGTLVTVSS |
| | | | SEQ ID NO: 25168 | SEQ ID NO: 29174 |
| **iPS:423019** | **21-225_31D12_LC1** | NA | GATGTTGTGATGACTCAGTCTCCACTCTCCCTGCCCGTCACCCTTGGACAGCCGGCCTCCATCTCCTGCAGGTCTAGTCAAAGCCTCATATACAGTGATGGAAACACCTTCTTGAATTGGTTTCAGCAGAGGCCAGGCCAATCTCCAAGGCGCCTAATTTATAAGGTTTCTAATTGGGACTCTGGGGTCCCAGACAGATTCAGCGGCAGTGGGTCAGGCACTGATTTCACACTGAAAATCAGCAGGTTGGAGGCTGAGGATGTTGGGATTTATTACTGCATGCAAGGTACACACTGGCCTCTCACCTTCGGCCAAGGGACACGACTGGAGATTAAA | CAGGTGAAGCTGGTGCAGTCTGGGGCTGAGGTGAAGAAGCCTGGGGCCTCAGTGAAGGTCTCCTGCAAGGCTTCAGGATACACCTTCACCGGCTACTATATGCACTGGGTGCGACAGGCCCCTGGACAAGGGCTTGAGTGGATGGGATGGATCAACCCTAACAGTGGTGGCACAAACTATGTACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGAGTGTATTACTATGGTTCGGGGAGTTATTATAACGAGTTTGACTACTGGGGCCAGGGAACCCTGGTCACCGTCTCCTCA |
| | | | SEQ ID NO: 25169 | SEQ ID NO: 29175 |

FIGURE 50

| | | | | |
|---|---|---|---|---|
| | | AA | DVVMTQSPLSLPVTLGQPASISCRSSQSLIYSDGN TFLNWFQQRPGQSPRRLIYKVSNWDSGVPDRFS GSGSGTDFTLKISRLEAEDVGIYYCMQGTHWPL TFGQGTRLEIK | QVKLVQSGAEVKKPGASVKVSCKASGYTFTGYYM HWVRQAPGQGLEWMGWINPNSGGTNYVQKFQGR VTMTRDTSISTAYMELSRLRSDDTAVYYCARVYYY GSGSYYNEFDYWGQGTLVTVSS |
| | | | SEQ ID NO: 25170 | SEQ ID NO: 29176 |
| **iPS:423314** | **21-225_12F11** | NA | GACATCGTGATGACCCAGTCTCCAGACTCCCT GGCTGTGTCTCTGGGCGAGAGGGCCACCATCA ATTGCAAGTCCAGCCAGAGTGTTTTACACAGC TCCAACAATTATAACTACTTAGCTTGGTACCA GCAGAAACCAGGACAGCCTCCTAACCTGCTCA TTTTCTGGGCATCTACCCGGGAATCCGGGGTC CCTGACCGATTCAGTGGCAGCGGGTTTGGGAC AGATTTCACTCTCAACATCAGCAGCCTGCAGG CTGAAGATGTGGCAGTTTATTACTGTCAGCAA TATTATGATACTCCATTCACTTTCGGCCCTGGG ACCAAAGTGGATATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC CTGAGTGGATGGGATGGATGCACCCTAACAGTG GTAACACAGGCTATGCAAAGAAGTTCCAGGGCA GAGTCACCATGACCAGGAACACCTCCATAAGCG CAGCCTATATGGTTCTGAGCAGCCTGAGATCTGA GGACACGGCCGTGTATTACTGTGCGCTTAGCAGT GGCTGGTACTACTTTGACTACTGGGGCCAGGGAA CCCTGGTCACCGTCTCCTCA |
| | | | SEQ ID NO: 25171 | SEQ ID NO: 29177 |
| | | AA | DIVMTQSPDSLAVSLGERATINCKSSQSVLHSSN NYNYLAWYQQKPGQPPNLLIFWASTRESGVPDR FSGSGFGTDFTLNISSLQAEDVAVYYCQQYYDTP FTFGPGTKVDIK | QVQLVQSGAEVKKPGASVKVSCKASGYTFTNYDIN WVRQATGQGPEWMGWMHPNSGNTGYAKKFQGR VTMTRNTSISAAYMVLSSLRSEDTAVYYCALSSGW YYFDYWGQGTLVTVSS |
| | | | SEQ ID NO: 25172 | SEQ ID NO: 29178 |

FIGURE 50

| | | | | |
|---|---|---|---|---|
| **iPS:424419** | **21-225_25A4.001** | NA | GACATCGTGATGACCCAGTTTCCAGACTCCCTGGCTGTGTCTCTGGGCGAGAGGGCCACCATCAAGTGTAAGTCCAGCCAGAGTGTTTTATACAGCTCCCACAATAACAACTACTTAGCTTGGTACCAGCAGAAACCAGGACAGCCTCCTAAGTTGCTCCTTTACTGGGCATCTACCCGGGAATCCGGGGTCCCTGACCGATTCAGTGGCAGCGGGTCTGGGACAGAATTCACTCTCACCATCAGCAGCCTGGAGGCTGAAGATGTGGCAGTTTATTACTGTCAGCAGTATTATAGTACTCCTCCGACGTTCGGCCAAGGGACCAAGGTGGAAATCAAA | CAGGTGCTCCTGGTGCAGTCTGGGGCTGAGGTGAAGAGGCCTGGGGCCTCAGTGAAGGTCTCCTGCAAGGCTTCTGGATACACCTTCACCAATTATGATATTAATTGGGTGCGACAGGCCACTGGACAAGGGCTTGAGTGGATGGGATGGATGTACCCTAACAGTGGTAACACAGGCTATGCACAGAAATTCCAGGGCAGAGTCACCATGACCAGGGACACCTCCATCAGCACAGCCTACATGGAGCTGAGCAGCCTGAGATCTGAGGACACGGCCGTCTATTACTGTGCGAGTAGCAGTGGCTGGTACTACTTTGACTACTGGGGCCAGGGAACCCTGGTCACCGTGTCCTCA |
| | | | SEQ ID NO: 25173 | SEQ ID NO: 29179 |
| | | AA | DIVMTQFPDSLAVSLGERATIKCKSSQSVLYSSHNNNYLAWYQQKPGQPPKLLLYWASTRESGVPDRFSGSGSGTEFTLTISSLEAEDVAVYYCQQYYSTPPTFGQGTKVEIK | QVLLVQSGAEVKRPGASVKVSCKASGYTFTNYDINWVRQATGQGLEWMGWMYPNSGNTGYAQKFQGRVTMTRDTSISTAYMELSSLRSEDTAVYYCASSSGWYYFDYWGQGTLVTVSS |
| | | | SEQ ID NO: 25174 | SEQ ID NO: 29180 |
| **iPS:424460** | **21-225_7E11.001** | NA | GACATCCAGATGACCCAGTCTCCATCCTCCCTGTCTGCATCTGTAGGAGACAGAGTCACCATCGCTTGCCGGGCAAGTCAAAACATTATCAGCTATTTAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATGATGGAAGTAATAAATACTATGCAGACTCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 25175 | SEQ ID NO: 29181 |

FIGURE 50

| | | | | |
|---|---|---|---|---|
| | | AA | DIQMTQSPSSLSASVGDRVTIACRASQNIISYLNWYQQKPGKAPKFLIYTASSLQSGVPSRFSGSGSGTDFTLTISSLQPEDFAIYYCQQTYSTPLTFGGGTKVEIK | QVQLVESGGGVVQPGRSLRLSCAASGFTFSSFGMHWVRQAPGKGLEWVAIIWHDGSNKYYADSVKGRFTISRDNSKNTLYLQMSSLRAEDTAVYYCARDLSMGGMDVWGQGTTVTVSS |
| | | | SEQ ID NO: 25176 | SEQ ID NO: 29182 |
| iPS:426108 | 21-225_10G6 | NA | GACATCCAGATGACCCAGTCTCCATCTTCCGTGTCTGCATCTGTGGGAGACAGAGTCACCATCACTTGTCGGGCGAGTCAGGGTATTAGCAAATGGTTAGCCTGGTATCAGCAGAAACCAGGGAAAGCCCCTAAGATCCTGATATATGCTGCATATAGTTTACAAAGTGGGGTCCCAGCAAGGTTCAGCGGCAGTGGATCTGGGACAGATTTCACTCTCACCATCAGGAGCCTGCAGCCTGAAGATTTTGCAACTTACTATTGTCAACAGGCTAACAGTTTCCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGCCTACCATATGCACTGGGTGCGACAGGCCCCTGGACAAGGACTTGAGTGGATGGGATGGATCAACCCTAACAATAATGGCACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTTTATTACTGTGGGAGAGATGTTACCAGCTCGTTTGACTATTGGGGCCAGGGAACCCTGGTCACCGTCTCCTCA |
| | | | SEQ ID NO: 25177 | SEQ ID NO: 29183 |
| | | AA | DIQMTQSPSSVSASVGDRVTITCRASQGISKWLAWYQQKPGKAPKILIYAAYSLQSGVPARFSGSGSGTDFTLTIRSLQPEDFATYYCQQANSFPFTFGPGTKVDIK | QVQLVQSGAEVKKPGASVKVSCKASGYTFTAYHMHWVRQAPGQGLEWMGWINPNNNGTNYAQKFQGRVTMTRDTSISTAYMELSRLRSDDTAVYYCGRDVTSSFDYWGQGTLVTVSS |
| | | | SEQ ID NO: 25178 | SEQ ID NO: 29184 |

FIGURE 50

| | | | | |
|---|---|---|---|---|
| iPS:426110 | 21-225_12E9 | NA | GACATCCAGATGACCCAGTCTCCATCTTCCGTGTCTGCATCTGTAAGAGACAGAGTCACCATCACTTGTCGGGCGAGTCAGGGTATTAGCAGCTGGTTAGCCTGGTATCAGCAGAAACCAGGGGAAGCCCCTAAGCTCCTGATCTATGCTGCATCCAGGTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGGCTAACAGTTTCCCATTCACTTTCGGCCCTGGGACCAAAGTGGATATCAAA | CAGGTGCAGCTGGTACAGTCTGGGGCTGAGGTGAAGAAGCCTGGGGCCTCAGTGAAGGTCTCCTGCAAGGCTTCTGGATACACCTTCACCGACTACTATTTGCACTGGGTGCGACAGGCCCCTGGACAAGGTCTTGAGTGGATGGGATGGGTCCACCCTAACAGTGGTGGCACAAACTTTGCACAGAAGTTTCAGGACAGGGTCACCATGACCAGGGACACGTCCATCAGCACAGCCTACATGGAGCTGAGCAGCCTGAGATCTGACGACACGGCCATATATTCCTGTGCGCGAGATGGTACCAGCTCGTTTGACTACTGGGGCCAGGGAACCCTGGTCACCGTCTCCTCA |
| | | | SEQ ID NO: 25179 | SEQ ID NO: 29185 |
| | | AA | DIQMTQSPSSVSASVRDRVTITCRASQGISSWLAWYQQKPGEAPKLLIYAASRLQSGVPSRFSGSGSGTDFTLTISSLQPEDFATYYCQQANSFPFTFGPGTKVDIK | QVQLVQSGAEVKKPGASVKVSCKASGYTFTDYYLHWVRQAPGQGLEWMGWVHPNSGGTNFAQKFQDRVTMTRDTSISTAYMELSSLRSDDTAIYSCARDGTSSFDYWGQGTLVTVSS |
| | | | SEQ ID NO: 25180 | SEQ ID NO: 29186 |
| iPS:426112 | 21-225_12F12 | NA | GACATCGTGATGACCCAGTCTCCAGACTCCCTGGCTGTGTCTCTGGGCGAGAGGGCCACCATCAACTGCAAGTCCAGCCAGACTGTTTTATTCAGCTCCAACAATAACCACTACTTAGCATGGTACCAGCAGAAACCAGGACAACCTCCTAACCTCCTCATTTACTGGGCATCTACCCGGGCATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTAGTCCGTGGACGTTCGGCCAAGGGACCAAGGTGGAAATCAAA | CAGGTGCAGCTGCTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGTACCCTAACAGTGGTAACACGGGCTATGCACAGAAGTTCCAGGGCAGAGTCACCATGACCATGAACACCTCCATAAGCACAGCCTACATGGAGTTGAGCAGCCTGAGATCTGAGGACACGGCCGTGTATTACTGTGCGATGAGCAGTGGCTGGTACTACTTTGACTTCTGGGGCCAGGGAACCCTGGTCACCGTCTCCTCA |
| | | | SEQ ID NO: 25181 | SEQ ID NO: 29187 |

FIGURE 50

| | | | | |
|---|---|---|---|---|
| | | AA | DIVMTQSPDSLAVSLGERATINCKSSQTVLFSSNNNHYLAWYQQKPGQPPNLLIYWASTRASGVPDRFSGSGSGTDFTLTISSLQAEDVAVYYCQQYYSSPWTFGQGTKVEIK | QVQLLQSGAEVKKPGASVKVSCKASGYTFTNYDINWVRQATGQGLEWMGWMYPNSGNTGYAQKFQGRVTMTMNTSISTAYMELSSLRSEDTAVYYCAMSSGWYYFDFWGQGTLVTVSS |
| | | | SEQ ID NO: 25182 | SEQ ID NO: 29188 |
| iPS:426114 | 21-225_28H2 | NA | GACATCCAGATGACCCAGTCTCCTTCCTCCCTGTCTGCATCTATAGGAGACAGAGTCACCATCACTTGCCGGGCAAGTCAGGGCATTAGAAATGATTTAGGCTGGTATCAGCAGAAACCAGGGAAAGCCCCTAAGCGCCTGATTTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAACATTATAGTTACCCTCGCAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGTCATGCACTGGGTCCGCCAGGCTCCAGGCAAGGGGCTGGAGTGGGTGGCAGTTATATGGTATGATGGAAGTAATAAGTACTATGCAGACTCCGTGAAGGGCCGATTCACCATCTCCAGAGACAATTCCAAGAACACGCTGTATCTACAAATGAACAGCCTGAGAGCCGTGGACACGGCTGTGTATTACTGTGCGAGAGAGGAGTATAGCAGTGGCTGGTACGACTACGGTATGGACGTCTGGGGCCAAGGGACCACGGTCACCGTCTCCTCA |
| | | | SEQ ID NO: 25183 | SEQ ID NO: 29189 |
| | | AA | DIQMTQSPSSLSASIGDRVTITCRASQGIRNDLGWYQQKPGKAPKRLIYAASSLQSGVPSRFSGSGSGTEFTLTISSLQPEDFATYYCLQHYSYPRSFGQGTKLEIK | QVQLVESGGGVVQPGRSLRLSCAASGFTFSNYVMHWVRQAPGKGLEWVAVIWYDGSNKYYADSVKGRFTISRDNSKNTLYLQMNSLRAVDTAVYYCAREEYSSGWYDYGMDVWGQGTTVTVSS |
| | | | SEQ ID NO: 25184 | SEQ ID NO: 29190 |

FIGURE 50

| | | | | |
|---|---|---|---|---|
| iPS:426116 | 21-225_29E2 | NA | GACATCCAGATGACCCAGTCTCCATCCTCCCTGTCTGCATCTGTAGGAGACAGAGTCACCATCACTTGCCGGGCAAGTCAGGCCATTAGAAATGATTTAGGCTGGTATCAGCAGAGACCAGGGAAAGCCCCTAAGCGCCTGATCTATGCTGCATCCAGTTTGCAAAGTGGGGTCCCATCAAGGTTCAGCGGCAGTGGATCTGGGACAGAATTTATTCTCACAATCAGCAGCCTGCAGCCTGAAGATTTTGCAACTTATTACTGTTTACAGCATTATAATTACCCTCGCAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTGTGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGAATCACCATCTCCAGAGACAATTCCAAGAACACGCTGTATCTGCAAATGAACAGCCTGAGAGCCGAGGACACGGCTGTGTATTACTGTGCGAGAGAGGAGTATAGCAGTGGCTGGTACGACTACGGTATGGACGTCTGGGGCCAAGGGACCACGGTCACCGTCTCCTCA |
| | | | SEQ ID NO: 25185 | SEQ ID NO: 29191 |
| | | AA | DIQMTQSPSSLSASVGDRVTITCRASQAIRNDLGWYQQRPGKAPKRLIYAASSLQSGVPSRFSGSGSGTEFILTISSLQPEDFATYYCLQHYNYPRSFGQGTKLEIK | QVQLVESGGGVVQPGRSLRLSCAASGFTFSNCVMHWVRQAPGKGLEWVAVIWYDGSNKYYADSVKGRITISRDNSKNTLYLQMNSLRAEDTAVYYCAREEYSSGWYDYGMDVWGQGTTVTVSS |
| | | | SEQ ID NO: 25186 | SEQ ID NO: 29192 |
| iPS:426118 | 21-225_7A10 | NA | GACATCCAGATGACCCAGTCTCCATCCTCCCTGTCTGCATCTGTAGGAGACAGAGTCACCATCACTTGCCGGGCAAGTCAGAACATTTACAGCTATTTAAATTGGTATCAGCAGAAACCAGGGAGAGCCCCTAAACTCGTGATCTATTCTACATCCAGTTTGCAAAGTGGGGTCCCATCAAGGTTCAGTGGCAGTGGATCTGGGACAGATTTCAGTCTCACCATCAGCAATCTGCAACCTGAAGATTTTTCAACTTACTACTGTCAACAGAGTTACAGTCCCCCTCTCACTTTCGGCGGAGGGACCAAGGTGGAGATCAGA | CAGGTGCAGCTGGTGGAGTCTGGGGGAGGCGTGGTCCAGCCTGGGAGGTCCCTGAGACTCTCCTGTGCAGCGTCTGGATTCAACTTCAGTAGCTATGGCATGCACTGGGTCCGCCAGGCTCCAGGCAAGGGGCTGGAGTGGATGGCAGTTATATGGTATGATGGAAGTAATAAATACTATGCAGACTCCGTGAAGGGCCGATTCACCATCTCCAGAGACAATTCCAAGAACACGCTGTATCTGCAAATGCACAGCCTGAGAGCCGAGGACACGGCTGTGTATTACTGTGCGAGAGATGAGAGGCTGGGGATTTTTGACTACTGGGGCCAGGGAACCCTGGTCACCGTCTCCTCA |
| | | | SEQ ID NO: 25187 | SEQ ID NO: 29193 |

FIGURE 50

| | | | | |
|---|---|---|---|---|
| | | AA | DIQMTQSPSSLSASVGDRVTITCRASQNIYSYLNWYQQKPGRAPKLVIYSTSSLQSGVPSRFSGSGSGTDFSLTISNLQPEDFSTYYCQQSYSPPLTFGGGTKVEIR | QVQLVESGGGVVQPGRSLRLSCAASGFNFSSYGMHWVRQAPGKGLEWMAVIWYDGSNKYYADSVKGRFTISRDNSKNTLYLQMHSLRAEDTAVYYCARDERLGIFDYWGQGTLVTVSS |
| | | | SEQ ID NO: 25188 | SEQ ID NO: 29194 |
| iPS:426124 | 21-225_32D6 | NA | GACATCCAGATGACCCAGTCTCCATCCTCCCTGTCTACATCTGTAGGAGACAGAGTCACCATCACTTGCCGGGCAAGTCAGAACATTATCAGCTATTTAAATTGGTATCAGCAGAAACCAGGGAAAGCCCCTAAGCTCCTGATGTATGTTGCATCCCGTTTGCAAAGTGGGGTCCCATCAAGGTTCAGTGGCAGTGGATCTGGGACAGATTTCACTCTCACCATCAGCAGTCTGCAAGCTGAAGATTTTGCAACTTACTACTGTCAACAGAGTTACAGTACCCCGTACACTTTCGGCGGAGGGACCAAGGTGGCGATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGACTGGAGTGGGTGACAGTTATATGGCATGATGGAAGTAATGCATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAATAGCAGCTCGTACTACTTTGACTACTGGGGCCAGGGAACCCTGGTCACCGTCTCCTCA |
| | | | SEQ ID NO: 25189 | SEQ ID NO: 29195 |
| | | AA | DIQMTQSPSSLSTSVGDRVTITCRASQNIISYLNWYQQKPGKAPKLLMYVASRLQSGVPSRFSGSGSGTDFTLTISSLQAEDFATYYCQQSYSTPYTFGGGTKVAIK | QVQLVESGGGVVQPGRSLRLSCAASGFTFSSYGMHWVRQAPGKGLEWVTVIWHDGSNAYYADSVKGRFTISRDNSKNTLYLQMNSLRAEDTAVYYCARENSSSYYFDYWGQGTLVTVSS |
| | | | SEQ ID NO: 25190 | SEQ ID NO: 29196 |

FIGURE 50

| | | | | |
|---|---|---|---|---|
| **iPS:426126** | **21-225_6G6** | NA | GACATCGTGATGACCCAGTCTCCAGACTCCCTGGCTGTGTCTCTGGGCGAGAGGGCCACCATCAATTGCAAGTCCAGCCAGAGTGTTTTACACAACTCCAACAATTATAACTATTTAGCTTGGTACCAGCAGAAACCAGGACAGCCTCCTAACCTGCTCATTTTCTGGGCATCTACCCGGGAATCCGGGGTCCCTGACCGATTCAGTGGCAGCGGGTTTGGGACAGATTTCACTCTCAACATCAGCAGCCTGCAGGCTGAAGATGTGGCAGTTTATTACTGTCAGCAATATTATGATACTCCATTCACTTTCGGCCATGGGACCAAAGTGGATATCAAA | CAGGTGCAGCTGGTGCAGTCTGGGGCTGAGGTGAGGAAGCCTGGGGCCTCAGTGAAGGTCTCCTGCAAGGCTTCTGGATACACCTTCACCAATTATGATATCAACTGGGTGCGACAGGCCACTGGACAAGGGCCTGAGTGGATGGGATGGATGCACCCTAACAGTGGTAACACAGGCTATGCAAAGAAGTTCCAGGGCAGAGTCACCATGACCAGGAACACCTCCATAAGCGCAGCCTATATGGTTCTGAGCAGCCTGAGATCTGAGGACACGGCCGTGTATTACTGTGCGCTTAGCAGTGGCTGGTACTACTTTGACTACTGGGGCCAGGGAACCCTGGTCACCGTCTCCTCA |
| | | | SEQ ID NO: 25191 | SEQ ID NO: 29197 |
| | | AA | DIVMTQSPDSLAVSLGERATINCKSSQSVLHNSNNYNYLAWYQQKPGQPPNLLIFWASTRESGVPDRFSGSGFGTDFTLNISSLQAEDVAVYYCQQYYDTPFTFGHGTKVDIK | QVQLVQSGAEVRKPGASVKVSCKASGYTFTNYDINWVRQATGQGPEWMGWMHPNSGNTGYAKKFQGRVTMTRNTSISAAYMVLSSLRSEDTAVYYCALSSGWYYFDYWGQGTLVTVSS |
| | | | SEQ ID NO: 25192 | SEQ ID NO: 29198 |
| **iPS:433895** | **21-225_43E1** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGAAGAAACCAGGGAAAGCCCCTAAGCGCCTGATCAATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCCGCCATTAGTGGTAATAGTACTTACATATACTACGCAGACTCGTTGAAGGGCCGATTCACCATCTCCAGAGACAACGCCAAGAACTCACTGTTTCTGCAACTGAACAGCCTGAGAGCCGAGGACACGGCTGTTTATTACTGTGCGAGAGATCGGGGCAGTGAATGGGGCCAGGGAACCCTGGTCACCGTCTCCTCA |
| | | | SEQ ID NO: 25193 | SEQ ID NO: 29199 |

FIGURE 50

| | | | | |
|---|---|---|---|---|
| | | AA | DIQMTQSPSSLSASVGDRVTITCRASQGIRNDLGWYQKKPGKAPKRLINAASSLQSGVPSRFSGSGSGTEFTLTISSLQPEDFATYYCLQHNSYPFTFGPGTKVDIK | EVQLVESGGGLVKPGGSLRLSCAASGFTFSSYSMNWVRQAPGKGLEWVSAISGNSTYIYYADSLKGRFTISRDNAKNSLFLQLNSLRAEDTAVYYCARDRGSEWGQGTLVTVSS |
| | | | SEQ ID NO: 25194 | SEQ ID NO: 29200 |
| **iPS:433897** | **21-225_43C2** | NA | GACATCCAGATGACCCAGTCTCCATCTTCCGTGTCTGCATCTGTAGGAGACAGAGTCACCATCACTTGTCGGGCGAGTCAGGGTATTAGCGACTGGTTAGCCTGGTATCAGCAGAAACCAGGGAAAGCCCCTAGGCTCCTGATCTATGCTGCATCCAGTTTGCAAAGTGGGGTCCCATCAAGGTTCAGCGGCAGTGGATCTGGGACAGATTTCACTCTCACCATCAGCAACCTGCAGCCTGAAGATTTTGCAACTTACTATTGTCAACAGACTAACAGTTTCCCGTGGACGTTCGGCCAAGGGACCAAGGTGGAAATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTCGTGGTGTTAACACATTCGACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAGGAAAGGAGTGGGAGCTATTTTGACTACTGGGGCCAGGGAACCCTGGTCACCGTCTCCTCA |
| | | | SEQ ID NO: 25195 | SEQ ID NO: 29201 |
| | | AA | DIQMTQSPSSVSASVGDRVTITCRASQGISDWLAWYQQKPGKAPRLLIYAASSLQSGVPSRFSGSGSGTDFTLTISNLQPEDFATYYCQQTNSFPWTFGQGTKVEIK | EVQLLESGGGLVQPGGSLRLSCAASGFTFSSYAMSWVRQAPGKGLEWVSAISGRGVNTFDADSVKGRFTISRDNSKNTLYLQMNSLRAEDTAVYYCAKERSGSYFDYWGQGTLVTVSS |
| | | | SEQ ID NO: 25196 | SEQ ID NO: 29202 |

FIGURE 50

| | | | | |
|---|---|---|---|---|
| iPS:433899 | 21-225_43C3 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCTCTCACTTTCGGCGGAGGGACCAAGGTGGGGATCACA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATACACTGGGTCCGCCAGGCTCCAGGCAAGGGGCTGGAGTGGGTGGCAGTTATATGGTATGATGAAAATAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGCTAGGATTTTCCAATGACTACTGGGGCCAGGGAACCCTGGTCACCGTCTCCTCA |
| | | | SEQ ID NO: 25197 | SEQ ID NO: 29203 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVGIT | QVQLVESGGGVVQPGRSLRLSCAASGFTFSSYGIHWVRQAPGKGLEWVAVIWYDENNKYYADSVKGRFTISRDNSKNTLYLQMNSLRAEDTAVYYCARELGFSNDYWGQGTLVTVSS |
| | | | SEQ ID NO: 25198 | SEQ ID NO: 29204 |
| iPS:433901 | 21-225_43A4 | NA | GACATCCAGATGACCCAGTCTCCATCCTCACTGTCTGCATCTGTAGGAGACAGAGTCACCATCACTTGTCGGGCGAGTCAGGGCATTAACAATTATTTAGCCTGGTTTCAGCAGAAACCAGGGAAAGCCCCTAAGTCCCTGATCAATGCTGCATCCAGTTTGCAAAGTGGGGTCCCATCAAGGTTCAGCGGCAGTGGATCTGGGACAGATTTCACTCTCGCCATCAGCAGCCTACAGCCTGAAGATTTTGCAACTTATTACTGCCAACAGTATTATAGTTACCCATTCACTTTCGGCCCTGGGACCAAAGTGGATATCAAA | GAGGTGCAGCTGGTGGAGTCTGGGGGAGGCCTGGTCAAGCCGGGGGGGTCCCTGAGACTCTCCTGTGCAGCCTCTGGATTCACCTTCAGTAGCTATACCATGAACTGGGTCCGCCAGGTTCCAGGGAAGGGGCTGGAGTGGGTCTCATCCATTAGTGGAAGTAGTACTTACATATACTACGCAGACTCAGTGAAGGGCCGATTCACCATCTCCAGAGACGACGCCCAGAACTCACTGTATCTGCAAATGAACAGCCTGAGAGGCGAGGACACGGCTGTGTATTACTGTGCGAGGGTGACCTCTTTTGACTACTGGGGCCAGGGAGCCCTGGTCACCGTCTCCTCA |
| | | | SEQ ID NO: 25199 | SEQ ID NO: 29205 |

FIGURE 50

| | | | | |
|---|---|---|---|---|
| | | AA | DIQMTQSPSSLSASVGDRVTITCRASQGINNYLAWFQQKPGKAPKSLINAASSLQSGVPSRFSGSGSGTDFTLAISSLQPEDFATYYCQQYYSYPFTFGPGTKVDIK | EVQLVESGGGLVKPGGSLRLSCAASGFTFSSYTMNWVRQVPGKGLEWVSSISGSSTYIYYADSVKGRFTISRDDAQNSLYLQMNSLRGEDTAVYYCARVTSFDYWGQGALVTVSS |
| | | | SEQ ID NO: 25200 | SEQ ID NO: 29206 |
| iPS:433903 | 21-225_43H4 | NA | GACATCCAGATGACCCAGTCTCCATCTTCCGTGTCTGCATCTGTAGGAGACAGAGTCACCATCACTTGTCGGGCGAGTCAGGGTATTATCAACTGGTTAGCCTGGTATCAACAGAAACCAGGGAAAGCCCCTAAGCTCCTGATCTATGCTGCATCCAGTTTGCAAAGTGGGGTCCCATCAAGGTTCAGCGGCAGTGGATCTGGGACAGATTTCACTCTCACCATCAGCAACCTGCAGCCTGAAGATTTTGCAACTTACTATTGTCAACAGACTAACAGTTTCCCGTGGACGTTCGGCCAAGGGACCAAGGTGGAAATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTCGTGGTATTAACACATTCGACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAGGAAAGGAGTGGGAGCTATTTTGACTACTGGGGCCAGGGAACCCTGGTCACCGTCTCCTCA |
| | | | SEQ ID NO: 25201 | SEQ ID NO: 29207 |
| | | AA | DIQMTQSPSSVSASVGDRVTITCRASQGIINWLAWYQQKPGKAPKLLIYAASSLQSGVPSRFSGSGSGTDFTLTISNLQPEDFATYYCQQTNSFPWTFGQGTKVEIK | EVQLLESGGGLVQPGGSLRLSCAASGFTFSSYAMSWVRQAPGKGLEWVSAISGRGINTFDADSVKGRFTISRDNSKNTLYLQMNSLRAEDTAVYYCAKERSGSYFDYWGQGTLVTVSS |
| | | | SEQ ID NO: 25202 | SEQ ID NO: 29208 |

FIGURE 50

| | | | | |
|---|---|---|---|---|
| iPS:433905 | 21-225_43E5 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGGTGCATCCAATTTGCAAAGTGGGGTCCCATCAAGGTTCAGCGGCAGTGGATCTGGGACAGAATTCACTCTCACAGTCAGCAGCCTGCAGCCTGAAGATTTTGCAACTTATTTCTGTCTACAGCATACTAGTTTCCCATTCACTTTCGGCCCTGGGACCAAAGTGGATATCAAA<br>SEQ ID NO: 25203 | CAGGTGCAGCTGGTGGAGTCTGGGGGAGGCTTGGTCAAGCCTGGAGGGTCCCTGAGACTCTCCTGTGCAGCCTCTGGATTCACCTTCAGTGACTACTACATGATCTGGATCCGCCAGGCTCCAGGGAAGGGGCTGGAGTGGGTTTCATACATTAGTAGTAGTGGTATTACCAAATACTACGCAGACTCTATGAAGGGCCGATTCACCATCTCCAGGGACAACGCCAAGAACTCACTGTATCTGCAAATGAACAGCCTGAGAGCCGAGGACACGGCCGTGTATTACTGTGCGGCCGATACAATCTACTGGGGCCAGGGAACCCTGGTCACCGTCTCCTCA<br>SEQ ID NO: 29209 |
| | | AA | DIQMTQSPSSLSASVGDRVTITCRASQGIRNDLGWYQQKPGKAPKRLIYGASNLQSGVPSRFSGSGSGTEFTLTVSSLQPEDFATYFCLQHTSFPFTFGPGTKVDIK<br>SEQ ID NO: 25204 | QVQLVESGGGLVKPGGSLRLSCAASGFTFSDYYMIWIRQAPGKGLEWVSYISSSGITKYYADSMKGRFTISRDNAKNSLYLQMNSLRAEDTAVYYCAADTIYWGQGTLVTVSS<br>SEQ ID NO: 29210 |
| iPS:433909 | 21-225_43D8 | NA | GACATCGTGATGACCCAGTCTCCAGACTCCCTGGCTGTGTCTCTGGGCGAGAGGGCCACCATCAACTGCAAGTCCAGCCAGAGTGTTTTAATGACCTCCAACGATAAGAACTACTTAACTTGGTACCAGCAGAGACCAGGACAGCCTCCTAAGCTGCTCATTTACTGGGCTTCTACCCGGGAATCCGGGGTCCCTGACCGATTCAGTGGCGGCGGGTCTGGGACAGATTTCACTCTCACCATCAGCGGCCTGCAGGCTGAAGATGTGGCAGTTTATTACTGTCAGCAATATTATAGTACTCCTCCGACGTTCGGCCAAGGGACCAAGGTGGAAATCAAG<br>SEQ ID NO: 25205 | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGACATCTGAGGACACGGCCGTGTATTACTGTGCCCATAGCAGTGGCTGGACCCTCTTTGACTACTGGGGCCAGGGAACCCTGGTCACCGTCTCCGCA<br>SEQ ID NO: 29211 |

FIGURE 50

| | | | | |
|---|---|---|---|---|
| | | AA | DIVMTQSPDSLAVSLGERATINCKSSQSVLMTSNDKNYLTWYQQRPGQPPKLLIYWASTRESGVPDRFSGGGSGTDFTLTISGLQAEDVAVYYCQQYYSTPPTFGQGTKVEIK | QVQLVQSGAEVKKPGASVKVSCKASGYTFTNYDINWVRQATGQGLEWMGWMHPNSGNTGYAQKFQGRVTMTRNTSISTAYMELSSLTSEDTAVYYCAHSSGWTLFDYWGQGTLVTVSA |
| | | | SEQ ID NO: 25206 | SEQ ID NO: 29212 |
| iPS:433911 | 21-225_43E8 | NA | GACATCCAGATGAACCAGTCTCCATCTTCCGTGTCTGCATCTGTAGGAGACAGAGTCACCATCACTTGTCGGGCGAGTCAGGGTATTAGCAACTGGTTAGCCTGGTATCAGCAGAAACCAGGGAGAGCCCCTAAGCTCCTGATCTATGCTGCATCCAGTTTGCAAAGTGGGGTCCCTTCAAGGTTCAGCGGCAGTGGATCTGGGACAGATTTCACTCTCACCATCAGCAACCTGCAGCCTGAAGATTTTGCAACTTACTATTGTCAACAGACTAACAGTTTCCCGTGGACGTTCGGCCAAGGGACCAAGGTGGAAATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTCGTGGTATTAACACATTCGACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAGGAAAGGAGTGGGAGCTATTTTGACTACTGGGGCCAGGGAACCCTGGTCACCGTCTCCTCA |
| | | | SEQ ID NO: 25207 | SEQ ID NO: 29213 |
| | | AA | DIQMNQSPSSVSASVGDRVTITCRASQGISNWLAWYQQKPGRAPKLLIYAASSLQSGVPSRFSGSGSGTDFTLTISNLQPEDFATYYCQQTNSFPWTFGQGTKVEIK | EVQLLESGGGLVQPGGSLRLSCAASGFTFSSYAMSWVRQAPGKGLEWVSAISGRGINTFDADSVKGRFTISRDNSKNTLYLQMNSLRAEDTAVYYCAKERSGSYFDYWGQGTLVTVSS |
| | | | SEQ ID NO: 25208 | SEQ ID NO: 29214 |

FIGURE 50

| | | | | |
|---|---|---|---|---|
| **iPS:433913** | **21-225_43H8** | NA | GACATCCAAATGACCCAGTCTCCATCCTCCCTGTCTGCATCTGTAGGAGACAGAGTCACCATCACTTGCCGGGCAAGTCAGGGCATTAGAAATGATTTAGGCTGGCATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCTCAATCAGCAGCCTGCAGCCTGAAGATTTTGCAACTTATTACTGTCTACAGCATAATAGTTTCCCATTCACTTTCGGCCCTGGGACCAAAGTGGATATCAAA | CAGGTGCAGCTGGTGGAGTCTGGGGGAGGCTTGGTCAAGCCTGGAGGGTCCCTGAGACTCTCCTGTGCAGCCTCTGGAATCACCTTCAGTGACTACTACATGAACTGGATCCGCCAGGCTCCAGGGAAGGGGCTGGAGTGGGTTTCATACATTAGTAGTAGTGGTAGAACCATATTCTACGCAGACTCTTTGAAGGGCCGATTCACCATCTCCAGGGACAACGCCAAGAACTCACTGTATCTGCAAATGAACAGCCTGAGAGCCGAGGACACGGCCGTGTATTACTGTGCGGCCGATACAATCTACTGGGGCCAGGGAACCCTGGTCACCGTCTCCTCA |
| | | | SEQ ID NO: 25209 | SEQ ID NO: 29215 |
| | | AA | DIQMTQSPSSLSASVGDRVTITCRASQGIRNDLGWHQQKPGKAPKRLIYAASSLQSGVPSRFSGSGSGTEFTLSISSLQPEDFATYYCLQHNSFPFTFGPGTKVDIK | QVQLVESGGGLVKPGGSLRLSCAASGITFSDYYMNWIRQAPGKGLEWVSYISSSGRTIFYADSLKGRFTISRDNAKNSLYLQMNSLRAEDTAVYYCAADTIYWGQGTLVTVSS |
| | | | SEQ ID NO: 25210 | SEQ ID NO: 29216 |
| **iPS:433915** | **21-225_43H9** | NA | GACATCCAGATGACCCAGTCTCCATCTTCCGTGTCTGCATCTGTAGGAGACAGAGTCACCATCACTTGTCGGGCGAGTCAGGATATTAGCAGCTGGTTAGCCTGGTATCAGAAGAAACCAGGGAAAGCCCCTAAACTCCTGATCTATGATGCATCCAGTTTGCAAAGTGGGGTCCCATCAAGGTTCAGCGGCAGTGGATCTGGGACAGATTTCATTCTCACCATCAGCAGCCTGCAGCCTGAAGATTTTGCAACTTACTTTTGTCAACAGGCTAACAGTCTCCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGTTGGGTCCGCCAGGCTCCAGGGATGGGGCTGGAGTGGGTCTCAGCTATTAGTGGTAGTGGTAGTAACACATTCTACGCAGACTCCGTGAAGGGCCGGTTCACCATCTCCAGAGACAATTCCAAGAACACGCTGTATCTGCACATGAACAGCCTGAGAGCCGAGGACACTGCCGTATATTTCTGTGCGAAACGAACGCCCTCTGATGTTTTTGATATCTGGGGCCAAGGGACAATGGTCACCGTCTCTTCA |
| | | | SEQ ID NO: 25211 | SEQ ID NO: 29217 |

FIGURE 50

| | | | | |
|---|---|---|---|---|
| | | AA | DIQMTQSPSSVSASVGDRVTITCRASQDISSWLAWYQKKPGKAPKLLIYDASSLQSGVPSRFSGSGSGTDFILTISSLQPEDFATYFCQQANSLPFTFGPGTKVDIK | EVQLLESGGGLVQPGGSLRLSCAASGFTFSSYAMSWVRQAPGMGLEWVSAISGSGSNTFYADSVKGRFTISRDNSKNTLYLHMNSLRAEDTAVYFCAKRTPSDVFDIWGQGTMVTVSS |
| | | | SEQ ID NO: 25212 | SEQ ID NO: 29218 |
| iPS:433917 | 21-225_43E11 | NA | GATATTGTGATGACCCAGACTCCACTCTCTCTGTCCGTCACCCCTGGACAGCCGGCCTCCATCTCCTGCAAGTCTAGTCAGAGCCTCCTGCACAGTGATGGAAGGACCTATTTGTATTGGTACCTTCAGAAGCCAGGCCAGCCTCCGCAGCTCCTGATCTATGAAGTTTCCAACCGGTTCTCTGGAGTGCCAGATAGGTTCAGTGGCAGCGGGTCAGGGACAGACTTCACACTGAAAATCAGCCGGGTGGAGGCTGAGGATGTTGGGGTTTATTACTGTATGCAAAGTATACAGCTTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCTCCTTCAGTGACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGACTCACCATCTCCAGAGACAATTCCAAGAACACGCTGTATCTGCAAATGAACAGCCTGAGAGCCGAGGACACGGCTGTGTATTACTGTGCGAGGCGGTATGTCAGAAGCTGGGTGGGAGGTATGGACGTCTGGGGCCAAGGGACCACGGTCACCGTCTCCTCA |
| | | | SEQ ID NO: 25213 | SEQ ID NO: 29219 |
| | | AA | DIVMTQTPLSLSVTPGQPASISCKSSQSLLHSDGRTYLYWYLQKPGQPPQLLIYEVSNRFSGVPDRFSGSGSGTDFTLKISRVEAEDVGVYYCMQSIQLPWTFGQGTKVEIK | QVQLVESGGGVVQPGRSLRLSCAASGFSFSDYGMHWVRQAPGKGLEWVAVIWYDGSNKYYADSVKGRLTISRDNSKNTLYLQMNSLRAEDTAVYYCARRYVRSWVGGMDVWGQGTTVTVSS |
| | | | SEQ ID NO: 25214 | SEQ ID NO: 29220 |

FIGURE 50

| | | | | |
|---|---|---|---|---|
| **iPS:433919** | **21-225_44B3** | NA | GACATCCAGATGACCCAGTCTCCTTCCTCCCTGTCTGCATCTGTAGGAGACAGAGTCACCATCACTTGCCGGGCAAGTCAGGGCATTAGAAATGATTTAGGCTGGTATCAACAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACTGCATTATAATTACCCTCGGACGTTCGGCCAAGGGACCAAAGTGGAAATCAAG | CAGGTGCAGCTGGTGGAGTCTGGGGGAGGCGTGGTCCAGCCTGGGAGGTCCCTGAGACTCTCCTGTACAGCGTCTGGATTCACCTTCAGTGACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGAGGTATAGCAGTGGCTGGTACGACTACGGTATGGACGTCTGGGGCCAAGGGACCACGGTCACCGTCTCCTCA |
| | | | SEQ ID NO: 25215 | SEQ ID NO: 29221 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLLHYNYPRTFGQGTKVEIK | QVQLVESGGGVVQPGRSLRLSCTASGFTFSDYGMHWVRQAPGKGLEWVAVIWYDGSNKYYADSVKGRFTISRDNSKNTLYLQMNSLRAEDTAVYYCARERYSSGWYDYGMDVWGQGTTVTVSS |
| | | | SEQ ID NO: 25216 | SEQ ID NO: 29222 |
| **iPS:433921** | **21-225_44C3** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCGGTGGATCTGGGACAGAATTCACTCTCACAATCAGCAGCCTGCAGCCTGAAGATTTTGCGACTTATTACTGTCTACAGCATAGTAGTTACCCTCTCACTTTCGGCGGAGGGACCAAGGTGGAGATCAAG | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTTTGAAGGAAGTAATAAATACTATGCAGATTCCGTGAAGGGCCGATTCACCATCTCCAGAGACAATTCCAAGAACACGCTGTATCTGCAAATGAGCAGCCTGAGAGCCGAGGACACGGCTGTGTATTACTGTGTGAGAGAACTAGGATTTTCCACCGACTACTGGGGCCAGGGAACCCTGGTCACCGTCTCCTCA |
| | | | SEQ ID NO: 25217 | SEQ ID NO: 29223 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGGGSGTEFTLTISSLQPEDFATYYCLQHSSYPLTFGGGTKVEIK | QVQLVESGGGVVQPGRSLRLSCAASGFTFSSYGMHWVRQAPGKGLEWVAVIWFEGSNKYYADSVKGRFTISRDNSKNTLYLQMSSLRAEDTAVYYCVRELGFSTDYWGQGTLVTVSS |
| | | | SEQ ID NO: 25218 | SEQ ID NO: 29224 |
| **iPS:433923** | **21-225_44D3** | NA | GACATCCAGATGACCCAGTCTCCATCCTCCCTGTCTGCATCTGTGGGAGACAGAGTCACCTTCACTTGCCGGGCAAGTCAGGGCATTAGAGATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTAGATCTGGGAGAGAATTCACTCTCACAATCAGCAGCCTGCAACCTGAAGATTTTGCAACTTATTACTGTCTACAGCATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGTCATGCACTGGGTCCGCCAGGCTCCAGGCAAGGGGCTGGAGTGGGTGGCAGTTATATGGTATGATGGAAGTAATAAATACTATGCTGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAGGTATAGCAGTGGCTTGTACGACTACGGTATGGACGTCTGGGGCCAAGGGACCACGGTCACCGTCTCCTCA |
| | | | SEQ ID NO: 25219 | SEQ ID NO: 29225 |
| | | AA | DIQMTQSPSSLSASVGDRVTFTCRASQGIRDDLGWYQQKPGKAPKRLIYAASSLQSGVPSRFSGSRSGREFTLTISSLQPEDFATYYCLQHYSYPRTFGQGTKVEIK | QVQLVESGGGVVQPGRSLRLSCAASGFTFSSYVMHWVRQAPGKGLEWVAVIWYDGSNKYYADSVKGRFTISRDNSKNTLYLQMNSLRAEDTAVYYCARERYSSGLYDYGMDVWGQGTTVTVSS |
| | | | SEQ ID NO: 25220 | SEQ ID NO: 29226 |

FIGURE 50

| | | | | |
|---|---|---|---|---|
| **iPS:433925** | **21-225_44F3** | NA | GACATCCAGATGACCCAGTCTCCATCTTCCGTGTCTGCATCTGTAGGAGACAGAGTCACCATCACTTGTCGGGCGAGTCAGGGTATTAGCGACTGGTTAGCCTGGTATCAGCAGAAACCAGGGAAAGCCCCTAAGCTCCTGATTTATGCTGCATCCAGTTTACAAAGTGGAGTCCCATCAAGGTTCAGCGGCAGTGGATTTGGGACAGATTTCACTCTCACCATCAGCAGCCTGCAGCCTGAAGATTTTGCAACTTACTATTGTCAACAGGCTAACAGTTTCCCATTCACTTTCGGCCCTGGGACCAAAGTGGATATCAAA | GAGGTGCACCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAATTCTCAGTGGTGGTGGTAAGACCACATACTACGCAGACTCCGTGAAGGGCCGGTTCACCATCTCCAGAGACAATTCCAAGAACACGCTGTTTCTGCAAATGAACAGCCTGAGAGCCGAGGACACGGCCGTATATTACTGTGCGAAACGAACGCCCTCTGATGCTTTTGATATCTGGGGCCAAGGGACAATGGTCACCGTCTCTTCA |
| | | | SEQ ID NO: 25221 | SEQ ID NO: 29227 |
| | | AA | DIQMTQSPSSVSASVGDRVTITCRASQGISDWLAWYQQKPGKAPKLLIYAASSLQSGVPSRFSGSGFGTDFTLTISSLQPEDFATYYCQQANSFPFTFGPGTKVDIK | EVHLLESGGGLVQPGGSLRLSCAASGFTFSSYAMSWVRQAPGKGLEWVSILSGGGKTTYYADSVKGRFTISRDNSKNTLFLQMNSLRAEDTAVYYCAKRTPSDAFDIWGQGTMVTVSS |
| | | | SEQ ID NO: 25222 | SEQ ID NO: 29228 |
| **iPS:433929** | **21-225_44D5** | NA | GACATCCAGATGACCCAGTCTCCATCCTCCCTGTCTGCATCTGTAGGAGACAGAGTCACCATCACTTGCCGGGCAAGTCAGGACATTAGAAAAGATTTAGGCTGGTATCAGCAGAAACCAGGGAAAGCCCCTAAGCGCCTGATCTATGCTGCATCCACTTTGGAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTTCCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGTCCCGTATAGCAGCAGCTGGTACGACTACGGTATGGACGTCTGGGGCCAAGGGACCACGGTCACCGTCTCCTCA |
| | | | SEQ ID NO: 25223 | SEQ ID NO: 29229 |

FIGURE 50

| | | | | |
|---|---|---|---|---|
| | | AA | DIQMTQSPSSLSASVGDRVTITCRASQDIRKDLGWYQQKPGKAPKRLIYAASTLESGVPSRFSGSGSGTEFTLTISSLQPEDFATYYCLQHYSFPWTFGQGTKVEIK | QVQLVESGGGVVQPGRSLRLSCAASGFTFSSYVMHWVRQAPGKGLEWVAVIWYDGSNKYYADSVKGRFTISRDNSKNTLYLQMNSLRAEDTAVYYCARVPYSSSWYDYGMDVWGQGTTVTVSS |
| | | | SEQ ID NO: 25224 | SEQ ID NO: 29230 |
| **iPS:433931** | **21-225_44F6** | NA | GAAATTGTGTTGACGCAGTCTCCAGGCACCCTGTCTTTGTCTCCAGGGGAAAGAGCCACCCTCTCCTGCAGGGCCAGTCAGAGTGTTAGTGGAAGCTACTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTTTATTACTGTCAGCAGTATGGTAGTTCACCGTGGACGTTCGGCCAAGGGACCAAGGTGGAAATCAAA | CAGGTGCACCTGCAGGAGTCGGGCCCAGGACTGGTGAAGCCTTCGGAGACCCTGTCCCTCACCTGCACTGTCTCTGGTGGCTCCATCAGTAGTTACTACTGGAGCTGGATCCGGCAGCCCCCAGGGAAGGGACTGGAGTGGATTGGGTATATCTATTACAGTGGAAACACCAACTACAACCCCTCCCTCAAGAGTCGAGTCACCATATCAGTAGACACGTCCAAGAACCAGTTCTCCCTGAAGCTGAGCTCTGTGACCGCTGCGGACACGGCCGTGTATTACTGTGTGAGAGGGGTGGCTATAAAGAACTACTGGGGCCAGGGAATCCTGGTCACCGTCTCCTCA |
| | | | SEQ ID NO: 25225 | SEQ ID NO: 29231 |
| | | AA | EIVLTQSPGTLSLSPGERATLSCRASQSVSGSYLAWYQQKPGQAPRLLIYGASSRATGIPDRFSGSGSGTDFTLTISRLEPEDFAVYYCQQYGSSPWTFGQGTKVEIK | QVHLQESGPGLVKPSETLSLTCTVSGGSISSYYWSWIRQPPGKGLEWIGYIYYSGNTNYNPSLKSRVTISVDTSKNQFSLKLSSVTAADTAVYYCVRGVAIKNYWGQGILVTVSS |
| | | | SEQ ID NO: 25226 | SEQ ID NO: 29232 |

FIGURE 50

| | | | | |
|---|---|---|---|---|
| **iPS:433933** | **21-225_44C8** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAATTTGCAAAGTGGGGTCCCATCAAGGTTCAGCGGCAGTAGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAATAACTATGGCATGCACTGGGTCCGCCAGGCTCCAGGCAAGGGGCTGGAGTGGGTGGCAGTTATATGGTATGAAGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGTGAGAGAACTGGGGTTCCTCTCTGACTACTGGGGCCAGGGAACCCTGGTCACCGTCTCCTCA |
| | | | SEQ ID NO: 25227 | SEQ ID NO: 29233 |
| | | AA | DIQMTQSPSSLSASVGDRVTITCRASQGIRNDLGWYQQKPGKAPKRLIYAASNLQSGVPSRFSGSRSGTEFTLTISSLQPEDFATYYCLQHNSYPFTFGPGTKVDIK | QVQLVESGGGVVQPGRSLRLSCAASGFTFNNYGMHWVRQAPGKGLEWVAVIWYEGSNKYYADSVKGRFTISRDNSKNTLYLQMNSLRAEDTAVYYCVRELGFLSDYWGQGTLVTVSS |
| | | | SEQ ID NO: 25228 | SEQ ID NO: 29234 |
| **iPS:433935** | **21-225_44F9** | NA | GACGTCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTCATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTCCACCATTATAATTACCCTCGGACGTTCGGCCAAGGGACCAAGGTGGAAATCACA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACCATATAGCAGCAGCTGGTACGACTACGGTATGGACGTCGGGGGCCAAGGGACCACGGTCACCGTCTCCTCA |
| | | | SEQ ID NO: 25229 | SEQ ID NO: 29235 |

FIGURE 50

| | | | | |
|---|---|---|---|---|
| | | AA | DVQMTQSPSSLSASVGDRVTITCRASQGIRNDLG WYQQKPGKAPKRLIYAASSLQSGVPSRFSGSGS GTEFTLTISSLQPEDFATYYCLHHYNYPRTFGQG TKVEIT | QVQLVESGGGVVQPGRSLRLSCAASGFTFSSYVMH WVRQAPGKGLEWVAVIWYDGSNKYYADSVKGRF TISRDNSKNTLYLQMNSLRAEDTAVYYCAREPYSS SWYDYGMDVGGQGTTVTVSS |
| | | | SEQ ID NO: 25230 | SEQ ID NO: 29236 |
| iPS:433937 | 21-225_44B10 | NA | CATATTGTGATGACCCAGACTCCACTCTCTCTG TCCGTCACCCCTGGACAGCCGGCCTCCATCTC CTGCAAGTCTAGTCAGAGCCTCCTGCATAGTG AGGGAAGGACCTATTTGTATTGGTACCTGCAG AAGCCAGGCCAGCCTCCACAGCTCCTGATCTA TGAAATTTCCCACCGGTTCTCTGGAGTGCCAG ATAGATTCAGTGGCAGCGGGTCAGGGACAGA TTTCACACTGAAAATCAGCCGGGTGGAGGCTG AGGATGTTGGGGTTTATTACTGCATGCAAAGT ATCCACCTTCCGTTCACTTTCGGCGGAGGGAC CAAGGTGGAGATCAAA | CAGGTGCAGTTGGTGGAGTCTGGGGGAGGCGTG GTCCAGCCTGGGAGGTCCCTGAGACTCTCCTGTG TAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTATTGTGCGAGGCGGTATAG CAGCAGCTGGGTGGGGGTATGGACGTCTGGGG CCAAGGGACCACGGTCACCGTCTCCTCA |
| | | | SEQ ID NO: 25231 | SEQ ID NO: 29237 |
| | | AA | HIVMTQTPLSLSVTPGQPASISCKSSQSLLHSEGR TYLYWYLQKPGQPPQLLIYEISHRFSGVPDRFSG SGSGTDFTLKISRVEAEDVGVYYCMQSIHLPFTF GGGTKVEIK | QVQLVESGGGVVQPGRSLRLSCVASGFTFSSYGMH WVRQAPGKGLEWVAVIWYDGSNKYYADSVKGRF TISRDNSKNTLYLQMNSLRAEDTAVYYCARRYSSS WVGGMDVWGQGTTVTVSS |
| | | | SEQ ID NO: 25232 | SEQ ID NO: 29238 |

FIGURE 50

| | | | | |
|---|---|---|---|---|
| **iPS:433939** | **21-225_44C10** | NA | GACATCCAGATGACCCAGTCTCCATCCTCCCT<br>GTCTGCATCTGTAGGAGACAGAGTCACCTTCA<br>CTTGCCGGGCAAGTCAGGGCATTAGAGATGAT<br>TTAGGCTGGTATCAGCAGAAACCAGGGAAAG<br>CCCCTAAGCGCCTGATCTATGCTACATCCAGT<br>TTGCAAAGTGGGGTCCCATCAAGGTTCAGCGG<br>CAGTGGATCTGGGACAGAATTCACTCTCACAA<br>TCAGCAGCCTGCAGCCTGAAGATTTTGCAACT<br>TATTACTGTCTACAGCATTATAGTTACCCTCGG<br>ACGTTCGGCCAAGGGACCAAGGTGGAAATCA<br>AA | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTGACTGTGTCAT<br>GCACTGGGTCCGCCAGGCTCCAGGCAAGGGGCT<br>GGAGTGGGTGGCAGTTATATGGTATGATGGAAGT<br>AATAAATACTATGCTGACTCCGTGAAGGGCCGAT<br>TCACCATCTCCAGAGACAATTCCAAGAACACGCT<br>GTATCTGCAAATGAACAGCCTGAGAGCCGAGGA<br>CACGGCTGTGTATTACTGTGCGAGAGAAAGGTAT<br>AGCAGTGGCTTGTACGACTACGGTATGGACGTCT<br>GGGGCCAAGGGACCACGGTCACCGTCTCCTCA |
| | | | SEQ ID NO: 25233 | SEQ ID NO: 29239 |
| | | AA | DIQMTQSPSSLSASVGDRVTFTCRASQGIRDDLG<br>WYQQKPGKAPKRLIYATSSLQSGVPSRFSGSGSG<br>TEFTLTISSLQPEDFATYYCLQHYSYPRTFGQGT<br>KVEIK | QVQLVESGGGVVQPGRSLRLSCAASGFTFSDCVMH<br>WVRQAPGKGLEWVAVIWYDGSNKYYADSVKGRF<br>TISRDNSKNTLYLQMNSLRAEDTAVYYCARERYSS<br>GLYDYGMDVWGQGTTVTVSS |
| | | | SEQ ID NO: 25234 | SEQ ID NO: 29240 |
| **iPS:433941** | **21-225_44D10** | NA | GACATCCAGATGACCCAGTCTCCATCTTCCGT<br>GTCTGCATCTGTAGGAGACAGAGTCACCATCA<br>CTTGTCGGGCGAGTCAGGGTATTAGCGACTGG<br>TTAGCCTGGTATCAACAGAAACCAGGGAAAG<br>CCCCTAGGCTCCTGATCTATGCTGCATCCAGTT<br>TGCAAAGTGGGGTCCCATCAAAATTCAGCGGC<br>AGTGGATCTGGGACAGATTTCACTCTCACCAT<br>CAGCAACCTGCAGCCTGAAGATTTTGCAACTT<br>ACTATTGTCAACAGACTAACAGTTTCCCGTGG<br>ACGTTCGGCCAAGGGACCAAGGTGGAAATCA<br>AA | GAGGTGCAGCTGTTGGAGTCTGGGGGAGGCTTG<br>GTACAGCCTGGGGGGTCCCTGAGACTCTCCTGTG<br>CAGCCTCTGGATTCACCTTTAGCAGCTATGCCAT<br>GAGCTGGGTCCGCCAGGCTCCAGGGAAGGGGCT<br>GGAGTGGGTCTCAGCTATTAGTGGTCGTGGTGTT<br>AACACATTCGACGCAGACTCCGTGAAGGGCCGG<br>TTCACCATCTCCAGAGACAATTCCAAGAACACGC<br>TGTATCTGCAAATGAACAGCCTGAGAGCCGAGG<br>ACACGGCCGTATATTACTGTGCGAAGGAAAGGA<br>GTGGGAGCTATTTTGACTACTGGGGCCAGGGAAC<br>CCTGGTCACCGTCTCCTCA |
| | | | SEQ ID NO: 25235 | SEQ ID NO: 29241 |

FIGURE 50

| | | | | |
|---|---|---|---|---|
| | | AA | DIQMTQSPSSVSASVGDRVTITCRASQGISDWLAWYQQKPGKAPRLLIYAASSLQSGVPSKFSGSGSGTDFTLTISNLQPEDFATYYCQQTNSFPWTFGQGTKVEIK | EVQLLESGGGLVQPGGSLRLSCAASGFTFSSYAMSWVRQAPGKGLEWVSAISGRGVNTFDADSVKGRFTISRDNSKNTLYLQMNSLRAEDTAVYYCAKERSGSYFDYWGQGTLVTVSS |
| | | | SEQ ID NO: 25236 | SEQ ID NO: 29242 |
| iPS:433943 | 21-225_44E10 | NA | GATATTGTGATGACTCAGTCTCCACTCTCCCTGCCCGTCACCCCTGGAGAGCCGGCCTCCATCTCCTGCAGGTCTAGTCAGAGCCTCCTGCATAGTAATGGATACAGCTATTTGGAGTGGTACCTGCAGAAGCCAGGACAGTCTCCACAACTCCTGATCTATTTGGGTTCTAATCGGGCCTCCGGGGTCCCTGACAGGTTCAGTGGCAGTGGATCAGGCACAGATTTTACACTGAAAATCAGCAGAGTAGAGGCTGAGGATGTTGGGGTTTATTACTGCATGCAAACTCTACAAACTCCATTCACTTTCGGCCCTGGGACCAAAGTGGATATCAAA | GAGGTGCAGCTGTTGGAGTCTGGGGGAGGCTTGGTACAGTCTGGAGGGTCCCTGAGACTCTCCTGTGCAGCCTCTGGATTCACCTTTAGCAGCTATGCCATGAACTGGGTCCGCCAGGCTCCAGGGAAGGGGCTGGAGTGGGTCTCAGGTGTTGTTGGTAGTGGTGGTAGAACATACTACGCAGACTCCGTGAAGGGCCGGTTCATCATCTCCAGAGACAATTCCAAGAACACGCTGTATCTGCAAATGAACAGCCTGAGAGCCGAGGACACGGCCGTATATTACTGTGCGAAAGATCGGGGGCAGTGGCTCCTAGGCGGTATGGACGTCTGGGGCCAAGGGACCACGGTCACCGTCTCCTCA |
| | | | SEQ ID NO: 25237 | SEQ ID NO: 29243 |
| | | AA | DIVMTQSPLSLPVTPGEPASISCRSSQSLLHSNGYSYLEWYLQKPGQSPQLLIYLGSNRASGVPDRFSGSGSGTDFTLKISRVEAEDVGVYYCMQTLQTPFTFGPGTKVDIK | EVQLLESGGGLVQSGGSLRLSCAASGFTFSSYAMNWVRQAPGKGLEWVSGVVGSGGRTYYADSVKGRFIISRDNSKNTLYLQMNSLRAEDTAVYYCAKDRGQWLLGGMDVWGQGTTVTVSS |
| | | | SEQ ID NO: 25238 | SEQ ID NO: 29244 |

FIGURE 50

| | | | | |
|---|---|---|---|---|
| **iPS:433945** | **21-225_44C12** | NA | GACATCCAGATGACCCAGTCTCCATCTTCCGTGTCTGCATCTGTAGGAGACAGAGTCACCATCACTTGTCGGGCGAGTCAGGGTATTAGCAACTGGTTAGCCTGGTATCAGCAGAAACCAGGGAAAGCCCCTAAGCTCCTGATCTCTGCTGCATTCAGTTTGCAAAGTGGGGTCCCATCAAGGTTCAGCGGCAGTGGATCTGGGACAGATTTCACTCTCAGCATCAGCAGCCTGCAGCCTGAAGATTTTGCAACTTACTATTGTCAACAGTCTAACAGTTTCCCGTGGACGTTCGGCCAAGGGACCAAGGTGGAAATCAAA | GAGGTGCAGCTGGTGGAGTCTGGGGGAGGCTTGGTACAGCCTGGGGGGTCCCTGAGACTCTCCTGTGCAGCCTCTGGATTCACCTTCAGTAGCTATAGCGTGAACTGGGTCCGCCAGGCTCCAGGGAAGGGGCTGGAGTGGGTTTCATACATTAGTAGTAGTAGTAGTACCATATACTACGCAGACTCTGTGAAGGGCCGATTCACCATCTCCAGAGACAATGCCAAGAACTCACTGTATCTGCAAATGAACAGCCTGAGAGACGAGGACACGGCTGTGTATTACTGTGCGAGAAGTGGATACAGCTATGCTTACTACTACTACTACGGTATGGACGTCTGGGGCCAAGGGACCACGGTCACCGTCTCCTCA |
| | | | SEQ ID NO: 25239 | SEQ ID NO: 29245 |
| | | AA | DIQMTQSPSSVSASVGDRVTITCRASQGISNWLAWYQQKPGKAPKLLISAAFSLQSGVPSRFSGSGSGTDFTLSISSLQPEDFATYYCQQSNSFPWTFGQGTKVEIK | EVQLVESGGGLVQPGGSLRLSCAASGFTFSSYSVNWVRQAPGKGLEWVSYISSSSSTIYYADSVKGRFTISRDNAKNSLYLQMNSLRDEDTAVYYCARSGYSYAYYYYYGMDVWGQGTTVTVSS |
| | | | SEQ ID NO: 25240 | SEQ ID NO: 29246 |
| **iPS:433947** | **21-225_44E12** | NA | GACATCCAGATGACCCAGTCTCCATCCTCCCTGTCTGCATCTGTAGGAGACAGAGTCACCATCACTTGCCGGACAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAACATAATAGTTACCCGCTCACTTTCGGCGGAGGGACCAAGGTGGAGATCAAA | CAGGTGCACCTGGCGGAGTCTGGGGGAGGCGTGGTCCAGCCTGGGAGGTCCCTGAGACTCTCCTGTGAAGCGTCTGGATTCACCCTCAGTAGCGATGACACGCACTGGGTCCGCCAGCCTCCAGGCAAGGGGCTGGAGTGGGTGGCAGTTATATGGTTTGATGAATATAATAAATACTATGCAGACTCCGTGAAGGGCCGATTCACCATCTCCAGAGACAATTCCAAGAACACGCTGTATCTGCAAATGAACAGCCTGAGAGCCGAGGACGCGGCTGTGTATTACTGTGCGAGAGATCTAATAGCAGCAGCTGGGACGGGAGACTACTGGGGCCAGGGAACCCTGGTCACCGTCTCCTCA |
| | | | SEQ ID NO: 25241 | SEQ ID NO: 29247 |

FIGURE 50

| | | | | |
|---|---|---|---|---|
| | | AA | DIQMTQSPSSLSASVGDRVTITCRTSQGIRNDLGWYQQKPGKAPKRLIYAASSLQSGVPSRFSGSGSGTEFTLTISSLQPEDFATYYCLQHNSYPLTFGGGTKVEIK | QVHLAESGGGVVQPGRSLRLSCEASGFTLSSDDTHWVRQPPGKGLEWVAVIWFDEYNKYYADSVKGRFTISRDNSKNTLYLQMNSLRAEDAAVYYCARDLIAAAGTGDYWGQGTLVTVSS |
| | | | SEQ ID NO: 25242 | SEQ ID NO: 29248 |
| iPS:433949 | 21-225_45H2 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGGTGCATCCAATTTGCAAAGTGGGGTCCCATCAAGGTTCAGCGGCAGTGGATCTGGGACAGAATTCACTCTCACAGTCAGCAGCCTGCAGCCTGAAGATTTTGCAACTTATTTCTGTCTACAGCATACTAGTTTCCCATTCACTTTCGGCCCTGGGACCAAAGTGGATATCAAA | CAGGTGCAGCTGGTGGAGTCTGGGGGAGGCTTGGTCAAGCCTGGAGGGTCCCTGAGACTCTCCTGTGCAGCCTCTGGATTCACCTTTAGTGACTACTACATGAACTGGATCCGCCAGGCTCCAGGGAAGGGGCTGGAGTGGGTTTCATACATTAGTAGTAGTGGTATTACCAAATACTACGCAGACTCTGTGAAGGGCCGATTCACCATCTCCAGGGACAACGCCAAGAACTCACTGTATCTGCAAATGAACAGCCTGAGAGCCGAGGACACGGCCGTGTATTACTGTGCGGCCGATACAATCTACTGGGGCCAGGGAACCCTGGTCACCGTCTCCTCA |
| | | | SEQ ID NO: 25243 | SEQ ID NO: 29249 |
| | | AA | DIQMTQSPSSLSASVGDRVTITCRASQGIRNDLGWYQQKPGKAPKRLIYGASNLQSGVPSRFSGSGSGTEFTLTVSSLQPEDFATYFCLQHTSFPFTFGPGTKVDIK | QVQLVESGGGLVKPGGSLRLSCAASGFTFSDYYMNWIRQAPGKGLEWVSYISSSGITKYYADSVKGRFTISRDNAKNSLYLQMNSLRAEDTAVYYCAADTIYWGQGTLVTVSS |
| | | | SEQ ID NO: 25244 | SEQ ID NO: 29250 |

FIGURE 50

| | | | | |
|---|---|---|---|---|
| iPS:433951 | 21-225_45B4 | NA | GACATCCAGATGACCCAGTCTCCATCCTCCCTGTCTGCATCTGTAGGAGACAGAGTCACCTTCACTTGCCGGGCAAGTCAGGGCATTAGAGATGATTTAGGCTGGTATCAACAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTGTGTCATGCACTGGGTCCGCCAGGCTCCAGGCAAGGGGCTGGAGTGGGTGGCAGTTATATGGTATGATGGAAGTAATAAATACTATGCTGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAGGTATAGCAGTGGCTTGTACGACTACGGTATGGACGTCTGGGGCCAAGGGACCACGGTCACCGTCTCCTCA |
| | | | SEQ ID NO: 25245 | SEQ ID NO: 29251 |
| | | AA | DIQMTQSPSSLSASVGDRVTFTCRASQGIRDDLGWYQQKPGKAPKRLIYAASSLQSGVPSRFSGSGSGTEFTLTISSLQPEDFATYYCLQHYSYPRTFGQGTKVEIK | QVQLVESGGGVVQPGRSLRLSCAASGFTFSDCVMHWVRQAPGKGLEWVAVIWYDGSNKYYADSVKGRFTISRDNSKNTLYLQMNSLRAEDTAVYYCARERYSSGLYDYGMDVWGQGTTVTVSS |
| | | | SEQ ID NO: 25246 | SEQ ID NO: 29252 |
| iPS:433953 | 21-225_45H4 | NA | GACATCCAGATGACCCAGTCTCCATCTTCCGTGTCTGCATCTGTAGGAGACAGAGTCACCATCACTTGTCGGGCGAGTCAGGATATTAGCAGCTGGTTAGCCTGGTATCAGAAGAAACCAGGGAAAGCCCCTAAGTACCTGATCTATGATGCATCCAGTTTGCAAAGTGGGGTCCCATCAAGGTTCAGCGGCAGTGGATCTGGGACAGATTTCATTCTCACCATCAGCAGCCTGCAGCCTGAAGATTTTGCAATTTACTTTTGTCAACAGGCTAACAGTCTCCCTTTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGTTGGGTCCGCCAGGCTCCAGGGATGGGGCTGGAGTGGGTCTCAGCTATTAGTGGTAGTGGTAGTAACACATTCTACGCAGACTCCGTGAAGGGCCGGTTCACCATCTCCAGAGACAATTCCAAGAACACGCTGTATCTGCACATGAACAGCCTGAGAGCCGAGGACACTGCCGTATATTTCTGTGCGAAACGAACGCCCTCTGATGTTTTTGATATCTGGGGCCAAGGGACAATGGTCACCGTCTCTTCA |
| | | | SEQ ID NO: 25247 | SEQ ID NO: 29253 |

FIGURE 50

| | | | | |
|---|---|---|---|---|
| | | AA | DIQMTQSPSSVSASVGDRVTITCRASQDISSWLAWYQKKPGKAPKYLIYDASSLQSGVPSRFSGSGSGTDFILTISSLQPEDFAIYFCQQANSLPFTFGPGTKVDIK | EVQLLESGGGLVQPGGSLRLSCAASGFTFSSYAMSWVRQAPGMGLEWVSAISGSGSNTFYADSVKGRFTISRDNSKNTLYLHMNSLRAEDTAVYFCAKRTPSDVFDIWGQGTMVTVSS |
| | | | SEQ ID NO: 25248 | SEQ ID NO: 29254 |
| iPS:433955 | 21-225_45B8 | NA | GACATCCAGATGACCCAGTCTCCATCCTCCCTGTCTGCATCTGTAGGAGACAGAGTCACCTTCACTTGCCGGGCAAGTCAGGACATTAGAGATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAATTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTGTGTCATGCACTGGGTCCGCCAGGCTCCAGGCAAGGGGCTGGAGTGGGTGGCAGTTATATGGTATGATGGAAGTAATAAATACTATGCTGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAGGTATAGCAGTGGCTTGTACGACTACGGTATGGACGTCTGGGGCCAAGGGACCACGGTCACCGTCTCCTCA |
| | | | SEQ ID NO: 25249 | SEQ ID NO: 29255 |
| | | AA | DIQMTQSPSSLSASVGDRVTFTCRASQDIRDDLGWYQQKPGKAPKRLIYAASSLQSGVPSRFSGSGSGTEFTLTISSLQPEDFATYYCLQHYNYPRTFGQGTKVEIK | QVQLVESGGGVVQPGRSLRLSCAASGFTFSDCVMHWVRQAPGKGLEWVAVIWYDGSNKYYADSVKGRFTISRDNSKNTLYLQMNSLRAEDTAVYYCARERYSSGLYDYGMDVWGQGTTVTVSS |
| | | | SEQ ID NO: 25250 | SEQ ID NO: 29256 |

FIGURE 50

| | | | | |
|---|---|---|---|---|
| iPS:433957 | 21-225_45F8 | NA | GACATCCAGATGACCCAGTCTCCATCTTCCGTGTCTGCATCTGTAGGAGACAGAGTCACCATCACTTGTCGGGCGAGTCAGGGTATTAGCGACTGGTTAGCCTGGTATCAGCAGAAACCAGGGAAAGCCCCTAGGCTCCTGATCTATGGTGCATCCAGTTTGCAAAGTGGGGTCCCATCAAGGTTCAGCGGCAGTGGATCTGGGACAGATTTCACTCTCACCATCAGCAACCTGCAGCCTGAAGATTTTGCAACTTACTATTGTCAACAGACTAACAGTTTCCCGTGGACGTTCGGCCAAGGGACCAAGGTGGAAATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTCGTGGTGTTAACACATTCGACGCAGACTCCGTGAAGGGCCGGTTCACCATATCCAGAGACAATTCCAAGAACACGCTGTATCTGCAAATGAACAGCCTGAGAGCCGAGGACACGGCCGTATATTACTGTGCGAAGGAAAGGAGTGGGAGCTATTTTGACTACTGGGGCCAGGGAACCCTGGTCACCGTCTCCTCA |
| | | | SEQ ID NO: 25251 | SEQ ID NO: 29257 |
| | | AA | DIQMTQSPSSVSASVGDRVTITCRASQGISDWLAWYQQKPGKAPRLLIYGASSLQSGVPSRFSGSGSGTDFTLTISNLQPEDFATYYCQQTNSFPWTFGQGTKVEIK | EVQLLESGGGLVQPGGSLRLSCAASGFTFSSYAMSWVRQAPGKGLEWVSAISGRGVNTFDADSVKGRFTISRDNSKNTLYLQMNSLRAEDTAVYYCAKERSGSYFDYWGQGTLVTVSS |
| | | | SEQ ID NO: 25252 | SEQ ID NO: 29258 |
| iPS:433959 | 21-225_45C9 | NA | GACATCCAGATGACCCAGTCTCCATCTTCCGTGTCTGTTTCTGTAGGAGACAGAGTCACCATCACTTGTCGGGCGAGTCAGGATATTAGCGACTGGTTAGCTTGGTATCAGCAGAGACCAGGGAAAGCCCCTAAGCTCTTGATCTATGCTGCATCCAGTTTGGAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGGCTAACAGTTTCCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAACAGCTATGCCATGAGCTGGGTCCGCCAGGCTCCAGGGAAGGGGCTGGAGTGGGTCTCAGTTATTAGTGGTCGTGGTGGTACCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAACGAACGCCCTCTGATGCTTTTGATATCTGGGGCCAAGGGACAATGGTCACCGTCTCTTCA |
| | | | SEQ ID NO: 25253 | SEQ ID NO: 29259 |

FIGURE 50

| | | | | |
|---|---|---|---|---|
| | | AA | DIQMTQSPSSVSVSVGDRVTITCRASQDISDWLAWYQQRPGKAPKLLIYAASSLESGVPSRFSGSGSGTDFTLTISSLQPEDFATYYCQQANSFPFTFGPGTKVDIK | EVQLLESGGGLVQPGGSLRLSCAASGFTFNSYAMSWVRQAPGKGLEWVSVISGRGGTTYYADSVKGRFTISRDNSKNTLYLQMNSLRAEDTAVYYCAKRTPSDAFDIWGQGTMVTVSS |
| | | | SEQ ID NO: 25254 | SEQ ID NO: 29260 |
| iPS:433961 | 21-225_45D9 | NA | GACATCCAGATGACCCAGTCTCCATCCTCACTGTCTGCATCTGTAGGAGACAGAGTCACCATCACTTGTCGGGCGAGTCAGGGCATTAACAATTATTTAGCCTGGTTTCAGCAGAAACCAGGGAAAGCCCCTAAGTCCCTGATCAATGCTGCATCCAGTTTGCAAAGTGGGGTCCCATCAAGGTTCAGCGGCAGTGGATCTGGGACAGATTTCACTCTCGCCATCAGCAGCCTACAGCCTGAAGATTTTGCAACTTATTACTGCCAACACTATTATAGTTACCCATTCACTTTCGGCCGTGGGACCAAAGTGGATATCAAA | GAGGTGCAGCTGGTGGAGTCTGGGGGAGGCCTGGTCAAGCCGGGGGGGTCCCTGAGACTCTCCTGTGCAGCCTCTGGATTCACCTTCAGTAGCTATACCATGAACTGGGTCCGCCAGGTTCCAGGGAAGGGGCTGGAGTGGGTCTCATCCATTAGTGGAAGTAGTACTTACATATACTACGCAGACTCAGTGAAGGGCCGATTCACCATCTCCAGAGACGACGCCCAGAACTCACTGTATCTGCAAATGAACAGCCTGAGAGGCGAGGACACGGCTGTGTATTACTGTGCGAGGGTGACCTCTTTTGACTACTGGGGCCAGGGAGCCCTGGTCACCGTCTCCTCA |
| | | | SEQ ID NO: 25255 | SEQ ID NO: 29261 |
| | | AA | DIQMTQSPSSLSASVGDRVTITCRASQGINNYLAWFQQKPGKAPKSLINAASSLQSGVPSRFSGSGSGTDFTLAISSLQPEDFATYYCQHYYSYPFTFGRGTKVDIK | EVQLVESGGGLVKPGGSLRLSCAASGFTFSSYTMNWVRQVPGKGLEWVSSISGSSTYIYYADSVKGRFTISRDDAQNSLYLQMNSLRGEDTAVYYCARVTSFDYWGQGALVTVSS |
| | | | SEQ ID NO: 25256 | SEQ ID NO: 29262 |

FIGURE 50

| | | | | |
|---|---|---|---|---|
| **iPS:433963** | **21-225_46B1** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAGGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCACCTGGCGGAGTCTGGGGGAGGCGTGGTCCAGCCTGGGAGGTCCCTGAGACTCTCCTGTGAAGCGTCTGGATTCACCCTCAGTAGCGATGACTCGCACTGGGTCCGCCAGCCTCCAGGCAAGGGGCTGGAGTGGGTGGCAGTTATATGGTTTGATGAATATACTAAATACTATGCAGACTCCGTGAAGGGCCGATTCACCATCTCCAGAGACAATTCCAAGAACACGCTGTATCTGCAAATGAACAACCTGAGAGCCGAGGACGCGGCTGTGTATTACTGTGCGAGAGATCTAATAGCAGCAACTGGGACGGAGACTACTGGGGCCAGGGAACCCTGGTCACCGTCTCCTCA |
| | | | SEQ ID NO: 25257 | SEQ ID NO: 29263 |
| | | AA | DIQMTQSPSSLSASVGDRVTITCRASQGIRNDLGWYQQKPGKAPKRLIYAASSLQGGVPSRFSGSGSGTEFTLTISSLQPEDFATYYCLQHNSYPLTFGGGTKVEIK | QVHLAESGGGVVQPGRSLRLSCEASGFTLSSDDSHWVRQPPGKGLEWVAVIWFDEYTKYYADSVKGRFTISRDNSKNTLYLQMNNLRAEDAAVYYCARDLIAATGTGDYWGQGTLVTVSS |
| | | | SEQ ID NO: 25258 | SEQ ID NO: 29264 |
| **iPS:433965** | **21-225_46F2** | NA | GATATCGTGATGACCCAGACTCCACTCTCTCTGACCGTCACCCCTGGACAGCCGGCCTCCATCTCCTGCAAGTCTAGTCAGAGCCTCCTGCATGGTGATGGAAAGACATATTTGTATTGGTACCTGCAGAAGCCAGGCCAGCCTCCACAGGTCCTGATCTATGAAGTTTCCAATCGGTTCTCTGGAGTGCCAGATAGGTTCAGTGGCAGCGGGTCAGGGACAGATTTCACACTGAAACTCAGCCGGGTGGAGGCTGAGGATGTTGGGGTTTATTACTGCATGCAAAGTATACAGCTTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAATTATATGGTATGATGGAAGTAATAAATACTATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGATACGATTTTTGGAGTGGTTACTTTGACTACTGGGGCCAGGGAACCCTGGTCACCGTCTCCTCA |
| | | | SEQ ID NO: 25259 | SEQ ID NO: 29265 |

FIGURE 50

| | | | | |
|---|---|---|---|---|
| | | AA | DIVMTQTPLSLTVTPGQPASISCKSSQSLLHGDGKTYLYWYLQKPGQPPQVLIYEVSNRFSGVPDRFSGSGSGTDFTLKLSRVEAEDVGVYYCMQSIQLPWTFGQGTKVEIK | QVQLVESGGGVVQPGRSLRLSCAASGFTFSSYGMHWVRQAPGKGLEWVAIIWYDGSNKYYVDSVKGRFTISRDNSKNTLYLQMNSLRAEDTAVYYCARDRYDFWSGYFDYWGQGTLVTVSS |
| | | | SEQ ID NO: 25260 | SEQ ID NO: 29266 |
| iPS:433967 | 21-225_46C3 | NA | GACATCCAGATGACCCAGTCTCCATCCTCCCTGTCTGCATCTGTAGGAGACAGAGTCACCTTCACTTGCCGGGCAAGTCAGGGCATTAGAGATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTAGATCTGGGACAGAATTCACTCTCACAATCAGCAGCCTGCAACCTGAAGATTTTGCAACTTATTACTGTCTACAGCATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGTCATGCACTGGGTCCGCCAGGCTCCAGGCAAGGGGCTGGAGTGGGTGGCAGTTATATGGTATGATGGAAGTAATAAATACTATGCTGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAGGTATAGCAGTGGCTTGTACGACTACGGTATGGACGTCTGGGGCCAAGGGACCACGGTCACCGTCTCCTCA |
| | | | SEQ ID NO: 25261 | SEQ ID NO: 29267 |
| | | AA | DIQMTQSPSSLSASVGDRVTFTCRASQGIRDDLGWYQQKPGKAPKRLIYAASSLQSGVPSRFSGSRSGTEFTLTISSLQPEDFATYYCLQHYSYPRTFGQGTKVEIK | QVQLVESGGGVVQPGRSLRLSCAASGFTFSSYVMHWVRQAPGKGLEWVAVIWYDGSNKYYADSVKGRFTISRDNSKNTLYLQMNSLRAEDTAVYYCARERYSSGLYDYGMDVWGQGTTVTVSS |
| | | | SEQ ID NO: 25262 | SEQ ID NO: 29268 |

FIGURE 50

| | | | | |
|---|---|---|---|---|
| **iPS:433969** | **21-225_46F3** | NA | GACATCCAGATGACCCAGTCTCCATCCTCCCTGTCTGCATCTGTAGGAGACAGAGTCACCATCACTTGCCGGGCAAGTCAGGGCATTAGAAAAGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCAGTCTCACAATCAGCAGCCTGCAGCCTGAAGATTTTGCAACTTATTACTGTCTACAGCATAATAGTTACCCTCTCACTTTCGGCGGAGGGACCAAGGTGGAGATCAAG | CAGGTGCAGCTGGTGGAGTCTGGGGGAGGCGTGGTCCAGCCTGGGAGGTCCCTGAGACTCTCCTGTGCAGCGTCTGGATTCACCTTCAGTGATTATGGCATGCACTGGGTCCGCCAGGCTCCAGGCAAGGGGCTGGAGTGGGTGGCAGTTATATGGTTTGAAGGAAGTAATAAATACTATGCAGATTCCGTGAAGGGCCGATTCACCATCTCCAGAGACAATTCCAAGAACACGCTGTATGTGCAAATGAACAGCCTGAGAGCCGAGGACACGGCTGTGTATTACTGTGTGAGAGAACTAGGATTTTCCAATGACTACTGGGGCCAGGGAACCCTGGTCACCGTCTCCTCA |
| | | | SEQ ID NO: 25263 | SEQ ID NO: 29269 |
| | | AA | DIQMTQSPSSLSASVGDRVTITCRASQGIRKDLGWYQQKPGKAPKRLIYAASSLQSGVPSRFSGSGSGTEFSLTISSLQPEDFATYYCLQHNSYPLTFGGGTKVEIK | QVQLVESGGGVVQPGRSLRLSCAASGFTFSDYGMHWVRQAPGKGLEWVAVIWFEGSNKYYADSVKGRFTISRDNSKNTLYVQMNSLRAEDTAVYYCVRELGFSNDYWGQGTLVTVSS |
| | | | SEQ ID NO: 25264 | SEQ ID NO: 29270 |
| **iPS:433971** | **21-225_46D4** | NA | GACATCCAGATGACCCAGTCTCCATCCTCCCTGTCTGCATCTGTAGGAGACAGAGTCACCATCACTTGCCGGACAAGTCAGGACATTAGAAAAGATTTAGGCTGGTATCAGCAGAAAGTAGGGAAAGCCCCTAAGCGCCTGATCTATGCTGCATCCAGTTTGGAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTTCCCGTGGACGTTCGGCCAAGGGACCAAGGTGGAAATCAAA | CAGGTGCAGCTGGTGGAGTCTGGGGGAGGCGTAGTCCAGCCTGGGAGGTCCCTGAGACTCTCCTGTGCAGCGTCTGGATTCACCTTCAGTAGCTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGTCCCGTATAGCAGCAGTTGGTACGACTACGGTATGGACGTCTGGGGCCAAGGGACCACGGTCACCGTCTCCTCA |
| | | | SEQ ID NO: 25265 | SEQ ID NO: 29271 |

FIGURE 50

| | | | | |
|---|---|---|---|---|
| | | AA | DIQMTQSPSSLSASVGDRVTITCRTSQDIRKDLGWYQQKVGKAPKRLIYAASSLESGVPSRFSGSGSGTEFTLTISSLQPEDFATYYCLQHYSFPWTFGQGTKVEIK | QVQLVESGGGVVQPGRSLRLSCAASGFTFSSYVMHWVRQAPGKGLEWVAVIWYDGSNKYYADSVKGRFTISRDNSKNTLYLQMNSLRAEDTAVYYCARVPYSSSWYDYGMDVWGQGTTVTVSS |
| | | | SEQ ID NO: 25266 | SEQ ID NO: 29272 |
| iPS:433973 | 21-225_46A6 | NA | GACATCCAGATGAACCAGTCTCCATCTTCCGTGTCTGCATCTGTAGGAGACAGAGTCAACATCACTTGTCGGGCGAGTCAGGGTATTAGCAACTGGTTAGCCTGGTATCAGCAGAAACCAGGGAAAGTCCCTAAGCTCCTGATCTATGCTGCATCCAGTTTGCAAAGTGGGGTCCCATCAAGGTTCAGCGGCAGTGGATCTGGGACAGATTTCACTCTCACCATCAGCAACCTGCAGCCTGAAGATTTTGCAACTTACTATTGTCAACAGACTAACAGTTTCCCGTGGACGTTCGGCCAAGGGACCAAGGTGGAAATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTCGTGGTATTAACACATTCGACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAGGAAAGGAGTGGGAGCTATTTTGACTACTGGGGCCAGGGAACCCTGGTCACCGTCTCCTCA |
| | | | SEQ ID NO: 25267 | SEQ ID NO: 29273 |
| | | AA | DIQMNQSPSSVSASVGDRVNITCRASQGISNWLAWYQQKPGKVPKLLIYAASSLQSGVPSRFSGSGSGTDFTLTISNLQPEDFATYYCQQTNSFPWTFGQGTKVEIK | EVQLLESGGGLVQPGGSLRLSCAASGFTFSSYAMSWVRQAPGKGLEWVSAISGRGINTFDADSVKGRFTISRDNSKNTLYLQMNSLRAEDTAVYYCAKERSGSYFDYWGQGTLVTVSS |
| | | | SEQ ID NO: 25268 | SEQ ID NO: 29274 |

FIGURE 50

| | | | | |
|---|---|---|---|---|
| **iPS:433975** | **21-225_46C6** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCTCTCACTTTCGGCGGAGGGACCAAGGTGGAGATCACA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATACACTGGGTCCGCCAGGCTCCAGGCAAGGGGCTGGAGTGGGTGGCAGTTATATGGTATGATGAAAATAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGCTAGGATTTTCCAATGACTACTGGGGCCAGGGAACCCTGGTCACCGTCTCCTCA |
| | | | SEQ ID NO: 25269 | SEQ ID NO: 29275 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVEIT | QVQLVESGGGVVQPGRSLRLSCAASGFTFSSYGIHWVRQAPGKGLEWVAVIWYDENNKYYADSVKGRFTISRDNSKNTLYLQMNSLRAEDTAVYYCARELGFSNDYWGQGTLVTVSS |
| | | | SEQ ID NO: 25270 | SEQ ID NO: 29276 |
| **iPS:433977** | **21-225_46D8** | NA | GACATCCAGATGACCCAGTCTCCATCCTCCCTGTCTGCATCTGTAGGAGACAGAGTCACCATCACTTGCCGGGCAAGTCAGGGCATTAGAAAAGATTTAGGCTGGTATCAGCAGAAACCAGGGAAAGCCCCTAAGCGCCTGATCTATGCTGCATCCAGTTTGCAAAGTGGGGTCCCATCAAGGTTCAGTGGCAGTGGATCTGGGACAGAATTCAGTCTCACAATCAGCAGCCTGCAGCCTGAAGATTTTGCAACTTATTACTGTCTACAGCATAATAGTTACCCTCTCACTTTCGGCGGAGGGACCAAGGTGGAGATCAAG | CAGGTGCAGCTGGTGGAGTCTGGGGGAGGCGTGGTCCAGCCTGGGAGGTCCCTGAGACTCTCCTGTGCAGCGTCTGGATTCACCTTCAGTGATTATGGCATACACTGGGTCCGCCAGGCTCCAGGCAAGGGGCTGGAGTGGGTGGCAGTTATATGGTTTGAAGGAAGTAATAAATACTATGCAGATTCCGTGAAGGGCCGATTCACCATCTCCAGAGACAATTCCAAGAACACGCTGTATGTGCAAATGAACAGCCTGAGAGCCGAGGACACGGCTGTGTATTACTGTGTGAGAGAACTAGGATTTTCCAATGACTACTGGGGCCAGGGAACCCTGGTCACCGTCTCCTCA |
| | | | SEQ ID NO: 25271 | SEQ ID NO: 29277 |

FIGURE 50

| | | | | |
|---|---|---|---|---|
| | | AA | DIQMTQSPSSLSASVGDRVTITCRASQGIRKDLGWYQQKPGKAPKRLIYAASSLQSGVPSRFSGSGSGTEFSLTISSLQPEDFATYYCLQHNSYPLTFGGGTKVEIK | QVQLVESGGGVVQPGRSLRLSCAASGFTFSDYGIHWVRQAPGKGLEWVAVIWFEGSNKYYADSVKGRFTISRDNSKNTLYVQMNSLRAEDTAVYYCVRELGFSNDYWGQGTLVTVSS |
| | | | SEQ ID NO: 25272 | SEQ ID NO: 29278 |
| iPS:433979 | 21-225_46B9 | NA | GATATTGTGATGACCCAGACTCCACTCTCTCTGTCCGTCACCCCTGGACAGCCGGCCTCCATCTCCTGCAAGTCTAGTCAGAGCCTCCTGCATAGTGAGGGAAAGACCTATTTGTATTGGTACCTGCAGAAGCCAGGCCAGCCTCCACAGCTCCTGATCTATGAAGTTTCTTACCGGTTCTCTGGAGTGCCAGATAGGTTCAGTGGCAGCGGGTCAGGGACAGATTTCACACTGAAAATCAGCCGGATGGAGGCTGAGGATGTTGGGGTTTATTACTGCATGCACAGTATACAGTATCCGCTCACGTTTGGCGGAGGGACCAAGGTGGAGATCCAA | CAGGTGCAGCTGGTGGAGTCTGGGGGAGGCGTGGTCCAGCCTGGGAGGTCCCTGAGACTCTCCTGTTCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGAAATAAATACTATGCAGACTCCGTGAAGGGCCGAATCACCATCTCCAGAGACAATTCCAAGAACACGCTGTATCTGCAAATGAACAGCCTGAGAGCCGAGGACACGGCTGTGTATTACTGTGCGAGGCGGTATAGCAGCAGCTGGATGGGAGGTATGGACGTCTGGGGCCAAGGGACCACGGTCACCGTCTCCTCA |
| | | | SEQ ID NO: 25273 | SEQ ID NO: 29279 |
| | | AA | DIVMTQTPLSLSVTPGQPASISCKSSQSLLHSEGKTYLYWYLQKPGQPPQLLIYEVSYRFSGVPDRFSGSGSGTDFTLKISRMEAEDVGVYYCMHSIQYPLTFGGGTKVEIQ | QVQLVESGGGVVQPGRSLRLSCSASGFTFSSYGMHWVRQAPGKGLEWVAVIWYDGRNKYYADSVKGRITISRDNSKNTLYLQMNSLRAEDTAVYYCARRYSSSWMGGMDVWGQGTTVTVSS |
| | | | SEQ ID NO: 25274 | SEQ ID NO: 29280 |

FIGURE 50

| | | | | |
|---|---|---|---|---|
| **iPS:433981** | **21-225_46E9** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAGTCAGCAGCCTGCAGCCTGAAGATTTTGCAACTTATTTCTGTCTTCAGCATACTAGTTTCCCATTCACTTTCGGCCCTGGGACCAAAGTGGATATCAAA | CAGGTGCAGCTGGTGGAGTCTGGGGGAGGCTTGGTCAAGCCTGGAGGGTCCCTGAGACTCTCCTGTGCAGCCTCTGGATTCACCTTCAGTGACTACTACATGAACTGGATCCGCCAGGCTCCAGGGAAGGGGCTGGAGTGGGTTTCATACATTAATAGTAATGGTTTTACCATATACTACGCAGACTCTGTGAAGGGCCGATTCACCATCTCCAGGGACAACGCCAAGAACTCACTGTATCTGCAAATGAACAGCCTGAGAGCCGAGGACACGGCCGTGTATTACTGTGCGGCCGATACAATCTACTGGGGCCAGGGAACCCTGGTCACCGTCTCCTCA |
| | | | SEQ ID NO: 25275 | SEQ ID NO: 29281 |
| | | AA | DIQMTQSPSSLSASVGDRVTITCRASQGIRNDLGWYQQKPGKAPKRLIYAASSLQSGVPSRFSGSGSGTEFTLTVSSLQPEDFATYFCLQHTSFPFTFGPGTKVDIK | QVQLVESGGGLVKPGGSLRLSCAASGFTFSDYYMNWIRQAPGKGLEWVSYINSNGFTIYYADSVKGRFTISRDNAKNSLYLQMNSLRAEDTAVYYCAADTIYWGQGTLVTVSS |
| | | | SEQ ID NO: 25276 | SEQ ID NO: 29282 |
| **iPS:433983** | **21-225_47A1** | NA | GACATCCAGATGACCCAGTCTCCATCCTCCCTGTCTGCATCTGTAGGAGACAGAGTCACCATCACTTGCCGGGCAAGTCAGGACATTAGAAATGATTTAGGCTGGTATCAGCAGAAACCAGGGAAAGCCCCTAAGCGCCTGATCTATGCTGCATTCAGTTTGCAAAGTGGGGTCCCATCAAGGTTCAGCGGCAGTAGATCTGGGACAGAATTCACTCTCACAATCAGCAGCCTGCAGCCTGAAGATTTTGCAACTTATTACTGTCTGCAACATAATAGTTACCCGCTCACTTTCGGCGGAGGGACCAAGGTGGAGATCAAA | CAGGTGCAGCTGGTGGAGTCGGGGGGAGGCGTGGTCCAGCCTGGGAGGTCCCTGAGACTCTCCTGTGCAGCGTCTGGATTCACCTTCAGTGACTATGGCATGCACTGGGTCCGCCAAGCTCCAGGCAAGGGGCTGGAGTGGGTGGCAGTTATATGGTATGATGACTATAATAAAAAGTATGCAGACTCCGTGAAGGGCCGATTCACCATCTCCAGAGACAATGCCAAGAACACGCTGTATCTGCAAGTGAACAGCCTGAGAGTCGAAGACACGGCTGTGTATTACTGTGCGACAGAACTGGGGATGCTCTTTGACTACTGGGGCCAGGGAACCCTGGTCACCGTCTCCTCA |
| | | | SEQ ID NO: 25277 | SEQ ID NO: 29283 |

FIGURE 50

| | | | | |
|---|---|---|---|---|
| | | AA | DIQMTQSPSSLSASVGDRVTITCRASQDIRNDLGWYQQKPGKAPKRLIYAAFSLQSGVPSRFSGSRSGTEFTLTISSLQPEDFATYYCLQHNSYPLTFGGGTKVEIK | QVQLVESGGGVVQPGRSLRLSCAASGFTFSDYGMHWVRQAPGKGLEWVAVIWYDDYNKKYADSVKGRFTISRDNAKNTLYLQVNSLRVEDTAVYYCATELGMLFDYWGQGTLVTVSS |
| | | | SEQ ID NO: 25278 | SEQ ID NO: 29284 |
| iPS:433985 | 21-225_47C1 | NA | GATATTGTGATGACCCAGACTCCACTCTCTCTGTCCGTCACCCCTGGACAGCCGGCCTCCATCTCCTGCACGTCTAGTCAGAGCCTCCTGCATAGTGAAGGAAAGACCTATTTGTATTGGTACCTCCAGAAGCCAGGCCAGCCTCCACAGCTCCTGATCTATGAAGTTTCCAACCGGTTCTCTGGAGTGCCAGATAGGTTCAGTGGCAGCGGGTCAGGGACAGATTTCACACTAAAAATCAGCCGGGTGGAGGCTGAGGATGTTGGGGTTTTTTACTGTATGCAAAGTATACAGCTTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGACTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTTTCTGCAAATGAACAGCCTGAGAGACGAGGACACGGCTGTGTATTACTGTGCGAGACGGTATAGCCGCAGCTGGGTGGGAGGTATGGACGTCTGGGGCCAAGGGACCACGGTCACCGTCTCCTCA |
| | | | SEQ ID NO: 25279 | SEQ ID NO: 29285 |
| | | AA | DIVMTQTPLSLSVTPGQPASISCTSSQSLLHSEGKTYLYWYLQKPGQPPQLLIYEVSNRFSGVPDRFSGSGSGTDFTLKISRVEAEDVGVFYCMQSIQLPWTFGQGTKVEIK | QVQLVESGGGVVQPGRSLRLSCAASGFTFSDYGMHWVRQTPGKGLEWVAVIWYDGSNKYYADSVKGRFTISRDNSKNTLFLQMNSLRDEDTAVYYCARRYSRSWVGGMDVWGQGTTVTVSS |
| | | | SEQ ID NO: 25280 | SEQ ID NO: 29286 |

FIGURE 50

| | | | | |
|---|---|---|---|---|
| **iPS:433987** | **21-225_47A5** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACGATGACACACACTGGGTCCGCCAGGCTCCAGGCAAGGGGCTGGAGTGGGTGGCAGTTATATGGTTTGATGGAAGTAATAAATACTATGCAGACTCCGTGAAGGGCCGATTCACCATCTCCAGAGACAATTCCAAGAACACGCTGTATCTGCAAATGAACAGCCTGAGCGCCGAGGACACGGCTGTGTATTACTGTGCGAGAGATCTTATAGCAGCAGCTGGTACAGTTGACTACTGGGGCCAGGGAACCCTGGTCACCGTCTCCTCA |
| | | | SEQ ID NO: 25281 | SEQ ID NO: 29287 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVEIK | QVQLVESGGGVVQPGRSLRLSCAASGFTFSDDDTHWVRQAPGKGLEWVAVIWFDGSNKYYADSVKGRFTISRDNSKNTLYLQMNSLSAEDTAVYYCARDLIAAAGTVDYWGQGTLVTVSS |
| | | | SEQ ID NO: 25282 | SEQ ID NO: 29288 |
| **iPS:433989** | **21-225_47C7** | NA | GATATTGTGATGACTCAGTCTCCACTCTCCCCGCCCGTCACCCCTGGAGAGCCGGCCTCCATCTCCTGCAGGTCTAGTCAGAGCCTCCTGCATAGTAATGGATACAACTATTTGGAATGGTACCTGCAGAAGTCAGGGCAGTCTCCACAGTTCCTGATCTATTTGGGTTTTAATCGGGCCTCCGGGGTCCCTGACAGGTTCACTGGCAGTGGATCAGGCACAGATTTTACACTGAAAATCAGCAGAGTGGAGGCTGAGGATGTTGGGGTTTATTACTGCATGCAAGTTCTACAAACTCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGTAACTATGCCATGAGCTGGGTCCGCCAGGCTCCAGGGAAGGGGCTGGAGTGGGTCTCAGGTATTAGTGGTAGTGGTAGTCGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAAGATCGGGGGCAGTGGCTCATAGGCGGTATGGACGTCTGGGGCCAAGGGACCACGGTCACCGTCTCCTCA |
| | | | SEQ ID NO: 25283 | SEQ ID NO: 29289 |

FIGURE 50

| | | | | |
|---|---|---|---|---|
| | | AA | DIVMTQSPLSPPVTPGEPASISCRSSQSLLHSNGYNYLEWYLQKSGQSPQFLIYLGFNRASGVPDRFTGSGSGTDFTLKISRVEAEDVGVYYCMQVLQTPFTFGPGTKVDIK | EVQLLESGGGLVQPGGSLRLSCAASGFTFSNYAMSWVRQAPGKGLEWVSGISGSGSRTYYADSVKGRFTISRDNSKNTLYLQMNSLRAEDTAVYYCAKDRGQWLIGGMDVWGQGTTVTVSS |
| | | | SEQ ID NO: 25284 | SEQ ID NO: 29290 |
| **iPS:433991** | **21-225_47E7** | NA | GATATTGTGATGACCCAAACTCCACTCTCTCTGTCCGTCACCCCTGGACAGCCGGCCTCCATCTCCTGCAAGTCTAGTCAGAGCCTCCTGCATAGTGATGGAAGGACCTATTTGTATTGGTACCTGCAGAAGCCAGGCCAGCCTCCACAGCTCCTGATCTATGAAGTTTCCAGCCGGTTCTCTGGAGTGCCAGATAGGTTCAGTGGCAGCGGGTCAGGGACAGATTTCGCACTGAAAATCAGCCGGGTGGAGGCTGAGGATGTTGGGGTTTATTACTGCATGCAAAGTACACAACTTCCGTGGACGTTCGGCCAAGGGACCAAGGCGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTATCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTTCAAATGAACAGCCTGAGAGCCGAGGACACGGCTGTGTATTACTGTGCGAGACGGTATAGCAGAAGCTGGGTGGGAGGTATGGACGTCTGGGGCCAAGGGACCACGGTCACCGTCTCCTCA |
| | | | SEQ ID NO: 25285 | SEQ ID NO: 29291 |
| | | AA | DIVMTQTPLSLSVTPGQPASISCKSSQSLLHSDGRTYLYWYLQKPGQPPQLLIYEVSSRFSGVPDRFSGSGSGTDFALKISRVEAEDVGVYYCMQSTQLPWTFGQGTKAEIK | QVQLVESGGGVVQPGRSLRLSCAASGFTFSIYGMHWVRQAPGKGLEWVAVIWYDGSNKYYADSVKGRFTISRDNSKNTLYLQMNSLRAEDTAVYYCARRYSRSWVGGMDVWGQGTTVTVSS |
| | | | SEQ ID NO: 25286 | SEQ ID NO: 29292 |

FIGURE 50

| | | | | |
|---|---|---|---|---|
| **iPS:433993** | **21-225_47G7** | NA | GACATCCAGATGACCCAGTCTCCATCTTCCGTGTCTGCATCTGTAGGAGACAGAGTCACCATCACTTGTCGGGCGAGTCAGGGTATTAGCAACTGGTTAGCCTGGTATCAGCAGAAACCAGGGAAAGCCCCTAAGCTCCTGATCTTTGCTGCCTCCAATTTGCAAAGTGGGGTCCCATCAAGGTTCAGCGGCAGTGGATCTGGGACAGATTTCACTCTCACCATCAGCAGTCTGCAGCCTGCAGATTTTGCAACTTACTGTTGTCAACAGGTTAACAGTTTCCCGTGGACGTTCGGCCAAGGGACCAAGGTGGAAATCAAA | GAGGTGCAGTTGTTGGACTCTGGGGGAGGCTTGGTGCAGCCTGGGGGGTCCCTGAGACTCTCCTGTGCAGCCTCTGGATTCACCTTTAGCAGCTATGCCATGAGTTGGGTCCGCCAGGCTCCAGGGAAGGGGCTGGAGTGGGTCTCAGCTATTAGTGGTCGTGGTGGTAACACATTCTACGCAGAGTCCGTGAGGGGCCGGTTCACCATCTCCAGAGACAATTCCAAGAACACGCTGTATCTGCAAATGAACAGCCTGAGAGCCGAGGACACGGCCGTATATTACTGTGCGAAGATTATCCGGGAGCAGTGGGCCTTTGACTACTGGGGCCAGGGAACCCTGGTCACCGTCTCCTCA |
| | | | SEQ ID NO: 25287 | SEQ ID NO: 29293 |
| | | AA | DIQMTQSPSSVSASVGDRVTITCRASQGISNWLAWYQQKPGKAPKLLIFAASNLQSGVPSRFSGSGSGTDFTLTISSLQPADFATYCCQQVNSFPWTFGQGTKVEIK | EVQLLDSGGGLVQPGGSLRLSCAASGFTFSSYAMSWVRQAPGKGLEWVSAISGRGGNTFYAESVRGRFTISRDNSKNTLYLQMNSLRAEDTAVYYCAKIIREQWAFDYWGQGTLVTVSS |
| | | | SEQ ID NO: 25288 | SEQ ID NO: 29294 |
| **iPS:433995** | **21-225_47H7** | NA | GACATCCAGATGACCCAGTCTCCATCCTCCCTGTCTGCATCTGTAGGAGACAGAGTCACCATCACTTGCCGGACAAGTCAGGGCATTAGAAATGATTTAGGCTGGTATCAGAAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAGTCAGCAGCCTGCAGCCTGAAGATTTTGCAACTTATTTCTGTCTACAACATACTAGTTTCCCATTCACTTTCGGCCCTGGGACCAAAGTGGATATCAAA | CAGGTGCAGCTGGTGGAGTCTGGGGGAGGCTTGGTCAAGCCTGGAGGGTCCCTGAGACTCTCCTGTGCAGCCTCTGGATTCACCTTCAGTGACTACTACATGATCTGGATCCGCCAGGCTCCAGGGAAGGGGCTGGAGTGGGTTTCATACATTAATAGTAATGGTTTTACCAAATACTACGCAGACTCTGTGAAGGGCCGATTCACCATCTCCAGGGACAACGCCAAGAATTCACTGTATCTGCAAATGAACAGTCTGAGAGCCGAGGACACGGCCGTGTATTACTGTGCGGCCGATACAGTCTACTGGGGCCAGGGAACCCTGGTCACCGTCTCCTCA |
| | | | SEQ ID NO: 25289 | SEQ ID NO: 29295 |

FIGURE 50

| | | | | |
|---|---|---|---|---|
| | | AA | DIQMTQSPSSLSASVGDRVTITCRTSQGIRNDLGWYQKKPGKAPKRLIYAASSLQSGVPSRFSGSGSGTEFTLTVSSLQPEDFATYFCLQHTSFPFTFGPGTKVDIK | QVQLVESGGGLVKPGGSLRLSCAASGFTFSDYYMIWIRQAPGKGLEWVSYINSNGFTKYYADSVKGRFTISRDNAKNSLYLQMNSLRAEDTAVYYCAADTVYWGQGTLVTVSS |
| | | | SEQ ID NO: 25290 | SEQ ID NO: 29296 |
| iPS:433997 | 21-225_48C1 | NA | GACATCCAGATGACCCAGTCTCCATCCTCCCTGTCTGCATCTGTAGGAGACAGAGTCACCATCACTTGCCGGACAAGTCAGGGCATTAGAAATGATTTAGGCTGGTATCAGCAGAAACCAGGGAAAGCCCCTAAGCGCCTGATCTATAGAGCATCCAGTTTGCAAAGTGGGGTCCCAGCAAGGTTCAGCGGCAGTGGATCTGGGACAGAATTCACTCTTACAATCAGCAGCCTGCAGCCTGAAGATTTTGCAATTTATTACTGTCTACAGCATAATTTTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTGTATGGTATGATGAAATTAATAAAAAGTATGCAGACTCCGTGAAGGGCCGAGTCACCATCTCCAGAGACAATTCCAAGAACACGCTGTATCTGCAAATGAACAGCCTGAGAGCCGAGGACACGGCTATGTATTACTGTGCGAGAGAATTAGGGTGGGAGGCTGACTACTGGGGCCAGGGAACCCTGGTCACCGTCTCCTCA |
| | | | SEQ ID NO: 25291 | SEQ ID NO: 29297 |
| | | AA | DIQMTQSPSSLSASVGDRVTITCRTSQGIRNDLGWYQQKPGKAPKRLIYRASSLQSGVPARFSGSGSGTEFTLTISSLQPEDFAIYYCLQHNFYPWTFGQGTKVEIK | QVQLVESGGGVVQPGRSLRLSCAASGFTFSSYGMHWVRQAPGKGLEWVAVVWYDEINKKYADSVKGRVTISRDNSKNTLYLQMNSLRAEDTAMYYCARELGWEADYWGQGTLVTVSS |
| | | | SEQ ID NO: 25292 | SEQ ID NO: 29298 |

FIGURE 50

| | | | | |
|---|---|---|---|---|
| **iPS:433999** | **21-225_48D1** | NA | GACATCCAGATGACCCAGTCTCCATCCTCCCT GTCTGCGTCTGTAGGAGACAGAGTCAACATCA CTTGCCGGGCAAGTCAGAGCATTAGCAGCTAT TTAATTTGGTATCAGCAGAAACCAGGGAAAGC CCCTAAGCTCCTGATCTATGCTGCATCCAGTTT GCAAAGTGGGGTCCCATCAAGGTTCAGTGCCA GTGGATCTGGGACAGATTTCACTCTCACCATC AGCAGTCTGCAACCTGAAGATTTTGCAAGTTA CTACTGTCAACAGAGTAACAGTATTCCATTCA CTTTCGGCCCTGGGACCAAAGTGGATATCAAA | GAGGTGCAGCTGTTGGAGTCTGGGGGAGGCTTG GTACAGCCTGGGGGGTCCCTGAGACTCTCCTGTG CAGCCTCTAGATTCACCTTTAGCAGTTATGCCAT GAGCTGGGTCCGCCAGGCTCCAGGGAAGGGGCT GGAGTGGGTCTCAGTTATTAGTGGTCGTGGTGGT AGCACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTATATTACTGTGCGCGTCGTATAGCA GTGGCTGGGAATGAGGCTTTTGATATCTGGGGCC AAGGGACAATGGTCACCGTCTCTTCA |
| | | | SEQ ID NO: 25293 | SEQ ID NO: 29299 |
| | | AA | DIQMTQSPSSLSASVGDRVNITCRASQSISSYLIW YQQKPGKAPKLLIYAASSLQSGVPSRFSASGSGT DFTLTISSLQPEDFASYYCQQSNSIPFTFGPGTKV DIK | EVQLLESGGGLVQPGGSLRLSCAASRFTFSSYAMS WVRQAPGKGLEWVSVISGRGGSTFYADSVKGRFTI SRDNSKNTLYLQMNSLRAEDTAVYYCARRIAVAG NEAFDIWGQGTMVTVSS |
| | | | SEQ ID NO: 25294 | SEQ ID NO: 29300 |
| **iPS:434001** | **21-225_48F2** | NA | GACATCCAGATGACCCAGTCTCCATCCTCCCT GTCTGCATCTGTAGGAGACAGAGTCACCATCA CTTGCCGGGCAAGTCGGGGCATTAGAGATGAT TTAGGCTGGTATCAACAGAAACCAGGGAAAC CCCCTAAGCGCCTGATCTATGCTGCATCCAGT TTGCAAAGTGGGGTCCCATCAAGGTTCAACGG CAGTGGATCTGGGACAGAATTCACTCTCACAA TCAGCAGCCTGCAGTCTGAAGATCTTGCAACT TATTACTGTCTACAGCAATATAGTTATCCTCGG ACGTTCGGCCAAGGGACCAAGGTGGAAATCA AA | CAGGTGCAGCTGGTGGAGTCTGGGGGAGGCGTG GTCCAGCCTGGGAGGTCTCTGAGACTCTCCTGTG CAGCGTCTGGATTCACCTTCAGTAACTATGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGAGGTA TAGCAGCAGCTGGTACGACTACGGTCTGGACGTC TGGGGCCAAGGGACCACGGTCACCGTCTCCTCA |
| | | | SEQ ID NO: 25295 | SEQ ID NO: 29301 |

FIGURE 50

| | | | | |
|---|---|---|---|---|
| | | AA | DIQMTQSPSSLSASVGDRVTITCRASRGIRDDLGWYQQKPGKPPKRLIYAASSLQSGVPSRFNGSGSGTEFTLTISSLQSEDLATYYCLQQYSYPRTFGQGTKVEIK | QVQLVESGGGVVQPGRSLRLSCAASGFTFSNYVMHWVRQAPGKGLEWVAVIWYDGSNKYYADSVKGRFTISRDNSKNTLYLQMNSLRAEDTAVYYCARERYSSSWYDYGLDVWGQGTTVTVSS |
| | | | SEQ ID NO: 25296 | SEQ ID NO: 29302 |
| **iPS:434003** | **21-225_48C3** | NA | GACATCCAGATGACCCAGTCTCCATCCTCCCTGTCTGCGTCTGTAGGAGACAGAGTCAACATCACTTGCCGGGCAAGTCAGAGCATTATCAGCTATTTAATTTGGTATCAGCAGAAACCAGGGAAAGCCCCTAGGCTCCTGATCTATGCTGCATCCAGTTTGCAAAGTGGGGTCCCATCAAGGTTCAGTGCCAGTGGATCTGGGACAGATTTCACTCTCACCATCAGCAGTCTGCAACCTGAAGATTTTGCAAGTTACTACTGTCAACAGACTAACAGTATTCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTAGATTCACCTTTAGCAGTTATGCCATGAGCTGGGTCCGCCAGGCTCCAGGGAAGGGGCTGGAGTGGGTCTCAGTTATTAGTGGTCGTGGTGGTAGCACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGCGTCGTATAGCAGTGGCTGGGAATGAGGCTTTTGATATCTGGGGCCAAGGGACAATGGTCACCGTCTCTTCA |
| | | | SEQ ID NO: 25297 | SEQ ID NO: 29303 |
| | | AA | DIQMTQSPSSLSASVGDRVNITCRASQSIISYLIWYQQKPGKAPRLLIYAASSLQSGVPSRFSASGSGTDFTLTISSLQPEDFASYYCQQTNSIPFTFGPGTKVDIK | EVQLLESGGGLVQPGGSLRLSCAASRFTFSSYAMSWVRQAPGKGLEWVSVISGRGGSTFYADSVKGRFTISRDNSKNTLYLQMNSLRAEDTAVYYCARRIAVAGNEAFDIWGQGTMVTVSS |
| | | | SEQ ID NO: 25298 | SEQ ID NO: 29304 |

FIGURE 50

| | | | | |
|---|---|---|---|---|
| **iPS:434007** | **21-225_48D7** | NA | GACATCCAGATGACCCAGTCTCCATCTTCCGTGTCTGCATCTGTAGGAGACAGAGTCACCATCACTTGTCGGGCGAGTCAAAATATTACCAGCTGGTTAGCCTGGTATCAGCAGAAACCAGGGAAAGCCCCTAAGCTCCTGATCTATAGTGCATCCAGTTTGCAAAATGGGGTCCCATCAAGGTTCAGCGGCAGTGGATCTGGGACAGATTTCACTCTCACCATCAGCAGCCTGCAGCCTGAAGATTTTGTAACTTACTGTTGTCAACAGGCTAACAGTTTCCCGTGGACGTTCGGCCAAGGGACCAAGGTGGAAATCAAA | GAGGTGCAGCTGTTGGAGTCTGGGGGAGGCTTGGTACAGCCGGGGGGGTCCCTGAGACTCTCCTGTGCAGCCTCTGGATTCACCTTTAGAAACTCTGCCATGAACTGGGTCCGCCAGGCTCCAGGGAAGGGGCTGGAGTGGGTCTCAGCTATTAGTGGTAGTGGTGGTACCACATTCTACGCAGACTCCGTGAAGGGCCGGTTCACCATCTCCAGAGACAATTCCAAGAACACGTTGTATCTGCAAATAAACAGCCTGAGAGCCGAGGACACGGCCGTATATTACTGTGCGAAATGTGGGCGGGAGCAGTGGCTTGACTACTGGGGCCAGGGAACCCTGGTCACCGTCTCCTCA |
| | | | SEQ ID NO: 25299 | SEQ ID NO: 29305 |
| | | AA | DIQMTQSPSSVSASVGDRVTITCRASQNITSWLAWYQQKPGKAPKLLIYSASSLQNGVPSRFSGSGSGTDFTLTISSLQPEDFVTYCCQQANSFPWTFGQGTKVEIK | EVQLLESGGGLVQPGGSLRLSCAASGFTFRNSAMNWVRQAPGKGLEWVSAISGSGGTTFYADSVKGRFTISRDNSKNTLYLQINSLRAEDTAVYYCAKCGREQWLDYWGQGTLVTVSS |
| | | | SEQ ID NO: 25300 | SEQ ID NO: 29306 |
| **iPS:434009** | **21-225_48A9** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATATTGCATCCAGTTTGCAAAGTGGGGTCCCATCAAGGTTCAGCGGCAGTGGATCTGGGACAGAATTCACTCTCACTATCAGCGGCTTGCAGCCTGAAGATTTTGCAATTTATTACTGTCTACAGCATAATCGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCCCCAGGCAAGGGGCTGGAGTGGGTGGCAGTTATATGGTATGAGGAAAATAAGAAATACTATGCAGACTCCGTGAAGGGCCGATTCACCATCTCCAGAGACAATTCCAAGAACACGCTGTTTCTGCAAATGAATAGCCTGAGAGCCGAGGACACGGCTATGTATTTCTGTGCGAGAGAACTTGCCTGGTACGAGGACTACTGGGGCCAGGGAACCCTGGTCACCGTCTCCTCA |
| | | | SEQ ID NO: 25301 | SEQ ID NO: 29307 |

FIGURE 50

| | | | | |
|---|---|---|---|---|
| | | AA | DIQMTQSPSSLSASVGDRVTITCRASQGIRNDLGWYQQKPGKAPKRLIYIASSLQSGVPSRFSGSGSGTEFTLTISGLQPEDFAIYYCLQHNRYPWTFGQGTKVEIK | QVQLVESGGGVVQPGRSLRLSCAASGFTFSSYGMHWVRQAPGKGLEWVAVIWYEENKKYYADSVKGRFTISRDNSKNTLFLQMNSLRAEDTAMYFCARELAWYEDYWGQGTLVTVSS |
| | | | SEQ ID NO: 25302 | SEQ ID NO: 29308 |
| **iPS:434011** | **21-225_48B10** | NA | GACATCCAGATGACCCAGTCTCCATCCTCCCTGTCTGCGTCTGTAGGAGACAGAGTCACCATCACTTGCCGGGCAAGTCAGAGCATTAGGAAGTATTTAAATTGGTATCAGAAGACACCAGGGAAAGCCCCTAAACTCTTGATCTATGCTGCTTCCAGTTTGCAAAGTGGGGTCCCATCAAGGTTCAGTGGCAGTGGATCTGGGACAGATTTCACTCTCACCATCAGCAGTGTGCAACCTGAAGATTTTGCAACTTACTACTGTCAACAGACTTACAGTAACCCACTCACTTTCGGCGGAGGGACCAAGGTGGAGTTCACA | CAGGTGCAGCTGGTGGAGTCTGGGGGAGGCTTGGTCAAGCCTGGAGGGTCCCTGAGACTCTCCTGTGCAGCCTCTGGATTCACCTTCAGTGACTACTTCATGACCTGGATCCGCCAGGCTCCAGGGCAGGGGCTGGAGTGGGTTTCATACATTAGTAGTGCTGGTGGTGCCATATACTACGCAGACTCTGTGAAGGGCCGATTCACCATCTCCAGGGACAACGCCAAAAACTCACTGTATCTGCAAATGAACAGCCTGAGAGCCGAGGACACGGCCGTGTATTACTGTGCAATAGCAGTGGCTGCCCCTGGTGTTTTTGATATCTGGGGCCAAGGGACAATGGTCACCGTCTCTTCA |
| | | | SEQ ID NO: 25303 | SEQ ID NO: 29309 |
| | | AA | DIQMTQSPSSLSASVGDRVTITCRASQSIRKYLNWYQKTPGKAPKLLIYAASSLQSGVPSRFSGSGSGTDFTLTISSVQPEDFATYYCQQTYSNPLTFGGGTKVEFT | QVQLVESGGGLVKPGGSLRLSCAASGFTFSDYFMTWIRQAPGQGLEWVSYISSAGGAIYYADSVKGRFTISRDNAKNSLYLQMNSLRAEDTAVYYCAIAVAAPGVFDIWGQGTMVTVSS |
| | | | SEQ ID NO: 25304 | SEQ ID NO: 29310 |

FIGURE 50

| | | | | |
|---|---|---|---|---|
| **iPS:434013** | **21-225_48D12** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCACTTTGCAAAGTGGGGTCCCATCAAGGATCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAGGGGCCTGGAGTGGGTGGCAGTTATATGGTATGATGTAAGTAATAAATACTATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGATGAGATCTGACTACTGGGGCCAGGGAACCCTGGTCACCGTCTCCTCA |
| | | | SEQ ID NO: 25305 | SEQ ID NO: 29311 |
| | | AA | DIQMTQSPSSLSASVGDRVTITCRASQGIRNDLGWYQQKPGKAPKRLIYAASTLQSGVPSRISGSGSGTEFTLTISSLQPEDFATYYCLQHNSYPLTFGGGTKVEIK | QVQLVESGGGVVQPGRSLRLSCAASGFTFSDYGMHWVRQAPGRGLEWVAVIWYDVSNKYYVDSVKGRFTISRDNSKNTLYLQMNSLRAEDTAVYYCARELGMRSDYWGQGTLVTVSS |
| | | | SEQ ID NO: 25306 | SEQ ID NO: 29312 |
| **iPS:434015** | **21-225_48F12** | NA | GACATCCAGATGACCCAGTCTCCATCCTCCCTGTCTGCGTCTGTAGGAGACAGAGTCACCATCACTTGCCGGGCAAGTCAGAGCATTAGGAAGTATTTAAATTGGTATCAGAAGACACCAGGGAAAGCCCCTAAACTCTTGATCTATGCTGCTTCCAGTTTGCAAAGTGGGGTCCCATCAAGGTTCAGTGGCAGTGGATCTGGGACAGATTTCACTCTCACCATCAGCAGTCTGCAACCTGAAGATTTTGCAAATTATTACTGTCAACAGACTTACAGTAACCCGCTCACTTTCGGCGGAGGGACCGAGGTGGAGATCACA | CAGGTGCAGCTGGTGGAGTCTGGGGGAGGCTTGGTCAAGCCTGGAGGGTCCCTGAGACTCTCCTGTGCAGCCTCTGGATTCACCTTCAGTGACTACTTCATGACCTGGATCCGCCAGGCTCCAGGGCAGGGGCTGGAGTGGGTTTCATACATTAGTAGTGCTGGTGGTGCCATATACTACGCAGACTCTGTGAAGGGCCGATTCACCATCTCCAGGGACAACGCCAAGAACTCACTGTTTCTGCAAATGAACAGCCTGAGAGCCGAGGACACGGCCGTGTATTACTGTGCTATAGCAGTGGCTGCCCCTGGTGCTTTTGATATCTGGGGCCAAGGGACATTGGTCACCGTCTCTTCA |
| | | | SEQ ID NO: 25307 | SEQ ID NO: 29313 |

FIGURE 50

| | | | | |
|---|---|---|---|---|
| | | AA | DIQMTQSPSSLSASVGDRVTITCRASQSIRKYLNWYQKTPGKAPKLLIYAASSLQSGVPSRFSGSGSGTDFTLTISSLQPEDFANYYCQQTYSNPLTFGGGTEVEIT | QVQLVESGGGLVKPGGSLRLSCAASGFTFSDYFMTWIRQAPGQGLEWVSYISSAGGAIYYADSVKGRFTISRDNAKNSLFLQMNSLRAEDTAVYYCAIAVAAPGAFDIWGQGTLVTVSS |
| | | | SEQ ID NO: 25308 | SEQ ID NO: 29314 |
| iPS:434017 | 21-225_48G12 | NA | GACATCCAGATGACCCAGTCTCCATCCTCCCTGTCTGCGTCTGTAGGAGACAGAGTCACCATCACTTGCCGGGCAAGTCAGAGCATTAGGAAGTATTTAAATTGGTATCAGAAGACACCAGGGAAAGCCCCTAAACTCTTGATATATGCTGCTTCCAGTTTGCAAAGTGGGGTCCCATCAAGGTTCAGTGGCAGTGGATCTGGGACAGATTTCACTCTCACCATCAGCAGTGTGCAACCTGAAGATTTTGCAAATTATTACTGTCAACAGACTTACAGTAACCCGCTCACTTTCGGCGGAGGGACCGAGGTGGAGATCACA | CAGGTGCAGCTGGTGGAGTCTGGGGGAGGCTTGGTCAAGCCTGGAGGGTCCCTGAGACTCTCCTGTGCAGCCTCTGGATTCACCTTCAGTGACTACTTCATGACCTGGATCCGCCAGGCTCCAGGGCAGGGGCTGGAGTGGGTTTCATACATTAGTAGTGCTGGTGGTGCCATATACTACGCAGACTCTGTGAAGGGCCGATTCACCATCTCCAGGGACAACGCCAAGAACTCACTGTTTCTGCAAATGAACAGCCTGAGAGCCGAGGACACGGCCGTGTATTACTGTGCTATAGCAGTGGCTGCCCCTGGTGCTTTTGATATCTGGGGCCAAGGGACATTGGTCACCGTCTCTTCA |
| | | | SEQ ID NO: 25309 | SEQ ID NO: 29315 |
| | | AA | DIQMTQSPSSLSASVGDRVTITCRASQSIRKYLNWYQKTPGKAPKLLIYAASSLQSGVPSRFSGSGSGTDFTLTISSVQPEDFANYYCQQTYSNPLTFGGGTEVEIT | QVQLVESGGGLVKPGGSLRLSCAASGFTFSDYFMTWIRQAPGQGLEWVSYISSAGGAIYYADSVKGRFTISRDNAKNSLFLQMNSLRAEDTAVYYCAIAVAAPGAFDIWGQGTLVTVSS |
| | | | SEQ ID NO: 25310 | SEQ ID NO: 29316 |

FIGURE 50

| | | | | |
|---|---|---|---|---|
| **iPS:434019** | **21-225_49A1** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGACTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGTAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGAAGATAATAAATATTATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGTTCCTCTCTGACTACTGGGGCCAGGGAACCCTGGTCACCGTCTCCTCA |
| | | | SEQ ID NO: 25311 | SEQ ID NO: 29317 |
| | | AA | DIQMTQSPSSLSASVGDRVTITCRASQGIRNDLDWYQQKPGKAPKRLIYAASSLQSGVPSRFSGSGSGTEFTLTISSLQPEDFATYYCLQHNSYPLTFGGGTKVEIK | QVQLVESGGGVVQPGRSLRLSCAASGFTFSDYGMHWVRQAPGKGLEWVAVIWYDEDNKYYVDSVKGRFTISRDNSKNTLYLQMNSLRAEDTAVYYCARELGFLSDYWGQGTLVTVSS |
| | | | SEQ ID NO: 25312 | SEQ ID NO: 29318 |
| **iPS:434021** | **21-225_49C1** | NA | GATATTGTGATGACCCAGACTCCACTCTCTCTGTCCGTCACCCCTGGACAGCCGGCCTCCATCTCCTGCAAGTCTAGTCAGAGCCTCCTGCATAGGGAAGGAAAGACCTATTTGTACTGGTACCTGCAGAAGCCAGGCCAGGCTCCACAGTTCCTGATCTTTGAAGTTTCCCACCGGTTCTCTGGCGTGCCAGATAGGTTCAGTGGCAGCGGGTCAGGGACAGATTTCACACTGAAAATCAGCCGGGTGGAGGCTGAGGATGTTGGGGTTTATTACTGCATGCAAAGTATACAGATTCCGATCACCCTCGGCCAAGGGACACGACTGGAGATTAAA | CAGGTGCAGCTGGTGGAGTCTGGGGGAGGCGTGGTCCAGCCTGGGAGGTCCCTGAGACTCTCCTGTACAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAAGGTATAGCAGCAGCTGGTCGGGCGGTATGGACGTCTGGGGCCAAGGGACCACGGTCACCGTCTCCTCA |
| | | | SEQ ID NO: 25313 | SEQ ID NO: 29319 |

FIGURE 50

| | | | | |
|---|---|---|---|---|
| | | AA | DIVMTQTPLSLSVTPGQPASISCKSSQSLLHREGKTYLYWYLQKPGQAPQFLIFEVSHRFSGVPDRFSGSGSGTDFTLKISRVEAEDVGVYYCMQSIQIPITLGQGTRLEIK | QVQLVESGGGVVQPGRSLRLSCTASGFTFSSYGMHWVRQAPGKGLEWVAVIWYDGSNKYYADSVKGRFTISRDNSKNTLYLQMNSLRAEDTAVYYCARRYSSSWSGGMDVWGQGTTVTVSS |
| | | | SEQ ID NO: 25314 | SEQ ID NO: 29320 |
| **iPS:434023** | **21-225_49F1** | NA | GACATCCAGATGACCCAGTCTCCATCTTCCGTGTCTGCATCTGTAGGAGACAGAGTCACCATCACTTGTCGGGCGAGTCGGGATATTAACGGCTGGTTAGCCTGGTATCAGCAGAAACCAGGGAAAGCCCCTAAGCTCCTGATCTATACTGTCTCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGTCTAACAGTTTCCCATTCACTTTCGGCCCTGGGACCAAAGTGGATATCAAA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGACTGGGTCTCAGTTATTAGTGGTAGTGGTGGTAGCACATTCTACGCAGACTCCGTGAAGGGCCGGTTCACCATCTCCAGAGACAATTCCAAGAGCACGCTGTATCTGCAAATGAACAGCCTGAGAGCCGAGGACACGGCCGTATATTATTGTGCGAGCGCTATAGCAGCGGCTGGTGCCCACTATTTTGACTACTGGGGCCAGGGAACCCTGGTCACCGTCTCCTCA |
| | | | SEQ ID NO: 25315 | SEQ ID NO: 29321 |
| | | AA | DIQMTQSPSSVSASVGDRVTITCRASRDINGWLAWYQQKPGKAPKLLIYTVSSLQSGVPSRFSGSGSGTDFTLTISSLQPEDFATYYCQQSNSFPFTFGPGTKVDIK | EVQLLESGGGLVQPGGSLRLSCAASGFTFSSYAMSWVRQAPGKGLDWVSVISGSGGSTFYADSVKGRFTISRDNSKSTLYLQMNSLRAEDTAVYYCASAIAAAGAHYFDYWGQGTLVTVSS |
| | | | SEQ ID NO: 25316 | SEQ ID NO: 29322 |

FIGURE 50

| | | | | |
|---|---|---|---|---|
| iPS:434025 | 21-225_49G3 | NA | GATATTGTGATGACCCAGACTCCACTCTCTCTGTCCGTCACCCCTGGACAGCCGGCCTCCATGTCCTGCAAGTCTAGTCAGAGCCTCCTGCATAGTGAAGGAAAGACCTATTTGTATTGGTACCTGCAGAAGACAGGCCAGCCTCCACACCTCCTGATCTATGAAGTTTCCAACCGGCTCTCTGGCGTGCCAGATAGGTTCAGTGGCAGCGGGTCAGGGACAGATTTCACACTGAAAATCAGCCGGATGGAGGCTGAGGATGTTGGGGTTTATTTCTGCATGCAAAGTATGCAGCTTCCGATCACCTTCGGCCAGGGGACACGACTGGAGATTAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGTTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAAGGTATAGCAGCAGCTGGTCGGGCGGAATGGACGTCTGGGGCCAAGGGACCACGGTCACCGTCTCCTCA |
| | | | SEQ ID NO: 25317 | SEQ ID NO: 29323 |
| | | AA | DIVMTQTPLSLSVTPGQPASMSCKSSQSLLHSEGKTYLYWYLQKTGQPPHLLIYEVSNRLSGVPDRFSGSGSGTDFTLKISRMEAEDVGVYFCMQSMQLPITFGQGTRLEIK | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARRYSSSWSGGMDVWGQGTTVTVSS |
| | | | SEQ ID NO: 25318 | SEQ ID NO: 29324 |
| iPS:434027 | 21-225_49H5 | NA | GACATCCAGATGACCCAGTCTCCATCTTCCGTGTCTGCATCTGTAGGAGACAGAGTCACCATCACTTGTCGGGCGAGTCAGGGTTTTAGCACCTGGTTAGCCTGGTTTCAGCAGAAACCAGGGAAAGCCCCTAAGCTCCTGATCTATGCTGCATCCAGTTTGCAAGATGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGACTAACAGTTTCCCGTTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGACCTGGGTCCGCCAGACTCCAGGGAAGGGGCTGGAGTGGGTCTCAGCTATTAGTGGTAGTGGTGGTAACTCATTCTACGCAGACTCCGTGAAGGGCCGGTTCACCATCTCCAGAGACAATTCCGAGAACACGTTGTATCTGCAAATGAACAGCCTGAGAGCCGAGGACACGGCCGTATATTACTGTGCGAAAGCAAGGGCAGTGGCTGGGTCACACTGGTTCGACCCCTGGGGCCAGGGAACCCTGGTCACCGTCTCCTCA |
| | | | SEQ ID NO: 25319 | SEQ ID NO: 29325 |

FIGURE 50

| | | | | |
|---|---|---|---|---|
| | | AA | DIQMTQSPSSVSASVGDRVTITCRASQGFSTWLAWFQQKPGKAPKLLIYAASSLQDGVPSRFSGSGSGTDFTLTISSLQPEDFATYYCQQTNSFPFTFGPGTKVDIK | EVQLLESGGGLVQPGGSLRLSCAASGFTFSSYAMTWVRQTPGKGLEWVSAISGSGGNSFYADSVKGRFTISRDNSENTLYLQMNSLRAEDTAVYYCAKARAVAGSHWFDPWGQGTLVTVSS |
| | | | SEQ ID NO: 25320 | SEQ ID NO: 29326 |
| iPS:434029 | 21-225_49C6 | NA | GACATCCAGATGACCCAGTCTCCATCCTCCCTGTCTGCATCTGTCGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGTTTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCTCTCACTTTCGGCGGAGGGACCAAGGTGGAGATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTTTGATGTAAGTAATAAAAAGTATGTAGACTCCGTGAAGGGCCGATTCACCATCTCCAGAGACAATTCCAAGAACACGCTGTATCTGCAAATGAGCAGCCTGAGAGCCGAGGACACGGCTGTGTATTATTGTGCGAGAGATCTGGGGATGATCGAGGACTACTGGGGCCAGGGAACCCTGGTCACCGTCTCCTCA |
| | | | SEQ ID NO: 25321 | SEQ ID NO: 29327 |
| | | AA | DIQMTQSPSSLSASVGDRVTITCRASQGIRNDLGWYQQKPGKAPKRLIYAASSLQSGFPSRFSGSGSGTEFTLTISSLQPEDFATYYCLQHNSYPLTFGGGTKVEIK | QVQLVESGGGVVQPGRSLRLSCAASGFTFSNYGMHWVRQAPGKGLEWVAVIWFDVSNKKYVDSVKGRFTISRDNSKNTLYLQMSSLRAEDTAVYYCARDLGMIEDYWGQGTLVTVSS |
| | | | SEQ ID NO: 25322 | SEQ ID NO: 29328 |

FIGURE 50

| | | | | |
|---|---|---|---|---|
| **iPS:434031** | **21-225_49E7** | NA | GATATTGTGATGACCCAGACTCCACTCTCTCT GTCTGTCACCCCTGGACAGCCGGCCTTCATGT CCTGCAAGTCTAGTCAGATCTTCTTGCATAGT GAAGGAAAGACCTATTTGTATTGGTACCTGCA GAAGACAGGCCAGCCTCCACACCTCCTGATCT ATGAAGTTTCCAAGCGGCTCTCTGGCGTGCCA GATAGGTTCAGTGGCAGCGGGTCAGGGACAG ATTTCACACTGAAAATCAGCCGGATGGAGGCT GAGGATGTCGGGGTTTATTACTGCATGCAAAG TATGCAGCTTCCGATTATCTTCGGCCAGGGGA CACGACTGGAGATTAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTCTGTATTACTGTGCGAGAAGGTATAG CAGCAGCTGGTCGGGCGGTATGGACGTCTGGGG CCAAGGGACCACGGTCACCGTCTCCTCA |
| | | | SEQ ID NO: 25323 | SEQ ID NO: 29329 |
| | | AA | DIVMTQTPLSLSVTPGQPAFMSCKSSQIFLHSEG KTYLYWYLQKTGQPPHLLIYEVSKRLSGVPDRF SGSGSGTDFTLKISRMEAEDVGVYYCMQSMQLP IIFGQGTRLEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTALYYCARRYSSS WSGGMDVWGQGTTVTVSS |
| | | | SEQ ID NO: 25324 | SEQ ID NO: 29330 |
| **iPS:434033** | **21-225_49F9** | NA | GATATTGTGATGACCCAGACTCCACTCTCTCT GTCCGTCACCCCTGGACAGCCGGCCTCCATCT CCTGCAAGTCTAATCAGAGCCTCGTGCATAAT GAAGGAAAGACCTATTTGTATTGGTATTTGCA GAAGCCAGGCCAGCCTCCACAGCTCCTGATCT TTGAAGTTTCCAACCGGTTCTCTGGAGTGCCA GATAGGTTCAGTGGCAGCGGGTCAGGGACAG ATTTCACACTGAAAATCAGTCGGGTGGAGGCT GAGGATGTTGGGGTTTATTACTGCATGCAAAG TATACAGTATCCGATCACCTTCGGCCAAGGGA CACGACTGGAGATTAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGTTATGGCAT GCACTGGGTCCGCCAGGCTCCAGGCAAGGGGCT GGAGTGGGTGGCACTTATATGGTATGATGGAAG GAATAAATACTATGCAGACTCCGTGAAGGGCCG ATTCACCATCTCCAGAGACAATCCCAAGAACACG CTGTATCTGCAAATGAACAGCCTGAGAGCCGAG GACACGGCTGTGTATCACTGTGCGAGAAGGTATA GCAGCAGCTGGTCGGGCGGTATGGACGTCTGGG GCCAAGGGACCACGGTCACCGTCTCCTCA |
| | | | SEQ ID NO: 25325 | SEQ ID NO: 29331 |

FIGURE 50

| | | | | |
|---|---|---|---|---|
| | | AA | DIVMTQTPLSLSVTPGQPASISCKSNQSLVHNEGKTYLYWYLQKPGQPPQLLIFEVSNRFSGVPDRFSGSGSGTDFTLKISRVEAEDVGVYYCMQSIQYPITFGQGTRLEIK | QVQLVESGGGVVQPGRSLRLSCAASGFTFSSYGMHWVRQAPGKGLEWVALIWYDGRNKYYADSVKGRFTISRDNPKNTLYLQMNSLRAEDTAVYHCARRYSSSWSGGMDVWGQGTTVTVSS |
| | | | SEQ ID NO: 25326 | SEQ ID NO: 29332 |
| iPS:434035 | 21-225_49F10 | NA | GACATCCAGATGACCCAGTCTCCATCTTCCGTGTCTGCATCTGTAGGAGACAGAGTCACCATCACTTGTCGGGCGAGTCAGGGTATTAGCAGGTGGTTAGCCTGGTATCAGCAGAAACCAGGGAAAGCCCCTAAGGTCCTGATCTATGCTGCATCCACTTTGCAAAGTGGGGTCCCATCAAGGTTCAGCGGCAGTGGATCTGGGACAGATTTCACTCTCACCATCAGCAGCCTGCAACCTGAAGATTTTGCAACTTACTATTGTCAACAGGCTAACAGTTTCCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACCATATGCACTGGGTGCGACAGGCCCCTGGACAAGGGCTTGAGTGGATGGGATGGATCAACCCTAATAACAATGCCACAAACTATGCTCAGAACTTTCAGGGCAGGGTCACCCTGACCAGGGACACGTCCATCAGCACAGCCTACATGGAGCTGAGCAGGCTGAGATCTGACGACACGGCCGTGTATTACTGTGCGAGAGACGGTACCAGCAGCTTTGACTTCTGGGGCCAGGGAACCCTGGTCACCGTCTCCTCA |
| | | | SEQ ID NO: 25327 | SEQ ID NO: 29333 |
| | | AA | DIQMTQSPSSVSASVGDRVTITCRASQGISRWLAWYQQKPGKAPKVLIYAASTLQSGVPSRFSGSGSGTDFTLTISSLQPEDFATYYCQQANSFPFTFGPGTKVDIK | QVQLVQSGAEVKKPGASVKVSCKASGYTFTGYHMHWVRQAPGQGLEWMGWINPNNNATNYAQNFQGRVTLTRDTSISTAYMELSRLRSDDTAVYYCARDGTSSFDFWGQGTLVTVSS |
| | | | SEQ ID NO: 25328 | SEQ ID NO: 29334 |

FIGURE 50

| | | | | |
|---|---|---|---|---|
| **iPS:434037** | **21-225_49G12** | NA | GACATCCAGATGACCCAGTCTCCATCCTCCCTGTCTGCGTCTGTAGGAGACAGAGTCACCATCACTTGCCGGTCAAGTCAGAGCATTAGTACCTATTTAATGTGGTATCAGCAGAAACCAGGGAAAGCCCCTAAGCTCTTGATCTATGCTGCATCCAGTTTGCAAATTGGGGTCCCATCAGAGTTCAGTGCCAGTGGATCTGGGACAGATTTCACTCTCACCATCAGCAGTCTGCAACCTGAAGATTTTGCAACTTACTACTGTCAACAGAGTTACAGTATCCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTACAGCCTCTGGATTCACCTTTAGCAGCTATGCCATGAGCTGGGTCCGCCAGGCTCCAGGGAAGGGGCTGGAGTGGGTCTCAGTTATTAGTGGTAGTGGTGGTACCACATTCTACGCAGACTCCGTGAAGGGCCGGCTCACCATCTCCAGAGACAATTCCAAGAACACACTGTATCTGCAAATGAACAGCCTGAGAGCCGAGGACACGGCCGTATATTACTGTGCGCGTCGTATAGCAGTGGCTGGGAATGATGCTTTTGATATCTGGGGCCAAGGGACAATGGTCACCGTCTCTTCA |
| | | | SEQ ID NO: 25329 | SEQ ID NO: 29335 |
| | | AA | DIQMTQSPSSLSASVGDRVTITCRSSQSISTYLMWYQQKPGKAPKLLIYAASSLQIGVPSEFSASGSGTDFTLTISSLQPEDFATYYCQQSYSIPFTFGPGTKVDIK | EVQLLESGGGLVQPGGSLRLSCTASGFTFSSYAMSWVRQAPGKGLEWVSVISGSGGTTFYADSVKGRLTISRDNSKNTLYLQMNSLRAEDTAVYYCARRIAVAGNDAFDIWGQGTMVTVSS |
| | | | SEQ ID NO: 25330 | SEQ ID NO: 29336 |
| **iPS:434039** | **21-225_43B1** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAGTCAGCAGCCTGCAGCCTGAAGATTTTGCAACTTATTTCTGTCTACAGCATACTAGTTTCCCATTCACTTTCGGCCCTGGGACCAAAGTGGATATCAAA | CAGGTGCAGCTGGTGGAGTCTGGGGGAGGCTTGGTCAAGCCTGGAGGGTCCCTGAGACTCTCCTGTGCAGCCTCTGGATTCACCTTCAGTGACTACTACATGAACTGGATCCGCCAGGCTCCAGGGAAGGGGCTGGAGTGGGTTTCATACATTAATAGTAATGGTTTTACCATATACTACGCAGACTCTGTGAAGGGCCGATTCACCATCTCCAGGGACAACGCCAAGAACTCACTGTATCTGCAAATGAACAGCCTGAGAGCCGAGGACACGGCCGTGTATTACTGTGCGGCCGATACAATCTACTGGGGCCAGGGAACCCGGGTCACCGTCTCCTCA |
| | | | SEQ ID NO: 25331 | SEQ ID NO: 29337 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTVSSLQPEDFATYFCLQHTSFPFTFGPGTKVDIK | QVQLVESGGGLVKPGGSLRLSCAASGFTFSDYYMNWIRQAPGKGLEWVSYINSNGFTIYYADSVKGRFTISRDNAKNSLYLQMNSLRAEDTAVYYCAADTIYWGQGTRVTVSS |
| | | | SEQ ID NO: 25332 | SEQ ID NO: 29338 |
| iPS:434041 | 21-225_50H8 | NA | GACATCCAGATGACCCAGTCTCCATCCTCCCTGTCTGCGTCTGTAGGAGACAGAGTCAACATCACTTGCCGGGCAAGTCAGAGCATTAGCAGCTATTTAATTTGGTATCAGCAGAAACCAGGGAAAGCCCCTAAGCTCCTGATCTATGCTGCATCCAGTCTGCAAAGTGGGGTCCCATCAAGGTTCAGTGCCAGTGGATCTGGGACAGATTTCACTCTCACCATCAGCAGTCTGCAACCTGAAGATTTTGCAAGTTACTACTGTCAACAGAGTAACAGTCTTCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTAGATTCACCTTTAGCAGTTATGCCATGAGCTGGGTCCGCCAGGCTCCAGGGAAGGGGCTGGAGTGGGTCTCAGTTATTAGTGGTCGTGGTGGTACCACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGCGTCGTATAGCAGTGGCTGGGAATGAGGCTTTTGATATCTGGGGCCAAGGGACAATGGTCACCGTCTCTTCA |
| | | | SEQ ID NO: 25333 | SEQ ID NO: 29339 |
| | | AA | DIQMTQSPSSLSASVGDRVNITCRASQSISSYLIWYQQKPGKAPKLLIYAASSLQSGVPSRFSASGSGTDFTLTISSLQPEDFASYYCQQSNSLPFTFGPGTKVDIK | EVQLLESGGGLVQPGGSLRLSCAASRFTFSSYAMSWVRQAPGKGLEWVSVISGRGGTTFYADSVKGRFTISRDNSKNTLYLQMNSLRAEDTAVYYCARRIAVAGNEAFDIWGQGTMVTVSS |
| | | | SEQ ID NO: 25334 | SEQ ID NO: 29340 |

FIGURE 50

| | | | | |
|---|---|---|---|---|
| **iPS:434043** | **21-225_50G10** | NA | GACATCCAGATGACCCAGTCTCCATCCTCCCTGTCTGCATCTGTAGGAGACAGAGTCACCATCACTTGCCGGGCAAGTCAGGGCATTAGAAATAATTTAGGCTGGTATCAGCAGAAACCAGGGAAAGTCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGTATAATAGTTACCCGTTCACTTTCGGCGGAGGGACCAAGGTGGAGAGCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCTGGGAAGGGGCTGGAGTGGGTCTCATCCATTAGTGGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGTAGCAACTTTTGACTACTGGGGCCAGGGAACCCTGGTCACCGTCTCCTCA |
| | | | SEQ ID NO: 25335 | SEQ ID NO: 29341 |
| | | AA | DIQMTQSPSSLSASVGDRVTITCRASQGIRNNLGWYQQKPGKVPKRLIYAASSLQSGVPSRFSGSGSGTEFTLTISSLQPEDFATYYCLQYNSYPFTFGGGTKVESK | EVQLVESGGGLVKPGGSLRLSCAASGFTFSSYSMNWVRQAPGKGLEWVSSISGSSSYIYYADSVKGRFTISRDNAKNSLYLQMNSLRAEDTAVYYCARVATFDYWGQGTLVTVSS |
| | | | SEQ ID NO: 25336 | SEQ ID NO: 29342 |
| **iPS:434045** | **21-225_50H10** | NA | GACATCCAGATGACCCAGTCTCCATCCTCCCTGTCTGCGTCTGTAGGAGACAGAGTCAACATCACTTGCCGGGCAAGTCAGAGCATTTACAGCTATTTAATTTGGTATCAGCAGAAACCAGGGAAAGCCCCTAAGCTCCTGATCTATGCTGCATCCAGTTTGCAAAGTGGGGTCCCATCAAGGTTCAGTGCCAGTGGATCTGGGACAGATTTCACTCTCACCATCAGCAGTCTGCAACCTGAAGATTTTGCAAGTTACTACTGTCAACAGAGTAACAGTATTCCATTCACTTTCGGCCCTGGGACCAAAGTGGATATCAAA | GAGGGGCAGTTGTTGGAGTCTGGGGGAGGCTTGGTACAGCCTGGGGGGTCCCTGAGACTCTCCTGTGCAGCCTCTAGATTCACCTTTAGCAGTTATGCCATGAGCTGGGTCCGCCAGGCTCCAGGGAAGGGGCTGGAGTGGGTCTCAGTTATTAGTGGTCGTGGTGGTAGCACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGCGTCGTATAGCAGTGGCTGGGAATGAGGCTTTTGATATCTGGGGCCAAGGGACAATGGTCACCGTCTCTTCA |
| | | | SEQ ID NO: 25337 | SEQ ID NO: 29343 |

FIGURE 50

| | | | | |
|---|---|---|---|---|
| | | AA | DIQMTQSPSSLSASVGDRVNITCRASQSIYSYLIWYQQKPGKAPKLLIYAASSLQSGVPSRFSASGSGTDFTLTISSLQPEDFASYYCQQSNSIPFTFGPGTKVDIK | EGQLLESGGGLVQPGGSLRLSCAASRFTFSSYAMSWVRQAPGKGLEWVSVISGRGGSTFYADSVKGRFTISRDNSKNTLYLQMNSLRAEDTAVYYCARRIAVAGNEAFDIWGQGTMVTVSS |
| | | | SEQ ID NO: 25338 | SEQ ID NO: 29344 |
| **iPS:434047** | **21-225_50A12** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATACTGCATCCAATTTACAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | CAGGTGCAACTGGTGCAGTCTGGGGCTGAGGTGAAGAAGCCTGGGGCCTCAGTGAAGGTCTCCTGCAAGGCTTCTGGATACACCTTCACCGGCCACTATATAAACTGGGTGCGACAGGCCCCTGGACAAGGGCCTGAGTGGATGGCATGGGTCAACCCTAACAGTGGTGGCACAAACTCTGCACAGAAGTTTCAGGGCAGGGTCACCATGACCAGGGACACGTCCATCAGCACAGTCTACATGGAGCTGAGCAGGCTGAGATCTGACGACACGGCCGTGTATTACTGTGCGAGGGGAGGGCAGCTCGGCGGGTTTAACTACTACTACTACGGTATGGACGTCTGGGGCCAAGGGACCACGGTCACCGTCTCCTCA |
| | | | SEQ ID NO: 25339 | SEQ ID NO: 29345 |
| | | AA | DIQMTQSPSSLSASVGDRVTITCRASQGIRNDLGWYQQKPGKAPKRLIYTASNLQSGVPSRFSGSGSGTEFTLTISSLQPEDFATYYCLQHNSYPWTFGQGTKVEIK | QVQLVQSGAEVKKPGASVKVSCKASGYTFTGHYINWVRQAPGQGPEWMAWVNPNSGGTNSAQKFQGRVTMTRDTSISTVYMELSRLRSDDTAVYYCARGGQLGGFNYYYYGMDVWGQGTTVTVSS |
| | | | SEQ ID NO: 25340 | SEQ ID NO: 29346 |

FIGURE 50

| | | | | |
|---|---|---|---|---|
| iPS:434049 | 21-225_50B12 | NA | GACATCCAGATGACCCAGTCTCCATCCTCCCTGTCTGCATCTGTGGGAGACAGAGTCACCATCACTTGTCGGGCAAGTCAGAGCATTAGTAGTTATTTAAATTGGTATCAGCATAAACCAGGGAAAGCCCCTAGGCTCCTGATCTATGCTGCATCCAGTTTGCAAAGTGGGGTCCCATCAAGGTTCAGTGGCAGTGAATCTGGGACAGATTTCATTCTCACTATCAGCAGTCTGCAACCTGAAGATTTTACAACTTATTACTGTCAACAGAGTTACATTGCCCCATTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCCATAGCATGAACTGGGTCCGCCAGGCTCCAGGGAAGGGGCTGGAGTGGGTCTCATCCATCAGTAGTAGTAGTAATTACATATACTACGCAGACTCAGTGAAGGGCCGATTCACCATCTCCAGAGACTACGCCAAGAACTCACTTTATCTGCAAATGAACAGCCTGAGAGCCGAGGACACGGCTGTGTATTACTGTGCGAGAGATCGGAGCATAGTAGTGGCTGGTCCCTGGGACTACTACGGTATGGACGTCTGGGGCCAAGGGACCACGGTCACCGTCTCCTCA |
| | | | SEQ ID NO: 25341 | SEQ ID NO: 29347 |
| | | AA | DIQMTQSPSSLSASVGDRVTITCRASQSISSYLNWYQHKPGKAPRLLIYAASSLQSGVPSRFSGSESGTDFILTISSLQPEDFTTYYCQQSYIAPFTFGPGTKVDIK | EVQLVESGGGLVKPGGSLRLSCAASGFTFSSHSMNWVRQAPGKGLEWVSSISSSSNYIYYADSVKGRFTISRDYAKNSLYLQMNSLRAEDTAVYYCARDRSIVVAGPWDYYGMDVWGQGTTVTVSS |
| | | | SEQ ID NO: 25342 | SEQ ID NO: 29348 |
| iPS:434053 | 21-225_51E1 | NA | GATATTGTGATGACCCAGACTCCACTCTCTCTGTCCGTCACCCCTGGACAGCCGGCCTCCATGTCCTGCAAGTCTAGTCAGAGCCTCCTGCATAGTGAGGGAAAGACCTATTTGTATTGGTACCTGCGGAAGCCAGGCCAGCCTCCACAGTTCCTGATCTTTGAAGTTTCCAACCGGTTCTCTGGAGTGCCAGATAGGTTCAGTGGCAGCGGGTCAGGGACAGAATTCACACTGAAAATCAGCCGGGTGGAGGCTGAGGATGTTGGGGTTTATTACTGCATGCAAAGTATACAGCTTCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAGTAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAAGGTATAGCAGCAGTTGGTCGGGCGGTATGGACGTCTGGGGCCAAGGGACCACGGTCACCGTCTCCTCA |
| | | | SEQ ID NO: 25343 | SEQ ID NO: 29349 |

FIGURE 50

| | | | | |
|---|---|---|---|---|
| | | AA | DIVMTQTPLSLSVTPGQPASMSCKSSQSLLHSEGKTYLYWYLRKPGQPPQFLIFEVSNRFSGVPDRFSGSGSGTEFTLKISRVEAEDVGVYYCMQSIQLPFTFGPGTKVDIK | QVQLVESGGGVVQPGRSLRLSCAASGFTFSSYGMHWVRQAPGKGLEWVAVIWYDGSSKYYADSVKGRFTISRDNSKNTLYLQMNSLRAEDTAVYYCARRYSSSWSGGMDVWGQGTTVTVSS |
| | | | SEQ ID NO: 25344 | SEQ ID NO: 29350 |
| iPS:434055 | 21-225_51B4 | NA | GACATCCAGATGACCCAGTCTCCATCCTCCCTGTCTGCATCTGTAGGAGACAGAGTCACCATCACTTGCCAGGCGAGTCGGGACATTACCTTCTATTTAAATTGGTATCAGCAGAAACCAGGGAAAGCCCCTAAGCTCCTGATCTACGATGCATCCAATTTGGAAACAGGGGTCCCATCAAGGTTCAGTGGCAGTGGATCTGGGACAGATTTTACTTTCACCATCAGCTGCGTGCATCCTGAAGATATTGCAACATATTTATGTCAACAGTATGATAATCTTCCATTCACTTTCGGCCCAGGGACCACAGTGGATATCAAA | GAGGTGCAGCTGTTGGAGTCTGGGGGAGGCTTGGTACAGCCTGGGGGGTCCCTGAGTCTCTCCTGTGCAGCCTCTGGATTCACCTTTAGAAGCTATGTCATGAGCTGGGTCCGCCAGGCTCCAGGGAAGGGGCTGGAGTGGGTCTCAGCTATTAGTGGTCGTGGTAGTAACACATTCTACACAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAAGGGATAACTGGATCACACGGTGCTTTTGATATCTGGGGCCAAGGGACAATGGTCACCGTCTCTTCA |
| | | | SEQ ID NO: 25345 | SEQ ID NO: 29351 |
| | | AA | DIQMTQSPSSLSASVGDRVTITCQASRDITFYLNWYQQKPGKAPKLLIYDASNLETGVPSRFSGSGSGTDFTFTISCVHPEDIATYLCQQYDNLPFTFGPGTTVDIK | EVQLLESGGGLVQPGGSLSLSCAASGFTFRSYVMSWVRQAPGKGLEWVSAISGRGSNTFYTDSVKGRFTISRDNSKNTLYLQMNSLRAEDTAVYYCAKGITGSHGAFDIWGQGTMVTVSS |
| | | | SEQ ID NO: 25346 | SEQ ID NO: 29352 |

FIGURE 50

| | | | | |
|---|---|---|---|---|
| **iPS:434057** | **21-225_51E4** | NA | GACATCCAGATGACCCAGTCTCCATCCTCCCTGTCTGCATCTGTGGGAGACAGAGTCACCATCACTTGCCGGGCAAGTCAGGGCATTAGAAATGATTTAGGCTGGTATCAGCAGAAACCAGGGAAAGCCCCTCAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAGGATTTTGCAGCTTATTATTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGAAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGGAACTGGGATTTCTCTCTGACTACTGGGGCCAGGGAACCCTGGTCACCGTCTCCTCA |
| | | | SEQ ID NO: 25347 | SEQ ID NO: 29353 |
| | | AA | DIQMTQSPSSLSASVGDRVTITCRASQGIRNDLGWYQQKPGKAPQRLIYAASSLQSGVPSRFSGSGSGTEFTLTISSLQPEDFAAYYCLQHNSYPFTFGPGTKVDIK | QVQLVESGGGVVQPGRSLRLSCAASGFTFSSYGMHWVRQAPGKGLEWVAVIWYDESNKYYADSVKGRFTISRDNSKNTLYLQMNSLRAEDTAVYYCARELGFLSDYWGQGTLVTVSS |
| | | | SEQ ID NO: 25348 | SEQ ID NO: 29354 |
| **iPS:434059** | **21-225_51C5** | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAACCAGGGGAAGCCCCTAAGTCCCTTATCTATGCTGCATCCAGTTTGCGAAGTGGGGTCCCATCACAGTTCAGCGGCAGTGGATCTGGAACAGATTTCATTCTCACCATCAGCAGCCTGCAGCCTGAAGATTTTGCAACTTATTACTGCCAACAGTATTATAGTTACCCATTCACTTTCGGCCCTGGGACCAAAGTGGATATCAAA | GAGGTGCAGTTGTTGGAGTCTGGGGGAGGCTTGGTACAGCCTGGGGGGTCCCTGAGACTCTCCTGTGCAGCCTCTGGATTCACCTTTAGCAGCTATGTCATGAGCTGGGTCCGCCAGACTCCAGGGAAGGGGCTGGAGTGGGTCTCAACTATGAGTGGTAGTGGTGGTCGCACATACTACGCAGACTCCGTGAACGGCCGATTCACCGTCTCCAGAGACAATTCCAAGAACACGCTGTATTTGCAAATGAGCAGCCTGAGAGCCGAGGACACGGCCGTATATTACTGTGCGCGGGTGACTGCTTTTGACTACTGGGGCCAGGGAACCCTGGTCACCGTCTCCTCA |
| | | | SEQ ID NO: 25349 | SEQ ID NO: 29355 |

FIGURE 50

| | | | | |
|---|---|---|---|---|
| | | AA | DIQMTQSPSSLSASVGDRVTITCRASQGISNYLAWFQQKPGEAPKSLIYAASSLRSGVPSQFSGSGSGTDFILTISSLQPEDFATYYCQQYYSYPFTFGPGTKVDIK | EVQLLESGGGLVQPGGSLRLSCAASGFTFSSYVMSWVRQTPGKGLEWVSTMSGSGGRTYYADSVNGRFTVSRDNSKNTLYLQMSSLRAEDTAVYYCARVTAFDYWGQGTLVTVSS |
| | | | SEQ ID NO: 25350 | SEQ ID NO: 29356 |
| iPS:434061 | 21-225_51C7 | NA | GACATCCAGATGACCCAGTCTCCATCTTCCGTGTCTGCATCTGTAGGAGACAGAGTCACCATCACTTGTCGGGCGAGTCAGGATGTTAACAACTACTTAGCCTGGTTTCAGCAGAAACCAGGGAAAGCCCCTAAGCTCCTGATCTATGCTGCATCCAGTTTGCAAAATGGGGTCCCATCAAGGTTCAGCGGCAGTGGATCTGGGACAGATTTCACTCTCACCATCAGCAGCCTGCTGCCTGAAGATTTTGCAACTTACTATTGTCAACAAACTAACAGTTTCCCATTCACTTTCGGCCCTGGGACCAAAGTGGATATCAAA | GAGGTGCAGCTGTTGGAGTCTGGGGGAGGCTTGGTGCAGCCTGGGGGGTCCCTGAGACTCTCCTGTGCAGCCTCTGGATTCACCATTAGCAACTATGCCATGACCTGGGTCCGCCAGACTCCAGGGAAGGGGCTGGAGTGGGTCTCAGTTATTAGTGCTAGTGGTGGTAACTCATTCTACGCAGACTCCGTGAAGGGCCGGTTCACCATCTCCAGAGACAATTCCGAGAACACGTTCTATCTGCAAATGAACAGCCTGAGAGCCGAGGACACGGCCGTATATTACTGTGCGAAAGCAAGGGCAGTGGCTGGGTCACACTGGTTCGACCCCTGGGGCCAGGGAACCCTGGTCACCGTCTCCTCA |
| | | | SEQ ID NO: 25351 | SEQ ID NO: 29357 |
| | | AA | DIQMTQSPSSVSASVGDRVTITCRASQDVNNYLAWFQQKPGKAPKLLIYAASSLQNGVPSRFSGSGSGTDFTLTISSLLPEDFATYYCQQTNSFPFTFGPGTKVDIK | EVQLLESGGGLVQPGGSLRLSCAASGFTISNYAMTWVRQTPGKGLEWVSVISASGGNSFYADSVKGRFTISRDNSENTFYLQMNSLRAEDTAVYYCAKARAVAGSHWFDPWGQGTLVTVSS |
| | | | SEQ ID NO: 25352 | SEQ ID NO: 29358 |

FIGURE 50

| | | | | |
|---|---|---|---|---|
| **iPS:434063** | **21-225_51G7** | NA | GACATCCAGATGACCCAGTCTCCATCTTCCGTGTCTGCATCTGTAGGAGACAGAGTCACCATCACTTGTCGGGCGAGTCAGGATATTAGCAGTTGGTTAGCCTGGTATCAGCAGAAACCAGGGAAAGCCCCTAAGGTCCTGATCTATGCTCCATCCAATTTGCAAAGTGCGGTCCCATCAAGGTTCAGCGGCAGTGGATCTGGGACAGATTTCACTCTCACCATCAGCAGCCTGCAGCCTGAAGATTTTGCAACTTACTATTGTCAACAGGCTCACAGTTTCCCGTGGACGTTCGGCCAAGGGACCAAGGTGGAAATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTAGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGCTCGCCTTGACTACTGGGGCCAGGGAACCCTGGTCACCGTCTCCTCA |
| | | | SEQ ID NO: 25353 | SEQ ID NO: 29359 |
| | | AA | DIQMTQSPSSVSASVGDRVTITCRASQDISSWLAWYQQKPGKAPKVLIYAPSNLQSAVPSRFSGSGSGTDFTLTISSLQPEDFATYYCQQAHSFPWTFGQGTKVEIK | EVQLVESGGGLVKPGGSLRLSCAASGFTFSSYSMNWVRQAPGKGLEWVSSISSSSSYIYYADSVKGRFTISRDNAKNSLYLQMNSLRAEDTAVYYCARARLDYWGQGTLVTVSS |
| | | | SEQ ID NO: 25354 | SEQ ID NO: 29360 |
| **iPS:434065** | **21-225_50D4** | NA | GACATCCAGATGACCCAGTCTCCATCTTCCGTGTCTGCATCTGTGGGAGACAGACTCACCATCACTTGTCGGGCGAGTCAGGGTATTAGCAGGTGGTTAGCCTGGTATCAGCAGAAACCAGGGAAAGCCCCTAAGGTCCTGATCTATGCTGCATCCACTTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGGCTAACAGTTTCCCATTCACTTTCGGCCCTGGGACCAAAGTGGATATCAGA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACCATATGCACTGGGTGCGACAGGCCCCTGGACAAGGGCTTGAGTGGATGGGATGGATCAACCCTAATAATAATGCCACAAACTATGCTCAGAGCTTTCAGGGCAGGGTCACCCTGACCAGGGACACGTCCATCAGCACAGCCTACATGGAGCTGAGCAGGCTGAGATCTGACGACACGGCCGTGTATTACTGTGCGAGAGACGGTACCAGCAGCTTTGACTTCTGGGGCCAGGGAACCCTGGTCACCGTCTCCTCA |
| | | | SEQ ID NO: 25355 | SEQ ID NO: 29361 |

FIGURE 50

| | | | | |
|---|---|---|---|---|
| | | AA | DIQMTQSPSSVSASVGDRLTITCRASQGISRWLAWYQQKPGKAPKVLIYAASTLQSGVPSRFSGSGSGTDFTLTISSLQPEDFATYYCQQANSFPFTFGPGTKVDIR | QVQLVQSGAEVKKPGASVKVSCKASGYTFTGYHMHWVRQAPGQGLEWMGWINPNNNATNYAQSFQGRVTLTRDTSISTAYMELSRLRSDDTAVYYCARDGTSSFDFWGQGTLVTVSS |
| | | | SEQ ID NO: 25356 | SEQ ID NO: 29362 |
| iPS:434067 | 21-225_51H8 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTGGGCTGGTATCAGCAGAAACCAGGGAAAGCCCCTAAGCGCCTGATCTATGCTGCTTCCAGTTTGCAAAGTGGGGTCCCATCAAGGTTCAGCGGCGGTGGATCTGGGACACACTTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCCACTATATGAACTGGGTGCGACAGGCCCCTGGACAAGGACTTGAGTGGATGGGATGGGTCAACCCTAACAGTGGTGGCTCAAACTCTGCACAGCAGTTTCAGGGCAGGGTCACCATGACCAGGGACACGTCCATCAGCACAGTCTACATGGAGCTGAGCAGGCTGAGTTCTGACGACACGGCCGTGTATTACTGTGCGAGGGGAGGGCAGCTCGGCGGCTTTAACTTCTACTACTACGGTATGGACGTCTGGGGCCAAGGGACCACGGTCACCGTCTCCTCA |
| | | | SEQ ID NO: 25357 | SEQ ID NO: 29363 |
| | | AA | DIQMTQSPSSLSASVGDRVTITCRASQGIRNDLGWYQQKPGKAPKRLIYAASSLQSGVPSRFSGGGSGTHFTLTISSLQPEDFATYYCLQHNSYPWTFGQGTKVEIK | QVQLVQSGAEVKKPGASVKVSCKASGYTFTGHYMNWVRQAPGQGLEWMGWVNPNSGGSNSAQQFQGRVTMTRDTSISTVYMELSRLSSDDTAVYYCARGGQLGGFNFYYYGMDVWGQGTTVTVSS |
| | | | SEQ ID NO: 25358 | SEQ ID NO: 29364 |

FIGURE 50

| | | | | |
|---|---|---|---|---|
| **iPS:434069** | **21-225_51E9** | NA | GACATCCAGATGACCCAGTCTCCATCTTCCGTGTCTGCCTCTGTAGGAGACAGAGTCACCATCACTTGTCGGGCGAGTCAGGGTATTAGCAGTTGGTTAGCCTGGTATCAGCAGAAACCAGGGAAAGCCCCTAAGCTCCTGATCTATGTTGCATCCAGTTTGCAAAGTGGGGTCCCATCAAGGTTCAGCGGCAGTGGATCTGGGACAGATTTCACTCTCACCATCAGGAGCCTGCAGCCTGAAGATTTTGCAACTTACTATTGTCAACAGGCTAAAAGTTTCCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTAGATACACCTTCACCGGCTACCATATACACTGGGTGCGACAGGCCCCTGGACAAGGGCTTGAGTGGATGGGATGGATCAACCCTAACACTAATGGCACACAGTATGCACAGAAGTTTCAGGGCCGGGTCACCATGACCAGGGACACGTCCATCAGCACAGCCTACATGGAGCTGAGCAGGCTGAGATCTGACGACACGGCCGTGTATTACTGTGCGAGAGATGGCACCTCGTCCTTTGACTACTGGGGCCAGGGAACCCTGGTCACCGTCTCCTCA |
| | | | SEQ ID NO: 25359 | SEQ ID NO: 29365 |
| | | AA | DIQMTQSPSSVSASVGDRVTITCRASQGISSWLAWYQQKPGKAPKLLIYVASSLQSGVPSRFSGSGSGTDFTLTIRSLQPEDFATYYCQQAKSFPFTFGPGTKVDIK | QVQLVQSGAEVKKPGASVKVSCKASRYTFTGYHIHWVRQAPGQGLEWMGWINPNTNGTQYAQKFQGRVTMTRDTSISTAYMELSRLRSDDTAVYYCARDGTSSFDYWGQGTLVTVSS |
| | | | SEQ ID NO: 25360 | SEQ ID NO: 29366 |
| **iPS:434071** | **21-225_51F9** | NA | GACATCCAGATGACCCAGTCTCCATCCTCCCTGTCTGCATCTGTAGGAGACAGAGTCACCATCACTTGCCGGGCAAGTCAGGGCATTAGGACTGATTTAGGCTGGTATCAGCAGAAACCAAGGAAAGCCCCTCAGCGCCTGATCTATGCTGCATCCAGTTTGCAACGTGGAGTCCCATCAAGATTCAGCGGCAGTGGATCTGGGACAGACTTCACTCTCACAATCAGCAGCCTGCAGCCTGAAGATTTTGCATCTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCGGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTTTGATGGAAATAATAAATACTATGCAGACTCCGTGAAGGGCCGATTCACCATCTCCAGAGACAATTCCAAGAACACGCTGTATCTGCAAATGAACAGCCTGAGCGCCGAGGACACGGCTGTGTATTACTGTGCGAGGGAGCTGGGATTTCTCTCTGACTACTGGGGCCAGGGAACCCTGGTCACCGTCTCCTCA |
| | | | SEQ ID NO: 25361 | SEQ ID NO: 29367 |

FIGURE 50

| | | | | |
|---|---|---|---|---|
| | | AA | DIQMTQSPSSLSASVGDRVTITCRASQGIRTDLGWYQQKPRKAPQRLIYAASSLQRGVPSRFSGSGSGTDFTLTISSLQPEDFASYYCLQHNSYPFTFGPGTKVDIK | QVQLVESGGGVVQPGRSLRLSCAASGFTFSDYGMHWVRQAPGKGLEWVAVIWFDGNNKYYADSVKGRFTISRDNSKNTLYLQMNSLSAEDTAVYYCARELGFLSDYWGQGTLVTVSS |
| | | | SEQ ID NO: 25362 | SEQ ID NO: 29368 |
| iPS:434073 | 21-225_51H10 | NA | GACATCCAGATGACCCAGTCTCCATCCTCCCTGTCTGCGTCTGTAGGAGACAGAGTCACCATCACTTGCCGGGCAAGTCAGAGCATTAGTACCTATTTAATGTGGTATCAGCAGAAACCAGGGAAAGCCCCTAAGCTCTTGATATATGCTGCATCCAGTTTGCAAAGTGGGGTCCCATCAGAGTTCAGTGCCAGTGGATCTGGGACAGATTTCACTCTCACCATCAGCAGTCTGCAACCTGAAGATTTTGCAACTTACTACTGTCAACAGAGTTACAGTATCCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTACAGCCTCTGGATTCACCTTTAGCAGCTATGCCATGAGCTGGGTCCGCCAGGCTCCAGGGAAGGGGCTGGAGTGGGTCTCAGTTATTAGTGGTAGTGGTGGTACCACATTCTACGCAGACTCCGTGAAGGGCCGGCTCACCATCTCCAGAGACAATTCCAAGAACACACTGTATCTGCAAATGAACAGCCTGAGAGCCGAGGACACGGCCGTATATTACTGTGCGCGTCGTATAGCAGTGGCTGGGAATGATGCTTTTGATATCTGGGGCCAAGGGACAATGGTCACCGTCTCTTCA |
| | | | SEQ ID NO: 25363 | SEQ ID NO: 29369 |
| | | AA | DIQMTQSPSSLSASVGDRVTITCRASQSISTYLMWYQQKPGKAPKLLIYAASSLQSGVPSEFSASGSGTDFTLTISSLQPEDFATYYCQQSYSIPFTFGPGTKVDIK | EVQLLESGGGLVQPGGSLRLSCTASGFTFSSYAMSWVRQAPGKGLEWVSVISGSGGTTFYADSVKGRLTISRDNSKNTLYLQMNSLRAEDTAVYYCARRIAVAGNDAFDIWGQGTMVTVSS |
| | | | SEQ ID NO: 25364 | SEQ ID NO: 29370 |

FIGURE 50

| | | | | |
|---|---|---|---|---|
| **iPS:434075** | **21-225_51B11** | NA | GCCATCCAGATGACCCAGTCTCCATCCTCCCTGTCTGCATCTGTAGGAGACAGAGTCACCATCACTTGCCGGACAAGTCAGGGCATTAGAAATGATTTAGGCTGGTATCAGCAGAAACCAAGGAAAGCCCCTAAACGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCGGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTTTGGTGGAAATAATAAATACTATGGAGACTCCGTGAAGGGCCGATTCACCATCTCCAGAGACAATTCCAAGAACACGCTGTATCTGCAAATGAACAGCCTGAGCGCCGAGGACACGGCTGTGTATTACTGTGCGAGGGAGCTGGGATTTCTCTCTGACTACTGGGGCCAGGGAACCCTGGTCACCGTCTCCTCA |
| | | | SEQ ID NO: 25365 | SEQ ID NO: 29371 |
| | | AA | AIQMTQSPSSLSASVGDRVTITCRTSQGIRNDLGWYQQKPRKAPKRLIYAASSLQSGVPSRFSGSGSGTEFTLTISSLQPEDFATYYCLQHNSYPFTFGPGTKVDIK | QVQLVESGGGVVQPGRSLRLSCAASGFTFSDYGMHWVRQAPGKGLEWVAVIWFGGNNKYYGDSVKGRFTISRDNSKNTLYLQMNSLSAEDTAVYYCARELGFLSDYWGQGTLVTVSS |
| | | | SEQ ID NO: 25366 | SEQ ID NO: 29372 |
| **iPS:434077** | **21-225_51F11** | NA | GACATCCAGATGACCCAGTCTCCATCCGCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGTTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTTTGGCATGCACTGGGTCCGCCAGGCTCCAGGCAAGGGGCTGGAGTGGGTGGCAGTTATATGGTATGAGGAAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGTTCCTCTCTGACTTCTGGGGCCAGGGAACCCTGGTCACCGTCTCCTCA |
| | | | SEQ ID NO: 25367 | SEQ ID NO: 29373 |

FIGURE 50

| | | | | |
|---|---|---|---|---|
| | | AA | DIQMTQSPSALSASVGDRVTITCRASQGIRNDLG WYQQKPGKAPKRLIYAASSLQSGVPSRFSGSGS GTEFTLTISSLQPEDFATYYCLQHNSYPFTFGGGT KVEIK | QVQLVESGGGVVQPGRSLRLSCAASGFTFSNFGMH WVRQAPGKGLEWVAVIWYEESNKYYADSVKGRF TISRDNSKNTLYLQMNSLRAEDTAVYYCARELGFL SDFWGQGTLVTVSS |
| | | | SEQ ID NO: 25368 | SEQ ID NO: 29374 |
| **iPS:434079** | **21-225_52B1** | NA | GACATCCAGATGACCCAGTCTCCATCTTCCGT GTCTACATTTGTAGGAGACAGAATCACCATCA CTTGTCGGGCGAGTCAGGATATTCGCACCTGG TTAGCCTGGTATCAGCAGAAACCTGGGAAAGC CCCTAAGCTCCTGATCTATGCTGCATCCAGTTT GCAAAATGGGGTCCCATCAAGGTTCAGCGGCA GTGGATCTGGGACAGATTTCACTCTCACCATC AGTAGCCTGCAGCCTGAAGATTTTGCAACTTA CTTTTGTCAACAGGCTAAAAGTTTCCCATTCAC TTTCGGCCCTGGGACCAAAGTGGATATCAAA | CAGGTGCAGTTGGTGCAGTCTGGGGCTGAGGTGA AGAAGCCTGGGGCCTCAGTGAAGGTCTCCTGCA AGGCTTCTGGATACACCTTCACCGGCTATCATAT GCAGTGGGTGCGACAGGCCCCTGGACAAGGGCT TGAGTGGATGGGATGGATCAACCCTAACAGTGGT GCCACAAACTATGCACAGAACTTTCAGGGCAGG GTCACCATGACCCGGGACACGTCCATCAGCACA GCCTACCTGGACCTGAGCAGGCTGAGATCTGACG ACACGGCCGTGTATTACTGTGCGAGAGATGGCAC CTCGTCCTTTGACTACTGGGGCCAGGGAACCCTG GTCACCGTCTCCTCA |
| | | | SEQ ID NO: 25369 | SEQ ID NO: 29375 |
| | | AA | DIQMTQSPSSVSTFVGDRITITCRASQDIRTWLA WYQQKPGKAPKLLIYAASSLQNGVPSRFSGSGS GTDFTLTISSLQPEDFATYFCQQAKSFPFTFGPGT KVDIK | QVQLVQSGAEVKKPGASVKVSCKASGYTFTGYHM QWVRQAPGQGLEWMGWINPNSGATNYAQNFQGR VTMTRDTSISTAYLDLSRLRSDDTAVYYCARDGTS SFDYWGQGTLVTVSS |
| | | | SEQ ID NO: 25370 | SEQ ID NO: 29376 |

FIGURE 50

| | | | | |
|---|---|---|---|---|
| **iPS:434081** | **21-225_52B2** | NA | GACATCCAGATGACCCAGTCTCCATCGTCACTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAGGCGCCTGATCTATGCTGCATCCTTTTTGCAAAGTGGGGTCCCATCGACATTCAGCGGCAGTGGATCTGGGACAGAATTCACTCTCACAATCAGCAGCCTGCAGCCTGAAGATTTTGCAACTTATTACTGTCTGCAGCATAATAGCTACCCTCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTACATGGTTTGATGGAAGTAATCAACGCTATGCAGACTCCGTGAAGGGCCGATTCACCATCTCCAGAGACATTTCCAAGAACACGCTGTATCTGCAAATGAACAGCCTGAGTGCCGAGGACACGGCTGTGTATTACTGTGCGAGAGATCTGGGGATGATCGAGGACTTCTGGGGCCAGGGAACCCTGGTCACCGTCTCCTCA |
| | | | SEQ ID NO: 25371 | SEQ ID NO: 29377 |
| | | AA | DIQMTQSPSSLSASVGDRVTITCRASQGIRNDLGWYQQKPGKAPRRLIYAASFLQSGVPSTFSGSGSGTEFTLTISSLQPEDFATYYCLQHNSYPLTFGGGTKVEIK | QVQLVESGGGVVQPGRSLRLSCAASGFTFSNYGMHWVRQAPGKGLEWVAVTWFDGSNQRYADSVKGRFTISRDISKNTLYLQMNSLSAEDTAVYYCARDLGMIEDFWGQGTLVTVSS |
| | | | SEQ ID NO: 25372 | SEQ ID NO: 29378 |
| **iPS:434083** | **21-225_52H2** | NA | GACATCCAGATGACCCAGTCTCCATCTTCCGTGTCTGCATCTGTAGGAGACAGAGTCACCATCACTTGTCGGGCGAGTCAGAATATTACCAACTGGTTAGCCTGGTTTCAGCAGAAACCAGGGAGAGCCCCTAAGCTCCTGATCTATACTACATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTGTTGTCAACAGACTAACAGTTTCCCGTGGACGTTCGGCCATGGGACCAAGGTAGAAGTCAAA | GAGGTGCAGCTGTTGGAGTCTGGGGGAGGCTTGGTACAGCCGGGGGGGTCCCTGAGACTCTCCTGTGCAGCCTCTGGATTCACCTTTAGCAGAAATGCCATGAGCTGGGTCCGCCAGGCTCCAGGGATGGGGCTGGAGTGGGTCTCAGCTATTAGTGGTCGTGGTGGTAATACATTCTACGCAGACTCCGTGAAGGGCCGGTTCACCGTCTCCAGAGACAATTCCAAGAACACGCTGTTTCTGCAAATGAACAGCCTGAGAGCCGAGGACACGGCCGTATATTACTGTGCGAAAAATGGGCGAGAGCAGTGGCTTGACTACTGGGGCCAGGGAACCCTGGTCACCGTCTCCTCA |
| | | | SEQ ID NO: 25373 | SEQ ID NO: 29379 |

FIGURE 50

| | | | | |
|---|---|---|---|---|
| | | AA | DIQMTQSPSSVSASVGDRVTITCRASQNITNWLA WFQQKPGRAPKLLIYTTSSLQSGVPSRFSGSGSG TDFTLTISSLQPEDFATYCCQQTNSFPWTFGHGT KVEVK | EVQLLESGGGLVQPGGSLRLSCAASGFTFSRNAMS WVRQAPGMGLEWVSAISGRGGNTFYADSVKGRFT VSRDNSKNTLFLQMNSLRAEDTAVYYCAKNGREQ WLDYWGQGTLVTVSS |
| | | | SEQ ID NO: 25374 | SEQ ID NO: 29380 |
| iPS:434085 | 21-225_52E3 | NA | GACATCCAGATGACCCAATCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCAATGCTGCATCCAGTTT ACAAAGTGGGGTCCCATCAAAGTTCAGCGGCA GTGGATCTGGGACAGATTTCACTCTCACTATC AGCAGCCTGCAGCCTGAAGATTTTGCAACTTA TTACTGCCAACAGTATAATAGTTTCCCTTTCAC TTTCGGCCCTGGGACCAAAGTGGATATCAAA | GAGGTGCAGCTGGTGGAGTCTGGGGGAGGCCTG GTCAAGCCTGGGGGGTCCCTGAGACTCTCCTGTG CAGCCTCTGGATTCACCTTCAGTAGCTATAAAAT GAACTGGGTCCGCCAGGCTCCAGGGAAGGGGCT GGAGTGGGTCTCATCCATTAGTAGTGGTAATAGT TCCATATACTACGCAGACTCAGTGAAGGGCCGAT TCACCATCTCCAGAGACAACGCCGAAAACTCACT GTATCTGCAAATGAACAGCCTGAGAGCCGAAGA CACGGCTGTGTATTACTGTGCGAGAGTTAGCAGT AATGACTACTGGGGCCAGGGAACCCTGGTCACC GTCTCCTCA |
| | | | SEQ ID NO: 25375 | SEQ ID NO: 29381 |
| | | AA | DIQMTQSPSSLSASVGDRVTITCRASQGISNYLA WFQQKPGKAPKSLINAASSLQSGVPSKFSGSGSG TDFTLTISSLQPEDFATYYCQQYNSFPFTFGPGTK VDIK | EVQLVESGGGLVKPGGSLRLSCAASGFTFSSYKMN WVRQAPGKGLEWVSSISSGNSSIYYADSVKGRFTIS RDNAENSLYLQMNSLRAEDTAVYYCARVSSNDY WGQGTLVTVSS |
| | | | SEQ ID NO: 25376 | SEQ ID NO: 29382 |

FIGURE 50

| | | | | |
|---|---|---|---|---|
| **iPS:434087** | **21-225_52F6** | NA | GACATCCAGATGACCCAGTCTCCATCCTCCCTGTCTGCATCTGTAGGAGACAGAGTCACCATCACTTGCCAGGCGAGTCAGGACATTAGTAACTATTTACATTGGTATCAGCAGAAACCAGGGAAAGCCCCTAAACTCCTGATCTACGATGCATCCACTTTGGGAACAGGGGTCCCATTAAGGTTCAGTGGAAGTGGATCTGGGACAGAATTTACTTTCACCATTAACAGCCTGCAGCCTGAAGATATTGCAACATATTCCTGTCAACAGTGTGATAATCTCCCGCTCACTTTCGGCGGGGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAGTGGGTGGCAATTATATCATATGGAGGAAGTAATAAATACTATGCAGACTCCGTGAAGGGCCGATTCACCATCTCCAGAGACAATTCCAAGAATACGCTGTATCTGCAAATGAACAGCCTGAGAGCTGATGACACGGCTGTGTATTACTGTGCGAGAAGGTCAGCAGCTCGGCCGGGCTACGGTATGGACGTCTGGGGCCAGGGGACCACGGTCACCGTCTCCTCA |
| | | | SEQ ID NO: 25377 | SEQ ID NO: 29383 |
| | | AA | DIQMTQSPSSLSASVGDRVTITCQASQDISNYLHWYQQKPGKAPKLLIYDASTLGTGVPLRFSGSGSGTEFTFTINSLQPEDIATYSCQQCDNLPLTFGGGTKVEIK | QVQLVESGGGVVQPGRSLRLSCAASGFTFSSYGMHWVRQAPGKGLEWVAIISYGGSNKYYADSVKGRFTISRDNSKNTLYLQMNSLRADDTAVYYCARRSAARPGYGMDVWGQGTTVTVSS |
| | | | SEQ ID NO: 25378 | SEQ ID NO: 29384 |
| **iPS:434091** | **21-225_52B9** | NA | GCCATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAAGGAAAGCCCCTAAGCGCCTGATCTATGCTGCATCCAGTTTGCAAAGTGGGGTCCCATTAAGGTTCAGCGGCAGTGGATCTGGGACAGAATTCACTCTCACAATCAGAAGCCTGCAGCCTGAAGATTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCGGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTTTGATGGAAATAATAAATACTATGCAGACTCCGTGAAGGGCCGATTCACCATCTCCAGAGACAATTCCAAGAACACGCTGTATCTGCAAATGAACAGCCTGAGCGCCGAGGACACGGCTGTGTATTACTGTGCGAGGGAGCTGGGATTTCTCTCTGACTACTGGGGCCAGGGAACCCTGGTCACCGTCTCCTCA |
| | | | SEQ ID NO: 25379 | SEQ ID NO: 29385 |

FIGURE 50

| | | | | |
|---|---|---|---|---|
| | | AA | AIQMTQSPSSLSASVGDRVTITCRASQGIRNDLGWYQQKPRKAPKRLIYAASSLQSGVPLRFSGSGSGTEFTLTIRSLQPEDFATYYCLQHNSYPFTFGPGTKVDIK | QVQLVESGGGVVQPGRSLRLSCAASGFTFSDYGMHWVRQAPGKGLEWVAVIWFDGNNKYYADSVKGRFTISRDNSKNTLYLQMNSLSAEDTAVYYCARELGFLSDYWGQGTLVTVSS |
| | | | SEQ ID NO: 25380 | SEQ ID NO: 29386 |
| **iPS:434093** | **21-225_52D10** | NA | GATGTTATGATGACCCAGATTCCACTCTCTCTGTCCGTCACCCCTGGACAGCCGGCCTCCATCTCCTGCAAGTCTAGTCAGAGCCTCCTGCATAGTGAAGGAAAGACCTATTTGTATTGGTACTTGCAGAAGCCAGGCCAGCCTCCACAGCTCCTGATCTTTGAAGTTTCCAACCGGGTCTCTGGAGTGCCAGATAGGTTCAGTGGCAGAGGGTCAGGGACAGATTTCACACTGAAAATCAGCCGGGTGGAGGCTGAGGATGTTGGGGTTTATTACTGCATGCAAAGTATACAGTATCCGATCACCTTCGGCCAAGGGACACGACTGGAGATTAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGTTATGGCATGCACTGGGTCCGCCAGGCTCCAGGCAAGGGGCTGGAGTGGGTGGCACTTATATGGTATGATGGAAGTAATAAATACCATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAAGGTATAGCAGCAGCTGGTCGGGCGGTATGGACGTCTGGGGCCAAGGGACCACGGTCACCGTCTCCTCA |
| | | | SEQ ID NO: 25381 | SEQ ID NO: 29387 |
| | | AA | DVMMTQIPLSLSVTPGQPASISCKSSQSLLHSEGKTYLYWYLQKPGQPPQLLIFEVSNRVSGVPDRFSGRGSGTDFTLKISRVEAEDVGVYYCMQSIQYPITFGQGTRLEIK | QVQLVESGGGVVQPGRSLRLSCAASGFTFSSYGMHWVRQAPGKGLEWVALIWYDGSNKYHADSVKGRFTISRDNSKNTLYLQMNSLRAEDTAVYYCARRYSSSWSGGMDVWGQGTTVTVSS |
| | | | SEQ ID NO: 25382 | SEQ ID NO: 29388 |

FIGURE 50

| | | | | |
|---|---|---|---|---|
| **iPS:434095** | **21-225_52F10** | NA | GACATCCAGATGACCCAGTCTCCATCCTCACTGTCTGTATCTGTAGGAGACAGAGTCACCATCACTTGTCGGGCGAGTCAGGGTATTAGTAATTATTTAGGCTGGTTTCAGCAGAAACCAGGGAAAGCCCCGAAGTCCCTGATTTATGCTGCATCTAGTTTGCAAAGTGGGGTCCCATCAAGGTTCAGCGGCAGTGGATCTGGGACAGATTTCACTCTCACCATCAGCAGCCTACAGCCTGAAGATTTTGCAACTTATTACTGCCAACAGTATAATAGTTATCCTCCGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTTTCTATGGCATGCACTGGGTCCGCCAGGCTCCAGGCAAGGGGCTGGAGTGGGTGGCAGTTATATGGGACGATGGAAGTAATAAATACTATGCAGACTCCGTGAAGGGCCGATTCACCATCTCCAGAGACAATTCCAAGAACACGCTGTTTCTGCAAATGATGAGCCTGAGAGCCGAGGACACGGCTGTGTATTACTGTGCGAGAGATTCTCTGTATAGCAGCAGCTGGTTGTTTGACTACTGGGGCCAGGGAACCCTGGTCACCGTCTCCTCA |
| | | | SEQ ID NO: 25383 | SEQ ID NO: 29389 |
| | | AA | DIQMTQSPSSLSVSVGDRVTITCRASQGISNYLGWFQQKPGKAPKSLIYAASSLQSGVPSRFSGSGSGTDFTLTISSLQPEDFATYYCQQYNSYPPTFGQGTKVEIK | QVQLVESGGGVVQPGRSLRLSCAASGFTFSFYGMHWVRQAPGKGLEWVAVIWDDGSNKYYADSVKGRFTISRDNSKNTLFLQMMSLRAEDTAVYYCARDSLYSSSWLFDYWGQGTLVTVSS |
| | | | SEQ ID NO: 25384 | SEQ ID NO: 29390 |
| **iPS:434097** | **21-225_52H10** | NA | GACATCCAGATGACCCAGTCTCCATCTTCCGTGTCTGCATCTGTAGGAGACAGAGTCACCATCACTTGTCGGGCGAGTCAGGATATTAACAGTTGGTTAGCCTGGTATCAGCAGAAACCAGGGAAAGCCCCTAAACTCCTGATCTATGTTGCATCCAGTTTGCAAAGTGGGGCCCCATCAAGGTTCAGCGGCAGTGGATCTGGGACAGATTTCACTCTCACCATCAGGAGCCTGCAGCCTGAAGATTTTGCTACTTACTATTGTCAACAGGCTAAAAGTTTCCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACCATATGCAGTGGGTGCGACAGGCCCCTGGACAAGGGCTTGAGTGGATGGGATGGATCAACCCTAACAATGGTGGCACACAGTATGCACAGAAGTTTCAGGGCCGGGTCACCATGACCAGGGACACGTCCATCAGCACAGCCTACATGGAACTGAGCAGGCTGAGATCTGACGACACGGCCGTGTATTACTGTGCGAGAGATGGCACCTCGTCCTTTGACTACTGGGGCCAGGGAACCCTGGTCACCGTCTCCTCG |
| | | | SEQ ID NO: 25385 | SEQ ID NO: 29391 |

FIGURE 50

| | | | | |
|---|---|---|---|---|
| | | AA | DIQMTQSPSSVSASVGDRVTITCRASQDINSWLAWYQQKPGKAPKLLIYVASSLQSGAPSRFSGSGSGTDFTLTIRSLQPEDFATYYCQQAKSFPFTFGPGTKVDIK | QVQLVQSGAEVKKPGASVKVSCKASGYTFTGYHMQWVRQAPGQGLEWMGWINPNNGGTQYAQKFQGRVTMTRDTSISTAYMELSRLRSDDTAVYYCARDGTSSFDYWGQGTLVTVSS |
| | | | SEQ ID NO: 25386 | SEQ ID NO: 29392 |
| iPS:434101 | 21-225_52H12 | NA | GACATCCAGATGACCCAGTCTCCATCCTCCCTGTCTGCATCTGTAGGAGACAGAGTCACCATCACTTGCCGGGCAAGTCAGGGCATAAGAAATAATTTAGGCTGGTATCAGCAGAAACCAGGGAAAGCCCCTAAGCGCCTGATCTATGGTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGTATAATAGTTATCCATTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTACTTACATATACTACGCAGACTCAGTGAAGGGCCGATTCACCGTCTCCAGAGACAACGCCAAGAACTCACTGTATCTGCAAGTGAACAGCCTGAGAGCCGAGGACACGGCTGTGTATTACTGTGCGAGAGTCAACTCCTTTGACTACTGGGGCCAGGGAACCCTGGTCACCGTCTCCTCA |
| | | | SEQ ID NO: 25387 | SEQ ID NO: 29393 |
| | | AA | DIQMTQSPSSLSASVGDRVTITCRASQGIRNNLGWYQQKPGKAPKRLIYGASSLQSGVPSRFSGSGSGTEFTLTISSLQPEDFATYYCLQYNSYPFTFGPGTKVDIK | EVQLVESGGGLVKPGGSLRLSCAASGFTFSSYSMNWVRQAPGKGLEWVSSISGSSTYIYYADSVKGRFTVSRDNAKNSLYLQVNSLRAEDTAVYYCARVNSFDYWGQGTLVTVSS |
| | | | SEQ ID NO: 25388 | SEQ ID NO: 29394 |

FIGURE 50

| | | | | |
|---|---|---|---|---|
| **iPS:434103** | **21-225_53G1** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATCCTGCATCCAGTTTACAAAGTGGGGTCCCATCAAGGTTCAGCGGCAGCGGATCTGGGACAGAATTCAGTCTCACAATCAGCAGCCTGCAGCCTGAAGATTTTGCAACTTATTACTGTCTACAGGATAATAGTTACCCATTCACTTTCGGCCCTGGGACCAAAGTGGATATCAAA | GAGGTGCAGCTGGTGGAGTCTGGGGGAGGCCTGGTCAAGCCTGGGGAGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGATCGGGGCAGCACCTGGGGCCAGGGAACCCTGGTCACCGTCTCCTCA |
| | | | SEQ ID NO: 25389 | SEQ ID NO: 29395 |
| | | AA | DIQMTQSPSSLSASVGDRVTITCRASQGIRNDLGWYQQKPGKAPKRLIYPASSLQSGVPSRFSGSGSGTEFSLTISSLQPEDFATYYCLQDNSYPFTFGPGTKVDIK | EVQLVESGGGLVKPGESLRLSCAASGFTFSSYSMNWVRQAPGKGLEWVSSISGSSSYIYYADSVKGRFTISRDNAKNSLYLQMNSLRAEDTAVYYCARDRGSTWGQGTLVTVSS |
| | | | SEQ ID NO: 25390 | SEQ ID NO: 29396 |
| **iPS:434105** | **21-225_53D2** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAACATAATAGATACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAATGGGTGACAGTTGTATGGGATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTACGAGGGGCCTTGGCTTTACGGGAGACTACTGGGGCCAGGGAGCCCTGGTCACCGTCTCCTCA |
| | | | SEQ ID NO: 25391 | SEQ ID NO: 29397 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNRYPLTFGGGTKVEIK | QVQLVESGGGVVQPGRSLRLSCAASGFTFSNYGMHWVRQAPGKGLEWVTVVWDDGSNKYYADSVKGRFTISRDNSKNTLYLQMNSLRAEDTAVYYCTRGLGFTGDYWGQGALVTVSS |
| | | | SEQ ID NO: 25392 | SEQ ID NO: 29398 |
| iPS:434107 | 21-225_53E2 | NA | GACATCCAGATGACCCAGTCTCCATCCTCCCTGTCTGCATCTGTAGGAGACAGAGTCACCATCACTTGCCGGGCAAGTCAGAGTTTTAGCCACTATTTAAATTGGTATCAGCAGAAACCAGGGAAAGCCCCTAACCTCCTGATCTTTGCTGTATCCAGTTTGCAAAGTGGGGTCCCATCACGGTTCAGTGGCAGTGGATCTGGGTCAGATTTCACTCTCCCCATCAGTAGTTTGCAACCTGAAGATTTTGCAATTTACTTCTGTCAACAGAGTTTCAGTACCCCATTCACTTTCGGCCCTGGGACCAAAGTGGATATCAAA | GAGGTGCAGTTGTTGGAGTCTGGGGGAGGCTTGATACAGCCTGGGGGGTCCCTGAGACTCTCCTGTGCAGCCTCTGGATTCACCTTTAGGAGCTATGCCATGAGCTGGGTCCGCCAGGCTCCAGGGAAGGGGCTGGAGTGGGTCTCAGCTATTAGTGGTAGTGGTGGTAACACATTCTACGCAGACTCCGTGAAGGGCCGGTTCACCATCTCCAGAGACAATTCCAAGAACACGCTGTATTTGCAAATGAACACCCTGAGAGCCGACGACACGGCCGTATATTACTGTGCGAAAAAGGTCGTGGATACAGCCATGGCTCTTGACTACTGGGGCCAGGGAACCCTGGTCACCGTCTCCTCA |
| | | | SEQ ID NO: 25393 | SEQ ID NO: 29399 |
| | | AA | DIQMTQSPSSLSASVGDRVTITCRASQSFSHYLNWYQQKPGKAPNLLIFAVSSLQSGVPSRFSGSGSGSDFTLPISSLQPEDFAIYFCQQSFSTPFTFGPGTKVDIK | EVQLLESGGGLIQPGGSLRLSCAASGFTFRSYAMSWVRQAPGKGLEWVSAISGSGGNTFYADSVKGRFTISRDNSKNTLYLQMNTLRADDTAVYYCAKKVVDTAMALDYWGQGTLVTVSS |
| | | | SEQ ID NO: 25394 | SEQ ID NO: 29400 |

FIGURE 50

| | | | | |
|---|---|---|---|---|
| **iPS:434111** | **21-225_53H2** | NA | GACATCCAGATGACCCAGTCTCCATCCTCCCTGTCTGCATCTGTAGGAGACAGAGTCACCATCACTTGCCAGGCGAGTCAGGACATTAGCAACTATTTACATTGGTATCAGCAGAAACCAGGGAAAGCCCCTCAGCTCCTGATCTACGATGCATCCAATTTGGAAACAGGGGTCCCATCAAGGTTCACTGGAAGTGGATCTGGGACAGATTTTACTTTCACCATCAGCAGCCTGCAGCCTGAAGATATTGCAACATATTACTGTCATCAGTATGATAATCTCCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCCGGAGTGGGTGGCAGTTATATCATATGGTGGAAGTAATAAATACCATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTACTGTGCGAGGCGGGGAGCAGCTCGTCCTGGCTACGGTATGGACGTCTGGGGCCAAGGGACCACGGTCACCGTCTCCTCA |
| | | | SEQ ID NO: 25395 | SEQ ID NO: 29401 |
| | | AA | DIQMTQSPSSLSASVGDRVTITCQASQDISNYLHWYQQKPGKAPQLLIYDASNLETGVPSRFTGSGSGTDFTFTISSLQPEDIATYYCHQYDNLPLTFGGGTKVEIK | QVQLVESGGGVVQPGRSLRLSCAASGFTFSSYGMHWVRQAPGKGPEWVAVISYGGSNKYHADSVKGRFTISRDNSKNTLYLQMNSLRAEDTAVYYCARRGAARPGYGMDVWGQGTTVTVSS |
| | | | SEQ ID NO: 25396 | SEQ ID NO: 29402 |
| **iPS:434115** | **21-225_53E4** | NA | GACATCCAGATGACCCAGTCTCCATCCTCACTGTCTGCATCTGTAGGAGACAGAGTCACCATCACTTGTCGGGCGAGTCAGGTCATTAGCAATTATTTAGCCTGGTTTCAGCAGAAACCAGGGAAAGCCCCTAAGTCCCTGATCTCTGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTACTACTGCCAACAGTATCATAGTTACCCACTCACTTTCGGCCGTGGGACCAAAGTGGATATCAAA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATGTCATGAGCTGGGTCCGCCAGGCTCCAGGGAAGGGGCTGGAGTGGGTCTCAGGTATTAGTGGTAGTGGTGGTCGCACATACTACGCAGACTCCGTGAAGGGCCGGTTCAACATCTCCAGAGACAATTCCAAGAACACGCTGTATCTGCAAATGAACAGCCTGAGAGCCGAGGACACGGCCGTCTATTACTGTGCGAGGGTGGCCCTTTTTGACTACTGGGGCCAGGGAACCCTGGTCACCGTCTCCTCA |
| | | | SEQ ID NO: 25397 | SEQ ID NO: 29403 |

FIGURE 50

| | | | | |
|---|---|---|---|---|
| | | AA | DIQMTQSPSSLSASVGDRVTITCRASQVISNYLAWFQQKPGKAPKSLISAASSLQSGVPSKFSGSGSGTDFTLTISSLQPEDFATYYCQQYHSYPLTFGRGTKVDIK | EVQLLESGGGLVQPGGSLRLSCAASGFTFSSYVMSWVRQAPGKGLEWVSGISGSGGRTYYADSVKGRFNISRDNSKNTLYLQMNSLRAEDTAVYYCARVALFDYWGQGTLVTVSS |
| | | | SEQ ID NO: 25398 | SEQ ID NO: 29404 |
| **iPS:434117** | **21-225_53C6** | NA | GACATCCAGATGACCCAGTCTCCATCCTCCCTGTCTGCATCTGTAGGAGACAGAGTCACCATCACTTGCCGGGCAAGTCAGTACAGTAGCGACTATTTAAATTGGTATCAGCAGAAACCAGGGAAAGCCCCTAAGGTCCTGATCTTTGCTGCATCCAGTTTGAAAAGTGGGGTCCCATCAAGGTTCAGTGGCAGTGGATCTGGGACAGATTTCACTCTCACCATCAGCAGTCTGGAACCTGAGGATTTTGCGACTTACTTCTGTCAACAGAGTTACAGTACCCCGTTCACCTTCGGCCAAGGGACACGACTGGAGATTAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAACTGGGTCCGCCAGGCTCCAGGGAAGGGGCTGGAGTGGGTCTCAGCTATTAGTGGTAGTGGTGGTGCCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTATTGTGCGAAACCTCTAGTGGGAGCCCATGATGCTTTTGAAATCTGGGGCCAAGGGACAATGGTCACCGTCTCTTCA |
| | | | SEQ ID NO: 25399 | SEQ ID NO: 29405 |
| | | AA | DIQMTQSPSSLSASVGDRVTITCRASQYSSDYLNWYQQKPGKAPKVLIFAASSLKSGVPSRFSGSGSGTDFTLTISSLEPEDFATYFCQQSYSTPFTFGQGTRLEIK | EVQLLESGGGLVQPGGSLRLSCAASGFTFSSYAMNWVRQAPGKGLEWVSAISGSGGATYYADSVKGRFTISRDNSKNTLYLQMNSLRAEDTAVYYCAKPLVGAHDAFEIWGQGTMVTVSS |
| | | | SEQ ID NO: 25400 | SEQ ID NO: 29406 |

FIGURE 50

| | | | | |
|---|---|---|---|---|
| iPS:434119 | 21-225_53E6 | NA | GACATCCAGATGACCCAGTCTCCATCCTCCCTGTCTGCCTCTGTAGGCGCCAGAGTCACCATCACTTGCCGGGCAAGTCAGGGCATTAGAAATGATTTAGGCTGGTATCAGCAGAATCCGGGGAAAGCCCCTAAGCGCCTGATCTATGCTGCATCCAATTTGCAAAGTGGGGTCCCATCAAGGTTCAGCGGCAGTGGATCTGGGACAGAATTCACTCTCACTATCAGCAGCCTGCAGCCTGAAGATTTTGCAACTTATTTCTGTCTACAACATAATCGTTACCCGCTCACTTTCGGCGGAGGGACCAAGGTGGAGATCAAA | CAGGTCCAGCTGGTGGAGTCTGGGGGAGGCGTGGTCCAGCCTGGGAGGTCCCTGAGACTCTCCTGTACAACGTCTGGATTCACCTTCAGTGACTATGGCATCCACTGGGTCCGCCAGGCTCCAGGCAAGGGGCTGGAGTGGGTGGCAGTTATATGGTATGATGAAAGTAATAAATACTATGGAGACTCCGTGAAGGGCCGATTCGCCATCTCCAGAGACAATTCCAAGAACACGCTGTATCTGCAAATGAACAGCCTGAGAGCCGAGGACACGGCTGTGTATTATTGTGTGAGAGAACTGGGGATGACGTCTGACTACTGGGGCCAGGGAACCCTGGTCACCGTCTCCTCA |
| | | | SEQ ID NO: 25401 | SEQ ID NO: 29407 |
| | | AA | DIQMTQSPSSLSASVGARVTITCRASQGIRNDLGWYQQNPGKAPKRLIYAASNLQSGVPSRFSGSGSGTEFTLTISSLQPEDFATYFCLQHNRYPLTFGGGTKVEIK | QVQLVESGGGVVQPGRSLRLSCTTSGFTFSDYGIHWVRQAPGKGLEWVAVIWYDESNKYYGDSVKGRFAISRDNSKNTLYLQMNSLRAEDTAVYYCVRELGMTSDYWGQGTLVTVSS |
| | | | SEQ ID NO: 25402 | SEQ ID NO: 29408 |
| iPS:434121 | 21-225_53F6 | NA | GACATCCAGATGACCCAGTCTCCATCCTCCCTGTCTGCATCTGTAGGAGACAGAGTCACCATCACTTGCCAGGCGAGTCAAGACATTACCAACTATTTAGATTGGTATCAGCAGAAACCGGGGAAAGCCCCTAAACTCCTGATCTACGATGCATCCAATTTGGGAACAGGGGTCCCATCAAGGTTCAGTGGAAGTGGATCTGGGACAGATTTTACTTTCACCATCAGCAGCCTGCAGCCTGAAGATATTGCAACATATTACTGTCAACAGTGTGATAATCTCCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAGTGGGTGGCAGTTATATCATATGGTGGAAGTAATAAATACGATGCAGACTCCGTGAAGGGCCGATTCACCATCTCCAGAGACAATTCCAAGAACACGCTGTATCTGCAAATGACCAGCCTGAGAGCTGAGGACACGGCTGTGTATTACTGTGCGAGACGACGGGCAGCTCGTCCAGGGTACGGTATGGACGTCTGGGGCCAAGGGACCACGGTCACCGTCTCCTCA |
| | | | SEQ ID NO: 25403 | SEQ ID NO: 29409 |

FIGURE 50

| | | | | |
|---|---|---|---|---|
| | | AA | DIQMTQSPSSLSASVGDRVTITCQASQDITNYLDWYQQKPGKAPKLLIYDASNLGTGVPSRFSGSGSGTDFTFTISSLQPEDIATYYCQQCDNLPLTFGGGTKVEIK | QVQLVESGGGVVQPGRSLRLSCAASGFTFSSYGMHWVRQAPGKGLEWVAVISYGGSNKYDADSVKGRFTISRDNSKNTLYLQMTSLRAEDTAVYYCARRRAARPGYGMDVWGQGTTVTVSS |
| | | | SEQ ID NO: 25404 | SEQ ID NO: 29410 |
| **iPS:434123** | **21-225_53F7** | NA | GACATCCAGATGTCCCAGTCTCCATCTTCCGTGTCTGCATCTGTAGGAGACAGAGTCACCATCACTTGTCGGGCGAGTCAGGGTATTAGCAGGTGGTTAGCCTGGTATCAGCAGAAACCAGGGAAAGCCCCTAACCTCCTGATCTATGCTGCATCCAGTTTGCAAAGTGGGGTCCCATCAAGGTTCAGCGGCAGTGGCTCTGGGACAGATTTCACTCTCACCATCAGCAGCCTGCAGCCTGAAGATTTTGCAATTTACTATTGTCAGCAGGCTAACAGTTTCCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACCATATGCACTGGGTGCGACAGGCCCCTGGACAAGGGCTTGAGTGGATGGGATGGATCAACCCTAACAATAACGGCACAAACTATGCACAGAAGTTTCAGGGCAGGGTCACCATGACCAGGGACACGTCCATCAGCACAGCCTACATGGAGCTGAACAGGCTGACATCTGACGACACGGCCGTGTATTACTGTGCGAGAGACGGTACCAGCAGCTTTGACTACTGGGGCCAGGGAACCCTGGTCACCGTCTCCTCA |
| | | | SEQ ID NO: 25405 | SEQ ID NO: 29411 |
| | | AA | DIQMSQSPSSVSASVGDRVTITCRASQGISRWLAWYQQKPGKAPNLLIYAASSLQSGVPSRFSGSGSGTDFTLTISSLQPEDFAIYYCQQANSFPFTFGPGTKVDIK | QVQLVQSGAEVKKPGASVKVSCKASGYTFTGYHMHWVRQAPGQGLEWMGWINPNNNGTNYAQKFQGRVTMTRDTSISTAYMELNRLTSDDTAVYYCARDGTSSFDYWGQGTLVTVSS |
| | | | SEQ ID NO: 25406 | SEQ ID NO: 29412 |

FIGURE 50

| | | | | |
|---|---|---|---|---|
| iPS:434127 | 21-225_53H8 | NA | GAAATTGTGTTGACGCAGTCTCCAGGCACCCTGTCTTTGTCTCCAGGGGAAAGAGCCACCCTCTCCTGCAGGGCCAGTCAGAGTATTACAAGCAGCTACTTAGCCTGGTACCAGCAGAAACCTGGCCAGGCTCCCAGGCTCCTCATCTATGGTGCGTCCGGCAGGGCCACTGGCATCCCAGACAGGTTCAGTGGCAGTGGGTCTGGGACAGACTTCACCCTCACCATCAGCAGACTGGAGCCTGAAGATTTTGCAGTGTATTACTGTCAGCAGTTTGAAAGCTCACCCATGTGCAGTTTTGGCCAGGGGACCAACCTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGGCCCGTATTGGGTACTTTGACTCCTGGGGCCAGGGAACCCTGGTCACCGTCTCCTCA |
| | | | SEQ ID NO: 25407 | SEQ ID NO: 29413 |
| | | AA | EIVLTQSPGTLSLSPGERATLSCRASQSITSSYLAWYQQKPGQAPRLLIYGASGRATGIPDRFSGSGSGTDFTLTISRLEPEDFAVYYCQQFESSPMCSFGQGTNLEIK | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARARIGYFDSWGQGTLVTVSS |
| | | | SEQ ID NO: 25408 | SEQ ID NO: 29414 |
| iPS:434129 | 21-225_53B12 | NA | GACATCCAGATGACCCAGTCTCCATCCTCCCTGTCTGCATCTGTAGGAGACAGAGTCACCATCACTTGTCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCGGGGAGGTCCCTGAGACTCTCCTGTGCAGCGTCTGGATTCACCTTCAGTAACTTTGGCATGCACTGGGTCCGCCAGGCTCCAGGCAAGGGGCTGGAGTGGGTGGCAGTTGTATGGTATGATGGAAATAATAGATACTATGCAGACTCCGTGAAGGGCCGATTCACCATCTCCAGAGACAATTCCAAGAACACGCTCTATCTGCAAATGCACAGCCTGAGAGCCGAGGACACGGCTGTGTATTACTGTGCGAGGGAACTGGGATTTCTCTCTGACTTCTGGGGCCAGGGAACCCTGGTCACCGTCTCCTCA |
| | | | SEQ ID NO: 25409 | SEQ ID NO: 29415 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPFTFGPGTKVDIK | QVQLVESGGGVVQPGRSLRLSCAASGFTFSNFGMHWVRQAPGKGLEWVAVVWYDGNNRYYADSVKGRFTISRDNSKNTLYLQMHSLRAEDTAVYYCARELGFLSDFWGQGTLVTVSS |
| | | | SEQ ID NO: 25410 | SEQ ID NO: 29416 |
| iPS:434131 | 21-225_54D3 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGATTCAGCGGCAGTGGATCTGGGACAGAATTCACTCTCACAATCAGCAGCCTGCAGCCTGAAGATTTTGCAACTTATTACTGTCTACAACATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTACATGGTTTGATGGAAATAATAACTACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGTTCCTTTCTGATTATTGGGGCCAGGGAACCCTGGTCACCGTCTCCTCA |
| | | | SEQ ID NO: 25411 | SEQ ID NO: 29417 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPFTFGPGTKVDIK | QVQLVESGGGVVQPGRSLRLSCAASGFTFSNYGMHWVRQAPGKGLEWVAVTWFDGNNNYYADSVKGRFTISRDNSKNTLYLQMNSLRAEDTAVYYCARELGFLSDYWGQGTLVTVSS |
| | | | SEQ ID NO: 25412 | SEQ ID NO: 29418 |

FIGURE 50

| | | | | |
|---|---|---|---|---|
| **iPS:434133** | **21-225_54G3** | NA | GACATCCAGATGACCCAGTCTCCATCTTCCGTGTCTGCATCTGTAGGAGACAGAGTCACCATCACTTGTCGGGCGAGTCAGGGTATTAGCAGCTGGTTAGCCTGGTATCAGCAGAAACCAGGGAAAGCCCCTAAACTCCTGATCTATGATGCATCCAGTTTGCAAAGTGGGGTCCCATCAAGGTTCAGCGGCAGTGGATCTGAGACAGATTTCACTCTCACCATCAGCAGCCTGCAGCCTGAAGATTTTGCAACTTACTATTGTCAACAGGCTAACAGTTTCCCGTGGACGTTCGGCCAAGGGACCAAGGTGGAAATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCACCTATGCCATGAGTTGGGTCCGCCAGGCTCCAGGGAAGGGGCTGGAGTGGGTCTCAGCTATTAGTGGTAGTGGTGTTAACACATTCTACGCAGACTCCGTGAAGGGCCGGTTCACCATCTCCAGAGACAATTCCAAGAACACGCTGTTTCTGCAAATGAACAGCCTGAGAGCCGAGGACACGGCCGTATATTACTGTGCGAAACTGGGGAAGGACTACTACTACTACGGTATGGACGTCTGGGGCCAAGGGACCACGGTCACCGTCTCCTCA |
| | | | SEQ ID NO: 25413 | SEQ ID NO: 29419 |
| | | AA | DIQMTQSPSSVSASVGDRVTITCRASQGISSWLAWYQQKPGKAPKLLIYDASSLQSGVPSRFSGSGSETDFTLTISSLQPEDFATYYCQQANSFPWTFGQGTKVEIK | EVQLLESGGGLVQPGGSLRLSCAASGFTFSTYAMSWVRQAPGKGLEWVSAISGSGVNTFYADSVKGRFTISRDNSKNTLFLQMNSLRAEDTAVYYCAKLGKDYYYYGMDVWGQGTTVTVSS |
| | | | SEQ ID NO: 25414 | SEQ ID NO: 29420 |
| **iPS:434135** | **21-225_54H3** | NA | GACATCCAGATGACCCAATCTCCATCCTCCCTGTCTGCATCTGTTGGAGACAGAGTCACCATCACTTGCCGGGCAAGTCAGGACATTAGAAATATTTTAGGCTGGTATCAGCAGAAACCAGGGAAAGCCCCTAAGCGCCTGATCTATGCTGCATCCAATTTGCAAAGTGGAGTCCCATCAAGGTTCAGCGGCAGTGGATCTGGGACAGAATTCACTCTCACAATCAGCAGCCTGCAGCCTGAGGATTTCGCAACTTATTACTGTCTACAGTATAATAGTTACCCGTGGACGTTCGGCCAAGGGACCAAGGTGGAAATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGATCTGGGTCCGCCAGGCTCCAGGGAAGGGGCTGGAGTGGGTCTCATCCATTAGTGGTACTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGGGAATGACTACAGTAATTTGGGGCCAGGGAACCCTGGTCACCGTCTCCTCA |
| | | | SEQ ID NO: 25415 | SEQ ID NO: 29421 |

FIGURE 50

| | | | | |
|---|---|---|---|---|
| | | AA | DIQMTQSPSSLSASVGDRVTITCRASQDIRNILG WYQQKPGKAPKRLIYAASNLQSGVPSRFSGSGS GTEFTLTISSLQPEDFATYYCLQYNSYPWTFGQG TKVEIK | EVQLVESGGGLVKPGGSLRLSCAASGFTFSSYSMI WVRQAPGKGLEWVSSISGTSSYIYYADSVKGRFTIS RDNAKNSLYLQMNSLRAEDTAVYYCAGMTTVIW GQGTLVTVSS |
| | | | SEQ ID NO: 25416 | SEQ ID NO: 29422 |
| **iPS:434137** | **21-225_54D4** | NA | GATATTGTGATGACCCAGACTCCACTCTCTCT GTCCGTCACCCCTGGACAGCCGGCCTCCATGT CCTGCAAGTCTAGTCAGAGCCTCCTGCATAGT GAGGGAAAGACCTATTTGTATTGGTACCTGCG GAAGCCAGGCCAGCCTCCACAGTTCCTGATCT TTGAAGTTTCCAACCGGTTCTCTGGAGTGCCA GATAGGTTCAGTGGCAGCGGGTCAGGGACAG AATTCACACTGAAAATCAGCCGGGTGGAGGCT GAGGATGTTGGGATTTATTACTGCATGCAAAG TATACAGTTTCCATTCACTTTCGGCCCTGGGAC CAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAAGGTATAG CAGCAGCTGGTCGGGCGGTATGGACGTCTGGGG CCAAGGGACCACGGTCACCGTCTCCTCA |
| | | | SEQ ID NO: 25417 | SEQ ID NO: 29423 |
| | | AA | DIVMTQTPLSLSVTPGQPASMSCKSSQSLLHSEG KTYLYWYLRKPGQPPQFLIFEVSNRFSGVPDRFS GSGSGTEFTLKISRVEAEDVGIYYCMQSIQFPFTF GPGTKVDIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARRYSSS WSGGMDVWGQGTTVTVSS |
| | | | SEQ ID NO: 25418 | SEQ ID NO: 29424 |

FIGURE 50

| | | | | |
|---|---|---|---|---|
| **iPS:434141** | **21-225_54C6** | NA | GACATCCAGATGACCCAGTCTCCATCCTCCCTGTCTGCCTCTGTAGGCGCCAGAGTCACCATCACTTGCCGGGCAAGTCAGGGCATTAGAAATGATTTAGGCTGGTATCAGCAGAATCCGGGGAAAGCCCCTAAGCGCCTGATCTATGCTGCATCCAATTTGCAAAGTGGGGTCCCATCAAGGTTCAGCGGCAGTGGATCTGGGACAGAATTCACTCTCACTATCAGCAGCCTGCAGCCTGAAGATTTTGCAACTTATTTCTGTCTACAGCATAATCGTTACCCGCTCACTTTCGGCGGAGGGACCAAGGTGGAGATCAAA | CAGGTCCAGCTGGTGGAGTCTGGGGGAGGCGTGGTCCAGCCTGGGAGGTCCCTGAGACTCTCCTGTACAACGTCTGGATTCACCTTCAGTGACTATGGCATCCACTGGGTCCGCCAGGCTCCAGGCAAGGGGCTGGACTGGGTGGCAGTTATATGGTATGATGAAAATAATAAATACTATGCAGACTCCGTGAAGGGCCGATTCGCCATCTCCAGAGACAATTCCAAGAACACGCTGTATCTGCAAATGAACAGCCTGAGAGCCGAGGACACGGCTGTGTATTATTGTGTGAGAGAACTGGGGATGACGTCTGACTACTGGGGCCAGGGAACCCTGGTCACCGTCTCCTCA |
| | | | SEQ ID NO: 25419 | SEQ ID NO: 29425 |
| | | AA | DIQMTQSPSSLSASVGARVTITCRASQGIRNDLGWYQQNPGKAPKRLIYAASNLQSGVPSRFSGSGSGTEFTLTISSLQPEDFATYFCLQHNRYPLTFGGGTKVEIK | QVQLVESGGGVVQPGRSLRLSCTTSGFTFSDYGIHWVRQAPGKGLDWVAVIWYDENNKYYADSVKGRFAISRDNSKNTLYLQMNSLRAEDTAVYYCVRELGMTSDYWGQGTLVTVSS |
| | | | SEQ ID NO: 25420 | SEQ ID NO: 29426 |
| **iPS:434143** | **21-225_54G7** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCTAGGTTCAGCGGCAGTGGATCTGGGACAGAATTCACTCTCACAATCAGCAGCCTGCAGCCTGAAGATTTTGCAACTTATTACTGTCTACATCATAATACTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGAGGAAAGTAATAAATACTATGGAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAATTGGGGTTCCTCTCTGACTACTGGGGCCAGGGAACCCTGGTCACCGTCTCCTCA |
| | | | SEQ ID NO: 25421 | SEQ ID NO: 29427 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLHHNTYPFTFGPGTKVDIK | QVQLVESGGGVVQPGRSLRLSCAASGFTFSNYGMHWVRQAPGKGLEWVAVIWYEESNKYYGDSVKGRFTISRDNSKNTLYLQMNSLRAEDTAVYYCARELGFLSDYWGQGTLVTVSS |
| | | | SEQ ID NO: 25422 | SEQ ID NO: 29428 |
| iPS:434145 | 21-225_55B1 | NA | GACATCCAGATGACCCAGTCTCCATCTTCCGTGTCTGCATCTGTAGGGGACAGAGTCACCATCACTTGTCGGGCGAGTCAGGTTATTAGCCGCTGGTTAGCCTGGTATCAGCAGAAACCAGGGAAAGCCCCTAACCTCCTGATCTATGCTGCATCCAGTTTGCAAAGTGGGGTCCCATCAAGGTTCAGCGGCAGTGGATCTGGGACAGATTTCACTCTGACCATCAGCAGCCTGCAGCCTGAAGATTTTGCAACTTACTATTGTCAACAGGCTAACAGTTTCCCATTCACTTTCGGCCCTGGGACCAAAGTGGATCTCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATTTCCACTGGGTGCGACAGGCCCCTGGTCAAGGGCTTGAGTGGATGGGATGGATCCACCCTAACAATAATGCCACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAAGGATGGCAGATCGTCCTTTGACTACTGGGGCCAGGGAACCCTGGTCACCGTCTCCTCA |
| | | | SEQ ID NO: 25423 | SEQ ID NO: 29429 |
| | | AA | DIQMTQSPSSVSASVGDRVTITCRASQVISRWLAWYQQKPGKAPNLLIYAASSLQSGVPSRFSGSGSGTDFTLTISSLQPEDFATYYCQQANSFPFTFGPGTKVDLK | QVQLVQSGAEVKKPGASVKVSCKASGYTFTGYYFHWVRQAPGQGLEWMGWIHPNNNATNYAQKFQGRVTMTRDTSISTAYMELSRLRSDDTAVYYCAKDGRSSFDYWGQGTLVTVSS |
| | | | SEQ ID NO: 25424 | SEQ ID NO: 29430 |

FIGURE 50

| | | | | |
|---|---|---|---|---|
| **iPS:434147** | **21-225_55E1** | NA | GAAATAGTGATGACGCAGTCTCCAGCCACCCT GTCTGTGTCTCCAGGGGAAAGAGCCACCCTCT CCTGCAGGGCCAGTCAGAGTGTTAGCAGCGAC TTAGCCTGGTACCAGCTGAAACCTGGCCAGGC TCCCAGGCTCCTCATCTATGATGCATCCGCCA GGGCCACTGGTATCCCAGCCAGGTTCAGTGGC AGTGGGTCTGGGACAGAGTTCACTCTCACCAT CAGCAGCCTGCAGTCTGAAGATTTTGCATTTT ATTACTGTCAGCAGTATTATAACTGGCCTCTC ACTTTCGGCGGAGGGACCAAGGTGGAGATCA AA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTAGT AGCACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTTTTTTACTGTGCGAAAGATCACGGT ATAGTGGGAACTATTTACTTTGACTACTGGGGCC AGGGAACCCTGGTCACCGTCTCCTCA |
| | | | SEQ ID NO: 25425 | SEQ ID NO: 29431 |
| | | AA | EIVMTQSPATLSVSPGERATLSCRASQSVSSDLA WYQLKPGQAPRLLIYDASARATGIPARFSGSGSG TEFTLTISSLQSEDFAFYYCQQYYNWPLTFGGGT KVEIK | EVQLLESGGGLVQPGGSLRLSCAASGFTFSSYAMS WVRQAPGKGLEWVSAISGRGSSTFYADSVKGRFTI SRDNSKNTLYLQMNSLRAEDTAVFYCAKDHGIVG TIYFDYWGQGTLVTVSS |
| | | | SEQ ID NO: 25426 | SEQ ID NO: 29432 |
| **iPS:434149** | **21-225_55H1** | NA | GATATTGTGATGACCCAGACTCCACTCTCTCT GTCCGTCACCCCTGGACAGCCGGCCTCCATCT CCTGCAAATCTAGTCAGAGCCTCCTGCATAGT GAAGGAAAGACCTATTTGTATTGGTACCTGCA GAAGCCAGGCCAGCCTCCACAGTTCCTGATCT TTGAAGTTTCCCACCGGTTCTCTGGAGTGCCA GATAGGTTCAGTGGCAGCGGGTCAGGGACAG ATTTCACTCTGAAAATCAGCCGGGTGGAGGCT GAGGATGTTGGGGTTTATTACTGCATGCAAAG TATACAGCTTCCATTCACTTTCGGCCCTGGGAC CAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAAGGTATAG CAGCAGCTGGTCGGGCGGTATGGACGTCTGGGG CCAAGGGACCACGGTCACCGTCTCCTCA |
| | | | SEQ ID NO: 25427 | SEQ ID NO: 29433 |

FIGURE 50

| | | | | |
|---|---|---|---|---|
| | | AA | DIVMTQTPLSLSVTPGQPASISCKSSQSLLHSEGKTYLYWYLQKPGQPPQFLIFEVSHRFSGVPDRFSGSGSGTDFTLKISRVEAEDVGVYYCMQSIQLPFTFGPGTKVDIK | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARRYSSSWSGGMDVWGQGTTVTVSS |
| | | | SEQ ID NO: 25428 | SEQ ID NO: 29434 |
| iPS:434151 | 21-225_55C2 | NA | GATGTTATGATGACCCAGATTCCACTCTCTCTGTCCGTCACCCCTGGACAGCCGGCCTCCATCTCCTGCAAGTCTAGTCAGAGCCTCGTGCATAGTGAAGGAAAGACCTATTTGTATTGGTATTTGCAGAAGCCAGGCCAGCCTCCACAGCTCCTGATCTTTGAAGTTTCCAACCGGGTCTCTGGAGTGCCAGATAGGTTCAGTGGCAGAGGGTCAGGGACAGATTTCACACTGAAAATCAGCCGGGTGGAGGCTGAGGATGTTGGGGTTTATTACTGCATGCAAAGTATACTGTATCCGATCACCTTCGGCCAAGGGACACGACTGGAGATTAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGTTATGGCATGCACTGGGTCCGCCAGGCTCCAGGCAAGGGGCTGGAGTGGGTGGCACTTATATGGTATGATGGAAGTAATAAATACCATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGCGCGAGAAGGTATAGCAGCAGCTGGTCGGGCGGTATGGACGTCTGGGGCCAAGGGACCACGGTCACCGTCTCCTCA |
| | | | SEQ ID NO: 25429 | SEQ ID NO: 29435 |
| | | AA | DVMMTQIPLSLSVTPGQPASISCKSSQSLVHSEGKTYLYWYLQKPGQPPQLLIFEVSNRVSGVPDRFSGRGSGTDFTLKISRVEAEDVGVYYCMQSILYPITFGQGTRLEIK | QVQLVESGGGVVQPGRSLRLSCAASGFTFSSYGMHWVRQAPGKGLEWVALIWYDGSNKYHADSVKGRFTISRDNSKNTLYLQMNSLRAEDTAVYYCARRYSSSWSGGMDVWGQGTTVTVSS |
| | | | SEQ ID NO: 25430 | SEQ ID NO: 29436 |

FIGURE 50

| | | | | |
|---|---|---|---|---|
| **iPS:434155** | **21-225_55B3** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTAGATCTGGGACAGAATTCACTCTCACAATCAGCAGCCTGCAGCCTGAAGATTTTGCATCTTATTACTGTCTACAACATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTTTGATGGAAATAATAAATACTATGAAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGTGAGGGAACTGGGATTTCTCTCTGACTACTGGGGCCAGGGAACCCTGGTCACCGTCTCCTCA |
| | | | SEQ ID NO: 25431 | SEQ ID NO: 29437 |
| | | AA | DIQMTQSPSSLSASVGDRVTITCRASQGIRNDLGWYQQKPGKAPKRLIYAASSLQSGVPSRFSGSRSGTEFTLTISSLQPEDFASYYCLQHNSYPFTFGPGTKVDIK | QVQLVESGGGVVQPGRSLRLSCAASGFTFSSYGMHWVRQAPGKGLEWVAVIWFDGNNKYYEDSVKGRFTISRDNSKNTLYLQMNSLRAEDTAVYYCVRELGFLSDYWGQGTLVTVSS |
| | | | SEQ ID NO: 25432 | SEQ ID NO: 29438 |
| **iPS:434157** | **21-225_55D4** | NA | GACATCCAGATGACCCAGTCTCCATCCTCTCTGTTTGCATCTGTAGGAGACAGAGTCACCATCACTTGTCGGGCGAGTCAGGACATTAGCAATTATTTAATCTGGTTTCAGCAGAAACCAGGGAAAGCCCCTAAGTCCCTGATTTATACTGCATCCAGTTTGCAAAGTGGGGTCCCCTCAAAGTTCAGCGGCAGTGGATTTGGGACAGATTTCACTCTCACCATCAGCAACCTGCAGCCTGAAGATTTTGCGACTTATTACTGCCAACAGTATCATAGTTTCCCTCTCACTTTCGGCGGAGGGACCAGGGTGGAGATCAGA | GAGGTGCAACTGGTGGAGTCTGGGGGAGGCCTGGTCAAGCCTGGGGGGTCCCTGAGACTCTCCTGTGCAGCCTCTGGATTCACCTTCAATAGTTATAGGATGAACTGGGTCCGCCAGGCTCCAGGGAAGGGGCTGGAGTGGGTCTCATCCATTAGTAGTAGTAGTAATCACATAGACTACGCAGACTCAGTGAAGGGCCGATTCACCATCTCCAGAGACAACGCCAAAAACTCACTATATTTGCAAATGAACAGCCTGAGAGCCGAGGACACGGCTTTATATTACTGTGCGAGAGGGACTGACTACTGGGGCCAGGGAACCCTGGTCTCCGTCTCCTCA |
| | | | SEQ ID NO: 25433 | SEQ ID NO: 29439 |

FIGURE 50

| | | | | |
|---|---|---|---|---|
| | | AA | DIQMTQSPSSLFASVGDRVTITCRASQDISNYLIWFQQKPGKAPKSLIYTASSLQSGVPSKFSGSGFGTDFTLTISNLQPEDFATYYCQQYHSFPLTFGGGTRVEIR | EVQLVESGGGLVKPGGSLRLSCAASGFTFNSYRMNWVRQAPGKGLEWVSSISSSSNHIDYADSVKGRFTISRDNAKNSLYLQMNSLRAEDTALYYCARGTDYWGQGTLVSVSS |
| | | | SEQ ID NO: 25434 | SEQ ID NO: 29440 |
| iPS:434159 | 21-225_55B8 | NA | GACATCCAGATGACCCAGTCTCCATCCTCCCTGTCTTCATCTGTAGGAGACAGAGTCACCATCACTTGCCGGGCAAGTCAGGCCATTAGAAATGATTTAGGCTGGTATCAGCAGAAACCAGGGAAAGCCCCTAAGCGCCTGATCCATGCTGCATTCAGGTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCTCTCACTTTCGGCGGAGGGACCAAGGTGGGGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGAAAATAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTACGAGAGAATGGTTTGACTACTGGGGCCAGGGAACCCTGGTCACCGTCTCCTCA |
| | | | SEQ ID NO: 25435 | SEQ ID NO: 29441 |
| | | AA | DIQMTQSPSSLSSSVGDRVTITCRASQAIRNDLGWYQQKPGKAPKRLIHAAFRLQSGVPSRFSGSGSGTEFTLTISSLQPEDFATYYCLQHNSYPLTFGGGTKVGIK | QVQLVESGGGVVQPGRSLRLSCAASGFTFSDYGMHWVRQAPGKGLEWVAVIWYDENNKYYADSVKGRFTISRDNSKNTLYLQMNSLRAEDTAVYYCTREWFDYWGQGTLVTVSS |
| | | | SEQ ID NO: 25436 | SEQ ID NO: 29442 |

FIGURE 50

| | | | | |
|---|---|---|---|---|
| **iPS:434161** | **21-225_55F9** | NA | GATATTGTGATGACCCAGACTCCACTCTCTCT<br>GTCCGTCACCCCTGGACAGTCGGCCTCCATCT<br>CCTGCAAGTCTAGTCAAAGCCTCCTGCATAGT<br>GAAGGAAAGACCTATTTGTATTGGTACTTGCA<br>GAAGCCAGGCCAGCCTCCACAGCTCCTGATCT<br>ATGAAGTTTCCAACCGGTTCTCTGGAGTGCCA<br>GATAGGTTCAGCGGCAGCGGGTCAGGGACAG<br>ATTTTACACTGAAAATCAGCCGGGTGGAGGCT<br>GAGGATGTTGGGGTTTATTACTGCATACAAAG<br>TATACAACTTCCGATCACCTTCGGCCAAGGGA<br>CACGACTGGAGATTAAA | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTAGCTATGGCAT<br>GCACTGGGTCCGCCAGGCTCCAGGCAAGGGGCT<br>GGAGTGGGTGGCAGTTATATGGTATGATGGAAAT<br>GGCAAATATTATGCAGACTCCGTGAAGGGCCGA<br>TTCACCATCTCCAGAGACAATTCCAAGAACACGC<br>TGTATCTGCAAATGAACAGCCTGAGAGCCGAGG<br>ACACGGCTGTGTATTACTGTGCGAGAAGGTATAG<br>CAGCAGCTGGTCGGGCGGTATGGACGTCTGGGG<br>CCAAGGGACCACGGTCACCGTCTCCTCA |
| | | | SEQ ID NO: 25437 | SEQ ID NO: 29443 |
| | | AA | DIVMTQTPLSLSVTPGQSASISCKSSQSLLHSEGK<br>TYLYWYLQKPGQPPQLLIYEVSNRFSGVPDRFSG<br>SGSGTDFTLKISRVEAEDVGVYYCIQSIQLPITFG<br>QGTRLEIK | QVQLVESGGGVVQPGRSLRLSCAASGFTFSSYGMH<br>WVRQAPGKGLEWVAVIWYDGNGKYYADSVKGRF<br>TISRDNSKNTLYLQMNSLRAEDTAVYYCARRYSSS<br>WSGGMDVWGQGTTVTVSS |
| | | | SEQ ID NO: 25438 | SEQ ID NO: 29444 |
| **iPS:434163** | **21-225_50H1** | NA | GACATCCAGATGACCCAGTCTCCATCCTCCCC<br>GTCTGCTTCTGTAGGAGACAGAGTCACCATCA<br>CTTGCCAGGCGAGTCAAGACATTAGCAACTAT<br>TTAGATTGGTATCAGCAGAAACCAGGGAAAG<br>CCCCTAAACTCCTGATATACGATGCATCCAAT<br>TTGGAAACAGGGGTCCCATCAAGGTTCAGTGG<br>AAGTGGATCTGGGACAGATTTTGCTTTCACCA<br>TCAGCAGCCTGCAGCCTGAAGATATTGCAACA<br>TATTACTGTCAACAGTGTGATAATCTCCCGCTC<br>ACTTTCGGCGGAGGGACCAAGGTGGAGATCA<br>AA | CAGGTGCAGTTGGTGGAGTCTGGGGGAGGCGTG<br>GTCCAGCCTGGGAGGTCCCTGAGACTCTCCTGTG<br>CAGCCTCTGGATTCACCTTCAGTAGCTATGGCAT<br>GCACTGGGTCCGCCAGGCTCCAGGCAAGGGGCT<br>GGAGTGGGTGTCAATTATATCATATGGTGGAAGT<br>AATAAATACGATGCAGACTCCGTGAAGGGCCGA<br>TTCACCATCTCCAGAGACAATTCCAAGAACACGC<br>TGTATCTGCAAATGACCAGCCTGAGAGCTGAGG<br>ACACGGCTGTGTATTACTGTGCGAGACGACGGGC<br>AGCTCGTCCAGGGTACGGTATGGACGTCTGGGGC<br>CAAGGGATCACGGTCACCGTCTCCTCA |
| | | | SEQ ID NO: 25439 | SEQ ID NO: 29445 |

FIGURE 50

| | | | | |
|---|---|---|---|---|
| | | AA | DIQMTQSPSSPSASVGDRVTITCQASQDISNYLDWYQQKPGKAPKLLIYDASNLETGVPSRFSGSGSGTDFAFTISSLQPEDIATYYCQQCDNLPLTFGGGTKVEIK | QVQLVESGGGVVQPGRSLRLSCAASGFTFSSYGMHWVRQAPGKGLEWVSIISYGGSNKYDADSVKGRFTISRDNSKNTLYLQMTSLRAEDTAVYYCARRRAARPGYGMDVWGQGITVTVSS |
| | | | SEQ ID NO: 25440 | SEQ ID NO: 29446 |
| iPS:434165 | 21-225_50F2 | NA | GACATCCAGATGACCCAGTCTCCATCCTCCCTGTCTGCATCTGTAGGAGACAGAATCACCATCACTTGCCGGGCAAGTCAGAGCATTCTCAGCTATTTGAATTGGTATCAGCAGAAACCAGGAAAGGCCCCTAAACTCCTGATCTATGTTGCATCCAGTTTCCAAAGTGGGGTCCCATCAAGGTTCAGTGGCAGTGGATCTGGGACAGATTTCACTCTCACCATCAGCAGTCTGCAACCTGATGATTTTGCAACTTACTACTGTCAACAGAGTTACAGTCCCCCTCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGCATTCACCTTCAGTAGCTATGGCATGCACTGGGTCCGCCAGACTCCAGGCAAGGGGCTGGAGTGGGTGGCAGTTATATGGCATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGACGAGCAGCTCGGGACCTTTGACTACTGGGGCCAGGGAACCCTGGTCACCGTCTCCTCA |
| | | | SEQ ID NO: 25441 | SEQ ID NO: 29447 |
| | | AA | DIQMTQSPSSLSASVGDRITITCRASQSILSYLNWYQQKPGKAPKLLIYVASSFQSGVPSRFSGSGSGTDFTLTISSLQPDDFATYYCQQSYSPPLTFGGGTKVEIK | QVQLVESGGGVVQPGRSLRLSCAASAFTFSSYGMHWVRQTPGKGLEWVAVIWHDGSNKYYADSVKGRFTISRDNSKNTLYLQMNSLRAEDTAVYYCARDEQLGTFDYWGQGTLVTVSS |
| | | | SEQ ID NO: 25442 | SEQ ID NO: 29448 |

FIGURE 50

| | | | | |
|---|---|---|---|---|
| iPS:434167 | 21-225_50F3 | NA | GACATCCAGATGACCCAGTCTCCATCTTCCGT<br>GTCTGCATCTGTAGGAGACAGAGTCACCATCA<br>CTTGTCGGGCGAGTCAGGATATTAGCAGCTGG<br>TTGGCCTGGTTTCAGCAGAAACCAGGGAAAGC<br>CCCTAAGCTCCTGATCTATGCTGCATCCAGTTT<br>GCAAAATGGGGTCCCGTCAAGGTTCAGCGGCA<br>GTGGATCTGGGACAGATTTCACTCTCACCATC<br>AGCAGCCTGCAGCCTGAAGATTTTGCAACTTA<br>CTATTGCCAACAGACTAACAGTTTCCCATTCA<br>CTTTCGGCCCTGGGACCAAAGTGGATATCAAA | GAGGTGCAGCTGTTGGAGTCTGGGGGAGGCTTG<br>GTACAGCCTGGGGGGTCCCTGAGACTCTCCTGTG<br>CAGCCTCTGGATTCACCTTTAGAACCTATGCCAT<br>GACCTGGGTCCGCCAGGCTCCAGGGAAGGGGCT<br>GGAGTGGGTCTCAGCTATCAGTGGTAGTGGTGTT<br>AACTCATTCTACGCAGACTCCGTGAAGGGCCGGT<br>TCACCATCTCCAGAGACAATTCCGAGAACACGCT<br>GTATCTGCAAATGAACAGCCTGAGAGCCGAGGA<br>CACGGCCGTATATTACTGTGCGAAAGCAAGGGC<br>AGTGGCTGGGTCACACTGGTTCGACCCCTGGGGC<br>CAGGGAACCCTGGTCACCGTCTCCTCA |
| | | | SEQ ID NO: 25443 | SEQ ID NO: 29449 |
| | | AA | DIQMTQSPSSVSASVGDRVTITCRASQDISSWLA<br>WFQQKPGKAPKLLIYAASSLQNGVPSRFSGSGS<br>GTDFTLTISSLQPEDFATYYCQQTNSFPFTFGPGT<br>KVDIK | EVQLLESGGGLVQPGGSLRLSCAASGFTFRTYAMT<br>WVRQAPGKGLEWVSAISGSGVNSFYADSVKGRFTI<br>SRDNSENTLYLQMNSLRAEDTAVYYCAKARAVAG<br>SHWFDPWGQGTLVTVSS |
| | | | SEQ ID NO: 25444 | SEQ ID NO: 29450 |
| iPS:434169 | 21-225_50C4 | NA | GACATCCAGATGACCCAGTCTCCATCCTCCCT<br>GTCTGCATCTGTAGGAGACAGAGTCACCCTCA<br>CTTGCCGGGCAAGTCAGGGCATTAGAAATGAT<br>TTAGGCTGGTATCAGCAGAAACCAGGGATAGC<br>CCCTAAGCGCCTGATCTATGCTGCATCCAGTTT<br>GCAAAGTGGGGTCCCATCAAGGTTCAGCGGCA<br>GTGGATCTGGGACAGAATTCACTCTCACAATC<br>AGCAGCCTGCAGCCTGAAGATTTTGCAACTTA<br>TTACTGTCTACAGCATAATCGTTACCCATTCAC<br>TTTCGGCCCTGGGACCAAAGTGGATATCAAA | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTGACTATGGCAT<br>GCACTGGGTCCGCCAGGCTCCAGGCAAGGGGCT<br>GGAGTGGGTGGCAGTTATATGGTATGAAGAAAC<br>TAATAAATACTATGCAGACTCCGTGAAGGGCCG<br>ATTCACCATCTCCAGAGACAATTCCAAGAACACG<br>CTGTATCTGCAAATGAACAGCCTGAGAGGCGAG<br>GACACGGCTGTGTATTACTGTGCGAGAGAAGTGG<br>GGTTCCTGAATGACTACTGGGGCCAGGGAATCCT<br>GGTCACCGTCTCCTCA |
| | | | SEQ ID NO: 25445 | SEQ ID NO: 29451 |

FIGURE 50

| | | | | |
|---|---|---|---|---|
| | | AA | DIQMTQSPSSLSASVGDRVTLTCRASQGIRNDLGWYQQKPGIAPKRLIYAASSLQSGVPSRFSGSGSGTEFTLTISSLQPEDFATYYCLQHNRYPFTFGPGTKVDIK | QVQLVESGGGVVQPGRSLRLSCAASGFTFSDYGMHWVRQAPGKGLEWVAVIWYEETNKYYADSVKGRFTISRDNSKNTLYLQMNSLRGEDTAVYYCAREVGFLNDYWGQGILVTVSS |
| | | | SEQ ID NO: 25446 | SEQ ID NO: 29452 |
| **iPS:434171** | **21-225_50G4** | NA | GACATCCAGATGACCCAGTCTCCAACCTCCCTGTCTGCATCTGTGGGAGACAGAGTCACCATCACTTGCCAGGCGAGTCAGGACATTACCAACTTTTTAAATTGGTATCAGCAGAAACCAGGGAAAGCCCCTAAGCTCCTGATCTACGATGCCTCCAATTTGGAAACAGGGGTCCCATCAAGGTTCAGTGGAAGTGGATCTGGGACAGATTTTACTTTCACCATCAGCAGCCTTCAGCCTGAAGATATTGCAACATATTACTGTCAACAGTATGATAATCTGATCACCTTCGGCCAAGGGACACGACTGGAGATTAAA | CAGGTGCAGCTGGTGCAGTCTGGGGCTGAGGTGAAGGAGCCTGGGGCCTCAGTGAAGGTTTCCTGCAAGGCATCTGGATACACCTTCACCAGTTACTATATACACTGGGTGCGACAGGCCCCTGGACAAGGGCTTGAGTGGATGGGAGTAATCAACCCTAGTAATGGTAGAACAAGCTACGCACAGAAGTTCCAGGGCAGAGTCACCATGACCAGGGACACGTCCACGAGCACAGTCTACATGGAGCTGAGCAGCCTGAGATCTGAGGACACGGCCGTGTATTACTGTGCGAGAGATCGAGGAGATGGTTACTACTTCTACTACGGTATGGACGTCTGGGGCCAAGGGACCACGGTCACCGTCTCCTCA |
| | | | SEQ ID NO: 25447 | SEQ ID NO: 29453 |
| | | AA | DIQMTQSPTSLSASVGDRVTITCQASQDITNFLNWYQQKPGKAPKLLIYDASNLETGVPSRFSGSGSGTDFTFTISSLQPEDIATYYCQQYDNLITFGQGTRLEIK | QVQLVQSGAEVKEPGASVKVSCKASGYTFTSYYIHWVRQAPGQGLEWMGVINPSNGRTSYAQKFQGRVTMTRDTSTSTVYMELSSLRSEDTAVYYCARDRGDGYYFYYGMDVWGQGTTVTVSS |
| | | | SEQ ID NO: 25448 | SEQ ID NO: 29454 |

FIGURE 50

| | | | | |
|---|---|---|---|---|
| iPS:434175 | 21-225_55A11 | NA | GACATCCAGATGACCCAGTCTCCATCCTCACTGTCTGCATCTGTAGGAGACAGAGTCACCATCACTTGTCGGGCGAGTCAGGACATTAACATTTATTTAGCCTGGTTTCAGCAGAAACCAGGGAAAGCCCCTAAGTCCCTGATCTATGCTGCATCCAGTTTGCAAAGTGGGGTCCCATCGAAGTTCAGCGGCAGTGGATCTGGGACAGATTTCACTCTCACCATCAGCAGTCTGCAGCCTGAAGATTTTGCAACTTATTACTGCCAACAGTATAATAGTTATCCTCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGACTCCAGGCAAGGGGCTGGAGTGGGTGGCAGTTATATCATATGTTGGAAGTACTAAATACTATGCAGACTCCGTGAGGGGCCGATTCACCATCTCCAGAGACAATTCCAAGAACACGCTGTATCTGCAAATGAACAGCCTGAGAACTGAGGACACGGCTGTGTATTACTGTGCGAGAGGGAGAGGTCGATATAGTGACTACGGTCATGATGCTTTTGATATCTGGGGCCAAGGGACAATGGTCACCGTCTCTTCA |
| | | | SEQ ID NO: 25449 | SEQ ID NO: 29455 |
| | | AA | DIQMTQSPSSLSASVGDRVTITCRASQDINIYLAWFQQKPGKAPKSLIYAASSLQSGVPSKFSGSGSGTDFTLTISSLQPEDFATYYCQQYNSYPLTFGGGTKVEIK | QVQLVESGGGVVQPGRSLRLSCAASGFTFSSYGMHWVRQTPGKGLEWVAVISYVGSTKYYADSVRGRFTISRDNSKNTLYLQMNSLRTEDTAVYYCARGRGRYSDYGHDAFDIWGQGTMVTVSS |
| | | | SEQ ID NO: 25450 | SEQ ID NO: 29456 |
| iPS:434177 | 21-225_56A1 | NA | GACATCGTCATGACCCAGTCTCCAGACTCCCTGGCTGTGTCTCTGGGCGAGAGGGCCACCATCAACTGCAAGTCCAGCCAGAGTGTTTTACATAGTTCCAACAATAAGAACTACTTAGTTTGGTACCAGCAGAGACCAGGACAGCCTCCTAACCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCTCCGACGTTCGGCCAAGGGACCAAGGTGGAAATCAAA | CAGGTACAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGCTGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCATATAGCAGTGGCTGGTACGTTTTTGACTACTGGGGCCAGGGAACCCTGGTCACCGTCTCCTCA |
| | | | SEQ ID NO: 25451 | SEQ ID NO: 29457 |

FIGURE 50

| | | | | |
|---|---|---|---|---|
| | | AA | DIVMTQSPDSLAVSLGERATINCKSSQSVLHSSNNKNYLVWYQQRPGQPPNLLIYWASTRESGVPDRFSGSGSGTDFTLTISSLQAEDVAVYYCQQYYSTPPTFGQGTKVEIK | QVQLVQSGAEVKKPGASVKVSCKASGYTFTNYDINWVRQATGQGLEWLGWMHPNSGNTGYAQKFQGRVTMTRNTSISTAYMELSSLRSEDTAVYYCAYSSGWYVFDYWGQGTLVTVSS |
| | | | SEQ ID NO: 25452 | SEQ ID NO: 29458 |
| iPS:434179 | 21-225_56F1 | NA | GACATCCAGATGACCCAGTCTCCATCCTCCCTGTCTGCATCTGTAGGAGACAGAGTCACCATCACTTGCCGGGCAAGTCAGGACATTAGAAATAATTTAGGCTGGTATCAGCAGAAACCAGGGAAAGCCCCTAAGCGCCTGATCAATGCTGCATCCAGTTTACAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGGATAATAGTCACCCGTTCACTTTCGGCGGAGGGACCAAGGTGGAGATCAAA | GAGGTGCAGCTGGTGGAGTCTGGGGGAGGCCTGGTCAAGTTTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTAGTAGTAGTACTTACATATACTACGGAGACTCAGTGAAGGGCCGATTCACCATCTCCCGAGACAACGCCAAGAACTCACTATATCTGCAGATGAACAGCCTGAGAGCCGAGGACACGGCTGTGTATTACTGTGCGAGAGATCGGGGCAGCAGCTGGGGCCAGGGAACCCTGGTCACCGTCTCCTCA |
| | | | SEQ ID NO: 25453 | SEQ ID NO: 29459 |
| | | AA | DIQMTQSPSSLSASVGDRVTITCRASQDIRNNLGWYQQKPGKAPKRLINAASSLQSGVPSRFSGSGSGTEFTLTISSLQPEDFATYYCLQDNSHPFTFGGGTKVEIK | EVQLVESGGGLVKFGGSLRLSCAASGFTFSSYSMNWVRQAPGKGLEWVSSISSSSTYIYYGDSVKGRFTISRDNAKNSLYLQMNSLRAEDTAVYYCARDRGSSWGQGTLVTVSS |
| | | | SEQ ID NO: 25454 | SEQ ID NO: 29460 |

FIGURE 50

| | | | | |
|---|---|---|---|---|
| **iPS:434181** | **21-225_56B2** | NA | GACATCCAGATGACCCAGTCTCCATCCTCCCTGTCTGCATCTGTAGGAGACAGAGTCACCATCACTTGCCGGGCAAGTCAGAGTTTTAGCCACTATTTAAATTGGTATCAGCAGAAACCAGGGAAAGCCCCTAACCTCCTGATCTTTGCTGTATCCAGTTTGCAAAGTGGGGTCCCATCAAGGTTCAGTGGCAGTGGATCTGGGACAGATTTCACTCTCACCATCAGTAGTCTGCAACCTGAAGATTTTGCAATTTACTTCTGTCAACAGAGTTACAGTACCCCATTCACTTTCGGCCCTGGGACCAAAGTGGATATCAAA | GAGGTGCAGTTGTTGGAGTCTGGGGGAGGCTTGATACAGCCTGGGGGGTCCCTGAGACTCTCCTGTGCAGCCTCTGGATTCACCTTTAGGAGCTATGCCATGAGCTGGGTCCGCCAGGCTCCAGGGAAGGGGCTGGAGTGGGTCTCAGCTATTAGTGGTAGTGGTGGTAACACATTCTACGCAGACTCCGTGAAGGGCCGGTTCACCATCTCCAGAGACAATTCCAAGAACACGCTGTATTTGCAAATGAACACCCTGAGAGCCGACGACACGGCCGTATATTACTGTGCGAAAAAGGTCGTGGATACAGCCATGGCTCTTGACTACTGGGGCCAGGGAACCCTGGTCACCGTCTCCTCA |
| | | | SEQ ID NO: 25455 | SEQ ID NO: 29461 |
| | | AA | DIQMTQSPSSLSASVGDRVTITCRASQSFSHYLNWYQQKPGKAPNLLIFAVSSLQSGVPSRFSGSGSGTDFTLTISSLQPEDFAIYFCQQSYSTPFTFGPGTKVDIK | EVQLLESGGGLIQPGGSLRLSCAASGFTFRSYAMSWVRQAPGKGLEWVSAISGSGGNTFYADSVKGRFTISRDNSKNTLYLQMNTLRADDTAVYYCAKKVVDTAMALDYWGQGTLVTVSS |
| | | | SEQ ID NO: 25456 | SEQ ID NO: 29462 |
| **iPS:434187** | **21-225_56A5** | NA | GACATCCAGATGACCCAGTCTCCATCCTCCCTGTCTGCATCTGTAGGAGACAGAGTCACCATCACTTGCCGGGCAAGTCAGGACATTAGAAATCTTTTAGGCTGGTATCAGCAGAAACCAGGGAAAGCCCCTAAGCGCTTGATCTATGCTGCATCCAGTTTGCAAAGTGGGGTCCCATCAAGGTTCAGCGGCAGTGGATCTGGGACAGAGTTCACTCTCACAATCAGCAGCCTGCAGCCTGAAGATTTTGCAACTTATTACTGTCTACAGTATAATAGTTACCCATTCACTTTCGGCCCTGGGACCAAAGTGGATATCAGA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTAGTTACATATACTACGCAGACTCAGTGAAGGGCCGATTCACCATCTCCAGAGACAACGCCAAGAACTCACTATATCTGCAAATGAACAGCCTGAGAGCCGAGGACACGGCTGTGTATTACTGTGCGAGAGTGGCTACTTTTGACTACTGGGGCCAGGGAACCCTGGTCACCGTCTCCTCA |
| | | | SEQ ID NO: 25457 | SEQ ID NO: 29463 |

FIGURE 50

| | | | | |
|---|---|---|---|---|
| | | AA | DIQMTQSPSSLSASVGDRVTITCRASQDIRNLLGWYQQKPGKAPKRLIYAASSLQSGVPSRFSGSGSGTEFTLTISSLQPEDFATYYCLQYNSYPFTFGPGTKVDIR | EVQLVESGGGLVKPGGSLRLSCAASGFTFSSYSMNWVRQAPGKGLEWVSSISGSSSYIYYADSVKGRFTISRDNAKNSLYLQMNSLRAEDTAVYYCARVATFDYWGQGTLVTVSS |
| | | | SEQ ID NO: 25458 | SEQ ID NO: 29464 |
| iPS:434189 | 21-225_56E5 | NA | GACATCCAGATGACCCAGTCTCCATCTTCCGTGTCTGCATCTGTAGGCGACAGAGTCACCATCACTTGTCGGGCGAGTCAGGGTATTAGGAAGTGGTTAGCCTGGTATCAGCAGAAACCAGGGAAAGCCCCTAAGCTCCTGATCTATGCTGCATCCAGTTTGCAAAGTGGGGTCCCGTCAAGGTTCAGCGGCAGTGGATCTGGGACAGATTTCACTCTCACCATCAGCAGCCTGCAGCCTGAAGATTTTGCAACTTACTATTGTCAACAGGCTAACAGTTTCCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCCAGGCTTCTGGATACACCTTCACCGGCTACCATATGCACTGGGTGCGACAGGCCCCTGGACAAGGGCTTGAGTGGATGGGATGGATCAACCCTAACAATAATGCCACAAACTATGCACAGAAGTTTCAGGGCAGGGTCACCATGACCAGGGACACGTCCATCAGCACAGCCTACATGGAACTGAGGAGGCTGAGATCTGACGACACGGCCGTGTATCACTGTGCGAGAGATGGCACCTCGTCTTTTGACTACTGGGGCCAGGGAACCCTGGTCACCGTCTCCTCA |
| | | | SEQ ID NO: 25459 | SEQ ID NO: 29465 |
| | | AA | DIQMTQSPSSVSASVGDRVTITCRASQGIRKWLAWYQQKPGKAPKLLIYAASSLQSGVPSRFSGSGSGTDFTLTISSLQPEDFATYYCQQANSFPFTFGPGTKVDIK | QVQLVQSGAEVKKPGASVKVSCQASGYTFTGYHMHWVRQAPGQGLEWMGWINPNNNATNYAQKFQGRVTMTRDTSISTAYMELRRLRSDDTAVYHCARDGTSSFDYWGQGTLVTVSS |
| | | | SEQ ID NO: 25460 | SEQ ID NO: 29466 |

FIGURE 50

| | | | | |
|---|---|---|---|---|
| **iPS:434191** | **21-225_56B6** | NA | GACATCCAGATGACCCAGTCTCCATCCTCCCTGTCTGTATCTGTAGGAGACAGAGTCACCATCACTTGCCGGGCAAGTCAGAGCATTTTCAGATATTTAAATTGGTATCAGCAGAAACCAGGAAGAGCCCCTAAGCTCCTGATCTTTGCTGCATCCAGTTTCCAAAGTGGGGTCCCATCAAGGTTCAGTGGCAGTGGATCTGGGACAGACTTCACTCTCACCATCAGCAGCCTACAACCTGAAGATTTTGCAACTTACTACTGTCAACAGACTTACAGTCCCCCTCTCACTTTCGGCGGAGGGACCAAGGTGGAGATCAAA | CAGGTGCAACTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGCATGATGGAAGTAATAAATATTATGTAGACTCCGTGAAGGGCCGATTCACCATCTCCAGAGACAATTCCAAGAATACGCTGTATCTGCAAATGAACAGCCTGAGAGCCGAGGACACGGCTGTGTATTACTGTGCGAGAGACGAGCAGCTCGGGACCTTTGACTACTGGGGCCAGGGAACCCTGGTCACCGTCTCCTCA |
| | | | SEQ ID NO: 25461 | SEQ ID NO: 29467 |
| | | AA | DIQMTQSPSSLSVSVGDRVTITCRASQSIFRYLNWYQQKPGRAPKLLIFAASSFQSGVPSRFSGSGSGTDFTLTISSLQPEDFATYYCQQTYSPPLTFGGGTKVEIK | QVQLVESGGGVVQPGRSLRLSCAASGFTFSSYGMHWVRQAPGKGLEWVAVIWHDGSNKYYVDSVKGRFTISRDNSKNTLYLQMNSLRAEDTAVYYCARDEQLGTFDYWGQGTLVTVSS |
| | | | SEQ ID NO: 25462 | SEQ ID NO: 29468 |
| **iPS:434193** | **21-225_56C6** | NA | GACATCCAGATGACCCAGTCTCCATCTTCCGTGTCTGCATCTGTGGGAGACAGAGTCACCATCACTTGTCGGGCGAGTCAGGGTATTAGCAGCTGGTTAGCCTGGTATCAGCAGAAATCAGGGAATGCCCCTAAGCTCCTGATCTATGCTGCATCCAGATTGCAAAGTGGGGTCCCATCAAGGTTCAGCGGCAGTGGATCTGGTACATATTTCACTCTCATTATCAGCAGCCTGCAGTCTGAAGATTTTGCAACTTACTATTGTCAACAGGCTAACAGTTTCCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGACTACCATATGCACTGGGTGCGACAGGCCCCTGGACAAGGGCTTGAGTGGATGGGATGGATCAACCCTAACAGAGGTGGCACAAATTATGTACAGAAGTTTCAGGGTAGGGTCGCCATGACCAATGACACGTCCATCAGCACAGCCTATATGGAGCTGAGTGGGCTGAGATCTGACGACACGGCCGTGTATTACTGTGCGAGAGATGGCACCTCGTCTTTTGACTATTGGGGCCGGGGAACCCTGGTCACCGTCTCCTCA |
| | | | SEQ ID NO: 25463 | SEQ ID NO: 29469 |

FIGURE 50

| | | | | |
|---|---|---|---|---|
| | | AA | DIQMTQSPSSVSASVGDRVTITCRASQGISSWLAWYQQKSGNAPKLLIYAASRLQSGVPSRFSGSGSGTYFTLIISSLQSEDFATYYCQQANSFPFTFGPGTKVDIK | QVQLVQSGAEVKKPGASVKVSCKASGYTFTDYHMHWVRQAPGQGLEWMGWINPNRGGTNYVQKFQGRVAMTNDTSISTAYMELSGLRSDDTAVYYCARDGTSSFDYWGRGTLVTVSS |
| | | | SEQ ID NO: 25464 | SEQ ID NO: 29470 |
| iPS:434195 | 21-225_56F6 | NA | GACATCCAGATGACCCAGTCTCCATCTTCTGTGTGTGCATATGTAGGAGACAGAGTCACCATCACTTGTCGGGTGAGTCAGGATATTAGCAAATGGTTAGCCTGGTTTCAGCAGAAACCAGGGAAAGCCCCTAAATTCTTGATATATGTTGCATCCGGTTTGCAAAGTGGGGTCCCATCAAGGTTCAGCGGCAGTGGATCTGGGACAGATTTCACTTTCACCATCAGCAGCCTGCAGCCTGAAGATTTTGCAACTTACTATTGTCAACAGGCTAACAGTTTCCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATTCACCTTCACCGACTACTATATGCACTGGGTGCGACAGGCCCCTGGACAAGGGCTTGAGTGGATGGGATGGATCAACCCTAACAGTGGTGGCACAAACTATGCACAGAGGTTTCAGGGCAGGGTCACCATGACCAGGGACACGTCCATCAGCACAGCCTACATGGAACTGAGCAGACTGAGATCTGACGACACGGCCGTGTATTACTGTACGAGAGAGGGAGCAACTCGTCCGACGGGTTTTGACTACTGGGGCCAGGGAACCCTGGTCACCGTCTCCTCA |
| | | | SEQ ID NO: 25465 | SEQ ID NO: 29471 |
| | | AA | DIQMTQSPSSVCAYVGDRVTITCRVSQDISKWLAWFQQKPGKAPKFLIYVASGLQSGVPSRFSGSGSGTDFTFTISSLQPEDFATYYCQQANSFPFTFGPGTKVDIK | QVQLVQSGAEVKKPGASVKVSCKASGFTFTDYYMHWVRQAPGQGLEWMGWINPNSGGTNYAQRFQGRVTMTRDTSISTAYMELSRLRSDDTAVYYCTREGATRPTGFDYWGQGTLVTVSS |
| | | | SEQ ID NO: 25466 | SEQ ID NO: 29472 |

FIGURE 50

| | | | | |
|---|---|---|---|---|
| iPS:434197 | 21-225_56C7 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATACTGCATCCAATTTACAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTATCCGTGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCCACTATATAAACTGGGTGCGACAGGCCCCTGGACAAGGGCTTGAGTGGATGGCATGGGTCAACCCTAACAGTGGTGGCACAAACTCTGCACAGAAGTTTCAGGGCAGGGTCACCATGACCAGGGACACGTCCATCAGCACAGTCTACATGGAGCTGAGCAGGCTGAGATCTGACGACACGGCCGTGTATTACTGTGCGAGGGGAGGGCAGCTCGGCGGGTTTAACTACTACTACTACGGTATGGACGTCTGGGGCCAAGGGACCACGGTCACCGTCTCCTCA |
| | | | SEQ ID NO: 25467 | SEQ ID NO: 29473 |
| | | AA | DIQMTQSPSSLSASVGDRVTITCRASQGIRNDLGWYQQKPGKAPKRLIYTASNLQSGVPSRFSGSGSGTEFTLTISSLQPEDFATYYCLQHNSYPWTFGQGTKVEIK | QVQLVQSGAEVKKPGASVKVSCKASGYTFTGHYINWVRQAPGQGLEWMAWVNPNSGGTNSAQKFQGRVTMTRDTSISTVYMELSRLRSDDTAVYYCARGGQLGGFNYYYYGMDVWGQGTTVTVSS |
| | | | SEQ ID NO: 25468 | SEQ ID NO: 29474 |
| iPS:434199 | 21-225_59F11 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATCGTTACCCTTTCACTTTCGGCCCTGGGACCAAAGTGGATTTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGATGAAAGTAATAAACACTATGCAGACTCCGTGAAGGGCCGATTCACCATCTCCAGAGACAATTCCAAGACCACGCTGTATCTGCAAATGAGCAGCCTGAGAGCCGAGGACACGGCTGTGTATTATTGTTCGAGAGAACTGGGGATGAACGGAGACTACTGGGGCCAGGGAACCCTGGTCACCGTCTCCTCA |
| | | | SEQ ID NO: 25469 | SEQ ID NO: 29475 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNRYPFTFGPGT KVDFK | QVQLVESGGGVVQPGRSLRLSCAASGFTFSNYGM HWVRQAPGKGLEWVAVIWYDESNKHYADSVKGR FTISRDNSKTTLYLQMSSLRAEDTAVYYCSRELGM NGDYWGQGTLVTVSS |
| | | | SEQ ID NO: 25470 | SEQ ID NO: 29476 |
| iPS:434201 | 21-225_59A12 | NA | GATATTGTGATGACCCAGACTCCACTCTCTCT GTCCGTCACCCCTGGACAGCCGGCCTCCATCT CCTGCAAGTCTAGTCAGAGCCTCCAGCATGGT GAAGGAAAGACCTATTTGTATTGGTACGTGCA GAAGCCAGGCCAGCCTCCACAGCTCCTGATCT ATGAAGTTTCCTATCGGTTTTCTGGAGTGCCA GATAGGTTCAGTGGTAGTGGGTCAGGGACAG ATTTCACACTGAAAATCAGCCGGGTGGAGGTT GAGGATGTTGGGGTTTATTACTGCATGCAAAG TACACAGCTTCCGCTCACCTTCGGCGGAGGGA CCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGGCGGTATAG CAGCAGCTGGGACGGGGGTATGGACGTCTGGGG CCAAGGGACCACGGTCACCGTCTCCTCA |
| | | | SEQ ID NO: 25471 | SEQ ID NO: 29477 |
| | | AA | DIVMTQTPLSLSVTPGQPASISCKSSQSLQHGEG KTYLYWYVQKPGQPPQLLIYEVSYRFSGVPDRF SGSGSGTDFTLKISRVEVEDVGVYYCMQSTQLP LTFGGGTKVEIK | QVQLVESGGGVVQPGRSLRLSCAASGFTFSSYGMH WVRQAPGKGLEWVAVIWYDGSNKYYADSVKGRF TISRDNSKNTLYLQMNSLRAEDTAVYYCARRYSSS WDGGMDVWGQGTTVTVSS |
| | | | SEQ ID NO: 25472 | SEQ ID NO: 29478 |

FIGURE 50

| | | | | |
|---|---|---|---|---|
| **iPS:434203** | **21-225_60E2** | NA | GACATCCAGATGACCCAGTCTCCATCCTCCCTGTCTGCATCTGTAGGAGACAGAGTCACCATCACTTGCCGGGCAAGTCAGGGCATTAGAAAAGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAATGACTATGGCATGCACTGGGTCCGCCAGGCTCCAGGCAAGGGGCTGGAGTGGGTGGCAATTATATGGTATGATGAAAATAATAAATACTATGCAGACTCCGTGAAGGGCCGATTCACCATCTCCAGAGACAATTCCAAGAACACGCTGTATCTGCAAATGAGCAGCCTGAGAGCCGAGGACACGGCTGTATATTACTGTGCGAGAGATGTTCTGGACCCTTTTGACTACTGGGGCCAGGGAACCCTGGTCACCGTCTCCTCA |
| | | | SEQ ID NO: 25473 | SEQ ID NO: 29479 |
| | | AA | DIQMTQSPSSLSASVGDRVTITCRASQGIRKDLGWYQQKPGKAPKRLIYAASSLQSGVPSRFSGSGSGTEFTLTISSLQPEDFATYYCLQHNSYPWTFGQGTKVEIK | QVQLVESGGGVVQPGRSLRLSCAASGFTFNDYGMHWVRQAPGKGLEWVAIIWYDENNKYYADSVKGRFTISRDNSKNTLYLQMSSLRAEDTAVYYCARDVLDPFDYWGQGTLVTVSS |
| | | | SEQ ID NO: 25474 | SEQ ID NO: 29480 |
| **iPS:434205** | **21-225_60G2** | NA | GATATTGTGATGACCCAGACTCCACTCTCTCTGTCCGTCACACCTGGACAGTCGGCCTCCATCTCCTGCAAGTCTAGTCAGAGCCTCCTGCATAGTGAAGGAAAGACCTATTTGTATTGGTACCTGCAGAAGCCAGGCCAGCCTCCACAGCTCCTGATCTATGAAGTTTCCAACCGGATCTCTGGAGTGCCAGATAGGTTCAGTGGCAGCGGGTCAGGGACAGATTTCACACTGAAAATCAGCCGGGTGGAGGCTGAGGATGTTGGGGTTTATTACTGCATGCAAAGTATACAGCTTCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGTTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAAGGTATAGCAGAAGCTGGACGGGAGGCATGGACGTCTGGGGCCAAGGGACCACGGTCACCGTCTCCTCA |
| | | | SEQ ID NO: 25475 | SEQ ID NO: 29481 |

FIGURE 50

| | | | | |
|---|---|---|---|---|
| | | AA | DIVMTQTPLSLSVTPGQSASISCKSSQSLLHSEGKTYLYWYLQKPGQPPQLLIYEVSNRISGVPDRFSGSGSGTDFTLKISRVEAEDVGVYYCMQSIQLPLTFGGGTKVEIK | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARRYSRSWTGGMDVWGQGTTVTVSS |
| | | | SEQ ID NO: 25476 | SEQ ID NO: 29482 |
| iPS:434207 | 21-225_60A3 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATTTAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCTTTCACTTTCGGCCCTGGGACCAAAGTGGATATCAAA | CAGGTGCAACTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGAAGAAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTATATTACTGTGCGAGAGAACTGGGGATGACCGGAGACTACTGGGGCCAGGGAACCCTGGTCACCGTCTCCTCA |
| | | | SEQ ID NO: 25477 | SEQ ID NO: 29483 |
| | | AA | DIQMTQSPSSLSASVGDRVTITCRASQGIRNDLGWYQQKPGKAPKRLIYAAFSLQSGVPSRFSGSGSGTEFTLTISSLQPEDFATYYCLQHNSYPFTFGPGTKVDIK | QVQLVESGGGVVQPGRSLRLSCAASGFTFSSYGMHWVRQAPGKGLEWVAVIWYEESNKYYADSVKGRFTISRDNSKNTLYLQMNSLRAEDTAVYYCARELGMTGDYWGQGTLVTVSS |
| | | | SEQ ID NO: 25478 | SEQ ID NO: 29484 |

FIGURE 50

| | | | | |
|---|---|---|---|---|
| iPS:434209 | 21-225_60C3 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATTCTGCATCCAGTTTACAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGTGGACGTTCGGCCTAGGGACCAAGGTGGAAATCAAA | CAGGTGCAACTAGTGCAGTCTGGGGCTGAGGTGAAGAAGCCTGGGGCCTCAGTGAAGGTCTCCTGCAAGGCTTCTGGATACAGTTTCACCGGCCACTATATACACTGGGTGCGACAGGCCCCTGGACAAGGGCTTGAGTACATGGGATGGATCAACCCTAACAGCGGTGGCACAAACTATGTACAGAAATTTCAGGGCAGGGTCACCATGACCAGGGACACGTCCATCAGCACAGCCTACATGGAGCTGAGCAGGCTGAGATCTGACGACACGGCCATATATTACTGTTCGAGAGGGGGCCTACTGGGAGCTACCAACTACTATTATTACGGTATGGACGTCTGGGGCCAAGGGACCACGGTCACCGTCTCCTCA |
| | | | SEQ ID NO: 25479 | SEQ ID NO: 29485 |
| | | AA | DIQMTQSPSSLSASVGDRVTITCRASQGIRNDLGWYQQKPGKAPKRLIYSASSLQSGVPSRFSGSGSGTEFTLTISSLQPEDFATYYCLQHNSYPWTFGLGTKVEIK | QVQLVQSGAEVKKPGASVKVSCKASGYSFTGHYIHWVRQAPGQGLEYMGWINPNSGGTNYVQKFQGRVTMTRDTSISTAYMELSRLRSDDTAIYYCSRGGLLGATNYYYYGMDVWGQGTTVTVSS |
| | | | SEQ ID NO: 25480 | SEQ ID NO: 29486 |
| iPS:434211 | 21-225_60F3 | NA | GACATCGTGATGACCCAGTCTCCAGACTCCCTGACTGTGTCTCTGGGCGAGAGGGCCACCATCAACTGCAAGTCCAGCCAGAGTGTTTTATACAGCTCCAACAATAAGAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAATTTATTACTGTCAGCAATATTATAGTACTCCGTGCAGTTTTGGCCAGGGGACCAAGCTGGAGATCAAC | CAGGTGCTGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGTATAGCAGTGGCTGGTACTTCTTTGACTACTGGGGCCAGGGAACCCTGGTCACCGTCTCCTCA |
| | | | SEQ ID NO: 25481 | SEQ ID NO: 29487 |

FIGURE 50

| | | | | |
|---|---|---|---|---|
| | | AA | DIVMTQSPDSLTVSLGERATINCKSSQSVLYSSNNKNYLAWYQQKPGQPPKLLIYWASTRESGVPDRFSGSGSGTDFTLTISSLQAEDVAIYYCQQYYSTPCSFGQGTKLEIN | QVLLVQSGAEVKKPGASVKVSCKASGYTFTNYDINWVRQATGQGLEWMGWMHPNSGNTGYAQKFQGRVTMTRNTSISTAYMELSSLRSEDTAVYYCAYSSGWYFFDYWGQGTLVTVSS |
| | | | SEQ ID NO: 25482 | SEQ ID NO: 29488 |
| iPS:434213 | 21-225_60A4 | NA | GACATCCAGATGACCCAGTCTCCATCCTCACTGTCTGCATCTGTAGGAGACAGAGTCACCATCACATGTCGGGCGAGTCAGGGCATTAGCAATTACTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGTCTGAAGATTTTGCAACGTACTACTGCCAACAATATAAAAGTCACCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGTCATGAGCTGGGTCCGCCAGGCTCCAGGGAAGGGGCTGGAGTGGGTCTCATCTATTAGTGGTAGTGGTGGTTGGACAAACTACGCAGACTCCGTGAAGGGCCGATTCACCACCTCCAGAGACAATTCCAAGAACACGCTGTATCTGCAAATGAACAGCCTGAGAGCCGAGGACACGGCCGTTTATTACTGTGCGAGACTAACTGGATTTGACTATTGGGGCCAGGGAACCCTGGTCACCGTCTCCTCA |
| | | | SEQ ID NO: 25483 | SEQ ID NO: 29489 |
| | | AA | DIQMTQSPSSLSASVGDRVTITCRASQGISNYLAWFQQKPGKAPKSLIYAASSLQSGVPSKFSGSGSGTDFTLTISSLQSEDFATYYCQQYKSHPFTFGPGTKVDIK | EVQLLESGGGLVQPGGSLRLSCAASGFTFSSYVMSWVRQAPGKGLEWVSSISGSGGWTNYADSVKGRFTTSRDNSKNTLYLQMNSLRAEDTAVYYCARLTGFDYWGQGTLVTVSS |
| | | | SEQ ID NO: 25484 | SEQ ID NO: 29490 |

FIGURE 50

| | | | | |
|---|---|---|---|---|
| **iPS:434215** | **21-225_60F7** | NA | GACATCCAGATGACCCAGTCTCCATCCTCACTGTCTGCATCTGTAGGAGACAGAGTCACCATCTCTTGTCGGGCGAGTCAGGTCATTAAGAATTATTTAGTCTGGGTTCAGCAGAAACCAGGGAAAGCCCCTAAGTCCCTGATCTATGCTGCGTCCAGTTTGCAAAGTGGGGTCCCATCAACGTTCAGCGGCAGTGGATCTGGGACAGATTTCACTCTCACCATCAGCAGCCTGCAGCCTGAAGATTTTGCAACTTATTACTGCCTACAGTTTCATAGTTACCCATTCACTTTCGGCCCTGGGACCAAAATGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGTCATGAGCTGGGTCCGCCAGGCTCCAGGGAAGGGGCTGGAGTGGGTCTCAGGTATTAGTGGTAGTGGTAATAGAACATACTACGCAGACTCCGTGAAGGGCCGGTTCACCATCTCCAGAGACAATTCCAAGAATACGCTGTATCTGCAAATGAACAGCCTGAGAGCCGAGGACTCGGCCGTTTATTACTGTGGGAGTTTGGGGATTGACTGGGGCCAGGGAACCCTGGTCACCGTCTCCTCA |
| | | | SEQ ID NO: 25485 | SEQ ID NO: 29491 |
| | | AA | DIQMTQSPSSLSASVGDRVTISCRASQVIKNYLVWVQQKPGKAPKSLIYAASSLQSGVPSTFSGSGSGTDFTLTISSLQPEDFATYYCLQFHSYPFTFGPGTKMDIK | EVQLLESGGGLVQPGGSLRLSCAASGFTFSSYVMSWVRQAPGKGLEWVSGISGSGNRTYYADSVKGRFTISRDNSKNTLYLQMNSLRAEDSAVYYCGSLGIDWGQGTLVTVSS |
| | | | SEQ ID NO: 25486 | SEQ ID NO: 29492 |
| **iPS:434217** | **21-225_60E8** | NA | GACATCCAGATGACCCAGTCTCCATCCTCCCTGTCTGCATCTGTAGGAGACAGAGTCACCTTCACTTGCCGGGCAAGTCAGGGCATTAGAAATGATTTAGGCTGGTTTCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAGTAGTTACCCGCTCACTTTCGGCGGAGGGACCAAGGTGGAGATCAAA | CAGGTGCAGCTGCAGGAGTCGGGCCCAGGACTGGTGAGGCCTTCGGCGACCCTGTCCCTCACCTGCACTGTCTCTGGTGGCTCCATCAGCAGGAGTAGTTACTACTGGGGCTGGATCCGCCAGCCCCCAGGGAAGGGACTGGAGTGGATTGGGAGTATCTATTATAGTGGGAGCGCCTCCTACAACCCGTCCCTCAAGAGTCGAGTCACCATATCCGTAGACACGTCCGAGAACCAATTCTCCCTGAGGCTGAGCTCTGTGACCGCCGCAGACACGGCTGTGTATTACTGTGCGAGACTGGACAGTGGCTGGTCGTTTGACTACTGGGGCCAGGGAACCCTGGTCACCGTCTCCTCA |
| | | | SEQ ID NO: 25487 | SEQ ID NO: 29493 |

FIGURE 50

| | | | | |
|---|---|---|---|---|
| | | AA | DIQMTQSPSSLSASVGDRVTFTCRASQGIRNDLGWFQQKPGKAPKRLIYAASSLQSGVPSRFSGSGSGTEFTLTISSLQPEDFATYYCLQHSSYPLTFGGGTKVEIK | QVQLQESGPGLVRPSATLSLTCTVSGGSISRSSYYWGWIRQPPGKGLEWIGSIYYSGSASYNPSLKSRVTISVDTSENQFSLRLSSVTAADTAVYYCARLDSGWSFDYWGQGTLVTVSS |
| | | | SEQ ID NO: 25488 | SEQ ID NO: 29494 |
| iPS:434219 | 21-225_60E9 | NA | GAAATAGTGATGACGCAGTCTCCAGCCACCCTGTCTGTGTCTCCAGGGGAAAGAGCCACCCTCTCCTGTAGGGCCAGTCAGAGTGTTAGCAGTTCCTTAGCCTGGTACCGGCAGAAACCTGGCCAGGCTCCCAGGCTCCTCATCTATGGTGCATCCACCAGGGCCACTGGTATCCCGGCCAGGTTCAGTGGCAGTGGGTCTGGGACAGAGTTCACTCTCACCATCAGCAGCCTGCAGTCTGAAGATTTTGCAGTTTATTGCTGTCAGCAGTATAATAACTGGCCATTCACTTTCGGCCCTGGGACCAAAATAGATATCAAA | GAGGTGCAGTTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTAGTGGTGGTAACACATTCTACGCAGACTCCGTGAAGGGCCGGTTCACCATCTCCAGAGACAATTCCAAGAACACGCTGTTTCTTCAAATGAACAGCCTGAGAGCCGAGGACACGGCCGTATATTACTGTGCGAAATTTTTCGGTATAGTGGGAGCCGGGTACTTTGACTACTGGGGCCAGGGAACCCTGGTCACCGTCTCCTCA |
| | | | SEQ ID NO: 25489 | SEQ ID NO: 29495 |
| | | AA | EIVMTQSPATLSVSPGERATLSCRASQSVSSSLAWYRQKPGQAPRLLIYGASTRATGIPARFSGSGSGTEFTLTISSLQSEDFAVYCCQQYNNWPFTFGPGTKIDIK | EVQLLESGGGLVQPGGSLRLSCAASGFTFSSYAMSWVRQAPGKGLEWVSAISGSGGNTFYADSVKGRFTISRDNSKNTLFLQMNSLRAEDTAVYYCAKFFGIVGAGYFDYWGQGTLVTVSS |
| | | | SEQ ID NO: 25490 | SEQ ID NO: 29496 |

FIGURE 50

| | | | | |
|---|---|---|---|---|
| **iPS:434221** | **21-225_60A11** | NA | GACATCCAGATGACCCAGTCTCCATCTTCCGTGTCTGCATCTGTAGGAGACAGAGTCACCATCACTTGTCGGGCGAGTCAGGTTATTAGCAACTGGTTAGCCTGGTATCAGCTGAAACCAGGGAAAGCCCCTAAGCTCCTGATCTATACTGCATCCAGTTTGCAAAGTGGGGTCCCATCAAGGTTCAGCGGCAATGAATCTGGGACAGATTTCACTCTCACCATCAGCAGCCTGCAGCCTGAAGATTTTGCAACTTACTATTGTCAACAGGCTAACAGTTTCCCGTGGACGTTCGGCCAAGGGACCAAGGTGGAAATCAAA | GAGGTGCAGCTGTTGGAGTCTGGGGGAGGCTTGGTACAGCCGGGGGGGTCCCTGAGACTCTCCTGTGCAGCCTCTGGATTCACCTTTAGCAACTATGCCATGACCTGGGTCCGCCAGGCTCCAGGGAAGGGGCTGGAGTGGGTCTCAGCTATTAGTGGTAGTGGTGGTAACACATTCTACGCAGACTCCGTGACGGGCCGGGTCACCATCTCCAGAGACAATTCCAAGAACACGCTGTATCTGCAAATGAACAGTCTGAGAGCCGAGGACACGGCCGTATATTACTGTGCGAAACTGGGGAAGGACTACCACTACTACGGTATGGACGTCTGGGGCCAAGGGACCACGGTCACCGTCTCCTCA |
| | | | SEQ ID NO: 25491 | SEQ ID NO: 29497 |
| | | AA | DIQMTQSPSSVSASVGDRVTITCRASQVISNWLAWYQLKPGKAPKLLIYTASSLQSGVPSRFSGNESGTDFTLTISSLQPEDFATYYCQQANSFPWTFGQGTKVEIK | EVQLLESGGGLVQPGGSLRLSCAASGFTFSNYAMTWVRQAPGKGLEWVSAISGSGGNTFYADSVTGRVTISRDNSKNTLYLQMNSLRAEDTAVYYCAKLGKDYHYYGMDVWGQGTTVTVSS |
| | | | SEQ ID NO: 25492 | SEQ ID NO: 29498 |
| **iPS:434223** | **21-225_60C12** | NA | GACATTGTGATGACCCAGACTCCACTCTCTCTGTCCGTCACCCCTGGACAGCCGGCCTCCATCTCCTGCAAGTCTAGTCAGAGCCTCCTGCATAGTGAGGGAAAGACCTATTTGTATTGGTACCTGCAGAAGCCAGGCCAGCCTCCACAGTTCCTGATCTATGAAGTTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGATTTATTACTGCATGCAAAGTATAAAGTATCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAAGGTATAGCAGAAGCTGGACGGGAGGTATGGACGTCTGGGGCCAGGGGACCACGGTCACCGTCTCCTCA |
| | | | SEQ ID NO: 25493 | SEQ ID NO: 29499 |

FIGURE 50

| | | | | |
|---|---|---|---|---|
| | | AA | DIVMTQTPLSLSVTPGQPASISCKSSQSLLHSEGKTYLYWYLQKPGQPPQFLIYEVSNRFSGVPDRFSGSGSGTDFTLKISRVEAEDVGIYYCMQSIKYPLTFGGGTKVEIK | QVQLVESGGGVVQPGRSLRLSCAASGFTFSSYGMHWVRQAPGKGLEWVAVIWYDGSNKYYVDSVKGRFTISRDNSKNTLYLQMNSLRAEDTAVYYCARRYSRSWTGGMDVWGQGTTVTVSS |
| | | | SEQ ID NO: 25494 | SEQ ID NO: 29500 |
| **iPS:434225** | **21-225_60E12** | NA | GACATCCAGATGACCCAGTCTCCATCCTCCCTGTCTGCATCTGTAGGAGACAGAGTCACCATCACTTGCCGGGCAAGTCAGAGCATTAGCAGCTATTTAAATTGGTATCAGCAGAAACCAGGGAAAGCCCCTAAGCTCCTGATCTATGCTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCACCTTACTACTGTCAACAGAGTTACAATATTTCATTCACTTTCGGCCCTGGGACCAAAGTGGATATCAAA | CAGGTGCAGCTGCAGGAGTCGGGCCCAGGACTGGTGAAGCCTTCGGAGACCCTGTCCCTCACCTGCACTGTCTCTGGTGGCTCCATCAGTAGTTACTTCTGGAGCTGGATCCGGCAGCCCGCCGGGAAGGGACTGGAGTGGATTGGGCGCATCTATACCAGGGGGAGCACCAACTACAACCCCTCCCTCAAGAGTCGAGTCACCATGTCAGTAGACACGTCCAAGAACCAGTTCTCCCTGAAGCTGAGCTCTGTGACCGCCGCGGACACGGCCGTGTATTACTGTGCGAGAGAGGGAAAAACTGGGGGGGTTTCTTACTTTGACTACTGGGGCCAGGGAACCCTGGTCACCGTCTCCTCA |
| | | | SEQ ID NO: 25495 | SEQ ID NO: 29501 |
| | | AA | DIQMTQSPSSLSASVGDRVTITCRASQSISSYLNWYQQKPGKAPKLLIYAASSLQSGVPSRFSGSGSGTDFTLTISSLQPEDFAPYYCQQSYNISFTFGPGTKVDIK | QVQLQESGPGLVKPSETLSLTCTVSGGSISSYFWSWIRQPAGKGLEWIGRIYTRGSTNYNPSLKSRVTMSVDTSKNQFSLKLSSVTAADTAVYYCAREGKTGGVSYFDYWGQGTLVTVSS |
| | | | SEQ ID NO: 25496 | SEQ ID NO: 29502 |

FIGURE 50

| | | | | |
|---|---|---|---|---|
| **iPS:434227** | **21-225_61A1** | NA | GACATCCAGATGACCCAGTCTCCATCCTCCCTGTCTGCATCTGTAGGAGACAGAGTCACCATCACTTGCCGGGCAAGTCAGAGCATTAGCAGCTATTTAAATTGGTATCAGCAGAAACCAGGGAAAGCCCCTAAGCTCCTGATCTATGCTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCACCTTACTACTGTCAACAGAGTTACAATATTTCATTCACTTTCGGCCCTGGGACCAAAGTGGATATCAAA | CAGGTGCAGCTGCAGGAGTCGGGCCCAGGACTGGTGAAGCCTTCGGAGACCCTGTCCCTCACCTGCACTGTCTCTGGTGGCTCCATCAGTAGTCACTTCTGGAGCTGGATCCGGCAGCCCGCCGGGAAGGGACTGGAGTGGATTGGGCGCATCTATATCAGGGGGAGCACCAACTACAACCCCTCCCTCAAGAGTCGAGTCACCATGTCAGTAGACACGTCCAAGAACCAGTTCTCCCTGAAGCTGAGCTCTGTGACCGCCGCGGACACGGCCGTGTATTACTGTGCGAGAGAGGGAAAAACTGGGGGGGTTTCTTACTTTGACTACTGGGGCCAGGGAACCCTGGTCACCGTCTCCTCA |
| | | | SEQ ID NO: 25497 | SEQ ID NO: 29503 |
| | | AA | DIQMTQSPSSLSASVGDRVTITCRASQSISSYLNWYQQKPGKAPKLLIYAASSLQSGVPSRFSGSGSGTDFTLTISSLQPEDFAPYYCQQSYNISFTFGPGTKVDIK | QVQLQESGPGLVKPSETLSLTCTVSGGSISSHFWSWIRQPAGKGLEWIGRIYIRGSTNYNPSLKSRVTMSVDTSKNQFSLKLSSVTAADTAVYYCAREGKTGGVSYFDYWGQGTLVTVSS |
| | | | SEQ ID NO: 25498 | SEQ ID NO: 29504 |
| **iPS:434229** | **21-225_61H1** | NA | GACATCCAGATGACCCAGTCTCCATCCTCCCTGTCTGCATCTGTAGGAGACAGAGTCACCATCACTTGCCGGGCAAGTCAGGGCATTAGAAAAGATTTAGGCTGGTATCAGCAGAAACCAGGGAAAGCCCCTAAGCGCCTGATCTTTGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAATGACTATGGCATGCACTGGGTCCGCCAGGCTCCAGGCAAGGGGCTGGAGTGGGTGGCAATTATATGGTATGATGAAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTATATTACTGTGCGAGAGATGTTCTGGACCCTTTTGACTACTGGGGCCAGGGAACCCTGGTCACCGTCTCCTCA |
| | | | SEQ ID NO: 25499 | SEQ ID NO: 29505 |

FIGURE 50

| | | | | |
|---|---|---|---|---|
| | | AA | DIQMTQSPSSLSASVGDRVTITCRASQGIRKDLGWYQQKPGKAPKRLIFAASSLQSGVPSRFSGSGSGTEFTLTISSLQPEDFATYYCLQHNSYPWTFGQGTKVEIK | QVQLVESGGGVVQPGRSLRLSCAASGFTFNDYGMHWVRQAPGKGLEWVAIIWYDESNKYYADSVKGRFTISRDNSKNTLYLQMNSLRAEDTAVYYCARDVLDPFDYWGQGTLVTVSS |
| | | | SEQ ID NO: 25500 | SEQ ID NO: 29506 |
| iPS:434231 | 21-225_61F2 | NA | GACATCCAGATGACCCAGTCTCCATCCTCCCTGTCTGCATCTGTAGGAGACAGAGTCACCGTCACTTGCCGGGCAAGTCAGGGCATTAGAGATGATTTAGGCTGGTATCAGCAGAAACCAGGGAAAGCCCCTGAGCGCCTGATCTATGCTGCATCCAGTTTGCAAAGTGGGGTCCCATCAAGGTTCAGCGGCAGTGGATCTGGGACAGAGTTCACTCTCACAATCAGCAGCCTGCAGCCTGAAGATTTTGCAACTTATTACTGTCTACAGCATTATAGTTACCCTCGCAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGAGGTATAGCAGTGGCTGGTACGACTACGGTATGGACGTCTGGGGCCAAGGGACCACGGTCACCGTCTCCTCA |
| | | | SEQ ID NO: 25501 | SEQ ID NO: 29507 |
| | | AA | DIQMTQSPSSLSASVGDRVTVTCRASQGIRDDLGWYQQKPGKAPERLIYAASSLQSGVPSRFSGSGSGTEFTLTISSLQPEDFATYYCLQHYSYPRSFGQGTKLEIK | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARERYSSGWYDYGMDVWGQGTTVTVSS |
| | | | SEQ ID NO: 25502 | SEQ ID NO: 29508 |

FIGURE 50

| | | | | |
|---|---|---|---|---|
| **iPS:434233** | **21-225_61B3** | NA | GATATTGTGATGACCCAGACTCCACTCTCTCTGTCCGTCACACCTGGACAGCCGGCCTCCATCTCCTGCAAGTCTAGTCAGAGCCTCCTGCATAGTGAAGGAAAGACCTATTTGTATTGGTACCTGCAGAAGCCAGGCCAGCCTCCACAGCTCCTGATCTATGAAGTTTCCAACCGGATCTCTGGAGTGCCAGATAGGTTCAGTGGCAGCGGGTCAGGGACAGATTTCACACTGAAAATCAGCCGGGTGGAGGCTGAGGATGTTGGGGTTTATTACTGCATGCAAAGTATACAGCTTCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAAGGTATAGCAGAAGCTGGGCGGGAGGCATGGACGTCTGGGGCCAAGGGACCACGGTCACCGTCTCCTCA |
| | | | SEQ ID NO: 25503 | SEQ ID NO: 29509 |
| | | AA | DIVMTQTPLSLSVTPGQPASISCKSSQSLLHSEGKTYLYWYLQKPGQPPQLLIYEVSNRISGVPDRFSGSGSGTDFTLKISRVEAEDVGVYYCMQSIQLPLTFGGGTKVEIK | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARRYSRSWAGGMDVWGQGTTVTVSS |
| | | | SEQ ID NO: 25504 | SEQ ID NO: 29510 |
| **iPS:434235** | **21-225_61E3** | NA | GACATCGTGATGACCCAGTCTCCAGACTCCCTGGCTGTGTCTCTGGGCGAGAGGGCCACCATCAACTGCAAGTCCAGCCAGAGTGTTTTACACATCTCCAACAATAACAATTACTTAGCTTGGTACCAGCAGCAACCAGGACAGCCTCCTAAACTGCTCATTTACTGGGCATCTATCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTATTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGACCCCTAACAGTGGTAACACAGGCTATGCACAGAAGTTCCAGGGCAGAGTCACCATGACCAGGAACACCTCCATAAGCACAGCCTACATGGAGCTGAGCAGCCTGAAATCTGAGGACACGGCCGTGTATTACTGTGCGTATAGCAGTGGCTGGTACCGCTTTGACTACTGGGGCCAGGGAACCCTGGTCACCGTCTCCTCA |
| | | | SEQ ID NO: 25505 | SEQ ID NO: 29511 |

FIGURE 50

| | | | | |
|---|---|---|---|---|
| | | AA | DIVMTQSPDSLAVSLGERATINCKSSQSVLHISNNNNYLAWYQQQPGQPPKLLIYWASIRESGVPDRFSGSGSGTDFTLTISSLQAEDVAVYYCQQYYSIPCSFGQGTKLEIK | QVQLVQSGAEVKKPGASVKVSCKASGYTFTNYDINWVRQATGQGLEWMGWMTPNSGNTGYAQKFQGRVTMTRNTSISTAYMELSSLKSEDTAVYYCAYSSGWYRFDYWGQGTLVTVSS |
| | | | SEQ ID NO: 25506 | SEQ ID NO: 29512 |
| iPS:434237 | 21-225_61B5 | NA | GACATCGTGATGACCCAGTCTCCAGACTCCCTGGCTGTGTCTCTGGGCGAGAGGGCCACCATCAACTGCAAGTCCAGCCAGAGTGTTTTATACAGCTCCAACAATAACAACTCCTTAACTTGGTACCAGCTGAAACCAGGACAGCCTCCTAAGAAGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCTCCGACGTTCGGCCAAGGGTCCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAGCACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGTATAGCAGTGGCTGGTACTACTTTGACTACTGGGGCCAGGGAACCCTGGTCACCGTCTCCTCA |
| | | | SEQ ID NO: 25507 | SEQ ID NO: 29513 |
| | | AA | DIVMTQSPDSLAVSLGERATINCKSSQSVLYSSNNNNSLTWYQLKPGQPPKKLIYWASTRESGVPDRFSGSGSGTDFTLTISSLQAEDVAVYYCQQYYSTPPTFGQGSKVEIK | QVQLVQSGAEVKKPGASVKVSCKASGYTFTNYDINWVRQATGQGLEWMGWMHPNSGSTGYAQKFQGRVTMTRNTSISTAYMELSSLRSEDTAVYYCAYSSGWYYFDYWGQGTLVTVSS |
| | | | SEQ ID NO: 25508 | SEQ ID NO: 29514 |

FIGURE 50

| | | | | |
|---|---|---|---|---|
| **iPS:434239** | **21-225_58F1** | NA | GACATCCAGATGACCCAGTCTCCATCCTCCCTGTCTGCATCTGTAGGAGACAGAGTCACCATCACTTGCCGGGCAAGTCAGAGCATTACCAACTTTTTAAATTGGTATCAGCAGAAACCAGGGAAAGCGCCTAAACTCCTGATCTTCGCTGCATCCAGTTTGCAAAGTGGAATCCCATCAAGGTTCAGTGGCAGTGGATCTGGGACAGATTTCACTCTCACCATCAGCAGTCTGCAACCTGAAGATTTTGCAACTTACTACTGTCAACAGAGTTACAGTATCCCGTGGACGTTCGGCCAAGGGACCAAGGTGGAAATCAAA | GAGGTGCAGCTGTTGGAGTCTGGGGGAGGCTTGGTTCAGCCTGGGGGGTCCCTGAGACTCTCCTGTGCAGCCTCTGGATTCACCTTTAGCAGCTATGCCATGAGTTGGGTCCGCCAGGCTCCAGGGAAGGGGCTGGAGTGGGTCTCAGCTATTAGTACTGGTGGTGGTAACACATACTACGCAGACTCCGTGAAGGGCCGGTTCACCATTTCCAGAGACAATTCCAAGAACACGCTGTATCTGCAAATGAACAGCCTGAGAGCCGAGGACACGGCCGTATATTACTGTGCGAAACGGGGGGTCTACGGTGACTTTGATGCTTTTGATATCTGGGGCCAAGGGACAATGGTCACCGTCTCTTCA |
| | | | SEQ ID NO: 25509 | SEQ ID NO: 29515 |
| | | AA | DIQMTQSPSSLSASVGDRVTITCRASQSITNFLNWYQQKPGKAPKLLIFAASSLQSGIPSRFSGSGSGTDFTLTISSLQPEDFATYYCQQSYSIPWTFGQGTKVEIK | EVQLLESGGGLVQPGGSLRLSCAASGFTFSSYAMSWVRQAPGKGLEWVSAISTGGGNTYYADSVKGRFTISRDNSKNTLYLQMNSLRAEDTAVYYCAKRGVYGDFDAFDIWGQGTMVTVSS |
| | | | SEQ ID NO: 25510 | SEQ ID NO: 29516 |
| **iPS:434241** | **21-225_61E6** | NA | GACATCCAGATGACCCAGTCTCCATCCTCCCTGTCTGCATCTGTAGGAGACAGAATCACCATCACTTGCCGGGCAAGTCAGGGCATTGGAAATGATTTAGGCTGGTATCAGCAGAAACCAGGGAAAGCCCCTGAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAACATAATAGTTTCCCTCCGTGGACGTTCGGCCAAGGGACCAAGGTGGAAATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGAAGCTATGCCATGAGCTGGGTCCGCCAGGCTCCAGGGAAGGGGCTGGAGTGGGTCTCAGCTACTAGTGGTAGTGGTGTTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATTGGAACTGGGGATCTTTGACTACTGGGGCCAGGGAACCCTGGTCACCGTCTCCTCA |
| | | | SEQ ID NO: 25511 | SEQ ID NO: 29517 |

FIGURE 50

| | | | | |
|---|---|---|---|---|
| | | AA | DIQMTQSPSSLSASVGDRITITCRASQGIGNDLGWYQQKPGKAPERLIYAASSLQSGVPSRFSGSGSGTEFTLTISSLQPEDFATYYCLQHNSFPPWTFGQGTKVEIK | EVQLLESGGGLVQPGGSLRLSCAASGFTFRSYAMSWVRQAPGKGLEWVSATSGSGVNTFYADSVKGRFTISRDNSKNTLYLQMNSLRAEDTAVYYCAKLELGIFDYWGQGTLVTVSS |
| | | | SEQ ID NO: 25512 | SEQ ID NO: 29518 |
| **iPS:434243** | **21-225_62C1** | NA | GATATTGTGATGACTCAGTCTCCACTCTCCCTGCCCGTCACCCCTGGAGAGCCGGCCTCCATCTCCTGCAGGTCTAGTCAGAGCCTCCTACATAGTAATGGATACAACTATTTGGATTGGTACCTGCAGAAGCCAGGGCAGTCTCCACAGCTCCTGATCTATTTGGTTTCTAATCGGGCCTCCGGGGTCCCTGACAGGTTCAGTGGCAGTGGATCAGGCACAGATTTTACACTGAAAATCAGCAGAGTGGGGGCTGAGGATGTTGGGGTTTATTTCTGCCTGCAAGCTCTACAAACTCCTCTCACCTTCGGCCAAGGGACACGACTGGAGATTAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGACTGGAGTGGGTCTCATCCATTAGTAGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGATTTTTGGAGTGGACTGGGGCCAGGGAACCCTGGTCACCGTCTCCTCA |
| | | | SEQ ID NO: 25513 | SEQ ID NO: 29519 |
| | | AA | DIVMTQSPLSLPVTPGEPASISCRSSQSLLHSNGYNYLDWYLQKPGQSPQLLIYLVSNRASGVPDRFSGSGSGTDFTLKISRVGAEDVGVYFCLQALQTPLTFGQGTRLEIK | EVQLVESGGGLVKPGGSLRLSCAASGFTFSSYSMNWVRQAPGKGLEWVSSISSSSSYIYYADSVKGRFTISRDNAKNSLYLQMNSLRAEDTAVYYCAIFGVDWGQGTLVTVSS |
| | | | SEQ ID NO: 25514 | SEQ ID NO: 29520 |

FIGURE 50

| | | | | |
|---|---|---|---|---|
| iPS:434245 | 21-225_62H1 | NA | GACATCCAGATGACCCAGTCTCCATCCTCCCTGTCTGCATATGTAGGAGACAGAGTCACCATCACTTGCCGGGCAAGTCAGAACATTTTCAGCTATTTAAATTGGTATCAGCAGAAACCAGGGAAAGCCCCTAAGGTCCTGATCTATGCTGTATTTAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACAGTACCCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCCAGGGGCTGGAGTGGATGGCAATTATATGGTATGATGGAAGTAATAAATACTATGCAGACTCCGTGAAGGGCCGATTCACCATCTCCAGAGACAATTCCAAGAACACGCTGTATCTGCAAATCAACAGCCTGAGAGCCGAGGACACGGCTGTGTATTACTGTGCGAGAGAAGACCCGCGTACCAGCTGCTCTGACTACTGGGGCCAGGGAACCCTGGTCACCGTCTCCTCA |
| | | | SEQ ID NO: 25515 | SEQ ID NO: 29521 |
| | | AA | DIQMTQSPSSLSAYVGDRVTITCRASQNIFSYLNWYQQKPGKAPKVLIYAVFSLQSGVPSRFSGSGSGTDFTLTISSLQPEDFATYYCQQSYSTPFTFGPGTKVDIK | QVQLVESGGGVVQPGRSLRLSCAASGFTFSSYGMHWVRQAPGQGLEWMAIIWYDGSNKYYADSVKGRFTISRDNSKNTLYLQINSLRAEDTAVYYCAREDPRTSCSDYWGQGTLVTVSS |
| | | | SEQ ID NO: 25516 | SEQ ID NO: 29522 |
| iPS:434247 | 21-225_62D2 | NA | GACATCCAGATGACCCAGTCTCCATCCTCCCTGTCTGCATCTGTAGGAGACAGAGTCACCATCATTTGCCGGGCAAGTCAGAGCATTATCAGTTATTTAAATTGGTATCAGCAGAAACCAGGGAAAGCCCCTAAGCTCCTGATCTATGCTACATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGACTTACAGTCCCCCGCTCACTTTCGGCGGAGGGACCAAGGTGGAGATCAAA | CAGGTGTATCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTCTGCAGACTCCGTGAAGGGCCGATTCACCATCTCCAGAGACAATTCCAAGAACACGCTGGATCTGCAAATGAACAGCCTGAGAGCCGAGGACACGGCTGTGTATTACTGTGCGAGAGATAATGGTAACTGGAACTACCTTGACTACTGGGGCCAGGGAACCCTGGTCACCGTCTCCTCA |
| | | | SEQ ID NO: 25517 | SEQ ID NO: 29523 |

FIGURE 50

| | | | | |
|---|---|---|---|---|
| | | AA | DIQMTQSPSSLSASVGDRVTIICRASQSIISYLNWYQQKPGKAPKLLIYATSSLQSGVPSRFSGSGSGTDFTLTISSLQPEDFATYYCQQTYSPPLTFGGGTKVEIK | QVYLVESGGGVVQPGRSLRLSCAASGFTFSSYGMHWVRQAPGKGLEWVAVIWYDGSNKYSADSVKGRFTISRDNSKNTLDLQMNSLRAEDTAVYYCARDNGNWNYLDYWGQGTLVTVSS |
| | | | SEQ ID NO: 25518 | SEQ ID NO: 29524 |
| **iPS:434249** | **21-225_62E2** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCCGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAGTAATTACCCTCTCACTTTCGGCGGAGGGACCAGGGTTGAGATCAAA | CAGCTGCAGCTGCAGGAGTCGGGCCCAGGACTGGTGAAGCCTTCGGAGACCCTGTCCCTCACCTGTATTGTCTCTGGTGGCTCCATCAGCAGAAGTAGTTACTACTGGGGCTGGATCCGCCAGCCCCCAGGGAAGGGGCTGGAGTGGATTGGGAGCATCTATTATAGTGGGATCGCCTCCTATAATCCGTCCCTCAAGAGTCGAGTCACCATATCCGTAGACACGTCCAAGAACCAGTTCTCCCTGAAGCTGAACTCTGTGACCGCCACAGACACGGCTGTATATTACTGTGCGAGACTGAGCAGTGGCTGGTCCTTTGACTACTGGGGCCAGGGAACCCTGGTCACCGTCTCCTCA |
| | | | SEQ ID NO: 25519 | SEQ ID NO: 29525 |
| | | AA | DIQMTQSPSSLSASVGDRVTITCRASQGIRNDLGWYQQKPGKAPKRLIYAASRLQSGVPSRFSGSGSGTEFTLTISSLQPEDFATYYCLQHSNYPLTFGGGTRVEIK | QLQLQESGPGLVKPSETLSLTCIVSGGSISRSSYYWGWIRQPPGKGLEWIGSIYYSGIASYNPSLKSRVTISVDTSKNQFSLKLNSVTATDTAVYYCARLSSGWSFDYWGQGTLVTVSS |
| | | | SEQ ID NO: 25520 | SEQ ID NO: 29526 |

FIGURE 50

| | | | | |
|---|---|---|---|---|
| **iPS:434251** | **21-225_62G3** | NA | GACATCCATATGACCCAGTCTCCATCCTCCCTGTCTGCATCTGTAGGAGACAGAGTCACCATCACTTGCCGGGCAAGTCAGGACATTAGAAATAATTTAGGCTGGTATCAGCAGAAACCAGGGAAAGCCCCTAAGCGCCTGATCTATACTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCGACTTATTACTGTCTACAGTATAATAGTTACCCATTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTAGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTATATTACTGTGCGAGGGTTAACTCTTTTGACTCCTGGGGCCAGGGAACCCTGGTCACCGTCTCCTCA |
| | | | SEQ ID NO: 25521 | SEQ ID NO: 29527 |
| | | AA | DIHMTQSPSSLSASVGDRVTITCRASQDIRNNLGWYQQKPGKAPKRLIYTASSLQSGVPSRFSGSGSGTEFTLTISSLQPEDFATYYCLQYNSYPFTFGPGTKVDIK | EVQLVESGGGLVKPGGSLRLSCAASGFTFSSYSMNWVRQAPGKGLEWVSSISSSSSYIYYADSVKGRFTISRDNAKNSLYLQMNSLRAEDTAVYYCARVNSFDSWGQGTLVTVSS |
| | | | SEQ ID NO: 25522 | SEQ ID NO: 29528 |
| **iPS:434253** | **21-225_62E4** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTAATCTATGCTGCATTCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTATCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATAGAAGTAATAAATACTATGCAGACTCCGTGAAGGGCCGATTCACCATCTCCAGAGACAATTCCAAGAACACGCTGTATCTGCAAATGAACAGCCTGAGAGTCGAGGACACGGCTGTGTATCACTGTGCGAGAGAGCTTGGGTTCAGCAGTGACTACTGGGGCCAGGGAACCCTGGTCACCGTCTCCTCA |
| | | | SEQ ID NO: 25523 | SEQ ID NO: 29529 |

FIGURE 50

| | | | | |
|---|---|---|---|---|
| | | AA | DIQMTQSPSSLSASVGDRVTITCRASQGIRNDLG WYQQKPGKAPKRLIYAAFSLQSGVPSRFSGSGS GTEFTLTISSLQPEDFATYYCLQHNSYPLTFGGG TKVEIK | QVQLVESGGGVVQPGRSLRLSCAASGFTFSDYGM HWVRQAPGKGLEWVAVIWYDRSNKYYADSVKGR FTISRDNSKNTLYLQMNSLRVEDTAVYHCARELGF SSDYWGQGTLVTVSS |
| | | | SEQ ID NO: 25524 | SEQ ID NO: 29530 |
| iPS:434255 | 21-225_62E6 | NA | GAAATAGTGATGACGCAGTCTCCAGCCACCCT GTCTGTGTCTCCAGGGGAAAGAGCCACCCTCT CCTGCAGGGCCAGTCAGAGTGTTAACAGCAAC TTAGCCTGGTACCAGCAGAAACCTGGCCAGGC TCCCAGGCTCCTCATCTCTGTTGCATCCACCAG GGCCACTGGTATCCCAGCCAGGTTCAATGGCA GTGGGTCTGGGACAGAGTTCACTCTCACCATC AGCAGCCTGCAGTCTGAAGATTTTGCAGTTTA TTACTGTCAGCAGTATAATGACTGGCCGTGTA GTTTTGGCCAGGGGACCAAGCTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTTCCTATGGCAT GCACTGGGTCCGCCAGGCTCCAGGCAAGGGGCT GGAGTGGGTGGCAGCTATATGGTATGATGGAAG TAATAAATACTATGGAGACTCCGTGAAGGGCCG AGTCACCATCTCCAGAGACAATTCCAAGAACTCG CTGCATCTGCAAATGAACAGCCTGAGAGCCGAG GACACGGCTGTGTATTATTGTGCGAGAGATCAGG GCATAGTGGGAGCTACTTGGTTTGACTACTGGGG CCAGGGAACCCTGGTCACCGTCTCCTCA |
| | | | SEQ ID NO: 25525 | SEQ ID NO: 29531 |
| | | AA | EIVMTQSPATLSVSPGERATLSCRASQSVNSNLA WYQQKPGQAPRLLISVASTRATGIPARFNGSGSG TEFTLTISSLQSEDFAVYYCQQYNDWPCSFGQGT KLEIK | QVQLVESGGGVVQPGRSLRLSCAASGFTFSSYGMH WVRQAPGKGLEWVAAIWYDGSNKYYGDSVKGRV TISRDNSKNSLHLQMNSLRAEDTAVYYCARDQGIV GATWFDYWGQGTLVTVSS |
| | | | SEQ ID NO: 25526 | SEQ ID NO: 29532 |

FIGURE 50

| | | | | |
|---|---|---|---|---|
| **iPS:434257** | **21-225_62F7** | NA | GACATCCAGATGACCCAGTCTCCATCCTCCCTGTCTGCATCTGTAGGAGACAGAGTCACCATCACTTGCCGGGCAAGTCAGGCCATTAGAAATGATTTAGGCTGGTATCAGCAGAAACCAGGGAAAGCCCCTAAGCGCCTGATCTATCCTGCATCCCGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGTATAATAGTTACCCTCCGTGGACGTTCGGCCAAGGGTCCAAGGTGGAAATCAAA | GAGGTGCAGCTGTTGGAGTCTGGGGGAGGCTTGGTTCAGCCTGGGGGGTCCCTGAGACTCTCCTGTGCAGCCTCTGGATTCACCTTTAGCAGCTATGTTATGAGCTGGGTCCGCCAGGCTCCAGGGAAGGGGCTGGAGTGGGTCTCAGGTATTAGTGGTAGTGGTGCTAAAACATACTATGCAGACTCCGTGAAGGGCCGATTCACCATCTCCAGAGACAATTCCAAGAACACGCTGTATCTGCAAATGAACAGCCTGAGAGCCGAGGACACGGCCGTATATTACTGTGCGGAACTGGGGATAGACTACTACTACGGTATGGACGTCTGGGGCCAAGGGACCACGGTCACCGTCTCCTCA |
| | | | SEQ ID NO: 25527 | SEQ ID NO: 29533 |
| | | AA | DIQMTQSPSSLSASVGDRVTITCRASQAIRNDLGWYQQKPGKAPKRLIYPASRLQSGVPSRFSGSGSGTEFTLTISSLQPEDFATYYCLQYNSYPPWTFGQGSKVEIK | EVQLLESGGGLVQPGGSLRLSCAASGFTFSSYVMSWVRQAPGKGLEWVSGISGSGAKTYYADSVKGRFTISRDNSKNTLYLQMNSLRAEDTAVYYCAELGIDYYYGMDVWGQGTTVTVSS |
| | | | SEQ ID NO: 25528 | SEQ ID NO: 29534 |
| **iPS:434259** | **21-225_62G7** | NA | GACATCCAGATGACCCAGTCTCCATCTTCCGTGTCTGCATCTGTAGGAGACAGAGTCACCTTCACTTGTCGGGCGAGTCAGGATATTAGCAGCTGGTTAGCCTGGTATCAGCAGAATCCAGGGAAGGCCCCTAAACTCCTGATCTATGCTGCATCCAGTTTGCAAAGTGGGGTCCCATCAAGGTTCAGCGGCAGTGGATCTGGGACAAATTTCACTCTCACCATCAGCAGCCTCCAGCCTGAAGATTTTGCAACTTACTATTGTCAACAGACTAACAGTTTCCCTCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGACTTGAGTGGATGGGATGGATCAAACCTAAAAGTGGTGGCACAAACCAAGCACAGAAGTTTCAGGGCAGGGTCACCATGACCAGGGACACGTCCATCAGCACAGCCTACATGGAGCTGAGCAGTCTGAGATCTGACGACACGGCCGTGTATTACTGTGCGAGAGCTCCGGGTATAGCAGCAGCTGGTACATGGGGATACTTTGACTACTGGGGCCAGGGAACCCTGGTCACCGTCTCCTCA |
| | | | SEQ ID NO: 25529 | SEQ ID NO: 29535 |

FIGURE 50

| | | | | |
|---|---|---|---|---|
| | | AA | DIQMTQSPSSVSASVGDRVTFTCRASQDISSWLAWYQQNPGKAPKLLIYAASSLQSGVPSRFSGSGSGTNFTLTISSLQPEDFATYYCQQTNSFPLTFGGGTKVEIK | QVQLVQSGAEVKKPGASVKVSCKASGYTFTGYYMHWVRQAPGQGLEWMGWIKPKSGGTNQAQKFQGRVTMTRDTSISTAYMELSSLRSDDTAVYYCARAPGIAAAGTWGYFDYWGQGTLVTVSS |
| | | | SEQ ID NO: 25530 | SEQ ID NO: 29536 |
| iPS:434261 | 21-225_56F7 | NA | GACATCCTGATGACCCAGTCTCCATCCTCACTGTCTGCATCTGTAGGAGACAGAGTCACCATCACTTGTCGGGCGAGTCAGGGCATTAGCACTTATTTAGCCTGGTTTCAGCAGACACCAGGGACAGCCCCTAAGTCCCTGATCTATGCTGCATCCAGTTTGCAAGGTGGGGTCCCATCAAAGTTCAGCGGCAGTGGATCTGGGACAGATTTCACTCTCACCATCAGCAGCCTGCAGCCTGAAGATTTTGCAACTTATTACTGCCATCAGTATAATAGTTTCCCATTTAAGTTCGGGCGTGGGACCAAAGTGGATATCACA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGTCTTAAACTGGGTCCGCCAGGCTCCAGGGAAGGGGCTGGAGTGGGTCTCAGCTATGAGTGGTAGTGGTGGTAGAACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTTCTGTGCGATGACTACGCACTTTGACTACTGGGGCCAGGGAACCCTGGTCACCGTCTCCTCA |
| | | | SEQ ID NO: 25531 | SEQ ID NO: 29537 |
| | | AA | DILMTQSPSSLSASVGDRVTITCRASQGISTYLAWFQQTPGTAPKSLIYAASSLQGGVPSKFSGSGSGTDFTLTISSLQPEDFATYYCHQYNSFPFKFGRGTKVDIT | EVQLLESGGGLVQPGGSLRLSCAASGFTFSSYVLNWVRQAPGKGLEWVSAMSGSGGRTYYADSVKGRFTISRDNSKNTLYLQMNSLRAEDTAVYFCAMTTHFDYWGQGTLVTVSS |
| | | | SEQ ID NO: 25532 | SEQ ID NO: 29538 |

FIGURE 50

| | | | | |
|---|---|---|---|---|
| iPS:434263 | 21-225_56H7 | NA | GACATCCAGCTGACCCAGTCTCCATCCTCCCTGTCTGCATCTGTAGGAGACAGAGTCACCATCACTTGCCGGGCAAGTCAGGACATTAGAAATGATTTAGGCTGGTATCAGCAGAGGCCAGGGAAAGCCCCTAAGCGCCTGATCTATCCTGCATCCAGTTTGCTAAGTGGGGTCCCATCAAGGTTCAGCGGCAGTGGATCTGGGACAGAATTCACTCTCACAATCAGCAGCCTGCAGCCTGAAGATTTTGCAACTTACTACTGTCTACAGGATAATAGTTACCCATTCACTTTCGGCCCTGGGACCAAAGTGGATAGCAAA | GAGGTGCAGCTGGTGGAGTCTGGGGGAGGCCTGGTCAAGCCTGGGGGGTCCCTGAGACTCTCCTGTGCAGCCTCTGGATTCTCCTTCAGTAGCTATAGCATGAACTGGGTCCGCCAGGCTCCAGGGAAGGGGCTGGAGTGGGTCTCATCCATTAGTAGTAGTAGTACTTACATATACTACGGAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGATCGGGGCAGCAGCTGGGGCCAGGGAACCCTGGTCACCGTCTCCTCA |
| | | | SEQ ID NO: 25533 | SEQ ID NO: 29539 |
| | | AA | DIQLTQSPSSLSASVGDRVTITCRASQDIRNDLGWYQQRPGKAPKRLIYPASSLLSGVPSRFSGSGSGTEFTLTISSLQPEDFATYYCLQDNSYPFTFGPGTKVDSK | EVQLVESGGGLVKPGGSLRLSCAASGFSFSSYSMNWVRQAPGKGLEWVSSISSSSTYIYYGDSVKGRFTISRDNAKNSLYLQMNSLRAEDTAVYYCARDRGSSWGQGTLVTVSS |
| | | | SEQ ID NO: 25534 | SEQ ID NO: 29540 |
| iPS:434265 | 21-225_57B2 | NA | GACATCCAGATGACCCAGTCTCCATCCTCCCTGTCTGTATCTGTAGGAGACAGAGTCACCATCACTTGCCGGGCAAGTCAGGGCATTAGAAATGCTTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTAGTTACATAAACTACACAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGTGGCTGGCTTTGACTACTGGGGCCAGGGAACCCTGGTCACCGTCTCCTCA |
| | | | SEQ ID NO: 25535 | SEQ ID NO: 29541 |

FIGURE 50

| | | | | |
|---|---|---|---|---|
| | | AA | DIQMTQSPSSLSVSVGDRVTITCRASQGIRNALGWYQQKPGKAPKRLIYAASSLQSGVPSRFSGSGSGTEFTLTISSLQPEDFATYYCLQHNSYPFTFGPGTKVDIK | EVQLVESGGGLVKPGGSLRLSCAASGFTFSSYSMNWVRQAPGKGLEWVSSISGSSSYINYTDSVKGRFTISRDNAKNSLYLQMNSLRAEDTAVYYCARVAGFDYWGQGTLVTVSS |
| | | | SEQ ID NO: 25536 | SEQ ID NO: 29542 |
| **iPS:434267** | **21-225_57F2** | NA | GACATCCAGATGACCCAGTCTCCATCCTCCCTGTCTGCATCTGTAGGAGACAGAGTCACCATCACTTGCCGGGCAAGTCAGAGCATTAGCAGCTATTTAAATTGGTATCAGCAGAAACCAGGGAAAGCCCCTAAGCTCCTGATCTATGCTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCACCTTACTACTGTCAACAGAGTTACAATATTTCATTCACTTTCGGCCCTGGGACCAAAGTGGATATCAAA | CAGGTGCAGCTGCAGGAGTCGGGCCCAGGACTGGTGAAGCCTTCGGAGACCCTGTCCCTCACCTGCACTGTCTCTGGTGGCTCCATCAGTAGTTACTACTGGAGCTGGATCCGGCAGCCCGCCGGGAAGGGACTGGAGTGGATTGGGCGCATCTATACCAGGGGGAGCACCAACTACAACCCCTCCCTCAAGAGTCGAGTCACCATGTCAATAGACACGTCCAAGAACCAGTTCTCCCTGAAGCTGAGCTCTGTGACCGCCGCGGACACGGCCGTGTATTACTGTGCGAGAGAGGGAAAAACTGGGGGGGTTTCTTACTTTGACTACTGGGGCCAGGGAACCCTGGTCACCGTCTCCTCA |
| | | | SEQ ID NO: 25537 | SEQ ID NO: 29543 |
| | | AA | DIQMTQSPSSLSASVGDRVTITCRASQSISSYLNWYQQKPGKAPKLLIYAASSLQSGVPSRFSGSGSGTDFTLTISSLQPEDFAPYYCQQSYNISFTFGPGTKVDIK | QVQLQESGPGLVKPSETLSLTCTVSGGSISSYYWSWIRQPAGKGLEWIGRIYTRGSTNYNPSLKSRVTMSIDTSKNQFSLKLSSVTAADTAVYYCAREGKTGGVSYFDYWGQGTLVTVSS |
| | | | SEQ ID NO: 25538 | SEQ ID NO: 29544 |

FIGURE 50

| | | | | |
|---|---|---|---|---|
| **iPS:434269** | **21-225_57H3** | NA | GAAATAGTGATGACGCAGTCTCCAGCCACCCTGTCTGTGTCTCCAGGGGAAAGAGCCACCCTCTCCTGCAGGGCCAGTCAGAGTGTTAGCAGCAGCTTAGCCTGGTACCAGCAGAAACCTGGCCAGGCTCCCAGGCTCCTCATCTATGGTGCATCCACCAGGGCCACTGGTTTCCCAGCCAGGTTCAATGGCAGTGGGTCTTGGACAGAATTCACTCTCACCATCAGCAGCCTGCAGTCTGAAGATTTTGCAATTTATTACTGTCAGCAGTATAATGACTGGCCGTGCAGTTTTGGCCAGGGGACCAAGCTGGAGATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGCTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATTATGGTATAGTGGGAGCTACATATTTTGACTACTGGGGCCAGGGAACCCTGGTCACCGTCTCCTCA |
| | | | SEQ ID NO: 25539 | SEQ ID NO: 29545 |
| | | AA | EIVMTQSPATLSVSPGERATLSCRASQSVSSSLAWYQQKPGQAPRLLIYGASTRATGFPARFNGSGSWTEFTLTISSLQSEDFAIYYCQQYNDWPCSFGQGTKLEIK | QVQLVESGGGVVQPGRSLRLSCAASGFTFSSYGMHWVRQAPGKGLEWVAAIWYDGSNKYYADSVKGRFTISRDNSKNTLYLQMNSLRAEDTAVYYCARDYGIVGATYFDYWGQGTLVTVSS |
| | | | SEQ ID NO: 25540 | SEQ ID NO: 29546 |
| **iPS:434271** | **21-225_57A4** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCTGCAGAAACCAGGGAAAGCCCCTAAGCGCCTGATCTATGCTGCATCCAGTTTGCTAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGTAGCGTCTGGATTCACCTTCAGTGACTATGGCATGCACTGGGTCCGCCAGGCTCCAGGCAAGGGGCTGGAGTGGGTGGCAGTTATATGGTATGCTGGAAGTAATAAATACTATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGGAACTGGGGATGAGGTCTGACTACTGGGGCCAGGGAACCCTGGTCACCGTCTCCTCA |
| | | | SEQ ID NO: 25541 | SEQ ID NO: 29547 |

FIGURE 50

| | | | | |
|---|---|---|---|---|
| | | AA | DIQMTQSPSSLSASVGDRVTITCRASQGIRNDLGWYLQKPGKAPKRLIYAASSLLSGVPSRFSGSGSGTEFTLTISSLQPEDFATYYCLQHNSYPFTFGPGTKVDIK | QVQLVESGGGVVQPGRSLRLSCVASGFTFSDYGMHWVRQAPGKGLEWVAVIWYAGSNKYYVDSVKGRFTISRDNSKNTLYLQMNSLRAEDTAVYYCARELGMRSDYWGQGTLVTVSS |
| | | | SEQ ID NO: 25542 | SEQ ID NO: 29548 |
| iPS:434273 | 21-225_57E4 | NA | GACATCCAGATGACCCAGTCTCCATCTTCCGTGTCTGCATCTGTAGGAGACAGAGTCTCCATCACTTGTCGGGCGAGTCAGGATATTAGCAACTGGTTAGCCTGGTATCAGCAGAAACCAGGGAAAGCCCCTAAGCTCCTGATCTATGCTGCATCCAGTTTGCAAAGTGGGGTCCCATCAAGGTTCAGCGGCAGTGGTTCTGGGACAGATTTCACTCTCACCATCAGCAGCCTGCAGCCTGAAGATTTTGCAACTTACTCTTGTCAACAGGGTAACAGTTTCCCATTCACTTTCGGCCCTGGGACCAAAGTGGATATCAAA | GAGGTGCAGCTGTTGGAGTCTGGGGGAGGCTTGGTACAGCCGGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTATTAGTGGTAGTGGTGGTAGTACATTCTACGCAGACTCCGTGAAGGGCCGGTTCACCATCTCCAGAGACAATTCCAAGACCACGCTGTATCTGCAAATGAACAGCCTGAGAGCCGAGGACACGGCCGTATATTACTGTGCGAAAAGGGACTGGAACGACGTTTTTGACTACTGGGGCCAGGGAACCCTGGTCACCGTCTCCTCA |
| | | | SEQ ID NO: 25543 | SEQ ID NO: 29549 |
| | | AA | DIQMTQSPSSVSASVGDRVSITCRASQDISNWLAWYQQKPGKAPKLLIYAASSLQSGVPSRFSGSGSGTDFTLTISSLQPEDFATYSCQQGNSFPFTFGPGTKVDIK | EVQLLESGGGLVQPGGSLRLSCAASGFTFSSYAMSWVRQAPGKGLEWVSVISGSGGSTFYADSVKGRFTISRDNSKTTLYLQMNSLRAEDTAVYYCAKRDWNDVFDYWGQGTLVTVSS |
| | | | SEQ ID NO: 25544 | SEQ ID NO: 29550 |

FIGURE 50

| | | | | |
|---|---|---|---|---|
| **iPS:434275** | **21-225_57F4** | NA | GACATCCAGATGACCCAGTCTCCATCCTCCCTGTCTGCATCTGTAGGAGACAGAGTCACCATCACTTGCCGGGCAAGTCAGGTCATTAGAAATGATTTAGGCTGGTATCAGCAGAAGCCAGGGAAAGCCCCTAAGCGCTTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATCACTGTCTACAGTATGGTAGTTTCCCATTCACTTTCGGCCCTGGGACCAAAGTGGATATCAAA | CAGGTGCAGCTGGTGGAGTCTGGGGGAGGCTTGGTCAAGCCTGGAGGGTCCCTGAGACTGTCCTGTGCAGCCTCTGGATTCATCTTCAGTGACTACTACATGAACTGGATCCGCCAGGCTCCAGGGAAGGGGCTGGAGTGGGTTTCATACATTAGTAGTAGTGGTAGTACCATATACTACGCAGACTCTGTGAAGGGCCGATTCACCATCTCCAGGGACAACGCCAAGAACTCACTGTATCTGCAAATGAACAGCCTGAGAGCCGAGGACACGGCCGTGTATTACTGTGCGAGAGATATGATTACGTGGGGCCAGGGAACCCTGGTCACCGTCTCCTCA |
| | | | SEQ ID NO: 25545 | SEQ ID NO: 29551 |
| | | AA | DIQMTQSPSSLSASVGDRVTITCRASQVIRNDLGWYQQKPGKAPKRLIYAASSLQSGVPSRFSGSGSGTEFTLTISSLQPEDFATYHCLQYGSFPFTFGPGTKVDIK | QVQLVESGGGLVKPGGSLRLSCAASGFIFSDYYMNWIRQAPGKGLEWVSYISSSGSTIYYADSVKGRFTISRDNAKNSLYLQMNSLRAEDTAVYYCARDMITWGQGTLVTVSS |
| | | | SEQ ID NO: 25546 | SEQ ID NO: 29552 |
| **iPS:434277** | **21-225_57A7** | NA | GACATCCAGATGACCCAGTCTCCATCTTCCGTGTCTGCATCTGTAGGAGACAGAGTCACCATCACTTGTCGGGCGAGTCAGGGTATTAGCAGGTGGTTAGCCTGGTATCAGCAGAAACCAGGGAAAGCCCCTAACCTCCTGATCTATGCTGCATCCAATTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGGCTAACAGTTTCCCATTCACTTTCGGCCCTGGGACCAAAGTAGATATCAAA | CAGGTGCAACTGGTGCAGTCTGGGGCTGAGGTGAAGAAGCCTGGGGCCTCAGTGAAGGTCTCCTGCAAGGCTTCTGGATACACCTTCACCGGCTACCATATACACTGGGTGCGACAGGCCCCTGGACAAGATCTTGAGTGGATGGGATGGATCAACCCTAACAATAATGGCACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGAGATGGGAGAAGTGGTTTTGACTACTGGGGCCAGGGAACCCTGGTCACCGTCTCCTCA |
| | | | SEQ ID NO: 25547 | SEQ ID NO: 29553 |

FIGURE 50

| | | | | |
|---|---|---|---|---|
| | | AA | DIQMTQSPSSVSASVGDRVTITCRASQGISRWLAWYQQKPGKAPNLLIYAASNLQSGVPSRFSGSGSGTDFTLTISSLQPEDFATYYCQQANSFPFTFGPGTKVDIK | QVQLVQSGAEVKKPGASVKVSCKASGYTFTGYHIHWVRQAPGQDLEWMGWINPNNNGTNYAQKFQGRVTMTRDTSISTAYMELSRLRSDDTAVYYCARDGRSGFDYWGQGTLVTVSS |
| | | | SEQ ID NO: 25548 | SEQ ID NO: 29554 |
| iPS:434279 | 21-225_57F7 | NA | GAAATAGTGATGACGCAGTCTCCAGCCATCCTGTCTGTGTTTCCAGGGGAAAGAGCCACCCTCTCCTGCAGGGCCAGTCAGAGTGTTAGCAGCGACTTAGCCTGGTACCAGCAGAAACCTGGCCAGGCTCCCAGGCTCCTCATCTATGGTGCATCCACCAGGGCCACTGGTATGCCAGCCAGGTTCAGTGGCGGTGGGTCTGGGACAGAGTTCACTCTCACCATCAGCAGCCTGCAGTCTGAACATTTTGCAGTTTATTACTGTCAGCAGTATAGTAACTGGCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTAGTGGTGGTAACACATTCTACGCAGACTCCGTGAAGGGCCGGTTCACCATCTCCAGAGACAATTCCAAGAACACGCTGTATTTGCAAATGAACAGCCTGAGAGCCGAGGACACGGCCGTATATTACTGTGCGAAATTTTTCGGTGTAGTGGGAGTCGGGTGCTTTGACTACTGGGGCCAGGGAACCCTGGTCACCGTCTCCTCA |
| | | | SEQ ID NO: 25549 | SEQ ID NO: 29555 |
| | | AA | EIVMTQSPAILSVFPGERATLSCRASQSVSSDLAWYQQKPGQAPRLLIYGASTRATGMPARFSGGGSGTEFTLTISSLQSEHFAVYYCQQYSNWPFTFGPGTKVDIK | EVQLLESGGGLVQPGGSLRLSCAASGFTFSSYAMSWVRQAPGKGLEWVSAISGSGGNTFYADSVKGRFTISRDNSKNTLYLQMNSLRAEDTAVYYCAKFFGVVGVGCFDYWGQGTLVTVSS |
| | | | SEQ ID NO: 25550 | SEQ ID NO: 29556 |

FIGURE 50

| | | | | |
|---|---|---|---|---|
| iPS:434281 | 21-225_57B8 | NA | GACATCCAGATGACCCAGTCTCCATCCTCCCTGTCTGCATCTGTAGGAGACAGAATCACCATCACTTGCCGGGCAAGTCAGGGCATTGGAAATGATTTAGGCTGGTATCAGCAGAAACCAGGGAAAGCCCCTGAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAACATAATAGTTTCCCTCCGTGGACGTTCGGCCAAGGGACCAAGGTGGAAATCAAA<br>SEQ ID NO: 25551 | GAGGTGCAGCTGTTGGAGTCTGGGGGAGGCTTGGTACAGCCTGGGGGGTCCCTGAGACTCTCCTGTGCAGCCTCTGGATTCACCTTTAGAAGCTATGCCATGAGCTGGGTCCGCCAGGCTCCAGGGAAGGGGCTGGAGTGGGTCTCAGCTATTAGTGGTAGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATTGGAACTGGGGATCTTTGACTACTGGGGCCAGGGAACCCTGGTCACCGTCTCCTCA<br>SEQ ID NO: 29557 |
| | | AA | DIQMTQSPSSLSASVGDRITITCRASQGIGNDLGWYQQKPGKAPERLIYAASSLQSGVPSRFSGSGSGTEFTLTISSLQPEDFATYYCLQHNSFPPWTFGQGTKVEIK<br>SEQ ID NO: 25552 | EVQLLESGGGLVQPGGSLRLSCAASGFTFRSYAMSWVRQAPGKGLEWVSAISGSGGNTFYADSVKGRFTISRDNSKNTLYLQMNSLRAEDTAVYYCAKLELGIFDYWGQGTLVTVSS<br>SEQ ID NO: 29558 |
| iPS:434283 | 21-225_57F8 | NA | GACATCCAGATGACCCAGTCTCCATCTTCCGTGTCTGCATCTGTAGGAGACAGAGTCACCATCACTTGTCGGGCGAGTCAGGGTATTAGCAACTGGTTAGCCTGGTATCAGCAGAAACCAGGGAAAGCCCCTAAGCTCCTGATCTATACTGCATCCAGTTTGCAAAGTGGGGTCCCATCAAGGTTCAGCGGCAATGAATCTGGGACAGATTTCACTCTCACCATCAGCAGCCTGCAGCCTGAAGATTTTGCAACTTACTATTGTCAACAGGCTAACAGTTTCCCGTGGACGTTCGGCCAAGGGACCAAGGTGGAAATCAAA<br>SEQ ID NO: 25553 | GAGGTGCAGCTGTTGGAGTCTGGGGGAGGCTTGGTACAGCCGGGGGGGTCCCTGAGACTCTCCTGTGTAGTCTCTGGATTCACCTTTAGCAACTATGCCATGAGCTGGGTCCGCCAGGCTCCAGGGAAGGGGCTGGAGTGGGTCTCAGCTAGCAGTGGTAGTGGTGGTAACACATTCTACGCAGACTCCGTGACGGGCCGGGTCACCATCTCCAGAGACAATTCCAAGAACACGCTGTATCTGCAAATGAACAGTCTGAGAGCCGAGGACACGGCCGTATATTACTGTGCGAAACTGGGGAAGGACTACCACTACTACGGTATGGACGTCTGGGGCCAAGGGACCACGGTCACCGTCTCCTCA<br>SEQ ID NO: 29559 |

FIGURE 50

| | | | | |
|---|---|---|---|---|
| | | AA | DIQMTQSPSSVSASVGDRVTITCRASQGISNWLA WYQQKPGKAPKLLIYTASSLQSGVPSRFSGNESG TDFTLTISSLQPEDFATYYCQQANSFPWTFGQGT KVEIK | EVQLLESGGGLVQPGGSLRLSCVVSGFTFSNYAMS WVRQAPGKGLEWVSASSGSGGNTFYADSVTGRVT ISRDNSKNTLYLQMNSLRAEDTAVYYCAKLGKDY HYYGMDVWGQGTTVTVSS |
| | | | SEQ ID NO: 25554 | SEQ ID NO: 29560 |
| **iPS:434285** | **21-225_57A11** | NA | GACATCGTGATGACCCAGTCTCCAGACTCCCT GACTGTGTCTCTGGGCGAGAGGGCCACCATCA ACTGCAAGTCCAGCCAAAGTGTTTTACACAGC TCCAACAATTATAACTACTTAGCTTGGTACCA GCAGAGACCAGGACAGCCTCCTAAGCTGGTCA TTTACTGGGCATCTACCCGGGCATCCGGGGTC CCTGACCGATTCAGTGGCAGCGGGTCTGGGAC AGATTTCACTCTCACCATCAGCAGCCTGCAGG CTGAAGATATGGCAGTTTATTACTGTCAGCAA TATTATAGTACTCCGTGGACGTTCGGCCAAGG GACCAAGGTGGAATTCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTGT TAACACAGGCTATGCACAGAAGTTCCAGGGCAG AGTCACCATGACCAGGGACACCTCCATAAGCAC AGCCTACATGGAGCTGAGCAGCCTGAGATCTGA GGACACGGCCGTGTATTACTGTGCGATCAGCAGT GGCTGGAACTGGTTCGACCCCTGGGGCCAGGGA ACCCTGGTCACCGTCTCCTCA |
| | | | SEQ ID NO: 25555 | SEQ ID NO: 29561 |
| | | AA | DIVMTQSPDSLTVSLGERATINCKSSQSVLHSSN NYNYLAWYQQRPGQPPKLVIYWASTRASGVPD RFSGSGSGTDFTLTISSLQAEDMAVYYCQQYYST PWTFGQGTKVEFK | QVQLVQSGAEVKKPGASVKVSCKASGYTFTNYDIN WVRQATGQGLEWMGWMNPNSVNTGYAQKFQGR VTMTRDTSISTAYMELSSLRSEDTAVYYCAISSGW NWFDPWGQGTLVTVSS |
| | | | SEQ ID NO: 25556 | SEQ ID NO: 29562 |

FIGURE 50

| | | | | |
|---|---|---|---|---|
| iPS:434287 | 21-225_57F12 | NA | GACATCGTGATGACCCAGTCTCCAGACTCCCTGGCTGTGTCTCTGGGCGAGAGGGCCACCATCAACTGCAAGTCCAGCCAGAGTGTTTTATTCAGCTCCAACAATTACAATTACTTAGCTTGGTACCAGCAGAAAACAGGACAGCCTCCCAAGCTGATCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAATTTATTACTGTCAGCAATATTATAGTAATCCGTGTAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGATAAGCAGTGGCTGGTACCGGTTCGACCCCTGGGGCCAGGGAACCCTGGTCACCGTCTCCTCA |
| | | | SEQ ID NO: 25557 | SEQ ID NO: 29563 |
| | | AA | DIVMTQSPDSLAVSLGERATINCKSSQSVLFSSNNYNYLAWYQQKTGQPPKLIIYWASTRESGVPDRFSGSGSGTDFTLTISSLQAEDVAIYYCQQYYSNPCSFGQGTKLEIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYYCAISSGWYRFDPWGQGTLVTVSS |
| | | | SEQ ID NO: 25558 | SEQ ID NO: 29564 |
| iPS:434289 | 21-225_57H12 | NA | GAAATAGTGATGACGCAGTCTCCAGCCACCCTGTCTGTGTCTCCAGGGGAAAGAGCCACCCTCTCCTGCAGGGCCAGTCAGAGTGTTAGCAGCGACTTAGCCTGGTACCAGCAGAAACCTGGCCAGGCTCCCAGGCTCCTCATCTATGCTGCATCTACCAGGGCCACTGGTATCCCAGCCAGGTTCAGTGGCAGTGGGTCTGGGACAGAGTTCACTCTCACCATCAGCAGCCTGCAGTCTGAAGATTTTGCAGTTTATTACTGTCAGCAGTATGATAACTGGCCATTCACTTTCGGCCCTGGGACCAAAGTGGATAACAAG | GAGGTGCAGCTGTTGGAGTCTGGGGGAGGCTTGGTACAGCCGGGGGGGTCCCTGAGACTCTCCTGTGCAGCCTCTGGATTAACGTTTAGTAGCTACGCCATGAGCTGGGTCCGCCAGGATCCAGGGAAGGGGCTGGAGTGGGTCTCAGCTATTAGTGGTAGTGGTGGTAACACATTCTACGGAGACTCCGTGAAGGGCCGGTTCACCATCTCCAGAGACAATTCCAAGAAAACGCTGTATCTGCAAATGAACAGCCTGAGAGCCGAGGACACGGCCGTATATTACTGTGCGAAATTTTTCGGTATAGTGGGTGCCGGGTACTTTGACTACTGGGGCCAGGGAACCCTGGTCACCGTCTCCTCA |
| | | | SEQ ID NO: 25559 | SEQ ID NO: 29565 |

FIGURE 50

| | | | | |
|---|---|---|---|---|
| | | AA | EIVMTQSPATLSVSPGERATLSCRASQSVSSDLA WYQQKPGQAPRLLIYAASTRATGIPARFSGSGSG TEFTLTISSLQSEDFAVYYCQQYDNWPFTFGPGT KVDNK | EVQLLESGGGLVQPGGSLRLSCAASGLTFSSYAMS WVRQDPGKGLEWVSAISGSGGNTFYGDSVKGRFTI SRDNSKKTLYLQMNSLRAEDTAVYYCAKFFGIVGA GYFDYWGQGTLVTVSS |
| | | | SEQ ID NO: 25560 | SEQ ID NO: 29566 |
| iPS:434291 | 21-225_58A4 | NA | GAAATAGTGATGACGCAGTCTCCAGCCACCCT GTCTGTGTCTCCAGGGGAAAGAGCCACCCTCT CCTGCAGGGCCAGTCAGAGTGTTAGCAGCGAC TTAGCCTGGTACCAGCAGAGACCTGGCCAGGC TCCCAGGCTCCTCATCTATGCTGCATCTACCAG GGCCACTGGTATCCCAGCCAGGTTCAGTGGCA GTGGGTCTGGGACAGAGTTCACTCTCACCATC AGCAGCCTGCAGTCTGAGGATTTTGCAGTTTA TTACTGTCAGCAGTTTAATAACTGGCCATTCA CTTTCGGCCCTGGGACCAAAGTGGATATCAAA | GAGGTGCAGCTGTTGGAGTCTGGGGGAGGCTTG GTACAGCCTGGGGGGTCCCTGAGACTCTCCTGTG CAGCCTCTGGATTAACGTTTAGTAGCTACGCCAT GAGCTGGGTCCGCCAGGATCCAGGGAAGGGGCT GGAGTGGGTCTCAGCTATTAGTGGTAGTGGTGGT AACACATTCTACGGAGACTCCGTGAAGGGCCGG TTCACCATCTCCAGAGACAATTCCAAGAAAACGC TGTATCTGCAAATGAACAGCCTGAGAGCCGAGG ACACGGCCGTATATTACTGTGCGAAATTTTTCGG TATAGTGGGAGCCGGGTTCTTTGACTCCTGGGGC CAGGGAACCCTGGTCACCGTCTCCTCA |
| | | | SEQ ID NO: 25561 | SEQ ID NO: 29567 |
| | | AA | EIVMTQSPATLSVSPGERATLSCRASQSVSSDLA WYQQRPGQAPRLLIYAASTRATGIPARFSGSGSG TEFTLTISSLQSEDFAVYYCQQFNNWPFTFGPGT KVDIK | EVQLLESGGGLVQPGGSLRLSCAASGLTFSSYAMS WVRQDPGKGLEWVSAISGSGGNTFYGDSVKGRFTI SRDNSKKTLYLQMNSLRAEDTAVYYCAKFFGIVGA GFFDSWGQGTLVTVSS |
| | | | SEQ ID NO: 25562 | SEQ ID NO: 29568 |

FIGURE 50

| | | | | |
|---|---|---|---|---|
| iPS:434293 | 21-225_58F5 | NA | GACATCCAGATGACCCAGTCTCCATCCTCCCTGTCTGCATCTGTAGGGGACAGAGTCACCATCACTTGCCGGGCAAGTCAGGGCATTAGAAATGATTTAGGCTGGTTTCTGCAGACACCAGGGAAAGCCCCTAAGCGCCTGATCTATGCTGCATCCAGTTTGCTAAGTGGGGTCCCATCACGGTTCGGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGAGATCAAA | CAGGTGCAGCTGGTGGAGTCTGGGGGAGGCGTGGTCCAGCCTGGGAGGTCCCTGAGACTCTCCTGTGTAGCGTCTGGATTCACCTTCAGTGACTATGGCATGCACTGGGTCCGCCAGGCTCCAGGCAAGGGGCTGGAGTGGGTGGCAGTTATATGGTATGCTGGAAGTAATAAATACCATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGGAACTGGGGATGAGGTCTGACTACTGGGGCCAGGGAACCCTGGTCACCGTCTCCTCA |
| | | | SEQ ID NO: 25563 | SEQ ID NO: 29569 |
| | | AA | DIQMTQSPSSLSASVGDRVTITCRASQGIRNDLGWFLQTPGKAPKRLIYAASSLLSGVPSRFGGSGSGTEFTLTISSLQPEDFATYYCLQHNSYPFTFGPGTKVEIK | QVQLVESGGGVVQPGRSLRLSCVASGFTFSDYGMHWVRQAPGKGLEWVAVIWYAGSNKYHVDSVKGRFTISRDNSKNTLYLQMNSLRAEDTAVYYCARELGMRSDYWGQGTLVTVSS |
| | | | SEQ ID NO: 25564 | SEQ ID NO: 29570 |
| iPS:434295 | 21-225_58B9 | NA | GACATCGTGATGACCCAGTCTCCAGACTCCCTGGCTGTGTCTCTGGGCGAGAGGGCCACCATCAACTGCAAGTCCGGCCAGAGTATTTTATACAGCTCCAACAATAACAACTACTTAGCTTGGTACCAGCAGAAACCAGGACAGCCTCCTAAGAAACTCATTTACTGGGCATCTACCCGGGATTCCGGGGTCCCTGCCCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCTCCGACGTTCGGCCAAGGGTCCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAGCACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGTATAGCAGTGGCTGGTACTACTTTGACTACTGGGGCCAGGGAACCCTGGTCACCGTCTCCTCA |
| | | | SEQ ID NO: 25565 | SEQ ID NO: 29571 |

FIGURE 50

| | | | | |
|---|---|---|---|---|
| | | AA | DIVMTQSPDSLAVSLGERATINCKSGQSILYSSNNNNYLAWYQQKPGQPPKKLIYWASTRDSGVPARFSGSGSGTDFTLTISSLQAEDVAVYYCQQYYSTPPTFGQGSKVEIK | QVQLVQSGAEVKKPGASVKVSCKASGYTFTNYDINWVRQATGQGLEWMGWMNPNSGSTGYAQKFQGRVTMTRNTSISTAYMELSSLRSEDTAVYYCAYSSGWYYFDYWGQGTLVTVSS |
| | | | SEQ ID NO: 25566 | SEQ ID NO: 29572 |
| **iPS:434297** | **21-225_58A10** | NA | GAAATAGTGATGACGCAGTCTCCAGCCACCCTGTCTGTGTGTCCAGGGGAAAGAGCCACCCTCTCCTGCAGGGCCAGTCAGAGTGTTAGCAGCTCCTTAGCCTGGTACCAGCAGAAACCTGGCCAGGCTCCCAGGCTCCTCATCTATGGTGCATCCACCAGGGCCACTGGTATCCCAGCCAGGTTCAGTGGCAGTGGGTCTGGGACAGAATTCACTCTCACCATCAGCAGCCTGCAGTCTGAAGATTTTGCAGTTTATTACTGTCAGCAGTATAATAACTGGCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGAAGCTATGCCATGAGCTGGGTCCGCCAGGCTCCAGGGAAGGGGCTGGAGTGGGTCTCAGCTATTAGTGGTAGTGGTGGTAACACATTCTACGCAGACTCCGTGAAGGGCCGGTTCACCATCTCCAGAGACAATTCCAAGAACACGCTGTTTCTTCAAATGAACAGCCTGAGAGCCGAGGACACGGCCGTATATTACTGTGCGAAATTTTTCGGTATAGTGGGAGCCGGGTACTTTGACTACTGGGGCCAGGGAACCCTGGTCACCGTCTCCTCA |
| | | | SEQ ID NO: 25567 | SEQ ID NO: 29573 |
| | | AA | EIVMTQSPATLSVCPGERATLSCRASQSVSSSLAWYQQKPGQAPRLLIYGASTRATGIPARFSGSGSGTEFTLTISSLQSEDFAVYYCQQYNNWPFTFGPGTKVDIK | EVQLLESGGGLVQPGGSLRLSCAASGFTFRSYAMSWVRQAPGKGLEWVSAISGSGGNTFYADSVKGRFTISRDNSKNTLFLQMNSLRAEDTAVYYCAKFFGIVGAGYFDYWGQGTLVTVSS |
| | | | SEQ ID NO: 25568 | SEQ ID NO: 29574 |

FIGURE 50

| | | | | |
|---|---|---|---|---|
| **iPS:434299** | **21-225_58D11** | NA | GACATCCAGATGACCCAGTCTCCATCCTCCCTGTCTGCATCTGTAGGAGACAGAGTCACCATCACTTGCCGGGCAAGTCAGGGCATTAGAAGTGATTTAGACTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCGCAATCAGCAGCCTGCGGCCTGAAGATTTTGCAACTTATTACTGTCTCCAGCATAATAATTTCCCATTCACTTTCGGCCCTGGGACCAAGGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGACATACACTGGGTCCGCCAGTCTCCAGGCAAGGGGCTGGAGTGGGTGGCAGTTATATGGTATGATGGAAGTAAAAAATATTATGCAGACTCCGTGAAGGGCCGATTCACCATCTCCAGAGACAATTCCAAGAACACGTTGTATCTGCAAATGAACAGCCTGAGAGCCGAGGACACGGCTCTGTATTACTGTGCGAGAGATCGGGTCACTTTTGACTACTGGGGCCAGGGAACCCTGGTCACCGTCTCCTCA |
| | | | SEQ ID NO: 25569 | SEQ ID NO: 29575 |
| | | AA | DIQMTQSPSSLSASVGDRVTITCRASQGIRSDLDWYQQKPGKAPKRLIYAASSLQSGVPSRFSGSGSGTEFTLAISSLRPEDFATYYCLQHNNFPFTFGPGTKVDIK | QVQLVESGGGVVQPGRSLRLSCAASGFTFSDYDIHWVRQSPGKGLEWVAVIWYDGSKKYYADSVKGRFTISRDNSKNTLYLQMNSLRAEDTALYYCARDRVTFDYWGQGTLVTVSS |
| | | | SEQ ID NO: 25570 | SEQ ID NO: 29576 |
| **iPS:434301** | **21-225_58F11** | NA | GAAATAGTGATGACGCAGTCTCCAGCCACCCTGTCTGTGTCTCCAGGGGAAAGAGCCACCCTCTCCTGCAGGGCCAGTCAGAGTGTTAGCAGCGACTTAGTCTGGTACCAGCAGAAACCTGGCCAGGCTCCCAGGCTCCTCATCTATGGTGTATCCACCAGGGCCACTGGTATCCCAGCCAGGTTCAGTGGCAGTGGGTCTGGGACAGAGTTCACTCTCACCATCAGCAGCCTGCAGTCTGAAGATTTTGCAGTTTATTACTGTCAGCAGTATAATAACTGGCCATTCACTTTCGGCCCTGGGACCAAAGTGGATATCAAA | GAGGTGCAGTTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTAGTGGTGGTAACACATTCTACGCAGACTCCGTGAAGGGCCGGTTCACCATCTCCAGATACAATTCCAAGAACACGCTGTATCTGCAAATGAACAGCCTGAGAGCCGAGGACACGGCCGTATATTACTGTGCGAAATTTTTCGGTATGGTGGGAGCCGGATTCTTTGACTACTGGGGCCAGGGAACCCTGGTCACCGTCTCCTCA |
| | | | SEQ ID NO: 25571 | SEQ ID NO: 29577 |

FIGURE 50

| | | | | |
|---|---|---|---|---|
| | | AA | EIVMTQSPATLSVSPGERATLSCRASQSVSSDLVWYQQKPGQAPRLLIYGVSTRATGIPARFSGSGSGTEFTLTISSLQSEDFAVYYCQQYNNWPFTFGPGTKVDIK | EVQLLESGGGLVQPGGSLRLSCAASGFTFSSYAMSWVRQAPGKGLEWVSAISGSGGNTFYADSVKGRFTISRYNSKNTLYLQMNSLRAEDTAVYYCAKFFGMVGAGFFDYWGQGTLVTVSS |
| | | | SEQ ID NO: 25572 | SEQ ID NO: 29578 |
| iPS:434303 | 21-225_58H11 | NA | GATATTGTGATGACCCAGACTCCACTCTCTCTGTCCGTCACCCCTGGACAGCCGGCCTCCATCTCCTGCAAGTCTAGTCAGAGCCTCCTGCATAGTGAGGGAAAGACCTATTTGTATTGGTACGTGCAGAAGCCAGGCCAGCCTCCACAACTCCTGATCTATGAAGTTTCCTATCGGTTTTCTGGAGTGCCAGATAGGTTCAGTGGCAGCGGGTCAGGGACAGATTTCACACTGAAAATCAGCCGGGTGGAGGCTGAGGATGTTGGGGTTTATTACTGCATGCAAAGTATACAGCTTCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACCATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGCGGTATAGCAGCAGCTGGGACGGGGGTATGGACGTCTGGGGCCAAGGGACCACGGTCACCGTCTCCTCA |
| | | | SEQ ID NO: 25573 | SEQ ID NO: 29579 |
| | | AA | DIVMTQTPLSLSVTPGQPASISCKSSQSLLHSEGKTYLYWYVQKPGQPPQLLIYEVSYRFSGVPDRFSGSGSGTDFTLKISRVEAEDVGVYYCMQSIQLPLTFGGGTKVEIK | QVQLVESGGGVVQPGRSLRLSCAASGFTFSSYGMHWVRQAPGKGLEWVAVIWYDGSNKYHADSVKGRFTISRDNSKNTLYLQMNSLRAEDTAVYYCARRYSSSWDGGMDVWGQGTTVTVSS |
| | | | SEQ ID NO: 25574 | SEQ ID NO: 29580 |

FIGURE 50

| | | | | |
|---|---|---|---|---|
| **iPS:434305** | **21-225_59E1** | NA | GACATCGTGATGACCCAGTCTCCAGACTCCCT<br>GGCTGTGTCTCTGGGCGAGAGGGCCACCATCA<br>ACTGCAAGTCCAGCCAGAGTGTTTTATACAGC<br>TCCAACAATAACAACTACTTAGCTTGGTACCA<br>GCAGAGACCAGGACAGCCTCCTAAGTTGCTCA<br>TTTACTGGTCATCTACCCGGGAATCCGGGGTC<br>CCTGACCGATTCAGTGGCAGCGGGTCTGGGAC<br>AGATTTCACTCTCACCATCAGCAGCCTGCAGG<br>CTGAAGATGTGGCAGTTTATTACTGTCAGCAA<br>TATTTTAGTATTCCGTGCAGTTTTGGCCAGGGG<br>ACCAAGCTGGAGATCAAA | CAGGTGCAGCTGGTGCAGTCTGGGGCTGAGGTG<br>AAGAAGCCTGGGGCCTCAGTGAAGGTCTCCTGC<br>AAGGCTTCTGGATACACCTTCACCAATTATGATA<br>TCAACTGGGTGCGACAGGCCACTGGACAAGGGC<br>TTGAGTGGATGGGATGGATGACTCCTAACAGTGG<br>TAACACAGGCTATGCACAGAAGTTCCAGGGCAG<br>AGTCACCATGACCAGGACCACCTCCATAAGCAC<br>AGCCTACATGGAGCTGAGCAGCCTGAGATCTGA<br>GGACACGGCCGTGTATTACTGTGCGTTTAGCAGT<br>GGCTGGTACTTCTTTGACTACTGGGGCCAGGGAA<br>CCCTGGTCACCGTCTCCTCA |
| | | | SEQ ID NO: 25575 | SEQ ID NO: 29581 |
| | | AA | DIVMTQSPDSLAVSLGERATINCKSSQSVLYSSN<br>NNNYLAWYQQRPGQPPKLLIYWSSTRESGVPDR<br>FSGSGSGTDFTLTISSLQAEDVAVYYCQQYFSIPC<br>SFGQGTKLEIK | QVQLVQSGAEVKKPGASVKVSCKASGYTFTNYDIN<br>WVRQATGQGLEWMGWMTPNSGNTGYAQKFQGR<br>VTMTRTTSISTAYMELSSLRSEDTAVYYCAFSSGW<br>YFFDYWGQGTLVTVSS |
| | | | SEQ ID NO: 25576 | SEQ ID NO: 29582 |
| **iPS:434307** | **21-225_59B2** | NA | GAAATTGTGTTGACGCAGTCTCCAGGCACCCT<br>GTCTTTGTCTCCAGGGGAAAGAGCCACCCTCT<br>CCTGCTGGGCCAGTCAGAGTGTTTACAGCAGC<br>TTCTTAGCCTGGTTCCAGCAGAAATCTGGCCA<br>GGCTCCCAGGCTCCTCATCTATGGTGCATCCA<br>GCAGGGCCACTGGCATCCCAGACAGGTTCAGT<br>GGCAGTGGGTCTGGGACAGACTTCACTCTCAC<br>CATCAGCAGACTGGAGCCTGAAGATTTTGCAG<br>TGTTTTACTGTCAGCAATATGGTACCTCACCGT<br>GGACGTTCGGCCAAGGGACCAAGGTGGAAAT<br>CAAA | CAGGTGCAGTTGGTGCAGTCTGGGGCTGAGGTGA<br>AGAAGCCTGGGGCCTCAGTGAAGGTCTCCTGCA<br>AGGCTTCTGGATACACCTTCGCCGGCTACTATAT<br>ACACTGGGTGCGACAGGCCCCTGGACAAGGACT<br>TGAGTGGTTGGGTTGGATCAACCCTAACAGTGGT<br>GGCACAAACTATGCACAGAAGTTTCAGGGCAGG<br>GTCACCATGACCAGGGACACGTCCATCAGCACA<br>GCCTACATGGAGCTGAGCAGGCTGAGATTTGAC<br>GACACGGCCGTGTATTACTGTGCGAGAGATCCGG<br>GGCCCTTTGACTACTGGGGCCAGGGAACCCTGGT<br>CACCGTCTCCTCA |
| | | | SEQ ID NO: 25577 | SEQ ID NO: 29583 |

FIGURE 50

| | | | | |
|---|---|---|---|---|
| | | AA | EIVLTQSPGTLSLSPGERATLSCWASQSVYSSFLAWFQQKSGQAPRLLIYGASSRATGIPDRFSGSGSGTDFTLTISRLEPEDFAVFYCQQYGTSPWTFGQGTKVEIK | QVQLVQSGAEVKKPGASVKVSCKASGYTFAGYYIHWVRQAPGQGLEWLGWINPNSGGTNYAQKFQGRVTMTRDTSISTAYMELSRLRFDDTAVYYCARDPGPFDYWGQGTLVTVSS |
| | | | SEQ ID NO: 25578 | SEQ ID NO: 29584 |
| iPS:434309 | 21-225_59B5 | NA | GACATCCAGATGACCCAGTCTCCATCCTCCCTGTCTGCATCTGTAGGAGACAGAGTCACCATCACTTGCCGGGCAAGTCAGAGCATTATCAGCTATTTAAATTGGTATCAGCAGAAACCAGGGAAAGCCCCTAAACTCCTGATCTATGGTGCATCCAGTTTGCAGAGTGGGGTCCCATCAAGGTTCAGTGGCAGTGGATCCGGGACAGATTTCACTCTCACCATCAGCAGTCTGCAACCTGAAGATTTTGCAACTTACTACTGTCAACAGAGTTACAGTACCCCTATGTTCAGTTTTGGCCAGGGGACCAAGCTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAACTGGGTCCGCCAGGCTCCAGGGAAGGGGCTGGAGTGGGTCTCAGCTATTAGTGGTAGTGGTGGTAACACATTCTACGCAGACTCCGTGAAGGGCCGGTTCACCATTTCCAGAGACAATTCCAAGAACACGCTGTATCTGCAAATGAACAGCCTGAGAGCCGAGGACACGGCCGTATATTACTGTGCGAAAAGGGGGGTCTACGGTGACTACGAGGCTTTTGATATCTGGGGCCAAGGGACAATGGTCACCGTCTCTTCA |
| | | | SEQ ID NO: 25579 | SEQ ID NO: 29585 |
| | | AA | DIQMTQSPSSLSASVGDRVTITCRASQSIISYLNWYQQKPGKAPKLLIYGASSLQSGVPSRFSGSGSGTDFTLTISSLQPEDFATYYCQQSYSTPMFSFGQGTKLEIK | EVQLLESGGGLVQPGGSLRLSCAASGFTFSSYAMNWVRQAPGKGLEWVSAISGSGGNTFYADSVKGRFTISRDNSKNTLYLQMNSLRAEDTAVYYCAKRGVYGDYEAFDIWGQGTMVTVSS |
| | | | SEQ ID NO: 25580 | SEQ ID NO: 29586 |

FIGURE 50

| | | | | |
|---|---|---|---|---|
| **iPS:434311** | **21-225_59H5** | NA | GAAATTGTGTTGACGCAGTCTCCAGGCACCCTGTCTTTGTCTCCAGGGGAAAGAGCCACCCTCTCCTGCAGGGCCAGTCAGAGTGTTAGCAGCATCTACTTAGCCTGGTTCCTGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTGACCATCAGCAGACTGGAGCCTGAAGATTTTGCAGTGTATTACTGTCACCAGTATGGTAACTCACCATTCACTTTCGGCCCTGGGACCAAAGTGGATTTCAAA | CAGGTACAACTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGAGGGGTATAGCAGTGGGGTACTACGGTATGGACGTCTGGGGCCAAGGGACCACGGTCACCGTCTCCTCA |
| | | | SEQ ID NO: 25581 | SEQ ID NO: 29587 |
| | | AA | EIVLTQSPGTLSLSPGERATLSCRASQSVSSIYLAWFLQKPGQAPRLLIYGASSRATGIPDRFSGSGSGTDFTLTISRLEPEDFAVYYCHQYGNSPFTFGPGTKVDFK | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARERGIAVGYYGMDVWGQGTTVTVSS |
| | | | SEQ ID NO: 25582 | SEQ ID NO: 29588 |
| **iPS:434313** | **21-225_59E6** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAGGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGTAGAAGTAGTTACTACTGGGGCTGGATCCGCCAGCCCCCAGGGAAGGGGCTGGAGTGGATTGGGAATATCTATTATAGTGGGAGCACCTACTACAACCCGTCCCTCAAGAGTCGAGTCACCATATCCGTAGACACGTCCAAGAACCAGTTCTCCCTGAAGCTGAGCTCTGTGACCGCCGCAGACACGGCTCTGTATTACTGTGCGAGACATAGCAGCAGCTGGTCCCTTGACTACTGGGGCCAGGGAACCCTGGTCACCGTCTCCTCA |
| | | | SEQ ID NO: 25583 | SEQ ID NO: 29589 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHNSYPLTFGGG TRVEIK | QLQLQESGPGLVKPSETLSLTCTVSGGSISRSSYYW GWIRQPPGKGLEWIGNIYYSGSTYYNPSLKSRVTIS VDTSKNQFSLKLSSVTAADTALYYCARHSSSWSLD YWGQGTLVTVSS |
| | | | SEQ ID NO: 25584 | SEQ ID NO: 29590 |
| **iPS:434315** | **21-225_59G7** | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATTCTGCATCCAGTT TACAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATAATAGTTACCCGTGG ACGTTCGGCCAAGGGACCAAGGTGGAAATCA AA | CAGGTGCAACTAGTGCAGTCTGGGGCTGAGGTG AAGAAGCCTGGGGCCTCAGTGAAGGTCTCCTGC AAGGCTTCTGGATACAGTTTCACCGGCCACTATA TACACTGGGTGCGACAGGCCCCTGGACAAGGGC TTGAGTACATGGGATGGATCAACCCGAACAGTG GTGGCACAAACTATGTACAGAAATTTCAGGGCA GGGTCACCATGACCAGGGACACGTCCATCAGCA CAGCCTACATGGAGCTGAGCAGGCTGAGATCTG ACGACACGGCCATATATTACTGTTCGAGAGGGG GCCTACTGGGAGCTACCAACTACTACTACTACGG TATGGACGTCTGGGGCCAAGGGACCACGGTCAC CGTCACCTCA |
| | | | SEQ ID NO: 25585 | SEQ ID NO: 29591 |
| | | AA | DIQMTQSPSSLSASVGDRVTITCRASQGIRNDLG WYQQKPGKAPKRLIYSASSLQSGVPSRFSGSGSG TEFTLTISSLQPEDFATYYCLQHNSYPWTFGQGT KVEIK | QVQLVQSGAEVKKPGASVKVSCKASGYSFTGHYIH WVRQAPGQGLEYMGWINPNSGGTNYVQKFQGRV TMTRDTSISTAYMELSRLRSDDTAIYYCSRGGLLGA TNYYYYGMDVWGQGTTVTVTS |
| | | | SEQ ID NO: 25586 | SEQ ID NO: 29592 |

FIGURE 50

| | | | | |
|---|---|---|---|---|
| iPS:434317 | 21-225_59E8 | NA | GACATCCAGATGACCCAGTCTCCATCCTCCCTGTCTGCATCTGTAGGAGACAGAGTCACCATCACTTGCCGGGCAAGTCAGAGCATTAGCAGCTATTTAAATTGGTATCAGCAGAAACCAGGGAAAGCCCCTAAGCTCCTGATCTATGCTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTTCTGTCAACAGAGTTTCAGTAATTCGATCACCTTCGGCCAAGGGACACGACTGGAGATTAAA | GAGGTGCAGCTGGTGGAGTCTGGGGGAGGCTTGGTACAGCCTGGGGGGTCCCTGAGACTCTCCTGTGCAGCCTCTGGATTCACTTTCAGTAGCTATAGCATGAATTGGGTCCGCCAGGCTCCAGGGAAGGGGCTGGGGTGGGTGTCATACATTAGTAGTAGTAGTGGGACCATATACTACGCAGACTCTGTGAAGGGCCGATTCACCATCTCCAGAGACAATGCCAAGAACTCACTGTATCTGCAAATGAACAGCCTGAGAGACGAGGACACGGCTGTGTATTACTGTGCGAGAGAATGGGGAATGGCAGTGGCTGGCCCGTTTGACTACTGGGGCCAGGGAACCCTGGTCACCGTCTCCTCA |
| | | | SEQ ID NO: 25587 | SEQ ID NO: 29593 |
| | | AA | DIQMTQSPSSLSASVGDRVTITCRASQSISSYLNWYQQKPGKAPKLLIYAASSLQSGVPSRFSGSGSGTDFTLTISSLQPEDFATYFCQQSFSNSITFGQGTRLEIK | EVQLVESGGGLVQPGGSLRLSCAASGFTFSSYSMNWVRQAPGKGLGWVSYISSSSGTIYYADSVKGRFTISRDNAKNSLYLQMNSLRDEDTAVYYCAREWGMAVAGPFDYWGQGTLVTVSS |
| | | | SEQ ID NO: 25588 | SEQ ID NO: 29594 |
| iPS:434319 | 21-225_59B9 | NA | GACATCCAGATGACCCAGTCTCCATCCTCCCTGTCTGCATCTGTAGGAGACAGAGTCACCATCACTTGCCGGGCAAGTCAGGACATTAGAAATGATTTAGGCTGGTATCAGCAGCACCCAGGGAAAGCCCCTAAGCGCCTGATCTATGCTGCATCCAGTTTACAAAGTGGGGTCCCATCAAGGTTCAGCGGCACTAGATCTGGGACAGAATTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | CAGGTGCAGCTGGTGCAGTCTGGGCCTGAGGTGAAGAAGCCTGGGGCCTCAGTGAAGGTCTCCTGCAAGGCTTCTGGATACATCTTCACCGGCAATTATATACACTGGGTGCGACAGGCCCCTGGACAAGGGCTTGAGTACATGGGATGGATCAACCCTAACAGTGGTGGCACAAACTATGTACAGAAGTTTCAGGGCAGGGTCACCATGACCAGGGACACGTCCATCAGCACAGCCAACATGGAACTGACCAGTCTGAGATCTGACGACACGGCCGTGTATTACTGTTCGAGAGGGGGCCTACTGGGAGCTACCTACTACTACTACTACGGTATGGACGTCTGGGGCCAAGGGACCACGGTCACCGTCTCCTCA |
| | | | SEQ ID NO: 25589 | SEQ ID NO: 29595 |

FIGURE 50

| | | | | |
|---|---|---|---|---|
| | | AA | DIQMTQSPSSLSASVGDRVTITCRASQDIRNDLGWYQQHPGKAPKRLIYAASSLQSGVPSRFSGTRSGTEFTLTISSLQPEDFATYYCLQHNSYPWTFGQGTKVEIK | QVQLVQSGPEVKKPGASVKVSCKASGYIFTGNYIHWVRQAPGQGLEYMGWINPNSGGTNYVQKFQGRVTMTRDTSISTANMELTSLRSDDTAVYYCSRGGLLGATYYYYYGMDVWGQGTTVTVSS |
| | | | SEQ ID NO: 25590 | SEQ ID NO: 29596 |
| **iPS:434321** | **21-225_59F10** | NA | GACATCGTGATGACCCAGTTTCCAGACTCCCTGGCTGTGTCTCTGGGCGAGAGGGCCACCATCAACTGCAAGTCCAGCCAGACTGTTTTATACAGGTCCAACAATTACAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTTTAGTACTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACAAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATAACTGTGCGGTTAGCAGTGGCTGGTACTACTTTGACTACTGGGGCCAGGGAACCCTGGTCACCGTCTCCTCA |
| | | | SEQ ID NO: 25591 | SEQ ID NO: 29597 |
| | | AA | DIVMTQFPDSLAVSLGERATINCKSSQTVLYRSNNYNYLAWYQQKPGQPPKLLIYWASTRESGVPDRFSGSGSGTDFTLTISSLQAEDVAVYYCQQYFSTPPTFGQGTKVEIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYNCAVSSGWYYFDYWGQGTLVTVSS |
| | | | SEQ ID NO: 25592 | SEQ ID NO: 29598 |

FIGURE 50

| | | | | |
|---|---|---|---|---|
| **iPS:434323** | **21-225_62H8** | NA | GACATCCAGATGACCCAGTCTCCATCCTCCCTGTCTGCATCTGTAGGAGACAGAGTCACCATCACTTGCCGGGCAAGTCAGAGCATTTTCAGCTATTTAAATTGGTATCAGCAGAATCCGGGGAAAGCCCCTAAGCTCCTGATCTATGCGTCATCCAGTTTGCAAAGTGGGGTCCCATCAAGGTTAAGTGGCAATGGATCTGGGACAGATTTCATTCTCACCATCAGCAGTCTGCAACCTGAAGATTTTGCAACTTACTACTGTCAACAGAGTTACAGTACCCCATTCACTTTCGGCCCTGGGACCAGAGTGGATATCAAA | CAGGTGCAGCTGGTGGAATCTGGGGGAGGCGTGGTCCAGCCTGGGAGGTCCCTGAGACTCTCCTGTGCAGCGTCTGGATTCACCTTCAGCACCTATGGCATGCACTGGGTCCGCCAGGCTCCAGGCAAGGGGCTGGAGTGGGTGGCAGTTATATGGCATGATGGAAGTGATAAATATTATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAGACCCGCGTACCAGCTGCTCTGACTACTGGGGCCAGGGAACCCTGGTCACCGTCTCCTCA |
| | | | SEQ ID NO: 25593 | SEQ ID NO: 29599 |
| | | AA | DIQMTQSPSSLSASVGDRVTITCRASQSIFSYLNWYQQNPGKAPKLLIYASSSLQSGVPSRLSGNGSGTDFILTISSLQPEDFATYYCQQSYSTPFTFGPGTRVDIK | QVQLVESGGGVVQPGRSLRLSCAASGFTFSTYGMHWVRQAPGKGLEWVAVIWHDGSDKYYVDSVKGRFTISRDNSKNTLYLQMNSLRAEDTAVYYCAREDPRTSCSDYWGQGTLVTVSS |
| | | | SEQ ID NO: 25594 | SEQ ID NO: 29600 |
| **iPS:434327** | **21-225_63G6** | NA | GACATCCAGATGACCCAGTCTCCATCCTCCCTGTCTGCATCTGTAGGAGACAGAGTCATCATCTCTTGCCGGGCAAGTCAGAGCATTTTCAGCTATTTAAATTGGTATCAGGTGAAACCAGGGAAAGCCCCTAAGCTCCTGATCTATGATACATCCACTTTGCAAACTGGGGTCCCATCAAGGTTCAGTGGCAGTGGATCTGGGACAGATTTCACTCTCACCATCAACAGTCTGCAACCTGAAGATTTTGCAACTTACTACTGTCAACAGAGTTACGGTATCCCCATCACCTTCGGCCAAGGGACACGACTGGAGATTCAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTAGAATGGGTGACAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATAGCCTCTCGGGTATAGCAGCAGCTTTTGACTACTGGGGCCAGGGAACCCTGGTCACCGTCTCCTCA |
| | | | SEQ ID NO: 25595 | SEQ ID NO: 29601 |

FIGURE 50

| | | | | |
|---|---|---|---|---|
| | | AA | DIQMTQSPSSLSASVGDRVIISCRASQSIFSYLNWYQVKPGKAPKLLIYDTSTLQTGVPSRFSGSGSGTDFTLTINSLQPEDFATYYCQQSYGIPITFGQGTRLEIQ | QVQLVESGGGVVQPGRSLRLSCAASGFTFSSYGMHWVRQAPGKGLEWVTVIWYDGSNKYYADSVKGRFTISRDNSKNTLYLQMNSLRAEDTAVYYCARDSLSGIAAAFDYWGQGTLVTVSS |
| | | | SEQ ID NO: 25596 | SEQ ID NO: 29602 |
| **iPS:434331** | **21-225_63H8** | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAACCAGGGAAAGCCCATAAGTCCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAATATCATAGTTACCCATTCACTTTCGGCCCTGGGACCAAAGTGGATATCAGA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAACATGAACTGGGTCCGCCAGGCTCCAGGGAAGGGGCTGGAGTGGGTCTCATCCATTAGTGGTAGTAGCACTTACATGAACTACACAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGACTACGTAATTTTGACTACTGGGGCCAGGGAACCCTGGTCACCGTCTCCTCA |
| | | | SEQ ID NO: 25597 | SEQ ID NO: 29603 |
| | | AA | DIQMTQSPSSLSASVGDRVTITCRASQGISNYLAWFQQKPGKAHKSLIYAASSLQSGVPSKFSGSGSGTDFTLTISSLQPEDFATYYCQQYHSYPFTFGPGTKVDIR | EVQLVESGGGLVKPGGSLRLSCAASGFTFSSYNMNWVRQAPGKGLEWVSSISGSSTYMNYTDSVKGRFTISRDNAKNSLYLQMNSLRAEDTAVYYCARLRNFDYWGQGTLVTVSS |
| | | | SEQ ID NO: 25598 | SEQ ID NO: 29604 |

FIGURE 50

| | | | | |
|---|---|---|---|---|
| **iPS:434333** | **21-225_63C9** | NA | GACATCCAGATGACCCAGTCTCCATCTTCCGTGTCTGCATCTGTCGGAGACAGAGTCACCATCACTTGTCGGGCGAGTCAGGGTATTAGCAGCTGGTTAGCCTGGTATCAGCAGAAACCAGGGAAAGCCCCTAACCTCCTGATCTATGCTGCATCCAGTTTGCAAAGTGGGGTCCCATCAAGGTTCAGCGGCAGTGGATCTGGGACAGATTTCACGCTCACCATCAGCGGCCTGCAGCCTGAAGATTTTGCAACTTACTTTTGTCAACAGATTAACAGTTTCCCTCTCACTTTCGGCGGAGGGACCAAGGTGGCGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAACAGTGGTGGCACAAACTTTGCACAGAAGTTTCAGGGCAGGGTCACCATGACCAGGGACGCGTCCATCAACACAGCCTACATGGAGCTGCGCAGCCTGATATCTGACGACACGGCCGTATATTACTGTGCGAGAGCTCCGGGTGTAGCAGCAGCTGGTTCATGGGGATACTTTGACTACTGGGGCCAGGGAACCCTGGTCACCGTCTCCTCA |
| | | | SEQ ID NO: 25599 | SEQ ID NO: 29605 |
| | | AA | DIQMTQSPSSVSASVGDRVTITCRASQGISSWLAWYQQKPGKAPNLLIYAASSLQSGVPSRFSGSGSGTDFTLTISGLQPEDFATYFCQQINSFPLTFGGGTKVAIK | QVQLVQSGAEVKKPGASVKVSCKASGYTFTGYYMHWVRQAPGQGLEWMGWINPNSGGTNFAQKFQGRVTMTRDASINTAYMELRSLISDDTAVYYCARAPGVAAAGSWGYFDYWGQGTLVTVSS |
| | | | SEQ ID NO: 25600 | SEQ ID NO: 29606 |
| **iPS:434335** | **21-225_63C10** | NA | GACATCCAGATGACCCAGTCTCCGTCCTCCCTGTCTACATCTGTAGGAGACAGAGTCACCATCACTTGCCGGGCAAGTCAGAGCATTTTCAGCTATTTACATTGGTATCAGCAGAAACCAGGGAAAGCCCCTAAGCTCCTGATCTCTGCTGCATCCAGTTTACAAAGTGGGGTCCCATCAAGGTTCAGTGGCAGTGGATCTGGGACAGATTTCACTCTCACCATCAGTAGTCTGCAACCTGAAGATTTTGCAACTTTCTACTGTCAACAGACTTACAGTCCCCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGACTGGGTGGCAGTCATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATGATCCCAGATCCTCGGCCGGGGACTACTGGGGCCAGGGAACCCTGGTCACCGTCTCCTCA |
| | | | SEQ ID NO: 25601 | SEQ ID NO: 29607 |

FIGURE 50

| | | | | |
|---|---|---|---|---|
| | | AA | DIQMTQSPSSLSTSVGDRVTITCRASQSIFSYLHWYQQKPGKAPKLLISAASSLQSGVPSRFSGSGSGTDFTLTISSLQPEDFATFYCQQTYSPPLTFGGGTKVEIK | QVQLVESGGGVVQPGRSLRLSCAASGFTFSSYGMHWVRQAPGKGLDWVAVIWYDGSNKYYADSVKGRFTISRDNSKNTLYLQMNSLRAEDTAVYYCARDDPRSSAGDYWGQGTLVTVSS |
| | | | SEQ ID NO: 25602 | SEQ ID NO: 29608 |
| iPS:434337 | 21-225_64E1 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCACGGTTCAGCGGCAGTAAATCTGGGACAGAATTCACTCTCACAATCAGCAGCTTGCAGCCTGAAGATCTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGTAGCGTCTGGATTCACCTTCAGTAGCTATGGCATGCACTGGGTCCGCCAGGCTCCAGGCAAGGGGCTGGAGTGGGTGGCAGTTATATGGTTTGATGAAACTAATAAATACTATGGAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGCTTGGGTTCAGCAGTGACTATTGGGGCCAGGGAACCCTGGTCACCGTCTCCTCA |
| | | | SEQ ID NO: 25603 | SEQ ID NO: 29609 |
| | | AA | DIQMTQSPSSLSASVGDRVTITCRASQGIRNDLGWYQQKPGKAPKRLIYAASSLQSGVPSRFSGSKSGTEFTLTISSLQPEDLATYYCLQHNSYPLTFGGGTKVEIK | QVQLVESGGGVVQPGRSLRLSCVASGFTFSSYGMHWVRQAPGKGLEWVAVIWFDETNKYYGDSVKGRFTISRDNSKNTLYLQMNSLRAEDTAVYYCARELGFSSDYWGQGTLVTVSS |
| | | | SEQ ID NO: 25604 | SEQ ID NO: 29610 |

FIGURE 50

| | | | | |
|---|---|---|---|---|
| **iPS:434339** | **21-225_64A4** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAACGCCTGATCTATGCTGCATCCAGTTTGCAAAGTGGGGTCCCATCAAGGTTCAGCGGCAGTGGATCTGGGACAGAATTCACTCTCACAATCAGCAGCCTACAGCCTGAAGATCTTGCAACTTATTACTGTCTACAGCATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGCGATTATGTCATGCACTGGGTCCGCCAGGCTCCAGGCAAGGGGCTGGAGTGGGTGGCAGTCATATGGTATGATGGAAGTAATAAATACTATGCAGACTCCGTGAAGGGCCGATTCACCATCTCCAGAGACAATTCCCAGAACACGCTGTATCTGCAAATGAATAGCCTGAGAGCCGAGGACACGGCTGTGTATTACTGTGCGAGAGAAAGGTATAGCAGCAGCTGGTACGACTACGGTATGGACGTCTGGGGCCAAGGGACCACGGTCACCGTCTCCTCA |
| | | | SEQ ID NO: 25605 | SEQ ID NO: 29611 |
| | | AA | DIQMTQSPSSLSASVGDRVTITCRASQGIRNDLGWYQQKPGKAPKRLIYAASSLQSGVPSRFSGSGSGTEFTLTISSLQPEDLATYYCLQHYSYPRTFGQGTKVEIK | QVQLVESGGGVVQPGRSLRLSCAASGFTFSDYVMHWVRQAPGKGLEWVAVIWYDGSNKYYADSVKGRFTISRDNSQNTLYLQMNSLRAEDTAVYYCARERYSSSWYDYGMDVWGQGTTVTVSS |
| | | | SEQ ID NO: 25606 | SEQ ID NO: 29612 |
| **iPS:434341** | **21-225_64F7** | NA | GACATCCAGATGACCCAGTCTCCATCCTCCCTGTCTGCATCTGTAGGAGACAGAGTCACCATCACTTGCCGGGCAAGTCAGAACATTAAGAAATATTTAAATTGGTATCAGCAGAAACCAGGGAAAGCCCCTAAGTTCCTGATCTATGGTGCATCCAGTTTGCAAAGTGGGGTCCCATCAAGGTTCAGTGGCAGTGGATCTGGGACAGATTTCACTCTCACCATCAGTAGTCTGCAACCTGAAGATTTTGCAGCTTACTACTGTCAACAGAGTTACAATATTTCGTTCACTTTCGGCGGAGGGACCAAGGTGGAGCTCAAA | CAGGTGCAGCTGCAGGAGTCGGGCCCAGGACTGGTGAAGCCTTCGGAGACCCTGTCCCTCACCTGCACTGTCTCTGGTGGCTCCATCAGTAGTTACTTCTGGAGCTGGATCCGGCAGCCCGCCGGGAAGGGACTGGAGTGGATTGGGCGTATCTATACCAGTGGGATCTCCAACTACAATCCCTCCCTCAAGAGTCGAGTCACCATGTCAGTTGACACGTCCAAGAACCAGTTCTCCCTGAAGCTGAGCTCTGTGACCGCCGCGGACACGGCCGTGTATTACTGTGCGAGGTTTAGCAGTGGCTTTTTTGACTACTGGGGCCAGGGTACCCTGGTCACCGTCTCCTCA |
| | | | SEQ ID NO: 25607 | SEQ ID NO: 29613 |

FIGURE 50

| | | | | |
|---|---|---|---|---|
| | | AA | DIQMTQSPSSLSASVGDRVTITCRASQNIKKYLNWYQQKPGKAPKFLIYGASSLQSGVPSRFSGSGSGTDFTLTISSLQPEDFAAYYCQQSYNISFTFGGGTKVELK | QVQLQESGPGLVKPSETLSLTCTVSGGSISSYFWSWIRQPAGKGLEWIGRIYTSGISNYNPSLKSRVTMSVDTSKNQFSLKLSSVTAADTAVYYCARFSSGFFDYWGQGTLVTVSS |
| | | | SEQ ID NO: 25608 | SEQ ID NO: 29614 |
| iPS:434343 | 21-225_64C8 | NA | GACATCCAGATGACCCAGTCTCCATCCTCCCTGTCTGCAGCTGTAGGAGACAGAGTCACCATCACTTGCCGGGCAAGTCAGGGCATTAGAAATGATTTAGGCTGGTATCAGCAGAAACCAGGGAAAGCCCCTAAGTGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTATTGTCTACACCATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGTCATGCACTGGGTCCGCCAGGCTCCAGGCAAGGGGCTGGAGTGGGTGGCAGTTATATGGTATGATGGAAGTAATAAATACTATGCAGACTCCATGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACGGTATAGCAGTGGCTGGTACGACTACGGTATGGACGTCTGGGGCCAAGGGACCACGGTCACCGTCTCCTCA |
| | | | SEQ ID NO: 25609 | SEQ ID NO: 29615 |
| | | AA | DIQMTQSPSSLSAAVGDRVTITCRASQGIRNDLGWYQQKPGKAPKCLIYAASSLQSGVPSRFSGSGSGTEFTLTISSLQPEDFATYYCLHHYSYPRTFGQGTKVEIK | QVQLVESGGGVVQPGRSLRLSCAASGFTFSDYVMHWVRQAPGKGLEWVAVIWYDGSNKYYADSMKGRFTISRDNSKNTLYLQMNSLRAEDTAVYYCARERYSSGWYDYGMDVWGQGTTVTVSS |
| | | | SEQ ID NO: 25610 | SEQ ID NO: 29616 |

FIGURE 50

| | | | | |
|---|---|---|---|---|
| **iPS:434345** | **21-225_64H9** | NA | GAAATTGTGATGACCCAGACTCCACTCTCTCTGTCCGTCACCCCTGGACAGCCGGCCTCCATCTCCTGCAAGTCTAGTCAGAGCCTCCTTCATGGTGATGGAAAGACCTATTTGTTTTGGTACCTGCAGAAGCCAGGCCAGCCTCCACAGGTCCTGATCTATGAAGTTTCCAACCGGTTGTGTGGAGTGCCAGACAGGTTCAGTGGCAGCGGGTCAGGGACAGATTTCTCATTGAAAATCAGCCGGGTGGAGGCTGAGGACGTTGGGGTTTATTACTGCATGCAAAGTATACAGGTTCCGTGGACGTTCGGCCAAGGGACCAAGGTGGAAATCACA | CAGGTGCAGCTGGTGGAGTCTGGGGGAGGCGTGGTCCAGCCTGGGAGGTCCCTGAGACTCTCCTGTGCAGCGTCTGGATTCACCTTCAGTACCTATGGCATGCACTGGGTCCGCCAGGCTCCAGGCAAGGGGCTGGAGTGGGTGGCAATTATATGGTATGATGGAAGTAATAAATACTATGCAGACTCCGTGAAGGGCCGATTCACCATCTCCAGAGACAATTCCAAGAACACGCTATATCTACAAATGAACAGCCTGAGAGCCGAGGACACGGCTGTGTATTACTGTGCGAGAGATACATACGATTTTTGGAGTGGTTATTTGGGCTACTGGGGCCAGGGAACCCTGGTCACCGTCTCCTCA |
| | | | SEQ ID NO: 25611 | SEQ ID NO: 29617 |
| | | AA | EIVMTQTPLSLSVTPGQPASISCKSSQSLLHGDGKTYLFWYLQKPGQPPQVLIYEVSNRLCGVPDRFSGSGSGTDFSLKISRVEAEDVGVYYCMQSIQVPWTFGQGTKVEIT | QVQLVESGGGVVQPGRSLRLSCAASGFTFSTYGMHWVRQAPGKGLEWVAIIWYDGSNKYYADSVKGRFTISRDNSKNTLYLQMNSLRAEDTAVYYCARDTYDFWSGYLGYWGQGTLVTVSS |
| | | | SEQ ID NO: 25612 | SEQ ID NO: 29618 |
| **iPS:434347** | **21-225_64H10** | NA | GACATCCAGATGACCCAGTCTCCATCTTCCGTGTCTGCATCTGTAGGAGACAGAGTCACCATCACTTGTCGGGCGAGTCAGGGTATTAGCAGCTGGTTAGCCTGGTATCAGCAGAAACCAGGGAAAGCCCTTAAGCTCTTGATCTATGCTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGATTAACAGTTTCCCTCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAAACCAAACAGTGGTGGCACAAACCAAGCACAGAAGTTTCAGGGCAGGGTCACCATGACCAGGGACACGTCCATCAGCACAGCCTACATGGAGCTGAGCGGGCTGAGATCTGACGACACGGCCGTGTATTACTGTGCGAGAGCTCCGGGTACTGCAGCAACTGGTACATGGGGATACTTTGACTACTGGGGCCAGGGAACCCTGGTCACCGTCTCCTCA |
| | | | SEQ ID NO: 25613 | SEQ ID NO: 29619 |

FIGURE 50

| | | | | |
|---|---|---|---|---|
| | | AA | DIQMTQSPSSVSASVGDRVTITCRASQGISSWLAWYQQKPGKALKLLIYAASSLQSGVPSRFSGSGSGTDFTLTISSLQPEDFATYYCQQINSFPLTFGGGTKVEIK | QVQLVQSGAEVKKPGASVKVSCKASGYTFTGYYMHWVRQAPGQGLEWMGWIKPNSGGTNQAQKFQGRVTMTRDTSISTAYMELSGLRSDDTAVYYCARAPGTAATGTWGYFDYWGQGTLVTVSS |
| | | | SEQ ID NO: 25614 | SEQ ID NO: 29620 |
| iPS:434351 | 21-225_64A12 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTACACGCCTGATCTATACTGCATCCACTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATGGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGAAAGTAATAAATACTATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGGAACTCGGGTTCCTCTCTGACCACTGGGGCCAGGGAACCCTGGTCACCGTCTCCTCA |
| | | | SEQ ID NO: 25615 | SEQ ID NO: 29621 |
| | | AA | DIQMTQSPSSLSASVGDRVTITCRASQGIRNDLGWYQQKPGKAPTRLIYTASTLQSGVPSRFSGSGSGTEFTLTISSLQPEDFATYYCLQHNGYPFTFGPGTKVDIK | QVQLVESGGGVVQPGRSLRLSCAASGFTFSSYGMHWVRQAPGKGLEWVAVIWYDESNKYYVDSVKGRFTISRDNSKNTLYLQMNSLRAEDTAVYYCARELGFLSDHWGQGTLVTVSS |
| | | | SEQ ID NO: 25616 | SEQ ID NO: 29622 |

FIGURE 50

| | | | | |
|---|---|---|---|---|
| **iPS:434353** | **21-225_64B12** | NA | GACATCCAGATGACCCAGTCTCCATCCTCCCTGTCTGCATCTGTAGGAGACAGAGTCACCATCACTTGCCGGGCGAGTCAGGACATTAGCAATTATTTAAATTGGTATCAGCAGAAACCAGGGACAGCCCCTAACCTCCTGATCTCTGATGCATCCATTTTGGAAACAGGGGTCCCATCAACGTTCAGTGGAAGTGGATCTGGGACAGATTTTACTTTCACCATCAGCAGCCTGCAGCCTGAAGATATTGCAACATATTACTGTCAACAGAGTGATAATCTCCCGTGCAGTTTTGGCCAGGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGTCTCCATCAGCAGAAGTAGTTACTACTGGGGCTGGATCCGCCAGCCCCCAGGGAAGGGGCTGGAGTGGATTGGGAGTATCTATTACAGTGGGAGCACCTCCTACAACCCGTCCCTCAAGAGTCGAGTCACCATATCCGTAGACACGTCCAAGAACCAGTTCTCCCTGAAGCTGAGCTCTGTGACCGCCGCAGACACGGCTGTGTATTATTGTGCGAGACTGGACAGTGGCTGGTCCTTTGACTACTGGGGCCAGGGAACCCTGGTCACCGTCTCCTCA |
| | | | SEQ ID NO: 25617 | SEQ ID NO: 29623 |
| | | AA | DIQMTQSPSSLSASVGDRVTITCRASQDISNYLNWYQQKPGTAPNLLISDASILETGVPSTFSGSGSGTDFTFTISSLQPEDIATYYCQQSDNLPCSFGQGTKVEIK | QLQLQESGPGLVKPSETLSLTCTVSGVSISRSSYYWGWIRQPPGKGLEWIGSIYYSGSTSYNPSLKSRVTISVDTSKNQFSLKLSSVTAADTAVYYCARLDSGWSFDYWGQGTLVTVSS |
| | | | SEQ ID NO: 25618 | SEQ ID NO: 29624 |
| **iPS:434355** | **21-225_64G12** | NA | GACATCCAGATGACCCAGTCTCCCTCTTCCGTGTCTGCATCTGTAGGAGACAGAGTCACCATCACTTGTCGGGCGAGTCAGAATATTACCACCTGGTTAGCCTGGTATCAGCAGAAACCAGGGAAAGCCCCTAAACTCCTGATCTCTGCTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTATATTTGTCAACAGGCTAACAGTTTTCCATTCACTTTCGGCCCTGGGACCAAACTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGTAATGCCATGAGCTGGGTCCGCCAGGCTCCAGGGAAGGGGCTGGAGTGGGTCTCAGTTATTAGTGGTAGTGGTGGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCTTATATTACTGTGCGAAAAGGAACTACGACGATGCTTTTGATATCTGGGGCCAAGGGACAATGGTCACCGTCTCTTCA |
| | | | SEQ ID NO: 25619 | SEQ ID NO: 29625 |

FIGURE 50

| | | | | |
|---|---|---|---|---|
| | | AA | DIQMTQSPSSVSASVGDRVTITCRASQNITTWLAWYQQKPGKAPKLLISAASSLQSGVPSRFSGSGSGTDFTLTISSLQPEDFATYICQQANSFPFTFGPGTKLDIK | EVQLLESGGGLVQPGGSLRLSCAASGFTFSSNAMSWVRQAPGKGLEWVSVISGSGGSTYYADSVKGRFTISRDNSKNTLYLQMNSLRAEDTALYYCAKRNYDDAFDIWGQGTMVTVSS |
| | | | SEQ ID NO: 25620 | SEQ ID NO: 29626 |
| **iPS:434357** | **21-225_65C1** | NA | GACATCCAGTTGACCCAGTCTCCATCCTCCCTGTCTGCATCTGTAGGAGACAGAGTCACCATCACTTGCCGGGCGAGTCAGGTCATTAGCAGTTATTTACATTGGTATCAGCAGAAACCAGGGAAAGTTCCTAAGGTCCTGATCTATAGTGCATCCAATTTGCAATGTGGAGTCCCATCTCGGTTCAGCGGCAGTGGATCTGGGACAGATTTCACTCTCACCTTCAGCAGCCTGCAGCCTGAAGATGTTGCAACTTATTACGGTCAACGGCCTTACAATGCCCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTGATATGGTTTGAGGGAAGTAATAAACACTATACAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTTGGGTTCAGCAGTGACTACTGGGGCCAGGGAACCCTGGTCACCGTCTCCTCA |
| | | | SEQ ID NO: 25621 | SEQ ID NO: 29627 |
| | | AA | DIQLTQSPSSLSASVGDRVTITCRASQVISSYLHWYQQKPGKVPKVLIYSASNLQCGVPSRFSGSGSGTDFTLTFSSLQPEDVATYYGQRPYNAPLTFGGGTKVEIK | QVQLVESGGGVVQPGRSLRLSCAASGFTFSDYGMHWVRQAPGKGLEWVAVIWFEGSNKHYTDSVKGRFTISRDNSKNTLYLQMNSLRAEDTAVYYCARELGFSSDYWGQGTLVTVSS |
| | | | SEQ ID NO: 25622 | SEQ ID NO: 29628 |

FIGURE 50

| | | | | |
|---|---|---|---|---|
| **iPS:434359** | **21-225_65G3** | NA | GACATCCAGATGACCCAGTCTCCATCCTCCGTGTCTGCATCTGTAGGAGACAGAGTCACCATCACTTGTCGGGCGAGTCAGGGTATTAGCAGCTGGTTAGCCTGGTATCAGCAGAAACCAGGGAAAGCCCCTAAGCTCTTGATCTATGCTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTTCTATTGTCAACAGGTTAACAGTTTCCCTCTCACTTTCGGCGGAGGGACCAAGGTGGAGATCAAA | CAGGTGCAACTGGTGCAGTCTGGGGCTGAGGTGAAGAAGCCTGGGGCCTCAGTGAAGGTCTCCTGCAAGGCTTCTGGATACACCTTCACCGGCTACTATATACACTGGGTGCGACAGGCCCCTGGACAAGGGCTTGAGTGGATGGGATGGATCAACCCTAACAGTGGTGGCACAAACTCTGCACAGAAGTTTCAGGGCAGGGTCACCATGACCAGGGACACGTCCATCAGCACAGCCTACATGGAGCTGAGCAGGCTGAGATCTGACGACACGGCCGTATATTACTGTGCGAGAGCTCCGGGTAAAGCAGCAGCTGGTACATGGGGATACTTTGACTACTGGGGCCAGGGAACCCTGGTCACCGTCTCCTCA |
| | | | SEQ ID NO: 25623 | SEQ ID NO: 29629 |
| | | AA | DIQMTQSPSSVSASVGDRVTITCRASQGISSWLAWYQQKPGKAPKLLIYAASSLQSGVPSRFSGSGSGTDFTLTISSLQPEDFATFYCQQVNSFPLTFGGGTKVEIK | QVQLVQSGAEVKKPGASVKVSCKASGYTFTGYYIHWVRQAPGQGLEWMGWINPNSGGTNSAQKFQGRVTMTRDTSISTAYMELSRLRSDDTAVYYCARAPGKAAAGTWGYFDYWGQGTLVTVSS |
| | | | SEQ ID NO: 25624 | SEQ ID NO: 29630 |
| **iPS:434361** | **21-225_65D5** | NA | GACATCCAGATGACCCAGTCTCCGTCCTCACTATCTGCATCTGTAGGAGACAGAGTCACCATCACTTGTCGGGCGAGTCAGGACATTAACAATTATTTAGCCTGGTTTCAGCAGAAACCAGGGAAAGCCCCTAGGTCCCTAATTTATGCTGCATCCAGTTTGCACAGTGGGGTCCCATCACAGTTCAGCGCCAGTGGTTCTGGGTCAGATTTCACTCTTACTATCAGCAGCCTGCAGCCTGAAGATTTTGCAACTTATTACTGCCCACTGTATAAAAGTTATCCACTCACTTTTGGCCCTGGGACCAAAGTGGATATCAAA | CAGGTTCAGCTGGTGCAGTCTGGAGCTGAGGTGAAGAAGCCTGGGGCCTCAGTGAAGGTCTCCTGCAAGGCTTCTGGTTACACCTTTCCCAGCTATGGTATCAGTTGGGTGCGACAGGCCCCTGGACAAGGACTTGAGTGGATGGGATGGATCAGCGCTTACAGTGGTAACACAAACTATGCACAGAAGCTCCAGGGCAGAGTCACCATGACCACAGACACATCCACGAGCACAGCCTACATGGAGCTGAGGAGCCTGAGATCTGACGACACGGCCGTGTATTTCTGTGCGAGAGGGGAAGCAGTGGCTGTCTTCGACCCCTGGGGCCAGGGAACCCTGGTCACCGTCTCCTCA |
| | | | SEQ ID NO: 25625 | SEQ ID NO: 29631 |

FIGURE 50

| | | | | |
|---|---|---|---|---|
| | | AA | DIQMTQSPSSLSASVGDRVTITCRASQDINNYLAWFQQKPGKAPRSLIYAASSLHSGVPSQFSASGSGSDFTLTISSLQPEDFATYYCPLYKSYPLTFGPGTKVDIK | QVQLVQSGAEVKKPGASVKVSCKASGYTFPSYGISWVRQAPGQGLEWMGWISAYSGNTNYAQKLQGRVTMTTDTSTSTAYMELRSLRSDDTAVYFCARGEAVAVFDPWGQGTLVTVSS |
| | | | SEQ ID NO: 25626 | SEQ ID NO: 29632 |
| iPS:434363 | 21-225_65A6 | NA | GAAATAGTGATGACGCAGTCTCCAGTCACCCTGTTTGTGTCTCCAGGGGAAAGAGCCACCCTCTCCTGCAGGGCCAGTCAGAGTGTTAACAGCAACTTAGCCTGGTACCAGCAGAAACCTGGCCAGGCTCCCAGGCTCCTCATCTATGGTGCATCCACCAGGGCCACTGGTATCCCAGCCAGGTTCAATGGCAGTGGGTCTGGGACAGAGTTCACTCTCACCATCAGCAGCCTGCAGTCTGAAGATTTTGCAGTTTATTATTGTCAGCAGTATAATGACTGGCCGTGCAGTTTTGGCCTGGAGACCAAGCTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGATGGAAGTAATAAATACTATGGAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCAGGGCATAGTGGGAGCTACTTGGTTTGACTACTGGGGCCAGGGAACCCTGGTCACCGTCTCCTCA |
| | | | SEQ ID NO: 25627 | SEQ ID NO: 29633 |
| | | AA | EIVMTQSPVTLFVSPGERATLSCRASQSVNSNLAWYQQKPGQAPRLLIYGASTRATGIPARFNGSGSGTEFTLTISSLQSEDFAVYYCQQYNDWPCSFGLETKLEIK | QVQLVESGGGVVQPGRSLRLSCAASGFTFSNYGMHWVRQAPGKGLEWVAVIWYDGSNKYYGDSVKGRFTISRDNSKNTLYLQMNSLRAEDTAVYYCARDQGIVGATWFDYWGQGTLVTVSS |
| | | | SEQ ID NO: 25628 | SEQ ID NO: 29634 |

FIGURE 50

| | | | | |
|---|---|---|---|---|
| **iPS:434367** | **21-225_65H11** | NA | GACATCCAGATGACCCAGTCTCCATCCTCTCT<br>GTCTGCATCTGTAGGAGACAGAGTCACCATCA<br>CTTGTCGGGCGAGTCAGGACATTAGCACTTAT<br>TTAGCCTGGTTTCAGCAGAAACCAGGGAAAGC<br>CCCTAAGTCACTGATCTATGCTGCATCCAGTTT<br>GCAAAGTGGGGTCCCATCAAAGTTCAGCGGCA<br>GTGGATCTGGGACAGATTTCACTCTCACCATC<br>AGTAGCCTGCAGCCTGAAGATTTTGCAACTTA<br>TTACTGCCAACAGTATAATAGTTTCCCTCTCAC<br>TTTCGGCGGAGGGACCAAGGTGGAGATCAAA | GAGGTGCAGCTGGTGGAGTCTGGGGGAGGCCTG<br>GTCAAGCCGGGGGGGTCCCTGAGACTCTCCTGTG<br>CAGCCTCTGGATTCACCTTCAGTAGCTATAGGAT<br>GAACTGGGTCCGCCAGGCTCCAGGGAAGGGGCT<br>GGAGTGGGTCTCATCCATTAGTAGTAGTAATAGT<br>TCCATATACTACGCAGACTCAGTGAAGGGCCGAT<br>TCACCACCTCCAGAGACAACGCCAAGAACTCACT<br>GTATCTGCAAATGAACAGCCTGAGAGCCGAGGA<br>CACGGCTGTGTATTACTGTACGAGTACAAGTGGG<br>AGCTGGGGCCAGGGAACCCTGGTCACCGTCTCCT<br>CA |
| | | | SEQ ID NO: 25629 | SEQ ID NO: 29635 |
| | | AA | DIQMTQSPSSLSASVGDRVTITCRASQDISTYLA<br>WFQQKPGKAPKSLIYAASSLQSGVPSKFSGSGSG<br>TDFTLTISSLQPEDFATYYCQQYNSFPLTFGGGT<br>KVEIK | EVQLVESGGGLVKPGGSLRLSCAASGFTFSSYRMN<br>WVRQAPGKGLEWVSSISSSNSSIYYADSVKGRFTTS<br>RDNAKNSLYLQMNSLRAEDTAVYYCTSTSGSWGQ<br>GTLVTVSS |
| | | | SEQ ID NO: 25630 | SEQ ID NO: 29636 |
| **iPS:434369** | **21-225_66B1** | NA | GACATCCAGATGACCCAGTCTCCATCTTCCGT<br>GTCTGCATCTGTAGGAGACAGAGTCACCATCA<br>CTTGTCGGGCGAGTCAGGGTATTAGCAGCTGG<br>TTAGCCTGGTATCAGCAGAAACCAGGGAAAG<br>CCCTTAAGCTCTTGATCTATGCTGCATCCAGTT<br>TGCAAAGTGGGGTCCCATCAAGGTTCAGCGGC<br>AGTGGATCTGGGACAGATTTCACTCTCACCAT<br>CAGCAGCCTGCAGCCTGAAGATTTTGCAACTT<br>ACTATTGTCAACAGACTAACAGTTTCCCTCTC<br>ACTTTCGGCGGAGGGACCAAGGTGGAGATCA<br>AA | CAGGTGCAGCTGGTGCAGTCTGGGGCTGAGGTG<br>AAGAAGCCTGGGGCCTCAGTGAAGGTCTCCTGC<br>AAGGCTTCTGGATACACCTTCACCGGCTACTATA<br>TGCACTGGGTGCGACAGGCCCCTGGACAAGGGC<br>TTGAGTGGATGGGATGGATCAACCCTAACAGTGG<br>TGGCACAAACAATGCACAGAAGTTTCAGGGCAG<br>GGTCACCATGACCAGGGCCACGTCCATCAGCAC<br>AGCCTACATGGAACTGAGCAGGCTGAGATCTGA<br>CGACACGGCCGTGTATTATTGTGCGAGAGCTCCG<br>GGTACAGCAGCAGCTGGTACATGGGGATACTTTG<br>ACTACTGGGGCCAGGGAACCCTGGTCACCGTCTC<br>CTCA |
| | | | SEQ ID NO: 25631 | SEQ ID NO: 29637 |

FIGURE 50

| | | | | |
|---|---|---|---|---|
| | | AA | DIQMTQSPSSVSASVGDRVTITCRASQGISSWLAWYQQKPGKALKLLIYAASSLQSGVPSRFSGSGSGTDFTLTISSLQPEDFATYYCQQTNSFPLTFGGGTKVEIK | QVQLVQSGAEVKKPGASVKVSCKASGYTFTGYYMHWVRQAPGQGLEWMGWINPNSGGTNNAQKFQGRVTMTRATSISTAYMELSRLRSDDTAVYYCARAPGTAAAGTWGYFDYWGQGTLVTVSS |
| | | | SEQ ID NO: 25632 | SEQ ID NO: 29638 |
| iPS:434373 | 21-225_66A7 | NA | GACATCCAGATGACCCAGTCTCCATCTTCCGTGTCTGCATCTGTAGGGGACAGAGTCACCATCACTTGTCGGGCGAGTCAGGGTATTAGCAGCTGGTTAGCCTGGTATCAGCAGAAACCAGGGAAAGCCCCTAAGCTCTTGATCTATGCTGCATCCAGTTTGCAAAGTGGGGTCCCTTCAAGGTTCAGCGGCAGTGGATCTGGGACAGATTTCACTCTCACCATCAGCAGCCTGCAGCCTGAAGATTTTGCAACTTACTATTGTCAACAGATTAATAGTTTCCCTCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAAACCAAACAGTGGTGGCACAAACCAAGCACAGAAGTTCCAGGGCAGGGTCACCATGACCAGGGACACGTCCATCAGCACAGGTTACATGGAGCTGAGCAGGCTGAGATCTGACGACACGGCCGTGTATTACTGTGCGAGAGCTCCGGGCACAGTAGCAGCTGGTACATGGGGATACTTTGACTATTGGGGCCAGGGAACCCTGGTCACCGTCTCCTCA |
| | | | SEQ ID NO: 25633 | SEQ ID NO: 29639 |
| | | AA | DIQMTQSPSSVSASVGDRVTITCRASQGISSWLAWYQQKPGKAPKLLIYAASSLQSGVPSRFSGSGSGTDFTLTISSLQPEDFATYYCQQINSFPLTFGGGTKVEIK | QVQLVQSGAEVKKPGASVKVSCKASGYTFTGYYMHWVRQAPGQGLEWMGWIKPNSGGTNQAQKFQGRVTMTRDTSISTGYMELSRLRSDDTAVYYCARAPGTVAAGTWGYFDYWGQGTLVTVSS |
| | | | SEQ ID NO: 25634 | SEQ ID NO: 29640 |

FIGURE 50

| | | | | |
|---|---|---|---|---|
| **iPS:434375** | **21-225_66C7** | NA | GACATCCAGTTGACCCAGTCTCCATCCTCCCT<br>GTCTGCATCTGTAGGAGACAGAGTCACCATCA<br>CTCGCCGGGCGAGTCAGGGCATTAGCAATTAT<br>TTACATTGGTATCAGCAGAAACCAGGGAAAGC<br>TCCTAAGCTCTTGATCTATTGTGCATCCAATTT<br>ACAATGTGGAGTCCCATCACGGTTCAGCGGCA<br>GTGGATCTGGGACAGATTTCACTCTCACAATC<br>AGCAGCCTGCAGCCTGAAGATGTTGCAACTTA<br>TTACTGTCAACAGCATAATAATTCCCCGCTCA<br>CTTTCGGCGGAGGGACCAAGGTGGAGATCAA<br>A | CAGGTGCAGCTGGTGGAGTCTGGGGGAGGCGTG<br>GTCCAGCCTGGGAGGTCCCTGAGACTCTCCTGTG<br>TAGCGTCTGGATTCACCTTCAGTGACTATGGCAT<br>GCACTGGGTCCGCCAGGCTCCAGGCAAGGGGCT<br>GGAGTGGGTGGCAGTTATATGGTTTGAGGGAAGT<br>CATAAATACTATACAGACTCCGTGAAGGGCCGAT<br>TCACCATCTCCAGAGACAATTCCAAGAACACGCT<br>GTATCTGCAAATGAACAGCCTGAGAGCCGAGGA<br>CACGGCTGTGTATTACTGTGCGAGAGAACTTGGG<br>TTCAGCAGTGACTACTGGGGCCAGGGAACCCTG<br>GTCACCGTCTCCTCA |
| | | | SEQ ID NO: 25635 | SEQ ID NO: 29641 |
| | | AA | DIQLTQSPSSLSASVGDRVTITRRASQGISNYLH<br>WYQQKPGKAPKLLIYCASNLQCGVPSRFSGSGS<br>GTDFTLTISSLQPEDVATYYCQQHNNSPLTFGGG<br>TKVEIK | QVQLVESGGGVVQPGRSLRLSCVASGFTFSDYGM<br>HWVRQAPGKGLEWVAVIWFEGSHKYYTDSVKGR<br>FTISRDNSKNTLYLQMNSLRAEDTAVYYCARELGF<br>SSDYWGQGTLVTVSS |
| | | | SEQ ID NO: 25636 | SEQ ID NO: 29642 |
| **iPS:434379** | **21-225_66A9** | NA | GACATCCAGATGACCCAGTCTCCATCCTCCCT<br>GTCTGCATCTGTAGGAGACAGAGTCACCATCA<br>CTTGCCGGGCAAGTCAGAACATTTTCAGCTAT<br>TTAAATTGGTATCAGCAGAAACCAGGGAAAG<br>CCCCTAAACTCCTGATCTTTGCTGCATCCAGTT<br>TACAAAGTGGGGTCCCATCAAGGTTCAGTGGC<br>AGTGGATCTGGGACAGATTTCACTCTCACCAT<br>CAGCAGTCTGCAACCTGAGGACTTTGCAACTT<br>ACTACTGTCAACAGACTTACAGTGTCCCTTTC<br>ACTTTCGGCCCTGGGACTAAGGTGGATTTCAA<br>A | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTAGCTATGGCAT<br>GCACTGGGTCCGCCAGGCTCCAGGCAAGGGGCT<br>GGAGTGGGTGGCAGTTATATGGCATGATGGAAG<br>TGATAAATACTATGCAGACTCCGTGAAGGGCCG<br>ATTCACCATCTCCAGAGACAATTCCAAGAACACG<br>CTGTATCTGCAAATGAACAGCCTGAGAGCCGAG<br>GACACGGCTGTGTATTACTGTGCGAGAGAAGAC<br>CCGCGTACCAGTTGTTCTGACTACTGGGGCCAGG<br>GAACCCTGGTCACCGTCTCCTCA |
| | | | SEQ ID NO: 25637 | SEQ ID NO: 29643 |

FIGURE 50

| | | | | |
|---|---|---|---|---|
| | | AA | DIQMTQSPSSLSASVGDRVTITCRASQNIFSYLNWYQQKPGKAPKLLIFAASSLQSGVPSRFSGSGSGTDFTLTISSLQPEDFATYYCQQTYSVPFTFGPGTKVDFK | QVQLVESGGGVVQPGRSLRLSCAASGFTFSSYGMHWVRQAPGKGLEWVAVIWHDGSDKYYADSVKGRFTISRDNSKNTLYLQMNSLRAEDTAVYYCAREDPRTSCSDYWGQGTLVTVSS |
| | | | SEQ ID NO: 25638 | SEQ ID NO: 29644 |
| iPS:434383 | 21-225_66F9 | NA | GACATCCAGATGACCCAGTCTCCATCCTCCCTGTCTGCATCTGTAGGAGACAGAGTCACCATCACTTGCCGGGCAAGTCAGGACATTAGAAATGTTTTAGGCTGGTATCAGCAGAAACCAGGGAAAGCCCCTAAGCGCCTGATCTATACTGCATCCAGTTTACAAAGTGGGGTCCCATCAGGGTTCAGCGGCAGTGGATCTGGGACAGAATTCACTCTCACAATCAGCAGCCTGCAGCCTGAAGATTTTGCAACTTATTACTGTCTACAGTATAATAGTTACCCATTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGACTGGAGTGGGTCTCATCCATTAGTGGTACTAGTAGTTACATATACTACGCAGACTCAGTGAAGGGCCGATTCACCATCTCCAGAGACAACGCCAAGAACTCACTGTATCTGCAAATGAACGGCCTGAGAGCCGAGGACACGGCTGTGTATTTCTGTGCGAGAACCAATGCTTTTGATATCTGGGGCCAGGGACAATGGTCACCGTCTCTTCA |
| | | | SEQ ID NO: 25639 | SEQ ID NO: 29645 |
| | | AA | DIQMTQSPSSLSASVGDRVTITCRASQDIRNVLGWYQQKPGKAPKRLIYTASSLQSGVPSGFSGSGSGTEFTLTISSLQPEDFATYYCLQYNSYPFTFGPGTKVDIK | EVQLVESGGGLVKPGGSLRLSCAASGFTFSSYSMNWVRQAPGKGLEWVSSISGTSSYIYYADSVKGRFTISRDNAKNSLYLQMNGLRAEDTAVYFCARTNAFDIWGQGTMVTVSS |
| | | | SEQ ID NO: 25640 | SEQ ID NO: 29646 |

FIGURE 50

| | | | | |
|---|---|---|---|---|
| iPS:434385 | 21-225_66C10 | NA | GACATCCAGATGACCCAGTCTCCATCCTCCCTGTCTGCATCTGTAGGAGACAGAGTCACCATCACTTGCCGGGCAAGTCAGGCCATTAGAAATGATTTAGGCTGGTATCAGCAGAAACCAGGGAAAGCCCCTAAGCGCCTGATCTATCCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGTATAATAGTTACCCTCCGTGGACGTTCGGCCAAGGGTCCAAGGTGGAAATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGTTATGAGCTGGGTCCGCCAGGCTCCAGGGAAGGGGCTGGAGTGGGTCTCAGGTATTAGTGGTAGTGGTGCTAGAACATACTACGCAGACTCCGTGAAGGGCCGATTCACCATCTCCAGAGACAATTCCAAGAACACGCTGTATCTGCAAATGAACAGCCTGAGAGCCGAGGACACGGCCGTATATTACTGTGCGGAACTGGGGATAGACTACTACTACGGTATGGACGTCTGGGGCCAAGGGACCACGGTCACCGTCTCCTCA |
| | | | SEQ ID NO: 25641 | SEQ ID NO: 29647 |
| | | AA | DIQMTQSPSSLSASVGDRVTITCRASQAIRNDLGWYQQKPGKAPKRLIYPASSLQSGVPSRFSGSGSGTEFTLTISSLQPEDFATYYCLQYNSYPPWTFGQGSKVEIK | EVQLLESGGGLVQPGGSLRLSCAASGFTFSSYVMSWVRQAPGKGLEWVSGISGSGARTYYADSVKGRFTISRDNSKNTLYLQMNSLRAEDTAVYYCAELGIDYYYGMDVWGQGTTVTVSS |
| | | | SEQ ID NO: 25642 | SEQ ID NO: 29648 |
| iPS:434387 | 21-225_66D11 | NA | GATATCCAGATGACCCAGTTTCCATCCTCCCAGTCTGCATCTGTAGGAGACAGAGTCACCATCACTTGCCGGGCAAGTCAGGGCATTAGAAATGATTTAGGTTGGTATCAGCAGAAACCAGGGAAAGCCCATAAGCGCTTGATCTATGCTGCATCCAGTTGTCAAAGTGGGGTCCCATCAAGGTTCAGCGGCAGTGGATGTGGGACAGAATTCACTATCTCAATCAGCAGCATGCAGCGTGAAGATTTTGCAACTTATTACTGTATAGTGCATAATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGGAGCGTCTGGATTCACCTTCAGTAGTTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTCTCTGCAAATGAACAGCCTGAGAGCCGAGGACACGGCTCTGTATTACTGTGCGAGAGAGATGTATAGCAGCAACTGGTACGACTACGGTTTGGACGTCTGGGGCCAAGGGACCACGGTCACCGTCTCCTCA |
| | | | SEQ ID NO: 25643 | SEQ ID NO: 29649 |

FIGURE 50

| | | | | |
|---|---|---|---|---|
| | | AA | DIQMTQFPSSQSASVGDRVTITCRASQGIRNDLGWYQQKPGKAHKRLIYAASSCQSGVPSRFSGSGCGTEFTISISSMQREDFATYYCIVHNSYPRTFGQGTKVEIK | QVQLVESGGGVVQPGRSLRLSCGASGFTFSSYGMHWVRQAPGKGLEWVAVIWYDGSNKYYADSVKGRFTISRDNSKNTLSLQMNSLRAEDTALYYCAREMYSSNWYDYGLDVWGQGTTVTVSS |
| | | | SEQ ID NO: 25644 | SEQ ID NO: 29650 |
| iPS:434389 | 21-225_66F11 | NA | GACATCCAGATGACCCAGTCTCCATCTTCCGTGTGTGCATCTGTAGGAGACAGAGTCACCATCACTTGTCGGGAGAGTCAGGGTATTAGCATCTGGTTAGCCTGGTATCAGCAGAAACCAGGGAAAGCCCCTAAGCTTTTGATCTATGCTGCATCCAGTTTGCAAAGTGGGGTCCCATCAAGGTTCAGCGGCAGTGGATATGGGACAGATTTCACTCTCACCATCAGCAGCGTGCAGCCTGAAGATTTTGCAACTTACTATTGTCAACAGGCTAACAGTTTCCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACCATATGCACTGGGTCCGACAGGCCCCTGGACAAGGGCTTGAGTGGATGGGATGGATCAACCCTAACAATGGTGGCACACACTATGCACAGAAGTTTCAGGACTGGGTCACCATGACCAGGGACACGTCCATCAGCACAGCCTATATGGAACTGAGCAGGCTGAGATCTGACGACACGGCCGTGTATTACTGTGCGAGAGATAGTAGAAGTTCGTGGGACTACTGGGGCCAGGGAACCCTGGTCTCCGTCTCCTCA |
| | | | SEQ ID NO: 25645 | SEQ ID NO: 29651 |
| | | AA | DIQMTQSPSSVCASVGDRVTITCRESQGISIWLAWYQQKPGKAPKLLIYAASSLQSGVPSRFSGSGYGTDFTLTISSVQPEDFATYYCQQANSFPFTFGPGTKVDIK | QVQLVQSGAEVKKPGASVKVSCKASGYTFTGYHMHWVRQAPGQGLEWMGWINPNNGGTHYAQKFQDWVTMTRDTSISTAYMELSRLRSDDTAVYYCARDSRSSWDYWGQGTLVSVSS |
| | | | SEQ ID NO: 25646 | SEQ ID NO: 29652 |

FIGURE 50

| | | | | |
|---|---|---|---|---|
| iPS:434393 | 21-225_67C3 | NA | GAAATAGTGATGACGCAGTCTCCAGCCACCCTGTCTGTGTCTCCAGGGGAAAGAGCCACCCTCTCCTGCAGGGCCAGTCAGAGTGTTAACAGCAACTTAGCCTGGTACCAGCAGAAACCTGGCCAGGCTCCCAGGCTCCTCATCTCTATTGCATCCACCAGGGCCACTGGTATCCCAGCCAGGTTCAATGGCAGTGGGTCTGGGACAGAGTTCACTCTCACCATCAGCAGCCTGCAGTCTGAAGATTTTGCAGTTTATTACTGTCAGCAGTATAATGACTGGCCGTGTAGTTTTGGCCAGGGGACCAAGCTGGAGATCAAA | CAGGTGCAGCTGGTGGAGTCGGGGGGAGGCGTGGTCCAGCCTGGGAGGTCCCTGAGACTCTCCTGTGCAGCGTCTGGATTCACCTTCAGTAGTTATGGCATGCACTGGGTCCGCCAGGCTCCAGGCAAGGGGCTGGAGTGGGTGGCAGCTATATGGTATGATGGAAGTAATAAATATTATGGAGACTCCGTGAAGGGCCGAGTCACCATCTCCAGAGACAATTCCAAGAACTCGCTGCATCTGCAAATGAACAGCCTGAGAGCCGAGGACACGGCTGTGTATTACTGTGCGAGAGATCAGGGCATAGTGGGAGCTACTTGGTTTGACTACTGGGGCCAGGGAACCCTGGTCACCGTCTCCTCA |
| | | | SEQ ID NO: 25647 | SEQ ID NO: 29653 |
| | | AA | EIVMTQSPATLSVSPGERATLSCRASQSVNSNLAWYQQKPGQAPRLLISIASTRATGIPARFNGSGSGTEFTLTISSLQSEDFAVYYCQQYNDWPCSFGQGTKLEIK | QVQLVESGGGVVQPGRSLRLSCAASGFTFSSYGMHWVRQAPGKGLEWVAAIWYDGSNKYYGDSVKGRVTISRDNSKNSLHLQMNSLRAEDTAVYYCARDQGIVGATWFDYWGQGTLVTVSS |
| | | | SEQ ID NO: 25648 | SEQ ID NO: 29654 |
| iPS:434397 | 21-225_67H4 | NA | GACATCCAGATGACCCAGTCTCCATCTTCCGTGTCTGCATCTGTAGGAGACAGAGTCACCATCACTTGTCGGGCGAGTCAGGGTATTAGCAGCTGGTTAGCCTGGTATCAGCAGAAACCAGGGAAAGCCCCTAAGCTCTTGATCTATGCTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGATTAACAGTTTCCCTCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAAACCAAACAGTGGTGGCACAAACCAAGCACAGAAGTTTCAGGGCAGGGTCACCATGACCAGGGACACGTCCATCAGCACAGCCTACATGGAGCTGAGCGGGCTGAGATCTGACGACACGGCCGTGTATTACTGTGCGAGAGCTCCGGGTACTGCAGCAACTGGTACATGGGGATACTTTGACTACTGGGGCCAGGGAACCCTGGTCACCGTCTCCTCA |
| | | | SEQ ID NO: 25649 | SEQ ID NO: 29655 |

FIGURE 50

| | | | | |
|---|---|---|---|---|
| | | AA | DIQMTQSPSSVSASVGDRVTITCRASQGISSWLAWYQQKPGKAPKLLIYAASSLQSGVPSRFSGSGSGTDFTLTISSLQPEDFATYYCQQINSFPLTFGGGTKVEIK | QVQLVQSGAEVKKPGASVKVSCKASGYTFTGYYMHWVRQAPGQGLEWMGWIKPNSGGTNQAQKFQGRVTMTRDTSISTAYMELSGLRSDDTAVYYCARAPGTAATGTWGYFDYWGQGTLVTVSS |
| | | | SEQ ID NO: 25650 | SEQ ID NO: 29656 |
| iPS:434399 | 21-225_67B7 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTTTCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCTCTCTCACAATCAGCAGCCTGCAGCCTGAAGATTTTGCAACTTATTACTGTCTACACCATAATAGTTATCCATTCAAATTTGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAACTATGGCATGCACTGGGTCCGCCAGGCTCCAGGCAAGGGGCTGGAATGGGTGGCAGTTATATTATATGATGGAAGTAAGAAATACTATGCAGACTCCGTGAAGGGCCGATTCACCATCTCCAGAGACAATTCCAAGAACACGCTTTATCTGCAAATGAACAGCCTGAGAGCTGAGGACACGGCTGTGTATTACTGTGCGAGGAGTATCCCGGAATTTGACTATTGGGGCCAGGGAACCCTGGTCACCGTCTCCTCA |
| | | | SEQ ID NO: 25651 | SEQ ID NO: 29657 |
| | | AA | DIQMTQSPSSLSASVGDRVTITCRASQGIRNDLGWFQQKPGKAPKRLIYAASSLQSGVPSRFSGSGSGTEFSLTISSLQPEDFATYYCLHHNSYPFKFGPGTKVDIK | QVQLVESGGGVVQPGRSLRLSCAASGFTFSNYGMHWVRQAPGKGLEWVAVILYDGSKKYYADSVKGRFTISRDNSKNTLYLQMNSLRAEDTAVYYCARSIPEFDYWGQGTLVTVSS |
| | | | SEQ ID NO: 25652 | SEQ ID NO: 29658 |

FIGURE 50

| | | | | |
|---|---|---|---|---|
| **iPS:434405** | **21-225_68E6** | NA | GACATCCAGATGACCCAGTCTCCATCCTCACTGTCTGCATCTGTAGGAGACAGAGTCACCATCACTTGTCGGGCGAGTCAGGGCATTAGCTATTATTTAGCCTGGTTTCAGCAGAAACCAGGGAGAGCCCCTAAGTCCCTGATCTATGTTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAATTTATTACTGCCAACAGTATGATAGTTACCCATTCACTTTCGGCCCTGGGACCAAAGTGGATATCAGA | GAGGTGCAGCTGGTGGAGTCTGGGGGAGGCCTGGTCAAGCCGGGGGGGTCCCTGAGACTCTCCTGTGCAGCCTCTGGATTCACCTTAAGTAGCTTTGGCATGAACTGGGTCCGCCAGGCTCCAGGGAAGGGGCTGGAGTGGGTCTCATACATTAGTAGAAGTAGTAGTCACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGGTCTCTAGTGGGAGCCCCTTTGACTACTGGGGCCAGGGAACCCTGGTCACCGTCTCCTCA |
| | | | SEQ ID NO: 25653 | SEQ ID NO: 29659 |
| | | AA | DIQMTQSPSSLSASVGDRVTITCRASQGISYYLAWFQQKPGRAPKSLIYVASSLQSGVPSRFSGSGSGTDFTLTISSLQPEDFAIYYCQQYDSYPFTFGPGTKVDIR | EVQLVESGGGLVKPGGSLRLSCAASGFTLSSFGMNWVRQAPGKGLEWVSYISRSSSHIYYADSVKGRFTISRDNAKNSLYLQMNSLRAEDTAVYYCAVSSGSPFDYWGQGTLVTVSS |
| | | | SEQ ID NO: 25654 | SEQ ID NO: 29660 |
| **iPS:434407** | **21-225_68G8** | NA | GACATCCAGATGACCCAGTCTCCATCCTCCCTGTCTGCATCTGTAGGAGACAGAGTCACCATCACTTGCCGGGCAAGTCAGGGCATTAGAAATAATTTAGGCTGGTATCAGCAGAGACCAGGGAAAGCCCCTAAGCGCCTGATCTATGCTGCATCCAGTGTGCAAAGTGGGGTCCCATCACGGTTCAGCGGCAGTGGATCTGGGACAGAATTCACTCTCACAATCAGCAGCCTGCAGCCTGAAGATTTTGCAACTTATTACTGTCTACAGTATAATAGTTACCCATTCACTTTCGGCCCTGGGACCAAAGTGGATATCAAA | GAGGTGCAGTTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTAGTTACATATATTACGCAGACTCAGTGATGGGCCGATTCACCATCTCCAGAGACAACGCCAAGAACTCTCTGTATCTGCAAATGAACAGCCTGAGAGCCGAGGACACGGCTGTGTATTACTGTGCGAGAGTCAACAGCTTTGACTCCTGGGGCCAGGGAACCCTGGTCACCGTCTCCTCA |
| | | | SEQ ID NO: 25655 | SEQ ID NO: 29661 |

FIGURE 50

| | | | | |
|---|---|---|---|---|
| | | AA | DIQMTQSPSSLSASVGDRVTITCRASQGIRNNLGWYQQRPGKAPKRLIYAASSVQSGVPSRFSGSGSGTEFTLTISSLQPEDFATYYCLQYNSYPFTFGPGTKVDIK | EVQLVESGGGLVKPGGSLRLSCAASGFTFSSYSMNWVRQAPGKGLEWVSSISGSSSYIYYADSVMGRFTISRDNAKNSLYLQMNSLRAEDTAVYYCARVNSFDSWGQGTLVTVSS |
| | | | SEQ ID NO: 25656 | SEQ ID NO: 29662 |
| iPS:434411 | 21-225_68F11 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGCCAGAATTCACTCTCTCAATCAGCAGCCTGCAGCCTGAAGATTTTGCAACTTATTACTGTCTACAGCATAATAGTTATCCGTTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCTGTTATATGGTATGATGTAAGTAATAAATACTATGCAGACTCCGTGAAGGGCCGATTCACCATCTCCAGAGACAATTCCAAGAACACGCTGTATCTTCAAATGAACAGCCTGAGAGCCGAGGACACGGCTGTGTATTACTGTGTGAGAGAACTGGGGATGACCTCTGACTGCTGGGGCCAGGGAACCCTGGTCACCGTCTCCTCA |
| | | | SEQ ID NO: 25657 | SEQ ID NO: 29663 |
| | | AA | DIQMTQSPSSLSASVGDRVTITCRASQGIRNDLGWYQQKPGKAPKRLIYAASSLQSGVPSRFSGSGSGPEFTLSISSLQPEDFATYYCLQHNSYPFTFGGGTKVEIK | QVQLVESGGGVVQPGRSLRLSCAASGFTFSDYGMHWVRQAPGKGLEWVAVIWYDVSNKYYADSVKGRFTISRDNSKNTLYLQMNSLRAEDTAVYYCVRELGMTSDCWGQGTLVTVSS |
| | | | SEQ ID NO: 25658 | SEQ ID NO: 29664 |

FIGURE 50

| | | | | |
|---|---|---|---|---|
| **iPS:434413** | **21-225_68D12** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGATTCAGCGGCAGTGGATCTGGGACAGAATTCACTCTCACAATCAGCAGCCTGCAGCCTGAAGATTTTGCAACTTATTACTGTCTACAGCATAGTACTTACCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGCAGAAGTAGTTACTACTGGGGCTGGATCCGCCAGCCCCCAGGGAAGGGGCTGGAGTGGATTGGGAATATCTATTATAGTGGGAGCACCTATTACATCCCGTCCCTCAAGAGTCGAGTCACCATATCCGTAGACACGTCCAAGAACCAGTTCTCCCTGAAGCTGACCTCTGTGACCGCCGCAGACACGGCTGTGTATTACTGTGCGAGACATAGCACCAGCTGGTCCATTGACTACTGGGGCCAGGGAACCCTGGTCACCGTCTCCTCA |
| | | | SEQ ID NO: 25659 | SEQ ID NO: 29665 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHSTYPLTFGGTKVEIK | QLQLQESGPGLVKPSETLSLTCTVSGGSISRSSYYWGWIRQPPGKGLEWIGNIYYSGSTYYIPSLKSRVTISVDTSKNQFSLKLTSVTAADTAVYYCARHSTSWSIDYWGQGTLVTVSS |
| | | | SEQ ID NO: 25660 | SEQ ID NO: 29666 |
| **iPS:434417** | **21-225_69C8** | NA | GACATCCAGATGACCCAGTCTCCATCCTCCCTGTCTGCATCTGTAGGAGACAGAGTCACCTTCACTTGCCGGGCAGGTCAGACCATTTACAATTATTTAAATTGGTATCAGCAGAAACCAGGGAAAGCCCCTAAGCTCCTGATCCATGTTGCGTCCAGTTTGCAAAGTGGGGTCCCATCAAGGTTCAGTGGCAGTGGATCTGGGACAGATTTCACTCTCGTCATTAGTAGTCTGCAACCTGAAGATTTTGCAACTTACTACTGTCAACAGAGTTACAGTACCCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGCCATGCACTGGGTCCGCCAGGCTCCAGGCAAGGGGCTGGAGTGGGTGGCAGTTATATGGTATGATGGAAGTGATAAATACTATGCAGACTCCGTGAAGGGCCGATTCACCATCTCCAGAGACAATTCCAAGAACACGCTGTATCTGCAAATGATCAGCCTGAGAGCCGAGGACACGGCTGTTTATTACTGTGCGAGAGATATCCCTAGCAACTCGGCGGGGGACTACTGGGGCCAGGGAACCCTGGTCACCGTCTCCTCA |
| | | | SEQ ID NO: 25661 | SEQ ID NO: 29667 |

FIGURE 50

| | | | | |
|---|---|---|---|---|
| | | AA | DIQMTQSPSSLSASVGDRVTFTCRAGQTIYNYLNWYQQKPGKAPKLLIHVASSLQSGVPSRFSGSGSGTDFTLVISSLQPEDFATYYCQQSYSTPFTFGPGTKVDIK | QVQLVESGGGVVQPGRSLRLSCAASGFTFSNYAMHWVRQAPGKGLEWVAVIWYDGSDKYYADSVKGRFTISRDNSKNTLYLQMISLRAEDTAVYYCARDIPSNSAGDYWGQGTLVTVSS |
| | | | SEQ ID NO: 25662 | SEQ ID NO: 29668 |
| iPS:434423 | 21-225_70D1 | NA | GACATCCAGATGACCCAGTCTCCATCTTCCGTGTCTGCATCTGTAGGAGACAGAGTCACCATCACTTGTCGGGCGAGTCAGGGTGTTAGCAGGTGGTTAGCCTGGTATCAGCAGAAACCAGGGAAAGCCCCTAAGCTCCTGATCTATGCTGCATCCAGTTTGCAAAGTGGGGTCCCATCAAGGTTCAGCGGCAGTGGATCTGGGACAGATTTCACTGTCACCATCAGCAGCCTGCAGCCTGAAGATTTTGCAACTTACTATTGTCAACAGGCTAACAGTTTCCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGTAAGGCTTCTGGATACACCTTCACCGGCTACCATATGCACTGGGTGCGACAGGCCCCTGGACAAGGGCTTGAGTGGATGGGATGGATCAACCCTAACAGTAATGCCACAAACTATGCACAGAAGTTTCAGGGCAGGGTCACCATGACCAGGGACACGTCCATCAGCACAGCCTACATGGAACTGAGCAGGCTGAGATCTGACGACACGGCCGTGTATTACTGTGCGAGAGATAGCATATCGTCGTGGGACTACTGGGGCCAGGGAACCCTGGTCACCGTCTCCTCA |
| | | | SEQ ID NO: 25663 | SEQ ID NO: 29669 |
| | | AA | DIQMTQSPSSVSASVGDRVTITCRASQGVSRWLAWYQQKPGKAPKLLIYAASSLQSGVPSRFSGSGSGTDFTVTISSLQPEDFATYYCQQANSFPFTFGPGTKVDIK | QVQLVQSGAEVKKPGASVKVSCKASGYTFTGYHMHWVRQAPGQGLEWMGWINPNSNATNYAQKFQGRVTMTRDTSISTAYMELSRLRSDDTAVYYCARDSISSWDYWGQGTLVTVSS |
| | | | SEQ ID NO: 25664 | SEQ ID NO: 29670 |

FIGURE 50

| | | | | |
|---|---|---|---|---|
| **iPS:434425** | **21-225_70A5** | NA | GAAATAGTGATGACGCAGTCTCCAGCCACCCTGTCTGTGTCTCCAGGGGAAAGAGCCACCCTCTCCTGCAGGGCCAGTCAGAGTGTTAACAGCAACTTAGCCTGGTACCAGCTGAAACCTGGCCAGGCTCCCAGGCTCCTCATCTCTATTGCATCCACCAGGGCCACTGGTATCCCACCCCGGTTCAATGGCAGTGGGTCTGGGACAGAGTTCACTCTCACCATCAGCAGCCTGCAGTCTGAAGATTTTGCAGTTTATTACTGTCAGCAGTATAATGACTGGCCGTGTAGTTTTGGCCAGGGGACCAAGCTGGAGATCAAA | CAGGTTCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACTCGCTGTATCTGCAAATGAACAGCCTGAGCGCCGAGGACACGGCTGTTTATTACTGTGCGAGAGATCAGGGCATAGTGGGAGCTACTTGGTTTGACTACTGGGGCCAGGGAACCCTGGTCACCGTCTCCTCA |
| | | | SEQ ID NO: 25665 | SEQ ID NO: 29671 |
| | | AA | EIVMTQSPATLSVSPGERATLSCRASQSVNSNLAWYQLKPGQAPRLLISIASTRATGIPPRFNGSGSGTEFTLTISSLQSEDFAVYYCQQYNDWPCSFGQGTKLEIK | QVQLVESGGGVVQPGRSLRLSCAASGFTFSSYGMHWVRQAPGKGLEWVAVIWYDGSNKYYADSVKGRFTISRDNSKNSLYLQMNSLSAEDTAVYYCARDQGIVGATWFDYWGQGTLVTVSS |
| | | | SEQ ID NO: 25666 | SEQ ID NO: 29672 |
| **iPS:434427** | **21-225_70D6** | NA | GACATCCAGATGACCCAGTCTCCATCTTCCGTGTCTGCATCTGTAGGAGACAGAGTCACCATCACTTGTCGGGCGAGTCAGGGTATTAGCAAATGGTTAGCCTGGTATCAGCAGAATCCAGGGAAAGCCCTTAAGCTCTTGATCTTTGCTGCATCCAGTTTGCAAAGTGGGGTCCCATCAAGGTTCAGCGGCAGTGGATCTGGGACAGATTTCACTCTCACCATCAGCAGCCTCCAGCCTGAAGATTTTGCAAATTACTATTGTCAACAGACTAACAGTTTCCCTCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCCTCTGGATACATCTTCACCGGCTACTATATGCACTGGGTGCGACAGGCCCCTGGACAAGGGCTTGAGTGGATGGGATGGATCAACCCTAAGAGTGGTGGCACAAACTCTGCACAGAAGTTTCAGGGCAGGGTCTCCATGACCAGGGACACGTCCATCGGCACAGCCTACATGGAGCTGCGCGGGCTAAGATCTGACGACACGGCCGAGTATTACTGTGCGAGAGCTCCGGGTAAAGCAGCAGCTGGTACATGGGGATTCTTTGACTACTGGGGCCAGGGAACCCTGGTCACCGTCTCCTCA |
| | | | SEQ ID NO: 25667 | SEQ ID NO: 29673 |

FIGURE 50

| | | | | |
|---|---|---|---|---|
| | | AA | DIQMTQSPSSVSASVGDRVTITCRASQGISKWLAWYQQNPGKALKLLIFAASSLQSGVPSRFSGSGSGTDFTLTISSLQPEDFANYYCQQTNSFPLTFGGGTKVEIK | QVQLVQSGAEVKKPGASVKVSCKASGYIFTGYYMHWVRQAPGQGLEWMGWINPKSGGTNSAQKFQGRVSMTRDTSIGTAYMELRGLRSDDTAEYYCARAPGKAAAGTWGFFDYWGQGTLVTVSS |
| | | | SEQ ID NO: 25668 | SEQ ID NO: 29674 |
| iPS:434429 | 21-225_70H6 | NA | GACATCCAGATGACCCAGTCTCCATCCTCCCTGTCTGCATCTTTAGGAGACAGAGTCACCATCACTTGCCGGACAAGTCAGAGCATTTTCAACTATTTAAATTGGTTTCAGCGGAAACCAGGGAAAGCCCCTAAGGTCCTGATCTATACTGCATCCAGTTTGCAAAGTGGGATCCCATCAAGGTTCAGTGGCAGTGGATCTGGGACAGACTTCACTCTCACCATCAGCAGTCTGCAACCTGAAGATTCTGCAACTTACTACTGTCAACAGAGTTACAGTATCCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGTTATGGCATGCACTGGGTCCGCCAGACTCCAGGCAAGGGGCTGGACTGGGTGGCAGTTATATGGCATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATGATCCCAGATCCTCGGCCGGGGACTACTGGGGCCAGGGAACCCTGGTCACCGTCTCCTCA |
| | | | SEQ ID NO: 25669 | SEQ ID NO: 29675 |
| | | AA | DIQMTQSPSSLSASLGDRVTITCRTSQSIFNYLNWFQRKPGKAPKVLIYTASSLQSGIPSRFSGSGSGTDFTLTISSLQPEDSATYYCQQSYSIPLTFGGGTKVEIK | QVQLVESGGGVVQPGRSLRLSCAASGFTFSSYGMHWVRQTPGKGLDWVAVIWHDGSNKYYADSVKGRFTISRDNSKNTLYLQMNSLRAEDTAVYYCARDDPRSSAGDYWGQGTLVTVSS |
| | | | SEQ ID NO: 25670 | SEQ ID NO: 29676 |

FIGURE 50

| | | | | |
|---|---|---|---|---|
| iPS:434431 | 21-225_70E7 | NA | GACATCGTGATGACCCAGTCTCCAGACTCCCTGGCTGTGTCTCTGGGCGAGAGGGCCACCCTCAACTGCAAGTCCAGCCAGAGTGTTTTATACAGCTCCAACAATAACAACTACTTGGCTTGGTACCAGCAGAAACCAGGACAGCCTCCTAAACTGCTCATTTACTGGGCATCTACCCGGGAATCCGGGGTCCCTGACCGATTCAGTGGCAGCGGGTCTGGGACAGATTTCACTCTCACTATCAGCAGCCTGCAGGCTGAAGATGTGGCAGTTTATTACTGTCAGCAATATTATAATATTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGGTGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGTATAGCAGTGGCTGGTACGTCTTTGACTACTGGGGCCAGGGAACCCTGGTCACCGTCTCCTCA |
| | | | SEQ ID NO: 25671 | SEQ ID NO: 29677 |
| | | AA | DIVMTQSPDSLAVSLGERATLNCKSSQSVLYSSNNNNYLAWYQQKPGQPPKLLIYWASTRESGVPDRFSGSGSGTDFTLTISSLQAEDVAVYYCQQYYNIPPTFGQGTKVEIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAYSSGWYVFDYWGQGTLVTVSS |
| | | | SEQ ID NO: 25672 | SEQ ID NO: 29678 |
| iPS:434433 | 21-225_70E8 | NA | GACATCCAGATGACCCAGTCTCCATCCTCCCTGTCTACATCTGTCGGAGACAGAGTCACCATCACTTGCCGGGCAAGTCAGGGCATTAGAAAGGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATCGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCTCTGGGTCCGCCAGGCTCCAGGCAAGGGTCTGGAGTGGGTGGCAATTATATGGTATGATGAAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGACCTACTGGACCCACGGGACTACTGGGGCCAGGGAACCCTGGTCACCGTCTCCTCA |
| | | | SEQ ID NO: 25673 | SEQ ID NO: 29679 |

FIGURE 50

| | | | | |
|---|---|---|---|---|
| | | AA | DIQMTQSPSSLSTSVGDRVTITCRASQGIRKDLG<br>WYQQKPGKAPKRLIYAASSLQSGVPSRFSGSGS<br>GTEFTLTISSLQPEDFATYYCLQHNRYPLTFGGG<br>TKVEIK | QVQLVESGGGVVQPGRSLRLSCAASGFTFSSYGML<br>WVRQAPGKGLEWVAIIWYDESNKYYADSVKGRFT<br>ISRDNSKNTLYLQMNSLRAEDTAVYYCARDLLDPR<br>DYWGQGTLVTVSS |
| | | | SEQ ID NO: 25674 | SEQ ID NO: 29680 |
| iPS:434435 | 21-225_70G9 | NA | GACATCCAAATGACCCAGTCTCCATCTTCCGT<br>GTCTGCATCTGTAGGAGACAGAGTCACCATCA<br>CTTGTCGGGCGAGTCAGGATATTAGCAGCTGG<br>TTAGCCTGGTATCAGCAGAATCCAGGGAAAGC<br>CCCTAAACTCTTGATCTATGCTGCATCCAGTTT<br>GCAAAGTGGGGTCCCATCAAGGTTCAGCGGCA<br>GTGGATCTGGGACAGATTTCACTCTCACCATC<br>AGCAGCCTCCAGCCTGAAGATTTTGCAACTTA<br>CTATTGTCAACAGACTAACAGTTTCCCTCTCAC<br>TTTCGGCGGAGGGACCAAGGTGGAGATCAAA | CAGGTGCAGCTGGTGCAGTCTGGGGCTGAGGTG<br>AAGAAGCCTGGGGCCTCAGTGAAGGTCTCCTGC<br>AAGGCTTCTGGATACACCTTCACCGGCTACTATA<br>TGCACTGGGTGCGACAGGCCCCTGGACAAGGAC<br>TTGAGTGGATGGGATGGATCAAACCTAACAGTG<br>GTGGCACAAACCAAGCACAGAAGTTTCAGGGCA<br>GGGTCACCATGACCAGGGACACGTCCATCAGCA<br>CAGCCTACATGGAGCTGAGCAGTCTGAGATCTGA<br>CGACACGGCCGTGTATTACTGTGCGAGAGCTCCG<br>GGTATAGCAGCAGCTGGTACATGGGGATACTTTG<br>ACTACTGGGGCCAGGGAACCCTGGTCACCGTCTC<br>CTCA |
| | | | SEQ ID NO: 25675 | SEQ ID NO: 29681 |
| | | AA | DIQMTQSPSSVSASVGDRVTITCRASQDISSWLA<br>WYQQNPGKAPKLLIYAASSLQSGVPSRFSGSGS<br>GTDFTLTISSLQPEDFATYYCQQTNSFPLTFGGGT<br>KVEIK | QVQLVQSGAEVKKPGASVKVSCKASGYTFTGYYM<br>HWVRQAPGQGLEWMGWIKPNSGGTNQAQKFQGR<br>VTMTRDTSISTAYMELSSLRSDDTAVYYCARAPGI<br>AAAGTWGYFDYWGQGTLVTVSS |
| | | | SEQ ID NO: 25676 | SEQ ID NO: 29682 |

FIGURE 50

| | | | | |
|---|---|---|---|---|
| **iPS:434437** | **21-225_70A12** | NA | GACATCCAGATGACCCAGTCTCCATCTTCCGTGTCTGCATCTGTAGGAGACAGAGTCACCATCACTTGTCGGGCGAGTCAGGGTATTAGCAGCTGGTTAGCCTGGTATCAGCAGAAACCAGGGAAAGCCCTTAAGCTCTTGATATATGCTGCATCCAGTTTGCAAAGTGGGGTCCCATCAAGGTTCAGCGGCAGTGGATCTGGGACAGATTTCACTCTCACCATCAGCAGCGTGCAGCCTGAAGATTTTGCAACTTACTATTGTCAACAGATTAACAGTTTCCCTCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAAACCAAACAGTGGTGGCACAAACCAAGCACAGAAGTTTCAGGGCAGGGTCACCATGACCAGGGACACGTCCATCAGCACAGCCTACATGGAGCTGAGCGGGCTGAGATCTGACGACACGGCCGTGTATTACTGTGCGAGAGCTCCGGGTACTGCAGCAACTGGTACATGGGGATACTTTGACTACTGGGGCCAGGGAACCCTGGTCACCGTCTCCTCA |
| | | | SEQ ID NO: 25677 | SEQ ID NO: 29683 |
| | | AA | DIQMTQSPSSVSASVGDRVTITCRASQGISSWLAWYQQKPGKALKLLIYAASSLQSGVPSRFSGSGSGTDFTLTISSVQPEDFATYYCQQINSFPLTFGGGTKVEIK | QVQLVQSGAEVKKPGASVKVSCKASGYTFTGYYMHWVRQAPGQGLEWMGWIKPNSGGTNQAQKFQGRVTMTRDTSISTAYMELSGLRSDDTAVYYCARAPGTAATGTWGYFDYWGQGTLVTVSS |
| | | | SEQ ID NO: 25678 | SEQ ID NO: 29684 |
| **iPS:434439** | **21-225_70E12** | NA | GACATCCAGATGACCCAGTCTCCATCCTCCCTATCTGCATCTGTAGGAGACAGAGTCACCATCACTTGCCGGGCAAGTCAGGGCATTAACAATAATTTAAACTGGTTTCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGTCCAAGGTGGAGATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCTTCCATTAGTGGTAATAGTACTTACATATACTACACAGACTCAGTGAAGGGCCGATTCACCATCTCCAGAGACAACGCCAAGAACTCACTGTATCTGCAAATGGACAGCCTGACAGCCGAGGACACGGCTGTGTATTACTGTGCGAGAGTGGCCGCCTTTGACTGCTGGGGCCAGGGAACCCTGGTCACCGTCTCCTCA |
| | | | SEQ ID NO: 25679 | SEQ ID NO: 29685 |

FIGURE 50

| | | | | |
|---|---|---|---|---|
| | | AA | DIQMTQSPSSLSASVGDRVTITCRASQGINNNLNWFQQKPGKAPKRLIYAASSLQSGVPSRFSGSGSGTEFTLTISSLQPEDFATYYCLQHNSYPLTFGGGSKVEIK | EVQLVESGGGLVKPGGSLRLSCAASGFTFSSYSMNWVRQAPGKGLEWVSSISGNSTYIYYTDSVKGRFTISRDNAKNSLYLQMDSLTAEDTAVYYCARVAAFDCWGQGTLVTVSS |
| | | | SEQ ID NO: 25680 | SEQ ID NO: 29686 |
| iPS:434441 | 21-225_71A2 | NA | GACATCCAGATGACCCAGTCTCCATCCTCCCGGTCTGCATCTGTAGGAGACAGAGTCACCATCACTTGCCGTGCAAGTCAGGGCATTAGAAATGATTTAGGATGGTATCAGCAGAAACCAGGAAAAGCCCCTAAGCGCCTGATCTATATTGCATTCAGATTGCAAATTGGGGTCCCATCAAGGTTCAGCGGCAGTGGATCTGGGACAGAATTCACTCTCACAATCAGCAGCCTGCAGCCTGAAGATTTTGCAACTTATTACTGTATACACCATAATAGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAACGTCTGGATTCACCTTCAGTGACTATGGCATGCACTGGGTCCGCCAGGCTCCAGGCAAGGGGCTGGAGTGGGTGGCAGTGATATGGTATGATGAAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAATTGGGGTGGCAGGATGATTACTGGGGCCAGGGAACCCTGGTCACCGTCTCCTCA |
| | | | SEQ ID NO: 25681 | SEQ ID NO: 29687 |
| | | AA | DIQMTQSPSSRSASVGDRVTITCRASQGIRNDLGWYQQKPGKAPKRLIYIAFRLQIGVPSRFSGSGSGTEFTLTISSLQPEDFATYYCIHHNSYPWTFGQGTKVEIK | QVQLVESGGGVVQPGRSLRLSCATSGFTFSDYGMHWVRQAPGKGLEWVAVIWYDESNKYYADSVKGRFTISRDNSKNTLYLQMNSLRAEDTAVYYCARELGWQDDYWGQGTLVTVSS |
| | | | SEQ ID NO: 25682 | SEQ ID NO: 29688 |

FIGURE 50

| | | | | |
|---|---|---|---|---|
| **iPS:434443** | **21-225_71G3** | NA | GACATCGTGATGACCCAGTCTCCAGACTCCCTGGCTGTGTCTCTGGGCGAGAGGGTCGCCATCAATTGCAAGTCCAGCCAGAGTGTTTTACACAGCTCCAACAATAACAACTACTTAGATTGGTATCAGCAGAAACCAGGACAGCTTCCTAAACTGCTCATTTTCTGGGCATCTACCCGGGAATTCGGGGTTCCTGACCGATTCAGTGGCAGCGGGTTTGGGACAGATTTCACTCTCACCATCAGCAGCCTGCAGGCTGAAGATGTGGCAGATTACTACTGTCAACAATATTATATTACTCCGTGCAGTTTTGGCCAGGGGACCAAGCTGGAGATCAAA | CAGGTGCAACTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAGCACAGGCTATGCACAGAAGTTCCAGGGCAGAGTCACCATGACCAGGGACACCTCCGTCAGCACAGCCTACATGGAGCTGAGCAGCCTGAGATCTGAGGACACGGCCGTCTATTACTGTGCATATAGCAGTGGCTGGTACTACTTTGACTACTGGGGCCAGGGAACCCTGGTCACCGTCTCCTCA |
| | | | SEQ ID NO: 25683 | SEQ ID NO: 29689 |
| | | AA | DIVMTQSPDSLAVSLGERVAINCKSSQSVLHSSNNNNYLDWYQQKPGQLPKLLIFWASTREFGVPDRFSGSGFGTDFTLTISSLQAEDVADYYCQQYYITPCSFGQGTKLEIK | QVQLVQSGAEVKKPGASVKVSCKASGYTFTNYDINWVRQATGQGLEWMGWMHPNSGSTGYAQKFQGRVTMTRDTSVSTAYMELSSLRSEDTAVYYCAYSSGWYYFDYWGQGTLVTVSS |
| | | | SEQ ID NO: 25684 | SEQ ID NO: 29690 |
| **iPS:434447** | **21-225_71B6** | NA | GACATCCAGATGACCCAGTCTCCATCCTCCCCGTCTGCATCTGTAGGAGACAGAGTCACCATCACTTGCCGGGCAAGTCAGGGCATTAGAAATGATTTAGATTGGTATCAGCAGAAGCCAGGGAAGGCCCCTCAGCGCCTTATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGACGATTTTGCAACTTATTACTGTCTACAGCATAATAGTTACCCATTCACTTTCGGCCCTGGGACCAAAGTGGATATCAAA | CAGGTGCAACTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCCCCAGGCAAGGGGCTGGAGTGGGTGGCAGTTATTTGGTATGATAGAACAAATAAATACTATGCAGACTCCGTGAAGGGCCGATTCACCATCTCCAGAGACAATTCCAAGAACACGCTGCATCTGCAAATGAACAGCCTGAGAGCCGAGGACACGGCTGTGTATTACTGTGCGAGAGAACTGGGGATGTTGTCTGACTACTGGGGCCAGGGAACCCTGGTCACCGTCTCCTCA |
| | | | SEQ ID NO: 25685 | SEQ ID NO: 29691 |

FIGURE 50

| | | | | |
|---|---|---|---|---|
| | | AA | DIQMTQSPSSPSASVGDRVTITCRASQGIRNDLDWYQQKPGKAPQRLIYAASSLQSGVPSRFSGSGSGTEFTLTISSLQPDDFATYYCLQHNSYPFTFGPGTKVDIK | QVQLVESGGGVVQPGRSLRLSCAASGFTFSNYGMHWVRQAPGKGLEWVAVIWYDRTNKYYADSVKGRFTISRDNSKNTLHLQMNSLRAEDTAVYYCARELGMLSDYWGQGTLVTVSS |
| | | | SEQ ID NO: 25686 | SEQ ID NO: 29692 |
| iPS:434449 | 21-225_71H6 | NA | GACATCCAGATGACCCAGTCTCCATCCTCCCTGTCTGCATCTGTAGGAGACAGAGTCACCATCACTTGCCGGGCAAGTCAGGGCATTAGAAATGTTTTAGGCTGGTATCAGCAGAAACCAGGGAAAGCCCCTAAGCGCCTGATCTATGCTGCATCCAGTTTACAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGTATAATAGTTACCCATTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTGTCCTGTGCAGCCTCTGGATTCACCTTCAGTAGCTATAGCATGAACTGGGTCCGCCAGGCTCCAGGGAAGGGACTGGAGTGGGTCTCATCCATTAGTGGTACTAGTAGTTACATATACTACGCAGACTCAGTGAAGGGCCGATTCACCATCTCCAGAGACAACGCCAAGAACTCACTGTATCTGCAAATGAACGGCCTGAGAGCCGAGGACACGGCTGTGTATTTCTGTGCGAAAACCAATGCTTTTGATATCTGGGGCCAGGGGACAATGGTCACCGTCTCTTCA |
| | | | SEQ ID NO: 25687 | SEQ ID NO: 29693 |
| | | AA | DIQMTQSPSSLSASVGDRVTITCRASQGIRNVLGWYQQKPGKAPKRLIYAASSLQSGVPSRFSGSGSGTEFTLTISSLQPEDFATYYCLQYNSYPFTFGPGTKVDIK | EVQLVESGGGLVKPGGSLRLSCAASGFTFSSYSMNWVRQAPGKGLEWVSSISGTSSYIYYADSVKGRFTISRDNAKNSLYLQMNGLRAEDTAVYFCAKTNAFDIWGQGTMVTVSS |
| | | | SEQ ID NO: 25688 | SEQ ID NO: 29694 |

FIGURE 50

| | | | | |
|---|---|---|---|---|
| **iPS:434451** | **21-225_71B7** | NA | GACATCCAGATGACCCAGTCTCCATCTTCCGTGTCTGCATCTGTAGGAGACAGAGTCACCATCACTTGTCGGGCGAGTCAGGGTATTAGCAGCTGGTTAGCCTGGTATCAGCAGAATCCAGGGGAAGCCCCTAAGCTCCTGATCTATGCTGCATCCAGTTTGCAAGGTGGGGTCCCATCAAGGTTCAGCGGCAGTGGATCTGGGACAGATTTCACTCTCACCATCAGCAGCCTCCAGCCTGAAGATTTTGCAAATTACTATTGTCAACAGACTAATAGTTTCCCTCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACATCTTCACCGGCTACTATATGCACTGGGTGCGACAGGCCCCTGGACAAGGGCTTGAGTGGATGGGATGGATCAACCCTAACAGTGGTGGCACAAACTCTGCACAGAAGTTTCAGGGCAGGGTCACCATGACCAGGGACACGTCCATCGGCACAGCCTACATGGAGCTGAGCGGGCTGAGATCTGACGACACGGCCGTGTATTACTGTGCGAGAGCTCCGGGTAAAGCAGCAGCTGGTACATGGGGATTCTTTGACTACTGGGGCCAGGGAACCCTGGTCACCGTCTCCTCA |
| | | | SEQ ID NO: 25689 | SEQ ID NO: 29695 |
| | | AA | DIQMTQSPSSVSASVGDRVTITCRASQGISSWLAWYQQNPGEAPKLLIYAASSLQGGVPSRFSGSGSGTDFTLTISSLQPEDFANYYCQQTNSFPLTFGGGTKVEIK | QVQLVQSGAEVKKPGASVKVSCKASGYIFTGYYMHWVRQAPGQGLEWMGWINPNSGGTNSAQKFQGRVTMTRDTSIGTAYMELSGLRSDDTAVYYCARAPGKAAAGTWGFFDYWGQGTLVTVSS |
| | | | SEQ ID NO: 25690 | SEQ ID NO: 29696 |
| **iPS:434453** | **21-225_71B11** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGATTGGTATCAGCAGACACCAGGGAAAGCCCCTAAGCGCCTGATCTATGCTGCATCCAGTTTGCAAAGTGGGGTCCCATCAAGGTTCAGCGGCGGTGGATCTGGGACAGAATTCTCTCTCACAATCAGCAGCCTGCAGCCTGAAGATTTTACAACTTATTACTGTCTACAGCATAATACTTACCCATTCACTTTCGGCCCTGGGACCAAAGTGGATGTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATAGAAATAATAAATACTATGGAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGATGTTGTCTGACTACTGGGGCCAGGGAACCCTGGTCACCGTCTCCTCA |
| | | | SEQ ID NO: 25691 | SEQ ID NO: 29697 |

FIGURE 50

| | | | | |
|---|---|---|---|---|
| | | AA | DIQMTQSPSSLSASVGDRVTITCRASQGIRNDLDWYQQTPGKAPKRLIYAASSLQSGVPSRFSGGGSGTEFSLTISSLQPEDFTTYYCLQHNTYPFTFGPGTKVDVK | QVQLVESGGGVVQPGRSLRLSCAASGFTFSDYGMHWVRQAPGKGLEWVAVIWYDRNNKYYGDSVKGRFTISRDNSKNTLYLQMNSLRAEDTAVYYCARELGMLSDYWGQGTLVTVSS |
| | | | SEQ ID NO: 25692 | SEQ ID NO: 29698 |
| iPS:434455 | 21-225_72F5 | NA | GACATCCAGATGACCCAGTCTCCATCCTCCCTGTCTGCATCTGTAGGAGACAGAGTCATCATCCCTTGCCGGGCAAGTCAGAACATTAGCAGCTATTTAAATTGGTATCAGCAGAAACCAGGGAAAGCCCCTAAACTCCTGATCTATGCTGCATCCAGTTTGCAAAGTGGGGTCCCATCAAGGTTCAGTGGCAGTGGATCTGGGACAGATTTCACTCTGACCATCAGCAGTCTGCAGCCTGAAGATTTTGCAACTTACTCCTGTCAACAGACTTACAGTACCCCCACCTTCGGCCAAGGGACACGACTGGATATTAAT | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGATCTGGGTCCGCCAGGCTCCAGGGAAGGGGCTGGAGTGGGTCTCAACTATTAGTGGTAGTGGTGGTTACACATACTCCGCAGACTCCGTGAAGGGCCGGTTCACCATCTCCAGAGACAATTCCAAGAACACGCTGTATCTTCAAATGAACAGCCTGAGAGCCGAGGACACGGCCGTATATTACTGTGCGAGACGTATAGCAGTGACTGGGACGGAATGGTACGACCCCTGGGGCCAGGGAACCCTGGTCACCGTCTCCTCA |
| | | | SEQ ID NO: 25693 | SEQ ID NO: 29699 |
| | | AA | DIQMTQSPSSLSASVGDRVIIPCRASQNISSYLNWYQQKPGKAPKLLIYAASSLQSGVPSRFSGSGSGTDFTLTISSLQPEDFATYSCQQTYSTPTFGQGTRLDIN | EVQLLESGGGLVQPGGSLRLSCAASGFTFSSYAMIWVRQAPGKGLEWVSTISGSGGYTYSADSVKGRFTISRDNSKNTLYLQMNSLRAEDTAVYYCARRIAVTGTEWYDPWGQGTLVTVSS |
| | | | SEQ ID NO: 25694 | SEQ ID NO: 29700 |

FIGURE 50

| | | | | |
|---|---|---|---|---|
| iPS:434457 | 21-225_72G12 | NA | GACATCCAGTTGACCCAGTCTCCATCCTCCCTGTCTGCATCTGTAGGAGACAGAGTCACCATCACTTGCCGGGCGAGTCAGGGCATTAGCAGTTATTTAAATTGGTCTCAGCAGAAACCAGGGAAAGTTCCTAAGCTCCTGATCTGTGGTGCTTCCAATTTGCAATCTGGAGTCCCATCTCGGTTCAGCGGCAGTGCATCTGGGACAGAATTCATTCTCACTATCAGCAGCCTGCAGCCTGAAGATGTTACAACTTATTACGGTCAACAGAATTACAATGCCCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGACACGGGGCTGGAGTGGGTGGCAGTTATATGGTTTGATGAAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGCTTGGTTTCAGCAGTGACTACTGGGGCCAGGGAACCCTGGTCACCGTCTCCTCA |
| | | | SEQ ID NO: 25695 | SEQ ID NO: 29701 |
| | | AA | DIQLTQSPSSLSASVGDRVTITCRASQGISSYLNWSQQKPGKVPKLLICGASNLQSGVPSRFSGSASGTEFILTISSLQPEDVTTYYGQQNYNAPLTFGGGTKVEIK | QVQLVESGGGVVQPGRSLRLSCAASGFTFSSYGMHWVRQAPDTGLEWVAVIWFDESNKYYADSVKGRFTISRDNSKNTLYLQMNSLRAEDTAVYYCARELGFSSDYWGQGTLVTVSS |
| | | | SEQ ID NO: 25696 | SEQ ID NO: 29702 |
| iPS:434459 | 21-225_71A7 | NA | GACATCCAGATGACCCAGTCTCCATCTTCCGTGTCTGCATCTGTAGGAGACAGAGTCACCATCACTTGTCGGGCGAGTCAGGGTATTAGCAGCTGGTTAGCCTGGTATCAGCAGAAACCTGGGAAAGCCCCTAAGCTCCTGATCTATGCTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGGTTAACAGTTTCCCTCTCACTTTCGGCGGAGGGACCAAGGTGGAGCTCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAAAAGTGGTGGCACAAATTATGTACAGAAGTTTCAGGGCAGGGTCACCATGACCAGGGACACGTCCATCAGCACAGCCTACATGGAGCTGAGCAGTCTGAGATCTGACGACACGGCCGTGTATTACTGTGCGAGAGCTCCGGGTACAGCACCAGCTGGGTCATGGGGATACTTTGACTACTGGGGCCAGGGAACCCTGGTCACCGTCTCCTCA |
| | | | SEQ ID NO: 25697 | SEQ ID NO: 29703 |

FIGURE 50

| | | | | |
|---|---|---|---|---|
| | | AA | DIQMTQSPSSVSASVGDRVTITCRASQGISSWLAWYQQKPGKAPKLLIYAASSLQSGVPSRFSGSGSGTDFTLTISSLQPEDFATYYCQQVNSFPLTFGGGTKVELK | QVQLVQSGAEVKKPGASVKVSCKASGYTFTGYYMHWVRQAPGQGLEWMGWINPKSGGTNYVQKFQGRVTMTRDTSISTAYMELSSLRSDDTAVYYCARAPGTAPAGSWGYFDYWGQGTLVTVSS |
| | | | SEQ ID NO: 25698 | SEQ ID NO: 29704 |
| **iPS:434461** | **21-225_73A3** | NA | GACATCCAGATGACCCAGTCTCCATCTTCCGTGTCTGCATCTATAGGAGACAGAGTCACCATCACTTGTCGGGCGAGTCAGGGTATTAGCAACTGGTTAGCCTGGTATCAGCAGAAACCAGGGAAAGCCCCTAAGCTCCTGATCTATGCTGCATCCAGTTTGCAAAGTGGGGTCCCGTCAAGGTTCAGCGGCAGTGGATCTGAGACAGATTTCACTCTCACCATCAGCAGCCTGCAGCCTGAAGATTTTGCAACTTACTATTGTCAACAGGTTAACAGTTTCCCTCTCACTTTCGGCGGAGGGACCAAGGTGGAGATCAAA | CAGGTGCAACTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTACGACAGGCCCCTGGACAAGGACTTGACTGGATGGGATGGATCAACCCTAAAAGTGGTGGCACGAATCATGTCCAGAAGTTTCAGGGCAGGGTCGCCATGACCAGGGACACGTCCATCAGCACAGCCTACATGGAGCTGAGCAGTCTGAGATCTGACGACACGGCCGTGTATTACTGTGCGAGAGCTCCGGGTACAGCAGCAGCTGGGTCATGGGGATGCTTTGACTACTGGGGCCAGGGAACCCTGGTCACCGTCTCCTCA |
| | | | SEQ ID NO: 25699 | SEQ ID NO: 29705 |
| | | AA | DIQMTQSPSSVSASIGDRVTITCRASQGISNWLAWYQQKPGKAPKLLIYAASSLQSGVPSRFSGSGSETDFTLTISSLQPEDFATYYCQQVNSFPLTFGGGTKVEIK | QVQLVQSGAEVKKPGASVKVSCKASGYTFTGYYMHWVRQAPGQGLDWMGWINPKSGGTNHVQKFQGRVAMTRDTSISTAYMELSSLRSDDTAVYYCARAPGTAAAGSWGCFDYWGQGTLVTVSS |
| | | | SEQ ID NO: 25700 | SEQ ID NO: 29706 |

FIGURE 50

| | | | | |
|---|---|---|---|---|
| **iPS:434463** | **21-225_73A6** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTTTCAGCAGAAACCAGGGAAAGCCCCTAAGCGCCTGATCTATGCTGCATCCAATTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTTACTATGGCATGCACTGGGTCCGCCAGGCTCCAGGCAAGGGGCTGGAGTGGGTGGCAGTTATATTATATGATGGAAGTAAGAAATACTATGCAGCCTCCGTGAAGGGCCGATTCACCATCTCCAGAGACAATTCCAAGAACACGCTGTATCTGCAAATGAACAGCCTGAGAGCTGAGGACACGGCTGTGTATTACTGTGCGAGGAGTATCCCGGACTTTGACTACTGGGGCCAGGGAACCCTGGTCACCGTCTCCTCA |
| | | | SEQ ID NO: 25701 | SEQ ID NO: 29707 |
| | | AA | DIQMTQSPSSLSASVGDRVTITCRASQGIRNDLGWFQQKPGKAPKRLIYAASNLQSGVPSRFSGSGSGTEFTLTISSLQPEDFATYYCLQHNSYPFTFGPGTKVDIK | QVQLVESGGGVVQPGRSLRLSCAASGFTFSYYGMHWVRQAPGKGLEWVAVILYDGSKKYYAASVKGRFTISRDNSKNTLYLQMNSLRAEDTAVYYCARSIPDFDYWGQGTLVTVSS |
| | | | SEQ ID NO: 25702 | SEQ ID NO: 29708 |
| **iPS:434467** | **21-225_73H8** | NA | GACATCCAGATGACCCAGTCTCCATCCTCCCTGTATGCATCTGTAGGAGACAGAGTCACCATCACTCGCCGGGCAAGTCAGGACATCAGAAATGATTTAGGCTGGTATCAGCAGAAACCAGGGAAAGCCCTTAAGCGCGTGATCTATGCTGCATCCAGTTTGCAAAGTGGGGTCCCATCAAGCTTCAGCGGCAGTGGATCTGGGACAGAATTCACTCTCACAATCAGCAGCTTGCAGCCTGAAGATTTTGCAACTTATTACGGTATACAGCATAATAGTTACCCTCCGATCACCGTCGGCCAAGGGACACGACTGGAGATTAAA | GAGGTGCAGTTACTGGAGTCTGGGGGAGGCTGGGTACAGTCTGGGGGGTCCCTGAGACTCTCCTGTGCAGCCTCTGGATTCACCTTTAGCAGCAATGCCATGAGCTGGGTCCGCCAGGCTCCAGGGAAGGGGCTGGAGTGGGTCTCAGACATTAGTCGTAGTGGTGGTACCACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATGGGATAGCAGCAGCTGGTACGACGTGACTCCCTTTGACTACTGGGGCCAGGGAACCCTGGTCACCGTCTCCTCA |
| | | | SEQ ID NO: 25703 | SEQ ID NO: 29709 |

FIGURE 50

| | | | | |
|---|---|---|---|---|
| | | AA | DIQMTQSPSSLYASVGDRVTITRRASQDIRNDLGWYQQKPGKALKRVIYAASSLQSGVPSSFSGSGSGTEFTLTISSLQPEDFATYYGIQHNSYPPITVGQGTRLEIK | EVQLLESGGGWVQSGGSLRLSCAASGFTFSSNAMSWVRQAPGKGLEWVSDISRSGGTTFYADSVKGRFTISRDNSKNTLYLQMNSLRAEDTAVYYCAKWDSSSWYDVTPFDYWGQGTLVTVSS |
| | | | SEQ ID NO: 25704 | SEQ ID NO: 29710 |
| iPS:434469 | 21-225_73C9 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGCAATTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTATATTACTGTGCGAGAGAAAGGTATAGCAGCAGCTGGTTCGACTACGGTATGGACGTCTGGGGCCAAGGGACCACGGTCACCGTCTCCTCA |
| | | | SEQ ID NO: 25705 | SEQ ID NO: 29711 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPRTFGQGTKVEIK | QVQLVESGGGVVQPGRSLRLSCAASGFTFSNYVMHWVRQAPGKGLEWVAVIWYDGSNKYYADSVKGRFTISRDNSKNTLYLQMNSLRAEDTAVYYCARERYSSSWFDYGMDVWGQGTTVTVSS |
| | | | SEQ ID NO: 25706 | SEQ ID NO: 29712 |

FIGURE 50

| | | | | |
|---|---|---|---|---|
| **iPS:434471** | **21-225_75G3** | NA | GAAATTGTGTTGACGCAGTCGCCAGGCACCCTGTCTTTGTCTCCAGGGGAGAGAGCCACCCTCTCCTGCAGGGCCCGTCAGAATGTTGACAGCAGCTACTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTTTTACTGTCAGCAGTATGAACGCTCACCGTGGACGTTCGGCCAAGGGACCAAGGTGGAGATCAAA | CAGGTGCAGCTACAGCAGTGGGGCGCAGGACTGTTGAAGCCTTCGGAGACCCTGTCCCTCACCTGCGCTGTCTATGGTGGGTCCCTCAGTGGTTCCTACTGGAGCTGGATCCGCCAGCCCCCAGGGAAGGGGCTGGAGTGGATTGGGGAAATCAATCTTAGTGGAAGTACCAACTACAACCCGTCCCTCAAGAGTCGAGTCACCATATCAGTAGACACGTCCAAGAGCCAGTTCTCCCTGACGCTGCGCTCTGTGACCGCCGCGGACACGGCTGTGTATTACTGTGCGAGAGACTACGGTGGCCTTGACTACTGGGGCCAGGGAACCCTGGTCACCGTCTCCTCA |
| | | | SEQ ID NO: 25707 | SEQ ID NO: 29713 |
| | | AA | EIVLTQSPGTLSLSPGERATLSCRARQNVDSSYLAWYQQKPGQAPRLLIYGASSRATGIPDRFSGSGSGTDFTLTISRLEPEDFAVFYCQQYERSPWTFGQGTKVEIK | QVQLQQWGAGLLKPSETLSLTCAVYGGSLSGSYWSWIRQPPGKGLEWIGEINLSGSTNYNPSLKSRVTISVDTSKSQFSLTLRSVTAADTAVYYCARDYGGLDYWGQGTLVTVSS |
| | | | SEQ ID NO: 25708 | SEQ ID NO: 29714 |
| **iPS:434473** | **21-225_76D1** | NA | GAAATTGTGTTGACGCAGTCTCCAGGCACCCTGTCTTTGTCTCCAGGGGAAAGAGCCACCCTCTCCTGCAGGGCCAGTCAGAATATTTACAGCAACTACCTAGCCTGGTACCAGGAGAAGCCTGGCCAGGCTCCCAGACTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGTAGTGTATTACTGTCAGCAGTATGAAAGTTCACCGTGGACGTTCGGCCAAGGGACCAAGGTGGAAATCAAA | CAGGTACAACTACAGCAGTGGGGCGCAGGACTGTTGAAGCCTTCGGAGACCCTGTCCCTCACCTGCGCTGTCTATAGTGGGTCCTTCAGTGGTTGCTACTGGAGCTGGATCCGCCAGCCCCCAGGGAAGGGGCTGGAGTGGATTGGGGAAATCAATCATAGTGGAAGAACCAACTACAACCCGTCCCTCAAGAGTCGAGTCACCATATCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAGGGGACCACGGTCACCGTCTCCTCA |
| | | | SEQ ID NO: 25709 | SEQ ID NO: 29715 |

FIGURE 50

| | | | | |
|---|---|---|---|---|
| | | AA | EIVLTQSPGTLSLSPGERATLSCRASQNIYSNYLAWYQEKPGQAPRLLIYGASSRATGIPDRFSGSGSGTDFTLTISRLEPEDFVVYYCQQYESSPWTFGQGTKVEIK | QVQLQQWGAGLLKPSETLSLTCAVYSGSFSGCYWSWIRQPPGKGLEWIGEINHSGRTNYNPSLKSRVTISVDTSKNQFSLKLSSVTAADTAVYYCARDYGGMDVWGQGTTVTVSS |
| | | | SEQ ID NO: 25710 | SEQ ID NO: 29716 |
| iPS:434475 | 21-225_74F9 | NA | GACATCGTGATGACCCAGTCTCCAGACTCCCTGGCTGTGTCTCTGGGCGAGAGGGCCACCATCAACTGCAAGTCCAGCCAGAGTGTTTTATACAGCTCCAACAATTACAACTACTTAGCTTGGTACCAGCAGAAACCAGGACAGCCTCCTAAGAAGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTCCTGTCAGCAATATTATAGTAGTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTGTGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGAGTAGCAGTGGCTGGAACTTCTTTGACTACTGGGGCCAGGGAACCCTGGTCACCGTCTCCTCA |
| | | | SEQ ID NO: 25711 | SEQ ID NO: 29717 |
| | | AA | DIVMTQSPDSLAVSLGERATINCKSSQSVLYSSNNYNYLAWYQQKPGQPPKKLIYWASTRESGVPDRFSGSGSGTDFTLTISSLQAEDVAVYSCQQYYSSPPTFGQGTKVEIK | QVQLVQSGAEVKKPGASVKVSCKASGYTFTNYDINWVRQATGQGLEWMGWMNPNSGNTGCAQKFQGRVTMTRNTSISTAYMELSSLRSEDTAVYYCASSSGWNFFDYWGQGTLVTVSS |
| | | | SEQ ID NO: 25712 | SEQ ID NO: 29718 |

FIGURE 50

| | | | | |
|---|---|---|---|---|
| **iPS:434477** | **21-225_74A6** | NA | GACATCGTGATGACCCAGTCTCCAGACTCCCT GGCTGTGTCTCCGGGCGAGAGGGCCACCATCA ACTGCAAGTCCAGCCAGAGTGTTTTACACAGC TCCAACAATAACAATTACTTAGCCTGGTACCA GCAGAAACCAGGACAGCCTCCTGACCTGCTCA TTTACTGGGCATCAACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTTTAGTACTCCGTGGACGTTCGGCCAAGG GACCCAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCGGGGCAG AGTCACCATGACCTGGAACACCTCCATAAGCACA GCCTACATGGAGCTGAGCAGCCTGAGATCTGAG GACACGGCCGTGTATTACTGTGCGAGTAGCAGTG GCTGGTACTACTTTGACTACTGGGGCCAGGGAAC CCTGGTCACCGTCTCCTCA |
| | | | SEQ ID NO: 25713 | SEQ ID NO: 29719 |
| | | AA | DIVMTQSPDSLAVSPGERATINCKSSQSVLHSSN NNNYLAWYQQKPGQPPDLLIYWASTRESGVPD RFSGSGSGTDFTLTISSLQAEDVAVYYCQQYFST PWTFGQGTQVEIK | QVQLVQSGAEVKKPGASVKVSCKASGYTFTNYDIN WVRQATGQGLEWMGWMHPNSGNTGYAQKFRGR VTMTWNTSISTAYMELSSLRSEDTAVYYCASSSGW YYFDYWGQGTLVTVSS |
| | | | SEQ ID NO: 25714 | SEQ ID NO: 29720 |
| **iPS:434479** | **21-225_76H1** | NA | GAATTTATGTTGACGCAGTCTCCAGGCACCCT GTATTGGTCTCCAGGGGAAAGAGCCACCCTCT CGTGCAGGGCCAGTCAGAGTGTTAGCAGCAGC TACTTAGTCTGGTATCAGCAGAAACCTGGCCA GGCTCCCAGGCTCCTCATCTATGGTGCATCCA CCAGGGCCACTGGCATCCCAGACAGGTTCAGT GGCAGTGGGTCTGGGACAGACTTCACTCTCAC CATCAGCAGACTGGAGCCTGAATATTTTGCAG TTTATTACTGTCAGCAGTATGGTTGCTCACCGC TCACTTTCGGCGGAGGGACCAAGGTGGAGATC ACA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGTCCCTGGACAAGGGC TTGAGTGGATGGGATGGATGCACCCTAACAGTGG TAACACAGGCTATGCACAGAAGTTCCAGGGCAG AGTCACCATGACCAGGAACACCTCCATCAGCAC AGCCTACATGGAGCTGAGCAGCCTGAGATCTGA GGACACGGCCGTGTATTACTGTGCGATTAGTAGT GGCTGGTACTGGTTCGACCCCTGGGGCCAGGGA ACCCTGGTCACCGTCTCCTCA |
| | | | SEQ ID NO: 25715 | SEQ ID NO: 29721 |

FIGURE 50

| | | | | |
|---|---|---|---|---|
| | | AA | EFMLTQSPGTLYWSPGERATLSCRASQSVSSSYLVWYQQKPGQAPRLLIYGASTRATGIPDRFSGSGSGTDFTLTISRLEPEYFAVYYCQQYGCSPLTFGGGTKVEIT | QVQLVQSGAEVKKPGASVKVSCKASGYTFTNYDINWVRQVPGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25716 | SEQ ID NO: 29722 |
| iPS:434481 | 21-225_74B10 | NA | GACATCGTGATGACCCAGTCTCCAGACTCCCTGGCTGTGTCTCTGGGCGAGAGGGCCACCATCAACTGCAAGTCCAGCCAGAGTGTTTTACACAGCTCCAACAATAAGAACTACTTAACTTGGTACCAGCAGAAACCAGGACAGCCTCCTAAGCTGCTCATTTACTGGGCATCTACTCGGGAATCCGGGGTCCCTGACCGATTCAGTGGCAGCGGGTCTGGGACAGATTTCAGTCTCACGATCGGCAGCCTGCAGGCTGAAGATGTGGCAGTTTATTACTGTCAGCAATATTATAGTATTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATTCACCTTCACCAATTATGATATCAACTGGGTGCGACAGGCCACTGGACAAGGGCTTGAGTGGATGGGATGGATGCACCCTAACAGTGGTAACACAGGCTTTGCACAGAAGTTCCAGGGCAGAGTCACCATGACCAGGAACACCTCCATAAGCACAGCCTACATGGAACTGAGCAGCCTGAGATCTGAGGACACGGCCGTGTATTACTGTGCGGTTTCCAGTGGCTGGTACTGGTTCGACCCCTGGGGCCAGGGAACCCTGGTCACCGTCTCCTCA |
| | | | SEQ ID NO: 25717 | SEQ ID NO: 29723 |
| | | AA | DIVMTQSPDSLAVSLGERATINCKSSQSVLHSSNNKNYLTWYQQKPGQPPKLLIYWASTRESGVPDRFSGSGSGTDFSLTIGSLQAEDVAVYYCQQYYSIPPTFGQGTKVEIK | QVQLVQSGAEVKKPGASVKVSCKASGFTFTNYDINWVRQATGQGLEWMGWMHPNSGNTGFAQKFQGRVTMTRNTSISTAYMELSSLRSEDTAVYYCAVSSGWYWFDPWGQGTLVTVSS |
| | | | SEQ ID NO: 25718 | SEQ ID NO: 29724 |

FIGURE 50

| | | | | |
|---|---|---|---|---|
| iPS:434483 | 21-225_74C12 | NA | GACATCGTGATGACCCAGTCTCCAGACTCCCTGGCTGTGTCTCTGGGCGAGAGGGCCACCATCAACTGCAAGTCCAGCCAGAGTGTTTTATACAGCTCCAACAATGCGAACTACTTAGCTTGGTACCAGCAGAAACCAGGACAGCCTCCTAACCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTCA |
| | | | SEQ ID NO: 25719 | SEQ ID NO: 29725 |
| | | AA | DIVMTQSPDSLAVSLGERATINCKSSQSVLYSSNNANYLAWYQQKPGQPPNLLIYWASTRESGVPDRFSGSGSGTDFTLTISSLQAEDVAVYYCQQYYSTPCSFGQGTKLEIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYYCAISSGWYWFDPWGQGTLVTVSS |
| | | | SEQ ID NO: 25720 | SEQ ID NO: 29726 |
| iPS:434485 | 21-225_76D2 | NA | GAAATAGTGATGACGCAGTCTCCAGCCACCCCGTCTGTGTCTCCAGGGGAAAGAGCCACCCTCTCCTGCAGGGCCAGTGTGAGTGTTGTCAACAGCTTAGCCTGGTACCAGCAGAAACCTGGCCAGGCTCCCAGGCTCCTCATCCATGGTGCATCCACCAGGGCCACTGGTATCCCAGCCAGGTTCAGTGGCAGTGGGTCTGGGACAGAGTTCACTCTCACCATCAGCAGTGTGCAGTCTGAAGATTTTGCAATTTATTACTGTCAGCAATATAATGACTGGCCGTGCAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATTTGGTATGATGGAAGTAATAAATACTATGCAGACTCCGTGAAGGGCCGATTCACCATCTCCAGAGACAATTCCAAGAACACGCTGTTTCTGCAAATGAACAGCCTGAGAGCCGAGGACACGGCTGTGTATTACTGTGCGAGCGATCGCAATATAGTGGGAGCTACTTACTTTGAGTCCTGGGGCCAGGGAACCCTGGTCACCGTCTCCTCA |
| | | | SEQ ID NO: 25721 | SEQ ID NO: 29727 |

FIGURE 50

| | | | | |
|---|---|---|---|---|
| | | AA | EIVMTQSPATPSVSPGERATLSCRASVSVVNSLAWYQQKPGQAPRLLIHGASTRATGIPARFSGSGSGTEFTLTISSVQSEDFAIYYCQQYNDWPCSFGQGTKLEIK | QVQLVESGGGVVQPGRSLRLSCAASGFTFSSYGMHWVRQAPGKGLEWVAVIWYDGSNKYYADSVKGRFTISRDNSKNTLFLQMNSLRAEDTAVYYCASDRNIVGATYFESWGQGTLVTVSS |
| | | | SEQ ID NO: 25722 | SEQ ID NO: 29728 |
| **iPS:434487** | **21-225_76G2** | NA | GACATCGTGATGACCCAGTCTCCAGACTCCCTGGCTGTGTCTCTGGGCGAGAGGGCCACCATCAACTGCAAGTCCAGCCAGAGTGTTTTACACAGCTCCAACAATTACAACTACTTAGCTTGGTACCAGCAGAGACCAGGACAGCCTCCTAGGCTGCTCATTTACTGGGCATCTACCCGGGAATCCGGGGTCCCTGACCGATTCAGTGGCAGCGGTTCTGGGACAGATTTCACTCTCACCATCAGCAGCCTGCAGGTTGAAGATGTGGCAGTTTATTACTGTCAGCAATATTATAGTTCTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGACTTGAGTGGATGGGATGGATGAACCCTAACAGTGGTAACACAGGCTATGCACAGAAGTTCCAGGGCAGAGTCACCATGACCAGGAACACCTCCATAAGCACAGCCTATATGGAGCTGAGCAGCCTGAGATCTGAAGACACGGCCGTGTATTACTGTGCGGGTAGCAGTGGCTGGTACATGTTTGACTACTGGGGCCAGGGAACCTCTGGTCACCGTCTCCTCA |
| | | | SEQ ID NO: 25723 | SEQ ID NO: 29729 |
| | | AA | DIVMTQSPDSLAVSLGERATINCKSSQSVLHSSNNYNYLAWYQQRPGQPPRLLIYWASTRESGVPDRFSGSGSGTDFTLTISSLQVEDVAVYYCQQYYSSPPTFGQGTKVEIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYYCAGSSGWYMFDYWGQGTLVTVSS |
| | | | SEQ ID NO: 25724 | SEQ ID NO: 29730 |

FIGURE 50

| | | | | |
|---|---|---|---|---|
| **iPS:434489** | **21-225_74E4** | NA | GACATCCAGATGACCCAGTCTCCATCCTCCCTGTCTGCTTCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCACTTTGCAAAGTGGGGTCCCATCAAGGTTCAGCGGCAGTGGATCTGGGACAGAATTCACTCTCACAATCAGCGGCCTGCAGCCTGAAGATTTTGCAACTTATCACTGTCTACAGCATAGTAATTACCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCATCTGTACTGTCTCTGGTGGCTCCATCAGCAGTAGTAATTACTACTGGGGCTGGATCCGCCAGCCCCCAGGGAAGGGGCTGGAGTGGATTGGGAGTATCTATTATAGTGGATACACCTCCTACAACCCGTCCCTCAAGAGTCGAGTCACCATATCCGTAGACTCGTCCAAGAACCACTTCTCCCTGAGGCTGAGCTCTGTGACCGCCGCAGACACGGCTGTGTATTACTGTGCGAGACTTGACTCTAACTGGGGTCTTGACTACTGGGGCCAGGGAACCCTGGTCACCGTCTCCTCA |
| | | | SEQ ID NO: 25725 | SEQ ID NO: 29731 |
| | | AA | DIQMTQSPSSLSASVGDRVTITCRASQGIRNDLGWYQQKPGKAPKRLIYAASTLQSGVPSRFSGSGSGTEFTLTISGLQPEDFATYHCLQHSNYPLTFGGGTKVEIK | QLQLQESGPGLVKPSETLSLICTVSGGSISSSNYYWGWIRQPPGKGLEWIGSIYYSGYTSYNPSLKSRVTISVDSSKNHFSLRLSSVTAADTAVYYCARLDSNWGLDYWGQGTLVTVSS |
| | | | SEQ ID NO: 25726 | SEQ ID NO: 29732 |
| **iPS:434493** | **21-225_76F3** | NA | GACATCGTTATGACCCAGTGTCCAGACTCCCCGGCTGTGTCTCTGGGCGAGAGGGCCACCATCAATTGCAAGTCCAGCCAGAGTGTTTTATTCAGCTCCAACAATTATAATTACTTAGCTTGGTACCAGCAGAGACCAGGACAGCCTCATGACCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGCTGATGTGGCAGTTTATTACTGTCAGCAATATCATAGTTCTCCTCTGACGTTCGGCCAAGGGACCACGGTGCAAATCAAA | CAGGTGCAGTTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCACAAGCACAGCCCACATGGAGCTGAGCAGCCTGAGATCTGAGGACACGGCCGTGTATTACTGTGCGGTCTCCAGTGGCTGGAACTGGTTCGACCCCTGGGGCCAGGGAACCCTGGTCACCGTCTCCTCA |
| | | | SEQ ID NO: 25727 | SEQ ID NO: 29733 |

FIGURE 50

| | | | | |
|---|---|---|---|---|
| | | AA | DIVMTQCPDSPAVSLGERATINCKSSQSVLFSSNNYNYLAWYQQRPGQPHDLLIYWASTRESGVPDRFSGSGSGTDFTLTISSLQAADVAVYYCQQYHSSPLTFGQGTTVQIK | QVQLVQSGAEVKKPGASVKVSCKASGYTFTNYDINWVRQATGQGLEWMGWMNPNSGNTGYAQKFQGRVTMTRNTSTSTAHMELSSLRSEDTAVYYCAVSSGWNWFDPWGQGTLVTVSS |
| | | | SEQ ID NO: 25728 | SEQ ID NO: 29734 |
| iPS:434495 | 21-225_74B2 | NA | GAACTTGTGTTGACGCAGTCTCCAGGCACCCTGTCTTTGTCTCCAGGGGAAAGAGCCACCCTCTCCTGCAGGGCCAGTCAGAATATTTACAGCAGTTACTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTATTGCTGTCAGCAGTATGAAAGCTCACCGTGGACCTTCGGCCAAGGGACCAAGGTGGAAATCAAA | CAAGTGCAGCTACAGCAGTGGGGCGCAGGACTGTTGAAGCCTTCGGAGCCCCTGTCCCTCACCTGCGCTGTCTATGGTGGGTCCTTCAGTGGTCCCTACTGGAGCTGGATCCGCCAGCCCCCCGGGAAGGGGCTGGAGTGGATTGGGGAAATCAATCATAGTGGAAGCACCAACTACAACCCGTCCCTCACGAGTCGAGTCACCATATCAGTAGACACGTCCAAGAACCAGTTCTCCCTGAAACTGACCTCTGTGACCGCCGCGGACTCGGCTGTGTATTACTGTGCGAGAGACTACGGGGGTTTGGACGTCTGGGGCCAAGGGACCACGGTCACCGTCTCCTCA |
| | | | SEQ ID NO: 25729 | SEQ ID NO: 29735 |
| | | AA | ELVLTQSPGTLSLSPGERATLSCRASQNIYSSYLAWYQQKPGQAPRLLIYGASSRATGIPDRFSGSGSGTDFTLTISRLEPEDFAVYCCQQYESSPWTFGQGTKVEIK | QVQLQQWGAGLLKPSEPLSLTCAVYGGSFSGPYWSWIRQPPGKGLEWIGEINHSGSTNYNPSLTSRVTISVDTSKNQFSLKLTSVTAADSAVYYCARDYGGLDVWGQGTTVTVSS |
| | | | SEQ ID NO: 25730 | SEQ ID NO: 29736 |

FIGURE 50

| | | | | |
|---|---|---|---|---|
| **iPS:434497** | **21-225_76A4** | NA | GAAATTGTGTTGACGCAGTCTCCAGGCACCCT<br>GTATTTGTCTCCAGGGGAAAGAGCCACCCTCT<br>CATGCAGGGCCAGTCAGAGTGTTTACAGCAGC<br>TACTTAGCCTGGTACCAGCAGAAACCTGGCCA<br>GGCTCCCAGGCTCCTCATTTATGGTGCATCCA<br>GCCGGGCCACTGGCATCCCAGACAGGTTCAGT<br>GGCAGTGGGTCTGGGACAGACTTCGCTCTCAC<br>CATCAGCAGACTGGAGCCTGAAGATTTTGCAG<br>TGTATTACTGTCAGCACTCTGATAACTCACCGT<br>GGACGTTCGGCCAAGGGACCAAGGTGGAAAT<br>CAAA | CAGGTGCAGCTACAGCAGTGGGGCGCAGGACTG<br>TTGAAGCCTTCGGAGACCCTGTCCCTCACCTGCG<br>CTGTCCATGGTGGGTCCTTCAGTGGTTGCTACTG<br>GAGCTGGATCCGCCAGCCCCCAGGGAAGGGGCT<br>GGAGTGGATTGGGGAAATCAATCATAGTGGAAG<br>CACCAACTACAACCCGTCCCTCAAGAGTCGAGTC<br>ACCATTTCAGTAGACACGTCCAAGAACCAGTTCT<br>CCCTGAAGCTGAGCTCTGTGACCGCCGCGGACAC<br>GGCTGTGTATTACTGTGCGAGGGACTACGGCGGT<br>ATGGACGTCTGGGGCCAAGGGACCACGGTCACC<br>GTCTCCTCA |
| | | | SEQ ID NO: 25731 | SEQ ID NO: 29737 |
| | | AA | EIVLTQSPGTLYLSPGERATLSCRASQSVYSSYLA<br>WYQQKPGQAPRLLIYGASSRATGIPDRFSGSGSG<br>TDFALTISRLEPEDFAVYYCQHSDNSPWTFGQGT<br>KVEIK | QVQLQQWGAGLLKPSETLSLTCAVHGGSFSGCYW<br>SWIRQPPGKGLEWIGEINHSGSTNYNPSLKSRVTISV<br>DTSKNQFSLKLSSVTAADTAVYYCARDYGGMDV<br>WGQGTTVTVSS |
| | | | SEQ ID NO: 25732 | SEQ ID NO: 29738 |
| **iPS:434501** | **21-225_76G4** | NA | GAAATTGTGTTGACGCAGTCTCCAGGCACCCT<br>GTCTTTGTCTCCAGGGGAAAGAGCCACCCTCT<br>CATGCAGGGCCAGTCAGAGTGTTTACAGCAGC<br>TACTTAGCCTGGTACCAGCAGAAACCTGGCCA<br>GGCTCCCAGGCTCCTCATTTATGGTGCATCCA<br>GCCGGGCCACTGGCATCCCAGACAGGTTCAGT<br>GGCAGTGGGTCTGGGACAGACTTCGCTCTCAC<br>CATCAGCAGACTGGAGCCTGAAGATTGTGCAG<br>TGTATTACTGTCAGCACTCTGATAACTCACCGT<br>GGACGTTCGGCCAAGGGACCAAGGTGGAAAT<br>CAAA | CAGGTGCAGCTACAGCAGTGGGGCGCAGGACTG<br>TTGAAGCCTTCGGAGACCCTGTCCCTCACCTGCG<br>CTGTCCATGGTGGGTCCTTCAGTGGTTGCTACTG<br>GAGCTGGATCCGCCAGCCCCCAGGGAAGGGGCT<br>GGAGTGGATTGGGGAAATCAATCATAGTGGAAG<br>CACCAACTACAACCCGTCCCTCAAGAGTCGAGTC<br>ACCATTTCAGTAGACACGTCCAAGAACCAGTTCT<br>CCCTGAAGCTGAGCTCTGTGACCGCCGCGGACAC<br>GGCTGTGTATTACTGTGCGAGGGACTACGGCGGT<br>ATGGACGTCTGGGGCCAAGGGACCACGGTCACC<br>GTCTCCTCA |
| | | | SEQ ID NO: 25733 | SEQ ID NO: 29739 |

FIGURE 50

| | | | | |
|---|---|---|---|---|
| | | AA | EIVLTQSPGTLSLSPGERATLSCRASQSVYSSYLAWYQQKPGQAPRLLIYGASSRATGIPDRFSGSGSGTDFALTISRLEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25734 | SEQ ID NO: 29740 |
| **iPS:434503** | **21-225_74D7** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATTTGGGACAGAATTCACTCTCACCATCAGCAGCCTGCAGCCTGAAGATTTTGCAACTTATTACTGTCTTCAGCATAGTAATTACCCGCTCACTTTCGGCGGAGGGACCAAGGTGGAGATCAAA | CAACTGCAGCTGCAGGAGTCGGGCCCAGGACTGGTGAAGCCTTCGGAGACCCTGTCCCTCACCTGCAGTGTCTCTGGTGGCTCCATCTTCAGAAGTAGTTACTACTGGGGCTGGATCCGCCAGCCCCCAGGGAAGGGGCTGGAGTGGATTGGGGGTATCTATTATAGTGGGAGCACCTCCTACAACCCGTCCCTCAAGAGTCGAGTCACCATATCCGTAGACACGTCCGAGAACCAGTTCTCCCTGAAGCTGAGCTCTGTGACCGCCGCAGACACGGCTGTGTATTACTGTGCGAGACTGCGACCTAACTGGGACTTTGACTACTGGGGCCAGGGAACCCTGGTCACCGTCTCCTCA |
| | | | SEQ ID NO: 25735 | SEQ ID NO: 29741 |
| | | AA | DIQMTQSPSSLSASVGDRVTITCRASQGIRNDLGWYQQKPGKAPKRLIYAASSLQSGVPSRFSGSGFGTEFTLTISSLQPEDFATYYCLQHSNYPLTFGGGTKVEIK | QLQLQESGPGLVKPSETLSLTCSVSGGSIFRSSYYWGWIRQPPGKGLEWIGGIYYSGSTSYNPSLKSRVTISVDTSENQFSLKLSSVTAADTAVYYCARLRPNWDFDYWGQGTLVTVSS |
| | | | SEQ ID NO: 25736 | SEQ ID NO: 29742 |

FIGURE 50

| | | | | |
|---|---|---|---|---|
| **iPS:434507** | **21-225_74C5** | NA | GAAATTGTGTTGACGCAGTCTCCAGGCACCCTGTCTTTGTCTCCAGGGGAAAGAGCCACCCTCTCTTGCAGGGCCAGTCAGAGTGTTAACAGCAACTACTTAGCCTGGTACCAGCAGAAACCTGGCCAGGCTCCCAGGCTCCTCATCTATGGTGCATTCAGCAGGGCCACTGGCATCCCAGACAGGGTCAGTGGCAGTGGGTCTGGGACAGACTTCAATCTCATCATCAGCAGACTGGAGCCTGAAGATTTTGCAGTGTATTACTGTCAGCAGTATGAAAGCTCACCGTGGACGTTCGGCCAAGGGACCAAGGTGGAAATCAAA | CAGGTGCAGCTACAACAGTGGGGCGCAGGACTGTTGAAGCCTTCGGAGACCCTGTCCCTCACTTGCGCTGTCTATGGTGGGTCCTTCAGTGGTTGCTACTGGAGCTGGATCCGCCAGCCCCCAGGGAAGGGGCTGGAGTGGATTGGGGAAATCAATCATAGTGGATGCACCAACTTCAACCCGTCCCTCAAGAGTCGAGTCACCATATCAGTTGACACGTCCAAGAACCAGTTCTCCCTGAAGCTGAGCTCTGTGACCGCCGCGGACACGGCTGTGTATTACTGTGCGAGAGACTACGGCGGTATGGACGTCTGGGGCCAAGGGACCACGGTCACCGTCTCCTCA |
| | | | SEQ ID NO: 25737 | SEQ ID NO: 29743 |
| | | AA | EIVLTQSPGTLSLSPGERATLSCRASQSVNSNYLAWYQQKPGQAPRLLIYGAFSRATGIPDRVSGSGSGTDFNLIISRLEPEDFAVYYCQQYESSPWTFGQGTKVEIK | QVQLQQWGAGLLKPSETLSLTCAVYGGSFSGCYWSWIRQPPGKGLEWIGEINHSGCTNFNPSLKSRVTISVDTSKNQFSLKLSSVTAADTAVYYCARDYGGMDVWGQGTTVTVSS |
| | | | SEQ ID NO: 25738 | SEQ ID NO: 29744 |
| **iPS:434509** | **21-225_76F5** | NA | GTCATCGTGTTGACCCAGTCTCCAGACTCCCTGGCTGTGTCTCTGGGCGAGAGGGCCACCATCAACTGCAAGTCCAGCCAGAGTGTATTACACAGCTCCAACAGTTACAACTACTTAGCTTGGTACCAGCAGAAACCAGGACAGTCTCCTAAGGTGCTCATTTACTGGACATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTAGTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTATATGGAGCTGAGCAGCCTGAGATCTGAGGACACGGCCGTGTATTACTGTGCGAGTAGCAGTGGCTGGTACTGGTTCGACCCCTGGGGCCAGGGAACCCTGGTCACCGTCTCCTCA |
| | | | SEQ ID NO: 25739 | SEQ ID NO: 29745 |

FIGURE 50

| | | | | |
|---|---|---|---|---|
| | | AA | VIVLTQSPDSLAVSLGERATINCKSSQSVLHSSNSYNYLAWYQQKPGQSPKVLIYWTSTRESGVPDRFSGSGSGTDFTLTISSLQAEDVAVYYCQQYYSSPPTFGQGTKVEIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYYCASSSGWYWFDPWGQGTLVTVSS |
| | | | SEQ ID NO: 25740 | SEQ ID NO: 29746 |
| iPS:434511 | 21-225_74B11 | NA | GATATCGTGATGACCCAGTCTCCAGACTCCCTGACTGTGTCTCTGGGCGAGAGGGCCACCATCAACTGCAAGTCCAGCCAGAGTATTTTATACAACTCCAACAATAACAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCATTCTCACCATCAGCAGCCTGCAGGCTGAAGATGTGGCAGTTTACTACTGTCAGCAATATTATAGCACTCCTCCTACTTTCGGCGGAGGGACCAAGGTGGAGATCAAA | CAGGTGCAGCTGGTGCAGTCTGGGGCTGAGGTGAAGAAGCCTGGGGCCTCAGTGAGGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGTATAGCAGTGGCTGGTACTACTTTGACTACTGGGGCCAGGGAACCCTGGTCACCGTCTCCTCA |
| | | | SEQ ID NO: 25741 | SEQ ID NO: 29747 |
| | | AA | DIVMTQSPDSLTVSLGERATINCKSSQSILYNSNNNNYLAWYQQKPGQPPKLLIYWASTRESGVPDRFSGSGSGTDFILTISSLQAEDVAVYYCQQYYSTPPTFGGGTKVEIK | QVQLVQSGAEVKKPGASVRVSCKASGYTFTNYDINWVRQATGQGLEWMGWMHPNSGNTGYAQKFQGRVTMTRNTSISTAYMELSSLRSEDTAVYYCAYSSGWYYFDYWGQGTLVTVSS |
| | | | SEQ ID NO: 25742 | SEQ ID NO: 29748 |

FIGURE 50

| | | | | |
|---|---|---|---|---|
| **iPS:434513** | **21-225_76A6** | NA | GAAATTGTGTTGACGCAGTCTCCAGGCACCCGGTCTTGGTCTCCAGGGGAAAGAGCCACCCTCTCGTGCAGGGCCAGTCAGAGTGTTAGCAGCAGCTACTTAGCCTGGTACCAGCAGAAACCTGGCCAGGCTCCCAGGCTCCTCATCTATGGTGCATCCACCAGGGCCACTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTAACTCACCGCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAATGGTAACACAGGCTATGCACAGAAGTTCCAGGGCAGAGTCACCATGACCAGGAACACCTCCATAAGCACAGCCTACATGGAGCTGAACAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTTA |
| | | | SEQ ID NO: 25743 | SEQ ID NO: 29749 |
| | | AA | EIVLTQSPGTRSWSPGERATLSCRASQSVSSSYLAWYQQKPGQAPRLLIYGASTRATGIPDRFSGSGSGTDFTLTISRLEPEYFAVYYCQQYGNSPLTFGGGTKVEIK | QVQLVQSGAEVKKPGASVKVSCKASGYTFTNYDINWVRQATGQGLEWMGWMHPNNGNTGYAQKFQGRVTMTRNTSISTAYMELNSLRSEDTAVYYCAISSGWYWFDPWGQGTLVTVSL |
| | | | SEQ ID NO: 25744 | SEQ ID NO: 29750 |
| **iPS:434515** | **21-225_74A5** | NA | GAATTTATGTTGACGCAGTCTCCAGGCACCCTGTCTTTGTCTCCAGGGGAAAGAGCCACCCTCTCGTGCAGGGCCAGTCAGAGTGTTAGCAGCAGCTACTTAGTCTGGTATCAGCAGAAACCTGGCCAGGCTCCCAGGCTCCTCATCTATGGTGCATCCACCAGGGCCACTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTTGCTCACCGCTCACTTTCGGCGGAGGGACCAAGGTGGAGATCACA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGTCCCTGGACAAGGGCTTGAGTGGATGGGATGGATGCACCCTAACAGTGGTAACACAGGCTATGCACAGAAGTTCCAGGGCAGAGTCACCATGACCAGGAACACCTCCATCAGCACAGCCTACATGGAGCTGAGCAGCCTGAGATCTGAGGACACGGCCGTGTATTACTGTGCGATTAGTAGTGGCTGGTACTGGTTCGACCCCTGGGGCCAGGGAACCCTGGTCACCGTCTCCTCA |
| | | | SEQ ID NO: 25745 | SEQ ID NO: 29751 |

FIGURE 50

| | | | | |
|---|---|---|---|---|
| | | AA | EFMLTQSPGTLSLSPGERATLSCRASQSVSSSYLVWYQQKPGQAPRLLIYGASTRATGIPDRFSGSGSGTDFTLTISRLEPEYFAVYYCQQYGCSPLTFGGGTKVEIT | QVQLVQSGAEVKKPGASVKVSCKASGYTFTNYDINWVRQVPGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25746 | SEQ ID NO: 29752 |
| iPS:434517 | 21-225_76A7 | NA | GAAATTGCGTTGACGCAGTCTCCAGGCACCCTGTCTTTGTCTCCAGGGGAAAGAGCCACCCTCTCCTGCAGGGCCAGTCCGAGTGTTGACAGCAGCTACTTAGCCTGGTACCAGCAGAGACCTGGCCAGGCTCCCAGGCTCCTCATCTATGGTGCATCCAGCAGGGCCCCTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGAAAGTTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGGTTGCTACTGGAGCTGGATCCGCCAGCCCCCAGGGAAGGGGCTGGAGTGGATTGGGGAAATCAATCATAGTGGAAGGACCAACTACAACCCGTCCCTCAAGAGTCGAGTCACCATATCAGTAGACACGTCCGAGAACCAGTTCTCCCTGAAGCTGAGCTCTGTGACCGCCGCGGACACGGCTGTGTATTACTGTGCGAGAGACTACGGTGGGCTTGACTACTGGGGCCAGGGAGCCCTGGTCACCGTCTCCTCA |
| | | | SEQ ID NO: 25747 | SEQ ID NO: 29753 |
| | | AA | EIALTQSPGTLSLSPGERATLSCRASPSVDSSYLAWYQQRPGQAPRLLIYGASSRAPGIPDRFSGSGSGTDFTLTISRLEPEDFAVYYCQQYESSPWTFGQGTKVEIK | QVQLQQWGAGLLKPSETLSLTCAVYGGSFSGCYWSWIRQPPGKGLEWIGEINHSGRTNYNPSLKSRVTISVDTSENQFSLKLSSVTAADTAVYYCARDYGGLDYWGQGALVTVSS |
| | | | SEQ ID NO: 25748 | SEQ ID NO: 29754 |

FIGURE 50

| | | | | |
|---|---|---|---|---|
| **iPS:434519** | **21-225_74C7** | NA | GAAATTGTGTTGACGCAGTCGCCAGGCACCCTGTCTTTGTCTCCAGGGGAGAGAGCCACCCTCTCCTGCAGGACCAGTCCGAATGTTGACAGCAGCTACTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTTTTACTGTCAGCAGTATGAACGCTCACCGTGGACGTTCGGCCAAGGGACCAAGGTGGAAATCAAA | CAGGTGCAGTTACAGCAGTGGGGCGCAGGACTGTTGAAGCCTTCGGAGACCCTGTCCCTCACCTGCGCTGTCTATGGTGGGTCCCTCAGTGGTTCCTACTGGAGCTGGATCCGCCAGCCCCCAGGGAAGGGGCTGGAGTGGATTGGGGAAATCAATCTTAGTGGAAGCACCAACTACAACCCGTCCCTCAAGAGTCGAGTCACCATATCAGTAGACACGTCCAAGAGCCAGTTCTCCCTGACGCTGCGCTCTGTGACCGCCGCGGACACGGCTGTGTATTACTGTGCGAGAGACTACGGTGGCCTTGACTACTGGGGCCAGGGAACCCTGGTCACCGTCTCCTCA |
| | | | SEQ ID NO: 25749 | SEQ ID NO: 29755 |
| | | AA | EIVLTQSPGTLSLSPGERATLSCRTSPNVDSSYLAWYQQKPGQAPRLLIYGASSRATGIPDRFSGSGSGTDFTLTISRLEPEDFAVFYCQQYERSPWTFGQGTKVEIK | QVQLQQWGAGLLKPSETLSLTCAVYGGSLSGSYWSWIRQPPGKGLEWIGEINLSGSTNYNPSLKSRVTISVDTSKSQFSLTLRSVTAADTAVYYCARDYGGLDYWGQGTLVTVSS |
| | | | SEQ ID NO: 25750 | SEQ ID NO: 29756 |
| **iPS:434523** | **21-225_75C3** | NA | GAAATTGTGTTGACGCAGTCTCCAGGCACCCTGTCTTTGTCTCAAGGGGAAAGAGCCACCCTCTCCTGCAGGGCCAGTCAGAGTGTTAGCAGCAGGTACTTAGCCTGGTACCAGCAGAAACCTGGCCAGGCTCCCAGACTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTATATTACTGTCAGCATTATGATAGCTCACCGTGGACGTTCGGCCAAGGGACCAAGGTGGAAATCAAA | CAGGTGCAGCTACAGCAGGGGGGCGCAGGACCGTTGAAGCCTTCGGAGACCCTGTCCCTCACCTGCGCTGTCTATGGTGGGTCCTTCAGTGGTTGCTACTGGAGCTGGATCCGCCAGCCCCCAGGGAAGGGGCTGGAGTGGATTGGGGAAATCAATTATAGTGGAAGAACCAACTACAACCCGTCCCTCAAGAGTCGAGTCACCATATCAGTAGACACGTCCAAGAACCAGTTCTCCCTGAAGCTGAGCTCTGTGACCGCCGCGGACACGGCTGTGTATTACTGTGCGAGAGACTACGGCGGTATGGACGTCTGGGGCCAAGGGACCACGGTCACCGTCTCCTCA |
| | | | SEQ ID NO: 25751 | SEQ ID NO: 29757 |

FIGURE 50

| | | | | |
|---|---|---|---|---|
| | | AA | EIVLTQSPGTLSLSQGERATLSCRASQSVSSRYLAWYQQKPGQAPRLLIYGASSRATGIPDRFSGSGSGTDFTLTISRLEPEDFAVYYCQHYDSSPWTFGQGTKVEIK | QVQLQQGGAGPLKPSETLSLTCAVYGGSFSGCYWSWIRQPPGKGLEWIGEINYSGRTNYNPSLKSRVTISVDTSKNQFSLKLSSVTAADTAVYYCARDYGGMDVWGQGTTVTVSS |
| | | | SEQ ID NO: 25752 | SEQ ID NO: 29758 |
| iPS:434525 | 21-225_76E8 | NA | GACATCGTGATGACCCAGTCTCCAGACTCCCTGGCTGTGTCTCTGGGCGAGAGGGCCACCATCAACTGCAAGTCCAGCCAGACTGTTTTACACAGCTCCAACAATTATAACTACTTAGCTTGGTACCAGCAGAAGCCAGGACAGCCCCCTAAGGTGCTCATTTACTGGACATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTTTAGTAGTCCTCTGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCCCCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTATTGTGCGGTTTCCAGTGGCTGGCACTGGTTCGACCCCTGGGGCCAGGGAACCCTGGTCACCGTCGCCTCA |
| | | | SEQ ID NO: 25753 | SEQ ID NO: 29759 |
| | | AA | DIVMTQSPDSLAVSLGERATINCKSSQTVLHSSNNYNYLAWYQQKPGQPPKVLIYWTSTRESGVPDRFSGSGSGTDFTLTISSLQAEDVAVYYCQQYFSSPLTFGQGTKVEIK | QVQLVQSGAEVKKPGASVKVSCKASGYTFPNYDINWVRQATGQGLEWMGWMNPNSGNTGYAQKFQGRVTMTRNTSISTAYMELSSLRSEDTAVYYCAVSSGWHWFDPWGQGTLVTVAS |
| | | | SEQ ID NO: 25754 | SEQ ID NO: 29760 |

FIGURE 50

| | | | | |
|---|---|---|---|---|
| iPS:434529 | 21-225_76B9 | NA | GAATTTATGTTGACGCAGTCTCCAGGCACCCTGTCTTGGTCTCCAGGGGAAAGAGCCACCCTCTCGTGCAGGGCCAGTCAGAGTGTTAGCAGCAGCTACTTAGTCTGGTATCAGCAGAAACCTGGCCAGGCTCCCAGGCTCCTCATCTATGGTGCATCCACCAGGGCCACTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTTGCTCACCGCTCACTTTCGGCGGAGGGACCAAGGTGGAGATCACA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGTCCCTGGACAAGGGCTTGAGTGGATGGGATGGATGCACCCTAACAGTGGTAACACAGGCTATGCACAGAAGTTCCAGGGCAGAGTCACCATGACCAGGAACACCTCCATCAGCACAGCCTACATGGAGCTGAGCAGCCTGAGATCTGAGGACACGGCCGTGTATTACTGTGCGATTAGTAGTGGCTGGTACTGGTTCGACCCCTGGGGCCAGGGAACCCTGGTCACCGTCTCCTCA |
| | | | SEQ ID NO: 25755 | SEQ ID NO: 29761 |
| | | AA | EFMLTQSPGTLSWSPGERATLSCRASQSVSSSYLVWYQQKPGQAPRLLIYGASTRATGIPDRFSGSGSGTDFTLTISRLEPEYFAVYYCQQYGCSPLTFGGGTKVEIT | QVQLVQSGAEVKKPGASVKVSCKASGYTFTNYDINWVRQVPGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25756 | SEQ ID NO: 29762 |
| iPS:434531 | 21-225_76C9 | NA | GAAATTGTGTTGACGCAGTCTCCAGGCACCCTGTCTTTGTCTCCAGGGGAAAGAGCCACCCTCTCCTGCAGGGCCAGTCAGAGTGTTAGCAGCAGTTACTTATCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGGTAGGTCACGGACGTTCGGCCAAGGGACCAAGGTGGAAATCAGA | GAGGTGCAGCTGGTGGAGTCTGGGGGAGGCTTGGTAAAGCCTGGGGGGTCCCTTAGACTCTCCTGTGCAGCCTCTGGATTCAGTTTCAGTAACGCCTGGATGAACTGGGTCCGCCAGGCTCCAGGGAAGGGGCTGGAGTGGGTTGGCCGTATTAAAAACAAAGCTGATGGTGGGACAACAGACTTCGCTGCACCCGTGAAAGGCAGATTCACCATCTCAAGAGATGATTCAAAACACACGCTGTATCTGCAAATGAACAGCCTGAAAACCGAGGACACAGCCGTGTATTACTGTACCACAGTGGGACCTACTACGGACTACTGGGGCCAGGGAACCCTGGTCACCGTCTCCTCA |
| | | | SEQ ID NO: 25757 | SEQ ID NO: 29763 |

FIGURE 50

| | | | | |
|---|---|---|---|---|
| | | AA | EIVLTQSPGTLSLSPGERATLSCRASQSVSSSYLSWYQQKPGQAPRLLIYGASSRATGIPDRFSGSGSGTDFTLTISRLEPEDFAVYYCQQYGRSRTFGQGTKVEIR | EVQLVESGGGLVKPGGSLRLSCAASGFSFSNAWMNWVRQAPGKGLEWVGRIKNKADGGTTDFAAPVKGRFTISRDDSKHTLYLQMNSLKTEDTAVYYCTTVGPTTDYWGQGTLVTVSS |
| | | | SEQ ID NO: 25758 | SEQ ID NO: 29764 |
| **iPS:434533** | **21-225_85F7** | NA | GAACCTGTGTTGACGCAGTCTCCAGGCACCCTGTCTTTGTCTCCAGGGGAAAGAGCCACCCTCTCCTGCAGGGCCAGTCAGAATATTTACAGCAACTACTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTATTGCTGTCAGCAGTATGAAAGCTCACCGTGGACCTTCGGCCAAGGGACCAAGGTGGAAATCAAA | CAAGTGCAGCTACAGCAGTGGGGCGCAGGACTGTTGAAGCCTTCGGAGCCCCTGTCCCTCACCTGCGCTGTCTATGGTGGGTCCTTCAGTGGTCCCTACTGGAGCTGGATCCGCCAGCCCCCCGGGAAGGGGCTGGAGTGGATTGGGGAAATCAATCATAGTGGAAGCACCAACTACAACCCGTCCCTCAAGAGTCGAGTCACCATATCAGTAGACACGTCCAAGAACCAGTTCTCCCTGAAACTGACCTCTGTGACCGCCGCGGACACGGCTGTGTATTACTGTGCGAGAGACTACGGGGGTTTGGACGTCTGGGGCCAAGGGACCACGGTCACCGTCTCCTCA |
| | | | SEQ ID NO: 25759 | SEQ ID NO: 29765 |
| | | AA | EPVLTQSPGTLSLSPGERATLSCRASQNIYSNYLAWYQQKPGQAPRLLIYGASSRATGIPDRFSGSGSGTDFTLTISRLEPEDFAVYCCQQYESSPWTFGQGTKVEIK | QVQLQQWGAGLLKPSEPLSLTCAVYGGSFSGPYWSWIRQPPGKGLEWIGEINHSGSTNYNPSLKSRVTISVDTSKNQFSLKLTSVTAADTAVYYCARDYGGLDVWGQGTTVTVSS |
| | | | SEQ ID NO: 25760 | SEQ ID NO: 29766 |

FIGURE 50

| | | | | |
|---|---|---|---|---|
| **iPS:434535** | **21-225_74C8** | NA | GACATCCAGATGACCCAGTCTCCATCCTCACTGTCTGCATCAGTAGGAGACAGAGTCACCATCACTTGTCGGGCGAGTCAGGGCATTGGCAAGTATTTAGCCTGGTTTCAGCAGAAACCAGGGAAAGCCCCTAAGTCCCTGATCTATACTACATCCAATTTACAAAGTGGGGCCCCATCAAAGTTCAGCGGCAGTGGATCTGGGACAGATTTCACTCTCACCATCAGCAGCCTGCAGTATGAAGATTTTGCAACTTATTACTGCCAACAGTACAGTAATTACCCGCTCACTTTCGGCGGAGGGACCAAGGTGGAGATCAAT | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATGGCAGCTATGGTTATGACGGCCTTGACTACTGGGGCCAGGGAACCCTGGTCACCGTCTCCTCA |
| | | | SEQ ID NO: 25761 | SEQ ID NO: 29767 |
| | | AA | DIQMTQSPSSLSASVGDRVTITCRASQGIGKYLAWFQQKPGKAPKSLIYTTSNLQSGAPSKFSGSGSGTDFTLTISSLQYEDFATYYCQQYSNYPLTFGGGTKVEIN | QVQLVESGGGVVQPGRSLRLSCAASGFTFSSYGMHWVRQAPGKGLEWVAVIWYDGSNKNYADSVKGRFTISRDNSKNTLYLQMNSLRAEDTAVYYCARDGSYGYDGLDYWGQGTLVTVSS |
| | | | SEQ ID NO: 25762 | SEQ ID NO: 29768 |
| **iPS:434537** | **21-225_74E11** | NA | GAAATAGTGATGACGCAGTCTCCAGCCACCCTGTCTGTGTCTCCAGGGGAAAGAGCCACCCTCTCCTGCAGGGCCAGTCTGAGTGTTGTCAACAGCTTAGCCTGGTACCAGCAGAAACCTGGCCAGGCTCCCAGGCTCCTCATCCATGGTGCATCCACCAGGGCCACTGGTATCCCAGCCAGGTTCAGTGGCAGTGGGTCTGGGACAGAGTTCACTCTCACCATCAGCAGTCTGCAGTCTGAAGATTTTGCAATTTATTACTGTCAGCAGTATAATGACTGGCCGTGCAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATTTGGTATGATGGAAGTAATAAATACTATGCAGACTCCGTGAAGGGCCGATTCACCATCTCCAGAGACAATTCCAAGAACACGCTGTTTCTGCAAATGAACAGCCTGAGAGCCGAGGACACGGCTGTGTATTACTGTGCGAGCGATCGCAATATAGTGGGAGCTACTTACTTTGAGTCCTGGGGCCAGGGAACCCTGGTCACCGTCTCCTCA |
| | | | SEQ ID NO: 25763 | SEQ ID NO: 29769 |

FIGURE 50

| | | | | |
|---|---|---|---|---|
| | | AA | EIVMTQSPATLSVSPGERATLSCRASLSVVNSLA WYQQKPGQAPRLLIHGASTRATGIPARFSGSGSG TEFTLTISSLQSEDFAIYYCQQYNDWPCSFGQGT KLEIK | QVQLVESGGGVVQPGRSLRLSCAASGFTFSSYGMH WVRQAPGKGLEWVAVIWYDGSNKYYADSVKGRF TISRDNSKNTLFLQMNSLRAEDTAVYYCASDRNIV GATYFESWGQGTLVTVSS |
| | | | SEQ ID NO: 25764 | SEQ ID NO: 29770 |
| iPS:434539 | 21-225_74A2 | NA | GATATTGTGATGACTCAGTCTCCACTCTCCCTG CCCGTCACCCCTGGAGAGCCGGCCTCCATCTC CTGCAGGTCTAGTCAGAGCCTCCTGCATAGTA ATGGACACAACTATTTGGATTGGTACCTACAG AAGCCAGGGCGGTCTCCACAGCTCCTGATCTA TTTGGGTTCTAATCGGGCCTCCGGGGTCCCTG AGAGGTTCAGTGGCAGTGGATCAGGCACAGA TTTTACACTGAAAATCAGCAGAGTGGAGGCTG AGGATGTTGGGGTTTATTACTGCATGCAACCT CTACAAACTCCGTTCACTTTCGGCGGAGGGAC CAAGGTGGAGATCAAA | CAGGTGCAGGTACAGCAGTGGGGCGCAGGACTG TTGAAGCCTTCGGAGACCCTGTCCCTCACCTGCG CTGTCTATGGTGGGTCCTTCACTGATTACTACTGG AGCTGGATCCGCCAGCCCCCAGGGAAGGGGCTG GAGTGGATTGGGGAAATCAATCATAGTGGAGAC ACCAACTACAACCCGTCCCTCAAGAGTCGAGTCA CCATATCAGTAGACACGTCCAAGAACCAGTTCTC CCTGAAGCTGAGTTCTGTGACCGCCGCGGACACG GCTGTGTATTACTGTGCGAGAGAGTTTCCATATA GTGGAAGCTACCTCTACTACTACGGTATGGACGT CTGGGGCCAAGGGACCACGGTCACCGTCTCCTCA |
| | | | SEQ ID NO: 25765 | SEQ ID NO: 29771 |
| | | AA | DIVMTQSPLSLPVTPGEPASISCRSSQSLLHSNGH NYLDWYLQKPGRSPQLLIYLGSNRASGVPERFS GSGSGTDFTLKISRVEAEDVGVYYCMQPLQTPF TFGGGTKVEIK | QVQVQQWGAGLLKPSETLSLTCAVYGGSFTDYYW SWIRQPPGKGLEWIGEINHSGDTNYNPSLKSRVTIS VDTSKNQFSLKLSSVTAADTAVYYCAREFPYSGSY LYYYGMDVWGQGTTVTVSS |
| | | | SEQ ID NO: 25766 | SEQ ID NO: 29772 |

FIGURE 50

| | | | | |
|---|---|---|---|---|
| **iPS:434547** | **21-225_74H5** | NA | GAAATTGTGTTGACGCAGTCTCCAGGCACCCTGTCTTTGTCTCCAGGGGAGAGAGCCACCCTCTCCTGCAGGGCCAGTCAGAGTGTTAACAGCAACTACTTAGCCTGGTACCGGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCATTCTCATCATCAGCAGACTGGAGCCTGAGGATTTTGCAGTGTATTACTGTCAGCAGTATGAAAGCTCGCCGTGGACGTTCGGCCAAGGGACCAAGGTGGAAATCAAA | CAGGTGCAGCTACAACAGTGGGGCGCAGGACTGTTGAAGCCTTCGGAGACCCTGTCCCTCACTTGCGCTGTCAATGGTGGGCCCTTCAGTGGTTGCTACTGGAGCTGGATCCGCCAGCCCCCAGGGAAGGGGCTGGAGTGGATTGGGGAAATCAATCATAGTGGAAGGACCAACTTCAACCCGTCCCTCAAGAGTCGAGTCACCATATCAGTTGACACGTCCAAGAACCAGTTCTCCCTGAAGCTGAGCTCTGTGACCGCCGCGGACACGGCTGTGTATTACTGTGCGAGAGACTACGGCGGTATGGACGTCTGGGGCCAAGGGACCACGGTCACCGTCTCCTCA |
| | | | SEQ ID NO: 25767 | SEQ ID NO: 29773 |
| | | AA | EIVLTQSPGTLSLSPGERATLSCRASQSVNSNYLAWYRQKPGQAPRLLIYGASSRATGIPDRFSGSGSGTDFILIISRLEPEDFAVYYCQQYESSPWTFGQGTKVEIK | QVQLQQWGAGLLKPSETLSLTCAVNGGPFSGCYWSWIRQPPGKGLEWIGEINHSGRTNFNPSLKSRVTISVDTSKNQFSLKLSSVTAADTAVYYCARDYGGMDVWGQGTTVTVSS |
| | | | SEQ ID NO: 25768 | SEQ ID NO: 29774 |
| **iPS:434549** | **21-225_76E11** | NA | GACATCGTGATGACCCAGTCTCCAGACTCCCTGGCTGTGTCTCTGGGCGAGGGGGCCACCATCAACTGCAAGTCCCGCCAGAGTGTTTTACACAGCTCCAACAATTACAACTACTTAGCTTGGTACCAGCAGAAAGCAGGACAGCCTCCTAAACTGCTCATTTACTGGGCTTCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCTCCGACGTTCGGCCAAGGGACCAAGGTGGAAATCAGA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGACTCACCATGACCAGGAACACCTCCATAAGCACAGCCTACATGGAGCTGAGCAGCCTGAGATCTGAGGACACGGCCGTGTTTTACTGTGCATATAGCAGTGGCTGGTACTACTTTGACTACTGGGGCCAGGGAACCCTGGTCACCGTCTCCTCA |
| | | | SEQ ID NO: 25769 | SEQ ID NO: 29775 |

FIGURE 50

| | | | | |
|---|---|---|---|---|
| | | AA | DIVMTQSPDSLAVSLGEGATINCKSRQSVLHSSNNYNYLAWYQQKAGQPPKLLIYWASTRESGVPDRFSGSGSGTDFTLTISSLQAEDVAVYYCQQYYSTPPTFGQGTKVEIR | QVQLVQSGAEVKKPGASVKVSCKASGYTFTNYDINWVRQATGQGLEWMGWMNPNSGNTGYAQKFQGRLTMTRNTSISTAYMELSSLRSEDTAVFYCAYSSGWYYFDYWGQGTLVTVSS |
| | | | SEQ ID NO: 25770 | SEQ ID NO: 29776 |
| iPS:434551 | 21-225_75C4 | NA | GACATCGTGATGACCCAGTCTCCAGACTCCCTGGCTGTGTCTCTGGGCGAGAGGGCCACCATCAACTGTAAGTCCAGCCAGAGTATTTTATACAGCTCCAACAATAATAACTACTTAGCTTGGTTCCAGCAGAAACCAGGACAGCCTCCTAAGCTGCTCATTTATTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATATTACTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCATCCTAACAGTGGTAACACAGGCTATGCACAGAAGTTCCAGGGCAGAGTCACCATGACCAGGAACACCTCCATAAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTCTCCTCA |
| | | | SEQ ID NO: 25771 | SEQ ID NO: 29777 |
| | | AA | DIVMTQSPDSLAVSLGERATINCKSSQSILYSSNNNNYLAWFQQKPGQPPKLLIYWASTRESGVPDRFSGSGSGTDFTLTISSLQAEDVAVYYCQQYYITPPTFGQGTKVEIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCASSSGWYYFDYWGQGTLVTVSS |
| | | | SEQ ID NO: 25772 | SEQ ID NO: 29778 |

FIGURE 50

| | | | | |
|---|---|---|---|---|
| iPS:434559 | 21-225_74D11 | NA | GAAATTGTGTTGACGCAGTCTCCAGGCACCCTGTCTTTGTCTCCAGGGGAAAGAGCCACCCTCTCCTGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGGCCACTGGCATCCCAGACAGGTTCAGTGGCAGTGGGTCTGGGACAGACTTCGCTCTCACCATCAGCAGACTGGAGCCTGAAGATTTTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25773 | SEQ ID NO: 29779 |
| | | AA | EIVLTQSPGTLSLSPGERATLSCRASQSVYSSYLAWYQQKPGQAPRLLIYGASSRATGIPDRFSGSGSGTDFALTISRLEPEDFAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25774 | SEQ ID NO: 29780 |
| iPS:434561 | 21-225_77G1 | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGGCCACTGGCATCCCAGACAGGTTCAGTGGCAGTGGGTCTGGGACAGACTTCGCTCTCACCATCAGCAGACTGGAGCCTGAAGATTGTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25775 | SEQ ID NO: 29781 |

FIGURE 50

| | | | | |
|---|---|---|---|---|
| | | AA | EIVLTQSPGTLYLSPGERATLSCRASQSVYSSYLAWYQQKPGQAPRLLIYGASSRATGIPDRFSGSGSGTDFALTISRLEPEDCAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25776 | SEQ ID NO: 29782 |
| iPS:434563 | 21-225_75D8 | NA | GATATTGTGATGACTCAGTCTCCACTCTCCCTGCCCGTCACCCCTGGAGAGCCGGCCTCCATCTCCTGCAGGTCTAGTCAGAGCCTCCTGCATAGTAGTGGATACAACTATTTGGATTGGTACCTGCAGAAGCCAGGGCAGTCTCCACAGCTCCTGATCTATTTGGGTTCTAATCGGGCCTCCGGGGTCCCTGACAGGTTCAGTGGCAGTGGATCAGGCTCAGATTTTACACTGAAGATCAGCAGAGTGGAGGCTGAGGATGTTGGACTTTATTACTGCATGCAAGCTCTACACCCTCCTCTCACTTTCGGCGGAGGGACCAAGGTGGAGATCAAA | GAGGTGCAGCTGGTGGAGTCTGGGGGAGGCTTGGTACAGCCGGGGGGGTCCCTGAGACTCTCCTGTGCAGCCTCTGGATTCACCTTCAGTAACTACGACATGCACTGGGTCCGCCAAGCTACAGGAAAAGGTCTGGAGTGGGTCTCAGCTATTGGTACTGCTGGTGACACATACTATCCAGGCTCCGTGAAGGGCCGATTCACCATCTCCAGAGAAAATGCCAAGAACTCCTTGTATCTTCAAATGAACAGCCTGAGAGCCGGGGACACGGCTGTGTATTACTGTGCAAGAGTTCTTGACTACGGTGACTCCTTGGGCTACTACTACTACGGTATGGACGTCTGGGGCCAAGGGACCACGGTCACCGTCTCCTCA |
| | | | SEQ ID NO: 25777 | SEQ ID NO: 29783 |
| | | AA | DIVMTQSPLSLPVTPGEPASISCRSSQSLLHSSGYNYLDWYLQKPGQSPQLLIYLGSNRASGVPDRFSGSGSGSDFTLKISRVEAEDVGLYYCMQALHPPLTFGGGTKVEIK | EVQLVESGGGLVQPGGSLRLSCAASGFTFSNYDMHWVRQATGKGLEWVSAIGTAGDTYYPGSVKGRFTISRENAKNSLYLQMNSLRAGDTAVYYCARVLDYGDSLGYYYYGMDVWGQGTTVTVSS |
| | | | SEQ ID NO: 25778 | SEQ ID NO: 29784 |

FIGURE 50

| | | | | |
|---|---|---|---|---|
| **iPS:434565** | **21-225_75B10** | NA | GAAATTGTGTTGACGCAGTCTCCAGGCACCCTATCTTTGTCTCCAGGGGAAAGAGCCACCCTCTCCTGCAGGGCCAGTCCGAGTGTTAACAGCTACTACTTAGCCTGGTACCAGCAGAAACCTGGCCAGGCTCCCAGGCTCCTCATCTATGGTGCAACCAGCAGGGCCACTGGCATCCCAGACAGGTTCAGTGGCAGTGGGTCTGGAACAGACTTCACTCTCACCATCAGCAGACTGGAGCCTGAAGATTTTGCAGTTTATTTCTGTCAGCAGTATGAAGACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCAAATGCGATGTCTATGGTGGGTCCTTCAGTGGTTACTACTGGAGCTGGATCCGCCAGCCCCCAGGGAAGGGGCTGGAGTGGATTGGGGAAATCAATCACAGTGGAAGCACCAACTACAACCCGTCCCTCAAGAGTCGAGTCACCATATCAGTAGACACGTCCAAGAACCAGTTCTCCCTGAAGCTGAGCTCTGTGACCGCCGCGGACACGGCTGTGTATTACTGTGCGAGAGACTACGGTGGTTTGGACGTCTGGGGCCAAGGGACCACGGTCACCGTCTCCTCA |
| | | | SEQ ID NO: 25779 | SEQ ID NO: 29785 |
| | | AA | EIVLTQSPGTLSLSPGERATLSCRASPSVNSYYLAWYQQKPGQAPRLLIYGATSRATGIPDRFSGSGSGTDFTLTISRLEPEDFAVYFCQQYEDSPWTFGQGTKVEIK | QVQLQQWGAGLLKPSETLSLKCDVYGGSFSGYYWSWIRQPPGKGLEWIGEINHSGSTNYNPSLKSRVTISVDTSKNQFSLKLSSVTAADTAVYYCARDYGGLDVWGQGTTVTVSS |
| | | | SEQ ID NO: 25780 | SEQ ID NO: 29786 |
| **iPS:434569** | **21-225_77H5** | NA | GAAATAGTGATGACGCAGTCTCCAGTCACCCTGTCTGTGTCTCCAGGGGAAAGAGCCACCCTCTCCTGCAGGGCCAGTCAGAGTGTTAGCAGCAGCTTAGCCTGGTACCAGCAGAAACCTGGCCTGGCTCCCAGGCTCCTCATCTATGGTGCATCCACCAGGGCCACTGGTATCCCAGCCAGGTTCAGTGGCAGTGGGTCTGGGACAGAGTTCTCTTTTACCATCAGCAGCCTGCAGTCTGAAGATTTTGCAGTTTATTTCTGTCAGCAGTATAATGACTGGCCGTGCAGTTTTGGCCAGGGCTCCAAGCTGGAGATCCAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAATTATGGCATGCACTGGGTCCGCCAGGCTCCAGGCAAGGGGCTGGAGTGGGTGGCAGTTATATGGTATGATGGAAGAAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTCTATATTACTGTGCGAGAGATCGGAGTATATTGGGAGCTACTTTCTTTGACTACTGGGGCCAGGGAACCCTGGTCACCGTCTCCTCA |
| | | | SEQ ID NO: 25781 | SEQ ID NO: 29787 |

FIGURE 50

| | | | | |
|---|---|---|---|---|
| | | AA | EIVMTQSPVTLSVSPGERATLSCRASQSVSSSLAWYQQKPGLAPRLLIYGASTRATGIPARFSGSGSGTEFSFTISSLQSEDFAVYFCQQYNDWPCSFGQGSKLEIQ | QVQLVESGGGVVQPGRSLRLSCAASGFTFSNYGMHWVRQAPGKGLEWVAVIWYDGRNKYYADSVKGRFTISRDNSKNTLYLQMNSLRAEDTALYYCARDRSILGATFFDYWGQGTLVTVSS |
| | | | SEQ ID NO: 25782 | SEQ ID NO: 29788 |
| iPS:434571 | 21-225_74D2 | NA | GAAATTGTGTTGACGCAGTCTCCAGGCACCCTGTCTTTGTCTCCAGGGGAAAGAGCCACCCTCTCCTGCAGGGCCAGTCAGAGTGTTGACAGCAACTACTTAGCCTGGTACCAGCAGAAACCTGGCCAGGCTCCCAGGCTCCTCATCTATGGTGCATCCAGCAGGGCCCCTGGCATCCCAGACAGGTTCAGTGGCAGTGGGTCTGGGACAGACTTCACTCTCACCATCAACAGACTGGAGCCTGAAGATTTTGCAGTGTATTTCTGTCAGCAGTATGAAAGCTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGGTTGCTACTGGAGCTGGATCCGCCAGCCCCCAGGGAAGGGGCTGGAGTGGATTGGGGAAATCAATCATAGTGGAAGGACCAACTACAACCCGTCCCTCAAGAGTCGAGTCACCATCTCAGTAGACACGTCCGAGAACCAGTTCTCCCTGAAGTTGAGCTCTGTGACCGCCGCGGACACGGCTGTGTATTACTGTGCGAGAGACTACGGTGGGCTTGACTACTGGGGCCAGGGAACCCTGGTCACCGTCTCCTCA |
| | | | SEQ ID NO: 25783 | SEQ ID NO: 29789 |
| | | AA | EIVLTQSPGTLSLSPGERATLSCRASQSVDSNYLAWYQQKPGQAPRLLIYGASSRAPGIPDRFSGSGSGTDFTLTINRLEPEDFAVYFCQQYESSPWTFGQGTKVEIK | QVQLQQWGAGLLKPSETLSLTCAVYGGSFSGCYWSWIRQPPGKGLEWIGEINHSGRTNYNPSLKSRVTISVDTSENQFSLKLSSVTAADTAVYYCARDYGGLDYWGQGTLVTVSS |
| | | | SEQ ID NO: 25784 | SEQ ID NO: 29790 |

FIGURE 50

| | | | | |
|---|---|---|---|---|
| **iPS:434573** | **21-225_77E6** | NA | GACATCCAGATGACCCAGTCTCCATCCTCACTGTCTGCATCTGTAGGAGACAGAGTCACCATCACTTGTCGGGCGAGTCAGGGCATTAGCAAGTATTTAGCCTGGTTTCAGCAGAAACCAGGGAAAGCCCCTAAGTCCCTGATCTATGCTGCATCCAGTTTGCAAGGTGGGGCCCCATCAAAGTTCAGCGGCAGTGGATCTGGGACAGATTTCACTCTCACCATCAGCAGCCTGCAGCCTGAAGATTTTGCAACTTATTACTGCCAACAGTACAGTAATTACCCGCTCACTTTCGGCGGAGGGACCAGGGTGGAGATCAAA | CAGGTGCAGCTGGTGGAGTCTGGGGGAGGCGTGGTCCAGTCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATCAAAACTATGCAGACTCCGTGAAGGGCCGATTCACCATCTCCAGAGACAATTCCAAGAACACGCTGTTTCTGCAAATGAACAGCCTGAGAGCCGAGGACACGGCTGTGTATTACTGTGCGAGAGATGGCAGCTATGGTTACGACGGCCTTGACTACTGGGGCCAGGGAACCCTGGTCACCGTCTCCTCA |
| | | | SEQ ID NO: 25785 | SEQ ID NO: 29791 |
| | | AA | DIQMTQSPSSLSASVGDRVTITCRASQGISKYLAWFQQKPGKAPKSLIYAASSLQGGAPSKFSGSGSGTDFTLTISSLQPEDFATYYCQQYSNYPLTFGGGTRVEIK | QVQLVESGGGVVQSGRSLRLSCAASGFTFSSYGMHWVRQAPGKGLEWVAVIWYDGSNQNYADSVKGRFTISRDNSKNTLFLQMNSLRAEDTAVYYCARDGSYGYDGLDYWGQGTLVTVSS |
| | | | SEQ ID NO: 25786 | SEQ ID NO: 29792 |
| **iPS:434575** | **21-225_77C7** | NA | GACATCGTGATGACCCAGTCTCCAGACTCCCTGGCTGTGTCTCTGGGCGAGAGGGCCACCATCAACTGCAAGTCCAGCCAGACTGTTTTACACAGCTCCAACAATTATAACTACTTAGCTTGGTACCAGCAGAAGCCAGGACAGCCCCCTAAGGTGCTCCTTTACTGGACATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTTTAGTAGTCCTCCGACGTTCGGCCAAGGGACCAGGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCCCCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTATTGTGCGGTTTCCAGTGGCTGGCACTGGTTCGACCCCTGGGGCCAGGGAACCCTGGTCACCGTCGCCTCA |
| | | | SEQ ID NO: 25787 | SEQ ID NO: 29793 |

FIGURE 50

| | | | | |
|---|---|---|---|---|
| | | AA | DIVMTQSPDSLAVSLGERATINCKSSQTVLHSSNNYNYLAWYQQKPGQPPKVLLYWTSTRESGVPDRFSGSGSGTDFTLTISSLQAEDVAVYYCQQYFSSPPTFGQGTRVEIK | QVQLVQSGAEVKKPGASVKVSCKASGYTFPNYDINWVRQATGQGLEWMGWMNPNSGNTGYAQKFQGRVTMTRNTSISTAYMELSSLRSEDTAVYYCAVSSGWHWFDPWGQGTLVTVAS |
| | | | SEQ ID NO: 25788 | SEQ ID NO: 29794 |
| **iPS:434579** | **21-225_77F7** | NA | GAAATTGTGTTGACGCAGTCTCCAGGCACCCTGTATTTGTCTCCAGGGGAAAGAGCCACCCTGTCATGCAGGGCCAGTCAGAGTGTTTACAGCAGCTACTTAGCGTGGTACCAGCAGAAACCTGGCCAGGCTCCCAGGCTCATCATTTATGGTGCATCCAGCCGGGCCACTGGCATCCCAGACAGGTTCAGTGGCAGTGGGTGTGGGACAGACTTCGCTCTCACCATCAGCAGAGTGGAGCATGAAGATTGTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25789 | SEQ ID NO: 29795 |
| | | AA | EIVLTQSPGTLYLSPGERATLSCRASQSVYSSYLAWYQQKPGQAPRLIIYGASSRATGIPDRFSGSGCGTDFALTISRVEHEDCAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25790 | SEQ ID NO: 29796 |

FIGURE 50

| | | | | |
|---|---|---|---|---|
| **iPS:434581** | **21-225_74B12** | NA | GAAATTGTGTTGACGCAGTCTCCAGGCACCCTGTATTTGTCTCCAGGGGAAAGAGCCACCCTCTCCTGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGACTGGAGCCTGAAGATTTTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25791 | SEQ ID NO: 29797 |
| | | AA | EIVLTQSPGTLYLSPGERATLSCRASQSVYSSYLAWYQQKPGQAPRLLIYGASSRSTGIPDRFSGSGSGTDFALTISRLEPEDFAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25792 | SEQ ID NO: 29798 |
| **iPS:434583** | **21-225_74B6** | NA | GAAATTGTGTTGACGCAGTCTCCAGGCACCCTGTCTTTGTCTCCAGGGGAAAGAGCCACCCTCTCCTGCAGGGCCAGTCAGAGTGTTAGCAGCAGCTACTTAGCCTGGTACCAGCAGAAACCTGGCCAGGCTCCCAGGCTCCTCATCTATGGTGCATCCACCAGGGCCACTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTAACTCACCGCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAATGGTAACACAGGCTATGCACAGAAGTTCCAGGGCAGAGTCACCATGACCAGGAACACCTCCATAAGCACAGCCTACATGGAGCTGAACAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTTA |
| | | | SEQ ID NO: 25793 | SEQ ID NO: 29799 |

FIGURE 50

| | | | | |
|---|---|---|---|---|
| | | AA | EIVLTQSPGTLSLSPGERATLSCRASQSVSSSYLAWYQQKPGQAPRLLIYGASTRATGIPDRFSGSGSGTDFTLTISRLEPEYFAVYYCQQYGNSPLTFGGGTKVEIK | QVQLVQSGAEVKKPGASVKVSCKASGYTFTNYDINWVRQATGQGLEWMGWMHPNNGNTGYAQKFQGRVTMTRNTSISTAYMELNSLRSEDTAVYYCAISSGWYWFDPWGQGTLVTVSL |
| | | | SEQ ID NO: 25794 | SEQ ID NO: 29800 |
| iPS:434585 | 21-225_75A12 | NA | GAAATTGTGTTGACGCAGTCTCCAGGCACCCTGTCTTTGTCTCCAGGGGAAAGAGCCACCCTCTCCTGCAGGGCCAGTCAGAGTGTTAGCAGCAGGTACTTAGCCTGGTACCAGCAGAAACCTGGCCAGGCTCCCAGACTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGCAGATTTTGCAGTATATTACTGTCAGCATTATGATAGCTCACCGTGGACGTTCGGCCAAGGGACCAAGGTGGAAATCAAA | CAGGTGCAGCTACAGCAGGGGGGCGCAGGACCGTTGAAGCCTTCGGAGACCCTGTCCCTCACCTGCGCTGTCTATGGTGGGTCCTTCAGTGGTTGCTACTGGAGCTGGATCCGCCAGCCCCCAGGGAAGGGGCTGGAGTGGATTGGGGAAATCAATTATAGTGGAAGAACCAACTACAACCCGTCCCTCAAGAGTCGAGTCACCATATCAGTAGACACGTCCAAGAACCAGTTCTCCCTGAAGCTGAGCTCTGTGACCGCCGCGGACACGGCTGTGTATTACTGTGCGAGAGACTACGGCGGTATGGACGTCTGGGGCCAAGGGACCACGGTCACCGTCTCCTCA |
| | | | SEQ ID NO: 25795 | SEQ ID NO: 29801 |
| | | AA | EIVLTQSPGTLSLSPGERATLSCRASQSVSSRYLAWYQQKPGQAPRLLIYGASSRATGIPDRFSGSGSGTDFTLTISRLEPADFAVYYCQHYDSSPWTFGQGTKVEIK | QVQLQQGGAGPLKPSETLSLTCAVYGGSFSGCYWSWIRQPPGKGLEWIGEINYSGRTNYNPSLKSRVTISVDTSKNQFSLKLSSVTAADTAVYYCARDYGGMDVWGQGTTVTVSS |
| | | | SEQ ID NO: 25796 | SEQ ID NO: 29802 |

FIGURE 50

| | | | | |
|---|---|---|---|---|
| iPS:434587 | 21-225_74G3 | NA | GAATTTATGTTGACGCAGTCTCCAGGCACCCTGTGTTGGTCTCCAGGGGAAAGAGCCACCCTCTCGTGCAGGGCCAGTCAGAGTGTTAGCAGCAGCTACTTAGTCTGGTATCAGCAGAAACCTGGCCAGGCTCCCAGGCTCCTCATCTATGGTGCATCCACCAGGGCCACTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTTGCTCACCGCTCACTTTCGGCGGAGGGACCAAGGTGGAGATCACA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGTCCCTGGACAAGGGCTTGAGTGGATGGGATGGATGCACCCTAACAGTGGTAACACAGGCTATGCACAGAAGTTCCAGGGCAGAGTCACCATGACCAGGAACACCTCCATCAGCACAGCCTACATGGAGCTGAGCAGCCTGAGATCTGAGGACACGGCCGTGTATTACTGTGCGATTAGTAGTGGCTGGTACTGGTTCGACCCCTGGGGCCAGGGAACCCTGGTCACCGTCTCCTCA |
| | | | SEQ ID NO: 25797 | SEQ ID NO: 29803 |
| | | AA | EFMLTQSPGTLCWSPGERATLSCRASQSVSSSYLVWYQQKPGQAPRLLIYGASTRATGIPDRFSGSGSGTDFTLTISRLEPEYFAVYYCQQYGCSPLTFGGGTKVEIT | QVQLVQSGAEVKKPGASVKVSCKASGYTFTNYDINWVRQVPGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25798 | SEQ ID NO: 29804 |
| iPS:434595 | 21-225_77A10 | NA | GAAATTGTGTTGACGCAGTCTCCAGGCACCCTGTCTTTGTCTCCAGGGGAGAGAGCCACCCTCTCCTGCAGGGCCAGTCAGAGTGTTCACAGCAGGTACTTAGCCTGGTACCAGCAGAAACCTGGCCAGGCTCCCAAACTCCTCATCTTTGGTGCATCCAGCAGGGCCACTGGCATCCCAGACAGGTTCAGTGGCAGTGGGTCTGGGACGGACTTCACTCTCACCATCAGCAGACTGGAGCCTGAAGATTTTGCAGTATATTACTGTCAGCATTATGATAGCTCACCGTGGACGTTCGGCCAAGGGACCAAGGTGGAAATCAAA | CAGGTGCAGCTACAACAGGGGGGCGCAGGACCGTTGAAGCCTTCGGAGACCCTGTCCCTCACCTGCGCTGTCTATGGTGGGTCCTTCAGTGGTTGCTACTGGAGCTGGATCCGCCAGCCCCCAGGGAAGGGGCTGGAGTGGATTGGGGAAATCAATTATAGTGGAAGAACCAACTACAACCCGTCCCTCAAGAGTCGAGTCACCATATCAGTAGACACGTCCAAGAACCAGTTCTCCCTGAAGCTGAGCTCTGTGACCGCCGCGGACACGGCTGTGTATTACTGTGCGAGAGACTACGGCGGTATGGACGTCTGGGGCCAAGGGACCACGGTCACCGTCTCCTCA |
| | | | SEQ ID NO: 25799 | SEQ ID NO: 29805 |

FIGURE 50

| | | | | |
|---|---|---|---|---|
| | | AA | EIVLTQSPGTLSLSPGERATLSCRASQSVHSRYLAWYQQKPGQAPKLLIFGASSRATGIPDRFSGSGSGTDFTLTISRLEPEDFAVYYCQHYDSSPWTFGQGTKVEIK | QVQLQQGGAGPLKPSETLSLTCAVYGGSFSGCYWSWIRQPPGKGLEWIGEINYSGRTNYNPSLKSRVTISVDTSKNQFSLKLSSVTAADTAVYYCARDYGGMDVWGQGTTVTVSS |
| | | | SEQ ID NO: 25800 | SEQ ID NO: 29806 |
| iPS:434597 | 21-225_77C10 | NA | GACATCGTGATGACCCAGTCTCCAGATTGCCTGGCTGTGTCTCTGGGCGAGAGGGCCACCATCAACTGCAAGTCCAGCCAGAGTGTTTTATACACCTCCAACAATAACAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTTGCAGTTTATTATTGTCAGCACTATTATAATACTCCGTGGAAGTTTGTCCAAGGGACCAAGGTGGAAATCACA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTCA |
| | | | SEQ ID NO: 25801 | SEQ ID NO: 29807 |
| | | AA | DIVMTQSPDCLAVSLGERATINCKSSQSVLYTSNNNNYLAWYQQKPGQPPKLLIYWASTRESGVPDRFSGSGSGTDFTLTISSLQAEDVAVYYCQHYYNTPWKFVQGTKVEIT | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25802 | SEQ ID NO: 29808 |

FIGURE 50

| | | | | |
|---|---|---|---|---|
| **iPS:434603** | **21-225_77D11** | NA | GAATTTATGTTGACGCAGTCTCCAGGCACCCTGTATTGGTCTCCAGGGGAAAGAGCCACCATCTCGTGCAGGGCCAGTCAGAGTGTTAGCAGCAGCTACTTAGTCTGGTATCAGCAGAAACCTGGCCAGGCTCCCAGGCTCCTCATCTATGGTGCATCCACCAGGGCCACTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTTGCTCACCGCTCACTTTCGGCGGAGGGACCAAGGTGGAGATCACA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGTCCCTGGACAAGGGCTTGAGTGGATGGGATGGATGCACCCTAACAGTGGTAACACAGGCTATGCACAGAAGTTCCAGGGCAGAGTCACCATGACCAGGAACACCTCCATCAGCACAGCCTACATGGAGCTGAGCAGCCTGAGATCTGAGGACACGGCCGTGTATTACTGTGCGATTAGTAGTGGCTGGTACTGGTTCGACCCCTGGGGCCAGGGAACCCTGGTCACCGTCTCCTCA |
| | | | SEQ ID NO: 25803 | SEQ ID NO: 29809 |
| | | AA | EFMLTQSPGTLYWSPGERATISCRASQSVSSSYLVWYQQKPGQAPRLLIYGASTRATGIPDRFSGSGSGTDFTLTISRLEPEYFAVYYCQQYGCSPLTFGGGTKVEIT | QVQLVQSGAEVKKPGASVKVSCKASGYTFTNYDINWVRQVPGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25804 | SEQ ID NO: 29810 |
| **iPS:434611** | **21-225_77C12** | NA | GAAATTGTGTTGACGCAGTCTCCAGGCACCCTGTCTTTGTCTCCAGGGGAAAGAGCCACCCTCTCCTGCAGGGCCAGGCAGAGTGTTGACAGCAGTTATTTAGCCTGGTACCAGCAGAAACGTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGGCTGGAGCCTGAAGATTTTGCAGTATATTACTGTCAGCAGTATGAAAGCTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTATGGTGGGGCCTTCAGTGGTTCCTACTGGAGCTGGATCCGCCAGTCCCCAGGGAAGGGGCTGGAGTGGATTGGGGAAATCAATTATAGGGGAAGCACCAACTACAACCCGTCCCTCAAGAGTCGAGTCACCATATCAGTAGACACGTCCAAGAACCAGTTCTCCCTGAACCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25805 | SEQ ID NO: 29811 |

FIGURE 50

| | | | | |
|---|---|---|---|---|
| | | AA | EIVLTQSPGTLSLSPGERATLSCRARQSVDSSYLAWYQQKRGQAPRLLIYGASSRATGIPDRFSGSGSGTDFTLTISRLEPEDFAVYYCQQYESSPWTFGQGTKVEIK | QVQLQQWGAGLLKPSETLSLTCAVYGGAFSGSYWSWIRQSPGKGLEWIGEINYRGSTNYNPSLKSRVTISVDTSKNQFSLNLSSVTAADTAVYYCARDYGGMDVWGQGTTVTVSS |
| | | | SEQ ID NO: 25806 | SEQ ID NO: 29812 |
| **iPS:434613** | **21-225_77D12** | NA | GACATCGTGATGACCCAGTCTCCAGACTCCCTGGCTGTGTCTCTGGGCGCGAGGGCCACCATCAATTGCAGGTCCAGCCAGAGTGTTTTATACAGCTCCAACAATTATAACTACTTAGCTTGGTACCAGCAGAAACCAGGACAGCCTCCTAAGCTGCTCATTTACTGGGCATCTACCCGGGATTCCGGGGTCCCTGACCGATTCAGTGGCAGCGGGTCTGGGACAGATTTCACTCTCACCATCAGCAGCCTGCAGGCTGAAGATGTGGCCGTTTATTACTGTCAGCAATATTATACTACTCCGTGCAGTTTTGGCCAGGGGACCAAGCTGGAGATCAAG | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAACACAGCCTACATGGAGCTGAGCAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTCA |
| | | | SEQ ID NO: 25807 | SEQ ID NO: 29813 |
| | | AA | DIVMTQSPDSLAVSLGARATINCRSSQSVLYSSNNYNYLAWYQQKPGQPPKLLIYWASTRDSGVPDRFSGSGSGTDFTLTISSLQAEDVAVYYCQQYYTTPCSFGQGTKLEIK | QVQLVQSGAEVKKPGASVKVSCKASGYTFTNYDINWVRQATGQGLEWMGWMNPNSGNTGYAQKFQGRVTMTRNTSINTAYMELSSLRSEDTAVYYCAISSGWYWFDPWGQGTLVTVSS |
| | | | SEQ ID NO: 25808 | SEQ ID NO: 29814 |

FIGURE 50

| | | | | |
|---|---|---|---|---|
| **iPS:434615** | **21-225_76C5** | NA | GACATCCAGATGACCCAGTCTCCATCCTCACTGTCTGCATCTGTAGGAGACAGAGTCACCATCACTTGTCGGGCGAGTCAGGTCATTAGCAAGTATTTAGCCTGGTTTCAGCAGAAACCAGGGAAAGCCCCTAAGTCCCTGATCTATGCTGCATCCAGTTTGCAAAGTGGGGCCCCATCAAAGTTCAGCGGCAGTGGATCTGGGACAGATTTCACTCTCACCATCAGCAGCCTGCAGCCTGAAGATTTTGCAACTTATTACTGCCAACAGTACAGTAATTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATGGCAGCTATGGTTACGACGGCCTTGACTACTGGGGCCAGGGAACCCTGGTCACCGTCTCCTCA |
| | | | SEQ ID NO: 25809 | SEQ ID NO: 29815 |
| | | AA | DIQMTQSPSSLSASVGDRVTITCRASQVISKYLAWFQQKPGKAPKSLIYAASSLQSGAPSKFSGSGSGTDFTLTISSLQPEDFATYYCQQYSNYPLTFGGGTKVEIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAVYYCARDGSYGYDGLDYWGQGTLVTVSS |
| | | | SEQ ID NO: 25810 | SEQ ID NO: 29816 |
| **iPS:434617** | **21-225_74B8** | NA | GACAGCGTGATGACCCAGTCTCCAGACTCCCTGGCTGTGTCTCTGGGCGAGAGGGCCACCATCAACTGCAAGTCCAGCCAGAGTGTTTTACACAGCTCTAATAAAAAGAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGTCTGCAGGCTGAAGATGTGGCAGTTTATTACTGTCAGCAATATTATAGGACTCCGTGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAATAGTGGTAACACAGGCTATGCACAGAAGTTCCAGGGCAGAGTCACCCTGACCAGGAACACCTCCATAAGCACAGCCTACATGGAGCTGAGCAGCCTGAGATCTGAGGACACGGCCGTGTATTACTGTGCGGTTTCCAGTGGCTGGTACTGGTTCGACCCCTGGGGCCAGGGAACCCTGGTCACCGTCTCCTCA |
| | | | SEQ ID NO: 25811 | SEQ ID NO: 29817 |

FIGURE 50

| | | | | |
|---|---|---|---|---|
| | | AA | DSVMTQSPDSLAVSLGERATINCKSSQSVLHSSNKKNYLAWYQQKPGQPPKLLIYWASTRESGVPDRFSGSGSGTDFTLTISSLQAEDVAVYYCQQYYRTPWTFGQGTKVEIK | QVQLVQSGAEVKKPGASVKVSCKASGYTFTNYDINWVRQATGQGLEWMGWMHPNSGNTGYAQKFQGRVTLTRNTSISTAYMELSSLRSEDTAVYYCAVSSGWYWFDPWGQGTLVTVSS |
| | | | SEQ ID NO: 25812 | SEQ ID NO: 29818 |
| **iPS:434619** | **21-225_78C1** | NA | GACATCGTGATGACCCAGTCTCCAGATTGCCTGGCTGTGTCTCTGGGCGAGAGGGCCACCATCAACTGCAAGTCCAGCCAGAGTGTTTTATACACCTCCAACAATAACAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTTGCAGTTTATTATTGTCAGCACTATTATAATACTCCGTGGAAGTTTGTCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTCA |
| | | | SEQ ID NO: 25813 | SEQ ID NO: 29819 |
| | | AA | DIVMTQSPDCLAVSLGERATINCKSSQSVLYTSNNNNYLAWYQQKPGQPPKLLIYWASTRESGVPDRFSGSGSGTDFTLTISSLQAEDVAVYYCQHYYNTPWKFVQGTKVEIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25814 | SEQ ID NO: 29820 |

FIGURE 50

| | | | | |
|---|---|---|---|---|
| **iPS:434621** | **21-225_74D1** | NA | GAAATAGTGATGACGCAGTCTCCAGCCACCCTGTCTGTGTCTCCAGGGGAAAGAGCCACCCTCTCCTGCAGGGCCAGTCAGAGTGTTAGCAGAAATTTAGCCTGGTTCCAGCAGAAACCTGGCCAGGCTCCCAGGCTCCTCATCTATGGTGCATCCATCAGGGCCACTGGTATCCCAGCCAGGTTCAGTGGCAGTGGGTCTGGGACAGAGTTCACTCTCACCATCTACAGCCTGCAGTCTGAAGATTTTGCAGTTTATTACTGTCAGCAGTATAATAACTGGCCTCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACCATGCAGACTCCGTGAAGGGCCGATTCACCATCTCCAGAGACAATTCCAAGAACACGCTGTATCTGCAAATGAACAGCCTGAGAGCCGAGGACACGGCTATGTATTACTGTGCGAGAGATGAGGGGTTCGGGGAGTTCGACTACTACAACTACGGTATGGACGTCTGGGGCCAAGGGACCACGGTCACCGTCTCCTCA |
| | | | SEQ ID NO: 25815 | SEQ ID NO: 29821 |
| | | AA | EIVMTQSPATLSVSPGERATLSCRASQSVSRNLAWFQQKPGQAPRLLIYGASIRATGIPARFSGSGSGTEFTLTIYSLQSEDFAVYYCQQYNNWPPLTFGGGTKVEIK | QVQLVESGGGVVQPGRSLRLSCAASGFTFSSYGMHWVRQAPGKGLEWVAVIWYDGSNKYHADSVKGRFTISRDNSKNTLYLQMNSLRAEDTAMYYCARDEGFGEFDYYNYGMDVWGQGTTVTVSS |
| | | | SEQ ID NO: 25816 | SEQ ID NO: 29822 |
| **iPS:434629** | **21-225_74C3** | NA | GAAATAGTGATGACGCAGTCTCCAGCCACCCTGTCTGTGTCTCCAGGGGAAAGAGCCACCCTCTCCTGCAGGGCCAGTCAGAGTGTTGCCAGCAGCTTAGCCTGGTACCAGCAGAAACCTGGCCAGGCTCCCAGGCTCCTCATCTTTGGTACATCCACCAGGGCCACTGGTATCCCAGCCAGGTTCAGTGGCAGTGGGTCTGGGACAGAGTTCACTCTCATAATCAGCAGCCTGCAGTCTGAAGATTTTGCAGTTTATTACTGTCAGCAGTATAATGACTGGCCGTGCAGTTTTGGCCTGGGGACCAAGCTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTTTGGCATGCACTGGGCCCGCCAGGCTCCAGGCAAGGGGCTGGAATGGGTGGCAGCTATTTGGTATGATGGAAGTAATAAATACTGTGCAGACTCCGTGAAGGGCCGATTCACCATCTCCAGAGACAATTCCAAGAACACACTGTCTCTGCAAATGAACAGCCTGAGAGCCGAGGACTCGGCTGTGTATTACTGTGCGAGAGATCGGAGTATACTGGGAGCTGCTTTCTTTGACTACTGGGGCCAGGGAACCCTGGTCACCGTCTCCTCA |
| | | | SEQ ID NO: 25817 | SEQ ID NO: 29823 |

FIGURE 50

| | | | | |
|---|---|---|---|---|
| | | AA | EIVMTQSPATLSVSPGERATLSCRASQSVASSLA WYQQKPGQAPRLLIFGTSTRATGIPARFSGSGSG TEFTLIISSLQSEDFAVYYCQQYNDWPCSFGLGT KLEIK | QVQLVESGGGVVQPGRSLRLSCAASGFTFSSFGMH WARQAPGKGLEWVAAIWYDGSNKYCADSVKGRF TISRDNSKNTLSLQMNSLRAEDSAVYYCARDRSILG AAFFDYWGQGTLVTVSS |
| | | | SEQ ID NO: 25818 | SEQ ID NO: 29824 |
| **iPS:434633** | **21-225_74G8** | NA | GAAATTGTGTTGACGCAGTCTCCAGGCACCCT GTCTTTGTCTCCAGGGGAAAGAGCCACCCTCT CCTGCAGGGCCAGTCAGAGTTTTAGCAGCGCC TACTTAGCCTGGTACCAGCAGAAACCTGGCCA GGCTCCCAGGCTCCTCATCTATGGTACTTCCA GCAGGGCCACTGGCATCCCAGACAGGTTCAGT GGCAGTGGGTCTGGGACAGATTTCACTCTCAC CATCGGCAGACTGGAGCCTGAAGATTTTGCAG TGTATTACTGTCAACAGTATGGTAACTCAAGG ACGTTCGGCCAAGGGACCAAGGTGGAAATCA AA | GAGGTGCAGCTGGTGGAGTCTGGGGGAGGCTTG GTAAAGCCTGGGGGGTCCCTTAGACTCTCCTGTG CAGCCTCTGGATTCACTTTCAGTAACGCCTGGAT GAACTGGGTCCGCCAGGCTCCAGGGAAGGGGCT GGAGTGGGTTGGCCGTATTAAAAACAAAGCTGA TGGTGGGACAACAGACTACGCTGCACCCGTGAA AGGCAGATTCACCATCTCAAGAGATGATTCAAA AAACACGCTGTATCTGCAAATGAACAGCCTGAA AACCGAGGACACAGCCGTGTATTACTGTACCACA GTGGGAGCTACTACGGACTACTGGGGCCAGGGA ACCCTGGTCACCGTCTCCTCA |
| | | | SEQ ID NO: 25819 | SEQ ID NO: 29825 |
| | | AA | EIVLTQSPGTLSLSPGERATLSCRASQSFSSAYLA WYQQKPGQAPRLLIYGTSSRATGIPDRFSGSGSG TDFTLTIGRLEPEDFAVYYCQQYGNSRTFGQGT KVEIK | EVQLVESGGGLVKPGGSLRLSCAASGFTFSNAWM NWVRQAPGKGLEWVGRIKNKADGGTTDYAAPVK GRFTISRDDSKNTLYLQMNSLKTEDTAVYYCTTVG ATTDYWGQGTLVTVSS |
| | | | SEQ ID NO: 25820 | SEQ ID NO: 29826 |

FIGURE 50

| | | | | |
|---|---|---|---|---|
| **iPS:434635** | **21-225_78E6** | NA | GACATCGTGATGACCCAGTCTCCAGACTCCCTGGCTGTGTCTCTGGGCGAGAGGGCCACCATCAACTGCAAGTCCAGCCAGAGTGTTTTGTACAGCTCCAACAGTCACAACTACTTAGCTTGGTACCAGCAGAAACCAGGACAGCCTCCTAAACTGCTCATTTACTGGGCATCTATCCGGGAATCCGGGGTCCCTGACCGATTCAGTGGCAGCGGGTCTGGGACAGATTTCACTCTCTCCATCAGCAGCCTGCAGGCTGAAGATGTGGCAGTTTATTACTGTCAGCAATATTATAGTACTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCTCTGGACAAGGACTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACTCGGCCGTATATTACTGTGCGTATAGTAGTGGCTGGTACAAATTTGACTACTGGAGCCAGGGAACCCTGGTCACCGTCTCCTCA |
| | | | SEQ ID NO: 25821 | SEQ ID NO: 29827 |
| | | AA | DIVMTQSPDSLAVSLGERATINCKSSQSVLYSSNSHNYLAWYQQKPGQPPKLLIYWASIRESGVPDRFSGSGSGTDFTLSISSLQAEDVAVYYCQQYYSTPCSFGQGTKLEIK | QVQLVQSGAEVKKPGASVKVSCKASGYTFTNYDINWVRQASGQGLEWMGWMHPNSGNTGYAQKFQGRVTMTRNTSISTAYMELSSLRSEDSAVYYCAYSSGWYKFDYWSQGTLVTVSS |
| | | | SEQ ID NO: 25822 | SEQ ID NO: 29828 |
| **iPS:434637** | **21-225_78E7** | NA | GAAATTGTGTTGACGCAGTCGCCAGGCACCCTGTCTTTGTCTCCAGGGGAGAGAGCCACCCTCTCCTGCAGGGCCAGTCAGAATGTTGACAGCAACTACTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTTTTACTGTCAGCAGTATGAACGCTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTATGGTGGGTCCCTCAGTGGTTCCTACTGGAGCTGGATCCGCCAGCCCCCAGGGAAGGGGCTGGAGTGGATTGGGGAAATCAATCTTAGTGGAAGCACCAACTACAACCCGTCCCTCAAGAGTCGAGTCACCATATCAGTAGACACGTCCAAGAGCCAGTTCTCCCTGACGCTGCGCTCTGTGACCGCCGCGGACACGGCTGTGTATTACTGTGCGAGAGACTACGGTGGCCTTGACTACTGGGGCCAGGGAACCCTGGTCACCGTCTCCTCA |
| | | | SEQ ID NO: 25823 | SEQ ID NO: 29829 |

FIGURE 50

| | | | | |
|---|---|---|---|---|
| | | AA | EIVLTQSPGTLSLSPGERATLSCRASQNVDSNYLAWYQQKPGQAPRLLIYGASSRATGIPDRFSGSGSGTDFTLTISRLEPEDFAVFYCQQYERSPWTFGQGTKVEIK | QVQLQQWGAGLLKPSETLSLTCAVYGGSLSGSYWSWIRQPPGKGLEWIGEINLSGSTNYNPSLKSRVTISVDTSKSQFSLTLRSVTAADTAVYYCARDYGGLDYWGQGTLVTVSS |
| | | | SEQ ID NO: 25824 | SEQ ID NO: 29830 |
| **iPS:434639** | **21-225_74B7** | NA | GACATCGTGATGACCCAGTCTCCAGACTCCCTGGCTGTGTCTCTGGGCGAGAGGGCCACCATCAACTGCAAGTCCAGCCAGACTGTTTTACACAGCTCCAACAATTATAACTACTTAGCTTGGTACCAGCAGAAGCCAGGACAGCCCCCTAAGGTGCTCATTTACTGGACATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTTTAGTAGTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCCCCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTATTGTGCGGTTTCCAGTGGCTGGCACTGGTTCGACCCCTGGGGCCAGGGAACCCTGGTCACCGTCGCCTCA |
| | | | SEQ ID NO: 25825 | SEQ ID NO: 29831 |
| | | AA | DIVMTQSPDSLAVSLGERATINCKSSQTVLHSSNNYNYLAWYQQKPGQPPKVLIYWTSTRESGVPDRFSGSGSGTDFTLTISSLQAEDVAVYYCQQYFSSPPTFGQGTKVEIK | QVQLVQSGAEVKKPGASVKVSCKASGYTFPNYDINWVRQATGQGLEWMGWMNPNSGNTGYAQKFQGRVTMTRNTSISTAYMELSSLRSEDTAVYYCAVSSGWHWFDPWGQGTLVTVAS |
| | | | SEQ ID NO: 25826 | SEQ ID NO: 29832 |

FIGURE 50

| | | | | |
|---|---|---|---|---|
| **iPS:434649** | **21-225_78E11** | NA | GACATCGTGATGACCCAGTCTCCAGACTCCCTGGCTGTGTCTCTGGGCGAGAGGGCCACCATCAACTGCAAGTCCAGCCAGAGTGTTTTATACAGCTTCAACAATTACAACTACTTAGCTTGGTACCAGCAGAAACCAGGACAGCCTCCTAAGAAGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTCCTGTCAGCAATATTATAGTAGTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTGTGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGAGTAGCAGTGGCTGGAACTTCTTTGACTACTGGGGCCAGGGAACCCTGGTCACCGTCTCCTCA |
| | | | SEQ ID NO: 25827 | SEQ ID NO: 29833 |
| | | AA | DIVMTQSPDSLAVSLGERATINCKSSQSVLYSFNNYNYLAWYQQKPGQPPKKLIYWASTRESGVPDRFSGSGSGTDFTLTISSLQAEDVAVYSCQQYYSSPPTFGQGTKVEIK | QVQLVQSGAEVKKPGASVKVSCKASGYTFTNYDINWVRQATGQGLEWMGWMNPNSGNTGCAQKFQGRVTMTRNTSISTAYMELSSLRSEDTAVYYCASSSGWNFFDYWGQGTLVTVSS |
| | | | SEQ ID NO: 25828 | SEQ ID NO: 29834 |
| **iPS:434653** | **21-225_74B5** | NA | GACATCGTTATGACCCAGTCTCCAGACTCCCTGGCTGTGTCTCTGGGCGAGAGGGCCACCATCAACTGCAAGTCCAGCCAGAGTGTTTTATTCAGCTCCAACAATTATAACTACTTAGCTTGGTACCAGCAGAGACCAGGACAGCCTCCTAACCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGCTGATGTGGCAGTTTATTACTGTCAGCAATATTATAGTTCTCCTCCGACGTTCGGCCAAGGGACCACGGTGCAAATCAAA | CAGGTGCAGTTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCACAAGCACAGCCCACATGGAGCTGAGCAGCCTGAGATCTGAGGACACGGCCGTGTATTACTGTGCGGTCTCCAGTGGCTGGAACTGGTTCGACCCCTGGGGCCAGGGAACCCTGGTCACCGTCTCCTCA |
| | | | SEQ ID NO: 25829 | SEQ ID NO: 29835 |

FIGURE 50

| | | | | |
|---|---|---|---|---|
| | | AA | DIVMTQSPDSLAVSLGERATINCKSSQSVLFSSNNYNYLAWYQQRPGQPPNLLIYWASTRESGVPDRFSGSGSGTDFTLTISSLQAADVAVYYCQQYYSSPPTFGQGTTVQIK | QVQLVQSGAEVKKPGASVKVSCKASGYTFTNYDINWVRQATGQGLEWMGWMNPNSGNTGYAQKFQGRVTMTRNTSTSTAHMELSSLRSEDTAVYYCAVSSGWNWFDPWGQGTLVTVSS |
| | | | SEQ ID NO: 25830 | SEQ ID NO: 29836 |
| **iPS:434655** | **21-225_78H12** | NA | GACATCGTGATGACCCAGTCTCCAGACTCCCTGGCTGTGTCTCTGGGCGAGAGGGCCACCATCAACTGCAAGTCCAGCCAGACTGTTTTACACAGCTTCAACAATTATAACTACTTAGCTTGGTACCAGCAGAAGCCAGGACAGCCCCCTAAGGTGCTCATTTACTGGACATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTTTAGTAGTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCCCCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTATTGTGCGGTTTCCAGTGGCTGGCACTGGTTCGACCCCTGGGGCCAGGGAACCCTGGTCACCGTCGCCTCA |
| | | | SEQ ID NO: 25831 | SEQ ID NO: 29837 |
| | | AA | DIVMTQSPDSLAVSLGERATINCKSSQTVLHSFNNYNYLAWYQQKPGQPPKVLIYWTSTRESGVPDRFSGSGSGTDFTLTISSLQAEDVAVYYCQQYFSSPPTFGQGTKVEIK | QVQLVQSGAEVKKPGASVKVSCKASGYTFPNYDINWVRQATGQGLEWMGWMNPNSGNTGYAQKFQGRVTMTRNTSISTAYMELSSLRSEDTAVYYCAVSSGWHWFDPWGQGTLVTVAS |
| | | | SEQ ID NO: 25832 | SEQ ID NO: 29838 |

FIGURE 50

| | | | | |
|---|---|---|---|---|
| **iPS:434657** | **21-225_79G1** | NA | GAAATTGTGTTGACGCAGTCTCCAGGCACCCT<br>GTATTTGTCTTCAGGGGAAAGAGCCACCCTCT<br>CCTGCAGGGCCAGTCAGAGTGTTTACAGCAGC<br>TACTTAGCCTGGTACCAGCAGAAACCTGCCCA<br>GGCTCCCAGGCTCCTCATTTATGGTGCATCCA<br>GCCGGTCCACTGGCATCCCAGACAGGTTCAGT<br>GGCAGTGGGTCTGGGACAGACTTCGCTCTCAC<br>CATCAGCAGACTGGAGCCTGAAGATTTTGCAG<br>TGTATTACTGTCAGCACTATGATAACTCACCG<br>TGGACGTTCGGCCAAGGGACCAAGGTGGAAA<br>TCAAA | CAGGTGCAGCTACAGCAGTGGGGCGCAGGACTG<br>TTGAAGCCTTCGGAGACCCTGTCCCTCACCTGCG<br>CTGTCCATGGTGGGTCCTTCAGTGGTTGCTACTG<br>GAGCTGGATCCGCCAGCCCCCAGGGAAGGGGCT<br>GGAGTGGATTGGGGAAATCAATCATAGTGGAAG<br>CACCAACTACAACCCGTCCCTCAAGAGTCGAGTC<br>ACCATTTCAGTAGACACGTCCAAGAACCAGTTCT<br>CCCTGAAGCTGAGCTCTGTGACCGCCGCGGACAC<br>GGCTGTGTATTACTGTGCGAGGGACTACGGCGGT<br>ATGGACGTCTGGGGCCAAGGGACCACGGTCACC<br>GTCTCCTCA |
| | | | SEQ ID NO: 25833 | SEQ ID NO: 29839 |
| | | AA | EIVLTQSPGTLYLSSGERATLSCRASQSVYSSYLA<br>WYQQKPAQAPRLLIYGASSRSTGIPDRFSGSGSG<br>TDFALTISRLEPEDFAVYYCQHYDNSPWTFGQG<br>TKVEIK | QVQLQQWGAGLLKPSETLSLTCAVHGGSFSGCYW<br>SWIRQPPGKGLEWIGEINHSGSTNYNPSLKSRVTISV<br>DTSKNQFSLKLSSVTAADTAVYYCARDYGGMDV<br>WGQGTTVTVSS |
| | | | SEQ ID NO: 25834 | SEQ ID NO: 29840 |
| **iPS:434663** | **21-225_79F3** | NA | GAAATTGTGTTGACGCAGTCTCCAGGCACCCT<br>GTATTTGTCTCCAGGGGAAAGAGCCACCCTCT<br>CCTGCAGGGCCAGTCAGAGTGTTTACAGCAGC<br>TACTTAGCCTGGTACCAGCAGAAACCTGCCCA<br>GGCTCCCAGGCTCCTCATCTATGGTGCATCCA<br>GCCGGTCCACTGGCATCCCAGACAGGTTCAGT<br>GGCAGTGGGTCTGGGACAGACTTCGCTCTCAC<br>CATCAGCAGACTGGAGCCTGAAGATTTTGCAG<br>TGTATTACTGTCAGCACTATGATAACTCACCG<br>TGGACGTTCGGCCAAGGGACCAAGGTGGAAA<br>TCAAA | CAGGTGCAGCTACAGCAGTGGGGCGCAGGACTG<br>TTGAAGCCTTCGGAGACCCTGTCCCTCACCTGCG<br>CTGTCCATGGTGGGTCCTTCAGTGGTTGCTACTG<br>GAGCTGGATCCGCCAGCCCCCAGGGAAGGGGCT<br>GGAGTGGATTGGGGAAATCAATCATAGTGGAAG<br>CACCAACTACAACCCGTCCCTCAAGAGTCGAGTC<br>ACCATTTCAGTAGACACGTCCAAGAACCAGTTCT<br>CCCTGAAGCTGAGCTCTGTGACCGCCGCGGACAC<br>GGCTGTGTATTACTGTGCGAGGGACTACGGCGGT<br>ATGGACGTCTGGGGCCAAGGGACCACGGTCACC<br>GTCTCCTCA |
| | | | SEQ ID NO: 25835 | SEQ ID NO: 29841 |

FIGURE 50

| | | | | |
|---|---|---|---|---|
| | | AA | EIVLTQSPGTLYLSPGERATLSCRASQSVYSSYLAWYQQKPAQAPRLLIYGASSRSTGIPDRFSGSGSGTDFALTISRLEPEDFAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25836 | SEQ ID NO: 29842 |
| iPS:434665 | 21-225_74G4 | NA | GACATCGTGATGACCCAGTCTCCAGACTCCCTGGCTGTGTCTCTGGGCGAGAGGGCCACCATCAACTGCAAGTCCAGCCAGAGTGTTTTATACAGTTCCAACAATAATAACTACTTAGCTTGGTACCAGCAGAAGGCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCGGAAGATGTGGCAGTTTATTACTGTCAGCAATATTATAGTATTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATGTCAACTGGGTGCGACAGGCCACTGGACAAGGGCTTGAATGGATGGGATGGATGCACCCTAACAGTGGTAACACAGGCTATGCACAGAAGTTCCAGGGCAGAGTCACCATGACCAGGAACACCTCCATACGCACAGCCTACATGGAGCTGAGCAGCCTGAGATCTGAGGACACGGCCGTGTATTACTGTGCGAGTAGCAGTGGCTGGTACCATTTTGACTACTGGGGCCAGGGAACCCTGGTCACCGTCTCCTCA |
| | | | SEQ ID NO: 25837 | SEQ ID NO: 29843 |
| | | AA | DIVMTQSPDSLAVSLGERATINCKSSQSVLYSSNNNNYLAWYQQKAGQPPKLLIYWASTRESGVPDRFSGSGSGTDFTLTISSLQAEDVAVYYCQQYYSIPPTFGQGTKVEIK | QVQLVQSGAEVKKPGASVKVSCKASGYTFTNYDVNWVRQATGQGLEWMGWMHPNSGNTGYAQKFQGRVTMTRNTSIRTAYMELSSLRSEDTAVYYCASSSGWYHFDYWGQGTLVTVSS |
| | | | SEQ ID NO: 25838 | SEQ ID NO: 29844 |

FIGURE 50

| | | | | |
|---|---|---|---|---|
| **iPS:434669** | **21-225_79F4** | NA | GACATCCAGATGACCCAGTCTCCATCCTCACTGTCTGCATCTGTAGGAGACAGAGTCACCATCACTTGTCGGGCGAGTCAGGGCATTAGCAAGTATTTAGCCTGGTTTCAGCAGAAACCAGGGAAAGCCCCTAAGTCCCTGATCTATGCTGCATCCAGTTTGCAAGGTGGGGCCCCATCTAAGTTCAGCGGCAGTGGATCTGGGACAGATTTCACTCTCACCATCAGCAGCCTGCAGCCTGAAGATTTTGCAACTTATTACTGCCAACAGTACAGTAATTACCCACTCACTTTCGGCGGAGGGACCAGGGTGGAGATCAAA | CAGGTGCAGTTGGTGGAGTCTGGGGGAGGCGTGGTCCAGTCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGATGGAAGTAATCAAAACTATGCAGACTCCGTGAAGGGCCGATTCACCATCTCCAGAGACAATTCCAAGAACACGCTGTTTCTGCAAATGAACAGCCTGAGAGCCGAGGACACGGCTGTGTACTACTGTGCGAGAGATGGCAGCTATGGTTATGACGGCCTTGACTACTGGGGCCAGGGAACCCTGGTCACCGTCTCCTCA |
| | | | SEQ ID NO: 25839 | SEQ ID NO: 29845 |
| | | AA | DIQMTQSPSSLSASVGDRVTITCRASQGISKYLAWFQQKPGKAPKSLIYAASSLQGGAPSKFSGSGSGTDFTLTISSLQPEDFATYYCQQYSNYPLTFGGGTRVEIK | QVQLVESGGGVVQSGRSLRLSCAASGFTFSNYGMHWVRQAPGKGLEWVAVIWYDGSNQNYADSVKGRFTISRDNSKNTLFLQMNSLRAEDTAVYYCARDGSYGYDGLDYWGQGTLVTVSS |
| | | | SEQ ID NO: 25840 | SEQ ID NO: 29846 |
| **iPS:434671** | **21-225_74F4** | NA | GAAATTGTGTTGACGCAGTCTCCAGGCACCCTGTCTTTGTCTCCAGGGGAAAGAGCCACCCTCTCCTGCAGGGCCAGTCAGATTTTTAGCAGCAGCTACTTAGCCTGGTACCAGCAGAAACCTGGCCAGTCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGGTAGCTCACGGACGTTCGGCCAAGGGACCAAGGTGGAAATCAAA | GAGGTGCAGCTGGTGGAGTCTGGGGGAGGCTTGGTAAAGCCTGGGGGGTCCCTTAGACTCTCCTGTGCAGCCTCTGGATTCACTTTCAGTAACGCCTGGATGAACTGGGTCCGCCAGGCTCCAGGGAAGGGGCTGGAGTGGGTTGGCCGAATTAAAAACAAAATTGATGGTGGGACAACAGACTACGCTGCACCCGTGAAAGGCAGATTCACCATCTCAAGAGATGATTCAAAAAACACGCTGTATCTGCAAATGAACAGCCTGAAAACCGAGGACACAGCCGTGTATTACTGTACCACAGTGGGAGCTACTACGGACTACTGGGGCCAGGGAACCCTGGTCACCGTCTCCTCA |
| | | | SEQ ID NO: 25841 | SEQ ID NO: 29847 |

FIGURE 50

| | | | | |
|---|---|---|---|---|
| | | AA | EIVLTQSPGTLSLSPGERATLSCRASQIFSSSYLAWYQQKPGQSPRLLIYGASSRATGIPDRFSGSGSGTDFTLTISRLEPEDFAVYYCQQYGSSRTFGQGTKVEIK | EVQLVESGGGLVKPGGSLRLSCAASGFTFSNAWMNWVRQAPGKGLEWVGRIKNKIDGGTTDYAAPVKGRFTISRDDSKNTLYLQMNSLKTEDTAVYYCTTVGATTDYWGQGTLVTVSS |
| | | | SEQ ID NO: 25842 | SEQ ID NO: 29848 |
| iPS:434673 | 21-225_74E3 | NA | GAAATAGTGATGACGCAGTCTCCAGCCACCCTGTCTCTGTCTCCAGGGGAAAGAGCCACCCTCTCCTGCAGGGCCAGTCTGAGTGTTGTCAACAGCTTAGCCTGGTACCAGCAGAAACCTGGCCAGGCTCCCAGGCTCCTCATCTATGGTGCATCCACCAGGGCCACTGGTATCCCAGCCAGGTTCAGTGGCAGTGGGTCTGGGACAGAGTTCACTCTCACCATCAGCAGCCTGCAGTCTGAAGATTTTGCAATTTATTACTGTCAGCAGTATAATGACTGGCCGTGCAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTACAGCGTCTGGATTCACCTTCAGTAGCTATGGCATGCACTGGGTCCGCCAGGCTCCAGGCAAGGGGCTGGAGTGGGTGGCAGTTATTTGGTATGATGGAAGTAATAAATACTATGCAGACTCCGTGAAGGGCCGATTCACCATCTCCAGAGACAATTCCAAGAACACGCTGTTTCTGCAAATGAACAGCCTGAGAGCCGAGGACACGGCTGTGTATTACTGTGCGAGCGATCGCAATATAGTGGGAGCTACTTACTTTGAGTCCTGGGGCCAGGGAACCCTGGTCACCGTCTCCTCA |
| | | | SEQ ID NO: 25843 | SEQ ID NO: 29849 |
| | | AA | EIVMTQSPATLSLSPGERATLSCRASLSVVNSLAWYQQKPGQAPRLLIYGASTRATGIPARFSGSGSGTEFTLTISSLQSEDFAIYYCQQYNDWPCSFGQGTKLEIK | QVQLVESGGGVVQPGRSLRLSCTASGFTFSSYGMHWVRQAPGKGLEWVAVIWYDGSNKYYADSVKGRFTISRDNSKNTLFLQMNSLRAEDTAVYYCASDRNIVGATYFESWGQGTLVTVSS |
| | | | SEQ ID NO: 25844 | SEQ ID NO: 29850 |

FIGURE 50

| | | | | |
|---|---|---|---|---|
| **iPS:434675** | **21-225_79G6** | NA | GACATCGTGATGACCCAGTCTCCAGACTGCCT GGCTGTGTCTCTGGGCGAGAGGGCCACCATCA GCTGCATGTCCAGCCAGAGTGTTTTACACAGC TTCAACAATAAGAACTACTTAACTTGGTACCA GCAGAAACCAGGACAGCCTCCTAAGCTGCTCA TTTACTGGGCATCTACTTGGGAATCCGGGGTC CCTGACCGATTCAGTGGCAGCGGGTCTGGGAC AGATTTCAGTCTCCCGATCGGCAGCCTGCAGG CTGAAGATGTGGCAGTTTATTACTGTCAGCAA TATTATAGTATTCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATTCACCTTCACCAATTATGATA TCAACTGGGTGCGACAGGCCACTGGACAAGGGC TTGAGTGGATGGGATGGATGCACCCTAACAGTGG TAACACAGGCTTTGCACAGAAGTTCCAGGGCAG AGTCACCATGACCAGGAACACCTCCATAAGCAC AGCCTACATGGAACTGAGCAGCCTGAGATCTGA GGACACGGCCGTGTATTACTGTGCGGTTTCCAGT GGCTGGTACTGGTTCGACCCCTGGGGCCAGGGA ACCCTGGTCACCGTCTCCTCA |
| | | | SEQ ID NO: 25845 | SEQ ID NO: 29851 |
| | | AA | DIVMTQSPDCLAVSLGERATISCMSSQSVLHSFN NKNYLTWYQQKPGQPPKLLIYWASTWESGVPD RFSGSGSGTDFSLPIGSLQAEDVAVYYCQQYYSI PPTFGQGTKVEIK | QVQLVQSGAEVKKPGASVKVSCKASGFTFTNYDIN WVRQATGQGLEWMGWMHPNSGNTGFAQKFQGR VTMTRNTSISTAYMELSSLRSEDTAVYYCAVSSGW YWFDPWGQGTLVTVSS |
| | | | SEQ ID NO: 25846 | SEQ ID NO: 29852 |
| **iPS:434679** | **21-225_79G7** | NA | GACATCGTGATGACCCAGTCTCCAGACTCCCT GGCTGTGTCTCTGGGCGAGAGGGCCACCATCA ACTGCAAGTCCAGCCAGAGTGTTTTGTACAGC TCCAACAGTCACAACTACTTAGCTTGGTACCA GCAGAAACCAGGACAGCCTCCTAAACTGCTCA TTTTCTGGGCATCTATCCGGGAATCCGGGGTC CCTGACCGATTCAGTGGCAGCGGGTCTGGGAC AGATTTCACTCTCTCCATCAGCAGCATGCAGG CTGAAGATGTGGCAGTTTATTACTGTCAGCAA TATTATAGTACTCCGTGCAGTTTTGGCCAGGG GACCAAGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCTCTGGACAAGGAC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACTCGGCCGTATATTACTGTGCGTATAGTAGT GGCTGGTACAAATTTGACTACTGGAGCCAGGGA ACCCTGGTCACCGTCTCCTCA |
| | | | SEQ ID NO: 25847 | SEQ ID NO: 29853 |

FIGURE 50

| | | | | |
|---|---|---|---|---|
| | | AA | DIVMTQSPDSLAVSLGERATINCKSSQSVLYSSN SHNYLAWYQQKPGQPPKLLIFWASIRESGVPDR FSGSGSGTDFTLSISSMQAEDVAVYYCQQYYSTP CSFGQGTKLEIK | QVQLVQSGAEVKKPGASVKVSCKASGYTFTNYDIN WVRQASGQGLEWMGWMHPNSGNTGYAQKFQGR VTMTRNTSISTAYMELSSLRSEDSAVYYCAYSSGW YKFDYWSQGTLVTVSS |
| | | | SEQ ID NO: 25848 | SEQ ID NO: 29854 |
| iPS:434685 | 21-225_79E9 | NA | GACATCGTGATGACCCAGTCTCCAGACTCCCT GGCTGTGTCTCTGGGCGAGAGGGCCACCATCA ACTGTAAGTCCAGCCAGAGTATTTTATACAGC TCCAACAATAATAACTACTTAGCTTGGTTCCA GCAGAAACCAGGACAGCCTCCTAAGCTGCTCA TTTATTGGGCATCTACCCGGGAATCCGGGGTC CCTGACCGATTCAGTGGCAGCGGGTGTGGGAC AGATTTCACTCTCACCATCAGCAGCCTGCAGG CTGAAGATGTGGCAGTTTATTACTGTCAGCAA TATTATATTACTCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCATCCTAACAGTGG TAACACAGGCTATGCACAGAAGTTCCAGGGCAG AGTCACCATGACCAGGAACACCTCCATAAGCAC AGCCTACATGGAACTGAGCAGCCTGAGATCTGA GGACACGGCCGTGTATTACTGTGCGAGTAGCAGT GGCTGGTACTACTTTGACTACTGGGGCCAGGGAA CCCTGGTCACCGTCTCCTCA |
| | | | SEQ ID NO: 25849 | SEQ ID NO: 29855 |
| | | AA | DIVMTQSPDSLAVSLGERATINCKSSQSILYSSNN NNYLAWFQQKPGQPPKLLIYWASTRESGVPDRF SGSGCGTDFTLTISSLQAEDVAVYYCQQYYITPP TFGQGTKVEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCASSSGW YYFDYWGQGTLVTVSS |
| | | | SEQ ID NO: 25850 | SEQ ID NO: 29856 |

FIGURE 50

| | | | | |
|---|---|---|---|---|
| **iPS:434687** | **21-225_75A5** | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGGCCACTGGCATCCCAGACAGGTTCAGTGGCAGTGGGTCTGGGACAGACTTCGCTCTCACCATCAGCAGACTGGAGCCTGAAGATTTTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25851 | SEQ ID NO: 29857 |
| | | AA | EIVLTQSPGTLYLSPGERATLSCRASQSVYSSYLAWYQQKPGQAPRLLIYGASSRATGIPDRFSGSGSGTDFALTISRLEPEDFAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25852 | SEQ ID NO: 29858 |
| **iPS:434689** | **21-225_79G10** | NA | GACATCGTTATGACCCAGTCTCCAGACTCCCTGGCTGTGTCTCTGGGCGAGAGGGCCACCATCAATTGCAAGTCCAGCCAGAGTGTTTTATTCAGCTCCAACAATTATAATTACTTAGCTTGGTACCAGCAGAGACCAGGACAGCCTCATAACCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGCTGATGTGGCAGTTTATTACTGTCAGCAATATCATAGTTCTCCTCTGACGTTCGGCCAAGGGACCACGGTGCAAATCAAA | CAGGTGCAGTTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCACAAGCACAGCCCACATGGAGCTGAGCAGCCTGAGATCTGAGGACACGGCCGTGTATTACTGTGCGGTCTCCAGTGGCTGGAACTGGTTCGACCCCTGGGGCCAGGGAACCCTGGTCACCGTCTCCTCA |
| | | | SEQ ID NO: 25853 | SEQ ID NO: 29859 |

FIGURE 50

| | | | | |
|---|---|---|---|---|
| | | AA | DIVMTQSPDSLAVSLGERATINCKSSQSVLFSSNNYNYLAWYQQRPGQPHNLLIYWASTRESGVPDRFSGSGSGTDFTLTISSLQAADVAVYYCQQYHSSPLTFGQGTTVQIK | QVQLVQSGAEVKKPGASVKVSCKASGYTFTNYDINWVRQATGQGLEWMGWMNPNSGNTGYAQKFQGRVTMTRNTSTSTAHMELSSLRSEDTAVYYCAVSSGWNWFDPWGQGTLVTVSS |
| | | | SEQ ID NO: 25854 | SEQ ID NO: 29860 |
| **iPS:434691** | **21-225_75G7** | NA | GAAATTGTGTTGACGCAGTCTCCAGGCACCCTGTCTTTGTCTCCAGGGGAAAGAGCCACCCTCTCCTGCAGGGCCAGGCAGAGTGTTGACAGCAGTTATTTAGCCTGGTACCAGCAGAAACGTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGGCTGGAGCCTGAAGATTTTGCAGTATATTACTGTCAGCAGTATGAAAGCTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCACTGTCTATGGTGGGGCCTTCAGTGGTTCCTACTGGAGCTGGATCCGCCAGTCCCCAGGGAAGGGGCTGGAGTGGATTGGGGAAATCAATTATAGGGGAAGCACCAACTACAACCCGTCCCTCAAGAGTCGAGTCACCATATCAGTAGACACGTCCAAGAACCAGTTCTCCCTGAACCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25855 | SEQ ID NO: 29861 |
| | | AA | EIVLTQSPGTLSLSPGERATLSCRARQSVDSSYLAWYQQKRGQAPRLLIYGASSRATGIPDRFSGSGSGTDFTLTISRLEPEDFAVYYCQQYESSPWTFGQGTKVEIK | QVQLQQWGAGLLKPSETLSLTCTVYGGAFSGSYWSWIRQSPGKGLEWIGEINYRGSTNYNPSLKSRVTISVDTSKNQFSLNLSSVTAADTAVYYCARDYGGMDVWGQGTTVTVSS |
| | | | SEQ ID NO: 25856 | SEQ ID NO: 29862 |

FIGURE 50

| | | | | |
|---|---|---|---|---|
| **iPS:434693** | **21-225_79F11** | NA | GAAATTGTGTTGACGCAGTCTCCAGGCACCCTGTCTTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGGCCACTGGCATCCCAGACAGGTTCAGTGGCAGTGGGTCTGGGACAGACTTCGCTCTCACCATCAGCAGACTGGAGCCTGAAGATTTTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25857 | SEQ ID NO: 29863 |
| | | AA | EIVLTQSPGTLSLSPGERATLSCRASQSVYSSYLAWYQQKPGQAPRLLIYGASSRATGIPDRFSGSGSGTDFALTISRLEPEDFAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25858 | SEQ ID NO: 29864 |
| **iPS:434697** | **21-225_79F12** | NA | GACATCGTGATGACCCAGTCTCCAGACTCCCTGGCTGTGTCTCTGGGCGAGAGGGCCACCATCAACTGCAAGTCCAGCCAGAGTATTTTATACAGCTCCAACAATTACAACTACTTAGCTTGGTACCAGCAGAAACCAGGACAGCCTCCTAAGTTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCGTGGACGTTCGGCCAAGGGACCAAGGTGGAAATCAAA | CAGGTGCAGCTGGTGCAGTCTGGGGCTGAGGTGAAGAAGCCTGGGGCCTCTGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGAAACACCTCCATAAGCACAGCCTACATGGAGCTGAGCAGCCTGAGATCTGAGGACACGGCCGTATATTACTGTGCGAGTAGCAGTGGCTGGTACTTCTTTGACTACTGGGGCCAGGGAACCCTGGTCACCCTCTCCTCA |
| | | | SEQ ID NO: 25859 | SEQ ID NO: 29865 |

FIGURE 50

| | | | | |
|---|---|---|---|---|
| | | AA | DIVMTQSPDSLAVSLGERATINCKSSQSILYSSNNYNYLAWYQQKPGQPPKLLIYWASTRESGVPDRFSGSGSGTDFTLTISSLQAEDVAVYYCQQYYSTPWTFGQGTKVEIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYYCASSSGWYFFDYWGQGTLVTLSS |
| | | | SEQ ID NO: 25860 | SEQ ID NO: 29866 |
| iPS:434699 | 21-225_79G12 | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGACTGGAGCCTGAAGATTGTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25861 | SEQ ID NO: 29867 |
| | | AA | EIVLTQSPGTLYLSPGERATLSCRASQSVYSSYLAWYQQKPGQAPRLLIYGASSRSTGIPDRFSGSGSGTDFALTISRLEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25862 | SEQ ID NO: 29868 |

FIGURE 50

| | | | | |
|---|---|---|---|---|
| **iPS:434701** | **21-225_80A1** | NA | GAAATTGTGTTGACGCAGTCTCCAGGCACCCGGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTGTGGGACAGACTTCGCTCTCACCATCAGCAGAGTGGAGCCTGAAGATTGTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25863 | SEQ ID NO: 29869 |
| | | AA | EIVLTQSPGTRYLSPGERATLSCRASQSVYSSYLAWYQQKPGQAPRLLIYGASSRSTGIPDRFSGSGCGTDFALTISRVEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25864 | SEQ ID NO: 29870 |
| **iPS:434703** | **21-225_80C1** | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGAGTGGAGCCTGAAGATTGTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25865 | SEQ ID NO: 29871 |

FIGURE 50

| | | | | |
|---|---|---|---|---|
| | | AA | EIVLTQSPGTLYLSPGERATLSCRASQSVYSSYLAWYQQKPGQAPRLLIYGASSRSTGIPDRFSGSGSGTDFALTISRVEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25866 | SEQ ID NO: 29872 |
| iPS:434705 | 21-225_80A2 | NA | GAATTTATGTTGACGCAGTCTCCAGGCACCCTGTATTTGTCTCCAGGGGAAAGAGCCACCCTCTCGTGCAGGGCCAGTCAGAGTGTTAGCAGCAGCTACTTAGTCTGGTATCAGCAGAAACCTGGCCAGGCTCCCAGGCTCCTCATCTATGGTGCATCCACCAGGGCCACTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTTGCTCACCGCTCACTTTCGGCGGAGGGACCAAGGTGGAGATCACA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGTCCCTGGACAAGGGCTTGAGTGGATGGGATGGATGCACCCTAACAGTGGTAACACAGGCTATGCACAGAAGTTCCAGGGCAGAGTCACCATGACCAGGAACACCTCCATCAGCACAGCCTACATGGAGCTGAGCAGCCTGAGATCTGAGGACACGGCCGTGTATTACTGTGCGATTAGTAGTGGCTGGTACTGGTTCGACCCCTGGGGCCAGGGAACCCTGGTCACCGTCTCCTCA |
| | | | SEQ ID NO: 25867 | SEQ ID NO: 29873 |
| | | AA | EFMLTQSPGTLYLSPGERATLSCRASQSVSSSYLVWYQQKPGQAPRLLIYGASTRATGIPDRFSGSGSGTDFTLTISRLEPEYFAVYYCQQYGCSPLTFGGGTKVEIT | QVQLVQSGAEVKKPGASVKVSCKASGYTFTNYDINWVRQVPGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25868 | SEQ ID NO: 29874 |

FIGURE 50

| | | | | |
|---|---|---|---|---|
| **iPS:434707** | **21-225_80D3** | NA | GACATCGTGATGACCCAGTCTCCAGATTGCCTGGCTGTGTCTCTGGGCGAGAGGGCCACCATCAACTGCAAGTCCAGCCAGAGTGTTTTATACACCTCCAACAATAACAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTTGCAGTTTATTATTGTCAGCACTACAATGAAACTCCAGGGAAGTTTGTCCAAGTGACCAAGGTGGAAATCACA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTCA |
| | | | SEQ ID NO: 25869 | SEQ ID NO: 29875 |
| | | AA | DIVMTQSPDCLAVSLGERATINCKSSQSVLYTSNNNNYLAWYQQKPGQPPKLLIYWASTRESGVPDRFSGSGSGTDFTLTISSLQAEDVAVYYCQHYNETPGKFVQVTKVEIT | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25870 | SEQ ID NO: 29876 |
| **iPS:434709** | **21-225_80E3** | NA | GAAATTGTGTTGACGCAGTCTCCAGGCACCCTGTATTTGTCTTCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTGTGGGACAGACTTCGCTCTCACCATCAGCAGAGTGGAGCCTGAAGATTTTGCAGTGTATTACTGTCAGCATTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25871 | SEQ ID NO: 29877 |

FIGURE 50

| | | | | |
|---|---|---|---|---|
| | | AA | EIVLTQSPGTLYLSSGERATLSCRASQSVYSSYLAWYQQKPGQAPRLLIYGASSRSTGIPDRFSGSGCGTDFALTISRVEPEDFAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25872 | SEQ ID NO: 29878 |
| iPS:434711 | 21-225_80H3 | NA | GACATCGTGATGACCCAGTCTCCAGACTCCCTGGCTGTGTCTCTGGGCGAGAGGGCCACCACCAATTGTAAGTCCAGCCAGAGTGTTTTACACAGGTCCAACAATTACAACTACTTAGCGTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTGACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAACTGAGCAGCCTGAGATCTGAGGACACGGCCGTGTATTACTGTGGGAGTACCAGTGGCTGGAACTTCTTTGACTACTGGGGCCAGGGAACCCTGGTCACCGTCTCCTCA |
| | | | SEQ ID NO: 25873 | SEQ ID NO: 29879 |
| | | AA | DIVMTQSPDSLAVSLGERATTNCKSSQSVLHRSNNYNYLAWYQQKPGQPPKLLIYWASTRESGVPDRFSGSGSGTDFTLTISSLQAEDVAVYYCQQYYSTPPTFGQGTKVEIK | QVQLVQSGAEVKKPGASVKVSCKASGYTLTNYDINWVRQATGQGLEWMGWMNPNSGNTGYAQKFQGRVTMTRNTSISTAYMELSSLRSEDTAVYYCGSTSGWNFFDYWGQGTLVTVSS |
| | | | SEQ ID NO: 25874 | SEQ ID NO: 29880 |

FIGURE 50

| | | | | |
|---|---|---|---|---|
| **iPS:434715** | **21-225_80D5** | NA | GAACTTGTGTTGACGCAGTCTCCAGGCACCCTGTCTTTGTCTCCAGGGAAAAGAGTCACCCTCTCCTGCAGGGCCAGTCAGAATATTTACAGCAGCTACTTAGCCTGGTACCAGCAGAAACCTGGCCAGGCTCCCAGACTCCTCATCTATGGTGCATCCAGCAGGGCCACTGGCATCCCAGACAGGTTCAGTGGCAGTGGGTCTGGGACAGACTTCACTCTCACCATCACCAGACTGGAGCCTGAAGATTTTGCAGTGTATTGCTGTCAGCAGTATGAAAGCTCACCGTGGACCTTCGGCCAAGGGACCAAGGTGGAAATCAAA | CAAGTGCAGCTACAGCAGTGGGGCGCAGGACTGTTGAAGCCTTCGGAGCCCCTGTCCCTCACCTGCGCTGTCTATGGTGGGTCCTTCAGTGGTCCCTACTGGAGCTGGATCCGCCAGCCCCCGGGAAGGGGCTGGAGTGGATTGGGGAAATCAATCATAGTGGAAGCACCAACTACAACCCGTCCCTCAAGAGTCGAGTCACCATATCAGTAGACACGTCCAAGAACCAGTTCTCCCTGAAACTGACCTCTGTGACCGCCGCGGACATGGCTGTGTATTACTGTGCGAGAGACTACGGGGGTTTGGACGTCTGGGGCCAAGGGACCACGGTCACCGTCTCCTCA |
| | | | SEQ ID NO: 25875 | SEQ ID NO: 29881 |
| | | AA | ELVLTQSPGTLSLSPGKRVTLSCRASQNIYSSYLAWYQQKPGQAPRLLIYGASSRATGIPDRFSGSGSGTDFTLTITRLEPEDFAVYCCQQYESSPWTFGQGTKVEIK | QVQLQQWGAGLLKPSEPLSLTCAVYGGSFSGPYWSWIRQPPGKGLEWIGEINHSGSTNYNPSLKSRVTISVDTSKNQFSLKLTSVTAADMAVYYCARDYGGLDVWGQGTTVTVSS |
| | | | SEQ ID NO: 25876 | SEQ ID NO: 29882 |
| **iPS:434717** | **21-225_80A6** | NA | GAAATTGTGTTGACGCAGTCTCCAGGCACCCTGTCTTTGTCTCCAGGGGAAATACCCACCCTCTCCTGCAGGGCCAGTCAGAGTGTTGACAGCGGCTACTTAGCCTGGTACCAGCAGAAACCTGGCCAGGCTCCCAGGCTCCTCATCTATGGTGCATCCAGCAGGGCCCCTGGCATCCCAGACAGGTTCAGTGGCAGTGGGTCTGGGACAGACTTCACTCTCACCATCAGCAGACTGGAACCTGAAGATTTTGCAGTGTATTACTGTCAGCAGTATGAAAGCTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGGTTGCTACTGGAGCTGGATCCGCCAGCCCCCAGGAAAGGGGCTGGAGTGGATTGGGGAAATCAATCATAGTGGAAGGACCAACTACAACCCGTCCCTCAAGAGTCGAGTCACCATATCAGTAGACACGTCCGAGAAGCAGTTCTCCCTGAAGCTGAGCTCTGTGACCGCCGCGGACACGGCTGTGTATTACTGTGCGAGAGACTACGGTGGGCTTGACTACTGGGGCCAGGGAACCCTGGTCACCGTCTCCTCA |
| | | | SEQ ID NO: 25877 | SEQ ID NO: 29883 |

FIGURE 50

| | | | | |
|---|---|---|---|---|
| | | AA | EIVLTQSPGTLSLSPGEIPTLSCRASQSVDSGYLAWYQQKPGQAPRLLIYGASSRAPGIPDRFSGSGSGTDFTLTISRLEPEDFAVYYCQQYESSPWTFGQGTKVEIK | QVQLQQWGAGLLKPSETLSLTCAVYGGSFSGCYWSWIRQPPGKGLEWIGEINHSGRTNYNPSLKSRVTISVDTSEKQFSLKLSSVTAADTAVYYCARDYGGLDYWGQGTLVTVSS |
| | | | SEQ ID NO: 25878 | SEQ ID NO: 29884 |
| iPS:434725 | 21-225_80H7 | NA | GAAATTGTGTTGACGCAGTCTCCAGGCACCCTGTCTTTGTCTCCAGGGGAAAGAGCCACCCTCTCCTGCAGGGCCAGTCAGAGTATTAACAGCAACTACTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTTTTACTGTCAGCAGTATGAGAGCTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTATGTTGGGTCCTTCAGTGGTTCCTACTGGAGCTGGATCCGCCAGCCCCCAGGGAAGGGGCTAGAGTGGATTGGGGAAATCAATCAAAGTGGACGCACCAACTACAACCCGTCCCTCAAGAGTCGAGTCACCATATCAGTAGACACGTCCAAGAACCAGTTCTCCCTGAAGCTGAGCTCTGTGACCGCCGCGGACACGGCTGTGTATTACTGTGCGAGAGACTACGGGGGTATAGACGTCTGGGGCCAAGGGACCACGGTCACCGTCTCCTCA |
| | | | SEQ ID NO: 25879 | SEQ ID NO: 29885 |
| | | AA | EIVLTQSPGTLSLSPGERATLSCRASQSINSNYLAWYQQKPGQAPRLLIYGASSRATGIPDRFSGSGSGTDFTLTISRLEPEDFAVFYCQQYESSPWTFGQGTKVEIK | QVQLQQWGAGLLKPSETLSLTCAVYVGSFSGSYWSWIRQPPGKGLEWIGEINQSGRTNYNPSLKSRVTISVDTSKNQFSLKLSSVTAADTAVYYCARDYGGIDVWGQGTTVTVSS |
| | | | SEQ ID NO: 25880 | SEQ ID NO: 29886 |

FIGURE 50

| | | | | |
|---|---|---|---|---|
| **iPS:434729** | **21-225_80B12** | NA | GACATCGTGTTGACCCAGTCGCCAGACTCCCTGGCTGTGTCTCTGGGCGAGAGGGCCACCATCAACTGCAAGTCCAGACAGAGTGTTTTATACAGCTCCAACAATTACAACTACTTAACTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGACGTGGCAGTTTATTACTGTCAGCAATATTATAGTTCTCCTCCTACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGACTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGTCAGAGTCACCATGACCAGGAACACCTCCATAAGCACAGCCTACATGGAGCTGAGCAGCCTGAGATCTGAGGACACGGCCGTCTATTACTGTGCGTATAGCAGTGGCTGGTACATCTTTGACTACTGGGGCCAGGGAACCCTGGTCACCGTCTCCTCA |
| | | | SEQ ID NO: 25881 | SEQ ID NO: 29887 |
| | | AA | DIVLTQSPDSLAVSLGERATINCKSRQSVLYSSNNYNYLTWYQQKPGQPPKLLIYWASTRESGVPDRFSGSGSGTDFTLTISSLQAEDVAVYYCQQYYSSPPTFGGGTKVEIK | QVQLVQSGAEVKKPGASVKVSCKTSGYTFTNYDINWVRQATGQGLEWMGWMNPNSGNTGYAQKFQVRVTMTRNTSISTAYMELSSLRSEDTAVYYCAYSSGWYIFDYWGQGTLVTVSS |
| | | | SEQ ID NO: 25882 | SEQ ID NO: 29888 |
| **iPS:434731** | **21-225_80E9** | NA | GACATCGTGATGACCCAGTCTCCAGATTGCCTGGCTGTGTCTCTGGGCGAGAGGGCCACCATCAACTGCAAGTCCAGCCAGAGTGTTTTATACACCTCCAACAATAACAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTTGCAGTTTATTATTGTCAGCAATATTATAATACTCCGTGGACGTTCGTCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTCA |
| | | | SEQ ID NO: 25883 | SEQ ID NO: 29889 |

FIGURE 50

| | | | | |
|---|---|---|---|---|
| | | AA | DIVMTQSPDCLAVSLGERATINCKSSQSVLYTSNNNNYLAWYQQKPGQPPKLLIYWASTRESGVPDRFSGSGSGTDFTLTISSLQAEDVAVYYCQQYYNTPWTFVQGTKVEIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25884 | SEQ ID NO: 29890 |
| iPS:434735 | 21-225_80B10 | NA | GAAATTGTGTTGACGCAGTCTCCAGGCACCCTGTCTTTGTCTCCAGGGGAAAGAGCCACCCTCTCCTGCAGGGCCAGTCAGAGTGTTGACAGCAGCTACTTAGCCTGGTACCAGCAGAAACCTGGCCAGGCTCCCAGGCTCCTCATCTATGGTGCATCCAGCAGGGCCCCTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGAAAGCTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATATCAGTAGACACGTCCAAGAACCAGTTCTCCCTGAAGCTGAGCTCTGTGACCGCCGCGGACACGGCTGTGTATTACTGTGCGAGAGACTACGGTGGGCTTGACTACTGGGGCCAGGGAACCCTGGTCACCGTCTCCTCA |
| | | | SEQ ID NO: 25885 | SEQ ID NO: 29891 |
| | | AA | EIVLTQSPGTLSLSPGERATLSCRASQSVDSSYLAWYQQKPGQAPRLLIYGASSRAPGIPDRFSGSGSGTDFTLTISRLEPEDFAVYYCQQYESSPWTFGQGTKVEIK | QVQLQQWGAGLLKPSETLSLTCAVYGGSFSGCYWSWIRQPPGKGLEWIGEINHSGSTNYNPSLKSRVTISVDTSKNQFSLKLSSVTAADTAVYYCARDYGGLDYWGQGTLVTVSS |
| | | | SEQ ID NO: 25886 | SEQ ID NO: 29892 |

FIGURE 50

| | | | | |
|---|---|---|---|---|
| **iPS:434737** | **21-225_74G6** | NA | GACATCCAGATGACCCAGTCTCCATCCTCACT<br>GTCTGCATCAGTAGGAGACAGAGTCACCATCA<br>CTTGTCGGGCGAGTCAGGGCATTGGCAAGTAT<br>TTAGCCTGGTTTCAGCAGAAACCAGGGAAAGC<br>CCCTAAGTCCCTGATCTATACTACATCCAGTTT<br>GCAAAGTGGGGCCCCATCAAAGTTCAGCGGC<br>AGTGGATCTGGGACAGATTTCACTCTCACCAT<br>CAGCAGCCTGCAGTATGAAGATTTTGCAACTT<br>ATTACTGCCAACAGTACAGTAATTACCCGCTC<br>ACTTTCGGCGGAGGGACCAAGGTGGAGATCA<br>AT | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTAGCTATGGCAT<br>GCACTGGGTCCGCCAGGCTCCAGGCAAGGGGCT<br>GGAGTGGGTGGCAGTTATATGGTATGATGGAAGT<br>AATAAAAACTATGCAGACTCCGTGAAGGGCCGA<br>TTCACCATCTCCAGAGACAATTCCAAGAACACGC<br>TGTATCTGCAAATGAACAGCCTGAGAGCCGAGG<br>ACACGGCTGTGTATTACTGTGCGAGAGATGGCAG<br>CTATGGTTATGACGGCCTTGACTACTGGGGCCAG<br>GGAACCCTGGTCACCGTCTCCTCA |
| | | | SEQ ID NO: 25887 | SEQ ID NO: 29893 |
| | | AA | DIQMTQSPSSLSASVGDRVTITCRASQGIGKYLA<br>WFQQKPGKAPKSLIYTTSSLQSGAPSKFSGSGSG<br>TDFTLTISSLQYEDFATYYCQQYSNYPLTFGGGT<br>KVEIN | QVQLVESGGGVVQPGRSLRLSCAASGFTFSSYGMH<br>WVRQAPGKGLEWVAVIWYDGSNKNYADSVKGRF<br>TISRDNSKNTLYLQMNSLRAEDTAVYYCARDGSYG<br>YDGLDYWGQGTLVTVSS |
| | | | SEQ ID NO: 25888 | SEQ ID NO: 29894 |
| **iPS:434741** | **21-225_80C11** | NA | GACATCCAGATGACCCAGTCTCCATCCTCACT<br>GTCTGCTTCTGTAGGGGACAGAGTCACCATCA<br>CTTGTCGGGCGAGTCAGGGCATTGGCAGGTAT<br>TTAGCCTGGTTTCAGCAGAAACCAGGGAGAGC<br>CCCTAAGTCCCTGATCTATACTGCATCCAGTTT<br>GCAAAGTGGGGCCCCATCAAAGTTCAGCGGC<br>AGTGGATCTGGGACAGATTTCACTCTCACCAT<br>CAGCAGCCTGCAGCCTGAAGATTTTGCAACTT<br>ATTACTGCCAACAGTACAGTAATTACCCGCTC<br>ACTTTCGGCGGAGGGACCAAAGTGGAGATCA<br>AT | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGCAGCTATGGCAT<br>GCACTGGGTCCGCCAGGCTCCAGGCAAGGGGCT<br>GGAGTGGGTGGCAGTTATATGGTATGATGGAAGT<br>AATAAAAACTATGCAGACTCCGTGAAGGGCCGA<br>TTCACCATCTCCAGAGACAATTCCAAGAACACGC<br>TGTCTCTGCAAATGAACAGCCTGAGAGCCGAGG<br>ACACGGCTGTGTATTACTGTGCGAGAGATGGCAG<br>CTATGGGTATGACGGCCTTGACTACTGGGGCCAG<br>GGAACCCTGGTCACCGTCTCCTCA |
| | | | SEQ ID NO: 25889 | SEQ ID NO: 29895 |

FIGURE 50

| | | | | |
|---|---|---|---|---|
| | | AA | DIQMTQSPSSLSASVGDRVTITCRASQGIGRYLAWFQQKPGRAPKSLIYTASSLQSGAPSKFSGSGSGTDFTLTISSLQPEDFATYYCQQYSNYPLTFGGGTKVEIN | QVQLVESGGGVVQPGRSLRLSCAASGFTFSSYGMHWVRQAPGKGLEWVAVIWYDGSNKNYADSVKGRFTISRDNSKNTLSLQMNSLRAEDTAVYYCARDGSYGYDGLDYWGQGTLVTVSS |
| | | | SEQ ID NO: 25890 | SEQ ID NO: 29896 |
| **iPS:434743** | **21-225_74A4** | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTGTGGGACAGACTTCGCTCTCACCATCAGCAGACTGGAGCCTGAAGATTGTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25891 | SEQ ID NO: 29897 |
| | | AA | EIVLTQSPGTLYLSPGERATLSCRASQSVYSSYLAWYQQKPGQAPRLLIYGASSRSTGIPDRFSGSGCGTDFALTISRLEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25892 | SEQ ID NO: 29898 |

FIGURE 50

| | | | | |
|---|---|---|---|---|
| **iPS:434747** | **21-225_80C12** | NA | GAAATTGTGTTGACGCAGTCTCCAGGCACCCTGTATTTGTCTCCAGGGGAAAGAGCCACCCTCTCGTGCAGGGCCAGTCAGAGTGTTAGCAGCAGCTACTTAGCCTGGTACCAGCAGAAACCTGGCCAGGCTCCCAGGCTCCTCATCTATGGTGCATCCACCAGGGCCACTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTAACTCACCGCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAATGGTAACACAGGCTATGCACAGAAGTTCCAGGGCAGAGTCACCATGACCAGGAACACCTCCATAAGCACAGCCTACATGGAGCTGAACAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTTA |
| | | | SEQ ID NO: 25893 | SEQ ID NO: 29899 |
| | | AA | EIVLTQSPGTLYLSPGERATLSCRASQSVSSSYLAWYQQKPGQAPRLLIYGASTRATGIPDRFSGSGSGTDFTLTISRLEPEYFAVYYCQQYGNSPLTFGGGTKVEIK | QVQLVQSGAEVKKPGASVKVSCKASGYTFTNYDINWVRQATGQGLEWMGWMHPNNGNTGYAQKFQGRVTMTRNTSISTAYMELNSLRSEDTAVYYCAISSGWYWFDPWGQGTLVTVSL |
| | | | SEQ ID NO: 25894 | SEQ ID NO: 29900 |
| **iPS:434751** | **21-225_80H12** | NA | GAAATTGTGTTGACGCAGTCTCCAGGCACCCTGTCTTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGACTGGAGCCTGAAGATTTTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25895 | SEQ ID NO: 29901 |

FIGURE 50

| | | | | |
|---|---|---|---|---|
| | | AA | EIVLTQSPGTLSLSPGERATLSCRASQSVYSSYLAWYQQKPGQAPRLLIYGASSRSTGIPDRFSGSGSGTDFALTISRLEPEDFAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25896 | SEQ ID NO: 29902 |
| iPS:434759 | 21-225_81C5 | NA | GAAATTGTGTTGACGCAGTCTCCAGGCACCCTGTATTTGTCTTCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTGTGGGACAGACTTCGCTCTCACCATCAGCAGAGTGGAGCCTGAAGATTGTGCAGTGTATTACTGTCAGCATTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25897 | SEQ ID NO: 29903 |
| | | AA | EIVLTQSPGTLYLSSGERATLSCRASQSVYSSYLAWYQQKPGQAPRLLIYGASSRSTGIPDRFSGSGCGTDFALTISRVEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25898 | SEQ ID NO: 29904 |

FIGURE 50

| | | | | |
|---|---|---|---|---|
| iPS:434761 | 21-225_81E5 | NA | GACATCGTTATGACCCAGTCTCCAGACTCCCTGGCTGTGTCTCTGGGCGAGAGGGCCACCATCAATTGCAAGTCCAGCCAGAGTGTTTTATTCAGCTCCAACAATTATAATTACTTAGCTTGGTACCAGCAGAGACCAGGACAGCCTCCTAACCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGCTGATGTGGCAGTTTATTACTGTCAGCAATATTATAGTTCTCCTCTGACGTTCGGCCAAGGGACCACGGTGCAAATCAAA | CAGGTGCAGTTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCACAAGCACAGCCCACATGGAGCTGAGCAGCCTGAGATCTGAGGACACGGCCGTGTATTACTGTGCGGTCTCCAGTGGCTGGAACTGGTTCGACCCCTGGGGCCAGGGAACCCTGGTCACCGTCTCCTCA |
| | | | SEQ ID NO: 25899 | SEQ ID NO: 29905 |
| | | AA | DIVMTQSPDSLAVSLGERATINCKSSQSVLFSSNNYNYLAWYQQRPGQPPNLLIYWASTRESGVPDRFSGSGSGTDFTLTISSLQAADVAVYYCQQYYSSPLTFGQGTTVQIK | QVQLVQSGAEVKKPGASVKVSCKASGYTFTNYDINWVRQATGQGLEWMGWMNPNSGNTGYAQKFQGRVTMTRNTSTSTAHMELSSLRSEDTAVYYCAVSSGWNWFDPWGQGTLVTVSS |
| | | | SEQ ID NO: 25900 | SEQ ID NO: 29906 |
| iPS:434771 | 21-225_81F9 | NA | GACATCGTGATGACCCAGTCTCCAGATTGCCTGGCTGTGTCTCTGGGCGAGAGGGCCACCATCAACTGCAAGTCCAGCCAGAGTGTTTTATACACCTCCAACAATAACAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTTGCAGTTTATTATCGTCAGCACTACAATGATACTCCAGGGAAGTTTGTCCAAGGCATCATGGTGGAAATCACA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTCA |
| | | | SEQ ID NO: 25901 | SEQ ID NO: 29907 |

FIGURE 50

| | | | | |
|---|---|---|---|---|
| | | AA | DIVMTQSPDCLAVSLGERATINCKSSQSVLYTSNNNNYLAWYQQKPGQPPKLLIYWASTRESGVPDRFSGSGSGTDFTLTISSLQAEDVAVYYRQHYNDTPGKFVQGIMVEIT | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25902 | SEQ ID NO: 29908 |
| iPS:434773 | 21-225_75D9 | NA | GAAATTGTGTTGACGCAGTCTCCAGGCACCCTGTCTTTGTCTCCAGGGGAAAGAGCCACCCTCTCCTGCAGGGCCAGTCAGAGTGTTAGCAGCAGCTACTTAGCCTGGTACCAGCAGAAACGTGGCCAGGCTCCCAGGCTCCTCATCTATGGTGCATCCAGCAGGGCCACTGGCATTCCAGACAGGTTCAGTGGCAGTGGGTCTGGGACAGACTTCACTCTCACCATCAGCAGACTGGAGCCTGAAGATTTTGCAGTGTATTACTGTCAGCAGTATGAAAGCTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGGTCCCTACTGGAGCTGGATCCGCCAGCCCCCAGGGAAGGGGCTGGAGTGGATTGGGGAAATCAATTATAGGGGAAGCACCAACTACAACCCGTCCCTCAAGAGTCGAGTCACCATTTCAGTAGACACGTCCAAGAACCAGTTCTCCCTGAACCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25903 | SEQ ID NO: 29909 |
| | | AA | EIVLTQSPGTLSLSPGERATLSCRASQSVSSSYLAWYQQKRGQAPRLLIYGASSRATGIPDRFSGSGSGTDFTLTISRLEPEDFAVYYCQQYESSPWTFGQGTKVEIK | QVQLQQWGAGLLKPSETLSLTCAVYGGSFSGPYWSWIRQPPGKGLEWIGEINYRGSTNYNPSLKSRVTISVDTSKNQFSLNLSSVTAADTAVYYCARDYGGMDVWGQGTTVTVSS |
| | | | SEQ ID NO: 25904 | SEQ ID NO: 29910 |

FIGURE 50

| | | | | |
|---|---|---|---|---|
| **iPS:434777** | **21-225_81C11** | NA | GAAATTGTGTTGACGCAGTCTCCAGGCACCCGGTATTTGTCTCCAGTGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCCTCCCAGACAGGTTCAGTGGCAGTGGGTGTGGGACAGACTTCGCTCTCACCATCAGCAGAGTGGAGCCTGAAGATTGTGCAGTGTATTACTGTCAGCATTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25905 | SEQ ID NO: 29911 |
| | | AA | EIVLTQSPGTRYLSPVERATLSCRASQSVYSSYLAWYQQKPGQAPRLLIYGASSRSTGLPDRFSGSGCGTDFALTISRVEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25906 | SEQ ID NO: 29912 |
| **iPS:434793** | **21-225_82A5** | NA | GAAATTGTGTTGACGCAGTCTCCAGGCACCCTGTCTTGGTCTCCAGGGGAAAGAGCCACCCTCTCGTGCAGGGCCAGTCAGAGTGTTAGCAGCAGCTACTTAGCCTGGTACCAGCAGAAACCTGGCCAGGCTCCCAGGCTCCTCATCTATGGTGCATCCACCAGGGCCACTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTAACTCACCGCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAATGGTAACACAGGCTATGCACAGAAGTTCCAGGGCAGAGTCACCATGACCAGGAACACCTCCATAAGCACAGCCTACATGGAGCTGAACAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTTA |
| | | | SEQ ID NO: 25907 | SEQ ID NO: 29913 |

FIGURE 50

| | | | | |
|---|---|---|---|---|
| | | AA | EIVLTQSPGTLSWSPGERATLSCRASQSVSSSYLAWYQQKPGQAPRLLIYGASTRATGIPDRFSGSGSGTDFTLTISRLEPEYFAVYYCQQYGNSPLTFGGGTKVEIK | QVQLVQSGAEVKKPGASVKVSCKASGYTFTNYDINWVRQATGQGLEWMGWMHPNNGNTGYAQKFQGRVTMTRNTSISTAYMELNSLRSEDTAVYYCAISSGWYWFDPWGQGTLVTVSL |
| | | | SEQ ID NO: 25908 | SEQ ID NO: 29914 |
| iPS:434797 | 21-225_82G5 | NA | GAATTTATGTTGACGCAGTCTCCAGGCACCCTGTATTGGTCTCCAGGGGAAAGAGCCACCCTCTCGAGCAGGGCCAGTGAGAGTGTTAGCAGCAGCTACTTAGTCTGGTATCAGCAGAAACCTGGCCAGGCTCCCAGGCTCCTCATCTATGGTGCATCCACCAGGGCCACTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTTGCTCACCGCTCACTTTCGGCGGAGGGACCAAGGTGGAGATCACA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGTCCCTGGACAAGGGCTTGAGTGGATGGGATGGATGCACCCTAACAGTGGTAACACAGGCTATGCACAGAAGTTCCAGGGCAGAGTCACCATGACCAGGAACACCTCCATCAGCACAGCCTACATGGAGCTGAGCAGCCTGAGATCTGAGGACACGGCCGTGTATTACTGTGCGATTAGTAGTGGCTGGTACTGGTTCGACCCCTGGGGCCAGGGAACCCTGGTCACCGTCTCCTCA |
| | | | SEQ ID NO: 25909 | SEQ ID NO: 29915 |
| | | AA | EFMLTQSPGTLYWSPGERATLSSRASESVSSSYLVWYQQKPGQAPRLLIYGASTRATGIPDRFSGSGSGTDFTLTISRLEPEYFAVYYCQQYGCSPLTFGGGTKVEIT | QVQLVQSGAEVKKPGASVKVSCKASGYTFTNYDINWVRQVPGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25910 | SEQ ID NO: 29916 |

FIGURE 50

| | | | | |
|---|---|---|---|---|
| iPS:434805 | 21-225_82D9 | NA | GAATTTATGTTGACGCAGTCTCCAGGCACCCTGTCTTGGTCTCCAGGGGAAAGAGCCACCCTCTCGTGCAGGGCCAGTGAGAGTGTTAGCAGCAGCTACTTAGTCTGGTATCAGCAGAAACCTGGCCAGGCTCCCAGGCTCCTCATCTATGGTGCATCCACCAGGGCCACTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTTGCTCACCGCTCACTTTCGGCGGAGGGACCAAGGTGGAGATCACA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGTCCCTGGACAAGGGCTTGAGTGGATGGGATGGATGCACCCTAACAGTGGTAACACAGGCTATGCACAGAAGTTCCAGGGCAGAGTCACCATGACCAGGAACACCTCCATCAGCACAGCCTACATGGAGCTGAGCAGCCTGAGATCTGAGGACACGGCCGTGTATTACTGTGCGATTAGTAGTGGCTGGTACTGGTTCGACCCCTGGGGCCAGGGAACCCTGGTCACCGTCTCCTCA |
| | | | SEQ ID NO: 25911 | SEQ ID NO: 29917 |
| | | AA | EFMLTQSPGTLSWSPGERATLSCRASESVSSSYLVWYQQKPGQAPRLLIYGASTRATGIPDRFSGSGSGTDFTLTISRLEPEYFAVYYCQQYGCSPLTFGGGTKVEIT | QVQLVQSGAEVKKPGASVKVSCKASGYTFTNYDINWVRQVPGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25912 | SEQ ID NO: 29918 |
| iPS:434809 | 21-225_74F5 | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGACTGGAGCCTGAAGATTTTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25913 | SEQ ID NO: 29919 |

FIGURE 50

| | | | | |
|---|---|---|---|---|
| | | AA | EIVLTQSPGTLYLSPGERATLSCRASQSVYSSYLAWYQQKPGQAPRLLIYGASSRSTGIPDRFSGSGSGTDFALTISRLEPEDFAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25914 | SEQ ID NO: 29920 |
| **iPS:434813** | **21-225_82C12** | NA | GAAATTGTGTTGACGCAGTCTCCAGGCACCCTGTCTTGGTCTCCAGGGGAAAGAGCCACCCTCTCGTGCAGGGCCAGTCAGAGTGTTAGCAGCAGCTACTTAGCCTGGTACCAGCAGAAACCTGGCCAGGCTCCCAGGCTCCTCATCTATGGTGCATCCACCAGGGCCTCTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTAACTCACCGCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAATGGTAACACAGGCTATGCACAGAAGTTCCAGGGCAGAGTCACCATGACCAGGAACACCTCCATAAGCACAGCCTACATGGAGCTGAACAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTTA |
| | | | SEQ ID NO: 25915 | SEQ ID NO: 29921 |
| | | AA | EIVLTQSPGTLSWSPGERATLSCRASQSVSSSYLAWYQQKPGQAPRLLIYGASTRASGIPDRFSGSGSGTDFTLTISRLEPEYFAVYYCQQYGNSPLTFGGGTKVEIK | QVQLVQSGAEVKKPGASVKVSCKASGYTFTNYDINWVRQATGQGLEWMGWMHPNNGNTGYAQKFQGRVTMTRNTSISTAYMELNSLRSEDTAVYYCAISSGWYWFDPWGQGTLVTVSL |
| | | | SEQ ID NO: 25916 | SEQ ID NO: 29922 |

FIGURE 50

| | | | | |
|---|---|---|---|---|
| **iPS:434815** | **21-225_74A11** | NA | GACATCCAGATGACCCAGTCTCCATCCTCCCTGTCTGCATCTGTAGGAGACAGAGTCACCATCACTTGCCGGGCAAGTCAGGACATTAGAAATGATTTAGGCTGGTATCAGCAGAAACCAGGGAAAGCCCCTAAGCGCCTGATCTGTGCTGCATCCAGTTTGCAAAGTGGGGTCCCATCAAGGTTCAGCGGCAGTGGATCTGGGACCGAGTTCACTCTCACAATCAGCAGCCTGCAGCCTGAGGATTTTGCAACTTATTTCTGTCTACAGCATAATGATTACCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGTAAGGCTTCTGGATACACCTTCACCAGTTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCTGGAACACCTCCAAAAGCACAGCCTACATGGAGCTGAGCAGCCTGAGATCTGAGGACACGGCCGTGTATTACTGTGCGAGAGGCTTTTACGATACTTTGACTGGTTCCGGCTACTACTACGTTATGGACGTCTGGGGCCAAGGGACCACGGTCACCGTCTCCTCA |
| | | | SEQ ID NO: 25917 | SEQ ID NO: 29923 |
| | | AA | DIQMTQSPSSLSASVGDRVTITCRASQDIRNDLGWYQQKPGKAPKRLICAASSLQSGVPSRFSGSGSGTEFTLTISSLQPEDFATYFCLQHNDYPFTFGPGTKVDIK | QVQLVQSGAEVKKPGASVKVSCKASGYTFTSYDINWVRQATGQGLEWMGWMNPNSGNTGYAQKFQGRVTMTWNTSKSTAYMELSSLRSEDTAVYYCARGFYDTLTGSGYYYVMDVWGQGTTVTVSS |
| | | | SEQ ID NO: 25918 | SEQ ID NO: 29924 |
| **iPS:434821** | **21-225_83G1** | NA | GAAATTGTGTTGACGCAGTCTCCAGGCACCCGGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACGGTCCTCCCAGACAGGTTCAGTGGCAGTGGGTCTGGGACAGACTTCGCTCTCACCATCAGCAGAGTGGAGCCTGAAGATTGTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25919 | SEQ ID NO: 29925 |

FIGURE 50

| | | | | |
|---|---|---|---|---|
| | | AA | EIVLTQSPGTRYLSPGERATLSCRASQSVYSSYLAWYQQKPGQAPRLLIYGASSRSTVLPDRFSGSGSGTDFALTISRVEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25920 | SEQ ID NO: 29926 |
| **iPS:434825** | **21-225_83C2** | NA | GAATTTATGTTGACGCAGTCTCCAGGCACCCTGTGTTTGTCTCCAGGGGAAAGAGCCACCCTCTCGTGCAGGGCCAGTCAGAGTGTTAGCAGCAGCTACTTAGTCTGGTATCAGCAGAAACCTGGCCAGGCTCCCAGGCTCCTCATCTATGGTGCATCCACCAGGGCCACTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTTGCTCACCGCTCACTTTCGGCGGAGGGACCAAGGTGGAGATCACA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGTCCCTGGACAAGGGCTTGAGTGGATGGGATGGATGCACCCTAACAGTGGTAACACAGGCTATGCACAGAAGTTCCAGGGCAGAGTCACCATGACCAGGAACACCTCCATCAGCACAGCCTACATGGAGCTGAGCAGCCTGAGATCTGAGGACACGGCCGTGTATTACTGTGCGATTAGTAGTGGCTGGTACTGGTTCGACCCCTGGGGCCAGGGAACCCTGGTCACCGTCTCCTCA |
| | | | SEQ ID NO: 25921 | SEQ ID NO: 29927 |
| | | AA | EFMLTQSPGTLCLSPGERATLSCRASQSVSSSYLVWYQQKPGQAPRLLIYGASTRATGIPDRFSGSGSGTDFTLTISRLEPEYFAVYYCQQYGCSPLTFGGGTKVEIT | QVQLVQSGAEVKKPGASVKVSCKASGYTFTNYDINWVRQVPGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25922 | SEQ ID NO: 29928 |

FIGURE 50

| | | | | |
|---|---|---|---|---|
| iPS:434827 | 21-225_83F3 | NA | GACATCGTGATGACCCAGTCTCCAGATTGCCTGGCTGTGTCTCTGGGCGAGAGGGCCACCATCAACTGCAAGTCCAGCCAGAGTGTTTTATACACCTCCAACAATAACAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTTGCAGTTTATTATTGTCAGCACTATAATGATACTCCATGGAAGTTTGTCCAAGGGATCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTCA |
| | | | SEQ ID NO: 25923 | SEQ ID NO: 29929 |
| | | AA | DIVMTQSPDCLAVSLGERATINCKSSQSVLYTSNNNNYLAWYQQKPGQPPKLLIYWASTRESGVPDRFSGSGSGTDFTLTISSLQAEDVAVYYCQHYNDTPWKFVQGIKVEIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25924 | SEQ ID NO: 29930 |
| iPS:434829 | 21-225_83G3 | NA | GACATCGTGATGACCCAGTCTCCAGATTGCCTGGCTGTGTCTCTGGGCGAGAGGGCCACCATCAACTGCAAGTCCAGCCAGAGTGTTTTATACACCTCCAACAATAACAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTTGCAGTTTATTATTGTCAGCACTATTATAATACTCCGTGGACGTTTGTCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTCA |
| | | | SEQ ID NO: 25925 | SEQ ID NO: 29931 |

FIGURE 50

| | | | | |
|---|---|---|---|---|
| | | AA | DIVMTQSPDCLAVSLGERATINCKSSQSVLYTSNNNNYLAWYQQKPGQPPKLLIYWASTRESGVPDRFSGSGSGTDFTLTISSLQAEDVAVYYCQHYYNTPWTFVQGTKVEIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25926 | SEQ ID NO: 29932 |
| iPS:434833 | 21-225_83C5 | NA | GAATTTATGTTGACGCAGTCTCCAGGCACCCTGTGTTGGTCTCCAGGGGAAAGAGCCACCCTCTCGTGCAGGGCCAGTGAGAGTGTTAGCAGCAGCTACTTAGTCTGGTATCAGCAGAAACCTGGCCAGGCTCCCAGGCTCCTCATCTATGGTGCATCCACCAGGGCCACTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTTGCTCACCGCTCACTTTCGGCGGAGGGACCAAGGTGGAGATCACA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGTCCCTGGACAAGGGCTTGAGTGGATGGGATGGATGCACCCTAACAGTGGTAACACAGGCTATGCACAGAAGTTCCAGGGCAGAGTCACCATGACCAGGAACACCTCCATCAGCACAGCCTACATGGAGCTGAGCAGCCTGAGATCTGAGGACACGGCCGTGTATTACTGTGCGATTAGTAGTGGCTGGTACTGGTTCGACCCCTGGGGCCAGGGAACCCTGGTCACCGTCTCCTCA |
| | | | SEQ ID NO: 25927 | SEQ ID NO: 29933 |
| | | AA | EFMLTQSPGTLCWSPGERATLSCRASESVSSSYLVWYQQKPGQAPRLLIYGASTRATGIPDRFSGSGSGTDFTLTISRLEPEYFAVYYCQQYGCSPLTFGGGTKVEIT | QVQLVQSGAEVKKPGASVKVSCKASGYTFTNYDINWVRQVPGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25928 | SEQ ID NO: 29934 |

FIGURE 50

| | | | | |
|---|---|---|---|---|
| **iPS:434835** | **21-225_83B6** | NA | GAAATTGTGTTGACGCAGTCTCCAGGCACTTTGTCTTTGTCTCCAGGGGAAAGAGCCACCCTCTCCTGCAGGGCCAGTCAGAGTGTTGACAGCGGCTACTTAGCCTGGTACCAGCAGAAACCTGGCCAGGCTCCCAGGCTCCTCATCTATGGTGCATCCAGCAGGACCCCTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGAAAGCTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGGTTGTTACTGGAGCTGGATCCGCCAGCCCCCAGGGAAGGGGCTGGAGTGGATTGGGGAAATCAATCATAGTGGAAGGACCAACTACAACCCCTCCCTCAAGAGTCGAGTCACCATATCAGTAGACACGTCCGAGAACCAGTTCTCCCTGAAGCTGAGCTCTGTGACCGCCGCGGACACGGCTGTGTATTACTGTGCGAGAGACTACGGTGGGCTTGACTACTGGGGCCAGGGAACCCTGGTCACCGTCTCCTCA |
| | | | SEQ ID NO: 25929 | SEQ ID NO: 29935 |
| | | AA | EIVLTQSPGTLSLSPGERATLSCRASQSVDSGYLAWYQQKPGQAPRLLIYGASSRTPGIPDRFSGSGSGTDFTLTISRLEPEDFAVYYCQQYESSPWTFGQGTKVEIK | QVQLQQWGAGLLKPSETLSLTCAVYGGSFSGCYWSWIRQPPGKGLEWIGEINHSGRTNYNPSLKSRVTISVDTSENQFSLKLSSVTAADTAVYYCARDYGGLDYWGQGTLVTVSS |
| | | | SEQ ID NO: 25930 | SEQ ID NO: 29936 |
| **iPS:434839** | **21-225_83B7** | NA | GAAATTGTGTTGACGCAGTCTCCAGGCACCCGGTATTTGTCTTCAGTGGAAAGAGCCACCCTCTCATGCAGGGCCAGTCAGAGTGTTTACAGCAGCTACTTAGCGTGGTACCAGCAGAAACCTGGCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGACTGGAGCCTGAAGATTGTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25931 | SEQ ID NO: 29937 |

FIGURE 50

| | | | | |
|---|---|---|---|---|
| | | AA | EIVLTQSPGTRYLSSVERATLSCRASQSVYSSYL AWYQQKPGQAPRLLIYGASSRSTGIPDRFSGSGS GTDFALTISRLEPEDCAVYYCQHSDNSPWTFGQ GTKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 25932 | SEQ ID NO: 29938 |
| iPS:434841 | 21-225_83G7 | NA | GACATCGTGATGACCCAGTCTCCAGACTCCCT GGCTGTGTCTCTGGGCGAGAGGGCCACCATCA TCTGCAAGTCCAGCCAGACTGTTTTACACAGC TCCAACAATTATAACTACTTAGCTTGGTACCA GCAGAAGCCAGGACAGCCCCCTAAGGTGCTC ATTTACTGGACATCTACCCGGGAATCCGGGGT CCCTGACCGATTCAGTGGCAGCGGGTCTGGGA CAGATTTCACTCTCACCATCAGCAGCCTGCAG GCTGAAGATGTGGCAGTTTATTACTGTCAGCA ATATTTTAGTAGTCCTCTGACGTTCGGCCAAG GGACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCCCCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTATTGTGCGGTTTCCAG TGGCTGGCACTGGTTCGACCCCTGGGGCCAGGGA ACCCTGGTCACCGTCGCCTCA |
| | | | SEQ ID NO: 25933 | SEQ ID NO: 29939 |
| | | AA | DIVMTQSPDSLAVSLGERATIICKSSQTVLHSSNN YNYLAWYQQKPGQPPKVLIYWTSTRESGVPDRF SGSGSGTDFTLTISSLQAEDVAVYYCQQYFSSPL TFGQGTKVEIK | QVQLVQSGAEVKKPGASVKVSCKASGYTFPNYDIN WVRQATGQGLEWMGWMNPNSGNTGYAQKFQGR VTMTRNTSISTAYMELSSLRSEDTAVYYCAVSSGW HWFDPWGQGTLVTVAS |
| | | | SEQ ID NO: 25934 | SEQ ID NO: 29940 |

FIGURE 50

| | | | | |
|---|---|---|---|---|
| **iPS:434849** | **21-225_83C10** | NA | GAAATTGTGTTGACGCAGTCTCCAGGCACCCTGTCTTTGTCTCCAGGGGAAAGAGCCACCCTCTCCTGCAGGGCCAGTCCGAGTGTTCACAGCAACTACTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCATTCTCACCATCAGCAGACTGGAGCCTGAAGATTTTGCAGTGTATTACTGTCAGCAGTATGAAAGTTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCACTGTCTATGGTGGGTCCTTCAGTGGTTACTACTGGAGCTGGATCCGCCAGCCCCCAGGGAAGGGGCTGGAATGGATTGGGGAAATCAATCATAGTGGAAGCACCAACTTCAACCCGTCCCTCAAGAGTCGAGTCACCATATCAGTAGACACGTCCAAGAACCAGTTCTCCCTGAAGCTGAGCTCTGTGACCGCCGCGGACACGGCTGTGTTTTACTGTGCGAGAGACTACGGTGGGCTTGACTACTGGGGCCAGGGAACCCTGGTCACCGTCTCCTCA |
| | | | SEQ ID NO: 25935 | SEQ ID NO: 29941 |
| | | AA | EIVLTQSPGTLSLSPGERATLSCRASPSVHSNYLAWYQQKPGQAPRLLIYGASSRATGIPDRFSGSGSGTDFILTISRLEPEDFAVYYCQQYESSPWTFGQGTKVEIK | QVQLQQWGAGLLKPSETLSLTCTVYGGSFSGYYWSWIRQPPGKGLEWIGEINHSGSTNFNPSLKSRVTISVDTSKNQFSLKLSSVTAADTAVFYCARDYGGLDYWGQGTLVTVSS |
| | | | SEQ ID NO: 25936 | SEQ ID NO: 29942 |
| **iPS:434851** | **21-225_75A6** | NA | GACATCGTGATGACCCAGTCGCCAGACTCCCTGGCTGTGTCTCTGGGCGAGAGGGCCACCATCAACTGCAAGTCCAGACAGAGTGTTTTACACAGCTCCAACAATTACAACTACTTAGCTTGGTACCAGCAGAAACCAGGACAGCCTCCTGAACTACTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAGGATGTGGCAGTTTATTACTGTCAGCAATATTATAGTACTCCTCCTACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGTATAGCAGTGGCTGGTACATCTTTGACTACTGGGGCCAGGGAACCCTGGTCACCGTCTCCTCA |
| | | | SEQ ID NO: 25937 | SEQ ID NO: 29943 |

FIGURE 50

| | | | | |
|---|---|---|---|---|
| | | AA | DIVMTQSPDSLAVSLGERATINCKSRQSVLHSSNNYNYLAWYQQKPGQPPELLIYWASTRESGVPDRFSGSGSGTDFTLTISSLQAEDVAVYYCQQYYSTPPTFGGGTKVEIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYYCAYSSGWYIFDYWGQGTLVTVSS |
| | | | SEQ ID NO: 25938 | SEQ ID NO: 29944 |
| iPS:434863 | 21-225_84G7 | NA | GACATCGTGATGACCCAGTCTCCAGACTCCCCGGCTGTGTCTCTGGGCGAGAGGGCCACCATCATCTGCAAGTCCAGCCAGACTGTTTTACACAGCTCCAACAATTATAACTACTTAGCTTGGTACCAGCAGAAGCCAGGACAGCCCCCTAAGGTGCTCATTTACTGGACATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTTTAGTAGTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCCCCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGACAGAGTCACCATGACCAGGAACACCTCCATAAGCACAGCCTACATGGAGCTGAGCAGCCTGAGATCTGAGGACACGGCCGTGTATTATTGTGCGGTTTCCAGTGGCTGGCACTGGTTCGACCCCTGGGGCCAGGGAACCCTGGTCACCGTCGCCTCA |
| | | | SEQ ID NO: 25939 | SEQ ID NO: 29945 |
| | | AA | DIVMTQSPDSPAVSLGERATIICKSSQTVLHSSNNYNYLAWYQQKPGQPPKVLIYWTSTRESGVPDRFSGSGSGTDFTLTISSLQAEDVAVYYCQQYFSSPPTFGQGTKVEIK | QVQLVQSGAEVKKPGASVKVSCKASGYTFPNYDINWVRQATGQGLEWMGWMNPNSGNTGYAQKFQDRVTMTRNTSISTAYMELSSLRSEDTAVYYCAVSSGWHWFDPWGQGTLVTVAS |
| | | | SEQ ID NO: 25940 | SEQ ID NO: 29946 |

FIGURE 50

| | | | | |
|---|---|---|---|---|
| **iPS:434867** | **21-225_79A12** | NA | GACATCCAGATGACCCAGTCTCCATCCTCACTGTCTGCATCTGTAGGAGACAGAGTCACCATCACTTGTCGGGCGAGTCAGGTCATTAGCAAGTATTTAGCCTGGTTTCAGCAGAAACCAGGGAAAGCCCCTAAGTCCCTGATCTATGCTGCATCCAGTTTGCAAAGTGGGGCCCCATCAAAGTTCAGCGGCAGTGGATCTGGGACAGATTTCACTCTCACCATCAGCAGCCTGCAGCCTGAAGATTTTGCAACTTATTACTGCCAACAGTACAGTAATTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATGGCAGCTATGGTTACGACGGCCTTGACTACTGGGGCCAGGGAACCCTGGTCGCCGTCTCCTCA |
| | | | SEQ ID NO: 25941 | SEQ ID NO: 29947 |
| | | AA | DIQMTQSPSSLSASVGDRVTITCRASQVISKYLAWFQQKPGKAPKSLIYAASSLQSGAPSKFSGSGSGTDFTLTISSLQPEDFATYYCQQYSNYPLTFGGGTKVEIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAVYYCARDGSYGYDGLDYWGQGTLVAVSS |
| | | | SEQ ID NO: 25942 | SEQ ID NO: 29948 |
| **iPS:434869** | **21-225_84E12** | NA | GAAATTGTGTTGACGCAGTCTCCAGGCACCCTGTCTTTGTCTCCAGGGGAAAGAGCCACCCTCTCCTGCAGGGCCAGTCAGAGTATTAACAGCAACTACTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGACGATTTTGCAGTGTTTTACTGTCAGCAGTATGAGAGCTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGGTTCCTACTGGAGCTGGATCCGCCAGCCCCCAGGGAAGGGGCTAGAGTGGATTGGGGAAATCAATCAAAGTGGACGCACCAACTACAACCCGTCCCTCAAGAGTCGAGTCACCATATCAGTAGACACGTCCAAGAACCAGTTCTCCCTGAAGCTGAGCTCTGTGACCGCCGCGGACACGGCTGTGTATTACTGTGCGAGAGACTACGGGGGTATAGACGTCTGGGGCCAAGGGACCACGGTCACCGTCTCCTCA |
| | | | SEQ ID NO: 25943 | SEQ ID NO: 29949 |

FIGURE 50

| | | | | |
|---|---|---|---|---|
| | | AA | EIVLTQSPGTLSLSPGERATLSCRASQSINSNYLAWYQQKPGQAPRLLIYGASSRATGIPDRFSGSGSGTDFTLTISRLEPDDFAVFYCQQYESSPWTFGQGTKVEIK | QVQLQQWGAGLLKPSETLSLTCAVYGGSFSGSYWSWIRQPPGKGLEWIGEINQSGRTNYNPSLKSRVTISVDTSKNQFSLKLSSVTAADTAVYYCARDYGGIDVWGQGTTVTVSS |
| | | | SEQ ID NO: 25944 | SEQ ID NO: 29950 |
| **iPS:434871** | **21-225_85H1** | NA | GAGATAGTGATGACGCAGTCTCCAGCCACCCTGTCTGTGTCTCCAGGGGAAAGAGCCACCCTCTCCTGCAGGGCCAGTCAGGATGTTATCACCTACTTAGCCTGGTACCAGCAGAAACCTGGCCAGGCTCCCAGGCTCCTCATCTATGGTGCATCCACCAGGGCCACTGGTGTCCCAGCCAGGTTCAGTGGCAGTGGGTCTGGGACAGAGTTCACTCTCACCATCAGCAGCCTGCAGTCTGAAGATTTTGCACTTTATTACTGTCAGGAGTATAATGACTGGCCGTGCAGTTTTGGCCAGGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATACACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCCCTTTATAGTGGGAGCTACTTACTTTGACTACTGGGGCCAGGGAACCCTGGTCACCGTCTCCTCA |
| | | | SEQ ID NO: 25945 | SEQ ID NO: 29951 |
| | | AA | EIVMTQSPATLSVSPGERATLSCRASQDVITYLAWYQQKPGQAPRLLIYGASTRATGVPARFSGSGSGTEFTLTISSLQSEDFALYYCQEYNDWPCSFGQGTKVEIK | QVQLVESGGGVVQPGRSLRLSCAASGFTFSSYGIHWVRQAPGKGLEWVAVIWYDGSNKYYADSVKGRFTISRDNSKNTLYLQMNSLRAEDTAVYYCARDPFIVGATYFDYWGQGTLVTVSS |
| | | | SEQ ID NO: 25946 | SEQ ID NO: 29952 |

FIGURE 50

| | | | | |
|---|---|---|---|---|
| **iPS:434877** | **21-225_85H2** | NA | GACAGCATGATGACCCAGTCTCCAGACTCCCTGGCTGTGTCTCTGGGCGAGAGGGCCACCATCAACTGCAAGTCCAGCCAGAGTGTTTTACACAGCTCTAATAAAAAGAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGTCTGCAGGCTGAAGATGTGGCAGTTTATTACTGTCAGCAATATTATAGGACTCCGTGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAATAGTGGTAACACAGGCTATGCACAGAAGTTCCAGGGCAGAGTCACCCTGACCAGGAACACCTCCATAAGCACAGCCTACATGGAGCTGAGCAGCCTGAGATCTGAGGACACGGCCGTGTATTACTGTGCGGTTTCCAGTGGCTGGTACTGGTTCGACCCCTGGGGCCAGGGAACCCTGGTCACCGTCTCCTCA |
| | | | SEQ ID NO: 25947 | SEQ ID NO: 29953 |
| | | AA | DSMMTQSPDSLAVSLGERATINCKSSQSVLHSSNKKNYLAWYQQKPGQPPKLLIYWASTRESGVPDRFSGSGSGTDFTLTISSLQAEDVAVYYCQQYYRTPWTFGQGTKVEIK | QVQLVQSGAEVKKPGASVKVSCKASGYTFTNYDINWVRQATGQGLEWMGWMHPNSGNTGYAQKFQGRVTLTRNTSISTAYMELSSLRSEDTAVYYCAVSSGWYWFDPWGQGTLVTVSS |
| | | | SEQ ID NO: 25948 | SEQ ID NO: 29954 |
| **iPS:434879** | **21-225_85A3** | NA | GAAATTGTGTTGACGCAGTCTCCAGGCACCCTGTTTTTGTCTCCAGGGGAAAGAGCCACCCTCTCCTGCAGGGCCAGTCAGAGTGTTTACAGCAGCTACTTAGCCTGGTACCAGCAGAAACCTGCCCAGGCTCCCAGGCTCCTCATCTATGGTGCATCCAGCCGGGCCAGTGGCATCCCAGACAGGTTCAGTGGCAGTGGGTCTGGGACAGACTTCGCTCTCACCATCAGCAGACTGGAGCCTGAAGATTTTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25949 | SEQ ID NO: 29955 |

FIGURE 50

| | | | | |
|---|---|---|---|---|
| | | AA | EIVLTQSPGTLFLSPGERATLSCRASQSVYSSYLAWYQQKPAQAPRLLIYGASSRASGIPDRFSGSGSGTDFALTISRLEPEDFAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25950 | SEQ ID NO: 29956 |
| iPS:434881 | 21-225_85B4 | NA | GAAATTGTGTTGACGCAGTCTCCAGGCACCCTGTTTTTGTCTCCAGGGGAAAGAGCCACCCTCTCCTGCAGGGCCAGTCAGAGTGTTTACAGCAGCTACTTAGCCTGGTACCAGCAGAAACCTGGCCAGGCTCCCAGGCTCCTCATCTATGGTGCATCCAGCCGGGCCACTGGCATCCCAGACAGGTTCAGTGGCAGTGGGTCTGGGACAGACTTCGCTCTCACCATCAGCAGACTGGAGCCTGAAGATTTTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25951 | SEQ ID NO: 29957 |
| | | AA | EIVLTQSPGTLFLSPGERATLSCRASQSVYSSYLAWYQQKPGQAPRLLIYGASSRATGIPDRFSGSGSGTDFALTISRLEPEDFAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25952 | SEQ ID NO: 29958 |

FIGURE 50

| | | | | |
|---|---|---|---|---|
| **iPS:434883** | **21-225_85B5** | NA | GAATTTATGTTGACGCAGTCTCCAGGCACCCTGTCTTTGTCTCCAGGGGAAAGAGCCACCCTCTCGTGCAGGTCCAGTCAGAGTGTTAGCAGCAGCTACTTAGTCTGGTATCAGCAGAAACCTGGCCAGGCTCCCAGGCTCCTCATCTATGGTGCATCCACCAGGGCCACTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTTGCTCACCGCTCACTTTCGGCGGAGGGACCAAGGTGGAGATCACA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGTCCCTGGACAAGGGCTTGAGTGGATGGGATGGATGCACCCTAACAGTGGTAACACAGGCTATGCACAGAAGTTCCAGGGCAGAGTCACCATGACCAGGAACACCTCCATCAGCACAGCCTACATGGAGCTGAGCAGCCTGAGATCTGAGGACACGGCCGTGTATTACTGTGCGATTAGTAGTGGCTGGTACTGGTTCGACCCCTGGGGCCAGGGAACCCTGGTCACCGTCTCCTCA |
| | | | SEQ ID NO: 25953 | SEQ ID NO: 29959 |
| | | AA | EFMLTQSPGTLSLSPGERATLSCRSSQSVSSSYLVWYQQKPGQAPRLLIYGASTRATGIPDRFSGSGSGTDFTLTISRLEPEYFAVYYCQQYGCSPLTFGGGTKVEIT | QVQLVQSGAEVKKPGASVKVSCKASGYTFTNYDINWVRQVPGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25954 | SEQ ID NO: 29960 |
| **iPS:434887** | **21-225_85D6** | NA | GAAATTGTGTTGACGCAGTCTCCAGGCACCCTGTTTTTGTCTCAAGGGGAAAGAGCCACCCTCTCCTGCAGGGCCAGTCAGAGTGTTAGCAGCAGGTACTTAGCCTGGTACCAGCAGAAACCTGGCCAGGCTCCCAGACTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTATATTACTGTCAGCATTATGATAGCTCACCGTGGACGTTCGGCCAAGGGACCAAGGTGGAAATCAAA | CAGGTGCAGCTACAGCAGGGGGGCGCAGGACCGTTGAAGCCTTCGGAGACCCTGTCCCTCACCTGCGCTGTCTATGGTGGGTCCTTCAGTGGTTGCTACTGGAGCTGGATCCGCCAGCCCCCAGGGAAGGGGCTGGAGTGGATTGGGGAAATCAATTATAGTGGAAGAACCAACTACAACCCGTCCCTCAAGAGTCGAGTCACCATATCAGTAGACACGTCCAAGAACCAGTTCTCCCTGAAGCTGAGCTCTGTGACCGCCGCGGACACGGCTGTGTATTACTGTGCGAGAGACTACGGCGGTATGGACGTCTGGGGCCAAGGGACCACGGTCACCGTCTCCTCA |
| | | | SEQ ID NO: 25955 | SEQ ID NO: 29961 |

FIGURE 50

| | | | | |
|---|---|---|---|---|
| | | AA | EIVLTQSPGTLFLSQGERATLSCRASQSVSSRYLAWYQQKPGQAPRLLIYGASSRATGIPDRFSGSGSGTDFTLTISRLEPEDFAVYYCQHYDSSPWTFGQGTKVEIK | QVQLQQGGAGPLKPSETLSLTCAVYGGSFSGCYWSWIRQPPGKGLEWIGEINYSGRTNYNPSLKSRVTISVDTSKNQFSLKLSSVTAADTAVYYCARDYGGMDVWGQGTTVTVSS |
| | | | SEQ ID NO: 25956 | SEQ ID NO: 29962 |
| iPS:434891 | 21-225_85G6 | NA | GAAATTGCGTTGACGCAGTCTCCAGGCACCCTGTCTTTGTCTCCAGGGGAAAGAGCCACCCTCTCCTGCAGGGCCAGTCCGAGTGTTGACAGCAGCTACTTAGCCTGGTACCAGCAGAAACCTGGCCAGGCTCCCAGGCTCCTCATCTATGGTGCAGCCAGCAGGGCCCCTGGCATCCCAGACAGGTTCAGTGGCAGTGGGTCTGGGACAGACTTCACTCTCACCATCAGCAGACTGGAGCCTGAAGACTTTGTAGTGTATTACTGTCAGCAGTATGAAAGTTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGATTGCTACTGGAGCTGGATCCGCCAGCCCCCAGGGAAGGGGCTGGAGTGGATTGGGGAAATCAATCATAGTGGAAGGACCAACTACAACCCGTCCCTCAAGAGTCGAGTCACCATATCAGTAGACACGTCCGAGAACCAGTTCTCCCTGAAGCTGAGCTCTGTGACCGCCGCGGACACGGCTGTGTATTACTGTGCGAGAGACTACGGTGGGCTTGACTACTGGGGCCAGGGAACCCTGGTCACCGTCTCCTCA |
| | | | SEQ ID NO: 25957 | SEQ ID NO: 29963 |
| | | AA | EIALTQSPGTLSLSPGERATLSCRASPSVDSSYLAWYQQKPGQAPRLLIYGAASRAPGIPDRFSGSGSGTDFTLTISRLEPEDFVVYYCQQYESSPWTFGQGTKVEIK | QVQLQQWGAGLLKPSETLSLTCAVYGGSFSDCYWSWIRQPPGKGLEWIGEINHSGRTNYNPSLKSRVTISVDTSENQFSLKLSSVTAADTAVYYCARDYGGLDYWGQGTLVTVSS |
| | | | SEQ ID NO: 25958 | SEQ ID NO: 29964 |

FIGURE 50

| | | | | |
|---|---|---|---|---|
| **iPS:434895** | **21-225_74H7** | NA | GAACTTGTGTTGACGCAGTCTCCAGGCACCCTGTCTTTGTCTCCAGGGGAAAGAGCCACCCTCTCCTGCAGGGCCAGTCAGAATATTTACAGCAGCTACTTAGCCTGGTACCAACAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTATTGCTGTCAGCAGTATGAAAGCTCACCGTGGACCTTCGGCCAAGGGACCAAGGTGGAAATCAAA | CAAGTGCAGCTACAGCAGTGGGGCGCAGGACTGTTGAAGCCTTCGGAGCCCCTGTCCCTCACCTGCGCTGTCTATGGTGGGTCCTTCAGTGGTCCCTACTGGAGCTGGATCCGCCAGCCCCCGGGAAGGGGCTGGAGTGGATTGGGGAAATCAATCATAGTGGAAGCACCAACTACAACCCGTCCCTCAAGAGTCGAGTCACCATATCAGTAGACACGTCCAAGAACCAGTTCTCCCTGAAACTGACCTCTGTGACCGCCGCGGACACGGCTGTGTATTACTGTGCGAGAGACTACGGGGGTTTGGACGTCTGGGGCCAAGGGACCACGGTCACCGTCTCCTCA |
| | | | SEQ ID NO: 25959 | SEQ ID NO: 29965 |
| | | AA | ELVLTQSPGTLSLSPGERATLSCRASQNIYSSYLAWYQQKPGQAPRLLIYGASSRATGIPDRFSGSGSGTDFTLTISRLEPEDFAVYCCQQYESSPWTFGQGTKVEIK | QVQLQQWGAGLLKPSEPLSLTCAVYGGSFSGPYWSWIRQPPGKGLEWIGEINHSGSTNYNPSLKSRVTISVDTSKNQFSLKLTSVTAADTAVYYCARDYGGLDVWGQGTTVTVSS |
| | | | SEQ ID NO: 25960 | SEQ ID NO: 29966 |
| **iPS:434899** | **21-225_85B9** | NA | GAAATTGTGTTGACGCAGTCTCCAGGCACCCTGTTTTTGTCTCCAGGGGAGAGAGCCACCCTCTCCTGCAGGGCCAGTCAGAGTGTTAACAGCAACTACTTAGCCTGGTACCGGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCATTCTCATCATCAGCAGACTGGAGCCTGAGGATTTTGCAGTGTATTACTGTCAGCAGTATGAAAGCTCGCCGTGGACGTTCGGCCAAGGGACCAAGGTGGAAATCAAA | CAGGTGCAGCTACAACAGTGGGGCGCAGGACTGTTGAAGCCTTCGGAGACCCTGTCCCTCACTTGCGCTGTCAATGGTGGGCCCTTCAGTGGTTGCTACTGGAGCTGGATCCGCCAGCCCCCAGGGAAGGGGCTGGAGTGGATTGGGGAAATCAATCATAGTGGAAGGACCAACTTCAACCCGTCCCTCAAGAGTCGAGTCACCATATCAGTTGACACGTCCAAGAACCAGTTCTCCCTGAAGCTGAGCTCTGTGACCGCCGCGGACACGGCTGTGTATTACTGTGCGAGAGACTACGGCGGTATGGACGTCTGGGGCCAAGGGACCACGGTCACCGTCTCCTCA |
| | | | SEQ ID NO: 25961 | SEQ ID NO: 29967 |

FIGURE 50

| | | | | |
|---|---|---|---|---|
| | | AA | EIVLTQSPGTLFLSPGERATLSCRASQSVNSNYLAWYRQKPGQAPRLLIYGASSRATGIPDRFSGSGSGTDFILIISRLEPEDFAVYYCQQYESSPWTFGQGTKVEIK | QVQLQQWGAGLLKPSETLSLTCAVNGGPFSGCYWSWIRQPPGKGLEWIGEINHSGRTNFNPSLKSRVTISVDTSKNQFSLKLSSVTAADTAVYYCARDYGGMDVWGQGTTVTVSS |
| | | | SEQ ID NO: 25962 | SEQ ID NO: 29968 |
| **iPS:434901** | **21-225_85H9** | NA | GACATCGTGATGACCCAGTCTCCAGACTCCCTGGCTGTGTCTCTGGGCGAGAGGGCCACCACCAATTGTAAGTCCAGCCAGAGTGTTTTACACAGGTCCAACAATTACAACTACTTAGCGTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTGACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAACTGAGCAGCCTGAGATCTGAGGACACGGCCGTGTATTACCGTGGGAGTACCAGTGGCTGGAACTTCTTTGACTACTGGGGCCAGGGAACCCTGGTCACCGTCTCCTCA |
| | | | SEQ ID NO: 25963 | SEQ ID NO: 29969 |
| | | AA | DIVMTQSPDSLAVSLGERATTNCKSSQSVLHRSNNYNYLAWYQQKPGQPPKLLIYWASTRESGVPDRFSGSGSGTDFTLTISSLQAEDVAVYYCQQYYSTPPTFGQGTKVEIK | QVQLVQSGAEVKKPGASVKVSCKASGYTLTNYDINWVRQATGQGLEWMGWMNPNSGNTGYAQKFQGRVTMTRNTSISTAYMELSSLRSEDTAVYYRGSTSGWNFFDYWGQGTLVTVSS |
| | | | SEQ ID NO: 25964 | SEQ ID NO: 29970 |

FIGURE 50

| | | | | |
|---|---|---|---|---|
| **iPS:434907** | **21-225_85G10** | NA | GAAATTGTGTTGACGCAGTCTCCAGGCTCCCT<br>GTCTTTGTCTCCAGGGGAAAGAGCCACCCTCT<br>CCTGCAGGGCCAGTCAGAGTGTTTGGAGCGGC<br>TACTTAGCCTGGTACCAGCAGAAACCTGGCCA<br>GGCTCCCAGGCTCCTCATCTATGGTGCATCTA<br>GCAGGGCCACTGGCATCCCAGACAGGTTCAGT<br>GGCAGTGGGTCTGGGACAGACTTCACTCTCAC<br>CATCAGCAGACTGGAGCCTGAAGATTTTGCAG<br>TGTATTTCTGTCAGCAGTATGAGAGTTCACCG<br>TGGACGTTCGGCCAAGGGACCAAGGTGGAAA<br>TCAAA | CAGGTACAGCTACAGCAGCGGGGCGCAGGACTG<br>TTGAAGCCTTCGGAGACCCTGTCCCTCACCTGCG<br>CTGTCTATGGTGGGTCCTTCAGTGGTTGTTACTGG<br>AGCTGGATCCGCCAGCCCCCGGGAAGGGGCTG<br>GAGTGGATTGGGGAAATCAATCATAGTGGAATC<br>ACCAACTACAACCCGTCCCTCAAGAGTCGAGTCA<br>CCATATCAGTAGACACGTCCAAGAACCAGTTCTC<br>CCTGAAGCTGACCTCTGTGACCGCCGCGGACACG<br>GCTGTGTATTACTGTGCGAGAGACTACGGTGGTT<br>TGGACGTCTGGGGCCAAGGGACCACGGTCACCG<br>TCTCCTCA |
| | | | SEQ ID NO: 25965 | SEQ ID NO: 29971 |
| | | AA | EIVLTQSPGSLSLSPGERATLSCRASQSVWSGYL<br>AWYQQKPGQAPRLLIYGASSRATGIPDRFSGSGS<br>GTDFTLTISRLEPEDFAVYFCQQYESSPWTFGQG<br>TKVEIK | QVQLQQRGAGLLKPSETLSLTCAVYGGSFSGCYWS<br>WIRQPPGKGLEWIGEINHSGITNYNPSLKSRVTISVD<br>TSKNQFSLKLTSVTAADTAVYYCARDYGGLDVWG<br>QGTTVTVSS |
| | | | SEQ ID NO: 25966 | SEQ ID NO: 29972 |
| **iPS:434909** | **21-225_85C11** | NA | GACATCGTGATGACCCAGTCTCCAGACTCCCT<br>GGCTGTGTCTCTGGGCGAGAGGGCCACCATCA<br>ACTGCAAGTCCAGCCAGAGTGTTTTGTACAGC<br>TCCAACAGTCACAACTTCTTAGCTTGGTACCA<br>GCAGAACCCAGGACAGCCTCCTAAACTGCTCA<br>TTTTCTGGGCATTTATCCGGGAATCCGGGGTC<br>CCTGAAGGATTCAGTGGCAGCGGGTCTGGGGC<br>AGATTTCACTCTCTCCATCAGCGGCCTACAGG<br>CAGAAGATGTGGCAGTTTATTACTGTCAGCAA<br>TATTATAGTACTCCGTGCAGTTTTGGCCAGGG<br>GACCAAGCTGGAGATCAAA | CAGGTGCAGCTGGTGCAGTCTGGGGCTGAGGTG<br>AAGAAGCCTGGGGCCTCAGTGAAGGTCTCCTGC<br>AAGGCTTCTGGATACACCTTCACCAATTATGATA<br>TCAACTGGGTGCGACAGGCCTCTGGACAAGGAC<br>TTGAGTGGATGGGATGGATGCACCCTAACAGTGG<br>TAACACAGGCTATGCACAGAAGTTCCAGGGCAG<br>AGTCACCATGACCAGGAACACCTCCATAAGCAC<br>AGCCTACATGGAGCTGAGCAGCCTGAGATCTGA<br>GGACTCGGCCGTATATTACTGTGCGTATAGTAGT<br>GGCTGGTACAAATTTGACTACTGGAGCCAGGGA<br>ACCCTGGTCACCGTCTCCTCA |
| | | | SEQ ID NO: 25967 | SEQ ID NO: 29973 |

FIGURE 50

| | | | | |
|---|---|---|---|---|
| | | AA | DIVMTQSPDSLAVSLGERATINCKSSQSVLYSSNSHNFLAWYQQNPGQPPKLLIFWAFIRESGVPEGFSGSGSGADFTLSISGLQAEDVAVYYCQQYYSTPCSFGQGTKLEIK | QVQLVQSGAEVKKPGASVKVSCKASGYTFTNYDINWVRQASGQGLEWMGWMHPNSGNTGYAQKFQGRVTMTRNTSISTAYMELSSLRSEDSAVYYCAYSSGWYKFDYWSQGTLVTVSS |
| | | | SEQ ID NO: 25968 | SEQ ID NO: 29974 |
| iPS:434911 | 21-225_85D11 | NA | GAATTTATGTTGACGCAGTCTCCAGGCACCCTGTCTTTGTCTACAGGGGAAAGAGCCACCCTCTCGTGCAGGTCCAGTCAGAGTGTTAGCAGCAGCTACTTAGTCTGGTATCAGCAGAAACCTGGCCAGGCTCCCAGGCTCCTCATCTATGGTGCATCCACCAGGGCCACTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTTGCTCACCGCTCACTTTCGGCGGAGGGACCAAGGTGGAGATCACA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGTCCCTGGACAAGGGCTTGAGTGGATGGGATGGATGCACCCTAACAGTGGTAACACAGGCTATGCACAGAAGTTCCAGGGCAGAGTCACCATGACCAGGAACACCTCCATCAGCACAGCCTACATGGAGCTGAGCAGCCTGAGATCTGAGGACACGGCCGTGTATTACTGTGCGATTAGTAGTGGCTGGTACTGGTTCGACCCCTGGGGCCAGGGAACCCTGGTCACCGTCTCCTCA |
| | | | SEQ ID NO: 25969 | SEQ ID NO: 29975 |
| | | AA | EFMLTQSPGTLSLSTGERATLSCRSSQSVSSSYLVWYQQKPGQAPRLLIYGASTRATGIPDRFSGSGSGTDFTLTISRLEPEYFAVYYCQQYGCSPLTFGGGTKVEIT | QVQLVQSGAEVKKPGASVKVSCKASGYTFTNYDINWVRQVPGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25970 | SEQ ID NO: 29976 |

FIGURE 50

| | | | | |
|---|---|---|---|---|
| **iPS:434913** | **21-225_86C1** | NA | GAAATTGTGTTGACGCAGTCTCCAGGCACCCTGTATTTGTCTCCAGGGGAAAGAGCCACCCTCTCCTGCAGGGCCAGTCAGAGTGTTTACAGCAGCTACTTAGCCTGGTACCAGCAGAAACCTGCCCAGGCTCCCAGGCTCCTCATCTATGGTGCATCCAGCCGGGCCACTGGCATCCCAGACAGGTTCAGTGGCAGTGGGTCTGGGACAGACTTCGCTCTCACCATCAGCAGACTGGAGCCTGAAGATTGTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25971 | SEQ ID NO: 29977 |
| | | AA | EIVLTQSPGTLYLSPGERATLSCRASQSVYSSYLAWYQQKPAQAPRLLIYGASSRATGIPDRFSGSGSGTDFALTISRLEPEDCAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25972 | SEQ ID NO: 29978 |
| **iPS:434921** | **21-225_86E4** | NA | GAAATTGTGTTGACGCAGTCTCCAGGCACCCGGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGACTGGAGCCTGAAGATTGTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25973 | SEQ ID NO: 29979 |

FIGURE 50

| | | | | |
|---|---|---|---|---|
| | | AA | EIVLTQSPGTRYLSPGERATLSCRASQSVYSSYL<br>AWYQQKPGQAPRLLIYGASSRSTGIPDRFSGSGS<br>GTDFALTISRLEPEDCAVYYCQHSDNSPWTFGQ<br>GTKVEIK | QVQLQQWGAGLLKPSETLSLTCAVHGGSFSGCYW<br>SWIRQPPGKGLEWIGEINHSGSTNYNPSLKSRVTISV<br>DTSKNQFSLKLSSVTAADTAVYYCARDYGGMDV<br>WGQGTTVTVSS |
| | | | SEQ ID NO: 25974 | SEQ ID NO: 29980 |
| iPS:434935 | 21-225_86E9 | NA | GACATCGTGATGACCCAGTGTCCAGACTCCCC<br>GGCTGTGTCTCTGGGCGAGAGGGCCACCATCA<br>ATTGCAAGTCCAGCCAGAGTGTTTTGCACAGA<br>TCCAACAATTATAATTACTTAGCTTGGTACCA<br>GCAGAAACCAGGACAGCCTCCTAACCTGCTCA<br>TTTACTGGGCATCTACCCGGGAATCCGGGGTC<br>CCTGACCGATTCAGTGGCAGCGGGTCTGGGAC<br>AGATTTCACTCTCACCATCAGCAGCCTGCAGG<br>CTGCTGATGTGGCAGTTTATTACTGTCAGCAA<br>TATCATAGTAGTCCACTGACGTTCGGCCAAGG<br>GACCACGGTGGAAATCAAA | CAGGTGCAGCTGGTGCAGTCTGGGGCTGAGGTG<br>AAGAAGCCTGGGGCCTCAGTGAAGGTCTCCTGC<br>AAGGCTTCTGGATACACCTTCACCAATTATGATA<br>TCAACTGGGTGCGACAGGCCACTGGACAAGGGC<br>TTGAGTGGATGGGATGGATGAACCCTAACAGTG<br>GTAACACAGGCTATGCACAGAAGTTCCAGGGCA<br>GAGTCACCATGACCAGGAGCACCTCCACAAGCA<br>CAGCCCACATGGAGCTGAGCAGCCTGAGATCTG<br>AGGACACGGCCGTGTATTACTGTGCGGTTTCCAG<br>TGGCTGGTCCTGGTTCGACCCCTGGGGCCAGGGA<br>ACCCTGGTCACCGTCTCCTCA |
| | | | SEQ ID NO: 25975 | SEQ ID NO: 29981 |
| | | AA | DIVMTQCPDSPAVSLGERATINCKSSQSVLHRSN<br>NYNYLAWYQQKPGQPPNLLIYWASTRESGVPD<br>RFSGSGSGTDFTLTISSLQAADVAVYYCQQYHSS<br>PLTFGQGTTVEIK | QVQLVQSGAEVKKPGASVKVSCKASGYTFTNYDIN<br>WVRQATGQGLEWMGWMNPNSGNTGYAQKFQGR<br>VTMTRSTSTSTAHMELSSLRSEDTAVYYCAVSSGW<br>SWFDPWGQGTLVTVSS |
| | | | SEQ ID NO: 25976 | SEQ ID NO: 29982 |

FIGURE 50

| | | | | |
|---|---|---|---|---|
| **iPS:434939** | **21-225_86C11** | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCCTCCCAGACAGGTTCAGTGGCAGTGGGTCTGGGACAGACTTCGCTCTCACCATCAGCAGACTGGAGCCTGAAGATTGTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25977 | SEQ ID NO: 29983 |
| | | AA | EIVLTQSPGTLYLSPGERATLSCRASQSVYSSYLAWYQQKPGQAPRLLIYGASSRSTGLPDRFSGSGSGTDFALTISRLEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25978 | SEQ ID NO: 29984 |
| **iPS:434943** | **21-225_87H1** | NA | GAAATTGTGTTGACGCAGTCTCCAGGCACCCTGTCTTTGTCTCCAGGGGAAAGAGCCACCCTCTCCTGCAGGGCCAGTCAGAGTGTTGACAGCAACTACTTAGCCTGGTACCAGCAGAAACCTGGCCAGGCTCCCAGGCTCCTCATCTATGGTGCATCTGCCAGGACCACTGGCATCCCAGACAGGTTCAGTGGCAGTGGGTCTGGGACAGACTTCACTCTCACCATCAGCAGACTGGAGCCTGAAGATTTTGCAGTGTATTCCTGTCAGCAGTATGAAAGCTCACCGTGGACGTTCGGCCAAGGGACCAAGGTGGAAATCAAA | CAGGTGCAGCTGCAGCCGTGGGGCGCAGGACTGTTGAAGCCTTCGGAGACCCTGTCCCTCACCTGCGCTGTCTATGGTGGGTCCTTCAGTGGTTACTACTGGAGCTGGATCCGCCAGCCCCCAGGGAAGGGGCTGGAGTGGATTGGGGAAATCAATCATAGTGGACGCACCAACTACAACCCGTCCCTCAAGAGTCGAGTCACCATATCAGTAGACACGTCCAAGGACCAGTTCTCCCTGAAGCTGAGCTCTGTGACCGCCGCGGACACGGCTGTGTATTACTGTGCGAGAGACTACGGTGGTTTGGACGTCTGGGGCCAAGGGACCACGGTCACCGTCTCCTCA |
| | | | SEQ ID NO: 25979 | SEQ ID NO: 29985 |

FIGURE 50

| | | | | |
|---|---|---|---|---|
| | | AA | EIVLTQSPGTLSLSPGERATLSCRASQSVDSNYLAWYQQKPGQAPRLLIYGASARTTGIPDRFSGSGSGTDFTLTISRLEPEDFAVYSCQQYESSPWTFGQGTKVEIK | QVQLQPWGAGLLKPSETLSLTCAVYGGSFSGYYWSWIRQPPGKGLEWIGEINHSGRTNYNPSLKSRVTISVDTSKDQFSLKLSSVTAADTAVYYCARDYGGLDVWGQGTTVTVSS |
| | | | SEQ ID NO: 25980 | SEQ ID NO: 29986 |
| **iPS:434945** | **21-225_87E5** | NA | GAATTTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGAGTGGAGCCTGAAGATTGTGCAGTGTATTACTGTCAGCATTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25981 | SEQ ID NO: 29987 |
| | | AA | EFVLTQSPGTLYLSPGERATLSCRASQSVYSSYLAWYQQKPGQAPRLLIYGASSRSTGIPDRFSGSGSGTDFALTISRVEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25982 | SEQ ID NO: 29988 |

FIGURE 50

| | | | | |
|---|---|---|---|---|
| **iPS:434947** | **21-225_87B7** | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAACCAGGGAAAGCCCCTAAGTCCCTGATCTATGCTGCATCCAGTTTGCACAGTGGGGTCCCATCAAAGTTCAGCGGCAGTGGATCTGGGACAGATTTCACTCTCACCATCAGCAGCCTGCAGCCTGAAGATTCTGCAACTTATTTCTGCCTACTCTATCTTACTTACCCGCTCACCTTCGGCCAAGGGACACGACTGGAGATTAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATTTTGGAGTGGGCTACTACGGTATGGACGTCTGGGGCCAAGGGACCACGGTCACCGTCTCCTCA |
| | | | SEQ ID NO: 25983 | SEQ ID NO: 29989 |
| | | AA | DIQMTQSPSSLSASVGDRVTITCRASQGISNYLAWFQQKPGKAPKSLIYAASSLHSGVPSKFSGSGSGTDFTLTISSLQPEDSATYFCLLYLTYPLTFGQGTRLEIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAVYYCARDFGVGYYGMDVWGQGTTVTVSS |
| | | | SEQ ID NO: 25984 | SEQ ID NO: 29990 |
| **iPS:434955** | **21-225_87C9** | NA | GAAATTGTGTTGACGCAGTCTCCAGGCACCCTGTATTTGTCTCCAGTGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGACTGGAGCCTGAAGATTGTGCAGTGTATTACTGTCAGCATTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25985 | SEQ ID NO: 29991 |

FIGURE 50

| | | | | |
|---|---|---|---|---|
| | | AA | EIVLTQSPGTLYLSPVERATLSCRASQSVYSSYLAWYQQKPGQAPRLLIYGASSRSTGIPDRFSGSGSGTDFALTISRLEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25986 | SEQ ID NO: 29992 |
| **iPS:434957** | **21-225_87A10** | NA | GAATTTGTGTTGACGCAGTCTCCAGGCACCCTGTATTTGTCTCCAGGGGAAAGAGCCACCCTCTCGTGCAGGGCCAGTCAGAGTGTTAGCAGCAGCTACTTAGCCTGGTACCAGCAGAAACCTGGCCAGGCTCCCAGGCTCCTCATCTATGGTGCATCCACCAGGGCCTCTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTAACTCACCGCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAATGGTAACACAGGCTATGCACAGAAGTTCCAGGGCAGAGTCACCATGACCAGGAACACCTCCATAAGCACAGCCTACATGGAGCTGAACAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTTA |
| | | | SEQ ID NO: 25987 | SEQ ID NO: 29993 |
| | | AA | EFVLTQSPGTLYLSPGERATLSCRASQSVSSSYLAWYQQKPGQAPRLLIYGASTRASGIPDRFSGSGSGTDFTLTISRLEPEYFAVYYCQQYGNSPLTFGGGTKVEIK | QVQLVQSGAEVKKPGASVKVSCKASGYTFTNYDINWVRQATGQGLEWMGWMHPNNGNTGYAQKFQGRVTMTRNTSISTAYMELNSLRSEDTAVYYCAISSGWYWFDPWGQGTLVTVSL |
| | | | SEQ ID NO: 25988 | SEQ ID NO: 29994 |

FIGURE 50

| | | | | |
|---|---|---|---|---|
| **iPS:434959** | **21-225_87E10** | NA | GACATCGTGATGACCCAGTCTCCAGACTCCCTGGCTGTGTCTCTGGGCGAGAGGGCCACCATCAAGTGCAAGTCCAGCCAGAGTGTTTTACACAGCTCCAACAATATGAACTACTTAGCTTGGTACCAGCAGAAACCTGGACAGCCTCCTAAACTGCTCATTTACTGGGCATCTACCCGGAAATCCGGGGTCCCTGACCGATTCAGTGGCACCGGGTCTGGGACAGATTTCACTCTCACCATCAGCAGCCTGCAGGCTGAAGATGTGGCAGTTTATTACTGTCAGCAATATTATAGTAGTCCGTGCAGTTTTGGCCAGGGGACCAAGCTGAAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCCTATAGCAGTGGCTGGTACTTCTTTGACTACTGGGGCCAGGGAACCCTGGTCACCGTCTCCTCA |
| | | | SEQ ID NO: 25989 | SEQ ID NO: 29995 |
| | | AA | DIVMTQSPDSLAVSLGERATIKCKSSQSVLHSSNNMNYLAWYQQKPGQPPKLLIYWASTRKSGVPDRFSGTGSGTDFTLTISSLQAEDVAVYYCQQYYSSPCSFGQGTKLKIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYYCAYSSGWYFFDYWGQGTLVTVSS |
| | | | SEQ ID NO: 25990 | SEQ ID NO: 29996 |
| **iPS:434961** | **21-225_87A12** | NA | GAAATTGTGTTGACGCAGTCTCCAGGCACCCTGTCTTTGTCTCCAGGGGAAAGAGCCACCCTCTCCTGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGTCATCCCAGACAGGTTCAGTGGCAGTGGGTGTGGGACAGACTTCGCTCTCACCATCAGCAGAGTGGAGCCTGAAGATTGTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25991 | SEQ ID NO: 29997 |

FIGURE 50

| | | | | |
|---|---|---|---|---|
| | | AA | EIVLTQSPGTRYLSPGERATLSCRASQSVYSSYLAWYQQKPGQAPRLLIYGASSRSTVIPDRFSGSGCGTDFALTISRVEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25992 | SEQ ID NO: 29998 |
| iPS:434965 | 21-225_88A1 | NA | AATATCGTGATGACCCAGTCTCCAGACTCCCTGGCTGTGTCTCTGGGCGCGAGGGCCACCATCAACTGCAAGTCCAGCCAGAGTGTTTTACACAGCTCCAACAATTACAACTACTTAACTTGGTACCAGCAGAAACCAGGACAGCCTCCTAAGCTGCTCCTTTACTGGGCATCTACCCGGAAATCCGGGGTCCCTGACCGATTCAGTGGCAGCGGGTCTGGGACAGATTTCACTCTCACCATCAGCAGCCTGCAGGCTGAAGATGTGGCAGTTTATTTCTGTCAGCAATATTATAGTTCTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGTATAGCAGTGGCTGGTACTACTTTGACTACTGGGGCCAGGGAACCCTGGTCACCGTCTCCTCA |
| | | | SEQ ID NO: 25993 | SEQ ID NO: 29999 |
| | | AA | NIVMTQSPDSLAVSLGARATINCKSSQSVLHSSNNYNYLTWYQQKPGQPPKLLLYWASTRKSGVPDRFSGSGSGTDFTLTISSLQAEDVAVYFCQQYYSSPPTFGQGTKVEIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYYCAYSSGWYYFDYWGQGTLVTVSS |
| | | | SEQ ID NO: 25994 | SEQ ID NO: 30000 |

FIGURE 50

| | | | | |
|---|---|---|---|---|
| **iPS:434969** | **21-225_88H1** | NA | GAAATTGTGTTGACGCAGTCTCCAGGCACCCTGTATTTGTCTCCAGGGGAAAGAGCCACCCTGTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACGGTCATCCCAGACAGGTTCAGTGGCAGTGGGTGTGGGACAGACTTCGCTCTCACCATCAGCAGAGTGGAGCCTGAAGATTGTGCAGTGTATTACTGTCAGCATTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 25995 | SEQ ID NO: 30001 |
| | | AA | EIVLTQSPGTLYLSPGERATLSCRASQSVYSSYLAWYQQKPGQAPRLLIYGASSRSTVIPDRFSGSGCGTDFALTISRVEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 25996 | SEQ ID NO: 30002 |
| **iPS:434971** | **21-225_88G2** | NA | GACATCGTGATGACCCAGTCTCCAGATTGCCTGGCTGTGTCTCTGGGCGAGAGGGCCACCATCAACTGCAAGTCCAGCCAGAGTGTTTTATACACCTCCAACAATAACAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTTGCAGTTTATTATTGTCAGCAATATTATAATACTCCGTGGACGTTTGTCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTCA |
| | | | SEQ ID NO: 25997 | SEQ ID NO: 30003 |

FIGURE 50

| | | | | |
|---|---|---|---|---|
| | | AA | DIVMTQSPDCLAVSLGERATINCKSSQSVLYTSNNNNYLAWYQQKPGQPPKLLIYWASTRESGVPDRFSGSGSGTDFTLTISSLQAEDVAVYYCQQYYNTPWTFVQGTKVEIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 25998 | SEQ ID NO: 30004 |
| iPS:434973 | 21-225_88B4 | NA | GACATCGTGATGACCCAGTCTCCAGACTCCCTGGCTGTGTCTCTGGGCGAGAGGGCCACCATCAACTGCAAGTCCAGCCAGAGTGTTTTATACATCTCCAACAATAATAATTACTTAGCTTGGTACCAGCAGAAACCAGGACAGCCTCCTAAACTGCTCATTTACTGGGCATCTACCCGGGAATCCGGGGTCCCTGCCCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTGACACAGGCTATGCACAGAAGTTCCAGGGCAGTGTCACCATGACCAGGAACACCTCCATAACCACAGCCTACATGGAGCTGAGCAGCCTGAGATCTGAGGACACGGCCGTGTATTACTGTTCGTATAGCAGTGGCTGGTACTACTTTGACTACTGGGGCCAGGGAACCCTGGTCACCGTCTCCTCA |
| | | | SEQ ID NO: 25999 | SEQ ID NO: 30005 |
| | | AA | DIVMTQSPDSLAVSLGERATINCKSSQSVLYISNNNNYLAWYQQKPGQPPKLLIYWASTRESGVPARFSGSGSGTDFTLTISSLQAEDVAVYYCQQYYSTPPTFGQGTKVEIK | QVQLVQSGAEVKKPGASVKVSCKASGYTFTNYDINWVRQATGQGLEWMGWMNPNSGDTGYAQKFQGSVTMTRNTSITTAYMELSSLRSEDTAVYYCSYSSGWYYFDYWGQGTLVTVSS |
| | | | SEQ ID NO: 26000 | SEQ ID NO: 30006 |

FIGURE 50

| | | | | |
|---|---|---|---|---|
| **iPS:434977** | **21-225_88A5** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATGATTACCCATTCACTTTCGGCCCTGGGACCAAAGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAGTTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCTGGAACACCTCCATACGCACTGCCTACATGGAGCTGAGCAGCCTGAGATCTGAGGACACGGCCGTGTATTACTGTGCGCGAGGGTTTTACGATTTTTTGACTGGTTATTCCCCCACCTACTACTACTACGATATGGACGTCTGGGGCCAAGGGACCACGGTCACCGTCTCCTCA |
| | | | SEQ ID NO: 26001 | SEQ ID NO: 30007 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNDYPFTFGPGTKVEIK | QVQLVQSGAEVKKPGASVKVSCKASGYTFTSYDINWVRQATGQGLEWMGWMNPNSGNTGYAQKFQGRVTMTWNTSIRTAYMELSSLRSEDTAVYYCARGFYDFLTGYSPTYYYYDMDVWGQGTTVTVSS |
| | | | SEQ ID NO: 26002 | SEQ ID NO: 30008 |
| **iPS:434981** | **21-225_88E7** | NA | GAAATTGTGTTGACGCAGTCTCCAGGCACCCTGTCTTTGTCTCCAGGGGAAAGAGCCACCCTCTCCTGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGAGTGGAGCCTGAAGATTGTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |

FIGURE 50

| | | | | |
|---|---|---|---|---|
| | | | SEQ ID NO: 26003 | SEQ ID NO: 30009 |
| | | AA | EIVLTQSPGTLSLSPGERATLSCRASQSVYSSYLAWYQQKPGQAPRLLIYGASSRSTGIPDRFSGSGSGTDFALTISRVEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26004 | SEQ ID NO: 30010 |
| iPS:434983 | 21-225_88F7 | NA | GAAATTGTGTTGACGCAGTCTCCAGGCACCCTGTCTTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGACTGGAGCCTGAAGATTGTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26005 | SEQ ID NO: 30011 |
| | | AA | EIVLTQSPGTLSLSPGERATLSCRASQSVYSSYLAWYQQKPGQAPRLLIYGASSRSTGIPDRFSGSGSGTDFALTISRLEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26006 | SEQ ID NO: 30012 |

FIGURE 50

| | | | | |
|---|---|---|---|---|
| iPS:434995 | 21-225_88G9 | NA | GAAATTGTGTTGACGCAGTCTCCAGGCACCCGGTATTTGTCTCCAGGGGAAAGAGCCACCCTCTCCTGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGAGTGGAGCCTGAAGATTGTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26007 | SEQ ID NO: 30013 |
| | | AA | EIVLTQSPGTRYLSPGERATLSCRASQSVYSSYLAWYQQKPGQAPRLLIYGASSRSTGIPDRFSGSGSGTDFALTISRVEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26008 | SEQ ID NO: 30014 |
| iPS:434997 | 21-225_88C10 | NA | GACATCGTGATGACCCAGTCTCCAGACTCCCTGGCTGTGTCTCTGGGCGAGAGGGCCACCATCAACTGCAAGTCCAGCCAGAGTGTTTTACACAGCTCCAACAATTGGAATTACTTAGCTTGGCACCAGCAGAAACCAGGACAGCCTCCTAAGTTGCTCATTCACTGGGCATTTACTCGGAAATCCGGGGTCCCTGACCGATTCAGTGGCGGCGGGTCTGGGACAAATTTCACTCTCACCATCAGCAGCCTGCAGGCTGAAGATGTGGCAGTTTATTACTGTCAGCAATATTATAGAGCTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAGGGTCTCCTGCAAGGCTTCTGGATACACCTTCAGCAATTATGATATCAACTGGGTGCGACAGGCCACTGGACAAGGGCTTGAGTGGATGGGATGGATGACCCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGAGTAGCAGTGGCTGGTACTACTTTGACTCCTGGGGCCAGGGAACCCTGGTCACCGTCTCCTTA |
| | | | SEQ ID NO: 26009 | SEQ ID NO: 30015 |

FIGURE 50

| | | | | |
|---|---|---|---|---|
| | | AA | DIVMTQSPDSLAVSLGERATINCKSSQSVLHSSNNWNYLAWHQQKPGQPPKLLIHWAFTRKSGVPDRFSGGGSGTNFTLTISSLQAEDVAVYYCQQYYRAPPTFGQGTKVEIK | QVQLVQSGAEVKKPGASVRVSCKASGYTFSNYDINWVRQATGQGLEWMGWMTPNSGNTGYAQKFQGRVTMTRNTSISTAYMELSSLRSEDTAVYYCASSSGWYYFDSWGQGTLVTVSL |
| | | | SEQ ID NO: 26010 | SEQ ID NO: 30016 |
| iPS:434999 | 21-225_75A8 | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGGCCACTGGCATCCCAGACAGGTTCAGTGGCAGTGGGTCTGGGACAGACTTCGCTCTCACCATCAGCAGAGTGGAGCCTGAAGATTGTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26011 | SEQ ID NO: 30017 |
| | | AA | EIVLTQSPGTLYLSPGERATLSCRASQSVYSSYLAWYQQKPGQAPRLLIYGASSRATGIPDRFSGSGSGTDFALTISRVEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26012 | SEQ ID NO: 30018 |

FIGURE 50

| | | | | |
|---|---|---|---|---|
| **iPS:435009** | **21-225_89G4** | NA | GATATTGTGATGACTCAGTCTCCACTCTCCCTGCCCGTCACCCCTGGAGAGCCGGCCTCCATCTCCTGCAGGTCTAGTCAGAGCCTCCTGCATAGTAGTGGATACAACTATTTGGATTGGTACCTGCAGAAGCCAGGGCAGTCTCCACAGCTCCTGATCTATTTGGGTTCTAATCGGGCCTCCGGGGTCCCTGACAGGTTCAGTGGCAGTGGTTCAGGCACAGATTTTACACTGAAAATCAGCAGAGTGGAGGCTGAGGATATTGGGGTTTATTACTGCATGCAAGCTCTACATATTCCTCTCACTTTCGGCGGAGGGACCAAGGTGGAGATCAAA | GAGGTGCAGCTGGTGGAGTCTGGGGGAGGCTTGGTACAGCCGGGGGGGTCCCTGAGACTCTCCTGTGCAGCCTCTGGATTCACCTTCAGTAGCTACGACATGCACTGGGTCCGCCAAGCTACAGGAAAAGGTCTGGAGTGGGTCTCAGCTATTGGTACTGCTGGTGACACATACTATCCAGGCTCCGTGAAGGGCCGATTCACCATCTCCAGAGAAAATGCCAAGAACTCCTTGTATCTTCAAATGAACAGCCTGAGAGCCGGGGACACGGCTGTGTATTTCTGTGCAAGAGCTCTTGACTACGGTGACTCCTTGGGCTACTACTACTACGGTATGGACGTCTGGGGCCAAGGGACCACGGTCACCGTCTCCTCA |
| | | | SEQ ID NO: 26013 | SEQ ID NO: 30019 |
| | | AA | DIVMTQSPLSLPVTPGEPASISCRSSQSLLHSSGYNYLDWYLQKPGQSPQLLIYLGSNRASGVPDRFSGSGSGTDFTLKISRVEAEDIGVYYCMQALHIPLTFGGGTKVEIK | EVQLVESGGGLVQPGGSLRLSCAASGFTFSSYDMHWVRQATGKGLEWVSAIGTAGDTYYPGSVKGRFTISRENAKNSLYLQMNSLRAGDTAVYFCARALDYGDSLGYYYYGMDVWGQGTTVTVSS |
| | | | SEQ ID NO: 26014 | SEQ ID NO: 30020 |
| **iPS:435013** | **21-225_89D5** | NA | GAAATTGTGTTGACGCAGTCTCCAGGCACCCTGTATTTGTCTTCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGACTGGAGCCTGAAGATTTTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26015 | SEQ ID NO: 30021 |

FIGURE 50

| | | | | |
|---|---|---|---|---|
| | | AA | EIVLTQSPGTLYLSSGERATLSCRASQSVYSSYLAWYQQKPGQAPRLLIYGASSRSTGIPDRFSGSGSGTDFALTISRLEPEDFAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26016 | SEQ ID NO: 30022 |
| iPS:435015 | 21-225_89H5 | NA | GAAATTGTGTTGACGCAGTCTCCAGGCACCCTGTCTTTGTCTCCAGGGGAAAGAGCCACCCTCTCCTGCAGGGCCAGTCAGAGTGTTAACAGCAACTACTTAGCCTGGTACCAGCAGAAACCTGGCCAGGCTCCCAGGCTCCTCATCTATGGTGCATTCAGCAGGGCCACTGGCATCCCAGACAGGGTCAGTGGCAGTGGGTCTGGGACAGACTTCAATCTCATCATCAGCAGACTGGAGCCTGAAGATTTTGCAGTGTATTACTGTCAGCAGTATGAAAGCTCAGTGTGGACGTTCGGCCAAGGGACCAAGGTGGAAATCAAA | CAGGTGCAGCTACAACAGTGGGGCGCAGGACTGTTGAAGCCTTCGGAGACCCTGTCCCTCACTTGCGCTGTCTATGGTGGGTCCTTCAGTGGTTGCTACTGGAGCTGGATCCGCCAGCCCCCAGGGAAGGGGCTGGAGTGGATTGGGGAAATCAATTATAGTGGAAGCACCAACTTCAACCCGTCCCTCAAGAGTCGAGTCACCATATCAGCTGACACGTCCAAGAACCAGTTCTCCCTGAAGCTGAGCTCTGTGACCGCCGCGGACACGGCTGTGTATTACTGTGCGAGAGACTACGGCGGTATGGACGTCTGGGGCCAAGGGACCACGGTCACCGTCTCCTCA |
| | | | SEQ ID NO: 26017 | SEQ ID NO: 30023 |
| | | AA | EIVLTQSPGTLSLSPGERATLSCRASQSVNSNYLAWYQQKPGQAPRLLIYGAFSRATGIPDRVSGSGSGTDFNLIISRLEPEDFAVYYCQQYESSVWTFGQGTKVEIK | QVQLQQWGAGLLKPSETLSLTCAVYGGSFSGCYWSWIRQPPGKGLEWIGEINYSGSTNFNPSLKSRVTISADTSKNQFSLKLSSVTAADTAVYYCARDYGGMDVWGQGTTVTVSS |
| | | | SEQ ID NO: 26018 | SEQ ID NO: 30024 |

FIGURE 50

| | | | | |
|---|---|---|---|---|
| iPS:435025 | 21-225_89E10 | NA | GAAATTGTGTTGACGCAGTCTCCAGGCACCCTGTATTTGTCTTCAGGGGAAAGAGCCACCCTCTCCTGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGTCATCCCAGACAGGTTCAGTGGCAGTGGGTCTGGGACAGACTTCGCTCTCACCATCAGCAGAGTGGAGCCTGAAGATTTTGCAGTGTATTACTGTCAGCATTCTGATAACTCTCCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26019 | SEQ ID NO: 30025 |
| | | AA | EIVLTQSPGTLYLSSGERATLSCRASQSVYSSYLAWYQQKPGQAPRLLIYGASSRSTVIPDRFSGSGSGTDFALTISRVEPEDFAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26020 | SEQ ID NO: 30026 |
| iPS:435029 | 21-225_89A11 | NA | GAAATTGTGTTGACGCAGTCTCCAGGCACCCTGTCTTTGTCTCCAGGGGAAAGAGCCACCCTCTCCTGCAGGGCCAGTCAGAGTGTTGACAGCAACTTCTTAGCCTGGTACCAGCAGAAACCTGGCCAGGCTCCCAGGCTCCTCATCTATGGTGCATCTGCCAGGACCACTGGCATCCCAGACAGGTTCAGTGGCAGTGGGTCTGGGACAGACTTCACTCTCACCATCAGCAGACTGGAGCCTGAAGATTTTGCAGTGTATTCCTGTCAGCAGTATGAAATCTCACCGTGGACGTTCGGCCAAGGGACCAAGGTGGAAATCAAA | CAGGTGCAGCTGCAGCCGTGGGGCGCAGGACTGTTGAAGCCTTCGGAGACCCTGTCCCTCACCTGCGCTGTCTATGGTGGGTCCTTCAGTGGTTACTACTGGAGCTGGATCCGCCAGCCCCCAGGGAAGGGGCTGGAGTGGATTGGGGAAATCAATCATAGTGGACGCACCAGCTACAACCCGTCCCTCAAGAGTCGAGTCACCATATCAGTAGACACGTCCAAGAACCAGTTCTCCCTGAAGCTGAGCTCTGTGACCGCCGCGGACACGGCTGTGTATTACTGTGCGAGAGACTACGGTGGTTTGGACGTCTGGGGCCAAGGGACCACGGTCACCGTCTCCTCA |
| | | | SEQ ID NO: 26021 | SEQ ID NO: 30027 |

FIGURE 50

| | | | | |
|---|---|---|---|---|
| | | AA | EIVLTQSPGTLSLSPGERATLSCRASQSVDSNFLAWYQQKPGQAPRLLIYGASARTTGIPDRFSGSGSGTDFTLTISRLEPEDFAVYSCQQYEISPWTFGQGTKVEIK | QVQLQPWGAGLLKPSETLSLTCAVYGGSFSGYYWSWIRQPPGKGLEWIGEINHSGRTSYNPSLKSRVTISVDTSKNQFSLKLSSVTAADTAVYYCARDYGGLDVWGQGTTVTVSS |
| | | | SEQ ID NO: 26022 | SEQ ID NO: 30028 |
| iPS:435039 | 21-225_90G4 | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGTCATCCCAGACAGGTTCAGTGGCAGTGGGTCTGGGACAGACTTCGCTCTCACCATCAGCAGAGTGGAGCCTGAAGATTGTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26023 | SEQ ID NO: 30029 |
| | | AA | EIVLTQSPGTLYLSPGERATLSCRASQSVYSSYLAWYQQKPGQAPRLLIYGASSRSTVIPDRFSGSGSGTDFALTISRVEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26024 | SEQ ID NO: 30030 |

FIGURE 50

| | | | | |
|---|---|---|---|---|
| **iPS:435041** | **21-225_90A5** | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGGCCACTGGCATCCCAGACAGGTTCAGTGGCAGTGGGTCTGGGACAGACTTCGCTCTCACCATCAGCAGAGTGGAGCATGAAGATTTTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26025 | SEQ ID NO: 30031 |
| | | AA | EIVLTQSPGTLYLSPGERATLSCRASQSVYSSYLAWYQQKPGQAPRLLIYGASSRATGIPDRFSGSGSGTDFALTISRVEHEDFAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26026 | SEQ ID NO: 30032 |
| **iPS:435043** | **21-225_90G5** | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCATCATTTATGGTGCATCCAGCCGGGCCACTGGCATCCCAGACAGGTTCAGTGGCAGTGGGTCTGGGACAGACTTCGCTCTCACCATCAGCAGAGTGGAGCATGAAGATTTTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26027 | SEQ ID NO: 30033 |

FIGURE 50

| | | | | |
|---|---|---|---|---|
| iPS:435045 | 21-225_90H5 | AA | EIVLTQSPGTLYLSPGERATLSCRASQSVYSSYLAWYQQKPGQAPRLIIYGASSRATGIPDRFSGSGSGTDFALTISRVEHEDFAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26028 | SEQ ID NO: 30034 |
| | | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATATTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGATCACCTTCGGCCAAGGGACACGACTGGAGATTAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGTCTCCAGGCAAGGGGCTGGAGTGGGTGGCAGTTATATGGTATGAAGGAAGTAATACATACTATGCAGACTCCGTGAAGGGCCGATTCACCATCTCCAGAGACAATTCCAAGAACACGCTGTATCTGCAAATGAACAGCCTGAGAGCCGAGGACACGGCTGTATATTACTGTGTGAGAGAGATGGGGTGGTTAGATGACTACTGGGGCCAGGGAACCCTGGTCACCGTCTCCTCA |
| | | | SEQ ID NO: 26029 | SEQ ID NO: 30035 |
| | | AA | DIQMTQSPSSLSASVGDRVTITCRASQGIRNDLGWYQQKPGKAPKRLIYIASSLQSGVPSRFSGSGSGTEFTLTISSLQPEDFATYYCLQHNSYPITFGQGTRLEIK | QVQLVESGGGVVQPGRSLRLSCAASGFTFSDYGMHWVRQSPGKGLEWVAVIWYEGSNTYYADSVKGRFTISRDNSKNTLYLQMNSLRAEDTAVYYCVREMGWLDDYWGQGTLVTVSS |
| | | | SEQ ID NO: 26030 | SEQ ID NO: 30036 |

FIGURE 50

| | | | | |
|---|---|---|---|---|
| **iPS:435051** | **21-225_90D9** | NA | GACATCGTGATGACCCAGTCTCCAGACTCCCT<br>GGCTGTGTCTCTGGGCGAGAGGGCCACCATCA<br>TCTGCAAGTCCAGCCAGACTGTTTTACACAGC<br>TCCAACAATTATAACTACTTAGCTTGGTACCA<br>GCAGAAGCCAGGACAGCCCCCTAAGGTGCTC<br>ATTTACTGGACATCTACCCGGGAATCCGGGGT<br>CCCTGACCGATTCAGTGGCAGCGGGTCTGGGA<br>CAGATTTCACTCTCACCATCAGCAGCCTGCAG<br>GCTGAAGATGTGGCAGTTTATTACTGTCAGCA<br>ATATCTTAGTAGTCCTCTGACGTTCGGCCAAG<br>GGACCAAGGTGGAAATCAAA | CAGGTGCAGCTGGTGCAGTCTGGGGCTGAGGTG<br>AAGAAGCCTGGGGCCTCAGTGAAGGTCTCCTGC<br>AAGGCTTCTGGATACACCTTCCCCAATTATGATA<br>TCAACTGGGTGCGACAGGCCACTGGACAAGGGC<br>TTGAGTGGATGGGATGGATGAACCCTAACAGTG<br>GTAACACAGGCTATGCACAGAAGTTCCAGGGCA<br>GAGTCACCATGACCAGGAACACCTCCATAAGCA<br>CAGCCTACATGGAGCTGAGCAGCCTGAGATCTG<br>AGGACACGGCCGTGTATTATTGTGCGGTTTCCAG<br>TGGCTGGCACTGGTTCGACCCCTGGGGCCAGGGA<br>ACCCTGGTCACCGTCGCCTCA |
| | | | SEQ ID NO: 26031 | SEQ ID NO: 30037 |
| | | AA | DIVMTQSPDSLAVSLGERATIICKSSQTVLHSSNN<br>YNYLAWYQQKPGQPPKVLIYWTSTRESGVPDRF<br>SGSGSGTDFTLTISSLQAEDVAVYYCQQYLSSPL<br>TFGQGTKVEIK | QVQLVQSGAEVKKPGASVKVSCKASGYTFPNYDIN<br>WVRQATGQGLEWMGWMNPNSGNTGYAQKFQGR<br>VTMTRNTSISTAYMELSSLRSEDTAVYYCAVSSGW<br>HWFDPWGQGTLVTVAS |
| | | | SEQ ID NO: 26032 | SEQ ID NO: 30038 |
| **iPS:435053** | **21-225_75F9** | NA | GACATCGTGATGACCCAGTCTCCAGACTCCCT<br>GCCTGTGTCTCTGGGCGAGAGGGCCACCGTCA<br>ACTGCAAGTCCAGCCAGAGTGTTTTACACAAC<br>TCCAACAATAATAACTACTTGGCTTGGTACCA<br>GCAGAAACCAGGACAGCCTCCTAAGCTGCTCA<br>TTTACTGGGCATCTACGCGGGAGTCCGGGGTC<br>CCTGACCGATTCAGTGGCAGCGGGTCTGGGAC<br>AGATTTCACTCTCACCATCAGCAGCCTGCAGG<br>CTGAAGATGTGGCAGTTTATTACTGTCAACAA<br>TATTATAGTAGTCCTCCGACGTTCGGCCAAGG<br>GACCAAGGTGGAAATCAAA | CAGGTGCAGCTGGTGCAGTCTGGGGCTGAGGTG<br>AAGAAGCCTGGGGCCTCAGTGAAGGTCTCCTGC<br>AAGGCTTCTGGATACACCTTCACCAATTATGATA<br>TCAACTGGGTGCGACAGGCCACTGGACAAGGGC<br>TTGAGTGGATGGGATGGATGCACCCTAACAGTGG<br>TAACACAGGCTATGCACAGAAGTTCCAGGGCAG<br>AGTCACCATGACCAGGAACACCTCCATAAGCAC<br>AGCCTACATGGAGCTGAGCAGCCTGAGATCTGA<br>GGACACGGCCGTGTATTACTGTGCCTATAGCAGT<br>GGCTGGTACATCTTTGACTACTGGGGCCAGGGAA<br>CCCTGGTCACCGTCTCCTCA |
| | | | SEQ ID NO: 26033 | SEQ ID NO: 30039 |

FIGURE 50

| | | | | |
|---|---|---|---|---|
| | | AA | DIVMTQSPDSLPVSLGERATVNCKSSQSVLHNSNNNNYLAWYQQKPGQPPKLLIYWASTRESGVPDRFSGSGSGTDFTLTISSLQAEDVAVYYCQQYYSSPPTFGQGTKVEIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAYSSGWYIFDYWGQGTLVTVSS |
| | | | SEQ ID NO: 26034 | SEQ ID NO: 30040 |
| iPS:435055 | 21-225_90F10 | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGACTGGAGCCTGAAGATTGTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26035 | SEQ ID NO: 30041 |
| | | AA | EIVLTQSPGTLYLSPGERATLSCRASQSVYSSYLAWYQQKPGQAPRLLIYGASSRSTGIPDRFSGSGSGTDFALTISRLEPEDCAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26036 | SEQ ID NO: 30042 |

FIGURE 50

| | | | | |
|---|---|---|---|---|
| iPS:435059 | 21-225_90C11 | NA | GATATTGTGATGACTCAGTCTCCACTCTCCCTGCCCGTCACCCCTGGAGAGCCGGCCTCCATCTCATGCAGGTATAGTCAGAGCCTCGTGCATAGTAGTGGATACAACTATTTGGATTGGTACCTGCAGAAGCCAGGGCAGTCTCCACAGCTCGTTATCTATTTGGGTTCTAATCGGGCCTCCGGGGTCCCTGACAGGTTCAGTGGCAGTGGATCAGGTTCAGATTTTACACTGAAGATCAGCAGAGTGGAGGCTGAGGATGTTGGACTTTATTACTGCATGCAAGCTCTACACCCTCCTCTCACTTTCGGCGGAGGGACCAAGGTGGAGATCAAA | GAGGTGCAGCTGGTGGAGTCTGGGGGAGGCTTGGTACAGCCGGGGGGGTCCCTGAGACTCTCCTGTGCAGCCTCTGGATTCACCTTCAGTAACTACGACATGCACTGGGTCCGCCAAGCTACAGGAAAAGGTCTGGAGTGGGTCTCAGCTATTGGTACTGCTGGTGACACATACTATCCAGGCTCCGTGAAGGGCCGATTCACCATCTCCAGAGAAAATGCCAAGAACTCCTTGTATCTTCAAATGAACAGCCTGAGAGCCGGGGACACGGCTGTGTATTACTGTGCAAGAGTTCTTGACTACGGTGACTCCTTGGGCTACTACTACTACGGTATGGACGTCTGGGGCCAAGGGACCACGGTCACCGTCTCCTCA |
| | | | SEQ ID NO: 26037 | SEQ ID NO: 30043 |
| | | AA | DIVMTQSPLSLPVTPGEPASISCRYSQSLVHSSGYNYLDWYLQKPGQSPQLVIYLGSNRASGVPDRFSGSGSGSDFTLKISRVEAEDVGLYYCMQALHPPLTFGGGTKVEIK | EVQLVESGGGLVQPGGSLRLSCAASGFTFSNYDMHWVRQATGKGLEWVSAIGTAGDTYYPGSVKGRFTISRENAKNSLYLQMNSLRAGDTAVYYCARVLDYGDSLGYYYYGMDVWGQGTTVTVSS |
| | | | SEQ ID NO: 26038 | SEQ ID NO: 30044 |
| iPS:435071 | 21-225_91F1 | NA | GACATCGTGATGACCCAGTCTCCAGATTGCCTGGCTGTGTCTCTGGGCGAGAGGGCCACCATCAACTGCAAGTCCAGCCAGAGTGTTTTATACACCTCCAACAATAACAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTTGCAGTTTATTATTGTCAGCACTATTATAATACTCCGTGGAAGTTTGTCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTCA |
| | | | SEQ ID NO: 26039 | SEQ ID NO: 30045 |

FIGURE 50

| | | | | |
|---|---|---|---|---|
| | | AA | DIVMTQSPDCLAVSLGERATINCKSSQSVLYTSNNNNYLAWYQQKPGQPPKLLIYWASTRESGVPDRFSGSGSGTDFTLTISSLQAEDVAVYYCQHYYNTPWKFVQGTKVEIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 26040 | SEQ ID NO: 30046 |
| iPS:435073 | 21-225_91B2 | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGAGTGGAGCCTGAAGATTGTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26041 | SEQ ID NO: 30047 |
| | | AA | EIVLTQSPGTLYLSPGERATLSCRASQSVYSSYLAWYQQKPGQAPRLLIYGASSRSTGIPDRFSGSGSGTDFALTISRVEPEDCAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26042 | SEQ ID NO: 30048 |

FIGURE 50

| | | | | |
|---|---|---|---|---|
| **iPS:435075** | **21-225_91B3** | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGAGTGGAGCATGAAGATTGTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26043 | SEQ ID NO: 30049 |
| | | AA | EIVLTQSPGTLYLSPGERATLSCRASQSVYSSYLAWYQQKPGQAPRLLIYGASSRSTGIPDRFSGSGSGTDFALTISRVEHEDCAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26044 | SEQ ID NO: 30050 |
| **iPS:435077** | **21-225_91F3** | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGCCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGAGTGGAGCCTGAAGATTGTGCAGTGTATTACTGTCAGCATTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26045 | SEQ ID NO: 30051 |

FIGURE 50

| | | | | |
|---|---|---|---|---|
| | | AA | EIVLTQSPGTLYLSPGERATLSCRASQSVYSSYLAWYQQKPAQAPRLLIYGASSRSTGIPDRFSGSGSGTDFALTISRVEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26046 | SEQ ID NO: 30052 |
| iPS:435079 | 21-225_91B4 | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCATCATTTATGGTGCATCCAGCCGGTCCACTGGCATCCCAGACAGGTTCAGTGGCAGTGGGTCTGGGACAGACTTCGCTCTCACCATCAGCAGAGTGGAGCCTGAAGATTGTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26047 | SEQ ID NO: 30053 |
| | | AA | EIVLTQSPGTLYLSPGERATLSCRASQSVYSSYLAWYQQKPGQAPRLIIYGASSRSTGIPDRFSGSGSGTDFALTISRVEPEDCAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26048 | SEQ ID NO: 30054 |

FIGURE 50

| | | | | |
|---|---|---|---|---|
| **iPS:435087** | **21-225_91G8** | NA | GACATCGTGATGACCCAGTCTCCAGATTGCCTGGCTGTGTCTCTGGGCGAGAGGGCCACCATCAACTGCAAGTCCAGCCAGAGTGTTTTATACACCTCCAACAATAACAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTTGCAGTTTATTATTGTCAGCAATATTATACTACTCCGTGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTCA |
| | | | SEQ ID NO: 26049 | SEQ ID NO: 30055 |
| | | AA | DIVMTQSPDCLAVSLGERATINCKSSQSVLYTSNNNNYLAWYQQKPGQPPKLLIYWASTRESGVPDRFSGSGSGTDFTLTISSLQAEDVAVYYCQQYYTTPWTFGQGTKVEIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 26050 | SEQ ID NO: 30056 |
| **iPS:435089** | **21-225_91E9** | NA | GAAATTGTGTTGACGCAGTCTCCAGGCACCCTGTATTTGTCTCCAGGGGAAAGAGCCACCCTGTCATGCAGGGCCAGTCAGAGTGTTTACAGCAGCTACTTAGCGTGGTACCAGCAGAAACCTGGCCAGGCTCCCAGGCTCCTCATTTATGGTGCATCCAGCCGGGCCACGGGCATCCCAGACAGGTTCAGTGGCAGTGGGTGTGGGACAGACTTCGCTCTCACCATCAGCAGAGTGGAGCATGAAGATTGTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26051 | SEQ ID NO: 30057 |

FIGURE 50

| | | | | |
|---|---|---|---|---|
| | | AA | EIVLTQSPGTLYLSPGERATLSCRASQSVYSSYLAWYQQKPGQAPRLLIYGASSRATGIPDRFSGSGCGTDFALTISRVEHEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26052 | SEQ ID NO: 30058 |
| iPS:435097 | 21-225_92B1 | NA | GAAATTGTGTTGACGCAGTCTCCAGGCACCCTGTCTTTGTCTCCAGGGGAAAGAGCCACCCTCTCCTGCAGGGCCAGTCAGAGTGTTGGCAGCAACTACTTAGCCTGGTACCAGCAGAAACGTGGCCAGGCTCCCAGGCTCCTCATCTATGGTGCATCCAGCAGGGCCACTGACATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGAAAGTTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGGTTCCTACTGGAGCTGGATCCGCCAGCCCCCAGGGAAGGGGCTGGAGTGGATTGGGGAAATCAATTATAGGGGAAGCACCAACTACAACCCGTCCCTCAAGAGTCGAGTCGCCATATCAGTAGACACGTCCAAGAACCAGTTCTCCCTGAACCTGACCTCTGTGACCGCCGCGGACACGGCTGTGTATTACTGTGCGAGGGACTACGGCGGTTTGGACGTCTGGGGCCAAGGGACCACGGTCACCGTCTCCTCA |
| | | | SEQ ID NO: 26053 | SEQ ID NO: 30059 |
| | | AA | EIVLTQSPGTLSLSPGERATLSCRASQSVGSNYLAWYQQKRGQAPRLLIYGASSRATDIPDRFSGSGSGTDFTLTISRLEPEDFAVYYCQQYESSPWTFGQGTKVEIK | QVQLQQWGAGLLKPSETLSLTCAVYGGSFSGSYWSWIRQPPGKGLEWIGEINYRGSTNYNPSLKSRVAISVDTSKNQFSLNLTSVTAADTAVYYCARDYGGLDVWGQGTTVTVSS |
| | | | SEQ ID NO: 26054 | SEQ ID NO: 30060 |

FIGURE 50

| | | | | |
|---|---|---|---|---|
| **iPS:435103** | **21-225_92B2** | NA | GATATTGTGATGACTCAGTCTCCACTCTCCCTCCCTGTCACCCCTGGAGAGCCGGCCTCCATCTCATGCAGGTCTAGTCAGAGCCTCGTGCATAGTAGTGGATACAACTATTTGGATTGGTACCTGCAGAAGCCAGGGCAGTCTCCACAACTCGTGATCTATTTGGGTTCTAATCGGGCCTCCGGGGTCCCTGACAGGTTCAGTGGCAGTGGTTCAGTCACAGATTTTACACTGAGAATCAGCAGAGTGGAGGCTGAGGATATTGGGATTTATTATTGCATGCAAGCTCTACATATTCCTCTCACTTTCGGCGGAGGGACCAAGGTGGAGATCAAA | GAGGTGCAGCTGGTGGAGTCTGGGGGAGGCTTGGTACAGCCGGGGGGGTCCCTGAGACTCTCCTGTGCAGCCTCTGGATTCACCTTCAGTAACTACGACATGCACTGGGTCCGCCAAGCTACAGGAAAAGGTCTGGAGTGGGTCTCAGCTATTGGTACTGCTGGTGACACATACTATCCAGGCTCCGTGAAGGGCCGATTCACCATCTCCAGAGAAAATGCCAAGAACTCCTTGTATCTTCAAATGAACAGCCTGAGAGCCGGGGACACGGCTGTGTATTTCTGTGCAAGAGCTCTTGACTACGGTGACTCCTTGGGCTACTACTACTACGGTATGGACGTCTGGGGCCAAGGGACCACGGTCACCGTCTCCTCA |
| | | | SEQ ID NO: 26055 | SEQ ID NO: 30061 |
| | | AA | DIVMTQSPLSLPVTPGEPASISCRSSQSLVHSSGYNYLDWYLQKPGQSPQLVIYLGSNRASGVPDRFSGSGSVTDFTLRISRVEAEDIGIYYCMQALHIPLTFGGGTKVEIK | EVQLVESGGGLVQPGGSLRLSCAASGFTFSNYDMHWVRQATGKGLEWVSAIGTAGDTYYPGSVKGRFTISRENAKNSLYLQMNSLRAGDTAVYFCARALDYGDSLGYYYYGMDVWGQGTTVTVSS |
| | | | SEQ ID NO: 26056 | SEQ ID NO: 30062 |
| **iPS:435109** | **21-225_92H5** | NA | GAAATAGTGATGACGCAGTCTCCAGCCACCCTGTCTGTGTCTCCAGGGGAAAGAGCCACCCTCTCCTGCCGGGCCAGTCAGGATGTTATCACCTACTTAGCCTGGTACCAGCAGAAACCTGGCCAGGCTCCCAGGCTCCTCATCTATGGTGCATCCACCAGGGCCACTGGTGTCCCAGCCAGGTTCAGTGGCAGTGGGTCTGGGACAGAGTTCACTCTCACCATCACCAGCCTGCAGTCTGAAGATTTTGCACTTTATTACTGTCAGGAGTATAATGACTGGCCGTGCAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCCCTTTATAGTGGGAGCTACTTACTTTGACTACTGGGGCCAGGGAACCCTGGTCACCGTCTCCTCA |
| | | | SEQ ID NO: 26057 | SEQ ID NO: 30063 |

FIGURE 50

| | | | | |
|---|---|---|---|---|
| | | AA | EIVMTQSPATLSVSPGERATLSCRASQDVITYLAWYQQKPGQAPRLLIYGASTRATGVPARFSGSGSGTEFTLTITSLQSEDFALYYCQEYNDWPCSFGQGTKLEIK | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARDPFIVGATYFDYWGQGTLVTVSS |
| | | | SEQ ID NO: 26058 | SEQ ID NO: 30064 |
| **iPS:435111** | **21-225_92D6** | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGTCATCCCAGACAGGTTCAGTGGCAGTGGGTGTGGGACAGACTTCGCTCTCACCATCAGCAGACTGGAGCCTGAAGATTGTGCAGTGTATTACTGTCAGCATTATGATAACTCTCCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26059 | SEQ ID NO: 30065 |
| | | AA | EIVLTQSPGTLYLSPGERATLSCRASQSVYSSYLAWYQQKPGQAPRLLIYGASSRSTVIPDRFSGSGCGTDFALTISRLEPEDCAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26060 | SEQ ID NO: 30066 |

FIGURE 50

| | | | | |
|---|---|---|---|---|
| **iPS:435113** | **21-225_92E6** | NA | GACATCGTGATGACCCAGTCTCCAGACTCCCTGGCTGCGTCTCTGGGCGAGAGGGCCACCATCAACTGCAAGTCCAGTCAGAATATTTTATCCAGCTCCAACAATAAGAACTACTTAACTTGGTACCAGCAGAAACCAGGACAGCCTCCTAAAATACTCATTTACTGGACATCTACCCGGGAATCCGGGGTCCCTGACCGATTCAGTGGCAGCGGGTTTGGGACAGATTTCACTCTCACCATCAGCAGCCTGCAGGCTGAAGATGTGGCTGTTTATTACTGTCAGCAATATTTTAGTGTTCCTCCGACGTTCGGCCAAGGGACCAAGGTGGAAATCAAA | CAGGTGCAGCTGGTGCAGTCTGGGGCTGAGGTGAAGAAACCTGGGGCCTCAGTGAGGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATGAGCACAGCCTACATGGAGCTGAGCAGCCTGAGATCTGAGGACACGGCCGTGTATTACTGTGCCCATAGCAGTGGCTGGTACTTTTTTGACTACTGGGGCCAGGGAACCCTGGTCACCGTCTCCTCA |
| | | | SEQ ID NO: 26061 | SEQ ID NO: 30067 |
| | | AA | DIVMTQSPDSLAASLGERATINCKSSQNILSSSNNKNYLTWYQQKPGQPPKILIYWTSTRESGVPDRFSGSGFGTDFTLTISSLQAEDVAVYYCQQYFSVPPTFGQGTKVEIK | QVQLVQSGAEVKKPGASVRVSCKASGYTFTNYDINWVRQATGQGLEWMGWMHPNSGNTGYAQKFQGRVTMTRNTSMSTAYMELSSLRSEDTAVYYCAHSSGWYFFDYWGQGTLVTVSS |
| | | | SEQ ID NO: 26062 | SEQ ID NO: 30068 |
| **iPS:435115** | **21-225_77C5** | NA | GAAATTGTGTTGACGCAGTCTCCAGGCACCCTGTCTTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGACTGGAGCCTGAAGATTTTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26063 | SEQ ID NO: 30069 |

FIGURE 50

| | | | | |
|---|---|---|---|---|
| | | AA | EIVLTQSPGTLSLSPGERATLSCRASQSVYSSYLAWYQQKPGQAPRLLIYGASSRSTGIPDRFSGSGSGTDFALTISRLEPEDFAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26064 | SEQ ID NO: 30070 |
| **iPS:435167** | **21-225_92F12** | NA | GACATCGTGATGACCCAGTCTCCAGACTCCCTGGCTGTGTCTCTGGGCGAGAGGGCCACCACCAATTGTAAGTCCAGCCAGAGTGTTTTACACAGGTCCAACAATTACAACTACTTAGCGTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTGACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAACTGAGCAGCCTGAGATCTGAGGACACGGCCGTGTATTACCGTGGGAGTACCAGTGGCGGGAAGTTCTTCGACTACTGGGGCCAGGGAACCCTGGTCACCGTCTCCTCA |
| | | | SEQ ID NO: 26065 | SEQ ID NO: 30071 |
| | | AA | DIVMTQSPDSLAVSLGERATTNCKSSQSVLHRSNNYNYLAWYQQKPGQPPKLLIYWASTRESGVPDRFSGSGSGTDFTLTISSLQAEDVAVYYCQQYYSTPPTFGQGTKVEIK | QVQLVQSGAEVKKPGASVKVSCKASGYTLTNYDINWVRQATGQGLEWMGWMNPNSGNTGYAQKFQGRVTMTRNTSISTAYMELSSLRSEDTAVYYRGSTSGGKFFDYWGQGTLVTVSS |
| | | | SEQ ID NO: 26066 | SEQ ID NO: 30072 |

FIGURE 50

| | | | | |
|---|---|---|---|---|
| **iPS:435171** | **21-225_93C2** | NA | GAAATTGTGTTGACGCAGTCTCCAGGCACCCTGTATTTGTCTCCAGGGGAAAGAGCCACCCTCTCCTGCAGGGCCAGTCAGAGTGTTTACAGCAGCTACTTAGCCTGGTACCAGCAGAAACCTGGCCAGGCTCCCAGGCTCCTCATCTATGGTGCATCCAGCCGGGCCACTGGCATACCAGACAGGTTCAGTGGCAGTGGGTCTGGGACAGACTTCGCTCTCACCATCAGCAGACTGGAGCATGAAGATTGTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26067 | SEQ ID NO: 30073 |
| | | AA | EIVLTQSPGTLYLSPGERATLSCRASQSVYSSYLAWYQQKPGQAPRLLIYGASSRATGIPDRFSGSGSGTDFALTISRLEHEDCAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26068 | SEQ ID NO: 30074 |
| **iPS:435177** | **21-225_93E4** | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTGTGGGACAGACTTCGCTCTCACCATCAGCAGAGTGGAGCCTGAAGATTTTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26069 | SEQ ID NO: 30075 |

FIGURE 50

| | | | | |
|---|---|---|---|---|
| | | AA | EIVLTQSPGTLYLSPGERATLSCRASQSVYSSYLAWYQQKPGQAPRLLIYGASSRSTGIPDRFSGSGCGTDFALTISRVEPEDFAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26070 | SEQ ID NO: 30076 |
| **iPS:435183** | **21-225_93E9** | NA | GAAATTGTGTTGACGCAGTCTCCAGGCACCCTGTCTTTGTCTCCAGGGGAAAGAGCCACCCTCTCCTGCAGGGCCAGTCAGAGTGTTGACAGCAGCTACCTAGCCTGGTACCAGCAGAAAACTGGCCAGGCTCCCAGGCTCCTCATCTATGGTGCATCCAGCAGGGCCCCTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGAAAGCTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGGTTGCTACTGGAGCTGGATCCGCCAGCCCCCAGGGAAGGGGCTGGAGTGGATTGGGGAAATCAATCATAGTGGAAGGACCAACTACAACCCGTCCCTCAAGAGTCGAGTCACCATATCAGTAGACACGTCCGAGAACAAGTTCTCCCTGAAGCTGAGCTCTGTGACCGCCGCGGACACGGCTGTGTATTACTGTGCGAGAGACTACGGTGGGCTTGACTACTGGGGCCAGGGAACCCTGGTCACCGTCTCCTCA |
| | | | SEQ ID NO: 26071 | SEQ ID NO: 30077 |
| | | AA | EIVLTQSPGTLSLSPGERATLSCRASQSVDSSYLAWYQQKTGQAPRLLIYGASSRAPGIPDRFSGSGSGTDFTLTISRLEPEDFAVYYCQQYESSPWTFGQGTKVEIK | QVQLQQWGAGLLKPSETLSLTCAVYGGSFSGCYWSWIRQPPGKGLEWIGEINHSGRTNYNPSLKSRVTISVDTSENKFSLKLSSVTAADTAVYYCARDYGGLDYWGQGTLVTVSS |
| | | | SEQ ID NO: 26072 | SEQ ID NO: 30078 |

FIGURE 50

| | | | | |
|---|---|---|---|---|
| **iPS:435195** | **21-225_94D3** | NA | GAAATTGTGTTGACGCAGTCTCCAGGCACCCTGTCTTTGTCTCAAGGGGAAAGAGCCACCCTCTCCTGCAGGGCCAGTCAGAGTGTTAGCAGCAGGTACTTAGCCTGGTACCAGCAGAAACCTGGCCAGGCTCCCAGACTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTATATTACTGTCAGCATTATGATAGCTCACCGTGGACGTTCGGCCAAGGGACCAAGGTGGAAAATCAA | CAGGTGCAGCTACAGCAGGGGGGCGCAGGACCGTTGAAGCCTTCGGAGACCCTGTCCCTCACCTGCGCTGTCTATGGTGGGTCCTTCAGTGGTTGCTACTGGAGCTGGATCCGCCAGCCCCCAGGGAAGGGGCTGGAGTGGATTGGGGAAATCAATTATAGTGGAAGAACCAACTACAACCCGTCCCTCAAGAGTCGAGTCACCATATCAGTAGACACGTCCAAGAACCAGTTCTCCCTGAAGCTGAGCTCTGTGACCGCCGCGGACACGGCTGTGTATTACTGTGCGAGAGACTACGGCGGTATGGACGTCTGGGGCCAAGGGACCACGGTCACCGTCTCCTCA |
| | | | SEQ ID NO: 26073 | SEQ ID NO: 30079 |
| | | AA | EIVLTQSPGTLSLSQGERATLSCRASQSVSSRYLAWYQQKPGQAPRLLIYGASSRATGIPDRFSGSGSGTDFTLTISRLEPEDFAVYYCQHYDSSPWTFGQGTKVENQ | QVQLQQGGAGPLKPSETLSLTCAVYGGSFSGCYWSWIRQPPGKGLEWIGEINYSGRTNYNPSLKSRVTISVDTSKNQFSLKLSSVTAADTAVYYCARDYGGMDVWGQGTTVTVSS |
| | | | SEQ ID NO: 26074 | SEQ ID NO: 30080 |
| **iPS:435197** | **21-225_94F3** | NA | GACATCCAGATGACCCAGTCTCCATCCTCCCTGTCTGCATCTGTAGGAGACAGAGTCACCATCACTTGCCGGGCAAGTCAGGCCATTAGAGATGATTTAGGCTGGTATCAGCAGAAACCAGGGAAAGCCCCTCAGCGCCTGATCTTTGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTCCAGCATTATAGTTACCCTCGGACGTTCGGCCGAGGGACCAAGGTGGCAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACGATATCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATTAACAGCCTGAGAGCCGAGGACACGGCTGTGTATTTCTGTGCGAGAGAAAAATATAGCAGTGGCTGGTACGACTACGGTATGGACGTCTGGGGCCAAGGGACCACGGTCACCGTCTCCTCA |
| | | | SEQ ID NO: 26075 | SEQ ID NO: 30081 |

FIGURE 50

| | | | | |
|---|---|---|---|---|
| | | AA | DIQMTQSPSSLSASVGDRVTITCRASQAIRDDLGWYQQKPGKAPQRLIFAASSLQSGVPSRFSGSGSGTEFTLTISSLQPEDFATYYCLQHYSYPRTFGRGTKVAIK | QVQLVESGGGVVQPGRSLRLSCAASGFTFSNDIMHWVRQAPGKGLEWVAVIWYDGSNKYYADSVKGRFTISRDNSKNTLYLQINSLRAEDTAVYFCAREKYSSGWYDYGMDVWGQGTTVTVSS |
| | | | SEQ ID NO: 26076 | SEQ ID NO: 30082 |
| **iPS:435203** | **21-225_75A7** | NA | GACATCGTGATGACCCAGTCTCCAGACTCCCTGGCTGTGTCTCTGGGCGAGAGGGCCACCATCAACTGCAAGTCCAGCCAGACTGTTTTACACAGCTCCAACAATTATAACTACTTAGCTTGGTACCAGCAGAAGCCAGGACAGCCCCCTAAGGTGCTCATTTACTGGACATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTTTAGTAGTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCCCCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGACAGAGTCACCATGACCAGGAACACCTCCATAAGCACAGCCTACATGGAGCTGAGCAGCCTGAGATCTGAGGACACGGCCGTGTATTATTGTGCGGTTTCCAGTGGCTGGCACTGGTTCGACCCCTGGGGCCAGGGAACCCTGGTCACCGTCGCCTCA |
| | | | SEQ ID NO: 26077 | SEQ ID NO: 30083 |
| | | AA | DIVMTQSPDSLAVSLGERATINCKSSQTVLHSSNNYNYLAWYQQKPGQPPKVLIYWTSTRESGVPDRFSGSGSGTDFTLTISSLQAEDVAVYYCQQYFSSPPTFGQGTKVEIK | QVQLVQSGAEVKKPGASVKVSCKASGYTFPNYDINWVRQATGQGLEWMGWMNPNSGNTGYAQKFQDRVTMTRNTSISTAYMELSSLRSEDTAVYYCAVSSGWHWFDPWGQGTLVTVAS |
| | | | SEQ ID NO: 26078 | SEQ ID NO: 30084 |

FIGURE 50

| | | | | |
|---|---|---|---|---|
| **iPS:435209** | **21-225_75A10** | NA | AATATCGTGATGACCCAGTCTCCAGACTCCCTGGCTGTGTCTCTGGGCGCGAGGGCCACCATCAACTGCAAGTCCAGCCAGAGTGTTTTACACAACTCCAACAATTACAACTACTTAACTTGGTACCAGCAGAAACCAGGACAGCCTCCTAAGCTGCTCCTTTACTGGGCATCTACCCGGAAATCCGGGGTCCCTGACCGATTCAGTGGCAGCGGGTCTGGGACAGATTTCACTCTCACCATCAGCAGCCTGCAGGCTGAAGATGTGGCAGTTTATTTCTGTCAGCAATATTATAGTTCTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGTATAGCAGTGGCTGGTACTACTTTGACTACTGGGGCCAGGGAACCCTGGTCACCGTCTCCTCA |
| | | | SEQ ID NO: 26079 | SEQ ID NO: 30085 |
| | | AA | NIVMTQSPDSLAVSLGARATINCKSSQSVLHNSNNYNYLTWYQQKPGQPPKLLLYWASTRKSGVPDRFSGSGSGTDFTLTISSLQAEDVAVYFCQQYYSSPPTFGQGTKVEIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYYCAYSSGWYYFDYWGQGTLVTVSS |
| | | | SEQ ID NO: 26080 | SEQ ID NO: 30086 |
| **iPS:435211** | **21-225_94E11** | NA | GACATCGTTATGACCCAGTCTCCAGACTCCCTGGCTGTGTCTCTGGGCGAGAGGGCCACCATCAATTGCAAGTCCAGCCAGAGTGTTTTATTCAGCTCCAACAATTATAATTACTTAGCTTGGTACCAGCAGAGACCAGGACAGCCTCCTAACCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGCTGATGTGGCAGTTTATTACTGTCAGCAATATCATAGTTCTCCTCTGACGTTCGGCCAAGGGACCACGGTGCAAATCAAA | CAGGTGCAGTTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCACAAGCACAGCCCACATGGAGCTGAGCAGCCTGAGATCTGAGGACACGGCCGTGTATTACTGTGCGGTCTCCAGTGGCTGGAAGTGGTTCGACCCCTGGGGCCAGGGAACCCTGGTCACCGTCTCCTCA |
| | | | SEQ ID NO: 26081 | SEQ ID NO: 30087 |

FIGURE 50

| | | | | |
|---|---|---|---|---|
| | | AA | DIVMTQSPDSLAVSLGERATINCKSSQSVLFSSNNYNYLAWYQQRPGQPPNLLIYWASTRESGVPDRFSGSGSGTDFTLTISSLQAADVAVYYCQQYHSSPLTFGQGTTVQIK | QVQLVQSGAEVKKPGASVKVSCKASGYTFTNYDINWVRQATGQGLEWMGWMNPNSGNTGYAQKFQGRVTMTRNTSTSTAHMELSSLRSEDTAVYYCAVSSGWKWFDPWGQGTLVTVSS |
| | | | SEQ ID NO: 26082 | SEQ ID NO: 30088 |
| **iPS:435215** | **21-225_94E12** | NA | GACATCGTGATGACCCAGTCTCCAGACTCCCTGGCTGTGTCTCTGGGCGAGAGGGCCACCACCAATTGTAAGTCCAGCCAGAGTGTTTTACACAGGTCCAACAATTACAACTACTTAGCGTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTGACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAACTGAGCAGCCTGAGATCTGAGGACACGGCCGTGTATTACCGTGGGAGTACCAGTGGCTGGAAGTTCTTTGACTACTGGGGCCAGGGAACCCTGGTCACCGTCTCCTCA |
| | | | SEQ ID NO: 26083 | SEQ ID NO: 30089 |
| | | AA | DIVMTQSPDSLAVSLGERATTNCKSSQSVLHRSNNYNYLAWYQQKPGQPPKLLIYWASTRESGVPDRFSGSGSGTDFTLTISSLQAEDVAVYYCQQYYSTPPTFGQGTKVEIK | QVQLVQSGAEVKKPGASVKVSCKASGYTLTNYDINWVRQATGQGLEWMGWMNPNSGNTGYAQKFQGRVTMTRNTSISTAYMELSSLRSEDTAVYYRGSTSGWKFFDYWGQGTLVTVSS |
| | | | SEQ ID NO: 26084 | SEQ ID NO: 30090 |

FIGURE 50

| | | | | |
|---|---|---|---|---|
| **iPS:435217** | **21-225_94F12** | NA | GAAATTGTGTTGACGCAGTCTCCAGGCACCCTGTATTTGTCTCCAGGGGAAAGAGCCACCTTCTCATGCAGGGCCAGTCAGAGTGTTTACAGCAGCTACTTAGCGTGGTACCAGCAGAAACCTGGCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGACTGGAGCCTGAAGATTTTGCAGTGTATTACTGTCAGCATTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26085 | SEQ ID NO: 30091 |
| | | AA | EIVLTQSPGTLYLSPGERATFSCRASQSVYSSYLAWYQQKPGQAPRLLIYGASSRSTGIPDRFSGSGSGTDFALTISRLEPEDFAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26086 | SEQ ID NO: 30092 |
| **iPS:435219** | **21-225_95D2** | NA | GAAATTGTGTTGACGCAGTCTCCAGGCACCCTGTCTTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATCTATGGTGCATCCAGCCGGGCCACTGGCATCCCAGACAGGTTCAGTGGCAGTGGGTCTGGGACAGACTTCGCTCTCACCATCAGCAGACTGGAGCCTGAAGATTGTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26087 | SEQ ID NO: 30093 |

FIGURE 50

| | | | | |
|---|---|---|---|---|
| | | AA | EIVLTQSPGTLSLSPGERATLSCRASQSVYSSYLAWYQQKPGQAPRLLIYGASSRATGIPDRFSGSGSGTDFALTISRLEPEDCAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26088 | SEQ ID NO: 30094 |
| iPS:435221 | 21-225_95G2 | NA | GAAATAGTGATGACGCAGTCTCCAGCCACCCTGTCTCTGTCTCCAGGGGAAAGAGCCACCCTCTCCTGCAGGGCCAGTATGAGTGTTGTCAACAGCTTAGCCTGGTACCAGCAGAAACCTGGCCAGGCTCCCAGGCTCCTCATCTATGGTGCATCCACCAGGGCCACTGGTATCCCAGCCAGGTTCAGTGGCAGTGGGTCTGGGACAGAGTTCACTCTCACCATCAGCAGCCTGCAGTCTGAAGATTTTGCAATTTATTACTGTCAGCAGTATAATGACTGGCCGTGCAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTACAGCGTCTGGATTCACCTTCAGTAGCTATGGCATGCACTGGGTCCGCCAGGCTCCAGGCAAGGGGCTGGAGTGGGTGGCAGTTATTTGGTATGATGGAAGTAATAAATACTATGCAGACTCCGTGAAGGGCCGATTCACCATCTCCAGAGACAATTCCAAGAACACGCTGTTTCTGCAAATGAACAGCCTGAGAGCCGAGGACACGGCTGTGTATTACTGTGCGAGCGATCGCAATATAGTGGGAGCTACTTACTTTGAGTCCTGGGGCCAGGGAACCCTGGTCACCGTCTCCTCA |
| | | | SEQ ID NO: 26089 | SEQ ID NO: 30095 |
| | | AA | EIVMTQSPATLSLSPGERATLSCRASMSVVNSLAWYQQKPGQAPRLLIYGASTRATGIPARFSGSGSGTEFTLTISSLQSEDFAIYYCQQYNDWPCSFGQGTKLEIK | QVQLVESGGGVVQPGRSLRLSCTASGFTFSSYGMHWVRQAPGKGLEWVAVIWYDGSNKYYADSVKGRFTISRDNSKNTLFLQMNSLRAEDTAVYYCASDRNIVGATYFESWGQGTLVTVSS |
| | | | SEQ ID NO: 26090 | SEQ ID NO: 30096 |

FIGURE 50

| | | | | |
|---|---|---|---|---|
| **iPS:435227** | **21-225_95G4** | NA | GACATCGTTATGACCCAGTGTCCAGACTCCCTGGCTGTGTCTCTGGGCGAGAGGGCCACCATCAATTGCAAGTCCAGCCAGAGTGTTTTATTCAGATCCAACAATTATAATTACTTAGCTTGGTACCAGCAGAGACCAGGACAGCCTCATAACCTGCTCATTTACTGGGCATCTACCCGGGAATCCGGGGTCCCTGACCGATTCAGTGGCAGCGGGTATGGGACAGATTTCACTCTCACCATCAGCAGCGTGCAGGCTGCTGATGTGGCAGTTTATTACTGTCAGCAATATCATAGTTCTCCTCTGACGTTCGGCCAAGGGACCACGGTGCAAATCAAA | CAGGTGCAGTTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCACAAGCACAGCCCACATGGAGCTGAGCAGCCTGAGATCTGAGGACACGGCCGTGTATTACTGTGCGGTCTCCAGTGGCTGGAACTGGTTCGACCCCTGGGGCCAGGGAACCCTGGTCACCGTCTCCTCA |
| | | | SEQ ID NO: 26091 | SEQ ID NO: 30097 |
| | | AA | DIVMTQCPDSLAVSLGERATINCKSSQSVLFRSNNYNYLAWYQQRPGQPHNLLIYWASTRESGVPDRFSGSGYGTDFTLTISSVQAADVAVYYCQQYHSSPLTFGQGTTVQIK | QVQLVQSGAEVKKPGASVKVSCKASGYTFTNYDINWVRQATGQGLEWMGWMNPNSGNTGYAQKFQGRVTMTRNTSTSTAHMELSSLRSEDTAVYYCAVSSGWNWFDPWGQGTLVTVSS |
| | | | SEQ ID NO: 26092 | SEQ ID NO: 30098 |
| **iPS:435235** | **21-225_95F9** | NA | GAAATTGTGTTGACGCAGTCTCCAGGCACCCTGTATTTGTCTCCAGGGGAAAGAGCCACCCTCTCCTGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTGTGGGACAGACTTCGCTCTCACCATCAGCAGACTGGAGCCTGAAGATTTTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26093 | SEQ ID NO: 30099 |

FIGURE 50

| | | | | |
|---|---|---|---|---|
| | | AA | EIVLTQSPGTLYLSPGERATLSCRASQSVYSSYLAWYQQKPGQAPRLLIYGASSRSTGIPDRFSGSGCGTDFALTISRLEPEDFAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26094 | SEQ ID NO: 30100 |
| **iPS:435237** | **21-225_95G9** | NA | GAAATTGTGTTGACGCAGTCTCCAGGCACCCTGTATTTGTCTTCAGGGGAAAGAGCCACCCTCTCATGCAGGGCCAGTCAGAGTGTTTACAGCAGCTACTTAGCGTGGTACCAGCAGAAACCTGGCCAGGCTCCCAGGCTCCTCATTTATGGTGCATCCAGCCGGTCCACTGGCATCCCAGACAGGTTCAGTGGCAGTGGGTCTGGGACAGACTTCGCTCTCACCATCAGCAGAGTGGAGCATGAAGATTGTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26095 | SEQ ID NO: 30101 |
| | | AA | EIVLTQSPGTLYLSSGERATLSCRASQSVYSSYLAWYQQKPGQAPRLLIYGASSRSTGIPDRFSGSGSGTDFALTISRVEHEDCAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26096 | SEQ ID NO: 30102 |

FIGURE 50

| | | | | |
|---|---|---|---|---|
| iPS:435239 | 21-225_95H10 | NA | GAAATTGTGTTGTCGCAGTCTCCAGGCATCCTGTATTTGTCTTCAGGGGAAAGAGCCACCCTCTCCTGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGGCCACTGGCATCCCAGACAGGTTCAGTGGCAGTGGGTCTGGGACAGACTTCGCTCTCACCATCAGCAGACTGGAGCCTGAAGATTTTGCAGTGTATTACTGTCAGCACTATGATAACTCTCCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26097 | SEQ ID NO: 30103 |
| | | AA | EIVLSQSPGILYLSSGERATLSCRASQSVYSSYLAWYQQKPGQAPRLLIYGASSRATGIPDRFSGSGSGTDFALTISRLEPEDFAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26098 | SEQ ID NO: 30104 |
| iPS:435245 | 21-225_95E12 | NA | GACATCGTGATGACCCAGTCTCCAGACTCCCTGGCTGTGTCTCTGGGCGAGAGGGCCACCATCAACTGCAAGTCCAGCCAGAGTGTTTTATACAGCTCCAACAATGCGAACTACTTAGCTTGGTACCAGCAGAAACCAGGACAGCCTCCTAACCTGTTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCGTGCAGTTTTGGCCAGGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTCA |
| | | | SEQ ID NO: 26099 | SEQ ID NO: 30105 |

FIGURE 50

| | | | | |
|---|---|---|---|---|
| | | AA | DIVMTQSPDSLAVSLGERATINCKSSQSVLYSSNNANYLAWYQQKPGQPPNLFIYWASTRESGVPDRFSGSGSGTDFTLTISSLQAEDVAVYYCQQYYSTPCSFGQGTKVEIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYYCAISSGWYWFDPWGQGTLVTVSS |
| | | | SEQ ID NO: 26100 | SEQ ID NO: 30106 |
| **iPS:435247** | **21-225_96G1** | NA | GAAATTGTGTTGACGCAGTCTCCAGGCACCCTGTCTTTGTCTACAGGGGAAAGAGCCACCCTCTCGTGCAGGGCCAGTCAGAGCGTTAGCAGCAGCTACTTAGCTTGGTACCAGCAGAAACCTGGCCAGCCTCCCAGGCTCCTCATTTATGGTGCATCCACCAGGGCCTCTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTAACTCACCGCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAATGGTAACACAGGCTATGCACAGAAGTTCCAGGGCAGAGTCACCATGACCAGGAACACCTCCATAAGCACAGCCTACATGGAGCTGAACAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTTA |
| | | | SEQ ID NO: 26101 | SEQ ID NO: 30107 |
| | | AA | EIVLTQSPGTLSLSTGERATLSCRASQSVSSSYLAWYQQKPGQPPRLLIYGASTRASGIPDRFSGSGSGTDFTLTISRLEPEYFAVYYCQQYGNSPLTFGGGTKVEIK | QVQLVQSGAEVKKPGASVKVSCKASGYTFTNYDINWVRQATGQGLEWMGWMHPNNGNTGYAQKFQGRVTMTRNTSISTAYMELNSLRSEDTAVYYCAISSGWYWFDPWGQGTLVTVSL |
| | | | SEQ ID NO: 26102 | SEQ ID NO: 30108 |

FIGURE 50

| | | | | |
|---|---|---|---|---|
| iPS:435249 | 21-225_96E2 | NA | GACAGCGTGATGACCCAGTCTCCAGACTCCCTGGCTGTGTCTCTGGGCGAGAGGGCCACCATCAACTGCAAGTCCAGCCAGAGTGTTTTACACAGCTCTAATAAAAAGAACTACTTAGCTTGGTACCAGCAGAAACCAGGACAGCCTCCTAAGCTGCTCATTTACTGGGCATCTACCTGGGAATCCGGGGTCCCTGACCGATTCAGTGGCAGCGGGTCTGGGACAGATTTCACTCTCACCATCAGCAGTCTGCAGGCTGAAGATGTGGCAGTTTATTACTGTCAGCAATATTATAGGACTCCGTGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAATAGTGGTAACACAGGCTATGCACAGAAGTTCCAGGGCAGAGTCACCCTGACCAGGAACACCTCCATAAGCACAGCCTACATGGAGCTGAGCAGCCTGAGATCTGAGGACACGGCCGTGTATTACTGTGCGGTTTCCAGTGGCTGGTACTGGTTCGACCCCTGGGGCCAGGGAACCCTGGTCACCGTCTCCTCA |
| | | | SEQ ID NO: 26103 | SEQ ID NO: 30109 |
| | | AA | DSVMTQSPDSLAVSLGERATINCKSSQSVLHSSNKKNYLAWYQQKPGQPPKLLIYWASTWESGVPDRFSGSGSGTDFTLTISSLQAEDVAVYYCQQYYRTPWTFGQGTKVEIK | QVQLVQSGAEVKKPGASVKVSCKASGYTFTNYDINWVRQATGQGLEWMGWMHPNSGNTGYAQKFQGRVTLTRNTSISTAYMELSSLRSEDTAVYYCAVSSGWYWFDPWGQGTLVTVSS |
| | | | SEQ ID NO: 26104 | SEQ ID NO: 30110 |
| iPS:435251 | 21-225_96A3 | NA | GACATCCAGATGACCCAGTCTCCATCCTCCCTGTCTGCTTCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCACTTTGCAAAGTGGGGTCCCATCAAGGTTCAGCGGCAGTGGATCTGGGACAGAATTCACTCTCACAATCAGCGGCCTGCAGCGTGAAGATTTTGCAACTTATCACTGTCTACAGCATAGTAATTACCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCATCTGTACTGTCTCTGGTGGCTCCATCAGCAGTAGTAATTACTACTGGGGCTGGATCCGCCAGCCCCCAGGGAAGGGGCTGGAGTGGATTGGGAGTATCTATTATAGTGGATACACCTCCTACAACCCGTCCCTCAAGAGTCGAGTCACCATATCCGTAGACTCGTCCAAGAACCACTTCTCCCTGAGGCTGAGCTCTGTGACCGCCGCAGACACGGCTGTGTATTACTGTGCGAGACTTGACTCTAACTGGGGTCTTGACTACTGGGGCCAGGGAACCCTGGTCACCGTCTCCTCA |
| | | | SEQ ID NO: 26105 | SEQ ID NO: 30111 |

FIGURE 50

| | | | | |
|---|---|---|---|---|
| | | AA | DIQMTQSPSSLSASVGDRVTITCRASQGIRNDLGWYQQKPGKAPKRLIYAASTLQSGVPSRFSGSGSGTEFTLTISGLQREDFATYHCLQHSNYPLTFGGGTKVEIK | QLQLQESGPGLVKPSETLSLICTVSGGSISSSNYYWGWIRQPPGKGLEWIGSIYYSGYTSYNPSLKSRVTISVDSSKNHFSLRLSSVTAADTAVYYCARLDSNWGLDYWGQGTLVTVSS |
| | | | SEQ ID NO: 26106 | SEQ ID NO: 30112 |
| iPS:435253 | 21-225_96A4 | NA | GACATCCAGATGACCCAGTCTCCATCCTCCCTGTCTGCATCTGTAGGAGACAGAGTCACCATCACTTGCCGGGCAAGTCAGGACATTAGAAATGATTTAGGCTGGTATCAGCAGAAACCAGGGAAAGCCCCTAAGCGCCTGATCTATGGTGTATCCAGTTTGCAAAGTGGGGTCCCATCAAGGTTCAGCGGCAGTGGATCTGGGACCGAATTCACTCTCACAATCAGCAGCCTGCAGCGTGAAGATTTTGCAACTTATTACTGTCTACAGCATAATGATTACCCATTCACTTTCGGCCGTGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAGTTATGATATCAACTGGGTGCGACAGGCCACTGGACACGGGCTTGAGTGGATGGGATGGATGAACCCTAACAGTGGTAACACAGGCTATGCACAGAAGTTCCAGGGCAGAGTCACCATGACCTGGAACACCTCCAAAAGCACAGCCTACATGGAGCTGAGTAGCCTGAGATCTGAGGACACGGCCGTGTATTACTGTGCGAGAGGCTTTTACGATACTTTGACTGGTTCCGGCTACTACTACGTTATGGACGTCTGGGGCCAAGGGACCACGGTCACCGTCTCCTCA |
| | | | SEQ ID NO: 26107 | SEQ ID NO: 30113 |
| | | AA | DIQMTQSPSSLSASVGDRVTITCRASQDIRNDLGWYQQKPGKAPKRLIYGVSSLQSGVPSRFSGSGSGTEFTLTISSLQREDFATYYCLQHNDYPFTFGRGTKVDIK | QVQLVQSGAEVKKPGASVKVSCKASGYTFTSYDINWVRQATGHGLEWMGWMNPNSGNTGYAQKFQGRVTMTWNTSKSTAYMELSSLRSEDTAVYYCARGFYDTLTGSGYYYVMDVWGQGTTVTVSS |
| | | | SEQ ID NO: 26108 | SEQ ID NO: 30114 |

FIGURE 50

| | | | | |
|---|---|---|---|---|
| **iPS:435255** | **21-225_96D5** | NA | GACATCGTGATGACCCAGTCTCCAGACTCCCT<br>GGCTGTGTCTCTGGGCGAGAGGGCCACCATCA<br>ACTGCAAGTCCAGCCAGAGTGTTTTGCACAGC<br>TCCAACAATTATAATTACTTAGCTTGGTACCA<br>GCAGAAACCAGGACAGCCTCCTAACCTGCTCA<br>TTTACTGGGCATCTACCCGGGAATCCGGGGTC<br>CCTGACCGATTCAGTGGCAGCGGGTCTGGGAC<br>AGATTTCACTCTCACCATCAGCAGCCTGCAGG<br>CTGCTGATGTGGCAGTTTATTACTGTCAGCAA<br>TATTATAGTAGTCCACCGACGTTCGGCCAAGG<br>GACCACGGTGGAAATCAAA | CAGGTGCAGCTGGTGCAGTCTGGGGCTGAGGTG<br>AAGAAGCCTGGGGCCTCAGTGAAGGTCTCCTGC<br>AAGGCTTCTGGATACACCTTCACCAATTATGATA<br>TCAACTGGGTGCGACAGGCCACTGGACAAGGGC<br>TTGAGTGGATGGGATGGATGAACCCTAACAGTG<br>GTAACACAGGCTATGCACAGAAGTTCCAGGGCA<br>GAGTCACCATGACCAGGAGCACCTCCACAAGCA<br>CAGCCCACATGGAGCTGAGCAGCCTGAGATCTG<br>AGGACACGGCCGTGTATTACTGTGCGGTTTCCAG<br>TGGCTGGTCCTGGTTCGACCCCTGGGGCCAGGGA<br>ACCCTGGTCACCGTCTCCTCA |
| | | | SEQ ID NO: 26109 | SEQ ID NO: 30115 |
| | | AA | DIVMTQSPDSLAVSLGERATINCKSSQSVLHSSN<br>NYNYLAWYQQKPGQPPNLLIYWASTRESGVPD<br>RFSGSGSGTDFTLTISSLQAADVAVYYCQQYYSS<br>PPTFGQGTTVEIK | QVQLVQSGAEVKKPGASVKVSCKASGYTFTNYDIN<br>WVRQATGQGLEWMGWMNPNSGNTGYAQKFQGR<br>VTMTRSTSTSTAHMELSSLRSEDTAVYYCAVSSGW<br>SWFDPWGQGTLVTVSS |
| | | | SEQ ID NO: 26110 | SEQ ID NO: 30116 |
| **iPS:435257** | **21-225_96H5** | NA | GACATCGTGATGACCCAGTCTCCAGACTCCCT<br>GGCTGTGTCTCTGGGCGAGAGGGCCACCATCA<br>ACTGCAAGTCCAGCCAGAGTGTTTTGTACAGC<br>TCCAACAGTCACAACTACTTAGCTTGGTACCA<br>GCAGAAACCAGGACAGCCTCCTAAACTGCTCA<br>TTTACTGGGCATCTATCCGGGAATCCGGGGTC<br>CCTGACCGATTCAGTGGCAGCGGGTCTGGGAC<br>AGATTTCACTCTCTCCATCAGCAGCATGCAGG<br>CTGAAGATGTGGCAGTTTATTACTGTCAGCAA<br>TATTATAGTACTCCGTGCAGTTTTGGCCAGGG<br>GACCAAGGTGGAGATCAAA | CAGGTGCAGCTGGTGCAGTCTGGGGCTGAGGTG<br>AAGAAGCCTGGGGCCTCAGTGAAGGTCTCCTGC<br>AAGGCTTCTGGATACACCTTCACCAATTATGATA<br>TCAACTGGGTGCGACAGGCCTCTGGACAAGGAC<br>TTGAGTGGATGGGATGGATGCACCCTAACAGTGG<br>TAACACAGGCTATGCACAGAAGTTCCAGGGCAG<br>AGTCACCATGACCAGGAACACCTCCATAAGCAC<br>AGCCTACATGGAGCTGAGCAGCCTGAGATCTGA<br>GGACTCGGCCGTATATTACTGTGCGTATAGTAGT<br>GGCTGGTACAAATTTGACTACTGGAGCCAGGGA<br>ACCCTGGTCACCGTCTCCTCA |
| | | | SEQ ID NO: 26111 | SEQ ID NO: 30117 |

FIGURE 50

| | | | | |
|---|---|---|---|---|
| | | AA | DIVMTQSPDSLAVSLGERATINCKSSQSVLYSSN SHNYLAWYQQKPGQPPKLLIYWASIRESGVPDR FSGSGSGTDFTLSISSMQAEDVAVYYCQQYYSTP CSFGQGTKVEIK | QVQLVQSGAEVKKPGASVKVSCKASGYTFTNYDIN WVRQASGQGLEWMGWMHPNSGNTGYAQKFQGR VTMTRNTSISTAYMELSSLRSEDSAVYYCAYSSGW YKFDYWSQGTLVTVSS |
| | | | SEQ ID NO: 26112 | SEQ ID NO: 30118 |
| iPS:435259 | 21-225_96C6 | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTATGCTGCTTCCAGTTT GCAAAGTGGGGTCCCCTCAAAGTTCAGCGGCA GTGGATCTGGGACAGATTTCACTCTCACCATC AACAGCCTGCAGCCTGAAGATTTTGCAACTTA TTACTGCCACCAGTATAATGATTACCCATTCA CTTTCGGCCCTGGGACCAAAGTGGATATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAGTTATGATA TCAACTGGGTGCGTCAGGCCACTGGACAAGGGC TTGAGTGGATGGGATGGATGAACCCTAACAGTCG TAACACAGGCTATGCACAGAAGTTCCAGGGCAG AGTCACCATGACCTGGAACACCTCCATAAGCACA GCCTACATGGAGCTGAGCAGCCTGAGATCTGAG GACACGGCCGTGTATTACTGTGCGAGAGGCGGCT ACGATGTTTTGCCTGGGAATAACTACTACTACGA TATGGACGTCTGGGGCCAAGGGACCACGGTCAC CGTCTCCTCA |
| | | | SEQ ID NO: 26113 | SEQ ID NO: 30119 |
| | | AA | DIQMTQSPSSLSASVGDRVTITCRASQGISNYLA WFQQKPGKAPKSLIYAASSLQSGVPSKFSGSGSG TDFTLTINSLQPEDFATYYCHQYNDYPFTFGPGT KVDIK | QVQLVQSGAEVKKPGASVKVSCKASGYTFTSYDIN WVRQATGQGLEWMGWMNPNSRNTGYAQKFQGR VTMTWNTSISTAYMELSSLRSEDTAVYYCARGGY DVLPGNNYYYDMDVWGQGTTVTVSS |
| | | | SEQ ID NO: 26114 | SEQ ID NO: 30120 |

FIGURE 50

| | | | | |
|---|---|---|---|---|
| **iPS:435267** | **21-225_96D10** | NA | GACATCGTGATGACCCAGTCTCCAGACTCCCTGGCTGTGTCTCTGGGCGAGAGGGCCACCATCAATTGCAAGTCCAGTCAGAATATTTTATCCAGCTCCAACAATAAGAACTACTTAACTTGGTACCAGCAGAAACCAGGACAGCCTCCTAAAATACTCATTTACTGGACATCTACCCGGGAATCCGGGGTCCCTGACCGATTCAGTGGCAGCGGGTTTGGGACAGATTTCACTCTCACCATCAGCAGCCTGCAGGCTGAAGATGTGGCTGTTTATTACTGTCAGCAATATTTTAGTGTTCCTCCGACGTTCGGCCAAGGGACCAAGGTGGAAATCAAA | CAGGTGCAGCTGGTGCAGTCTGGGGCTGAGGTGAAGAAACCTGGGGCCTCAGTGAGGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATGAGCACAGCCTACATGGAGCTGAGCAGCCTGAGATCTGAGGACACGGCCGTGTATTACTGTGCCCATAGCAGTGGCTGGTACTTTTTTGACTACTGGGGCCAGGGAACCCTGGTCACCGTCTCCTCA |
| | | | SEQ ID NO: 26115 | SEQ ID NO: 30121 |
| | | AA | DIVMTQSPDSLAVSLGERATINCKSSQNILSSSNNKNYLTWYQQKPGQPPKILIYWTSTRESGVPDRFSGSGFGTDFTLTISSLQAEDVAVYYCQQYFSVPPTFGQGTKVEIK | QVQLVQSGAEVKKPGASVRVSCKASGYTFTNYDINWVRQATGQGLEWMGWMHPNSGNTGYAQKFQGRVTMTRNTSMSTAYMELSSLRSEDTAVYYCAHSSGWYFFDYWGQGTLVTVSS |
| | | | SEQ ID NO: 26116 | SEQ ID NO: 30122 |
| **iPS:435273** | **21-225_97A2** | NA | GAAATTGTGTTGACGCAGTCTCCAGGCACCCTGTATTTGTCTTCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTGTGGGACAGACTTCGCTCTCACCATCAGCAGACTGGAGCCTGAAGATTGTGCAGTGTATTACTGTCAGCATTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26117 | SEQ ID NO: 30123 |

FIGURE 50

| | | | | |
|---|---|---|---|---|
| | | AA | EIVLTQSPGTLYLSSGERATLSCRASQSVYSSYLAWYQQKPGQAPRLLIYGASSRSTGIPDRFSGSGCGTDFALTISRLEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26118 | SEQ ID NO: 30124 |
| iPS:435279 | 21-225_97H4 | NA | GACATCGTGATGACCCAGTCTCCAGATTGCCTGGCTGTGTCTCTGGGCGAGAGGGCCACCATCAACTGCAAGTCCAGCCAGAGTGTTTTATACACCTCCAACAATAACAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTTGCAGTTTATTATTGTCAGCACTACAATGATACTCCATGGAAGTTTGTCCAAGGGACCAAGGTGGAAATCACA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTCA |
| | | | SEQ ID NO: 26119 | SEQ ID NO: 30125 |
| | | AA | DIVMTQSPDCLAVSLGERATINCKSSQSVLYTSNNNNYLAWYQQKPGQPPKLLIYWASTRESGVPDRFSGSGSGTDFTLTISSLQAEDVAVYYCQHYNDTPWKFVQGTKVEIT | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 26120 | SEQ ID NO: 30126 |

FIGURE 50

| | | | | |
|---|---|---|---|---|
| iPS:435281 | 21-225_97E5 | NA | GAAATTGTGTTGACGCAGTCTCCAGGCACCCTGTCTTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGGCCACTGGCATCCCAGACAGGTTCAGTGGCAGTGGGTGTGGGACAGACTTCGCTCTCACCATCAGCAGAGTGGAGCCTGAAGATTGTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 26121 | SEQ ID NO: 30127 |
| | | AA | EIVLTQSPGTLSLSPGERATLSCRASQSVYSSYLAWYQQKPGQAPRLLIYGASSRATGIPDRFSGSGCGTDFALTISRVEPEDCAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 26122 | SEQ ID NO: 30128 |
| iPS:435291 | 21-225_146E1 | NA | GACATCAAGATGACCCAGTCTCCATCTTCCGTGTCTGCATCTGTAGGAGACAGAGTCACCATCACTTGTCGGGCGAGTCAGGGTATTAACAACTGGTTAGTCTGGTATCAGCAGAAACCAGGGAAAGCCCCTAAGCTCCTGATCTATGCTGCATCCAGTTTGCAAAGTGGGGTCCCATCAAGGTTCCGCGGCAGTGGATCTGGGACAGATTTCACTCTCACCATCAGCAGCCTGCAGCCTGAAGATTTTGCAACTTACTATTGTCAACAGGCTAACAGTTTCCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGAAGCGTCTGGAATCACCTTCAGTAGCTATGGCATGCACTGGGTCCGCCAGGCTCCAGGCAAGGGGCTGGAGTGGGTGGCAATTATATGGTATGATGGAAGTAATAAATACTATGCAGACTCCGTGAAGGGCCGATTCACCATCTCCAGAGACAATTCCAAGAACACGCTGTTTCTGCAAATGAACAGCCTGAGAGCCGAGGACACGGCTGTGTATTACTGTGCGAGAGATCGTTTAGTGGGAGCTACCGCTGATGCTTTTGATATCTGGGGCCAAGGGACAATGGTCACCGTCTCTTCA |
| | | | SEQ ID NO: 26123 | SEQ ID NO: 30129 |

FIGURE 50

| | | | | |
|---|---|---|---|---|
| | | AA | DIKMTQSPSSVSASVGDRVTITCRASQGINNWLVWYQQKPGKAPKLLIYAASSLQSGVPSRFRGSGSGTDFTLTISSLQPEDFATYYCQQANSFPFTFGPGTKVDIK | QVQLVESGGGVVQPGRSLRLSCEASGITFSSYGMHWVRQAPGKGLEWVAIIWYDGSNKYYADSVKGRFTISRDNSKNTLFLQMNSLRAEDTAVYYCARDRLVGATADAFDIWGQGTMVTVSS |
| | | | SEQ ID NO: 26124 | SEQ ID NO: 30130 |
| **iPS:435293** | **21-225_146F1** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCGGTGGATCTGGGACAGAATTCACTCTCACAATCAGCAGCCTGCAGCCTGAAGATTTTGCAACTTATTACTGTCTTCAGCATAGTACTTACCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGCAGAAGTAGTTACTACTGGGGCTGGATCCGCCAGCCCCCAGGGAAGGGGCTGGAGTGGATTGGGAGTATATATTATAGTGGGAGTACCTCCTACAACCCGTCCCTCAAGAGTCGAGTCACCATATCCGTCGACACGTCCAAGAACCAGTTCTCCCTGAAGCTGAGCTCTGTGACCGCCGCAGACACGGCTGTTTATTACTGTGCGAGACTTGATCTCCTGTGGAGTTTTGACTACTGGGGCCAGGGAACCCTGGTCACCGTCTCCTCA |
| | | | SEQ ID NO: 26125 | SEQ ID NO: 30131 |
| | | AA | DIQMTQSPSSLSASVGDRVTITCRASQGIRNDLGWYQQKPGKAPKRLIYAASSLQSGVPSRFSGGGSGTEFTLTISSLQPEDFATYYCLQHSTYPLTFGGGTKVEIK | QLQLQESGPGLVKPSETLSLTCTVSGGSISRSSYYWGWIRQPPGKGLEWIGSIYYSGSTSYNPSLKSRVTISVDTSKNQFSLKLSSVTAADTAVYYCARLDLLWSFDYWGQGTLVTVSS |
| | | | SEQ ID NO: 26126 | SEQ ID NO: 30132 |

FIGURE 50

| | | | | |
|---|---|---|---|---|
| iPS:435295 | 21-225_146H1 | NA | GACATCCAGATGACCCAGTCTCCATCCTCCCTGTCTGCATCTGTAGGAGACAGAGTCACCATCACTTGCCGGGCAAGTCAGAGCATTAGCGACTATTTAAATTGGTATCAGCAAAAACCAGGGAAAGCCCCTAAGGTCCTGATCTATACTACATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACAGTACCCCCACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTCGTGGTGGTAACACATTCTACGCAGACTCCGTGAAGGGCCGGTTCACCATCTCCAGAGACAATTCCAAGAACACACTATATCTGCAAATGAACAGCCTGAGAGCCGAGGACACGGCCGTATATTACTGTGCGAAACGGGTGACGGACTACGGTGGTAACGACTGGTTCGACCCCTGGGGCCAGGGAACCCTGGTCACCGTCTCCTCA |
| | | | SEQ ID NO: 26127 | SEQ ID NO: 30133 |
| | | AA | DIQMTQSPSSLSASVGDRVTITCRASQSISDYLNWYQQKPGKAPKVLIYTTSSLQSGVPSRFSGSGSGTDFTLTISSLQPEDFATYYCQQSYSTPTFGGGTKVEIK | EVQLLESGGGLVQPGGSLRLSCAASGFTFSSYAMSWVRQAPGKGLEWVSAISGRGGNTFYADSVKGRFTISRDNSKNTLYLQMNSLRAEDTAVYYCAKRVTDYGGNDWFDPWGQGTLVTVSS |
| | | | SEQ ID NO: 26128 | SEQ ID NO: 30134 |
| iPS:435297 | 21-225_146B3 | NA | GATATTGTGATGACCCAGACTCCACTCTCTCTGTCCGTCACCCCTGGACAGCCGGCCTCCATCTCCTGCAAGTCTAGTCAGAGCCTCCTGCATGGTGATGGAAAGACCTATTTGTATTGGTACCTGCAGAAGCCAGGCCAGCCTCCACAGCTCCTGATCTATGAAGTTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGCTTTATCACTGCATGCAAAGTATACAGCTTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTTATAAATACTATGCAGACTCCGTGAAGGGCCGATTCACCATCTCCAGAGACAATTCCAAGAACACGCTGGATCTGCAAATGAACAGCCTGAGAGCCGAGGACACGGCTGTGTATTACTGTGTGAAAATGGGTATAGAAGTGGCTGTGGACTACTACTACGGTATGGACGTCTGGGGCCAAGGGACCACGGTCACCGTCTCCTCA |
| | | | SEQ ID NO: 26129 | SEQ ID NO: 30135 |

FIGURE 50

| | | | | |
|---|---|---|---|---|
| | | AA | DIVMTQTPLSLSVTPGQPASISCKSSQSLLHGDGKTYLYWYLQKPGQPPQLLIYEVSNRFSGVPDRFSGSGSGTDFTLKISRVEAEDVGLYHCMQSIQLPWTFGQGTKVEIK | QVQLVESGGGVVQPGRSLRLSCAASGFTFSSYGMHWVRQAPGKGLEWVAVIWYDGSYKYYADSVKGRFTISRDNSKNTLDLQMNSLRAEDTAVYYCVKMGIEVAVDYYYGMDVWGQGTTVTVSS |
| | | | SEQ ID NO: 26130 | SEQ ID NO: 30136 |
| **iPS:435299** | **21-225_146D4** | NA | GACATCGTGATGACCCAGTCTCCAGACTCCCTGGCTGTGTCTCTGGGCGAGAGGGCCACCATCAACTGCAAGTCCAGCCAGAGTGTTTTATACAGCTCCAACAATAACAACTACTTAGCTTGGTACCAGCAGAAACCAGGACAGCCTCCTAAGCTGGTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGGTGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGGGGAGCAGTGGCTGGTACTACTTTGACTACTGGGGCCAGGGAACCCTGGTCACCGTCTCCTCA |
| | | | SEQ ID NO: 26131 | SEQ ID NO: 30137 |
| | | AA | DIVMTQSPDSLAVSLGERATINCKSSQSVLYSSNNNNYLAWYQQKPGQPPKLVIYWASTRESGVPDRFSGSGSGTDFTLTISSLQAEDVAVYYCQQYYSTPCSFGQGTKLEIK | QVQLVQSGAEVKKPGASVKVSCKASGYTFTNYDINWVRQATGQGLEWMGWVHPNSGNTGYAQKFQGRVTMTRNTSISTAYMELSSLRSEDTAVYYCAGSSGWYYFDYWGQGTLVTVSS |
| | | | SEQ ID NO: 26132 | SEQ ID NO: 30138 |

FIGURE 50

| | | | | |
|---|---|---|---|---|
| **iPS:435301** | **21-225_146G4** | NA | GAAATTGTATTGACGCAGTCTCCAGGCACCCTGTCTTTATTTCCAGGGGAAAGAGCCACCCTCTCCTGCAGGGCCAGTCAGAATATTATCAGCAGCTATTTAGCCTGGTACCAGCAGAAACCTGGCCAGGCTCCCAGGCTCCTCATCTTTGGTGTATCTAGTCGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAACAATATGGTAGGTCACCATTCAATTTCGGCCCTGGGACCAAAGTGGATATCAAC | CAGGTGCAGCTGCAGGAGTCGGGCCCAGGACTGGTGAAGCCTTCACAGACCCTGTCCCTCAATTGCACTGTCTCTGGTGGCTCCATCAGCAACAGTGGTTACTACTGGAGCTGGATCCGCCAGCACCCAGGGAAGGGCCTGGAATGGATTGGGTACAGCTATTACAGTGGGAGCACCTACTACAACCCGTCCCTCAAGAGTCGAATTACCATATCAGTAGACACGTCTAACAACCAGTTCTCCCTGAAGCTGACCTCTGTGACTGCCGCGGACACGGCCGTGTATTACTGTGCGAGAGGGAAATATAACTGGAACCATGCTTTTGATATCTGGGGCCAAGGGACAATGGTCACCGTCTCTTCA |
| | | | SEQ ID NO: 26133 | SEQ ID NO: 30139 |
| | | AA | EIVLTQSPGTLSLFPGERATLSCRASQNIISSYLAWYQQKPGQAPRLLIFGVSSRATGIPDRFSGSGSGTDFTLTISRLEPEDFAVYYCQQYGRSPFNFGPGTKVDIN | QVQLQESGPGLVKPSQTLSLNCTVSGGSISNSGYYWSWIRQHPGKGLEWIGYSYYSGSTYYNPSLKSRITISVDTSNNQFSLKLTSVTAADTAVYYCARGKYNWNHAFDIWGQGTMVTVSS |
| | | | SEQ ID NO: 26134 | SEQ ID NO: 30140 |
| **iPS:435303** | **21-225_146A6** | NA | GACATCCAGATGACCCAGTCTCCAGCTTCCGTGTCTGCATCTGTGGGAGACAGAGTCACCATAACTTGTCGGGCGAGTCAGGGTATTAGCAACTGGTTAGCCTGGTATCAGCAGAAACCAGGGAAAGCCCCTAAACTCCTGATCTATGCTGCATCCAGTTTGCAAGGTGGGGTCCCATCAAGGTTCAGCGGCAGTGGATCTGGGACAGATTTCACTCTCTCCATCAGCAGCCTGCAGCCTGAAGATTTTGCAACTTACTATTGTCAACAGACTGACAGTTTCCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAACAGCTATGCCATGAACTGGGTCCGCCAGGCTCCAGGGAAGGGGCTGGAGTGGGTCTCAGCTATTAGTGGTAGTGGTGGTAACACATTCTACGCAGACTCCGTGAAGGGCCGCTTCACCATCTCCAGAGACAATTCCAAGAACACACTGTATCTGCAAATGAACAGCCTGAGAGCCGAGGACACGGCCGTATATTACTGTGCGAAAAAGGATAATGATTACGTTTGGGGGAGTCCTTACTTTGACTACTGGGGCCAGGGAACCCTGGTCACCGTCTCCTCA |
| | | | SEQ ID NO: 26135 | SEQ ID NO: 30141 |

FIGURE 50

| | | | | |
|---|---|---|---|---|
| | | AA | DIQMTQSPASVSASVGDRVTITCRASQGISNWLAWYQQKPGKAPKLLIYAASSLQGGVPSRFSGSGSGTDFTLSISSLQPEDFATYYCQQTDSFPFTFGPGTKVDIK | EVQLLESGGGLVQPGGSLRLSCAASGFTFNSYAMNWVRQAPGKGLEWVSAISGSGGNTFYADSVKGRFTISRDNSKNTLYLQMNSLRAEDTAVYYCAKKDNDYVWGSPYFDYWGQGTLVTVSS |
| | | | SEQ ID NO: 26136 | SEQ ID NO: 30142 |
| **iPS:435305** | **21-225_146C9** | NA | GACATCGTGATGACCCAGTCTCCAGACTCCCTGGCTGTGTCTCTGGGCGAGAGGGCCACCATCAACTGCAAGTCCAGCCAGAGTGTTTTACACAGCTCCAACAATTATAATTACTTAGCTTGGTACCAGCAGAAACCAGGACAGCCTCCTAAGGTGCTCATTTACTGGGCATCTACCCGGAAATCCGGGGTCCCTGACCGATTCAGTGGTAGCGGGTCTGGGACAGATTTCACTCTCACCATCAGCAGCCTGCAGGCTGAAGATGTGGCAGTTTATTATTGTCAGCAATATTATAGTACTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCTGGAACACCTCCATAAGCACAGCCTACATGGCCCTGAGCAGCCTGAGATCTGAGGACACGGCCGTGTATTACTGTGCGTATAGCAGTGGCTGGTACTCCTTTGACTACTGGGGCCAGGGAACCCTGGTCACCGTCTCCTCA |
| | | | SEQ ID NO: 26137 | SEQ ID NO: 30143 |
| | | AA | DIVMTQSPDSLAVSLGERATINCKSSQSVLHSSNNYNYLAWYQQKPGQPPKVLIYWASTRKSGVPDRFSGSGSGTDFTLTISSLQAEDVAVYYCQQYYSTPCSFGQGTKLEIK | QVQLVQSGAEVKKPGASVKVSCKASGYTFTNYDINWVRQATGQGLEWMGWMHPNSGNTGYAQKFQGRVTMTWNTSISTAYMALSSLRSEDTAVYYCAYSSGWYSFDYWGQGTLVTVSS |
| | | | SEQ ID NO: 26138 | SEQ ID NO: 30144 |

FIGURE 50

| | | | | |
|---|---|---|---|---|
| **iPS:435307** | **21-225_146E9** | NA | GACATCCAGATGACCCAGTCTCCATCCTCCCTGTCTGCATCTGTAGGAGACAGAGTCACCATCACTTGCCGGGCAAGTCAGAGCATTAGCGACTATTTAAATTGGTATCAGCTGAAACCAGGGAAAGCCCCTAAGGTCCTGATCTATACTACATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTTCTGTCAACAGAGTTACAGTACCCCCACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTCGTGGTGGTAACACATTCTACGCAGACTCCGTGAAGGGCCGGTTCACCATCTCCAGAGACAATTCCAAGAAAACACTATATCTGCAAATGAACAGCCTGAGAGCCGAGGACACGGCCGTATATTACTGTGCGAAACGGGTGACGGACTACGGTGGTAACGACTGGTTCGACCCCTGGGGCCAGGGAACCCTGGTCACCGTCTCCTCA |
| | | | SEQ ID NO: 26139 | SEQ ID NO: 30145 |
| | | AA | DIQMTQSPSSLSASVGDRVTITCRASQSISDYLNWYQLKPGKAPKVLIYTTSSLQSGVPSRFSGSGSGTDFTLTISSLQPEDFATYFCQQSYSTPTFGGGTKVEIK | EVQLLESGGGLVQPGGSLRLSCAASGFTFSSYAMSWVRQAPGKGLEWVSAISGRGGNTFYADSVKGRFTISRDNSKKTLYLQMNSLRAEDTAVYYCAKRVTDYGGNDWFDPWGQGTLVTVSS |
| | | | SEQ ID NO: 26140 | SEQ ID NO: 30146 |
| **iPS:435309** | **21-225_146F9** | NA | GACATCGTGATGACCCAGTCTCCAGACTCCCTGGCTGTGTCTCTGGGCGAGAGGGCCACCATCAACTGCAAGTCCAGCCAGAATATTTTACACAGCTCCAACAATAACAACTACTTAGCTTGGTACCAGCAGAAACCAGGACAGCCTCCTTACCTGCTCATTTACTGGGCATCTACCCGGGAATCCGGGGTCCCTGACCGATTCAGTGGCAGCGGGTCTGGGACAGATTTCACTCTCACCATCACCAGCCTGCAGGCTGAAGATGTGGCAGTTTATTACTGTCAGCAATATTATACTACTCCGTGCAGTTTTGGCCAGGGGACCAAGCTGGAGATCACT | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGAGTAGCAGTGGCTGGTACTTTTTTGACTACTGGGGCCAGGGAACCCTGGTCACCGTCTCCTCA |
| | | | SEQ ID NO: 26141 | SEQ ID NO: 30147 |

FIGURE 50

| | | | | |
|---|---|---|---|---|
| | | AA | DIVMTQSPDSLAVSLGERATINCKSSQNILHSSNNNNYLAWYQQKPGQPPYLLIYWASTRESGVPDRFSGSGSGTDFTLTITSLQAEDVAVYYCQQYYTTPCSFGQGTKLEIT | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCASSSGWYFFDYWGQGTLVTVSS |
| | | | SEQ ID NO: 26142 | SEQ ID NO: 30148 |
| iPS:435311 | 21-225_146H9 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCAGCTTCAGTAGCTATGGCATGCACTGGGTCCGCCAGGCTCCAGGCAAGGGGCTGGAGTGGGTGGCAGTGATATGGTTTGATGAAAGTAATAAACACTATGGAGACTCCGTGAAGGGCCGATTCACCATCTCCAGGGACAATTCCAAGAACACGCTGTATCTGCAAATGAACAGCCTGAGAGCCGAGGACACGGCTGTGTATTACTGTGCGAGAGAATTGGGATTTCTCTCTGACTATTGGGGCCAGGGAACCCTGGTCACCGTCTCCTCA |
| | | | SEQ ID NO: 26143 | SEQ ID NO: 30149 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVEIK | QVQLVESGGGVVQPGRSLRLSCAASGFSFSSYGMHWVRQAPGKGLEWVAVIWFDESNKHYGDSVKGRFTISRDNSKNTLYLQMNSLRAEDTAVYYCARELGFLSDYWGQGTLVTVSS |
| | | | SEQ ID NO: 26144 | SEQ ID NO: 30150 |

FIGURE 50

| | | | | |
|---|---|---|---|---|
| **iPS:435313** | **21-225_146G11** | NA | GACATCCAGATGACCCAGTCTCCATCCTCCCTGTCTGCATCTGTAGGAGACAGAGTCACCATCACTTGCCGGGCAAGTCAGGACATTAGAAATAATTTTGGCTGGTATCAGCAGAAACCAGGGAAAGCCCCTAAGCGCCTGATCTATGCTGCATCCAGTTTGCAAAGTGGGGTCCCATTAAGGTTCAGCGGCAGTGGATCTGGGACAGAATTCACTCTCACAATCAGCAGCCTGCAGCCTGAAGATTTTGCAACTTATTACTGTCTCCAACATGATAGTTACCCGCTCACTTTCGGCGGAGGGACCAAGGTGGAGATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTGGTAGCTACACATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGGTAGCAGCTCGTCCGGGTTTGACTACTGGGGCCAGGGAACCCTGGTCACCGTCTCCTCA |
| | | | SEQ ID NO: 26145 | SEQ ID NO: 30151 |
| | | AA | DIQMTQSPSSLSASVGDRVTITCRASQDIRNNFGWYQQKPGKAPKRLIYAASSLQSGVPLRFSGSGSGTEFTLTISSLQPEDFATYYCLQHDSYPLTFGGGTKVEIK | EVQLVESGGGLVKPGGSLRLSCAASGFTFSSYSMNWVRQAPGKGLEWVSSISGSGSYTYYADSVKGRFTISRDNAKNSLYLQMNSLRAEDTAVYYCARGSSSGFDYWGQGTLVTVSS |
| | | | SEQ ID NO: 26146 | SEQ ID NO: 30152 |
| **iPS:435315** | **21-225_147B2** | NA | GATATTGTGATGACCCAGACTCCCCTCTCTCTGTCCGTCACGCCTGGACAGCCGGCCTCCATCTCCTGCAAGTCTAGTCAGAGCCTCCTGCATAGTGAAGGAAAGACCTATTTGTATTGGTACCTGCAGAAGCCAGGCCAGCCTCCACAGCTCCTGATCTATGAAGTTTCCCACCGGGTCTCTGGAGTGCCAGATAGGTTCAGTGGCAGCGGGTCAGGGACAGATTTCACAGTGAAAATCAGCCGGGTGGAGGCTGAGGATGTTGGGGTTTATTACTGCATGCAAAGTACACAGTTTCCTCCCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGAGGTATAGCAGCAGCTGGTCGGGGGGTATGGACGTCTGGGGCCAAGGGACCACGGTCACCGTCTCCTCA |
| | | | SEQ ID NO: 26147 | SEQ ID NO: 30153 |

FIGURE 50

| | | | | |
|---|---|---|---|---|
| | | AA | DIVMTQTPLSLSVTPGQPASISCKSSQSLLHSEGKTYLYWYLQKPGQPPQLLIYEVSHRVSGVPDRFSGSGSGTDFTVKISRVEAEDVGVYYCMQSTQFPPTFGPGTKVDIK | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARRYSSSWSGGMDVWGQGTTVTVSS |
| | | | SEQ ID NO: 26148 | SEQ ID NO: 30154 |
| **iPS:435317** | **21-225_147D2** | NA | GAAATTGTGTTGACGCAGTCTCCAGGCACCCTGTCTTTGTCTCCAGGGGAAAGAGCCACCCTCTCCTGCAGGGCCAGTCAGAGTGTTGGCAGCAGCTACTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGGTAGCTTATTCACTTTCGGCCCTGGGACCAAAGTGGATATCAAA | CAGGTGCTACTGCAGGAGTCGGGCCCAGGACTGGTGAAGCCTTCACAGACCCTGTCCCTCACCTGCGCTGTCTCTGGTGGCCCCATCAGCAGTGGTGATTACTACTGGAACTGGATCCGCCAGCGCCCAGGGAAGGGCCTGGAGTGGATTGGGTTCATCTATTACACTGGGAGCACCTACTACAACCCGTCCCTCAAGAGTCGAGTTTCCATATCGGAAGACACGTCTGAGAACCAGTTCTCCCTGAACCTGAGTTCTGTGACTGCCGCGGACACGGCCGTGTATTACTGTGCGAGAGGGGGAGCTTACTACTCCTACTACGGTATGGACGTCTGGGGCCAAGGGACCACGGTCACCGTCTCCTCA |
| | | | SEQ ID NO: 26149 | SEQ ID NO: 30155 |
| | | AA | EIVLTQSPGTLSLSPGERATLSCRASQSVGSSYLAWYQQKPGQAPRLLIYGASSRATGIPDRFSGSGSGTDFTLTISRLEPEDFAVYYCQQYGSLFTFGPGTKVDIK | QVLLQESGPGLVKPSQTLSLTCAVSGGPISSGDYYWNWIRQRPGKGLEWIGFIYYTGSTYYNPSLKSRVSISEDTSENQFSLNLSSVTAADTAVYYCARGGAYYSYYGMDVWGQGTTVTVSS |
| | | | SEQ ID NO: 26150 | SEQ ID NO: 30156 |

FIGURE 50

| | | | | |
|---|---|---|---|---|
| **iPS:435319** | **21-225_147E3** | NA | GAAATTGTGTTGACGCAGTCTCCAGGCACCCTGTCTTTGTCTCCAGGGGAAAGAGCCACCCTCTCCTGCAGGGCCAGTCAGAGTGTTATCAGTAGCTACTTAGCCTGGTACCAGCAGAAACCTGGCCAGGCTCCCAGGCTCCTCATCTATGGTGCATCCAGCAGGGCCACTGCCATCCCAGACAGGTTCAGTGGCAGTGGGTCTGGGACAGACTTCACTCTCACCATCAGCAGACTGGAGCCTGAAGATTTTGCAGTGTATTACTGTCAACAATATGGTAGGTCACCATTCAATTTCGGCCCTGGGACCAAAGTGGATATCAAA | CAGGTGCAGCTGCAGGAGTCGGGCCCAGGACTGGTGAAGCCTTCACAGACCCTGTCCCTCACCTGCACTGTCTCTGGTGGCTCCATCACCAATAGTGGTTACTACTATAGCTGGATCCGCCAGCACCCAGGGAAGGGCCTGGAATGGATTGGGTACATCTATTACAGTGGGGGCACCTACTACAACCCGTCCCTCAAGAGTCGAATTACCATATCAGTGGACACGTCTAACAACCAGTTCTCCCTGAAGCTGAGCTCTGTGACTGCCGCGGACACGGCCGTGTATTACTGTGCGAGAGGGGGATATAACTGGAACCATGCTTTTGATTTCTGGGGCCAAGGGACAATGGTCACCGTCTCTTCA |
| | | | SEQ ID NO: 26151 | SEQ ID NO: 30157 |
| | | AA | EIVLTQSPGTLSLSPGERATLSCRASQSVISSYLAWYQQKPGQAPRLLIYGASSRATAIPDRFSGSGSGTDFTLTISRLEPEDFAVYYCQQYGRSPFNFGPGTKVDIK | QVQLQESGPGLVKPSQTLSLTCTVSGGSITNSGYYYSWIRQHPGKGLEWIGYIYYSGGTYYNPSLKSRITISVDTSNNQFSLKLSSVTAADTAVYYCARGGYNWNHAFDFWGQGTMVTVSS |
| | | | SEQ ID NO: 26152 | SEQ ID NO: 30158 |
| **iPS:435321** | **21-225_147E4** | NA | GACATCGTGATGACCCAGTCTCCAGACTCCCTGGCTGTGTCTCTGGGCGAGAGGGCCACCATCAACTGCAAGTCCAGCCAGAGTGTTTTACACAGCTCCAACAATTACAACTACTTAGCTTGGTACCAGCTGAAACCAAGACAGCCTCCTAAGCTGCTCATTTACTGGGCATCTACCCGGGAATCCGGGGTCCCTGACCGATTCAGTGGCCGCGGGTCGGGGACAGACTTCACTCTCACCATCAGCAGCCTGCAGGCTGAAGATGTGGCAATTTATTACTGTCAGCAATATTATAGTACTCCATCCACTTTCGGCCCTGGGACCAAAGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGAGTAGCAGTGGCTGGTACTTTTTTGACTACTGGGGCCAGGGAACCCTGGTCACCGTCTCCTCA |
| | | | SEQ ID NO: 26153 | SEQ ID NO: 30159 |

FIGURE 50

| | | | | |
|---|---|---|---|---|
| | | AA | DIVMTQSPDSLAVSLGERATINCKSSQSVLHSSNNYNYLAWYQLKPRQPPKLLIYWASTRESGVPDRFSGRGSGTDFTLTISSLQAEDVAIYYCQQYYSTPSTFGPGTKVEIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYYCASSSGWYFFDYWGQGTLVTVSS |
| | | | SEQ ID NO: 26154 | SEQ ID NO: 30160 |
| iPS:435323 | 21-225_147D5 | NA | GACATCGTGATGACCCAGTCTCCAGACTCCCTGGCTGTGTCTCTGGGCGAGAGGGCCACCATCAACTGCAAGTCCAGCCAGAGTGTTTTATACAGCTCCAACAATAACAACTACTTAGCTTGGTACCAGCAGAAACCAGGACAGCCTCCTAAACTGCTCATTTACTGGGCATCTACCCGGGAATCCGGGGTCCCTGACCGATTCAGTGGCAGTGGGTCTGGGACAGATTTCACTCTCTCCATCAGCAGCCTGCAGGCTGAAGATGTGGCAGTTTATTACTGTCACCAATATTATAGTACTCCGTGCAGTTTTGGCCAGGGGACCAAACTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGGTGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGGGGACACGGCCGTGTATTACTGTGCGGGGAGCAGTGGCTGGTACTACTTTGACTACTGGGGCCAGGGCACCCTGGTCACCGTCTCCTCA |
| | | | SEQ ID NO: 26155 | SEQ ID NO: 30161 |
| | | AA | DIVMTQSPDSLAVSLGERATINCKSSQSVLYSSNNNNYLAWYQQKPGQPPKLLIYWASTRESGVPDRFSGSGSGTDFTLSISSLQAEDVAVYYCHQYYSTPCSFGQGTKLEIK | QVQLVQSGAEVKKPGASVKVSCKASGYTFTNYDINWVRQATGQGLEWMGWVHPNSGNTGYAQKFQGRVTMTRNTSISTAYMELSSLRSGDTAVYYCAGSSGWYYFDYWGQGTLVTVSS |
| | | | SEQ ID NO: 26156 | SEQ ID NO: 30162 |

FIGURE 50

| | | | | |
|---|---|---|---|---|
| **iPS:435325** | **21-225_147H5** | NA | GACATCCAGATGACCCAGTCTCCATCCTCCCTGTCTGCATCTGTAGGAGACAGAGTCACCATCACTTGCCGGGCAAGTCGGGGCATTAGAGATGATTTAGGCTGGTATCAGCAGAAACCAGGGAAAGCCCCTAAGCGCCTGATCTATGCTGCATCCAGTTTGCAGAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTATCCTCGGACGTTCGGCCAAGGGACCAAGGTGGAAATCAAA<br>SEQ ID NO: 26157 | CAGGTGCAGCTGGTGGAGTCTGGGGGAGGCGTGGCCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGGAAGTAATAAGTACTATGCAGACTCCGTGAAGGGCCGACTCACCATCTCCAGAGACAATTCCAAGAACACGTTGTATCTGCAAATGAACAGCCTGAGCGCCGAGGACACGGCTGTGTATTACTGTGCGAGAGAGAGGTATAGCAGTGGCTGGTACGACTACGGTTTGGACGTCTGGGGCCAAGGGACCACGGTCACCGTCTCCTCA<br>SEQ ID NO: 30163 |
| | | AA | DIQMTQSPSSLSASVGDRVTITCRASRGIRDDLGWYQQKPGKAPKRLIYAASSLQSGVPSRFSGSGSGTEFTLTISSLQPEDFATYYCLQHYSYPRTFGQGTKVEIK<br>SEQ ID NO: 26158 | QVQLVESGGGVAQPGRSLRLSCAASGFTFSDYGMHWVRQAPGKGLEWVAVIWYDGSNKYYADSVKGRLTISRDNSKNTLYLQMNSLSAEDTAVYYCARERYSSGWYDYGLDVWGQGTTVTVSS<br>SEQ ID NO: 30164 |
| **iPS:435327** | **21-225_147G6** | NA | GACATCGTGATGACCCAGTCTCCAGACTCCCTGGCTGTGTCTCTGGGCGAGAGGGCCACCATCAACTGCAAGTCCAGCCAGAGTGTTTTATACAGCTCCAACAGTAACAACTACTTAGCTTGGTACCAGCAGAAACCAGGCCAGCCTCCTAAGCTGCTCATTTACTGGGCATCTGCCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATACTACTCCTCCCACTTTCGGCCCTGGGACCAAAGTGGATATCAAA<br>SEQ ID NO: 26159 | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGTTGGATGCACCCCAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGTATAGCAGTGGCTGGTACTGGTTCGACCCCTGGGGCCAGGGAACCCTGGTCACCGTCTCCTCA<br>SEQ ID NO: 30165 |

FIGURE 50

| | | | | |
|---|---|---|---|---|
| | | AA | DIVMTQSPDSLAVSLGERATINCKSSQSVLYSSNSNNYLAWYQQKPGQPPKLLIYWASARESGVPDRFSGSGSGTDFTLTISSLQAEDVAVYYCQQYYTTPPTFGPGTKVDIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAYSSGWYWFDPWGQGTLVTVSS |
| | | | SEQ ID NO: 26160 | SEQ ID NO: 30166 |
| iPS:435329 | 21-225_147A8 | NA | GATATTGTGATGACCCAGACTCCACTCTCTCTGTCCGTCACCCCTGGACAGCCGGCCTCCATCTCCTGCAAGACTAGTCAGAGCCTCCTGCATAGTGAAGGAAAGACCTATTTGTATTGGTACCTGCAGAAGCCAGGCCAGCCTCCACAGCTCCTGATCTATGAAGTTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGGTTTATTACTGCATGCAAAGTATACAGCTAATCACCTTCGGCCAAGGGACACGACTGGAGATTAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAAGGTATAGCAGCAGCTGGACGGGGGGTATGGACGTCTGGGGCCAAGGGACCACGGTCACCGTCTCCTCA |
| | | | SEQ ID NO: 26161 | SEQ ID NO: 30167 |
| | | AA | DIVMTQTPLSLSVTPGQPASISCKTSQSLLHSEGKTYLYWYLQKPGQPPQLLIYEVSNRFSGVPDRFSGSGSGTDFTLKISRVEAEDVGVYYCMQSIQLITFGQGTRLEIK | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARRYSSSWTGGMDVWGQGTTVTVSS |
| | | | SEQ ID NO: 26162 | SEQ ID NO: 30168 |

FIGURE 50

| | | | | |
|---|---|---|---|---|
| **iPS:435331** | **21-225_147G8** | NA | CAAATTGTGTTGACGCAGTCTCCAGGCACCCTGTCTTTGTCTCCAGGGGAAAGAGCCACCCTCTCCTGCAGGGCCAGTCAGAGAATTTTCAGCAACTACTTAGCCTGGTACCAGCAGAAGCCTGGCCAGGCTCCCAGGATCCTCATCTATGGTGCATCCAGCAGGGCCACTGGCATCCCAGACAGGATCAGTGGCAGTGGGTCTGGGACAGACTTCACTCTCACCATCACCAGACTGGAGCCTGAAGATTTTGCAGTGTATTACTGTCAGCAGTATGATAGCTCACCGTGGACGTTCGGCCAAGGGACCAAGGTGGAAATCAAC | CAGGTGCAGCTACAGCAGTGGGGCGCAGGACTGTTGAAGCCTTCGGAGACCCTGTCCCTCACCTGCGGTGTCTATGGTGGGTCCTTCAGTGCTTACTACTGGAGCTGGATCCGCCAGCCCCCAGGGAAGGGGCTGGAGTGGATTGGGGAAATCAATCATAGTGGAAGTACCAACTACAAACCGTCCCTCAAGAGTCGAGTCACCATATCAGTAGACACGTCCAAGAACCAGTTCTCCCTGAAGCTGAGCTCTGTGACCGCCGCGGACACGGCTGTGTATTACTGTGCGAGAGACTACGGTGTTTTTGACTACTGGGGCCAGGGAACCCTGGTCACCGTCTCCTCA |
| | | | SEQ ID NO: 26163 | SEQ ID NO: 30169 |
| | | AA | QIVLTQSPGTLSLSPGERATLSCRASQRIFSNYLAWYQQKPGQAPRILIYGASSRATGIPDRISGSGSGTDFTLTITRLEPEDFAVYYCQQYDSSPWTFGQGTKVEIN | QVQLQQWGAGLLKPSETLSLTCGVYGGSFSAYYWSWIRQPPGKGLEWIGEINHSGSTNYKPSLKSRVTISVDTSKNQFSLKLSSVTAADTAVYYCARDYGVFDYWGQGTLVTVSS |
| | | | SEQ ID NO: 26164 | SEQ ID NO: 30170 |
| **iPS:435333** | **21-225_147E9** | NA | GACATCCAGATGACCCAGTCTCCATCCTCACTGTCTGCATCTGTAGGAGACAGAGTCACCATCACTTGTCGGGCGAGTCAGGACATTAACAATTATTTAGCCTGGTTTCAGCAGAAACCAGGGAAAGCCCCTAAGTCCCTGATCGTTGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATAATAGTTACCCTCTCACTTTCGGCGGAGGGACCAAGGTGGAGATCAAA | GAGGTGCAGCTGGTGGAGTCTGGGGGAGGCTTGGTACAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTTTCATCCATTAGTGGTAGAAACACTACCATATACTATGCAGACTCTGTGAAGGGCCGATTCACCATCTCCAGAGACAATGCCAAGAACTCACTGTATCTGCAAATGAACAGCCTGAGAGACGAGGACACGGCTGTTTATTACTGTTCGAGAGATCGGGGCAGTTGCTGGGGCCAGGGAACCCTGGTCACCGTCTCCTCA |
| | | | SEQ ID NO: 26165 | SEQ ID NO: 30171 |

FIGURE 50

| | | | | |
|---|---|---|---|---|
| | | AA | DIQMTQSPSSLSASVGDRVTITCRASQDINNYLAWFQQKPGKAPKSLIVAASSLQSGVPSKFSGSGSGTDFTLTISSLQPEDFATYYCQQYNSYPLTFGGGTKVEIK | EVQLVESGGGLVQPGGSLRLSCAASGFTFSSYSMNWVRQAPGKGLEWVSSISGRNTTIYYADSVKGRFTISRDNAKNSLYLQMNSLRDEDTAVYYCSRDRGSCWGQGTLVTVSS |
| | | | SEQ ID NO: 26166 | SEQ ID NO: 30172 |
| **iPS:435335** | **21-225_147D10** | NA | GACATCCAGATGACCCAGTCTCCAGCTTCCGTGTCTGCATCTGTAGGAGACAGAGTCACCATAACTTGTCGGGCGAGTCAGAATATTAGCAACTGGTTAACCTGGTATCAGCAGAAACCAGGGAAAGCCCCTAAGCTCCTGATCTATGCTGCATCCAGTTTGCAAAGTGGGGTCCCATCAAGGTTCAGCGGCAGTGGATCTGGGACAGATTTCACTCTCTCCATCAGCAGCCTGCAGCCTGAAGATTTTGCAACTTACTATTGTCAACAGACTGACAGTTTCCCATTCACTTTCGGCCCTGGGACCAAAGTGGATGTCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTCGTGGTGGTAACACATTCTACGCAGACTCCGTGAAGGGCCGCTTCACCATCTCCAGAGACAATTCCAAGAACACGCTCTATCTGCAAATGAACAGCCTGAGAGCCGAGGACACGGCCGTATATTACTGTGCGAAAAAGGATTATGATTACGTTTGGGGGAGTCCTTACTTTGACTACTGGGGCCAGGGAACCCTGGTCACCGTCTCCTCA |
| | | | SEQ ID NO: 26167 | SEQ ID NO: 30173 |
| | | AA | DIQMTQSPASVSASVGDRVTITCRASQNISNWLTWYQQKPGKAPKLLIYAASSLQSGVPSRFSGSGSGTDFTLSISSLQPEDFATYYCQQTDSFPFTFGPGTKVDVK | EVQLLESGGGLVQPGGSLRLSCAASGFTFSSYAMSWVRQAPGKGLEWVSAISGRGGNTFYADSVKGRFTISRDNSKNTLYLQMNSLRAEDTAVYYCAKKDYDYVWGSPYFDYWGQGTLVTVSS |
| | | | SEQ ID NO: 26168 | SEQ ID NO: 30174 |

FIGURE 50

| | | | | |
|---|---|---|---|---|
| **iPS:435339** | **21-225_147D12** | NA | GACATCCAGATGACCCAGTCTCCAGCTTCCGTGTCTGCATCTGTAGGAGACAGAGTCACCATAACTTGTCGGGCGAGTCAGGGTATTAGCAACTGGTTAGCCTGGTATCAGGAGAAACCAGGGAAAGCCCCTAAACTCCTGATCTATGCTGCATCCAGTTTGCAAAGTGGGGTCCCATCAAGGTTCAGCGGCAGTGGATCCGGGACAGATTTCACTCTCTCCATCAGCAGCCTGCAGCCTGAAGATTTTGCAACTTACTATTGTCAACAGACTGACAGTTTCCCATTCACTTTCGGCCCTGGGACCAAAGTGGATGTCAAA | GAGGTGCAGCTGTTGGAGTCTGGGGGAGGCTTGGTACAGTCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTCGTGGTGGTAACACATTCTACGCAGACTCCGTGAAGGGCCGCTTCACCATCTCCAGAGACAATTCCAAGAACACGCTCTATCTGCAAATGAACAGCCTGAGAGCCGAGGACACGGCCGTATATTACTGTGCGAAAAAGGATTATGATTACGTTTGGGGGAGTCCTTACTTTGACTACTGGGGCCAGGGAACCCTGGTCACCGTCTCCTCA |
| | | | SEQ ID NO: 26169 | SEQ ID NO: 30175 |
| | | AA | DIQMTQSPASVSASVGDRVTITCRASQGISNWLAWYQEKPGKAPKLLIYAASSLQSGVPSRFSGSGSGTDFTLSISSLQPEDFATYYCQQTDSFPFTFGPGTKVDVK | EVQLLESGGGLVQSGGSLRLSCAASGFTFSSYAMSWVRQAPGKGLEWVSAISGRGGNTFYADSVKGRFTISRDNSKNTLYLQMNSLRAEDTAVYYCAKKDYDYVWGSPYFDYWGQGTLVTVSS |
| | | | SEQ ID NO: 26170 | SEQ ID NO: 30176 |
| **iPS:435341** | **21-225_148B2** | NA | GCTATTGTGATGACCCAGACTCCACTCTCTCTGTCCGTCACCCCTGGACAGCCGGCCTCCATCTCCTGCAAGTCTAGTCAGAGCCTCCTGCATGGTGATGGAAAGACCTATTTTTATTGGTACCTGCAGAAGCCAGGCCAGCCTCCACAGCTCCTGATCTATGAAGTTTCCCACCGGTTCTCTGGAGTGCCAGATAGGTTCAGTGGCAGCGGGTCAGGGACAGATTTCACACTGAAAATCAGCCGGGTGGAGGCTGAGGATGTTGGGGTTTATTACTGCATGCAAAGTATACAGATTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAATTATCTGGTATGATGGAAGTTATAAATACTATGCAGACTCCGTGAAGGGCCGATTCACCATCTCCAGAGACAATTCCAAGAACACGCTGTATCTGCAAATGAACAGCCTGAGTGCCGAGGACACGGCTGTGTATTACTGTGCGAGAGATCATTTCGATTTTTGGAGTGGTCACTTTGACTACTGGGGCCAGGGAACCCTGGTCACCGTCTCCTCA |
| | | | SEQ ID NO: 26171 | SEQ ID NO: 30177 |

FIGURE 50

| | | | | |
|---|---|---|---|---|
| | | AA | AIVMTQTPLSLSVTPGQPASISCKSSQSLLHGDG KTYFYWYLQKPGQPPQLLIYEVSHRFSGVPDRFS GSGSGTDFTLKISRVEAEDVGVYYCMQSIQIPWT FGQGTKVEIK | QVQLVESGGGVVQPGRSLRLSCAASGFTFSSYGMH WVRQAPGKGLEWVAIIWYDGSYKYYADSVKGRFT ISRDNSKNTLYLQMNSLSAEDTAVYYCARDHFDF WSGHFDYWGQGTLVTVSS |
| | | | SEQ ID NO: 26172 | SEQ ID NO: 30178 |
| iPS:435343 | 21-225_148E2 | NA | GACATCCAGATGACCCAGTCTCCAGCTTCCGT GTCTGCATCTGTAGGAGACAGAGTCACCATAA CTTGTCGGGCGAGTCAGGGTATTAGCAACTGG TTAGCCTGGTATCAGCAGAAACCAGGGAAAG CCCCTAAGCTCCTGATCTATGCTGCATCCAGTT TGCAAAGTGGGGTCCCATCAAGGTTCAGCGGC AATGAATCTGGGACAGATTTCACTCTCTCCAT CAGCAGCCTGCAGCCTGAAGATTTTGCAACTT ACTATTGTCAACAGACTGACAGTTTCCCATTC ACTTTCGGCCCTGGGACCAAAGTGGATGTCAA A | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTGGT AACACATTCTACGCAGACTCCGTGAAGGGCCGCT TCACCATCTCCAGAGACAATTCCAAGAACACGCT CTATCTGCAAATGAACAGCCTGAGAGCCGAGGA CACGGCCGTATATTACTGTGCGAAAAAGGATTAT GATTACGTTTGGGGGAGTCCTTACTTTGACTACT GGGGCCAGGGAACCCTGGTCACCGTCTCCTCA |
| | | | SEQ ID NO: 26173 | SEQ ID NO: 30179 |
| | | AA | DIQMTQSPASVSASVGDRVTITCRASQGISNWLA WYQQKPGKAPKLLIYAASSLQSGVPSRFSGNES GTDFTLSISSLQPEDFATYYCQQTDSFPFTFGPGT KVDVK | EVQLLESGGGLVQPGGSLRLSCAASGFTFSSYAMS WVRQAPGKGLEWVSAISGRGGNTFYADSVKGRFTI SRDNSKNTLYLQMNSLRAEDTAVYYCAKKDYDYV WGSPYFDYWGQGTLVTVSS |
| | | | SEQ ID NO: 26174 | SEQ ID NO: 30180 |

FIGURE 50

| | | | | |
|---|---|---|---|---|
| iPS:435345 | 21-225_148G3 | NA | GACATCGTGATGACCCAGTCTCCAGACTCCCT GGCTGTGTCTCTGGGCGAGAGGGCCACCATCC ACTGCAAGTCCAGCCAACGTGTTTTACACAGC TCCAACAATTATAACTACTTAGCTTGGTACCA GCAGAAACCAGGACAGCCTCCTAAGCTGCTCA TTTACTGGGCATCTACCCGGGATTCCGGGGTC CCTGACCGATTCAGTGGCAGCGGGTCTGGGGC CGATTTCACTCTCACCATCAGCAGCCTGCAGG CTGAAGATGTGGCACTTTATTACTGTCAGCAA TATTATAGTACTCCATTCACTTTCGGCCCTGGG ACCAAAGTGGATATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCTCTGGACAAGGGC TTGAGTGGATGGGATGGATGCACCCTAACAGTGG TAACACAGGCTATGCACAGAACTTCCAGGGCAG AGTCACCATGACCAGGAACACCTCCATAAGCAC AGCCTACATGGAGCTGAGCAGCCTGAGATCTGA GGACACGGCCGTGTATTACTGTGCGAGTAGCAGT GGCTGGTACTTTTTTGACTACTGGGGCCAGGGAA CCCTGGTCACCGTCTCCTCA |
| | | | SEQ ID NO: 26175 | SEQ ID NO: 30181 |
| | | AA | DIVMTQSPDSLAVSLGERATIHCKSSQRVLHSSN NYNYLAWYQQKPGQPPKLLIYWASTRDSGVPD RFSGSGSGADFTLTISSLQAEDVALYYCQQYYST PFTFGPGTKVDIK | QVQLVQSGAEVKKPGASVKVSCKASGYTFTNYDIN WVRQASGQGLEWMGWMHPNSGNTGYAQNFQGR VTMTRNTSISTAYMELSSLRSEDTAVYYCASSSGW YFFDYWGQGTLVTVSS |
| | | | SEQ ID NO: 26176 | SEQ ID NO: 30182 |
| iPS:435347 | 21-225_148C4 | NA | GACATCCAGATGACCCAGTCTCCATCCTCCCT GTCTGCATCTGTAGGAGACAGAGTCACCATCA CTTGCCGGGCAAGTCAGAGCATTATCAACTAT TTAAATTGGTATCAGCAGAAACCAGGGAAAG CCCCTAAGGTCCTGATCTATACTGCATCCAGTT TACAGAGTGGGGTCCCATCAAGGTTCAGTGGC AGTGGATCTGGGACAGATTTCACTCTCACCAT CAGCAGTCTGCAACCTGAAGATTTTACAACTT ACTACTGTCAACAGAGTTACAGTACCCCCACT TTCGGCGGAGGGACCAAGGTAGAGATCGAA | GAGGTGCAGCTCTTGGAGTCTGGGGGAGGCTTGG TACAGCCTGGGGGGTCCCTGAGACTCTCCTGTGC AGCCTCTGGATTCACCTTTAGCAGCTATGCCATG AACTGGGTCCGCCAGGCTCCAGGGAAGGGGCTG GAGTGGGTCTCAGCTATTAGTGGTAGTGGTGGTA ACACATTCTACGCAGACTCCGTGAAGGGCCGGTT CACCATCTCCAGAGACAATTCCAAGAACACGCTG TATCTGCAAATGAACAGCCTGAGAGCCGAGGAC ACGGCCGTATATTACTGTGCGAAACGGGTGACG GACTACGGTGGTAACGACTGGTTCGACCCCTGGG GCCAGGGAACCCTGGTCACCGTCTCCTCA |
| | | | SEQ ID NO: 26177 | SEQ ID NO: 30183 |

FIGURE 50

| | | | | |
|---|---|---|---|---|
| | | AA | DIQMTQSPSSLSASVGDRVTITCRASQSIINYLNWYQQKPGKAPKVLIYTASSLQSGVPSRFSGSGSGTDFTLTISSLQPEDFTTYYCQQSYSTPTFGGGTKVEIE | EVQLLESGGGLVQPGGSLRLSCAASGFTFSSYAMNWVRQAPGKGLEWVSAISGSGGNTFYADSVKGRFTISRDNSKNTLYLQMNSLRAEDTAVYYCAKRVTDYGGNDWFDPWGQGTLVTVSS |
| | | | SEQ ID NO: 26178 | SEQ ID NO: 30184 |
| **iPS:435349** | **21-225_148F5** | NA | GATATTGTGATGACCCAGACTCCACTCTCTCTGTCCGTCACCCCTGGACAGCCGGCCTCCATCTCCTGTAAGTCTAGTCAGAGCCTCCTGCATAGTGAAGGAAAGACCTATTTGTATTGGTACCTGCAGAAGCCAGGCCAGCCTCCACAGCTCCTGATCTATGAAGTTTCCTACCGGGTCTCTGGAGTGCCAGATAGATTCAGTGGCAGCGGGTCAGGGACAGATTTCACACTGAAAATCAGCCGGGTGGAGGCAGAGGATGTTGGGGTCTATTTCTGCATGCAAAGTATACAGCTTCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGAGGTATAGCAGCAGCTGGTCGGGGGGTATGGACGTCTGGGGCCAAGGGACCACGGTCACCGTCTCCTCA |
| | | | SEQ ID NO: 26179 | SEQ ID NO: 30185 |
| | | AA | DIVMTQTPLSLSVTPGQPASISCKSSQSLLHSEGKTYLYWYLQKPGQPPQLLIYEVSYRVSGVPDRFSGSGSGTDFTLKISRVEAEDVGVYFCMQSIQLPLTFGGGTKVEIK | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARRYSSSWSGGMDVWGQGTTVTVSS |
| | | | SEQ ID NO: 26180 | SEQ ID NO: 30186 |

FIGURE 50

| | | | | |
|---|---|---|---|---|
| **iPS:435351** | **21-225_148B6** | NA | GACATCCAGATGACCCAGTCTCCATCCTCACT GTCTGCATCTGTAGGAGACAGAGTCAGCATCA CTTGTCGGGCGAGTCAGGGCATTAGCAAATAT TTAGCCTGGTTTCAGCAGAAACCAGGGAAAGC CCCTAAGTCCCTGATCTTTGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATAATAGTTTCCCATTCAC TTTCGGCCCTGGGACCAAAGTGGATATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCGGCTACTATA TGCACTGGGTGCGACAGGCCCCTGGACAAGGTCT TGAGTGGATGGGTTGGATCCACCCTAACAATGGT GGCACAAACTATGCACAGACGTTTCAGGGCAGG GTCACCATGACCAGGGACACGTCCATCAGCACA GTCTACATGGAGCTGAGCAGGCTGAGATCTGAC GACACGGCCGTGTATTACTGTGCGAGAGATCCTG TAGTAGTACCAGCTGCCCCCTTTGACTACTGGGG CCAGGGAACCCTGGTCACCGTCTCCTCA |
| | | | SEQ ID NO: 26181 | SEQ ID NO: 30187 |
| | | AA | DIQMTQSPSSLSASVGDRVSITCRASQGISKYLA WFQQKPGKAPKSLIFAASSLQSGVPSKFSGSGSG TDFTLTISSLQPEDFATYYCQQYNSFPFTFGPGTK VDIK | QVQLVQSGAEVKKPGASVKVSCKASGYTFTGYYM HWVRQAPGQGLEWMGWIHPNNGGTNYAQTFQGR VTMTRDTSISTVYMELSRLRSDDTAVYYCARDPVV VPAAPFDYWGQGTLVTVSS |
| | | | SEQ ID NO: 26182 | SEQ ID NO: 30188 |
| **iPS:435353** | **21-225_148F8** | NA | GACATCGTGATGACCCAGTCTCTAGACTCCCT GGCTGTGTCTCTGGGCGAGAGGGCCACCATCA ACTGCAAGTCCAGCCAGAGTGCTTTACACAGC TCCAACAATTACAACTACTTAGCTTGGTACCA GCAGAAACCAGGACAGCCTCCTAAACTGCTCA TTTACTGGGCATCTACCCGGAAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTATTCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGCCAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGAGTAGCA GTGGCTGGTACTACTTTGACTACTGGGGCCAGGG AACCCTGGTCACCGTCTCCTCA |
| | | | SEQ ID NO: 26183 | SEQ ID NO: 30189 |

FIGURE 50

| | | | | |
|---|---|---|---|---|
| | | AA | DIVMTQSLDSLAVSLGERATINCKSSQSALHSSN<br>NYNYLAWYQQKPGQPPKLLIYWASTRKSGVPD<br>RFSGSGSGTDFTLTISSLQAEDVAVYYCQQYYSI<br>PPTFGQGTKVEIK | QVQLVQSGAEVKKPGASVKVSCKASGYTFTNYDIN<br>WVRQATGQGLEWMGWMNPNSGNTGYAQKFQGR<br>VTMTRNTSISTAYMELSSLRSEDTAVYYCASSSGW<br>YYFDYWGQGTLVTVSS |
| | | | SEQ ID NO: 26184 | SEQ ID NO: 30190 |
| iPS:435355 | 21-225_148H9 | NA | GACATCCAGATGACCCAGTCTCCATCCTCCCT<br>GTCTGCATCTGTAGGAGACAGAGTCACCATCA<br>CTTGCCGGGCAAGTCAGAGCATTAGTAACTAT<br>TTAAATTGGTATCAGCAGAAACCAGGGAAAG<br>CCCCTAAGGTCCTGATCTATATTGCATCCAGTT<br>TGCAAAGTGGGGTCCCATCTAGGTTCAGTGGC<br>AGTGGATCTGGGACAGATTTCACTCTCACCAT<br>CAGCAGTCTGCAACCTGAAGATTTTGCAACTT<br>ACTACTGTCAACAGAGTTACAGTACCCCCACT<br>TTCGGCGGAGGGACCAAGGTGGAGATCAAA | GAGGTGCAGCTGTTGGAGTCTGGGGGAGGCTTG<br>GTACAGCCGGGGGGGTCCCTGAGACTCTCCTGTG<br>CAGCCTCTGGATTCACCTTTAGCAGCTATGCCAT<br>GAGCTGGGTCCGCCAGGCTCCAGGGAAGGGGCT<br>GGAGTGGGTCTCAGCTATTAGTGGTCGTGGTGGT<br>AACACATTCTACGCAGACTCCGTGAAGGGCCGGT<br>TCACCATCTCCAGAGACAATTCCAAGAACACACT<br>ATATTTGCAAATGAACAGCCTGAGAGCCGAGGA<br>CACGGCCGTATATTACTGTGCGAAACGGGTGACG<br>GACTACGGTGGTAACGACTGGTTCGACCCCTGGG<br>GCCAGGGAACCCTGGTCACCGTCTCCTCA |
| | | | SEQ ID NO: 26185 | SEQ ID NO: 30191 |
| | | AA | DIQMTQSPSSLSASVGDRVTITCRASQSISNYLN<br>WYQQKPGKAPKVLIYIASSLQSGVPSRFSGSGSG<br>TDFTLTISSLQPEDFATYYCQQSYSTPTFGGGTK<br>VEIK | EVQLLESGGGLVQPGGSLRLSCAASGFTFSSYAMS<br>WVRQAPGKGLEWVSAISGRGGNTFYADSVKGRFTI<br>SRDNSKNTLYLQMNSLRAEDTAVYYCAKRVTDYG<br>GNDWFDPWGQGTLVTVSS |
| | | | SEQ ID NO: 26186 | SEQ ID NO: 30192 |

FIGURE 50

| | | | | |
|---|---|---|---|---|
| **iPS:435357** | **21-225_148G10** | NA | GATATTGTGATGACCCAGACTCCACTCTCTCTGTCCGTCACCCCTGGACAGCCGGCCTCCATCTCCTGCAAGTCTAGTCAGAGCCTCCTGCATGGTGATGGAAAGACCTATTTGTATTGGTACCTGCAGAGGCCAGGCCAGCCTCCACAGCTCCTGATCTATGAAGTTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGGTTTATTACTGCATGCAAAGTATACAGCTTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAATTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAAGACACGGCTGTGTATTACTGTGCGAGAGATCGTTACGATTTTTGGAGTGGTCACTTTGACTACTGGGGCCAGGGAACCCTGGTCACCGTCTCCTCA |
| | | | SEQ ID NO: 26187 | SEQ ID NO: 30193 |
| | | AA | DIVMTQTPLSLSVTPGQPASISCKSSQSLLHGDGKTYLYWYLQRPGQPPQLLIYEVSNRFSGVPDRFSGSGSGTDFTLKISRVEAEDVGVYYCMQSIQLPWTFGQGTKVEIK | QVQLVESGGGVVQPGRSLRLSCAASGFTFSSYGMHWVRQAPGKGLEWVAIIWYDGSNKYYADSVKGRFTISRDNSKNTLYLQMNSLRAEDTAVYYCARDRYDFWSGHFDYWGQGTLVTVSS |
| | | | SEQ ID NO: 26188 | SEQ ID NO: 30194 |
| **iPS:435359** | **21-225_148H10** | NA | GATATTGTGATGACCCAGACTCCACTCTCTCTGTCCGTCACTCCTGGACAGCCGGCCTCCATCTCCTGCAAGTCTAGTCAGAGCCTCCTGCATAGTGAGGGAAAGACCTATTTGTATTGGTACCTGCAGAAGCCAGGCCAGCCTCCACAGCTCCTGATCTATGAAGTTTCCTACCGGGTCTCTGGAGTGCCAGATAGGTTCAGTGGCAGCGGGTCAGGGACAGATTTCACACTGAAAATCAGCCGGGTGGAGGCAGAGGATGTTGGGGTCTATTACTGCATGCAGAGTATACAGCTTCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGGAGACTCCGTGAAGGGCCGATTCACCATCTCCAGAGACAATTCCAAGAACACGCTGTATCTGCAAATGAACAGCCTGAGAGCCGAGGACACGGCTGTGTATTATTGTGCGAGGAGGTATAGCAGCAGCTGGTCGGGGGGTATGGACGTCTGGGGCCAAGGGACCACGGTCACCGTCTCCTCA |
| | | | SEQ ID NO: 26189 | SEQ ID NO: 30195 |

FIGURE 50

| | | | | |
|---|---|---|---|---|
| | | AA | DIVMTQTPLSLSVTPGQPASISCKSSQSLLHSEGKTYLYWYLQKPGQPPQLLIYEVSYRVSGVPDRFSGSGSGTDFTLKISRVEAEDVGVYYCMQSIQLPLTFGGGTKVEIK | QVQLVESGGGVVQPGRSLRLSCAASGFTFSSYGMHWVRQAPGKGLEWVAVIWYDGSNKYYGDSVKGRFTISRDNSKNTLYLQMNSLRAEDTAVYYCARRYSSSWSGGMDVWGQGTTVTVSS |
| | | | SEQ ID NO: 26190 | SEQ ID NO: 30196 |
| **iPS:435361** | **21-225_148E11** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTCTGCTGCATCCAGTTTGCAAAGTGGGGTCCCATCAAGGTTCACCGGCAGTGGATCTGGGACAGAATTCACTTTCACAATCAGCAGCGTGCAGCCTGAAGATTTTGCAACTTATTACTGTCTACAGCATCGTAATTACCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGTCTCCATCAGCAGAAGTAGTTACTACTGGGGCTGGATCCGCCAGCCCCCAGGGAAGGGGCTGGAGTGGATTGGGAGTATCTATTATAGTGGGAGCACCTCCTACAACCCGTCCCTCAAGAGTCGAGTCACCATATCCGTAGACACGTCCAAGAACCAGTTCTCCCTGAAGCTGAGCTCTGTGACCGCCGCAGACACGGCTGTGTTTTACTGTGCGAGACTTGATCCCCAGTGGAGTTTTGACTACTGGGGCCAGGGAATCCTGGTCACCGTCTCCTCA |
| | | | SEQ ID NO: 26191 | SEQ ID NO: 30197 |
| | | AA | DIQMTQSPSSLSASVGDRVTITCRASQGIRNDLGWYQQKPGKAPKRLISAASSLQSGVPSRFTGSGSGTEFTFTISSVQPEDFATYYCLQHRNYPLTFGGGTKVEIK | QLQLQESGPGLVKPSETLSLTCTVSGVSISRSSYYWGWIRQPPGKGLEWIGSIYYSGSTSYNPSLKSRVTISVDTSKNQFSLKLSSVTAADTAVFYCARLDPQWSFDYWGQGILVTVSS |
| | | | SEQ ID NO: 26192 | SEQ ID NO: 30198 |

FIGURE 50

| | | | | |
|---|---|---|---|---|
| iPS:435363 | 21-225_148F12 | NA | GACATCCAGATGACCCAGTCTCCTTCCTCCCTGTCTGCATCTGTAGGAGACAGAGTCACCATCACTTGCCGGGCAAGTCAGGGCATTAGAAATGCCTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTTCTGTCTACAGCATAATAGTTACCCTCTCATTTTCGGCGGAGGGACCAAGGTGGAGATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAATGGTGGTTACTACTGGAACTGGATCCGCCAGCACCCAGGGAAGGGCCTGGAGTGGATTGGGTACATCTATTACAGTGGGAGCACCTACTACAACCCGTCCCTCAAGAGTCAAATTACCATATCAGTGGACACGTCTAAGGACCAGTTCTCCCTGAGGCTGAGCTCTGTGACTGCCGCGGACACGGCCGTGTATTACTGTGCGAGGTACAGTACCTACGACTACTACTACGGTATGGACGTCTGGGGCCAAGGGACCACGGTCACCGTCTCCTCA |
| | | | SEQ ID NO: 26193 | SEQ ID NO: 30199 |
| | | AA | DIQMTQSPSSLSASVGDRVTITCRASQGIRNALGWYQQKPGKAPKRLIYAASSLQSGVPSRFSGSGSGTEFTLTISSLQPEDFATYFCLQHNSYPLIFGGGTKVEIK | QVQLQESGPGLVKPSQTLSLTCTVSGGSISNGGYYWNWIRQHPGKGLEWIGYIYYSGSTYYNPSLKSQITISVDTSKDQFSLRLSSVTAADTAVYYCARYSTYDYYYGMDVWGQGTTVTVSS |
| | | | SEQ ID NO: 26194 | SEQ ID NO: 30200 |
| iPS:435365 | 21-225_149F1 | NA | GATATTGTGATGACCCAGTCTCCACTCTCTCTGTTCGTCACTCCTGGACAGCCGGCCTCCATCTCCTACAAGTCTAGTCAGAGCCTCCTGCATGGTGATGGAAAGACCTATTTTTATTGGTACCTGCAGAAGCCAGGCCAGCCTCCACAGCTCTTGATTTATGAAGTTTCCCACCGGTTCTCTGGAGTGCCAGATAGGTTCAGTGGCAGCGGGTCAGGGACAGATTTCACACTGAAAATCAGCCGGGTGGAGGCTGAGGATGTTGGGGTTTATTACTGCATGCAAAGTATACAGATTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAATTATCTGGTATGATGGAAGTTATAAATACTATGCAGACTCCGTGAAGGGCCGATTCACCATCTCCAGAGACAATTCCAAGAACACGCTGTATCTGCAAATGAACAGCCTGAGTGCCGAGGACACGGCTGTGTATTACTGTGCGAGAGATCATTTCGATTTTTGGAGTGGTCACTTTGACTACTGGGGCCAGGGAACCCTGGTCACCGTCTCCTCA |
| | | | SEQ ID NO: 26195 | SEQ ID NO: 30201 |

FIGURE 50

| | | | | |
|---|---|---|---|---|
| | | AA | DIVMTQSPLSLFVTPGQPASISYKSSQSLLHGDGKTYFYWYLQKPGQPPQLLIYEVSHRFSGVPDRFSGSGSGTDFTLKISRVEAEDVGVYYCMQSIQIPWTFGQGTKVEIK | QVQLVESGGGVVQPGRSLRLSCAASGFTFSSYGMHWVRQAPGKGLEWVAIIWYDGSYKYYADSVKGRFTISRDNSKNTLYLQMNSLSAEDTAVYYCARDHFDFWSGHFDYWGQGTLVTVSS |
| | | | SEQ ID NO: 26196 | SEQ ID NO: 30202 |
| iPS:435367 | 21-225_149G1 | NA | GACATCCAGATGACCCAGTCTCCATCCTCCCTGTCTGCATCTGTAGGAGCCAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAATTTGCAAAGTGGGGTCCCATCAAGGTTCAGTGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGAAGGAAGTAATAAATACTATGCAGACTCCGTGAAGGGCCGATTCACCATCTCCAGAGACAATTCCAAGAACATGTTGTATCTGCAAATGAACAGCCTGAGAGCCGAGGACACGGCTGTGTATTACTGTGCGAGAGAAATAGGATTCAGTGAGGACTACTGGGGCCAGGGAACCCTGGTCACCGTCTCCTCA |
| | | | SEQ ID NO: 26197 | SEQ ID NO: 30203 |
| | | AA | DIQMTQSPSSLSASVGARVTITCRASQGIRNDLGWYQQKPGKAPKRLIYAASNLQSGVPSRFSGSGSGTEFTLTISSLQPEDFATYYCLQHNSYPLTFGGGTKVEIK | QVQLVESGGGVVQPGRSLRLSCAASGFTFSDYGMHWVRQAPGKGLEWVAVIWYEGSNKYYADSVKGRFTISRDNSKNMLYLQMNSLRAEDTAVYYCAREIGFSEDYWGQGTLVTVSS |
| | | | SEQ ID NO: 26198 | SEQ ID NO: 30204 |

FIGURE 50

| | | | | |
|---|---|---|---|---|
| **iPS:435369** | **21-225_149A2** | NA | GACATCGTGATGACCCAGTCTCCAGACTCCCTGGCTGTGTCTCTGGGCGAGAGGGCCACCATCAACTGCAAGTCCAGCCAGAGTGTTTTATACAGTCCCAACAATAACAACTACTTAGCTTGGTACCAGCAGAAACCAGGACAGCCCCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCGTGCAGTTTTGGCCAGGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGGTGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGGGGAGCAGTGGCTGGTACTACTTTGACTACTGGGGCCAGGGAACCCTGGTCACCGTCTCCTCA |
| | | | SEQ ID NO: 26199 | SEQ ID NO: 30205 |
| | | AA | DIVMTQSPDSLAVSLGERATINCKSSQSVLYSPNNNNYLAWYQQKPGQPPKLLIYWASTRESGVPDRFSGSGSGTDFTLTISSLQAEDVAVYYCQQYYSTPCSFGQGTKVEIK | QVQLVQSGAEVKKPGASVKVSCKASGYTFTNYDINWVRQATGQGLEWMGWVHPNSGNTGYAQKFQGRVTMTRNTSISTAYMELSSLRSEDTAVYYCAGSSGWYYFDYWGQGTLVTVSS |
| | | | SEQ ID NO: 26200 | SEQ ID NO: 30206 |
| **iPS:435371** | **21-225_149A3** | NA | GACATCCAGATGACCCAGTCTCCATCCTCCCTGTCTGCATCTGTAGGAGACAGAGTCACCATCACTTGCCGGGCAAGTCAGAGCATTAGCAGTTATTTAAATTGGTATCAGCAGAAACCAGGGAAAGCCCCTAAGGTCATGATCTATACTGCATCCAGTTTGCAAAGTGGGGTCCCATCAAGGTTCAGTGGCAGTGGATCTGGGACAGATTTCACTCTCACCATCAACAGTCTGCAACCTGAAGATTTTGCAACTTACTACTGTCAACAGAGTTACAGTATCCCCACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAACTATGCCATGACCTGGGTCCGCCAGGCTCCAGGGAAGGGGCTGGAGTGGGTCTCAGCTATTAGTGGTCGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTACGAAACGGGTGACGGACTACGGTGGTAACGACTGGTTCGACCCCTGGGGCCAGGGAACCCTGGTCACCGTCTCCTCA |
| | | | SEQ ID NO: 26201 | SEQ ID NO: 30207 |

FIGURE 50

| | | | | |
|---|---|---|---|---|
| | | AA | DIQMTQSPSSLSASVGDRVTITCRASQSISSYLNWYQQKPGKAPKVMIYTASSLQSGVPSRFSGSGSGTDFTLTINSLQPEDFATYYCQQSYSIPTFGGGTKVEIK | EVQLLESGGGLVQPGGSLRLSCAASGFTFSNYAMTWVRQAPGKGLEWVSAISGRGGNTFYADSVKGRFTISRDNSKNTLYLQMNSLRAEDTAVYYCTKRVTDYGGNDWFDPWGQGTLVTVSS |
| | | | SEQ ID NO: 26202 | SEQ ID NO: 30208 |
| **iPS:435373** | **21-225_149E3** | NA | GACATCGTGATGACCCAGTCTCCAGACTCCCTGGCTGTGTCTCTGGGCGAGAGGGCCACCATCAGCTGCAAGTCCAGCCAGACTGTTTTACACAACTCCAATAATCACAATTACTTTGCTTGGTACCAGCAGAAACCAGGACAGCCTCCTAAGCTGCTCATTTACTGGGCATCTACCCTGAGATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGACATCTGAGGACACGGCCGTGTATTACTGTGCATATAGCAGTGGCTGGTACTGGTTTGACTACTGGGGCCAGGGAACCCTGGTCACCGTCTCCTCA |
| | | | SEQ ID NO: 26203 | SEQ ID NO: 30209 |
| | | AA | DIVMTQSPDSLAVSLGERATISCKSSQTVLHNSNNHNYFAWYQQKPGQPPKLLIYWASTLRSGVPDRFSGSGSGTDFTLTISSLQAEDVAVYYCQQYYSTPPTFGQGTKVEIK | QVQLVQSGAEVKKPGASVKVSCKASGYTFTNYDINWVRQATGQGLEWMGWMHPNSGNTGYAQKFQGRVTMTRNTSISTAYMELSSLTSEDTAVYYCAYSSGWYWFDYWGQGTLVTVSS |
| | | | SEQ ID NO: 26204 | SEQ ID NO: 30210 |

FIGURE 50

| | | | | |
|---|---|---|---|---|
| **iPS:435375** | **21-225_149H4** | NA | GACATCGTGATGACCCAGTCTCCAGACTCCCT GGCTGTATCTCTGGGCGAGAGGGCCACCATCA ACTGCAAGTCCAGCCAGAGTGTTTTATCCAGC TCCAACGATAACAACTACTTAGCTTGGTACCA ACAGAAACCAGGACGGCCTCCTAAGCTGCTCA TTTACTGGTCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCACCAA TATTATAGTTATCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGACTATGCACAGAAGTTCCAGGGCAG AGTCACCATGACCAGGAACACCTCCATAACCAC AGTTTACATGGAGCTGAGCAGCCTGAGATCTGAG GACACGGCCGTGTATTATTGTACGTTTAGCAGTG GCTGGTACTACTTTGACTACTGGGGCCAGGGAAC CCTGGTCACCGTCTCCTCA |
| | | | SEQ ID NO: 26205 | SEQ ID NO: 30211 |
| | | AA | DIVMTQSPDSLAVSLGERATINCKSSQSVLSSSN DNNYLAWYQQKPGRPPKLLIYWSSTRESGVPDR FSGSGSGTDFTLTISSLQAEDVAVYYCHQYYSYP PTFGQGTKVEIK | QVQLVQSGAEVKKPGASVKVSCKASGYTFTNYDIN WVRQATGQGLEWMGWMHPNSGNTDYAQKFQGR VTMTRNTSITTVYMELSSLRSEDTAVYYCTFSSGW YYFDYWGQGTLVTVSS |
| | | | SEQ ID NO: 26206 | SEQ ID NO: 30212 |
| **iPS:435377** | **21-225_149G5** | NA | GACATCCAGATGACCCAGTCTCCTTCCTCCCT GTCTGCATCTGTAGGAGACAGAGTCACCATCA CTTGCCGGGCAAGTCAGGGCATTAGAAATGCC TTAGGCTGGTTTCAGCAGAAACCAGGGAAAGC CCCTAAGCGCCTGATCTATGCTGCATCCAGTTT GCAAAGTGGGGTCCCATCAAGGTTCAGCGGCA GTGGATCTGGGACAGAATTCACTCTCACAATC AGCAGCCTGCAGCCTGAAGATTTTGCAACTTA TTACTGTCTACAGCATAATAGTTACCCTCTCAC TTTCGGCGGAGGGACCAAGGTGGAGATCAAA | CAGGTGCAGCTGCAGGAGTCGGGCCCAGGACTG GTGAAGCCTTCACAGACCCTGTCCCTCACCTGCA CTGTCTCTGGTGGCTCCATCAGCAATGGTGGTTA CTACTGGAACTGGATCCGCCAGCACCCAGGGAA GGGCCTGGAGTGGATTGGGTACATCTATTACAGT GGGAGCACCTACTACAACCCGTCCCTCAAGAGTC GAGTTACCATATCAGTAGACACGTCTAAGGACCA GTTCTCCCTGAGGCTGAGCTCTGTGACTGCCGCG GACACGGCCGTGTATTACTGTGCGAGGTACAGTA CCTACGACTACTACTACGGTATGGACGTCTGGGG CCAAGGGACCACGGTCACCGTCTCCTCA |
| | | | SEQ ID NO: 26207 | SEQ ID NO: 30213 |

FIGURE 50

| | | | | |
|---|---|---|---|---|
| | | AA | DIQMTQSPSSLSASVGDRVTITCRASQGIRNALGWFQQKPGKAPKRLIYAASSLQSGVPSRFSGSGSGTEFTLTISSLQPEDFATYYCLQHNSYPLTFGGGTKVEIK | QVQLQESGPGLVKPSQTLSLTCTVSGGSISNGGYYWNWIRQHPGKGLEWIGYIYYSGSTYYNPSLKSRVTISVDTSKDQFSLRLSSVTAADTAVYYCARYSTYDYYYGMDVWGQGTTVTVSS |
| | | | SEQ ID NO: 26208 | SEQ ID NO: 30214 |
| iPS:435379 | 21-225_149B6 | NA | GACATCCAGATGACCCAGTCTCCATCTTCCGTGTCGGCATCTGTAGGAGACAGAGTCACCATCACTTGTCGGGCGAGTCAGGGTATTATCAGTTGGTTAGCCTGGTATCAGCAGAAACCAGGAAAAGCCCCTAAGCTCCTGATCTATGCTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGGGTAACAGTTTCCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTATTAGTGGTAGTGGTGGTAGCACATTCTACGCAGACTCCGTGAAGGGCCGGTTCACCATCTCCAGAGACAATTCCAAGAACACGCTGTTTCTGCAAATGAACAGCCTGAGAGCCGAGGACACGGCCGTATATTACTGTGCGAAACGAACTCCGGAAGATGTTTTTGATATCTGGGGCCAAGGGACAATGGTCACCGTCTCTTCA |
| | | | SEQ ID NO: 26209 | SEQ ID NO: 30215 |
| | | AA | DIQMTQSPSSVSASVGDRVTITCRASQGIISWLAWYQQKPGKAPKLLIYAASSLQSGVPSRFSGSGSGTDFTLTISSLQPEDFATYYCQQGNSFPFTFGPGTKVDIK | EVQLLESGGGLVQPGGSLRLSCAASGFTFSSYAMSWVRQAPGKGLEWVSVISGSGGSTFYADSVKGRFTISRDNSKNTLFLQMNSLRAEDTAVYYCAKRTPEDVFDIWGQGTMVTVSS |
| | | | SEQ ID NO: 26210 | SEQ ID NO: 30216 |

FIGURE 50

| | | | | |
|---|---|---|---|---|
| **iPS:435381** | **21-225_149C6** | NA | GACATCCAGATGACCCAGTCTCCAGCTTCCGTGTCTGCATCTGTAGGAGACAGAGTCACCATAACTTGTCGGGCGAGTCAGGGTATTAGCAACTGGTTAGCCTGGTATCAGCAGAAACCAGGGAGAGCCCCTAAGCTCCTGATCTATGCTGCATCCAGTTTGCAAGGTGGGGTCCCATCAAGGTTCAGCGGCAGTGGATCTGGGACAGATTACACTCTCTCCATCAGCAGCCTGCAGCCTGAAGATTTTGCAACTTACTATTGTCAACAGACTGACAGTTTCCCATTCACTTTCGGCCCTGGGACCAAAGTGGATATCAAA | GAGGTGCAGCTGTTGGAGTCTGGGGGAGGCTTAGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTAGTGGTGGTAACACATTCTACGCAGACTCCGTGAAGGGCCGCTTCACCATCTCCAGAGACAATTCCAAGAACACGCTGTATCTGCACATGAACAGCCTGAGAGCCGAGGACACGGCCGTTTATTACTGTGCGAAAAAGGATTATGATTACGTTTGGGGGAGTCCTTACTTTGACTACTGGGGCCAGGGAACCCTGGTCACCGTCTCATCA |
| | | | SEQ ID NO: 26211 | SEQ ID NO: 30217 |
| | | AA | DIQMTQSPASVSASVGDRVTITCRASQGISNWLAWYQQKPGRAPKLLIYAASSLQGGVPSRFSGSGSGTDYTLSISSLQPEDFATYYCQQTDSFPFTFGPGTKVDIK | EVQLLESGGGLVQPGGSLRLSCAASGFTFSSYAMSWVRQAPGKGLEWVSAISGSGGNTFYADSVKGRFTISRDNSKNTLYLHMNSLRAEDTAVYYCAKKDYDYVWGSPYFDYWGQGTLVTVSS |
| | | | SEQ ID NO: 26212 | SEQ ID NO: 30218 |
| **iPS:435383** | **21-225_149D7** | NA | GAAATTGTGTTGACGCAGTCTCCAGGCACCCTGTCTTTGTTTCCAGGGGAAAGAGTCACCCTCTCCTGCAGGGCCAGTCAGAGTATTATCAGCAACTACTTAGCCTGGTACCAGCAGAAACCTGGCCAGGCTCCCAGGCTCCTCATCTTTGGTGTATCTAGTAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAACAATATGGTCGGTCACCATTCAATTTCGGCCCTGGGACCAAAGTGGATATCAAA | CAGGTGCAGCTGCAGGAGTCGGGCCCAGGACTGGTGAAGCCTTCACAGACCCTGTCCCTCAATTGCACTGTCTCTGGTGGCTCCATCAGCAACAGTGGTTACTACTGGAGCTGGATCCGCCAGCACCCAGGGAAGGGCCTGGAATGGATTGGGTACAGCTATTACAGTGGGAGCACCTACTACAACCCGTCCCTCAAGAGTCGAATTACCATATCAGTAGACACGTCTAACAACCAGTTCTCCCTGAAGCTGAGCTCTGTGACTGCCGCGGACACGGCCGTGTATTACTGTGCGAGAGGGGGATATAACTGGAACCATGCTTTTGATATCTGGGGCCAAGGGACAATGGTCATCGTCTCTTCA |
| | | | SEQ ID NO: 26213 | SEQ ID NO: 30219 |

FIGURE 50

| | | | | |
|---|---|---|---|---|
| | | AA | EIVLTQSPGTLSLFPGERVTLSCRASQSIISNYLAWYQQKPGQAPRLLIFGVSSRATGIPDRFSGSGSGTDFTLTISRLEPEDFAVYYCQQYGRSPFNFGPGTKVDIK | QVQLQESGPGLVKPSQTLSLNCTVSGGSISNSGYYWSWIRQHPGKGLEWIGYSYYSGSTYYNPSLKSRITISVDTSNNQFSLKLSSVTAADTAVYYCARGGYNWNHAFDIWGQGTMVIVSS |
| | | | SEQ ID NO: 26214 | SEQ ID NO: 30220 |
| iPS:435391 | 21-225_149F8 | NA | GACATCCAGATGACCCAGTCTCCAGCTTCCGTGTCTGCATCTGTAGGAGACAGAGTCACCATAACTTGTCGGGCGAGTCAGGGTATTAGCAACTGGTTAGCCTGGTATCAGCAGAAACCAGGGAAAGCCCCTAAGCTCCTGATCTATGCTGCATCCAGTTTGCAAAGTGGGGTCCCATCAAGGTTCAGCGGCAATGAATCTGGGACAGATTTCACTCTCTCCATCAGCAGCCTGCAGCCTGAAGATTTTGCAATTTACTATTGTCAACAGACTGACAGTTTCCCATTCACTTTCGGCCCTGGGACCAAAGTGGATGTCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTCGTGGTGGTAACACATTCTACGCAGACTCCGTGAAGGGCCGCTTCACCATCTCCAGAGACAATTCCAAGAACACGCTCTATCTGCAAATGAACAGCCTGAGAGCCGAGGACACGGCCGTATATTACTGTGCGAAAAAGGATTATGATTACGTTTGGGGGAGTCCTTACTTTGACTACTGGGGCCAGGGAACCCTGGTCACCGTCTCCTCA |
| | | | SEQ ID NO: 26215 | SEQ ID NO: 30221 |
| | | AA | DIQMTQSPASVSASVGDRVTITCRASQGISNWLAWYQQKPGKAPKLLIYAASSLQSGVPSRFSGNESGTDFTLSISSLQPEDFAIYYCQQTDSFPFTFGPGTKVDVK | EVQLLESGGGLVQPGGSLRLSCAASGFTFSSYAMSWVRQAPGKGLEWVSAISGRGGNTFYADSVKGRFTISRDNSKNTLYLQMNSLRAEDTAVYYCAKKDYDYVWGSPYFDYWGQGTLVTVSS |
| | | | SEQ ID NO: 26216 | SEQ ID NO: 30222 |

FIGURE 50

| | | | | |
|---|---|---|---|---|
| iPS:435393 | 21-225_149D10 | NA | GACATCCAGATGACCCAGTCTCCATCCTCCCTGTCTGCATCTGTAGGAGACAGAGTCACCATCACTTGCCGGGCAAGTCGGGGCATTAGAAATGATTTAGGCTGGTATCAGCAGAAACCAGGGAAAGCCCCTAAGCGCCTGATCTATGCTGCATCCAGTTTGCAGAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTATCCTCGGACGTTCGGCCAAGGGACCAAGGTGGAAATCAAA | CAGGTGCAGCTGGTGGAGTCTGGGGGAGGCGTGGCCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGGAAGTAATAAGTACTATGCAGACTCCGTGAAGGGCCGACTCACCATCTCCAGAGACAATTCCAAGAACACGCTGTATCTGCAAATGAACAGCCTGAGAGCCGAGGACACGGCTGTGTATTACTGTGCGAGAGAGAGGTATAGCAGTGGCTGGTACGACTACGGTATGGACGTCTGGGGCCAAGGGACCACGGTCACCGTCTCCTCA |
| | | | SEQ ID NO: 26217 | SEQ ID NO: 30223 |
| | | AA | DIQMTQSPSSLSASVGDRVTITCRASRGIRNDLGWYQQKPGKAPKRLIYAASSLQSGVPSRFSGSGSGTEFTLTISSLQPEDFATYYCLQHYSYPRTFGQGTKVEIK | QVQLVESGGGVAQPGRSLRLSCAASGFTFSDYGMHWVRQAPGKGLEWVAVIWYDGSNKYYADSVKGRLTISRDNSKNTLYLQMNSLRAEDTAVYYCARERYSSGWYDYGMDVWGQGTTVTVSS |
| | | | SEQ ID NO: 26218 | SEQ ID NO: 30224 |
| iPS:435395 | 21-225_149D11 | NA | GACATCCAGATGACCCAGTCTCCAGCTTCCGTGTCTGCATCTGTAGGAGACAGAGTCACCATAACTTGTCGGGCGAGTCAGAATATTAGCAACTGGTTAACCTGGTATCAGCAGAAACCAGGGAAAGCCCCTAAGCTCCTGATCTATGCTGCATCCAGTTTGCAAAGTGGGGTCCCATCAAGGTTCAGCGGCAGTGGATCTGGGACAGATTTCACTCTCTCCATCAGCAGCCTGCAGCCTGAAGATTTTGCAACTTACTATTGTCAACAGACTGACAGTTTCCCATTCACTTTCGGCCCTGGGACCAAAGTGGATGTCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTCGTGGTGGTAACACATTCTACGCAGACTCCGTGAAGGGCCGCTTCACCATCTCCAGAGACAATTCCAAGAACACGCTCTATCGGCAAATGAACAGCCTGAGAGCCGAGGACACGGCCGTATATTACTGTGCGAAAAAGGATTATGATTACGTTTGGGGGAGTCCTTACTTTGACTACTGGGGCCAGGGAACCCTGGTCACCGTCTCCTCA |
| | | | SEQ ID NO: 26219 | SEQ ID NO: 30225 |

FIGURE 50

| | | | | |
|---|---|---|---|---|
| | | AA | DIQMTQSPASVSASVGDRVTITCRASQNISNWLTWYQQKPGKAPKLLIYAASSLQSGVPSRFSGSGSGTDFTLSISSLQPEDFATYYCQQTDSFPFTFGPGTKVDVK | EVQLLESGGGLVQPGGSLRLSCAASGFTFSSYAMSWVRQAPGKGLEWVSAISGRGGNTFYADSVKGRFTISRDNSKNTLYRQMNSLRAEDTAVYYCAKKDYDYVWGSPYFDYWGQGTLVTVSS |
| | | | SEQ ID NO: 26220 | SEQ ID NO: 30226 |
| iPS:435397 | 21-225_149F12 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAATTTGCAAAGTGGGGTCCCATCAAGGTTCAGCGGCAGTGGATCTGGGACAGAATTCACTCTCACAATCAGCAGCCTGCAGCCTGAAGATTTTGCAATTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGAAGAAAATAATAAATACTATGCAGACTCCGTGAAGGGCCGATTCACCATCTCCAGAGACAATTCCAAGAACACGCTGTTTCTGCAAATGAACAGCCTGAGAGCCGAGGACACGGCTGTGTATTACTGTGCGAGAGAAATAGGGTTCAGTGAGGACTACTGGGGCCAGGGAACCCTGGTCACCGTCTCCTCA |
| | | | SEQ ID NO: 26221 | SEQ ID NO: 30227 |
| | | AA | DIQMTQSPSSLSASVGDRVTITCRASQGIRNDLGWYQQKPGKAPKRLIYAASNLQSGVPSRFSGSGSGTEFTLTISSLQPEDFAIYYCLQHNSYPLTFGGGTKVEIK | QVQLVESGGGVVQPGRSLRLSCAASGFTFSDYGMHWVRQAPGKGLEWVAVIWYEENNKYYADSVKGRFTISRDNSKNTLFLQMNSLRAEDTAVYYCAREIGFSEDYWGQGTLVTVSS |
| | | | SEQ ID NO: 26222 | SEQ ID NO: 30228 |

FIGURE 50

| | | | | |
|---|---|---|---|---|
| **iPS:435399** | **21-225_150D2** | NA | GACATCGTGATGACCCAGTCTCCAGACTCCCTGGCTGTGTCTCTGGGCGAGAGGGCCACCATCAAGTGCAAGTCCAGCCAGAGTGTTTTATACAGATCCAACAGTAAGAAATACTTAACTTGGTACCAGCAGAAACCAGGACAGCCTCCTAAGCTGTTCATTTATTGGGCATCTACCCGGAAATCCGGGGTCCCTGACCGATTCAGTGGCAGCGGGTCTGGGACAGATTTCACTCTCACCATCAGCAACCTGCAGGCTGAAGATGTGGCAGTTTATTTCTGTCAGCAATATTTTAGTACTCCGTACAATTTTGGCCAGGGGACCAAGAGGGAGATCAAA | CAGGTGCAGCTGGTGCAATCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCTTATAGCAGTGGCTGGTACTACTTTGACTACTGGGGCCAGGGAACCCTGGTCACCGTCTCCTCA |
| | | | SEQ ID NO: 26223 | SEQ ID NO: 30229 |
| | | AA | DIVMTQSPDSLAVSLGERATIKCKSSQSVLYRSNSKKYLTWYQQKPGQPPKLFIYWASTRKSGVPDRFSGSGSGTDFTLTISNLQAEDVAVYFCQQYFSTPYNFGQGTKREIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAYSSGWYYFDYWGQGTLVTVSS |
| | | | SEQ ID NO: 26224 | SEQ ID NO: 30230 |
| **iPS:435401** | **21-225_150E2** | NA | GACATCCAGATGACCCAGTCTCCATCCTCCCTGTCTGCATCTGTGGGAGACAGAGTCACCATCTCTTGCCGGGCAAGTCAGGGCATTGGAAATGATTTAGGCTGGTATCAGCAGAAACCAGGGAAAGCCCCTACGCGCCTGATCTATGCTGCATCCAGTTTGCAAAGTGGGGTCCCATCAAGGTTCAGCGGCAGTGGATCTGGGACAGAATTCACTCTCACTATCAGCAGCCTGCAGCCTGCAGATTTTGCAACTTATTACTGTCTACAACATTATAGTTTCCCGTACAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGGAGTATAGCAGCAGCTGGTACGGGTACGGTATGGACGTCTGGGGCCAAGGGACCACGGTCACCGTCTCCTCA |
| | | | SEQ ID NO: 26225 | SEQ ID NO: 30231 |

FIGURE 50

| | | | | |
|---|---|---|---|---|
| | | AA | DIQMTQSPSSLSASVGDRVTISCRASQGIGNDLGWYQQKPGKAPTRLIYAASSLQSGVPSRFSGSGSGTEFTLTISSLQPADFATYYCLQHYSFPYSFGQGTKLEIK | QVQLVESGGGVVQPGRSLRLSCAASGFTFSSYVMHWVRQAPGKGLEWVAVIWYDGSNKYYADSVKGRFTISRDNSKNTLYLQMNSLRAEDTAVYYCAREEYSSSWYGYGMDVWGQGTTVTVSS |
| | | | SEQ ID NO: 26226 | SEQ ID NO: 30232 |
| iPS:435403 | 21-225_150C5 | NA | GACATCCAGATGACCCAGTCTCCAGGTTCCGTGTCTGCATCTGTAGGAGACAGAGTCACCATAACTTGTCGGGCGAGTCAGGGTATTAACAACTGGTTAGCCTGGTATCAGCAGAAACCAGGGAAAGCCCCTAAGCTCCTGATCTATGCTGCATCCAGTTTGCAAGGTGGGGTCCCATCAAGGTTCAGCGGCAGTGGATCTGGGACAGATTTCACTCTCTCCATCAGCAGCCTGCAGCCTGAAGATTTTGCAACTTACTATTGTCAACAGACTGACAGTTTCCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTAGTGGTGGTAACACATTCTACGCAGACTCCGTGAAGGGCCGCTTCACCATCTCCAGAGACAATTCCAAGAACACGCTGTATCTGCAAATGAACAGCCTGAGAGCCGAGGACACGGCCGTATATTACTGTGCGAAAAAGGATTATGATTACGTTTGGGGGAGTCCTTACTTTGACTACTGGGGCCAGGGAACCCTGGTCACCGTCTCCTCA |
| | | | SEQ ID NO: 26227 | SEQ ID NO: 30233 |
| | | AA | DIQMTQSPGSVSASVGDRVTITCRASQGINNWLAWYQQKPGKAPKLLIYAASSLQGGVPSRFSGSGSGTDFTLSISSLQPEDFATYYCQQTDSFPFTFGPGTKVDIK | EVQLLESGGGLVQPGGSLRLSCAASGFTFSSYAMSWVRQAPGKGLEWVSAISGSGGNTFYADSVKGRFTISRDNSKNTLYLQMNSLRAEDTAVYYCAKKDYDYVWGSPYFDYWGQGTLVTVSS |
| | | | SEQ ID NO: 26228 | SEQ ID NO: 30234 |

FIGURE 50

| | | | | |
|---|---|---|---|---|
| **iPS:435405** | **21-225_150B7** | NA | GACATCGTGATGACCCAGTCTCCAGACTCCCTGGCTGTGTCTCTGGGCGAGAGGGCCACCATCAACTGCAAGTCCAGCCAGAATGTTTTATACAGCTCCCACAATAACAACTACTTAGCTTGGTACCAGCAGAAACCAGGGCAGCCTCCTAAACTACTCATTTACTGGGCATCTACCCGGAAATCCGGGGTCCCTGACCGATTCAGTGGCAGCGGGTCTGGGACAGATTTCACTCTCACCATCACCAGCCTGCAGGCTGAAGATGTGGCAGTTTATTACTGTCAGCAATATTATAGTACTCCATTCACTTTCGGCCCTGGGACCAAAGTGGATATCAAA | CAGGTGCAGCTGGTGCAGTCTGGGGCTGAGGTAAAGAAGCCTGGGGCCTCAGTGACGGTCTCCTGCAAGGCTTCTGGATTCCCCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACTTGGAGCTGAGCAGCCTGAGATCTGAGGACACGGCCGTGTATTACTGTGCGAGTAGCAGTGGCTGGTACTTTTTTGACTACTGGGGCCAGGGAACCCTGGTCACCGTCTCCTCA |
| | | | SEQ ID NO: 26229 | SEQ ID NO: 30235 |
| | | AA | DIVMTQSPDSLAVSLGERATINCKSSQNVLYSSHNNNYLAWYQQKPGQPPKLLIYWASTRKSGVPDRFSGSGSGTDFTLTITSLQAEDVAVYYCQQYYSTPFTFGPGTKVDIK | QVQLVQSGAEVKKPGASVTVSCKASGFPFTNYDINWVRQATGQGLEWMGWMHPNSGNTGYAQKFQGRVTMTRNTSISTAYLELSSLRSEDTAVYYCASSSGWYFFDYWGQGTLVTVSS |
| | | | SEQ ID NO: 26230 | SEQ ID NO: 30236 |
| **iPS:435407** | **21-225_150E7** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAATTTGCAAAGTGGGGTCCCATTAAGGTTCAGCGGCAGTGGATCTGGGACAGACTTCACTCTCACAATCAGCAGCCTGCAGCCTGAAGATTTTGCAGCTTATTACTGTCTACAGCATAATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGAAGAAAATAATAAATACTATGCAGACTCCGTGAAGGGCCGATTCACCATCTCCAGAGACAATTCCAAGAACACGCTGTTTCTGCAAATGAACAGCCTGAGAGCCGAGGACACGGCTGTGTATTACTGTGCGAGAGAAATAGGGTTCAGTGAGGACTACTGGGGCCAGGGAACCCTGGTCACCGTCTCCTCA |
| | | | SEQ ID NO: 26231 | SEQ ID NO: 30237 |

FIGURE 50

| | | | | |
|---|---|---|---|---|
| | | AA | DIQMTQSPSSLSASVGDRVTITCRASQGIRNDLGWYQQKPGKAPKRLIYAASNLQSGVPLRFSGSGSGTDFTLTISSLQPEDFAAYYCLQHNSYPLTFGGGTKVEIK | QVQLVESGGGVVQPGRSLRLSCAASGFTFSDYGMHWVRQAPGKGLEWVAVIWYEENNKYYADSVKGRFTISRDNSKNTLFLQMNSLRAEDTAVYYCAREIGFSEDYWGQGTLVTVSS |
| | | | SEQ ID NO: 26232 | SEQ ID NO: 30238 |
| iPS:435409 | 21-225_150G8 | NA | GACATCCAGATGACCCAGTCTCCATCCTCACTGTCTGCATCTGTAGGAGACAGAGTCACCATCACTTGTCGGGCGAGTCAGGGCATTAGCCATTATTTAGCCTGGTTTCAGCAGAAACCAGGGAAAGCCCCTAAGTCCCTGATCTATGTTGCATCCAGTTTGCAAAATGGGGTCCCATCAAGGTTCAGCGGCAGTGGATCTGGGACAGATTTCACTCTCACCATCAGCAGCCTGCAGCCTGAAGATTTTGCAACTTATTACTGCCAACAGTATAATAATTACCCGCTCACTTTCGGCGGAGGGACCAAGGTGGAGATCAAA | GAGGTGCAGCTGGTGGAGTCTGGGGGAGGCTTGGTGCAGCCTGGGGGGTCCCTGAGACTCTCCTGTGCAGCCTCGGGATTTACTTTCAGTACCTATAGCATGACTTGGGTCCGCCAGGCTCCAGGGAAGGGGCTGGACTGGGTTTCATACATTAGTAGGAGTAGTAGTACCATATACTACGCAGACTCTGTGAAGGGCCGATTCTCCATCTCCAGAGACAATGCCAAGAACTCACTGTATCTGCAAATGAACAGCCTGAGAGACGAGGACACGGCTCTGTATTACTGTGCGAGATCGGCATTTAGCCCTTTTGATTACTGGGGCCAGGGAACCCTGGTCACCGTCTCCTCA |
| | | | SEQ ID NO: 26233 | SEQ ID NO: 30239 |
| | | AA | DIQMTQSPSSLSASVGDRVTITCRASQGISHYLAWFQQKPGKAPKSLIYVASSLQNGVPSRFSGSGSGTDFTLTISSLQPEDFATYYCQQYNNYPLTFGGGTKVEIK | EVQLVESGGGLVQPGGSLRLSCAASGFTFSTYSMTWVRQAPGKGLDWVSYISRSSSTIYYADSVKGRFSISRDNAKNSLYLQMNSLRDEDTALYYCARSAFSPFDYWGQGTLVTVSS |
| | | | SEQ ID NO: 26234 | SEQ ID NO: 30240 |

FIGURE 50

| | | | | |
|---|---|---|---|---|
| **iPS:435413** | **21-225_150B11** | NA | GATATTGTGATGACCCAGACTCCACTCTCTCTGTCCGTCACCCCTGGACAGCCGGCCTCCATCTCCTGCAAGTCTAGTCAGAGCCTCGTGCATGGTGATGGAAAGACCTATTTGTATTGGTACCTGCAGAGGCCAGGCCAGCCTCCACAGCTCCTGATCTATGAAGTTTCCAACCGGTTCTCTGGAGTGCCAGATAGGTTCAGTGGCAGCGGGTCAGGGACAGATTTCACATTGAAAATCAGCCGGGTGGAGGCTGAGGATGTTGGGGTTTATTACTGCATGCAAAGTATACAGCTTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAATTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAAGACACGGCTGTGTATTACTGTGCGAGAGATCGTTACGATTTTTGGAGTGGTCACTTTGACTACTGGGGCCAGGGAACCCTGGTCACCGTCTCCTCA |
| | | | SEQ ID NO: 26235 | SEQ ID NO: 30241 |
| | | AA | DIVMTQTPLSLSVTPGQPASISCKSSQSLVHGDGKTYLYWYLQRPGQPPQLLIYEVSNRFSGVPDRFSGSGSGTDFTLKISRVEAEDVGVYYCMQSIQLPWTFGQGTKVEIK | QVQLVESGGGVVQPGRSLRLSCAASGFTFSSYGMHWVRQAPGKGLEWVAIIWYDGSNKYYADSVKGRFTISRDNSKNTLYLQMNSLRAEDTAVYYCARDRYDFWSGHFDYWGQGTLVTVSS |
| | | | SEQ ID NO: 26236 | SEQ ID NO: 30242 |
| **iPS:435415** | **21-225_150C11** | NA | GACATCCAGATGACCCAGTCTCCATCCTCCCTGTCTGCATCTGTAGGAGACAGAGTCACCATCACTTGCCGGGCAAGTCAGAGCATTAGCAGCTATTTAAATTGGTATCAGCAGAAACCAGGGAAAGCCCCTAAACTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACAGTATTTACACTTTCGGCGGAGGGTCCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAACTGGGTCCGCCAGGCTCCAGGGAAGGGGCTGGAGTGGGTCTCAGCTATTAGTGGTAGTGGTGGTAACACATTCTACGCAGACTCCGTGAAGGGCCGGTTCACCATCTCCAGAGACAATTCCAAGAACACGCTGAATCTGCAAATGAGCAGCCTGAGAGCCGAGGACACGGCCGTATATTACTGCGCGAAACGGGTGACGGACTACGGTGGTAACGACTGGTTCGACCCCTGGGGCCAGGGAACCCTGGTCACCGTCTCCTCA |
| | | | SEQ ID NO: 26237 | SEQ ID NO: 30243 |

FIGURE 50

| | | | | |
|---|---|---|---|---|
| | | AA | DIQMTQSPSSLSASVGDRVTITCRASQSISSYLNWYQQKPGKAPKLLIYTASSLQSGVPSRFSGSGSGTDFTLTISSLQPEDFATYYCQQSYSIYTFGGGSKVEIK | EVQLLESGGGLVQPGGSLRLSCAASGFTFSSYAMNWVRQAPGKGLEWVSAISGSGGNTFYADSVKGRFTISRDNSKNTLNLQMSSLRAEDTAVYYCAKRVTDYGGNDWFDPWGQGTLVTVSS |
| | | | SEQ ID NO: 26238 | SEQ ID NO: 30244 |
| **iPS:435417** | **21-225_150D11** | NA | GATATTGTGATGACCCAGACTCCACTCTCTCTGTCCGTCACTCCTGGACAGCCGGCCTCCATCTCCTGCAAGTCTAGTCAGAGCCTCCTGCATAGTGAGGGAAAGACCTATTTGTATTGGTACCTGCAGAAGCCAGGCCAGCCTCCACAGCTCCTGATCTATGAAGTTTCCTACCGGTTCTCTGGAGTGCCAGATAGGTTCAGTGGCAGCGGGTCAGGGACAGATTTCACACTGAAAATCAGCCGGGTGGAGGCTGAGGATGTTGGGGTTTATTACTGCATGCAAGGTATACAGCTTCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATTCTATGATGGAAGTAATAAACACTATGCAGACTCCGTGAAGGGCCGATTCACCATCTCCAGAGACAATTCCAAGAACACGCTGTATCTGCAAATGAACAGCCTGAGAGCCGAGGACTCGGCTGTGTATTACTGTACGAGGAGGTTTAGCAGCAGCTGGTCGGGGGGTATGGACGTCTGGGGCCAAGGGACCACGGTCACCGTCTCCTCA |
| | | | SEQ ID NO: 26239 | SEQ ID NO: 30245 |
| | | AA | DIVMTQTPLSLSVTPGQPASISCKSSQSLLHSEGKTYLYWYLQKPGQPPQLLIYEVSYRFSGVPDRFSGSGSGTDFTLKISRVEAEDVGVYYCMQGIQLPLTFGGGTKVEIK | QVQLVESGGGVVQPGRSLRLSCAASGFTFSSYGMHWVRQAPGKGLEWVAVIFYDGSNKHYADSVKGRFTISRDNSKNTLYLQMNSLRAEDSAVYYCTRRFSSSWSGGMDVWGQGTTVTVSS |
| | | | SEQ ID NO: 26240 | SEQ ID NO: 30246 |

FIGURE 50

| | | | | |
|---|---|---|---|---|
| **iPS:435419** | **21-225_150C12** | NA | GACATCCAGATGACCCAGTCTCCATCCTCCCTGTCTGCATCTGTAGGAGACAGAGTCACCATCACTTGCCGGGCAAGTCAGAGCATTAGCGACTATTTAAATTGGTATCAGCAGAAACCAGGGAAAGCCCCTAAGGTCCTGATCTATACTACATCCAGTTTGCAAAGTGGGGTCCCATCAAGGTTCAGTGGCAGTGGATCTGGGACAGATTTCACTCTCACCATCAGCAGTCTGCAGCCTGAAGATTTTGCAACTTACTACTGTCAACAGAGTTTCAGTACCCCCACTTTCGGCGGAGGGACCAAGGTGGAGATCAAA<br>SEQ ID NO: 26241 | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTCGTGGTGGTAACACATTCTACGCAGACTCCGTGAAGGGCCGGTTCACCATCTCCAGAGACAATTCCAAGAACACACTATATCTGCAAATGAACAGCCTGAGAGCCGAGGACACGGCCGTATATTACTGTGCGAAACGGGTGACGGACTACGGTGGTAACGACTGGTTCGACCCCTGGGGCCAGGGAACCCTGGTCACCGTCTCCTCA<br>SEQ ID NO: 30247 |
| | | AA | DIQMTQSPSSLSASVGDRVTITCRASQSISDYLNWYQQKPGKAPKVLIYTTSSLQSGVPSRFSGSGSGTDFTLTISSLQPEDFATYYCQQSFSTPTFGGGTKVEIK<br>SEQ ID NO: 26242 | EVQLLESGGGLVQPGGSLRLSCAASGFTFSSYAMSWVRQAPGKGLEWVSAISGRGGNTFYADSVKGRFTISRDNSKNTLYLQMNSLRAEDTAVYYCAKRVTDYGGNDWFDPWGQGTLVTVSS<br>SEQ ID NO: 30248 |
| **iPS:435421** | **21-225_151F1** | NA | GAAATGGTGATGACGCAGTCTCCAGCCACCCTGTCTGTGTCTCCAGGGGAAAGAGTCACCCTCTCCTGCAGGGCCAGTCAGAGTATTAACATCAATATAGCCTGGTACCAGCAGAAACCTGGCCAGGCTCCCAGGCTCCTCATCTATGGTGCATCCACCAGGGCCACTGGTATCCCAGCCAGGTTCAGTGGCAGTGGGTCTGGGACAGAGTTCACTCTCACCATCAGCAGCCTGCAGTCTGAAGATTTTGCAGTTTATTACTGTCAGCAGTATAATGACTGGCCTCCGTGGACGTTCGGCCAAGGGACCAAGGTGGAAATCAAA<br>SEQ ID NO: 26243 | GAGGTGCAGCTGGTGGAGTCTGGGGGAGGCCTGGTCAAGCCTGGGGGGTCCCTGAGACTCTCCTGTGCAGCCTCTGGATACACCTTCAGGAGCTTTAGCATGAACTGGGTCCGCCAGGCTCCAGGGAAGGGGCTGGAGTGGGTCTCATCCATTAGTAGTAGTAGTTATTACATATACTACGCAGACTCAGTGAAGGGCCGATTCACCATCTCCAGAGACAACGCCAAGAACTCACTGTATCTGCAAATGAACAGCCTGAGAGCCGAAGACACGGCTGTGTATTACTGTGCGAGAGATACACCACTGGTTTACTGGGGCCAGGGAACCCTGGTCACCGTCTCCTCA<br>SEQ ID NO: 30249 |

FIGURE 50

| | | | | |
|---|---|---|---|---|
| | | AA | EMVMTQSPATLSVSPGERVTLSCRASQSININIAWYQQKPGQAPRLLIYGASTRATGIPARFSGSGSGTEFTLTISSLQSEDFAVYYCQQYNDWPPWTFGQGTKVEIK | EVQLVESGGGLVKPGGSLRLSCAASGYTFRSFSMNWVRQAPGKGLEWVSSISSSSYYIYYADSVKGRFTISRDNAKNSLYLQMNSLRAEDTAVYYCARDTPLVYWGQGTLVTVSS |
| | | | SEQ ID NO: 26244 | SEQ ID NO: 30250 |
| **iPS:435423** | **21-225_151G5** | NA | GATATTGTGATGACCCAGACTCCACTCTCTCTGTCCGTCACCCCTGGACAGCCGGCCTCCATCTCCTGCAAGTCTAGTCAGCGCCTCCTGCATGGTGATGGAAAGACCTATTTGTATTGGTACCTGCAGAAGCCAGGCCAGCCTCCACAGCTCCTGCTCTATGAAGTTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGGTTTATTACTGCATGCAAAGTATACAGGTTCCGTGGACGTTCGGCCAAGGGACCAAGGTGGAAATCAAA | CAGGTGCAGCTGATGGAGTCTGGGGGAGGCGTGGTCCAGCCTGGGAGGTCCCTGAGACTCTCCTGTGCAGCGTCTGGATTCATCTTCAGTAGCTATGGCATGCACTGGGTCCGCCAGGCTCCAGGCAAGGGGCTGGAGTGGGTGGCAATTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGATACGATTTTTGGAGTGGTCACTTTGACTACTGGGGCCAGGGAACCCTGGTCACCGTCTCCTCA |
| | | | SEQ ID NO: 26245 | SEQ ID NO: 30251 |
| | | AA | DIVMTQTPLSLSVTPGQPASISCKSSQRLLHGDGKTYLYWYLQKPGQPPQLLLYEVSNRFSGVPDRFSGSGSGTDFTLKISRVEAEDVGVYYCMQSIQVPWTFGQGTKVEIK | QVQLMESGGGVVQPGRSLRLSCAASGFIFSSYGMHWVRQAPGKGLEWVAIIWYDGSNKYYADSVKGRFTISRDNSKNTLYLQMNSLRAEDTAVYYCARDRYDFWSGHFDYWGQGTLVTVSS |
| | | | SEQ ID NO: 26246 | SEQ ID NO: 30252 |

FIGURE 50

| | | | | |
|---|---|---|---|---|
| iPS:435425 | 21-225_151B12 | NA | GACATACAGATGACCCAGTCTCCATCCTCCCT<br>GTCTGCGTCTGTAGGAGACAGAGTCACCATCA<br>CTTGCCGGGCAAGTCAGAGCATTAGCAACTTT<br>TTAAATTGGTATCAGCAGAAACCAGGGAAAG<br>CCCCTAAGGTCCTGATCTATACTGCATCCAGTT<br>TGGAAAGTGGGGTCCCATCAAGGTTCAGTGGC<br>AGTGAATCTGGGACAGATTTCACTCTCACCAT<br>CAGCAGTCTGCAACCTGAAGATTTTGCAACTT<br>ACTACTGTCAACAGAGTTACAGTACCCCCACT<br>TTCGGCGGAGGGACCAGGGTGGAGATCAAA | GAGGTGCAGCTGTTGGAGTCTGGGGGAGGCTTG<br>GTACAGCCTGGGGGGTCCCTGAGACTCTCCTGTG<br>CAGCCTCTGGATTCACCTTTAGCAGCTATGCCAT<br>GAACTGGGTCCGCCACGCTCCAGGGAAGGGGCT<br>GGAGTGGGTCTCAGCTATTAGTGGTAGTGGTAAA<br>AACACATTCTACGCAGACTCCGTGAAGGGCCGGT<br>TCACCATCTCCAGAGACAATTCCAAGAACACGCT<br>GTATCTGCAAATGAACAGCCTGAGAGCCGAGGA<br>CACGGCCGTATATTACTGTGCGAAACGGGTGACG<br>GACTACGGTGGTAACGACTGGTTCGACCCCTGGG<br>GCCAGGGAACCCTGGTCACCGTCTCCTCA |
| | | | SEQ ID NO: 26247 | SEQ ID NO: 30253 |
| | | AA | DIQMTQSPSSLSASVGDRVTITCRASQSISNFLNW<br>YQQKPGKAPKVLIYTASSLESGVPSRFSGSESGT<br>DFTLTISSLQPEDFATYYCQQSYSTPTFGGGTRV<br>EIK | EVQLLESGGGLVQPGGSLRLSCAASGFTFSSYAMN<br>WVRHAPGKGLEWVSAISGSGKNTFYADSVKGRFTI<br>SRDNSKNTLYLQMNSLRAEDTAVYYCAKRVTDYG<br>GNDWFDPWGQGTLVTVSS |
| | | | SEQ ID NO: 26248 | SEQ ID NO: 30254 |
| iPS:435427 | 21-225_151C9 | NA | GACATCCAGATGACCCAGTCTCCATCCTCACT<br>GTCTGCTTCTGTAGGAGACAGAGTCACCATCA<br>CTTGTCGGGCGAGTCAGGGCATTAGCAAGTAT<br>TTAGCCTGGTTTCAGCAGAAACCAGGGAAAGC<br>CCCTAAGTCTCTGATCTATGATGCATCCAGGTT<br>GCAAAGTGGGGTCCCATCAAAGTTCAGCGGCA<br>GTGGATCTGGGACAGATTTCACTCTCACCATC<br>AGCAGCCTGCAGCCAGAAGATTTTGCAAGTTA<br>TTACTGCCATCAGTATAAACATTACCCGATCA<br>CCTTCGGCCAAGGGACACGACTGGAGATTAAA | CAGGTGCAGCTGGTGGAGTCTGGGGGAGGCGTG<br>GTCCAGCCTGGGAGGTCCCTGAGACTCTCCTGTG<br>CAGCCTCTGGATTCACCTTCAGTAGCTATGGCAT<br>GCACTGGGTCCGCCAGGCTCCAGGCAAGGGGCT<br>GGAGTGGGTGGCAGTTATATCATATGATGGAAGT<br>AATAAATACTATGCAGACTCCGTGAAGGGCCGA<br>TTCACCATCTCCAGAGACAATTCCAAGAACACGC<br>TGTATCTGCAAATGAACAGCCTGAGAGCCGAGG<br>ACACGGCTGTGTATTACTGTGCGAGAGGGGTATT<br>ACTATGGTTCGGGGAGCTAGAAGATGACTGGTTC<br>GACCCCTGGGGCCAGGGAACCCTGGTCACCGTCT<br>CCTCA |
| | | | SEQ ID NO: 26249 | SEQ ID NO: 30255 |

FIGURE 50

| | | | | |
|---|---|---|---|---|
| | | AA | DIQMTQSPSSLSASVGDRVTITCRASQGISKYLAWFQQKPGKAPKSLIYDASRLQSGVPSKFSGSGSGTDFTLTISSLQPEDFASYYCHQYKHYPITFGQGTRLEIK | QVQLVESGGGVVQPGRSLRLSCAASGFTFSSYGMHWVRQAPGKGLEWVAVISYDGSNKYYADSVKGRFTISRDNSKNTLYLQMNSLRAEDTAVYYCARGVLLWFGELEDDWFDPWGQGTLVTVSS |
| | | | SEQ ID NO: 26250 | SEQ ID NO: 30256 |
| **iPS:435429** | **21-225_151A10** | NA | GATATTGTGATGACCCAGACTCCACTCTCTCTGTCCGTCACCCCTGGACAGCCGGCCTCCATCTCCTGCAAGTCTAGTCAGAGCCTCCTGCATGGTGATGGAAAGACCTATTTGTATTGGTACCTACAGAAGCCAGGCCAGCCTCCACAGCTCCTGATCTATGAAGTTTCCCACCGGTTCTCTGGAGTGCCAGATAGGTTCAGTGGCAGCGGGTCAGGGACAGATTTCACACTGAAAATCAGCCGGGTGGAGGCTGAGGATGTTGGGGTTTATTACTGCATGCAAAGTATACAGATTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGTTGGCAATTATATGGTATGATGGAAGTAATAAATACTATGCAGACTCCGTGAAGGGCCGATTCACCATCTCCAGAGACAATTCCAAGAACACGCTGTATCTACAAATGAACAGCCTGAGAGCCGAGGACACGGCTGTGTATTACTGTGCGAGAGATCATTACGATTTTTGGAGTGGTCACTTTGACTACTGGGGCCAGGGAACCCTGGTCACCGTCTCCTCA |
| | | | SEQ ID NO: 26251 | SEQ ID NO: 30257 |
| | | AA | DIVMTQTPLSLSVTPGQPASISCKSSQSLLHGDGKTYLYWYLQKPGQPPQLLIYEVSHRFSGVPDRFSGSGSGTDFTLKISRVEAEDVGVYYCMQSIQIPWTFGQGTKVEIK | QVQLVESGGGVVQPGRSLRLSCAASGFTFSNYGMHWVRQAPGKGLEWLAIIWYDGSNKYYADSVKGRFTISRDNSKNTLYLQMNSLRAEDTAVYYCARDHYDFWSGHFDYWGQGTLVTVSS |
| | | | SEQ ID NO: 26252 | SEQ ID NO: 30258 |

FIGURE 50

| | | | | |
|---|---|---|---|---|
| **iPS:435431** | **21-225_152D2** | NA | GACATCCAGATGACCCTGTCTCCATCCTCCCTGTCTGCATCTGTAGGAGACAGAGTCACCATCACTTGCCGGGCAAGTCAGAGCATTAGCGACTATTTAAATTGGTATCAGCTGAAACCAGGGAAAGCCCCTAAGGTCTTGATCTATACTACATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTTCTGTCAACAGAGTTACAGTACCCCCACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTCGTGGTGGTAACACATTCTACGCAGACTCCGTGAAGGGCCGGTTCACCATCTCCAGAGACAATTCCAAGAAAACACTATATCTGCAAATGAACAGCCTGAGAGCCGAGGACACGGCCGTATATTACTGTGCGAAACGGGTGACGGACTACGGTGGTAACGACTGGTTCGACCCCTGGGGCCAGGGAACCCTGGTCACCGTCTCCTCA |
| | | | SEQ ID NO: 26253 | SEQ ID NO: 30259 |
| | | AA | DIQMTLSPSSLSASVGDRVTITCRASQSISDYLNWYQLKPGKAPKVLIYTTSSLQSGVPSRFSGSGSGTDFTLTISSLQPEDFATYFCQQSYSTPTFGGGTKVEIK | EVQLLESGGGLVQPGGSLRLSCAASGFTFSSYAMSWVRQAPGKGLEWVSAISGRGGNTFYADSVKGRFTISRDNSKKTLYLQMNSLRAEDTAVYYCAKRVTDYGGNDWFDPWGQGTLVTVSS |
| | | | SEQ ID NO: 26254 | SEQ ID NO: 30260 |
| **iPS:435433** | **21-225_152E3** | NA | GACATCGTGATGACCCAGTCTCCAGACTCCCTGGCTGTGTCTCTGGGCGAGAGGGCCACCATCAACTGCAAGTCCAGCCAGAGTGTTTTACACAGCTCCAACAATTACAACTACTTAGTTTGGTACCAGCAGAAACCAGGACAGTCTCCTAAGCGGCTCATTTACTGGGCATCTACCCGGGAATCCGGGGTCCCTGACCGATTCAGTGGCAGCGGGTCTGGGACAGATTTCTCTCTCACCATCAGCAGCCTGCAGGCTGAAGATGTGGCAGTTTATTACTGTCAGCAATATTACAGTACTCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGAGTAGCAGTGGCTGGTACTTTTTTGACTACTGGGGCCAGGGAACCCTGGTCACCGTCTCCTCA |
| | | | SEQ ID NO: 26255 | SEQ ID NO: 30261 |

FIGURE 50

| | | | | |
|---|---|---|---|---|
| | | AA | DIVMTQSPDSLAVSLGERATINCKSSQSVLHSSNNYNYLVWYQQKPGQSPKRLIYWASTRESGVPDRFSGSGSGTDFSLTISSLQAEDVAVYYCQQYYSTPFTFGPGTKVDIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCASSSGWYFFDYWGQGTLVTVSS |
| | | | SEQ ID NO: 26256 | SEQ ID NO: 30262 |
| **iPS:435435** | **21-225_152H3** | NA | GACATCGTGATGACCCAGTCTCCAGACTCCCTGGCTGTGTCTCTGGGCGAGAAGGCCACCATCAACTGCAAGTCCAGCCAGAGTGTTTTGCACAGCTCCAACAATTACAACTACTTAACTTGGTACCAGCAGAAACCAGGACAGCCTCCTAAACTGCTCATTTACTGGGCATCTACCCGGAAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCCCCAATTATGATATCAACTGGGTGCGACAGGCCACTGGACAAGGCCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCATATAGCAGTGGCTGGTACTGGTTTGACTACTGGGGCCAGGGAACCCTGGTCACCGTCTCCTCA |
| | | | SEQ ID NO: 26257 | SEQ ID NO: 30263 |
| | | AA | DIVMTQSPDSLAVSLGEKATINCKSSQSVLHSSNNYNYLTWYQQKPGQPPKLLIYWASTRKSGVPDRFSGSGSGTDFTLTISSLQAEDVAVYYCQQYYSTPCSFGQGTKLEIK | QVQLVQSGAEVKKPGASVKVSCKASGYTFPNYDINWVRQATGQGLEWMGWMHPNSGNTGYAQKFQGRVTMTRNTSISTAYMELSSLRSEDTAVYYCAYSSGWYWFDYWGQGTLVTVSS |
| | | | SEQ ID NO: 26258 | SEQ ID NO: 30264 |

FIGURE 50

| | | | | |
|---|---|---|---|---|
| **iPS:435437** | **21-225_152F4** | NA | GACATCGTGATGACCCAGTCTCCAGACTCCCTGGCTGTGTCTCTGGGCGAGAGGGCCACCATCAACTGCAAGTCCAGCCAGAGTGTTTTATACAGCTCCAACAATTACAACTACTTAGCTTGGTACCAGCAGAAACCAGGGCAGCCTCCTAAACTGCTCATTTACTGGGCATCTACCCGGAAATCCGGGGTCCCTGACCGATTCAGTGGCAGCGGGTCTGGGACAGATTTCACTCTCACCGTCAGCAGCCTGCAGGCTGAAGATGTGGCAGTTTATTACTGTCAGCAATATTTTAATACTCCTCCCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGACGACACGGCCGTGTATTACTGTGCGTATAGCAGTGGCTGGTACTGGTTCGACCCCTGGGGCCAGGGAACCCTGGTCACCGTCTCCTCA |
| | | | SEQ ID NO: 26259 | SEQ ID NO: 30265 |
| | | AA | DIVMTQSPDSLAVSLGERATINCKSSQSVLYSSNNYNYLAWYQQKPGQPPKLLIYWASTRKSGVPDRFSGSGSGTDFTLTVSSLQAEDVAVYYCQQYFNTPPTFGPGTKVDIK | QVQLVQSGAEVKKPGASVKVSCKASGYTFTNYDINWVRQATGQGLEWMGWMHPNSGNTGYAQKFQGRVTMTRNTSISTAYMELSSLRSDDTAVYYCAYSSGWYWFDPWGQGTLVTVSS |
| | | | SEQ ID NO: 26260 | SEQ ID NO: 30266 |
| **iPS:435439** | **21-225_152G4** | NA | GACATCCAGATGACCCAGTCTCCATCCTCCCTGTCTGCATCTGTAGGAGACAGAGTCACCATCACTTGCCGGGCAAGTCAGAGCATTAGCGACTATTTAAATTGGTATCAGCAAAAACCAGGGAAAGCCCCTAAGGTCCTGATCTATACTACATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTTCTGTCAACAGAGTTACAGTACCCCCACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTCGTGGTGGTAACACATTCTACGCAGACTCCGTGAAGGGCCGGTTCACCATCTCCAGAGACAATTCCAAGAACACACTATATCTGCAAATGAACAGCCTGAGAGCCGAGGACACGGCCGTATATTACTGTGCGAAACGGGTGACGGACTACGGTGGTAACGACTGGTTCGACCCCTGGGGCCAGGGAACCCTGGTCACCGTCTCCTCA |
| | | | SEQ ID NO: 26261 | SEQ ID NO: 30267 |

FIGURE 50

| | | | | |
|---|---|---|---|---|
| | | AA | DIQMTQSPSSLSASVGDRVTITCRASQSISDYLNWYQQKPGKAPKVLIYTTSSLQSGVPSRFSGSGSGTDFTLTISSLQPEDFATYFCQQSYSTPTFGGGTKVEIK | EVQLLESGGGLVQPGGSLRLSCAASGFTFSSYAMSWVRQAPGKGLEWVSAISGRGGNTFYADSVKGRFTISRDNSKNTLYLQMNSLRAEDTAVYYCAKRVTDYGGNDWFDPWGQGTLVTVSS |
| | | | SEQ ID NO: 26262 | SEQ ID NO: 30268 |
| iPS:435441 | 21-225_152F6 | NA | GATATTGTGATGACCCAGACTCCACTCTCTCTGTCCGTCACCCCTGGACAGCCGGCCTCCATCTCCTGCAAGTCTAGTCAGAGCCTCCGGCATGGTGATGGAAAGACCTATTTGACTTGGTACCTACAGAGGCCAGGCCAGCCTCCACAGGTCCTGATCCATGAAATTTCCAAGCGGTTCACTGGAGTGCCAGATAGGTTCAGTGGCAGCGGGTCAGGGACAGATTTCACACTGAACATCAGCCGGGTGGAGGCTGAGGATGTTGGCTTTTATTACTGCATGCAAAGTATACAGGTTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAATTATATGGTATGATGGAAGTTATAAATACTATGCAGACTCCGTGAAGGGCCGATTCACCATCTCCAGAGACAATTCCAAGAATACGCTGTATCTGCAAATGAACAGCCTGAGAGCCGAGGACACGGCTGTGTATTACTGTGCGAGAGAGGGGTACGATTTTTGGAGTGGTTACCTTGGCTACTGGGGCCAGGGAACCCTGGTCACCGTCTCCTCA |
| | | | SEQ ID NO: 26263 | SEQ ID NO: 30269 |
| | | AA | DIVMTQTPLSLSVTPGQPASISCKSSQSLRHGDGKTYLTWYLQRPGQPPQVLIHEISKRFTGVPDRFSGSGSGTDFTLNISRVEAEDVGFYYCMQSIQVPWTFGQGTKVEIK | QVQLVESGGGVVQPGRSLRLSCAASGFTFSSYGMHWVRQAPGKGLEWVAIIWYDGSYKYYADSVKGRFTISRDNSKNTLYLQMNSLRAEDTAVYYCAREGYDFWSGYLGYWGQGTLVTVSS |
| | | | SEQ ID NO: 26264 | SEQ ID NO: 30270 |

FIGURE 50

| | | | | |
|---|---|---|---|---|
| **iPS:435443** | **21-225_152E7** | NA | GAAATTGTGTTGACGCAGTCTCCAGGCACCCTGTCTTTGTTTCCAGGGGAAAGAGCCACCCTCTCCTGCAGGGCCAGTCAGAGTGTTATCAGCAGCTACTTAGCCTGGTACCAGCAGAAACCTGGCCAGGCTCCCAGGCTCCTCATCTTTGGTGTATCTAGTAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAACAATATGGTAGGTCACCATTCAATTTCGGCCCTGGGACCAAAGTGGATATCAAA | CAGGTGCAGCTGCAGGAGTCGGGCCCAGGACTGGTGAAGCCTTCACAGACCCTGTCCCTCAATTGCACTGTCTCTGGTGGCTCCATCAGCAACAGTGGTTACTACTGGAGCTGGATCCGCCAGCACCCAGGCAAGGGCCTGGAATGGATTGGGTACAGTTATTACAGTGGGAGCACCTACTACAACCCGTCCCTCAAGAGTCGAATTACCATATCAGTAGACACGTCTAACAACCAGTTCTCCCTGAACCTGAGCTCTGTGACTGCCGCGGACACGGCCGTGTATTACTGTGCGAGAGGGGGATATAACTGGAACCATGCTTTTGATATCTGGGGCCAAGGGACAATGGTCACCGTCTCTTCA |
| | | | SEQ ID NO: 26265 | SEQ ID NO: 30271 |
| | | AA | EIVLTQSPGTLSLFPGERATLSCRASQSVISSYLAWYQQKPGQAPRLLIFGVSSRATGIPDRFSGSGSGTDFTLTISRLEPEDFAVYYCQQYGRSPFNFGPGTKVDIK | QVQLQESGPGLVKPSQTLSLNCTVSGGSISNSGYYWSWIRQHPGKGLEWIGYSYYSGSTYYNPSLKSRITISVDTSNNQFSLNLSSVTAADTAVYYCARGGYNWNHAFDIWGQGTMVTVSS |
| | | | SEQ ID NO: 26266 | SEQ ID NO: 30272 |
| **iPS:435445** | **21-225_152F7** | NA | GACATCCAGATGACCCAGTCTCCATCCTCCCTGTCTGCATCTGTTGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTACATCCAGTTTGCAAAGTGGGGTCCCATCAAGGTTCAGCGGCAGTGGATCTGGGACAGAATTCACTCTCACAATCAGCAGCCTACAGCCTGAAGATTTTGCAACTTATTACTGTCTACAGCATTATAATTTCCCGTACAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATATCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGGAGTATAGCAGCAGCTGGTACGGGTACGGTATGGACGTCTGGGGCCAAGGGACCACGGTCACCGTCTCCTCA |
| | | | SEQ ID NO: 26267 | SEQ ID NO: 30273 |

FIGURE 50

| | | | | |
|---|---|---|---|---|
| | | AA | DIQMTQSPSSLSASVGDRVTITCRASQGIRNDLGWYQQKPGKAPKRLIYATSSLQSGVPSRFSGSGSGTEFTLTISSLQPEDFATYYCLQHYNFPYSFGQGTKLEIK | QVQLVESGGGVVQPGRSLRLSCAASGFTFSSYIMHWVRQAPGKGLEWVAVIWYDGSNKYYADSVKGRFTISRDNSKNTLYLQMNSLRAEDTAVYYCAREEYSSSWYGYGMDVWGQGTTVTVSS |
| | | | SEQ ID NO: 26268 | SEQ ID NO: 30274 |
| iPS:435447 | 21-225_152H7 | NA | GACATCCAGATGACCCAGTCTCCAGCTTCCGTGTCTGCATCTGTGGGAGACAGAGTCACCATAACTTGTCGGGCGAGTCAGGATATTAGCAACTGGTTAGCCTGGTATCAGCAGAAACCAGGGAAAGCCCCTAAACTCCTGATCTATGCTGCATCCAGTTTGCAAGGTGGGGTCCCATCAAGGTTCAGCGGCAGTGGATCTGGGACAGATTTCACTCTCTCCATCAGCACCCTGCAGCCTGAAGATTTTGCAACTTACTATTGTCAACAGACTGACAGTTTCCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAACAGCTATGCCATGAACTGGGTCCGCCAGGCTCCAGGGAAGGGGCTGGAGTGGGTCTCAGCTATTAGTGGTAGTGGTGGTAACACATTCTACGCAGACTCCGTGAAGGGCCGCTTCACCATCTCCAGAGACAATTCCAAGAACACACTGTATCTGCAAATGAACAGCCTGAGAGCCGAGGACACGGCCGTATATTACTGTGCGAAAAAGGATAATGATTACGTTTGGGGGAGTCCTTACTTTGACTACTGGGGCCAGGGAACCCTGGTCACCGTCTCCTCA |
| | | | SEQ ID NO: 26269 | SEQ ID NO: 30275 |
| | | AA | DIQMTQSPASVSASVGDRVTITCRASQDISNWLAWYQQKPGKAPKLLIYAASSLQGGVPSRFSGSGSGTDFTLSISTLQPEDFATYYCQQTDSFPFTFGPGTKVDIK | EVQLLESGGGLVQPGGSLRLSCAASGFTFNSYAMNWVRQAPGKGLEWVSAISGSGGNTFYADSVKGRFTISRDNSKNTLYLQMNSLRAEDTAVYYCAKKDNDYVWGSPYFDYWGQGTLVTVSS |
| | | | SEQ ID NO: 26270 | SEQ ID NO: 30276 |

FIGURE 50

| | | | | |
|---|---|---|---|---|
| iPS:435449 | 21-225_152H9 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCACCGGCAGTGGATCTGGGACAGAATTCACTTTCACAATCAGCAGCCTGCAGCCTGAAGATTTTGCAACTTATTACTGTCTACAGCATAGTAATTACCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGCAGAAGTAGTTACTACTGGGGCTGGATCCGCCAGCCCCCAGGGAAGGGGCTGGAGTGGATTGGGAGTATCTATTATAGTGGGAGCGCCTCCTACAACCCGTCCCTCAAGAGTCGAGTCACCATATCCGTAGACACGTCCAAGAACCAGTTCTCCCTGAAGCTGAGCTCTGTGACCGCCGCAGACACGGCTGTGTTTTACTGTGCGAGACTTGATCTCCAGTGGAGTTTTGACTTCTGGGGCCAGGGAACCCTGGTCACCGTCTCCTCA |
| | | | SEQ ID NO: 26271 | SEQ ID NO: 30277 |
| | | AA | DIQMTQSPSSLSASVGDRVTITCRASQGIRNDLGWYQQKPGKAPKRLIYAASSLQSGVPSRFTGSGSGTEFTFTISSLQPEDFATYYCLQHSNYPLTFGGGTKVEIK | QLQLQESGPGLVKPSETLSLTCTVSGGSISRSSYYWGWIRQPPGKGLEWIGSIYYSGSASYNPSLKSRVTISVDTSKNQFSLKLSSVTAADTAVFYCARLDLQWSFDFWGQGTLVTVSS |
| | | | SEQ ID NO: 26272 | SEQ ID NO: 30278 |
| iPS:435451 | 21-225_152D10 | NA | GGCATCGTGATGACCCAGTCTCCAGACTCCCTGGCTGTGTCTCTGGGCGCGAGGGCCACCATCGACTGCAAGTCCAGCCAGAGTGTTTTATACAGCTCCAACAATAAGAACTACTTAGCTTGGTACCAGCAGAAACCAGGACAGCCTCCTAAGTTGCTCATTTACTGGGCATCTACCCGGGAATCCGGGGTCCCTGACCGATTCAGTGGCAGCGGATCTGGGACAGATTTCACTCTCACCATCTACAGCCTGCAGGCTGAAGATGTGGCAGTTTATTACTGTCAGCAATATTATCGTAGTCCTAGTTTTGGCCAGGGGACCAAGCTGGAGATCAAA | CAGGTGCAGCTGCAGGAGTCGGGCCCAGGACTGGTGAAGCCTTCGGAGACCCTGTCCCTCACCTGCACTGTCTCTGGTGGCTCCATCAGTAATTACTACTGGAGCTGGATCCGGCAGCCCGCCGGGAAGGGACTGGAGTGGATTGGGCGTATCGATACCAGTGGGATCACCAACTACAACCCCTCCCTCAAGAGTCGAGTCACCATGTCAGTCGACACGTCCAAGAACCAGTTCTCCCTGAAGCTGACCTCTGTGACCGCCGCGGACACGGCCGTGTATTACTGTGCGAGAGAGGGGGGATTGGGAGCTACCTTCTTTGACTACTGGGGCCAGGGAACCCTGGTCACCGTCTCCTCA |
| | | | SEQ ID NO: 26273 | SEQ ID NO: 30279 |

FIGURE 50

| | | | | |
|---|---|---|---|---|
| | | AA | GIVMTQSPDSLAVSLGARATIDCKSSQSVLYSSNNKNYLAWYQQKPGQPPKLLIYWASTRESGVPDRFSGSGSGTDFTLTIYSLQAEDVAVYYCQQYYRSPSFGQGTKLEIK | QVQLQESGPGLVKPSETLSLTCTVSGGSISNYYWSWIRQPAGKGLEWIGRIDTSGITNYNPSLKSRVTMSVDTSKNQFSLKLTSVTAADTAVYYCAREGGLGATFFDYWGQGTLVTVSS |
| | | | SEQ ID NO: 26274 | SEQ ID NO: 30280 |
| iPS:435453 | 21-225_152G10 | NA | GACATCCAGATGACCCAGTCTCCAGCTTCCGTGTCTGCATCTGTAGGAGACAGAGTCACCATAACTTGTCGGGCGAGTCAGGGTATTAGCAACTGGTTAGCCTGGTATCAGCAGAAACCAGGGAAAGCCCCTAAGCTCCTGATCTCTGCTGCATCCAGTTTGCAAAGTGGGGTCCCATCAAGGTTCAGCGGCAATGAATCTGGGACAGATTTCACTCTCTCCATCAGCAGCCTGCAGCCTGAAGATTTTGCAACTTACTATTGTCAACAGACTGACAGTTTCCCATTCACTTTCGGCCCTGGGACCAAAGTGGATGTCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTCGTGGTGGTAACACATTCTACGCAGACTCCGTGAAGGGCCGCTTCACCATCTCCAGAGACAATTCCAAGAACACGCTCTATCTGCAAATGAACAGCCTGAGAGCCGAGGACACGGCCGTATATTACTGTGCGAAAAAGGATTATGATTACGTTTGGGGGAGTCCTTACTTTGACTACTGGGGCCAGGGAACCCTGGTCACCGTCTCCTCA |
| | | | SEQ ID NO: 26275 | SEQ ID NO: 30281 |
| | | AA | DIQMTQSPASVSASVGDRVTITCRASQGISNWLAWYQQKPGKAPKLLISAASSLQSGVPSRFSGNESGTDFTLSISSLQPEDFATYYCQQTDSFPFTFGPGTKVDVK | EVQLLESGGGLVQPGGSLRLSCAASGFTFSSYAMSWVRQAPGKGLEWVSAISGRGGNTFYADSVKGRFTISRDNSKNTLYLQMNSLRAEDTAVYYCAKKDYDYVWGSPYFDYWGQGTLVTVSS |
| | | | SEQ ID NO: 26276 | SEQ ID NO: 30282 |

FIGURE 50

| | | | | |
|---|---|---|---|---|
| **iPS:435455** | **21-225_152B11** | NA | GACATCCAGATGACCCAGTCTCCATCCTCCCTGTCTGCATCTGTAGGAGACAGAGTCACCATCACTTGCCGGGCAAGTCAGAGCATTAGCGACTATTTAAATTGGTATCAGCAGAAACCAGGGAAAGCCCCTAAGGTCCTGATCTATACTACATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTTCTGTCAACAGAGTTACAGTACCCCCACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAACTGGGTCCGCCAGGCTCCAGGGAAGGGGCTGGAGTGGGTCTCAGCTATTAGTGGTCGTGGTGGTAACACATTCTACGCAGACTCCGTGAAGGGCCGGTTCACCATCTCCAGAGACAATTCCAAGAACACACTATATCTGCAAATGAACAGCCTGAGAGCCGAGGACACGGCCGTATATTACTGTGCGAAACGGGTGACGGACTACGGTGGTAACGACTGGTTCGACCCCTGGGGCCAGGGAACCCTGGTCACCGTCTCCTCA |
| | | | SEQ ID NO: 26277 | SEQ ID NO: 30283 |
| | | AA | DIQMTQSPSSLSASVGDRVTITCRASQSISDYLNWYQQKPGKAPKVLIYTTSSLQSGVPSRFSGSGSGTDFTLTISSLQPEDFATYFCQQSYSTPTFGGGTKVEIK | EVQLLESGGGLVQPGGSLRLSCAASGFTFSSYAMNWVRQAPGKGLEWVSAISGRGGNTFYADSVKGRFTISRDNSKNTLYLQMNSLRAEDTAVYYCAKRVTDYGGNDWFDPWGQGTLVTVSS |
| | | | SEQ ID NO: 26278 | SEQ ID NO: 30284 |
| **iPS:435457** | **21-225_152C11** | NA | GATATTGTGATGACCCAGGCTCCACTCTCTCTGTCCGTCACCCCTGGACAGCCGGCCTCCATCTCCTGCAAGTCTAGTCAGAGCCTCCTGCATGGTGATGGAAAGACCTATTTATATTGGTACCTGCAGAAGCCAGGCCAGCCTCCACAGATCCTGATCTATGAAGTTTCCAACCGGTTCTCTGGAGTGCCAGATAGGTTCAGTGGCAGCGGGTCAGGGACAGATTTCACACTGAACATCAGCCGGGTGGAGGCTGAGGATTTTGGGTTTTATTACTGCATGCAAAGTATACAGATTCCGTGGACGTTCGGCCAAGGGACCAAGGTGGACATCAAA | CAGGTACAACTGGTGGAGTCTGGGGGAGGCGTGGTCCAGCCTGGGAGGTCCCTGAGACTCTCCTGTGCAGCGTCTGGATTCACCTTCCGTAACTATGGCATGCACTGGGTCCGCCAGGCTCCAGGCAAGGGGCTGGAGTGGGTGGCAATTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGGCTTACGATTTTTGGAGTGGTTATTTTGACTACTGGGGCCAGGGAATTCTGGTCACCGTCTCCTCA |
| | | | SEQ ID NO: 26279 | SEQ ID NO: 30285 |

FIGURE 50

| | | | | |
|---|---|---|---|---|
| | | AA | DIVMTQAPLSLSVTPGQPASISCKSSQSLLHGDGKTYLYWYLQKPGQPPQILIYEVSNRFSGVPDRFSGSGSGTDFTLNISRVEAEDFGFYYCMQSIQIPWTFGQGTKVDIK | QVQLVESGGGVVQPGRSLRLSCAASGFTFRNYGMHWVRQAPGKGLEWVAIIWYDGSNKYYADSVKGRFTISRDNSKNTLYLQMNSLRAEDTAVYYCAREAYDFWSGYFDYWGQGILVTVSS |
| | | | SEQ ID NO: 26280 | SEQ ID NO: 30286 |
| **iPS:435459** | **21-225_152E12** | NA | GCCGTCGTGATGACCCAGTCTCCAGACTCCCTGGCTGTGTCTCTGGGCGAGAGGGCCACCATCAACTGCACGTCCAGCCAGAGTATTTTACACAGCTCCAATAATTACAACTACTTAGCTTGGTACCAGCAGAAACCAGGACAGCCTCCTAAATTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAACAATATTATAGTGGTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCTTATAGCAGTGGCTGGTACTACTTTGACTACTGGGGCCAGGGAACCCTGGTCACCGTCTCCTCA |
| | | | SEQ ID NO: 26281 | SEQ ID NO: 30287 |
| | | AA | AVVMTQSPDSLAVSLGERATINCTSSQSILHSSNNYNYLAWYQQKPGQPPKLLIYWASTRESGVPDRFSGSGSGTDFTLTISSLQAEDVAVYYCQQYYSGPCSFGQGTKLEIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYYCAYSSGWYYFDYWGQGTLVTVSS |
| | | | SEQ ID NO: 26282 | SEQ ID NO: 30288 |

FIGURE 50

| | | | | |
|---|---|---|---|---|
| **iPS:435461** | **21-225_153A1** | NA | GACATCCAGATGACCCAGTCTCCATCCTCACT<br>GTCTGCATCTGTAGGAGACAGAGTCACCATCA<br>CTTGTCGGGCGAGTCAGGTCATTAGCAATTAT<br>TTAGCCTGGTTTCAGCAGAAACCAGGGAAAGC<br>CCCTAAGTCCCTGATCTCTGCTGCATCCAGTTT<br>GCGAAGTGGGGTCCCATCAAACTTCAGCGGCA<br>GTGGATCTGGGACAGATTTCACTCTCACCATC<br>AGCAGCCTGCAGCCTGAAGATTTTGCAACTTA<br>TTACTGCCAACAGTATCATAGTTACCCATTCA<br>CTTTCGGCCCTGGGACCAAAGTGGATTTCAAA | GAGGTGCAGCTGTTGGAGTCTGGGGGAGGCCTG<br>GTACAGCCTGGGGGGTCCCTGAGACTCTCCTGTG<br>CAGCCTCTGGATTCAGCTTTAGCAGCTATGTCAT<br>GAGTTGGGTCCGCCAGGGTCCAGGGAAGGGGCT<br>GGAGTGGGTCTCAGCTATTAGTGGAAGTGGTGAT<br>AGAACATACTACGCAGACTCCGTGAAGGGCCGG<br>TTCACCATCTCCAGAGACAATTCCAAGAACACGC<br>TGTACCTGCAAATGAACAGCCTGAGAGCCGAGG<br>ACACGGCCGTATATTACTGTGCGCGTACGGCGAC<br>TAAGGACTACTGGGGCCAGGGAACCCTGGTCAC<br>CGTCTCCTCA |
| | | | SEQ ID NO: 26283 | SEQ ID NO: 30289 |
| | | AA | DIQMTQSPSSLSASVGDRVTITCRASQVISNYLA<br>WFQQKPGKAPKSLISAASSLRSGVPSNFSGSGSG<br>TDFTLTISSLQPEDFATYYCQQYHSYPFTFGPGT<br>KVDFK | EVQLLESGGGLVQPGGSLRLSCAASGFSFSSYVMS<br>WVRQGPGKGLEWVSAISGSGDRTYYADSVKGRFTI<br>SRDNSKNTLYLQMNSLRAEDTAVYYCARTATKDY<br>WGQGTLVTVSS |
| | | | SEQ ID NO: 26284 | SEQ ID NO: 30290 |
| **iPS:435463** | **21-225_153D2** | NA | GATATTGTGATGACCCAGACTCCACTCTCTCT<br>GTCCGTCACCCCTGGACAGCCGGCCTCCATCT<br>CCTGCAAGTCTAGTCAGAGCCTCCGGCATGGT<br>GATGGAAAGACCTATTTGACTTGGTACCTACA<br>GAGGCCAGGCCAGCCTCCACAGGTCCTGATCC<br>ATGAAGTTTCCAAGCGGTTCACTGGAGTGCCA<br>GATAGGTTCAGTGGCAGCGGGTCAGGGACAG<br>ATTTCACACTGAACATCAGCCGGGTGGAGGCT<br>GAGGATGTTGGGTTTTATTACTGCATGCAAAG<br>TATACAGGTTCCGTGGACGTTCGGCCAAGGGA<br>CCAAGGTGGAAATCAAA | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTAGCTATGGCAT<br>GCACTGGGTCCGCCAGGCTCCAGGCAAGGGGCT<br>GGAGTGGGTGGCAATTATATGGTATGATGGAAGT<br>TATAAATACTATGCAGACTCCGTGAAGGGCCGAT<br>TCACCATCTCCAGAGACAATTCCAAGAACACGGT<br>GTATCTGCAAATGAACAGCCTGAGAGCCGAGGA<br>CACGGCTGTATATTACTGTGCGAGAGAGGGGTAC<br>GATTTTTGGAGTGGTTACCTTGGCTACTGGGGCC<br>AGGGAACCCTGGTCACCGTCTCCTCA |
| | | | SEQ ID NO: 26285 | SEQ ID NO: 30291 |

FIGURE 50

| | | | | |
|---|---|---|---|---|
| | | AA | DIVMTQTPLSLSVTPGQPASISCKSSQSLRHGDGKTYLTWYLQRPGQPPQVLIHEVSKRFTGVPDRFSGSGSGTDFTLNISRVEAEDVGFYYCMQSIQVPWTFGQGTKVEIK | QVQLVESGGGVVQPGRSLRLSCAASGFTFSSYGMHWVRQAPGKGLEWVAIIWYDGSYKYYADSVKGRFTISRDNSKNTVYLQMNSLRAEDTAVYYCAREGYDFWSGYLGYWGQGTLVTVSS |
| | | | SEQ ID NO: 26286 | SEQ ID NO: 30292 |
| iPS:435465 | 21-225_153A6 | NA | GAAATTGTGTTGACGCAGTCTCCAGGCACCCTGTCTTTGTTTCCAGGGGAAAGAGCCCCCCTCTCCTGCAGGGCCAGTCAGAGTGTTATCAGCAGCTACTTAGCCTGGTACCAGCAGAAACCTGGCCAGGCTCCCAGGCTCCTCATCTTTGGTGTATCTAGTAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAACAATATGGTAGGTCACCATTCAATTTCGGCCCTGGGACCAAAGTGGATATCAAA | CAGGTGCAGCTGCAGGAGTCGGGCCCAGGACTGGTGAAGCCTTCACAGACCCTGTCCCTCAATTGCACTGTCTCTGGTGGCTCCATCAGCAACAGTGGTTACTACTGGAGCTGGATCCGCCAGCACCCAGGCAAGGGCCTGGAATGGATTGGGTACAGCTATTACAGTGGGAGCACCTACTACAACCCGTCCCTCAAGAGTCGAATTACCATATCAGTAGACACGTCTAACAACCAGTTCTCCCTGAACCTGAGCTCTGTGACTGCCGCGGACACGGCCGTGTATTACTGTGCGAGAGGGGGATATAACTGGAACCATGCTTTTGATATCTGGGGCCAAGGGACAATGGTCACCGTCTCTTCA |
| | | | SEQ ID NO: 26287 | SEQ ID NO: 30293 |
| | | AA | EIVLTQSPGTLSLFPGERAPLSCRASQSVISSYLAWYQQKPGQAPRLLIFGVSSRATGIPDRFSGSGSGTDFTLTISRLEPEDFAVYYCQQYGRSPFNFGPGTKVDIK | QVQLQESGPGLVKPSQTLSLNCTVSGGSISNSGYYWSWIRQHPGKGLEWIGYSYYSGSTYYNPSLKSRITISVDTSNNQFSLNLSSVTAADTAVYYCARGGYNWNHAFDIWGQGTMVTVSS |
| | | | SEQ ID NO: 26288 | SEQ ID NO: 30294 |

FIGURE 50

| | | | | |
|---|---|---|---|---|
| **iPS:435467** | **21-225_153B9** | NA | GGCATCGTGATGACCCAGTTTCCAGATTCCCTGGCTGTGTCTCTGGGCGAGAGGGCCACCATCAACTGCAAGTCCAGCCAGAGTGTTTTATACAGCTCCAACAATAAGAACTACTTAGCTTGGTACCAGCAGAAACCAGGACAGCCTCATAAGTTGCTCATTTACTGGGCATCTACCCGGGAATTTGGGGTCCCTGACCGATTCAGTGGCAGCGGGTGTGGGACAGATTTCACTCTCACCATCTACAGCGTGCAGGCTGAAGATGTGGCAGTTTATTACTGTCAGCAATATAATCGTAGTCTTAGTTTTGGCCAGGGGACCAAGCTGGAGATCAAA | CAGGTGCAGCTGCAGGAGTCGGGCCCAGGACTGGTGAAGCCTTCGGAGACCCTGTCCCTCACCTGCACTGTCTCTGGTGGCTCCATCAGTAGTTACTACTGGAGCTGGATCCGGCAGCCCGCCGGGAAGGGACTGGAGTGGATTGGGCGTATCGATACCAGTGGGATCACCAACTACAACCCCTCCCTCAAGAGTCGAGTCACCATGTCAGTCGACACGTCCAAGAACCAGTTCTCCCTGAAGCTGACCTCTGTGACCGCCGCGGACACGGCCGTGTATTACTGTGCGAGAGAGGGGGGAGTGGGAGCTACGTACTTTGACTACTGGGGCCAGGGAACCCTGGTCACCGTCTCCTCA |
| | | | SEQ ID NO: 26289 | SEQ ID NO: 30295 |
| | | AA | GIVMTQFPDSLAVSLGERATINCKSSQSVLYSSNNKNYLAWYQQKPGQPHKLLIYWASTREFGVPDRFSGSGCGTDFTLTIYSVQAEDVAVYYCQQYNRSLSFGQGTKLEIK | QVQLQESGPGLVKPSETLSLTCTVSGGSISSYYWSWIRQPAGKGLEWIGRIDTSGITNYNPSLKSRVTMSVDTSKNQFSLKLTSVTAADTAVYYCAREGGVGATYFDYWGQGTLVTVSS |
| | | | SEQ ID NO: 26290 | SEQ ID NO: 30296 |
| **iPS:435469** | **21-225_153G9** | NA | GATATTGTGATGACCCAGACTCCCTTCTCTCTGTCCGTCACCCCTGGACAGCCGGCCTCCATCTCCTGCAAGTCTAGTCAGAGCCTCCTGCATAGTGATGGAAAGACCTATTTGTATTGGTACCTGCAGAAGCCAGGCCAGCCTCCACAGTTCCTGATCTATGAAGTTTCCAACCGGTTCTCTGGAGTGCCAGATAGGTTCAGTGGCAGCGGGTCAGGGACAGATTTCACACTGAAAATCAGCCGGGTGGAGGCTGACGATGTTGGGGTTTATTACTGCATGCAAAATATAAAGTATCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCACTTATATTCTATGATGGAAGTAATAAATACTATGCAGACTCCGTGAAGGGCCGATTCACCATCTCCAGAGACAATTCCAAGAACACGCTGTATCTGCAAATAAACAGCCTGAGAGCCGAGGACACGGCTGTGTATTTCTGTGCGCGACGCTATAGCCGCAGCTGGGCCGGGGGTATGGACGTCTGGGGCCAAGGGACCGCGGTCACCGTCTCCTCA |
| | | | SEQ ID NO: 26291 | SEQ ID NO: 30297 |

FIGURE 50

| | | | | |
|---|---|---|---|---|
| | | AA | DIVMTQTPFSLSVTPGQPASISCKSSQSLLHSDGKTYLYWYLQKPGQPPQFLIYEVSNRFSGVPDRFSGSGSGTDFTLKISRVEADDVGVYYCMQNIKYPLTFGGGTKVEIK | QVQLVESGGGVVQPGRSLRLSCAASGFTFSSYGMHWVRQAPGKGLEWVALIFYDGSNKYYADSVKGRFTISRDNSKNTLYLQINSLRAEDTAVYFCARRYSRSWAGGMDVWGQGTAVTVSS |
| | | | SEQ ID NO: 26292 | SEQ ID NO: 30298 |
| **iPS:435471** | **21-225_153F11** | NA | GACATCGTGATGACCCAGTCTCCAGACTCCCTGGCTGTGTCTCTGGGCGAGAGGGCCACCATCAACTGCAAGTCCAGCCAGAGTGTTTTATACAGCTCCAACAATTACAAGTACTTAGCTTGGTACCAGCAGAAACCAGGACAGCCTCCTAACCTGCTCATTTACTGGGCATCTACCCGGAAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCGTGCAGTTTTGGCCAGGGGACCAAGCTGGAGATCAAA | CAGGTGCAGCTGGTGCAGTCTGGGGCTGAGGTGAAGGAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAATACCTCCATAAACACAGCCTACATGGAGCTGAGCAGCCTGAGATCTGAGGACACGGCCGTGTATTACTGTGCGTATAGCAGTGGCTGGTACTTCTTTGACAACTGGGGCCAGGGAACCCTGGTCACCGTCTCCTCA |
| | | | SEQ ID NO: 26293 | SEQ ID NO: 30299 |
| | | AA | DIVMTQSPDSLAVSLGERATINCKSSQSVLYSSNNYKYLAWYQQKPGQPPNLLIYWASTRKSGVPDRFSGSGSGTDFTLTISSLQAEDVAVYYCQQYYSTPCSFGQGTKLEIK | QVQLVQSGAEVKEPGASVKVSCKASGYTFTNYDINWVRQATGQGLEWMGWMHPNSGNTGYAQKFQGRVTMTRNTSINTAYMELSSLRSEDTAVYYCAYSSGWYFFDNWGQGTLVTVSS |
| | | | SEQ ID NO: 26294 | SEQ ID NO: 30300 |

FIGURE 50

| | | | | |
|---|---|---|---|---|
| **iPS:435475** | **21-225_154H6** | NA | GACATCGTGATGACCCAGTCTCCAGACTCCCTGGCTGTGTCTCTGGGCGAGAGGGCCACCATCAACTGCAAGTCCAGCCAGAGTGTTTTACACAGTTCCAACAATTACAACTATTTAGCTTGGTACCAGCAGAAACCAGGACAGCCTCCTAAGCTGCTCATTTACTGGACATCTACCCGGAAATCCGGGGTCCCTGACCGATTCAGTGGCAGCGGGTCTGGGACACATTTCACTCTCTCCATCAGCAGCCTGCAGGCTGAAGATGTGGCAGTTTATTACTGTCAGCATTATTATAGTACTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGTATAGCAGTGGCTGGTACATCTTTGACTACTGGGGCCAGGGAACCCTGGTCACCGTCTCCTCA |
| | | | SEQ ID NO: 26295 | SEQ ID NO: 30301 |
| | | AA | DIVMTQSPDSLAVSLGERATINCKSSQSVLHSSNNYNYLAWYQQKPGQPPKLLIYWTSTRKSGVPDRFSGSGSGTHFTLSISSLQAEDVAVYYCQHYYSTPCSFGQGTKLEIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYYCAYSSGWYIFDYWGQGTLVTVSS |
| | | | SEQ ID NO: 26296 | SEQ ID NO: 30302 |
| **iPS:435479** | **21-225_154E9** | NA | GACATTCAGATGACCCTGTCTCCATCCTCCGTGTATGCATCTGTCGGAGACAGAGTCACCATCACTTGTCGGGCGAGTCAGGATATTAGCAACTGGTTAGCCTGGTATCAGCAGAGACCAGGGAAAGCCCCTAAGGTCCTTATCTATGCTGCATCCAGTTTGCAAAGTGGGGTCCCATCAAGATTCAGCGGCAGTGGATCTGGGACAGATTTCACTCTCACCATCAGCAGCGTGCAGCCTGAAGATTTTGCAACTTACTATTGTCAACAGGGTAACAGTTTCCCGCTCACTTTCGGCGGAGGGACCAAGGTGGACATCAAA | GAGGTGAAGTTGTTGGAGTCTGGGGGAGGCTTGGTACAGCCGGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTCGTGGTGGTAACACATTCTACGCAGACTCCGTGAAGGGCCGGTTCACCATCTCCAGAGACAATTCCAAGAACACGCTGTTTCTGCAAATGAACAGCCTGAGAGCCGAGGACACGGCCGTATATTACTGTGCGAAAAGGGGATTTCGATTTTTGGAGTGGTTGGGGGGCTTTGACTACTGGGGCCAGGGAACCCTGGTCACCGTCTCCTCA |
| | | | SEQ ID NO: 26297 | SEQ ID NO: 30303 |

FIGURE 50

| | | | | |
|---|---|---|---|---|
| | | AA | DIQMTLSPSSVYASVGDRVTITCRASQDISNWLAWYQQRPGKAPKVLIYAASSLQSGVPSRFSGSGSGTDFTLTISSVQPEDFATYYCQQGNSFPLTFGGGTKVDIK | EVKLLESGGGLVQPGGSLRLSCAASGFTFSSYAMSWVRQAPGKGLEWVSAISGRGGNTFYADSVKGRFTISRDNSKNTLFLQMNSLRAEDTAVYYCAKRGFRFLEWLGGFDYWGQGTLVTVSS |
| | | | SEQ ID NO: 26298 | SEQ ID NO: 30304 |
| iPS:435481 | 21-225_154A11 | NA | GACATCGTGATGACCCAGTCTCCAGACTCCCTGGCTGTGTCTCTGGGCGAGAGGGCCACCATCAACTGCAGGTCAAGCCAGAGTGTTTTACACAGCTCCAACAATTATAACTACTTAGCTTGGTACCAGCAGAAACCAGGACAGCCTCCTAAGCTGCTCATTTACTGGGCATCTAAACGGGATTCCGGGGTCCCTGACCGATTCAGTGGCAGCGGGTCTGGGACAGATTTCACTCTCACCATCAGCAGCCTGCAGGCTGAGGATGTGGCAGTTTATTACTGTCAGCAATATTTTAGTTCTCCTCGGACGTTCGGCCAAGGGACCAAGGTGGAAATCAAG | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAGGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGAGTAGCAGTGGCTGGTACTACTTCGACTACTGGGGCCAGGGAACCCTGGTCACCGTCTCCTCA |
| | | | SEQ ID NO: 26299 | SEQ ID NO: 30305 |
| | | AA | DIVMTQSPDSLAVSLGERATINCRSSQSVLHSSNNYNYLAWYQQKPGQPPKLLIYWASKRDSGVPDRFSGSGSGTDFTLTISSLQAEDVAVYYCQQYFSSPRTFGQGTKVEIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYYCASSSGWYYFDYWGQGTLVTVSS |
| | | | SEQ ID NO: 26300 | SEQ ID NO: 30306 |

FIGURE 50

| | | | | |
|---|---|---|---|---|
| **iPS:435483** | **21-225_155A4** | NA | GACATCCAGATGACCCAGTCTCCAGCTTCCGTGTCTGCATCTGTAGGAGACAGAGTCACCATAACTTGTCGGGCGAGTCAGGGTATTAGCAACTGGTTAGCCTGGTATCACCAGAAACCAGGGAAAGCCCCTAAGCTCCTGATCTATGCTGCATCCAGTTTGCAAGGTGGGGTCCCATCAAGGTTCAGCGGCAGTGGATCTGGGACAGATTTCACTCTCTCCATCAGCAGCCTGCAGCCTGAAGATTTTGCAACTTACTATTGTCACCAGACTGACAGTTTCCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCTTGTGCAGCCTCTGGATTCACCTTTAACAGCTATGCCATGAGCTGGGTCCGCCAGGCTCCAGGGAAGGGGCTGGAGTGGGTCTCAGCTATTAGTGGTAGTGGTGGTAACACATTCTACGCAGACTCCGTGAAGGGCCGCTTCACCATCTCCAGAGACAATTCCAAGAACACGCTGTATCTGCAAATGAACAGCCTGAGAGCCGAGGACACGGCCGTATATTACTGTGCGAAAAAGGATTATGATTACGTTTGGGGGAGTCCTTACTTTGACTACTGGGGCCAGGGAACCCTGGTCACCGTCTCCTCA |
| | | | SEQ ID NO: 26301 | SEQ ID NO: 30307 |
| | | AA | DIQMTQSPASVSASVGDRVTITCRASQGISNWLAWYHQKPGKAPKLLIYAASSLQGGVPSRFSGSGSGTDFTLSISSLQPEDFATYYCHQTDSFPFTFGPGTKVDIK | EVQLLESGGGLVQPGGSLRLSCAASGFTFNSYAMSWVRQAPGKGLEWVSAISGSGGNTFYADSVKGRFTISRDNSKNTLYLQMNSLRAEDTAVYYCAKKDYDYVWGSPYFDYWGQGTLVTVSS |
| | | | SEQ ID NO: 26302 | SEQ ID NO: 30308 |
| **iPS:435485** | **21-225_155B4** | NA | GACATCCAGATGACCCAGTCTCCAGCTTCCGTGTCTGCATCTGTAGGAGACAGAGTCACCATAACTTGTCGGGCGAGTCAGGATATTAGCAACTGGTTAGCCTGGTATCAGCAGAAACCAGGGAAAGCCCCTAAGCTCCTGATCTATGCTGCATCCAGTTTGCAAGGTGGGGTCCCATCAAGGTTCAACGGCAGTGGATCTGGGACAGATTTCACTCTCTCCATCAGCAGCCTGCAGCCTGAAGATTTTGCAACTTACTATTGTCACCAGACTGACAGTTTCCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTAGTGGTGGTAACACATTCTACGCAGACTCCGTGAAGGGCCGCTTCACCATCTCCAGAGACAATTCCAAGAACACGCTGTATCTGCAAATGAACAGCCTGAGAGCCGAGGACACGGCCGTATATTACTGTGCGAAAAAGGATTATGATTACGTTTGGGGGAGTCCTTACTTTGACTACTGGGGCCAGGGAACCCTGGTCACCGTCTCCTCA |
| | | | SEQ ID NO: 26303 | SEQ ID NO: 30309 |

FIGURE 50

| | | | | |
|---|---|---|---|---|
| | | AA | DIQMTQSPASVSASVGDRVTITCRASQDISNWLA WYQQKPGKAPKLLIYAASSLQGGVPSRFNGSGS GTDFTLSISSLQPEDFATYYCHQTDSFPFTFGPGT KVDIK | EVQLLESGGGLVQPGGSLRLSCAASGFTFSSYAMS WVRQAPGKGLEWVSAISGSGGNTFYADSVKGRFTI SRDNSKNTLYLQMNSLRAEDTAVYYCAKKDYDYV WGSPYFDYWGQGTLVTVSS |
| | | | SEQ ID NO: 26304 | SEQ ID NO: 30310 |
| iPS:435487 | 21-225_155C4 | NA | GACATCCAGATGACCCAGTCTCCATCCTCCCT GTCTGCATCTGTAGGAGACAGAGTCACCATCA CTTGCCGGGCAAGTCAGAGCATTAGCAGCTAT TTAAATTGGTATCAGCAGAAACCAGGGAAAG CCCCTAAGGTCCTGATCTTTACTGCATCCAGTT TGCAAAGTGGGGTCCCATCAAGATTCAGTGGC AGTGGATCTGGGACAGATTTCACTCTCACCAT CAGCAGTCTGCAACCTGAAGATTTTGCAACTT ACTACTGTCAACAGAGTTACAGTACCCCCACT TTCGGCGGAGGGACCAGGGTGGAGATCAAA | GAGGTGCAGCTGTTGGAGTCTGGGGGAGGCTTG GTACAGCCTGGGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTCGTGGTGGT AACACATTCTACGCAGACTCCGTGAAGGGCCGGT TCACCATCTCCAGAGACAATTCCAAGAACACGCT GTATCTGCAAATGAACAGTCTGAGAGCCGAGGA CACGGCCGTATATTACTGTGCGAAACGGGTGACG GACTACGGTGGTAACGACTGGTTCGACCCCTGGG GCCAGGGAACCCTGGTCACCGTCTCCTCA |
| | | | SEQ ID NO: 26305 | SEQ ID NO: 30311 |
| | | AA | DIQMTQSPSSLSASVGDRVTITCRASQSISSYLNW YQQKPGKAPKVLIFTASSLQSGVPSRFSGSGSGT DFTLTISSLQPEDFATYYCQQSYSTPTFGGGTRV EIK | EVQLLESGGGLVQPGGSLRLSCAASGFTFSSYAMS WVRQAPGKGLEWVSAISGRGGNTFYADSVKGRFTI SRDNSKNTLYLQMNSLRAEDTAVYYCAKRVTDYG GNDWFDPWGQGTLVTVSS |
| | | | SEQ ID NO: 26306 | SEQ ID NO: 30312 |

FIGURE 50

| | | | | |
|---|---|---|---|---|
| **iPS:435489** | **21-225_155A5** | NA | GATATTGTGATGACCCAGACTCCACTCTCTCT<br>GTCCGTCACCCCTGGACAGCCGGCCTCCATCT<br>CCTGCAAGTCTAGTCAGAGCCTCCTGCATGGT<br>GATGGAAAGACCTATTTGTATTGGTACCTGCA<br>GAAGCCAGGCCAGCCTCCACAGCTCCTGATTT<br>ATGAAGTTTCCAATCGGTTCTCTGGAGTGCCA<br>GATAGGTTCAGTGGCAGCGGTTCAGGGACAG<br>ATTTCACACTGAAAATCAGCCGGGTGGAGGCT<br>GAGGATGTTGGATTTTATTACTGCATGCAAAG<br>TATACAGGTTCCGTGGACGTTCGGCCAAGGGA<br>CCAAGGTGGAAATCAAA | CAGGTGCAGCTAGTGGAGTCTGGGGGAGACGTG<br>GTCCAGCCTGGGAGGTCCCTGAGGCTCTCCTGTG<br>CAGCGTCTGGATTCACCTTCAGTAGCTATGGCAT<br>GCACTGGGTCCGCCAGGCTCCAGGCAAGGGGCT<br>GGAGTGGGTGGCAATTATATGGTATGATGGAAGT<br>AGTAAATACTATGCAGACTCCGTGAAGGGCCGA<br>TTCACCATCTCCAGAGACAATTCCAAGAACACGC<br>TGTATCTGCACATGAACAGCCTGAGAGCCGAGG<br>ACACGGCAGTGTATTACTGTGCGAGAGATCGATA<br>CGATTTTTGGAGTGGTCACTTTGACTACTGGGGC<br>CAGGGAACCCTGGTCACCGTCTCCTCA |
| | | | SEQ ID NO: 26307 | SEQ ID NO: 30313 |
| | | AA | DIVMTQTPLSLSVTPGQPASISCKSSQSLLHGDG<br>KTYLYWYLQKPGQPPQLLIYEVSNRFSGVPDRFS<br>GSGSGTDFTLKISRVEAEDVGFYYCMQSIQVPW<br>TFGQGTKVEIK | QVQLVESGGDVVQPGRSLRLSCAASGFTFSSYGMH<br>WVRQAPGKGLEWVAIIWYDGSSKYYADSVKGRFT<br>ISRDNSKNTLYLHMNSLRAEDTAVYYCARDRYDF<br>WSGHFDYWGQGTLVTVSS |
| | | | SEQ ID NO: 26308 | SEQ ID NO: 30314 |
| **iPS:435491** | **21-225_155E5** | NA | GACATCGTGATGACCCAGTCTCCAGACTCCCT<br>GGCTGTGTCTCTGGGCGAGCGGGCCACCATCA<br>ACTGCAAGTCCAGCCAGAGTGTTTTATCCAGC<br>TCCAACAATAATAATTATTTAGCTTGGTACCA<br>GCAGAAACCAGGACAGCCTCCTAAGCTGCTCA<br>TTTACTGGGCATCTACCCGGAAATCCGGGGTC<br>CCTGACCGATTCAGTGGCAGCGGGTCTGGGAC<br>AGATTTCACTCTCACCATCAGCAGCCTGCAGG<br>CTGAAGATGTGGCAGTTTATTACTGTCAGCAA<br>TATTATAGTACTCCGTGCAGTTTTGGCCAGGG<br>GACCAAGCTGGAGATCAAG | CAGGTGCAGCTGGTGCAGTCTGGGGCTGAGGTG<br>AAGAAGCCTGGGGCCTCAGTGAAGGTCTCCTGC<br>AAGGCTTCTGGATACACCTTCACCAATTATGATA<br>TCAACTGGGTGCGACAGGCCACTGGACAAGGGC<br>TTGAGTGGATGGGATGGATGCACCCTAACAGTGG<br>TAGTACAGGCTATGCACAGAGGTTCCAGGGCAG<br>AGTCACCATGACCAGGAACACCTCCATAAGCAC<br>AGCCTACATGGAGCTGAGCAGCCTGAGATCTGA<br>GGACACGGCCGTGTATTACTGTGCGTTTAGCAGT<br>GGCTGGTACTATTTTGACTACTGGGGCCAGGGAA<br>CCCTGGTCACCGTCTCCTCA |
| | | | SEQ ID NO: 26309 | SEQ ID NO: 30315 |

FIGURE 50

| | | | | |
|---|---|---|---|---|
| | | AA | DIVMTQSPDSLAVSLGERATINCKSSQSVLSSSNNNNYLAWYQQKPGQPPKLLIYWASTRKSGVPDRFSGSGSGTDFTLTISSLQAEDVAVYYCQQYYSTPCSFGQGTKLEIK | QVQLVQSGAEVKKPGASVKVSCKASGYTFTNYDINWVRQATGQGLEWMGWMHPNSGSTGYAQRFQGRVTMTRNTSISTAYMELSSLRSEDTAVYYCAFSSGWYYFDYWGQGTLVTVSS |
| | | | SEQ ID NO: 26310 | SEQ ID NO: 30316 |
| **iPS:435495** | **21-225_155B6** | NA | GACATCGTGATGACCCAGTCTCCAGACTCCCTGGCTGTGTCTCTGGGCGAGAGGGCCACCATCAACTGCAAGTCCAGCCAGAGTGTTTTACACAGCTCCAATAATTACAACTACTTAGCTTGGTACCAGCAGAAACCAGGACAGCCCCCTAAACTGCTCATTTACTGGACATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAACAATATTATAGTACTCCGTGCAGTTTTGGCCAGGGGACCAAGTTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCTTATAGCAGTGGCTGGTACTACTTTGACTACTGGGGCCAGGGAACCCTGGTCACCGTCTCCTCA |
| | | | SEQ ID NO: 26311 | SEQ ID NO: 30317 |
| | | AA | DIVMTQSPDSLAVSLGERATINCKSSQSVLHSSNNYNYLAWYQQKPGQPPKLLIYWTSTRESGVPDRFSGSGSGTDFTLTISSLQAEDVAVYYCQQYYSTPCSFGQGTKLEIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYYCAYSSGWYYFDYWGQGTLVTVSS |
| | | | SEQ ID NO: 26312 | SEQ ID NO: 30318 |

FIGURE 50

| | | | | |
|---|---|---|---|---|
| **iPS:435497** | **21-225_155H9** | NA | GAAATAGTGATGACGCAGTCTCCAGCCACCCTGTCTGTGTCTCCAGGGGAAAGAGCCACCCTCTCCTGCAGGGCCAGTCAGAGTGTTAGTAGTAACTTAGCCTGGTACCAGCAGAAACCTGGCCAGGCTCCCAGACTCCTCATCTATGGTGCATCCACCAGGGCCACTGGTATCCCAGCCAGGTTCAGTGGCAGTGGGTCTGGGACAGAGTTCACTCTCACCATCAGCAGCCTGCAGTCTGAAGATTTTGCAGTTTATTACTGTCAGCAGTATGATGACTGGCCTCCGTGGACGTTCGGCCAAGGGACCAAGGTGGAAATCAAA | GAGGTGCAACTATTGGAGTCTGGGGGAGGCTTGGTTCAGCCTGGGGGGTCCCTGAGACTCTCCTGTGCAGCCTCTGGATTCACCTTTAGCAGCTATGCCATGAACTGGGTCCGCCAGGCTCCAGGGAAGGGGCTGGAATGGGTCTCAACTATTAGTGGTAGAGGTCTTGGCACATACTACGCAGACTCCGTGAAGGGCCGGTTCACCATCTCCAGAGACAATTCCAAGAACACGCTGTATCTGCAACTGAACAGCCTGAGAGCCGAGGACACGGCCGTATATTACTGTGCGAAAGACCATGACTACGGTGACTACAATATCTACTTTGACTACTGGGGCCAGGGAACCCTGGTCACCGTCTCCTCA |
| | | | SEQ ID NO: 26313 | SEQ ID NO: 30319 |
| | | AA | EIVMTQSPATLSVSPGERATLSCRASQSVSSNLAWYQQKPGQAPRLLIYGASTRATGIPARFSGSGSGTEFTLTISSLQSEDFAVYYCQQYDDWPPWTFGQGTKVEIK | EVQLLESGGGLVQPGGSLRLSCAASGFTFSSYAMNWVRQAPGKGLEWVSTISGRGLGTYYADSVKGRFTISRDNSKNTLYLQLNSLRAEDTAVYYCAKDHDYGDYNIYFDYWGQGTLVTVSS |
| | | | SEQ ID NO: 26314 | SEQ ID NO: 30320 |
| **iPS:435499** | **21-225_156G1** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTTTCACAATCAGCAGCCTGCAGCCTGAAGATTTTGCAACTTATTACTGTCTACAGCATAGTAATTACCCGCTCACTTTCGGCGGAGGGACCAAGGTGGAGATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGCAGAAGTAGTTACTACTGGGGCTGGATCCGCCAGCCCCCAGGGAAGGGGCTGGAGTGGATTGGGAGTATCTATTATAGTGGGAGCGCCTCCTACAACCCGTCCCTCAAGAGTCGAGTCACCATATCCGTAGACACGTCCAAGAACCAGTTCTCCCTGAAGCTGAGCTCTGTGACCGCCGCAGACACGGCTGTGTTTTACTGTGCGAGACTTGATCTCCAGTGGAGTTTTGACTTCTGGGGCCAGGGAACCCTGGTCACCGTCTCCTCA |
| | | | SEQ ID NO: 26315 | SEQ ID NO: 30321 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTFTISSLQPEDFATYYCLQHSNYPLTFGGGTKVEIK | QLQLQESGPGLVKPSETLSLTCTVSGGSISRSSYYWGWIRQPPGKGLEWIGSIYYSGSASYNPSLKSRVTISVDTSKNQFSLKLSSVTAADTAVFYCARLDLQWSFDFWGQGTLVTVSS |
| | | | SEQ ID NO: 26316 | SEQ ID NO: 30322 |
| **iPS:435501** | **21-225_156H1** | NA | GACATCGTGATGACCCAGTCTCCAGACTCCCTGGCTGTGTCTCTGGGCGAGAGGGCCACCATCAACTGCAAGTCCAGCCAGAGTGTTTTATACAGCTCCAACAATAACAACTACTTAGCTTGGTACCAGCAGAAACCAGGACAGCCTCCTAAGCTGCTCATTTACTGGGCATCTACCCGGGAATCCGGGGTCCCTGCCCGATTCAGTGGCAGCGGGTCTGGGACAGATTTCACTCTCACCATCAGCAGCCTGCAGGCTGAAGATGTGGCAGTTTATTACTGTCACCAATATTATAGTACTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGGTGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGGGGAGCAGTGGCTGGTACTACTTTGACTACTGGGGCCAGGGAACCCTGGTCACCGTCTCCTCA |
| | | | SEQ ID NO: 26317 | SEQ ID NO: 30323 |
| | | AA | DIVMTQSPDSLAVSLGERATINCKSSQSVLYSSNNNNYLAWYQQKPGQPPKLLIYWASTRESGVPARFSGSGSGTDFTLTISSLQAEDVAVYYCHQYYSTPCSFGQGTKLEIK | QVQLVQSGAEVKKPGASVKVSCKASGYTFTNYDINWVRQATGQGLEWMGWVHPNSGNTGYAQKFQGRVTMTRNTSISTAYMELSSLRSEDTAVYYCAGSSGWYYFDYWGQGTLVTVSS |
| | | | SEQ ID NO: 26318 | SEQ ID NO: 30324 |

FIGURE 50

| | | | | |
|---|---|---|---|---|
| **iPS:435503** | **21-225_156E4** | NA | GACATCCAGATGACCCAGTCTCCATCCTCCCT<br>GTCTGCATCTGTAGGAGACAGAGTCACCATCA<br>CTTGCCGGGCAAGTCAGAGCATTAGCAGCTAT<br>TTAAATTGGTATCAGCAGAAACCAGGGAAAG<br>CCCCTAAGGTCCTGATCTATACTGCATCCAGTT<br>TGCAAAGTGGGGTCCCATCAAGGTTCAGTGGC<br>AGTGGATCTGGGACAGATTTCACTCTCACCAT<br>CAGCAGTCTGCAACCTGAAGATTTTGCAACTT<br>ACTACTGTCAACAGAGTTACAGTGCCCCCACT<br>TTCGGCGGAGGGACCAAGGTGGAGATCAAA | GAGGTGCAGCTGTTGGAGTCTGGGGGAGGCTTG<br>GTACAGCCTGGGGGGTCCCTGAGACTCTCCTGTG<br>CAGCCTCTGGATTCACCTTTAGCAGCTATGCCAT<br>GAGCTGGGTCCGCCAGGCTCCAGGGAAGGGGCT<br>GGAGTGGGTCTCAGCTATTAGTGGTCGTGGTGGT<br>AACACATTCTACGCAGACTCCGTGAAGGGCCGGT<br>TCACCATCTCCAGAGACAATTCCAAGAACACACT<br>ATATCTGCAAATGAACAGCCTGAGAGCCGAGGA<br>CACGGCCGTATATTACTGTGCGAAACGGGTGACG<br>GACTACGGTGGTAACGACTGGTTCGACCCCTGGG<br>GCCAGGGAACCCTGGTCACCGTCTCCTCA |
| | | | SEQ ID NO: 26319 | SEQ ID NO: 30325 |
| | | AA | DIQMTQSPSSLSASVGDRVTITCRASQSISSYLNW<br>YQQKPGKAPKVLIYTASSLQSGVPSRFSGSGSGT<br>DFTLTISSLQPEDFATYYCQQSYSAPTFGGGTKV<br>EIK | EVQLLESGGGLVQPGGSLRLSCAASGFTFSSYAMS<br>WVRQAPGKGLEWVSAISGRGGNTFYADSVKGRFTI<br>SRDNSKNTLYLQMNSLRAEDTAVYYCAKRVTDYG<br>GNDWFDPWGQGTLVTVSS |
| | | | SEQ ID NO: 26320 | SEQ ID NO: 30326 |
| **iPS:435505** | **21-225_157C1** | NA | GACATCCAGATGACCCAGTCTCCATCCTCACT<br>GTCTGCATCTATAGGAGACAGAATCACCCTCA<br>CTTGTCGGGCGAGTCAGGGCATTAGCAATTAT<br>TTAGCCTGGTTTCAGCAGAGACCAGGGAAAGC<br>CCCTAAGTCCCTGATCTATGCTGCATCCAGTTT<br>GCTAAGTGGGGTCCCATCAAAGTTCAGCGGCA<br>GTGGATCTGGGACAGATTTCACTCTCACCATC<br>AGCAGCCTGCAGCCTGAAGATTTTGCAACTTA<br>TTACTGCCAACAGTATAATAGTTTTCCATTCAC<br>TTTCGGCGGAGGGACCAAGGTGGAGCTCAAA | GAGGTGCAGCTGGTGGAGTCTGGGGGAGGCCTG<br>GTCAAGCCTGGGGGGTCCCTGAGACTCTCCTGTG<br>CAGCCTCTGGATTCACCTTCAGTAGCTATAGAAT<br>GAACTGGGTCCGCCAGGCTCCAGGGAAGGGGCT<br>GGAGTGGGTCTCATCCATGAGTAATAGTAGTAGT<br>TCCATATACTACGCAGACTCAGTGAAGGGCCGAT<br>TCACCATCTCCAGAGACAACGCCAAGAACTCACT<br>GTATCTGCAAATGAACAGCCTGAGAGCCGAGGA<br>CACGGCTGTGTATTACTGTGCGAGACAGGCAGCC<br>CAGGACTACTGGGGCCAGGGAACCCTGGTCACC<br>GTCTCCTCA |
| | | | SEQ ID NO: 26321 | SEQ ID NO: 30327 |

FIGURE 50

| | | | | |
|---|---|---|---|---|
| | | AA | DIQMTQSPSSLSASIGDRITLTCRASQGISNYLAWFQQRPGKAPKSLIYAASSLLSGVPSKFSGSGSGTDFTLTISSLQPEDFATYYCQQYNSFPFTFGGGTKVELK | EVQLVESGGGLVKPGGSLRLSCAASGFTFSSYRMNWVRQAPGKGLEWVSSMSNSSSSIYYADSVKGRFTISRDNAKNSLYLQMNSLRAEDTAVYYCARQAAQDYWGQGTLVTVSS |
| | | | SEQ ID NO: 26322 | SEQ ID NO: 30328 |
| iPS:435509 | 21-225_157H1 | NA | GACATCCAGATGACCCAGTCTCCATCCTCACTGTCTGCATCTGTAGGAGACAGAGTCACCATCACTTGTCGGGCGAGTCAGGACATTAGCAATTATTTAGTCTGGTTTCAGCAGAGACCAGGGAAAGCCCCTAGGTCCCTGATCTATGCTGCATCCAGTTTGCAAAGTGGGGTCCCATCTAAGTTCAGCGGCAGTGGATCTGGGACAGATTTCACTCTCACCATCAGCAGCCTGCAGCCTGAAGATTTTGCAACTTATTACTGCCAACAATATCATAGTTACCCATTCACTTTCGGCCCTGGGACCAAAGTGGATATCAAA | GAGGTGCAGCTGTTGGAGTCTGGGGGAGGGTTGGTACAGCCTGGGGGGTCCCTGAGACTCTCCTGTGCAGCCTCTGGATTCACCTTTAGCAGTTATGCCATGAGGTGGATCCGCCAGGCTCCAGGGAAGGGGCTGCAGTGGGTCTCAGATATTAGTGGTAGTGGTGGTACCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAGACCTACCTCTGGGGCCAGGGAACCCTGGTCACCGTCTCCTCA |
| | | | SEQ ID NO: 26323 | SEQ ID NO: 30329 |
| | | AA | DIQMTQSPSSLSASVGDRVTITCRASQDISNYLVWFQQRPGKAPRSLIYAASSLQSGVPSKFSGSGSGTDFTLTISSLQPEDFATYYCQQYHSYPFTFGPGTKVDIK | EVQLLESGGGLVQPGGSLRLSCAASGFTFSSYAMRWIRQAPGKGLQWVSDISGSGGTTYYADSVKGRFTISRDNSKNTLYLQMNSLRAEDTAVYYCAKTYLWGQGTLVTVSS |
| | | | SEQ ID NO: 26324 | SEQ ID NO: 30330 |

FIGURE 50

| | | | | |
|---|---|---|---|---|
| iPS:435511 | 21-225_157C3 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTCATCTATGCTGCATCCAGTTTGCAAAGTGGGGTCCCATCAAGGTTCAGCGGCAGTGGATCTGGGACAGACTTCACTCTCACAATCAGCAGCCTGCAGCCTGATGACTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTACCTATGGCATGCACTGGGTCCGCCAGGCTCCAGGCAAGGGGCTGGAGTGGGTGGCTGTTATATGGTATGATGTAAATAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGCTGGGGTTCCTCTCTGACTATTGGGGCCAGGGAACCCTGGTCACCGTCTCCTCA |
| | | | SEQ ID NO: 26325 | SEQ ID NO: 30331 |
| | | AA | DIQMTQSPSSLSASVGDRVTITCRASQGIRNDLGWYQQKPGKAPKRLIYAASSLQSGVPSRFSGSGSGTDFTLTISSLQPDDFATYYCLQHNSYPFTFGPGTKVDIK | QVQLVESGGGVVQPGRSLRLSCAASGFTFSTYGMHWVRQAPGKGLEWVAVIWYDVNNKYYADSVKGRFTISRDNSKNTLYLQMNSLRAEDTAVYYCARELGFLSDYWGQGTLVTVSS |
| | | | SEQ ID NO: 26326 | SEQ ID NO: 30332 |
| iPS:435513 | 21-225_157F3 | NA | GACATCCAGATGACCCAGTCTCCATCCTCCCTGTCTGCATCTGTAGGAGACAGAGTCACCATCACTTGCCGGGCAAGTCAGAACATTAGCAGTTATTTAAATTGGTATCAGCTGAAACCAGGGAAAGCCCCTAAACTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAGGATTTTGCAACTTACTACTGTCAACAGAGTTACAATACCCCCACGTGGACGTTCGGCCAAGGGACCAAGGTGGAAATCAAA | GAGGTGCAACTGTTGGAGTCTGGGGGAGGCTTGGTACAGCCTGGGGGGTCCCTGAGACTCTCCTGTGCAGCCTCTGAATTCACCTTTAGCACCTATGCCATGAGCTGGGTCCGCCAGGCTCCAGGGAAGGGGCTGGAGTGGGTCTCAGTTATTAGTGGTAGTGGTGGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAAAGGAGCAGTGGCTGGTACGAGGATGCTCTTGATATCTGGGGCCAAGGGACAATGGTCACCGTCTCTTCA |
| | | | SEQ ID NO: 26327 | SEQ ID NO: 30333 |

FIGURE 50

| | | | | |
|---|---|---|---|---|
| | | AA | DIQMTQSPSSLSASVGDRVTITCRASQNISSYLNWYQLKPGKAPKLLIYTASSLQSGVPSRFSGSGSGTDFTLTISSLQPEDFATYYCQQSYNTPTWTFGQGTKVEIK | EVQLLESGGGLVQPGGSLRLSCAASEFTFSTYAMSWVRQAPGKGLEWVSVISGSGGSTYYADSVKGRFTISRDNSKNTLYLQMNSLRAEDTAVYYCAKRSSGWYEDALDIWGQGTMVTVSS |
| | | | SEQ ID NO: 26328 | SEQ ID NO: 30334 |
| iPS:435515 | 21-225_157E4 | NA | GACATCCAGATGACCCAGTCTCCATCCTCACTGTCTGCATCTGTAGGAGACAGAGTCACCTTCACTTGTCGGGCGAGTCAGGGCATTAGCAATTATTTAGCCTGGTTTCAGCAGAAACCAGGGAGAGCCCCTAAGTCCCTGATCTATGCTGCATCCAGTTTGCGAAGTGGGGTCCCATCACAGTTCAGCGGCAGTGGATCTGGGACAGATTTCACTCTCACCATCAACAGCCTGCAGCCTGAAGATTTTGCAACTTATTACTGCCAACAGTATCATAGTTATCCATTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATACCATGAACTGGGTCCGCCAGGCTCCAGGGAAGGGGCTGGAGTGGGTCTCATCCATTAGTGGTAGTAGTAGTTACATATACTACGCAGACTCAGTGAAGGGCCGATTCACCATCTCCAGAGACAACGCCAAGAACTCACTGTATCTGCAAATGAACAGCCTGAAAGCCGAGGACACGGCTGTGTATTACTGTGCGAGAGTAGCTACCTTTGACTACTGGGGCCAGGGAACCCTGGTCACCGTCTCCTCA |
| | | | SEQ ID NO: 26329 | SEQ ID NO: 30335 |
| | | AA | DIQMTQSPSSLSASVGDRVTFTCRASQGISNYLAWFQQKPGRAPKSLIYAASSLRSGVPSQFSGSGSGTDFTLTINSLQPEDFATYYCQQYHSYPFTFGPGTKVDIK | EVQLVESGGGLVKPGGSLRLSCAASGFTFSSYTMNWVRQAPGKGLEWVSSISGSSSYIYYADSVKGRFTISRDNAKNSLYLQMNSLKAEDTAVYYCARVATFDYWGQGTLVTVSS |
| | | | SEQ ID NO: 26330 | SEQ ID NO: 30336 |

FIGURE 50

| | | | | |
|---|---|---|---|---|
| **iPS:435521** | **21-225_157H4** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATCCTGCATCCAGTTTACAAACTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGGATAATAGTTACCCATTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGTTATAGCATGAACTGGGTCCGCCAGGCTCCAGGGAAGGGGCTGGAGTGGGTCTCATCCATTAGTAGTAGTAGTACTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGATAGAGGGTCCATCTGGGGCCAGGGAACCCTGGTCACCGTCTCCTCA |
| | | | SEQ ID NO: 26331 | SEQ ID NO: 30337 |
| | | AA | DIQMTQSPSSLSASVGDRVTITCRASQGIRNDLGWYQQKPGKAPKRLIYPASSLQTGVPSRFSGSGSGTEFTLTISSLQPEDFATYYCLQDNSYPFTFGPGTKVDIK | EVQLVESGGGLVKPGGSLRLSCAASGFTFSSYSMNWVRQAPGKGLEWVSSISSSSTYIYYADSVKGRFTISRDNAKNSLYLQMNSLRAEDTAVYYCARDRGSIWGQGTLVTVSS |
| | | | SEQ ID NO: 26332 | SEQ ID NO: 30338 |
| **iPS:435523** | **21-225_157G5** | NA | GACATCCAGATGACCCAGTCTCCATCCTCACTGTCTGCATCTGTAGGGGACAGAGTCACCATCACTTGTCGGGCGAGTCAGGGCATTAACAATTATTTAGCCTGGTTTCAGCAGAAACCAGGGAAAGCCCCTGAGTCCCTGATCTATGCTGCATCCAGTTTACAAAGTGGGGTCCCATCACAGTTCAGCGGCAGTGGATCTGGGACAGATTTCACTCTCACCATCAGCAGCCTGCAGCCTGAAGATTTTGCAACTTATTACTGCCAACAGTATAATAGTTATCCATTCACTTTCGGCCCTGGGACCAAAGTGGATATCAAA | GAGGTGCAGCTGTTGGAGTCTGGGGGAGGCTTGGTACAGCCGGGGGGGTCCCTGAGACTCTCCTGTGCAGCCTCTGGATTCACCTTTAGTAGCTATGTCATGAGCTGGGTCCGCCAGGCTCCAGGGAAGGGACTGGACTGGGTCTCAGCTATGAGTGGTAGTGGTGGTAGAACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTGTATTACTGTGCGAAGTATACCTGGAACGGCTACTGGGGCCAGGGAACCCTGGTCACCGTCTCCTCA |
| | | | SEQ ID NO: 26333 | SEQ ID NO: 30339 |

FIGURE 50

| | | | | |
|---|---|---|---|---|
| | | AA | DIQMTQSPSSLSASVGDRVTITCRASQGINNYLA WFQQKPGKAPESLIYAASSLQSGVPSQFSGSGSG TDFTLTISSLQPEDFATYYCQQYNSYPFTFGPGT KVDIK | EVQLLESGGGLVQPGGSLRLSCAASGFTFSSYVMS WVRQAPGKGLDWVSAMSGSGGRTYYADSVKGRF TISRDNSKNTLYLQMNSLRAEDTAVYYCAKYTWN GYWGQGTLVTVSS |
| | | | SEQ ID NO: 26334 | SEQ ID NO: 30340 |
| iPS:435525 | 21-225_157E7 | NA | GACATCCAGATGACCCAGTCTCCATCCTCCCT GTCTGCATCTGTAGGAGACAGAGTCACCATCA CTTGCCGGGCAAGTCAGAGCTTTAGCAGCTAT TTAAATTGGTATCAGCAGAAACCAGGGATAGC CCCTAAACTCCTGATCTATGCTGCATCCAGTTT GCAAAGTGGGGTCCCATCAAGGTTCAGTGGCA GTGGATCTGGGACAGATTTCACTCTCACCATC AGCAGTCTGCAACCTGAAGATTTTGCAACTTA CTACTGTCAAGAGAGTTATAGTATCCGCTTCG CCTTCGGCCAAGGGACACGACTGGAGATTAAA | CAGCTGCACCTGCAGGAGTCGGGCCCAGGACTG GTGAAGCCTTCGGAGACCCTGTCCCTCACCTGCA CTGTCTCTGGTGGCTCCATCAGCAGTGGTAGTTA CTACTGGGGCTGGATCCGCCAGCCCCCAGGGAA GGGACTGGAGTGGATTGGGAGTATCTACTATAGT GGGAGCACCTACTACAATCCGTCCCTCAAGAGTC GAGTCACCATATCCGTAGACACGTCCAAGAACC AGTTCTCCCTGAACCTGAGCTCTGTGACCGCCGC AGACACGGCTGTCTATTACTGTGCGAGACATAAA GTGGCTGGTCCCTTTGACTACTGGGGCCAGGGAA CCCTGGTCACCGTCTCCTCA |
| | | | SEQ ID NO: 26335 | SEQ ID NO: 30341 |
| | | AA | DIQMTQSPSSLSASVGDRVTITCRASQSFSSYLN WYQQKPGIAPKLLIYAASSLQSGVPSRFSGSGSG TDFTLTISSLQPEDFATYYCQESYSIRFAFGQGTR LEIK | QLHLQESGPGLVKPSETLSLTCTVSGGSISSGSYYW GWIRQPPGKGLEWIGSIYYSGSTYYNPSLKSRVTIS VDTSKNQFSLNLSSVTAADTAVYYCARHKVAGPF DYWGQGTLVTVSS |
| | | | SEQ ID NO: 26336 | SEQ ID NO: 30342 |

FIGURE 50

| | | | | |
|---|---|---|---|---|
| **iPS:435527** | **21-225_157G7** | NA | GACATTCAGATGACTCAGTCTCCATCCTCCCTGTGTGCATCAGTAGGAGACAGAGTCACCATCACTTGCCGGGCAAGTCAGGGCATTAGAAATGATTTAGGCTGGTATCAGCAGAAACCAGGGAAAGCCCCTAAGCGCTTGATCAATGTTGCATCCAGTTTGCAAAGTGGGGTCCCATCAAGGTTCAGCGGCAGTGGATCTGGGACAGAATTCACTCTCACAATCAGCAGCGTGCAGCGTGAAGATTTTGCAACTTATTACTGTATACAGGATAATAGTCACCCATTCACTTTCGGCCCTGGGACCAAAGTGGAAATCAAA | GAGGTGCAACTGGTGGAGTCTGGGGGAGGCCTGGTCAAGCCTGGGGGGTCCCTGAGATTCTCCTGTGCAGCCTCTGGATTCACCTTCAGTAGTTATAGCATGAACTGGGTCCGCCAGGCTCCAGGGAAGGGGCTGGAGTGGGTCTCATCCATTAGTGGTAGTAGTACGTACATATACTACGCAGACTCAGTGAAGGGCCGATTCACCATCTCCAGAGACAACGCCAAGAACTCACTGTATCTGCAAATGAACAGCCTGAGAGCCGAAGACACGGCTGTGTATTACTGTGCGAGAGATCGGGGCAGCAGCTGGGGCCAGGGAACCCTGGTCACCGTCTCCTCA |
| | | | SEQ ID NO: 26337 | SEQ ID NO: 30343 |
| | | AA | DIQMTQSPSSLCASVGDRVTITCRASQGIRNDLGWYQQKPGKAPKRLINVASSLQSGVPSRFSGSGSGTEFTLTISSVQREDFATYYCIQDNSHPFTFGPGTKVEIK | EVQLVESGGGLVKPGGSLRFSCAASGFTFSSYSMNWVRQAPGKGLEWVSSISGSSTYIYYADSVKGRFTISRDNAKNSLYLQMNSLRAEDTAVYYCARDRGSSWGQGTLVTVSS |
| | | | SEQ ID NO: 26338 | SEQ ID NO: 30344 |
| **iPS:435529** | **21-225_157H7** | NA | GACATCCAGATGACCCAGTCTCCATCCTCACTGTCTGCATCTGTAGGAGACAGAGTCACCATCACTTGTCGGGCGAGTCAGGACATTAGCAATTTTTTAGCCTGGTTTCAGCAGAAACCAGGGAAAGCCCCTAAGTCCCTGGTCTCTACTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATCATAGTTACCCGATCACCTTCGGCCAAGGGACACGACTGGAGATTAAA | GAGGTGCAGCTGGTGGAGTCTGGGGGAGGCCTGGTCAAGCCTGGGGGGTCCCTGAGACTCTCCTGTGCAGCCTCTGGATTCACATTCAGTAGCTATAGCATGAACTGGGTCCGCCAGGCTCCAGGGAAGGGGCTAGAGTGGGTCTCATGCATTAGTGGTAGTAGTAGTTACATATATTATGCAGACTCAGTGAAGGGCCGATTCACCATGTCCAGAGACAACGCCAAGAACTCACTGTATCTGCAAATGAACAGCCTGAGAGCCGAGGACACGGCTGTGTATTACTGTGTGAGAGATCGAGGGGGCTATTGGGGCCAGGGAACCCTGGTCACCGTCTCCTCA |
| | | | SEQ ID NO: 26339 | SEQ ID NO: 30345 |

FIGURE 50

| | | | | |
|---|---|---|---|---|
| | | AA | DIQMTQSPSSLSASVGDRVTITCRASQDISNFLAWFQQKPGKAPKSLVSTASSLQSGVPSKFSGSGSGTDFTLTISSLQPEDFATYYCQQYHSYPITFGQGTRLEIK | EVQLVESGGGLVKPGGSLRLSCAASGFTFSSYSMNWVRQAPGKGLEWVSCISGSSSYIYYADSVKGRFTMSRDNAKNSLYLQMNSLRAEDTAVYYCVRDRGGYWGQGTLVTVSS |
| | | | SEQ ID NO: 26340 | SEQ ID NO: 30346 |
| iPS:435531 | 21-225_157G8 | NA | GATATTGTGATGACCCAGACTCCACTCTCTCTGTCCGTCACCCCTGGACAGCCGGCCTCCATCTCCTGCAAGTCTAGTCAGAGCCTCCTGCATGGTGATGGAAAGACCTATTTATATTGGTACCTGCAGAAGCCAGGCCAGTCTCCACAACTCCTGATCTATGAAATTTCCAAGCGGTTCTCTGGAGTGCCAGATAGGTTCAGTGGCAGCGGGTCAGGGACAGATTTCACACTGAAAATCAGCCGGGTGGAGGCTGAGGATGTTGGGGTTTACTACTGCATGCAAAGTATACAGGTTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAATTATGGCATGCACTGGGTCCGCCAGGCTCCAGGCAAGGGGCTGGAGTGGGTGGCAATTATATGGTATGATGGAAGTTATAAATACTATGCAGACTCCGTGAAGGGCCGATTCACCGTCTCCAGAGACAATTCCAAGAACACGCTGTATCTGCAAATGAACAGCCTGAGAGCCGAGGACACGGCTGTGTATTACTGTGCGAGAGAGGGATACGATTTTTGGAGTGGTTTCTTTGACTCCTGGGGCCAGGGAACCCTGGTCACCGTCTCCTCA |
| | | | SEQ ID NO: 26341 | SEQ ID NO: 30347 |
| | | AA | DIVMTQTPLSLSVTPGQPASISCKSSQSLLHGDGKTYLYWYLQKPGQSPQLLIYEISKRFSGVPDRFSGSGSGTDFTLKISRVEAEDVGVYYCMQSIQVPWTFGQGTKVEIK | QVQLVESGGGVVQPGRSLRLSCAASGFTFSNYGMHWVRQAPGKGLEWVAIIWYDGSYKYYADSVKGRFTVSRDNSKNTLYLQMNSLRAEDTAVYYCAREGYDFWSGFFDSWGQGTLVTVSS |
| | | | SEQ ID NO: 26342 | SEQ ID NO: 30348 |

FIGURE 50

| | | | | |
|---|---|---|---|---|
| **iPS:435533** | **21-225_157H8** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGACTTCACTCTCACAATCAGCAGCCTGCAGCCTGATGACTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCTGTTATATGGTATGATGTAAATAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGCTGGGGTTCCTCTCTGACTACTGGGGCCAGGGAACCCTGGTCACCGTCTCCTCA |
| | | | SEQ ID NO: 26343 | SEQ ID NO: 30349 |
| | | AA | DIQMTQSPSSLSASVGDRVTITCRASQGIRNDLGWYQQKPGKAPKRLIYAASSLQSGVPSRFSGSGSGTDFTLTISSLQPDDFATYYCLQHNSYPFTFGPGTKVDIK | QVQLVESGGGVVQPGRSLRLSCAASGFTFSSYGMHWVRQAPGKGLEWVAVIWYDVNNKYYADSVKGRFTISRDNSKNTLYLQMNSLRAEDTAVYYCARELGFLSDYWGQGTLVTVSS |
| | | | SEQ ID NO: 26344 | SEQ ID NO: 30350 |
| **iPS:435535** | **21-225_157H10** | NA | GACATCCAGATGACCCAGTCTCCATCCTCACTGTCTGCATCTGTAGGAGACAGAGTCACCATCACTTGTCGGGCGAGTCAGGGCATTACCAATTATTTAGCCTGGTTTCAGCAGAAACCAGGGAAAGCCCCTAAGTCCCTGATCTATACTGCATCCAATTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATCATAGTTACCCATTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATACCATGAACTGGGTCCGCCAGGCTCCAGGGAAGGGGCTGGAGTGGGTCTCATCCATTAGTGGTAGCAGTAGTTACATAAACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGTGGCTCACTTTGACTACTGGGGCCAGGGAACCCTGGTCACCGTCTCCTCA |
| | | | SEQ ID NO: 26345 | SEQ ID NO: 30351 |

FIGURE 50

| | | | | |
|---|---|---|---|---|
| | | AA | DIQMTQSPSSLSASVGDRVTITCRASQGITNYLA WFQQKPGKAPKSLIYTASNLQSGVPSKFSGSGSG TDFTLTISSLQPEDFATYYCQQYHSYPFTFGPGT KVDIK | EVQLVESGGGLVKPGGSLRLSCAASGFTFSSYTMN WVRQAPGKGLEWVSSISGSSSYINYADSVKGRFTIS RDNAKNSLYLQMNSLRAEDTAVYYCARVAHFDY WGQGTLVTVSS |
| | | | SEQ ID NO: 26346 | SEQ ID NO: 30352 |
| iPS:435537 | 21-225_157H12 | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTTGGCTGGTATCAGCAGAGACCAGGGAAAGC CCCTAAGTGCCTGATTCATGCTGCATCCAGTTT ACAGAGTGGGGTCCCATCAAGGTTCAGCGGCA GTGGATCTGGGACAGAATTCACTCTCACAATC AGCAGCCTGCAGCCTGAAGATTTTGCAACTTA TTACTGTCTACAGCATTATAGTTACCCATTCAC TTTCGGCCCTGGGACCAAAGTGGATATCAAA | GAGGTGCAGTGGGTGGAGTCTGGGGGAGGCCTG GTCAAGCCTGGGGGGTCCCTGAGACTCTCCTGTG CAGCCTCTGGATTCACCTTCAGTAGCTATAGCAT GAACTGGGTCCGCCAGGCTCCAGGGAAGGGGCT GGAGTGGGTCTCATCCATTAGTGGTAGTGGTAGT TACATAAACTACGCAGACTCAGTGAAGGGCCGG TTCACCATCTCCAGAGACAACGCCAAGACCTCAC TGTATCTGCAAGTGAACGGCCTGAGAGCCGAGG ACACGGCTGTGTATTACTGTGCGAGATCCAAGTT TGACTCCTGGGGCCAGGGAACCCTGGTCACCGTC TCCTCA |
| | | | SEQ ID NO: 26347 | SEQ ID NO: 30353 |
| | | AA | DIQMTQSPSSLSASVGDRVTITCRASQGIRNDFG WYQQRPGKAPKCLIHAASSLQSGVPSRFSGSGS GTEFTLTISSLQPEDFATYYCLQHYSYPFTFGPGT KVDIK | EVQWVESGGGLVKPGGSLRLSCAASGFTFSSYSMN WVRQAPGKGLEWVSSISGSGSYINYADSVKGRFTIS RDNAKTSLYLQVNGLRAEDTAVYYCARSKFDSWG QGTLVTVSS |
| | | | SEQ ID NO: 26348 | SEQ ID NO: 30354 |

FIGURE 50

| | | | | |
|---|---|---|---|---|
| **iPS:435539** | **21-225_158G1** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATACTGCATCCAATTTGCAAAGTGGGGTCCCATCAAGGTTCAGCGGCAGTGGATGTGGGACAGAATTCACTCTCACAGTCAGCAGCCTGCAGCCTGAAGATTTTGCAACTTATTACTGTCTACAGCATAATAGTTACCCGTGGACGTTCGGCCAAGGGACCAAGGTGGAAATCAAA | GAGGTGCAGCTGGTGGAATCTGGGGGAGGCCTGGTCAAGCCTGGGGGGTCCCTGAGACTCTCCTGTGCAGCCTCTGGATTCACCTTCAGTAGTTATGGCATGAACTGGGTCCGCCAGGCTCCAGGGAAGGGGCTGGAGTGGATCTCATCCATTAGTGGTAGTGGTAGTTACATATACTACGCAGACTCAGTGAAGGGCCGATTCACCATTTCCAGAGACAACGCCAAGAACTCACTGTATCTGCAAATGAACAGCCTGAGAGCCGAGGACACGGCTGTGTATTACTGTGCGATTAGCAGTGGCTGGTCTTGGGGCCAGGGAACCCTGGTCACCGTCTCCTCA |
| | | | SEQ ID NO: 26349 | SEQ ID NO: 30355 |
| | | AA | DIQMTQSPSSLSASVGDRVTITCRASQGIRNDLGWYQQKPGKAPKRLIYTASNLQSGVPSRFSGSGCGTEFTLTVSSLQPEDFATYYCLQHNSYPWTFGQGTKVEIK | EVQLVESGGGLVKPGGSLRLSCAASGFTFSSYGMNWVRQAPGKGLEWISSISGSGSYIYYADSVKGRFTISRDNAKNSLYLQMNSLRAEDTAVYYCAISSGWSWGQGTLVTVSS |
| | | | SEQ ID NO: 26350 | SEQ ID NO: 30356 |
| **iPS:435543** | **21-225_158D4** | NA | GACATCCAGATGACCCAGTCTCCATCCTCCCTGTCTGCATCTGTAGGAGACAGAGTCACCATCACTTGCCGGGCAAGTCAGGACATTAGAAAGGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACATTCAGCAGCCTGCAGCCTGAAGATTTTGCAACTTATTACTGTCTACAGCATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGTCATGCACTGGGTCCGCCAGGCTCCAGGCAAGGGGCTGGAGTGGGTGTCAGTTATATGGTATGATGGAAGTAATAAATACTATGCAGACTCCGTGAAGGGCCGATTCACCATCTCCAGAGACAATTCCAAGAACACACTGTATCTGCAAATGAACAGCCTGAGAGCCGAGGACACGGCTGTGTATTACTGTGCGAGAGAACCGTATACTAGTGGCTGGTACGACTACGGTATGGACGTCTGGGGCCAAGGGACCACGGTCACCGTCTCCTCA |
| | | | SEQ ID NO: 26351 | SEQ ID NO: 30357 |

FIGURE 50

| | | | | |
|---|---|---|---|---|
| | | AA | DIQMTQSPSSLSASVGDRVTITCRASQDIRKDLGWYQQKPGKAPKRLIYAASSLQSGVPSRFSGSGSGTEFTLTFSSLQPEDFATYYCLQHYSYPRTFGQGTKVEIK | QVQLVESGGGVVQPGRSLRLSCAASGFTFSDYVMHWVRQAPGKGLEWVSVIWYDGSNKYYADSVKGRFTISRDNSKNTLYLQMNSLRAEDTAVYYCAREPYTSGWYDYGMDVWGQGTTVTVSS |
| | | | SEQ ID NO: 26352 | SEQ ID NO: 30358 |
| **iPS:435545** | **21-225_158F4** | NA | GACATCCAGATGACCCAGTCTCCATCCTCCCTGCCTGCATCTGTAGGAGACAGAGTCACCATCACTTGCCGGGCAAGTCAGAACATTAGAAAGTATTTACATTGGTATCAGTTCTTACCAGGGAAAGCCCCTAAGCTCCTGATCTATACTGCATCCACTTTACAAAGTGGGGTCCCATCAAGGTTCAGTGGCAGCGGATCTGGGACAGATTTCACTCTCACCATCAGCAGTCTGCAACCTGAAGATTTCGCAACTTACTACTGTCAACAGAGTTACAATATTTCATTCACTTTCGGCCCTGGGACCAAAGTGGATATCAAA | CAGGTGCAGCTGCAGGAGTCGGGCCCAGGACTGGTGAAGCCTTCGGAGACCCTGTCCCTCACCTGCACTGTCTCTGGTGGCTCCATCAGTAGTCACTTCTGGAGCTGGATCCGGCAGCCCGCCGGGAAGGGACTGGAGTGGATTGGCCGTATCTATACCAGTGGGACCACCAACTACACCCCCTCCCTCAAGAGTCGAGTCACCATGTCAGTAGACACGTCCAAGAACCAGTTCTCCCTGAAGCTGAGCTCTGTGACCGCCGCGGACACGGCCGTGTATTACTGTGCGAGATTGAGCAGTGGCTGGTTTGACTACTGGGGCCAGGGAACCCTGGTCACCGTCTCCTCA |
| | | | SEQ ID NO: 26353 | SEQ ID NO: 30359 |
| | | AA | DIQMTQSPSSLPASVGDRVTITCRASQNIRKYLHWYQFLPGKAPKLLIYTASTLQSGVPSRFSGSGSGTDFTLTISSLQPEDFATYYCQQSYNISFTFGPGTKVDIK | QVQLQESGPGLVKPSETLSLTCTVSGGSISSHFWSWIRQPAGKGLEWIGRIYTSGTTNYTPSLKSRVTMSVDTSKNQFSLKLSSVTAADTAVYYCARLSSGWFDYWGQGTLVTVSS |
| | | | SEQ ID NO: 26354 | SEQ ID NO: 30360 |

FIGURE 50

| | | | | |
|---|---|---|---|---|
| **iPS:435547** | **21-225_158F5** | NA | GACATTCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCAATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGGATAATAGTCACCCATTCACTTTCGGCCCTGGGACCAAAGTGGAAATCAAA | GAGGTGCAACTGGTGGAGTCTGGGGGAGGCCTGGTCAAGCCTGGGGGGTCCCTGAGATTCTCCTGTGCAGCCTCTGGATTCACCTTCAGTAGTTATAGCATGAACTGGGTCCGCCAGGCTCCAGGGAAGGGGCTGGAGTGGGTCTCATCCATTAGTGGTAGTAGTACGTACATATACTACGCAGACTCAGTGAAGGGCCGATTCACCATCTCCAGAGACAACGCCAAGAACTCACTGTATCTGCAAATGAACAGCCTGAGAGCCGAAGACACGGCTGTGTATTACTGTGCGAGAGATCGGGGCAGCAGCTGGGGCCAGGGAACCCTGGTCACCGTCTCCTCA |
| | | | SEQ ID NO: 26355 | SEQ ID NO: 30361 |
| | | AA | DIQMTQSPSSLSASVGDRVTITCRASQGIRNDLGWYQQKPGKAPKRLINAASSLQSGVPSRFSGSGSGTEFTLTISSLQPEDFATYYCLQDNSHPFTFGPGTKVEIK | EVQLVESGGGLVKPGGSLRFSCAASGFTFSSYSMNWVRQAPGKGLEWVSSISGSSTYIYYADSVKGRFTISRDNAKNSLYLQMNSLRAEDTAVYYCARDRGSSWGQGTLVTVSS |
| | | | SEQ ID NO: 26356 | SEQ ID NO: 30362 |
| **iPS:435549** | **21-225_158H5** | NA | GACATCCAGATGATCCAGTCTCCATCCTTCCTGTTTGCATCTGTAGGAGACAGAGTTACCATCACTTGCCGGGCAAGTCAGGGCATGAGAATTGATTTAGGGTGGTATCAGCAGAAACCAGGAAAAGCCCCTAAGCGCCTGATTTATCGTGCATCCAGTTTGCAAAGTGGGGTCCCATCAAGGTTCAGCGGCAGTGGATGTGGGACAGAATTCACTCTCACAATCAGCAGCGTGCAGCGTGAAGATTTTGCAAGTTATTACTGTGTACAGCATAATAGTTACCCTCTCACTTTCGGCGGAGGGACCAAGGTGGAGATCAAA | GAGGTGCAACTGGTGGAGTCTGGGGGAGGCCTGGTCAAGCCTGGGGGGTCCCTGAGACTCTCCTGTGCAGCCTCTGGGTTCACCTTCAGTAGCTATAGCATGAACTGGGTCCGCCAGGCTCCAGGGAAGGGGCTGGAGTGGGTCTCATCCATCAGTGGTAGTAGTACTTACATATACTACGCAGACTCAGTGAAGGGCCGATTCACCATCTCCAGAGACAACGCCAAGAACTCACTGCATCTGCAAATGAACAGCCTGAGAGCCGAGGACACGGCTGTGTATTACTGTGCGAGAGATCGGGGCAGCAGCTGGGGCCAGGGAACCCTGGTCACCGTCTCCTCA |
| | | | SEQ ID NO: 26357 | SEQ ID NO: 30363 |

FIGURE 50

| | | | | |
|---|---|---|---|---|
| | | AA | DIQMIQSPSFLFASVGDRVTITCRASQGMRIDLGWYQQKPGKAPKRLIYRASSLQSGVPSRFSGSGCGTEFTLTISSVQREDFASYYCVQHNSYPLTFGGGTKVEIK | EVQLVESGGGLVKPGGSLRLSCAASGFTFSSYSMNWVRQAPGKGLEWVSSISGSSTYIYYADSVKGRFTISRDNAKNSLHLQMNSLRAEDTAVYYCARDRGSSWGQGTLVTVSS |
| | | | SEQ ID NO: 26358 | SEQ ID NO: 30364 |
| iPS:435551 | 21-225_158H6 | NA | GACATCCAGATGACCCAGTCTCCATCCTCCCTGTCTGCATCTGTAGGAGACAGAGTCACCATCACTTGCCGGGCAAGTCAGGGCATTAGAAGTGATTTAGGCTGGTATCAGCAGAAACCAGGGAAAGCCCCTAAGCGCCTGATCTATACTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGACATCAAA | CAGGTGCAGCTGGTGGAGTCTGGGGGAGGCGTGGTCCAGCCTGGGAGGTCCCTGAGACTCTCCTGTGCAGTGTCTGGATTCACCTTCAGTAGCTATGGCATGCACTGGGTCCGCCAGGCTCCAGGCAAGGGGCTGGAGTGGGTGGCAGTTATATGGTATGATGTAACTAATAAATACTATGCAGACTCCGTGAAGGGCCGATTCACCATCTCCAGAGACAATTCCAAGAACACGCTGTATCTGCAAATGAACAGCCTGAGAGACGAGGACACGGCTGTGTATTATTGTGTGAGAGAACTGGGATGGGCGGAGGACTACTGGGGCCAGGGAACCCTGGTCACCGTCTCCTCA |
| | | | SEQ ID NO: 26359 | SEQ ID NO: 30365 |
| | | AA | DIQMTQSPSSLSASVGDRVTITCRASQGIRSDLGWYQQKPGKAPKRLIYTASSLQSGVPSRFSGSGSGTEFTLTISSLQPEDFATYYCLQHNSYPFTFGPGTKVDIK | QVQLVESGGGVVQPGRSLRLSCAVSGFTFSSYGMHWVRQAPGKGLEWVAVIWYDVTNKYYADSVKGRFTISRDNSKNTLYLQMNSLRDEDTAVYYCVRELGWAEDYWGQGTLVTVSS |
| | | | SEQ ID NO: 26360 | SEQ ID NO: 30366 |

FIGURE 50

| | | | | |
|---|---|---|---|---|
| **iPS:435553** | **21-225_158G8** | NA | GACATCCAGATGACCCAGTCTCCATCCTCCCT<br>GTCTGCATCTGTAGGAGACAGAGTCACCATCA<br>CTTGCCGGGCAAGTCAGGACATTAGAAATGAT<br>TTAGGCTGGTATCAGCAGAAACCAGGGAAAG<br>CCCCTAAGCGCCTGATGTATACTGCATCCAGT<br>TTGCAAAGTGGGGTCCCATCAAGGTTCAGCGG<br>CAGTGGATCTGGGACAGAATTCACTCTCACAA<br>TCAGCAGCCTGCAGCCTGAAGATTTTGCAACT<br>TATTACTGTCTACAGCATAATAGTTACCCATTC<br>ACTTTCGGCCCTGGGACCAAAGTGGATATCAA<br>A | GAGGTGCAGCTGGTGGAGTCTGGGGGAGGCCTG<br>GTCACGCCTGGGGGGTCCCTGAGACTCTCCTGTG<br>CAGCCTCTGGATTCACCTTCAGTAGCTATACCAT<br>GAACTGGGTCCGCCAGGCTCCAGGGAAGGGGCT<br>GGAGTGGGTCTCATTGATTAGTGGCAGTAGTAGT<br>TACATATACTACGCAGACTCAGTGAAGGGCCGAT<br>TCACCATCTCCAGAGACAACGCCAAGAACTCACT<br>GTATCTGCAAATGAACAGCCTGAGAGCCGAGGA<br>CACGGCTGTGTATTACTGTGCGAGAGATCGAGGC<br>AGCCTCTGGGGCCAGGGAACCCTGGTCACCGTCT<br>CCTCA |
| | | | SEQ ID NO: 26361 | SEQ ID NO: 30367 |
| | | AA | DIQMTQSPSSLSASVGDRVTITCRASQDIRNDLG<br>WYQQKPGKAPKRLMYTASSLQSGVPSRFSGSGS<br>GTEFTLTISSLQPEDFATYYCLQHNSYPFTFGPGT<br>KVDIK | EVQLVESGGGLVTPGGSLRLSCAASGFTFSSYTMN<br>WVRQAPGKGLEWVSLISGSSSYIYYADSVKGRFTIS<br>RDNAKNSLYLQMNSLRAEDTAVYYCARDRGSLW<br>GQGTLVTVSS |
| | | | SEQ ID NO: 26362 | SEQ ID NO: 30368 |
| **iPS:435557** | **21-225_158B12** | NA | GACATCGTGATGACCCAGTCTCCAGACTCCCC<br>GGCTGTGTCTCTGGGCGAGAGGGCCACCATCA<br>ACTGCAAGTCCAGCCAGAATGTTTTACACAGC<br>TCCAACAATAACAACTACTTAACTTGGTACCA<br>GCAGAGACCAGGACAGCCTCCTAAGTTGCTCA<br>TTTACTGGGCATCTACCCGGGAATCCGGGGTC<br>CCTGACCGATTCAGTGGCAGCGGGTCTGGGAC<br>AGAATTCACTCTCACCATCAGCAGCCTGCAGG<br>CTGAAGATGTGGCAGTTTATTACTGTCAGCAA<br>TATTATAGTACTCCTCCGACGTTCGGCCAAGG<br>GACCAAGGTGGAAATCAAA | CAGGTGCAGCTGGTGCAGTCTGGGGCTGAGGTG<br>AGGAAGCCTGGGGCCTCAGTGAAGGTCTCCTGC<br>AAGGCTTCTGGATACACCTTCACCAATTATGATA<br>TCAACTGGGTGCGACAGGCCACTGGACAAGGGC<br>TTGAGTGGATGGGATGGATGCACCCTAACAGTGG<br>TAACACAGGCTATGCACAGAAGTTCCAGGGCAG<br>ATTCACCATGACCAGGAACACCTCCATAAGCACA<br>GCCTACATGGAGCTGAGCAGCCTGAGATCTGAG<br>GACACGGCCGTGTATTACTGTGCCTATAGCAGTG<br>GCTGGTACCGCTTTGACTACTGGGGCCAGGGAAC<br>CCTGGTCACCGTCTCCTCA |
| | | | SEQ ID NO: 26363 | SEQ ID NO: 30369 |

FIGURE 50

| | | | | |
|---|---|---|---|---|
| | | AA | DIVMTQSPDSPAVSLGERATINCKSSQNVLHSSNNNNYLTWYQQRPGQPPKLLIYWASTRESGVPDRFSGSGSGTEFTLTISSLQAEDVAVYYCQQYYSTPPTFGQGTKVEIK | QVQLVQSGAEVRKPGASVKVSCKASGYTFTNYDINWVRQATGQGLEWMGWMHPNSGNTGYAQKFQGRFTMTRNTSISTAYMELSSLRSEDTAVYYCAYSSGWYRFDYWGQGTLVTVSS |
| | | | SEQ ID NO: 26364 | SEQ ID NO: 30370 |
| **iPS:435559** | **21-225_158H12** | NA | GACATCCAGATGACCCAGTCTCCATCCTCACTGTCTGCATCTGTAGGAGACAGAGTCACCATCACTTGTCGGGCGAGTCAGGGCATTAACAATTATTTAGCCTGGTTTCAGCAGAAACCAGGGAAAGCCCCTAAGTCCCTGATCTCTGCTGCATCCAGTTTACAAAGTGGGGTCCCATCAAAGTTCAGCGGCAGTGGATCTGGGACAGATTTCACTCTCAGTATCAGCAGCCTACAGCCTGAAGATTTTGCAACTTATTACTGTCAACAGTATCATAGTTACCCATTCACTTTCGGCCCTGGGACCAAAGTGGATATCAAA | GAGGTGCAGCTGGTGGAGTCTGGGGGAGGCTTGGTACAGCCTGGGGGGTCCCTGAGACTCTCCTGTGCAGCCTCTGGATTCACCTTTAGCAGCTATGTCATGAGCTGGGTCCGCCAGGCTCCAGGGAAGGGGCTGGAGTGGGTCTCAGCTATTAGTGGTAGTGGTGGTAGGACAGACTACGCAGACTCCGTAAAGGGCCGGTTCACCATCTCCAGAGACAATTCCAAGAACACGCTGTATCTGCAAATGAACAGCCTGAGAGCCGAGGACACGGCCGTATATTACTGTGCGAAAGGGGGCTGGAACCACGACTGGGGCCAAGGGACCACGGTCACCGTCTCCTCA |
| | | | SEQ ID NO: 26365 | SEQ ID NO: 30371 |
| | | AA | DIQMTQSPSSLSASVGDRVTITCRASQGINNYLAWFQQKPGKAPKSLISAASSLQSGVPSKFSGSGSGTDFTLSISSLQPEDFATYYCQQYHSYPFTFGPGTKVDIK | EVQLVESGGGLVQPGGSLRLSCAASGFTFSSYVMSWVRQAPGKGLEWVSAISGSGGRTDYADSVKGRFTISRDNSKNTLYLQMNSLRAEDTAVYYCAKGGWNHDWGQGTTVTVSS |
| | | | SEQ ID NO: 26366 | SEQ ID NO: 30372 |

FIGURE 50

| | | | | |
|---|---|---|---|---|
| **iPS:435561** | **21-225_159F1** | NA | GACATCCAGATGACCCAGTCTCCATCCTCCCTGTCTGCATCGGTAGGAGACAGAGTCACCATCACTTGCCGGGCAAGTCAGCGCGTCAGAAATGATTTAGGCTGGTATCAGCAGAAACCAGCGAAAGCCCCTAAGCGCATCATTTTCGATGCATCCAATTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATCATAGTTTCCCGATCACCTTCGGCCAAGGGACCCGACTGGAGATTAAA | GAGGTGCAGCTGGTGGAGTCTGGGGGAGGCCTGGTCAAGCCGGGGGGGTCCCTGAGACTCTCCTGTGCAGCCTCTGGATTCACCTTCAGTAGCTATAGAATGAACTGGGTCCGCCAGGCTCCAGGGAAGGGGCTGGAGTGGGTCTCATCCATAAGTGGTAGTGGTAATTACATAGACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGGTTGGGACGTCTGGGGCCAAGGGACCACGGTCACCGTCTCCTCA |
| | | | SEQ ID NO: 26367 | SEQ ID NO: 30373 |
| | | AA | DIQMTQSPSSLSASVGDRVTITCRASQRVRNDLGWYQQKPAKAPKRIIFDASNLQSGVPSRFSGSGSGTEFTLTISSLQPEDFATYYCLQHHSFPITFGQGTRLEIK | EVQLVESGGGLVKPGGSLRLSCAASGFTFSSYRMNWVRQAPGKGLEWVSSISGSGNYIDYADSVKGRFTISRDNAKNSLYLQMNSLRAEDTAVYYCARGWDVWGQGTTVTVSS |
| | | | SEQ ID NO: 26368 | SEQ ID NO: 30374 |
| **iPS:435563** | **21-225_159H2** | NA | GACATCCAGTTGACCCAGTCTCCATCCTCCCTGTCTGCATCTGTAGGAGACAGAGTCACCATCACTTGCCGGGCAAGTCAGAGCATTAGCAAATATTTAAATTGGTATCAGCAGAAACCAGGGAAAGCCCCTGAACTCCTGATCTATGCTACATCCAATTTGCAAAGTGGGGTCCCATCAAGGTTCAGTGGCAGTGGATCTGGGACAGATTTCACTCTCACCATCAGCACTCTGCAACCTGAAGATTTTGTAACTTACTACTGTCAACAGAGTTACAGTCTCCCGGTCACTTTCGGCGGAGGGACCAAGGTAGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAGTTATGATATCAACTGGGTGCGACAGGCCACTGGACAAGGGCTTGAGTGGATGGGATGGATGAACCCTAACAGTGGTAACACAGGCTATGTACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGAGAAAGAAAACTGGGGACTACTGGGGCCAGGGAACCCTGGTCACCGTCTCCTCA |
| | | | SEQ ID NO: 26369 | SEQ ID NO: 30375 |

FIGURE 50

| | | | | |
|---|---|---|---|---|
| | | AA | DIQLTQSPSSLSASVGDRVTITCRASQSISKYLNWYQQKPGKAPELLIYATSNLQSGVPSRFSGSGSGTDFTLTISTLQPEDFVTYYCQQSYSLPVTFGGGTKVEIK | QVQLVQSGAEVKKPGASVKVSCKASGYTFTSYDINWVRQATGQGLEWMGWMNPNSGNTGYVQKFQGRVTMTRNTSISTAYMELSSLRSEDTAVYYCARKKTGDYWGQGTLVTVSS |
| | | | SEQ ID NO: 26370 | SEQ ID NO: 30376 |
| **iPS:435565** | **21-225_159C4** | NA | GACATCCAGATGACCCAGTCTCCATCCTCCCTGTCTGCATCTGTAGGAGACAGAGTCACCATCACTTGCCAGGCGAGTCAGGACATTAGCGACTATTTAAATTGGTATCAACAGAAACCAGGGAAAGCCCCTAAACTCCTGATCTACGATGCCTCCACTTTGGAAACAGGGGTCCCATCAAGGTTCAGTGGAAGTGGATCTGGGACAGATTTTACTTTCACCATCAGCAGCCTGCAGCCTGAAGATATTGCAACATATTTCTGTCAACAATATGATAATCTCCCGATCACCTTCGGCCAAGGGACACGACTGGAGATTAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAACTATGGCATGCACTGGGTCCGCCAGGCTCCAGGCAAGGGGCTGGAGTGGGTGGCAGTTATATCATATTCTGGAAACAATAAATACTATGCAGACTCCGTGAAGGGCCGATTCACCATCTCCAGAGACAATTCCAAGAACACGCTGTATCTGCAGTTGAACAGCCTGAGAGCTGAGGACATGGCTGTGTATTACTGTGCGAGACGGAGCAGCTCGTGGGGGGGCTACGGTATGGACGTCTGGGGCCACGGGACCACGGTCACCGTCTCCTCA |
| | | | SEQ ID NO: 26371 | SEQ ID NO: 30377 |
| | | AA | DIQMTQSPSSLSASVGDRVTITCQASQDISDYLNWYQQKPGKAPKLLIYDASTLETGVPSRFSGSGSGTDFTFTISSLQPEDIATYFCQQYDNLPITFGQGTRLEIK | QVQLVESGGGVVQPGRSLRLSCAASGFTFSNYGMHWVRQAPGKGLEWVAVISYSGNNKYYADSVKGRFTISRDNSKNTLYLQLNSLRAEDMAVYYCARRSSSWGGYGMDVWGHGTTVTVSS |
| | | | SEQ ID NO: 26372 | SEQ ID NO: 30378 |

FIGURE 50

| | | | | |
|---|---|---|---|---|
| **iPS:435569** | **21-225_159C5** | NA | GACATCCAGATGACCCAGTCTCCATCCTCCCTGTCTGCATCTGTAGGAGACAGAGTCACCATCACTTGCCGGACAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGACTTCACTCTCACAATCATCAGCCTGCAGCCTGATGACTTTGCAACTTATTACTGTCTACAGCATAATAGTTATCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATACACTGGGTCCGCCAGGCTCCAGGCAAGGGGCTGGAGTGGGTGGCTGTTGTATGGTATGATGTAAATAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGCTGGGGTTCCTCTCTGACTACTGGGGCCAGGGAACCCTGGTCACCGTCTCCTCA |
| | | | SEQ ID NO: 26373 | SEQ ID NO: 30379 |
| | | AA | DIQMTQSPSSLSASVGDRVTITCRTSQGIRNDLGWYQQKPGKAPKRLIYAASSLQSGVPSRFSGSGSGTDFTLTIISLQPDDFATYYCLQHNSYPFTFGPGTKVDIK | QVQLVESGGGVVQPGRSLRLSCAASGFTFSSYGIHWVRQAPGKGLEWVAVVWYDVNNKYYADSVKGRFTISRDNSKNTLYLQMNSLRAEDTAVYYCARELGFLSDYWGQGTLVTVSS |
| | | | SEQ ID NO: 26374 | SEQ ID NO: 30380 |
| **iPS:435571** | **21-225_159C8** | NA | GACATCCAGATGACCCAGTCTCCATCCTCCCTGTCTGCATCTGTAGGAGACAGAGTCACCATCACTTGCCGGGCAAGTCAGGACATTAGAAAGGATTTAGGGTGGTATCAGCAGAAACCAGGGAAAGCCCCTAACCGCCTGATCTATGCTGCATCCAGTTTGCAAAGTGGGGTCCCATCAAGGTTCAGCGGCAGTGGATCTGGGACAGAATTCACTCTCACATTCAGCAGCCTGCAGCCTGAAGATTTTGCAACTTATTACTGTCTACAGCATCATAGTTATCCTCGGACGTTCGGCCAAGGGACCAAGGTGGAAATCAAA | CAGGTGCAACTGGTGGAGTCTGGGGGAGGCGTGGTCCAGCCTGGGAGGTCCCTGAGACTCTCCTGTGCAGCGTCTGGATTCACCTTTAGTGACTATGTCATGCAGTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACCGTATAATAGTGGCTGGTACGACTACGGTATGGACGTCTGGGGCCAAGGGACCACGGTCACCGTCTCCTCA |
| | | | SEQ ID NO: 26375 | SEQ ID NO: 30381 |

FIGURE 50

| | | | | |
|---|---|---|---|---|
| | | AA | DIQMTQSPSSLSASVGDRVTITCRASQDIRKDLGWYQQKPGKAPNRLIYAASSLQSGVPSRFSGSGSGTEFTLTFSSLQPEDFATYYCLQHHSYPRTFGQGTKVEIK | QVQLVESGGGVVQPGRSLRLSCAASGFTFSDYVMQWVRQAPGKGLEWVAVIWYDGSNKYYADSVKGRFTISRDNSKNTLYLQMNSLRAEDTAVYYCAREPYNSGWYDYGMDVWGQGTTVTVSS |
| | | | SEQ ID NO: 26376 | SEQ ID NO: 30382 |
| **iPS:435573** | **21-225_159D8** | NA | GACATCCAGATGACCCAGTCTCCATCCTCCCTGTCTGCATCTGTTGGAGACAGAGTCACCATCACTTGCCGGGCAAGTCGGGACATTGGAAATGATTTAGGCTGGTATCAGCAGAAACCAGGGAAAGCCCCTAAGCGCCTGATCTCTGCTGCATCCAGTTTGCAAAGTGGGGTCCCATCAAGGTTCAGCGGCAGTGGATCTGGGACAGAATTCACTCTCACATTCAGCAGCCTGCAGCCTGAAGATTTTGCAACTTATTACTGTCTACAGCATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGCGCAGCGTCTGGATTCACCTTCAGTAACTATGTCATGCACTGTGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACCGTATAGTAGTGGCTGGTACGACTACGGTATGGACGTCTGGGGCCAAGGGACCACGGTCACCGTCTCCTCA |
| | | | SEQ ID NO: 26377 | SEQ ID NO: 30383 |
| | | AA | DIQMTQSPSSLSASVGDRVTITCRASRDIGNDLGWYQQKPGKAPKRLISAASSLQSGVPSRFSGSGSGTEFTLTFSSLQPEDFATYYCLQHYSYPRTFGQGTKVEIK | QVQLVESGGGVVQPGRSLRLSCAASGFTFSNYVMHCVRQAPGKGLEWVAVIWYDGSNKYYADSVKGRFTISRDNSKNTLYLQMNSLRAEDTAVYYCAREPYSSGWYDYGMDVWGQGTTVTVSS |
| | | | SEQ ID NO: 26378 | SEQ ID NO: 30384 |

FIGURE 50

| | | | | |
|---|---|---|---|---|
| **iPS:435575** | **21-225_159H11** | NA | GACATCCAGATGACCCAGTCTCCATCCTCACTGTCTGCATCTGTAGGAGACAGAGTCACCATCACTTGTCGGGCGAGTCAGGGCATTAGCAAATATTTAGTCTGGTTTCAGCAGAAACCAGGGAAAGCCCCTAAGTCCCTGATCTATGCTGCATCCAGTCTGCAAAGTGGGGTCCCATCAAAGTTCAGCGGCAGTGGATCTGGGACAGATTTCACTCTCACCATCAGCAACCTGCAGCCTGAAGATTTTGCAACTTATTACTGCCAACAGTATAATAGTTACCCATTCACTTTCGGCCCTGGGACCAAAGTGGATATCAAA | GAGGTGCAGCTGGTGGAGTCTGGGGGAGGCCTGGTCAAGCCTGGGGGGTCCCTAAGACTCTCCTGTGCAGCCTCTGGATTCACCTTCAGTAGCTATACCATGAACTGGGTCCGCCAGGCTCCAGGGAAGGGGCTGGAGTGGGTCTCATCCATTAGTGGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTATATTACTGTGCGCGAGTGAGCTGGGCTGACTGCTGGGGCCAGGGAACCCTGGTCACCGTCTCCTCA |
| | | | SEQ ID NO: 26379 | SEQ ID NO: 30385 |
| | | AA | DIQMTQSPSSLSASVGDRVTITCRASQGISKYLVWFQQKPGKAPKSLIYAASSLQSGVPSKFSGSGSGTDFTLTISNLQPEDFATYYCQQYNSYPFTFGPGTKVDIK | EVQLVESGGGLVKPGGSLRLSCAASGFTFSSYTMNWVRQAPGKGLEWVSSISGSSSYIYYADSVKGRFTISRDNAKNSLYLQMNSLRAEDTAVYYCARVSWADCWGQGTLVTVSS |
| | | | SEQ ID NO: 26380 | SEQ ID NO: 30386 |
| **iPS:435577** | **21-225_160B1** | NA | GATATTGTGATGACCCAGACTCCACTCTCTCTGTCCGTCACCCCTGGACAGCCGGCCTCCATCTCCTGCAAGTCTAGTCAGAGCCTCCTGCATGGTGATGGGAAGACCTATTTCTATTGGTACCTGCAGAAGCCAGGCCAGCCTCCACAGGTCCTGATCTATGAAGTATCCAAGCGGTTCTCTGGAGTGTCAGAAAGGTTCAGTGGCAGCGGGTCAGGGACAGATTTCACACTGAAAATCAGCCGGGTGGAGGCTGAGGATGTTGGGGTTTATTACTGCATGCAAAGTATACAGATTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTTATAAATACTATGCAGACTCCGTGAAGGGCCGATTCACCATCTCCAGAGACAATTCCAAGAACACGCTGTATCTTCAAATGAACAGCCTGAGAGCCGAGGACACGGCTGTGTATTACTGTGCGAGAGAGGCCTACGATTTTTGGAGTGGTTATTATGACTACTGGGGCCAGGGAACCCTGGTCACCGTCTCCTCA |
| | | | SEQ ID NO: 26381 | SEQ ID NO: 30387 |

FIGURE 50

| | | | | |
|---|---|---|---|---|
| | | AA | DIVMTQTPLSLSVTPGQPASISCKSSQSLLHGDGKTYFYWYLQKPGQPPQVLIYEVSKRFSGVSERFSGSGSGTDFTLKISRVEAEDVGVYYCMQSIQIPWTFGQGTKVEIK | QVQLVESGGGVVQPGRSLRLSCAASGFTFSSYGMHWVRQAPGKGLEWVAVIWYDGSYKYYADSVKGRFTISRDNSKNTLYLQMNSLRAEDTAVYYCAREAYDFWSGYYDYWGQGTLVTVSS |
| | | | SEQ ID NO: 26382 | SEQ ID NO: 30388 |
| **iPS:435579** | **21-225_160G1** | NA | GACATCCAGATGACCCAGTCTCCATCCTCGCTGTCTGCCTCTGTAGGAGACAGAGTCACCATCACTTGTCGGGCGAGTCAGGACATTAACAATTATTTAGCCTGGTTTCAGCAGAAACCAGGAAAAGCCCCTACGTCCCTGATCTATGCTTCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAATATCATAGTTACCCATTCACTTTCGGCCCTGGGACCAAAGTGGATATCAGA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGTCATGAGCTGGGTCCGCCAGGCTCCAGGGAAGGGGCTGGAGTGGGTCTCAGCTATGAGTGGTAGTGGTGGTCACACATACTACGCAGACTCCGTGAAGGGCCGGTTCACCATCTCCAGAGACAATTCCAAGAACACGGTGTATCTGCAAATGAACAGCCTGAGAGCCGAGGACACGGCCGTATATTACTGTGTGAAACATGGATACAGCTGGGGCCAGGGAACCCTGGTCACCGTCTCCTCA |
| | | | SEQ ID NO: 26383 | SEQ ID NO: 30389 |
| | | AA | DIQMTQSPSSLSASVGDRVTITCRASQDINNYLAWFQQKPGKAPTSLIYASSSLQSGVPSKFSGSGSGTDFTLTISSLQPEDFATYYCQQYHSYPFTFGPGTKVDIR | EVQLLESGGGLVQPGGSLRLSCAASGFTFSSYVMSWVRQAPGKGLEWVSAMSGSGGHTYYADSVKGRFTISRDNSKNTVYLQMNSLRAEDTAVYYCVKHGYSWGQGTLVTVSS |
| | | | SEQ ID NO: 26384 | SEQ ID NO: 30390 |

FIGURE 50

| | | | | |
|---|---|---|---|---|
| iPS:435581 | 21-225_160H1 | NA | GACATCCAGATGACCCAGTCTCCATCCTCCCTGTCTGCATCTGTAGGAGACAGAGTCACCATCACTTGCCGGGCAAGTCAGGACATTAGAAATGATTTAGGCTGGTATCAGCAGAAACCAGGGAAAGCCCCTAAGCGCCTGATCTATGCTGCATCCAGTTTGCAGAATGGGGTCCCATCAAGGTTCAGCGGCAGTGGATCTGGGACAGAATTCACTCTCACAATCAGCAGCCTGCAGCCTGAAGATTTTACAACTTATTACTGTCTACAGCATAATAGTTTCCCGTGGACGTTCGGCCAAGGGACCAAGGTGGAAATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATCGCATGAACTGGGTCCGCCAGGCTCCAGGGAAGGGGCTGGAGTGGGTCTCATCCATTAGTAGTAGTACTGGTTACATGTACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTTCTGTGCGAGAGATAAAGATTACTGGGGCCAGGGAACCCTGGTCACCGTCTCCTCA |
| | | | SEQ ID NO: 26385 | SEQ ID NO: 30391 |
| | | AA | DIQMTQSPSSLSASVGDRVTITCRASQDIRNDLGWYQQKPGKAPKRLIYAASSLQNGVPSRFSGSGSGTEFTLTISSLQPEDFTTYYCLQHNSFPWTFGQGTKVEIK | EVQLVESGGGLVKPGGSLRLSCAASGFTFSSYRMNWVRQAPGKGLEWVSSISSSTGYMYYADSVKGRFTISRDNAKNSLYLQMNSLRAEDTAVYFCARDKDYWGQGTLVTVSS |
| | | | SEQ ID NO: 26386 | SEQ ID NO: 30392 |
| iPS:435583 | 21-225_160F2 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATACTGCATCCAATTTGCAAAGTGGGGTCCCATCAAGGTTCAGCGGCAGTGGATCTGGGACAGAATTCACTCTCACAGTCAGCAGCCTGCAGCCTGAAGATTTTGCAACTTATTACTGTCTACAGCATAATAGTTACCCGTGGACGTTCGGCCAAGGGACCAAGGTGGAAATCAAA | GAGGTGCAGCTGGTGGAATCTGGGGGAGGCCTGGTCAAGCCTGGGGGGTCCCTGAGACTCTCCTGTGCAGCCTCTGGATTCACCTTCAGTAGTTATGGCATGAACTGGGTCCGCCAGGCTCCAGGGAAGGGGCTGGAGTGGATCTCATCCATTAGTGGTAGTGGTAGTTACATATACTACGCAGACTCAGTGAAGGGCCGATTCACCATTTCCAGAGACAACGCCAAGAACTCACTGTATCTGCAAATGAACAGCCTGAGAGCCGAGGACACGGCTGTGTATTACTGTGCGATTAGCAGTGGCTGGTCTTGGGGCCAGGGAACCCTGGTCACCGTCTCCTCA |
| | | | SEQ ID NO: 26387 | SEQ ID NO: 30393 |

FIGURE 50

| | | | | |
|---|---|---|---|---|
| | | AA | DIQMTQSPSSLSASVGDRVTITCRASQGIRNDLGWYQQKPGKAPKRLIYTASNLQSGVPSRFSGSGSGTEFTLTVSSLQPEDFATYYCLQHNSYPWTFGQGTKVEIK | EVQLVESGGGLVKPGGSLRLSCAASGFTFSSYGMNWVRQAPGKGLEWISSISGSGSYIYYADSVKGRFTISRDNAKNSLYLQMNSLRAEDTAVYYCAISSGWSWGQGTLVTVSS |
| | | | SEQ ID NO: 26388 | SEQ ID NO: 30394 |
| **iPS:435585** | **21-225_160G3** | NA | GACATCCAGATGACCCAGTCTCCATCCTCGCTGTCTGCCTCTGTAGGAGACAGAGTCACCATCACTTGTCGGGCGAGTCAGGACATTAACAATTATTTAGCCTGGTTTCAGCAGAGACCAGGGAAAGCCCCTACGTCCCTGATCTATGCTTCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAATATCATAGTTACCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGTCATGAGCTGGGTCCGCCAGACTCCAGGGAAGGGACTGGAGTGGGTCTCAGCTATGAGTGGTAGTGGTGGTCACACATACTACGCAGACTCCGTGAAGGGCCGGTTCACCATCTCCAGAGACAATTCCAAGAACACGCTGTATCTGCAAATGAACAGCCTGAGAGCCGAGGACACGGCCATATATTACTGTGTGAAACATGGATACAGCTGGGGCCAGGGAACCCTGGTCACCGTCTCCTCA |
| | | | SEQ ID NO: 26389 | SEQ ID NO: 30395 |
| | | AA | DIQMTQSPSSLSASVGDRVTITCRASQDINNYLAWFQQRPGKAPTSLIYASSSLQSGVPSKFSGSGSGTDFTLTISSLQPEDFATYYCQQYHSYPFTFGPGTKVDIK | EVQLLESGGGLVQPGGSLRLSCAASGFTFSSYVMSWVRQTPGKGLEWVSAMSGSGGHTYYADSVKGRFTISRDNSKNTLYLQMNSLRAEDTAIYYCVKHGYSWGQGTLVTVSS |
| | | | SEQ ID NO: 26390 | SEQ ID NO: 30396 |

FIGURE 50

| | | | | |
|---|---|---|---|---|
| **iPS:435587** | **21-225_160H3** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATTTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAGTAATTACCCGCTCACTTTCGGCGGAGGGACCCAGGTGGAGAGCAAA | CAGCTGCAGCTGCAGGAGTCGGGCCCAGGACTGGTGAAGCCTTCGGAGACCCTGTCCCTCACCTGCACCGTCTCTGGTGGCTCCATCAGCAGAAGTAGTTACTACTGGGGCTGGATCCGCCAGCCCCCAGGGAAGGGACTGGAGTGGATTGGGAGTATCTATTATAGTGGGAGTACCTCCTACAACCCGTCTCTCGAGAGTCGAGTTACCATATCCGTAGACACGTCCAAGAACCAGTTCTCCCTGAAGCTGAGCTCTGTGACCGCCGCCGACACGGCTGTGTTTTACTGTGCGAGACTCTCTCAACGGTGGGACTTTGACTACTGGGGCCAGGGAACCCTGGTCACCGTCTCCTCA |
| | | | SEQ ID NO: 26391 | SEQ ID NO: 30397 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHSNYPLTFGGGTQVESK | QLQLQESGPGLVKPSETLSLTCTVSGGSISRSSYYWGWIRQPPGKGLEWIGSIYYSGSTSYNPSLESRVTISVDTSKNQFSLKLSSVTAADTAVFYCARLSQRWDFDYWGQGTLVTVSS |
| | | | SEQ ID NO: 26392 | SEQ ID NO: 30398 |
| **iPS:435589** | **21-225_160A4** | NA | GACATCGTGATGACCCAGTCTCCAGACTCCCTGGCTGTGTCTCTGGGCGAGAGGGCCACCATCAACTGCAAGTCCAGCCAGAGTGTTTTACACAGCTCCAACAATAATAACTACTTAGCTTGGTACCAGCAGAAACCAGGACAGCCTCCTAAGCTACTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAATAGTCCGTGCAGTTTTGGCCAGGGGACCAAGCTGGAGATCAAA | CAGGTGCAGCTGGTGCAGTCTGGGACTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGACTGAGTGGATGGGATGGATGCACCCTAACAGTGGTAACACAGGCTATCCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGTATAGCAGCGGCTGGTACATTTTTGACTACTGGGGCCAGGGAACCCTGGTCACCGTCTCCTCA |
| | | | SEQ ID NO: 26393 | SEQ ID NO: 30399 |

FIGURE 50

| | | | | |
|---|---|---|---|---|
| | | AA | DIVMTQSPDSLAVSLGERATINCKSSQSVLHSSN NNNYLAWYQQKPGQPPKLLIYWASTRESGVPD RFSGSGSGTDFTLTISSLQAEDVAVYYCQQYYNS PCSFGQGTKLEIK | QVQLVQSGTEVKKPGASVKVSCKASGYTFTNYDIN WVRQATGQGPEWMGWMHPNSGNTGYPQKFQGR VTMTRNTSISTAYMELSSLRSEDTAVYYCAYSSGW YIFDYWGQGTLVTVSS |
| | | | SEQ ID NO: 26394 | SEQ ID NO: 30400 |
| iPS:435591 | 21-225_160C4 | NA | GACATCCAGATGACCCAGTCTCCATCCTCCCT GTCTGCATCTGTAGGAGACAGAGTCACCATCA CTTGCCGGGCAAGTCAGGACATTAGAAAGGAT TTAGGGTGGTATCAGCAGAAACCAGGGAAAG CCCCTAACCGCCTGATCTATGCTGCATCCAGTT TGCAAAGTGGGGTCCCATCAAGGTTCAGCGGC AGTGGATCTGGGACAGAATTCACTCTCACATT CAGCAGCCTGCAGCCTGAAGATTTTGCAACTT ATTACTGTCTACAGCATTATAGTTATCCTCGGA CGTTCGGCCAAGGGACCAAGGTGGAAATCAA A | CAGGTGCAACTGGTGGAGTCTGGGGGAGGCGTG GTCCAGCCTGGGAGGTCCCTGAGACTCTCCTGTG CAGCGTCTGGATTCACCTTTAGTGACTATGTCAT GCAGTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAACCGTA TAATAGTGGCTGGTACGACTACGGTATGGACGTC TGGGGCCAAGGGACCACGGTCACCGTCTCCTCA |
| | | | SEQ ID NO: 26395 | SEQ ID NO: 30401 |
| | | AA | DIQMTQSPSSLSASVGDRVTITCRASQDIRKDLG WYQQKPGKAPNRLIYAASSLQSGVPSRFSGSGS GTEFTLTFSSLQPEDFATYYCLQHYSYPRTFGQG TKVEIK | QVQLVESGGGVVQPGRSLRLSCAASGFTFSDYVM QWVRQAPGKGLEWVAVIWYDGSNKYYADSVKGR FTISRDNSKNTLYLQMNSLRAEDTAVYYCAREPYN SGWYDYGMDVWGQGTTVTVSS |
| | | | SEQ ID NO: 26396 | SEQ ID NO: 30402 |

FIGURE 50

| | | | | |
|---|---|---|---|---|
| **iPS:435593** | **21-225_160F4** | NA | GACATTCAGATGACCCAGTCTCCATCCTCCCCGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCTTGATCAATGTTGCATCCAGTTTGCAAAGTGGGGTCCCATCAAGGTTCAGCGGCAGTGGATCTGGGACAGAATTCACTCTTACAATCAGCAGCGTGCAGCCTGAAGATTTTGCAACTTATTACTGTATACAGGATAATAGTCACCCATTCACTTTCGGCCCTGGGACCAAAGTGGAAATCAAA | GAGGTGCAACTGGTGGAGTCTGGGGGAGGCCTGGTCAAGCCTGGGGGGTCCCTGAGATTCTCCTGTGCAGCCTCTGGATTCACCTTCAGTAGTTATAGCATGAACTGGGTCCGCCAGGCTCCAGGGAAGGGGCTGGAGTGGGTCTCATCCATTAGTGGTAGTAGTACGTACATATACTACGCAGACTCAGTGAAGGGCCGATTCACCATCTCCAGAGACAACGCCAAGAACTCACTGTATCTGCAAATGAACAGCCTGAGAGCCGAAGACACGGCTGTGTATTACTGTGCGAGAGATCGGGGCAGCAGCTGGGGCCAGGGAACCCTGGTCACCGTCTCCTCA |
| | | | SEQ ID NO: 26397 | SEQ ID NO: 30403 |
| | | AA | DIQMTQSPSSPSASVGDRVTITCRASQGIRNDLGWYQQKPGKAPKRLINVASSLQSGVPSRFSGSGSGTEFTLTISSVQPEDFATYYCIQDNSHPFTFGPGTKVEIK | EVQLVESGGGLVKPGGSLRFSCAASGFTFSSYSMNWVRQAPGKGLEWVSSISGSSTYIYYADSVKGRFTISRDNAKNSLYLQMNSLRAEDTAVYYCARDRGSSWGQGTLVTVSS |
| | | | SEQ ID NO: 26398 | SEQ ID NO: 30404 |
| **iPS:435595** | **21-225_160H4** | NA | GACATCCAGATGACCCAGTCTCCATCCTCACTGTCTGCATCTGTAGGAGACAGAGTCACCATCACTTGTCGGGCGAGTCAGGACATTAGTAATTATTTAGTCTGGTTTCAGCAGAAATTAGGGAAAGCCCCTAAGTCCCTGATCTATGTTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTATTACTGCCAACAGTATAATAGTTACCCTCTCACTTTCGGCGGAGGGACCAAAGTAGAGATCAAA | GAGGTGCAGCTGGTGGAGTCTGGGGGAGGCCTGGTCAAGTCTGGGGGGTCCCTGAGACTCTCCTGTGCAGCCTCTGGATTCACCTTCAGTAGCTATAGGATGAACTGGGTCCGCCAGGCTCCAGGGAAGGGGCTGGAGTGGGTCTCATCCATTAGTGGTAGTAGTAGTTACATAGACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAAAGAGTTGGTTTGACTACTGGGGCCAGGGAACCCTGGTCACCGTCTCCTCA |
| | | | SEQ ID NO: 26399 | SEQ ID NO: 30405 |

FIGURE 50

| | | | | |
|---|---|---|---|---|
| | | AA | DIQMTQSPSSLSASVGDRVTITCRASQDISNYLV<br>WFQQKLGKAPKSLIYVASSLQSGVPSRFSGSGSG<br>TDFTLTISSLQPEDFATYYCQQYNSYPLTFGGGT<br>KVEIK | EVQLVESGGGLVKSGGSLRLSCAASGFTFSSYRMN<br>WVRQAPGKGLEWVSSISGSSSYIDYADSVKGRFTIS<br>RDNAKNSLYLQMNSLRAEDTAVYYCARKSWFDY<br>WGQGTLVTVSS |
| | | | SEQ ID NO: 26400 | SEQ ID NO: 30406 |
| **iPS:435599** | **21-225_160B10** | NA | GACATCCAGATGACCCAGTCTCCATCCTCCCC<br>GTCTGCATCTGTAGGAGACAGAGTCACCATCA<br>CTTGCCGGGCAAGTCAGGGCATTAGAAATGAT<br>TTAGGCTGGTATCAGCAGAAACCAGGGACAG<br>CCCCTAAGCGCCTTATCTATGCTGCATCCAGTT<br>TGCAAAGTGGGGTCCCATCAAGGTTCAGCGGC<br>AGTGGATCTGGGACAGAATTCACTCTCACAAT<br>CAGCAACCTGCAGCCTGAGGATTTTGCAACTT<br>ATTACTGTCTACAGCATAGTAGTTACCCGCTC<br>ACTTTCGGCGGCGGGACCAAGGTGGAGATCA<br>AA | CAGCTGCAGCTGCAGGAGTCGGGCCCAGGACTG<br>GTGAAGCCTTCGGAGACCCTGTCCCTCACCTGCA<br>CTGTCTCTGGTGGCTCCATCAGTAGAAGTAGCTA<br>CTACTGGGGCTGGATCCGCCAGTACCCAGGGAA<br>GGGGCTGGAGTGGATTGGGAATATCTATTATAGT<br>GGGAGCGCCTACCACATTCCGTCCCTCAAGAGTC<br>GAGTCACCATATCCGTAGACACGTCCAAGAACC<br>AGTTCTCCCTGAAGTTGAACTCTGTGACCGCCGC<br>AGACACGGCTGTGTATTACTGTGTGAGACATGAC<br>CCAAACTGGGGAGTTGACTACTGGGGCCAGGGA<br>ACCCTGGTCACCGTCTCCTCA |
| | | | SEQ ID NO: 26401 | SEQ ID NO: 30407 |
| | | AA | DIQMTQSPSSPSASVGDRVTITCRASQGIRNDLG<br>WYQQKPGTAPKRLIYAASSLQSGVPSRFSGSGSG<br>TEFTLTISNLQPEDFATYYCLQHSSYPLTFGGGT<br>KVEIK | QLQLQESGPGLVKPSETLSLTCTVSGGSISRSSYYW<br>GWIRQYPGKGLEWIGNIYYSGSAYHIPSLKSRVTIS<br>VDTSKNQFSLKLNSVTAADTAVYYCVRHDPNWGV<br>DYWGQGTLVTVSS |
| | | | SEQ ID NO: 26402 | SEQ ID NO: 30408 |

FIGURE 50

| | | | | |
|---|---|---|---|---|
| **iPS:435601** | **21-225_160G10** | NA | GCTATTGTGATGACCCAGACTCCACTCTCTCTGTCCGTCACCCCTGGACAGCCGGCCTCCATCTCCTGCAAGTCTAGTCAGAGCCTCCTGCACGGTGATGGAAAGACCTATTTGTATTGGTACCTGCAGAAGCCAGGCCAGCCTCCACACCTCCTGATCTATGAAGTTTCCAAACGGTTCTCTGGAGTGCCAGATAGGTTCAGTGGCAGCGGGTCAGGGACAGATTTCACACTGAAAATCAGCCGGGTGGAGGCTGAGGATGTTGGGCTTTATTACTGCATGCAAAGTATACAGCTTCCGTGGACGTTTGTCCAAGGGACCAAGGTGGAAATCACA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTTTGGCATGCACTGGGTCCGCCAGGCTCCAGGCAAGGGCCTGGAGTGGGTGGCAGTCATATGGTATGATGGAAGTTATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGTTGGTATAGAAGTGGCTGGTGACTACTACTTCGGTATGGAAGTCTGGGGCCAAGGGACCACGGTCACCGTCTCCTCA |
| | | | SEQ ID NO: 26403 | SEQ ID NO: 30409 |
| | | AA | AIVMTQTPLSLSVTPGQPASISCKSSQSLLHGDGKTYLYWYLQKPGQPPHLLIYEVSKRFSGVPDRFSGSGSGTDFTLKISRVEAEDVGLYYCMQSIQLPWTFVQGTKVEIT | QVQLVESGGGVVQPGRSLRLSCAASGFTFSSFGMHWVRQAPGKGLEWVAVIWYDGSYKYYADSVKGRFTISRDNSKNTLYLQMNSLRAEDTAVYYCARVGIEVAGDYYFGMEVWGQGTTVTVSS |
| | | | SEQ ID NO: 26404 | SEQ ID NO: 30410 |
| **iPS:435605** | **21-225_161A4** | NA | GAAATAGTGATGACGCAGTCTCCAGCCACCCTGTCTGTGTCTCCAGGGGAAAGAGCCTCCCTCTCCTGCAGGTCCAGTCAGAGTGTTAACAGCAACTTAGCCTGGTACCAGCAGAGGCCTGGCCAGGCTCTCAGGCTCCTCATCTATGGTGCATCCATCAGGGCCACTGATATCCCAGCCAGGTTCAATGGCAGTGGGTCTGGGACAGAGTTCACTCTCACCATCAGCAGCCTGCAGTCTGAAGATTTTGCAGTTTATTTCTGTCAGCAGTATAATAACTGGTGGACGTTCGGCCAAGGGACCACGGTGGAAATCAAA | GAGGTGCAGCTGGTGGAGTCTGGAGGAGGCTTGATCCAGCCTGGGGGGTCCCTGAGACTCTCCTGTGCAGCCTCTGGGTTCACCGTCAGTAGCAACTACATGAGCTGGGTCCGCCAGGCTCCAGGGAAGGGGCTGGAGTGGGTCTCAGTTATTTATACCGGTGGTAGCACATACAACGCAGACTCCGTGAAGGGCCGATTCACCATCTCCAGAGACAATTCCAAGAACACGCTGTATCTTCAAATGAACAGCCTGAGAGCCGAGGACACGGCCGTGTATTACTGTGCGAGAAATTGGGGAATGGCTGGCCCCTTTGACTACTGGGGCCAGGGAACCCTGGTCACCGTCTCCTCA |
| | | | SEQ ID NO: 26405 | SEQ ID NO: 30411 |

FIGURE 50

| | | | | |
|---|---|---|---|---|
| | | AA | EIVMTQSPATLSVSPGERASLSCRSSQSVNSNLAWYQQRPGQALRLLIYGASIRATDIPARFNGSGSGTEFTLTISSLQSEDFAVYFCQQYNNWWTFGQGTTVEIK | EVQLVESGGGLIQPGGSLRLSCAASGFTVSSNYMSWVRQAPGKGLEWVSVIYTGGSTYNADSVKGRFTISRDNSKNTLYLQMNSLRAEDTAVYYCARNWGMAGPFDYWGQGTLVTVSS |
| | | | SEQ ID NO: 26406 | SEQ ID NO: 30412 |
| iPS:435607 | 21-225_161G4 | NA | GACATCCAGATGACCCAGTCTCCATCCTCCCTGTCTGCATCTGTAGGAGACAGAGTCACCATCACTTGCCAGGCGAGTCAGGACATTTACAATTATTTAAATTGGTATCAGCAGAAACCAGGGAAAGCCCCTAAGCTCCTGATCTACGATGCATCCAATTTGGAAACAGGGGTCCCATCAAGGTTCAGTGGAAGTGGATCTGGGACAGATTTTACTTTCGCCATCAGCAGCCTGCAGCCTGAAGATATTGCAACATATTACTGTCAACAGTATGATATTCTCCCGATCACCTTCGGCCAAGGGACACGACTGGAGATTAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATCATATGGTGGAAGTAATAAATACCATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTACTGTGTGAGACGGAGCAGCTCGTCGGGGGGCTACGGTATGGACGTCTGGGGCCAAGGGACCACGGTCACCGTCTCCTCA |
| | | | SEQ ID NO: 26407 | SEQ ID NO: 30413 |
| | | AA | DIQMTQSPSSLSASVGDRVTITCQASQDIYNYLNWYQQKPGKAPKLLIYDASNLETGVPSRFSGSGSGTDFTFAISSLQPEDIATYYCQQYDILPITFGQGTRLEIK | QVQLVESGGGVVQPGRSLRLSCAASGFTFSSYGMHWVRQAPGKGLEWVAVISYGGSNKYHADSVKGRFTISRDNSKNTLYLQMNSLRAEDTAVYYCVRRSSSSGGYGMDVWGQGTTVTVSS |
| | | | SEQ ID NO: 26408 | SEQ ID NO: 30414 |

FIGURE 50

| | | | | |
|---|---|---|---|---|
| iPS:435609 | 21-225_161F7 | NA | GACATCCAGATGACCCAGTCTCCATCCTCCCTGTCTGCATCTGTAGGCGACAGAGTCACCATCACTTGCCGGGCAAGTCAGGGCATTAGAAATGATTTGGGCTGGTATCAGCAGAAACCAGGAGAAGCCCCTAAGCGCCTGATCTATGCTGCATCCACTTTGCAAAGTGGGGTCCCATCGCGGTTCAGCGGCAGTGGATCTGGGGCAGAATTCACTGTCACAATCGGCAGCGTGCAGCGTGAAGATTTTGCAACTTATTACGGTCTACTATATATTCGTTACCCATTCACTTTTGGCCGTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTTTGGCTTGCACTGGGTCCGCCAGGCTCCAGGCCAGGGACTGGAGTGGGTGGCAGTTATATGGTTTGATGGAAGTAATAAATACTATGCAGACTCCGTGAAGGGCCGATTCACCATTTTCAGAGACAATTCCAAGAACACGCTGTATCTGCAAATGAACAGCCTGAGAGCCGAGGACACGGCTGTGTATTACTGTGCGAGAGAGATTGGCTGGCTCTCTGACTACTGGGGCCAGGGAACCCTGGTCACCGTCTCCTCA |
| | | | SEQ ID NO: 26409 | SEQ ID NO: 30415 |
| | | AA | DIQMTQSPSSLSASVGDRVTITCRASQGIRNDLGWYQQKPGEAPKRLIYAASTLQSGVPSRFSGSGSGAEFTVTIGSVQREDFATYYGLLYIRYPFTFGRGTKVDIK | QVQLVESGGGVVQPGRSLRLSCAASGFTFSDFGLHWVRQAPGQGLEWVAVIWFDGSNKYYADSVKGRFTIFRDNSKNTLYLQMNSLRAEDTAVYYCAREIGWLSDYWGQGTLVTVSS |
| | | | SEQ ID NO: 26410 | SEQ ID NO: 30416 |
| iPS:435611 | 21-225_161F10 | NA | GACATCCAGATGACCCAGTCTCCATCCTCCCTGTCTGCATCTGTAGGAGACAGAGTCACCATCACTTGCCAGGCGAGTCAGGACATTTACAACCATTTAAGTTGGTATCAGCAGAAACCAGGGAAAGCCCCTAAGCTCCTGATCTACGATGCATCCAATTGGGAAACAGGGGTCCCATCCAGGTTCAGTGGAGGTGGATCTGGGACAGATTTTACTTTCACCATCAGCAGCCTGCAGCCTGAAGATTTTGCAACATATTACTGTCAACAGTATGAAAATCTCCCGCTCACCTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAATGGGTGGCAATTATATCATATTCTGGAAGAAATGATTTCTATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTACTGTGCGAGACGTATAGCAGCAGCTGGTCACTACGGTATGGACGTCTGGGGCCAAGGGACCACGGTCACCGTCTCCTCA |
| | | | SEQ ID NO: 26411 | SEQ ID NO: 30417 |

FIGURE 50

| | | | | |
|---|---|---|---|---|
| | | AA | DIQMTQSPSSLSASVGDRVTITCQASQDIYNHLSWYQQKPGKAPKLLIYDASNWETGVPSRFSGGGSGTDFTFTISSLQPEDFATYYCQQYENLPLTFGGGTKVEIK | QVQLVESGGGVVQPGRSLRLSCAASGFTFSSYGMHWVRQAPGKGLEWVAIISYSGRNDFYADSVKGRFTISRDNSKNTLYLQMNSLRAEDTAVYYCARRIAAAGHYGMDVWGQGTTVTVSS |
| | | | SEQ ID NO: 26412 | SEQ ID NO: 30418 |
| **iPS:435613** | **21-225_161D11** | NA | GACATCCAGATGACCCAGTCTCCATCCTCCCTGTCTGCATCTGTAGGCGACAGAGTCACCATCACTTGCCGGGCAAGTCAGGGCATTAGAAATGATTTGGGCTGGTATCAGCAGAAACCAGGAGAAGCCCCTAAGCGCCTGATCTATGCTGCATCCACTTTGCAAAGTGGGGTCCCATCGCGGTTCAGCGGCAGTGGATCTGGGGCAGAATTCACTCTCACAATCGGCAGCGTGCAGCGTGAAGATTTTGCAACTTATTACGGTCTACAATATAATCGTTACCCATTCACTTTTGGCCGTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTTTGGCTTGCACTGGGTCCGCCAGGCTCCAGGCCAGGGACTGGAGTGGGTGGCAGTTATATGGTTTGATGGAAGTAATAAATACTATGCAGACTCCGTGAAGGGCCGATTCACCATTTTCAGAGACAATTCCAAGAACACGCTGTATCTGCAAATGAACAGCCTGAGAGCCGAGGACACGGCTGTGTATTACTGTGCGAGAGAGATTGGCTGGCTCTCTGACTACTGGGGCCAGGGAACCCTGGTCACCGTCTCCTCA |
| | | | SEQ ID NO: 26413 | SEQ ID NO: 30419 |
| | | AA | DIQMTQSPSSLSASVGDRVTITCRASQGIRNDLGWYQQKPGEAPKRLIYAASTLQSGVPSRFSGSGSGAEFTLTIGSVQREDFATYYGLQYNRYPFTFGRGTKVDIK | QVQLVESGGGVVQPGRSLRLSCAASGFTFSDFGLHWVRQAPGQGLEWVAVIWFDGSNKYYADSVKGRFTIFRDNSKNTLYLQMNSLRAEDTAVYYCAREIGWLSDYWGQGTLVTVSS |
| | | | SEQ ID NO: 26414 | SEQ ID NO: 30420 |

FIGURE 50

| | | | | |
|---|---|---|---|---|
| **iPS:435615** | **21-225_161G12** | NA | GACATCCAGATGACCCAGTCTCCATCCTCCCGGTCTGCATCTGTAGGAGACAGAGTCACCATCACTTGCCGGGCAAGTCAGGACATTAGAAAGGATTTAGGGTGGTATCAGCAGAAACCAGGGAAAGCCCCTAACCGCCTGATCTATGCTGCATCCAGTTTGCAAAGTGGGGTCCCATCAAGGTTCAGCGGCAGTGGATCTGGGACAGAATTCACTCTCACATTCAGCAGCCTGCAGCCTGAAGATTTTGCAACTTATTACTGTCTACAGCATCATAGTTATCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTTAGTGACTATGTCATGCAGTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACCGTATAATAGTGGCTGGTACGACTACGGTATGGACGTCTGGGGCCAAGGGACCACGGTCACCGTCTCCTCA |
| | | | SEQ ID NO: 26415 | SEQ ID NO: 30421 |
| | | AA | DIQMTQSPSSRSASVGDRVTITCRASQDIRKDLGWYQQKPGKAPNRLIYAASSLQSGVPSRFSGSGSGTEFTLTFSSLQPEDFATYYCLQHHSYPRTFGQGTKVEIK | QVQLVESGGGVVQPGRSLRLSCAASGFTFSDYVMQWVRQAPGKGLEWVAVIWYDGSNKYYADSVKGRFTISRDNSKNTLYLQMNSLRAEDTAVYYCAREPYNSGWYDYGMDVWGQGTTVTVSS |
| | | | SEQ ID NO: 26416 | SEQ ID NO: 30422 |
| **iPS:435617** | **21-225_162F2** | NA | GACATTCAGATGACCCAGTCTCCATCCTCCCTGTGTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCTTGATCAATGCTGCATCCAGTTTGCAAAGTGGGGTCCCATCAAGGTTCAGCGGCAGTGGATCTGGGACAGAATTCACTCTCACAATCAGCAGCGTGCAGCCTGAAGATTTTGCAACTTATTACTGTATACAGGATAATAGTCACCCATTCACTTTCGGCCCTGGGACCAAAGTGGAAATCAAA | GAGGTGCAACTGGTGGAGTCTGGGGGAGGCCTGGTCAAGCCTGGGGGGTCCCTGAGATTCTCCTGTGCAGCCTCTGGATTCACCTTCAGTAGTTATAGCATGAACTGGGTCCGCCAGGCTCCAGGGAAGGGGCTGGAGTGGGTCTCATCCATTAGTGGTAGTAGTACGTACATATACTACGCAGACTCAGTGAAGGGCCGATTCACCATCTCCAGAGACAACGCCAAGAACTCACTGTATCTGCAAATGAACAGCCTGAGAGCCGAAGACACGGCTGTGTATTACTGCGCGAGAGATCGGGGCAGCAGCTGGGGCCAGGGAACCCTGGTCACCGTCTCCTCA |
| | | | SEQ ID NO: 26417 | SEQ ID NO: 30423 |

FIGURE 50

| | | | | |
|---|---|---|---|---|
| | | AA | DIQMTQSPSSLCASVGDRVTITCRASQGIRNDLGWYQQKPGKAPKRLINAASSLQSGVPSRFSGSGSGTEFTLTISSVQPEDFATYYCIQDNSHPFTFGPGTKVEIK | EVQLVESGGGLVKPGGSLRFSCAASGFTFSSYSMNWVRQAPGKGLEWVSSISGSSTYIYYADSVKGRFTISRDNAKNSLYLQMNSLRAEDTAVYYCARDRGSSWGQGTLVTVSS |
| | | | SEQ ID NO: 26418 | SEQ ID NO: 30424 |
| **iPS:435621** | **21-225_162H3** | NA | GACATTCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCTTGATCAATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGGATAATAGTCACCCATTCACTTTCGGCCCTGGGACCAAAGTGGAAATCAAA | GAGGTGCAACTGGTGGAGTCTGGGGGAGGCCTGGTCAAGCCTGGGGGGTCCTTGAGATTCTCCTGTGCAGCCTCTGGATTCACCTTCAGTAGTTATAGCATGAACCGGGTCCGCCAGGCTCCAGGGAAGGGGCTGGAGTGGGTCTCATCCATTAGTGGTAGTAGTACGTACATATACTACGCAGACTCAGTGAAGGGCCGATTCACCATCTCCAGAGACAACGCCAAGAACTCACTGTATCTGCAAATGAACAGCCTGAGAGCCGAAGACACGGCTGTGTATTACTGCGCGAGAGATCGGGGCAGCAGCTGGGGCCAGGGAACCCTGGTCACCGTCTCCTCA |
| | | | SEQ ID NO: 26419 | SEQ ID NO: 30425 |
| | | AA | DIQMTQSPSSLSASVGDRVTITCRASQGIRNDLGWYQQKPGKAPKRLINAASSLQSGVPSRFSGSGSGTEFTLTISSLQPEDFATYYCLQDNSHPFTFGPGTKVEIK | EVQLVESGGGLVKPGGSLRFSCAASGFTFSSYSMNRVRQAPGKGLEWVSSISGSSTYIYYADSVKGRFTISRDNAKNSLYLQMNSLRAEDTAVYYCARDRGSSWGQGTLVTVSS |
| | | | SEQ ID NO: 26420 | SEQ ID NO: 30426 |

FIGURE 50

| | | | | |
|---|---|---|---|---|
| **iPS:435623** | **21-225_162D5** | NA | GACATTGTGATGACCCAGTCTCCAGACTTCCG<br>TAATGTGTCTATGGGCGAGAGGGCCATCATCA<br>ACTTCAAGTCCAACCATAGTGTTTTATACAGG<br>TCCAACAATAATCAATACTTAGCTTGGTACCA<br>GCGGAAACCAGGACAGCCTCCTAAGTTGCTCA<br>TTTACCGGACATCTATCCGGAAATCCGGGGTC<br>CCTGACCGATTCAGTGGCAGCGGGTGTGGGAC<br>AGATTTCACTCTCACCATCGACAGCCTGCAGG<br>CTGAAGATGTGGCAGTTTATTACTGTCAGCAA<br>TATTATAGTACTCCTCCCACTTTCGGCGGAGG<br>GACCAAGGTGGAGATCAAA | CAGGTGCAGCTGGTGCAGTCTGGGTCTGAGGTGA<br>AGAAGCCTGGGGCCTCAGTGAAGGTCTCCTGCA<br>AGGCTTCTGGATACACCTTCACCAATTATGATAT<br>CAACTGGGTGCGACAGGCCACTGGACAAGGGCT<br>TGAGTGGATGGGATGGATGCACCCTAACAGTGGT<br>AACACAGGCTATGCACAGAAGTTCCAGGGCAGA<br>GTCACCATGACCAGGAACACCTCCATAGACACA<br>GCCTACATGGAACTGAGCAGCCTGAGTTCTGAGG<br>ACACGGCCGTGTATTTCTGTGCGTTTAGCAGTGG<br>CTGGTACTTCTTTGACTACTGGGGCCAGGGAACC<br>CTGGTCACCGTCTCCTCA |
| | | | SEQ ID NO: 26421 | SEQ ID NO: 30427 |
| | | AA | DIVMTQSPDFRNVSMGERAIINFKSNHSVLYRSN<br>NNQYLAWYQRKPGQPPKLLIYRTSIRKSGVPDR<br>FSGSGCGTDFTLTIDSLQAEDVAVYYCQQYYSTP<br>PTFGGGTKVEIK | QVQLVQSGSEVKKPGASVKVSCKASGYTFTNYDIN<br>WVRQATGQGLEWMGWMHPNSGNTGYAQKFQGR<br>VTMTRNTSIDTAYMELSSLSSEDTAVYFCAFSSGW<br>YFFDYWGQGTLVTVSS |
| | | | SEQ ID NO: 26422 | SEQ ID NO: 30428 |
| **iPS:435627** | **21-225_162F6** | NA | GACATCGTGATGACCCAGTCTCCAGACTCCCT<br>GGCTGTGTCTCTGGGCGAGAGGGCCACCATCA<br>ACTGCAAGTCCAGCCAGAATGTTTTACACAGC<br>TCCAACAATAACAACTACTTAACTTGGTACCA<br>GCAGAGACCAGGACAGCCTCCTAAGTTGCTCA<br>TTTACTGGGCATCTACCCGGGAATCCGGGGTC<br>CCTGACCGATTCAGTGGCAGCGGGTCTGGGAC<br>AGAATTCACTCTCACCATCAGCAGCCTGCAGG<br>CTGAAGATGTGGCAGTTTATTACTGTCAGCAA<br>TATTATAGTACTCCTCCGACGTTCGGCCAAGG<br>GACCAAGGTGGAAATCAAA | CAGGTGCAGCTGGTGCAGTCTGGGGCTGAGGTG<br>AGGAAGCCTGGGGCCTCAGTGAAGGTCTCCTGC<br>AAGGCTTCTGGATACACCTTCACCAATTATGATA<br>TCAACTGGGTGCGACAGGCCACTGGACAAGGGC<br>TTGAGTGGATGGGATGGATGCACCCTAACAGTGG<br>TAACACAGGCTATGCACAGAAGTTCCAGGGCAG<br>ATTCACCATGACCAGGAACACCTCCATAAGCACA<br>GCCTACATGGAGCTGAGCAGCCTGAGATCTGAG<br>GACACGGCCGTGTATTACTGTGCCTATAGCAGTG<br>GCTGGTACCGCTTTGACTACTGGGGCCAGGGAAC<br>CCTGGTCACCGTCTCCTCA |
| | | | SEQ ID NO: 26423 | SEQ ID NO: 30429 |

FIGURE 50

| | | | | |
|---|---|---|---|---|
| | | AA | DIVMTQSPDSLAVSLGERATINCKSSQNVLHSSNNNNYLTWYQQRPGQPPKLLIYWASTRESGVPDRFSGSGSGTEFTLTISSLQAEDVAVYYCQQYYSTPPTFGQGTKVEIK | QVQLVQSGAEVRKPGASVKVSCKASGYTFTNYDINWVRQATGQGLEWMGWMHPNSGNTGYAQKFQGRFTMTRNTSISTAYMELSSLRSEDTAVYYCAYSSGWYRFDYWGQGTLVTVSS |
| | | | SEQ ID NO: 26424 | SEQ ID NO: 30430 |
| **iPS:435629** | **21-225_162H6** | NA | GATATTGTGATGACCCAGACTCCACTCTCTCTGTCCGTCACCCCTGGACAGCCGGCCTCCATCTCCTGCAAGTCTACTCAGAGCCTCCTGCATGGTGATGGAAAGACCTATTTGTATTGGTACCTGCAGAAGCCAGGCCAGCCTCCACAACTCCTGATCTATGAAGTTTCCAAGCGGTTCTCTGGAGTGCCAGAAAGGTTCAGTGGCAGCGGGTCAGGGACAGATTTCACACTGAAAATCAGCCGGGTGGAGGCTGAGGATGTTGGGGTTTATTACTGCAAGCAAAGTATACAGCTTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAATAACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAAAAGGTATAGCAGCAGTTGGAGACTACTACTACGGTATGGACGTCTGGGGCCAAGGGACCACGGTCACCGTCTCCTCA |
| | | | SEQ ID NO: 26425 | SEQ ID NO: 30431 |
| | | AA | DIVMTQTPLSLSVTPGQPASISCKSTQSLLHGDGKTYLYWYLQKPGQPPQLLIYEVSKRFSGVPERFSGSGSGTDFTLKISRVEAEDVGVYYCKQSIQLPWTFGQGTKVEIK | QVQLVESGGGVVQPGRSLRLSCAASGFTFNNYGMHWVRQAPGKGLEWVAVIWYDGSNKYYADSVKGRFTISRDNSKNTLYLQMNSLRAEDTAVYYCARKGIAAVGDYYYGMDVWGQGTTVTVSS |
| | | | SEQ ID NO: 26426 | SEQ ID NO: 30432 |

FIGURE 50

| | | | | |
|---|---|---|---|---|
| **iPS:435635** | **21-225_163F1** | NA | GACATCCAGATGACCCAGTCTCCATCCTCACTGTCTGCATCTGTAGGAGACAGAGTCACCATCACTTGTCGGGCGAGTCAGGACATTAGCAATTATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAGCTTATTACTGCCAACAGTATAATAGTTACCCATTCACTTTCGGCCCTGGGACCCAAGTGGATGCCCAA | GAGGTGCAGCTGGTGGACTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTGGTAGTTACATATACTACGCAGACTCAGTGAAGGGCCGATTCACCACCTCCAGAGACAACGCCAAGAACTCACTGTATCTGCAAATGAACAGCCTGAGCGCCGACGACACGGCTGTTTATTACTGTACGCTCTATAGCAGCTCGCACTATTGGGGCCAGGGAACCCTGGTCACCGTCTCCTCA |
| | | | SEQ ID NO: 26427 | SEQ ID NO: 30433 |
| | | AA | DIQMTQSPSSLSASVGDRVTITCRASQDISNYLAWFQQKPGKAPKSLIYAASSLQSGVPSKFSGSGSGTDFTLTISSLQPEDFAAYYCQQYNSYPFTFGPGTQVDAQ | EVQLVDSGGGLVKPGGSLRLSCAASGFTFSSYSMNWVRQAPGKGLEWVSSISGSGSYIYYADSVKGRFTTSRDNAKNSLYLQMNSLSADDTAVYYCTLYSSSHYWGQGTLVTVSS |
| | | | SEQ ID NO: 26428 | SEQ ID NO: 30434 |
| **iPS:435637** | **21-225_163E2** | NA | GACATCCAGATGACCCAGTCTCCATCCTCCCTGTCTGCATCTGTAGGAGACAGAGTCACCATCACTTGCCGGGCAAGTCAGGACATTAGAAATGATTTAGGCTGGTATCAGCAGAAACCAGGGAAAGCCCCTACGCGCCTGATCTATCCTGCATCCAGTTTGCAAAGTGGGGTCCCATCAAGGTTCAGCGGCAGTGGATCTGGGACAGAATTCACTCTCTCAATCAGTAGCCTGCAGCCTGAAGATTTTGCAACTTATTACTGTCTACAGCATAATAGTCACCCATTCACTTTCGGCCCTGGGACCAAAGTGGATATCAAA | GAGGTGCAACTGGTGGAGTCTGGGGGAGGCCTGGTCCAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCCTCCACTAGTGGGAGTTCTACTTACATATACTACGCAGACTCAGTGAAGGGCCGATTCACCATCTCCAGAGACAACGCCAAGAACTTAGTGTATCTGCAAATGAACAGCCTGAGACCCGAGGACACGGCTGTGTATTACTGTGCGAGAGATCGAGGCAGCCTCTGGGGCCAGGGAACCCTGGTCACCGTCTCCTCA |
| | | | SEQ ID NO: 26429 | SEQ ID NO: 30435 |

FIGURE 50

| | | | | |
|---|---|---|---|---|
| | | AA | DIQMTQSPSSLSASVGDRVTITCRASQDIRNDLGWYQQKPGKAPTRLIYPASSLQSGVPSRFSGSGSGTEFTLSISSLQPEDFATYYCLQHNSHPFTFGPGTKVDIK | EVQLVESGGGLVQPGGSLRLSCAASGFTFSSYSMNWVRQAPGKGLEWVSSTSGSSTYIYYADSVKGRFTISRDNAKNLVYLQMNSLRPEDTAVYYCARDRGSLWGQGTLVTVSS |
| | | | SEQ ID NO: 26430 | SEQ ID NO: 30436 |
| iPS:435639 | 21-225_163G6 | NA | GACATCCAGATGACCCAGTCTCCATCCTCACTGTCTGCATCTGTAGGAGACAGAGTCACCATCACTTGTCGGGCGAGTCAGGACATTAGCAATTATTTAGTCTGGTTTCAGCAGAAACCAGGGAAAGCCCCTAAGTCCCTGATCTATGCTGCATCCAGTTTGCTAAGTGGGGTCCCTTCAAAGTTCAGCGGCAGTGGATCTGGGACAGATTTCACTCTCACCATCAGCAGCCTGCAGCCTGAAGATTTTGCAACTTATTACTGCCAACAGTATCATAGTTACCCGCTCACTTTCGGCGGAGGGACCAAGGTGGCGATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGTTATAGCATGAGCTGGGTCCGCCAGGCTCCAGGGAAGGGGCTGGAGTGGGTCTCATCCATTAGTGGTAGTAGTGCTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGATTGAGCGGTATGGACGTCTGGGGCCAAGGGACCACGGTCACCGTCTCCTCA |
| | | | SEQ ID NO: 26431 | SEQ ID NO: 30437 |
| | | AA | DIQMTQSPSSLSASVGDRVTITCRASQDISNYLVWFQQKPGKAPKSLIYAASSLLSGVPSKFSGSGSGTDFTLTISSLQPEDFATYYCQQYHSYPLTFGGGTKVAIK | EVQLVESGGGLVKPGGSLRLSCAASGFTFSSYSMSWVRQAPGKGLEWVSSISGSSAYIYYADSVKGRFTISRDNAKNSLYLQMNSLRAEDTAVYYCARLSGMDVWGQGTTVTVSS |
| | | | SEQ ID NO: 26432 | SEQ ID NO: 30438 |

FIGURE 50

| | | | | |
|---|---|---|---|---|
| **iPS:435641** | **21-225_163F9** | NA | GACATCCAGATGACCCAGTCTCCATCCTCCCTGTCTGCATCTGTAGGAGACAGAGTCACCATCACTTGCCGGGCAAGTCAGGACATTAGAGATGATTTAGGCTGGTATCAGCAGAAACCAGGGAAAGCCCCTAAGCGCCTGATCTATCCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAGCTTATTACTGTCTACAGGATAATAGTTACCCATTCACTTTCGGCCCTGGGACCAAAGTGGATATCAAA | GAGGTGCAGCTGGTGGAGTCTGGGGGAGGCCTGGTCCAGCCGGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTACTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGATCGAGGCAGCCTCTGGGGCCAGGGAACCCTGGTCACCGTCTCCTCA |
| | | | SEQ ID NO: 26433 | SEQ ID NO: 30439 |
| | | AA | DIQMTQSPSSLSASVGDRVTITCRASQDIRDDLGWYQQKPGKAPKRLIYPASSLQSGVPSRFSGSGSGTEFTLTISSLQPEDFAAYYCLQDNSYPFTFGPGTKVDIK | EVQLVESGGGLVQPGGSLRLSCAASGFTFSSYSMNWVRQAPGKGLEWVSSISGSSTYIYYADSVKGRFTISRDNAKNSLYLQMNSLRAEDTAVYYCARDRGSLWGQGTLVTVSS |
| | | | SEQ ID NO: 26434 | SEQ ID NO: 30440 |
| **iPS:435643** | **21-225_163G10** | NA | GACATCCAGATGACCCAGTCTCCATCCTCCCTGTCTGCATCTGTAGGAGACAGAGTCACCATCACTTGCCGGGCAAGTCAGGACATTAGAAATAATTTAGGCTGGTATCAGCAGAAACCAGGGAAAGCCCCTAAGCGCCTGATCTATCCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGGATTATAGTTACCCATTCACTTTCGGCCCTGGGACCAAAGTGGATATCAAA | GAGGTGCAGTTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTACTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGCCCGTATGGACGTCTGGGGCCAAGGGACCACGGTCACCGTCTCCTCA |
| | | | SEQ ID NO: 26435 | SEQ ID NO: 30441 |

FIGURE 50

| | | | | |
|---|---|---|---|---|
| | | AA | DIQMTQSPSSLSASVGDRVTITCRASQDIRNNLG WYQQKPGKAPKRLIYPASSLQSGVPSRFSGSGSG TEFTLTISSLQPEDFATYYCLQDYSYPFTFGPGTK VDIK | EVQLVESGGGLVKPGGSLRLSCAASGFTFSSYSMN WVRQAPGKGLEWVSSISGSSTYIYYADSVKGRFTIS RDNAKNSLYLQMNSLRAEDTAVYYCARARMDVW GQGTTVTVSS |
| | | | SEQ ID NO: 26436 | SEQ ID NO: 30442 |
| iPS:435649 | 21-225_165H2 | NA | GACATCGTGATGACCCAGTCTCCAGACTCCCT GACTGTGTCTCCGGGCGAGAGGGCCACCATCA ACTGCAAGTCCAGCCAGAGTGTTTTACACAGC TCCAACAATAAGAACTACTTAACTTGGTACCA GCAGAAACCAGGACAGCCTCCTAAACTGCTCA TTTACTGGGCATCTACCCGAGAATCCGGGGTC CCTGTCCGATTCAGTGGCAGCGGGTCGGGGAC AGATTTCACTGTCCCCATCAGCAGCATGCAGG ATGATGATGTGGCAGTTTATTACCGTCAGCAA TCTTATAGTATTCCTCCCACTTTCGGCCCCGGG ACCAACGTGGATATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCCATTATGATA TCAACTGGGTGCGACAGGCCACTGGACAAGGGC TTGAGTGGGTGGGGTGGATGCACCCTAACAGTCA TAAGACAGGCTATGCACAGAAGTTCCAGGGCAG AGTCACCATGACCAGGAACACCTCCAACAGCAC AGCCTACATGGACCTCAGCAGCCTGAGATCTGAG GACACGGCCGTGTATTACTGTGCTTATAGCAGTG GCTGGTACATGTTTGACTACTGGGGCCAGGGAAC CCTGGTCACCGTCTCCTCA |
| | | | SEQ ID NO: 26437 | SEQ ID NO: 30443 |
| | | AA | DIVMTQSPDSLTVSPGERATINCKSSQSVLHSSN NKNYLTWYQQKPGQPPKLLIYWASTRESGVPVR FSGSGSGTDFTVPISSMQDDDVAVYYRQQSYSIP PTFGPGTNVDIK | QVQLVQSGAEVKKPGASVKVSCKASGYTFTHYDIN WVRQATGQGLEWVGWMHPNSHKTGYAQKFQGR VTMTRNTSNSTAYMDLSSLRSEDTAVYYCAYSSG WYMFDYWGQGTLVTVSS |
| | | | SEQ ID NO: 26438 | SEQ ID NO: 30444 |

FIGURE 50

| | | | | |
|---|---|---|---|---|
| **iPS:435653** | **21-225_166H12** | NA | GACATCCAGATGACCCAGTCTCCATCCTCACTGTCTACATCTGTAGGAGACAGAGTCACCATCACTTGTCGGGCGAGTCAGGACATTAGCCATTATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATAATAGTTTCCCGCTCACTTTCGGCGGAGGGACCAAGGTGGAGATCACA | GAGGTGCAGCTGGTGGAGTCTGGGGGAGCCCTGGTCAAGCCTGGGGGGTCCCTGAGACTCTCCTGTGCAGCCTCTGGATTCACCTTCAGTAGCTATAGCATGAGCTGGGTCCGCCAGGCTCCAGGGAAGGGGCTGGGGTGGGTCTCATCCATTAGTGGGAGTAGTAGTTACAGTTACTACGCAGACTCAGTGAAGGGCCGATTCACCATCTCCAGAGACAATGCCAAGAACTCACTGTATCTGCAAATGAACAGCCTGAGAGCCGAGGACACGGCTGTGTATTACTGTGCGAGACTAACTGGCTTTGACTACTGGGGCCAGGGAACCCTGGTCACCGTCTCCTCA |
| | | | SEQ ID NO: 26439 | SEQ ID NO: 30445 |
| | | AA | DIQMTQSPSSLSTSVGDRVTITCRASQDISHYLAWFQQKPGKAPKSLIYAASSLQSGVPSKFSGSGSGTDFTLTISSLQPEDFATYYCQQYNSFPLTFGGGTKVEIT | EVQLVESGGALVKPGGSLRLSCAASGFTFSSYSMSWVRQAPGKGLGWVSSISGSSSYSYYADSVKGRFTISRDNAKNSLYLQMNSLRAEDTAVYYCARLTGFDYWGQGTLVTVSS |
| | | | SEQ ID NO: 26440 | SEQ ID NO: 30446 |
| **iPS:435655** | **21-225_167E2** | NA | GATATTGTGATGACCCAGACTCCACTCTCTCTGTCCGTCACCCCTGGACAGCCGGCCTCCATCTCCTGTAAGTCTAGTCAGAGCCTCCTGCACGGTGATGGGAAGACCTATTTGTATTGGTACCTGCAGAAGCCAGGCCAGCCTCCACACCTCCTGATCTATGAAGTTTCCAAACGGTTCTCTGGAGTGCCAGATAGGTTCAGTGGCAGCGGGTCAGGGACAGATTTCACACTGAAGATCAGCCGGGTGGAGGCTGAGGATGTTGGGCTTTATCACTGCATGCAAAGCATACAGCTTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTTAGTAGCTTTGGCATGCACTGGGTCCGCCAGGCTCCAGGCAAGGGCCTGGAGTGGGTGGCAGTCATATGGTATGATGGAACTTATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGTTGGTATTGAAGTGGCTGGTGACTACTACTACGGTATGGAAGTCTGGGGCCAAGGGACCACGGTCACCGTCTCCTCA |
| | | | SEQ ID NO: 26441 | SEQ ID NO: 30447 |

FIGURE 50

| | | | | |
|---|---|---|---|---|
| | | AA | DIVMTQTPLSLSVTPGQPASISCKSSQSLLHGDGKTYLYWYLQKPGQPPHLLIYEVSKRFSGVPDRFSGSGSGTDFTLKISRVEAEDVGLYHCMQSIQLPWTFGQGTKVEIK | QVQLVESGGGVVQPGRSLRLSCAASGFTFSSFGMHWVRQAPGKGLEWVAVIWYDGTYKYYADSVKGRFTISRDNSKNTLYLQMNSLRAEDTAVYYCARVGIEVAGDYYYGMEVWGQGTTVTVSS |
| | | | SEQ ID NO: 26442 | SEQ ID NO: 30448 |
| **iPS:435657** | **21-225_167H10** | NA | GATATTGTGATGACCCAGACTCCACTCTCTCTGTCCGTCACCCCTGGACAGCCGGCCTCCATCTCCTGCAAGTCTAGTCAGAGCCTCCTGCACGGTGATGGAAAGACCTATTTGTATTGGTACCTGCAGAAGCCAGGCCAGCCTCCACACCTCCTGATCTATGAAGTTTCCAAACGGTTCTCTGGAGTGCCAGATAGGTTCAGTGGCAGCGGGTCAGGGACAGATTTCACACTGAAAATCAGCCGGGTGGAGGCTGAGGATGTTGGGCTTTATCACTGCATGCAAAGCATACAGCTTCCGTGGACGTTCGGCCAAGGGACCAAGGTGGAAATCAAA | CAGGTGCAGTTGGTGGAGTCTGGGGGAGGCGTGGTCCAGCCTGGGAGGTCCCAGAGACTCTCCTGTGCAGCGTCTGGATTCACCTTTAGTAGCTTTGGCATGCACTGGGTCCGCCAGGCTCCAGGCAAGGGCCTGGAGTGGGTGGCAGTCATATGGTATGATGGAAGTTATAAGTACCATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGTTGGTATAGAAGTGGCTGGTGACTACTACTACGGTATGGAAGTCTGGGGCCAAGGGACCACGGTCACCGTCTCCTCA |
| | | | SEQ ID NO: 26443 | SEQ ID NO: 30449 |
| | | AA | DIVMTQTPLSLSVTPGQPASISCKSSQSLLHGDGKTYLYWYLQKPGQPPHLLIYEVSKRFSGVPDRFSGSGSGTDFTLKISRVEAEDVGLYHCMQSIQLPWTFGQGTKVEIK | QVQLVESGGGVVQPGRSQRLSCAASGFTFSSFGMHWVRQAPGKGLEWVAVIWYDGSYKYHADSVKGRFTISRDNSKNTLYLQMNSLRAEDTAVYYCARVGIEVAGDYYYGMEVWGQGTTVTVSS |
| | | | SEQ ID NO: 26444 | SEQ ID NO: 30450 |

FIGURE 50

| | | | | |
|---|---|---|---|---|
| iPS:435659 | 21-225_167D12 | NA | GACATCCAGATGACCCAGTCTCCATCCTCACTGTCTGCATCTGTAGGGGACAGAGTCACCATCACTTGTCGGGCGAGTCAGGGCATTAACAATTATTTAGCCTGGTTTCAGCAGAAACCAGGGAAAGCCCCTGAGTCCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTTCTGCCAACAGTATAATAGTTATCCATTCACTTTCGGCCCTGGGACCAAAGTGGATATCAAA | GAGGTGCAGCTGTTGGAGTCTGGGGGAGGCTTGGTACAGCCGGGGGGGTCCCTGAGACTCTCCTGTGCAGCCTCTGGATTCACCTTTAGTAGCTATGTCATGAGCTGGGTCCGCCAGGCTCCAGGGAAGGGGCTGGAGTGGGTCTCAGCTATGAGTGGTAGTGGTGGTAGAACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTGTATTACTGTGCGAAGTATACCTGGAACGGCTACTGGGGCCAGGGAACCCTGGTCACCGTCTCCTCA |
| | | | SEQ ID NO: 26445 | SEQ ID NO: 30451 |
| | | AA | DIQMTQSPSSLSASVGDRVTITCRASQGINNYLAWFQQKPGKAPESLIYAASSLQSGVPSKFSGSGSGTDFTLTISSLQPEDFATYFCQQYNSYPFTFGPGTKVDIK | EVQLLESGGGLVQPGGSLRLSCAASGFTFSSYVMSWVRQAPGKGLEWVSAMSGSGGRTYYADSVKGRFTISRDNSKNTLYLQMNSLRAEDTAVYYCAKYTWNGYWGQGTLVTVSS |
| | | | SEQ ID NO: 26446 | SEQ ID NO: 30452 |
| iPS:435663 | 21-225_169B1 | NA | GACATCCAGATGACCCAATCTCCATCCTCCCTGTCTGCATCTGTAGGAGACAGAGTCACCATCACTTGCCGGGCTAGTCAGGGCATTAGAAATGATTTAGGCTGGTATCAGCAGAAACCAGGGAAAGCCCCTAAGCGCCTGATCGGTGCTGAATCCAGTTTGCAAAGTGGGGTCCCATCAAGGTTCAGCGGCAGTGGATCTGGGACAGAATTCACTCTCACAATCAGCGGCCTGCAGCCTGAAGATTTTGCAACTTATTACTGTCTACAGCATTATAGTTACCCGCTCACTTTCGGCGGAGGGACCAAAGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAGGGGCCGATTCACCATCTCCAGAGACAATTCCAAGAACACGCTGTATCTGCAAATGAACAGCCTGAGAGCCGAAGACACGGCTGTGTATTACTGTGCGAGAGATCCCTTACGTGGATACAATGACCCGGTTATGGACTACTGGGGCCAGGGAACCCTGGTCACCGTCTCCTCA |
| | | | SEQ ID NO: 26447 | SEQ ID NO: 30453 |

FIGURE 50

| | | | | |
|---|---|---|---|---|
| | | AA | DIQMTQSPSSLSASVGDRVTITCRASQGIRNDLG WYQQKPGKAPKRLIGAESSLQSGVPSRFSGSGSG TEFTLTISGLQPEDFATYYCLQHYSYPLTFGGGT KVEIK | QVQLVESGGGVVQPGRSLRLSCAASGFTFSSYGMH WVRQAPGKGLEWVAVIWYDGSNKYYADSVRGRF TISRDNSKNTLYLQMNSLRAEDTAVYYCARDPLRG YNDPVMDYWGQGTLVTVSS |
| | | | SEQ ID NO: 26448 | SEQ ID NO: 30454 |
| **iPS:435665** | **21-225_169F2** | NA | GACATCGTGATGACCCAGTCTCCAGACTCCCT GGCTGTGTCTCTGGGCGAGAGGGCCACCATCA ACTGCAAGTCCAGCCAGAGTGTTTTATACATC TCCAACAATAAAAACTACTTAGCTTGGTACCA GCAGAAACCAGGACAGCCTCCTAAACTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATCGTGCTCCCACCTTCGGCCAAGGGAC ACGACTGGAGATTAAA | CAGGTGCAGCTGCAGGAGTCGGGCCCAGGACTG GTGAAGCCTTCGGAGACCCTGTCCCTCACCTGCA CTGTCTCTGGTGGCTCCATCAGTAGTTACTACTG GAGTTGGATCCGGCAGCCCGCCGGGAAGGGACT GGAGTGGATTGGGCGTATCGATACCAGTGGGATC ACCAACTACAACCCCTCCCTCAAGAGTCGAGTCA CCATGTCAATAGACACGTCCAAGAGCCAGATCTC CCTGAAGCTGAGCTCTGTGACCGCCGCGGACACG GCCGTGTATTACTGTGCGAGAGAGGGAGGAGTG GGAGCTACCTACTTTGACTACTGGGGCCAGGGAA CCCTGGTCACCGTCTCCTCA |
| | | | SEQ ID NO: 26449 | SEQ ID NO: 30455 |
| | | AA | DIVMTQSPDSLAVSLGERATINCKSSQSVLYISN NKNYLAWYQQKPGQPPKLLIYWASTRESGVPD RFSGSGSGTDFTLTISSLQAEDVAVYYCQQYYR APTFGQGTRLEIK | QVQLQESGPGLVKPSETLSLTCTVSGGSISSYYWSW IRQPAGKGLEWIGRIDTSGITNYNPSLKSRVTMSIDT SKSQISLKLSSVTAADTAVYYCAREGGVGATYFDY WGQGTLVTVSS |
| | | | SEQ ID NO: 26450 | SEQ ID NO: 30456 |

FIGURE 50

| | | | | |
|---|---|---|---|---|
| **iPS:435667** | **21-225_169E3** | NA | GACATTGTGATGACTCAGTCTCCACTCTCCCTG CCCGTCACCCCTGGAGAGCCGGCCTCCATCTC CTGCAGGTCTAGTCAGAGCCTCCTGCATAATA ATGGATACAAGTATTTGGATTGGTACCTGCAG AAGCCAGGGCAGTCTCCACAGCTCCTGATCTA TTTGGGTTCTAATCGGGCCTCCGGGGTCCCTG ACAGGTTCAGTGCCAGTGGATCAGGCACAGAT TTTACACTGAAAATCAGCAGAGTGGAGGCTGA GGATGTTGGGGTTTATTACTGCATGCAAGTTC TACAAACTCCGTGGACGTTCGGCCAAGGGACC AAGGTGGAAATCAAA | GAGGTGCAACTGGTGGAGTCTGGGGGAGGCTTG GTACAGCCGGGGGGGTCCCTGAGACTCTCCTGTG CAGCCTCAGGATTCACCTTCAGTGGCCATAGCAT GAACTGGGTCCGCCAGGCTCCAGGGAAGGGGCT GGAGTGGGTTGCATACATTAGCCTTAGTGGTAGT ACCATAAAGTACGCAGACTCTGTGAAGGGCCGA TTCACCATCTCCAGAGACAATGCCAGGGACTCAC TGTATCTGCAAATGAACAGCCTGAGAGACGAGG ACACGGCTGTGTATTACTGTGCGAGAAGGGGGAT TACTGTGGTTCGGAATGAGGACGGTTTGGACGTC TGGGGCCAAGGGACCACGGTCACCGTCTCCTCA |
| | | | SEQ ID NO: 26451 | SEQ ID NO: 30457 |
| | | AA | DIVMTQSPLSLPVTPGEPASISCRSSQSLLHNNGY KYLDWYLQKPGQSPQLLIYLGSNRASGVPDRFS ASGSGTDFTLKISRVEAEDVGVYYCMQVLQTPW TFGQGTKVEIK | EVQLVESGGGLVQPGGSLRLSCAASGFTFSGHSMN WVRQAPGKGLEWVAYISLSGSTIKYADSVKGRFTI SRDNARDSLYLQMNSLRDEDTAVYYCARRGITVV RNEDGLDVWGQGTTVTVSS |
| | | | SEQ ID NO: 26452 | SEQ ID NO: 30458 |
| **iPS:435669** | **21-225_169F9** | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTTTGCTGCATCCAGTT TGCAAAGTGGGGTCCCATCAAGGTTCAGCGGC AGTGGATCTGGGACAGAATTCACTCTCACAAT CAGCAGCCTGCAGCCTGAAGATTTTGCAACTT ATTACTGTCTACAGCATTATAGTTACCCGCTCA CTTTCGGCGGAGGGACCAAGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTACCTATGGCAT GCACTGGGTCCGCCAGGCTCCAGGCAAGGGGCT GGAGTGGGTGTCAATTATATGGTATGATGGAACT AATAAATACTATGCAGACTCCGTGAAGGGCCGA TTCACCATCTCCAGAGACAATTCCAAGAACACGC TGTATCTGCAACTGAACAGCCTGAGAGCCGAGG ACACGGCTGTGTATTACTGTGCGCGAGATCCCTT ACGTGGATACAATGACCCGGTTATGGACTACTGG GGCCAGGGAACCCTGGTCACCGTCTCCTCA |
| | | | SEQ ID NO: 26453 | SEQ ID NO: 30459 |

FIGURE 50

| | | | | |
|---|---|---|---|---|
| | | AA | DIQMTQSPSSLSASVGDRVTITCRASQGIRNDLGWYQQKPGKAPKRLIFAASSLQSGVPSRFSGSGSGTEFTLTISSLQPEDFATYYCLQHYSYPLTFGGGTKVEIK | QVQLVESGGGVVQPGRSLRLSCAASGFTFSTYGMHWVRQAPGKGLEWVSIIWYDGTNKYYADSVKGRFTISRDNSKNTLYLQLNSLRAEDTAVYYCARDPLRGYNDPVMDYWGQGTLVTVSS |
| | | | SEQ ID NO: 26454 | SEQ ID NO: 30460 |
| iPS:435671 | 21-225_169H5 | NA | GACATCGTGATGACCCAGTCTCCAGACTCCCTGGCTGTGTCTCTGGGCGAGAGGGCCACCATCAACTGCAAGTCCAGCCAGAGTGTTTTATACATCTCCAACAATAAAAACTACTTAGCTTGGTACCAGCAGAAACCAGGACAGCCTCCTAAACTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATCGTGCTCCCACCTTCGGCCAAGGGACACGACTGGAGATTAAA | CAGGTGCAGCTGCAGGAGTCGGGCCCAGGACTGGTGAAGCCTTCGGAGACCCTGTCCCTCACCTGCACTGTCTCTGGTGGCTCCATCAGTAGTTACTACTGGAGTTGGATCCGGCAGCCCGCCGGGAAGGGACTGGAGTGGATTGGGCGTATCGATACCAGTGGGATCACCAACTACAACCCCTCCCTCAAGAGTCGAGTCACCATGTCAGTAGACACGTCCAAGAGCCAGATCTCCCTGAAGCTGAGCTCTGTGACCGCCGCGGACACGGCCGTGTATTACTGTGCGAGAGAGGGAGGAGTGGGAGCTACCTACTTTGACTACTGGGGCCAGGGAACCCTGGTCACCGTCTCCTCA |
| | | | SEQ ID NO: 26455 | SEQ ID NO: 30461 |
| | | AA | DIVMTQSPDSLAVSLGERATINCKSSQSVLYISNNKNYLAWYQQKPGQPPKLLIYWASTRESGVPDRFSGSGSGTDFTLTISSLQAEDVAVYYCQQYYRAPTFGQGTRLEIK | QVQLQESGPGLVKPSETLSLTCTVSGGSISSYYWSWIRQPAGKGLEWIGRIDTSGITNYNPSLKSRVTMSVDTSKSQISLKLSSVTAADTAVYYCAREGGVGATYFDYWGQGTLVTVSS |
| | | | SEQ ID NO: 26456 | SEQ ID NO: 30462 |

FIGURE 50

| | | | | |
|---|---|---|---|---|
| **iPS:435673** | **21-225_169E6** | NA | GACATTGTGATGACTCAGTCTCCACTCTCCCTGCCCGTCACCCCTGGAGAGCCGGCCTCCATCTCCTGCAGGTCTAGTCAGAGCCTCCTGCATAATAATGGATACAAGTATTTGGATTGGTACCTGCAGAAGCCAGGGCAGTCTCCACAGCTCCTGATCTATTTGGGTTCTAATCGGGCCTCCGGGGTCCCTGACAGGTTCAGTGCCAGTGGATCAGGCACAGATTTTACACTGAAAATCAGCAGAGTGGAGGCTGAGGATGTTGGGGTTTATTACTGCATGCAAGTTCTACAAACTCCGTGGACGTTCGGCCAAGGGACCAAGGTGGAAATCAAA | GAGGTGCAACTGGTGGAGTCTGGGGGAGGCTTGGTACAGCCTGGGGGGTCCCTGAGACTCTCCTGTGCAGCCTCAGGATTCACCTTCAGTGGCCATAGCATGAACTGGGTCCGCCAGGCTCCAGGGAAGGGGCTGGAGTGGGTTGCATACATTAGCATTAGTAGTAGTACCATAAAGTACGCAGACTCTGTGAAGGGCCGATTCACCATCTCCAGAGACAATGCCAGGGACTCACTGTATCTGCAAATGAACAGCCTGAGAGACGAGGACACGGCTGTGTATTACTGTGCGAGAAGGGGGATTACTGTGGTTCGGAATGAGGACGGTTTGGACGTCTGGGGCCAAGGGACCACGGTCACCGTCTCCTCA |
| | | | SEQ ID NO: 26457 | SEQ ID NO: 30463 |
| | | AA | DIVMTQSPLSLPVTPGEPASISCRSSQSLLHNNGYKYLDWYLQKPGQSPQLLIYLGSNRASGVPDRFSASGSGTDFTLKISRVEAEDVGVYYCMQVLQTPWTFGQGTKVEIK | EVQLVESGGGLVQPGGSLRLSCAASGFTFSGHSMNWVRQAPGKGLEWVAYISISSSTIKYADSVKGRFTISRDNARDSLYLQMNSLRDEDTAVYYCARRGITVVRNEDGLDVWGQGTTVTVSS |
| | | | SEQ ID NO: 26458 | SEQ ID NO: 30464 |
| **iPS:435675** | **21-225_169D7** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATCATAGTTGCCCGTGGACGTTCGGCCAAGGGACCAAGGTGGAAATCAAA | CAGGTGCAGCTGCAGGAGTCGGGCCCAGGACTGGTGAAGCCTTCGGAGACCCTGTCCCTCACCTGCACTGTCTCTGGTGGCTCCATCAGTAGTTACTACTGGACCTGGATCCGGCAGCCCGCCGGGAAGGGACTGGAGTGGATTGGGCGTATCTATACCAGTGGGAGCACCAACTACAACCCCTCCCTCAAGAGTCGAGTCACCATGTCAGTAGACACGTCCAAGAACCAGTTCTCCCTGAAGCTGAGCTCTGTGACCGCCGCGGACACGGCCGTGTATTTCTGTGCGAAAGTCGGGAGGTACTACTACGGTATGGACGTCTGGGGCCAAGGGACCACGGTCACCGTCTCCTCA |
| | | | SEQ ID NO: 26459 | SEQ ID NO: 30465 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHHSCPWTFGQGTKVEIK | QVQLQESGPGLVKPSETLSLTCTVSGGSISSYYWTWIRQPAGKGLEWIGRIYTSGSTNYNPSLKSRVTMSVDTSKNQFSLKLSSVTAADTAVYFCAKVGRYYYGMDVWGQGTTVTVSS |
| | | | SEQ ID NO: 26460 | SEQ ID NO: 30466 |
| iPS:435677 | 21-225_169C10 | NA | GACATCCAGATGACCCAGTCTCCATCCTCACTGTCTGCATCTGTAGGAGACAGAGTCACCATCACTTGTCGGGCGAGTCAGGACATTAGCAATTATTTAGCCTGGTTTCAGCAGAAACCAGGGAAAGCCCCTAAGTCCCTAATCTTTTCTGCATCCAGTTTGCAAAGTGGGGTCCCATCAAAATTCAGCGGCAGTGGATCTGGGACAGATTTCAATCTCACCATCAGCAGCCTGCAGCCTGAAGATTTTGCAACTTATTACTGCCAACAATCTGATAGTTACCCTCTCACTTTCGGCGGAGGGACCAAGGTGGAGATCAAA | CAGGTGCAGCTGGTGCAGTCTGGGGCTGAGGTGAAGAAGCCTGGGGCCTCAGTGAGGGTCTCCTGCAAGGCTTCTGGATACACCTTCACCGGCTACTTTATGCACTGGGTGCGACAGGCCCCTGGACAAGGGCTTGAGTGGATGGGATGGATCAAGCCTAAGAGTGGTGGCACAAACTCTGCACAGAGGTTTCAGGGCAGGGTCACCATGACCAGGGACACGTCCATCAACACAGCCTACATGGAGCTGAACAGGCTGAGATCTGACGACACGGCCGTGTATTACTGTGCGAGAGGGGGGACTACGGTGGCTACGTGGGGGGTCTTTGACTACTGGGGCCAGGGAACCCTGGTCACCGTCTCCTCA |
| | | | SEQ ID NO: 26461 | SEQ ID NO: 30467 |
| | | AA | DIQMTQSPSSLSASVGDRVTITCRASQDISNYLAWFQQKPGKAPKSLIFSASSLQSGVPSKFSGSGSGTDFNLTISSLQPEDFATYYCQQSDSYPLTFGGGTKVEIK | QVQLVQSGAEVKKPGASVRVSCKASGYTFTGYFMHWVRQAPGQGLEWMGWIKPKSGGTNSAQRFQGRVTMTRDTSINTAYMELNRLRSDDTAVYYCARGGTTVATWGVFDYWGQGTLVTVSS |
| | | | SEQ ID NO: 26462 | SEQ ID NO: 30468 |

FIGURE 50

| | | | | |
|---|---|---|---|---|
| iPS:435679 | 21-225_169D10 | NA | GACATCCAGATGACCCAGTCTCCATCTTCACT GTCTGCATCTGTAGGAGACAGAGTCACCATCA CTTGTCGGGCGAGTCAGGACATTAGCAATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGGACAGATTTCACTCTCACCATC AGCAGCCTGCAGCCTGAAGATTTTGCAACTTA TTACTGCCAACAGTATCATAGTTACCCATTCA CTTTCGGCCCTGGGACCAAAGTGGATATCAAA | GAGGTGCAGTTGTTGGAGTCTGGGGGAGGCTTGG TGCAGTCTGGGGGGTCCCTGAGACTCTCCTGTGC AGCCTCTGGATTCACCTTTAGCAGCTATGTCATG AGTTGGGTCCGCCAGGCTCCAGGGAAGGGGCTG GAGTGGGTCTCAGCTATTAGTGGTAGTGGTAGTA GAATATACTACGCGGACTCCGTGAAGGGCCGGTT CACCATCTCCAGAGACAATTCCAAGAACACGCTG TATCTGCAAATGAACAGCCTGAGAGCCGAGGAC ACGGCCTTATATTACTGTGCGAGAGTGGCTTTCT TTGACTATTGGGGCCAGGGAACCCTGGTCACCGT CTCCTCA |
| | | | SEQ ID NO: 26463 | SEQ ID NO: 30469 |
| | | AA | DIQMTQSPSSLSASVGDRVTITCRASQDISNYLA WFQQKPGKAPKSLIYAASSLQSGVPSKFSGSGSG TDFTLTISSLQPEDFATYYCQQYHSYPFTFGPGT KVDIK | EVQLLESGGGLVQSGGSLRLSCAASGFTFSSYVMS WVRQAPGKGLEWVSAISGSGSRIYYADSVKGRFTI SRDNSKNTLYLQMNSLRAEDTALYYCARVAFFDY WGQGTLVTVSS |
| | | | SEQ ID NO: 26464 | SEQ ID NO: 30470 |
| iPS:435681 | 21-225_169D11 | NA | GACATCCAGATGACCCAGTCTCCATCCTCCCT GTCTGCATCTGTAGGAGACAGAGTCACCATCA CTTGCCGGGCAAGTCAGGGCATTAGAGATGAT TTAGGCTGGTATCAACAGAAACCAGGGAAAG TCCCTAAGCGCCTGATATATGCTGCATCCAGT TTGCAAAGTGGGGTCCCATCAAGGTTCAGCGG CAGTGGATCTGGGACAGAATTCACTCTCACAA TCAGCAGCCTACAGCCTGAAGATTTTGCAACT TATTACTGTCTTCAGCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAATTGAACAGCCTGAGAGCCGAGG ACACGGCTGTGTATTACTGTGCGAGAGAAAGGTA TAGCAGTGGCTGGTACGACTACGGTATGGACGTC TGGGGCCAAGGGACCACGGTCACCGTCTCCTCA |
| | | | SEQ ID NO: 26465 | SEQ ID NO: 30471 |

FIGURE 50

| | | | | |
|---|---|---|---|---|
| | | AA | DIQMTQSPSSLSASVGDRVTITCRASQGIRDDLGWYQQKPGKVPKRLIYAASSLQSGVPSRFSGSGSGTEFTLTISSLQPEDFATYYCLQHYSYPRTFGQGTKVEIK | QVQLVESGGGVVQPGRSLRLSCAASGFTFSDYVMHWVRQAPGKGLEWVAVIWYDGSNKYYADSVKGRFTISRDNSKNTLYLQLNSLRAEDTAVYYCARERYSSGWYDYGMDVWGQGTTVTVSS |
| | | | SEQ ID NO: 26466 | SEQ ID NO: 30472 |
| **iPS:435683** | **21-225_170A1** | NA | GAGATTGTGATGACCCAGACTCCACTCTTCCTGTCCGTCACCCCTGGACAGCCGGCCTCCATCTCCTGCAAGTCTAGTCAGAGCCTCCTGCATGGTGATGGAAAGACCTATTTGTTTTGGTACCTGCAGAAGCCAGGCCAGCCTCCACAGGTCCTGATCTATGAAGTTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGGTTTATTACTGCATGCAAAGTATTCAGCTTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAATTATATGGTATGATGGAAGTTATAAATACTATGCAGATTCCGTGAAGGGCCGATTCACCATCTCCAGAGACAATTCCAAGAACACACTGTATCTGCAAATGAACAGCCTGAGAGCCGAGGACACGGCTGTGTATTACTGTGCGAGAGATGCCCACGATTTTTGGAGTGGTTACTTTGACTCCTGGGGCCAGGGAACCCTGGTCACCGTCTCCTCA |
| | | | SEQ ID NO: 26467 | SEQ ID NO: 30473 |
| | | AA | EIVMTQTPLFLSVTPGQPASISCKSSQSLLHGDGKTYLFWYLQKPGQPPQVLIYEVSNRFSGVPDRFSGSGSGTDFTLKISRVEAEDVGVYYCMQSIQLPWTFGQGTKVEIK | QVQLVESGGGVVQPGRSLRLSCAASGFTFSSYGMHWVRQAPGKGLEWVAIIWYDGSYKYYADSVKGRFTISRDNSKNTLYLQMNSLRAEDTAVYYCARDAHDFWSGYFDSWGQGTLVTVSS |
| | | | SEQ ID NO: 26468 | SEQ ID NO: 30474 |

FIGURE 50

| | | | | |
|---|---|---|---|---|
| **iPS:435685** | **21-225_170E1** | NA | GACATCCAGATGACCCAGTCTCCATCCTCACTGTCTGCATCTGAAGGAGACAGAGTCACCATCACTTGTCGGGCGAGTCAGGGCATTAGCAATTATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATCATAGTTACCCATTCACTTTCGGCCCTGGGACCAAAGTGGATATCAAA | GAGGTGCAGTTGTTGGAGTCTGGGGGAGGCTTGGTACAGCCTGGGGGGTCCCTGAGACTCTCCTGTGCAGCCTCTGGATTCACCTTTAGCAGCTATGTCATGAGTTGGGTCCGCCAGGCTCCAGGGAAGGGGCTGGAGTGGGTCTCAGCTATTAGTGGTAGTGGTAATAGAATATACTACGCAGACTCCGTGAAGGGCCGGTTCACCATCTCCAGAGACAATTCCAAGAACACGCTGTATCTGCAAATGAACAGCCTGAGAGCCGAGGACACGGCCTTATATTACTGTGCGAGAGTGGCTTTCTTTGACTATTGGGGCCAGGGAACCCTGGTCACCGTCTCCTCA |
| | | | SEQ ID NO: 26469 | SEQ ID NO: 30475 |
| | | AA | DIQMTQSPSSLSASEGDRVTITCRASQGISNYLAWFQQKPGKAPKSLIYAASSLQSGVPSKFSGSGSGTDFTLTISSLQPEDFATYYCQQYHSYPFTFGPGTKVDIK | EVQLLESGGGLVQPGGSLRLSCAASGFTFSSYVMSWVRQAPGKGLEWVSAISGSGNRIYYADSVKGRFTISRDNSKNTLYLQMNSLRAEDTALYYCARVAFFDYWGQGTLVTVSS |
| | | | SEQ ID NO: 26470 | SEQ ID NO: 30476 |
| **iPS:435687** | **21-225_170H1** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTACATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTATTGTCTACAGCATAGTAGTAACCCGTGGACGTTCGGCCAAGGGACCAAGGTGGAAAGCAAA | CAGGTGCAGCTGCAGGAGTCGGGCCCAGGACTGGTGAAGCCTTCGGAGACCCTGTCCCTCACCTGCACTGTCTCTGGTGGCTCCATCAGTAGTTATTACTGGAGCTGGATCCGGCAGCCCGCCGGGAAGGGACTGGAGTGGATTGGGCGTATCTATACCAGTGGGAGCACCAACTACAACCCCTCCCTCAAGAGTCGAGTCACCATGTCAGTAGACACGTCCAAGAACCAGTTCTCCCTGAAGCTGAGCTCTGTGACCGCCGCGGACACGGCCGTGTATTACTGTGCGAGAGTCGGGAGGTACTACTATGGTATGGACGTCTGGGGCCAAGGGACCACGGTCACCGTCTCCTCA |
| | | | SEQ ID NO: 26471 | SEQ ID NO: 30477 |

FIGURE 50

| | | | | |
|---|---|---|---|---|
| | | AA | DIQMTQSPSSLSASVGDRVTITCRASQGIRNDLGWYQQKPGKAPKRLIYATSSLQSGVPSRFSGSGSGTEFTLTISSLQPEDFATYYCLQHSSNPWTFGQGTKVESK | QVQLQESGPGLVKPSETLSLTCTVSGGSISSYYWSWIRQPAGKGLEWIGRIYTSGSTNYNPSLKSRVTMSVDTSKNQFSLKLSSVTAADTAVYYCARVGRYYYGMDVWGQGTTVTVSS |
| | | | SEQ ID NO: 26472 | SEQ ID NO: 30478 |
| **iPS:435689** | **21-225_170F3** | NA | GACATCCAGATGACCCAGTCTCCATCCTCCCTGTCTGCTTCTGTACAAGACAGAGTCACCATCACTTGCCGGGCAAGTCGGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAACAGCCTGCAGCCTGAAGATTTTGCAACTTATTACTGTCTACAGCATTATAGTTACCCTCGGACGTTCGGCCAAGGGACCAAGGTGGAAATCAAA | CAGGTGCAGCTGGTGGAATCTGGGGGAGGCGTGGTCCAGCCTGGGAGGTCCCTGAGACTCTCCTGTGCAGCGTCTGGATTCTCCTTCAGTGACTATGTCATGCACTGGGTCCGCCAGGCTCCAGGCAAGGGGCTGGAGTGGGTGGCAGTTATATGGTATGATGGAAGTAATAAATACTATGAAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGACGTATAGCAGCAGCTGGTACGACTACGGTATGGACGTCTGGGGCCAAGGGACCACGGTCACCGTCTCCTCA |
| | | | SEQ ID NO: 26473 | SEQ ID NO: 30479 |
| | | AA | DIQMTQSPSSLSASVQDRVTITCRASRGIRNDLGWYQQKPGKAPKRLIYAASSLQSGVPSRFSGSGSGTEFTLTINSLQPEDFATYYCLQHYSYPRTFGQGTKVEIK | QVQLVESGGGVVQPGRSLRLSCAASGFSFSDYVMHWVRQAPGKGLEWVAVIWYDGSNKYYEDSVKGRFTISRDNSKNTLYLQMNSLRAEDTAVYYCARETYSSSWYDYGMDVWGQGTTVTVSS |
| | | | SEQ ID NO: 26474 | SEQ ID NO: 30480 |

FIGURE 50

| | | | | |
|---|---|---|---|---|
| **iPS:435693** | **21-225_170G4** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCACCAGAAACCGGGGAAAGCCCCTAAGCGCCTGATCTATGCTGCATCCACTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTACAGCGTCTGGATTCACCTTCAGTACCTATGGCATGCACTGGGTCCGCCAGGCTCCAGGCAAGGGGCTGGAGTGGGTGTCAATTATATGGTATGATGGAACTAATAAATACTATGCAGACTCCGTGAAGGGCCGATTCACCATCTCCAGAGACAATTCCAAGAACACGCTGTTTCTGCAACTGAACAGCCTGAGAGCCGAGGACACGGCTATGTATTACTGTGCGCGAGATCCCTTACGTGGATACAATGACCCGGTTATGGACTACTGGGGCCAGGGAACCCTGGTCACCGTCTCCTCA |
| | | | SEQ ID NO: 26475 | SEQ ID NO: 30481 |
| | | AA | DIQMTQSPSSLSASVGDRVTITCRASQGIRNDLGWYHQKPGKAPKRLIYAASTLQSGVPSRFSGSGSGTEFTLTISSLQPEDFATYYCLQHYSYPLTFGGGTKVEIK | QVQLVESGGGVVQPGRSLRLSCTASGFTFSTYGMHWVRQAPGKGLEWVSIIWYDGTNKYYADSVKGRFTISRDNSKNTLFLQLNSLRAEDTAMYYCARDPLRGYNDPVMDYWGQGTLVTVSS |
| | | | SEQ ID NO: 26476 | SEQ ID NO: 30482 |
| **iPS:435695** | **21-225_170D5** | NA | GACATCCAGATGACCCAGTCTCCATCCTCCCTGTCTGCATCTGTCGGAGACAGAGTCACCATCACTTGCCGGGCAAGTCAGGGCATTAGAAATGATTTAGGCTGGTATCAGGAGAAACCAGGGAAAGCCCCTAAACACCTGATCTATGCTGCATCCAGTTTGCAAAATGGGGTCCCATCAAGGTTCAGCGGCAGTGGATCTGGGACAGAGTTCACTCTCACAATCAGCAGCCTGCAGCCTGAAGATTTTGCAACTTATTACTGTCTACAGCATTATAGTTTCCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAATTATATGGTATGATGGAACTAATAAATACTATGCAGACTCCGTGAAGGGCCGATTCACCATCTCCAGAGACAATTCCAAGAACACGCTGTATCTGCAACTGAACAGCCTGAGAGCCGAGGACACGGCTGTGTATTACTGTGCGCGAGATCCCTTACGTGGATACAATGACCCGGTTATGGACTACTGGGGCCAGGGAACCCTGGTCACCGTCTCCTCA |
| | | | SEQ ID NO: 26477 | SEQ ID NO: 30483 |

FIGURE 50

| | | | | |
|---|---|---|---|---|
| | | AA | DIQMTQSPSSLSASVGDRVTITCRASQGIRNDLG WYQEKPGKAPKHLIYAASSLQNGVPSRFSGSGS GTEFTLTISSLQPEDFATYYCLQHYSFPLTFGGGT KVEIK | QVQLVESGGGVVQPGRSLRLSCAASGFTFSSYGMH WVRQAPGKGLEWVAIIWYDGTNKYYADSVKGRF TISRDNSKNTLYLQLNSLRAEDTAVYYCARDPLRG YNDPVMDYWGQGTLVTVSS |
| | | | SEQ ID NO: 26478 | SEQ ID NO: 30484 |
| iPS:435697 | 21-225_170G5 | NA | GACATCCAGATGACCCAGTCTCCATCCTCCCT GTCTGCATCTGTAGGAGACAGAGTCACCATCA CTTGCCGGGCAAGTCAGGGCATTAGAACTGAT TTAGGCTGGTTTCAGCAGAAACCAGGGAAAGC CCCTAAGCGCCTGATCTATACTGCATCCAGTTT GCAAAGTGGGGTCCCATCAAGGTTCAGCGGCA GTGGATCTGGGACAGAATTCACTCTCACGATC AGCAGCCTGCAGCCTGAAGATTTTGCAACTTA TTACTGTCTACAGCATTATAGTTACCCGCTCAC TTTCGGCGGAGGGACCAAGGTGGAGATCAAA | CAGGTGCAGCTGGTGGAGTCTGGGGGAGGCGTG GTCCAGCCTGGGGGGTCCCTGAGACTCTCCTGTG CAGCGTCTGGCTTCACCTTCAGTACCTATGGCAT GCACTGGGTCCGCCAGGCTCCAGGCAAGGGGCT GGAGTGGGTGACAATTATATGGTATGATGGGACT AATAAATACTATGCAGACTCCGTGAAGGGCCGA TTCACCATCTCCAGAGACAATTCCAAGAGCACGC TGTATCTGCAAATGAACAGCCTGAGAGCCGAGG ACACGGCTGTGTATTTCTGTGCGAGAGATCCCTT ACGTGGATACAATGACCCGGTTATGGACTACTGG GGCCAGGGAACCCTGGTCACCGTCTCCTCA |
| | | | SEQ ID NO: 26479 | SEQ ID NO: 30485 |
| | | AA | DIQMTQSPSSLSASVGDRVTITCRASQGIRTDLG WFQQKPGKAPKRLIYTASSLQSGVPSRFSGSGSG TEFTLTISSLQPEDFATYYCLQHYSYPLTFGGGT KVEIK | QVQLVESGGGVVQPGGSLRLSCAASGFTFSTYGMH WVRQAPGKGLEWVTIIWYDGTNKYYADSVKGRFT ISRDNSKSTLYLQMNSLRAEDTAVYFCARDPLRGY NDPVMDYWGQGTLVTVSS |
| | | | SEQ ID NO: 26480 | SEQ ID NO: 30486 |

FIGURE 50

| | | | | |
|---|---|---|---|---|
| **iPS:435699** | **21-225_170D6** | NA | GACATCCAGATGACCCAGTCTCCATCCTCTCTGTCTGCATCTGTAGGAGACAGAGTCGCCATCACTTGTCGGGCGAGTCAGGACATTGGCAATTGTTTAGCCTGGTTTCAGCAGAAACCAGGGAAAGCCCCTAAGTCCCTGATCTATTCTGCGTCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAATCTGATAGTTACCCTCTCACTTTCGGCGGAGGGACCAAGGTGGAGATCAAA | CAGGTGCAGCTGGTGCAGTCTGGGGCTGAGGTGAAGAAGCCTGGGGCCTCAGTGAGGGTCTCCTGCAAGGCTTCTGGATACACCTTCACCGGCTACTTTATACACTGGGTGCGACAGGCCCCTGGACAAGGGCTTGAGTGGATGGGATGGATCAAGCCTAACAGTGGTGGCACAAACTCTGCACAGAGGTTTCAGGGCAGGGTCACCATGACCAGGGACACGTCCATCAACACAGCCTACATGGAGCTGAACAGGCTGAGATCTGACGACACGGCCGTGTATTACTGTGCGAGAGGGGGGACTACGGTGGCTACGTGGGGGGTCTTTGACTACTGGGGCCAGGGAACCCTGGTCACCGTCTCCTCA |
| | | | SEQ ID NO: 26481 | SEQ ID NO: 30487 |
| | | AA | DIQMTQSPSSLSASVGDRVAITCRASQDIGNCLAWFQQKPGKAPKSLIYSASSLQSGVPSKFSGSGSGTDFTLTISSLQPEDFATYYCQQSDSYPLTFGGGTKVEIK | QVQLVQSGAEVKKPGASVRVSCKASGYTFTGYFIHWVRQAPGQGLEWMGWIKPNSGGTNSAQRFQGRVTMTRDTSINTAYMELNRLRSDDTAVYYCARGGTTVATWGVFDYWGQGTLVTVSS |
| | | | SEQ ID NO: 26482 | SEQ ID NO: 30488 |
| **iPS:435701** | **21-225_170F6** | NA | GACATCGTGATGACCCAGTCTCCAGACTCCCTGGCTGTGTCTCTGGGCGCGAGGGCCACCATCAACTGCAAGTCCAGCCAGAGTGTTTTACACAGCTCCAACAATTACAACTACTTAGCTTGGTACCAGCAGAGACCAGGACAGCCTCCTAAAGTGCTCATTCACTGGGCATCTACCCGGAAATCCGGGGTCCCTGACCGATTCAGTGGCAGCGTGTCTGGGACAGATTTCACTCTCACCATCAACAGCCTGCAGGCTGAAGATGTGGCAGTTTATTACTGTCAGCAATATTATAGTACTCCGTGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGACAGAGTCACCATGACCAGGCACACCTCCATAAGCACAGCCTACATGGAGCTGAGCAGCCTGAGATCTGAGGACACGGCCGTGTATTACTGTGCGATTAGCAGTGGCTGGTACTGGTTCGACCCCTGGGGCCAGGGAACCCTGGTCACCGTCTCCTCA |
| | | | SEQ ID NO: 26483 | SEQ ID NO: 30489 |

FIGURE 50

| | | | | |
|---|---|---|---|---|
| | | AA | DIVMTQSPDSLAVSLGARATINCKSSQSVLHSSNNYNYLAWYQQRPGQPPKVLIHWASTRKSGVPDRFSGSVSGTDFTLTINSLQAEDVAVYYCQQYYSTPWTFGQGTKVEIK | QVQLVQSGAEVKKPGASVKVSCKASGYTFTNYDINWVRQATGQGLEWMGWMNPNSGNTGYAQKFQDRVTMTRHTSISTAYMELSSLRSEDTAVYYCAISSGWYWFDPWGQGTLVTVSS |
| | | | SEQ ID NO: 26484 | SEQ ID NO: 30490 |
| iPS:435703 | 21-225_170D11 | NA | GACATCCAGATGACCCAGTCTCCATCCTCCCTGTCTGCATCTGTCGGAGACAGAGTCACCATCACTTGCCGGGCAAGTCAGGGCATTAGAAATGATTTAGGCTGGTATCAGCAGAAACCAGGGAAAGCCCCTAAACACCTGATCTATGCTGCATCCAGTTTGCAAAATGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTTCCCGCTCACTTTCGGCGGAGGGACCAAGGTGGAGATCAGA | CAGGTGCAGCTGGTGGAGTCTGGGGGAGGCGTGGTCCAGCCTGGGAGGTCCCTGAGACTCTCCTGTGTAGCGTCTGGATTCACCTTCAGTAGCTATGGCATGCACTGGGTCCGCCAGGCTCCAGGCAAGGGGCTGGAGTGGGTGGCAATTATATGGTATGATGGAACTAATAAATACTATGCAGACTCCGTGAAGGGCCGATTCACCATCTCCAGAGACAATTCCAAGAACACGCTGTATCTGCAACTGAACAGCCTGAGAGCCGAGGACACGGCTGTGTATTACTGTGCGCGAGATCCCTTACGTGGATACAATGACCCGGTTATGGACTACTGGGGCCAGGGAACCCTGGTCACCGTCTCCTCA |
| | | | SEQ ID NO: 26485 | SEQ ID NO: 30491 |
| | | AA | DIQMTQSPSSLSASVGDRVTITCRASQGIRNDLGWYQQKPGKAPKHLIYAASSLQNGVPSRFSGSGSGTEFTLTISSLQPEDFATYYCLQHYSFPLTFGGGTKVEIR | QVQLVESGGGVVQPGRSLRLSCVASGFTFSSYGMHWVRQAPGKGLEWVAIIWYDGTNKYYADSVKGRFTISRDNSKNTLYLQLNSLRAEDTAVYYCARDPLRGYNDPVMDYWGQGTLVTVSS |
| | | | SEQ ID NO: 26486 | SEQ ID NO: 30492 |

FIGURE 50

| | | | | |
|---|---|---|---|---|
| **iPS:435705** | **21-225_171C3** | NA | GACATCCAGATGACCCAGTCTCCATCCTCCCTGTCTGCATCTGTAGGAGACAGAGTCACCATCACTTGCCGGGCAAGTCAGGGCATTAGAACTGATTTAGGCTGGTTTCAGCAGAAACCAGGGAAAGCCCCTAAGCGCCTGATCTATACTGCATCCAGTTTGCAAAGTGGGGTCCCATCAAGGTTCAGCGGCAGTGGATCTGGGACAGAATTCACTCTCACGATCAGCAGCCTGCAGCCTGAAGATTTTGCAACTTATTACTGTCTACAGCATTATAGTTACCCGCTCACTTTCGGCGGAGGGACCAAGGTGGAGATCAAA | CAGGTGCAGCTGGTGGAGTCTGGGGGAGGCGTGGTCCAGCCTGGGGGGTCCCTGAGACTCTCCTGTGCAGCGTCTGGCTTCACCTTCAGTACCTATGGCATGCACTGGGTCCGCCAGGCTCCAGGCAAGGGGCTGGAGTGGGTGACAATTATATGGTATGATGGGACTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCCCTTACGTGGATACAATGACCCGGTTATGGACTACTGGGGCCAGGGAACCCTGGTCACCGTCTCCTCA |
| | | | SEQ ID NO: 26487 | SEQ ID NO: 30493 |
| | | AA | DIQMTQSPSSLSASVGDRVTITCRASQGIRTDLGWFQQKPGKAPKRLIYTASSLQSGVPSRFSGSGSGTEFTLTISSLQPEDFATYYCLQHYSYPLTFGGGTKVEIK | QVQLVESGGGVVQPGGSLRLSCAASGFTFSTYGMHWVRQAPGKGLEWVTIIWYDGTNKYYADSVKGRFTISRDNSKNTLYLQMNSLRAEDTAVYYCARDPLRGYNDPVMDYWGQGTLVTVSS |
| | | | SEQ ID NO: 26488 | SEQ ID NO: 30494 |
| **iPS:435709** | **21-225_171A4** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATACTGCATCCAGTTTGCAAAGTGGGGTCCCATCAAGGTTCAGCGGCAGTGGATCTGGGACAGAATTCACTCTCACGATCAGCAGCCTGCAGCCTGAAGATTTTGCAACTTATTACTGTCTACAGCATTATAGTTACCCGCTCACTTTCGGCGGAGGGACCAAGGTGGAGATCAAA | CAGGTGCAACTGGTGGAGTCTGGGGGAGGCGTGGTCCAGCCTGGGGGGTCCCTGAGACTCTCCTGTGCAGCGTCTGGCTTCACCTTCAGTACCTATGGCATGCACTGGGTCCGCCAGGCTCCAGGCAAGGGGCTGGAGTGGGTGGCAATTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAAGACACGGCTGTGTATTACTGTGCGAGAGATCCCTTACGTGGATACAATGACCCGGTTATGGACTACTGGGGCCAGGGAACCCTGGTCACCGTCTCCTCA |
| | | | SEQ ID NO: 26489 | SEQ ID NO: 30495 |

FIGURE 50

| | | | | |
|---|---|---|---|---|
| | | AA | DIQMTQSPSSLSASVGDRVTITCRASQGIRNDLGWYQQKPGKAPKRLIYTASSLQSGVPSRFSGSGSGTEFTLTISSLQPEDFATYYCLQHYSYPLTFGGGTKVEIK | QVQLVESGGGVVQPGGSLRLSCAASGFTFSTYGMHWVRQAPGKGLEWVAIIWYDGSNKYYADSVKGRFTISRDNSKNTLYLQMNSLRAEDTAVYYCARDPLRGYNDPVMDYWGQGTLVTVSS |
| | | | SEQ ID NO: 26490 | SEQ ID NO: 30496 |
| **iPS:435711** | **21-225_171G4** | NA | GACATCCAGATGACCCAGTCTCCATCTTCCGTGTCTGCATCTGTAGGAGACAGAGTCACCATCACTTGTCGGGCGAGTCAGGGTGTTAACGACTGGTTAGCCTGGTATCAGCAGAAACCAGGGAGAGCCCCTAAGCTCCTGATCTATGATGCATCAAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGGCTAACAGTTTCCCGTGGACGTTCGGCCAAGGGACCAAGGTGGAAATCAAA | GAGGTGCAGCTGTTGGAGTCTGGGGGAGACTTAGTACAGCCTGGGGGGTCCCTGAGACTCTCCTGTGCAGCCTCTGGATTCACCTTTAGCAGCTGTGCCATGACCTGGGTCCGCCAGGCTCCAGGGAAGGGGCTGGAGTGGGTCTCAGCTATTAGTGGTCGTGGTGGTACCACGTTCTACGCAGACTCCGTGAGGGGCCGGTTCACCATCTCCAGAGACAATTCCAAGAACACGCTGTTTCTGCAAATGAACAGCCTGAGAGCCGAGGACACGGCCGTATATTACTGTGCGAAAGATCTTATTGGGGGAGCTACTTACTTTGACTACTGGGGCCAGGGAACCCTGGTCACCGTCTCCTCA |
| | | | SEQ ID NO: 26491 | SEQ ID NO: 30497 |
| | | AA | DIQMTQSPSSVSASVGDRVTITCRASQGVNDWLAWYQQKPGRAPKLLIYDASSLQSGVPSRFSGSGSGTDFTLTISSLQPEDFATYYCQQANSFPWTFGQGTKVEIK | EVQLLESGGDLVQPGGSLRLSCAASGFTFSSCAMTWVRQAPGKGLEWVSAISGRGGTTFYADSVRGRFTISRDNSKNTLFLQMNSLRAEDTAVYYCAKDLIGGATYFDYWGQGTLVTVSS |
| | | | SEQ ID NO: 26492 | SEQ ID NO: 30498 |

FIGURE 50

| | | | | |
|---|---|---|---|---|
| **iPS:435713** | **21-225_171D7** | NA | GATATTGTGATGACTCAGTCTCCACTCTCCCTGCCCGTCACCCCTGGAGAGCCGGCCTCCATCTCCTGCAGGTCTAGTCAGAGCCTCCTGTATCATAATGGATACAACTATTTGGATTGGTACCTGCAGAAGACAGGGCAGTCTCCACAGCTCCTGATCTATGTGGGTTCTAATCGGGCCTCCGGGGTCCCTGACAGGTTCAGTGGCAGTGGATCAGGCACAGATTTTACACTGAAAATCAGCAGAGTGGAGGCTGAGGATGTTGGGGTTTATTACTGCATGCAAACTCTACAAACTCCGCTCACTTTCGGCGGAGGGACCAAGGTGGAGATCAAA | CAGGTGCAGCTGGTGGAGTCTAGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAGTGGGTGGCAGTTATATCATATGACGGAAACAATAGACACTATGCAGACTCCGTGCAGGGCCGATTCACCATTTCCAGAGACAATTCCAAGAACACGCTGTCTCTGCAAATGAACAGCCTGGGAGCTGAGGACACGGCTGTGTATTACTGTGCGAGAGATCGTCACCGTTTGGACTACTACGCTTTGGACGTCTGGGGCCAAGGGACCACGGTCACCGTCTCCTCA |
| | | | SEQ ID NO: 26493 | SEQ ID NO: 30499 |
| | | AA | DIVMTQSPLSLPVTPGEPASISCRSSQSLLYHNGYNYLDWYLQKTGQSPQLLIYVGSNRASGVPDRFSGSGSGTDFTLKISRVEAEDVGVYYCMQTLQTPLTFGGGTKVEIK | QVQLVESRGGVVQPGRSLRLSCAASGFTFSSYGMHWVRQAPGKGLEWVAVISYDGNNRHYADSVQGRFTISRDNSKNTLSLQMNSLGAEDTAVYYCARDRHRLDYYALDVWGQGTTVTVSS |
| | | | SEQ ID NO: 26494 | SEQ ID NO: 30500 |
| **iPS:435715** | **21-225_171A8** | NA | GCCATCCAGATGACCCAGTCTCCATCTTCCGTGTCTGCATCTGTAGGAGACAGAGTCACCATCACTTGTCGGGCGAGTCAGGGTATTAGCAACTGGTTAGCCTGGTATCAGCAGAAACCAGGGAAAGCCCCTAACCTCATGATCCATGCTGCATTCAGTTTGCAAGGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGACTAACAGTTTCCCGTGGACGTTCGGCCAAGGGACCAAGGTGGAAATCAAA | GAGGTGCAGTTGTTGGAGTCTGGGGGAGGCTTGGTACAGCCTGGGGGGTCCCTGAGACTCTCCTGTGCAGCCTCTGGATTCACCTTTAGGAGCTCTGCCATGAGCTGGGTCCGCCAGGCTCCAGGGAAGGGGCTGGAGTGGGTCTCAGTTATTAGTGGTAGTGGTGGTAGCACATTCTACACAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATCGAATAGCAGTGGCTGGTTTGACTACTGGGGCCAGGGAACCCTGGTCACCGTCTCCTCA |
| | | | SEQ ID NO: 26495 | SEQ ID NO: 30501 |

FIGURE 50

| | | | | |
|---|---|---|---|---|
| | | AA | AIQMTQSPSSVSASVGDRVTITCRASQGISNWLAWYQQKPGKAPNLMIHAAFSLQGGVPSRFSGSGSGTDFTLTISSLQPEDFATYYCQQTNSFPWTFGQGTKVEIK | EVQLLESGGGLVQPGGSLRLSCAASGFTFRSSAMSWVRQAPGKGLEWVSVISGSGGSTFYTDSVKGRFTISRDNSKNTLYLQMNSLRAEDTAVYYCAKSNSSGWFDYWGQGTLVTVSS |
| | | | SEQ ID NO: 26496 | SEQ ID NO: 30502 |
| iPS:435717 | 21-225_171A9 | NA | GACATCCAGATGACCCAGTCTCCATCTTCCGTGTCTGCATCTGTAGGAGACAGAGTCACCATCACTTGTCGGGCGAGTCAGGATATTACCACCTGGTTAGCCTGGTATCAGCAGAAACCAGGGAAAGCCCCTAAGCTCCTGATCTATGATGCATCCAGTTTGCAAAGTGCGGTCCCATCAAGGTTCAGCGGCAGTGGATCTGGGACAGATTTCACTCTCACCGTCAGCAGTTTGCAGCCTGAAGATTTTGCAACTTACTATTGTCTACAGACTAACAGTTTCCCGTGGACGTTCGGCCAAGGGACCAAGGTGGAGATCAAG | GAGGTGCAGTTGTTGGAGTCTGGGGGAGGCTTGGCACAGCCTGGGGGGTCCCTGAGACTCTCCTGTGCAGCCTCTGGATTCACCTTTAGCAGCTATGCCATGACTTGGGTCCGCCAGGCTCCAGGGAAGGGGCTGGAGTGGGTCTCAGCTATTAGTGGTAGTGGTGGTAACACATTCAACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCAAAGCTGGGGATCGACTACTACTACTACGGTATGGACGTCTGGGGCCAAGGGACCACGGTCACCGTCTCCTCA |
| | | | SEQ ID NO: 26497 | SEQ ID NO: 30503 |
| | | AA | DIQMTQSPSSVSASVGDRVTITCRASQDITTWLAWYQQKPGKAPKLLIYDASSLQSAVPSRFSGSGSGTDFTLTVSSLQPEDFATYYCLQTNSFPWTFGQGTKVEIK | EVQLLESGGGLAQPGGSLRLSCAASGFTFSSYAMTWVRQAPGKGLEWVSAISGSGGNTFNADSVKGRFTISRDNSKNTLYLQMNSLRAEDTAVYYCAKLGIDYYYYGMDVWGQGTTVTVSS |
| | | | SEQ ID NO: 26498 | SEQ ID NO: 30504 |

FIGURE 50

| | | | | |
|---|---|---|---|---|
| **iPS:435719** | **21-225_171A11** | NA | GACATCCAGATGACCCAGTCTCCATCCTCCCTGTCTGCATCTGTAGGAGACAGAGTCACCATCACTTGCCGGGCAAGTCAGGGCATTAGAAATAATTTAGGCTGGTATCAGCAGAAACCAGGGAAAGCCCCTAAGCGCCTGATCTATCCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGGATCATAGTTACCCATTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTGGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGATAGGGGCAGCTCCTGGGGCCAGGGAATCCTGGTCACCGTCTCCTCA |
| | | | SEQ ID NO: 26499 | SEQ ID NO: 30505 |
| | | AA | DIQMTQSPSSLSASVGDRVTITCRASQGIRNNLGWYQQKPGKAPKRLIYPASSLQSGVPSRFSGSGSGTEFTLTISSLQPEDFATYYCLQDHSYPFTFGPGTKVDIK | EVQLVESGGGLVKPGGSLRLSCAASGFTFSSYSMNWVRQAPGKGLEWVSSISGSSSYIYYADSVKGRFTISRDNAKNSLYLQMNSLRAEDTAVYYCARDRGSSWGQGILVTVSS |
| | | | SEQ ID NO: 26500 | SEQ ID NO: 30506 |
| **iPS:435721** | **21-225_172B3** | NA | GACATCCAGATGACCCAATCTCCATCCTCCCTGTCTGCATCTGTAGGAGACAGAGTCACCATCACTTGCCGGGCTAGTCAGGGCATTAGAAATGATTTAGGCTGGTATCAGCAGAAACCAGGGAAAGCCCCTAAGCGCCTGATCGGTGCTGAATCCAGTTTGCAAAGTGGGGTCCCATCAAGGTTCAGCGGCAGTGGATCTGGGACAGAATTCACTCTCACAATCAGCGGCCTGCAGCCTGAAGATTTTGCAACTTATTACTGTCTACAGCATTATAGTTACCCGCTCACTTTCGGCGGAGGGACCAAAGTGGAGATCAAA | CAGGTGCAGCTGGTGGAGTCTGGGGGAGGCGTGGTCCAGCCTGGGAGGCCCCTGAGACTCTCCTGTGCAGCGTCTGGATTCACCTTCAGTACCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCCCTTACGTGGATACAATGACCCGGTTATGGACTACTGGGGCCAGGGAACCCTGGTCACCGTCTCCTCA |
| | | | SEQ ID NO: 26501 | SEQ ID NO: 30507 |

FIGURE 50

| | | | | |
|---|---|---|---|---|
| | | AA | DIQMTQSPSSLSASVGDRVTITCRASQGIRNDLGWYQQKPGKAPKRLIGAESSLQSGVPSRFSGSGSGTEFTLTISGLQPEDFATYYCLQHYSYPLTFGGGTKVEIK | QVQLVESGGGVVQPGRPLRLSCAASGFTFSTYGMHWVRQAPGKGLEWVAVIWYDGSNKYYADSVKGRFTISRDNSKNTLYLQMNSLRAEDTAVYYCARDPLRGYNDPVMDYWGQGTLVTVSS |
| | | | SEQ ID NO: 26502 | SEQ ID NO: 30508 |
| iPS:435723 | 21-225_172B7 | NA | GATATTGTGATGACCCAGACTCCACTCTCTCTGTCCGTCACCCCTGGACAGCCGGCCTCCATCTCCTGCAAGTCTAGTCAGAGCCTCCTGCATGGTGATGGAAAGACCTATTTCTATTGGTACCTGCAGAAGCCAGGCCAGCCTCCACAGGTCCTGTTATTTGAAGTTTCCCACCGGTTCTCTGGAGTGCCAGATAGGTTCAGTGGCGGCGGGTCAGGGACAGATTTCACACTGAAGATCAGCCGGGTGGAGGCTGAGGATGTTGGGGTTTACTATTGCATGCAAAGTATACAGTTTCCGTGGACGTTCGGCCAAGGGACCAGGGTGGACATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTAGAGTGGGTGGCAATTATATGGTATGATGGAAGTAATAAATACTATGTAGACTCCGTGAAGGGCCGATTCACCATCTCCAGAGACAATTCCAAGAACACGCTGTATCTCCAAATGAACAGCCTGAGAGCCGAAGACACGGCTGTGTACTATTGTGCGAGAGAGGCGTACGATTTTTGGAGTGGTTATTGGGACTACTGGGGCCAGGGAACCCTGGTCACCGTCTCCTCA |
| | | | SEQ ID NO: 26503 | SEQ ID NO: 30509 |
| | | AA | DIVMTQTPLSLSVTPGQPASISCKSSQSLLHGDGKTYFYWYLQKPGQPPQVLLFEVSHRFSGVPDRFSGGGSGTDFTLKISRVEAEDVGVYYCMQSIQFPWTFGQGTRVDIK | QVQLVESGGGVVQPGRSLRLSCAASGFTFSNYGMHWVRQAPGKGLEWVAIIWYDGSNKYYVDSVKGRFTISRDNSKNTLYLQMNSLRAEDTAVYYCAREAYDFWSGYWDYWGQGTLVTVSS |
| | | | SEQ ID NO: 26504 | SEQ ID NO: 30510 |

FIGURE 50

| | | | | |
|---|---|---|---|---|
| **iPS:435725** | **21-225_172G8** | NA | GACATCCAGATGACCCAGTCTCCATCCTCCCTGTCTGCATCTGTCGGAGACAGAGTCACCATCACTTGCCGGGCAAGTCAGGGCGTTAGAAATGATTTAGGCTGGTATCAGCAGAAACCAGGGAAAGCCCCTAAACACCTGATCTATGCTGCATCCAGTTTGCAAAATGGGGTCCCATCAAGGTTCAGCGGCAGTGGCTCTGGGACAGAATTCACTCTCACAATCAGCAGCCTGCAGCCTGAAGATTTTGCCACTTATTACTGTCTACACCATTATAGTTTCCCGCTCACTTTCGGCGGAGGGACCAAGGTGGAGATCAAA | CAGGTGCAGATGGTGGAGTCTGGGGGAGGCGTGGTCCAGCCTGGGAGGTCCCTGAGACTCTCCTGTGCAGCGTCTGGATTCACCTTCAGTACCTATGGCATGCACTGGGTCCGCCAGGCTCCAGGCAAGGGGCTGGAATGGATGGCAATTATATGGTATGATGGAACTAATAAATACTATGCAGACTCCGTGAAGGGCCGATTCACCATCTCCAGAGACAATTCCAAGAACACGCTGTATCTGCAACTGAACAGCCTGAGAGCCGAGGACACGGCTGTGTATTACTGTGCGCGAGATCCCTTACGTGGATACAATGACCCGGTTATGGACTACTGGGGCCAGGGAACCCTGGTCACCGTCTCCTCA |
| | | | SEQ ID NO: 26505 | SEQ ID NO: 30511 |
| | | AA | DIQMTQSPSSLSASVGDRVTITCRASQGVRNDLGWYQQKPGKAPKHLIYAASSLQNGVPSRFSGSGSGTEFTLTISSLQPEDFATYYCLHHYSFPLTFGGGTKVEIK | QVQMVESGGGVVQPGRSLRLSCAASGFTFSTYGMHWVRQAPGKGLEWMAIIWYDGTNKYYADSVKGRFTISRDNSKNTLYLQLNSLRAEDTAVYYCARDPLRGYNDPVMDYWGQGTLVTVSS |
| | | | SEQ ID NO: 26506 | SEQ ID NO: 30512 |
| **iPS:435727** | **21-225_172E11** | NA | GACATCGTGATGACCCAGTCTCCAGACTCCCTGGCGGTGTCTCTGGGCGAGAGGGCCACCATCAACTGCAAGTCCAGCCAGAGTGTTTTACACAGCTCCAACAATAACAACTACTTAGCTTGGTACCAGCAGAAACCAGGACAGCCTCCTAAGCTGCTCATTTACTGGGCATCTACTCGGGAATCCGGGGTCCCTGACCGATTCAGTGGCAGCGGGTCTGGGACAGATTTCATTCTCACCATCAGCGGCCTGCAGGCTGAAGATGTGGCAGTTTATTACTGTCAGCAATATTTTACTACTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGATGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGTATAGCAGTGGCTGGTACCGGTTTGACTACTGGGGCCAGGGAACCCTGGTCACCGTCTCCTCA |
| | | | SEQ ID NO: 26507 | SEQ ID NO: 30513 |

FIGURE 50

| | | | | |
|---|---|---|---|---|
| | | AA | DIVMTQSPDSLAVSLGERATINCKSSQSVLHSSNNNNYLAWYQQKPGQPPKLLIYWASTRESGVPDRFSGSGSGTDFILTISGLQAEDVAVYYCQQYFTTPCSFGQGTKLEIK | QVQLVQSGAEVKKPGASVMVSCKASGYTFTNYDINWVRQATGQGLEWMGWMHPNSGNTGYAQKFQGRVTMTRNTSISTAYMELSSLRSEDTAVYYCAYSSGWYRFDYWGQGTLVTVSS |
| | | | SEQ ID NO: 26508 | SEQ ID NO: 30514 |
| **iPS:435729** | **21-225_173E7** | NA | GACATCCAGATGACCCAGTCTCCATCCTCCCGGTCTGCATGTATAGGAGACAGAGCCACCATCACTTACCGTGCAAGTCAGACCATTAGCAACTATTTAAATTGGTATCAGCAGAAACCAGGGAAAGCCCCTAAACTCCTTATCTATGCTGCATCCAGTTTGCAAATTGGGGTCCCATCAAGGTTCAGTGGCAGTGGATCTGGGACAGATTTCACTCTCACCATCAGCAGTGTGCAACCTGAAGATTTTGCAACTTACTTCTGTCAACAGAGTTACAGAACCCCTCAGTGGACGTTCGGCCAAGGGACCAAGGTGGAAATCAAA | GAGGTGCAGTTGTTGGAGTCTGGGGGAGGCTCGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCATTTATTAGTGGTAGTGGTGGTAACACATTCTACGCAGACTCCGTGAAGGGCCGGTTCACCATCTCCAGAGACAATTCCAAGAACACGCTGTATCTGCAAATGAACAGCCTGCAAGCCGAGGACACGGCCGTATATTACTGTACGAAAAGGGATACCTACAACGGTTGGGATGCTTTTGATATCTGGGGCCAAGGGACAATGGTCACCGTCTCTTCA |
| | | | SEQ ID NO: 26509 | SEQ ID NO: 30515 |
| | | AA | DIQMTQSPSSRSACIGDRATITYRASQTISNYLNWYQQKPGKAPKLLIYAASSLQIGVPSRFSGSGSGTDFTLTISSVQPEDFATYFCQQSYRTPQWTFGQGTKVEIK | EVQLLESGGGSVQPGGSLRLSCAASGFTFSSYAMSWVRQAPGKGLEWVSFISGSGGNTFYADSVKGRFTISRDNSKNTLYLQMNSLQAEDTAVYYCTKRDTYNGWDAFDIWGQGTMVTVSS |
| | | | SEQ ID NO: 26510 | SEQ ID NO: 30516 |

FIGURE 50

| | | | | |
|---|---|---|---|---|
| **iPS:435731** | **21-225_173A11** | NA | GATATTGTGATGACCCAGACTCCACTCTCTCT GTCCGTCACCCCTGGACAGCCGGCCTCCATCT CCTGCAAGTCTAGTCAGAGCCTCCTGCATGGT GATGGAAAGACCTATTTGTATTGGTACCTGCA GAAGCCAGGCCAGCCTCCACAGCTCCTGATCT ATGAAGTTTCCAACCGGTTCTCTGGCGTGCCA GATAGGTTCAGTGGCAGCGGGTCAGGGACAG ATTTCACACTGAAAATCAGCCGGGTGGAGGCT GAGGATGTTGGGGTTTTTTTCTGCATGCAAAG TATACAGGTTCCGTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT AGAGTGGGTGGCAATTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGGCCTA CGATTTTTGGAGTGGTTTCTTTGACTCCTGGGGCC AGGGAACCCTGGTCACCGTCTCCTCA |
| | | | SEQ ID NO: 26511 | SEQ ID NO: 30517 |
| | | AA | DIVMTQTPLSLSVTPGQPASISCKSSQSLLHGDG KTYLYWYLQKPGQPPQLLIYEVSNRFSGVPDRFS GSGSGTDFTLKISRVEAEDVGVFFCMQSIQVPWT FGQGTKVEIK | QVQLVESGGGVVQPGRSLRLSCAASGFTFSSYGMH WVRQAPGKGLEWVAIIWYDGSNKYYADSVKGRFT ISRDNSKNTLYLQMNSLRAEDTAVYYCAREAYDF WSGFFDSWGQGTLVTVSS |
| | | | SEQ ID NO: 26512 | SEQ ID NO: 30518 |
| **iPS:435733** | **21-225_173C11** | NA | GATATTGTGATGACCCAGACTCCACTCTCTCT GTCCGTCACCCCTGGACAGCCGGCCTCCATCT CCTGCAAGTCTAGTCAGAGCCTCCTGCATAGT GAGGGAAAGACCTACTTGTATTGGTACCTGCA GAAGCCAGGCCAGCCTCCACAGCTCCTGATCT ATGAAGTTTCCCACCGGTTCTCTGGAGTGCCA GATAGGTTCAGTGGCAGCGGGTCAGGGACAG ATTTCACACTGAAAATCAGCCGGGTGGAGGCT GAGGATGTTGGGGTTTATTACTGCATGCAAAG TATACAGCTTCTCACTTTCGGCGGAGGGACCA AGGTGGAGATCAAA | CAGGTGCAGCTGGTGGAGTCGGGGGGAGGCGTG GTCCAGCCTGGGAGGTCCCTGAGACTCTCCTGTG CAGCGTCTGGATTCACCTTCAGTAGCTATGGCAT ACACTGGGTCCGCCAGGCTCCAGGCAAGGGACT GGAGTGGGTGGCACTTATATTTTATGATGGAAGT AATAAATACTATGCAGACTCCGTGAAGGGCCGA TTCACCATCTCCAGAGACAATTCCAAGAACACGC TGTATCTGCATATGAGCAGCCTGAGAGCCGAGG ACACGGCTGTGTATTACTGTGCGAGACGGTATAG CAGCAGCTGGTCCGGTGGTATGGACGTCTGGGGC CAAGGGACCACGGTCACCGTCTCCTCA |
| | | | SEQ ID NO: 26513 | SEQ ID NO: 30519 |

FIGURE 50

| | | | | |
|---|---|---|---|---|
| | | AA | DIVMTQTPLSLSVTPGQPASISCKSSQSLLHSEGKTYLYWYLQKPGQPPQLLIYEVSHRFSGVPDRFSGSGSGTDFTLKISRVEAEDVGVYYCMQSIQLLTFGGGTKVEIK | QVQLVESGGGVVQPGRSLRLSCAASGFTFSSYGIHWVRQAPGKGLEWVALIFYDGSNKYYADSVKGRFTISRDNSKNTLYLHMSSLRAEDTAVYYCARRYSSSWSGGMDVWGQGTTVTVSS |
| | | | SEQ ID NO: 26514 | SEQ ID NO: 30520 |
| iPS:435735 | 21-225_173H12 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATACTACATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAAGTTATTACTGTCTACAGCATTATAGTTTCCCGAACACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAATTATATGGTATGATGGAACTAACAAATACTATGCAGACTCCGTGAAGGGCCGATTCACCATCTCCAGAGACAATTCCAAGAACACGCTGTATCTGCAACTGAACAGCCTGAGAGCCGAGGACACGGCTGTGTATTACTGTGCGCGAGATCCCTTACGTGGATACAATGACCCGGTTATGGACTACTGGGGCCAGGGAACCCTGGTCACCGTCTCCTCA |
| | | | SEQ ID NO: 26515 | SEQ ID NO: 30521 |
| | | AA | DIQMTQSPSSLSASVGDRVTITCRASQGIRNDLGWYQQKPGKAPKRLIYTTSSLQSGVPSRFSGSGSGTEFTLTISSLQPEDFASYYCLQHYSFPNTFGGGTKVEIK | QVQLVESGGGVVQPGRSLRLSCAASGFTFSSYGMHWVRQAPGKGLEWVAIIWYDGTNKYYADSVKGRFTISRDNSKNTLYLQLNSLRAEDTAVYYCARDPLRGYNDPVMDYWGQGTLVTVSS |
| | | | SEQ ID NO: 26516 | SEQ ID NO: 30522 |

FIGURE 50

| | | | | |
|---|---|---|---|---|
| **iPS:435737** | **21-225_174G5** | NA | GACATCGTGATGACCCAGTCTCCAGATTCCCTGGCTGTGTCTCTGGGCGCGAGGGCCACCATCAATTGCAAGTCCAGCCAGAGTGTATTACACAGCTCCAACAATTACAACTACTTAACTTGGTACCAGCAGAAATCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATCGTACTCCGTGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGGTTAGCAGTGGCTGGTACTGGTTCGACCCCTGGGGCCAGGGAACCCTGGTCACCGTCTCCTCA |
| | | | SEQ ID NO: 26517 | SEQ ID NO: 30523 |
| | | AA | DIVMTQSPDSLAVSLGARATINCKSSQSVLHSSNNYNYLTWYQQKSGQPPKLLIYWASTRESGVPDRFSGSGSGTDFTLTISSLQAEDVAVYYCQQYYRTPWTFGQGTKVEIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAVSSGWYWFDPWGQGTLVTVSS |
| | | | SEQ ID NO: 26518 | SEQ ID NO: 30524 |
| **iPS:435739** | **21-225_174G7** | NA | GCCATCCAGATGACCCAGTCTCCATCTTCCGTGTCTGCATCTGTAGGAGACAGAGTCACCATCACTTGTCGGGCGAGTCAGGGTATTAGCAACTGGTTAGCCTGGTATCAGCAGAAACCAGGGAAAGCCCCTAACCTCCTGATCCATGCTGCATTCAGTTTGCAAGGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGACTAACAGTTTCCCGTGGACGTTCGGCCAAGGGACCAAGGTGGAAATCAAA | GAGGTGCAGTTGTTGGAGTCTGGGGGAGGCTTGGTACAGCCTGGGGGGTCCCTGAGACTCTCCTGTGCAGCCTCTGGATTCACCTTTAGGAGCTCTGCCATGAGCTGGGTCCGCCAGGCTCCAGGGAAGGGGCTGGAGTGGGTCTCAGTTATTAGTGGTAGTGGTGGTAGCACATTCTACACAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATCGAATAGCAGTGGCTGGTTTGACTACTGGGGCCAGGGAACCCTGGTCACCGTCTCCTCA |
| | | | SEQ ID NO: 26519 | SEQ ID NO: 30525 |

FIGURE 50

| | | | | |
|---|---|---|---|---|
| | | AA | AIQMTQSPSSVSASVGDRVTITCRASQGISNWLAWYQQKPGKAPNLLIHAAFSLQGGVPSRFSGSGSGTDFTLTISSLQPEDFATYYCQQTNSFPWTFGQGTKVEIK | EVQLLESGGGLVQPGGSLRLSCAASGFTFRSSAMSWVRQAPGKGLEWVSVISGSGGSTFYTDSVKGRFTISRDNSKNTLYLQMNSLRAEDTAVYYCAKSNSSGWFDYWGQGTLVTVSS |
| | | | SEQ ID NO: 26520 | SEQ ID NO: 30526 |
| iPS:435741 | 21-225_174G10 | NA | GACATCCAGATGACCCAGTCTCCATCCTCCCTGTCTGCATCTGTAGGAGACAGAGTCACCATCACTTGCCGGGCAAGTCAGGGCATTAGAGATGATTTAGGCTGGTATCAACAGAAACCAGGGAAAGTCCCTAAGCGCCTGATATATGCTGCATCCAGTTTGCAAAGTGGGGTCCCATCAAGGTTCAGCGGCAGTGGATCTGGGACAGAATTCACTCTCACAATCAGCAGCCTACAGCCTGAAGATTTTGCAACTTATTACTGTCTTCAGCATCATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAATTGAACAGCCTGAGAGCCGAGGACACGGCTGTGTATTACTGTGCGAGAGAAAGGTATAGCAGTGGCTGGTACGACTACGGTATGGACGTCTGGGGCCAAGGGACCACGGTCACCGTCTCCTCA |
| | | | SEQ ID NO: 26521 | SEQ ID NO: 30527 |
| | | AA | DIQMTQSPSSLSASVGDRVTITCRASQGIRDDLGWYQQKPGKVPKRLIYAASSLQSGVPSRFSGSGSGTEFTLTISSLQPEDFATYYCLQHHSYPRTFGQGTKVEIK | QVQLVESGGGVVQPGRSLRLSCAASGFTFSDYVMHWVRQAPGKGLEWVAVIWYDGSNKYYADSVKGRFTISRDNSKNTLYLQLNSLRAEDTAVYYCARERYSSGWYDYGMDVWGQGTTVTVSS |
| | | | SEQ ID NO: 26522 | SEQ ID NO: 30528 |

FIGURE 50

| | | | | |
|---|---|---|---|---|
| **iPS:435743** | **21-225_175G1** | NA | GACATCCAGATGACCCAGTCTCCATCCTCCCTGTCTGCATCTGTAGGAGACAGAGTCACCATCACTTGCCGGGCAAGTCAGGGCATTAGAACTGATTTAGGCTGGTATCAGCAGAAACCAGGGAAAGCCCCTAAGCGCCTGATCTATACTGCATCCAGTTTGCAAAGTGGGGTCCCATCAAGGTTCAGCGGCAGTGGATCTGGGACAGAATTCACTCTCACGATCAGCAGCCTGCAGCCTGAAGATTTTGCAACTTATTACTGTCTACAGCATTATAGTTACCCGCTCACTTTCGGCGGAGGGACCAAGGTGGAGATCAAG | CAGGTGCAACTGGTGGAGTCTGGGGGAGGCGTGGTCCAGCCTGGGGGGTCCCTGAGACTCTCCTGTGCAGCGTCTGGCTTCACCTTCAGTACCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATATCCAGAGACAATTCCAAGAACACGCTGTATGTGCAAATGAACAGCCTGAGAGCCGAGGACACGGCTGTGTATTACTGTGCGAGAGATCCCTTACGTGGATACAATGACCCGGTTATGGACTACTGGGGCCAGGGAACCCTGGTCACCGTCTCCTCA |
| | | | SEQ ID NO: 26523 | SEQ ID NO: 30529 |
| | | AA | DIQMTQSPSSLSASVGDRVTITCRASQGIRTDLGWYQQKPGKAPKRLIYTASSLQSGVPSRFSGSGSGTEFTLTISSLQPEDFATYYCLQHYSYPLTFGGGTKVEIK | QVQLVESGGGVVQPGGSLRLSCAASGFTFSTYGMHWVRQAPGKGLEWVAVIWYDGSNKYYADSVKGRFTISRDNSKNTLYVQMNSLRAEDTAVYYCARDPLRGYNDPVMDYWGQGTLVTVSS |
| | | | SEQ ID NO: 26524 | SEQ ID NO: 30530 |
| **iPS:435745** | **21-225_175G3** | NA | GACATCCAGATGACCCAGTCTCCATCCTCACTGTCTGCATCTGTAGGAGACAGAGTCACCATCACTTGTCGGGCGAGTCAGGACATTAGCAATGATTTAGCCTGGTTTCAGCAGAAACCAGGGAAAGCCCCTAAGTCCCTGATCTTTTCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAATCTGATAGTTACCCTCTCACTTTCGGCGGAGGGACCAAGGTTGAGATCAAA | CAGGTACAGGTGGTGCAGTCTGGGGCTGAGGTGAAGAAGCCTGGGGCCTCAGTGAAGGTCTCCTGCAAGGCTTCTGGATACACCTTCACCGGCTACTTTATGCACTGGGTGCGACAGGCCCCTGGACAAGGGCTTGAATGGATGGATGGATCAAGCCTAAAAGTGGTGGCACAAACTGTGCACAGAGGTTTCAGGGCAGGGTCACCATGACCAGGGACACGTCCATCACCACAGCCTACATGGAACTGAGCAGGCTGCGATCTGACGACACGGCCGTGTATTATTGTGTGAGAGGGGGGACTACGGTGACTACGTGGGGGGTCTTTGACTACTGGGGCCAGGGAACCATGGTCACCGTCTCCTCA |
| | | | SEQ ID NO: 26525 | SEQ ID NO: 30531 |

FIGURE 50

| | | | | |
|---|---|---|---|---|
| | | AA | DIQMTQSPSSLSASVGDRVTITCRASQDISNDLAWFQQKPGKAPKSLIFSASSLQSGVPSKFSGSGSGTDFTLTISSLQPEDFATYYCQQSDSYPLTFGGGTKVEIK | QVQVVQSGAEVKKPGASVKVSCKASGYTFTGYFMHWVRQAPGQGLEWMGWIKPKSGGTNCAQRFQGRVTMTRDTSITTAYMELSRLRSDDTAVYYCVRGGTTVTTWGVFDYWGQGTMVTVSS |
| | | | SEQ ID NO: 26526 | SEQ ID NO: 30532 |
| iPS:435747 | 21-225_175C4 | NA | GACATCCAGATGACCCAGTCTCCATCCTCACTGTCTGCATCTGTAGGAGACAGAGTCACCATCACTTGTCGGGCGAGTCAGGGCATTGGGAATTATTTAGCCTGGTTTCAGCAGAAACCAGGGAAAGCCCCTAAGTCCCTGATCTATGCTGCATCCGGTTTGCAAAGTGGGTTCCCATCAAAATTCAGCGGCAGTGGATCTGGGACAGATTTCACTCTCACCATCAGCAGCCTACAGCCTGAAGATTTTGCAACTTATTACTGCCAACAGTATTATAGTTACCCATTCACTTTCGGCCCTGGGACCAAAGTGGATATCAAA | GAGGTGCAGCTGTTGGAGTCTGGGGGAGGCTTGGTGCAGCCTGGGGGGTCCCTGAGACTCTCCTGTGCAGCCTCTGCATTCACCTTTAGCAGCTATGTCATGAGCTGGGTCCGCCAGGCTCCAGGGAAGGGGCTGGAGTGGGTCTCTGCTATTAGTGGTAGTGGTGATAGAACATACTACGCAGACTCCGTGAAGGGCCGGTTCACCATCTCCAGAGACGATTCCAATACCACGCTGTATCTGCAAATGAACAGCCTGAGAGCCGAGGACACGGCCGTATATTACTGTGCGAGAACAGCGGGCTTTGACTACTGGGGCCAGGGAACCCTGGTCACCGTCTCCTCA |
| | | | SEQ ID NO: 26527 | SEQ ID NO: 30533 |
| | | AA | DIQMTQSPSSLSASVGDRVTITCRASQGIGNYLAWFQQKPGKAPKSLIYAASGLQSGFPSKFSGSGSGTDFTLTISSLQPEDFATYYCQQYYSYPFTFGPGTKVDIK | EVQLLESGGGLVQPGGSLRLSCAASAFTFSSYVMSWVRQAPGKGLEWVSAISGSGDRTYYADSVKGRFTISRDDSNTTLYLQMNSLRAEDTAVYYCARTAGFDYWGQGTLVTVSS |
| | | | SEQ ID NO: 26528 | SEQ ID NO: 30534 |

FIGURE 50

| | | | | |
|---|---|---|---|---|
| **iPS:435749** | **21-225_175C10** | NA | GACATCCAGATGACCCAGTCTCCATCTTCCGTGTCTGCATCTGTAGGAGACAGAGTCACCATCACTTGTCGGGCGAGTCAGGGTATTACCGACTGGTTAGCCTGGTATCAGCAGAAACCAGGGAAAGCCCCTAAGCTCCTGATCTATGCTGCATCCAGTTTGCAAAGTGGGGTCCCATCAAGGTTCAGCGGCAGTGGATCTGGGACAGATTTCACTCTCACCTTCAGCAGCCTGCAGCCTGACGATTTTGCAACTTACTATTGTCAACAGACTAACAGTTTCCCGTGGACGTTCGGCCAAGGGACCAAGGTGGAAATCAAA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCGTCTATTAGTGGTCGTGGTGGTAGCACGTTCTACGCAGACTCCGTGAAGGGCCGGTTCACCGTCTCCAGAGACAATTCCAAGAACACGCTGTATCTGCAAATGAACAGCCTGAGAGCCGAGGACACGGCCGTATATTACTGTGCGAAATCGAATAGCAGTGGCTGGTTTGACTACTGGGGCCAGGGAACCCTGGTCACCGTCTCCTCA |
| | | | SEQ ID NO: 26529 | SEQ ID NO: 30535 |
| | | AA | DIQMTQSPSSVSASVGDRVTITCRASQGITDWLAWYQQKPGKAPKLLIYAASSLQSGVPSRFSGSGSGTDFTLTFSSLQPDDFATYYCQQTNSFPWTFGQGTKVEIK | EVQLLESGGGLVQPGGSLRLSCAASGFTFSSYAMSWVRQAPGKGLEWVSSISGRGGSTFYADSVKGRFTVSRDNSKNTLYLQMNSLRAEDTAVYYCAKSNSSGWFDYWGQGTLVTVSS |
| | | | SEQ ID NO: 26530 | SEQ ID NO: 30536 |
| **iPS:435751** | **21-225_175D10** | NA | GACATCGTGATGACCCAGTCTCCAGACTCCCTGGCTGTGTCTCTGGGCGAGAGGGCCACCATCAACTGCAAGTCCAGCCAGAGTGTTTTATACAGCTCCAACAATAACAACTACTTAGCTTGGTACCAGCAGAAACCAGGACAGCCTCCTAACCTGCTCATTTACTGGACATCTACCCGGGAATCCGGGGTCCCTGACCGATTCAGTGGCAGCGGGTCTGGGACAAATTTCACTCTCACCATCAGCAGCCTGCAGGCTGAAGATGTGGCAGTTTATTACTGTCAGCAATATTATAGTACTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGTTACACCTTCACCAATTATGATCTCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGTTTACATGGAGCTGAGCAGCCTGAGATCTGAGGACACGGCCGTGTATTACTGTGCGTATAGCAGTGGCTGGTACTACTTTGACTACTGGGGCCAGGGAACCCTGGTCACCGTCTCCTCA |
| | | | SEQ ID NO: 26531 | SEQ ID NO: 30537 |

FIGURE 50

| | | | | |
|---|---|---|---|---|
| | | AA | DIVMTQSPDSLAVSLGERATINCKSSQSVLYSSNNNNYLAWYQQKPGQPPNLLIYWTSTRESGVPDRFSGSGSGTNFTLTISSLQAEDVAVYYCQQYYSTPPTFGQGTKVEIK | QVQLVQSGAEVKKPGASVKVSCKASGYTFTNYDLNWVRQATGQGLEWMGWMHPNSGNTGYAQKFQGRVTMTRNTSISTVYMELSSLRSEDTAVYYCAYSSGWYYFDYWGQGTLVTVSS |
| | | | SEQ ID NO: 26532 | SEQ ID NO: 30538 |
| iPS:435753 | 21-225_175G10 | NA | GACATCCAGATGACCCAGTCTCCATCCTCCCTGTCTGCATCTGTAGGAGACAGAGTCATCATCACTTGCCGGGCAAGTCAGACCATTGGCAACTATTTAAATTGGTATCAGCAGAAACCAGGGAGAGCCCCTAAGCTCCTGATCTATGCTGCATCCAGTTTGCACAGTGGGGTCCCATCAGGGTTCAGTGGCAGTGGATCTGGGACAGATTTCACTCTCACCATCAGCAGTCTGCAACCTGAAGATTTTGCAACTTACTTCTGTCAACAGAGTTACAGAACCCCTCAGTGGACGTTCGGCCAAGGGACCAAGGTGGAAATCAAA | GAGGTGCAGTTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGACTCCAGGGAAGGGGCTGGAGTGGGTCTCAATTATTAGTGGTAGTGGTGGTAACACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAAAGGGATACCTGGAACGGTTGGGATGCTTTTGATATCTGGGGCCAAGGGACAATGGTCACCGTCTCTTTA |
| | | | SEQ ID NO: 26533 | SEQ ID NO: 30539 |
| | | AA | DIQMTQSPSSLSASVGDRVIITCRASQTIGNYLNWYQQKPGRAPKLLIYAASSLHSGVPSGFSGSGSGTDFTLTISSLQPEDFATYFCQQSYRTPQWTFGQGTKVEIK | EVQLLESGGGLVQPGGSLRLSCAASGFTFSSYAMSWVRQTPGKGLEWVSIISGSGGNTYYADSVKGRFTISRDNSKNTLYLQMNSLRAEDTAVYYCAKRDTWNGWDAFDIWGQGTMVTVSL |
| | | | SEQ ID NO: 26534 | SEQ ID NO: 30540 |

FIGURE 50

| | | | | |
|---|---|---|---|---|
| **iPS:435755** | **21-225_176H4** | NA | GATATTGTGATGACCCAGACTCCACTCTCTCTGTCCGTCACCCCTGGACAGCCGGCCTCCATCTCCTGCAAGTCTAGTCAGAGCCTCCTGCATGGTGATGGAAAGACCTATTTGTATTGGTACCTGCAGAAGCCAGGCCAGCCTCCACAGCTCCTGATCTATGAAGTTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGATTTATTACTGCATGCAAAGTATACAGATTCCGTGGACGTTCGGCCAAGGGACCAGGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAATTATATGGTATGATGGAAGTTATAAATACTATGCAGACTCCGTGAAGGGCCGATTCACCATCTCCAGAGACAATTCCAAGAACACGCTGTATCTGCAAATGAGCAGCCTGAGAGCCGAGGACACGGCTGTGTATTACTGTGCGAGAGATGCCCACGATTTTTGGAGTGGTTACTTTGCCTACTGGGGCCAGGGAGCCCTGGTCACCGTCTCCTCA |
| | | | SEQ ID NO: 26535 | SEQ ID NO: 30541 |
| | | AA | DIVMTQTPLSLSVTPGQPASISCKSSQSLLHGDGKTYLYWYLQKPGQPPQLLIYEVSNRFSGVPDRFSGSGSGTDFTLKISRVEAEDVGIYYCMQSIQIPWTFGQGTRVEIK | QVQLVESGGGVVQPGRSLRLSCAASGFTFSSYGMHWVRQAPGKGLEWVAIIWYDGSYKYYADSVKGRFTISRDNSKNTLYLQMSSLRAEDTAVYYCARDAHDFWSGYFAYWGQGALVTVSS |
| | | | SEQ ID NO: 26536 | SEQ ID NO: 30542 |
| **iPS:435759** | **21-225_176E6** | NA | GACATTGTGATGACTCAGTCTCCACTCTCCCTGCCCGTCACCCCTGGAGAGCCGGCCTCCATCTCCTGCAGGTCTAGTCAGAGCCTCCTGCATAATAATGGATACAAGTATTTGGATTGGTACCTGCAGAAGCCAGGGCAGTCTCCACAGCTCCTGATCTATTTGGGTTCTAATCGGGCCTCCGGGGTCCCTGACAGGTTCAGTGCCAGTGGATCAGGCACAGATTTTACACTGAAAATCAGCAGAGTGGAGGCTGAGGATGTTGGGGTTTATTACTGCATGCAAGTTCTACAAACTCCGTGGACGTTCGGCCAAGGGACCAAGGTGGAAATCAAA | GAGGTGCAACTGGTGGAGTCTGGGGGAGGCTTGGTACAGCCTGGGGGGTCCCTGAGACTCTCCTGTGCAGCCTCAGGATTCACCTTCAGTGGCCATAGTATGAACTGGGTCCGCCAGGCTCCAGGGAAGGGGCTGGAGTGGGTTGCATACATTAGCATTAGTGGTAGTACCATAAAGTACGCAGACTCTGTGAAGGGCCGATTCATCATCTCCAGAGACAATGCCAGGGATTCACTGTATCTGCAAATGAACAGCCTGAGAGACGAGGACACGGCTGTGTATTACTGTGCGAGAAGGGGGATTACTGTGGTTCGGAATGAGGACGGTTTGGACGTCTGGGGCCAAGGGACCACGGTCACCGTCTCCTCA |
| | | | SEQ ID NO: 26537 | SEQ ID NO: 30543 |

FIGURE 50

| | | | | |
|---|---|---|---|---|
| | | AA | DIVMTQSPLSLPVTPGEPASISCRSSQSLLHNNGYKYLDWYLQKPGQSPQLLIYLGSNRASGVPDRFSASGSGTDFTLKISRVEAEDVGVYYCMQVLQTPWTFGQGTKVEIK | EVQLVESGGGLVQPGGSLRLSCAASGFTFSGHSMNWVRQAPGKGLEWVAYISISGSTIKYADSVKGRFIISRDNARDSLYLQMNSLRDEDTAVYYCARRGITVVRNEDGLDVWGQGTTVTVSS |
| | | | SEQ ID NO: 26538 | SEQ ID NO: 30544 |
| iPS:435761 | 21-225_176B11 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTTTCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACACTCAGCAGCCTGCAGCCTGAAGATTTTGCAACTTATTATTGTCTACAGCATTATAGTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTACCTATGGCATGCACTGGGTCCGCCAGGCTCCAGGCAAGGGCCTGGAGTGGGTGTCAATTATATGGTATGATGGAACTAATAAATACTATGCAGACTCCGTGAAGGGCCGATTCACCATCTCCAGAGACAATTCCAAGAACACGCTGTTTCTGCAACTGAACAGCCTGAGAGCCGAGGACACGGCTGTCTATTACTGTGCGCGAGATCCCTTACGTGGATACAATGACCCGGTTTTGGACTACTGGGGCCAGGGAACCCTGGTCACCGTCTCCTCA |
| | | | SEQ ID NO: 26539 | SEQ ID NO: 30545 |
| | | AA | DIQMTQSPSSLSASVGDRVTITCRASQGIRNDLGWFQQKPGKAPKRLIYAASSLQSGVPSRFSGSGSGTEFTLTLSSLQPEDFATYYCLQHYSYPLTFGGGTKVEIK | QVQLVESGGGVVQPGRSLRLSCAASGFTFSTYGMHWVRQAPGKGLEWVSIIWYDGTNKYYADSVKGRFTISRDNSKNTLFLQLNSLRAEDTAVYYCARDPLRGYNDPVLDYWGQGTLVTVSS |
| | | | SEQ ID NO: 26540 | SEQ ID NO: 30546 |

FIGURE 50

| | | | | |
|---|---|---|---|---|
| iPS:435763 | 21-225_176H12 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAACCGCCTGATCTATGCTGCATCCAGTTTGCAAAGTGGGGTCCCATCACGGTTCAGCGGCAGTGGATCTGGGACAGAATTCACTCTCACAATCAGCAGCCTGCAGCGTGAAGATTTTGCAACTTATTACGGTCTACAGCATAATAGTTACCCTCGCAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGTCATGCACTGGGTCCGCCAGGCTCCAGGCAAGGGGCTGGAGTGGGTGGCAGTTATATGGTATGATGGAAGTAATAAATACTATGCCGACTCCGTGAAGGGCCGATTCACCATCTCCAGAGACAATTCCAAGAACACGCTGTATCTGCAAATGAACAGCCTGAGAGCCGAGGACACGGCTGTATATTACTGTGCGAGAGAAAAGTATAGCAGCAACTGGTACGACTACGGTATGGACGTCTGGGGCCAAGGGACCACGGTCACCGTCTCCTCA |
| | | | SEQ ID NO: 26541 | SEQ ID NO: 30547 |
| | | AA | DIQMTQSPSSLSASVGDRVTITCRASQGIRNDLGWYQQKPGKAPNRLIYAASSLQSGVPSRFSGSGSGTEFTLTISSLQREDFATYYGLQHNSYPRSFGQGTKLEIK | QVQLVESGGGVVQPGRSLRLSCAASGFTFSDYVMHWVRQAPGKGLEWVAVIWYDGSNKYYADSVKGRFTISRDNSKNTLYLQMNSLRAEDTAVYYCAREKYSSNWYDYGMDVWGQGTTVTVSS |
| | | | SEQ ID NO: 26542 | SEQ ID NO: 30548 |
| iPS:435765 | 21-225_177D3 | NA | GACATCCAGATGTCCCAGTCTCCATCCTCACTGTCTGCTTCTGTAGGAGACAGAGTCACCATCACTTGTCGGGCGAGTCAGGGCATTACCAATTATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATAATAGTTACCCATTCACTTTCGGCCCTGGGACCAAAGTGGATATCAAA | GAGGTGCAGTTGTTGGAGTCTGGGGGAGGCTTGGTACAGCCTGGGGGGTCCCTGAGACTCTCCTGTGCAGCCTCTGGATTCACCTTTAGCAGCTATGTCATGAACTGGGTCCGCCAGGCTCCAGGGAAGGGGCTGGAGTGGGTCTCAGGTATGAGCGGTAGTGGTGGTAGAACATACTACGCAGACTCCGTGAAGGACCGGTTCACCATCTCCAGAGACAATTCCAAGAACACGCTGTCTCTGCAAATGAACAGCCTGAGGGCCGAGGACACGGCCGTATATTACTGTGCGAGAGTGACTTTCTTTGACTATTGGGGCCAGGGAACCCTGGTCACCGTCTCCTCA |
| | | | SEQ ID NO: 26543 | SEQ ID NO: 30549 |

FIGURE 50

| | | | | |
|---|---|---|---|---|
| | | AA | DIQMSQSPSSLSASVGDRVTITCRASQGITNYLAWFQQKPGKAPKSLIYAASSLQSGVPSKFSGSGSGTDFTLTISSLQPEDFATYYCQQYNSYPFTFGPGTKVDIK | EVQLLESGGGLVQPGGSLRLSCAASGFTFSSYVMNWVRQAPGKGLEWVSGMSGSGGRTYYADSVKDRFTISRDNSKNTLSLQMNSLRAEDTAVYYCARVTFFDYWGQGTLVTVSS |
| | | | SEQ ID NO: 26544 | SEQ ID NO: 30550 |
| **iPS:435767** | **21-225_177B4** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTTCCCTCGCAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGATTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCATAGTCTCCAGAGACAATTCCAAGAACACGCTGTATCTGCAAATGAACAGCCTGAGAGCCGAGGACACGGCTGTGTATTACTGTGCGAGAGAAAAGTATAGCAGCAGCTGGTACGACTACGGTTTGGACGTCTGGGGCCAAGGGACCACGGTCACCGTCTCCTCA |
| | | | SEQ ID NO: 26545 | SEQ ID NO: 30551 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHYSFPRSFGQGTKLEIK | QVQLVESGGGVVQPGRSLRLSCAASGFTFSDYVMHWVRQAPGKGLEWVAVIWYDGSNKYYADSVKGRFIVSRDNSKNTLYLQMNSLRAEDTAVYYCAREKYSSSWYDYGLDVWGQGTTVTVSS |
| | | | SEQ ID NO: 26546 | SEQ ID NO: 30552 |

FIGURE 50

| | | | | |
|---|---|---|---|---|
| **iPS:435769** | **21-225_177B6** | NA | GACATCCAGATGACCCAGTCTCCATCCTCCCTGTCTGCATCTGTAGGAGACAGAGTCACCATCACTTGCCGGGCAAGTCAGGACATTAGCAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGTTCTGGGACAGAATTCACTCTCACAATCAACAGCCTGCAGCCTGAAGATTTTGCAACTTATTACTGTCTACAGCTTAATAGTTACCCATTCACTTTCGGCCCTGGGACCAAAGTGGATATCAAA | GAGGTGCAGCTGTTGGAGTCTGGGGGAGGCTTGGTACAGCCGGGGGGGTCCCGGAGACTCTCCTGTGAAGCCTCTGGATTCACCTTTAGCAGTTATGCCATGAGCTGGGTCCGCCAGGCTCCAGGGAAGGGGCTGGAGTGGGTCTCAGTTATTAGTGGTAGTGGTAGTAACACATACTACGTAGACTCCGTGAAGGGCCGGTTCACCATCTCCAGAGACAATTCCAAGAACACGCTGAATCTGCAAATGAACAGCCTGAGAGCCGAGGACTCGGCCGTATATTACTGTACGAAAGGTTACTATGATAGTAGTGGTTATTACTACCCTTTTGACTTCTGGGGCCAGGGAACCCTGGTCACCGTCTCCTCA |
| | | | SEQ ID NO: 26547 | SEQ ID NO: 30553 |
| | | AA | DIQMTQSPSSLSASVGDRVTITCRASQDISNDLGWYQQKPGKAPKRLIYAASSLQSGVPSRFSGSGSGTEFTLTINSLQPEDFATYYCLQLNSYPFTFGPGTKVDIK | EVQLLESGGGLVQPGGSRRLSCEASGFTFSSYAMSWVRQAPGKGLEWVSVISGSGSNTYYVDSVKGRFTISRDNSKNTLNLQMNSLRAEDSAVYYCTKGYYDSSGYYYPFDFWGQGTLVTVSS |
| | | | SEQ ID NO: 26548 | SEQ ID NO: 30554 |
| **iPS:435771** | **21-225_177B11** | NA | GATATTGTGATGACCCAGACTCCACTCTCTCTGTCCGTCACTCCTGGACAGCCGGCCTCCATCTCCTGCAAGTCTAGTCAGCGCCTCCTGCATGGTGATGGAAAGACCTATTTGTATTGGTACCTGCAGAAGCCAGGCCAGCCTCCACAGATCCTGATCTATGAAGTTTCCAACCGGTTCTCTGGAGTGCCAGATAGGTTCAGTGGCAGCGGGTCAGGGACAGAGTTCACACTTAAAATCAGCCGGGTGGAGGCTGAGGATGTTGGGGTTTATTACTGCATGCAAAGTATACAGATTCCGTGGACGTTCGGCCAAGGGACCAAGGTGGAAATCAAA | CAGGTGCAACTGGTGGAGTCTGGGGGAGGCGTGGTCCAGCCTGGGAGGTCCCTGAGACTCACCTGTGCAGCGTCTGGATTCACCTTCAGTAGCTATGGCATGCACTGGGTCCGCCAGGCTCCAGGCAAGGGGCTGGAGTGGGTGGCAATTATATGGTATGATGGAAGTTATAAATACTATACAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGACTTACGATTTTTGGAGTGGTTATTTTGTCTTCTGGGGCCAGGGAACCCTGGTCACCGTCTCCTCA |
| | | | SEQ ID NO: 26549 | SEQ ID NO: 30555 |

FIGURE 50

| | | | | |
|---|---|---|---|---|
| | | AA | DIVMTQTPLSLSVTPGQPASISCKSSQRLLHGDG KTYLYWYLQKPGQPPQILIYEVSNRFSGVPDRFS GSGSGTEFTLKISRVEAEDVGVYYCMQSIQIPWT FGQGTKVEIK | QVQLVESGGGVVQPGRSLRLTCAASGFTFSSYGMH WVRQAPGKGLEWVAIIWYDGSYKYYTDSVKGRFT ISRDNSKNTLYLQMNSLRAEDTAVYYCARETYDF WSGYFVFWGQGTLVTVSS |
| | | | SEQ ID NO: 26550 | SEQ ID NO: 30556 |
| iPS:435773 | 21-225_177B12 | NA | GACATCGTGATGACCCAGTCTCCAGACTCCCT GGCTGTGTCTCTGGGCGAGAGGGCTACCGTCA ACTGCAAGTCCAGCCAGAGTGTTTTACACAGC TCCAACAATAACAACTACTTAACTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTAGTCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCGTATAGCAGT GGCTGGTACTACTTTGACTTCTGGGGCCAGGGAA CCCTGGTCACCGTCTCCTCA |
| | | | SEQ ID NO: 26551 | SEQ ID NO: 30557 |
| | | AA | DIVMTQSPDSLAVSLGERATVNCKSSQSVLHSSN NNNYLTWYQQKPGQPPKLLIYWASTRESGVPDR FSGSGSGTDFTLTISSLQAEDVAVYYCQQYYSSP PTFGQGTKVEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAYSSGW YYFDFWGQGTLVTVSS |
| | | | SEQ ID NO: 26552 | SEQ ID NO: 30558 |

FIGURE 50

| | | | | |
|---|---|---|---|---|
| **iPS:435775** | **21-225_178A5** | NA | GACATCCAGATGACCCAGTCTCCATCTTCCGTGTCTGCATCTGTAGGAGACAGAGTCACCATCACTTGTCGGGCGAGTCAGGGTATTAGCAACTGGTTAGCCTGGTATCAGCAGAAACCAGGGAAAGCCCCTAAGCTCCTGATCTATGCTGCTTCCAGTTTGCAAAGTGGGGTCCCATCAAGGTTCAACGGCAGTGGATCTGGGACAGATTTCACTCTCACCATCAGCAGCCTGCAGCCTGAAGATTTTGCAACTTACTGTTGTCAACAGGCTAACAGTTTACCGTGGACGTTCGGCCAAGGGACCAAGGTGGAAATCAAA | GAGGTGCACCTGTTGGAGTCTGGGGGAGGCTTGGTACAGACTGGGGGGTCCCTGAGACTCTCCTGTGCAGCCTCTGGATTCACCTTTAGCAGCTATGCCATGACCTGGGTCCGCCAGGCTCCAGGGAAGGGGCTGGAGTGGGTCTCAGTTATTAGTGGTAGTGGTGGTAATACATTCTACGCAGACTCCGTGAAGGGCCGGTTCACCATCTCCAGAGACAATTCCAAGAATACGCTGTATCTGCAAATGAACAGCCTGAGAGCCGAGGACACGGCCGTATATTACTGTGCGCGCCGGGACGGTGACTACTTTGACTACTGGGGCCAGGGAACCCTGGTCACCGTCTCCTCA |
| | | | SEQ ID NO: 26553 | SEQ ID NO: 30559 |
| | | AA | DIQMTQSPSSVSASVGDRVTITCRASQGISNWLAWYQQKPGKAPKLLIYAASSLQSGVPSRFNGSGSGTDFTLTISSLQPEDFATYCCQQANSLPWTFGQGTKVEIK | EVHLLESGGGLVQTGGSLRLSCAASGFTFSSYAMTWVRQAPGKGLEWVSVISGSGGNTFYADSVKGRFTISRDNSKNTLYLQMNSLRAEDTAVYYCARRDGDYFDYWGQGTLVTVSS |
| | | | SEQ ID NO: 26554 | SEQ ID NO: 30560 |
| **iPS:435777** | **21-225_178F7** | NA | GACATCCAGATGACCCAGTCTCCATCTTCCGTGTCTGCATCTGTAGGAGACAGAGTCACCATCACTTGTCGGGCGAGTCAGGATATTACTGACTGGTTAGCCTGGTATCAGCAGAAACCAGGGAAAGCCCCTAAACTCCTGATCTCTGCTGCATCCAGTTTGCAGAGTGGGGTCCCATCAAGGTTCAGCGGCAGTGGATCTGGGACAGATTTCACTCTCGCCATCAGCAGCCTGCAGCCTGAAGATTTTGCAACTTACTATTGTCAACAGGCTAACAGTTTACCGTGGACGTTCGGCCAAGGGACCAAGGTGGAAATCAAA | GAGGTGCACCTGTTGGAGTCTGGGGGAGGCTTGGTACAGACTGGGGGGTCCCTGAGACTCTCCTGTGCAGCCTCTGGATTCACCTTTAGCAGCTATGCCATGACCTGGGTCCGCCAGGCTCCAGGGAAGGGGCTGGAGTGGGTCTCAGTTATTAGTGGTAGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGCGCCGGTACGGTGACTACTTTGACTACTGGGGCCAGGGAACCCTGGTCACCGTCTCCTCA |
| | | | SEQ ID NO: 26555 | SEQ ID NO: 30561 |

FIGURE 50

| | | | | |
|---|---|---|---|---|
| | | AA | DIQMTQSPSSVSASVGDRVTITCRASQDITDWLAWYQQKPGKAPKLLISAASSLQSGVPSRFSGSGSGTDFTLAISSLQPEDFATYYCQQANSLPWTFGQGTKVEIK | EVHLLESGGGLVQTGGSLRLSCAASGFTFSSYAMTWVRQAPGKGLEWVSVISGSGGNTFYADSVKGRFTISRDNSKNTLYLQMNSLRAEDTAVYYCARRYGDYFDYWGQGTLVTVSS |
| | | | SEQ ID NO: 26556 | SEQ ID NO: 30562 |
| **iPS:435779** | **21-225_178B10** | NA | GACATCCAGATGACCCAGTCTCCATCCTCCCTGTCTGCATCTGTCGGAGACAGAGTCACCATCACTTGCCGGGCAAGTCAGGGCATTAGAAATGATTTAGGCTGGTATCAGCAGAAACCAGGGAAAGCCCCTAAACACCTGATCTATGCTGCATCCAGTTTGCAAAATGGGGTCCCATCAAGGTTCAGCGGCAGTGGCTCTGGGACAGAATTCACTCTCACAATCAGCAGCCTGCAGCCTGAAGATTTTGCCACTTATTACTGTCTACACCATTATAGTTTCCCGCTCACTTTCGGCGGAGGGACCAAGGTGGAGATCAAA | CAGGTGCAGCTGGTGGAGTCGGGGGGAGGCGTGGTCCAGCCTGGGAGGTCCCTGAGACTCTCCTGTGTAGCGTCTGGATTCACCTCCAGTACCTATGGCATGCACTGGGTCCGCCAGGCTCCAGGCAAGGGGCTGGAATGGATGGCAATTATATGGTATGATGGAACTAATAAATACTATGCAGACTCCGTGAAGGGCCGATTCACCATCTCCAGAGACAATTCCAAGAACACGCTGTATCTGCAACTGAACAGCCTGAGAGCCGAGGACACGGCTGTGTATTACTGTGCGCGAGATCCCTTACGTGGATACAATGACCCGGTTATGGACTACTGGGGCCAGGGAACCCTGGTCACCGTCTCCTCA |
| | | | SEQ ID NO: 26557 | SEQ ID NO: 30563 |
| | | AA | DIQMTQSPSSLSASVGDRVTITCRASQGIRNDLGWYQQKPGKAPKHLIYAASSLQNGVPSRFSGSGSGTEFTLTISSLQPEDFATYYCLHHYSFPLTFGGGTKVEIK | QVQLVESGGGVVQPGRSLRLSCVASGFTSSTYGMHWVRQAPGKGLEWMAIIWYDGTNKYYADSVKGRFTISRDNSKNTLYLQLNSLRAEDTAVYYCARDPLRGYNDPVMDYWGQGTLVTVSS |
| | | | SEQ ID NO: 26558 | SEQ ID NO: 30564 |

FIGURE 50

| | | | | |
|---|---|---|---|---|
| **iPS:435781** | **21-225_178G10** | NA | CATATTGTGATGACCCAGACTCCACTCTCTCTGTCCGTCACCCCTGGACAGCCGGCCTCCATCTCCTGCAAGTCTAGTCAGAGCCTCCTGCATGGTGATGGAAAGACCTATTTGTATTGGTACCTGCAGAAGCCAGGCCAGCCTCCACAGCTCCTGATCTATGAAGTTTCCAACCGGTTTTCTGGAGTGCCAGACAGACTCAGTGGCGGCGGGTCAGGGACAGATTTCACACTGAAAATCAGCCGGGTGGAGGCTGAGGATGTTGGCATTTATTACTGCATGCAAAGTATACAGGTTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTACCTATGGCATGCACTGGGTCCGCCAGGCTCCAGGCAAGGGGCTGGAGTGGGTGGCAGTTATATGGTATGATGGAAGTAATAAATACTACGCAGACTCCGTGAAGGGCCGATTCACCATCTCCAGAGACAATTTCAAGAACACGCTGTATCTGCAAATGAACAGCCTGAGAGCCGAGGACACGGCTGTTTATTACTGTGCGAGAGAACGGTACGATTTTTGGAGTGGTCATTTTGACTACTGGGGCCAGGGAACCCTGGTCACCGTCTCCTCA |
| | | | SEQ ID NO: 26559 | SEQ ID NO: 30565 |
| | | AA | HIVMTQTPLSLSVTPGQPASISCKSSQSLLHGDGKTYLYWYLQKPGQPPQLLIYEVSNRFSGVPDRLSGGGSGTDFTLKISRVEAEDVGIYYCMQSIQVPWTFGQGTKVEIK | QVQLVESGGGVVQPGRSLRLSCAASGFTFSTYGMHWVRQAPGKGLEWVAVIWYDGSNKYYADSVKGRFTISRDNFKNTLYLQMNSLRAEDTAVYYCARERYDFWSGHFDYWGQGTLVTVSS |
| | | | SEQ ID NO: 26560 | SEQ ID NO: 30566 |
| **iPS:435783** | **21-225_179G1** | NA | GACATCCAGATGACCCAGTCTCCATCTTCCGTGTCTGCATCTGTAGGAGACAGAGTCACCATCACTTGTCGGGCGAGTCAGGATATTAGCGACTGGTTAGCCTGGTATCAGCAAAAATCAGGGAAAGCCCCTAAACTCCTGATCTCTGCTGCATCCAGTTTGCAAAGTGGGGTCCCATCAAGGTTCGGCGGCAGTGGATCTGGGACAGATTTCACTCTCACCATCAGCAGCCTGCAGCCTGAAGATTTTGCAACTTACTATTGTCAACAGGCTAACAGTTTACCGTGGACGTTCGGCCAAGGGACCAAGGTGGAAATCAAA | GAGGTGCACCTGTTGGAGTCGGGGGGAGGCTTGGTACAGACTGGGGGGTCCCTGAGACTCTCCTGTGCAGCCTCTGGATTCACCTTTAGCAGCTATGCCATGACCTGGGTCCGCCAGGCTCCAGGGAAGGGGCTGGAGTGGGTCTCAGTTATTAGTGGTTTTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGCGCCGGTACGGTGACTACTTTGACTACTGGGGCCAGGGAACCCTGGTCACCGTCTCCTCA |
| | | | SEQ ID NO: 26561 | SEQ ID NO: 30567 |

FIGURE 50

| | | | | |
|---|---|---|---|---|
| | | AA | DIQMTQSPSSVSASVGDRVTITCRASQDISDWLAWYQQKSGKAPKLLISAASSLQSGVPSRFGGSGSGTDFTLTISSLQPEDFATYYCQQANSLPWTFGQGTKVEIK | EVHLLESGGGLVQTGGSLRLSCAASGFTFSSYAMTWVRQAPGKGLEWVSVISGFGGNTFYADSVKGRFTISRDNSKNTLYLQMNSLRAEDTAVYYCARRYGDYFDYWGQGTLVTVSS |
| | | | SEQ ID NO: 26562 | SEQ ID NO: 30568 |
| iPS:435785 | 21-225_179C2 | NA | GATATTGTGATGACCCAGACTCCACTCTCTCTGTCCGTCACCCCTGGACAGCCGGCCTCCATCTCCTGCAAATCTAGTCAGAGCCTCCTGCATAGTGAGGGAAAGACCTACTTGTATTGGTACCTGCAGAAGCCAGGCCAGCCTCCACAGCTCCTGATCTATGAGGTTTCCCACCGGTTCTCTGGAGTGCCAGATAGGTTCAGTGGCAGCGGGTCAGGGACAGATTTCACACTGAAAATCAGCCGGGTGGAGGCTGAGGATGTTGGGGTTTATTACTGCATGCAAAGTATACAGGTTCTCACTTTCGGCGGAGGGACCAAGGTGGAGATCAAA | CAGGTGCAGCTGGTGGAGTCGGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCACTTATATTTTATGATGGAAGTAATAAATACTATGCAGACTCCGTGAAGGGCCGATTCACCATCTCCAGAGACAATTCCAAGAACACGCTGTTTCTGCAAATGAACAGCCTGAGAGCCGAGGACACGGCTGTGTATTACTGTGCGAGACGGTATAGCGGCAGCTGGTCCGGTGGTATGGACGTCTGGGGCCAAGGGACCACGGTCACCGTCTCCTCA |
| | | | SEQ ID NO: 26563 | SEQ ID NO: 30569 |
| | | AA | DIVMTQTPLSLSVTPGQPASISCKSSQSLLHSEGKTYLYWYLQKPGQPPQLLIYEVSHRFSGVPDRFSGSGSGTDFTLKISRVEAEDVGVYYCMQSIQVLTFGGGTKVEIK | QVQLVESGGGVVQPGRSLRLSCAASGFTFSSYGMHWVRQAPGKGLEWVALIFYDGSNKYYADSVKGRFTISRDNSKNTLFLQMNSLRAEDTAVYYCARRYSGSWSGGMDVWGQGTTVTVSS |
| | | | SEQ ID NO: 26564 | SEQ ID NO: 30570 |

FIGURE 50

| | | | | |
|---|---|---|---|---|
| **iPS:435787** | **21-225_180A3** | NA | GACATCCAGATGACCCAGTCTCCATCTTCCGTGTCTGCATCTGTAGGAGACAGAGTCACCATCACTTGTCGGGCGAGTCAGGATATTACCAGCTGGTTAGCCTGGTATCAGCAGAAACCAGGGAAAGCCCCTAAGCTCCTGATCTATGCTGCATCCAGTTTGCAAAGTGGGGTCCCATCAAGGTTCAGCGGCAGTGGATCTGGGACAGATTTCATTCTCACCATCAGCAGCCTGCAGCCTGAAGATTTTGCATTTTACCATTGTCAACAGGCTAACAGTATCCCATTCACTTTCGGCCCTGGGACCAAAGTGGACATCAAC | GAGGTGCAGCTGTTGGAGTCTGGGGGAGGCTTGGAACAGCCTGGGGGGTCCCTGAGACTCTCCTGTGCAGCCTCTGGATTCACCTTTAGCAGCTTTGCCATGAACTGGGTCCGCCAGGCTCCAGGGAAGGGGCTGGAGTGGGTCTCAGTTATTAGCGGTCGCGGTGGTAACACATTCTACGCAGACTCCGTGAAGGGCCGGTTCACCATCTCCAGAGACAATTCCAAGAACACGCTGTTTCTGCAAATGAACAGCCTGAGAGCCGAGGACACGGCCGTATATTTCTGTGCGAAACGGACTGGGGATGATGTTTTTGATGTCTGGGGCCAAGGGACAATGGTCACCGTCTCTTCA |
| | | | SEQ ID NO: 26565 | SEQ ID NO: 30571 |
| | | AA | DIQMTQSPSSVSASVGDRVTITCRASQDITSWLAWYQQKPGKAPKLLIYAASSLQSGVPSRFSGSGSGTDFILTISSLQPEDFAFYHCQQANSIPFTFGPGTKVDIN | EVQLLESGGGLEQPGGSLRLSCAASGFTFSSFAMNWVRQAPGKGLEWVSVISGRGGNTFYADSVKGRFTISRDNSKNTLFLQMNSLRAEDTAVYFCAKRTGDDVFDVWGQGTMVTVSS |
| | | | SEQ ID NO: 26566 | SEQ ID NO: 30572 |
| **iPS:435789** | **21-225_180C4** | NA | GATATTGTGATGACCCAGACTCCACTCTCTCTGTCCGTCACCCCTGGACAGCCGGCCTCCATCTCCTGCAAGTCTAGTCAGAGCCTCCTGCATGGTGATGGAAAGACCTATTTGTATTGGTACCTGCAGAAGCCAGGCCAGCCTCCACAGCTCCTGATCTATGCAACTTCCAACCGGTTCCCTGGAGTGTCAGATAGGTTCAGTGGCAGCGGGTCAGGTACAGACTTCACACTGAAAATCAGCCGGGTGGAGGCTGAGGATGTTGGGGTTTATTACTGCATGCAAAGTATACAGGTTCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGCCTATGGCATGCACTGGGTCCGCCAGGCTCCAGGCAAGGGGCTGGAGTGGGTGACAATTATTTGGTATGATGGAAGTTATAAATACTATGCAGACTCCGTGAAGGGCCGATTCGCCATCTCCAGAGACAATTCCAAGAATACGCTGTATCTGCAAATGAACAGCCTGAGAGCCGAGGACACGGCTGTGTATTACTGTGCGAGAACCGGTGTGGATCCCTGGGACTACTACAACGGAATGGACGTCTGGGGCCAAGGGACCACGGTCACCGTCTCCTCA |
| | | | SEQ ID NO: 26567 | SEQ ID NO: 30573 |

FIGURE 50

| | | | | |
|---|---|---|---|---|
| | | AA | DIVMTQTPLSLSVTPGQPASISCKSSQSLLHGDGKTYLYWYLQKPGQPPQLLIYATSNRFPGVSDRFSGSGSGTDFTLKISRVEAEDVGVYYCMQSIQVPWTFGQGTKVEIK | QVQLVESGGGVVQPGRSLRLSCAASGFTFSAYGMHWVRQAPGKGLEWVTIIWYDGSYKYYADSVKGRFAISRDNSKNTLYLQMNSLRAEDTAVYYCARTGVDPWDYYNGMDVWGQGTTVTVSS |
| | | | SEQ ID NO: 26568 | SEQ ID NO: 30574 |
| iPS:435791 | 21-225_180H7 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATACTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCATCTTATTACTGTCTACAGCATAATAGTTACCCATTCACTTTCGGCCCTGGGACCAAAGTGGATTTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGACTGGAGTGGGTGGCAGTTATATGGTATGATGAAAATAATAAACACTATGCAGACTCCGCGAAGGGCCGATTCACCATCTCCAGAGACAATTCCAAGAACACGCTGTATCTGCAAATGAACAGCCTGAGAGTCGAAGACACGGCTGTGTATTACTGTGCGAGAGAGGTTGGCTGGTCCGATGACTACTGGGGCCAGGGAACCCTGGTCACCGTCTCCTCA |
| | | | SEQ ID NO: 26569 | SEQ ID NO: 30575 |
| | | AA | DIQMTQSPSSLSASVGDRVTITCRASQGIRNDLGWYQQKPGKAPKRLIYTASSLQSGVPSRFSGSGSGTEFTLTISSLQPEDFASYYCLQHNSYPFTFGPGTKVDFK | QVQLVESGGGVVQPGRSLRLSCAASGFTFSDYGMHWVRQAPGKGLEWVAVIWYDENNKHYADSAKGRFTISRDNSKNTLYLQMNSLRVEDTAVYYCAREVGWSDDYWGQGTLVTVSS |
| | | | SEQ ID NO: 26570 | SEQ ID NO: 30576 |

FIGURE 50

| | | | | |
|---|---|---|---|---|
| **iPS:435793** | **21-225_180F8** | NA | GACATCCAGATGACCCAGTCTCCATCCTCCCTGTCTGCATCTGTAGGAGACGGAGTCACCATCACTTGCCGGGCAAGTCAGACCATTCTCAGCTATTTAAATTGGTATCAGCAGAAACCAGGGAAAGCCCCTAAACTCCTGATCTATGGTGTATCCAGTTTACAAAGTGGGGTCCCATCAAGGTTCAGTGGCAGTGGATCTGGGACAGATTTCTCTCTCACCATCAGCAGTCTGCAACCTGAAGATTTTGCAACTTACTACTGTCAGCAGAGTTACAGTACCCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGTCAGTTATATGGTATGATGGAAGTGATAAATACTATGAAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCATCCCCGGTGGAGCTACGGAGACTACTGGGGCCAGGGAACCCTGGTCACCGTGTCCTCA |
| | | | SEQ ID NO: 26571 | SEQ ID NO: 30577 |
| | | AA | DIQMTQSPSSLSASVGDGVTITCRASQTILSYLNWYQQKPGKAPKLLIYGVSSLQSGVPSRFSGSGSGTDFSLTISSLQPEDFATYYCQQSYSTPFTFGPGTKVDIK | QVQLVESGGGVVQPGRSLRLSCAASGFTFSSYGMHWVRQAPGKGLEWVSVIWYDGSDKYYEDSVKGRFTISRDNSKNTLYLQMNSLRAEDTAVYYCARDHPRWSYGDYWGQGTLVTVSS |
| | | | SEQ ID NO: 26572 | SEQ ID NO: 30578 |
| **iPS:435795** | **21-225_181C2** | NA | GAAATTGTGATGACCCAGACTCCACTCTCTCTGTCCGTCACCCCTGGACAGCCGGCCTCCATCTCCTGCAAGTCTAGTCAGAGCCTCCTGCATGGTGATGGAAAGACCTATTTGTATTGGTACCTGCAGAAGCCAGGCCAGCCTCCACAGCTCCTGATCCATGAAGTTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGGTTTATTACTGCATGCAAAGTATACAGGTTCCCTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGTAGCGTCTGGATTCACCTTCAGTAGCTATGGCATGCACTGGGTCCGCCAGGCTCCAGGCAAGGGGCTGGAATGGGTGACAATTATATGGTATGATGGAAGTTATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCATTACGATTTTTGGAGTGGGCACTTTGACTTCTGGGGCCAGGGAACCCTGGTCACCGTCTCCTCA |
| | | | SEQ ID NO: 26573 | SEQ ID NO: 30579 |

FIGURE 50

| | | | | |
|---|---|---|---|---|
| | | AA | EIVMTQTPLSLSVTPGQPASISCKSSQSLLHGDGKTYLYWYLQKPGQPPQLLIHEVSNRFSGVPDRFSGSGSGTDFTLKISRVEAEDVGVYYCMQSIQVPWTFGQGTKVEIK | QVQLVESGGGVVQPGRSLRLSCVASGFTFSSYGMHWVRQAPGKGLEWVTIIWYDGSYKYYADSVKGRFTISRDNSKNTLYLQMNSLRAEDTAVYYCARDHYDFWSGHFDFWGQGTLVTVSS |
| | | | SEQ ID NO: 26574 | SEQ ID NO: 30580 |
| **iPS:435797** | **21-225_181G2** | NA | GACATCCAGATGACCCAGTCTCCATCCTCACTGTCTGCATCTATAGGAGAGAGAGTCACCATCACTTGTCGGGCGAGTCAGGGCATTAGCAATTATTTAGCCTGGATTCAGCAGAAACCAGGGACAGCCCCTAAGTCCCTGATCTATGCTGCATCCAGTTTGCAAAGTGGGGTCTCATCAAGGTTCAGCGGCAGTGGATTTGGGACAGATTTCACTCTCACCATCAGTAGTCTGCAGCCTGAAGATTTTGCAACTTATTACTGCCAACAGTATAATGGTTACCCATTCACTTTCGGCCCTGGGACCAAAGTGGATATCAAA | CAGGTGCAGCTGGTGCAGTCTGGGGCTGAAGTGAAGACGCCTGGGGCCTCAGTGAAGGTCTCCTGCAGGGCTTCTGGATACACCTTCACCAGCTACAATATGCACTGGGTGCGACAGGTCCCTGGACAAGGGCTTGAGTGGATGGGATGGATCAACCCTAACAATGGTGGCTCAAACTATACACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGAAAGTTTGGGGACTGGGGCCAGGGAACCCTGGTCACCGTCTCCTCA |
| | | | SEQ ID NO: 26575 | SEQ ID NO: 30581 |
| | | AA | DIQMTQSPSSLSASIGERVTITCRASQGISNYLAWIQQKPGTAPKSLIYAASSLQSGVSSRFSGSGFGTDFTLTISSLQPEDFATYYCQQYNGYPFTFGPGTKVDIK | QVQLVQSGAEVKTPGASVKVSCRASGYTFTSYNMHWVRQVPGQGLEWMGWINPNNGGSNYTQKFQGRVTMTRDTSISTAYMELSRLRSDDTAVYYCARKFGDWGQGTLVTVSS |
| | | | SEQ ID NO: 26576 | SEQ ID NO: 30582 |

FIGURE 50

| | | | | |
|---|---|---|---|---|
| iPS:435799 | 21-225_181G3 | NA | GACATCCAGATGACCCAGTCTCCATCCTCCCT GTCTGCATCTGTAGGAGACAGAGTCAACATCA CTTGCCGGGCAAGTCACAGCATTAGCAACTAT TTAAATTGGTATCAGCAGAAAGCAGGGAAAG CCCCTAACCTCTTGATCTATACTACATTGAATT TGCAAAGTGGGGTCCCATCAAGGTTCAGTGGC AGTGGATCTGGGACAGATTTCACTCTCACCAT CAGCAGTCTGCAACCTGAAGATTTTGCAACTT ACTACTGTCAACAGAGTTACAGTTCTCCTCCG TGGACGTTCGGCCAAGGGACCAAGGTGGAAA TCAAA | GAGGTGCAACTGTTGGAGTCTGGGGGAGGCTTG GTACAGCCTGGGGGGTCCCTGAGACTCTCCTGTG CAGCCTCTGGATTCACCTTTAGCAGCTATGCCAT GAGTTGGGTCCGCCAGGCTCCAGGGAAGGGGCT GGAGTGGGTCTCAGTTATTAGTGGTAGTGGTGGT AACACATTCTACGGAGACTCCGTGAAGGGCCGG TTCACCATCTCCAGAGACAATTCCAAGAACACGC TGTATCTGCAAATGAACAGCCTGAGAGCCGAGG ACACGGCCATATATTACTGTGCGAAACGGGAGA CCTACGACTGGGGATCCGATGCTTTTGATATCTG GGGCCAAGGGACAATGGTCACCGTCTCTTCA |
| | | | SEQ ID NO: 26577 | SEQ ID NO: 30583 |
| | | AA | DIQMTQSPSSLSASVGDRVNITCRASHSISNYLN WYQQKAGKAPNLLIYTTLNLQSGVPSRFSGSGS GTDFTLTISSLQPEDFATYYCQQSYSSPPWTFGQ GTKVEIK | EVQLLESGGGLVQPGGSLRLSCAASGFTFSSYAMS WVRQAPGKGLEWVSVISGSGGNTFYGDSVKGRFTI SRDNSKNTLYLQMNSLRAEDTAIYYCAKRETYDW GSDAFDIWGQGTMVTVSS |
| | | | SEQ ID NO: 26578 | SEQ ID NO: 30584 |
| iPS:435801 | 21-225_181E5 | NA | GACATCGTGATGACCCAGTCTCCAGACTCCCT GGCTGTGTCTCTGGGCGAGAGGGCCACCATCA ACTGCAAGTCCAGCCAGAGTGTTTTACACAGC TCCAACAATTACAACTACTTAACTTGGTACCA GCAGAAACCAGGACAGCCTCCTAAGCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCATCAG TATTTTATTACTCCGTGGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACACAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGAGTAGTAG TGGCTGGTACATCTTTGACTACTGGGGCCAGGGA ACCCTGGTCACCGTCTCCTCA |
| | | | SEQ ID NO: 26579 | SEQ ID NO: 30585 |

FIGURE 50

| | | | | |
|---|---|---|---|---|
| | | AA | DIVMTQSPDSLAVSLGERATINCKSSQSVLHSSNNYNYLTWYQQKPGQPPKLLIYWASTRESGVPDRFSGSGSGTDFTLTISSLQAEDVAVYYCHQYFITPWTFGQGTKVEIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYYCASSSGWYIFDYWGQGTLVTVSS |
| | | | SEQ ID NO: 26580 | SEQ ID NO: 30586 |
| iPS:435805 | 21-225_181A8 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGAAAAGCCCCTAAGCGCCTGATCTATACTGCATCCAGTTTGCAAAGTGGGGTCCCATCAAGGTTCAGCGGCAGTGGATCTGGGACAGATTTCACTCTCACAATCAGCAGCCTGCAGCCTGAAGATTTTGCATCTTATTACTGTCTACAGCATAATAGTTACCCATTCACTTTCGGCCCTGGGACCAAAGTGGATTTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGACTGGAGTGGGTGGCAGTTATATGGTATGATGAAAATAATAAACACTATGCAGACTCCGCGAAGGGCCGATTCACCATCTCCAGAGACAATTCCAAGAACACGCTGTATCTGCAAATGAACAGCCTGAGAGCCGAAGACACGGCTGTGTATTACTGTGCGAGAGAGGTTGGCTGGTCCGATGACTACTGGGGCCAGGGAACCCTGGTCATCGTCTCCTCA |
| | | | SEQ ID NO: 26581 | SEQ ID NO: 30587 |
| | | AA | DIQMTQSPSSLSASVGDRVTITCRASQGIRNDLGWYQQKPGKAPKRLIYTASSLQSGVPSRFSGSGSGTDFTLTISSLQPEDFASYYCLQHNSYPFTFGPGTKVDFK | QVQLVESGGGVVQPGRSLRLSCAASGFTFSDYGMHWVRQAPGKGLEWVAVIWYDENNKHYADSAKGRFTISRDNSKNTLYLQMNSLRAEDTAVYYCAREVGWSDDYWGQGTLVIVSS |
| | | | SEQ ID NO: 26582 | SEQ ID NO: 30588 |

FIGURE 50

| | | | | |
|---|---|---|---|---|
| **iPS:435807** | **21-225_181C10** | NA | GATATTGTGATGACCCAGACTCCACTCTCTCTGTCCGTCACCCCTGGACAGCCGGCCTCCATCTCCTGCAAGTCTAGTCAGAGCCTCCTGCATGGTGATGGAAAGACCTATTTGTATTGGTACCTGCAGAAGCCAGGCCAGCCTCCACAGCTCCTGATCTATGAAGTTTCCAATCGGTTCTCTGGAGTGCCAGATAGGTTCAGTGGCAGCGGGTCAGGGACAGATTTCACACTTAAAATCAGCCGGGTGGAGGCTGAGGATGTTGGGGTTTATCACTGCATGCAAAGTATACAGATTCCCTGGACGTTCGGCCAAGGGACCAAGGTGGAAATCAAA | CAGGTGCAACTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGATGGCAATTATATGGTATGATGGAAGTTATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCATTACGATTTTTGGAGTGGGCACTTTGACTACTGGGGCCAGGGAACCCTGGTCACCGTCTCCTCA |
| | | | SEQ ID NO: 26583 | SEQ ID NO: 30589 |
| | | AA | DIVMTQTPLSLSVTPGQPASISCKSSQSLLHGDGKTYLYWYLQKPGQPPQLLIYEVSNRFSGVPDRFSGSGSGTDFTLKISRVEAEDVGVYHCMQSIQIPWTFGQGTKVEIK | QVQLVESGGGVVQPGRSLRLSCAASGFTFSSYGMHWVRQAPGKGLEWMAIIWYDGSYKYYADSVKGRFTISRDNSKNTLYLQMNSLRAEDTAVYYCARDHYDFWSGHFDYWGQGTLVTVSS |
| | | | SEQ ID NO: 26584 | SEQ ID NO: 30590 |
| **iPS:435809** | **21-225_182H5** | NA | GACATCCAGATGACCCAGTCTCCATCTTCCGTGTATGCATCTGTTGGAGACAGAGTCACCATCACTTGTCGGGCGAGTCAGGATATTACCAGCTGGTTAGCCTGGTATCAGCAGAAACCAGGGAAAGCCCCTAAACTCCTGATCTATGCTGCATCCAGTTTACAAAGTGGGGTCCCATCAAGGTTCAGCGGCAGTGGATCTGGGACAGATTTCATTCTCACCATCACCAGCGTGCAGCCTGATGATTTTGCAACTTACTATTGTCAACAGGTTAACAGTTTCCCATTCACTTTCGGCCACGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGTTGGGTCCGCCAGGCTCCAGGGAAGGGGCTGGAGTGGGTCTCAGTTATTAGTGGTAGAGGTGGTACCACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAACGGACTGGGGATGATGTTTTTGATATCTGGGGCCAAGGGACAATGGTCACCGTCTCTTCA |
| | | | SEQ ID NO: 26585 | SEQ ID NO: 30591 |

FIGURE 50

| | | | | |
|---|---|---|---|---|
| | | AA | DIQMTQSPSSVYASVGDRVTITCRASQDITSWLA WYQQKPGKAPKLLIYAASSLQSGVPSRFSGSGS GTDFILTITSVQPDDFATYYCQQVNSFPFTFGHG TKVDIK | EVQLLESGGGLVQPGGSLRLSCAASGFTFSSYAMS WVRQAPGKGLEWVSVISGRGGTTFYADSVKGRFTI SRDNSKNTLYLQMNSLRAEDTAVYYCAKRTGDDV FDIWGQGTMVTVSS |
| | | | SEQ ID NO: 26586 | SEQ ID NO: 30592 |
| **iPS:435811** | **21-225_183H6** | NA | GACATCCAGATGACCCAGTCTCCATCCTCCCT GTCTGCATCTGTAGGAGACAGAGTCACCATCG CTTGCCAGGCGAGTCAGGACATTAGCAACTAT TTAAATTGGTATCAGCAGACACCAGGGAAAGC CCCTAAGGTCCTGATCTACGATGCATCCAATT TGGAAACAGGGGTCCCAGCAAGGTTCAGTGG AAGTGGATCTGGGACAGATTTTACTTTCACCA TCAGCAGCCTGCAGCCTGAAGATATTGCAACA TATTACTGTCAACAGTATGATAATCTCCCTCTC ACTTTCGGCGGAGGGACCAAGGTGGACATCA AA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAGTGGGTGGCAATTATATCATATGCTGGAAGT ACTAAATTCTATGCAGACTCCGTGAAGGGCCGAT TCACCATCTCCAGAGACAATTCCAAGAACACGCT GTATCTGCAAATGAACAGCCTGAGAGCTGAGGA CACGGCTGTGTATTACTGTGTGAGAAGGCCCCCG CAGTGGCTGGTAGAGGGCTACGGTATGGACGTCT GGGGCCAAGGGACCACGGTCACCGTCTCCTCA |
| | | | SEQ ID NO: 26587 | SEQ ID NO: 30593 |
| | | AA | DIQMTQSPSSLSASVGDRVTIACQASQDISNYLN WYQQTPGKAPKVLIYDASNLETGVPARFSGSGS GTDFTFTISSLQPEDIATYYCQQYDNLPLTFGGG TKVDIK | QVQLVESGGGVVQPGRSLRLSCAASGFTFSSYGMH WVRQAPGKGLEWVAIISYAGSTKFYADSVKGRFTI SRDNSKNTLYLQMNSLRAEDTAVYYCVRRPPQWL VEGYGMDVWGQGTTVTVSS |
| | | | SEQ ID NO: 26588 | SEQ ID NO: 30594 |

FIGURE 50

| | | | | |
|---|---|---|---|---|
| **iPS:435813** | **21-225_183A12** | NA | GACATCCAGATGACCCAGTCTCCATCCTCCCTGTGTGCCTCTGTAGGAGACAGAGTCACCATCACTTGCCGGGCAAGTCGGAACATCAGCAACTATTTAAATTGGTATCAGCAGAAACCAGGGAAAGCCCCTAAGCTCCTGATCTATGTTGTATCCAGTTTGCAAAGTGGGGTCCCATCAAGGTTCAGTGGCAGTGGATCTGGGACAGATTTCACTCTCACCATCAGCAGTCTACAACCTGAAGATTTTGCAACTTACTACTGTCAACAGAGTTACAGTTCCCCTCCGTGGACGTTCGGCCAAGGGACCAAGGTGGATATCAGA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAGTGGGTGGCAGTTATATCATCTGCTGGAAGTAATAAATACTATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTATTGTGCGAGAAGGTATAGCAGTGGCTGGGACTGGTTCGACCCCTGGGGCCAGGGAACCCTGGTCACCGTCTCCTCA |
| | | | SEQ ID NO: 26589 | SEQ ID NO: 30595 |
| | | AA | DIQMTQSPSSLCASVGDRVTITCRASRNISNYLNWYQQKPGKAPKLLIYVVSSLQSGVPSRFSGSGSGTDFTLTISSLQPEDFATYYCQQSYSSPPWTFGQGTKVDIR | QVQLVESGGGVVQPGRSLRLSCAASGFTFSSYGMHWVRQAPGKGLEWVAVISSAGSNKYYADSVKGRFTISRDNSKNTLYLQMNSLRAEDTAVYYCARRYSSGWDWFDPWGQGTLVTVSS |
| | | | SEQ ID NO: 26590 | SEQ ID NO: 30596 |
| **iPS:435815** | **21-225_190G10** | NA | GAAATTGTGTTGACGCAGTCTCCAGGCACCCTGTCTTTGTCTCCAGGGGAAAGAGCCACCCTCTCCTGCAGGGCCAGTCCGAGTGTTAGCAGCAGATTCTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCAACAGCAGACTGGAGCCTGAAGATTTTGCAGTGTATTACTGTCAGCAGTATGGTAGCTCACCTCCGTGGACGTTCGGCCAAGGGACCAAGGTAGAAATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGGGACTATAGCATGAACTGGGTCCGCCAGGCTCCAGGGAAGGGGCTGGAGTGGGTCTCATCTATTAGTAGTGGTAGTGGTTACATACACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGCGAGCAACTATGGCCCTTGACTACTGGGGCCAGGGAACCCTGGTCACCGTCTCCTCA |
| | | | SEQ ID NO: 26591 | SEQ ID NO: 30597 |

FIGURE 50

| | | | | |
|---|---|---|---|---|
| | | AA | EIVLTQSPGTLSLSPGERATLSCRASPSVSSRFLAWYQQKPGQAPRLLIYGASSRATGIPDRFSGSGSGTDFTLTNSRLEPEDFAVYYCQQYGSSPPWTFGQGTKVEIK | EVQLVESGGGLVKPGGSLRLSCAASGFTFRDYSMNWVRQAPGKGLEWVSSISSGSGYIHYADSVKGRFTISRDNAKNSLYLQMNSLRAEDTAVYYCARATMALDYWGQGTLVTVSS |
| | | | SEQ ID NO: 26592 | SEQ ID NO: 30598 |
| **iPS:435817** | **21-225_190B11** | NA | GAAATTGTGCTGACTCAGTCTCCAGACTTTCAGTCTGTGGCTCCAAAGGAGAAAGTCACCATCACCTGCCGGGCCAGTCAGAGTATTGGTAGTAACTTACACTGGTACCAGCAGAAACCAGATCAGTCTCCGAAACTCCTCATCAAGTCTGCTTCCCAGTCCTTCTCAGGGGTCCCCTCGAGGTTCAGTGGCAGTGGATCTGGGACAGATTTCACCCTCACCATCAATAGCCTGGAAACTGAAGATGCTGCAACGTATTACTGTCAGCAGAGTAGTAGTTTACCGTGGACGTTCGGCCAAGGGACCAAGGTGGAAATCAAA | CAGGTGCAGCTGCAGGAGTCGGGCCCAGGACTGGTGAAGCCTTCGGAGACCCTGTCCCTCACCTGCACTGTCTCTGGTGGCTCCATCAATAATTACTACTGGAGCTGGATCCGGCAGCCCGCCGGGAAGGGACTGGAGTGGATTGGGCGTATCTATACCAGTGGGAGCACCAACTACAACCCCTCCCTCAAGAGTCGAGTCACCATGTCAGTAGACACGTCCAAGAACCAGTTCTCCCTGAAGCTGAGCTCTGTGACCGCCGCGGACACGGCCGTGCATTACTGTGCGAGAGATCGGGGATACTATGGCTACTACGGTATGGACGTCTGGGGCCAAGGGACCACGGTCACCGTCTCCTCA |
| | | | SEQ ID NO: 26593 | SEQ ID NO: 30599 |
| | | AA | EIVLTQSPDFQSVAPKEKVTITCRASQSIGSNLHWYQQKPDQSPKLLIKSASQSFSGVPSRFSGSGSGTDFTLTINSLETEDAATYYCQQSSSLPWTFGQGTKVEIK | QVQLQESGPGLVKPSETLSLTCTVSGGSINNYYWSWIRQPAGKGLEWIGRIYTSGSTNYNPSLKSRVTMSVDTSKNQFSLKLSSVTAADTAVHYCARDRGYYGYYGMDVWGQGTTVTVSS |
| | | | SEQ ID NO: 26594 | SEQ ID NO: 30600 |

FIGURE 50

| | | | | |
|---|---|---|---|---|
| **iPS:435819** | **21-225_190C11** | NA | GACATCCAGATGACCCAGTCTCCTTCCTCACTGTCTGCATCTGTAGGAGACAGAGTCACCATCACTTGTCGGACGAGTCAGGGCATTGGCAATTATTTAGCCTGGTTTCAGCAGAAACCAGGGAAAGCCCCTAAGTCCCTGCTCTATAAAACATCCAGTTTACAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATATGACTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCAGGGCGTGGGCTACGACGGTTTGGACGTCTGGGGCCAAGGGACCACGGTCACCGTCTCCTCA |
| | | | SEQ ID NO: 26595 | SEQ ID NO: 30601 |
| | | AA | DIQMTQSPSSLSASVGDRVTITCRTSQGIGNYLAWFQQKPGKAPKSLLYKTSSLQSGVPSKFSGSGSGTDFTLTISSLQPEDFATYYCQQYMTYPLTFGGGTKVEIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAVYYCARDQGVGYDGLDVWGQGTTVTVSS |
| | | | SEQ ID NO: 26596 | SEQ ID NO: 30602 |
| **iPS:435821** | **21-225_190E11** | NA | GAAATAGTGATGACGCAGTCTCCAGCCACCCTGTCTGTGTCTCCAGGGGAAAGAGCCACCCTCTCCTGCAGGGCCAGTCAGAGTTTTCGCATCAACTTAGCCTGGTACCAGCAGAGACCTGGCCAGGCTCCCAGGCTCCTCATCTATGGTGCATCCACCAGGGCCACTGGTATCCCAGCCAGGTTCAGTGGCAGTGGGTCTGGGACAGAGTTCACTCTCACCATCAGCAGCCTGCAGTCTGAAGATTTTGCAGTTTATTACTGTCAGCAGTATAATAACTGGCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTACAGCGTCTGGATTCACCTTCAGTAACTATGGCATGCACTGGGTCCGCCAGGCTCCAGGCAAGGGGCTGGAGTGGGTGGCAATTATATGGTTTGATGGAAGTAATAAATACTATGCAGACTCCGTGAAGGGCCGATTCACCATCTCCAGAGACAATTCCAATAACACGCTGTATCTGCAAATGAACAGCCTGCGAGCCGAGGACACGGCTGTGTATTACTGTGCGAAAGCCCAGGGGGTCTACTACTACGTTATGGACGTCTGGGGCCAAGGGACCACGGTCACCGTCTCCTCA |
| | | | SEQ ID NO: 26597 | SEQ ID NO: 30603 |

FIGURE 50

| | | | | |
|---|---|---|---|---|
| | | AA | EIVMTQSPATLSVSPGERATLSCRASQSFRINLAWYQQRPGQAPRLLIYGASTRATGIPARFSGSGSGTEFTLTISSLQSEDFAVYYCQQYNNWPLTFGGGTKVEIK | QVQLVESGGGVVQPGRSLRLSCTASGFTFSNYGMHWVRQAPGKGLEWVAIIWFDGSNKYYADSVKGRFTISRDNSNNTLYLQMNSLRAEDTAVYYCAKAQGVYYYVMDVWGQGTTVTVSS |
| | | | SEQ ID NO: 26598 | SEQ ID NO: 30604 |
| **iPS:435823** | **21-225_190F11** | NA | GAAATTGTGCTGACTCAGTTTCCAGACTCTCAGTCTGTGACTCCAAAGGAGAAAGTCACCATCACCTGCCGGGCCAGTCAGAACATTGGTAGTAGCTTACACTGGTACCAGCAGAAACCAGAACAGTCTCCAAAGGTCCTCATCAAGTATGCTTCCCAGTCCTTCTCAGGGGTCCCCTCGAGGTTCAGTGGCAGTGGATCTGGGACAGATTTCACCCTCACCATCAATAGCCTGGAAGCTGAAGATGCTGCAACGTATTACTGTCATCAGAGTAGTAGTTTCCCTCGGACGTTCGGCCAAGGGACCAAGGTGGAAATCAAA | GAGGTGCAGCTGTTGGACTCTGGGGGAGGCTTGGGACAGCCTGGGGGGTCCCTGAGACTCTCCTGTGCAGCCTCTGGATTCACCTTTGACAGCTATGCCATGAACTGGGTCCGCCAGGCTCCAGGGAAGGGGCTGGAGTGGGTCTCAACTATTAGTGGTACTGGTCGTAGGACATACTACGCAGACTCCGTGAAGGGCCGGTTCACCATCTCCAGAGACAATTCCAAGAACACGCTGTATCTGCAAATGAATAGCCTGAGAGCCGAGGACACGGCCGTATATTACTGTGCGAAAGAGGAGGATTACTATGATAGTAGTGGCCCGGGGTTCGACCCCTGGGGCCAGGGAACCCTGGTCACCGTCTCCTCA |
| | | | SEQ ID NO: 26599 | SEQ ID NO: 30605 |
| | | AA | EIVLTQFPDSQSVTPKEKVTITCRASQNIGSSLHWYQQKPEQSPKVLIKYASQSFSGVPSRFSGSGSGTDFTLTINSLEAEDAATYYCHQSSSFPRTFGQGTKVEIK | EVQLLDSGGGLGQPGGSLRLSCAASGFTFDSYAMNWVRQAPGKGLEWVSTISGTGRRTYYADSVKGRFTISRDNSKNTLYLQMNSLRAEDTAVYYCAKEEDYYDSSGPGFDPWGQGTLVTVSS |
| | | | SEQ ID NO: 26600 | SEQ ID NO: 30606 |

FIGURE 50

| | | | | |
|---|---|---|---|---|
| **iPS:435825** | **21-225_190G11** | NA | GACATCCAGATGACCCAGTCTCCTTCCTCACTGTCTGCATCTGTAGGAGACAGAGTCACCATCACTTGTCGGACGAGTCAGGGCATTGGCAATTATTTAGCCTGGTTTCAGCAGAAACCAGGGAAAGCCCCTAAGTCCCTGCTCTATAAAGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATATGACTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTATTTATGGCATGCACTGGGTCCGCCAGGCTCCAGGCAAGGGGCTGGAGTGGGTGGCAGTTATATGGTATGATGGAAGTAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCAGGGCGTGGGCTACGACGGTTTGGACGTCTGGGGCCAAGGGACCACGGTCACCGTCTCCTCA |
| | | | SEQ ID NO: 26601 | SEQ ID NO: 30607 |
| | | AA | DIQMTQSPSSLSASVGDRVTITCRTSQGIGNYLAWFQQKPGKAPKSLLYKASSLQSGVPSKFSGSGSGTDFTLTISSLQPEDFATYYCQQYMTYPLTFGGGTKVEIK | QVQLVESGGGVVQPGRSLRLSCAASGFTFSIYGMHWVRQAPGKGLEWVAVIWYDGSNKNYADSVKGRFTISRDNSKNTLYLQMNSLRAEDTAVYYCARDQGVGYDGLDVWGQGTTVTVSS |
| | | | SEQ ID NO: 26602 | SEQ ID NO: 30608 |
| **iPS:435827** | **21-225_190H11** | NA | GATATTGTGATGACCCAGACTCCACTCTCTCTGTCCGTCACCCCTGGACAGCCGGCCTCCATCTCCTGCAAGTCTAGTCAGAGCCTCCTCCATAGTGATGGAAGGACCTATTTGTATTGGTACCTGCAGAAGCCAGGCCAGCCTCCACAGCTCCTGATCTGTGAGGTTTCCAACCGGTTCGCTGGAGTGACAGATAGGTTCAGTGGCAGCGGGTCAGGGACAGATTTCACACTGAAAATCAGCCGGGTGGAGGCTGGGGATGTTGGGGTTTATTACTGCATGCAAAGTATACAGTTTCCCTGGACGTTCGGCCAAGGGACCAAGGTGGAAATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGAAGCTACCACTGGAGCTGGATCCGGCAGCCCGCCGGGAAGGGACTGGAGTGGATTGGGCTTATCTATACCAGTAGGAGCACCATTTACAACCCCTCCCTCAAGAGTCGAGTCACCCTGTCAGTAGACACGTCCAAGAACCAGTTCTCCCTGAAGCTGAGCTCTGTGACCGCCGCGGACACGGCCGTGTATTACTGTGCGAGACTCCGGTATAACTGGAACTTCCCTTACTTTGACTACTGGGGCCAGGGAACCCTGGTCACCGTCTCCTCA |
| | | | SEQ ID NO: 26603 | SEQ ID NO: 30609 |

FIGURE 50

| | | | | |
|---|---|---|---|---|
| | | AA | DIVMTQTPLSLSVTPGQPASISCKSSQSLLHSDGRTYLYWYLQKPGQPPQLLICEVSNRFAGVTDRFSGSGSGTDFTLKISRVEAGDVGVYYCMQSIQFPWTFGQGTKVEIK | QLQLQESGPGLVKPSETLSLTCTVSGGSIRSYHWSWIRQPAGKGLEWIGLIYTSRSTIYNPSLKSRVTLSVDTSKNQFSLKLSSVTAADTAVYYCARLRYNWNFPYFDYWGQGTLVTVSS |
| | | | SEQ ID NO: 26604 | SEQ ID NO: 30610 |
| **iPS:435829** | **21-225_190B12** | NA | GAAATTGTGCTGACTCAGTCTCCAGACTTTCAGTCTGTGACTCCAAAGGAGAAAGTCACCATCACCTGCCGGGCCAGTCAGAGTATTGGTAGTAGCTTACACTGGTACCAGCAGAAACCAGATCAGTCTCCAAAGCTCCTCATCAAGTATGCTTCCCAGTCCTTCTCAGGGGACCCCTCGAGGTTCAGTGGCAGTGGATCTGGGACAGATTTCACCCTCACCATCAATAGCCTGGAAGCTGAAGATGCTGCAACGTATTACTGTCATCAGACTAGAAGTTTACCTCTCACTTTCGGCGGAGGGACCAAGGTGGAGATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGGTTACTACTGGAACTGGATCCGCCAGCACCCAGGGAAGGGCCTGGAGTGGATTGGGTATATCTATTACAGTGGGAGCACCTACTACAACCCGTCCCTCAAGAGTCGAGTTACCATATCAGTAGACACGTCTAAGAACCAGTTTTTTCTGAAGCTGAACTCTGTGACTGCCGCGGACACGGCCGTGTATTACTGTGCGAGATCCGGGTATAATTGGGACGCCGGGGTCGACCCCTGGGGCCGGGGAACCCTGGTCACCGTCTCCTCA |
| | | | SEQ ID NO: 26605 | SEQ ID NO: 30611 |
| | | AA | EIVLTQSPDFQSVTPKEKVTITCRASQSIGSSLHWYQQKPDQSPKLLIKYASQSFSGDPSRFSGSGSGTDFTLTINSLEAEDAATYYCHQTRSLPLTFGGGTKVEIK | QVQLQESGPGLVKPSQTLSLTCTVSGGSISSGGYYWNWIRQHPGKGLEWIGYIYYSGSTYYNPSLKSRVTISVDTSKNQFFLKLNSVTAADTAVYYCARSGYNWDAGVDPWGRGTLVTVSS |
| | | | SEQ ID NO: 26606 | SEQ ID NO: 30612 |

FIGURE 50

| | | | | |
|---|---|---|---|---|
| **iPS:435831** | **21-225_190C12** | NA | GACATCCAGATGACCCAGTCTCCATCCTCCCTGTCTGCATCTGTAGGAGACAGAGTCACCATCACTTGCCGGGCAAGTCAGGGCATTAGAAAAGATTTAGGCTGGTATCAGCAGAAACCAGGGAAAGCCCCTAAGCGCCTGATCCATACTGCATCCAGTTTGCAAAGTGGGGTCCCATTAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | CAGGTGCAGCTGGTGGAGTCTGGGGGAGCCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAGTGGGTGGCAGTTATATCATATGATGGAGGTTATAAAAACTATGTAGACTCCGTGAAGGGCCGATTCACCATCTCCAGAGACAATTCCAAGAACACGCTGTATCTGCAAATGAATAGCCTGAGAGCTGAGGACACGGCTGTGTATTACTGTGCGAGAGGTACCCACGGGTACTACTACGGTGTGGACGTCTGGGGCCAAGGGACCACGGTCACCGTCTCCTCA |
| | | | SEQ ID NO: 26607 | SEQ ID NO: 30613 |
| | | AA | DIQMTQSPSSLSASVGDRVTITCRASQGIRKDLGWYQQKPGKAPKRLIHTASSLQSGVPLRFSGSGSGTEFTLTISSLQPEDFATYYCLQHNSYPWTFGQGTKVEIK | QVQLVESGGAVVQPGRSLRLSCAASGFTFSSYGMHWVRQAPGKGLEWVAVISYDGGYKNYVDSVKGRFTISRDNSKNTLYLQMNSLRAEDTAVYYCARGTHGYYYGVDVWGQGTTVTVSS |
| | | | SEQ ID NO: 26608 | SEQ ID NO: 30614 |
| **iPS:435833** | **21-225_190D12** | NA | GACATTCAGATGACCCAGTCTCCATCCTCCCTGTCTGCATCTGTAGGAGACAGAGTCACCATCACTTGCCGGGCGAGTCAGGGCATTAGCAATTATTTAGCCTGGTATCAGCAGAAACCAGGGAAAGTCCCTAAACTCCTGATCTATGTTGCATCCACTTTGCAATCAGGGGTCCCATCTCGGTTCAGTGGCAGTGGATCTGGGACAGATTTCACTCTCACCATCAGCAGCCTGCAGCCTGAAGATGTTGCAACTTATTACTGTCAAAAGTATAACAGTGCCCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGACTCAGCTATTATTGGTAATGGTGGTAGGACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAAGATATGGGTAGATACAGCTATGGTTTCTTTGACTACTGGGGCCAGGGAACCCTGGTCACCGTCTCCTCA |
| | | | SEQ ID NO: 26609 | SEQ ID NO: 30615 |

FIGURE 50

| | | | | |
|---|---|---|---|---|
| | | AA | DIQMTQSPSSLSASVGDRVTITCRASQGISNYLA WYQQKPGKVPKLLIYVASTLQSGVPSRFSGSGS GTDFTLTISSLQPEDVATYYCQKYNSAPFTFGPG TKVDIK | EVQLLESGGGLVQPGGSLRLSCAASGFTFSSYAMS WVRQAPGKGLEWDSAIIGNGGRTYYADSVKGRFTI SRDNSKNTLYLQMNSLRAEDTAVYYCAKDMGRYS YGFFDYWGQGTLVTVSS |
| | | | SEQ ID NO: 26610 | SEQ ID NO: 30616 |
| **iPS:435835** | **21-225_190F12** | NA | GACATCCAGATGACCCAGTCTCCATCCTCACT GTCTGCATCTGTGGGAGACAGAGTCACCATCA CTTGTCGGGCGAGTCAGGGCATTGGCAAGTAT TTAGCCTGGTTTCAGCAGAAACCAGGGAAAGC CCCTAAGTCACTGATCTATGCTGCATCCAGTTT GCAAAGTGGGGTCCCATCAAAATTCAGCGGCA GTGGATCTGGGACAGATTTCACTCTCACCATC AGCAGCCTGCAGCCTGACGATTTTGCAACTTA TTACTGCCAACGATATGATACTTACCCATTCA CTTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGGAACTATGGCAT GCACTGGGTCCGCCAGGCTCCAGGCAAGGGGCT GGAGTGGGTGGCAGTTATATGGTTTGATGGAAGT AATGACTACTATGCAGACTCCGTGAAGGGCCGAT TCACCATCTCCAGAGACAATTCCAAGAACACGCT GTCACTGCAAATGAACAGCCTGAGAGCCGAGGA CACGGCTGTGTATTACTGTGCGAGAGATAGAAGC GTCGGCTACGACGGTTTAGATGTCTGGGGCCAAG GGACCACGGTCACCGTCTCCTCA |
| | | | SEQ ID NO: 26611 | SEQ ID NO: 30617 |
| | | AA | DIQMTQSPSSLSASVGDRVTITCRASQGIGKYLA WFQQKPGKAPKSLIYAASSLQSGVPSKFSGSGSG TDFTLTISSLQPDDFATYYCQRYDTYPFTFGPGT KVDIK | QVQLVESGGGVVQPGRSLRLSCAASGFTFRNYGM HWVRQAPGKGLEWVAVIWFDGSNDYYADSVKGR FTISRDNSKNTLSLQMNSLRAEDTAVYYCARDRSV GYDGLDVWGQGTTVTVSS |
| | | | SEQ ID NO: 26612 | SEQ ID NO: 30618 |

FIGURE 50

| | | | | |
|---|---|---|---|---|
| **iPS:435837** | **21-225_198G3** | NA | GACATCCAGATGGCCCAGTCTCCTTCCTCACTGTCTGCATCTGTAGGAGACAGAGTCACCATCACTTGTCGGACGAGTCAGGGCATTGGCAAGTATTTAGCCTGGTTTCAGCAGAAGCCAGGGAAAGCCCCTAAGTCCCTGCTCTATAAAGCATCCAGTTTGCAAGGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATATGACTTACCCGCTCACTTTCGGCGGAGGGACCAAGGTGGAGATCAAA | CAGGTGCAGCTGGTGGAATCTGGGGGAGGCGTGGTCCAGCCTGGGAGGTCCCTGAAACTCTCCTGTGCAGCGTCTGGATTCACCTTCAGTACCTATGGCATGCACTGGGTCCGCCAGGCTCCAGGCAAGGGACTGGAGTGGGTGGCAGTTATATGGTATGATGGAACTAATAAAAACTATGCAGACTCCGTGAAGGGCCGATTCACCATCTCCAGAGACAATTCCAAGAACACGCTGTGTCTGCAAATGAACAGCCTGAGAGCCGAGGACACGGCTGTGTATTACTGTGCGAGAGATCAGGGCGTGGGCTACGACGGTTTGGACGTCTGGGGCCAAGGGACCACGGTCACCGTCTCCTCA |
| | | | SEQ ID NO: 26613 | SEQ ID NO: 30619 |
| | | AA | DIQMAQSPSSLSASVGDRVTITCRTSQGIGKYLAWFQQKPGKAPKSLLYKASSLQGGVPSKFSGSGSGTDFTLTISSLQPEDFATYYCQQYMTYPLTFGGGTKVEIK | QVQLVESGGGVVQPGRSLKLSCAASGFTFSTYGMHWVRQAPGKGLEWVAVIWYDGTNKNYADSVKGRFTISRDNSKNTLCLQMNSLRAEDTAVYYCARDQGVGYDGLDVWGQGTTVTVSS |
| | | | SEQ ID NO: 26614 | SEQ ID NO: 30620 |
| **iPS:435839** | **21-225_191B1** | NA | GACATTGTGATGACCCAGACTCCACTCTCTCTGTCCGTCACCCCTGGACAGCCGGCCTCCATCTCCTGTAGGTCTAGTCAGAGCCTCCTGCATAGTGATGGAAAGACCTATTTGTTTTGGTACCTGCAGAAGCCAGGCCAGCCTCCACAGGTCCTGATCTATGAACTTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGGTTTATTACTGCATGCAAAGTTTCCAGCTTCCCTGGACGTTCGGTCAAGGGACCAAGGTGGAAATCAAT | CAGGTGCAGCTGCAGGAGTCGGGCCCAGGACTGGTGAAGCCTTCGGAGACCCTGTCCCTCACCTGCACTGTCTCTGGTGGCTCCATCAGTAGTTATCACTGGAGCTGGATCCGGCAGCCCGCCGGGAAGGGACTGGAGTGGATTGGCCATATCTATACCAGTGGGAGCACCAAGTACAACCCCTCCCTCAAGAGTCGAGTCACCATGTCAGTAGACACGTCCAAGAACCAGTTCTCCCTGAAGCTGAGCTCTGTGACCGCCGCGGACACGGCCGTGTATTACTGTGCGAGACTCCGGTATAACTGGAACTTCCCTTTCTTTGACTATTGGGGCCAGGGAACCCTGGTCACCGTCTCCTCA |
| | | | SEQ ID NO: 26615 | SEQ ID NO: 30621 |

FIGURE 50

| | | | | |
|---|---|---|---|---|
| | | AA | DIVMTQTPLSLSVTPGQPASISCRSSQSLLHSDGKTYLFWYLQKPGQPPQVLIYELSNRFSGVPDRFSGSGSGTDFTLKISRVEAEDVGVYYCMQSFQLPWTFGQGTKVEIN | QVQLQESGPGLVKPSETLSLTCTVSGGSISSYHWSWIRQPAGKGLEWIGHIYTSGSTKYNPSLKSRVTMSVDTSKNQFSLKLSSVTAADTAVYYCARLRYNWNFPFFDYWGQGTLVTVSS |
| | | | SEQ ID NO: 26616 | SEQ ID NO: 30622 |
| iPS:435841 | 21-225_191D8 | NA | GACATCATGATGACCCAGTCTCCAGACTCCCTGGCTGTGTCTCTGGGCGAGAGGGCCATCATCAGCTGCAGGTCCAGCCAGAGTGTTTTACACAGCTCCAACAATTACAACTACTTAGCTTGGTACCAGCAGAAACCAGGACATCCTCCTAAACTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAACAATATTATAGTACTCCTCCGACGTTCGGCCTAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCCCATAGCAGTGGCTGGTACTACTTTGACTACTGGGGCCAGGGAACCCTGGTCACCGTCTCCTCA |
| | | | SEQ ID NO: 26617 | SEQ ID NO: 30623 |
| | | AA | DIMMTQSPDSLAVSLGERAIISCRSSQSVLHSSNNYNYLAWYQQKPGHPPKLLIYWASTRESGVPDRFSGSGSGTDFTLTISSLQAEDVAVYYCQQYYSTPPTFGLGTKVEIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAHSSGWYYFDYWGQGTLVTVSS |
| | | | SEQ ID NO: 26618 | SEQ ID NO: 30624 |

FIGURE 50

| | | | | |
|---|---|---|---|---|
| iPS:435843 | 21-225_191F1 | NA | GAAATTGTGTTGACGCAGTCTCCAGGCACCCTGTCTTTGTCTCCAGGGGAAAGAGCCGCCCTCTCCTGCAGGGCCAGTCAGAGTATTAGCCTCAACTTCTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAATAGACTGGAGCCTGAAGATTTTGCAGTGTATTACTGTCAGCAGTATGGTAGGTCACCGTGGACGTTCGGCCAAGGGACCAAGGTGGAAGTCAAA | CAGGTGCAGCTGCAGGAGTCGGGCCCAGGACTGGTGAAGCCTTCACAGACCCTGTCCCTCACCTGCACTGTTTCTGGTGGCTCCATCAACAGTGGTGGTTACTACTGGAACTGGATCCGCCAGCACCCAGGGAAGGGCCTGGAGTGGATTGGGTACATCTTTTACAGTGGGAGCACCTACTACAACCCGTCCCTCAAGAGTCGAGTCACCATATCAGTAGACACGTCTAAGAACCAGTTCTCCCTGAAGCTGAGCTCTGTGACTGCCGCGGACACGGCCGTGTATTACTGTGCGAGAGGGGATTACGATGGTTCGGGGAGTTATCACTACTATTACGGTATGGACGTCTGGGGCCAAGGGACCACGGTCACCGTCTCCTCA |
| | | | SEQ ID NO: 26619 | SEQ ID NO: 30625 |
| | | AA | EIVLTQSPGTLSLSPGERAALSCRASQSISLNFLAWYQQKPGQAPRLLIYGASSRATGIPDRFSGSGSGTDFTLTINRLEPEDFAVYYCQQYGRSPWTFGQGTKVEVK | QVQLQESGPGLVKPSQTLSLTCTVSGGSINSGGYYWNWIRQHPGKGLEWIGYIFYSGSTYYNPSLKSRVTISVDTSKNQFSLKLSSVTAADTAVYYCARGDYDGSGSYHYYYGMDVWGQGTTVTVSS |
| | | | SEQ ID NO: 26620 | SEQ ID NO: 30626 |
| iPS:435845 | 21-225_191G1 | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATCTGGAACAGATTTCACTCTCACCATCAGCAGCCTGCAGCCTGAAGATTTTGCAACTTATTACTGCCAACATTATCTTACTTACCCTCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATGAACACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATAGGGGGGTGGGTTACTACGGTTTGGACGTCTGGGGCCAAGGGACCACGGTCACCGTCTCCTCA |
| | | | SEQ ID NO: 26621 | SEQ ID NO: 30627 |

FIGURE 50

| | | | | |
|---|---|---|---|---|
| | | AA | DIQMTQSPSSLSASVGDRVTITCRASQGISNYLAWFQQKPGKAPKSLIYAASSLQSGVPSKFSGSGSGTDFTLTISSLQPEDFATYYCQHYLTYPLTFGGGTKVEIK | QVQLVESGGGVVQPGRSLRLSCAASGFTFSSYGMHWVRQAPGKGLEWVAVIWYDGSNEHYADSVKGRFTISRDNSKNTLYLQMNSLRAEDTAVYYCARDRGVGYYGLDVWGQGTTVTVSS |
| | | | SEQ ID NO: 26622 | SEQ ID NO: 30628 |
| **iPS:435847** | **21-225_191A3** | NA | GAAATTGTGTTGACGCAGTCTCCAGGCACCCTGTCTTTGTCTCCAGGGGAAAGAGCCACCCTCTCCTGCAGGGCCAGTCAGAGTATTCGCAGCAGCTTCTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAATATGGTAACTCACCGTGGGCGTTCGGCCAAGGGACCAAGGTGGAAGTCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGATTACTACTGGAACTGGATCCGCCAGCACCCAGGGAAGGGCCTGGAGTGGATTGGGTACATCTTTTACAGTGGGAGCACCTACTACAACCCGTCCCTCAAGAGTCGAGTTACCATATCAGTAGACACGTCTAAGAACCAGTTCTCCCTGAAGCTGAGCTCTGTGACTGCCGCGGACACGGCCGTGTATTACTGTGCGAGAGGGGATTACGATGGTTCGGGGAGTTATCACTACTACTACGGTATGGACGTCTGGGGCCAAGGGACCACGGTCACCGTCTCCTCA |
| | | | SEQ ID NO: 26623 | SEQ ID NO: 30629 |
| | | AA | EIVLTQSPGTLSLSPGERATLSCRASQSIRSSFLAWYQQKPGQAPRLLIYGASSRATGIPDRFSGSGSGTDFTLTISRLEPEDFAVYYCQQYGNSPWAFGQGTKVEVK | QVQLQESGPGLVKPSQTLSLTCTVSGGSISSGDYYWNWIRQHPGKGLEWIGYIFYSGSTYYNPSLKSRVTISVDTSKNQFSLKLSSVTAADTAVYYCARGDYDGSGSYHYYYGMDVWGQGTTVTVSS |
| | | | SEQ ID NO: 26624 | SEQ ID NO: 30630 |

FIGURE 50

| | | | | |
|---|---|---|---|---|
| iPS:435849 | 21-225_191C3 | NA | GAAATTGTGTTGACGCAGTCTCCAGGCACCCTGTCTTTGTCTCCAGGGGAAAGAGCCACCCTCTCCTGCAGGGCCAGTCAGAGTATTCGCAGCAGCTTCTTAGCCTGGTATCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGGTAACTCACCGTGGGCGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGATTACTACTGGAACTGGATCCGCCAGCTCCCAGGGAAGGGCCTGGAGTGGATTGGGTACATCTTTTACAGTGGGAGCACCTACTACAACCCGTCCCTCAAGAGTCGACTTACCATATCAGTGGACACGTCTAAGAACCAGTTCTCCCTGAAACTGAACTCTGTGACTGCCGCGGACACGGCCGTGTATTACTGTGCGAGAGGGGATTACGATGGTTCGGGGAGTTATCACTTCTACTACGGTATGGACGTCTGGGGCCAAGGGACCACGGTCACCGTCTCCTCA |
| | | | SEQ ID NO: 26625 | SEQ ID NO: 30631 |
| | | AA | EIVLTQSPGTLSLSPGERATLSCRASQSIRSSFLAWYQQKPGQAPRLLIYGASSRATGIPDRFSGSGSGTDFTLTISRLEPEDFAVYYCQQYGNSPWAFGQGTKVEIK | QVQLQESGPGLVKPSQTLSLTCTVSGGSISSGDYYWNWIRQLPGKGLEWIGYIFYSGSTYYNPSLKSRLTISVDTSKNQFSLKLNSVTAADTAVYYCARGDYDGSGSYHFYYGMDVWGQGTTVTVSS |
| | | | SEQ ID NO: 26626 | SEQ ID NO: 30632 |
| iPS:435851 | 21-225_191D3 | NA | GAAATTGTGTTGACGCAGTCTCCAGGCACCCTGTCTTTGTCTCCAGGGGAAAGCGCCACCCTCTCCTGCAGGGCCGGTCAAAGTATTAGAACCAACTTCTTAGCCTGGTACCAGCAGCAGCCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAACAGACTGGAGCCTGAAGATTTTGCAGTGTATTACTGTCAGCAGTATGGTAGCTCACCGTGGACGTTCGGCCAAGGGACCAAGGTGGAAATCAAA | CAGGTGCAACTGAAGGAGTCGGGCCCAGGACTGGTGAAGCCTTCACAGACCCTGTCCCTCACCTGCACTGTCTCTGGTGGCTCCATCAACAGTGGTGATTACTACTGGAACTGGATCCGCCAGCACCCAGGGAAGGGCCTGGACTGGATTGGGTACATCTTTTACAGTGGGAGCACCTACTACAACCCGTCCCTCAAGAGTCGAGTTACCATATCAGTAGACACGTCTAAGAACCAGTTCTCCCTGAAGCTGAGCTCTGTGACTGCCGCGGACACGGCCGTGTATTACTGTGCGAGAGGGGATTACGATGGTTCGGGGAGTTATCACTACTATTACGGTATGGACGTCTGGGGCCAAGGGACCACGGTCACCGTCTCCTCA |
| | | | SEQ ID NO: 26627 | SEQ ID NO: 30633 |

FIGURE 50

| | | | | |
|---|---|---|---|---|
| | | AA | EIVLTQSPGTLSLSPGESATLSCRAGQSIRTNFLAWYQQQPGQAPRLLIYGASSRATGIPDRFSGSGSGTDFTLTINRLEPEDFAVYYCQQYGSSPWTFGQGTKVEIK | QVQLKESGPGLVKPSQTLSLTCTVSGGSINSGDYYWNWIRQHPGKGLDWIGYIFYSGSTYYNPSLKSRVTISVDTSKNQFSLKLSSVTAADTAVYYCARGDYDGSGSYHYYYGMDVWGQGTTVTVSS |
| | | | SEQ ID NO: 26628 | SEQ ID NO: 30634 |
| **iPS:435853** | **21-225_191E3** | NA | GATATTGTAATGACCCAGACTCCACTCTCTCTGTCCGTCACCCCTGGACAGCCGGCCTCCATCTCCTGCAAGTCTAGTCAGAGCCTCCTCCATAGTGATGGAAGGACCTATTTGTATTGGTACCTGCAGAAGCCAGGCCAGCCTCCACAGCTCCTGATCTGTGAGGTTTCCAACCGGTTCGCTGGAGTGACAGATAGGTTCAGTGGCAGCGGGTCAGGGACAGATTTCACACTGAAAATCAGCCGGGTGGAGGCTGGGGATGTTGGGGTTTATTACTGCATGCAAAGTATACAACTTCCCTGGACGTTCGGCCAAGGGACCAAGGTGGAAATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGAAGCTACCACTGGAGCTGGATCCGGCAGCCCGCCGGGAAGGGACTGGAGTGGATTGGACTTATCTATACCAGTAGGAGCACCAATTACAACCCCTCCCTCAAGAGTCGAGTCACCATGTCAGTTGACACGTCCAAGAACCAGTTCTCCCTGAAGCTGAACTCTGTGACCGCCGCGGACACGGCCGTGTATTACTGTGCGAGACTCCGGTATAACTGGAACTTCCCTTACTTTGACTACTGGGGCCAGGGAACCCTGGTCACCGTCTCCTCA |
| | | | SEQ ID NO: 26629 | SEQ ID NO: 30635 |
| | | AA | DIVMTQTPLSLSVTPGQPASISCKSSQSLLHSDGRTYLYWYLQKPGQPPQLLICEVSNRFAGVTDRFSGSGSGTDFTLKISRVEAGDVGVYYCMQSIQLPWTFGQGTKVEIK | QLQLQESGPGLVKPSETLSLTCTVSGGSIRSYHWSWIRQPAGKGLEWIGLIYTSRSTNYNPSLKSRVTMSVDTSKNQFSLKLNSVTAADTAVYYCARLRYNWNFPYFDYWGQGTLVTVSS |
| | | | SEQ ID NO: 26630 | SEQ ID NO: 30636 |

FIGURE 50

| | | | | |
|---|---|---|---|---|
| **iPS:435855** | **21-225_191G3** | NA | GACATCGTGATGACCCAGTCTCCAGACTCCCTGGCTGTGTCTCTGGGCGAGAGGGCCACCATCGACTGTAAGTCCAGCCAGAGTGTTTTACACAGCTCCAACAGTTACAACTACTTAGCTTGGTACCAGCAGAAATTAGGACAGCCTCCTAAACTGCTCATTTACTGGGCATCTACCCGAAAATCCGGGGTCCCTGACCGATTCAGTGGCAGCGGGTCTGGGACAGATTTCACTCTCACCATCAGCAGCCTGCAGGCTGAAGATGTGGCATTTTATTACTGTCAGCAATATTATAGTAGTCCTCCCACTTTCGGCCCTGGGACCAAAATGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGACGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCCCATAGCAGTGGCTGGTACATCTTTGACTACTGGGGCCAGGGAACCCTGGTCACCGTCTCCTCA |
| | | | SEQ ID NO: 26631 | SEQ ID NO: 30637 |
| | | AA | DIVMTQSPDSLAVSLGERATIDCKSSQSVLHSSNSYNYLAWYQQKLGQPPKLLIYWASTRKSGVPDRFSGSGSGTDFTLTISSLQAEDVAFYYCQQYYSSPPTFGPGTKMDIK | QVQLVQSGAEVKKPGASVKVSCKASGYTFTNYDINWVRQATGQGLEWMGRMNPNSGNTGYAQKFQGRVTMTRNTSISTAYMELSSLRSEDTAVYYCAHSSGWYIFDYWGQGTLVTVSS |
| | | | SEQ ID NO: 26632 | SEQ ID NO: 30638 |
| **iPS:435857** | **21-225_191A4** | NA | GACATCCAGATGACCCAGTCTCCATCCTCCCTGTCTGCATCTGTAGGAGACAGAGTCACCATCACTTGCCGGGCAAGTCAGGGCATTAGAAAAGATTTAGGCTGGTATCAGCAGAAACCAGGGAAAGCCCCTAAGCGCCTGATCCATACTGCATCCAGTTTGCAAAATGGGGTCCCATTAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAGTGGGTGGCAGTTATATCATATGATGGAGGTTATAAAAACTATGCAGACTCCGTGAAGGGCCGATTCACCATCTCCAGAGACAATTCCAAGAACACGCTGTATCTGCAAATGAATAGCCTGAGAGCTGAGGACACGGCTGTGTATTACTGTGCGAGAGGTACCCACGGGTACTACTACGGTGTGGACGTCTGGGGCCAAGGGACCACGGTCACCGTCTCCTCA |
| | | | SEQ ID NO: 26633 | SEQ ID NO: 30639 |

FIGURE 50

| | | | | |
|---|---|---|---|---|
| | | AA | DIQMTQSPSSLSASVGDRVTITCRASQGIRKDLGWYQQKPGKAPKRLIHTASSLQNGVPLRFSGSGSGTEFTLTISSLQPEDFATYYCLQHNSYPWTFGQGTKVEIK | QVQLVESGGGVVQPGRSLRLSCAASGFTFSSYGMHWVRQAPGKGLEWVAVISYDGGYKNYADSVKGRFTISRDNSKNTLYLQMNSLRAEDTAVYYCARGTHGYYYGVDVWGQGTTVTVSS |
| | | | SEQ ID NO: 26634 | SEQ ID NO: 30640 |
| iPS:435859 | 21-225_190E6 | NA | GACATCCAGATGACCCAGTCTCCTTCCTCACTGTCTGCATCTGTAGGAGACAGAGTCACCATCACTTGTCGGACGAGTCAGGGCATTGGCAATTATTTAGCCTGGTTTCAGCAGAAACCAGGGAAAGCCCCTAAGTCCCTGCTCTATAAAGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATATGACTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCAGGGCGTGGGCTACGACGGTTTGGACGTCTGGGGCCAAGGGACCACGGTCACCGTCTCCTCA |
| | | | SEQ ID NO: 26635 | SEQ ID NO: 30641 |
| | | AA | DIQMTQSPSSLSASVGDRVTITCRTSQGIGNYLAWFQQKPGKAPKSLLYKASSLQSGVPSKFSGSGSGTDFTLTISSLQPEDFATYYCQQYMTYPLTFGGGTKVEIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAVYYCARDQGVGYDGLDVWGQGTTVTVSS |
| | | | SEQ ID NO: 26636 | SEQ ID NO: 30642 |

FIGURE 50

| | | | | |
|---|---|---|---|---|
| **iPS:435861** | **21-225_190A5** | NA | GACATCCAGATGACCCAGTCTCCATCCTCACT<br>GTCTGCATCTGTAGGAGACAGAGTCACCATCA<br>CTTGTCGGGCGAGTCAGGGGATTGGCAATCAT<br>TTAGCCTGGTTTCAGCAGAAACCAGGGAAAGC<br>CCCTAAGTCCCTGATCCATGCTGCATCCAGTTT<br>GCAAAGTGGGGTCCCATCAAAGTTCAGCGGCA<br>GTGGATCTGGGACAGATTTCACTCTCACCATC<br>AGCAGCCTGCAGCCTGAAGATTTTGCAACTTA<br>TTACTGCCAACAGTATAGTAATTACCCAGTCA<br>CTTTCGGCCCTGGGACCAAAGTGGATATCAAA | CAGGTGCAGTTGGTGGAGTCTGGGGGAGGCGTG<br>GGCCAGCCTGGGAGGTCCCTGAGACTCTCCTGTG<br>CAGCGTCTGGATTCACCTTCAGTAGTTATGGCAT<br>GCACTGGGTCCGCCAGGCTCCAGGCAAGGGGCT<br>GGAGTGGGTGGCAGTTATATGGTATGATGGAAGT<br>AATAAAAACTATGCAGACTCCGTGAAGGGCCGA<br>TTCACCATCTCCAGAGACAATTCCAAGAACACGC<br>TGTATCTGCAAATGAACAGCCTGAGAGCCGAGG<br>ACACGGCTGTGTATTACTGTGCGAGAGATTTCTC<br>TGTAGGGTACGACGGTATGGACGTCTGGGGCCA<br>AGGGACCACGGTCACCGTCTCCTCA |
| | | | SEQ ID NO: 26637 | SEQ ID NO: 30643 |
| | | AA | DIQMTQSPSSLSASVGDRVTITCRASQGIGNHLA<br>WFQQKPGKAPKSLIHAASSLQSGVPSKFSGSGSG<br>TDFTLTISSLQPEDFATYYCQQYSNYPVTFGPGT<br>KVDIK | QVQLVESGGGVGQPGRSLRLSCAASGFTFSSYGMH<br>WVRQAPGKGLEWVAVIWYDGSNKNYADSVKGRF<br>TISRDNSKNTLYLQMNSLRAEDTAVYYCARDFSVG<br>YDGMDVWGQGTTVTVSS |
| | | | SEQ ID NO: 26638 | SEQ ID NO: 30644 |
| **iPS:435863** | **21-225_191H4** | NA | GAAATTGTGCTGACTCAGTCTCCAGACTTTCA<br>GTCTGTGACTCCAAAGGAGAAAGTCACCATCA<br>CCTGCCGGGCCAATCAGAGCATTGGTAGTAGC<br>TTACACTGGTACCAGCAGAAACCAGATCAGTC<br>TCCAAAGCTCCTCATCAAGTATGCTTCCCAGT<br>CCCTCTCAGGGGTCCCCTCGAGGTTCAGTGCC<br>AGTGGATCTGGGACAGATTTCACCCTCACCAT<br>CAATAGCCTGGATGCTGAAGATGCTGCAACGT<br>ATTACTGTCATCAGACTGGTAGGTTACCTCTC<br>ACTTTCGGCGGAGGGACCAAGGTGGAGATCA<br>AA | CAGGTGCAGCTGCAGGAGTCGGGCCCAGGACTG<br>GTGAAGCCTTCACAGACCCTGTCCCTCACCTGCA<br>CTGTCTCTGGTGGCTCCATCAGCAGTGGTGGTTA<br>CTACTGGAACTGGATCCGCCAGCACCCAGGGAA<br>GGGCCTGGAGTGGATTGGGTACATCTTTTACAGT<br>GGGAGCACCTACTACAACCCGTCCCTCAGGAGTC<br>GACTTACCATATCAATAGACACGTCTAAGAACCA<br>GTTCTCCCTGAAGCTGACCTCTGTGACTGCCGCG<br>GACACGGCCGTATATTACTGTGGGAGATCCGGGT<br>ATAACTGGGACAACGGGGTCGACCCCTGGGGCC<br>AGGGAACCCTGGTCACCGTCTCCTCA |
| | | | SEQ ID NO: 26639 | SEQ ID NO: 30645 |

FIGURE 50

| | | | | |
|---|---|---|---|---|
| | | AA | EIVLTQSPDFQSVTPKEKVTITCRANQSIGSSLHWYQQKPDQSPKLLIKYASQSLSGVPSRFSASGSGTDFTLTINSLDAEDAATYYCHQTGRLPLTFGGGTKVEIK | QVQLQESGPGLVKPSQTLSLTCTVSGGSISSGGYYWNWIRQHPGKGLEWIGYIFYSGSTYYNPSLRSRLTISIDTSKNQFSLKLTSVTAADTAVYYCGRSGYNWDNGVDPWGQGTLVTVSS |
| | | | SEQ ID NO: 26640 | SEQ ID NO: 30646 |
| iPS:435865 | 21-225_191A5 | NA | GAAATTGTGTTGACGCAGTCTCCAGGCACCCTGTCTTTGTCTCCAGGGGAAAGAGCCACCCTCTCCTGCAGGGCCAGTCAGAGTGTTAGCAGCAGGTTCTTAGCCTGGTACCAGCAGAAACCTGGCCAGGCTCCCAGGCTCCTCATCTATGGTGCTTCCAACAGGGCCACTGGCATCCCCGACAGGTTCAGTGGCAGTGGGTCTGGGACAGACTTCACTCTCACCATCAGCAGACTGGAGCCTGAAGATTTTGCAGTGTATTACTGTCAGCAGTATGGTGGTTCACCTCCGTGGACGTTCGTCCAAGGGACCAAGGTGGAAATCAAA | GAGATACAGGTGGTGGAGTCTGGGGGAGGCCTGGTCAAGCCTGGGGGGTCCCTGAGACTCTCCTGTGCAGCCTCTGGATTCACCTTCAGAGACTATAGCATGAACTGGGTCCGCCAGGCTCCAGGGAAGGGGCTGGAGTGGGTCTCATCCATTAGTAGTGGTAGTGGTTACATATATTATGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGCTACTATGGCCCTTGACTACTGGGGCCAGGGAGCCCTGGTCACCGTCTCCTCA |
| | | | SEQ ID NO: 26641 | SEQ ID NO: 30647 |
| | | AA | EIVLTQSPGTLSLSPGERATLSCRASQSVSSRFLAWYQQKPGQAPRLLIYGASNRATGIPDRFSGSGSGTDFTLTISRLEPEDFAVYYCQQYGGSPPWTFVQGTKVEIK | EIQVVESGGGLVKPGGSLRLSCAASGFTFRDYSMNWVRQAPGKGLEWVSSISSGSGYIYYADSVKGRFTISRDNAKNSLYLQMNSLRAEDTAVYYCARATMALDYWGQGALVTVSS |
| | | | SEQ ID NO: 26642 | SEQ ID NO: 30648 |

FIGURE 50

| | | | | |
|---|---|---|---|---|
| iPS:435867 | 21-225_191E5 | NA | GAAATTGTGCTGACTCAGTTTCCAGACTCTCA GTCTGTGACTCCAAAGGAGAAAGTCACCATCA CCTGCCGGGCCAGTCAGAGCATTGGTAGTAGC TTACACTGGTACCAGCAGAAACCAGATCAGTC TCCAAAGCTCCTCATCAAGTATGCTTCCCAGT CCTTCTCAGGGGTCCCCTCGAGGTTCAGTGGC AGTGGATCTGGGACAGATTTCACCCTCACCAT CAATAGTCTGGAGGCTGAAGATGCTGCAACGT ATTACTGTCATCAGAGTAGTAGTTTCCCTCGG ACGTTCGGCCAAGGGACCAAGGTGGAAATCA AA | GAGGTGCAGCTGTTGGACTCTGGGGGAGGCTTGG GACAGCCTGGGGGGTCCCTGAGACTCTCCTGTGC AGCCTCTGGATTCACCTTTGACAGCTATGCCATG AACTGGGTCCGCCAGGCTCCAGGGAAGGGGCTG GAGTGGGTCTCAACTATTAGTGGTACTGGTCGTA GGACATACTACGCAGACTCCGTGAAGGGCCGGT TCACCATCTCCAGAGACAATTCCAAGAACACGCT GTATCTGCAAATGAATAGCCTGAGAGCCGAGGA CACGGCCGTATATTACTGTGCGAAAGAGGAGGA TTACTATGATAGTAGTGGCCCGGGGTTCGACCCC TGGGGCCAGGGAACCCTGGTCACCGTCTCCTCA |
| | | | SEQ ID NO: 26643 | SEQ ID NO: 30649 |
| | | AA | EIVLTQFPDSQSVTPKEKVTITCRASQSIGSSLHW YQQKPDQSPKLLIKYASQSFSGVPSRFSGSGSGT DFTLTINSLEAEDAATYYCHQSSSFPRTFGQGTK VEIK | EVQLLDSGGGLGQPGGSLRLSCAASGFTFDSYAMN WVRQAPGKGLEWVSTISGTGRRTYYADSVKGRFTI SRDNSKNTLYLQMNSLRAEDTAVYYCAKEEDYYD SSGPGFDPWGQGTLVTVSS |
| | | | SEQ ID NO: 26644 | SEQ ID NO: 30650 |
| iPS:435869 | 21-225_190B1 | NA | GACATCCAGATGACCCAGTCTCCATCCTCACT GTCTGCATCTGTTGGAGACAGAGTCACCATCA CTTGTCGGGCGAGTCAGGGCATTAGAAATTAT TTAGCCTGGTTTCAGCAGAAACCAGGGAAAGC CCCTAAGTCCCTGATCTATGTTGCATCCAGTTT GGAAAGTGGGGTCCCATCAAAGTTCAGTGGCA GTGGATCTGGGACAGAGTTCACTCTCACCATC AGCAGCCTGCAACCTGAAGATTTTGGAACTTA TTACTGCCAACAGTATCTTAATTACCCAGTCA CTTTCGGCCCTGGGACCAAAGTGGATATCAGA | CAGGTGCAGCTGGTGGAGTCTGGGGGAGGCGTG GTCCAGCCTGGGAGGTCCCTGAGACTCTCCTGTG CAACGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAACATTATGCAGACTCCGTGAAGGGCCGA TTCACCATCTCCAGAGACAATTCCAAGAACACAC TATATCTGCAAATGAACAGCCTGAGAGCCGAGG ACACGGCTGTTTATTACTGTGCGAGAGATAGAAC AGTGGGATACTCCGGTATGGACGTCTGGGGCCA AGGGACCACGGTCACCGTCTCCTCA |
| | | | SEQ ID NO: 26645 | SEQ ID NO: 30651 |

FIGURE 50

| | | | | |
|---|---|---|---|---|
| | | AA | DIQMTQSPSSLSASVGDRVTITCRASQGIRNYLAWFQQKPGKAPKSLIYVASSLESGVPSKFSGSGSGTEFTLTISSLQPEDFGTYYCQQYLNYPVTFGPGTKVDIR | QVQLVESGGGVVQPGRSLRLSCATSGFTFSSYGMHWVRQAPGKGLEWVAVIWYDGSNKHYADSVKGRFTISRDNSKNTLYLQMNSLRAEDTAVYYCARDRTVGYSGMDVWGQGTTVTVSS |
| | | | SEQ ID NO: 26646 | SEQ ID NO: 30652 |
| **iPS:435871** | **21-225_191E6** | NA | GATATTGTGATGACCCAGACTCCACTCTCTCTGTCCGTCACCCCTGGACAGCCGGCCTCCATCTCCTGCAAGTCTAGTCAGAGCCTCCTCCATAGTGATGGAAGGACCTATTTGTATTGGTACCTGCAGAAGCCAGGCCAGCCTCCACAGCTCCTGATCTGTGAGGTTTCCAACCGGTTCGCTGGAGTGACAGATAGGTTCAGTGGCAGCGGGTCAGGGACAGATTTCACACTGAAAATCAGCCGGGTGGAGGCTGGGGATGTTGGGATTTATTACTGCATGCAAAGTATACATTTTCCCTGGACGTTCGGCCAAGGGACCAAGGTGGAAATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGAAGCTACCACTGGAGCTGGATCCGGCAGCCCGCCGGGAAGGGACTGGAGTGGATTGGGCTTATCTATACCAGTAGGAGCACCAATTACAACCCCTCCCTCAAGAGTCGAGTCACCATGTCAGTAGACACGTCCAAGAACCAGTTCTCCCTGAAGCTGAGCTCTGTGACCGCCGCGGACACGGCCGTGTATTACTGTGCGAGACTCCGGTATAACTGGAACTTCCCTTACTTTGACTTCTGGGGCCAGGGAACCCTGGTCACCGTCTCCTCA |
| | | | SEQ ID NO: 26647 | SEQ ID NO: 30653 |
| | | AA | DIVMTQTPLSLSVTPGQPASISCKSSQSLLHSDGRTYLYWYLQKPGQPPQLLICEVSNRFAGVTDRFSGSGSGTDFTLKISRVEAGDVGIYYCMQSIHFPWTFGQGTKVEIK | QLQLQESGPGLVKPSETLSLTCTVSGGSIRSYHWSWIRQPAGKGLEWIGLIYTSRSTNYNPSLKSRVTMSVDTSKNQFSLKLSSVTAADTAVYYCARLRYNWNFPYFDFWGQGTLVTVSS |
| | | | SEQ ID NO: 26648 | SEQ ID NO: 30654 |

FIGURE 50

| | | | | |
|---|---|---|---|---|
| **iPS:435873** | **21-225_190G4** | NA | GACATCCAGATGACCCAGTCTCCATCCTCACTGTCTGCATCTGTAGGAGACAGAGTCACCATCACTTGTCGGGCGAGTCAGGACATTGGCAGATATTTAGCCTGGTTTCAGCAGAAGCCAGGGAAAGCCCCTAAGTCCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAAATTTTGCAACTTATTACTGCCAACAATATAGTACTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTACGCAGACTCCGTGAAGGGCCGATTCACCATCTCCAGAGACAATTCCAAGAACACGCTGTATCTGCAAATGAACAGCCTGAGAGCCGAGGACACGGCTATGTATTACTGTGCGAGAGATCAGGGCGTGGGCTACGACGGTTTGGACGTCTGGGGCCAAGGGACCTCGGTCACCGTCTCCTCA |
| | | | SEQ ID NO: 26649 | SEQ ID NO: 30655 |
| | | AA | DIQMTQSPSSLSASVGDRVTITCRASQDIGRYLAWFQQKPGKAPKSLIYAASSLQSGVPSKFSGSGSGTDFTLTISSLQPENFATYYCQQYSTYPLTFGGGTKVEIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAMYYCARDQGVGYDGLDVWGQGTSVTVSS |
| | | | SEQ ID NO: 26650 | SEQ ID NO: 30656 |
| **iPS:435875** | **21-225_190B9** | NA | GACATCCAGATGACCCAGTCTCCATCTTCCGTGTCTGCATCTGTAGGAGACAGAGTCACCATCACTTGTCGGGCGAGTCAGGGTATTAACAACTGGTTAGCCTGGTATCAGCAGAAACCAGGGAAAGCCCCTAAGCTCCTGATCTATGGTGTTTCCAGTTTGCAGAGTGGGGTCCCATCAAGGTTCAGCGGCAGTGGATCTGGGACAGATTTCACTCTCACCATCAGCAGCCTGCAGCCTGAAGATTTTGCAACTTACTCTTGTCAACAGGCTAACAGTTTCCCGTGGACGTTCGGCCAAGGGACCAAGGTGGAAATCAGA | GAGGTGCAGCTGTTGGAGTCTGGGGGAGGCTTGGTACAGCCTGGGGGGTCCCTGAGACTCTCCTGTGCAGCCTCTGGATTCACCTTTAGCACCTATGCCATGAGTTGGGTCCGCCAGGCTCCAGGGAAGGGGCTGGAGTGGGTCTCAGCTATTAGTCGTAGTGGTGGTAACACACACTACGCAGACTCCGTGAAGGGCCGGTTCACCATCTCCAGAGACAATTCCAAGAATACGCTGTATCTGCAAATGAACAGCCTGAGAGCCGAGGACACGGCCGTATATTCCTGTGCGAAAGATGGATTCGGTGGGAGCTCCTACTTTGACTACTGGGGCCAGGGAACCCTGGTCACCGTCTCCTCA |
| | | | SEQ ID NO: 26651 | SEQ ID NO: 30657 |

FIGURE 50

| | | | | |
|---|---|---|---|---|
| | | AA | DIQMTQSPSSVSASVGDRVTITCRASQGINNWLA WYQQKPGKAPKLLIYGVSSLQSGVPSRFSGSGS GTDFTLTISSLQPEDFATYSCQQANSFPWTFGQG TKVEIR | EVQLLESGGGLVQPGGSLRLSCAASGFTFSTYAMS WVRQAPGKGLEWVSAISRSGGNTHYADSVKGRFTI SRDNSKNTLYLQMNSLRAEDTAVYSCAKDGFGGS SYFDYWGQGTLVTVSS |
| | | | SEQ ID NO: 26652 | SEQ ID NO: 30658 |
| **iPS:435877** | **21-225_184E7** | NA | GACATCCAGATGACCCAGTCTCCATCCTCACG GTCTGCATCTATAGGAGAGAGAGTCACCATCA CTTGTCGGGCGAGTCAGGGCATTAGCAATTAT TTAGCCTGGATTCAGCAGAAACCAGGGACAGC CCCTAAGTCCCTTATTTATGCTGCATCCAGTTT GCAAAGTGGGGTTTCATCAAGGTTTAGCGGCA GTGGATTTGGGACAGATTTCACTATCACCATC AGTAGTGTGCAGCGTGAAGATTTTGCAACTTA TTACTGCCAACAGTATAATGGTTACCCATTCA CTTTCGGCCATGGGACCAAAGTGGATATCAAA | CAGGTGCAGCTGGTGCAGTCTGGGGCTGAAGTG AAGACGCCTGGGGCCTCAGTGAAGGTCTCCTGCA GGGCTTCTGGATACACCTTCACCAGCTACAATAT GCACTGGGTGCGACAGGTCCCTGGACAAGGGCT TGAGTGGATGGGATGGATCAACCCTAACAATGGT GGCTCAAACTATACACAGAAGTTTCAGGGCAGG GTCACCATGACCAGGGACACGTCCATCAGCACA GCCTACATGGAGCTGAGCAGGCTGAGATCTGAC GACACGGCCGTGTATTACTGTGCGAGAAAGTTTG GGGACTGGGGCCAGGGAACCCTGGTCACCGTCT CCTCA |
| | | | SEQ ID NO: 26653 | SEQ ID NO: 30659 |
| | | AA | DIQMTQSPSSRSASIGERVTITCRASQGISNYLAW IQQKPGTAPKSLIYAASSLQSGVSSRFSGSGFGTD FTITISSVQREDFATYYCQQYNGYPFTFGHGTKV DIK | QVQLVQSGAEVKTPGASVKVSCRASGYTFTSYNM HWVRQVPGQGLEWMGWINPNNGGSNYTQKFQGR VTMTRDTSISTAYMELSRLRSDDTAVYYCARKFGD WGQGTLVTVSS |
| | | | SEQ ID NO: 26654 | SEQ ID NO: 30660 |

FIGURE 50

| | | | | |
|---|---|---|---|---|
| **iPS:435879** | **21-225_184H10** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATATTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGTAGCGTCTGGATTCACCTTCAGTGACTATGGCATGCACTGGGTCCGCCAGGCTCCAGGCAAGGGGCTGGAGTGGGTGGCAGTTATTTGGTATGATGAAACTAATAAACACTATGGAGACTCCGTGAAGGGCCGATTCACCATCTCCAGAGACAATTCCAAGGACACGCTGTATCTGCAAATGAACAGCCTGAGAGCCGAGGACACGGCTGTGTATTACTGTGCGAGAGAGGTTGGCTGGCACGATGACTATTGGGGCCAGGGAACCCTGGTCACCGTCTCCTCA |
| | | | SEQ ID NO: 26655 | SEQ ID NO: 30661 |
| | | AA | DIQMTQSPSSLSASVGDRVTITCRASQGIRNDLGWYQQKPGKAPKRLIYIASSLQSGVPSRFSGSGSGTEFTLTISSLQPEDFATYYCLQHNSYPFTFGPGTKVDIK | QVQLVESGGGVVQPGRSLRLSCVASGFTFSDYGMHWVRQAPGKGLEWVAVIWYDETNKHYGDSVKGRFTISRDNSKDTLYLQMNSLRAEDTAVYYCAREVGWHDDYWGQGTLVTVSS |
| | | | SEQ ID NO: 26656 | SEQ ID NO: 30662 |
| **iPS:435881** | **21-225_184D11** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATATTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATTTGGTATGATGAAACTAATAAACACTATGGAGACTCCGTGAAGGGCCGATTCACCATCTCCAGAGACAATTCCAAGGACACGCTGTATCTGCAAATGAACAGCCTGAGAGCCGAGGACACGGCTGTGTATTACTGTGCGAGAGAGGTTGGCTGGCACGATGACTATTGGGGCCAGGGAACCCTGGTCACCGTCTCCTCA |
| | | | SEQ ID NO: 26657 | SEQ ID NO: 30663 |

FIGURE 50

| | | | | |
|---|---|---|---|---|
| | | AA | DIQMTQSPSSLSASVGDRVTITCRASQGIRNDLG<br>WYQQKPGKAPKRLIYIASSLQSGVPSRFSGSGSG<br>TEFTLTISSLQPEDFATYYCLQHNSYPFTFGPGTK<br>VDIK | QVQLVESGGGVVQPGRSLRLSCAASGFTFSDYGM<br>HWVRQAPGKGLEWVAVIWYDETNKHYGDSVKGR<br>FTISRDNSKDTLYLQMNSLRAEDTAVYYCAREVG<br>WHDDYWGQGTLVTVSS |
| | | | SEQ ID NO: 26658 | SEQ ID NO: 30664 |
| iPS:435883 | 21-225_185A1 | NA | GACATCCAGATGACCCAGTCTCCATCCTCACT<br>GTCTGCATCTGTAGGAGACAGAGTCACCATCA<br>CTTGTCGGGCGAGTCAGGGCATTAGCAATTAT<br>TTAGCCTGGTTTCAGCAGACACCAGGGAAAGC<br>CCCTAAGTCCCTGATCTCTGTTGCATCCAGTTT<br>GCAAAGTGGGGTCCCATCAAGATTCAGCGCCA<br>GTGGATCTGGGACAGATTTCACTCTCACCATC<br>AGCAGCCTGCAGCCTGAAGATTTTGCAACTTA<br>TTACTGCCGACAATATCATAGTTACCCATTCA<br>CTTTCGGCCCTGGGACCAAAGTGGATATCAAA | GAGGTGCAGCTGGTGGAGTCTGGGGGAGGCCTG<br>GTCAAGCCTGGGGGGTCCCTGAGACTCTCCTGTG<br>CAGCCTCTGGATTCACCTTCAATAGCTATAGCAT<br>GAACTGGGTCCGCCAGGCTCCAGGGAAGGGGCT<br>GGAGTGGGTCTCATCCATTAGCAGTAGTGGTAGT<br>TACATATATTACGCAGACTCAGTGAAGGGCCGAT<br>TCACCATCTCCAGAGACAACGCCAAGAACTCACT<br>GTATCTGCAAATGCACAGCCTGAGAGCCGAGGA<br>CACGGCTGTGTATTACTGTGCGAGAAGCAACCTT<br>TTTGACTGCTGGGGCCAGGGAACCCCGGTCACCG<br>TCTCCTCA |
| | | | SEQ ID NO: 26659 | SEQ ID NO: 30665 |
| | | AA | DIQMTQSPSSLSASVGDRVTITCRASQGISNYLA<br>WFQQTPGKAPKSLISVASSLQSGVPSRFSASGSG<br>TDFTLTISSLQPEDFATYYCRQYHSYPFTFGPGTK<br>VDIK | EVQLVESGGGLVKPGGSLRLSCAASGFTFNSYSMN<br>WVRQAPGKGLEWVSSISSSGSYIYYADSVKGRFTIS<br>RDNAKNSLYLQMHSLRAEDTAVYYCARSNLFDCW<br>GQGTPVTVSS |
| | | | SEQ ID NO: 26660 | SEQ ID NO: 30666 |

FIGURE 50

| | | | | |
|---|---|---|---|---|
| **iPS:435885** | **21-225_185E10** | NA | GACATCCAGATGACCCAGTCTCCATCCTCACGGTCTGCATCTATAGGAGAGAGAGTCACCATCACTTGTCGGGCGAGTCAGGGCATTAGCAATTATTTAGCCTGGATTCAGCAGAAACCAGGGACAGCCCCTAAGTCCCTTATCTATGCTGCATCCAGTTTGCAAAGTGGGGTTTCATCAAGGTTTAGCGGCAGTGGATTTGGGACAGATTTCACTCTCACCATCAGTAGTCTGCAGCCTGAAGATTTTGCAACTTATTACTGCCAACAGTATAATGGTTACCCATTCACTTTCGGCCCTGGGACCAAAGTGGATATCAAA | CAGGTGCAGCTGGTGCAGTCTGGGGCTGAAGTGAAGACGCCTGGGGCCTCAGTGAAGGTCTCCTGCAGGGCTTCTGGATACACCTTCACCAGCTACAATATGCACTGGGTGCGACAGGTCCCTGGACAAGGGCTTGAGTGGATGGGATGGATCAACCCTAACAATGGTGGCTCAAACTATACACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGAAAGTTTGGGGACTGGGGCCAGGGAACCCTGGTCACCGTCTCCTCA |
| | | | SEQ ID NO: 26661 | SEQ ID NO: 30667 |
| | | AA | DIQMTQSPSSRSASIGERVTITCRASQGISNYLAWIQQKPGTAPKSLIYAASSLQSGVSSRFSGSGFGTDFTLTISSLQPEDFATYYCQQYNGYPFTFGPGTKVDIK | QVQLVQSGAEVKTPGASVKVSCRASGYTFTSYNMHWVRQVPGQGLEWMGWINPNNGGSNYTQKFQGRVTMTRDTSISTAYMELSRLRSDDTAVYYCARKFGDWGQGTLVTVSS |
| | | | SEQ ID NO: 26662 | SEQ ID NO: 30668 |
| **iPS:435887** | **21-225_186F7** | NA | GATGTTGTGATGGCCCAGACTCCACTCTCTCTGTCCGTCACCCCTGGACAGCCGGCCTCCATCTCCTGCAAGTCTAGTCAGAGCCTCCTGCATGGTGATGGAAAGACCTATTTGTGTTGGTACCTCCAGAAGCCAGGCCAGCCTCCACAGCTCCTGATCTATGAAGTTTCCAAGCGGTTCTCTGGAGTGCCAGATAGGTTCAGTGGCAGCGGGTCAGGGACAGATTTCACACTGAAAATCAGCCGGGTGGAGGCTGAGGATGTTGGGGTCTATTACTGCATGCAAAGTATACAGGTTCCCTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTACCTATGGCATGCACTGGGTCCGCCAGGCTCCAGGCAAGGGGCTGGAGTGGGTGGCAATTATATGGTATGATGGAAGTTATAAATATTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCATTACGATTTTTGGAGTGGGCACTTTGACTACTGGGGCCAGGGAACCCTGGTCACCGTCTCCTCA |
| | | | SEQ ID NO: 26663 | SEQ ID NO: 30669 |

FIGURE 50

| | | | | |
|---|---|---|---|---|
| | | AA | DVVMAQTPLSLSVTPGQPASISCKSSQSLLHGDGKTYLCWYLQKPGQPPQLLIYEVSKRFSGVPDRFSGSGSGTDFTLKISRVEAEDVGVYYCMQSIQVPWTFGQGTKVEIK | QVQLVESGGGVVQPGRSLRLSCAASGFTFSTYGMHWVRQAPGKGLEWVAIIWYDGSYKYYADSVKGRFTISRDNSKNTLYLQMNSLRAEDTAVYYCARDHYDFWSGHFDYWGQGTLVTVSS |
| | | | SEQ ID NO: 26664 | SEQ ID NO: 30670 |
| iPS:435889 | 21-225_186A11 | NA | GACATCCAGATGACCCAGTCTCCATCTTCCGTGTCTGCATCTGTTGGAGACAGAGTCACCATCACTTGTCGGGCGAGTCAGGATATTACCAGCTGGTTAGCCTGGTATCAGCAGAAACCAGGGAAAGCCCCTAAACTCCTGATCTATGCTGCATCCAGTTTACAAAGTGGGGTCCCATCAAGGTTCAGCGGCAGTGGATCTGGGACAGATTTCATTCTCACCATCACCAGCGTGCAGCCTGATGATTTTGCAACTTACTATTGTCAACAGGTTAACAGTTTCCCATTCACTTTCGGCCATGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGTTGGGTCCGCCAGGCTCCAGGGAAGGGGCTGGAGTGGGTCTCAGTTATTAGTGGTAGAGGTGGTACCACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAACGGACTGGGGATGATGTTTTTGATATCTGGGGCCAAGGGACAATGGTCACCGTCTCTTCA |
| | | | SEQ ID NO: 26665 | SEQ ID NO: 30671 |
| | | AA | DIQMTQSPSSVSASVGDRVTITCRASQDITSWLAWYQQKPGKAPKLLIYAASSLQSGVPSRFSGSGSGTDFILTITSVQPDDFATYYCQQVNSFPFTFGHGTKVDIK | EVQLLESGGGLVQPGGSLRLSCAASGFTFSSYAMSWVRQAPGKGLEWVSVISGRGGTTFYADSVKGRFTISRDNSKNTLYLQMNSLRAEDTAVYYCAKRTGDDVFDIWGQGTMVTVSS |
| | | | SEQ ID NO: 26666 | SEQ ID NO: 30672 |

FIGURE 50

| | | | | |
|---|---|---|---|---|
| iPS:435891 | 21-225_188H5 | NA | GACATCCAGATGACCCAGTCTCCATCCTCACTGTCTGCATCTATAGGAGAGAGAGTCACCATCACTTGTCGGGCGAGTCAGGGCATTAGCAATTATTTAGCCTGGCTTCAGCAGAAACCAGGGACAGCCCCTAAGTCCCTGATCTATGCTGCTTCCAGTTTGCAAAGTGGGGTCTCATCAAGGTTCAGCGGCAGTGGATTTGGGACAGATTTCACTCTCACCATCAGTAGTCTGCAGCCTGAAGATTTTGCAACTTATTACTGCCAACAGTATAATAGTTATCCATTCACTTTCGGCCCTGGGACCAAAGTGGATATCAAA | CAGGTGCAGCTGGTGCAGTCTGGGGCTGAGGTGAAGACGCCTGGGGCCTCAGTGAAGGTCTCCTGCAGGGCTTCTGGATACACCTTCACCAGCTACAATATGCACTGGGTACGACAGGTCCCTGGACAAGGGCTTGAGTGGATGGGATGGATCAACCCTAACAGTGGTGGCTCAAACTATACACAGAAGTTTCAGGGCAGGATCACCATGACCAGGGACACGTCCATCAGCACAGCCTACATGGAGCTGAGCAGGCTGAGATCTGACGACACGGCCGTATATTACTGTGCGAGAAAGTTTGGGGACTGGGGCCAGGGAACCCTGGTCACCGTCTCCTCA |
| | | | SEQ ID NO: 26667 | SEQ ID NO: 30673 |
| | | AA | DIQMTQSPSSLSASIGERVTITCRASQGISNYLAWLQQKPGTAPKSLIYAASSLQSGVSSRFSGSGFGTDFTLTISSLQPEDFATYYCQQYNSYPFTFGPGTKVDIK | QVQLVQSGAEVKTPGASVKVSCRASGYTFTSYNMHWVRQVPGQGLEWMGWINPNSGGSNYTQKFQGRITMTRDTSISTAYMELSRLRSDDTAVYYCARKFGDWGQGTLVTVSS |
| | | | SEQ ID NO: 26668 | SEQ ID NO: 30674 |
| iPS:435895 | 21-225_188E8 | NA | GACATCCAGATGACCCAGTCTCCATCTTCCGTGTCTGCATCTGTAGGAGACAGAGTCACCATCACTTGTCGGGCGAATCAGGATATTTCCAGCTGGTTAGCCTGGTATCAGCAGAAACCAGGGAAAGCCCCTAAGCTCCTGATCTATGCTGCATCCAATTTGCAAAGTGGGGTCCCATCAGGGTTCAGCGGCAGTGGATCTGGGACAGATTTCACTCTCACCATCAGCAGCCTGCAGCCTGAAGATTTTGCAACTTACTATTGTCAGCAGGCTAACAGTTTCCCGTGGACGTTCGGCCAAGGGACCAAGGTGGAAATCAAG | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTCTGCCATGAACTGGGTCCGCCAGGCTCCAGGGAAGGGGCTGGAGTGGGTCTCAGTTATTAGTGGTAGTGGTGGTTACACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTTCTGTGCGAAAAGGAACACCGATGATGCTTTTGATATCTGGGGCCAAGGGACAATGGTCACCGTCTCTTCA |
| | | | SEQ ID NO: 26669 | SEQ ID NO: 30675 |

FIGURE 50

| | | | | |
|---|---|---|---|---|
| | | AA | DIQMTQSPSSVSASVGDRVTITCRANQDISSWLAWYQQKPGKAPKLLIYAASNLQSGVPSGFSGSGSGTDFTLTISSLQPEDFATYYCQQANSFPWTFGQGTKVEIK | EVQLLESGGGLVQPGGSLRLSCAASGFTFSSSAMNWVRQAPGKGLEWVSVISGSGGYTYYADSVKGRFTISRDNSKNTLYLQMNSLRAEDTAVYFCAKRNTDDAFDIWGQGTMVTVSS |
| | | | SEQ ID NO: 26670 | SEQ ID NO: 30676 |
| **iPS:435897** | **21-225_188B9** | NA | GACATCCAGATGACCCAGTCTCCATCCTCACTGTCTGCATCTATAGGAGAGAGAGTCACCATCACTTGTCGGGCGAGTCAGGGCATTAGCAATTATTTAGCCTGGCTTCAGCAGAAACCAGGGACAGCCCCTAAGTCCCTGATCTATGCTGCATCCAGTTTGCAAAGTGGGGTCTCATCAAGGTTCAGCGGCAGTGGATTTGGGACAGATTTCACTCTCACCATCAGTAGTCTGCAGCCTGAAGATTTTGCAACTTATTACTGCCAACAGTATAATAGTTACCCATTCACTTTCGGCCCTGGGACCAAAGTGGATATCAAA | CAGGTGCAGCTGGTGCAGTCTGGGGCTGAGGTGAAGACGCCTGGGGCCTCAGTGAAGGTCTCCTGCAGGGCTTCTGGATACACCTTCACCAGCTACAATATGCACTGGGTGCGACAGGTCCCTGGACAAGGGCTTGAGTGGATGGGATGGATCAACCCTAACAGTGGTGGCTCAAACTATACACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGAAAGTTTGGGGACTGGGGCCAGGGAACCCTGGTCACCGTCTCCTCA |
| | | | SEQ ID NO: 26671 | SEQ ID NO: 30677 |
| | | AA | DIQMTQSPSSLSASIGERVTITCRASQGISNYLAWLQQKPGTAPKSLIYAASSLQSGVSSRFSGSGFGTDFTLTISSLQPEDFATYYCQQYNSYPFTFGPGTKVDIK | QVQLVQSGAEVKTPGASVKVSCRASGYTFTSYNMHWVRQVPGQGLEWMGWINPNSGGSNYTQKFQGRVTMTRDTSISTAYMELSRLRSDDTAVYYCARKFGDWGQGTLVTVSS |
| | | | SEQ ID NO: 26672 | SEQ ID NO: 30678 |

FIGURE 50

| | | | | |
|---|---|---|---|---|
| iPS:435899 | 21-225_188G11 | NA | GATATTGTGATGACCCAGACTCCACTCTCTCT GTCCGTCACCCCTGGACAGCCGGCCTCCATCT CCTGCATGTCTAGTCAGAGCCTCCTGCATGGT GATGGAAAGACCTATTTATATTGGTACCTGCA GAAGCCCGGCCAGCCTCCACAGCTCCTGATCT ATGAAGTTTCCAACCGGTTCTCTGGAGTGCCA GATACGTTCAGTGGCAGCGGGTCAGGGACAG ATTTCACACTGAAAATCAGCCGGGTGGAGGCT GAGGATGTTGGGGTTTATTACTGCATGCAAAG TATACAGATTCCTTGGACGTTCGGCCAAGGGA CCAAGGTGGAAATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGTTATGGCAT GCACTGGGTCCGCCAGGCTCCAGGCAAGGGGCT GGAGTGGGTGACAATTATATGGTATGATGGAAGT TATAAATACTATGCAGACTCCGTGAAGGGCCGAT TCACCATCTCCAGAGACAATTCCAAGAACACGCT GTTTCTGCAAATGAACAGCCTGAGAGCCGAAGA CACGGCTGTGTATTACTGTGCGAGAGAGAGATAC GATTTTTGGAGTGGTCATTTTGACTACTGGGGCC AGGGAACCCTGGTCACCGTCTCCTCA |
| | | | SEQ ID NO: 26673 | SEQ ID NO: 30679 |
| | | AA | DIVMTQTPLSLSVTPGQPASISCMSSQSLLHGDG KTYLYWYLQKPGQPPQLLIYEVSNRFSGVPDTFS GSGSGTDFTLKISRVEAEDVGVYYCMQSIQIPWT FGQGTKVEIK | QVQLVESGGGVVQPGRSLRLSCAASGFTFSSYGMH WVRQAPGKGLEWVTIIWYDGSYKYYADSVKGRFT ISRDNSKNTLFLQMNSLRAEDTAVYYCARERYDF WSGHFDYWGQGTLVTVSS |
| | | | SEQ ID NO: 26674 | SEQ ID NO: 30680 |
| iPS:435901 | 21-225_189G2 | NA | GATATTGTGATGACCCAGACTCCACTCTCTCT GTCCGTCACCCCTGGACAGCCGGCCTCCATCT CCTGCAAGTCTAGTCAGAGCCTCCTGCATGGT GATGGAAAGACCTATTTGTTTTGGTACCTGCA GAAGCCAGGCCAGCCTCCACAACTCCTGATCT ATGAAGTTTCCAACCGGTTCTCTGGAGTGTCA GATAGGTTCAGTGGCAGCGGGTCAGGGACAG ATTTCACACTGAAAATCAGCCGGGTGGAGGCT GAGGATGTTGGGGTTTACTACTGCATGCAAAG TATACAGATTCCGTGGACGTTCGGCCAAGGGA CCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAATTATATGGTATGATGGAAGT TATAAATACTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTATTGTGCGAGAGATCGATTC GATTTTTGGAGTGGTTATTCCGACTACTGGGGCC AGGGAACCCTGGTCACCGTCTCCTCA |
| | | | SEQ ID NO: 26675 | SEQ ID NO: 30681 |

FIGURE 50

| | | | | |
|---|---|---|---|---|
| | | AA | DIVMTQTPLSLSVTPGQPASISCKSSQSLLHGDGKTYLFWYLQKPGQPPQLLIYEVSNRFSGVSDRFSGSGSGTDFTLKISRVEAEDVGVYYCMQSIQIPWTFGQGTKVEIK | QVQLVESGGGVVQPGRSLRLSCAASGFTFSNYGMHWVRQAPGKGLEWVAIIWYDGSYKYYADSVKGRFTISRDNSKNTLYLQMNSLRAEDTAVYYCARDRFDFWSGYSDYWGQGTLVTVSS |
| | | | SEQ ID NO: 26676 | SEQ ID NO: 30682 |
| iPS:435903 | 21-225_190E2 | NA | GACATCGTGATGACCCAGTCTCCAGACTCCCTGGCTGTGTCTCTGGGCGAGAGGGCCACCATCAACTGCAAGTCCAGCCAGAGTGTTTTATTCAACTCCAACAATAAGAACTACTTAGCTTGGTACCAGCAGAAACCAGGTCAGCCTCCTAACCTGCTCATTTACTGGGCATCTACCCGGGAATCCGGGGTCCCTGACCGATTCAGTGGCAGCGGGTCTGGGACAGATTTCACTCTCACCATCAGCAGCATGCAGGCTGAAGATGTGGCAGTTTATTACTGTCAGCAATATTGTAGTCTTCCATTCACTTTCGGCCCTGGGACCAAAGTGGATATCAGA | CAGGTGCAACTGGTGGAGTCTGGGGGAGGCTTGGTCAAGCCTGGAGGGTCCCTGAGACTCTCCTGTGCAGCCTCTGGATTCACCTTCAGTGACTACTACATGAGCTGGATCCGCCAGGCTCCAGGGAAGGGGCTGGAGTGGCTTTCATACATTAGTAGTAGTGGTACTACCGTATTCTACGCAGACTCTGTGAAGGGCCGATTCACCATCTCCAGGGACAACGCCAAGAACTCACTGTATCTGCAAATGAACAGCCTGAGAGCCGAGGACACGGCCGTGTATTACTGTGCGAGAGAATGGGTGGGAGCCGACTACTGGGGCCAGGGAACCCTGGTCACCGTCTCCTCA |
| | | | SEQ ID NO: 26677 | SEQ ID NO: 30683 |
| | | AA | DIVMTQSPDSLAVSLGERATINCKSSQSVLFNSNNKNYLAWYQQKPGQPPNLLIYWASTRESGVPDRFSGSGSGTDFTLTISSMQAEDVAVYYCQQYCSLPFTFGPGTKVDIR | QVQLVESGGGLVKPGGSLRLSCAASGFTFSDYYMSWIRQAPGKGLEWLSYISSSGTTVFYADSVKGRFTISRDNAKNSLYLQMNSLRAEDTAVYYCAREWVGADYWGQGTLVTVSS |
| | | | SEQ ID NO: 26678 | SEQ ID NO: 30684 |

FIGURE 50

| | | | | |
|---|---|---|---|---|
| **iPS:435905** | **21-225_190A3** | NA | GAAATTATGTTGACGCAGTCTCCAGGCACCCTGTCTTTGTCTCCAGGGGAAAGAGCCACCCTCTCCTGCAGGGCCAGTCAGAATATAAGGAGCAACTTCTTAGCCTGGTACCAGCAGAAACCTGGCCAGGCTCCCAGGCTCCTCATCTATGGTGCATCCAGCAGGGCCACTGGCATCCCAGACAGATTCAGTGGCAGTGTGTCTGGGACAGACTTCACTCTCACCATCAACAGACTGGAGCCTGAAGATTTTGCGGTGTATTACTGTCAGCAGTATGGTAACTCACCGTGGACGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGGCTCCATCAACAGTGGTGGTTACTACTGGAACTGGATCCGCCAGCACCCAGGGAAGGGCCTGGAGTGGATTGGGTTCATCTTTTATAGTGGGAGCACCTACTACAACCCGTCCCTCAAGAGTCGAGTTACCATATCAGTAGACACGTCTAAGAACCAGTTCTCCCTGAAGCTGAACTCTGTGACTGCCGCGGACACGGCCGTGTATTACTGTGCGAGAGGGGATTACGATGGTTCGGGGAGTTATCACTACTATTACGGTATGGACGTCTGGGGCCAAGGGACCACGGTCACCGTCTCCTCA |
| | | | SEQ ID NO: 26679 | SEQ ID NO: 30685 |
| | | AA | EIMLTQSPGTLSLSPGERATLSCRASQNIRSNFLAWYQQKPGQAPRLLIYGASSRATGIPDRFSGSVSGTDFTLTINRLEPEDFAVYYCQQYGNSPWTFGQGTKVEIK | QVQLQESGPGLVKPSQTLSLTCTVSGGSINSGGYYWNWIRQHPGKGLEWIGFIFYSGSTYYNPSLKSRVTISVDTSKNQFSLKLNSVTAADTAVYYCARGDYDGSGSYHYYYGMDVWGQGTTVTVSS |
| | | | SEQ ID NO: 26680 | SEQ ID NO: 30686 |
| **iPS:435907** | **21-225_190G3** | NA | GACATCCAGATGACCCAGTCTCCATCCTCCCTGTCTGCATCTGTAAGAGACAGAGTCACCATCACTTGCCGGGCAAGTCAGGGCATTAGAAAAGATTTAGGCTGGTATCAGCAGAAACCAGGGAAAGCCCCTAAGCGCCTGATCCATACTGCATCCAGTTTGCAAAGTGGGGTCCCATTAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAGTGGGTGGCAGTTATATCATATGATGGAGGTTATAAAAACTATGTAGACTCCGTGAAGGGCCGATTCACCATCTCCAGAGACAATTCCAAGAACACGCTGTATCTGCAAATGAATAGCCTGAGAGCTGAGGACACGGCTGTGTATTACTGTGCGAGAGGTACCCACGGGTACTACTACGGTGTGGACGTCTGGGGCCAAGGGACCACGGTCACCGTCTCCTCA |
| | | | SEQ ID NO: 26681 | SEQ ID NO: 30687 |

FIGURE 50

| | | | | |
|---|---|---|---|---|
| | | AA | DIQMTQSPSSLSASVRDRVTITCRASQGIRKDLGWYQQKPGKAPKRLIHTASSLQSGVPLRFSGSGSGTEFTLTISSLQPEDFATYYCLQHNSYPWTFGQGTKVEIK | QVQLVESGGGVVQPGRSLRLSCAASGFTFSSYGMHWVRQAPGKGLEWVAVISYDGGYKNYVDSVKGRFTISRDNSKNTLYLQMNSLRAEDTAVYYCARGTHGYYYGVDVWGQGTTVTVSS |
| | | | SEQ ID NO: 26682 | SEQ ID NO: 30688 |
| iPS:435909 | 21-225_190H3 | NA | GACATCCAGATGACCCAGTCTCCATCTTCCGTGTCTGCATCTGTAGGAGACAGAGTCACCATCACTTGTCGGGCGAGTCAGGGTCTTAACAACTGGTTAGCCTGGTATCAGCTGAAACCAGGGAAAGCCCCTAAGCTCCTGATCTATGCTGTGTCCAGTTTGCAAAGTGGGGTCCCATCAAGGTTCAGCGGCAGTGGATCTGGGTCAGAGTTCACTCTCACCATCAGCAGCCTGCAGCCTGAAGATTTTGCAACTTACCATTGTCAACAGGCTAACAGTCTCCCGTGGACGTTCGGCCAAGGGACCAAGGTGGAAATCAAA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTCGTGGTGGTAACACATACTACGCAGACTCCGTGAAGGGCCGGTTCACCATCTCCAGAGACAATTCCAAGAACACGCTGTATCTGCAAATGAACAGTTTGAGAGCCGAGGACACGGCCGTATATTACTGTGCGAAAGATGGATTCGGTGGGAGCTCCTATTTTGACTACTGGGGCCAGGGAACCCTGGTCACCGTCTCCTCA |
| | | | SEQ ID NO: 26683 | SEQ ID NO: 30689 |
| | | AA | DIQMTQSPSSVSASVGDRVTITCRASQGLNNWLAWYQLKPGKAPKLLIYAVSSLQSGVPSRFSGSGSGSEFTLTISSLQPEDFATYHCQQANSLPWTFGQGTKVEIK | EVQLLESGGGLVQPGGSLRLSCAASGFTFSSYAMSWVRQAPGKGLEWVSAISGRGGNTYYADSVKGRFTISRDNSKNTLYLQMNSLRAEDTAVYYCAKDGFGGSSYFDYWGQGTLVTVSS |
| | | | SEQ ID NO: 26684 | SEQ ID NO: 30690 |

FIGURE 50

| | | | | |
|---|---|---|---|---|
| **iPS:435911** | **21-225_190B4** | NA | GAAATTGTGTTGACGCAGTCTCCAGGCACCCTGTCTTTGTCTCCAGGGGAAAGAGCCACACTCTCCTGCAGGGCCAGTCAGAGTATTCGCAGCAGCTTCTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGGTAACTCACCGTGGGCGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGATTACTACTGGAACTGGATCCGCCAGCACCCAGGGAAGGGCCTGGAGTGGATTGGGTACATCTTTTACAGTGGGAGCACCTACTACAACCCGTCCCTCAAGAGTCGAGTTACCATATCAGTAGACACGTCTAAGAACCAGTTCTCCCTGAAGCTGAGCTCTGTGACTGCCGCGGACACGGCCGTATATTACTGTGCGAGAGGGGATTACGATGGTTCGGGGAGTTATCACTACTACTACGGTATGGACGTCTGGGGCCAAGGGACCACGGTCACCGTCTCCTCA |
| | | | SEQ ID NO: 26685 | SEQ ID NO: 30691 |
| | | AA | EIVLTQSPGTLSLSPGERATLSCRASQSIRSSFLAWYQQKPGQAPRLLIYGASSRATGIPDRFSGSGSGTDFTLTISRLEPEDFAVYYCQQYGNSPWAFGQGTKVEIK | QVQLQESGPGLVKPSQTLSLTCTVSGGSISSGDYYWNWIRQHPGKGLEWIGYIFYSGSTYYNPSLKSRVTISVDTSKNQFSLKLSSVTAADTAVYYCARGDYDGSGSYHYYYGMDVWGQGTTVTVSS |
| | | | SEQ ID NO: 26686 | SEQ ID NO: 30692 |
| **iPS:435913** | **21-225_190A7** | NA | GAAATTGTGTTGACGCAGTCTCCAGGCACCCTGTCTTTGTCTCCAGGGGAAAGAGCCACCCTCTCCTGCAGGGCCAGTCAGAGTGTTAGAAGCAACTTCTTAGCCTGGCACCAGCAGAAACCTGGCCAGGCTCCCAGGCTCCTCATCTATGGTGCATACCGCAGGGCCACTGGCATCCCAGACAGGTTCAGTGGCAGTGGGTCTGGGACAGACTTCACTCTCACCATCAACAGACTGGAGCCTGAAGATTTTGCAGTGTATTACTGTCAGCAGTATGGTAACTCACCGTGGACGTTCGGCCAAGGGACCAAGGTGGAAATCAAA | CAGGTGCAGCTGCAGGAGTCGGGCCCAGGACTGGTGAACCCTTCACAGACCCTGTCCCTCACCTGCACTGTCTCTGGTGGCTCCATCAGCAGTGGTGTTTACTACTGGAGCTGGATCCGCCAGCACCCAGGGAAGGGCCTGGAGTGGATTGGGAACATCTATTACAGTGGGAGCACCTACAACAACCCGTCCCTCAAGAGTCGAATTATCATATCAGTAGACACGTCTAAGAACCAGTTCTCCCTGAAGCTGAGCTCTGTGACTGCCGCGGACACGGCCGTGTATTACTGTGCGAGAGGGGATTACGATGGTTCGGGGAGTTATCACTACTACTACGGTTTGGACGTCTGGGGCCAAGGGACCACGGTCACCGTCTCCTCA |
| | | | SEQ ID NO: 26687 | SEQ ID NO: 30693 |

FIGURE 50

| | | | | |
|---|---|---|---|---|
| | | AA | EIVLTQSPGTLSLSPGERATLSCRASQSVRSNFLAWHQQKPGQAPRLLIYGAYRRATGIPDRFSGSGSGTDFTLTINRLEPEDFAVYYCQQYGNSPWTFGQGTKVEIK | QVQLQESGPGLVNPSQTLSLTCTVSGGSISSGVYYWSWIRQHPGKGLEWIGNIYYSGSTYNNPSLKSRIIISVDTSKNQFSLKLSSVTAADTAVYYCARGDYDGSGSYHYYYGLDVWGQGTTVTVSS |
| | | | SEQ ID NO: 26688 | SEQ ID NO: 30694 |
| **iPS:435915** | **21-225_190H4** | NA | GCCAACGTGATGACCCAGTCTCCAGACTCCCTGGCTGTGTCTCTGGGCGAGAGGACCACCATCAACTGCAAGTCCAGCCAGAGTGTTTTACACAGCTCCAACAATTACAACTACTTAGCTTGGTACCGGCAGAAACCAGGACAGCCTCCTAAGCTGCTCATTTACTGGGCATCTACCCGGGAATCCGGGGTCCCTGACCGATTCAGTGGCAGCGGGTCTGGTACAGATTTCACTCTCACCATCAGCAGCCTGCAGGCTGAAGATGTGGCAGTTTATTACTGTCAGCAATATTATAGTATTCCTCCCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGAAACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCCCATAGCAGTGGCTGGTACATCTTTGACTACTGGGGCCAGGGAACCCTGGTCACCGTCTCCTCA |
| | | | SEQ ID NO: 26689 | SEQ ID NO: 30695 |
| | | AA | ANVMTQSPDSLAVSLGERTTINCKSSQSVLHSSNNYNYLAWYRQKPGQPPKLLIYWASTRESGVPDRFSGSGSGTDFTLTISSLQAEDVAVYYCQQYYSIPPTFGPGTKVDIK | QVQLVQSGAEVKKPGASVKVSCKASGNTFTNYDINWVRQATGQGLEWMGWMNPNSGNTGYAQKFQGRVTMTRNTSISTAYMELSSLRSEDTAVYYCAHSSGWYIFDYWGQGTLVTVSS |
| | | | SEQ ID NO: 26690 | SEQ ID NO: 30696 |

FIGURE 50

| | | | | |
|---|---|---|---|---|
| **iPS:435917** | **21-225_190D5** | NA | GAAATTGTGCTGACTCAGTCTCCAGACTTTCAGTCTGTGGCTCCAAAGGAGAAAGTCACCATCACCTGCCGGGCCAGTCAGAGTATTGGTAGTAACTTACACTGGTACCAGCAGAAACCTGATCAGTCTCCAAAGCTCCTCATCAAGTCTGCTTCCCAGTCCTTCTCAGGGGTCCCCTCGAGGTTCAGTGGCAGTGGATCTGGGACAGATTTCACCCTCACCATCAATAGCCTGGAAACTGAAGATGCTGCAACGTATTACTGTCAGCAGAGTAGTAGTTTACCGTGGACGTTCGGCCAAGGGACCAAGGTGGAAATCAAA | CAGGTGCAGCTGCAGGAGTCGGGCCCAGGACTGGTGAAGCCTTCGGAGACCCTGTCCCTCACCTGCACTGTCTCTGGTGGCTCCATCAATAATTACTACTGGAGCTGGATCCGGCAGCCCGCCGGGAAGGGACTGGAGTGGATTGGGCGTATCTATGCCAGTGGGAGCACCAACTACAACCCCTCCCTCAAGAGTCGAGTCACCATGTCAATAGACACGTCCAAGAACCAGTTCTCCCTGAAGCTGAGCTCTGTGACCGCCGCGGACACGGCCGTGTATTACTGTGCGAGAGATCGGGGATACTATGGCTACTACGGTATGGACGTCTGGGGCCAAGGGACCACGGTCACCATCTCCTCA |
| | | | SEQ ID NO: 26691 | SEQ ID NO: 30697 |
| | | AA | EIVLTQSPDFQSVAPKEKVTITCRASQSIGSNLHWYQQKPDQSPKLLIKSASQSFSGVPSRFSGSGSGTDFTLTINSLETEDAATYYCQQSSSLPWTFGQGTKVEIK | QVQLQESGPGLVKPSETLSLTCTVSGGSINNYYWSWIRQPAGKGLEWIGRIYASGSTNYNPSLKSRVTMSIDTSKNQFSLKLSSVTAADTAVYYCARDRGYYGYYGMDVWGQGTTVTISS |
| | | | SEQ ID NO: 26692 | SEQ ID NO: 30698 |
| **iPS:435919** | **21-225_190H5** | NA | GACATCCAGATGACCCAGTCTCCATCCTCCCTGTCTGCATCTGTAGGAGACAGAGTCACCATCACTTGCCGGGCAAGTCAGGGCATTAGAAAAGATTTAGGCTGGTATCAGCAGAAACCAGGGAAAGCCCCTAAGCGCCTGATCCATACTGCATCCAGTTTGCAAAGTGGGGTCCCATTAAGGTTCAGCGGCAGTGGATCTGGGACAGAATTCACTCTCACAATCAGCAGCCTGCAGCCTGAAGATTTTGCAACTTATTACTGTCTACAGCATAATAATTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAGTGGGTGGCAGTTATATCATATGATGGAGGTTATAAAAACTATGCAGACTCCGTGAAGGGCCGATTCACCATCTCCAGAGACAATTCCAAGAACACGCTGTATCTGCAAATGAATAGCCTGAGAGCTGAGGACACGGCTGTGTATTACTGTGCGAGAGGTACCCACGGGTACTACTACGGTGTGGACGTCTGGGGCCAAGGGACCACGGTCACCGTCTCCTCA |
| | | | SEQ ID NO: 26693 | SEQ ID NO: 30699 |

FIGURE 50

| | | | | |
|---|---|---|---|---|
| | | AA | DIQMTQSPSSLSASVGDRVTITCRASQGIRKDLG WYQQKPGKAPKRLIHTASSLQSGVPLRFSGSGS GTEFTLTISSLQPEDFATYYCLQHNNYPWTFGQG TKVEIK | QVQLVESGGGVVQPGRSLRLSCAASGFTFSSYGMH WVRQAPGKGLEWVAVISYDGGYKNYADSVKGRF TISRDNSKNTLYLQMNSLRAEDTAVYYCARGTHG YYYGVDVWGQGTTVTVSS |
| | | | SEQ ID NO: 26694 | SEQ ID NO: 30700 |
| **iPS:435921** | **21-225_190D6** | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCTGAAACCAGGGAAAGC CCCTAAGCGCCTGATTTATGCTGCATCCAGTTT GCAAAGTGGGGTCCCATCAAGGTTCAGCGGCA GTGGATCTGGGCCAGAATTCACTCTCACAATC AGCAGCCTGCAGCCTGAAGATTTTGCAACTTA TTACTGTCTACAGCATTATAGTTTCCCATTCAC TTTCGGCCCTGGGACCAAAGTGGATATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTTTATCAT GCACTGGGTCCGCCAGGCTCCAGGCAGGGGGCT GGAGTGGGTGGCAGTTATATGGTATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGAGGAGTA TAGCAGTGGCTGGTTCGGGTACGGTATGGACGTC TGGGGCCAAGGGACCACGGTCACCGTCTCCTCA |
| | | | SEQ ID NO: 26695 | SEQ ID NO: 30701 |
| | | AA | DIQMTQSPSSLSASVGDRVTITCRASQGIRNDLG WYQLKPGKAPKRLIYAASSLQSGVPSRFSGSGSG PEFTLTISSLQPEDFATYYCLQHYSFPFTFGPGTK VDIK | QVQLVESGGGVVQPGRSLRLSCAASGFTFSSFIMH WVRQAPGRGLEWVAVIWYDGSNKYYADSVKGRF TISRDNSKNTLYLQMNSLRAEDTAVYYCAREEYSS GWFGYGMDVWGQGTTVTVSS |
| | | | SEQ ID NO: 26696 | SEQ ID NO: 30702 |

FIGURE 50

| | | | | |
|---|---|---|---|---|
| iPS:435923 | 21-225_190H6 | NA | GACATCGTGATGACCCAGTCTCCAGACTCCCTGGCTGTGTCTCTGGGCGAGAGGGCCACCATCAACTGCAAGTCCAGCCAGAGTGTTTTATTCAACTCCAACAATAAGAACTACTTAGCTTGGTACCAGCAGAAACCAGGTCAGCCTCCTAACCTGCTCATTTACTGGGCATCTACCCGGGAATCCGGGGTCCCTGACCGATTCAGTGGCAGCGGGTGTGGGACAGATTTCACTCTCACCATCAGCAGCCTGCAGGCTGAAGATGTGGCAGTTTATTACTGTCAGCAATATTGTAGTCTTCCATTCACTTTCGGCCCTGGGACCAAAGTGGATATCAGA | CAGGTGCAACTGGTGGAGTCTGGGGGAGGCTTGGTCAAGCCTGGAGGGTCCCTGAGACTCTCCTGTGCAGCCTCTGGATTCACCTTCAGTGACTACTACATGAGCTGGATCCGCCAGGCTCCAGGGAAGGGGCTGGAGTGGCTTTCATACATTAGTAGTAGTGGTACTACCGTATTCTACGCAGACTCTGTGAAGGGCCGATTCACCATCTCCAGGGACAACGCCAAGAACTCACTGTATCTGCAAATGAACAGCCTGAGAGCCGAGGACACGGCCGTGTATTACTGTGCGAGAGAATGGGTGGGAGCCGACTACTGGGGCCAGGGAACCCTGGTCACCGTCTCCTCA |
| | | | SEQ ID NO: 26697 | SEQ ID NO: 30703 |
| | | AA | DIVMTQSPDSLAVSLGERATINCKSSQSVLFNSNNKNYLAWYQQKPGQPPNLLIYWASTRESGVPDRFSGSGCGTDFTLTISSLQAEDVAVYYCQQYCSLPFTFGPGTKVDIR | QVQLVESGGGLVKPGGSLRLSCAASGFTFSDYYMSWIRQAPGKGLEWLSYISSSGTTVFYADSVKGRFTISRDNAKNSLYLQMNSLRAEDTAVYYCAREWVGADYWGQGTLVTVSS |
| | | | SEQ ID NO: 26698 | SEQ ID NO: 30704 |
| iPS:435925 | 21-225_190D7 | NA | GACATCGTGATGACCCAGTCTCCAGACTCCCTGGCTGTGTCTCTGGGCGAGAGGGCCACCATCAACTGCAAGTCCAGCCAGAGTGTTTTATCCAGCTCCAACAATTACAACTATTTAGTTTGGTATCAGCAGAAACCAGGACAGCCTCCTAAGCTGCTCATTTACTGGGCATCTACCCGGAAATCCGGGGTCCCTGACCGATTCAGTGGCAGCGGGTCTGGGACAGATTTTACTCTCACCATCAGCAGCCTGCAGGCTGATGACGTGGCAGTTTATTACTGTCAACAATATTATCGTACTCCGTGGACGTTCGGCCAAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAATAGTGGTAATACAGGCTATGCACAGAAGTTCCAGGGCAGAGTCACCATGACCAGGAATACCTCCATAAGCACAGCCTACATGGAGCTGAGCAGCCTGAGATCTGAGGACACGGCCGTGTATTACTGTGCGAGTAGCAGTGGCTGGTACTTCTTTGACTACTGGGGCCAGGGAACCCTGGTCACCGTCTCCTCA |
| | | | SEQ ID NO: 26699 | SEQ ID NO: 30705 |

FIGURE 50

| | | | | |
|---|---|---|---|---|
| | | AA | DIVMTQSPDSLAVSLGERATINCKSSQSVLSSSNNYNYLVWYQQKPGQPPKLLIYWASTRKSGVPDRFSGSGSGTDFTLTISSLQADDVAVYYCQQYYRTPWTFGQGTKVEIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYYCASSSGWYFFDYWGQGTLVTVSS |
| | | | SEQ ID NO: 26700 | SEQ ID NO: 30706 |
| **iPS:435927** | **21-225_190E7** | NA | GATATTGTGTTGACCCAGACTCCACTCTCTCTGTCCGTCACCCCTGGACAGCCGGCCTCCATCTCCTGCAAGTCTAGTCAGAGCCTCCTCCATAGTGATGGAAGGACCTATTTGTATTGGTACCTGCAGAAACCAGGCCAGCCTCCACAGGTCCTGATCTGTGAGGTTTCCAACCGGTTCTCTGGAGTGCCAGATAGGTTCAGTGGCAGCGGGTCAGGGACAGATTTCACACTGAAAATCAGCCGGGTGGAGGCTGGAGATGTTGGGGTTTATTACTGCATGCAAAGTATACAGCTTCCCTGGACGTTCGGCCAAGGGACCAAGGTGGAAATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGAAGTTACCACTGGAGTTGGATCCGGCAGCCCGCCGGGAAGGGACTGGAGTGGATTGGGCATATCTATACCAGTAGGAGCACCAACTACAACCCCTCCCTCAAGAGTCGAGTCACCATTTCAGTAGACACGTCCAAGAACCAGTTCTCCCTGAAGCTGAGCTCTGTGACCGCCACGGACACGGCCGTGTATTACTGTGCGAGACTCCGGTATAACTGGAACTTCCCTTACTTTGACTACTGGGGCCAGGGAACCCTGGTCACCGTCTCCTCA |
| | | | SEQ ID NO: 26701 | SEQ ID NO: 30707 |
| | | AA | DIVLTQTPLSLSVTPGQPASISCKSSQSLLHSDGRTYLYWYLQKPGQPPQVLICEVSNRFSGVPDRFSGSGSGTDFTLKISRVEAGDVGVYYCMQSIQLPWTFGQGTKVEIK | QLQLQESGPGLVKPSETLSLTCTVSGGSIRSYHWSWIRQPAGKGLEWIGHIYTSRSTNYNPSLKSRVTISVDTSKNQFSLKLSSVTATDTAVYYCARLRYNWNFPYFDYWGQGTLVTVSS |
| | | | SEQ ID NO: 26702 | SEQ ID NO: 30708 |

FIGURE 50

| | | | | |
|---|---|---|---|---|
| **iPS:435929** | **21-225_190D9** | NA | GAAATTGTGCTGACTCAGTTTCCAGACTCTCAGTCTGTGACTCCAAAGGAGAAAGTCACCATCACCTGCCGGGCCAGTCAGAGCATTGGTAGTAGCTTACACTGGTACCAGCAGAAACCAGATCAGTCTCCAAAGCTCCTCATCAAGTATGCTTCCCAGTCCTTCTCAGGGGTCCCCTCGAGGTTCAGTGGCAGTGGATCTGGGACAGATTTCACCCTCACCATCAATAGCCTGGAAGCTGAAGATGCTGCAACATATTACTGTCATCAGAGTAGTAGTTTCCCTCGGACGTTCGGCCAAGGGACCAAGGTGGAAATCAAA | GAGGTGCAGCTGTTGGACTCTGGGGGAGGCTTGGGACAGCCTGGGGGGTCCCTGAGACTCTCCTGTGCAGCCTCTGGATTCACCTTTGACAGCTATGCCATGAGTTGGGTCCGCCAGGCTCCAGGGAAGGGGCTGGAGTGGGTCTCAACTATTAGTGGTACTGGTCGTAGGACATACTACGCAGACTCCGTGAAGGGCCGGTTCACCATCTCCAGAGACAATTCCAAGAACACGCTGTATCTGCAAATGAATAGCCTGAGAGCCGAGGACACGGCCGTATATTACTGTGCGAAAGAGGAGGATTACTATGATAGTAGTGGCCCGGGGTTCGACCCCTGGGGCCAGGGAACCCTGGTCACCGTCTCCTCA |
| | | | SEQ ID NO: 26703 | SEQ ID NO: 30709 |
| | | AA | EIVLTQFPDSQSVTPKEKVTITCRASQSIGSSLHWYQQKPDQSPKLLIKYASQSFSGVPSRFSGSGSGTDFTLTINSLEAEDAATYYCHQSSSFPRTFGQGTKVEIK | EVQLLDSGGGLGQPGGSLRLSCAASGFTFDSYAMSWVRQAPGKGLEWVSTISGTGRRTYYADSVKGRFTISRDNSKNTLYLQMNSLRAEDTAVYYCAKEEDYYDSSGPGFDPWGQGTLVTVSS |
| | | | SEQ ID NO: 26704 | SEQ ID NO: 30710 |
| **iPS:435933** | **21-225_190F8** | NA | GACATCCAGATGACCCAGTCTCCTTCCTCACTGTCTGCATCTGTAGGAGACAGAGTCACCATCACTTGTCGGACGAGTCAGGGCATTGGCAATTATTTAGCCTGGTTTCAGCAGAAACCAGGGAAAGCCCCTAAGTCCCTACTCTATAAAGCATCCAGTTTACAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATATGACTTACCCACTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTACTTATGGCATGCACTGGGTCCGCCAGGCTCCAGGCAAGGGGCTGGAGTGGGTGGCAGTTATATGGTATGATGGAAGTAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCAGGGCGTGGGCTACGACGGTTTGGACGTCTGGGGCCAAGGGACCACGGTCACCGTCTCCTCA |
| | | | SEQ ID NO: 26705 | SEQ ID NO: 30711 |

FIGURE 50

| | | | | |
|---|---|---|---|---|
| | | AA | DIQMTQSPSSLSASVGDRVTITCRTSQGIGNYLA WFQQKPGKAPKSLLYKASSLQSGVPSKFSGSGS GTDFTLTISSLQPEDFATYYCQQYMTYPLTFGGG TKVEIK | QVQLVESGGGVVQPGRSLRLSCAASGFTFSTYGMH WVRQAPGKGLEWVAVIWYDGSNKNYADSVKGRF TISRDNSKNTLYLQMNSLRAEDTAVYYCARDQGV GYDGLDVWGQGTTVTVSS |
| | | | SEQ ID NO: 26706 | SEQ ID NO: 30712 |
| **iPS:435935** | **21-225_190H8** | NA | GAAATTGTGCTGACTCAGTTTCCAGACTCTCA GTCTGTGACTCCAAAGGAGAAAGTCACCATCA CCTGCCGGGCCAGTCAGAGCATTGGTAGTAGC TTACACTGGTACCAGCAGAAACCAGATCAGTC TCCAAAGCTCCTCATCAAGTATGCTTCCCAGT CCTTCTCAGGGGTCCCCTCGAGGTTCAGTGGC AGTGGATCTGGGACAGATTTCACCCTCACCAT CAATAGCCTGGAAGCTGAAGATGCTGCAACGT ATTACTGTCATCAGAGTAGTAGTTTCCCTCGG ACGTTCGGCCAAGGGACCAAGGTGGAAATCA AA | GGGGTACAACTGTTGGACTCTGGGGGAGGCTTGG GACAGCCTGGGGGGTCCCTGAGACTCTCCTGTGC AGCCTCTGGATTCACCTTTGACAGCTATGCCATG AGCTGGGTCCGCCAGGCTCCAGGGAAGGGGCTG GAGTGGGTCTCAACTATTAGTGGTACTGGTCGTA GGACATATTACGCAGACTCCGTGAAGGGCCGGTT CACCATCTCCAGAGACAATTCCAAGAACACACTG TATCTGCAGATGAATAGCCTGAGAGCCGAGGAC ACGGCCGTATATTACTGTGCGAAAGAGGAGGATT ACTATGATAGTAGTGGCCCGGGGTTCGACCCCTG GGGCCAGGGAACCCTGGTCACCGTCTCCTCA |
| | | | SEQ ID NO: 26707 | SEQ ID NO: 30713 |
| | | AA | EIVLTQFPDSQSVTPKEKVTITCRASQSIGSSLHW YQQKPDQSPKLLIKYASQSFSGVPSRFSGSGSGT DFTLTINSLEAEDAATYYCHQSSSFPRTFGQGTK VEIK | GVQLLDSGGGLGQPGGSLRLSCAASGFTFDSYAMS WVRQAPGKGLEWVSTISGTGRRTYYADSVKGRFTI SRDNSKNTLYLQMNSLRAEDTAVYYCAKEEDYYD SSGPGFDPWGQGTLVTVSS |
| | | | SEQ ID NO: 26708 | SEQ ID NO: 30714 |

FIGURE 50

| | | | | |
|---|---|---|---|---|
| **iPS:435937** | **21-225_190H9** | NA | GACATCCAGATGACCCAGTCTCCATCCTCACTGTCTGCATCTGTGGGAGACAGAGTCACCATCACTTGTCGGGCGAGTCAGGGCATTGGCAAGTATTTAGCCTGGTTTCAGCAGAAACCAGGGAAAGCCCTTAAGTCACTGATCTATGCTGCATCCAGTTTGCAAAGTGGGGTCCCATCAAAATTCAGCGGCAGTGGATCTGGGACAGATTTCACTCTCACCATCAGCAGCCTGCAGCCTGACGATTTTGCAACTTATTACTGCCAACGATATGATACTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGGAACTATGGCATGCACTGGGTCCGCCAGGCTCCAGGCAAGGGGCTGGAGTGGGTGGCAGTTATATGGTTTGATGGAAGTAATGACTACTATGCAGACTCCGTGAAGGGCCGATTCACCATCTCCAGAGACAATTCCAAGAACACGCTGTCACTGCAAATGAACAGCCTGAGAGCCGAGGACACGGCTGTGTATTACTGTGCGAGAGATAGAAGCGTCGGCTACGACGGTTTAGATGTCTGGGGCCAAGGGACCACGGTCACCGTCTCCTCA |
| | | | SEQ ID NO: 26709 | SEQ ID NO: 30715 |
| | | AA | DIQMTQSPSSLSASVGDRVTITCRASQGIGKYLAWFQQKPGKALKSLIYAASSLQSGVPSKFSGSGSGTDFTLTISSLQPDDFATYYCQRYDTYPFTFGPGTKVDIK | QVQLVESGGGVVQPGRSLRLSCAASGFTFRNYGMHWVRQAPGKGLEWVAVIWFDGSNDYYADSVKGRFTISRDNSKNTLSLQMNSLRAEDTAVYYCARDRSVGYDGLDVWGQGTTVTVSS |
| | | | SEQ ID NO: 26710 | SEQ ID NO: 30716 |
| **iPS:435939** | **21-225_191H7** | NA | GAAATTGTGTTGACGCAGTCTCCAGGCACCCTGTCTTTGTCTCCAGGGGAAAGCGCCACCCTCTCCTGCAGGGCCGGTCAAAGTATTAGAACCGACTTCTTAGCCTGGTACCAGCAGCAACCTGGCCAGGCTCCCAGGCTCCTCATCTATGGTCCATCCAGCAGGGCCACTGGCATCCCAGACAGGTTCAGTGGCAGTGGGTCTGGGACAGACTTCACTCTCACCATCAACAGACTGGAGCCTGAAGATTTTGCAGTGTATTTCTGTCAGCAGTATGGTAGCTCACCGTGGACGTTCGGCCAAGGGACCAAGGTGGAAATCAAT | CAGGTGCAACTGCAGGAGTCGGGCCCAGGACTGGTGAAGCCTTCACAGACCCTGTCCCTCACCTGCACTGTCTCTGGTGGCTCCATCAACAGTGGTGATTACTACTGGAACTGGATCCGCCAGCACCCAGGGAAGGGCCTGGAGTGGATTGGGTACATCTTTTACAGTGGGAGCACCTACTACAACCCGTCCCTCAAGAGTCGAGTTACCATATCAGTAGACACGTCTAAGAACCAGTTCTCCCTGAAGCTGAGCTCTGTGACTGCCGCGGACACGGCCGTGTATTACTGTGCGAGAGGGGATTACGATGGTTCGGGGAGTTATCACTACTATTACGGTATGGACGTCTGGGGCCAAGGGACCACGGTCACCGTCTCCTCA |
| | | | SEQ ID NO: 26711 | SEQ ID NO: 30717 |

FIGURE 50

| | | | | |
|---|---|---|---|---|
| | | AA | EIVLTQSPGTLSLSPGESATLSCRAGQSIRTDFLAWYQQQPGQAPRLLIYGPSSRATGIPDRFSGSGSGTDFTLTINRLEPEDFAVYFCQQYGSSPWTFGQGTKVEIN | QVQLQESGPGLVKPSQTLSLTCTVSGGSINSGDYYWNWIRQHPGKGLEWIGYIFYSGSTYYNPSLKSRVTISVDTSKNQFSLKLSSVTAADTAVYYCARGDYDGSGSYHYYYGMDVWGQGTTVTVSS |
| | | | SEQ ID NO: 26712 | SEQ ID NO: 30718 |
| iPS:435941 | 21-225_191E8 | NA | GAAATAGTGATGACGCAGTCTCCAGCCACCCTGTCTGTGTCTCCAGGGGAAAGAGCCACCCTCTCCTGCAGGCCCAGTCAGAGTTTTAGCAGAAACTTAGCCTGGTACCAGCAGAAACCTGGCCAGGCTCCCAGGCTCCTCATCTATGGTGCATCCACTAGGGCCACTGGTATCCCATCCAGGTTCAGTGGCAGTGGGTCTGGGACAGAGTTCACTCTCACCATCAGCAGCCTGGAGTCTGAAGATTTTGCAGTTTACTACTGTCAGCAGTATAATAACTGGCCGCTCACTTTCGGCGGAGGGATCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAATTATATGGTTTGATGGAAGTAATCAATACTATGCCGACTCCGTGAAGGGCCGATTCACCATCTCCAGAGACAATTCCAAGAACACGCTGTATCTGCAATTGAACAGCCTGAGAGCCGAGGACACGGCTGTCTATTACTGTGCGAGAGCCCACGGGGTCTACTACTACGCTATGGACGTCTGGGGCCAAGGGACCACGGTCACCGTCTCCTCA |
| | | | SEQ ID NO: 26713 | SEQ ID NO: 30719 |
| | | AA | EIVMTQSPATLSVSPGERATLSCRPSQSFSRNLAWYQQKPGQAPRLLIYGASTRATGIPSRFSGSGSGTEFTLTISSLESEDFAVYYCQQYNNWPLTFGGGIKVEIK | QVQLVESGGGVVQPGRSLRLSCAASGFTFSNYGMHWVRQAPGKGLEWVAIIWFDGSNQYYADSVKGRFTISRDNSKNTLYLQLNSLRAEDTAVYYCARAHGVYYYAMDVWGQGTTVTVSS |
| | | | SEQ ID NO: 26714 | SEQ ID NO: 30720 |

FIGURE 50

| | | | | |
|---|---|---|---|---|
| iPS:435943 | 21-225_191C9 | NA | GAAATTGTGCTGACTCAGTCTCCAGACTTTCAGTCTGTGACTCCAAAGGAGAAAGTCACCATCACCTGCCGGGCCAGTCAGAGTATTGGTAGTAGTTTACACTGGTACCAGCAGAAACCAGATCAGTCTCCAAAGCTCCTCATCAAGTATGCTTCCCAGTCCTTCTCAGGGGACCCCTCGAGGTTCAGTGGCAGTGGATCTGGGACAGATTTCACCCTCACCATCAATAGCCTGGAAGCTGAAGATGCTGCAACGTATTACTGTCATCAGACTAGAAGTTTACCTCTCACTTTCGGCGGAGGGACCAAGGTGGAGATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGGTTACTACTGGAACTGGATCCGCCAGCACCCAGGGAAGGGCCTGGATTGGATTGGATATATCTATTACAGTGGGAGCACCTACTACAACCCGTCCCTCAAGAGTCGAGTTACCATATCAGTAGACACGTCTAAGAACCAGTTCTCCCTGAAGCTGAACTCTGTGACTGCCGCGGACACGGCCGTGTATTACTGTGCGAGATCCGGGTATAATTGGGACGCCGGGGTCGACCCCTGGGGCCAGGGAACCCTGGTCACCGTCTCCTCA |
| | | | SEQ ID NO: 26715 | SEQ ID NO: 30721 |
| | | AA | EIVLTQSPDFQSVTPKEKVTITCRASQSIGSSLHWYQQKPDQSPKLLIKYASQSFSGDPSRFSGSGSGTDFTLTINSLEAEDAATYYCHQTRSLPLTFGGGTKVEIK | QVQLQESGPGLVKPSQTLSLTCTVSGGSISSGGYYWNWIRQHPGKGLDWIGYIYYSGSTYYNPSLKSRVTISVDTSKNQFSLKLNSVTAADTAVYYCARSGYNWDAGVDPWGQGTLVTVSS |
| | | | SEQ ID NO: 26716 | SEQ ID NO: 30722 |
| iPS:435945 | 21-225_191A10 | NA | GACATCCAGATGACCCAGTCTCCATCCTCACTGTCTGCATCTGTAGGAGACAGAGTCACCATCACTTGTCGGGCGAGTCAGGGCATTGGCAAGTATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATATGGGACAGATTTCACTCTCACCATCAGCAGCCTGCAGCCTGAAAATTTTGCAATTTATTACTGCCAACAGTATAGTACTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTACGCAGACTCCGTGAAGGGCCGATTCACCATCTCCAGAGACAATTCCAAGAACACGCTGTATCTGCAAATGAACAGCCTGAGAGCCGAGGACACGGCTATGTATTACTGTGCGAGAGATCAGGGCGTGGGCTACGACGGTTTGGACGTCTGGGGCCAAGGGACCTCGGTCACCGTCTCCTCA |
| | | | SEQ ID NO: 26717 | SEQ ID NO: 30723 |

FIGURE 50

| | | | | |
|---|---|---|---|---|
| | | AA | DIQMTQSPSSLSASVGDRVTITCRASQGIGKYLAWFQQKPGKAPKSLIYAASSLQSGVPSKFSGSGYGTDFTLTISSLQPENFAIYYCQQYSTYPLTFGGGTKVEIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAMYYCARDQGVGYDGLDVWGQGTSVTVSS |
| | | | SEQ ID NO: 26718 | SEQ ID NO: 30724 |
| iPS:435947 | 21-225_191E10 | NA | GACATCCAGATGACCCAGTCTCCATCCTCACTGTCTGCATCTGTAGGAGACAGAGTCACCATCACTTGTCGGGCGAGTCAGGGCATTGGCAAGTATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAAATTTTGCAACTTATTACTGCCAACAGTATAGTACTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTACGCAGACTCCGTGAAGGGCCGATTCACCATCTCCAGAGACAATTCCAAGAACACGCTGTATCTGCAAATGAACAGCCTGAGAGCCGAGGACACGGCTATGTATTACTGTGCGAGAGATCAGGGCGTGGGCTACGACGGTTTGGACGTCTGGGGCCAAGGGACCTCGGTCACCGTCTCCTCA |
| | | | SEQ ID NO: 26719 | SEQ ID NO: 30725 |
| | | AA | DIQMTQSPSSLSASVGDRVTITCRASQGIGKYLAWFQQKPGKAPKSLIYAASSLQSGVPSKFSGSGSGTDFTLTISSLQPENFATYYCQQYSTYPLTFGGGTKVEIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAMYYCARDQGVGYDGLDVWGQGTSVTVSS |
| | | | SEQ ID NO: 26720 | SEQ ID NO: 30726 |

FIGURE 50

| | | | | |
|---|---|---|---|---|
| **iPS:435953** | **21-225_191B12** | NA | GACATCGTGATGACCCAGTCTCCAGACTCCCTGGCTGTGTCTCTGGGCGAGAGGGCCACCATCAACTGCAAGTCCAGCCAGAGTGTTTTATTCAACTCCAACAATAAGAACTACTTAGCTTGGTACCAGCAGAAACCAGGTCAGCCTCCTAAACTGCTCATTTACTGGGCCTCTACCCGGGAATCCGGGGTCCCTGACCGATTCAGTGGCAGCGGGTCTGGGACAGATTTCACTCTCACCATCAGCAACCTGCAGGCTGAAGATGTGGCAATTTATTACTGTCAGCAATATTCTAGTCTTCCATTCACTTTCGGCCCTGGGACCAAAGTGGATATCAAA | CAGGTGCAGCTGGTGGAGTCTGGGGGAGGCTTGGTCAAGCCTGGAGGGTCCCTGAGACTCTCCTGTGCAGCCTCTGGATTCACCTTCAGTGACTACTACATGAGCTGGATCCGCCAGGCTCCAGGGAAGGGGCTGGAGTGGGTTTCATACATTAGTAGTAGTGGTACTACCGTATTCTACGCAGACTCTGTGAAGGGCCGATTCACCATCTCCAGGGACAACGCCAAGAACTCACTGTATCTGCAAATGAACAGCCTGAGAGCCGAGGACACGGCCGTGTATTACTGTGCGAGAGAATGGGTGGGAGCCGACTACTGGGGCCAGGGAACCCTGGTCACCGTCTCCTCA |
| | | | SEQ ID NO: 26721 | SEQ ID NO: 30727 |
| | | AA | DIVMTQSPDSLAVSLGERATINCKSSQSVLFNSNNKNYLAWYQQKPGQPPKLLIYWASTRESGVPDRFSGSGSGTDFTLTISNLQAEDVAIYYCQQYSSLPFTFGPGTKVDIK | QVQLVESGGGLVKPGGSLRLSCAASGFTFSDYYMSWIRQAPGKGLEWVSYISSSGTTVFYADSVKGRFTISRDNAKNSLYLQMNSLRAEDTAVYYCAREWVGADYWGQGTLVTVSS |
| | | | SEQ ID NO: 26722 | SEQ ID NO: 30728 |
| **iPS:435957** | **21-225_191G12** | NA | GACATCCAGATGACCCAGTCTCCTTCCTCACTGTCTGCATCTGTAGGAGACAGAGTCACCATCACTTGTCGGACGAGTCAGGGCATTGGCAATTATTTAGCCTGGTTTCAGCAGAAACCAGGGAAAGCCCCTAAGTCCCTGCTCTATAAAGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATATCACTTACCCGCTCACTTTCGGCGGAGGGTCCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCAGGGCGTGGGCTACGACGGTTTGGACGTCTGGGGCCAAGGGACCACGGTCACCGTCTCCTCA |
| | | | SEQ ID NO: 26723 | SEQ ID NO: 30729 |

FIGURE 50

| | | | | |
|---|---|---|---|---|
| | | AA | DIQMTQSPSSLSASVGDRVTITCRTSQGIGNYLAWFQQKPGKAPKSLLYKASSLQSGVPSKFSGSGSGTDFTLTISSLQPEDFATYYCQQYITYPLTFGGGSKVEIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAVYYCARDQGVGYDGLDVWGQGTTVTVSS |
| | | | SEQ ID NO: 26724 | SEQ ID NO: 30730 |
| iPS:435961 | 21-225_192A2 | NA | GACATCCAGATGACCCAGTCTCCATCCTCACTGTCTGCATCTGTAGGAGACAGAGTCACCATCACTTGTCGGGCGAATCAGGGCATTAACAATTATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGCTGGCAGTTATATGGTATGATGGAAGTTATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGGATTCCCCTTATAGTGGCTACGCCTTGGACTACTTCTACGGTATGGACGTCTGGGGCCAAGGGACCACGGTCACCGTCTCCTCA |
| | | | SEQ ID NO: 26725 | SEQ ID NO: 30731 |
| | | AA | DIQMTQSPSSLSASVGDRVTITCRANQGINNYLAWFQQKPGKAPKSLIYAASSLQSGVPSKFSGSGSGTDFTLTISSLQPEDFATYYCQQYNSYPFTFGPGTKVDIK | QVQLVESGGGVVQPGRSLRLSCAASGFTFSSYGMHWVRQAPGKGLEWLAVIWYDGSYKYYADSVKGRFTISRDNSKNTLYLQMNSLRAEDTAVYYCAREDSPYSGYALDYFYGMDVWGQGTTVTVSS |
| | | | SEQ ID NO: 26726 | SEQ ID NO: 30732 |

FIGURE 50

| | | | | |
|---|---|---|---|---|
| **iPS:435963** | **21-225_192D2** | NA | GACATCCAGATGATTCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACATTATGTTACTTACCCGAACACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGCAGACTCCGTGAAGGGCCGATTCACCATCTCCAGAGACAATTCCAAGAACATGTTGTATCTGCAAATGAACAGCCTGAGAGCCGAGGAAACGGCTGTGTATTACTGTGCGAGAGATCGTGGGGTTGGCTACTACGGTATGGACGTCTGGGGCCAAGGGACCACGGTCACCGTCTCCTCA |
| | | | SEQ ID NO: 26727 | SEQ ID NO: 30733 |
| | | AA | DIQMIQSPSSLSASVGDRVTITCRASQGISNYLAWFQQKPGKAPKSLIYAASSLQSGVPSKFSGSGSGTDFTLTISSLQPEDFATYYCQHYVTYPNTFGGGTKVEIK | QVQLVESGGGVVQPGRSLRLSCAASGFTFSSYGMHWVRQAPGKGLEWVAVIWYDGSNKNYADSVKGRFTISRDNSKNMLYLQMNSLRAEETAVYYCARDRGVGYYGMDVWGQGTTVTVSS |
| | | | SEQ ID NO: 26728 | SEQ ID NO: 30734 |
| **iPS:435965** | **21-225_192H2** | NA | GACATCCAGATGACCCAGTCTCCATCTTCCGTGTCTGCATCTGTAGGAGACAGAGTCACCATCACTTGTCGGGCGAGTCAGGGTATAAGTAGTTGGATAGCCTGGTATCAGCAGAAACCAGGGAAAGCCCCTAAGCTCCTGATCTATGGTGCATCCAGTTTGCAAAGTGGGGTCCCATCTAGGTTCAGCGGCAGTGGATCTGGGACAGATTTCACTCTCACCATCAGCAGCCTGCAGCCTGAAGATTTTGCAACTTACTATTGTCAACAGTCTAACAGTTTCCCATTCACTTTCGGCCCTGGGACCAAAGTGGATGTCAAA | GAGGTGCAGCTGTTGGAATCTGGGGGAGACTTAATACAGCCTGGGGGGTCCCTGAGACTCTCCTGTGCAGCCTCTGGATTCACCTTTAGCAGCTCTGCCATGAGCTGGGTCCGCCAGGCTCCAGGGAAGGGGCTGGAGTGGGTCTCAGCCATTAGTGGTAGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAAGACACGGCCGTTTATTACTGTGCGAAACTCATAGCAGTAGTTGGGTCCCACTACTTTGACTACTGGGGCCAGGGAACCCTGGTCACCGTCTCCTCA |
| | | | SEQ ID NO: 26729 | SEQ ID NO: 30735 |

FIGURE 50

| | | | | |
|---|---|---|---|---|
| iPS:435967 | 21-225_192B3 | AA | DIQMTQSPSSVSASVGDRVTITCRASQGISSWIAWYQQKPGKAPKLLIYGASSLQSGVPSRFSGSGSGTDFTLTISSLQPEDFATYYCQQSNSFPFTFGPGTKVDVK | EVQLLESGGDLIQPGGSLRLSCAASGFTFSSSAMSWVRQAPGKGLEWVSAISGSGGNTFYADSVKGRFTISRDNSKNTLYLQMNSLRAEDTAVYYCAKLIAVVGSHYFDYWGQGTLVTVSS |
| | | | SEQ ID NO: 26730 | SEQ ID NO: 30736 |
| | | NA | GAAATTGTGTTGACGCAGTCTCCAGGCACCCTGTCTTTGTCTCCAGGGGAAAGAGCCACCCTCTCCTGCAGGGCCAGTCAGAGTGTTCGCAGCAGCTTCCTTGCCTGGTACCAGCAGAAACCTGGCCAGGCTCCCAGGCTCCTCATCTATGGTGCATCTAGCAGGGCCACTGGCATCCCAGACAGGTTCAGTGGCAGTGGGTCTGGGACAGACTTCACTCTCACCATCAGCAGACTGGAGCCTGAAGATTTTGCTGTGTATTACTGTCAGCAGTATGGTAACTCACCGTGGGCGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGATTACTACTGGAACTGGATCCGCCAGCACCCAGGGAAGGGCCTGGAATGGATTGGGTTCATCTTTTACAGTGGGAGCACCTACTACAACCCGTCCCTCAAGAGTCGAGTTACCATATCAGTAGACACGTCTAAGAACCAGTTCTCCCTGAAGCTGAGCTCTGTGACTGTCGCGGACACGGCCGTGTATTACTGTGCGAGAGGGGATTACGATGGTTCGGGGAGTTATCACCACTACTACGGTATGGACGTCTGGGGCCAAGGGACCACGGTCACCGTCTCCTCA |
| | | | SEQ ID NO: 26731 | SEQ ID NO: 30737 |
| | | AA | EIVLTQSPGTLSLSPGERATLSCRASQSVRSSFLAWYQQKPGQAPRLLIYGASSRATGIPDRFSGSGSGTDFTLTISRLEPEDFAVYYCQQYGNSPWAFGQGTKVEIK | QVQLQESGPGLVKPSQTLSLTCTVSGGSISSGDYYWNWIRQHPGKGLEWIGFIFYSGSTYYNPSLKSRVTISVDTSKNQFSLKLSSVTVADTAVYYCARGDYDGSGSYHHYYGMDVWGQGTTVTVSS |
| | | | SEQ ID NO: 26732 | SEQ ID NO: 30738 |

FIGURE 50

| | | | | |
|---|---|---|---|---|
| **iPS:435971** | **21-225_192D3** | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATCTGGAACAGATTTCACTCTCACCATCAGCAGCCTGCAGCCTGAAGATTTTGCAACTTATTACTGCCTACATTATCTTACTTACCCTCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATGAACACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATAGGGGGGTGGGTTACTACGGTTTGGACGTCTGGGGCCAAGGGACCACGGTCACCGTCTCCTCA |
| | | | SEQ ID NO: 26733 | SEQ ID NO: 30739 |
| | | AA | DIQMTQSPSSLSASVGDRVTITCRASQGISNYLAWFQQKPGKAPKSLIYAASSLQSGVPSKFSGSGSGTDFTLTISSLQPEDFATYYCLHYLTYPLTFGGGTKVEIK | QVQLVESGGGVVQPGRSLRLSCAASGFTFSSYGMHWVRQAPGKGLEWVAVIWYDGSNEHYADSVKGRFTISRDNSKNTLYLQMNSLRAEDTAVYYCARDRGVGYYGLDVWGQGTTVTVSS |
| | | | SEQ ID NO: 26734 | SEQ ID NO: 30740 |
| **iPS:435973** | **21-225_192H3** | NA | GAAATTGTGTTGACGCAGTCTCCAGGCACCCTGTCTTTGTCTCCAGGGGAAAGAGCCACCCTCTCCTGCAGGGCCAGTCAGAGTGTTAGCAGCAACTTCTTAGCCTGGTACCAGCAGAAACCTGGCCAGGCTCCCAGGCTCCTCATCTTTGGTGCATCCAGCAGGGCCACTGGCATCCCAGACAGGTTCAGTGGCAGTGGGTCTGGGACAGACTTCACTCTCACCATCAACAGACTGGAGCCTGAAGATTTTGCAGTGTATTACTGTCAGCAATATGGTATCTCACCGTGGACGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGTTAGTTACTACTGGAGCTGGATCCGCCAGCGCCCAGGGAAGGGCCTGGAGTGGATTGGGAACCTCTATTACAGTGGGAGCACCTACTACAACCCGTCCCTCAGGAGTCGAGCTACCATATCAGTAGACACGTCTAAGAACCAGTTCTCCCTGAAGCTGAGTTCTGTGACTGCCGCGGACACGGCCGTGTATTACTGTACGAGAGGGGATTACGATGGTTCGGGGAGTTATCACTACTACCACGGTATGGACGTCTGGGGCCAAGGGACCACGGTCACCGTCTCCTCA |
| | | | SEQ ID NO: 26735 | SEQ ID NO: 30741 |

FIGURE 50

| | | | | |
|---|---|---|---|---|
| | | AA | EIVLTQSPGTLSLSPGERATLSCRASQSVSSNFLAWYQQKPGQAPRLLIFGASSRATGIPDRFSGSGSGTDFTLTINRLEPEDFAVYYCQQYGISPWTFGQGTKVEIK | QVQLQESGPGLVKPSQTLSLTCTVSGGSISSVSYYWSWIRQRPGKGLEWIGNLYYSGSTYYNPSLRSRATISVDTSKNQFSLKLSSVTAADTAVYYCTRGDYDGSGSYHYYHGMDVWGQGTTVTVSS |
| | | | SEQ ID NO: 26736 | SEQ ID NO: 30742 |
| iPS:435977 | 21-225_192E4 | NA | GACATCCAGATGACCCAGTCTCCATCCTCACTGTCTGCATCTGTAGGAGACAGAGTCACCATCACTTGTCGGGCGAGTCAGGGCATTGGCAATTATTTAGCCTGGTTTCAGCAGAAACCAGGGAAAGCCCCTAAGTCCCTGATCTATGTTGTATCCAGTTTACAAAGTGGGGTCCCATCAAGGTTCAGCGGCAGTGGATCTGGGACAGATTTCACTCTCACCATCAGCAGCCTGCAGCCTGAAGATTTTGCGACTTATTACTGCCAACGGTATGATACTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGTTATGGCATGCACTGGGTCCGCCAGGCTCCAGGCAAGGGGCTGGAGTGGGTGGCAGTTATATGGTATGATGGAAGTAACAAAAACTATGTAGACTCCGTGAGGGGCCGATTCACCATCTCCAGAGACAACTCCAAGAACACGCTGTATCTGCAAATGAACAGCCTGAGAGCCGAGGACACGGCTGTGTATTATTGTGCGAGAGATAGAAGCGTCGGCTACGACGGTATGGACGTCTGGGGCCAAGGGACCACGGTCACCGTCTCCTCA |
| | | | SEQ ID NO: 26737 | SEQ ID NO: 30743 |
| | | AA | DIQMTQSPSSLSASVGDRVTITCRASQGIGNYLAWFQQKPGKAPKSLIYVVSSLQSGVPSRFSGSGSGTDFTLTISSLQPEDFATYYCQRYDTYPFTFGPGTKVDIK | QVQLVESGGGVVQPGRSLRLSCAASGFTFSSYGMHWVRQAPGKGLEWVAVIWYDGSNKNYVDSVRGRFTISRDNSKNTLYLQMNSLRAEDTAVYYCARDRSVGYDGMDVWGQGTTVTVSS |
| | | | SEQ ID NO: 26738 | SEQ ID NO: 30744 |

FIGURE 50

| | | | | |
|---|---|---|---|---|
| **iPS:435979** | **21-225_192H4** | NA | GACATCCAGATGACCCAGTCTCCATCCTCACTGTCTGCATCTGTAGGAGACAGAGTCACCATCACTTGTCGGGCGAGTCAGGACATTAGCAATTATTTAGCCTGGTTTCAGCAGAAACCAGGGAAAGCCCCTAAGTCCCTGATCTCTGCTGCATCCAGTTTGCAAAGTGGGGTCCCATCAAAGTTCAGCGGCAGTAGATCTGGGACAGATTTCACTCTCACCATCAGCAGCCTGCAGCCTGAAGATTTTGCAACTTATTACGGCCTACATTATCTCAATTACCCGCTCACTTTCGGCGGAGGGACCAGGGTGGAGATCAGA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGCAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCAAGGTGTGGGGTACTACGGTATGGACGTCTGGGGCCAAGGGACCACGGTCACCGTCTCCTCA |
| | | | SEQ ID NO: 26739 | SEQ ID NO: 30745 |
| | | AA | DIQMTQSPSSLSASVGDRVTITCRASQDISNYLAWFQQKPGKAPKSLISAASSLQSGVPSKFSGSRSGTDFTLTISSLQPEDFATYYGLHYLNYPLTFGGGTRVEIR | QVQLVESGGGVVQPGRSLRLSCAASGFTFSSYGMHWVRQAPGKGLEWVAVIWYDGSNKNYADSVKGRFTISRDNSKNTLYLQMNSLRAEDTAVYYCARDQGVGYYGMDVWGQGTTVTVSS |
| | | | SEQ ID NO: 26740 | SEQ ID NO: 30746 |
| **iPS:435983** | **21-225_192E5** | NA | GAAATTGTTCTGACTCAGTCTCCAGATTTTCAGTCTGTGACTCCAAAGGAGAAAGTCACCATCACCTGCCGGGCCAGTCAGAGCATTGGTAGGAGTTTACACTGGTACCAGCAGAAACCAGATCAGTCTCCAAAGCTCCTCATCAAGTATGCTTCCCAGTCATTCTCAGGGGTCCCCTCGAGGTTCAGTGGCAGTGGATCTGGGACAGATTTCACCCTCACCATCAATAGCCTGGAGGCTGAAGATGCTGCAACGTATTACTGTCATCAGAGTAGTCGTTTACCGCTCACTTTCGGCGGAGGGACCAAGGTGGAGATCAAA | CAGGTGCAGCTGCAGGAGTCGGGCCCAGGACTGGTGAAGCCTTCACAGACCCTGTCCCTCACCTGCACTGTCTCTGGTGACTCCATCAATAATGGTGGATACTACTGGAGCTGGATCCGCCAGCACCCAGGGAAGGGCCTGGAGTGGATTGGATACATCTTTTACAGCGGGAGCACCTACTACAACCCGTCCCTCAAGAGTCGAGTTACCATATCAGTTGACACGTCTAAGAATCAGTTCTCCCTGAAACTGAACTCTGTGACTGCCGCGGACACGGCCGTGTATTTTTGTGCGAGAGCGGGATATAACTGGGACAACGGGTTTGACTACTGGGGCCAGGGAACCCTGGTCACCGTCTCCTCA |
| | | | SEQ ID NO: 26741 | SEQ ID NO: 30747 |

FIGURE 50

| | | | | |
|---|---|---|---|---|
| | | AA | EIVLTQSPDFQSVTPKEKVTITCRASQSIGRSLHWYQQKPDQSPKLLIKYASQSFSGVPSRFSGSGSGTDFTLTINSLEAEDAATYYCHQSSRLPLTFGGGTKVEIK | QVQLQESGPGLVKPSQTLSLTCTVSGDSINNGGYYWSWIRQHPGKGLEWIGYIFYSGSTYYNPSLKSRVTISVDTSKNQFSLKLNSVTAADTAVYFCARAGYNWDNGFDYWGQGTLVTVSS |
| | | | SEQ ID NO: 26742 | SEQ ID NO: 30748 |
| iPS:435985 | 21-225_192F6 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCTGAAACCAGGGAAAGCCCCTAAGCGCCTGATCTATGCTGCATCCAGTTTGCAAAGTGGGGTCCCATCAAGGTTCAGCGGCAGTGGATCTGGGCCAGAATTCACTCTCACAATCAGCAGCCTGCAGCCTGAAGATTTTGCAACTTATTACTGTCTACAGCATTATAGTTTCCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTTTATCATGCACTGGGTCCGCCAGGCTCCAGGCAGGGGGCTGGAGTGGGTGGCAGTTATATGGTATGATGGAAGTAATAAATACTATGTAGACTCCGTGAAGGGCCGATTCACCATCTCCAGAGACAATTCCAAGAACACGCTGTATCTGCAAATGAACAGCCTGAGAGCCGAGGACACGGCTGTGTACTACTGTGCGAGAGAGGAGTATAGTAGCGGCTGGTTCGGGTACGGTATGGACGTCTGGGGCCAAGGGACCACGGTCACCGTCTCCTCA |
| | | | SEQ ID NO: 26743 | SEQ ID NO: 30749 |
| | | AA | DIQMTQSPSSLSASVGDRVTITCRASQGIRNDLGWYQLKPGKAPKRLIYAASSLQSGVPSRFSGSGSGPEFTLTISSLQPEDFATYYCLQHYSFPFTFGPGTKVDIK | QVQLVESGGGVVQPGRSLRLSCAASGFTFSSFIMHWVRQAPGRGLEWVAVIWYDGSNKYYVDSVKGRFTISRDNSKNTLYLQMNSLRAEDTAVYYCAREEYSSGWFGYGMDVWGQGTTVTVSS |
| | | | SEQ ID NO: 26744 | SEQ ID NO: 30750 |

FIGURE 50

| | | | | |
|---|---|---|---|---|
| **iPS:435987** | **21-225_192G6** | NA | GACATCAAGATGACCCAGTCTCCTTCCTCACTGTCTGCATCTGTAGGAGACAGAGTCACCATCACTTGTCGGACGAGTCAGGGCATTGGCAATTATTTAGCCTGGTTTCAGCAGAAACCAGGGAAAGCCCCTAAGTCCCTGCTCTATAAAGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATATGACTTACCCGCTCACTTTCGGCGGGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTTTATGGTATGATGGAACTAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCAGGGCGTGGGCTACGACGGTTTGGACGTCTGGGGCCAAGGGACCACGGTCACCGTCTCCTCA |
| | | | SEQ ID NO: 26745 | SEQ ID NO: 30751 |
| | | AA | DIKMTQSPSSLSASVGDRVTITCRTSQGIGNYLAWFQQKPGKAPKSLLYKASSLQSGVPSKFSGSGSGTDFTLTISSLQPEDFATYYCQQYMTYPLTFGGGTKVEIK | QVQLVESGGGVVQPGRSLRLSCAASGFTFSSYGMHWVRQAPGKGLEWVAVLWYDGTNKNYADSVKGRFTISRDNSKNTLYLQMNSLRAEDTAVYYCARDQGVGYDGLDVWGQGTTVTVSS |
| | | | SEQ ID NO: 26746 | SEQ ID NO: 30752 |
| **iPS:435989** | **21-225_192F7** | NA | GACATCCAGATGACCCAGTCTCCATCCTCCCTGTCTGCGTCTGTAGGAGACAGAGTCACCATCACTTGCCGGGCAAGTCAGGGCATTAGAAAAGATTTAGGCTGGTATCAACAGAAACCAGGGAAAGCCCCTAAGCGCCTAATCCATACTGCATCCAGTTTGCAAAGTGGGGTCCCATTAAGGTTCAGCGGCAGTGGATCTGGGACAGAATTCACTCTCACAATCAGCAGCCTGCAGCCTGAAGATTTTGCAACTTATTACTGTCTACAGCATACTAGTTACCCGTGGACGTTCGGCCAAGGGACCAAGGTGGAAATCTAC | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAGTGGGTGACAGTTATATCATATGATGGAGGTTATAAAAACTATGCAGACTCCGTGAAGGGCCGATTCACCATCTCCAGAGACAATTCCAAGAACACGCTGTATCTGCAAATGAATAGCCTGAGAGCTGAGGACACGGCTGTGTATTACTGTGCGAGAGGTACCCACGGGTACTACTACGGTGTGGACGTCTGGGGCCAAGGGACCACGGTCACCGTCTCCTCA |
| | | | SEQ ID NO: 26747 | SEQ ID NO: 30753 |

FIGURE 50

| | | | | |
|---|---|---|---|---|
| | | AA | DIQMTQSPSSLSASVGDRVTITCRASQGIRKDLG WYQQKPGKAPKRLIHTASSLQSGVPLRFSGSGS GTEFTLTISSLQPEDFATYYCLQHTSYPWTFGQG TKVEIY | QVQLVESGGGVVQPGRSLRLSCAASGFTFSSYGMH WVRQAPGKGLEWVTVISYDGGYKNYADSVKGRF TISRDNSKNTLYLQMNSLRAEDTAVYYCARGTHG YYYGVDVWGQGTTVTSS |
| | | | SEQ ID NO: 26748 | SEQ ID NO: 30754 |
| **iPS:435993** | **21-225_192C8** | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGAACAGATTTCACTCTCACCATC AGCAGCCTGCAGCCTGAGGATTTTGCTACTTA TTACTGCCAACATTATCTTACTTACCCTCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATGAACACTATGCAGACTCCGTGAAGGGCCGA TTCATGATCTCCAGAGACAATTCCAAGAACACGC TGTATCTGCAAATGAACAGCCTGAGAGCCGAGG ACACGGCTGTGTATTACTGTGCGAGAGATAGGGG AGTGGGTTACTACGGTATGGACGTCTGGGGCCAA GGGACCACGGTCACCGTCTCCTCA |
| | | | SEQ ID NO: 26749 | SEQ ID NO: 30755 |
| | | AA | DIQMTQSPSSLSASVGDRVTITCRASQGISNYLA WFQQKPGKAPKSLIYAASSLQSGVPSKFSGSGSG TDFTLTISSLQPEDFATYYCQHYLTYPLTFGGGT KVEIK | QVQLVESGGGVVQPGRSLRLSCAASGFTFSSYGMH WVRQAPGKGLEWVAVIWYDGSNEHYADSVKGRF MISRDNSKNTLYLQMNSLRAEDTAVYYCARDRGV GYYGMDVWGQGTTVTVSS |
| | | | SEQ ID NO: 26750 | SEQ ID NO: 30756 |

FIGURE 50

| | | | | |
|---|---|---|---|---|
| **iPS:435995** | **21-225_192F8** | NA | GAAATTGTGTTGACGCAGTCTCCAGGCACCCTGTCTTTGTCTCCAGGGGAAAGAGCCACCCTCTCCTGCAGGGCCAGTCAGAGTATTAGCAGCAGCTACTTAGCCTGGTACCAGCAGAAACCTGGCCAGGCTCCCGGCTCCTCATCTATGGTGCATCCAGCAGGGCCACTGGCATCCCAGCCAGGTTCAGTGGCAGTGGGTCTGGGACAGACTTCACTCTCACCATCAGCAGACTGGAGCCTGAAGATTTTGCAGTGTATTACTGTCAGCAGTATGAGAGTTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGGTTGCTACTGGAGCTGGATCCGCCAGCCCCCAGGGAAGGGGCTGGAGTGGATTGGGGAAATCAATCAAAGTGGAAGGTCCAACTACAACCCGTCCCTCAAGAGTCGAGTCACCATTTCAGTAGACACGTCCACGAACCAGTTCTCCCTGAAGCTGAGATCTGTGACCGCCGCGGACACGGCTGTGTATTACTGTGCGAGAGACTACGGTGTCTTTGACTACTGGGGCCAGGGCACCCTGGTCACCGTCTCCTCA |
| | | | SEQ ID NO: 26751 | SEQ ID NO: 30757 |
| | | AA | EIVLTQSPGTLSLSPGERATLSCRASQSISSSYLAWYQQKPGQAPRLLIYGASSRATGIPARFSGSGSGTDFTLTISRLEPEDFAVYYCQQYESSPWTFGQGTKVEIK | QVQLQQWGAGLLKPSETLSLTCAVYGGSFSGCYWSWIRQPPGKGLEWIGEINQSGRSNYNPSLKSRVTISVDTSTNQFSLKLRSVTAADTAVYYCARDYGVFDYWGQGTLVTVSS |
| | | | SEQ ID NO: 26752 | SEQ ID NO: 30758 |
| **iPS:435997** | **21-225_192G8** | NA | GACATCCAGATGACCCAGTCTCCTTCCTCACTGTCTGCATCTGTAGGAGACAGAGTCACCATCACTTGTCGGACGAGTCAGGGCATTGGCAATTATTTAGCCTGGTTTCAGCAGAAACCAGGGAAAGCCCCTAAGTCCCTGCTCTATAAAGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATATCACTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCAGGGCGTGGGCTACGACGGTTTGGACGTCTGGGGCCAAGGGACCACGGTCACCGTCTCCTCA |
| | | | SEQ ID NO: 26753 | SEQ ID NO: 30759 |

FIGURE 50

| | | | | |
|---|---|---|---|---|
| | | AA | DIQMTQSPSSLSASVGDRVTITCRTSQGIGNYLAWFQQKPGKAPKSLLYKASSLQSGVPSKFSGSGSGTDFTLTISSLQPEDFATYYCQQYITYPLTFGGGTKVEIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAVYYCARDQGVGYDGLDVWGQGTTVTVSS |
| | | | SEQ ID NO: 26754 | SEQ ID NO: 30760 |
| **iPS:435999** | **21-225_192F9** | NA | GATATTGTAATGACCCAGACTCCACTCTCTCTGTCCGTCACCCCTGGACAGCCGGCCTCCATCTCCTGCAAGTCTAGTCAGAGCCTCCTCCATAGTGATGGAAGGACCTATTTGTATTGGTACCTGCAGAGGCCAGGCCAGCCTCCACAGCTCCTGATCTGTGAGGTTTCCAACCGGTTCGCTGGAGTGACAGATAGGTTCAGTGGCAGCGGGTCAGGGACAGATTTCACACTGAAAATCAGCCGGGTGGAGGCTGGGGATGTTGGGGTTTATTACTGCATGCAAAGTATACAGCTTCCCTGGACGTTCGGCCAAGGGACCAAGGTGGAAATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGAAGCTACCACTGGAGCTGGATCCGGCAGCCCGCCGGGAAGGGACTGGAGTGGATTGGGCTTATCTATACCAGTAGGAGCACCAATTACAACCCCTCCCTCAAGAGTCGAGTCACCATGTCAGTTGACACGTCCAAGAACCAGTTCTCCCTGAAGCTGAACTCTGTGACCGCCGCGGACACGGCCGTGTATTACTGTGCGAGACTCCGGTATAACTGGAACTTCCCTTACTTTGACTACTGGGGCCAGGGAACCCTGGTCACCGTCTCCTCA |
| | | | SEQ ID NO: 26755 | SEQ ID NO: 30761 |
| | | AA | DIVMTQTPLSLSVTPGQPASISCKSSQSLLHSDGRTYLYWYLQRPGQPPQLLICEVSNRFAGVTDRFSGSGSGTDFTLKISRVEAGDVGVYYCMQSIQLPWTFGQGTKVEIK | QLQLQESGPGLVKPSETLSLTCTVSGGSIRSYHWSWIRQPAGKGLEWIGLIYTSRSTNYNPSLKSRVTMSVDTSKNQFSLKLNSVTAADTAVYYCARLRYNWNFPYFDYWGQGTLVTVSS |
| | | | SEQ ID NO: 26756 | SEQ ID NO: 30762 |

FIGURE 50

| | | | | |
|---|---|---|---|---|
| **iPS:436001** | **21-225_192C10** | NA | GACATCCAGATGACCCAGTCTCCATCCTCACT GTCTGCATCTGTGGGAGACAGAGTCACCATCA CTTGTCGGGCGAGTCAGGGCATTGGCAAGTAT TTAGCCTGGTTTCAGCAGAAACCAGGGAAAGC CCCTAAGTCACTGATCTATGCTGCATCCAGTTT GCAAAGTGGGGTCCCATCAAAGTTCAGCGGCA GTGGGTCTGGGACAGATTTCACTCTCACCATC AGCAGCCTGCAGCCTGACGATTTTGCAACTTA TTACTGCCAACGATATGATACTTACCCATTCA CTTTCGGCCCTGGGACCAAAGTGGATTTCAAA | CAGGTGCAGCTGGTGGAGTCTGGGGGAGGCGTG GTCCAGCCTGGGAGGTCCCTGAGACTCTCCTGTG CATCGTCTGGATTCACCTTCAGGAACTATGGCAT GCACTGGGTCCGCCAGGCTCCAGGCAAGGGGCT GAAATGGGTGGCAGTTATATGGTTTGATGGAAGT AATGACTACTATGCAGACTCCGTGAAGGGCCGAT TCACCATCTCCAGAGACAATTCCAAGAACACGCT GTCACTGCAAATGAACAGCCTGAGAGCCGAGGA CACGGCTGTGTATTACTGTGCGAGAGATAGAAGC GTCGGCTACGACGGTTTAGATGTCTGGGGCCAAG GGACCACGGTCACCGTCTCCTCA |
| | | | SEQ ID NO: 26757 | SEQ ID NO: 30763 |
| | | AA | DIQMTQSPSSLSASVGDRVTITCRASQGIGKYLA WFQQKPGKAPKSLIYAASSLQSGVPSKFSGSGSG TDFTLTISSLQPDDFATYYCQRYDTYPFTFGPGT KVDFK | QVQLVESGGGVVQPGRSLRLSCASSGFTFRNYGMH WVRQAPGKGLKWVAVIWFDGSNDYYADSVKGRF TISRDNSKNTLSLQMNSLRAEDTAVYYCARDRSVG YDGLDVWGQGTTVTVSS |
| | | | SEQ ID NO: 26758 | SEQ ID NO: 30764 |
| **iPS:436003** | **21-225_192G10** | NA | GACATCCAGATGACCCAGTCTCCATCCTCCCT GTCTGCATCTGTAGGAGACAGAGTCACCATCA CTTGCCGGGCAAGTCAGAGCATTAGCAACTAT TTAAATTGGTATCAGCAGACACCAGGAAAAGC CCCTAAGCTCCTGATCTATGCTGAATCCAGTTT ACAAAGTGGGGTCCCATCAAGGTTCAGTGGCA GTGGATCTGGGACAGATTTCACTCTCACCATC AGCAGTCTGCAACCTGAAGATTTTGCAACTTA CTCCTGTCAACAGAGTTACAGTTCCCCTCCGT GGACGTTCGGCCAAGGGACCAAGGTGGAAAT CAAA | GAGGTGCAGCTGTTGGAGTCTGGGGGAGGCTTG GTACAGCCTGGGGGGTCCCTGAGACTCTCCTGTT CAGCCTCTGGATTCACCTTTAGCAGCTATGCCAT GAGCTGGGTCCGCCAGGCTCCAGGGAAGGGGCT GGAGTGGGTCTCAGCTATTAGTGGTAGAGGCGGT AGTACATTCTACGCAGACTCCGTGAAGGGCCGGT TCACCATCTCCAGAGACAATTCCAAGAACACGCT GTATCTGCAAATGAACAGCCTGAGAGCCGAGGA CACGGCCGTCTATTACTGTGCGCGACGTTTAGCA CTGGATGGCTATGATGCTTTTGATATCTGGGGCC AAGGGACAATGGTCACCGTCTCTTCA |
| | | | SEQ ID NO: 26759 | SEQ ID NO: 30765 |

FIGURE 50

| | | | | |
|---|---|---|---|---|
| | | AA | DIQMTQSPSSLSASVGDRVTITCRASQSISNYLNWYQQTPGKAPKLLIYAESSLQSGVPSRFSGSGSGTDFTLTISSLQPEDFATYSCQQSYSSPPWTFGQGTKVEIK | EVQLLESGGGLVQPGGSLRLSCSASGFTFSSYAMSWVRQAPGKGLEWVSAISGRGGSTFYADSVKGRFTISRDNSKNTLYLQMNSLRAEDTAVYYCARRLALDGYDAFDIWGQGTMVTVSS |
| | | | SEQ ID NO: 26760 | SEQ ID NO: 30766 |
| **iPS:436005** | **21-225_192H10** | NA | GACATCCAGATGACCCAGTCTCCATCCTCACTGTCTGCATCTGTAGGAGACAGAGTCACCATCACTTGTCGGGCGAGTCAGGGCATTGGCAAGTATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATATGGGACAGATTTCACTCTCACCATCAGCAGCCTGCAGCCTGAAAATTTTGCAACTTATTACTGCCAACAGTATAGTACTTACCCGCTCACTTTCGGCGGAGGGACCAAGGTGGAGATCAAG | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTACGCAGACTCCGTGAAGGGCCGATTCACCATCTCCAGAGACAATTCCAAGAACACGCTGTATCTGCAAATGAACAGCCTGAGAGCCGAGGACACGGCTATGTATTACTGTGCGAGAGATCAGGGCGTGGGCTACGACGGTTTGGACGTCTGGGGCCAAGGGACCTCGGTCACCGTCTCCTCA |
| | | | SEQ ID NO: 26761 | SEQ ID NO: 30767 |
| | | AA | DIQMTQSPSSLSASVGDRVTITCRASQGIGKYLAWFQQKPGKAPKSLIYAASSLQSGVPSKFSGSGYGTDFTLTISSLQPENFATYYCQQYSTYPLTFGGGTKVEIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAMYYCARDQGVGYDGLDVWGQGTSVTVSS |
| | | | SEQ ID NO: 26762 | SEQ ID NO: 30768 |

FIGURE 50

| | | | | |
|---|---|---|---|---|
| iPS:436007 | 21-225_192G12 | NA | GAAATTGTGTTGACGCAGTCTCCAGGCACCCTGTCTTTGTCTCCAGGGGAAAGAGCCACCCTCTCCTGCAGGGCCAGTCAGAGTGTCAGAAGCGACTTCTTAGCCTGGCTCCAGCAGAAACCTGGCCAGGCTCCCAGGCTCCTCATCTATGGTGTATCCCGCAGGGCCACTGGCATCCCAGACAGGTTCAGTGGCAGTGGGTCTGGGACAGACTTCACTCTCACCATCAACAGACTGGAGCCTGAAGATTTTGCAGTGTATTACTGTCAGCAGTATGGTAACTCACCGTGGACGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGTTTACCACTGGAGCTGGATCCGCCAGCACCCAGGGAAGGGCCTGGAGTGGATTGGGAACATCCATTACAGCGGGAGCACCTACAACAACCCGTCCCTCAAGAGTCGAGTTACCATATCAGTAGACACGTCTAAGAACCAGTTCTCCCTGAAGCTGAGCTCTGTGACTGCCGCGGACACGGCCGTGTATTACTGTGCGAGAGGGGATTACGATGGTTCGGGGAGTTATCACTACTACTACGGTATGGACGTCTGGGGCCAAGGGACCACGGTCACCGTCTCCTCA |
| | | | SEQ ID NO: 26763 | SEQ ID NO: 30769 |
| | | AA | EIVLTQSPGTLSLSPGERATLSCRASQSVRSDFLAWLQQKPGQAPRLLIYGVSRRATGIPDRFSGSGSGTDFTLTINRLEPEDFAVYYCQQYGNSPWTFGQGTKVEIK | QVQLQESGPGLVKPSQTLSLTCTVSGGSISSGVYHWSWIRQHPGKGLEWIGNIHYSGSTYNNPSLKSRVTISVDTSKNQFSLKLSSVTAADTAVYYCARGDYDGSGSYHYYYGMDVWGQGTTVTVSS |
| | | | SEQ ID NO: 26764 | SEQ ID NO: 30770 |
| iPS:436009 | 21-225_193A1 | NA | GAAATTGTGTTGACGCAGTCTCCAGGCACCCTGTCTTTGTCTCCAGGGGAAAGAGCCACCCTCTCTTGTAGGGCCAGTCAGAGTGTTAGAAGCAACTTCTTAGCCTGGCACCAGCAGAAACCTGGCCAGGCTCCCAGGCTCTTCATCTATGGTGCATCCCGCAGGGCCACTGGCATCCCAGACAGGTTCAGTGGCAGTGGGTCTGGGACAGACTTCACTCTCACCATCAACAGACTGGAGCCTGAAGATTTTGCAGTGTATTACTGTCAGCAGTATGGTAACTCACCGTGGACGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGTTTACTACTGGAGCTGGATCCGCCAGCACCCAGGGAAGGGCCTGGAGTGGATTGGGAACATCTATTACAGTGGGAGCACCTACAACAACCCGTCCCTCAAGAGTCGACTTACCATATCAGCAGACACGTCTAAGAACCAGTTCTCCCTGAAGCTGAGCTCTGTGACTGCCGCGGACACGGCCGTGTATTACTGTGCGAGAGGGGATTACGATGGTTCGGGGAGTTATCACTACTACTACGGTATGGACGTCTGGGGCCAAGGGACCACGGTCACCGTCTCCTCA |
| | | | SEQ ID NO: 26765 | SEQ ID NO: 30771 |

FIGURE 50

| | | | | |
|---|---|---|---|---|
| | | AA | EIVLTQSPGTLSLSPGERATLSCRASQSVRSNFLAWHQQKPGQAPRLFIYGASRRATGIPDRFSGSGSGTDFTLTINRLEPEDFAVYYCQQYGNSPWTFGQGTKVEIK | QVQLQESGPGLVKPSQTLSLTCTVSGGSISSGVYYWSWIRQHPGKGLEWIGNIYYSGSTYNNPSLKSRLTISADTSKNQFSLKLSSVTAADTAVYYCARGDYDGSGSYHYYYGMDVWGQGTTVTVSS |
| | | | SEQ ID NO: 26766 | SEQ ID NO: 30772 |
| **iPS:436011** | **21-225_193B1** | NA | GAAATTGTGTTGACGCAGTCTCCAGGCACCCTGTCTTTGTCTCCAGGGGACAGAGCCACCCTCTCTTGCAGGGCCAGTCAGAGTGTTAGAAGCAACTTCTTAGCCTGGCACCAGCAGAAACCTGGCCAGGCTCCCAGGCTCCTCATCTATGGTGCATCCCGCAGGGCCACTGGCATCCCAGACAGGTTCAGTGGCAGTGGGTCTGGGACAGACTTCACTCTCACCATCAACAGACTGGAACCTGAAGATTTTGCAGTGTATTTCTGTCAGCAGTATGGTAACTCACCGTGGACGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGTTTACTACTGGAGCTGGATCCGCCAGCACCCAGGGAAGGGCCTGGAGTGGATTGGGAACATCTATTACAGTGGGAGCACCTACAACAACCCGTCCCTCAAGAGTCGAGTTACCATATCAGTAGACACGTCTAAGAACCAGTTCTCCCTGAAGCTGAGCTCTGTGACTGCCGCGGACACGGCCGTGTATTACTGTGCGAGAGGGGATTACGATGGTTCGGGGAGTTATCACTACTACTACGGTATGGACGTCTGGGGCCAAGGGACCACGGTCACCGTCTCCTCA |
| | | | SEQ ID NO: 26767 | SEQ ID NO: 30773 |
| | | AA | EIVLTQSPGTLSLSPGDRATLSCRASQSVRSNFLAWHQQKPGQAPRLLIYGASRRATGIPDRFSGSGSGTDFTLTINRLEPEDFAVYFCQQYGNSPWTFGQGTKVEIK | QVQLQESGPGLVKPSQTLSLTCTVSGGSISSGVYYWSWIRQHPGKGLEWIGNIYYSGSTYNNPSLKSRVTISVDTSKNQFSLKLSSVTAADTAVYYCARGDYDGSGSYHYYYGMDVWGQGTTVTVSS |
| | | | SEQ ID NO: 26768 | SEQ ID NO: 30774 |

FIGURE 50

| | | | | |
|---|---|---|---|---|
| **iPS:436013** | **21-225_193F2** | NA | GACATCCAGATGACCCAGTCTCCATCTTCCGTGTCTGCATCTGTAGGAGACAGAGTCACCATCACTTGTCGGGCGAGTCAGGGTATTAACAACTGGTTAGCCTGGTATCAGCAGAAACCAGGGAAAGCCCCTAAGCTCCTGATCTATGGTGTTTCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTCTTGTCAACAGGCTAACAGTTTCCCGTGGACGTTCGGCCAAGGGACCAAGGTGGAAATCAGA | GAGGTGCAGCTGTTGGAGTCTGGGGGAGGCTTGGTACAGCCTGGGGGGTCCCTGAGACTCTCCTGTGCAGCCTCTGGATTCACCTTTAGCACCTTTGCCATGAGTTGGGTCCGCCAGGCTCCAGGGAAGGGGCTGGAGTGGGTCTCAGCTATTAGTCGTAGTGGTGGTAACACACACTACGCAGACTCCGTGAAGGGCCGGTTCACCATCTCCAGAGACAATTCCAAGAATACGCTGTATCTGCAAATGAACAGCCTGAGAGCCGAGGACACGGCCGTATATTCCTGTGCGAAAGATGGATTCGGTGGGAGCTCCTACTTTGACTACTGGGGCCAGGGAACCCTGGTCACCGTCTCCTCA |
| | | | SEQ ID NO: 26769 | SEQ ID NO: 30775 |
| | | AA | DIQMTQSPSSVSASVGDRVTITCRASQGINNWLAWYQQKPGKAPKLLIYGVSSLQSGVPSRFSGSGSGTDFTLTISSLQPEDFATYSCQQANSFPWTFGQGTKVEIR | EVQLLESGGGLVQPGGSLRLSCAASGFTFSTFAMSWVRQAPGKGLEWVSAISRSGGNTHYADSVKGRFTISRDNSKNTLYLQMNSLRAEDTAVYSCAKDGFGGSSYFDYWGQGTLVTVSS |
| | | | SEQ ID NO: 26770 | SEQ ID NO: 30776 |
| **iPS:436015** | **21-225_193D3** | NA | GAAATTGTGTTGACGCAGTCTCCAGGCACCCTGTCTTTGTCTCCAGGGGAAAGAGCCACCCTCTCCTGCAGGGCCAGTCAGAGTATTCGCAGCAGCTTCTTAGCCTGGTATCAGCAGAAACCTGGCCAGGCTCCCAGGCTCCTCATCTATGGTGCATCCAACAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGGTAACTCACCGTGGGCGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGATTACTACTGGAACTGGATCCGCCAGCTCCCAGGGAAGGGCCTGGAGTGGATTGGGTACATCTTTTACAGTGGGAGCACCTACTACAACCCGTCCCTCAAGAGTCGACTTACCATATCAGTGGACACGTCTAAGAACCAGTTCTCCCTGAAGCTGAGCTCTGTGACTGCCGCGGACACGGCCGTGTATTACTGTACAAGGGGGGATTACGATGGTTCGGGGAGTTATCACTTCTACTACGGTATGGACGTCTGGGGCCAAGGGACCACGGTCACCGTCTCCTCA |
| | | | SEQ ID NO: 26771 | SEQ ID NO: 30777 |

FIGURE 50

| | | | | |
|---|---|---|---|---|
| | | AA | EIVLTQSPGTLSLSPGERATLSCRASQSIRSSFLAWYQQKPGQAPRLLIYGASNRATGIPDRFSGSGSGTDFTLTISRLEPEDFAVYYCQQYGNSPWAFGQGTKVEIK | QVQLQESGPGLVKPSQTLSLTCTVSGGSISSGDYYWNWIRQLPGKGLEWIGYIFYSGSTYYNPSLKSRLTISVDTSKNQFSLKLSSVTAADTAVYYCTRGDYDGSGSYHFYYGMDVWGQGTTVTVSS |
| | | | SEQ ID NO: 26772 | SEQ ID NO: 30778 |
| iPS:436017 | 21-225_193F3 | NA | GAAATTGTGTTGACGCAGTCTCCAGGCACCCTGTCTTTGTCTCCAGGGGAAAGCGCCACCCTCTCCTGCAGGGCCGGTCAAAGTATTAGAACCGACTTCTTAGTCTGGTACCAGCAGCAACCTGGCCAGGCTCCCAGGCTCCTCATCTATGGTGCATCCAGCAGGGCCACTGGCTTCCCAGAGAGGTTCAGTGGCAGTGGGTCTGGGACAGACTTCACTCTCACCATCAACAGACTGGAGCCTGAAGATTTTGCAGTGTATTACTGTCAGCAGTATGGAAGCTCACCGTGGACGTTCGGCCAAGGGACCAAGGTGGAAATCAAT | CAGGTGCAACTGCAGGAGTCGGGCCCAGGACTGGTGAAGCCTTCACAGACCCTGTCCCTCACCTGCACTGTCTCTGGTGGCTCCATCAACAGTGGTGATTACTACTGGAACTGGATCCGCCAGCACCCAGGGAAGGGCCTGGAGTGGATTGGGTACATCTTTTACAGTGGGAGCACCTACTACAACCCGTCCCTCAAGAGTCGAGTTACCATATCAGTAGACACGTCTAAGAACCAGTTCTCCCTGAAGCTGAGCTCTGTGACTGCCGCGGACACGGCCGTGTATTACTGTGCGAGAGGGGATTACGATGGTTCGGGGAGTTATCACTACTATTACGGTATGGACGTCTGGGGCCAAGGGACCACGGTCACCGTCTCCTCA |
| | | | SEQ ID NO: 26773 | SEQ ID NO: 30779 |
| | | AA | EIVLTQSPGTLSLSPGESATLSCRAGQSIRTDFLVWYQQQPGQAPRLLIYGASSRATGFPERFSGSGSGTDFTLTINRLEPEDFAVYYCQQYGSSPWTFGQGTKVEIN | QVQLQESGPGLVKPSQTLSLTCTVSGGSINSGDYYWNWIRQHPGKGLEWIGYIFYSGSTYYNPSLKSRVTISVDTSKNQFSLKLSSVTAADTAVYYCARGDYDGSGSYHYYYGMDVWGQGTTVTVSS |
| | | | SEQ ID NO: 26774 | SEQ ID NO: 30780 |

FIGURE 50

| | | | | |
|---|---|---|---|---|
| iPS:436019 | 21-225_193C4 | NA | GACATCCAGATGACCCAGTCTCCATCCTCCCTGTCTGCATCTGTTGGAGACAGAGTCACCATCACTTGCCGGCCGAGTCAGGGCATTAGCATTTATTTAGCCTGGTATCAGCAGAAACCAGGGAATGTTCCTAAGCTCCTGATCTATGCTGCATCCACTTTACAATCAGGGGTCCCATCTCGGTTCAGTGGCAGTGGATCTGGGACAGATTTCACTCTCACCATCAGCAGCCTGCAGCCTGAAGATGTTGCAACTTATTACTGTCAAAAGTATAACAGTGCCCCATTCACTTTCGGCCCTGGGACCAAAGTGGATATCAAA | GAGGTGCAGCTGTTGGAGTCTGGGGGAGGCTTGGCACAGCCTGGGGGGTCCCTGAGACTCTCCTGTGCAGCCTCTGGATTCACCTTTAGCAGCTATGCCATGAACTGGGTCCGCCAGGCTCCAGGGAAGGGGCTGGAGTGGGTCTCAGCTATTATTGGTAATGGTGGTAGAACATACTACGCAGACTCCGTGAAGGGCCGGTTCTCCATCTCCAGAGACAATTCCAAGAACACGCTGTTTCTGCAAATGAACAGCCTGAGAGCCGAGGACACGGCCGTATATTACTGTGCGAAAGATCTGGGTAGATACAGCTATGGTTTCTTTGACTACTGGGGCCAGGGAACCCTGGTCACCGTCTCCTCA |
| | | | SEQ ID NO: 26775 | SEQ ID NO: 30781 |
| | | AA | DIQMTQSPSSLSASVGDRVTITCRPSQGISIYLAWYQQKPGNVPKLLIYAASTLQSGVPSRFSGSGSGTDFTLTISSLQPEDVATYYCQKYNSAPFTFGPGTKVDIK | EVQLLESGGGLAQPGGSLRLSCAASGFTFSSYAMNWVRQAPGKGLEWVSAIIGNGGRTYYADSVKGRFSISRDNSKNTLFLQMNSLRAEDTAVYYCAKDLGRYSYGFFDYWGQGTLVTVSS |
| | | | SEQ ID NO: 26776 | SEQ ID NO: 30782 |
| iPS:436021 | 21-225_193G4 | NA | GACATCGTGATGACCCAGTCTCCAGACTCCCTGGCTGTGTCTCTGGGCGAGAGGGCCACCATCAACTGCAAGTCCAGCCAGAGTGTTTTATACAGCTCCAACAATTATAACTACTTGACTTGGTACCAGCAGAAACCAGGACAGCCTCCTAAGCTGCTCATTTACTGGGCATCTACCCGAAAATCCGGGGTCCCTGACCGATTCAGTGGCAGCGGGTCTGGGACAGATTTCACTCTCACCATCAGCAGCCTGCAGGCTGACGATGTGGCAGTTTATTACTGTCAGCAATATTATATTACTCCGTGGACGTTCGGCCAAGGGACCAAGGTGGACATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGAACAGCCTACATGGAGCTGAACAGCCTGAGATCTGAGGACACGGCCGTGTATTACTGTGCGAGTAGCAGTGGCTGGTACTTCTTTGACTACTGGGGCCAGGGAACCCTGGTCACCGTCTCCTCA |
| | | | SEQ ID NO: 26777 | SEQ ID NO: 30783 |

FIGURE 50

| | | | | |
|---|---|---|---|---|
| | | AA | DIVMTQSPDSLAVSLGERATINCKSSQSVLYSSNNYNYLTWYQQKPGQPPKLLIYWASTRKSGVPDRFSGSGSGTDFTLTISSLQADDVAVYYCQQYYITPWTFGQGTKVDIK | QVQLVQSGAEVKKPGASVKVSCKASGYTFTNYDINWVRQATGQGLEWMGWMNPNSGNTGYAQKFQGRVTMTRNTSIRTAYMELNSLRSEDTAVYYCASSSGWYFFDYWGQGTLVTVSS |
| | | | SEQ ID NO: 26778 | SEQ ID NO: 30784 |
| iPS:436023 | 21-225_193A5 | NA | GACATCCAGATGACCCAGTCTCCATCCTCCCTGTTTGCATCTGTAGGAGACAGAGTCATCATCTCTTGCCGGGCAAGTCAGGGCATTAGAAATGATTTAGGGTGGTATCAGCAGTATCCAGGGAAAGCCCCTAAGCGCGTGATTTATGCTGCATCCAGTTTGCAAAGTGGGGTCCCATCAAGGTTCAGCGGCAGTGGATTTGGGACAGAATTCACTATCACAATCAGCAGCGTGCAGCCTGAAGATTTTGAAACTTATTACTGTCTACAGCATAATGATTTCCCGTTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCATCAGTTATGATATCAACTGGGTGCGACAGGCCACTGGACAAGGGCTTGAGTGGATGGGATGGATGAACCCTAAAAGGGGTAACACAGGCTATGCACAGAAGTTCCAGGGCAGAGTCACCATGACCAGGGACACCTCCATAAGCACAGCCTACATGGAGCTGAGCAGCCTGAGATCTGAGGACACGGCCGTTTATTACTGTGCGAGAGGAGACCCGTATAACTGGAACTCCTACGCTATGGACGTCTGGGGCCAAGGGGCCACGGTCACCGTCTCCTCA |
| | | | SEQ ID NO: 26779 | SEQ ID NO: 30785 |
| | | AA | DIQMTQSPSSLFASVGDRVIISCRASQGIRNDLGWYQQYPGKAPKRVIYAASSLQSGVPSRFSGSGFGTEFTITISSVQPEDFETYYCLQHNDFPFTFGGGTKVEIK | QVQLVQSGAEVKKPGASVKVSCKASGYTFISYDINWVRQATGQGLEWMGWMNPKRGNTGYAQKFQGRVTMTRDTSISTAYMELSSLRSEDTAVYYCARGDPYNWNSYAMDVWGQGATVTVSS |
| | | | SEQ ID NO: 26780 | SEQ ID NO: 30786 |

FIGURE 50

| | | | | |
|---|---|---|---|---|
| **iPS:436025** | **21-225_193B5** | NA | GAAATTGTGCTGACTCAGTCTCCAGACTTTCA GTCTGTGACTCCAAAGGAGAAAGTCACCATCA CCTGCCGGGCCAGTCAGAGCATTGGTAGTAGC TTACACTGGTACCAGCAGAAACCAGATCAGTC TCCAAAGCTCCTCATCAAGTATGCTTCCCAGT CCTTCTCAGGGGTCCCCTCGAGGTTCAGTGGC AGTGGATCTGGGACAGATTTCACCCTCACCAT CAATAGCCTGGAAGCTGAAGATGCTGCAACGT ATTACTGTCATCAGAGTAGTCGTTTACCATTCA CTTTCGGCCCTGGGACCAAAGTGGATATCAAA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTTA CTACTGGAGCTGGATCCGCCAGCACCCAGGGAA GGGCCTGGAGTGGATTGGGTACATCTATTACAGT GGGAGCACCTACTACAACCCGTCCCTCAAGAGTC GAGTTACCATATCAGTAGACACGTCTAAGAACCA GTTCTCCCTGAAGCTGAGCTCTGTGGCTGCCGCG GACACGGCCGTGTATTATTGTGCGAGAGGAGAGT ATAACTGGAACCACGGTATGGACGTCTGGGGCC AAGGGACCACGGTCACCGTCTCCTCA |
| | | | SEQ ID NO: 26781 | SEQ ID NO: 30787 |
| | | AA | EIVLTQSPDFQSVTPKEKVTITCRASQSIGSSLHW YQQKPDQSPKLLIKYASQSFSGVPSRFSGSGSGT DFTLTINSLEAEDAATYYCHQSSRLPFTFGPGTK VDIK | QVQLQESGPGLVKPSQTLSLTCTVSGGSISSGGYYW SWIRQHPGKGLEWIGYIYYSGSTYYNPSLKSRVTIS VDTSKNQFSLKLSSVAAADTAVYYCARGEYNWNH GMDVWGQGTTVTVSS |
| | | | SEQ ID NO: 26782 | SEQ ID NO: 30788 |
| **iPS:436027** | **21-225_193E6** | NA | GAAATTGTGTTGGCGCAGTCTCCAGGCACCCT GTCTTTGTCTCCAGGGGAAAGAGCCACCCTCT CCTGCAGGGCCAGTCAGAGTGTTAGGAGCGGT TACTTAGCCTGGTACCAGCAGAAACCTGGCCA GGCTCCCAGGCTCCTCATCTATGGTGCATCCA GCAGGGCCACTGGCATCCCAGACAGGTTCGGT GGCAGTGGGTCTGGGACAGACTTCACTCTCAC CATCAGCAGACTGGAGCCTGAAGATTTTGCAG TGTATTACTGTCAGCAGTATGAGAGTTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCTTTGGTGGGTCCTTCAGTGGTCCCTACTGG AGTTGGATCCGCCAGCCCCCAGGGAAGGGGCTG GAGTGGATTGGGGAATCCAATCATAGTGGACGC ACCAACTACAACCCGTCCCTCAAGAGTCGAGTCA CCATATCAGTGGACACGTCCAAGAACCAGTTCTC CCTGAGGCTGAGCTCTGTGACCGCCGCGGACACG GCTGTGTATTACTGTGCGAGAGACTACGGTGGTT TGGACTACTGGGGCCAGGGAACCCTGGTCACCGT CTCCTCA |
| | | | SEQ ID NO: 26783 | SEQ ID NO: 30789 |

FIGURE 50

| | | | | |
|---|---|---|---|---|
| | | AA | EIVLAQSPGTLSLSPGERATLSCRASQSVRSGYLAWYQQKPGQAPRLLIYGASSRATGIPDRFGGSGSGTDFTLTISRLEPEDFAVYYCQQYESSPWTFGQGTKVEIK | QVQLQQWGAGLLKPSETLSLTCAVFGGSFSGPYWSWIRQPPGKGLEWIGESNHSGRTNYNPSLKSRVTISVDTSKNQFSLRLSSVTAADTAVYYCARDYGGLDYWGQGTLVTVSS |
| | | | SEQ ID NO: 26784 | SEQ ID NO: 30790 |
| **iPS:436029** | **21-225_193H6** | NA | GAAATTGTGTTGACGCAGTCTCCAGGCACCCTGTCTTTGTCTCCAGGGGAAAGCGCCACCCTCTCCTGCAGGGCCGGTCAAAGTATTAGAACCAACTTCTTAGCCTGGTACCAGCAGCAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAACAGACTGGAGCCTGAAGATTTTGCAGTGTATTTCTGTCAGCAGTATGGTAGCTCACCGTGGACGTTCGGCCAAGGGACCAAGGTGGAAATCAAT | CAGGTGCAACTGCAGGAGTCGGGCCCAGGACTGGTGAAGCCTTCACAGACCCTGTCCCTCACCTGCACTGTCTCTGGTGGCTCCATCAACAGTGGTGATTACTACTGGAACTGGATCCGCCAGCACCCAGGGAAGGGCCTGGAGTGGATTGGGTACATCTTTTACAGTGGGAGCACCTACTACAACCCGTCCCTCAAGAGTCGAGTTACCATATCAGTAGACACGTCTAAGAACCAGTTCTCCCTGAAGCTGAGCTCTGTGACTGCCGCGGACACGGCCGTGTATTACTGTGCGAGAGGGGATTACGATGGTTCGGGGAGTTATCACTACTATTACGGTATGGACGTCTGGGGCCAAGGGACCACGGTCACCGTCTCCTCA |
| | | | SEQ ID NO: 26785 | SEQ ID NO: 30791 |
| | | AA | EIVLTQSPGTLSLSPGESATLSCRAGQSIRTNFLAWYQQQPGQAPRLLIYGASSRATGIPDRFSGSGSGTDFTLTINRLEPEDFAVYFCQQYGSSPWTFGQGTKVEIN | QVQLQESGPGLVKPSQTLSLTCTVSGGSINSGDYYWNWIRQHPGKGLEWIGYIFYSGSTYYNPSLKSRVTISVDTSKNQFSLKLSSVTAADTAVYYCARGDYDGSGSYHYYYGMDVWGQGTTVTVSS |
| | | | SEQ ID NO: 26786 | SEQ ID NO: 30792 |

FIGURE 50

| | | | | |
|---|---|---|---|---|
| **iPS:436031** | **21-225_193C7** | NA | GACATCCAGATGACCCAGTCTCCATCCTCACTGTCTGCATCTGTAGGAGACAGAGTCACCATCACTTGTCGGGCGAGTCAGGGCATTGGCAAGTATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATAGTACTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTACGCAGACTCCGTGAAGGGCCGATTCACCATCTCCAGAGACAATTCCAAGAACACGCTGTATCTGCAAATGAACAGCCTGAGAGCCGAGGACACGGCTATGTATTACTGTGCGAGAGATCAGGGCGTGGGCTACGACGGCTTGGACGTCTGGGGCCAAGGGACCTCGGTCACCGTCTCCTCA |
| | | | SEQ ID NO: 26787 | SEQ ID NO: 30793 |
| | | AA | DIQMTQSPSSLSASVGDRVTITCRASQGIGKYLAWFQQKPGKAPKSLIYAASSLQSGVPSKFSGSGSGTDFTLTISSLQPEDFATYYCQQYSTYPLTFGGGTKVEIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAMYYCARDQGVGYDGLDVWGQGTSVTVSS |
| | | | SEQ ID NO: 26788 | SEQ ID NO: 30794 |
| **iPS:436033** | **21-225_193E7** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTAATCTATTCTGCATCCAGTTTGCAAAGGGGGGTCCCATCAAGATTCAGCGGCAGTGGATCTGGGACAGAATTCACTCTCACAATCAGCAGCCTGCAGCCTGAAGATTTTGCAACTTATTACTGTCTACAGCATAAAAGGTACCCGCTCACTTTCGGCGGAGGGACCAAGGTGGAGATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGGTTACTTCTGGACCTGGATCCGCCAGCCCCCAGGGAAGGGGCTGGAGTGGATTGGGGAAATCAATCATAGTGGAAGCACCAACTACAACCCGTCCCTCAAGAGTCGCGTCACCATATCAGTAGACACGTCCAAGAACCAGTTCTCCCTGAAGCTGAGCTCTGTGACCGCCGCGGACACGGCTGTGTATTACTGTGCGAGAGACTACGGTGCTGACTACTGGGGCCAGGGAACCCTGGTCGCCGTCTCCGCA |
| | | | SEQ ID NO: 26789 | SEQ ID NO: 30795 |

FIGURE 50

| | | | | |
|---|---|---|---|---|
| | | AA | DIQMTQSPSSLSASVGDRVTITCRASQGIRNDLGWYQQKPGKAPKRLIYSASSLQRGVPSRFSGSGSGTEFTLTISSLQPEDFATYYCLQHKRYPLTFGGGTKVEIK | QVQLQQWGAGLLKPSETLSLTCAVYGGSFSGYFWTWIRQPPGKGLEWIGEINHSGSTNYNPSLKSRVTISVDTSKNQFSLKLSSVTAADTAVYYCARDYGADYWGQGTLVAVSA |
| | | | SEQ ID NO: 26790 | SEQ ID NO: 30796 |
| **iPS:436035** | **21-225_193C8** | NA | GAAATTGTGTTGACGCAGTCTCCAGGCACCCTGTTTTTGTCTCCAGGGGAAAGAGCCACCCTCTCCTGCAGGGCCAGTCAGAGTATTCGCAGCAGCTTCTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAATATGGTAACTCACCGTGGGCGTTCGGCCAAGGGATCAAGGTGGAAGTCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGATTACTACTGGAACTGGATCCGCCAGCACCCAGGGAAGGGCCTGGAGTGGATTGGGTACATCTTTTACAGTGGGAGCACCTACTACAACCCGTCCCTCAAGAGTCGAGTTACCATATCAGTAGACACGTCTAAGAACCAGTTCTCCCTGAAGCTGAGCTCTGTGACTGCCGCGGACACGGCCGTGTATTACTGTGCGAGAGGGGATTACGATGGTTCGGGGAGTTATCACTACTACTACGGTATGGACGTCTGGGGCCAAGGGACCACGGTCACCGTCTCCTCA |
| | | | SEQ ID NO: 26791 | SEQ ID NO: 30797 |
| | | AA | EIVLTQSPGTLFLSPGERATLSCRASQSIRSSFLAWYQQKPGQAPRLLIYGASSRATGIPDRFSGSGSGTDFTLTISRLEPEDFAVYYCQQYGNSPWAFGQGIKVEVK | QVQLQESGPGLVKPSQTLSLTCTVSGGSISSGDYYWNWIRQHPGKGLEWIGYIFYSGSTYYNPSLKSRVTISVDTSKNQFSLKLSSVTAADTAVYYCARGDYDGSGSYHYYYGMDVWGQGTTVTVSS |
| | | | SEQ ID NO: 26792 | SEQ ID NO: 30798 |

FIGURE 50

| | | | | |
|---|---|---|---|---|
| **iPS:436037** | **21-225_193D8** | NA | GAAATTGTGTTGAAGCAGTCTCCAGGCACCCTGTTTTTGTCTCCAGGGGAGAGAGCCACCCTCTCCTGCAGGGCCAGTCAGAGTATAAGGACCAACTTCTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAACAGACTGGAGCCTGAAGATTTTGCAGTGTATTACTGTCAGCAGTATGGTAACTCACCGTGGACGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGGCTCCATCAACAGTGGTGGTTACTACTGGAACTGGATCCGCCAGCACCCAGGGAAGGGCCTGGAGTGGATTGGGTTCATCTTTTACAGTGGGAGCACCTACTACAACCCGTCCCTCAAGAGTCGAGTTTCCATATCAGTAGACACGTCTAAGAACCAGTTCTCCCTGAAGCTGAGCTCTGTGACTGCCGCGGACACGGCCGTGTATTACTGTGCGAGAGGGGATTACGATGGTTCGGGGAGTTATCACTACTATTACGGTATGGACGTCTGGGGCCAGGGGACCACGGTCACCGTCTCCTCA |
| | | | SEQ ID NO: 26793 | SEQ ID NO: 30799 |
| | | AA | EIVLKQSPGTLFLSPGERATLSCRASQSIRTNFLAWYQQKPGQAPRLLIYGASSRATGIPDRFSGSGSGTDFTLTINRLEPEDFAVYYCQQYGNSPWTFGQGTKVEIK | QVQLQESGPGLVKPSQTLSLTCTVSGGSINSGGYYWNWIRQHPGKGLEWIGFIFYSGSTYYNPSLKSRVSISVDTSKNQFSLKLSSVTAADTAVYYCARGDYDGSGSYHYYYGMDVWGQGTTVTVSS |
| | | | SEQ ID NO: 26794 | SEQ ID NO: 30800 |
| **iPS:436039** | **21-225_193F8** | NA | GACATCCAGATGATCCAGTCTCCATCCTCACTGTCTGCATCTGTAGGAGACAGAGTCACCATCACTTGTCGGGCGAGTCAGGGCGTTAGCAATCATTTAGCCTGGTTTCAGCAGAAACCAGGGAGAGCCCCTAAGTCCCTGATCTATGCTGCATCCAGTTTGCAAAGTGGGGTCCCATCAAAGTTCAGCGGCAGTGGATCTGGGGCAGATTTCACTCTCACCATCAGCAGCCTGCAGCCTGAAGATTTTGCAACTTATTACTGCCAACAGTATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTATCTATGGCATGGACTGGGTCCGCCAGGCTCCAGGCAAGGGGCTGGAGTGGGTGGCAGTTATATGGTATGATGGAAGTTATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTATTGTGTGAGAGAGGATTCCCCTTATAGTGGCTACGGCTTGGACTACTACTACGGTATGGACGTCTGGGGCCAAGGGACCACGGTCACCGTCTCCTCA |
| | | | SEQ ID NO: 26795 | SEQ ID NO: 30801 |

FIGURE 50

| | | | | |
|---|---|---|---|---|
| | | AA | DIQMIQSPSSLSASVGDRVTITCRASQGVSNHLA WFQQKPGRAPKSLIYAASSLQSGVPSKFSGSGSG ADFTLTISSLQPEDFATYYCQQYNSYPFTFGPGT KVDIK | QVQLVESGGGVVQPGRSLRLSCAASGFTFSIYGMD WVRQAPGKGLEWVAVIWYDGSYKYYADSVKGRF TISRDNSKNTLYLQMNSLRAEDTAVYYCVREDSPY SGYGLDYYYGMDVWGQGTTVTVSS |
| | | | SEQ ID NO: 26796 | SEQ ID NO: 30802 |
| **iPS:436041** | **21-225_193G8** | NA | GAAATTGTGTTGACGCAGTCTCCAGGCACCCT GTCTTTGTCTCCAGGGGAAAGAGCCACCCTCT CCTGCAGGGCCAGTCAGAGTGTTAGAACCAAC TTCTTAGCCTGGCACCAGCAGAAACCTGGCCA GGCTCCCAGGCTCCTCATCTATGGTGCATCCC GCAGGGCCACTGGCATCCCAGACAGGTTCAGT GGCAGTGGGTCTGGGACAGACTTCACTCTCAC CATCAACAGACTGGAGCCTGAAGATTTTGCAC TGTATTACTGTCAGCAGTATGGTAACTCACCG TGGACGTTCGGCCAAGGGACCAAGGTGGAAA TCAAA | CAGGTGCAGCTGCAGGAGAAAGGCCCAGGACTG GTGAAGCCTTCACAGACCCTGTCCCTCACCTGCA CTGTCTCTGGTGGCTCCGTCAGCAGTGGTGTTTA CTACTGGAGCTGGATCCGCCAGCACCCAGGGAA GGGCCTGGAGTGGATTGGGAACATCTATTACAGT GGGAGCACCTACAACAACCCGTCCCTCAAGAGT CGAGTTACCATATCAGTAGACACGTCTAAGAACC AGTTCTCCCTGAAGCTGAACTCTGTGACTGCCGC GGACACGGCCGTCTATTACTGTGCGCGAGGGGAT TACGATGGTTCGGGGAGTTATCACTTCTACTACG GTTTGGACGTCTGGGGCCATGGGACCACGGTCAC CGTCTCCTCA |
| | | | SEQ ID NO: 26797 | SEQ ID NO: 30803 |
| | | AA | EIVLTQSPGTLSLSPGERATLSCRASQSVRTNFLA WHQQKPGQAPRLLIYGASRRATGIPDRFSGSGSG TDFTLTINRLEPEDFALYYCQQYGNSPWTFGQG TKVEIK | QVQLQEKGPGLVKPSQTLSLTCTVSGGSVSSGVYY WSWIRQHPGKGLEWIGNIYYSGSTYNNPSLKSRVTI SVDTSKNQFSLKLNSVTAADTAVYYCARGDYDGS GSYHFYYGLDVWGHGTTVTVSS |
| | | | SEQ ID NO: 26798 | SEQ ID NO: 30804 |

FIGURE 50

| | | | | |
|---|---|---|---|---|
| iPS:436043 | 21-225_193G9 | NA | GAAATTGTACTGACTCAGTCTCCAGATTTTCAGTCTGTGACTCCAAAGGAGAAAGTCACCATCACCTGCCGGGCCAGTCAGAGCATTGGTAGGAGTTTACACTGGTACCAGCAGAGACCAGATCAGTCTCTAAAGCTCCTCATCAAGTATGCTTCCCAGTCATTCTCAGGGGTCCCCTCGAGGTTCAGTGGCAGTGGATCTGGGACAGATTTCACCCTCACCATCAATAGCCTGGAGGCTGAAGATGCTGCAACGTATTTCTGTCATCAGAGTAGTCGTTTACCGCTCACTTTCGGCGGCGGGACCAAGGTGGAGATCAAA | CAGGTGCAGCTGCAGGAGTCGGGCCCAGGACTGGTGAAGCCTTCACAGACCCTGTCCCTCACCTGCACTGTCTCTGGTGACTCCATCAATAATGGTGGATACTACTGGAGCTGGATCCGCCAGCACCCAGGGAAGGGCCTGGAGTGGATTGGGTACATCTTTTACAGCGGGAGCACCTACTACAACCCGTCCCTCAAGAGTCGAGTTACCATATCAGTTGACACGTCTAAGAATCAGTTCTCCCTGAAGCTGAACTCTGTGACTGCCGCGGACACGGCCGTGTATTTTTGTGCGAGAGCGGGATATAACTGGGACAACGGGTTTGACTACTGGGGCCAGGGAACCCTGGTCACCGTCTCCTCA |
| | | | SEQ ID NO: 26799 | SEQ ID NO: 30805 |
| | | AA | EIVLTQSPDFQSVTPKEKVTITCRASQSIGRSLHWYQQRPDQSLKLLIKYASQSFSGVPSRFSGSGSGTDFTLTINSLEAEDAATYFCHQSSRLPLTFGGGTKVEIK | QVQLQESGPGLVKPSQTLSLTCTVSGDSINNGGYYWSWIRQHPGKGLEWIGYIFYSGSTYYNPSLKSRVTISVDTSKNQFSLKLNSVTAADTAVYFCARAGYNWDNGFDYWGQGTLVTVSS |
| | | | SEQ ID NO: 26800 | SEQ ID NO: 30806 |
| iPS:436045 | 21-225_193A10 | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATCTGGAACAGATTTCACTCTCACCATCAGCAGCCTGCAGCCTGAAGATTTTGCAACTTATTTCTGCCAACATTATCTTACTTACCCTCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATGAACACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATAGGGGGGTGGGTTACTACGGTTTGGACGTCTGGGGCCAAGGGACCACGGTCACCGTCTCCTCA |
| | | | SEQ ID NO: 26801 | SEQ ID NO: 30807 |

FIGURE 50

| | | | | |
|---|---|---|---|---|
| | | AA | DIQMTQSPSSLSASVGDRVTITCRASQGISNYLAWFQQKPGKAPKSLIYAASSLQSGVPSKFSGSGSGTDFTLTISSLQPEDFATYFCQHYLTYPLTFGGGTKVEIK | QVQLVESGGGVVQPGRSLRLSCAASGFTFSSYGMHWVRQAPGKGLEWVAVIWYDGSNEHYADSVKGRFTISRDNSKNTLYLQMNSLRAEDTAVYYCARDRGVGYYGLDVWGQGTTVTVSS |
| | | | SEQ ID NO: 26802 | SEQ ID NO: 30808 |
| **iPS:436047** | **21-225_193B10** | NA | GAAATTGTGTTGACGCAGTCTCCAGGCACCCTGTCTTTGTCTCCAGGGGAAAGAGCCACCCTCTCCTGCAGGGCCAGTCCGAGTGTTAGCAGCAGCTACTTAGCCTGGTACCAGCAAAAACCTGGCCAGGCTCCCAGGCTCGTCATCTATGGTGCATCCAGGAGGGCCACTGGCATCCCAGACAGGTTCAGAGGCAGTGGGTCTGGGACAGACTTCACTCTCACCATCAGTAGACTGGAGCCTGAGGATTTTGCAGTGTATTACTGTCAGCAGTATGGTAGCTCACCTCCGTGGACGTTCGGCCAAGGGACCAAGGTGGAAATCAAA | GAGGTGCAGTTGGTGGAGTCTGGGGGAGGCCTGGTCAAGCCTGGGGGGTCCCTGAGACTCTCCTGTGCAGCCTCTGGATTCACCTTCAGGGACTATAGCATGAACTGGGTCCGCCAGGCTCCAGGGAAGGGGCTGGAGTGGGTCTCATCCATTAGTAGTGCTGGTGGTTACATATACTACGCAGACTCACTGAAGGGCCGATTCACCATCTCCAGAGACAACGCCAAAAACTCACTGTATCTGCAAATGAACAGCCTGAGAGCCGAGGACACGGCTGTGTATTACTGTGCGAGAGCAACTATGGCCCTTGACTACTGGGGCCAGGGAACCCTGGTCACCGTCTCCTCA |
| | | | SEQ ID NO: 26803 | SEQ ID NO: 30809 |
| | | AA | EIVLTQSPGTLSLSPGERATLSCRASPSVSSSYLAWYQQKPGQAPRLVIYGASRRATGIPDRFRGSGSGTDFTLTISRLEPEDFAVYYCQQYGSSPPWTFGQGTKVEIK | EVQLVESGGGLVKPGGSLRLSCAASGFTFRDYSMNWVRQAPGKGLEWVSSISSAGGYIYYADSLKGRFTISRDNAKNSLYLQMNSLRAEDTAVYYCARATMALDYWGQGTLVTVSS |
| | | | SEQ ID NO: 26804 | SEQ ID NO: 30810 |

FIGURE 50

| | | | | |
|---|---|---|---|---|
| **iPS:436049** | **21-225_193B12** | NA | GAAATTGTGTTGACGCAGTCTCCAGGCACCCTGTCTTTGTCTCCAGGGGAAAGAGCCACCCTCTCCTGCAGGGCCAGTCAGAGTATTCGCAGCAGCTTCTTAGCCTGGTATCAGCAGAAACCTGGCCAGGCTCCCAGGCTCCTCATCTATGATGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGGTAACTCACCGTGGGCGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGCTGATTACTACTGGAACTGGATCCGCCAGCTCCCAGGGAAGGGCCTGGAGTGGATTGGGTACATCTTTTACAGTGGGAGCACCTACTACAACCCGTCCCTCAAGAGTCGACTTACCATATCAGTGGACACGTCTAAGAACCAGTTCTCCCTGAAGCTGAGCTCTGTGACTGCCGCGGACACGGCCGTGTATTACTGTGCGAGAGGGGATTACGATGGTTCGGGGAGTTATCACTTCTACTACGGTATGGACGTCTGGGGCCAAGGGACCACGGTCACCGTCTCCTCA |
| | | | SEQ ID NO: 26805 | SEQ ID NO: 30811 |
| | | AA | EIVLTQSPGTLSLSPGERATLSCRASQSIRSSFLAWYQQKPGQAPRLLIYDASSRATGIPDRFSGSGSGTDFTLTISRLEPEDFAVYYCQQYGNSPWAFGQGTKVEIK | QVQLQESGPGLVKPSQTLSLTCTVSGGSISSADYYWNWIRQLPGKGLEWIGYIFYSGSTYYNPSLKSRLTISVDTSKNQFSLKLSSVTAADTAVYYCARGDYDGSGSYHFYYGMDVWGQGTTVTVSS |
| | | | SEQ ID NO: 26806 | SEQ ID NO: 30812 |
| **iPS:436051** | **21-225_193G12** | NA | GAAATAGTGATGACGCAGTCTCCAGCCACCCTGTCTGTGTCTCCAGGGGAAAGAGCCACCCTCTCCTGCAGGGCCAGTCAGAGTGTTAGCAGCAGCTTAGCCTGGTATCAGCAGAAACCTGGCCAGGCTCCCAGGATCCTCATCTCTGGTGCATCCACCAGGGCCACTGGTATCCCAGCCAGGTTCAGTGGCAGTGGGTCTGGGACAGAGTTCACTCTCACCATCAGCAGCCTGCAGTCTGCAGATTTTGCAGTTTATTACTGCCAGCAGTATAATAACTGGCCGTGCAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTGGAGCGTCTGGATTCACCTTCAGTAGCTATGCCATGCACTGGGTCCGCCAGGCTCCAGGCAAGGGGCTGGAGTGGGTGGCAGTTATATGGTATGATGGAACTAATAAATACTATGGAGACTCCGTGAAGGGCCGATTCACCATCTCCAGAGACAATTCCAAGAACACGCTGAATCTGCAAATGAACAGCCTGAGAGCCGAGGACACGGCTGTCTATTACTGTGCGAGAGATTTCACTATAACTGGAGCTACATATTTTGACTACTGGGGCCAGGGAACCCTGGTCACCGTCTCCTCA |
| | | | SEQ ID NO: 26807 | SEQ ID NO: 30813 |

FIGURE 50

| | | | | |
|---|---|---|---|---|
| | | AA | EIVMTQSPATLSVSPGERATLSCRASQSVSSSLAWYQQKPGQAPRILISGASTRATGIPARFSGSGSGTEFTLTISSLQSADFAVYYCQQYNNWPCSFGQGTKLEIK | QVQLVESGGGVVQPGRSLRLSCGASGFTFSSYAMHWVRQAPGKGLEWVAVIWYDGTNKYYGDSVKGRFTISRDNSKNTLNLQMNSLRAEDTAVYYCARDFTITGATYFDYWGQGTLVTVSS |
| | | | SEQ ID NO: 26808 | SEQ ID NO: 30814 |
| iPS:436054 | 21-225_194C1 | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATCTGGAACAGATTTCACTCTCACCATCAGCAGCCTGCAGCCTGAAGATTTTGCAACTTATTACTGCCTACATTATCTTACTTACCCTCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATGAACACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAAATAGGGGGGTGGGTTACTACGGTTTGGACGTCTGGGGCCAAGGGACCACGGTCACCGTCTCCTCA |
| | | | SEQ ID NO: 26809 | SEQ ID NO: 30815 |
| | | AA | DIQMTQSPSSLSASVGDRVTITCRASQGISNYLAWFQQKPGKAPKSLIYAASSLQSGVPSKFSGSGSGTDFTLTISSLQPEDFATYYCLHYLTYPLTFGGGTKVEIK | QVQLVESGGGVVQPGRSLRLSCAASGFTFSSYGMHWVRQAPGKGLEWVAVIWYDGSNEHYADSVKGRFTISRDNSKNTLYLQMNSLRAEDTAVYYCARNRGVGYYGLDVWGQGTTVTVSS |
| | | | SEQ ID NO: 26810 | SEQ ID NO: 30816 |

FIGURE 50

| | | | | |
|---|---|---|---|---|
| **iPS:436056** | **21-225_194C3** | NA | GAAATTGTGCTGACTCAGTCTCCAGACTTTCAGTCTGTGGCTCCAAAGGAGAAAGTCACCATCACCTGCCGGGCCAGTCAGAGTATTGGTAGTAACTTACACTGGTACCAGCAGAAACCTGATCAGTCTCCAAAGCTCCTCATCAAGTCTGCTTCCCAGTCCTTCTCAGGGGTCCCCTCGAGGTTCAGTGGCAGTGGATCTGGGACAGATTTCACCCTCACCATCAATAGCCTGGAAACTGAAGATGCTGCAACGTATTACTGTCAGCAGAGTAGTAGTTTACCGTGGACGTTCGGCCAAGGGACCAAGGTGGAAATCAAA | CAGGTGCAGCTGCAGGAGTCGGGCCCAGGACTGGTGAAGCCTTCGGAGACCCTGTCCCTCACCTGCACTGTCTCTGGTGGCTCCATCAATAATTACTACTGGAGCTGGATCCGGCAGCCCGCCGGGAAGGGACTGGAGTGGATTGGGCGTATCTATGCCAGTGGGAGCACCAACTACAACCCCTCCCTCAAGAGTCGAGTCACCATGTCAATAGACACGTCCAAGAACCAGTTCTCCCTGAAGCTGAGCTCTGTGACCGCCGCGGACACGGCCGTGCATTACTGTGCGAGAGATCGGGGATACTATGGCTACTACGGTATGGACGTCTGGGGCCAAGGGACCACGGTCACCATCTCCTCA |
| | | | SEQ ID NO: 26811 | SEQ ID NO: 30817 |
| | | AA | EIVLTQSPDFQSVAPKEKVTITCRASQSIGSNLHWYQQKPDQSPKLLIKSASQSFSGVPSRFSGSGSGTDFTLTINSLETEDAATYYCQQSSSLPWTFGQGTKVEIK | QVQLQESGPGLVKPSETLSLTCTVSGGSINNYYWSWIRQPAGKGLEWIGRIYASGSTNYNPSLKSRVTMSIDTSKNQFSLKLSSVTAADTAVHYCARDRGYYGYYGMDVWGQGTTVTISS |
| | | | SEQ ID NO: 26812 | SEQ ID NO: 30818 |
| **iPS:436058** | **21-225_194A4** | NA | GAAATTGTGTTGACGCAGTCTCCAGGCACCCTGTCTTTGTCTCCAGGGGAAAGAGCCACCCTCTCCTGCAGGGCCAGTCGGGGTGTTAGCAACATCTACTTAGCCTGGTACCAACAGAAACCTGGCCAGGCTCCCAGGCTCCTCATCTATGGTGCTTCCAACAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCACAATGATTACTCAATGTTCACTTTCGGCCCTGGGACCAAAGTGGATATCAAA | CAGGTGCAGTTGGTGCAATCTGGGACTGAGGTGAAGAAGCCTGGGGCCTCTTTGAAGGTCTCCTGCAAGGCTTCTGGATACACCTTCACCGTCTACTATTTGAACTGGGTGCGACAGGCCCCTGGACAAGGGCTTGAGTGGATGGGATGGATCAACCCTAACAGTGGTGGCACAAACTATGCACAGAAGTTTCAGGGCAGGGTCACCATGACCAGGGACACGTCCATCAGCACAGCCTACATGGAACTGAGCAGGCTGAGATCTGACGACACGGCCGTGTATTACTGTGCGAGAGGCTACGATATTTTGACTGGTTGGGGCCAGGGAACCCTGGTCACCGTCTCCTCA |
| | | | SEQ ID NO: 26813 | SEQ ID NO: 30819 |

FIGURE 50

| | | | | |
|---|---|---|---|---|
| | | AA | EIVLTQSPGTLSLSPGERATLSCRASRGVSNIYLA WYQQKPGQAPRLLIYGASNRATGIPDRFSGSGS GTDFTLTISRLEPEDFAVYYCQHNDYSMFTFGPG TKVDIK | QVQLVQSGTEVKKPGASLKVSCKASGYTFTVYYL NWVRQAPGQGLEWMGWINPNSGGTNYAQKFQGR VTMTRDTSISTAYMELSRLRSDDTAVYYCARGYDI LTGWGQGTLVTVSS |
| | | | SEQ ID NO: 26814 | SEQ ID NO: 30820 |
| iPS:436060 | 21-225_194F4 | NA | GATATTGTGATGACCCAGACTCCACTCTCTCT GTCCGTCACCCCTGGACAGCCGGCCTCCATCT CCTGCAAGTCTAGTCAGAGCCTCCTCCATAGT GATGGAAGGACCTATTTGTATTGGTACCTGCA GAAGCCAGGCCAGCCTCCACAGCTCCTGATCT GTGAGGTTTCCAACCGGTTCTCTGGAGTGCCA GATAGGTTCAGTGGCAGCGGGTCAGGGACAG ATTTCACACTGAAAATCAGCCGGGTGGAGGCT GGGGATGTTGGGGTTTATTACTGCATGCAAAG TATACAGCTTCCCTGGACGTTCGGCCAAGGGA CCAAGGTGGAAATCAAA | CAGCTGCAGCTGCAGGAGTCGGGCCCAGGACTG GTGAAGCCTTCGGAGACCCTGTCCCTCACCTGCA CTGTCTCTGGTGGCTCCATCAGTAGTTACCACTG GAGCTGGATCCGGCAGCCCGCCGGGAAGGGACT GGAGTGGATTGGACTTATCTATACCAGTAGGAGC ACCAACTACAACCCCTCCCTCAAGAGTCGAGTCA CCATGTCAGTAGACAGGTCCAAGAGCCAGTTCTC CCTGAAGCTGAGCTCTGTGACCGCCGCGGACACG GCCGTGTATTACTGTGCGAGACTCCGGTATAACT GGAACTTCCCTTACTTTGACTACTGGGGCCAGGG AACCCTGGTCACCGTCTCCTCA |
| | | | SEQ ID NO: 26815 | SEQ ID NO: 30821 |
| | | AA | DIVMTQTPLSLSVTPGQPASISCKSSQSLLHSDGR TYLYWYLQKPGQPPQLLICEVSNRFSGVPDRFSG SGSGTDFTLKISRVEAGDVGVYYCMQSIQLPWT FGQGTKVEIK | QLQLQESGPGLVKPSETLSLTCTVSGGSISSYHWSW IRQPAGKGLEWIGLIYTSRSTNYNPSLKSRVTMSVD RSKSQFSLKLSSVTAADTAVYYCARLRYNWNFPYF DYWGQGTLVTVSS |
| | | | SEQ ID NO: 26816 | SEQ ID NO: 30822 |

FIGURE 50

| | | | | |
|---|---|---|---|---|
| iPS:436062 | 21-225_194E5 | NA | GAAATTGTGTTGACGCAGTCTCCAGGCACCCTGTCTTTGTCTCCAGGGGAAAGCGCCACCCTCTCCTGCAGGGCCGGTCAAAGTATTAGAACCAACTTCTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAACAGACTGGAGCCTAAAGATTTTGCAGTGTATTACTGTCAGCAGTATGGTAGTTCACCGTGGACGTTCGGCCAAGGGACCAAGGTGGAAATCAAA | CAGGTGCAACTGCAGGAGTCGGGCCCAGGACTGGTGAAGCCTTCACAGACCCTGTCCCTCACCTGCACTGTCTCTGGTGGCTCCATCAACAGTGGTGATTACTACTGGAACTGGATCCGCCACCACCCAGGGAAGGGCCTGGAGTGGATTGGGTACATCTTTTACAGTGGGAGCACCTACTACAACCCGTCCCTCAAGAGTCGAGTTACCATATCAGTAGACACGTCTAAGAACCAGTTCTCCCTGAAGCTGAGCTCTGTGACTGCCGCGGACACGGCCGTATATTACTGTGCGAGAGGGGATTACGATGGTTCGGGGAGTTATCACTACTATTACGGTATGGACGTCTGGGGCCAAGGGACCACGGTCACCGTCTCCTCA |
| | | | SEQ ID NO: 26817 | SEQ ID NO: 30823 |
| | | AA | EIVLTQSPGTLSLSPGESATLSCRAGQSIRTNFLAWYQQKPGQAPRLLIYGASSRATGIPDRFSGSGSGTDFTLTINRLEPKDFAVYYCQQYGSSPWTFGQGTKVEIK | QVQLQESGPGLVKPSQTLSLTCTVSGGSINSGDYYWNWIRHHPGKGLEWIGYIFYSGSTYYNPSLKSRVTISVDTSKNQFSLKLSSVTAADTAVYYCARGDYDGSGSYHYYYGMDVWGQGTTVTVSS |
| | | | SEQ ID NO: 26818 | SEQ ID NO: 30824 |
| iPS:436064 | 21-225_194E6 | NA | GAAATTGTGTTGACGCAGTCTCCAGGCACCCTGTCTTTGTCTCCAGGGGAAAGAGCCACCCTCTCCTGCAGGGCCAGTCAGAGTATTAGAAGCAACTTCTTAGCCTGGTATCAGCAGAAACCTGGCCAGGCTCCCAGGCTCCTCATCTATGGTGCATCCAGCAGGGCCACTGGCATCCCAGACAGGTTCAGTGGCAGTGTGTCTGGGACAGACTTCACTCTCACCATCAACAGACTGGAGCCTGAAGATTTTGCAGTGTATTACTGTCAGCAGTATGGTAGCTCACCGTGGACGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGACTCCATCAACAGTGGTGATTACTACTGGAACTGGATCCGCCAGCACCCAGGGAAGGGCCTGGAGTGGATTGGATTCATCTTTTACAGTGGGAGCACCTACTACAACCCGTCCCTCAAGAGTCGAGTTACCATATCAATAGACACGTCTAAGAACCAGTTCTCCCTGAAGCTGAGCTCTGTGAATGTCGCGGACACGGCCGTGTATTACTGTGCGAGAGGGGATTACGATGGTTCGGGGAGTTATCACTACTATTACGGTATGGACGTCTGGGGCCAAGGGACCACGGTCACCGTCTCCTCA |
| | | | SEQ ID NO: 26819 | SEQ ID NO: 30825 |

FIGURE 50

| | | | | |
|---|---|---|---|---|
| | | AA | EIVLTQSPGTLSLSPGERATLSCRASQSIRSNFLA WYQQKPGQAPRLLIYGASSRATGIPDRFSGSVSG TDFTLTINRLEPEDFAVYYCQQYGSSPWTFGQGT KVEIK | QVQLQESGPGLVKPSQTLSLTCTVSGDSINSGDYY WNWIRQHPGKGLEWIGFIFYSGSTYYNPSLKSRVTI SIDTSKNQFSLKLSSVNVADTAVYYCARGDYDGSG SYHYYYGMDVWGQGTTVTVSS |
| | | | SEQ ID NO: 26820 | SEQ ID NO: 30826 |
| **iPS:436066** | **21-225_194B7** | NA | GACATCCAGATGACCCAGTCTCCATCCTCACT GTCTGCATCTGTAGGAGACAGAGTCACCATCA CTTGTCGGGCGAGTCAGGGCATTAGCAAATAT TTAGCCTGGTTTCAGCAGAAACCAGGGAAAGC CCCTAAGTCCCTGATCTATGGTGCATCCAGGT TGCAAAGTGGGGTCCCATCAAAGTTCAGCGGC AGTGGATCTGGGACAGATTTCACTCTCACCAT CAGCAGCCTGCAGCCTGAAGATTTTGCAACTT ATTACTGCCAACATTATCTTAATTACCCTCTCA CCTTCGGCCAAGGGACACGACTGGAGATTAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTATTGTGCGAGAGATCGGTC TAAGGGTTACGACGGTATGGACGTCTGGGGCCA AGGGACCACGGTCACCGTCTCCTCA |
| | | | SEQ ID NO: 26821 | SEQ ID NO: 30827 |
| | | AA | DIQMTQSPSSLSASVGDRVTITCRASQGISKYLA WFQQKPGKAPKSLIYGASRLQSGVPSKFSGSGSG TDFTLTISSLQPEDFATYYCQHYLNYPLTFGQGT RLEIK | QVQLVESGGGVVQPGRSLRLSCAASGFTFSSYGMH WVRQAPGKGLEWVAVIWYDGSNKNYADSVKGRF TISRDNSKNTLYLQMNSLRAEDTAVYYCARDRSKG YDGMDVWGQGTTVTVSS |
| | | | SEQ ID NO: 26822 | SEQ ID NO: 30828 |

FIGURE 50

| | | | | |
|---|---|---|---|---|
| **iPS:436068** | **21-225_194F7** | NA | GACATCCAGATGACCCAGTCTCCATCTTCCGTGTCTGCATCTGTAGGAGACAGAGTCACCATCACTTGTCGGGCGAGTCAGGGTATTAGCAGGTGGTTAGCCTGGTTTCAGCAGAAACCAGGGAAAGCCCCTAAGATCCTGATCTATGCTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGGCTAACAGTTTCCCGTGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGACTACTACATACACTGGGTGCGACAGGCCCCTGGACAAGGGCTTGAGTGGATGGGATGGATCAACCCTAACAATGGTGGCACAAACTATGCACAGAAATTTCAGGGCAGGGTCACCATGACCAGGGACACGTCCATCAGCACAGCCTACATGGAGCTGAGCAGGCTGAGATCTGACGACACGGCCGTGTATTACTGTGCGAGAGAACCCCTTGGTTACTATGGTTCGGGGAGTTATGGGGCCTACGGTATGGACGTCTGGGGCCAAGGGACCACGGTCACCGTCTCCTCA |
| | | | SEQ ID NO: 26823 | SEQ ID NO: 30829 |
| | | AA | DIQMTQSPSSVSASVGDRVTITCRASQGISRWLAWFQQKPGKAPKILIYAASSLQSGVPSRFSGSGSGTDFTLTISSLQPEDFATYYCQQANSFPWTFGQGTKVEIK | QVQLVQSGAEVKKPGASVKVSCKASGYTFTDYYIHWVRQAPGQGLEWMGWINPNNGGTNYAQKFQGRVTMTRDTSISTAYMELSRLRSDDTAVYYCAREPLGYYGSGSYGAYGMDVWGQGTTVTVSS |
| | | | SEQ ID NO: 26824 | SEQ ID NO: 30830 |
| **iPS:436072** | **21-225_194C10** | NA | GAAATTGTGTTGACGCAGTCTCCAGGCACCCTGTCTTTGTCTCCAGGGGAAAGAGCCACCCTCTCCTGCAGGGCCAGTCCGAGTGTTAACAGCGGCTACTTAGCCTGGTACCAGCAGAAGCCTGGCCAGACTCCCAGGCTCCTCATCTTTGGTGCATCCAGCAGGGCCACTGGCATCCCCGACAGGTTCAGTGCCAGTGGGTCTGGGGCAGACTTCACTCTCACCATCAGTAGACTGGAGCCTGAAGATTTTGCAGTGTATTTCTGTCAGCAGTATGAAAGCTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTCTGGTGGGTCCTTCAGATATTACTACTGGAGCTGGATCCGCCAGCCCCCCGGGAAGGGGCTGGAGTGGTTTGGGGAAATCAATCATAGTGGAAGCACCAACTACAACCCGTCCCTCAAGAGTCGAGTCACCATATCAATAGACACGTCCAAGAACCAGTTCTCCCTGAAGCTGAGGTCTGTGACCGCCGCGGACACGGCTGTGTATTACTGTGCGAGAGACTACGGTGCTTTTGATATCTGGGGCCAAGGGACAATGGTCACCGTCTCTTCA |
| | | | SEQ ID NO: 26825 | SEQ ID NO: 30831 |

FIGURE 50

| | | | | |
|---|---|---|---|---|
| | | AA | EIVLTQSPGTLSLSPGERATLSCRASPSVNSGYLAWYQQKPGQTPRLLIFGASSRATGIPDRFSASGSGADFTLTISRLEPEDFAVYFCQQYESSPWTFGQGTKVEIK | QVQLQQWGAGLLKPSETLSLTCAVSGGSFRYYYWSWIRQPPGKGLEWFGEINHSGSTNYNPSLKSRVTISIDTSKNQFSLKLRSVTAADTAVYYCARDYGAFDIWGQGTMVTVSS |
| | | | SEQ ID NO: 26826 | SEQ ID NO: 30832 |
| **iPS:436074** | **21-225_194F10** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCTGAAACCAGGGAAAGCCCCTAAGCGCCTGATCTATGCTGCATCCAGTTTACAAAGTGGGGTCCCATCAAGGTTCAGCGGCAGTGGATCTGGGCCAGAATTCACTCTCACAATCAGCAGCCTGCAGCCTGAAGATTTTGCAACTTATTACTGTCTACAGCATTATAGTTTCCCATTCACTTTCGGCCCTGGGACCAAAGTGGATATCAAA | CAGGTGCAGCTGGTGGAGTCGGGGGGAGGCGTGGTCCAGCCTGGGAGGTCCCTGAGACTCTCCTGTGCAGCGTCTGGATTCACCTTCAGTAGCTTTATCATGCACTGGGTCCGCCAGGCTCCAGGCAGGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGGAAATGAACAGCCTGAGAGTCGAGGACACGGCTGTGTATTACTGTGCGAGAGAGGAGTATAGCAGTGGCTGGTTCGGGTACGGTATGGACGTCTGGGGCCAAGGGACCACGGTCACCGTCTCCTCA |
| | | | SEQ ID NO: 26827 | SEQ ID NO: 30833 |
| | | AA | DIQMTQSPSSLSASVGDRVTITCRASQGIRNDLGWYQLKPGKAPKRLIYAASSLQSGVPSRFSGSGSGPEFTLTISSLQPEDFATYYCLQHYSFPFTFGPGTKVDIK | QVQLVESGGGVVQPGRSLRLSCAASGFTFSSFIMHWVRQAPGRGLEWVAVIWYDGSNKYYADSVKGRFTISRDNSKNTLYLEMNSLRVEDTAVYYCAREEYSSGWFGYGMDVWGQGTTVTVSS |
| | | | SEQ ID NO: 26828 | SEQ ID NO: 30834 |

FIGURE 50

| | | | | |
|---|---|---|---|---|
| **iPS:436076** | **21-225_194H11** | NA | GACATCCAGATGACCCAGTCTCCATCCTCACT<br>GTCTGCATCTGTAGGAGACAGAGTCACCATCA<br>CTTGTCGGGCGAGTCAGGGCATTAGCAATTAT<br>TTAGCCTGGTTTCAGCAGAAACCAGGGAAAGC<br>CCCTAAGTCCCTGATCTATGCTGCATCCAGTTT<br>ACAAAGTGGGGTCCCATCAAAGTTCAGCGGCA<br>GTGGATCTGGAACAGATTTCACTCTCACCATC<br>AGCAGCCTGCAGCCTGAAGATTTTGCAACTTA<br>TTACTGCCAACATTATCTTACTTACCCTCTCAC<br>TTTCGGCGGAGGGACCAAGGTGGAGATCAAA | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTAGCTATGGCAT<br>GCACTGGGTCCGCCAGGCTCCAGGCAAGGGGCT<br>GGAGTGGGTGGCAGTTATATGGTATGATGGAAGT<br>AATGAACACTATGCAGACTCCGTGAAGGGCCGA<br>TTCACCATCTCCAGAGACAATTCCAAGAACACGC<br>TGTCTCTGCAAATGAACAGCCTGAGAGCCGAGG<br>ACACGGCTGTGTATTACTGTGCGAGAGATAGGGG<br>GGTGGGTTATTACGGTTTGGACGTCTGGGGCCAA<br>GGGACCACGGTCACCGTCTCCTCA |
| | | | SEQ ID NO: 26829 | SEQ ID NO: 30835 |
| | | AA | DIQMTQSPSSLSASVGDRVTITCRASQGISNYLA<br>WFQQKPGKAPKSLIYAASSLQSGVPSKFSGSGSG<br>TDFTLTISSLQPEDFATYYCQHYLTYPLTFGGGT<br>KVEIK | QVQLVESGGGVVQPGRSLRLSCAASGFTFSSYGMH<br>WVRQAPGKGLEWVAVIWYDGSNEHYADSVKGRF<br>TISRDNSKNTLSLQMNSLRAEDTAVYYCARDRGVG<br>YYGLDVWGQGTTVTVSS |
| | | | SEQ ID NO: 26830 | SEQ ID NO: 30836 |
| **iPS:436078** | **21-225_194H12** | NA | GACATCCAGATGACCCAGTCTCCATCCTCACT<br>GTCTGCATCTGTGGGAGACAGAGTCACCATCA<br>CTCGTCGGGCGAGTCAGGGCATTGGCAAGTAT<br>TTAGCATGGTTTCAGCAGAAACCAGGGAAAGC<br>CCTTAAGTCACTGATATATGCTGCATCCAGTTT<br>GCAAAGTGGGGTCCCATCAAAATTCAGCGGCA<br>GTGGATCTGGGACAGATTTCACTCTCACCATC<br>AGCAGCCTGCAGCCTGACGATTTTGCAACTTA<br>TTACTGCCAACGATATGATACTTACCCATTCA<br>CTTTCGGCCCTGGGACCAAAGTGGATATCAAA | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGGAACTATGGCAT<br>GCACTGGGTCCGCCAGGCTCCAGGCAAGGGGCT<br>GGAGTGGGTGGCAGTTATATGGTTTGATGGAAGT<br>AATGACTACTATGCAGACTCCGTGAAGGGCCGAT<br>TCACCATCTCCAGAGACAATTCCAAGAACACGCT<br>GTCACTGCAAATGAACAGCCTGAGAGCCGAGGA<br>CACGGCTGTGTATTACTGTGCGAGAGATAGAAGC<br>GTCGGCTACGACGGTTTAGATGTCTGGGGCCAAG<br>GGACCACGGTCACCGTCTCCTCA |
| | | | SEQ ID NO: 26831 | SEQ ID NO: 30837 |

FIGURE 50

| | | | | |
|---|---|---|---|---|
| | | AA | DIQMTQSPSSLSASVGDRVTITRRASQGIGKYLAWFQQKPGKALKSLIYAASSLQSGVPSKFSGSGSGTDFTLTISSLQPDDFATYYCQRYDTYPFTFGPGTKVDIK | QVQLVESGGGVVQPGRSLRLSCAASGFTFRNYGMHWVRQAPGKGLEWVAVIWFDGSNDYYADSVKGRFTISRDNSKNTLSLQMNSLRAEDTAVYYCARDRSVGYDGLDVWGQGTTVTVSS |
| | | | SEQ ID NO: 26832 | SEQ ID NO: 30838 |
| iPS:436080 | 21-225_195B1 | NA | GAAATTGTGTTGACGCAGTCTCCAGGCACCCTGTCTTTGTCTTCAGGGGAAAGAGCCACCCTCTCCTGCAGGGCCAGTCCGAGTGTTAACAGTAACTACTTAGCCTGGTATCAGCAGAAACCTGGCCAGACTCCCAGGCTCCTCATCTATGGTGCATCCAACAGGGCCACTGGCGTCCCAGACAGGTTCAGTGCCAGTGGGTCTGGGACAGACTTCACTCTCACCATCAGAAGACTGGAGCCTGAAGATTTTGCAGTGTATTTCTGTCAGCAGTATGAAAGCTCACCGTGGACGTTCGGCCAAGGGACCAAGGTGGAAATCAAA | CAGGTGCAGTTACAGCAGTGGGGCGCAGGACTGTTGAAGCCTTCGGAGACCCTGTCCCTCACCTGCGCTGTCTCTGGTGGGTCCTTCAGATATTACTTCTGGAGCTGGATCCGCCAGTCCCCCGGGAAGGGGCTGGAGTGGTTTGGGGAAATCAATCATAGTGGACGCACCAACTACAACCCGTCCCTCAAGAGTCGAGTCACCATATCAGTAGACACGTCCAAGAACCAGTTCTCCCTGAAGCTGAGGTCTGTGACCGCCGCGGACACGGCTGTGTATTACTGTGCGAGAGACTACGGTGCTTTTGATATCTGGGGCCAAGGCACATTGGTCACCGTCTCTTCA |
| | | | SEQ ID NO: 26833 | SEQ ID NO: 30839 |
| | | AA | EIVLTQSPGTLSLSSGERATLSCRASPSVNSNYLAWYQQKPGQTPRLLIYGASNRATGVPDRFSASGSGTDFTLTIRRLEPEDFAVYFCQQYESSPWTFGQGTKVEIK | QVQLQQWGAGLLKPSETLSLTCAVSGGSFRYYFWSWIRQSPGKGLEWFGEINHSGRTNYNPSLKSRVTISVDTSKNQFSLKLRSVTAADTAVYYCARDYGAFDIWGQGTLVTVSS |
| | | | SEQ ID NO: 26834 | SEQ ID NO: 30840 |

FIGURE 50

| | | | | |
|---|---|---|---|---|
| **iPS:436082** | **21-225_195D9** | NA | GACATCCAGATGACCCAGTCTCCATCTTCCGTGTCTGCATCTGTAGGAGACAGAGTCACCATCACTTGTCGGGCGAGTCAGGGTATTAGCAGGTGGTTAGCCTGGTATCAGCAGAAACCAGGGAAAGCCCCTAAGCTCCTGATCTATGCTGCATCCAGTTTGCTAGGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACGGGCTAACAGTTTCCCGTGCAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTCACTATGTCATGCACTGGGTCCGCCAGGCTCCAGGCAAGGGGCTGGAGTGGGTGGCAGTTATTTGGTATGATGGAACTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATTGGTTCGGGGAGGGGAACTACTACGGTATGGACGTCTGGGGCCAAGGGACCACGGTCACCGTCTCCTCA |
| | | | SEQ ID NO: 26835 | SEQ ID NO: 30841 |
| | | AA | DIQMTQSPSSVSASVGDRVTITCRASQGISRWLAWYQQKPGKAPKLLIYAASSLLGGVPSRFSGSGSGTDFTLTISSLQPEDFATYYCQRANSFPCSFGQGTKLEIK | QVQLVESGGGVVQPGRSLRLSCAASGFTFSHYVMHWVRQAPGKGLEWVAVIWYDGTNKYYADSVKGRFTISRDNSKNTLYLQMNSLRAEDTAVYYCARDWFGEGNYYGMDVWGQGTTVTVSS |
| | | | SEQ ID NO: 26836 | SEQ ID NO: 30842 |
| **iPS:436084** | **21-225_195F2** | NA | GAAATTGTGCTGACTCAGTCTCCAGACTTTCAGTCTGTGACTCCAAAGGAGAAAGTCACCATCACCTGCCGGGCCAGTCAGAGCATTGGTAGTAGCTTACACTGGTACCAGCAGAAACCAGATCAGTCTCCAAAGCTCCTCATCAAGTATGCTTCCCAGTCCTTCTCAGGGGTCCCCTCGAGGTTCAGTGGCAGTGGATCTGGGACAGATTTCGCCCTCACCATCAGTAGCCTGGAAGCTGAAGATGCTGCAACGTATTACTGTCATCAGAGTAGAACTTTACCGCTCACTTTCGGCGGAGGGACCAAGGTGGAGATCAAA | CAGGTGCAGCTGCAGGAGTCGGGCCCAGGACTGGTGAAGCCTTCACAGACCCTGTCCCTCACCTGCACTGTCTCTGGTGACTCCATCAGCAGCGGTGGTTACTACTGGAGCTGGATCCGCCAGCACCCAGGGAAGGGCCTGGAGTGGATTGGGTACAGCTATTACAGTGGGAGCACCAACTATAACCCGTCCCTCAAGAGTCGAGTTACCATATCAGTAGACATGTCTAAGAACCAGTTCTCCCTGAAGCTGAGCTCTGTGACTGACGCGGACACGGCCGTGTATTATTGTGCGAGAGGGGGGTATAACTGGAACAACGGTTTGACTACTGGGGCCAGGGAGCCCTGGTCACCGTCTCCTCA |
| | | | SEQ ID NO: 26837 | SEQ ID NO: 30843 |

FIGURE 50

| | | | | |
|---|---|---|---|---|
| | | AA | EIVLTQSPDFQSVTPKEKVTITCRASQSIGSSLHWYQQKPDQSPKLLIKYASQSFSGVPSRFSGSGSGTDFALTISSLEAEDAATYYCHQSRTLPLTFGGGTKVEIK | QVQLQESGPGLVKPSQTLSLTCTVSGDSISSGGYYWSWIRQHPGKGLEWIGYSYYSGSTNYNPSLKSRVTISVDMSKNQFSLKLSSVTDADTAVYYCARGGYNWNNGFDYWGQGALVTVSS |
| | | | SEQ ID NO: 26838 | SEQ ID NO: 30844 |
| iPS:436086 | 21-225_191G10 | NA | GACATCCAGATGACCCAGTCTCCTTCCTCACTGTCTGCATCTGTAGGAGACAGAGTCACCATCACTTGTCGGACGAGTCAGGGCATTGGCAAGTATTTAGCCTGGTTTCAGCAGAAACCAGGGAAAGCCCCTAAGTCCCTGCTCTATAAAGTATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATATGACTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCAGGGCGTGGGCTACGACGGTTTGGACGTCTGGGGCCAAGGGACCACGGTCACCGTCTCCTCA |
| | | | SEQ ID NO: 26839 | SEQ ID NO: 30845 |
| | | AA | DIQMTQSPSSLSASVGDRVTITCRTSQGIGKYLAWFQQKPGKAPKSLLYKVSSLQSGVPSKFSGSGSGTDFTLTISSLQPEDFATYYCQQYMTYPLTFGGGTKVEIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAVYYCARDQGVGYDGLDVWGQGTTVTVSS |
| | | | SEQ ID NO: 26840 | SEQ ID NO: 30846 |

FIGURE 50

| | | | | |
|---|---|---|---|---|
| iPS:436088 | 21-225_195C8 | NA | GAAATTGTGTTGACGCAGTCTCCAGGCACCCTGTCTTTGTCTCCAGGGGAAAGAGCCACCCTCTCCTGCAGGGCCAGTCAGAGTATTCGCAGCAGCTTCTTAGCCTGGTACCAACAGAAACCTGGCCAGGCTCCCAGGCTCCTCATCTATGGTGCATTTAGTAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGGTAACTCACCGTGGGCGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGATTACTACTGGAACTGGATCCGCCAGCTCCCAGGGAAGGGCCTGGAGTGGATTGGGTACATCTTTTACAGTGGGAGCACCTACTACAACCCGTCCCTCAAGAGTCGACTTACCATATCAGTGGACACGTCTAAGAACCAGTTCTCCCTGAAGCTGAGCTCTGTGACTGCCGCGGACACGGCCGTGTATTACTGTGCGAGAGGGGATTACGATGGTTCGGGGAGTTATCACTTCTACTACGGTATGGACGTCTGGGGCCAAGGGACCACGGTCACCGTCTCCTCA |
| | | | SEQ ID NO: 26841 | SEQ ID NO: 30847 |
| | | AA | EIVLTQSPGTLSLSPGERATLSCRASQSIRSSFLAWYQQKPGQAPRLLIYGAFSRATGIPDRFSGSGSGTDFTLTISRLEPEDFAVYYCQQYGNSPWAFGQGTKVEIK | QVQLQESGPGLVKPSQTLSLTCTVSGGSISSGDYYWNWIRQLPGKGLEWIGYIFYSGSTYYNPSLKSRLTISVDTSKNQFSLKLSSVTAADTAVYYCARGDYDGSGSYHFYYGMDVWGQGTTVTVSS |
| | | | SEQ ID NO: 26842 | SEQ ID NO: 30848 |
| iPS:436090 | 21-225_195A9 | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATCTGGAACAGATTTCACTCTCACCATCAGCAGCCTGCAGCCTGAAGATTTTGCAACTTATTACTGCCAACATTATCTTACTTACCCTCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGCAGTGGGTGGCAGTTATATGGTATGATGGAAGTAATGAACACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATAGGGGGGTGGGTTACTACGGTTTGGACGTCTGGGGCCAAGGGACCACGGTCACCGTCTCCTCA |
| | | | SEQ ID NO: 26843 | SEQ ID NO: 30849 |

FIGURE 50

| | | | | |
|---|---|---|---|---|
| | | AA | DIQMTQSPSSLSASVGDRVTITCRASQGISNYLAWFQQKPGKAPKSLIYAASSLQSGVPSKFSGSGSGTDFTLTISSLQPEDFATYYCQHYLTYPLTFGGGTKVEIK | QVQLVESGGGVVQPGRSLRLSCAASGFTFSSYGMHWVRQAPGKGLQWVAVIWYDGSNEHYADSVKGRFTISRDNSKNTLYLQMNSLRAEDTAVYYCARDRGVGYYGLDVWGQGTTVTVSS |
| | | | SEQ ID NO: 26844 | SEQ ID NO: 30850 |
| iPS:436092 | 21-225_195B9 | NA | GACATCCAGATGACCCAGTCTCCATCCTCCCTGTCTGCATCTGTAGGAGACAGAGTCACCATCACTTGCCGGGCAAGTCAGGGCATAAGAAAAGATTTAGGCTGGTATCAGCAGAAACCAGGGAAAGCCCCTAAGCGCCTGATCTATGCTGCATCCGATTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTGCTGTCTACAGCATTATCGTTACCCATTCACTTTCGGCCCTGGGACCAAAGTGGATTTCAAA | CAGGTGCAACTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAATGGCTACAATTCAGGTACTACTACGGTATGGACGTCTGGGGCCAAGGGACCACGGTCACCGTCTCCTCA |
| | | | SEQ ID NO: 26845 | SEQ ID NO: 30851 |
| | | AA | DIQMTQSPSSLSASVGDRVTITCRASQGIRKDLGWYQQKPGKAPKRLIYAASDLQSGVPSRFSGSGSGTEFTLTISSLQPEDFATYCCLQHYRYPFTFGPGTKVDFK | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCAREWLQFRYYYGMDVWGQGTTVTVSS |
| | | | SEQ ID NO: 26846 | SEQ ID NO: 30852 |

FIGURE 50

| | | | | |
|---|---|---|---|---|
| **iPS:436094** | **21-225_195B10** | NA | GAAATTGTGCTGACTCAGTCTCCAGACTTTCAGTCTGTGACTCCAAAGGAGAAAGTCACCATCACCTGCCGGGCCAGTCAGAGCATTGGTAGTAGCTTACACTGGTACCAGCAGAAACCAGATCAGTCTCCAAAGCTCCTCATCAAGTATGCTTCCCAGTCCTTCTCAGGGGTCCCCTCGAGGTTCAGTGGCAGTGGATCTGGGACAGATTTCACCCTCACCATCAATAGCCTGGAAGCTGAAGATGCTGCAACGTATTACTGTCATCAGAGTGGTCGTTTACCGCTCACTTTCGGCGGAGGGACCAAGGTGGAGATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAATAGTGGTTACTACTGGAGCTGGATCCGCCAGCACCCAGGGAAGGGCCTGGAATGGATTGGGTACATGTATTACAGTGGGAGCACCTACTACAACCCGTCCCTCAAGAGTCGGGTTACCATATCTGTAGACACGTCTAAGAACCAGTTTTATCTGAAGCTGAGCGCTGTGACTGCCGCGGACACGGCCGTGTATTACTGTGCGAAAGGGGGGTATAACTGGAACAATGGGTTTGACTGTTGGGGCCAGGGAACCCTGGTCACCGTCTCCTCA |
| | | | SEQ ID NO: 26847 | SEQ ID NO: 30853 |
| | | AA | EIVLTQSPDFQSVTPKEKVTITCRASQSIGSSLHWYQQKPDQSPKLLIKYASQSFSGVPSRFSGSGSGTDFTLTINSLEAEDAATYYCHQSGRLPLTFGGGTKVEIK | QVQLQESGPGLVKPSQTLSLTCTVSGGSISNSGYYWSWIRQHPGKGLEWIGYMYYSGSTYYNPSLKSRVTISVDTSKNQFYLKLSAVTAADTAVYYCAKGGYNWNNGFDCWGQGTLVTVSS |
| | | | SEQ ID NO: 26848 | SEQ ID NO: 30854 |
| **iPS:436096** | **21-225_195E10** | NA | GAAATTGTGCTGACTCAGTCTCCAGACTTTCAGTCTGTGACTCCAAAGGAGAAAGTCACCATCACCTGCCGGGCCAGTCAGAGCATTGGTAGTAGCTTACACTGGTACCAGCAGAAACCAGATCAGTCTCCAAAGCTCCTCATCAAGTATGCTTCCCAGTCCTTCTCAGGGGTCCCCTCGAGGTTCAGTGGCAGTGGATCTGGGACAGATTTCACCCTCACCATCAATAGCCTGGAAGCTGAAGATGCTGCAACGTATTACTGTCATCAGAGTAGTCGTTTACCATTCACTTTCGGCCCTGGGACCAAAGTGGATATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGGTTACTACTGGAGCTGGATCCGCCAGCACCCAGGGAAGGGCCTGGAGTGGATTGGGTACATCTATTACAGTGGGAGCACCTACTACAACCCGTCCCTCAAGAGTCGAGTTACCATATCAGTAGACACGTCTAAGAACCAGTTCTCCCTGAAGCTGAGCTCTGTGGCTGCCGCGGACACGGCCGTGTATTATTGTGCGAGAGGGGGGTATAACTGGAACCACGGTATGGACGTCTGGGGCCAAGGGACCACGGTCACCGTCTCCTCA |
| | | | SEQ ID NO: 26849 | SEQ ID NO: 30855 |

FIGURE 50

| | | | | |
|---|---|---|---|---|
| | | AA | EIVLTQSPDFQSVTPKEKVTITCRASQSIGSSLHWYQQKPDQSPKLLIKYASQSFSGVPSRFSGSGSGTDFTLTINSLEAEDAATYYCHQSSRLPFTFGPGTKVDIK | QVQLQESGPGLVKPSQTLSLTCTVSGGSISSGGYYWSWIRQHPGKGLEWIGYIYYSGSTYYNPSLKSRVTISVDTSKNQFSLKLSSVAAADTAVYYCARGGYNWNHGMDVWGQGTTVTVSS |
| | | | SEQ ID NO: 26850 | SEQ ID NO: 30856 |
| iPS:436098 | 21-225_195G11 | NA | GACATCGTGATGACCCAGTCTCCAGACTCCCTGGCTGTGTCTCTGGGCGAGAGGGCCACCATCAACTGCAAGTCCAGCCAGAGTGTTTTATTCAACTCCAACAATAAGAACTACTTAGCTTGGTACCAGCAGAAACCCGGTCAGCCTCCTAACCTGCTCATTTACTGGGCATCTACCCTGGAATCTGGGGTCCCTGACCGATTCAGTGGCAGCGGGTGTGGGACAGATTTCACTCTCACCATCAGCAGCATGCAGGCTGAAGATGTGGCAGTTTATTACTGTCAGCAATATTGTAGTTTTCCATTCACTTTCGGCCCTGGGACCAAAGTGGATATCAGA | CAGGTGCAACTGGTGGAGTCTGGGGGAGGCTTGGTCAAGCCTGGAGGGTCCCTGAGACTCTCCTGTGCAGCCTCTGGATTCACCTTCAGTGACTACTACATGAGCTGGATCCGCCAGGCTCCAGGGAAGGGGCTGGAGTGGCTTTCATACATTAGTAGTAGTGGTACTACCGTATTCTACGCAGACTCTGTGAAGGGCCGATTCACCATCTCCAGGGACAACGCCAAGAACTCACTGTATCTGCAAATGAACAGCCTGAGAGCCGAGGACACGGCCGTGTATTACTGTGCGAGAGAATGGGTGGGAGCCGACTACTGGGGCCAGGGAACCCTGGTCACCGTCTCCTCA |
| | | | SEQ ID NO: 26851 | SEQ ID NO: 30857 |
| | | AA | DIVMTQSPDSLAVSLGERATINCKSSQSVLFNSNNKNYLAWYQQKPGQPPNLLIYWASTLESGVPDRFSGSGCGTDFTLTISSMQAEDVAVYYCQQYCSFPFTFGPGTKVDIR | QVQLVESGGGLVKPGGSLRLSCAASGFTFSDYYMSWIRQAPGKGLEWLSYISSSGTTVFYADSVKGRFTISRDNAKNSLYLQMNSLRAEDTAVYYCAREWVGADYWGQGTLVTVSS |
| | | | SEQ ID NO: 26852 | SEQ ID NO: 30858 |

FIGURE 50

| | | | | |
|---|---|---|---|---|
| **iPS:436100** | **21-225_195G12** | NA | GACATCCAGATGACCCAGTCTCCATCTTCCGTGTCTGCATCTGTAGGAGACAGAGTCACCATCACTTGTCGGGCGAGTCAGGGTATTAACAACTGGTTAGCCTGGTATCAGCAGAAACCAGGGAAAGCCCCTAAGCTCCTGATCTATGGTGTTTCCAGTTTGCAGAGTGGGGTCCCATCAAGGTTCAGCGGCAGTGGATCTGGGACAGATTTCACTCTCACCATCAGCAGCCTGCAGCCTGAAGATTTTGCAACTTACTCTTGTCAACAGGCTAACAGTTTCCCGTGGACGTTCGGCCGAGGGACCAAGGTGGAAAATCAG | GAGGTGCAGCTGTTGGAGTCTGGGGGAGGCTTGGTACAGCCTGGGGGGTCCCTGAGACTCTCCTGTGCAGCCTCTGGATTCACCTTTAGCACCTATGCCATGAGTTGGGTCCGCCAGGCTCCAGGGAAGGGGCTGGAGTGGGTCTCAGCTATTAGTCGTAGTGGTGGTAACACACACTACGCAGACTCCGTGAAGGGCCGGTTCACCATCTCCAGAGACAATTCCAAGAATACGCTGTATCTGCAAATGAACAGCCTGAGAGCCGAGGACACGGCCGTATATTCCTGTGCGAAAGATGGATTCGGTGGGAGCTCCTACTTTGACTACTGGGGCCAGGGAACCCTGGTCACCGTCTCCTCA |
| | | | SEQ ID NO: 26853 | SEQ ID NO: 30859 |
| | | AA | DIQMTQSPSSVSASVGDRVTITCRASQGINNWLAWYQQKPGKAPKLLIYGVSSLQSGVPSRFSGSGSGTDFTLTISSLQPEDFATYSCQQANSFPWTFGRGTKVENQ | EVQLLESGGGLVQPGGSLRLSCAASGFTFSTYAMSWVRQAPGKGLEWVSAISRSGGNTHYADSVKGRFTISRDNSKNTLYLQMNSLRAEDTAVYSCAKDGFGGSSYFDYWGQGTLVTVSS |
| | | | SEQ ID NO: 26854 | SEQ ID NO: 30860 |
| **iPS:436102** | **21-225_196B1** | NA | GACATCGTGATGACCCAGTCTCCAGACTCCCTGGCTGTGTTTCTGGGCGAGAGGGCCACCATCAACTGCAAGTCCAGCCAGAGTATTTTATTCAGCTCCAACAATAAGAGGTACTTAGCTTGGTATCAGCAGAAACCAGGACAGCCTCCTAAGCTGCTCATTTACTGGGCATCTATCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTCTAGTCTTCCATTCACTTTCGGCCCTGGGACCAAAGTGGATATCAAA | CAGGTGCAGCTGGTGGAGTCTGGGGGAGGCTTGGTCAAGCCTGGAGGGTCCCTGAGACTCTCCTGTGCAGCCTCTGGATTCACCTTCAGTGACTACTACATGAGCTGGATCCGCCAGGCTCCAGGGAAGGGGCTGGAGTGGATTTCATACATTAGTAGTAGTGGTATTACCATGTACTACGCAGACTCTGTGAAGGGCCGATTCACCATCTCCAGGGACAACGCCAAGAACTCACTGTATCTGCAAATGAACAGCCTGAGAGCCGAGGACACGGCCGTGTATTACTGTGCGAGAGAATGGGTGGGAGCCGACTACTGGGGCCAGGGAACCCTGGTCACCGTCTCCTCA |
| | | | SEQ ID NO: 26855 | SEQ ID NO: 30861 |

FIGURE 50

| | | | | |
|---|---|---|---|---|
| | | AA | DIVMTQSPDSLAVFLGERATINCKSSQSILFSSNNKRYLAWYQQKPGQPPKLLIYWASIRESGVPDRFSGSGSGTDFTLTISSLQAEDVAVYYCQQYSSLPFTFGPGTKVDIK | QVQLVESGGGLVKPGGSLRLSCAASGFTFSDYYMSWIRQAPGKGLEWISYISSSGITMYYADSVKGRFTISRDNAKNSLYLQMNSLRAEDTAVYYCAREWVGADYWGQGTLVTVSS |
| | | | SEQ ID NO: 26856 | SEQ ID NO: 30862 |
| **iPS:436104** | **21-225_196C1** | NA | GACATCGTGATGACCCAGTCTCCAGACTCCCTGGCTGTGTCTCTGGGCGAGAGGGCCACCATCAACTGCAAGTCCAGCCAGAGTGTTTTATTCAACTCCAACAATAAGAACTACTTAGCTTGGTACCAGCAGAAACCCGGTCAGCTTCCTAACCTGCTCATTTACTGGGCATCTACCCTGGAATCTGGGGTCCCTGACCGATTCAGTGGCAGCGGCTGTGGGACAGATTTCACTCTCACCATCAGCAGCATGCAGGCTGAAGATGTGGCAGTTTATTACTGTCAGCAATATTGTAGTTTTCCATTCACTTTCGGCCCTGGGACCAAAGTGGATATCAGA | CAGGTGCAACTGGTGGAGTCTGGGGGAGGCTTGGTCAAGCCTGGAGGGTCCCTGAGACTCTCCTGTGCAGCCTCTGGATTCACCTTCAGTGACTACTACATGAGCTGGATCCGCCAGGCTCCAGGGAAGGGGCTGGAGTGGCTTTCATACATTAGTAGTAGTGGTACTACCGTATTCTACGCAGACTCTGTGAAGGGCCGATTCACCATCTCCAGGGACAACGCCAAGAACTCACTGTATCTGCAAATGAACAGCCTGAGAGCCGAGGACACGGCCGTGTATTACTGTGCGAGAGAATGGGTGGGAGCCGACTACTGGGGCCAGGGAACCCTGGTCACCGTCTCCTCA |
| | | | SEQ ID NO: 26857 | SEQ ID NO: 30863 |
| | | AA | DIVMTQSPDSLAVSLGERATINCKSSQSVLFNSNNKNYLAWYQQKPGQLPNLLIYWASTLESGVPDRFSGSGCGTDFTLTISSMQAEDVAVYYCQQYCSFPFTFGPGTKVDIR | QVQLVESGGGLVKPGGSLRLSCAASGFTFSDYYMSWIRQAPGKGLEWLSYISSSGTTVFYADSVKGRFTISRDNAKNSLYLQMNSLRAEDTAVYYCAREWVGADYWGQGTLVTVSS |
| | | | SEQ ID NO: 26858 | SEQ ID NO: 30864 |

FIGURE 50

| | | | | |
|---|---|---|---|---|
| **iPS:436106** | **21-225_196F2** | NA | GACATCCAGATGACCCAGTCTCCATCTTCCGTGTCTGCATCTGTAGGAGACAGAGTCACCATCACTTGTCGGGCGAGTCAGGGTATTAGCAGCTGGTTAGCCTGGTATCAGCAGAAACCAGGGAAAGCCCCTAAACTCCTGATCTATGCTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGACTAACAGTGTCCCATTCACTTTCGGCCCTGGGACCAAAGTAGATATCAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTTTGGCATGCACTGGGTCCGCCAGGCTCCAGGCAAGGGGCTGGAGTGGGTGGCAGTTATATTAAATGATGGAAGTAATAAAAAGTGTGCAGACTCCGTGAAGGGCCGATGCACCATTTCCAGAGACAACTCCAAGAACACGCTGTATCTGCAAATGAACAGCCTGAGAGCTGAGGACACGGCTGTTTATTACTGTGCGAGAGGACAGCAGTGGCTGGTAAACGGTGTGGACGTCTGGGGCCAGGGGACCACGGTCACCGTCTCCTCA |
| | | | SEQ ID NO: 26859 | SEQ ID NO: 30865 |
| | | AA | DIQMTQSPSSVSASVGDRVTITCRASQGISSWLAWYQQKPGKAPKLLIYAASSLQSGVPSRFSGSGSGTDFTLTISSLQPEDFATYYCQQTNSVPFTFGPGTKVDIK | QVQLVESGGGVVQPGRSLRLSCAASGFTFSSFGMHWVRQAPGKGLEWVAVILNDGSNKKCADSVKGRCTISRDNSKNTLYLQMNSLRAEDTAVYYCARGQQWLVNGVDVWGQGTTVTVSS |
| | | | SEQ ID NO: 26860 | SEQ ID NO: 30866 |
| **iPS:436110** | **21-225_196F4** | NA | GACTTTCAGATGATCCAGTCTCCATCCTCCCTGTCTGCATCTGTAGGCGACAGAGTCACCATCACTTGCCGGGCAAGTCAGCGCATTCACAGCTATTTAAATTGGTATCAGCAGAAACCAGGGAAAGCTCCTAAGCTCCTGATCTATACTGCATCCAGTTTGCAAGGTGGGGTCCCATCAAGGTTCAGTGGCAGTGGATCTGGGACAGATTTCACTCTCACCATCAGCAGTCTGCAGCCTGAAGATTTTGCAACTTACTACTGTCAACAGAGCTACGGTTCCCCTCTCACTTTCGGCGGAGGGACCAAGGTGGAGATCAAA | GAGGTGCAGCTGTTGGAGTCTGGGGGAGGCTTGGTACAGCCTGGGGGGTCCCTGAGATTCTCCTGTGCAGCCTCTGGATTCACCTTTAGCAGCTGTGCCATGACCTGGGTCCGCCAGGCTCCAGGGAAGGGGCTGGAGTGGGTCTCAGCTATTAGTGGTAGTGGTGGTAGCACATACTACGCAGACTCCGTGAAGGGCCGGTTCACCATTTCCAGAGACAATTCCAAGAACACGCTGTATCTGCAAATGAACAGCCTGAGAGCCGAGGACACGGCCGTATATTACTGTGCGAAAGTGGGGGGTTTGACTGGCTCCTACTACTACTACGGTATGGACGTCTGGGGCCAAGGGACCACGGTCACCGTCTCCTCA |
| | | | SEQ ID NO: 26861 | SEQ ID NO: 30867 |

FIGURE 50

| | | | | |
|---|---|---|---|---|
| | | AA | DFQMIQSPSSLSASVGDRVTITCRASQRIHSYLN WYQQKPGKAPKLLIYTASSLQGGVPSRFSGSGS GTDFTLTISSLQPEDFATYYCQQSYGSPLTFGGG TKVEIK | EVQLLESGGGLVQPGGSLRFSCAASGFTFSSCAMT WVRQAPGKGLEWVSAISGSGGSTYYADSVKGRFTI SRDNSKNTLYLQMNSLRAEDTAVYYCAKVGGLTG SYYYYGMDVWGQGTTVTVSS |
| | | | SEQ ID NO: 26862 | SEQ ID NO: 30868 |
| **iPS:436112** | **21-225_196C7** | NA | GACATCCAGATGACCCAGTCTCCATCCTCACT GTCTGCATCTGTAGGAGACAGAGTCACCATCA CTTGTCGGGCGAATCAGGCCATTAGCAATTAT TTAGCCTGGTTTCAGCAGAAACCAGGGAAAGC CCCTAAGTCCCTGATCTATGCTGCATCCAGTTT GCAAAGTGGGGTCCCATCAAAGTTCAGCGGCA GTGGATCTGGAACAGATTTCACTCTCACCATC AGCAGCCTGCAGCCTGAAGATTTTGCAACTTA TTACTGCCAACATTATCTCACTTACCCTCTCAC TTTCGGCGGAGGGACCAAGGTGGAGATCAAA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATGAACACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGATAGGGG GGTGGGTTACTACGGTTTGGACGTCTGGGGCCAA GGGACCACGGTCACCGTCTCCTCA |
| | | | SEQ ID NO: 26863 | SEQ ID NO: 30869 |
| | | AA | DIQMTQSPSSLSASVGDRVTITCRANQAISNYLA WFQQKPGKAPKSLIYAASSLQSGVPSKFSGSGSG TDFTLTISSLQPEDFATYYCQHYLTYPLTFGGGT KVEIK | QVQLVESGGGVVQPGRSLRLSCAASGFTFSSYGMH WVRQAPGKGLEWVAVIWYDGSNEHYADSVKGRF TISRDNSKNTLYLQMNSLRAEDTAVYYCARDRGV GYYGLDVWGQGTTVTVSS |
| | | | SEQ ID NO: 26864 | SEQ ID NO: 30870 |

FIGURE 50

| | | | | |
|---|---|---|---|---|
| iPS:436114 | 21-225_196G8 | NA | GACATCGTGATGACCCAGTCTCCAGACTCCCTGACTGTGTCTCTGGGCGAGAGGGCCACCGTCAACTGCAAGTCCAGCCAGAGTGTTTTACACAGCTCCAACAATAACAACTACTTAGCTTGGTACCAGCAGAAACCAGGACAGCCTCCTAAGCTGCTCATTTACTGGGCATCTACCCGGGAATCCGGAGTCCCTGACCGATTCAGTGGCAGCGGGTCTGGGACAGATTTCACTCTCGCCATCAGCAGCCTGCAGGCTGAAGATGTGGCGGTTTATTACTGTCAGCAATATTATAATACTCCTCCGACATTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGATGCGGCAGGCCACTGGTCAAGGGCTTGAGTGGATGGGATGGATGCACCTTAACAGTGGTAACACAGGCTATGCACCGAAGTTCCAGGGCAGAGTCACCATGACCAGGGACACCTCCATAAGCACAGCCTTCATGGAGCTGAGCAGCCTGAGATCTGAGGACACGGCCGTGTATTACTGTGCCTATAGCGGTGGCTGGTACGTGTTCGACCCCTGGGGCCAGGGAACCCTGGTCACCGTCTCCTCA |
| | | | SEQ ID NO: 26865 | SEQ ID NO: 30871 |
| | | AA | DIVMTQSPDSLTVSLGERATVNCKSSQSVLHSSNNNNYLAWYQQKPGQPPKLLIYWASTRESGVPDRFSGSGSGTDFTLAISSLQAEDVAVYYCQQYYNTPPTFGQGTKVEIK | QVQLVQSGAEVKKPGASVKVSCKASGYTFTNYDINWMRQATGQGLEWMGWMHLNSGNTGYAPKFQGRVTMTRDTSISTAFMELSSLRSEDTAVYYCAYSGGWYVFDPWGQGTLVTVSS |
| | | | SEQ ID NO: 26866 | SEQ ID NO: 30872 |
| iPS:436116 | 21-225_196B9 | NA | GACATCCAGATGACCCAGTCTCCATCTTCCGTGTCTGCATCTGTAGGAGACAGAGTCACCATCACTTGTCGGGCGAGTCAGGGTATTAGCAACTGCTTAGCCTGGTATCAGCAGAAACCAGGGAAAGCCCCTAAGTTCCTGATCTGTGCTGCATCCAGTTTGCAAAGTGCGGTCCCATCAAGGTTCAGCGGCAGTGGATCTGGGACAGATTTCACTCTCACCATCAGCAGCCTGCAGCCTGAAGATTTTGCATCTTACTATTGTCAACAGGGTGACAGTTTCCCTCCGACGTTCGGCCAAGGGACCAAGGTGGAATTCAGA | CAGGTGCAGCTGGTGCAGTCTGGGGCTGAGGTGAAGAAGCCTGGGGCCTCAATGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAACAGTGGTGGCACAAACTTTGCACAGAAGTTTCGGGGCAGGGTCACCATGACCAGGGACACGTCCATCAGCACAGCCTACATGGAGCTGAGCAGGCTGAGCTCTGACGACACGGCCGTGTATTACTGTGCGAGAGGGGGGGTTCGGGGAGTTCCCAACTACTACTACGTTATGGACGTCTGGGGCCAAGGGACCACGGTCACCGTCTCCTCA |
| | | | SEQ ID NO: 26867 | SEQ ID NO: 30873 |

FIGURE 50

| | | | | |
|---|---|---|---|---|
| | | AA | DIQMTQSPSSVSASVGDRVTITCRASQGISNCLAWYQQKPGKAPKFLICAASSLQSAVPSRFSGSGSGTDFTLTISSLQPEDFASYYCQQGDSFPPTFGQGTKVEFR | QVQLVQSGAEVKKPGASMKVSCKASGYTFTGYYMHWVRQAPGQGLEWMGWINPNSGGTNFAQKFRGRVTMTRDTSISTAYMELSRLSSDDTAVYYCARGGVRGVPNYYYVMDVWGQGTTVTVSS |
| | | | SEQ ID NO: 26868 | SEQ ID NO: 30874 |
| iPS:436118 | 21-225_196A10 | NA | GACATCCAGATGACCCAGTATCCATCTTACGTGTCTGCATCTGTAGGAGACAGAGTCAGCATCACTTGTCGGGCGAGTCAGGGTATTAGCAGGTGGTTAGCCTGGTATCAGCAGAAGCCAGGGAAAGCCGCCAAGTTCCTGATCTATGCTGCATCCAGTTTGCTAGGTGGGGTCTCATCAAGGTTCAGCGGCAGTGGATCTGGGACAGATTTCACTCTCACCATCAGCAGCCTGCAGCCTGAAGATTTTGCAATTTACTATTGTCAACGGGATAACAGTTTACCGTGCAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTCACTATGTCATGCACTGGGTCCGCCAGGCTCCAGGCAAGGGGCTGGAGTGGGTGGCAGTTATTTGGTATGATGGAACTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATTGGTTCGGGGAGGGGAACTACTACGGTATGGACGTCTGGGGCCAAGGGACCACGGTCACCGTCTCCTCA |
| | | | SEQ ID NO: 26869 | SEQ ID NO: 30875 |
| | | AA | DIQMTQYPSYVSASVGDRVSITCRASQGISRWLAWYQQKPGKAAKFLIYAASSLLGGVSSRFSGSGSGTDFTLTISSLQPEDFAIYYCQRDNSLPCSFGQGTKLEIK | QVQLVESGGGVVQPGRSLRLSCAASGFTFSHYVMHWVRQAPGKGLEWVAVIWYDGTNKYYADSVKGRFTISRDNSKNTLYLQMNSLRAEDTAVYYCARDWFGEGNYYGMDVWGQGTTVTVSS |
| | | | SEQ ID NO: 26870 | SEQ ID NO: 30876 |

FIGURE 50

| | | | | |
|---|---|---|---|---|
| **iPS:436120** | **21-225_196C10** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGTTCTGGGACAGAATTCACTCTCACAATCAGCAGCCTGCAGCCTGAAGATTTTGCAACTTATTACTGTCTACAATATAATAGTTACCCTCTCACTTTCGGCGGAGGGACCAAGGTGGAGATCAAA<br>SEQ ID NO: 26871 | CAGGTGCAGCTGCAGGAGTCGGGCCCAGGACTGGTGAAGCCTTCACAGACCCTGTCCCTCACCTGCACTGTCTCTGGTGGCTCCATGAGGAGTGGTGGTGACTACTGGAGCTGGATCCGCCAGCACCCAGGGAAGGGCCTGGAGTGGTTTGGGTTCATCTATTACAGTGGGAGCACCTACTACAATCCGTCCCTCAAGAGTCGAGTTACCTTATCAGTAGACACGTCTAAGAACCAGTTCTCCCTGAAGCTGAGCTCTGTGACTGCCGCGGACACGGCCGTGTATTACTGTGCGAGAATGGACTACAGTAACTACTACTACGGTATGGACGTCTGGGGCCAAGGGACCACGGTCACCGTCTCCTCA<br>SEQ ID NO: 30877 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQYNSYPLTFGGGTKVEIK<br>SEQ ID NO: 26872 | QVQLQESGPGLVKPSQTLSLTCTVSGGSMRSGGDYWSWIRQHPGKGLEWFGFIYYSGSTYYNPSLKSRVTLSVDTSKNQFSLKLSSVTAADTAVYYCARMDYSNYYYGMDVWGQGTTVTVSS<br>SEQ ID NO: 30878 |
| **iPS:436122** | **21-225_196G10** | NA | GAAATTGTGTTGACGCAGTCTCCAGGCACCCTGTTATTGTCTCCCGGGGAAAGAGCCACCCTCTCCTGCAGGGCCAGTCCGAGTGTTAGCAACAGCTTCTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCAACAGCAGACTGGAGCCTGAAGATTTTGCAGTGTATTACTGTCAGCAGTATGGTAGCTCACCTCCGTGGACGTTCGGCCAAGGGACCAAGGTAGAACTCAAA<br>SEQ ID NO: 26873 | GAGGTGCAGCTGGTGGAGTCTGGGGGAGGCCTGGTCAAGCCTGGGGGGTCCCTGAGACTCTCCTGTGCAGCCTCTGGATTCACCTTCAGGGACTATAGCATGAACTGGGTCCGCCAGGCTCCAGGGAAGGGGCTGGAGTGGGTCTCATCTATTAGTAGTGGTAGTGGTTACATACACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGCGAGCAACTATGGCCCTTGACTACTGGGGCCAGGGAACCCTGGTCACCGTCTCCTCA<br>SEQ ID NO: 30879 |

FIGURE 50

| | | | | |
|---|---|---|---|---|
| | | AA | EIVLTQSPGTLLLSPGERATLSCRASPSVSNSFLAWYQQKPGQAPRLLIYGASSRATGIPDRFSGSGSGTDFTLTNSRLEPEDFAVYYCQQYGSSPPWTFGQGTKVELK | EVQLVESGGGLVKPGGSLRLSCAASGFTFRDYSMNWVRQAPGKGLEWVSSISSGSGYIHYADSVKGRFTISRDNAKNSLYLQMNSLRAEDTAVYYCARATMALDYWGQGTLVTVSS |
| | | | SEQ ID NO: 26874 | SEQ ID NO: 30880 |
| iPS:436132 | 21-225_196C12 | NA | GACATCCAGATGACCCTGTCTCCATCGTCCCTGTTTGCATGTGTAGGAGACAGAGTCATCATCACTTGCCGGGCAAGTCAGGGCATTAGAAATGATCTAGGCTGGTCTCAGCAGAATCCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTATCACAATCAGCAGCCTGCAGCCTGAAGATTTTGAAACTTATTACTGTCTACAGCATAATGATTTCCCGTTCACTTTCGGCCGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCATCAGTTATGATATCAACTGGGTGCGACAGGCCACTGGACAAGGGCTTGAGTGGATGGGATGGATGAACCCTAAAAGGGGTAACACAGGCTATGCACAGAAGTTCCAGGGCAGAGTCACCATGACCAGGGACACCTCCATAAGCACAGCCTACATGGAGCTGAGCAGCCTGAGATCTGAGGACACGGCCGTTTATTACTGTGCGAGAGGAGACCCGTATAACTGGAACTCCTACGCTATGGACGTCTGGGGCCAAGGGGCCACGGTCACCGTCTCCTCA |
| | | | SEQ ID NO: 26875 | SEQ ID NO: 30881 |
| | | AA | DIQMTLSPSSLFACVGDRVIITCRASQGIRNDLGWSQQNPGKAPKRLIYAASSLQSGVPSRFSGSGSGTEFTITISSLQPEDFETYYCLQHNDFPFTFGRGTKVEIK | QVQLVQSGAEVKKPGASVKVSCKASGYTFISYDINWVRQATGQGLEWMGWMNPKRGNTGYAQKFQGRVTMTRDTSISTAYMELSSLRSEDTAVYYCARGDPYNWNSYAMDVWGQGATVTVSS |
| | | | SEQ ID NO: 26876 | SEQ ID NO: 30882 |

FIGURE 50

| | | | | |
|---|---|---|---|---|
| **iPS:436134** | **21-225_196H12** | NA | GAAATTGTGTTGACGCAGTCTCCAGGCACCCTGTTTTTGTCTCCAGGGGAAAGAGCCACCCTCTCCTGCAGGGCCAGTCAGAGTGTTAGAAGCAACTTCTTAGCCTGGCACCAGCAGAAACCTGGCCAGGCTCCCAGGCTCCTCATCTATGGTGCATACCGCAGGGCCACTGGCATCCCAGACAGGTTCAGTGGCAGTGGGTCTGGGACAGACTTCACTCTCACCATCAACAGACTGGAGCCTGAAGATTTTGCAGTGTATTACTGTCAGCAGTATGGTAACTCACCGTGGACGTTCGGCCAAGGGACCAAGGTGGAAATCAAA | CAGGTGCAGCTGCAGGAGTCGGGCCCAGGACTGGTGAACCCTTCACAGACCCTGTCCCTCACCTGCACTGTCTCTGGTGGCTCCATCAGCAGTGGTGTTTACTACTGGAGCTGGATCCGCCAGCACCCAGGGAAGGGCCTGGAGTGGATTGGGAACATCTATTACAGTGGGAGCACCTACAACAACCCGTCCCTCAAGAGTCGAATTATCATATCAGTAGACACGTCTAAGAACCAGTTCTCCCTGAAGCTGAGCTCTGTGACTGCCGCGGACACGGCCGTGTATTACTGTGCGAGAGGGGATTACGATGGTTCGGGGAGTTATCACTACTACTACGGTTTGGACGTCTGGGGCCAAGGGACCACGGTCACCGTCTCCTCA |
| | | | SEQ ID NO: 26877 | SEQ ID NO: 30883 |
| | | AA | EIVLTQSPGTLFLSPGERATLSCRASQSVRSNFLAWHQQKPGQAPRLLIYGAYRRATGIPDRFSGSGSGTDFTLTINRLEPEDFAVYYCQQYGNSPWTFGQGTKVEIK | QVQLQESGPGLVNPSQTLSLTCTVSGGSISSGVYYWSWIRQHPGKGLEWIGNIYYSGSTYNNPSLKSRIIISVDTSKNQFSLKLSSVTAADTAVYYCARGDYDGSGSYHYYYGLDVWGQGTTVTVSS |
| | | | SEQ ID NO: 26878 | SEQ ID NO: 30884 |
| **iPS:436138** | **21-225_197F2** | NA | GACATCCAGATGATCCAGTCTCCTTCCTCACTGTCTGCATCTGTAGGAGACAGAGTCACCATCACTTGTCGGACGAGTCAGGGCATTGGCAATTATTTAGCCTGGTTTCAGCAGAAACCAGGGAAAGCCCCTAAGTCCCTGCTCTATAAAACATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTTCCAACAATATATCACTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGACTGGAGTGGGTGGCAGTTATATGGTATGATGGAAGTAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCAGGGCGTGGGCTACGACGGTTTGGACGTCTGGGGCCAAGGGACCACGGTCACCGTCTCCTCA |
| | | | SEQ ID NO: 26879 | SEQ ID NO: 30885 |

FIGURE 50

| | | | | |
|---|---|---|---|---|
| | | AA | DIQMIQSPSSLSASVGDRVTITCRTSQGIGNYLA<br>WFQQKPGKAPKSLLYKTSSLQSGVPSKFSGSGS<br>GTDFTLTISSLQPEDFATYYFQQYITYPLTFGGGT<br>KVEIK | QVQLVESGGGVVQPGRSLRLSCAASGFTFSSYGMH<br>WVRQAPGKGLEWVAVIWYDGSNKNYADSVKGRF<br>TISRDNSKNTLYLQMNSLRAEDTAVYYCARDQGV<br>GYDGLDVWGQGTTVTVSS |
| | | | SEQ ID NO: 26880 | SEQ ID NO: 30886 |
| **iPS:436140** | **21-225_197G3** | NA | GACATCCAGATGACCCAGTCTCCGTCCTCACT<br>GTCTGCATCTGTAGGAGACAGAGTCACCATCA<br>CTTGTCGGGCGAGTCAGGGCATTGGCAATCAT<br>TTAGCCTGGTTTCAGCAGAAACCAGGGAAAGC<br>CCCTAAGTCCCTGATCTATGCTGCATCCAGTTT<br>GCAAAGTGGGGTCCCATCAAAGTTCAGCGGCA<br>GTAGATCTGGGACAGATTTCTCTCTCACCATC<br>AGCAGCCTGCAGCCTGAAGATTTTGCAACTTA<br>TTACTGCCAACAGTATAGTAATTACCCGGTCA<br>CTTTCGGCCCTGGGACCAAAGTGGATATCAAG | CAGGTGCAACTGGTGGAGTCTGGGGGAGGCGTG<br>GTCCAGCCTGGGAGGTCCCTGAGACTCTCCTGTG<br>TAGCGTCTGGGTTCACCTTCAGTAGCCATGGCAT<br>GCACTGGGTCCGCCAGGCTCCAGGCAAGGGGCT<br>GGAGTGGGTGGCAGTTATATGGTATGATGGAAGT<br>AATAAAAACTATGCAGACTCCGTGAAGGGCCGA<br>TTCACCATCTCCAGAGACAATTCCAAGAACACGC<br>TGTATCTGCAAATGAACAGCCTGAGAGCCGAGG<br>ACACGGCTGTGTATTACTGTGCGAGAGATCCCTC<br>TGTAGGGTACGACGGTATGGACGTCTGGGGCCA<br>AGGGACCACGGTCACCGTCTCCTCA |
| | | | SEQ ID NO: 26881 | SEQ ID NO: 30887 |
| | | AA | DIQMTQSPSSLSASVGDRVTITCRASQGIGNHLA<br>WFQQKPGKAPKSLIYAASSLQSGVPSKFSGSRSG<br>TDFSLTISSLQPEDFATYYCQQYSNYPVTFGPGT<br>KVDIK | QVQLVESGGGVVQPGRSLRLSCVASGFTFSSHGMH<br>WVRQAPGKGLEWVAVIWYDGSNKNYADSVKGRF<br>TISRDNSKNTLYLQMNSLRAEDTAVYYCARDPSVG<br>YDGMDVWGQGTTVTVSS |
| | | | SEQ ID NO: 26882 | SEQ ID NO: 30888 |

FIGURE 50

| | | | | |
|---|---|---|---|---|
| **iPS:436146** | **21-225_197F4** | NA | GAAATTGTGTTGACGCAGTCTCCAGGCACCCTGTCTTTGTCTCCAGGGGAAGGAGCCACCCTCTCCTGCAGGGCCAGTCAGAGTATTCGCAGCAGCTTCTTAGCCTGGTACCTGCAGAAGCCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGGAAACTCACCGTGGGCGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGATTACTACTGGAACTGGATCCGCCAGCACCCAGGGAAGGGCCTGGAGTGGATTGGGTACATCTTTTACAGTGGGAGCACCTACTACAACCCGTCCCTCAAGAGTCGAATTACCATATCAGTAGACACGTCTAAGAACCAGTTCTCCCTGAAGCTGAGCTCTGTGACTGCCGCGGACACGGCCGTGTATTACTGTGCGAGAGGGGATTACGATGGTTCGGGGAGTTATCACTACTACTACGGTTTGGACGTCTGGGGCCAAGGGACCACGGTCACCGTCTCCTCA |
| | | | SEQ ID NO: 26883 | SEQ ID NO: 30889 |
| | | AA | EIVLTQSPGTLSLSPGEGATLSCRASQSIRSSFLAWYLQKPGQAPRLLIYGASSRATGIPDRFSGSGSGTDFTLTISRLEPEDFAVYYCQQYGNSPWAFGQGTKVEIK | QVQLQESGPGLVKPSQTLSLTCTVSGGSISSGDYYWNWIRQHPGKGLEWIGYIFYSGSTYYNPSLKSRITISVDTSKNQFSLKLSSVTAADTAVYYCARGDYDGSGSYHYYYGLDVWGQGTTVTVSS |
| | | | SEQ ID NO: 26884 | SEQ ID NO: 30890 |
| **iPS:436150** | **21-225_197H4** | NA | GACATCATGATGACCCAGTCTTCAGACTCCCTGACTGTGTCTCTGGGCGAGAGGGCCATCATCAGCTGCAGGTCCAGCCAGAGTGTTTTACACAGCTTCAACAATTACAACTACTTAGCTTGGTACCAGCAGAAAGCAGGACATCCTCCTAACCTGCTCATTTACTGGGCATCTACCCGGGAATCCGGGGTCCCTGACCGATTCAGTGGCAGCGGGTCTGGGACAGATTTCACTCTCCCCATCAGCAGCCTGCAGGCTGAAGATGTGGCAGTTTATTACTGTCAACAATATTATAGTACTCCTCCGACGTTCGGCCTAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCCCATAGCAGTGGCTGGTACTACTTTGACTACTGGGGCCAGGGAACCCTGGTCACCGTCTCCTCA |
| | | | SEQ ID NO: 26885 | SEQ ID NO: 30891 |

FIGURE 50

| | | | | |
|---|---|---|---|---|
| | | AA | DIMMTQSSDSLTVSLGERAIISCRSSQSVLHSFNNYNYLAWYQQKAGHPPNLLIYWASTRESGVPDRFSGSGSGTDFTLPISSLQAEDVAVYYCQQYYSTPPTFGLGTKVEIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAHSSGWYYFDYWGQGTLVTVSS |
| | | | SEQ ID NO: 26886 | SEQ ID NO: 30892 |
| iPS:436152 | 21-225_197B6 | NA | GACATCCAGATGACCCAGTCTCCATCCTCACTGTCTGCATCTGTAGGAGACAGAGTCACCATCACTTGTCGGGCGAGTCAGGGCATTGGCAAATATTTAGCCTGGTTTCAGCAGAAACCAGGGAAAGCCCCTAAGTCCCTGATCTATGGTGCATCCAGTTTGCAAAGTGGGGTCCCATCAAAGTTCAGCGGCAGTGGATCTGGGACAGATTTCACTCTCACCATCAGCAGCCTGCAGCCTGAAAATTTTGCAACTTATTACTGCCAACAATATAGTACTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTACGCAGACTCCGTGAAGGGCCGATTCACCATCTCCAGAGACAATTCCAAGAACATGTTGTATCTGCAAATGAACAGCCTGAGAGCCGAGGACACGGCTATGTATTACTGTGCGAGAGATCAGGGCGTGGGCTACGACGGTTTGGACGTCTGGGGCCAAGGGACCTCGGTCACCGTCTCCTCA |
| | | | SEQ ID NO: 26887 | SEQ ID NO: 30893 |
| | | AA | DIQMTQSPSSLSASVGDRVTITCRASQGIGKYLAWFQQKPGKAPKSLIYGASSLQSGVPSKFSGSGSGTDFTLTISSLQPENFATYYCQQYSTYPLTFGGGTKVEIK | QVQLVESGGGVVQPGRSLRLSCAASGFTFSSYGMHWVRQAPGKGLEWVAVIWYDGSNKNYADSVKGRFTISRDNSKNMLYLQMNSLRAEDTAMYYCARDQGVGYDGLDVWGQGTSVTVSS |
| | | | SEQ ID NO: 26888 | SEQ ID NO: 30894 |

FIGURE 50

| | | | | |
|---|---|---|---|---|
| iPS:436154 | 21-225_197C6 | NA | GACATCATGATGACCCAGTCTCCAGACTCCCT<br>GGCTGTGTCTCTGGGCGAGAGGGCCATCATCA<br>GCTGCAGGTCCAGCCAGAGTGTTTTACACAGC<br>TCCAACAATTACAACTACTTAGCTTGGTACCA<br>GCAGAAACCAGGACATCCTCCTAACCTGCTCA<br>TTTACTGGGCATCTACCCGGGAATCCGGGGTC<br>CCTGACCGATTCAGTGGCAGCGGGTCTGGGAC<br>AGATTTCACTCTCACCATCAGCAGCCTGCAGG<br>CTGAAGATGTGGCAGTTTATTACTGTCAACAA<br>TATTATAGTACTCCTCCGACGTTCGGCCTAGG<br>GACCAAGGTGGAAATCAAA | CAGGTGCAGCTGGTGCAGTCTGGGGCTGAGGTG<br>AAGAAGCCTGGGGCCTCAGTGAAGGTCTCCTGC<br>AAGGCTTCTGGATACACCTTCACCAATTATGATA<br>TCAACTGGGTGCGACAGGCCACTGGACAAGGGC<br>TTGAGTGGATGGGATGGATGCACCCTAACAGTGG<br>TAACACAGGCTATGCACAGAAGTTCCAGGGCAG<br>AGTCACCATGACCAGGAACACCTCCATAAGCAC<br>AGCCTACATGGAGCTGAGCAGCCTGAGATCTGA<br>GGACACGGCCGTGTATTACTGTGCCCATAGCAGT<br>GGCTGGTACTACTTTGACTACTGGGGCCAGGGAA<br>CCCTGGTCACCGTCTCCTCA |
| | | | SEQ ID NO: 26889 | SEQ ID NO: 30895 |
| | | AA | DIMMTQSPDSLAVSLGERAIISCRSSQSVLHSSNN<br>YNYLAWYQQKPGHPPNLLIYWASTRESGVPDRF<br>SGSGSGTDFTLTISSLQAEDVAVYYCQQYYSTPP<br>TFGLGTKVEIK | QVQLVQSGAEVKKPGASVKVSCKASGYTFTNYDIN<br>WVRQATGQGLEWMGWMHPNSGNTGYAQKFQGR<br>VTMTRNTSISTAYMELSSLRSEDTAVYYCAHSSGW<br>YYFDYWGQGTLVTVSS |
| | | | SEQ ID NO: 26890 | SEQ ID NO: 30896 |
| iPS:436156 | 21-225_197C8 | NA | GACATCGTGATGACCCCGTCTCCAGACTCCCT<br>GGCTGTGTCTCTGGGCGAGAGGGCCACCATCA<br>ACTGCAAGTCCAGCCAGAGTGTTTTACACAGT<br>TCCAACAATAAGAACTACTTAGCTTGGTACCA<br>GCAGAAACCAGGACAGCCTCCTAAGCTGCTCA<br>TTTACTGGGCATCTACCCGGGAATCCGGGGTC<br>CCTGACCGATTCAGTGGCAGCGGGTCTGGGAC<br>AGATTTCACTCTCAGCATCAGCAGCCTGCAGG<br>CTGAAGATGTGGCAGTTTATTACTGTCAGCAG<br>TCTTATACTATTCCATTCACTTTCGGCCCTGGG<br>ACCAAAGTGGATAACAAA | GAGGTGCAACTATTGGAGTCTGGGGGAGGCTTG<br>GTGCAGCCTGGGGGGTCCCTGAGACTCTCCTGTG<br>CAGCCTCTGGATTCACCTTTAGCAGCTCTGCCAT<br>GACCTGGGTCCGCCAGGCTCCAGGGAAGGGACT<br>GGAGTGGGTCTCAGCTATCATTGGTAATGGTGGT<br>AGAGCATACTACGCAGACTCCGTGAAGGGCCGG<br>TTCACCATCTCCAGAGACAATTCCAAGAACACGC<br>TGTATCTGCAAATGAACAGCCTGAGAACCGAGG<br>ACACGGCCGTATATTACTGTGCGAAAGATCGGG<br>GATATAGCAGGATAGCAGTGGCTGGTACCTTTGA<br>CTACTGGGGCCAGGGAACCCTGGTCACCGTCTCC<br>TCA |
| | | | SEQ ID NO: 26891 | SEQ ID NO: 30897 |

FIGURE 50

| | | | | |
|---|---|---|---|---|
| | | AA | DIVMTPSPDSLAVSLGERATINCKSSQSVLHSSNNKNYLAWYQQKPGQPPKLLIYWASTRESGVPDRFSGSGSGTDFTLSISSLQAEDVAVYYCQQSYTIPFTFGPGTKVDNK | EVQLLESGGGLVQPGGSLRLSCAASGFTFSSSAMTWVRQAPGKGLEWVSAIIGNGGRAYYADSVKGRFTISRDNSKNTLYLQMNSLRTEDTAVYYCAKDRGYSRIAVAGTFDYWGQGTLVTVSS |
| | | | SEQ ID NO: 26892 | SEQ ID NO: 30898 |
| **iPS:436158** | **21-225_197G8** | NA | GAGATTGTGATGACCCAGACTCCACTTTCTCTGTCCGTCATCCCTGGACAGCCGGCCTCCATCTCCTGCAAGTCTAGTCAGAACCTCCTGCATAGTGATGGAAAGACCTATTTGTATTGGTACCTGCAGAAGCCAGGCCAGCCTCCACAGGTCCTGATCTATGAAGTTTCCAACCGGTTCTCTGGGGTGCCAGATAGGTTCAGTGGCAGCGGGTCAGGGACAGATTTCACACTGAAAATTAGCCGGGTGGAGGCTGAGGATGTTGGGGTTTATTCCTGCATGCAAAGTATACAGCTTCCCTGGACGTTCGCCCAAGGGTCCAAGGTGGAAATCAAA | CAGGTGCATCTGCAGGAGTCGGGCCCAGGACTGGTGAAGCCTTCGGAGACCCTGTCCCTCACCTGCACTGTCTCTGGTGGCTCCATCAGTGCTTACTCCTGGAGCTGGATCCGGCAGCCCGCCGGGAAGGGACTGGAGTGGATTGGGCGTCTCTCTCCTGGTGGGAGCACCAACTTCAACCCCTCCCTCAAGAGTCGAGTCACCATGTCAGTAGACACGTCCAAGAACCAGTTCTCCCTGAGGCTGAGCTCTGTGACCGCCGCGGACACGGCCGTTTATTACTGTGCGAGACTCCGGTATAACTGGAACTTCCCTTACTTTGACTACTGGGGCCAGGGAGCCCTGGTCACCGTCTCCTCA |
| | | | SEQ ID NO: 26893 | SEQ ID NO: 30899 |
| | | AA | EIVMTQTPLSLSVIPGQPASISCKSSQNLLHSDGKTYLYWYLQKPGQPPQVLIYEVSNRFSGVPDRFSGSGSGTDFTLKISRVEAEDVGVYSCMQSIQLPWTFAQGSKVEIK | QVHLQESGPGLVKPSETLSLTCTVSGGSISAYSWSWIRQPAGKGLEWIGRLSPGGSTNFNPSLKSRVTMSVDTSKNQFSLRLSSVTAADTAVYYCARLRYNWNFPYFDYWGQGALVTVSS |
| | | | SEQ ID NO: 26894 | SEQ ID NO: 30900 |

FIGURE 50

| | | | | |
|---|---|---|---|---|
| **iPS:436160** | **21-225_197C9** | NA | GACATCCAGATGACCCAGTCTCCATCTTCCGTGTCTGCATCTGTAGGAGACAGAGTCACCATCACTTGTCGGGCGAGTCAGGGTATTAGCAACTGGTTAGCCTGGTTTCAGCAGAAACCAGGGAAAGCCCCTCAGCTCCTGATCTATGCTGCATCCAGTTTGCAAAGTGGGGTCCCATCAAGGTTCAGCGGCAGTGGATCTGGGACAGACTTCAGTCTCACCATCAGCAGCCTGCAGCCTGAAGATTTTGCAACTTACTATTGTCAACAGGCTAACAGTTTCCCGTGGACGTTCGGGCAAGGGACCAAGGTGGAAATCAAA | GAGGTGCAGCTGTTGGAGTCTGGGGGAGGCTTGGCACAGCCGGGGGGGTCCCTGAGACTCTCCTGTGCAGCCTCTGGATTCACCTTTAGGAGCTATGCCATGAGCTGGGTCCGCCAGGCTCCAGGGAAGGGGCTGGAGTGGGTCTCAGTTATTAGTGGTAGAGGTGGTAACACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAAGGCATAGCAGTGGCTGGCTCGCACTACTTTGACTACTGGGGCCAGGGAACCCTGGTCACCGTCTCCTCA |
| | | | SEQ ID NO: 26895 | SEQ ID NO: 30901 |
| | | AA | DIQMTQSPSSVSASVGDRVTITCRASQGISNWLAWFQQKPGKAPQLLIYAASSLQSGVPSRFSGSGSGTDFSLTISSLQPEDFATYYCQQANSFPWTFGQGTKVEIK | EVQLLESGGGLAQPGGSLRLSCAASGFTFRSYAMSWVRQAPGKGLEWVSVISGRGGNTYYADSVKGRFTISRDNSKNTLYLQMNSLRAEDTAVYYCAKGIAVAGSHYFDYWGQGTLVTVSS |
| | | | SEQ ID NO: 26896 | SEQ ID NO: 30902 |
| **iPS:436164** | **21-225_197G10** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGGTGCATCCAGTTTACAAAGTGGGGTCCCATCGAGGTTCAGCGGCAGTGGATCTGGGACAGAATTCACTCTCACAATCAGCAGCCTGCAGCCTGAAGATTTTGCAACTTATTACTGTCTACAGCATTATAGGTACCCATTCACTTTCGGCCCTGGAACCAAAGTGGATATCAAA | CAGGTGCAGCTGGTGGAGTCTGGGGGAGGCGTGGTCCAGCCTGGGAGGTCCCTGAGACTCTCCTGTGTAGCGTCTGGATTCACCTTCAGTAGCTATGGCATGCACTGGGTCCGCCAGGCTCCAGGCAAGGGGCTGGAGTGGGTGGCAGTTACATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAATGGCTACAATTTAGGTACTACTACGGTATAGACGTCTGGGGCCAAGGGACCACGGTCACCGTCTCCTCA |
| | | | SEQ ID NO: 26897 | SEQ ID NO: 30903 |

FIGURE 50

| | | | | |
|---|---|---|---|---|
| | | AA | DIQMTQSPSSLSASVGDRVTITCRASQGIRNDLG<br>WYQQKPGKAPKRLIYGASSLQSGVPSRFSGSGS<br>GTEFTLTISSLQPEDFATYYCLQHYRYPFTFGPGT<br>KVDIK | QVQLVESGGGVVQPGRSLRLSCVASGFTFSSYGMH<br>WVRQAPGKGLEWVAVTWYDGSNKYYADSVKGR<br>FTISRDNSKNTLYLQMNSLRAEDTAVYYCAREWLQ<br>FRYYYGIDVWGQGTTVTVSS |
| | | | SEQ ID NO: 26898 | SEQ ID NO: 30904 |
| **iPS:436167** | **21-225_197E11** | NA | GACATCCAGATGACCCAGTCTCCATCCTCGCT<br>GTCTGCATCTGTGGGAGACAGAGTCACCATCA<br>CTTGTCGGGCGAGTCAGGGCATTAACAAGTAT<br>TTATCCTGGTTTCAGCAGAAACCAGGGAAAGC<br>CCCTAAGTCATTGATCTATGCTGCATCCAGTGT<br>GCAAAGTGGGGTCCCATCAAAATTCAGCGGCA<br>GTGGATCTGGGACAGATTTCACTCTCACCATC<br>AGCCGCCTGCAGCCTGACGATTTTGCAACTTA<br>TTACGGCCAACGATATGACACTTACCCATTCA<br>CTTTCGGCCCTGGGACCAAAGTGGATATCAAA | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGGAACTATGGCAT<br>GCACTGGGTCCGCCAGGCTCCAGGCAAGGGGCT<br>GGAGTGGGTGGCAGTTATATGGTTTGATGGAAGT<br>AATGACTACTATGCAGACTCCGTGAAGGGCCGAT<br>TCACCATCTCCAGAGACAATTCCAAGAACACGCT<br>GTCACTGCAAATGAACAGCCTGAGAGCCGAGGA<br>CACGGCTGTGTATTACTGTGCGAGAGATAGAAGC<br>GTCGGCTACGACGGTTTAGATGTCTGGGGCCAAG<br>GGACCACGGTCACCGTCTCCTCA |
| | | | SEQ ID NO: 26899 | SEQ ID NO: 30905 |
| | | AA | DIQMTQSPSSLSASVGDRVTITCRASQGINKYLS<br>WFQQKPGKAPKSLIYAASSVQSGVPSKFSGSGSG<br>TDFTLTISRLQPDDFATYYGQRYDTYPFTFGPGT<br>KVDIK | QVQLVESGGGVVQPGRSLRLSCAASGFTFRNYGM<br>HWVRQAPGKGLEWVAVIWFDGSNDYYADSVKGR<br>FTISRDNSKNTLSLQMNSLRAEDTAVYYCARDRSV<br>GYDGLDVWGQGTTVTVSS |
| | | | SEQ ID NO: 26900 | SEQ ID NO: 30906 |

FIGURE 50

| | | | | |
|---|---|---|---|---|
| iPS:436173 | 21-225_197G12 | NA | GACATCCAGATGATCCAGTCTCCTTCCTCACTGTCTGCATCTGTAGGAGACAGAGTCACCATCACTTGTCGGACGAGTCAGGGCATTGGCAATTATTTAGCCTGGTTTCAGCAGAAACCAGGGAAAGCCCCTAAGTCCCTGCTCTATAAAACATCCAGTTTGCAAAGTGGGTTCCCATCAAAGTTCAGCGGCAGTGGATCTGGGACAGATTTCACTCTCACCATCAGCAGCCTGCAGCCTGAAGATTTTGCAACTTATTACTTCCAACAATATATGACTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGACTGGAGTGGGTGGCAGTTATATGGTATGATGGAAGTAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCAGGGCGTGGGCTACGACGGTTTGGACGTCTGGGGCCAAGGGACCACGGTCACCGTCTCCTCA |
| | | | SEQ ID NO: 26901 | SEQ ID NO: 30907 |
| | | AA | DIQMIQSPSSLSASVGDRVTITCRTSQGIGNYLAWFQQKPGKAPKSLLYKTSSLQSGFPSKFSGSGSGTDFTLTISSLQPEDFATYYFQQYMTYPLTFGGGTKVEIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAVYYCARDQGVGYDGLDVWGQGTTVTVSS |
| | | | SEQ ID NO: 26902 | SEQ ID NO: 30908 |
| iPS:436177 | 21-225_198B1 | NA | GAAATTGTGTTGACGCAGTCTCCAGGCACCCTGTCTTTGTCTCCAGGGGAAAGCGCCACCCTCTCCTGCAGGGCCGGTCAAAGTATTAGAACCAACTTCTTAGCCTGGTACCAGCAGCAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAACAGACTGGAGCCTGAAGATTTTGCAGTGTATTACTGTCAGCAGTATGGTAGCTCACCGTGGACGTTCGGCCAAGGGACCAAGGTGGAAATCAAA | CAGGTGCAACTGAAGGAGTCGGGCCCAGGACTGGTGAAGCCTTCACAGACCCTGTCCCTCACCTGCACTGTCTCTGGTGGCTCCATCAACAGTGGTGATTACTACTGGAACTGGTTCCGCCAGCACCCAGGGAAGGGCCTGGAGTGGATTGGGTACATCTTCCACAGTGGGAGCACCTACTACAACCCGTCCCTCAAGAGTCGAGTAACCGTATCAGTAGACACGTCTAAGAACCAGTTCTCCCTGAAGCTGAGTTCTGTGACTGCCGCGGACACGGCCGTGTATTACTGTGCGAGAGGGGATTACGATGGTTCGGGGAGTTATCACTACTATTACGGTATGGACGTCTGGGGCCGAGGGACCACGGTCACCGTCTCCTCA |
| | | | SEQ ID NO: 26903 | SEQ ID NO: 30909 |

FIGURE 50

| | | | | |
|---|---|---|---|---|
| | | AA | EIVLTQSPGTLSLSPGESATLSCRAGQSIRTNFLAWYQQQPGQAPRLLIYGASSRATGIPDRFSGSGSGTDFTLTINRLEPEDFAVYYCQQYGSSPWTFGQGTKVEIK | QVQLKESGPGLVKPSQTLSLTCTVSGGSINSGDYYWNWFRQHPGKGLEWIGYIFHSGSTYYNPSLKSRVTVSVDTSKNQFSLKLSSVTAADTAVYYCARGDYDGSGSYHYYYGMDVWGRGTTVTVSS |
| | | | SEQ ID NO: 26904 | SEQ ID NO: 30910 |
| **iPS:436179** | **21-225_198E1** | NA | GAAATTGTGTTGACGCAGTCTCCAGGCACCCTGTCTTTGTCTCCAGGGGAAAGAGCCACCCTCTCCTGCAGGGCCAGTCAGAGTATAAGGAGCAACTTCTTAGCCTGGTACCAGCAGAAACCTGGCCAGGCTCCCAGGCTCCTCATCTATGGTGCATTCAGTAGGGCCACTGGCATCCCAGACAGGTTCAGTGGCAGTGGGTCTGGGACAGACTTCACTCTCACCATCAACAGACTGGAGCCTGAAGATTTTGCAGTGTATTACTGTCAGCAGTATGGTAATTCACCGTGGACGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGGCTCCATCAACAGTGGTGGTTACTACTGGAACTGGATCCGCCAGCACCCAGGGAAGGGCCTGGAGTGGATTGGGTTCATCTTTTACAGTGGGAGCACCTACTACAACCCGTCCCTCAAGAGTCGACTTTCCATATCAGTAGACACGTCTAAGAACCAGTTCTCCCTGAAGCTGAGCTCTGTGACTGCCGCGGACACGGCCGTGTATTACTGTGCGAGAGGGGATTACGATGGTTCGGGGAGTTATCACTACTATTACGGTATGGACGTCTGGGGCCAAGGGACCACGGTCACCGTCTCCTCA |
| | | | SEQ ID NO: 26905 | SEQ ID NO: 30911 |
| | | AA | EIVLTQSPGTLSLSPGERATLSCRASQSIRSNFLAWYQQKPGQAPRLLIYGAFSRATGIPDRFSGSGSGTDFTLTINRLEPEDFAVYYCQQYGNSPWTFGQGTKVEIK | QVQLQESGPGLVKPSQTLSLTCTVSGGSINSGGYYWNWIRQHPGKGLEWIGFIFYSGSTYYNPSLKSRLSISVDTSKNQFSLKLSSVTAADTAVYYCARGDYDGSGSYHYYYGMDVWGQGTTVTVSS |
| | | | SEQ ID NO: 26906 | SEQ ID NO: 30912 |

FIGURE 50

| | | | | |
|---|---|---|---|---|
| iPS:436181 | 21-225_198C2 | NA | GAAATTGTGGTGACGCAGTCTCCAGGCACCCTGTTATTGTATTCCGAGGAGAGATCCACCCTCTCCTGCAGGGCCAGTCAGAGTGTTAGAAGCAGCTACTTAGCCTGGTACCAGCAGAAACCTGGCCAGGCTCCCAGGCTCCTCATCTGTGGTGCATTCAGCAGGGCCAGTGGCATCCCAGACAGGTTCAGTGGCAGTGGGTCTGGGACAGACTTCACTCTCACCATCAGCAGACTGGAGCCTGAAGATTTCGCAGTGTACTACTGTCAGCAGTATGGTAACTCACCGTGGACGTTGGGCCACGCGACCAAGGTGGAAATCAAA | CAGGTGCAGCTGCAGGAGTCGGGCCCAGGACTGGTGAAGCCCTCACAGACCCTATCCCTCACCTGCACTGTCTCTGGTGGCTCCATCAGCAGTGGTGGTTACTACTGGAGCTGGATCCGCCAGCACCCAGGGAAGGGCCTGGAGTGGATTGGGAACATCTATTACAGTGGGAGCACCTACTACAACCCGTCCCTCAAGAGTCGAGTTACCATATCAGTAGACACGTCTAAGAACCAGTTCTCCCTGAAGCTGAGCTCTGTGACTGCCGCGGACACGGCCGTGTATTACTGTGCGAGAGGGGATTACTATGGTTCGGGGAGTTATCACAACTACTACGGTTTGGACGTCTGGGGCCAAGGGACCACGGTCACCGTCTCCTCA |
| | | | SEQ ID NO: 26907 | SEQ ID NO: 30913 |
| | | AA | EIVVTQSPGTLLLYSEERSTLSCRASQSVRSSYLAWYQQKPGQAPRLLICGAFSRASGIPDRFSGSGSGTDFTLTISRLEPEDFAVYYCQQYGNSPWTLGHATKVEIK | QVQLQESGPGLVKPSQTLSLTCTVSGGSISSGGYYWSWIRQHPGKGLEWIGNIYYSGSTYYNPSLKSRVTISVDTSKNQFSLKLSSVTAADTAVYYCARGDYYGSGSYHNYYGLDVWGQGTTVTVSS |
| | | | SEQ ID NO: 26908 | SEQ ID NO: 30914 |
| iPS:436189 | 21-225_198B6 | NA | GACATCCAGATGACCCAGTCTCCATCCTCACTGTCTGCATCTGTAGGAGACAGAGTCACCATCACTTGTCGGGCGAGTCAGGGCATTGGCAATTATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAATAGATCTGGGACAGATTTCACTCTCACCATCAGCAGCCTGCAGCCTGAAGATTTTGCAACTTATTACTGCCAACAATATAGTACTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAACACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCAGGGCGTGGGCTACTACGGTATGGACGTCTGGGGCCAAGGGACCACGGTCACCGTCTCCTCA |
| | | | SEQ ID NO: 26909 | SEQ ID NO: 30915 |

FIGURE 50

| | | | | |
|---|---|---|---|---|
| | | AA | DIQMTQSPSSLSASVGDRVTITCRASQGIGNYLA WFQQKPGKAPKSLIYAASSLQSGVPSKFSGNRSG TDFTLTISSLQPEDFATYYCQQYSTYPLTFGGGT KVEIK | QVQLVESGGGVVQPGRSLRLSCAASGFTFSSYGMH WVRQAPGKGLEWVAVIWYDGSNKHYADSVKGRF TISRDNSKNTLYLQMNSLRAEDTAVYYCARDQGV GYYGMDVWGQGTTVTVSS |
| | | | SEQ ID NO: 26910 | SEQ ID NO: 30916 |
| **iPS:436191** | **21-225_198B9** | NA | GACATCCAGATGACCCAGTCTCCATCCTCCCT GTCTGCATCTGTAGGAGACAGAGTCACCATCA CTTGCCGGGCAAGTCAGGGCATAAGAAAAGA TTTAGGCTGGTATCAGCAGAAACCAGGGAAA GCCCCTAAGCGCCTGATCTATGCTGCATCCAG TTTGCAAAGTGGGGTCCCATCAAGGTTCAGCG GCAGTGGATCTGGGACAGAATTCACTCTCACA ATCAGCAGCCTGCAGCCTGAAGATTTTGCAAC TTATGTCTGTCTACAACATTATCGTTACCCTTT CACTTTCGGCCCTGGGACCAAAGTGGATTTCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGGCAT GCACTGGGTCCGCCAGGCTCCAGGCAAGGGGCT GGAGTGGGTGGCAATTATATGGTTTGATGGAAGT AATAAATACTATGTAGACTCCGTGAAGGGCCGAT TCACCATCTCCAGAGACAATTCCAAGAACACGCT GTTTCTGCAAATGAGCAGCCTGAGAGCCGAGGA CACGGCTGTGTATTATTGCGCGAGAGAATGGCTA CAATTCAGGTACTACTACGGTATGGACGTCTGGG GCCAAGGGACCACGGTCACCGTCTCCTCA |
| | | | SEQ ID NO: 26911 | SEQ ID NO: 30917 |
| | | AA | DIQMTQSPSSLSASVGDRVTITCRASQGIRKDLG WYQQKPGKAPKRLIYAASSLQSGVPSRFSGSGS GTEFTLTISSLQPEDFATYVCLQHYRYPFTFGPGT KVDFK | QVQLVESGGGVVQPGRSLRLSCAASGFTFSNYGM HWVRQAPGKGLEWVAIIWFDGSNKYYVDSVKGRF TISRDNSKNTLFLQMSSLRAEDTAVYYCAREWLQF RYYYGMDVWGQGTTVTVSS |
| | | | SEQ ID NO: 26912 | SEQ ID NO: 30918 |

FIGURE 50

| | | | | |
|---|---|---|---|---|
| **iPS:436193** | **21-225_198A10** | NA | GATATTGTGTTGACCCAGACTCCACTCTCTCTGTCCGTCACCCCTGGACAGCCGGCCTCCATATCGTGCAAGTCTAGTCAGAGCCTCCTCTATAGTGATGGAAGGACCTATTTGTATTGGTACCTGCAGAAACCAGGCCAGCCTCCACAGGTCCTGATCTGTGAGGTTTCCAACCGGTTCTCTGGAGTGCCAGATAGGTTCAGTGGCAGCGGGTCAGGGACAGATTTCACACTGAAAATCAGCCGGGTGGAGGCAGGAGATGTTGGGGTTTATTACTGCATGCAAAGTATACAGCTTCCCTGGACGTTCGGCCAAGGGACCAAGGTGGAAATCAAA | CAGCTGCAGCTGCAGGAGTCGGGCCCAGGACTGGTGAAGCCTTCGGAGACCCTGTCCCTCACCTGCACTGTCTCTGGTGGCTCCATCAGAAGTTACCACTGGAGTTGGATCCGGCAGCCCGCCGGGAAGGGACTGGAGTGGATTGGGCATATCTATACCAGTAGGAGCACCAACTACAACCCCTCCCTCAAGAGTCGAGTCACCATTTCAGTAGACACGTCCAAGAACCAGTTCTCCCTGAAGCTGAGCTCTGTGACCGCCACGGACACGGCCGTGTATTACTGTGCGAGACTCCGGTATAACTGGAACTTCCCTTACTTTGACTACTGGGGCCAGGGAACCCTGGTCACCGTCTCCTCA |
| | | | SEQ ID NO: 26913 | SEQ ID NO: 30919 |
| | | AA | DIVLTQTPLSLSVTPGQPASISCKSSQSLLYSDGRTYLYWYLQKPGQPPQVLICEVSNRFSGVPDRFSGSGSGTDFTLKISRVEAGDVGVYYCMQSIQLPWTFGQGTKVEIK | QLQLQESGPGLVKPSETLSLTCTVSGGSIRSYHWSWIRQPAGKGLEWIGHIYTSRSTNYNPSLKSRVTISVDTSKNQFSLKLSSVTATDTAVYYCARLRYNWNFPYFDYWGQGTLVTVSS |
| | | | SEQ ID NO: 26914 | SEQ ID NO: 30920 |
| **iPS:436195** | **21-225_198G10** | NA | GAAATTGTGTTGACGCAGTCTCCAGGCACCCTGTCTTTGTCTCCAGGGGAAAGAGCCACCCTCTCCTGCAGGGCCAGTCAGAGTATTCGCAGCAGCTTCTTAGCCTGGTATCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGGTAACTCACCGTGGGCGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGATTACTACTGGAACTGGATCCGCCAGCTCCCAGGGAAGGGCCTGGAGTGGATTGGGTACATCTTTTACAGTGGGAGCACCTACTACAACCCGTCCCTCAAGAGTCGACTTACCATATCAGTGGACACGTCTAAGAACCAGTTCTCCCTGAAGCTGAGTTCTGTGACTGCCGCGGACACGGCCGTGTATTACTGTGCGAGAGGGGATTACGATGGTTCGGGGAGTTATCACTTCTACTACGGTATGGACGTCTGGGGCCAAGGGACCACGGTCACCGTCTCCTCA |
| | | | SEQ ID NO: 26915 | SEQ ID NO: 30921 |

FIGURE 50

| | | | | |
|---|---|---|---|---|
| | | AA | EIVLTQSPGTLSLSPGERATLSCRASQSIRSSFLAWYQQKPGQAPRLLIYGASSRATGIPDRFSGSGSGTDFTLTISRLEPEDFAVYYCQQYGNSPWAFGQGTKVEIK | QVQLQESGPGLVKPSQTLSLTCTVSGGSISSGDYYWNWIRQLPGKGLEWIGYIFYSGSTYYNPSLKSRLTISVDTSKNQFSLKLSSVTAADTAVYYCARGDYDGSGSYHFYYGMDVWGQGTTVTVSS |
| | | | SEQ ID NO: 26916 | SEQ ID NO: 30922 |
| iPS:436197 | 21-225_199C2 | NA | GAAATTGTGTTGACGCAGTCTCCAGGCACCCTGTCTTTGTCTCCAGGGGAAAGAGCCACCCTCTCCTGCAGGGCCAGTCAGAGTATTCGCAGCAGCTTCTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAACAGACTGGAGCCTGAAGATTTTGCAGTGTATTACTGTCAGCAGTATGGTAACTCACCGTGGGCGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGATTACTACTGGAACTGGCTCCGCCAGCACCCAGAGAAGGGCCTGGAGTGGATTGGGTACATCTTTTACAGTGGGAGCACCTACTACAACCCGTCCCTCAAGAGTCGAGTTACCATATCAGTGGACACGTCTATGACCCAGTTCTCCCTGAAGCTGACCTCTGTGACTGCCGCGGACACGGCCGTGTATTACTGTGCGAGAGGGGATTACGATGGTTCGGGGAGTTATCACTACTACTACGGTATGGACGTCTGGGGCCAAGGGACCACGGTCACCGTCTCCTCA |
| | | | SEQ ID NO: 26917 | SEQ ID NO: 30923 |
| | | AA | EIVLTQSPGTLSLSPGERATLSCRASQSIRSSFLAWYQQKPGQAPRLLIYGASSRATGIPDRFSGSGSGTDFTLTINRLEPEDFAVYYCQQYGNSPWAFGQGTKVEIK | QVQLQESGPGLVKPSQTLSLTCTVSGGSISSGDYYWNWLRQHPEKGLEWIGYIFYSGSTYYNPSLKSRVTISVDTSMTQFSLKLTSVTAADTAVYYCARGDYDGSGSYHYYYGMDVWGQGTTVTVSS |
| | | | SEQ ID NO: 26918 | SEQ ID NO: 30924 |

FIGURE 50

| | | | | |
|---|---|---|---|---|
| iPS:436199 | 21-225_199E3 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATTTCTTCTGCATCCAGTTTGCAAAGGGGGGTCCCATCAAGATTCAGCGGCAGTGGATCTGGGACAGAATTCACTCTCACAATCAGCAGCCTGCAGCCTGAAGATTTTGCAACTTATTACTGTCTACAGCATAAAAGGTACCCGCTCACTTTCGGCGGAGGGACCAAGGTGGAGATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGGTTACTTCTGGACCTGGATCCGCCAGCCCCCAGGGAAGGGGCTGGAGTGGATTGGGGAAATCAATCATAGTGGAAGCACCAACTACAACCCGTCCCTCAAGAGTCGCGTCACCATATCAGTAGACACGTCCAAGAACCAGTTCTCCCTGAAGCTGAGCTCTGTGACCGCCGCGGACACGGCTGTGTATTACTGTGCGAGAGACTACGGTGCTGACTACTGGGGCCAGGGAACCCTGGTCGCCGTCTCCGCA |
| | | | SEQ ID NO: 26919 | SEQ ID NO: 30925 |
| | | AA | DIQMTQSPSSLSASVGDRVTITCRASQGIRNDLGWYQQKPGKAPKRLISSASSLQRGVPSRFSGSGSGTEFTLTISSLQPEDFATYYCLQHKRYPLTFGGGTKVEIK | QVQLQQWGAGLLKPSETLSLTCAVYGGSFSGYFWTWIRQPPGKGLEWIGEINHSGSTNYNPSLKSRVTISVDTSKNQFSLKLSSVTAADTAVYYCARDYGADYWGQGTLVAVSA |
| | | | SEQ ID NO: 26920 | SEQ ID NO: 30926 |
| iPS:436201 | 21-225_199C5 | NA | GACATCCAGATGACACAGTCTCCATCCTCACTGTCTGCATCTGTAGGAGACAGAGTCACCATCACTTGTCGGGCGAGTCAGGGCATTAGCAAGTATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAGCTTATTACTGCCAACAGTATCTTACTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCAGGGCGTGGGCTACTACGGTATGGACGTCTGGGGCCAAGGGACCACGGTCACCGTCTCCTCA |
| | | | SEQ ID NO: 26921 | SEQ ID NO: 30927 |

FIGURE 50

| | | | | |
|---|---|---|---|---|
| | | AA | DIQMTQSPSSLSASVGDRVTITCRASQGISKYLAWFQQKPGKAPKSLIYAASSLQSGVPSKFSGSGSGTDFTLTISSLQPEDFAAYYCQQYLTYPLTFGGGTKVEIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAVYYCARDQGVGYYGMDVWGQGTTVTVSS |
| | | | SEQ ID NO: 26922 | SEQ ID NO: 30928 |
| **iPS:436203** | **21-225_199A6** | NA | GACATAGTGATGACGCAGTCTCCAGCCACCCTGTCTGTGTCTCCAGGGGATAGAGCCACCCTCTCCTGCAGGCCCAGTCAGAGTTTTAGCAGAAACTTAGCCTGGTACCAGCAGAAACCTGGCCAGGCTCCCAGGCTCCTCATCTATGGTGCATCCACTAGGGCCACTGGTATCCCAGCCAGGTTCAGTGGCAGTGGGTCTGGGACAGAGTTCACTCTCACCATCAGCAGCCTGGAGTGTGAAGATTTTGCAGTTTACTACTGTCAGCAGTATAATAACTGGCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAATTATATGGTTTGATGGAAGTAATCAATACTATGCCGACTCCGTGAAGGGCCGATTCACCATCTCCAGAGACAATTCCAAGAACACGCTGTATCTGCAATTGAACAGCCTGAGAGCCGAGGACACGGCTGTCTATTACTGTGCGAGAGCCCACGGGGTCTACTACTACGCTATGGACGTCTGGGGCCAAGGGACCACGGTCACCGTCTCCTCA |
| | | | SEQ ID NO: 26923 | SEQ ID NO: 30929 |
| | | AA | DIVMTQSPATLSVSPGDRATLSCRPSQSFSRNLAWYQQKPGQAPRLLIYGASTRATGIPARFSGSGSGTEFTLTISSLECEDFAVYYCQQYNNWPLTFGGGTKVEIK | QVQLVESGGGVVQPGRSLRLSCAASGFTFSNYGMHWVRQAPGKGLEWVAIIWFDGSNQYYADSVKGRFTISRDNSKNTLYLQLNSLRAEDTAVYYCARAHGVYYYAMDVWGQGTTVTVSS |
| | | | SEQ ID NO: 26924 | SEQ ID NO: 30930 |

FIGURE 50

| | | | | |
|---|---|---|---|---|
| iPS:436205 | 21-225_199A7 | NA | GACATCCAGATGACCCAGTCTCCATCCTCCCTGTCTGCATCTGTAGGAGACAGAGTCACCATCACTTGCCGGGCAAGTCAGGGCATAAGAAAAGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATGTCTGTCTACAACATTATCGTTACCCTTTCACTTTCGGCCCTGGGACCAAAGTGGATTTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAATTATATGGTTTGATGGAAGTAATAAATACTATGCAGACTCCGTGAAGGGCCGATTCACCATCTCCAGAGACAATTCCAAGAACACGCTGTTTCTGCAAATGAGCAGCCTGAGAGCCGAGGACACGGCTGTGTATTACTGTGCGAGAGAATGGCTACAATTCAGGTACTACTACGGTATGGACGTCTGGGGCCAAGGGACCACGGTCACCGTCTCCTCA |
| | | | SEQ ID NO: 26925 | SEQ ID NO: 30931 |
| | | AA | DIQMTQSPSSLSASVGDRVTITCRASQGIRKDLGWYQQKPGKAPKRLIYAASSLQSGVPSRFSGSGSGTEFTLTISSLQPEDFATYVCLQHYRYPFTFGPGTKVDFK | QVQLVESGGGVVQPGRSLRLSCAASGFTFSNYGMHWVRQAPGKGLEWVAIIWFDGSNKYYADSVKGRFTISRDNSKNTLFLQMSSLRAEDTAVYYCAREWLQFRYYYGMDVWGQGTTVTVSS |
| | | | SEQ ID NO: 26926 | SEQ ID NO: 30932 |
| iPS:436207 | 21-225_199C7 | NA | GAAATTGTGTTGACGCAGTCTCCAGGCACCCTGTCTTTGTCTCCAGGGGAAAGAGCCACCCTCTCCTGCAGGGCCAGTCAGAGTATAAGGACCAACTTCTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAACAGACTGGAGCCTGAAGATTTTGCAGTGTATTACTGTCAGCAGTATGGTAACTCACCGTGGACGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGGCTCCATCAACAGTGGTGGTTACTACTGGAACTGGATCCGCCAGCACCCAGGGAAGGGCCTGGAGTGGATTGGGTTCATCTTTTACAGTGGGAGCACCTACTACAACCCGTCCCTCAAGAGTCGAGTTTCCATATCAGTAGACACGTCTAAGAACCAGTTCTCCCTGAAGCTGAGCTCTGTGACTGCCGCGGACACGGCCGTGTATTACTGTGCGAGAGGGGATTACGATGGTTCGGGGAGTTATCACTACTATTACGGTATGGACGTCTGGGGCCAGGGGACCACGGTCACCGTCTCCTCA |
| | | | SEQ ID NO: 26927 | SEQ ID NO: 30933 |

FIGURE 50

| | | | | |
|---|---|---|---|---|
| | | AA | EIVLTQSPGTLSLSPGERATLSCRASQSIRTNFLA<br>WYQQKPGQAPRLLIYGASSRATGIPDRFSGSGSG<br>TDFTLTINRLEPEDFAVYYCQQYGNSPWTFGQG<br>TKVEIK | QVQLQESGPGLVKPSQTLSLTCTVSGGSINSGGYY<br>WNWIRQHPGKGLEWIGFIFYSGSTYYNPSLKSRVSI<br>SVDTSKNQFSLKLSSVTAADTAVYYCARGDYDGS<br>GSYHYYYGMDVWGQGTTVTVSS |
| | | | SEQ ID NO: 26928 | SEQ ID NO: 30934 |
| **iPS:436210** | **21-225_199G11** | NA | GAAATTGTGGTGACGCAGTCTCCAGGCACCCT<br>GTCTTTGTCTCCAGGGGAAAGAGCCACCCTCT<br>CCTGCAGGGCCAGTCAGAGTGTTAGAAGCAGC<br>TACTTAGCCTGGTACCAGCAGAAACCTGGCCA<br>GGCTCCCAGGCTCCTCATCTATGGTGCATTCA<br>GCAGGGCCACTGGCATCCCAGACAGGTTCAGT<br>GGCAGTGGGTCTGGGACAGACTTCACTCTCAC<br>CATCAGCAGACTGGAGCCTGAAGATTTTGCAG<br>TGTACTACTGTCAGCAGTATGGTAACTCACCG<br>TGGACGTTCGGCCACGGGACCAAGGTGGAAA<br>TCAAA | CAGGTGCAGCTGCAGGAGTCGGGCCCAGGACTG<br>GTGAAGCCCTCACAGACCCTATCCCTCACCTGCA<br>CTGTCTCTGGTGGCTCCATCAGCAGTGGTGGTTA<br>CTACTGGAGCTGGATCCGCCAGCACCCAGGGAA<br>GGGCCTGGAGTGGATTGGGAACATCTATTACAGT<br>GGGAGCACCTACTACAACCCGTCCCTCAAGAGTC<br>GAGTTACCATATCAGTAGACACGTCTAAGAACCA<br>GTTCTCCCTGAAGCTGAGCTCTGTGACTGCCGCG<br>GACACGGCCGTGTATTACTGTGCGAGAGGGGATT<br>ACTATGGTTCGGGGAGTTATCACAACTACTACGG<br>TTTGGACGTCTGGGGCCAAGGGACCACGGTCACC<br>GTCTCCTCA |
| | | | SEQ ID NO: 26929 | SEQ ID NO: 30935 |
| | | AA | EIVVTQSPGTLSLSPGERATLSCRASQSVRSSYLA<br>WYQQKPGQAPRLLIYGAFSRATGIPDRFSGSGSG<br>TDFTLTISRLEPEDFAVYYCQQYGNSPWTFGHGT<br>KVEIK | QVQLQESGPGLVKPSQTLSLTCTVSGGSISSGGYYW<br>SWIRQHPGKGLEWIGNIYYSGSTYYNPSLKSRVTIS<br>VDTSKNQFSLKLSSVTAADTAVYYCARGDYYGSG<br>SYHNYYGLDVWGQGTTVTVSS |
| | | | SEQ ID NO: 26930 | SEQ ID NO: 30936 |

FIGURE 50

| | | | | |
|---|---|---|---|---|
| **iPS:436212** | **21-225_200G1** | NA | GACATCCAGATGACCCAGTCTCCATCCTCCCTGTCTGCATCTGTAGGAGACAGAGTCACCATCACTTGCCGGGCAAGTCAGAGCATTAGCAGCTATTTAAATTGGTTTCAGCAGAAACCAGGGAAAGCCCCTAAGCTCCTGATCTATGCTGAGTCCAGTTTACAAAGTGGGGTCCCATCAAGGTTCAGTGGCAGTGGATCTGGGACAGATTTCACTCTCACCATCAGCAGTCTGCAACCTGAAGATTTTGCAACTTATTCCTGTCAACAGAGTTACAGTTCCCCTCCGTGGACGTTCGGCCAAGGGACCAAGGTGGAGTTCAAA | GAGGTGCAACTGTTGGAGTCTGGGGGAGGCTTGGTGCAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTAGAGGCGGTAATACATTCTACGCAGACTCCGTGAGGGGCCGGTTCACCATCTCCAGAGACAATTCCAAGAACACGCTGTATCTGCAAATGAACAGCCTGAGAGCCGAGGACACGGCCGTATATTACTGTGCGCGACGTATAGCAGTGGATGGCTATGATGCTTTTGATGTCTGGGGCCAAGGGACAATGGTCACCGTCTCTTCA |
| | | | SEQ ID NO: 26931 | SEQ ID NO: 30937 |
| | | AA | DIQMTQSPSSLSASVGDRVTITCRASQSISSYLNWFQQKPGKAPKLLIYAESSLQSGVPSRFSGSGSGTDFTLTISSLQPEDFATYSCQQSYSSPPWTFGQGTKVEFK | EVQLLESGGGLVQPGGSLRLSCAASGFTFSSYAMSWVRQAPGKGLEWVSAISGRGGNTFYADSVRGRFTISRDNSKNTLYLQMNSLRAEDTAVYYCARRIAVDGYDAFDVWGQGTMVTVSS |
| | | | SEQ ID NO: 26932 | SEQ ID NO: 30938 |
| **iPS:436214** | **21-225_200F6** | NA | GACATCCAGATGACCCAGTCTCCATCCTCCCTGTCTGCATCTGTAGGAGACAGAGTCACCATCACTTGCCGGGCAAGTCAGGACATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATCACTGTCTACAGCATTATCGTTACCCATTCACTTTCGGCCCTGGGACCAAAGTGGATATCAAA | CAGGTGCAGCTGGTGGAGTCTGGGGGAGGCGTGGTCCAGCCTGGAAGGTCCCTGAGACTCTCCTGTGCAGCGTCTGGATTCACCTTCAGTAGTTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTACGAGAGAATGGCTACAATTTAGGTATTACTACGGTATGGACGTCTGGGGCCAAGGGACCACGGTCACCGTCTCCTCA |
| | | | SEQ ID NO: 26933 | SEQ ID NO: 30939 |

FIGURE 50

| | | | | |
|---|---|---|---|---|
| | | AA | DIQMTQSPSSLSASVGDRVTITCRASQDIRNDLGWYQQKPGKAPKRLIYAASSLQSGVPSRFSGSGSGTEFTLTISSLQPEDFATYHCLQHYRYPFTFGPGTKVDIK | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCTREWLQFRYYYGMDVWGQGTTVTVSS |
| | | | SEQ ID NO: 26934 | SEQ ID NO: 30940 |
| **iPS:436216** | **21-225_200B7** | NA | GAAATTGTGCTGACTCAGTCTCCAGACTTTCAGTCTGTGACTCCAAAGGAGAAAGTCACCATCACCTGCCGGGCCAGTCAGAACATTGGTAATACCTTGCACTGGTACCAGCAGAAACCAGATCAGTCTCCTAAGCTCCTCATCAAGTATGCTTCCCAGTCCTTCTCAGGGGTCCCCTCGAGGTTCAGTGGCAGTGGGTCTGGGACAGATTTCATCCTCACCATCAATAGCCTGGAAGCTGAAGATGCTGCAACGTATTACTGTCATCAGAGTGGTAGTTTACCTCAGACGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGGTTACTACTGGAGCTGGATCCGCCAGCACCCAGGGAAGGGCCTGGAGTGGATTGGGTACATCTTTTACAGTGGGAGCACCAACTACAACCCGTCCCTCAGGAGTCGAGTTACCATATCAGTAGACACGTCTAAGAACCAGTTCTCCCTGAAACTGAGTTCTGTGACTGCCGCGGACACGGCCGTGTATTACTGTGCGAGAGCCGGGTATAACTGGAACAACGGTATGGACGTCTGGGGCCAAGGGACCACGGTCACCGTCTCCTCA |
| | | | SEQ ID NO: 26935 | SEQ ID NO: 30941 |
| | | AA | EIVLTQSPDFQSVTPKEKVTITCRASQNIGNTLHWYQQKPDQSPKLLIKYASQSFSGVPSRFSGSGSGTDFILTINSLEAEDAATYYCHQSGSLPQTFGQGTKVEIK | QVQLQESGPGLVKPSQTLSLTCTVSGGSISSGGYYWSWIRQHPGKGLEWIGYIFYSGSTNYNPSLRSRVTISVDTSKNQFSLKLSSVTAADTAVYYCARAGYNWNNGMDVWGQGTTVTVSS |
| | | | SEQ ID NO: 26936 | SEQ ID NO: 30942 |

FIGURE 50

| | | | | |
|---|---|---|---|---|
| iPS:436218 | 21-225_200G7 | NA | GACATCGTGATGACCCAGTCTCCAGACTCCCCGACTGCGTCTCTGGGCGAGAGGGCCACCGTCAAATGCAAGTCCAGCCAGAGTGTTTTACACAGCTCCAACAATAACAACTACTTAGCTTGGTACCAGCAGAAACCAGGACAGCCTCCTAAGCTGCTCATTTACTGGGCATCTACCCGGGAATCCGGAGTCCCTGACCGATTCAGTGGCAGCGGGTCTGGGACAGATTTCACTCTCGCCATCAGCAGCCTGCAGGCTGAAGATGTGGCGGTTTATTACTGTCAGCAATATTATAATACTCCTCCGACATTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGATGCGGCAGGCCACTGGTCAAGGGCTTGAGTGGATGGGATGGATGCACCTTAACAGTGGTAACACAGGCTATGCACCGAAGTTCCAGGGCAGAGTCACCATGACCAGGGACACCTCCATAAGCACAGCCTTCATGGAGCTGAGCAGCCTGAGATCTGAGGACACGGCCGTGTATTACTGTGCCTATAGCGGTGGCTGGTACGTGTTCGACCCCTGGGGCCAGGGAACCCTGGTCACCGTCTCCTCA |
| | | | SEQ ID NO: 26937 | SEQ ID NO: 30943 |
| | | AA | DIVMTQSPDSPTASLGERATVKCKSSQSVLHSSNNNNYLAWYQQKPGQPPKLLIYWASTRESGVPDRFSGSGSGTDFTLAISSLQAEDVAVYYCQQYYNTPPTFGQGTKVEIK | QVQLVQSGAEVKKPGASVKVSCKASGYTFTNYDINWMRQATGQGLEWMGWMHLNSGNTGYAPKFQGRVTMTRDTSISTAFMELSSLRSEDTAVYYCAYSGGWYVFDPWGQGTLVTVSS |
| | | | SEQ ID NO: 26938 | SEQ ID NO: 30944 |
| iPS:436220 | 21-225_200F8 | NA | GAAATTGTGCTGACTCAGTCTCCAGACTTTCAGTCTGTGGCTCCAAAGGAGAAAGTCACCATCACCTGCCGGGCCAGTCAGAGTATTGGTAGTAACTTACACTGGTACCAGCAGAAACCAGATCAGTCTCCGAAACTCCTCATCAAGTCTGCTTCCCAGTCCTTCTCAGGGGTCCCCTCGAGGTTCAGTGGCAGTGGATCTGGGACAGATTTCACCCTCACCATCAATAGCCTGGAAACTGAAGATGCTGCAACGTATTACTGTCAGCAGAGTAGTAGTTTACCGTGGACGTTCGGCCAAGGGACCAAGGTGGAAATCAAA | CAGGTGCAGCTGCAGGAGTCGGGCCCAGGACTGGTGAAGCCTTCGGAGACCCTGTCCCTCACCTGCACTGTCTCTGGTGGCTCCATCAATAATTACTACTGGAGCTGGATCCGGCAGCCCGCCGGGAAGGGACTGGAGTGGATTGGGCGTATCTATACCAGTGGGAGCACCAACTACAACCCCTCCCTCAAGAGTCGAGTCACCATGTCAGTAGACACGTCCAAGAACCAGTTCTCCCTGAAGCTGAGCTCTGTGACCGCCGCGGACACGGCCGTGTATTACTGTGCGAGAGATCGGGGATACTATGGCTACTACGGTATGGACGTCTGGGGCCAAGGGACCACGGTCACCGTCTCCTCA |
| | | | SEQ ID NO: 26939 | SEQ ID NO: 30945 |

FIGURE 50

| | | | | |
|---|---|---|---|---|
| | | AA | EIVLTQSPDFQSVAPKEKVTITCRASQSIGSNLHWYQQKPDQSPKLLIKSASQSFSGVPSRFSGSGSGTDFTLTINSLETEDAATYYCQQSSSLPWTFGQGTKVEIK | QVQLQESGPGLVKPSETLSLTCTVSGGSINNYYWSWIRQPAGKGLEWIGRIYTSGSTNYNPSLKSRVTMSVDTSKNQFSLKLSSVTAADTAVYYCARDRGYYGYYGMDVWGQGTTVTVSS |
| | | | SEQ ID NO: 26940 | SEQ ID NO: 30946 |
| iPS:436222 | 21-225_200C9 | NA | GACATCCAGATGACCCAGTCTCCATCCTCCCTGTCTGCATCTGTTGGAGACAGAGTCACCATCACTTGCCGGGCAACTCAGGGCATTAGAAAAGATTTAGGCTGGTATCAGCAGAAACCAGGGAAAGCCCCTAAGCGCCTGATCCATACTGCATCCAGTTTGCAAAGTGGGGTCCCATTAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGTGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAGTGGGTGGCAGTTATATCATATGATGGAGGTTATAAAAACTATATAGACTCCGTGAAGGGCCGATTCACCATCTCCAGAGACAATTCCAAGAACACGCTGTATCTGCAAATGAACAGCCTGAGAGCTGAGGACACGGCTGTGTTTTACTGTGCGAGAGGTACCCACGGGTACTACTACGGTGTGGACGTCTGGGGCCAAGGGACCACGGTCACCGTCTCCTCA |
| | | | SEQ ID NO: 26941 | SEQ ID NO: 30947 |
| | | AA | DIQMTQSPSSLSASVGDRVTITCRATQGIRKDLGWYQQKPGKAPKRLIHTASSLQSGVPLRFSGSGSGTEFTLTISSLQPEDFATYYCLQHNSYPWTFGQGTKVEIK | QVQLVESGGGVVQPGRSLRLSCAASGFTFSSYGMHWVRQAPGKGLEWVAVISYDGGYKNYIDSVKGRFTISRDNSKNTLYLQMNSLRAEDTAVFYCARGTHGYYYGVDVWGQGTTVTVSS |
| | | | SEQ ID NO: 26942 | SEQ ID NO: 30948 |

FIGURE 50

| | | | | |
|---|---|---|---|---|
| **iPS:436226** | **21-225_200F10** | NA | GAAATTGTGTTGACGCAGTCTCCAGGCACCCTGTCTTTGTCTCCAGGGGAAAGAGCCACCCTCTCCTGCAGGGCCAGTCAGAATATTCGCAGCAGCTTCTTAGCCTGGTACCAACAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAACAGACTGGAGCCTGAAGATTTTGCAGTGTATTACTGTCAGCAGTATGGTAACTCACCGTGGGCGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGATTACTACTGGAACTGGCTCCGCCAGCACCCAGAGAAGGGCCTGGAGTGGATTGGGTACATCTTTTACAGTGGGAGCACCTACTACAACCCGTCCCTCAAGAGTCGAATTACCATATCAGTGGACACGTCTATGACCCAGTTCTCCCTGAAGCTGACCTCTGTGACTGCCGCGGACACGGCCGTGTATTACTGTGCGAGAGGGGATTACGATGGTTCGGGGAGTTATCACTACTACTACGGTATGGACGTCTGGGGCCAAGGGACCACGGTCACCGTCTCCTCA |
| | | | SEQ ID NO: 26943 | SEQ ID NO: 30949 |
| | | AA | EIVLTQSPGTLSLSPGERATLSCRASQNIRSSFLAWYQQKPGQAPRLLIYGASSRATGIPDRFSGSGSGTDFTLTINRLEPEDFAVYYCQQYGNSPWAFGQGTKVEIK | QVQLQESGPGLVKPSQTLSLTCTVSGGSISSGDYYWNWLRQHPEKGLEWIGYIFYSGSTYYNPSLKSRITISVDTSMTQFSLKLTSVTAADTAVYYCARGDYDGSGSYHYYYGMDVWGQGTTVTVSS |
| | | | SEQ ID NO: 26944 | SEQ ID NO: 30950 |
| **iPS:436228** | **21-225_200F12** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAGAGCCCCTAAGCGCCTGATCTATTCTGCATCCAGTTTGCATACTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAAGAGTTACCCGCTCACTTTCGGCGGAGGGACCAAGGTGGAGATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGGTTACTTCTGGACCTGGATACGCCAGCCCCCAGGGAAGGGACTGGAGTGGATTGGGGAAATCAGTCATAGTGGAAGCACCAACTACAACCCGTCCCTCAAGAGTCGAGTCACCATATCAGTAGACACGTCCAAGAACCAGTTCTCCCTGAAGGTGAGCTCTGTGACCGCCGCGGACACGGCTGTGTATTACTGTGCGAGGGACTACGGGGCGGACTACTGGGGCCAGGGAACCCTGGTCACCGTCTCCTCA |
| | | | SEQ ID NO: 26945 | SEQ ID NO: 30951 |

FIGURE 50

| | | | | |
|---|---|---|---|---|
| | | AA | DIQMTQSPSSLSASVGDRVTITCRASQGIRNDLGWYQQKPGRAPKRLIYSASSLHTGVPSRFSGSGSGTEFTLTISSLQPEDFATYYCLQHKSYPLTFGGGTKVEIK | QVQLQQWGAGLLKPSETLSLTCAVYGGSFSGYFWTWIRQPPGKGLEWIGEISHSGSTNYNPSLKSRVTISVDTSKNQFSLKVSSVTAADTAVYYCARDYGADYWGQGTLVTVSS |
| | | | SEQ ID NO: 26946 | SEQ ID NO: 30952 |
| iPS:436230 | 21-225_201A1 | NA | GACATCCAGATGACCCAGTCTCCATCCTCCCTGTCTGCATCTGTAGGAGACAGAGTCACCATCACTTGCCGGGCAAGTCAGGGCATTAGGAATGATTTAGGCTGGTATCAGCAGAAACCAGGGAAAGCCCCTAAGCGCCTGATCTATTCTGCATCCATTTTACAAAGGGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAAAAGTTACCCTCTCACTTTCGGCGGAGGGACCAAGGTGGAGGTCAAA | CAGGTGCAGCTACAGCAGTGGGGCGCAGGACTGTTGAAACCTTCGGAGACCCTGTCCCTCACCTGCGCTGTCACTGGTGGGTCCTTCAGTGGTTACTTCTGGACCTGGATCCGCCAGCCCCCAGGGAAGGGGCTGGAGTGGATTGGGGAAATCAGTCATAGTGGACGCACCAACTACAACCCGTCCCTCAAGAGTCGAGTCACCATATCAGGAGACACGTCCAAGAACCAGTTCTCCCTGAAGCTGAGCTCTGTGACCGCCGCGGACACGGCTGTGTATTACTGTGCGAGGGACTACGGGGCGGACTACTGGGGCCAGGGAACCCTGGTCACCGTCTCCTCA |
| | | | SEQ ID NO: 26947 | SEQ ID NO: 30953 |
| | | AA | DIQMTQSPSSLSASVGDRVTITCRASQGIRNDLGWYQQKPGKAPKRLIYSASILQRGVPSRFSGSGSGTEFTLTISSLQPEDFATYYCLQHKSYPLTFGGGTKVEVK | QVQLQQWGAGLLKPSETLSLTCAVTGGSFSGYFWTWIRQPPGKGLEWIGEISHSGRTNYNPSLKSRVTISGDTSKNQFSLKLSSVTAADTAVYYCARDYGADYWGQGTLVTVSS |
| | | | SEQ ID NO: 26948 | SEQ ID NO: 30954 |

FIGURE 50

| | | | | |
|---|---|---|---|---|
| iPS:436232 | 21-225_201E1 | NA | GAAATTGTGTTGACGCAGTCTCCAGACACCCTGTCTTTGTCTCCAGGGGAAAGAGCCACCCTCTCCTGCAGGGCCAGTCCGAGTATTAACAGCGGCTTCTTAGCCTGGTACCAGCAGAAACCTGGCCAAGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAATGTTTCACTGTCACCAGTATGAGACCTCACCGTGGACGTTCGGCCAAGGGACCAAGGTGGAGATCAAA | CAGGTGCAGCTACAGCAGTGGGGCGCAGGACTGTTGAAGCCTTCGGAGACCCTGTCCCTCACCTGCGCTGTCTTTGATGGGTCCTTCAGTCCTTACTACTGGAGCTGGATCCGCCAGCCCCCAGGGAAGGGGCTGGAGTGGATTGGGGAAGTCAATCATAGTGGAAGCACCAACTACAACCCGTCCCTCAAGAGTCGAGTCACCATATCAGTAGACACGTCCAAGAACCAGTTCTCCCTGAAGCTGAGCTCTGTGACCGCCGCGGACACGGCTGTGTATTACTGTGCGAGAGACTACGGGGGTTTAGACTACTGGGGCCAGGGAGCCCTGGTCACCGTCTCCTCA |
| | | | SEQ ID NO: 26949 | SEQ ID NO: 30955 |
| | | AA | EIVLTQSPDTLSLSPGERATLSCRASPSINSGFLAWYQQKPGQAPRLLIYGASSRATGIPDRFSGSGSGTDFTLTISRLEPEDFAMFHCHQYETSPWTFGQGTKVEIK | QVQLQQWGAGLLKPSETLSLTCAVFDGSFSPYYWSWIRQPPGKGLEWIGEVNHSGSTNYNPSLKSRVTISVDTSKNQFSLKLSSVTAADTAVYYCARDYGGLDYWGQGALVTVSS |
| | | | SEQ ID NO: 26950 | SEQ ID NO: 30956 |
| iPS:436234 | 21-225_51E3 | NA | CAGTCTGTGCTGACTCAGCCACCCTCAGCGTCTGGGACCCCCGGGCAGAGGGTCACCATCTCTTGTTCTGGAAGCAACTCCAACATCGGAAGTAATATTGTAACCTGGTACCAGCAGCTCCCAGGAACGGCCCCCAAACTCCTCATCTATAGTAATGATCAGCGGCCCTCAGGGGTCCCTGACCGATTCTCTGGCTCCAAGTCTGGCACCTCAGCCTCCCTGGCCATCAGTGGGCTCCAGTCTGAGGATGAGGCTGATTATTACTGTACAGCATGGGATGACAGCCTGAATGGTTGGGTGTTCGGCGGAGGTACCACGCTGACCGTCCTA | CAGGTTCAGCTGGTGCAGTCTGGAGCTGAGGTGAAGAAGCCTGGGGCCTCAGTGAAGGTCTCCTGCAAGGCTTCAGGTTACACCTTTAACAGCTATGGTATCAGCTGGGTGCGACTGGCCCCTGGACAAGGGCTTGAGTGGATGGGATGGATCAGCGCTTATAATGGTAACACAAAGAATGCACAGAGGTTCCAGGGCAGAGTCACCATGACCACAGACACATCCACGAGCACGGCCTACATGGAGCTGAGGAGCCTGAGATCTGACGACACGGCCGTTTATTACTGTGCGAGACACGATTTTTGGAGTGGTTATTATAAGGGTATGGACGTCTGGGGCCAAGGGACCACGGTCACCGTCTCCTCA |
| | | | SEQ ID NO: 26951 | SEQ ID NO: 30957 |

FIGURE 50

| | | | | |
|---|---|---|---|---|
| | | AA | QSVLTQPPSASGTPGQRVTISCSGSNSNIGSNIVTWYQQLPGTAPKLLIYSNDQRPSGVPDRFSGSKSGTSASLAISGLQSEDEADYYCTAWDDSLNGWVFGGGTTLTVL | QVQLVQSGAEVKKPGASVKVSCKASGYTFNSYGISWVRLAPGQGLEWMGWISAYNGNTKNAQRFQGRVTMTTDTSTSTAYMELRSLRSDDTAVYYCARHDFWSGYYKGMDVWGQGTTVTVSS |
| | | | SEQ ID NO: 26952 | SEQ ID NO: 30958 |
| **iPS:436236** | **21-225_201F7** | NA | GAAATAGTGATGACGCAGTCTCCAGCCACCCTGTCTGTGTCTCCAGGGGAAAGAGCCACCCTCTCCTGCAGGGCCAGTCAGAATATTAAAAACAACTTAGCCTGGTACCAGCAGAAACCTGGCCAGGCTCCCAGGCTCCTCATCTTTGGTGCATCCACCAGGGCCACTGGTATCCCAGCCAGGTTCAGTGGCAGTGGGTCTGGGACAGAGTTCACTCTCACCATCAGCAGCCTGCAGTCTGAAGATTTTGCAGTTTATTACTGTCAGCAGTTTTATAACTGGCTGTGCAGTTTTGGCCAGGGGACCAAGCTGGAGCTCAAA | CAGGTACAGCTGCAGCAGTCAGGTCCAGGACTGGTGAAGCCCTCGCAGACCCTCTCACTCACCTGTGCCATCTCCGGGGACAGTGTCTCTAGCAACAGTGCTGCTTGGAACTGGATCAGGCAGTCCCCATCGAGAGGCCTTGAGTGGCTGGGAAGGACATACTACAGGTCCAAGTGGTATAATTATTATGAAGTATCTGTGAGAAGTCGAATAACCATCAACCCAGACACATCCAAGAACCAGTTCTCCCTGCAGCTGAACTCTGTGACTCCCGAGGACACGGCTGTATATTTCTGTGCGAGAGATCAACGGTACTACGGTATGGACGTCTGGGGCCAAGGGACCACGGTCACCGTCTCCTCA |
| | | | SEQ ID NO: 26953 | SEQ ID NO: 30959 |
| | | AA | EIVMTQSPATLSVSPGERATLSCRASQNIKNNLAWYQQKPGQAPRLLIFGASTRATGIPARFSGSGSGTEFTLTISSLQSEDFAVYYCQQFYNWLCSFGQGTKLELK | QVQLQQSGPGLVKPSQTLSLTCAISGDSVSSNSAAWNWIRQSPSRGLEWLGRTYYRSKWYNYYEVSVRSRITINPDTSKNQFSLQLNSVTPEDTAVYFCARDQRYYGMDVWGQGTTVTVSS |
| | | | SEQ ID NO: 26954 | SEQ ID NO: 30960 |

FIGURE 50

| | | | | |
|---|---|---|---|---|
| iPS:436238 | 21-225_201B2 | NA | GAAATTGTTTTGACGCAGTCTCCAGGCACCCTGTCTTTGTCTCCAGGGGAAAGAGCCACCCTCTCCTGCAGGGCCAGTCAGAGTGTTAGCAGCAACTACTTAGCCTGGTATCAGCAGAGACCTGGCCAGGCTCCCAGACTCCTCATCTATGGTGCATCCAGCAGGGCCACTGGCATCCCAGACAGATTCAGTGGCAGTGGGTCTGGGACAGACTTCACTCTCACCATCAGCAGACTGGAGCCTGAAGATTTTGCAGTGTATTTCTGTCAGCAGTATGAAAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTTTGGTGGGTCCATCAGTGTTTACTACTGGACCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATATCAGTAGACACGTCCAAGAACCAGTTCTCCCTGAAGCTGAGCTCTGTGACCGCCGCGGACACGGCTGTGTATTACTGTGCGAGGGACTATGGTGTCTTTGATTACTGGGGCCAGGGAACCCTGGTCACCGTCTCCTCA |
| | | | SEQ ID NO: 26955 | SEQ ID NO: 30961 |
| | | AA | EIVLTQSPGTLSLSPGERATLSCRASQSVSSNYLAWYQQRPGQAPRLLIYGASSRATGIPDRFSGSGSGTDFTLTISRLEPEDFAVYFCQQYENSPWTFGQGTKVEIK | QVQLQQWGAGLLKPSETLSLTCAVFGGSISVYYWTWIRQPPGKGLEWIGEINHSGSTNYNPSLKSRVTISVDTSKNQFSLKLSSVTAADTAVYYCARDYGVFDYWGQGTLVTVSS |
| | | | SEQ ID NO: 26956 | SEQ ID NO: 30962 |
| iPS:436240 | 21-225_201E8 | NA | GAAATTGTGCTGACTCAGTCTCCAGCCTTTCAGTCTGTGACTCCAAAGGAGAAAGTCACCATCACCTGCCGGGCCAGTCAGAACATTGGTCGTAGTTTACACTGGTACCAGCAGAAACCAGATCAGTCTCCAAAACTCCTCATCAAGTATGCTTCCCAGTCCTTCTCAGGGGTCCCCTCGAGGTTCAGTGGCAGTGGATCTGGGACAAATTTCACCCTCACCATCAATAGCCTGGAAGCTGAAGATGCTGTAACGTATTACTGTCATCAGAGTCGAAGTTTACCGCTCACTTTCGGCGGAGGGACCAAGGTAGAGATCAGA | CAGGTGCAGCTGGTACAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCGACCCTAACAGTGGTGGCACAAACTATCCACAGAAGTTTCAGGGCAGGGTCACCATGACCAGGGACACGTCCATCAGCACAGCCTACATGGAACTGAGCAGGCTGAGATCTGACGACACGGCCGTGTTTTACTGTGCGAAAGATCAAGGGTATAACTGGAACTCTTTTGACTACTGGGGCCAGGGAACCCTGGTCACCGTCTCCTCA |
| | | | SEQ ID NO: 26957 | SEQ ID NO: 30963 |

FIGURE 50

| | | | | |
|---|---|---|---|---|
| | | AA | EIVLTQSPAFQSVTPKEKVTITCRASQNIGRSLHWYQQKPDQSPKLLIKYASQSFSGVPSRFSGSGSGTNFTLTINSLEAEDAVTYYCHQSRSLPLTFGGGTKVEIR | QVQLVQSGAEVKKPGASVKVSCKASGYTFTGYYMHWVRQAPGQGLEWMGWIDPNSGGTNYPQKFQGRVTMTRDTSISTAYMELSRLRSDDTAVFYCAKDQGYNWNSFDYWGQGTLVTVSS |
| | | | SEQ ID NO: 26958 | SEQ ID NO: 30964 |
| **iPS:436242** | **21-225_201A10** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAGAGCCCCTAAGCGCCTGATCTATTCTACATCCAGTTTGCATTCTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGAGGGACCAAGGTGGAGATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGGTTACTTCTGGACCTGGATACGCCAGCCCCCAGGGAAGGGACTGGAGTGGATTGGGGAAATCAGTCATAGTGGAAGCACCAACTACAACCCGTCCCTCAAGAGTCGAGTCACCATATCAGTAGACACGTCCAAGAACCAGTTCTCCCTGAAGGTGAACTCTGTGACCGCCGCGGACACGGCTGTGTATTACTGTGCGAGGGACTACGGGGCGGACTACTGGGGCCAGGGAACCCTGGTCACCGTCTCCTCA |
| | | | SEQ ID NO: 26959 | SEQ ID NO: 30965 |
| | | AA | DIQMTQSPSSLSASVGDRVTITCRASQGIRNDLGWYQQKPGRAPKRLIYSTSSLHSGVPSRFSGSGSGTEFTLTISSLQPEDFATYYCLQHNSYPLTFGGGTKVEIK | QVQLQQWGAGLLKPSETLSLTCAVYGGSFSGYFWTWIRQPPGKGLEWIGEISHSGSTNYNPSLKSRVTISVDTSKNQFSLKVNSVTAADTAVYYCARDYGADYWGQGTLVTVSS |
| | | | SEQ ID NO: 26960 | SEQ ID NO: 30966 |

FIGURE 50

| | | | | |
|---|---|---|---|---|
| iPS:436244 | 21-225_201H10 | NA | GACATCCAGATGACCCAGTCTCCATCCTCCCTGTCTGCATCTGTTGGAGACAGAGTCACCATCACTTGCCGGGCAAGTCACAACATTAACAGCTATTTAAATTGGTATCAGCAGAAATCAGGGAAAGCCCCTAAACTCCTAATCTATGCTGCATCCAGTTTGCAAAGTGGGGTCCCGTCAAGGTTCAGTGGCAGTGGATCTGGGACAGATTTCACTCTCACCTTCAGTAGTCTACAACCTGAAGATTTTGCAACTTACTACTGTCAACAGAGTTACAGTTTCCCGCTCACTTTCGGCGGAGGGACCAAGGTGGAGATGAGG | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATCCACTGGGTGCGACAGGCCCCTGGACAAGGGCTTGAGTGGATGGCATGGATCAACCCTAATAGTGGTGGCACAAACTATGCACAGAAGTTTCAGGGCAGGGTCACCATGACCAGGGACACGTCCATCACCACAACCTACATGGAGCTGAGCAGGCTGAGATCTGACGACACGGCCGTGTATTACTGTGCGAGAGGATACAGCTATGGTTACAACTGGTTCGACCCCTGGGGCCAGGGAACCCTGGTCACCGTCTCCTCA |
| | | | SEQ ID NO: 26961 | SEQ ID NO: 30967 |
| | | AA | DIQMTQSPSSLSASVGDRVTITCRASHNINSYLNWYQQKSGKAPKLLIYAASSLQSGVPSRFSGSGSGTDFTLTFSSLQPEDFATYYCQQSYSFPLTFGGGTKVEMR | QVQLVQSGAEVKKPGASVKVSCKASGYTFTGYYIHWVRQAPGQGLEWMAWINPNSGGTNYAQKFQGRVTMTRDTSITTTYMELSRLRSDDTAVYYCARGYSYGYNWFDPWGQGTLVTVSS |
| | | | SEQ ID NO: 26962 | SEQ ID NO: 30968 |
| iPS:436246 | 21-225_201G6 | NA | GATATTGTGATGACTCAGTCTCCACTCTCCCTGCCCGTCACCCCTGGAGAGCCGGCCTCCATCTCCTGCAGGTCTAGTCAGAGCCTCCTCCATAATAATAGATACAACCATTTGGATTGGTACCTGCAGAAGCCAGGACAGTCTCCACAGCTCCTGATCTATTTGGGTTCTAATCGGGCCTCCGGGGTCCCTGACAGGTTCAGTGGCAGTGGATCAGGCACAGATTTTACACTGAAAATCAGCAGAGTGGAGGCTGAGGATGTTGGGGTTTATTACTGCATGCAAGCTCTACAAACTCCCACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGTAGCCTCTGGATTCACCTTTAGCAGCTATGCCATGAGCTGGGTCCGCCAGGCTCCAGGGAAGGGGCTGGAGTGGGTCTCAACTATTAGTGGTAGTGGTGTTAGAACATACTACGCAGACTCCGTGAAGGGCCGGTTCACCATCTCCAGAGACAATTCCAAGAACACGCTGTTTCTGCAAATGAACAGCCTGAGAGCCGAGGACACGGCCGTATATTACTGTGCGAAAGGGGGAGCTAGGAGCAGTGGCTGGTTCCACTTTGACTACTGGGGCCAGGGAACCCTGGTCACCGTCTCCTCA |
| | | | SEQ ID NO: 26963 | SEQ ID NO: 30969 |

FIGURE 50

| | | | | |
|---|---|---|---|---|
| | | AA | DIVMTQSPLSLPVTPGEPASISCRSSQSLLHNNRYNHLDWYLQKPGQSPQLLIYLGSNRASGVPDRFSGSGSGTDFTLKISRVEAEDVGVYYCMQALQTPTFGGGTKVEIK | EVQLLESGGGLVQPGGSLRLSCVASGFTFSSYAMSWVRQAPGKGLEWVSTISGSGVRTYYADSVKGRFTISRDNSKNTLFLQMNSLRAEDTAVYYCAKGGARSSGWFHFDYWGQGTLVTVSS |
| | | | SEQ ID NO: 26964 | SEQ ID NO: 30970 |
| iPS:436248 | 21-225_202A3 | NA | GACATCCAGATGTCCCAATCTCCATCCTCCCTGTCTGCATCTGTTGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTGTGCTGCATCCAGGTTGCAAAGTGGGGTCCCATCAAGGTTCAGCGGCAGTGGATCTGGGACAGAATTCACTCTCACAATCAGCAGCCTGCAGCCTGAAGATTTTGCAATTTATTACTGTCTACAGCATCATGACTACCCATTCACTTTCGGCCCTGGGACCAAAGTGGATATCAAA | CAGGTGCAGCTGGAGCAGTCTGGGGCTGAGGTGAAGAAGCCTGGGGCCTCAGTGAAGGTCTCCTGCAAGGCTTCTGGATACACCTTCACCAGTTATGATATCAACTGGGTGCGACAGGCCACAGGACAAGGGCTTGAGTGGCTGGGATGGATGAACCCTAAGAGAGGTAACACAGGCTATGCACAGAAGTTCCAGGGCAGAGTCACCATGACCAGGAATACCTCCATAAGCACAGCCCACATGGAGCTGAGCAGCCTGAGATCTGAGGACACGGCCGTGTATTACTGTGCGAGAGGAAGGTATAGCAGGGAGGATTACTACTACTATTATGATATGGACGTCTGGGGCCAAGGGACCACGGTCACCGTCTCCTCA |
| | | | SEQ ID NO: 26965 | SEQ ID NO: 30971 |
| | | AA | DIQMSQSPSSLSASVGDRVTITCRASQGIRNDLGWYQQKPGKAPKRLICAASRLQSGVPSRFSGSGSGTEFTLTISSLQPEDFAIYYCLQHHDYPFTFGPGTKVDIK | QVQLEQSGAEVKKPGASVKVSCKASGYTFTSYDINWVRQATGQGLEWLGWMNPKRGNTGYAQKFQGRVTMTRNTSISTAHMELSSLRSEDTAVYYCARGRYSREDYYYYYDMDVWGQGTTVTVSS |
| | | | SEQ ID NO: 26966 | SEQ ID NO: 30972 |

FIGURE 50

| | | | | |
|---|---|---|---|---|
| iPS:436250 | 21-225_201A4 | NA | GAAATAGTGATGACGCAGTCTCCAGCCACCCTGTCTGTGTCTCCAGGGGAAAGTGCCACCCTCTCCTGCAGGTCCAGTCAGAATATTAAAAGCAACTTAGCCTGGTACCAGCAGAAACCTGGCCAGGCTCCCAGGCTCCTCATCTTTGGTGCATCCACCAGGGCCACTGGTATCCCAGCCAGGTTCAGTGGCAGTGGGTCTGGGACAGAGTTCACTCTCACCATCAGCAGCCTGCAGTCTGAAGATTTTGCAGTTTATTACTGTCAGCAGTTTTATAACTGGCTGTGCAGTTTTGGCCAGGGGACCAAGCTGGAGCTCAAA | CAGGTACAGCTGCAGCAGTCAGGTCCAGGACTGGTGAAGCCCTCGCAGACCCTCTCACTCACCTGTGCCATCTCCGGGGACAGTGTCTCTAGCAACAGTGCTGCTTGGAACTGGATCAGGCAGTCCCCATCGAGAGGCCTTGAGTGGCTGGGAAGGACATACTACAGGTCCAAGTGGTATAATTATTATGAAGTATCTGTGAAAAGTCGAATAACCATCAACCCAGACACATCCAAGAACCAGTTCTCCCTGCAGCTGAACTCTGTGACTCCCGAGGACACGGCTGTATATTTCTGTGCGAGAGATCAACGGTACTACGGTATGGACGTCTGGGGCCAAGGGACCACGGTCACCGTCTCCTCA |
| | | | SEQ ID NO: 26967 | SEQ ID NO: 30973 |
| | | AA | EIVMTQSPATLSVSPGESATLSCRSSQNIKSNLAWYQQKPGQAPRLLIFGASTRATGIPARFSGSGSGTEFTLTISSLQSEDFAVYYCQQFYNWLCSFGQGTKLELK | QVQLQQSGPGLVKPSQTLSLTCAISGDSVSSNSAAWNWIRQSPSRGLEWLGRTYYRSKWYNYYEVSVKSRITINPDTSKNQFSLQLNSVTPEDTAVYFCARDQRYYGMDVWGQGTTVTVSS |
| | | | SEQ ID NO: 26968 | SEQ ID NO: 30974 |
| iPS:436252 | 21-225_202A8 | NA | GAAATAGTGATGACGCAGTCACCAGCCACCCTGTCTGTGTCTCCAGGGGAAAGAGCCACCCTCTCCTGCAGGGCCAGTCAGAGAATTAACAACAACTTAGCCTGGTACCAGCAGAACCCTGGCCAGGCTCCCAGGCTCCTCATTTATGGTGCATCCACCAGGGCCACTGGTGTCCCGGCCAGGTTCAGTGGCAGTGGGTCTGGGACAGAGTTCACTCTCACCATCAGCAGCCTGCAGTCTGAAGACTTTACAGTTTATTACTGTCAGCAGTATTATAACTGGCTGTGCAGTTTTGGCCAGGGGACCAAGCTGGAGATCAGA | CAGGTACAGCTGCAGCAGTCAGGTCCAGGACTGGTGAAGCCCTCGCAGACCCTCTCACTCACCTGTGCCATCTCCGGGGACAGTGTCTCTAGCAACAGTGCTGCTTGGAACTGGATCAGGCAGTCCCCATCGAGAGGCCTTGAGTGGCTGGGAAGGACATACTACAGGTCCAAGTGGTATAATGAGTATGCAGTATCTGTGAGAAGTCGAATAACCATCAACCCAGACACATCCAAGAACCAGTTCTCCCTGCAGCTGAACTCTGTGACTCCCGAGGACACGGCTCTGTATTACTGTACAAGAGATCAACGGTACTACGGTATGGACGTCTGGGGCCAAGGGACCCCGGTCACCGTCTCCTCA |
| | | | SEQ ID NO: 26969 | SEQ ID NO: 30975 |

FIGURE 50

| | | | | |
|---|---|---|---|---|
| | | AA | EIVMTQSPATLSVSPGERATLSCRASQRINNNLAWYQQNPGQAPRLLIYGASTRATGVPARFSGSGSGTEFTLTISSLQSEDFTVYYCQQYYNWLCSFGQGTKLEIR | QVQLQQSGPGLVKPSQTLSLTCAISGDSVSSNSAAWNWIRQSPSRGLEWLGRTYYRSKWYNEYAVSVRSRITINPDTSKNQFSLQLNSVTPEDTALYYCTRDQRYYGMDVWGQGTPVTVSS |
| | | | SEQ ID NO: 26970 | SEQ ID NO: 30976 |
| **iPS:436254** | **21-225_202C12** | NA | GATATTGTGCTGACTCAGTCTCCACTCTCCCTGCCCGTCACCCCTGGAGAGCCGGCCTCCATCTCCTGCAGGTCTAGTCAGAGCCTCCTGCATAATAATAAATACAACCATTTGGATTGGTACCTGCAGAAGCCAGGGCAGTCTCCACAGCTCCTGATCTATTTGGGTTCTAATCGGGCCTCCGGGGTCCCTGACAGGTTCAGTGGCAGTGGATCAGGCACAGATTTTACACTGAAAATCAGCAGAGTGGAGGCTGAGGATGTTGGGGTTTATTACTGCATGCAAGCTCTACAAACTCCCACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAACTATTAGTGGTAGTGGTGTTAGAACATACTACGCAGACTCCGTGAAGGGCCGGTCCACCATCTCCAGAGACAATTCCAAGAATACGCTGTTTCTGCAAATGAACAGCCTGAGAGCCGAGGACACGGCCGTATATTACTGTGCGAAAGGGGGAGCTAGGAGCAGTGGCTGGTTCCACTTTGACTACTGGGGCCAGGGAACCCTGGTCACCGTCTCCTCA |
| | | | SEQ ID NO: 26971 | SEQ ID NO: 30977 |
| | | AA | DIVLTQSPLSLPVTPGEPASISCRSSQSLLHNNKYNHLDWYLQKPGQSPQLLIYLGSNRASGVPDRFSGSGSGTDFTLKISRVEAEDVGVYYCMQALQTPTFGGGTKVEIK | EVQLLESGGGLVQPGGSLRLSCAASGFTFSSYAMSWVRQAPGKGLEWVSTISGSGVRTYYADSVKGRSTISRDNSKNTLFLQMNSLRAEDTAVYYCAKGGARSSGWFHFDYWGQGTLVTVSS |
| | | | SEQ ID NO: 26972 | SEQ ID NO: 30978 |

FIGURE 50

| | | | | |
|---|---|---|---|---|
| **iPS:436256** | **21-225_202D9** | NA | GAAATTGTGTTGACGCAGTCTCCAGACACCCTGTCTTTGTCTCCAGGGGAAAGAGCCACCCTCTCCTGCAGGGCCAGTCAGAGTGTTAACAGCGGCTACTTAGCCTGGTACCAGCAGAAACCTGGCCAGTCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGCATTACTGTCAACAATATGAGACCTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTATGATGGGTCCTTCAGTCCTTACTACTGGAGCTGGATCCGCCAGCCCCCAGGGAAGGGGCTGGAGTGGATTGGGGAAATCAATCATAGTGGAAGCACCAACTACAATCCGTCCCTCAAGAGTCGAGTCACCATATCAGTAGACACGTCCAAGAACCAGTTCTCCCTGAAGCTGAGCTCTGTGACCGCCGCGGACACGGCTGTATATTACTGTGCGAGAGACTACGGGGGTTTAGACTACTGGGGCCAGGGAACCCTGGTCACCGTCTCCTCA |
| | | | SEQ ID NO: 26973 | SEQ ID NO: 30979 |
| | | AA | EIVLTQSPDTLSLSPGERATLSCRASQSVNSGYLAWYQQKPGQSPRLLIYGASSRATGIPDRFSGSGSGTDFTLTISRLEPEDFAVHYCQQYETSPWTFGQGTKVEIK | QVQLQQWGAGLLKPSETLSLTCAVYDGSFSPYYWSWIRQPPGKGLEWIGEINHSGSTNYNPSLKSRVTISVDTSKNQFSLKLSSVTAADTAVYYCARDYGGLDYWGQGTLVTVSS |
| | | | SEQ ID NO: 26974 | SEQ ID NO: 30980 |
| **iPS:436258** | **21-225_202F12** | NA | GAAATAGTGATGACGCAGTCTCCAGCCACCCTGTCTGTGTCTCCAGGGGAAAGAGCCACCCTCTCCTGCAGGGCCAGTCAGAGTGTTCTGAACAACTTAGCCTGGTACCAGCAGAGACCTGGCCAGGCTCCCAGGCTCCTCATCTATGGTGCATCCACTAGGGCCACTGGTATCCCAGCCAGGTTCAGTGGCAGTGGGTCTGGGACAGAGTTCACTCTCACCATCAGCAGCCTGCAGTCTGAAGATTTTGCAGTTTATTACTGTCAGCAGTATGATAACTGGCCTCCGTGCAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTGAAGCGTCTGGATTCACCTTCAGTTACTATGGCATGCACTGGGTCCGCCAGGCTCCAGGCAAGGGACTGGAGTGGGTGGCAATTATATGGTATGATGGAAGTAATAAATTCTATGCAGACTCCGTGAAGGGCCGATTCACCATCTCCAGAGACAGTTCCAAGAACACGCTGTATCTGCAAATGAACAGCCTGAGAGCCGAGGACACGGCTGTGTATTACTGTGCGAGTAATATAGCAGCAGCTGCCCCTTACTTTGACTACTGGGGCCAGGGAACCCTGGTCACCGTCTCCTCA |
| | | | SEQ ID NO: 26975 | SEQ ID NO: 30981 |

FIGURE 50

| | | | | |
|---|---|---|---|---|
| | | AA | EIVMTQSPATLSVSPGERATLSCRASQSVLNNLAWYQQRPGQAPRLLIYGASTRATGIPARFSGSGSGTEFTLTISSLQSEDFAVYYCQQYDNWPPCSFGQGTKLEIK | QVQLVESGGGVVQPGRSLRLSCEASGFTFSYYGMHWVRQAPGKGLEWVAIIWYDGSNKFYADSVKGRFTISRDSSKNTLYLQMNSLRAEDTAVYYCASNIAAAAPYFDYWGQGTLVTVSS |
| | | | SEQ ID NO: 26976 | SEQ ID NO: 30982 |
| iPS:436260 | 21-225_203H1 | NA | GACATCCAGATGACCCAGTCTCCATCCTCACTGTCTGCATCTGTCGGAGACAGAGTCACCATCACTTGTCGGGCGAGTCAGGGCATTGGCAATTATTTAGCCTGGTTTCAGCAGAAACCAGGGAAAGCCCCTAAGTCCCTGATCTATGTTGCATCCAGGTTGCAAAGTGGGGTCCCATCAAAGTTCAGCGGCACTGGATCTGGGTCAGATTTCACTCTCACCATCAGCAGCCTGCAGCCTGACGATTTTGCGACTTATTACTGCCAACGGTATCATACTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATAGAACAGTTGGCTACAACGGTATGGACGTCTGGGGCCAAGGGACCACGGTCACCGTCTCCTCA |
| | | | SEQ ID NO: 26977 | SEQ ID NO: 30983 |
| | | AA | DIQMTQSPSSLSASVGDRVTITCRASQGIGNYLAWFQQKPGKAPKSLIYVASRLQSGVPSKFSGTGSGSDFTLTISSLQPDDFATYYCQRYHTYPLTFGGGTKVEIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAVYYCARDRTVGYNGMDVWGQGTTVTVSS |
| | | | SEQ ID NO: 26978 | SEQ ID NO: 30984 |

FIGURE 50

| | | | | |
|---|---|---|---|---|
| **iPS:436262** | **21-225_203E3** | NA | GACATCCAGATGACCCAGTCTCCATCCTCCCCGTCTGCATCTGTTGGAGACAGAGTCACCATCACTCGCCGGGCAAGTCACAACATTAACAGCTATTTAAATTGGTATCAGCAGAAATCAGGGAAAGCCCCTAAACTCCTTATCTATGCTGCATCCAGTTTGCAAAGTGGGGTCCCGTCAAGGTTCAGTGGCAGTGGATCTGGGACAGATTTCACTCTCACTTTCAGTAGTCTACAACCTGAAGATTTTGCAACTTACTACTGTCAACAGAGTTACAGTTTCCCGCTCACTTTCGGCGGAGGGACCAAGGTGGAGATGAGG | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATCCACTGGGTGCGACAGGCCCCTGGACAAGGGCTTGAGTGGATGGCATGGATCAACCCTAATAGTGGTGGCACAAACTATGCACAGAAGTTTCAGGGCAGGGTCACCATGACCAGGGACACGTCCATCACCACAGCCTACATGGAGCTGAGCAGGCTGAGATCTGACGACACGGCCGTGTATTACTGTGCGAGAGGATACAGCTATGGTTACAACTGGTTCGACCCCTGGGGCCAGGGAACCCTGGTCACCGTCTCCTCA |
| | | | SEQ ID NO: 26979 | SEQ ID NO: 30985 |
| | | AA | DIQMTQSPSSPSASVGDRVTITRRASHNINSYLNWYQQKSGKAPKLLIYAASSLQSGVPSRFSGSGSGTDFTLTFSSLQPEDFATYYCQQSYSFPLTFGGGTKVEMR | QVQLVQSGAEVKKPGASVKVSCKASGYTFTGYYIHWVRQAPGQGLEWMAWINPNSGGTNYAQKFQGRVTMTRDTSITTAYMELSRLRSDDTAVYYCARGYSYGYNWFDPWGQGTLVTVSS |
| | | | SEQ ID NO: 26980 | SEQ ID NO: 30986 |
| **iPS:436264** | **21-225_203F7** | NA | GACATCCAGATGACCCAGTCTCCATCCTCCCGTTTTGCATCTGTAGGAGACAGAGTCACCATCACTTGCCGGGCAAGTCAGGGCATTAGACATGATTTAGGCTGGTATCAGCAGAAACCAGGGAAAGCCCTTAAGCGCTTGATATATGCTGCATCCAGTTTGCAAAGTGGGGTCCCATCAAGGTTCAGCGGCAGTGGATGTGGGACAGAATTCACTCTCACAATCAGCAGCGTGCAGCCTGAAGATTTTGCAAATTATTACTGTCTACAGCATTATAGTTTCCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGTCATGCACTGGGTCCGCCAGGCTCCAGGCAAGGGGCTGGAGTGGGTGGCAGTTATATGGTATGATGGAAGTAATAAATACTATGTAGACTCCGTGAAGGGCCGATTCACCATCTCCAGAGACAATTCCAGGAACACGCTGTATCTGCAAATGAACAGCCTGAGAGCCGAGGACACGGCTGTGTATTACTGTGCGAGAGAACGGTATAGCAGTGGCTTGTACGACTACGGTATGGACGTCTGGGGCCAAGGGACCACGGTCACCGTCTCCTCA |
| | | | SEQ ID NO: 26981 | SEQ ID NO: 30987 |

FIGURE 50

| | | | | |
|---|---|---|---|---|
| | | AA | DIQMTQSPSSRFASVGDRVTITCRASQGIRHDLGWYQQKPGKALKRLIYAASSLQSGVPSRFSGSGCGTEFTLTISSVQPEDFANYYCLQHYSFPRTFGQGTKVEIK | QVQLVESGGGVVQPGRSLRLSCAASGFTFSDYVMHWVRQAPGKGLEWVAVIWYDGSNKYYVDSVKGRFTISRDNSRNTLYLQMNSLRAEDTAVYYCARERYSSGLYDYGMDVWGQGTTVTVSS |
| | | | SEQ ID NO: 26982 | SEQ ID NO: 30988 |
| **iPS:436268** | **21-225_203B9** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATAGGGCATCCAGTGTGCAAAATGGGGTCCCATCAAGGTTCAGCGGCAGTGGATCTGGGACAGAATTCACTCTCACAATCAGCAGCCTGCAGCCTGAAGATTTTGCAACTTATCACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCAGGATTCACCCTCAGTAGTTTTGGCATGCACTGGGTCCGCCAGGCTCCAGGCAAGGGGCTGGAGTGGGTGGCAGTTATATGGTATGATGTAAATAATAAATACTATGCAGACTCCGTGAAGGGCCGATTCACAATCTCCAGAGACAATTCCAAGAACACGCTGTATCTCCAAATGAACAGCCTGAGACCCGAGGACACGGCTGTGTATTACTGTGCGAGAGAACTGGGGTTCCTCTCTGACTACTGGGGCCAGGGAATCCTGGTCACCGTCTCCTCA |
| | | | SEQ ID NO: 26983 | SEQ ID NO: 30989 |
| | | AA | DIQMTQSPSSLSASVGDRVTITCRASQGIRNDLGWYQQKPGKAPKRLIYRASSVQNGVPSRFSGSGSGTEFTLTISSLQPEDFATYHCLQHNSYPFTFGPGTKVDIK | QVQLVESGGGVVQPGRSLRLSCAASGFTLSSFGMHWVRQAPGKGLEWVAVIWYDVNNKYYADSVKGRFTISRDNSKNTLYLQMNSLRPEDTAVYYCARELGFLSDYWGQGILVTVSS |
| | | | SEQ ID NO: 26984 | SEQ ID NO: 30990 |

FIGURE 50

| | | | | |
|---|---|---|---|---|
| **iPS:436270** | **21-225_203F10** | NA | GACATCGTGATGACCCAGTCTCCAGACTCCCTGGCTGTGTCTCTGGGCGAGAGGGCCACCATCAACTGCAAGTCCAGCCAGAGTGTTTTTTTCCACTCGAACAATAAGAACTACTTAGCTTGGTACCAGCAGAAACCAGGACAGCCTCCTAAGTTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGACAGTTTATTACTGTCAACAATATTTTAGTCTTCCATTCACTTTCGGCCCTGGGACCAAAGTGGATATCACA | CAGGTGCAACTGGTGGAGTCTGGGGGAGGCTTGGTCAAGCCTGGAGGGTCCCTGAGACTCTCCTGTGCAGCCTCTGGATTCACCTTCAGTGACTACTACATGAGCTGGATCCGCCAGGCTCCAGGGAAGGGGCTGGAGTGGGTTTTATACATTAGTGGTAGTGGTACTACCACATACTACGCAGACTCTGTGAAGGGCCGATTCACCATCTCCAGGGACAACGCCAAGAACTCACTGTATCTGCAAATGAACAGCCTGAGAGCCGAGGACACGGCCGTGTATTACTGTGCGAGAGATAGGGGGGGTTTGGACGTCTGGGGCCAAGGGACCACGGTCACCGTCTCCTCA |
| | | | SEQ ID NO: 26985 | SEQ ID NO: 30991 |
| | | AA | DIVMTQSPDSLAVSLGERATINCKSSQSVFFHSNNKNYLAWYQQKPGQPPKLLIYWASTRESGVPDRFSGSGSGTDFTLTISSLQAEDVTVYYCQQYFSLPFTFGPGTKVDIT | QVQLVESGGGLVKPGGSLRLSCAASGFTFSDYYMSWIRQAPGKGLEWVLYISGSGTTTYYADSVKGRFTISRDNAKNSLYLQMNSLRAEDTAVYYCARDRGGLDVWGQGTTVTVSS |
| | | | SEQ ID NO: 26986 | SEQ ID NO: 30992 |
| **iPS:436272** | **21-225_201F5** | NA | GACATCGTGATGACCCAGTCTCCAGAGTCCCTGGCTGTGTCTCTGGGCGAGAGGGCCACCATCAACTGCAAGTCCAGCCAGAGTGTTTTATACAGTTCCAACAATAAGAACTACTTAGTTTGGTACCAGCAGAAACCAGGACAGCCTCCTAAACTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCTCCGACGTTCGGCCAAGGGACCAAGGTGGAAATCAAA | CAGGTGCAGCTGGTGCAGTCTGGGGCTGCGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCCTATAGCAGTGGCTGGTACTACTTTGACTACTGGGGCCAGGGAACCCTGGTCACCGTCTCCTCA |
| | | | SEQ ID NO: 26987 | SEQ ID NO: 30993 |

FIGURE 50

| | | | | |
|---|---|---|---|---|
| | | AA | DIVMTQSPESLAVSLGERATINCKSSQSVLYSSNNKNYLVWYQQKPGQPPKLLIYWASTRESGVPDRFSGSGSGTDFTLTISSLQAEDVAVYYCQQYYSTPPTFGQGTKVEIK | QVQLVQSGAAVKKPGASVKVSCKASGYTFTNYDINWVRQATGQGLEWMGWMHPNSGNTGYAQKFQGRVTMTRNTSISTAYMELSSLRSEDTAVYYCAYSSGWYYFDYWGQGTLVTVSS |
| | | | SEQ ID NO: 26988 | SEQ ID NO: 30994 |
| iPS:436274 | 21-225_204H3 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTGAACTCCTGATCTATGCTGCAGCCAGTTTGCAAGGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTACCCTCGGACGTTCGGCCAAGGGACCAAGGTGGAACTCCAA | CAGGTGCAGCTGGTGGAGTCTGGGGGAGGCGTGGGCCAGCCTGGGAGGTCCCTGAGACTCTCCTGTACAGCGTCTGGATTCACCTTCAGTAGCTATGTCATGCACTGGGTCCGCCAGGCTCCAGGCAAGGGGCTGGAGTGGGTGGCAGTTATATGGTATGATGGAAGTAATAAATATAATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACCGTATAGCAGCAGCTGGTACGACTACGGTATGGACGTCTCGGGCCAAGGGACCACGGTCACCGTCTCCTCA |
| | | | SEQ ID NO: 26989 | SEQ ID NO: 30995 |
| | | AA | DIQMTQSPSSLSASVGDRVTITCRASQGIRNDLGWYQQKPGKAPELLIYAAASLQGGVPSRFSGSGSGTEFTLTISSLQPEDFATYYCLQHYSYPRTFGQGTKVELQ | QVQLVESGGGVGQPGRSLRLSCTASGFTFSSYVMHWVRQAPGKGLEWVAVIWYDGSNKYNADSVKGRFTISRDNSKNTLYLQMNSLRAEDTAVYYCAREPYSSSWYDYGMDVSGQGTTVTVSS |
| | | | SEQ ID NO: 26990 | SEQ ID NO: 30996 |

FIGURE 50

| | | | | |
|---|---|---|---|---|
| iPS:436276 | 21-225_204H4 | NA | GACATCCAGATGACCCTGTCTCCATCCTCCCCGTCTGCATTTGTTGGAGACAGAGTCACCATCACTCGCCGGGCAAGTCACAACATTAACAGCTATTTAAATTGGTATCAGCAGAAATCAGGGAAAGCCCCTAAACTCCTTATCTATGCTGCATCCAGTTTGCAAAGTGGGGTCCCGTCAAGGTTCAGTGGCAGTGGATCTGGGACAGATTTCACTCTCACTTTCAGTAGTCTACAACCTGAAGATTTTGCAACTTACTACTGTCAACAGAGTTACAGTTTCCCGCTCACTTTCGGCGGAGGGACCAAGGTGGAGATGAGG | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATCCACTGGGTGCGACAGGCCCCTGGACAAGGGCTTGAGTGGATGGCATGGATCAACCCTAATAGTGGTGGCACAAACTATGCACAGAAGTTTCAGGGCAGGGTCACCATGACCAGGGACACGTCCATCACCACAGCCTACATGGAGCTGAGCAGGCTGAGATCTGACGACACGGCCGTGTATTACTGTGCGAGAGGATACAGCTATGGTTACAACTGGTTCGACCCCTGGGGCCAGGGAACCCTGGTCACCGTCTCCTCA |
| | | | SEQ ID NO: 26991 | SEQ ID NO: 30997 |
| | | AA | DIQMTLSPSSPSAFVGDRVTITRRASHNINSYLNWYQQKSGKAPKLLIYAASSLQSGVPSRFSGSGSGTDFTLTFSSLQPEDFATYYCQQSYSFPLTFGGGTKVEMR | QVQLVQSGAEVKKPGASVKVSCKASGYTFTGYYIHWVRQAPGQGLEWMAWINPNSGGTNYAQKFQGRVTMTRDTSITTAYMELSRLRSDDTAVYYCARGYSYGYNWFDPWGQGTLVTVSS |
| | | | SEQ ID NO: 26992 | SEQ ID NO: 30998 |
| iPS:436278 | 21-225_201F2 | NA | GAGATAGTGATGACGCAGTCTCCAGCCACCCTGTCTGTGTCTCCAGGGGAAAGAGCCACCCTCTCCTGCAGGGCCAGTCAGAATATTAAAAGCAACTTAGCCTGGTACCAGCAGAAACCTGGCCAGGCTCCCAGGCTCCTCATCTTTGGTGCATCCACCAGGGCCACTGGTATCCCAGCCAGGTTCAGTGGCAGTGGGTCTGGTACAGAGTTCACTCTCACCATCAGCAGCCTGCAGTCTGAAGATTTTGCAGTGTATTACTGTCAGCAGTTTTATAACTGGCTGTGCAGTTTTGGCCAGGGGACCAAGCTGGAGCTCAAG | CAGGTACAGCTGCAGCAGTCAGGTCCAGGACTGGTGAAGCCCTCGCAGACCCTCTCACTCACCTGTGCCATCTCCGGGGACAGTGTCTCTAGCAACAGTGCTGCTTGGAACTGGATCAGGCAGTCCCCATCGAGAGGCCTTGAGTGGCTGGGAAGGACATACTACAGGTCCAAGTGGTATAATTATTATGAAGTATCTGTGAGAAGTCGAGTAACCATCAACCCAGACACATCCAAGAACCAGTTCTCCCTGCAACTGAACTCTGTGACTCCCGAGGACACGGCTGTGTATTTCTGTGCGAGAGATCAACGGTACTACGGTATGGACGTCTGGGGCCAAGGGACCACGGTCACCGTCTCCTCA |
| | | | SEQ ID NO: 26993 | SEQ ID NO: 30999 |

FIGURE 50

| | | | | |
|---|---|---|---|---|
| | | AA | EIVMTQSPATLSVSPGERATLSCRASQNIKSNLAWYQQKPGQAPRLLIFGASTRATGIPARFSGSGSGTEFTLTISSLQSEDFAVYYCQQFYNWLCSFGQGTKLELK | QVQLQQSGPGLVKPSQTLSLTCAISGDSVSSNSAAWNWIRQSPSRGLEWLGRTYYRSKWYNYYEVSVRSRVTINPDTSKNQFSLQLNSVTPEDTAVYFCARDQRYYGMDVWGQGTTVTVSS |
| | | | SEQ ID NO: 26994 | SEQ ID NO: 31000 |
| iPS:436280 | 21-225_204D6 | NA | GACAGCCAGATGACCCAGTCTCCATCCTCCCTGTCTGCATCTGTAGGAGACAGAGTCACCATCACTTGCCGGGCAAGTCGGAGCGTTCACACCTATTTAAATTGGTATCAACAGAAGCCAGGGAAAGCCCCTAAGGTCCTGATCTATGGTGCATCCAGTTTGCAACGTGGGGTCCCATCAAGGTTCAGTGGCAGTGGATCTGGGACAGATTTCACTCTCACCATCAGCAGTCTGCAACCTGAAGATGTTGCAACTTACTACTGTCAACAGAGTTACAGTTCCCCGCTCACTTTCGGCGGGGGGACCAAGGTGGAGATCCAA | GAGGTGCAGGTGTTGGAGTCTGGGGGAGGCTTGGTGCAGCCTGGGGGGTCCCTGCGACTCTCCTGTGCAGCCTCTGGATTCACCTTTAGCACCTATGCCATGAGCTGGGTCCGCCAGGCTCCAGGGAAGGGGCTGGAGTGGGTCTCAGCTATTAGTGGTAGTGGTGGTAGCACATATTACGCAGACTCCGTGAAGGGCCGGTTCACCATCTCCAGAGACAATTCCAAGAACACGCTGTATCTGCAAATGGACAGCCTGAGAGCCGAGGACACGGCCGTATATTACTGTGCGAAAGGGATAAGTGGAACCGGCTCCTACTACTACTACGGTGTGGACGTCTGGGGCCAAGGGACCACGGTCACCGTCTCCTCA |
| | | | SEQ ID NO: 26995 | SEQ ID NO: 31001 |
| | | AA | DSQMTQSPSSLSASVGDRVTITCRASRSVHTYLNWYQQKPGKAPKVLIYGASSLQRGVPSRFSGSGSGTDFTLTISSLQPEDVATYYCQQSYSSPLTFGGGTKVEIQ | EVQVLESGGGLVQPGGSLRLSCAASGFTFSTYAMSWVRQAPGKGLEWVSAISGSGGSTYYADSVKGRFTISRDNSKNTLYLQMDSLRAEDTAVYYCAKGISGTGSYYYYGVDVWGQGTTVTVSS |
| | | | SEQ ID NO: 26996 | SEQ ID NO: 31002 |

FIGURE 50

| | | | | |
|---|---|---|---|---|
| **iPS:436282** | **21-225_204G6** | NA | GACATCCGGATGACCCAGTCTCCATCCTCACTGTCTGCATCTGTAGGAGACAGAATCACCATCACTTGTCGGACGAGTCAGGGCATTGGCAATTATTTAGCCTGGTTTCAGCAGAAACCAGGGAAAGCCCCTAAGTCCCTGATCTATGCTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTATTACTGCCAACACTATCTTAGTTACCCTCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTACAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGAGGTGTCGGCTACGACGGTATGGACGTCTGGGGCCAAGGGACCACGGTCACCGTCTCCTCA |
| | | | SEQ ID NO: 26997 | SEQ ID NO: 31003 |
| | | AA | DIRMTQSPSSLSASVGDRITITCRTSQGIGNYLAWFQQKPGKAPKSLIYAASSLQSGVPSRFSGSGSGTDFTLTISSLQPEDFATYYCQHYLSYPLTFGGGTKVEIK | QVQLVESGGGVVQPGRSLRLSCTASGFTFSSYGMHWVRQAPGKGLEWVAVIWYDGSNKNYADSVKGRFTISRDNSKNTLYLQMNSLRAEDTAVYYCARDRGVGYDGMDVWGQGTTVTVSS |
| | | | SEQ ID NO: 26998 | SEQ ID NO: 31004 |
| **iPS:436284** | **21-225_204G8** | NA | GACATCCAGATGACCCAGTCTCCATCCTCACTGTCTGCATCTGTAGGAGACAGAGTCACCATCACTTGTCGGGCGAGTCAGGGCATAAGTAATCATTTAGCCTGGTTTCAGCAGAAACCAGGGAAAGCCCCTAAGTCCCTGATCTTTGCTGCATCCAGTTTGCAAAGTGGGGTCCCATCACAGTTCAGCGGCAGTGGATCTGGGACCGAATTCACTCTCACCATCAGCAGCCTGCAGCCTGAAGATTTTGCAACTTATTACTGCCAACAGTATAGTAATTACCCGGTCACTTTCGGCGGAGGGACCAAGGTGGAGATCAAA | CAGGTGCAGCTGGTGGAGTCTGGGGGAGACGTGGTCCAGCCTGGGAGGTCCCTGAGACTCTCATGTGCAGCGTCTGGATTCACCTTCAGTAGCTATGGCATGCACTGGGCCGCCAGGCTCCAGGCAAGGGGCTGGAGTGGGTGGCAGTTATATGGTATGATGGTAGCAATGAAAATTATGTAGCCTCCGTGAAGGGCCGATTCACCATCTTCAGAGACAATTCCAAGAACACGCTGTATCTGCAAATGAACAGCCTGAGAGCCGAGGACACGGCTGTGTATTACTGTGCGAGAGATCTGGGGATAGGGTATTACGGTATGGACGTCTGGGGCCAAGGGACCACGGTCACCGTCTCCTCA |
| | | | SEQ ID NO: 26999 | SEQ ID NO: 31005 |

FIGURE 50

| | | | | |
|---|---|---|---|---|
| | | AA | DIQMTQSPSSLSASVGDRVTITCRASQGISNHLAWFQQKPGKAPKSLIFAASSLQSGVPSQFSGSGSGTEFTLTISSLQPEDFATYYCQQYSNYPVTFGGGTKVEIK | QVQLVESGGDVVQPGRSLRLSCAASGFTFSSYGMHWGRQAPGKGLEWVAVIWYDGSNENYVASVKGRFTIFRDNSKNTLYLQMNSLRAEDTAVYYCARDLGIGYYGMDVWGQGTTVTVSS |
| | | | SEQ ID NO: 27000 | SEQ ID NO: 31006 |
| iPS:436286 | 21-225_204H8 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATTCTGCATCCAGTTTGCAAAGTGGGGTCCCATCAAGGTTCAGCGGCCGTGGATCTGGGACAGAATTCACTCTCACAGTCAGCAGCCTGCAGCCTGAAGATTTTGCAACTTATTACTGTCTACAACATAATAGTTACCCTCTCACTTTCGGCGGAGGGACCAAGGTGGAGATCAAA | CAGGTGCAGCTACAGCAGTGGGGCGCAGGACTGTTGAAGCCTTCGGAGACCCTGTTCCTCACCTGCGCTGTCTATGGTGGGTCCTTCAGTGGTTACTTCTGGACCTGGGTCCGCCAGCCCCCAGGGAAGGGACTGGAGTGGATTGGGGAAATCAGTCATAGTGGAAGCACCAGTTACAACCCGTCCCTCAAGAGTCGAGTCACCATATCAGTAGACAAGTCCAAGAACCAGTTCTCCCTGAAGCTGAGCTCTGTGACCGCCGCGGACACGGCTGTGTATTACTGTGCGAGGGACTACGGGGCCGACTACTGGGGCCAGGGAACCCTGGTCACCGTCTCCTCA |
| | | | SEQ ID NO: 27001 | SEQ ID NO: 31007 |
| | | AA | DIQMTQSPSSLSASVGDRVTITCRASQGIRNDLGWYQQKPGKAPKRLIYSASSLQSGVPSRFSGRGSGTEFTLTVSSLQPEDFATYYCLQHNSYPLTFGGGTKVEIK | QVQLQQWGAGLLKPSETLFLTCAVYGGSFSGYFWTWVRQPPGKGLEWIGEISHSGSTSYNPSLKSRVTISVDKSKNQFSLKLSSVTAADTAVYYCARDYGADYWGQGTLVTVSS |
| | | | SEQ ID NO: 27002 | SEQ ID NO: 31008 |

FIGURE 50

| | | | | |
|---|---|---|---|---|
| **iPS:436290** | **21-225_205G3** | NA | GAAATCGTGTTGACGCAGTCTCCAGGCACCCTGTCTTTGTCTCCAGGGGAAAGAGCCACCCTCTCCTGCAGGGCCAGTCAGAATGTTAGTTACAGCTACTTAGCCTGGTACCAGCAGAAACCTGGCCAGGCTCCCAGGCTCCTCATCTATGGTGCATCCAGGAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGGTAGCTCACCGTGCAGTTTTGGCCAGGGGACCAAGCTGGAGATCAAA | CAGGTGCAGCTACAGCAGTGGGGCGCAGGACTGTTGAAGCCTTCGGAGACCCTGTCCCTCACCTGCGCTGTCTTTGGTGGGTCCTTCAGTGGTCACTACTGGAGCTGGATCCGCCAGCCCCCAGGGAAGGGGCTGGAGTGGATTGGGGAAATGTATCATTTTGGAAACACCAACTACAACCCGTCCCTCAAGAGTCGAGTCACCATGTCAGTAGACACGTCCAAGAAACAGTTCTCCCTGAAGCTGAGCTCTGTGACCGCCGCGGACACGGCTGTGTATTACTGTGCGAGAGTGGGGCAGTGGCTGGCTTTTGATATCTGGGGCCAAGGGACAATGGTCACCGTCTCTTCA |
| | | | SEQ ID NO: 27003 | SEQ ID NO: 31009 |
| | | AA | EIVLTQSPGTLSLSPGERATLSCRASQNVSYSYLAWYQQKPGQAPRLLIYGASRRATGIPDRFSGSGSGTDFTLTISRLEPEDFAVYYCQQYGSSPCSFGQGTKLEIK | QVQLQQWGAGLLKPSETLSLTCAVFGGSFSGHYWSWIRQPPGKGLEWIGEMYHFGNTNYNPSLKSRVTMSVDTSKKQFSLKLSSVTAADTAVYYCARVGQWLAFDIWGQGTMVTVSS |
| | | | SEQ ID NO: 27004 | SEQ ID NO: 31010 |
| **iPS:436292** | **21-225_205H3** | NA | GACATCCCGATGACCCAGTCTCCATCCTCACTGTCTGCATCTGTAGGAGACAGAGTCACCATCACTTGTCGGGCGAGTCAGGCCATTAGTAATCATTTAGCCTGGTTTCAGCTGAAACCAGGGAAAGCCCCTAAGTCCCTGATCTATGCTGCATCCAGTTTGCAAAGTGGGGTCCCATCAAAGTTCAGCGGCAGTGGATCTGGGTCAGATTTCACTCTCACCATCAGCAGCCTGCAGCCTGAAGATTTTGCAACTTATTACTGCCAACAATATAGTAATTACCCACTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTATATTACTGTGCGAGAGATAGATCAGTTGGCTACGACGGTACGGACGTCTGGGGCCAAGGGACCACGGTCACCGTCTCCTCA |
| | | | SEQ ID NO: 27005 | SEQ ID NO: 31011 |

FIGURE 50

| | | | | |
|---|---|---|---|---|
| | | AA | DIPMTQSPSSLSASVGDRVTITCRASQAISNHLA<br>WFQLKPGKAPKSLIYAASSLQSGVPSKFSGSGSG<br>SDFTLTISSLQPEDFATYYCQQYSNYPLTFGGGT<br>KVEIK | QVQLVESGGGVVQPGRSLRLSCAASGFTFSSYGMH<br>WVRQAPGKGLEWVAVIWYDGSNKYYADSVKGRF<br>TISRDNSKNTLYLQMNSLRAEDTAVYYCARDRSVG<br>YDGTDVWGQGTTVTVSS |
| | | | SEQ ID NO: 27006 | SEQ ID NO: 31012 |
| **iPS:436294** | **21-225_205G4** | NA | GAAATAGTGATGACGCAGTCTCCAGCCACCCT<br>GTCTGTGTCTCCAGGGGAAAGAGCCACCCTCT<br>CCTGCAGGGCCAGTCAGAATATTAAAAGCAAC<br>TTAGCCTGGTACCAGCAGAAACCTGGCCAGGC<br>TCCCAGGCTCCTCATCTTTGGTGCATCCACCAG<br>GGCCACTGGTATCCCAGCCAGGTTCAGTGGCA<br>GTGGGTCTGGGACAGAGTTCACTCTCACCATC<br>AGCAGCCTGCAGTCTGAAGATTTTGCAGTTTA<br>TTACTGTCAGCAGTTTTATAACTGGCTGTGCA<br>GTTTTGGCCAGGGGACCAAGCTGGAGCTCAAA | CAGGTACAGCTGCAGCAGTCAGGTCCAGGACTG<br>GTGAAGCCCTCGCAGACCCTCTCACTCACCTGTG<br>CCATCTCCGGGGACAGTGTCTCTAGCAACAGTGC<br>TGCTTGGAACTGGATCAGGCAGTCCCCATCGAGA<br>GGCCTTGAGTGGCTGGGAAGGACATATTACAGGT<br>CCAAGTGGTATAATTATTATGAAGTATCTGTGAG<br>AAGTCGAATAACCATCAACCCAGACACATCCAA<br>GAACCAGTTCTCCCTGCAGCTGAATTCTGTGACT<br>CCCGAGGACACGGCTGTGTATTTCTGTGCGAGAG<br>ATCAACGGTACTACGGTATGGACGTCTGGGGCCA<br>AGGGACCACGGTCACCGTCTCCTCA |
| | | | SEQ ID NO: 27007 | SEQ ID NO: 31013 |
| | | AA | EIVMTQSPATLSVSPGERATLSCRASQNIKSNLA<br>WYQQKPGQAPRLLIFGASTRATGIPARFSGSGSG<br>TEFTLTISSLQSEDFAVYYCQQFYNWLCSFGQGT<br>KLELK | QVQLQQSGPGLVKPSQTLSLTCAISGDSVSSNSAA<br>WNWIRQSPSRGLEWLGRTYYRSKWYNYYEVSVRS<br>RITINPDTSKNQFSLQLNSVTPEDTAVYFCARDQRY<br>YGMDVWGQGTTVTVSS |
| | | | SEQ ID NO: 27008 | SEQ ID NO: 31014 |

FIGURE 50

| | | | | |
|---|---|---|---|---|
| **iPS:436296** | **21-225_205F5** | NA | GACATCCAGATGACCCAGTCTCCATCCTCACTGTCTGCATCTGTAGGAGACAGAGTCACCATCACTTGTCGGGCGAGTCAGGGCATTGGCAATTATTTAGCCTGGTTTCAGCAGAAACCAGGGAAAGCCCCTAAGTCCCTGATCTATGGTGTCTCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATACCTGCCAACAATATAGTAATTACCCTCTCACTTTCGGCGGAGGGACCAAGGTGGACATCAGA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGATATGGCATGCACTGGGTCCGCCAGGCTCCAGGCAAGGGACTGGAGTGGGTGGCAGTTATATGGTATGATGGAAGTAATGAGAATTATGTAGACTCCGTGAAGGGCCGATTCACCATCTCCAGAGACACTTCCAAGAAAATGCTGTTTCTGCAAATGAACAGCCTGAGAACCGATGACACGGCTGTGTATTACTGTGCGAGAGATATGGGGATAGGGTATTATGGTATGGACGTCTGGGGCCAAGGGACCACGGTCACCGTCTCCTCA |
| | | | SEQ ID NO: 27009 | SEQ ID NO: 31015 |
| | | AA | DIQMTQSPSSLSASVGDRVTITCRASQGIGNYLAWFQQKPGKAPKSLIYGVSSLQSGVPSKFSGSGSGTDFTLTISSLQPEDFATYTCQQYSNYPLTFGGGTKVDIR | QVQLVESGGGVVQPGRSLRLSCAASGFTFSRYGMHWVRQAPGKGLEWVAVIWYDGSNENYVDSVKGRFTISRDTSKKMLFLQMNSLRTDDTAVYYCARDMGIGYYGMDVWGQGTTVTVSS |
| | | | SEQ ID NO: 27010 | SEQ ID NO: 31016 |
| **iPS:436302** | **21-225_205G7** | NA | GAAATTGTGTTGACGCAGTCTCCAGGCACCCTGTCTTTGTCTCCAGGGGAAAGAGCCACCCTCTCCTGCAGGGCCAGTCAGAGTGTTTTCAGCAACTACTTAGCCTGGTACCAGCAGAAACCTGGCCAGGCTCCCAGGCTCCTCATCTATGGTGCATCCAGCAGGGCCGCTGGCATCCCCGACAGGTTCAGTGGCAGTGGGTCTGGGACAGACTTCACTCTCACCATCAGCAGACTGGAGCCTGAAAATTTTGCAGTGTATTACTGTCAGCAGTATGAAAGTTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGTTTATTACTGGAGCTGGATCCGCCAGCCCCCAGGGAAGGGGCTGGAGTGGATTGGGGAAAGCAATCAGAGTGGACGCACCACCTACAACCCGTCCCTCAAGAGTCGAGTCACCATATCAGTAGACACGTCCAAGAACCAGTTCTCCCTGAATCTGATCTCTGTGACCGCCGCGGACACGGCTGTGTATTACTGTGCGAGGGACTACGGTGTCTTTGACTACTGGGGCCAGGGAACCCTGGTCACCGTCTCCTCA |
| | | | SEQ ID NO: 27011 | SEQ ID NO: 31017 |

FIGURE 50

| | | | | |
|---|---|---|---|---|
| | | AA | EIVLTQSPGTLSLSPGERATLSCRASQSVFSNYLAWYQQKPGQAPRLLIYGASSRAAGIPDRFSGSGSGTDFTLTISRLEPENFAVYYCQQYESSPWTFGQGTKVEIK | QVQLQQWGAGLLKPSETLSLTCAVYGGSFSVYYWSWIRQPPGKGLEWIGESNQSGRTTYNPSLKSRVTISVDTSKNQFSLNLISVTAADTAVYYCARDYGVFDYWGQGTLVTVSS |
| | | | SEQ ID NO: 27012 | SEQ ID NO: 31018 |
| **iPS:436304** | **21-225_201F3** | NA | GATATTGTGATGACTCAGTCTCCACTCTCCCTGCCCGTCACCCCTGGAGAGCCGGCCTCCATCTCCTGCAGGTCTAGTCAGAGCCTCCTGCATAATAATAGATACAACCATTTGGATTGGTACCTGCAGAAGCCAGGGCAGTCTCCACAGCTCCTGATCTATTTGGGTTCTAATCGGGCCTCCGGGGTCCCTGACAGGTTCAGTGGCAGTGGATCAGGCACAGATTTTACACTGAAAATCAGCAGAGTGGAGGCTGAGGATGTTGGGGTTTATTACTGCATGCAAGCTCTACAAACTCCCACTTTCGGCGGAGGGACCAAGGTGGAGATCAAA | GAGGTGCAACTGTTGGAGTCTGGGGGAGGCTTGGTACAGCCTGGGGGGTCCCAGAGACTCTCCTGTGCAGCCTCTGGATTCACCTTTAGCAGCTATGCCATGAGCTGGGTCCGCCAGGCTCCAGGGAAGGGGCTGGAGTGGGTCTCAACTATTAGTGGTAGTGGTGTTAGAACATACTACGCAGACTCCGTGAAGGGCCGGTTCACCATCTCCAGAGACAATTCCAATAACACGCTGTTTCTGCAAATGAACAGCCTGAGAGCCGAGGACACGGCCGTATATTACTGTGCGAAAGGGGGAGCTAGGAGCAGTGGCTGGTTCCACTTTGACTACTGGGGCCAGGGAACCCTGGTCACCGTCTCCTCA |
| | | | SEQ ID NO: 27013 | SEQ ID NO: 31019 |
| | | AA | DIVMTQSPLSLPVTPGEPASISCRSSQSLLHNNRYNHLDWYLQKPGQSPQLLIYLGSNRASGVPDRFSGSGSGTDFTLKISRVEAEDVGVYYCMQALQTPTFGGGTKVEIK | EVQLLESGGGLVQPGGSQRLSCAASGFTFSSYAMSWVRQAPGKGLEWVSTISGSGVRTYYADSVKGRFTISRDNSNNTLFLQMNSLRAEDTAVYYCAKGGARSSGWFHFDYWGQGTLVTVSS |
| | | | SEQ ID NO: 27014 | SEQ ID NO: 31020 |

FIGURE 50

| | | | | |
|---|---|---|---|---|
| **iPS:436306** | **21-225_201H4** | NA | GAAATAGTGATGACGCAGTCTCCAGCCACCCTGTCTGTGTCTCCAGGGGAAAGAGCCACCCTCTCCTGCAGGGCCAGTCAGAGTGTTAATAGCTACTTAGCCTGGTACCAGCAGAAACCTGGCCAGGCTCCCAGGCTCCTCATCTATGGTGCATCCACCAGGGCCACTGGTATCCCAGCCAGGTTCAGTGGCAGTGGGTCTGGGACAGAATTCACTCTCACCATCAGCAGCCTGCAGTCTGAAGATTTTGCAGTTTATTACTGTCAAGAGTATAATGACTGGCCGTGCAGTTTTGGCCAGGGGACCAACCTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGCCATGCACTGGGTCCGCCAGGCTCCAGGCAAGGGGCTGGAGTGGGTGGCAGCTATATGGTATGATGGAAGTAATAAATACAATGCAGACTCCGTGAAGGGCCGATTCACCATCTCCAGAGACAATTCCAAGAACACGCTGTATATGCAAATGAACAGCCTGAGAGCCGAGGACACGGCTGTATACTACTGTGCGAGAGATGTGGGTACAGTGGGAGCTACCTACTTTGACTGCTGGGGCCCGGGAACCCTGGTCACCGTCTCCTCA |
| | | | SEQ ID NO: 27015 | SEQ ID NO: 31021 |
| | | AA | EIVMTQSPATLSVSPGERATLSCRASQSVNSYLAWYQQKPGQAPRLLIYGASTRATGIPARFSGSGSGTEFTLTISSLQSEDFAVYYCQEYNDWPCSFGQGTNLEIK | QVQLVESGGGVVQPGRSLRLSCAASGFTFSSYAMHWVRQAPGKGLEWVAAIWYDGSNKYNADSVKGRFTISRDNSKNTLYMQMNSLRAEDTAVYYCARDVGTVGATYFDCWGPGTLVTVSS |
| | | | SEQ ID NO: 27016 | SEQ ID NO: 31022 |
| **iPS:436308** | **21-225_205H8** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACAAGGGAAAGCCCCTAAGCTCCTGATCTATTCTGCATCCTTTTTGCAAAGAGGGGTCCCATCAAGATTCAGCGGCAGTGGATCTGGGACAGAATTCACTCTCACAATCAGCAGCCTGCAGCCTGAAGATTCTGCAGCTTATTACTGTCTACAGCATAATAGTTACCCGCTCACTTTCGGCGGAGGGACCACGGTGAAGATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGGTTACTTCTGGAGCTGGATCCGCCAGCCCCCAGGGAAGGGGCTGGAGTGGATTGGGGAAATCAGTCATAGTGGACGCACCAACTACAACCCGTCCCTCAAGAGCCGAGTCACCATATCAGTAGACACGTCCAAGAACCAGTTCTCCCTGAAGGTGAGCTCTGTGACCGCCGCGGACACGGCTGTGTATTACTGTGCGAGGGACTACGGGGCGGACTACTGGGGCCAGGGAACCCTGGTCACCGTCTCCTCA |
| | | | SEQ ID NO: 27017 | SEQ ID NO: 31023 |

FIGURE 50

| | | | | |
|---|---|---|---|---|
| | | AA | DIQMTQSPSSLSASVGDRVTITCRASQGIRNDLGWYQQKQGKAPKLLIYSASFLQRGVPSRFSGSGSGTEFTLTISSLQPEDSAAYYCLQHNSYPLTFGGGTTVKIK | QVQLQQWGAGLLKPSETLSLTCAVYGGSFSGYFWSWIRQPPGKGLEWIGEISHSGRTNYNPSLKSRVTISVDTSKNQFSLKVSSVTAADTAVYYCARDYGADYWGQGTLVTVSS |
| | | | SEQ ID NO: 27018 | SEQ ID NO: 31024 |
| **iPS:436310** | **21-225_202D11** | NA | GAAATTGTGTTGACGCAGTCTCCAGGCACCCTGTCTTTGTCTCCAGGGGAAAGAGCCACCCTCTCCTGCAGGGCCAGTCAGAGTATTAACAGCAACTACTTAGCCTGGTACCAGCGGAAGCCTGGCCAGGCTCCCAGGGTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTTTTACTGTCAGCAGTATGAAAACTCACCGTGGACGTTCGGCCAAGGGACCAAGGTGGAAATCAAA | CAGGTGCAGCTACAGCAGCGGGGCGCAGGACTGTTGAAGCCTTCGGAGACCCTGTCCCTCACCTGCGCTGCCTATGGTGGGTCCTTCAGTGGTCCCTACTGGAGCTGGATCCGCCAGCCCCCAGGGAAGGGCCTGGAGTGGATTGGGGAAATCAATCATAGTGGAAGCACCAACTACAACCCGTCCCTCAAGAGTCGAGTCACCATATCAGTAGACACGTCCAAGAACCAGTTCTCCCTGAAGCTGAGCTCTGTGACCGCCGCGGACACGGCTGTTTACTACTGTGCGAGGGACTACGGTGTCCTTGACTACTGGGGCCAGGGAACCCTGGTCACCGTCTCCTCA |
| | | | SEQ ID NO: 27019 | SEQ ID NO: 31025 |
| | | AA | EIVLTQSPGTLSLSPGERATLSCRASQSINSNYLAWYQRKPGQAPRVLIYGASSRATGIPDRFSGSGSGTDFTLTISRLEPEDFAVFYCQQYENSPWTFGQGTKVEIK | QVQLQQRGAGLLKPSETLSLTCAAYGGSFSGPYWSWIRQPPGKGLEWIGEINHSGSTNYNPSLKSRVTISVDTSKNQFSLKLSSVTAADTAVYYCARDYGVLDYWGQGTLVTVSS |
| | | | SEQ ID NO: 27020 | SEQ ID NO: 31026 |

FIGURE 50

| | | | | |
|---|---|---|---|---|
| **iPS:436312** | **21-225_206A4** | NA | GACATCCAGATGACCCTGTCTCCATCCTCCCCGTCTGCATTTGTTGGAGACAGAGTCACCATCACTCGCCGGGCAAGTCACAACATTAACAGCTATTTAAATTGGTATCAGCAGAAATCAGGGAAAGCCCCTAAACTCCTTATCTGTGCTGCATCCAGTTTGCAAAGTGGGGTCCCGTCAAGGTTCAGTGGCAGTGGATCTGGGACAGATTTCACTCTCACTTTCAGTAGTCTACAACCTGAAGATTTTGCAACTTACTACTGTCAACAGAGTTACAGTTTCCCGCTCACTTTCGGCGGAGGGACCAAGGTGGAGATGAGG | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATCCACTGGGTGCGACAGGCCCCTGGACAAGGGCTTGAGTGGATGGCATGGATCAACCCTAATAGTGGTGGCACAAACTATGCACAGAAGTTTCAGGGCAGGGTCACCATGACCAGGGACACGTCCATCACCACAGCCTACATGGAGCTGAGCAGGCTGAGATCTGACGACACGGCCGTGTATTACTGTGCGAGAGGATACAGCTATGGTTACAACTGGTTCGACCCCTGGGGCCAGGGAACCCTGGTCACCGTCTCCTCA |
| | | | SEQ ID NO: 27021 | SEQ ID NO: 31027 |
| | | AA | DIQMTLSPSSPSAFVGDRVTITRRASHNINSYLNWYQQKSGKAPKLLICAASSLQSGVPSRFSGSGSGTDFTLTFSSLQPEDFATYYCQQSYSFPLTFGGGTKVEMR | QVQLVQSGAEVKKPGASVKVSCKASGYTFTGYYIHWVRQAPGQGLEWMAWINPNSGGTNYAQKFQGRVTMTRDTSITTAYMELSRLRSDDTAVYYCARGYSYGYNWFDPWGQGTLVTVSS |
| | | | SEQ ID NO: 27022 | SEQ ID NO: 31028 |
| **iPS:436314** | **21-225_206G4** | NA | GAAATTGTGCTGACTCAGTCTCCAGACTTTCAGTCTGTGACTCCAAAGGAGAAAGTCACCATCACCTGCCGGGCCAGTCAGAGCATTGGTCGTAGCTTACACTGGTACCAGCAGAAACCAGATCAGTCTCCAAAACTCCTCATCAAGTATGCTTCCCAGTCCTTCTCAGGGGTCCCCTCGAGGTTCAGTGGCAGTGGATCTGGGACAGATTTCACCCTCACCATCAATAGCCTGGAAGCTGAAGATGCTGCAACGTATTACTGTCATCAGAGTAGAAGTTTACCGCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATACACTGGGTGCGACAGGCCCCTGGACAAGGGCTTGAGTGGATGGGATGGATCGACCCTAATAGTGGTGGCACAAACTATGCACAGAAGTTTCAGGGCAGAATCACCATGACCAGGGACACGTCCATCAGTACAGCCTACATGGAACTGAGCAGGCTGAGATCTGACGACACGGCCGTGTTTTACTGTGCGAAAGATCAAGGGTATAACTGGAACTCTTTTGACTACTGGGGCCAGGGAACCCTGGTCACCGTCTCCTCA |
| | | | SEQ ID NO: 27023 | SEQ ID NO: 31029 |

FIGURE 50

| | | | | |
|---|---|---|---|---|
| | | AA | EIVLTQSPDFQSVTPKEKVTITCRASQSIGRSLHWYQQKPDQSPKLLIKYASQSFSGVPSRFSGSGSGTDFTLTINSLEAEDAATYYCHQSRSLPLTFGGGTKVEIK | QVQLVQSGAEVKKPGASVKVSCKASGYTFTGYYIHWVRQAPGQGLEWMGWIDPNSGGTNYAQKFQGRITMTRDTSISTAYMELSRLRSDDTAVFYCAKDQGYNWNSFDYWGQGTLVTVSS |
| | | | SEQ ID NO: 27024 | SEQ ID NO: 31030 |
| **iPS:436316** | **21-225_206A5** | NA | GACATCCAGATGACCCAGTCTCCATCCTCCCCGTCTGCATCTGTTGGAGACAGAGTCACCATCACTCGCCGGGCAAGTCACAACATTAACAGCTATTTAAATTGGTATCAGCAGAAATCAGGGAAAGCCCCTAAACTCCTTATCTGTGCTGCATCCAGTTTGCAAAGTGGGGTCCCGTCAAGGTTCAGTGGCAGTGGATCTGGGACAGATTTCACTCTCACTTTCAGTAGTCTACAACCTGAAGATTTTGCAACTTACTACTGTCAACAGAGTTACAGTTTCCCGCTCACTTTCGGCGGAGGGACCAAGGTGGAGATGAGG | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATCCACTGGGTGCGACAGGCCCCTGGACAAGGGCTTGAGTGGATGGCATGGATCAACCCTAATAGTGGTGGCACAAACTATGCACAGAAGTTTCAGGGCAGGGTCACCATGACCAGGGACACGTCCATCACCACAGCCTACATGGAGCTGAGCAGGCTGAGATCTGACGACACGGCCGTGTATTACTGTGCGAGAGGATACAGCTATGGTTACAACTGGTTCGACCCCTGGGGCCAGGGAACCCTGGTCACCGTCTCCTCA |
| | | | SEQ ID NO: 27025 | SEQ ID NO: 31031 |
| | | AA | DIQMTQSPSSPSASVGDRVTITRRASHNINSYLNWYQQKSGKAPKLLICAASSLQSGVPSRFSGSGSGTDFTLTFSSLQPEDFATYYCQQSYSFPLTFGGGTKVEMR | QVQLVQSGAEVKKPGASVKVSCKASGYTFTGYYIHWVRQAPGQGLEWMAWINPNSGGTNYAQKFQGRVTMTRDTSITTAYMELSRLRSDDTAVYYCARGYSYGYNWFDPWGQGTLVTVSS |
| | | | SEQ ID NO: 27026 | SEQ ID NO: 31032 |

FIGURE 50

| | | | | |
|---|---|---|---|---|
| **iPS:436324** | **21-225_207G6** | NA | GATATCCAGATGACCCAGTCTCCATCCTCACTGTCTGCATCTGTAGGAGACAGAGTCACCATCACTTGTCGGGCGAGTCAGGGCATTAGAAATTATTTAGCCTGGCTTCAGCAGAAACCAGGGAAGGCCCCTAAGTCCCTGATCCATGCTGCATCCAGTTTGCAAAGTGGGGTCCCATCAAAGTTCAGCGGCAATAGATCTGGGACAGATTTCACTCTCACCATCAGCAGCCTGCAGCCTGAAGATTTTGCAACTTATTACTGCCAACAGTACAGTAATTACCCTCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGCAGAGTCCGTGAAGGGCCGATTCACCATCTCCAGAGACAATTCCAAGAACACGCTGTATCTGCAAATGAACAGCCTGAGAGCCGAGGACACGGCTGTGTATTATTGTGCGAGAGATGCGGGTATTGGATACTACGGTATAGACGTCTGGGGCCAAGGGACCACGGTCACCGTCTCCTCA |
| | | | SEQ ID NO: 27027 | SEQ ID NO: 31033 |
| | | AA | DIQMTQSPSSLSASVGDRVTITCRASQGIRNYLAWLQQKPGKAPKSLIHAASSLQSGVPSKFSGNRSGTDFTLTISSLQPEDFATYYCQQYSNYPLTFGGGTKVEIK | QVQLVESGGGVVQPGRSLRLSCAASGFTFSSYGMHWVRQAPGKGLEWVAVIWYDGSNKNYAESVKGRFTISRDNSKNTLYLQMNSLRAEDTAVYYCARDAGIGYYGIDVWGQGTTVTVSS |
| | | | SEQ ID NO: 27028 | SEQ ID NO: 31034 |
| **iPS:436328** | **21-225_207F12** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTTCTGTCTACAGCATAATAGTTACCCTCTCACCTTCGGCCAAGGGACACGACTGGAAATTAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGATAGAAATAATAAATACTATGGTGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACTGGGGTTCCTCTTTGACTACTGGGGCCAGGGAACCCTGGTCACCGTCTCCTCA |
| | | | SEQ ID NO: 27029 | SEQ ID NO: 31035 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYFCLQHNSYPLTFGQGT RLEIK | QVQLVESGGGVVQPGRSLRLSCAASGFTFSNYGM HWVRQAPGKGLEWVAVIWYDRNNKYYGDSVKG RFTISRDNSKNTLYLQMNSLRAEDTAVYYCARELG FLFDYWGQGTLVTVSS |
| | | | SEQ ID NO: 27030 | SEQ ID NO: 31036 |
| **iPS:436332** | **21-225_208B2** | NA | GACATCCAGATGACCCAGTCTCCATCCTCCCT GTCTGCATCTGTAGGAGACAGAGTCACCATCA CTTGCCGGGCAAGTCAGGGCATTAGACATGAT TTAGGCTGGTATCAACAGAAACCAGGGAAAG CCCCTAAGCGCCTGATCTATGCTGCATCCAGT TTGCAAAGTGGGGTCCCATCAGGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTGTGTCAT GCACTGGGTCCGCCAGGCTCCAGGCAAGGGGCT GGAGTGGGTGGCAGTTATATGGTATGATGGAAGT AATAAATACTATGTAGACTCCGTGAAGGGCCGAT TCACCATCTCCAGAGACAATTCCAGGAACACGCT GTATCTGCAAATGAACAGCCTGAGAGCCGAGGA CACGGCTGTGTATTACTGTGCGAGAGAACGGTAT AGCAGTGGCTTGTACGACTACGGTTTGGACGTCT GGGGCCAAGGGACCACGGTCACCGTCTCCTCA |
| | | | SEQ ID NO: 27031 | SEQ ID NO: 31037 |
| | | AA | DIQMTQSPSSLSASVGDRVTITCRASQGIRHDLG WYQQKPGKAPKRLIYAASSLQSGVPSGFSGSGS GTEFTLTISSLQPEDFATYYCLQHYSYPRTFGQG TKVEIK | QVQLVESGGGVVQPGRSLRLSCAASGFTFSDCVMH WVRQAPGKGLEWVAVIWYDGSNKYYVDSVKGRF TISRDNSRNTLYLQMNSLRAEDTAVYYCARERYSS GLYDYGLDVWGQGTTVTVSS |
| | | | SEQ ID NO: 27032 | SEQ ID NO: 31038 |

FIGURE 50

| | | | | |
|---|---|---|---|---|
| **iPS:436334** | **21-225_208G3** | NA | GATATTGTGCTGACTCAGTCTCCACTCTCCCTGCCCGTCACCCCTGGAGAGCCGGCCTCCATCTCCTGCAGGTCTAGTCAGAGCCTCCTGCATAATAATAAATACAACCATTTGGATTGGTACCTGCAGAAGCCAGGGCAGTCTCCACAGCTCCTGATCTATTTGGGTTCTAATCGGGCCTCCGGGGTCCCTGACAGGTTCAGTGGCAGTGGATCAGGCACAGATTTTACACTGAAAATCAGCAGAGTGGAGGCTGAGGATGTTGGGGTTTATTACTGCATGCAAGCTCTACAAACTCCCACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAACTATTAGTGGTAGTGGTGTTAGAACATACTACGCAGACTCCGTGAAGGGCCGGTCCACCATCTCCAGAGACAATTCCAAGAATACGCTGTTTCTGCAAATGAACAGCCTGAGAGCCGAGGACAAGGCCGTATATTACTGTGCGAAAGGGGGAGCTAGGAGCAGTGGCTGGTTCCACTTTGACTACTGGGGCCAGGGAACCCTGGTCACCGTCTCCTCA |
| | | | SEQ ID NO: 27033 | SEQ ID NO: 31039 |
| | | AA | DIVLTQSPLSLPVTPGEPASISCRSSQSLLHNNKYNHLDWYLQKPGQSPQLLIYLGSNRASGVPDRFSGSGSGTDFTLKISRVEAEDVGVYYCMQALQTPTFGGGTKVEIK | EVQLLESGGGLVQPGGSLRLSCAASGFTFSSYAMSWVRQAPGKGLEWVSTISGSGVRTYYADSVKGRSTISRDNSKNTLFLQMNSLRAEDKAVYYCAKGGARSSGWFHFDYWGQGTLVTVSS |
| | | | SEQ ID NO: 27034 | SEQ ID NO: 31040 |
| **iPS:436336** | **21-225_208B5** | NA | GAAATTGTTTTGACGCAGTCTCCAGGCACCCTGTCTTTGTCTCCAGGGGAAAGAGCCACCCTCTCCTGCAGGGCCAGTCAGAGTGTTAGCAGCAACTACTTAGCCTGGTATCAGCAGAGACCTGGCCAGGCTCCCAGACTCCTCATCTATGGTGCATCCAGCAGGGCCACTGGCATCCCAGACAGATTCAGTGGCAGTGGGTCTGGGACAGACTTCACTCTCACCATCAGCAGACTGGAGCCTGAAGATTTTGCAGTGTATTTCTGTCAGCACTACGAAAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTTTGGTGGGTCCATCAGTGTTTACTACTGGACCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATATCAGTAGACACGTCCAAGAACCAGTTCTCCCTGAAGCTGAGCTCTGTGACCGCCGCGGACACGGCTGTGTATTACTGTGCGAGGGACTATGGTGTCTTTGATTACTGGGGCCAGGGAACCCTGGTCACCGTCTCCTCA |
| | | | SEQ ID NO: 27035 | SEQ ID NO: 31041 |

FIGURE 50

| | | | | |
|---|---|---|---|---|
| | | AA | EIVLTQSPGTLSLSPGERATLSCRASQSVSSNYLAWYQQRPGQAPRLLIYGASSRATGIPDRFSGSGSGTDFTLTISRLEPEDFAVYFCQHYENSPWTFGQGTKVEIK | QVQLQQWGAGLLKPSETLSLTCAVFGGSISVYYWTWIRQPPGKGLEWIGEINHSGSTNYNPSLKSRVTISVDTSKNQFSLKLSSVTAADTAVYYCARDYGVFDYWGQGTLVTVSS |
| | | | SEQ ID NO: 27036 | SEQ ID NO: 31042 |
| iPS:436338 | 21-225_208E8 | NA | GACATCCAGATGACCCAGTCTCCATCCTCCCCGTCTGCATTTGTTGGAGACAGAGTCACCATCACTCGCCGGGCAAGTCACAACATTAACAGCTATTTAAATTGGTATCAGCAGAAATCAGGGAAAGCCCCTAAACTCCTTATCTATGCTGCATCCAGTTTGCAAAGTGGGGTCCCGTCAAGGTTCAGTGGCAGTGGATCTGGGACAGATTTCACTCTCACTTTCAGTAGTCTACAACCTGAAGATTTTGCAACTTACTACTGTCAACAGAGTTACAGTTTCCCGCTCACTTTCGGCGGAGGGACCAAGGTGGAGATGAGG | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATCCACTGGGTGCGACAGGCCCCTGGACAAGGGCTTGAGTGGATGGCATGGATCAACCCTAATAGTGGTGGCACAAACTATGCACAGAAGTTTCAGGGCAGGGTCACCATGACCAGGGACACGTCCATCACCACAGCCTACATGGAGCTGAGCAGGCTGAGATCTGACGACACGGCCGTGTATTACTGTGCGAGAGGATACAGCTATGGTTACAACTGGTTCGACCCCTGGGGCCAGGGAACCCTGGTCACCGTCTCCTCA |
| | | | SEQ ID NO: 27037 | SEQ ID NO: 31043 |
| | | AA | DIQMTQSPSSPSAFVGDRVTITRRASHNINSYLNWYQQKSGKAPKLLIYAASSLQSGVPSRFSGSGSGTDFTLTFSSLQPEDFATYYCQQSYSFPLTFGGGTKVEMR | QVQLVQSGAEVKKPGASVKVSCKASGYTFTGYYIHWVRQAPGQGLEWMAWINPNSGGTNYAQKFQGRVTMTRDTSITTAYMELSRLRSDDTAVYYCARGYSYGYNWFDPWGQGTLVTVSS |
| | | | SEQ ID NO: 27038 | SEQ ID NO: 31044 |

FIGURE 50

| | | | | |
|---|---|---|---|---|
| **iPS:436340** | **21-225_208A9** | NA | GAAATTGTGTTGACGCAGTCTCCAGGCACCCTGTCTTTGTCTCCAGGGGAAAGAGCCACCCTCTCCTGCAGGGCCAGTCAGAGTGTTAGCAACAACTACTTAGCCTGGTACCAGCAGAAACCTGGCCAGGCTCCCAGGGTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCACTATCATAGCTCACCGTGGACGTTCGGCCAAGGGACCAAGGTGGAAATCAAA | CAGGTGCAGCTACAGCAGTGGGGCGCAGGACTGTTGAAGCCTTCGGAGCCCCTGTCCCTCACCTGCGCTGTCTATGGTGGGTCCTTCAGTGTTTCCTACTGGAGCTGGATCCGCCAGCCCCCAGGGAAGGGGCTGGAGTGGATTGGGGAAATCAATCATAGTGGACGCGCCAACTACAACCCGTCCCTCAAGAGTCGAGTCACCATATCAATAGACACGTCCAAGAACCAGTTCTCCCTGAAGCTGAGCTCTGTGACCGCCGCGGACACGGCTGTGTATTACTGTGCGAGGGACTACGGTGTCCTTGACTACTGGGGCCAGGGAACCCTGGTCACCGTCTCCTCA |
| | | | SEQ ID NO: 27039 | SEQ ID NO: 31045 |
| | | AA | EIVLTQSPGTLSLSPGERATLSCRASQSVSNNYLAWYQQKPGQAPRVLIYGASSRATGIPDRFSGSGSGTDFTLTISRLEPEDFAVYYCQHYHSSPWTFGQGTKVEIK | QVQLQQWGAGLLKPSEPLSLTCAVYGGSFSVSYWSWIRQPPGKGLEWIGEINHSGRANYNPSLKSRVTISIDTSKNQFSLKLSSVTAADTAVYYCARDYGVLDYWGQGTLVTVSS |
| | | | SEQ ID NO: 27040 | SEQ ID NO: 31046 |
| **iPS:436344** | **21-225_208B11** | NA | GACATCCAGATGACCCAGTCTCCATCCTCCCCGTCTGCATCTGTTGGAGACAGAGTCACCATCACTTGCCGGGCAAGTCACAACATTAACAGCTATTTAAATTGGTATCAGCAGAAATCAGGGAAAGCCCCTAAACTCCTTATCTATGCTGCATCCAGTTTGCAAAGTGGGGTCCCGTCAAGGTTCAGTGGCAGTGGATCTGGGACAGATTTCACTCTCACTTTCAGTAGTCTACAACCTGAAGATTTTGCAACTTACTACTGTCAACAGAGTTACAGTTTCCCGCTCACTTTCGGCGGAGGGACCAAGGTGGAGATGAGG | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATCCACTGGGTGCGACAGGCCCCTGGACAAGGGCTTGAGTGGATGGCATGGATCAACCCTAATAGTGGTGGCACAAACTATGCACAGAAGTTTCAGGGCAGGGTCACCATGACCAGGGACACGTCCATCACCACAGCCTACATGGAGCTGAGCAGGCTGAGATCTGACGACACGGCCGTGTATTACTGTGCGAGAGGATACAGCTATGGTTACAACTGGTTCGACCCCTGGGGCCAGGGAACCCTGGTCACCGTCTCCTCA |
| | | | SEQ ID NO: 27041 | SEQ ID NO: 31047 |

FIGURE 50

| | | | | |
|---|---|---|---|---|
| | | AA | DIQMTQSPSSPSASVGDRVTITCRASHNINSYLNWYQQKSGKAPKLLIYAASSLQSGVPSRFSGSGSGTDFTLTFSSLQPEDFATYYCQQSYSFPLTFGGGTKVEMR | QVQLVQSGAEVKKPGASVKVSCKASGYTFTGYYIHWVRQAPGQGLEWMAWINPNSGGTNYAQKFQGRVTMTRDTSITTAYMELSRLRSDDTAVYYCARGYSYGYNWFDPWGQGTLVTVSS |
| | | | SEQ ID NO: 27042 | SEQ ID NO: 31048 |
| **iPS:436350** | **21-225_210E4** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCCTCATTAACTATGGCATGCACTGGGTCCGCCAGGCTCCAGGCAAGGGGCTGGAGTGGGTGGCAGTTATATGGTATGATGAAAATAATAAATACTATGTAGACTCCGTGAAGGGCCGATTCACCATCTCCAGAGACGATTCCAAGAACACGCTGTATCTGCAAATGAACAGCCTGAGAGCCGAGGACTCGGCTGTGTATTACTGTGCGAGAGAGACGGGTTTCTTGAGCGACTACTGGGGCCAGGGAACCCTGGTCACCGTCTCCTCA |
| | | | SEQ ID NO: 27043 | SEQ ID NO: 31049 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPFTFGPGTKVDIK | QVQLVESGGGVVQPGRSLRLSCAASGFTLINYGMHWVRQAPGKGLEWVAVIWYDENNKYYVDSVKGRFTISRDDSKNTLYLQMNSLRAEDSAVYYCARETGFLSDYWGQGTLVTVSS |
| | | | SEQ ID NO: 27044 | SEQ ID NO: 31050 |

FIGURE 50

| | | | | |
|---|---|---|---|---|
| **iPS:436352** | **21-225_210G5** | NA | GACATCCAGATGACCCAGTCTCCATCCTCCCTGTCTGCATCTGTAGGAGACAGAGTCACCATCACTTGCCGGGCAAGTCAGGGCATTAGACATGATTTAGGCTGGTATCAACAGAAACCAGGGAAAGCCCCTAAGCGCCTGATCTATGCTGCATCCAGTTTGCAAAGTGGGGTCCCATCAGGGTTCAGCGGCAGTGGATCTGGGACAGAATTCACTCTCACAATCAGCAGCCTGCAGCCTGAAGATTTTGCAACTTATTACTGTCTACACCATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTGTGTCATGCACTGGGTCCGCCAGGCTCCAGGCAAGGGGCTGGAGTGGGTGGCAGTTATATGGTATGATGGAAGTAATAAATACTATGTAGACTCCGTGAAGGGCCGATTCACCATCTCCAGAGACAATTCCAGGAACACGCTGTATCTGCAAATGAACAGCCTGAGAGCCGAGGACACGGCTGTGTATTACTGTGCGAGAGAACGGTATAGCAGTGGCTTGTACGACTACGGTTTGGACGTCTGGGGCCAAGGGACCACGGTCACCGTCTCCTCA |
| | | | SEQ ID NO: 27045 | SEQ ID NO: 31051 |
| | | AA | DIQMTQSPSSLSASVGDRVTITCRASQGIRHDLGWYQQKPGKAPKRLIYAASSLQSGVPSGFSGSGSGTEFTLTISSLQPEDFATYYCLHHYSYPRTFGQGTKVEIK | QVQLVESGGGVVQPGRSLRLSCAASGFTFSDCVMHWVRQAPGKGLEWVAVIWYDGSNKYYVDSVKGRFTISRDNSRNTLYLQMNSLRAEDTAVYYCARERYSSGLYDYGLDVWGQGTTVTVSS |
| | | | SEQ ID NO: 27046 | SEQ ID NO: 31052 |
| **iPS:436354** | **21-225_210G10** | NA | GACATCCAGATGACCCAGTCTCCATCCTCCCTGTCTGCATCTGTAGGAGACAGAGTCACCATCACTTTCCGGACAAGTCAGGGCATTAGAAATGATTTAGGCTGGTATCAGCAGCAACCAGGGAAAACCCCTAAGCGCATGATTTATGCTGCATCCAGTTTGTTTAGTGGGGTCCCATCAAGGTTCAGCGGCAGTAGATCTGGGACAGATTTCACTCTCACAATCAGCAGCCTGCAGCCTGAAGATTTTGCAACTTATTACTGTCTACAGTATAATAGTTACCCTCCCACCTTCGGCCAAGGGACACGACTGGAGATTAAA | CAGGTGCAGCTGCAGGAGTCGGGCCCAGGACTGGTGAAGCCTTCGGAGACCCTGTCCCTCAACTGCACTGTCTCTGGTGGCTCCATCAGGAGTTACTACTGGAGCTGGATCCGGCAGCCCGCCGGGAAGGGACTGGAGTGGATTGGGCGTATCTATACCAGTGGGAGCACCGACTACAACCCCTCCCTCAAGAGTCGAATCACCATGTCAGTAGACACGTCCAAGAACCAGTTCTCTTTGAAGCTGAGCTCTGTGACCGCCGCGGACACGGCCGTGTATTACTGTGCGAGAGGGTTCGGTGACTGGGACTACTGGGGCCAGGGAACCCTGGTCACCGTCTCCTCA |
| | | | SEQ ID NO: 27047 | SEQ ID NO: 31053 |

FIGURE 50

| | | | | |
|---|---|---|---|---|
| | | AA | DIQMTQSPSSLSASVGDRVTITFRTSQGIRNDLGWYQQQPGKTPKRMIYAASSLFSGVPSRFSGSRSGTDFTLTISSLQPEDFATYYCLQYNSYPPTFGQGTRLEIK | QVQLQESGPGLVKPSETLSLNCTVSGGSIRSYYWSWIRQPAGKGLEWIGRIYTSGSTDYNPSLKSRITMSVDTSKNQFSLKLSSVTAADTAVYYCARGFGDWDYWGQGTLVTVSS |
| | | | SEQ ID NO: 27048 | SEQ ID NO: 31054 |
| **iPS:436356** | **21-225_210H10** | NA | GAAATAGTGATGACGCAGTCTCCAGCCACCCTGTCTGTGTCTCCAGGGGAAAGAGCCACCCTCTCCTGCAGGGCCAGTCAGAGTGTTAAAAGCAACTTAGCCTGGTACCAGCAGAAACCTGGCCAGGCTCCCAGGCTCCTCATCTATGGTGCATCCACCAGGGCCACTGGTATCCCAGCCAGGTTCAGTGGCAGTGGGTCTGGGACAGAGTTCACTCTCACCATCAGCAGCCTGCAGTCTGAAGATTTTGCAGTTTATTCCTGTCAGCAGTATTATAACTGGCTGTGCAGTTTTGGCCAGGGGACCAAGCTGGAGATCAAA | CAGGTACAGCTGCAGCAGTCAGGTCCAGGACTGGTGAAGCCCTCGCAGACCCTCTCACTCACCTGTGCCATCTCCGGGGACAGTGTCTCTAGCAACAGTGCTGCTTGGAACTGGATCAGGCAGTCCCCATCGAGAGGCCTTGAGTGGCTGGGAAGGACATACTACAGGTCCAAGTGGTATAATTATTATCCAGTATCTGTGAGAAGTCGAATAACCATCAACCCAGACACATCCAAGAACCAGTTCTCCCTGCTGCTGAACTCTGTGACTCCCGAGGACACGGCTGTGTATTACTGTGCAAGAGATCAACGGTACTACGGTATGGACGTCTGGGGCCAAGGGACCACGGTCACCGTCTCCTCA |
| | | | SEQ ID NO: 27049 | SEQ ID NO: 31055 |
| | | AA | EIVMTQSPATLSVSPGERATLSCRASQSVKSNLAWYQQKPGQAPRLLIYGASTRATGIPARFSGSGSGTEFTLTISSLQSEDFAVYSCQQYYNWLCSFGQGTKLEIK | QVQLQQSGPGLVKPSQTLSLTCAISGDSVSSNSAAWNWIRQSPSRGLEWLGRTYYRSKWYNYYPVSVRSRITINPDTSKNQFSLLLNSVTPEDTAVYYCARDQRYYGMDVWGQGTTVTVSS |
| | | | SEQ ID NO: 27050 | SEQ ID NO: 31056 |

FIGURE 50

| | | | | |
|---|---|---|---|---|
| **iPS:436358** | **21-225_210D11** | NA | GACATCCAGATGACCCAGTCTCCATCCTCCCTGTCTGCATCTGTTGGAGACAGAGTCACCATCACTTGCCGGGCAAGTCACAACATTAACAGCTATTTAAATTGGTATCAGCAGAAATCAGGGAAAGCCCCTAAACTCCTAATCTATGCTGCATCCAGTTTGCAAAGTGGGGTCCCGTCAAGGTTCAGTGGCAGTGGATCTGGGACAGATTTCACTCTCACCTTCAGTAGTCTACAACCTGAAGATTTTGCAACTTACTACTGTCAACAGAGTTACAGTTTCCCGCTCACTTTCGGCGGAGGGACCAAGGTGGAGATGAGG | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATCCACTGGGTGCGACAGGCCCCTGGACAAGGGCTTGAGTGGATGGCATGGATCAACCCTAATAGTGGTGGCACAAACTATGCACAGAAGTTTCAGGGCAGGGTCACCATGACCAGGGACACGTCCATCACCACAGCCTACATGGAGCTGAGCAGGCTGAGATCTGACGACACGGCCGTGTATTACTGTGCGAGAGGATACAGCTATGGTTACAACTGGTTCGACCCCTGGGGCCAGGGAACCCTGGTCACCGTCTCCTCA |
| | | | SEQ ID NO: 27051 | SEQ ID NO: 31057 |
| | | AA | DIQMTQSPSSLSASVGDRVTITCRASHNINSYLNWYQQKSGKAPKLLIYAASSLQSGVPSRFSGSGSGTDFTLTFSSLQPEDFATYYCQQSYSFPLTFGGGTKVEMR | QVQLVQSGAEVKKPGASVKVSCKASGYTFTGYYIHWVRQAPGQGLEWMAWINPNSGGTNYAQKFQGRVTMTRDTSITTAYMELSRLRSDDTAVYYCARGYSYGYNWFDPWGQGTLVTVSS |
| | | | SEQ ID NO: 27052 | SEQ ID NO: 31058 |
| **iPS:436360** | **21-225_210H11** | NA | GACATCCAGATGACCCAGTCTCCATCTTCCGTGTCTGCTTCTGTAGGAGACAGAGTCACCATCACTTGTCGGGCGAGTCAGGGTATTAGCATCTGGTTAGCCTGGTATCAGCAGAAACCAGGGAAAGCCCCTAACCTCCTGATCTATGCTGCATCCAGTTTGCAAAGTGGAGTCCCATCAAGGTTCAGCGGCAGAGGATCTGGGACAGATTTCACTCTCACCATCAGCAGCCTGCAGCCTGAAGATTTTGCAACTTACTATTGTCAACAGGCTAAAAGTTTCCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCCATGGCATGCACTGGGTCCGCCAGGCTCCAGGCAAGGGGCTGGAGTGGGTGGCAGTTACATGGTATGATGGAAGTGATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTATATTACTGTGCGCGAGACCGGCTAGTGGGAGCTACTACCGATGCTTTTGATATCTGGGGCCAAGGGACAATGGTCACCGTCTCTTCA |
| | | | SEQ ID NO: 27053 | SEQ ID NO: 31059 |

FIGURE 50

| | | | | |
|---|---|---|---|---|
| | | AA | DIQMTQSPSSVSASVGDRVTITCRASQGISIWLAWYQQKPGKAPNLLIYAASSLQSGVPSRFSGRGSGTDFTLTISSLQPEDFATYYCQQAKSFPFTFGPGTKVDIK | QVQLVESGGGVVQPGRSLRLSCAASGFTFSSHGMHWVRQAPGKGLEWVAVTWYDGSDKYYADSVKGRFTISRDNSKNTLYLQMNSLRAEDTAVYYCARDRLVGATTDAFDIWGQGTMVTVSS |
| | | | SEQ ID NO: 27054 | SEQ ID NO: 31060 |
| **iPS:436362** | **21-225_210C12** | NA | GATATTGTGATGACTCAGTCTCCACTCTCCCTGCCCGTCACCCCTGGAGAGCCGGCCTCCATCTCCTGCAGGTCTAGTCAGAGCCTCCTGCATTATAATGGACACAACTTTTTGGATTGGTACCTGCAGAAGCCAGGGCAGTCTCCACAGCTCCTGATCTATTTGGTTTCTAATCGGGCCTCCGGGGTCCCTGACAGGTTCAGTGGCAGTGGATCAGGCACAGATTTTACACTGAAAATCAGCAGAGTGGAGGCTGAGGATGTTGGGGTTTATTACTGCATGCAAGCTCTACAAACTCCCATGTGCAGTTTTGGCCAGGGGACCAAGTTGGAGATCAAA | CAGGTTCAGCTGGTGCAGTCTGGAGCTGAGGTGACGAAGCCTGGGGCCTCAGTGAAGGTCTCCTGCAAGGCTTCTGGTTACACCTTTACCAACAATGGTATCAGCTGGGTGCGACAGGCCCCTGGACAAGGGCTTGAGTGGATGGGATGGATCAACGCTTACAATGGTCACACAAACTATGCACAGAAGTTCCAGGGCAGAGTCACCATGACCACAGACACATCCACGAGCACAGCCTACATGGAGCTGAGGAGCCTGAGATCTGACGACACGGCCGTGTATTACTGTGCGAGAGATCCTACGGTGACCCACTACTATTACTACGGTATGGACGTCTGGGGCCAAGGGACCACGGTCACCGTCTCCTCA |
| | | | SEQ ID NO: 27055 | SEQ ID NO: 31061 |
| | | AA | DIVMTQSPLSLPVTPGEPASISCRSSQSLLHYNGHNFLDWYLQKPGQSPQLLIYLVSNRASGVPDRFSGSGSGTDFTLKISRVEAEDVGVYYCMQALQTPMCSFGQGTKLEIK | QVQLVQSGAEVTKPGASVKVSCKASGYTFTNNGISWVRQAPGQGLEWMGWINAYNGHTNYAQKFQGRVTMTTDTSTSTAYMELRSLRSDDTAVYYCARDPTVTHYYYYGMDVWGQGTTVTVSS |
| | | | SEQ ID NO: 27056 | SEQ ID NO: 31062 |

FIGURE 50

| | | | | |
|---|---|---|---|---|
| **iPS:436364** | **21-225_211A11** | NA | GACATCCAGATGACCCAGTCTCCATCCTCACTGTCTGCATCTGTAGGAGACAGAGTCACCATCACTTGTCGGGCGAGTCAGGGCATTGGCAATTATTTAGCCTGGTTTCAGCAGAAACCAGGGAAAGCCCCTAGGTCCCTGATCTATGATGCATCCAGTTTGGAAAGTGGGGTCCCATCAAAGTTCAGCGGCAGTAGGTCTGGGACAGATTTCACTCTCACCATCGGCAGCCTGCAGCCTGAAGATTTTGCAACTTATTACTGCCAACACTATATGACTTACCCGCTCACTTTCGGCGCAGGGACCAAGGTGGAGATCAAA | CAGGTGCAGCTGGTGGGGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAATGGGTGGCAGTTCTTTGGTTTGATGGAAGTAATAGAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGGGGAGTGGGCTACTACGGTACGGACGTCTGGGGCCAAGGGACCACGGTCACCGTCTCCTCA |
| | | | SEQ ID NO: 27057 | SEQ ID NO: 31063 |
| | | AA | DIQMTQSPSSLSASVGDRVTITCRASQGIGNYLAWFQQKPGKAPRSLIYDASSLESGVPSKFSGSRSGTDFTLTIGSLQPEDFATYYCQHYMTYPLTFGAGTKVEIK | QVQLVGSGGGVVQPGRSLRLSCAASGFTFSSYGMHWVRQAPGKGLEWVAVLWFDGSNRNYADSVKGRFTISRDNSKNTLYLQMNSLRAEDTAVYYCARDRGVGYYGTDVWGQGTTVTVSS |
| | | | SEQ ID NO: 27058 | SEQ ID NO: 31064 |
| **iPS:436366** | **21-225_211A3** | NA | GACATCCGGATGACCCAGTCTCCATCCTCACTGTCTGCATCTGTAGGAGACAGAGTCACCATCACTTGTCGGGCGAGTCAGGCCATTGGGAAACATTTAGCCTGGTTTCAGCAGAGGCCTGGGAAAGCCCCTAAGTCCCTGATCTATGCTGCATCCAGATTGCAAAGTGGGGTCCCATCAAAGTTCAGCGGCAGTGGATCTGGGACAGATTTCACTCTCACCATCAGCAGCCTGCAGCCTGAAGATCTTGCAACTTATTACTGTCAACAGTATAGTAATTATCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGTGTCTGGAATCACCTTCAGTAGTTATGGCATGCACTGGGCCCGCCAGGCTCCAGGCAAGGGGCTGGAGTGGGTGGCAGTTATATGGTATGATGGAAGTAATAAAAACTATGAAGACTCCGTGAAGGGCCGATTCACCATCTCCAGAGACAACTCCAAGAACACGCTGTATCTGCAAATGAACAGCCTGAGAGCCGAGGACACGGCTGTGTATTACTGTGCGAGAGATGGGAGTTATGGTTACGACGGTATGGACGTCTGGGGCCAAGGGACCACGGTCACCGTCTCCTCA |
| | | | SEQ ID NO: 27059 | SEQ ID NO: 31065 |

FIGURE 50

| | | | | |
|---|---|---|---|---|
| | | AA | DIRMTQSPSSLSASVGDRVTITCRASQAIGKHLAWFQQRPGKAPKSLIYAASRLQSGVPSKFSGSGSGTDFTLTISSLQPEDLATYYCQQYSNYPLTFGGGTKVEIK | QVQLVESGGGVVQPGRSLRLSCAVSGITFSSYGMHWARQAPGKGLEWVAVIWYDGSNKNYEDSVKGRFTISRDNSKNTLYLQMNSLRAEDTAVYYCARDGSYGYDGMDVWGQGTTVTVSS |
| | | | SEQ ID NO: 27060 | SEQ ID NO: 31066 |
| **iPS:436368** | **21-225_211G3** | NA | GAAATTGTGTTGACGCAGTCTCCAGGCACCCTGTCTTTGTCTCCAGGGGAAAGAGCCACCCTCTCCTGCAGGGCCAGTCAGAGTGTTAGCAGCAGCTTCTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGTTAGCTCACCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGCATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGTTAGACTACAGTAACTACGGGTGGTTCGACCCCTGGGGCCAGGGAACCCTGGTCACCGTCTCCTCA |
| | | | SEQ ID NO: 27061 | SEQ ID NO: 31067 |
| | | AA | EIVLTQSPGTLSLSPGERATLSCRASQSVSSSFLAWYQQKPGQAPRLLIYGASSRATGIPDRFSGSGSGTDFTLTISRLEPEDFAVYYCQQYVSSPLTFGGGTKVEIK | QVQLVESGGGVVQPGRSLRLSCAASGFTFSNYGMHWVRQAPGKGLEWVAVIWHDGSNKYYADSVKGRFTISRDNSKNTLYLQMNSLRAEDTAVYYCARLDYSNYGWFDPWGQGTLVTVSS |
| | | | SEQ ID NO: 27062 | SEQ ID NO: 31068 |

FIGURE 50

| | | | | |
|---|---|---|---|---|
| iPS:436370 | 21-225_211A6 | NA | GACATCCAGATGACCCAGTCTCCATCCTCACTGTCTGCATCTGTAGGAGACAGCGTCACCATCACTTGTCGGGCGAGTCAGGGCATTGGCAAGTATTTAGCCTGGTTTCAGCAGAAACCAGGGAAAGCCCCTAAGTCCCTGATCTATGCTGCATCCAGTTTGCTAAGTGGGGTCCCATCAAAGTTCAGCGGCAGTGGATCTGGGACAGATTTCACTCTCACCATCAGCAGCCTTCAGCCTGAAGATTTTGCCACTTATTACTGCCAAAAGTATGATACTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGCAGACTCCGTGAAGGGCCGATTCACCATCTCCAGAGACAACTCCAAGAACACGCTGTATCTGCAAATGAACAGCCTGAGAGCCGAGGACACGGCTGTGTTTTACTGTGCGAGAGATAGGACGGTGGGCTATGATGGTTTTGATATCTGGGGCCAAGGGACAATGGTCACCGTCTCTTCA |
| | | | SEQ ID NO: 27063 | SEQ ID NO: 31069 |
| | | AA | DIQMTQSPSSLSASVGDSVTITCRASQGIGKYLAWFQQKPGKAPKSLIYAASSLLSGVPSKFSGSGSGTDFTLTISSLQPEDFATYYCQKYDTYPFTFGPGTKVDIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAVFYCARDRTVGYDGFDIWGQGTMVTVSS |
| | | | SEQ ID NO: 27064 | SEQ ID NO: 31070 |
| iPS:436372 | 21-225_211A8 | NA | GACATCGAGATGACCCAGTCTCCACCCTCACTGTCTGCATTTGTAGGAGACAGAGTCACCATCACTTGTCGGGCGAGTCAGGGCATTAGCAGATATTTAGCCTGGGTTCAGCAGAAACCAGGGAAAGCCCCTAAGTCCCTGATCTATGCTGCATCCAGTTTGCAAAGTGGGGTCTCATCAAGGTTCAGCGGCAGTGGATCTGGGACAGATTTCACTCTCACCATCAGCAGCCTGCAGCCTGAAGATTTCGCAACTTATTACTGCCTACGGTATGATACTTACCCTCTCATTTTCGGCGGAGGGACCAAGGTGGAGATCAAG | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATGAACACTATGCAGACTCCGTGAAGGGCCGATTCACCATCTCCAGAGACAATTCCAAGAAAACGCTGTATCTGCAAATGAACAGCCTGAGAGCCGAGGACACGGCTGTGTATTACTGTGCGAGAGACCACGGTGTCGGGTACTACGGTATGGACGTCTGGGGCCAAGGGACCACGGTCACCGTCTCCTCA |
| | | | SEQ ID NO: 27065 | SEQ ID NO: 31071 |

FIGURE 50

| | | | | |
|---|---|---|---|---|
| | | AA | DIEMTQSPPSLSAFVGDRVTITCRASQGISRYLAWVQQKPGKAPKSLIYAASSLQSGVSSRFSGSGSGTDFTLTISSLQPEDFATYYCLRYDTYPLIFGGGTKVEIK | QVQLVESGGGVVQPGRSLRLSCAASGFTFSSYGMHWVRQAPGKGLEWVAVIWYDGSNEHYADSVKGRFTISRDNSKKTLYLQMNSLRAEDTAVYYCARDHGVGYYGMDVWGQGTTVTVSS |
| | | | SEQ ID NO: 27066 | SEQ ID NO: 31072 |
| **iPS:436374** | **21-225_211C10** | NA | GATATTGTGATGACTCAGTCTCCACTCTCCCTGCCCGTCACCCCTGGAGAGCCGGCCTCCATCTCCTGCAGGTCTAGTCAGAGCCTCCTCCATAGTAATGGATACAACTATTTGGATTGGTACCTGCTGAAGCCAGGGCAGTCTCCACAGCTCCTGATCTATTTGGGTTCTAATCGGGCCTCCGGGGTCCCTGACAGGTTCAGTGGCAGTGGATCAGGCACAGATTTTACACTGAAAATCAGCAGAATGGAGGCTGAGGATGTTGGGATTTATTACTGCATGCAAGCTCTACTAACTCCCGTGTGCAGTTTTGGCCAGGGGACCAAGCTGGAGATCAAA | CAGGTTCAGCTGGTGCAGTCTGGAGCTGAGGTGAAGAAGCCTGGGGCCTCAGTGAAGGTCTCCTGCAAGGCTTCTGGTTACACCTTTACCAGGCATGGTATCAGCTGGGTGCGACTGGCCCCTGGACAAGGGCTTGAGTGGATGGGATGGATCAGCGCTTACAATGGTCTCACAAACTATGCACAGAAGTTCCAGGGCAGAGTCACCATGACCACAGACACATCCACGAGCACAGGCTACATGGAGCTGCGGAGCCTGAGATCTGACGACACGGCCGTGTATTACTGTGCGAGAGATCCTACGGTGACCCACTACTACTACTACGGTATGGACGTCTGGGGCCAAGGGACCACGGTCACCGTCTCCTCA |
| | | | SEQ ID NO: 27067 | SEQ ID NO: 31073 |
| | | AA | DIVMTQSPLSLPVTPGEPASISCRSSQSLLHSNGYNYLDWYLLKPGQSPQLLIYLGSNRASGVPDRFSGSGSGTDFTLKISRMEAEDVGIYYCMQALLTPVCSFGQGTKLEIK | QVQLVQSGAEVKKPGASVKVSCKASGYTFTRHGISWVRLAPGQGLEWMGWISAYNGLTNYAQKFQGRVTMTTDTSTSTGYMELRSLRSDDTAVYYCARDPTVTHYYYYGMDVWGQGTTVTVSS |
| | | | SEQ ID NO: 27068 | SEQ ID NO: 31074 |

FIGURE 50

| | | | | |
|---|---|---|---|---|
| **iPS:436376** | **21-225_212E6** | NA | GACATCCAGATGACCCAGTCTCCATCCTCACTGTCTGCATCTGTAGGAGACAGAGTCACCATCACTTGTCGGGCGAGTCAGGGCATTAGCAATCATTTAGCCTGGTTTCAGCAGAAACCAGGGCAAGCCCCTAAGTCCCTGATCTATGCTGCATCCAGTTTGCAAAGTGGGGTCCCATCAAAGTTCAGCGGCAGTAGATCTGGGACAGATTTCACTCTCACCATCAGCAGCCTGCAGCCTGAAGATTTTGCAACTTATTACTGCCAACAGTATGTTACCTACCCTCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGACTACGGTGTCGGGTACTACGGTACGGACGTCTGGGGCCAAGGGACCACGGTCACCGTCTCCTCA |
| | | | SEQ ID NO: 27069 | SEQ ID NO: 31075 |
| | | AA | DIQMTQSPSSLSASVGDRVTITCRASQGISNHLAWFQQKPGQAPKSLIYAASSLQSGVPSKFSGSRSGTDFTLTISSLQPEDFATYYCQQYVTYPLTFGGGTKVEIK | QVQLVESGGGVVQPGRSLRLSCAASGFTFSSYGMHWVRQAPGKGLEWVAVIWYDGSNKNYVDSVKGRFTISRDNSKNTLYLQMNSLRAEDTAVYYCARDYGVGYYGTDVWGQGTTVTVSS |
| | | | SEQ ID NO: 27070 | SEQ ID NO: 31076 |
| **iPS:436378** | **21-225_212D7** | NA | GACATCCAGATGACCCAGTCTCCATCCTCACTGTCTGCATCTGTAGGAGACAGAGTCACCATCACTTGTCGGGCGAGTCAGGGCATTAGCAATCATTTAGCCTGGTTTCAGCAGAAACCAGGGAAAGCCCCTAAGTCCCTGATCCATGCTGCATCCAGTTTGCAAAGTGGGGTCCCATCAAAATTCAGCGGCAATAGATCTGGGACAGATTTCACTCTCACCATCAACAACCTGCAGCCTGAAGATTTTGTAACTTATTACTGCCAGCAGTATAGTAATTACCCTCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAATTATGCAGACTCCGTGAAGGGCCGATTCACCATCTCCAGAGACAATTCCAAGAACACGCTGTCTCTGCAAATGAACAGCCTGAGAGCCGAGGACACGGCTGTGTATTACTGTGCGAGAGACTACGGTGTCGGGTACTACGGTACGGACGTCTGGGGCCAAGGGACCACGGTCACCGTCTCCTCA |
| | | | SEQ ID NO: 27071 | SEQ ID NO: 31077 |

FIGURE 50

| | | | | |
|---|---|---|---|---|
| | | AA | DIQMTQSPSSLSASVGDRVTITCRASQGISNHLA WFQQKPGKAPKSLIHAASSLQSGVPSKFSGNRSG TDFTLTINNLQPEDFVTYYCQQYSNYPLTFGGGT KVEIK | QVQLVESGGGVVQPGRSLRLSCAASGFTFSSYGMH WVRQAPGKGLEWVAVIWYDGSNKNYADSVKGRF TISRDNSKNTLSLQMNSLRAEDTAVYYCARDYGVG YYGTDVWGQGTTVTVSS |
| | | | SEQ ID NO: 27072 | SEQ ID NO: 31078 |
| **iPS:436380** | **21-225_212H9** | NA | GACATCGAGATGACCCAGTCTCCATCCTCACT GTCTGCATCTGTAGGAGACAGAGTCACCATCA CTTGTCGGGCGAGTCAGGGCATTAGCAGTTAT TTAGCCTGGCTTCAGCAGAAACCAGGGAAAGC CCCTAAGTCCCTGATCTATGCTGCATCCAGTTT GCAAAGTGGGGTCCCATCAAGGTTCAGCGGCA GTGGATCTGGGACAGATTTCACTCTCACCATC AGCAGCCTGCAGCCTGAAGATTTCGCAACTTA TTATTGCCTACGGTATGATACTTACCCTCTCAC TTTCGGCGGAGGGACCAAGGTGGAGATCAAG | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATGAACACTATGCAGACTCCGTGAAGGGCCGA TTCACCATCTCCAGAGACAATTCCAAGAAAACGC TGTATCTGCAAATGAACAGCCTGAGAGCCGAGG ACACGGCTGTGTATTACTGTGCGAGAGACCACGG TGTCGGGTACTACGGTATGGACGTCTGGGGCCAA GGGACCACGGTCACCGTCTCCTCA |
| | | | SEQ ID NO: 27073 | SEQ ID NO: 31079 |
| | | AA | DIEMTQSPSSLSASVGDRVTITCRASQGISSYLA WLQQKPGKAPKSLIYAASSLQSGVPSRFSGSGSG TDFTLTISSLQPEDFATYYCLRYDTYPLTFGGGT KVEIK | QVQLVESGGGVVQPGRSLRLSCAASGFTFSSYGMH WVRQAPGKGLEWVAVIWYDGSNEHYADSVKGRF TISRDNSKKTLYLQMNSLRAEDTAVYYCARDHGV GYYGMDVWGQGTTVTVSS |
| | | | SEQ ID NO: 27074 | SEQ ID NO: 31080 |

FIGURE 50

| | | | | |
|---|---|---|---|---|
| **iPS:436382** | **21-225_212C10** | NA | GAAATAGTGATGACGCAGTCTCCAGCCACCCTGTCTGTGTCTCCAGGGGAAAGAGCCACCCTCTCCTGCAGGGCCAGTCAGAGTGTTGCCAGCAGCTTAGCCTGGTACCAGCAGAAGCCTGGCCAGGCTCCCAGGCTCCTCATCCATGGTACATCCACCAGGGCCACTGATGTCCCAGCCAGGTTCAGTGGCTTTGGGTCTGGGTCGGACTTCACTCTCACCATCAGCAGCCTGCAGTCTGAAGATTTTGCAGTTTATTCCTGTCAGCAGTATAATGACTGGCCGTGCAGTTTTGGCCAGGGGACCAAGCTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAATGGGTGACAGCTATATGGTATGATGGAAGTCATAAATACTATACAGATTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGGAGTATAGTGGGAGCTACCTACTTTGACTACTGGGGCCAGGGAACCCTGGTCACCGTCTCCTCA |
| | | | SEQ ID NO: 27075 | SEQ ID NO: 31081 |
| | | AA | EIVMTQSPATLSVSPGERATLSCRASQSVASSLAWYQQKPGQAPRLLIHGTSTRATDVPARFSGFGSGSDFTLTISSLQSEDFAVYSCQQYNDWPCSFGQGTKLEIK | QVQLVESGGGVVQPGRSLRLSCAASGFTFSSYGMHWVRQAPGKGLEWVTAIWYDGSHKYYTDSVKGRFTISRDNSKNTLYLQMNSLRAEDTAVYYCARDRSIVGATYFDYWGQGTLVTVSS |
| | | | SEQ ID NO: 27076 | SEQ ID NO: 31082 |
| **iPS:436384** | **21-225_212F10** | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGACTGGTTTCAGCAGAAACCAGGGAAAGCCCCTAAGTCCCTGATCTATTCTGCATCCAATTTGCAAAGTGGGGTCCCATCAAAGTTCAGCGGCAGTAGATCTGGGACAGATTTCACTCTCACCATCAGCAGCCTGCAGCCTGAAGATTTTGCAACTTATTACTGCCAACACTATAGTAATTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGATATGGCATGCACTGGGTCCGCCAGGCTCCAGGCAAGGGGCTGGAGTGGGTGGCAGTTATATGGTATGATGGAAGTAATAAACACTATGCAGACTCCGTGAAGGGCCGATTCACCATCTCCAGAGACAATTCCAAGAATACGTTGTATCTGCAAATGAACAGTCTGAGAGGCGAGGACACGGCTGTGTATTACTGTGCGAGAGATCGGGGAGTGGGCTACAACGGTATGGACGTCTGGGGCCAAGGGACCACGGTCACCGTCTCCTCA |
| | | | SEQ ID NO: 27077 | SEQ ID NO: 31083 |

FIGURE 50

| | | | | |
|---|---|---|---|---|
| | | AA | DIQMTQSPSSLSASVGDRVTITCRASQGISNYLDWFQQKPGKAPKSLIYSASNLQSGVPSKFSGSRSGTDFTLTISSLQPEDFATYYCQHYSNYPLTFGGGTKVEIK | QVQLVESGGGVVQPGRSLRLSCAASGFTFSRYGMHWVRQAPGKGLEWVAVIWYDGSNKHYADSVKGRFTISRDNSKNTLYLQMNSLRGEDTAVYYCARDRGVGYNGMDVWGQGTTVTVSS |
| | | | SEQ ID NO: 27078 | SEQ ID NO: 31084 |
| iPS:436386 | 21-225_212B11 | NA | GACATCCAGATGACCCAGTCTCCATCTTCCCTGCCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCATTCTCACAATCAGCAGCCTGCAGCCTGAAGATTTTGCAACTTATTACTGTCTACTGCATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGTCATGCACTGGGTCCGCCAGGCTCCAGGCAAGGGGCTGGAGTGGGTGACAGTTATATGGTATGATGGAAGTAATAAATATTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACGTTATAGCAGTGGCTGGTACGACTACGGTATGGACGTCTGGGGCCAAGGGACCACGGTCACCGTCTCCTCA |
| | | | SEQ ID NO: 27079 | SEQ ID NO: 31085 |
| | | AA | DIQMTQSPSSLPASVGDRVTITCRASQGIRNDLGWYQQKPGKAPKRLIYAASSLQSGVPSRFSGSGSGTEFILTISSLQPEDFATYYCLLHYSYPRTFGQGTKVEIK | QVQLVESGGGVVQPGRSLRLSCAASGFTFSDYVMHWVRQAPGKGLEWVTVIWYDGSNKYYADSVKGRFTISRDNSKNTLYLQMNSLRAEDTAVYYCARERYSSGWYDYGMDVWGQGTTVTVSS |
| | | | SEQ ID NO: 27080 | SEQ ID NO: 31086 |

FIGURE 50

| | | | | |
|---|---|---|---|---|
| **iPS:436388** | **21-225_212H11** | NA | GACATCCGGATGACCCAGTCTCCATCCTCACTGTCTGCATCTGTAGGAGACAGAGTCACCATCACTTGTCGGGCGAGTCAGGCCATTGGGAAACATTTAGCCTGGTTTCAGCAGAGGCCTGGGAAAGCCCCTAAGTCCCTGATCTATGCTGCATCCAGATTGCAAAGTGGGGTCCCATCAAAGTTCAGCGGCAGTGGATCTGGGACAGATTTCACTCTCACCATCAGCAGCCTGCAGCCTGAAGATCTTGCAACTTATTACTGTCAACACTATAGTAATTATCCGCTCACTTTTGTCGGAGGGACCAAGGTGGAGATCACA | CAGGTGCAGCTGGTGGAGTCTGGGGGAGGCGTGGTCCAGCCTGGGAGGTCCCTGAGACTCTCCTGTGCAGTGTCTGGAATCACCTTCAGTAGTTATGGCATGCACTGGGCCCGCCAGGCTCCAGGCAAGGGGCTGGAGTGGGTGGCAGTTATATGGTATGATGGAAGTAATAAAAACTATGAAGACTCCGTGAAGGGCCGATTCACCATCTCCAGAGACAACTCCAAGAACACGCTGTATCTGCAAATGAACAGCCTGAGAGCCGAGGACACGGCTGTGTATTACTGTGCGAGAGATGGGAGTTATGGTTACGACGGTATGGACGTCTGGGGCCAAGGGACCACGGTCACCGTCTCCTCA |
| | | | SEQ ID NO: 27081 | SEQ ID NO: 31087 |
| | | AA | DIRMTQSPSSLSASVGDRVTITCRASQAIGKHLAWFQQRPGKAPKSLIYAASRLQSGVPSKFSGSGSGTDFTLTISSLQPEDLATYYCQHYSNYPLTFVGGTKVEIT | QVQLVESGGGVVQPGRSLRLSCAVSGITFSSYGMHWARQAPGKGLEWVAVIWYDGSNKNYEDSVKGRFTISRDNSKNTLYLQMNSLRAEDTAVYYCARDGSYGYDGMDVWGQGTTVTVSS |
| | | | SEQ ID NO: 27082 | SEQ ID NO: 31088 |
| **iPS:436390** | **21-225_213D2** | NA | GACATCCAGATGACCCAGTCTCCATCCTCACTGTCTGCATCTGTAGGAGACAGAGTCACCATCACTTGTCGGGCGAGTCAGGGCATTAGCAATCATTTAGCCTGGTTTCAGCAGAAACCAGGGAAAGCCCCTAAGTCCCTGATCCATGCTGCATCCAGTTTGCAAAGTGGGGTCCCATCAAAATTCAGCGGCAATAGATCTGGGACAGATTTCACTCTCACCATCAACAACCTGCAGCCTGAAGATTTTGTAACTTATTACTGCCACCAGTATAGTAATTACCCTCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAATTATGCAGACTCCGTGAAGGGCCGATTCACCATCTCCAGAGACAATTCCAAGAACACGCTGTCTCTGCAAATGAACAGCCTGAGAGCCGAGGACACGGCTGTGTATTACTGTGCGAGAGACTACGGTGTCGGGTACTACGGTACGGACGTCTGGGGCCAAGGGACCACGGTCACCGTCTCCTCA |
| | | | SEQ ID NO: 27083 | SEQ ID NO: 31089 |

FIGURE 50

| | | | | |
|---|---|---|---|---|
| | | AA | DIQMTQSPSSLSASVGDRVTITCRASQGISNHLAWFQQKPGKAPKSLIHAASSLQSGVPSKFSGNRSGTDFTLTINNLQPEDFVTYYCHQYSNYPLTFGGGTKVEIK | QVQLVESGGGVVQPGRSLRLSCAASGFTFSSYGMHWVRQAPGKGLEWVAVIWYDGSNKNYADSVKGRFTISRDNSKNTLSLQMNSLRAEDTAVYYCARDYGVGYYGTDVWGQGTTVTVSS |
| | | | SEQ ID NO: 27084 | SEQ ID NO: 31090 |
| iPS:436392 | 21-225_213B3 | NA | GACATCCAGATGACCCAGTCTCCATCCTCACTGTCTGCATCTGTAGGAGACAGAGTCACCATCACTTGTCGGGCGAGTCAGGGCATTGGCAAGTATTTAGCCTGGTTTCAGCAGAAACCAGGGAAAGCCCCTAAGTCCCTGATCTCTGCTGCATCCAGTGTGCTAAGTGGGGTCCCATCAAAGTTCAGCGGCAGTGGATCTGGGACAGATTTCACTCTCACCATCAGCAGCCTTCAGCCTGAAGATTTTGCCACTTATTACTGCCAAAAGTATGATACTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGCAGACTCCGTGAAGGGCCGATTCACCATCTCCAGAGACAACTCCAAGAACACGCTGTATCTGCAAATGAACAGCCTGAGAGCCGAGGACACGGCTGTGTATTACTGTGCGAGAGATAGGACGGTGGGCTATGATGGTTTTGATATCTGGGGCCAAGGGACAATGGTCACCGTCTCTTCA |
| | | | SEQ ID NO: 27085 | SEQ ID NO: 31091 |
| | | AA | DIQMTQSPSSLSASVGDRVTITCRASQGIGKYLAWFQQKPGKAPKSLISAASSVLSGVPSKFSGSGSGTDFTLTISSLQPEDFATYYCQKYDTYPFTFGPGTKVDIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAVYYCARDRTVGYDGFDIWGQGTMVTVSS |
| | | | SEQ ID NO: 27086 | SEQ ID NO: 31092 |

FIGURE 50

| | | | | |
|---|---|---|---|---|
| **iPS:436394** | **21-225_213C4** | NA | GACATCCATATGACCCAGTCTCCATCCTCACTGTCTGCATCTGTAGGAGACAGAGTCACCATCACTTGTCGGGCGAGTCAGGCCATTAGGAATTATTTAGCCTGGTGTCAGCAGAAACCAGGGAAAGCCCCTAAGACCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATAGTAATTACCCTCTCACTTTCGGCGGAGGGACCAAGGTGGAGATCAAA | CAGGTGCAGTTGGTGGAGTCTGGGGGAGGCGTGGTCCAGCCTGGGAGGTCCCTGAGACTCTCCTGTGCAACGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAACACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGACTACGGTGTCGGGTACGACGGTATGGACGTCTGGGGCCAAGGGACCACGGTCACCGTCTCCTCA |
| | | | SEQ ID NO: 27087 | SEQ ID NO: 31093 |
| | | AA | DIHMTQSPSSLSASVGDRVTITCRASQAIRNYLAWCQQKPGKAPKTLIYAASSLQSGVPSKFSGSGSGTDFTLTISSLQPEDFATYYCQQYSNYPLTFGGGTKVEIK | QVQLVESGGGVVQPGRSLRLSCATSGFTFSSYGMHWVRQAPGKGLEWVAVIWYDGSNKHYADSVKGRFTISRDNSKNTLYLQMNSLRAEDTAVYYCARDYGVGYDGMDVWGQGTTVTVSS |
| | | | SEQ ID NO: 27088 | SEQ ID NO: 31094 |
| **iPS:436396** | **21-225_213E5** | NA | GACATCCGGATGACCCAGTCTCCATCCTCACTGTCTGCATCTGTAGGAGACAGAGTCACCATCACTTGTCGGGCGAGTCAGGCCATTGGGAAACATTTAGCCTGGTTTCAGCAGAGGCCTGGGAAAGCCCCTAAGTCCCTGATCTATGCTGCATCCAGATTGCAAAGTGGGGTCCCATCAAAGTTCAGCGGCAGTGGATCTGGGACAGATTTCACTCTCACCATCAGCAGCCTGCAGCCTGAAGATCTTGCAACTTATTACTGTCAACACTATAGTAATTATCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGTGTCTGGAATCACCTTCAGTAGTTATGGCATGCACTGGGCCCGCCAGGCTCCAGGCAAGGGGCTGGAGTGGGTGGCAGTTATATGGTATGATGGAAGTAATAAAAACTATGAAGACTCCGTGAAGGGCCGATTCACCATCTCCAGAGACAACTCCAAGAACACGCTGTATCTGCAAATGAACAGCCTGAGAGCCGAGGACACGGCTGTGTATTACTGTGCGAGAGATGGGAGTTATGGTTACGACGGTATGGACGTCTGGGGCCAAGGGACCACGGTCACCGTCTCCTCA |
| | | | SEQ ID NO: 27089 | SEQ ID NO: 31095 |

FIGURE 50

| | | | | |
|---|---|---|---|---|
| | | AA | DIRMTQSPSSLSASVGDRVTITCRASQAIGKHLAWFQQRPGKAPKSLIYAASRLQSGVPSKFSGSGSGTDFTLTISSLQPEDLATYYCQHYSNYPLTFGGGTKVEIK | QVQLVESGGGVVQPGRSLRLSCAVSGITFSSYGMHWARQAPGKGLEWVAVIWYDGSNKNYEDSVKGRFTISRDNSKNTLYLQMNSLRAEDTAVYYCARDGSYGYDGMDVWGQGTTVTVSS |
| | | | SEQ ID NO: 27090 | SEQ ID NO: 31096 |
| **iPS:436398** | **21-225_213B8** | NA | GACATCCAGATGACCCAGTCTCCATCCTCACTGTCTGCATCTGTAGGAGACAGAGTCACCATCACTTGTCGGGCGAGTCAGGGCATTAGCAATCATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATAGTAATTACCCTCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAACACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGACTACGGTGTCGGGTACTACGGTACGGACGTCTGGGGCCAAGGGACCACGGTCACCGTCTCCTCA |
| | | | SEQ ID NO: 27091 | SEQ ID NO: 31097 |
| | | AA | DIQMTQSPSSLSASVGDRVTITCRASQGISNHLAWFQQKPGKAPKSLIYAASSLQSGVPSKFSGSGSGTDFTLTISSLQPEDFATYYCQQYSNYPLTFGGGTKVEIK | QVQLVESGGGVVQPGRSLRLSCAASGFTFSSYGMHWVRQAPGKGLEWVAVIWYDGSNKHYADSVKGRFTISRDNSKNTLYLQMNSLRAEDTAVYYCARDYGVGYYGTDVWGQGTTVTVSS |
| | | | SEQ ID NO: 27092 | SEQ ID NO: 31098 |

FIGURE 50

| | | | | |
|---|---|---|---|---|
| **iPS:436400** | **21-225_213H7** | NA | GACATCGTGATGACCCAGTCTCCAGACTCCCTGGCTGTGTCTCTGGGCGAGAGGGCCACCATCAACTGCAAGTCCAGCCAGAATGTTTTAGACATCTCCAACAATAAGAATTCCTTAGGTTGGTTCCAGCAGAAACCAGGTCAGCCTCCCAAGCTGCTCATTAACTGGGCATCTACCCGGGAATCCGGGGTCCCTGACCGCTTCAGTGGCAGCGGGTCTGGGACAGATTTCACTCTCACCATCAGCAGCCTGCAGACTGAAGATGTGGCAGTTTATCACTGTCAGCAATATTATAACATTCCTCCGACGTTCGGCCGAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACCATATGCACTGGGTGCGACAGGCCCCTGGACAAGGGCTTGAGTGGATGGGATGGATCAATCCTAAGAGTGATGGCACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGAGAAAAGCCTGGGAGCTACTACAAATACTGGGGCCAGGGAACCCTGGTCACCGTCTCCTCA |
| | | | SEQ ID NO: 27093 | SEQ ID NO: 31099 |
| | | AA | DIVMTQSPDSLAVSLGERATINCKSSQNVLDISNNKNSLGWFQQKPGQPPKLLINWASTRESGVPDRFSGSGSGTDFTLTISSLQTEDVAVYHCQQYYNIPPTFGRGTKVEIK | QVQLVQSGAEVKKPGASVKVSCKASGYTFTGYHMHWVRQAPGQGLEWMGWINPKSDGTNYAQKFQGRVTMTRDTSISTAYMELSRLRSDDTAVYYCAREKPGSYYKYWGQGTLVTVSS |
| | | | SEQ ID NO: 27094 | SEQ ID NO: 31100 |
| **iPS:436402** | **21-225_213H12** | NA | GACATCGTGATGACCCAGTCTCCAGACTCCCTGGCTGTGTCTCTGGGCGAGAGGGCCACCATCACCTGCAAGTCCAGCCAGAATGTTTTAAAGACCTCCAACAATAGGAACTACTTAGCTTGGTACCAGCAGAAACCAGGACAGCCTCCTAAGGTGCTCATTTACTGGGCATCTACCCGGGAATCCGGGGTCCCTGACCGATTCAGTGGCAGCGGGTCTGGGACAGATTTCACTCTCATCATCAGCAGCCTGCAGGCTGAAGATGTGGCAGTTTATTACTGTCACCAATATTATAGTATTCCGTGGACCTTCGGCCAAGGGACCAAGGTGGAAATCAAG | CAGGTTCAGCTGGTGCAGTCTGGAGCTGAGGTGAAGAAGCCTGGGGCCTCAGTGAAGGTCTCCTGCAAGGCTTCTGGTTTCACCTTTACCAGCTATGGTATCAACTGGGTGCGACAGGCCCCTGGACAAGGGCTTGAGTGGATGGGATGGATCAGCGTTCACAATGGTAACACAGACTATGCACAGAAGTTCCAGGGCAGAGTCACCATGACCACAGACACATCCACGAGCACAGCCTACATGGAACTGAGGAGCCTGAGATCTGACGACACGGCCGTGTATTACTGTGCGAGAGACTACTACTACGGTATGGACGTCTGGGGCCAAGGGACCAAGGTCACCGTCTCCTCA |
| | | | SEQ ID NO: 27095 | SEQ ID NO: 31101 |

FIGURE 50

| | | | | |
|---|---|---|---|---|
| | | AA | DIVMTQSPDSLAVSLGERATITCKSSQNVLKTSNNRNYLAWYQQKPGQPPKVLIYWASTRESGVPDRFSGSGSGTDFTLIISSLQAEDVAVYYCHQYYSIPWTFGQGTKVEIK | QVQLVQSGAEVKKPGASVKVSCKASGFTFTSYGINWVRQAPGQGLEWMGWISVHNGNTDYAQKFQGRVTMTTDTSTSTAYMELRSLRSDDTAVYYCARDYYYGMDVWGQGTKVTVSS |
| | | | SEQ ID NO: 27096 | SEQ ID NO: 31102 |
| **iPS:436404** | **21-225_214C3** | NA | GACATCCAGATGACCCAGTCTCCATCCTCACTGTCTGCATCTGTTGGAGACAGAGTCACCATAACTTGTCGGGCGAGTCAGGGCATTGGCAATTATTTAGCCTGGTTTCAGCAGAAACCAGGTAAAGTCCCTAAGTCCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTACAACTTATTACTGCCAACAATATATGACTTACCCAATCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGAAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGGAGACTCCGTGAAGGGCCGATTCACCATCTCCAGAGACAATTCCAAGAATACGCTGTATCTGCAAATGAACAGCCTGAGAGCCGAGGACACGGCTGTGTATTACTGTGCGAGAGATCGGGGAGTGGGCTACGACGGAATGGACGTCTGGGGCCAAGGGACCACGGTCACCGTCTCCTCA |
| | | | SEQ ID NO: 27097 | SEQ ID NO: 31103 |
| | | AA | DIQMTQSPSSLSASVGDRVTITCRASQGIGNYLAWFQQKPGKVPKSLIYAASSLQSGVPSKFSGSGSGTDFTLTISSLQPEDFTTYYCQQYMTYPITFGPGTKVDIK | QVQLVESGGGVVQPGRSLRLSCEASGFTFSSYGMHWVRQAPGKGLEWVAVIWYDGSNKNYGDSVKGRFTISRDNSKNTLYLQMNSLRAEDTAVYYCARDRGVGYDGMDVWGQGTTVTVSS |
| | | | SEQ ID NO: 27098 | SEQ ID NO: 31104 |

FIGURE 50

| | | | | |
|---|---|---|---|---|
| **iPS:436406** | **21-225_214E4** | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATCTTACTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATGAAAACTATGCAGACTCCGTGAAGGGCCGAATCACCATCTCCAGAGACAATTCCAAGAACACGCTGTATCTGCAAATGAACAGCCTGAGAGCCGAGGACACGGCTGTGTATTACTGTGCGAGAGATAGGACGGTGGGCTATGATGGTTGTGATATCTGGGGCCAAGGGGCAATGGTCACCGTCTCTTCA |
| | | | SEQ ID NO: 27099 | SEQ ID NO: 31105 |
| | | AA | DIQMTQSPSSLSASVGDRVTITCRASQGISNYLAWFQQKPGKAPKSLIYAASSLQSGVPSKFSGSGSGTDFTLTISSLQPEDFATYYCQQYLTYPFTFGPGTKVDIK | QVQLVESGGGVVQPGRSLRLSCAASGFTFSSYGMHWVRQAPGKGLEWVAVIWYDGSNENYADSVKGRITISRDNSKNTLYLQMNSLRAEDTAVYYCARDRTVGYDGCDIWGQGAMVTVSS |
| | | | SEQ ID NO: 27100 | SEQ ID NO: 31106 |
| **iPS:436408** | **21-225_214H8** | NA | GAAATTGTGCTGACTCAGTCTCCAGACTTTCAGTCTGTGACTCCAAAGGAGAAAGTCACCATCACCTGCCGGGCCAGTCAGAGCATTGGTGTTAGCTTACACTGGTACCAGCAGAAACCAGATCAGTCTCCACAACTCCTCATCAAGTATGCTTCCCAGTCCTTCTCAGGGGTCCCCTCGAGGTTCAGTGGCAGTGGATCTGGGACAGATTTCACCCTCACCATCAATAGCCTGGAAGCTGAAGATGCTGCAACGTATTACTGTCATCAGAGTCGTAGTTTACCATTCACTTTCGGCCCTGGGTCCAAAGTGGATATCAAA | CAGGTCCAGCTGGTGCAGTCTGGGGCTGAGGTGAAGAAGCCTGGGGCCTCAGTGAAGGTCTCCTGCAAGGCTTCTGGATACACCTTCACCGGCCACTATATACACTGGGTGCGACAGGCCCCTGGACAAGGGCTTGAGTGGATGGGATGGATCAACTCTAACAGTGGTGGCACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTATATTACTGTGCGAAAGACGGGAGATACAGCTATGGTTACGACTGGTTCGACCCCTGGGGCCAGGGAACCCTGGTCACCGTCTCCTCA |
| | | | SEQ ID NO: 27101 | SEQ ID NO: 31107 |

FIGURE 50

| | | | | |
|---|---|---|---|---|
| | | AA | EIVLTQSPDFQSVTPKEKVTITCRASQSIGVSLHWYQQKPDQSPQLLIKYASQSFSGVPSRFSGSGSGTDFTLTINSLEAEDAATYYCHQSRSLPFTFGPGSKVDIK | QVQLVQSGAEVKKPGASVKVSCKASGYTFTGHYIHWVRQAPGQGLEWMGWINSNSGGTNYAQKFQGRVTMTRDTSISTAYMELSRLRSDDTAVYYCAKDGRYSYGYDWFDPWGQGTLVTVSS |
| | | | SEQ ID NO: 27102 | SEQ ID NO: 31108 |
| iPS:436410 | 21-225_212E10 | NA | GACATCCAGATGACCCAGTCTCCATCCTCACTGTCTGCATCTGTAGGAGACAGAGTCACCATCACTTGTCGGGCGAGTCAGGGCATTAGCAATCATTTAGCCTGGTTTCAGCAGAAACCAGGGAAAGCCCCTAAGTCCCTGATCTATGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATAGTAATTACCCTCTCACTTTCGGCGGAGGGACCAAGGTGGAGATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTCTCTGCAAATGAACAGCCTGAGAGCCGAGGACACGGCTGTGTATTACTGTGCGAGAGACTACGGTGTCGGGTACTACGGTACGGACGTCTGGGGCCAAGGGACCACGGTCACCGTCTCCTCA |
| | | | SEQ ID NO: 27103 | SEQ ID NO: 31109 |
| | | AA | DIQMTQSPSSLSASVGDRVTITCRASQGISNHLAWFQQKPGKAPKSLIYAASSLQSGVPSKFSGSGSGTDFTLTISSLQPEDFATYYCQQYSNYPLTFGGGTKVEIK | QVQLVESGGGVVQPGRSLRLSCAASGFTFSSYGMHWVRQAPGKGLEWVAVIWYDGSNKYYADSVKGRFTISRDNSKNTLSLQMNSLRAEDTAVYYCARDYGVGYYGTDVWGQGTTVTVSS |
| | | | SEQ ID NO: 27104 | SEQ ID NO: 31110 |

FIGURE 50

| | | | | |
|---|---|---|---|---|
| **iPS:436412** | **21-225_214H9** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGTCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTATTTTACTGTGCGAGAGAGAGGTATACCAGCAGCTGGTACGACTACGGTATGGACGTCTGGGGCCAAGGGACCACGGTCACCGTCTCCTCA |
| | | | SEQ ID NO: 27105 | SEQ ID NO: 31111 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPRTFGQGTKVEIK | QVQLVESGGGVVQPGRSLRLSCAASGFTFSSYVMHWVRQAPGKGLEWVAVIWYDGSNKYYADSVKGRFTISRDNSKNTLYLQMNSLRAEDTAVFYCARERYTSSWYDYGMDVWGQGTTVTVSS |
| | | | SEQ ID NO: 27106 | SEQ ID NO: 31112 |
| **iPS:436414** | **21-225_214G10** | NA | GACATCCAGATGACCCTGTGTCCATCTTCCCCGCCTGCATCTGTTGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCATTCTCACAATCAGCAGCCTGCAGCCTGAAGATTTTGCAACTTATTACTGTCTACTGCATAATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGTCATGCACTGGGTCCGCCAGGCTCCAGGCAAGGGGCTGGAGTGGGTGACAGTTATATGGTATGATGGAAGTAATAAATATTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACGTTATAGCAGTGGCTGGTACGACTACGGTATGGACGTCTGGGGCCAAGGGACCACGGTCACCGTCTCCTCA |
| | | | SEQ ID NO: 27107 | SEQ ID NO: 31113 |

FIGURE 50

| | | | | |
|---|---|---|---|---|
| | | AA | DIQMTLCPSSPPASVGDRVTITCRASQGIRNDLG WYQQKPGKAPKRLIYAASSLQSGVPSRFSGSGS GTEFILTISSLQPEDFATYYCLLHNSYPRTFGQGT KVEIK | QVQLVESGGGVVQPGRSLRLSCAASGFTFSDYVM HWVRQAPGKGLEWVTVIWYDGSNKYYADSVKGR FTISRDNSKNTLYLQMNSLRAEDTAVYYCARERYS SGWYDYGMDVWGQGTTVTVSS |
| | | | SEQ ID NO: 27108 | SEQ ID NO: 31114 |
| **iPS:436416** | **21-225_214G12** | NA | GACATCCAGATGACCCAGTCTCCATCTTCCCC GCCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTTATCTATGCTGCATCCAGTT TGCAAAGTGGGGTCCCATCAAGGTTCAGCGGC AGTGGATCTGGGACAGAATTCATTCTCACAAT CAGCAGCCTGCAGCCTGAAGATTTTGCAACTT ATTACTGTGTAATGCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGACAGTTATATGGTATGATGGAAGT AATAAATATTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACGTTAT AGCAGTGGCTGGTACGACTACGGTATGGACGTCT GGGGCCAAGGGACCACGGTCACCGTCTCCTCA |
| | | | SEQ ID NO: 27109 | SEQ ID NO: 31115 |
| | | AA | DIQMTQSPSSPPASVGDRVTITCRASQGIRNDLG WYQQKPGKAPKRLIYAASSLQSGVPSRFSGSGS GTEFILTISSLQPEDFATYYCVMHYSYPRTFGQG TKVEIK | QVQLVESGGGVVQPGRSLRLSCAASGFTFSDYVM HWVRQAPGKGLEWVTVIWYDGSNKYYADSVKGR FTISRDNSKNTLYLQMNSLRAEDTAVYYCARERYS SGWYDYGMDVWGQGTTVTVSS |
| | | | SEQ ID NO: 27110 | SEQ ID NO: 31116 |

FIGURE 50

| | | | | |
|---|---|---|---|---|
| **iPS:436418** | **21-225_215E3** | NA | GACATCCAGATGACCCAGTCTCCATCCTCCCCGCCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTTATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTGTAATGCATAATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGTCATACACTGGGTCCGCCAGGCTCCAGGCAAGGGGCTGGAGTGGGTGACAGTTATATGGTATGATGGAAGTAATAAATATTATGCAGACTCCGTGAAGGGCCGATTCACCATCTCCAGAGACAATTCCAAGAACACGCTGTATCTGCAAATGAACAGTGTGAGAGCCGAGGACACGGCTGTGTATTACTGTGCGAGAGAACGTTATAGCAGTGGCTGGTACGACTACGGTATGGACGTCTGGGGCCAAGGGACCACGGTCACCGTCTCCTCA |
| | | | SEQ ID NO: 27111 | SEQ ID NO: 31117 |
| | | AA | DIQMTQSPSSPPASVGDRVTITCRASQGIRNDLGWYQQKPGKAPKRLIYAASSLQSGVPSRFSGSGSGTEFTLTISSLQPEDFATYYCVMHNSYPRTFGQGTKVEIK | QVQLVESGGGVVQPGRSLRLSCAASGFTFSDYVIHWVRQAPGKGLEWVTVIWYDGSNKYYADSVKGRFTISRDNSKNTLYLQMNSVRAEDTAVYYCARERYSSGWYDYGMDVWGQGTTVTVSS |
| | | | SEQ ID NO: 27112 | SEQ ID NO: 31118 |
| **iPS:436420** | **21-225_215B5** | NA | GACATCCAGATGACCCAGTCTCCATCCTCACTGTCTGCATCTGTAGGAGACAGAGTCACCATCACTTGTCGGGCGAGTCAGGGCATTAGCAATCATTTAGCCTGGTTTCAGCAGAAACCAGGGAAAGCCCCTAAGTCCCTGATCCATGCTGCATCCAGTTTGCAAAGTGGGGTCCCATCAAAATTCAGCGGCAATAGATCTGGGACAGATTTCACTCTCACCATCAACAACCTGCAGCCTGAAGATTTTGTAACTTATTACTGCCAGCAGTATATTAATTACCCTCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAATTATGCAGACTCCGTGAAGGGCCGATTCACCATCTCCAGAGACAATTCCAAGAACACGCTGTCTCTGCAAATGAACAGCCTGAGAGCCGAGGACACGGCTGTGTATTACTGTGCGAGAGACTACGGTGTCGGGTACTACGGTACGGACGTCTGGGGCCAAGGGACCACGGTCACCGTCTCCTCA |
| | | | SEQ ID NO: 27113 | SEQ ID NO: 31119 |

FIGURE 50

| | | | | |
|---|---|---|---|---|
| | | AA | DIQMTQSPSSLSASVGDRVTITCRASQGISNHLAWFQQKPGKAPKSLIHAASSLQSGVPSKFSGNRSGTDFTLTINNLQPEDFVTYYCQQYINYPLTFGGGTKVEIK | QVQLVESGGGVVQPGRSLRLSCAASGFTFSSYGMHWVRQAPGKGLEWVAVIWYDGSNKNYADSVKGRFTISRDNSKNTLSLQMNSLRAEDTAVYYCARDYGVGYYGTDVWGQGTTVTVSS |
| | | | SEQ ID NO: 27114 | SEQ ID NO: 31120 |
| **iPS:436422** | **21-225_215D6** | NA | GACATCCAGATGACCCAGTCTCCATCCTCACTGTCTGCATCTGTAGGAGACAGAGTCACCATCACTTGTCGGGCGAGTCAGGGCATTAGCAATCATTTAGCCTGGTTTCAGCAGAAACCAGGGAAAGCCCCTAAGTCCCTGATCTATGCTGCATCCAGTTTGCATAGTGGGGTCCCATCAAAGTTCAGCGGCAGTAGATCTGGGACAGATTTCACTCTCACCATCAGCAGCCTGCAGCCTGAAGATTTTGCAACTTATTACTGCCAACAGTATGTTACTTACCCTCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGCAGATTCCGTGAAGGGCCGATTCACCATCTCCAGAGACAATTCCAAGAACACACTGTATCTGCAAATGAACAGCCTGAGAGCCGAGGACACGGCTGTGTATTACTGTGCGAGAGACTGCGGTGTCGGATACTACGGTACGGACGTCTGGGGCCAAGGGACCACGGTCACCGTCTCCTCA |
| | | | SEQ ID NO: 27115 | SEQ ID NO: 31121 |
| | | AA | DIQMTQSPSSLSASVGDRVTITCRASQGISNHLAWFQQKPGKAPKSLIYAASSLHSGVPSKFSGSRSGTDFTLTISSLQPEDFATYYCQQYVTYPLTFGGGTKVEIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAVYYCARDCGVGYYGTDVWGQGTTVTVSS |
| | | | SEQ ID NO: 27116 | SEQ ID NO: 31122 |

FIGURE 50

| | | | | |
|---|---|---|---|---|
| **iPS:436424** | **21-225_215H6** | NA | GAAATTGTGCTGACTCAGTCTCCAGACTTTCAGTCTGTGACTCCAAAGGAGAAAGTCACCATCACCTGCCGGGCCAGTCAGAGCATCGGTGTTAGCTTACACTGGTACCAGCAGAAACCAGATCAGTCTCCACAGCTCCTCATCAAGTATGCTTCCCAGTCCCTCTCAGGGGTCCCCTCGAGGTTCAGTGGCAGTGGATCTGGGACAGATTTCACCCTCACCATCAATAGCCTGGAAGCTGAAGATGCTGCAACGTATTACTGTCATCAGAGTCGCAGTTTACCATTCACTTTCGGCCCTGGGTCCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCCACTATATACACTGGGTGCGACAGGCCCCTGGACAAGGGCTTGAGTGGATGGGATGGATCAACTCTAACAGTGGTGGCACAAATTATGCAGAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAAAGACGGGAGATACAGCTATGGTCACGACTGGTTCGACCCCTGGGGCCAGGGAACCCTGGTCACCGTCTCCTCA |
| | | | SEQ ID NO: 27117 | SEQ ID NO: 31123 |
| | | AA | EIVLTQSPDFQSVTPKEKVTITCRASQSIGVSLHWYQQKPDQSPQLLIKYASQSLSGVPSRFSGSGSGTDFTLTINSLEAEDAATYYCHQSRSLPFTFGPGSKVDIK | QVQLVQSGAEVKKPGASVKVSCKASGYTFTGHYIHWVRQAPGQGLEWMGWINSNSGGTNYAEKFQGRVTMTRDTSISTAYMELSRLRSDDTAVYYCAKDGRYSYGHDWFDPWGQGTLVTVSS |
| | | | SEQ ID NO: 27118 | SEQ ID NO: 31124 |
| **iPS:436426** | **21-225_215C7** | NA | GAAATTGTGTTGACGCAGTCTCCAGGCACCCTGTCTTTGTCTCCAGGGGAAAGAGTCACCCTCTCCTGCAGGGCCAGTCAGAGGATTACCACCAACTTCTTAGCTTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGTCTGAAGATTTTGCAGTTTATTACTGTCAGCAGTATGTTAGTTCATTGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTTACTATGGCATGCACTGGGTCCGCCAGGCTCCAGGCAAGGGGCTGGAGTGGGTGGCAGTTATATGGCATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGTTAGACTACAGTAATTACGGGTGGTTCGACCCCTGGGGCCAGGGAACCCTGGTCACCGTCTCCTCA |
| | | | SEQ ID NO: 27119 | SEQ ID NO: 31125 |

FIGURE 50

| | | | | |
|---|---|---|---|---|
| | | AA | EIVLTQSPGTLSLSPGERVTLSCRASQRITTNFLA WYQQKPGQAPRLLIYGASSRATGIPDRFSGSGSG TDFTLTISRLESEDFAVYYCQQYVSSLLTFGGGT KVEIK | QVQLVESGGGVVQPGRSLRLSCAASGFTFSYYGM HWVRQAPGKGLEWVAVIWHDGSNKYYADSVKGR FTISRDNSKNTLYLQMNSLRAEDTAVYYCARLDYS NYGWFDPWGQGTLVTVSS |
| | | | SEQ ID NO: 27120 | SEQ ID NO: 31126 |
| **iPS:436428** | **21-225_215E11** | NA | GACATCCAGATGACCCAGTGTCCATCTTCCCC GCCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTTATCTATGCTGCATCCAGTT TGCAAAGTGGGGTCCCATCAAGGTTCAGCGGC AGTGGATCTGGGACAGAATTCATTCTCACAAT CAGCAGCGTGCAGCGTGAAGATTTTGCAACTT ATTACTGTGTAATGCATTATAGTTACCCTCGG ACGTTCGGCCAAGGGACCAAGGTGGAAATCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTGACTATGTCAT GCACTGGGTCCGCCAGGCTCCAGGCAAGGGGCT GGAGTGGGTGACAGTTATATGGTATGATGGAAGT AATAAATATTATGCAGACTCCGTGAAGGGCCGAT TCACCATCTCCAGAGACAATTCCAAGAACACGCT GTATCTGCAAATGAACAGCCTGAGAGCCGAGGA CACGGCTGTGTATTACTGTGCGAGAGAACGTTAT AGCAGTGGCTGGTACGACTACGGTATGGACGTCT GGGGCCAAGGGACCACGGTCACCGTCTCCTCA |
| | | | SEQ ID NO: 27121 | SEQ ID NO: 31127 |
| | | AA | DIQMTQCPSSPPASVGDRVTITCRASQGIRNDLG WYQQKPGKAPKRLIYAASSLQSGVPSRFSGSGS GTEFILTISSVQREDFATYYCVMHYSYPRTFGQG TKVEIK | QVQLVESGGGVVQPGRSLRLSCAASGFTFSDYVM HWVRQAPGKGLEWVTVIWYDGSNKYYADSVKGR FTISRDNSKNTLYLQMNSLRAEDTAVYYCARERYS SGWYDYGMDVWGQGTTVTVSS |
| | | | SEQ ID NO: 27122 | SEQ ID NO: 31128 |

FIGURE 50

| | | | | |
|---|---|---|---|---|
| **iPS:436430** | **21-225_215A12** | NA | GACATCCAGATGACCCAGTCTCCATCCTCACTGTCTGCATCTGTAGGAGACAGAATCACCATCACTTGTCGGACGAGTCAGGACATTGGCAATTATTTAGCCTGGTTTCAGCAGAAACCAGGGAAAGCCCCTAAGTCCCTGATCTATGCTGCATCCAGTTTACAGAGTGGGGTCCCATCAAAGTTCAGCGGCAGTAGATCTGGGACAGATTTCACTCTCACCATCAGCAGCCTGCAGTCTGAAGATTTTGCAACTTATTACTGCCAACAGTATGTTACTTACCCGCTCACTTTCGGCGGAGGGACCAAGGTGGAGATCAAA | CAGGTGCAACTGGTGGAGTCTGGGGGAGGCGTGGTCCAGCCTGGGAGGTCCCTGAGACTCTCCTGTGCAGCGTCTGGACTCACCTTCAGTAGCTATGGCATGCACTGGGTCCGCCAGGCTCCAGGCAAGGGGCTGGAGTGGGTGGCAGTTATATGGTATGATGGAAGTAATGAACACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGGGGAGTGGGCTACTACGGTATGGACGTCTGGGGCCAAGGGACCACGGTCACCGTCTCCTCA |
| | | | SEQ ID NO: 27123 | SEQ ID NO: 31129 |
| | | AA | DIQMTQSPSSLSASVGDRITITCRTSQDIGNYLAWFQQKPGKAPKSLIYAASSLQSGVPSKFSGSRSGTDFTLTISSLQSEDFATYYCQQYVTYPLTFGGGTKVEIK | QVQLVESGGGVVQPGRSLRLSCAASGLTFSSYGMHWVRQAPGKGLEWVAVIWYDGSNEHYADSVKGRFTISRDNSKNTLYLQMNSLRAEDTAVYYCARDRGVGYYGMDVWGQGTTVTVSS |
| | | | SEQ ID NO: 27124 | SEQ ID NO: 31130 |
| **iPS:436432** | **21-225_215H12** | NA | GAAATTGTGTTGACGCAGTCTCCAGGCACCCTGTCTTTGTCTCCAGGGGAAAGAGCCACCCTCTCCTGCAGGGCCAGTCAGAGTGTTAGCAGCAGCTTCTTAGCTTGGTACCAGCAGAAACCTGGCCAGGCTCCCAGGCTCCTCATGTATGGTGCATCCAGCAGGGCCATTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGTTAGCTCACCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGCATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGTTAGACTACAGTAACTACGGGTGGTTCGACCCCTGGGGCCAGGGAACCCTGGTCACCGTCTCCTCA |
| | | | SEQ ID NO: 27125 | SEQ ID NO: 31131 |

FIGURE 50

| | | | | |
|---|---|---|---|---|
| | | AA | EIVLTQSPGTLSLSPGERATLSCRASQSVSSSFLAWYQQKPGQAPRLLMYGASSRAIGIPDRFSGSGSGTDFTLTISRLEPEDFAVYYCQQYVSSPLTFGGGTKVEIK | QVQLVESGGGVVQPGRSLRLSCAASGFTFSNYGMHWVRQAPGKGLEWVAVIWHDGSNKYYADSVKGRFTISRDNSKNTLYLQMNSLRAEDTAVYYCARLDYSNYGWFDPWGQGTLVTVSS |
| | | | SEQ ID NO: 27126 | SEQ ID NO: 31132 |
| iPS:436434 | 21-225_216B10 | NA | GAAATAGTGATGACGGAGTCTCCAGCCACCCTGTCTGTGTCTCCAGGGGAAAGAGCCACCCTCTCCTGCAGGGCCAGTCAGAGTGTTAACAACAACTTAGCCTGGTACCGGCAGAAACCTGGCCAGGCTCCCAGGCTCCTCATCTATGGTGCATCCACCAGGGCCACTGGTATCCCACCCAGGTTCAGTGGCAGTGGGTCTGGGACAGAGTTCACTCTCTCCATCAGCAGCCTGCAGTCTGAAGATTTTGCAGTTTATTACTGTCAGCAGTATAATGACTGGCCGTGCAGTTTTGGCCAGGGGACCAAACTGGAGATCAGA | CAGGTGCAGCTGGTGGAGTCGGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGCTATATGGTATGATGGAAGTAATAAATACTATGCAGACTCCGTGAAGGGCCGATTCACCATCTCCAGAGACAATTCCAAGCGCACGCTGTATCTGCAGATGAACAGCCTGAGAGCCGAGGACACGGCTGTGTATTACTGTGCGAGAGATCCCAACATAGTGGGAGCTACTTGGTTTGACTACTGGGGCCAGGGAACCCTGGTCACCGTCTCCTCA |
| | | | SEQ ID NO: 27127 | SEQ ID NO: 31133 |
| | | AA | EIVMTESPATLSVSPGERATLSCRASQSVNNNLAWYRQKPGQAPRLLIYGASTRATGIPPRFSGSGSGTEFTLSISSLQSEDFAVYYCQQYNDWPCSFGQGTKLEIR | QVQLVESGGGVVQPGRSLRLSCAASGFTFSSYGMHWVRQAPGKGLEWVAAIWYDGSNKYYADSVKGRFTISRDNSKRTLYLQMNSLRAEDTAVYYCARDPNIVGATWFDYWGQGTLVTVSS |
| | | | SEQ ID NO: 27128 | SEQ ID NO: 31134 |

FIGURE 50

| | | | | |
|---|---|---|---|---|
| **iPS:436436** | **21-225_216F10** | NA | GAAGTTGTGTTGACGCAGTCTCCAGGCACCCTGTCTTTGTCTCCAGGGGAAAGAGCCACCCTCTCCTGCAGGGCCAGTCAGAGTGTTAGCAGCAGCTTCTTAGCCTGGTACCAGCAGAAACCTGGCCAGGCTCCCAGGCTCCTCATCTATGGTACATCCACCAGGGCCACTGGCATCCCTGACAGGTTCAGTGGCAGTGGGTCTGGGACAGACTTCATTCTCACCATCAGCAGACTGGAGCCTGAAGATTTTGCAGTATATTACTGTCAACAGTATGATAGGTCACCATTCACTTTCGGCCCTGGGACCAAAGTGGATATCAAA | GAGGTACAGCTGGTGGAGTCTGGGGGAGGCTTGGTACAGCCTGGGGGGTCCCTGAGACTCTCCTGTGCAGCCTCTGGATTCTCCTTCAGAAGCTATAGCATGAACTGGGTCCGCCAGGCTCCAGGGAAGGGGCTGGAGTGGGTTTCATACATTACTGGTAGTAGTAGTACCATATACTACGCAGACTCTGTGAAGGGCCGATTCACCATCTCCAGAGACAATGCCAAGAACTCACTGTATCTGCAAATGAACAGCCTGAGAGACGAGGACACGGCTGTGTATTACTGTGCGAGATCGGGTTTAGCAGTGGAGGACTACTGGGGCCAGGGAACCCTGGTCACCGTCTCCTCA |
| | | | SEQ ID NO: 27129 | SEQ ID NO: 31135 |
| | | AA | EVVLTQSPGTLSLSPGERATLSCRASQSVSSSFLAWYQQKPGQAPRLLIYGTSTRATGIPDRFSGSGSGTDFILTISRLEPEDFAVYYCQQYDRSPFTFGPGTKVDIK | EVQLVESGGGLVQPGGSLRLSCAASGFSFRSYSMNWVRQAPGKGLEWVSYITGSSSTIYYADSVKGRFTISRDNAKNSLYLQMNSLRDEDTAVYYCARSGLAVEDYWGQGTLVTVSS |
| | | | SEQ ID NO: 27130 | SEQ ID NO: 31136 |
| **iPS:436438** | **21-225_216E8** | NA | GACATCCAGATGACCCAGTCTCCATCTTCCCTGCCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTTATCTATGCTGCATCCAGTTTGCAAAGTGGGGTCCCATCAAGGTTCAGCGGCAGTGGATCTGGGACAGAATTCATTCTCACAATCAGCAGCCTGCAGCCTGAAGATTTTGCAACTTATTACTGTCTAATGCATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGTCATGCACTGGGTCCGCCAGGCTCCAGGCAAGGGGCTGGAGTGGGTGACAGTTATATGGTATGATGGAAGTAATAAATATTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACGTTATAGCAGTGGCTGGTACGACTACGGTATGGACGTCTGGGGCCAAGGGACCACGGTCACCGTCTCCTCA |
| | | | SEQ ID NO: 27131 | SEQ ID NO: 31137 |

FIGURE 50

| | | | | |
|---|---|---|---|---|
| | | AA | DIQMTQSPSSLPASVGDRVTITCRASQGIRNDLGWYQQKPGKAPKRLIYAASSLQSGVPSRFSGSGSGTEFILTISSLQPEDFATYYCLMHYSYPRTFGQGTKVEIK | QVQLVESGGGVVQPGRSLRLSCAASGFTFSDYVMHWVRQAPGKGLEWVTVIWYDGSNKYYADSVKGRFTISRDNSKNTLYLQMNSLRAEDTAVYYCARERYSSGWYDYGMDVWGQGTTVTVSS |
| | | | SEQ ID NO: 27132 | SEQ ID NO: 31138 |
| **iPS:436440** | **21-225_216H12** | NA | GACATCCAGATGACCCTGTCTCCATCTTCCCTGCCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTTATCTATGGTGCATCCAGTTTGCAAAGTGGGGTCCCATCAAGGTTCAGCGGCAGTGGATCTGGGACAGAATTCATTCTCACAATCAGCAGCCTGCAGCCTGAAGATTTTGCAACTTATTACTGTGTAATGCATAATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGTCATGCACTGGGTCCGCCAGGCTCCAGGCAAGGGGCTGGAGTGGGTGACAGTTATATGGTATGATGGAAGTAATAAATATTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACGTTATAGCAGTGGCTGGTACGACTACGGTATGGACGTCTGGGGCCAAGGGACCACGGTCACCGTCTCCTCA |
| | | | SEQ ID NO: 27133 | SEQ ID NO: 31139 |
| | | AA | DIQMTLSPSSLPASVGDRVTITCRASQGIRNDLGWYQQKPGKAPKRLIYGASSLQSGVPSRFSGSGSGTEFILTISSLQPEDFATYYCVMHNSYPRTFGQGTKVEIK | QVQLVESGGGVVQPGRSLRLSCAASGFTFSDYVMHWVRQAPGKGLEWVTVIWYDGSNKYYADSVKGRFTISRDNSKNTLYLQMNSLRAEDTAVYYCARERYSSGWYDYGMDVWGQGTTVTVSS |
| | | | SEQ ID NO: 27134 | SEQ ID NO: 31140 |

FIGURE 50

| | | | | |
|---|---|---|---|---|
| **iPS:436448** | **21-225_217A3** | NA | GAAATTGTGCTGACTCAGTCTCCAGACTTTAAGTCTGTGACTCCAAAGGAGAAAGTCACCATCACCTGCCGGGCCAGTCAGAGCATTGGTAGTAGCTTACACTGGTACCAGCAGAAACCAGATCAGTCTCCAAAGCTCCTCGTCAAGTATGCTTCCCAGTCCTTCTCAGGGGTCCCCTCGAGGTTCAGTGGCAGTGGATCTGGGACAGATTTCACCCTCACCATCAACAGCCTGGAGGCTGAAGATGGTGCAACGTATTACTGTCATCAGAGTAGAAGTTTACCGTGGACGTTCGGCCAAGGGACCAAGGTGGAAATCAAA | GAGGTGCAACTGGTGGAGTCGGGGGGAGGCTTGGTACAGCCTGGGGGGTCCCTGAGACTCTCCTGTGCAGCCTCTGGATTCACCTTCAGTAGCTATAATATGAACTGGGTCCGCCAGGCTCCAGGGAAGGGCCTGGAGTGGGTTTCATACATTAGTAGTAGTCGTAATATCATATATTACGCAGACTCTGTGAAGGGCCGATTCACCATCTCCAGAGAAAATGCCAAGAACTCACTGTCTCTGCAAATGGACAGCCTGAGAGACGAGGACACGGCTGTGTATTACTGTGCGCGAGATGGCTCTTATAGCAGTGGCTGGTACTGGGGTTTTGACTACTGGGGCCAGGGCACCCTGGTCACCGTCTCCTCA |
| | | | SEQ ID NO: 27135 | SEQ ID NO: 31141 |
| | | AA | EIVLTQSPDFKSVTPKEKVTITCRASQSIGSSLHWYQQKPDQSPKLLVKYASQSFSGVPSRFSGSGSGTDFTLTINSLEAEDGATYYCHQSRSLPWTFGQGTKVEIK | EVQLVESGGGLVQPGGSLRLSCAASGFTFSSYNMNWVRQAPGKGLEWVSYISSSRNIIYYADSVKGRFTISRENAKNSLSLQMDSLRDEDTAVYYCARDGSYSSGWYWGFDYWGQGTLVTVSS |
| | | | SEQ ID NO: 27136 | SEQ ID NO: 31142 |
| **iPS:436450** | **21-225_217E5** | NA | GACATCCAGATGACCCAGTGTCCATCTTCCCCGCCTGCATTTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTTATCTGTGCTGCATCCAGTTTGCAAAGTGGGGTCCCATCAAGGTTCAGCGGCAGTGGATCTGGGACAGAATTCATTCTCACAATCAGCAGCCTGCAGCCTGAAGATTTTGCAACTTATTACTGTGTAATGCATAATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGTCATGCACTGGGTCCGCCAGGCTCCAGGCAAGGGGCTGGAGTGGGTGACAGTTATATGGTATGATGGAAGTAATAAATATTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACGTTATAGCAGTGGCTGGTACGACTACGGTATGGACGTCTGGGGCCAAGGGACCACGGTCACCGTCTCCTCA |
| | | | SEQ ID NO: 27137 | SEQ ID NO: 31143 |

FIGURE 50

| | | | | |
|---|---|---|---|---|
| | | AA | DIQMTQCPSSPPAFVGDRVTITCRASQGIRNDLG WYQQKPGKAPKRLICAASSLQSGVPSRFSGSGS GTEFILTISSLQPEDFATYYCVMHNSYPRTFGQG TKVEIK | QVQLVESGGGVVQPGRSLRLSCAASGFTFSDYVM HWVRQAPGKGLEWVTVIWYDGSNKYYADSVKGR FTISRDNSKNTLYLQMNSLRAEDTAVYYCARERYS SGWYDYGMDVWGQGTTVTVSS |
| | | | SEQ ID NO: 27138 | SEQ ID NO: 31144 |
| iPS:436452 | 21-225_217G5 | NA | GACATCCTGATGACCCAGTCTCCATCCTCACT GTCTGCATCTGTAGGAGACAGAGTCACCATCA CTTGTCGGGCGAGTCAGGGCATTGGCAATTAT TTAGCCTGGTTTCAGCAGAGACCAGGGAAAGC CCCTAAGTCCCTGATCTATGCTGCATCCAGTTT GCAAAGTGGGGTCCCATCAAAGTTCAGCGGCA CTCGATCTGGGACAGATTTCACTCTCACCATC AGCAGCCTGCAGCCTGACGATTTTGCAACTTA TTACTGCCAACAGTATGTTAATTACCCTCTCAC TTTCGGCGGAGGGACCAAGGTGGAGATCAAC | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCAATGGCAT GCACTGGGTCCGCCAGGCTCCAGGCAAGGGGCT GGAGTGGGTGGCAGTTATATGGTATGATGGAAGT AATAAAAACTATGCAGACTCCGTGAAGGGCCGA TTCACCATCTCCAGAGACAATTCCAAGAACACGC TGTATCTGCAAATGAACAGCCTGAGAGCCGAGG ACACGGCTGTGTATTACTGTGCGAGAGACTACGG TGTCGGGTACTACGGTCTGGACGTCTGGGGCCAA GGGACCACGGTCACCGTCTCCTCA |
| | | | SEQ ID NO: 27139 | SEQ ID NO: 31145 |
| | | AA | DILMTQSPSSLSASVGDRVTITCRASQGIGNYLA WFQQRPGKAPKSLIYAASSLQSGVPSKFSGTRSG TDFTLTISSLQPDDFATYYCQQYVNYPLTFGGGT KVEIN | QVQLVESGGGVVQPGRSLRLSCAASGFTFSSNGMH WVRQAPGKGLEWVAVIWYDGSNKNYADSVKGRF TISRDNSKNTLYLQMNSLRAEDTAVYYCARDYGV GYYGLDVWGQGTTVTVSS |
| | | | SEQ ID NO: 27140 | SEQ ID NO: 31146 |

FIGURE 50

| | | | | |
|---|---|---|---|---|
| **iPS:436454** | **21-225_217B10** | NA | GACATCCGGATGACCCAGTCTCCATCCTCACTGTCTGCATCTGTAGGAGACAGAGTCACCATCACTTGTCGGGCGAGTCAGGCCATTGGGAAACATTTAGCCTGGTTTCAGCAGAGGCCTGGGAAAGCCCCTAAGTCCCTGATCTATGCTGCATCCAGATTGCAAAGTGGGGTCCCATCAAAGTTCAGCGGCAGTGGATCTGGGACAGATTTCACTCTCACCATCAGCAGCGTGCAGCCTGAAGATCTTGCAACTTATTACCGTCAACACACCAGTAAATCTCCAGTGCAGCTTGTCGGAGGCACCAAGGTGGAGATCACA | CAGGTGCAGCTGGTGGAGTCTGGGGGAGGCGTGGTCCAGCCTGGGAGGTCCCTGAGACTCTCCTGTGCAGTGTCTGGAATCACCTTCAGTAGTTATGGCATGCACTGGGCCCGCCAGGCTCCAGGCAAGGGGCTGGAGTGGGTGGCAGTTATATGGTATGATGGAAGTAATAAAAACTATGAAGACTCCGTGAAGGGCCGATTCACCATCTCCAGAGACAACTCCAAGAACACGCTGTATCTGCAAATGAACAGCCTGAGAGCCGAGGACACGGCTGTGTATTACTGTGCGAGAGATGGGAGTTATGGTTACGACGGTATGGACGTCTGGGGCCAAGGGACCACGGTCACCGTCTCCTCA |
| | | | SEQ ID NO: 27141 | SEQ ID NO: 31147 |
| | | AA | DIRMTQSPSSLSASVGDRVTITCRASQAIGKHLAWFQQRPGKAPKSLIYAASRLQSGVPSKFSGSGSGTDFTLTISSVQPEDLATYYRQHTSKSPVQLVGGTKVEIT | QVQLVESGGGVVQPGRSLRLSCAVSGITFSSYGMHWARQAPGKGLEWVAVIWYDGSNKNYEDSVKGRFTISRDNSKNTLYLQMNSLRAEDTAVYYCARDGSYGYDGMDVWGQGTTVTVSS |
| | | | SEQ ID NO: 27142 | SEQ ID NO: 31148 |
| **iPS:436456** | **21-225_217G10** | NA | GACATCCAGATGACCCAGTCTCCATCTTCCCCGCCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTTATCTATGCTGCATCCAGTTTGCAAAGTGGGGTCCCATCAAGGTTCAGCGGCAGTGGATCTGGGACAGAATTCATTCTCACTATCAGCAGCCTGCAGCCTGAAGATTTTGCAACTTATTACTGTGTAATGCATAATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGTCATGCACTGGGTCCGCCAGGCTCCAGGCAAGGGGCTGGAGTGGGTGACAGTTATATGGTATGATGGAAGTAATAAATATTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACGTTATAGCAGTGGCTGGTACGACTACGGTATGGACGTCTGGGGCCAAGGGACCACGGTCACCGTCTCCTCA |
| | | | SEQ ID NO: 27143 | SEQ ID NO: 31149 |

FIGURE 50

| | | | | |
|---|---|---|---|---|
| | | AA | DIQMTQSPSSPPASVGDRVTITCRASQGIRNDLGWYQQKPGKAPKRLIYAASSLQSGVPSRFSGSGSGTEFILTISSLQPEDFATYYCVMHNSYPRTFGQGTKVEIK | QVQLVESGGGVVQPGRSLRLSCAASGFTFSDYVMHWVRQAPGKGLEWVTVIWYDGSNKYYADSVKGRFTISRDNSKNTLYLQMNSLRAEDTAVYYCARERYSSGWYDYGMDVWGQGTTVTVSS |
| | | | SEQ ID NO: 27144 | SEQ ID NO: 31150 |
| **iPS:436458** | **21-225_217H12** | NA | GACATCCAGATGACCCAGTCTCCATCTTCCCCGCCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTTATCTATGCTGCATCCAGTTTGCAAAGTGGGGTCCCATCAAGGTTCAGCGGCAGTGGATCTGGGACAGAATTCATTCTCACAATCAGCAGCCTGCAGCCTGAAGATTTTGCAACTTATTACTGTCTAATGCATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGTCATGCACTGGGTCCGCCAGGCTCCAGGCAAGGGGCTGGAGTGGGTGACAGTTATATGGTATGATGGAAGTAATAAATATTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACGTTATAGCAGTGGCTGGTACGACTACGGTATGGACGTCTGGGGCCAAGGGACCACGGTCACCGTCTCCTCA |
| | | | SEQ ID NO: 27145 | SEQ ID NO: 31151 |
| | | AA | DIQMTQSPSSPPASVGDRVTITCRASQGIRNDLGWYQQKPGKAPKRLIYAASSLQSGVPSRFSGSGSGTEFILTISSLQPEDFATYYCLMHYSYPRTFGQGTKVEIK | QVQLVESGGGVVQPGRSLRLSCAASGFTFSDYVMHWVRQAPGKGLEWVTVIWYDGSNKYYADSVKGRFTISRDNSKNTLYLQMNSLRAEDTAVYYCARERYSSGWYDYGMDVWGQGTTVTVSS |
| | | | SEQ ID NO: 27146 | SEQ ID NO: 31152 |

FIGURE 50

| | | | | |
|---|---|---|---|---|
| iPS:436462 | 21-225_218C4 | NA | GACATCCAGATGACCCAGTGTCCATCTTCCCCGCCTGCATTTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTTATCTATGCTGCATCCAGTTTGCAAAGTGGGGTCCCATCAAGGTTCAGCGGCAGTGGATCTGGGACAGAATTCATTCTCACAATCAGCAGCGTGCAGCGTGAAGATTTTGCAACTTATTACTGTGTAATGCATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGTCATGCACTGGGTCCGCCAGGCTCCAGGCAAGGGGCTGGAGTGGGTGACAGTTATATGGTATGATGGAAGTAATAAATATTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACGTTATAGCAGTGGCTGGTACGACTACGGTATGGACGTCTGGGGCCAAGGGACCACGGTCACCGTCTCCTCA |
| | | | SEQ ID NO: 27147 | SEQ ID NO: 31153 |
| | | AA | DIQMTQCPSSPPAFVGDRVTITCRASQGIRNDLGWYQQKPGKAPKRLIYAASSLQSGVPSRFSGSGSGTEFILTISSVQREDFATYYCVMHYSYPRTFGQGTKVEIK | QVQLVESGGGVVQPGRSLRLSCAASGFTFSDYVMHWVRQAPGKGLEWVTVIWYDGSNKYYADSVKGRFTISRDNSKNTLYLQMNSLRAEDTAVYYCARERYSSGWYDYGMDVWGQGTTVTVSS |
| | | | SEQ ID NO: 27148 | SEQ ID NO: 31154 |
| iPS:436464 | 21-225_219H1 | NA | GACATCCAGATGACCCAGTCTCCATCCTCACAGTCTGCATCTGTAGGAGACAGAGTCACCATCACTTGTCGGGCGAGTCAGGGCATTAGCAATTATTTAGACTGGTTTCAGCAGAAACCAGGGAAAGCCCCTAAGTCCCTGATCTATTCTGCATCCAATTTGCAAAGTGGGGTCCCATCAAAGTTCAGCGGCAGTAGATCTGGGACAGATTTCACTCTCACCATCAGCAGCCTGCAGCCTGAAGATTTTGCAACTTATTACTGCCAACACTATAGTAATTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGATATGGCATGCACTGGGTCCGCCAGGCTCCAGGCAAGGGGCTGGAGTGGGTGGCAGTTATATGGTATGATGGAAGTAATAAACACTATGCAGACTCCGTGAAGGGCCGATTCACCATCTCCAGAGACAATTCCAAGAATACGTTGTATCTGCAAATGAACAGTCTGAGAGGCGAGGACACGGCTGTGTATTACTGTGCGAGAGATCGGGGAGTGGGCTACAACGGTATGGACGTCTGGGGCCAAGGGACCACGGTCACCGTCTCCTCA |
| | | | SEQ ID NO: 27149 | SEQ ID NO: 31155 |

FIGURE 50

| | | | | |
|---|---|---|---|---|
| | | AA | DIQMTQSPSSQSASVGDRVTITCRASQGISNYLDWFQQKPGKAPKSLIYSASNLQSGVPSKFSGSRSGTDFTLTISSLQPEDFATYYCQHYSNYPLTFGGGTKVEIK | QVQLVESGGGVVQPGRSLRLSCAASGFTFSRYGMHWVRQAPGKGLEWVAVIWYDGSNKHYADSVKGRFTISRDNSKNTLYLQMNSLRGEDTAVYYCARDRGVGYNGMDVWGQGTTVTVSS |
| | | | SEQ ID NO: 27150 | SEQ ID NO: 31156 |
| iPS:436472 | 21-225_220E1 | NA | GAAATTGTGTTGACGCAGTCTCCAGGCACCCTGTCTTTGTCTCCAGGGGAAAGAGCCACCCTCTCCTGCAGGGCCAGTCAGAGTATTAGCCGCAGCCACTTAGTCTGGTACCAGCAGAAACCTAACCAGGCTCCCAGGCTCCTCCTCTATGTTACATCCAGCAGGGCCACTGGCATCCCAGACAGGTTTAGTGGCAGTGGGTCTGGGACAGACTTCACTCTCACCATCAGAAGTCTGGAGCCTGAAGATTTTGCAATGTATTACTGTCAGCAGTATGGTAGCTCACCGTGGACGTTCGGCCAAGGGACCAAGGTGGAAATCAAA | CAGGTGCAGCTGCAGGAGTCGGGCCCAGGACTGGTGAAGCCTTCGGAGACCCTGTCCCTCACCTGCACTGTCTCTGGTGGCTCCATCAGTACTTACTACTGGAGCTGGATCCGGCAGCCCCCAGGGAAGGGACTGGAGTGGATTGGGTATATCTATTACAGTGGGACCACCAACTACAACCCCTCCCTCAAGAGTCGAGTCACCATATCAGTAGACACGTCCAAGAACCAGTTCTCCCTGAAGCTGAGCTCTGTGACCGCTGCGGACACGGCCGTGTATTACTGTGCGAGAGACCAGCAGTGGCTGGTACGTGGGAGGGACAACTACTACTACGGTATGGACGTCTGGGGCCAAGGGACCACGGTCACCGTCTCCTCA |
| | | | SEQ ID NO: 27151 | SEQ ID NO: 31157 |
| | | AA | EIVLTQSPGTLSLSPGERATLSCRASQSISRSHLVWYQQKPNQAPRLLLYVTSSRATGIPDRFSGSGSGTDFTLTIRSLEPEDFAMYYCQQYGSSPWTFGQGTKVEIK | QVQLQESGPGLVKPSETLSLTCTVSGGSISTYYWSWIRQPPGKGLEWIGYIYYSGTTNYNPSLKSRVTISVDTSKNQFSLKLSSVTAADTAVYYCARDQQWLVRGRDNYYYGMDVWGQGTTVTVSS |
| | | | SEQ ID NO: 27152 | SEQ ID NO: 31158 |

FIGURE 50

| | | | | |
|---|---|---|---|---|
| **iPS:436480** | **21-225_220F8** | NA | GACATCCAGATGACCCTGTCTCCATCTTCCCCGCCTGCATTTGTAGGAGACAGAGTCACCATCACTCGCCGGGCAAGTCAGGGCATTAGAAATGATTTAGGCTGGTATCAGCAGAAACCAGGGAAAGCCCCTAAGCGCCTTATATGTGGTGCATCCAGTTTGCAAAGTGGGGTCCCATCAAGGTTCAGCGGCAGTGGATCTGGGACAGAATTCATTCTCACAATCAGCAGCCTGCAGCGTGAAGATTTTGCAACTTATTACTGTGTAATGCATAATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGTCATGCACTGGGTCCGCCAGGCTCCAGGCAAGGGGCTGGAGTGGGTGACAGTTATATGGTATGATGGAAGTAATAAATATTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAACGTTATAGCAGTGGCTGGTACGACTACGGTATGGACGTCTGGGGCCAAGGGACCACGGTCACCGTCTCCTCA |
| | | | SEQ ID NO: 27153 | SEQ ID NO: 31159 |
| | | AA | DIQMTLSPSSPPAFVGDRVTITRRASQGIRNDLGWYQQKPGKAPKRLICGASSLQSGVPSRFSGSGSGTEFILTISSLQREDFATYYCVMHNSYPRTFGQGTKVEIK | QVQLVESGGGVVQPGRSLRLSCAASGFTFSDYVMHWVRQAPGKGLEWVTVIWYDGSNKYYADSVKGRFTISRDNSKNTLYLQMNSLRAEDTAVYYCARERYSSGWYDYGMDVWGQGTTVTVSS |
| | | | SEQ ID NO: 27154 | SEQ ID NO: 31160 |
| **iPS:436488** | **21-225_221A6** | NA | GACATCCAGATGACCCAGTCTCCATCTTCCGTGTCTGCATCTGTAGGAGACAGAGTCACCATCACTTGTCGGGCGAGTCAGGGTATTAGCAGCTGGTTAGCCTGGTATCAGCAGAAACCAGGGAAAGCCCCTAAGCTCCTGATCTATACTGCATCCAATTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGGCTAACAGTTTCCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGACTTCTGGATACACCTTCACCGGCTACTATATGCACTGGGTGCGACAGGCCCCTGGACAAGGGCTTGAGTGGATGGGATGGATCCACCCTAACAGTGGTGGCACAAACTATGCACAGAAATTTCAGGGCAGGGTCACCCTGACCAGGGACACGTCCATCAGCACAGCCTACATGGACCTGAGCAGGCTGAGATCTGACGACACGGCCGTGTATTACTGTGCGAGAGATGGGACCAGCTCGTTTGACTACTGGGGCCAGGGAACCCTGGTCACCGTCTCCTCA |
| | | | SEQ ID NO: 27155 | SEQ ID NO: 31161 |

FIGURE 50

| | | | | |
|---|---|---|---|---|
| | | AA | DIQMTQSPSSVSASVGDRVTITCRASQGISSWLA WYQQKPGKAPKLLIYTASNLQSGVPSRFSGSGS GTDFTLTISSLQPEDFATYYCQQANSFPFTFGPGT KVDIK | QVQLVQSGAEVKKPGASVKVSCKTSGYTFTGYYM HWVRQAPGQGLEWMGWIHPNSGGTNYAQKFQGR VTLTRDTSISTAYMDLSRLRSDDTAVYYCARDGTS SFDYWGQGTLVTVSS |
| | | | SEQ ID NO: 27156 | SEQ ID NO: 31162 |
| iPS:436490 | 21-225_221F6 | NA | GACATCCAGATGACCCAGTCTCCATCCTCACT GTCTGGATCTGTAGGAGACAGAGTCACCATCA CTTGTCGGGCGAGTCAGGGCATTAGCAATTAT TTAGCCTGGTTTCAGCAGAAACCAGGGAAAGC CCCTAAATCCCTGATCTATGCTGCATCCAATTT ACAAAGTGGGGTCCCATCAAAGTTCAGCGGCA GTAGATCTGGGACAGATTTCACTCTCACCATC AGCAGCCTGCAGCCTGAAGATTTTGCAACTTA TTACTGCCAACAGTATATGACTTACCCGCTCA CTTTCGGCGGAGGGACCAGGGTGGAGATCAA A | CAGGTGCAGCTGGTGGAGTCTGGGGGAGGCGTG GTCCAGCCTGGGAGGTCCCTGAGACTCTCCTGTG CAGCGTCTGGATTCACCTTCAGTAGCAATGGCAT GCACTGGGTCCGCCAGGCTCCAGGCAAGGGGCT GGAGTGGGTGGCAGTTATATGGTACGATGGAAG TAATGAAAACTATGCAGACTCCGTGAAGGGCCG ATTCACCATCTCCAGAGACAATTCCAAGAACACG CTGTATCTGCAAATGAACAGCCTGAGAGCCGAG GACACGGCTGTGTATTACTGTGCGAGAGATCGGA CAGTGGGCTACAACGGTATGGACGTCTGGGGCC AAGGGACCACGGTCACCGTCTCCTCA |
| | | | SEQ ID NO: 27157 | SEQ ID NO: 31163 |
| | | AA | DIQMTQSPSSLSGSVGDRVTITCRASQGISNYLA WFQQKPGKAPKSLIYAASNLQSGVPSKFSGSRSG TDFTLTISSLQPEDFATYYCQQYMTYPLTFGGGT RVEIK | QVQLVESGGGVVQPGRSLRLSCAASGFTFSSNGMH WVRQAPGKGLEWVAVIWYDGSNENYADSVKGRF TISRDNSKNTLYLQMNSLRAEDTAVYYCARDRTVG YNGMDVWGQGTTVTVSS |
| | | | SEQ ID NO: 27158 | SEQ ID NO: 31164 |

FIGURE 50

| | | | | |
|---|---|---|---|---|
| **iPS:436494** | **21-225_221F12** | NA | GACATCCAGATGACCCAGTCTCCATCTTCCGTGTCTGCATCTGTAGGAGACAGAGTCACCATCACTTGTCGGGCGAGTCAGGGTATTAGCAGCTGGTTAGCCTGGTATCAGCAGAAACCAGGGAAAGCCCCTAAGCTCCTGATCTATACTGCATCCAATTTGCAAAGTGGGGTCCCATCAAGGTTCAGCGGCAGTGGATCTGGGACAGATTTCACTCTCACCATCAGCAGCCTGCAGCGTGAAGATTTTGCAACTTACTATTGTCAACAGGCTAACAGTTTCCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGACTTCTGGATACACCTTCACCGGCTACTATATGCACTGGGTGCGACAGGCCCCTGGACAAGGGCTTGAGTGGATGGGATGGATCCACCCTAACAGTGGTGGCACAAACTATGCACAGAAATTTCAGGGCAGGGTCACCCTGACCAGGGACACGTCCATCAGCACAGCCTACATGGACCTGAGCAGGCTGAGATCTGACGACACGGCCGTGTATTACTGTGCGAGAGATGGGACCAGCTCGTTTGACTACTGGGGCCAGGGAACCCTGGTCACCGTCTCCTCA |
| | | | SEQ ID NO: 27159 | SEQ ID NO: 31165 |
| | | AA | DIQMTQSPSSVSASVGDRVTITCRASQGISSWLAWYQQKPGKAPKLLIYTASNLQSGVPSRFSGSGSGTDFTLTISSLQREDFATYYCQQANSFPFTFGPGTKVDIK | QVQLVQSGAEVKKPGASVKVSCKTSGYTFTGYYMHWVRQAPGQGLEWMGWIHPNSGGTNYAQKFQGRVTLTRDTSISTAYMDLSRLRSDDTAVYYCARDGTSSFDYWGQGTLVTVSS |
| | | | SEQ ID NO: 27160 | SEQ ID NO: 31166 |
| **iPS:436496** | **21-225_222E1** | NA | GACATCCAGATGACCCAGTCTCCATCTTCCGTGTCTGCATCTGTAGGAGACAGAGTCACCATCACTTGTCGGGCGAGTCAGGGTATTAGCAGCTGGTTAGCCTGGTATCAGCAGAAACCAGGGAAAGCCCCTAAGCTCTTGATCTATACTGCATCCAATTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGGCTAACAGTTTCCCATTCACTTTCGGCCGTGGGACCAAAGTGGATATCAAA | CAGGTGCAGCTGGTGCAGTCTGGGGCTGAGGTGAAGAAGCCTGGGGCCTCAGTGAAGGTCTCCTGCAAGACTTCTGGATACACCTTCACCGGCTACTATATGCACTGGGTGCGACAGGCCCCTGGACAAGGGCTTGAGTGGATGGGATGGATCCACCCTAACAGTGGTGGCACAAACTATGCACAGAAATTTCAGGGCAGGGTCACCCTGACCAGGGACACGTCCATCAGCACAGCCTACATGGACCTGAGCAGGCTGAGATCTGACGACACGGCCGTGTATTACTGTGCGAGAGATGGGACCAGCTCGTTTGACTACTGGGGCCAGGGAACCCTGGTCACCGTCTCCTCA |
| | | | SEQ ID NO: 27161 | SEQ ID NO: 31167 |

FIGURE 50

| | | | | |
|---|---|---|---|---|
| | | AA | DIQMTQSPSSVSASVGDRVTITCRASQGISSWLAWYQQKPGKAPKLLIYTASNLQSGVPSRFSGSGSGTDFTLTISSLQPEDFATYYCQQANSFPFTFGRGTKVDIK | QVQLVQSGAEVKKPGASVKVSCKTSGYTFTGYYMHWVRQAPGQGLEWMGWIHPNSGGTNYAQKFQGRVTLTRDTSISTAYMDLSRLRSDDTAVYYCARDGTSSFDYWGQGTLVTVSS |
| | | | SEQ ID NO: 27162 | SEQ ID NO: 31168 |
| **iPS:436500** | **21-225_222H3** | NA | GACATCGTGATGACCCAGTCTCCAGACTCCCTGGCTGTGTCTCTGGGCGAGAGGGCCACCATCAACTGCAAGTCCAGCCAGAGTGTTTTGAAAAGTTCCAACCATAGGAACTACTTAGCTTGGTACCAACAGAAACCAGGGCAGCCTCCTCAGCTTCTCATTTACTGGGCATCTACCCGGGAAACCGGGGTCCCTGACCGATTCAGTGGCAGCGGGTCTGGGACAGATTTCACTCTCACCATCAGCAGCCTTCAGGCTGAAGATGTGTCAGTTTATTCCTGTCAGCAATATTCTTCTATTCCGTGGACGTTCGGCCAAGGGACCAAGGTGGAAATCAAT | CAGGTTCAGCTGGTGCAGTCTGGAGCTGAGGTGAAGAAGCCTGGGGCCTCAGTGAAGGTCTCCTGCAAGGCTTCTGGTTTCACCTTTACCAGCTATGGTATCAACTGGGTGCGACAGGCCCCTGGACAAGGGCTTGAGTGGATGGGATGGATCAGCGTTTACAATGGTAACACAAACTATGCACAGAAGCTCCAGGGCAGAGTCACCATGACCACAGACACATCCACGAGCACAGCCTACATGGAGCTGAGGAGCCTGAGATCTGACGACACGGCCGTGTATTACTGTGCGAGAGACTACTACTACGGTTTTGACGTCTGGGGCCAAGGGACCACGGTCACCGTCTCCTCA |
| | | | SEQ ID NO: 27163 | SEQ ID NO: 31169 |
| | | AA | DIVMTQSPDSLAVSLGERATINCKSSQSVLKSSNHRNYLAWYQQKPGQPPQLLIYWASTRETGVPDRFSGSGSGTDFTLTISSLQAEDVSVYSCQQYSSIPWTFGQGTKVEIN | QVQLVQSGAEVKKPGASVKVSCKASGFTFTSYGINWVRQAPGQGLEWMGWISVYNGNTNYAQKLQGRVTMTTDTSTSTAYMELRSLRSDDTAVYYCARDYYYGFDVWGQGTTVTVSS |
| | | | SEQ ID NO: 27164 | SEQ ID NO: 31170 |

FIGURE 50

| | | | | |
|---|---|---|---|---|
| **iPS:436502** | **21-225_222A11** | NA | GACATCCAGATGACCCAGTCTCCATCCTCACTGTCTGCATCTGTAGGAGACAGAGTCACCATCACTTGTCGGGCGAGTCAGGGCATTAGCAATTATTTGGCCTGGTTTCAGCAGAAACCAGGGAAAGCCCCTAAGTCCCTGATCTACGCTGCATCCAGTTTGCAAAGTGGGGTCCCATCAAAGTTCAGCGGCAGTGGATCTGGGACAGATTTCACTCTCACCATCAGTAGCCTGCAGCCTGAAGATTTTGCAACTTATTACTGCCTATATTATCTTAATTATCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAATGGGTGGCAGTTATATGGTATGATGGAAGTAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTATATTACTGTGCGAGAGATCGGGATGTCGGGTACAACGGTATGGACGTCTGGGGCCAAGGGACCACGGTCACCGTCTCCTCA |
| | | | SEQ ID NO: 27165 | SEQ ID NO: 31171 |
| | | AA | DIQMTQSPSSLSASVGDRVTITCRASQGISNYLAWFQQKPGKAPKSLIYAASSLQSGVPSKFSGSGSGTDFTLTISSLQPEDFATYYCLYYLNYPLTFGGGTKVEIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAVYYCARDRDVGYNGMDVWGQGTTVTVSS |
| | | | SEQ ID NO: 27166 | SEQ ID NO: 31172 |
| **iPS:436504** | **21-225_222H4** | NA | GACATCCAGATGACCCATTCTCCATCCTCCCTGTCTGCATCTGTAGGAGACAGAGTCACCATCACTTGCCGGGCAAGTCAGAACATTAGTAATTATGTTAATTGGTATCAGCAGAAACCAGGGAAAGCCCCTAAGTTCCTGATCTATACTGCATCGAGTTTGCAAAGTGGGGTCTCGTCACGGTTCAGTGGCAGTGGATCTGGGACAGATTTCACTCTCACCATCAGCAGTGTGCACCGTGACGATTTTGCAATTTACTATTGTCAGCAGTATTACTTTACCCCATTCACTTTCGGCCGTGGGACCAAAGTGGATATCAAA | GAGGTGCAGCTATTGGAGTCTGGGGGAGGCTTGGTACAGCCTGGGGGGTCCCTGAGACTCTCCTGTGCAGCCTCTGGCTTCACCTTTAGCAACTTTGCCATGAGTTGGGTCCGCCAGGCTCCAGGGAAGGGACTGGAGTGGGTCTCAAGTATTGTTGGTAGTGGTGGTCGCACGTACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTATTGTGCGAAAGACCCTTATCGTGTAGCAGTGGCTGGGGCCTTTGACTACTGGGGCCAGGGAACCCTGATCACCGTCTCCTCA |
| | | | SEQ ID NO: 27167 | SEQ ID NO: 31173 |

FIGURE 50

| | | | | |
|---|---|---|---|---|
| | | AA | DIQMTHSPSSLSASVGDRVTITCRASQNISNYVNWYQQKPGKAPKFLIYTASSLQSGVSSRFSGSGSGTDFTLTISSVHRDDFAIYYCQQYYFTPFTFGRGTKVDIK | EVQLLESGGGLVQPGGSLRLSCAASGFTFSNFAMSWVRQAPGKGLEWVSSIVGSGGRTYYADSVKGRFTISRDNSKNTLYLQMNSLRAEDTAVYYCAKDPYRVAVAGAFDYWGQGTLITVSS |
| | | | SEQ ID NO: 27168 | SEQ ID NO: 31174 |
| iPS:436506 | 21-225_222C7 | NA | GAAATTGTGTTGACGCAGTCTCCAGGCACCCTGTCTTTGTCTCCAGGGGAAAGAGCCACCCTCTCCTGCAGGGCCAGTCAGAGTGTTTACAGCAACTACTTAGCCTGGTACCAGCAGAAACCTGGCCAGGCTCCCAGGCTCCTCATCTATGGTGCATCAAGCAGGGCCACTGGCATCCCAGACAGGTTCGGTGGCAGTGGGTCTGGGACAGACTTCACTCTCGCCATAAGCAGACTGGAGCCTGAAGACTTTACAATATATTACTGTCAGCAGTATGAAGACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGGTCGCTACTGGAGCTGGATCCGCCAGCCCCCAGGGAAGGGGCTGGAGTGGATTGGGGAAATCAATCATAGTGGAAGCGCCAACTACAACCCGTCCCTCAAGAGTCGAGTCACCATATCAGTAGACACGTCCAAGAACCAGTTCTCCCTGAAGCTGAGTTCTGTGACCGCCGCGGACACGGCTGTATATTACTGTGCGAGAGACTACGGCGCCCTTGATTTCTGGGGCCAAGGGACAATGGTCACCGTCTCTTCA |
| | | | SEQ ID NO: 27169 | SEQ ID NO: 31175 |
| | | AA | EIVLTQSPGTLSLSPGERATLSCRASQSVYSNYLAWYQQKPGQAPRLLIYGASSRATGIPDRFGGSGSGTDFTLAISRLEPEDFTIYYCQQYEDSPWTFGQGTKVEIK | QVQLQQWGAGLLKPSETLSLTCAVYGGSFSGRYWSWIRQPPGKGLEWIGEINHSGSANYNPSLKSRVTISVDTSKNQFSLKLSSVTAADTAVYYCARDYGALDFWGQGTMVTVSS |
| | | | SEQ ID NO: 27170 | SEQ ID NO: 31176 |

FIGURE 50

| | | | | |
|---|---|---|---|---|
| **iPS:436508** | **21-225_222F7** | NA | GACATCCAGATGACCCAGTCTCCATCTTCCGTGTCTGCATCTGTAGGAGACAGAGTCACCATCACTTGTCGGGCGAGTCAGGGTATTAGCAGCTGGTTAGCCTGGTATCAGCAGAAACCAGGGAAAGCCCCTAAGCTCCTGATCTATACTGCATCCAATTTGCAAAGTGGGGTCCCATCAAGGTTCAGCGGCAGTGGATCTGGGACAGATTTCACTCTCACCATCAGCAGCGTGCAGCCTGAAGATTTTGCAACTTACTATTGTCAACAGGCTAACAGTTTCCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGACTTCTGGATACACCTTCACCGGCTACTATATGCACTGGGTGCGACAGGCCCCTGGACAAGGGCTTGAGTGGATGGGATGGATCCACCCTAACAGTGGTGGCACAAACTATGCACAGAAATTTCAGGGCAGGGTCACCCTGACCAGGGACACGTCCATCAGCACAGCCTACATGGACCTGAGCAGGCTGAGATCTGACGACACGGCCGTGTATTACTGTGCGAGAGATGGGACCAGCTCGTTTGACTACTGGGGCCAGGGAACCCTGGTCACCGTCTCCTCA |
| | | | SEQ ID NO: 27171 | SEQ ID NO: 31177 |
| | | AA | DIQMTQSPSSVSASVGDRVTITCRASQGISSWLAWYQQKPGKAPKLLIYTASNLQSGVPSRFSGSGSGTDFTLTISSVQPEDFATYYCQQANSFPFTFGPGTKVDIK | QVQLVQSGAEVKKPGASVKVSCKTSGYTFTGYYMHWVRQAPGQGLEWMGWIHPNSGGTNYAQKFQGRVTLTRDTSISTAYMDLSRLRSDDTAVYYCARDGTSSFDYWGQGTLVTVSS |
| | | | SEQ ID NO: 27172 | SEQ ID NO: 31178 |
| **iPS:436510** | **21-225_222H8** | NA | GACATCCAGATGACCCATTCTCCATCTTCCCTGTCTGCATCTGTAGGAGACAGAGTCACCATCACTTGCCGGGCAAGTCAGAACATTAGTAATTATGTTAATTGGTATCAGCAGAAACCAGGGAAAGCCCCTAAGTTCCTGATCTATATTGCATCGAGTTTGCAAAGTGGGGTCTCGTCACGGTTCAGTGGCAGTGGATCTGGGACAGATTTCACTCTCACCATCAGCAGTGTGCACCGTGACGATTTTGCAATTTACTACTGTCAGCAGTATTACTTTACCCCATTCACTTTCGGCCGTGGGACCAAAGTGGATATCAAA | GAGGTGCAGCTATTGGAGTCTGGGGGAGGCTTGGTACAGCCTGGGGGGTCCCTGAGACTCTCCTGTGCAGCCTCTGGCTTCACCTTTAGCAACTTTGCCATGAGTTGGGTCCGCCAGGCTCCAGGGAAGGGACTGGAGTGGGTCTCAAGTATTGTTGGTAGTGGTGGTCGCACGTACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTATTGTGCGAAAGACCCTTATCGTGTAGCAGTGGCTGGGGCCTTTGACTACTGGGGCCAGGGAACCCTGATCACCGTCTCCTCA |
| | | | SEQ ID NO: 27173 | SEQ ID NO: 31179 |

FIGURE 50

| | | | | |
|---|---|---|---|---|
| | | AA | DIQMTHSPSSLSASVGDRVTITCRASQNISNYVNWYQQKPGKAPKFLIYIASSLQSGVSSRFSGSGSGTDFTLTISSVHRDDFAIYYCQQYYFTPFTFGRGTKVDIK | EVQLLESGGGLVQPGGSLRLSCAASGFTFSNFAMSWVRQAPGKGLEWVSSIVGSGGRTYYADSVKGRFTISRDNSKNTLYLQMNSLRAEDTAVYYCAKDPYRVAVAGAFDYWGQGTLITVSS |
| | | | SEQ ID NO: 27174 | SEQ ID NO: 31180 |
| iPS:436514 | 21-225_222D10 | NA | GACATCCAGATGACCCAGTCTCCATCCTCACTGTCTGCATCTGTAGGAGACAGAGTCACCATCACTTGTCGGGCGAGTCAGGGCATTAGCAATTATTTGGCCTGGTTTCAGCAGAAACCAGGGAAAGCCCCTAAGTCCCTGATCTACGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCTACATTATCTTAATTACCCGCTCACTTTCGGCGGAGGGACCAGGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGGGATGTCGGGTACAACGGTATGGACGTCTGGGGCCAAGGGACCACGGTCACCGTCTCCTCA |
| | | | SEQ ID NO: 27175 | SEQ ID NO: 31181 |
| | | AA | DIQMTQSPSSLSASVGDRVTITCRASQGISNYLAWFQQKPGKAPKSLIYAASSLQSGVPSKFSGSGSGTDFTLTISSLQPEDFATYYCLHYLNYPLTFGGGTRVEIK | QVQLVESGGGVVQPGRSLRLSCAASGFTFSSYGMHWVRQAPGKGLEWVAVIWYDGSNKNYADSVKGRFTISRDNSKNTLYLQMNSLRAEDTAVYYCARDRDVGYNGMDVWGQGTTVTVSS |
| | | | SEQ ID NO: 27176 | SEQ ID NO: 31182 |

FIGURE 50

| | | | | |
|---|---|---|---|---|
| iPS:436516 | 21-225_222C12 | NA | GACATCCAGATGACTCAGTGTCCATCTTCCGTGTCTGCATCTGTAGGAGACAGAGTCACCATCACTTGTCGGGTGAGTCAGGGTATTAGCAGCTGGTTAGCCTGGTATCAGCAGAAACCAGGGAAAGCCCTTAAGCTCGTGATCTATACTGCATCCAATTTGCAAAGTGGGGTCCCATCAAGGTTCAGCGGCAGTGGATCTGGGACAGATTTCACTCTCACCATCAGCAGCGTGCAGCGTGAAGATTTTGCAACTTACTATTGTCAACAGGATAACAGTTTCCCATTCACTTTCGGCCGAGGGACCAAAGTGGATATCAAA | CAGGTGCAGCTGGTGCAGTCTGGGGCTGAGGTGAAGAAGCCTGGGGCCTCAGTGAAGGTCTCCTGCAAGACTTCTGGATACACCTTCACCGGCTACTATATGCACTGGGTGCGACAGGCCCCTGGACAAGGGCTTGAGTGGATGGGATGGATCCACCCTAACAGTGGTGGCACAAACTATGCACAGAAATTTCAGGGCAGGGTCACCCTGACCAGGGACACGTCCATCAGCACAGCCTACATGGACCTGAGCAGGCTGAGATCTGACGACACGGCCGTGTATTACTGTGCGAGAGATGGGACCAGCTCGTTTGACTACTGGGGCCAGGGAACCCTGGTCACCGTCTCCTCA |
| | | | SEQ ID NO: 27177 | SEQ ID NO: 31183 |
| | | AA | DIQMTQCPSSVSASVGDRVTITCRVSQGISSWLAWYQQKPGKALKLVIYTASNLQSGVPSRFSGSGSGTDFTLTISSVQREDFATYYCQQDNSFPFTFGRGTKVDIK | QVQLVQSGAEVKKPGASVKVSCKTSGYTFTGYYMHWVRQAPGQGLEWMGWIHPNSGGTNYAQKFQGRVTLTRDTSISTAYMDLSRLRSDDTAVYYCARDGTSSFDYWGQGTLVTVSS |
| | | | SEQ ID NO: 27178 | SEQ ID NO: 31184 |
| iPS:436520 | 21-225_223G10 | NA | GACATCGTGATGACCCAGTCTCCAGACTCCCTGGCTGTGTCTCTGGGCGAGAGGGCCACCATCAACTGCAAGTCCAGCCAGAGTATTTTACTCAGCTCCAACAATAAGAACTACTTAGCTTGGCACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCTGCAATATTTTAGTACTCCGTGGACGTTCGGCCAAGGGACCAAGGTGGAAATCAAA | CAGGTTCAGCTGGTGCAGTCTGGAGCTGAGGTGAAGAAGCCTGGGGCCTCAGTGAAGGTCTCCTGCAAGGCTTCTGGTTTCACCTTTACCAGCTATGGTATCAACTGGGTGCGACAGGCCCCTGGACAAGGACTTGAGTGGATGGGATGGATCAGCGTTTACAGTGGTAACACAAACTATGCACAGAAGCTCCAGGGCAGAGTCACCATGACCACAGATACATCCACGAGCACAGCCTACATGGAGCTGAGGAGCCTGAGATCTGACGACACGGCCGTGTATTACTGTGCGAGAGACTACTACTACGGTATGGACGTCTGGGGCCAAGGGACCACGGTCACCGTCTCCTCA |
| | | | SEQ ID NO: 27179 | SEQ ID NO: 31185 |

FIGURE 50

| | | | | |
|---|---|---|---|---|
| | | AA | DIVMTQSPDSLAVSLGERATINCKSSQSILLSSNNKNYLAWHQQKPGQPPKLLIYWASTRESGVPDRFSGSGSGTDFTLTISSLQAEDVAVYYCLQYFSTPWTFGQGTKVEIK | QVQLVQSGAEVKKPGASVKVSCKASGFTFTSYGINWVRQAPGQGLEWMGWISVYSGNTNYAQKLQGRVTMTTDTSTSTAYMELRSLRSDDTAVYYCARDYYYGMDVWGQGTTVTVSS |
| | | | SEQ ID NO: 27180 | SEQ ID NO: 31186 |
| **iPS:436522** | **21-225_223H10** | NA | GACATCCAGATGACCCAGTCTCCATCCTCACTGTCTGCATCTGTAGGAGACAGAGTCACCATCACTTGTCGGGCGAGTCAGGGCATTAGTAATTATTTGGCCTGGTTTCAGCAGAAACCAGGGAAAGCCCCTAAGTCCCTGATCTACGCTGCATCCAATTTGCAAAGTGGGGTCCCATCAAAGTTCAGCGGCAGTGGGTCTGGGACAGATTTCACTCTCACCATCAGCAGCCTGCAGCCTGAAGATTTTGCAACTTATTACTGCCTACATTATCTTAATTACCCACTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAAAACTATGCAGACTCCGTGAAGGGCCGATTCACCATCTCCAGAGACCATTCCAAGAACACGCTGTATCTGCAAATGAACAGCCTGAGAGCCGAGGACACGGCTGTGTATTACTGTGCGAGAGATCGGGATGTCGGGTACAACGGTATGGACGTCTGGGGCCAAGGGACCACGGTCACCGTCTCCTCA |
| | | | SEQ ID NO: 27181 | SEQ ID NO: 31187 |
| | | AA | DIQMTQSPSSLSASVGDRVTITCRASQGISNYLAWFQQKPGKAPKSLIYAASNLQSGVPSKFSGSGSGTDFTLTISSLQPEDFATYYCLHYLNYPLTFGGGTKVEIK | QVQLVESGGGVVQPGRSLRLSCAASGFTFSSYGMHWVRQAPGKGLEWVAVIWYDGSNKNYADSVKGRFTISRDHSKNTLYLQMNSLRAEDTAVYYCARDRDVGYNGMDVWGQGTTVTVSS |
| | | | SEQ ID NO: 27182 | SEQ ID NO: 31188 |

FIGURE 50

| | | | | |
|---|---|---|---|---|
| **iPS:436526** | **21-225_224A1** | NA | GACATCCAGATGACCCAGTCTCCATCCTCCCTGTCTGCATCTGTAGGAGACAGAGTCACCATCACTTGCCGGGCAAGTCAGGGCATTGAAAATGATTTAGGCTGGTATCAGCAGAAGCCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCGCTCACTTTCGGCGGTGGGACCAAGGTGGAGATCAAA | GAGGTGCAGGTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGCAGAGGCGGCAGCACATACTACGCAGACGCCGTGAAGGGCCGGTTCACCATCTCCAGAGACAATTCCAAGAACACGCTGTATCTGCAAATGAACAGCCTGAGAGCCGAGGACACGGCCGTATATTACTGCGCGAAAGGCTCCTACGATAGTAGTGGTTATTACCACTACTTAGACCACTGGGGCCAGGGAACCCTGGTCACCGTCTCCTCA |
| | | | SEQ ID NO: 27183 | SEQ ID NO: 31189 |
| | | AA | DIQMTQSPSSLSASVGDRVTITCRASQGIENDLGWYQQKPGKAPKRLIYAASSLQSGVPSRFSGSGSGTEFTLTISSLQPEDFATYYCLQHNSYPLTFGGGTKVEIK | EVQVLESGGGLVQPGGSLRLSCAASGFTFSSYAMSWVRQAPGKGLEWVSAISGRGGSTYYADAVKGRFTISRDNSKNTLYLQMNSLRAEDTAVYYCAKGSYDSSGYYHYLDHWGQGTLVTVSS |
| | | | SEQ ID NO: 27184 | SEQ ID NO: 31190 |
| **iPS:436528** | **21-225_224B1** | NA | GACATCCAGATGACCCAGTCTCCATCCTCCCTGTCTGCATCTGTGGGAGACAGAGTCACCATCACTTGCCGGGCAAGTCAGGGCATTAGTAATGATTTAGGCTGGTATCAGCAGAAGCCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATCACTGTCTACAGCATAATAGTTATCCTCCTACTTTCGGCGGAGGGACCAAGGTGGAGATCAAA | GAGGTGCAGGTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTAGTGGTGGTAACACATACTACGCAGACTCCGTGAAGGGCCGGTTCACCATCTCCAGAGACAATTCCAAGAACACGCTGTATCTGCACATGAACAGCCTGAGAGCCGAGGACACGGCCGTATATTACTGTGCGGGAGAGGGGGGCTACTACTATTACTACGGTGTGGACGTCTGGGGCCAAGGGACCACGGTCACCGTCTCCTCA |
| | | | SEQ ID NO: 27185 | SEQ ID NO: 31191 |

FIGURE 50

| | | | | |
|---|---|---|---|---|
| | | AA | DIQMTQSPSSLSASVGDRVTITCRASQGISNDLGWYQQKPGKAPKRLIYAASSLQSGVPSRFSGSGSGTEFTLTISSLQPEDFATYHCLQHNSYPPTFGGGTKVEIK | EVQVLESGGGLVQPGGSLRLSCAASGFTFSSYAMSWVRQAPGKGLEWVSAISGSGGNTYYADSVKGRFTISRDNSKNTLYLHMNSLRAEDTAVYYCAGEGGYYYYYGVDVWGQGTTVTVSS |
| | | | SEQ ID NO: 27186 | SEQ ID NO: 31192 |
| iPS:436534 | 21-225_224F1 | NA | GCCATCCAGATGACCCAGTCTCCATCCTCCCTGTCTGCATCTGTAGGAGACAGAGTCACCATCACTTGCCGGGCAAGTCAGGGCATTAGAGATGATTTAGGCTGGTATCAGCAGAAACCAGGGAAAGCCCCTAAGCGCCTGATCTATGCTGCATCCAGTTTGCAAAGTGGGGTCCCGTCAAGGATCAGCGGCAGTGGATCTGGGACAGAATTCACTCTCACAATCAGCAGCCTGCAGCCTGAAGATTTTGCAATTTATTACTGTCTACAGCATTATAGTTACCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATATCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGAGGTATAGCAGCAACTGGTACGACTACGGTATGGACGTCTGGGGCCAAGGGACCACGGTCACCGTCTCCTCA |
| | | | SEQ ID NO: 27187 | SEQ ID NO: 31193 |
| | | AA | AIQMTQSPSSLSASVGDRVTITCRASQGIRDDLGWYQQKPGKAPKRLIYAASSLQSGVPSRISGSGSGTEFTLTISSLQPEDFAIYYCLQHYSYPRTFGQGTKVEIK | QVQLVESGGGVVQPGRSLRLSCAASGFTFSSYIMHWVRQAPGKGLEWVAVIWYDGSNKYYADSVKGRFTISRDNSKNTLYLQMNSLRAEDTAVYYCARERYSSNWYDYGMDVWGQGTTVTVSS |
| | | | SEQ ID NO: 27188 | SEQ ID NO: 31194 |

FIGURE 50

| | | | | |
|---|---|---|---|---|
| **iPS:436536** | **21-225_224G1** | NA | GATATTGTGATGACCCAGACTCCACTCTCTCT<br>GTCCGTCACCCCTGGACAGCCGGCCTCCATCT<br>CCTGCAAGTCTGGTCAGAGCCTCCTGCATAGT<br>GATGGAAAGACCTTTTTGTCTTGGTACCTGCA<br>GAAGCCAGGCCAGCCTCCACAGCTCCTGATCT<br>ATGAAATTTCCAACCGGTTCTCTGGAGTGCCA<br>GATAGGTTCAGTGGCAGCGGGTCAGGGACAG<br>ATTTCACACTGAAAATCAGCCGGGTGGAGGCT<br>GAGGATGTTGGGATTTTTACTGCATGCAAAG<br>TACACAGCTTCCTCGGACGTTCGGCCAAGGGA<br>CCAAGGTGGAAATCAAA | CAGGTGCAGCTGGTGCAGTCTGGGGCTGAGGTG<br>AAGAAGCCTGGGGCCTCAGTGAAGGTCTCCTGC<br>AAGGCTTCTGGATACACCTTCACCGGCTACTATA<br>TACACTGGGTGCGACAGGCCCCTGGACAAGGGC<br>TTGAGTGGATGGGATGGATCAACCCTTACAGTGG<br>TGACACAAACTATGCACAGAAGTTTCAGGGCAG<br>AGTCACCATGACCAGGGACACGTCCATCAGCAC<br>AGCCTACATGGAACTGAGCAGGCTGAGATTTGA<br>CGACACGGCCGTGTTTTACTGTGCGAGAGATTGG<br>GGTGGCTACAGTTCTTACTACTACGGTATGGACG<br>TCTGGGGCCAAGGGACCACGGTCACCGTCTCTTC<br>A |
| | | | SEQ ID NO: 27189 | SEQ ID NO: 31195 |
| | | AA | DIVMTQTPLSLSVTPGQPASISCKSGQSLLHSDG<br>KTFLSWYLQKPGQPPQLLIYEISNRFSGVPDRFSG<br>SGSGTDFTLKISRVEAEDVGIFYCMQSTQLPRTF<br>GQGTKVEIK | QVQLVQSGAEVKKPGASVKVSCKASGYTFTGYYIH<br>WVRQAPGQGLEWMGWINPYSGDTNYAQKFQGRV<br>TMTRDTSISTAYMELSRLRFDDTAVFYCARDWGG<br>YSSYYYGMDVWGQGTTVTVSS |
| | | | SEQ ID NO: 27190 | SEQ ID NO: 31196 |
| **iPS:436538** | **21-225_224C3** | NA | GACATCCAGATGACCCAGTCTCCATCCTCCCT<br>GTCTGCATCTGTAGGAGACAGAGTCACCATCA<br>CTTGCCGGGCAAGTCAGGACATTAGAAATGAT<br>TTAGGCTGGTATCAGCAGAAACCAGGGAAAG<br>CCCCTAAGCGCCTGATCTATGCTACATCCAGT<br>TTGCAAAGTGGGGTCCCATCAAGGTTCAGCGG<br>CAGTGGATCTGGGACAGAATTCACTCTCACAA<br>TCAGCAGCCTGCAGCCTGAAGATTTTGCAACT<br>TATTACTGTCTACAGCATAATAGTTACCCGCTC<br>ACTTTCGGCGGAGGGACCAAGGTGGAGATCA<br>AA | CAGCTGCAGCTGCAGGAGTCGGGCCCAGGACTG<br>GTGAAGTCTTCGGAGACCCTGTCCCTCACCTGCA<br>CTGTCTCTGGCGGCTCCATCAGCAGAAGTAGTTA<br>CTACTGGGGCTGGATCCGCCAGCCCCCAGGGAA<br>GGGCCTGGAGTGGATTGGGAATATCTATTATAGT<br>GGGAGCACCTACTACAACCCGTCCCTCAAGAGTC<br>GAGTCACCATATCCGTAGACACGTCCAAGAACC<br>AGTTCTCCCTGAAGCTGAGCTCTGTGACCGCCGC<br>AGACACGGCTGTGTATTACTGTGCGAGACAGGGT<br>CGGGACTGGGGTGTTGACTACGGGGGCCAGGGA<br>ACCCTAGTCACCGTCTCCTCA |
| | | | SEQ ID NO: 27191 | SEQ ID NO: 31197 |

FIGURE 50

| | | | | |
|---|---|---|---|---|
| | | AA | DIQMTQSPSSLSASVGDRVTITCRASQDIRNDLGWYQQKPGKAPKRLIYATSSLQSGVPSRFSGSGSGTEFTLTISSLQPEDFATYYCLQHNSYPLTFGGGTKVEIK | QLQLQESGPGLVKSSETLSLTCTVSGGSISRSSYYWGWIRQPPGKGLEWIGNIYYSGSTYYNPSLKSRVTISVDTSKNQFSLKLSSVTAADTAVYYCARQGRDWGVDYGGQGTLVTVSS |
| | | | SEQ ID NO: 27192 | SEQ ID NO: 31198 |
| iPS:436540 | 21-225_224F3 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAACAGAAACCAGGGAAAGCCCCTGAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTTCAGCATTATAATTACCCTCGGGCGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCAGCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGAGGTATAGCAGCAGCTGGTACGACTACGGTATGGACGTCTGGGGCCAAGGGACCACGGTCACCGTCTCCTCA |
| | | | SEQ ID NO: 27193 | SEQ ID NO: 31199 |
| | | AA | DIQMTQSPSSLSASVGDRVTITCRASQGIRNDLGWYQQKPGKAPERLIYAASSLQSGVPSRFSGSGSGTEFTLTISSLQPEDFATYYCLQHYNYPRAFGQGTKVEIK | QVQLVESGGGVVQPGRSLRLSCAASGFSFSSYGMHWVRQAPGKGLEWVAVIWYDGSNKYYADSVKGRFTISRDNSKNTLYLQMNSLRAEDTAVYYCARERYSSSWYDYGMDVWGQGTTVTVSS |
| | | | SEQ ID NO: 27194 | SEQ ID NO: 31200 |

FIGURE 50

| | | | | |
|---|---|---|---|---|
| **iPS:436544** | **21-225_224H5** | NA | GACATCGTGATGACCCAGTCTCCAGACTCCCTGGCTGTGTCTCTGGGCGAGAGGGCCACCATCAACTGCAAGTCCAGCCAGAGTGTTTTATACAGCTCCAACAATTTCAACTACTTAACTTGGTATCAGCAGAAACCAGGACAGCCTCCTAAGTTGCTCATTTACTGGGCATCTACCCGGGAATCCGGGGTCCCTGACCGATTCAGTGGCAGCGGGTCTGGGACAGATTTCACTCTCACCATCAACAGCCTGCAGGCTGAAGATGTGGCAGTTTATTACTGTCAGCAATATTATAGTACTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGAGTTCCAGTGGCTGGAACTGGTTCGACCCCTGGGGCCAGGGAACCCTGGTCACCGTCTCCTCA |
| | | | SEQ ID NO: 27195 | SEQ ID NO: 31201 |
| | | AA | DIVMTQSPDSLAVSLGERATINCKSSQSVLYSSNNFNYLTWYQQKPGQPPKLLIYWASTRESGVPDRFSGSGSGTDFTLTINSLQAEDVAVYYCQQYYSTPPTFGQGTKVEIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYYCASSSGWNWFDPWGQGTLVTVSS |
| | | | SEQ ID NO: 27196 | SEQ ID NO: 31202 |
| **iPS:436546** | **21-225_224D6** | NA | GACATCCAGATGACCCAGTCTCCATCTTCCGTGTCTGCATCTGTAGGAGACAGAGTCACCATCACTTGTCGGGCGAGTCAGGGTATTAGCAGCTGGTTAGCCTGGTATCAGCAGAAACCAGGGAAAGCCCCTAAGCTCCTGATCTATGCTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGGCTAACAGTTTCCCGTGGACGTTCGGCCCAGGGACCAAGGTGGAAATCAAA | GAGGTGCAGGTGTTGGAGTCTGGGGGAGGCTTGGTACAGCCTGGGGGGTCCCTGAGACTCTCCTGTGCAGCCTCTGGATCCACCTTTAGCAGCGATGCCATGAGCTGGGTCCGCCAGGCTCCAGGGAAGGGGCTGGAGTGGGTCTCAGCTATTAGTGGTAGTGGTGATAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAAGTCTATAGTGCCTACGATTCTCACTGGTTCGACCCCTGGGGCCAGGGAACCCTGGTCACCGTCTCCTCA |
| | | | SEQ ID NO: 27197 | SEQ ID NO: 31203 |

FIGURE 50

| | | | | |
|---|---|---|---|---|
| | | AA | DIQMTQSPSSVSASVGDRVTITCRASQGISSWLAWYQQKPGKAPKLLIYAASSLQSGVPSRFSGSGSGTDFTLTISSLQPEDFATYYCQQANSFPWTFGPGTKVEIK | EVQVLESGGGLVQPGGSLRLSCAASGSTFSSDAMSWVRQAPGKGLEWVSAISGSGDNTFYADSVKGRFTISRDNSKNTLYLQMNSLRAEDTAVYYCAKVYSAYDSHWFDPWGQGTLVTVSS |
| | | | SEQ ID NO: 27198 | SEQ ID NO: 31204 |
| **iPS:436548** | **21-225_224A7** | NA | GATATTGTGATGACCCAGACTCCACTCTCTCTGTCCGTCACCCCTGGACAGCCGGCCTCCATCTCCTGCAAGTCTAGTCAGAGCCTCCTGCATAGTGATGGAAAGACCTTTTTGTATTGGTTCCTGCAGAAGCCAGGCCAGCCTCCACAGCTCCTGATCTATGAAATTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGATTTATTACTGCATGCAAAGTACACAGCTTCCTCGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATACACTGGGTGCGACAGGCCCCTGGACAAGGGCTTGAGTGGATGGGATGGATCAACCCTTACAGTGGTGACACAAACTATGCACAGAAGTTTCAGGGCAGGGTCACCATGACCAGGGACACGTCCATCACCACAGCCTACATGGAACTGAGCAGGCTGAGATTTGACGACACGGCCGTGTTTTACTGTGCGAGAGATTGGGGTGGCTACAGTTCTTACTACTACGGTATGGACGTCTGGGGCCAAGGGACCACGGTCACCGTCTCTTCA |
| | | | SEQ ID NO: 27199 | SEQ ID NO: 31205 |
| | | AA | DIVMTQTPLSLSVTPGQPASISCKSSQSLLHSDGKTFLYWFLQKPGQPPQLLIYEISNRFSGVPDRFSGSGSGTDFTLKISRVEAEDVGIYYCMQSTQLPRTFGQGTKVEIK | QVQLVQSGAEVKKPGASVKVSCKASGYTFTGYYIHWVRQAPGQGLEWMGWINPYSGDTNYAQKFQGRVTMTRDTSITTAYMELSRLRFDDTAVFYCARDWGGYSSYYYGMDVWGQGTTVTVSS |
| | | | SEQ ID NO: 27200 | SEQ ID NO: 31206 |

FIGURE 50

| | | | | |
|---|---|---|---|---|
| **iPS:436550** | **21-225_224D8** | NA | GACATCGTGATGACCCAGTCTCCAGACTCCCT GGCTGTTTCTCTGGGCGAGAGGGCCGCCATCA ACTGCAAGTCCAGCCAGAGTGTTTTATACAGC TCCAACAATAAGAACTACTTAGCTTGGTACCA GCAGAAACCAGGACAGCCTCCTAAGCTGCTCA TTTATTGGTCGTCTACCCGGAAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTTTAGTACTCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGTTGTACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCTTATAGCAGT GGCTGGTACTACTTTGACTACTGGGGCCAGGGAA CCCTGGTCATCGTCTCCTCA |
| | | | SEQ ID NO: 27201 | SEQ ID NO: 31207 |
| | | AA | DIVMTQSPDSLAVSLGERAAINCKSSQSVLYSSN NKNYLAWYQQKPGQPPKLLIYWSSTRKSGVPD RFSGSGSGTDFTLTISSLQAEDVAVYYCQQYFST PPTFGQGTKVEIK | QVQLVQSGAEVKKPGASVKVSCKASGYTFTNYDIN WVRQATGQGLEWMGWLYPNSGNTGYAQKFQGR VTMTRNTSISTAYMELSSLRSEDTAVYYCAYSSGW YYFDYWGQGTLVIVSS |
| | | | SEQ ID NO: 27202 | SEQ ID NO: 31208 |
| **iPS:436554** | **21-225_224C10** | NA | GACATCGTGATGACCCAGTCTCCAGACTCCCT GGCTGCGTCTCTGGGCGAGAGGGCCACCATCA CCTGCAAGTCCAGCCAGAGTGTTTTATACAAT TCCAACAATAAGAACTACTTAGCTTGGTACCA GCAGAAACCAGGACAGCCTCCTAAGCTGCTCA TTTACTGGGCATCTACCCGGGAGTCCGGGGTC CCTGACCGATTCAGTGGCAGCGGGTCTGGGAC AGATTTCACTCTCACCATCAGCAGCCTGCAGG CTGAAGATGTGGCAATTTATTACTGTCAACAA TATTATATTAATCCGTGCAGTTTTGGCCAGGG GACCAGGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATCCACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCCTATAGCAGT GGCTGGTACAAGTTTGACTACTGGGGCCAGGGA ACCCTGGTCACCGTCTCCTCA |
| | | | SEQ ID NO: 27203 | SEQ ID NO: 31209 |

FIGURE 50

| | | | | |
|---|---|---|---|---|
| | | AA | DIVMTQSPDSLAASLGERATITCKSSQSVLYNSNNKNYLAWYQQKPGQPPKLLIYWASTRESGVPDRFSGSGSGTDFTLTISSLQAEDVAIYYCQQYYINPCSFGQGTRLEIK | QVQLVQSGAEVKKPGASVKVSCKASGSTFTNYDINWVRQATGQGLEWMGWMHPNSGNTGYAQKFQGRVTMTRNTSISTAYMELSSLRSEDTAVYYCAYSSGWYKFDYWGQGTLVTVSS |
| | | | SEQ ID NO: 27204 | SEQ ID NO: 31210 |
| iPS:436556 | 21-225_224D10 | NA | GACATCCTGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTACAAAGTGGGGTCCCATCGCGGTTCAGCGGCAGTGGATCTGGGACAGAATTCACTCTCACAATCAGCAGCCTGCAGCCTGAAGATTTTGCAACTTATTACTGTCTACAACATAATAGTTACCCGCTCACTTTTGGCGGAGGGACCAAGGTGGAGATCAAA | CAGGTGCAGCTGGTGGAGTCTGGGGGAGGCCTGGTCCAGCCTGGAAAGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGAGGGAAGTAATAAATACTATGTAGACTCCGTGAGGGGCCGATTCACCATCTCCAGAGACAATTCCAAGAGTACGCTGTATCTGCAAATGAACAGCCTGCGAGCCGAGGACACGGCTGTGTATTACTGTGTGAGAGAGCTAGGCTTCCAGTCTGACTACTGGGGCCAGGGAACCCCGGTCACCGTCTCCTCA |
| | | | SEQ ID NO: 27205 | SEQ ID NO: 31211 |
| | | AA | DILMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVEIK | QVQLVESGGGLVQPGKSLRLSCAASGFTFSSYGMHWVRQAPGKGLEWVAVIWYEGSNKYYVDSVRGRFTISRDNSKSTLYLQMNSLRAEDTAVYYCVRELGFQSDYWGQGTPVTVSS |
| | | | SEQ ID NO: 27206 | SEQ ID NO: 31212 |

FIGURE 50

| | | | | |
|---|---|---|---|---|
| **iPS:436558** | **21-225_224C11** | NA | GATTTTGTGATGACCCAGACTCCACTCTCTCTGTCCGTCACCCCTGGACAGCCGGCCTCCATCTCCTGCAAGTCTAGTCAGAGCCTCCTGCATAGTGATGGAAAGACCTTTTTGTATTGGTACCTGCAGAAGCCAGGCCAGCCTCCACAGCTCCTGATCTATGAAATTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGATTTATTACTGCATGCAAAGTACACAGCTTCCTCGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATACACTGGGTGCGACAGGCCCCTGGACAAGGGCTTGAGTGGATGGGATGGATCAACCCTTACAGTGGTGACACAAACTATGCACAGAAGTTTCAGGGCAGGGTCACCATGACCAGGGACACGTCCATCAGCACAGCCTACATGGAACTGAGGAGGCTGAGATTTGACGACACGGCCGTGTTTTACTGTGCGAGAGATTGGGGTGGCTACAGTTCTTACTACTTCGGTATGGACGTCTGGGGCCAAGGGACCACGGTCACCGTCTCTTCA |
| | | | SEQ ID NO: 27207 | SEQ ID NO: 31213 |
| | | AA | DFVMTQTPLSLSVTPGQPASISCKSSQSLLHSDGKTFLYWYLQKPGQPPQLLIYEISNRFSGVPDRFSGSGSGTDFTLKISRVEAEDVGIYYCMQSTQLPRTFGQGTKVEIK | QVQLVQSGAEVKKPGASVKVSCKASGYTFTGYYIHWVRQAPGQGLEWMGWINPYSGDTNYAQKFQGRVTMTRDTSISTAYMELRRLRFDDTAVFYCARDWGGYSSYYFGMDVWGQGTTVTVSS |
| | | | SEQ ID NO: 27208 | SEQ ID NO: 31214 |
| **iPS:436560** | **21-225_224F11** | NA | AACATCGTGATGACCCAGTCTCCAGACTCCCTGGCTGTGTCTCTGGACGAGAGGGCCACCATCAACTGCAAGTCCAGCCAGAGTGTTTTATCCAGCTCCAACAATCACAACTACTTAGCTTGGTACCAGCAGAAACCAGGACAGCCTCCTAAGATGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATACTACTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCCTATAGCAGTGGCTGGTACAAGTTTGACTACTGGGGCCAGGGAACCCTGGTCACCGTCTCCTCA |
| | | | SEQ ID NO: 27209 | SEQ ID NO: 31215 |

FIGURE 50

| | | | | |
|---|---|---|---|---|
| | | AA | NIVMTQSPDSLAVSLDERATINCKSSQSVLSSSNNHNYLAWYQQKPGQPPKMLIYWASTRESGVPDRFSGSGSGTDFTLTISSLQAEDVAVYYCQQYYTTPCSFGQGTKLEIK | QVQLVQSGAEVKKPGASVKVSCKASGYTFTNYDINWVRQATGQGLEWMGWMNPNSGNTGYAQKFQGRVTMTRNTSISTAYMELSSLRSEDTAVYYCAYSSGWYKFDYWGQGTLVTVSS |
| | | | SEQ ID NO: 27210 | SEQ ID NO: 31216 |
| iPS:436562 | 21-225_224H11 | NA | GATATTGTGATGACCCAGACTCCACTCTCTCTGTCCGTCACCCCTGGACAGCCGGCCTCCATCTCCTGCAAGTCTAGTCAGAGCCTCCTGCATAGTGATGGAAAGACCTTTTTGTATTGGTACCTGCAGAAGCCAGGCCAGCCTCCACAGCTCCTGATCTATGAAATTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGATTTATTACTGCATGCAAAGTACACAGCTTCCTCGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATACACTGGGTGCGACAGACCCCTGGACAAGGGCTTGAGTGGATGGGATGGATCAACCCTTACAGTGGTGACACAAACTATGCACAGAAGTTTCAGGGCAGGGTCACCATGACCAGGGACACGTCCATCAGCACAGCCTACATGGAACTGCGCAGGCTGAGATTTGACGACACGGCCGTCTTTTACTGTGCGAGAGATTGGGGTGGCTACAGTTCTTACTACTACGGTATGGACGTCTGGGGCCAAGGGACCACGGTCACCGTCTCTTCA |
| | | | SEQ ID NO: 27211 | SEQ ID NO: 31217 |
| | | AA | DIVMTQTPLSLSVTPGQPASISCKSSQSLLHSDGKTFLYWYLQKPGQPPQLLIYEISNRFSGVPDRFSGSGSGTDFTLKISRVEAEDVGIYYCMQSTQLPRTFGQGTKVEIK | QVQLVQSGAEVKKPGASVKVSCKASGYTFTGYYIHWVRQTPGQGLEWMGWINPYSGDTNYAQKFQGRVTMTRDTSISTAYMELRRLRFDDTAVFYCARDWGGYSSYYYGMDVWGQGTTVTVSS |
| | | | SEQ ID NO: 27212 | SEQ ID NO: 31218 |

FIGURE 50

| | | | | |
|---|---|---|---|---|
| **iPS:436564** | **21-225_225A1** | NA | GACATCCAGATGACCCAGTCTCCATCCTCCCT<br>GTCTGCATCTGTAGGAGACAGAGTCACCATCA<br>CTTGCCGGGCAAGTCAGGGCATTAGAGATGAT<br>TTAGGCTGGTATCAGCAGATACCAGGGAAAGC<br>CCCTAAGCGCCTGATCTATGCTGCATCCAGTTT<br>GCAAAGTGGGGTCCCATCAAGGTTCAGCGGCA<br>GTGGATCTGGGACAGAATTCACTCTCACAATC<br>AGCAGCCTGCAGCCTGAAGATTTTGCAACTTA<br>TTACTGTCTGCAGCATTATAGTTACCCTCGGAC<br>GTTCGGCCAAGGGACCAAGGTGGAAATCAAA | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTGACTATGTCAT<br>CCACTGGGTCCGCCAGGCTCCAGGCAAGGGGCT<br>GGAGTGGGTGGCAGTTATATGGTATGATGGAAGT<br>AATAAATACTATGCAGACTCCGTGAAGGGCCGA<br>TTCACCATCTCCAGAGACAATTCCAAGAACACGC<br>TGTATCTGCAAATGAACAGCCTGAGAGCCGAGG<br>ACACGGCTGTGTATTACTGTGCGAGAGAGAGGTA<br>TAGCAGTGGCTGGTACGACTACGGTATGGACGTC<br>TGGGGCCAAGGGACCACGGTCACCGTCTCCTCA |
| | | | SEQ ID NO: 27213 | SEQ ID NO: 31219 |
| | | AA | DIQMTQSPSSLSASVGDRVTITCRASQGIRDDLG<br>WYQQIPGKAPKRLIYAASSLQSGVPSRFSGSGSG<br>TEFTLTISSLQPEDFATYYCLQHYSYPRTFGQGT<br>KVEIK | QVQLVESGGGVVQPGRSLRLSCAASGFTFSDYVIH<br>WVRQAPGKGLEWVAVIWYDGSNKYYADSVKGRF<br>TISRDNSKNTLYLQMNSLRAEDTAVYYCARERYSS<br>GWYDYGMDVWGQGTTVTVSS |
| | | | SEQ ID NO: 27214 | SEQ ID NO: 31220 |
| **iPS:436568** | **21-225_225B3** | NA | GAAATTGTGCTGACGCAGTCTCCAGGCACCCT<br>GTCTTTGTCTCCAGGGGAAAGAGCCACCCTCT<br>CCTGCAGGGCCAGTCAGAATCTTAGCAGCAGC<br>TACTTAGGCTGGTACCAGCAGAAACCTGGCCA<br>GGCTCCCAGACTCCTCATCTATGATACATCCA<br>GCAGGGCCACTGGCATCCCAGACAGGTTCAGT<br>GGCAGTGGGTCTGGGACAGACTTCACTCTCAC<br>CATCAGCAGACTGGAGCCTGAAGATTTTGCAG<br>TGTATTACTGTCAGGAGTATGGTAGCTCACTC<br>ATGTGCAGTTTTGGCCAGGGGACCAAGCTGGA<br>GATCAAA | CAGGTGCAACTGGTGCAGTCTGGGGCTGAGGTG<br>AAGAAGCCTGGGGCCTCAGTGAAGGTTTCCTGCA<br>AGGCATCTGGATACACCTTCACCAGCTACTATAT<br>GCACTGGGTGCGACAGGCCCCTGGACAAGGGCT<br>TGAGTGGATGGGAATAATCAACCCTAGTGGTGGT<br>AGCACAAGCTACGCACAGAAGTTCCAGGGCAGA<br>GTCACCATGACCAGGGACACGTCCACGAGCACA<br>GTCTACATGGAGCTGAGCAGCCTGAGATCTGCGG<br>ACACGGCCGTGTATTACTGTGCGAGGGATTTAGC<br>AGCTCGTTCTTACTACTACTACTTCGGTATGGAC<br>GTCTGGGGCCAAGGGGCCACGGTCACCGTCTCCT<br>CA |
| | | | SEQ ID NO: 27215 | SEQ ID NO: 31221 |

FIGURE 50

| | | | | |
|---|---|---|---|---|
| | | AA | EIVLTQSPGTLSLSPGERATLSCRASQNLSSSYLGWYQQKPGQAPRLLIYDTSSRATGIPDRFSGSGSGTDFTLTISRLEPEDFAVYYCQEYGSSLMCSFGQGTKLEIK | QVQLVQSGAEVKKPGASVKVSCKASGYTFTSYYMHWVRQAPGQGLEWMGIINPSGGSTSYAQKFQGRVTMTRDTSTSTVYMELSSLRSADTAVYYCARDLAARSYYYYFGMDVWGQGATVTVSS |
| | | | SEQ ID NO: 27216 | SEQ ID NO: 31222 |
| iPS:436570 | 21-225_225F4 | NA | GACATCGTGATGACCCAGTCTCCAGACTCCCTGGCTGTGTCTCTGGGCGAGAGGGCCACCATCAACTGCAAGTCCAGCCAGAGTGTTTTATATAGCTCCAACAATAAGAACTACTTAGCTTGGTACCAGCAGAAACCAGGACAGCCTCCTAAACTACTCATTTACTGGGCATCTACCCGGGAATCCGGGGTCCCTGACCGATTCACTGGCAGCGGGTCTGGGACAGATTTCACTCTCACCATCAGCTGCGTGCAGCCGGAAGATGTGGCAGTTTATTACTGTCACCAATATCATAATTCTCCTCCCACTTTCGGCCACGGGACCGAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAGCACAGGCTATGCACAGAAGTTCCAGGGCAGACTCACCATGACCAGGAACACCTCCATAAGCACAGTCTACATGGAACTGAACAGCCTGAGATCTGAGGACACGGCCGTGTATTATTGTGCGAGTAGCAGTGGCTGGTACTGGTTCGACCCCTGGGGCCAGGGAACCCTGGTCACCGTCTCCTCA |
| | | | SEQ ID NO: 27217 | SEQ ID NO: 31223 |
| | | AA | DIVMTQSPDSLAVSLGERATINCKSSQSVLYSSNNKNYLAWYQQKPGQPPKLLIYWASTRESGVPDRFTGSGSGTDFTLTISCVQPEDVAVYYCHQYHNSPPTFGHGTEVDIK | QVQLVQSGAEVKKPGASVKVSCKASGYTFTNYDINWVRQATGQGLEWMGWMHPNSGSTGYAQKFQGRLTMTRNTSISTVYMELNSLRSEDTAVYYCASSSGWYWFDPWGQGTLVTVSS |
| | | | SEQ ID NO: 27218 | SEQ ID NO: 31224 |

FIGURE 50

| | | | | |
|---|---|---|---|---|
| **iPS:436572** | **21-225_225G4** | NA | GATATTGTGATGACCCAGACTCCACTCTCTCTGTCCGTCACCCCTGGACAGCCGGCCTCCATCTCCTGCAAGTCTAGTCAGAGCCTCCTGCATAGTGATGGAAAGACCTTTTTGTATTGGTACCTGCAGAAGCCAGGCCAGCCTCCACAGCTCCTGATCTATGAAATTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGATTTATTACTGCATGCAAAGTACACAGCTTCCTCGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATACACTGGGTGCGACAGGCCCCTGGACAAGGGCTTGAGTGGATGGGATGGATCAACCCTTACAGTGGTGACACAAACTATGCACAGAAGTTTCAGGGCAGGGTCACCATGACCAGGGACACGTCCATCAGCACAGCCTACATGGAACTGCGCAGGCTGAGATTTGACGACACGGCCGTCTTTTACTGTGCGAGAGATTGGGGTGGCTACAGTTCTTACTACTACGGTATGGACGTCTGGGGCCAAGGGACCACGGTCACCGTCTCTTCA |
| | | | SEQ ID NO: 27219 | SEQ ID NO: 31225 |
| | | AA | DIVMTQTPLSLSVTPGQPASISCKSSQSLLHSDGKTFLYWYLQKPGQPPQLLIYEISNRFSGVPDRFSGSGSGTDFTLKISRVEAEDVGIYYCMQSTQLPRTFGQGTKVEIK | QVQLVQSGAEVKKPGASVKVSCKASGYTFTGYYIHWVRQAPGQGLEWMGWINPYSGDTNYAQKFQGRVTMTRDTSISTAYMELRRLRFDDTAVFYCARDWGGYSSYYYGMDVWGQGTTVTVSS |
| | | | SEQ ID NO: 27220 | SEQ ID NO: 31226 |
| **iPS:436574** | **21-225_225F5** | NA | GACATCGTGATGACCCAGTCTCCAGACTCCCTGGCTGTGTCTCTGGGCGAGAGGGCCACCATCAACTGCAAGTCCAGCCAGAATGTTTTATACAACTCCAACAATAACAACTACTTAGCTTGGTACCAGCAGAAACCAGGACAGCCTCCTAACCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTTCTGTCAGCAATATTATAGTTCTCCTCCGACGTTCGGCCAAGGGACCAAGGTGGAAATCAAC | CAGGTGCAGCTGGTGCAGTCTGGGGCTGAGGTGAAGAAGCCTAGGGCCTCAGTGAAGGTCTCCTGCAAGGCTTCTGGACACACCTTCACCAATTATGATATCAACTGGGTGCGACAGGCCACTGGACAAGGGCTTGAGTGGATGGGATGGATGCATCCTAACAGTGGTAACACAGGCTTTGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCATATAGCAGTGGCTGGTACCGCTTTGACTACTGGGGCCAGGGAACCCTGGTCACCGTCTCCTCA |
| | | | SEQ ID NO: 27221 | SEQ ID NO: 31227 |

FIGURE 50

| | | | | |
|---|---|---|---|---|
| | | AA | DIVMTQSPDSLAVSLGERATINCKSSQNVLYNSNNNNYLAWYQQKPGQPPNLLIYWASTRESGVPDRFSGSGSGTDFTLTISSLQAEDVAVYFCQQYYSSPPTFGQGTKVEIN | QVQLVQSGAEVKKPRASVKVSCKASGHTFTNYDINWVRQATGQGLEWMGWMHPNSGNTGFAQKFQGRVTMTRNTSISTAYMELSSLRSEDTAVYYCAYSSGWYRFDYWGQGTLVTVSS |
| | | | SEQ ID NO: 27222 | SEQ ID NO: 31228 |
| iPS:436576 | 21-225_225B6 | NA | GACATCCAGATGACCCAGTCTCCATCCTCCCTGTCTGCATCTGTAGGAGACAGAGTCATCATCACTTGCCGGGCAAGTCAGGGCATGAGAAAAGATTTAGGCTGGTATCAGCAGAAACCAGGGAAAGCCCCTAAGCGCCTGATCTATGCTGCAACCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTATCCATTCACTTTCGGCCCTGGGACCAAAGTGGATATCAAA | CAGGTGCGGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGAAAATAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAGTGGGATTCACTGAGGACTACTGGGGCCAGGGAACCCTGGTCACCGTCTCCTCA |
| | | | SEQ ID NO: 27223 | SEQ ID NO: 31229 |
| | | AA | DIQMTQSPSSLSASVGDRVIITCRASQGMRKDLGWYQQKPGKAPKRLIYAATSLQSGVPSRFSGSGSGTEFTLTISSLQPEDFATYYCLQHNSYPFTFGPGTKVDIK | QVRLVESGGGVVQPGRSLRLSCAASGFTFSDYGMHWVRQAPGKGLEWVAVIWYDENNKYYADSVKGRFTISRDNSKNTLYLQMNSLRAEDTAVYYCAREVGFTEDYWGQGTLVTVSS |
| | | | SEQ ID NO: 27224 | SEQ ID NO: 31230 |

FIGURE 50

| | | | | |
|---|---|---|---|---|
| **iPS:436578** | **21-225_225D6** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTACAAAGTGGGGTCCCATCAAGGTTCAGCGGCAGTGGATCTGGGACAGAATTCACTCTCACAATCAGCAGCCTGCAGCCTGAAGATTTTGCAACTTATTGCTGTCTTCAGCATAATACTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGATGAAAATAATAAATACTATGCAGACTCCGTGAAGGGCCGATTCACCATCTCCAGAGACAATTCCCAGAACACGCTGTATCTGCAAATGACCAGCCTGAGAGCCGAGGACACGGCTGTGTATTACTGTGCGAGAGAAGTGGGATTCACTGAGGACTACTGGGGCCAGGGAACCCTGGTCACCGTCTCCTCA |
| | | | SEQ ID NO: 27225 | SEQ ID NO: 31231 |
| | | AA | DIQMTQSPSSLSASVGDRVTITCRASQGIRNDLGWYQQKPGKAPKRLIYAASSLQSGVPSRFSGSGSGTEFTLTISSLQPEDFATYCCLQHNTYPFTFGPGTKVDIK | QVQLVESGGGVVQPGRSLRLSCAASGFTFSNYGMHWVRQAPGKGLEWVAVIWYDENNKYYADSVKGRFTISRDNSQNTLYLQMTSLRAEDTAVYYCAREVGFTEDYWGQGTLVTVSS |
| | | | SEQ ID NO: 27226 | SEQ ID NO: 31232 |
| **iPS:436580** | **21-225_225E7** | NA | GAAATTGTGTTGACGCAGTCTCCAGGCACCCTGTCTTTGTCTCCAGGGGAAAGAGCCACCCTCTCCTGCAGGGCCAGTCAGAGTGTTTACAGCAGCTACTTAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTATATTACTGTCAGCAGTATGGTACCTCACCTCGGACGTTCGGCCAAGGGACCAAGGTGGAAATCAAA | CAGGTGCAGCTGCAGGAGTCGGGCCCAGGACTGGTGAAGCCTTCACAGACCCTGTCCCTCACCTGCACTGTCTCTGGTAACTCCATCAGCAGTGGTCATTACTACTGGAGCTGGATCCGCCAGCACCCAGGGAAGGGCCTTGAGTGGATTGGGTTCATCTATTACACTGGGAGCACCTACTACAACCCGTCCCTCAAGAGTCGAGTTACCATATCAGTAGACACGTCTAAGAACCAGTTCTCCCTGAAGCTGAGCTCTGTGACTGCCGCGGACACGGCCGTGTATTACTGTGCGAGAGAGGCCGGTGACTACGGCTACTACGGTATGGACGTCTGGGGCCAAGGGACCACGGTCACCGTCTCCTCA |
| | | | SEQ ID NO: 27227 | SEQ ID NO: 31233 |

FIGURE 50

| | | | | |
|---|---|---|---|---|
| | | AA | EIVLTQSPGTLSLSPGERATLSCRASQSVYSSYLAWYQQKPGQAPRLLIYGASSRATGIPDRFSGSGSGTDFTLTISRLEPEDFAVYYCQQYGTSPRTFGQGTKVEIK | QVQLQESGPGLVKPSQTLSLTCTVSGNSISSGHYYWSWIRQHPGKGLEWIGFIYYTGSTYYNPSLKSRVTISVDTSKNQFSLKLSSVTAADTAVYYCAREAGDYGYYGMDVWGQGTTVTVSS |
| | | | SEQ ID NO: 27228 | SEQ ID NO: 31234 |
| iPS:436582 | 21-225_225F8 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATATATGCTGCATCCAGTTTGCTAGGTGGTGTCCCATCAAGATTCAGCGGCAGTGGATCTGGGACAGAATTCACTCTCACAATCAGCAGCCTGCAGCCTGAAGATTTTGCAACTTATTACTGTCTACAACATAATAGTTACCCATTCACTTTCGGCCCTGGGACCAAGGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGAAAATAATAAATACTATGTAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAGTGGGATTTACTGAGGACTACTGGGGCCAGGGAACCCTGGTCACCGTCTCCTCA |
| | | | SEQ ID NO: 27229 | SEQ ID NO: 31235 |
| | | AA | DIQMTQSPSSLSASVGDRVTITCRASQGIRNDLGWYQQKPGKAPKRLIYAASSLLGGVPSRFSGSGSGTEFTLTISSLQPEDFATYYCLQHNSYPFTFGPGTKVDIK | QVQLVESGGGVVQPGRSLRLSCAASGFTFSSYGMHWVRQAPGKGLEWVAVIWYDENNKYYVDSVKGRFTISRDNSKNTLYLQMNSLRAEDTAVYYCAREVGFTEDYWGQGTLVTVSS |
| | | | SEQ ID NO: 27230 | SEQ ID NO: 31236 |

FIGURE 50

| | | | | |
|---|---|---|---|---|
| **iPS:436584** | **21-225_225B9** | NA | GACATCGTGATGACCCAGTCTCCAGATTCCCT GGCTGTGTCTCTGGGCGAGAGGGCCACCATCA ACTGCAAGTCCAGCCAGAGTGTTTTATACAGC TCCAACAATAACAACTACTTAGCTTGGTACCA ACAAAAACCAGGACAGCCTCCTAAGCTGCTCA TTTTCTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAACAC TCCAAGAGTATTCCTGGTAAGTTTGGGCAGGG GATCAAACTGGAGATCCAA | CAGGTGCAGCTGGTGCAGTCTGGGACTGAGGTG AGGAAGCCTGGGGCCTCAGTGAAGGTCTCCTGC AAGGCTTCTGGATACACCTTCACCAATTATGATA TCAACTGGGTACGACAGGCCACTGGACAAAGGC TTGAGTGGATGGGATGGATGCACCCTAACAGTGG TAACACAGGCTATGCACAGAAGTTCCGGGGCAG AGTCACCATGACCAGGAACACCTCCATAAACAC AGCCTACATGGAGCTGAACAGCCTGAGATCTGA GGACACGGCCGTATATTATTGTGCATATAGCAGT GGCTGGACCCTTTTTGACTACTGGGGCCAGGGAA CCCTGGTCACCGTCTCCTCA |
| | | | SEQ ID NO: 27231 | SEQ ID NO: 31237 |
| | | AA | DIVMTQSPDSLAVSLGERATINCKSSQSVLYSSN NNNYLAWYQQKPGQPPKLLIFWASTRESGVPDR FSGSGSGTDFTLTISSLQAEDVAVYYCQHSKSIPG KFGQGIKLEIQ | QVQLVQSGTEVRKPGASVKVSCKASGYTFTNYDIN WVRQATGQRLEWMGWMHPNSGNTGYAQKFRGR VTMTRNTSINTAYMELNSLRSEDTAVYYCAYSSG WTLFDYWGQGTLVTVSS |
| | | | SEQ ID NO: 27232 | SEQ ID NO: 31238 |
| **iPS:436586** | **21-225_225F11** | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATTACAACTACTTAGCTTGGTACCA GCAGAGACCAGGACAGCCTCCTAAGCTGCTCA TTTACTGGGCTTCTACCCGGGAATCCGGGGTC CCTGACCGATTCAGTGGCAGCGGGTCTGGGCC AGATTTCACTCTCACCATCAGCAGCCTGCAGG CTGAAGATGTGGCAGTTTATTACTGTCAGCAA TATTATACTACTCCTCCGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAACAC AGCCTACATGGAGCTGAGCAGCCTGAGATCTGA GGACACGGCCGTGTATTACTGTGCGTATAGCAGT GGCTGGTACCGCTTTGACTACTGGGGCCAGGGAA CCCTGGTCACCGTCTCCTCA |
| | | | SEQ ID NO: 27233 | SEQ ID NO: 31239 |

FIGURE 50

| | | | | |
|---|---|---|---|---|
| | | AA | DIVMTQSPDSLAVSLGERATINCKSSQSVLYSSNNYNYLAWYQQRPGQPPKLLIYWASTRESGVPDRFSGSGSGPDFTLTISSLQAEDVAVYYCQQYYTTPPTFGQGTKVEIK | QVQLVQSGAEVKKPGASVKVSCKASGYTFTNYDINWVRQATGQGLEWMGWMHPNSGNTGYAQKFQGRVTMTRNTSINTAYMELSSLRSEDTAVYYCAYSSGWYRFDYWGQGTLVTVSS |
| | | | SEQ ID NO: 27234 | SEQ ID NO: 31240 |
| **iPS:436588** | **21-225_225F12** | NA | GACATCGTGATGACCCAGTCTCCAGACTCCCTGGCTGTGTCTCTGGGCGAGAGGGCCACCATCAACTGCAAGTCCAGCCAGAGTGTTTTATACAGCTCCAACAATCAGAACTACTTAGCTTGGTACCAGCAGAAACCAGGACAGCCTCCTAGGCTGCTCATTTACTGGACATCTACCCGGGAATCCGGGGTCCCTGACCGATTCAGTGGCAGCGGGTCTGGGACAGATTTCACTCTCACCATCAGCAACCTGCAGGCTGAAGATGTGGCTGTTTATTACTGTCAGCAATATTATATTACTCCGTGCAGTTTTGGCCAGGGGACCAAACTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCCATTATGATATCAACTGGGTGCGACAGGCCACTGGACAAGGGCTTGAGTGGATGGGATGGATGCACCCAAACAGTGGTAACACAGGCTATGCACAGAAGTTCCAGGGCAGAGTCACCATGGCCAGGAACACCTCCATAAGCACAGCCTACATGGAGCTGAGCAGCCTGAGATCTGAGGACACGGCCGTGTATTACTGTGCCTATAGCAGTGGCTGGTACAAGTTTGACTACTGGGGCCAGGGAACCCTGGTCACCGTCTCCTCA |
| | | | SEQ ID NO: 27235 | SEQ ID NO: 31241 |
| | | AA | DIVMTQSPDSLAVSLGERATINCKSSQSVLYSSNNQNYLAWYQQKPGQPPRLLIYWTSTRESGVPDRFSGSGSGTDFTLTISNLQAEDVAVYYCQQYYITPCSFGQGTKLEIK | QVQLVQSGAEVKKPGASVKVSCKASGYTFTHYDINWVRQATGQGLEWMGWMHPNSGNTGYAQKFQGRVTMARNTSISTAYMELSSLRSEDTAVYYCAYSSGWYKFDYWGQGTLVTVSS |
| | | | SEQ ID NO: 27236 | SEQ ID NO: 31242 |

FIGURE 50

| | | | | |
|---|---|---|---|---|
| iPS:436590 | 21-225_225H12 | NA | GACATCGTGATGACCCAGTCTCCAGACTCCCTGGCTGTGTCTCTGGGCGAGAGGGCCACCATCAACTGCAAGTCCAGCCAGAGTGTTTTATACAACTCCAACAATAACAACTACTTAGCTTGGTACCAGCAGAAACCAGGACAGCCTCCTAACCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGACTGAAGATGTGGCAGTTTATTACTGTCAGCAATATTATATTACTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCCTATAGCAGTGGCTGGTACAAGTTTGACTACTGGGGCCAGGGAACCCTGGTCACCGTCTCCTCA |
| | | | SEQ ID NO: 27237 | SEQ ID NO: 31243 |
| | | AA | DIVMTQSPDSLAVSLGERATINCKSSQSVLYNSNNNNYLAWYQQKPGQPPNLLIYWASTRESGVPDRFSGSGSGTDFTLTISSLQTEDVAVYYCQQYYITPCSFGQGTKLEIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAYSSGWYKFDYWGQGTLVTVSS |
| | | | SEQ ID NO: 27238 | SEQ ID NO: 31244 |
| iPS:436592 | 21-225_226B1 | NA | GATATTGTGATGACCCAGACTCCACTCTCTCTGTCCGTCACCCCTGGACAGCCGGCCTCCATCTCCTGCAAGTCTAGTCAGAGCCTCCTGCATGGTGATGGAAAGACCTATTTGTATTGGTACCTGCAGAGGCCAGGCCAGCCTCCACAGCTCCTGATCAATGAAGTTTCCATCCGGTTCTCTGGAGTGCCAGATAGGTTCAGTGGCAGCGGGTCAGGGACAGATTTCACACTGAAAATCAGCCGGGTGGAGGCTGAGGATGTTGGGGTTTACTACTGCATGCAAAGTATACAGATTCCGTGGACGTTCGGCCAGGGGACCAAGGTGGACATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTACCTATGGCATGCACTGGGTCCGCCAGGCTCCAGGCAAGGGGCTGGAGTGGGTGGCAATTATATGGTATGATGGAGGTTATAAATACTATGCAGACTCCGTGAAGGGCCGATTCACCATCTCCAGAGACAATTCCAAGAACACGCTGTTTCTGCAAATGAACAGCCTGAGAGCCGAGGACACGGCTGTGTATTACTGTGCGAGAGATCACTACGATTTTTGGAGTGGTTATCTTACCCACTGGGGCCAGGGAACCCTGGTCACCGTCTCCTCA |
| | | | SEQ ID NO: 27239 | SEQ ID NO: 31245 |

FIGURE 50

| | | | | |
|---|---|---|---|---|
| | | AA | DIVMTQTPLSLSVTPGQPASISCKSSQSLLHGDGKTYLYWYLQRPGQPPQLLINEVSIRFSGVPDRFSGSGSGTDFTLKISRVEAEDVGVYYCMQSIQIPWTFGQGTKVDIK | QVQLVESGGGVVQPGRSLRLSCAASGFTFSTYGMHWVRQAPGKGLEWVAIIWYDGGYKYYADSVKGRFTISRDNSKNTLFLQMNSLRAEDTAVYYCARDHYDFWSGYLTHWGQGTLVTVSS |
| | | | SEQ ID NO: 27240 | SEQ ID NO: 31246 |
| **iPS:436594** | **21-225_226A5** | NA | GATATTGTGATGACCCAGACTCCACTCTCTCTGTCCGTCACCCCTGGACAGCCGGCCTCCATCTCCTGCAAGTCTAGTCAGAGCCTCCTACATGGTGATGGAAAGACCTATTTGTATTGGTACCTGCAGAAGCCAGGCCAGCCTCCACAGCTCCTGATCTATGAAGTTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGGTTTATTACTGCATGCAAAGTATACAGATTCCGTGGACGTTCGGCCAAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAATTATATGGTATGATGGAACTAATAAATACTATACAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGGGTCACGATTTTTGGAGTGGCTTTTTTTGTTACTGGGGCCAGGGAACCCTGGTCACCGTCTCCTCA |
| | | | SEQ ID NO: 27241 | SEQ ID NO: 31247 |
| | | AA | DIVMTQTPLSLSVTPGQPASISCKSSQSLLHGDGKTYLYWYLQKPGQPPQLLIYEVSNRFSGVPDRFSGSGSGTDFTLKISRVEAEDVGVYYCMQSIQIPWTFGQGTKVEIK | QVQLVESGGGVVQPGRSLRLSCAASGFTFSNYGMHWVRQAPGKGLEWVAIIWYDGTNKYYTDSVKGRFTISRDNSKNTLYLQMNSLRAEDTAVYYCAREGHDFWSGFFCYWGQGTLVTVSS |
| | | | SEQ ID NO: 27242 | SEQ ID NO: 31248 |

FIGURE 50

| | | | | |
|---|---|---|---|---|
| **iPS:436596** | **21-225_226C6** | NA | GACATCCAGATGACCCAGTCTCCATCCTCCCTGTCTGCATCTATAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGCTCCCATCAAGGTTCAGCGGCAGTGGATCTGGGACAGAATTCACTCTCACAATCAGCAACCTGCAGCCTGAAGATTTTGCAACTTATTACTGTCTACAGCATTATAATTACCCTCGGGCGTTCGGCCAAGGGACCAAGGTGGAAATCCAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAACGAACAGCCTGAGAGCCGAGGACACGGCTGTGTATTACTGTGCGAGAGAGAGGTATAGCAGCAGCTGGTACGACTACGGTATGGACGTCTGGGGCCAAGGGACCACGGTCACCGTCTCCTCA |
| | | | SEQ ID NO: 27243 | SEQ ID NO: 31249 |
| | | AA | DIQMTQSPSSLSASIGDRVTITCRASQGIRNDLGWYQQKPGKAPKRLIYAASSLQSGLPSRFSGSGSGTEFTLTISNLQPEDFATYYCLQHYNYPRAFGQGTKVEIQ | QVQLVESGGGVVQPGRSLRLSCAASGFTFSDYGMHWVRQAPGKGLEWVAVIWYDGSNKYYADSVKGRFTISRDNSKNTLYLQTNSLRAEDTAVYYCARERYSSSWYDYGMDVWGQGTTVTVSS |
| | | | SEQ ID NO: 27244 | SEQ ID NO: 31250 |
| **iPS:436598** | **21-225_226D6** | NA | GACATCGTGATGACCCAGTCTCCAGACTCCCTGGCTGTGTCTCTGGGCGAGAGGGCCACCATCAACTGCAGGTCCAGCCAGAGTATTTTATACATCTCCAACAATAAGAACTACTTAGCTTGGTACCAGCAGAAACCAGGACAGCCTCCTAAGATGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTTCTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCCTATAGCAGTGGCTGGTACAAGTTTGACTACTGGGGCCAGGGAACCCTGGTCACCGTCTCCTCA |
| | | | SEQ ID NO: 27245 | SEQ ID NO: 31251 |

FIGURE 50

| | | | | |
|---|---|---|---|---|
| | | AA | DIVMTQSPDSLAVSLGERATINCRSSQSILYISNNKNYLAWYQQKPGQPPKMLIYWASTRESGVPDRFSGSGSGTDFTLTISSLQAEDVAVYYCQQYYSSPCSFGQGTKLEIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAYSSGWYKFDYWGQGTLVTVSS |
| | | | SEQ ID NO: 27246 | SEQ ID NO: 31252 |
| iPS:436600 | 21-225_226F6 | NA | GACATCGTGATGACCCAGTCTCCAGACTCCCTGGCTGTGTCTCTGGGCGAGAGGGCCACCATCAACTGCAAGTCCAGCCAGAGTATTTTATACAGCTCCAACAATTACAACTACTTAGCTTGGTACCAGCAGAAGCCAGGACAGCCTCCTAAGCTGCTCATTTACTGGGCTTCTACCCGGGAATCCGGGGTCCCTGACCGATTCAGTGGCAGCGGGTCTGGGCCAGATTTCACTCTCACCATCAGCAGCCTGCAGGCTGAAGATGTGGCAGTTTATTACTGTCAGCAATATTATACTACTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAACACAGCCTACATGGAGCTGAGCAGCCTGAGATCTGAGGACACGGCCGTGTATTACTGTGCGTATAGCAGTGGCTGGTACCGCTTTGACTACTGGGGCCAGGGAACCCTGGTCACCGTCTCCTCA |
| | | | SEQ ID NO: 27247 | SEQ ID NO: 31253 |
| | | AA | DIVMTQSPDSLAVSLGERATINCKSSQSILYSSNNYNYLAWYQQKPGQPPKLLIYWASTRESGVPDRFSGSGSGPDFTLTISSLQAEDVAVYYCQQYYTTPPTFGQGTKVEIK | QVQLVQSGAEVKKPGASVKVSCKASGYTFTNYDINWVRQATGQGLEWMGWMHPNSGNTGYAQKFQGRVTMTRNTSINTAYMELSSLRSEDTAVYYCAYSSGWYRFDYWGQGTLVTVSS |
| | | | SEQ ID NO: 27248 | SEQ ID NO: 31254 |

FIGURE 50

| | | | | |
|---|---|---|---|---|
| iPS:436602 | 21-225_226E7 | NA | GATATTGTGATGACCCAGACTCCACTCTCTCTGTCCGTCACCCCTGGACAGCCGGCCTCCATCTCCTGCAAGTCTAGTCAGAGCCTCCTGCATGGTGATGGAAAGACCTATTTGTATTGGTACCAGCAGAAGCCAGGCCAGCCTCCACAGATCCTGATCTATGAAGTTTCCAACCGGTTCTCTGGAGTGCCAGATAGGTTCAGTGGCAGCGGGTCAGGGACAGACTTCACACTGAAAATCAGCCGGGTGGAGGCTGAGGATGTTGGGGTTTATTACTGCATGCAAAGTATACAGGTTCCGTGGACGTTCGGCCAAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTACCTATGGCATGCACTGGGTCCGCCAGGCTCCAGGCAAGGGGCTGGAATGGGTGGCAATTATATGGTATGATGGAAGTAATAAATACTATGCAGACTCCGTGAAGGGCCGATTCACCATCTCCAGAGACAATTTCAAGAACACGCTGTATCTGCAAATGCACAGCCTGAGAGCCGACGACACGGCTGTGTATTACTGTGCGAGAGAGAATTACGATTTTTGGAGTGGTTATTATGGCTACTGGGGCCAGGGAACCCTGGTCACCGTCTCCTCA |
| | | | SEQ ID NO: 27249 | SEQ ID NO: 31255 |
| | | AA | DIVMTQTPLSLSVTPGQPASISCKSSQSLLHGDGKTYLYWYQQKPGQPPQILIYEVSNRFSGVPDRFSGSGSGTDFTLKISRVEAEDVGVYYCMQSIQVPWTFGQGTKVEIK | QVQLVESGGGVVQPGRSLRLSCAASGFTFSTYGMHWVRQAPGKGLEWVAIIWYDGSNKYYADSVKGRFTISRDNFKNTLYLQMHSLRADDTAVYYCARENYDFWSGYYGYWGQGTLVTVSS |
| | | | SEQ ID NO: 27250 | SEQ ID NO: 31256 |
| iPS:436604 | 21-225_226F7 | NA | GACATCCAGATGACCCAGTCTCCATCCTCCCTGTCTGCATCTGTTGGAGACAGAGTCACCATCACTTGCCGGGCAAGTCAGGGCATTGGGAATGATTTAGGCTGGTATCAGCAGAAACCAGGGAAAGCCCCTAAGCGCCTGATCTATGCTGCCTCCAGTTTGCAAAGTGGGGTCCCATCAAGGTTCAGCGGCAGTGTATCTGGGACAGAATTCATTCTCACAATCAGCAGCCTGCAGCCTGAAGATTTTGCAACTTATTACTGTCTACATCATTATAGTTACCCTCGGACGTTCGGCCAGGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAATTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGAGGTATAACAGCGGCTGGTACGACTACGGTTTGGACGTCTGGGGCCAAGGGACCACGGTCACCGTCTCCTCA |
| | | | SEQ ID NO: 27251 | SEQ ID NO: 31257 |

FIGURE 50

| | | | | |
|---|---|---|---|---|
| | | AA | DIQMTQSPSSLSASVGDRVTITCRASQGIGNDLGWYQQKPGKAPKRLIYAASSLQSGVPSRFSGSVSGTEFILTISSLQPEDFATYYCLHHYSYPRTFGQGTKVEIK | QVQLVESGGGVVQPGRSLRLSCAASGFTFSSYGMHWVRQAPGKGLEWVAIIWYDGSNKYYADSVKGRFTISRDNSKNTLYLQMNSLRAEDTAVYYCARERYNSGWYDYGLDVWGQGTTVTVSS |
| | | | SEQ ID NO: 27252 | SEQ ID NO: 31258 |
| **iPS:436606** | **21-225_226G8** | NA | GATATTGTGATGACCCAGACTCCACTCTCTCTGTCCGTCACCCCTGGACAGCCGGCCTCCATCTCCTGCAAGTCTAGTCAGAGCCTCCTGCATAGTGATGGAAAGACCTTTTTGTATTGGTACCTGCAGAAGCCAGGCCAGCCTCCACAGCTCCTGATCTATGAAATTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGATTTATTACTGCATGCAAAGTACACAGCTTCCTCGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATACACTGGGTGCGACAGGCCCCTGGACAAGGGCTTGAGTGGATGGGATGGATCAACCCTTACAGTGGTGACACAAAGTATGCACAGAAGTTTCAGGGCAGGGTCACCATGACCAGGGACACGTCCATCAGCACAGCCTACATGGAACTGAGCAGGCTGAGATTTGACGACACGGCCGTGTTTTACTGTGCGAGAGATTGGGGTGGCTACAGTTCTTACTACTACGGTATGGACGTCTGGGGCCAAGGGACCACGGTCACCGTCTCTTCA |
| | | | SEQ ID NO: 27253 | SEQ ID NO: 31259 |
| | | AA | DIVMTQTPLSLSVTPGQPASISCKSSQSLLHSDGKTFLYWYLQKPGQPPQLLIYEISNRFSGVPDRFSGSGSGTDFTLKISRVEAEDVGIYYCMQSTQLPRTFGQGTKVEIK | QVQLVQSGAEVKKPGASVKVSCKASGYTFTGYYIHWVRQAPGQGLEWMGWINPYSGDTKYAQKFQGRVTMTRDTSISTAYMELSRLRFDDTAVFYCARDWGGYSSYYYGMDVWGQGTTVTVSS |
| | | | SEQ ID NO: 27254 | SEQ ID NO: 31260 |

FIGURE 50

| | | | | |
|---|---|---|---|---|
| **iPS:436608** | **21-225_226A9** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGAGGAAAGTAATAAATACTATACAGACTCCGTGAAGGGCCGATTCACCATCTCCAGAGACAATTCCAAGAACACGCTGTTTCTGCAAATGAACAGCCTGAGAGCCGAGGACACGGCTGTGTATTACTGTGCGAGAGAAGTGGGATTCACTGAGGACTACTGGGGCCAGGGAACCCTGGTCACCGTCTCCTCA |
| | | | SEQ ID NO: 27255 | SEQ ID NO: 31261 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPFTFGPGTKVDIK | QVQLVESGGGVVQPGRSLRLSCAASGFTFSNYGMHWVRQAPGKGLEWVAVIWYEESNKYYTDSVKGRFTISRDNSKNTLFLQMNSLRAEDTAVYYCAREVGFTEDYWGQGTLVTVSS |
| | | | SEQ ID NO: 27256 | SEQ ID NO: 31262 |
| **iPS:436610** | **21-225_226F9** | NA | GATATTGTGATGACCCAGACTCCACTCTCTCTGTCCGTCACCCCTGGACAGCCGGCCTCCATCTCCTGCAAGTCTAGTCAGAGCCTCCTGCATAGTGATGGAAAGACCTTTTTGTATTGGTACCTGCAGAAGCCAGGCCAGCCTCCACAGCTCCTGATCTATGAAATTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGATTTATTACTGCATGCAAAGTACACAGCTTCCTCGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATACACTGGGTGCGACAGGCCCCTGGACAAGGGCTTGAGTGGATGGGATGGATCAACCCTTACAGTGGTGACACAAAGTATGCACAGAAGTTTCAGGGCAGGGTCACCATGACCAGGGCCACGTCCATCAGCACAGCCTACATGGAACTGAGCAGGCTGAGATTTGACGACACGGCCGTGTTTTACTGTGCGAGAGATTGGGGTGGCTACAGTTCTTACTACTACGGTATGGACGTCTGGGGCCAAGGGACCACGGTCACCGTCTCTTCA |
| | | | SEQ ID NO: 27257 | SEQ ID NO: 31263 |

FIGURE 50

| | | | | |
|---|---|---|---|---|
| | | AA | DIVMTQTPLSLSVTPGQPASISCKSSQSLLHSDGKTFLYWYLQKPGQPPQLLIYEISNRFSGVPDRFSGSGSGTDFTLKISRVEAEDVGIYYCMQSTQLPRTFGQGTKVEIK | QVQLVQSGAEVKKPGASVKVSCKASGYTFTGYYIHWVRQAPGQGLEWMGWINPYSGDTKYAQKFQGRVTMTRATSISTAYMELSRLRFDDTAVFYCARDWGGYSSYYYGMDVWGQGTTVTVSS |
| | | | SEQ ID NO: 27258 | SEQ ID NO: 31264 |
| **iPS:436612** | **21-225_226H9** | NA | GATATTGTGATGACCCAGACTCCACTCTCTCTGTCCGTCATCCCTGGACAGCCGGCCTCCATCTCCTGCAAGTCTAGTCAGAGCCTCCTGCATAGTGATGGAAAGACCTTTTTGTATTGGTACCTGCAGAGGCCAGGCCAGCCTCCACAGCTCCTGATCTATGAAGTTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGATTTATTACTGCATGCAAAGTACACAGCTTCCTCGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATACACTGGGTGCGACAGGCCCCTGGACAAGGGCTTGAGTGGATGGGATGGATCAACCCTTACAGTGGTGACACAAACTCTGCACAGAAGTTTCAGGGCCGGGTCACCATGACCAGGGACACGTCCATCAGCACAGCCTACATGGAACTGAGCAGGCTGAGATTTGACGACACGGCCGTGTTTTACTGTGCGAGAGATTGGGGTGGCTACAGTTCTTACTACTACGGTATGGACGTCTGGGGCCAAGGGACCACGGTCACCGTCTCTTCA |
| | | | SEQ ID NO: 27259 | SEQ ID NO: 31265 |
| | | AA | DIVMTQTPLSLSVIPGQPASISCKSSQSLLHSDGKTFLYWYLQRPGQPPQLLIYEVSNRFSGVPDRFSGSGSGTDFTLKISRVEAEDVGIYYCMQSTQLPRTFGQGTKVEIK | QVQLVQSGAEVKKPGASVKVSCKASGYTFTGYYIHWVRQAPGQGLEWMGWINPYSGDTNSAQKFQGRVTMTRDTSISTAYMELSRLRFDDTAVFYCARDWGGYSSYYYGMDVWGQGTTVTVSS |
| | | | SEQ ID NO: 27260 | SEQ ID NO: 31266 |

FIGURE 50

| | | | | |
|---|---|---|---|---|
| iPS:436614 | 21-225_226F10 | NA | GATATTGTGATGACCCAGACTCCACTCTCTCTGTCCGTCACCCCTGGACAGCCGGCCTCCATCTCCTGCAAGTCTAGTCAGAGCCTCCTGCATAGTGATGGAAAGACCTTTTTGTATTGGTACCTGCAGAAGCCAGGCCAGCCTCCACAGCTCCTGATCTATGAAATTTCCAACCGGTTCTCTGGGGTGCCAGATAGGTTCAGTGGCAGCGGGTCAGGGACAGATTTCACACTGAAAATCAGCCGAGTGGAGGCTGAGGATGTTGGGATTTATTACTGCATGCAAAGTACACAGCTTCCTCGGACGTTCGGCCAAGGGACCAAGGTGGAAATCAAA | CAGGTGCAGCTGGTGCAGTCTGGGGGTGAGGTGAAGAAGCTGAGGGCCTCAGTGAAGGTCTCCTGCAAGGCTTCTGGATACACCTTCACCGGCTATTATATACACTGGGTGCGACAGGCCCCTGGACAAGGGCTTGAGTGGATGGGATGGATCAACCCATACAGTGGTGACACAAACTATGCACAGAAGTTTCAGGGCAGGGTCACCATGACCAGGGACACGTCCGTCAGCACAGCCTACATGGAACTGAGCAGGTTGAGATTGGACGACACGGCCGTGTTTTACTGTGCGAGAGATTGGGGTGGCTACAGTTCTTACTACTACGGTATGGACGTCTGGGGCCAAGGGACCCCGGTCACCGTCTCTTCA |
| | | | SEQ ID NO: 27261 | SEQ ID NO: 31267 |
| | | AA | DIVMTQTPLSLSVTPGQPASISCKSSQSLLHSDGKTFLYWYLQKPGQPPQLLIYEISNRFSGVPDRFSGSGSGTDFTLKISRVEAEDVGIYYCMQSTQLPRTFGQGTKVEIK | QVQLVQSGGEVKKLRASVKVSCKASGYTFTGYYIHWVRQAPGQGLEWMGWINPYSGDTNYAQKFQGRVTMTRDTSVSTAYMELSRLRLDDTAVFYCARDWGGYSSYYYGMDVWGQGTPVTVSS |
| | | | SEQ ID NO: 27262 | SEQ ID NO: 31268 |
| iPS:436616 | 21-225_226D11 | NA | GACATCGTGATGACCCAGTCTCCAGACTCCCTGGCTGTGTCTCTGGGCGAGAGGGCCACCATCAACTGCAAGTCCAGCCAGAATGTTTTACACAGCTCCAACAGTAATAACTACTTAGTTTGGTACCAGCAGAAACCAGGACAGCCTCCTAAACTGCTCATTTACTGGGCATCTACCCGGGAATCCGGGGTCCCTGACCGATTCAGAGGCAGCGGGTCTGGGACAGATTTCACTCTCACCATCAGCAGCCTGCAGGCTGAAGATGTGGCAGTTTATTACTGTCAGCAATATTATAAAACTCCGTGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAGGTCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCCTATAGCAGTGGCTGGTACTACTTTGACTACTGGGGCCAGGGAACCCTGGTCACCGTCTCCTCA |
| | | | SEQ ID NO: 27263 | SEQ ID NO: 31269 |

FIGURE 50

| | | | | |
|---|---|---|---|---|
| | | AA | DIVMTQSPDSLAVSLGERATINCKSSQNVLHSSN<br>SNNYLVWYQQKPGQPPKLLIYWASTRESGVPDR<br>FRGSGSGTDFTLTISSLQAEDVAVYYCQQYYKTP<br>WTFGQGTKVEIK | QVQLVQSGAEVKKPGASVKVSCRSSGYTFTNYDIN<br>WVRQATGQGLEWMGWMHPNSGNTGYAQKFQGR<br>VTMTRNTSISTAYMELSSLRSEDTAVYYCAYSSGW<br>YYFDYWGQGTLVTVSS |
| | | | SEQ ID NO: 27264 | SEQ ID NO: 31270 |
| **iPS:436618** | **21-225_226E11** | NA | GATATTGTGATGACCCAGACTCCACTCTCTCT<br>GTCCGTCACCCCTGGACAGCCGGCCTCCATCT<br>CCTGCAAGTCTAGTCAGAGCCTCCTGCATAGT<br>GATGGAAAGACCTTTTTGTATTGGTACCTGCA<br>CAAGCCAGGCCAGCCTCCACACCTCCTGATCT<br>ATGAAATTTCCAACCGGTTCTCTGGAGTGCCA<br>GATAGGTTCAGTGGCAGCGGGTCAGGGACAG<br>ATTTCACACTGAAAATCAGCCGGGTGGAGGCT<br>GAGGATGTTGGGATTTATTACTGCATGCAAAG<br>TACACAGCTTCCTCGGACGTTCGGCCAAGGGA<br>CCAAGGTGGAAATCAAA | CAGGTGCAGCTGGTGCAGTCTGGGGCTGAGGTG<br>AAGAAGCCTGGGGCCTCAGTGAAGGTCTCCTGC<br>AAGGCTTCTGGATACACCTTCACCGGCTACTATA<br>TACACTGGGTGCGACAGGCCCCTGGACAAGGGC<br>TTGAGTGGATGGGATGGATCAACCCTTACAGTGG<br>TGACACAAACTATGCACAGAAGTTTCAGGGCAG<br>GGTCACCATGACCAGGGACACGTCCATCAGCAC<br>AGCCTACATGGAACTGAGCAGGCTGAGATTTGA<br>CGACACGGCCGTGTTTTACTGTGCGAGAGATTGG<br>GGTGGCTACAGTTCTTACTACTACGGTATGGACG<br>TCTGGGGCCAAGGGACCACGGTCACCGTCTCTTC<br>A |
| | | | SEQ ID NO: 27265 | SEQ ID NO: 31271 |
| | | AA | DIVMTQTPLSLSVTPGQPASISCKSSQSLLHSDGK<br>TFLYWYLHKPGQPPHLLIYEISNRFSGVPDRFSGS<br>GSGTDFTLKISRVEAEDVGIYYCMQSTQLPRTFG<br>QGTKVEIK | QVQLVQSGAEVKKPGASVKVSCKASGYTFTGYYIH<br>WVRQAPGQGLEWMGWINPYSGDTNYAQKFQGRV<br>TMTRDTSISTAYMELSRLRFDDTAVFYCARDWGG<br>YSSYYYGMDVWGQGTTVTVSS |
| | | | SEQ ID NO: 27266 | SEQ ID NO: 31272 |

FIGURE 50

| | | | | |
|---|---|---|---|---|
| **iPS:436620** | **21-225_226H11** | NA | GACATCCAGATGACCCAGTCTCCATCCTCCCTGTCTGCATCTGTAGGAGACAGAGTCACCATCACTTGCCGGACAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGTGGCAGTGGATCTGGGACAGATTTCACTCTCACAATCAGCAGCCTGCAGCCTGAAGATTTTGCAACTTATTACTGTCTACAGCATTATAGTTACCCTCGGACGTTCGGCCAAGGGACCAAGGTGGAAATCAAA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAACTGTGGCATGCACTGGGTCCGCCAGGCTCCAGGCAAGGGGCTGGAGTGGGTGACAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGCTGTATAGCAGCAGCTGGTACGACTACGGTTTGGACGTCTGGGGCCAAGGGACCACGGTCACCGTCTCCTCA |
| | | | SEQ ID NO: 27267 | SEQ ID NO: 31273 |
| | | AA | DIQMTQSPSSLSASVGDRVTITCRTSQGIRNDLGWYQQKPGKAPKRLIYAASSLQSGVPSRFSGSGSGTDFTLTISSLQPEDFATYYCLQHYSYPRTFGQGTKVEIK | QVQLVESGGGVVQPGRSLRLSCAASGFTFSNCGMHWVRQAPGKGLEWVTVIWYDGSNKYYADSVKGRFTISRDNSKNTLYLQMNSLRAEDTAVYYCARELYSSSWYDYGLDVWGQGTTVTVSS |
| | | | SEQ ID NO: 27268 | SEQ ID NO: 31274 |
| **iPS:436622** | **21-225_226A12** | NA | GACATCGTGATGACCCAGTCTCCAGACTCCCTGGCTGTGTCTCTCGGCGAGAGGGCCACCATCAACTGCAAGTCCAGCCAGAATGTTTTATACAGTTCCAACAATAACAACTACTTAGCTTGGTACCAGCAGACACCTGGACAGCCTCCTAAGCTGCTCTTTTACTGGGCATCTACCCGGAAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTAGTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCTTATAGCAGTGGCTGGTACTACTTTGACTACTGGGGCCAGGGAACCCTGGTCACCGTCTCCTCA |
| | | | SEQ ID NO: 27269 | SEQ ID NO: 31275 |

FIGURE 50

| | | | | |
|---|---|---|---|---|
| | | AA | DIVMTQSPDSLAVSLGERATINCKSSQNVLYSSNNNNYLAWYQQTPGQPPKLLFYWASTRKSGVPDRFSGSGSGTDFTLTISSLQAEDVAVYYCQQYYSSPPTFGQGTKVEIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAYSSGWYYFDYWGQGTLVTVSS |
| | | | SEQ ID NO: 27270 | SEQ ID NO: 31276 |
| iPS:436624 | 21-225_226H12 | NA | GATATTGTGATGACCCAGACTCCACTCTCTCTGTCCGTCACCCCTGGACAGCCGGCCTCCATCTCCTGCAAGTCTAGTAAGACCCTCCTGCATAGTGATGGAAAGACCTTTTTGTATTGGTACCTGCAGAAGCCAGGCCAGCCTCCACAGCCCCTGATCTATGAAATTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGATTTATTACTGCATGCAAAGTACACAGCTTCCTCGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATACACTGGGTGCGACAGGCCCCTGGACAAGGGCTTGAGTGGATGGGATGGATCAACCCTTACAGTGGTGACACAAACTATGCACAGAAGTTTCAGGGCAGGGTCACCATGACCAGGGACACGTCCATCAGCACAGCCTACATGGAACTGAGGAGGCTGAGATTTGACGACACGGCCGTGTTTTACTGTGCGAGAGATTGGGGTGGCTACAGTTCTTACTACTACGGTATGGACGTCTGGGGCCAAGGGACCACGGTCACCGTCTCTTCA |
| | | | SEQ ID NO: 27271 | SEQ ID NO: 31277 |
| | | AA | DIVMTQTPLSLSVTPGQPASISCKSSKTLLHSDGKTFLYWYLQKPGQPPQPLIYEISNRFSGVPDRFSGSGSGTDFTLKISRVEAEDVGIYYCMQSTQLPRTFGQGTKVEIK | QVQLVQSGAEVKKPGASVKVSCKASGYTFTGYYIHWVRQAPGQGLEWMGWINPYSGDTNYAQKFQGRVTMTRDTSISTAYMELRRLRFDDTAVFYCARDWGGYSSYYYGMDVWGQGTTVTVSS |
| | | | SEQ ID NO: 27272 | SEQ ID NO: 31278 |

FIGURE 50

| | | | | |
|---|---|---|---|---|
| **iPS:436626** | **21-225_227C1** | NA | GATATTGTGATGACCCAGACTCCACTCTCTCTGTCCGTCACCCCTGGACAGCCGGCCTCCATCTCCTGCAAGTCTAGTCAGAGCCTCCTGCATAGTGATGGAAAGACCTTTTTGTATTGGTACCTGCAGAAGCCAGGCCAGCCTCCACAGCTCCTGATCTATGAAATTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGATTTATTACTGCATGCAAAGTACACAGCTTCCTCGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATACACTGGGTGCGACAGGCCCCTGGACAAGGGCTTGAGTGGATGGGATGGATCAACCCTTACAGTGGTGGCACAAACTATGCACAGAAGTTTCAGGGCAGGGTCACCATGACCAGGGACACGTCCATCAGCACAGCCTACATGGAACTGAGCAGGCTGAGATTTGACGACACGGCCGTGTATTACTGTGCGAGAGATTGGGGTGGCTACAGTTCTTACTACTACGGTATGGACGTCTGGGGCCAAGGGACCACGGTCACCGTCTCTTCA |
| | | | SEQ ID NO: 27273 | SEQ ID NO: 31279 |
| | | AA | DIVMTQTPLSLSVTPGQPASISCKSSQSLLHSDGKTFLYWYLQKPGQPPQLLIYEISNRFSGVPDRFSGSGSGTDFTLKISRVEAEDVGIYYCMQSTQLPRTFGQGTKVEIK | QVQLVQSGAEVKKPGASVKVSCKASGYTFTGYYTHWVRQAPGQGLEWMGWINPYSGGTNYAQKFQGRVTMTRDTSISTAYMELSRLRFDDTAVYYCARDWGGYSSYYYGMDVWGQGTTVTVSS |
| | | | SEQ ID NO: 27274 | SEQ ID NO: 31280 |
| **iPS:436628** | **21-225_227F2** | NA | GATATTGTGATGACCCAGACTCCACTCTCTCTGTCCGTCACCCCTGGACAGCCGGCCTCCATCTCCTGCAAGTCTAGTCAGAGCCTCCTGCATAGTGATGGAAAGACCTTTTTGTATTGGTACCTGCAGAAGCCAGGCCAGCCTCCACAGCTCCTGATCTATGAAATTTCCAACCGGTTCTCTGGAGTGCCAGATAGGTTCAGTGGCAGCGGGTCAGGGACAGATTTCACACTGAAAATCAGCCGGGTGGAGGCTGAGGATGTTGGGATTTATTACTGCATGCAAAGTACACAGCTTCCTCGGACGTTCGGCCAAGGGACCAAGGTGGAAATCAAA | CAGGTGCACCTGGTGCAGTCTGGGGCTGAGGTGAAGAAGCCTGGGGCCTCAGTGAAGGTCTCCTGCAAGGCTTCTGGATACACCTTCACCGGCTACTATATACACTGGGTGCGACAGGCCCCTGGACAAGGGCTTGAGTGGATGGGATGGATCAACCCTTACAGTGGTGACACAAAGTATGCACAGAAGTTTCAGGGCAGGGTCACCATGACCAGGGACACGTCCATCAGCACAGCCTACATGGAACTGAGCAGGCTGAGATTTGACGACACGGCCGTGTTTTACTGTGCGAGAGATTGGGGTGGCTACAGTTCTTACTACTACGGTATGGACGTCTGGGGCCAAGGGACCACGGTCACCGTCTCTTCA |
| | | | SEQ ID NO: 27275 | SEQ ID NO: 31281 |

FIGURE 50

| | | | | |
|---|---|---|---|---|
| | | AA | DIVMTQTPLSLSVTPGQPASISCKSSQSLLHSDGKTFLYWYLQKPGQPPQLLIYEISNRFSGVPDRFSGSGSGTDFTLKISRVEAEDVGIYYCMQSTQLPRTFGQGTKVEIK | QVHLVQSGAEVKKPGASVKVSCKASGYTFTGYYIHWVRQAPGQGLEWMGWINPYSGDTKYAQKFQGRVTMTRDTSISTAYMELSRLRFDDTAVFYCARDWGGYSSYYYGMDVWGQGTTVTVSS |
| | | | SEQ ID NO: 27276 | SEQ ID NO: 31282 |
| iPS:436630 | 21-225_227G3 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGCAGATTTTGCAACTTATTACTGTCTACACCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGTTGGAAGTAATCAATACTATGCAGACTCCGTGAAGGGCCGATTCACCATCTCCAGAGACAATTCCAAGAACACGCTGTATCTGCAAATGAGCAGCCTGAGAGCCGAGGACACGGCTGTGTATTACTGTGCGAGAGAAGTGGGATTCACTGAGGACTACTGGGGCCAGGGAACCCTGGTCACCGTCTCCTCC |
| | | | SEQ ID NO: 27277 | SEQ ID NO: 31283 |
| | | AA | DIQMTQSPSSLSASVGDRVTITCRASQGIRNDLGWYQQKPGKAPKRLIYAASSLQSGVPSRFSGSGSGTEFTLTISSLQPADFATYYCLHHNSYPFTFGPGTKVDIK | QVQLVESGGGVVQPGRSLRLSCAASGFTFSSYGMHWVRQAPGKGLEWVAVIWYVGSNQYYADSVKGRFTISRDNSKNTLYLQMSSLRAEDTAVYYCAREVGFTEDYWGQGTLVTVSS |
| | | | SEQ ID NO: 27278 | SEQ ID NO: 31284 |

FIGURE 50

| | | | | |
|---|---|---|---|---|
| iPS:436632 | 21-225_227E4 | NA | GACATCCTGATGACCCAGTCTCCATCTTCCGTGTCTGCATCTGTAGGAGACAGAGTCACCATCACTTGTCGGGCGAGTCAGGGTATTATCAACTGGTTAGCCTGGTATCAGCAGAAACCAGGGAAAGCCCCTAAGCTCCTGATCTATGCTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAATTTACTATTGTCAACAGGCTAACAGTTTCCCGTGGACGTTCGGCCAAGGGACCAAGGTGGAAATCAAA | GAGGGGCAGCTATTGGAGTCTGGGGGAGGCTTGGTACAGCCTGGGGGGTCACTGAGACTCTCCTGTACAGCCTCTGGATTCACCTTTAGCACTTTTGCCATGACCTGGGTCCGCCAGGCTCCAGGGAGGGGGCTGGAGTGGGTCTCAGTTATTAGTGGTAGAGGTGGTAGCTCATTCTACGCAGACTCCGTGAAGGGCCGGTTCACCATCTCCAGAGACAATACCAAGAACACGCTGTATCTGCAAATGAACAGCCTGAGAGCCGAGGACACGGCCGTATATTACTGTGCGAAAGATCAACTATGGTTTGACTACTGGGGCCAGGGAACCCTGGTCACCGTCTCCTCA |
| | | | SEQ ID NO: 27279 | SEQ ID NO: 31285 |
| | | AA | DILMTQSPSSVSASVGDRVTITCRASQGIINWLAWYQQKPGKAPKLLIYAASSLQSGVPSRFSGSGSGTDFTLTISSLQPEDFAIYYCQQANSFPWTFGQGTKVEIK | EGQLLESGGGLVQPGGSLRLSCTASGFTFSTFAMTWVRQAPGRGLEWVSVISGRGGSSFYADSVKGRFTISRDNTKNTLYLQMNSLRAEDTAVYYCAKDQLWFDYWGQGTLVTVSS |
| | | | SEQ ID NO: 27280 | SEQ ID NO: 31286 |
| iPS:436634 | 21-225_227H5 | NA | GACATCCAGATGACCCAGTCTCCATCCTCCCTGTCTGCATCTGTAGGAGACAGAGTCACCATCACTTGCCGGGCAAGTCAGGACATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGACTGGGTGGCAGTTATATGGTATGAAGAAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTATGTATTACTGTGCGAGAGAAGTGGGATTCACTGAGGACTACTGGGGCCAGGGAACCCTGGTCACCGTCTCCTCA |
| | | | SEQ ID NO: 27281 | SEQ ID NO: 31287 |

FIGURE 50

| | | | | |
|---|---|---|---|---|
| | | AA | DIQMTQSPSSLSASVGDRVTITCRASQDIRNDLG WYQQKPGKAPKRLIYAASSLQSGVPSRFSGSGS GTEFTLTISSLQPEDFATYYCLQHNSYPFTFGPGT KVDIK | QVQLVESGGGVVQPGRSLRLSCAASGFTFSNYGM HWVRQAPGKGLDWVAVIWYEESNKYYADSVKGR FTISRDNSKNTLYLQMNSLRAEDTAMYYCAREVGF TEDYWGQGTLVTVSS |
| | | | SEQ ID NO: 27282 | SEQ ID NO: 31288 |
| **iPS:436636** | **21-225_227E6** | NA | GACATCGTGATGACCCAGTCTCCAGACTCCCT GACTGTGTCTCTGGGCGAGAGGGCCACCATCA ACTGCAAGTCCAGCCAGAGTGTTTTATACAGC TCCAACGATAAGAACTACTTAGCTTGGTACCA GCAGAAACCAGGACAGCCTCCTAAGATGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATATTACTCCGTGCAGTTTTGGCCAGGG GACCAAGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAACACAGGCTATGCACAGAAGTTCCAGGGCAG AGTCACCATGACCAGGAACACCTCCATAAGCAC AGCCTACATGGAGCTGAGCAGCCTGAGATCTGA GGACACGGCCGTGTATTACTGTGCCTATAGCAGT GGCTGGTACAAGTTTGACTACTGGGGCCAGGGA ACCCTGGTCACCGTCTCCTCA |
| | | | SEQ ID NO: 27283 | SEQ ID NO: 31289 |
| | | AA | DIVMTQSPDSLTVSLGERATINCKSSQSVLYSSN DKNYLAWYQQKPGQPPKMLIYWASTRESGVPD RFSGSGSGTDFTLTISSLQAEDVAVYYCQQYYIT PCSFGQGTKLEIK | QVQLVQSGAEVKKPGASVKVSCKASGYTFTNYDIN WVRQATGQGLEWMGWMHPNSGNTGYAQKFQGR VTMTRNTSISTAYMELSSLRSEDTAVYYCAYSSGW YKFDYWGQGTLVTVSS |
| | | | SEQ ID NO: 27284 | SEQ ID NO: 31290 |

FIGURE 50

| | | | | |
|---|---|---|---|---|
| **iPS:436638** | **21-225_227C7** | NA | GACATCGTGATGACCCAGTCTCCAGACTCCCTGGCTGTGTCTCTGGGCGAGAGGGCCACCATCAACTGCAGGTCCAGCCAGATTGTTTTATCCGACTCCAACAATAACAACTACTTAGCTTGGTACCAGCAGAAACCAGGACAGCCTCCTAACCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTTCTGTCAGCAATATTATAGTTCTCCTCCGACGTTCGGCCAAGGGACCAAGGTGGAAATCAAA | CAGGTGCAGCTGGTGCAGTCTGGGGCTGAGGTGAAGAAGCCTAGGGCCTCAGTGAAGGTCTCCTGCAAGGCTTCTGGACACACCTTCACCAATTATGATATCAACTGGGTGCGACAGGCCACTGGACAAGGGCTTGAGTGGATGGGATGGATGCATCCTAACAGTGGTAACACAGGCTATGCACAGAAGTTCCAGGGCAGAGTCACCATGACCAGGAACACCTCCATAAGCACCGCCTACATGGAGCTGAGCAGCCTGAGATCTGAGGACACGGCCGTGTATTACTGTGCATATAGCAGTGGCTGGTACCGCTTTGACTACTGGGGCCAGGGAACCCTGGTCACCGTCTCCTCA |
| | | | SEQ ID NO: 27285 | SEQ ID NO: 31291 |
| | | AA | DIVMTQSPDSLAVSLGERATINCRSSQIVLSDSNNNNYLAWYQQKPGQPPNLLIYWASTRESGVPDRFSGSGSGTDFTLTISSLQAEDVAVYFCQQYYSSPPTFGQGTKVEIK | QVQLVQSGAEVKKPRASVKVSCKASGHTFTNYDINWVRQATGQGLEWMGWMHPNSGNTGYAQKFQGRVTMTRNTSISTAYMELSSLRSEDTAVYYCAYSSGWYRFDYWGQGTLVTVSS |
| | | | SEQ ID NO: 27286 | SEQ ID NO: 31292 |
| **iPS:436640** | **21-225_227A8** | NA | GATATTGTGATGACCCAGACTCCACTCTCTCTGTCCGTCACCCCTGGACAGCCGGCCTCCATCTCCTGCAAGTCTAGTCAGAGCCTCCTGCATAGTGATGGAAAGACCTTTTTGTATTGGTATCTGCAGAAGCCAGGCCAGCCCCCACAGCTCCTGATCTATGAAATTTCCAACCGGTTCTCTGGGGTGCCAGATAGGTTCAGTGGCAGCGGGTCAGGGACAGATTTCACACTGAAAATCAGTCGAGTGGAGGCTGAGGATGTTGGGATTTATTACTGCATGCAAAGTACACAGCTTCCTCGGACGTTCGGCCAAGGGACCAGGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATACACTGGGTGCGACAGGCCCCTGGACAAGGGCTTGAGTGGATGGGATGGATCAACCCTTACAGTGGTGACACAAACTATGCACAGAAGTTTCAGGGCAGGGTCACCATGACCAGGGACACGTCCGTCAGCACAGCCTACATGGAACTGAGCAGGCTGAGATTTGACGACACGGCCGTGTTTTACTGTGCGAGAGATTGGGGTGGCTACAGTTCTTACTACTACGGTATGGACGTCTGGGGCCAAGGGACCACGGTCACCGTCTCTTCA |
| | | | SEQ ID NO: 27287 | SEQ ID NO: 31293 |

FIGURE 50

| | | | | |
|---|---|---|---|---|
| | | AA | DIVMTQTPLSLSVTPGQPASISCKSSQSLLHSDGKTFLYWYLQKPGQPPQLLIYEISNRFSGVPDRFSGSGSGTDFTLKISRVEAEDVGIYYCMQSTQLPRTFGQGTRVEIK | QVQLVQSGAEVKKPGASVKVSCKASGYTFTGYYIHWVRQAPGQGLEWMGWINPYSGDTNYAQKFQGRVTMTRDTSVSTAYMELSRLRFDDTAVFYCARDWGGYSSYYYGMDVWGQGTTVTVSS |
| | | | SEQ ID NO: 27288 | SEQ ID NO: 31294 |
| **iPS:436644** | **21-225_227G9** | NA | GACATCGTGATGACCCAGTCTCCAGACTCCCTGGCTGTGTCTCTGGGCGAGAGGGCCACCATCAACTGCAAGTCCAGCCAGAGTGTTTTACACAGCTCCAACAATAACAACTACTTAGCTTGGTATCAGCAGAAACCAGGACAGCCTCCTAAGCTGCTCATTTACTGGGGATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTGCTCCGTACAGTTTTGGCCAGGGGACCAAGTTGGAGATCAAA | CAGGTGCAGCTGGTGCAGTCTGGGGCTGAGGTGAAGAAGCCTGGGGCCTCAGTGAAGGTCTCCTGCAGGGCTTCTGGATACACCTTCACCAATTATGATATCAACTGGGTGCGACAGGCCACTGGACGAGGGCTTGAGTGGATGGGATGGATGTACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGCTTAGCAGTGGCTGGTACTGGTTCGACCCCTGGGGCCAGGGAACCCTGGTCACCGTCTCCTCA |
| | | | SEQ ID NO: 27289 | SEQ ID NO: 31295 |
| | | AA | DIVMTQSPDSLAVSLGERATINCKSSQSVLHSSNNNNYLAWYQQKPGQPPKLLIYWGSTRESGVPDRFSGSGSGTDFTLTISSLQAEDVAVYYCQQYYSAPYSFGQGTKLEIK | QVQLVQSGAEVKKPGASVKVSCRASGYTFTNYDINWVRQATGRGLEWMGWMYPNSGNTGYAQKFQGRVTMTRNTSISTAYMELSSLRSEDTAVYYCALSSGWYWFDPWGQGTLVTVSS |
| | | | SEQ ID NO: 27290 | SEQ ID NO: 31296 |

FIGURE 50

| | | | | |
|---|---|---|---|---|
| **iPS:436646** | **21-225_227D11** | NA | GACATCGTGATGACCCAGTCTCCAGACTCCCTGGCTGTGTCTCTGGACGAGAGGGCCACCATCAACTGCAAGTCCAGCCAGAGTGTTTTACACAGTTCCAACAATAATAACTACTTAGCTTGGTACCAGCAGAAGCCAGGACAGCCTCCTAAGCTGCTCATTTACTGGGCATCTACCCGGGAATCCGGGATCCCTGACCGATTCAGTGGCAGCGGGTCTGGGACAGATTTCACTCTCACCATCAGCAGCCTGCAGGCTGAGGATGTGGCAGTTTATTACTGTCAGCAATATTATAATACTCCGTGCAGTTTTGGCCAGGGGACCAAGCTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCCGAGTCACCATGACCAGGAACACCTCCATAAGCACAGCCTACATGGAACTGAGCAGCCTGAGATCTGAGGACACGGCCGTGTATTACTGTGCGTATAGCAGTGGCTGGTACTACTTTGACTACTGGGGCCAGGGAACCCTGGTCACCGTCTCCTCA |
| | | | SEQ ID NO: 27291 | SEQ ID NO: 31297 |
| | | AA | DIVMTQSPDSLAVSLDERATINCKSSQSVLHSSNNNNYLAWYQQKPGQPPKLLIYWASTRESGIPDRFSGSGSGTDFTLTISSLQAEDVAVYYCQQYYNTPCSFGQGTKLEIK | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCAYSSGWYYFDYWGQGTLVTVSS |
| | | | SEQ ID NO: 27292 | SEQ ID NO: 31298 |
| **iPS:436648** | **21-225_227F11** | NA | GATGTTGTGATGACTCAGTCTCCACTCTCCCTGCCCGTCACCCCTGGAGAGCCGGCCTCCATCTCCTGCTGGTCTAGTCAGAGCCTCCTGCATAGTAATGGATACAACTATTTGGATTGGTACCTGCAGAAGCCAGGGCAGTCTCCACAGGTCCTGATCTATTTGGGTTCTAATCGGGCCTCCGGGGTCCCTGACAGGTTCAGTGGCAGTGGATCAGGCACAGATTTTACACTGAAAATCAGCAGAGTGGAGGCTGAGGATGTTGGGGTTTATTACTGCATGCAAGCTCTACAAACTCCTCTCACCTTCGGCCAAGGGACACGACTGGAGATTAAA | GAGGTGCAGCTGGTGGAGTCTGGGGGAGGCCTGGTCAAGCCTGGGGGGTCCCTGAGACTCTCCTGTGCAGTCTCTGGATTCACCTTCAGTACCTATAGCATGAACTGGGTCCGCCAGGCTCCAGGGAAGGGGCTGGAGTGGGTCTCATCCATTAGTAGTAGTATTAATTACATGTACTACGCAGACTCAGTGAAGGGCCGATTCACCATCTCCAGAGACAACGCCAAGAACTCACTGTATCTGCAAATGAACAGCCTGAGAGCCGAGGACTCGGCTGTGTATTACTGTGCGAGATTAGGGGTCTACTGGGGCCAGGGAACCCTGGTCACCGTCTCCTCA |
| | | | SEQ ID NO: 27293 | SEQ ID NO: 31299 |

FIGURE 50

| | | | | |
|---|---|---|---|---|
| | | AA | DVVMTQSPLSLPVTPGEPASISCWSSQSLLHSNGYNYLDWYLQKPGQSPQVLIYLGSNRASGVPDRFSGSGSGTDFTLKISRVEAEDVGVYYCMQALQTPLTFGQGTRLEIK | EVQLVESGGGLVKPGGSLRLSCAVSGFTFSTYSMNWVRQAPGKGLEWVSSISSSINYMYYADSVKGRFTISRDNAKNSLYLQMNSLRAEDSAVYYCARLGVYWGQGTLVTVSS |
| | | | SEQ ID NO: 27294 | SEQ ID NO: 31300 |
| **iPS:436650** | **21-225_227C12** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATAGTTACCCATTCACTTTCGGCCCTGGGACCAAAGTGGATATCAAA | CAGGTGCAGCTGGTGGAGTCTGGGGGAGGCGTGGTCCAGCCTGGGAAGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATATTGGAAGTAATCAATACTATGCGGACTCCGTGAAGGGCCGATTCACCATCTCCAGAGACAATTCCAAGAACACGCTGTATCTGCAAATGAGCAGCCTGAGAGCCGAGGACACGGCTGTGTATTACTGTGCGAGAGAAGTGGGATTCACTGAGGACTACTGGGGCCAGGGAACCCTGGTCACCGTCTCCTCC |
| | | | SEQ ID NO: 27295 | SEQ ID NO: 31301 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPFTFGPGTKVDIK | QVQLVESGGGVVQPGKSLRLSCAASGFTFSNYGMHWVRQAPGKGLEWVAVIWYIGSNQYYADSVKGRFTISRDNSKNTLYLQMSSLRAEDTAVYYCAREVGFTEDYWGQGTLVTVSS |
| | | | SEQ ID NO: 27296 | SEQ ID NO: 31302 |

FIGURE 50

| | | | | |
|---|---|---|---|---|
| **iPS:436652** | **21-225_146B11** | NA | TCCTATGAGCTGACTCAGCCACCCTCAGTGTC CGTGTCCCCAGGACAGACAGCCAGCATCACCT GCTCTGGAGATAAATTGGGGGATAAATATGCT TCCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGATCCAGGCTATGGATGAGGCTGACTATT ACTGTCAGGCGTGGGACAGCAGCACTGTGGTA TTCGGCGGAGGGACCAAGCTGACCGTCCTA | GAGGTGCAACTGTTGGAGTCTGGGGGAGGCTTG GTACAGCCTGGGGGGTCCCTGAGACTCTCCTGTG CAGCTTCTGGATTCACCTTTAGCAGCTATGCCAT GAGCTGGGTCCGCCAGGCTCCAGGGAAGGGGCT GGAGTGGGTCTCAGTTATTAGTGGTGGTGGTAGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
| | | | SEQ ID NO: 27297 | SEQ ID NO: 31303 |
| | | AA | SYELTQPPSVSVSPGQTASITCSGDKLGDKYASW YQQKPGQSPVLVIYQDSKRPSGIPERFSGSNSGN TATLTISGIQAMDEADYYCQAWDSSTVVFGGGT KLTVL | EVQLLESGGGLVQPGGSLRLSCAASGFTFSSYAMS WVRQAPGKGLEWVSVISGGGSSTYYADSVKGRFTI SRDNSKNTLYLQMNSLRAEDTAVYYCAKWRGNPT DYGMDVWGQGTTVTVSS |
| | | | SEQ ID NO: 27298 | SEQ ID NO: 31304 |
| **iPS:436654** | **21-225_146C11** | NA | TCCTATGAACTGACTCAGCCACCCTCAGTGTC CGTGTCCCCAGGACAGACAGCCAGCATCACCT GCTCTGGAGATAAATTGGGGGATAAATATGCT TCCTGGTATCAGCAGAAGCCAGGCCAGTCCCC TGTGCTGGTCATCTATCAAGATAGCAAGCGGC CCTCAGGGATCCCTGAGCGATTCTCTGGCTCC AACTCTGGGAACACAGCCACTCTGACCATCAG CGGGATCCAGGCTATGGATGAGGCTGACTATT ACTGTCAGGCGTGGGACAGCAGCACTGTGGTA TTCGGCGGAGGGACCAAGCTGACCGTCCTA | GAGGTGCAACTGTTGGAGTCTGGGGGAGGCTTG GTACAGCCGGGGGGGTCCCTGAGACTCTCCTGTG CAGCCTCTGGAATCACCTTTAGCAGCTATGCCAT GAGCTGGGTCCGCCAGGCTCCAGGGAAGGGGCT GGAGTGGGTCTCAGTTATTAGTGGTGGTGGTAGT AGCACATACTACGCAGACTCCGTGAAGGGCCGG TTCACCATCTCCAGAGACAATTCCAAGAACACGC TGTATCTGCAAATGAACAGCCTGAGAGCCGAGG ACACGGCCGTATATTACTGTGCGAAATGGCGAG GTAACCCCACTGACTACGGTATGGACGTCTGGGG CCAAGGGACCACGGTCACCGTCTCCTCA |
| | | | SEQ ID NO: 27299 | SEQ ID NO: 31305 |

FIGURE 50

| | | | | |
|---|---|---|---|---|
| | | AA | SYELTQPPSVSVSPGQTASITCSGDKLGDKYASWYQQKPGQSPVLVIYQDSKRPSGIPERFSGSNSGNTATLTISGIQAMDEADYYCQAWDSSTVVFGGGTKLTVL | EVQLLESGGGLVQPGGSLRLSCAASGITFSSYAMSWVRQAPGKGLEWVSVISGGGSSTYYADSVKGRFTISRDNSKNTLYLQMNSLRAEDTAVYYCAKWRGNPTDYGMDVWGQGTTVTVSS |
| | | | SEQ ID NO: 27300 | SEQ ID NO: 31306 |
| iPS:436658 | 21-225_146A2 | NA | TCCTATGAGCTGACTCAGCCACCCTCAGTGTCCGTGTCCCCAGGACAGACAGCCAGCATCACCTGCTCTGGAGATAAATTGGGGGATAAATATGCTTCCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGATCCAGGCTATGGATGAGGCTGACTATTACTGTCAGGCGTGGGACAGCAGCACTGTGGTATTCGGCGGAGGGACCAAGCTGACCGTCCTA | GAGGTGCAACTGTTGGAGTCTGGGGGAGGCCTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTATTAGTGGTGGTGGTAGTAGCACATACTACGCAGACTCCGTGAAGGGCCGGTTCACCATCTCCAGAGACAATTCCAAGAACACGCTGCATCTGCAAATGAACAGCCTGAGAGCCGAGGACACGGCCGTATATTACTGTGCGAAATGGCGAGGTAACCCCACTGACTACGGTATGGACGTCTGGGGCCAAGGGACCACGGTCACCGTCTCCTCA |
| | | | SEQ ID NO: 27301 | SEQ ID NO: 31307 |
| | | AA | SYELTQPPSVSVSPGQTASITCSGDKLGDKYASWYQQKPGQSPVLVIYQDSKRPSGIPERFSGSNSGNTATLTISGIQAMDEADYYCQAWDSSTVVFGGGTKLTVL | EVQLLESGGGLVQPGGSLRLSCAASGFTFSSYAMSWVRQAPGKGLEWVSVISGGGSSTYYADSVKGRFTISRDNSKNTLHLQMNSLRAEDTAVYYCAKWRGNPTDYGMDVWGQGTTVTVSS |
| | | | SEQ ID NO: 27302 | SEQ ID NO: 31308 |

FIGURE 50

| | | | | |
|---|---|---|---|---|
| iPS:436660 | 21-225_146D8 | NA | CAGTCTGTACTGACTCAGCCACCCTCAACGTCTGGGACCCCCGGGCAGAGGGTCACCATCTCTTGTTCTGGAAGCAGCTCCTACATCGGAAGTAATACTGTAGACTGGTACCAGCAGCTCCCAGGAACGGCCCCCAAACTCCTCATCTATAGTAATAATCAGCGGCCCTCAGGGGTCCCTGACCGATTCTCTGGCTCCAAGTCTGGCACCTCAGCCTCCCTGGCCATCAGTGGGCTCCAGTCTGAGGATGAGGCTGATTATTACTGTGCAGCATGGGATGACAGCCTTAATGGCGTGGTATTCGGCGGAGGGACCAAACTGACCGTCCTA | GAGGTGCAGCTGGTGGAGTCTGGGGGAGGCTTGGTACAGCCTGGGGGGTCCCTGAGACTCTCCTGTGCAGCCTCTGGATTCACCTTCAGTAACTATAACATGAACTGGGTCCGCCAGGCTCCAGGGAAGGGGCTGGAGTGGGTTTCATACATTAGTAGAAGTAGTAATACCAAATACTATGTAGACTCTGTGAAGGGCCGATTCACCATCTCCAGAGACAATGCCAAGAACTCATTGTATCTGCAAATGAACAGCCTGAGAGACGAGGACACGGCTGTGTATTACTGTGCGAGAGATAGGAGTGGGAGCTACGGGTACTTCTACTACTACGGTTTGGACGTCTGGGGCCAAGGGACCACGGTCACCGTCTCCTCA |
| | | | SEQ ID NO: 27303 | SEQ ID NO: 31309 |
| | | AA | QSVLTQPPSTSGTPGQRVTISCSGSSSYIGSNTVDWYQQLPGTAPKLLIYSNNQRPSGVPDRFSGSKSGTSASLAISGLQSEDEADYYCAAWDDSLNGVVFGGGTKLTVL | EVQLVESGGGLVQPGGSLRLSCAASGFTFSNYNMNWVRQAPGKGLEWVSYISRSSNTKYYVDSVKGRFTISRDNAKNSLYLQMNSLRDEDTAVYYCARDRSGSYGYFYYYGLDVWGQGTTVTVSS |
| | | | SEQ ID NO: 27304 | SEQ ID NO: 31310 |
| iPS:436662 | 21-225_147D7 | NA | TCCTATGAGTTGACTCAGCCACCCTCAGTGTCCGTGTCCCCAGGACAGACAGCCAGCATCACCTGCTCTGGAGATAAATTGGGGGATAAATTTGCTTGCTGGTATCAGCAGAAACCAGGCCAGTCCCCTGTACTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGGAACACCGCTGTCTTCGGAACTGGGACCAAGGTCACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCAGTTATGATATCAACTGGGTGCGACAGGCCACTGGACAAGGGCTTGAGTGGATGGGATGGATGAACCCTAATAGTGGTAACACAGGCTATGCACAGAAGTTCCAGGGCAGAGTCACCATGACCAGGAACACCTCCATAAGCACAGCCTATATGGAGCTGAGAAGCCTGAGATCTGAGGACACGGCCGTGTATTACTGTGCGAGAGCGGATATTGTATTAGTACCAGCTGCTATCCCTTATAATTACTACTTCGCTATGGACGTCTGGGGCCAAGGGACCACGGTCACCGTCTCCTCA |

FIGURE 50

| | | | | |
|---|---|---|---|---|
| | | | SEQ ID NO: 27305 | SEQ ID NO: 31311 |
| | | AA | SYELTQPPSVSVSPGQTASITCSGDKLGDKFACWYQQKPGQSPVLVIYQDRKRPSGIPERFSGSNSGNTATLTISGTQAMDEADYYCQAWDRNTAVFGTGTKVTVL | QVQLVQSGAEVKKPGASVKVSCKASGYTFTSYDINWVRQATGQGLEWMGWMNPNSGNTGYAQKFQGRVTMTRNTSISTAYMELRSLRSEDTAVYYCARADIVLVPAAIPYNYYFAMDVWGQGTTVTVSS |
| | | | SEQ ID NO: 27306 | SEQ ID NO: 31312 |
| iPS:436664 | 21-225_147E7 | NA | TCCTATGAGCTGACTCAGCCACCCTCAGTGTCCGTGTCCCCAGGACAGACAGCCAGCATCACCTGCTCTGGAGATAAATTGGGGGATAAATATGCTTCCTGGTATCAGCAGAAGCCAGGCCAGTCCCCTGTGCTGGTCATCTATCAAGATAGCAAGCGCCCCTCAGGGATCCCTGAGCGATTCTCTGGCTCCAACTCTGGGAACACAGCCACTCTGACCATCAGCGGGATCCAGGCTATGGATGAGGCTGACTATTACTGTCAGGCGTGGGACAGCAGCACTGTGGTATTCGGCGGAGGGACCAAGCTGACCGTCCTA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTATTAGTGGTGGTGGTAGTAGTACATACTACGCAGACTCCGTGAAGGGCCGGTTCACCCTCTCCAGAGACAATTCCAAGAACACGCTGTATCTGCAAATGAACAGCCTGAGAGCCGAGGACACGGCCGTTTTTTACTGTGCGAAATGGCGAGGTAACCCCACTGACTACGGTATGGACGTCTGGGGCCAAGGGACCACGGTCACCGTCTCCTCA |
| | | | SEQ ID NO: 27307 | SEQ ID NO: 31313 |
| | | AA | SYELTQPPSVSVSPGQTASITCSGDKLGDKYASWYQQKPGQSPVLVIYQDSKRPSGIPERFSGSNSGNTATLTISGIQAMDEADYYCQAWDSSTVVFGGGTKLTVL | EVQLLESGGGLVQPGGSLRLSCAASGFTFSSYAMSWVRQAPGKGLEWVSVISGGGSSTYYADSVKGRFTLSRDNSKNTLYLQMNSLRAEDTAVFYCAKWRGNPTDYGMDVWGQGTTVTVSS |
| | | | SEQ ID NO: 27308 | SEQ ID NO: 31314 |

FIGURE 50

| | | | | |
|---|---|---|---|---|
| **iPS:436666** | **21-225_147B8** | NA | TCCTATGAACTGACTCAGCCACCCTCAGTGTCCGTGTCCCCAGGACAGACAGCCAGTATCACCTGCTCTGGAGATAAATTGGGGGATAAATATGTTTGCTGGTATCAGCAGAAGCCAGGCCAGTCCCCTGAGCTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGGCAGTAACACTGCTGTGGTATTCGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCGACTACTATTTGCACTGGGTGCGACAGGCCCCTGGACAAGGGCTTGAGTGGATGGGATGGATCAACCCTAACAGTGGTGACACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGAGATCGGGACTCTGGTTCGGGGAGTTACCCCTACTACTACTACTACGGTATGGACGTCTGGGGCCAAGGGACCACGGTCACCGTCTCCTCA |
| | | | SEQ ID NO: 27309 | SEQ ID NO: 31315 |
| | | AA | SYELTQPPSVSVSPGQTASITCSGDKLGDKYVCWYQQKPGQSPELVIYQDRKRPSGIPERFSGSNSGNTATLTISGTQAMDEADYYCQAWGSNTAVVFGGGTKLTVL | QVQLVQSGAEVKKPGASVKVSCKASGYTFTDYYLHWVRQAPGQGLEWMGWINPNSGDTNYAQKFQGRVTMTRDTSISTAYMELSRLRSDDTAVYYCARDRSGSGSYPYYYYYGMDVWGQGTTVTVSS |
| | | | SEQ ID NO: 27310 | SEQ ID NO: 31316 |
| **iPS:436668** | **21-225_147B9** | NA | TCCTATGAGCTGACTCAGCCGCCCTCAGTGTCCGTGTCCCCAGGACAGACAGCCAGCATCACCTGCTCTGGAGATAAATTGGGGGATAAATATGTTTCCTGGTATCAGCAGAGGCCAGGCCAGTCCCCTGTGCTGGTCATCTATCAAGATAGGAAGCGGCCCTCAGGGATCCCTGAGCGATTCTCTGGCTCCAACTCTGGGAACACAGCCACTCTGACCATCAGCGGGACCCTGGCTGTGGATGAGGCTGACTATTACTGTCTGGCGTGGGACAGCAGCACTTTTGTGGTATTCGGCGGAGGGACCAAGTTGACCGTCCTA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGTGACTACGGTGACCCCCCCTACTACTACTACTACGGTATGGACGTCTGGGGCCAAGGGACCACGGTCACCGTCTCCTCA |

FIGURE 50

| | | | | |
|---|---|---|---|---|
| | | | SEQ ID NO: 27311 | SEQ ID NO: 31317 |
| | | AA | SYELTQPPSVSVSPGQTASITCSGDKLGDKYVSWYQQRPGQSPVLVIYQDRKRPSGIPERFSGSNSGNTATLTISGTLAVDEADYYCLAWDSSTFVVFGGGTKLTVL | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARDRDYGDPPYYYYYGMDVWGQGTTVTVSS |
| | | | SEQ ID NO: 27312 | SEQ ID NO: 31318 |
| iPS:436670 | 21-225_147D9 | NA | TCCTATGAGCTGACTCAGCCACCCTCAGTGTCCGTGTCCCCAGGACAGACAGCCAGCATCACCTCCTCTGGAGATAAATTGGGTAATAAATATGTTTGCTGGTATCAGCAGAGGCCAGGCCAGTCCCCTGTGCTGGTCATCTATCAAGATAGCAAGCGGCCCTCAGGGATCCCTGAGCGATTCTCTGGCTCCAACTCCGGGAACACAGCCACTCTGACCATCAGCGGGACCCAGGCTATGGATGAGGCTGACTATTACTGTCAGGCGTGGGACAGGAACACTTATGTGGTGTTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGATATATGGTTTGATGGCAGTAATAAATACTATGTAGACTCCGTGAAGGACCGATTCACCATCTCCAGAGACAATTCCAAGAACACGCTGTATCTGCAAATGAACAGCCTGAGAGCCGAGGACACGGCTGTTTATTACTGTGCGAGAGATCGGGTGGAGGGTTCGGGGACTCCCTACTACTACTACGGTATGGACGTCTGGGGCCAAGGGACCACGGTCACCGTCTCCTCA |
| | | | SEQ ID NO: 27313 | SEQ ID NO: 31319 |
| | | AA | SYELTQPPSVSVSPGQTASITSSGDKLGNKYVCWYQQRPGQSPVLVIYQDSKRPSGIPERFSGSNSGNTATLTISGTQAMDEADYYCQAWDRNTYVVFGGGTKLTVL | QVQLVESGGGVVQPGRSLRLSCAASGFTFSSYGMHWVRQAPGKGLEWVADIWFDGSNKYYVDSVKDRFTISRDNSKNTLYLQMNSLRAEDTAVYYCARDRVEGSGTPYYYYGMDVWGQGTTVTVSS |
| | | | SEQ ID NO: 27314 | SEQ ID NO: 31320 |

FIGURE 50

| | | | | |
|---|---|---|---|---|
| **iPS:436672** | **21-225_147F9** | NA | TCCTATGAGCTGACTCAGCCACCCTCAGTGTCCGTGTCCCCAGGACAGACAGCCAGCATCACCTGTTCTGGAGATGAATTGGGGAATAAATATGCTTGCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGCACAGCAGCACTGTGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTACCTATGGCATGCACTGGGTCCGCCAGGCTCCAGGCAAGGGGCTGGAGTGGGTGGCAGTTATATGGTATGGTGGAAGTGATAAAGACTATGCAGACTCCGTGAAGGGCCGATTCACCATCTCCAGAGACATTTCCAAGAACACGCTGTATCTGCAAATGAACAGCCTGAGAGCCGAGGACACGGCTGTGTATTACTGTGCGAGAGATCGGGATTATTGTAGTGGTGGTACTTGTCCTTACTACTACTACTACGGTATGGACGTCTGGGGCCAAGGGACCACGGTCACCGTCTCCTCA |
| | | | SEQ ID NO: 27315 | SEQ ID NO: 31321 |
| | | AA | SYELTQPPSVSVSPGQTASITCSGDELGNKYACWYQQKPGQSPVLVIYQDSKRPSGIPERFSGSNSGNTATLTISGTQAMDEADYYCQAWHSSTVVFGGGTKLTVL | QVQLVESGGGVVQPGRSLRLSCAASGFTFSTYGMHWVRQAPGKGLEWVAVIWYGGSDKDYADSVKGRFTISRDISKNTLYLQMNSLRAEDTAVYYCARDRDYCSGGTCPYYYYYGMDVWGQGTTVTVSS |
| | | | SEQ ID NO: 27316 | SEQ ID NO: 31322 |

FIGURE 50

| | | | | |
|---|---|---|---|---|
| **iPS:436674** | **21-225_147G9** | NA | TCCTATGAGCTGACTCAGCCACCCTCAGTGTCCGTGTCCCCAGGACAGACAGCCAGCATCACCTGCTCTGGAGATAAATTGGGGGATAAATATGCTTGCTGGTATCAGCAGAAGCCAGGCCAGTCCCCTGTGCTGGTCATCTATCAAGATAGGAAGCGGCCCTCAGGGATCCCTGAGCGATTCTCTGGCTCCAACTCTGGGAACACAGCCACTCTGACCATTAGCGGGACCCAGGCTATGGATGAGGCTGACTATTACTGTCAGGCGTGGCACAGCAGTACTGTGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTACCTATGGCATGCACTGGGTCCGCCAGGCTCCAGGCAAGGGGCTGGAGTGGGTGGCAGTTATATGGTATGGTGGAAGTGATAAAGACTATGCAGACTCCGTGAAGGGCCGATTCACCATCTCCAGAGACATTTCCAAGAACACGCTGTATCTGCAAATGAACAGCCTGAGAGCCGAGGACACGGCTGTGTATTACTGTGCGAGAGATCGGGATTATTGTAGTGGTGGTAGCTGCCCTTACTACTACTACTACGGTATGGACGTCTGGGGCCAAGGGACCACGGTCACCGTCTCCTCA |
| | | | SEQ ID NO: 27317 | SEQ ID NO: 31323 |
| | | AA | SYELTQPPSVSVSPGQTASITCSGDKLGDKYACWYQQKPGQSPVLVIYQDRKRPSGIPERFSGSNSGNTATLTISGTQAMDEADYYCQAWHSSTVVFGGGTKLTVL | QVQLVESGGGVVQPGRSLRLSCAASGFTFSTYGMHWVRQAPGKGLEWVAVIWYGGSDKDYADSVKGRFTISRDISKNTLYLQMNSLRAEDTAVYYCARDRDYCSGGSCPYYYYYGMDVWGQGTTVTVSS |
| | | | SEQ ID NO: 27318 | SEQ ID NO: 31324 |

FIGURE 50

| | | | | |
|---|---|---|---|---|
| **iPS:436676** | **21-225_147E11** | NA | TCCTATGAGCTGACTCAGCCACCCTCAGTGTCCGTGTCCCCAGGACAGACAGCCAGCATCACCTGCTCTGGAGATAAATTGGGGGATAAATATGCTTCCTGGTATCAGCAGAAGCCAGGCCAGTCCCCTGTGTTGGTCATCTATCAAGATAGCAAGCGGCCCTCAGGGATCCCTGAGCGATTCTCTGGCTCCAACTCTGGGAACACAGCCACTCTGACCATCAGCGGGATCCAGGCTATGGATGAGGCTGACTATTACTGTCAGGCGTGGGACAGCAGCACTGTGGTATTCGGCGGAGGGACCAAGCTGACCGTCCTA | GAGGTGCAACTGTTGGAGTCTGGGGGAGGCTTGGTACAGCCTGGGGGGTCCCTGAGACTCTCCTGTGCAGCCTCTGGATTCACCTTTAGCAACTATGTCATGAGCTGGGTCCGCCAGGCTCCAGGGAAGGGGCTGGAGTGGGTCTCAGTTATTAGTGGTGGTGGTAGTAGTACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATGGCGAGGTAACCCCACTGACTACGGTATGGACGTCTGGGGCCAAGGGACCACGGTCACCGTCTCCTCA |
| | | | SEQ ID NO: 27319 | SEQ ID NO: 31325 |
| | | AA | SYELTQPPSVSVSPGQTASITCSGDKLGDKYASWYQQKPGQSPVLVIYQDSKRPSGIPERFSGSNSGNTATLTISGIQAMDEADYYCQAWDSSTVVFGGGTKLTVL | EVQLLESGGGLVQPGGSLRLSCAASGFTFSNYVMSWVRQAPGKGLEWVSVISGGGSSTYYADSVKGRFTISRDNSKNTLYLQMNSLRAEDTAVYYCAKWRGNPTDYGMDVWGQGTTVTVSS |
| | | | SEQ ID NO: 27320 | SEQ ID NO: 31326 |
| **iPS:436678** | **21-225_147B12** | NA | TCCTATGAGCTGACTCAGCCACCCTCAGTGTCCGTGTCCCCAGGACAGACAGCCAGCATCACCTGCTCTGGAGATAAATTGGGGGATAAATATGCTTCCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACGCAGCCACTCTGACCATCAGCGGGATCCAGGCTATGGATGAGGCTGACTATTACTGTCAGGCGTGGGACAGCAGCACTGTGGTATTCGGCGGAGGGACCAAGCTGACCGTCCTA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTATTAGTGGTGGTGGTAGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATGGCGAGGTAACCCCACTGACTACGGTATGGACGTCTGGGGCCAAGGGACCACGGTCACCGTCTCCTCA |
| | | | SEQ ID NO: 27321 | SEQ ID NO: 31327 |

FIGURE 50

| | | | | |
|---|---|---|---|---|
| | | AA | SYELTQPPSVSVSPGQTASITCSGDKLGDKYASWYQQKPGQSPVLVIYQDSKRPSGIPERFSGSNSGNAATLTISGIQAMDEADYYCQAWDSSTVVFGGGTKLTVL | EVQLLESGGGLVQPGGSLRLSCAASGFTFSSYAMSWVRQAPGKGLEWVSVISGGGSSTYYADSVKGRFTISRDNSKNTLYLQMNSLRAEDTAVYYCAKWRGNPTDYGMDVWGQGTTVTVSS |
| | | | SEQ ID NO: 27322 | SEQ ID NO: 31328 |
| **iPS:436680** | **21-225_147H12** | NA | CAGTCTGTGTTGACTCAGCCACCCTCAGCGTCTGGGACCCCCGGGCAGAGGGTCACCATCTCTTGTTCTGGAAGCAGCTCCAACATCGGAAGTTATGCTGTAAACTGGTATCAGCAGCTCCCAGGAACGGCCCCCAAACTCCTCATTTATAGTAATAATCACCGGCCCTCAGGGGTCCCTGACCGATTCTCTGGCTCCAAGTCTGGCACCTCAGCCTCCCTGGCCATCAGTGGGCTCCAGTCTGAGGATGAGGCTGATTATTACTGTGAAGCATGGGATGACAGCCTGAATGGTCCGGTATTCGGCGGAGGGACCAAGCTGACCGTCCTA | CAGGTGCAGCTGCAGGAGTCGGGCCCAGGACTGGTGAATCCTTCACAGACCCTGTCCCTCACCTGCGCTGTCTCTGGTGGCTCCATCAGCAGTGGTTATTACCACTGGAGCTGGATCCGCCAGCACCCAGGGAAGGGCCTGGAGTGGATTGGGTACATCTATTACAGTGGGAGCACCTACTACAACCCGTCCCTCAAGAGTCGAGTTACCATATCAGTTGACACGTCTAAGAACCAGTTCTCCCTGAAGCTGACCTCTGTGACTGCCGCGGACACGGCCGTGTATTACTGTGCGAGAGATTGGGGTGGCTACGATTCGAGTGGCTGGTTCGACCCCTGGGGCCAGGGAACCCTGGTCACCGTCTCCTCA |
| | | | SEQ ID NO: 27323 | SEQ ID NO: 31329 |
| | | AA | QSVLTQPPSASGTPGQRVTISCSGSSSNIGSYAVNWYQQLPGTAPKLLIYSNNHRPSGVPDRFSGSKSGTSASLAISGLQSEDEADYYCEAWDDSLNGPVFGGGTKLTVL | QVQLQESGPGLVNPSQTLSLTCAVSGGSISSGYYHWSWIRQHPGKGLEWIGYIYYSGSTYYNPSLKSRVTISVDTSKNQFSLKLTSVTAADTAVYYCARDWGGYDSSGWFDPWGQGTLVTVSS |
| | | | SEQ ID NO: 27324 | SEQ ID NO: 31330 |

FIGURE 50

| | | | | |
|---|---|---|---|---|
| iPS:436682 | 21-225_146A8 | NA | CAGTCTGTGCTGACTCAGCCACCCTCAGCGTCTGGGACCCCCGGGCAGAGGGTCACCATCTCTTGTTCTGGAAGCAGCTCCAACATCGGAAGTAATTCTATAAACTGGTACCAGCAACTCCCAAGAACGGCCCCCAAACTCCTCATCTATAGTAATGATCAGCGGCCCTCAGGGGTCCCTGACCGATTCTCTGGCTCCAAGTCTGGCACCTCAGCCTCCCTGGCCATCAGTGGGCTCCAGTCTGAGGATGAGGCTGATTATTACTGTGCAGCATGGGATGACAGCCTGAACGGCGTGGTATTCGGCGGAGGGACCAAGCTGACCGTCCTA | GAGGTGAAGCTGGTGGAGTCTGGGGGAGGCTTGGTACAGCCTGGGGAGTCCCTGAGACTCTCCTGTGTAGCCTCTGGATTCACCTTCAGTAACTATAACATGAACTGGGTCCGCCAGGCTCCAGGGAAGGGACTGGAGTGGGTTTCATACATTAGTAGAAGTAGTAATACCAAATACTACGCAGACTCTGTGAGGGGCCGATTCACCATCTCCAGAGACAATGCCAAGAATTCACTATATCTGCAAATGAACAGCCTGAGAGACGAGGACACGGCTGTGTATTACTGTGCGAGAGATAGGAGTGGGAGCTACGGGTACTTCTACTACTACGGTTTGGACGTCTGGGGCCAAGGGACCACGGTCACCGTCTCCTCA |
| | | | SEQ ID NO: 27325 | SEQ ID NO: 31331 |
| | | AA | QSVLTQPPSASGTPGQRVTISCSGSSSNIGSNSINWYQQLPRTAPKLLIYSNDQRPSGVPDRFSGSKSGTSASLAISGLQSEDEADYYCAAWDDSLNGVVFGGGTKLTVL | EVKLVESGGGLVQPGESLRLSCVASGFTFSNYNMNWVRQAPGKGLEWVSYISRSSNTKYYADSVRGRFTISRDNAKNSLYLQMNSLRDEDTAVYYCARDRSGSYGYFYYYGLDVWGQGTTVTVSS |
| | | | SEQ ID NO: 27326 | SEQ ID NO: 31332 |
| iPS:436684 | 21-225_146B6 | NA | CAGTCTGTGCTGACTCAGCCACCCTCAGCGTCTGGGACCCCCGGGCAGAGGGTCACCATCTCTTGTTCTGGAAGCAGCTCCAACATCGGAAGTAATGCTGTAAACTGGTACCAGCAGCTCCCAGGAACGGCCCCCAAACTCCTCATCTATAGTAATAATCAGCGGCCCTCAGGGGTCCCTGACCGGTTCTCTGGCTCCAAGTCTGGCACCTCAGCCTCCCTGGCCATCAGTGGGCTCCAGTCTGAGGATGAGGCTGATTATTACTGTGCAGCATGGGATGACAGCCTGAATGGCGTGGTATTCGGCGGAGGGACCAAGCTGACCGTCCTA | GAGGTGCAGCTGGTGGAGTCTGGGGGAGGCTTGGTACAGCCTGGGGGGTCCCTGAGACTCTCCTGTGCAGCCTCTGGATTCACCTTCAGTAGCTATAACATGAACTGGGTCCGCCAGGCTCCAGGGAAGGGGCTGGAGTGGGTTTCATACATTAGTAGAAGTAGTAATACCAAACACTACGCAGACTCTGTGAAGGGCCGATTCACCATCTCCAGAGACAATGCCAAGAACTCACTGTATCTGCAAATGGACAGCCTGAGAGACGAGGACACGGCTGTGTATTACTGTGCGAGAGATAGGAGTGGGAGCTACGGGTACTTCTACTACTACGGTTTGGACGTCTGGGGCCAAGGGACCACGGTCACCGTCTCCTCA |
| | | | SEQ ID NO: 27327 | SEQ ID NO: 31333 |

FIGURE 50

| | | | | |
|---|---|---|---|---|
| | | AA | QSVLTQPPSASGTPGQRVTISCSGSSSNIGSNAVNWYQQLPGTAPKLLIYSNNQRPSGVPDRFSGSKSGTSASLAISGLQSEDEADYYCAAWDDSLNGVVFGGGTKLTVL | EVQLVESGGGLVQPGGSLRLSCAASGFTFSSYNMNWVRQAPGKGLEWVSYISRSSNTKHYADSVKGRFTISRDNAKNSLYLQMDSLRDEDTAVYYCARDRSGSYGYFYYYGLDVWGQGTTVTVSS |
| | | | SEQ ID NO: 27328 | SEQ ID NO: 31334 |
| iPS:436686 | 21-225_148G6 | NA | TCCTATGAGCTGACTCAGCCACCCTCAGTGTCCGTGTCCCCAGGACAGACAGCCAGCATCACCTGCTCTGGAGATAAATTGGGGGATAAATATGCTTCCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGATCCAGGCTATGGATGAGGCTGACTATTACTGTCAGGCGTGGGACAGCAGCACTGTGGTATTCGGCGGAGGGACCAAGCTGACCGTCCTA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTATTAGTGGTGGTGGTAGTAGCACATACTACGCAGACTCCGTGAAGGGCCGGTTCACCATCTCCAGAGACAATTCCAAGAACATGCTGCATCTGCAAATGAACAGCCTGAGAGCCGAGGACACGGCCGTATATTACTGTGCGAAATGGCGAGGTAACCCCACTGACTACGGTATGGACGTCTGGGGCCAAGGGACCACGGTCACCGTCTCCTCA |
| | | | SEQ ID NO: 27329 | SEQ ID NO: 31335 |
| | | AA | SYELTQPPSVSVSPGQTASITCSGDKLGDKYASWYQQKPGQSPVLVIYQDSKRPSGIPERFSGSNSGNTATLTISGIQAMDEADYYCQAWDSSTVVFGGGTKLTVL | EVQLLESGGGLVQPGGSLRLSCAASGFTFSSYAMSWVRQAPGKGLEWVSVISGGGSSTYYADSVKGRFTISRDNSKNMLHLQMNSLRAEDTAVYYCAKWRGNPTDYGMDVWGQGTTVTVSS |
| | | | SEQ ID NO: 27330 | SEQ ID NO: 31336 |

FIGURE 50

| | | | | |
|---|---|---|---|---|
| **iPS:436688** | **21-225_148C8** | NA | TCCTATGAGCTGACTCAGCCGCCCTCAGTGTCCGTGTCCCCAGGACAGACAGCCAGCATCACCTGCTCTGGAGATAAATTGGGGGATAAATATGTTTCCTGGTATCAGCAGAAGCCAGGCCAGTCCCCTATACTGGTCATCTATCAAGATAGGAAGCGGCCCTCAGGGACCCCTGAGCGATTCTCTGGCTCCAACTCTGGGAACACAGCCACTCTGACCATCAGCGGGACCCAGGCTATGGATGAGGCTGATTATTACTGTCTGGCGTGGGACAGCAGCACTTTTGTGGTATTCGGCGGAGGGACCAAGTTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGTGACTACGGTGACCCCCCCTACTACTACTACTACGGTATGGACGTCTGGGGCCAAGGGACCACGGTCACCGTCTCCTCA |
| | | | SEQ ID NO: 27331 | SEQ ID NO: 31337 |
| | | AA | SYELTQPPSVSVSPGQTASITCSGDKLGDKYVSWYQQKPGQSPILVIYQDRKRPSGTPERFSGSNSGNTATLTISGTQAMDEADYYCLAWDSSTFVVFGGGTKLTVL | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARDRDYGDPPYYYYYGMDVWGQGTTVTVSS |
| | | | SEQ ID NO: 27332 | SEQ ID NO: 31338 |
| **iPS:436690** | **21-225_148A9** | NA | TCCTATGAGCTGACTCAGCCACCCTCAGTGTCCGTGTCCCCAGGACAGACAGCCAGCATCACCTGTTCTGGAGATAAATTGGGGAATAAATATGTTTGCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGCACAGCAGCACTGTGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTACCTATGGCATGCACTGGGTCCGCCAGGCTCCAGGCAAGGGGCTGGAGTGGGTGGCAGTTATATGGTATGGTGGAAGTGATAAAGACTATGCAGACTCCGTGAAGGGCCGATTCACCATCTCCAGAGACATTTCCAAGAACACGCTGTATCTGCAAATGAACAGCCTGAGAGCCGAGGACACGGCTGTGTATTACTGTGCGAGAGATCGGGATTATTGTAGTGGTGGTACTTGTCCTTACTACTACTACTACGGTATGGACGTCTGGGGCCAAGGGACCACGGTCACCGTCTCCTCA |
| | | | SEQ ID NO: 27333 | SEQ ID NO: 31339 |

FIGURE 50

| | | | | |
|---|---|---|---|---|
| | | AA | SYELTQPPSVSVSPGQTASITCSGDKLGNKYVCWYQQKPGQSPVLVIYQDSKRPSGIPERFSGSNSGNTATLTISGTQAMDEADYYCQAWHSSTVVFGGGTKLTVL | QVQLVESGGGVVQPGRSLRLSCAASGFTFSTYGMHWVRQAPGKGLEWVAVIWYGGSDKDYADSVKGRFTISRDISKNTLYLQMNSLRAEDTAVYYCARDRDYCSGGTCPYYYYYGMDVWGQGTTVTVSS |
| | | | SEQ ID NO: 27334 | SEQ ID NO: 31340 |
| iPS:436694 | 21-225_148G11 | NA | TCCTATGAGCTGACTCAGCCACCCTCAGTGTCCGTGTCCCCAGGACAGACAGCCAGCATCACCTGCTCTGGAGATAAATTGGGGGATAAATTTGCTTCCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGATCCAGGCTATGGATGAGGCTGACTATTACTGTCAGGCGTGGGACAGCAGCACTGTGGTATTCGGCGGAGGGACCAAGCTGACCGTCCTA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATCCCATGAGCTGGGTCCGCCAGGCTCCCGGGAAGGGGCTGGAGTGGGTCTCAGTTATTAGTGGTGGTGGTAGTAGTGCATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATGGCGAGGTAACCCCACTGACTACGGTATGGACGTCTGGGGCCAAGGGACCACGGTCACCGTCTCCTCA |
| | | | SEQ ID NO: 27335 | SEQ ID NO: 31341 |
| | | AA | SYELTQPPSVSVSPGQTASITCSGDKLGDKFASWYQQKPGQSPVLVIYQDSKRPSGIPERFSGSNSGNTATLTISGIQAMDEADYYCQAWDSSTVVFGGGTKLTVL | EVQLLESGGGLVQPGGSLRLSCAASGFTFSSYPMSWVRQAPGKGLEWVSVISGGGSSAYYADSVKGRFTISRDNSKNTLYLQMNSLRAEDTAVYYCAKWRGNPTDYGMDVWGQGTTVTVSS |
| | | | SEQ ID NO: 27336 | SEQ ID NO: 31342 |

FIGURE 50

| | | | | |
|---|---|---|---|---|
| **iPS:436696** | **21-225_149A1** | NA | CAGTCTGTGCTGACTCAGCCACCCTCAGCGTCTGGGACCCCCGGGCAGAGGGTCACCATCTCTTGTTCTGGAAGCAGCTCCAACATCGGAAGTAATGCTGTAAACTGGTACCAGCAGCTCCCAGGAACGGCCCCCAAACTCCTCATCTATAGTAATAATCAGCGGCCCTCAGGGGTCCCTGACCGGTTCTCTGGCTCCAAGTCTGGCACCTCAGCCTCCCTGGCCATCAGTGGGCTCCAGTCTGAGGATGAGGCTGATTATTACTGTGCAGCATGGGATGACAGCCTGAATGGCGTGGTATTCGGCGGAGGGACCAAGCTGACCGTCCTA | GAGGTGCAGCTGGTGGAGTCTGGGGGAGGCTTGGTACAGCCTGGGGGGTCCCTGAGACTCTCCTGTGCAGCCTCTGGATTCACCTTCAGTAGCTATAACATGAACTGGGTCCGCCAGGCTCCAGGGAAGGGGCTGGAGTGGGTTTCATACATTAGTAGAAGTAGTAATACCAAACACTACGCAGACTCTGTGAAGGGCCGATTCACCATCTCCAGAGACAATGCCAAGAACTCACTGTATCTGCAAATGAACAGCCTGAGAGACGAGGACACGGCTGTGTATTACTGTGCGAGAGATAGGAGTGGGAGCTACGGGTACTTCTACTACTACGGTTTGGACGTCTGGGGCCAAGGGACCACGGTCACCGTCTCCTCA |
| | | | SEQ ID NO: 27337 | SEQ ID NO: 31343 |
| | | AA | QSVLTQPPSASGTPGQRVTISCSGSSSNIGSNAVNWYQQLPGTAPKLLIYSNNQRPSGVPDRFSGSKSGTSASLAISGLQSEDEADYYCAAWDDSLNGVVFGGGTKLTVL | EVQLVESGGGLVQPGGSLRLSCAASGFTFSSYNMNWVRQAPGKGLEWVSYISRSSNTKHYADSVKGRFTISRDNAKNSLYLQMNSLRDEDTAVYYCARDRSGSYGYFYYYGLDVWGQGTTVTVSS |
| | | | SEQ ID NO: 27338 | SEQ ID NO: 31344 |
| **iPS:436698** | **21-225_149B5** | NA | TCCTATGAACTGACTCAGCCACCCTCAGTGTCCGTGTCCCCAGGACAGACAGCCAGCATCACCTGCTCTGGATATAAATTGGGGTATAAATATGTTTGCTGGTATCAGCAGAAGCCAGGCCAGTCCCCTGTACTGGTCATCTTTCAAAATAACCAGCGGCCCTCAGGGATCCCTGAGCGATTCTCTGGCTCCAACTCTGGGAACACAGCCTCTCTGACCATCAGCGGGACCCAGGCTATGGATGAGGCTGACTATTACTGTCAGGCGTGGGACAGCAGCACTGTGGTATTCGGCGGAGGGACCAAGCTGACCGTCCTA | GAGGTGCAGCTGGTGGAGTCTGGGGGAGGCTTGGTCCAGCCTGGGGGGTCCCTGAGACTCTCCTGTGCAGCCTCTGGATTCACCTTTAGTGGCTATTGGATGAACTGGGTCCGCCAGGCTCCAGGGAAGGGGCTGGAGTGGGTGGCCAACATAAAGCAAGATGGAAGTGAGAAATACTATGTGGACTCTGTGAAGGGCCGATTCACCATCTCCAGAGACAACGCCAAGAATTCACTGTATCTGCAAATGAACAGCCTGAGAGCCGAGGACACGGCTGTGTATTACTGTGCGAGAGGGATGTATAGCAGTGGCTGGTACGTCTTTGACTACTGGGGCCAGGGAACCCTGGTCACCGTCTCCTCA |
| | | | SEQ ID NO: 27339 | SEQ ID NO: 31345 |

FIGURE 50

| | | | | |
|---|---|---|---|---|
| | | AA | SYELTQPPSVSVSPGQTASITCSGYKLGYKYVCWYQQKPGQSPVLVIFQNNQRPSGIPERFSGSNSGNTASLTISGTQAMDEADYYCQAWDSSTVVFGGGTKLTVL | EVQLVESGGGLVQPGGSLRLSCAASGFTFSGYWMNWVRQAPGKGLEWVANIKQDGSEKYYVDSVKGRFTISRDNAKNSLYLQMNSLRAEDTAVYYCARGMYSSGWYVFDYWGQGTLVTVSS |
| | | | SEQ ID NO: 27340 | SEQ ID NO: 31346 |
| iPS:436700 | 21-225_149C7 | NA | TCCTATGAGCTGACTCAGCCACCCTCAGTGTCCGTGTCCCCAGGACAGACAGCCAGCATCACCTGCTCTGGAAATAAATTGGGGGATAAATATGCTTCCTGGTATCAGCAGAAGCCAGGCCAGTCCCCTGTGCTGGTCATCTATCAAGATAGCAAGCGGCCCTCAGGGATCCCTGAGCGATTCTCTGGCTCCAAGTCTGGGAACACAGCCACTCTGACCATCAGCGGGATCCAGGCTATGGATGAGGCTGACTATTACTGTCAGGCGTGGGACAGCAGCACTGTGGTATTCGGCGGAGGGACCAAGCTGACCGTCCCA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTATTAGTGGTGGTGGTAGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATGGCGAGGTAACCCCACTGACTACGGTATGGACGTCTGGGGCCAAGGGACCACGGTCACCGTCTCCTCA |
| | | | SEQ ID NO: 27341 | SEQ ID NO: 31347 |
| | | AA | SYELTQPPSVSVSPGQTASITCSGNKLGDKYASWYQQKPGQSPVLVIYQDSKRPSGIPERFSGSKSGNTATLTISGIQAMDEADYYCQAWDSSTVVFGGGTKLTVP | EVQLLESGGGLVQPGGSLRLSCAASGFTFSSYAMSWVRQAPGKGLEWVSVISGGGSSTYYADSVKGRFTISRDNSKNTLYLQMNSLRAEDTAVYYCAKWRGNPTDYGMDVWGQGTTVTVSS |
| | | | SEQ ID NO: 27342 | SEQ ID NO: 31348 |

FIGURE 50

| | | | | |
|---|---|---|---|---|
| **iPS:436702** | **21-225_149E8** | NA | CAGGCTGTGTCGACTCAGCCGTCTTCCCTCTCTGCATCTCCTGGAGCATCAGCCAGTCTCACCTGCACCTTACGCAGTGGCATCACTGTTACTACCTATAGGATATACTGGTACCAGCAGAAGCCAGGGAGTCCTCCCCAGTTTCTCCTGCGGTACACATCAGACTCAGATAAACACCAGGGCTCTGGAGTCCCCAGCCGCTTCTCTGGATCCAAAGATGCTTCGGCCAATGCAGGGATTTTATTCATCTCTGGGCTCCAGTCTGAGGATGAGGCTGACTATTACTGTATGATTTGGCACAGCAGCGCTTGGGTGTTCGGCGGAGGGACCAAGCTGACCGTCCTA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGTTATAGCATGAACTGGGTCCGCCGGGCTCCAGGGAAGGGGCTGGAGTGGGTCTCAGCCATTAGTAGTACTGGTAGTTACATATATTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGGACGGCAGTGGCTGGTACTGGGTGGTTCGACCCCTGGGGCCAGGGAACCCTGGTCACCGTCTCCTCA |
| | | | SEQ ID NO: 27343 | SEQ ID NO: 31349 |
| | | AA | QAVSTQPSSLSASPGASASLTCTLRSGITVTTYRIYWYQQKPGSPPQFLLRYTSDSDKHQGSGVPSRFSGSKDASANAGILFISGLQSEDEADYYCMIWHSSAWVFGGGTKLTVL | EVQLVESGGGLVKPGGSLRLSCAASGFTFSSYSMNWVRRAPGKGLEWVSAISSTGSYIYYADSVKGRFTISRDNAKNSLYLQMNSLRAEDTAVYYCARTAVAGTGWFDPWGQGTLVTVSS |
| | | | SEQ ID NO: 27344 | SEQ ID NO: 31350 |
| **iPS:436704** | **21-225_149C10** | NA | TCCTATGAGCTGACTCAGCCACCCTCAGTGTCCGTGTCCCCAGGACAGACAGCCAGCATCACCTGCTCTGGAGATAAATTGGGGGATAAATATGCTTCCTGGTATCAGCAGAAGCCAGGCCAGTCCCCTGTGCTGGTCATCTATCAAGATAACAAGCGGCCCTCAGGGATCCCTGAGCGATTCTCTGGCTCCAACTCTGGGAACACAGCCACTCTGACCATCGGCGGGATCCAGGCTATGGATGAGGCTGACTATTACTGTCAGGCGTGGGACAGCAGCACTGTGGTATTCGGCGGAGGGACCAAGCTGACCGTCCTA | GAGGTGCAACTGTTGGAGTCTGGGGGAGGCTTGGTACAGCCTGGGGGGTCCCTGAGATTCTCCTGTGCAGCCTCTGGATTCACCTTTAGCAGCCACGCCATGAGCTGGGTCCGCCAGGCTCCAGGGAAGGGGCTGGAGTGGGTCTCAGTTATAAGTGGAGGTGGTAGTAGCACATATTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATGGCGAGGTAACCCCACTGACTACGGTATGGACGTCTGGGGCCAAGGGACCACGGTCACCGTCTCCTCA |
| | | | SEQ ID NO: 27345 | SEQ ID NO: 31351 |

FIGURE 50

| | | | | |
|---|---|---|---|---|
| | | AA | SYELTQPPSVSVSPGQTASITCSGDKLGDKYASWYQQKPGQSPVLVIYQDNKRPSGIPERFSGSNSGNTATLTIGGIQAMDEADYYCQAWDSSTVVFGGGTKLTVL | EVQLLESGGGLVQPGGSLRFSCAASGFTFSSHAMSWVRQAPGKGLEWVSVISGGGSSTYYADSVKGRFTISRDNSKNTLYLQMNSLRAEDTAVYYCAKWRGNPTDYGMDVWGQGTTVTVSS |
| | | | SEQ ID NO: 27346 | SEQ ID NO: 31352 |
| iPS:436706 | 21-225_149A11 | NA | TCCTATGAACTGACTCAGCCGCCCTCAGTGTCCGTGTCCCCAGGACAGACAGCCAGCATCACCTGCTCTGGAGATAAATTGGGGAATAAATATGTTTCCTGGTATCAGCAGAAGCCAGGCCAGTCCCCTGTGCTGGTCATCTATCAAGATAGCAGGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCTGGCGTGGGACAGCAGCACTTTTGTGGTCTTCGGCGGAGGGACCAAGTTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGTGACTACGGTGACCCCCCCTACTACTACTACTACGGTATGGACGTCTGGGGCCAAGGGACCACGGTCACCGTCTCCTCA |
| | | | SEQ ID NO: 27347 | SEQ ID NO: 31353 |
| | | AA | SYELTQPPSVSVSPGQTASITCSGDKLGNKYVSWYQQKPGQSPVLVIYQDSRRPSGIPERFSGSNSGNTATLTISGTQAMDEADYYCLAWDSSTFVVFGGGTKLTVL | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARDRDYGDPPYYYYYGMDVWGQGTTVTVSS |
| | | | SEQ ID NO: 27348 | SEQ ID NO: 31354 |

FIGURE 50

| | | | | |
|---|---|---|---|---|
| **iPS:436708** | **21-225_150D3** | NA | TCCTATGAGCTGACTCAGCCACCCTCAGTGTC<br>CGTGTCCCCAGGACAGACAGCCAGCATCACCT<br>GCTCTGGAGATGAATTGGGGAATAAATATGCT<br>TGCTGGTATCAGCAGAAGCCAGGCCAGTCCCC<br>TGTGCTGGTCATCTATCAAGATAACAAGCGGC<br>CCTCAGGGATCCCTGAGCGATTCTCTGGCTCC<br>AGCTCTGGGAACACAGCCACTCTGACCATCAG<br>CGGGACCCAGGCTATGGATGAGGCTGACTATT<br>ACTGTCAGGCGTGGCACAGCAGCACTGTGGTA<br>TTCGGCGGAGGGACCAAGCTGACCGTCCTA | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTACCTATGGCAT<br>GCACTGGGTCCGCCAGGCTCCAGGCAAGGGGCT<br>GGAGTGGGTGGCAGTTATATGGTATGGTGGAAGT<br>AATAAAGACTATGCAGACTCCGTGAAGGGCCGA<br>TTCACCATCTCCAGAGACAATTCCAAGAACACGC<br>TGTATCTGCAAATGAACAGTTTGAGAGCCGAGGA<br>CACGGCTGTGTATTACTGTGCGAGAGATCGGGAT<br>TATTGTAGTGGTGGTACCTGCCCTTACTACTACTA<br>CTACGGTATGGACGTCTGGGGCCAAGGGACCAC<br>GGTCACCGTCTCCTCA |
| | | | SEQ ID NO: 27349 | SEQ ID NO: 31355 |
| | | AA | SYELTQPPSVSVSPGQTASITCSGDELGNKYACW<br>YQQKPGQSPVLVIYQDNKRPSGIPERFSGSSSGN<br>TATLTISGTQAMDEADYYCQAWHSSTVVFGGG<br>TKLTVL | QVQLVESGGGVVQPGRSLRLSCAASGFTFSTYGMH<br>WVRQAPGKGLEWVAVIWYGGSNKDYADSVKGRF<br>TISRDNSKNTLYLQMNSLRAEDTAVYYCARDRDY<br>CSGGTCPYYYYYGMDVWGQGTTVTVSS |
| | | | SEQ ID NO: 27350 | SEQ ID NO: 31356 |

FIGURE 50

| | | | | |
|---|---|---|---|---|
| iPS:436710 | 21-225_150F6 | NA | TCCTATGAGCTGACTCAGCCACCCTCAGTGTCCGTGTCCCCAGGACAGACAGCCAGCATCACCTGCTCTGGAGATAAATTGGGGGATAAATATGCTTCCTGGTATCAGCAGAAGCCAGGCCAGTCCCCTGTGCTGGTCATCTGTCAAGATAGCAAGCGGCCCTCAGGGATCCCTGAGCGATTCTCTGGCTCCAACTCTGGGAACACAGCCACTCTGACCATCAGCGGGATCCAGGCTATGGATGAGGCTGACTATTGCTGTCAGGCGTGGGACAGCAGCACTGTGGTATTCGGCGGAGGGACCAAGCTGACCGTCCTA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTATTAGTGGTGGTGGTAGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATGGCGAGGTAACCCCACTGACTACGGTATGGACGTCTGGGGCCAAGGGACCACGGTCACCGTCTCCTCA |
| | | | SEQ ID NO: 27351 | SEQ ID NO: 31357 |
| | | AA | SYELTQPPSVSVSPGQTASITCSGDKLGDKYASWYQQKPGQSPVLVICQDSKRPSGIPERFSGSNSGNTATLTISGIQAMDEADYCCQAWDSSTVVFGGGTKLTVL | EVQLLESGGGLVQPGGSLRLSCAASGFTFSSYAMSWVRQAPGKGLEWVSVISGGGSSTYYADSVKGRFTISRDNSKNTLYLQMNSLRAEDTAVYYCAKWRGNPTDYGMDVWGQGTTVTVSS |
| | | | SEQ ID NO: 27352 | SEQ ID NO: 31358 |
| iPS:436712 | 21-225_150F9 | NA | CAGTCTGTGCTGACTCAGCCACCCTCAGCGTCTGGGACCCCCGGGCAGAGGGTCACCATCTCTTGTTCTGGAAGCAGCTCCAACATCGGAAGTAATGCTGTAAACTGGTACCAGCAGCTCCCAGGAACGGCCCCCAAACTCCTCATCTATAGTAATAGTCAGCGGCCCTCAGGGGTCCCTGACCGGTTCTCTGGCTCCAAGTCTGGCACCTCAGCCTCCCTGGCCATCAGTGGGCTCCAGTCTGAGGATGAGGCTGATTATTTCTGTGCAGCATGGGATGACAGCCTGAATGGCGTGGTATTCGGCGGAGGGACCAAGCTGACCGTCCTA | GAGATGCAGCTGGTGGAGTCTGGGGGAGGCTTGGTACAGCCTGGGGGGTCCCTGAGACTCTCCTGTGCAGCCTCTGGATTCACCTTCAGTAGCTATAACATGAACTGGGTCCGCCAGGTTCCAGGGAAGGGGCTGGAGTGGGTTTCATACATTAGTAGAAGTAGTAATACCAAACACTACGCAGACTCTGTGAAGGGCCGATTCACCATCTCCAGAGACAATGCCAAGAACTCACTGTATCTGCAAATGAACAGCCTGAGAGACGAGGACACGGCTGTGTATTACTGTGCGAGAGATAGGAGTGGGAGCTACGGGTACTTCTACTACTACGGTTTGGACGTCTGGGGCCAAGGGACCACGGTCACCGTCTCCTCA |
| | | | SEQ ID NO: 27353 | SEQ ID NO: 31359 |

FIGURE 50

| | | | | |
|---|---|---|---|---|
| | | AA | QSVLTQPPSASGTPGQRVTISCSGSSSNIGSNAVNWYQQLPGTAPKLLIYSNSQRPSGVPDRFSGSKSGTSASLAISGLQSEDEADYFCAAWDDSLNGVVFGGGTKLTVL | EMQLVESGGGLVQPGGSLRLSCAASGFTFSSYNMNWVRQVPGKGLEWVSYISRSSNTKHYADSVKGRFTISRDNAKNSLYLQMNSLRDEDTAVYYCARDRSGSYGYFYYYGLDVWGQGTTVTVSS |
| | | | SEQ ID NO: 27354 | SEQ ID NO: 31360 |
| iPS:436714 | 21-225_150H11 | NA | TCCTATGAGCTGACTCAGCCACCCTCAGTGTCCGTGTCCCCAGGACAGACAGCCAGCATCACCTGCTCTGGAGATAAATTGGGGGATAAATATGCTTCCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGATCCAGGCTATGGATGAGGCTGACTTTTACTGTCAGGCGTGGGACAGCAGCACTGTGGTATTCGGCGGAGGGACCAAGCTGACCGTCCTA | GAGGTGCAACTGTTGGAGTCTGGGGGAGGCTTGGTACAGCCTGGGGGGTCCCTGAGACTCTCCTGTGCAGCCTCTGGATTCACCTTTAGCACCTATGCCATGAGCTGGGTCCGCCAGGCTCCAGGGAAGGGGCTGGAGTGGGTCTCAATTATTAGTGGTGGTGGTAGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATGGCGAGGTAACCCCACTGACTACGGTATGGACGTCTGGGGCCAAGGGACCACGGTCACCGTCTCCTCA |
| | | | SEQ ID NO: 27355 | SEQ ID NO: 31361 |
| | | AA | SYELTQPPSVSVSPGQTASITCSGDKLGDKYASWYQQKPGQSPVLVIYQDSKRPSGIPERFSGSNSGNTATLTISGIQAMDEADFYCQAWDSSTVVFGGGTKLTVL | EVQLLESGGGLVQPGGSLRLSCAASGFTFSTYAMSWVRQAPGKGLEWVSIISGGGSSTYYADSVKGRFTISRDNSKNTLYLQMNSLRAEDTAVYYCAKWRGNPTDYGMDVWGQGTTVTVSS |
| | | | SEQ ID NO: 27356 | SEQ ID NO: 31362 |

FIGURE 50

| | | | | |
|---|---|---|---|---|
| **iPS:436716** | **21-225_151F3** | NA | TCTTATGAGCTGACTCAGCCACCCTCAGTGTCCGTGTCCCCAGGACAGACAGCCAGCATCACCTGCTCTGGAGATAAATTGGGGGATAAATATGTTTGCTGGTATCAGCAGAAGCCAGGCCAGTCCCCTGTGCTGGTCATCTATCAAGATCGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGCACAGCAGCACTGTGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTACCTATGGCATGCACTGGGTCCGCCAGGCTCCAGGCAAGGGGCTGGAGTGGGTGGCAGTTATATGGTATGGTGGAAGTAATACAGACTATGCAGACTCCGTGAAGGGCCGATTCACCATCTCCAGAGACATTTCCAAGAACACGCTGTATCTGCAAATGAACAGCCTGAGAGCCGAGGACACGGCTGTGTATTACTGTGCGAGAGATCGGGATTATTGTAGTGGTACTAGCTGCCCTTACTACTACTACTACGGTATGGACGTCTGGGGCCAAGGGACCACGGTCACCGTCTCCTCA |
| | | | SEQ ID NO: 27357 | SEQ ID NO: 31363 |
| | | AA | SYELTQPPSVSVSPGQTASITCSGDKLGDKYVCWYQQKPGQSPVLVIYQDRKRPSGIPERFSGSNSGNTATLTISGTQAMDEADYYCQAWHSSTVVFGGGTKLTVL | QVQLVESGGGVVQPGRSLRLSCAASGFTFSTYGMHWVRQAPGKGLEWVAVIWYGGSNTDYADSVKGRFTISRDISKNTLYLQMNSLRAEDTAVYYCARDRDYCSGTSCPYYYYYGMDVWGQGTTVTVSS |
| | | | SEQ ID NO: 27358 | SEQ ID NO: 31364 |

FIGURE 50

| | | | | |
|---|---|---|---|---|
| iPS:436718 | 21-225_151H5 | NA | TCCTATGAGCTGACTCAGCCACCCTCAGTGTCCGTGTCCCCAGGACAGACAGCCAGCATCGCCTGCTCTGGAGATAAATTGGGGGATAAATATGCTTCCTGGTATCAACAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGATCCAGGCTATGGATGAGGCTGACTATTACTGTCAGGCGTGGGACAGCAGCACTGTGGTATTCGGCGGAGGGACCAAGCTGACCGTCCTA | GAGGTGCAACTGTTGGAGTCTGGGGGAGGCTTGGTACAGCCTGGGGGGTCCCTGAGACTCTCCTGTGCAGCCTCTGGATTCACCTTCAGCAGCTATGCCATGAGCTGGGTCCGCCAGGCTCCAGGGAAGGGGCTGGAGTGGGTCTCAGTTATTAGTGGTGGTGGTAGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATGGCGAGGTAACCCCACTGACTACGGTATGGACGTCTGGGGCCAAGGGACCACGGTCACCGTCTCCTCA |
| | | | SEQ ID NO: 27359 | SEQ ID NO: 31365 |
| | | AA | SYELTQPPSVSVSPGQTASIACSGDKLGDKYASWYQQKPGQSPVLVIYQDSKRPSGIPERFSGSNSGNTATLTISGIQAMDEADYYCQAWDSSTVVFGGGTKLTVL | EVQLLESGGGLVQPGGSLRLSCAASGFTFSSYAMSWVRQAPGKGLEWVSVISGGGSSTYYADSVKGRFTISRDNSKNTLYLQMNSLRAEDTAVYYCAKWRGNPTDYGMDVWGQGTTVTVSS |
| | | | SEQ ID NO: 27360 | SEQ ID NO: 31366 |
| iPS:436720 | 21-225_151H6 | NA | TCCTATGAGCTGACTCAGCCACCCTCAGTGTCCGTGTCCCCAGGACAGACAGCCAGCATCACCTGCTCTGGAGATAAATTGGGGGATAAATATGCTTGCTGGTATCAGCAGAAGCCAGGCCAGTCCCCTGTGCTGGTCATCTATCAAGATACCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTTATGTCTTCGGAACTGGGACCAAGGTCACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCACTTATATGGTATGATGGAAGTAATAAATACTATGCAGACTCCGTGAAGGGCCGATTCACCATCTCCAGAGACAATTCCAAGAACACGCTGTATCTACAAATGAACAGCCTGAGAGCCGAGGACACGGCTGTGTATTACTGTGCGAGAGATGACCGATCTTGTAGTAGAACCAGCTGCCCTTACTACTACTACTACGGTTTGGACGTCTGGGGCCAAGGGACCACGGTCATCGTCTCCTCA |
| | | | SEQ ID NO: 27361 | SEQ ID NO: 31367 |

FIGURE 50

| | | | | |
|---|---|---|---|---|
| | | AA | SYELTQPPSVSVSPGQTASITCSGDKLGDKYACWYQQKPGQSPVLVIYQDTKRPSGIPERFSGSNSGNTATLTISGTQAMDEADYYCQAWDSSTYVFGTGTKVTVL | QVQLVESGGGVVQPGRSLRLSCAASGFTFSDYGMHWVRQAPGKGLEWVALIWYDGSNKYYADSVKGRFTISRDNSKNTLYLQMNSLRAEDTAVYYCARDDRSCSRTSCPYYYYYGLDVWGQGTTVIVSS |
| | | | SEQ ID NO: 27362 | SEQ ID NO: 31368 |
| iPS:436722 | 21-225_151H7 | NA | TCCTATGACCTGACTCAGCCACCCTCAGTGTCCGTGTCCCCAGGACAGACAGCCAGCATCACCTGCTCTGGAGATAAATTGGGGGATAAATATGCTTCCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGATCCAGGCTATGGATGAGGCTGACTATTACTGTCAGGCGTGGGACAGCAGCACTGTGGTATTCGGCGGAGGGACCAAGCTGACCGTCCTA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTATTAGTGGTGGTGGTAGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATGGCGAGGTAACCCCACTGACTACGGTATGGACGTCTGGGGCCAAGGGACCACGGTCACCGTCTCCTCA |
| | | | SEQ ID NO: 27363 | SEQ ID NO: 31369 |
| | | AA | SYDLTQPPSVSVSPGQTASITCSGDKLGDKYASWYQQKPGQSPVLVIYQDSKRPSGIPERFSGSNSGNTATLTISGIQAMDEADYYCQAWDSSTVVFGGGTKLTVL | EVQLLESGGGLVQPGGSLRLSCAASGFTFSSYAMSWVRQAPGKGLEWVSVISGGGSSTYYADSVKGRFTISRDNSKNTLYLQMNSLRAEDTAVYYCAKWRGNPTDYGMDVWGQGTTVTVSS |
| | | | SEQ ID NO: 27364 | SEQ ID NO: 31370 |

FIGURE 50

| | | | | |
|---|---|---|---|---|
| **iPS:436724** | **21-225_151B9** | NA | TCCTATGAGCTGACTCAGCCACCCTCAGTGTCCGTGTCCCCAGGACAGACAGCCAGCATCGCCTGCTCTGGAGATAATTTGGGGGATAAATATGCTTCCTGGTATCAACAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGATCCAGGCTATGGATGAGGCTGACTATTACTGTCAGGCGTGGGACAGCAGCACTGTGGTATTCGGCGGAGGGACCAAGCTGACCGTCCTA | GAGGTGCAACTGTTGGAGTCTGGGGGAGGCTTGGTACAGCCTGGGGGGTCCCTGAGACTCTCCTGTGCAGCCTCTGGATTCACCTTCAGCAGCTATGCCATGAGCTGGGTCCGCCAGGCTCCAGGGAAGGGGCTGGAGTGGGTCTCAGTTATTAGTGGTGGTGGTAGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATGGCGAGGTAACCCCACTGACTACGGTATGGACGTCTGGGGCCAAGGGACCACGGTCACCGTCTCCTCA |
| | | | SEQ ID NO: 27365 | SEQ ID NO: 31371 |
| | | AA | SYELTQPPSVSVSPGQTASIACSGDNLGDKYASWYQQKPGQSPVLVIYQDSKRPSGIPERFSGSNSGNTATLTISGIQAMDEADYYCQAWDSSTVVFGGGTKLTVL | EVQLLESGGGLVQPGGSLRLSCAASGFTFSSYAMSWVRQAPGKGLEWVSVISGGGSSTYYADSVKGRFTISRDNSKNTLYLQMNSLRAEDTAVYYCAKWRGNPTDYGMDVWGQGTTVTVSS |
| | | | SEQ ID NO: 27366 | SEQ ID NO: 31372 |
| **iPS:436726** | **21-225_152G5** | NA | TCCTATGAGATGACTCAGCCACCCTCAGTGTCCGTGTCCCCAGGACAGACAGCCATCATCACCTGCTCTGGAGATAAATTGGGGGATAAATATGCTTGCTGGTATCAACAGAAGCCAGGCCAGTCCCCTGTGCTGGTCATCTATCAAGATTCCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTTATGTCTTCGGAACTGGGACCAAGGTCACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCACTTATATGGTATGATGGAAGTAATAAATACTATGCAGACTCCGTGAAGGGCCGATTCACCATCTCCAGAGACAATTCCAAGAACACGCTGTATCTACAAATGAACAGCCTGAGAGCCGAGGACACGGCTGTGTATTACTGTGCGAGAGATGACCGATCTTGTAGTAGAACCAGCTGCCCTTACTACTACTACTACGGTTTGGACGTCTGGGGCCAAGGGACCACGGTCATCGTCTCCTCA |
| | | | SEQ ID NO: 27367 | SEQ ID NO: 31373 |

FIGURE 50

| | | | | |
|---|---|---|---|---|
| | | AA | SYEMTQPPSVSVSPGQTAIITCSGDKLGDKYACWYQQKPGQSPVLVIYQDSKRPSGIPERFSGSNSGNTATLTISGTQAMDEADYYCQAWDSSTYVFGTGTKVTVL | QVQLVESGGGVVQPGRSLRLSCAASGFTFSDYGMHWVRQAPGKGLEWVALIWYDGSNKYYADSVKGRFTISRDNSKNTLYLQMNSLRAEDTAVYYCARDDRSCSRTSCPYYYYYGLDVWGQGTTVIVSS |
| | | | SEQ ID NO: 27368 | SEQ ID NO: 31374 |
| iPS:436728 | 21-225_152G6 | NA | TCCTATGAGCTGACTCAGCCACCCTCAGTGTCCGTGTCCCCAGGACAGACAGCCAGCATCACCTGCTCTGGAGATAAATTGGGGGATAAATATGCTTCCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGATCCAGGCTCTGGATGAGGCTGACTATTACTGTCAGGCGTGGGACAACAGCACTGTGGTATTCGGCGGAGGGACCAAGCTGACCGTCCTA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTATTAGTGGTGGTGGTAGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATGGCGAGGTAACCCCACTGACTACGGTATGGACGTCTGGGGCCAAGGGACCACGGTCACCGTCTCCTCA |
| | | | SEQ ID NO: 27369 | SEQ ID NO: 31375 |
| | | AA | SYELTQPPSVSVSPGQTASITCSGDKLGDKYASWYQQKPGQSPVLVIYQDSKRPSGIPERFSGSNSGNTATLTISGIQALDEADYYCQAWDNSTVVFGGGTKLTVL | EVQLLESGGGLVQPGGSLRLSCAASGFTFSSYAMSWVRQAPGKGLEWVSVISGGGSSTYYADSVKGRFTISRDNSKNTLYLQMNSLRAEDTAVYYCAKWRGNPTDYGMDVWGQGTTVTVSS |
| | | | SEQ ID NO: 27370 | SEQ ID NO: 31376 |

FIGURE 50

| | | | | |
|---|---|---|---|---|
| **iPS:436730** | **21-225_152D7** | NA | TCCTATGAGCTGACTCAGCCACCCTCAGTGTCCGTGTCCCCAGGACAGACAGCCAGCATCACCTGCTCTGGAGATAAATTGGGGGATAAATATGCTTCCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGATCCAGGCTATGGATGAGGCTGACTATTACTGTCAGGCGTGGGACAGCAGCACTGTGGTATTCGGCGGAGGGACCAAGCTGACCGTCCTA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTATTAGTGGTGGTGGTAGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATGGCGAGGTAACCCCACTGACTCCGGTATGGACGTCTGGGGCCAAGGGACCACGGTCACCGTCTCCTCA |
| | | | SEQ ID NO: 27371 | SEQ ID NO: 31377 |
| | | AA | SYELTQPPSVSVSPGQTASITCSGDKLGDKYASWYQQKPGQSPVLVIYQDSKRPSGIPERFSGSNSGNTATLTISGIQAMDEADYYCQAWDSSTVVFGGGTKLTVL | EVQLLESGGGLVQPGGSLRLSCAASGFTFSSYAMSWVRQAPGKGLEWVSVISGGGSSTYYADSVKGRFTISRDNSKNTLYLQMNSLRAEDTAVYYCAKWRGNPTDSGMDVWGQGTTVTVSS |
| | | | SEQ ID NO: 27372 | SEQ ID NO: 31378 |
| **iPS:436732** | **21-225_152B12** | NA | TCCTATGAGCTGACTCAGCCACCCTCAGTGTCCGTGTCCCCAGGACAGACAGCCAGCATCACCTGCTCTGGCGATAAATTGGGAAATAAATATGCTTGCTGGTATCAGCAGAAGCCAGGCCAGTCCCCTGTGCTGGTCATCTATCAAGATACCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTTATGTCTTCGGAACTGGGACCAAGGTCACCGTCCTA | CAGGTGCAGGTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTACAAATGAACAGCCTGAGAGCCGAGGACACGGCTGTGTATTACTGTGCGAGAGATGACCGATCTTGTAGTAGTACCAGCTGCCCTTACTACTACTACTACGGTTTGGACGTCTGGGGCCAAGGGACCACGGTCACCGTCTCCTCA |
| | | | SEQ ID NO: 27373 | SEQ ID NO: 31379 |

FIGURE 50

| | | | | |
|---|---|---|---|---|
| | | AA | SYELTQPPSVSVSPGQTASITCSGDKLGNKYACWYQQKPGQSPVLVIYQDTKRPSGIPERFSGSNSGNTATLTISGTQAMDEADYYCQAWDSSTYVFGTGTKVTVL | QVQVVESGGGVVQPGRSLRLSCAASGFTFSDYGMHWVRQAPGKGLEWVAVIWYDGSNKYYADSVKGRFTISRDNSKNTLYLQMNSLRAEDTAVYYCARDDRSCSSTSCPYYYYYGLDVWGQGTTVTVSS |
| | | | SEQ ID NO: 27374 | SEQ ID NO: 31380 |
| iPS:436734 | 21-225_153A8 | NA | TCCTATGAGCTGACTCAGCCACCCTCAGTGTCCGTGTCCCCAGGGCAGACAGCCAGCATCACCTGCTCTGGAGATAAATTGGGGGATAAATATGCTTGCTGGTATCAGCAGAAGCCAGGCCAGTCCCCTGTGCTGGTCATCTATCAAGATACCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTTATGTCTTCGGAACTGGGACCAAGGTCACCGTCCTA | CAGGTGCAGCTGATGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCACTTATATGGTATGATGGAAGTAATAAATACTATGCAGACTCCGTGAAGGGCCGATTCACCATCTCCAGAGACAATTCCAAGAACACGCTTTATCTACAAATGAACAGCCTGAGAGCCGAGGACACGGCTGTGTATTACTGTGCGAGAGATGACCGATCTTGTAGTAGAACCAGCTGCCCTTACTACTACTACTACGGTTTGGACGTCTGGGGCCAAGGGACCACGGTCATCGTCTCCTCA |
| | | | SEQ ID NO: 27375 | SEQ ID NO: 31381 |
| | | AA | SYELTQPPSVSVSPGQTASITCSGDKLGDKYACWYQQKPGQSPVLVIYQDTKRPSGIPERFSGSNSGNTATLTISGTQAMDEADYYCQAWDSSTYVFGTGTKVTVL | QVQLMESGGGVVQPGRSLRLSCAASGFTFSDYGMHWVRQAPGKGLEWVALIWYDGSNKYYADSVKGRFTISRDNSKNTLYLQMNSLRAEDTAVYYCARDDRSCSRTSCPYYYYYGLDVWGQGTTVIVSS |
| | | | SEQ ID NO: 27376 | SEQ ID NO: 31382 |

FIGURE 50

| | | | | |
|---|---|---|---|---|
| **iPS:436736** | **21-225_153E8** | NA | TCCTATGAGCTGACTCAGCCACCCTCAGTGTCCGTGTCCCCAGGACAGACAGCCAGCATCACCTGCTCTGGAAGTAAATTGGGTAATAAATATGTTTGCTGGTATCAGCAGAGGCCAGGCCAGTCCCCTGTACTGGTCATCTATCAAGATAACAAGCGGCCCTCAGGGATCCCTGAGCGATTCTCTGGCTCCAACTCCGGAAACACAGCCACTCTGACCATCAGCGGGACCCAGGCTATGGATGAGGCTGGCTATTACTGTCAGGCGTGGGACAGCAGCACTTATGTGATATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCCTCAGTAACTATGGCATGCACTGGGTCCGCCAGGCTCCAGGCAAGGGGCTGGAGTGGGTGGCAGTTATATGGTTTGATGGCAGTAATAAATACTATGTTGACTCCGTGAAGGACCGATTCACCATCTCCAGAGACAATTCCAAGAACACGCTATATCTGCAAATGAACAGCCTGAGAGCCGAAGACACGGCTGTGTATTACTGTGCGAGAGATCGGGTGGAGGGTTCGGGGACTCCCTACTACTACTACGGTATGGACGTCTGGGGCCAAGGGACCACGGTCACCGTCTCCTCA |
| | | | SEQ ID NO: 27377 | SEQ ID NO: 31383 |
| | | AA | SYELTQPPSVSVSPGQTASITCSGSKLGNKYVCWYQQRPGQSPVLVIYQDNKRPSGIPERFSGSNSGNTATLTISGTQAMDEAGYYCQAWDSSTYVIFGGGTKLTVL | QVQLVESGGGVVQPGRSLRLSCAASGFTLSNYGMHWVRQAPGKGLEWVAVIWFDGSNKYYVDSVKDRFTISRDNSKNTLYLQMNSLRAEDTAVYYCARDRVEGSGTPYYYYGMDVWGQGTTVTVSS |
| | | | SEQ ID NO: 27378 | SEQ ID NO: 31384 |
| **iPS:436738** | **21-225_153D9** | NA | TCCTATGAGCTGACTCAGCCACCCTCAGTGTCCGTGTCCCCAGGACAGACAGCGAGCATCACCTGCTCTGGAGATAAATTGGGGGATAAATATGCTTGCTGGTATCAGCAGAAGCCAGGCCAGTCCCCTGTACTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGCACAGCAGTACTGTGGTATTCGGCGGAGGGACCAAGGTGACCGTCCTA | CAGGTGCAGTTGGTGGAGTCTGGGGGAGGCGTGGTCCAGCCTGGGAGGTCCCTGAGACTCTCCTGTGCAGCGTCTGGATTCACCGTCAGTACCTATGGCATGCACTGGGTCCGCCAGGCTCCAGGCAAGGGGCTGGAGTGGGTGGCAGTTATATGGTATGGTGGAAGTAATAAAGACTATGCAGACTCCGTGAAGGGCCGATTCACCATCTCCAGAGACATTTCCAAGAACACGCTGTATCTGCAAATGAACAGCCTGAGAGCCGAGGACACGGCTGTGTATTACTGTGCGAGAGATCGGGATTATTGTAGTGGTGGTAGCTGTCCTTACTACTACTACTACGGTATGGACGTCTGGGGCCAAGGGACCACGGTCACCGTCTCCTCA |

FIGURE 50

| | | | | |
|---|---|---|---|---|
| | | | SEQ ID NO: 27379 | SEQ ID NO: 31385 |
| | | AA | SYELTQPPSVSVSPGQTASITCSGDKLGDKYACWYQQKPGQSPVLVIYQDRKRPSGIPERFSGSNSGNTATLTISGTQAMDEADYYCQAWHSSTVVFGGGTKVTVL | QVQLVESGGGVVQPGRSLRLSCAASGFTVSTYGMHWVRQAPGKGLEWVAVIWYGGSNKDYADSVKGRFTISRDISKNTLYLQMNSLRAEDTAVYYCARDRDYCSGGSCPYYYYYGMDVWGQGTTVTVSS |
| | | | SEQ ID NO: 27380 | SEQ ID NO: 31386 |
| iPS:436740 | 21-225_154C3 | NA | TCCTATGAGCTGACTCAGCCACCCTCAGTGTCCGTGTCCCCAGGACAGACAGCCAGCATCACCTGCTCTGGAGATAAATTGGGGGATAAATATGTTTGCTGGTATCAGCAGAAGCCAGGCCAGTCCCCTGTGTTGGTCATCTATCAAGATAGGAAGCGGCCCTCAGGGATCCCTGAGCGATTCTCTGGCTCCAACTCTGGGAACACAGCCACTCTGACCATCACCGGGACCCAGGCTATGGATGAGGCTGACTATTACTGTCAGGCGTGGCACAGCAGCACTGTGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTACAGCGTCTGGATTCACCTTCAGTACCTATGGCATGCACTGGGTCCGCCAGGCTCCAGGCAAGGGGCTGGAGTGGGTGGCAGTTGTATGGTATGGTGGAAATAATAAAGACTATGCAGACTCCGTGAAGGGCCGATTCACCATCTCCAGAGACATTTCCAAGAACACACTGTATCTGCAAATGAACAGCCTGAGAGCCGAGGACACGGCTGTGTATTACTGTGCGAGAGATCGGGATTATTGTAGTGGTGGTAGCTGCCCTTACTACTACTACTACGGTATGGACGTCTGGGGCCAAGGGACCACGGTCACCGTCTCCTCA |
| | | | SEQ ID NO: 27381 | SEQ ID NO: 31387 |
| | | AA | SYELTQPPSVSVSPGQTASITCSGDKLGDKYVCWYQQKPGQSPVLVIYQDRKRPSGIPERFSGSNSGNTATLTITGTQAMDEADYYCQAWHSSTVVFGGGTKLTVL | QVQLVESGGGVVQPGRSLRLSCTASGFTFSTYGMHWVRQAPGKGLEWVAVVWYGGNNKDYADSVKGRFTISRDISKNTLYLQMNSLRAEDTAVYYCARDRDYCSGGSCPYYYYYGMDVWGQGTTVTVSS |
| | | | SEQ ID NO: 27382 | SEQ ID NO: 31388 |

FIGURE 50

| | | | | |
|---|---|---|---|---|
| **iPS:436742** | **21-225_154C4** | NA | TCCTATGAGCTGACTCAGCCACCCTCAGTGTCCGTGTCCCCAGGACAGACAGCCAGAATCACCTGCTCTGGAGATAAATTGGGGGATAAATATGCTTCCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGATCCAGGCTATGGATGAGGCTGACTATTACTGTCAGGCGTGGGACAGCAGCACTGTGGTATTCGGCGGAGGGACCAAGCTGACCGTCCTA | GAGGTGCAACTGTTGGAGTCTGGGGGAGGCTTGATACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTATTAGTGGTGGTGGTAGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATGGCGAGGTAACCCCACTGACTACGGTATGGACGTCTGGGGCCAAGGGACCACGGTCACCGTCTCCTCA |
| | | | SEQ ID NO: 27383 | SEQ ID NO: 31389 |
| | | AA | SYELTQPPSVSVSPGQTARITCSGDKLGDKYASWYQQKPGQSPVLVIYQDSKRPSGIPERFSGSNSGNTATLTISGIQAMDEADYYCQAWDSSTVVFGGGTKLTVL | EVQLLESGGGLIQPGGSLRLSCAASGFTFSSYAMSWVRQAPGKGLEWVSVISGGGSSTYYADSVKGRFTISRDNSKNTLYLQMNSLRAEDTAVYYCAKWRGNPTDYGMDVWGQGTTVTVSS |
| | | | SEQ ID NO: 27384 | SEQ ID NO: 31390 |
| **iPS:436744** | **21-225_154F4** | NA | TCCTATGAGCTGACTCAGCCACCCTCAGTGTCCGTGTCCCCAGGACAGACAGCCAGCATCACCTGCTCTGGAGATAAATTGGGAAATAAATATGTTTGTTGGTATCAGCAGAAGCCAGGCCAGTCCCCTGTGGAAGTCATCTATAAAGATAGTAAGCGGCCCTCAGGGATCCCTGAGCGATTCTCTGGCTCCAATTCTGGGAACACAGGCACTCTGACCATCAGCGGGACCCAGGCTATGGATGAGGCTGACTATTACTGTCAGGCGTGGGACAACAGTACTTTAGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAGGATTCCTATTGTAGTGGTACCAGCTGCCCCTACTACTACTACTACGGTATGGACGTCTGGGGCCAAGGGACCACGGTCACCGTCTCCTCA |

FIGURE 50

| | | | | |
|---|---|---|---|---|
| | | | SEQ ID NO: 27385 | SEQ ID NO: 31391 |
| | | AA | SYELTQPPSVSVSPGQTASITCSGDKLGNKYVCWYQQKPGQSPVEVIYKDSKRPSGIPERFSGSNSGNTGTLTISGTQAMDEADYYCQAWDNSTLVFGGGTKLTVL | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCAREDSYCSGTSCPYYYYYGMDVWGQGTTVTVSS |
| | | | SEQ ID NO: 27386 | SEQ ID NO: 31392 |
| iPS:436746 | 21-225_154E10 | NA | TCCTATGAGCTGACTCAGCCACCCTCAGTGTCCGTGTCCCCAGGACAGACAGCCAGCATCACCTGCTCTGGAGATAAATTGGGGGATAAATATGCTTCCTGGTATCAGCAGAAGCCAGGTCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGATCCAGGCTATGGATGAGGCTGACTATTACTGTCAGGCGTGGGACAACAGCACTGTGGTATTCGGCGGAGGGACCAAGCTGACCGTCCTA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTATTAGTGGTGGTGGTAGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATGGCGAGGTAACCCCACTGACTACGGTATGGACGTCTGGGGCCAAGGGACCACGGTCACCGTCTCCTCA |
| | | | SEQ ID NO: 27387 | SEQ ID NO: 31393 |
| | | AA | SYELTQPPSVSVSPGQTASITCSGDKLGDKYASWYQQKPGQSPVLVIYQDSKRPSGIPERFSGSNSGNTATLTISGIQAMDEADYYCQAWDNSTVVFGGGTKLTVL | EVQLLESGGGLVQPGGSLRLSCAASGFTFSSYAMSWVRQAPGKGLEWVSVISGGGSSTYYADSVKGRFTISRDNSKNTLYLQMNSLRAEDTAVYYCAKWRGNPTDYGMDVWGQGTTVTVSS |
| | | | SEQ ID NO: 27388 | SEQ ID NO: 31394 |

FIGURE 50

| | | | | |
|---|---|---|---|---|
| **iPS:436748** | **21-225_154D11** | NA | TCCTATGAGCTGACTCAGCCACCCTCAGTGTC<br>CGTGTCCCCAGGACAGACAGCCAGCATCACCT<br>GCTCTGGAGATAAATTGGGGGATAAATATGCT<br>TGCTGGTATCAGCAGAAGCCAGGCCAGTCCCC<br>TGTGCTGGTCATCTATCAAGATAAGAAGCGGC<br>CCTCAGGGATCCCTGAGCGATTCTCTGGCTCC<br>AACTCTGGGAACATAGCCACTCTGACCATCAG<br>CGGGACCCAGGCTATGGATGAGGCTGACTATT<br>ACTGTCAGGCGTGGCACAGCAGTATTGTGGTA<br>TTCGGCGGAGGGACCAAGCTGACCGTCCTA | CAGGTGCAGCTGGTGGAGTCTGGGGGAGGGGTG<br>GTCCAGCCTGGGAGGTCCCTGAGACTCTCCTGTG<br>CAGCGTCTGGATTCAACGTCAGTACCTATGGCAT<br>GCACTGGGTCCGCCAGGCTCCAGGCAAGGGGCT<br>GGAGTGGGTGGCAGTTATATGGTATGGTGGAAGT<br>AATAAAGACTATGCAGACTCTGTGAAGGGCCGA<br>TTCACCATCTCCAGAGACAGTTCCAAGAACACGC<br>TGTATCTGCAAATGAACAGCCTGAGAGCCGAGG<br>ACACGGCTGTGTATTACTGTGCGAGAGATCGGGA<br>TTATTGTAGTGGTGGTAGTTGCCCTTACTACTACT<br>ACTACGGTATGGACGTCTGGGGCCAAGGGACCA<br>CGGTCACCGTCTCCTCA |
| | | | SEQ ID NO: 27389 | SEQ ID NO: 31395 |
| | | AA | SYELTQPPSVSVSPGQTASITCSGDKLGDKYACW<br>YQQKPGQSPVLVIYQDKKRPSGIPERFSGSNSGNI<br>ATLTISGTQAMDEADYYCQAWHSSIVVFGGGTK<br>LTVL | QVQLVESGGGVVQPGRSLRLSCAASGFNVSTYGM<br>HWVRQAPGKGLEWVAVIWYGGSNKDYADSVKGR<br>FTISRDSSKNTLYLQMNSLRAEDTAVYYCARDRDY<br>CSGGSCPYYYYYGMDVWGQGTTVTVSS |
| | | | SEQ ID NO: 27390 | SEQ ID NO: 31396 |

FIGURE 50

| | | | | |
|---|---|---|---|---|
| **iPS:436750** | **21-225_154G12** | NA | CAGTCTGTACTGACTCAGCCACCCTCAGCGTCTGGGACCCCCGGGCAGAGGGTCACCATCTCTTGTTCTGGAAGCAGCTCCAACATCGGAAATAATGCTGTAAGCTGGTATCAGCAGCTCCCAGGAACGGCCCCCAAACTCCTCATCTATAGTAATGATCACCGGCCCTCAGGGGTCCCTGACCGATTCTCTGGCTCCAAGTCTGGCACCTCAGCCTCCCTGGCCATCAGTGGGCTCCAGTCTGAGGATGAGGCTGATTATTACTGTGCAGCATGGGATGACAGCCTGAAGGGTCCGGTATTCGGCGGAGGGACCAAGCTGACCGTCCTA | CAGGTGCAGCTGCAGGAGTCGGGCCCAGGACTGGTGAATCCTTCACAGACCCTGTCCCTCACCTGCGGTGTCTCTGGTGGCTCCATCAGCAGTGGTTATTACTACTGGAGCTGGATCCGCCAGCACCCAGGGAAGGGCCTGGAGTGGATTGGGTACATCTATTATAGTGGGAGCACCTACTACAACCCGTCCCTCAAGAGTCGAGTTTCCATATCATTAGACACGCCTAAGAACCAGTTCTCCCTGAAGCTGACCTCTGTGACTGCCGCGGACACGGCCGTGTATTACTGTGCGAGAGATTGGGGTGGCTACGATTCGAGTGGCTGGTTCGACCCCTGGGGCCAGGGAACCCTGGTCACCGTCTCCTCA |
| | | | SEQ ID NO: 27391 | SEQ ID NO: 31397 |
| | | AA | QSVLTQPPSASGTPGQRVTISCSGSSSNIGNNAVSWYQQLPGTAPKLLIYSNDHRPSGVPDRFSGSKSGTSASLAISGLQSEDEADYYCAAWDDSLKGPVFGGGTKLTVL | QVQLQESGPGLVNPSQTLSLTCGVSGGSISSGYYYWSWIRQHPGKGLEWIGYIYYSGSTYYNPSLKSRVSISLDTPKNQFSLKLTSVTAADTAVYYCARDWGGYDSSGWFDPWGQGTLVTVSS |
| | | | SEQ ID NO: 27392 | SEQ ID NO: 31398 |
| **iPS:436752** | **21-225_155H1** | NA | CAGTCTGTGCTGACGCAGCCGCCCTCAGTGTCTGGGGCCCCAGGGCAGAGGGTCACCATCTCCTGCACTGGGAGCAGCTCCAATATCGGGGCAGGTTATGATGTACACTGGTACCAGCAGCTTCCAGGAACAGCCCCCAAACTCCTCATCTATGGTAACAGCAATCGGCCCTCAGGGGTCCCTGACCGATTCTCTGGCTCCAAGTCTGGCACCTCAGCCTCCCTGGCCATCACTGGCCTCCAGGCTGAGGATGAGGCTGATTATCACTGCCAGTCCTATGACAGCAGCCTGAGTGGTCCTGTGATATTCGGCGGAGGGACCAAGCTGACCGTCCTA | GAGGTGCAGCTAGTGCAGTCTGGAGCAGAGGTGAAAAAGCCCGGGGAGTCTCTGAAGATCTCCTGTAAGGGTTCTGGATACAGCTTTACCAGCTACTGGATCGGCTGGGTGCGCCAGATGCCCGGGAAAGGCCTGGAGTGGATGGGGCTCATCTATCCTGGTGCCTCTGATACCAGATACAGCCCGTCCTTCCAAGGCCAGGTCACCATCTCAGCCGACAAGTCCATCAGCACCGCCTACCTGCAGTGGAGCAGCCTGAAGGCCTCGGACACCGCCATGTATTACTGTGCGAGACAGGCCATAGCAAGTCGAGGGAGGTACTACTACTACGGTATGGACGTCTGGGGCCAAGGGACCACGGTCACCGTCTCCTCA |
| | | | SEQ ID NO: 27393 | SEQ ID NO: 31399 |

FIGURE 50

| | | | | |
|---|---|---|---|---|
| | | AA | QSVLTQPPSVSGAPGQRVTISCTGSSSNIGAGYD VHWYQQLPGTAPKLLIYGNSNRPSGVPDRFSGS KSGTSASLAITGLQAEDEADYHCQSYDSSLSGPV IFGGGTKLTVL | EVQLVQSGAEVKKPGESLKISCKGSGYSFTSYWIG WVRQMPGKGLEWMGLIYPGASDTRYSPSFQGQVT ISADKSISTAYLQWSSLKASDTAMYYCARQAIASR GRYYYYGMDVWGQGTTVTVSS |
| | | | SEQ ID NO: 27394 | SEQ ID NO: 31400 |
| iPS:436754 | 21-225_155G3 | NA | TCCTATGAGCTGACTCAGCCACCCTCAGTGTC CGTGTCCCCAGGACAGACAGCCAGCATCACCT GCTCTGGAGATAAGTTGGGGGATAAATATGTT TGCTGGTATCAGCAGAAGCCAGGCCAGTCCCC TATGTTGGTCATCTATCAAGATAGTAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAATAGTATTTATGTC TTCGGAACTGGGACCAAGGTCACCGTCCTA | CAGGTGCAGCTGGTGGAGTCTGGGGGAGGCGTG GTCCAGCCTGGGAGGTCCCTGAGACTCTCCTGTA CAGCCTCTGGATTCACCTTCAGTAGCTATGGCAT GCACTGGGTCCGCCAGGCTCCAGGCAAGGGGCT GGAGTGGGTGGCAGTTATATCATATGATGGAAGT AATAAATACTATGCAGACTCCGTGAAGGGCCGA TTCACCATCTCCAGAGACAATTCCAAGAACACGC TGTATCTGCAAATGAACAGCCTGAGAGCTGAGG ACACGGCTGTGTATTACTGTGCGAGAGATACGGA GAGATGGCTACCATACTCCTACGGTATGGACGTC TGGGGCCAAGGGACCACGGTCACCGTCTCCTCA |
| | | | SEQ ID NO: 27395 | SEQ ID NO: 31401 |
| | | AA | SYELTQPPSVSVSPGQTASITCSGDKLGDKYVCW YQQKPGQSPMLVIYQDSKRPSGIPERFSGSNSGN TATLTISGTQAMDEADYYCQAWDNSIYVFGTGT KVTVL | QVQLVESGGGVVQPGRSLRLSCTASGFTFSSYGMH WVRQAPGKGLEWVAVISYDGSNKYYADSVKGRFT ISRDNSKNTLYLQMNSLRAEDTAVYYCARDTERW LPYSYGMDVWGQGTTVTVSS |
| | | | SEQ ID NO: 27396 | SEQ ID NO: 31402 |

FIGURE 50

| | | | | |
|---|---|---|---|---|
| **iPS:436756** | **21-225_146A10** | NA | TCCTATGAGCTGACTCAGCCACCCTCAGTGTC<br>CGTGTCCCCAGGACAGACAGCCAGCATCACCT<br>GCTCTGGAGATAAATTGGGGGATAAATATGTT<br>TGCTGGTATCAGCAGAAGCCAGGCCAGTCCCC<br>TGTGCTGGTCATCTTTCAAGATAGAAAGCGGC<br>CCTCAGGGATCCCTGAGCGATTCTCTGGCTCC<br>AACTCTGGGAACACAGCCACTCTGACCATCAG<br>CGGGACCCAGGCTATGGATGAGGCTGACTATT<br>ACTGTCAGGCGTGGGACAGCAGCACTGTGGTG<br>TTCGGCGGAGGGACCAAAGTTACCGTCCTA | CAGGTGCAGCTGGAGGAGTCTGGGGGAGGCGTG<br>GTCCAGCCTGGGAGGTCCCTGAGACTCTCCTGTG<br>CAGCGTCTGGATTCACCTTCAGTGGCTATGGCAT<br>GCACTGGGTCCGCCAGGCTCCAGGCAAGGGGCT<br>GGAGTGGATGACACTTATACGGTATGATGGAAG<br>CGATAAAAACTATGCAGACTCCGTGAAGGGCCG<br>ATTCACCATCTCCAGAGACAACTCCAAGAACACG<br>CTGTATCTGCAAATGAACAGCCTGAGAGCCGAG<br>GACACGGCTGTGTATTACTGTGCGAGAGATCGGG<br>TTTTTTGTAGTAGTACCAGCTGCCTCTCTTACTAC<br>TACTACTACGGTATGGACGTCTGGGGCCAAGGG<br>ACCACGGTCACCGTCTCCTCA |
| | | | SEQ ID NO: 27397 | SEQ ID NO: 31403 |
| | | AA | SYELTQPPSVSVSPGQTASITCSGDKLGDKYVCW<br>YQQKPGQSPVLVIFQDRKRPSGIPERFSGSNSGN<br>TATLTISGTQAMDEADYYCQAWDSSTVVFGGG<br>TKVTVL | QVQLEESGGGVVQPGRSLRLSCAASGFTFSGYGMH<br>WVRQAPGKGLEWMTLIRYDGSDKNYADSVKGRF<br>TISRDNSKNTLYLQMNSLRAEDTAVYYCARDRVFC<br>SSTSCLSYYYYYGMDVWGQGTTVTVSS |
| | | | SEQ ID NO: 27398 | SEQ ID NO: 31404 |

FIGURE 50

| | | | | |
|---|---|---|---|---|
| iPS:436758 | 21-225_155C10 | NA | TCCTATGAGCTGACTCAGCCACCCTCAGTGTCCGTGTCCCCAGGACAGACAGTCAGCATCACCTGCTCTGGAGATAAATTGGGGGATAAATATGTTTCCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGATCCAGGCTATGGATGAGGCTGACTATTACTGTCAGGCGTGGGACAGCAGCACTGTGGTATTCGGCGGAGGGACCAAGCTGACCGTCCTA | GAGGTGCAACTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTATTAGTGGTGGTGGTAGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATGGCGAGGTAACCCCACTGACTACGGTATGGACGTCTGGGGCCAAGGGACCACGGTCACCGTCTCCTCA |
| | | | SEQ ID NO: 27399 | SEQ ID NO: 31405 |
| | | AA | SYELTQPPSVSVSPGQTVSITCSGDKLGDKYVSWYQQKPGQSPVLVIYQDSKRPSGIPERFSGSNSGNTATLTISGIQAMDEADYYCQAWDSSTVVFGGGTKLTVL | EVQLLESGGGLVQPGGSLRLSCAASGFTFSSYAMSWVRQAPGKGLEWVSVISGGGSSTYYADSVKGRFTISRDNSKNTLYLQMNSLRAEDTAVYYCAKWRGNPTDYGMDVWGQGTTVTVSS |
| | | | SEQ ID NO: 27400 | SEQ ID NO: 31406 |
| iPS:436760 | 21-225_155E10 | NA | TCCTATGAGCTGACTCAGCCGCCCTCAGTGTCCGTGTCCCCAGGACAGACAGCCAGCATCACCTGCTCTGGAGATAAATTGGGGGATAAATATGTTTCCTGGTATCAGCAGAAGCCAGGCCAGTCCCCTGTGCTGGTCATCTATCAAGATAGGAAGCGGCCCTCAGGGACCCCTGAGCGATTCTCTGGCTCCAACTCTGGGAACACAGCCACTCTGACCATCAGCGGGACCCAGGCTATGGATGAGGCTGACTATTACTGTCTGGCGTGGGACAGCAGCACTTTTGTGGTATTCGGCGGAGGGACCAAGTTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGTGACTACGGTGACCCCCCCTACTACTACTACTACGGTATGGACGTCTGGGGCCAAGGGACCACGGTCACCGTCTCCTCA |
| | | | SEQ ID NO: 27401 | SEQ ID NO: 31407 |

FIGURE 50

| | | | | |
|---|---|---|---|---|
| | | AA | SYELTQPPSVSVSPGQTASITCSGDKLGDKYVSWYQQKPGQSPVLVIYQDRKRPSGTPERFSGSNSGNTATLTISGTQAMDEADYYCLAWDSSTFVVFGGGTKLTVL | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARDRDYGDPPYYYYYGMDVWGQGTTVTVSS |
| | | | SEQ ID NO: 27402 | SEQ ID NO: 31408 |
| iPS:436762 | 21-225_156H2 | NA | CAGTCTGTGGTGACTCAGCCACCCTCAGCGTCTGGGACCCCCGGGCAGAGGGTCACCATCTCTTGTTCTGGAAGCAGCTCCAACATCGGAAGTAATACTGTAAATTGGTACCAGCAGCTCCCAGGAACGGCCCCCAAACTCCTCATCTATAGTAGTAATCAGCGGCCCTCAGGGGTCCCTGACCGATTGTCTGGCTCCAAGTCTGGCACCTCAGCCTCCCTGGCCATCAGTGGGCTCCAGTCTGAGGATGAGGCTGATTATTACTGTGCAGCATGGGATGACAGCCTGAATGGCGTGGTATTCGGCGGAGGGACCAAGGTGACCGTCCTA | GAGGTGCAGCTGGTGGAGTCTGGGGGAGGCTTGATACAGCCTGGGGGGTCCCTGAGACTCTCCTGTGCAGCCTCTGGATTCACCTTCAGTAACTATAACATGAACTGGGTCCGCCAGGCTCCAGGGAAGGGGCTGGAGTGGGTTTCATACATTAGTAGAAGTAGTAATACCAAATACTACGCAGACTCTGTGAAGGGCCGATTCACCATCTCCAGAGACAATGCCAAGAACTCACTGTATCTGCAAATGAACAGCCTGAGAGACGAGGACACGGCTGTGTATTACTGTGCGAGAGATAGGAGTGGGAGCTACGGGTACTTCTACTACTACGGTATGGACGTCTGGGGCCAAGGGACCACGGTCACCGTCTCCTCA |
| | | | SEQ ID NO: 27403 | SEQ ID NO: 31409 |
| | | AA | QSVVTQPPSASGTPGQRVTISCSGSSSNIGSNTVNWYQQLPGTAPKLLIYSSNQRPSGVPDRLSGSKSGTSASLAISGLQSEDEADYYCAAWDDSLNGVVFGGGTKVTVL | EVQLVESGGGLIQPGGSLRLSCAASGFTFSNYNMNWVRQAPGKGLEWVSYISRSSNTKYYADSVKGRFTISRDNAKNSLYLQMNSLRDEDTAVYYCARDRSGSYGYFYYYGMDVWGQGTTVTVSS |
| | | | SEQ ID NO: 27404 | SEQ ID NO: 31410 |

FIGURE 50

| | | | | |
|---|---|---|---|---|
| **iPS:436764** | **21-225_158E9** | NA | TCCTATGACCTGACTCAGCCACCCTCAGTGTCCGTGTCCCCAGGACAGACAGCCAGCATCACCTGCTCTGGAGATAAATTGGGGGATAAATATGTTTGCTGGTATCAGCAGAAGCCAGGCCAGTCCCCTGTGCTGGTCATCTATCAAGATAGTAAGCGGCCCTCAGGGATCCCTGAGCGATTCTCTGGCTCCAACTCTGGGAACACAGCCACTCTGACCATCACCGGGACCCAGGCTATGGATGAGGCTAACTATTACTGTCAGGCGTGGGACAACAGCAGCTTTGTGCTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAGTAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGGGTTTTTTGTAGTGGTACCAGCTGCCCTTACTACTACTACTACGGTATGGACGTCTGGGGCCAAGGGACCACGGTCACCGTCTCCTCA |
| | | | SEQ ID NO: 27405 | SEQ ID NO: 31411 |
| | | AA | SYDLTQPPSVSVSPGQTASITCSGDKLGDKYVCWYQQKPGQSPVLVIYQDSKRPSGIPERFSGSNSGNTATLTITGTQAMDEANYYCQAWDNSSFVLFGGGTKLTVL | QVQLVESGGGVVQPGRSLRLSCAASGFTFSSYGMHWVRQAPGKGLEWVAVIWYDGSSKYYADSVKGRFTISRDNSKNTLYLQMNSLRAEDTAVYYCARDRVFCSGTSCPYYYYYGMDVWGQGTTVTVSS |
| | | | SEQ ID NO: 27406 | SEQ ID NO: 31412 |

FIGURE 50

| | | | | |
|---|---|---|---|---|
| **iPS:436766** | **21-225_158D10** | NA | TCCTATGAGCTGACTCAGCCACCCTCAGTGACCGTGTCCCCAGGACAGACAGCCAGCATCACCTGCTCTGGAGATAAATTGGGGGATAAATATGTTTGCTGGTATCAGCAGAAGCCAGGCCAGTCCCCTGTGCTGGTCATCTATCAAGATCGCAAGCGGCCCTCAGGGATCCCTGAGCGATTGTCTGGCTCCAACTCTGGGAACACAGCCACTCTGACCATCAGCGGGACCCAGGCTCTGGATGAGGCTGACTATTACTGTCAGGCGTGGGGCAACAGCAGCTTTGTGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGGGTTTCTTGTAGTAGTACCAGCTGCCCTTACTACTACTACTACGGTATGGACGTCTGGGGCCAAGGGACCACGGTCACCGTCTCCTCA |
| | | | SEQ ID NO: 27407 | SEQ ID NO: 31413 |
| | | AA | SYELTQPPSVTVSPGQTASITCSGDKLGDKYVCWYQQKPGQSPVLVIYQDRKRPSGIPERLSGSNSGNTATLTISGTQALDEADYYCQAWGNSSFVVFGGGTKLTVL | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARDRVSCSSTSCPYYYYYGMDVWGQGTTVTVSS |
| | | | SEQ ID NO: 27408 | SEQ ID NO: 31414 |

FIGURE 50

| | | | | |
|---|---|---|---|---|
| **iPS:436768** | **21-225_159H8** | NA | TCCTATGAGCTGACTCAGCCACCCTCAGTGTCCGTGTCCCCAGGACAGACAGCCAGCATCACCTGCTCTGGAGATAAATTGGGGGATAAATATGTTTGCTGGTATCAGCAGAAGCCAGGCCAGTCCCCTGTGTTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTCTGGATGAGGCTGACTATTACTGTCAGGCGTGGGGCAACAGCAGCTTTGTGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTACCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGGGTTTCTTGTAGTAGTACCAGCTGCCCTTACTACTACTACTACGGTATGGACGTCTGGGGCCAAGGGACCACGGTCACCGTCTCCTCA |
| | | | SEQ ID NO: 27409 | SEQ ID NO: 31415 |
| | | AA | SYELTQPPSVSVSPGQTASITCSGDKLGDKYVCWYQQKPGQSPVLVIYQDRKRPSGIPERFSGSNSGNTATLTISGTQALDEADYYCQAWGNSSFVVFGGGTKLTVL | QVQLVESGGGVVQPGRSLRLSCAASGFTFSTYGMHWVRQAPGKGLEWVAVIWYDGSNKYYADSVKGRFTISRDNSKNTLYLQMNSLRAEDTAVYYCARDRVSCSSTSCPYYYYYGMDVWGQGTTVTVSS |
| | | | SEQ ID NO: 27410 | SEQ ID NO: 31416 |

FIGURE 50

| | | | | |
|---|---|---|---|---|
| **iPS:436770** | **21-225_160B12** | NA | TCCGATGAGCTGACTCAGTCACCCTCAGTGTCCGTGTCCCCAGGACAGACAGCCAGCATCACCTGCTCTGGAGATAAATTGGGGGATAAATATGTTTGCTGGTATCAGCAGAAGCCAGGCCAGTCCCCTGTGTTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTCTGGATGAGGCTGACTATTACTGTCAGGCGTGGGGCAACAGCAGCTTTGTGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGGGTTTCTTGTAGTAGTACCAGCTGCCCTTACTACTACTACTACGGTATGGACGTCTGGGGCCAAGGGACCACGGTCACCGTCTCCTCA |
| | | | SEQ ID NO: 27411 | SEQ ID NO: 31417 |
| | | AA | SDELTQSPSVSVSPGQTASITCSGDKLGDKYVCWYQQKPGQSPVLVIYQDSKRPSGIPERFSGSNSGNTATLTISGTQALDEADYYCQAWGNSSFVVFGGGTKLTVL | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARDRVSCSSTSCPYYYYYGMDVWGQGTTVTVSS |
| | | | SEQ ID NO: 27412 | SEQ ID NO: 31418 |

FIGURE 50

| | | | | |
|---|---|---|---|---|
| **iPS:436772** | **21-225_161H3** | NA | TCCTATGAGCTGACTCAGCCACCCTCAGTGTC<br>CGTGTCCCCAGGACAGACAGCCAGCATCACCT<br>GCTCTGGAGATAGATTGGGGGATAAATATGTT<br>TGCTGGTATCAGCAGAAGCCAGGCCAGTCCCC<br>TGTACTGGTCATCTTTCAAGATAACAAGCGGC<br>CCTCAGGGATCCCTGAGCGATTCTCTGGCTCC<br>AACTCTGGGAACACAGCCACTCTGACCATCAG<br>CGGGACCCAGGCTCTGGATGAGGCTGACTATT<br>ACTGTCAGGCGTGGGTCAACAACACTGCAGTG<br>GTTTTCGGCGGAGGGACCAAGCTGACCGTCCT<br>A | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTAGCTATGGCAT<br>GCACTGGGTCCGCCAGGCTCCAGGCAAGGGGCT<br>GGAGTGGGTGGCAGTTATATGGTATGATGGAAGT<br>AATAAATACTATGCAGACTCCGTGAAGGGCCGA<br>TTCACCATCTCCAGAGACAATTCCAAGAACACGC<br>TGTATCTGCAAATGAACAGCCTGAGAGCCGAGG<br>ACACGGCTGTGTATTACTGTGCGAGAGTCGGGTA<br>TAGCGGTGGCTGGTACATCTTTGACTACTGGGGC<br>CAGGGAACCCTGGTCACCGTCTCCTCA |
| | | | SEQ ID NO: 27413 | SEQ ID NO: 31419 |
| | | AA | SYELTQPPSVSVSPGQTASITCSGDRLGDKYVCW<br>YQQKPGQSPVLVIFQDNKRPSGIPERFSGSNSGN<br>TATLTISGTQALDEADYYCQAWVNNTAVVFGG<br>GTKLTVL | QVQLVESGGGVVQPGRSLRLSCAASGFTFSSYGMH<br>WVRQAPGKGLEWVAVIWYDGSNKYYADSVKGRF<br>TISRDNSKNTLYLQMNSLRAEDTAVYYCARVGYSG<br>GWYIFDYWGQGTLVTVSS |
| | | | SEQ ID NO: 27414 | SEQ ID NO: 31420 |
| **iPS:436774** | **21-225_161E10** | NA | TCCTTTGACCTGACTCAGCCACCCTCAGTGTCC<br>GTGTCCCCAGGACAGACAGCCAGCATCACCTG<br>CTCTGGAGATAAATTGGGGGATAAATATGTTT<br>GCTGGTATCAGCAGAAGCCAGGCCAGTCCCCT<br>GTGCTGGTCATCTATCAAGATAGCAAGCGGCC<br>CTCAGGGATCCCTGAGCGAATCTCTGGCTCCA<br>ACTCTGGGAACACAGCCACTCTGACCATCAGC<br>GGGACCCAGGCTATGGATGAGGCTGACTATTA<br>CTGTCAGACGTGGGACAACAGTAGTTTTGCGC<br>TTTTCGGCGGAGGGACCAAGCTGACCGTCCTA | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTAGCTATGGCAT<br>GCACTGGGTCCGCCAGGCTCCAGGCAAGGGGCT<br>GGAGTGGGTGGCAGTTATATGGTATGATGGAAGT<br>AATAAATACTATGTAGACTCCGTGAAGGGCCGAT<br>TCACCATCTCCAGAGACAATTCCAAGAACACGCT<br>GTATCTGCAAATGAACAGCCTGAGAGCCGAGGA<br>CACGGCTGTGTATTACTGTGCGAGAGATCGGGTT<br>TTTTGTAGTGGTACCAGCTGCCCTTACTACTACTA<br>CTACGGTATGGACGTCTGGGGCCAAGGGACCAC<br>GGTCACCGTCTCCTCA |
| | | | SEQ ID NO: 27415 | SEQ ID NO: 31421 |

FIGURE 50

| | | | | |
|---|---|---|---|---|
| | | AA | SFDLTQPPSVSVSPGQTASITCSGDKLGDKYVCW YQQKPGQSPVLVIYQDSKRPSGIPERISGSNSGNT ATLTISGTQAMDEADYYCQTWDNSSFALFGGGT KLTVL | QVQLVESGGGVVQPGRSLRLSCAASGFTFSSYGMH WVRQAPGKGLEWVAVIWYDGSNKYYVDSVKGRF TISRDNSKNTLYLQMNSLRAEDTAVYYCARDRVFC SGTSCPYYYYYGMDVWGQGTTVTVSS |
| | | | SEQ ID NO: 27416 | SEQ ID NO: 31422 |
| iPS:436776 | 21-225_161F12 | NA | TCCTATGAGCTGACTCAGCCACCCTCAGTGTC CGTGTCCCCAGGACAGACAGCCAGCATCACCT GCTCTGGAGATAAATTGGGTGATAAATATGCT TGCTGGTATCAGCAGAAGCCAGGCCAGTCCCC TGTGCTGGTCATCTATCAAGATACCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCACCACTCTGGTT TTCGGCGGAGGGACCAAGCTGACCGTCCTA | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTTA CTACTGGAGCTGGATCCGCCAGCACCCAGGGAA GGGCCTGGAGTGGATTGGGTACATCTATTACAGT GGGAGCCCCTACTACAACCCGTCCCTCAAGAGTC GAATTACCATATCAATAGACACGTCTAAGAACCA GTTCTCCCTGAAGCTGAACTCTGTGACTGCCGCG GACACGGCCGTGTATTACTGTGCGAGATCGAATT GTAGTAGTGCCAACTGCTATACGGTGGGGTTCTA CTACTACGGTTTGGACGTCTGGGGCCGAGGGACC ACGGTCACCGTCTCCTCA |
| | | | SEQ ID NO: 27417 | SEQ ID NO: 31423 |
| | | AA | SYELTQPPSVSVSPGQTASITCSGDKLGDKYACW YQQKPGQSPVLVIYQDTKRPSGIPERFSGSNSGN TATLTISGTQAMDEADYYCQAWDSTTLVFGGG TKLTVL | QVQLQESGPGLVKPSQTLSLTCTVSGGSISSGGYYW SWIRQHPGKGLEWIGYIYYSGSPYYNPSLKSRITISI DTSKNQFSLKLNSVTAADTAVYYCARSNCSSANCY TVGFYYYGLDVWGRGTTVTVSS |
| | | | SEQ ID NO: 27418 | SEQ ID NO: 31424 |

FIGURE 50

| | | | | |
|---|---|---|---|---|
| **iPS:436780** | **21-225_165H3** | NA | TCCTATGAGCTGACTCAGCCACCCTCAGTGTCCGTGTCCCCAGGACAGACAGCCAGCATCACCTGCTCTGGAGATAAATTGGGTGATAAATATGCTTGCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCACCACTCTGGTTTTCGGCGGAGGGACCAAGCTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGGTTACTACTGGAGCTGGATCCGCCAGCACCCAGGGAAGGGCCTGGAGTGGATTGGGTACATCTATTACAGTGGGAGCCCCTACTACAATCCGTCCCTCAAGAGTCGAATTACCATATCAATAGACACGTCTAAGAACCAGTTCTCCCTGAAGCTGAACTCTGTGACTGCCGCGGACACGGCCGTGTATTACTGTGCGAGATCGAATTGTAGTAGTGCCAACTGCTATACGGTGGGGTTCTACTACTACGGTATGGACGTCTGGGGCCAAGGGACCACGGTCACCGTCTCCTCA |
| | | | SEQ ID NO: 27419 | SEQ ID NO: 31425 |
| | | AA | SYELTQPPSVSVSPGQTASITCSGDKLGDKYACWYQQKPGQSPVLVIYQDSKRPSGIPERFSGSNSGNTATLTISGTQAMDEADYYCQAWDSTTLVFGGGTKLTVL | QVQLQESGPGLVKPSQTLSLTCTVSGGSISSGGYYWSWIRQHPGKGLEWIGYIYYSGSPYYNPSLKSRITISIDTSKNQFSLKLNSVTAADTAVYYCARSNCSSANCYTVGFYYYGMDVWGQGTTVTVSS |
| | | | SEQ ID NO: 27420 | SEQ ID NO: 31426 |

FIGURE 50

| | | | | |
|---|---|---|---|---|
| **iPS:436782** | **21-225_166G11** | NA | TCCTATGAGCTGAGTCAGCCACCCTCAGTGTCCGTGTCCCCAGGACAGACAGCCAGCATCACCTGCTCTGGAGATAAATTGGGGGATAAATATGTTCACTGGTATCAGCAGAAGCCAGGCCAGTCCCCTGTGTTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGATTATTACTGTCAGGCGTGGGACAACAGCACTGCGGTATTCGGCGGAGGGACCAAGTTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAATGGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACACTTCCAAGAACACGCTGTTTCTGCAAATGAACAGCCTGACAGCCGAGGACACGGCTGTGTATTACTGTGCGAGAGATGATAGATATTGTAGTAGTCCCACCTGCCATCCTTACTACTACTACTACGGTCTGGACGTCTGGGGCCAAGGGACCACGGTCACCGTCTCCTCA |
| | | | SEQ ID NO: 27421 | SEQ ID NO: 31427 |
| | | AA | SYELSQPPSVSVSPGQTASITCSGDKLGDKYVHWYQQKPGQSPVLVIYQDSKRPSGIPERFSGSNSGNTATLTISGTQAMDEADYYCQAWDNSTAVFGGGTKLTVL | QVQLVESGGGVVQPGRSLRLSCAASGFTFNGYGMHWVRQAPGKGLEWVAVIWYDGSNKYYADSVKGRFTISRDTSKNTLFLQMNSLTAEDTAVYYCARDDRYCSSPTCHPYYYYYGLDVWGQGTTVTVSS |
| | | | SEQ ID NO: 27422 | SEQ ID NO: 31428 |

FIGURE 50

| | | | | |
|---|---|---|---|---|
| **iPS:436784** | **21-225_169C1** | NA | TCCTATGAGCTGACTCAGCCACCCTCAGTGTCCGTGTCCCCAGGACAGACAGCCAGCATCACCTGCTCTGGAGATAAATTGGGGGATAAATATGTTTGTTGGTATCAGCAGAAGCCAGGCCAGTCCCCTTTGCTGGTCATCTATAAAGATATCAAGCGGCCCTCAGGGATCCCTGAGCGATTCTCTGGCTCCAACTCTGGTAACACAGCCACTCTGACCATCAGCGGGACCCAGGCTATGGATGAGGCTGACTATTACTGTCAGGCGTGGGACACCAACACTGTGATATTCGGCGGAGGGACCAAGCTGACCGTCCTA | CAGGTGCAGTTGGTGGAGTCTGGGGGAGGCGTGGTCCAGCCTGGGAGGTCCCTGAGACTCTCCTGTGCAGCGTCTGGATTCACTTTGAGTAGCAATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACACTTCCAAGAACACGCTGTATCTGCAAATGAACAGCCTGAGAGCCGAGGACACGGCTGTGTATTACTGTGCGAGAGATCAGTACAACCGGAACGACGGACCACCAGCTTACTACTACTACTACGGTTTGGACGTCTGGGGCCAAGGGACCACGGTCACCGTCTCCTCA |
| | | | SEQ ID NO: 27423 | SEQ ID NO: 31429 |
| | | AA | SYELTQPPSVSVSPGQTASITCSGDKLGDKYVCWYQQKPGQSPLLVIYKDIKRPSGIPERFSGSNSGNTATLTISGTQAMDEADYYCQAWDTNTVIFGGGTKLTVL | QVQLVESGGGVVQPGRSLRLSCAASGFTLSSNGMHWVRQAPGKGLEWVAVIWYDGSNKYYADSVKGRFTISRDTSKNTLYLQMNSLRAEDTAVYYCARDQYNRNDGPPAYYYYYGLDVWGQGTTVTVSS |
| | | | SEQ ID NO: 27424 | SEQ ID NO: 31430 |

FIGURE 50

| | | | | |
|---|---|---|---|---|
| **iPS:436786** | **21-225_169A6** | NA | TCCTATGAGCTGACTCAGCCACCCTCAGTGTCCGTGTCCCCAGGACAGACAGCCAGCATCACCTGCTCTGGAGATAAATTGGGGGATAAATATGTTTGTTGGTATCAGCGGAAGCCAGGCCAGTCCCCTGTTCTGGTCATCTATCAGGATTACAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACACCAACACTGTGCTTTTCGGCGGAGGGACCAAGCTGACCGTCCTG | CAGGTGCAGCTGGTGGAGTCTGGGGGAGGCGTGGTCCAGCCTGGGAGGTCCCTGAGACTCTCCTGTGCAGCGTCTGGATTCACTTTGAGCAGCAATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTCTCTGCAAATGAACAGCCTGAGAGCCGAGGACACGGCTGTGTATTACTGTGCGAGAGATCAGTACAACCGGAACGACGGACCACCAGCTTACTACTACTACTACGGTATGGACGTCTGGGGCCAAGGGACCACGGTCACCGTCTCCTCA |
| | | | SEQ ID NO: 27425 | SEQ ID NO: 31431 |
| | | AA | SYELTQPPSVSVSPGQTASITCSGDKLGDKYVCWYQRKPGQSPVLVIYQDYKRPSGIPERFSGSNSGNTATLTISGTQAMDEADYYCQAWDTNTVLFGGGTKLTVL | QVQLVESGGGVVQPGRSLRLSCAASGFTLSSNGMHWVRQAPGKGLEWVAVIWYDGSNKYYADSVKGRFTISRDNSKNTLSLQMNSLRAEDTAVYYCARDQYNRNDGPPAYYYYYGMDVWGQGTTVTVSS |
| | | | SEQ ID NO: 27426 | SEQ ID NO: 31432 |

FIGURE 50

| | | | | |
|---|---|---|---|---|
| **iPS:436788** | **21-225_169B7** | NA | GCCTATGACCTGACTCAGCCACCCTCAGTGTCCGTGTCCCCAGGACAGACAGCCAGAATCACCTGCTCTGGAGATAAATTGGGGGGAAAATATGCTTCCTGGTATCAACAGAAGCCAGGCCAGTCCCCTGTGCTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAAGAACACTGTGGTATTCGGCGGAGGGACCAAGCTGACCGTCCTA | GAGGTGCAGCTGGTGGAGTCTGGGGGAGGCTTGGTACAGCCTGGGGGGTCCCTGAGACTCTCCTGTGCAGCCTCTGGATTCACCTTCAGTAGCTATAGCTTGAACTGGGTCCGCCAGGCTCCAGGGAAGGGGCTGGAGTGGGTTTCATACATTGGTAGTAGTGGCAGTATCATATTCTACGCAGACTCTGTGAAGGGCCGATTCACCATCTCCAGAGACAATGCCAAGAACTCACTGTATCTGCAAATGAACAGCCTGAGAGACGAGGACACGGCTGTGTATTACTGTGCGAGAGGGGATACAGCTGGGGTTACCTATTACTACGGTATGGACGTCTGGGGCCAAGGGACCACGGTCACCGTCTCCTCA |
| | | | SEQ ID NO: 27427 | SEQ ID NO: 31433 |
| | | AA | AYDLTQPPSVSVSPGQTARITCSGDKLGGKYASWYQQKPGQSPVLVIYQDRKRPSGIPERFSGSNSGNTATLTISGTQAMDEADYYCQAWDKNTVVFGGGTKLTVL | EVQLVESGGGLVQPGGSLRLSCAASGFTFSSYSLNWVRQAPGKGLEWVSYIGSSGSIIFYADSVKGRFTISRDNAKNSLYLQMNSLRDEDTAVYYCARGDTAGVTYYYGMDVWGQGTTVTVSS |
| | | | SEQ ID NO: 27428 | SEQ ID NO: 31434 |
| **iPS:436790** | **21-225_169G11** | NA | TCCTATGAGCTGACTCAGCCACCCTCAGTGTCCGTGTCCCCAGGACAGACAGCCAGCATCACCTGCTCTGGAGATAAATTGGGGGATAAATATGCTTGCTGGTATCAGCAGAAGCCAGGCCAGTCCCCTGTGCTGGTCATCTATCAAGATAGTAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAACAGCACTGCGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAATTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGGTGTGTATTACTGTGCGAGAGAGGGGGCTACGTATTACCATGGTTCGGGGAGTTATTATCCGGCTACTAACTACGGTATGGACGTCTGGGGCCAAGGGACCACGGTCACCGTCTCCTCA |

FIGURE 50

| | | | | |
|---|---|---|---|---|
| | | | SEQ ID NO: 27429 | SEQ ID NO: 31435 |
| | | AA | SYELTQPPSVSVSPGQTASITCSGDKLGDKYACW<br>YQQKPGQSPVLVIYQDSKRPSGIPERFSGSNSGN<br>TATLTISGTQAMDEADYYCQAWDNSTAVFGGG<br>TKLTVL | QVQLVESGGGVVQPGRSLRLSCAASGFTFSSYGMH<br>WVRQAPGKGLEWVAIIWYDGSNKYYADSVKGRFT<br>ISRDNSKNTLYLQMNSLRAEDTGVYYCAREGATY<br>YHGSGSYYPATNYGMDVWGQGTTVTVSS |
| | | | SEQ ID NO: 27430 | SEQ ID NO: 31436 |
| **iPS:436792** | **21-225_169D12** | NA | AATTTTATGCTGACTCAGCCCCACTCTGTGTCG<br>GAGTCTCCGGGGAAGACGGTAACCATCTCCTG<br>CACCCGCAGCAGTGGCAGCATTACCGGCAACT<br>ATGTGCAGTGGCACCAGCAGCGCCCGGGCAAT<br>TCCCCCACCACTTTGATCTATGAGGATAAAAA<br>AAGACCCTCTGGGGTCCCTGATCGGTTCTCTG<br>GCTCCATCGACAGCTCCTCCAACTCTGCCTCC<br>CTCACCATCTCTGGACTGAAGACTGAGGACGA<br>GGCTGACTATTACTGTCAGTCTTATTATAGCG<br>GCAATTGGGTGTTCGGCGGAGGGACCAAGCTG<br>ACCGTCCTG | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTAGCTATGGCAT<br>GCACTGGGTCCGCCAGGCTCCAGGCAAGGGGCT<br>GGAGTGGGTGGCAGTTATATGGTATGATGGAAGT<br>AATAAATATTATGCAGACTCCGTGAAGGGCCGAT<br>TCACCATCTCCAGAGACAATTCCAAGAACACGCT<br>GTATCTGCAAATGAACAGCCTGAGAGCCGAGGA<br>CACGGCTGTGTATTACTGTGCGAGTCCCCTTTAC<br>GATATGGGACTCTACTACGATATGGACGTCTGGG<br>GCCAAGGGACCACGGTCACCGTCTCCTCA |
| | | | SEQ ID NO: 27431 | SEQ ID NO: 31437 |
| | | AA | NFMLTQPHSVSESPGKTVTISCTRSSGSITGNYVQ<br>WHQQRPGNSPTTLIYEDKKRPSGVPDRFSGSIDS<br>SSNSASLTISGLKTEDEADYYCQSYYSGNWVFG<br>GGTKLTVL | QVQLVESGGGVVQPGRSLRLSCAASGFTFSSYGMH<br>WVRQAPGKGLEWVAVIWYDGSNKYYADSVKGRF<br>TISRDNSKNTLYLQMNSLRAEDTAVYYCASPLYDM<br>GLYYDMDVWGQGTTVTVSS |
| | | | SEQ ID NO: 27432 | SEQ ID NO: 31438 |

FIGURE 50

| | | | | |
|---|---|---|---|---|
| **iPS:436794** | **21-225_170F1** | NA | TCCTATGAGTTGAGTCAGCCACCCTCAGTGTC<br>CGTGTCCCCAGGACAGACAGCCAGCATCACCT<br>GCTCTGGAGATAAATTGGGGGATAAATATTCT<br>TGCTGGTATCAGCAGAAGCCAGGCCAGTCCCC<br>TGTGCTGGTCATCTATCAAGATAGTAAGCGGC<br>CCTCAGGGATCCCTGCACGATTCTCTGGCTCC<br>AACTCTGGGAACACAGCCACTCTGACCATCAG<br>CGGGACCCAGGCTATGGATGAGGCTGATTATT<br>ACTGTCAGGCGTGGGACAGCAACACTGCGGTA<br>TTCGGCGGAGGGACCAAACTGACCGTCCTA | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTGGCTATGGCAT<br>GAACTGGGTCCGCCAGGCTCCAGGCAAGGGGCT<br>GGAGTGGGTGGCAATTATATGGTATGATGGAAAT<br>AATAAATACTATGCAGACTCCGTGAAGGGCCGA<br>TTCACCATCTCCAGAGACAATTCCAAGAACACGC<br>TGTATCTGCAAATGAACAGCCTGAGAGCCGAGG<br>ACACGGCTGTGTATTACTGTGCGAGAGATCGGGT<br>TTATTGTAGTAGTACCAGCTGCCATCCCTATTACT<br>ACTACTACGCTATGGACGTCTGGGGCCAAGGGA<br>CCACGGTCACCGTCTCCTCA |
| | | | SEQ ID NO: 27433 | SEQ ID NO: 31439 |
| | | AA | SYELSQPPSVSVSPGQTASITCSGDKLGDKYSCW<br>YQQKPGQSPVLVIYQDSKRPSGIPARFSGSNSGN<br>TATLTISGTQAMDEADYYCQAWDSNTAVFGGG<br>TKLTVL | QVQLVESGGGVVQPGRSLRLSCAASGFTFSGYGM<br>NWVRQAPGKGLEWVAIIWYDGNNKYYADSVKGR<br>FTISRDNSKNTLYLQMNSLRAEDTAVYYCARDRVY<br>CSSTSCHPYYYYYAMDVWGQGTTVTVSS |
| | | | SEQ ID NO: 27434 | SEQ ID NO: 31440 |

FIGURE 50

| | | | | |
|---|---|---|---|---|
| **iPS:436796** | **21-225_170A5** | NA | TCCTATGACCTGACTCAGCCACCCTCAGTGTCCGTGTCCCCAGGACAGACAGCCAGCATCACCTGCTCTGGAGATAAATTGGGGGATAAATATGCTTGTTGGTATCAGCAGAAGCCAGGCCAGTCCCCTATACTGGTCATCTATCAAGATTACAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTATTGTCAGGCGTGGGACAACAGCACTATGGTATTCGGCGGAGGGACCAGGCTGACCGTCCTA | CAGGTGCAGCTGGTGGAGTCTGGGGGAGGCGTGGTCCAGCCTGGGAGGTCCCTGAGACTCTCCTGTGCAGCGTCTGGACTCACCTTCAGTAACTGTGGCATGCACTGGGTCCGCCAGGCTCCAGGCAAGGGGCTGGAGTGGGTGGCAATTATATGGTATGATGGAAGTAATAAATACTATGCAGACTCCGTGAAGGGCCGATTCACCATCTCCAGAGACAATTCCAAGAACACGCTGGATCTGCAAATGAACAGCCTGAGAGCCGAGGACACGGCTGTGTATTACTGTGCGAGAGATCAGTACAACAGGAACGACGGACCACCAGCTTACTACTACTACTACGGTTTGGACGTCTGGGGCCAAGGGACCACGGTCACCGTCTCCTCA |
| | | | SEQ ID NO: 27435 | SEQ ID NO: 31441 |
| | | AA | SYDLTQPPSVSVSPGQTASITCSGDKLGDKYACWYQQKPGQSPILVIYQDYKRPSGIPERFSGSNSGNTATLTISGTQAMDEADYYCQAWDNSTMVFGGGTRLTVL | QVQLVESGGGVVQPGRSLRLSCAASGLTFSNCGMHWVRQAPGKGLEWVAIIWYDGSNKYYADSVKGRFTISRDNSKNTLDLQMNSLRAEDTAVYYCARDQYNRNDGPPAYYYYYGLDVWGQGTTVTVSS |
| | | | SEQ ID NO: 27436 | SEQ ID NO: 31442 |

FIGURE 50

| | | | | |
|---|---|---|---|---|
| **iPS:436798** | **21-225_171F5** | NA | GCCTATGACCTGACTCAGCCACCCTCAGTGTCCGTGTCCCCAGGACAGACAGCCAGCATCACCTGCTCTGGAGATAAATTGGGGGGAAAATATGCTTCCTGGTATCAACAGAAGCCAGGCCAGTCCCCTGTGCTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAAGAACACTGTGGTATTCGGCGGAGGGACCAAGCTGACCGTCCTA | GAGGTGCAGCTGGTGGAGTCTGGGGGAGGCTTGGTACAGCCTGGGGGGTCCCTGAGACTCTCCTGTGCAGCCTCTGGATTCACCTTCAGTAGCTATAGCTTGAACTGGGTCCGCCAGGCTCCAGGGAAGGGGCTGGAGTGGGTTTCATACATTGGTAGTAGTGGCAGTATCATATTCTACGCAGACTCTGTGAAGGGCCGATTCACCATCTCCAGAGACAATGCCAAGAACTCACTGTATCTGCAAATGAACAGCCTGAGAGACGAGGACACGGCTGTGTATTACTGTGCGAGAGGGGATACAGCTGGGGTTACCTATTACTACGGTATGGACGTCTGGGGCCAAGGGACCACGGTCACCGTCTCCTCA |
| | | | SEQ ID NO: 27437 | SEQ ID NO: 31443 |
| | | AA | AYDLTQPPSVSVSPGQTASITCSGDKLGGKYASWYQQKPGQSPVLVIYQDRKRPSGIPERFSGSNSGNTATLTISGTQAMDEADYYCQAWDKNTVVFGGGTKLTVL | EVQLVESGGGLVQPGGSLRLSCAASGFTFSSYSLNWVRQAPGKGLEWVSYIGSSGSIIFYADSVKGRFTISRDNAKNSLYLQMNSLRDEDTAVYYCARGDTAGVTYYYGMDVWGQGTTVTVSS |
| | | | SEQ ID NO: 27438 | SEQ ID NO: 31444 |
| **iPS:436800** | **21-225_171D12** | NA | TCTTCTGAGCTGACTCAGGACCCTGCTGTGTCTGTGGCCTTGGGACAGACAGTCAGGATCACATGCCAAGGAGACAGCCTCAGAAGCTATTATGCAAGCTGGTACCAGCAGAAGCCAGGACAGGCCCCTATACTTGTCATCTATGCTAAAAACAACCGGCCCTCAGGGATCCCAGACCGATTCTCTGGCTCCAACTCAGGAAACACAGCTTCCTTGACCATCACTGGGGCTCAGGCGGAAGATGAGGCTGACTATTACTGTAACTCCCGGGACAGCAGTGGCAGCCATGTGGTATTCGGCGGAGGGACCAAACTGACCGTCCTA | CAGGTGCAGCTGGTGCAGTCTGGGGCTGAGGTGAAGAAGCCTGGGGCCTCAGTGAAGGTTTCCTGCAAGGCATCTGGATACACCTTCAACAGTTACTATATGTATTGGGTGCGACAGGCCCCTGGACAAGGGCTTGAGTGGATGGGAATAATCAACCCTAGTGGTGGTAGCACAAACTACGCACAGAAGTTCCAGGGCAGAGTCACCATGACCAGGGACACGTCCACGAGCACACTCTACATGGAGCTGAACAGCCTGAGATCTGAGGACACGGCCGTGTATTACTGTGCGAGTGGTTGGGAGTTAAACTACTGGGGCCAGGGAACCCTGGTCACCGTCTCCTCA |
| | | | SEQ ID NO: 27439 | SEQ ID NO: 31445 |

FIGURE 50

| | | | | |
|---|---|---|---|---|
| | | AA | SSELTQDPAVSVALGQTVRITCQGDSLRSYYASWYQQKPGQAPILVIYAKNNRPSGIPDRFSGSNSGNTASLTITGAQAEDEADYYCNSRDSSGSHVVFGGGTKLTVL | QVQLVQSGAEVKKPGASVKVSCKASGYTFNSYYMYWVRQAPGQGLEWMGIINPSGGSTNYAQKFQGRVTMTRDTSTSTLYMELNSLRSEDTAVYYCASGWELNYWGQGTLVTVSS |
| | | | SEQ ID NO: 27440 | SEQ ID NO: 31446 |
| **iPS:436802** | **21-225_171E12** | NA | TCCTATGAGCTGACTCAGCCACCCTCAGTGTCCGTGTCCCCAGGACAGACAGCCAGCATCACCTGCTCTGGAGATAAATTGGGGGATAAATATGCTTGCTGGTATCAGCAGAAGCCAGGCCAGTCCCCTGTGCTGGTCATCTATCAAGATCGCAAGCGGCCCTCAGGGATCCCTGAGCGATTCTCTGGCTCCAACTCTGGGAACACAGCCACCCTGACCATCAGCGGGACCCAGGCTATGGATGAGGCTGACTATTACTGTCAGGCGTGGGACATCAGCACTTATGTGGTATTCGGCGGAGGGACCAAACTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGTTATGGCATGCACTGGGTCCGCCAGGCTCCAGGCAAGGGGCTGGAGTGGGTGGCAGTTATATGGAATGATGGAGGTAATAAATATAATGGAGACTCCGTGAAGGGCCGATTCACCATCTCCAGAGACAATTCCAAGAACACGCTGTATCTGCAAATGAACAGTCTGAGAGCCGAGGACACGGCTGTGTATTACTGTGCGAGAGACCGTACGTATTACTCTGGTTCGGGGAGCCCCCCCTACTACTACTACTACGGTATGGACGTCTGGGGCCAAGGGACCACGGTCACCGTCTCCTCA |
| | | | SEQ ID NO: 27441 | SEQ ID NO: 31447 |
| | | AA | SYELTQPPSVSVSPGQTASITCSGDKLGDKYACWYQQKPGQSPVLVIYQDRKRPSGIPERFSGSNSGNTATLTISGTQAMDEADYYCQAWDISTYVVFGGGTKLTVL | QVQLVESGGGVVQPGRSLRLSCAASGFTFSSYGMHWVRQAPGKGLEWVAVIWNDGGNKYNGDSVKGRFTISRDNSKNTLYLQMNSLRAEDTAVYYCARDRTYYSGSGSPPYYYYYGMDVWGQGTTVTVSS |
| | | | SEQ ID NO: 27442 | SEQ ID NO: 31448 |

FIGURE 50

| | | | | |
|---|---|---|---|---|
| **iPS:436804** | **21-225_172C3** | NA | TCTTCTGAGCTGACTCAGGACCCTGCTGTGTCTGTGGCCTTGGGACAGACAGTCAGGATCACATGCCAAGGAGACAGCCTCAGAAACTATTATGTAAGCTGGTACCAGCAGAAGCCAGGACAGGCCCCTATACTTGTCATCTATACTAAAAACAGCCGGCCCTCAGGGATCCCAGACCGATTCTCTGGCTCCACCTCAGGAAACACAGCTTCCTTGACCATCACTGGGACTCAGGCGGAAGATGAGGCTGACTATTACTGTAACTCCCGGGACAGCAGTGGCAACCATGTGGTATTCGGCGGAGGGACCAAGCTGACCGTCCTA | CAGGTGCAGCTGGTGCAGTCTGGGGCTGAGGTGAAGAAGCCTGGGGCCTCAGTGAAGGTTTCCTGCAAGGCATCTGGATACACCTTCAGAAGTTACTATATGTATTGGGTGCGACAGGCCCCTGGACAAGGGCTTGAGTGGGTGGGAACAATCAACCCTAGTGGTGGTAGCACAAACTACGCACAGAAGTTCCAGGGCAGAGTCACCATGACCAGGGACACGTCCACGAGCACACTCTACATGGAGCTGAACAGCCTGAGATCTGAGGACACGGCCGTGTATTACTGTGCGAGTGGCTGGGAGTTAAACTACTGGGGCCAGGGAACCCTGGTCACCGTCTCCTCA |
| | | | SEQ ID NO: 27443 | SEQ ID NO: 31449 |
| | | AA | SSELTQDPAVSVALGQTVRITCQGDSLRNYYVSWYQQKPGQAPILVIYTKNSRPSGIPDRFSGSTSGNTASLTITGTQAEDEADYYCNSRDSSGNHVVFGGGTKLTVL | QVQLVQSGAEVKKPGASVKVSCKASGYTFRSYYMYWVRQAPGQGLEWVGTINPSGGSTNYAQKFQGRVTMTRDTSTSTLYMELNSLRSEDTAVYYCASGWELNYWGQGTLVTVSS |
| | | | SEQ ID NO: 27444 | SEQ ID NO: 31450 |
| **iPS:436806** | **21-225_172B12** | NA | TCTTCTGAGCTGACTCAGGACCCTGCTGTGTCTGTGGCCTTGGGACAGACAGTCAGGATCACATGCCAAGGAGACAGCCTCAGAAACTATTATGCAAGCTGGTACCAGCAGAAGCCAGGACAGGCCCCTATACTTGTCATCTATACTAAAAACAGCCGGCCCTCAGGGATCCCAGACCGATTCTCTGGCTCCACCTCAGGAAACACAGCTTCCTTGACCATCACTGGGGCTCAGGCGGAAGATGAGGCTGACTATTACTGTAACTCCCGGGACAGCAGTGGCAACCATGTGGTATTCGGCGGAGGGACCAAGCTGACCGTCCTA | CAGGTGCAGCTGGTGCAGTCTGGGGCTGAGGTGAAGAAGCCTGGGGCCTCAGTGACGGTTTCCTGCAAGGCATCTGGATACACCTTCAGAAGTTACTATATGTATTGGGTGCGACAGGCCCCTGGACAAGGGCTTGAGTGGGTGGGAACAATCAACCCTAGTGGTGGTAGCACAGACTACGCACAGAAGTTCCAGGGCAGAGTCACCATGACCAGGGACACGTCCACGAGCACACTCTACATGGAGCTGAACAGCCTGAGATCTGAGGACACGGCCGTGTATTACTGTGCGAGTGGCTGGGAATTAAACTACTGGGGCCAGGGAACCCTGGTCACCGTCTCCTCA |
| | | | SEQ ID NO: 27445 | SEQ ID NO: 31451 |

FIGURE 50

| | | | | |
|---|---|---|---|---|
| | | AA | SSELTQDPAVSVALGQTVRITCQGDSLRNYYAS WYQQKPGQAPILVIYTKNSRPSGIPDRFSGSTSG NTASLTITGAQAEDEADYYCNSRDSSGNHVVFG GGTKLTVL | QVQLVQSGAEVKKPGASVTVSCKASGYTFRSYYM YWVRQAPGQGLEWVGTINPSGGSTDYAQKFQGRV TMTRDTSTSTLYMELNSLRSEDTAVYYCASGWELN YWGQGTLVTVSS |
| | | | SEQ ID NO: 27446 | SEQ ID NO: 31452 |
| **iPS:436808** | **21-225_173F8** | NA | TCCTATGAGCTGACTCAGCCACCCTCAGTGTC CGTGTCCCCAGGACAGACAGCCAGCATCACCT GCTCTGGAAATAAATTGGGGAATAAATATGTT TGCTGGTATCAGCAGAGGCCAGGCCAGTCCCC TGTGCTGGTCATCTCTCAAGATAGCAGGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCTTCACTGTGGTA TTCGGCGGAGGGACCAAGCTGACCGTCCTA | CAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGGCAT GCACTGGGTCCGCCAGGCTCCAGGCAAGGGGCT GGAATGGGTGGCAGTTATATCATATGATGGAAGT CCTAAATACTGTGCAGACTCCGTGAAGGGCCGAT TCACCATCTCCAGAGACAATTCCAAGAACACGCT GTTTCTCCAAATGAACAGCCTGAGAGCTGAGGAC ACGGCTGTGTATTATTGTGCGAGAGATGAAAGGC AGTGGCTGCCGGCCCCCTACGGTATGGACGTCTG GGGCCAAGGGACCACGGTCACCGTCTCCTCA |
| | | | SEQ ID NO: 27447 | SEQ ID NO: 31453 |
| | | AA | SYELTQPPSVSVSPGQTASITCSGNKLGNKYVCW YQQRPGQSPVLVISQDSRRPSGIPERFSGSNSGNT ATLTISGTQAMDEADYYCQAWDSFTVVFGGGT KLTVL | QVQLVESGGGVVQPGRSLRLSCAASGFTFSSYGMH WVRQAPGKGLEWVAVISYDGSPKYCADSVKGRFT ISRDNSKNTLFLQMNSLRAEDTAVYYCARDERQW LPAPYGMDVWGQGTTVTVSS |
| | | | SEQ ID NO: 27448 | SEQ ID NO: 31454 |

FIGURE 50

| | | | | |
|---|---|---|---|---|
| iPS:436810 | 21-225_175F4 | NA | CAGTCTGCCCTGACTCAGCCTGCCTCCGTGTCTGGGTCTCCTGGACAGTCGATCACCATCTCCTGCACTGGAACCAGCAGTGATGTTGGACGTTTTAACCTTGTCTCCTGGTACCAACAGCACCCAGGCTACGCCCCCAAACTCATGATTTATGAGGTCAGTAAGCGGCCCTCAGGGGTTTCTAATCGCTTCTCTGGCTCCAAGTCTGGCAACACGGCCTCCCTGACAATCTCTGGGCTCCAGGCTGAGGACGAGGCTGATTATTACTGCTGCTCATATGCAGGTAGTAGCACCTATGTGGTATTCGGCGGAGGGACCAAGCTGACCGTCCTG | CAGGTACAGCTGCAGCAGTCAGGTCCAGGACTGGTGAAGCCCTCGCAGACCCTCTCACTCACCTGTGCCATCTCCGGGGACAGTGTCTCTAGCAACAGTGCTGCTTGGAACTGGATCAGGCAGTCCCCATCGAGAGGCCTTGAGTGGCTGGGAAGGACATACTACAGGTCCAAGTGGTATAATGCTTATCCAGTATCTATGGAAAGTCGAATATCCATCAACCCAGACACATCCAAGAACCAGTTCTCCCTGCAGCTGAACTCTGTGACTCCCGAGGACACGGCTGTTTATTACTGTGCAAGAGATAAGGCAGCTGGGAGGAATGACTTCTACTACTACGGTATGGACGTCTGGGGCCAAGGGACCACGGTCACCGTCTCCTCA |
| | | | SEQ ID NO: 27449 | SEQ ID NO: 31455 |
| | | AA | QSALTQPASVSGSPGQSITISCTGTSSDVGRFNLVSWYQQHPGYAPKLMIYEVSKRPSGVSNRFSGSKSGNTASLTISGLQAEDEADYYCCSYAGSSTYVVFGGGTKLTVL | QVQLQQSGPGLVKPSQTLSLTCAISGDSVSSNSAAWNWIRQSPSRGLEWLGRTYYRSKWYNAYPVSMESRISINPDTSKNQFSLQLNSVTPEDTAVYYCARDKAAGRNDFYYYGMDVWGQGTTVTVSS |
| | | | SEQ ID NO: 27450 | SEQ ID NO: 31456 |
| iPS:436812 | 21-225_175C6 | NA | TCCTATGACCTGACTCAGCCACCCTCAGTGTCCGTGTCCCCAGGACAGACAGCCAGCATCACCTGCTCTGGAGATAAATTGGGGGATAAATATGCTTGTTGGTATCAGCAGAAGCCAGGCCAGTCCCCTATACTGGTCATCTATCAAGATTACAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTATTGTCAGGCGTGGGACAACAGCACTATGGTATTCGGCGGAGGGACCAGGCTGACCGTCCTA | CAGGTGCAGCTGGTGGAGTCTGGGGGAGGCGTGGTCCAGCCTGGGAGGTCCCTGAGACTCTCCTGTGCAGCGTCTGGACTCACCTTCAGTAACTGTGGCATGCACTGGGTCCGCCAGGCTCCAGGCAAGGGGCTGGAGTGGGTGGCAATTATATGGTATGATGGAAGTAATAAATACTATGCAGACTCCGTGAAGGGCCGATTCACCGTCTCCAGAGACAATTCCAAGAACACGCTGGATCTGCAAATGAACAGCCTGAGAGCCGAGGACACGGCTGTGTATTACTGTGCGAGAGATCAGTACAACAGGAACGACGGACCACCAGCTTACTACTACTACTACGGTTTGGACGTCTGGGGCCAAGGGACCACGGTCACCGTCTCCTCA |

FIGURE 50

| | | | | |
|---|---|---|---|---|
| | | | SEQ ID NO: 27451 | SEQ ID NO: 31457 |
| | | AA | SYDLTQPPSVSVSPGQTASITCSGDKLGDKYACWYQQKPGQSPILVIYQDYKRPSGIPERFSGSNSGNTATLTISGTQAMDEADYYCQAWDNSTMVFGGGTRLTVL | QVQLVESGGGVVQPGRSLRLSCAASGLTFSNCGMHWVRQAPGKGLEWVAIIWYDGSNKYYADSVKGRFTVSRDNSKNTLDLQMNSLRAEDTAVYYCARDQYNRNDGPPAYYYYYGLDVWGQGTTVTVSS |
| | | | SEQ ID NO: 27452 | SEQ ID NO: 31458 |
| **iPS:436814** | **21-225_178H10** | NA | CAGTCTGCCCTGACTCAGCCTGCCTCCGTGTCTGGGTCTCCTGGACAGTCGATCACCATCTCCTGCACTGGAACCAGCAGTGATGTTGGACGTTTTAACCTTGTCTCCTGGTACCAACACCACCCAGGCAACGCCCCCAAACTCATGATTTATGAAGTCAGTAAGCGGCCCTCAGGGGTTTCTAATCGCTTCTCTGGCTCCAAGTCTGGCAACACGGCCTCCCTGACAATCTCTGGGCTCCAGGCTGAGGACGAGGCTGATTATTACTGCTGCTCATATGCAGGTAGTAGCACCTTTGTAGTATTCGGCGGAGGGACCAAGCTGACCGTCCTA | CAGGTACAGCTGCAGCAGTCAGGTCCAGGACTGGTGAAGCCCTCGCAGACCCTCTCACTCACCTGTGCCATCTCCGGGGACAGTGTCTCTAGCAACAGTGCTGCTTGGAACTGGATCAGGCAGTCCCCATCGAGAGGCCTTGAGTGGCTGGGAAGGACATACTACAGGTCCAAGTGGTATAGTGCTTATCCAGTATCTATGGAAAGTCGAGTATCCATCAACCCAGACACATCCAAGAACCAGTTCTCCCTGCAGCTGAACTCTGTGACTCCCGAGGACACGGCTGTTTATTACTGTGCAAGAGATAAGGCAGCTGGGAGGAATGACTTCTACTACTACGGTATGGACGTCTGGGGCCAAGGGACCACGGTCACCGTCTCCTCA |
| | | | SEQ ID NO: 27453 | SEQ ID NO: 31459 |
| | | AA | QSALTQPASVSGSPGQSITISCTGTSSDVGRFNLVSWYQHHPGNAPKLMIYEVSKRPSGVSNRFSGSKSGNTASLTISGLQAEDEADYYCCSYAGSSTFVVFGGGTKLTVL | QVQLQQSGPGLVKPSQTLSLTCAISGDSVSSNSAAWNWIRQSPSRGLEWLGRTYYRSKWYSAYPVSMESRVSINPDTSKNQFSLQLNSVTPEDTAVYYCARDKAAGRNDFYYYGMDVWGQGTTVTVSS |
| | | | SEQ ID NO: 27454 | SEQ ID NO: 31460 |

FIGURE 50

| | | | | |
|---|---|---|---|---|
| **iPS:436816** | **21-225_179H5** | NA | TCTTCTGAGCTGACTCAGGACCCTGCTGTGTCTGTGGCCTTGGGACAGACAGTCAGGATCACATGCCAAGGAGACAGTCTCAGAAACTATTATGCAAGCTGGTACCAGCAGAAGCCAGGACAGGCCCCTGTATTTGTCATCTATGGTAAAAACAACCGGCCCTCAGGGATCCCAGACCGATTCTCTGGCTCCAGGTCAGGAAACACAGCTTCCTTGACCATCACTGGGGCTCAGGCGGAAGATGAGGCTGACTATTACTGTAACTCCCGGGACAGCAGTGGTAACCATTGGGTGTTCGGCGGAGGGACCAAACTGACCGTCCTA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATGAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATATCCGGAACTACTACTACGGTTTGGACGTCTGGGGCCAAGGGACCACGGTCACCGTCTCCTCA |
| | | | SEQ ID NO: 27455 | SEQ ID NO: 31461 |
| | | AA | SSELTQDPAVSVALGQTVRITCQGDSLRNYYASWYQQKPGQAPVFVIYGKNNRPSGIPDRFSGSRSGNTASLTITGAQAEDEADYYCNSRDSSGNHWVFGGGTKLTVL | QVQLVESGGGVVQPGRSLRLSCAASGFTFSSYGMHWVRQAPGKGLEWVAVIWYDGSNEYYADSVKGRFTISRDNSKNTLYLQMNSLRAEDTAVYYCARDIRNYYYGLDVWGQGTTVTVSS |
| | | | SEQ ID NO: 27456 | SEQ ID NO: 31462 |
| **iPS:436818** | **21-225_179C7** | NA | TCCTATGAGCTGACTCAGCCACCCTCAGTGTCCGTGTCCCCAGGACAGACAGCCAGTATCACCTGCTCTGGAGATAAATTGGGGGATAAATATGTTTGCTGGTATCAACAGAGGCCGGGCCAGTCCCCTGTGCTGGTCATCTATCAGGATAGTAAGCGGCCCTCAGGGATCCCTGAGCGATTCTCTGGCTCCAACTCTGGGAACACAGCCACTCTGACCATCCGCGGGACCCAGGCTATGGATGAGGCTGACTATTACTGTCAGGCGTGGGACAGCAACACTGCAGTGGTATTCGGCGGAGGGACCAAGCTGACCGTCCTA | CAGGCGCAGCTGGTGGAGTCTGGGGGAGGCGTGGTCCAGCCTGGGAGGTCCCTGAGACTCTCCTGTGCAGCGTCTGGATTCACCTTCAGTAACTCTGGCATGCACTGGGTCCGCCAGGGTCCAGGCAAGGGGCTGGAGTGGGTGGCGATTATATATTATGATGGAAGTTATAAATACAATGCAGACTCCGTGAAGGGCCGATTCACCATCTCCAGAGACAATTCCAAGAACACGCTGTATCTGCAAATGAACAGCCTGAGAGCCGAGGACACGGCTGTATATTACTGTGCGAGAGACCGTCATTACGATTTCCACGTTCCCTACTATTACTATTACGGTATGGACGTCTGGGGCCAAGGGACCACGGTCACCGTCTCCTCA |
| | | | SEQ ID NO: 27457 | SEQ ID NO: 31463 |

FIGURE 50

| | | | | |
|---|---|---|---|---|
| | | AA | SYELTQPPSVSVSPGQTASITCSGDKLGDKYVCWYQQRPGQSPVLVIYQDSKRPSGIPERFSGSNSGNTATLTIRGTQAMDEADYYCQAWDSNTAVVFGGGTKLTVL | QAQLVESGGGVVQPGRSLRLSCAASGFTFSNSGMHWVRQGPGKGLEWVAIIYYDGSYKYNADSVKGRFTISRDNSKNTLYLQMNSLRAEDTAVYYCARDRHYDFHVPYYYYYGMDVWGQGTTVTVSS |
| | | | SEQ ID NO: 27458 | SEQ ID NO: 31464 |
| iPS:436820 | 21-225_179D10 | NA | CAGTCTGTGTTGACGCAGCCGCCCTCAGTGTCTGGGGCCCCAGGGCAGAGGGTCACCATCTCCTGCACTGGGAGCAGCTCCAACTTCGGGACAGATTATGATGTACACTGGTACCAGCAATTTCCAGGAACAGCCCCCAAACTCCTCATCTATGGTCACAGCAACCGGCCCTCAGGGGTCCCTGACCGATTTTCTGGCTCCAAGTCTGGCACCTCAGCCTCCCTGGCCATCACTGGGCTCCAGGCTGAGGATGAGGCTGATTATTACTGCCAGTCCTATGATAGAAGCCTGAATGTGGTCTTCGGCGGAGGGACCAAGCTGACCGTCCTA | GAGGTGCAGCTGGTGGAGTCTGGGGGAGGCTTGGTACAGCCTGGGGGGTCCCTGAGATTCTCCTGTGCAGCCTCTGGATTCACCTTCAGTAGCTATAGCATGAACTGGGTCCGCCAGGCTCCAGGGAAGGGGCTGGAGTGGGTTGCATACATTAGTAGTAGTGGAAGTACCACATACTACGCAGACTCTGTGCAGGGCCGATTCACCATCTCCAGAGACAATGCCAAGAACTCACTGTATCTGCAAATGAACAGCCTGAGAGACGAGGACACGGCTGTGTATCGCTGTGCGAGAGATAGTAGGAAGGGGTTCTACTACGGTCTGGACGTCTGGGGCCAAGGGATCACGGTCACCGTCTCCTCA |
| | | | SEQ ID NO: 27459 | SEQ ID NO: 31465 |
| | | AA | QSVLTQPPSVSGAPGQRVTISCTGSSSNFGTDYDVHWYQQFPGTAPKLLIYGHSNRPSGVPDRFSGSKSGTSASLAITGLQAEDEADYYCQSYDRSLNVVFGGGTKLTVL | EVQLVESGGGLVQPGGSLRFSCAASGFTFSSYSMNWVRQAPGKGLEWVAYISSSGSTTYYADSVQGRFTISRDNAKNSLYLQMNSLRDEDTAVYRCARDSRKGFYYGLDVWGQGITVTVSS |
| | | | SEQ ID NO: 27460 | SEQ ID NO: 31466 |

FIGURE 50

| | | | | |
|---|---|---|---|---|
| **iPS:436822** | **21-225_180D4** | NA | TCCTATGAGCTGACTCAGACACCCTCAGTGTCCGTGTCCCCAGGACAGACAGCCAGCATCACCTGCTCTGGAGATAGATTGGGGGATAAATATGCTTGCTGGTATCAGCAGAAGCCAGGCCAGTCCCCTGTGCTGGTCATCTATGAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGGTGACTATTACTGTCAGGCGTGGGACAGTAGGAAAGTGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTTTGGCATGCACTGGGTCCGCCAGGCTCCAGGCAAGGGGCTGGAGTGGGTGGCAATTATATGGTATGATGGAAGTGATAAATACTATGCAGACTCCGTGAAGGGCCGATTCACCATCTCCAGAGACAATTCCAAGAACACGCTGTATCTGCAAATGAACACCCTGAGAGCCGAAGACACGGCTGTGTATTTCTGTGCGAGAGGGGGGCCCCCGTTCTCTACGGTGACTATGTACTTTGACTACTGGGGCCAGGGAACCCTGGTCACCGTCTCCTCA |
| | | | SEQ ID NO: 27461 | SEQ ID NO: 31467 |
| | | AA | SYELTQTPSVSVSPGQTASITCSGDRLGDKYACWYQQKPGQSPVLVIYEDRKRPSGIPERFSGSNSGNTATLTISGTQAMDEGDYYCQAWDSRKVVFGGGTKLTVL | QVQLVESGGGVVQPGRSLRLSCAASGFTFSNFGMHWVRQAPGKGLEWVAIIWYDGSDKYYADSVKGRFTISRDNSKNTLYLQMNTLRAEDTAVYFCARGGPPFSTVTMYFDYWGQGTLVTVSS |
| | | | SEQ ID NO: 27462 | SEQ ID NO: 31468 |
| **iPS:436824** | **21-225_180C5** | NA | TCCTATGAACTGACTCAGCCACCCTCAGTGTCCGTGTCCCCAGGACAGACAGCCAGCATCACCTGCTCTGGAGATAAATTGGGGGAAAAATATGCTTGCTGGTATCAGCAGAAGCCAGGCCAGTCCCCTGTACTGGTCATCTATCAAGATAGAAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTGCGGTATTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGGTTCTCACTCAGCACTAGTGGAGTGGGTGTGGGCTGGATCCGTCAGCCCCCAGGAAAGGCCCTGGAGTGGCTTGGATTCATTTCTTGGAATGATGATAAGCGCTACAGCCCATCTCTGAAGAGCAGCCTCACCATCACCAAGGACACCTCCAAAAACCAGGTGGTTCTTATAATGACCAACATGGACCCTGTGGACACAGCCACATATTACTGTGCACACAAAGCAGCAGCTGTTGCTTTTGATATCTGGGGCCAAGGGACAATGGTCACCGTCTCTTCA |
| | | | SEQ ID NO: 27463 | SEQ ID NO: 31469 |

FIGURE 50

| | | | | |
|---|---|---|---|---|
| | | AA | SYELTQPPSVSVSPGQTASITCSGDKLGEKYACWYQQKPGQSPVLVIYQDRKRPSGIPERFSGSNSGNTATLTISGTQAMDEADYYCQAWDSSTAVFGGGTKLTVL | QITLKESGPTLVKPTQTLTLTCTFSGFSLSTSGVGVGWIRQPPGKALEWLGFISWNDDKRYSPSLKSSLTITKDTSKNQVVLIMTNMDPVDTATYYCAHKAAAVAFDIWGQGTMVTVSS |
| | | | SEQ ID NO: 27464 | SEQ ID NO: 31470 |
| **iPS:436826** | **21-225_180G5** | NA | TCCTATGAGCTGACTCAGCCACCCTCAGTGTCCGTGTACCCAGGACAGACAGCCAGCATCACCTGCTCTGGAGATAAATTGGGGGATAAATATGTTAGCTGGTATCAACAGAAGCCAGGCCAGTCCCCTGTTCTGGTCATCTATCAAGATAGCAAGCGGCCCTCAGGGATCCCTGAGCGATTCTCTGGCTCCAACTCTGGGAACCCAGCCTCTCTGACCATCAGCGGGACCCAGGCTATGGATGAGGCTGACTATTACTGTCAGGCGTGGGACATCACCACTGCGGTATTCGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCAGTTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGAGAGGGTTTTATTACTATGGTTCGGGGAGTCATGTCCCTTACCACTACTACTACGGTTTGGACGTCTGGGGCCAAGGGACCACGGTCACCGTCTCCTCA |
| | | | SEQ ID NO: 27465 | SEQ ID NO: 31471 |
| | | AA | SYELTQPPSVSVYPGQTASITCSGDKLGDKYVSWYQQKPGQSPVLVIYQDSKRPSGIPERFSGSNSGNPASLTISGTQAMDEADYYCQAWDITTAVFGGGTKLTVL | QVQLVQSGAEVKKPGASVKVSCKASGYTFTSYDINWVRQATGQGLEWMGWMNPNSGNTGYAQKFQGRVTMTRNTSISTAYMELSSLRSEDTAVYYCARGFYYYGSGSHVPYHYYYGLDVWGQGTTVTVSS |
| | | | SEQ ID NO: 27466 | SEQ ID NO: 31472 |

FIGURE 50

| | | | | |
|---|---|---|---|---|
| **iPS:436828** | **21-225_181H1** | NA | TCCTATGAGCTGACTCAGACACCCTCAGTGTC<br>CGTGTCCCCAGGACAGACAGCCAGCATCACCT<br>GCTCTGGAGATAAATTGGGGGATAAATATGCT<br>TGCTGGTATCAGCAGAAGCCAGGCCAGTCCCC<br>TGTGCTGGTCATCTATGAAGATAGGAAGCGGC<br>CCTCAGGGATCCCTGAGCGATTCTCTGGCTCC<br>AACTCTGGGAACACAGCCACTCTGACCATCAG<br>CGGGACCCAGGCTATGGATGAGGCTGACTATT<br>ACTGTCAGGCGTGGGACAGCAGGAAAGTGGT<br>ATTCGGCGGAGGGACCAAGCTGACCGTCCTA | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTAACTATGGCAT<br>GCACTGGGTCCGCCAGGCTCCAGGCAAGGGGCT<br>GGAGTGGGTGGCAATTATATGGTATGATGGAAGT<br>GATAAATACTATGCAGACTCCGTGAAGGGCCGA<br>ATCACCATCTCCAGAGACAATTCCAAGAACACGC<br>TGTATCTGCAAATGAACACCCTGAGAGCCGAAG<br>ACACGGCTGTGTATTTCTGTGCGAGAGGGGGGCC<br>CCCGTTTTCTACGGTGACTATGTACTTCGACTACT<br>GGGGCCAGGGAACCCTGGTCACCGTCTCCTCA |
| | | | SEQ ID NO: 27467 | SEQ ID NO: 31473 |
| | | AA | SYELTQTPSVSVSPGQTASITCSGDKLGDKYAC<br>WYQQKPGQSPVLVIYEDRKRPSGIPERFSGSNSG<br>NTATLTISGTQAMDEADYYCQAWDSRKVVFGG<br>GTKLTVL | QVQLVESGGGVVQPGRSLRLSCAASGFTFSNYGM<br>HWVRQAPGKGLEWVAIIWYDGSDKYYADSVKGRI<br>TISRDNSKNTLYLQMNTLRAEDTAVYFCARGGPPF<br>STVTMYFDYWGQGTLVTVSS |
| | | | SEQ ID NO: 27468 | SEQ ID NO: 31474 |
| **iPS:436830** | **21-225_51F4** | NA | CAGTCTGTGCTGACTCAGCCACCCTCAGCGTC<br>TGGGACCCCCGGGCAGAGGGTCACCATCTCTT<br>GTTCTGGAAGCAGCTCCAACATCGGAAGTAAT<br>ATTGTGACCTGGTACCAGCAGCTCCCAGGAAC<br>GGCCCCCAAACTCCTCATCTATAGTAATGATC<br>AGCGGCCCTCAGGGGTCCCTGACCGATTCTCT<br>GGCTCCAAGTCTGGCACCTCAGCCTCCCTGGC<br>CATCAGTGGGCTCCAGTCTGAGGATGAGACTG<br>ATTATTACTGTACAGCATGGGATGACAGCCTG<br>AATGGTTGGGTGTTCGGCGGAGGGACCACGCT<br>GACCGTCCTA | CAGGTTCAGCTGGTGCAGTCTGGAGCTGAGGTGA<br>AGAAGCCTGGGGCCTCAGTGAAGGTCTCCTGCA<br>AGGCTTCTGGTTACACCTTTAACAGCTATGGTAT<br>CAGCTGGGTGCGACAGGCCCCTGGACAAGGGCT<br>TGAGTGGATGGGATGGATCAGCGCTTATAATGGT<br>AACACAAAGTATGCACAGAAGCTCCAGGGCAGA<br>GTCACCATGACCACAGACACATCCACGAGCACA<br>GCCTACATGGAGCTGAGGAGCCTGAGATCTGAC<br>GACACGGCCGTGTATTACTGTGCGAGACACGATT<br>TTTGGAGTGGTTATTATAAGGGTATGGACGTCTG<br>GGGCCAAGGGACCACGGTCACCGTCTCCTCA |
| | | | SEQ ID NO: 27469 | SEQ ID NO: 31475 |

FIGURE 50

| | | | | |
|---|---|---|---|---|
| | | AA | QSVLTQPPSASGTPGQRVTISCSGSSSNIGSNIVT<br>WYQQLPGTAPKLLIYSNDQRPSGVPDRFSGSKS<br>GTSASLAISGLQSEDETDYYCTAWDDSLNGWVF<br>GGGTTLTVL | QVQLVQSGAEVKKPGASVKVSCKASGYTFNSYGIS<br>WVRQAPGQGLEWMGWISAYNGNTKYAQKLQGR<br>VTMTTDTSTSTAYMELRSLRSDDTAVYYCARHDF<br>WSGYYKGMDVWGQGTTVTVSS |
| | | | SEQ ID NO: 27470 | SEQ ID NO: 31476 |
| **iPS:436832** | **21-225_51D8** | NA | CAGTCTGTGCTGACGCAGCCGCCCTCAGTGTC<br>TGGGGCCCCAGGGCAGAGGGTCACCATCTCCT<br>GCACTGGGAGCAGCTCCAACATCGGGGCAGG<br>TTTTGAAGTACACTGGTACCAGCAGCTTCCAG<br>GAACAGCCCCCAAACTCCTCATCTATGGTAAC<br>AGCAATCGGCCCTCAGGGGTCCCTGACCGATT<br>CTCTGGCTCCAAGTCTGGCACCTCAGCCTCCCT<br>GGCCATCACTGGGCTCCAGGCTGAGGATGAGG<br>CTGATTATTACTGCCAGTCCTATGACAGCAGC<br>CTGAGTGGTTATGTCTTCGGAACTGGGACCAA<br>GGTCACCGTCCTA | CAGGTACAGCTGCAGCAGTCAGGTCCAGGACTG<br>GTGAAGCCCTCGCAGACCCTCTCACTCACCTGTG<br>CCATCTCCGGGGACAGTGTCTCTAGTAACAGTGC<br>TGCTTGGAACTGGATCAGGCAGTCCCCATCGAGA<br>GGCCTTGAGTGGCTGGGAAGGACATACTACAGG<br>TCCAAGTGGTATAATGATTATGCAGTATCTGTGA<br>AAAGTCGAATAACCTTCAACCCGGACACATCCA<br>AGAACCAGTTCTCCCTGCGGCTGAACTCTGTGAC<br>TCCCGAGGACACGGCTGTGTATTACTGTGCAAGA<br>GACCGCTATAACTGGAACTACCCCTACTGGTACT<br>TCGATCTCTGGGGCCGTGGCACCCTGGTCACTGT<br>CTCCTCA |
| | | | SEQ ID NO: 27471 | SEQ ID NO: 31477 |
| | | AA | QSVLTQPPSVSGAPGQRVTISCTGSSSNIGAGFEV<br>HWYQQLPGTAPKLLIYGNSNRPSGVPDRFSGSK<br>SGTSASLAITGLQAEDEADYYCQSYDSSLSGYVF<br>GTGTKVTVL | QVQLQQSGPGLVKPSQTLSLTCAISGDSVSSNSAA<br>WNWIRQSPSRGLEWLGRTYYRSKWYNDYAVSVK<br>SRITFNPDTSKNQFSLRLNSVTPEDTAVYYCARDRY<br>NWNYPYWYFDLWGRGTLVTVSS |
| | | | SEQ ID NO: 27472 | SEQ ID NO: 31478 |

FIGURE 50

| | | | | |
|---|---|---|---|---|
| **iPS:436834** | **21-225_52F1** | NA | CAGTCTGTGCTGACTCAGCCACCCTCAGCGTCTGGGACCCCCGGGCAGAGGGTCACCATCTCTTGTTCTGGAAGCAGCTCCAACATCGGAAGTAATATTGTGACCTGGTACCAGCAGCTCCCAGGAACGGCCCCCAAACTCCTCATCTATAGTAATGATCAGCGGCCCTCAGGGGTCCCTGACCGATTCTCTGGCTCCAAGTCTGGCACCTCAGCCTCCCTGGCCATCAGTGGGCTCCAGTCTGAGGATGAGGCTGATTATTACTGTGCAGCATGGGATGACAGCCTGAATGGTTGGGTGTTCGGCGGAGGGACCACGCTGACCGTCCTA | CAGGTTCAGCTGGTGCAGTCTGGAGCTGAGGTGAAGAAGCCTGGGGCCTCAGTGAAGGTCTCCTGCAAGGCTTCTGGTTACACCTTTAACAGCTATGGTGTCAGCTGGGTGCGACAGGCCCCTGGACAAGGGCTTGAGTGGATGGGATGGATCAGCGCTTATAATGGTAACAGAAAGTATGCACAGAAGCTCCAGGGCAGAGTCTCCATGACCACAGACACATCCACGAGCACAGCCTACATGGAGCTGAGGAGCCTGAGATCTGACGACACGGCCGTGTATTACTGTGCGAGACACGATTTTTGGAGTGGTTATTATAAGGGTATGGACGTCTGGGGCCAAGGGACCACGGTCACCGTCTCCTCA |
| | | | SEQ ID NO: 27473 | SEQ ID NO: 31479 |
| | | AA | QSVLTQPPSASGTPGQRVTISCSGSSSNIGSNIVTWYQQLPGTAPKLLIYSNDQRPSGVPDRFSGSKSGTSASLAISGLQSEDEADYYCAAWDDSLNGWVFGGGTTLTVL | QVQLVQSGAEVKKPGASVKVSCKASGYTFNSYGVSWVRQAPGQGLEWMGWISAYNGNRKYAQKLQGRVSMTTDTSTSTAYMELRSLRSDDTAVYYCARHDFWSGYYKGMDVWGQGTTVTVSS |
| | | | SEQ ID NO: 27474 | SEQ ID NO: 31480 |
| **iPS:436836** | **21-225_52H1** | NA | TCCTATGAGCTGACTCAGCCACCCTCAGTGTTCGTGTCCCCAGGACAGACAGCCAGCATCACCTCCTCTGGAGATAAATTGGGGGATAAATATGTTTCCTGGTATCAGCAGAAGCCAGGCCAGTCCCCTGTGCTGGTCATCTATCAAGATAGAAAGCGGCCCTCAGGGATCCCTGAGCGATTCTCTGGCTCCAATTCTGGGAATACAGCCACTCTGACCATCAGCGGGACCCAGGCTATGGATGAGGCTGACTATTATTGTCAGGCGTGGGACAACAGCACTGTGGTATTCGGCGGAGGGACCAAGCTGACCGTCCTA | CAGGTGCAGCTGGTGGAGTCTGGGGGAGGCGTGGTCCAGCCCGGGAGGTCCCTGAGACTCTCCTGTGCAGCGTCTGGATTCACCTTCAGTGGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTTTATTACTGTGCGAGAGATCGGGTCTATTGTAGTAGTTCCAGCTGCTCATATTACTACTACTACTACGGTATGGACGTCTGGGGCCAAGGGACCACGGTCACCGTCTCCTCA |

FIGURE 50

| | | | | |
|---|---|---|---|---|
| | | | SEQ ID NO: 27475 | SEQ ID NO: 31481 |
| | | AA | SYELTQPPSVFVSPGQTASITSSGDKLGDKYVSWYQQKPGQSPVLVIYQDRKRPSGIPERFSGSNSGNTATLTISGTQAMDEADYYCQAWDNSTVVFGGGTKLTVL | QVQLVESGGGVVQPGRSLRLSCAASGFTFSGYGMHWVRQAPGKGLEWVAVIWYDGSNKYYADSVKGRFTISRDNSKNTLYLQMNSLRAEDTAVYYCARDRVYCSSSSCSYYYYYYGMDVWGQGTTVTVSS |
| | | | SEQ ID NO: 27476 | SEQ ID NO: 31482 |
| iPS:436838 | 21-225_52H4 | NA | CAGTCTGCCCTGACTCAGCCTGCCTCCGTGTCTGGGTCTCCTGGACAGTCGATCACCATCTCCTGCACTGGAACCAGCAGTGACGTTGGTGGTTATAACTATGTCTCCTGGTACCAACAGCACCCAGGCAAAGCCCCCAAACTCATGATTTATGAGGTCAGTAATCGGCCCTCAGGGGTTTCTAATCGCTTCTCTGGCTCCAAGTCTGGCAATACGGCCTCCCTGACCATCTCTGGGCTCCAGGCTGAGGACGAGGCTGATTATTACTGCAACTCATATACAAGCAACATCACTTGGGTGTTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTCACTTTGGCATGCACTGGGTCCGCCAGGCTCCAGGCAAGGGGCTGAAATGGGTGGCAGTTATTTGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGGGGACTGGAACTACGAGGGTTTTGACTACTGGGGCCAGGGAACCCTGGTCACCGTCTCCTCA |
| | | | SEQ ID NO: 27477 | SEQ ID NO: 31483 |
| | | AA | QSALTQPASVSGSPGQSITISCTGTSSDVGGYNYVSWYQQHPGKAPKLMIYEVSNRPSGVSNRFSGSKSGNTASLTISGLQAEDEADYYCNSYTSNITWVFGGGTKLTVL | QVQLVESGGGVVQPGRSLRLSCAASGFTFSHFGMHWVRQAPGKGLKWVAVIWYDGSNKYYADSVKGRFTISRDNSKNTLYLQMNSLRAEDTAVYYCARGDWNYEGFDYWGQGTLVTVSS |
| | | | SEQ ID NO: 27478 | SEQ ID NO: 31484 |

FIGURE 50

| | | | | |
|---|---|---|---|---|
| **iPS:436840** | **21-225_53E9** | NA | TCCTATGAGCTGACTCAGCCACCCTCAGTGTCCGTGTCCCCAGGACAGACAGCCAGCATCACCTGCTCTGGAACTAAATTGGGGGATAAATATGTTTGCTGGTATCAACAGAAGCCAGGCCAGTCCCCTGTGTTGGTCATCAATCAAGATACAATGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGACGTGGGACAGCAGCACTGCGGTTTTCGGCGGAGGGACCACGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGAAACACCTTCACCGGCTACTATATGCACTGGGTGCGACAGGCCCCTGGACAAGGGCTTGAGTGGATGGGATGGATCATCCCTAACAGTGGTGACACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTCTATTACTGTGCGAGAGATGGGTATAGCAGTGGCTGGTTCAACTGGTTCGACCCCTGGGGCCAGGGAACCCTGGTCACCGTCTCCTCA |
| | | | SEQ ID NO: 27479 | SEQ ID NO: 31485 |
| | | AA | SYELTQPPSVSVSPGQTASITCSGTKLGDKYVCWYQQKPGQSPVLVINQDTMRPSGIPERFSGSNSGNTATLTISGTQAMDEADYYCQTWDSSTAVFGGGTTLTVL | QVQLVQSGAEVKKPGASVKVSCKASGNTFTGYYMHWVRQAPGQGLEWMGWIIPNSGDTNYAQKFQGRVTMTRDTSISTAYMELSRLRSDDTAVYYCARDGYSSGWFNWFDPWGQGTLVTVSS |
| | | | SEQ ID NO: 27480 | SEQ ID NO: 31486 |
| **iPS:436842** | **21-225_54E9** | NA | CAGTCTGTGCTGACTCAGCCACCCTCAGCGTCTGGGACCCCCGGGCAGAGGGTCACCATCTCTTGTTCTGGAAGCAACTCCAACATCGGAAATAATATTGTCACCTGGTACCAGCAGCTCCCAGGAACGGCCCCCAAACTCCTCATCTATGTTAATGATCAGCGGCCCTCAGGGGTCCCTGACCGATTCTCTGGCTCCAAGTCTGGCACCTCTGCCTCCCTGGCCATCAGTGGGCTCCAGTCTGAGGATGAGGCTGATTATTACTGTGCAGCATGGGATGACAGCCTGAATGGTTGGGTGTTCGGCGGGGGGACCACGCTGACCGTCCTA | CAGGTTCAGCTGGTGCAGTCTGGAGCTGAGGTGAAGAAGCCTGGGGCCTCAGTGAAGGTCTCCTGCAAGGCTTCTGGTTACACCTTTAACAGCTATGGTATCAGCTGGGTGCGACTGGCCCCTGGACAAGGGCTTGAGTGGATGGGATGGATTAGTGCTTATAATGGTAACACAAAGAATGCACAGAAGCTCCAGGGCAGAGTCACCATGACCACAGACACATCCACGAGCACAGCCTACATGGAGCTGAGGAGCCTGAGATCTGACGACACGGCCGTTTATTACTGTGCGAGACACGATTTTTGGAGTGGTTATTATAAGGGTATGGACGTCTGGGGCCAAGGGACCACGGTCACCGTCTCCTCA |
| | | | SEQ ID NO: 27481 | SEQ ID NO: 31487 |

FIGURE 50

| | | | | |
|---|---|---|---|---|
| | | AA | QSVLTQPPSASGTPGQRVTISCSGSNSNIGNNIVTWYQQLPGTAPKLLIYVNDQRPSGVPDRFSGSKSGTSASLAISGLQSEDEADYYCAAWDDSLNGWVFGGGTTLTVL | QVQLVQSGAEVKKPGASVKVSCKASGYTFNSYGISWVRLAPGQGLEWMGWISAYNGNTKNAQKLQGRVTMTTDTSTSTAYMELRSLRSDDTAVYYCARHDFWSGYYKGMDVWGQGTTVTVSS |
| | | | SEQ ID NO: 27482 | SEQ ID NO: 31488 |
| **iPS:436844** | **21-225_56G1** | NA | CAGTCTGTGCTGACTCAGCCACCCTCAGCGTCTGGGACCCCCGGGCAGAGGGTCACCATCTCTTGTTCTGGAAGCAGCTCCAACATCGGAAGTCATATTGTTACCTGGTACCAGCAGCTCCCAGGAACGGCCCCCAAACTCCTCATCTACAGTAATGATCAGCGGCCCTCAGGGGTCCCTGACCGATTCTCTGGCTCCAAGTCTGGCACCTCAGCCTCCCTGGCCATCAGTGGGCTCCAGTCTGAGGATGAGGCTGATTATTACTGTGCAGTATGGGATGACAGCCTGATTGGTTGGGTGTTCGGCGGAGGGACCACGCTGACCGTCCTA | CAGGTTCAGCTGGTGCAGTCTGGAGCTGAGGTGAAGAAGCCTGGGGCCTCAGTGAAGGTCTCCTGCAAGGCTTCTGGCTACACCTTTAACAGCTATGGTATCAGCTGGGTGCGACAGGCCCCTGGACAAGGGCTTGAGTGGATGGGATGGATCAGCGCTTATAATGGTAACACAAAGTATGCACAGAAGTTCCAGGGCAGAGTCACCATGACCACAGACACATCCACGAGCACAGCCTACATGGAGCTGAGGAGCCTGAGATCTGACGACACGGCCGTGTATTACTGTGCGAGACACGATTTTTGGAGTGGTTATTATAAGGGTATGGACGTCTGGGGCCAAGGGACCACGGTCACCGTCTCCTCA |
| | | | SEQ ID NO: 27483 | SEQ ID NO: 31489 |
| | | AA | QSVLTQPPSASGTPGQRVTISCSGSSSNIGSHIVTWYQQLPGTAPKLLIYSNDQRPSGVPDRFSGSKSGTSASLAISGLQSEDEADYYCAVWDDSLIGWVFGGGTTLTVL | QVQLVQSGAEVKKPGASVKVSCKASGYTFNSYGISWVRQAPGQGLEWMGWISAYNGNTKYAQKFQGRVTMTTDTSTSTAYMELRSLRSDDTAVYYCARHDFWSGYYKGMDVWGQGTTVTVSS |
| | | | SEQ ID NO: 27484 | SEQ ID NO: 31490 |

FIGURE 50

| | | | | |
|---|---|---|---|---|
| **iPS:436846** | **21-225_56E3** | NA | CAGTCTGTGCTGACTCAGCCACCCTCAGCGTCTGGGACCCCCGGGCAGAGGGTCACCATCTCTTGTTCTGGAAGCAGCTCCAACATCGGAAGTAATATTGTTACCTGGTACCAGCAGCTCCCAGGAACGGCCCCCAAACTCCTCATCTATAGTAATAATCAGCGGCCCTCAGGGGTCCCTGACCGATTCTCTGGCTCCAAGTCTGGCACCTCAGCCTCCCTGGCCATCAGTGGACTCCAGTCTGAGGATGAGGCTGATTATTGCTGTGCAGCATGGGATGACAGCCTGAATGGTTGGGTGTTCGGCGGAGGGACCACGCTGACCGTCCTA | CAGGTTCAACTGGTGCAGTCTGGAGCTGAGGTGAAGAAGCCTGGGGCCTCAGTGAAGGTCTCCTGCAAGGCTTCTGGTTACACCTTTAACAGCTATGGTTTCAGCTGGGTGCGACAGGCCCCTGGACAAGGGCTTGAGTGGATGGGATGGATCAGCGCTTATAATGGTAACACAAAGGAAGCACAGAAGTTCCAGGGCAGAGTCACCATGACCACAGACACATCCACGAGCACAGCCTACATGGAGCTGAGGAGCCTGAGAGCTGACGACACGGCCGTGTATTACTGTGCGAGACACGATTTTTGGAGTGGTTATTATAAGGGTATGGACGTCTGGGGCCAAGGGACCACGGTCACCGTCTCCTCA |
| | | | SEQ ID NO: 27485 | SEQ ID NO: 31491 |
| | | AA | QSVLTQPPSASGTPGQRVTISCSGSSSNIGSNIVTWYQQLPGTAPKLLIYSNNQRPSGVPDRFSGSKSGTSASLAISGLQSEDEADYCCAAWDDSLNGWVFGGGTTLTVL | QVQLVQSGAEVKKPGASVKVSCKASGYTFNSYGFSWVRQAPGQGLEWMGWISAYNGNTKEAQKFQGRVTMTTDTSTSTAYMELRSLRADDTAVYYCARHDFWSGYYKGMDVWGQGTTVTVSS |
| | | | SEQ ID NO: 27486 | SEQ ID NO: 31492 |
| **iPS:436848** | **21-225_57F1** | NA | TCCTATGAGCTGACTCAGCCACCCTCAGCGTCCGTGTCCCCAGGACAGACAGCCAGCATCACCTGCTCTGGAGATAAACTGGGGGAAAAATATGCTTGCTGGTATCAGCAGAAGCCAGGCCAGTCCCCTGTGTTGGTCATCTATCAAGATAGGAAGCGGCCCTCAGGGATCCCTGAGCGATTCTCTGGCTCCAATTCTGGGAACACAGCCACTCTGACCATCAGCGGGACCCAGGCTATGGATGAGGCTGACTATTACTGTCAGGCGTGGGACAGCAGCACTGTGGTATTCGGCGGAGGGACCAAACTGACCGTCCTA | CAGATCACCTTGAAGGAGTCTGGTCCTACGCTGGTGAAACCCACACAGACCCTCACGCTGACCTGCACCTTCTCTGGGTTCTCACTCAGTACTAGTGGAGTGGGTGTGGGCTGGATCCGTCAGCCCCCAGGAAAGGCCCTGGAGTGGCTTGCACTCATTTATTGGCATGAAGATAAGCGCTACAGCCCATCTCTGAAGAGCAGGCTCACCATCACTGAGGACACCTCCAAAAACCAGGTGGACCTTACAATGACCAACATGGCCCCTGTGGACACAGCCACATATTACTGTGCACACGTCACAGGTATAGCAGCTCCCTACTGGGGCCAGGGAACCCTGGTCACCGTCTCCTCA |
| | | | SEQ ID NO: 27487 | SEQ ID NO: 31493 |

FIGURE 50

| | | | | |
|---|---|---|---|---|
| | | AA | SYELTQPPSASVSPGQTASITCSGDKLGEKYACWYQQKPGQSPVLVIYQDRKRPSGIPERFSGSNSGNTATLTISGTQAMDEADYYCQAWDSSTVVFGGGTKLTVL | QITLKESGPTLVKPTQTLTLTCTFSGFSLSTSGVGVGWIRQPPGKALEWLALIYWHEDKRYSPSLKSRLTITEDTSKNQVDLTMTNMAPVDTATYYCAHVTGIAAPYWGQGTLVTVSS |
| | | | SEQ ID NO: 27488 | SEQ ID NO: 31494 |
| iPS:436850 | 21-225_57D9 | NA | TCCTATGAGCTGACTCAGCCACCCTCAGTGTCCGTGTCCCCAGGACAGACAGCCAGCATCACCTGCTCTGGAGAGAAATTGGGGGAAAAATTTGCTTGCTGGTCTCAGCAGAAGCCAGGCCAGTCCCCTGTGTTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTGTGGTATTCGGCGGAGGGACCAAGCTGACCGTCCTA | CAGATCACCTTGAAGGAGTCTGGTCCTATGCTGGTGAAACCCACACAGACCCTCACGCTGACCTGCACCTTCTCTGGGTTCTCACTCAGCACTAGTGGAGTGGGTGTGGGCTGGATCCGTCAGCCCCCAGGAAAGGCCCTGGAGTGGCTTGCACTCATTTATTGGAATGATGATAAGCGCTACAGTCCATCTCTGAAGAGCAGGCTCACCATCACCGAGGACACCTCCAAAAACCAGGTGGTCCTTACAATGACCAACATGGACCCTGTGGACACAGCCACATATTACTGTGCACATGCAGTGGCTGTCTCCTTTGACTACTGGGGCCAGGGAACCCTGGTCACCGTCTCCTCA |
| | | | SEQ ID NO: 27489 | SEQ ID NO: 31495 |
| | | AA | SYELTQPPSVSVSPGQTASITCSGEKLGEKFACWSQQKPGQSPVLVIYQDSKRPSGIPERFSGSNSGNTATLTISGTQAMDEADYYCQAWDSSTVVFGGGTKLTVL | QITLKESGPMLVKPTQTLTLTCTFSGFSLSTSGVGVGWIRQPPGKALEWLALIYWNDDKRYSPSLKSRLTITEDTSKNQVVLTMTNMDPVDTATYYCAHAVAVSFDYWGQGTLVTVSS |
| | | | SEQ ID NO: 27490 | SEQ ID NO: 31496 |

FIGURE 50

| | | | | |
|---|---|---|---|---|
| iPS:436852 | 21-225_57H11 | NA | TCCTATGCGCTGACTCAGCCACCCTCAGCGTCCGTGTCCCCAGGACAGACAGCCAGCATCACCTGCTCTGGAGATAAACTGGGGGAAAAATATGCTTGCTGGTATCAGCAGAAGCCAGGCCAGTCCCCTGTGTTGGTCATCTATCAAGATAGGAAGCGGCCCTCAGGGATCCCTGAGCGATTCTCTGGCTCCAATTCTGGGAACACAGCCACTCTGACCATCAGCGGGACCCAGGCTATGGATGAGGCTGACTATTACTGTCAGGCGTGGGACAGCAGCACTGTGGTATTCGGCGGAGGGACCAAACTGACCGTCCTA | CAGATCACCTTGAAGGAGTCTGGTCCTACGCTGGTGAAACCCACACAGACCCTCACGCTGACCTGCACCTTCTCTGGGTTCTCACTCACTACTAGTGGAGTGGGTGTGGGCTGGATCCGTCAGCCCCCAGGAAAGGCCCTGGAGTGGCTTGCACTCATTTATTGGCATGAAGATAGGCGCTACAGCCCATCTCTGAAGAGCAGGCTCACCATCACTGAGGACACCTCCAAAAACCAGGTGGACCTTACAATGACCAACATGGCCCCTGTGGACACAGCCACATATTACTGTGCACACGTCACAGGTATAGCAGCTCCCTACTGGGGCCAGGGAACCCTGGTCACCGTCTCCTCA |
| | | | SEQ ID NO: 27491 | SEQ ID NO: 31497 |
| | | AA | SYALTQPPSASVSPGQTASITCSGDKLGEKYACWYQQKPGQSPVLVIYQDRKRPSGIPERFSGSNSGNTATLTISGTQAMDEADYYCQAWDSSTVVFGGGTKLTVL | QITLKESGPTLVKPTQTLTLTCTFSGFSLTTSGVGVGWIRQPPGKALEWLALIYWHEDRRYSPSLKSRLTITEDTSKNQVDLTMTNMAPVDTATYYCAHVTGIAAPYWGQGTLVTVSS |
| | | | SEQ ID NO: 27492 | SEQ ID NO: 31498 |
| iPS:436854 | 21-225_58C1 | NA | TCCTATGAGCTGACTCAGCCACCCTCAGTGTCCGTGTCCCCAGGACAGACAGCCAACATCACCTGCTCTGGAGATAAATTGGGGAATAAATATGCTTGCTGGTATCAGCAGAAGCCAGGCCAGTCCCCTGTATTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTGCATTCGGCGGAGGGACCAAGCTGACCGTCCTC | CAGATCACCTTGAAGGAGTCTGGTCCTACGCTGGTGAAACCCACACAGACCCTCACGCTGACCTGCACCTTCTCTGGGTTCTCACTCAGCACTAGTGGAGTGGGTGTGGGCTGGATCCGTCAGCCCCCAGGAAAGGCCCTGGAGTGGCTTGCACTCATTTATTGGGATGATGATAAGCGCTACAGCCCATCTCTGAAGAGCAGGCTCACCATCACCGAGGACACCTCCAAAAACCAGGTGGTCCTTACAATGACCAACATGGACCCTGTGGACACAGCCACATATTACTGTGCACACCTTATAGCAGTGGCCTTTGACTCCTGGGGCCAGGGAACCCTGGTCACCGTCTCCTCA |
| | | | SEQ ID NO: 27493 | SEQ ID NO: 31499 |

FIGURE 50

| | | | | |
|---|---|---|---|---|
| | | AA | SYELTQPPSVSVSPGQTANITCSGDKLGNKYACWYQQKPGQSPVLVIYQDRKRPSGIPERFSGSNSGNTATLTISGTQAMDEADYYCQAWDSSTAFGGGTKLTVL | QITLKESGPTLVKPTQTLTLTCTFSGFSLSTSGVGVGWIRQPPGKALEWLALIYWDDDKRYSPSLKSRLTITEDTSKNQVVLTMTNMDPVDTATYYCAHLIAVAFDSWGQGTLVTVSS |
| | | | SEQ ID NO: 27494 | SEQ ID NO: 31500 |
| iPS:436856 | 21-225_58C5 | NA | CAGTCTGTGTTGACGCAGCCGCCCTCAGTGTCTGCGGCCCCAGGACAGAAGGTCACCATCTCCTGCTCTGGAAGCAGCTCCAACATTGGGAATAATTATGTATCCTGGTACCAGCAGCTCCCAGGAACAGCCCCCAAACTCCTCATTTATGACAATAATAAGCGACCCTCAGGGATTCCTGACCGATTTTCTGGCTCCAAGTCTGGCACGTCAGCCACCCTGGGCATCACCGGACTCCAGACTGGGGACGAGGCCGATTATTACTGCGGAACATGGGATATCAGTCTGAGTGTTGGGGTATTCGGCGGAGGGACCAAGCTGACCGTCCTA | GAGGTGCAGTTGGTGGAGTCTGGGGGAGGCCTGGTCAAGCCTGGGGGGTCCCTGAGACTCTCCTGTGCAGCCTCTGGATTCACCTTCAATAGCTATAGCATGAACTGGGTCCGCCAGGCTCCAGGGAAGGGGCTGGAGTGGATCTCATCCATTAGTAGTAGTAGTTATTACTTATACTACGCAGACTCAGTGAAGGGCCGATTCACCATCTCCAGAGACAGCGCCAAGAATTCACTGTATCTGCAAATGAACAGCCTGAGAGCCGAGGACACGGCTGTATATTACTGTGCGAGGACCTATAGTGGGAGTTTTGACTACTGGGGCCAGGGAACCCTGGTCACCGTCTCCTCA |
| | | | SEQ ID NO: 27495 | SEQ ID NO: 31501 |
| | | AA | QSVLTQPPSVSAAPGQKVTISCSGSSSNIGNNYVSWYQQLPGTAPKLLIYDNNKRPSGIPDRFSGSKSGTSATLGITGLQTGDEADYYCGTWDISLSVGVFGGGTKLTVL | EVQLVESGGGLVKPGGSLRLSCAASGFTFNSYSMNWVRQAPGKGLEWISSISSSSYYLYYADSVKGRFTISRDSAKNSLYLQMNSLRAEDTAVYYCARTYSGSFDYWGQGTLVTVSS |
| | | | SEQ ID NO: 27496 | SEQ ID NO: 31502 |

FIGURE 50

| | | | | |
|---|---|---|---|---|
| **iPS:436858** | **21-225_58E7** | NA | TCCTATGAACTGACTCAGTCACCCTCGGTGTCCGTGTCCCCAGGACAGACAGCCAGCATCACCTGTTCTGGAGATAAATTGGGGGATAAATATACTTGCTGGTATCAGAAGAAGCCAGGCCAGTCCCCTGTGCTGGTCATCTATCAAGATAACAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTTCTGTCAGGCGTGGAACAACTACACTGTGGTATTCGGCGGAGGGACCAAGCTGACCGCTCTA | CAGGTGCAGTTGGTGGAGTCTGGGGGAGGCGTGGTCCAGCCTGGGAGGTCCCTGAGACTCTCCTGTGCAGCCTCTGGATTCACCTTCAGTTTCTATGGCATGCACTGGGTCCGCCAGGCTCCAGGCAAGGGGCTGGAGTGGGTGGCAGTTACATCATATGATGGAAGTGATAAATACTATGCAGACTCCGTGAAGGGCCGATTCTCCATCTCCAGAGACAATTCCAAGAACACGCTGTATCTGCAAATGAGCAGCCTGAGAGCTGAGGACACGGCTATGTATTACTGTGCGAGAGATGACTATGGTTCGGGGAGTCCCCTATACTACGGTATGGACGTCTGGGGCCAAGGGACCACGGTCACCGTCTCCTCA |
| | | | SEQ ID NO: 27497 | SEQ ID NO: 31503 |
| | | AA | SYELTQSPSVSVSPGQTASITCSGDKLGDKYTCWYQKKPGQSPVLVIYQDNKRPSGIPERFSGSNSGNTATLTISGTQAMDEADYFCQAWNNYTVVFGGGTKLTAL | QVQLVESGGGVVQPGRSLRLSCAASGFTFSFYGMHWVRQAPGKGLEWVAVTSYDGSDKYYADSVKGRFSISRDNSKNTLYLQMSSLRAEDTAMYYCARDDYGSGSPLYYGMDVWGQGTTVTVSS |
| | | | SEQ ID NO: 27498 | SEQ ID NO: 31504 |
| **iPS:436860** | **21-225_58F7** | NA | TCCTATGAGCTGACTCAGCCACCCTCAGTGTCCGTGTCCCCAGGACAGACAGCCAGCATCACCTGCTCTGGAGATAAATTGGGGGATAAATATGCTTGCTGGTATCAGCAGAAGCCAGGCCAGTCCCCTGTGCTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTGTGGTATTCGGCGGAGGGACCAAGCTGACCGTCCTA | GAGGTGCAGCTGGTGGAGTCTGGGGGAGGCTTGGTCCAGCCTGGGGGGTCCCTGAGACTCTCCTGTGCAGCCTCTGGATTCACCTTTAGTAGCTTTTGGATGAGCTGGGTCCGCCAGGCTCCAGGGAAGGGGCTGGAGTGGGTGGCCCACATAAAGCAAGATGGAAGTGAGAAATACTATGTGGACTCTGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGGGGACCTCCCATACAGCTCGGGCTACTACTACGGTATGGACGTCTGGGGCCAAGGGACCACGGTCACCGTCTCCTCA |
| | | | SEQ ID NO: 27499 | SEQ ID NO: 31505 |

FIGURE 50

| | | | | |
|---|---|---|---|---|
| | | AA | SYELTQPPSVSVSPGQTASITCSGDKLGDKYACWYQQKPGQSPVLVIYQDRKRPSGIPERFSGSNSGNTATLTISGTQAMDEADYYCQAWDSSTVVFGGGTKLTVL | EVQLVESGGGLVQPGGSLRLSCAASGFTFSSFWMSWVRQAPGKGLEWVAHIKQDGSEKYYVDSVKGRFTISRDNAKNSLYLQMNSLRAEDTAVYYCARGDLPYSSGYYYGMDVWGQGTTVTVSS |
| | | | SEQ ID NO: 27500 | SEQ ID NO: 31506 |
| **iPS:436862** | **21-225_58F8** | NA | TCCTATGAGCTGACTCAGCCACCCTCAGTGTCCGTGTCCCCAGGACAGACAGCCAGCATCACCTGCTCTGGAGATAAATTGGGAAATAAATATGCTTGCTGGTATCAGCAGAAGCCAGGCCAGTCCCCTGTACTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTGTGGTATTCGGCGGAGGGACCAAACTGACCGTCCTA | CAGGTGCAACTGGTGGAGTCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAGTGGGTGGCAGTTATATCATATGATGGAAGTAATAAATACTATGCAGACTCCGTGAAGGGCCGATTCACCATCTCCAGAGACAATTCCAAGAACACGTTGTATCTGCAAATGAACAGCCTGAGAGCTGAGGACACGGCTGTGTATTACTGTGCGAGAGATGAGGGACGTGGATATGGTGGCTACGAGAGGGGATATTACTACTACTACTACGGTATGGACGTCTGGGGCCAAGGGACCACGGTCACCGTCTCCTCA |
| | | | SEQ ID NO: 27501 | SEQ ID NO: 31507 |
| | | AA | SYELTQPPSVSVSPGQTASITCSGDKLGNKYACWYQQKPGQSPVLVIYQDSKRPSGIPERFSGSNSGNTATLTISGTQAMDEADYYCQAWDSSTVVFGGGTKLTVL | QVQLVESGGGVVQPGRSLRLSCAASGFTFSSYGMHWVRQAPGKGLEWVAVISYDGSNKYYADSVKGRFTISRDNSKNTLYLQMNSLRAEDTAVYYCARDEGRGYGGYERGYYYYYYGMDVWGQGTTVTVSS |
| | | | SEQ ID NO: 27502 | SEQ ID NO: 31508 |

FIGURE 50

| | | | | |
|---|---|---|---|---|
| **iPS:436864** | **21-225_58G11** | NA | TCCTATGAGCTGACTCAGCCACCCTCAGTGTCCGTGTCCCCAGGACAGACAGCCAGCATCACCTGCTCTGGAGATAAATTGGGGGATAAATATGCTTCCTGGTATCAGCAGAAGCCAGGCCAGTCCCCTGTGCTGGTCATCTATCAAGATAACAAGCGGCCCTCAGGGATCCCTGAGCGCTTCTCTGGCTCCAACTCTGGGAACACAGCCACTCTGACCATCAGCGGGACCCAGGCTATGGATGAGGCTGACTATTACTGTCAGGCGTGGAACAACAACACTGTAATGTTCGGCGGAGGGACCAAGCTGACCGTCCTA | GAGGTGCAGCTGGTGGAATCTGGGGGAGGCTTGGTACAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGATTTCATACATTAGTACTAGTAGTAGTACCATATTCTACGCAGACTCTGTGAAGGGCCGATTCACCATCTCCAGAGACAGTGCCAAGAACTCACTGTATCTGCAAATGAACAGCCTGAGAGACGAGGACACGGCTGTGTATTACTGTGCGAGGGGGGATACAGCTATGGTCCTCTACTACTACGGTATGGACGTCTGGGGCCAAGGGACCACGGTCACCGTCTCCTCA |
| | | | SEQ ID NO: 27503 | SEQ ID NO: 31509 |
| | | AA | SYELTQPPSVSVSPGQTASITCSGDKLGDKYASWYQQKPGQSPVLVIYQDNKRPSGIPERFSGSNSGNTATLTISGTQAMDEADYYCQAWNNNTVMFGGGTKLTVL | EVQLVESGGGLVQPGGSLRLSCAASGFTFSSYSMNWVRQAPGKGLEWISYISTSSSTIFYADSVKGRFTISRDSAKNSLYLQMNSLRDEDTAVYYCARGDTAMVLYYYGMDVWGQGTTVTVSS |
| | | | SEQ ID NO: 27504 | SEQ ID NO: 31510 |
| **iPS:436866** | **21-225_59F2** | NA | TCCTATGAACTGACTCAGCCACCCTCAGTGTCCGTGTCCCCAGGACAGACAGCCAGCATCACCTGCTCTGGAGATAAATTGGGGGATAAATATGCTTCTTGGTATCAGCAGAGGCCAGGCCAGTCCCCTGTGTTGGTCATCTATCAAGATAACAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAACAACACTGTGGTCTTCGGCGGAGGGACCAAGCTGACCGTCCTA | GAGGTGCAGCTGGTGGAGTCTGGGGGAGGCTTGGTACAGCCTGGGGGGTCCCTGAGACTCTCCTGTGGAGCCTCTGGATTCACCTTCAGTAGCTATAGCATGAACTGGGTCCGCCAGGCTCCAGGGAAGGGACTGGAGTGGGTTTCATACATTAGTGGGAGTAGTAATATCATATACTACACAGACTCTGTGAAGGGCCGATTCACCATCTCCAGAGACAATGCCAAGAACTCACTGTATCTGCAAATGAACAGCCTGAGAGACGAGGACACGGCTGTGTATTACTGTGCGAGAGCGGATACACCTATGGTCCTTTACTTCTACGGTATGGACGTCTGGGGCCAAGGGACCACGGTCACCGTCTCCTCA |
| | | | SEQ ID NO: 27505 | SEQ ID NO: 31511 |

FIGURE 50

| | | | | |
|---|---|---|---|---|
| | | AA | SYELTQPPSVSVSPGQTASITCSGDKLGDKYASWYQQRPGQSPVLVIYQDNKRPSGIPERFSGSNSGNTATLTISGTQAMDEADYYCQAWDNNTVVFGGGTKLTVL | EVQLVESGGGLVQPGGSLRLSCGASGFTFSSYSMNWVRQAPGKGLEWVSYISGSSNIIYYTDSVKGRFTISRDNAKNSLYLQMNSLRDEDTAVYYCARADTPMVLYFYGMDVWGQGTTVTVSS |
| | | | SEQ ID NO: 27506 | SEQ ID NO: 31512 |
| iPS:436868 | 21-225_59B11 | NA | TCCTATGAGCTGACTCAGCCACCCTCAGTGTCCGTGTCCCCAGGACAGACAGCCAGCATCACCTGCTCTGGAGATAAATTGGGGGATAAATATGCTTGCTGGTATCAGCAGAAGCCAGGCCAGTCCCCTGTGCTGGTCATCTATCAAGATAGCAAGCGGCCCTCAGGGATCCTTGAGCGATTCTCTGGCTCCAATTCTGGGAACACAGCCACTCTGACCATCAGCGGAACCCAGGCTATGGATGAGGCTGACTATTACTGTCAGGCGTGGGACAGCAGCACTTATGTGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGTTATGGCGTGCACTGGGTCCGCCAGGCTCCAGGCAAGGGGCTGGAGTGGGTGGCAGCTATATGGTATGATGGAAGTAATAAATACTATGCAGACTCCGTGAAGGGCCGATTCACCATCTCCAGAGACAATTCCAAGAACACGCTGTATCTGCTAATGAACAGCCTGAGAGCCGAGGACACGGCTGTGTATTACTGTGCGAGAGATAGGGACTATTGTAGTAGTTCCAGCTGCCCTTACTACTACTACTACGGTATGGACGTCTGGGGCCAAGGGACCACGGTCACCGTCTCCTCA |
| | | | SEQ ID NO: 27507 | SEQ ID NO: 31513 |
| | | AA | SYELTQPPSVSVSPGQTASITCSGDKLGDKYACWYQQKPGQSPVLVIYQDSKRPSGILERFSGSNSGNTATLTISGTQAMDEADYYCQAWDSSTYVVFGGGTKLTVL | QVQLVESGGGVVQPGRSLRLSCAASGFTFSSYGVHWVRQAPGKGLEWVAAIWYDGSNKYYADSVKGRFTISRDNSKNTLYLLMNSLRAEDTAVYYCARDRDYCSSSSCPYYYYYGMDVWGQGTTVTVSS |
| | | | SEQ ID NO: 27508 | SEQ ID NO: 31514 |

FIGURE 50

| | | | | |
|---|---|---|---|---|
| iPS:436870 | 21-225_60B1 | NA | TCCTATGAGCTGACTCAGCCACCCTCAGCGTCCGTGTCCCCAGGACAGACAGCCAGCATCACCTGCTCTGGAGATAAACTGGGGGAAAAATATGCTTGCTGGTATCAGCAGAAGCCAGGCCAGTCCCCTGTGTTGGTCATCTATCAAGATAGGAAGCGGCCCTCAGGGATCCCTGAGCGATTCTCTGGCTCCAATTCTGGGAACACAGCCACTCTGACCATCAGCGGGACCCAGGCTATGGATGAGGCTGACTATTACTGTCAGGCGTGGGACAGCAGCACTGTGGTATTCGGCGGAGGGACCAAACTGACCGTCCTA | CAGATCACCTTGAAGGAGTCTGGTCCTACGCTGGTGAAACCCACACAGACCCTCACGCTGACCTGCACCTTCTCTGGGTTCTCACTCAGTACTAGTGGAGTGGGTGTGGGCTGGATCCGTCAGCCCCCAGGAAAGGCCCTGGAGTGGCTTGCACTCATTTATTGGCATGAAGATAAGCGCTACAGCCCATCTCTGAAGAGCAGGCTCACCATCACTGAGGACACCTCCAAAAACCAGGTGGACCTTACAATGACCAACATGGCCCCTGTGGACACAGCCACATATTACTGTGCACACGTCACATATATAGCAGCTCCCTACTGGGGCCAGGGAACCCTGGTCACCGTCTCCTCA |
| | | | SEQ ID NO: 27509 | SEQ ID NO: 31515 |
| | | AA | SYELTQPPSASVSPGQTASITCSGDKLGEKYACWYQQKPGQSPVLVIYQDRKRPSGIPERFSGSNSGNTATLTISGTQAMDEADYYCQAWDSSTVVFGGGTKLTVL | QITLKESGPTLVKPTQTLTLTCTFSGFSLSTSGVGVGWIRQPPGKALEWLALIYWHEDKRYSPSLKSRLTITEDTSKNQVDLTMTNMAPVDTATYYCAHVTYIAAPYWGQGTLVTVSS |
| | | | SEQ ID NO: 27510 | SEQ ID NO: 31516 |
| iPS:436872 | 21-225_60D2 | NA | TCCTATGAACTGACTCAGCCACCCTCAGTGTCCGTGTCCCCAGGACAGACAGCCAGCATCACCTGCTCTGGAAATAAATTGGGGGATAAATATGCTTCTTGGTATCAGCAGAGGCCAGGCCAGTCCCCTGTATTAGTCATCTATCAAGATAACAAGCGGCCCTCAGGGATCCCTGAGCGATTCTCTGGCTCCAACTCTGGAAACACAGCCACTCTGACCATCAGCGGGACCCAGACTATGGATGAGGCTGACTATTACTGTCAGGCGTGGGACAACAACACTGTGGTCTTCGGCGGAGGGACCAAGCTGACCGTCCTA | GAGGTGCAGCTGGTGGAGTCTGGGGGAGGCTTGGTACAGCCTGGGGGGTCCCTGAGACTCTCCTGTGGAGCCTCTGGATTCACCTTCAGTAGCTATAGCATGAACTGGGTCCGCCAGGCTCCAGGAAAGGGACTGGAGTGGGTTTCATACATTAGTGAGAGTAGTAATATCATATACTACACAGACTCTGTGAAGGGCCGATTCACCATCTCCAGAGACAATGCCATGAACTCACTGTATCTGCAAATGAACAGCCTGAGAGACGAGGACACGGCTGTGTATTACTGTGCGAGAGCGGATACACCTATGGTCCTTTACTTCTACGGTATGGACGTCTGGGGCCAAGGGACCACGGTCACCGTCTCCTCA |
| | | | SEQ ID NO: 27511 | SEQ ID NO: 31517 |

FIGURE 50

| | | | | |
|---|---|---|---|---|
| | | AA | SYELTQPPSVSVSPGQTASITCSGNKLGDKYASWYQQRPGQSPVLVIYQDNKRPSGIPERFSGSNSGNTATLTISGTQTMDEADYYCQAWDNNTVVFGGGTKLTVL | EVQLVESGGGLVQPGGSLRLSCGASGFTFSSYSMNWVRQAPGKGLEWVSYISESSNIIYYTDSVKGRFTISRDNAMNSLYLQMNSLRDEDTAVYYCARADTPMVLYFYGMDVWGQGTTVTVSS |
| | | | SEQ ID NO: 27512 | SEQ ID NO: 31518 |
| iPS:436874 | 21-225_60A12 | NA | TCCTATGAGCTGACTCAGCCACCCTCAGTGTCCGTGTCCCCAGGACAGACAGCCAACATCACCTGCTCTGGAGATAAATTGGGGAATAAATATGCTTGCTGGTATCAGCAGAAGCCAGGCCAGTCCCCTGTATTGGTCATTTATCAAGATAGGAAGCGGCCCTCAGGGATCCCTGAGCGATTCTCTGGCTCCAACTCTGGGAACACAGCCATTCTGACCATCAGCGGGACCCAGGCTATGGATGAGGCTGACTATTACTGTCAGGCGTGGGACAGCAGCACTGCTTTCGGCGGAGGGACCAAGCTGACCGTCCTC | CAGATCACCTTGAAGGAGTCTGGTCCTACGCTGGTGAAACCCACACAGACCCTCACGCTGACCTGCACCTTCTCTGGGTTCTCACTCAGCACTAGTGGAGTGGGTGTGGGCTGGATCCGTCAGCCCCCAGGAAAGGCCCTGGAGTGGCTTGCACTCATTTATTGGGATGATGATAAGCGCTACAGCCCATCTCTGAAGAGCAGGCTCACCATCACCGAGGACACCTCCAAAAACCAGGTGGTCCTTACAATGACCAACATGGACCCTGTGGACACAGCCACATATTACTGTGCACACCTTATAGCAGTGGCCTTTGACTCCTGGGGCCAGGGAACCCTGGTCACCGTCTCCTCA |
| | | | SEQ ID NO: 27513 | SEQ ID NO: 31519 |
| | | AA | SYELTQPPSVSVSPGQTANITCSGDKLGNKYACWYQQKPGQSPVLVIYQDRKRPSGIPERFSGSNSGNTAILTISGTQAMDEADYYCQAWDSSTAFGGGTKLTVL | QITLKESGPTLVKPTQTLTLTCTFSGFSLSTSGVGVGWIRQPPGKALEWLALIYWDDDKRYSPSLKSRLTITEDTSKNQVVLTMTNMDPVDTATYYCAHLIAVAFDSWGQGTLVTVSS |
| | | | SEQ ID NO: 27514 | SEQ ID NO: 31520 |

FIGURE 50

| | | | | |
|---|---|---|---|---|
| iPS:436876 | 21-225_61F5 | NA | TCCTATGAGCTGACTCAGCCACCCTCAGTGTCCGTGTCCCCAGGACAGACAGCCAGCATCACCTGCTCTGGAGATAAATTGGGGGAAAAATATGCTTGCTGGTATCAGCAGAAGCCAGGCCAGTCCCCTGTGTTGGTCATCTATCAAGATAGCAAGCGGCCCTCAGGGATCCCTGAGCGATTCTCTGGCTCCAATTCTGGGAACACAGCCACTCTGACCATCAGCGGGACCCAGGCTATGGATGAGGCTGACTATTACTGTCAGGCGTGGGACAGCAGCACTGTGGTTTTCGGCGGAGGGACCAAACTGACCGTCCTA | CAGATCACCTTGAAGGAGTCTGGTCCTACGCTGGTGAAACCCACACAGACCCTCACGCTGACCTGCACCTTCTCTGGGTTCTCACTCAGTACTAGTGGGTTGGGTGTGGGCTGGATCCGTCAGCCCCCAGGAAAGGCCCTGGAGTGGCTTGCACTCATTTATTCACATGAAGATAAGCGCTACAGCCCATCTCTGAAGAGCAGGCTCACCATCACTGAGGACACCTCCAAAAACCAGGTGGACCTTACAATGACCAACATGGCCCCTGTGGACACAGCCACATATTACTGTGCACACGTCACAGGTATAGCAGCTCCCTACTGGGGCCAGGGAACCCTGGTCACCGTCTCCTCA |
| | | | SEQ ID NO: 27515 | SEQ ID NO: 31521 |
| | | AA | SYELTQPPSVSVSPGQTASITCSGDKLGEKYACWYQQKPGQSPVLVIYQDSKRPSGIPERFSGSNSGNTATLTISGTQAMDEADYYCQAWDSSTVVFGGGTKLTVL | QITLKESGPTLVKPTQTLTLTCTFSGFSLSTSGLGVGWIRQPPGKALEWLALIYSHEDKRYSPSLKSRLTITEDTSKNQVDLTMTNMAPVDTATYYCAHVTGIAAPYWGQGTLVTVSS |
| | | | SEQ ID NO: 27516 | SEQ ID NO: 31522 |
| iPS:436878 | 21-225_62E3 | NA | TCCTATGACCTGACTCAGCCACCCTCAGTGTCCGTGTCCCCAGGACAGACAGCCAGCATCACCTGCTCTGGAGATAAATTGGGGAATAAATATGCTTGCTGGTATCAGCAGAAGCCAGGCCAGTCCCCTGTGGTGGTCATCTATCAAGATAGGAAGCGGCCCTCAGGGATCCCTGAGCGATTCTCTGGCTCCAATTCTGGGAACACAGCCACTCTGACCATCAGCGGGACCCAGGCTATGGATGAGGCTGACTATTACTGTCAGGCGTGGGACAGCAGCACTGCGGTATTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGGTTCTCGCTCAGCACTAGTGGAGTGGGTGTGGGCTGGATCCGTCAGCCCCCAGGAAAGGCCCTGGAGTGGCTTGCACTCATTAATTGGAATGATGATAAGCGCTACAGCCCATCTCTGAAGAGCAGGTTCACCATCACCAGGGACACCTCCAAAGACCAGGTGGTCCTTACAATGACCAACATGGACCCTGTGGACACAGCCACATATTACTGTGCACATAAAGCTACCTGGGTGGCTTTTGATATCTGGGGCCAAGGGACAATGGTCACCGTCTCTTCA |
| | | | SEQ ID NO: 27517 | SEQ ID NO: 31523 |

FIGURE 50

| | | | | |
|---|---|---|---|---|
| | | AA | SYDLTQPPSVSVSPGQTASITCSGDKLGNKYACWYQQKPGQSPVVVIYQDRKRPSGIPERFSGSNSGNTATLTISGTQAMDEADYYCQAWDSSTAVFGGGTKLTVL | QITLKESGPTLVKPTQTLTLTCTFSGFSLSTSGVGVGWIRQPPGKALEWLALINWNDDKRYSPSLKSRFTITRDTSKDQVVLTMTNMDPVDTATYYCAHKATWVAFDIWGQGTMVTVSS |
| | | | SEQ ID NO: 27518 | SEQ ID NO: 31524 |
| **iPS:436880** | **21-225_62E8** | NA | TCCTATGACCTGACTCAGCCACCCTCAGTGTCCGTGTCCCCAGGACAGACAGCCAGCATCACCTGCTCTGGAGATAGATTGGGGAATAAATATGCTTCCTGGTATCAGCAGAAGCCAGGCCAGTCCCCTGTGGTGGTCATCTATCAGGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTGCGGTATTCGGCGGAGGGACCAAGCTGACCGTCCTA | CAGATCACCTTGAAGGAGTCTGGTCCTACGCTGGTGAAACCCACACAGACCCTCACGCTGACCTGTACCTTCTCTGGGTTCTCACTCAGCACTAGTGGAGTGGGTGTGGGCTGGATCCGTCAGCCCCCAGGAAAGGCCCTGGAGTGGCTTGCACTCATTAATTGGAATGATGATAAGCGCTACAGCCCATCTCTGAAGAGCAGGTTCACCATCACCAGGGACACCTCCAAAGACCAGGTGGTCCTTACAATGACCAACATGGACCCTGTGGACACAGCCACATATTACTGTGCACATAAAGCTACCTGGGTGGCTTTTGATATCTGGGGCCAAGGGACAATGGTCACCGTCTCTTCA |
| | | | SEQ ID NO: 27519 | SEQ ID NO: 31525 |
| | | AA | SYDLTQPPSVSVSPGQTASITCSGDRLGNKYASWYQQKPGQSPVVVIYQDRKRPSGIPERFSGSNSGNTATLTISGTQAMDEADYYCQAWDSSTAVFGGGTKLTVL | QITLKESGPTLVKPTQTLTLTCTFSGFSLSTSGVGVGWIRQPPGKALEWLALINWNDDKRYSPSLKSRFTITRDTSKDQVVLTMTNMDPVDTATYYCAHKATWVAFDIWGQGTMVTVSS |
| | | | SEQ ID NO: 27520 | SEQ ID NO: 31526 |

FIGURE 50

| | | | | |
|---|---|---|---|---|
| iPS:436882 | 21-225_62D10 | NA | TCCTATGACCTGACTCAGCCACCCTCAGTGTCCGTGTCCCCAGGACAGACAGCCAGCATCACCTGCTCTGGAGATAAATTGGGGAATAAATATGCTTGCTGGTATCAGCAGAAGCCAGGCCAGTCCCCTGTGGTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTGCGGTATTCGGCGGAGGGACCAAGCTGACCGTCCTA | CAGATCACCTTGAAGGAGTCTGGTCCTACGCTGGTGAAATCCACACAGACCCTCACGCTGACCTGCACCTTCTCTGGGTTCTCACTCAGCACTAGTGGAGTGGGTGTGGGCTGGATCCGTCAGCCCCCAGGAAAGGCCCTGGAGTGGCTTGCACTCATTAATTGGAATGATGATAAGCGCTACAGCCCATCTCTGAAGAGCAGGTTCACCATCACCAGGGACACCTCCAAAGACCAGGTGGTCCTTACAATGACCAACATGGACCCTGTGGACACAGCCACATATTACTGTGCACATAAAGCTACCTGGGTGGCTTTTGATATCTGGGGCCAAGGGACAATGGTCACCGTCTCTTCA |
| | | | SEQ ID NO: 27521 | SEQ ID NO: 31527 |
| | | AA | SYDLTQPPSVSVSPGQTASITCSGDKLGNKYACWYQQKPGQSPVVVIYQDRKRPSGIPERFSGSNSGNTATLTISGTQAMDEADYYCQAWDSSTAVFGGGTKLTVL | QITLKESGPTLVKSTQTLTLTCTFSGFSLSTSGVGVGWIRQPPGKALEWLALINWNDDKRYSPSLKSRFTITRDTSKDQVVLTMTNMDPVDTATYYCAHKATWVAFDIWGQGTMVTVSS |
| | | | SEQ ID NO: 27522 | SEQ ID NO: 31528 |
| iPS:436884 | 21-225_62A12 | NA | TCCTATGACCTGACTCAGCCACCCTCAGTGTCCGTGTCCCCAGGACAGACAGCCAGCATCACCTGCTCTGGAGATAAATTGGGGAATAAATATGCTTGCTGGTATCAGCAGAAGCCAGGCCAGTCCCCTGTGGTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTGCGGTATTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGGTTCTCACTCAGCACTAGTGGAGTGGGTGTGGGCTGGATCCGTCAGCCCCCAGGAAAGGCCCTGGAGTGGCTTGCACTCATTAATTGGAATGATGATAAACGCTACAGCCCATCTCTGAAGAGCAGGTTCACCATCACCAGGGACACCTCCAAAGACCAGGTGGTCCTTACAATGACCAACATGGACCCTCTGGACACAGCCACATATTACTGTGCACATAAAACTACCTGGGTGGCTTTTGATATCTGGGGCCAAGGGACAATGGTCACCGTCTCTTCA |
| | | | SEQ ID NO: 27523 | SEQ ID NO: 31529 |

FIGURE 50

| | | | | |
|---|---|---|---|---|
| | | AA | SYDLTQPPSVSVSPGQTASITCSGDKLGNKYACWYQQKPGQSPVVVIYQDRKRPSGIPERFSGSNSGNTATLTISGTQAMDEADYYCQAWDSSTAVFGGGTKLTVL | QITLKESGPTLVKPTQTLTLTCTFSGFSLSTSGVGVGWIRQPPGKALEWLALINWNDDKRYSPSLKSRFTITRDTSKDQVVLTMTNMDPLDTATYYCAHKTTWVAFDIWGQGTMVTVSS |
| | | | SEQ ID NO: 27524 | SEQ ID NO: 31530 |
| iPS:436886 | 21-225_62B12 | NA | TCCTATGACCTGACTCAGCCACCCTCAGTGTCCGTGTCCCCAGGACAGACAGCCAGCATCACCTGCTCTGGAGATAAATTGGGGAATAAATATACTTGCTGGTATCAGCAGAAGCCAGGCCAGTCCCCTGTGGTGGTCATCTATCAAGATAGGAAGCGGCCCTCAGGGATCCCTGAGCGATTCTCTGGCTCCAATTCTGGGAACACAGCCACTCTGACCATCAGCGGGACCCAGGCTATGGATGAGGCTGACTATTACTGTCAGGCGTGGGACAGCAGCACTGCGGTATTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGGTTCTCACTCAACACTAGTGGAGTGGGTGTGGGCTGGATCCGTCAGCCCCCAGGAAAGGCCCTGGAGTGGCTTGCACTCATTAATTGGAATGATGATAAGCGCTACAGCCCGTCTCTGAAGAGCAGGTTCACCATCACCAGGGACACCTCCAAAGACCAGGTGGTCCTTACAATGACCAACATGGACCCTGTGGACACAGCCACATATTACTGTGCACATAAAGCTACCTGGGTGGCTTTTGATATCTGGGGCCAAGGGACAATGGTCACCGTCTCTTCA |
| | | | SEQ ID NO: 27525 | SEQ ID NO: 31531 |
| | | AA | SYDLTQPPSVSVSPGQTASITCSGDKLGNKYTCWYQQKPGQSPVVVIYQDRKRPSGIPERFSGSNSGNTATLTISGTQAMDEADYYCQAWDSSTAVFGGGTKLTVL | QITLKESGPTLVKPTQTLTLTCTFSGFSLNTSGVGVGWIRQPPGKALEWLALINWNDDKRYSPSLKSRFTITRDTSKDQVVLTMTNMDPVDTATYYCAHKATWVAFDIWGQGTMVTVSS |
| | | | SEQ ID NO: 27526 | SEQ ID NO: 31532 |

FIGURE 50

| | | | | |
|---|---|---|---|---|
| **iPS:436888** | **21-225_63G7** | NA | AATTTTATGCTGACTCAGCCCCACTCTGTGTCGGAGTCTCCGGGGAAGACGGTAACCATCTCCTGCACCCGCAGCAATGGCAGCATTGTCAGCAACTATGTGCAGTGGTACCAGCAGCGCCCGGGCAGTTCCCCCACCACTATGATCTATGAGGATAGCCGAAGACCCTCTGGGGTCCCTGATCGGTTCTCTGGCTCCATCGACAGCTCCTCCAACTCTGCCTCCCTCACCATCTCTGGACTGAAGACTGAGGACGAGGCTGACTACTCCTGTCAGTCTTATGATGGCATCAATGTGGTATTCGGCGGAGGGACCAAGCTGACCGTCCTA | GAGGTGCAGCTGGTGGAGTCTGGGGGAGGCTTGGTACAGCCTGGGGGGTCCCTGAGACTCTCCTGTGCAGCCTCTGGATTCACCTTCAGTAGCTATAGCATGAACTGGGTCCGCCAGGCTCCGGGGAAGGGGCTGGAGTGGGTTTCATACATTAGTAGTAGTACTAGTACCATATACTACGCAGCCTCTGTGAAGGGCCGATTCACCATCTCCAGAGACAATGCCAAGAACTCACTGTATCTGCAAATGAACAGCCTGAGAGACGAGGACACGGCTGTGTATTACTGTGCGAGAGATCACCGTTACTATGATAGTAGTGGTTATTACTCTGATGCTTTTGATATCTGGGGCCAAGGGACAATGGTCACCGTCTCTTCA |
| | | | SEQ ID NO: 27527 | SEQ ID NO: 31533 |
| | | AA | NFMLTQPHSVSESPGKTVTISCTRSNGSIVSNYVQWYQQRPGSSPTTMIYEDSRRPSGVPDRFSGSIDSSSNSASLTISGLKTEDEADYSCQSYDGINVVFGGGTKLTVL | EVQLVESGGGLVQPGGSLRLSCAASGFTFSSYSMNWVRQAPGKGLEWVSYISSSTSTIYYAASVKGRFTISRDNAKNSLYLQMNSLRDEDTAVYYCARDHRYYDSSGYYSDAFDIWGQGTMVTVSS |
| | | | SEQ ID NO: 27528 | SEQ ID NO: 31534 |
| **iPS:436890** | **21-225_63A10** | NA | AATTTTATGCTGACTCAGCCCCACTCTGTGTCGGAGTCTCCGGGGAAGACGGTAACCATCTCCTGCACCCGCAGCAATGGCAGCATTGTCAGCAACTATGTGCAGTGGTACCAGCAGCGCCCGGGCAGTTCCCCCACCACTGTGATCTATGAGGATAAAAGAAGACCCTCAGGGGTCCCTGATCGGTTCTCTGGCTCCATCGACAGCTCCTCCAACTCTGCCTCCCTCACCATCTCTGGACTGAAGACTGAGGACGAGGCTGACTACTACTGTCAGTCTTATGATAGCATCAATGTGGTATTCGGCGGAGGGACCAAGCTGACCGTCCTA | GAGGTGCAGCTGGTGGAGTCTGGGGGAGGCTTGGTACAGCCTGGGGGGTCCCTGAGACTCTCCTGTGCAGCCTCTGGATTCACCTTCAGTAGCTATAGCATGAACTGGGTCCGCCAGGCTCCGGGGAAGGGGCTGGAGTGGGTTTCATACATTAGTAGTAGTACTAGTACCATATACTACGCAGCCTCTGTGAAGGGCCGATTCACCATCTCCAGAGACAATGCCAAGAATTCACTGTATCTGCAAATGAACAGCCTGAGAGACGAGGACACGGCTGTGTATTACTGTGCGAGAGATCACCGTTACTATGATAGTAGTGGTTATTACTCTGATGCTTTTGATATCTGGGGCCAAGGGACAATGGTCACCGTCTCTTCA |
| | | | SEQ ID NO: 27529 | SEQ ID NO: 31535 |

FIGURE 50

| | | | | |
|---|---|---|---|---|
| | | AA | NFMLTQPHSVSESPGKTVTISCTRSNGSIVSNYVQWYQQRPGSSPTTVIYEDKRRPSGVPDRFSGSIDSSSNSASLTISGLKTEDEADYYCQSYDSINVVFGGGTKLTVL | EVQLVESGGGLVQPGGSLRLSCAASGFTFSSYSMNWVRQAPGKGLEWVSYISSSTSTIYYAASVKGRFTISRDNAKNSLYLQMNSLRDEDTAVYYCARDHRYYDSSGYYSDAFDIWGQGTMVTVSS |
| | | | SEQ ID NO: 27530 | SEQ ID NO: 31536 |
| iPS:436892 | 21-225_65E9 | NA | TCCTATGAGCTGACTCAGCCACCCTCAGTGTCCGTGTCCCCAGGACAGACAGCCAGCATCACCTGCTCTGGAGATAAATTGGGGAATAAATATGATTACTGGTATCAGCAGAAGCCAGGCCAGTCCCCTGTGCTGGTCATCTATCAAGATAGAAAGCGGCCCTCAGGGATCCCTGAGCGATTCTCTGGCTCCAACTCTGGGAACACAGCCACCCTGACCATCAGCGGGACCCAGGCTATGGATGAGGCTGACTATTACTGTCAGGCGTGGGACAACAGCACTGTGGTATTCGGCGGAGGGACCAAACTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAACAGTGGTGGCACAAATTATGCACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGAGCGTATTATTATGGTTCGGGGAGTTATTATAATGAATTTGATATGTGGGGCCAAGGGACAATGGTCACCGTCTCTTCA |
| | | | SEQ ID NO: 27531 | SEQ ID NO: 31537 |
| | | AA | SYELTQPPSVSVSPGQTASITCSGDKLGNKYDYWYQQKPGQSPVLVIYQDRKRPSGIPERFSGSNSGNTATLTISGTQAMDEADYYCQAWDNSTVVFGGGTKLTVL | QVQLVQSGAEVKKPGASVKVSCKASGYTFTGYYMHWVRQAPGQGLEWMGWINPNSGGTNYAQKFQGRVTMTRDTSISTAYMELSRLRSDDTAVYYCARAYYYGSGSYYNEFDMWGQGTMVTVSS |
| | | | SEQ ID NO: 27532 | SEQ ID NO: 31538 |

FIGURE 50

| | | | | |
|---|---|---|---|---|
| **iPS:436894** | **21-225_66G9** | NA | TCCTATGACCTGACTCAGCCACCCTCAGTGACCGTGTCCCCAGGACAGACAGCCAGCATCACCTGCTCTGGAGATAAATTGGGGAATAAATATGCTTGCTGGTATCAGCAGAAGCCAGGCCAGTCCCCTGTGGTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACATCAACACTGCGGTATTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGGTTCTCACTCAGCACTAGTGGAGTGGGTGTGGGCTGGATCCGTCAGCCCCCAGGAAAGGCCCTGGAGTGGCTTGCACTCATTAATTGGAATGATGATAAGCGCTTCAGCCCATCTCTGAAGAGCAGGTTCACCATCACCAGGGACACCTCCAAAGACCAGGTGGTCCTTACAATGACCAACATGGACCCTGTGGACACAGCCACATATTACTGTGCACATAAAGCTACCTGGGTGGCTTTTGATATCTGGGGCCAAGGGACAATGGTCACCGTCTCTTCA |
| | | | SEQ ID NO: 27533 | SEQ ID NO: 31539 |
| | | AA | SYDLTQPPSVTVSPGQTASITCSGDKLGNKYACWYQQKPGQSPVVVIYQDRKRPSGIPERFSGSNSGNTATLTISGTQAMDEADYYCQAWDINTAVFGGGTKLTVL | QITLKESGPTLVKPTQTLTLTCTFSGFSLSTSGVGVGWIRQPPGKALEWLALINWNDDKRFSPSLKSRFTITRDTSKDQVVLTMTNMDPVDTATYYCAHKATWVAFDIWGQGTMVTVSS |
| | | | SEQ ID NO: 27534 | SEQ ID NO: 31540 |
| **iPS:436896** | **21-225_67F10** | NA | TCCTATGAACTGACTCAGCCACCCTCAGTTTCCGTGTCCCCAGGACAGACAGCCAGCATCACCTGCTCTGGAGATAAATTGGGGTATAAATATGCTTGGTGGTATCAGCAGAAGCCAGGCCAGTCCCCTGTGCTGGTCATCTTTGAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAACAGCACTGTGGTATTCGGCGGAGGGACCAGGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAACAGTGGTGGCACAAACTATGGACAGAAGTTTCAGGGCAGGGTCACCATGACCAGGGACACGTCCATCAGCACAGCCTACATGGAGCTGAGCAGGCTGAGATCTGACGACACGGCCGTATATTACTGTGCGAGAACGTATTTCTATGGTTCGGGGAGTTATTATAACGGCTTTGACTACTGGGGCCAGGGAACCCTGGTCACCGTCTCCTCA |
| | | | SEQ ID NO: 27535 | SEQ ID NO: 31541 |

FIGURE 50

| | | | | |
|---|---|---|---|---|
| | | AA | SYELTQPPSVSVSPGQTASITCSGDKLGYKYAWWYQQKPGQSPVLVIFEDRKRPSGIPERFSGSNSGNTATLTISGTQAMDEADYYCQAWDNSTVVFGGGTRLTVL | QVQLVQSGAEVKKPGASVKVSCKASGYTFTGYYMHWVRQAPGQGLEWMGWINPNSGGTNYGQKFQGRVTMTRDTSISTAYMELSRLRSDDTAVYYCARTYFYGSGSYYNGFDYWGQGTLVTVSS |
| | | | SEQ ID NO: 27536 | SEQ ID NO: 31542 |
| iPS:436898 | 21-225_68D8 | NA | TCCTATGAGCTGACTCAGCCACCCTCAGTGTCCGTGTCCCCAGGACAGACAGCCAGCATCACCTGCTCTGGAGATAAATTGGGGGATAAATATGCTTGCTGGTATCAGCAGAAGCCAGGCCAGTCCCCTGTGTTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAACAGCACTGTGGTATTCGGCGGAGGGACCAAGCTGACCGTCCTA | CAGGTACAGCTGCAGCAGTCAGGTCCAGGACTGGTGAAGCCCTCGCAGACCCTCTCACTCACCTGTGCCATCTCCGGGGACAGTGTCTCTAGCAACAGTGCTGCTTGGAACTGGATCAGGCAGTCCCCATCGAGAGGCCTTGAGTGGCTGGGAAGGACATACTACAGGTCCGAGTGCTATAATGATTATGCAGTATCTGTGCAGAGTCGAATAACCATCAACCCAGACACATCCAAGAACCAGTTCTCCCTGCACCTGAACTCTGTGACTCCCGAGGACACGGCTGTGTATTTCTGTGCAAGAGATAGAGGGCATAGAGGGTTCTACGGTATGGACGTCTGGGGCCAAGGGACCACGGTCACCGTCTCCTCA |
| | | | SEQ ID NO: 27537 | SEQ ID NO: 31543 |
| | | AA | SYELTQPPSVSVSPGQTASITCSGDKLGDKYACWYQQKPGQSPVLVIYQDSKRPSGIPERFSGSNSGNTATLTISGTQAMDEADYYCQAWDNSTVVFGGGTKLTVL | QVQLQQSGPGLVKPSQTLSLTCAISGDSVSSNSAAWNWIRQSPSRGLEWLGRTYYRSECYNDYAVSVQSRITINPDTSKNQFSLHLNSVTPEDTAVYFCARDRGHRGFYGMDVWGQGTTVTVSS |
| | | | SEQ ID NO: 27538 | SEQ ID NO: 31544 |

FIGURE 50

| | | | | |
|---|---|---|---|---|
| **iPS:436900** | **21-225_69B9** | NA | TCCTATGAGCTGACTCAGCCACCCTCAGTGTCCGTGTCCCCAGGACAGACAGCCAGCATCACCTGCTCTGGAGATAAATTGGGGAATAAATATGATTACTGGTATCAGCAGAAGCCAGGCCAGTCCCCTGTGCTGGTCATCTATCAAGATAGAAAGCGGCCCTCAGGGATCCCTGAGCGATTCTCTGGCTCCAACTCTGGGAACACAGCCACCCTGACCATCAGCGGGACCCAGGCTATGGATGAGGCTGACTATTACTGTCAGGCGTGGGACAACAGCACTGTGGTATTCGGCGGAGGGACCAAACTGACCGTCCTA | CAGGTGCAGATGGTGCAGTCTGGGGATGAGGTGAAGAAGCCTGGGGCCTCAGTGAAGGTCTCGTGCAAGGCTTCGGGATACACCTTCACCGGCTACCATATGCACTGGGTGCGACAGGCCCCTGGACAAGGGCTTGAGTGGATGGGATGGATCAACCCTAACAGTGGTGGCACAAATTATGCACAGAAGTTTCAGGGCAGGGTCACCATGACCAGGGACACGTCCATCAGCACAGCCTACATGGAGCTGAGCAGGATGAGATCTGACGACACGGCCGTGTATTACTGTGCGAGAGCGTATTATTATGGTTCGGGGAGTTATTATAATGAATCTGATATGTGGGGCCAAGGGACAATGGTCACCGTCTCTTCA |
| | | | SEQ ID NO: 27539 | SEQ ID NO: 31545 |
| | | AA | SYELTQPPSVSVSPGQTASITCSGDKLGNKYDYWYQQKPGQSPVLVIYQDRKRPSGIPERFSGSNSGNTATLTISGTQAMDEADYYCQAWDNSTVVFGGGTKLTVL | QVQMVQSGDEVKKPGASVKVSCKASGYTFTGYHMHWVRQAPGQGLEWMGWINPNSGGTNYAQKFQGRVTMTRDTSISTAYMELSRMRSDDTAVYYCARAYYYGSGSYYNESDMWGQGTMVTVSS |
| | | | SEQ ID NO: 27540 | SEQ ID NO: 31546 |
| **iPS:436902** | **21-225_69B11** | NA | TCCTATGAGCTGACTCAGCCACCCTCAGTGTCCGTGTCCCCAGGACAGGCAGCCAGCATCACCTGCTCTGGAGATAAATTGGGGGATAAATATGCTTGGTGGTATCAGCAGAAGCCAGGCCAGTCCCCTGTGCTGGTCATCTATGAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAACAGCACTGTGGTATTCGGCGGAGGGACCAAGCTGACCGTCCTA | CAGGTGCAGCTGGTGCAGTCCGGGGCTGAGGTGAAGAAGCCTGGGGCCTCAGTGAGGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAACAGTGGTGGCACAAACTATGGACAGAAGTTTCAGGACAGGGTCACCATGACCAGGGACACGTCCATCAGCACAGCCTTCATGGAGCTGAGCAGGCTGAGATCTGACGACACGGCCGCATATTACTGTGCGAGAACGTATTACTATGGGTCGGGGAGTTATTATAACGGCTTTGACTACTGGGGCCAGGGAACCCTGGTCACCGTCTCCTCA |
| | | | SEQ ID NO: 27541 | SEQ ID NO: 31547 |

FIGURE 50

| | | | | |
|---|---|---|---|---|
| | | AA | SYELTQPPSVSVSPGQAASITCSGDKLGDKYAWWYQQKPGQSPVLVIYEDRKRPSGIPERFSGSNSGNTATLTISGTQAMDEADYYCQAWDNSTVVFGGGTKLTVL | QVQLVQSGAEVKKPGASVRVSCKASGYTFTGYYMHWVRQAPGQGLEWMGWINPNSGGTNYGQKFQDRVTMTRDTSISTAFMELSRLRSDDTAAYYCARTYYYGSGSYYNGFDYWGQGTLVTVSS |
| | | | SEQ ID NO: 27542 | SEQ ID NO: 31548 |
| iPS:436904 | 21-225_71D4 | NA | TCCTATGAGCTGACTCAGCCACCCTCAGTGTCCGTGTCTCCAGGACAGACAGCCAGCATCACCTGCTCTGGAGATAAATTGGGGGATAAATATGCTTACTGGTATCAGCAGAAACCAGGCCAGTCCCCTGTGGTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGTCAACAGCACTGTGGTATTCGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTGTATGCACTGGGTGCGACAGGCCCCTGGACAAGGGCTTGAGTGGATGGGATGGATCAACCCTAACAGTGGTGGCACAAACTATGCACAGAAGTTTCAGGTCAGGGTCACCATGACCAGGGACACGTCCGTCAGCACAGTCTACATGGACCTGAGCAGGCTGAGATCTGACGACACGGCCGTGTATTACTGTGCGAGAGCGTATTACTATGGTTCGGGGACTTATCATAACGAATTTGACTACTGGGGCCAGGGAAGTTTGGTCACCGTCTCCTCA |
| | | | SEQ ID NO: 27543 | SEQ ID NO: 31549 |
| | | AA | SYELTQPPSVSVSPGQTASITCSGDKLGDKYAYWYQQKPGQSPVVVIYQDRKRPSGIPERFSGSNSGNTATLTISGTQAMDEADYYCQAWVNSTVVFGGGTKLTVL | QVQLVQSGAEVKKPGASVKVSCKASGYTFTGYCMHWVRQAPGQGLEWMGWINPNSGGTNYAQKFQVRVTMTRDTSVSTVYMDLSRLRSDDTAVYYCARAYYYGSGTYHNEFDYWGQGSLVTVSS |
| | | | SEQ ID NO: 27544 | SEQ ID NO: 31550 |

FIGURE 50

| | | | | |
|---|---|---|---|---|
| **iPS:436906** | **21-225_72B4** | NA | TCCTATGAGCTGACTCAGCCACCCTCAGTGTCCGTGTCCCCAGGACAGACAGCCAGCATCACCTGCTCTGGAGATAAATTGGGGGATAAATATGCTTGGTGGTATCAGCAGAGGCCAGGCCAGTCCCCTGTGCTGGTCATCTATGAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAACAGCACTGTGGTATTCGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAACAGTGGTGGCACAAACTATGGACAGAAGTTTCAGGGCAGGGTCACCATGACCAGGGACACGTCCATCAGCACAGCCTACATGGAGCTGAGCAGGCTGAGATCTGACGACACGGCCGTATATTACTGTGCGAGAACGTATTACTATGGTTCGGGGAGTTATTATAACGGCTTTGACTACTGGGGCCAGGGAACCCTGGTCACCGTCTCCTCA |
| | | | SEQ ID NO: 27545 | SEQ ID NO: 31551 |
| | | AA | SYELTQPPSVSVSPGQTASITCSGDKLGDKYAWWYQQRPGQSPVLVIYEDRKRPSGIPERFSGSNSGNTATLTISGTQAMDEADYYCQAWDNSTVVFGGGTKLTVL | QVQLVQSGAEVKKPGASVKVSCKASGYTFTGYYMHWVRQAPGQGLEWMGWINPNSGGTNYGQKFQGRVTMTRDTSISTAYMELSRLRSDDTAVYYCARTYYYGSGSYYNGFDYWGQGTLVTVSS |
| | | | SEQ ID NO: 27546 | SEQ ID NO: 31552 |
| **iPS:436908** | **21-225_72D5** | NA | TCCTATGACCTGACTCAGCCACCCTCAGTGTCCGTGTCCCCAGGACAGACAGCCAGCATCACCTGCTCTGGAGATAAATTGGGGAATAAATATGCTTGCTGGTATCAGCAGAAGCCAGGCCAGTCCCCTGTGGTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTGCGGTATTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGGTTCTCACTCAGCACTAGTGGAGTGGGTGTGGGCTGGATCCGTCAGCCCCCAGGAAAGGCCCTGGAGTGGCTTGCACTCATTAATTGGAATGATGATAAGCGCTACAGCCCATCTCTGAAGAGCAGGTTCACCATCACCAGGGACACCTCCAAAGACCAGGTGGTCTTTACAATGACCAACATGGACCCTGTGGACACAGGCACATATTACTGTGCACATAAAGCTACCTGGGTGGCTTTTGATATCTGGGGCCAAGGGACAATGGTCACCGTCTCTTCA |
| | | | SEQ ID NO: 27547 | SEQ ID NO: 31553 |

FIGURE 50

| | | | | |
|---|---|---|---|---|
| | | AA | SYDLTQPPSVSVSPGQTASITCSGDKLGNKYACWYQQKPGQSPVVVIYQDRKRPSGIPERFSGSNSGNTATLTISGTQAMDEADYYCQAWDSSTAVFGGGTKLTVL | QITLKESGPTLVKPTQTLTLTCTFSGFSLSTSGVGVGWIRQPPGKALEWLALINWNDDKRYSPSLKSRFTITRDTSKDQVVFTMTNMDPVDTGTYYCAHKATWVAFDIWGQGTMVTVSS |
| | | | SEQ ID NO: 27548 | SEQ ID NO: 31554 |
| **iPS:436910** | **21-225_73G1** | NA | CAGACTGTGGTGACCCAGGAGCCATCGTTCTCAGTGTCCCCTGGAGGGACAGTCACACTCACTTGTGGCTTGAGCTCTGGCTCAGTCTCTACTAGTTACTACCCCAGCTGGTACCAGCAGACCCCAGGCCAGGCTCCACGCACGCTCATCTACAACACAAACACTCGCTCTTCTGGGGTCCCTGATCGCTTCTCTGGCTCCATCCTTGGGAACAAAGCTGCCCTCACCATCACGGGGGCCCAGGCAGATGATGAATCTGATTATTACTGTGTTCTATATATGGGTAGTGCCATTTGGGTGTTCGGCGGAGGGACCAAGTTGACCGTCCTA | CAGGTGCAGCTGGTGGAGTCTGGGGGAGGCGTGGTCCAGCCTGGGAGGTCCCTGAGACTCTCCTGTGCAGGCACTGGATTCACCTTCAGTTACTATGGCATGCACTGGGTCCGCCAGGCTCCAGGCAAGGGGCTGGAGTGGGTGGCAGTTACAACATATGATGGAAGTAATAAATACTATGCAGACTCCGTGAAGGGCCGATTCACCAGCTCCAGAGACAATTCCAAGAACACGCTGTATCTGCAAATGAATAGCCTGAGAGCTGAGGACACGGCTGTGTATCACTGTGCGAGAGAGACTGGAACCTGGGCTTTTGATATCTGGGGCCAAGGGACAATGGTCACCGTCTCTTTA |
| | | | SEQ ID NO: 27549 | SEQ ID NO: 31555 |
| | | AA | QTVVTQEPSFSVSPGGTVTLTCGLSSGSVSTSYYPSWYQQTPGQAPRTLIYNTNTRSSGVPDRFSGSILGNKAALTITGAQADDESDYYCVLYMGSAIWVFGGGTKLTVL | QVQLVESGGGVVQPGRSLRLSCAGTGFTFSYYGMHWVRQAPGKGLEWVAVTTYDGSNKYYADSVKGRFTSSRDNSKNTLYLQMNSLRAEDTAVYHCARETGTWAFDIWGQGTMVTVSL |
| | | | SEQ ID NO: 27550 | SEQ ID NO: 31556 |

FIGURE 50

| | | | | |
|---|---|---|---|---|
| iPS:436912 | 21-225_73C4 | NA | TCCTATGACCTGACTCAGCCACCCTCAGTGTCCGTGTCCCCAGGACAGACAGCCAGCATCACCTGCTCTGGAGATAAATTGGGGAATAAATATGCTTGCTGGTATCAGCAGAAGCCAGGCCAGTCCCCTGTGGTGGTCATCTATCAAGATATGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTGCGGTATTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGGTTCTCACTCAGCACTAGTGGAGTGGGTGTGGGCTGGATCCGTCAGCCCCCAGGAAAGGCCCTGGAGTGGCTTGCACTCATTAATTGGAATGATGATAAGCGCTACAGCCCATCTCTGAAGAGCAGGTTCACCATCACCAGGGACACCTCCAAAGACCAGGTGGTCCTTACAATGACCAACATGGACCCTGTGGACACAGCCACATATTACTGTGCACATAAAACTACCTGGGTGGCTTTTGATATCTGGGGCCAAGGGACAATGGTCACCGTCTCTTCA |
| | | | SEQ ID NO: 27551 | SEQ ID NO: 31557 |
| | | AA | SYDLTQPPSVSVSPGQTASITCSGDKLGNKYACWYQQKPGQSPVVVIYQDMKRPSGIPERFSGSNSGNTATLTISGTQAMDEADYYCQAWDSSTAVFGGGTKLTVL | QITLKESGPTLVKPTQTLTLTCTFSGFSLSTSGVGVGWIRQPPGKALEWLALINWNDDKRYSPSLKSRFTITRDTSKDQVVLTMTNMDPVDTATYYCAHKTTWVAFDIWGQGTMVTVSS |
| | | | SEQ ID NO: 27552 | SEQ ID NO: 31558 |
| iPS:436914 | 21-225_76B4 | NA | CCCTATGAGCTGAATCAGACACCCTCAGTGTCCGTGTCCCCAGGACAGACAGCCAGCATCACCTGCTCTGGAGATAGATTGGGGACTAAATTTGCTTGCTGGTATCAGCAGAAGCCAGGCCAGTCCCCTGTGCTGGTCATTTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTGTATTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGGTTCTCACTCAGCACTGGTGGAGTGGGTGTGGGCTGGATCCGTCAGCCCCCAGGAAAGGCCCTGGAGTGGCTTGCACTCATTTATTGGGATGATGATAAGCGCTACAGCCCATCTCTGAAGAGCAGGCTCACCATCACCAAGGACACCCCCAAAAACCAGGTGGTCCTTACAATGACCAACATGGACCCTGTGGACACAGCCACATATTACTGTGCACACCTTATAGCAGTGGCCTTTGACTACTGGGGCCAGGGAACCCTGGTCACCGTCTCCTCA |
| | | | SEQ ID NO: 27553 | SEQ ID NO: 31559 |

FIGURE 50

| | | | | |
|---|---|---|---|---|
| | | AA | PYELNQTPSVSVSPGQTASITCSGDRLGTKFACW YQQKPGQSPVLVIYQDSKRPSGIPERFSGSNSGN TATLTISGTQAMDEADYYCQAWDSSTVFGGGT KLTVL | QITLKESGPTLVKPTQTLTLTCTFSGFSLSTGGVGVG WIRQPPGKALEWLALIYWDDDKRYSPSLKSRLTIT KDTPKNQVVLTMTNMDPVDTATYYCAHLIAVAFD YWGQGTLVTVSS |
| | | | SEQ ID NO: 27554 | SEQ ID NO: 31560 |
| iPS:436916 | 21-225_74A9 | NA | TCCTATGAGCTGACTCAGCCACCCTCAGTGTC CGTGTCCCCAGGACAGACAGCCAGCATCACCT GCTCTGGAGATAAATTGGGTAATAAATATGTT TGTTGGTATCAGCAGAAGCCAGGCCAGTCCCC TGTGCTGGTCATCTATCAAGATAACAGGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGTCCTGTGATA TTCGGCGGAGGGACCAAACTGACCGTCCTA | CAGGTGCAACTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAAT AATAAATCCTATGCAGACTCCGTGAAGGGCCGAT TCACCATCTCCAGAGACATTTCCAAGAACACGCT GTTTCTGCAAATGAACAGCCTGAGAGCCGATGAC ACGGCTGTGTATTACTGTGCGAGAGATCGAGATT ATTGTAGTGGTACCAGCTGCCCTTATTATTACTAC TACGGTATGGACGTCTGGGGCCAAGGGACCACG GTCACCGTCTCCTCA |
| | | | SEQ ID NO: 27555 | SEQ ID NO: 31561 |
| | | AA | SYELTQPPSVSVSPGQTASITCSGDKLGNKYVCW YQQKPGQSPVLVIYQDNRRPSGIPERFSGSNSGN TATLTISGTQAMDEADYYCQAWDSSPVIFGGGT KLTVL | QVQLVESGGGVVQPGRSLRLSCAASGFTFSSYGMH WVRQAPGKGLEWVAVIWYDGNNKSYADSVKGRF TISRDISKNTLFLQMNSLRADDTAVYYCARDRDYC SGTSCPYYYYYGMDVWGQGTTVTVSS |
| | | | SEQ ID NO: 27556 | SEQ ID NO: 31562 |

FIGURE 50

| | | | | |
|---|---|---|---|---|
| **iPS:436918** | **21-225_77A2** | NA | TCCTATGAGCTGACTCAGCCACCCTCAGTGTCCGTGTCCCCAGGACAGACAGCCAGCATCACCTGCTCTGGAGATAGATTGGGGGATAAATATGCTTGCTGGTATCAGCAGAAGCCAGGCCAGTCCCCTGTACTGGTCATCTATCAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGTACTGCGGTATTCGGCGGAGGGACCAAGCTGACCGTCCTA | CAGATCACCTTGAAGGAGTCTGGTCCTTCGCTGGTGAAACCCACACAGACCCTCACGCTGACCTGCACCTTCTCTGGGTTCTCACTCAGCACTAGTGGAGTGGGTGTGGGCTGGATCCGTCAGCCCCCAGGAAAGGCCCTGGAGTGGCTTGTATTCATTTATTGGGATGATGATAAGCGCTACAGCCCATCTCTGAAGAGCAGGCTCACCATCACCAAGGACACCTCCAAAAACCAGGTGGTCCTTACAATGACCAACATGGACCCTGTGGACACAGCCACATATTACTGTGCACACCTTATAGCAGTGGCCTTTGACTACTGGGGCCAGGGAACCCTGGTCACCGTCTCCTCA |
| | | | SEQ ID NO: 27557 | SEQ ID NO: 31563 |
| | | AA | SYELTQPPSVSVSPGQTASITCSGDRLGDKYACWYQQKPGQSPVLVIYQDRKRPSGIPERFSGSNSGNTATLTISGTQAMDEADYYCQAWDSSTAVFGGGTKLTVL | QITLKESGPSLVKPTQTLTLTCTFSGFSLSTSGVGVGWIRQPPGKALEWLVFIYWDDDKRYSPSLKSRLTITKDTSKNQVVLTMTNMDPVDTATYYCAHLIAVAFDYWGQGTLVTVSS |
| | | | SEQ ID NO: 27558 | SEQ ID NO: 31564 |
| **iPS:436920** | **21-225_74E5** | NA | CAGACTGTGGTGACTCAGGAGCCCTCACTGACTGTGTCCCCAGGAGGGACAGTCACTCTCACCTGTGCTTCCAGCACTGAAACAGTCACCAGTGGTTCTTATCCGAACTGGTTCCAGCAGAAACCTGGACAAGCACCCAGGGCACTGATTTATAGTACAAGCAACAAACACTCCTGGACCCCTGCCCGGTTCTCAGGCTCCCTCCTTGGGGGCAAAGCTGCCCTGACACTGTCAGATGTGCAGCCTGAGGACGAGGCTGAGTATTACTGCCTGCTCTACTATGGTGGTGCTCAACTGGTATTCGGCGGAGGGACCAAGCTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGAAGTGGTGGTTACTACTGGAGCTGGATCCGCCAGCACCCAGGGAAGGGCCTGGAGTGGATTGGATACATCTATTACAGTGGGAGCACCTACTACAACCCGTCCCTCAGGAGTCGAGCTTCCATATCAGTAGACACGTCTAAGAACCAGTTCTCCCTGAAGCTGAGCTCTGTGACTGCCGCGGACACGGCCGTGTATTACTGTGCGAGAGATTCACCAGTGGCTGGTACTGACTACTGGGGCCAGGGAACCCTGGTCACCGTCTCCTCA |
| | | | SEQ ID NO: 27559 | SEQ ID NO: 31565 |

FIGURE 50

| | | | | |
|---|---|---|---|---|
| | | AA | QTVVTQEPSLTVSPGGTVTLTCASSTETVTSGSYPNWFQQKPGQAPRALIYSTSNKHSWTPARFSGSLLGGKAALTLSDVQPEDEAEYYCLLYYGGAQLVFGGGTKLTVL | QVQLQESGPGLVKPSQTLSLTCTVSGGSIRSGGYYWSWIRQHPGKGLEWIGYIYYSGSTYYNPSLRSRASISVDTSKNQFSLKLSSVTAADTAVYYCARDSPVAGTDYWGQGTLVTVSS |
| | | | SEQ ID NO: 27560 | SEQ ID NO: 31566 |
| **iPS:436922** | **21-225_78E9** | NA | TCTTATGAGTTGACTCAGCCACCCTCAGAGTCTGTGTCCCCAGGACAGACAGCCAGCATCACGTGCTCAGGAGATAAATTGGGGAATAAATATGTTTCCTGGTATCAGCAGAAGCCAGGCCAGTCCCCTGTGCTGGTCATCTATCAAGATAACAGGCGGCCGTCAGGGATCCCTGAGCGATTTTCTGGCTCCAACTCTGGGAGCACAGCCACTTTGACCATCAGCGGGACCCAGGCTATGGATGAGGCTGACTATTACTGTCAGGCGTGGGACAGCAGCCCTGTGATATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAATAATAAATCCTATGCAGACTCCGTGAAGGGCCGATTCACCATCTCCAGAGACATTTCCAAAAACACGCTGTATCTGCAAATGAACAGCCTGAGAGCCGAGGACACGGCTGTGTATTACTGTGCGAGGGATCGAGATTATTGTAGTAGTACCAGCTGCCCTTATTATTACTACTACGGTATGGACGTCTGGGGCCAAGGGACCACGGTCACCGTCTCCTCA |
| | | | SEQ ID NO: 27561 | SEQ ID NO: 31567 |
| | | AA | SYELTQPPSESVSPGQTASITCSGDKLGNKYVSWYQQKPGQSPVLVIYQDNRRPSGIPERFSGSNSGSTATLTISGTQAMDEADYYCQAWDSSPVIFGGGTKLTVL | QVQLVESGGGVVQPGRSLRLSCAASGFTFSSYGMHWVRQAPGKGLEWVAVIWYDGNNKSYADSVKGRFTISRDISKNTLYLQMNSLRAEDTAVYYCARDRDYCSSTSCPYYYYYGMDVWGQGTTVTVSS |
| | | | SEQ ID NO: 27562 | SEQ ID NO: 31568 |

FIGURE 50

| | | | | |
|---|---|---|---|---|
| **iPS:436924** | **21-225_74B3** | NA | TCCTATGAGCTGACTCAGCCACCCTCAGTGTC<br>CGTGTCCCCAGGACAGACAGCCAGCATCACCT<br>GCTCTGGAGATAAATTGGGGGATAAATATGCT<br>TGCTGGTATCAGCAGAAGCCAGGCCAGTCCCC<br>TGTGCTGGTCATCTATCAAGATAGCAAGCGGC<br>CCTCAGGGATCCCTGAGCGATTCTCTGGCTCC<br>AACTCTGGGAACACAGCCACTCTGACCATCAG<br>CGGGACCCAGGCTATGGATGAGGCTGACTATT<br>ACTGTCAGGCGTGGGACAGCACCACTGTGGTA<br>TTCGGCGGAGGGACCAAGCTGACCGTCCTA | CAGGTGCAGCTGGTGGAGTCTGGGGGAGGCGTG<br>GTCCAGCCTGGGAGGTCCCTGAGACTCTCCTGTG<br>CAGCGTCGGGATTCACCTTCAGTCGATATGGCAT<br>GCACTGGGTCCGCCAGGCTCCAGGCAAGGGGCT<br>GGAGTGGGTGGCAGTTTTTGGTATGATGGAAGT<br>AATAAAGACTATGCAGACTCCGTGAAGGGCCGA<br>TTCACCATCTCCAGAGACAATTCCAAGAACACGC<br>TGTATCTGCAAATGAACAGCCTGAGAGCCGAGG<br>ACACGGCTGTGTATTACTGTGCGAGAGATCGAGA<br>TTATTGTAGTAGTACCAGCTGCCCTTACTACTACT<br>ACTACGGTATGGACGTCTGGGGCCAAGGGACCA<br>CGGTCACCGTCTCCTCA |
| | | | SEQ ID NO: 27563 | SEQ ID NO: 31569 |
| | | AA | SYELTQPPSVSVSPGQTASITCSGDKLGDKYACW<br>YQQKPGQSPVLVIYQDSKRPSGIPERFSGSNSGN<br>TATLTISGTQAMDEADYYCQAWDSTTVVFGGG<br>TKLTVL | QVQLVESGGGVVQPGRSLRLSCAASGFTFSRYGMH<br>WVRQAPGKGLEWVAVFWYDGSNKDYADSVKGRF<br>TISRDNSKNTLYLQMNSLRAEDTAVYYCARDRDY<br>CSSTSCPYYYYYGMDVWGQGTTVTVSS |
| | | | SEQ ID NO: 27564 | SEQ ID NO: 31570 |

FIGURE 50

| | | | | |
|---|---|---|---|---|
| **iPS:436926** | **21-225_78D10** | NA | CAGACTGTGGTGACTCAGGAGCCCTCACTGACTGTGTCCCCAGGAGGGACAGTCACTCTCACCTGTGCTTCCAGCACTGGAGCAGTCACCAGTGGTTACTTTCCAAACTGGTTCCAGCAGAAACCTGGACAAGCACCCAGGGCACTGATTTATAGTACAGACAACAAACACTCCTGGACCCCTGCCCGGTTCTCAGGCTCCCTCCTTGGGGGCAAAGCTGCCCTGACACTGTCAGGTGTGCAGCCTGAGGACGAGGCTGAGTATTACTGCCTCCTCTACTATGGTGGTGCTCAGCTGATGTTCGGCGGAGGGACCAAGCTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGGTTACTACTGGAGCTGGATCCGCCAGCACCCAGGGAAGGGCCTGGAGTGGATTGGGTACATCTATTACATTGGGAGTGTTTACTACAACCCGTCCCTCAAGAGTCGAGTTACCATATCAGTAGACACGTCTAAGAACCAGTTCTCCCTGAAGCTGAGCTCTGTGACTGCCGCGGACACGGCCGTGTACTACTGTGCGAGAGATGCCCCCGACTTCGGTATGGACGTCTGGGGCCAAGGGACCACGGTCACCGTCTCCTCA |
| | | | SEQ ID NO: 27565 | SEQ ID NO: 31571 |
| | | AA | QTVVTQEPSLTVSPGGTVTLTCASSTGAVTSGYFPNWFQQKPGQAPRALIYSTDNKHSWTPARFSGSLLGGKAALTLSGVQPEDEAEYYCLLYYGGAQLMFGGGTKLTVL | QVQLQESGPGLVKPSQTLSLTCTVSGGSISSGGYYWSWIRQHPGKGLEWIGYIYYIGSVYYNPSLKSRVTISVDTSKNQFSLKLSSVTAADTAVYYCARDAPDFGMDVWGQGTTVTVSS |
| | | | SEQ ID NO: 27566 | SEQ ID NO: 31572 |
| **iPS:436928** | **21-225_79E7** | NA | TCCTATGAGCTGACTCAGCCACCCTCAGTGTCCGTGTCCCCAGGACAGACAGCCAGCATCACCTGCTCAGGAGATAAATTGGGGAATAAATATGTTTCCTGGTATCAGCAGAAGCCAGGCCAGTCCCCTGTGCTGGTCATCTATCAAGATAACAGGCGGCCCTCAGGGATCCCTGAGCGATTCTCTGGCTCCAACTCTGGGAGCACAGCCACTCTGACCATCAGCGGGACCCAGGCTATGGATGAGGCTGACTATTACTGTCAGGCGTGGGACAGCAGCCCTGTGATATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAATAATAAATCCTATGCAGACTCCGTGAAGGGCCGATTCACCATCTCCAGAGACATTTCCAAAAACACGCTGTATCTGCAAATGAACAGCCTGAGAGCCGAGGACACGGCTGTGTATTACTGTGCGAGGGATCGAGATTATTGTAGTAGTACCAGCTGCCCTTATTATTACTACTACGGTATGGACGTCTGGGGCCAAGGGACCACGGTCACCGTCTCCTCA |
| | | | SEQ ID NO: 27567 | SEQ ID NO: 31573 |

FIGURE 50

| | | | | |
|---|---|---|---|---|
| | | AA | SYELTQPPSVSVSPGQTASITCSGDKLGNKYVSWYQQKPGQSPVLVIYQDNRRPSGIPERFSGSNSGSTATLTISGTQAMDEADYYCQAWDSSPVIFGGGTKLTVL | QVQLVESGGGVVQPGRSLRLSCAASGFTFSSYGMHWVRQAPGKGLEWVAVIWYDGNNKSYADSVKGRFTISRDISKNTLYLQMNSLRAEDTAVYYCARDRDYCSSTSCPYYYYYGMDVWGQGTTVTVSS |
| | | | SEQ ID NO: 27568 | SEQ ID NO: 31574 |
| **iPS:436932** | **21-225_92A4** | NA | TCCTATGAGCTGACTCAGCCACCCTCAGTGTCCGTGTCCCCAGGACAGACAGCCAACATCACCTGCTCTGGAGATAAATTGGGGAATAAATATGTTTGCTGGTATCAACAGAAGCCAGGCCAGTCCCCTGTGTTGGTCATCTATCAAGATAACAGGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCCCTGTGATATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAATAATAAATCCTATGCAGACTCCGTGAAGGGCCGATTCACCATCTACAGAGACATTTCCAAGAACACGCTGTATCTGCAAATGAACAGCCTGAGAGCCGAGGACACGGCTGTGTATTACTGTGCGAGAGATCGAGATTATTGTAGTAGTACCAGCTGCCCTTATTATTACTACTACGGTATGGACGTCTGGGGCCAAGGGACCACGGTCACCGTCTCCTCA |
| | | | SEQ ID NO: 27569 | SEQ ID NO: 31575 |
| | | AA | SYELTQPPSVSVSPGQTANITCSGDKLGNKYVCWYQQKPGQSPVLVIYQDNRRPSGIPERFSGSNSGNTATLTISGTQAMDEADYYCQAWDSSPVIFGGGTKLTVL | QVQLVESGGGVVQPGRSLRLSCAASGFTFSSYGMHWVRQAPGKGLEWVAVIWYDGNNKSYADSVKGRFTIYRDISKNTLYLQMNSLRAEDTAVYYCARDRDYCSSTSCPYYYYYGMDVWGQGTTVTVSS |
| | | | SEQ ID NO: 27570 | SEQ ID NO: 31576 |

FIGURE 50

| | | | | |
|---|---|---|---|---|
| iPS:436934 | 21-225_96B5 | NA | CCCTATGAGCTGAATCAGACACCCTCAGTGTCCGTGTCCCCAGGACAGACAGCCAGCATCACCTGCTCTGGAGATAGATTGGGGACTAAATTTGCTTGCTGGTATCAGCAGAAGCCAGGCCAGTCCCCTGTGCTGGTCATTTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTGTATTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGGTTCTCACTCAGCACTGGTGGAGTGGGTGTGGGCTGGATCCGTCAGCCCCCAGGAAAGGCCCTGGAGTGGCTTGCACTCATTTATTGGGATGATGATAAGCGCTACAGCCCATCTCTGAAGAGCAGGCTCACCATCACCAAGGACACCCCCAAAAACCAGGTGGTCCTTACAATGACCAACATGGACCCTGTGGACACAGCCACATATTACTGTGCACACCTTATAGCAGTGGCCTGTGACTACTGGGGCCAGGGAACCCTGGTCACCGTCTCCTCA |
| | | | SEQ ID NO: 27571 | SEQ ID NO: 31577 |
| | | AA | PYELNQTPSVSVSPGQTASITCSGDRLGTKFACWYQQKPGQSPVLVIYQDSKRPSGIPERFSGSNSGNTATLTISGTQAMDEADYYCQAWDSSTVFGGGTKLTVL | QITLKESGPTLVKPTQTLTLTCTFSGFSLSTGGVGVGWIRQPPGKALEWLALIYWDDDKRYSPSLKSRLTITKDTPKNQVVLTMTNMDPVDTATYYCAHLIAVACDYWGQGTLVTVSS |
| | | | SEQ ID NO: 27572 | SEQ ID NO: 31578 |
| iPS:436936 | 21-225_97E6 | NA | TCCTATGAGCTGACTCAGCCACCCTCAGTGTCTGTGTCCCCAGGACAGACAGTCAGCATCACCTGCTCTGGAGATAAATTGGGGAATAAATATGTTTCCTGGTATCAGCAGAAGCCAGGCCAGTCCCCTGTGCTGGTCATCTATCAAGATAACAGGCGGCCGTCAGGGATCCCTGAGCGATTCTCTGGCTCCAACTCTGGGAGCACAGCCACTCTGACCATCAGCGGGACCCAGGCTATGGATGAGGCTGACTATTACTGTCAGGCGTGGGACAGCACCCCTGTGATATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAATAATAAATCCTATGCAGACTCCGTGAAGGGCCGATTCACCATCTCCAGAGACATTTCCAAAAACACGCTGTATCTGCAAATGAACAGCCTGAGAGCCGAGGACACGGCTGTGTATTACTGTGCGAGGGATCGAGATTATTGTAGTAGTACCAGCTGCCCTTATTATTACTACTACGGTATGGACGTCTGGGGCCAAGGGACCACGGTCACCGTCTCCTCA |
| | | | SEQ ID NO: 27573 | SEQ ID NO: 31579 |

FIGURE 50

| | | | | |
|---|---|---|---|---|
| | | AA | SYELTQPPSVSVSPGQTVSITCSGDKLGNKYVSWYQQKPGQSPVLVIYQDNRRPSGIPERFSGSNSGSTATLTISGTQAMDEADYYCQAWDSTPVIFGGGTKLTVL | QVQLVESGGGVVQPGRSLRLSCAASGFTFSSYGMHWVRQAPGKGLEWVAVIWYDGNNKSYADSVKGRFTISRDISKNTLYLQMNSLRAEDTAVYYCARDRDYCSSTSCPYYYYYGMDVWGQGTTVTVSS |
| | | | SEQ ID NO: 27574 | SEQ ID NO: 31580 |
| **iPS:436938** | **21-225_146A3** | NA | TCCTATGCGATGACTCAGCCACCCTCAATGTCCGTGTCCCCAGGACAGACAGCCAGCATCACCTGCTCTGGAAATAAATTGGGGAATAGATATGCTTGCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACATAGCCACTCTGACCATCAGCGGGACCCAGGCTATGGATGAGGCTGACTATTACTGTCAGGCGTGGGACAGCAGCACTGTGGTATTCGGCGGAGGGACCAAGCTGACCGTCCTA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGTTATTAGTGGTGGTGGTACTACCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATGGCGAGGTAACCCCACTGACTACGGTATGGACGTCTGGGGCCAAGGGACCACGGTCACCGTCTCCTCA |
| | | | SEQ ID NO: 27575 | SEQ ID NO: 31581 |
| | | AA | SYAMTQPPSMSVSPGQTASITCSGNKLGNRYACWYQQKPGQSPVLVIYQDSKRPSGIPERFSGSNSGNIATLTISGTQAMDEADYYCQAWDSSTVVFGGGTKLTVL | EVQLLESGGGLVQPGGSLRLSCAASGFTFSSYAMSWVRQAPGKGLEWVSVISGGGTTTYYADSVKGRFTISRDNSKNTLYLQMNSLRAEDTAVYYCAKWRGNPTDYGMDVWGQGTTVTVSS |
| | | | SEQ ID NO: 27576 | SEQ ID NO: 31582 |

FIGURE 50

| | | | | |
|---|---|---|---|---|
| **iPS:436940** | **21-225_146B8** | NA | TCCTATGAGCTGACTCAGCCACCCTCAGTGTC<br>CGTGTCCCCAGGACAGACAGCCAGCATCACCT<br>GCTCTGGAGATAAATTGGGGGATAAATATGTT<br>TGCTGGTATCAGCAGAAGCCAGGCCAGTCCCC<br>TGTGTTGGTCATCTATCAAGATAGGAAGCGGC<br>CCTCAGGGATCCCTGAGCGATTCTCTGGCTCC<br>AACTCTGGGAACACAGCCACTCTGACCATCAG<br>CGGGACCCAGGCTATGGATGAGGCTGACTATT<br>ACTGTCAGGCGTGGCACAGCAGCACTGTGGTA<br>TTCGGCGGAGGGACCAAGCTGACCGTCCTA | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTACCTATGGCAT<br>GCACTGGGTCCGCCAGGCTCCAGGCAAGGGGCT<br>GGAGTGGGTGGCAGTTGTATGGTATGGTGGAAAT<br>GATAAAGACTTTGCAGACTCCGTGACGGGCCGAT<br>TCACCATCTCCAGAGACATTTCCAAGAACACACT<br>GTATCTGCAAATGAACAGCCTGAGAGCCGAGGA<br>CACGGCTGTGTATTACTGTGCGAGAGATCGGGAT<br>TATTGTAGTGGTGGTAGCTGCCCTTACTACTACT<br>ACTACGGTATGGACGTCTGGGGCCAAGGGACCA<br>CGGTCACCGTCTCCTCA |
| | | | SEQ ID NO: 27577 | SEQ ID NO: 31583 |
| | | AA | SYELTQPPSVSVSPGQTASITCSGDKLGDKYVCW<br>YQQKPGQSPVLVIYQDRKRPSGIPERFSGSNSGN<br>TATLTISGTQAMDEADYYCQAWHSSTVVFGGG<br>TKLTVL | QVQLVESGGGVVQPGRSLRLSCAASGFTFSTYGMH<br>WVRQAPGKGLEWVAVVWYGGNDKDFADSVTGR<br>FTISRDISKNTLYLQMNSLRAEDTAVYYCARDRDY<br>CSGGSCPYYYYYGMDVWGQGTTVTVSS |
| | | | SEQ ID NO: 27578 | SEQ ID NO: 31584 |

FIGURE 50

| | | | | |
|---|---|---|---|---|
| **iPS:436942** | **21-225_146H8** | NA | TCCTATGAGCTGACTCAGCCACCCTCAGTGTCCGTGTCCCCAGGACAGACAGCCAGCATCACCTGCTCTGGAGATAAATTGGGGGATAAATATGCTTGCTGGTATCAGCAGAAGCCAGGCCAGTCCCCTGTGCTGGTCATCTATCAAGATAAGAAGCGGCCCTCAGGGATCCCTGAGCGATTCTCTGGCTCCAACTCTGGGAACACAGCCACTCTGACCATCAGCGGGACCCAGGTTATGGATGAGGCTGACTATTACTGTCAGGCGTGGGACATCAGAACTGTGGTATTCGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCATCAGTTATGATATCAATTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCAAAAGCACAGCCTACATGGAGCTGAGCAGCCTGAGATCTGAGGACACGGCCGTGTATTACTGTGCGAGAGGAGATTATTACTATGATAGTAGTGGTCACCAGCCTTACTACTACTACTACGGTATGGACGTCTGGGGCCAAGGGACCACGGTCACCGTCTCCTCA |
| | | | SEQ ID NO: 27579 | SEQ ID NO: 31585 |
| | | AA | SYELTQPPSVSVSPGQTASITCSGDKLGDKYACWYQQKPGQSPVLVIYQDKKRPSGIPERFSGSNSGNTATLTISGTQVMDEADYYCQAWDIRTVVFGGGTKLTVL | QVQLVQSGAEVKKPGASVKVSCKASGYTFISYDINWVRQATGQGLEWMGWMNPNSGNTGYAQKFQGRVTMTRNTSKSTAYMELSSLRSEDTAVYYCARGDYYYDSSGHQPYYYYYGMDVWGQGTTVTVSS |
| | | | SEQ ID NO: 27580 | SEQ ID NO: 31586 |

FIGURE 50

| | | | | |
|---|---|---|---|---|
| iPS:436944 | 21-225_182D12 | NA | TCCTATGAGCTGACTCAGCCACCCTCAGTGTCCGTGTCCCCAGGACAGACAGCCAGCATCACCTGCTCTGGAGATAAATTGGGGGAGAAATATGCTTGCTGGTATCAGCAGAAGTCAGGCCAGTCCCCTGTGTTGGTCATCTATCAAGATAGAAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGTAGAACTGCGGTATTCGGCGGAGGGACCAAGCTGACCGTCCTA | CAGATCACCTTGAAGGAGTCTGGTCCTACGCTGGTGAAACCCACACAGCCCCTCACGCTGACCTGTACCTTCTCTGGGTTCTCACTCAGCACTACTGGAGTGGGTGTGGGCTGGATCCGTCAGCCCCCAGGAAAGGCCCTGGAGTGGCTTGGAATCCTTTTTTGGAATGATGATGAGCGCTACAGCCCATCTCTGAAGAGCAGGCTCACCATCACCAAGGACACCTCCAAAAACCAGGTGGTCCTTACAATGACCAACATGGACCCTGTGGACACAGCCACATATTACTGTGCACACAAATCGCAGCTCGTCTACTTTGACTACTGGGGCCAGGGAACCCTGGTCACCGTCTCCTCA |
| | | | SEQ ID NO: 27581 | SEQ ID NO: 31587 |
| | | AA | SYELTQPPSVSVSPGQTASITCSGDKLGEKYACWYQQKSGQSPVLVIYQDRKRPSGIPERFSGSNSGNTATLTISGTQAMDEADYYCQAWDSRTAVFGGGTKLTVL | QITLKESGPTLVKPTQPLTLTCTFSGFSLSTTGVGVGWIRQPPGKALEWLGILFWNDDERYSPSLKSRLTITKDTSKNQVVLTMTNMDPVDTATYYCAHKSQLVYFDYWGQGTLVTVSS |
| | | | SEQ ID NO: 27582 | SEQ ID NO: 31588 |
| iPS:436946 | 21-225_183F4 | NA | TCCTATGAGCTGACTCAGCCACCCTCAGTGTCCGTGTCCCCAGGACAGACAGCCAGCATCACCTGCTCTGGAGATAAATTGGGGGATAAATATGCTTGTTGGTATCAGCAGAAGCCAGGCCAGTCCCCTGTGTTGGTCATCTATCAAGATAAGAAACGGCCCTCAGGGATCCCTGAGCGATTCTCTGGCTCCAACTCTGGAAACACAGCCACTCTGACCATCAGCGGGACCCAGGCTATGGATGAGGCTGACTATTACTGTCAGGCGTGGGACAACAGCACTGCTGTGGTATTCGGCGGAGGGACCAAACTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGAAAGGACGTATTGTAGTGGTACCACCTGCCCCTACTACTACTACTACGGTCTGGGCGTCTGGGGCCAAGGGACCACGGTCACCGTCTCCTCA |
| | | | SEQ ID NO: 27583 | SEQ ID NO: 31589 |

FIGURE 50

| | | | | |
|---|---|---|---|---|
| | | AA | SYELTQPPSVSVSPGQTASITCSGDKLGDKYACWYQQKPGQSPVLVIYQDKKRPSGIPERFSGSNSGNTATLTISGTQAMDEADYYCQAWDNSTAVVFGGGTKLTVL | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARERTYCSGTTCPYYYYYGLGVWGQGTTVTVSS |
| | | | SEQ ID NO: 27584 | SEQ ID NO: 31590 |
| iPS:436948 | 21-225_183F5 | NA | CAGACTGTGGTGACCCAGGAGCCATCGTTCTCAGTGTCCCCTGGAGGGACAGTCACACTCACTTGTGGCTTGAGCTCTGGCTCAGTCTCTACTACTTTCTACCCCAGCTGGTACCAGCAGACCCCAGGCCAGGCTCCACGCACGCTCATCTACAACACAAACACTCGCTCTTCTGGGGTCCCTGATCGCTTCTCTGGCTCCATCCTTGGGAACAAAGCTGCCCTCACCATCACGGGGGCCCAGGCAGATGATGAATCTGATTATTACTGTGTGCTTTATATGGGTAGTGGCATTTGGGTGTTCGGCGGAGGGACCAAGCTGACCGTCCTA | CAGGTACAGCTGGTGGAGTCTGGGGGAGGCGTGGTCCAGCCTGGGAGGTCCCTGAGACTCTCCTGTGCAGCGTCTGGATTCACCTTCAGTAGTTATGGCATGCTCTGGGTCCGCCAGGCTCCAGGCAAGGGGCTGGAGTGGGTGACAGTTATATGGTATGATGGAAGTGGTAAATACTATGCAGACTCCGTGAAGGGCCGATTCACCATCTCCAGAGACAATTCCAAGAACACACTGTATCTGCAAATGAACAGCCTGAGAGCCGAGGACACGGCTGTTTATTACTGTGCGAGAGAGAATTTTTGGAGTGGTGACTACTGGGGCCAGGGAACCCTGGTCACCGTCTCCTCA |
| | | | SEQ ID NO: 27585 | SEQ ID NO: 31591 |
| | | AA | QTVVTQEPSFSVSPGGTVTLTCGLSSGSVSTTFYPSWYQQTPGQAPRTLIYNTNTRSSGVPDRFSGSILGNKAALTITGAQADDESDYYCVLYMGSGIWVFGGGTKLTVL | QVQLVESGGGVVQPGRSLRLSCAASGFTFSSYGMLWVRQAPGKGLEWVTVIWYDGSGKYYADSVKGRFTISRDNSKNTLYLQMNSLRAEDTAVYYCARENFWSGDYWGQGTLVTVSS |
| | | | SEQ ID NO: 27586 | SEQ ID NO: 31592 |

FIGURE 50

| | | | | |
|---|---|---|---|---|
| **iPS:436950** | **21-225_184G4** | NA | TCCTATGAGCTGACTCAGCCACCCTCAGTGTCCGTGTCCCCAGGACAGACAGCCAGCATCACCTGCTCTGGAGATAAATTGGGGGATAAATTTGCTTGCTGGTATCAGCAGAAGCCAGGCCAGTCCCCTGTGCTGGTCATCTATGAAGATAGGAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCCGCACTGTGGTATTCGGCGGAGGGACCCAGCTGACCGTCCTA | CAGGTGCAGCTGGTGGAGTCTGGGGGAGGCGTGGTCCAGCCTGGGAGGTCCCTGAGACTCTCCTGTGCAGTGTCTGGATTCACCTTTAGTAGCTATGGCATGCACTGGGTCCGCCAGGCTCCAGGCAAGGGGCTGGAGTGGGTGGCAATTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGGGGGGCCCCCGTTCTCTACGGTGACTATGTACTTTGACTACTGGGGCCAGGGAACCCTGGTCACCGTCTCCTCA |
| | | | SEQ ID NO: 27587 | SEQ ID NO: 31593 |
| | | AA | SYELTQPPSVSVSPGQTASITCSGDKLGDKFACWYQQKPGQSPVLVIYEDRKRPSGIPERFSGSNSGNTATLTISGTQAMDEADYYCQAWDSRTVVFGGGTQLTVL | QVQLVESGGGVVQPGRSLRLSCAVSGFTFSSYGMHWVRQAPGKGLEWVAIIWYDGSNKYYADSVKGRFTISRDNSKNTLYLQMNSLRAEDTAVYYCARGGPPFSTVTMYFDYWGQGTLVTVSS |
| | | | SEQ ID NO: 27588 | SEQ ID NO: 31594 |
| **iPS:436952** | **21-225_185D2** | NA | TCCTATGAGCTGACTCAGACACCCTCAGTGTCCGTGTCCCCAGGACAGACAGCCAGCATCACCTGCTCTGGAGATAAATTGGGGGATAAATATGCTTGCTGGTATCAGCAGAAGCCAGGCCAGTCCCCTGTGCTGGTCATCTATGAAGATAGGAAGCGGCCCTCAGGGATCCCTGACCGATTCTCTGGCTCCAACTCTGGGAACACAGCCACTCTGACCATCAGCGGGACCCAGGCTATGGATGAGGCTGACTATTACTGTCAGGCGTGGGACAGCAGGAAAGTGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAATTATATGGTATGATGGAAGTGATAAATACTATGCAGACTCCGTGAAGGGCCGATTCACCATCTCCAGAGACAATTCCAAGAACACGCTGTATCTGCAAATGAACACCCTGAGAGCCGAAGACACGGCTGTGTATTTCTGTGCGAGAGGGGGGCCCCCGTTCTCTACGGTGACTATGTACTTTGACTACTGGGGCCAGGGAACCCTGGTCACCGTCTCCTCA |
| | | | SEQ ID NO: 27589 | SEQ ID NO: 31595 |

FIGURE 50

| | | | | |
|---|---|---|---|---|
| | | AA | SYELTQTPSVSVSPGQTASITCSGDKLGDKYACWYQQKPGQSPVLVIYEDRKRPSGIPDRFSGSNSGNTATLTISGTQAMDEADYYCQAWDSRKVVFGGGTKLTVL | QVQLVESGGGVVQPGRSLRLSCAASGFTFSNYGMHWVRQAPGKGLEWVAIIWYDGSDKYYADSVKGRFTISRDNSKNTLYLQMNTLRAEDTAVYFCARGGPPFSTVTMYFDYWGQGTLVTVSS |
| | | | SEQ ID NO: 27590 | SEQ ID NO: 31596 |
| iPS:436954 | 21-225_185G7 | NA | TCCTATGAGCTGACTCAGCCACCCTCAGTGTCCGTGTCCCCAGGACAGACAGCCAGCATCACCTGCTCTGGAGATAAATTGGGGCATAAATTTGTTTGCTGGTATCAGCAGAAGCCAGGCCAGTCCCCTGTGTTGGTCATCTATCAAGATCGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACGGTATTCGGCGGAGGGACCAAGCTGACCGTCCTA | CAGATCACCCTGAAGGAGTCTGGTCCTACGCTGGTGAAACCCACACAGACCCTCACGTTGACCTGCACCTTCTCTGGGTTCTCACTCACCACTGGTGGAGTGGGTGTGGGCTGGATCCGTCAGCCCCCAGGAAAGGCCCTGGAGTGGCTTGCACTCATTTATTGGAATGATGATGAGCGCTACAGCCCATCTCTGAAGAGCAGGCTCACCATCACCAAGGACACCTCCAAAAACCAGGTGGTCCTTACAATGACCAACATGGACCCTGTGGACACAGCCACATATTACTGTGCACACATTATAGCAGTGGCCTTCCAGCATTGGGGCCAGGGCACCCTGGTCACCGTCTCCTCA |
| | | | SEQ ID NO: 27591 | SEQ ID NO: 31597 |
| | | AA | SYELTQPPSVSVSPGQTASITCSGDKLGHKFVCWYQQKPGQSPVLVIYQDRKRPSGIPERFSGSNSGNTATLTISGTQAMDEADYYCQAWDSSTVFGGGTKLTVL | QITLKESGPTLVKPTQTLTLTCTFSGFSLTTGGVGVGWIRQPPGKALEWLALIYWNDDERYSPSLKSRLTITKDTSKNQVVLTMTNMDPVDTATYYCAHIIAVAFQHWGQGTLVTVSS |
| | | | SEQ ID NO: 27592 | SEQ ID NO: 31598 |

FIGURE 50

| | | | | |
|---|---|---|---|---|
| **iPS:436956** | **21-225_186H6** | NA | TCCTATGAGCTGACTCAGCCACCCTCAGTGTCCGTGTCCCCAGGACAGACAGCCAGCATCACCTGCTCTGGAGATAAAATGGGGGAAAAATATGCTTGCTGGTATCAGCAGAAGCCAGGCCAGTCCCCTGTGCTGGTCATTTATCAAGATAGAAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTGCGGTATTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGGTTCTCACTCAGCACTAGTGGAGTGGGTGTGGGCTGGATCCGTCAGCCCCCAGGAAAGGCCCTGGAGTGGCTTGGATTCATTTCTTGGAATGATGATAAGCGCTACAGCCCATCTCTGAAGAGCAGCCTCACCATCACCAAGGACACCTCCAAAAACCAGGTGGTCCTTACAATGACCAACATGGACCCTGTGGACACAGCCACATATTACTGTGCACACAAAGCAGCAGCTGTTGCTTTTGATATCTGGGGCCAAGGGACAATGGTCACCGTCTCTTCA |
| | | | SEQ ID NO: 27593 | SEQ ID NO: 31599 |
| | | AA | SYELTQPPSVSVSPGQTASITCSGDKMGEKYACWYQQKPGQSPVLVIYQDRKRPSGIPERFSGSNSGNTATLTISGTQAMDEADYYCQAWDSSTAVFGGGTKLTVL | QITLKESGPTLVKPTQTLTLTCTFSGFSLSTSGVGVGWIRQPPGKALEWLGFISWNDDKRYSPSLKSSLTITKDTSKNQVVLTMTNMDPVDTATYYCAHKAAAVAFDIWGQGTMVTVSS |
| | | | SEQ ID NO: 27594 | SEQ ID NO: 31600 |
| **iPS:436958** | **21-225_190D1** | NA | CAGACTGTGGTGACTCAGGAGCCCTCACTGACTGTGTCCCCAGGAGGGACAGTCACTCTCACCTGTGCTTCCAGCACTGGAGCAGTCACCAGTGGTTCCTATCCAAACTGGTTCCAGCAGAAACCTGGACAAGCACCCAGGGCACTGATTTACAGTACAAGTAACAAACACTCCTGGACCCCTGCCCGGTTCTCAGGCTCCCTCCTTGGGGGCAAAGCTGCCCTGACACTGTCAGGTGTGCAGCCTGAGGACGAGGCTGACTATTACTGCCTGCTCTACTATGGTGGTGCTCAGGTGGCATTCGGCGGAGGGACCAAGTTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGGTTACTACTGGAGCTGGATCCGCCAGCACCCAGGGAAGGGCCTGGAGTGGATTGGATACATCTATTACAGTGGGAGCACCTACTACAACCCGTCCCTCAAGAGTCGAGTCACCATATCAGTAGACACGTCTAAGAACCAGTTCTCCCTGAAGCTGAGCTCTGTGACTGCCGCGGACACGGCCGTTTATTACTGTGCGAGAGATTCCCCACTACGAGGCTTTGACTACTGGGGCCAGGGAACCCTGGTCACCGTCTCCTCA |
| | | | SEQ ID NO: 27595 | SEQ ID NO: 31601 |

FIGURE 50

| | | | | |
|---|---|---|---|---|
| | | AA | QTVVTQEPSLTVSPGGTVTLTCASSTGAVTSGSYPNWFQQKPGQAPRALIYSTSNKHSWTPARFSGSLLGGKAALTLSGVQPEDEADYYCLLYYGGAQVAFGGGTKLTVL | QVQLQESGPGLVKPSQTLSLTCTVSGGSISSGGYYWSWIRQHPGKGLEWIGYIYYSGSTYYNPSLKSRVTISVDTSKNQFSLKLSSVTAADTAVYYCARDSPLRGFDYWGQGTLVTVSS |
| | | | SEQ ID NO: 27596 | SEQ ID NO: 31602 |
| **iPS:436960** | **21-225_198D2** | NA | CAGTCTGTGTTGACGCAGCCGCCCTCAGTGTCTGCGGCCCCAGGACAGAAGGTCACCATCTCCTGCTCTGGAAGCAGCTCCAACATTGGGAGTAATTATGTTTCCTGGTACCAACAGCTCCCAGGAACAGCCCCCAAAGTCCTCATTTATGACAATAATAAGCGACCCTCAGGGATTCCTGACCGATTCTCTGGCTCCAAGTCTGGCACGTCAGCCACCCTGGGCATCACCGGACTCCAGACTGGGGACGAGGCCGATTATTACTGCGGAACATGGGATAGCAGACTGAATGTTGGGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCCTCTGGATTCACCTTCAGAAGCTATGGCATGCATTGGGTCCGCCAGGCTCCAGGCAAGGGGCTGGAGTGGGTGGCAGTTATAATATATGATGGAAGTTATAAGTACTATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTACTGTGCGAGAACGTATAGCGGGGGTATGGACGTCTGGGGCCAAGGGACCACGGTCACCGTCTCCTCA |
| | | | SEQ ID NO: 27597 | SEQ ID NO: 31603 |
| | | AA | QSVLTQPPSVSAAPGQKVTISCSGSSSNIGSNYVSWYQQLPGTAPKVLIYDNNKRPSGIPDRFSGSKSGTSATLGITGLQTGDEADYYCGTWDSRLNVGVFGGGTKLTVL | QVQLVESGGGVVQPGRSLRLSCAASGFTFRSYGMHWVRQAPGKGLEWVAVIIYDGSYKYYADSVKGRFTISRDNSKNTLYLQMNSLRAEDTAVYYCARTYSGGMDVWGQGTTVTVSS |
| | | | SEQ ID NO: 27598 | SEQ ID NO: 31604 |

FIGURE 50

| | | | | |
|---|---|---|---|---|
| iPS:436962 | 21-225_190H1 | NA | TCCTATGAGTTGACTCAGCCACCCTCAGTGTCCGTGTCCCCAGGACAGACAGCCAGCATCACCTGCTCTGGAGATAAATTGGGGGATAGATTTGCTTACTGGTATCAGCAGAAGCCAGGCCAGTCCCCTGTACTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTAAGGCGTGGGACAGCAGCACTGTGGTATTCGGCGGAGGGACCAAGCTGACCGTCCTA | CAGGTACAGCTGCAGCAGTCAGGTCCAGGACTGGTGAAGCCCTCGCAGACCCTCTCACTCACCTGTGACATCTCCGGGGACAGTGTCTCTAGGAAAAGTGCTACTTGGAACTGGATCAGGCAGTCCCCATCGAGAGGCCTTGAGTGGCTGGGAAAGACATACTACAGGTCCAAGTGGTATAATGATTATGCAGTATCTGTGAAAAGTCGAATAACCATCAATCCAGACACATCCAAGAACCAGTTCTCCCTGCAATTGAACTCTGTGACTCCCGAGGACACGGCTGTGTATTACTGTGCAAGAGATCCGGGTGGCCTCTTTGACTACTGGGGCCAGGGAACCCTGGTCACCGTCTCCTCA |
| | | | SEQ ID NO: 27599 | SEQ ID NO: 31605 |
| | | AA | SYELTQPPSVSVSPGQTASITCSGDKLGDRFAYWYQQKPGQSPVLVIYQDSKRPSGIPERFSGSNSGNTATLTISGTQAMDEADYYCKAWDSSTVVFGGGTKLTVL | QVQLQQSGPGLVKPSQTLSLTCDISGDSVSRKSATWNWIRQSPSRGLEWLGKTYYRSKWYNDYAVSVKSRITINPDTSKNQFSLQLNSVTPEDTAVYYCARDPGGLFDYWGQGTLVTVSS |
| | | | SEQ ID NO: 27600 | SEQ ID NO: 31606 |
| iPS:436964 | 21-225_190B3 | NA | TCTTCTGAGCTGACTCAGGACCCTGCTGTGTCTGTGGCCTTGGGACAGACAGTCAGGATCACATGCCAAGGAGACAAACTCAGAACCTATTATGCAAGCTGGTACCAGCAGAAGCCAGGACAGGCCCCTGTACTTGTCGTCTATGGAAAAAACAACCGGCCCTCAGGGATCCCAGACCGATTCTCTGGCTCCAGCTCAGGAAACACAGCTTCCTTGACCATCACTGGGGCTCAGGCGGAAGATGAGGCTGACTATTACTGTAACTCCCGGGACAGCAGTGGTAACCATCTTGTACTATTCGGCGGAGGGACCAAGCTGACCGTCCTA | CAGGTGCAGCTGGTGGAATCTGGGGGAGGCGTGGTCCAGCCTGGGAGGTCCCTGAGACTCTCCTGTGCAGCGTCTGGATTCACCTTCAATAACTATGGCATACACTGGGTCCGCCAGGCTCCAGGCAAGGGGCTGGAGTGGGTGGCAGTTATATGGTTTGATGGAGATAATAAATACTATGCAGACTCCGTGAAGGGCCGATTCACCATCTCCAGAGACAATTCCAAGAACACGCTGTATCTGCAAATGAACAGCCTGAGAGCCGAAGACACGGCTGTGTATTACTGTGCGAGAGATAACTGGAACTACGGCGATCACTACTACTACTTCGGTATGGACGTCTGGGGCCAAGGGACCACGGTCACCGTCTCCTCA |
| | | | SEQ ID NO: 27601 | SEQ ID NO: 31607 |

FIGURE 50

| | | | | |
|---|---|---|---|---|
| | | AA | SSELTQDPAVSVALGQTVRITCQGDKLRTYYASWYQQKPGQAPVLVVYGKNNRPSGIPDRFSGSSSGNTASLTITGAQAEDEADYYCNSRDSSGNHLVLFGGGTKLTVL | QVQLVESGGGVVQPGRSLRLSCAASGFTFNNYGIHWVRQAPGKGLEWVAVIWFDGDNKYYADSVKGRFTISRDNSKNTLYLQMNSLRAEDTAVYYCARDNWNYGDHYYYFGMDVWGQGTTVTVSS |
| | | | SEQ ID NO: 27602 | SEQ ID NO: 31608 |
| **iPS:436966** | **21-225_190C3** | NA | CAGTCTGTGTTGACGCAGCCGCCCTCAGTGTCTGCGGCCCCAGGACAGAAGGTCACCATCTCCTGCTCTGGAAGCAGCTCCAACATTGGAAATAATTATGTATCCTGGTACCAGCAGCTCCCAGGAACAGCCCCCAAACTCCTCCTTTATGACAGTAATAAGCGACCCTCAGGGATTCCTGGCCGATTCTCTGGCTCCAAGTCTGGCACGTCAGCCACCCTGGGCATCACCGGCCTCCAGACTGGGGACGAGGCCGATTATTACTGCGGAACATGGGATAGCAGCCTGAGTACTGTGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAACTGGTACTACTACTACTACGGTATGGACGTCTGGGGCCAAGGGACCACGGTCACCGTCTCCTCA |
| | | | SEQ ID NO: 27603 | SEQ ID NO: 31609 |
| | | AA | QSVLTQPPSVSAAPGQKVTISCSGSSSNIGNNYVSWYQQLPGTAPKLLLYDSNKRPSGIPGRFSGSKSGTSATLGITGLQTGDEADYYCGTWDSSLSTVVFGGGTKLTVL | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCANWYYYYYGMDVWGQGTTVTVSS |
| | | | SEQ ID NO: 27604 | SEQ ID NO: 31610 |

FIGURE 50

| | | | | |
|---|---|---|---|---|
| iPS:436968 | 21-225_190B10 | NA | CAGTCTGTGTTGACGCAGCCGCCCTCAGTGTCTGCGGCCCCAGGACAGAAGGTCACCATCTCCTGCTCTGGAAGCAGCTCCAACATTGGGAATAATTATGTATCCTGGTACCAGCATCTCCCAGGAACAGCCCCCAAACTCCTCATTTATGACAATAATAAGCGACCCTCAGGGATTCCTGACCGATTCTCTGGCTCCAAGTCTGGCACGTCAGCCACCCTGGGCATCACCGGACTCCAGACTGGGGACGAGGCCGATTATTACTGCGGAACATGGGATAGCAGCCTGAGTGCTGGGGTTTTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGTAGCGTCTGGATTCACCTTCAGTAGCTATGGCATGCACTGGGTCCGCCAGGCTCCAGGCAAGGGGCTGGAGTGGGTGGCAGTTATATGGAATGATGGAAGTAAAAAATACCATGTAGACTCCGTGAAGGGCCGATTTACCATCTCCAGAGACAATTCCAAGAACACGCTGTATCTGCAAATGAACAGCCTGAGAGCCGAGGACACGGCTCTGTATTACTGTGCGAGAGATCTGGATAAGAGGAACTTTCCTTATTACTACTACTACGGTATGGACGTCTGGGGCCAAGGGACCACGGTCACCGTCTCCTCA |
| | | | SEQ ID NO: 27605 | SEQ ID NO: 31611 |
| | | AA | QSVLTQPPSVSAAPGQKVTISCSGSSSNIGNNYVSWYQHLPGTAPKLLIYDNNKRPSGIPDRFSGSKSGTSATLGITGLQTGDEADYYCGTWDSSLSAGVFGGGTKLTVL | QVQLVESGGGVVQPGRSLRLSCVASGFTFSSYGMHWVRQAPGKGLEWVAVIWNDGSKKYHVDSVKGRFTISRDNSKNTLYLQMNSLRAEDTALYYCARDLDKRNFPYYYYYGMDVWGQGTTVTVSS |
| | | | SEQ ID NO: 27606 | SEQ ID NO: 31612 |
| iPS:436970 | 21-225_190B8 | NA | TCTTCTGAGCTGACTCAGGACCCTGCTGTGTCTGTGGCCTTGGGACAGACAGTCAGGATCACATGCCAAGGAGACACCCTCAGACCCTATTATGTAAGCTGGTACCAGCAGAAGCCAGGACAGGCCCCTGTACTTGTCATCTATGGTAAAAACAACCGGCCCTCAGGGATCCCAGACCGATTCTCTGGCTCCAGCTCAGGAAACACAGCTTCCTTGACCATCACTGGGGCTCAGGCGGAAGATGAGGCTGACTATTACTGTAACTCCCGGGACAGCAGTGGTAACCATCTTGTGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTTTGATGGAAGTAATAAATACTATACAGACTCCGTGAAGGGCCGATTCACCATCTCCAGAGACAATTCCAAGAACACGCTGTATCTGCAAATGAACAGCCTGAGAGCCGAGGACACGGCTGTATATTATTGTGCGAGAGATAACTGGAACTACGGCGATTACTACTACTACTACGGTATGGACGTCTGGGGCCAAGGGACCACGGTCACCGTCTCCTCA |
| | | | SEQ ID NO: 27607 | SEQ ID NO: 31613 |

FIGURE 50

| | | | | |
|---|---|---|---|---|
| | | AA | SSELTQDPAVSVALGQTVRITCQGDTLRPYYVSWYQQKPGQAPVLVIYGKNNRPSGIPDRFSGSSSGNTASLTITGAQAEDEADYYCNSRDSSGNHLVVFGGGTKLTVL | QVQLVESGGGVVQPGRSLRLSCAASGFTFSSYGMHWVRQAPGKGLEWVAVIWFDGSNKYYTDSVKGRFTISRDNSKNTLYLQMNSLRAEDTAVYYCARDNWNYGDYYYYYGMDVWGQGTTVTVSS |
| | | | SEQ ID NO: 27608 | SEQ ID NO: 31614 |
| **iPS:436972** | **21-225_190C7** | NA | CAGTCTGTGTTGACGCAGCCGCCCTCAGTGTCTGCGGCCCCAGGACAGAAGGTCACCATCTCCTGCTCTGGAGGCAGCTCCAACATTGGGAATAATTATGTATCCTGGTTTCAGCAGTTCCCAGGAACAGCCCCCAAATTCCTCATTTATGACAATAATAAGCGACCCTCAGGGATTCCTGACCGATTCTCTGGCTCCAAGTCTGGCACGTCAGCCACCCTGGGCATCACCGGACTCCAGACTGGTGACGAGGCCGATTATTACTGCGGAACATGGGATCGCACCCTGAGTGATTGGGTATTCGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAACAGTGGTGGCACAAACTATGCACAGAAGTTTCAGGGCAGGGTCACCATGACCAGGGACACGTCCATCAGCACAGCCTACATGGAACTGAGCAGGCTGAGATCTGACGACACGGCCGTGTATTACTGTGCGAGAGATAGAGCAGTGGCTGGAAACTACTTCTACTACGGTATGGACGTCTGGGGCCAAGGGACCACGGTCACCGTCTCCTCA |
| | | | SEQ ID NO: 27609 | SEQ ID NO: 31615 |
| | | AA | QSVLTQPPSVSAAPGQKVTISCSGGSSNIGNNYVSWFQQFPGTAPKFLIYDNNKRPSGIPDRFSGSKSGTSATLGITGLQTGDEADYYCGTWDRTLSDWVFGGGTKLTVL | QVQLVQSGAEVKKPGASVKVSCKASGYTFTGYYMHWVRQAPGQGLEWMGWINPNSGGTNYAQKFQGRVTMTRDTSISTAYMELSRLRSDDTAVYYCARDRAVAGNYFYYGMDVWGQGTTVTVSS |
| | | | SEQ ID NO: 27610 | SEQ ID NO: 31616 |

FIGURE 50

| | | | | |
|---|---|---|---|---|
| **iPS:436974** | **21-225_190H7** | NA | CAGTCTGTGTTGACGCAGCCGCCCTCAGTGTC<br>TGCGGCCCCAGGACAGAAGGTCACCATCTCCT<br>GCTCTGGAAGCAGCTCCAACATTGGGAGTAAT<br>TATGTTTCCTGGTACCAACAGCTCCCAGGAAC<br>AGCCCCCAAAGTCCTCATTTATGACAATAATA<br>AGCGACCCTCAGGGATTCCTGACCGATTCTCT<br>GGCTCCAAGTCTGGCACGTCAGCCACCCTGGG<br>CATCACCGGACTCCAGACTGGGGACGAGGCC<br>GATTATTACTGCGGAACATGGGATGGCAGACT<br>GAATGTTGGGGTATTCGGCGGAGGGACCAAG<br>CTGACCGTCCTA | CAGGTGCAGCTGGTGGAGTCTGGGGGAGGCGTG<br>GTCCAGCCTGGGAGGTCCCTGAGACTCTCCTGTG<br>CAGCCTCTGGATTCAACTTCAGAAGCTATGGCAT<br>GCATTGGGTCCGCCAGGCTCCAGGCAAGGGGCT<br>GGAGTGGGTGGCAGTTATAATATATGATGGAAGT<br>TATAAGTACTATGCAGACTCCGTGAAGGGCCGAT<br>TCACCATCTCCAGAGACAATTCCAAGAACACGCT<br>GTATCTGCAAATGAACAGCCTGAGAGCTGAGGA<br>CACGGCTGTGTATTACTGTGCGAGAACGTATAGC<br>GGGGGTATGGACGTCTGGGGCCAAGGGACCACG<br>GTCACCGTCTCCTCA |
| | | | SEQ ID NO: 27611 | SEQ ID NO: 31617 |
| | | AA | QSVLTQPPSVSAAPGQKVTISCSGSSSNIGSNYVS<br>WYQQLPGTAPKVLIYDNNKRPSGIPDRFSGSKSG<br>TSATLGITGLQTGDEADYYCGTWDGRLNVGVF<br>GGGTKLTVL | QVQLVESGGGVVQPGRSLRLSCAASGFNFRSYGM<br>HWVRQAPGKGLEWVAVIIYDGSYKYYADSVKGRF<br>TISRDNSKNTLYLQMNSLRAEDTAVYYCARTYSGG<br>MDVWGQGTTVTVSS |
| | | | SEQ ID NO: 27612 | SEQ ID NO: 31618 |
| **iPS:436976** | **21-225_190D8** | NA | CAGTCTGTGTTGACGCAGCCGCCCTCAGTGTC<br>TGCGGCCCCAGGACAGAAGGTCACCATCTCCT<br>GCTCTGGAAGCAGCTCCAACATTGGGAATCAT<br>TATGTCTCCTGGTACCAGCAGCTTCCAGGAAC<br>AGCCCCCAAACTCCTCATTTATGACAGTAGTA<br>AGCGACCCTCAGAGATTCCTGACCGATTCTCT<br>GGCTCCAAGTCTGGCACGTCCGCCACCCTGGG<br>CATCACCGGACTCCAGACTGGGGACGAGGCC<br>GATTATTACTGCGGAACATGGGATAGTAGTCT<br>GAGTACTGTGGTATTCGGCGGAGGGACCAAGC<br>TGACCGTCCTA | CAGGTGCAGCTGGTGGAGTCTGGGGGAGACGTG<br>GTCCAGCCTGGAAGGTCCCTGAGACTCTCCTGTG<br>AAGCGTCTGGATTCACCTTCAGTAGCTATGGCCT<br>GCACTGGGTCCGCCAGGCTCCCGGCAAGGGACT<br>GGAGTGGGTGGCAGTTATATGGTATGATGGAAGT<br>AATAAATACTATGCAGACTCCGTGAAGGGCCGA<br>TTCACCATCTCCAGAGACAATTCCAAGAACACGC<br>TGTATCTGCAAATGAACAGCCTGAGAGCCGAGG<br>ACACGGCTGTGTATTACTGTGCGAACTGGTACTA<br>CTACTACTACGGTATGGACGTCTGGGGCCAAGGG<br>ACCACGGTCACCGTCTCCTCA |
| | | | SEQ ID NO: 27613 | SEQ ID NO: 31619 |

FIGURE 50

| | | | | |
|---|---|---|---|---|
| | | AA | QSVLTQPPSVSAAPGQKVTISCSGSSSNIGNHYVSWYQQLPGTAPKLLIYDSSKRPSEIPDRFSGSKSGTSATLGITGLQTGDEADYYCGTWDSSLSTVVFGGGTKLTVL | QVQLVESGGDVVQPGRSLRLSCEASGFTFSSYGLHWVRQAPGKGLEWVAVIWYDGSNKYYADSVKGRFTISRDNSKNTLYLQMNSLRAEDTAVYYCANWYYYYGMDVWGQGTTVTVSS |
| | | | SEQ ID NO: 27614 | SEQ ID NO: 31620 |
| **iPS:436978** | **21-225_190G9** | NA | TCCTATGAGCTGACTCAGCCACCCTCAGTGTCCGTGTCCCCAGGACAGACAGCCAGCATCACCTGCTCTGGAGATAAATTGGGGGATAGATTTGCTTACTGGTATCAGCAGAAGCCAGGCCAGTCCCCTGTGCTGGTCATCTATCAAGATAACAAGCGGCCCTCAGGGATCCCTGAGCGATTCTCTGGCTCCAACTCTGGGAACACAGCCTCTCTGACCATCAGCGGGACCCAGGCTATGGATGAGGCTGACTATTACTGTCAGGCGTGGGACAGCAGCACTGTGGTATTCGGCGGAGGGACCAGGCTGACCGTCCTA | CAGGTACAGTTGCAGCAGTCAGGTCCAGGACTGGTGAAGCCCTCGCAGACCCTCTCACTCACCTGTGCCATCTCCGGGGACAGTGTCTCTAGGAAAAGTGCTACTTGGAACTGGATCAGGCAGTCCCCATCGAGAGGCCTTGAGTGGCTGGGAAGGACATACTACAGGTCCAAGTGGTATAATGATTATGCAGTATCTGTGAAAAGTCGAATAATCATCAATCCAGACACATCCAAGAACCAGTTCTCCCTGCAGCTGAACTCTGTGACTCCCGAGGACACGGCTGTGTATTACTGTGCAAGAGATCCGGGTGGCCTCTTTGACTACTGGGGCCAGGGAACCCTGGTCACCGTCTCCTCA |
| | | | SEQ ID NO: 27615 | SEQ ID NO: 31621 |
| | | AA | SYELTQPPSVSVSPGQTASITCSGDKLGDRFAYWYQQKPGQSPVLVIYQDNKRPSGIPERFSGSNSGNTASLTISGTQAMDEADYYCQAWDSSTVVFGGGTRLTVL | QVQLQQSGPGLVKPSQTLSLTCAISGDSVSRKSATWNWIRQSPSRGLEWLGRTYYRSKWYNDYAVSVKSRIIINPDTSKNQFSLQLNSVTPEDTAVYYCARDPGGLFDYWGQGTLVTVSS |
| | | | SEQ ID NO: 27616 | SEQ ID NO: 31622 |

FIGURE 50

| | | | | |
|---|---|---|---|---|
| iPS:436980 | 21-225_190C10 | NA | TCTTCTGAGCTGACTCAGGACCCTGCTGTGTCTGTGGCCTTGGGACAGACAGTCAGGATCACATGCCAAGGAGACAGCCTCAGACCCTATTATGCAAGCTGGTACCAGCAGAAGCCAGGACAGGCCCCTGTACTTGTCATCTATGGTAAAAACAACCGGCCCTCAGGGATCCCAGACCGATTCTCTGGCTCCAGTTCAGGAAACACAGCTTCCTTGACCATCACTGAGGCTCAGGCGGAAGATGAGGCTGACTATTACTGTAACTCCCGGGACAGCAGTGGTAACCATCTTGTGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCCTCAGTAACTATGGCATGCACTGGGTCCGCCAGGCTCCAGGCAAGGGGCTGGAGTGGGTGGCAGTTATATGGTTTGGTGGAGATAATAAATACTATGCAGACTCCGTGAGGGGCCGATTCACCATCTCCAGAGACAATTCCAAGAACACGCTGTATCTGCAAATGAACAGCCTGAGAGCCGAGGACACGGCTGTGTTTTACTGTGCGAGAGATAACTGGAACTACGGCGATCACTACTACTATTACGGAATGGACGTCTGGGGCCAAGGGACCACGGTCACCGTCTCCTCA |
| | | | SEQ ID NO: 27617 | SEQ ID NO: 31623 |
| | | AA | SSELTQDPAVSVALGQTVRITCQGDSLRPYYASWYQQKPGQAPVLVIYGKNNRPSGIPDRFSGSSSGNTASLTITEAQAEDEADYYCNSRDSSGNHLVVFGGGTKLTVL | QVQLVESGGGVVQPGRSLRLSCAASGFTLSNYGMHWVRQAPGKGLEWVAVIWFGGDNKYYADSVRGRFTISRDNSKNTLYLQMNSLRAEDTAVFYCARDNWNYGDHYYYYGMDVWGQGTTVTVSS |
| | | | SEQ ID NO: 27618 | SEQ ID NO: 31624 |
| iPS:436982 | 21-225_190D10 | NA | CAGTCTGTGTTGACGCAGCCGCCCTCAGTGTCTGCGGCCCCAGGACAGAAGGTCACCATCTCCTGCTCTGGAAGCAGCTCCAACATTGGGAGTAATTATGTTTCCTGGTACCAACAGCTCCCAGGAACAGTCCCCAAAGTCCTCATTTATGACAATAATAAGCGACCCTCAGGGATTCCTGACCGATTCTCTGGCTCCAAGTCTGGCACGTCAGCCACCCTGGGCATCACCGGACTCCAGACTGGGGACGAGGCCGATTATTACTGCGGAACATGGGATAGCAGACTGAATGTTGGGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCCTCTGGATTCACCTTCAGAAGCTATGGCATGCATTGGGTCCGCCAGGCTCCAGGCAAGGGGCTGGAGTGGGTGGCAGTTATAATATATGATGGAAGTTATAAGTACTATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTACTGTGCGAGAACGTATAGCGGGGGTATGGACGTCTGGGGCCAAGGGACCACGGTCACCGTCTCCTCA |
| | | | SEQ ID NO: 27619 | SEQ ID NO: 31625 |

FIGURE 50

| | | | | |
|---|---|---|---|---|
| | | AA | QSVLTQPPSVSAAPGQKVTISCSGSSSNIGSNYVSWYQQLPGTVPKVLIYDNNKRPSGIPDRFSGSKSGTSATLGITGLQTGDEADYYCGTWDSRLNVGVFGGGTKLTVL | QVQLVESGGGVVQPGRSLRLSCAASGFTFRSYGMHWVRQAPGKGLEWVAVIIYDGSYKYYADSVKGRFTISRDNSKNTLYLQMNSLRAEDTAVYYCARTYSGGMDVWGQGTTVTVSS |
| | | | SEQ ID NO: 27620 | SEQ ID NO: 31626 |
| **iPS:436984** | **21-225_190F10** | NA | CAGACTGTGGTGACTCAGGAGCCCTCACTGACTGTGTCCCCAGGAGGGACAGTCACTCTCACTTGTGTTTTTAGCACTGGAGCAGTCACCAGTGGTTCCTTTCCAAACTGGTTCCAGCAGAAACCTGGACAAGCACCCAGGGCACTGATTTATAGTACAAGCAACAAACACTCCTGGACCCCTGCCCGGTTCTCAGGCTCCCTCCTTGGGGGCAAAGCTGCCCTGACACTGTCAGGTGTGCAGCCTGAGGACGAGGCTGACTATTACTGCCTGCTCTACTGTGGTGGTGCTCAGCTGGTGTTCGGCGGAGGGACCAAGCTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGAAGTGGTGGTGACTACTGGAGCTGGATCCGCCAGCACCCAGGGGAGGGCCTGGAGTGGATTGGGTACATCTATTACAGTGGGATCACCTACTACAATCCGTCCCTCAAGAGTCGAGTTACCATATCAGTAGACACGTCTAAGAACCAGTTCTCCCTGAAGCTGAGCTCTGTGACTGCCGCGGACACGGCCGTGTATTACTGTGCGAGAGATAGCAGCTCGCGGGGTATGGACGTCTGGGGCCAAGGGACCACGGTCACCGTCTCCTCA |
| | | | SEQ ID NO: 27621 | SEQ ID NO: 31627 |
| | | AA | QTVVTQEPSLTVSPGGTVTLTCVFSTGAVTSGSFPNWFQQKPGQAPRALIYSTSNKHSWTPARFSGSLLGGKAALTLSGVQPEDEADYYCLLYCGGAQLVFGGGTKLTVL | QVQLQESGPGLVKPSQTLSLTCTVSGGSIRSGGDYWSWIRQHPGEGLEWIGYIYYSGITYYNPSLKSRVTISVDTSKNQFSLKLSSVTAADTAVYYCARDSSSRGMDVWGQGTTVTVSS |
| | | | SEQ ID NO: 27622 | SEQ ID NO: 31628 |

FIGURE 50

| | | | | |
|---|---|---|---|---|
| **iPS:436986** | **21-225_191A1** | NA | CAGTCTGTGTTGACGCAGCCGCCCTCAGTGTCTGCGGCCCCAGGACAGAGGGTCACCATCTCCTGCTCTGGAAGCAGCTCCAACCTTGGAAATAATTTTGTATCCTGGTACCAGCAGTTCCCAGGAACAGCCCCCAAACTCCTCATTTATGACAATTATAAGCGACCCTCAGGGATTCCTGACCGATTCTCTGTCTCCAAGTCTGGCACGTCAGCCACCCTGGGCATCACCGGACTCCAGACTGGGGACGAGGCCGATTATTACTGCGGAACATGGGATAGCAGCCTGAATACTGGGGTATTCGGCGGAGGGACCAAGCTGACCGTCCTA | CAGGTGCAGCTGCAGGAGTCGGGCCCAGGACTGGTGAAGCCTTCGGAGACCCTGTCCCTCACCTGCACTGTCTCTGGTGGCTCCATCAGAAGTTACTACTGGATCTGGATCCGGCAGCCCCCAGGGAAGGGACTGGAGTGGATTGGGTATATCTATTACAGTGGGAGTACTAAGTACAACCCCTCCCTCAAGAGTCGAGTCACCATATCAGTAGACACGTCCAAGAACCAGTTCTCCCTGAAACTGAGCTCTGTGACCGCTGCGGACACGGCCGTGTATTACTGTGCGAGAAAGGGAGTGGGAACCATCCACTTTGACTACTGGGGCCAGGGAACCCTGGTCACCGTCTCCTCA |
| | | | SEQ ID NO: 27623 | SEQ ID NO: 31629 |
| | | AA | QSVLTQPPSVSAAPGQRVTISCSGSSSNLGNNFVSWYQQFPGTAPKLLIYDNYKRPSGIPDRFSVSKSGTSATLGITGLQTGDEADYYCGTWDSSLNTGVFGGGTKLTVL | QVQLQESGPGLVKPSETLSLTCTVSGGSIRSYYWIWIRQPPGKGLEWIGYIYYSGSTKYNPSLKSRVTISVDTSKNQFSLKLSSVTAADTAVYYCARKGVGTIHFDYWGQGTLVTVSS |
| | | | SEQ ID NO: 27624 | SEQ ID NO: 31630 |
| **iPS:436988** | **21-225_191A2** | NA | CAGACTGTGGTGACTCAGGAGCCCTCACTGACTGTGTCCCCAGGAGGGACAGTCACTCTCACTTGTGTTCTTAGCACTGGAGCAGTCACCAGTGGTTCCTTTCCAAACTGGTTCCAGCAGAAACCTGGACAAGCACCCAGGGCACTGATTTATAGTACAAGCAACAAACACTCCTGGACCCCTGCCCGGTTTTCAGGCTCCCTCCTGGGGGGCAAAGCTGCCCTGACACTGTCAGGTGTGCAGCCTGAGGACGAGGCTGACTATTACTGCATGCTCTACTGTGGTGGTGCTCAGCTGGTGTTCGGCGGAGGGACCAAGCTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGAAGTGGTGGTGACTACTGGAGCTGGATCCGCCAGCACCCAGGGGAGGGCCTGGAGTGGATTGGGTACATCTATTACAGTGGGATCACCTACTACAATCCGTCCCTCAAGAGTCGAGTTACCATATCAGTAGACACGTCTAAGAACCAGTTCTCCCTGAAGCTGAGCTCTGTGACTGCCGCGGACACGGCCGTGTATTACTGTGCGAGAGATAGCAGCTCGCGGGGTATGGACGTCTGGGGCCAAGGGACCACGGTCACCGTCTCCTCA |
| | | | SEQ ID NO: 27625 | SEQ ID NO: 31631 |

FIGURE 50

| | | | | |
|---|---|---|---|---|
| | | AA | QTVVTQEPSLTVSPGGTVTLTCVLSTGAVTSGSFPNWFQQKPGQAPRALIYSTSNKHSWTPARFSGSLLGGKAALTLSGVQPEDEADYYCMLYCGGAQLVFGGGTKLTVL | QVQLQESGPGLVKPSQTLSLTCTVSGGSIRSGGDYWSWIRQHPGEGLEWIGYIYYSGITYYNPSLKSRVTISVDTSKNQFSLKLSSVTAADTAVYYCARDSSSRGMDVWGQGTTVTVSS |
| | | | SEQ ID NO: 27626 | SEQ ID NO: 31632 |
| **iPS:436992** | **21-225_191B8** | NA | TCTTCTGAGCTGACTCAGGACCCTGCTGTGTCTGTGGCCTTGGGACAGACAGTCAGGATCACATGCCAAGGAGACACCCTCAGACCCTATTATGCAAGTTGGTACCAGCAGAAGCCAGGACAGGCCCCTGTACTTGTCATCTATGGTAAAAACAACCGGCCCTCAGGGATCTCAGACCGATTCTCTGGCTCCAGCTCAGGAAACACAGCTTCCTTGACCATCACTGGGGCTCAGGCGGAAGATGAGGCTGACTATTACTGTAACTCCCGGGACAGCAGTGGTAACCATCTTGTGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCCTCAGTAGCTATGGCATGCACTGGGTCCGCCAGGCTCCAGGCAAGGGGCTGGAGTGGGTGGCAGTTATATGGTTTGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATAACTGGAACTACGGCGATCACTACTACTACTACGGTATGGACGTCTGGGGCCAAGGGACCACGGTCACCGTCTCCTCA |
| | | | SEQ ID NO: 27627 | SEQ ID NO: 31633 |
| | | AA | SSELTQDPAVSVALGQTVRITCQGDTLRPYYASWYQQKPGQAPVLVIYGKNNRPSGISDRFSGSSSGNTASLTITGAQAEDEADYYCNSRDSSGNHLVVFGGGTKLTVL | QVQLVESGGGVVQPGRSLRLSCAASGFTLSSYGMHWVRQAPGKGLEWVAVIWFDGSNKYYADSVKGRFTISRDNSKNTLYLQMNSLRAEDTAVYYCARDNWNYGDHYYYYGMDVWGQGTTVTVSS |
| | | | SEQ ID NO: 27628 | SEQ ID NO: 31634 |

FIGURE 50

| | | | | |
|---|---|---|---|---|
| **iPS:436994** | **21-225_191A9** | NA | TCTTCTGAGCTGACTCAGGACCCTGCTGTGTCTGTGGCCTTGGGACAGACAGTCAGGATCACATGCCAAGGAGACAGCCTCAGACCCTATTATGCAAGCTGGTACCAGCAGAAGCCAGGACAGGCCCCTGTACTTGTCATCTATGGTAAAAACAACCGGCCCTCAGGGATCCCAGACCGATTCTCTGGCTCCAGCTCAGGAAACACAGCTTCCTTGACCATCACTGAGGCTCAGGCGGAAGATGAGGCTGACTATTACTGTAACTCCCGGGACAGCTGTGGTAACCATCTTGTGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCCTCAGTAATTATGGCATGCACTGGGTCCGCCAGGCTCCAGGCAAGGGGCTGGAGTGGGTGGCAGTTATATGGTTTGGTGGAGATAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTTTTACTGTGCGAGAGATAACTGGAACTACGGCGATCACTACTACTATTACGGTATGGACGTCTGGGGCCAAGGGACCACGGTCACCGTCTCCTCA |
| | | | SEQ ID NO: 27629 | SEQ ID NO: 31635 |
| | | AA | SSELTQDPAVSVALGQTVRITCQGDSLRPYYASWYQQKPGQAPVLVIYGKNNRPSGIPDRFSGSSSGNTASLTITEAQAEDEADYYCNSRDSCGNHLVVFGGGTKLTVL | QVQLVESGGGVVQPGRSLRLSCAASGFTLSNYGMHWVRQAPGKGLEWVAVIWFGGDNKYYADSVKGRFTISRDNSKNTLYLQMNSLRAEDTAVFYCARDNWNYGDHYYYYGMDVWGQGTTVTVSS |
| | | | SEQ ID NO: 27630 | SEQ ID NO: 31636 |
| **iPS:436996** | **21-225_191B9** | NA | CAGTCTGTGTTGACGCAGCCGCCCTCAGTGTCTGCGGCCCCAGGACAGAAGGTCACCATCTCCTGCTCTGGAAGCAGCTCCAACATCGGGAATAATTATGTATCCTGGTACCAGCAGCTCCCAGGAACAGCCCCCAAACTCCTCATTTATGACAATAAAAAGCGACCCTCAGGGATTCCTGACCGATTCTCTGGCTCCAAGTCTGGCACGTCAGCCACCCTGGGCATCACCGGACTCCAGACTGGGGACGAGGCCGATTATTACTGCGGAACATGGGATAGCAGCCTGAGTGTTTGTGTCTTCGGAACTGGGACCAAGGTCACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTTTCCATGGCATGCACTGGGTCCGCCAGGCTCCAGGCAAGGGGCTGGAGTGGGTGGCAGTTATATGGTATGATGGAAGTAAAAAATACTATGCAGACTCCGTGAAGGGCCGATTCACCATCTCCAGAGACAATTCCAAGAACACGCTGCATCTGCAAATGAACAGTCTGAGAGCCGAGGACACGGCTGTGTATTACTGTGCGAAAGAAGGGTATAGCAGTGGCTTTTACAGGGGGTTTGACAACTGGGGCCAGGGAACCCTGGTCACCGTCTCCTCA |
| | | | SEQ ID NO: 27631 | SEQ ID NO: 31637 |

FIGURE 50

| | | | | |
|---|---|---|---|---|
| | | AA | QSVLTQPPSVSAAPGQKVTISCSGSSSNIGNNYVS<br>WYQQLPGTAPKLLIYDNKKRPSGIPDRFSGSKSG<br>TSATLGITGLQTGDEADYYCGTWDSSLSVCVFG<br>TGTKVTVL | QVQLVESGGGVVQPGRSLRLSCAASGFTFSFHGMH<br>WVRQAPGKGLEWVAVIWYDGSKKYYADSVKGRF<br>TISRDNSKNTLHLQMNSLRAEDTAVYYCAKEGYSS<br>GFYRGFDNWGQGTLVTVSS |
| | | | SEQ ID NO: 27632 | SEQ ID NO: 31638 |
| **iPS:437000** | **21-225_191G9** | NA | CAGGCTGTGTCGACTCGGCCGTCTTCCCTCTCT<br>GCATCTCCTGGAGCATCAGCCAGTCTCACCTG<br>CACCTTACGCAGTGGCATCAATGTTGGTACCT<br>ACAGGATATACTGGTACCAGCAGAAGCCAGG<br>GAGTCCTCCCCAGTATCTCCTGAGGTACAAAT<br>CAGACTCAGATAAGCAGCAGGGCTCTGGAGTC<br>CCCAGCCGCTTCTCTGGATCCAAAGATGCTTC<br>GGCCAATGCAGGGATTTTACTCATCTCTGGGC<br>TCCAGTCTGAGGATGAGGCTGACTATTACTGT<br>ATGATTTGGCACAGCAGCGCTGTGGTATTCGG<br>CGGAGGGACCAAGCTGACCGTCCTA | CAGGTGCAGCTGGTGGAGTCTGGGGGAGGCGTG<br>GTCCAGCCTGGGAGGTCCCTGAGACTCTCCTGTG<br>CAGCGTCTGGATTCACCTTCAGTACCTATGGCAT<br>GCACTGGGTCCGCCAGGCTCCAGGCAAGGGGCT<br>GGAGTGGGTGACACTTATATGGTTTGATGGAAGT<br>AATAAATACTATGCAGACTCCGTGAAGGGCCGA<br>TTCACCATCTCCAGAGACAATTCCAAGAACACGC<br>TGTATCTGCAAATGAACAGCCTGAGAGCCGAGG<br>ACACGGCTGTGTATTACTGTGCGAGAGATCGGGT<br>GGGAGGTACTAGTCCTCCTTACTACTACTACTAC<br>GGTATGGACGTCTGGGGCCAAGGGACCACGGTC<br>ACCGTCTCCTCA |
| | | | SEQ ID NO: 27633 | SEQ ID NO: 31639 |
| | | AA | QAVSTRPSSLSASPGASASLTCTLRSGINVGTYRI<br>YWYQQKPGSPPQYLLRYKSDSDKQQGSGVPSRF<br>SGSKDASANAGILLISGLQSEDEADYYCMIWHSS<br>AVVFGGGTKLTVL | QVQLVESGGGVVQPGRSLRLSCAASGFTFSTYGMH<br>WVRQAPGKGLEWVTLIWFDGSNKYYADSVKGRFT<br>ISRDNSKNTLYLQMNSLRAEDTAVYYCARDRVGG<br>TSPPYYYYYGMDVWGQGTTVTVSS |
| | | | SEQ ID NO: 27634 | SEQ ID NO: 31640 |

FIGURE 50

| | | | | |
|---|---|---|---|---|
| iPS:437002 | 21-225_191H9 | NA | CAGACTGTGGTGACTCAGGAGCCCTCACTGACTGTGTCCCCAGGAGGGACAGTCACTCTCACCTGTGCTTCCAGCACTGGAGCAGTCACCAGTGCTTACTATCCAAACTGGTTGCAGCAGAAACCTGGACAAGCACCCAGGACACTGATTTATAGTACAAACAACAAACACTCCTGGACCCCTGCCCGGTTCTCAGGCTCCCTCCTTGGGGGCAAAGCTGCCCTGACACTGTCAGATGTGCAGCCTGAGGACGAGGCTGAGTATTACTGCCTGATCTTCTATGGTGGTGTACATGTGATATTTGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGTAAGGTTTCTGGATATACCTTCACCGGCTACAATATGCACTGGGTGCGACAGGCCCCTGGACAAGGGCTTGAGTGGATGGGATGGATCAACCCTAATAGTGGTGGCACAAACTATGCACACAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGAGATTTCTATGATAGTGGTGGAGAAGGGTGGTTCGACCCCTGGGGCCAGGGAACCCTGGTCACCGTCTCCTCA |
| | | | SEQ ID NO: 27635 | SEQ ID NO: 31641 |
| | | AA | QTVVTQEPSLTVSPGGTVTLTCASSTGAVTSAYYPNWLQQKPGQAPRTLIYSTNNKHSWTPARFSGSLLGGKAALTLSDVQPEDEAEYYCLIFYGGVHVIFGGGTKLTVL | QVQLVQSGAEVKKPGASVKVSCKVSGYTFTGYNMHWVRQAPGQGLEWMGWINPNSGGTNYAHKFQGRVTMTRDTSISTAYMELSRLRSDDTAVYYCARDFYDSGGEGWFDPWGQGTLVTVSS |
| | | | SEQ ID NO: 27636 | SEQ ID NO: 31642 |
| iPS:437006 | 21-225_192G2 | NA | CAGTCTGTGTTGACGCAGCCGCCCTCAGTGTCTGCGGCCCCAGGACAGAAGGTCACCATCTCCTGCTCTGGAAGCAGCTCCAACATTGGGAATAATTATGTATCCTGGTACCAGCAGCTCCCAGGAACAGCCCCCAAACTCCTCATTTATGACAATAATAAGCGACCCTCAAGGATTCCTGACCGATTCTCTGGCTCCAAGTCTGGCACGTCAGCCACCCTGGGCATCACCGGACTCCAGACTGGGGACGAGGCCGATTATTACTGCGGAACATGGGATAGCAGCCTGAGTGCTGGGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGACAGTTATATGGAATGATGGAAGTAATAAATACTATGCAGACTCCGTGAAGGGCCGATTTACCATCTCCAGAGACAATTCCAAGAACACGCTGTATCTGCAAATGAACAGCCTGAGAGCCGAGGACACGGCTCTGTATTACTGTGCGAGAGATCTGGATAAGAGGAACTTTCCTTATTACTACTACTACGGTATGGACGTCTGGGGCCAAGGGACCACGGTCACCGTCTCCTCA |
| | | | SEQ ID NO: 27637 | SEQ ID NO: 31643 |

FIGURE 50

| | | | | |
|---|---|---|---|---|
| | | AA | QSVLTQPPSVSAAPGQKVTISCSGSSSNIGNNYVSWYQQLPGTAPKLLIYDNNKRPSRIPDRFSGSKSGTSATLGITGLQTGDEADYYCGTWDSSLSAGVFGGGTKLTVL | QVQLVESGGGVVQPGRSLRLSCAASGFTFSSYGMHWVRQAPGKGLEWVTVIWNDGSNKYYADSVKGRFTISRDNSKNTLYLQMNSLRAEDTALYYCARDLDKRNFPYYYYYGMDVWGQGTTVTVSS |
| | | | SEQ ID NO: 27638 | SEQ ID NO: 31644 |
| **iPS:437008** | **21-225_192E3** | NA | CAGACTGTGGTGACTCAGGAGCCCTCACTGACTGTGTCCCCAGGAGGGACAGTCACTCTCACCTGTGCTTTCAGCACTGGATCAGTCACCAGTGGTTCCTATCCAAACTGGTTCCAGCAGAAACCTGGACAAGCACCCAGGGCACTGATTTATAGTACAAACAACAAACACTCCTGGACCCCTGCCCGGTTCTCAGGCTCCCTCCTTGGGGGCAAAGCTGCCCTGACACTGTCAGGTGTGCAGCGTGAGGACGAGGCTGAGTATTACTGCCTGCTATACTATGGTGGTGCTCAGCTGGTGTTCGGCGGAGGGACCAAGCTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGGTTACTACTGGAGCTGGATCCGCCAGCCCCCAGGGAAGGGCCTGGAGTGGATTGGGTACATCTATTACACTGGGAGCACCTACTACAACCCGTCCCTCAAGAGTCGAGTTACCATATCAGTAGACACGTCTAAGAACCAGTTCTCCCTGAAGCTGAGCTCTGTGACTGCCGCGGACACGGCCGTGTATTACTGTGCGAGAGACGATCCCCTCTACGGAATGGACGTCTGGGGCCAAGGGACCACGGTCACCGTCTCCTCA |
| | | | SEQ ID NO: 27639 | SEQ ID NO: 31645 |
| | | AA | QTVVTQEPSLTVSPGGTVTLTCAFSTGSVTSGSYPNWFQQKPGQAPRALIYSTNNKHSWTPARFSGSLLGGKAALTLSGVQREDEAEYYCLLYYGGAQLVFGGGTKLTVL | QVQLQESGPGLVKPSQTLSLTCTVSGGSISSGGYYWSWIRQPPGKGLEWIGYIYYTGSTYYNPSLKSRVTISVDTSKNQFSLKLSSVTAADTAVYYCARDDPLYGMDVWGQGTTVTVSS |
| | | | SEQ ID NO: 27640 | SEQ ID NO: 31646 |

FIGURE 50

| | | | | |
|---|---|---|---|---|
| iPS:437010 | 21-225_192G3 | NA | CAGTCTGTGCTGACTCAGCCACCCTCAGCGTCTGGGACCCCCGGACAGAGGGTCACCATGTCTTGTTCTGGAAGCAGCTCCAACATCGGAAGTAATACTGTAAACTGGTACCAACAATTCCCAGGAACGGCCCCCAAACTCCTCATCTATGGTAATAAGCAGCGGCCCTCAAGGGTCCCTGACCGATTCTCTGGCTCCAAGTCTGGCACCTCAGCCTCCCTGGCCATCAGTGGGCTCCAGTCTGAGGATGAGACTGATTATTACTGTGCAGCGTGGGATGACAGCCTGAATGGTTGGGTGTTCGGCGGAGGGACCAAGCTGACCGTCCTA | CAGGTGCAGCTGCAGGAGTCGGGCCCAGGACTGGTGAAGCCGTCGGAGACCCTGTCCCTCACCTGCACTGTCTCTGGTGGCTCCATCAGTAATTACTACTGGAGCTGGATCCGGCAGCCCGCCGGGAAGGGACTGGAGTGGATTGGGCGGATCTATTCCAGTGGGAGCACCAACTACAACCCCTCCCTCAAGAGTCGAGTCACCATGTCAGTAGACACGTCCAAGAACCAGTTCTCCCTGAAGCTGAACTCTGTGACCGCCGCGGACACGGCCGTGTATTACTGTGCGAAAGGGTGGGAGCTAAACTACTGGGGCCAGGGAACCCTGGTCACCGTCTCCTCA |
| | | | SEQ ID NO: 27641 | SEQ ID NO: 31647 |
| | | AA | QSVLTQPPSASGTPGQRVTMSCSGSSSNIGSNTVNWYQQFPGTAPKLLIYGNKQRPSRVPDRFSGSKSGTSASLAISGLQSEDETDYYCAAWDDSLNGWVFGGGTKLTVL | QVQLQESGPGLVKPSETLSLTCTVSGGSISNYYWSWIRQPAGKGLEWIGRIYSSGSTNYNPSLKSRVTMSVDTSKNQFSLKLNSVTAADTAVYYCAKGWELNYWGQGTLVTVSS |
| | | | SEQ ID NO: 27642 | SEQ ID NO: 31648 |
| iPS:437012 | 21-225_192G7 | NA | CAGACTGTGGTGACTCAGGAGCCCTCACTGACTGTGTCCCCAGGAGGGACAGTCACTCTCACCTGTGCTTCCAGCACTGGAGCAGTCACCAGTGGTAACTATCCACAGTGGTTCCAGCAGAAACCTGGACAAGCACCCAGGGCACTGATTTACAGTACAACCAACAGACATTCCTGGACCCCTGCCCGGTTTTCAGGCTCCCTCCTGGGGGGCAAAGCTGCCCTGACACTGTCAGGTGTGCAGCCTGAGGACGAGGCTGAGTATTACTGCCTGTTCTACTATGGTGGTGCTCAGGTGATATTCGGCGGAGGGACCAAGCTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGGTTACTACTGGAGCTGGATCCGCCAGCACCCAGGGAAGGGCCTGGAGTGGATTGGGTACATCTATTACAGAGGGAGTACCTACTACAATCCGTCCCTCAAGAGTCGAGTTACCATATCAGTGGACACGTCTAAGAACCAGTTCTCCCTGAAACTGAACTCTGTGACTGCCGCGGACACGGCCGTGTATTACTGTGCGAGAGACTCCCCGGTGACAGGATTTGACTATTGGGGCCAGGGAATCCTGGTCACCGTCTCCTCA |
| | | | SEQ ID NO: 27643 | SEQ ID NO: 31649 |

FIGURE 50

| | | | | |
|---|---|---|---|---|
| | | AA | QTVVTQEPSLTVSPGGTVTLTCASSTGAVTSGNYPQWFQQKPGQAPRALIYSTTNRHSWTPARFSGSLLGGKAALTLSGVQPEDEAEYYCLFYYGGAQVIFGGGTKLTVL | QVQLQESGPGLVKPSQTLSLTCTVSGGSISSGGYYWSWIRQHPGKGLEWIGYIYYRGSTYYNPSLKSRVTISVDTSKNQFSLKLNSVTAADTAVYYCARDSPVTGFDYWGQGILVTVSS |
| | | | SEQ ID NO: 27644 | SEQ ID NO: 31650 |
| **iPS:437014** | **21-225_192H8** | NA | CAGACTGTGGTGACTCAGGAACCCTCACTGACTGTGTCCCCAGGAGGGACAGTCACTCTCACCTGTGCTTTCAGCACTGGAACAGTCACCAGTGGTTTCTATCCAAACTGGTTCCAGCAGAAACCTGGACAAGCACCCAGGGCATTGATTTATAATACAAGCAACAGACACTCCTGGACCCCTGCCCGGTTCTCAGGCTCCCTCCTTGGGGGCATGGCTGCCCTGACACTGTCAGGTGTGCAGCCTGAGGACGAGGCTGATTATTACTGCCTGCTGTACTATGGTGGTGCTCAGCTGATGTTCGGCGGAGGGACCAAGCTGACCGTCCTA | CAGGTGCAGCTGCAGGAGTCGGGCCCAGGAGTGGTGAAGCCTTCACAGACCCTGTCCCTCACCTGCACTGTCTCTGGTGGCTCCATCAGCAGTGGTGGTGACTACTGGAGCTGGATCCGCCAGCACCCAGGGAAGGGCCTGGAGTGGATTGGGTACATCTATTACAGTGGGCCCACCTACTACAACCCGTCCCTCAAGAGTCGACTTACCATGTCAGCAGACACGTCTAAGAACCAGTTCTCCCTGAAGCTGAGCTCTGTGACTGCCGCGGACACGGCCGTGTATTACTGTGCGAGAGATAGCTCCCTCTACGGTATGGACGTCTGGGGCCAAGGGACAACGGTCACCGTCTCCTCA |
| | | | SEQ ID NO: 27645 | SEQ ID NO: 31651 |
| | | AA | QTVVTQEPSLTVSPGGTVTLTCAFSTGTVTSGFYPNWFQQKPGQAPRALIYNTSNRHSWTPARFSGSLLGGMAALTLSGVQPEDEADYYCLLYYGGAQLMFGGGTKLTVL | QVQLQESGPGVVKPSQTLSLTCTVSGGSISSGGDYWSWIRQHPGKGLEWIGYIYYSGPTYYNPSLKSRLTMSADTSKNQFSLKLSSVTAADTAVYYCARDSSLYGMDVWGQGTTVTVSS |
| | | | SEQ ID NO: 27646 | SEQ ID NO: 31652 |

FIGURE 50

| | | | | |
|---|---|---|---|---|
| **iPS:437016** | **21-225_193A6** | NA | TCTTCTGAGCTGACTCAGGACCCTGCTGTGTCTGTGGCCTTGGGACAGACAGTCAGGATCACATGCCAAGGAGACAGCCTCAGAAGCTATTATGCAAACTGGTACCAGCAGAAGCCAGGACAGGCCCCTGTACTTTTCATCTATGCTAAGAACAACCGGCCCTCAGGGATCCCAGACCGATTCTCTGGCTCCAACTCAGGAAACACAGCTTCCTTGACCATCACTGGGGCTCAGGCGGAAGATGAGGCTGACTATTACTGTAATTCCCGGGACAGCAGTGGTAACCATCTGGTATTCGGCGGAGGGACCAAGCTGACCGTCCTA | CAGGTGCAGCTGCAGGAGTCGGGCCCAGGACTGGTGAAGCCTTCGGAGACCCTGTCCCTCACCTGCACTGTCTCTGGTGGCTCCATCAGTAGTTACTACTGGAGCTGGATCCGGCAGCCCCCAGGGAAGGGACTGGAGTGGATTGGGTATATCTATTACAGTGGGAGCACCAACTACAACCCCTCCCTCAAGAGTCGAGTCACCATATCAGTAGACACGTCCAAGAACCAGTTCTCCCTGAAGCTGAGCTCTGTGACCGCTGCGGACACGGCCGTGTATTACTGTGCGGGAGGATGGGAGCTAAACTACTGGGGCCAGGGAACCCTGGTCACCGTCTCCTCA |
| | | | SEQ ID NO: 27647 | SEQ ID NO: 31653 |
| | | AA | SSELTQDPAVSVALGQTVRITCQGDSLRSYYANWYQQKPGQAPVLFIYAKNNRPSGIPDRFSGSNSGNTASLTITGAQAEDEADYYCNSRDSSGNHLVFGGGTKLTVL | QVQLQESGPGLVKPSETLSLTCTVSGGSISSYYWSWIRQPPGKGLEWIGYIYYSGSTNYNPSLKSRVTISVDTSKNQFSLKLSSVTAADTAVYYCAGGWELNYWGQGTLVTVSS |
| | | | SEQ ID NO: 27648 | SEQ ID NO: 31654 |
| **iPS:437018** | **21-225_193H5** | NA | TCCTATGAACTGACTCAGCCATCCTCAGTGTCCGTGTCCCCAGGACAGACAGCCAGCATCACCTGCTCTGGAGATAAATTGGGGGATAGATTTGCTTGCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTGCGGTATTCGGCGGAGGGACCAAGCTGACCGTCCTA | GAGGTGCAGCTGGTGGAGTCTGGGGGAGGCTTGGTAATGCCTGGGGGGTCCCTTAGCCTCTCCTGTGCAGCCTCTGGATTCACTTTCAGTAACGCCTACATGACCTGGGTCCGCCAGGCTCCAGGGAAGGGGCTGGAGTGGGTTGGCCGTATTAAAAGCAAAACTGATGGTGGGACAACAGACTACGCTGCACCCGTGAAAGGCAGATTCACCATCTCAAGAGATGATTCAAAAAACACGCTGTATCTGCAAATGAACAGCCTGAAAACCGAGGACACAGCCGTGTATTACTGTACCACAGATCCCGGTGGTATCTTTGACTACTGGGGCCAGGGAACCCTGGTCACCGTCTCCTCA |
| | | | SEQ ID NO: 27649 | SEQ ID NO: 31655 |

FIGURE 50

| | | | | |
|---|---|---|---|---|
| | | AA | SYELTQPSSVSVSPGQTASITCSGDKLGDRFACWYQQKPGQSPVLVIYQDSKRPSGIPERFSGSNSGNTATLTISGTQAMDEADYYCQAWDSSTAVFGGGTKLTVL | EVQLVESGGGLVMPGGSLSLSCAASGFTFSNAYMTWVRQAPGKGLEWVGRIKSKTDGGTTDYAAPVKGRFTISRDDSKNTLYLQMNSLKTEDTAVYYCTTDPGGIFDYWGQGTLVTVSS |
| | | | SEQ ID NO: 27650 | SEQ ID NO: 31656 |
| iPS:437020 | 21-225_193F11 | NA | CAGTATGTGTTGACGCAGCCGCCATCAGTGTCTGCGGCCCCAGGACAGAAGGTCACCATCTCCTGCTTCGGAGGCAGCTCCAACATTGGGAATAATTATGTATCCTGGTTCCAGCAGTTCCCAGGAACAGCCCCCAAATTCCTCATTTATGACAATAATAAGCGACCCTCAGGGATTCTTGACCGATTATCTGGCTCCAAGTCTGGCACGTCAGCCACCCTGGACATCACCGGACTCCAGAATGGGGACGAGGCCGATTATTACTGCGGAACATGGGATCGCACCATGAGTGATTGGGTATTCGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGACTTGAGTGGATGGGATGGATCAACCCTTACAGTGGTGGCACAAACTATGCACAGAAGTTTCAGGGCAGGGTCACCATGACCAGGGACACGTCCATCAGCACAGCCTACATGGAACTGAGCAGGCTGAGATCTGACGACACGGCCGTGTATTACTGTGCGAGAGATAGAGCAGTGGCTGGAAACTACTTCTACTACGGTATGGACGTCTGGGGCCAAGGGACCACGGTCACCGTCTCCTCA |
| | | | SEQ ID NO: 27651 | SEQ ID NO: 31657 |
| | | AA | QYVLTQPPSVSAAPGQKVTISCFGGSSNIGNNYVSWFQQFPGTAPKFLIYDNNKRPSGILDRLSGSKSGTSATLDITGLQNGDEADYYCGTWDRTMSDWVFGGGTKLTVL | QVQLVQSGAEVKKPGASVKVSCKASGYTFTGYYMHWVRQAPGQGLEWMGWINPYSGGTNYAQKFQGRVTMTRDTSISTAYMELSRLRSDDTAVYYCARDRAVAGNYFYYGMDVWGQGTTVTVSS |
| | | | SEQ ID NO: 27652 | SEQ ID NO: 31658 |

FIGURE 50

| | | | | |
|---|---|---|---|---|
| iPS:437022 | 21-225_194G5 | NA | CAGACTGTGGTGACTCAGGAGCCCTCACTGACTGTGTCCCCAGGAGGGACAGTCACTCTCACCTGTGCTTCCAGCACTGGAGCAGTCACCAGTGGTAACTATCCAAACTGGTTCCAGCAGAAACCTGGACAAACACCCAGGGCACTGATTTATAGTACAAGCAACAAACACTCCTGGACCCCTGCCCGGTTCTCAGGCTCCCTCCTTGGGGGCAAAGGTGCCCTGACACTGTCAGGTGTGCAGCCTGAGGACGAGGCTGAGTATTACTGCCTGATCTACTATGGTGGTGCTCAGCTGATGTTCGGCGGAGGGACCAAGCTGACCGTCCTA | CAGGTGCAGCTGCAGGAGTCGGGCCCAGGACTGGTGAAGCCTTCACAGACCCTGTCCCTCATCTGCACTGTCTCTGGTGGCTCCATCAGAAGTGGTGGTGACTACTGGAGCTGGATCCGCCAGCACCCAGGGAAGGGCCTGGAGTGGATTGGGTACATCTATTACAGTGGGAGCACCTACTACAACCCGTCTCTCAAGAGTCGAGTTACCATATCAGTAGACACGTCTAAGAACCAGTTCTCCCTGAAGCTGAGCTCTGTGACTGCCGCGGACACGGCCGTTTATTACTGTGCGAGAGATCACTCCCTCTACGGTATGGACGTCTGGGGCCAAGGGACCACGGTCACCGTCTCCTCA |
| | | | SEQ ID NO: 27653 | SEQ ID NO: 31659 |
| | | AA | QTVVTQEPSLTVSPGGTVTLTCASSTGAVTSGNYPNWFQQKPGQTPRALIYSTSNKHSWTPARFSGSLLGGKGALTLSGVQPEDEAEYYCLIYYGGAQLMFGGGTKLTVL | QVQLQESGPGLVKPSQTLSLICTVSGGSIRSGGDYWSWIRQHPGKGLEWIGYIYYSGSTYYNPSLKSRVTISVDTSKNQFSLKLSSVTAADTAVYYCARDHSLYGMDVWGQGTTVTVSS |
| | | | SEQ ID NO: 27654 | SEQ ID NO: 31660 |
| iPS:437024 | 21-225_194F11 | NA | CAGTCTGTGTTGACGCAGCCGCCCTCAGTGTCTGCGGCCCCAGGACAGAAGGTCACCATCTCCTGCTCTGGAAGCAGCTCCAACATTGGGAATAATTATGTATCCTGGTACCAGCAGCTCCCAGGAACAGCCCCCAAACTCCTCATTTATGACAATAATAAGCGACCCTCAGGGATTCCTGACCGATTCTCTGGCTCCAAGTCTGGCACGTCAGCCACCCTGGGCATCACCGGACTCCAGACTGGGGACGAGGCCGATTATTACTGCGGAACATGGGATAGCAGCCTGAGTGCTGGGGTTTTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGAATGATGGAAGTAAAAAATACCATGTAGACTCCGTGAAGGGCCGATTTACCATCTCCAGAGACAATTCCAAGAACACGCTGTATCTGCAAATGAACAGCCTGAGAGCCGAGGACACGGCTCTGTATTACTGTGCGAGAGATCTGGATAAGAGGAACTTTCCTTATTACTACTACTACGGTATGGACGTCTGGGGCCAAGGGACCACGGTCACCGTCTCCTCA |
| | | | SEQ ID NO: 27655 | SEQ ID NO: 31661 |

FIGURE 50

| | | | | |
|---|---|---|---|---|
| | | AA | QSVLTQPPSVSAAPGQKVTISCSGSSSNIGNNYVSWYQQLPGTAPKLLIYDNNKRPSGIPDRFSGSKSGTSATLGITGLQTGDEADYYCGTWDSSLSAGVFGGGTKLTVL | QVQLVESGGGVVQPGRSLRLSCAASGFTFSSYGMHWVRQAPGKGLEWVAVIWNDGSKKYHVDSVKGRFTISRDNSKNTLYLQMNSLRAEDTALYYCARDLDKRNFPYYYYYGMDVWGQGTTVTVSS |
| | | | SEQ ID NO: 27656 | SEQ ID NO: 31662 |
| **iPS:437026** | **21-225_194D12** | NA | CAGACTGTGGTGACTCAGGAGCCCTCACTGACTGTGTCCCCAGGAGGGACAGTCACTCTCACCTGTGCTTCCAGCACTGGAGCAGTCACCAGTGGTTCCTTTCCAAGCTGGTTCCAGCAGAAACCTGGACAAGCACCCAGGGCACTGATTTATAGTACAAGCAACAGACACTCCTCGACCCCTGCCCGGTTCTCAGGCTCCCTCCTTGGGGGCAAAGCAGCCCTGACACTGTCAGGTGTGCAGCCTGAGGACGAGGCTGACTATTACTGCCTGATCTACTATGGTGGTGCTCAGCTGGCATTCGGCGGAGGGACCAAGCTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGGTGACTACTGGAGCTGGATCCGCCAGCACCCAGGGAAGGGCCTGGAGTGGATTGGGTACATCTATTACAGTGGGAGTACCTACTACAACCCGTCCCTCAAGAGTCGAGTTACCATATCAGTAGACACGTCTAAGAACCAGTTCTCCCTGAAGCTGAGCTCTGTGACTGCCGCGGACACGGCCGTGTATTACTGTGCGAGAGATGGGGCTCGGCACGGTATGGACGTCTGGGGCCAAGGGACCACGGTCACCGTCTCCTCA |
| | | | SEQ ID NO: 27657 | SEQ ID NO: 31663 |
| | | AA | QTVVTQEPSLTVSPGGTVTLTCASSTGAVTSGSFPSWFQQKPGQAPRALIYSTSNRHSSTPARFSGSLLGGKAALTLSGVQPEDEADYYCLIYYGGAQLAFGGGTKLTVL | QVQLQESGPGLVKPSQTLSLTCTVSGGSISSGGDYWSWIRQHPGKGLEWIGYIYYSGSTYYNPSLKSRVTISVDTSKNQFSLKLSSVTAADTAVYYCARDGARHGMDVWGQGTTVTVSS |
| | | | SEQ ID NO: 27658 | SEQ ID NO: 31664 |

FIGURE 50

| | | | | |
|---|---|---|---|---|
| **iPS:437028** | **21-225_194G12** | NA | CAGTCTGTGTTGACGCAGCCGCCCTCAGTGTCTGCGGCCCCAGGACAGAAGGTCACCATCTCCTGCTCTGGAAGCAGCTCCAACATTGGGAATAATTATGTATCCTGGTACCAGCAGCTCCCAGGAACAGCCCCCAAACTCCTCATTTATGACAATAATAAGCGACCCTCAAGGATTCCTGACCGATTCTCTGGCTCCAAGTCTGGCACGTCAGCCACCCTGGGCATCACCGGACTCCAGACTGGGGAGGAGGCCGATTATTACTGCGGAACATGGGATAGCAGCCTGAGTGTTGGGGTATTCGGCGGAGGGACCAAGCTGACCGTCTTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGACAGTTATATGGAATGATGGAAGTAATAAATACTATGCAGACTCCGTGAAGGGCCGATTTACCATCTCCAGAGACAATTCCAAGAACACGCTGTATCTGCAAATGAACAGCCTGAGAGCCGAGGACACGGCTCTGTATTACTGTGCGAGAGATCTGGATAAGAGGAACTTTCCTTATTACTACTACTACGGTATGGACGTCTGGGGCCAAGGGACCACGGTCACCGTCTCCTCA |
| | | | SEQ ID NO: 27659 | SEQ ID NO: 31665 |
| | | AA | QSVLTQPPSVSAAPGQKVTISCSGSSSNIGNNYVSWYQQLPGTAPKLLIYDNNKRPSRIPDRFSGSKSGTSATLGITGLQTGEEADYYCGTWDSSLSVGVFGGGTKLTVL | QVQLVESGGGVVQPGRSLRLSCAASGFTFSSYGMHWVRQAPGKGLEWVTVIWNDGSNKYYADSVKGRFTISRDNSKNTLYLQMNSLRAEDTALYYCARDLDKRNFPYYYYYGMDVWGQGTTVTVSS |
| | | | SEQ ID NO: 27660 | SEQ ID NO: 31666 |
| **iPS:437030** | **21-225_195E3** | NA | TCCTATGAGCTGACTCAGCCACCCTCAGTGTCCGTGTCCCCAGGACAGACAGCCAGCATCACCTGCTCTGGAGATAAATTGGGGTATAGATCTGTTTGCTGGTATCAGCAGAAGCCAGGCCAGTCCCCTGTGCTGGTCATCTATGAAGATAGCAAGCGACCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGTGTCACTGTGGTATTCGGCGGAGGGACCAAGCTGATCGTCCTA | CAGGTGCAGCTGGTGGAGTCTGGGGGAGGCTTGGTCAAGCCTGGAGGGTCCCTGAGACTCTCCTGTGCAGCCTCTGGATTCACCTTCAGTGACTACTACATGAGCTGGATCCGCCAGGCTCCAGGGAAGGGGCTGGAGTGGGTTTCATATATTACTAGTAGTGGTAATACCATATACTACGCAGACTCTGTGAAGGGCCGATTCACCATCTCCAGGGACAACGCCAAGAACTCACTGTATCTGCAAATGAACAGCCTGAGAGCCGAGGACACGGCCGTATATTACTGTGCGAGAGATAGTCGATATTTTGACTGGTTTGACTACTGGGGCCAGGGAACCCTGGTCACCGTCTCTTCA |
| | | | SEQ ID NO: 27661 | SEQ ID NO: 31667 |

FIGURE 50

| | | | | |
|---|---|---|---|---|
| | | AA | SYELTQPPSVSVSPGQTASITCSGDKLGYRSVCWYQQKPGQSPVLVIYEDSKRPSGIPERFSGSNSGNTATLTISGTQAMDEADYYCQAWDSVTVVFGGGTKLIVL | QVQLVESGGGLVKPGGSLRLSCAASGFTFSDYYMSWIRQAPGKGLEWVSYITSSGNTIYYADSVKGRFTISRDNAKNSLYLQMNSLRAEDTAVYYCARDSRYFDWFDYWGQGTLVTVSS |
| | | | SEQ ID NO: 27662 | SEQ ID NO: 31668 |
| iPS:437032 | 21-225_195H6 | NA | CAGTCTGTGCTGACTCAGCCACCCTCAGCGTCTGGGACCCCCGGGCAGAGGGTCACCATCTCTTGTTCTGGAAGCAGCTCCAACATCGGAAGTCATACTGTAAACTGGTACCAGCAACTCCCAGGAACGGCCCCCAAACTCCTCATCTATAATAATTATCAGCGGCCCTCAGGGGTCCCTGACCGATTCTCTGGCTCCAAGTCTGGCACCTCAGCCTCCCTGACCATCAGTGGGCTCCAGTCTGAGGATGAGGCTGATTATTACTGTGCAACATGGGATGACAGCCTGAGTGTTTGGGTGTTCGGCGGAGGGACCAAGGTGACCGTCCTA | CAGGTGCAGCTGCAGGAGTCGGGCCCAGGACTGGTGAAGCCTTCGGAGACCCTGTCCCTCACCTGCACTGTCTCTGGTGGCTCCATCAGGAATTACTACTGGAGCTGGATCCGGCAGCCCGCCGGGAAGGGACTGGAGTGGATTGGGCGTATCTATAGCAGTGGGAGCACCAACTACAACCCCTCCCTCAAGAGTCGAGTCTCCATGTCAGTAGACACGTCCAAGAACCAGTTCTCCCTGAAGCTGAGTTCTGTGACCGCCGCGGACACGGCCGTGTATTACTGTACGAGAGGGTGGGAGCTAAACAACTGGGGCCAGGGAACCCTGGTCACCGTCTCCTCA |
| | | | SEQ ID NO: 27663 | SEQ ID NO: 31669 |
| | | AA | QSVLTQPPSASGTPGQRVTISCSGSSSNIGSHTVNWYQQLPGTAPKLLIYNNYQRPSGVPDRFSGSKSGTSASLTISGLQSEDEADYYCATWDDSLSVWVFGGGTKVTVL | QVQLQESGPGLVKPSETLSLTCTVSGGSIRNYYWSWIRQPAGKGLEWIGRIYSSGSTNYNPSLKSRVSMSVDTSKNQFSLKLSSVTAADTAVYYCTRGWELNNWGQGTLVTVSS |
| | | | SEQ ID NO: 27664 | SEQ ID NO: 31670 |

FIGURE 50

| | | | | |
|---|---|---|---|---|
| iPS:437034 | 21-225_195E9 | NA | TCCTATGAGCTGACTCAGCCACCCTCAGTGTCCGTGTCCCCAGGACAGACAGCCAGCATCACCTGCTCAGGAGATAAATTGGGGAATAAATATGCTTACTGGTATCAGCAGAAGCCAGGCCAGTCCCCTGTGCTGGTCATCTATCAAGATAGGAAGCGGCCCTCAGGGATCCCTGAGCGATTCTCTGGCTCCAATTCTGGGAACACAGGCACTTTGACCATCAGCGGGACCCAGGGTATGGATGAGGCTGACTATTACTGTCAGGCGTGGGACAGAGGAATTGTGGTATTCGGCGGAGGGACCAAGCTGACCGTCCTA | CAGGTGCAGGTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAACAGTGGTGCCACAAACTATGCACAGAAGTTTCAGGGCAGGGTCACCATGACCAGGGACACGTCCATCAGCACAGCCTACATGGAGCTGAACAGGCTGAGATCTGACGACACGGCCGTGTATTACTGTGCGAGAGCCTATTACTATGGTTCGGGGACTTATTATAACGAGTTCGACTACTGGGGCCAGGGAACCCTGGTCACCGTCTCCTCA |
| | | | SEQ ID NO: 27665 | SEQ ID NO: 31671 |
| | | AA | SYELTQPPSVSVSPGQTASITCSGDKLGNKYAYWYQQKPGQSPVLVIYQDRKRPSGIPERFSGSNSGNTGTLTISGTQGMDEADYYCQAWDRGIVVFGGGTKLTVL | QVQVVQSGAEVKKPGASVKVSCKASGYTFTGYYMHWVRQAPGQGLEWMGWINPNSGATNYAQKFQGRVTMTRDTSISTAYMELNRLRSDDTAVYYCARAYYYGSGTYYNEFDYWGQGTLVTVSS |
| | | | SEQ ID NO: 27666 | SEQ ID NO: 31672 |
| iPS:437036 | 21-225_195H9 | NA | CAGTATGTGTTGACGCAGCCGCCCTCAGTGTCTGCGGCCCCAGGACAGAAGGTCACCATCTCCTGTTCTGGAGGCAGCTCCAACATTGGGAATAATTATGTATCCTGGTTCCAGCAGTTCCCAGGAACAGCCCCCAAATTCCTCATTTATGACAATAATAAGCGACCCTCAGGGATTCTTGACCGATTCTCTGGCTCCAAGTCTGGCACGTCAGCCACCCTGGGCATCACCGGACTCCAGACTGGGGACGAGGCCGATTATTACTGCGGAACATGGGATCGCACCATGAGTGATTGGGTATTCGGCGGAGGGACCAAGTTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTTACAGTGGTGGCACAAACTATGCACAGAAGTTTCAGGACAGGGTCACCATGACCAGGGACACGTCCATCACCACAGCCTACATGGAACTGAGCAGGCTGAGATCTGACGACACGGCCGTGTATTACTGTGCGAGAGATAGAGCAGTGGCTGGAAACTACTTCTACTACGGTATGGACGTCTGGGGCCAGGGGACCACGGTCACCGTCTCCTCA |
| | | | SEQ ID NO: 27667 | SEQ ID NO: 31673 |

FIGURE 50

| | | | | |
|---|---|---|---|---|
| | | AA | QYVLTQPPSVSAAPGQKVTISCSGGSSNIGNNYVSWFQQFPGTAPKFLIYDNNKRPSGILDRFSGSKSGTSATLGITGLQTGDEADYYCGTWDRTMSDWVFGGGTKLTVL | QVQLVQSGAEVKKPGASVKVSCKASGYTFTGYYMHWVRQAPGQGLEWMGWINPYSGGTNYAQKFQDRVTMTRDTSITTAYMELSRLRSDDTAVYYCARDRAVAGNYFYYGMDVWGQGTTVTVSS |
| | | | SEQ ID NO: 27668 | SEQ ID NO: 31674 |
| iPS:437040 | 21-225_196E7 | NA | CAGACTGTGGTGACTCAGGAGCCCTCACTGACTGTGTCCCCAGGAGGGACAGTCACTCTCACCTGTGCTTCCAGCACTGGAGCAGTCACCAGTGCTTACTATCCAAACTGGTTGCAGCAGAAACCTGGACAAGCACCCAGGACACTGATTTATAGTACAAACAACAAACACTCCTGGACCCCTGCCCGGTTCTCAGGCTCCCTCCTTGGGGGCAAAGCTGCCCTGACACTGTCAGATGTGCAGCCTGAGGACGAGGCTGAGTATTACTGCCTGATTTTCTATGGTGGTGTACATGTGATATTTGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGTAAGGTTTCTGGATATACCTTCACCGGCTACAATATGCACTGGGTGCGACAGGCCCCTGGACAAGGGCTTGAGTGGATGGGATGGATCAACCCTAATAGTGGTGGCACAAACTATGCACACAAGTTTCAGGGCAGGGTCACCATGACCAGGGACACGTCCATCAGCACAGCCTACATGGAACTGAGCAGGCTGAGATCCGACGACACGGCCGTGTATTACTGTGCGAGAGATTACTATGATACTAGTGGAGAAGGGTGGTTCGACCCCTGGGGCCAGGGAACCCTGGTCACCGTCTCCTCA |
| | | | SEQ ID NO: 27669 | SEQ ID NO: 31675 |
| | | AA | QTVVTQEPSLTVSPGGTVTLTCASSTGAVTSAYYPNWLQQKPGQAPRTLIYSTNNKHSWTPARFSGSLLGGKAALTLSDVQPEDEAEYYCLIFYGGVHVIFGGGTKLTVL | QVQLVQSGAEVKKPGASVKVSCKVSGYTFTGYNMHWVRQAPGQGLEWMGWINPNSGGTNYAHKFQGRVTMTRDTSISTAYMELSRLRSDDTAVYYCARDYYDTSGEGWFDPWGQGTLVTVSS |
| | | | SEQ ID NO: 27670 | SEQ ID NO: 31676 |

FIGURE 50

| | | | | |
|---|---|---|---|---|
| **iPS:437042** | **21-225_197E8** | NA | CAGTCTGTGTTGACGCAGCCGCCCTCAGTGTCTGCGGCCCCAGGACAGAAGGTCACCATCTCCTGCTCTGGAAGCAGCTCCAACATTGGGAATAAATATGTATCCTGGTACCAGCAGTTCCCAGGAACAGCCCCCAAACTCCTCATTTATGACAATAATAAGCGACCCTCAAAGATTCCTGACCGATTCTCTGGCTCCAAATCTGGCACGTCAGCCACCCTGGGCATCACCGGACTCCTGACTGGGGACGAGGCCGATTATTACTGCGGAATATGGGATCGCAGTCTGAGTGTTATGGTGTTCGGCGGAGGGACCAAGCTGACCGTCCTA | CAGGTGCAGCTGCTGCAGTCTGGGGCTGAGGTGAGGAAGCCTGGGGCCTCAGTGAGGGTCTCCTGCAAGGCTTCTGGATACACCTTCACCGGCTACTATATACACTGGGTGCGACAGGCCCCTGGACAAGGGCTTGAGTGGATGGGATGGATCAACCCTAACAGTGGTGGCACAAACTATGCACAGAGGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGAGAGATAGCAGTGGCTGGGAACTACTTCTACTACGGTATGGGCGTCTGGGGCCAAGGGACCACGGTCGCCGTCTCCTCA |
| | | | SEQ ID NO: 27671 | SEQ ID NO: 31677 |
| | | AA | QSVLTQPPSVSAAPGQKVTISCSGSSSNIGNKYVSWYQQFPGTAPKLLIYDNNKRPSKIPDRFSGSKSGTSATLGITGLLTGDEADYYCGIWDRSLSVMVFGGGTKLTVL | QVQLLQSGAEVRKPGASVRVSCKASGYTFTGYYIHWVRQAPGQGLEWMGWINPNSGGTNYAQRFQGRVTMTRDTSISTAYMELSRLRSDDTAVYYCAREIAVAGNYFYYGMGVWGQGTTVAVSS |
| | | | SEQ ID NO: 27672 | SEQ ID NO: 31678 |
| **iPS:437044** | **21-225_197F9** | NA | CAGTCTGTGTTGACTCAGCCACCCTCAGCGTCTGGGACCCCCGGGCAGAGGGTCACCATCTCTTGTTCTGGAAGCAGCTCCAACATCGGAAGTAATACTGTAAACTGGTACCAGCAACTCCCAGGAACGGCCCCCAAACTCCTCATTTATAGTAATAATCAGCGGCCCTCAGGGGTCCCTGACCGATTCTCTGGCTCCAAGTCTGGCACCTCAGCCTCCCTGGCCATCAGTGGGCTCCAGTCTGAGGATGAGGCTGATTATTACTGTGCAGCATGGGATGACAGTATGAATGGTCCGGTGTTCGGCGGAGGGACCAAGCTGACCGTCCTA | CAGGTGCAGCTGCAGGAGTCGGGCCCAGGACTGGTGAAGCCTTCGGAGACCCTGTCCCTCACCTGCACTGTCTCTGGTGGCTCCATCAGAATTTACTACTGGAGCTGGATCCGGCAGCCCCCAGGGAAGGGACTGGAGTGGATTGGGTATGTCTATTACAGTGGGAGCACCACCTACAACCCCTCCCTCAAGAGTCGAGTCACCATATCAGTAGACACGTCCAAGAACCAGTTCTCCCTGAAGCTGAACTCTGTGACCGCTGCGGACACGGCCGTGTATTACTGTGTGAGAGAAAGGGGGAGTAGCCACAGATGGGGGGACTACTACGGAATGGACGTCTGGGGCCGAGGGACCACGGTCACCGTCTCCTCA |
| | | | SEQ ID NO: 27673 | SEQ ID NO: 31679 |

FIGURE 50

| | | | | |
|---|---|---|---|---|
| | | AA | QSVLTQPPSASGTPGQRVTISCSGSSSNIGSNTVNWYQQLPGTAPKLLIYSNNQRPSGVPDRFSGSKSGTSASLAISGLQSEDEADYYCAAWDDSMNGPVFGGGTKLTVL | QVQLQESGPGLVKPSETLSLTCTVSGGSIRIYYWSWIRQPPGKGLEWIGYVYYSGSTTYNPSLKSRVTISVDTSKNQFSLKLNSVTAADTAVYYCVRERGSSHRWGDYYGMDVWGRGTTVTVSS |
| | | | SEQ ID NO: 27674 | SEQ ID NO: 31680 |
| **iPS:437048** | **21-225_197B11** | NA | CAGACTGTGGTGACTCAGGAGCCCTCACTGACTGTGTCCCCAGGAGGGACAGTCACTCTCACCTGTGCTTTCAGCACTGGATCAGTCACCAGTGGTTCCTATCCAAACTGGTTCCAGCAGAAACCTGGACAAGCACCCAGGGCACTGATTTATAGTACAAACAACAAACACTCCTGGACCCCTGCCCGGTTCTCAGGCTCCCTCCTTGGGGGCAAAGCTGCCCTGACACTGTCAGGTGTGCAGCCTGAGGACGAGGCTGAGTATTACTGCCTGCTCTACTATGGTGGTGCTCAGCTGGTGTTCGGCGGAGGGACCAAGCTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGGTTACTACTGGAGCTGGATCCGCCAGCCCCCAGGGAAGGGCCTGGAGTGGATTGGGTACATCTATTACACTGGGAGCACCTACTACAACCCGTCCCTCAAGAGTCGAGTTACCATATCAGTAGACACGTCTAAGAACCAGTTCTCCCTGAAGCTGAGCTCTGTGACTGCCGCGGACACGGCCGTGTATTACTGTGCGAGAGACGATCCCCTCTACGGAATGGACGTCTGGGGCCAAGGGACCACGGTCACCGTCTCCTCA |
| | | | SEQ ID NO: 27675 | SEQ ID NO: 31681 |
| | | AA | QTVVTQEPSLTVSPGGTVTLTCAFSTGSVTSGSYPNWFQQKPGQAPRALIYSTNNKHSWTPARFSGSLLGGKAALTLSGVQPEDEAEYYCLLYYGGAQLVFGGGTKLTVL | QVQLQESGPGLVKPSQTLSLTCTVSGGSISSGGYYWSWIRQPPGKGLEWIGYIYYTGSTYYNPSLKSRVTISVDTSKNQFSLKLSSVTAADTAVYYCARDDPLYGMDVWGQGTTVTVSS |
| | | | SEQ ID NO: 27676 | SEQ ID NO: 31682 |

FIGURE 50

| | | | | |
|---|---|---|---|---|
| **iPS:437050** | **21-225_197C11** | NA | CAGACTGTGGTGACTCAGGAGCCCTCACTGACTGTGTCCCCAGGAGGGACAGTCACTCTCACCTGTGCTTCCAGCACTGGAGCAGTCACCAGTGCTTACTATCCAAACTGGTTGCAGCAGAAACCTGGACAAGCACCCAGGACACTGATTTATAGTACAAGCAACAAACACTCCTGGACCCCTGCCCGGTTCTCAGGCTCCCTCCTTGGGGGCAAAGCTGCCCTGACACTGTCAGATGTGCAGCCTGAGGACGAGGCTGAGTATTACTGCCTGATCTTCTATGGTGGTGTACATGTGATATTTGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGTAAGGTTTCTGGATATACCTTCACCGGCTACAATATGCACTGGGTGCGACAGGCCCCTGGACAAGGGCTTGAGTGGATGGGATGGATCAACCCTAATAGTGGTGGCACAAACTATGCACACAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGAGATTACTATGATAGTAGTGGAGAAGGGTGGTTCGACCCCTGGGGCCAGGGAACCCTGGTCACCGTCTCCTCA |
| | | | SEQ ID NO: 27677 | SEQ ID NO: 31683 |
| | | AA | QTVVTQEPSLTVSPGGTVTLTCASSTGAVTSAYYPNWLQQKPGQAPRTLIYSTSNKHSWTPARFSGSLLGGKAALTLSDVQPEDEAEYYCLIFYGGVHVIFGGGTKLTVL | QVQLVQSGAEVKKPGASVKVSCKVSGYTFTGYNMHWVRQAPGQGLEWMGWINPNSGGTNYAHKFQGRVTMTRDTSISTAYMELSRLRSDDTAVYYCARDYYDSSGEGWFDPWGQGTLVTVSS |
| | | | SEQ ID NO: 27678 | SEQ ID NO: 31684 |
| **iPS:437054** | **21-225_194G3** | NA | CAGTCTGTGTTGACGCAGCCGCCCTCAGTGTCTGCGGCCCCAGGACAGAAGGTCACCATCTCCTGCTCTGGAAGCAGCTCCAACATCGGGAATAATTATATATCCTGGTACCAGCAGCTCCCAGGAACAGCCCCCAAACTCCTCATTTATGACAATAAAAAGCGACCCTCAGGGATTCCTGACCGATTCTCTGGCTCCAAGTCTGGCACGTCAGCCACCCTGGGCATCACCGGACTCCAGACTGGGGACGAGGCCGATTATTACTGCGGAACATGGGATAGCAGCCTGAGTGTTTGTGTCTTCGGAACTGGGACCAAGGTCACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTTAGTTTCCATGGCATGCACTGGGTCCGCCAGGCTCCAGGCAAGGGGCTGGAGTGGGTGGCAGTTATATGGTATGATGGAAGTAAAAAATACTATGCAGACTCCGTGAAGGGCCGATTCACCATCTCCAGAGACAATTCCAAGAACACGCTGCATCTGCAAATGAACAGTCTGAGAGCCGAGGACACGGCTGTGTATTACTGTGCGAAAGAAGGGTTTAGCAGTGGCTTTTACAGGGGGTTTGACAACTGGGGCCAGGGAACCCTGGTCACCGTCTCCTCA |
| | | | SEQ ID NO: 27679 | SEQ ID NO: 31685 |

FIGURE 50

| | | | | |
|---|---|---|---|---|
| | | AA | QSVLTQPPSVSAAPGQKVTISCSGSSSNIGNNYISWYQQLPGTAPKLLIYDNKKRPSGIPDRFSGSKSGTSATLGITGLQTGDEADYYCGTWDSSLSVCVFGTGTKVTVL | QVQLVESGGGVVQPGRSLRLSCAASGFTFSFHGMHWVRQAPGKGLEWVAVIWYDGSKKYYADSVKGRFTISRDNSKNTLHLQMNSLRAEDTAVYYCAKEGFSSGFYRGFDNWGQGTLVTVSS |
| | | | SEQ ID NO: 27680 | SEQ ID NO: 31686 |
| iPS:437056 | 21-225_198B8 | NA | CAGACCGTGGTGACTCAGGAGTCCTCACTGACTGTGTCCCCAGGAGGGACAGTCACTCTCACTTGTGTTCTTAGCACTGGAGCAGTCACCAGTGGTTCCTTTCCAAACTGGTTCCAGCAGAAACCTGGACAAGCACCCAGGGCTCTGATTTATAGTACAAGCAACAAACATTCCTGGACCCCTGCCCGGTTTTCAGGCTCCCTCCTTGGGGGCAAAGCTGCCCTGACATTGTCAGGTGTGCAGCCTGAGGACGAGGCTGATTATTACTTCATGCTTTACAGTGGTGGAGCTCAGATGGTGTTCGGCGGAGGGACCAAGCTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGAAGTGGTGGTGACTACTGGAGCTGGATCCGCCAGCACCCAGGGGAGGGCCTGGAGTGGATTGGGTACATCTATTACAGTGGGATCACCTACTACAATCCGTCCCTCAAGAGTCGAGTTACCATATCAGTAGACACGTCTAAGAACCAGTTCTCCCTGAAGCTGAGCTCTGTGACTGCCGCGGACACGGCCGTGTATTACTGTGCGAGAGATAGCAGCTCGCGGGGTATGGACGTCTGGGGCCAAGGGACCACGGTCACCGTCTCCTCA |
| | | | SEQ ID NO: 27681 | SEQ ID NO: 31687 |
| | | AA | QTVVTQESSLTVSPGGTVTLTCVLSTGAVTSGSFPNWFQQKPGQAPRALIYSTSNKHSWTPARFSGSLLGGKAALTLSGVQPEDEADYYFMLYSGGAQMVFGGGTKLTVL | QVQLQESGPGLVKPSQTLSLTCTVSGGSIRSGGDYWSWIRQHPGEGLEWIGYIYYSGITYYNPSLKSRVTISVDTSKNQFSLKLSSVTAADTAVYYCARDSSSRGMDVWGQGTTVTVSS |
| | | | SEQ ID NO: 27682 | SEQ ID NO: 31688 |

FIGURE 50

| | | | | |
|---|---|---|---|---|
| **iPS:437058** | **21-225_199F3** | NA | CAGTCTGTGTTGACGCAGCCGCCCTCAGTGTCTGCGGCCCCAGGACAGAAGGTCACCATCTCCTGCTCTGGAAGCAGCTCCAACATTGGGAATAATTATGTATCCTGGTACCAGCAACTCCCAGGAACAGCCCCCAAACTCCTCATTTATGACAATAATAAGCGCCCCTCAGGGATTCCTGACCGATTCTCTGGCTCCAAGTCTGGCACGTCAGCCACCCTGGGCATCACCGGCCTCCAGACTGGGGACGAGGCCGATTATTACTGCGGAACATGGGATAGCAGCCTGAGTGCTTGTGTCTTCGGAACTGGGACCAAGGTCACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTTTCTATGGCATGCACTGGGTCCGCCAGGCTCCAGGCAAGGGGCTGGAGTGGGTGGCAGTTATTTGGTATGATGGAAGTAGTAAATACTATGCAGACTCCGTGAAGGGCCGATTCACCGTCTCCAGAGACAATTCCAAGAACACGCTGTATCTGCAAATGAACAGCCTGAGAGCCGAGGACACGGCTGTGTATTATTGTGCGAAAGAAGGGTATAGCAGTGGCTTTTACAGGGGATTTGCCAACTGGGGCCAGGGAACCCTGGTCACCGTCTCCTCA |
| | | | SEQ ID NO: 27683 | SEQ ID NO: 31689 |
| | | AA | QSVLTQPPSVSAAPGQKVTISCSGSSSNIGNNYVSWYQQLPGTAPKLLIYDNNKRPSGIPDRFSGSKSGTSATLGITGLQTGDEADYYCGTWDSSLSACVFGTGTKVTVL | QVQLVESGGGVVQPGRSLRLSCAASGFTFSFYGMHWVRQAPGKGLEWVAVIWYDGSSKYYADSVKGRFTVSRDNSKNTLYLQMNSLRAEDTAVYYCAKEGYSSGFYRGFANWGQGTLVTVSS |
| | | | SEQ ID NO: 27684 | SEQ ID NO: 31690 |
| **iPS:437060** | **21-225_199C3** | NA | CAGTCTGTGTTGACTCAGCCACCCTCAGCGTCTGGGACCCCCGGGCAGAGGGTCACCATCTCTTGTTCTGGAAGCAGCTCCAACATCGGAAGTAATACTGTAAACTGGTACCAGCAGCTCCCAGGAACGGCCCCCAAACTCCTCATTTATAGTAATAATCAGCGGCCCTCAGGGGTCCCTGACCGATTCTCTGGCTCCAAGTCTGGCACCTCAGCCTCCCTGGCCATCAGTGGGCTCCAGTCTGAGGATGAGGCTGATTATTACTGTGCAGCATGGGATGACAGCCTGAATGGTCCGGTGTTCGGCGGAGGGACCAAGCTGACCGTCCTA | CAGGTGCAGCTGCAGGAGTCGGGCCCAGGACTGGTGAAGCCTTCGGAGACCCTGTCCCTCACCTGCACTGTCTCTGGTGGCTCCATCAGAATTTACTACTGGAGCTGGATCCGGCAGACCCCAGGGAAGGGACTGGAGTGGATTGGATATATCTATTACAGTGGGAGCACCACCTACAACCCCTCCCTCAAGAGTCGAGTCACCATATCAGTAGACACGTCCAAGAACCAGTTCTCCCTGAAGCTGAACTCTGTGACCGCTGCGGACACGGCCGTGTATTACTGTGTGAGAGAAAGGGGGAGTAGCCACAGATGGGGGGACTACTACGGAATGGACGTCTGGGGCCGAGGGACCACGGTCACCGTCTCCTCA |
| | | | SEQ ID NO: 27685 | SEQ ID NO: 31691 |

FIGURE 50

| | | | | |
|---|---|---|---|---|
| | | AA | QSVLTQPPSASGTPGQRVTISCSGSSSNIGSNTVNWYQQLPGTAPKLLIYSNNQRPSGVPDRFSGSKSGTSASLAISGLQSEDEADYYCAAWDDSLNGPVFGGGTKLTVL | QVQLQESGPGLVKPSETLSLTCTVSGGSIRIYYWSWIRQTPGKGLEWIGYIYYSGSTTYNPSLKSRVTISVDTSKNQFSLKLNSVTAADTAVYYCVRERGSSHRWGDYYGMDVWGRGTTVTVSS |
| | | | SEQ ID NO: 27686 | SEQ ID NO: 31692 |
| **iPS:437062** | **21-225_200H1** | NA | CAGACTGTGGTGACTCAGGAGCCCTCACTGACTGTGTCCCCAGGAGGGACAGTCACTCTCACCTGTGCTTCCAACACTGGAGCAGTCACCAGTGGTTCCTATCCAAACTGGTTCCAGCAGAAACCTGGACAGGCACCCAGGGCACTGATTTATCATACAAACAACAAACACTCCTGGACCCCTGCCCGGTTCTCAGGCTCCCTCCTTGGGGGCAAAGCTGCCCTGACACTGTCAGGTGTGCAGCCTGAGGACGAGGCTGAATATTACTGTCTGATCTACTATGGTGGTGCTCAGCTGGTGTTCGGCGGAGGGACCAAGCTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGGTGACTATTGGAGCTGGATCCGCCAGCACCCAGGGAAGGGCCTGGAGTGGATTGGGTACATCTATTATAGTGGGAGCACCTACTACAACCCGTCCCTCAAGAGTCGAGTTACCATATCAGTAGACACGTCTAAGAACCAGTTCTCCCTGAAGCTGAACTCTGTGACTGCCGCGGACACGGCCGTGTATTACTGTGCGAGAGATGGAGCAGCTCTGGGTATGGACGTCTGGGGCCAAGGGACCACGGTCACCGTCTCCTCA |
| | | | SEQ ID NO: 27687 | SEQ ID NO: 31693 |
| | | AA | QTVVTQEPSLTVSPGGTVTLTCASNTGAVTSGSYPNWFQQKPGQAPRALIYHTNNKHSWTPARFSGSLLGGKAALTLSGVQPEDEAEYYCLIYYGGAQLVFGGGTKLTVL | QVQLQESGPGLVKPSQTLSLTCTVSGGSISSGGDYWSWIRQHPGKGLEWIGYIYYSGSTYYNPSLKSRVTISVDTSKNQFSLKLNSVTAADTAVYYCARDGAALGMDVWGQGTTVTVSS |
| | | | SEQ ID NO: 27688 | SEQ ID NO: 31694 |

FIGURE 50

| | | | | |
|---|---|---|---|---|
| **iPS:437064** | **21-225_200G8** | NA | CAGTCTGTGTTGACGCAGCCGCCCTCAGTGTCTGCGGCCCCAGGACAGAGGGTCACCATCTCCTGCTCTGGAAGCAGCTCCAACCTTGGAAATAATTTTGTATCCTGGTACCAGCAGTTCCCAGGAACAGCCCCCAAACTCCTCATTTATGACAATTATAAGCGACCCTCAGGGATTCCTGACCGATTCTCTGTCTCCAAGTCTGGCACGTCAGCCACCCTGGGCATCACCGGACTCCAGACTGGGGACGAGGCCGATTATTACTGCGGAACTTGGGATAGCAGCCTGAATACTGGGGTATTCGGCGGAGGGACCAAGCTGACCGTCCTA | CAGGTGCAGCTGCAGGAGTCGGGCCCAGGACTGGTGAAGCCTTCGGAGACCCTGTCCCTCACCTGCACTGTCTCTGGTGGCTCCATCAGAAGTTACTACTGGAGCTGGATCCGGCAGCCCCCAGGGAAGGGACTGGAGTGGATTGGGTATATCTATTACAGTGGGAGTACTAAGTACAACCCCTCCCTCAAGAGTCGAGTCACCATATCAGTAGACACGTCCAAGAACCAGTTCTCCCTGAAACTGAGCTCTGTGACCGCTGCGGACACGGCCGTGTACTACTGTGCGAGAAAGGGAGTGGGAACCATCCACTTTGACTACTGGGGCCAGGGAACCCTGGTCACCGTCTCCTCA |
| | | | SEQ ID NO: 27689 | SEQ ID NO: 31695 |
| | | AA | QSVLTQPPSVSAAPGQRVTISCSGSSSNLGNNFVSWYQQFPGTAPKLLIYDNYKRPSGIPDRFSVSKSGTSATLGITGLQTGDEADYYCGTWDSSLNTGVFGGGTKLTVL | QVQLQESGPGLVKPSETLSLTCTVSGGSIRSYYWSWIRQPPGKGLEWIGYIYYSGSTKYNPSLKSRVTISVDTSKNQFSLKLSSVTAADTAVYYCARKGVGTIHFDYWGQGTLVTVSS |
| | | | SEQ ID NO: 27690 | SEQ ID NO: 31696 |
| **iPS:437066** | **21-225_200G9** | NA | CAGACTGTGGTGACTCAGGAGCCCTCACTGACTGTGTCCCCAGGAGGGACAGTCACTCTCACCTGTGCTTCCAACACTGGAGCAGTCACCAGTGGTTCCTATCCAAATTGGTTACAGCAGAAACCTGGACAAGCACCCAGGGCACTGATTTATCATACAGACAACAAACACTCCTGGACCCCTGCCCGGTTCTCAGGCTCCCTCCTTGGGGGCAAAGCTGCCCTGACACTGTCAGGTGCGCAGCCTGAGGACGAGGCTGAATATTACTGTCTGATCTACTATGGTGGTGCTCAGCTGGTGTTCGGCGGAGGGACCAAGCTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGGTGACTACTGGAGCTGGATCCGCCAGCACCCAGGGAAGGGCCTGGAGTGGATTGGGTACATCTATTACAGTGGGAGCACCTACTACAACCCGTCCCTCAAGAGTCGAGTTACCATATCAGTAGACACGTCTAAGAACCAGTTCTCCCTGAAGCTGAACTCTGTGACTGCCGCGGACACGGCCGTGTATTACTGTGCGAGAGATGGAGCAGCTCTGGGTATGGACGTCTGGGGCCAAGGGACCACGGTCACCGTCTCCTCA |
| | | | SEQ ID NO: 27691 | SEQ ID NO: 31697 |

FIGURE 50

| | | | | |
|---|---|---|---|---|
| | | AA | QTVVTQEPSLTVSPGGTVTLTCASNTGAVTSGSYPNWLQQKPGQAPRALIYHTDNKHSWTPARFSGSLLGGKAALTLSGAQPEDEAEYYCLIYYGGAQLVFGGGTKLTVL | QVQLQESGPGLVKPSQTLSLTCTVSGGSISSGGDYWSWIRQHPGKGLEWIGYIYYSGSTYYNPSLKSRVTISVDTSKNQFSLKLNSVTAADTAVYYCARDGAALGMDVWGQGTTVTVSS |
| | | | SEQ ID NO: 27692 | SEQ ID NO: 31698 |
| iPS:437068 | 21-225_200A11 | NA | CAGACTGTGGTGACTCAGGAGCCCTCACTGACTGTGTCCCCAGGAGGGACAGTCACTCTCACCTGTGCTTCCAGCACTGGAGCAGTCACCAGTGGTTACTATCCAAACTGGTTCCAGCAGAAACCTGGACAAGTACCCAGGGCACTGATTTATAGTACAAACAACAAACACTCCTGGACCCCTGCCCGGTTCTCAGGCTCCCTCCTTGGGGGCAAAGCTGCCCTGACACTGTCAGGTGTGCAGCCTGAGGACGAGGCTGACTATTACTGCCTGCTCTATTATGGTGGTGCTCACCTGGCATTCGGCGGAGGGACCAAGCTGACCGTCCTG | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGGTGACTACTGGAGCTGGATCCGCCAGCACCCAGGGAAGGGCCTGGAGTGGATTGGGTACATCTATTACAGAGGGAGCACCTACTACAACCCGTCCCTCAAGAGTCGAGTTACCATATCAGTAGACACGTCTAAGAACCAGTTCTCCCTGAAGCTGAGCTCTGTGACTGCCGCGGACACGGCCGTGTATTACTGTGCGAGAGATGCAGCAGCCCACGGCATGGACGTCTGGGGCCAAGGGACCACGGTCACCGTCTCCTCA |
| | | | SEQ ID NO: 27693 | SEQ ID NO: 31699 |
| | | AA | QTVVTQEPSLTVSPGGTVTLTCASSTGAVTSGYYPNWFQQKPGQVPRALIYSTNNKHSWTPARFSGSLLGGKAALTLSGVQPEDEADYYCLLYYGGAHLAFGGGTKLTVL | QVQLQESGPGLVKPSQTLSLTCTVSGGSISSGGDYWSWIRQHPGKGLEWIGYIYYRGSTYYNPSLKSRVTISVDTSKNQFSLKLSSVTAADTAVYYCARDAAAHGMDVWGQGTTVTVSS |
| | | | SEQ ID NO: 27694 | SEQ ID NO: 31700 |

FIGURE 50

| | | | | |
|---|---|---|---|---|
| **iPS:437070** | **21-225_201G11** | NA | TCCTATGAGCTGACTCAGCCACCCTCAGTGTC<br>CGTGTCCCCAGGACAGACAGCCAGCATCACCT<br>GTTCTGGAGATAAATTGGGGGATAGATTTGCT<br>TGCTGGTATCAACAGAAGCCAGGCCAGTCCCC<br>TGTGCTGGTCATCTATCAAGATAGCAAGCGGC<br>CCTCAGGGATCCCTGAGCGATTCTCTGGCTCC<br>AACTCTGGGAACACAGCCACTCTGACCATCAG<br>CGGGACCCAGGCTATGGATGAGGCTGACTATT<br>ACTGTCAGGCGTGGGACAGCAGCACTGTGGTA<br>TTCGGCGGAGGGACCAAGCTGACCGTCCTA | CAGGTACAGCTGCAGCAGTCAGGTCCAGGACTG<br>GTGAAGCCCTCGCAGACCCTCTCACTCACCTGTG<br>CCATCTCCGGGGACAGTGTCTCTCGCATCAATCC<br>TACTTGGAACTGGATCAGGCAGTCCCCATCGAGA<br>GGCCTTGAGTGGCTGGGAAGGACATACTACAGG<br>TCCAAGTGGTATCATGTTTATGCAGTATCTGTGA<br>AAAGTCGAATAACCATCAACCCAGACACATCCA<br>AGAACCAGTTCTCCCTGCAGCTGAATTCTGTGAC<br>TCCCGAGGACACGGCAGTGTATTACTGTGCAAGA<br>GATCCTGGGGGGCTCTTTGACTACTGGGGCCAGG<br>GAACCCTGGTCACCGTCTCCTCA |
| | | | SEQ ID NO: 27695 | SEQ ID NO: 31701 |
| | | AA | SYELTQPPSVSVSPGQTASITCSGDKLGDRFACW<br>YQQKPGQSPVLVIYQDSKRPSGIPERFSGSNSGN<br>TATLTISGTQAMDEADYYCQAWDSSTVVFGGG<br>TKLTVL | QVQLQQSGPGLVKPSQTLSLTCAISGDSVSRINPTW<br>NWIRQSPSRGLEWLGRTYYRSKWYHVYAVSVKSR<br>ITINPDTSKNQFSLQLNSVTPEDTAVYYCARDPGGL<br>FDYWGQGTLVTVSS |
| | | | SEQ ID NO: 27696 | SEQ ID NO: 31702 |
| **iPS:437074** | **21-225_203B2** | NA | TCCTATGAGCTGACTCAGCCACTCTCAGTGTC<br>AGTGGCCCTGGGACAGACGGCCAGGATTACCT<br>GTGGGGGAAACAACATTGGAAGAAAAAATGT<br>GCACTGGTACCAGCAGAAGCCAGGCCAGTCCC<br>CTGTGTTGATCATCCATAGGGATAGCGACCGG<br>CCCTCTGGGATCCCTGAGCGATTCTCTGGCTCC<br>AACTCGGGGAACACGGCCACCCTGACCATCAG<br>CAGAGCCCAAGCCGGGGATGAGGCTGACTATT<br>ACTGTCAGGTGTGGGACAGCGGCACTGCGGTA<br>TTCGGCGGAGGGACCAAGCTGCCCGTCCTA | CAGGTGCAGCTGGTGGAGTCTGGGGGAGGCGTG<br>GTCCAGCCGGGGAGGTCCCTGAGACTCTCCTGTG<br>CAGCGTCTGGATTCACCTTCAGTAACTATGGCAT<br>GCACTGGGTCCGCCAGGCTCCAGGCAAGGGGCT<br>GGAGTGGGTGGCAATTATATGGTTTGATGGAAGT<br>AATGAATACTATGCAGACTCCGTGAAGGGCCGA<br>TTCACCATCTCCAGAGACAATTCCATGAGCACGC<br>TGTATCTGCAAATGAACAGCCTGAGAGCCGAGG<br>ACACGGCTGTGTATTACTGTGCGAGAGAAAGTGG<br>GAGCTATGCTCTTTATATCTGGGGCCAAGGGACA<br>ATGGTCACCGTCTCTTCA |
| | | | SEQ ID NO: 27697 | SEQ ID NO: 31703 |

FIGURE 50

| | | | | |
|---|---|---|---|---|
| | | AA | SYELTQPLSVSVALGQTARITCGGNNIGRKNVHWYQQKPGQSPVLIIHRDSDRPSGIPERFSGSNSGNTATLTISRAQAGDEADYYCQVWDSGTAVFGGGTKLPVL | QVQLVESGGGVVQPGRSLRLSCAASGFTFSNYGMHWVRQAPGKGLEWVAIIWFDGSNEYYADSVKGRFTISRDNSMSTLYLQMNSLRAEDTAVYYCARESGSYALYIWGQGTMVTVSS |
| | | | SEQ ID NO: 27698 | SEQ ID NO: 31704 |
| iPS:437076 | 21-225_203G6 | NA | TCCTATGAACTGACTCAGCCACCCTCAGTGTCCGTGTCCCCAGGACAGACAGCCAGCATCACCTGTTCTGGAGATAAATTGGGGGATAGATTTGCTTGCTGGTATCAACAGAAGCCAGGCCAGTCCCCTGTACTGGTCATCTATCAAGATAACAAGCGGCCCTCAGGGATCCCTGAGCGATTCTCTGGCTCCAACTCTGGGAACACAGCCACTCTGACCATCAGCGGGACCCAGTCTATGGATGAGGCTGACTATTACTGTCAGGCGTGGGACAGCAGCACTGTGGTATTCGGCGGAGGGACCAAGCTGACCGTCCTG | CAGGTTCAGCTGCAGCAGTCAGGTCCAGGACTGGTGAAGCCCTCGCAGACCCTCTCACTCACCTGTGCCATCTCCGGGGACAGTGTCTCTCGCACCAATCCTACTTGGAACTGGATCAGGCAGTCCCCATCGAGAGGCCTTGAGTGGCTGGGAAGGACATACTACAGGTCCAAGTGGTATCATGTTTATGCACTATCTGTGAAAAGTCGAATAACCATCACCCCAGACACATCCAAGAACCAGTTCTCCCTGCAGCTGAACTCTGTGACTCCCGAGGACACGGCTGTGTATTACTGTGCAAGAGATCCTGGGGGCCTCTTTGACTACTGGGGCCAGGGAACCCTGGTCACCGTCTCCTCA |
| | | | SEQ ID NO: 27699 | SEQ ID NO: 31705 |
| | | AA | SYELTQPPSVSVSPGQTASITCSGDKLGDRFACWYQQKPGQSPVLVIYQDNKRPSGIPERFSGSNSGNTATLTISGTQSMDEADYYCQAWDSSTVVFGGGTKLTVL | QVQLQQSGPGLVKPSQTLSLTCAISGDSVSRTNPTWNWIRQSPSRGLEWLGRTYYRSKWYHVYALSVKSRITITPDTSKNQFSLQLNSVTPEDTAVYYCARDPGGLFDYWGQGTLVTVSS |
| | | | SEQ ID NO: 27700 | SEQ ID NO: 31706 |

FIGURE 50

| | | | | |
|---|---|---|---|---|
| iPS:437082 | 21-225_205E12 | NA | TCCTATGAGCTGACTCAGCCACTCTCAGTGTCAGCGGCCCTGGGACAGACGGCCAGGATTACCTGTGGGGGAAACAACATTGGAAGAAAAAATGTGCACTGGTACCAGCAGAAGCCAGGCCAGTCCCCTGTGTTGATCATCCATAGGGATAGCGACCGGCCCTCTGGGATCCCTGAGCGATTCTCTGGCTCCAACTCGGGGAACACGGCCACCCTGACCATCAGCAGAGCCCAAGCCGGGGATGAGGCTGACTATTACTGTCAGGTGTGGGACAGCGGCACTGCGGTATTCGGCGGAGGGACCAAGCTGCCCGTCCTA<br>SEQ ID NO: 27701 | CAGGTGCAGCTGGTGGAGTCTGGGGGAGGCGTGGTCCAGCCGGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAATTATATGGTTTGATGGAAGTAATGAATACTATGCAGACTCCGTGAAGGGCCGATTCACCATCTCCAGAGACAATTCCATGAGCACGCTGTATCTGCAAATGAACAGCCTGAGAGCCGAGGACACGGCTGTGTATTACTGTGCGAGAGAAAGTGGGAGCTATGCTCTTTATATCTGGGGCCAAGGGACAATGGTCACCGTCTCTTCA<br>SEQ ID NO: 31707 |
| | | AA | SYELTQPLSVSAALGQTARITCGGNNIGRKNVHWYQQKPGQSPVLIIHRDSDRPSGIPERFSGSNSGNTATLTISRAQAGDEADYYCQVWDSGTAVFGGGTKLPVL<br>SEQ ID NO: 27702 | QVQLVESGGGVVQPGRSLRLSCAASGFTFSNYGMHWVRQAPGKGLEWVAIIWFDGSNEYYADSVKGRFTISRDNSMSTLYLQMNSLRAEDTAVYYCARESGSYALYIWGQGTMVTVSS<br>SEQ ID NO: 31708 |
| iPS:437084 | 21-225_206B5 | NA | TCCTATGAATTGACTCAGCCACTCTCAGTGTCAGTGGCCCTGGGACAGACGGCCAGGATTGCCTGTGGGGGAAACAACATTGGAAGAAAAAATGTGCACTGGTACCAGCAGAAGCCAGGCCTGGCCCCTGTGCCGGTCATCCNTAGGGATAGCTACCGATCTTCTGGGATCCCTGACAGATTCTCTGGCTCCAACTGCGGGAACACGACCACCGTGACCATCAGCAGAGCCCAAGCCGGGGAGGAGGCAGAGTATTATTGTCAGGATTGGGACAGCAGCACTGTGGTGTTCGGCGGAGGGACCAAACTGACCGTCCTA<br>SEQ ID NO: 27703 | CAGGTGCAGCTGGTGGAGTCCGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATAACTGTGCGAGAGAGGGTGGGAGCTACCACCTTGACTACTGGGGCCAGGGAATCCTGGTCACCGTCTCCTCA<br>SEQ ID NO: 31709 |

FIGURE 50

| | | | | |
|---|---|---|---|---|
| | | AA | SYELTQPLSVSVALGQTARIACGGNNIGRKNVHWYQQKPGLAPVPVIXRDSYRSSGIPDRFSGSNCGNTTTVTISRAQAGEEAEYYCQDWDSSTVVFGGGTKLTVL | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYNCAREGGSYHLDYWGQGILVTVSS |
| | | | SEQ ID NO: 27704 | SEQ ID NO: 31710 |
| iPS:437086 | 21-225_209A8 | NA | CAGTCTGTGTTGACGCAGCCGCCCTCAGTGTCTGCGGCCCCAGGACAGAAGGTCACCATCTCCTGCTCTGGAAGCAGCTCCAACATTGGGAGTAATTTTTTATCCTGGTACCAGCAGCTCCCAGGAACAGCCCCCAAACTCCTCATTTATGACAATAATAAGCGACCCTCAGGGATTCCTGACCGATTCTCTGGCTCCAAGTCTGGCACGTCAGCCACCCTGGGCATCACCGGACTCCAGACTGGGGACGAGGCCGATTATTACTGCGGAACATGGGATAGCAGCCTGAGTGCTGGGGTATTCGGCGGAGGGACCAAGCTGACCGTCCTA | GAGGTGCAGCTGGTGGAGTCTGGGGGAGGCTTGGTACAGCCTGGGGGGTCCCTGAGACTCTCCTGTGCAGCCTCTGGATTCACTTTTAGAAGTTATAGCATGAACTGGGTCCGCCAGGCTCCAGGGAAGGGGCTGGAGTGGGTTTTATACATTAGTAGTAGTAGTAGTATCAAAAAGTACGCAGACTCTGTGAAGGGCCGATTCACCATCTCCAGAGACAATGCCAAGAACTCACTGTATCTGCAAATGAACAGCCTGAGAGACGAGGACACGGCTGTGTATTACTGTGTGAGAGATGATGGGAGCTACTACTTTGACTACTGGGGCCAGGGAACCCTGGTCACCGTCTCCTCA |
| | | | SEQ ID NO: 27705 | SEQ ID NO: 31711 |
| | | AA | QSVLTQPPSVSAAPGQKVTISCSGSSSNIGSNFLSWYQQLPGTAPKLLIYDNNKRPSGIPDRFSGSKSGTSATLGITGLQTGDEADYYCGTWDSSLSAGVFGGGTKLTVL | EVQLVESGGGLVQPGGSLRLSCAASGFTFRSYSMNWVRQAPGKGLEWVLYISSSSSIKKYADSVKGRFTISRDNAKNSLYLQMNSLRDEDTAVYYCVRDDGSYYFDYWGQGTLVTVSS |
| | | | SEQ ID NO: 27706 | SEQ ID NO: 31712 |

FIGURE 50

| | | | | |
|---|---|---|---|---|
| **iPS:437088** | **21-225_209H10** | NA | TCCTATGAATTGACTCAGCCACTCTCAGTGTCAGTGGCCCTGGGACAGACGGCCAGGATTGCCTGTGGGGGAAACAACATTGGAAGAAAAAATGTGCACTGGTACCAGCAGAAGCCAGGCCTGGCCCCTGTGCCGGTCATCCTTAGGGATAGCTACCGGTCTTCTGGGATCCCTGACAGATTCTCTGGCTCCAACTGGGGGAACACGGCCACCGTGACCATCAGCAGAGCCCAAGCCGGGGAGGAGGCAGAGTATTATTGTCAGGATTGGGACAGCAGCACTGTGGTGTTCGGCGGAGGGACCAAACTGACCGTCCTA | CAGGTGCAGCTGGTGGAGTCCGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATAACTGTGCGAGAGAGGGTGGGAGCTACCACCTTGACTACTGGGGCCAGGGAATCCTGGTCACCGTCTCCTCA |
| | | | SEQ ID NO: 27707 | SEQ ID NO: 31713 |
| | | AA | SYELTQPLSVSVALGQTARIACGGNNIGRKNVHWYQQKPGLAPVPVILRDSYRSSGIPDRFSGSNWGNTATVTISRAQAGEEAEYYCQDWDSSTVVFGGGTKLTVL | QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYNCAREGGSYHLDYWGQGILVTVSS |
| | | | SEQ ID NO: 27708 | SEQ ID NO: 31714 |
| **iPS:437090** | **21-225_210F11** | NA | CAGACTGTGGTGACTCAGGAGCCCTCACTGACTGTGTCCCCAGGAGGGACAGTCACTCTCACCTGTGCTTTCAGCACTGGAGCAGTCACCAGTGGTAATTATCCAAACTGGTTCCAGCAGAAACCTGGACAAGCACCCAGGGCACTGATTTACAGTACAAGCAACAAACACTCCTGGACCCCTGCCCGGTTCTCAGGCTCCCTCCTTGGGGGCAAAGCTGCCCTGACACTGTCAGGTGTGCAGCCTGAGGACGAGGCTGAATATTACTGCCTGCTCTACTATGGTGGTGCTCAGCTGGTGTTCGGCGGAGGGACCAAGCTGACCGTCCTA | CAGGTGCAGCTGCAGGAGTCGGGCCCAGGACTGGTGAAGCCGTCACAGACCCTGTCCCTCACCTGCACTGTCTCTGGTGGCTCCATCAGCAGTGGTGGTTCCTACTGGAGCTGGATCCGCCAGCACCCAGGGAAGGGCCTGGAGTGGATTGGGTACATCTATTACATTGGGACCACCTACTACAACCCGTCCCTCAAGAGTCGAGTTACCATATCAGTAGACACGTCTACGAACCACTTCTCCCTGAAACTGAGCTCTGTGACTGCCGCGGACACGGCCGTGTATTACTGTGCGAGAGATGAGCCATTGACCGGTATGGACGTCTGGGGCCAAGGGACCACGGTCACCGTCTCCTCA |
| | | | SEQ ID NO: 27709 | SEQ ID NO: 31715 |

FIGURE 50

| | | | | |
|---|---|---|---|---|
| | | AA | QTVVTQEPSLTVSPGGTVTLTCAFSTGAVTSGNYPNWFQQKPGQAPRALIYSTSNKHSWTPARFSGSLLGGKAALTLSGVQPEDEAEYYCLLYYGGAQLVFGGGTKLTVL | QVQLQESGPGLVKPSQTLSLTCTVSGGSISSGGSYWSWIRQHPGKGLEWIGYIYYIGTTYYNPSLKSRVTISVDTSTNHFSLKLSSVTAADTAVYYCARDEPLTGMDVWGQGTTVTVSS |
| | | | SEQ ID NO: 27710 | SEQ ID NO: 31716 |
| iPS:437092 | 21-225_210B12 | NA | CAGTCTGCCCTGACTCAGCCTGCCTCCGTGTCTGGGTCTCCTGGACAGTCGATCACCATCTCCTGCACTGGAACCAGCAGTGACGTTGGTGGTTATAACTATGTCTCCTGGTACCAACAACACCCAGGCAAAGCCCCCAAATTCATGATTTATGAGGTCAGGAATCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCAGCTCATATACCAGCAGCCGCACTCTGGTATTCGGCGGAGGGACCAAGTTGACCGTCCTA | CAGGTGCAGCTGGTGCAGTCTGGGGCTGAGGTGAAGAAGCCTGGGGCCTCAGTGAAGATCTCCTGCAAGGCTTCTGGATTCACCTTCACCGACTACTATATGAACTGGGTGCGACAGGCCCCTGGACAAGGGCTTGAGTGGATGGGATGGATCAACCCTAACAGTGGTGGCACAAACTATGCACAGAAGTTTCAGGGCAGGGTCACCATGACCAGGGACACGTCCATCAGCACAGCCTACATGGAGCTGAGTAGGCTGAGATCTGACGACACGGCCGTGTATTACTGTGCGAGAGGGTATGACTCGTTCGCCCCCTGGGGCCAGGGAACCCTGGTCACCGTCTCCTCA |
| | | | SEQ ID NO: 27711 | SEQ ID NO: 31717 |
| | | AA | QSALTQPASVSGSPGQSITISCTGTSSDVGGYNYVSWYQQHPGKAPKFMIYEVRNRPSGVSNRFSGSKSGNTASLTISGLQAEDEADYYCSSYTSSRTLVFGGGTKLTVL | QVQLVQSGAEVKKPGASVKISCKASGFTFTDYYMNWVRQAPGQGLEWMGWINPNSGGTNYAQKFQGRVTMTRDTSISTAYMELSRLRSDDTAVYYCARGYDSFAPWGQGTLVTVSS |
| | | | SEQ ID NO: 27712 | SEQ ID NO: 31718 |

FIGURE 50

| | | | | |
|---|---|---|---|---|
| iPS:437094 | 21-225_210D12 | NA | CAGTCTGCCCTGACTCAGCCTGCCTCCGTGTCTGGGTCTCCTGGACAGTCGATCACCATCTCCTGCACTGGAACCAGCAGTGACGTTGGTGGTTATAATTATGTCTCCTGGTACCAACAACACCCAGTCAAAGCCCCCAAACTCTTGATTTATGAGGTCAGTAATCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCAACTCATATACAAGCAGCATCACTTGGGTGTTCGGCGGAGGGACCAAGCTGACCGTCCTA | CAGGTGCAGTTGGTGGAGTCTGGGGGAGGCGTGGTCCAGCCTGGGAGGTCCCTGAGACTCTCCTGTGCAGCGTCTGGATTCACCTTCAGTCACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGGGGGACTGGAACCCCGAGGGTTTGGACGTCTGGGGCCAAGGGACCACGGTCACCGTCTCCTCA |
| | | | SEQ ID NO: 27713 | SEQ ID NO: 31719 |
| | | AA | QSALTQPASVSGSPGQSITISCTGTSSDVGGYNYVSWYQQHPVKAPKLLIYEVSNRPSGVSNRFSGSKSGNTASLTISGLQAEDEADYYCNSYTSSITWVFGGGTKLTVL | QVQLVESGGGVVQPGRSLRLSCAASGFTFSHYGMHWVRQAPGKGLEWVAVIWYDGSNKYYADSVKGRFTISRDNSKNTLYLQMNSLRAEDTAVYYCARGDWNPEGLDVWGQGTTVTVSS |
| | | | SEQ ID NO: 27714 | SEQ ID NO: 31720 |
| iPS:437096 | 21-225_210E12 | NA | CAGTCTGCCCTGACTCAGCCTGCCTCCGTGTCTGGGTCTCCTGGACAGTCGATCACCATCTCCTGCACTGGAACCAGCAGTGACGTTGGTGGTTATAACTATGTCTCCTGGTACCAACAACACCCAGGCAAAGCCCCCAAACTCATGATTTATGAGGTCAGTAATCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCGGCTCATATGTAAAAGGCATCACTTGGGTGTTCGGCGGAGGGACCAGTCTAACCGTCCTC | CAGGTGCACCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGGGGGACTGGAACCCCGAGGGTATGGACGTCTGGGGCCAAGGGACCACGGTCATCGTCTCCTCA |
| | | | SEQ ID NO: 27715 | SEQ ID NO: 31721 |

FIGURE 50

| | | | | |
|---|---|---|---|---|
| | | AA | QSALTQPASVSGSPGQSITISCTGTSSDVGGYNYVSWYQQHPGKAPKLMIYEVSNRPSGVSNRFSGSKSGNTASLTISGLQAEDEADYYCGSYVKGITWVFGGGTSLTVL | QVHLVESGGGVVQPGRSLRLSCAASGFTFSNYGMHWVRQAPGKGLEWVAVIWYDGSNKYYADSVKGRFTISRDNSKNTLYLQMNSLRAEDTAVYYCARGDWNPEGMDVWGQGTTVIVSS |
| | | | SEQ ID NO: 27716 | SEQ ID NO: 31722 |
| **iPS:437098** | **21-225_211C1** | NA | CAGTCTGCCCTGACTCAGCCTGCCTCCGTGTCTGGGTCTCCTGGACAGTCGATCACCATCTCCTGCACTGGAACCAGTAGTGACGTTGGTAGTTATAACTATGTCTCCTGGTACCAACAGTACCCAGGCAAAGCCCCCAAACTCATGATTTATGAGGTCAGTAATCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCAACTCATATACAAGCAGCATCACTTGGGTGTTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTCACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACACCCTGAGAGCCGAGGACACGGCTATCTATTACTGTGCGAGGGGGGACTGGAACCCCGAGGGTTTGGACGTCTGGGGCCAAGGGACCACGGTCACCGTCTCCTCA |
| | | | SEQ ID NO: 27717 | SEQ ID NO: 31723 |
| | | AA | QSALTQPASVSGSPGQSITISCTGTSSDVGSYNYVSWYQQYPGKAPKLMIYEVSNRPSGVSNRFSGSKSGNTASLTISGLQAEDEADYYCNSYTSSITWVFGGGTKLTVL | QVQLVESGGGVVQPGRSLRLSCAASGFTFSHYGMHWVRQAPGKGLEWVAVIWYDGSNKYYADSVKGRFTISRDNSKNTLYLQMNTLRAEDTAIYYCARGDWNPEGLDVWGQGTTVTVSS |
| | | | SEQ ID NO: 27718 | SEQ ID NO: 31724 |

FIGURE 50

| | | | | |
|---|---|---|---|---|
| **iPS:437100** | **21-225_211H2** | NA | TCCTATGAACTGACTCAGCCACTCTCAGTGTCAGTGGCCCTGGGACAGACGGCCAGGATTACCTGTGGGGAAACAACATTGGACGTAGAAATGTGCACTGGTACCAACAGAAGCCAGGCCAGGCCCCTATACTGGTCATCTATAGAGATCGCGACCGGCCCTCTGGGATCCCTGAGCGATTCTCTGGCTCCAACTCGGGGAACACGGCCACCCTGACCATCAGCAGAGCCCAAGCCGGGGATGAGGCTGACTATTTCTGTCAGGTGTGGGACAGCAGTACTGCGGTGTTCGGCGGAGGGACCAAACTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGCCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCGCCAGAGACAATTCCAAGAACACGCTGTATCTGCAAATGAACAGCCTGAGAGCCGAGGACACGGCTGTGTATTACTGTGCGAGAGATCCTGGGAGCTACGGGTTCGACCCCTGGGGCCAGGGAACCCTGGTCACCGTCTCCTCA |
| | | | SEQ ID NO: 27719 | SEQ ID NO: 31725 |
| | | AA | SYELTQPLSVSVALGQTARITCGGNNIGRRNVHWYQQKPGQAPILVIYRDRDRPSGIPERFSGSNSGNTATLTISRAQAGDEADYFCQVWDSSTAVFGGGTKLTVL | QVQLVESGGGVVQPGRSLRLSCAASGFTFSSYAMHWVRQAPGKGLEWVAVIWYDGSNKYYADSVKGRFTIARDNSKNTLYLQMNSLRAEDTAVYYCARDPGSYGFDPWGQGTLVTVSS |
| | | | SEQ ID NO: 27720 | SEQ ID NO: 31726 |
| **iPS:437102** | **21-225_211E5** | NA | TCCTATGAGCTGACACAGCCACCCTCGGTGTCAGTGTCCCCAGGACAGACGGCCAGGATCACCTGTTCTGGAGATGCATTGCCAAAGCAATATGCTTATTGGTACCAGCAGAAGCCAGGCCAGGCCCCAGTGCTGGTGATATATAAAGACAGTGCGAGGCCCTCAGGGATCCCTGAGCGATTCTCTGGCTCCCGCTCAGGGACAACAGTCACGTTGACCGTCAGTGGAGTCCAGGCAGAAGACGAGGCTGACTATTACTGTCAATTAGTGTACAGCAGTGATACTTATGTCTTCGGAACTGGGACCATGCTCACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGGAACTATGGCATGCACTGGGTCCGCCAGGCTCCAGGCAAGGGGCTGGAGTGGGTGTCAATTATATGGTTTGATGGAAGTGATCAATACTATGCAGACTCCGTGAAGGGCCGATTCACCATCTCCAGAGACAACTCCAAGAACACGCTGTACCTGCAAATGAACAGCCTGAGAGCCGAGGACACGGCTGTTTATTACTGTGCGAGAGGCCTCTCTGTCTACTACTACGGTATGGGCGTCTGGGGCCAAGGGACCACGGTCACCGTCTCCTCA |
| | | | SEQ ID NO: 27721 | SEQ ID NO: 31727 |

FIGURE 50

| | | | | |
|---|---|---|---|---|
| | | AA | SYELTQPPSVSVSPGQTARITCSGDALPKQYAYWYQQKPGQAPVLVIYKDSARPSGIPERFSGSRSGTTVTLTVSGVQAEDEADYYCQLVYSSDTYVFGTGTMLTVL | QVQLVESGGGVVQPGRSLRLSCAASGFTFRNYGMHWVRQAPGKGLEWVSIIWFDGSDQYYADSVKGRFTISRDNSKNTLYLQMNSLRAEDTAVYYCARGLSVYYYGMGVWGQGTTVTVSS |
| | | | SEQ ID NO: 27722 | SEQ ID NO: 31728 |
| iPS:437104 | 21-225_211G5 | NA | CAGTCTGCCCTGACTCAGCCTGCCTCCGTGTCTGGGTCTCCTGGACAGTCGATAACCATCTCCTGCACTGGAACCAGCAGTGACGTTGGTGGTTATAACTATGTCTCCTGGTACCAACAGCACCCAGGCAAAGCCCCCAAACTCATGATTTATGAGGTCAGTAATCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCAACTCATATACAAGAAGCATCACTTGGGTGTTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTCACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTACAAATGAACAGCCTGAGAGCCGAGGACACGGCTGTGTATTACTGTGCGAGGGGGGACTGGAACCCCGAGGGTTTGGACGTCTGGGGCCAAGGGACCACGGTCACCGTCTCCTCA |
| | | | SEQ ID NO: 27723 | SEQ ID NO: 31729 |
| | | AA | QSALTQPASVSGSPGQSITISCTGTSSDVGGYNYVSWYQQHPGKAPKLMIYEVSNRPSGVSNRFSGSKSGNTASLTISGLQAEDEADYYCNSYTRSITWVFGGGTKLTVL | QVQLVESGGGVVQPGRSLRLSCAASGFTFSHYGMHWVRQAPGKGLEWVAVIWYDGSNKYYADSVKGRFTISRDNSKNTLYLQMNSLRAEDTAVYYCARGDWNPEGLDVWGQGTTVTVSS |
| | | | SEQ ID NO: 27724 | SEQ ID NO: 31730 |

FIGURE 50

| | | | | |
|---|---|---|---|---|
| **iPS:437106** | **21-225_211H7** | NA | CAGACTGTGGTGACTCAGGAGCCCTCACTGACTGTGTCCCCAGGAGGGACAGTCACTCTCACCTGTGCTTTCAGCACTGGAGCAGTCACCAGTGGTAACTATCCAAGTTGGTTCCAGCAGAAACCTGGACAAGTTCCCAGGGCACTGATTTATAGTACAAGCAACAGACACTCCTGGACCCCTGCCCGGTTTTCTGGCTCCCTCCTTGGGGGCAAAGCTGCCCTGACACTGTCAGGTGTGCAGCCTGAGGACGAGGCTGAATATTACTGCCTGCTCTACTATGGTGGTGCTCAGCTGGTGTTCGGCGGAGGGACCAAGCTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGGTTACTACTGGAGCTGGACCCGCCAGCACCCAGGGAAGGGCCTGGAGTGGATTGGGTACATCTATTACGTTGGGAGCACCTACTACAACCCGTCCCTCAAGAGTCGAGTTACCATATCAGTAGACACGTCTAAGAACCAGTTCTCCCTGAAGCTGAGTTCTGTGACTGCCGCGGACACGGCCGTGTATTACTGTGCGAGAGATGGGCCATTGAGCGGTATGGACGTCTGGGGCCAAGGGACCACGGTCACCGTCTCCTCA |
| | | | SEQ ID NO: 27725 | SEQ ID NO: 31731 |
| | | AA | QTVVTQEPSLTVSPGGTVTLTCAFSTGAVTSGNYPSWFQQKPGQVPRALIYSTSNRHSWTPARFSGSLLGGKAALTLSGVQPEDEAEYYCLLYYGGAQLVFGGGTKLTVL | QVQLQESGPGLVKPSQTLSLTCTVSGGSISSGGYYWSWTRQHPGKGLEWIGYIYYVGSTYYNPSLKSRVTISVDTSKNQFSLKLSSVTAADTAVYYCARDGPLSGMDVWGQGTTVTVSS |
| | | | SEQ ID NO: 27726 | SEQ ID NO: 31732 |
| **iPS:437108** | **21-225_211C9** | NA | CAGACTGTGGTGACTCAGGAGCCCTCACTGACTGTGTCCCCAGGAGGGACAGTCACTCTCACCTGTGGTTCCAGCACTGGATCAGTCACCAGTGGTTACTTTCCAAACTGGTTCCAGCAGAAACCTGGACAAGCACCCAGGCCACTGATTTATAGTACAAACAACAAGCACTCCTGGACCCCTGCCCGGTTCTCAGGCTCCCTCCTTGGGGGCAAAGCTGCCCTGACACTGTCAGATGTGCAGCCTGAGGACGAGGCTGACTATTACTGCCTGCTCTACTATGGTGGTGCTCAGCTGGCATTCGGCGGAGGGACCAAACTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGAAGTGGTGGTGACTACTGGAGCTGGATCCGCCAGCACCCAGGGAAGGGCCTGGAGTGGATTGGATACATCTATTATAGTGGGAGCACCTACTACAACCCGTCCCTCAAGAGTCGAGTTACCATATTACTAGACACGTCTAAGAACCAGTTCTCCCTGAAGCTGAGCTCTGTGACTGTCGCGGACACGGCCGTGTATTACTGTGCGAGAGATTCAGCAGTGTACAATATGGACGTCTGGGGCCAAGGGACCACGGTCACCGTCTCCTCA |
| | | | SEQ ID NO: 27727 | SEQ ID NO: 31733 |

FIGURE 50

| | | | | |
|---|---|---|---|---|
| | | AA | QTVVTQEPSLTVSPGGTVTLTCGSSTGSVTSGYFPNWFQQKPGQAPRPLIYSTNNKHSWTPARFSGSLLGGKAALTLSDVQPEDEADYYCLLYYGGAQLAFGGGTKLTVL | QVQLQESGPGLVKPSQTLSLTCTVSGGSIRSGGDYWSWIRQHPGKGLEWIGYIYYSGSTYYNPSLKSRVTILLDTSKNQFSLKLSSVTVADTAVYYCARDSAVYNMDVWGQGTTVTVSS |
| | | | SEQ ID NO: 27728 | SEQ ID NO: 31734 |
| iPS:437110 | 21-225_211E9 | NA | CAGACTGTGGTGACTCAGGAGCCCTCACTGACTGTGTCCCCAGGAGGGACAGTCACTCTCACCTGTGCTTCCAGCACTGGAGCAGTCACCAGTGGTAACTATCCAAACTGGTTCCAGCAGAAACCTGGACAAGCACCCAGGGCACTGATTTATAGTACAATCAACAAACACTCCGGGACCCCTGCCCGGTTTACAGGCTTCCTCCTTGGGGGCAAAGCTGCCCTGACACTGTCAGGTGTACAGCCTGAGGACGAGGCTGAGTATTACTGCCTGCTCTACTATGGTGGTGCTCAGCTGGCATTCGGCGGAGGGACCAAGCTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGGTGACTACTGGAGCTGGATCCGCCAGCACCCAGGGAAGGGCCTGGAGTGGATTGGGTACATCTATTACACTGGGAGCAACTACTACAACCCGTCCCTCAAGAGTCGAGTTACCATATCAGTAGACACGTCTAAGAATCAGTTCTCCCTGAACCTGATCTCTGTGACTGCCGCGGACACGGCCGTGTATTACTGTGCGAGAGATTCAGCAGTGTACGGTATGGACGTCTGGGGCCAAGGGACCACGGTCACCGTCTCCTCA |
| | | | SEQ ID NO: 27729 | SEQ ID NO: 31735 |
| | | AA | QTVVTQEPSLTVSPGGTVTLTCASSTGAVTSGNYPNWFQQKPGQAPRALIYSTINKHSGTPARFTGFLLGGKAALTLSGVQPEDEAEYYCLLYYGGAQLAFGGGTKLTVL | QVQLQESGPGLVKPSQTLSLTCTVSGGSISSGGDYWSWIRQHPGKGLEWIGYIYYTGSNYYNPSLKSRVTISVDTSKNQFSLNLISVTAADTAVYYCARDSAVYGMDVWGQGTTVTVSS |
| | | | SEQ ID NO: 27730 | SEQ ID NO: 31736 |

FIGURE 50

| | | | | |
|---|---|---|---|---|
| **iPS:437112** | **21-225_212C2** | NA | CAGTCTGCCCTGACTCAGCCTGCCTCCGTGTCTGGGTCTCCTGGACAGTCGATCACCATCTCCTGCACTGGAACCAGCAGTGACGTTGGTGGTTATAACTATGTCTCCTGGTACCAACAACACCCAGGCAAAGCCCCCAAACTCATGATTTATGAGGTCAGAAATCGGCCCTCAGGAGTTTCTAATCGCTTCTCTGGCTCCAAGTCTGGCAACACGGCCTCCCTGACCATCTCTGGGCTCCAGGCTGAGGACGAGGCTGATTATTACTGCAGCTCATATACACGCAGCATCACTTGGGTGTTCGGCGGAGGGACCAAGCTGACCGTCCTA | CAGGTGCAACTGGTGGAGTCTGGGGGAGGCGTGGTCCAGCCTGGGAGGTCCCTGAGACTCTCCTGTGCAGCGTCTGGATTCACCTTCAGTCACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGGGGGACTGGAACCCCGAGGGTATGGACGTCTGGGGCCAAGGGACCTCGGTCACCGTCTCCTCA |
| | | | SEQ ID NO: 27731 | SEQ ID NO: 31737 |
| | | AA | QSALTQPASVSGSPGQSITISCTGTSSDVGGYNYVSWYQQHPGKAPKLMIYEVRNRPSGVSNRFSGSKSGNTASLTISGLQAEDEADYYCSSYTRSITWVFGGGTKLTVL | QVQLVESGGGVVQPGRSLRLSCAASGFTFSHYGMHWVRQAPGKGLEWVAVIWYDGSNKYYADSVKGRFTISRDNSKNTLYLQMNSLRAEDTAVYYCARGDWNPEGMDVWGQGTSVTVSS |
| | | | SEQ ID NO: 27732 | SEQ ID NO: 31738 |
| **iPS:437114** | **21-225_212A4** | NA | CAGTCTGCCCTGACTCAGCCTGCCTCCGTGTCTGGGTCTCCTGGACAGTCGATCACCATCTCCTGCACTGGAACCAGCAGTGACGTTGGTGGTTATAACTATGTCTCCTGGTACCAACAACACCCAGGCAAAGCCCCCAAACTCATGATTTATGAGGTCAGTAATCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCGGCTCATATGTAAAAGGCATCACTTGGGTGTTCGGCGGAGGGACCAGTCTAACCGTCCTC | CAGGTGCACCTGGTGGAGTCTGGGGGAGGCGTGGTCCAGGCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGGGGGACTGGAACCCCGAGGGTATGGACGTCTGGGGCCAAGGGACCACGGTCATCGTCTCCTCA |
| | | | SEQ ID NO: 27733 | SEQ ID NO: 31739 |

FIGURE 50

| | | | | |
|---|---|---|---|---|
| | | AA | QSALTQPASVSGSPGQSITISCTGTSSDVGGYNYVSWYQQHPGKAPKLMIYEVSNRPSGVSNRFSGSKSGNTASLTISGLQAEDEADYYCGSYVKGITWVFGGGTSLTVL | QVHLVESGGGVVQAGRSLRLSCAASGFTFSNYGMHWVRQAPGKGLEWVAVIWYDGSNKYYADSVKGRFTISRDNSKNTLYLQMNSLRAEDTAVYYCARGDWNPEGMDVWGQGTTVIVSS |
| | | | SEQ ID NO: 27734 | SEQ ID NO: 31740 |
| iPS:437116 | 21-225_212F6 | NA | CAGTCTGCCCTGACTCAGCCTGCCTCCGTGTCTGGGTCTCCTGGACAGTCGATCACCATCTCCTGCACTGGAACCAGTAGTGACGTTGGTGGTTATAACTATGTCTCCTGGTACCAACAGTACCCAGGCAAAGCCCCCAAACTCATGATTTATGAGGTCAGTAATCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCAACTCATATACAAGCAGCATCACTTGGGTGTTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTCACTATGGCATGCACTGGGTCCGCCAGGCTCCAGGCAAGGGGCTGGAGTGGGTGGCAGTTATATGGTATGATGGAAGTAATAAATACTATGCGGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGGGGGACTGGAACCCCGAGGGTTTGGACGTCTGGGGCCAAGGGACCACGGTCACCGTCTCCTCA |
| | | | SEQ ID NO: 27735 | SEQ ID NO: 31741 |
| | | AA | QSALTQPASVSGSPGQSITISCTGTSSDVGGYNYVSWYQQYPGKAPKLMIYEVSNRPSGVSNRFSGSKSGNTASLTISGLQAEDEADYYCNSYTSSITWVFGGGTKLTVL | QVQLVESGGGVVQPGRSLRLSCAASGFTFSHYGMHWVRQAPGKGLEWVAVIWYDGSNKYYADSVKGRFTISRDNSKNTLYLQMNSLRAEDTAVYYCARGDWNPEGLDVWGQGTTVTVSS |
| | | | SEQ ID NO: 27736 | SEQ ID NO: 31742 |

FIGURE 50

| | | | | |
|---|---|---|---|---|
| **iPS:437118** | **21-225_212G7** | NA | CAGTCTGCCCTGACTCAGCCTGCCTCCGTGTCTGGGTCTCCTGGACAGTCGATCACCATCTCCTGCACTGGAACCAGCAGTGACGTTGGTGGTTATAATTATGTCTCCTGGTACCAACAGCACCCAGGCAAAACCCCCAAACTCATGATTTATGAGGTCAGTAATCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCAACTCATATACAAGCAGCATCACTTGGGTGTTCGGCGGAGGGACCAAGCTGACCGTCCTA | CAGGTGCAGTTGGTGGAGTCTGGGGGAGGCGTGGTCCAGCCTGGGAGGTCCCTGAGACTCTCCTGTGCAGCGTCTGGATTCACCTTCAGTCACTATGGCATGCACTGGGTCCGCCAGGCTCCAGGCAAGGGGCTGGAGTGGGTGGCAGTTATATGGTATGATGGAAGTAATAAATACTGTGCAGACTCCGTGAAGGGCCGATTCACCATCTCCAGAGACAATTCCACGAACACGCTGTATCTGCAAATGAACAGCCTGAGAGCCGAGGACACGGCTGTGTATTACTGTGCGAGGGGAGACTGGAACCCCGAGGGTTTGGACGTCTGGGGCCAAGGGACCACGGTCACCGTCTCCTCA |
| | | | SEQ ID NO: 27737 | SEQ ID NO: 31743 |
| | | AA | QSALTQPASVSGSPGQSITISCTGTSSDVGGYNYVSWYQQHPGKTPKLMIYEVSNRPSGVSNRFSGSKSGNTASLTISGLQAEDEADYYCNSYTSSITWVFGGGTKLTVL | QVQLVESGGGVVQPGRSLRLSCAASGFTFSHYGMHWVRQAPGKGLEWVAVIWYDGSNKYCADSVKGRFTISRDNSTNTLYLQMNSLRAEDTAVYYCARGDWNPEGLDVWGQGTTVTVSS |
| | | | SEQ ID NO: 27738 | SEQ ID NO: 31744 |
| **iPS:437120** | **21-225_212A9** | NA | CAGACTGTGGTGACTCAGGAGCCCTCACTGACTGTGTCCCCAGGAGGGACAGTCACTCTCACCTGTGCTTCCAGCACTGGAGCAGTCACCAGTGGTTACTATCCAAACTGGTTCCAGCAGAAACCTGGACAAGCACCCAGGGCACTGATTTATAGTACAAACAACAAACACTCCTGGACCCCTGCCCGGTTCTCAGGCTCCCTCCTTGGGGGCAAAGCTGCCCTGACACTGTCAGGTGTACAGCCTGACGACGAGGCTGACTATTACTGCCTGCTCTACTATGGTGGTGCTCAGGTGGGATTCGGCGGAGGGACCAAGCTGACCGTCCTA | CAGGTGCAGCTGCAGGAGTCGGGCCCAGGACTGGTGAAGCCTTCACAGACCCTGTCCCTCACCTGCAGTGTCTCTGGTGGCTCCATCAGAAGTGGTGGTGACTACTGGAGCTGGATCCGCCAGCACCCAGGGAAGGGCCTGGAGTGGATTGGGTATATGTATTACAGTGGGAGCACCTACTACAACCCGTCCCTCAAGAGTCGAGTTACCATATCAGTAGACACGTCTAAGAACCAGTTCTCCCTGAAGCTGAGTTCTGTGACTGTCGCGGACACGGCCGTGTATTACTGTGCGCGAGATTCAGCAGTGTACGGTATGGACGTCTGGGGCCAAGGGACCACGGTCACCGTCTCCTCA |
| | | | SEQ ID NO: 27739 | SEQ ID NO: 31745 |

FIGURE 50

| | | | | |
|---|---|---|---|---|
| | | AA | QTVVTQEPSLTVSPGGTVTLTCASSTGAVTSGYYPNWFQQKPGQAPRALIYSTNNKHSWTPARFSGSLLGGKAALTLSGVQPDDEADYYCLLYYGGAQVGFGGGTKLTVL | QVQLQESGPGLVKPSQTLSLTCSVSGGSIRSGGDYWSWIRQHPGKGLEWIGYMYYSGSTYYNPSLKSRVTISVDTSKNQFSLKLSSVTVADTAVYYCARDSAVYGMDVWGQGTTVTVSS |
| | | | SEQ ID NO: 27740 | SEQ ID NO: 31746 |
| iPS:437124 | 21-225_212H12 | NA | CAGACTGTGGTGACTCAGGAGCCCTCACTGACTGTGTCCCCAGGAGGGACAGTCACTCTCACCTGTGCTTCCAGCACTGGAGCAGTCACCAGTGGTTACTATCCAAACTGGTTCCAGCAGAAACCTGGACAAGCACCCAGGGCACTGATTTATAGTACAAGCAACAAACACTCCTGGACCCCTGCCCGGTTCTCAGGCTCCCTCCTTGGGGGCAAAGCTGCCCTGACACTGTCAGGTGTGCAGCCTGAGGACGAGGCTGAGTATTACTGCCTGCTCTACTATGGTGGTGCTCATGTGGTATTCGGCGGAGGGACCAAGCTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGAAGTGGTGGTGACTACTGGAGTTGGATCCGCCAGCACCCAGGGAAGGGCCTGGAGTGGATTGGGTACATCTATTACAGTGGGAGCACCTACTACAACCCGTCCCTCAAGAGTCGAGTTACCATATCAGGAGACACGTCTAAGAACCAGTTCTCCCTGAAGCTGAGCTCTGTGACTGCCGCGGACACGGCCGTGTATTACTGTGCGAGGGATAGCAGCTCCTACGGTATGGACGTCTGGGGCCAAGGGACCACGGTCACCGTCTCCTCA |
| | | | SEQ ID NO: 27741 | SEQ ID NO: 31747 |
| | | AA | QTVVTQEPSLTVSPGGTVTLTCASSTGAVTSGYYPNWFQQKPGQAPRALIYSTSNKHSWTPARFSGSLLGGKAALTLSGVQPEDEAEYYCLLYYGGAHVVFGGGTKLTVL | QVQLQESGPGLVKPSQTLSLTCTVSGGSIRSGGDYWSWIRQHPGKGLEWIGYIYYSGSTYYNPSLKSRVTISGDTSKNQFSLKLSSVTAADTAVYYCARDSSSYGMDVWGQGTTVTVSS |
| | | | SEQ ID NO: 27742 | SEQ ID NO: 31748 |

FIGURE 50

| | | | | |
|---|---|---|---|---|
| **iPS:437128** | **21-225_213G3** | NA | CTGTCTGCCCTGACTCAGCCTGCCTCCGTGTCTGGGTCTCCTGGACAGTCGATCACCATCTCCTGCACTGGAACCAGCAGTGACGTTGGTGGTTATAACTATGTCTCCTGGTACCAACAACACCCAGGCAAAGCCCCCAAACTCATGATTTCTGAGGTCAGGAATCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCAACTCATATACACGCAGCATCACTTGGGTGTTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTCACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGCATCTGCAAATGAACAGCCTGAGAGCCGAGGACACGGCTGTGTATTACTGTGCGAGGGGGGACTGGAACCCCGAGGGTATGGACGTCTGGGGCCAAGGGACCTCGGTCACCGTCTCCTCA |
| | | | SEQ ID NO: 27743 | SEQ ID NO: 31749 |
| | | AA | LSALTQPASVSGSPGQSITISCTGTSSDVGGYNYVSWYQQHPGKAPKLMISEVRNRPSGVSNRFSGSKSGNTASLTISGLQAEDEADYYCNSYTRSITWVFGGGTKLTVL | QVQLVESGGGVVQPGRSLRLSCAASGFTFSHYGMHWVRQAPGKGLEWVAVIWYDGSNKYYADSVKGRFTISRDNSKNTLHLQMNSLRAEDTAVYYCARGDWNPEGMDVWGQGTSVTVSS |
| | | | SEQ ID NO: 27744 | SEQ ID NO: 31750 |
| **iPS:437130** | **21-225_213D5** | NA | CAGTCTGCCCTGACTCAGCCTGCCTCCGTGTCTGGGTCTCCTGGACAGTCGATCACCATCTCCTGCACTGGAACCAGCAGTGACGTTGGTGGTTATAACTATGTCTCCTGGTACCAACAACACCCAGGCAAAGCCCCCAAACTCGTGATTTATGAGGTCCGTAATCGGCCCTCAGGGGTTTCTACTCGCTTCTCTGGCTCCAAGTCTGGCAACAAGGCCTCCCTGACCATCTCTGGGCTCCAGGCTGAGGACGAGGCTGATTATTACTGCTGCTCATATACAAGAAGAATCACTTGGGTGTTCGGCGGAGGGACCAAGCTGACCGTCCTG | CAGGTGCAGCTGGTGGAATCTGGGGGAGGCGTGGTCCAGCCTGGGAGGTCCCTGAGACTCTCCTGTGCAGCGTCTGGATTCACCTTCAGTCACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTTTCTGCAAATGAACAGCCTGAGAGTCGAGGACACGGCTGTGTATTATTGTGCGAGGGGGGACTGGAACCCCGAGGGTATGGACGTCTGGGGCCAAGGGACCACGGTCACCGTCTCCTCA |
| | | | SEQ ID NO: 27745 | SEQ ID NO: 31751 |

FIGURE 50

| | | | | |
|---|---|---|---|---|
| | | AA | QSALTQPASVSGSPGQSITISCTGTSSDVGGYNYVSWYQQHPGKAPKLVIYEVRNRPSGVSTRFSGSKSGNKASLTISGLQAEDEADYYCCSYTRRITWVFGGGTKLTVL | QVQLVESGGGVVQPGRSLRLSCAASGFTFSHYGMHWVRQAPGKGLEWVAVIWYDGSNKYYADSVKGRFTISRDNSKNTLFLQMNSLRVEDTAVYYCARGDWNPEGMDVWGQGTTVTVSS |
| | | | SEQ ID NO: 27746 | SEQ ID NO: 31752 |
| iPS:437132 | 21-225_213F5 | NA | CAGACTGTGGTGACTCAGGAGCCCTCACTGACTGTGTCCCCAGGAGGGACAGTCACTCTCACCTGTGGTTCCAGCACTGGATCAGTCACCAGTGGTTACTTTCCAAACTGGTTCCAGCAGAAACCTGGACAAACACCCAGGCCACTGATTTATAGTACAAACAACAAGCACTCCTGGACCCCTGCCCGGTTCTCAGGCTCCCTCCTTGGGGGCAAAACTGCCCTGACACTGTCAGATGTGCAGCCTGAGGACGAGGCTGACTATTACTGCCTGCTCTACTTTGGTGGTGCTCAGCTGGCATTCGGCGGAGGGACCAAACTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGAAGTGGTGGTGACTACTGGAGCTGGATCCGCCAGCACCCAGGGAAGGGCCTGGAGTGGATTGGGTACATCTATTATAGTGGGAGCACCTACTACAACCCGTCCCTCAAGAGTCGAGTTACCATATCACTAGACACGTCTAAGAACCAGTTCTCCCTGAAGCTGAGCTCTGTGACTGTCGCGGACACGGCCGTGTATTACTGTGCGAGAGATTCAGCAGTGTACAATATGGACGTCTGGGGCCAAGGGACCACGGTCACCGTCTCCTCA |
| | | | SEQ ID NO: 27747 | SEQ ID NO: 31753 |
| | | AA | QTVVTQEPSLTVSPGGTVTLTCGSSTGSVTSGYFPNWFQQKPGQTPRPLIYSTNNKHSWTPARFSGSLLGGKTALTLSDVQPEDEADYYCLLYFGGAQLAFGGGTKLTVL | QVQLQESGPGLVKPSQTLSLTCTVSGGSIRSGGDYWSWIRQHPGKGLEWIGYIYYSGSTYYNPSLKSRVTISLDTSKNQFSLKLSSVTVADTAVYYCARDSAVYNMDVWGQGTTVTVSS |
| | | | SEQ ID NO: 27748 | SEQ ID NO: 31754 |

FIGURE 50

| | | | | |
|---|---|---|---|---|
| **iPS:437134** | **21-225_213A7** | NA | CAGTCTGCCCTGACTCAGCCTGCCTCCGTGTCTGGGTCTCCTGGACAGTCGATCACCATCTCCTGCACTGGAACCAGCAGTGACGTTGGTGGTTATAACTATGTCTCCTGGTACCAACAGCACCCAGGCAAAGCCCCCAAATTCATGATTTATGAGGTCAGGAATCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCAGCTCATATACCAGCAGCCGCACTCTGGTATTCGGCGGAGGGACCAAGTTGACCGTCCTA | CAGGTGCAGCTGGTGCAGTCTGGGGCTGAGGTGAAGAAGCCTGGGGCCTCCGTGAAGATCTCCTGCAGGGCTTCTGGATTCACCTTCACCGACTACTATATGAACTGGGTGCGACAGGCCCCTGGACAAGGGCTTGAGTGGATGGGATGGATCAACCCTAAGAATGGTGGCACAAACTATGCACAGAAGTTTCAGGGCAGGGTCACCATGACCAGGGACACGTCCCTCAGCAGAGCCTACATGGAGCTGAGTAGGCTGAGATCTGACGACACGGCCGTGTATTACTGTGCGAAAGGGTATGATTCGTTCGCCCCCTGGGGCCAGGGAACCCTGGTCACCGTCTCCTCA |
| | | | SEQ ID NO: 27749 | SEQ ID NO: 31755 |
| | | AA | QSALTQPASVSGSPGQSITISCTGTSSDVGGYNYVSWYQQHPGKAPKFMIYEVRNRPSGVSNRFSGSKSGNTASLTISGLQAEDEADYYCSSYTSSRTLVFGGGTKLTVL | QVQLVQSGAEVKKPGASVKISCRASGFTFTDYYMNWVRQAPGQGLEWMGWINPKNGGTNYAQKFQGRVTMTRDTSLSRAYMELSRLRSDDTAVYYCAKGYDSFAPWGQGTLVTVSS |
| | | | SEQ ID NO: 27750 | SEQ ID NO: 31756 |
| **iPS:437136** | **21-225_214H3** | NA | CAGACTGTGGTGACTCAGGAGCCCTCACTGACTGTGTCCCCAGGAGGGACAGTCACTCTCACCTGTGCTTCCAGCACTGGAGCAGTCACCAGTGGTTACTATCCAAACTGGTTCCAGCAGAAACCTGGACAAGCACCCAGGGCACTGATTTATAGTACAAGCAACAAACACTCCTGTACCCCTGCCCGGTTCTCAGGCTCCCTCCTTGGGGGCAAAGCTGCCCTGACACTGTCAGGTGTGCAGCCTGAGGACGAGGCTGAGTATTACTGCCTGCTCTACTATGGTGGTGCTCATGTGGTATTCGGCGGAGGGACCAAGCTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGAAGTGGTGGTGACTACTGGAGTTGGATCCGCCAGCACCCAGGGAAGGGCCTGGAGTGGATTGGGTACATCTATTACAGTGGGAGCACCTACTACAACCCGTCCCTCAAGAGTCGAGTTACCATATCAGGAGACACGTCTAAGAACCAGTTCTCCCTGAAGCTGAGCTCTGTGACTGCCGCGGACACGGCCGTGTATTACTGTGCGAGGGATAGCAGCTCCTACGGTATGGACGTCTGGGGCCAAGGGACCACGGTCACCGTCTCCTCA |
| | | | SEQ ID NO: 27751 | SEQ ID NO: 31757 |

FIGURE 50

| | | | | |
|---|---|---|---|---|
| | | AA | QTVVTQEPSLTVSPGGTVTLTCASSTGAVTSGYYPNWFQQKPGQAPRALIYSTSNKHSCTPARFSGSLLGGKAALTLSGVQPEDEAEYYCLLYYGGAHVVFGGGTKLTVL | QVQLQESGPGLVKPSQTLSLTCTVSGGSIRSGGDYWSWIRQHPGKGLEWIGYIYYSGSTYYNPSLKSRVTISGDTSKNQFSLKLSSVTAADTAVYYCARDSSSYGMDVWGQGTTVTVSS |
| | | | SEQ ID NO: 27752 | SEQ ID NO: 31758 |
| **iPS:437138** | **21-225_214D8** | NA | CAGTCTGTGTTGACGCAGCCGCCCTCAGTGTCTGCGGCCCCAGGACAGAAGGTCACCATCTCCTGCTCTGGAAGCAGCTCCAACATTGGGAATAATTATGTATCCTGGTACCAGCAGTTCCCAGGAACAGCCCCCAAACTCCTCATTCATGACAATAATAAGCGACCCTCAGGGATTCCTGACCGATTCTCTGGCTCCAAGTCTGGCACGTCAGCCACCCTGGGCATCACCGGACTCCAGACTGGGGACGAGGCCGATTATTACTGCGGAGCATGGGATAGCAGCCTGAGTGCTGTGGTAATCGGCGGAGGGAGCAAGCTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGTACTGCTTTTTACTACTGGAGCTGGATCCGCCAGCACCCAGGGAAGGGCCTGGAGTGGATTGGGTACATCTATTTCAGTGGGAGCACCTACTACAACCCGTCCCTCAAGAGTCGAGTTACCATATCAGTAGACACGTCTAAGAACCAGTTCTCCCTGAACCTGAGCTCTGTGACTGCCGCGGACACGGCCGTGTATTACTGTGCGAGAGCAAGGGGATATCACTACAGTATCTTTGACTACTGGGGCCAGGGAACCCTGGTCACCGTCTCCTCA |
| | | | SEQ ID NO: 27753 | SEQ ID NO: 31759 |
| | | AA | QSVLTQPPSVSAAPGQKVTISCSGSSSNIGNNYVSWYQQFPGTAPKLLIHDNNKRPSGIPDRFSGSKSGTSATLGITGLQTGDEADYYCGAWDSSLSAVVIGGGSKLTVL | QVQLQESGPGLVKPSQTLSLTCTVSGGSISTAFYYWSWIRQHPGKGLEWIGYIYFSGSTYYNPSLKSRVTISVDTSKNQFSLNLSSVTAADTAVYYCARARGYHYSIFDYWGQGTLVTVSS |
| | | | SEQ ID NO: 27754 | SEQ ID NO: 31760 |

FIGURE 50

| | | | | |
|---|---|---|---|---|
| iPS:437140 | 21-225_214E12 | NA | CAGACTGTGGTGACTCAGGAGCCCTCACTGACTGTGTCCCCAGGAGGGACAGTCACTCTCACCTGTGCTTCCAGCACTGGAGCAGTCACCAGTGGCTACTATCCAAACTGGTTCCAACAGAAACCTGGACAAGCACCCAGGGCACTGATTTATAGTACAAGCAATAAACACTCCTGGACCCCTGCCCGGTTCTCAGGCTCCCTCCTTGGGGGCAAAGCTGCCCTGACACTGTCAGATGTGCAGCCTGAGGACGAGGCTGAGTATTACTGCCTGCTCTACTGTGATGGTGCCCAGCTGGTGTTCGGCGGAGGGACCAAACTGACCGTCCTA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGGTGATTACTGGAGCTGGATCCGCCAGCACCCAGGGAAGGGCCTGGAGTGGATTGGGTACATCTATTACAGTGGGCCCACCTACTACAACCCGTCCCTCAAGAGTCGAGTTACCATATCAGTAGACACGTCTAAGAACCAGTTCTCCCTGAAGCTGAGCTCTGTGACTGCCGCGGACACGGCCGTGTATTATTGTGCGAGAGATGGGGCTGCGGAGGGTATGGACGTCTGGGGCCAAGGGACCACGGTCACCGTCTCCTCA |
| | | | SEQ ID NO: 27755 | SEQ ID NO: 31761 |
| | | AA | QTVVTQEPSLTVSPGGTVTLTCASSTGAVTSGYYPNWFQQKPGQAPRALIYSTSNKHSWTPARFSGSLLGGKAALTLSDVQPEDEAEYYCLLYCDGAQLVFGGGTKLTVL | QVQLQESGPGLVKPSQTLSLTCTVSGGSISSGGDYWSWIRQHPGKGLEWIGYIYYSGPTYYNPSLKSRVTISVDTSKNQFSLKLSSVTAADTAVYYCARDGAAEGMDVWGQGTTVTVSS |
| | | | SEQ ID NO: 27756 | SEQ ID NO: 31762 |
| iPS:437142 | 21-225_215A3 | NA | CAGACTGTGGTGACTCAGGAGCCCTCACTGACGGTGTCCCCAGGAGGGACAGTCACTCTCACCTGTGCTTCCAGCACTGAAGCCGTCACCAGTGGTAACTATCCAAGCTGGTTCCAGCAGAAACCTGGACAAGCACCCAGGGCACTGATTTATAGTACAAGCAACAAACACTCCGGTACCCCTGCCCGGTTTACAGGCTCCCTCCTTGGGGGCAAAGCTGCCCTGACACTGTCAGGTGTACAGCCTGAGGACGAGGCTGAGTATTACTGCCTGCTCTACTATGGTGGCGCTCAGCTGGCATTCGGCGGAGGGACCAAGCTGGCCGTCCTA | CAGGTGCAGTTGCAGGAGTCGGGCCCAGGACTGGTGAAGCCTTCACAGACCCTGTCCCTCACCTGCACTGTCTCTGGTGGCTCCATCAGCAGTGGTGGTGACTACTGGAGCTGGATCCGCCAGCACCCAGGGAAGGGCCTGGAGTGGATTGGGTACATCTATTACACTGGGAGCAACTACTACAACCCGTCCCTCAAGAGTCGAGTTACCATATCAGTAGACACGTCTAAGAATCAGTTCTCCCTGAAGGTGATCTCTGTGACTGCCGCGGACACGGCCGTGTATTACTGTGCGAGAGATTCAGCAGTGTACGGTATGGACGTCTGGGGCCAAGGGACCACGGTCACCGTCTCCTCA |
| | | | SEQ ID NO: 27757 | SEQ ID NO: 31763 |

FIGURE 50

| | | | | |
|---|---|---|---|---|
| | | AA | QTVVTQEPSLTVSPGGTVTLTCASSTEAVTSGNYPSWFQQKPGQAPRALIYSTSNKHSGTPARFTGSLLGGKAALTLSGVQPEDEAEYYCLLYYGGAQLAFGGGTKLAVL | QVQLQESGPGLVKPSQTLSLTCTVSGGSISSGGDYWSWIRQHPGKGLEWIGYIYYTGSNYYNPSLKSRVTISVDTSKNQFSLKVISVTAADTAVYYCARDSAVYGMDVWGQGTTVTVSS |
| | | | SEQ ID NO: 27758 | SEQ ID NO: 31764 |
| **iPS:437144** | **21-225_215B3** | NA | TCCTATGAGCTGACTCAGCCACCCTCAGTGTCCGTGTCCCCAGGACAGACAGCCAGCATCACCTGCTCTGGAGATAAATTGGGGGATAAATTTGCTTGCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTGTGGTATTCGGCGGAGGGACCAAGCTGACCGTCCTA | GAGGTGCAGCTGGTGGAGTCTGGGGGAGGCTTGGTAAAGCCTGGGGGGTCCCTTAGACTCTCCTGTGCAGCCTCTGGATTCACTTTCAGTAACGCCTGGATGCACTGGGTCCGCCAGGCTCCAGGGAAGGGGCTGGAGTGGGTTGGCCGTATTAAAAGCAAAACTAATGGTGGGACAACAGACTACGCTGCACCCGTGAAAGGCAGATTCACCATTTCAAGAGATGATTCAAAAAACACGCTGTATCTGCAAATGAACAGCCTGAAAACCGAGGACACAGCCGTGTATTACTGTACCACAGATCCGGGGGGGATCTTTGACTACTGGGGCCAGGGAACCCTGGTCACCGTCTCCTCA |
| | | | SEQ ID NO: 27759 | SEQ ID NO: 31765 |
| | | AA | SYELTQPPSVSVSPGQTASITCSGDKLGDKFACWYQQKPGQSPVLVIYQDSKRPSGIPERFSGSNSGNTATLTISGTQAMDEADYYCQAWDSSTVVFGGGTKLTVL | EVQLVESGGGLVKPGGSLRLSCAASGFTFSNAWMHWVRQAPGKGLEWVGRIKSKTNGGTTDYAAPVKGRFTISRDDSKNTLYLQMNSLKTEDTAVYYCTTDPGGIFDYWGQGTLVTVSS |
| | | | SEQ ID NO: 27760 | SEQ ID NO: 31766 |

FIGURE 50

| | | | | |
|---|---|---|---|---|
| **iPS:437146** | **21-225_215D3** | NA | CAGTCTGCCCTGACTCAGCCTGCCTCCGTGTCTGGGTCTCCTGGACAGTCGATCACCATCTCCTGCACTGGAACCAGCAGTGACATTGGTGGTTATAACTATGTCTCCTGGTACCAACAACACCCAGGCAAAGCCCCCACACTCATGATTTATGAGGTCAGTAATCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCAACTCATATAAAAGGGGCAGCACTTGGGTGTTCGGCGGAGGGACCAAGGTGACCGTCCTA | CAGACGCAGCTGGTGGAGTCTGGGGGAGGCGTGGTCCAGCCTGGGAGGTCCCTGAGACTCTCCTGTGCAGCGTCTGGATTCACCTTCAGTCACTATGGCATGCACTGGGTCCGCCAGGCTCCAGGCAAGGGGCTGGAATGGGTGGCAGTTATATGGTATGATGGAAGTAATGAGTACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGGGGGACTGGAACCCCGAGGGTATGGACGTCTGGGGCCAAGGGACCACGGTCACCGTCTCCTCA |
| | | | SEQ ID NO: 27761 | SEQ ID NO: 31767 |
| | | AA | QSALTQPASVSGSPGQSITISCTGTSSDIGGYNYVSWYQQHPGKAPTLMIYEVSNRPSGVSNRFSGSKSGNTASLTISGLQAEDEADYYCNSYKRGSTWVFGGGTKVTVL | QTQLVESGGGVVQPGRSLRLSCAASGFTFSHYGMHWVRQAPGKGLEWVAVIWYDGSNEYYADSVKGRFTISRDNSKNTLYLQMNSLRAEDTAVYYCARGDWNPEGMDVWGQGTTVTVSS |
| | | | SEQ ID NO: 27762 | SEQ ID NO: 31768 |
| **iPS:437148** | **21-225_215H3** | NA | CAGACTGTGGTGACTCAGGAGCCCTCACTGACTGTGTCCCCAGGAGGGACAGTCACTCTCACCTGTGCTTCCAGCACTGGAGCAGTCACCAGTGGTTACTATCCAAACTGGTTCCAGCAGAAACCTGGACAAGCACCCAGGGCACTGATTTATAGTACAAACAACAAACACTCCTGTGGACCTGCCCGGTTTTCAGGCTCCCTCCTTGGGGGCAAAGCTGCCCTGACACTGTCAGGTGTACAGCCTGACGACGAGGCTGACTATTACTGCCTGCTCTACTATGGTGGTGCTCAGGTGGGATTCGGCGGAGGGACCAAGCTGACCGTCCTA | CAGGTGCAGCTGCAGGAGTCGGGCCCAGGACTGGTGAAGCCTTCACAGACCCTGTCCCTCACCTGCAGTGTCTCTGGTGGCTCCATCAGAAGTGGTGGTGACTACTGGAGCTGGATCCGCCAGCACCCAGGGAAGGGCCTGGAGTGGATTGGGTATATGTATTACAGTGGGAGCACCTACTACAACCCGTCCCTCAAGAGTCGAGTTACCATATCAGTAGACACGTCTAAGAACCAGTTCTCCCTGAAGCTGAGTTCTGTGACTGTCGCGGACACGGCCGTGTATTACTGTGCGAGATTCAGCAGTGTACGGTATGGACGTCTGGGGCCAAGGGACCACGGTCACCGTCTCCTCA |
| | | | SEQ ID NO: 27763 | SEQ ID NO: 31769 |

FIGURE 50

| | | | | |
|---|---|---|---|---|
| | | AA | QTVVTQEPSLTVSPGGTVTLTCASSTGAVTSGYYPNWFQQKPGQAPRALIYSTNNKHSCGPARFSGSLLGGKAALTLSGVQPDDEADYYCLLYYGGAQVGFGGGTKLTVL | QVQLQESGPGLVKPSQTLSLTCSVSGGSIRSGGDYWSWIRQHPGKGLEWIGYMYYSGSTYYNPSLKSRVTISVDTSKNQFSLKLSSVTVADTAVYYCARDSAVYGMDVWGQGTTVTVSS |
| | | | SEQ ID NO: 27764 | SEQ ID NO: 31770 |
| iPS:437150 | 21-225_216A3 | NA | CAGTCTGCCCTGACTCAGCCTGCCTCCGTGTCTGGGTCTCCTGGACAGTCGATCACCATCTCCTGCACTGGAACCAGCAGTGACGTTGGTGGTTATAATTATGTCTCCTGGTACCAACAACACCCAGGCAAAGCCCCCAAACTCATGATTTATGAGGTCAGTAATCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCAACTCATATACAAGCAGCATCACTTGGGTGTTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTCACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGGGGGACTGGAACCCCGAGGGTTTGGACGTCTGGGGCCAAGGGACCACGGTCACCGTCTCCTCA |
| | | | SEQ ID NO: 27765 | SEQ ID NO: 31771 |
| | | AA | QSALTQPASVSGSPGQSITISCTGTSSDVGGYNYVSWYQQHPGKAPKLMIYEVSNRPSGVSNRFSGSKSGNTASLTISGLQAEDEADYYCNSYTSSITWVFGGGTKLTVL | QVQLVESGGGVVQPGRSLRLSCAASGFTFSHYGMHWVRQAPGKGLEWVAVIWYDGSNKYYADSVKGRFTISRDNSKNTLYLQMNSLRAEDTAVYYCARGDWNPEGLDVWGQGTTVTVSS |
| | | | SEQ ID NO: 27766 | SEQ ID NO: 31772 |

FIGURE 50

| | | | | |
|---|---|---|---|---|
| **iPS:437154** | **21-225_216A7** | NA | CAGACTGTGGTGACTCAGGAGCCCTCACTGAC<br>TGTGTCCCCAGGAGGGACAGTCACTCTCACCT<br>GTGCTTCCAGCACTGGAGCAGTCACCAGTGGT<br>TACTATCCAAACTGGTTCCAGCAGAAACCTGG<br>ACAAGCACCCAGGGCACTGATTTATAGTACAA<br>ACAACAAACACTCCTGTACCCCTGCCCGGTTC<br>TCAGGCTCCCTCCTTGGGGGCAAAGCTGCCCT<br>GACACTGTCAGGTGTACAGCCTGACGACGAGG<br>CTGACTATTACTGCCTGCTCTACTATGGTGGTG<br>CTCAGGTGGGATTCGGCGGAGGGACCAAGCT<br>GACCGTCCTA | CAGGTGCAGCTGCAGGAGTCGGGCCCAGGACTG<br>GTGAAGCCTTCACAGACCCTGTCCCTCACCTGCA<br>GTGTCTCTGGTGGCTCCATCAGAAGTGGTGGTGA<br>CTACTGGAGCTGGATCCGCCAGCACCCAGGGAA<br>GGGCCTGGAGTGGATTGGGTATATGTATTACAGT<br>GGGAGCACCTACTACAACCCGTCCCTCAAGAGTC<br>GAGTTACCATATCAGTAGACACGTCTAAGAACCA<br>GTTCTCCCTGAAGCTGAGTTCTGTGACTGTCGCG<br>GACACGGCCGTGTATTACTGTGCGCGAGATTCAG<br>CAGTGTACGGTATGGACGTCTGGGGCCAAGGGA<br>CCACGGTCACCGTCTCCTCA |
| | | | SEQ ID NO: 27767 | SEQ ID NO: 31773 |
| | | AA | QTVVTQEPSLTVSPGGTVTLTCASSTGAVTSGYY<br>PNWFQQKPGQAPRALIYSTNNKHSCTPARFSGSL<br>LGGKAALTLSGVQPDDEADYYCLLYYGGAQVG<br>FGGGTKLTVL | QVQLQESGPGLVKPSQTLSLTCSVSGGSIRSGGDY<br>WSWIRQHPGKGLEWIGYMYYSGSTYYNPSLKSRV<br>TISVDTSKNQFSLKLSSVTVADTAVYYCARDSAVY<br>GMDVWGQGTTVTVSS |
| | | | SEQ ID NO: 27768 | SEQ ID NO: 31774 |
| **iPS:437158** | **21-225_216H11** | NA | CAGACTGTGGTGACTCAGGAGCCCTCACTGAC<br>TGTGTCCCCAGGAGGGACAGTCACTCTCACCT<br>GTGCTTCCAGCACTGGAGCAGTCACCAGTGGC<br>TACTATCCAAACTGGTTCCAACAGAAACCTGG<br>ACAAGCACCCAGGGCACTGATTTATAGTACAA<br>GCAATAAACACTCCTGGACCCCTGCCCGGTTC<br>TCAGGCTCCCTCCTTGGGGGCAAAGCTGCCCT<br>GACACTGTCAGATGTGCAGCCTGAGGACGAG<br>GCTGAGTATTACTGCCTGCTCTACTGTGATGGT<br>GCTCAGCTGGTGTTCGGCGGAGGGACCAAACT<br>GACCGTCCTA | CAGGTGCAGCTGCAGGAGTCGGGCCCAGGACTG<br>GTGAAGCCTTCACAGACCCTGTCCCTCACCTGCA<br>CTGTCTCTGGTGGCTCCATCAGCAGTGGTGGTGA<br>TTACTGGAGCTGGATCCGCCAGCACCCAGGGAA<br>GGGCCTGGAGTGGATTGGGTACATCTATTACAGT<br>GGGCCCACCTACTACAACCCGTCCCTCAAGAGTC<br>GAGTTACCATATCAGTAGACACGTCTAAGAACCA<br>GTTCTCCCTGAAGCTGAGCTCTGTGACTGCCGCG<br>GACACGGCCGTGTATTATTGTGCGAGAGATGGGG<br>CTGCGGAGGGTTTGGACGTCTGGGGCCAAGGGA<br>CCACGGTCACCGTCTCCTCA |
| | | | SEQ ID NO: 27769 | SEQ ID NO: 31775 |

FIGURE 50

| | | | | |
|---|---|---|---|---|
| | | AA | QTVVTQEPSLTVSPGGTVTLTCASSTGAVTSGYYPNWFQQKPGQAPRALIYSTSNKHSWTPARFSGSLLGGKAALTLSDVQPEDEAEYYCLLYCDGAQLVFGGGTKLTVL | QVQLQESGPGLVKPSQTLSLTCTVSGGSISSGGDYWSWIRQHPGKGLEWIGYIYYSGPTYYNPSLKSRVTISVDTSKNQFSLKLSSVTAADTAVYYCARDGAAEGLDVWGQGTTVTVSS |
| | | | SEQ ID NO: 27770 | SEQ ID NO: 31776 |
| **iPS:437160** | **21-225_216B12** | NA | TCCTATGAGCTGACTCAGCCACTCTCAGTGTCAGTGGCCCTGGGACAGACGGCCAGGATTACCTGTGGGGGAGACAACATTAGAAGAAGAAATGTGCACTGGTACCAGCAGAAGCCAGGCCAGGCCCCTGTGCTGGTCATCTATAGGGATAGCAACCGGCCCTCTGGGATCCCTGAGCGATTCTCTGGCTCCAACTCGGGGAACACGGCCACCCTGACCATCAGCAGAGCCCAAGCCGGGGATGAGGCTGACTATTACTGTCAGGTGTGGGACAGCAGCACTGGGGTGTTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGCCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCTGGGACTGGGATACTTCTTTGACTACTGGGGCCAGGGAACCCTAGTCACCGTCTCCTCA |
| | | | SEQ ID NO: 27771 | SEQ ID NO: 31777 |
| | | AA | SYELTQPLSVSVALGQTARITCGGDNIRRRNVHWYQQKPGQAPVLVIYRDSNRPSGIPERFSGSNSGNTATLTISRAQAGDEADYYCQVWDSSTGVFGGGTKLTVL | QVQLVESGGGVVQPGRSLRLSCAASGFTFSSYAMHWVRQAPGKGLEWVAVIWYDGSNKYYADSVKGRFTISRDNSKNTLYLQMNSLRAEDTAVYYCARDLGLGYFFDYWGQGTLVTVSS |
| | | | SEQ ID NO: 27772 | SEQ ID NO: 31778 |

FIGURE 50

| | | | | |
|---|---|---|---|---|
| **iPS:437162** | **21-225_217B2** | NA | CAGTCTGCCCTGACTCAGCCTGCCTCCGTGTCTGGGTCTCCTGGACAGTCGATCACCATCTCCTGCACTGGAACCAGCAGTGACGTTGGTGGTTATAACTATGTCTCCTGGTACCAACAACACCCAGGCAAAGCCCCCAAACTCTTGATTTATGAGGTCAGTAATCGGCCCTCAGGGGTTTATAATCGCTTCTCTGGCTCCAAGTCTGGCAACACGGCCTCCCTGACCATCTCTGGGCTCCAGGCTGAGGACGAGGCTGATTATTACTGCGGCTCATATGTAAAAGGCATCACTTGGGTGTTCGGCGGAGGGACCAGTCTGACCGTCCTC<br>SEQ ID NO: 27773 | CAGGTGCACCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTATTGTGCGAGGGGGGACTGGAACCCCGAGGGTATGGACGTCTGGGGCCAAGGGACCACGGTCATCGTCTCCTCA<br>SEQ ID NO: 31779 |
| | | AA | QSALTQPASVSGSPGQSITISCTGTSSDVGGYNYVSWYQQHPGKAPKLLIYEVSNRPSGVYNRFSGSKSGNTASLTISGLQAEDEADYYCGSYVKGITWVFGGGTSLTVL<br>SEQ ID NO: 27774 | QVHLVESGGGVVQPGRSLRLSCAASGFTFSNYGMHWVRQAPGKGLEWVAVIWYDGSNKYYADSVKGRFTISRDNSKNTLYLQMNSLRAEDTAVYYCARGDWNPEGMDVWGQGTTVIVSS<br>SEQ ID NO: 31780 |
| **iPS:437164** | **21-225_217C6** | NA | TCCTATGAGCTGACACAGCCACCCTCGGTGTCAGTGTCCCCAGGACAGACGGCCAGGATCACCTGCTCTGGAGATGCATTGCCAAAGCAATATGCTTATTGGTACCAGCAGAAGCCAGGCCAGGCCCCAGTGCTGGTGATATATAAAGACAGTGAGAGGCCCTCAGGGATTCCTGAGCGATTCTCTGGCTCCCGCTCAGGGACAACAGTCACGTTGACCATCAGAGGAGTCCAGGCAGAAGACGAGGCTGACTATTACTGTCAATTAATAGTCAGCAGTGATACTTATGTCTTCGGAACTGGGACCAAGGTCACCGTCCTA<br>SEQ ID NO: 27775 | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGATGGCAATTATATGGTTTGATGGAAGTGATGAATACTATGCAGACTCCGTGAAGGGCCGATTCACCATCTCCAGAGACAATTCCAAGAACACGATGTATCTGCAAATGAACAGCCTGAGAGCCGAAGACACGGCTGTGTATTACTGTGCGAGAGGCCTATCTGTCTACTACTACGGTATGGACGTCTGGGGCCAAGGGACCACGGTCACCGTCTCCTCA<br>SEQ ID NO: 31781 |

FIGURE 50

| | | | | |
|---|---|---|---|---|
| | | AA | SYELTQPPSVSVSPGQTARITCSGDALPKQYAYWYQQKPGQAPVLVIYKDSERPSGIPERFSGSRSGTTVTLTIRGVQAEDEADYYCQLIVSSDTYVFGTGTKVTVL | QVQLVESGGGVVQPGRSLRLSCAASGFTFSNYGMHWVRQAPGKGLEWMAIIWFDGSDEYYADSVKGRFTISRDNSKNTMYLQMNSLRAEDTAVYYCARGLSVYYYGMDVWGQGTTVTVSS |
| | | | SEQ ID NO: 27776 | SEQ ID NO: 31782 |
| iPS:437166 | 21-225_217G11 | NA | TCCTATGAGCTGACACAGCCACCCTCGGTGTCAGTGTCCCCAGGACAGACGGCCAGGATCACCTGCTCTGGAGATGCATTGCCAAAACAATATGCTTATTGGTACCAGCAGAAGCCAGGCCAGGCCCCAGTACTGGTGATATATAAAGACAGTGAGAGGCCCTCAGGGATCCCTGAGCGATTCTCTGGCTCCCGCTCAGGGACAACAGTCACGTTGACCGTCAGTGGAGTCCAGGCAGAAGACGAGGCTGACTATTACTGTCAATTAGTGTACAGCAGTGATACTTATGTCTTCGGAACTGGGACCAAGGTCACCGTCCTA | CAGGTGCAGTTGGTGGAGTCTGGGGGAGGCGTGGTCCAGCCTGGGAGGTCCCTGAGACTCTCCTGTGCAGCGTCTGGATTCACCTTCAGGAACTATGGCATGCACTGGGTCCGCCAGGCTCCAGGCAAGGGGCTGGAGTGGGTGTCAATTATATGGTTTGATGGAAGTGATCAGTACTATGCAGACTCCGTGAAGGGCCGATTCACCATCTCCAGAGACAATTCCAAGAACACGCTGTATCTGCAAATGAACAGCCTGAGAGCCGAGGACACGGCTGTATATTACTGTGCGAGAGGCCTCTCTGTCTACTACTACGGTATGGACGTCTGGGGCCAAGGGACCACGGTCACCGTCTCCTCA |
| | | | SEQ ID NO: 27777 | SEQ ID NO: 31783 |
| | | AA | SYELTQPPSVSVSPGQTARITCSGDALPKQYAYWYQQKPGQAPVLVIYKDSERPSGIPERFSGSRSGTTVTLTVSGVQAEDEADYYCQLVYSSDTYVFGTGTKVTVL | QVQLVESGGGVVQPGRSLRLSCAASGFTFRNYGMHWVRQAPGKGLEWVSIIWFDGSDQYYADSVKGRFTISRDNSKNTLYLQMNSLRAEDTAVYYCARGLSVYYYGMDVWGQGTTVTVSS |
| | | | SEQ ID NO: 27778 | SEQ ID NO: 31784 |

FIGURE 50

| | | | | |
|---|---|---|---|---|
| **iPS:437168** | **21-225_218G4** | NA | CAGTCTGTGTTGACGCAGCCGCCCTCAGTGTCTGCGGCCCCAGGACAGAAGGTCACCATCTCCTGCTCTGGAAGCAGCTCCAACATTGGGAATAATTATGTATCCTGGTACCAGCAGCTCCCAGGAACAGCCCCCAAACTCCTCCTTTATGACAGTAATAAGCGACCCTCAGGGATTCCTGCCCGATTCTCTGGCTCCAAGTCTGGCACGTCAGCCACCCTGGGCATCACCGGACTCCAGACTGGGGACGAGGCCGATTATTACTGCGGAACATGGGATAGCAGCCTGAATACTGTGGTATTCGGCGGAGGGACCAAGCTGACCGTCCTA | CAGGTGCAGCTGGTGGAGTCTGGGGGAGGCGTAGTCCAGCCTGGGAGGTCCCTGAGACTCTCCTGTGCAGCGTCTGGATTCACCTTCAGTAGCTATGGCTTGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGTCGAGGACACGGCTGTGTACTACTGTGCGAACTGGTACTACTATTACTACGGTATGGACGTCTGGGGCCAAGGGACCACGGTCACCGTCTCCTCA |
| | | | SEQ ID NO: 27779 | SEQ ID NO: 31785 |
| | | AA | QSVLTQPPSVSAAPGQKVTISCSGSSSNIGNNYVSWYQQLPGTAPKLLLYDSNKRPSGIPARFSGSKSGTSATLGITGLQTGDEADYYCGTWDSSLNTVVFGGGTKLTVL | QVQLVESGGGVVQPGRSLRLSCAASGFTFSSYGLHWVRQAPGKGLEWVAVIWYDGSNKYYADSVKGRFTISRDNSKNTLYLQMNSLRVEDTAVYYCANWYYYYGMDVWGQGTTVTVSS |
| | | | SEQ ID NO: 27780 | SEQ ID NO: 31786 |
| **iPS:437170** | **21-225_218E5** | NA | TCCTATGAGCTGACACAGCCACCCTCGGTGTCAGTGTCCCCAGGACAGACGGCCAGGATCACCTGCTCTAGAGATGTATTGCCGAAGCAATATGCTTATTGGTACCAGCAGAAGCCAGGCCAGGCCCCAGTACTGGTGATATATAAAGACAGTGAGAGGCCCTCAGGGATCCCTGAGCGATTCTCTGGCTCCCGCTCAGGGACAACAGTCACGTTGACCATCAGAGGAGTCCAGGCAGAAGACGAGGCTGACTATTACTGTCAATTAGTTGTCAGCAGTGATACTTATGTCTTCGGAACTGGGACCAAGGTCACCGTCCTA | CAGGTGCAGTTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGATGGCAATTATATGGTTTGATGGAAGTGATGAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTATATTACTGTGCGAGAGGCCTATCTGTCTACTACTACGGTATGGACGTCTGGGGCCAAGGGACCACGGTCACCGTCTCCTCA |
| | | | SEQ ID NO: 27781 | SEQ ID NO: 31787 |

FIGURE 50

| | | | | |
|---|---|---|---|---|
| | | AA | SYELTQPPSVSVSPGQTARITCSRDVLPKQYAYWYQQKPGQAPVLVIYKDSERPSGIPERFSGSRSGTTVTLTIRGVQAEDEADYYCQLVVSSDTYVFGTGTKVTVL | QVQLVESGGGVVQPGRSLRLSCAASGFTFSNYGMHWVRQAPGKGLEWMAIIWFDGSDEYYADSVKGRFTISRDNSKNTLYLQMNSLRAEDTAVYYCARGLSVYYYGMDVWGQGTTVTVSS |
| | | | SEQ ID NO: 27782 | SEQ ID NO: 31788 |
| iPS:437172 | 21-225_219A7 | NA | CAGTCTGCCCTGACTCAGCCTGCCTCCGTGTCTGGGTCTCCTGGACAGTCGATCACCATCTCCTGCACTGGAACCAGCAGTGACGTTGGTGGTTATAATTATGTCTCCTGGTACCAACAGCACCCAGGCAAAGCCCCCAAACTCATGATTTATGAGGTCAGAAATCGGCCCTCAGGGGTTTCTAACCGCTTCTCTGGCTCCAAGTCTGGCAACACGGCCTCCCTGACCATCTCTGGGCTCCAGGCTGAGGACGAGGCTGATTATTACTGCTGCTCATATACAAGGAGCATCACTTGGGTGTTCGGCGGAGGGACCAAGTTGACCGTCCTA | CAGGTGCAGCTGGTGGAGTCTGGGGGAGGCGTGGTCCAGCCTGGGAGGTCCCTGAGACTCTCCTGTCCAGCGTCTGGATTCACCTTCAGTCACTATGGCATGCACTGGGTCCGCCAGGCTCCAGGCAAGGGGCTGGAGTGGGTGGCAGTCATATGGTATGATGGAAGTAATAAATACTATGCAGACTCCGTGAAGGGCCGATTCACCATCTCCAGAGACAATTCCAAGAATATGCTGTATCTGCAAATGAACAGCCTGAGAGCCGAGGACACGGCTGTGTATTACTGTGCGAGGGGGGACTGGAACCCCGAGGGTATGGACGTCTGGGGCCAAGGGACCACGGTCACCGTCTCCTCA |
| | | | SEQ ID NO: 27783 | SEQ ID NO: 31789 |
| | | AA | QSALTQPASVSGSPGQSITISCTGTSSDVGGYNYVSWYQQHPGKAPKLMIYEVRNRPSGVSNRFSGSKSGNTASLTISGLQAEDEADYYCCSYTRSITWVFGGGTKLTVL | QVQLVESGGGVVQPGRSLRLSCPASGFTFSHYGMHWVRQAPGKGLEWVAVIWYDGSNKYYADSVKGRFTISRDNSKNMLYLQMNSLRAEDTAVYYCARGDWNPEGMDVWGQGTTVTVSS |
| | | | SEQ ID NO: 27784 | SEQ ID NO: 31790 |

FIGURE 50

| | | | | |
|---|---|---|---|---|
| **iPS:437182** | **21-225_221H2** | NA | CTGTCTGCCCTGACTCAGCCTGCCTCCGTGTCTGGGTCTCCTGGACAGTCGATCACCATCTCCTGCACTGGAACCAGCAGTGACGTTGGTGGTTATAACTATGTCTCCTGGTACCAACAACACCCAGGCAAAGCCCCCAAACTCATGATTTCTGAGGTCAGGAATCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCAACTCATATACACGCAGCATCACTTGGGTGTTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTCACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGCATCTGCAAATGAACAGCCTGAGAGCCGAGGACACGGCTGTGTATTACTGTGCGAGGGGGGACTGGAACCCCGAGGGTATGGACGTCTGGGGCCAAGGGACCACGGTCACCGTCTCCTCA |
| | | | SEQ ID NO: 27785 | SEQ ID NO: 31791 |
| | | AA | LSALTQPASVSGSPGQSITISCTGTSSDVGGYNYVSWYQQHPGKAPKLMISEVRNRPSGVSNRFSGSKSGNTASLTISGLQAEDEADYYCNSYTRSITWVFGGGTKLTVL | QVQLVESGGGVVQPGRSLRLSCAASGFTFSHYGMHWVRQAPGKGLEWVAVIWYDGSNKYYADSVKGRFTISRDNSKNTLHLQMNSLRAEDTAVYYCARGDWNPEGMDVWGQGTTVTVSS |
| | | | SEQ ID NO: 27786 | SEQ ID NO: 31792 |
| **iPS:437184** | **21-225_221G4** | NA | CAGTCTGCCCTGACTCAGCCTGCCTCCGTGTCTGGGTCTCCTGGACAGTCGATCACCATCTCCTGCACTGGAACCAGCAGTGACGTTGGTGGTTATAACTATGTCTCCTGGTACCAACAACACCCAGGCAAAGCCCCCAAACTCATGATTTATGAGGTCAGGAATCGGCCCTCAGGGGTTTCTAATCGCTTCTCTGGCTCCAAGTCTGGCAACACGGCCTCCCTGACCATCTCTGGGCTCCAGGCTGAGGACGAGGCTGATTATTACTGCAACTCATATACACGCAGCATCACTTGGGTGTTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTCACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGCATCTGCAAATGAACAGCCTGAGAGCCGAGGACACGGCTGTGTATTACTGTGCGAGGGGGGACTGGAACCCCGAGGGTATGGACGTCTGGGGCCAAGGGACCTCGGTCACCGTCTCCTCA |
| | | | SEQ ID NO: 27787 | SEQ ID NO: 31793 |

FIGURE 50

| | | | | |
|---|---|---|---|---|
| | | AA | QSALTQPASVSGSPGQSITISCTGTSSDVGGYNY VSWYQQHPGKAPKLMIYEVRNRPSGVSNRFSGS KSGNTASLTISGLQAEDEADYYCNSYTRSITWVF GGGTKLTVL | QVQLVESGGGVVQPGRSLRLSCAASGFTFSHYGM HWVRQAPGKGLEWVAVIWYDGSNKYYADSVKGR FTISRDNSKNTLHLQMNSLRAEDTAVYYCARGDW NPEGMDVWGQGTSVTVSS |
| | | | SEQ ID NO: 27788 | SEQ ID NO: 31794 |
| **iPS:437186** | **21-225_224H2** | NA | TCCTATGAGCTGACTCAGCCATCCTCAGTGTC CGTGTCCCCAGGACAGACAGCCAGCATCACCT GCTCTGGAGATAATTTGGGGGTTAAATATACT TACTGGTATCAGCAGAAGCCAGGCCAGTCCCC TGTGCTAGTCGTCTATCAAGATAGCAAGCGGC CCTCAGGGATCCCTGAGCGATTCTCTGGCTCC AACTCTGGGAACACAGCCACTCTGACCATCAG CGGGACCCAGGCTATGGATGAGGCTGACTATT ACTGTCAGGCGTGGGACAGCAGCACTGTGGTA TTCGGCGGAGGGACCAAGCTGACCGTCCTA | CAGGTACAGCTGCAGCAGTCAGGTCCAGGACTG GTGAAGCCCTCGCAGACCCTCTCACTCACCTGTG ACATCTCCGGGGACAGTGTCTCTAGCAACAGTGC TGCTTGGAACTGGATCAGGCAGTCCCCATCGCGA GGCCTTGAGTGGCTGGGAAGGACATACTACAGG TCCAAGTGGTATAATGATTATGCAGTATCTGTGA AAAGTCGAGTAACCATCAACCCAGACACATCCA AGAACCAGTTCTCCCTGCAGCTGAACTCTGTGAC TCCCGAGGACACGGCTGTGTATTACTGTGCAAGA GAGGGGGGCCTAGGATATTGTAGTAGTACCAGCT GCTATGGAGGCTGGTTCGACCCCTGGGGCCAGG GAACCCTGGTCACCGTCTCCTCA |
| | | | SEQ ID NO: 27789 | SEQ ID NO: 31795 |
| | | AA | SYELTQPSSVSVSPGQTASITCSGDNLGVKYTYW YQQKPGQSPVLVVYQDSKRPSGIPERFSGSNSGN TATLTISGTQAMDEADYYCQAWDSSTVVFGGG TKLTVL | QVQLQQSGPGLVKPSQTLSLTCDISGDSVSSNSAA WNWIRQSPSRGLEWLGRTYYRSKWYNDYAVSVK SRVTINPDTSKNQFSLQLNSVTPEDTAVYYCAREGG LGYCSSTSCYGGWFDPWGQGTLVTVSS |
| | | | SEQ ID NO: 27790 | SEQ ID NO: 31796 |

FIGURE 50

| | | | | |
|---|---|---|---|---|
| **iPS:437188** | **21-225_224B11** | NA | CAGTCTGTGCTGACGCAGCCGCCCTCAGTGTCTGGGGCCCCAGGGCAGAGGGTCACCATCTCCTGCACTGGGAGCAGCTCCAACATCGGGGCAGGTTATGATGTACACTGGTACCAGCAGCTTCCAGGAACAGCCCCCAAACTCCTCATCTTTGGTAACAGCAATCGGCCCTCAGGGGTCCCTGACCGATTCTCTGGCTCCAAGTCTGGCACCTCAGCCTCCCTGGCCATCACTGGGCTCCAGGCTGAGGATGAGGCTGATTATCACTGCCAGTCCTATGACAACAGCCTGAGTGGTGTGTTCGGCGGAGGGACCAAGCTGACCGTCCTA | CAGGTACACCTGGTGCAGTCTGGGGCTGAGGTGAAGAAGCCTGGGGCCTCAGTGAAGGTCTCCTGCAAGGCTTCTGGATACACCTTCACCGGCTACTATATACACTGGGTGCGACAGGCCCCTGGACAAGGGCTTGAGTGGATGGGATGGATCAACCCTAAAAATGGTGGCACAAACTATGCACAGAAGTTTCAGGGCAGGGTCACCATGACCAGGGACGCGTCCATCAGCACAACCTACATGGAGCTGAGCAGGCTGAGATCTGACGACACGGCCGTGTATTACTGTGCGAGAGGAGCGTTTGATTACTTCTACTACTACGCTATGGACGTCTGGGGCCACGGGACCACGGTCACCGTCTCCTCA |
| | | | SEQ ID NO: 27791 | SEQ ID NO: 31797 |
| | | AA | QSVLTQPPSVSGAPGQRVTISCTGSSSNIGAGYDVHWYQQLPGTAPKLLIFGNSNRPSGVPDRFSGSKSGTSASLAITGLQAEDEADYHCQSYDNSLSGVFGGGTKLTVL | QVHLVQSGAEVKKPGASVKVSCKASGYTFTGYYIHWVRQAPGQGLEWMGWINPKNGGTNYAQKFQGRVTMTRDASISTTYMELSRLRSDDTAVYYCARGAFDYFYYYAMDVWGHGTTVTVSS |
| | | | SEQ ID NO: 27792 | SEQ ID NO: 31798 |
| **iPS:437190** | **21-225_225A9** | NA | TCCTATGAGCTGACTCAGCCACCCTCAGTGTCCGTGTCCCCAGGACAGACAGCCAGCATCACCTGCTCTGGAGATAAATTGGGGAATAAATATGCTTGCTGGTATCAGCAGAAGCCAGGCCAGTCCCCTGTGTTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAACACTGCATGTGTCTTCGGAACTGGGACCAAGGTCACCGTCCTA | CAGGTGCAGCTGGTGGAGTCTGGGGGAGGCGTGGTCCAGCCTGGGGGGTCCCTGAGACTCTCCTGTGCAGCGTCTGGATTCACCTTCAGTACCTATGGCATGCACTGGGTCCGCCTGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACATTTCCCAGAACACGCTGTATCTGCAAATGAACAGCCTGAGAGCCGAGGACACGGCTGTGTATTACTGTGCGAGAGATAACCACTATTGTAGTAGTACCAGCTGCTCCCCATACTACTACTACTTCGGTATGGACGTCTGGGGCCAAGGGACCACGGTCACCGTCTCCTCA |

FIGURE 50

| | | | | |
|---|---|---|---|---|
| | | | SEQ ID NO: 27793 | SEQ ID NO: 31799 |
| | | AA | SYELTQPPSVSVSPGQTASITCSGDKLGNKYACWYQQKPGQSPVLVIYQDSKRPSGIPERFSGSNSGNTATLTISGTQAMDEADYYCQAWDSNTACVFGTGTKVTVL | QVQLVESGGGVVQPGGSLRLSCAASGFTFSTYGMHWVRLAPGKGLEWVAVIWYDGSNKYYADSVKGRFTISRDISQNTLYLQMNSLRAEDTAVYYCARDNHYCSSTSCSPYYYYFGMDVWGQGTTVTVSS |
| | | | SEQ ID NO: 27794 | SEQ ID NO: 31800 |
| **iPS:437192** | **21-225_225E9** | NA | TCCTATGACCTGACTCAGCCACCCTCAGTGTCCGTGTCCCCAGGACAGACAGCCAGCATCACCTGCTCTGGAGATAATTTGGGGAATAGATATGCTTGCTGGTATCAGCAGAAGCCAGGCCAGTCCCCTGTACTGGTCATGTATCAAGATCGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGAACTGCTGTGGTATTCGGCGGAGGGACCAAGCTGACCGTCCTA | CAGGTGCAGTTGGTGGAGTCTGGGGGAGGCGTGGTCCAGCCTGGGAGGTCCCTGAGACTCTCCTGTGAAGCGTCTGGATTCATCTTCAGTAGCTATGGCATGCACTGGGTCCGCCAGGCTCCAGGCAAGGGGCTGGAGTGGGTGGCAGTTATGTGGTATGATGGAGGTAATAAAGACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGGGAATATTGTACTAGTACCAGCTGCCCTTACTACTACTACTACGGTATGGACGTCTGGGGCCAAGGGACCACGGTCACCGTCTCCTCA |
| | | | SEQ ID NO: 27795 | SEQ ID NO: 31801 |
| | | AA | SYDLTQPPSVSVSPGQTASITCSGDNLGNRYACWYQQKPGQSPVLVMYQDRKRPSGIPERFSGSNSGNTATLTISGTQAMDEADYYCQAWDSRTAVVFGGGTKLTVL | QVQLVESGGGVVQPGRSLRLSCEASGFIFSSYGMHWVRQAPGKGLEWVAVMWYDGGNKDYADSVKGRFTISRDNSKNTLYLQMNSLRAEDTAVYYCARDREYCTSTSCPYYYYYGMDVWGQGTTVTVSS |
| | | | SEQ ID NO: 27796 | SEQ ID NO: 31802 |

FIGURE 50

| | | | | |
|---|---|---|---|---|
| **iPS:437194** | **21-225_226B2** | NA | TCCTATGAGCTGACTCAGCCACCCTCAGTGTCCGTGTCCCCAGGACAGACAGCCAGCATCACCTGCTCTGGAGATACATTGGGGGGTAAATATGCTTGGTGGTATCAGCAGAGGCCAGGCCAGTCCCCTGTGCTGGTCATCTATCAAGATCGCAAGCGGCCCTCAGGGATCCCTGAGCGATTCTCTGGCTCCAGCTCTGGGAACACAGCCACTCTGACCATCAGCGGGACCCAGGCTATGGATGAGGCTGACTATTACTGTCAGGCGTGGGACAACGGCGCTGCGGTTTTCGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTTTATGCACTGGGTGCGACAGGCCCCTGGACAAGGGCTTGAGTGGATGGGATGGATCAACCCTAACAGTGGTGACACAAACTATGCACAGAAGTTTCAGGGCAGGGTCACCATGACCAGGGACACGTCCCTCAACACAGCCTACATGGAGCTGAGCAGGCTGAGATCTGACGACACGGCCATTTATTACTGTGCGAGAGGGACTTACTATGGTTCGGGGAGTTATTTTAACGAACTTGACTCCTGGGGCCAGGGAACCCTGGTCACCGTCTCCTCA |
| | | | SEQ ID NO: 27797 | SEQ ID NO: 31803 |
| | | AA | SYELTQPPSVSVSPGQTASITCSGDTLGGKYAWWYQQRPGQSPVLVIYQDRKRPSGIPERFSGSSSGNTATLTISGTQAMDEADYYCQAWDNGAAVFGGGTKLTVL | QVQLVQSGAEVKKPGASVKVSCKASGYTFTGYFMHWVRQAPGQGLEWMGWINPNSGDTNYAQKFQGRVTMTRDTSLNTAYMELSRLRSDDTAIYYCARGTYYGSGSYFNELDSWGQGTLVTVSS |
| | | | SEQ ID NO: 27798 | SEQ ID NO: 31804 |
| **iPS:437196** | **21-225_226B7** | NA | TCCTTTGAGCTGACACAGCCACCCTCGGTGTCAGTGTCCCCAGGACAGACGGCCAGGATCACCTGCTCTGGAGATGCATTGCCAAGGCATTATGTTTATTGGTACCAGCAGAACCCAGGCCAGGCCCCTGTGCTGGTGATATATAAAGACAGTGAGAGGCCCTCAGGGATCCCTGAGCGATTCTCTGGCTCCAGCTCAGGGACAACAGTCACGTTGACCATCAGTGGAGTCCAGGCAGAAGACGAGGCTGACTATTACTGTCAATCAGCAGACAGCAGTGGTACTTATGTCTTCGGAACTGGGACCAAGGTCACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGACTTGAGTGGATGGGATGGATCAACCCTAACAGTGGAGGCACAAACTATGCACAGAAGTTTCAGGACAGGGTCACCATGACCAGGGACACGTCCATCAGCACAGCCCACATGGAGCTGAGCAGGCTGAGATCTGACGACACGGCCGTGTATTATTGTGCGAGAGGATATTACTATGGTTCGGGGAGTTATTATAACTGGTTCGACTCCTGGGGCCAGGGAACCCTGGTCACCGTCTCCTCA |
| | | | SEQ ID NO: 27799 | SEQ ID NO: 31805 |

FIGURE 50

| | | | | |
|---|---|---|---|---|
| | | AA | SFELTQPPSVSVSPGQTARITCSGDALPRHYVYWYQQNPGQAPVLVIYKDSERPSGIPERFSGSSSGTTVTLTISGVQAEDEADYYCQSADSSGTYVFGTGTKVTVL | QVQLVQSGAEVKKPGASVKVSCKASGYTFTGYYMHWVRQAPGQGLEWMGWINPNSGGTNYAQKFQDRVTMTRDTSISTAHMELSRLRSDDTAVYYCARGYYYGSGSYYNWFDSWGQGTLVTVSS |
| | | | SEQ ID NO: 27800 | SEQ ID NO: 31806 |
| iPS:437198 | 21-225_226F8 | NA | CAGTCTGTGCTGACGCAGCCGCCCTCAGTGTCTGGGGCCCCAGGGCAGAGGGTCACCATCTCCTGCACTGGGAGCAGCTCCAACATCGGGGCAGGTTATGATGTACACTGGTACCAGCAGCTTCCAGGAACAGCCCCCAAACTCCTCATCTATGGTAACAGCAATCGGCCCTCAGGGGTCCCTGACCGATTCTCTGGCTCCAAGTCTGACACCTCAGCCTCCCTGGCCATCACTGGGCTCCAGGCTGAGGATGAGGCTGATTATTACTGCCAGTCCTATGACAACAGCCTGAGTGGTGTGTTCGGCGGAGGGACCAAGCTGACCGTCCTA | CAGGTGCAACTGGTGCAGTCTGGGGCTGAGGTGAAGAAGCCTGGGGCCTCAGTGAAAGTCTCCTGCAAGGCTTCTGGATACACCTTCACCGGCTACTATATACACTGGGTGCGACAGGCCCCTGGACAAGGGCTTGAGTGGATGGGATGGATCAACCCTAACAGTGGTGGCACAAACTATGCACGGAAGTTTCAGGGCAGGGTCACCATGACCAGGGACACGTCCATCAGCACAGCCTACATGGAGCTGAGCAGACTGAGATCTGACGACACGGCCGTGTATTACTGTGCGAGAGGAGCGTTTGATTACTACTACTACTACGCTTTGGACGTCTGGGGCCAAGGGACCACGGTCACCGTCTCCTCA |
| | | | SEQ ID NO: 27801 | SEQ ID NO: 31807 |
| | | AA | QSVLTQPPSVSGAPGQRVTISCTGSSSNIGAGYDVHWYQQLPGTAPKLLIYGNSNRPSGVPDRFSGSKSDTSASLAITGLQAEDEADYYCQSYDNSLSGVFGGGTKLTVL | QVQLVQSGAEVKKPGASVKVSCKASGYTFTGYYIHWVRQAPGQGLEWMGWINPNSGGTNYARKFQGRVTMTRDTSISTAYMELSRLRSDDTAVYYCARGAFDYYYYYALDVWGQGTTVTVSS |
| | | | SEQ ID NO: 27802 | SEQ ID NO: 31808 |

FIGURE 50

| | | | | |
|---|---|---|---|---|
| iPS:437200 | 21-225_226A10 | NA | TCCTATGAGCTGACTCAGCCACCCTCAGTGTCCGTGTCCCCAGGACAGACAGCCAGCATCACCTGCTCTGGAGATACATTGGGGGGTAAATATGCTTGGTGGTATCAGCAGAAGCCAGGCCAGTCCCCTGTGCTGGTCATCTATCAAGATCGCAAGCGGCCCTCAGGGATCCCTGAGCGATTCTCTGGCTCCAGCTCTGGGAACACAGCCACTCTGACCATCAGCGGGACCCAGGCTATGGATGAGGCTGACTATTACTGTCAGGCGTGGGACAACGGCGCTGCGGTTTTCGGCGGAGGGACCAAGCTGACCGTCCTA | CAGGTGCAACTGGTGCAGTCTGGGGCTGAGGTGAAGAAGCCTGGGGCCTCAGTGAAGGTCTCCTGCAAGGCTTCTGGATACACCTTCACCGGCTACTTTATGCACTGGGTGCGACAGGCCCCTGGACAAGGGCTTGAGTGGATGGGTTGGATCAACCCTAACAGTGGTGACACAAACTATGCACAGAAGTTTCAGGGCAGGGTCACCATGACCAGGGACACGTCCCTCAGCACAGCCTACATGGAGCTGAGCAGGCTGAGATCTGACGACACGGCCATTTATTACTGTGCGAGAGGGACTTACTATGGTTCGGGGAGTTATTTTAACGAACTTGACTCCTGGGGCCAGGGAACCCTGGTCACCGTCTCCTCA |
| | | | SEQ ID NO: 27803 | SEQ ID NO: 31809 |
| | | AA | SYELTQPPSVSVSPGQTASITCSGDTLGGKYAWWYQQKPGQSPVLVIYQDRKRPSGIPERFSGSSSGNTATLTISGTQAMDEADYYCQAWDNGAAVFGGGTKLTVL | QVQLVQSGAEVKKPGASVKVSCKASGYTFTGYFMHWVRQAPGQGLEWMGWINPNSGDTNYAQKFQGRVTMTRDTSLSTAYMELSRLRSDDTAIYYCARGTYYGSGSYFNELDSWGQGTLVTVSS |
| | | | SEQ ID NO: 27804 | SEQ ID NO: 31810 |
| iPS:437202 | 21-225_227D3 | NA | CAGTCTGTGCTGACGCAGCCGCCCTCAGTGTCTGGGGCCCCAGGGCAGAGGGTCACCATCTCCTGCACTGGGAGCAGCTCCAACATCGGGGCAGGTTATGATGTACACTGGTACCAGCAGCTTCCAGGAACAGCCCCCAAACTCCTCATCTATGGTAACAGCAATCGGCCCTCAGGGGTCCCTGACCGATTCTCTGGCTCCAAGTCTGACACCTCAGCCTCCCTGGCCATCACTGGGCTCCAGGCTGAGGATGAGGCTGATTATTACTGCCAGTCCTATGACAACAGCCTGAGTGGTGTGTTCGGCGGAGGGACCAAGCTGACCGTCCTA | CAGGTGCAATTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGCGGATGGGATGGATCAACCCTAAGAGTGGTGGCACAAACTTTGCACAGAAGTTTCAGGGCAGGGTCACCATGACCAGGGACACGTCCATCAGCACAGCCTACATGGAACTGAGCAGGCTGAGATCTGACGACACGGCCGTGTATTACTGCGCGAGAGGAGCGTTTGATTACTTCTACTACTACGGTATGGACGTCTGGGGCCAAGGGACCACGGTCACCGTCTCCTCA |
| | | | SEQ ID NO: 27805 | SEQ ID NO: 31811 |

FIGURE 50

| | | | | |
|---|---|---|---|---|
| | | AA | QSVLTQPPSVSGAPGQRVTISCTGSSSNIGAGYD VHWYQQLPGTAPKLLIYGNSNRPSGVPDRFSGS KSDTSASLAITGLQAEDEADYYCQSYDNSLSGV FGGGTKLTVL | QVQLVQSGAEVKKPGASVKVSCKASGYTFTGYYM HWVRQAPGQGLERMGWINPKSGGTNFAQKFQGR VTMTRDTSISTAYMELSRLRSDDTAVYYCARGAFD YFYYYGMDVWGQGTTVTVSS |
| | | | SEQ ID NO: 27806 | SEQ ID NO: 31812 |
| **iPS:437204** | **21-225_227E5** | NA | TCCTATGAGCTGAGTCAGCCACCCTCAGTGTC CGTGTCCCCAGGACAGACAGCCAGCATCACCT GCTCTGGAGATAAATTGGGGGAAAAATATGCT TGCTGGTATCAGCAGAAGCCAGGCCAGTCCCC TGTGCTGGTCATCTATCAAGATAGGAAGCGGC CCTCAGGGATCCCTGAGCGATTCTCTGGCTCC AACTCTGGGAACAAAGCCACTCTGACCATCAG CGGGACCCAGGCTATGGATGAGGCTGACTATT ACTGTCAGGCGTGGGTCAACAACACTATGATA TTCGGCGGAGGGACCAAGCTGACCGTCCTA | GAGGTGCAGCTGTTGGAGTCTGGGGGAGGCTTG GTACAGCCTGAGGGGTCCCTGAGACTCTCCTGTG CAGCCTCTGGATTCACCTTTAGCAGCTATGCCAT GAGCTGGGTCCGCCAGGCTCCAGGGAAGGGGCT GGAGTGGGTCTCAGCTATTAGTGGTAGTGGTGGT AGCACATACTACGCAGACTCCGTGAAGGGCCGG TTCACCATCTCCAGAGACAATTCCAAGAACACGC TGTGTCTGCAAATGAACAGCCTGAGAGCCGAGG ACACGGCCGTATATTACTGTGCGAAAGAATATTG TGGTGGTGACTGCTATTCCCCTTACTACTACTACT ACGGTATGGACGTCTGGGGCCAAGGGACCACGG TCACCGTCTCCTCA |
| | | | SEQ ID NO: 27807 | SEQ ID NO: 31813 |
| | | AA | SYELSQPPSVSVSPGQTASITCSGDKLGEKYACW YQQKPGQSPVLVIYQDRKRPSGIPERFSGSNSGN KATLTISGTQAMDEADYYCQAWVNNTMIFGGG TKLTVL | EVQLLESGGGLVQPEGSLRLSCAASGFTFSSYAMS WVRQAPGKGLEWVSAISGSGGSTYYADSVKGRFTI SRDNSKNTLCLQMNSLRAEDTAVYYCAKEYCGGD CYSPYYYYYGMDVWGQGTTVTVSS |
| | | | SEQ ID NO: 27808 | SEQ ID NO: 31814 |

FIGURE 50

| | | | | |
|---|---|---|---|---|
| **iPS:437208** | **21-225_227C10** | NA | CAGTCTGTGCTGACGCAGCCGCCCTCAGTGTCTGGGGCCCCAGGGCAGAGGGTCACCATCTCCTGCACTGGGAGCAGCTCCAACATCGGGGCAGGTTATGATGTACACTGGTACCAGCAGCTTCCAGGAACAGCCCCCAAACTCCTCATCTATGGTAACAGCAATCGGCCCTCAGGGGTCCCTGACCGATTCTCTGGCTCCAAGTCTGACACCTCAGCCTCCCTGGCCATCACTGGGCTCCAGGCTGAGGATGAGGCTGATTATTACTGCCAGTCCTATGACAACAACCTGAGTGGTGTGTTCGGCGGAGGGACCAAGCTGACCGTCCTA | CAGGTGCAATTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGCGGATGGGATGGATCAACCCTAAGAGTGGTGGCACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTGTATTACTGCGCGAGAGGAGCGTTTGATTACTTCTACTACTACGGTATGGACGTCTGGGGCCAAGGGACCACGGTCACCGTCTCCTCA |
| | | | SEQ ID NO: 27809 | SEQ ID NO: 31815 |
| | | AA | QSVLTQPPSVSGAPGQRVTISCTGSSSNIGAGYDVHWYQQLPGTAPKLLIYGNSNRPSGVPDRFSGSKSDTSASLAITGLQAEDEADYYCQSYDNNLSGVFGGGTKLTVL | QVQLVQSGAEVKKPGASVKVSCKASGYTFTGYYMHWVRQAPGQGLERMGWINPKSGGTNYAQKFQGRVTMTRDTSISTAYMELSRLRSDDTAVYYCARGAFDYFYYYGMDVWGQGTTVTVSS |
| | | | SEQ ID NO: 27810 | SEQ ID NO: 31816 |
| **iPS:437210** | **21-225_227E12** | NA | TCCTATGAACTGACTCAGCCACCCTCAGTGTCCGTGTCCCCAGGACAGACAGCCAGCATCACCTGCTCTGGAGATAAATTGGGGGATAAATATGTTTGTTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGAACAGCAGCAATGTGGTATTCGGCGGAGGGACCAAGCTGACCGTCCTA | CAGATCACCTTGAAGGAGTCTGGTCCTACGCTGGTGAAACCCACACAGACCCTCACGCTGACCTGCACCTTCTCTGGGTTCTCACTCAGCACTAGTGGAGTGGGTGTGGGCTGGATCCGTCAGCCCCCAGGAAAGGCCCTGGAGTGCCTTTCACTCATTTATTGGAATGATGATAAGGTCTACAGCCCATCTCTGAAGAGCAGGCTCACCATCACCAAGTACACCTCCAAAAACCAGGTGGTCCTTACAATGACCAACATGGACCCTGTGGACACAGCCACATATTACTGTGCACACAGGGGACAGCAGCTGGCCCTCGACTACTGGGGCCAGGGAACCCTGGTCACCGTCTCCTCA |
| | | | SEQ ID NO: 27811 | SEQ ID NO: 31817 |

FIGURE 50

| | | | | |
|---|---|---|---|---|
| | | AA | SYELTQPPSVSVSPGQTASITCSGDKLGDKYVCWYQQKPGQSPVLVIYQDSKRPSGIPERFSGSNSGNTATLTISGTQAMDEADYYCQAWNSSNVVFGGGTKLTVL | QITLKESGPTLVKPTQTLTLTCTFSGFSLSTSGVGVGWIRQPPGKALECLSLIYWNDDKVYSPSLKSRLTITKYTSKNQVVLTMTNMDPVDTATYYCAHRGQQLALDYWGQGTLVTVSS |
| | | | SEQ ID NO: 27812 | SEQ ID NO: 31818 |
| iPS:437214 | 21-225_48B12 | NA | TCCTATGAGCTGACACAGCCACCCTCGGTGTCAGTGTCCCCAGGACAAACGGCCAGGATCACCTGCTCTGGAGATGCATTGCCAAAAAAATATGCTTATTGGTACCAGCAGAAGTCAGGCCAGGCCCCTGTGCTGGTCATCTATGAGGACAGCAAACGACCCTCCGGGATCCCTGAGAGATTCTCTGGCTCCAGCTCAGGGACAATGGCCACCTTGACTATCAGTGGGGCCCAGGTGGAGGATGAAGCTGACTACTACTGTAACTCAACAGACAGCAGTGGTAATCATGTGGTATTCGGCGGAGGGACCAAGCTGACCGTCCTA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAACTGGGTCCGCCAGGCTCCAGGGAAGGGGCTGGAGTGGGTCTCAGCTATTAGTGGTCGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAAAGAGAGACGTATAACTGGAACTACGAAGGGTTTGACTACTGGGGCCAGGGAACCCTGGTCACCGTCTCCTCA |
| | | | SEQ ID NO: 27813 | SEQ ID NO: 31819 |
| | | AA | SYELTQPPSVSVSPGQTARITCSGDALPKKYAYWYQQKSGQAPVLVIYEDSKRPSGIPERFSGSSSGTMATLTISGAQVEDEADYYCNSTDSSGNHVVFGGGTKLTVL | EVQLLESGGGLVQPGGSLRLSCAASGFTFSSYAMNWVRQAPGKGLEWVSAISGRGGNTFYADSVKGRFTISRDNSKNTLYLQMNSLRAEDTAVYYCAKRETYNWNYEGFDYWGQGTLVTVSS |
| | | | SEQ ID NO: 27814 | SEQ ID NO: 31820 |

FIGURE 50

| | | | | |
|---|---|---|---|---|
| **iPS:437216** | **21-225_51D5** | NA | GACATCCAGATGACCCAGTCTCCATCCTCGCT<br>GTCTGCATCTGTAGGAGACAGAGTCACCATCA<br>CTTGTCGGGCGAGTCAGGGCATTAACAATTAT<br>TTAGCCTGGTTTCAGCAGAAACCAGGGGAAGC<br>CCCTAAGTCCCTGATCTATGCTGCATCCAGTTT<br>GCGAAGTGGGGTCCCATCACAGTTCAGCGGCA<br>GTGGATCTGGAACAGATTTCATTCTCACCATC<br>AGCAGCCTGCAGCCTGAAGATTTTGCAACTTA<br>TTACTGCCAACAGTATTATAGTTACCCATTCAC<br>TTTCGGCCCTGGGACCAAAGTGGATATCAAA | GAGGTGCAGTTGTTGGAGTCTGGGGGAGGCTTGG<br>TACAGCCTGGGGGGTCCCTGAGACTCTCCTGTGC<br>AGCCTCTGGATTCACCTTTAGCAGCTATGTCATG<br>AGCTGGGTCCGCCAGACTCCAGGGAAGGGGCTG<br>GAGTGGGTCTCAACTATGAGTGGTAGTGGTGGTC<br>GCACATACTACGCAGACTCCGTGAACGGCCGATT<br>CACCGTCTCCAGAGACAATTCCAAGAACACGCTG<br>TATTTGCAAATGAGCAGCCTGAGAGCCGAGGAC<br>ACGGCCGTATATTACTGTGCGCGGTGACTGCTT<br>TTGACTACTGGGGCCAGGGAACCCTGGTCACCGT<br>CTCCTCA |
| | | | SEQ ID NO: 27815 | SEQ ID NO: 31821 |
| | | AA | DIQMTQSPSSLSASVGDRVTITCRASQGINNYLA<br>WFQQKPGEAPKSLIYAASSLRSGVPSQFSGSGSG<br>TDFILTISSLQPEDFATYYCQQYYSYPFTFGPGTK<br>VDIK | EVQLLESGGGLVQPGGSLRLSCAASGFTFSSYVMS<br>WVRQTPGKGLEWVSTMSGSGGRTYYADSVNGRFT<br>VSRDNSKNTLYLQMSSLRAEDTAVYYCARVTAFD<br>YWGQGTLVTVSS |
| | | | SEQ ID NO: 27816 | SEQ ID NO: 31822 |
| **iPS:437220** | **21-225_55H6** | NA | GACATCCAGATGACCCAGTCTCCATCCTCCCT<br>GTCTGCATCTGTAGGAGACAGAGTCACCATCA<br>CTTGCCGGGCAAGTCAGGGCATTAGAAACGAT<br>TTAGGCTGGTATCAGCAGAAACCAGGGAAAG<br>CCCCTAAGCGCCTGATCTATGGTGCATCCAGT<br>TTGCAAAGTGGGGTCCCATCAAGATTCAGCGG<br>CAGTGGATCTGGGACAGAATTCACTCTCACAA<br>TCAGCAGCCTGCAGCCTGAAGATTTTGCAGCT<br>TATTACTGTCTACAGCGTGATAGTTACCCGTTC<br>ACTTTCGGCGGAGGGACCAAGGTGGAGATCA<br>AA | GAGGTGCACCTGGTGGAGTCTGGGGGAGGCCTG<br>GTCAAGCCTGGGGGGTCCCTGAGACTCTCCTGTG<br>CAGCCTCTGGATTCACCTTCAGTAGCTATAGCAT<br>GAACTGGGTCCGCCAGGCTCCAGGGAAGGGGCT<br>GGAGTGGGTCTCATCCATTAGTGGTAGTAGTACT<br>TACATATACTACGCAGACTCAGTGAAGGGCCGAT<br>TCACCATCTCCAGAGACAACGCCAAGAACTCACT<br>GTATCTGCAAATGAACAGCCTGAGAGCCGAGGA<br>CACGGCTGTGTATTACTGTGCGAGAACTGGGGTC<br>TTTGACTACTGGGGCCAGGGAACCCTGGTCACCG<br>TCTCCTCA |
| | | | SEQ ID NO: 27817 | SEQ ID NO: 31823 |

FIGURE 50

| | | | | |
|---|---|---|---|---|
| | | AA | DIQMTQSPSSLSASVGDRVTITCRASQGIRNDLG WYQQKPGKAPKRLIYGASSLQSGVPSRFSGSGS GTEFTLTISSLQPEDFAAYYCLQRDSYPFTFGGG TKVEIK | EVHLVESGGGLVKPGGSLRLSCAASGFTFSSYSMN WVRQAPGKGLEWVSSISGSSTYIYYADSVKGRFTIS RDNAKNSLYLQMNSLRAEDTAVYYCARTGVFDY WGQGTLVTVSS |
| | | | SEQ ID NO: 27818 | SEQ ID NO: 31824 |
| iPS:437224 | 21-225_56H1 | NA | GACATCCAGATGACCCAGTCTCCATCCTCACT GTCTGCATCTGTAGGAGACAGAGTCACCATCA CTTGTCGGGCGAGTCAGGGCATTAGCCATTAT TTAGCCTGGTTTCAGCAGAAACCAGGGAAAGC CCCTCAGTCCCTGATGTCTGCTGCATCCGGTTT GCAAAGTGGGGTCCCTTCAAAGTTCAGCGGCA GTGGATCTGGGACAGATTTCACTCTCACCATC AGCAGCCTGCAGCCTGAAGATTTTGCAACTTA CTACTGTCAACAATATCAGAATTACCCCTTCA CTTTCGGCCCTGGGACCAAAGTGGATATCAAA | GAGGTGCAGCTGGTGGAATCTGGGGGAGGCCTG GTCAAGCCGGGGGGGTCCCTGAGACTCTCCTGTG CAGCCTCTGGATTCACCTTCAGTAACTATAGAAT GAACTGGGTCCGCCAGGGTCCAGGGAAGGGGCT GGAGTGGATCTCATCCATTAGTGGTAGTAGTACT GACATATACTACGCAGACTCAGTGAAGGGCCGA TTCACCATCTCCAGAGACAACGCCAAGAACTCAC TGTATCTGCAAATGAACAGCCTGAGAGCCGAGG ACACGGCTGTGTATTACTGTGCGAGAGTGGCCTC CTTTGACTACTGGGGCCAGGGAACCCTGGTCACC GTCTCCTCA |
| | | | SEQ ID NO: 27819 | SEQ ID NO: 31825 |
| | | AA | DIQMTQSPSSLSASVGDRVTITCRASQGISHYLA WFQQKPGKAPQSLMSAASGLQSGVPSKFSGSGS GTDFTLTISSLQPEDFATYYCQQYQNYPFTFGPG TKVDIK | EVQLVESGGGLVKPGGSLRLSCAASGFTFSNYRMN WVRQGPGKGLEWISSISGSSTDIYYADSVKGRFTIS RDNAKNSLYLQMNSLRAEDTAVYYCARVASFDY WGQGTLVTVSS |
| | | | SEQ ID NO: 27820 | SEQ ID NO: 31826 |

FIGURE 50

| | | | | |
|---|---|---|---|---|
| **iPS:437226** | **21-225_57C2** | NA | GATGTTGTGATGACTCAGTCTCCACTCTCCCTGCCCGTCACCCTTGGACAGCCGGCCTCCATCTCCTGCAGGTCTAGTCAAAGCCTCGTATACAGTGATGGAAACACCTACTTGAATTGGTTTCAGCAGAGGCCAGGCCAATCTCCAAGGCGCCTAATTTATGAGGTTTCTAACTGGGACTCTGGGGTCCCAAACAGATTCAGCGGCAGTGGGTCAGGCACTGATTTCACACTGAAAATCAGTGCGGTGGAGGCTGAGGATGTTGGGGTTTATTACTGCGTGCAAGGTACACACTGGCCTCGGACGTTCGGCCAAGGGACCAAGGTGGAAATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTTTGGCATGAACTGGGTCCGCCAGGCTCCAGGGAAGGGGCTGGAGTGGGTCTCATCTATTAGTAGTAGTACTGGTTACATATACAACGCAGACTCAGTGAAGGGCCGATTCACCATCTCCAGAGACAACGCCAAGAACTCACTGTATCTGCAAATGAACAGCCTGAGAGCCGAAGACACGGCTGTGTATTACTGTGCGAGAACCTATAGTGGGAGCCTGGACGTCTGGGGCCAAGGGACCACGGTCACCGTCTCCTCA |
| | | | SEQ ID NO: 27821 | SEQ ID NO: 31827 |
| | | AA | DVVMTQSPLSLPVTLGQPASISCRSSQSLVYSDGNTYLNWFQQRPGQSPRRLIYEVSNWDSGVPNRFSGSGSGTDFTLKISAVEAEDVGVYYCVQGTHWPRTFGQGTKVEIK | EVQLVESGGGLVKPGGSLRLSCAASGFTFSSFGMNWVRQAPGKGLEWVSSISSSTGYIYNADSVKGRFTISRDNAKNSLYLQMNSLRAEDTAVYYCARTYSGSLDVWGQGTTVTVSS |
| | | | SEQ ID NO: 27822 | SEQ ID NO: 31828 |
| **iPS:437228** | **21-225_60C11** | NA | GAAATAGTGATGACGCAGTCTCCAGCCACCCTGTCTGTGTCTCCAGGGGAAAGAGCCACCCTCTCCTGCAGGGCCAGTCAGAGTGTTAGCAACGACTTAGCCTGGTACCAGCAGAAACCTGGCCAGGCTCCCAGGCTCCTCATCTATGGTGCATCCACCAGGGCCACTGGTATCCCAGCCAGGTTCAGTGGCGGTGGGTCTGGGACAGAGTTCACTCTCACCATCAGCGCCCTGCAGTCTGAACATTTTGCAGTTTATTACTGTCAGCAGTATAGTAACTGGCCATTCACTTTCGGCCCTGGGACCAAAGTGGATATCAAA | GAGGTGCAGCTGTTGGAGTCTGGGGGAGGCTTGGTACAGCCTGGGGGGTCCCTGAGACTCTCCTGTGCAGCCTCTGGATTCCCCTTTAGCAGCTATGCCATGAGCTGGGTCCGCCAGGCTCCAGGGAAGGGGCTGGAGTGGGTCTCAGCTATTAGTGGTAGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAATTTTTCGGTGTAGTGGGAGTCGGGTGCTTTGACTACTGGGGCCAGGGAACCCTGGTCACCGTCTCCTCA |
| | | | SEQ ID NO: 27823 | SEQ ID NO: 31829 |

FIGURE 50

| | | | | |
|---|---|---|---|---|
| | | AA | EIVMTQSPATLSVSPGERATLSCRASQSVSNDLAWYQQKPGQAPRLLIYGASTRATGIPARFSGGGSGTEFTLTISALQSEHFAVYYCQQYSNWPFTFGPGTKVDIK | EVQLLESGGGLVQPGGSLRLSCAASGFPFSSYAMSWVRQAPGKGLEWVSAISGSGGNTFYADSVKGRFTISRDNSKNTLYLQMNSLRAEDTAVYYCAKFFGVVGVGCFDYWGQGTLVTVSS |
| | | | SEQ ID NO: 27824 | SEQ ID NO: 31830 |
| iPS:437230 | 21-225_62H10 | NA | GACATCCAGATGACCCAGTCTCCATCCTCCCTGTCTGCATCTGTAGGAGACAGAGTCACCATCACTTGCCGGGCAAGTCAGAGCATTACCAGCTATTTAAATTGGTATCAGCAGAAACCAGGGAAAGTCCCTAAGCTCCTGATCTCTACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTCACAGTTTCCCATTCACTTTCGGCCCTGGGACCAATGTGGATTTCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCATCCATTAGTAGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGGGGGTTCGAGGGGGTTCGACCCCTGGGGCCAGGGAACCCTGGTCACCGTCTCCTCA |
| | | | SEQ ID NO: 27825 | SEQ ID NO: 31831 |
| | | AA | DIQMTQSPSSLSASVGDRVTITCRASQSITSYLNWYQQKPGKVPKLLISTASSLQSGVPSRFSGSGSGTDFTLTISSLQPEDFATYYCQQSHSFPFTFGPGTNVDFK | EVQLVESGGGLVKPGGSLRLSCAASGFTFSSYSMNWVRQAPGKGLEWVSSISSSSSYIYYADSVKGRFTISRDNAKNSLYLQMNSLRAEDTAVYYCARGGSRGFDPWGQGTLVTVSS |
| | | | SEQ ID NO: 27826 | SEQ ID NO: 31832 |

FIGURE 50

| | | | | |
|---|---|---|---|---|
| **iPS:437232** | **21-225_63E1** | NA | GACATTCAGATGACCCAGTCTCCATCTTCCGTGTATGCATCTGTAGGAGACAGAGTCACCATCACTTGTCGTGCGAGTCAGGGTATTAGCAGCTACTTAGCCTGGTATCAGCAGAAACCAGGGAAAGCCCCTAAGCTCCTGATCTATGCTGCATCCAGTTTGCAAAGTGGGGTCCCATCAAGATTCAGCGGCAGTGGGTCTGGGACAGATTTCACTCTCACCATCAGCAGCCTGCAGCCTGAAGATTTTGCAACTTACTATTGTCAACAGGCTAACAGTTTCCCGCTCACTTTCGGCGGAGGGACCAAGGTGGAGATCAAA | GAGGTGCAGATGTTGGAGTCTGGGGGAGGCTTGGGACAGTCGGGGGGTCCCTGAGACTCTCCTGTACAGCCTCTGGATTCACCTTCACCACTTCTGCCATGAGCTGGGTCCGCCAGGCTCCAGGGAAGGGGCTGGAGTGGGTCTCAGCTATTAGTGGTAGTGGTGCTAACACATTCTACGCAGACTCCGTGAAGGGCCGGTTCACCGTCACCAGAGACAATTCCAAGAACACGCTGTATCTGCAAATGAACAGCCTGACAGCCGAGGACACGGCCGTTTATTATTGTGTGAAAGTTATAGCAGTGGCTGGAGGGCACTTTTTCGACCCCTGGGGCCAGGGAACCCTGGTCACCGTCTCCTCA |
| | | | SEQ ID NO: 27827 | SEQ ID NO: 31833 |
| | | AA | DIQMTQSPSSVYASVGDRVTITCRASQGISSYLAWYQQKPGKAPKLLIYAASSLQSGVPSRFSGSGSGTDFTLTISSLQPEDFATYYCQQANSFPLTFGGGTKVEIK | EVQMLESGGGLGQSGGSLRLSCTASGFTFTTSAMSWVRQAPGKGLEWVSAISGSGANTFYADSVKGRFTVTRDNSKNTLYLQMNSLTAEDTAVYYCVKVIAVAGGHFFDPWGQGTLVTVSS |
| | | | SEQ ID NO: 27828 | SEQ ID NO: 31834 |
| **iPS:437234** | **21-225_64E2** | NA | GACATCCAGATGACCCAGTCTCCATCTTCCGTGTCTGCATCTGTAGGAGACAGAGTCACCATCACTTGTCGGGCGAGTCAGGGTATTAGCAGGTGGTTAGCCTGGTATCAGCAGAAACCAGGGAAAGCCCCTAAGCTCCTGATCTATGCTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGGCTAACAGTTTCCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAACAATAATGGCACAAACTATGCACAGAAGTTTCAGGGCAGGGTCACCATGACCAGGGACACGTCCATCAGCACAGCCTACATGGAGCTGAGCAGGCTGAGATCTGACGACACGGCCGTGTATTACTGTGCGAGAGATGGGAGCAGTGGCTTTGACTACTGGGGCCAGGGAACCCTGGTCACCGTCTCCTCA |
| | | | SEQ ID NO: 27829 | SEQ ID NO: 31835 |

FIGURE 50

| | | | | |
|---|---|---|---|---|
| | | AA | DIQMTQSPSSVSASVGDRVTITCRASQGISRWLAWYQQKPGKAPKLLIYAASSLQSGVPSRFSGSGSGTDFTLTISSLQPEDFATYYCQQANSFPFTFGPGTKVDIK | QVQLVQSGAEVKKPGASVKVSCKASGYTFTGYYMHWVRQAPGQGLEWMGWINPNNNGTNYAQKFQGRVTMTRDTSISTAYMELSRLRSDDTAVYYCARDGSSGFDYWGQGTLVTVSS |
| | | | SEQ ID NO: 27830 | SEQ ID NO: 31836 |
| iPS:437248 | 21-225_97H3 | NA | GATATTGTGATGATTCAGTCTCCACTCTCCCTGCCCGTCACCCCTGGAGAGCCGGCCTCCATCTCCTGCAGGTCTAGTCAGAGCCTCCTGCATAGTAATGGACACAACTATTTGGATTGGTACCTACAGAAGCCAGGGCGGTCTCCACAGCTCTTGATCTATTTGGGTTCTAATCGGGCCTCCGGGGTCCCTGAGAGGTTCAGTGGCAGTGGATCAGGCACAGATTTTACACTGAAAATCAGCAGAGTGGAGGCTGAGGATGTTGGGGTTTATTACTGCATGCAACCTCTACAAACTCCGTTCACTTTCGGCGGAGGGACCAAGGTGGAGATCAAA | CAGGTGCAGGTACAGCAGTGGGGCGCAGGACTGTTGAAGCCTTCGGAGACCCTGTCCCTCACCTGCGCTGTCTATGGTGGGTCCTTCACTGATTACTACTGGAGCTGGATCCGCCAGCCCCCAGGGAAGGGGCTGGAGTGGATTGGGGAAATCAATCATAGTGGAGACACCAACTACAACCCGTCCCTCAAGAGTCGAGTCACCATATCAGTAGACACGTCCAAGAACCAGTTCTCCCTGAAGCTGAGTTCTGTGACCGCCGCGGACACGGCTGTGTATTACTGTGCGAGAGAGTTTCCATATAGTGGAAGCTACCTCTACTACTACGGTATGGACGTCTGGGGCCAAGGGACCACGGTCACCGTCTCCTCA |
| | | | SEQ ID NO: 27831 | SEQ ID NO: 31837 |
| | | AA | DIVMIQSPLSLPVTPGEPASISCRSSQSLLHSNGHNYLDWYLQKPGRSPQLLIYLGSNRASGVPERFSGSGSGTDFTLKISRVEAEDVGVYYCMQPLQTPFTFGGGTKVEIK | QVQVQQWGAGLLKPSETLSLTCAVYGGSFTDYYWSWIRQPPGKGLEWIGEINHSGDTNYNPSLKSRVTISVDTSKNQFSLKLSSVTAADTAVYYCAREFPYSGSYLYYYGMDVWGQGTTVTVSS |
| | | | SEQ ID NO: 27832 | SEQ ID NO: 31838 |

FIGURE 50

| | | | | |
|---|---|---|---|---|
| **iPS:437250** | **21-225_148C6** | NA | GATGTTGTGATGACTCAGTCTCCACTCTCCCTG<br>CCCGTCACCCTTGGACAGCCGGCCTCCATCTC<br>CTGCAGGTCTAGTCAAAGCCTCGTATACAGTG<br>ATGGAAACACCTACTTGAATTGGTTTCAGCAG<br>AGGCCAGGCCAATCTCCAAGGCGCCTAATTTA<br>TAAGGTTTCTAACTGGGACTCTGGGGTCCCAG<br>ACAGATTCAGCGGCAGTGGGTCAGGCACTGAT<br>TTCACACTGAAAATCAGCAGGGTGGAGGCTGA<br>GGATGTTGGGGTTTATTACTGCATGCAAGGTA<br>CACACTGGTCGCTCACTTTCGGCGGAGGGACC<br>AAGGTGGAGATCAAA | GAGGTGCAACTGTTGGAGTCTGGGGGAGGCTTG<br>GTACAGCCTGGGGGGTCCCTGAGACTCTCCTGTG<br>CAGCCTCTGGATTCACCTTTAGCAGCTATGCCAT<br>GAGCTGGGTCCGCCAGGCTCCAGGGAAGGGGCT<br>GGAGTGGGTCTCAGTTATTAGTGGTGGTGGTAGT<br>AGTACATACTACGCAGACTCCGTGAAGGGCCGG<br>TTCACCATCTCCAGAGACAATTCCAAGAACACGC<br>TGTATCTGCAAATGAACAGCCTGAGAGCCGAGG<br>ACACGGCCGTATATTACTGTGCGAAATGGCGAG<br>GTAACCCCACTGACTACGGTATGGACGTCTGGGG<br>CCAAGGGACCACGGTCACCGTCTCCTCA |
| | | | SEQ ID NO: 27833 | SEQ ID NO: 31839 |
| | | AA | DVVMTQSPLSLPVTLGQPASISCRSSQSLVYSDG<br>NTYLNWFQQRPGQSPRRLIYKVSNWDSGVPDRF<br>SGSGSGTDFTLKISRVEAEDVGVYYCMQGTHWS<br>LTFGGGTKVEIK | EVQLLESGGGLVQPGGSLRLSCAASGFTFSSYAMS<br>WVRQAPGKGLEWVSVISGGGSSTYYADSVKGRFTI<br>SRDNSKNTLYLQMNSLRAEDTAVYYCAKWRGNPT<br>DYGMDVWGQGTTVTVSS |
| | | | SEQ ID NO: 27834 | SEQ ID NO: 31840 |
| **iPS:437252** | **21-225_148H11** | NA | GATGTTGTGATGACTCAGTCTCCACTCTCCCTG<br>CCCGTCACCCTTGGACAGCCGGCCTCCATCTC<br>CTGCAGGTCTAGTCAAAGCCTCGTATACAGTG<br>ATGGAAACACCTACTTGAATTGGTTTCAGCAG<br>AGGCCAGGCCAATCTCCAAGGCGCCTAATTTA<br>TAAGGTTTCTAACTGGGACTCTGGGGTCCCAG<br>ACAGATTCAGCGGCAGTGGGTCAGGCACTGAT<br>TTCACACTGAAAATCAGCAGGGTGGAGGCTGA<br>GGATGTTGGGGTTTATTACTGCATGCAAGGTA<br>CACACTGGTTGCTCACTTTCGGCGGAGGGACC<br>AAGGTGGAGATCAAA | GAGGTGCAACTGTTGGAGTCTGGGGGAGGCTTG<br>GTACAGCCTGGGGGGTCCCTGAGACTCTCCTGTG<br>CAGCCTCTGGATTCACCTTTAGCAGCTATGCCAT<br>GAGCTGGGTCCGCCAGGCTCCAGGGAAGGGGCT<br>GGAGTGGGTCTCAGTTATTAGTGGTGGTGGTAGT<br>AGCACATACTACGCAGACTCCGTGAAGGGCCGG<br>TTCACCATCTCCAGAGACAATTCCAAGAACACGC<br>TGTATCTGCAAATGAACAGCCTGAGAGCCGAGG<br>ACACGGCCGTATATTACTGTGCGAAATGGCGAG<br>GTAACCCCACTGACTACGGTATGGACGTCTGGGG<br>CCAAGGGACCACGGTCACCGTCTCCTCA |
| | | | SEQ ID NO: 27835 | SEQ ID NO: 31841 |

FIGURE 50

| | | | | |
|---|---|---|---|---|
| | | AA | DVVMTQSPLSLPVTLGQPASISCRSSQSLVYSDGNTYLNWFQQRPGQSPRRLIYKVSNWDSGVPDRFSGSGSGTDFTLKISRVEAEDVGVYYCMQGTHWLLTFGGGTKVEIK | EVQLLESGGGLVQPGGSLRLSCAASGFTFSSYAMSWVRQAPGKGLEWVSVISGGGSSTYYADSVKGRFTISRDNSKNTLYLQMNSLRAEDTAVYYCAKWRGNPTDYGMDVWGQGTTVTVSS |
| | | | SEQ ID NO: 27836 | SEQ ID NO: 31842 |
| iPS:437254 | 21-225_149F2 | NA | GATGTTGTGATGACTCAGTCTCCACTCTCCCTGCCCGTCACCCTTGGACAGCCGGCCTCCATCTCCTGCAGGTCTAGTCAAAGCCTCGTATACAGTGATGGAAACACCTCCTTGAATTGGTTTCAGCAGAGGCCAGGCCAATCTCCAAGGCGCCTAATTTATAAGGTTTCTAACTGGGACTCTGGGGTCCCAGTCAGATTCAGCGGCAGTGGGTCAGGCACTGATTTCACACTGAAAATCAGCAGGGTGGAGGCTGAGGATGTTGGGATTTATTACTGCATGCAAGGTACACACTGGCCTCCCACTTTCGGCGGAGGGACCAAGGTGGAGATCAAA | CAGGTGCAGCTGGGGGAGGCTGGGGGAGGCGTGGTCCAGCCTGGGAGGTCCCTGAGACTCTCCTGTGCAGCGTCTGGATTCACCTTCAGTCGCTATGGCATGCACTGGGTCCGCCAGGCTCCAGGCAAGGGGCTGGAGTGGGTGGCTTTTATATGGTATGATGGAAGTGAGAACTACTATGCAGACTCCGTGAAGGGCCGATTCACCATCTCCAGAGTCAATTCCAGGAACACGCTGTATCTGCAAATGAACAGCCTGAGAGCCGAGGACACGGCTGTGTATTACTGTGCGAGAGATCGGGTGGAGGGTTCGGGGACTCCCTACTACTACTACGGTATGGACGTCTGGGGCCAAGGGACCACGGTCACCGTCTCCTCA |
| | | | SEQ ID NO: 27837 | SEQ ID NO: 31843 |
| | | AA | DVVMTQSPLSLPVTLGQPASISCRSSQSLVYSDGNTSLNWFQQRPGQSPRRLIYKVSNWDSGVPVRFSGSGSGTDFTLKISRVEAEDVGIYYCMQGTHWPPTFGGGTKVEIK | QVQLGEAGGGVVQPGRSLRLSCAASGFTFSRYGMHWVRQAPGKGLEWVAFIWYDGSENYYADSVKGRFTISRVNSRNTLYLQMNSLRAEDTAVYYCARDRVEGSGTPYYYYGMDVWGQGTTVTVSS |
| | | | SEQ ID NO: 27838 | SEQ ID NO: 31844 |

FIGURE 50

| | | | | |
|---|---|---|---|---|
| **iPS:437256** | **21-225_150F11** | NA | GATGTTGTGATGAGTCAGTATCCACTCTCCCTGCCCGTCACCTTTGGACAGCCGGCCTCCATCTCATGCAGGTCTAGTCAAAGCCTCGTATACAGTGATGGAAACACCTCCTTGAATTGGTTTCAGCAGAGGCCAGGCCAATATCCAAGGCGCTTAATTTATAAGGTTTCTAACTGGGACTATGGGGTCCCAGTCAGATTCAGCGGCAGTGGGTCAGGCACTGATTTCACACTGAAAATCAGCAGGGTGGAGGCTGAGGATGTTGGGATTTATTACTGCATGCAAGGTACACACTGGCCTCCCACTTTCGGCGGAGGGACCAAGGTGGAGATCAAA | CAGGTGCAGCTGGGGGAGGCTGGGGGAGGCGTGGTCCAGCCTGGGAGGTCCCTGAGACTCTCCTGTGCAGCGTCTGGATTCACCTTCAGTCGCTATGGCATGCACTGGGTCCGCCAGGCTCCAGGCAAGGGGCTGGAGTGGGTGGCTTTTATATGGTATGATGGAAGTGAGAACTACTATGCAGACTCCGTGAAGGGCCGATTCACCATCTCCAGAGTCAATTCCAGGAACACGCTGTATCTGCAAATGAACAGCCTGAGAGCCGAGGACACGGCTGTGTATTACTGTGCGAGAGATCGGGTGGAGGGTTCGGGGACTCCCTACTACTACTACGGTATGGACGTCTGGGGCCAAGGGACCACGGTCACCGTCTCCTCA |
| | | | SEQ ID NO: 27839 | SEQ ID NO: 31845 |
| | | AA | DVVMSQYPLSLPVTFGQPASISCRSSQSLVYSDGNTSLNWFQQRPGQYPRRLIYKVSNWDYGVPVRFSGSGSGTDFTLKISRVEAEDVGIYYCMQGTHWPPTFGGGTKVEIK | QVQLGEAGGGVVQPGRSLRLSCAASGFTFSRYGMHWVRQAPGKGLEWVAFIWYDGSENYYADSVKGRFTISRVNSRNTLYLQMNSLRAEDTAVYYCARDRVEGSGTPYYYYGMDVWGQGTTVTVSS |
| | | | SEQ ID NO: 27840 | SEQ ID NO: 31846 |
| **iPS:437258** | **21-225_153F9** | NA | GACATCCAGATGACCCAGTCTCCATCCTCACTGTCTGCATCTGTAGGAGACAGAGTCACCATCACTTGTCGGGCGAGTCAGGGCATTAGCAATTATTTAGCCTGGTTTCAGCAGAAGCCAGGGAAAGCCCCTAAGTCCCTGATCTCTGCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAGTATAATAGTTACCCGCTCAGTTTCGGCGGAGGGACCAAGGTGGAGATCAAA | CAGGTACACCTGGTGGAGTCTGGGGGAGGCGTGGTCCAGCCTGGGAGGTCCCTGAGACTCTCCTGTGCAGCGTCTGGATTCACCATCAGTACCTATGGCATGCACTGGGTCCGCCAGGGTCCAGGCAAGGGGCTGGAGTGGGTGGCAGTTATATGGTATGGTGGAAGTGATACAGACTATGCAGACTCCGTGAGGGGCCGATTCACCATCTCCAGAGACATTTCCAAGAACACGCTGTATCTGCAAATGAACAGCCTGAGAGCCGAGGACACGGCTGTGTATTACTGTGCGAGAGATCGGGATTATTGTAGTGGTGGTAACTGCCCTTACTACTACTACTACGGTATGGACGTCTGGGGCCAAGGGACCACGGTCACCGTCTCCTCA |

FIGURE 50

| | | | | |
|---|---|---|---|---|
| | | | SEQ ID NO: 27841 | SEQ ID NO: 31847 |
| | | AA | DIQMTQSPSSLSASVGDRVTITCRASQGISNYLA WFQQKPGKAPKSLISAASSLQSGVPSKFSGSGSG TDFTLTISSLQPEDFATYYCQQYNSYPLSFGGGT KVEIK | QVHLVESGGGVVQPGRSLRLSCAASGFTISTYGMH WVRQGPGKGLEWVAVIWYGGSDTDYADSVRGRF TISRDISKNTLYLQMNSLRAEDTAVYYCARDRDYC SGGNCPYYYYYGMDVWGQGTTVTVSS |
| | | | SEQ ID NO: 27842 | SEQ ID NO: 31848 |
| **iPS:437260** | **21-225_170D1** | NA | GACATCCAGATGACCCAGTCTCCATCCTCACT GGCTGCATCTGTAGGAGACAGAGTCACCATCA CTTGTCGGGCGAGTCAGGACATTAGCAATTAT TTAGCCTGGTTTCAGCAGAAACCAGGGAAAGC CCCTAAGTCCCTGATCTATGCTGCATCCAGTTT GCAAAGTGGGGTCCCATCAAGGTTCAGCGGCA GTGGATCTGGGACAGATTTCACTCTCACCATC AGCAGCCTGCAGCCTGAAGATTTTGCAACTTA TTACTGCCAACAGTGTGATAGTTTCCCTCTCAC TTTCGGCGGAGGGACCAAGGTGGAGATCAAA | CAGGTGCAGTTGGTGCAGTCTGGGGCTGAGGTGA AGAAGCCTGGGGCCTCAGTGAAGGTCTCCTGCA AGGCTTCTGGATACACCTTCACCGGCTACTTTAT GCACTGGGTGCGACAGGCCCCTGGACAAGGGCT TGAGTGGATGGGATGGATCAAGCCTAAAAGCGG TGGCACAAACTGTGCACAGAAGTTTCAGGGCAG GGTCACCATGACCAGGGACACGTCCAGCAGCAC AGCCTACATGGAGCTGAGCAGGCTGACATCTGA CGACACGGCCGTGTATTACTGTGCGAGAGGGGG GGCTACGGTGACTACGTGGGGGGTCTTTGACTAC TGGGGCCAGGGAACCCTGGTCACCGTCTCCTCA |
| | | | SEQ ID NO: 27843 | SEQ ID NO: 31849 |
| | | AA | DIQMTQSPSSLAASVGDRVTITCRASQDISNYLA WFQQKPGKAPKSLIYAASSLQSGVPSRFSGSGSG TDFTLTISSLQPEDFATYYCQQCDSFPLTFGGGT KVEIK | QVQLVQSGAEVKKPGASVKVSCKASGYTFTGYFM HWVRQAPGQGLEWMGWIKPKSGGTNCAQKFQGR VTMTRDTSSSTAYMELSRLTSDDTAVYYCARGGA TVTTWGVFDYWGQGTLVTVSS |
| | | | SEQ ID NO: 27844 | SEQ ID NO: 31850 |

FIGURE 50

| | | | | |
|---|---|---|---|---|
| **iPS:437262** | **21-225_170E4** | NA | GACATCCAGATGACCCAGTCTCCATCCTCCCT<br>GTCTGCATCTGTAGGAGACAGAGTCACCATCA<br>CTTGCCGGGCAAGTCAGGGCATTAGAAATGAT<br>TTAGGCTATTATCAGCAGAAACCAGGGAAAGC<br>CCCTAAGCGCCTGATCTATGTTGCATCCGGTTT<br>GCAAAGTGGGGTCCCATCAAGGTTCAGCGGCA<br>GTGGATCTGGGACAGAATTCACTCTCACAATC<br>AGCAGCCTGCAGCCTGAAGATTTTGCAACTTA<br>TTACTGTCTACAGCACAATAGTTACCCTCCGT<br>GGACGTTCGGCCAAGGGACCAAGGTGGATAT<br>CAAA | GAGGTGCAACTGGTGGAGTCTGGGGGAGGCTCG<br>GTACAGCCTGGGGGGTCCCTGAGACTCTCCTGTG<br>CAGCCTCTGGATTCACCTTCAGTAGCTATAGCAT<br>GAACTGGGTCCGCCAGGCTCCAGGGAAGGGGCT<br>GGAGTGGGTTTCATACATTAGCAGTAGTGGTAGT<br>ACCAAATACTACGCAGACTCTGTGGAGGGCCGA<br>TTCACCATCTCCAGAGACAATGCCAAGAATTCAC<br>TGGATCTGCAAATGAACAGCCTGAGAGACGAGG<br>ACACGGCTGTGTATCGCTGTGCGAGAGATAGTAG<br>GAAGGGGTTCTACTACGGTCTGGACGTCTGGGGC<br>CAAGGGACCACGGTCACCGTCTCCTCA |
| | | | SEQ ID NO: 27845 | SEQ ID NO: 31851 |
| | | AA | DIQMTQSPSSLSASVGDRVTITCRASQGIRNDLG<br>YYQQKPGKAPKRLIYVASGLQSGVPSRFSGSGS<br>GTEFTLTISSLQPEDFATYYCLQHNSYPPWTFGQ<br>GTKVDIK | EVQLVESGGGSVQPGGSLRLSCAASGFTFSSYSMN<br>WVRQAPGKGLEWVSYISSSGSTKYYADSVEGRFTI<br>SRDNAKNSLDLQMNSLRDEDTAVYRCARDSRKGF<br>YYGLDVWGQGTTVTVSS |
| | | | SEQ ID NO: 27846 | SEQ ID NO: 31852 |
| **iPS:437264** | **21-225_171H12** | NA | GACATCCAGATGACCCAGTCTCCATCCTCACT<br>GTCTGCATCTGTAGGAGACAGAGTCACCATCA<br>CTTGTCGGGCGAGTCAGGACATTAGCAATTAT<br>TTAGCCTGGTTTCAGCAGAAGCCAGGGAAAGC<br>CCCTAAGTCCCTGATCTATTCTGCATCCAGTTT<br>GCAAAGTGGGGTCCCATCAAAGTTCAGCGGCA<br>GTGGATCTGGGACAGATTTCACTCTCACCATC<br>AGCAGCCTGCAGCCTGAAGATTTTGCAACTTA<br>TTACTGCCAACAATCTGATAGTTACCCTCTCAC<br>TTTCGGCGGAGGGACCAAGGTGGAGATCAAA | CAGGTGCAGCTGGTGCAGTCTGGGGCTGAGGTG<br>AAGAAGCCTGGGGCCTCAGTGAGGGTCTCCTGC<br>AAGGCTTCTGGATACACCTTCACCGGCTACTTTA<br>TGCACTGGGTGCGACAGGCCCCTGGACAAGGGC<br>TTGAGTGGATGGGATGGATCAAGCCTAAGAGTG<br>GTGGCACAAACTCTGCACAGAGGTTTCAGGGCA<br>GGGTCACCATGACCAGGGACACGTCCATCAACA<br>CAGCCTACATGGAGCTGAACAGGCTGAGATCTG<br>ACGACACGGCCGTATATTACTGTGCGAGAGGGG<br>GGACTACGGTGGCTACGTGGGGGTCTTTGACTA<br>CTGGGGCCAGGGAACCCTGGTCACCGTCTCCTCA |
| | | | SEQ ID NO: 27847 | SEQ ID NO: 31853 |

FIGURE 50

| | | | | |
|---|---|---|---|---|
| | | AA | DIQMTQSPSSLSASVGDRVTITCRASQDISNYLAWFQQKPGKAPKSLIYSASSLQSGVPSKFSGSGSGTDFTLTISSLQPEDFATYYCQQSDSYPLTFGGGTKVEIK | QVQLVQSGAEVKKPGASVRVSCKASGYTFTGYFMHWVRQAPGQGLEWMGWIKPKSGGTNSAQRFQGRVTMTRDTSINTAYMELNRLRSDDTAVYYCARGGTTVATWGVFDYWGQGTLVTVSS |
| | | | SEQ ID NO: 27848 | SEQ ID NO: 31854 |
| **iPS:437266** | **21-225_177A5** | NA | GACATCCAGATGACCCAGTCTCCATCCTCACTGTCTGCATCTGTAGGAGACAGAGTCACCATCACTTGTCGGGCGAGTCAGGACATTAGCAATTATTTAGCCTGGTTTCAGCAGAAGCCAGGGAAAGCCCCTAAGTCCCTGATCTATTCTGCATCCAGTTTGCAAAGTGGGGTCCCATCAAAGTTCAGCGGCAGTGGATCTGGGACAGATTTCACTCTCACCATCAGCAGCCTGCAGCCTGAAGATTTTGCAACTTATTACTGCCAACAATCTGATAGTTACCCTCTCACTTTCGGCGGAGGGACCAAGGTGGAGATCAAA | CAGGTGCAGCTGGTACAGTCTGGGGCTGAGGTGAAGAAGCCTGGGGCCTCAGTGCGGGTCTCCTGCAAGGCTTCTGGATACACCTTCACCGGCTACTTTATGCACTGGGTGCGACAGGCCCCTGGACAAGGGCTTGAGTGGATGGGATGGATCAAGCCTAAGAGTGGTGGCACAAACTCTGCACAGAGGTTTCAGGGCAGGGTCACCATGACCAGGGACACGTCCATCAACACAGCCTACATGGAGCTGAACTGGCTGAGATCTGACGACACGGCCGTATATTACTGTGCGAGAGGGGGGACTACGGTGGCTACGTGGGGGGTCTTTGACTACTGGGGCCAGGGAACCCTGGTCACCGTCTCCTCA |
| | | | SEQ ID NO: 27849 | SEQ ID NO: 31855 |
| | | AA | DIQMTQSPSSLSASVGDRVTITCRASQDISNYLAWFQQKPGKAPKSLIYSASSLQSGVPSKFSGSGSGTDFTLTISSLQPEDFATYYCQQSDSYPLTFGGGTKVEIK | QVQLVQSGAEVKKPGASVRVSCKASGYTFTGYFMHWVRQAPGQGLEWMGWIKPKSGGTNSAQRFQGRVTMTRDTSINTAYMELNWLRSDDTAVYYCARGGTTVATWGVFDYWGQGTLVTVSS |
| | | | SEQ ID NO: 27850 | SEQ ID NO: 31856 |

FIGURE 50

| | | | | |
|---|---|---|---|---|
| **iPS:437268** | **21-225_177D2** | NA | GATGTTGTGATGACTCAGTCTCCACTCTCCCTGCCCGTCACCCTTGGACAGCCGGCCTCCATCTCCTGCAGGTCTAGTCAAAGCCTCGTATACAGTGATGGAAACACCTACTTGAATTGGTTTCAGCAGAGGCCAGGCCAATCTCCAAGGCGCCTAATTTATAAGGTTTCTAACTGGGACTCTGGGGTCCCAGACAGATTCAGCGGCAGTGGGTCAGGCACTGATTTCACACTGAAAATCAGCAGGGTGGAGGCTGAGGATGTTGGGGTTTATTACTGCATGCAAGGTACACACTGGCCTCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGGACTGGGTCCGCCAGGCTCCAGGCAAGGGGCTGGAGTGGGTGGCAATTATATGGTTTGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGCATATTGTGGTGGTGACTGCTATTTCCCCCATCTCCATTACTACGGTATGGACGTCTGGGGCCAAGGGACCACGGTCACCGTCTCCTCA |
| | | | SEQ ID NO: 27851 | SEQ ID NO: 31857 |
| | | AA | DVVMTQSPLSLPVTLGQPASISCRSSQSLVYSDGNTYLNWFQQRPGQSPRRLIYKVSNWDSGVPDRFSGSGSGTDFTLKISRVEAEDVGVYYCMQGTHWPLTFGGGTKVEIK | QVQLVESGGGVVQPGRSLRLSCAASGFTFSSYGMDWVRQAPGKGLEWVAIIWFDGSNKYYADSVKGRFTISRDNSKNTLYLQMNSLRAEDTAVYYCARAYCGGDCYFPHLHYYGMDVWGQGTTVTVSS |
| | | | SEQ ID NO: 27852 | SEQ ID NO: 31858 |
| **iPS:437270** | **21-225_178H4** | NA | GACATCCAGATGACCCAGTCTCCATCCTCACTGTCTGCATCTGTAGGAGACAGAGTCACCATCACTTGTCGGGCGAGTCAGGACATTAGCAATTATTTAGCCTGGTTTCAGCAGAAACCAGGGAAAGCCCCTAAGTCCCTGATCTTTTCTGCATCCAGTTTGCAAAGTGGGGTCCCATCAAAGTTCAGCGGCAGTGGATCTGGGACAGATTTCACTCTCACCATCAGCAACCTGCAGCCTGAAGATTTTGCAACTTATTACTGCCAACAATCTAATAGTTACCCTCTCACTTTCGGCGGAGGGACCAAGGTGGAGATCAAA | CAGGTACAGCTGGTGCAGTCTGGGGCTGAGGTGAAGAAGCCTGGGGCCTCAGTGAAGGTCTCCTGCAAGGCTTCTGGATACACCTTCACCGGCTACTTTATGCACTGGGTGCGACAGGCCCCTGGACAAGGGCTTGAGTGGATGGGATGGATCAAGCCTAAAAGTGGTGGCACAAACTGTGCACAGAAGTTTCAGGGCAGGGTCACCATGACCAGGGACACGTCCATCAGCACAGCCTACATGGAACTGAGCAGGCTGAGATCTGACGACACGGCCGTGTATTATTGTGTGAGAGGGGGGACTACGGTGACTACGTGGGGGGTCTTTGACTACTGGGGCCAGGGAACCATGGTCACCGTCTCCTCA |

FIGURE 50

| | | | | |
|---|---|---|---|---|
| | | | SEQ ID NO: 27853 | SEQ ID NO: 31859 |
| | | AA | DIQMTQSPSSLSASVGDRVTITCRASQDISNYLA WFQQKPGKAPKSLIFSASSLQSGVPSKFSGSGSG TDFTLTISNLQPEDFATYYCQQSNSYPLTFGGGT KVEIK | QVQLVQSGAEVKKPGASVKVSCKASGYTFTGYFM HWVRQAPGQGLEWMGWIKPKSGGTNCAQKFQGR VTMTRDTSISTAYMELSRLRSDDTAVYYCVRGGTT VTTWGVFDYWGQGTMVTVSS |
| | | | SEQ ID NO: 27854 | SEQ ID NO: 31860 |
| **iPS:437274** | **21-225_196D4** | NA | GACATCCAGATGACCCAGTCTCCATCCTCACT GTCTGCATCTGTAGGAGACAGAGTCACCATCA CTTGTCGGGCGAGTCAGGGCATTAGCAATTAT TTAGCCTGGTTTCAGCAGAACCCAGGGAAAGC CCCTAAGTCCCTTATCTATGCTGCATCCAGTTT GCAAAGTGGGGTCCCATCAAAGTTCAGCGGCA GTGGATGTGGGACAGATTTCACTCTCACCATC AGCAGCCCGCAGCCTGAAGATGTTGCAACCTA TTACTGCCAACATTATCTTAATTACCCTCTCAC CTTCGGCCAAGGGACACGACTGGAGATTAAA | CAGGTGCAGGTGGTGGAGTCTGGGGGAGGCGTG GTCCAGCCTGGGAGGTCCCTGAGACTCTCCTGTG CAGCGTCTGGATTCACCTTCAGTAGCTATGGCAT GCACTGGGTCCGCCAGGCTCCAGGCAAGGGGCT GGAGTGGGTGGCAGTTATATGGTATGATGGAAGT AATAGAAACTATGCAGACTCCGTGAAGGGCCGA TTCACCATCTCCAGAGACAATTCCAAGAACACGC TGTATCTGCAAATGAACAGCCTGAGAGTCGAGG ACACGGCTGTGTATTACTGTGCGAGAGATCGGTC TAAGGGTTACGACGGTATGGACGTCTGGGGCCA AGGGACCACGGTCACCGTCTCCTCA |
| | | | SEQ ID NO: 27855 | SEQ ID NO: 31861 |
| | | AA | DIQMTQSPSSLSASVGDRVTITCRASQGISNYLA WFQQNPGKAPKSLIYAASSLQSGVPSKFSGSGCG TDFTLTISSPQPEDVATYYCQHYLNYPLTFGQGT RLEIK | QVQVVESGGGVVQPGRSLRLSCAASGFTFSSYGMH WVRQAPGKGLEWVAVIWYDGSNRNYADSVKGRF TISRDNSKNTLYLQMNSLRVEDTAVYYCARDRSKG YDGMDVWGQGTTVTVSS |
| | | | SEQ ID NO: 27856 | SEQ ID NO: 31862 |

FIGURE 50

| | | | | |
|---|---|---|---|---|
| **iPS:437280** | **21-225_203C10** | NA | GACATCCAGATGACCCAGTCTCCATCCTCCCTGTCTGCATCTGTAGGAGACAGAGTCACCATCACTTGCCGGGCAAGTCAGGACATTAGAAATGATTTAGGCTGGTATCAGCAGAAACCAGCGAAAGCCCCTAAGCGCCTGATCTATAGAGCATCCAGTTTGCAAAGTGGGGTCCCATCACGCTTCAGCGGCAGTGGATCTGGGACAGAATTCACTCTCACAATCAGCAGCCTGCAGCCTGAAGATTTTGCCGCTTATTACTGTCTACAGCATAATAGTTACCCGTGGACGTTCGGCCAAGGGACCAAGGTAGAGATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGGCATGCACTGGGTCCGCCAGGCTCCAGGCAAGGGGCTGGAGTGGGTGGCAGTTATATGGTATGATGGAGGTAATACACATTATACAGACTCCGTGAAGGGCCGATTCACCATCTCCAGAGACAATTCCAAGAACACGCTGTATCTGCAAATGAACAGCCTGAGAGCCGAGGACACGGCTGTATATTACTGTGCGAGAGAAGTGGGTTGGCTTGATGACTACTGGGGCCAGGGAACCCTGGTCACCGTCTCCTCA |
| | | | SEQ ID NO: 27857 | SEQ ID NO: 31863 |
| | | AA | DIQMTQSPSSLSASVGDRVTITCRASQDIRNDLGWYQQKPAKAPKRLIYRASSLQSGVPSRFSGSGSGTEFTLTISSLQPEDFAAYYCLQHNSYPWTFGQGTKVEIK | QVQLVESGGGVVQPGRSLRLSCAASGFTFSDYGMHWVRQAPGKGLEWVAVIWYDGGNTHYTDSVKGRFTISRDNSKNTLYLQMNSLRAEDTAVYYCAREVGWLDDYWGQGTLVTVSS |
| | | | SEQ ID NO: 27858 | SEQ ID NO: 31864 |
| **iPS:437282** | **21-225_207C9** | NA | GACAACCAGATGACCCAGTCTCCATCCTCCCTGTCTGCATCTGTAGGAGACAGAGTCACCATCACTTGCCGGGCAAGTCAGAGGTTTAGTAACTATTTAAATTGGTATCAGCAGAAACCAGGGAAAGCCCCTAAGCTCCTGATCTATACTGCATCCAGTTTGCAAAGTGGGGTCCCATCAAGGTTCAGTGCCAGTGTCTCTGGGACAGACTTCACTCTCACCATCAGCAGTCTGCAACCTGAAGATTTTGCAACTTATTACTGTCAACAGAGTTACAGTATTCCGCTCACTTTCGGCGGAGGGACCAAGGTGGAGATCAAA | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTAGTGGTGGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAAGCTGGTGGAACTACGGGGAGCTACTACTACAACGGTATGGACGTCTGGGGCCAAGGGACCACGGTCACCGTCTCCTCA |
| | | | SEQ ID NO: 27859 | SEQ ID NO: 31865 |

FIGURE 50

| | | | | |
|---|---|---|---|---|
| | | AA | DNQMTQSPSSLSASVGDRVTITCRASQRFSNYLN WYQQKPGKAPKLLIYTASSLQSGVPSRFSASVSG TDFTLTISSLQPEDFATYYCQQSYSIPLTFGGGTK VEIK | EVQLLESGGGLVQPGGSLRLSCAASGFTFSSYAMS WVRQAPGKGLEWVSAISGSGGSTYYADSVKGRFTI SRDNSKNTLYLQMNSLRAEDTAVYYCAKAGGTTG SYYYNGMDVWGQGTTVTVSS |
| | | | SEQ ID NO: 27860 | SEQ ID NO: 31866 |
| **iPS:437286** | **21-225_208F1** | NA | GACATCCAGATGACCCAGTCTCCATCCTCCCT GTCTGCATCTGTAGGAGACAGAGTCACCATCA CTTGCCGGGCAAGTCAGGGCATTAGACATGAT TTAGGCTGGTATCAGCAGAAACCAGGGAAAG CCCCTAAGCGCCTGATCTTTGCTGCATCCAGTT TGCAAAGTGGGGTCCCATCTAGATTCAGCGGC AGTGGATCTGGGACAGAATTCACTCTCACAAT CAGCAGCCTGCAGCCTGAAGATTTTGCAACTT ATTACTGTCTACAGCATTATAGTTTCCCTCGGA CGTTCGGCCAAGGGACCAAGGTGGAAATCAA A | CAGGTGCAGCTGGTGGAGTCTGGGGGAGGCGTG GTCCAGCCTGGGAGGTCCCTGAGACTCTCCTGTG CAGCGTCTAGATTCACCTTCAGTGACTATGTCAT GCACTGGGTCCGCCAGGCTCCAGGCAAGGGGCT GGAGTGGGTGGCAGTTATATGGTATGATGGAAGT AATAAATACTATGTAGACTCCGTGAAGGGCCGAT TCACCATCTCCAGAGACAATTCCAGGAACACGCT GTATCTGCAAATGAACAGCCTGAGAGCCGAGGA CACGGCTGTATATTACTGTGCGAGAGAACGGTAT AGCAGTGGCTTGTACGACTACGGTATGGACGTCT GGGGCCAAGGGACCACGGTCACCGTCTCCTCA |
| | | | SEQ ID NO: 27861 | SEQ ID NO: 31867 |
| | | AA | DIQMTQSPSSLSASVGDRVTITCRASQGIRHDLG WYQQKPGKAPKRLIFAASSLQSGVPSRFSGSGSG TEFTLTISSLQPEDFATYYCLQHYSFPRTFGQGTK VEIK | QVQLVESGGGVVQPGRSLRLSCAASRFTFSDYVMH WVRQAPGKGLEWVAVIWYDGSNKYYVDSVKGRF TISRDNSRNTLYLQMNSLRAEDTAVYYCARERYSS GLYDYGMDVWGQGTTVTVSS |
| | | | SEQ ID NO: 27862 | SEQ ID NO: 31868 |

FIGURE 50

| | | | | |
|---|---|---|---|---|
| **iPS:437290** | **21-225_210G6** | NA | GACATCCAGATGACCCAGTCTCCATCCTCCCGTTTTGCATTTGTAGGAGACAGAGTCACCATCACTTGCCGGGCAAGTCAGGGCATTAGACATGATTTAGGCTGGTATCAGCAGAAACCAGGGAAAGCCCTTAAGCGCTTGATATATGCTGCATCCAGTTCGCAAAGTGGGGTCCCATCAAGGTTCAGCGGCAGTGGATGTGGGACAGAATTCACTCTCACAATCAGCAGCGTGCAGCGCGAAGATTTTGCAAATTATTACTGTGTACAGCATTATAGTTTCCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTGACTATGTCATGCACTGGGTCCGCCAGGCTCCAGGCAAGGGGCTGGAGTGGGTGGCAGTTATATGGTATGATGGAAGTAATAAATACTATGTAGACTCCGTGAAGGGCCGATTCACCATCTCCAGAGACAATTCCAGGAACACGCTGTATCTGCAAATGAACAGCCTGAGAGCCGAGGACACGGCTGTGTATTACTGTGCGAGAGAACGGTATAGCAGTGGCTTGTACGACTACGGTATGGACGTCTGGGGCCAAGGGACCACGGTCACCGTCTCCTCA |
| | | | SEQ ID NO: 27863 | SEQ ID NO: 31869 |
| | | AA | DIQMTQSPSSRFAFVGDRVTITCRASQGIRHDLGWYQQKPGKALKRLIYAASSSQSGVPSRFSGSGCGTEFTLTISSVQREDFANYYCVQHYSFPRTFGQGTKVEIK | QVQLVESGGGVVQPGRSLRLSCAASGFTFSDYVMHWVRQAPGKGLEWVAVIWYDGSNKYYVDSVKGRFTISRDNSRNTLYLQMNSLRAEDTAVYYCARERYSSGLYDYGMDVWGQGTTVTVSS |
| | | | SEQ ID NO: 27864 | SEQ ID NO: 31870 |
| **iPS:437294** | **21-225_216D5** | NA | GATGTTGTGATGACTCAGTCTCCACTCTCCCTGCCCGTCACCCTTGGACAGCCGGCCTCCATCTCCTGCAGGTCTAGTCAAAGCCTCGTATACAGTGATGGAAACACCTACTTGAATTGGTTTCAGCAGAGGCCAGGCCAATCTCCAAGGCGCCTCATTTATAAGGTTTCTAACTGGGACTCTGGGGTCCCAGACAGAATCAGCGGCAGTGGGTCAGGCACTGATTTCACACTGAAAATCAGCAGGGTGGAGGCTGAGGATGTTGGGATTTATTACTGCATGCAAGGTGCACACTGGTTCACCTTCGGCCAAGGGACACGACTGGAGATTAAA | CAGGTGCAGCTGCAGGAGTCGGGCCCAGGACTGGTGAAGCCTTCACAGACCCTGTCCCTCACCTGCACTGTCTCTGGTGGCTCCATCAGCAGTGGTGGTTACTACTGGAGCTGGATCCGCCAGCACCCAGGGAAGGGCCTGGAGTGGATTGGGTACATCTATTACAGTGGGAGCACCTACTACAACCCGTCCCTCAAGAGTCGAGTTACCATATCAGTAGACACGTCTAAGAACCAATTCTCCCTGAAACTGAACTCTGTGACTGCCGCGGACACGGCCGTGTATTTCTGTGCGAGAGATTCCCCTGACAGGGGGTTTGACTACTGGGGCCAGGGAACCCTGGTCACCGTCTCCTCA |
| | | | SEQ ID NO: 27865 | SEQ ID NO: 31871 |

FIGURE 50

| | | | | |
|---|---|---|---|---|
| | | AA | DVVMTQSPLSLPVTLGQPASISCRSSQSLVYSDGNTYLNWFQQRPGQSPRRLIYKVSNWDSGVPDRISGSGSGTDFTLKISRVEAEDVGIYYCMQGAHWFTFGQGTRLEIK | QVQLQESGPGLVKPSQTLSLTCTVSGGSISSGGYYWSWIRQHPGKGLEWIGYIYYSGSTYYNPSLKSRVTISVDTSKNQFSLKLNSVTAADTAVYFCARDSPDRGFDYWGQGTLVTVSS |
| | | | SEQ ID NO: 27866 | SEQ ID NO: 31872 |
| iPS:437302 | 21-225_225B11 | NA | GACATCCAGATGACCCAGTCTCCATCCTCCCTGTCTGCATCTATAGGAGACAGAGTCACCATCACTTGCCAGGCGAGTCAGGACATTTTCAACTATTTAAATTGGTATCAGCAGAAACCAGGGAAAGCCCCTAAGCTCCTGATCTACGATGCATCCACTTTGGAAACAGGGGTCCCATCAAGGTTCAGTGGAAGTGGATCTGGGACAGATTTTACTTTCACCATCAGCAGCCTGCAGCCTGAAGATATTGCAACATATTACTGTCAACAGTATGATAATCTCCCGATCACCTTCGGCCAAGGGACACGACTGGAGATTAAA | CAGGTGCAGCTGGTGGAATCTGGGGGAGGCGTGGTCCAGCCTGGGAGGTCCCTGAGACTCTCCTGTGCAGCCTCTGGATTCACCTTCAGTAGCTATGGCATGCACTGGGTCCGCCAGGCTCCAGGCAAGGGGCTGGAGTGGGTGACAATTATATCATATAGTGGAAGTAATAAATACTATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTACTGTGCGAGACGGGGATACAGCTATGGCGGGTACGGTATGGACGTCTGGGGCCAAGGGACCACGGTCACCGTCTCCTCA |
| | | | SEQ ID NO: 27867 | SEQ ID NO: 31873 |
| | | AA | DIQMTQSPSSLSASIGDRVTITCQASQDIFNYLNWYQQKPGKAPKLLIYDASTLETGVPSRFSGSGSGTDFTFTISSLQPEDIATYYCQQYDNLPITFGQGTRLEIK | QVQLVESGGGVVQPGRSLRLSCAASGFTFSSYGMHWVRQAPGKGLEWVTIISYSGSNKYYADSVKGRFTISRDNSKNTLYLQMNSLRAEDTAVYYCARRGYSYGGYGMDVWGQGTTVTVSS |
| | | | SEQ ID NO: 27868 | SEQ ID NO: 31874 |

FIGURE 50

| | | | | |
|---|---|---|---|---|
| **iPS:437320** | **21-225_75A1** | NA | GATATTGTGATGACTCAGTCTCCACTCTCCCTGCCCGTCACCCCTGGAGAGCCGGCCTCCATCTCCTGCAGGTCTAGTCAGAGCCTCCTGCATAGTAATGGACACAACTATTTGGATTGGTACCTACAGAAGCCAGGGCGGTCTCCACAGCTCCTGATCTATTTGGGTTCTAATCGGGCCTCCGGGGTCCCTGAGAGGTTCAGTGGCAGTGGATCAGGCACAGATTTTACACTGAAAATCAGCAGAGTGGAGGCTGAGGATGTTGGGGTTTATTACTGCATGCAACCTCTACAAACTCCGTTCACTTTCGGCGGAGGGACCAAGGTGGAGATCAAA | CAGGTGCAGGTACAGCAGTGGGGCGCAGGACTGTTGAAGCATTCGGAGACCCTGTCCCTCACCTGCGCTGTCTATGGTGGGTCCTTCACTGATTACTACTGGAGCTGGATCCGCCAGCCCCCAGGGAAGGGGCTGGAGTGGATTGGGGAAATCAATCATAGTGGAGACACCAACTACAACCCGTCCCTCAAGAGTCGAGTCACCATATCAGTAGACACGTCCAAGAACCAGTTCTCCCTGAAGCTGAGTTCTGTGACCGCCGCGGACACGGCTGTGTATTACTGTGCGAGAGAGTTTCCATATAGTGGAAGCTACCTCTACTACTACGGTATGGACGTCTGGGGCCAAGGGACCACGGTCACCGTCTCCTCA |
| | | | SEQ ID NO: 27869 | SEQ ID NO: 31875 |
| | | AA | DIVMTQSPLSLPVTPGEPASISCRSSQSLLHSNGHNYLDWYLQKPGRSPQLLIYLGSNRASGVPERFSGSGSGTDFTLKISRVEAEDVGVYYCMQPLQTPFTFGGGTKVEIK | QVQVQQWGAGLLKHSETLSLTCAVYGGSFTDYYWSWIRQPPGKGLEWIGEINHSGDTNYNPSLKSRVTISVDTSKNQFSLKLSSVTAADTAVYYCAREFPYSGSYLYYYGMDVWGQGTTVTVSS |
| | | | SEQ ID NO: 27870 | SEQ ID NO: 31876 |
| **iPS:437322** | **21-225_75B1** | NA | GAATTTATGTTGACGCAGTCTCCAGGCACCCTGTATTGGTCTCCAGGGGAAAGAGCCACCATCTCGAGCAGGGCCAGTCAGAGTGTTAGCAGCAGCTACTTAGTCTGGTATCAGCAGAAACCTGGCCAGGCTCCCAGGCTCCTCATCTATGGTGCATCCACCAGGGCCACTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTTGCTCACCGCTCACTTTCGGCGGAGGGACCAAGGTGGAGATCACA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGTCCCTGGACAAGGGCTTGAGTGGATGGGATGGATGCACCCTAACAGTGGTAACACAGGCTATGCACAGAAGTTCCAGGGCAGAGTCACCATGACCAGGAACACCTCCATCAGCACAGCCTACATGGAGCTGAGCAGCCTGAGATCTGAGGACACGGCCGTGTATTACTGTGCGATTAGTAGTGGCTGGTACTGGTTCGACCCCTGGGGCCAGGGAACCCTGGTCACCGTCTCCTCA |
| | | | SEQ ID NO: 27871 | SEQ ID NO: 31877 |

FIGURE 50

| | | | | |
|---|---|---|---|---|
| | | AA | EFMLTQSPGTLYWSPGERATISSRASQSVSSSYLVWYQQKPGQAPRLLIYGASTRATGIPDRFSGSGSGTDFTLTISRLEPEYFAVYYCQQYGCSPLTFGGGTKVEIT | QVQLVQSGAEVKKPGASVKVSCKASGYTFTNYDINWVRQVPGQGLEWMGWMHPNSGNTGYAQKFQGRVTMTRNTSISTAYMELSSLRSEDTAVYYCAISSGWYWFDPWGQGTLVTVSS |
| | | | SEQ ID NO: 27872 | SEQ ID NO: 31878 |
| iPS:437324 | 21-225_75C2 | NA | GAAATTGTGTTGACGCAGTCTCCAGGCACCCGGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATCTATGGTGCATCCAGCCGGGCCACTGGCATCCCAGACAGGTTCAGTGGCAGTGGGTCTGGGACAGACTTCGCTCTCACCATCAGCAGACTGGAGCCTGAAGATTGTGCAGTGTATTACTGTCAGCACTATGATAACTCACCGTGGACGTTCGGCCGAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 27873 | SEQ ID NO: 31879 |
| | | AA | EIVLTQSPGTRYLSPGERATLSCRASQSVYSSYLAWYQQKPGQAPRLLIYGASSRATGIPDRFSGSGSGTDFALTISRLEPEDCAVYYCQHYDNSPWTFGRGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 27874 | SEQ ID NO: 31880 |

FIGURE 50

| | | | | |
|---|---|---|---|---|
| **iPS:437326** | **21-225_75C10** | NA | GACATCCAGATGACCCAGTCTCCGTCTTCCGTGTCTGCTTCTGTAGGAGACAGAGTCATCATCACTTGTCGGGCGAGTCAGGGCATTAGCATCTGGTTAGCCTGGTATCAGCAGAAACCAGGGAAAGCCCCTAAGCTCCTGATCTATGCTGCATCCAGTTTGCAAAGTGGGGTCCCATTAAGGTTCAGCGGCAGTGGATCTGGGACAGATTTCACTCTCACCATCAGCAGCCTGCAGCCTGAAGATTTTGCAACTTACTATTGTCAACAGGCTAAAAGTTTCCCGCTCACTTTCGGCGGAGGGACCAAGGTGGAGATCAAA | CAGGTGCAACTGGTGGAGTCTGGGGGAGGCGTGGTCCAGCCTGGGAGGTCCCTGAGACTCTCCTGTGCAGCGTCTGGATTCACCTTCAGTAGTTATGGCATGCATTGGGTCCGCCAGGCTCCAGGCAAGGGGCTGGAGTGGGTGGCAGTTATATGGTATGATGGAAGTGATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATCGGTTAGTGGGAGCTACGGTTGATGCTTTTGATATCTGGGGCCAAGGGACAATGGTCACCGTCTCTTCA |
| | | | SEQ ID NO: 27875 | SEQ ID NO: 31881 |
| | | AA | DIQMTQSPSSVSASVGDRVIITCRASQGISIWLAWYQQKPGKAPKLLIYAASSLQSGVPLRFSGSGSGTDFTLTISSLQPEDFATYYCQQAKSFPLTFGGGTKVEIK | QVQLVESGGGVVQPGRSLRLSCAASGFTFSSYGMHWVRQAPGKGLEWVAVIWYDGSDKYYADSVKGRFTISRDNSKNTLYLQMNSLRAEDTAVYYCARDRLVGATVDAFDIWGQGTMVTVSS |
| | | | SEQ ID NO: 27876 | SEQ ID NO: 31882 |
| **iPS:437328** | **21-225_75D3** | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCATCATTTATGGTGCATCCAGCCGGTCCACTGGCATACCAGACAGGTTCAGTGGCAGTGGGTGTGGGACAGACTTCGCTCTCACCATCAGCAGAGTGGAGCATGAAGATTGTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 27877 | SEQ ID NO: 31883 |

FIGURE 50

| | | | | |
|---|---|---|---|---|
| | | AA | EIVLTQSPGTLYLSPGERATLSCRASQSVYSSYLAWYQQKPGQAPRLIIYGASSRSTGIPDRFSGSGCGTDFALTISRVEHEDCAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 27878 | SEQ ID NO: 31884 |
| iPS:437332 | 21-225_75F3 | NA | GAAATTGTGTTGACGCAGTCTCCAGGCACCCTGTATTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGGCCACTGGCATCCCAGACAGGTTCAGTGGCAGTGGGTGTGGGACAGACTTCGCTCTCACCATCAGCAGACTGGAGCATGAAGATTTTGCAGTGTATTACTGTCAGCACTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 27879 | SEQ ID NO: 31885 |
| | | AA | EIVLTQSPGTLYLSPGERATLSCRASQSVYSSYLAWYQQKPGQAPRLLIYGASSRATGIPDRFSGSGCGTDFALTISRLEHEDFAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 27880 | SEQ ID NO: 31886 |

FIGURE 50

| | | | | |
|---|---|---|---|---|
| **iPS:437334** | **21-225_75F11** | NA | GAAATAGTGATGACGCAGTCTCCAGCCACCCTGTTTGTGTCTCCAGGGGAAAGAGCCACCCTCTCCTGCAGGGCCAGTCAGAGTGTTAGCAGAAATTTAGCCTGGTTCCAGCAGAAACCTGGCCAGGCTCCCAGGCTCCTCTTTTATGGTGCATCCATCAGGGCCACTGGTATCCCAGCCAGGTTCAGTGGCAGTGGGTCTGGGACAGAGTTCACTCTCACCATCTACAGCCTGCAGTATGAAGATTTTGCAGTTTATTACTGTCAGCAGTATAATAACTGGCCTCCGCTCACTTTCGGCGGAGGGACCAAGGTGGAGATCAAA | CAGATCACCTTGAAGGAGTCTGGTCCTACGCTGGTGAAACCCACACAGACCCTCACGCTGACCTGCACCTTCTCTGGGTTCTCACTCAGCACTGGTGGAGTGGGTGTGGGCTGGATCCGTCAGCCCCCAGGAAAGGCCCTGGAGTGGCTTGCACTCATTTATTGGGATGATGATAAGCGCTACAGCCCATCTCTGAAGAGCAGGCTCACCATCACCAAGGACACCCCCAAAAACCAGGTGGTCCTTACAATGACCAACATGGACCCTGTGGACACAGCCACATATTACTGTGCACACCTTATAGCAGTGGCCTTTGACTACTGGGGCCAGGGAACCCTGGTCACCGTCTCCTCA |
| | | | SEQ ID NO: 27881 | SEQ ID NO: 31887 |
| | | AA | EIVMTQSPATLFVSPGERATLSCRASQSVSRNLAWFQQKPGQAPRLLFYGASIRATGIPARFSGSGSGTEFTLTIYSLQYEDFAVYYCQQYNNWPPLTFGGGTKVEIK | QITLKESGPTLVKPTQTLTLTCTFSGFSLSTGGVGVGWIRQPPGKALEWLALIYWDDDKRYSPSLKSRLTITKDTPKNQVVLTMTNMDPVDTATYYCAHLIAVAFDYWGQGTLVTVSS |
| | | | SEQ ID NO: 27882 | SEQ ID NO: 31888 |
| **iPS:437340** | **21-225_75G9** | NA | GAAATTGCGTTGACGCAGTCTCCAGGCACCCTGTCTTTGTCTCCAGGGGAAAGAGCCACCCTCTCCTGCAGGGCCAGTCCGAGTGTTGACAGCAGCTACTTAGCCTGGTACCAGCAGAAACCTGGCCAGGCTCCCAGGCTCCTCATCTATGGTGCATCCAGCAGGGCCCCTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGAAAGTTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGGTTGCTACTGGAGCTGGATCCGCCAGCCCCCAGGGAAGGGGCTGGAGTGGATTGGGGAAATCAATCATAGTGGAAGGACCAACTACAACCCGTCCCTCAAGAGTCGAGTCACCATATCAGTAGACACGTCCGAGAACCAGTTCTCCCTGAAGCTGAGCTCTGTGACCGCCGCGGACACGGCTGTGTATTACTGTGCGAGAGACTACGGTGGGCTTGACTACTGGGGCCAGGGAACCCTGGTCACCGTCTCCTCA |
| | | | SEQ ID NO: 27883 | SEQ ID NO: 31889 |

FIGURE 50

| | | | | |
|---|---|---|---|---|
| | | AA | EIALTQSPGTLSLSPGERATLSCRASPSVDSSYLA WYQQKPGQAPRLLIYGASSRAPGIPDRFSGSGSG TDFTLTISRLEPEDFAVYYCQQYESSPWTFGQGT KVEIK | QVQLQQWGAGLLKPSETLSLTCAVYGGSFSGCYW SWIRQPPGKGLEWIGEINHSGRTNYNPSLKSRVTIS VDTSENQFSLKLSSVTAADTAVYYCARDYGGLDY WGQGTLVTVSS |
| | | | SEQ ID NO: 27884 | SEQ ID NO: 31890 |
| iPS:437344 | 21-225_75G12 | NA | GAAATTGTGTTGACGCAGTCTCCAGGCACCCT GTATTTGTCTCCAGGGGAAAGAGCCACCCTGT CATGCAGGGCCAGTCAGAGTGTTTACAGCAGC TACTTAGCCTGGTACCAGCAGAAACCTGGCCA GGCTCCCAGGCTCCTCATTTATGGTGCATCCA GCCGGGCCACTGGCATCCCAGACAGGTTCAGT GGCAGTGGGTCTGGGACAGACTTCGCTCTCAC CATCAGCAGAGTGGAGCCTGAAGATTGTGCAG TGTATTACTGTCAGCACTATGATAACTCACCG TGGACGTTCGGCCAAGGGACCAAGGTGGAAA TCAAA | CAGGTGCAGCTACAGCAGTGGGGCGCAGGACTG TTGAAGCCTTCGGAGACCCTGTCCCTCACCTGCG CTGTCCATGGTGGGTCCTTCAGTGGTTGCTACTG GAGCTGGATCCGCCAGCCCCCAGGGAAGGGGCT GGAGTGGATTGGGGAAATCAATCATAGTGGAAG CACCAACTACAACCCGTCCCTCAAGAGTCGAGTC ACCATTTCAGTAGACACGTCCAAGAACCAGTTCT CCCTGAAGCTGAGCTCTGTGACCGCCGCGGACAC GGCTGTGTATTACTGTGCGAGGGACTACGGCGGT ATGGACGTCTGGGGCCAAGGGACCACGGTCACC GTCTCCTCA |
| | | | SEQ ID NO: 27885 | SEQ ID NO: 31891 |
| | | AA | EIVLTQSPGTLYLSPGERATLSCRASQSVYSSYLA WYQQKPGQAPRLLIYGASSRATGIPDRFSGSGSG TDFALTISRVEPEDCAVYYCQHYDNSPWTFGQG TKVEIK | QVQLQQWGAGLLKPSETLSLTCAVHGGSFSGCYW SWIRQPPGKGLEWIGEINHSGSTNYNPSLKSRVTISV DTSKNQFSLKLSSVTAADTAVYYCARDYGGMDV WGQGTTVTVSS |
| | | | SEQ ID NO: 27886 | SEQ ID NO: 31892 |

FIGURE 50

| | | | | |
|---|---|---|---|---|
| **iPS:437346** | **21-225_75H7** | NA | GACATTCAGATGACCCAATCTCCATCCTCCCGGTATGCATCTGTAGGAGACAGAGTCACCATCAATAGCCGGGCAAGTCAGGGCATAAGAAATGATTTAGGCTGGTATCAACAGAAACCAGGGAAATCCCCTCAGCGCCTGATTTATGATGCATCCAGTTTGCAAAGTGGGGTCCCATCAAGGTTCAGCGGCAGTGGATCTGGGACAGAATTCACTCTCACAATCAGCAGCGTGCAGCCTGAAGATTTTGGTGTTTATTACTGTATACAGCATAGTAATTACCCGCTCACTTTCGGCGGAGGGACCAAGGTGGAGATCAAA | CAGCTGCAGCTGCAGGAGTCGGGCCCAGGACTGGTGACGCCTTCGGAGACCCTGTCCCTCACCTGCACTGTCTCTGGCGGCTCCATCAGCAGGAGTAGTTACTACTGGGGCTGGATCCGCCAGCCCCCAGGGAAGGGGCTGGAGTGGATTGGGAGTATCTATTATAGTGGGAGCGCCTACTCCAACCCGTCCCTCAAGAGTCGAGTCACCATATCCGTAGACACGTCCAAGAACCAGTTCTCCCTGAAGCTGAGCTCTGTGACCGCCGCGGACACGGCTCTGTTTTACTGTGCGAGACTTGACTCTAACTGGGGTCTTGACTACTGGGGCCAGGGAACCCTGGTCACCGTCTCCTCA |
| | | | SEQ ID NO: 27887 | SEQ ID NO: 31893 |
| | | AA | DIQMTQSPSSRYASVGDRVTINSRASQGIRNDLGWYQQKPGKSPQRLIYDASSLQSGVPSRFSGSGSGTEFTLTISSVQPEDFGVYYCIQHSNYPLTFGGGTKVEIK | QLQLQESGPGLVTPSETLSLTCTVSGGSISRSSYYWGWIRQPPGKGLEWIGSIYYSGSAYSNPSLKSRVTISVDTSKNQFSLKLSSVTAADTALFYCARLDSNWGLDYWGQGTLVTVSS |
| | | | SEQ ID NO: 27888 | SEQ ID NO: 31894 |
| **iPS:437350** | **21-225_74A3** | NA | GAAATTGTGTTGACGCAGTCTCCAGGCACCCTGTCTTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGTCCACTGGCATCCCAGACAGGTTCAGTGGCAGTGGGTGTGGGACAGACTTCGCTCTCACCATCAGCAGACTGGAGCCTGAAGATTTTGCAGTGTATTACTGTCAGCACTCTGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 27889 | SEQ ID NO: 31895 |

FIGURE 50

| | | | | |
|---|---|---|---|---|
| | | AA | EIVLTQSPGTLSLSPGERATLSCRASQSVYSSYLAWYQQKPGQAPRLLIYGASSRSTGIPDRFSGSGCGTDFALTISRLEPEDFAVYYCQHSDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 27890 | SEQ ID NO: 31896 |
| iPS:437356 | 21-225_74B1 | NA | GACATCGTGATGACCCAGTCTCCAGACTTCCTGGCTGTGTCTCTGGGCGAGAGGGCCACCACCAATTGTAAGTCCAGCCAGAGTGTTTTACACAGGTCCAACAATTACAACTACTTAGCGTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGATGTGGCAGTTTATTACTGTCAGCAATATTATAGTACTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTGACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAACTGAGCAGCCTGAGATCTGAGGACACGGCCGTGTATTACTGTGGGAGTACCAGTGGCTGGAACTTCTTTGACTACTGGGGCCAGGGAACCCTGGTCACCGTCTCCTCA |
| | | | SEQ ID NO: 27891 | SEQ ID NO: 31897 |
| | | AA | DIVMTQSPDFLAVSLGERATTNCKSSQSVLHRSNNYNYLAWYQQKPGQPPKLLIYWASTRESGVPDRFSGSGSGTDFTLTISSLQAEDVAVYYCQQYYSTPPTFGQGTKVEIK | QVQLVQSGAEVKKPGASVKVSCKASGYTLTNYDINWVRQATGQGLEWMGWMNPNSGNTGYAQKFQGRVTMTRNTSISTAYMELSSLRSEDTAVYYCGSTSGWNFFDYWGQGTLVTVSS |
| | | | SEQ ID NO: 27892 | SEQ ID NO: 31898 |

FIGURE 50

| | | | | |
|---|---|---|---|---|
| **iPS:437361** | **21-225_74C1** | NA | GACATCGTGATGACCCAGTGTCCAGACTCCCC GGCTGTGTCTCTGGGCGAGAGGGCCTCCATCA ATTGCAAGTCCAGCCAGAGTATTTTACACAGC TCCAACAATTACAATTACTTAGCTTGGTACCA GCAGAAACCAGGACATCCTCATAAGCTGCTCA TTTACTGGGCATCTACCCGGGAATCCGGGGTC CCTGACCGATTCAGTGGCAGCGGGTATGGGAC AGATTTCACTCTAACCATCAGCAGCCTGCAGG CTGAAGATGTGGCAGTTTATTACTGTCAGCAA TATTATAGTACTCCGTGGACGTTCGGCCAAGG GACCAAGGTGGAAATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTGACAGTG GTAACACAGGCTTTGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGGTTTCCAG TGGCTGGTACTGGTTCGACCCCTGGGGCCAGGGA ACCCTGGTCACCGTCTCCTCA |
| | | | SEQ ID NO: 27893 | SEQ ID NO: 31899 |
| | | AA | DIVMTQCPDSPAVSLGERASINCKSSQSILHSSNN YNYLAWYQQKPGHPHKLLIYWASTRESGVPDR FSGSGYGTDFTLTISSLQAEDVAVYYCQQYYSTP WTFGQGTKVEIK | QVQLVQSGAEVKKPGASVKVSCKASGYTFTNYDIN WVRQATGQGLEWMGWMNPDSGNTGFAQKFQGR VTMTRNTSISTAYMELSSLRSEDTAVYYCAVSSGW YWFDPWGQGTLVTVSS |
| | | | SEQ ID NO: 27894 | SEQ ID NO: 31900 |
| **iPS:437363** | **21-225_74C10** | NA | GACATCGTGATGACCCAGTCTCCAGACTCCCT GGCTGTGTCTCTGGGCGAGAGGGCCACCATCA ACTGCAAGTCCAGCCAGAGTGTTTTATACAGC TCCAACAATGCGAACTACTTAGCTTGGTACCA GCAGAAACCAGGACAGCCTCCTAACCTGCTCA TTTACTGGGCATCTACCCGGGAATCCGGGGTC CCTGACCGATTCAGTGGCAGCGGGTCTGGGAC AGATTTCACTCTCACCATCAGCAGCCTGCAGG CTGAAGATGTGGCAGTTTATTACTGTCAGCAA TATTATAGTACTCCGTGCAGTTTTGGCCAGGG GACCAAGCTGGAGATCAAA | CAGGTGCAGCTGGTGCAGTCTGGGGCTGAGGTG AAGAAGCCTGGGGCCTCAGTGAAGGTCTCCTGC AAGGCTTCTGGATACACCTTCACCAATTATGATA TCAACTGGGTGCGACAGGCCACTGGACAAGGGC TTGAGTGGATGGGATGGATGAACCCTAACAGTG GTAACATAGGCTATGCACAGAAGTTCCAGGGCA GAGTCACCATGACCAGGAACACCTCCATAAGCA CAGCCTACATGGAGCTGAGCAGCCTGAGATCTG AGGACACGGCCGTGTATTACTGTGCGATTAGCAG TGGCTGGTACTGGTTCGACCCCTGGGGCCAGGGA ACCCTGGTCACCGTCTCCTCA |
| | | | SEQ ID NO: 27895 | SEQ ID NO: 31901 |

FIGURE 50

| | | | | |
|---|---|---|---|---|
| | | AA | DIVMTQSPDSLAVSLGERATINCKSSQSVLYSSNNANYLAWYQQKPGQPPNLLIYWASTRESGVPDRFSGSGSGTDFTLTISSLQAEDVAVYYCQQYYSTPCSFGQGTKLEIK | QVQLVQSGAEVKKPGASVKVSCKASGYTFTNYDINWVRQATGQGLEWMGWMNPNSGNIGYAQKFQGRVTMTRNTSISTAYMELSSLRSEDTAVYYCAISSGWYWFDPWGQGTLVTVSS |
| | | | SEQ ID NO: 27896 | SEQ ID NO: 31902 |
| iPS:437369 | 21-225_74D6 | NA | GAAATTGTGTTGACGCAGTCTCCAGGCACCCTGTCTTTGTCTCCAGGGGAAAGAGCCACCCTCTCATGCAGGGCCAGTCAGAGTGTTTACAGCAGCTACTTAGCCTGGTACCAGCAGAAACCTGGCCAGGCTCCCAGGCTCCTCATTTATGGTGCATCCAGCCGGGCCACTGGCATCCCAGACAGGTTCAGTGGCAGTGGGTGTGGGACAGACTTCGCTCTCACCATCAGCAGACTGGAGCCTGAAGATTTTGCAGTGTATTACTGTCAGCATTATGATAACTCACCGTGGACGTTCGGCCAAGGGACCAAGGTGGAAATCAAA | CAGGTGCAGCTACAGCAGTGGGGCGCAGGACTGTTGAAGCCTTCGGAGACCCTGTCCCTCACCTGCGCTGTCCATGGTGGGTCCTTCAGTGGTTGCTACTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCAGTAGACACGTCCAAGAACCAGTTCTCCCTGAAGCTGAGCTCTGTGACCGCCGCGGACACGGCTGTGTATTACTGTGCGAGGGACTACGGCGGTATGGACGTCTGGGGCCAAGGGACCACGGTCACCGTCTCCTCA |
| | | | SEQ ID NO: 27897 | SEQ ID NO: 31903 |
| | | AA | EIVLTQSPGTLSLSPGERATLSCRASQSVYSSYLAWYQQKPGQAPRLLIYGASSRATGIPDRFSGSGCGTDFALTISRLEPEDFAVYYCQHYDNSPWTFGQGTKVEIK | QVQLQQWGAGLLKPSETLSLTCAVHGGSFSGCYWSWIRQPPGKGLEWIGEINHSGSTNYNPSLKSRVTISVDTSKNQFSLKLSSVTAADTAVYYCARDYGGMDVWGQGTTVTVSS |
| | | | SEQ ID NO: 27898 | SEQ ID NO: 31904 |

FIGURE 50

| | | | | |
|---|---|---|---|---|
| iPS:437371 | 21-225_74D8 | NA | GATATTGTGATGACTCAGTCTCCACTCTCCCTGCCCGTCACCCCTGGAGAGCCGGCCTCCATCTCATGCAGGTCTAGTCAGAGCCTCGTGCATAGTAGTGGATACAACTATTTGGATTGGTACCTGCAGAAGCCAGGGCAGTCTCCACAGCTCGTTATCTATTTGGGTTCTAATCGGGCCTCCGGGGTCCCTGACAGGTTCAGTGGCAGTGGATCAGGCTCAGATTTTACACTGAAGATCAGCAGAGTGGAGGCTGAGGATGTTGGACTTTATTACTGCATGCAAGCTCTACACCCTCCTCTCACTTTCGGCGGAGGGACCAAGGTGGAGATCAAA | GAGGTGCAGCTGGTGGAGTCTGGGGGAGGCTTGGTACAGCCGGGGGGGTCCCTGAGACTCTCCTGTGCAGCCTCTGGATTCACCTTCAGTAACTACGACATGCACTGGGTCCGCCAAGCTACAGGAAAAGGTCTGGAGTGGGTCTCAGCTATTGGTACTGCTGGTGACACATACTATCCAGGCTCCGTGAAGGGCCGATTCACCATCTCCAGAGAAAATGCCAAGAACTCCTTGTATCTTCAAATGAACAGCCTGAGAGCCGGGGACACGGCTGTGTATTACTGTGCAAGAGTTCTTGACTACGGTGACTCCTTGGGCTACTACTACTACGGTATGGACGTCTGGGGCCAAGGGACCACGGTCACCGTCTCCTCA |
| | | | SEQ ID NO: 27899 | SEQ ID NO: 31905 |
| | | AA | DIVMTQSPLSLPVTPGEPASISCRSSQSLVHSSGYNYLDWYLQKPGQSPQLVIYLGSNRASGVPDRFSGSGSGSDFTLKISRVEAEDVGLYYCMQALHPPLTFGGGTKVEIK | EVQLVESGGGLVQPGGSLRLSCAASGFTFSNYDMHWVRQATGKGLEWVSAIGTAGDTYYPGSVKGRFTISRENAKNSLYLQMNSLRAGDTAVYYCARVLDYGDSLGYYYYGMDVWGQGTTVTVSS |
| | | | SEQ ID NO: 27900 | SEQ ID NO: 31906 |
| iPS:437377 | 21-225_74G9 | NA | GAATTTATGTTGACGCAGTCTCCAGGCACCCTGTCTTTGTCTCCAGGGGAAAGAGCCACCCTCTCCTGCAGGGCCAGTCAGAGTGTTAGCAGCAGCTACTTAGTCTGGTATCAGCAGAAACCTGGCCAGGCTCCCAGGCTCCTCATCTATGGTGCATCCACCAGGGCCACTGGCATCCCAGACAGGTTCAGTGGCAGTGGGTCTGGGACAGACTTCACTCTCACCATCAGCAGACTGGAGCCTGAATATTTTGCAGTTTATTACTGTCAGCAGTATGGTTGCTCACCGCTCACTTTCGGCGGAGGGACCAAGGTGGAGATCACA | CAGATCACCTTGAAGGAGTCTGGTCCTACGCTGGTGAAACCCACACAGACCCTCACGCTGACCTGCACCTTCTCTGGGTTCTCACTCAGCACTGGTGGAGTGGGTGTGGGCTGGATCCGTCAGCCCCCAGGAAAGGCCCTGGAGTGGCTTGCACTCATTTATTGGGATGATGATAAGCGCTACAGCCCATCTCTGAAGAGCAGGCTCACCATCACCAAGGACACCCCCAAAAACCAGGTGGTCCTTACAATGACCAACATGGACCCTGTGGACACAGCCACATATTACTGTGCACACCTTATAGCAGTGGCCTTTGACTACTGGGGCCAGGGAACCCTGGTCACCGTCTCCTCA |
| | | | SEQ ID NO: 27901 | SEQ ID NO: 31907 |

FIGURE 50

| | | | | |
|---|---|---|---|---|
| | | AA | EFMLTQSPGTLSLSPGERATLSCRASQSVSSSYLVWYQQKPGQAPRLLIYGASTRATGIPDRFSGSGSGTDFTLTISRLEPEYFAVYYCQQYGCSPLTFGGGTKVEIT | QITLKESGPTLVKPTQTLTLTCTFSGFSLSTGGVGVGWIRQPPGKALEWLALIYWDDDKRYSPSLKSRLTITKDTPKNQVVLTMTNMDPVDTATYYCAHLIAVAFDYWGQGTLVTVSS |
| | | | SEQ ID NO: 27902 | SEQ ID NO: 31908 |
| **iPS:437379** | **21-225_74H2** | NA | GACATCGTGATGACCCAGTCTCCAGACTCCCTGGCTGTGTCTCTGGGCGAGAGGGCCACCATCAACTGCAAGTCCAGCCAGAGTGTTTTACACAGCTCCAACAATAAGAACTACTTAACTTGGTACCAGCAGAAACCAGGACAGCCTCATAAGCTGCTCATTTACTGGGCATCTACTCGGGAATCCGGGGTCCCTGACCGATTCAGTGGCAGCGGGTCTGGGACAGATTTCAGTCTCACGATCGGCAGCCTGCAGGCTGAAGATGTGGCAGTTTATTACTGTCAGCAATATTATAGTATTCCTCCGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATTCACCTTCACCAATTATGATATCAACTGGGTGCGACAGGCCACTGGACAAGGGCTTGAGTGGATGGGATGGATGCACCCTAACAGTGGTAACACAGGCTTTGCACAGAAGTTCCAGGGCAGAGTCACCATGACCAGGAACACCTCCATAAGCACAGCCTACATGGAACTGAGCAGCCTGAGATCTGAGGACACGGCCGTGTATTACTGTGCGGTTTCCAGTGGCTGGTACTGGTTCGACCCCTGGGGCCAGGGAACCCTGGTCACCGTCTCCTCA |
| | | | SEQ ID NO: 27903 | SEQ ID NO: 31909 |
| | | AA | DIVMTQSPDSLAVSLGERATINCKSSQSVLHSSNNKNYLTWYQQKPGQPHKLLIYWASTRESGVPDRFSGSGSGTDFSLTIGSLQAEDVAVYYCQQYYSIPPTFGQGTKVEIK | QVQLVQSGAEVKKPGASVKVSCKASGFTFTNYDINWVRQATGQGLEWMGWMHPNSGNTGFAQKFQGRVTMTRNTSISTAYMELSSLRSEDTAVYYCAVSSGWYWFDPWGQGTLVTVSS |
| | | | SEQ ID NO: 27904 | SEQ ID NO: 31910 |

FIGURE 50

| | | | | |
|---|---|---|---|---|
| **iPS:437383** | **21-225_74H8** | NA | GAAATTGTGTTGACGCAGTCTCCAGGCACCCTGTCTTTGTCTCCAGGGGAAAGAGCCACCCTCTCCTGCAGGGCCAGTCAGAGTTTTAGCAGCAGCTACTTAGCCTGGTACCAGCAGAAACCTGGCCAGGCTCCCAGGCTCCTCATTTATGGTGCATCCAGCAGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAGCAGTATGGTAGCTCAAGGACGTTCGGCCAAGGGACCAAGGTGGAAATCAAA | GAGGTGCACCTGGTGGAGTCTGGGGGAGGCTTGGTAAAGCCTGGGGGGTCCCTTAGACTCTCCTGTGCAGCCTCTGGATTCACTTTCAGTAACGCCTGGATGAACTGGGTCCGCCAGGCTCCAGGGAAGGGGCTGGAGTGGGTTGGCCGTATTAAAAGCAAAACTGATGGTGGGACAACAGACTACGCTGCACCCGTGAAAGGCAGATTCACCATCTCAAGAGATGATTCAAAAAACACGCTGTATCTGCAAATGAACAGCCTGAAAACCGAGGACACAGCCGTGTATTACTGTACCACAGTGGGAGCTACTACGGACTACTGGGGCCAGGGAACCCTGGTCACCGTCTCCTCA |
| | | | SEQ ID NO: 27905 | SEQ ID NO: 31911 |
| | | AA | EIVLTQSPGTLSLSPGERATLSCRASQSFSSSYLAWYQQKPGQAPRLLIYGASSRATGIPDRFSGSGSGTDFTLTISRLEPEDFAVYYCQQYGSSRTFGQGTKVEIK | EVHLVESGGGLVKPGGSLRLSCAASGFTFSNAWMNWVRQAPGKGLEWVGRIKSKTDGGTTDYAAPVKGRFTISRDDSKNTLYLQMNSLKTEDTAVYYCTTVGATTDYWGQGTLVTVSS |
| | | | SEQ ID NO: 27906 | SEQ ID NO: 31912 |
| **iPS:438664** | **21-225_216G1** | NA | GACATCGAGATGACCCAGTCTCCATCCTCACTGTCTGCATCTGTAGGAGACAGAGTCACCATCACTTGTCGGGCGAGTCAGGGCATTAGCAGTTATTTAGCCTGGCTTCAGCAGAAACCAGGGAAAGCCCCTAAGTCCCTGATCTATGCTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTCGCAACTTATTATTGCCTACGGTATGATACTTACCCTCTCACTTTCGGCGGAGGGACCAAGGTGGAGATCAAG | CAGGTGCACCTGGTGGAGTCTGGGGGAGGCGTGGTCCAGCCTGGGAGGTCCCTGAGACTCTCCTGTGCAGCGTCTGGATTCACCTTCAGTAACTATGGCATGCACTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGGGGGGACTGGAACCCCGAGGGTATGGACGTCTGGGGCCAAGGGACCACGGTCATCGTCTCCTCA |
| | | | SEQ ID NO: 27907 | SEQ ID NO: 31913 |

FIGURE 50

| | | | | |
|---|---|---|---|---|
| | | AA | DIEMTQSPSSLSASVGDRVTITCRASQGISSYLAWLQQKPGKAPKSLIYAASSLQSGVPSRFSGSGSGTDFTLTISSLQPEDFATYYCLRYDTYPLTFGGGTKVEIK | QVHLVESGGGVVQPGRSLRLSCAASGFTFSNYGMHWVRQAPGKGLEWVAVIWYDGSNKYYADSVKGRFTISRDNSKNTLYLQMNSLRAEDTAVYYCARGDWNPEGMDVWGQGTTVIVSS |
| | | | SEQ ID NO: 27908 | SEQ ID NO: 31914 |
| **iPS:441468** | **21-225_25A4.001.001** | NA | GACATCGTGATGACCCAGTTTCCAGACTCCCTGGCTGTGTCTCTGGGCGAGAGGGCCACCATCAAGTGTAAGTCCAGCCAGAGTGTTTTATACAGCTCCCACAATAACAACTACTTAGCTTGGTACCAGCAGAAACCAGGACAGCCTCCTAAGTTGCTCCTTTACTGGGCATCTACCCGGGAATCCGGGGTCCCTGACCGATTCAGTGGCAGCGGGTCTGGGACAGAATTCACTCTCACCATCAGCAGCCTGGAGGCTGAAGATGTGGCAGTTTATTACTGTCAGCAGTATTATAGTACTCCTCCGACGTTCGGCCAAGGGACCAAGGTGGAAATCAAA | CAGGTGCTCCTGGTGCAGTCTGGGGCTGAGGTGAAGAGGCCTGGGGCCTCAGTGAAGGTCAGCTGCAAGGCTTCTGGATACACCTTCACCAATTATGATATTAATTGGGTGCGACAGGCCACTGGACAAGGGCTTGAGTGGATGGGATGGATGTACCCTAACAGTGGTAGCACAGGCTATGCACAGAAATTCCAGGGCAGAGTCACCATGACCAGGGACACCTCCATCAGCACAGCCTACATGGAGCTGAGCAGCCTGAGATCTGAGGACACGGCCGTCTATTACTGTGCGAGTAGCAGTGGCTGGTACTACTTTGACTACTGGGGCCAGGGAACCCTGGTCACCGTGTCCTCA |
| | | | SEQ ID NO: 27909 | SEQ ID NO: 31915 |
| | | AA | DIVMTQFPDSLAVSLGERATIKCKSSQSVLYSSHNNNYLAWYQQKPGQPPKLLLYWASTRESGVPDRFSGSGSGTEFTLTISSLEAEDVAVYYCQQYYSTPPTFGQGTKVEIK | QVLLVQSGAEVKRPGASVKVSCKASGYTFTNYDINWVRQATGQGLEWMGWMYPNSGSTGYAQKFQGRVTMTRDTSISTAYMELSSLRSEDTAVYYCASSSGWYYFDYWGQGTLVTVSS |
| | | | SEQ ID NO: 27910 | SEQ ID NO: 31916 |

FIGURE 50

| | | | | |
|---|---|---|---|---|
| iPS:441475 | 21-225_25A4.001.002 | NA | GACATCGTGATGACCCAGTTTCCAGACTCCCTGGCTGTGTCTCTGGGCGAGAGGGCCACCATCAAGTGTAAGTCCAGCCAGAGTGTTTTATACAGCTCCCACAATAACAACTACTTAGCTTGGTACCAGCAGAAACCAGGACAGCCTCCTAAGTTGCTCCTTTACTGGGCATCTACCCGGGAATCCGGGGTCCCTGACCGATTCAGTGGCAGCGGGTCTGGGACAGAATTCACTCTCACCATCAGCAGCCTGGAGGCTGAAGATGTGGCAGTTTATTACTGTCAGCAGTATTATAGTACTCCTCCGACGTTCGGCCAAGGGACCAAGGTGGAAATCAAA | CAGGTGCTCCTGGTGCAGTCTGGGGCTGAGGTGAAGAGGCCTGGGGCCTCAGTGAAGGTCAGCTGCAAGGCTTCTGGATACACCTTCACCAATTATGATATTAATTGGGTGCGACAGGCCACTGGACAAGGGCTTGAGTGGATGGGATGGATGTACCCTAACAGTGGTAACGCAGGCTATGCACAGAAATTCCAGGGCAGAGTCACCATGACCAGGGACACCTCCATCAGCACAGCCTACATGGAGCTGAGCAGCCTGAGATCTGAGGACACGGCCGTCTATTACTGTGCGAGTAGCAGTGGCTGGTACTACTTTGACTACTGGGGCCAGGGAACCCTGGTCACCGTGTCCTCA |
| | | | SEQ ID NO: 27911 | SEQ ID NO: 31917 |
| | | AA | DIVMTQFPDSLAVSLGERATIKCKSSQSVLYSSHNNNYLAWYQQKPGQPPKLLLYWASTRESGVPDRFSGSGSGTEFTLTISSLEAEDVAVYYCQQYYSTPPTFGQGTKVEIK | QVLLVQSGAEVKRPGASVKVSCKASGYTFTNYDINWVRQATGQGLEWMGWMYPNSGNAGYAQKFQGRVTMTRDTSISTAYMELSSLRSEDTAVYYCASSSGWYYFDYWGQGTLVTVSS |
| | | | SEQ ID NO: 27912 | SEQ ID NO: 31918 |
| iPS:441482 | 21-225_25A4.001.003 | NA | GACATCGTGATGACCCAGTTTCCAGACTCCCTGGCTGTGTCTCTGGGCGAGAGGGCCACCATCAAGTGTAAGTCCAGCCAGAGTGTTTTATACAGCTCCCACAATAACAACTACTTAGCTTGGTACCAGCAGAAACCAGGACAGCCTCCTAAGTTGCTCCTTTACTGGGCATCTACCCGGGAATCCGGGGTCCCTGACCGATTCAGTGGCAGCGGGTCTGGGACAGAATTCACTCTCACCATCAGCAGCCTGGAGGCTGAAGATGTGGCAGTTTATTACTGTCAGCAGTATTATAGTACTCCTCCGACGTTCGGCCAAGGGACCAAGGTGGAAATCAAA | CAGGTGCTCCTGGTGCAGTCTGGGGCTGAGGTGAAGAGGCCTGGGGCCTCAGTGAAGGTCAGCTGCAAGGCTTCTGGATACACCTTCACCAATTATGATATTAATTGGGTGCGACAGGCCACTGGACAAGGGCTTGAGTGGATGGGATGGATGTACCCTAACAGTGGTAACGTAGGCTATGCACAGAAATTCCAGGGCAGAGTCACCATGACCAGGGACACCTCCATCAGCACAGCCTACATGGAGCTGAGCAGCCTGAGATCTGAGGACACGGCCGTCTATTACTGTGCGAGTAGCAGTGGCTGGTACTACTTTGACTACTGGGGCCAGGGAACCCTGGTCACCGTGTCCTCA |
| | | | SEQ ID NO: 27913 | SEQ ID NO: 31919 |

FIGURE 50

| | | | | |
|---|---|---|---|---|
| | | AA | DIVMTQFPDSLAVSLGERATIKCKSSQSVLYSSHNNNYLAWYQQKPGQPPKLLLYWASTRESGVPDRFSGSGSGTEFTLTISSLEAEDVAVYYCQQYYSTPPTFGQGTKVEIK | QVLLVQSGAEVKRPGASVKVSCKASGYTFTNYDINWVRQATGQGLEWMGWMYPNSGNVGYAQKFQGRVTMTRDTSISTAYMELSSLRSEDTAVYYCASSSGWYYFDYWGQGTLVTVSS |
| | | | SEQ ID NO: 27914 | SEQ ID NO: 31920 |
| **iPS:441489** | **21-225_25A4.001.004** | NA | GACATCGTGATGACCCAGTTTCCAGACTCCCTGGCTGTGTCTCTGGGCGAGAGGGCCACCATCAAGTGTAAGTCCAGCCAGAGTGTTTTATACAGCTCCCACAATAACAACTACTTAGCTTGGTACCAGCAGAAACCAGGACAGCCTCCTAAGTTGCTCCTTTACTGGGCATCTACCCGGGAATCCGGGGTCCCTGACCGATTCAGTGGCAGCGGGTCTGGGACAGAATTCACTCTCACCATCAGCAGCCTGGAGGCTGAAGATGTGGCAGTTTATTACTGTCAGCAGTATTATAGTACTCCTCCGACGTTCGGCCAAGGGACCAAGGTGGAAATCAAA | CAGGTGCTCCTGGTGCAGTCTGGGGCTGAGGTGAAGAGGCCTGGGGCCTCAGTGAAGGTCAGCTGCAAGGCTTCTGGATACACCTTCACCAATTATGATATTAATTGGGTGCGACAGGCCACTGGACAAGGGCTTGAGTGGATGGGATGGATGTACCCTAACAGTGGTCAAACAGGCTATGCACAGAAATTCCAGGGCAGAGTCACCATGACCAGGGACACCTCCATCAGCACAGCCTACATGGAGCTGAGCAGCCTGAGATCTGAGGACACGGCCGTCTATTACTGTGCGAGTAGCAGTGGCTGGTACTACTTTGACTACTGGGGCCAGGGAACCCTGGTCACCGTGTCCTCA |
| | | | SEQ ID NO: 27915 | SEQ ID NO: 31921 |
| | | AA | DIVMTQFPDSLAVSLGERATIKCKSSQSVLYSSHNNNYLAWYQQKPGQPPKLLLYWASTRESGVPDRFSGSGSGTEFTLTISSLEAEDVAVYYCQQYYSTPPTFGQGTKVEIK | QVLLVQSGAEVKRPGASVKVSCKASGYTFTNYDINWVRQATGQGLEWMGWMYPNSGQTGYAQKFQGRVTMTRDTSISTAYMELSSLRSEDTAVYYCASSSGWYYFDYWGQGTLVTVSS |
| | | | SEQ ID NO: 27916 | SEQ ID NO: 31922 |

FIGURE 50

| | | | | |
|---|---|---|---|---|
| **iPS:441496** | **21-225_25A4.001.005** | NA | GACATCGTGATGACCCAGTCTCCAGACTCCCTGGCTGTGTCTCTGGGCGAGAGGGCCACCATCAAGTGTAAGTCCAGCCAGAGTGTTTTATACAGCTCCCACAATAACAACTACTTAGCTTGGTACCAGCAGAAACCAGGACAGCCTCCTAAGTTGCTCCTTTACTGGGCATCTACCCGGGAATCCGGGGTCCCTGACCGATTCAGTGGCAGCGGGTCTGGGACAGAATTCACTCTCACCATCAGCAGCCTGCAGGCTGAAGATGTGGCAGTTTATTACTGTCAGCAGTATTATAGTACTCCTCCGACGTTCGGCCAAGGGACCAAGGTGGAAATCAAA | CAGGTGCAACTGGTGCAGTCTGGGGCTGAGGTGAAGAGGCCTGGGGCCTCAGTGAAGGTCAGCTGCAAGGCTTCTGGATACACCTTCACCAATTATGATATTAATTGGGTGCGACAGGCCACTGGACAAGGGCTTGAGTGGATGGGATGGATGTACCCTAACAGTGGTAGCACAGGCTATGCACAGAAATTCCAGGGCAGAGTCACCATGACCAGGGACACCTCCATCAGCACAGCCTACATGGAGCTGAGCAGCCTGAGATCTGAGGACACGGCCGTCTATTACTGTGCGAGTAGCAGTGGCTGGTACTACTTTGACTACTGGGGCCAGGGAACCCTGGTCACCGTGTCCTCA |
| | | | SEQ ID NO: 27917 | SEQ ID NO: 31923 |
| | | AA | DIVMTQSPDSLAVSLGERATIKCKSSQSVLYSSHNNNYLAWYQQKPGQPPKLLLYWASTRESGVPDRFSGSGSGTEFTLTISSLQAEDVAVYYCQQYYSTPPTFGQGTKVEIK | QVQLVQSGAEVKRPGASVKVSCKASGYTFTNYDINWVRQATGQGLEWMGWMYPNSGSTGYAQKFQGRVTMTRDTSISTAYMELSSLRSEDTAVYYCASSSGWYYFDYWGQGTLVTVSS |
| | | | SEQ ID NO: 27918 | SEQ ID NO: 31924 |
| **iPS:441505** | **21-225_25A4.001.006** | NA | GACATCGTGATGACCCAGTCTCCAGACTCCCTGGCTGTGTCTCTGGGCGAGAGGGCCACCATCAAGTGTAAGTCCAGCCAGAGTGTTTTATACAGCTCCCACAATAACAACTACTTAGCTTGGTACCAGCAGAAACCAGGACAGCCTCCTAAGTTGCTCCTTTACTGGGCATCTACCCGGGAATCCGGGGTCCCTGACCGATTCAGTGGCAGCGGGTCTGGGACAGAATTCACTCTCACCATCAGCAGCCTGCAGGCTGAAGATGTGGCAGTTTATTACTGTCAGCAGTATTATAGTACTCCTCCGACGTTCGGCCAAGGGACCAAGGTGGAAATCAAA | CAGGTGCAACTGGTGCAGTCTGGGGCTGAGGTGAAGAGGCCTGGGGCCTCAGTGAAGGTCAGCTGCAAGGCTTCTGGATACACCTTCACCAATTATGATATTAATTGGGTGCGACAGGCCACTGGACAAGGGCTTGAGTGGATGGGATGGATGTACCCTAACAGTGGTAACGCAGGCTATGCACAGAAATTCCAGGGCAGAGTCACCATGACCAGGGACACCTCCATCAGCACAGCCTACATGGAGCTGAGCAGCCTGAGATCTGAGGACACGGCCGTCTATTACTGTGCGAGTAGCAGTGGCTGGTACTACTTTGACTACTGGGGCCAGGGAACCCTGGTCACCGTGTCCTCA |
| | | | SEQ ID NO: 27919 | SEQ ID NO: 31925 |

FIGURE 50

| | | | | |
|---|---|---|---|---|
| | | AA | DIVMTQSPDSLAVSLGERATIKCKSSQSVLYSSHNNNYLAWYQQKPGQPPKLLLYWASTRESGVPDRFSGSGSGTEFTLTISSLQAEDVAVYYCQQYYSTPPTFGQGTKVEIK | QVQLVQSGAEVKRPGASVKVSCKASGYTFTNYDINWVRQATGQGLEWMGWMYPNSGNAGYAQKFQGRVTMTRDTSISTAYMELSSLRSEDTAVYYCASSSGWYYFDYWGQGTLVTVSS |
| | | | SEQ ID NO: 27920 | SEQ ID NO: 31926 |
| **iPS:441512** | **21-225_25A4.001.007** | NA | GACATCGTGATGACCCAGTCTCCAGACTCCCTGGCTGTGTCTCTGGGCGAGAGGGCCACCATCAAGTGTAAGTCCAGCCAGAGTGTTTTATACAGCTCCCACAATAACAACTACTTAGCTTGGTACCAGCAGAAACCAGGACAGCCTCCTAAGTTGCTCCTTTACTGGGCATCTACCCGGGAATCCGGGGTCCCTGACCGATTCAGTGGCAGCGGGTCTGGGACAGAATTCACTCTCACCATCAGCAGCCTGCAGGCTGAAGATGTGGCAGTTTATTACTGTCAGCAGTATTATAGTACTCCTCCGACGTTCGGCCAAGGGACCAAGGTGGAAATCAAA | CAGGTGCAACTGGTGCAGTCTGGGGCTGAGGTGAAGAGGCCTGGGGCCTCAGTGAAGGTCAGCTGCAAGGCTTCTGGATACACCTTCACCAATTATGATATTAATTGGGTGCGACAGGCCACTGGACAAGGGCTTGAGTGGATGGGATGGATGTACCCTAACAGTGGTAACGTAGGCTATGCACAGAAATTCCAGGGCAGAGTCACCATGACCAGGGACACCTCCATCAGCACAGCCTACATGGAGCTGAGCAGCCTGAGATCTGAGGACACGGCCGTCTATTACTGTGCGAGTAGCAGTGGCTGGTACTACTTTGACTACTGGGGCCAGGGAACCCTGGTCACCGTGTCCTCA |
| | | | SEQ ID NO: 27921 | SEQ ID NO: 31927 |
| | | AA | DIVMTQSPDSLAVSLGERATIKCKSSQSVLYSSHNNNYLAWYQQKPGQPPKLLLYWASTRESGVPDRFSGSGSGTEFTLTISSLQAEDVAVYYCQQYYSTPPTFGQGTKVEIK | QVQLVQSGAEVKRPGASVKVSCKASGYTFTNYDINWVRQATGQGLEWMGWMYPNSGNVGYAQKFQGRVTMTRDTSISTAYMELSSLRSEDTAVYYCASSSGWYYFDYWGQGTLVTVSS |
| | | | SEQ ID NO: 27922 | SEQ ID NO: 31928 |

FIGURE 50

| | | | | |
|---|---|---|---|---|
| **iPS:441519** | **21-225_25A4.001.008** | NA | GACATCGTGATGACCCAGTCTCCAGACTCCCTGGCTGTGTCTCTGGGCGAGAGGGCCACCATCAAGTGTAAGTCCAGCCAGAGTGTTTTATACAGCTCCCACAATAACAACTACTTAGCTTGGTACCAGCAGAAACCAGGACAGCCTCCTAAGTTGCTCCTTTACTGGGCATCTACCCGGGAATCCGGGGTCCCTGACCGATTCAGTGGCAGCGGGTCTGGGACAGAATTCACTCTCACCATCAGCAGCCTGCAGGCTGAAGATGTGGCAGTTTATTACTGTCAGCAGTATTATAGTACTCCTCCGACGTTCGGCCAAGGGACCAAGGTGGAAATCAAA | CAGGTGCAACTGGTGCAGTCTGGGGCTGAGGTGAAGAGGCCTGGGGCCTCAGTGAAGGTCAGCTGCAAGGCTTCTGGATACACCTTCACCAATTATGATATTAATTGGGTGCGACAGGCCACTGGACAAGGGCTTGAGTGGATGGGATGGATGTACCCTAACAGTGGTCAAACAGGCTATGCACAGAAATTCCAGGGCAGAGTCACCATGACCAGGGACACCTCCATCAGCACAGCCTACATGGAGCTGAGCAGCCTGAGATCTGAGGACACGGCCGTCTATTACTGTGCGAGTAGCAGTGGCTGGTACTACTTTGACTACTGGGGCCAGGGAACCCTGGTCACCGTGTCCTCA |
| | | | SEQ ID NO: 27923 | SEQ ID NO: 31929 |
| | | AA | DIVMTQSPDSLAVSLGERATIKCKSSQSVLYSSHNNNYLAWYQQKPGQPPKLLLYWASTRESGVPDRFSGSGSGTEFTLTISSLQAEDVAVYYCQQYYSTPPTFGQGTKVEIK | QVQLVQSGAEVKRPGASVKVSCKASGYTFTNYDINWVRQATGQGLEWMGWMYPNSGQTGYAQKFQGRVTMTRDTSISTAYMELSSLRSEDTAVYYCASSSGWYYFDYWGQGTLVTVSS |
| | | | SEQ ID NO: 27924 | SEQ ID NO: 31930 |
| **iPS:441554** | **21-225_25A4.001.013** | NA | GACATCGTGATGACCCAGTTTCCAGACTCCCTGGCTGTGTCTCTGGGCGAGAGGGCCACCATCAAGTGTAAGTCCAGCCAGAGTGTTTTATACAGCTCCCACAATAACAACTACTTAGCTTGGTACCAGCAGAAACCAGGACAGCCTCCTAAGTTGCTCCTTTACTGGGCATCTACCCGGGAATCCGGGGTCCCTGACCGATTCAGTGGCAGCGGGTCTGGGACAGAATTCACTCTCACCATCAGCAGCCTGCAGGCTGAAGATGTGGCAGTTTATTACTGTCAGCAGTATTATAGTACTCCTCCGACGTTCGGCCAAGGGACCAAGGTGGAAATCAAA | CAGGTGCTCCTGGTGCAGTCTGGGGCTGAGGTGAAGAGGCCTGGGGCCTCAGTGAAGGTCAGCTGCAAGGCTTCTGGATACACCTTCACCAATTATGATATTAATTGGGTGCGACAGGCCACTGGACAAGGGCTTGAGTGGATGGGATGGATGTACCCTAACAGTGGTAGCACAGGCTATGCACAGAAATTCCAGGGCAGAGTCACCATGACCAGGGACACCTCCATCAGCACAGCCTACATGGAGCTGAGCAGCCTGAGATCTGAGGACACGGCCGTCTATTACTGTGCGAGTAGCAGTGGCTGGTACTACTTTGACTACTGGGGCCAGGGAACCCTGGTCACCGTGTCCTCA |
| | | | SEQ ID NO: 27925 | SEQ ID NO: 31931 |

FIGURE 50

| | | | | |
|---|---|---|---|---|
| | | AA | DIVMTQFPDSLAVSLGERATIKCKSSQSVLYSSHNNNYLAWYQQKPGQPPKLLLYWASTRESGVPDRFSGSGSGTEFTLTISSLQAEDVAVYYCQQYYSTPPTFGQGTKVEIK | QVLLVQSGAEVKRPGASVKVSCKASGYTFTNYDINWVRQATGQGLEWMGWMYPNSGSTGYAQKFQGRVTMTRDTSISTAYMELSSLRSEDTAVYYCASSSGWYYFDYWGQGTLVTVSS |
| | | | SEQ ID NO: 27926 | SEQ ID NO: 31932 |
| **iPS:441595** | **21-225_25A4.001.019** | NA | GACATCGTGATGACCCAGTCTCCAGACTCCCTGGCTGTGTCTCTGGGCGAGAGGGCCACCATCAACTGTAAGTCCAGCCAGAGTGTTTTATACAGCTCCCACAATAACAACTACTTAGCTTGGTACCAGCAGAAACCAGGACAGCCTCCTAAGTTGCTCCTTTACTGGGCATCTACCCGGGAATCCGGGGTCCCTGACCGATTCAGTGGCAGCGGGTCTGGGACAGAATTCACTCTCACCATCAGCAGCCTGGAGGCTGAAGATGTGGCAGTTTATTACTGTCAGCAGTATTATAGTACTCCTCCGACGTTCGGCCAAGGGACCAAGGTGGAAATCAAA | CAGGTGCAACTGGTGCAGTCTGGGGCTGAGGTGAAGAGGCCTGGGGCCTCAGTGAAGGTCAGCTGCAAGGCTTCTGGATACACCTTCACCAATTATGATATTAATTGGGTGCGACAGGCCCCTGGACAAGGGCTTGAGTGGATGGGATGGATGTACCCTAACAGTGGTAGCACAGGCTATGCACAGAAATTCCAGGGCAGAGTCACCATGACCAGGGACACCTCCATCAGCACAGCCTACATGGAGCTGAGCAGCCTGAGATCTGAGGACACGGCCGTCTATTACTGTGCGAGTAGCAGTGGCTGGTACTACTTTGACTACTGGGGCCAGGGAACCCTGGTCACCGTGTCCTCA |
| | | | SEQ ID NO: 27927 | SEQ ID NO: 31933 |
| | | AA | DIVMTQSPDSLAVSLGERATINCKSSQSVLYSSHNNNYLAWYQQKPGQPPKLLLYWASTRESGVPDRFSGSGSGTEFTLTISSLEAEDVAVYYCQQYYSTPPTFGQGTKVEIK | QVQLVQSGAEVKRPGASVKVSCKASGYTFTNYDINWVRQAPGQGLEWMGWMYPNSGSTGYAQKFQGRVTMTRDTSISTAYMELSSLRSEDTAVYYCASSSGWYYFDYWGQGTLVTVSS |
| | | | SEQ ID NO: 27928 | SEQ ID NO: 31934 |

FIGURE 50

| | | | | |
|---|---|---|---|---|
| **iPS:441604** | **21-225_25A4.001.020** | NA | GACATCGTGATGACCCAGTCTCCAGACTCCCT GGCTGTGTCTCTGGGCGAGAGGGCCACCATCA ACTGTAAGTCCAGCCAGAGTGTTTTATACAGC TCCCACAATAACAACTACTTAGCTTGGTACCA GCAGAAACCAGGACAGCCTCCTAAGTTGCTCC TTTACTGGGCATCTACCCGGGAATCCGGGGTC CCTGACCGATTCAGTGGCAGCGGGTCTGGGAC AGAATTCACTCTCACCATCAGCAGCCTGCAGG CTGAAGATGTGGCAGTTTATTACTGTCAGCAG TATTATAGTACTCCTCCGACGTTCGGCCAAGG GACCAAGGTGGAAATCAAA | CAGGTGCTCCTGGTGCAGTCTGGGGCTGAGGTGA AGAGGCCTGGGGCCTCAGTGAAGGTCAGCTGCA AGGCTTCTGGATACACCTTCACCAATTATGATAT TAATTGGGTGCGACAGGCCCCTGGACAAGGGCTT GAGTGGATGGGATGGATGTACCCTAACAGTGGTC AAACAGGCTATGCACAGAAATTCCAGGGCAGAG TCACCATGACCAGGGACACCTCCATCAGCACAGC CTACATGGAGCTGAGCAGCCTGAGATCTGAGGA CACGGCCGTCTATTACTGTGCGAGTAGCAGTGGC TGGTACTACTTTGACTACTGGGGCCAGGGAACCC TGGTCACCGTGTCCTCA |
| | | | SEQ ID NO: 27929 | SEQ ID NO: 31935 |
| | | AA | DIVMTQSPDSLAVSLGERATINCKSSQSVLYSSH NNNYLAWYQQKPGQPPKLLLYWASTRESGVPD RFSGSGSGTEFTLTISSLQAEDVAVYYCQQYYST PPTFGQGTKVEIK | QVLLVQSGAEVKRPGASVKVSCKASGYTFTNYDIN WVRQAPGQGLEWMGWMYPNSGQTGYAQKFQGR VTMTRDTSISTAYMELSSLRSEDTAVYYCASSSGW YYFDYWGQGTLVTVSS |
| | | | SEQ ID NO: 27930 | SEQ ID NO: 31936 |
| **iPS:441613** | **21-225_25A4.001.021** | NA | GACATCGTGATGACCCAGTCTCCAGACTCCCT GGCTGTGTCTCTGGGCGAGAGGGCCACCATCA ACTGTAAGTCCAGCCAGAGTGTTTTATACAGC TCCCACAATAACAACTACTTAGCTTGGTACCA GCAGAAACCAGGACAGCCTCCTAAGTTGCTCC TTTACTGGGCATCTACCCGGGAATCCGGGGTC CCTGACCGATTCAGTGGCAGCGGGTCTGGGAC AGAATTCACTCTCACCATCAGCAGCCTGCAGG CTGAAGATGTGGCAGTTTATTACTGTCAGCAG TATTATAGTACTCCTCCGACGTTCGGCCAAGG GACCAAGGTGGAAATCAAA | CAGGTGCAACTGGTGCAGTCTGGGGCTGAGGTG AAGAGGCCTGGGGCCTCAGTGAAGGTCAGCTGC AAGGCTTCTGGATACACCTTCACCAATTATGATA TTAATTGGGTGCGACAGGCCACTGGACAAGGGC TTGAGTGGATGGGATGGATGTACCCTAACAGTGG TAACGCAGGCTATGCACAGAAATTCCAGGGCAG AGTCACCATGACCAGGGACACCTCCATCAGCAC AGCCTACATGGAGCTGAGCAGCCTGAGATCTGA GGACACGGCCGTCTATTACTGTGCGAGTAGCAGT GGCTGGTACTACTTTGACTACTGGGGCCAGGGAA CCCTGGTCACCGTGTCCTCA |
| | | | SEQ ID NO: 27931 | SEQ ID NO: 31937 |

FIGURE 50

| | | | | |
|---|---|---|---|---|
| | | AA | DIVMTQSPDSLAVSLGERATINCKSSQSVLYSSHNNNYLAWYQQKPGQPPKLLLYWASTRESGVPDRFSGSGSGTEFTLTISSLQAEDVAVYYCQQYYSTPPTFGQGTKVEIK | QVQLVQSGAEVKRPGASVKVSCKASGYTFTNYDINWVRQATGQGLEWMGWMYPNSGNAGYAQKFQGRVTMTRDTSISTAYMELSSLRSEDTAVYYCASSSGWYYFDYWGQGTLVTVSS |
| | | | SEQ ID NO: 27932 | SEQ ID NO: 31938 |
| **iPS:441841** | **21-225_4A2.001.001** | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATAGTACTCCAGTCACTTTCGGCCCTGGGACCAAAGTGGGTATCAAA | CAGGTGCAGCTGGTGCAGTCTGGGACTGAGGTGAAGAAGCCTGGGGCCTCAGTGAAGGTGTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCTTGACCAGGGACACCTCCATCAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTGTCCTCA |
| | | | SEQ ID NO: 27933 | SEQ ID NO: 31939 |
| | | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYSTPVTFGPGTKVGIK | QVQLVQSGTEVKKPGASVKVSCKASGYTFTNYDINWVRQATGQGLEWMGWMHPNSGNTGYAQKFQGRVTLTRDTSISTAYMELSSLRSEDTAVYYCASSSGWYYFDYWGQGTLVTVSS |
| | | | SEQ ID NO: 27934 | SEQ ID NO: 31940 |

FIGURE 50

| | | | | |
|---|---|---|---|---|
| **iPS:441847** | **21-225_4A2.001.002** | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATAATGCTCCAGTCACTTTCGGCCCTGGGACCAAAGTGGGTATCAAA | CAGGTGCAGCTGGTGCAGTCTGGGACTGAGGTGAAGAAGCCTGGGGCCTCAGTGAAGGTGTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCTTGACCAGGGACACCTCCATCAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTGTCCTCA |
| | | | SEQ ID NO: 27935 | SEQ ID NO: 31941 |
| | | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYNAPVTFGPGTKVGIK | QVQLVQSGTEVKKPGASVKVSCKASGYTFTNYDINWVRQATGQGLEWMGWMHPNSGNTGYAQKFQGRVTLTRDTSISTAYMELSSLRSEDTAVYYCASSSGWYYFDYWGQGTLVTVSS |
| | | | SEQ ID NO: 27936 | SEQ ID NO: 31942 |
| **iPS:441853** | **21-225_4A2.001.003** | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATCAAACTCCAGTCACTTTCGGCCCTGGGACCAAAGTGGGTATCAAA | CAGGTGCAGCTGGTGCAGTCTGGGACTGAGGTGAAGAAGCCTGGGGCCTCAGTGAAGGTGTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCTTGACCAGGGACACCTCCATCAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTGTCCTCA |
| | | | SEQ ID NO: 27937 | SEQ ID NO: 31943 |

FIGURE 50

| | | | | |
|---|---|---|---|---|
| | | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYQTPVTFGPGTKVGIK | QVQLVQSGTEVKKPGASVKVSCKASGYTFTNYDINWVRQATGQGLEWMGWMHPNSGNTGYAQKFQGRVTLTRDTSISTAYMELSSLRSEDTAVYYCASSSGWYYFDYWGQGTLVTVSS |
| | | | SEQ ID NO: 27938 | SEQ ID NO: 31944 |
| **iPS:441859** | **21-225_4A2.001.004** | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATAATACTCCAGTCACTTTCGGCCCTGGGACCAAAGTGGGTATCAAA | CAGGTGCAGCTGGTGCAGTCTGGGACTGAGGTGAAGAAGCCTGGGGCCTCAGTGAAGGTGTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACGCAGGCTATGCACAGAAGTTCCAGGGCAGAGTCACCTTGACCAGGGACACCTCCATCAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTGTCCTCA |
| | | | SEQ ID NO: 27939 | SEQ ID NO: 31945 |
| | | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYNTPVTFGPGTKVGIK | QVQLVQSGTEVKKPGASVKVSCKASGYTFTNYDINWVRQATGQGLEWMGWMHPNSGNAGYAQKFQGRVTLTRDTSISTAYMELSSLRSEDTAVYYCASSSGWYYFDYWGQGTLVTVSS |
| | | | SEQ ID NO: 27940 | SEQ ID NO: 31946 |

FIGURE 50

| | | | | |
|---|---|---|---|---|
| iPS:441866 | 21-225_4A2.001.005 | NA | GACATCGTGATGACCCAGTCTCCAGACTCCCT GGCTGTGTCTCTGGGCGAGAGGGCCACCATCA ACTGCAAGTCCAGCCAGAGTATTTTACACAGC TCCAACAATAACAACTACTTAGCTTGGTTCCA GCAGAAACCAGGACAGCCTCCTAAACTGCTCC TTTACTGGGCATCTACCCGGGAATCCGGGGTC CCTGACCGATTCAGTGGCAGCGGGTCTGGGAC AGATTTCACTCTCACCATCAGCAGCCTGCAGC CTGAAGATGTGGCAGTTTATTACTGTCAGCAA TATTATAATACTCCAGTCACTTTCGGCCCTGGG ACCAAAGTGGGTATCAAA | CAGGTGCAGCTGGTGCAGTCTGGGACTGAGGTG AAGAAGCCTGGGGCCTCAGTGAAGGTGTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TAGCACAGGCTATGCACAGAAGTTCCAGGGCAG AGTCACCTTGACCAGGGACACCTCCATCAGCACA GCCTACATGGAACTGAGCAGCCTGAGATCTGAG GACACGGCCGTGTATTACTGTGCGAGTAGCAGTG GCTGGTACTACTTTGACTACTGGGGCCAGGGAAC CCTGGTCACCGTGTCCTCA |
| | | | SEQ ID NO: 27941 | SEQ ID NO: 31947 |
| | | AA | DIVMTQSPDSLAVSLGERATINCKSSQSILHSSNN NNYLAWFQQKPGQPPKLLLYWASTRESGVPDR FSGSGSGTDFTLTISSLQPEDVAVYYCQQYYNTP VTFGPGTKVGIK | QVQLVQSGTEVKKPGASVKVSCKASGYTFTNYDIN WVRQATGQGLEWMGWMHPNSGSTGYAQKFQGR VTLTRDTSISTAYMELSSLRSEDTAVYYCASSSGWY YFDYWGQGTLVTVSS |
| | | | SEQ ID NO: 27942 | SEQ ID NO: 31948 |
| iPS:441873 | 21-225_4A2.001.006 | NA | GACATCGTGATGACCCAGTCTCCAGACTCCCT GGCTGTGTCTCTGGGCGAGAGGGCCACCATCA ACTGCAAGTCCAGCCAGAGTATTTTACACAGC TCCAACAATAACAACTACTTAGCTTGGTTCCA GCAGAAACCAGGACAGCCTCCTAAACTGCTCC TTTACTGGGCATCTACCCGGGAATCCGGGGTC CCTGACCGATTCAGTGGCAGCGGGTCTGGGAC AGATTTCACTCTCACCATCAGCAGCCTGCAGC CTGAAGATGTGGCAGTTTATTACTGTCAGCAA TATTATAATACTCCAGTCACTTTCGGCCCTGGG ACCAAAGTGGGTATCAAA | CAGGTGCAGCTGGTGCAGTCTGGGACTGAGGTG AAGAAGCCTGGGGCCTCAGTGAAGGTGTCCTGC AAGGCTTCTGGATACACCTTCACCAATTATGATA TCAACTGGGTGCGACAGGCCACTGGACAAGGGC TTGAGTGGATGGGATGGATGCACCCTAACAGTGG TCAAACAGGCTATGCACAGAAGTTCCAGGGCAG AGTCACCTTGACCAGGGACACCTCCATCAGCACA GCCTACATGGAACTGAGCAGCCTGAGATCTGAG GACACGGCCGTGTATTACTGTGCGAGTAGCAGTG GCTGGTACTACTTTGACTACTGGGGCCAGGGAAC CCTGGTCACCGTGTCCTCA |
| | | | SEQ ID NO: 27943 | SEQ ID NO: 31949 |

FIGURE 50

| | | | | |
|---|---|---|---|---|
| | | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYNTPVTFGPGTKVGIK | QVQLVQSGTEVKKPGASVKVSCKASGYTFTNYDINWVRQATGQGLEWMGWMHPNSGQTGYAQKFQGRVTLTRDTSISTAYMELSSLRSEDTAVYYCASSSGWYYFDYWGQGTLVTVSS |
| | | | SEQ ID NO: 27944 | SEQ ID NO: 31950 |
| iPS:441880 | 21-225_4A2.001.007 | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATAGTACTCCAGTCACTTTCGGCCCTGGGACCAAAGTGGGTATCAAA | CAGGTGCAGCTGGTGCAGTCTGGGACTGAGGTGAAGAAGCCTGGGGCCTCAGTGAAGGTGTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTCAAACAGGCTATGCACAGAAGTTCCAGGGCAGAGTCACCTTGACCAGGGACACCTCCATCAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTGTCCTCA |
| | | | SEQ ID NO: 27945 | SEQ ID NO: 31951 |
| | | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYSTPVTFGPGTKVGIK | QVQLVQSGTEVKKPGASVKVSCKASGYTFTNYDINWVRQATGQGLEWMGWMHPNSGQTGYAQKFQGRVTLTRDTSISTAYMELSSLRSEDTAVYYCASSSGWYYFDYWGQGTLVTVSS |
| | | | SEQ ID NO: 27946 | SEQ ID NO: 31952 |

FIGURE 50

| | | | | |
|---|---|---|---|---|
| iPS:441884 | 21-225_4A2.001.008 | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATAGTACTCCAGTCACTTTCGGCCCTGGGACCAAAGTGGGTATCAAA | CAGGTGCAGCTGGTGCAGTCTGGGACTGAGGTGAAGAAGCCTGGGGCCTCAGTGAAGGTGTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACGCAGGCTATGCACAGAAGTTCCAGGGCAGAGTCACCTTGACCAGGGACACCTCCATCAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTGTCCTCA |
| | | | SEQ ID NO: 27947 | SEQ ID NO: 31953 |
| | | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYSTPVTFGPGTKVGIK | QVQLVQSGTEVKKPGASVKVSCKASGYTFTNYDINWVRQATGQGLEWMGWMHPNSGNAGYAQKFQGRVTLTRDTSISTAYMELSSLRSEDTAVYYCASSSGWYYFDYWGQGTLVTVSS |
| | | | SEQ ID NO: 27948 | SEQ ID NO: 31954 |
| iPS:441888 | 21-225_4A2.001.009 | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATAATGCTCCAGTCACTTTCGGCCCTGGGACCAAAGTGGGTATCAAA | CAGGTGCAGCTGGTGCAGTCTGGGACTGAGGTGAAGAAGCCTGGGGCCTCAGTGAAGGTGTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTCAAACAGGCTATGCACAGAAGTTCCAGGGCAGAGTCACCTTGACCAGGGACACCTCCATCAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTGTCCTCA |
| | | | SEQ ID NO: 27949 | SEQ ID NO: 31955 |

FIGURE 50

| | | | | |
|---|---|---|---|---|
| iPS:441892 | 21-225_4A2.001.010 | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYNAPVTFGPGTKVGIK | QVQLVQSGTEVKKPGASVKVSCKASGYTFTNYDINWVRQATGQGLEWMGWMHPNSGQTGYAQKFQGRVTLTRDTSISTAYMELSSLRSEDTAVYYCASSSGWYYFDYWGQGTLVTVSS |
| | | | SEQ ID NO: 27950 | SEQ ID NO: 31956 |
| | | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATCAAACTCCAGTCACTTTCGGCCCTGGGACCAAAGTGGGTATCAAA | CAGGTGCAGCTGGTGCAGTCTGGGACTGAGGTGAAGAAGCCTGGGGCCTCAGTGAAGGTGTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACGCAGGCTATGCACAGAAGTTCCAGGGCAGAGTCACCTTGACCAGGGACACCTCCATCAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTGTCCTCA |
| | | | SEQ ID NO: 27951 | SEQ ID NO: 31957 |
| | | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYQTPVTFGPGTKVGIK | QVQLVQSGTEVKKPGASVKVSCKASGYTFTNYDINWVRQATGQGLEWMGWMHPNSGNAGYAQKFQGRVTLTRDTSISTAYMELSSLRSEDTAVYYCASSSGWYYFDYWGQGTLVTVSS |
| | | | SEQ ID NO: 27952 | SEQ ID NO: 31958 |

FIGURE 50

| | | | | |
|---|---|---|---|---|
| iPS:441896 | 21-225_4A2.001.011 | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATCAAACTCCAGTCACTTTCGGCCCTGGGACCAAAGTGGGTATCAAA | CAGGTGCAGCTGGTGCAGTCTGGGACTGAGGTGAAGAAGCCTGGGGCCTCAGTGAAGGTGTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTCAAACAGGCTATGCACAGAAGTTCCAGGGCAGAGTCACCTTGACCAGGGACACCTCCATCAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTGTCCTCA |
| | | | SEQ ID NO: 27953 | SEQ ID NO: 31959 |
| | | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYQTPVTFGPGTKVGIK | QVQLVQSGTEVKKPGASVKVSCKASGYTFTNYDINWVRQATGQGLEWMGWMHPNSGQTGYAQKFQGRVTLTRDTSISTAYMELSSLRSEDTAVYYCASSSGWYYFDYWGQGTLVTVSS |
| | | | SEQ ID NO: 27954 | SEQ ID NO: 31960 |
| iPS:441900 | 21-225_4A2.001.012 | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATCAAACTCCAGTCACTTTCGGCCCTGGGACCAAAGTGGGTATCAAA | CAGGTGCAGCTGGTGCAGTCTGGGACTGAGGTGAAGAAGCCTGGGGCCTCAGTGAAGGTGTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAGCACAGGCTATGCACAGAAGTTCCAGGGCAGAGTCACCTTGACCAGGGACACCTCCATCAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTGTCCTCA |
| | | | SEQ ID NO: 27955 | SEQ ID NO: 31961 |

FIGURE 50

| | | | | |
|---|---|---|---|---|
| | | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYQTPVTFGPGTKVGIK | QVQLVQSGTEVKKPGASVKVSCKASGYTFTNYDINWVRQATGQGLEWMGWMHPNSGSTGYAQKFQGRVTLTRDTSISTAYMELSSLRSEDTAVYYCASSSGWYYFDYWGQGTLVTVSS |
| | | | SEQ ID NO: 27956 | SEQ ID NO: 31962 |
| iPS:441955 | 21-225_4A2.001.022 | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATAGTACTCCAGTCACTTTCGGCCCTGGGACCAAAGTGGGTATCAAA | CAGGTGCAGCTGGTGCAGTCTGGGGCTGAGGTGAAGAAGCCTGGGGCCTCAGTGAAGGTGTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCTTGACCAGGGACACCTCCATCAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTGTCCTCA |
| | | | SEQ ID NO: 27957 | SEQ ID NO: 31963 |
| | | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYSTPVTFGPGTKVGIK | QVQLVQSGAEVKKPGASVKVSCKASGYTFTNYDINWVRQATGQGLEWMGWMHPNSGNTGYAQKFQGRVTLTRDTSISTAYMELSSLRSEDTAVYYCASSSGWYYFDYWGQGTLVTVSS |
| | | | SEQ ID NO: 27958 | SEQ ID NO: 31964 |

FIGURE 50

| | | | | |
|---|---|---|---|---|
| **iPS:441962** | **21-225_4A2.001.023** | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATAGTACTCCAGTCACTTTCGGCCAAGGGACCAAAGTGGAAATCAAA | CAGGTGCAGCTGGTGCAGTCTGGGGCTGAGGTGAAGAAGCCTGGGGCCTCAGTGAAGGTGTCCTGCAAGGCTTCTGGATACACCTTCACCAATTATGATATCAACTGGGTGCGACAGGCCCCTGGACAAGGGCTTGAGTGGATGGGATGGATGCACCCTAACAGTGGTAACACAGGCTATGCACAGAAGTTCCAGGGCAGAGTCACCTTGACCAGGGACACCTCCATCAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTGTCCTCA |
| | | | SEQ ID NO: 27959 | SEQ ID NO: 31965 |
| | | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYSTPVTFGQGTKVEIK | QVQLVQSGAEVKKPGASVKVSCKASGYTFTNYDINWVRQAPGQGLEWMGWMHPNSGNTGYAQKFQGRVTLTRDTSISTAYMELSSLRSEDTAVYYCASSSGWYYFDYWGQGTLVTVSS |
| | | | SEQ ID NO: 27960 | SEQ ID NO: 31966 |
| **iPS:441971** | **21-225_4A2.001.024** | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATAGTACTCCAGTCACTTTCGGCCCTGGGACCAAAGTGGGTATCAAA | CAGGTGCAGCTGGTGCAGTCTGGGGCTGAGGTGAAGAAGCCTGGGGCCTCAGTGAAGGTGTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTCAAACAGGCTATGCACAGAAGTTCCAGGGCAGAGTCACCTTGACCAGGGACACCTCCATCAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTGTCCTCA |
| | | | SEQ ID NO: 27961 | SEQ ID NO: 31967 |

FIGURE 50

| | | | | |
|---|---|---|---|---|
| | | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYSTPVTFGPGTKVGIK | QVQLVQSGAEVKKPGASVKVSCKASGYTFTNYDINWVRQATGQGLEWMGWMHPNSGQTGYAQKFQGRVTLTRDTSISTAYMELSSLRSEDTAVYYCASSSGWYYFDYWGQGTLVTVSS |
| | | | SEQ ID NO: 27962 | SEQ ID NO: 31968 |
| **iPS:441999** | **21-225_4A2.001.028** | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATAGTACTCCAGTCACTTTCGGCCAAGGGACCAAAGTGGAAATCAAA | CAGGTGCAGCTGGTGCAGTCTGGGGCTGAGGTGAAGAAGCCTGGGGCCTCAGTGAAGGTGTCCTGCAAGGCTTCTGGATACACCTTCACCAATTATGATATCAACTGGGTGCGACAGGCCCCTGGACAAGGGCTTGAGTGGATGGGATGGATGCACCCTAACAGTGGTCAAACAGGCTATGCACAGAAGTTCCAGGGCAGAGTCACCTTGACCAGGGACACCTCCATCAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTGTCCTCA |
| | | | SEQ ID NO: 27963 | SEQ ID NO: 31969 |
| | | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYSTPVTFGQGTKVEIK | QVQLVQSGAEVKKPGASVKVSCKASGYTFTNYDINWVRQAPGQGLEWMGWMHPNSGQTGYAQKFQGRVTLTRDTSISTAYMELSSLRSEDTAVYYCASSSGWYYFDYWGQGTLVTVSS |
| | | | SEQ ID NO: 27964 | SEQ ID NO: 31970 |

FIGURE 50

| | | | | |
|---|---|---|---|---|
| iPS:442006 | 21-225_4A2.001.029 | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATAGTACTCCAGTCACTTTCGGCCAAGGGACCAAAGTGGAAATCAAA | CAGGTGCAGCTGGTGCAGTCTGGGGCTGAGGTGAAGAAGCCTGGGGCCTCAGTGAAGGTGTCCTGCAAGGCTTCTGGATACACCTTCACCAATTATGATATCAACTGGGTGCGACAGGCCCCTGGACAAGGGCTTGAGTGGATGGGATGGATGCACCCTAACAGTGGTAGCACAGGCTATGCACAGAAGTTCCAGGGCAGAGTCACCTTGACCAGGGACACCTCCATCAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTGTCCTCA |
| | | | SEQ ID NO: 27965 | SEQ ID NO: 31971 |
| | | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYSTPVTFGQGTKVEIK | QVQLVQSGAEVKKPGASVKVSCKASGYTFTNYDINWVRQAPGQGLEWMGWMHPNSGSTGYAQKFQGRVTLTRDTSISTAYMELSSLRSEDTAVYYCASSSGWYYFDYWGQGTLVTVSS |
| | | | SEQ ID NO: 27966 | SEQ ID NO: 31972 |
| iPS:442020 | 21-225_4A2.001.031 | NA | GACATCGTGATGACCCAGTCTCCAGACTCCCTGGCTGTGTCTCTGGGCGAGAGGGCCACCATCAACTGCAAGTCCAGCCAGAGTATTTTACACAGCTCCAACAATAACAACTACTTAGCTTGGTTCCAGCAGAAACCAGGACAGCCTCCTAAACTGCTCCTTTACTGGGCATCTACCCGGGAATCCGGGGTCCCTGACCGATTCAGTGGCAGCGGGTCTGGGACAGATTTCACTCTCACCATCAGCAGCCTGCAGCCTGAAGATGTGGCAGTTTATTACTGTCAGCAATATTATAATACTCCAGTCACTTTCGGCCCTGGGACCAAAGTGGAAATCAAA | CAGGTGCAGCTGGTGCAGTCTGGGGGTGAGGTGAAGAAGCCTGGGGCCTCAGTGAAGGTGTCCTGCAAGGCTTCTGGATACACCTTCACCAATTATGATATCAACTGGGTGCGACAGGCCGAAGGACAAGGGCTTGAGTGGATGGGATGGATGCACCCTAACAGTGGTAACGAAGGCTATGCACAGAAGTTCCAGGGCAGAGTCACCTTGACCAGGGACACCTCCATCAGCACAGCCTACATGGAACTGAGCAGCCTGAGATCTGAGGACACGGCCGTGTATTACTGTGCGAGTAGCAGTGGCTGGTACTACTTTGACTACTGGGGCCAGGGAACCCTGGTCACCGTGTCCTCA |
| | | | SEQ ID NO: 27967 | SEQ ID NO: 31973 |

FIGURE 50

| | | | | |
|---|---|---|---|---|
| | | AA | DIVMTQSPDSLAVSLGERATINCKSSQSILHSSNNNNYLAWFQQKPGQPPKLLLYWASTRESGVPDRFSGSGSGTDFTLTISSLQPEDVAVYYCQQYYNTPVTFGPGTKVEIK | QVQLVQSGGEVKKPGASVKVSCKASGYTFTNYDINWVRQAEGQGLEWMGWMHPNSGNEGYAQKFQGRVTLTRDTSISTAYMELSSLRSEDTAVYYCASSSGWYYFDYWGQGTLVTVSS |
| | | | SEQ ID NO: 27968 | SEQ ID NO: 31974 |
| **iPS:442050** | **21-225_4H6.004** | NA | GACATCCAGATGACCCAGTCTCCATCTTCCGTGTCTGCATCTGTAGGAGACAGAGTCACCATCACTTGTCGGGCGAGTCAGGGTATTAGCAGGTGGTTAGCCTGGTATCAGCAGAAACCAGGGAAAGCCCCTAAGCTCCTGATCTATGGTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAATTTATTATTGTCAACAGGCTAACAGTTTCCCATTCACTTTCGGCCCTGGGACCAAAGTGGATATCAAA | CAGGTGCAGCTGGTACAGTCTGGGGCTGAGGTGAAGAAGCCTGGGGCCTCAGTGAAGGTCAGCTGCAAGGCTTCTGGATACACCTTCACCGACTACTATTTGCACTGGGTGCGACAGGCCCCTGGACAAGGTCTTGAGTGGATGGGATGGATCCACCCTAACAGTGGTGGCACAAACTATGCACAGAAGTTTCAGGGCAGGGTCACCATGACCAGGGACACGTCCATCAGCACAGCCTACATGGAGCTGAGCAGTCTGAGATCTGACGACACGGCCGTGTATTACTGTGCGAGAGATGGTACCAGCTCGTTTGACTACTGGGGCCAGGGAACCCTGGTCACCGTGTCCTCA |
| | | | SEQ ID NO: 27969 | SEQ ID NO: 31975 |
| | | AA | DIQMTQSPSSVSASVGDRVTITCRASQGISRWLAWYQQKPGKAPKLLIYGASSLQSGVPSRFSGSGSGTDFTLTISSLQPEDFAIYYCQQANSFPFTFGPGTKVDIK | QVQLVQSGAEVKKPGASVKVSCKASGYTFTDYYLHWVRQAPGQGLEWMGWIHPNSGGTNYAQKFQGRVTMTRDTSISTAYMELSSLRSDDTAVYYCARDGTSSFDYWGQGTLVTVSS |
| | | | SEQ ID NO: 27970 | SEQ ID NO: 31976 |

FIGURE 50

| | | | | |
|---|---|---|---|---|
| **iPS:442059** | **21-225_4H6.005** | NA | GACATCCAGATGACCCAGTCTCCATCTTCCGTGTCTGCATCTGTAGGAGATAGAGTCACCATCACTTGTCGGGCGAGTCAGGGTATTAGCAGGTGGTTAGCCTGGTATCAGCAGAAACCAGGGAAAGCCCCTAAGCTCCTGATCTATGGTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAATTTATTATTGTCAACAGGCTAACAGTTTCCCATTCACTTTCGGCCAAGGGACCAAAGTGGATATCAAA | CAGGTGCAGCTGGTACAGTCTGGGGCTGAGGTGAAGAAGCCTGGGGCCTCAGTGAAGGTCAGCTGCAAGGCTTCTGGATACACCTTCACCGACTACTATTTGCACTGGGTGCGACAGGCCCCTGGACAAGGTCTTGAGTGGATGGGATGGATCCACCCTAACAGTGGTGGCACAAACTATGCACAGAAGTTTCAGGGCAGGGTCACCATGACCAGGGACACGTCCATCAGCACAGCCTACATGGAGCTGAGCAGTCTGAGATCTGACGACACGGCCGTGTATTACTGTGCGAGAGATGGTACCAGCTCGTTTGACTACTGGGGCCAGGGAACCCTGGTCACCGTGTCCTCA |
| | | | SEQ ID NO: 27971 | SEQ ID NO: 31977 |
| | | AA | DIQMTQSPSSVSASVGDRVTITCRASQGISRWLAWYQQKPGKAPKLLIYGASSLQSGVPSRFSGSGSGTDFTLTISSLQPEDFAIYYCQQANSFPFTFGQGTKVDIK | QVQLVQSGAEVKKPGASVKVSCKASGYTFTDYYLHWVRQAPGQGLEWMGWIHPNSGGTNYAQKFQGRVTMTRDTSISTAYMELSSLRSDDTAVYYCARDGTSSFDYWGQGTLVTVSS |
| | | | SEQ ID NO: 27972 | SEQ ID NO: 31978 |
| **iPS:442065** | **21-225_4H6.006** | NA | GACATCCAGATGACCCAGTCTCCATCTTCCGTGTCTGCATCTGTAGGAGACAGAGTCACCATCACTTGTCGGGCGAGTCAGGGTATTAGCAGGTGGTTAGCCTGGTATCAGCAGAAACCAGGGAAAGCCCCTAAGCTCCTGATCTATGGTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTATTATTGTCAACAGGCTAACAGTTTCCCATTCACTTTCGGCCAAGGGACCAAAGTGGATATCAAA | CAGGTGCAGCTGGTACAGTCTGGGGCTGAGGTGAAGAAGCCTGGGGCCTCAGTGAAGGTCAGCTGCAAGGCTTCTGGATACACCTTCACCGACTACTATTTGCACTGGGTGCGACAGGCCCCTGGACAAGGTCTTGAGTGGATGGGATGGATCCACCCTAACAGTGGTGGCACAAACTATGCACAGAAGTTTCAGGGCAGGGTCACCATGACCAGGGACACGTCCATCAGCACAGCCTACATGGAGCTGAGCAGTCTGAGATCTGACGACACGGCCGTGTATTACTGTGCGAGAGATGGTACCAGCTCGTTTGACTACTGGGGCCAGGGAACCCTGGTCACCGTGTCCTCA |
| | | | SEQ ID NO: 27973 | SEQ ID NO: 31979 |

FIGURE 50

| | | | | |
|---|---|---|---|---|
| | | AA | DIQMTQSPSSVSASVGDRVTITCRASQGISRWLAWYQQKPGKAPKLLIYGASSLQSGVPSRFSGSGSGTDFTLTISSLQPEDFATYYCQQANSFPFTFGQGTKVDIK | QVQLVQSGAEVKKPGASVKVSCKASGYTFTDYYLHWVRQAPGQGLEWMGWIHPNSGGTNYAQKFQGRVTMTRDTSISTAYMELSSLRSDDTAVYYCARDGTSSFDYWGQGTLVTVSS |
| | | | SEQ ID NO: 27974 | SEQ ID NO: 31980 |
| iPS:442071 | 21-225_4H6.007 | NA | GACATCCAGATGACCCAGTCTCCATCTTCCGTGTCTGCATCTGTAGGAGACAGAGTCACCATCACTTGTCGGGCGAGTCAGGGTATTAGCAGGTGGTTAGCCTGGTATCAGCAGAAACCAGGGAAAGCCCCTAAGCTCCTGATCTATGGTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAATTTATTATTGTCAACAGGCTAACAGTTTCCCATTCACTTTCGGCCCTGGGACCAAAGTGGATATCAAA | CAGGTGCAGCTGGTACAGTCTGGGGCTGAGGTGAAGAAGCCTGGGGCCTCAGTGAAGGTCAGCTGCAAGGCTTCTGGATACACCTTCACCGACTACTATTTGCACTGGGTGCGACAGGCCCCTGGACAAGGTCTTGAGTGGATGGGATGGATCCACCCTAACAGTGGTGGCACAAACTATGCACAGAAGTTTCAGGGCAGGGTCACCATGACCAGGGACACGTCCATCAGCACAGCCTACATGGAGCTGAGCAGTCTGAGATCTGACGACACGGCCGTGTATTACTGTGCGAGAGATGCTACCAGCTCGTTTGACTACTGGGGCCAGGGAACCCTGGTCACCGTGTCCTCA |
| | | | SEQ ID NO: 27975 | SEQ ID NO: 31981 |
| | | AA | DIQMTQSPSSVSASVGDRVTITCRASQGISRWLAWYQQKPGKAPKLLIYGASSLQSGVPSRFSGSGSGTDFTLTISSLQPEDFAIYYCQQANSFPFTFGPGTKVDIK | QVQLVQSGAEVKKPGASVKVSCKASGYTFTDYYLHWVRQAPGQGLEWMGWIHPNSGGTNYAQKFQGRVTMTRDTSISTAYMELSSLRSDDTAVYYCARDATSSFDYWGQGTLVTVSS |
| | | | SEQ ID NO: 27976 | SEQ ID NO: 31982 |

FIGURE 50

| | | | | |
|---|---|---|---|---|
| iPS:442078 | 21-225_4H6.008 | NA | GACATCCAGATGACCCAGTCTCCATCTTCCGTGTCTGCATCTGTAGGAGACAGAGTCACCATCACTTGTCGGGCGAGTCAGGGTATTAGCAGGTGGTTAGCCTGGTATCAGCAGAAACCAGGGAAAGCCCCTAAGCTCCTGATCTATGGTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAATTTATTATTGTCAACAGGCTAACAGTTTCCCATTCACTTTCGGCCCTGGGACCAAAGTGGATATCAAA | CAGGTGCAGCTGGTACAGTCTGGGGCTGAGGTGAAGAAGCCTGGGGCCTCAGTGAAGGTCAGCTGCAAGGCTTCTGGATACACCTTCACCGACTACTATTTGCACTGGGTGCGACAGGCCCCTGGACAAGGTCTTGAGTGGATGGGATGGATCCACCCTAACAGTGGTGGCACAAACTATGCACAGAAGTTTCAGGGCAGGGTCACCATGACCAGGGACACGTCCATCAGCACAGCCTACATGGAGCTGAGCAGTCTGAGATCTGACGACACGGCCGTGTATTACTGTGCGAGAAGTGGTACCAGCTCGTTTGACTACTGGGGCCAGGGAACCCTGGTCACCGTGTCCTCA |
| | | | SEQ ID NO: 27977 | SEQ ID NO: 31983 |
| | | AA | DIQMTQSPSSVSASVGDRVTITCRASQGISRWLAWYQQKPGKAPKLLIYGASSLQSGVPSRFSGSGSGTDFTLTISSLQPEDFAIYYCQQANSFPFTFGPGTKVDIK | QVQLVQSGAEVKKPGASVKVSCKASGYTFTDYYLHWVRQAPGQGLEWMGWIHPNSGGTNYAQKFQGRVTMTRDTSISTAYMELSSLRSDDTAVYYCARSGTSSFDYWGQGTLVTVSS |
| | | | SEQ ID NO: 27978 | SEQ ID NO: 31984 |
| iPS:442085 | 21-225_4H6.009 | NA | GACATCCAGATGACCCAGTCTCCATCTTCCGTGTCTGCATCTGTAGGAGATAGAGTCACCATCACTTGTCGGGCGAGTCAGGGTATTAGCAGGTGGTTAGCCTGGTATCAGCAGAAACCAGGGAAAGCCCCTAAGCTCCTGATCTATGGTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAATTTATTATTGTCAACAGGCTAACAGTTTCCCATTCACTTTCGGCCAAGGGACCAAAGTGGATATCAAA | CAGGTGCAGCTGGTACAGTCTGGGGCTGAGGTGAAGAAGCCTGGGGCCTCAGTGAAGGTCAGCTGCAAGGCTTCTGGATACACCTTCACCGACTACTATTTGCACTGGGTGCGACAGGCCCCTGGACAAGGTCTTGAGTGGATGGGATGGATCCACCCTAACAGTGGTGGCACAAACTATGCACAGAAGTTTCAGGGCAGGGTCACCATGACCAGGGACACGTCCATCAGCACAGCCTACATGGAGCTGAGCAGTCTGAGATCTGACGACACGGCCGTGTATTACTGTGCGAGAAGTGGTACCAGCTCGTTTGACTACTGGGGCCAGGGAACCCTGGTCACCGTGTCCTCA |
| | | | SEQ ID NO: 27979 | SEQ ID NO: 31985 |

FIGURE 50

| | | | | |
|---|---|---|---|---|
| | | AA | DIQMTQSPSSVSASVGDRVTITCRASQGISRWLAWYQQKPGKAPKLLIYGASSLQSGVPSRFSGSGSGTDFTLTISSLQPEDFAIYYCQQANSFPFTFGQGTKVDIK | QVQLVQSGAEVKKPGASVKVSCKASGYTFTDYYLHWVRQAPGQGLEWMGWIHPNSGGTNYAQKFQGRVTMTRDTSISTAYMELSSLRSDDTAVYYCARSGTSSFDYWGQGTLVTVSS |
| | | | SEQ ID NO: 27980 | SEQ ID NO: 31986 |
| iPS:442089 | 21-225_4H6.010 | NA | GACATCCAGATGACCCAGTCTCCATCTTCCGTGTCTGCATCTGTAGGAGATAGAGTCACCATCACTTGTCGGGCGAGTCAGGGTATTAGCAGGTGGTTAGCCTGGTATCAGCAGAAACCAGGGAAAGCCCCTAAGCTCCTGATCTATGGTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAATTTATTATTGTCAACAGGCTAACAGTTTCCCATTCACTTTCGGCCAAGGGACCAAAGTGGATATCAAA | CAGGTGCAGCTGGTACAGTCTGGGGCTGAGGTGAAGAAGCCTGGGGCCTCAGTGAAGGTCAGCTGCAAGGCTTCTGGATACACCTTCACCGACTACTATTTGCACTGGGTGCGACAGGCCCCTGGACAAGGTCTTGAGTGGATGGGATGGATCCACCCTAACAGTGGTGGCACAAACTATGCACAGAAGTTTCAGGGCAGGGTCACCATGACCAGGGACACGTCCATCAGCACAGCCTACATGGAGCTGAGCAGTCTGAGATCTGACGACACGGCCGTGTATTACTGTGCGAGAGATGCTACCAGCTCGTTTGACTACTGGGGCCAGGGAACCCTGGTCACCGTGTCCTCA |
| | | | SEQ ID NO: 27981 | SEQ ID NO: 31987 |
| | | AA | DIQMTQSPSSVSASVGDRVTITCRASQGISRWLAWYQQKPGKAPKLLIYGASSLQSGVPSRFSGSGSGTDFTLTISSLQPEDFAIYYCQQANSFPFTFGQGTKVDIK | QVQLVQSGAEVKKPGASVKVSCKASGYTFTDYYLHWVRQAPGQGLEWMGWIHPNSGGTNYAQKFQGRVTMTRDTSISTAYMELSSLRSDDTAVYYCARDATSSFDYWGQGTLVTVSS |
| | | | SEQ ID NO: 27982 | SEQ ID NO: 31988 |

FIGURE 50

| | | | | |
|---|---|---|---|---|
| **iPS:442093** | **21-225_4H6.011** | NA | GACATCCAGATGACCCAGTCTCCATCTTCCGTGTCTGCATCTGTAGGAGACAGAGTCACCATCACTTGTCGGGCGAGTCAGGGTATTAGCAGGTGGTTAGCCTGGTATCAGCAGAAACCAGGGAAAGCCCCTAAGCTCCTGATCTATGGTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTATTATTGTCAACAGGCTAACAGTTTCCCATTCACTTTCGGCCAAGGGACCAAAGTGGATATCAAA | CAGGTGCAGCTGGTACAGTCTGGGGCTGAGGTGAAGAAGCCTGGGGCCTCAGTGAAGGTCAGCTGCAAGGCTTCTGGATACACCTTCACCGACTACTATTTGCACTGGGTGCGACAGGCCCCTGGACAAGGTCTTGAGTGGATGGGATGGATCCACCCTAACAGTGGTGGCACAAACTATGCACAGAAGTTTCAGGGCAGGGTCACCATGACCAGGGACACGTCCATCAGCACAGCCTACATGGAGCTGAGCAGTCTGAGATCTGACGACACGGCCGTGTATTACTGTGCGAGAGATGCTACCAGCTCGTTTGACTACTGGGGCCAGGGAACCCTGGTCACCGTGTCCTCA |
| | | | SEQ ID NO: 27983 | SEQ ID NO: 31989 |
| | | AA | DIQMTQSPSSVSASVGDRVTITCRASQGISRWLAWYQQKPGKAPKLLIYGASSLQSGVPSRFSGSGSGTDFTLTISSLQPEDFATYYCQQANSFPFTFGQGTKVDIK | QVQLVQSGAEVKKPGASVKVSCKASGYTFTDYYLHWVRQAPGQGLEWMGWIHPNSGGTNYAQKFQGRVTMTRDTSISTAYMELSSLRSDDTAVYYCARDATSSFDYWGQGTLVTVSS |
| | | | SEQ ID NO: 27984 | SEQ ID NO: 31990 |
| **iPS:442115** | **21-225_5E5.003** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTACCCTCGGACGTTCGGCCAAGGGACCAAGGTGGAAGTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGTCATGCACTGGGTCCGCCAGGCTCCAGGCAAGGGGCTGGAGTGGGTGGCAGTTATCTGGTATGATGCAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTACGAGAGAGGTATATAGCAGTGGCTGGTACGACTACGGTATGGACGTCTGGGGCCAAGGGACCACGGTCACCGTGTCCTCA |
| | | | SEQ ID NO: 27985 | SEQ ID NO: 31991 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPRTFGQGTKVEVK | QVQLVESGGGVVQPGRSLRLSCAASGFTFSNYVMHWVRQAPGKGLEWVAVIWYDASNKYYADSVKGRFTISRDNSKNTLYLQMNSLRAEDTAVYYCTREVYSSGWYDYGMDVWGQGTTVTVSS |
| | | | SEQ ID NO: 27986 | SEQ ID NO: 31992 |
| iPS:442122 | 21-225_5E5.004 | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTACCCTCGGACGTTCGGCCAAGGGACCAAGGTGGAAGTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGTCATGCACTGGGTCCGCCAGGCTCCAGGCAAGGGGCTGGAGTGGGTGGCAGTTATCTGGTATGATGGAAGTAATAAATACTATGCAGAATCCGTGAAGGGCCGATTCACCATCTCCAGAGACAATTCCAAGAACACGCTGTATCTGCAAATGAACAGCCTGAGAGCCGAGGACACGGCTGTGTATTACTGTACGAGAGAGGTATATAGCAGTGGCTGGTACGACTACGGTATGGACGTCTGGGGCCAAGGGACCACGGTCACCGTGTCCTCA |
| | | | SEQ ID NO: 27987 | SEQ ID NO: 31993 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPRTFGQGTKVEVK | QVQLVESGGGVVQPGRSLRLSCAASGFTFSNYVMHWVRQAPGKGLEWVAVIWYDGSNKYYAESVKGRFTISRDNSKNTLYLQMNSLRAEDTAVYYCTREVYSSGWYDYGMDVWGQGTTVTVSS |
| | | | SEQ ID NO: 27988 | SEQ ID NO: 31994 |

FIGURE 50

| | | | | |
|---|---|---|---|---|
| **iPS:442129** | **21-225_5E5.005** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTACCCTCGGACGTTCGGCCAAGGGACCAAGGTGGAAGTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGTCATGCACTGGGTCCGCCAGGCTCCAGGCAAGGGGCTGGAGTGGGTGGCAGTTATCTGGTATGATGGAAGTAATAAATACTATGCAGGCTCCGTGAAGGGCCGATTCACCATCTCCAGAGACAATTCCAAGAACACGCTGTATCTGCAAATGAACAGCCTGAGAGCCGAGGACACGGCTGTGTATTACTGTACGAGAGAGGTATATAGCAGTGGCTGGTACGACTACGGTATGGACGTCTGGGGCCAAGGGACCACGGTCACCGTGTCCTCA |
| | | | SEQ ID NO: 27989 | SEQ ID NO: 31995 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPRTFGQGTKVEVK | QVQLVESGGGVVQPGRSLRLSCAASGFTFSNYVMHWVRQAPGKGLEWVAVIWYDGSNKYYAGSVKGRFTISRDNSKNTLYLQMNSLRAEDTAVYYCTREVYSSGWYDYGMDVWGQGTTVTVSS |
| | | | SEQ ID NO: 27990 | SEQ ID NO: 31996 |
| **iPS:442136** | **21-225_5E5.006** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTACCCTCGGACGTTCGGCCAAGGGACCAAGGTGGAAGTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGTCATGCACTGGGTCCGCCAGGCTCCAGGCAAGGGGCTGGAGTGGGTGGCAGTTATCTGGTATGATGGAAGTAATAAATACTATGCAGACGCCGTGAAGGGCCGATTCACCATCTCCAGAGACAATTCCAAGAACACGCTGTATCTGCAAATGAACAGCCTGAGAGCCGAGGACACGGCTGTGTATTACTGTACGAGAGAGGTATATAGCAGTGGCTGGTACGACTACGGTATGGACGTCTGGGGCCAAGGGACCACGGTCACCGTGTCCTCA |
| | | | SEQ ID NO: 27991 | SEQ ID NO: 31997 |

FIGURE 50

| | | | | |
|---|---|---|---|---|
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEVK | QVQLVESGGGVVQPGRSLRLSCAASGFTFSNYVM HWVRQAPGKGLEWVAVIWYDGSNKYYADAVKG RFTISRDNSKNTLYLQMNSLRAEDTAVYYCTREVY SSGWYDYGMDVWGQGTTVTVSS |
| | | | SEQ ID NO: 27992 | SEQ ID NO: 31998 |
| **iPS:442171** | **21-225_5E5.011** | NA | GACATCCAGATGACCCAGTCTCCATCCTCCCT GTCTGCATCTGTAGGAGACAGAGTCACCATCA CTTGCCGGGCAAGTCAGGGCATTAGAAATGAT TTAGGCTGGTATCAGCAGAAACCAGGGAAAG CCCCTAAGCGCCTGATCTATGCTGCATCCAGT TTGCAAAGTGGGGTCCCATCAAGGTTCAGCGG CAGTGGATCTGGGACAGAATTCACTCTCACAA TCAGCAGCCTGCAGCCTGAAGATTTTGCAACT TATTACTGTCTACAGCATTATAGTTACCCTCGG ACGTTCGGCCAAGGGACCAAGGTGGAAGTCA AA | CAGGTGCAGCTGGTGGAGTCTGGGGGAGGCGTG GTCCAGCCTGGGAGGTCCCTGAGACTCTCCTGTG CAGCGTCTGGATTCACCTTCAGTAACTATGTCAT GCACTGGGTCCGCCAGGCTCCAGGCAAGGGGCT GGAGTGGGTGGCAGTTATCTGGTATGATGCAAGT AATAAATACTATGCAGAATCCGTGAAGGGCCGA TTCACCATCTCCAGAGACAATTCCAAGAACACGC TGTATCTGCAAATGAACAGCCTGAGAGCCGAGG ACACGGCTGTGTATTACTGTACGAGAGAGGTATA TAGCAGTGGCTGGTACGACTACGGTATGGACGTC TGGGGCCAAGGGACCACGGTCACCGTGTCCTCA |
| | | | SEQ ID NO: 27993 | SEQ ID NO: 31999 |
| | | AA | DIQMTQSPSSLSASVGDRVTITCRASQGIRNDLG WYQQKPGKAPKRLIYAASSLQSGVPSRFSGSGS GTEFTLTISSLQPEDFATYYCLQHYSYPRTFGQG TKVEVK | QVQLVESGGGVVQPGRSLRLSCAASGFTFSNYVM HWVRQAPGKGLEWVAVIWYDASNKYYAESVKGR FTISRDNSKNTLYLQMNSLRAEDTAVYYCTREVYS SGWYDYGMDVWGQGTTVTVSS |
| | | | SEQ ID NO: 27994 | SEQ ID NO: 32000 |

FIGURE 50

| | | | | |
|---|---|---|---|---|
| **iPS:442178** | **21-225_5E5.012** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTACCCTCGGACGTTCGGCCAAGGGACCAAGGTGGAAGTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGTCATGCACTGGGTCCGCCAGGCTCCAGGCAAGGGGCTGGAGTGGGTGGCAGTTATCTGGTATGATGCAAGTAATAAATACTATGCAGACGCCGTGAAGGGCCGATTCACCATCTCCAGAGACAATTCCAAGAACACGCTGTATCTGCAAATGAACAGCCTGAGAGCCGAGGACACGGCTGTGTATTACTGTACGAGAGAGGTATATAGCAGTGGCTGGTACGACTACGGTATGGACGTCTGGGGCCAAGGGACCACGGTCACCGTGTCCTCA |
| | | | SEQ ID NO: 27995 | SEQ ID NO: 32001 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPRTFGQGTKVEVK | QVQLVESGGGVVQPGRSLRLSCAASGFTFSNYVMHWVRQAPGKGLEWVAVIWYDASNKYYADAVKGRFTISRDNSKNTLYLQMNSLRAEDTAVYYCTREVYSSGWYDYGMDVWGQGTTVTVSS |
| | | | SEQ ID NO: 27996 | SEQ ID NO: 32002 |
| **iPS:442199** | **21-225_5E5.015** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTACCCTCGGACGTTCGGCCAAGGGACCAAGGTGGAAGTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGTCATGCACTGGGTCCGCCAGGCTCCAGGCAAGGGGCTGGAGTGGGTGGCAGTTATCTGGTATGATGCAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTACGAGAGAGGTATATAGCAGTGGCTATTACGACTACGGTATGGACGTCTGGGGCCAAGGGACCACGGTCACCGTGTCCTCA |
| | | | SEQ ID NO: 27997 | SEQ ID NO: 32003 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPRTFGQGTKVEVK | QVQLVESGGGVVQPGRSLRLSCAASGFTFSNYVMHWVRQAPGKGLEWVAVIWYDASNKYYADSVKGRFTISRDNSKNTLYLQMNSLRAEDTAVYYCTREVYSSGYYDYGMDVWGQGTTVTVSS |
| | | | SEQ ID NO: 27998 | SEQ ID NO: 32004 |
| **iPS:442206** | **21-225_5E5.016** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTACCCTCGGACGTTCGGCCAAGGGACCAAGGTGGAAGTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGTCATGCACTGGGTCCGCCAGGCTCCAGGCAAGGGGCTGGAGTGGGTGGCAGTTATCTGGTATGATGCAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTACGAGAGAGGTATATAGCAGTGGCTTCTACGACTACGGTATGGACGTCTGGGGCCAAGGGACCACGGTCACCGTGTCCTCA |
| | | | SEQ ID NO: 27999 | SEQ ID NO: 32005 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPRTFGQGTKVEVK | QVQLVESGGGVVQPGRSLRLSCAASGFTFSNYVMHWVRQAPGKGLEWVAVIWYDASNKYYADSVKGRFTISRDNSKNTLYLQMNSLRAEDTAVYYCTREVYSSGFYDYGMDVWGQGTTVTVSS |
| | | | SEQ ID NO: 28000 | SEQ ID NO: 32006 |

FIGURE 50

| | | | | |
|---|---|---|---|---|
| **iPS:442213** | **21-225_5E5.017** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTACCCTCGGACGTTCGGCCAAGGGACCAAGGTGGAAGTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGTCATGCACTGGGTCCGCCAGGCTCCAGGCAAGGGGCTGGAGTGGGTGGCAGTTATCTGGTATGATGGAAGTAATAAATACTATGCAGAATCCGTGAAGGGCCGATTCACCATCTCCAGAGACAATTCCAAGAACACGCTGTATCTGCAAATGAACAGCCTGAGAGCCGAGGACACGGCTGTGTATTACTGTACGAGAGAGGTATATAGCAGTGGCTATTACGACTACGGTATGGACGTCTGGGGCCAAGGGACCACGGTCACCGTGTCCTCA |
| | | | SEQ ID NO: 28001 | SEQ ID NO: 32007 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPRTFGQGTKVEVK | QVQLVESGGGVVQPGRSLRLSCAASGFTFSNYVMHWVRQAPGKGLEWVAVIWYDGSNKYYAESVKGRFTISRDNSKNTLYLQMNSLRAEDTAVYYCTREVYSSGYYDYGMDVWGQGTTVTVSS |
| | | | SEQ ID NO: 28002 | SEQ ID NO: 32008 |
| **iPS:442220** | **21-225_5E5.018** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTACCCTCGGACGTTCGGCCAAGGGACCAAGGTGGAAGTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGTCATGCACTGGGTCCGCCAGGCTCCAGGCAAGGGGCTGGAGTGGGTGGCAGTTATCTGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTACGAGAGAGGTATATAGCAGTGGCTATTACGACTACGGTATGGACGTCTGGGGCCAAGGGACCACGGTCACCGTGTCCTCA |
| | | | SEQ ID NO: 28003 | SEQ ID NO: 32009 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPRTFGQGTKVEVK | QVQLVESGGGVVQPGRSLRLSCAASGFTFSNYVMHWVRQAPGKGLEWVAVIWYDGSNKYYADSVKGRFTISRDNSKNTLYLQMNSLRAEDTAVYYCTREVYSSGYYDYGMDVWGQGTTVTVSS |
| | | | SEQ ID NO: 28004 | SEQ ID NO: 32010 |
| **iPS:442227** | **21-225_5E5.019** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTACCCTCGGACGTTCGGCCAAGGGACCAAGGTGGAAGTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGTCATGCACTGGGTCCGCCAGGCTCCAGGCAAGGGGCTGGAGTGGGTGGCAGTTATCTGGTATGATGGAAGTAATAAATACTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTACGAGAGAGGTATATAGCAGTGGCTTCTACGACTACGGTATGGACGTCTGGGGCCAAGGGACCACGGTCACCGTGTCCTCA |
| | | | SEQ ID NO: 28005 | SEQ ID NO: 32011 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPRTFGQGTKVEVK | QVQLVESGGGVVQPGRSLRLSCAASGFTFSNYVMHWVRQAPGKGLEWVAVIWYDGSNKYYADSVKGRFTISRDNSKNTLYLQMNSLRAEDTAVYYCTREVYSSGFYDYGMDVWGQGTTVTVSS |
| | | | SEQ ID NO: 28006 | SEQ ID NO: 32012 |

FIGURE 50

| | | | | |
|---|---|---|---|---|
| **iPS:442255** | **21-225_5E5.023** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTACCCTCGGACGTTCGGCCAAGGGACCAAGGTGGAAGTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGTCATGCACTGGGTCCGCCAGGCTCCAGGCAAGGGGCTGGAGTGGGTGGCAGTTATCTGGTATGATGCAAGTAATAAATACTATGCAGAATCCGTGAAGGGCCGATTCACCATCTCCAGAGACAATTCCAAGAACACGCTGTATCTGCAAATGAACAGCCTGAGAGCCGAGGACACGGCTGTGTATTACTGTACGAGAGAGGTATATAGCAGTGGCTATTACGACTACGGTATGGACGTCTGGGGCCAAGGGACCACGGTCACCGTGTCCTCA |
| | | | SEQ ID NO: 28007 | SEQ ID NO: 32013 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPRTFGQGTKVEVK | QVQLVESGGGVVQPGRSLRLSCAASGFTFSNYVMHWVRQAPGKGLEWVAVIWYDASNKYYAESVKGRFTISRDNSKNTLYLQMNSLRAEDTAVYYCTREVYSSGYYDYGMDVWGQGTTVTVSS |
| | | | SEQ ID NO: 28008 | SEQ ID NO: 32014 |
| **iPS:442262** | **21-225_5E5.024** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTACCCTCGGACGTTCGGCCAAGGGACCAAGGTGGAAGTCAAA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAACTATGTCATGCACTGGGTCCGCCAGGCTCCAGGCAAGGGGCTGGAGTGGGTGGCAGTTATCTGGTATGATGCAAGTAATAAATACTATGCAGACGCCGTGAAGGGCCGATTCACCATCTCCAGAGACAATTCCAAGAACACGCTGTATCTGCAAATGAACAGCCTGAGAGCCGAGGACACGGCTGTGTATTACTGTACGAGAGAGGTATATAGCAGTGGCTATTACGACTACGGTATGGACGTCTGGGGCCAAGGGACCACGGTCACCGTGTCCTCA |
| | | | SEQ ID NO: 28009 | SEQ ID NO: 32015 |

FIGURE 50

| | | | | |
|---|---|---|---|---|
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPRTFGQGTKVEVK | QVQLVESGGGVVQPGRSLRLSCAASGFTFSNYVMHWVRQAPGKGLEWVAVIWYDASNKYYADAVKGRFTISRDNSKNTLYLQMNSLRAEDTAVYYCTREVYSSGYYDYGMDVWGQGTTVTVSS |
| | | | SEQ ID NO: 28010 | SEQ ID NO: 32016 |
| **iPS:442269** | **21-225_5E5.025** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATTATAGTTACCCTCGGACGTTCGGCCAAGGGACCAAGGTGGAAGTCAAA | GAGGTGCAGCTGGTGGAGTCTGGGGGAGGCGTGGTCCAGCCTGGGGGGTCCCTGAGACTCTCCTGTGCAGCGTCTGGATTCACCTTCAGTAACTATGTCATGCACTGGGTCCGCCAGGCTCCAGGCAAGGGGCTGGAGTGGGTGGCAGTTATCTGGTATGATGGAAGTAATAAATACTATGCAGAATCCGTGAAGGGCCGATTCACCATCTCCAGAGACAATGCCAAGAACACGCTGTATCTGCAAATGAACAGCCTGAGAGCCGAGGACACGGCTGTGTATTACTGTACGAGAGAGGTATATAGCAGTGGCTGGTACGACTACGGTATGGACGTCTGGGGCCAAGGGACCACGGTCACCGTGTCCTCA |
| | | | SEQ ID NO: 28011 | SEQ ID NO: 32017 |
| | | AA | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPRTFGQGTKVEVK | EVQLVESGGGVVQPGGSLRLSCAASGFTFSNYVMHWVRQAPGKGLEWVAVIWYDGSNKYYAESVKGRFTISRDNAKNTLYLQMNSLRAEDTAVYYCTREVYSSGWYDYGMDVWGQGTTVTVSS |
| | | | SEQ ID NO: 28012 | SEQ ID NO: 32018 |

FIGURE 50

| | | | | |
|---|---|---|---|---|
| **iPS:442311** | **21-225_7E11.001.001** | NA | GACATCCAGATGACCCAGTCTCCATCCTCCCTGTCTGCATCTGTAGGAGACAGAGTCACCATTACTTGCCGGGCAAGTCAAAACATTATCAGCTATTTAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATGATGGAAGTAATAAATACTATGCAGACTCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28013 | SEQ ID NO: 32019 |
| | | AA | DIQMTQSPSSLSASVGDRVTITCRASQNIISYLNWYQQKPGKAPKFLIYTASSLQSGVPSRFSGSGSGTDFTLTISSLQPEDFAIYYCQQTYSTPLTFGGGTKVEIK | QVQLVESGGGVVQPGRSLRLSCAASGFTFSSFGMHWVRQAPGKGLEWVAIIWHDGSNKYYADSVKGRFTISRDNSKNTLYLQMSSLRAEDTAVYYCARDLSMGGMDVWGQGTTVTVSS |
| | | | SEQ ID NO: 28014 | SEQ ID NO: 32020 |
| **iPS:442317** | **21-225_7E11.001.002** | NA | GACATCCAGATGACCCAGTCTCCATCCTCCCTGTCTGCATCTGTAGGAGACAGAGTCACCATTACTTGCCGGGCAAGTCAAAACATTATCAGCTATTTAAATTGGTATCAACAGAAACCAGGGAAAGCCCCTAAACTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATGATGGAAGTAATAAATACTATGCAGACTCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28015 | SEQ ID NO: 32021 |

FIGURE 50

| | | | | |
|---|---|---|---|---|
| | | AA | DIQMTQSPSSLSASVGDRVTITCRASQNIISYLNWYQQKPGKAPKLLIYTASSLQSGVPSRFSGSGSGTDFTLTISSLQPEDFATYYCQQTYSTPLTFGGGTKVEIK | QVQLVESGGGVVQPGRSLRLSCAASGFTFSSFGMHWVRQAPGKGLEWVAIIWHDGSNKYYADSVKGRFTISRDNSKNTLYLQMSSLRAEDTAVYYCARDLSMGGMDVWGQGTTVTVSS |
| | | | SEQ ID NO: 28016 | SEQ ID NO: 32022 |
| iPS:442323 | 21-225_7E11.001.003 | NA | GACATCCAGATGACCCAGTCTCCATCCTCCCTGTCTGCATCTGTAGGAGACAGAGTCACCATTGCTTGCCGGGCAAGTCAAAACATTATCAGCTATTTAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATAGTGGAAGTAATAAATACTATGCAGACTCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28017 | SEQ ID NO: 32023 |
| | | AA | DIQMTQSPSSLSASVGDRVTIACRASQNIISYLNWYQQKPGKAPKFLIYTASSLQSGVPSRFSGSGSGTDFTLTISSLQPEDFAIYYCQQTYSTPLTFGGGTKVEIK | QVQLVESGGGVVQPGRSLRLSCAASGFTFSSFGMHWVRQAPGKGLEWVAIIWHSGSNKYYADSVKGRFTISRDNSKNTLYLQMSSLRAEDTAVYYCARDLSMGGMDVWGQGTTVTVSS |
| | | | SEQ ID NO: 28018 | SEQ ID NO: 32024 |

FIGURE 50

| | | | | |
|---|---|---|---|---|
| **iPS:442330** | **21-225_7E11.001.004** | NA | GACATCCAGATGACCCAGTCTCCATCCTCCCTGTCTGCATCTGTAGGAGACAGAGTCACCATTGCTTGCCGGGCAAGTCAAAACATTATCAGCTATTTAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATGAAGGAAGTAATAAATACTATGCAGACTCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28019 | SEQ ID NO: 32025 |
| | | AA | DIQMTQSPSSLSASVGDRVTIACRASQNIISYLNWYQQKPGKAPKFLIYTASSLQSGVPSRFSGSGSGTDFTLTISSLQPEDFAIYYCQQTYSTPLTFGGGTKVEIK | QVQLVESGGGVVQPGRSLRLSCAASGFTFSSFGMHWVRQAPGKGLEWVAIIWHEGSNKYYADSVKGRFTISRDNSKNTLYLQMSSLRAEDTAVYYCARDLSMGGMDVWGQGTTVTVSS |
| | | | SEQ ID NO: 28020 | SEQ ID NO: 32026 |
| **iPS:442337** | **21-225_7E11.001.005** | NA | GACATCCAGATGACCCAGTCTCCATCCTCCCTGTCTGCATCTGTAGGAGACAGAGTCACCATTGCTTGCCGGGCAAGTCAAAACATTATCAGCTATTTAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATGATGCAAGTAATAAATACTATGCAGACTCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28021 | SEQ ID NO: 32027 |

FIGURE 50

| | | | | |
|---|---|---|---|---|
| | | AA | DIQMTQSPSSLSASVGDRVTIACRASQNIISYLNWYQQKPGKAPKFLIYTASSLQSGVPSRFSGSGSGTDFTLTISSLQPEDFAIYYCQQTYSTPLTFGGGTKVEIK | QVQLVESGGGVVQPGRSLRLSCAASGFTFSSFGMHWVRQAPGKGLEWVAIIWHDASNKYYADSVKGRFTISRDNSKNTLYLQMSSLRAEDTAVYYCARDLSMGGMDVWGQGTTVTVSS |
| | | | SEQ ID NO: 28022 | SEQ ID NO: 32028 |
| iPS:442344 | 21-225_7E11.001.006 | NA | GACATCCAGATGACCCAGTCTCCATCCTCCCTGTCTGCATCTGTAGGAGACAGAGTCACCATTGCTTGCCGGGCAAGTCAAAACATTATCAGCTATTTAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATGATGGAAGTAATAAATACTATGCAGAATCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28023 | SEQ ID NO: 32029 |
| | | AA | DIQMTQSPSSLSASVGDRVTIACRASQNIISYLNWYQQKPGKAPKFLIYTASSLQSGVPSRFSGSGSGTDFTLTISSLQPEDFAIYYCQQTYSTPLTFGGGTKVEIK | QVQLVESGGGVVQPGRSLRLSCAASGFTFSSFGMHWVRQAPGKGLEWVAIIWHDGSNKYYAESVKGRFTISRDNSKNTLYLQMSSLRAEDTAVYYCARDLSMGGMDVWGQGTTVTVSS |
| | | | SEQ ID NO: 28024 | SEQ ID NO: 32030 |

FIGURE 50

| | | | | |
|---|---|---|---|---|
| **iPS:442351** | **21-225_7E11.001.007** | NA | GACATCCAGATGACCCAGTCTCCATCCTCCCTGTCTGCATCTGTAGGAGACAGAGTCACCATTGCTTGCCGGGCAAGTCAAAACATTATCAGCTATTTAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATGATGGAAGTAATAAATACTATGCAGACGCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28025 | SEQ ID NO: 32031 |
| | | AA | DIQMTQSPSSLSASVGDRVTIACRASQNIISYLNWYQQKPGKAPKFLIYTASSLQSGVPSRFSGSGSGTDFTLTISSLQPEDFAIYYCQQTYSTPLTFGGGTKVEIK | QVQLVESGGGVVQPGRSLRLSCAASGFTFSSFGMHWVRQAPGKGLEWVAIIWHDGSNKYYADAVKGRFTISRDNSKNTLYLQMSSLRAEDTAVYYCARDLSMGGMDVWGQGTTVTVSS |
| | | | SEQ ID NO: 28026 | SEQ ID NO: 32032 |
| **iPS:442358** | **21-225_7E11.001.008** | NA | GACATCCAGATGACCCAGTCTCCATCCTCCCTGTCTGCATCTGTAGGAGACAGAGTCACCATTGCTTGCCGGGCAAGTCAAAACATTATCAGCTATTTAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATGAAGGAAGTAATAAATACTATGCAGACGCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28027 | SEQ ID NO: 32033 |

FIGURE 50

| | | | | |
|---|---|---|---|---|
| | | AA | DIQMTQSPSSLSASVGDRVTIACRASQNIISYLN WYQQKPGKAPKFLIYTASSLQSGVPSRFSGSGSG TDFTLTISSLQPEDFAIYYCQQTYSTPLTFGGGTK VEIK | QVQLVESGGGVVQPGRSLRLSCAASGFTFSSFGMH WVRQAPGKGLEWVAIIWHEGSNKYYADAVKGRF TISRDNSKNTLYLQMSSLRAEDTAVYYCARDLSMG GMDVWGQGTTVTVSS |
| | | | SEQ ID NO: 28028 | SEQ ID NO: 32034 |
| iPS:442365 | 21-225_7E11.001.009 | NA | GACATCCAGATGACCCAGTCTCCATCCTCCCT GTCTGCATCTGTAGGAGACAGAGTCACCATTG CTTGCCGGGCAAGTCAAAACATTATCAGCTAT TTAAATTGGTATCAACAGAAACCAGGGAAAG CCCCTAAATTCCTGATCTATACTGCATCCAGTT TGCAAAGTGGGGTCCCATCAAGGTTCAGTGGC AGTGGATCTGGGACAGATTTCACTCTCACCAT CAGCAGTCTGCAACCTGAAGATTTTGCAATTT ACTACTGTCAACAGACTTACAGTACCCCGCTC ACTTTCGGCGGCGGGACCAAGGTGGAGATCA AA | CAGGTGCAGCTGGTGGAGTCTGGGGGAGGCGTG GTCCAGCCTGGGAGGTCCCTGCGACTCTCCTGTG CAGCGTCTGGATTCACCTTCAGTAGCTTTGGCAT GCACTGGGTCCGCCAGGCTCCAGGCAAGGGGCT GGAGTGGGTGGCAATTATCTGGCATGAAGGAAG TAATAAATACTATGCAGAATCCGTGAAGGGCCG ATTCACCATCTCCAGAGACAATTCCAAAAACACG CTGTATCTGCAAATGAGCAGCCTGCGAGCCGAG GACACGGCTGTGTATTACTGTGCGAGAGATCTGA GTATGGGCGGTATGGACGTCTGGGGCCAAGGGA CCACGGTCACCGTGTCCTCA |
| | | | SEQ ID NO: 28029 | SEQ ID NO: 32035 |
| | | AA | DIQMTQSPSSLSASVGDRVTIACRASQNIISYLN WYQQKPGKAPKFLIYTASSLQSGVPSRFSGSGSG TDFTLTISSLQPEDFAIYYCQQTYSTPLTFGGGTK VEIK | QVQLVESGGGVVQPGRSLRLSCAASGFTFSSFGMH WVRQAPGKGLEWVAIIWHEGSNKYYAESVKGRFT ISRDNSKNTLYLQMSSLRAEDTAVYYCARDLSMGG MDVWGQGTTVTVSS |
| | | | SEQ ID NO: 28030 | SEQ ID NO: 32036 |

FIGURE 50

| | | | | |
|---|---|---|---|---|
| **iPS:442372** | **21-225_7E11.001.010** | NA | GACATCCAGATGACCCAGTCTCCATCCTCCCTGTCTGCATCTGTAGGAGACAGAGTCACCATTGCTTGCCGGGCAAGTCAAAACATTATCAGCTATTTAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATAGTGGAAGTAATAAATACTATGCAGAATCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28031 | SEQ ID NO: 32037 |
| | | AA | DIQMTQSPSSLSASVGDRVTIACRASQNIISYLNWYQQKPGKAPKFLIYTASSLQSGVPSRFSGSGSGTDFTLTISSLQPEDFAIYYCQQTYSTPLTFGGGTKVEIK | QVQLVESGGGVVQPGRSLRLSCAASGFTFSSFGMHWVRQAPGKGLEWVAIIWHSGSNKYYAESVKGRFTISRDNSKNTLYLQMSSLRAEDTAVYYCARDLSMGGMDVWGQGTTVTVSS |
| | | | SEQ ID NO: 28032 | SEQ ID NO: 32038 |
| **iPS:442379** | **21-225_7E11.001.011** | NA | GACATCCAGATGACCCAGTCTCCATCCTCCCTGTCTGCATCTGTAGGAGACAGAGTCACCATTGCTTGCCGGGCAAGTCAAAACATTATCAGCTATTTAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATAGTGGAAGTAATAAATACTATGCAGACGCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28033 | SEQ ID NO: 32039 |

FIGURE 50

| | | | | |
|---|---|---|---|---|
| | | AA | DIQMTQSPSSLSASVGDRVTIACRASQNIISYLNWYQQKPGKAPKFLIYTASSLQSGVPSRFSGSGSGTDFTLTISSLQPEDFAIYYCQQTYSTPLTFGGGTKVEIK | QVQLVESGGGVVQPGRSLRLSCAASGFTFSSFGMHWVRQAPGKGLEWVAIIWHSGSNKYYADAVKGRFTISRDNSKNTLYLQMSSLRAEDTAVYYCARDLSMGGMDVWGQGTTVTVSS |
| | | | SEQ ID NO: 28034 | SEQ ID NO: 32040 |
| iPS:442386 | 21-225_7E11.001.012 | NA | GACATCCAGATGACCCAGTCTCCATCCTCCCTGTCTGCATCTGTAGGAGACAGAGTCACCATTACTTGCCGGGCAAGTCAAAACATTATCAGCTATTTAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATAGTGGAAGTAATAAATACTATGCAGACTCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28035 | SEQ ID NO: 32041 |
| | | AA | DIQMTQSPSSLSASVGDRVTITCRASQNIISYLNWYQQKPGKAPKFLIYTASSLQSGVPSRFSGSGSGTDFTLTISSLQPEDFAIYYCQQTYSTPLTFGGGTKVEIK | QVQLVESGGGVVQPGRSLRLSCAASGFTFSSFGMHWVRQAPGKGLEWVAIIWHSGSNKYYADSVKGRFTISRDNSKNTLYLQMSSLRAEDTAVYYCARDLSMGGMDVWGQGTTVTVSS |
| | | | SEQ ID NO: 28036 | SEQ ID NO: 32042 |

FIGURE 50

| | | | | |
|---|---|---|---|---|
| **iPS:442390** | **21-225_7E11.001.013** | NA | GACATCCAGATGACCCAGTCTCCATCCTCCCTGTCTGCATCTGTAGGAGACAGAGTCACCATTACTTGCCGGGCAAGTCAAAACATTATCAGCTATTTAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATGATGGAAGTAATAAATACTATGCAGAATCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28037 | SEQ ID NO: 32043 |
| | | AA | DIQMTQSPSSLSASVGDRVTITCRASQNIISYLNWYQQKPGKAPKFLIYTASSLQSGVPSRFSGSGSGTDFTLTISSLQPEDFAIYYCQQTYSTPLTFGGGTKVEIK | QVQLVESGGGVVQPGRSLRLSCAASGFTFSSFGMHWVRQAPGKGLEWVAIIWHDGSNKYYAESVKGRFTISRDNSKNTLYLQMSSLRAEDTAVYYCARDLSMGGMDVWGQGTTVTVSS |
| | | | SEQ ID NO: 28038 | SEQ ID NO: 32044 |
| **iPS:442394** | **21-225_7E11.001.014** | NA | GACATCCAGATGACCCAGTCTCCATCCTCCCTGTCTGCATCTGTAGGAGACAGAGTCACCATTACTTGCCGGGCAAGTCAAAACATTATCAGCTATTTAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATAGTGGAAGTAATAAATACTATGCAGAATCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28039 | SEQ ID NO: 32045 |

FIGURE 50

| | | | | |
|---|---|---|---|---|
| | | AA | DIQMTQSPSSLSASVGDRVTITCRASQNIISYLNWYQQKPGKAPKFLIYTASSLQSGVPSRFSGSGSGTDFTLTISSLQPEDFAIYYCQQTYSTPLTFGGGTKVEIK | QVQLVESGGGVVQPGRSLRLSCAASGFTFSSFGMHWVRQAPGKGLEWVAIIWHSGSNKYYAESVKGRFTISRDNSKNTLYLQMSSLRAEDTAVYYCARDLSMGGMDVWGQGTTVTVSS |
| | | | SEQ ID NO: 28040 | SEQ ID NO: 32046 |
| **iPS:442398** | **21-225_7E11.001.015** | NA | GACATCCAGATGACCCAGTCTCCATCCTCCCTGTCTGCATCTGTAGGAGACAGAGTCACCATTACTTGCCGGGCAAGTCAAAACATTATCAGCTATTTAAATTGGTATCAACAGAAACCAGGGAAAGCCCCTAAACTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATAGTGGAAGTAATAAATACTATGCAGACTCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28041 | SEQ ID NO: 32047 |
| | | AA | DIQMTQSPSSLSASVGDRVTITCRASQNIISYLNWYQQKPGKAPKLLIYTASSLQSGVPSRFSGSGSGTDFTLTISSLQPEDFATYYCQQTYSTPLTFGGGTKVEIK | QVQLVESGGGVVQPGRSLRLSCAASGFTFSSFGMHWVRQAPGKGLEWVAIIWHSGSNKYYADSVKGRFTISRDNSKNTLYLQMSSLRAEDTAVYYCARDLSMGGMDVWGQGTTVTVSS |
| | | | SEQ ID NO: 28042 | SEQ ID NO: 32048 |

FIGURE 50

| | | | | |
|---|---|---|---|---|
| **iPS:442402** | **21-225_7E11.001.016** | NA | GACATCCAGATGACCCAGTCTCCATCCTCCCTGTCTGCATCTGTAGGAGACAGAGTCACCATTACTTGCCGGGCAAGTCAAAACATTATCAGCTATTTAAATTGGTATCAACAGAAACCAGGGAAAGCCCCTAAACTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATAGTGGAAGTAATAAATACTATGCAGAATCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28043 | SEQ ID NO: 32049 |
| | | AA | DIQMTQSPSSLSASVGDRVTITCRASQNIISYLNWYQQKPGKAPKLLIYTASSLQSGVPSRFSGSGSGTDFTLTISSLQPEDFATYYCQQTYSTPLTFGGGTKVEIK | QVQLVESGGGVVQPGRSLRLSCAASGFTFSSFGMHWVRQAPGKGLEWVAIIWHSGSNKYYAESVKGRFTISRDNSKNTLYLQMSSLRAEDTAVYYCARDLSMGGMDVWGQGTTVTVSS |
| | | | SEQ ID NO: 28044 | SEQ ID NO: 32050 |
| **iPS:442406** | **21-225_7E11.001.017** | NA | GACATCCAGATGACCCAGTCTCCATCCTCCCTGTCTGCATCTGTAGGAGACAGAGTCACCATTACTTGCCGGGCAAGTCAAAACATTATCAGCTATTTAAATTGGTATCAACAGAAACCAGGGAAAGCCCCTAAACTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATGAAGGAAGTAATAAATACTATGCAGACGCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28045 | SEQ ID NO: 32051 |

FIGURE 50

| | | | | |
|---|---|---|---|---|
| | | AA | DIQMTQSPSSLSASVGDRVTITCRASQNIISYLNWYQQKPGKAPKLLIYTASSLQSGVPSRFSGSGSGTDFTLTISSLQPEDFATYYCQQTYSTPLTFGGGTKVEIK | QVQLVESGGGVVQPGRSLRLSCAASGFTFSSFGMHWVRQAPGKGLEWVAIIWHEGSNKYYADAVKGRFTISRDNSKNTLYLQMSSLRAEDTAVYYCARDLSMGGMDVWGQGTTVTVSS |
| | | | SEQ ID NO: 28046 | SEQ ID NO: 32052 |
| **iPS:442410** | **21-225_7E11.001.018** | NA | GACATCCAGATGACCCAGTCTCCATCCTCCCTGTCTGCATCTGTAGGAGACAGAGTCACCATTACTTGCCGGGCAAGTCAAAACATTATCAGCTATTTAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATGATGCAAGTAATAAATACTATGCAGAATCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28047 | SEQ ID NO: 32053 |
| | | AA | DIQMTQSPSSLSASVGDRVTITCRASQNIISYLNWYQQKPGKAPKFLIYTASSLQSGVPSRFSGSGSGTDFTLTISSLQPEDFAIYYCQQTYSTPLTFGGGTKVEIK | QVQLVESGGGVVQPGRSLRLSCAASGFTFSSFGMHWVRQAPGKGLEWVAIIWHDASNKYYAESVKGRFTISRDNSKNTLYLQMSSLRAEDTAVYYCARDLSMGGMDVWGQGTTVTVSS |
| | | | SEQ ID NO: 28048 | SEQ ID NO: 32054 |

FIGURE 50

| | | | | |
|---|---|---|---|---|
| **iPS:442417** | **21-225_7E11.001.019** | NA | GACATCCAGATGACCCAGTCTCCATCCTCCCTGTCTGCATCTGTAGGAGACAGAGTCACCATTGCTTGCCGGGCAAGTCAAAACATTATCAGCTATTTAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | GAGGTGCAGCTGGTGGAGTCTGGGGGAGGCGTGGTCCAGCCTGGGGGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATGATGGAAGTAATAAATACTATGCAGAATCCGTGAAGGGCCGATTCACCATCTCCAGAGACAATGCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28049 | SEQ ID NO: 32055 |
| | | AA | DIQMTQSPSSLSASVGDRVTIACRASQNIISYLNWYQQKPGKAPKFLIYTASSLQSGVPSRFSGSGSGTDFTLTISSLQPEDFAIYYCQQTYSTPLTFGGGTKVEIK | EVQLVESGGGVVQPGGSLRLSCAASGFTFSSFGMHWVRQAPGKGLEWVAIIWHDGSNKYYAESVKGRFTISRDNAKNTLYLQMSSLRAEDTAVYYCARDLSMGGMDVWGQGTTVTVSS |
| | | | SEQ ID NO: 28050 | SEQ ID NO: 32056 |
| **iPS:442431** | **21-225_7E11.001.021** | NA | GACATCCAGATGACCCAGTCTCCATCCTCCCTGTCTGCATCTGTAGGAGACAGAGTCACCATTGCTTGCCGGGCAAGTCAAAACATTATCAGCTATTTAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | GAGGTGCAGCTGGTGGAGTCTGGGGGAGGCGTGGTCCAGCCTGGGGGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATAGTGGAAGTAATAAATACTATGCAGAATCCGTGAAGGGCCGATTCACCATCTCCAGAGACAATGCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28051 | SEQ ID NO: 32057 |

FIGURE 50

| | | | | |
|---|---|---|---|---|
| | | AA | DIQMTQSPSSLSASVGDRVTIACRASQNIISYLNWYQQKPGKAPKFLIYTASSLQSGVPSRFSGSGSGTDFTLTISSLQPEDFAIYYCQQTYSTPLTFGGGTKVEIK | EVQLVESGGGVVQPGGSLRLSCAASGFTFSSFGMHWVRQAPGKGLEWVAIIWHSGSNKYYAESVKGRFTISRDNAKNTLYLQMSSLRAEDTAVYYCARDLSMGGMDVWGQGTTVTVSS |
| | | | SEQ ID NO: 28052 | SEQ ID NO: 32058 |
| iPS:442438 | 21-225_7E11.001.022 | NA | GACATCCAGATGACCCAGTCTCCATCCTCCCTGTCTGCATCTGTAGGAGACAGAGTCACCATTACTTGCCGGGCAAGTCAAAACATTATCAGCTATTTAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | GAGGTGCAGCTGGTGGAGTCTGGGGGAGGCGTGGTCCAGCCTGGGGGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATGAAGGAAGTAATAAATACTATGCAGAATCCGTGAAGGGCCGATTCACCATCTCCAGAGACAATGCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28053 | SEQ ID NO: 32059 |
| | | AA | DIQMTQSPSSLSASVGDRVTITCRASQNIISYLNWYQQKPGKAPKFLIYTASSLQSGVPSRFSGSGSGTDFTLTISSLQPEDFAIYYCQQTYSTPLTFGGGTKVEIK | EVQLVESGGGVVQPGGSLRLSCAASGFTFSSFGMHWVRQAPGKGLEWVAIIWHEGSNKYYAESVKGRFTISRDNAKNTLYLQMSSLRAEDTAVYYCARDLSMGGMDVWGQGTTVTVSS |
| | | | SEQ ID NO: 28054 | SEQ ID NO: 32060 |

FIGURE 50

| | | | | |
|---|---|---|---|---|
| **iPS:442568** | **21-225_149D8** | NA | GAAATTGTGTTGACGCAGTCTCCAGGCACCCTGTCTTTGTTTCCAGGGGAAAGAGCCACCCTCTCCTGCAGGGCCAGTCAGAGTGTGATCAGCAGCTACTTAGCCTGGTACCAGCAGAAACCTGGCCAGGCTCCCAGGCTCCTCATCTTTGGTGTATCTAGTTGGGCCACTGGCATCCCAGACAGGTTCAGTGGCAGTGGGTCTGGGACAGACTTCACTCTCACCATCAGCAGACTGGAGCCTGAAGATTTTGCAGTGTATTACTGTCAACAATATGGTAGGTCACCATTCAATTTCGGCCCTGGGACCAAAGTGGATATCAAA | CAGGTGCAGCTGCAGGAGTCGGGCCCAGGACTGGTGAAGCCTTCACAGACCCTGTCCCTCAATTGCACTGTCTCTGGTGGCTCCATCAGCAACAGTGGTTACTACTGGAGCTGGATCCGCCAGCACCCAGGGAAGGGCCTGGAATGGATTGGGTACAGCTATTACAGTGGGAGCACCTACTACAACCCGTCCCTCAAGAGTCGAATTACCATATCAGTAGACACGTCTAACAACCAGTTCTCCCTGAAGCTGAGCTCTGTGACTGCCGCGGACACGGCCGTGTATTACTGTGCGAGAGGGGGATATAACTGGAACCATGCTTTTGATATCTGGGGCCAAGGGACAATGGTCACCGTCTCTTCA |
| | | | SEQ ID NO: 28055 | SEQ ID NO: 32061 |
| | | AA | EIVLTQSPGTLSLFPGERATLSCRASQSVISSYLAWYQQKPGQAPRLLIFGVSSWATGIPDRFSGSGSGTDFTLTISRLEPEDFAVYYCQQYGRSPFNFGPGTKVDIK | QVQLQESGPGLVKPSQTLSLNCTVSGGSISNSGYYWSWIRQHPGKGLEWIGYSYYSGSTYYNPSLKSRITISVDTSNNQFSLKLSSVTAADTAVYYCARGGYNWNHAFDIWGQGTMVTVSS |
| | | | SEQ ID NO: 28056 | SEQ ID NO: 32062 |
| **iPS:443003** | **21-225_43F11_LC2** | NA | CAGTCTGTGCTGACGCAGCCGCCCTCAGTGTCTGGGGCCCCAGGGCAGAGGGTCACCATCTCCTGCACTGGGAGCAGCTCCAACATCGGGGCAGGTTATGATGTACACTGGTACCAGCAGCTTCCAGGAACAGCCCCCAAACTCCTCATCTATGGTAACAGCAATCGGCCCTCAGGGGTCCCTGACCGATTCTCTGGCTCCAAGTCTGGCACCTCAGCCTCCCTGGCCATCACTGGGCTCCAGGCTGAGGATGAGGCTGATTATTACTGCCAGTCCTATGACAACAGCCTGAGTGGTTCGGTATTCGGCGGAGGGACCAAGCTGACCGTCCTA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATACACTGGGTGCGACAGGCCCCTGGACAAGGCCTTGAGTGGATGGGATGGATCAACCCTAACAGTGGTGGCACAAACTATGCACAGAAGTTTCAGGGCGGGGTCACCATGACCAGGCTCACGTCCATCAACACAGCCTACATGGACCTGAGCAGGCTGAGATCTGACGACACGGCCGTGTATTACTGTGCGAGAGGAGGGAATTACTTCTACAACCACGTTATGGACGTCTGGGGCCAAGGGACCCCGGTCACCGTCTCCTCA |
| | | | SEQ ID NO: 28057 | SEQ ID NO: 32063 |

FIGURE 50

| | | | | |
|---|---|---|---|---|
| | | AA | QSVLTQPPSVSGAPGQRVTISCTGSSSNIGAGYDVHWYQQLPGTAPKLLIYGNSNRPSGVPDRFSGSKSGTSASLAITGLQAEDEADYYCQSYDNSLSGSVFGGGTKLTVL | QVQLVQSGAEVKKPGASVKVSCKASGYTFTGYYIHWVRQAPGQGLEWMGWINPNSGGTNYAQKFQGGVTMTRLTSINTAYMDLSRLRSDDTAVYYCARGGNYFYNHVMDVWGQGTPVTVSS |
| | | | SEQ ID NO: 28058 | SEQ ID NO: 32064 |
| **iPS:443005** | **21-225_43F11_LC1** | NA | GATGTTGTGATGACTCAGTCTCCACTCTCCCTGCCCGTCACCCTTGGACAGCCGGCCTCCATCTCCTGCAGGTCTAGTCAAAGCCTCGTATACAGTGATGGAAACACCTACTTGAATTGGTTTCAGCAGAGGCCAGGCCAATCTCCAAGGCGCCTAATTTATAAGGTTTCTAACTGGGACTCTGGGGTCCCAGACAGATTCAGCGGCAGTGGGTCAGGCACTGATTTCACACTGAAAATCAGCAGGGTGGAGGCTGAGGATGTTGGGGTTTATTACTGCATGCAAGGTACACACTGGCCGCTCACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCGGCTACTATATACACTGGGTGCGACAGGCCCCTGGACAAGGCCTTGAGTGGATGGGATGGATCAACCCTAACAGTGGTGGCACAAACTATGCACAGAAGTTTCAGGGCGGGGTCACCATGACCAGGCTCACGTCCATCAACACAGCCTACATGGACCTGAGCAGGCTGAGATCTGACGACACGGCCGTGTATTACTGTGCGAGAGGAGGGAATTACTTCTACAACCACGTTATGGACGTCTGGGGCCAAGGGACCCCGGTCACCGTCTCCTCA |
| | | | SEQ ID NO: 28059 | SEQ ID NO: 32065 |
| | | AA | DVVMTQSPLSLPVTLGQPASISCRSSQSLVYSDGNTYLNWFQQRPGQSPRRLIYKVSNWDSGVPDRFSGSGSGTDFTLKISRVEAEDVGVYYCMQGTHWPLTFGGGTKVEIK | QVQLVQSGAEVKKPGASVKVSCKASGYTFTGYYIHWVRQAPGQGLEWMGWINPNSGGTNYAQKFQGGVTMTRLTSINTAYMDLSRLRSDDTAVYYCARGGNYFYNHVMDVWGQGTPVTVSS |
| | | | SEQ ID NO: 28060 | SEQ ID NO: 32066 |

FIGURE 50

| | | | | |
|---|---|---|---|---|
| **iPS:443006** | **21-225_25A4.001.029** | NA | GACATCGTGATGACCCAGTTTCCAGACTCCCTGGCTGTGTCTCTGGGCGAGAGGGCCACCATCAAGTGTAAGTCCAGCCAGAGTGTTTTATACAGCTCCCACAATAACAACTACTTAGCTTGGTACCAGCAGAAACCAGGACAGCCTCCTAAGTTGCTCCTTTACTGGGCATCTACCCGGGAATCCGGGGTCCCTGACCGATTCAGTGGCAGCGGGTCTGGGACAGAATTCACTCTCACCATCAGCAGCCTGCAGGCTGAAGATGTGGCAGTTTATTACTGTCAGCAGTATTATAGTACTCCTCCGACGTTCGGCCAAGGGACCAAGGTGGAAATCAAA | CAGGTGCTCCTGGTGCAGTCTGGGGCTGAGGTGAAGAGGCCTGGGGCCTCAGTGAAGGTCAGCTGCAAGGCTTCTGGATACACCTTCACCAATTATGATATTAATTGGGTGCGACAGGCCACTGGACAAGGGCTTGAGTGGATGGGATGGATGTACCCTAACAGTGGTCAAACAGGCTATGCACAGAAATTCCAGGGCAGAGTCACCATGACCAGGGACACCTCCATCAGCACAGCCTACATGGAGCTGAGCAGCCTGAGATCTGAGGACACGGCCGTCTATTACTGTGCGAGTAGCAGTGGCTGGTACTACTTTGACTACTGGGGCCAGGGAACCCTGGTCACCGTGTCCTCA |
| | | | SEQ ID NO: 28061 | SEQ ID NO: 32067 |
| | | AA | DIVMTQFPDSLAVSLGERATIKCKSSQSVLYSSHNNNYLAWYQQKPGQPPKLLLYWASTRESGVPDRFSGSGSGTEFTLTISSLQAEDVAVYYCQQYYSTPPTFGQGTKVEIK | QVLLVQSGAEVKRPGASVKVSCKASGYTFTNYDINWVRQATGQGLEWMGWMYPNSGQTGYAQKFQGRVTMTRDTSISTAYMELSSLRSEDTAVYYCASSSGWYYFDYWGQGTLVTVSS |
| | | | SEQ ID NO: 28062 | SEQ ID NO: 32068 |
| **iPS:443016** | **21-225_4H6.014** | NA | GACATCCAGATGACCCAGTCTCCATCTTCCGTGTCTGCATCTGTAGGAGACAGAGTCACCATCACTTGTCGGGCGAGTCAGGGTATTAGCAGGTGGTTAGCCTGGTATCAGCAGAAACCAGGGAAAGCCCCTAAGCTCCTGATCTATGGTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTATTATTGTCAACAGGCTAACAGTTTCCCATTCACTTTCGGCCCTGGGACCAAAGTGGATATCAAA | CAGGTGCAGCTGGTACAGTCTGGGGCTGAGGTGAAGAAGCCTGGGGCCTCAGTGAAGGTCAGCTGCAAGGCTTCTGGATACACCTTCACCGACTACTATTTGCACTGGGTGCGACAGGCCCCTGGACAAGGTCTTGAGTGGATGGGATGGATCCACCCTAACAGTGGTGGCACAAACTATGCACAGAAGTTTCAGGGCAGGGTCACCATGACCAGGGACACGTCCATCAGCACAGCCTACATGGGGCTGAGCAGTCTGAGATCTGACGACACGGCCGTGTATTACTGTGCGAGAGATGCTACCAGCTCGTTTGACTACTGGGGCCAGGGAACCCTGGTCACCGTGTCCTCA |
| | | | SEQ ID NO: 28063 | SEQ ID NO: 32069 |

FIGURE 50

| | | | | |
|---|---|---|---|---|
| | | AA | DIQMTQSPSSVSASVGDRVTITCRASQGISRWLAWYQQKPGKAPKLLIYGASSLQSGVPSRFSGSGSGTDFTLTISSLQPEDFATYYCQQANSFPFTFGPGTKVDIK | QVQLVQSGAEVKKPGASVKVSCKASGYTFTDYYLHWVRQAPGQGLEWMGWIHPNSGGTNYAQKFQGRVTMTRDTSISTAYMGLSSLRSDDTAVYYCARDATSSFDYWGQGTLVTVSS |
| | | | SEQ ID NO: 28064 | SEQ ID NO: 32070 |
| **iPS:443027** | **21-225_7E11.001.023** | NA | GACATCCAGATGACCCAGTCTCCATCCTCCCTGTCTGCATCTGTAGGAGACAGAGTCACCATTGCTTGCCGGGCAAGTCAAAACATTATCAGCTATTTAAATTGGTATCAACAGAAACCAGGGAAAGCCCCTAAATTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAATTTACTACTGTCAACAGACTTACAGTACCCCGCTCACTTTCGGCGGCGGGACCAAGGTGGAGATCAAA | CAGGTGCAGCTGGTGGAGTCTGGGGGAGGCGTGGTCCAGCCTGGGAGGTCCCTGCGACTCTCCTGTGCAGCGTCTGGATTCACCTTCAGTAGCTTTGGCATGCACTGGGTCCGCCAGGCTCCAGGCAAGGGGCTGGAGTGGGTGGCAATTATCTGGCATGATGCAAGTAATAAATACTATGCAGACGCCGTGAAGGGCCGATTCACCATCTCCAGAGACAATTCCAAAAACACGCTGTATCTGCAAATGAGCAGCCTGCGAGCCGAGGACACGGCTGTGTATTACTGTGCGAGAGATCTGAGTATGGGCGGTATGGACGTCTGGGGCCAAGGGACCACGGTCACCGTGTCCTCA |
| | | | SEQ ID NO: 28065 | SEQ ID NO: 32071 |
| | | AA | DIQMTQSPSSLSASVGDRVTIACRASQNIISYLNWYQQKPGKAPKFLIYTASSLQSGVPSRFSGSGSGTDFTLTISSLQPEDFAIYYCQQTYSTPLTFGGGTKVEIK | QVQLVESGGGVVQPGRSLRLSCAASGFTFSSFGMHWVRQAPGKGLEWVAIIWHDASNKYYADAVKGRFTISRDNSKNTLYLQMSSLRAEDTAVYYCARDLSMGGMDVWGQGTTVTVSS |
| | | | SEQ ID NO: 28066 | SEQ ID NO: 32072 |

FIGURE 50

| | | | | |
|---|---|---|---|---|
| **iPS:446086** | **21-225_94D8** | NA | GACATCGTGTTGACCCAGTCGCCAGACTCCCTGGCTGTGTCTCTGGGCGAGAGGGCCACCATCAACTGCAAGTCCAGACAGAGTGTTTTATACAGCTCCAACAATTACAACTACTTAACTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGAAGACGTGGCAGTTTATTACTGTCAGCAATATTATAGTTCTCCTCCTACTTTCGGCGGAGGGACCAAGGTGGAGATCAAA | CAGGTGCAGCTGGTGCAGTCTGGGGCTGAGGTGAAGAAGCCTGGGGCCTCAGTGAAGGTCTCCTGCAAGACTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGTCAGAGTCACCATGACCAGGAACACCTCCATAAGCACAGCCTACATGGAGGTGAGCAGCCTGAGATCTGAGGACACGGCCGTCTATTACTGTGCGTATAGCAGTGGCTGGTACATCTTTGACTACTGGGGCCAGGGAACCCTGGTCACCGTCTCTTCA |
| | | | SEQ ID NO: 28067 | SEQ ID NO: 32073 |
| | | AA | DIVLTQSPDSLAVSLGERATINCKSRQSVLYSSNNYNYLTWYQQKPGQPPKLLIYWASTRESGVPDRFSGSGSGTDFTLTISSLQAEDVAVYYCQQYYSSPPTFGGGTKVEIK | QVQLVQSGAEVKKPGASVKVSCKTSGYTFTNYDINWVRQATGQGLEWMGWMNPNSGNTGYAQKFQVRVTMTRNTSISTAYMEVSSLRSEDTAVYYCAYSSGWYIFDYWGQGTLVTVSS |
| | | | SEQ ID NO: 28068 | SEQ ID NO: 32074 |
| **iPS:446094** | **21-225_77E1** | NA | GACATCGTGATGACCCAGTCTCCAGACTCCCTGGCTGTGTCTCTGGGCGAGAGGGCCACCATCAACTGCAAGTCCAGCCAGACTGTCTTACACAGCTCCAACAATTATAACTACTTAGCTTGGTACCAGCAGAAGCCAGGACAGCCCCCTAAGGTGCTCATTTACTGGACATCTACCCGGGAATCCGGGGTCCATGACCGATTCAGTGGCAGCGGGTCTGGGACAGATTTCACTCTCACCATCAGCAGCCTGCAGGCTGAAGATGTGGCAGTTTATTACTGTCACCAATATCTTAGTAGTCCTCTGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCCCCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTATTGTGCGGTTTCCAGTGGCTGGCACTGGTTCGACCCCTGGGGCCAGGGAACCCTGGTCACCGTCGCCTCA |
| | | | SEQ ID NO: 28069 | SEQ ID NO: 32075 |

FIGURE 50

| | | | | |
|---|---|---|---|---|
| | | AA | DIVMTQSPDSLAVSLGERATINCKSSQTVLHSSNNYNYLAWYQQKPGQPPKVLIYWTSTRESGVHDRFSGSGSGTDFTLTISSLQAEDVAVYYCHQYLSSPLTFGQGTKVEIK | QVQLVQSGAEVKKPGASVKVSCKASGYTFPNYDINWVRQATGQGLEWMGWMNPNSGNTGYAQKFQGRVTMTRNTSISTAYMELSSLRSEDTAVYYCAVSSGWHWFDPWGQGTLVTVAS |
| | | | SEQ ID NO: 28070 | SEQ ID NO: 32076 |
| iPS:448904 | 21-225_65C12 | NA | GAGATAGTGATGACGCAGTCTCCAGCCACCCTGTCTGTGTTTCCAGGGGAAGGAGCCACCCTCTCCTGCAGGGCCAGTCAGAGTGTTAGCATCAACTTAGCCTGGTACCAGCAGAAACCTGGCCAGGCTCCCAGACTCCTCATCTATGGTGCATCCACCAGGGCCACTGGTATCCCAGCCAGGTTCAATGCCAGTGGGTCTGGGACAGAGTTCACTCTCTCCATCAGCAGCCTGCAGTCTGAAAATTTTGCAGTTTATTACTGTCAGCAGTATAATACCTGGCCTCTCACTTTCGGCGGAGGGACCAAGGTGGAAATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGGAGCTTTAGCTTGAACTGGGTCCGCCAGGCTCCAGGGAAGGGGCTGGAGTGGGTCTCATCCATTAGTAGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTACTGTGCGAGAGATGCGTATAGCCACTACTGGGGCCAGGGAACCCTGGTCACCGTCTCCTCA |
| | | | SEQ ID NO: 28071 | SEQ ID NO: 32077 |
| | | AA | EIVMTQSPATLSVFPGEGATLSCRASQSVSINLAWYQQKPGQAPRLLIYGASTRATGIPARFNASGSGTEFTLSISSLQSENFAVYYCQQYNTWPLTFGGGTKVEIK | EVQLVESGGGLVKPGGSLRLSCAASGFTFRSFSLNWVRQAPGKGLEWVSSISSSSSYIYYADSVKGRFTISRDNAKNSLYLQMNSLRAEDTAVYYCARDAYSHYWGQGTLVTVSS |
| | | | SEQ ID NO: 28072 | SEQ ID NO: 32078 |

FIGURE 50

| | | | | |
|---|---|---|---|---|
| **iPS:448906** | **21-225_72G9** | NA | GACATCCAGATGACCCAGTCTCCATCCTCCCTGTCTGCATCTGTAGGAGACAGAGTCACCATCACTTGCCGGGCAAGTCAGAGCATTACCAGCTATTTAAATTGGTATCAGCAGAAACCAGGGAAAGCCCCTAAGCTCCTGATCTATACTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTCACAGTTTCCCATTCACTTTCGGCCCTGGGACCAAAGTGGATATCAAA | GAGGTGCAGCTGGTGGAGTCTGGGGGAGGCCTGGTCAAGCCTGGGGGGTCCCTGAGACTCTCCTGTGCAGCCTCTGGATTCACCTTCAGTAGTTATAGCATGAACTGGGTCCGCCAGGCTCCAGGGAAGGGGCTGGAGTGGGTCTCATCCATTAGTGGTAGTAGTAGTTACATATACTACGCAGACTCAGTGAAGGGCCGATTCACCATCTCCAGAGACAACGCCAAGAACTCACTGTATCTGCAAATGAACAGCCTGAGAGCCGAGGACACGGCTGTGTATTTCTGTGCGAGAGGGGGTTCGAGGGGGTTCGACCCCTGGGGCCAGGGAACCCTGGTCACCGTCTCCTCA |
| | | | SEQ ID NO: 28073 | SEQ ID NO: 32079 |
| | | AA | DIQMTQSPSSLSASVGDRVTITCRASQSITSYLNWYQQKPGKAPKLLIYTASSLQSGVPSRFSGSGSGTDFTLTISSLQPEDFATYYCQQSHSFPFTFGPGTKVDIK | EVQLVESGGGLVKPGGSLRLSCAASGFTFSSYSMNWVRQAPGKGLEWVSSISGSSSYIYYADSVKGRFTISRDNAKNSLYLQMNSLRAEDTAVYFCARGGSRGFDPWGQGTLVTVSS |
| | | | SEQ ID NO: 28074 | SEQ ID NO: 32080 |
| **iPS:448908** | **21-225_50G9** | NA | TCCTATGAGCTGACTCAGCCACCCTCAGTGTCCGTGTCCCCAGGACAGACAGCCAGCATCACCTGCTCTGGAGATAAATTGGGGGATAAATATGCTTGCTGGTATCAGCAGAAGCCAGGCCAGTCCCCTGTGCTGGTCATCTATCAAGATAGCAAGCGGCCCTCAGGGATCCCTGAGCGATTCTCTGGCTCCAACTCTGGGAACACAGCCACTCTGACCATCAGCGGGACCCAGGCTAGGGATGAGGCTGAATATTACTGTCAGGCGCGGAACAGCCGCAGAGGGGTATTCGGCGGAGGGACCAGGCTGACCGTCCTA | CAGGTGCAGCTGGTGGAGTCTGGGGGAGGCGTGGTCCAGCCTGGGAGGTCCCTGAGACTCTCCTGTGCAGCCTCCGGATTCATCTTCAGTAGCTATGGCATGCACTGGGTCCGCCAGGCTCCAGGCAAGGGGCTGGAGTGGGTGGCAGTTATATCACAAGATGGAATTATTAGATACTATGCAGACTCCGTGAAGGGCCGATTCACCATCTCCAGAGACAATTCCAAGAACACGCTGTATCTGCAAATGAACAGCCTGAGAGCTGAGGACACGGCTGTGTATTACTGTGCGAGAGATGTGAAGCAGTGGCTGGTACGGACCTACGGTATGGACGTCTGGGGCCAAGGGACCACGGTCACCGTCTCCTCA |
| | | | SEQ ID NO: 28075 | SEQ ID NO: 32081 |

FIGURE 50

| | | | | |
|---|---|---|---|---|
| | | AA | SYELTQPPSVSVSPGQTASITCSGDKLGDKYACWYQQKPGQSPVLVIYQDSKRPSGIPERFSGSNSGNTATLTISGTQARDEAEYYCQARNSRRGVFGGGTRLTVL | QVQLVESGGGVVQPGRSLRLSCAASGFIFSSYGMHWVRQAPGKGLEWVAVISQDGIIRYYADSVKGRFTISRDNSKNTLYLQMNSLRAEDTAVYYCARDVKQWLVRTYGMDVWGQGTTVTVSS |
| | | | SEQ ID NO: 28076 | SEQ ID NO: 32082 |
| iPS:451102 | 21-225_45F6 | NA | TCCTATGAGCTGACTCAGCCACCCTCAGTGTCCGTGTCCCCAGGACAGACAGCCAGCATCACCTGCTCTGGAGATAAATTGGGGGATAAATATGCTTCCTGGTATCAGCAGAAGCCAGGCCAGTCCCCTGTGCTGGTCATCTATCAAGATAGTAAGCGGCCCTCAGGGATCCCTGAGCGATTCTCTGGCTCCAACTCTGGAAACACAGCCACTCTGACCATCAGCGGGACCCAGGCTATGGATGAGGCTGACTATTACTGTCAGGCGTGGGACAACAGAACTATGGTATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCCTCTGGATTCACCTTCAGTTACTATGGCTTGCACTGGGTCCGCCAGGCTCCAGGCAAGGGGCTGGAGTGGGTGGCAGTTATATCATATGATGGAAGTAATAAATATTATGCAGACTCCGTGAAGGGCCGATTCACCATCTCCAGAGACAATTCCAAGAACACGCTGTATCTGCAAATGAACAACCTGAGAGCTGAGGACACGGCTGTGTTTTACTGTGCGAGAGAGGATCGATATTGTAGTGGTACCAGCTGCCCCTACTACTACTACTACGGTATGGACGTCTGGGGCCAAGGGACCACGGTCACCGTCTCCTCA |
| | | | SEQ ID NO: 28077 | SEQ ID NO: 32083 |
| | | AA | SYELTQPPSVSVSPGQTASITCSGDKLGDKYASWYQQKPGQSPVLVIYQDSKRPSGIPERFSGSNSGNTATLTISGTQAMDEADYYCQAWDNRTMVFGGGTKLTVL | QVQLVESGGGVVQPGRSLRLSCAASGFTFSYYGLHWVRQAPGKGLEWVAVISYDGSNKYYADSVKGRFTISRDNSKNTLYLQMNNLRAEDTAVFYCAREDRYCSGTSCPYYYYYGMDVWGQGTTVTVSS |
| | | | SEQ ID NO: 28078 | SEQ ID NO: 32084 |

FIGURE 50

| | | | | |
|---|---|---|---|---|
| **iPS:451104** | **21-225_49C5** | NA | CAGTCTGTGCTGACTCAGCCACCCTCAGCGTCTGGGACCCCCGGGCAGAGGGTCACCATCTCTTGTTCTGGAAGCAGCTCCAACATCGGAAGTAATATTGTGACCTGGTACCAGCAGCTCCCAGGAACGGCCCCCAAACTCCTCATCTATAGTAATGATCAGCGGCCCTCAGGGGTCCCTGACCGATTCTCTGGCTCCAAGTCTGGCACCTCAGCCTCCCTGGCCATCAGTGGGCTCCAGTCTGAGGATGAGGCTGATTATTACTGTACAGCATGGGATGACAGCCTGAATGGTTGGGTGTTCGGCGGAGGGACCACGCTGACCGTCCTA | CAGGTTCAGCTGGTGCAGTCTGGAGCTGAGGTGAAGAAGCCTGGGGCCTCAGTGAAGGTCTCCTGCAAGGCTTCTGGTTACACCTTTAACAGCTATGGTATCAGCTGGGTGCGACAGGCCCCTGGACAAGGGCTTGAGTGGATGGGATGGATCAGCGCTTATAATGGTAACACAAAGTATGCACAGAAGCTCCAGGGCAGAGTCACCATGACCACAGACACATCCACGAGCACAGCCTACATGGAGCTGAGGAGCCTGAGATCTGACGACACGGCCGTGTATTACTGTGCGAGACACGATTTTTGGAGTGGTTATTATAAGGGTATGGACGTCTGGGGCCAAGGGACCACGGTCACCGTCTCCTCA |
| | | | SEQ ID NO: 28079 | SEQ ID NO: 32085 |
| | | AA | QSVLTQPPSASGTPGQRVTISCSGSSSNIGSNIVTWYQQLPGTAPKLLIYSNDQRPSGVPDRFSGSKSGTSASLAISGLQSEDEADYYCTAWDDSLNGWVFGGGTTLTVL | QVQLVQSGAEVKKPGASVKVSCKASGYTFNSYGISWVRQAPGQGLEWMGWISAYNGNTKYAQKLQGRVTMTTDTSTSTAYMELRSLRSDDTAVYYCARHDFWSGYYKGMDVWGQGTTVTVSS |
| | | | SEQ ID NO: 28080 | SEQ ID NO: 32086 |
| **iPS:451106** | **21-225_49D10** | NA | CAGTCTGTGCTGACTCAGCCACCCTCAGCGTCTGGGACCCCCGGGCAGAGGGTCACCATCTCTTGTTCTGGAAGCAACTCCAACATCGGAAGTAATATTGTAACCTGGTACCAGCAGCTCCCAGGAACGGCCCCCAAACTCCTCATCTATAGTAATGATCAGCGGCCCTCAGGGGTCCCTGACCGATTCTCTGGCTCCAAGTCTGGCACCTCAGCCTCCCTGGCCATCAGTGGGCTCCAGTCTGAGGATGAGGCTGATTATTACTGTGCAGCATGGGATGACAGCCTGAATGGTTGGGTGTTCGGCGGAGGGACCACGCTGACCGTCCTA | CAGGTTCAGCTGGTGCAGTCTGGAGCTGAGGTGAAGAAGCCTGGGGCCTCAGTGAAGGTCTCCTGCAAGGCTTCTGGTTACACCTTTAACAGCTATGGTATCAGCTGGGTGCGACTGGCCCCTGGACAAGGGTTTGAGTGGATGGGATGGATCAGCGCTTATAATGGTAACACAAAGAATGCACAGAAGCTCCAGGGCAGAGTCACCATGACCACAGACACATCCACGAGCACAGCCTACATGGAGCTGAGGAGCCTGAGATCTGACGACACGGCCGTTTATTACTGTGCGAGACACGATTTTTGGAGTGGTTATTATAAGGGTATGGACGTCTGGGGCCAAGGGACCACGGTCACCGTCTCCTCA |
| | | | SEQ ID NO: 28081 | SEQ ID NO: 32087 |

FIGURE 50

| | | | | |
|---|---|---|---|---|
| | | AA | QSVLTQPPSASGTPGQRVTISCSGSNSNIGSNIVTWYQQLPGTAPKLLIYSNDQRPSGVPDRFSGSKSGTSASLAISGLQSEDEADYYCAAWDDSLNGWVFGGGTTLTVL | QVQLVQSGAEVKKPGASVKVSCKASGYTFNSYGISWVRLAPGQGFEWMGWISAYNGNTKNAQKLQGRVTMTTDTSTSTAYMELRSLRSDDTAVYYCARHDFWSGYYKGMDVWGQGTTVTVSS |
| | | | SEQ ID NO: 28082 | SEQ ID NO: 32088 |
| **iPS:451108** | **21-225_53E8** | NA | CAGTCTGTGCTGACTCAGCCACCCTCAGCGTCTGGGACCCCCGGGCAGAGGGTCACCATCTCTTGTTCTGGAAGCTGCTCCAACATCGGAAGTAATATTGTGACCTGGTACCAGCAGCTCCCAGGAACGGCCCCCAAACTCCTCATCTATAGTAATGATCAGCGGCCCTCAGGGGTCCCTGACCGATTCTCTGGCTCCAAGTCTGGCACCTCAGCCTCCCTGGCCATCAGTGGGCTCCAGTCTGAGGATGAGGCTGATTATTACTGTACAGCATGGGATGACAGCCTGAATGATTGGGTGTTCGGCGGAGGGACCACGCTGACCGTCCTA | CAGGTTCAGCTGGTGCAGTCTGGAGCTGAGGTGAAGAAGCCTGGGGCCTCAGTGAAGGTCTCCTGCAAGGCTTCTGGTTACACCTTTAACAGCTATGGTATCAGCTGGGTGCGACAGGCCCCTGGACAAGGGCTTGAGTGGATGGGATGGATCAGCGCTTATAATGGTAACACAAAGTTTGCACAGAAGCTCCAGGGCAGAGTCACCATGACCACAGACACATCCACGAGCACAGCCTACATGGAACTGAGGAGCCTGAGATCTGACGACACGGCCGTGTATTACTGTGCGAGACACGATTTTTGGAGTGGTTATTATAAGGGTATGGACGTCTGGGGCCAAGGGACCACGGTCACCGTCTCCTCA |
| | | | SEQ ID NO: 28083 | SEQ ID NO: 32089 |
| | | AA | QSVLTQPPSASGTPGQRVTISCSGSCSNIGSNIVTWYQQLPGTAPKLLIYSNDQRPSGVPDRFSGSKSGTSASLAISGLQSEDEADYYCTAWDDSLNDWVFGGGTTLTVL | QVQLVQSGAEVKKPGASVKVSCKASGYTFNSYGISWVRQAPGQGLEWMGWISAYNGNTKFAQKLQGRVTMTTDTSTSTAYMELRSLRSDDTAVYYCARHDFWSGYYKGMDVWGQGTTVTVSS |
| | | | SEQ ID NO: 28084 | SEQ ID NO: 32090 |

FIGURE 50

| | | | | |
|---|---|---|---|---|
| **iPS:451110** | **21-225_74C9** | NA | TCTTATGAGCTGACTCAGCCACCCTCAGAGTCTGTGTCCCCAGGACAGACAGCCAGCATCACCTGCTCAGGAGATAAATCGGGGAATAAATATGTTTCCTGGTATCAGCAGAAGCCAGGCCAGTCCCCTGTGCTGGTCATCTATCAAGATAACAGGCGGCCGTCAGGGATCCCTGAGCGATTTTCTGGCTCCAACTCTGGGAGCACAGCCACTTTGACCATCAGCGGGACCCAGGCTATGGATGAGGCTGACTATTACTGTCAGGCGTGGGACAGCACCCCTGTGATATTCGGCGGAGGGACCAAGCTGACCGTCCTA | CAGGTGCAGCTGGTGGAGTCTGGGGGAGGCGTGGTCCAGCCTGGGAGGTCCCTGAGACTCTCCTGTGCAGCGTCTGGATTCACCTTCAGTAGCTATGGCATGCACTGGGTCCGCCAGGCTCCAGGCAAGGGGCTGGAGTGGGTGGCAGTTATATGGTATGATGGAAATAATAAATCCTATGCAGACTCCGTGAAGGGCCGATTCACCATCTCCAGAGACATTTCCAAAAACACGCTGTATCTGCAAATGAACAGCCTGAGAGCCGAGGACACGGCTGTGTATTACTGTGCGAGGGATCGAGATTATTGTAGTAGTACCAGCTGCCCTTATTATTACTACTACGGTATGGACGTCTGGGGCCAAGGGACCACGGTCACCGTCTCCTCA |
| | | | SEQ ID NO: 28085 | SEQ ID NO: 32091 |
| | | AA | SYELTQPPSESVSPGQTASITCSGDKSGNKYVSWYQQKPGQSPVLVIYQDNRRPSGIPERFSGSNSGSTATLTISGTQAMDEADYYCQAWDSTPVIFGGGTKLTVL | QVQLVESGGGVVQPGRSLRLSCAASGFTFSSYGMHWVRQAPGKGLEWVAVIWYDGNNKSYADSVKGRFTISRDISKNTLYLQMNSLRAEDTAVYYCARDRDYCSSTSCPYYYYYGMDVWGQGTTVTVSS |
| | | | SEQ ID NO: 28086 | SEQ ID NO: 32092 |
| **iPS:451112** | **21-225_53D10** | NA | TCCTATGAGCTGACTCAGCCACCCTCAGTGTCCGTGTCCCCAGGACAGACAGCCAGCATCACCTGCTCTGGAGATAAATTGGGGAATAAATATGCTTGCTGGTATCAGCAGAGGCCAGGCCAGTCCCCTGTGCTGGTCATCTATCAAGATCGCAAGCGGCCCTCAGGGATCCCTGAGCGATTCTCTGGCTCCAACTCTGGGAACACAGCCACTCTGACCATCAGCGGGACCCAGGCTATGGATGAGGCTGACTATTACTGTCAGGCGTGGGACAGCAGCACTGTGGTATTCGGCGGAGGGACCAAGCTGACCGTCCTA | CAGGTGCAGTTGGTGCAGTCTGGGGCTGAGGTGAAGAAGCCTGGGGCCTCAGTGAAGGTCTCCTGCAAGGCTTCTGGATACATTTTCACCGGCTACTATATACACTGGGTGCGACAGGCCCCTGGACAAGGGCTTGAGTGGATGGGATGGATCAACCCTAACAGTGGTGGCACAAACTATGCACAGAAGTTTCAGGGCAGGGTCACCATGACCAGGGACACGTCCATCAGCACAGCCTACATGGAGCTGATCAGGCTGAGATCTGACGACACGGCCGTGTATTATTGTGCGAGAGAAAACGAAAGTCTAGCAACTCGTCCTTTCTACGACTACTACGGTATGGACGTCTGGGGCCAAGGGACCACGGTCACCGTCTCCTCA |

FIGURE 50

| | | | | |
|---|---|---|---|---|
| | | | SEQ ID NO: 28087 | SEQ ID NO: 32093 |
| | | AA | SYELTQPPSVSVSPGQTASITCSGDKLGNKYACWYQQRPGQSPVLVIYQDRKRPSGIPERFSGSNSGNTATLTISGTQAMDEADYYCQAWDSSTVVFGGGTKLTVL | QVQLVQSGAEVKKPGASVKVSCKASGYIFTGYYIHWVRQAPGQGLEWMGWINPNSGGTNYAQKFQGRVTMTRDTSISTAYMELIRLRSDDTAVYYCARENESLATRPFYDYYGMDVWGQGTTVTVSS |
| | | | SEQ ID NO: 28088 | SEQ ID NO: 32094 |
| **iPS:451114** | **21-225_159A3** | NA | GACATCCAGATGACCCAGTCTCCATCCTCCCTGTCTGCATCTGTAGGAGACAGAGTCACCATCACTTGCCGGGCAAGTCAGGACATTAGAAAGGATTTAGGCTGGTATCAGCAGAAACCAGGGAAAGCCCCTAACCGCCTGATCTATGCTGCATCCAGTTTGCAAAGTGGGGTCCCATCAAGGTTCAGCGGCAGTGGATCTGGGACAGAATTCACTCTCACATTCAGCAGCCTGCAGCCTGAAGATTTTGCAACTTATTACTGTCTACAGCATCATAGTTATCCTCGGACGTTCGGCCAAGGGACCAAGGTGGAAATCAAA | CAGGTGCAGCTGGTGGAGTCTGGGGGAGGCGTGGTCCAGCCTGGGAGGTCCCTGAGACTCTCCTGTGCAGCGTCTGGATTCACCTTTAGTGACTATGTCATGCAGTGGGTCCGCCAGGCTCCAGGCAAGGGGCTGGAGTGGGTGGCAGTTATATGGTATGATGGAAGTAATAAATACTATGCAGACTCCGTGAAGGGCCGATTCACCATCTCCAGAGACATTTCCAAGAACACGCTGTATCTGCAAATGAACAGCCTGAGAGCCGAGGACACGGCTGTGTATTACTGTGCGAGAGAACCGTATAATAGTGGCTGGTACGACTACGGTATGGACGTCTGGGGCCAAGGGACCACGGTCACCGTCTCCTCA |
| | | | SEQ ID NO: 28089 | SEQ ID NO: 32095 |
| | | AA | DIQMTQSPSSLSASVGDRVTITCRASQDIRKDLGWYQQKPGKAPNRLIYAASSLQSGVPSRFSGSGSGTEFTLTFSSLQPEDFATYYCLQHHSYPRTFGQGTKVEIK | QVQLVESGGGVVQPGRSLRLSCAASGFTFSDYVMQWVRQAPGKGLEWVAVIWYDGSNKYYADSVKGRFTISRDISKNTLYLQMNSLRAEDTAVYYCAREPYNSGWYDYGMDVWGQGTTVTVSS |
| | | | SEQ ID NO: 28090 | SEQ ID NO: 32096 |

FIGURE 50

| | | | | |
|---|---|---|---|---|
| iPS:451116 | 21-225_164A4 | NA | GACATCGTGATGACCCAGTATCCAGACTCCCGGGCTGTGTCTCTGGGCGAGAGGGCCACCATCAAGTGCAAGTCCAGCCAGAGTGTTTTATACAGCTCCAACAATAAGAACTACTTAACTTGGTACCAGCAGAAACCAGGACAGCCTCCCAAACTGTTCATTTACTGGGCATCTACCCGGGAATCCGGGGTTCCTGACCGATTCAGTGGCAGCGGGTGTGGGACAGATTTCACTCTCACCATCAGCAGCGTGCAGGCCGAAGATGTGGCAGTTTATTACTGTCAGCAATATTTTAGTACTCCGTGGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATTCACCTTCCCCAATTATGATATCAACTGGGTGCGACAGGCCACTGGACAAGGCCTTGAGTGGATGGGATGGATGCACCCTAACAGTGGTAACACAGGCTATGCACAGAAGTTCCAGGGCAGAGTCACCATGACCAGGAACACCTCCATAAGCACAGCCTACATGGAGCTGAGCAGCCTGAGATCTGAGGACACGGCCGTGTATTACTGTGCGAGTAGCAGTGGCTGGTACTTCTTTGACTACTGGGGCCAGGGAACCCTGGTCACCGTCTCCTCA |
| | | | SEQ ID NO: 28091 | SEQ ID NO: 32097 |
| | | AA | DIVMTQYPDSRAVSLGERATIKCKSSQSVLYSSNNKNYLTWYQQKPGQPPKLFIYWASTRESGVPDRFSGSGCGTDFTLTISSVQAEDVAVYYCQQYFSTPWTFGQGTKVEIK | QVQLVQSGAEVKKPGASVKVSCKASGFTFPNYDINWVRQATGQGLEWMGWMHPNSGNTGYAQKFQGRVTMTRNTSISTAYMELSSLRSEDTAVYYCASSSGWYFFDYWGQGTLVTVSS |
| | | | SEQ ID NO: 28092 | SEQ ID NO: 32098 |
| iPS:451118 | 21-225_191C8 | NA | GAAATAGTGATGACGCAGTCTCCAGCCACCCTGTCTGTGTCTCCAGGGGAAAGAGCCACCCTCTCCTGCAGGGCCAGTCAGAGTGTTCGCAGTAACTTAGCCTGGTACCAGCAGGAACCTGGCCAGGCTCCCAGGCTCCTCATCTATGGTGCATCCACCAGGGCCACTGGTATCCCAGCCAGGTTCAGTGGCAGTGGGTCTGGGACAGAATTCACTCTCACCATCAGCAGCTTGCAGTCTGAAGATTTTGCAGTTTATTACTGTCAGCAGTCTTTTACCTGGCTCCGGACGTTCGGCCAAGGGACCAAGGTGGAAATCAAA | CAGGTGCAGCTGCAGGAGTCGGGCCCAGGACTGGTGAAGCCTTCGGAGACCCTGTCCCTCACCTGCACTGTCTCTAGTGGCTCCGTCAGCAGTGGTGGTTACTACTGGAGCTGGATCCGGCAGCCCCCAGGGAAGGGACTGGAGTGGATTGGGTATATCTATTACAGTGGGACCACCATTTACAACCCCTCCCTCAAGAGTCGAGTCACCATATCAGTAGACACGTCCAAGAACCAGTTCTCCCTGAAGCTGACCTCTGTGACCGTTGCGGACACGGCCGTGTATTACTGTGCGCGAGACACGTTTTGCTTTGATGGTTGTGGTTATTTCTTTGACTCCTGGGGCCAGGGAACCCTGGTCACCGTCTCCTCA |
| | | | SEQ ID NO: 28093 | SEQ ID NO: 32099 |

FIGURE 50

| | | | | |
|---|---|---|---|---|
| | | AA | EIVMTQSPATLSVSPGERATLSCRASQSVRSNLAWYQQEPGQAPRLLIYGASTRATGIPARFSGSGSGTEFTLTISSLQSEDFAVYYCQQSFTWLRTFGQGTKVEIK | QVQLQESGPGLVKPSETLSLTCTVSSGSVSSGGYYWSWIRQPPGKGLEWIGYIYYSGTTIYNPSLKSRVTISVDTSKNQFSLKLTSVTVADTAVYYCARDTFCFDGCGYFFDSWGQGTLVTVSS |
| | | | SEQ ID NO: 28094 | SEQ ID NO: 32100 |
| iPS:451120 | 21-225_197D3 | NA | GACATCCAGATGACCCAGTCTCCATCCTCACTGTCTGCATCTGTAGGAGACAGAGTCACCATCACTTGTCGGGCGAGTCAGGGCATTAGAAATTATTTAGCCTGGTTTCAGCAGAAACCAGGGAAAGCCCCTAAGTCCCTGATCTATGCTGCATCCAGTTTGCAAAGTGGGGTCCCCTCAAAGTTCAGCGGCAGTGGATCTGGGACAGATTTCACTCTCACCATCAGCAGCCTGCAGCCTGAAGATTTTGCAACTTATTACTGCCAACATTATCTTACTTACCCGCTCACTTTCGGCGGAGGGACCAAGGTGGAGATCAAA | CAGGTGCAGCTGGTGGAGTCTGGGGGAGGCGTGGTCCAGCCTGGGAGGTCCCTGAGACTCTCCTGTGCAGCGTCTGGATTCATCTTCAGTAGCCATGGCATGCACTGGGTCCGCCAGGCTCCAGGCAAGGGGCTGGAGTGGGTGGCAGTTATATGGTATGATGGAAGTAATGAACACTATGCAGACTCCGTGAAGGGCCGATTCACCATCTCCAGAGACAATTCCAAGAACACGCTGTATCTGCAAATGAACAGCCTGAGAGCCGAGGACACGGCTGTTTATTACTGTGCGAGAGATCAAGGTGTGGGGTACTACGGTATGGACGTCTGGGGCCAAGGGACCACGGTCACCGTCTCCTCA |
| | | | SEQ ID NO: 28095 | SEQ ID NO: 32101 |
| | | AA | DIQMTQSPSSLSASVGDRVTITCRASQGIRNYLAWFQQKPGKAPKSLIYAASSLQSGVPSKFSGSGSGTDFTLTISSLQPEDFATYYCQHYLTYPLTFGGGTKVEIK | QVQLVESGGGVVQPGRSLRLSCAASGFIFSSHGMHWVRQAPGKGLEWVAVIWYDGSNEHYADSVKGRFTISRDNSKNTLYLQMNSLRAEDTAVYYCARDQGVGYYGMDVWGQGTTVTVSS |
| | | | SEQ ID NO: 28096 | SEQ ID NO: 32102 |

FIGURE 50

| | | | | |
|---|---|---|---|---|
| iPS:451122 | 21-225_200A1 | NA | GAAATTGTGTTGACGCAGTCTCCAGGCATCCTGTCTTTGTATCCAGGGGAAAGAGCCACCCTCTCCTGTAGGGCCAGTCAGAGTGTTAACAGCAACTATTTAGCCTGGTACCAGCAGAGACCTGGCCAGGCTCCCAGTCTCCTCATTTATGGGGCATCCAGCAGGGCCACTTGCATCCTGGACAGGTTCAGTGGCAGTGGGTGTGGGACAGACTTCACTCTCACGATCAGCAGACTGGAGCCTGAAGATTTTGCAGTGTATTGCTGTCAGCAGTATGAGATCTCACCGTGGACGTTCGGCCAAGGGACCAAGGTGGAAAGCAAA | CAGGTGCAGCTACAGCAGTGGGGCGCAGGACTGTTGAAGCCTTCGGAGACCCTGTCCCTCACCTGCGCTGTCTATGGTGGGTCCTTCAGTGTTTACTATTGGAGCTGGATCCGCCAGCCCCCAGGGAAGGGGCTGGAGTGGATTGGGGAAATCAATCATAGTGGAAGCACCAACTACAACCCGTCCCTCAAGAGTCGAGTCACCATTTCACTAGACACGTCCAAGAACCAGTTCTCCCTGAAGCTGAGCTCTGTGACCGCCGCGGACACGGCTGTGTATTACTGTGCGAGAGACTACGGTGTCTTTGACTACTGGGGCCAGGGAACCCTGGTCACCGTCTCCTCA |
| | | | SEQ ID NO: 28097 | SEQ ID NO: 32103 |
| | | AA | EIVLTQSPGILSLYPGERATLSCRASQSVNSNYLAWYQQRPGQAPSLLIYGASSRATCILDRFSGSGCGTDFTLTISRLEPEDFAVYCCQQYEISPWTFGQGTKVESK | QVQLQQWGAGLLKPSETLSLTCAVYGGSFSVYYWSWIRQPPGKGLEWIGEINHSGSTNYNPSLKSRVTISLDTSKNQFSLKLSSVTAADTAVYYCARDYGVFDYWGQGTLVTVSS |
| | | | SEQ ID NO: 28098 | SEQ ID NO: 32104 |
| iPS:451124 | 21-225_74F6 | NA | GACATCGTGATGACCCAGTCTCCAGACTCCCTGGCTGTGTCTCTGGGCGAGAGGGCCACCATCAATTGCAAGTCCAGTCAGAATATTTTATCCAGCTCCAACAATAAGAACTACTTAACTTGGTACCAGCAGAAACCAGGACAGCCTCCTAAAATACTCATTTACTGGACATCTACCCGGGAATCCGGGGTCCCTGACCGATTCAGTGGCAGCGGGTTTGGGACAGATTTCACTCTCACCATCAGCAGCCTGCAGGCTGAAGATGTGGCTGTTTATTACTGTCAGCAATATTTTAGTGTTCCTCTGACGTTCGGCCAAGGGACCAAGGTGGAAATCAAA | CAGGTGCAGCTGGTGCAGTCTGGGGCTGAGGTGAAGAAACCTGGGGCCTCAGTGAGGGTCTCCTGCAAGGCTTCTGGATACACCTTCACCAATTATGATATCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCATGAGCACAGCCTACATGGAGCTGAGCAGCCTGAGATCTGAGGACACGGCCGTGTATTACTGTGCCCATAGCAGTGGCTGGTACTTTTTTGACTACTGGGGCCAGGGAACCCTGGTCACCGTCTCCTCA |
| | | | SEQ ID NO: 28099 | SEQ ID NO: 32105 |

FIGURE 50

| | | | | |
|---|---|---|---|---|
| | | AA | DIVMTQSPDSLAVSLGERATINCKSSQNILSSSNNKNYLTWYQQKPGQPPKILIYWTSTRESGVPDRFSGSGFGTDFTLTISSLQAEDVAVYYCQQYFSVPLTFGQGTKVEIK | QVQLVQSGAEVKKPGASVRVSCKASGYTFTNYDINWVRQATGQGLEWMGWMHPNSGNTGYAQKFQGRVTMTRNTSMSTAYMELSSLRSEDTAVYYCAHSSGWYFFDYWGQGTLVTVSS |
| | | | SEQ ID NO: 28100 | SEQ ID NO: 32106 |
| iPS:451127 | 21-225_164A7 | NA | GACATCGTGATGACCCAGTGTCCAGACTCCCTGGCTGTGTCTCTGGGCGAGAGGGCCACCATCAATTGCAAGTCCAGCCAGAGTGTTTTACACAGCTCCAACAATAACAACTACTTAGCTTGGTACCAGCAGAAACCAGGACAGCCTCATAAGTTGCTCATTTACTGGACATCTACCCGGGAATCCGGGGTCCCTGACCGATTCAGTGGCAGCGGGTATGGGACAGATTTCTCTCTCACCATCGCCAGCCTGCAGGCTGAAGATGTGGCAGTTTATTACTGTCAGCAATATTATAGTATTCCTCTGACGTTCGGCCAAGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAATTATGATGTCAACTGGGTGCGACAGGCCACTGGACAAGGGCTTGAGTGGATGGGATGGATGCACCCTAACAGTGGTAACACAGGCTATGCACAGAAGTTCCAGGGCAGAGTCACCATGACCAGGAACACCTCCACAAGCACAGCCTACATGGAGCTGAGCAGCCTGAGATCTGAGGACTCGGCCGTGTATTACTGTGCGAGTAGCAGTGGCTGGTACCTCTTTGACTACTGGGGCCAGGGAACCCTGGTCACCGTCTCCTCA |
| | | | SEQ ID NO: 28101 | SEQ ID NO: 32107 |
| | | AA | DIVMTQCPDSLAVSLGERATINCKSSQSVLHSSNNNNYLAWYQQKPGQPHKLLIYWTSTRESGVPDRFSGSGYGTDFSLTIASLQAEDVAVYYCQQYYSIPLTFGQGTKVEIK | QVQLVQSGAEVKKPGASVKVSCKASGYTFTNYDVNWVRQATGQGLEWMGWMHPNSGNTGYAQKFQGRVTMTRNTSTSTAYMELSSLRSEDSAVYYCASSSGWYLFDYWGQGTLVTVSS |
| | | | SEQ ID NO: 28102 | SEQ ID NO: 32108 |

FIGURE 50

| | | | | |
|---|---|---|---|---|
| **iPS:451129** | **21-225_94D2** | NA | GACATCGTGATGACCCAGTCTCCAGACTCCCTGGCTGTGTCTCTGGGCGAGAGGGCCACCATCAACTGCAAGTCCAGCCAGAGTGTTTTACACAGCTCCAACAATAAGAACTACTTAACTTGGTACCAGCAGAAACCAGGACAGCCTCATAAGCTGCTCATTTACTGGGCATCTACTCGGGAATCCGGGGTCCCTGACCGATTCAGCGGCAGCGGGTCTGGGACAGATTTCAGTCTCACGATCGGCAGCCTGCAGCATGAAGATGTGGCAGTTTATTACTGTCAGCAATATCATAGTATTCCTCCGACGTTCGGCCACGGGACCAAGGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATTCACCTTCACCAATTATGATATCAACTGGGTGCGACAGGCCACTGGACAAGGGCTTGAGTGGATGGGATGGATGCACCCTAACAGTGGTAACACAGGCTTTGCACAGAAGTTCCAGGGCAGAGTCACAATGACCAGGAACACCTCCATAAGCACAGCCTACATGGAACTGAGCAGCCTGAGATCTGAGGACACGGCCGTGTATTACTGTGCGGTTTCCAGTGGCTGGTACTGGTTCGACCCCTGGGGCCAGGGAACCCTGGTCACCGTCTCCTCA |
| | | | SEQ ID NO: 28103 | SEQ ID NO: 32109 |
| | | AA | DIVMTQSPDSLAVSLGERATINCKSSQSVLHSSNNKNYLTWYQQKPGQPHKLLIYWASTRESGVPDRFSGSGSGTDFSLTIGSLQHEDVAVYYCQQYHSIPPTFGHGTKVEIK | QVQLVQSGAEVKKPGASVKVSCKASGFTFTNYDINWVRQATGQGLEWMGWMHPNSGNTGFAQKFQGRVTMTRNTSISTAYMELSSLRSEDTAVYYCAVSSGWYWFDPWGQGTLVTVSS |
| | | | SEQ ID NO: 28104 | SEQ ID NO: 32110 |
| **iPS:451131** | **21-225_160A7** | NA | GACATCGTGCTGACCCAGTCTCCAGACTCCCCGGCTGTGTCTCTGGGCGAGAGGGCCACCATCAATTGCAAGTCCAGCCAGAGTGTTTTATCCAACTCCCACAATAACAACTACTTAGCTTGGTACCAGCAGAGACCAGGACATCCTCATAAACTCCTCATTTTCTGGGCATCTACCCGGGAATCCGGGGTCCCTGACCGATTCAGTGGCAGCGGGTATGGGACAGATTTCACTCTCACCATCAGCAGCCTGCAGGCTGAAGATGTGGCAGTTTATTACTGTCAGCAATATTATAGTACTCCGTGCAGTTTTGGCCAGGGGACCAAGCTGGAGATCAAA | CAGGTGCAGCTGGTGCAGTCTGGGGCTGAAGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGCACCCTCACAGTGGTAACACAGGCTATGCACAGAAGTTCCAGGGCAGAGTCACCATGACCAGGAACACCTCCATAAACACAGCCTACATGGAGCTGAGCAGCCTGAGATCTGAGGACACGGCCGTGTATTACTGTGCCCATAGCAGTGGCTGGTACTACTTTGACTACTGGGGCCAGGGAACCCTGGTCACCGTCTCCTCA |
| | | | SEQ ID NO: 28105 | SEQ ID NO: 32111 |

FIGURE 50

| | | | | |
|---|---|---|---|---|
| | | AA | DIVLTQSPDSPAVSLGERATINCKSSQSVLSNSHNNNYLAWYQQRPGHPHKLLIFWASTRESGVPDRFSGSGYGTDFTLTISSLQAEDVAVYYCQQYYSTPCSFGQGTKLEIK | QVQLVQSGAEVKKPGASVKVSCKASGYTFTNYDINWVRQATGQGLEWMGWMHPHSGNTGYAQKFQGRVTMTRNTSINTAYMELSSLRSEDTAVYYCAHSSGWYYFDYWGQGTLVTVSS |
| | | | SEQ ID NO: 28106 | SEQ ID NO: 32112 |
| iPS:451133 | 21-225_95H4 | NA | GACATCGTTATGACCCAGTGTCCAGACTCCCTGGCTGTGTCTCTGGGCGAGAGGGCCACCATCAATTGCAAGTCCAGCCAGAGTGTTTTATTCAGCTCCAACAATTATAATTACTTAGCTTGGTACCAGCAGAGACCAGGACAGCCTCATAACCTGCTCATTTACTGGGCATCTACCCGGGAATCCGGGGTCCCTGACCGATTCAGTGGCAGCGGGTCTGGGACAGATTTCACTCTCACCATCAGCAGCCTGCAGGCTGCTGATGTGGCAGTTTATTACTGTCAGCAATATCATAGTTCTCCTCTGACGTTCGGCCAAGGGACCACGGTGCAAATCAAA | CAGGTGCAGTTGGTGCAGTCTGGGGCTGAGGTGAAGAAGCCTGGGGCCTCAGTGAAGGTCTCCTGCAAGGCTTCTGGATACACCTTCACCAATTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCAGGAACACCTCCACAAGCACAGCCCACATGGAGCTGAGCAGCCTGAGATCTGAGGACACGGCCGTGTATTACTGTGCGGTCTCCAGTGGCTGGAACTGGTTCGACCCCTGGGGCCAGGGAACCCTGGTCACCGTCTCCTCA |
| | | | SEQ ID NO: 28107 | SEQ ID NO: 32113 |
| | | AA | DIVMTQCPDSLAVSLGERATINCKSSQSVLFSSNNYNYLAWYQQRPGQPHNLLIYWASTRESGVPDRFSGSGSGTDFTLTISSLQAADVAVYYCQQYHSSPLTFGQGTTVQIK | QVQLVQSGAEVKKPGASVKVSCKASGYTFTNYDINWVRQATGQGLEWMGWMNPNSGNTGYAQKFQGRVTMTRNTSTSTAHMELSSLRSEDTAVYYCAVSSGWNWFDPWGQGTLVTVSS |
| | | | SEQ ID NO: 28108 | SEQ ID NO: 32114 |

FIGURE 50

| | | | | |
|---|---|---|---|---|
| **iPS:437240** | **21-225_84H12** | NA | GACATCCAGATGACCCAGTCTCCATCCTACCAGTCTGCATCTGTGGGAGACAGAGTCACCATCACTTGCCGGGCAAGTCAGGGCATTAGAAATGATTTAGGCTGGTTTCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTTTACAGCATAATGATTACCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAGTTATGATATCAACTGGGTGCGACAGGCCACTGGACAAGGGCTTGAGTGGATGGGATGGCTGAACCCTCACAGTGGTAACACAGGCTATGCACAGAAGTTCCAGGGCAGAATCACCATGACCTGGAACACCTCCATACGCACTGCCTACATGGAGCTGAGCAGCCTGAGATCTGAGGACACGGCCGTGTATTACTGTGCGAGAGGGTTTTACGATATTTTGACTGGTTATTCCCCCACCTACTACTACTACGATATGGACGTCTGGGGCCAAGGGACCACGGTCACCGTCTCCTCA |
| | | | SEQ ID NO: 28109 | SEQ ID NO: 32115 |
| | | AA | DIQMTQSPSYQSASVGDRVTITCRASQGIRNDLGWFQQKPGKAPKRLIYAASSLQSGVPSRFSGSGSGTEFTLTISSLQPEDFATYYCLQHNDYPFTFGPGTKVDIK | QVQLVQSGAEVKKPGASVKVSCKASGYTFTSYDINWVRQATGQGLEWMGWLNPHSGNTGYAQKFQGRITMTWNTSIRTAYMELSSLRSEDTAVYYCARGFYDILTGYSPTYYYYDMDVWGQGTTVTVSS |
| | | | SEQ ID NO: 28110 | SEQ ID NO: 32116 |

FIGURE 50

| | | | | |
|---|---|---|---|---|
| **iPS:434577** | **21-225_75C11** | NA | GACATCCAGATGACCCAGTCTCCATCCTCCCTGTCTGCATCTGTGGGAGACAGAGTCACCATCACTTGCCGGGCAAGTCAGGGCATTAGAAATGATTTAGGCTGGTTTCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATGATTACCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAGTTATGATATCAACTGGGTGCGACAGGCCACTGGACAAGGGCTTGAGTGGATGGGATGGCTGAACCCTCACAGTGGTAACACAGGCTATGCACAGAAGTTCCAGGGCAGAATCACCATGACCTGGAACACCTCCATACGCACTGCCTACATGGAGCTGAGCAGCCTGAGATCTGAGGACACGGCCGTGTATTACTGTGCGAGAGGGTTTTACGATATTTTGACTGGTTATTCCCCCACCTACTACTACTACGATATGGACGTCTGGGGCCAAGGGACCACGGTCACCGTCTCCTCA |
| | | | SEQ ID NO: 28111 | SEQ ID NO: 32117 |
| | | AA | DIQMTQSPSSLSASVGDRVTITCRASQGIRNDLGWFQQKPGKAPKRLIYAASSLQSGVPSRFSGSGSGTEFTLTISSLQPEDFATYYCLQHNDYPFTFGPGTKVDIK | QVQLVQSGAEVKKPGASVKVSCKASGYTFTSYDINWVRQATGQGLEWMGWLNPHSGNTGYAQKFQGRITMTWNTSIRTAYMELSSLRSEDTAVYYCARGFYDILTGYSPTYYYYDMDVWGQGTTVTVSS |
| | | | SEQ ID NO: 28112 | SEQ ID NO: 32118 |

FIGURE 50

| | | | | |
|---|---|---|---|---|
| **iPS:435477** | **21-225_154E8** | NA | GACATCCAGATGACCCAGTCTCCATCTTCCGTGTCTGCATCTATAGGAGACAGAGTCACCATCACTTGTCGGGCGAGTCAGTTTATTAGCAGCTGGTTAGCCTGGTATCAGCAGAAACCAGGGAAAGCCCCTAAGCTCCTGATCTATACTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTGTTGTCAACAGGCTAACAGTTTCCCGTGGACGTTCGGCCAAGGGACCAAGGTGGAATTCAAC | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAGGGGGCTGGAGTGGGTCTCAGCTATTAGTGGTAGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGATCCACGGTATAGCAGTGGCTGGTACTGGGGCTCACTACTTTGACTACTGGGGCCAGGGAACCCTGGCCACCGTCTCCTCA |
| | | | SEQ ID NO: 28113 | SEQ ID NO: 32119 |
| | | AA | DIQMTQSPSSVSASIGDRVTITCRASQFISSWLAWYQQKPGKAPKLLIYTASSLQSGVPSRFSGSGSGTDFTLTISSLQPEDFATYCCQQANSFPWTFGQGTKVEFN | EVQLLESGGGLVQPGGSLRLSCAASGFTFSSYAMSWVRQAPGRGLEWVSAISGSGGNTFYADSVKGRFTISRDNSKNTLYLQMNSLRAEDTAVYYCAIHGIAVAGTGAHYFDYWGQGTLATVSS |
| | | | SEQ ID NO: 28114 | SEQ ID NO: 32120 |
| **iPS:434553** | **21-225_76H12** | NA | GACATCCAGATGACCCAGTCTCCATCCTCCCTGTCTGCATCTGTGGGAGACAGAGTCACCATCACTTGCCGGGCAAGTCAGGGCATTAGAAATGATTTAGGCTGGTTTCAGCAGAAACCAGGGAAAGCCCCCAAGCGCCTGATCTATGCTGCATCCAGATTGCAAAGTGGGGTCCCATCAAGGTTCAGCGGCAGTGGATCTGGGACAGAATTCACTCTCACAATCAGCAGCCTGCAGCCTGAAGATTTTGCAACTTATTACTGTCTACAGCATAATGATTACCCATTCACTTTCGGCCCTGGGACCAAAGTGGATATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAGTTATGATATCAACTGGGTGCGACAGGCCACTGGACAAGGGCTTGAGTGGATGGGATGGCTGAACCCTCACAGTGGTAACACAGGCTATGCACAGAAGTTCCAGGGCAGAATCACCATGACCTGGAACACCTCCATACGCACTGCCTACATGGAGCTGAGCAGCCTGAGATCTGAGGACACGGCCGTGTATTACTGTGCGAGAGGGTTTTACGATATTTTGACTGGTTATTCCCCCACCTACTACTACTACGATATGGACGTCTGGGGCCAAGGGACCACGGTCACCGTCTCCTCA |

FIGURE 50

| | | | | |
|---|---|---|---|---|
| | | | SEQ ID NO: 28115 | SEQ ID NO: 32121 |
| | | AA | DIQMTQSPSSLSASVGDRVTITCRASQGIRNDLGWFQQKPGKAPKRLIYAASRLQSGVPSRFSGSGSGTEFTLTISSLQPEDFATYYCLQHNDYPFTFGPGTKVDIK | QVQLVQSGAEVKKPGASVKVSCKASGYTFTSYDINWVRQATGQGLEWMGWLNPHSGNTGYAQKFQGRITMTWNTSIRTAYMELSSLRSEDTAVYYCARGFYDILTGYSPTYYYYDMDVWGQGTTVTVSS |
| | | | SEQ ID NO: 28116 | SEQ ID NO: 32122 |
| **iPS:434927** | **21-225_86E5** | NA | GACATCCAGATGACCCAGTCTCCATCCTCCCTGTCTGCATCTGTAGGAGACAGAGTCACCATCACTTGCCGGGCAAGTCAGGGCATTAGAAATGATTTAGGCTGGTATCAGCAGAAACCAGGGAAAGCCCCTAAGCGCCTGATCTATGCTGCATCCAGTTTGCAAAGTGGGGTCCCATCAAGGTTCAGCGGCAGTGGATCTGGGACAGAATTCACTCTCACAATCAGCAGCCTGCAGCCTGAAGATTTTGCAACTTATCACTGTCTACAGCATAATGATTACCCATTCACTTTCGGCCCTGGGACCAAAGTGGAAATCAAA | CAGGTGCAGCTGGTGCAGTCTGGGGCTGAGGTGAAGAAGCCTGGGGCCTCAGTGAAGGTCTCCTGCAAGGCTTCTGGATACACCTTCACCAGTTATGATATCAACTGGGTGCGACAGGCCACTGGACAAGGGCTTGAGTGGATGGGATGGATGAACCCTAACAGTGGTAACACAGGCTATGCACAGAAGTTCCAGGGCAGAGTCACCATGACCTGGAACACCTCCATACGCACTGCCTACATGGAGCTGAGCAGCCTGAGATCTGAGGACACGGCCGTGTATTACTGTGCGAGAGGGTTTTACGATATTTTGACTGGTTATTCCCCCACCTACTACTACTACGATATGGACGTCTGGGGCCAAGGGACCACGGTCACCGTCTCCTCA |
| | | | SEQ ID NO: 28117 | SEQ ID NO: 32123 |
| | | AA | DIQMTQSPSSLSASVGDRVTITCRASQGIRNDLGWYQQKPGKAPKRLIYAASSLQSGVPSRFSGSGSGTEFTLTISSLQPEDFATYHCLQHNDYPFTFGPGTKVEIK | QVQLVQSGAEVKKPGASVKVSCKASGYTFTSYDINWVRQATGQGLEWMGWMNPNSGNTGYAQKFQGRVTMTWNTSIRTAYMELSSLRSEDTAVYYCARGFYDILTGYSPTYYYYDMDVWGQGTTVTVSS |
| | | | SEQ ID NO: 28118 | SEQ ID NO: 32124 |

FIGURE 50

| | | | | |
|---|---|---|---|---|
| **iPS:435385** | **21-225_149G7** | NA | GACATCCAGATGACCCAGTCTCCATCTTCCGTGTCTGCATCTGTAGGAGACAGAGTCACCATCACTTGTCGGGCGAGTCAGTTTATTAGCAGCTGGTTAGCCTGGTATCAGCAGAAACCAGGGAAAGCCCCTAAGTTCCTGATCTATGCTGCATCCAGTTTGCAAAGTGGGGTCCCATCAAGGTTCAGCGGCAGTGGATCTGGGACAGATTTCACTCTCACCATCAGCAGCCTGCAGCCTGAAGATTTTGCAACTTACTATTGTCAACAGGCTAACAGTTTCCCGTGGACGTTCGGCCAAGGGACCAAGGTGGAAATCAAC | GAGGTGCAGCTGTTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTAGTGGTGGTAACACATTCTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGATCCACGGTATAGCAGTGGCTGGTACTGGGGCTCACTACTTTGACTACTGGGGCCAGGGAACCCTGGCCACCGTCTCCTCA |
| | | | SEQ ID NO: 28119 | SEQ ID NO: 32125 |
| | | AA | DIQMTQSPSSVSASVGDRVTITCRASQFISSWLAWYQQKPGKAPKFLIYAASSLQSGVPSRFSGSGSGTDFTLTISSLQPEDFATYYCQQANSFPWTFGQGTKVEIN | EVQLLESGGGLVQPGGSLRLSCAASGFTFSSYAMSWVRQAPGKGLEWVSAISGSGGNTFYADSVKGRFTISRDNSKNTLYLQMNSLRAEDTAVYYCAIHGIAVAGTGAHYFDYWGQGTLATVSS |
| | | | SEQ ID NO: 28120 | SEQ ID NO: 32126 |

FIGURE 51 (Table 4)
Standard IgG Antibody Variable Region Protein Alignment
CDRs defined by Kabat
Alignment numbering defined by Amgen Reference (AHo)

| KAPPA_ VARIABLE | | Germline | SEQ ID NO: | K_FR1 | K_CDR1 | K_FR2 | K_CDR2 | K_FR3 | K_CDR3 | K_FR4 |
|---|---|---|---|---|---|---|---|---|---|---|
| | VK4\|B3/JK3 | | 32127 | DIVMTQSPDSLAV SLGERATINC | KSS-- QSVLYSSNNKN YLA | WYQQKPGQ PPKLLIY | W-------- ASTRES | GVPDRFSGSGSG-- TDFTLTISSLQAEDVAVYYC | QQYYS----------- ------------TPFT | FGPGT KVDIK |
| iPS:426 126 | 21-225_6G6 | VK4\|B3/J K3 | | DIVMTQSPDSLAV SLGERATINC | KSS-- QSVLHNSNNYN YLA | WYQQKPGQ PPNLLIF | W-------- ASTRES | GVPDRFSGSGFG-- TDFTLNISSLQAEDVAVYYC | QQYYD----------- ------------TPFT | FGHGT KVDIK |
| iPS:412 232 | 21-225_4A2 | VK4\|B3/J K3 | | DIVMTQSPDSLAV SLGERATINC | KSS-- QSILHSSNNNN YLA | WFQQKPGQ PPKLLLY | W-------- ASTRES | GVPDRFSGSGSG-- TDFTLTISSLQPEDVAVYYC | QQYYN----------- ------------TPVT | FGPGT KVGIK |
| iPS:451 141 | 21-225_164B1 1 | VK4\|B3/J K3 | | DIVMTQSPDSLAV SLGERATINC | KSS-- QSLLKSSNNKS YLA | SYQQKPGQ LPKLLIY | W-------- ASSRES | GVPDRFSGSGSG-- TDFTLTISSLQAEDVALYYC | QQYYS----------- ------------IPPT | FGHGT NVDIT |
| iPS:423 314 | 21-225_12F11 | VK4\|B3/J K3 | | DIVMTQSPDSLAV SLGERATINC | KSS-- QSVLHSSNNYN YLA | WYQQKPGQ PPNLLIF | W-------- ASTRES | GVPDRFSGSGFG-- TDFTLNISSLQAEDVAVYYC | QQYYD----------- ------------TPFT | FGPGT KVDIK |
| iPS:435 327 | 21-225_147G6 | VK4\|B3/J K3 | | DIVMTQSPDSLAV SLGERATINC | KSS-- QSVLYSSNSNN YLA | WYQQKPGQ PPKLLIY | W-------- ASARES | GVPDRFSGSGSG-- TDFTLTISSLQAEDVAVYYC | QQYYT----------- ------------TPPT | FGPGT KVDIK |
| iPS:435 345 | 21-225_148G3 | VK4\|B3/J K3 | | DIVMTQSPDSLAV SLGERATIHC | KSS-- QRVLHSSNNYN YLA | WYQQKPGQ PPKLLIY | W-------- ASTRDS | GVPDRFSGSGSG-- ADFTLTISSLQAEDVALYYC | QQYYS----------- ------------TPFT | FGPGT KVDIK |
| iPS:435 405 | 21-225_150B7 | VK4\|B3/J K3 | | DIVMTQSPDSLAV SLGERATINC | KSS-- QNVLYSSHNNN YLA | WYQQKPGQ PPKLLIY | W-------- ASTRKS | GVPDRFSGSGSG-- TDFTLTITSLQAEDVAVYYC | QQYYS----------- ------------TPFT | FGPGT KVDIK |
| iPS:435 433 | 21-225_152E3 | VK4\|B3/J K3 | | DIVMTQSPDSLAV SLGERATINC | KSS-- QSVLHSSNNYN YLV | WYQQKPGQ SPKRLIY | W-------- ASTRES | GVPDRFSGSGSG-- TDFSLTISSLQAEDVAVYYC | QQYYS----------- ------------TPFT | FGPGT KVDIK |
| iPS:435 437 | 21-225_152F4 | VK4\|B3/J K3 | | DIVMTQSPDSLAV SLGERATINC | KSS-- QSVLYSSNNYN YLA | WYQQKPGQ PPKLLIY | W-------- ASTRKS | GVPDRFSGSGSG-- TDFTLTVSSLQAEDVAVYYC | QQYFN----------- ------------TPPT | FGPGT KVDIK |
| iPS:435 649 | 21-225_165H2 | VK4\|B3/J K3 | | DIVMTQSPDSLTV SPGERATINC | KSS-- QSVLHSSNNKN YLT | WYQQKPGQ PPKLLIY | W-------- ASTRES | GVPVRFSGSGSG-- TDFTVPISSMQDDDVAVYYR | QQSYS----------- ------------IPPT | FGPGT NVDIK |
| iPS:435 855 | 21-225_191G3 | VK4\|B3/J K3 | | DIVMTQSPDSLAV SLGERATIDC | KSS-- QSVLHSSNSYN YLA | WYQQKLGQ PPKLLIY | W-------- ASTRKS | GVPDRFSGSGSG-- TDFTLTISSLQAEDVAFYYC | QQYYS----------- ------------SPPT | FGPGT KMDIK |

| | | | | | | | | | | |
|---|---|---|---|---|---|---|---|---|---|---|
| **iPS:435 903** | **21-225_190E2** | **VK4\|B3/JK3** | | DIVMTQSPDSLAV SLGERATINC | KSS-- QSVLFNSNNKN YLA | WYQQKPGQ PPNLLIY | W-------- ASTRES | GVPDRFSGSGSG-- TDFTLTISSMQAEDVAVYYC | QQYCS----------- ------------LPFT | FGPGT KVDIR |
| **iPS:435 915** | **21-225_190H4** | **VK4\|B3/JK3** | | ANVMTQSPDSLAV SLGERTTINC | KSS-- QSVLHSSNNYN YLA | WYRQKPGQ PPKLLIY | W-------- ASTRES | GVPDRFSGSGSG-- TDFTLTISSLQAEDVAVYYC | QQYYS----------- ------------IPPT | FGPGT KVDIK |
| **iPS:435 923** | **21-225_190H6** | **VK4\|B3/JK3** | | DIVMTQSPDSLAV SLGERATINC | KSS-- QSVLFNSNNKN YLA | WYQQKPGQ PPNLLIY | W-------- ASTRES | GVPDRFSGSGCG-- TDFTLTISSLQAEDVAVYYC | QQYCS----------- ------------LPFT | FGPGT KVDIR |
| **iPS:435 953** | **21-225_191B12** | **VK4\|B3/JK3** | | DIVMTQSPDSLAV SLGERATINC | KSS-- QSVLFNSNNKN YLA | WYQQKPGQ PPKLLIY | W-------- ASTRES | GVPDRFSGSGSG-- TDFTLTISNLQAEDVAIYYC | QQYSS----------- ------------LPFT | FGPGT KVDIK |
| **iPS:436 098** | **21-225_195G11** | **VK4\|B3/JK3** | | DIVMTQSPDSLAV SLGERATINC | KSS-- QSVLFNSNNKN YLA | WYQQKPGQ PPNLLIY | W-------- ASTLES | GVPDRFSGSGCG-- TDFTLTISSMQAEDVAVYYC | QQYCS----------- ------------FPFT | FGPGT KVDIR |
| **iPS:436 102** | **21-225_196B1** | **VK4\|B3/JK3** | | DIVMTQSPDSLAV FLGERATINC | KSS-- QSILFSSNNKR YLA | WYQQKPGQ PPKLLIY | W-------- ASIRES | GVPDRFSGSGSG-- TDFTLTISSLQAEDVAVYYC | QQYSS----------- ------------LPFT | FGPGT KVDIK |
| **iPS:436 104** | **21-225_196C1** | **VK4\|B3/JK3** | | DIVMTQSPDSLAV SLGERATINC | KSS-- QSVLFNSNNKN YLA | WYQQKPGQ LPNLLIY | W-------- ASTLES | GVPDRFSGSGCG-- TDFTLTISSMQAEDVAVYYC | QQYCS----------- ------------FPFT | FGPGT KVDIR |
| **iPS:436 156** | **21-225_197C8** | **VK4\|B3/JK3** | | DIVMTPSPDSLAV SLGERATINC | KSS-- QSVLHSSNNKN YLA | WYQQKPGQ PPKLLIY | W-------- ASTRES | GVPDRFSGSGSG-- TDFTLSISSLQAEDVAVYYC | QQSYT----------- ------------IPFT | FGPGT KVDNK |
| **iPS:436 270** | **21-225_203F10** | **VK4\|B3/JK3** | | DIVMTQSPDSLAV SLGERATINC | KSS-- QSVFFHSNNKN YLA | WYQQKPGQ PPKLLIY | W-------- ASTRES | GVPDRFSGSGSG-- TDFTLTISSLQAEDVTVYYC | QQYFS----------- ------------LPFT | FGPGT KVDIT |
| **iPS:436 570** | **21-225_225F4** | **VK4\|B3/JK3** | | DIVMTQSPDSLAV SLGERATINC | KSS-- QSVLYSSNNKN YLA | WYQQKPGQ PPKLLIY | W-------- ASTRES | GVPDRFTGSGSG-- TDFTLTISCVQPEDVAVYYC | HQYHN----------- ------------SPPT | FGHGT EVDIK |
| **iPS:394 065** | **21-225_11E2** | **VK4\|B3/JK3** | | DIVMTQSPDSLAV SLGERATINC | KSN-- QRVLSSSNNHN YLA | WYQQRPGQ PPKLLIY | W-------- ASTRES | GVPDRFSGSGSG-- TDFTLTISSLQAEDVAVYYC | QQYFS----------- ------------TPFT | FGPGT KVDIK |
| | | **Germline** | | **K_FR1** | **K_CDR1** | **K_FR2** | **K_CDR2** | **K_FR3** | **K_CDR3** | **K_FR4** |
| | **VK1\|A30/JK5** | | 32126 | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPIT | FGQGT RLEIK |
| **iPS:473 253** | **21-225_7C3_LC1** | **VK1\|A30/JK5** | | DIQMTQSPSSLSA SVGDRVTITC | RAS--QGIR-- ----SDLG | WYQQNPVK APKRLIY | A-------- ASSLQS | GVPSRFSGSGSG-- TEFTLTVSSLQPEDFAFYYC | LQHNS----------- -----------YLPIT | FGQGT RLEIK |
| **iPS:473 256** | **21-225_9F12_LC2** | **VK1\|A30/JK5** | | DIQMTQSPSSLSA SVGDRVTITC | RAS--QGIR-- ----NDLG | WYQQKPVK APKRLIY | A-------- ASSLQS | GVPSRFSGSGSG-- TEFTLTISSLQPEDFATYYC | LQHNS----------- -----------YLPIT | FGQGT RLEIK |
| **iPS:453 449** | **21-225_208A2** | **VK1\|A30/JK5** | | DIQMTQSPSSLSA SVGDRVTITC | RTS--QGIR-- ----NDLG | WYQQQPGK TPKRLIY | A-------- ASSLLS | GVPSRFSGSRSG-- TDFTLTISSLQPEDFATYYC | LQYNS----------- ------------YPPT | FGQGT RLEIK |
| **iPS:434 467** | **21-225_73H8** | **VK1\|A30/JK5** | | DIQMTQSPSSLYA SVGDRVTITR | RAS--QDIR-- ----NDLG | WYQQKPGK ALKRVIY | A-------- ASSLQS | GVPSSFSGSGSG-- TEFTLTISSLQPEDFATYYG | IQHNS----------- -----------YPPIT | VGQGT RLEIK |

| | | | | | | | | | | |
|---|---|---|---|---|---|---|---|---|---|---|
| iPS:435<br>561 | 21-<br>225_159F1 | VK1\|A30/<br>JK5 | | DIQMTQSPSSLSA<br>SVGDRVTITC | RAS--QRVR--<br>----NDLG | WYQQKPAK<br>APKRIIF | D--------<br>ASNLQS | GVPSRFSGSGSG--<br>TEFTLTISSLQPEDFATYYC | LQHHS-----------<br>------------FPIT | FGQGT<br>RLEIK |
| iPS:436<br>328 | 21-<br>225_207F12 | VK1\|A30/<br>JK5 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYFC | LQHNS-----------<br>------------YPLT | FGQGT<br>RLEIK |
| iPS:436<br>354 | 21-<br>225_210G1<br>0 | VK1\|A30/<br>JK5 | | DIQMTQSPSSLSA<br>SVGDRVTITF | RTS--QGIR--<br>----NDLG | WYQQQPGK<br>TPKRMIY | A--------<br>ASSLFS | GVPSRFSGSRSG--<br>TDFTLTISSLQPEDFATYYC | LQYNS-----------<br>------------YPPT | FGQGT<br>RLEIK |
| iPS:393<br>094 | 21-<br>225_34C4 | VK1\|A30/<br>JK5 | | DIQMTQSPSSLSA<br>SVGDRVTITC | RAS--QGIR--<br>----NDLG | WYQQKPGK<br>APKRLIY | T--------<br>ASNLQS | GVPSRFSGSGSG--<br>TEFTLTISSLQPEDFATYYC | LQHSS-----------<br>------------YPIT | FGQGT<br>RLEIK |
| iPS:398<br>484 | 21-<br>225_18D4 | VK1\|A30/<br>JK5 | | DIQMTQSPSSLSA<br>SVGDRVTITC | RAS--QGIR--<br>----NDLG | WYQQKPGK<br>APKRLIY | A--------<br>ASSLES | GVPSRFSGSGSG--<br>TEFTLTVSSLQPEDFATYYC | LHHNN-----------<br>-----------YLPIT | FGQGT<br>RLEIK |
| | | **Germline** | | **K_FR1** | **K_CDR1** | **K_FR2** | **K_CDR2** | **K_FR3** | **K_CDR3** | **K_FR4** |
| | **VK1\|L5/JK3** | | 32129 | DIQMTQSPSSVSA<br>SVGDRVTITC | RAS--QGIS--<br>----SWLA | WYQQKPGK<br>APKLLIY | A--------<br>ASSLQS | GVPSRFSGSGSG--<br>TDFTLTISSLQPEDFATYYC | QQANS-----------<br>------------FPFT | FGPGT<br>KVDIK |
| iPS:473<br>254 | 21-<br>225_7C3_L<br>C2 | VK1\|L5/J<br>K3 | | DIQMTQSPSSVSA<br>SLGDRVTITC | RAS--QGIS--<br>----SWLA | WYQQKQGK<br>APKLLIF | A--------<br>ASRLQS | GAPSRFSGSGSG--<br>TDFTLTISSLQPEDFATYYC | QQANS-----------<br>------------FPFT | FGPGT<br>KVDIK |
| iPS:473<br>255 | 21-<br>225_9F12_<br>LC1 | VK1\|L5/J<br>K3 | | DIQMTQSPSSVSA<br>SVGDRVTITC | RAS--QGIS--<br>----RWLA | WYQQKPGK<br>APKLLIY | A--------<br>ASRLQS | GVPSRFSGSGSG--<br>TDFTLTISGLLPEDFAIYYC | QQANS-----------<br>------------FPFT | FGPGT<br>KVDFK |
| iPS:426<br>108 | 21-<br>225_10G6 | VK1\|L5/J<br>K3 | | DIQMTQSPSSVSA<br>SVGDRVTITC | RAS--QGIS--<br>----KWLA | WYQQKPGK<br>APKILIY | A--------<br>AYSLQS | GVPARFSGSGSG--<br>TDFTLTIRSLQPEDFATYYC | QQANS-----------<br>------------FPFT | FGPGT<br>KVDIK |
| iPS:426<br>110 | 21-<br>225_12E9 | VK1\|L5/J<br>K3 | | DIQMTQSPSSVSA<br>SVRDRVTITC | RAS--QGIS--<br>----SWLA | WYQQKPGE<br>APKLLIY | A--------<br>ASRLQS | GVPSRFSGSGSG--<br>TDFTLTISSLQPEDFATYYC | QQANS-----------<br>------------FPFT | FGPGT<br>KVDIK |
| iPS:453<br>447 | 21-<br>225_65F10 | VK1\|L5/J<br>K3 | | DIQMTQSPSSVSA<br>SVGDRVTITC | RGG--QGIS--<br>----TWLA | WYQQKPGK<br>APKLLIY | A--------<br>ASILQS | GVPSRFRGRGSG--<br>TDFTLTISSLQPEDFATYYC | QQGNI-----------<br>------------FPFT | FGRGT<br>KVDIK |
| iPS:453<br>451 | 21-<br>225_52G11 | VK1\|L5/J<br>K3 | | DIQMTQSPSSVSA<br>SVGDRVTITC | RAS--QGIS--<br>----KWLA | WYQQKPGK<br>APKLLLY | A--------<br>ASSLQS | GVPSRFSASGSG--<br>TDFTLTISSLQPEDFATYYC | QQANS-----------<br>------------FPFT | FGPGT<br>KVDVK |
| iPS:453<br>453 | 21-<br>225_53F2 | VK1\|L5/J<br>K3 | | DIQMTQSPSSVSA<br>SVGDRVTITC | RAS--QGIS--<br>----KWLA | WYQQKPGK<br>APNLLLY | A--------<br>ASSLQS | GVPSKFSGSGSG--<br>TDFTLTISSLQPEDFATYYC | QQANS-----------<br>------------FPFT | FGPGT<br>KVDVK |
| iPS:433<br>915 | 21-<br>225_43H9 | VK1\|L5/J<br>K3 | | DIQMTQSPSSVSA<br>SVGDRVTITC | RAS--QDIS--<br>----SWLA | WYQKKPGK<br>APKLLIY | D--------<br>ASSLQS | GVPSRFSGSGSG--<br>TDFILTISSLQPEDFATYFC | QQANS-----------<br>------------LPFT | FGPGT<br>KVDIK |
| iPS:433<br>925 | 21-<br>225_44F3 | VK1\|L5/J<br>K3 | | DIQMTQSPSSVSA<br>SVGDRVTITC | RAS--QGIS--<br>----DWLA | WYQQKPGK<br>APKLLIY | A--------<br>ASSLQS | GVPSRFSGSGFG--<br>TDFTLTISSLQPEDFATYYC | QQANS-----------<br>------------FPFT | FGPGT<br>KVDIK |
| iPS:433<br>953 | 21-<br>225_45H4 | VK1\|L5/J<br>K3 | | DIQMTQSPSSVSA<br>SVGDRVTITC | RAS--QDIS--<br>----SWLA | WYQKKPGK<br>APKYLIY | D--------<br>ASSLQS | GVPSRFSGSGSG--<br>TDFILTISSLQPEDFAIYFC | QQANS-----------<br>------------LPFT | FGPGT<br>KVDIK |
| iPS:433<br>959 | 21-<br>225_45C9 | VK1\|L5/J<br>K3 | | DIQMTQSPSSVSV<br>SVGDRVTITC | RAS--QDIS--<br>----DWLA | WYQQRPGK<br>APKLLIY | A--------<br>ASSLES | GVPSRFSGSGSG--<br>TDFTLTISSLQPEDFATYYC | QQANS-----------<br>------------FPFT | FGPGT<br>KVDIK |
| iPS:434<br>023 | 21-<br>225_49F1 | VK1\|L5/J<br>K3 | | DIQMTQSPSSVSA<br>SVGDRVTITC | RAS--RDIN--<br>----GWLA | WYQQKPGK<br>APKLLIY | T--------<br>VSSLQS | GVPSRFSGSGSG--<br>TDFTLTISSLQPEDFATYYC | QQSNS-----------<br>------------FPFT | FGPGT<br>KVDIK |
| iPS:434<br>027 | 21-<br>225_49H5 | VK1\|L5/J<br>K3 | | DIQMTQSPSSVSA<br>SVGDRVTITC | RAS--QGFS--<br>----TWLA | WFQQKPGK<br>APKLLIY | A--------<br>ASSLQD | GVPSRFSGSGSG--<br>TDFTLTISSLQPEDFATYYC | QQTNS-----------<br>------------FPFT | FGPGT<br>KVDIK |
| iPS:434<br>035 | 21-<br>225_49F10 | VK1\|L5/J<br>K3 | | DIQMTQSPSSVSA<br>SVGDRVTITC | RAS--QGIS--<br>----RWLA | WYQQKPGK<br>APKVLIY | A--------<br>ASTLQS | GVPSRFSGSGSG--<br>TDFTLTISSLQPEDFATYYC | QQANS-----------<br>------------FPFT | FGPGT<br>KVDIK |
| iPS:434<br>061 | 21-<br>225_51C7 | VK1\|L5/J<br>K3 | | DIQMTQSPSSVSA<br>SVGDRVTITC | RAS--QDVN--<br>----NYLA | WFQQKPGK<br>APKLLIY | A--------<br>ASSLQN | GVPSRFSGSGSG--<br>TDFTLTISSLLPEDFATYYC | QQTNS-----------<br>------------FPFT | FGPGT<br>KVDIK |

| | | | | | | | | | | |
|---|---|---|---|---|---|---|---|---|---|---|
| iPS:434<br>065 | 21-<br>225_50D4 | VK1\|L5/J<br>K3 | | DIQMTQSPSSVSA<br>SVGDRLTITC | RAS--QGIS--<br>----RWLA | WYQQKPGK<br>APKVLIY | A--------<br>ASTLQS | GVPSRFSGSGSG--<br>TDFTLTISSLQPEDFATYYC | QQANS-----------<br>------------FPFT | FGPGT<br>KVDIR |
| iPS:434<br>069 | 21-<br>225_51E9 | VK1\|L5/J<br>K3 | | DIQMTQSPSSVSA<br>SVGDRVTITC | RAS--QGIS--<br>----SWLA | WYQQKPGK<br>APKLLIY | V--------<br>ASSLQS | GVPSRFSGSGSG--<br>TDFTLTIRSLQPEDFATYYC | QQAKS-----------<br>------------FPFT | FGPGT<br>KVDIK |
| iPS:434<br>079 | 21-<br>225_52B1 | VK1\|L5/J<br>K3 | | DIQMTQSPSSVST<br>FVGDRITITC | RAS--QDIR--<br>----TWLA | WYQQKPGK<br>APKLLIY | A--------<br>ASSLQN | GVPSRFSGSGSG--<br>TDFTLTISSLQPEDFATYFC | QQAKS-----------<br>------------FPFT | FGPGT<br>KVDIK |
| iPS:434<br>097 | 21-<br>225_52H10 | VK1\|L5/J<br>K3 | | DIQMTQSPSSVSA<br>SVGDRVTITC | RAS--QDIN--<br>----SWLA | WYQQKPGK<br>APKLLIY | V--------<br>ASSLQS | GAPSRFSGSGSG--<br>TDFTLTIRSLQPEDFATYYC | QQAKS-----------<br>------------FPFT | FGPGT<br>KVDIK |
| iPS:434<br>123 | 21-<br>225_53F7 | VK1\|L5/J<br>K3 | | DIQMSQSPSSVSA<br>SVGDRVTITC | RAS--QGIS--<br>----RWLA | WYQQKPGK<br>APNLLIY | A--------<br>ASSLQS | GVPSRFSGSGSG--<br>TDFTLTISSLQPEDFAIYYC | QQANS-----------<br>------------FPFT | FGPGT<br>KVDIK |
| iPS:434<br>145 | 21-<br>225_55B1 | VK1\|L5/J<br>K3 | | DIQMTQSPSSVSA<br>SVGDRVTITC | RAS--QVIS--<br>----RWLA | WYQQKPGK<br>APNLLIY | A--------<br>ASSLQS | GVPSRFSGSGSG--<br>TDFTLTISSLQPEDFATYYC | QQANS-----------<br>------------FPFT | FGPGT<br>KVDLK |
| iPS:434<br>167 | 21-<br>225_50F3 | VK1\|L5/J<br>K3 | | DIQMTQSPSSVSA<br>SVGDRVTITC | RAS--QDIS--<br>----SWLA | WFQQKPGK<br>APKLLIY | A--------<br>ASSLQN | GVPSRFSGSGSG--<br>TDFTLTISSLQPEDFATYYC | QQTNS-----------<br>------------FPFT | FGPGT<br>KVDIK |
| iPS:434<br>189 | 21-<br>225_56E5 | VK1\|L5/J<br>K3 | | DIQMTQSPSSVSA<br>SVGDRVTITC | RAS--QGIR--<br>----KWLA | WYQQKPGK<br>APKLLIY | A--------<br>ASSLQS | GVPSRFSGSGSG--<br>TDFTLTISSLQPEDFATYYC | QQANS-----------<br>------------FPFT | FGPGT<br>KVDIK |
| iPS:434<br>193 | 21-<br>225_56C6 | VK1\|L5/J<br>K3 | | DIQMTQSPSSVSA<br>SVGDRVTITC | RAS--QGIS--<br>----SWLA | WYQQKSGN<br>APKLLIY | A--------<br>ASRLQS | GVPSRFSGSGSG--<br>TYFTLIISSLQSEDFATYYC | QQANS-----------<br>------------FPFT | FGPGT<br>KVDIK |
| iPS:434<br>195 | 21-<br>225_56F6 | VK1\|L5/J<br>K3 | | DIQMTQSPSSVCA<br>YVGDRVTITC | RVS--QDIS--<br>----KWLA | WFQQKPGK<br>APKFLIY | V--------<br>ASGLQS | GVPSRFSGSGSG--<br>TDFTFTISSLQPEDFATYYC | QQANS-----------<br>------------FPFT | FGPGT<br>KVDIK |
| iPS:434<br>273 | 21-<br>225_57E4 | VK1\|L5/J<br>K3 | | DIQMTQSPSSVSA<br>SVGDRVSITC | RAS--QDIS--<br>----NWLA | WYQQKPGK<br>APKLLIY | A--------<br>ASSLQS | GVPSRFSGSGSG--<br>TDFTLTISSLQPEDFATYSC | QQGNS-----------<br>------------FPFT | FGPGT<br>KVDIK |
| iPS:434<br>277 | 21-<br>225_57A7 | VK1\|L5/J<br>K3 | | DIQMTQSPSSVSA<br>SVGDRVTITC | RAS--QGIS--<br>----RWLA | WYQQKPGK<br>APNLLIY | A--------<br>ASNLQS | GVPSRFSGSGSG--<br>TDFTLTISSLQPEDFATYYC | QQANS-----------<br>------------FPFT | FGPGT<br>KVDIK |
| iPS:434<br>355 | 21-<br>225_64G12 | VK1\|L5/J<br>K3 | | DIQMTQSPSSVSA<br>SVGDRVTITC | RAS--QNIT--<br>----TWLA | WYQQKPGK<br>APKLLIS | A--------<br>ASSLQS | GVPSRFSGSGSG--<br>TDFTLTISSLQPEDFATYIC | QQANS-----------<br>------------FPFT | FGPGT<br>KLDIK |
| iPS:434<br>389 | 21-<br>225_66F11 | VK1\|L5/J<br>K3 | | DIQMTQSPSSVCA<br>SVGDRVTITC | RES--QGIS--<br>----IWLA | WYQQKPGK<br>APKLLIY | A--------<br>ASSLQS | GVPSRFSGSGYG--<br>TDFTLTISSVQPEDFATYYC | QQANS-----------<br>------------FPFT | FGPGT<br>KVDIK |
| iPS:434<br>423 | 21-<br>225_70D1 | VK1\|L5/J<br>K3 | | DIQMTQSPSSVSA<br>SVGDRVTITC | RAS--QGVS--<br>----RWLA | WYQQKPGK<br>APKLLIY | A--------<br>ASSLQS | GVPSRFSGSGSG--<br>TDFTVTISSLQPEDFATYYC | QQANS-----------<br>------------FPFT | FGPGT<br>KVDIK |
| iPS:435<br>291 | 21-<br>225_146E1 | VK1\|L5/J<br>K3 | | DIKMTQSPSSVSA<br>SVGDRVTITC | RAS--QGIN--<br>----NWLV | WYQQKPGK<br>APKLLIY | A--------<br>ASSLQS | GVPSRFRGSGSG--<br>TDFTLTISSLQPEDFATYYC | QQANS-----------<br>------------FPFT | FGPGT<br>KVDIK |
| iPS:435<br>303 | 21-<br>225_146A6 | VK1\|L5/J<br>K3 | | DIQMTQSPASVSA<br>SVGDRVTITC | RAS--QGIS--<br>----NWLA | WYQQKPGK<br>APKLLIY | A--------<br>ASSLQG | GVPSRFSGSGSG--<br>TDFTLSISSLQPEDFATYYC | QQTDS-----------<br>------------FPFT | FGPGT<br>KVDIK |
| iPS:435<br>335 | 21-<br>225_147D1<br>0 | VK1\|L5/J<br>K3 | | DIQMTQSPASVSA<br>SVGDRVTITC | RAS--QNIS--<br>----NWLT | WYQQKPGK<br>APKLLIY | A--------<br>ASSLQS | GVPSRFSGSGSG--<br>TDFTLSISSLQPEDFATYYC | QQTDS-----------<br>------------FPFT | FGPGT<br>KVDVK |
| iPS:435<br>339 | 21-<br>225_147D1<br>2 | VK1\|L5/J<br>K3 | | DIQMTQSPASVSA<br>SVGDRVTITC | RAS--QGIS--<br>----NWLA | WYQEKPGK<br>APKLLIY | A--------<br>ASSLQS | GVPSRFSGSGSG--<br>TDFTLSISSLQPEDFATYYC | QQTDS-----------<br>------------FPFT | FGPGT<br>KVDVK |
| iPS:435<br>343 | 21-<br>225_148E2 | VK1\|L5/J<br>K3 | | DIQMTQSPASVSA<br>SVGDRVTITC | RAS--QGIS--<br>----NWLA | WYQQKPGK<br>APKLLIY | A--------<br>ASSLQS | GVPSRFSGNESG--<br>TDFTLSISSLQPEDFATYYC | QQTDS-----------<br>------------FPFT | FGPGT<br>KVDVK |
| iPS:435<br>379 | 21-<br>225_149B6 | VK1\|L5/J<br>K3 | | DIQMTQSPSSVSA<br>SVGDRVTITC | RAS--QGII--<br>----SWLA | WYQQKPGK<br>APKLLIY | A--------<br>ASSLQS | GVPSRFSGSGSG--<br>TDFTLTISSLQPEDFATYYC | QQGNS-----------<br>------------FPFT | FGPGT<br>KVDIK |
| iPS:435<br>381 | 21-<br>225_149C6 | VK1\|L5/J<br>K3 | | DIQMTQSPASVSA<br>SVGDRVTITC | RAS--QGIS--<br>----NWLA | WYQQKPGR<br>APKLLIY | A--------<br>ASSLQG | GVPSRFSGSGSG--<br>TDYTLSISSLQPEDFATYYC | QQTDS-----------<br>------------FPFT | FGPGT<br>KVDIK |

| | | | | | | | | | | |
|---|---|---|---|---|---|---|---|---|---|---|
| **iPS:435 391** | **21-225_149F8** | **VK1\|L5/JK3** | | DIQMTQSPASVSA<br>SVGDRVTITC | RAS--QGIS--<br>----NWLA | WYQQKPGK<br>APKLLIY | A--------<br>ASSLQS | GVPSRFSGNESG--<br>TDFTLSISSLQPEDFAIYYC | QQTDS-----------<br>------------FPFT | FGPGT<br>KVDVK |
| **iPS:435 395** | **21-225_149D11** | **VK1\|L5/JK3** | | DIQMTQSPASVSA<br>SVGDRVTITC | RAS--QNIS--<br>----NWLT | WYQQKPGK<br>APKLLIY | A--------<br>ASSLQS | GVPSRFSGSGSG--<br>TDFTLSISSLQPEDFATYYC | QQTDS-----------<br>------------FPFT | FGPGT<br>KVDVK |
| **iPS:435 403** | **21-225_150C5** | **VK1\|L5/JK3** | | DIQMTQSPGSVSA<br>SVGDRVTITC | RAS--QGIN--<br>----NWLA | WYQQKPGK<br>APKLLIY | A--------<br>ASSLQG | GVPSRFSGSGSG--<br>TDFTLSISSLQPEDFATYYC | QQTDS-----------<br>------------FPFT | FGPGT<br>KVDIK |
| **iPS:435 447** | **21-225_152H7** | **VK1\|L5/JK3** | | DIQMTQSPASVSA<br>SVGDRVTITC | RAS--QDIS--<br>----NWLA | WYQQKPGK<br>APKLLIY | A--------<br>ASSLQG | GVPSRFSGSGSG--<br>TDFTLSISTLQPEDFATYYC | QQTDS-----------<br>------------FPFT | FGPGT<br>KVDIK |
| **iPS:435 453** | **21-225_152G10** | **VK1\|L5/JK3** | | DIQMTQSPASVSA<br>SVGDRVTITC | RAS--QGIS--<br>----NWLA | WYQQKPGK<br>APKLLIS | A--------<br>ASSLQS | GVPSRFSGNESG--<br>TDFTLSISSLQPEDFATYYC | QQTDS-----------<br>------------FPFT | FGPGT<br>KVDVK |
| **iPS:435 483** | **21-225_155A4** | **VK1\|L5/JK3** | | DIQMTQSPASVSA<br>SVGDRVTITC | RAS--QGIS--<br>----NWLA | WYHQKPGK<br>APKLLIY | A--------<br>ASSLQG | GVPSRFSGSGSG--<br>TDFTLSISSLQPEDFATYYC | HQTDS-----------<br>------------FPFT | FGPGT<br>KVDIK |
| **iPS:435 485** | **21-225_155B4** | **VK1\|L5/JK3** | | DIQMTQSPASVSA<br>SVGDRVTITC | RAS--QDIS--<br>----NWLA | WYQQKPGK<br>APKLLIY | A--------<br>ASSLQG | GVPSRFNGSGSG--<br>TDFTLSISSLQPEDFATYYC | HQTDS-----------<br>------------FPFT | FGPGT<br>KVDIK |
| **iPS:435 787** | **21-225_180A3** | **VK1\|L5/JK3** | | DIQMTQSPSSVSA<br>SVGDRVTITC | RAS--QDIT--<br>----SWLA | WYQQKPGK<br>APKLLIY | A--------<br>ASSLQS | GVPSRFSGSGSG--<br>TDFILTISSLQPEDFAFYHC | QQANS-----------<br>------------IPFT | FGPGT<br>KVDIN |
| **iPS:435 809** | **21-225_182H5** | **VK1\|L5/JK3** | | DIQMTQSPSSVYA<br>SVGDRVTITC | RAS--QDIT--<br>----SWLA | WYQQKPGK<br>APKLLIY | A--------<br>ASSLQS | GVPSRFSGSGSG--<br>TDFILTITSVQPDDFATYYC | QQVNS-----------<br>------------FPFT | FGHGT<br>KVDIK |
| **iPS:435 889** | **21-225_186A11** | **VK1\|L5/JK3** | | DIQMTQSPSSVSA<br>SVGDRVTITC | RAS--QDIT--<br>----SWLA | WYQQKPGK<br>APKLLIY | A--------<br>ASSLQS | GVPSRFSGSGSG--<br>TDFILTITSVQPDDFATYYC | QQVNS-----------<br>------------FPFT | FGHGT<br>KVDIK |
| **iPS:435 965** | **21-225_192H2** | **VK1\|L5/JK3** | | DIQMTQSPSSVSA<br>SVGDRVTITC | RAS--QGIS--<br>----SWIA | WYQQKPGK<br>APKLLIY | G--------<br>ASSLQS | GVPSRFSGSGSG--<br>TDFTLTISSLQPEDFATYYC | QQSNS-----------<br>------------FPFT | FGPGT<br>KVDVK |
| **iPS:436 106** | **21-225_196F2** | **VK1\|L5/JK3** | | DIQMTQSPSSVSA<br>SVGDRVTITC | RAS--QGIS--<br>----SWLA | WYQQKPGK<br>APKLLIY | A--------<br>ASSLQS | GVPSRFSGSGSG--<br>TDFTLTISSLQPEDFATYYC | QQTNS-----------<br>------------VPFT | FGPGT<br>KVDIK |
| **iPS:436 360** | **21-225_210H11** | **VK1\|L5/JK3** | | DIQMTQSPSSVSA<br>SVGDRVTITC | RAS--QGIS--<br>----IWLA | WYQQKPGK<br>APNLLIY | A--------<br>ASSLQS | GVPSRFSGRGSG--<br>TDFTLTISSLQPEDFATYYC | QQAKS-----------<br>------------FPFT | FGPGT<br>KVDIK |
| **iPS:436 488** | **21-225_221A6** | **VK1\|L5/JK3** | | DIQMTQSPSSVSA<br>SVGDRVTITC | RAS--QGIS--<br>----SWLA | WYQQKPGK<br>APKLLIY | T--------<br>ASNLQS | GVPSRFSGSGSG--<br>TDFTLTISSLQPEDFATYYC | QQANS-----------<br>------------FPFT | FGPGT<br>KVDIK |
| **iPS:436 496** | **21-225_222E1** | **VK1\|L5/JK3** | | DIQMTQSPSSVSA<br>SVGDRVTITC | RAS--QGIS--<br>----SWLA | WYQQKPGK<br>APKLLIY | T--------<br>ASNLQS | GVPSRFSGSGSG--<br>TDFTLTISSLQPEDFATYYC | QQANS-----------<br>------------FPFT | FGRGT<br>KVDIK |
| **iPS:436 508** | **21-225_222F7** | **VK1\|L5/JK3** | | DIQMTQSPSSVSA<br>SVGDRVTITC | RAS--QGIS--<br>----SWLA | WYQQKPGK<br>APKLLIY | T--------<br>ASNLQS | GVPSRFSGSGSG--<br>TDFTLTISSVQPEDFATYYC | QQANS-----------<br>------------FPFT | FGPGT<br>KVDIK |
| **iPS:436 516** | **21-225_222C12** | **VK1\|L5/JK3** | | DIQMTQCPSSVSA<br>SVGDRVTITC | RVS--QGIS--<br>----SWLA | WYQQKPGK<br>ALKLVIY | T--------<br>ASNLQS | GVPSRFSGSGSG--<br>TDFTLTISSVQREDFATYYC | QQDNS-----------<br>------------FPFT | FGRGT<br>KVDIK |
| **iPS:437 234** | **21-225_64E2** | **VK1\|L5/JK3** | | DIQMTQSPSSVSA<br>SVGDRVTITC | RAS--QGIS--<br>----RWLA | WYQQKPGK<br>APKLLIY | A--------<br>ASSLQS | GVPSRFSGSGSG--<br>TDFTLTISSLQPEDFATYYC | QQANS-----------<br>------------FPFT | FGPGT<br>KVDIK |
| **iPS:392 996** | **21-225_28B1** | **VK1\|L5/JK3** | | DIQMTQSPSSVSA<br>SVGDRVTITC | RAS--QAIN--<br>----DWLA | WYQQKPGK<br>APKLLIY | A--------<br>ASSFQS | GVPSRFSGSGSG--<br>TDFTLTITSLQPEDFATYYC | QQASS-----------<br>------------FPFT | FGPGT<br>KVDIK |
| **iPS:393 010** | **21-225_25E11** | **VK1\|L5/JK3** | | DIQMTQSPSSVSA<br>SVGDRVTITC | RAS--QGIS--<br>----NWLA | WYQQKPGK<br>APKLLIY | T--------<br>ASSLQG | GVPSRFSGSGSG--<br>TDFTISISSLQPEDFATYYC | QQANS-----------<br>------------FPIT | FGPGT<br>KVDIK |
| **iPS:393** | **21-** | **VK1\|L5/J** | | DIQMTQSPSSVSA | RAS--QGIS-- | WYQQKPGK | A-------- | GVPSRFRGSGSG-- | QQANS----------- | FGPGT |

| | | | | | | | | | | |
|---|---|---|---|---|---|---|---|---|---|---|
| **iPS:393 024** | **21-225_31H9** | **VK1\|L5/JK3** | | DIQMTQSPSSVSA<br>SVGDRVTITC | RAS--QGIT--<br>----SWLT | WYQQRPGK<br>APKLLIY | D--------<br>TSSLQS | GVPSRFSGSGSG--<br>TDFIFTISSLQPEDFATYYC | QQGNS-----------<br>------------FPFT | FGQGT<br>KVDIK |
| **iPS:393 080** | **21-225_34F3** | **VK1\|L5/JK3** | | DIQMTQSPSSVSA<br>TVGDRVTSTC | RAS--QGIS--<br>----KWLA | WYQQKPGK<br>APKLLIY | A--------<br>ASSLQS | GVPSRFSGSGSG--<br>TDFTLTISSLQPEDSATYYC | QQANS-----------<br>------------FPFT | FGPGT<br>KVDIK |
| **iPS:393 084** | **21-225_35C6** | **VK1\|L5/JK3** | | DIQMTQSPSSVSA<br>SVGDRVTITC | RAS--QGIS--<br>----KWLA | WYQQKPGK<br>APKPLIY | A--------<br>ASSLQS | GVPTRFSGSGSG--<br>TDFTLTISSLQPEDFAIYYC | QQANS-----------<br>------------FPFT | FGPGT<br>KVDIK |
| **iPS:393 086** | **21-225_36H5** | **VK1\|L5/JK3** | | DIQMTQSPSSVSA<br>SVGDRVTITC | RAS--QGIS--<br>----RWLA | WYQQKPGK<br>APELLIY | A--------<br>ASRLQS | GIPSRFSGSGSG--<br>TDFTLTISSLQPEDFATYYC | QQANS-----------<br>------------FPFT | FGPGT<br>KVDIK |
| **iPS:393 098** | **21-225_35G6** | **VK1\|L5/JK3** | | DIQMTQSPSSVSA<br>SVGDRLTITC | RAS--QGIS--<br>----RWLA | WYQQKVGK<br>VPKLLIY | A--------<br>ASRLQS | GVPSRFSGSGSG--<br>TAFTLTIGSLQPEDFATYYC | QQANS-----------<br>------------FPFT | FGPGT<br>KVDLK |
| **iPS:393 112** | **21-225_33G1** | **VK1\|L5/JK3** | | DIQMTQSPSSVSV<br>SVGDRVTITC | RAS--QGIS--<br>----RWLA | WYQQKPGK<br>APKLLIY | G--------<br>AYSLQS | GVPSRFSGSGSG--<br>TDFTLTISSLQPEDFATYYC | QQANS-----------<br>------------FPFT | FGPGT<br>KVDIK |
| **iPS:393 116** | **21-225_34G7** | **VK1\|L5/JK3** | | DIQMTQSPSSVSA<br>SVGDRVTITC | RAS--QLIS--<br>----KWLA | WYQQKPGK<br>APKLLIY | A--------<br>ASSLQS | GVPLRFSGSGSG--<br>TDFTLTISSLQPEDFATYYC | QQANS-----------<br>------------FPFT | FGPGT<br>KVDIK |
| **iPS:393 132** | **21-225_33H7** | **VK1\|L5/JK3** | | DIQMTQSPSSVSA<br>SVGDRVTITC | RAS--QGIS--<br>----RWLA | WYQQKVGK<br>VPKLLIY | A--------<br>ASRLQS | GVPSRFSGSGSG--<br>TDFTLTIGSLQPEDFATYYC | QQANI-----------<br>------------FPFT | FGPGT<br>KVDLK |
| **iPS:393 140** | **21-225_35H12** | **VK1\|L5/JK3** | | DIQMTQSPSSVSA<br>SVGDRVTITC | RAS--QGIS--<br>----RWLA | WYQQKPGK<br>APELLIY | A--------<br>ASRLQS | GIPSRFSGSGSG--<br>TDFTLTISSLQPEDFATYYC | QQANS-----------<br>------------FPFT | FGPGT<br>KVDIK |
| **iPS:393 954** | **21-225_4H6** | **VK1\|L5/JK3** | | DIQMTQSPSSVSA<br>SVGDRVTITC | RAS--QGIS--<br>----RWLA | WYQQKPGK<br>APKLLIY | G--------<br>ASSLQS | GVPSRFSGSGSG--<br>TDFTLTISSLQPEDFAIYYC | QQANS-----------<br>------------FPFT | FGPGT<br>KVDIK |
| **iPS:398 502** | **21-225_23B11** | **VK1\|L5/JK3** | | DIQMTQSPSSVSA<br>SVGDRVTITC | RAS--QGIT--<br>----KWLA | WYQQKPGK<br>APKVLIY | A--------<br>ASSLQS | RVPSRFSGSRSG--<br>TDFTLTISSLQPEDFATYYC | QQANS-----------<br>------------FPFT | FGPGT<br>KVDIK |
| **iPS:398 520** | **21-225_31C4** | **VK1\|L5/JK3** | | DIQMTQSPSSVSA<br>SVGDRVTITC | RAS--QGIS--<br>----KWLA | WYQQKPGK<br>APKPLIY | A--------<br>ASSLQS | GVPTRFSGSGSG--<br>TDFTLTISSLQPEDFAIYYC | QQANS-----------<br>------------FPFT | FGPGT<br>KVDIK |
| **iPS:402 223** | **21-225_30A11** | **VK1\|L5/JK3** | | DIQMTQSPSSVSA<br>SVGDRVTITC | RAS--QGIS--<br>----RWLA | WYQQKPGR<br>APELLIY | A--------<br>ASRLQS | GIPSRFSGSGSG--<br>TDFTLTISSLQPEDFATYYC | QQANS-----------<br>------------FPFT | FGPGT<br>KVDNK |
| | | **Germline** | | **K_FR1** | **K_CDR1** | **K_FR2** | **K_CDR2** | **K_FR3** | **K_CDR3** | **K_FR4** |
| | **VK4\|B3/JK1** | | 32130 | DIVMTQSPDSLAV<br>SLGERATINC | KSS--<br>QSVLYSSNNKN<br>YLA | WYQQKPGQ<br>PPKLLIY | W--------<br>ASTRES | GVPDRFSGSGSG--<br>TDFTLTISSLQAEDVAVYYC | QQYYS-----------<br>------------TPWT | FGQGT<br>KVEIK |
| **iPS:426 112** | **21-225_12F12** | **VK4\|B3/JK1** | | DIVMTQSPDSLAV<br>SLGERATINC | KSS--<br>QTVLFSSNNNH<br>YLA | WYQQKPGQ<br>PPNLLIY | W--------<br>ASTRAS | GVPDRFSGSGSG--<br>TDFTLTISSLQAEDVAVYYC | QQYYS-----------<br>------------SPWT | FGQGT<br>KVEIK |
| **iPS:451 137** | **21-225_74A7** | **VK4\|B3/JK1** | | DIVMTQSPDSLAV<br>SLGERATINC | KSS--<br>QSVLFSSNNYN<br>YLA | WYQQRPGQ<br>PPNLLIY | W--------<br>ASTRES | GVPDRFSGSGSG--<br>TDFTLTISSLQAADVAVYYC | QQYHS-----------<br>------------SPPT | FGQGT<br>TVQIK |
| **iPS:433 909** | **21-225_43D8** | **VK4\|B3/JK1** | | DIVMTQSPDSLAV<br>SLGERATINC | KSS--<br>QSVLMTSNDKN<br>YLT | WYQQRPGQ<br>PPKLLIY | W--------<br>ASTRES | GVPDRFSGGGSG--<br>TDFTLTISGLQAEDVAVYYC | QQYYS-----------<br>------------TPPT | FGQGT<br>KVEIK |
| **iPS:434 177** | **21-225_56A1** | **VK4\|B3/JK1** | | DIVMTQSPDSLAV<br>SLGERATINC | KSS--<br>QSVLHSSNNKN<br>YLV | WYQQRPGQ<br>PPNLLIY | W--------<br>ASTRES | GVPDRFSGSGSG--<br>TDFTLTISSLQAEDVAVYYC | QQYYS-----------<br>------------TPPT | FGQGT<br>KVEIK |
| **iPS:434 237** | **21-225_61B5** | **VK4\|B3/JK1** | | DIVMTQSPDSLAV<br>SLGERATINC | KSS--<br>QSVLYSSNNNN<br>SLT | WYQLKPGQ<br>PPKKLIY | W--------<br>ASTRES | GVPDRFSGSGSG--<br>TDFTLTISSLQAEDVAVYYC | QQYYS-----------<br>------------TPPT | FGQGS<br>KVEIK |

| | | | | | | | | | | | |
|---|---|---|---|---|---|---|---|---|---|---|---|
| **iPS:434 285** | **21-225_57A11** | **VK4\|B3/J K1** | | DIVMTQSPDSLTV SLGERATINC | KSS-- QSVLHSSNNYN YLA | WYQQRPGQ PPKLVIY | W-------- ASTRAS | GVPDRFSGSGSG-- TDFTLTISSLQAEDMAVYYC | QQYYS----------- ------------TPWT | FGQGT KVEFK |
| **iPS:434 295** | **21-225_58B9** | **VK4\|B3/J K1** | | DIVMTQSPDSLAV SLGERATINC | KSG-- QSILYSSNNNN YLA | WYQQKPGQ PPKKLIY | W-------- ASTRDS | GVPARFSGSGSG-- TDFTLTISSLQAEDVAVYYC | QQYYS----------- ------------TPPT | FGQGS KVEIK |
| **iPS:434 321** | **21-225_59F10** | **VK4\|B3/J K1** | | DIVMTQFPDSLAV SLGERATINC | KSS-- QTVLYRSNNYN YLA | WYQQKPGQ PPKLLIY | W-------- ASTRES | GVPDRFSGSGSG-- TDFTLTISSLQAEDVAVYYC | QQYFS----------- ------------TPPT | FGQGT KVEIK |
| **iPS:434 431** | **21-225_70E7** | **VK4\|B3/J K1** | | DIVMTQSPDSLAV SLGERATLNC | KSS-- QSVLYSSNNNN YLA | WYQQKPGQ PPKLLIY | W-------- ASTRES | GVPDRFSGSGSG-- TDFTLTISSLQAEDVAVYYC | QQYYN----------- ------------IPPT | FGQGT KVEIK |
| **iPS:434 475** | **21-225_74F9** | **VK4\|B3/J K1** | | DIVMTQSPDSLAV SLGERATINC | KSS-- QSVLYSSNNYN YLA | WYQQKPGQ PPKKLIY | W-------- ASTRES | GVPDRFSGSGSG-- TDFTLTISSLQAEDVAVYSC | QQYYS----------- ------------SPPT | FGQGT KVEIK |
| **iPS:434 477** | **21-225_74A6** | **VK4\|B3/J K1** | | DIVMTQSPDSLAV SPGERATINC | KSS-- QSVLHSSNNNN YLA | WYQQKPGQ PPDLLIY | W-------- ASTRES | GVPDRFSGSGSG-- TDFTLTISSLQAEDVAVYYC | QQYFS----------- ------------TPWT | FGQGT QVEIK |
| **iPS:434 481** | **21-225_74B10** | **VK4\|B3/J K1** | | DIVMTQSPDSLAV SLGERATINC | KSS-- QSVLHSSNNKN YLT | WYQQKPGQ PPKLLIY | W-------- ASTRES | GVPDRFSGSGSG-- TDFSLTIGSLQAEDVAVYYC | QQYYS----------- ------------IPPT | FGQGT KVEIK |
| **iPS:434 487** | **21-225_76G2** | **VK4\|B3/J K1** | | DIVMTQSPDSLAV SLGERATINC | KSS-- QSVLHSSNNYN YLA | WYQQRPGQ PPRLLIY | W-------- ASTRES | GVPDRFSGSGSG-- TDFTLTISSLQVEDVAVYYC | QQYYS----------- ------------SPPT | FGQGT KVEIK |
| **iPS:434 493** | **21-225_76F3** | **VK4\|B3/J K1** | | DIVMTQCPDSPAV SLGERATINC | KSS-- QSVLFSSNNYN YLA | WYQQRPGQ PHDLLIY | W-------- ASTRES | GVPDRFSGSGSG-- TDFTLTISSLQAADVAVYYC | QQYHS----------- ------------SPLT | FGQGT TVQIK |
| **iPS:434 509** | **21-225_76F5** | **VK4\|B3/J K1** | | VIVLTQSPDSLAV SLGERATINC | KSS-- QSVLHSSNSYN YLA | WYQQKPGQ SPKVLIY | W-------- TSTRES | GVPDRFSGSGSG-- TDFTLTISSLQAEDVAVYYC | QQYYS----------- ------------SPPT | FGQGT KVEIK |
| **iPS:434 525** | **21-225_76E8** | **VK4\|B3/J K1** | | DIVMTQSPDSLAV SLGERATINC | KSS-- QTVLHSSNNYN YLA | WYQQKPGQ PPKVLIY | W-------- TSTRES | GVPDRFSGSGSG-- TDFTLTISSLQAEDVAVYYC | QQYFS----------- ------------SPLT | FGQGT KVEIK |
| **iPS:434 549** | **21-225_76E11** | **VK4\|B3/J K1** | | DIVMTQSPDSLAV SLGEGATINC | KSR-- QSVLHSSNNYN YLA | WYQQKAGQ PPKLLIY | W-------- ASTRES | GVPDRFSGSGSG-- TDFTLTISSLQAEDVAVYYC | QQYYS----------- ------------TPPT | FGQGT KVEIR |
| **iPS:434 551** | **21-225_75C4** | **VK4\|B3/J K1** | | DIVMTQSPDSLAV SLGERATINC | KSS-- QSILYSSNNNN YLA | WFQQKPGQ PPKLLIY | W-------- ASTRES | GVPDRFSGSGSG-- TDFTLTISSLQAEDVAVYYC | QQYYI----------- ------------TPPT | FGQGT KVEIK |
| **iPS:434 575** | **21-225_77C7** | **VK4\|B3/J K1** | | DIVMTQSPDSLAV SLGERATINC | KSS-- QTVLHSSNNYN YLA | WYQQKPGQ PPKVLLY | W-------- TSTRES | GVPDRFSGSGSG-- TDFTLTISSLQAEDVAVYYC | QQYFS----------- ------------SPPT | FGQGT RVEIK |
| **iPS:434 597** | **21-225_77C10** | **VK4\|B3/J K1** | | DIVMTQSPDCLAV SLGERATINC | KSS-- QSVLYTSNNNN YLA | WYQQKPGQ PPKLLIY | W-------- ASTRES | GVPDRFSGSGSG-- TDFTLTISSLQAEDVAVYYC | QHYYN----------- ------------TPWK | FVQGT KVEIT |
| **iPS:434 617** | **21-225_74B8** | **VK4\|B3/J K1** | | DSVMTQSPDSLAV SLGERATINC | KSS-- QSVLHSSNKKN | WYQQKPGQ PPKLLIY | W-------- ASTRES | GVPDRFSGSGSG-- TDFTLTISSLQAEDVAVYYC | QQYYR----------- ------------TPWT | FGQGT KVEIK |

| | | | | | | | | | |
|---|---|---|---|---|---|---|---|---|---|
| **iPS:434 619** | **21-225_78C1** | **VK4\|B3/JK1** | | DIVMTQSPDCLAV SLGERATINC | KSS--QSVLYTSNNNNYLA | WYQQKPGQ PPKLLIY | W--------ASTRES | GVPDRFSGSGSG--TDFTLTISSLQAEDVAVYYC | QHYYN-----------------------TPWK | FVQGT KVEIK |
| **iPS:434 639** | **21-225_74B7** | **VK4\|B3/JK1** | | DIVMTQSPDSLAV SLGERATINC | KSS--QTVLHSSNNYNYLA | WYQQKPGQ PPKVLIY | W--------TSTRES | GVPDRFSGSGSG--TDFTLTISSLQAEDVAVYYC | QQYFS-----------------------SPPT | FGQGT KVEIK |
| **iPS:434 649** | **21-225_78E11** | **VK4\|B3/JK1** | | DIVMTQSPDSLAV SLGERATINC | KSS--QSVLYSFNNYNYLA | WYQQKPGQ PPKKLIY | W--------ASTRES | GVPDRFSGSGSG--TDFTLTISSLQAEDVAVYSC | QQYYS-----------------------SPPT | FGQGT KVEIK |
| **iPS:434 653** | **21-225_74B5** | **VK4\|B3/JK1** | | DIVMTQSPDSLAV SLGERATINC | KSS--QSVLFSSNNYNYLA | WYQQRPGQ PPNLLIY | W--------ASTRES | GVPDRFSGSGSG--TDFTLTISSLQAADVAVYYC | QQYYS-----------------------SPPT | FGQGT TVQIK |
| **iPS:434 655** | **21-225_78H12** | **VK4\|B3/JK1** | | DIVMTQSPDSLAV SLGERATINC | KSS--QTVLHSFNNYNYLA | WYQQKPGQ PPKVLIY | W--------TSTRES | GVPDRFSGSGSG--TDFTLTISSLQAEDVAVYYC | QQYFS-----------------------SPPT | FGQGT KVEIK |
| **iPS:434 665** | **21-225_74G4** | **VK4\|B3/JK1** | | DIVMTQSPDSLAV SLGERATINC | KSS--QSVLYSSNNNNYLA | WYQQKAGQ PPKLLIY | W--------ASTRES | GVPDRFSGSGSG--TDFTLTISSLQAEDVAVYYC | QQYYS-----------------------IPPT | FGQGT KVEIK |
| **iPS:434 675** | **21-225_79G6** | **VK4\|B3/JK1** | | DIVMTQSPDCLAV SLGERATISC | MSS--QSVLHSFNNKNYLT | WYQQKPGQ PPKLLIY | W--------ASTWES | GVPDRFSGSGSG--TDFSLPIGSLQAEDVAVYYC | QQYYS-----------------------IPPT | FGQGT KVEIK |
| **iPS:434 685** | **21-225_79E9** | **VK4\|B3/JK1** | | DIVMTQSPDSLAV SLGERATINC | KSS--QSILYSSNNNNYLA | WFQQKPGQ PPKLLIY | W--------ASTRES | GVPDRFSGSGCG--TDFTLTISSLQAEDVAVYYC | QQYYI-----------------------TPPT | FGQGT KVEIK |
| **iPS:434 689** | **21-225_79G10** | **VK4\|B3/JK1** | | DIVMTQSPDSLAV SLGERATINC | KSS--QSVLFSSNNYNYLA | WYQQRPGQ PHNLLIY | W--------ASTRES | GVPDRFSGSGSG--TDFTLTISSLQAADVAVYYC | QQYHS-----------------------SPLT | FGQGT TVQIK |
| **iPS:434 697** | **21-225_79F12** | **VK4\|B3/JK1** | | DIVMTQSPDSLAV SLGERATINC | KSS--QSILYSSNNYNYLA | WYQQKPGQ PPKLLIY | W--------ASTRES | GVPDRFSGSGSG--TDFTLTISSLQAEDVAVYYC | QQYYS-----------------------TPWT | FGQGT KVEIK |
| **iPS:434 707** | **21-225_80D3** | **VK4\|B3/JK1** | | DIVMTQSPDCLAV SLGERATINC | KSS--QSVLYTSNNNNYLA | WYQQKPGQ PPKLLIY | W--------ASTRES | GVPDRFSGSGSG--TDFTLTISSLQAEDVAVYYC | QHYNE-----------------------TPGK | FVQVT KVEIT |
| **iPS:434 711** | **21-225_80H3** | **VK4\|B3/JK1** | | DIVMTQSPDSLAV SLGERATTNC | KSS--QSVLHRSNNYNYLA | WYQQKPGQ PPKLLIY | W--------ASTRES | GVPDRFSGSGSG--TDFTLTISSLQAEDVAVYYC | QQYYS-----------------------TPPT | FGQGT KVEIK |
| **iPS:434 731** | **21-225_80E9** | **VK4\|B3/JK1** | | DIVMTQSPDCLAV SLGERATINC | KSS--QSVLYTSNNNNYLA | WYQQKPGQ PPKLLIY | W--------ASTRES | GVPDRFSGSGSG--TDFTLTISSLQAEDVAVYYC | QQYYN-----------------------TPWT | FVQGT KVEIK |
| **iPS:434 761** | **21-225_81E5** | **VK4\|B3/JK1** | | DIVMTQSPDSLAV SLGERATINC | KSS--QSVLFSSNNYNYLA | WYQQRPGQ PPNLLIY | W--------ASTRES | GVPDRFSGSGSG--TDFTLTISSLQAADVAVYYC | QQYYS-----------------------SPLT | FGQGT TVQIK |
| **iPS:434 771** | **21-225_81F9** | **VK4\|B3/JK1** | | DIVMTQSPDCLAV SLGERATINC | KSS--QSVLYTSNNNNYLA | WYQQKPGQ PPKLLIY | W--------ASTRES | GVPDRFSGSGSG--TDFTLTISSLQAEDVAVYYR | QHYND-----------------------TPGK | FVQGI MVEIT |
| **iPS:434 827** | **21-225_83F3** | **VK4\|B3/JK1** | | DIVMTQSPDCLAV SLGERATINC | KSS--QSVLYTSNNNN | WYQQKPGQ PPKLLIY | W--------ASTRES | GVPDRFSGSGSG--TDFTLTISSLQAEDVAVYYC | QHYND-----------------------TPWK | FVQGI KVEIK |

| | | | | | | | | | | |
|---|---|---|---|---|---|---|---|---|---|---|
| **iPS:434 829** | **21-225_83G3** | **VK4\|B3/JK1** | | DIVMTQSPDCLAV SLGERATINC | KSS--QSVLYTSNNNN YLA | WYQQKPGQ PPKLLIY | W-------- ASTRES | GVPDRFSGSGSG-- TDFTLTISSLQAEDVAVYYC | QHYYN----------- ------------TPWT | FVQGT KVEIK |
| **iPS:434 841** | **21-225_83G7** | **VK4\|B3/JK1** | | DIVMTQSPDSLAV SLGERATIIC | KSS--QTVLHSSNNYN YLA | WYQQKPGQ PPKVLIY | W-------- TSTRES | GVPDRFSGSGSG-- TDFTLTISSLQAEDVAVYYC | QQYFS----------- ------------SPLT | FGQGT KVEIK |
| **iPS:434 863** | **21-225_84G7** | **VK4\|B3/JK1** | | DIVMTQSPDSPAV SLGERATIIC | KSS--QTVLHSSNNYN YLA | WYQQKPGQ PPKVLIY | W-------- TSTRES | GVPDRFSGSGSG-- TDFTLTISSLQAEDVAVYYC | QQYFS----------- ------------SPPT | FGQGT KVEIK |
| **iPS:434 877** | **21-225_85H2** | **VK4\|B3/JK1** | | DSMMTQSPDSLAV SLGERATINC | KSS--QSVLHSSNKKN YLA | WYQQKPGQ PPKLLIY | W-------- ASTRES | GVPDRFSGSGSG-- TDFTLTISSLQAEDVAVYYC | QQYYR----------- ------------TPWT | FGQGT KVEIK |
| **iPS:434 901** | **21-225_85H9** | **VK4\|B3/JK1** | | DIVMTQSPDSLAV SLGERATTNC | KSS--QSVLHRSNNYN YLA | WYQQKPGQ PPKLLIY | W-------- ASTRES | GVPDRFSGSGSG-- TDFTLTISSLQAEDVAVYYC | QQYYS----------- ------------TPPT | FGQGT KVEIK |
| **iPS:434 935** | **21-225_86E9** | **VK4\|B3/JK1** | | DIVMTQCPDSPAV SLGERATINC | KSS--QSVLHRSNNYN YLA | WYQQKPGQ PPNLLIY | W-------- ASTRES | GVPDRFSGSGSG-- TDFTLTISSLQAADVAVYYC | QQYHS----------- ------------SPLT | FGQGT TVEIK |
| **iPS:434 965** | **21-225_88A1** | **VK4\|B3/JK1** | | NIVMTQSPDSLAV SLGARATINC | KSS--QSVLHSSNNYN YLT | WYQQKPGQ PPKLLLY | W-------- ASTRKS | GVPDRFSGSGSG-- TDFTLTISSLQAEDVAVYFC | QQYYS----------- ------------SPPT | FGQGT KVEIK |
| **iPS:434 971** | **21-225_88G2** | **VK4\|B3/JK1** | | DIVMTQSPDCLAV SLGERATINC | KSS--QSVLYTSNNNN YLA | WYQQKPGQ PPKLLIY | W-------- ASTRES | GVPDRFSGSGSG-- TDFTLTISSLQAEDVAVYYC | QQYYN----------- ------------TPWT | FVQGT KVEIK |
| **iPS:434 973** | **21-225_88B4** | **VK4\|B3/JK1** | | DIVMTQSPDSLAV SLGERATINC | KSS--QSVLYISNNNN YLA | WYQQKPGQ PPKLLIY | W-------- ASTRES | GVPARFSGSGSG-- TDFTLTISSLQAEDVAVYYC | QQYYS----------- ------------TPPT | FGQGT KVEIK |
| **iPS:434 997** | **21-225_88C10** | **VK4\|B3/JK1** | | DIVMTQSPDSLAV SLGERATINC | KSS--QSVLHSSNNWN YLA | WHQQKPGQ PPKLLIH | W-------- AFTRKS | GVPDRFSGGGSG-- TNFTLTISSLQAEDVAVYYC | QQYYR----------- ------------APPT | FGQGT KVEIK |
| **iPS:435 051** | **21-225_90D9** | **VK4\|B3/JK1** | | DIVMTQSPDSLAV SLGERATIIC | KSS--QTVLHSSNNYN YLA | WYQQKPGQ PPKVLIY | W-------- TSTRES | GVPDRFSGSGSG-- TDFTLTISSLQAEDVAVYYC | QQYLS----------- ------------SPLT | FGQGT KVEIK |
| **iPS:435 053** | **21-225_75F9** | **VK4\|B3/JK1** | | DIVMTQSPDSLPV SLGERATVNC | KSS--QSVLHNSNNNN YLA | WYQQKPGQ PPKLLIY | W-------- ASTRES | GVPDRFSGSGSG-- TDFTLTISSLQAEDVAVYYC | QQYYS----------- ------------SPPT | FGQGT KVEIK |
| **iPS:435 071** | **21-225_91F1** | **VK4\|B3/JK1** | | DIVMTQSPDCLAV SLGERATINC | KSS--QSVLYTSNNNN YLA | WYQQKPGQ PPKLLIY | W-------- ASTRES | GVPDRFSGSGSG-- TDFTLTISSLQAEDVAVYYC | QHYYN----------- ------------TPWK | FVQGT KVEIK |
| **iPS:435 087** | **21-225_91G8** | **VK4\|B3/JK1** | | DIVMTQSPDCLAV SLGERATINC | KSS--QSVLYTSNNNN YLA | WYQQKPGQ PPKLLIY | W-------- ASTRES | GVPDRFSGSGSG-- TDFTLTISSLQAEDVAVYYC | QQYYT----------- ------------TPWT | FGQGT KVEIK |
| **iPS:435 113** | **21-225_92E6** | **VK4\|B3/JK1** | | DIVMTQSPDSLAA SLGERATINC | KSS--QNILSSSNNKN YLT | WYQQKPGQ PPKILIY | W-------- TSTRES | GVPDRFSGSGFG-- TDFTLTISSLQAEDVAVYYC | QQYFS----------- ------------VPPT | FGQGT KVEIK |
| **iPS:435 167** | **21-225_92E12** | **VK4\|B3/JK1** | | DIVMTQSPDSLAV SLGERATTNC | KSS--QSVLHRSNNYN | WYQQKPGQ PPKLLIY | W-------- ASTRES | GVPDRFSGSGSG-- TDFTLTISSLQAEDVAVYYC | QQYYS----------- ------------TPPT | FGQGT KVEIK |

| | | | | | | | | | | |
|---|---|---|---|---|---|---|---|---|---|---|
| **iPS:435 203** | **21-225_75A7** | **VK4\|B3/JK1** | | DIVMTQSPDSLAV SLGERATINC | KSS-- QTVLHSSNNYN YLA | WYQQKPGQ PPKVLIY | W-------- TSTRES | GVPDRFSGSGSG-- TDFTLTISSLQAEDVAVYYC | QQYFS----------- ------------SPPT | FGQGT KVEIK |
| **iPS:435 209** | **21-225_75A10** | **VK4\|B3/JK1** | | NIVMTQSPDSLAV SLGARATINC | KSS-- QSVLHNSNNYN YLT | WYQQKPGQ PPKLLLY | W-------- ASTRKS | GVPDRFSGSGSG-- TDFTLTISSLQAEDVAVYFC | QQYYS----------- ------------SPPT | FGQGT KVEIK |
| **iPS:435 211** | **21-225_94E11** | **VK4\|B3/JK1** | | DIVMTQSPDSLAV SLGERATINC | KSS-- QSVLFSSNNYN YLA | WYQQRPGQ PPNLLIY | W-------- ASTRES | GVPDRFSGSGSG-- TDFTLTISSLQAADVAVYYC | QQYHS----------- ------------SPLT | FGQGT TVQIK |
| **iPS:435 215** | **21-225_94E12** | **VK4\|B3/JK1** | | DIVMTQSPDSLAV SLGERATTNC | KSS-- QSVLHRSNNYN YLA | WYQQKPGQ PPKLLIY | W-------- ASTRES | GVPDRFSGSGSG-- TDFTLTISSLQAEDVAVYYC | QQYYS----------- ------------TPPT | FGQGT KVEIK |
| **iPS:435 227** | **21-225_95G4** | **VK4\|B3/JK1** | | DIVMTQCPDSLAV SLGERATINC | KSS-- QSVLFRSNNYN YLA | WYQQRPGQ PHNLLIY | W-------- ASTRES | GVPDRFSGSGYG-- TDFTLTISSVQAADVAVYYC | QQYHS----------- ------------SPLT | FGQGT TVQIK |
| **iPS:435 245** | **21-225_95E12** | **VK4\|B3/JK1** | | DIVMTQSPDSLAV SLGERATINC | KSS-- QSVLYSSNNAN YLA | WYQQKPGQ PPNLFIY | W-------- ASTRES | GVPDRFSGSGSG-- TDFTLTISSLQAEDVAVYYC | QQYYS----------- ------------TPCS | FGQGT KVEIK |
| **iPS:435 249** | **21-225_96E2** | **VK4\|B3/JK1** | | DSVMTQSPDSLAV SLGERATINC | KSS-- QSVLHSSNKKN YLA | WYQQKPGQ PPKLLIY | W-------- ASTWES | GVPDRFSGSGSG-- TDFTLTISSLQAEDVAVYYC | QQYYR----------- ------------TPWT | FGQGT KVEIK |
| **iPS:435 255** | **21-225_96D5** | **VK4\|B3/JK1** | | DIVMTQSPDSLAV SLGERATINC | KSS-- QSVLHSSNNYN YLA | WYQQKPGQ PPNLLIY | W-------- ASTRES | GVPDRFSGSGSG-- TDFTLTISSLQAADVAVYYC | QQYYS----------- ------------SPPT | FGQGT TVEIK |
| **iPS:435 257** | **21-225_96H5** | **VK4\|B3/JK1** | | DIVMTQSPDSLAV SLGERATINC | KSS-- QSVLYSSNSHN YLA | WYQQKPGQ PPKLLIY | W-------- ASIRES | GVPDRFSGSGSG-- TDFTLSISSMQAEDVAVYYC | QQYYS----------- ------------TPCS | FGQGT KVEIK |
| **iPS:435 267** | **21-225_96D10** | **VK4\|B3/JK1** | | DIVMTQSPDSLAV SLGERATINC | KSS-- QNILSSSNNKN YLT | WYQQKPGQ PPKILIY | W-------- TSTRES | GVPDRFSGSGFG-- TDFTLTISSLQAEDVAVYYC | QQYFS----------- ------------VPPT | FGQGT KVEIK |
| **iPS:435 279** | **21-225_97H4** | **VK4\|B3/JK1** | | DIVMTQSPDCLAV SLGERATINC | KSS-- QSVLYTSNNNN YLA | WYQQKPGQ PPKLLIY | W-------- ASTRES | GVPDRFSGSGSG-- TDFTLTISSLQAEDVAVYYC | QHYND----------- ------------TPWK | FVQGT KVEIT |
| **iPS:435 321** | **21-225_147E4** | **VK4\|B3/JK1** | | DIVMTQSPDSLAV SLGERATINC | KSS-- QSVLHSSNNYN YLA | WYQLKPRQ PPKLLIY | W-------- ASTRES | GVPDRFSGRGSG-- TDFTLTISSLQAEDVAIYYC | QQYYS----------- ------------TPST | FGPGT KVEIK |
| **iPS:435 353** | **21-225_148F8** | **VK4\|B3/JK1** | | DIVMTQSLDSLAV SLGERATINC | KSS-- QSALHSSNNYN YLA | WYQQKPGQ PPKLLIY | W-------- ASTRKS | GVPDRFSGSGSG-- TDFTLTISSLQAEDVAVYYC | QQYYS----------- ------------IPPT | FGQGT KVEIK |
| **iPS:435 369** | **21-225_149A2** | **VK4\|B3/JK1** | | DIVMTQSPDSLAV SLGERATINC | KSS-- QSVLYSPNNNN YLA | WYQQKPGQ PPKLLIY | W-------- ASTRES | GVPDRFSGSGSG-- TDFTLTISSLQAEDVAVYYC | QQYYS----------- ------------TPCS | FGQGT KVEIK |
| **iPS:435 373** | **21-225_149E3** | **VK4\|B3/JK1** | | DIVMTQSPDSLAV SLGERATISC | KSS-- QTVLHNSNNHN YFA | WYQQKPGQ PPKLLIY | W-------- ASTLRS | GVPDRFSGSGSG-- TDFTLTISSLQAEDVAVYYC | QQYYS----------- ------------TPPT | FGQGT KVEIK |
| **iPS:435 375** | **21-225_149H4** | **VK4\|B3/JK1** | | DIVMTQSPDSLAV SLGERATINC | KSS-- QSVLSSSNDNN | WYQQKPGR PPKLLIY | W-------- SSTRES | GVPDRFSGSGSG-- TDFTLTISSLQAEDVAVYYC | HQYYS----------- ------------YPPT | FGQGT KVEIK |

| | | | | | | | | | |
|---|---|---|---|---|---|---|---|---|---|
| **iPS:435 481** | **21-225_154A1 1** | **VK4\|B3/J K1** | | DIVMTQSPDSLAV SLGERATINC | RSS-- QSVLHSSNNYN YLA | WYQQKPGQ PPKLLIY | W-------- ASKRDS | GVPDRFSGSGSG-- TDFTLTISSLQAEDVAVYYC | QQYFS----------- ------------SPRT | FGQGT KVEIK |
| **iPS:435 557** | **21-225_158B1 2** | **VK4\|B3/J K1** | | DIVMTQSPDSPAV SLGERATINC | KSS-- QNVLHSSNNNN YLT | WYQQRPGQ PPKLLIY | W-------- ASTRES | GVPDRFSGSGSG-- TEFTLTISSLQAEDVAVYYC | QQYYS----------- ------------TPPT | FGQGT KVEIK |
| **iPS:435 627** | **21-225_162F6** | **VK4\|B3/J K1** | | DIVMTQSPDSLAV SLGERATINC | KSS-- QNVLHSSNNNN YLT | WYQQRPGQ PPKLLIY | W-------- ASTRES | GVPDRFSGSGSG-- TEFTLTISSLQAEDVAVYYC | QQYYS----------- ------------TPPT | FGQGT KVEIK |
| **iPS:435 701** | **21-225_170F6** | **VK4\|B3/J K1** | | DIVMTQSPDSLAV SLGARATINC | KSS-- QSVLHSSNNYN YLA | WYQQRPGQ PPKVLIH | W-------- ASTRKS | GVPDRFSGSVSG-- TDFTLTINSLQAEDVAVYYC | QQYYS----------- ------------TPWT | FGQGT KVEIK |
| **iPS:435 737** | **21-225_174G5** | **VK4\|B3/J K1** | | DIVMTQSPDSLAV SLGARATINC | KSS-- QSVLHSSNNYN YLT | WYQQKSGQ PPKLLIY | W-------- ASTRES | GVPDRFSGSGSG-- TDFTLTISSLQAEDVAVYYC | QQYYR----------- ------------TPWT | FGQGT KVEIK |
| **iPS:435 751** | **21-225_175D1 0** | **VK4\|B3/J K1** | | DIVMTQSPDSLAV SLGERATINC | KSS-- QSVLYSSNNNN YLA | WYQQKPGQ PPNLLIY | W-------- TSTRES | GVPDRFSGSGSG-- TNFTLTISSLQAEDVAVYYC | QQYYS----------- ------------TPPT | FGQGT KVEIK |
| **iPS:435 773** | **21-225_177B1 2** | **VK4\|B3/J K1** | | DIVMTQSPDSLAV SLGERATVNC | KSS-- QSVLHSSNNNN YLT | WYQQKPGQ PPKLLIY | W-------- ASTRES | GVPDRFSGSGSG-- TDFTLTISSLQAEDVAVYYC | QQYYS----------- ------------SPPT | FGQGT KVEIK |
| **iPS:435 801** | **21-225_181E5** | **VK4\|B3/J K1** | | DIVMTQSPDSLAV SLGERATINC | KSS-- QSVLHSSNNYN YLT | WYQQKPGQ PPKLLIY | W-------- ASTRES | GVPDRFSGSGSG-- TDFTLTISSLQAEDVAVYYC | HQYFI----------- ------------TPWT | FGQGT KVEIK |
| **iPS:435 841** | **21-225_191D8** | **VK4\|B3/J K1** | | DIMMTQSPDSLAV SLGERAIISC | RSS-- QSVLHSSNNYN YLA | WYQQKPGH PPKLLIY | W-------- ASTRES | GVPDRFSGSGSG-- TDFTLTISSLQAEDVAVYYC | QQYYS----------- ------------TPPT | FGLGT KVEIK |
| **iPS:435 925** | **21-225_190D7** | **VK4\|B3/J K1** | | DIVMTQSPDSLAV SLGERATINC | KSS-- QSVLSSSNNYN YLV | WYQQKPGQ PPKLLIY | W-------- ASTRKS | GVPDRFSGSGSG-- TDFTLTISSLQADDVAVYYC | QQYYR----------- ------------TPWT | FGQGT KVEIK |
| **iPS:436 021** | **21-225_193G4** | **VK4\|B3/J K1** | | DIVMTQSPDSLAV SLGERATINC | KSS-- QSVLYSSNNYN YLT | WYQQKPGQ PPKLLIY | W-------- ASTRKS | GVPDRFSGSGSG-- TDFTLTISSLQADDVAVYYC | QQYYI----------- ------------TPWT | FGQGT KVDIK |
| **iPS:436 114** | **21-225_196G8** | **VK4\|B3/J K1** | | DIVMTQSPDSLTV SLGERATVNC | KSS-- QSVLHSSNNNN YLA | WYQQKPGQ PPKLLIY | W-------- ASTRES | GVPDRFSGSGSG-- TDFTLAISSLQAEDVAVYYC | QQYYN----------- ------------TPPT | FGQGT KVEIK |
| **iPS:436 150** | **21-225_197H4** | **VK4\|B3/J K1** | | DIMMTQSSDSLTV SLGERAIISC | RSS-- QSVLHSFNNYN YLA | WYQQKAGH PPNLLIY | W-------- ASTRES | GVPDRFSGSGSG-- TDFTLPISSLQAEDVAVYYC | QQYYS----------- ------------TPPT | FGLGT KVEIK |
| **iPS:436 154** | **21-225_197C6** | **VK4\|B3/J K1** | | DIMMTQSPDSLAV SLGERAIISC | RSS-- QSVLHSSNNYN YLA | WYQQKPGH PPNLLIY | W-------- ASTRES | GVPDRFSGSGSG-- TDFTLTISSLQAEDVAVYYC | QQYYS----------- ------------TPPT | FGLGT KVEIK |
| **iPS:436 218** | **21-225_200G7** | **VK4\|B3/J K1** | | DIVMTQSPDSPTA SLGERATVKC | KSS-- QSVLHSSNNNN YLA | WYQQKPGQ PPKLLIY | W-------- ASTRES | GVPDRFSGSGSG-- TDFTLAISSLQAEDVAVYYC | QQYYN----------- ------------TPPT | FGQGT KVEIK |
| **iPS:436** | **21-** | **VK4\|B3/J** | | DIVMTQSPESLAV | KSS-- | WYQQKPGQ | W-------- | GVPDRFSGSGSG-- | QQYYS----------- | FGQGT |

| | | | | | | | | | | |
|---|---|---|---|---|---|---|---|---|---|---|
| **iPS:436 400** | **21-225_213H7** | **VK4\|B3/J K1** | | DIVMTQSPDSLAV SLGERATINC | KSS-- QNVLDISNNKN SLG | WFQQKPGQ PPKLLIN | W-------- ASTRES | GVPDRFSGSGSG-- TDFTLTISSLQTEDVAVYHC | QQYYN----------- ------------IPPT | FGRGT KVEIK |
| **iPS:436 402** | **21-225_213H12** | **VK4\|B3/J K1** | | DIVMTQSPDSLAV SLGERATITC | KSS-- QNVLKTSNNRN YLA | WYQQKPGQ PPKVLIY | W-------- ASTRES | GVPDRFSGSGSG-- TDFTLIISSLQAEDVAVYYC | HQYYS----------- ------------IPWT | FGQGT KVEIK |
| **iPS:436 500** | **21-225_222H3** | **VK4\|B3/J K1** | | DIVMTQSPDSLAV SLGERATINC | KSS-- QSVLKSSNHRN YLA | WYQQKPGQ PPQLLIY | W-------- ASTRET | GVPDRFSGSGSG-- TDFTLTISSLQAEDVSVYSC | QQYSS----------- ------------IPWT | FGQGT KVEIN |
| **iPS:436 520** | **21-225_223G10** | **VK4\|B3/J K1** | | DIVMTQSPDSLAV SLGERATINC | KSS-- QSILLSSNNKN YLA | WHQQKPGQ PPKLLIY | W-------- ASTRES | GVPDRFSGSGSG-- TDFTLTISSLQAEDVAVYYC | LQYFS----------- ------------TPWT | FGQGT KVEIK |
| **iPS:436 544** | **21-225_224H5** | **VK4\|B3/J K1** | | DIVMTQSPDSLAV SLGERATINC | KSS-- QSVLYSSNNFN YLT | WYQQKPGQ PPKLLIY | W-------- ASTRES | GVPDRFSGSGSG-- TDFTLTINSLQAEDVAVYYC | QQYYS----------- ------------TPPT | FGQGT KVEIK |
| **iPS:436 550** | **21-225_224D8** | **VK4\|B3/J K1** | | DIVMTQSPDSLAV SLGERAAINC | KSS-- QSVLYSSNNKN YLA | WYQQKPGQ PPKLLIY | W-------- SSTRKS | GVPDRFSGSGSG-- TDFTLTISSLQAEDVAVYYC | QQYFS----------- ------------TPPT | FGQGT KVEIK |
| **iPS:436 574** | **21-225_225F5** | **VK4\|B3/J K1** | | DIVMTQSPDSLAV SLGERATINC | KSS-- QNVLYNSNNNN YLA | WYQQKPGQ PPNLLIY | W-------- ASTRES | GVPDRFSGSGSG-- TDFTLTISSLQAEDVAVYFC | QQYYS----------- ------------SPPT | FGQGT KVEIN |
| **iPS:436 586** | **21-225_225F11** | **VK4\|B3/J K1** | | DIVMTQSPDSLAV SLGERATINC | KSS-- QSVLYSSNNYN YLA | WYQQRPGQ PPKLLIY | W-------- ASTRES | GVPDRFSGSGSG-- PDFTLTISSLQAEDVAVYYC | QQYYT----------- ------------TPPT | FGQGT KVEIK |
| **iPS:436 600** | **21-225_226F6** | **VK4\|B3/J K1** | | DIVMTQSPDSLAV SLGERATINC | KSS-- QSILYSSNNYN YLA | WYQQKPGQ PPKLLIY | W-------- ASTRES | GVPDRFSGSGSG-- PDFTLTISSLQAEDVAVYYC | QQYYT----------- ------------TPPT | FGQGT KVEIK |
| **iPS:436 616** | **21-225_226D11** | **VK4\|B3/J K1** | | DIVMTQSPDSLAV SLGERATINC | KSS-- QNVLHSSNSNN YLV | WYQQKPGQ PPKLLIY | W-------- ASTRES | GVPDRFRGSGSG-- TDFTLTISSLQAEDVAVYYC | QQYYK----------- ------------TPWT | FGQGT KVEIK |
| **iPS:436 622** | **21-225_226A12** | **VK4\|B3/J K1** | | DIVMTQSPDSLAV SLGERATINC | KSS-- QNVLYSSNNNN YLA | WYQQTPGQ PPKLLFY | W-------- ASTRKS | GVPDRFSGSGSG-- TDFTLTISSLQAEDVAVYYC | QQYYS----------- ------------SPPT | FGQGT KVEIK |
| **iPS:436 638** | **21-225_227C7** | **VK4\|B3/J K1** | | DIVMTQSPDSLAV SLGERATINC | RSS-- QIVLSDSNNNN YLA | WYQQKPGQ PPNLLIY | W-------- ASTRES | GVPDRFSGSGSG-- TDFTLTISSLQAEDVAVYFC | QQYYS----------- ------------SPPT | FGQGT KVEIK |
| **iPS:437 356** | **21-225_74B1** | **VK4\|B3/J K1** | | DIVMTQSPDFLAV SLGERATTNC | KSS-- QSVLHRSNNYN YLA | WYQQKPGQ PPKLLIY | W-------- ASTRES | GVPDRFSGSGSG-- TDFTLTISSLQAEDVAVYYC | QQYYS----------- ------------TPPT | FGQGT KVEIK |
| **iPS:437 361** | **21-225_74C1** | **VK4\|B3/J K1** | | DIVMTQCPDSPAV SLGERASINC | KSS-- QSILHSSNNYN YLA | WYQQKPGH PHKLLIY | W-------- ASTRES | GVPDRFSGSGYG-- TDFTLTISSLQAEDVAVYYC | QQYYS----------- ------------TPWT | FGQGT KVEIK |
| **iPS:437 379** | **21-225_74H2** | **VK4\|B3/J K1** | | DIVMTQSPDSLAV SLGERATINC | KSS-- QSVLHSSNNKN YLT | WYQQKPGQ PHKLLIY | W-------- ASTRES | GVPDRFSGSGSG-- TDFSLTIGSLQAEDVAVYYC | QQYYS----------- ------------IPPT | FGQGT KVEIK |
| **iPS:446** | **21-** | **VK4\|B3/J** | | DIVMTQSPDSLAV | KSS-- | WYQQKPGQ | W-------- | GVHDRFSGSGSG-- | HQYLS----------- | FGQGT |

| | | | | | | | | | | |
|---|---|---|---|---|---|---|---|---|---|---|
| **iPS:451 116** | **21-225_164A4** | **VK4\|B3/JK1** | | DIVMTQYPDSRAV SLGERATIKC | KSS--QSVLYSSNNKN YLT | WYQQKPGQ PPKLFIY | W-------- ASTRES | GVPDRFSGSGCG-- TDFTLTISSVQAEDVAVYYC | QQYFS----------- ------------TPWT | FGQGT KVEIK |
| **iPS:451 124** | **21-225_74F6** | **VK4\|B3/JK1** | | DIVMTQSPDSLAV SLGERATINC | KSS--QNILSSSNNKN YLT | WYQQKPGQ PPKILIY | W-------- TSTRES | GVPDRFSGSGFG-- TDFTLTISSLQAEDVAVYYC | QQYFS----------- ------------VPLT | FGQGT KVEIK |
| **iPS:451 127** | **21-225_164A7** | **VK4\|B3/JK1** | | DIVMTQCPDSLAV SLGERATINC | KSS--QSVLHSSNNNN YLA | WYQQKPGQ PHKLLIY | W-------- TSTRES | GVPDRFSGSGYG-- TDFSLTIASLQAEDVAVYYC | QQYYS----------- ------------IPLT | FGQGT KVEIK |
| **iPS:451 129** | **21-225_94D2** | **VK4\|B3/JK1** | | DIVMTQSPDSLAV SLGERATINC | KSS--QSVLHSSNNKN YLT | WYQQKPGQ PHKLLIY | W-------- ASTRES | GVPDRFSGSGSG-- TDFSLTIGSLQHEDVAVYYC | QQYHS----------- ------------IPPT | FGHGT KVEIK |
| **iPS:451 133** | **21-225_95H4** | **VK4\|B3/JK1** | | DIVMTQCPDSLAV SLGERATINC | KSS--QSVLFSSNNYN YLA | WYQQRPGQ PHNLLIY | W-------- ASTRES | GVPDRFSGSGSG-- TDFTLTISSLQAADVAVYYC | QQYHS----------- ------------SPLT | FGQGT TVQIK |
| **iPS:392 786** | **21-225_24E1** | **VK4\|B3/JK1** | | DIVMTQSPDSLAV SLGERATINC | KSS--QSVLYTSNNNN YLT | WYQQKPGQ RPNLLIY | W-------- ASTRES | GVPDRFSGSGSG-- TDFTLTISSLQAEDVAVYYC | QQFYS----------- ------------TPPT | FGQGT KVEIK |
| **iPS:392 886** | **21-225_23A12** | **VK4\|B3/JK1** | | DIVMTQSPDSLAV SLGERATINC | KSS--QSVLYSSNNNN YLA | WYQQKPGQ PPKLLIY | W-------- TSTRES | GVPDRFSGSGSG-- TDFTLTISSLQAEDVAVYYC | QQYYD----------- ------------TPPT | FGQGT KVEIK |
| **iPS:392 928** | **21-225_25A4** | **VK4\|B3/JK1** | | DIVMTQFPDSLAV SLGERATIKC | KSS--QSVLYSSHNNN YLA | WYQQKPGQ PPKLLLY | W-------- ASTRES | GVPDRFSGSGSG-- TEFTLTISSLEAEDVAVYYC | QQYYS----------- ------------TPPT | FGQGT KVEIK |
| **iPS:392 960** | **21-225_29E6** | **VK4\|B3/JK1** | | DIVMTQFPDSLAV SLGERATINC | KSS--QSVLYSSHNNY YLT | WYQQKPGQ PHKLLLY | W-------- ASSRES | GVPDRFSGSGSG-- TEFTLTISSLQAEDVAVYYC | QQYYS----------- ------------TPPT | FGQGT KVEIK |
| **iPS:392 992** | **21-225_26C4** | **VK4\|B3/JK1** | | DIVMTQSPDSLAV SLGERATINC | KSS--QSVLYRSNNYN YLA | WYQHKPGQ PPKLLIY | W-------- ASTRES | GVPDRFSGSGSG-- TDFTLTISSLQAEDVAVYYC | QQYYS----------- ------------TPPT | FGQGT KVEFK |
| **iPS:393 368** | **21-225_29H8** | **VK4\|B3/JK1** | | DIVMTQSPDSLAV SLGERATINC | RSS--QTILHSSNNYN YLA | WYQQKPGQ PPKLLIY | W-------- ASTRES | GVPDRFSGSGSG-- TDFTLTISSLQAEDVAVYYC | QQYYC----------- ------------TPPT | FGQGT KVEIK |
| **iPS:393 942** | **21-225_11E5** | **VK4\|B3/JK1** | | DIVMTQSPDSLAV SLGERATLNC | KSS--QSVLYSSNNNN YLT | WYQQKPGQ RPKLLIY | W-------- ASTRES | GVPDRFSGSGSG-- TNFTLTISSLQAEDVAVYYC | QQYYS----------- ------------TPPT | FGQGT KVEIK |
| **iPS:398 506** | **21-225_23G12** | **VK4\|B3/JK1** | | DIVMTQSPDSLAV SLGERATINC | KSS--QSILFSSNNNN YLA | WYQQKPGQ PPKLLIY | W-------- ASTRES | GVPDRFSGSGSG-- TDFTLTISSLQAEDVAVYFC | QQYSS----------- ------------TPWT | FGQGT KVEIK |
| | | **Germline** | | **K_FR1** | **K_CDR1** | **K_FR2** | **K_CDR2** | **K_FR3** | **K_CDR3** | **K_FR4** |
| | **VK1\|A30/JK2** | | 32131 | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPYT | FGQGT KLEIK |
| **iPS:426 114** | **21-225_28H2** | **VK1\|A30/JK2** | | DIQMTQSPSSLSA SIGDRVTITC | RAS--QGIR-- ----NDLG | WYQQKPGK APKRLIY | A-------- ASSLQS | GVPSRFSGSGSG-- TEFTLTISSLQPEDFATYYC | LQHYS----------- ------------YPRS | FGQGT KLEIK |
| **iPS:426 116** | **21-225_29E2** | **VK1\|A30/JK2** | | DIQMTQSPSSLSA SVGDRVTITC | RAS--QAIR-- ----NDLG | WYQQRPGK APKRLIY | A-------- ASSLQS | GVPSRFSGSGSG-- TEFILTISSLQPEDFATYYC | LQHYN----------- ------------YPRS | FGQGT KLEIK |

| | | | | | | | | | | |
|---|---|---|---|---|---|---|---|---|---|---|
| iPS:435 401 | 21-225_150E2 | VK1\|A30/JK2 | | DIQMTQSPSSLSA<br>SVGDRVTISC | RAS--QGIG--<br>----NDLG | WYQQKPGK<br>APTRLIY | A--------<br>ASSLQS | GVPSRFSGSGSG--<br>TEFTLTISSLQPADFATYYC | LQHYS-----------<br>------------FPYS | FGQGT<br>KLEIK |
| iPS:435 445 | 21-225_152F7 | VK1\|A30/JK2 | | DIQMTQSPSSLSA<br>SVGDRVTITC | RAS--QGIR--<br>----NDLG | WYQQKPGK<br>APKRLIY | A--------<br>TSSLQS | GVPSRFSGSGSG--<br>TEFTLTISSLQPEDFATYYC | LQHYN-----------<br>------------FPYS | FGQGT<br>KLEIK |
| iPS:435 763 | 21-225_176H12 | VK1\|A30/JK2 | | DIQMTQSPSSLSA<br>SVGDRVTITC | RAS--QGIR--<br>----NDLG | WYQQKPGK<br>APNRLIY | A--------<br>ASSLQS | GVPSRFSGSGSG--<br>TEFTLTISSLQREDFATYYG | LQHNS-----------<br>------------YPRS | FGQGT<br>KLEIK |
| iPS:435 767 | 21-225_177B4 | VK1\|A30/JK2 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHYS-----------<br>------------FPRS | FGQGT<br>KLEIK |
| iPS:392 974 | 21-225_26A11 | VK1\|A30/JK2 | | DIQMTQSPISLSA<br>SVGDRVTITC | RAS--QAIR--<br>----NDLG | WYQQRPGK<br>APKRLIY | A--------<br>ASSLQS | GVPSRFSGSGSG--<br>TEFILTISSLQPEDFATYYC | LQHYN-----------<br>------------YPRS | FGQGT<br>KLEIR |
| iPS:393 046 | 21-225_25A12 | VK1\|A30/JK2 | | DIQMTQSPSSLSA<br>SVGDRVTVTC | RAS--QAIR--<br>----DDLG | WYQQRPGK<br>APKRLIY | A--------<br>ASSLQS | GVPSRFSGSGSG--<br>TEFILTISSLQPEDFATYYC | LQHYN-----------<br>------------YPRS | FGQGT<br>KLEIK |
| | | Germline | | K_FR1 | K_CDR1 | K_FR2 | K_CDR2 | K_FR3 | K_CDR3 | K_FR4 |
| | VK1\|O12/JK4 | | 32132 | DIQMTQSPSSLSA<br>SVGDRVTITC | RAS--QSIS--<br>----SYLN | WYQQKPGK<br>APKLLIY | A--------<br>ASSLQS | GVPSRFSGSGSG--<br>TDFTLTISSLQPEDFATYYC | QQSYS-----------<br>------------TPLT | FGGGT<br>KVEIK |
| iPS:426 118 | 21-225_7A10 | VK1\|O12/JK4 | | DIQMTQSPSSLSA<br>SVGDRVTITC | RAS--QNIY--<br>----SYLN | WYQQKPGR<br>APKLVIY | S--------<br>TSSLQS | GVPSRFSGSGSG--<br>TDFSLTISNLQPEDFSTYYC | QQSYS-----------<br>------------PPLT | FGGGT<br>KVEIR |
| iPS:426 124 | 21-225_32D6 | VK1\|O12/JK4 | | DIQMTQSPSSLST<br>SVGDRVTITC | RAS--QNII--<br>----SYLN | WYQQKPGK<br>APKLLMY | V--------<br>ASRLQS | GVPSRFSGSGSG--<br>TDFTLTISSLQAEDFATYYC | QQSYS-----------<br>------------TPYT | FGGGT<br>KVAIK |
| iPS:451 135 | 21-225_64A11 | VK1\|O12/JK4 | | DIQMTQSPFSLSA<br>SVGDRVTITC | RAS--RSVS--<br>----RYLN | WYQQTLGK<br>ALKLLIS | V--------<br>ASRLQS | GVPSRFSGSGSG--<br>TDFTLTISSVQREDFATYFC | QQSDS-----------<br>------------FPLT | FGGGT<br>KVEIK |
| iPS:434 011 | 21-225_48B10 | VK1\|O12/JK4 | | DIQMTQSPSSLSA<br>SVGDRVTITC | RAS--QSIR--<br>----KYLN | WYQKTPGK<br>APKLLIY | A--------<br>ASSLQS | GVPSRFSGSGSG--<br>TDFTLTISSVQPEDFATYYC | QQTYS-----------<br>------------NPLT | FGGGT<br>KVEFT |
| iPS:434 015 | 21-225_48F12 | VK1\|O12/JK4 | | DIQMTQSPSSLSA<br>SVGDRVTITC | RAS--QSIR--<br>----KYLN | WYQKTPGK<br>APKLLIY | A--------<br>ASSLQS | GVPSRFSGSGSG--<br>TDFTLTISSLQPEDFANYYC | QQTYS-----------<br>------------NPLT | FGGGT<br>EVEIT |
| iPS:434 017 | 21-225_48G12 | VK1\|O12/JK4 | | DIQMTQSPSSLSA<br>SVGDRVTITC | RAS--QSIR--<br>----KYLN | WYQKTPGK<br>APKLLIY | A--------<br>ASSLQS | GVPSRFSGSGSG--<br>TDFTLTISSVQPEDFANYYC | QQTYS-----------<br>------------NPLT | FGGGT<br>EVEIT |
| iPS:434 165 | 21-225_50F2 | VK1\|O12/JK4 | | DIQMTQSPSSLSA<br>SVGDRITITC | RAS--QSIL--<br>----SYLN | WYQQKPGK<br>APKLLIY | V--------<br>ASSFQS | GVPSRFSGSGSG--<br>TDFTLTISSLQPDDFATYYC | QQSYS-----------<br>------------PPLT | FGGGT<br>KVEIK |
| iPS:434 191 | 21-225_56B6 | VK1\|O12/JK4 | | DIQMTQSPSSLSV<br>SVGDRVTITC | RAS--QSIF--<br>----RYLN | WYQQKPGR<br>APKLLIF | A--------<br>ASSFQS | GVPSRFSGSGSG--<br>TDFTLTISSLQPEDFATYYC | QQTYS-----------<br>------------PPLT | FGGGT<br>KVEIK |
| iPS:434 247 | 21-225_62D2 | VK1\|O12/JK4 | | DIQMTQSPSSLSA<br>SVGDRVTIIC | RAS--QSII--<br>----SYLN | WYQQKPGK<br>APKLLIY | A--------<br>TSSLQS | GVPSRFSGSGSG--<br>TDFTLTISSLQPEDFATYYC | QQTYS-----------<br>------------PPLT | FGGGT<br>KVEIK |
| iPS:434 335 | 21-225_63C10 | VK1\|O12/JK4 | | DIQMTQSPSSLST<br>SVGDRVTITC | RAS--QSIF--<br>----SYLH | WYQQKPGK<br>APKLLIS | A--------<br>ASSLQS | GVPSRFSGSGSG--<br>TDFTLTISSLQPEDFATFYC | QQTYS-----------<br>------------PPLT | FGGGT<br>KVEIK |
| iPS:434 341 | 21-225_64F7 | VK1\|O12/JK4 | | DIQMTQSPSSLSA<br>SVGDRVTITC | RAS--QNIK--<br>----KYLN | WYQQKPGK<br>APKFLIY | G--------<br>ASSLQS | GVPSRFSGSGSG--<br>TDFTLTISSLQPEDFAAYYC | QQSYN-----------<br>------------ISFT | FGGGT<br>KVELK |
| iPS:435 295 | 21-225_146H1 | VK1\|O12/JK4 | | DIQMTQSPSSLSA<br>SVGDRVTITC | RAS--QSIS--<br>----DYLN | WYQQKPGK<br>APKVLIY | T--------<br>TSSLQS | GVPSRFSGSGSG--<br>TDFTLTISSLQPEDFATYYC | QQSY------------<br>------------STPT | FGGGT<br>KVEIK |
| iPS:435 307 | 21-225_146E9 | VK1\|O12/JK4 | | DIQMTQSPSSLSA<br>SVGDRVTITC | RAS--QSIS--<br>----DYLN | WYQLKPGK<br>APKVLIY | T--------<br>TSSLQS | GVPSRFSGSGSG--<br>TDFTLTISSLQPEDFATYFC | QQSY------------<br>------------STPT | FGGGT<br>KVEIK |
| iPS:435 347 | 21-225_148C4 | VK1\|O12/JK4 | | DIQMTQSPSSLSA<br>SVGDRVTITC | RAS--QSII--<br>----NYLN | WYQQKPGK<br>APKVLIY | T--------<br>ASSLQS | GVPSRFSGSGSG--<br>TDFTLTISSLQPEDFTTYYC | QQSY------------<br>------------STPT | FGGGT<br>KVEIE |
| iPS:435 355 | 21-225_148H9 | VK1\|O12/JK4 | | DIQMTQSPSSLSA<br>SVGDRVTITC | RAS--QSIS--<br>----NYLN | WYQQKPGK<br>APKVLIY | I--------<br>ASSLQS | GVPSRFSGSGSG--<br>TDFTLTISSLQPEDFATYYC | QQSY------------<br>------------STPT | FGGGT<br>KVEIK |

| | | | | | | | | | | |
|---|---|---|---|---|---|---|---|---|---|---|
| **iPS:435 371** | **21-225_149A3** | **VK1\|O12/ JK4** | | DIQMTQSPSSLSA<br>SVGDRVTITC | RAS--QSIS--<br>----SYLN | WYQQKPGK<br>APKVMIY | T--------<br>ASSLQS | GVPSRFSGSGSG--<br>TDFTLTINSLQPEDFATYYC | QQSY------------<br>------------SIPT | FGGGT<br>KVEIK |
| **iPS:435 415** | **21-225_150C1 1** | **VK1\|O12/ JK4** | | DIQMTQSPSSLSA<br>SVGDRVTITC | RAS--QSIS--<br>----SYLN | WYQQKPGK<br>APKLLIY | T--------<br>ASSLQS | GVPSRFSGSGSG--<br>TDFTLTISSLQPEDFATYYC | QQSY------------<br>------------SIYT | FGGGS<br>KVEIK |
| **iPS:435 419** | **21-225_150C1 2** | **VK1\|O12/ JK4** | | DIQMTQSPSSLSA<br>SVGDRVTITC | RAS--QSIS--<br>----DYLN | WYQQKPGK<br>APKVLIY | T--------<br>TSSLQS | GVPSRFSGSGSG--<br>TDFTLTISSLQPEDFATYYC | QQSF------------<br>------------STPT | FGGGT<br>KVEIK |
| **iPS:435 425** | **21-225_151B1 2** | **VK1\|O12/ JK4** | | DIQMTQSPSSLSA<br>SVGDRVTITC | RAS--QSIS--<br>----NFLN | WYQQKPGK<br>APKVLIY | T--------<br>ASSLES | GVPSRFSGSESG--<br>TDFTLTISSLQPEDFATYYC | QQSY------------<br>------------STPT | FGGGT<br>RVEIK |
| **iPS:435 431** | **21-225_152D2** | **VK1\|O12/ JK4** | | DIQMTLSPSSLSA<br>SVGDRVTITC | RAS--QSIS--<br>----DYLN | WYQLKPGK<br>APKVLIY | T--------<br>TSSLQS | GVPSRFSGSGSG--<br>TDFTLTISSLQPEDFATYFC | QQSY------------<br>------------STPT | FGGGT<br>KVEIK |
| **iPS:435 439** | **21-225_152G4** | **VK1\|O12/ JK4** | | DIQMTQSPSSLSA<br>SVGDRVTITC | RAS--QSIS--<br>----DYLN | WYQQKPGK<br>APKVLIY | T--------<br>TSSLQS | GVPSRFSGSGSG--<br>TDFTLTISSLQPEDFATYFC | QQSY------------<br>------------STPT | FGGGT<br>KVEIK |
| **iPS:435 455** | **21-225_152B1 1** | **VK1\|O12/ JK4** | | DIQMTQSPSSLSA<br>SVGDRVTITC | RAS--QSIS--<br>----DYLN | WYQQKPGK<br>APKVLIY | T--------<br>TSSLQS | GVPSRFSGSGSG--<br>TDFTLTISSLQPEDFATYFC | QQSY------------<br>------------STPT | FGGGT<br>KVEIK |
| **iPS:435 487** | **21-225_155C4** | **VK1\|O12/ JK4** | | DIQMTQSPSSLSA<br>SVGDRVTITC | RAS--QSIS--<br>----SYLN | WYQQKPGK<br>APKVLIF | T--------<br>ASSLQS | GVPSRFSGSGSG--<br>TDFTLTISSLQPEDFATYYC | QQSY------------<br>------------STPT | FGGGT<br>RVEIK |
| **iPS:435 503** | **21-225_156E4** | **VK1\|O12/ JK4** | | DIQMTQSPSSLSA<br>SVGDRVTITC | RAS--QSIS--<br>----SYLN | WYQQKPGK<br>APKVLIY | T--------<br>ASSLQS | GVPSRFSGSGSG--<br>TDFTLTISSLQPEDFATYYC | QQSY------------<br>------------SAPT | FGGGT<br>KVEIK |
| **iPS:435 563** | **21-225_159H2** | **VK1\|O12/ JK4** | | DIQLTQSPSSLSA<br>SVGDRVTITC | RAS--QSIS--<br>----KYLN | WYQQKPGK<br>APELLIY | A--------<br>TSNLQS | GVPSRFSGSGSG--<br>TDFTLTISTLQPEDFVTYYC | QQSYS-----------<br>------------LPVT | FGGGT<br>KVEIK |
| **iPS:436 110** | **21-225_196F4** | **VK1\|O12/ JK4** | | DFQMIQSPSSLSA<br>SVGDRVTITC | RAS--QRIH--<br>----SYLN | WYQQKPGK<br>APKLLIY | T--------<br>ASSLQG | GVPSRFSGSGSG--<br>TDFTLTISSLQPEDFATYYC | QQSYG-----------<br>------------SPLT | FGGGT<br>KVEIK |
| **iPS:436 244** | **21-225_201H1 0** | **VK1\|O12/ JK4** | | DIQMTQSPSSLSA<br>SVGDRVTITC | RAS--HNIN--<br>----SYLN | WYQQKSGK<br>APKLLIY | A--------<br>ASSLQS | GVPSRFSGSGSG--<br>TDFTLTFSSLQPEDFATYYC | QQSYS-----------<br>------------FPLT | FGGGT<br>KVEMR |
| **iPS:436 262** | **21-225_203E3** | **VK1\|O12/ JK4** | | DIQMTQSPSSPSA<br>SVGDRVTITR | RAS--HNIN--<br>----SYLN | WYQQKSGK<br>APKLLIY | A--------<br>ASSLQS | GVPSRFSGSGSG--<br>TDFTLTFSSLQPEDFATYYC | QQSYS-----------<br>------------FPLT | FGGGT<br>KVEMR |
| **iPS:436 276** | **21-225_204H4** | **VK1\|O12/ JK4** | | DIQMTLSPSSPSA<br>FVGDRVTITR | RAS--HNIN--<br>----SYLN | WYQQKSGK<br>APKLLIY | A--------<br>ASSLQS | GVPSRFSGSGSG--<br>TDFTLTFSSLQPEDFATYYC | QQSYS-----------<br>------------FPLT | FGGGT<br>KVEMR |
| **iPS:436 280** | **21-225_204D6** | **VK1\|O12/ JK4** | | DSQMTQSPSSLSA<br>SVGDRVTITC | RAS--RSVH--<br>----TYLN | WYQQKPGK<br>APKVLIY | G--------<br>ASSLQR | GVPSRFSGSGSG--<br>TDFTLTISSLQPEDVATYYC | QQSYS-----------<br>------------SPLT | FGGGT<br>KVEIQ |
| **iPS:436 312** | **21-225_206A4** | **VK1\|O12/ JK4** | | DIQMTLSPSSPSA<br>FVGDRVTITR | RAS--HNIN--<br>----SYLN | WYQQKSGK<br>APKLLIC | A--------<br>ASSLQS | GVPSRFSGSGSG--<br>TDFTLTFSSLQPEDFATYYC | QQSYS-----------<br>------------FPLT | FGGGT<br>KVEMR |
| **iPS:436 316** | **21-225_206A5** | **VK1\|O12/ JK4** | | DIQMTQSPSSPSA<br>SVGDRVTITR | RAS--HNIN--<br>----SYLN | WYQQKSGK<br>APKLLIC | A--------<br>ASSLQS | GVPSRFSGSGSG--<br>TDFTLTFSSLQPEDFATYYC | QQSYS-----------<br>------------FPLT | FGGGT<br>KVEMR |
| **iPS:436 338** | **21-225_208E8** | **VK1\|O12/ JK4** | | DIQMTQSPSSPSA<br>FVGDRVTITR | RAS--HNIN--<br>----SYLN | WYQQKSGK<br>APKLLIY | A--------<br>ASSLQS | GVPSRFSGSGSG--<br>TDFTLTFSSLQPEDFATYYC | QQSYS-----------<br>------------FPLT | FGGGT<br>KVEMR |
| **iPS:436 344** | **21-225_208B1 1** | **VK1\|O12/ JK4** | | DIQMTQSPSSPSA<br>SVGDRVTITC | RAS--HNIN--<br>----SYLN | WYQQKSGK<br>APKLLIY | A--------<br>ASSLQS | GVPSRFSGSGSG--<br>TDFTLTFSSLQPEDFATYYC | QQSYS-----------<br>------------FPLT | FGGGT<br>KVEMR |
| **iPS:436 358** | **21-225_210D1** | **VK1\|O12/ JK4** | | DIQMTQSPSSLSA<br>SVGDRVTITC | RAS--HNIN--<br>----SYLN | WYQQKSGK<br>APKLLIY | A--------<br>ASSLQS | GVPSRFSGSGSG--<br>TDFTLTFSSLQPEDFATYYC | QQSYS-----------<br>------------FPLT | FGGGT<br>KVEMR |

| | | | | | | | | | | |
|---|---|---|---|---|---|---|---|---|---|---|
| iPS:437<br>282 | 21-<br>225_207C9 | VK1\|O12/<br>JK4 | | DNQMTQSPSSLSA<br>SVGDRVTITC | RAS--QRFS--<br>----NYLN | WYQQKPGK<br>APKLLIY | T--------<br>ASSLQS | GVPSRFSASVSG--<br>TDFTLTISSLQPEDFATYYC | QQSYS-----------<br>------------IPLT | FGGGT<br>KVEIK |
| iPS:392<br>636 | 21-<br>225_17A6 | VK1\|O12/<br>JK4 | | DIQMTQSPSSLSA<br>SVGDRVTITC | RAS--QTIS--<br>----NYLN | WYHQKPGK<br>APKLLIY | A--------<br>ASSLQS | GVPSRFSGSGSG--<br>TDFTLTISSLQPEDFATYYC | QQSHT-----------<br>------------SPLT | FGGGT<br>KVEIK |
| iPS:392<br>648 | 21-<br>225_16D11 | VK1\|O12/<br>JK4 | | DIQMTQSPSSLSA<br>SVGDRVTITC | RAS--QTIS--<br>----NYLN | WYHQKPGK<br>APKLLIY | A--------<br>ASSLQS | GVPSRFSGSGSG--<br>TDFTLTISSLQPEDFATYYC | QQSHS-----------<br>------------SPLT | FGGGT<br>KVEIK |
| iPS:392<br>664 | 21-<br>225_20F6 | VK1\|O12/<br>JK4 | | DIQMTQSPSSLSA<br>SVGDRVTITC | RAS--QSII--<br>----TYLN | WYQQKPGK<br>APKVLIH | T--------<br>ASSLQS | GVPSRFSGSGSG--<br>TDFTLTISSLQPEDFATYYC | QQTYS-----------<br>------------PPLT | FGGGT<br>KVEIK |
| iPS:392<br>738 | 21-<br>225_18G4 | VK1\|O12/<br>JK4 | | DIHMTQSPSSLSA<br>SVGDRVTITC | RAS--QSII--<br>----SYLN | WYQQKPGK<br>APKVLIY | T--------<br>ASSLQT | GVPSGFSGSGSG--<br>TDFTLTISSLQPEDFATYYC | QQTYS-----------<br>------------PPLT | FGGGT<br>KVEIK |
| iPS:392<br>798 | 21-<br>225_22C7 | VK1\|O12/<br>JK4 | | DIQMTQSPSSLSA<br>SVGDRVTITC | RAS--QNII--<br>----SYLN | WYQQKPEK<br>APKLLIH | I--------<br>ASSLQS | GVPSRFSGSGSG--<br>TDFTLTISSLQPEDFATYYC | QQTYS-----------<br>------------TPLT | FGGGT<br>KVEIK |
| iPS:392<br>922 | 21-<br>225_30G4 | VK1\|O12/<br>JK4 | | DIQMTQSPPSLST<br>SVGDRVTITC | RAT--QNIF--<br>----SYLN | WHQQKPGK<br>APKLLIH | T--------<br>ASSLQG | GVPSRFSGSGSG--<br>TDFTLTIISMQPEDFSTYYC | QLSYS-----------<br>------------PPYT | FGGGT<br>KVEIK |
| iPS:393<br>002 | 21-<br>225_30G1 | VK1\|O12/<br>JK4 | | DIQMTQSPSSLSA<br>SVGDRVTITC | RAS--QNIY--<br>----SYLN | WYQQKPGK<br>DPKLLIY | A--------<br>ASSLHS | GVPSRFSGSGSG--<br>TDFTLTISSLQPEDFATYYC | QQSYS-----------<br>------------TPLT | FGGGT<br>KVEIK |
| iPS:393<br>042 | 21-<br>225_31F1 | VK1\|O12/<br>JK4 | | DIQMTQSPSSLSA<br>SVGDRVTITC | RAS--QRIS--<br>----SYLN | WYQQKPGK<br>APKLLIF | A--------<br>ASSSQS | GVPSRFSGSGSG--<br>TDFTLTISSLQPEDFATYYC | QQSYI-----------<br>------------TPLT | FGGGT<br>TVEIR |
| iPS:393<br>066 | 21-<br>225_34D3 | VK1\|O12/<br>JK4 | | DIQMTQSPSSLSA<br>SVGDRVTITC | RAS--QNIY--<br>----SYLN | WYQQKPGK<br>DPKLLIY | A--------<br>ASSLHS | GVPSRFSGSGSG--<br>TDFTLTISSLQPEDFATFYC | QQSYS-----------<br>------------TPLT | FGGGT<br>KVEIK |
| iPS:393<br>082 | 21-<br>225_34C11 | VK1\|O12/<br>JK4 | | DIQMTQFPSSLST<br>SVGDRVTSTC | RAS--QNIR--<br>----NFLN | WYQQKPEK<br>DPKLQIY | G--------<br>ASTLQS | GVPSRFSGSGFG--<br>TDFTLTISSLQPKDFATYYC | QQTCS-----------<br>------------TPLT | FGGGT<br>KVEIK |
| iPS:393<br>092 | 21-<br>225_33C12 | VK1\|O12/<br>JK4 | | DIQMTQSPSSLST<br>SVGDRVTITC | RAS--QSII--<br>----SYLN | WYQQKPGK<br>APKLLIY | V--------<br>ASSLQG | GVPSRFNGSGSG--<br>TDFTLTISSLQPEDFATYYC | QQSYS-----------<br>------------TPYT | FGGGT<br>KVEIK |
| iPS:393<br>100 | 21-<br>225_36B8 | VK1\|O12/<br>JK4 | | DIQMTQSPSSLST<br>SVGDRVTITC | RAS--QSII--<br>----SYLN | WYQQKPGK<br>APKLLIY | V--------<br>ASSLQS | GVPSRFSGSGSG--<br>TDFTLTISSLQPEDFATYYC | QQSYS-----------<br>------------TPYT | FGGGT<br>KMEIK |
| iPS:393<br>108 | 21-<br>225_34G11 | VK1\|O12/<br>JK4 | | DIQMTQSPSSLSA<br>SVGDRVTITC | RAS--QNIN--<br>----RYLN | WYQQKPGK<br>APKLLIY | G--------<br>ASSLQS | GVPSRFSGSGSG--<br>TDFTLTISTLQPEDFATYYC | QQTYI-----------<br>------------TPLT | FGGGT<br>KVEIK |
| iPS:393<br>122 | 21-<br>225_33B2 | VK1\|O12/<br>JK4 | | DIQMTQSPSSLST<br>SVGDKVTITC | RAS--QSII--<br>----SYLN | WYQQKPGK<br>APKLLIY | V--------<br>ASSLQS | GVPSRFSGSGSG--<br>TDFTLTISSLQPEDFATYYC | QQSYS-----------<br>------------TPYT | FGGGT<br>KVEIK |
| iPS:393<br>134 | 21-<br>225_34C2 | VK1\|O12/<br>JK4 | | DIQMTQSPSSLST<br>FVGDRVTITC | RAS--QRII--<br>----SYLN | WFQQKPGK<br>APKLLIY | V--------<br>ASSLQS | GVPSRFSGSGSG--<br>TYFTLTISSLQPEDFATYYC | QQSYS-----------<br>------------TPYT | FGGGT<br>KMEIK |
| iPS:393<br>136 | 21-<br>225_34D8 | VK1\|O12/<br>JK4 | | DIQMTQSPSSLST<br>SVGDRVTITC | RAS--QIII--<br>----SYLN | WYQQKPGK<br>APKLLIF | V--------<br>ASSLQS | GVPSRFSGSGSG--<br>TDFTLTISSLQPEDFATYYC | QQSYS-----------<br>------------TPYT | FGGGT<br>KVEIK |
| iPS:393<br>840 | 21-225_3F8 | VK1\|O12/<br>JK4 | | DFQMTQSPSSLSA<br>SVGDRVTITC | RAS--QSIL--<br>----SYLN | WYRQKPGR<br>APQVLIH | T--------<br>TSSLQS | GVPSRFSGSGSG--<br>TVFTLTISSLQPEDFATYYC | QQTYS-----------<br>------------TPLT | FGGGT<br>RVEIN |
| iPS:393<br>844 | 21-225_3G7 | VK1\|O12/<br>JK4 | | DIQMTQSPSSLSA<br>SVGDRVTVTC | RAS--QNIY--<br>----RYLN | WYQGRPGR<br>APKLMIY | A--------<br>ASSSQS | GVPSRFSGSGSG--<br>TDFTVTISSLQPEDFATYYC | QQSYS-----------<br>------------PPFT | FGGGA<br>KVEID |
| iPS:393<br>852 | 21-<br>225_12A10 | VK1\|O12/<br>JK4 | | DVQMTQSPSSLSA<br>SAGDRVTITC | RAS--QNIY--<br>----SYLN | WYQQKPGR<br>APKVLIH | T--------<br>ASSLQS | GVPSRFSGSGSG--<br>ADFTLTINSLQPEDFATYYC | QQSYS-----------<br>------------PPLT | FGGGT<br>KVEIK |
| iPS:393<br>900 | 21-<br>225_10E12 | VK1\|O12/<br>JK4 | | DIQMTQSPSSLSA<br>SVGDRVTITC | RAS--QNIY--<br>----SYLN | WYQEKPGR<br>APKLLIY | A--------<br>TSSLQS | GVPSRFGGSGSG--<br>TDFTLTISSLQPEDFATYYC | QQNYS-----------<br>------------PPLT | FGGGT<br>KVEIE |
| iPS:393<br>920 | 21-<br>225_1H12 | VK1\|O12/<br>JK4 | | DIQMTQSPSSLSA<br>SVGDRVTITC | RAS--QNIY--<br>----RYLN | WYQEKPGR<br>APKLLIY | T--------<br>ASSLQS | GVPSRFSGSDSG--<br>TDFTLTISSLQPEDFATYYC | QQSYS-----------<br>------------PPLT | FGGGT<br>KVEIK |
| iPS:393<br>926 | 21-225_4G4 | VK1\|O12/<br>JK4 | | DIQMTQSPSSLAA<br>SVGDRVTITC | RAS--QTII--<br>----SYLN | WYQQKPGK<br>APKLLIH | T--------<br>ASSLQS | GVPSRFSGSGSG--<br>TDFTLSISSLQPEDFATYYC | QQTYS-----------<br>------------TPLT | FGGGT<br>KVEIK |

| | | | | | | | | | | |
|---|---|---|---|---|---|---|---|---|---|---|
| iPS:393 930 | 21-225_7E11 | VK1\|O12/ JK4 | | DIQMTQSPSSLSA SVGDRVTIAC | RAS--QNII-- ----SYLN | WYQQKPGK APKFLIY | T-------- ASSLQS | GVPSRFSGSGSG-- TDFTLTISSLQPEDFAIYYC | QQTYS----------- ------------TPLT | FGGGT KVEIK |
| iPS:393 932 | 21-225_10F5 | VK1\|O12/ JK4 | | DIQMTQSPSSLSA SVGDRVTITC | RAS--QNIY-- ----RYLN | WYQEKPGR APKLLIY | T-------- ASSLQS | GVPSRFSGSDSG-- TDFTLTISSLQPEDFATYYC | QQSYS----------- ------------PPLT | FGGGT KVEIK |
| iPS:393 964 | 21-225_6G1 | VK1\|O12/ JK4 | | DIQMTQSPSSLSA SVGDRVTITC | RTS--QNII-- ----SYLN | WYQQKPGK APKVLIY | T-------- ASNLQT | GVPSGFSGSGSG-- TDFTLTISSLQPEDFATYYC | QQPHS----------- ------------PPLT | FGGGT KVEIK |
| iPS:394 012 | 21-225_15A3 | VK1\|O12/ JK4 | | DIQMTQSPSSLSA SVGDRVTITC | RAS--QSII-- ----SYLN | WYLQKPGK APKFLIY | T-------- ASSLQS | GVPSRFSGSGSG-- TDFTLTISSLQPEDFATYYC | QQTYS----------- ------------TPLT | FGGGT KVEIK |
| iPS:394 016 | 21-225_13D4 | VK1\|O12/ JK4 | | DLQMTQSPSSLSA SVGDRVTITC | RAS--QSIF-- ----SYLN | WYQQKPGK APKLLIC | T-------- ASSLQN | GVPSRFSGSGSG-- TDFTLTISSLQPEDFATYYC | QQTYS----------- ------------LPLT | FGGGT KVEIK |
| iPS:394 083 | 21-225_16E6 | VK1\|O12/ JK4 | | DIQMTQSPSSLSA SVGDRVTITC | RAS--QSII-- ----SYLN | WYQQKPGK APKFLIY | T-------- TSSLQS | GVPSRFSGSGSG-- TDFTLTISSLQPEDFATYYC | QQTYS----------- ------------TPLT | FGGGT KVEIK |
| iPS:398 480 | 21-225_17G4 | VK1\|O12/ JK4 | | DIQMTQSPSSLSA SAGDRVTITC | RTS--QNIS-- ----NYLN | WYQQKPGK APKLLIY | V-------- ASSFPS | GVPSRFSGSGSG-- TEFTLTISSLQPEDFATYYC | QQSNF----------- ------------FPLT | FGGGT KVEII |
| iPS:398 486 | 21-225_19A1 | VK1\|O12/ JK4 | | DIQMTQSPSSLSA SVGDRVTITC | RAS--HTIT-- ----SYLN | WYQQKPGK APKFLIY | A-------- TSNLQS | GVPSRFSGSGSG-- TDFTFTISSLQPEDFAIYYC | QQSYN----------- ------------FPLT | FGGGT KVEIK |
| | | **Germline** | | **K_FR1** | **K_CDR1** | **K_FR2** | **K_CDR2** | **K_FR3** | **K_CDR3** | **K_FR4** |
| | VK2\|A18/JK 4 | | 32133 | DIVMTQTPLSLSV TPGQPASISC | KSS-- QSLLHSD- GKTYLY | WYLQKPGQ SPQLLIY | E-------- VSSRFS | GVPDRFSGSGSG-- TDFTLKISRVEAEDVGVYYC | MQGIH----------- ------------LPLT | FGGGT KVEIK |
| iPS:451 139 | 21-225_71A6 | VK2\|A18/ JK4 | | DIVMTQTPLSLSV TPGQPASISC | KSS-- QSLLRSD- GKTHLY | WYLQKPGQ PPQLLIY | E-------- VSNRFS | GVSDRFSGSGSG-- TDFTLKISRVEAEDVGVYYC | MQSKQ----------- ------------LPLT | FGGGT KVEFK |
| iPS:433 937 | 21-225_44B10 | VK2\|A18/ JK4 | | HIVMTQTPLSLSV TPGQPASISC | KSS-- QSLLHSE- GRTYLY | WYLQKPGQ PPQLLIY | E-------- ISHRFS | GVPDRFSGSGSG-- TDFTLKISRVEAEDVGVYYC | MQSIH----------- ------------LPFT | FGGGT KVEIK |
| iPS:433 979 | 21-225_46B9 | VK2\|A18/ JK4 | | DIVMTQTPLSLSV TPGQPASISC | KSS-- QSLLHSE- GKTYLY | WYLQKPGQ PPQLLIY | E-------- VSYRFS | GVPDRFSGSGSG-- TDFTLKISRMEAEDVGVYYC | MHSIQ----------- ------------YPLT | FGGGT KVEIQ |
| iPS:434 201 | 21-225_59A12 | VK2\|A18/ JK4 | | DIVMTQTPLSLSV TPGQPASISC | KSS-- QSLQHGE- GKTYLY | WYVQKPGQ PPQLLIY | E-------- VSYRFS | GVPDRFSGSGSG-- TDFTLKISRVEVEDVGVYYC | MQSTQ----------- ------------LPLT | FGGGT KVEIK |
| iPS:434 205 | 21-225_60G2 | VK2\|A18/ JK4 | | DIVMTQTPLSLSV TPGQSASISC | KSS-- QSLLHSE- GKTYLY | WYLQKPGQ PPQLLIY | E-------- VSNRIS | GVPDRFSGSGSG-- TDFTLKISRVEAEDVGVYYC | MQSIQ----------- ------------LPLT | FGGGT KVEIK |
| iPS:434 223 | 21-225_60C12 | VK2\|A18/ JK4 | | DIVMTQTPLSLSV TPGQPASISC | KSS-- QSLLHSE- GKTYLY | WYLQKPGQ PPQFLIY | E-------- VSNRFS | GVPDRFSGSGSG-- TDFTLKISRVEAEDVGIYYC | MQSIK----------- ------------YPLT | FGGGT KVEIK |
| iPS:434 233 | 21-225_61B3 | VK2\|A18/ JK4 | | DIVMTQTPLSLSV TPGQPASISC | KSS-- QSLLHSE- GKTYLY | WYLQKPGQ PPQLLIY | E-------- VSNRIS | GVPDRFSGSGSG-- TDFTLKISRVEAEDVGVYYC | MQSIQ----------- ------------LPLT | FGGGT KVEIK |
| iPS:434 303 | 21-225_58H11 | VK2\|A18/ JK4 | | DIVMTQTPLSLSV TPGQPASISC | KSS-- QSLLHSE- GKTYLY | WYVQKPGQ PPQLLIY | E-------- VSYRFS | GVPDRFSGSGSG-- TDFTLKISRVEAEDVGVYYC | MQSIQ----------- ------------LPLT | FGGGT KVEIK |
| iPS:435 349 | 21-225_148F5 | VK2\|A18/ JK4 | | DIVMTQTPLSLSV TPGQPASISC | KSS-- QSLLHSE- | WYLQKPGQ PPQLLIY | E-------- VSYRVS | GVPDRFSGSGSG-- TDFTLKISRVEAEDVGVYFC | MQSIQ----------- ------------LPLT | FGGGT KVEIK |

| | | | | | | | | | | |
|---|---|---|---|---|---|---|---|---|---|---|
| iPS:435 359 | 21-225_148H10 | VK2\|A18/JK4 | | DIVMTQTPLSLSV TPGQPASISC | KSS-- QSLLHSE-GKTYLY | WYLQKPGQ PPQLLIY | E-------- VSYRVS | GVPDRFSGSGSG-- TDFTLKISRVEAEDVGVYYC | MQSIQ----------- ------------LPLT | FGGGT KVEIK |
| iPS:435 417 | 21-225_150D11 | VK2\|A18/JK4 | | DIVMTQTPLSLSV TPGQPASISC | KSS-- QSLLHSE-GKTYLY | WYLQKPGQ PPQLLIY | E-------- VSYRFS | GVPDRFSGSGSG-- TDFTLKISRVEAEDVGVYYC | MQGIQ----------- ------------LPLT | FGGGT KVEIK |
| iPS:435 469 | 21-225_153G9 | VK2\|A18/JK4 | | DIVMTQTPFSLSV TPGQPASISC | KSS-- QSLLHSD-GKTYLY | WYLQKPGQ PPQFLIY | E-------- VSNRFS | GVPDRFSGSGSG-- TDFTLKISRVEADDVGVYYC | MQNIK----------- ------------YPLT | FGGGT KVEIK |
| iPS:435 733 | 21-225_173C11 | VK2\|A18/JK4 | | DIVMTQTPLSLSV TPGQPASISC | KSS-- QSLLHSE-GKTYLY | WYLQKPGQ PPQLLIY | E-------- VSHRFS | GVPDRFSGSGSG-- TDFTLKISRVEAEDVGVYYC | MQSI------------ ------------QLLT | FGGGT KVEIK |
| iPS:435 785 | 21-225_179C2 | VK2\|A18/JK4 | | DIVMTQTPLSLSV TPGQPASISC | KSS-- QSLLHSE-GKTYLY | WYLQKPGQ PPQLLIY | E-------- VSHRFS | GVPDRFSGSGSG-- TDFTLKISRVEAEDVGVYYC | MQSI------------ ------------QVLT | FGGGT KVEIK |
| iPS:392 618 | 21-225_16F10 | VK2\|A18/JK4 | | DIVMTQTPLSLSV IPGQPASISC | KSS-- QSLLHSD-GKTHLN | WYLQKPGQ PPQLLIY | E-------- VSYRFS | GVPDRFSGSGSG-- TVFTLEISRVEAADVGVYYC | FQSIQ----------- ------------LPLT | FGGGT KVEIK |
| iPS:392 860 | 21-225_22H8 | VK2\|A18/JK4 | | DIVMTQTPLSLSV TPGQPASISC | KSS-- QSLLHSD-GKTFLY | WYLQKPGH PPQLLIY | E-------- VSNRFS | GVPDRFSGSGSG-- TDFTLKISRVEAEDVGIYYC | MQSIQ----------- ------------LPLS | FGGGT KVEIN |
| iPS:392 888 | 21-225_25A2 | VK2\|A18/JK4 | | DIVMNQTPLSLSV TPGQPASISC | KSS-- QSLLHSE-GKTYLY | WYLQKPGQ PPQLLIY | E-------- ISNRFS | GVPARLSGSGSG-- TDFTLKISRVEAEDVGVYYC | MQSTQ----------- ------------FPLT | FGGGT KVEIK |
| iPS:392 938 | 21-225_29H4 | VK2\|A18/JK4 | | DIVMTQTPLSLSV TPGQPASFSC | KSS-- QSLLHSD-GKTYLY | WYLQKPGQ PPQLLIF | E-------- VSHRFS | GLPDRFSGSGSG-- TDFTLKISRVEAEDVGVYYC | MQSIQ----------- ------------HPFT | FGGGT RVEIK |
| iPS:392 994 | 21-225_26G11 | VK2\|A18/JK4 | | DIVMTQTPLSLSV TPGQPASISC | KSS-- QTLLHGE-GKTYLY | WYLQKPGQ PPHLLIY | E-------- VSNRFS | GVPDRFSGSGSG-- TDFTLKISRVEAEDVGIYYC | MQSIK----------- ------------LPLT | FGGGT KVEIK |
| iPS:393 012 | 21-225_26G7 | VK2\|A18/JK4 | | DILMTQTPLSLSV TPGQPASISC | KSS-- QSLLHSE-GKTYLY | WYLQKPGQ PPQFLIY | E-------- VSHRLS | GVPDRFSGSGSG-- TDFTLKISRVEAEDVGVYYC | MQSIQ----------- ------------LPLT | FGGGT KVEIK |
| iPS:393 144 | 21-225_34D2 | VK2\|A18/JK4 | | DIVMTQTPLSLSV TPGQPASISC | KSS-- QSLLHSD-GKTYLY | WYLQKPGQ PPQLLIY | E-------- VSNRFS | GVPDRFSGSGSG-- TDFTLKISRVEAEDVGVYYC | MQSK------------ ------------QLPP | FGGGT KVEIR |
| | | Germline | | **K_FR1** | **K_CDR1** | **K_FR2** | **K_CDR2** | **K_FR3** | **K_CDR3** | **K_FR4** |
| | VK1\|L1/JK3 | | 32134 | DIQMTQSPSSLSA SVGDRVTITC | RAS--QGIS-- ----NYLA | WFQQKPGK APKSLIY | A-------- ASSLQS | GVPSRFSGSGSG-- TDFTLTISSLQPEDFATYYC | QQYNS----------- ------------YPFT | FGPGT KVDIK |
| iPS:451 143 | 21-225_66H11 | VK1\|L1/JK3 | | DIQMTQFPSSLFA FVGDRVTITC | PAS--QGIS-- ----NYLA | WFQQKPGK APKSLIY | G-------- AFNLHS | GVPSKFSGSGFG-- TDFTLTINSLQPEDFANYYC | QQYSC----------- ------------YPFT | FGHGT KVDIK |
| iPS:468 814 | 21-225_223D11 | VK1\|L1/JK3 | | DIQMTQSPSSLSA SVGDRVTITC | RAS--QGIS-- ----NYLA | WFQQKPGK APKSLIY | A-------- ASTLQS | GVPSKFSGSRSG-- TDFNLTISNLQPEDFATYYC | QQYSG----------- ------------YPFT | FGPGT KVDTK |
| iPS:433 901 | 21-225_43A4 | VK1\|L1/JK3 | | DIQMTQSPSSLSA SVGDRVTITC | RAS--QGIN-- ----NYLA | WFQQKPGK APKSLIN | A-------- ASSLQS | GVPSRFSGSGSG-- TDFTLAISSLQPEDFATYYC | QQYYS----------- ------------YPFT | FGPGT KVDIK |

| | | | | | | | | | | |
|---|---|---|---|---|---|---|---|---|---|---|
| **iPS:434 059** | **21-225_51C5** | **VK1\|L1/J K3** | | DIQMTQSPSSLSA<br>SVGDRVTITC | RAS--QGIS--<br>----NYLA | WFQQKPGE<br>APKSLIY | A--------<br>ASSLRS | GVPSQFSGSGSG--<br>TDFILTISSLQPEDFATYYC | QQYYS-----------<br>------------YPFT | FGPGT<br>KVDIK |
| **iPS:434 085** | **21-225_52E3** | **VK1\|L1/J K3** | | DIQMTQSPSSLSA<br>SVGDRVTITC | RAS--QGIS--<br>----NYLA | WFQQKPGK<br>APKSLIN | A--------<br>ASSLQS | GVPSKFSGSGSG--<br>TDFTLTISSLQPEDFATYYC | QQYNS-----------<br>------------FPFT | FGPGT<br>KVDIK |
| **iPS:434 115** | **21-225_53E4** | **VK1\|L1/J K3** | | DIQMTQSPSSLSA<br>SVGDRVTITC | RAS--QVIS--<br>----NYLA | WFQQKPGK<br>APKSLIS | A--------<br>ASSLQS | GVPSKFSGSGSG--<br>TDFTLTISSLQPEDFATYYC | QQYHS-----------<br>------------YPLT | FGRGT<br>KVDIK |
| **iPS:434 213** | **21-225_60A4** | **VK1\|L1/J K3** | | DIQMTQSPSSLSA<br>SVGDRVTITC | RAS--QGIS--<br>----NYLA | WFQQKPGK<br>APKSLIY | A--------<br>ASSLQS | GVPSKFSGSGSG--<br>TDFTLTISSLQSEDFATYYC | QQYKS-----------<br>------------HPFT | FGPGT<br>KVDIK |
| **iPS:434 215** | **21-225_60F7** | **VK1\|L1/J K3** | | DIQMTQSPSSLSA<br>SVGDRVTISC | RAS--QVIK--<br>----NYLV | WVQQKPGK<br>APKSLIY | A--------<br>ASSLQS | GVPSTFSGSGSG--<br>TDFTLTISSLQPEDFATYYC | LQFHS-----------<br>------------YPFT | FGPGT<br>KMDIK |
| **iPS:434 261** | **21-225_56F7** | **VK1\|L1/J K3** | | DILMTQSPSSLSA<br>SVGDRVTITC | RAS--QGIS--<br>----TYLA | WFQQTPGT<br>APKSLIY | A--------<br>ASSLQG | GVPSKFSGSGSG--<br>TDFTLTISSLQPEDFATYYC | HQYNS-----------<br>------------FPFK | FGRGT<br>KVDIT |
| **iPS:434 331** | **21-225_63H8** | **VK1\|L1/J K3** | | DIQMTQSPSSLSA<br>SVGDRVTITC | RAS--QGIS--<br>----NYLA | WFQQKPGK<br>AHKSLIY | A--------<br>ASSLQS | GVPSKFSGSGSG--<br>TDFTLTISSLQPEDFATYYC | QQYHS-----------<br>------------YPFT | FGPGT<br>KVDIR |
| **iPS:434 361** | **21-225_65D5** | **VK1\|L1/J K3** | | DIQMTQSPSSLSA<br>SVGDRVTITC | RAS--QDIN--<br>----NYLA | WFQQKPGK<br>APRSLIY | A--------<br>ASSLHS | GVPSQFSASGSG--<br>SDFTLTISSLQPEDFATYYC | PLYKS-----------<br>------------YPLT | FGPGT<br>KVDIK |
| **iPS:434 405** | **21-225_68E6** | **VK1\|L1/J K3** | | DIQMTQSPSSLSA<br>SVGDRVTITC | RAS--QGIS--<br>----YYLA | WFQQKPGR<br>APKSLIY | V--------<br>ASSLQS | GVPSRFSGSGSG--<br>TDFTLTISSLQPEDFAIYYC | QQYDS-----------<br>------------YPFT | FGPGT<br>KVDIR |
| **iPS:435 259** | **21-225_96C6** | **VK1\|L1/J K3** | | DIQMTQSPSSLSA<br>SVGDRVTITC | RAS--QGIS--<br>----NYLA | WFQQKPGK<br>APKSLIY | A--------<br>ASSLQS | GVPSKFSGSGSG--<br>TDFTLTINSLQPEDFATYYC | HQYND-----------<br>------------YPFT | FGPGT<br>KVDIK |
| **iPS:435 351** | **21-225_148B6** | **VK1\|L1/J K3** | | DIQMTQSPSSLSA<br>SVGDRVSITC | RAS--QGIS--<br>----KYLA | WFQQKPGK<br>APKSLIF | A--------<br>ASSLQS | GVPSKFSGSGSG--<br>TDFTLTISSLQPEDFATYYC | QQYNS-----------<br>------------FPFT | FGPGT<br>KVDIK |
| **iPS:435 461** | **21-225_153A1** | **VK1\|L1/J K3** | | DIQMTQSPSSLSA<br>SVGDRVTITC | RAS--QVIS--<br>----NYLA | WFQQKPGK<br>APKSLIS | A--------<br>ASSLRS | GVPSNFSGSGSG--<br>TDFTLTISSLQPEDFATYYC | QQYHS-----------<br>------------YPFT | FGPGT<br>KVDFK |
| **iPS:435 509** | **21-225_157H1** | **VK1\|L1/J K3** | | DIQMTQSPSSLSA<br>SVGDRVTITC | RAS--QDIS--<br>----NYLV | WFQQRPGK<br>APRSLIY | A--------<br>ASSLQS | GVPSKFSGSGSG--<br>TDFTLTISSLQPEDFATYYC | QQYHS-----------<br>------------YPFT | FGPGT<br>KVDIK |
| **iPS:435 515** | **21-225_157E4** | **VK1\|L1/J K3** | | DIQMTQSPSSLSA<br>SVGDRVTFTC | RAS--QGIS--<br>----NYLA | WFQQKPGR<br>APKSLIY | A--------<br>ASSLRS | GVPSQFSGSGSG--<br>TDFTLTINSLQPEDFATYYC | QQYHS-----------<br>------------YPFT | FGPGT<br>KVDIK |
| **iPS:435 523** | **21-225_157G5** | **VK1\|L1/J K3** | | DIQMTQSPSSLSA<br>SVGDRVTITC | RAS--QGIN--<br>----NYLA | WFQQKPGK<br>APESLIY | A--------<br>ASSLQS | GVPSQFSGSGSG--<br>TDFTLTISSLQPEDFATYYC | QQYNS-----------<br>------------YPFT | FGPGT<br>KVDIK |
| **iPS:435 535** | **21-225_157H10** | **VK1\|L1/J K3** | | DIQMTQSPSSLSA<br>SVGDRVTITC | RAS--QGIT--<br>----NYLA | WFQQKPGK<br>APKSLIY | T--------<br>ASNLQS | GVPSKFSGSGSG--<br>TDFTLTISSLQPEDFATYYC | QQYHS-----------<br>------------YPFT | FGPGT<br>KVDIK |
| **iPS:435 559** | **21-225_158H12** | **VK1\|L1/J K3** | | DIQMTQSPSSLSA<br>SVGDRVTITC | RAS--QGIN--<br>----NYLA | WFQQKPGK<br>APKSLIS | A--------<br>ASSLQS | GVPSKFSGSGSG--<br>TDFTLSISSLQPEDFATYYC | QQYHS-----------<br>------------YPFT | FGPGT<br>KVDIK |
| **iPS:435 575** | **21-225_159H11** | **VK1\|L1/J K3** | | DIQMTQSPSSLSA<br>SVGDRVTITC | RAS--QGIS--<br>----KYLV | WFQQKPGK<br>APKSLIY | A--------<br>ASSLQS | GVPSKFSGSGSG--<br>TDFTLTISNLQPEDFATYYC | QQYNS-----------<br>------------YPFT | FGPGT<br>KVDIK |
| **iPS:435 579** | **21-225_160G1** | **VK1\|L1/J K3** | | DIQMTQSPSSLSA<br>SVGDRVTITC | RAS--QDIN--<br>----NYLA | WFQQKPGK<br>APTSLIY | A--------<br>SSSLQS | GVPSKFSGSGSG--<br>TDFTLTISSLQPEDFATYYC | QQYHS-----------<br>------------YPFT | FGPGT<br>KVDIR |
| **iPS:435 585** | **21-225_160G3** | **VK1\|L1/J K3** | | DIQMTQSPSSLSA<br>SVGDRVTITC | RAS--QDIN--<br>----NYLA | WFQQRPGK<br>APTSLIY | A--------<br>SSSLQS | GVPSKFSGSGSG--<br>TDFTLTISSLQPEDFATYYC | QQYHS-----------<br>------------YPFT | FGPGT<br>KVDIK |
| **iPS:435 635** | **21-225_163F1** | **VK1\|L1/J K3** | | DIQMTQSPSSLSA<br>SVGDRVTITC | RAS--QDIS--<br>----NYLA | WFQQKPGK<br>APKSLIY | A--------<br>ASSLQS | GVPSKFSGSGSG--<br>TDFTLTISSLQPEDFAAYYC | QQYNS-----------<br>------------YPFT | FGPGT<br>QVDAQ |

<table>
<tr><td>iPS:435 659</td><td>21-225_167D12</td><td>VK1|L1/JK3</td><td></td><td>DIQMTQSPSSLSA<br>SVGDRVTITC</td><td>RAS--QGIN--<br>----NYLA</td><td>WFQQKPGK<br>APESLIY</td><td>A--------<br>ASSLQS</td><td>GVPSKFSGSGSG--<br>TDFTLTISSLQPEDFATYFC</td><td>QQYNS-----------<br>------------YPFT</td><td>FGPGT<br>KVDIK</td></tr>
<tr><td>iPS:435 679</td><td>21-225_169D10</td><td>VK1|L1/JK3</td><td></td><td>DIQMTQSPSSLSA<br>SVGDRVTITC</td><td>RAS--QDIS--<br>----NYLA</td><td>WFQQKPGK<br>APKSLIY</td><td>A--------<br>ASSLQS</td><td>GVPSKFSGSGSG--<br>TDFTLTISSLQPEDFATYYC</td><td>QQYHS-----------<br>------------YPFT</td><td>FGPGT<br>KVDIK</td></tr>
<tr><td>iPS:435 685</td><td>21-225_170E1</td><td>VK1|L1/JK3</td><td></td><td>DIQMTQSPSSLSA<br>SEGDRVTITC</td><td>RAS--QGIS--<br>----NYLA</td><td>WFQQKPGK<br>APKSLIY</td><td>A--------<br>ASSLQS</td><td>GVPSKFSGSGSG--<br>TDFTLTISSLQPEDFATYYC</td><td>QQYHS-----------<br>------------YPFT</td><td>FGPGT<br>KVDIK</td></tr>
<tr><td>iPS:435 747</td><td>21-225_175C4</td><td>VK1|L1/JK3</td><td></td><td>DIQMTQSPSSLSA<br>SVGDRVTITC</td><td>RAS--QGIG--<br>----NYLA</td><td>WFQQKPGK<br>APKSLIY</td><td>A--------<br>ASGLQS</td><td>GFPSKFSGSGSG--<br>TDFTLTISSLQPEDFATYYC</td><td>QQYYS-----------<br>------------YPFT</td><td>FGPGT<br>KVDIK</td></tr>
<tr><td>iPS:435 765</td><td>21-225_177D3</td><td>VK1|L1/JK3</td><td></td><td>DIQMSQSPSSLSA<br>SVGDRVTITC</td><td>RAS--QGIT--<br>----NYLA</td><td>WFQQKPGK<br>APKSLIY</td><td>A--------<br>ASSLQS</td><td>GVPSKFSGSGSG--<br>TDFTLTISSLQPEDFATYYC</td><td>QQYNS-----------<br>------------YPFT</td><td>FGPGT<br>KVDIK</td></tr>
<tr><td>iPS:435 797</td><td>21-225_181G2</td><td>VK1|L1/JK3</td><td></td><td>DIQMTQSPSSLSA<br>SIGERVTITC</td><td>RAS--QGIS--<br>----NYLA</td><td>WIQQKPGT<br>APKSLIY</td><td>A--------<br>ASSLQS</td><td>GVSSRFSGSGFG--<br>TDFTLTISSLQPEDFATYYC</td><td>QQYNG-----------<br>------------YPFT</td><td>FGPGT<br>KVDIK</td></tr>
<tr><td>iPS:435 835</td><td>21-225_190F12</td><td>VK1|L1/JK3</td><td></td><td>DIQMTQSPSSLSA<br>SVGDRVTITC</td><td>RAS--QGIG--<br>----KYLA</td><td>WFQQKPGK<br>APKSLIY</td><td>A--------<br>ASSLQS</td><td>GVPSKFSGSGSG--<br>TDFTLTISSLQPDDFATYYC</td><td>QRYDT-----------<br>------------YPFT</td><td>FGPGT<br>KVDIK</td></tr>
<tr><td>iPS:435 861</td><td>21-225_190A5</td><td>VK1|L1/JK3</td><td></td><td>DIQMTQSPSSLSA<br>SVGDRVTITC</td><td>RAS--QGIG--<br>----NHLA</td><td>WFQQKPGK<br>APKSLIH</td><td>A--------<br>ASSLQS</td><td>GVPSKFSGSGSG--<br>TDFTLTISSLQPEDFATYYC</td><td>QQYSN-----------<br>------------YPVT</td><td>FGPGT<br>KVDIK</td></tr>
<tr><td>iPS:435 869</td><td>21-225_190B1</td><td>VK1|L1/JK3</td><td></td><td>DIQMTQSPSSLSA<br>SVGDRVTITC</td><td>RAS--QGIR--<br>----NYLA</td><td>WFQQKPGK<br>APKSLIY</td><td>V--------<br>ASSLES</td><td>GVPSKFSGSGSG--<br>TEFTLTISSLQPEDFGTYYC</td><td>QQYLN-----------<br>------------YPVT</td><td>FGPGT<br>KVDIR</td></tr>
<tr><td>iPS:435 877</td><td>21-225_184E7</td><td>VK1|L1/JK3</td><td></td><td>DIQMTQSPSSRSA<br>SIGERVTITC</td><td>RAS--QGIS--<br>----NYLA</td><td>WIQQKPGT<br>APKSLIY</td><td>A--------<br>ASSLQS</td><td>GVSSRFSGSGFG--<br>TDFTITISSVQREDFATYYC</td><td>QQYNG-----------<br>------------YPFT</td><td>FGHGT<br>KVDIK</td></tr>
<tr><td>iPS:435 883</td><td>21-225_185A1</td><td>VK1|L1/JK3</td><td></td><td>DIQMTQSPSSLSA<br>SVGDRVTITC</td><td>RAS--QGIS--<br>----NYLA</td><td>WFQQTPGK<br>APKSLIS</td><td>V--------<br>ASSLQS</td><td>GVPSRFSASGSG--<br>TDFTLTISSLQPEDFATYYC</td><td>RQYHS-----------<br>------------YPFT</td><td>FGPGT<br>KVDIK</td></tr>
<tr><td>iPS:435 885</td><td>21-225_185E10</td><td>VK1|L1/JK3</td><td></td><td>DIQMTQSPSSRSA<br>SIGERVTITC</td><td>RAS--QGIS--<br>----NYLA</td><td>WIQQKPGT<br>APKSLIY</td><td>A--------<br>ASSLQS</td><td>GVSSRFSGSGFG--<br>TDFTLTISSLQPEDFATYYC</td><td>QQYNG-----------<br>------------YPFT</td><td>FGPGT<br>KVDIK</td></tr>
<tr><td>iPS:435 891</td><td>21-225_188H5</td><td>VK1|L1/JK3</td><td></td><td>DIQMTQSPSSLSA<br>SIGERVTITC</td><td>RAS--QGIS--<br>----NYLA</td><td>WLQQKPGT<br>APKSLIY</td><td>A--------<br>ASSLQS</td><td>GVSSRFSGSGFG--<br>TDFTLTISSLQPEDFATYYC</td><td>QQYNS-----------<br>------------YPFT</td><td>FGPGT<br>KVDIK</td></tr>
<tr><td>iPS:435 897</td><td>21-225_188B9</td><td>VK1|L1/JK3</td><td></td><td>DIQMTQSPSSLSA<br>SIGERVTITC</td><td>RAS--QGIS--<br>----NYLA</td><td>WLQQKPGT<br>APKSLIY</td><td>A--------<br>ASSLQS</td><td>GVSSRFSGSGFG--<br>TDFTLTISSLQPEDFATYYC</td><td>QQYNS-----------<br>------------YPFT</td><td>FGPGT<br>KVDIK</td></tr>
<tr><td>iPS:435 937</td><td>21-225_190H9</td><td>VK1|L1/JK3</td><td></td><td>DIQMTQSPSSLSA<br>SVGDRVTITC</td><td>RAS--QGIG--<br>----KYLA</td><td>WFQQKPGK<br>ALKSLIY</td><td>A--------<br>ASSLQS</td><td>GVPSKFSGSGSG--<br>TDFTLTISSLQPDDFATYYC</td><td>QRYDT-----------<br>------------YPFT</td><td>FGPGT<br>KVDIK</td></tr>
<tr><td>iPS:435 961</td><td>21-225_192A2</td><td>VK1|L1/JK3</td><td></td><td>DIQMTQSPSSLSA<br>SVGDRVTITC</td><td>RAN--QGIN--<br>----NYLA</td><td>WFQQKPGK<br>APKSLIY</td><td>A--------<br>ASSLQS</td><td>GVPSKFSGSGSG--<br>TDFTLTISSLQPEDFATYYC</td><td>QQYNS-----------<br>------------YPFT</td><td>FGPGT<br>KVDIK</td></tr>
<tr><td>iPS:435 977</td><td>21-225_192E4</td><td>VK1|L1/JK3</td><td></td><td>DIQMTQSPSSLSA<br>SVGDRVTITC</td><td>RAS--QGIG--<br>----NYLA</td><td>WFQQKPGK<br>APKSLIY</td><td>V--------<br>VSSLQS</td><td>GVPSRFSGSGSG--<br>TDFTLTISSLQPEDFATYYC</td><td>QRYDT-----------<br>------------YPFT</td><td>FGPGT<br>KVDIK</td></tr>
<tr><td>iPS:436 001</td><td>21-225_192C10</td><td>VK1|L1/JK3</td><td></td><td>DIQMTQSPSSLSA<br>SVGDRVTITC</td><td>RAS--QGIG--<br>----KYLA</td><td>WFQQKPGK<br>APKSLIY</td><td>A--------<br>ASSLQS</td><td>GVPSKFSGSGSG--<br>TDFTLTISSLQPDDFATYYC</td><td>QRYDT-----------<br>------------YPFT</td><td>FGPGT<br>KVDFK</td></tr>
<tr><td>iPS:436 039</td><td>21-225_193F8</td><td>VK1|L1/JK3</td><td></td><td>DIQMIQSPSSLSA<br>SVGDRVTITC</td><td>RAS--QGVS--<br>----NHLA</td><td>WFQQKPGR<br>APKSLIY</td><td>A--------<br>ASSLQS</td><td>GVPSKFSGSGSG--<br>ADFTLTISSLQPEDFATYYC</td><td>QQYNS-----------<br>------------YPFT</td><td>FGPGT<br>KVDIK</td></tr>
<tr><td>iPS:436 078</td><td>21-225_194H12</td><td>VK1|L1/JK3</td><td></td><td>DIQMTQSPSSLSA<br>SVGDRVTITR</td><td>RAS--QGIG--<br>----KYLA</td><td>WFQQKPGK<br>ALKSLIY</td><td>A--------<br>ASSLQS</td><td>GVPSKFSGSGSG--<br>TDFTLTISSLQPDDFATYYC</td><td>QRYDT-----------<br>------------YPFT</td><td>FGPGT<br>KVDIK</td></tr>
<tr><td>iPS:436</td><td>21-</td><td>VK1|L1/J</td><td></td><td>DIQMTQSPSSLSA</td><td>RAS--QGIG--</td><td>WFQQKPGK</td><td>A--------</td><td>GVPSKFSGSRSG--</td><td>QQYSN-----------</td><td>FGPGT</td></tr>
</table>

| | | | | | | | | | | |
|---|---|---|---|---|---|---|---|---|---|---|
| **iPS:436 167** | **21-225_197E1 1** | **VK1\|L1/J K3** | | DIQMTQSPSSLSA<br>SVGDRVTITC | RAS--QGIN--<br>----KYLS | WFQQKPGK<br>APKSLIY | A--------<br>ASSVQS | GVPSKFSGSGSG--<br>TDFTLTISRLQPDDFATYYG | QRYDT-----------<br>------------YPFT | FGPGT<br>KVDIK |
| **iPS:436 370** | **21-225_211A6** | **VK1\|L1/J K3** | | DIQMTQSPSSLSA<br>SVGDSVTITC | RAS--QGIG--<br>----KYLA | WFQQKPGK<br>APKSLIY | A--------<br>ASSLLS | GVPSKFSGSGSG--<br>TDFTLTISSLQPEDFATYYC | QKYDT-----------<br>------------YPFT | FGPGT<br>KVDIK |
| **iPS:436 392** | **21-225_213B3** | **VK1\|L1/J K3** | | DIQMTQSPSSLSA<br>SVGDRVTITC | RAS--QGIG--<br>----KYLA | WFQQKPGK<br>APKSLIS | A--------<br>ASSVLS | GVPSKFSGSGSG--<br>TDFTLTISSLQPEDFATYYC | QKYDT-----------<br>------------YPFT | FGPGT<br>KVDIK |
| **iPS:436 404** | **21-225_214C3** | **VK1\|L1/J K3** | | DIQMTQSPSSLSA<br>SVGDRVTITC | RAS--QGIG--<br>----NYLA | WFQQKPGK<br>VPKSLIY | A--------<br>ASSLQS | GVPSKFSGSGSG--<br>TDFTLTISSLQPEDFTTYYC | QQYMT-----------<br>------------YPIT | FGPGT<br>KVDIK |
| **iPS:436 406** | **21-225_214E4** | **VK1\|L1/J K3** | | DIQMTQSPSSLSA<br>SVGDRVTITC | RAS--QGIS--<br>----NYLA | WFQQKPGK<br>APKSLIY | A--------<br>ASSLQS | GVPSKFSGSGSG--<br>TDFTLTISSLQPEDFATYYC | QQYLT-----------<br>------------YPFT | FGPGT<br>KVDIK |
| **iPS:437 216** | **21-225_51D5** | **VK1\|L1/J K3** | | DIQMTQSPSSLSA<br>SVGDRVTITC | RAS--QGIN--<br>----NYLA | WFQQKPGE<br>APKSLIY | A--------<br>ASSLRS | GVPSQFSGSGSG--<br>TDFILTISSLQPEDFATYYC | QQYYS-----------<br>------------YPFT | FGPGT<br>KVDIK |
| **iPS:437 224** | **21-225_56H1** | **VK1\|L1/J K3** | | DIQMTQSPSSLSA<br>SVGDRVTITC | RAS--QGIS--<br>----HYLA | WFQQKPGK<br>APQSLMS | A--------<br>ASGLQS | GVPSKFSGSGSG--<br>TDFTLTISSLQPEDFATYYC | QQYQN-----------<br>------------YPFT | FGPGT<br>KVDIK |
| **iPS:392 620** | **21-225_17H5** | **VK1\|L1/J K3** | | DIQMTQSPSSLSA<br>SVGDRVTITC | RAS--QGIS--<br>----NYLA | WFQQKPGK<br>APKSLIN | A--------<br>ASSLQS | GVPSKFSGSGSG--<br>TDFTLTISSLQPEDFATYYC | QQYHS-----------<br>------------YPFT | FGPGT<br>KVDIK |
| **iPS:392 692** | **21-225_18G10** | **VK1\|L1/J K3** | | DIQMTQSPSSLSA<br>SVGDRVTITC | RAS--QDIS--<br>----YYLA | WFQQKPGK<br>APKSLIY | V--------<br>ASSLQS | GVPSKFGGSGFG--<br>TDFTLTISSLQPEDFATYYC | LQYNS-----------<br>------------YPFT | FGPGT<br>KVDIK |
| **iPS:392 708** | **21-225_18D11** | **VK1\|L1/J K3** | | DIQMTQSPSSLFA<br>FVGDRVTITC | RAS--QGIS--<br>----YYLA | WFQQKPGK<br>APKSLIY | V--------<br>ASSLQS | GVPSKFSGSGFG--<br>TDFTLTISSLQPEDFATYYC | QQYNT-----------<br>------------YPFT | FGPGT<br>TVDIK |
| **iPS:392 714** | **21-225_16G12** | **VK1\|L1/J K3** | | DIQMTQSPSSLSA<br>SVGDRVTITC | RAS--QDIS--<br>----NYLA | WFQQKPGK<br>APKSLIY | A--------<br>ASSLQS | GVPSKFRGSGSG--<br>TDFTLTISNLQPEDFASYYC | QQYHS-----------<br>------------FPFT | FGPGT<br>KVDIK |
| **iPS:392 746** | **21-225_20H7** | **VK1\|L1/J K3** | | DIQMTQSPSSLSA<br>SVGDRVTITC | RTS--QGIN--<br>----NYLV | WFQQKPGK<br>APKRLIY | A--------<br>ASSLQS | GVPSKFSGSGSG--<br>TDFTLTISSLQPEDFATYYC | QQYYS-----------<br>------------YPFT | FGPGT<br>KMDFK |
| **iPS:392 782** | **21-225_22B12** | **VK1\|L1/J K3** | | DIQMTQSPSSLSA<br>SVGDRVTIIC | RAS--QDIS--<br>----NYLA | WFQEKPGK<br>AHKSLIY | G--------<br>ASSLRS | GVPSKFSGSGSG--<br>TDFNLTISSLQPEDLATYYC | QQYHS-----------<br>------------YPFT | FGPGT<br>KVDFK |
| **iPS:392 784** | **21-225_23C7** | **VK1\|L1/J K3** | | DIQMTQSPSSLSA<br>SVGDRVTITC | RAS--QGIG--<br>----IYLA | WFQQKPGK<br>APKSLIS | A--------<br>ASSLQS | GVPSKFSGSGSG--<br>TEFTLTISSLQPEDFATYYC | QQYNS-----------<br>------------YPFT | FGPGT<br>KVDIK |
| **iPS:392 802** | **21-225_23E7** | **VK1\|L1/J K3** | | DIQMTQSPSSLSA<br>SVGDRVTITC | RAS--QGIS--<br>----NYLA | WFQQIPGK<br>APKSLIY | A--------<br>ASSLQS | GVPSKFSGSGSG--<br>TDFTLTISSLQPEDFATYYC | QQFYS-----------<br>------------YPFT | FGPGT<br>KVDIN |
| **iPS:392 826** | **21-225_20B9** | **VK1\|L1/J K3** | | DIQMTQSPSSLSA<br>SVGDRVTITC | RAS--QGIS--<br>----NYLA | WFQQKPGK<br>APKSLIY | V--------<br>ASSLQS | GVPSKFSGSGSG--<br>TDFTLTISSLQPEDFATYYC | QQYNT-----------<br>------------YPFT | FGPGT<br>KVDIK |
| **iPS:392 840** | **21-225_23G1** | **VK1\|L1/J K3** | | DIQMTQSPSSLSA<br>SVGDRVTITC | RAS--QGIS--<br>----NYLA | WFQQKPGK<br>APKSLIS | A--------<br>ASSLQS | GVPSQFSGSGFG--<br>TDFTLTISSLQPEDFATYYC | QQYNS-----------<br>------------YPFT | FGPGT<br>KVDIK |
| **iPS:392 842** | **21-225_23G8** | **VK1\|L1/J K3** | | DIQMTQSPSSLSA<br>SVGDRVTITC | RAS--QGIR--<br>----NYLA | WFQQKPGK<br>APKSLIY | A--------<br>ASSLQS | GVPSNFSGSGSG--<br>TDFTLTISSLQPEDFATYYC | QQYNS-----------<br>------------YPFT | IGPGT<br>KVDIK |
| **iPS:392 890** | **21-225_20H9** | **VK1\|L1/J K3** | | DIQMTQSPSSLSA<br>SVGDRVTITC | RAS--QGIS--<br>----NYLA | WFQQKPGK<br>APKSLIS | A--------<br>ASSLQS | GVPSKFSGSGSG--<br>TDFTLTISSLQPEDFATYYC | QQYNS-----------<br>------------YPFT | FGPGT<br>KVDIK |
| **iPS:392 892** | **21-225_20C11** | **VK1\|L1/J K3** | | DIQMTQSPSSLSA<br>SVGDRVTITC | RAS--QDIS--<br>----NYLA | WFQQKPGK<br>APKSLIY | A--------<br>ASSLQS | GVPSKFRGSGSG--<br>TDFTLTISSLQPEDFATYYC | QQYHS-----------<br>------------FPFT | FGPGT<br>KVDVK |
| **iPS:392 950** | **21-225_25C10** | **VK1\|L1/J K3** | | DIQMTQSPSSLSA<br>SVGDRVTITC | RAS--QGIS--<br>----NYLA | WFQQKPGK<br>APKSLIY | A--------<br>ASSLQS | GFPSKFSGSGSG--<br>TDFTLTISSLQPEDFATYYC | QQYHS-----------<br>------------YPFT | FGPGT<br>KVDIK |
| **iPS:392 952** | **21-225_26G1** | **VK1\|L1/J K3** | | DIQMTQSPSSLSA<br>SVGDRVTITC | RAS--QDIS--<br>----NYLA | WFQQKPGK<br>APKSLIY | A--------<br>ASSLRS | GVPSNFSGSGSG--<br>TDFTLTISSLQPENFATYYC | QQYHS-----------<br>------------YPFT | FGPGT<br>KVDIK |
| **iPS:392** | **21-** | **VK1\|L1/J** | | DIQMTQSPSSLSA | RAS--QGIS-- | WFQQKPGK | A-------- | GVPSKFSGSGSG-- | QQYNS----------- | FGPGT |

| | | | | | | | | | | |
|---|---|---|---|---|---|---|---|---|---|---|
| iPS:392 976 | 21-225_27H12 | VK1\|L1/JK3 | | DIQMTQSPSSLSA SVGDRVTITC | RAS--QGIS-- ----NYLA | WFQQKPGK APKSLIN | G-------- ASSLQS | GVPSKFSGSGSG-- TDFTLTISSLQPEDFATYYC | QQYYS----------- ------------YPFT | FGPGT KVNIN |
| iPS:393 090 | 21-225_33A5 | VK1\|L1/JK3 | | DIQMTQSPSSLSA SVGDRVTITC | RAS--QGIS-- ----NYLA | WFQQKPGK APKSLIS | A-------- ASSLQS | GVPSKFSGSGSG-- TDFTLTISSLQPEDFATYYC | QQYNS----------- ------------YPFT | FGPGT NVDIK |
| iPS:393 120 | 21-225_35H8 | VK1\|L1/JK3 | | DIQMTQSPSSLSA SVGDRVTITC | RAS--QAIS-- ----NYLA | WFQQKPGK APKSLIY | G-------- ASGLQS | GVPSKFSGSGSG-- TDFTLTISSLQPEDFATYYC | QQYNS----------- ------------YPFT | FGPGT KVDFK |
| iPS:393 836 | 21-225_15A2 | VK1\|L1/JK3 | | DIQMTQSPSSLFA FIGDRVTITC | RAS--QGIS-- ----NYLA | WFQQKPGK APKSLIF | A-------- ASSLQS | GVPSKFSGSGFG-- TDFTFPISSLQPEDFANYYC | QQYYS----------- ------------YPFT | FGPGT QVDVK |
| iPS:393 870 | 21-225_7B1 | VK1\|L1/JK3 | | DIQMTQAPSSLSA SVGDRVTITC | RAS--QDIS-- ----NHLV | WFQQKPGK APKSLIF | A-------- ASSLQS | GVPSQFSGSGSG-- TDFTLTISILQPEDFATYYC | HQYNS----------- ------------YPFT | FGPGT KVDFK |
| iPS:393 894 | 21-225_5E11 | VK1\|L1/JK3 | | VIQMTQSPSSLSA SVGDRVTITC | RAS--QGIS-- ----NYLA | WFQQKPGK APKSLIN | A-------- ASSVQS | GVPSKFSGNGSG-- TDFTLTISSLQPEDFATYYC | HQYHS----------- ------------YPFT | FGPGT KVDIK |
| iPS:393 896 | 21-225_2A4 | VK1\|L1/JK3 | | DIQMTQSPSSLFA SVGDRVTITC | RAS--QGIS-- ----NYLA | WFQQKPGK APKRLIY | T-------- ASSLQS | GVPSKFSGSGFG-- TDFTLTISSLQPEDFATYYC | QQYNS----------- ------------YPFT | FGPGT KVDIK |
| iPS:393 914 | 21-225_16B8 | VK1\|L1/JK3 | | DIQMTQSPSSLSA SVGDRVTITC | RAS--QGIN-- ----NYLA | WFQQKPGK ALKSLIN | A-------- ASSVQS | GVPSKFSGSGSG-- TDFTLTISSLQPEDFATYYC | HQYHS----------- ------------YPFT | FGPGT KVDIV |
| iPS:393 968 | 21-225_5A5 | VK1\|L1/JK3 | | DIQMTQSPSSLSA SVGDRVTITC | RAS--QGIS-- ----NYLA | WFQQKPGK APKSLIS | A-------- ASSLQS | GVPSKFSGSGSG-- TDFTLTISSLQPEDFATYYC | QQYNS----------- ------------YPFT | FGPGT KVDIK |
| iPS:393 992 | 21-225_14H8 | VK1\|L1/JK3 | | DIQMTQSPSSLSA SVGDRVTITC | RAS--QGIS-- ----YYLA | WFQQKPGK APKSLIY | V-------- ASSLQS | GVPSKFSGSGSG-- TDFTLTISSLQPEDFATYYC | QQYNS----------- ------------YPFT | FGPGT KVDIN |
| iPS:394 018 | 21-225_15B1 | VK1\|L1/JK3 | | DIQMTQSPSSLSA SVGDRVTITC | RAS--QGIS-- ----NYLA | WFQQKPGK APKSLIS | A-------- ASSLQS | GVPSNFSGSGSG-- TDFTLTISSLQPEDFATYYC | QQYHS----------- ------------YPFT | FGPGT KVDIK |
| iPS:394 026 | 21-225_16C7 | VK1\|L1/JK3 | | DIQMTQSPSSLSA SVGDRVTITC | RAS--QGIS-- ----NYLA | WFQQKPGK APKSLIS | A-------- ASSLQS | GVPSKFSGSGSG-- TDFTLTISSLQPEDFATYYC | QQYNS----------- ------------YPFT | FGPGT KVDIK |
| iPS:394 055 | 21-225_9C8 | VK1\|L1/JK3 | | DIQMTQSPSSLSA SVGDRVTITC | RAS--QGIS-- ----YYLA | WFQQKPGK APKSLIY | V-------- ASSLQS | GVPSKFSGSGSG-- TDFTLTISSLQPEDFATYYC | QQYDS----------- ------------YPFT | FGPGT KVDIK |
| iPS:398 482 | 21-225_17H6 | VK1\|L1/JK3 | | DIQMTQSPSSLSA SVGDRVTITC | RAS--RDIS-- ----NYLA | WFQQKPGK APKSLIS | T-------- ASSLQS | GVPSKFSGSGSG-- TDFTLTISSLQPEDFAIYYC | QQYHS----------- ------------YPFT | FGPGT KVDIQ |
| iPS:398 500 | 21-225_23A11 | VK1\|L1/JK3 | | DIQMTQSPSSLST SVGDRVTITC | RAS--QDIS-- ----NYLA | WFQQKPGK APKRLIY | A-------- ASTLQS | GVPSKFSGSGSG-- TDFTLTISSLQPEDFATYYC | QQYNS----------- ------------YPFT | FGPGT KVDLK |
| iPS:398 526 | 21-225_32B3 | VK1\|L1/JK3 | | DIQMTQSPSSLSA SIGDRVTITC | RAS--QGIS-- ----NYLA | WFQQKPGK APRSLIY | A-------- ASSLQS | GVPSTFSGSGSG-- TDFTLTISSLQPEDFATYYC | QQYNS----------- ------------YPFT | FGPGT KVDIK |
| iPS:402 235 | 21-225_20F10 | VK1\|L1/JK3 | | DIQLTQSPSSLSA SVGDRVTITC | RAS--QGIN-- ----NYLA | WFQQKPGK APKSLIY | A-------- ASSLQS | GVPSRFSGSGFG-- TDFTLTISSLQPEDFATFYC | QQYNS----------- ------------YPFT | FGPGT KVDNK |
| | | Germline | | K_FR1 | K_CDR1 | K_FR2 | K_CDR2 | K_FR3 | K_CDR3 | K_FR4 |
| | VK3\|A27/JK1 | | 32135 | EIVLTQSPGTLSL SPGERATLSC | RAS--QSVSS- ----SYLA | WYQQKPGQ APRLLIY | G-------- ASSRAT | GIPDRFSGSGSG-- TDFTLTISRLEPEDFAVYYC | QQYGS----------- ------------SPWT | FGQGT KVEIK |
| iPS:468 810 | 21-225_74D5 | VK3\|A27/JK1 | | EIVLTQSPGTLSL SPGERATLSC | RAS--QSVNS- ----NYLA | WYRQKPGQ APRLLIY | G-------- ASSRAT | GIPDRFSGSGSG-- TDFILIISRLEPEDFAVYYC | QQYES----------- ------------SPWT | FGQGT KVEIK |
| iPS:468 834 | 21-225_94G10 | VK3\|A27/JK1 | | EIVLTQSPGTLSL SPGERATLSC | RAS--QSVNS- ----NYLA | WYRQKPGQ APRLLIY | G-------- ASSRAT | GIPDRFSGSGSG-- TDFILIISRLEPEDFAVYYC | QQYES----------- ------------SPWT | FGQGT KVEIK |
| iPS:468 838 | 21-225_80E12 | VK3\|A27/JK1 | | EIVLTQCPGTLSL SPGERATVSC | RAS--QSVNS- ----NYLA | WYRQKPDQ APRLLIY | G-------- ASSRAT | GIPDRFSGSGSG-- TDFILIISRLEPEDFAVYYC | QQYES----------- ------------SPWT | FGQGT KVEIK |
| iPS:468 820 | 21-225_76E10 | VK3\|A27/JK1 | | EIVLTQSPGTLSL SPGERATLSC | RAS--QSVNS- ----NYLA | WYRQKPGQ APRLLIY | G-------- ASSRAT | GIPDRFSGSGSG-- TDFILIISRLEPEDFAVYYC | QQYES----------- ------------SPWT | FGQGT KVEIK |
| iPS:433 | 21- | VK3\|A27/ | | EIVLTQSPGTLSL | RAS--QSVSG- | WYQQKPGQ | G-------- | GIPDRFSGSGSG-- | QQYGS----------- | FGQGT |

| | | | | | | | | | | |
|---|---|---|---|---|---|---|---|---|---|---|
| iPS:434<br>307 | 21-<br>225_59B2 | VK3\|A27/<br>JK1 | | EIVLTQSPGTLSL<br>SPGERATLSC | WAS--QSVYS-<br>----SFLA | WFQQKSGQ<br>APRLLIY | G--------<br>ASSRAT | GIPDRFSGSGSG--<br>TDFTLTISRLEPEDFAVFYC | QQYGT-----------<br>------------SPWT | FGQGT<br>KVEIK |
| iPS:434<br>471 | 21-<br>225_75G3 | VK3\|A27/<br>JK1 | | EIVLTQSPGTLSL<br>SPGERATLSC | RAR--QNVDS-<br>----SYLA | WYQQKPGQ<br>APRLLIY | G--------<br>ASSRAT | GIPDRFSGSGSG--<br>TDFTLTISRLEPEDFAVFYC | QQYER-----------<br>------------SPWT | FGQGT<br>KVEIK |
| iPS:434<br>473 | 21-<br>225_76D1 | VK3\|A27/<br>JK1 | | EIVLTQSPGTLSL<br>SPGERATLSC | RAS--QNIYS-<br>----NYLA | WYQEKPGQ<br>APRLLIY | G--------<br>ASSRAT | GIPDRFSGSGSG--<br>TDFTLTISRLEPEDFVVYYC | QQYES-----------<br>------------SPWT | FGQGT<br>KVEIK |
| iPS:434<br>495 | 21-<br>225_74B2 | VK3\|A27/<br>JK1 | | ELVLTQSPGTLSL<br>SPGERATLSC | RAS--QNIYS-<br>----SYLA | WYQQKPGQ<br>APRLLIY | G--------<br>ASSRAT | GIPDRFSGSGSG--<br>TDFTLTISRLEPEDFAVYCC | QQYES-----------<br>------------SPWT | FGQGT<br>KVEIK |
| iPS:434<br>497 | 21-<br>225_76A4 | VK3\|A27/<br>JK1 | | EIVLTQSPGTLYL<br>SPGERATLSC | RAS--QSVYS-<br>----SYLA | WYQQKPGQ<br>APRLLIY | G--------<br>ASSRAT | GIPDRFSGSGSG--<br>TDFALTISRLEPEDFAVYYC | QHSDN-----------<br>------------SPWT | FGQGT<br>KVEIK |
| iPS:434<br>501 | 21-<br>225_76G4 | VK3\|A27/<br>JK1 | | EIVLTQSPGTLSL<br>SPGERATLSC | RAS--QSVYS-<br>----SYLA | WYQQKPGQ<br>APRLLIY | G--------<br>ASSRAT | GIPDRFSGSGSG--<br>TDFALTISRLEPEDCAVYYC | QHSDN-----------<br>------------SPWT | FGQGT<br>KVEIK |
| iPS:434<br>507 | 21-<br>225_74C5 | VK3\|A27/<br>JK1 | | EIVLTQSPGTLSL<br>SPGERATLSC | RAS--QSVNS-<br>----NYLA | WYQQKPGQ<br>APRLLIY | G--------<br>AFSRAT | GIPDRVSGSGSG--<br>TDFNLIISRLEPEDFAVYYC | QQYES-----------<br>------------SPWT | FGQGT<br>KVEIK |
| iPS:434<br>517 | 21-<br>225_76A7 | VK3\|A27/<br>JK1 | | EIALTQSPGTLSL<br>SPGERATLSC | RAS--PSVDS-<br>----SYLA | WYQQRPGQ<br>APRLLIY | G--------<br>ASSRAP | GIPDRFSGSGSG--<br>TDFTLTISRLEPEDFAVYYC | QQYES-----------<br>------------SPWT | FGQGT<br>KVEIK |
| iPS:434<br>519 | 21-<br>225_74C7 | VK3\|A27/<br>JK1 | | EIVLTQSPGTLSL<br>SPGERATLSC | RTS--PNVDS-<br>----SYLA | WYQQKPGQ<br>APRLLIY | G--------<br>ASSRAT | GIPDRFSGSGSG--<br>TDFTLTISRLEPEDFAVFYC | QQYER-----------<br>------------SPWT | FGQGT<br>KVEIK |
| iPS:434<br>523 | 21-<br>225_75C3 | VK3\|A27/<br>JK1 | | EIVLTQSPGTLSL<br>SQGERATLSC | RAS--QSVSS-<br>----RYLA | WYQQKPGQ<br>APRLLIY | G--------<br>ASSRAT | GIPDRFSGSGSG--<br>TDFTLTISRLEPEDFAVYYC | QHYDS-----------<br>------------SPWT | FGQGT<br>KVEIK |
| iPS:434<br>531 | 21-<br>225_76C9 | VK3\|A27/<br>JK1 | | EIVLTQSPGTLSL<br>SPGERATLSC | RAS--QSVSS-<br>----SYLS | WYQQKPGQ<br>APRLLIY | G--------<br>ASSRAT | GIPDRFSGSGSG--<br>TDFTLTISRLEPEDFAVYYC | QQYG------------<br>------------RSRT | FGQGT<br>KVEIR |
| iPS:434<br>533 | 21-<br>225_85F7 | VK3\|A27/<br>JK1 | | EPVLTQSPGTLSL<br>SPGERATLSC | RAS--QNIYS-<br>----NYLA | WYQQKPGQ<br>APRLLIY | G--------<br>ASSRAT | GIPDRFSGSGSG--<br>TDFTLTISRLEPEDFAVYCC | QQYES-----------<br>------------SPWT | FGQGT<br>KVEIK |
| iPS:434<br>547 | 21-<br>225_74H5 | VK3\|A27/<br>JK1 | | EIVLTQSPGTLSL<br>SPGERATLSC | RAS--QSVNS-<br>----NYLA | WYRQKPGQ<br>APRLLIY | G--------<br>ASSRAT | GIPDRFSGSGSG--<br>TDFILIISRLEPEDFAVYYC | QQYES-----------<br>------------SPWT | FGQGT<br>KVEIK |
| iPS:434<br>559 | 21-<br>225_74D11 | VK3\|A27/<br>JK1 | | EIVLTQSPGTLSL<br>SPGERATLSC | RAS--QSVYS-<br>----SYLA | WYQQKPGQ<br>APRLLIY | G--------<br>ASSRAT | GIPDRFSGSGSG--<br>TDFALTISRLEPEDFAVYYC | QHYDN-----------<br>------------SPWT | FGQGT<br>KVEIK |
| iPS:434<br>561 | 21-<br>225_77G1 | VK3\|A27/<br>JK1 | | EIVLTQSPGTLYL<br>SPGERATLSC | RAS--QSVYS-<br>----SYLA | WYQQKPGQ<br>APRLLIY | G--------<br>ASSRAT | GIPDRFSGSGSG--<br>TDFALTISRLEPEDCAVYYC | QHYDN-----------<br>------------SPWT | FGQGT<br>KVEIK |
| iPS:434<br>565 | 21-<br>225_75B10 | VK3\|A27/<br>JK1 | | EIVLTQSPGTLSL<br>SPGERATLSC | RAS--PSVNS-<br>----YYLA | WYQQKPGQ<br>APRLLIY | G--------<br>ATSRAT | GIPDRFSGSGSG--<br>TDFTLTISRLEPEDFAVYFC | QQYED-----------<br>------------SPWT | FGQGT<br>KVEIK |
| iPS:434<br>571 | 21-<br>225_74D2 | VK3\|A27/<br>JK1 | | EIVLTQSPGTLSL<br>SPGERATLSC | RAS--QSVDS-<br>----NYLA | WYQQKPGQ<br>APRLLIY | G--------<br>ASSRAP | GIPDRFSGSGSG--<br>TDFTLTINRLEPEDFAVYFC | QQYES-----------<br>------------SPWT | FGQGT<br>KVEIK |
| iPS:434<br>579 | 21-<br>225_77F7 | VK3\|A27/<br>JK1 | | EIVLTQSPGTLYL<br>SPGERATLSC | RAS--QSVYS-<br>----SYLA | WYQQKPGQ<br>APRLIIY | G--------<br>ASSRAT | GIPDRFSGSGCG--<br>TDFALTISRVEHEDCAVYYC | QHYDN-----------<br>------------SPWT | FGQGT<br>KVEIK |
| iPS:434<br>581 | 21-<br>225_74B12 | VK3\|A27/<br>JK1 | | EIVLTQSPGTLYL<br>SPGERATLSC | RAS--QSVYS-<br>----SYLA | WYQQKPGQ<br>APRLLIY | G--------<br>ASSRST | GIPDRFSGSGSG--<br>TDFALTISRLEPEDFAVYYC | QHYDN-----------<br>------------SPWT | FGQGT<br>KVEIK |
| iPS:434<br>585 | 21-<br>225_75A12 | VK3\|A27/<br>JK1 | | EIVLTQSPGTLSL<br>SPGERATLSC | RAS--QSVSS-<br>----RYLA | WYQQKPGQ<br>APRLLIY | G--------<br>ASSRAT | GIPDRFSGSGSG--<br>TDFTLTISRLEPADFAVYYC | QHYDS-----------<br>------------SPWT | FGQGT<br>KVEIK |
| iPS:434<br>595 | 21-<br>225_77A10 | VK3\|A27/<br>JK1 | | EIVLTQSPGTLSL<br>SPGERATLSC | RAS--QSVHS-<br>----RYLA | WYQQKPGQ<br>APKLLIF | G--------<br>ASSRAT | GIPDRFSGSGSG--<br>TDFTLTISRLEPEDFAVYYC | QHYDS-----------<br>------------SPWT | FGQGT<br>KVEIK |
| iPS:434<br>611 | 21-<br>225_77C12 | VK3\|A27/<br>JK1 | | EIVLTQSPGTLSL<br>SPGERATLSC | RAR--QSVDS-<br>----SYLA | WYQQKRGQ<br>APRLLIY | G--------<br>ASSRAT | GIPDRFSGSGSG--<br>TDFTLTISRLEPEDFAVYYC | QQYES-----------<br>------------SPWT | FGQGT<br>KVEIK |
| iPS:434<br>633 | 21-<br>225_74G8 | VK3\|A27/<br>JK1 | | EIVLTQSPGTLSL<br>SPGERATLSC | RAS--QSFSS-<br>----AYLA | WYQQKPGQ<br>APRLLIY | G--------<br>TSSRAT | GIPDRFSGSGSG--<br>TDFTLTIGRLEPEDFAVYYC | QQYG------------<br>------------NSRT | FGQGT<br>KVEIK |

| | | | | | | | | | | |
|---|---|---|---|---|---|---|---|---|---|---|
| **iPS:434 637** | **21-225_78E7** | **VK3\|A27/ JK1** | | EIVLTQSPGTLSL<br>SPGERATLSC | RAS--QNVDS-<br>----NYLA | WYQQKPGQ<br>APRLLIY | G--------<br>ASSRAT | GIPDRFSGSGSG--<br>TDFTLTISRLEPEDFAVFYC | QQYER-----------<br>------------SPWT | FGQGT<br>KVEIK |
| **iPS:434 657** | **21-225_79G1** | **VK3\|A27/ JK1** | | EIVLTQSPGTLYL<br>SSGERATLSC | RAS--QSVYS-<br>----SYLA | WYQQKPAQ<br>APRLLIY | G--------<br>ASSRST | GIPDRFSGSGSG--<br>TDFALTISRLEPEDFAVYYC | QHYDN-----------<br>------------SPWT | FGQGT<br>KVEIK |
| **iPS:434 663** | **21-225_79F3** | **VK3\|A27/ JK1** | | EIVLTQSPGTLYL<br>SPGERATLSC | RAS--QSVYS-<br>----SYLA | WYQQKPAQ<br>APRLLIY | G--------<br>ASSRST | GIPDRFSGSGSG--<br>TDFALTISRLEPEDFAVYYC | QHYDN-----------<br>------------SPWT | FGQGT<br>KVEIK |
| **iPS:434 671** | **21-225_74F4** | **VK3\|A27/ JK1** | | EIVLTQSPGTLSL<br>SPGERATLSC | RAS--QIFSS-<br>----SYLA | WYQQKPGQ<br>SPRLLIY | G--------<br>ASSRAT | GIPDRFSGSGSG--<br>TDFTLTISRLEPEDFAVYYC | QQYG------------<br>------------SSRT | FGQGT<br>KVEIK |
| **iPS:434 687** | **21-225_75A5** | **VK3\|A27/ JK1** | | EIVLTQSPGTLYL<br>SPGERATLSC | RAS--QSVYS-<br>----SYLA | WYQQKPGQ<br>APRLLIY | G--------<br>ASSRAT | GIPDRFSGSGSG--<br>TDFALTISRLEPEDFAVYYC | QHYDN-----------<br>------------SPWT | FGQGT<br>KVEIK |
| **iPS:434 691** | **21-225_75G7** | **VK3\|A27/ JK1** | | EIVLTQSPGTLSL<br>SPGERATLSC | RAR--QSVDS-<br>----SYLA | WYQQKRGQ<br>APRLLIY | G--------<br>ASSRAT | GIPDRFSGSGSG--<br>TDFTLTISRLEPEDFAVYYC | QQYES-----------<br>------------SPWT | FGQGT<br>KVEIK |
| **iPS:434 693** | **21-225_79F11** | **VK3\|A27/ JK1** | | EIVLTQSPGTLSL<br>SPGERATLSC | RAS--QSVYS-<br>----SYLA | WYQQKPGQ<br>APRLLIY | G--------<br>ASSRAT | GIPDRFSGSGSG--<br>TDFALTISRLEPEDFAVYYC | QHSDN-----------<br>------------SPWT | FGQGT<br>KVEIK |
| **iPS:434 699** | **21-225_79G12** | **VK3\|A27/ JK1** | | EIVLTQSPGTLYL<br>SPGERATLSC | RAS--QSVYS-<br>----SYLA | WYQQKPGQ<br>APRLLIY | G--------<br>ASSRST | GIPDRFSGSGSG--<br>TDFALTISRLEPEDCAVYYC | QHSDN-----------<br>------------SPWT | FGQGT<br>KVEIK |
| **iPS:434 701** | **21-225_80A1** | **VK3\|A27/ JK1** | | EIVLTQSPGTRYL<br>SPGERATLSC | RAS--QSVYS-<br>----SYLA | WYQQKPGQ<br>APRLLIY | G--------<br>ASSRST | GIPDRFSGSGCG--<br>TDFALTISRVEPEDCAVYYC | QHSDN-----------<br>------------SPWT | FGQGT<br>KVEIK |
| **iPS:434 703** | **21-225_80C1** | **VK3\|A27/ JK1** | | EIVLTQSPGTLYL<br>SPGERATLSC | RAS--QSVYS-<br>----SYLA | WYQQKPGQ<br>APRLLIY | G--------<br>ASSRST | GIPDRFSGSGSG--<br>TDFALTISRVEPEDCAVYYC | QHSDN-----------<br>------------SPWT | FGQGT<br>KVEIK |
| **iPS:434 709** | **21-225_80E3** | **VK3\|A27/ JK1** | | EIVLTQSPGTLYL<br>SSGERATLSC | RAS--QSVYS-<br>----SYLA | WYQQKPGQ<br>APRLLIY | G--------<br>ASSRST | GIPDRFSGSGCG--<br>TDFALTISRVEPEDFAVYYC | QHSDN-----------<br>------------SPWT | FGQGT<br>KVEIK |
| **iPS:434 715** | **21-225_80D5** | **VK3\|A27/ JK1** | | ELVLTQSPGTLSL<br>SPGKRVTLSC | RAS--QNIYS-<br>----SYLA | WYQQKPGQ<br>APRLLIY | G--------<br>ASSRAT | GIPDRFSGSGSG--<br>TDFTLTITRLEPEDFAVYCC | QQYES-----------<br>------------SPWT | FGQGT<br>KVEIK |
| **iPS:434 717** | **21-225_80A6** | **VK3\|A27/ JK1** | | EIVLTQSPGTLSL<br>SPGEIPTLSC | RAS--QSVDS-<br>----GYLA | WYQQKPGQ<br>APRLLIY | G--------<br>ASSRAP | GIPDRFSGSGSG--<br>TDFTLTISRLEPEDFAVYYC | QQYES-----------<br>------------SPWT | FGQGT<br>KVEIK |
| **iPS:434 725** | **21-225_80H7** | **VK3\|A27/ JK1** | | EIVLTQSPGTLSL<br>SPGERATLSC | RAS--QSINS-<br>----NYLA | WYQQKPGQ<br>APRLLIY | G--------<br>ASSRAT | GIPDRFSGSGSG--<br>TDFTLTISRLEPEDFAVFYC | QQYES-----------<br>------------SPWT | FGQGT<br>KVEIK |
| **iPS:434 735** | **21-225_80B10** | **VK3\|A27/ JK1** | | EIVLTQSPGTLSL<br>SPGERATLSC | RAS--QSVDS-<br>----SYLA | WYQQKPGQ<br>APRLLIY | G--------<br>ASSRAP | GIPDRFSGSGSG--<br>TDFTLTISRLEPEDFAVYYC | QQYES-----------<br>------------SPWT | FGQGT<br>KVEIK |
| **iPS:434 743** | **21-225_74A4** | **VK3\|A27/ JK1** | | EIVLTQSPGTLYL<br>SPGERATLSC | RAS--QSVYS-<br>----SYLA | WYQQKPGQ<br>APRLLIY | G--------<br>ASSRST | GIPDRFSGSGCG--<br>TDFALTISRLEPEDCAVYYC | QHSDN-----------<br>------------SPWT | FGQGT<br>KVEIK |
| **iPS:434 751** | **21-225_80H12** | **VK3\|A27/ JK1** | | EIVLTQSPGTLSL<br>SPGERATLSC | RAS--QSVYS-<br>----SYLA | WYQQKRGQ<br>APRLLIY | G--------<br>ASSRST | GIPDRFSGSGSG--<br>TDFALTISRLEPEDFAVYYC | QHSDN-----------<br>------------SPWT | FGQGT<br>KVEIK |
| **iPS:434 759** | **21-225_81C5** | **VK3\|A27/ JK1** | | EIVLTQSPGTLYL<br>SSGERATLSC | RAS--QSVYS-<br>----SYLA | WYQQKPGQ<br>APRLLIY | G--------<br>ASSRST | GIPDRFSGSGCG--<br>TDFALTISRVEPEDCAVYYC | QHSDN-----------<br>------------SPWT | FGQGT<br>KVEIK |
| **iPS:434 773** | **21-225_75D9** | **VK3\|A27/ JK1** | | EIVLTQSPGTLSL<br>SPGERATLSC | RAS--QSVSS-<br>----SYLA | WYQQKRGQ<br>APRLLIY | G--------<br>ASSRAT | GIPDRFSGSGSG--<br>TDFTLTISRLEPEDFAVYYC | QQYES-----------<br>------------SPWT | FGQGT<br>KVEIK |
| **iPS:434 777** | **21-225_81C11** | **VK3\|A27/ JK1** | | EIVLTQSPGTRYL<br>SPVERATLSC | RAS--QSVYS-<br>----SYLA | WYQQKPGQ<br>APRLLIY | G--------<br>ASSRST | GLPDRFSGSGCG--<br>TDFALTISRVEPEDCAVYYC | QHSDN-----------<br>------------SPWT | FGQGT<br>KVEIK |
| **iPS:434 809** | **21-225_74F5** | **VK3\|A27/ JK1** | | EIVLTQSPGTLYL<br>SPGERATLSC | RAS--QSVYS-<br>----SYLA | WYQQKPGQ<br>APRLLIY | G--------<br>ASSRST | GIPDRFSGSGSG--<br>TDFALTISRLEPEDFAVYYC | QHSDN-----------<br>------------SPWT | FGQGT<br>KVEIK |
| **iPS:434 821** | **21-225_83G1** | **VK3\|A27/ JK1** | | EIVLTQSPGTRYL<br>SPGERATLSC | RAS--QSVYS-<br>----SYLA | WYQQKPGQ<br>APRLLIY | G--------<br>ASSRST | VLPDRFSGSGSG--<br>TDFALTISRVEPEDCAVYYC | QHSDN-----------<br>------------SPWT | FGQGT<br>KVEIK |
| **iPS:434 835** | **21-225_83B6** | **VK3\|A27/ JK1** | | EIVLTQSPGTLSL<br>SPGERATLSC | RAS--QSVDS-<br>----GYLA | WYQQKPGQ<br>APRLLIY | G--------<br>ASSRTP | GIPDRFSGSGSG--<br>TDFTLTISRLEPEDFAVYYC | QQYES-----------<br>------------SPWT | FGQGT<br>KVEIK |

| | | | | | | | | | | |
|---|---|---|---|---|---|---|---|---|---|---|
| iPS:434 839 | 21- 225_83B7 | VK3\|A27/ JK1 | | EIVLTQSPGTRYL SSVERATLSC | RAS--QSVYS- ----SYLA | WYQQKPGQ APRLLIY | G-------- ASSRST | GIPDRFSGSGSG-- TDFALTISRLEPEDCAVYYC | QHSDN----------- ------------SPWT | FGQGT KVEIK |
| iPS:434 849 | 21- 225_83C10 | VK3\|A27/ JK1 | | EIVLTQSPGTLSL SPGERATLSC | RAS--PSVHS- ----NYLA | WYQQKPGQ APRLLIY | G-------- ASSRAT | GIPDRFSGSGSG-- TDFILTISRLEPEDFAVYYC | QQYES----------- ------------SPWT | FGQGT KVEIK |
| iPS:434 869 | 21- 225_84E12 | VK3\|A27/ JK1 | | EIVLTQSPGTLSL SPGERATLSC | RAS--QSINS- ----NYLA | WYQQKPGQ APRLLIY | G-------- ASSRAT | GIPDRFSGSGSG-- TDFTLTISRLEPDDFAVFYC | QQYES----------- ------------SPWT | FGQGT KVEIK |
| iPS:434 879 | 21- 225_85A3 | VK3\|A27/ JK1 | | EIVLTQSPGTLFL SPGERATLSC | RAS--QSVYS- ----SYLA | WYQQKPAQ APRLLIY | G-------- ASSRAS | GIPDRFSGSGSG-- TDFALTISRLEPEDFAVYYC | QHYDN----------- ------------SPWT | FGQGT KVEIK |
| iPS:434 881 | 21- 225_85B4 | VK3\|A27/ JK1 | | EIVLTQSPGTLFL SPGERATLSC | RAS--QSVYS- ----SYLA | WYQQKPGQ APRLLIY | G-------- ASSRAT | GIPDRFSGSGSG-- TDFALTISRLEPEDFAVYYC | QHYDN----------- ------------SPWT | FGQGT KVEIK |
| iPS:434 887 | 21- 225_85D6 | VK3\|A27/ JK1 | | EIVLTQSPGTLFL SQGERATLSC | RAS--QSVSS- ----RYLA | WYQQKPGQ APRLLIY | G-------- ASSRAT | GIPDRFSGSGSG-- TDFTLTISRLEPEDFAVYYC | QHYDS----------- ------------SPWT | FGQGT KVEIK |
| iPS:434 891 | 21- 225_85G6 | VK3\|A27/ JK1 | | EIALTQSPGTLSL SPGERATLSC | RAS--PSVDS- ----SYLA | WYQQKPGQ APRLLIY | G-------- AASRAP | GIPDRFSGSGSG-- TDFTLTISRLEPEDFVVYYC | QQYES----------- ------------SPWT | FGQGT KVEIK |
| iPS:434 895 | 21- 225_74H7 | VK3\|A27/ JK1 | | ELVLTQSPGTLSL SPGERATLSC | RAS--QNIYS- ----SYLA | WYQQKPGQ APRLLIY | G-------- ASSRAT | GIPDRFSGSGSG-- TDFTLTISRLEPEDFAVYCC | QQYES----------- ------------SPWT | FGQGT KVEIK |
| iPS:434 899 | 21- 225_85B9 | VK3\|A27/ JK1 | | EIVLTQSPGTLFL SPGERATLSC | RAS--QSVNS- ----NYLA | WYRQKPGQ APRLLIY | G-------- ASSRAT | GIPDRFSGSGSG-- TDFILIISRLEPEDFAVYYC | QQYES----------- ------------SPWT | FGQGT KVEIK |
| iPS:434 907 | 21- 225_85G10 | VK3\|A27/ JK1 | | EIVLTQSPGSLSL SPGERATLSC | RAS--QSVWS- ----GYLA | WYQQKPGQ APRLLIY | G-------- ASSRAT | GIPDRFSGSGSG-- TDFTLTISRLEPEDFAVYFC | QQYES----------- ------------SPWT | FGQGT KVEIK |
| iPS:434 913 | 21- 225_86C1 | VK3\|A27/ JK1 | | EIVLTQSPGTLYL SPGERATLSC | RAS--QSVYS- ----SYLA | WYQQKPAQ APRLLIY | G-------- ASSRAT | GIPDRFSGSGSG-- TDFALTISRLEPEDCAVYYC | QHYDN----------- ------------SPWT | FGQGT KVEIK |
| iPS:434 921 | 21- 225_86E4 | VK3\|A27/ JK1 | | EIVLTQSPGTRYL SPGERATLSC | RAS--QSVYS- ----SYLA | WYQQKPGQ APRLLIY | G-------- ASSRST | GIPDRFSGSGSG-- TDFALTISRLEPEDCAVYYC | QHSDN----------- ------------SPWT | FGQGT KVEIK |
| iPS:434 939 | 21- 225_86C11 | VK3\|A27/ JK1 | | EIVLTQSPGTLYL SPGERATLSC | RAS--QSVYS- ----SYLA | WYQQKPGQ APRLLIY | G-------- ASSRST | GLPDRFSGSGSG-- TDFALTISRLEPEDCAVYYC | QHSDN----------- ------------SPWT | FGQGT KVEIK |
| iPS:434 943 | 21- 225_87H1 | VK3\|A27/ JK1 | | EIVLTQSPGTLSL SPGERATLSC | RAS--QSVDS- ----NYLA | WYQQKPGQ APRLLIY | G-------- ASARTT | GIPDRFSGSGSG-- TDFTLTISRLEPEDFAVYSC | QQYES----------- ------------SPWT | FGQGT KVEIK |
| iPS:434 945 | 21- 225_87E5 | VK3\|A27/ JK1 | | EFVLTQSPGTLYL SPGERATLSC | RAS--QSVYS- ----SYLA | WYQQKPGQ APRLLIY | G-------- ASSRST | GIPDRFSGSGSG-- TDFALTISRVEPEDCAVYYC | QHSDN----------- ------------SPWT | FGQGT KVEIK |
| iPS:434 955 | 21- 225_87C9 | VK3\|A27/ JK1 | | EIVLTQSPGTLYL SPVERATLSC | RAS--QSVYS- ----SYLA | WYQQKPGQ APRLLIY | G-------- ASSRST | GIPDRFSGSGSG-- TDFALTISRLEPEDCAVYYC | QHSDN----------- ------------SPWT | FGQGT KVEIK |
| iPS:434 961 | 21- 225_87A12 | VK3\|A27/ JK1 | | EIVLTQSPGTRYL SPGERATLSC | RAS--QSVYS- ----SYLA | WYQQKPGQ APRLLIY | G-------- ASSRST | VIPDRFSGSGCG-- TDFALTISRVEPEDCAVYYC | QHSDN----------- ------------SPWT | FGQGT KVEIK |
| iPS:434 969 | 21- 225_88H1 | VK3\|A27/ JK1 | | EIVLTQSPGTLYL SPGERATLSC | RAS--QSVYS- ----SYLA | WYQQKPGQ APRLLIY | G-------- ASSRST | VIPDRFSGSGCG-- TDFALTISRVEPEDCAVYYC | QHSDN----------- ------------SPWT | FGQGT KVEIK |
| iPS:434 981 | 21- 225_88E7 | VK3\|A27/ JK1 | | EIVLTQSPGTLSL SPGERATLSC | RAS--QSVYS- ----SYLA | WYQQKPGQ APRLLIY | G-------- ASSRST | GIPDRFSGSGSG-- TDFALTISRVEPEDCAVYYC | QHSDN----------- ------------SPWT | FGQGT KVEIK |
| iPS:434 983 | 21- 225_88F7 | VK3\|A27/ JK1 | | EIVLTQSPGTLSL SPGERATLSC | RAS--QSVYS- ----SYLA | WYQQKPGQ APRLLIY | G-------- ASSRST | GIPDRFSGSGSG-- TDFALTISRLEPEDCAVYYC | QHSDN----------- ------------SPWT | FGQGT KVEIK |
| iPS:434 995 | 21- 225_88G9 | VK3\|A27/ JK1 | | EIVLTQSPGTRYL SPGERATLSC | RAS--QSVYS- ----SYLA | WYQQKPGQ APRLLIY | G-------- ASSRST | GIPDRFSGSGSG-- TDFALTISRVEPEDCAVYYC | QHSDN----------- ------------SPWT | FGQGT KVEIK |
| iPS:434 999 | 21- 225_75A8 | VK3\|A27/ JK1 | | EIVLTQSPGTLYL SPGERATLSC | RAS--QSVYS- ----SYLA | WYQQKPGQ APRLLIY | G-------- ASSRAT | GIPDRFSGSGSG-- TDFALTISRVEPEDCAVYYC | QHSDN----------- ------------SPWT | FGQGT KVEIK |
| iPS:435 013 | 21- 225_89D5 | VK3\|A27/ JK1 | | EIVLTQSPGTLYL SSGERATLSC | RAS--QSVYS- ----SYLA | WYQQKPGQ APRLLIY | G-------- ASSRST | GIPDRFSGSGSG-- TDFALTISRLEPEDFAVYYC | QHYDN----------- ------------SPWT | FGQGT KVEIK |

| | | | | | | | | | | |
|---|---|---|---|---|---|---|---|---|---|---|
| iPS:435 015 | 21- 225_89H5 | VK3\|A27/ JK1 | | EIVLTQSPGTLSL SPGERATLSC | RAS--QSVNS- ----NYLA | WYQQKPGQ APRLLIY | G-------- AFSRAT | GIPDRVSGSGSG-- TDFNLIISRLEPEDFAVYYC | QQYES----------- ------------SVWT | FGQGT KVEIK |
| iPS:435 025 | 21- 225_89E10 | VK3\|A27/ JK1 | | EIVLTQSPGTLYL SSGERATLSC | RAS--QSVYS- ----SYLA | WYQQKPGQ APRLLIY | G-------- ASSRST | VIPDRFSGSGSG-- TDFALTISRVEPEDFAVYYC | QHSDN----------- ------------SPWT | FGQGT KVEIK |
| iPS:435 029 | 21- 225_89A11 | VK3\|A27/ JK1 | | EIVLTQSPGTLSL SPGERATLSC | RAS--QSVDS- ----NFLA | WYQQKPGQ APRLLIY | G-------- ASARTT | GIPDRFSGSGSG-- TDFTLTISRLEPEDFAVYSC | QQYEI----------- ------------SPWT | FGQGT KVEIK |
| iPS:435 039 | 21- 225_90G4 | VK3\|A27/ JK1 | | EIVLTQSPGTLYL SPGERATLSC | RAS--QSVYS- ----SYLA | WYQQKPGQ APRLLIY | G-------- ASSRST | VIPDRFSGSGSG-- TDFALTISRVEPEDCAVYYC | QHSDN----------- ------------SPWT | FGQGT KVEIK |
| iPS:435 041 | 21- 225_90A5 | VK3\|A27/ JK1 | | EIVLTQSPGTLYL SPGERATLSC | RAS--QSVYS- ----SYLA | WYQQKPGQ APRLLIY | G-------- ASSRAT | GIPDRFSGSGSG-- TDFALTISRVEHEDFAVYYC | QHYDN----------- ------------SPWT | FGQGT KVEIK |
| iPS:435 043 | 21- 225_90G5 | VK3\|A27/ JK1 | | EIVLTQSPGTLYL SPGERATLSC | RAS--QSVYS- ----SYLA | WYQQKPGQ APRLIIY | G-------- ASSRAT | GIPDRFSGSGSG-- TDFALTISRVEHEDFAVYYC | QHYDN----------- ------------SPWT | FGQGT KVEIK |
| iPS:435 055 | 21- 225_90F10 | VK3\|A27/ JK1 | | EIVLTQSPGTLYL SPGERATLSC | RAS--QSVYS- ----SYLA | WYQQKPGQ APRLLIY | G-------- ASSRST | GIPDRFSGSGSG-- TDFALTISRLEPEDCAVYYC | QHYDN----------- ------------SPWT | FGQGT KVEIK |
| iPS:435 073 | 21- 225_91B2 | VK3\|A27/ JK1 | | EIVLTQSPGTLYL SPGERATLSC | RAS--QSVYS- ----SYLA | WYQQKPGQ APRLLIY | G-------- ASSRST | GIPDRFSGSGSG-- TDFALTISRVEPEDCAVYYC | QHYDN----------- ------------SPWT | FGQGT KVEIK |
| iPS:435 075 | 21- 225_91B3 | VK3\|A27/ JK1 | | EIVLTQSPGTLYL SPGERATLSC | RAS--QSVYS- ----SYLA | WYQQKPGQ APRLLIY | G-------- ASSRST | GIPDRFSGSGSG-- TDFALTISRVEHEDCAVYYC | QHYDN----------- ------------SPWT | FGQGT KVEIK |
| iPS:435 077 | 21- 225_91F3 | VK3\|A27/ JK1 | | EIVLTQSPGTLYL SPGERATLSC | RAS--QSVYS- ----SYLA | WYQQKPAQ APRLLIY | G-------- ASSRST | GIPDRFSGSGSG-- TDFALTISRVEPEDCAVYYC | QHSDN----------- ------------SPWT | FGQGT KVEIK |
| iPS:435 079 | 21- 225_91B4 | VK3\|A27/ JK1 | | EIVLTQSPGTLYL SPGERATLSC | RAS--QSVYS- ----SYLA | WYQQKPGQ APRLIIY | G-------- ASSRST | GIPDRFSGSGSG-- TDFALTISRVEPEDCAVYYC | QHYDN----------- ------------SPWT | FGQGT KVEIK |
| iPS:435 089 | 21- 225_91E9 | VK3\|A27/ JK1 | | EIVLTQSPGTLYL SPGERATLSC | RAS--QSVYS- ----SYLA | WYQQKPGQ APRLLIY | G-------- ASSRAT | GIPDRFSGSGCG-- TDFALTISRVEHEDCAVYYC | QHSDN----------- ------------SPWT | FGQGT KVEIK |
| iPS:435 097 | 21- 225_92B1 | VK3\|A27/ JK1 | | EIVLTQSPGTLSL SPGERATLSC | RAS--QSVGS- ----NYLA | WYQQKRGQ APRLLIY | G-------- ASSRAT | DIPDRFSGSGSG-- TDFTLTISRLEPEDFAVYYC | QQYES----------- ------------SPWT | FGQGT KVEIK |
| iPS:435 111 | 21- 225_92D6 | VK3\|A27/ JK1 | | EIVLTQSPGTLYL SPGERATLSC | RAS--QSVYS- ----SYLA | WYQQKPGQ APRLLIY | G-------- ASSRST | VIPDRFSGSGCG-- TDFALTISRLEPEDCAVYYC | QHYDN----------- ------------SPWT | FGQGT KVEIK |
| iPS:435 115 | 21- 225_77C5 | VK3\|A27/ JK1 | | EIVLTQSPGTLSL SPGERATLSC | RAS--QSVYS- ----SYLA | WYQQKPGQ APRLLIY | G-------- ASSRST | GIPDRFSGSGSG-- TDFALTISRLEPEDFAVYYC | QHYDN----------- ------------SPWT | FGQGT KVEIK |
| iPS:435 171 | 21- 225_93C2 | VK3\|A27/ JK1 | | EIVLTQSPGTLYL SPGERATLSC | RAS--QSVYS- ----SYLA | WYQQKPGQ APRLLIY | G-------- ASSRAT | GIPDRFSGSGSG-- TDFALTISRLEHEDCAVYYC | QHYDN----------- ------------SPWT | FGQGT KVEIK |
| iPS:435 177 | 21- 225_93E4 | VK3\|A27/ JK1 | | EIVLTQSPGTLYL SPGERATLSC | RAS--QSVYS- ----SYLA | WYQQKPGQ APRLLIY | G-------- ASSRST | GIPDRFSGSGCG-- TDFALTISRVEPEDFAVYYC | QHSDN----------- ------------SPWT | FGQGT KVEIK |
| iPS:435 183 | 21- 225_93E9 | VK3\|A27/ JK1 | | EIVLTQSPGTLSL SPGERATLSC | RAS--QSVDS- ----SYLA | WYQQKTGQ APRLLIY | G-------- ASSRAP | GIPDRFSGSGSG-- TDFTLTISRLEPEDFAVYYC | QQYES----------- ------------SPWT | FGQGT KVEIK |
| iPS:435 195 | 21- 225_94D3 | VK3\|A27/ JK1 | | EIVLTQSPGTLSL SQGERATLSC | RAS--QSVSS- ----RYLA | WYQQKPGQ APRLLIY | G-------- ASSRAT | GIPDRFSGSGSG-- TDFTLTISRLEPEDFAVYYC | QHYDS----------- ------------SPWT | FGQGT KVENQ |
| iPS:435 217 | 21- 225_94F12 | VK3\|A27/ JK1 | | EIVLTQSPGTLYL SPGERATFSC | RAS--QSVYS- ----SYLA | WYQQKPGQ APRLLIY | G-------- ASSRST | GIPDRFSGSGSG-- TDFALTISRLEPEDFAVYYC | QHYDN----------- ------------SPWT | FGQGT KVEIK |
| iPS:435 219 | 21- 225_95D2 | VK3\|A27/ JK1 | | EIVLTQSPGTLSL SPGERATLSC | RAS--QSVYS- ----SYLA | WYQQKPGQ APRLLIY | G-------- ASSRAT | GIPDRFSGSGSG-- TDFALTISRLEPEDCAVYYC | QHYDN----------- ------------SPWT | FGQGT KVEIK |
| iPS:435 235 | 21- 225_95F9 | VK3\|A27/ JK1 | | EIVLTQSPGTLYL SPGERATLSC | RAS--QSVYS- ----SYLA | WYQQKPGQ APRLLIY | G-------- ASSRST | GIPDRFSGSGCG-- TDFALTISRLEPEDFAVYYC | QHYDN----------- ------------SPWT | FGQGT KVEIK |
| iPS:435 237 | 21- 225_95G9 | VK3\|A27/ JK1 | | EIVLTQSPGTLYL SSGERATLSC | RAS--QSVYS- ----SYLA | WYQQKPGQ APRLLIY | G-------- ASSRST | GIPDRFSGSGSG-- TDFALTISRVEHEDCAVYYC | QHYDN----------- ------------SPWT | FGQGT KVEIK |

| | | | | | | | | | | |
|---|---|---|---|---|---|---|---|---|---|---|
| **iPS:435 239** | **21-225_95H10** | **VK3\|A27/ JK1** | | EIVLSQSPGILYL<br>SSGERATLSC | RAS--QSVYS-<br>----SYLA | WYQQKPGQ<br>APRLLIY | G--------<br>ASSRAT | GIPDRFSGSGSG--<br>TDFALTISRLEPEDFAVYYC | QHYDN-----------<br>------------SPWT | FGQGT<br>KVEIK |
| **iPS:435 273** | **21-225_97A2** | **VK3\|A27/ JK1** | | EIVLTQSPGTLYL<br>SSGERATLSC | RAS--QSVYS-<br>----SYLA | WYQQKPGQ<br>APRLLIY | G--------<br>ASSRST | GIPDRFSGSGCG--<br>TDFALTISRLEPEDCAVYYC | QHSDN-----------<br>------------SPWT | FGQGT<br>KVEIK |
| **iPS:435 281** | **21-225_97E5** | **VK3\|A27/ JK1** | | EIVLTQSPGTLSL<br>SPGERATLSC | RAS--QSVYS-<br>----SYLA | WYQQKPGQ<br>APRLLIY | G--------<br>ASSRAT | GIPDRFSGSGCG--<br>TDFALTISRVEPEDCAVYYC | QHSDN-----------<br>------------SPWT | FGQGT<br>KVEIK |
| **iPS:435 331** | **21-225_147G8** | **VK3\|A27/ JK1** | | QIVLTQSPGTLSL<br>SPGERATLSC | RAS--QRIFS-<br>----NYLA | WYQQKPGQ<br>APRILIY | G--------<br>ASSRAT | GIPDRISGSGSG--<br>TDFTLTITRLEPEDFAVYYC | QQYDS-----------<br>------------SPWT | FGQGT<br>KVEIN |
| **iPS:435 815** | **21-225_190G10** | **VK3\|A27/ JK1** | | EIVLTQSPGTLSL<br>SPGERATLSC | RAS--PSVSS-<br>----RFLA | WYQQKPGQ<br>APRLLIY | G--------<br>ASSRAT | GIPDRFSGSGSG--<br>TDFTLTNSRLEPEDFAVYYC | QQYGS-----------<br>-----------SPPWT | FGQGT<br>KVEIK |
| **iPS:435 843** | **21-225_191F1** | **VK3\|A27/ JK1** | | EIVLTQSPGTLSL<br>SPGERAALSC | RAS--QSISL-<br>----NFLA | WYQQKPGQ<br>APRLLIY | G--------<br>ASSRAT | GIPDRFSGSGSG--<br>TDFTLTINRLEPEDFAVYYC | QQYGR-----------<br>------------SPWT | FGQGT<br>KVEVK |
| **iPS:435 847** | **21-225_191A3** | **VK3\|A27/ JK1** | | EIVLTQSPGTLSL<br>SPGERATLSC | RAS--QSIRS-<br>----SFLA | WYQQKPGQ<br>APRLLIY | G--------<br>ASSRAT | GIPDRFSGSGSG--<br>TDFTLTISRLEPEDFAVYYC | QQYGN-----------<br>------------SPWA | FGQGT<br>KVEVK |
| **iPS:435 849** | **21-225_191C3** | **VK3\|A27/ JK1** | | EIVLTQSPGTLSL<br>SPGERATLSC | RAS--QSIRS-<br>----SFLA | WYQQKPGQ<br>APRLLIY | G--------<br>ASSRAT | GIPDRFSGSGSG--<br>TDFTLTISRLEPEDFAVYYC | QQYGN-----------<br>------------SPWA | FGQGT<br>KVEIK |
| **iPS:435 851** | **21-225_191D3** | **VK3\|A27/ JK1** | | EIVLTQSPGTLSL<br>SPGESATLSC | RAG--QSIRT-<br>----NFLA | WYQQQPGQ<br>APRLLIY | G--------<br>ASSRAT | GIPDRFSGSGSG--<br>TDFTLTINRLEPEDFAVYYC | QQYGS-----------<br>------------SPWT | FGQGT<br>KVEIK |
| **iPS:435 865** | **21-225_191A5** | **VK3\|A27/ JK1** | | EIVLTQSPGTLSL<br>SPGERATLSC | RAS--QSVSS-<br>----RFLA | WYQQKPGQ<br>APRLLIY | G--------<br>ASNRAT | GIPDRFSGSGSG--<br>TDFTLTISRLEPEDFAVYYC | QQYGG-----------<br>-----------SPPWT | FVQGT<br>KVEIK |
| **iPS:435 905** | **21-225_190A3** | **VK3\|A27/ JK1** | | EIMLTQSPGTLSL<br>SPGERATLSC | RAS--QNIRS-<br>----NFLA | WYQQKPGQ<br>APRLLIY | G--------<br>ASSRAT | GIPDRFSGSVSG--<br>TDFTLTINRLEPEDFAVYYC | QQYGN-----------<br>------------SPWT | FGQGT<br>KVEIK |
| **iPS:435 911** | **21-225_190B4** | **VK3\|A27/ JK1** | | EIVLTQSPGTLSL<br>SPGERATLSC | RAS--QSIRS-<br>----SFLA | WYQQKPGQ<br>APRLLIY | G--------<br>ASSRAT | GIPDRFSGSGSG--<br>TDFTLTISRLEPEDFAVYYC | QQYGN-----------<br>------------SPWA | FGQGT<br>KVEIK |
| **iPS:435 913** | **21-225_190A7** | **VK3\|A27/ JK1** | | EIVLTQSPGTLSL<br>SPGERATLSC | RAS--QSVRS-<br>----NFLA | WHQQKPGQ<br>APRLLIY | G--------<br>AYRRAT | GIPDRFSGSGSG--<br>TDFTLTINRLEPEDFAVYYC | QQYGN-----------<br>------------SPWT | FGQGT<br>KVEIK |
| **iPS:435 939** | **21-225_191H7** | **VK3\|A27/ JK1** | | EIVLTQSPGTLSL<br>SPGESATLSC | RAG--QSIRT-<br>----DFLA | WYQQQPGQ<br>APRLLIY | G--------<br>PSSRAT | GIPDRFSGSGSG--<br>TDFTLTINRLEPEDFAVYFC | QQYGS-----------<br>------------SPWT | FGQGT<br>KVEIN |
| **iPS:435 967** | **21-225_192B3** | **VK3\|A27/ JK1** | | EIVLTQSPGTLSL<br>SPGERATLSC | RAS--QSVRS-<br>----SFLA | WYQQKPGQ<br>APRLLIY | G--------<br>ASSRAT | GIPDRFSGSGSG--<br>TDFTLTISRLEPEDFAVYYC | QQYGN-----------<br>------------SPWA | FGQGT<br>KVEIK |
| **iPS:435 973** | **21-225_192H3** | **VK3\|A27/ JK1** | | EIVLTQSPGTLSL<br>SPGERATLSC | RAS--QSVSS-<br>----NFLA | WYQQKPGQ<br>APRLLIF | G--------<br>ASSRAT | GIPDRFSGSGSG--<br>TDFTLTINRLEPEDFAVYYC | QQYGI-----------<br>------------SPWT | FGQGT<br>KVEIK |
| **iPS:435 995** | **21-225_192F8** | **VK3\|A27/ JK1** | | EIVLTQSPGTLSL<br>SPGERATLSC | RAS--QSISS-<br>----SYLA | WYQQKPGQ<br>APRLLIY | G--------<br>ASSRAT | GIPARFSGSGSG--<br>TDFTLTISRLEPEDFAVYYC | QQYES-----------<br>------------SPWT | FGQGT<br>KVEIK |
| **iPS:436 007** | **21-225_192G12** | **VK3\|A27/ JK1** | | EIVLTQSPGTLSL<br>SPGERATLSC | RAS--QSVRS-<br>----DFLA | WLQQKPGQ<br>APRLLIY | G--------<br>VSRRAT | GIPDRFSGSGSG--<br>TDFTLTINRLEPEDFAVYYC | QQYGN-----------<br>------------SPWT | FGQGT<br>KVEIK |
| **iPS:436 009** | **21-225_193A1** | **VK3\|A27/ JK1** | | EIVLTQSPGTLSL<br>SPGERATLSC | RAS--QSVRS-<br>----NFLA | WHQQKPGQ<br>APRLFIY | G--------<br>ASRRAT | GIPDRFSGSGSG--<br>TDFTLTINRLEPEDFAVYYC | QQYGN-----------<br>------------SPWT | FGQGT<br>KVEIK |
| **iPS:436 011** | **21-225_193B1** | **VK3\|A27/ JK1** | | EIVLTQSPGTLSL<br>SPGDRATLSC | RAS--QSVRS-<br>----NFLA | WHQQKPGQ<br>APRLLIY | G--------<br>ASRRAT | GIPDRFSGSGSG--<br>TDFTLTINRLEPEDFAVYFC | QQYGN-----------<br>------------SPWT | FGQGT<br>KVEIK |
| **iPS:436 015** | **21-225_193D3** | **VK3\|A27/ JK1** | | EIVLTQSPGTLSL<br>SPGERATLSC | RAS--QSIRS-<br>----SFLA | WYQQKPGQ<br>APRLLIY | G--------<br>ASNRAT | GIPDRFSGSGSG--<br>TDFTLTISRLEPEDFAVYYC | QQYGN-----------<br>------------SPWA | FGQGT<br>KVEIK |
| **iPS:436 017** | **21-225_193F3** | **VK3\|A27/ JK1** | | EIVLTQSPGTLSL<br>SPGESATLSC | RAG--QSIRT-<br>----DFLV | WYQQQPGQ<br>APRLLIY | G--------<br>ASSRAT | GFPERFSGSGSG--<br>TDFTLTINRLEPEDFAVYYC | QQYGS-----------<br>------------SPWT | FGQGT<br>KVEIN |

| | | | | | | | | | | |
|---|---|---|---|---|---|---|---|---|---|---|
| iPS:436 027 | 21-225_193E6 | VK3\|A27/ JK1 | | EIVLAQSPGTLSL<br>SPGERATLSC | RAS--QSVRS-<br>----GYLA | WYQQKPGQ<br>APRLLIY | G--------<br>ASSRAT | GIPDRFGGSGSG--<br>TDFTLTISRLEPEDFAVYYC | QQYES-----------<br>------------SPWT | FGQGT<br>KVEIK |
| iPS:436 029 | 21-225_193H6 | VK3\|A27/ JK1 | | EIVLTQSPGTLSL<br>SPGESATLSC | RAG--QSIRT-<br>----NFLA | WYQQQPGQ<br>APRLLIY | G--------<br>ASSRAT | GIPDRFSGSGSG--<br>TDFTLTINRLEPEDFAVYFC | QQYGS-----------<br>------------SPWT | FGQGT<br>KVEIN |
| iPS:436 035 | 21-225_193C8 | VK3\|A27/ JK1 | | EIVLTQSPGTLFL<br>SPGERATLSC | RAS--QSIRS-<br>----SFLA | WYQQKPGQ<br>APRLLIY | G--------<br>ASSRAT | GIPDRFSGSGSG--<br>TDFTLTISRLEPEDFAVYYC | QQYGN-----------<br>------------SPWA | FGQGI<br>KVEVK |
| iPS:436 037 | 21-225_193D8 | VK3\|A27/ JK1 | | EIVLKQSPGTLFL<br>SPGERATLSC | RAS--QSIRT-<br>----NFLA | WYQQKPGQ<br>APRLLIY | G--------<br>ASSRAT | GIPDRFSGSGSG--<br>TDFTLTINRLEPEDFAVYYC | QQYGN-----------<br>------------SPWT | FGQGT<br>KVEIK |
| iPS:436 041 | 21-225_193G8 | VK3\|A27/ JK1 | | EIVLTQSPGTLSL<br>SPGERATLSC | RAS--QSVRT-<br>----NFLA | WHQQKPGQ<br>APRLLIY | G--------<br>ASRRAT | GIPDRFSGSGSG--<br>TDFTLTINRLEPEDFALYYC | QQYGN-----------<br>------------SPWT | FGQGT<br>KVEIK |
| iPS:436 047 | 21-225_193B10 | VK3\|A27/ JK1 | | EIVLTQSPGTLSL<br>SPGERATLSC | RAS--PSVSS-<br>----SYLA | WYQQKPGQ<br>APRLVIY | G--------<br>ASRRAT | GIPDRFRGSGSG--<br>TDFTLTISRLEPEDFAVYYC | QQYGS-----------<br>-----------SPPWT | FGQGT<br>KVEIK |
| iPS:436 049 | 21-225_193B12 | VK3\|A27/ JK1 | | EIVLTQSPGTLSL<br>SPGERATLSC | RAS--QSIRS-<br>----SFLA | WYQQKPGQ<br>APRLLIY | D--------<br>ASSRAT | GIPDRFSGSGSG--<br>TDFTLTISRLEPEDFAVYYC | QQYGN-----------<br>------------SPWA | FGQGT<br>KVEIK |
| iPS:436 062 | 21-225_194E5 | VK3\|A27/ JK1 | | EIVLTQSPGTLSL<br>SPGESATLSC | RAG--QSIRT-<br>----NFLA | WYQQKPGQ<br>APRLLIY | G--------<br>ASSRAT | GIPDRFSGSGSG--<br>TDFTLTINRLEPKDFAVYYC | QQYGS-----------<br>------------SPWT | FGQGT<br>KVEIK |
| iPS:436 064 | 21-225_194E6 | VK3\|A27/ JK1 | | EIVLTQSPGTLSL<br>SPGERATLSC | RAS--QSIRS-<br>----NFLA | WYQQKPGQ<br>APRLLIY | G--------<br>ASSRAT | GIPDRFSGSVSG--<br>TDFTLTINRLEPEDFAVYYC | QQYGS-----------<br>------------SPWT | FGQGT<br>KVEIK |
| iPS:436 072 | 21-225_194C10 | VK3\|A27/ JK1 | | EIVLTQSPGTLSL<br>SPGERATLSC | RAS--PSVNS-<br>----GYLA | WYQQKPGQ<br>TPRLLIF | G--------<br>ASSRAT | GIPDRFSASGSG--<br>ADFTLTISRLEPEDFAVYFC | QQYES-----------<br>------------SPWT | FGQGT<br>KVEIK |
| iPS:436 080 | 21-225_195B1 | VK3\|A27/ JK1 | | EIVLTQSPGTLSL<br>SSGERATLSC | RAS--PSVNS-<br>----NYLA | WYQQKPGQ<br>TPRLLIY | G--------<br>ASNRAT | GVPDRFSASGSG--<br>TDFTLTIRRLEPEDFAVYFC | QQYES-----------<br>------------SPWT | FGQGT<br>KVEIK |
| iPS:436 088 | 21-225_195C8 | VK3\|A27/ JK1 | | EIVLTQSPGTLSL<br>SPGERATLSC | RAS--QSIRS-<br>----SFLA | WYQQKPGQ<br>APRLLIY | G--------<br>AFSRAT | GIPDRFSGSGSG--<br>TDFTLTISRLEPEDFAVYYC | QQYGN-----------<br>------------SPWA | FGQGT<br>KVEIK |
| iPS:436 122 | 21-225_196G10 | VK3\|A27/ JK1 | | EIVLTQSPGTLLL<br>SPGERATLSC | RAS--PSVSN-<br>----SFLA | WYQQKPGQ<br>APRLLIY | G--------<br>ASSRAT | GIPDRFSGSGSG--<br>TDFTLTNSRLEPEDFAVYYC | QQYGS-----------<br>-----------SPPWT | FGQGT<br>KVELK |
| iPS:436 134 | 21-225_196H12 | VK3\|A27/ JK1 | | EIVLTQSPGTLFL<br>SPGERATLSC | RAS--QSVRS-<br>----NFLA | WHQQKPGQ<br>APRLLIY | G--------<br>AYRRAT | GIPDRFSGSGSG--<br>TDFTLTINRLEPEDFAVYYC | QQYGN-----------<br>------------SPWT | FGQGT<br>KVEIK |
| iPS:436 146 | 21-225_197F4 | VK3\|A27/ JK1 | | EIVLTQSPGTLSL<br>SPGEGATLSC | RAS--QSIRS-<br>----SFLA | WYLQKPGQ<br>APRLLIY | G--------<br>ASSRAT | GIPDRFSGSGSG--<br>TDFTLTISRLEPEDFAVYYC | QQYGN-----------<br>------------SPWA | FGQGT<br>KVEIK |
| iPS:436 177 | 21-225_198B1 | VK3\|A27/ JK1 | | EIVLTQSPGTLSL<br>SPGESATLSC | RAG--QSIRT-<br>----NFLA | WYQQQPGQ<br>APRLLIY | G--------<br>ASSRAT | GIPDRFSGSGSG--<br>TDFTLTINRLEPEDFAVYYC | QQYGS-----------<br>------------SPWT | FGQGT<br>KVEIK |
| iPS:436 179 | 21-225_198E1 | VK3\|A27/ JK1 | | EIVLTQSPGTLSL<br>SPGERATLSC | RAS--QSIRS-<br>----NFLA | WYQQKPGQ<br>APRLLIY | G--------<br>AFSRAT | GIPDRFSGSGSG--<br>TDFTLTINRLEPEDFAVYYC | QQYGN-----------<br>------------SPWT | FGQGT<br>KVEIK |
| iPS:436 181 | 21-225_198C2 | VK3\|A27/ JK1 | | EIVVTQSPGTLLL<br>YSEERSTLSC | RAS--QSVRS-<br>----SYLA | WYQQKPGQ<br>APRLLIC | G--------<br>AFSRAS | GIPDRFSGSGSG--<br>TDFTLTISRLEPEDFAVYYC | QQYGN-----------<br>------------SPWT | LGHAT<br>KVEIK |
| iPS:436 195 | 21-225_198G10 | VK3\|A27/ JK1 | | EIVLTQSPGTLSL<br>SPGERATLSC | RAS--QSIRS-<br>----SFLA | WYQQKPGQ<br>APRLLIY | G--------<br>ASSRAT | GIPDRFSGSGSG--<br>TDFTLTISRLEPEDFAVYYC | QQYGN-----------<br>------------SPWA | FGQGT<br>KVEIK |
| iPS:436 197 | 21-225_199C2 | VK3\|A27/ JK1 | | EIVLTQSPGTLSL<br>SPGERATLSC | RAS--QSIRS-<br>----SFLA | WYQQKPGQ<br>APRLLIY | G--------<br>ASSRAT | GIPDRFSGSGSG--<br>TDFTLTINRLEPEDFAVYYC | QQYGN-----------<br>------------SPWA | FGQGT<br>KVEIK |

| | | | | | | | | | | |
|---|---|---|---|---|---|---|---|---|---|---|
| **iPS:436 207** | **21-225_199C7** | **VK3\|A27/ JK1** | | EIVLTQSPGTLSL SPGERATLSC | RAS--QSIRT- ----NFLA | WYQQKPGQ APRLLIY | G-------- ASSRAT | GIPDRFSGSGSG-- TDFTLTINRLEPEDFAVYYC | QQYGN----------- ------------SPWT | FGQGT KVEIK |
| **iPS:436 210** | **21-225_199G11** | **VK3\|A27/ JK1** | | EIVVTQSPGTLSL SPGERATLSC | RAS--QSVRS- ----SYLA | WYQQKPGQ APRLLIY | G-------- AFSRAT | GIPDRFSGSGSG-- TDFTLTISRLEPEDFAVYYC | QQYGN----------- ------------SPWT | FGHGT KVEIK |
| **iPS:436 226** | **21-225_200F10** | **VK3\|A27/ JK1** | | EIVLTQSPGTLSL SPGERATLSC | RAS--QNIRS- ----SFLA | WYQQKPGQ APRLLIY | G-------- ASSRAT | GIPDRFSGSGSG-- TDFTLTINRLEPEDFAVYYC | QQYGN----------- ------------SPWA | FGQGT KVEIK |
| **iPS:436 232** | **21-225_201E1** | **VK3\|A27/ JK1** | | EIVLTQSPDTLSL SPGERATLSC | RAS--PSINS- ----GFLA | WYQQKPGQ APRLLIY | G-------- ASSRAT | GIPDRFSGSGSG-- TDFTLTISRLEPEDFAMFHC | HQYET----------- ------------SPWT | FGQGT KVEIK |
| **iPS:436 238** | **21-225_201B2** | **VK3\|A27/ JK1** | | EIVLTQSPGTLSL SPGERATLSC | RAS--QSVSS- ----NYLA | WYQQRPGQ APRLLIY | G-------- ASSRAT | GIPDRFSGSGSG-- TDFTLTISRLEPEDFAVYFC | QQYEN----------- ------------SPWT | FGQGT KVEIK |
| **iPS:436 256** | **21-225_202D9** | **VK3\|A27/ JK1** | | EIVLTQSPDTLSL SPGERATLSC | RAS--QSVNS- ----GYLA | WYQQKPGQ SPRLLIY | G-------- ASSRAT | GIPDRFSGSGSG-- TDFTLTISRLEPEDFAVHYC | QQYET----------- ------------SPWT | FGQGT KVEIK |
| **iPS:436 302** | **21-225_205G7** | **VK3\|A27/ JK1** | | EIVLTQSPGTLSL SPGERATLSC | RAS--QSVFS- ----NYLA | WYQQKPGQ APRLLIY | G-------- ASSRAA | GIPDRFSGSGSG-- TDFTLTISRLEPENFAVYYC | QQYES----------- ------------SPWT | FGQGT KVEIK |
| **iPS:436 310** | **21-225_202D11** | **VK3\|A27/ JK1** | | EIVLTQSPGTLSL SPGERATLSC | RAS--QSINS- ----NYLA | WYQRKPGQ APRVLIY | G-------- ASSRAT | GIPDRFSGSGSG-- TDFTLTISRLEPEDFAVFYC | QQYEN----------- ------------SPWT | FGQGT KVEIK |
| **iPS:436 336** | **21-225_208B5** | **VK3\|A27/ JK1** | | EIVLTQSPGTLSL SPGERATLSC | RAS--QSVSS- ----NYLA | WYQQRPGQ APRLLIY | G-------- ASSRAT | GIPDRFSGSGSG-- TDFTLTISRLEPEDFAVYFC | QHYEN----------- ------------SPWT | FGQGT KVEIK |
| **iPS:436 340** | **21-225_208A9** | **VK3\|A27/ JK1** | | EIVLTQSPGTLSL SPGERATLSC | RAS--QSVSN- ----NYLA | WYQQKPGQ APRVLIY | G-------- ASSRAT | GIPDRFSGSGSG-- TDFTLTISRLEPEDFAVYYC | QHYHS----------- ------------SPWT | FGQGT KVEIK |
| **iPS:436 472** | **21-225_220E1** | **VK3\|A27/ JK1** | | EIVLTQSPGTLSL SPGERATLSC | RAS--QSISR- ----SHLV | WYQQKPNQ APRLLLY | V-------- TSSRAT | GIPDRFSGSGSG-- TDFTLTIRSLEPEDFAMYYC | QQYGS----------- ------------SPWT | FGQGT KVEIK |
| **iPS:436 506** | **21-225_222C7** | **VK3\|A27/ JK1** | | EIVLTQSPGTLSL SPGERATLSC | RAS--QSVYS- ----NYLA | WYQQKPGQ APRLLIY | G-------- ASSRAT | GIPDRFGGSGSG-- TDFTLAISRLEPEDFTIYYC | QQYED----------- ------------SPWT | FGQGT KVEIK |
| **iPS:436 580** | **21-225_225E7** | **VK3\|A27/ JK1** | | EIVLTQSPGTLSL SPGERATLSC | RAS--QSVYS- ----SYLA | WYQQKPGQ APRLLIY | G-------- ASSRAT | GIPDRFSGSGSG-- TDFTLTISRLEPEDFAVYYC | QQYGT----------- ------------SPRT | FGQGT KVEIK |
| **iPS:437 324** | **21-225_75C2** | **VK3\|A27/ JK1** | | EIVLTQSPGTRYL SPGERATLSC | RAS--QSVYS- ----SYLA | WYQQKPGQ APRLLIY | G-------- ASSRAT | GIPDRFSGSGSG-- TDFALTISRLEPEDCAVYYC | QHYDN----------- ------------SPWT | FGRGT KVEIK |
| **iPS:437 328** | **21-225_75D3** | **VK3\|A27/ JK1** | | EIVLTQSPGTLYL SPGERATLSC | RAS--QSVYS- ----SYLA | WYQQKPGQ APRLIIY | G-------- ASSRST | GIPDRFSGSGCG-- TDFALTISRVEHEDCAVYYC | QHYDN----------- ------------SPWT | FGQGT KVEIK |
| **iPS:437 332** | **21-225_75F3** | **VK3\|A27/ JK1** | | EIVLTQSPGTLYL SPGERATLSC | RAS--QSVYS- ----SYLA | WYQQKPGQ APRLLIY | G-------- ASSRAT | GIPDRFSGSGCG-- TDFALTISRLEHEDFAVYYC | QHYDN----------- ------------SPWT | FGQGT KVEIK |
| **iPS:437 340** | **21-225_75G9** | **VK3\|A27/ JK1** | | EIALTQSPGTLSL SPGERATLSC | RAS--PSVDS- ----SYLA | WYQQKPGQ APRLLIY | G-------- ASSRAP | GIPDRFSGSGSG-- TDFTLTISRLEPEDFAVYYC | QQYES----------- ------------SPWT | FGQGT KVEIK |
| **iPS:437 344** | **21-225_75G12** | **VK3\|A27/ JK1** | | EIVLTQSPGTLYL SPGERATLSC | RAS--QSVYS- ----SYLA | WYQQKPGQ APRLLIY | G-------- ASSRAT | GIPDRFSGSGSG-- TDFALTISRVEPEDCAVYYC | QHYDN----------- ------------SPWT | FGQGT KVEIK |
| **iPS:437 350** | **21-225_74A3** | **VK3\|A27/ JK1** | | EIVLTQSPGTLSL SPGERATLSC | RAS--QSVYS- ----SYLA | WYQQKPGQ APRLLIY | G-------- ASSRST | GIPDRFSGSGCG-- TDFALTISRLEPEDFAVYYC | QHSDN----------- ------------SPWT | FGQGT KVEIK |
| **iPS:437 369** | **21-225_74D6** | **VK3\|A27/ JK1** | | EIVLTQSPGTLSL SPGERATLSC | RAS--QSVYS- ----SYLA | WYQQKPGQ APRLLIY | G-------- ASSRAT | GIPDRFSGSGCG-- TDFALTISRLEPEDFAVYYC | QHYDN----------- ------------SPWT | FGQGT KVEIK |
| **iPS:437 383** | **21-225_74H8** | **VK3\|A27/ JK1** | | EIVLTQSPGTLSL SPGERATLSC | RAS--QSFSS- ----SYLA | WYQQKPGQ APRLLIY | G-------- ASSRAT | GIPDRFSGSGSG-- TDFTLTISRLEPEDFAVYYC | QQYG------------ ------------SSRT | FGQGT KVEIK |
| **iPS:451 122** | **21-225_200A1** | **VK3\|A27/ JK1** | | EIVLTQSPGILSL YPGERATLSC | RAS--QSVNS- ----NYLA | WYQQRPGQ APSLLIY | G-------- ASSRAT | CILDRFSGSGCG-- TDFTLTISRLEPEDFAVYCC | QQYEI----------- ------------SPWT | FGQGT KVESK |

| | | | | | | | | | | |
|---|---|---|---|---|---|---|---|---|---|---|
| **iPS:392 864** | **21-225_23B9** | **VK3\|A27/ JK1** | | EIVLTQSPGTLSL<br>SPGERATLSC | RAS--QNVYS-<br>----SYLA | WYQQKPGQ<br>TPRLLIY | G--------<br>ASSRAS | GIPDRFSGSGSG--<br>TDFTLTISRLEPEDFAVYYC | QQYGS-----------<br>------------SPRT | FGQGT<br>KVEIK |
| | | **Germline** | | **K_FR1** | **K_CDR1** | **K_FR2** | **K_CDR2** | **K_FR3** | **K_CDR3** | **K_FR4** |
| | **VK1\|A30/JK 4** | | 32136 | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVEIK |
| **iPS:468 812** | **21-225_48H4** | **VK1\|A30/ JK4** | | DIQMTQFPSSLSA<br>SVGDRVTITC | RAS--RDIR--<br>----NDLG | WYQQKPGK<br>APKRLIY | A--------<br>ASSLQS | GVPSRFSGSGSG--<br>TEFTLTISSLQPEDFATYYC | LQHYS-----------<br>------------YPLT | FGGGT<br>KVEIK |
| **iPS:468 824** | **21-225_73G6** | **VK1\|A30/ JK4** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVEIK |
| **iPS:468 818** | **21-225_190C8** | **VK1\|A30/ JK4** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFETYYC | LQHND-----------<br>------------YPFT | FGGGT<br>KVEIK |
| **iPS:468 840** | **21-225_200H9** | **VK1\|A30/ JK4** | | DIQMTQSPSSLSA<br>SVGDRVTITC | RAS--QGIR--<br>----NDLG | WYQQKPGK<br>APKRLIY | A--------<br>ASSLQS | GIPSRFSGSGSG--<br>TEFTLTISSLQPEDFATYYC | LQHNS-----------<br>------------YPLT | FGGGT<br>KVEIK |
| **iPS:468 868** | **21-225_74A1** | **VK1\|A30/ JK4** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHDS-----------<br>------------YPLT | FGGGA<br>KVEIK |
| **iPS:392 920** | **21-225_29G4** | **VK1\|A30/ JK4** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVEIT |
| **iPS:433 899** | **21-225_43C3** | **VK1\|A30/ JK4** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVGIT |
| **iPS:433 921** | **21-225_44C3** | **VK1\|A30/ JK4** | | DIQMTQSPSSLSA<br>SVGDRVTITC | RAS--QGIR--<br>----NDLG | WYQQKPGK<br>APKRLIY | A--------<br>ASSLQS | GVPSRFSGGGSG--<br>TEFTLTISSLQPEDFATYYC | LQHSS-----------<br>------------YPLT | FGGGT<br>KVEIK |
| **iPS:433 947** | **21-225_44E12** | **VK1\|A30/ JK4** | | DIQMTQSPSSLSA<br>SVGDRVTITC | RTS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVEIK |
| **iPS:433 963** | **21-225_46B1** | **VK1\|A30/ JK4** | | DIQMTQSPSSLSA<br>SVGDRVTITC | RAS--QGIR--<br>----NDLG | WYQQKPGK<br>APKRLIY | A--------<br>ASSLQG | GVPSRFSGSGSG--<br>TEFTLTISSLQPEDFATYYC | LQHNS-----------<br>------------YPLT | FGGGT<br>KVEIK |
| **iPS:433 969** | **21-225_46F3** | **VK1\|A30/ JK4** | | DIQMTQSPSSLSA<br>SVGDRVTITC | RAS--QGIR--<br>----KDLG | WYQQKPGK<br>APKRLIY | A--------<br>ASSLQS | GVPSRFSGSGSG--<br>TEFSLTISSLQPEDFATYYC | LQHNS-----------<br>------------YPLT | FGGGT<br>KVEIK |
| **iPS:433 975** | **21-225_46C6** | **VK1\|A30/ JK4** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVEIT |
| **iPS:433 977** | **21-225_46D8** | **VK1\|A30/ JK4** | | DIQMTQSPSSLSA<br>SVGDRVTITC | RAS--QGIR--<br>----KDLG | WYQQKPGK<br>APKRLIY | A--------<br>ASSLQS | GVPSRFSGSGSG--<br>TEFSLTISSLQPEDFATYYC | LQHNS-----------<br>------------YPLT | FGGGT<br>KVEIK |
| **iPS:433 983** | **21-225_47A1** | **VK1\|A30/ JK4** | | DIQMTQSPSSLSA<br>SVGDRVTITC | RAS--QDIR--<br>----NDLG | WYQQKPGK<br>APKRLIY | A--------<br>AFSLQS | GVPSRFSGSRSG--<br>TEFTLTISSLQPEDFATYYC | LQHNS-----------<br>------------YPLT | FGGGT<br>KVEIK |
| **iPS:433 987** | **21-225_47A5** | **VK1\|A30/ JK4** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVEIK |
| **iPS:434 013** | **21-225_48D12** | **VK1\|A30/ JK4** | | DIQMTQSPSSLSA<br>SVGDRVTITC | RAS--QGIR--<br>----NDLG | WYQQKPGK<br>APKRLIY | A--------<br>ASTLQS | GVPSRISGSGSG--<br>TEFTLTISSLQPEDFATYYC | LQHNS-----------<br>------------YPLT | FGGGT<br>KVEIK |
| **iPS:434 019** | **21-225_49A1** | **VK1\|A30/ JK4** | | DIQMTQSPSSLSA<br>SVGDRVTITC | RAS--QGIR--<br>----NDLD | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVEIK |
| **iPS:434 029** | **21-225_49C6** | **VK1\|A30/ JK4** | | DIQMTQSPSSLSA<br>SVGDRVTITC | RAS--QGIR--<br>----NDLG | WYQQKPGK<br>APKRLIY | A--------<br>ASSLQS | GFPSRFSGSGSG--<br>TEFTLTISSLQPEDFATYYC | LQHNS-----------<br>------------YPLT | FGGGT<br>KVEIK |
| **iPS:434 043** | **21-225_50G10** | **VK1\|A30/ JK4** | | DIQMTQSPSSLSA<br>SVGDRVTITC | RAS--QGIR--<br>----NNLG | WYQQKPGK<br>VPKRLIY | A--------<br>ASSLQS | GVPSRFSGSGSG--<br>TEFTLTISSLQPEDFATYYC | LQYNS-----------<br>------------YPFT | FGGGT<br>KVESK |
| **iPS:434 077** | **21-225_51F11** | **VK1\|A30/ JK4** | | DIQMTQSPSALSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPFT | FGGGT<br>KVEIK |
| **iPS:434** | **21-** | **VK1\|A30/** | | DIQMTQSPSSLSA | RAS--QGIR-- | WYQQKPGK | A-------- | GVPSTFSGSGSG-- | LQHNS----------- | FGGGT |

| | | | | | | | | | | |
|---|---|---|---|---|---|---|---|---|---|---|
| **iPS:434 105** | **21-225_53D2** | **VK1\|A30/ JK4** | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNR----------- ------------YPLT | FGGGT KVEIK |
| **iPS:434 119** | **21-225_53E6** | **VK1\|A30/ JK4** | | DIQMTQSPSSLSA SVGARVTITC | RAS--QGIR-- ----NDLG | WYQQNPGK APKRLIY | A-------- ASNLQS | GVPSRFSGSGSG-- TEFTLTISSLQPEDFATYFC | LQHNR----------- ------------YPLT | FGGGT KVEIK |
| **iPS:434 141** | **21-225_54C6** | **VK1\|A30/ JK4** | | DIQMTQSPSSLSA SVGARVTITC | RAS--QGIR-- ----NDLG | WYQQNPGK APKRLIY | A-------- ASNLQS | GVPSRFSGSGSG-- TEFTLTISSLQPEDFATYFC | LQHNR----------- ------------YPLT | FGGGT KVEIK |
| **iPS:434 159** | **21-225_55B8** | **VK1\|A30/ JK4** | | DIQMTQSPSSLSS SVGDRVTITC | RAS--QAIR-- ----NDLG | WYQQKPGK APKRLIH | A-------- AFRLQS | GVPSRFSGSGSG-- TEFTLTISSLQPEDFATYYC | LQHNS----------- ------------YPLT | FGGGT KVGIK |
| **iPS:434 179** | **21-225_56F1** | **VK1\|A30/ JK4** | | DIQMTQSPSSLSA SVGDRVTITC | RAS--QDIR-- ----NNLG | WYQQKPGK APKRLIN | A-------- ASSLQS | GVPSRFSGSGSG-- TEFTLTISSLQPEDFATYYC | LQDNS----------- ------------HPFT | FGGGT KVEIK |
| **iPS:434 217** | **21-225_60E8** | **VK1\|A30/ JK4** | | DIQMTQSPSSLSA SVGDRVTFTC | RAS--QGIR-- ----NDLG | WFQQKPGK APKRLIY | A-------- ASSLQS | GVPSRFSGSGSG-- TEFTLTISSLQPEDFATYYC | LQHSS----------- ------------YPLT | FGGGT KVEIK |
| **iPS:434 249** | **21-225_62E2** | **VK1\|A30/ JK4** | | DIQMTQSPSSLSA SVGDRVTITC | RAS--QGIR-- ----NDLG | WYQQKPGK APKRLIY | A-------- ASRLQS | GVPSRFSGSGSG-- TEFTLTISSLQPEDFATYYC | LQHSN----------- ------------YPLT | FGGGT RVEIK |
| **iPS:434 253** | **21-225_62E4** | **VK1\|A30/ JK4** | | DIQMTQSPSSLSA SVGDRVTITC | RAS--QGIR-- ----NDLG | WYQQKPGK APKRLIY | A-------- AFSLQS | GVPSRFSGSGSG-- TEFTLTISSLQPEDFATYYC | LQHNS----------- ------------YPLT | FGGGT KVEIK |
| **iPS:434 313** | **21-225_59E6** | **VK1\|A30/ JK4** | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQIINS----------- ------------YPLT | FGGGT RVEIK |
| **iPS:434 337** | **21-225_64E1** | **VK1\|A30/ JK4** | | DIQMTQSPSSLSA SVGDRVTITC | RAS--QGIR-- ----NDLG | WYQQKPGK APKRLIY | A-------- ASSLQS | GVPSRFSGSKSG-- TEFTLTISSLQPEDLATYYC | LQHNS----------- ------------YPLT | FGGGT KVEIK |
| **iPS:434 411** | **21-225_68F11** | **VK1\|A30/ JK4** | | DIQMTQSPSSLSA SVGDRVTITC | RAS--QGIR-- ----NDLG | WYQQKPGK APKRLIY | A-------- ASSLQS | GVPSRFSGSGSG-- PEFTLSISSLQPEDFATYYC | LQHNS----------- ------------YPFT | FGGGT KVEIK |
| **iPS:434 413** | **21-225_68D12** | **VK1\|A30/ JK4** | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHST----------- ------------YPLT | FGGGT KVEIK |
| **iPS:434 433** | **21-225_70E8** | **VK1\|A30/ JK4** | | DIQMTQSPSSLST SVGDRVTITC | RAS--QGIR-- ----KDLG | WYQQKPGK APKRLIY | A-------- ASSLQS | GVPSRFSGSGSG-- TEFTLTISSLQPEDFATYYC | LQHNR----------- ------------YPLT | FGGGT KVEIK |
| **iPS:434 439** | **21-225_70E12** | **VK1\|A30/ JK4** | | DIQMTQSPSSLSA SVGDRVTITC | RAS--QGIN-- ----NNLN | WFQQKPGK APKRLIY | A-------- ASSLQS | GVPSRFSGSGSG-- TEFTLTISSLQPEDFATYYC | LQHNS----------- ------------YPLT | FGGGS KVEIK |
| **iPS:434 489** | **21-225_74E4** | **VK1\|A30/ JK4** | | DIQMTQSPSSLSA SVGDRVTITC | RAS--QGIR-- ----NDLG | WYQQKPGK APKRLIY | A-------- ASTLQS | GVPSRFSGSGSG-- TEFTLTISGLQPEDFATYHC | LQHSN----------- ------------YPLT | FGGGT KVEIK |
| **iPS:434 503** | **21-225_74D7** | **VK1\|A30/ JK4** | | DIQMTQSPSSLSA SVGDRVTITC | RAS--QGIR-- ----NDLG | WYQQKPGK APKRLIY | A-------- ASSLQS | GVPSRFSGSGFG-- TEFTLTISSLQPEDFATYYC | LQHSN----------- ------------YPLT | FGGGT KVEIK |
| **iPS:435 251** | **21-225_96A3** | **VK1\|A30/ JK4** | | DIQMTQSPSSLSA SVGDRVTITC | RAS--QGIR-- ----NDLG | WYQQKPGK APKRLIY | A-------- ASTLQS | GVPSRFSGSGSG-- TEFTLTISGLQREDFATYHC | LQHSN----------- ------------YPLT | FGGGT KVEIK |
| **iPS:435 293** | **21-225_146F1** | **VK1\|A30/ JK4** | | DIQMTQSPSSLSA SVGDRVTITC | RAS--QGIR-- ----NDLG | WYQQKPGK APKRLIY | A-------- ASSLQS | GVPSRFSGGGSG-- TEFTLTISSLQPEDFATYYC | LQHST----------- ------------YPLT | FGGGT KVEIK |
| **iPS:435 311** | **21-225_146H9** | **VK1\|A30/ JK4** | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPLT | FGGGT KVEIK |
| **iPS:435 313** | **21-225_146G1 1** | **VK1\|A30/ JK4** | | DIQMTQSPSSLSA SVGDRVTITC | RAS--QDIR-- ----NNFG | WYQQKPGK APKRLIY | A-------- ASSLQS | GVPLRFSGSGSG-- TEFTLTISSLQPEDFATYYC | LQHDS----------- ------------YPLT | FGGGT KVEIK |
| **iPS:435 361** | **21-225_148E1 1** | **VK1\|A30/ JK4** | | DIQMTQSPSSLSA SVGDRVTITC | RAS--QGIR-- ----NDLG | WYQQKPGK APKRLIS | A-------- ASSLQS | GVPSRFTGSGSG-- TEFTFTISSVQPEDFATYYC | LQHRN----------- ------------YPLT | FGGGT KVEIK |
| **iPS:435 363** | **21-225_148F12** | **VK1\|A30/ JK4** | | DIQMTQSPSSLSA SVGDRVTITC | RAS--QGIR-- ----NALG | WYQQKPGK APKRLIY | A-------- ASSLQS | GVPSRFSGSGSG-- TEFTLTISSLQPEDFATYFC | LQHNS----------- ------------YPLT | FGGGT KVEIK |

| | | | | | | | | | | |
|---|---|---|---|---|---|---|---|---|---|---|
| **iPS:435367** | **21-225_149G1** | **VK1\|A30/JK4** | | DIQMTQSPSSLSA<br>SVGARVTITC | RAS--QGIR--<br>----NDLG | WYQQKPGK<br>APKRLIY | A--------<br>ASNLQS | GVPSRFSGSGSG--<br>TEFTLTISSLQPEDFATYYC | LQHNS-----------<br>------------YPLT | FGGGT<br>KVEIK |
| **iPS:435377** | **21-225_149G5** | **VK1\|A30/JK4** | | DIQMTQSPSSLSA<br>SVGDRVTITC | RAS--QGIR--<br>----NALG | WFQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVEIK |
| **iPS:435397** | **21-225_149F12** | **VK1\|A30/JK4** | | DIQMTQSPSSLSA<br>SVGDRVTITC | RAS--QGIR--<br>----NDLG | WYQQKPGK<br>APKRLIY | A--------<br>ASNLQS | GVPSRFSGSGSG--<br>TEFTLTISSLQPEDFAIYYC | LQHNS-----------<br>------------YPLT | FGGGT<br>KVEIK |
| **iPS:435407** | **21-225_150E7** | **VK1\|A30/JK4** | | DIQMTQSPSSLSA<br>SVGDRVTITC | RAS--QGIR--<br>----NDLG | WYQQKPGK<br>APKRLIY | A--------<br>ASNLQS | GVPLRFSGSGSG--<br>TDFTLTISSLQPEDFAAYYC | LQHNS-----------<br>------------YPLT | FGGGT<br>KVEIK |
| **iPS:435449** | **21-225_152H9** | **VK1\|A30/JK4** | | DIQMTQSPSSLSA<br>SVGDRVTITC | RAS--QGIR--<br>----NDLG | WYQQKPGK<br>APKRLIY | A--------<br>ASSLQS | GVPSRFTGSGSG--<br>TEFTFTISSLQPEDFATYYC | LQHSN-----------<br>------------YPLT | FGGGT<br>KVEIK |
| **iPS:435499** | **21-225_156G1** | **VK1\|A30/JK4** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTFTISSLQPEDFATYYC | LQHSN-----------<br>------------YPLT | FGGGT<br>KVEIK |
| **iPS:435549** | **21-225_158H5** | **VK1\|A30/JK4** | | DIQMIQSPSFLFA<br>SVGDRVTITC | RAS--QGMR--<br>----IDLG | WYQQKPGK<br>APKRLIY | R--------<br>ASSLQS | GVPSRFSGSGCG--<br>TEFTLTISSVQREDFASYYC | VQHNS-----------<br>------------YPLT | FGGGT<br>KVEIK |
| **iPS:435587** | **21-225_160H3** | **VK1\|A30/JK4** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHSN-----------<br>------------YPLT | FGGGT<br>QVESK |
| **iPS:435599** | **21-225_160B10** | **VK1\|A30/JK4** | | DIQMTQSPSSPSA<br>SVGDRVTITC | RAS--QGIR--<br>----NDLG | WYQQKPGT<br>APKRLIY | A--------<br>ASSLQS | GVPSRFSGSGSG--<br>TEFTLTISNLQPEDFATYYC | LQHSS-----------<br>------------YPLT | FGGGT<br>KVEIK |
| **iPS:435663** | **21-225_169B1** | **VK1\|A30/JK4** | | DIQMTQSPSSLSA<br>SVGDRVTITC | RAS--QGIR--<br>----NDLG | WYQQKPGK<br>APKRLIG | A--------<br>ESSLQS | GVPSRFSGSGSG--<br>TEFTLTISGLQPEDFATYYC | LQHYS-----------<br>------------YPLT | FGGGT<br>KVEIK |
| **iPS:435669** | **21-225_169F9** | **VK1\|A30/JK4** | | DIQMTQSPSSLSA<br>SVGDRVTITC | RAS--QGIR--<br>----NDLG | WYQQKPGK<br>APKRLIF | A--------<br>ASSLQS | GVPSRFSGSGSG--<br>TEFTLTISSLQPEDFATYYC | LQHYS-----------<br>------------YPLT | FGGGT<br>KVEIK |
| **iPS:435693** | **21-225_170G4** | **VK1\|A30/JK4** | | DIQMTQSPSSLSA<br>SVGDRVTITC | RAS--QGIR--<br>----NDLG | WYHQKPGK<br>APKRLIY | A--------<br>ASTLQS | GVPSRFSGSGSG--<br>TEFTLTISSLQPEDFATYYC | LQHYS-----------<br>------------YPLT | FGGGT<br>KVEIK |
| **iPS:435695** | **21-225_170D5** | **VK1\|A30/JK4** | | DIQMTQSPSSLSA<br>SVGDRVTITC | RAS--QGIR--<br>----NDLG | WYQEKPGK<br>APKHLIY | A--------<br>ASSLQN | GVPSRFSGSGSG--<br>TEFTLTISSLQPEDFATYYC | LQHYS-----------<br>------------FPLT | FGGGT<br>KVEIK |
| **iPS:435697** | **21-225_170G5** | **VK1\|A30/JK4** | | DIQMTQSPSSLSA<br>SVGDRVTITC | RAS--QGIR--<br>----TDLG | WFQQKPGK<br>APKRLIY | T--------<br>ASSLQS | GVPSRFSGSGSG--<br>TEFTLTISSLQPEDFATYYC | LQHYS-----------<br>------------YPLT | FGGGT<br>KVEIK |
| **iPS:435703** | **21-225_170D11** | **VK1\|A30/JK4** | | DIQMTQSPSSLSA<br>SVGDRVTITC | RAS--QGIR--<br>----NDLG | WYQQKPGK<br>APKHLIY | A--------<br>ASSLQN | GVPSRFSGSGSG--<br>TEFTLTISSLQPEDFATYYC | LQHYS-----------<br>------------FPLT | FGGGT<br>KVEIR |
| **iPS:435705** | **21-225_171C3** | **VK1\|A30/JK4** | | DIQMTQSPSSLSA<br>SVGDRVTITC | RAS--QGIR--<br>----TDLG | WFQQKPGK<br>APKRLIY | T--------<br>ASSLQS | GVPSRFSGSGSG--<br>TEFTLTISSLQPEDFATYYC | LQHYS-----------<br>------------YPLT | FGGGT<br>KVEIK |
| **iPS:435709** | **21-225_171A4** | **VK1\|A30/JK4** | | DIQMTQSPSSLSA<br>SVGDRVTITC | RAS--QGIR--<br>----NDLG | WYQQKPGK<br>APKRLIY | T--------<br>ASSLQS | GVPSRFSGSGSG--<br>TEFTLTISSLQPEDFATYYC | LQHYS-----------<br>------------YPLT | FGGGT<br>KVEIK |
| **iPS:435721** | **21-225_172B3** | **VK1\|A30/JK4** | | DIQMTQSPSSLSA<br>SVGDRVTITC | RAS--QGIR--<br>----NDLG | WYQQKPGK<br>APKRLIG | A--------<br>ESSLQS | GVPSRFSGSGSG--<br>TEFTLTISGLQPEDFATYYC | LQHYS-----------<br>------------YPLT | FGGGT<br>KVEIK |
| **iPS:435725** | **21-225_172G8** | **VK1\|A30/JK4** | | DIQMTQSPSSLSA<br>SVGDRVTITC | RAS--QGVR--<br>----NDLG | WYQQKPGK<br>APKHLIY | A--------<br>ASSLQN | GVPSRFSGSGSG--<br>TEFTLTISSLQPEDFATYYC | LHHYS-----------<br>------------FPLT | FGGGT<br>KVEIK |
| **iPS:435735** | **21-225_173H12** | **VK1\|A30/JK4** | | DIQMTQSPSSLSA<br>SVGDRVTITC | RAS--QGIR--<br>----NDLG | WYQQKPGK<br>APKRLIY | T--------<br>TSSLQS | GVPSRFSGSGSG--<br>TEFTLTISSLQPEDFASYYC | LQHYS-----------<br>------------FPNT | FGGGT<br>KVEIK |
| **iPS:435743** | **21-225_175G1** | **VK1\|A30/JK4** | | DIQMTQSPSSLSA<br>SVGDRVTITC | RAS--QGIR--<br>----TDLG | WYQQKPGK<br>APKRLIY | T--------<br>ASSLQS | GVPSRFSGSGSG--<br>TEFTLTISSLQPEDFATYYC | LQHYS-----------<br>------------YPLT | FGGGT<br>KVEIK |

| | | | | | | | | | | |
|---|---|---|---|---|---|---|---|---|---|---|
| **iPS:435 761** | **21-225_176B1 1** | **VK1\|A30/ JK4** | | DIQMTQSPSSLSA<br>SVGDRVTITC | RAS--QGIR--<br>----NDLG | WFQQKPGK<br>APKRLIY | A--------<br>ASSLQS | GVPSRFSGSGSG--<br>TEFTLTLSSLQPEDFATYYC | LQHYS-----------<br>------------YPLT | FGGGT<br>KVEIK |
| **iPS:435 779** | **21-225_178B1 0** | **VK1\|A30/ JK4** | | DIQMTQSPSSLSA<br>SVGDRVTITC | RAS--QGIR--<br>----NDLG | WYQQKPGK<br>APKHLIY | A--------<br>ASSLQN | GVPSRFSGSGSG--<br>TEFTLTISSLQPEDFATYYC | LHHYS-----------<br>------------FPLT | FGGGT<br>KVEIK |
| **iPS:436 023** | **21-225_193A5** | **VK1\|A30/ JK4** | | DIQMTQSPSSLFA<br>SVGDRVIISC | RAS--QGIR--<br>----NDLG | WYQQYPGK<br>APKRVIY | A--------<br>ASSLQS | GVPSRFSGSGFG--<br>TEFTITISSVQPEDFETYYC | LQHND-----------<br>------------FPFT | FGGGT<br>KVEIK |
| **iPS:436 033** | **21-225_193E7** | **VK1\|A30/ JK4** | | DIQMTQSPSSLSA<br>SVGDRVTITC | RAS--QGIR--<br>----NDLG | WYQQKPGK<br>APKRLIY | S--------<br>ASSLQR | GVPSRFSGSGSG--<br>TEFTLTISSLQPEDFATYYC | LQHKR-----------<br>------------YPLT | FGGGT<br>KVEIK |
| **iPS:436 120** | **21-225_196C1 0** | **VK1\|A30/ JK4** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQYNS-----------<br>------------YPLT | FGGGT<br>KVEIK |
| **iPS:436 199** | **21-225_199E3** | **VK1\|A30/ JK4** | | DIQMTQSPSSLSA<br>SVGDRVTITC | RAS--QGIR--<br>----NDLG | WYQQKPGK<br>APKRLIS | S--------<br>ASSLQR | GVPSRFSGSGSG--<br>TEFTLTISSLQPEDFATYYC | LQHKR-----------<br>------------YPLT | FGGGT<br>KVEIK |
| **iPS:436 228** | **21-225_200F12** | **VK1\|A30/ JK4** | | DIQMTQSPSSLSA<br>SVGDRVTITC | RAS--QGIR--<br>----NDLG | WYQQKPGR<br>APKRLIY | S--------<br>ASSLHT | GVPSRFSGSGSG--<br>TEFTLTISSLQPEDFATYYC | LQHKS-----------<br>------------YPLT | FGGGT<br>KVEIK |
| **iPS:436 230** | **21-225_201A1** | **VK1\|A30/ JK4** | | DIQMTQSPSSLSA<br>SVGDRVTITC | RAS--QGIR--<br>----NDLG | WYQQKPGK<br>APKRLIY | S--------<br>ASILQR | GVPSRFSGSGSG--<br>TEFTLTISSLQPEDFATYYC | LQHKS-----------<br>------------YPLT | FGGGT<br>KVEVK |
| **iPS:436 242** | **21-225_201A1 0** | **VK1\|A30/ JK4** | | DIQMTQSPSSLSA<br>SVGDRVTITC | RAS--QGIR--<br>----NDLG | WYQQKPGR<br>APKRLIY | S--------<br>TSSLHS | GVPSRFSGSGSG--<br>TEFTLTISSLQPEDFATYYC | LQHNS-----------<br>------------YPLT | FGGGT<br>KVEIK |
| **iPS:436 286** | **21-225_204H8** | **VK1\|A30/ JK4** | | DIQMTQSPSSLSA<br>SVGDRVTITC | RAS--QGIR--<br>----NDLG | WYQQKPGK<br>APKRLIY | S--------<br>ASSLQS | GVPSRFSGRGSG--<br>TEFTLTVSSLQPEDFATYYC | LQHNS-----------<br>------------YPLT | FGGGT<br>KVEIK |
| **iPS:436 308** | **21-225_205H8** | **VK1\|A30/ JK4** | | DIQMTQSPSSLSA<br>SVGDRVTITC | RAS--QGIR--<br>----NDLG | WYQQKQGK<br>APKLLIY | S--------<br>ASFLQR | GVPSRFSGSGSG--<br>TEFTLTISSLQPEDSAAYYC | LQHNS-----------<br>------------YPLT | FGGGT<br>TVKIK |
| **iPS:436 526** | **21-225_224A1** | **VK1\|A30/ JK4** | | DIQMTQSPSSLSA<br>SVGDRVTITC | RAS--QGIE--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVEIK |
| **iPS:436 528** | **21-225_224B1** | **VK1\|A30/ JK4** | | DIQMTQSPSSLSA<br>SVGDRVTITC | RAS--QGIS--<br>----NDLG | WYQQKPGK<br>APKRLIY | A--------<br>ASSLQS | GVPSRFSGSGSG--<br>TEFTLTISSLQPEDFATYHC | LQHNS-----------<br>------------YPPT | FGGGT<br>KVEIK |
| **iPS:436 538** | **21-225_224C3** | **VK1\|A30/ JK4** | | DIQMTQSPSSLSA<br>SVGDRVTITC | RAS--QDIR--<br>----NDLG | WYQQKPGK<br>APKRLIY | A--------<br>TSSLQS | GVPSRFSGSGSG--<br>TEFTLTISSLQPEDFATYYC | LQHNS-----------<br>------------YPLT | FGGGT<br>KVEIK |
| **iPS:436 556** | **21-225_224D1 0** | **VK1\|A30/ JK4** | | DILMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVEIK |
| **iPS:437 220** | **21-225_55H6** | **VK1\|A30/ JK4** | | DIQMTQSPSSLSA<br>SVGDRVTITC | RAS--QGIR--<br>----NDLG | WYQQKPGK<br>APKRLIY | G--------<br>ASSLQS | GVPSRFSGSGSG--<br>TEFTLTISSLQPEDFAAYYC | LQRDS-----------<br>------------YPFT | FGGGT<br>KVEIK |
| **iPS:437 346** | **21-225_75H7** | **VK1\|A30/ JK4** | | DIQMTQSPSSRYA<br>SVGDRVTINS | RAS--QGIR--<br>----NDLG | WYQQKPGK<br>SPQRLIY | D--------<br>ASSLQS | GVPSRFSGSGSG--<br>TEFTLTISSVQPEDFGVYYC | IQHSN-----------<br>------------YPLT | FGGGT<br>KVEIK |
| **iPS:472 730** | **21-225_14B1_ LC1** | **VK1\|A30/ JK4** | | DIQMTQSPSYLSA<br>SVGDRVTITC | RAS--QDIR--<br>----DNLG | WYQQKPGK<br>APKRLIY | T--------<br>AYSLQS | GVPSRFSGSGSG--<br>TEFTLTISSLQPEDFATYYC | LQHYN-----------<br>------------YPLT | FGGGT<br>KVEIK |
| **iPS:392 622** | **21-225_17H8** | **VK1\|A30/ JK4** | | DIQMTQSPSSLSA<br>SVGDRVTITC | RAS--QGIR--<br>----NDLG | WYQQKPGK<br>APKRLIY | G--------<br>ASSLQS | GVPSRFSGSGSG--<br>TEFTLTISSLQPEDFTTYYC | LQHNS-----------<br>------------YPLT | FGGGT<br>KVEIK |
| **iPS:392 624** | **21-225_17H12** | **VK1\|A30/ JK4** | | DIQMTQSPSSLSA<br>SVGDRVTITC | RAS--QDIR--<br>----NDLG | WYQQKAGK<br>APKRLIN | A--------<br>ASSLQS | GVPSRFSGIGSG--<br>TEFTLTITGLQPEDFATYYC | LQHYS-----------<br>------------YMFT | FGGGT<br>KVEIK |

| | | | | | | | | | | |
|---|---|---|---|---|---|---|---|---|---|---|
| iPS:392 628 | 21-225_20C2 | VK1\|A30/JK4 | | DIQMTQSPSSLSA<br>SVQDRVTITC | RAS--QGIR--<br>----NDLG | WYQQKPGK<br>APKRLIF | A--------<br>ASSLQS | GVPSRFSGSGSG--<br>TEFTLTISSLQPEDFATYYC | LQHAS-----------<br>------------YPLT | FGGGT<br>KVEIE |
| iPS:392 630 | 21-225_20E5 | VK1\|A30/JK4 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVEIK |
| iPS:392 638 | 21-225_17F9 | VK1\|A30/JK4 | | DIQMTQSPSSLSA<br>SVGDRVTITC | RAS--QVIR--<br>----NDLG | WYQQKPGK<br>APKRLIY | A--------<br>VSSLQS | GVPSRFSGSGSG--<br>TEFTLTINSLQPEDFATYYC | LQHNT-----------<br>------------YPLT | FGGGT<br>KVEFK |
| iPS:392 640 | 21-225_18A1 | VK1\|A30/JK4 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVEIK |
| iPS:392 642 | 21-225_18C6 | VK1\|A30/JK4 | | DIQMTQSPSSLSA<br>SVGDRVTITC | RTS--QGIR--<br>----NDLG | WYQQKPGK<br>APKRLIY | A--------<br>ASSLQS | GVPSRFSGSGSG--<br>TEFTLTISSLQPEDFATYYC | LQHSS-----------<br>------------YPLT | FGGGT<br>KVEIK |
| iPS:392 644 | 21-225_19E1 | VK1\|A30/JK4 | | DIQMTQSPSSLSA<br>SVGDRVTITC | RAS--QGIR--<br>----NDLG | WYQQKPGK<br>APKRLIY | A--------<br>ASNLQS | GVPSRFSGSGSG--<br>TEFTLTISSLQPEDFATYYC | LQHNS-----------<br>------------FPLT | FGGGT<br>KVEIK |
| iPS:392 646 | 21-225_20G2 | VK1\|A30/JK4 | | DIQMTQSPSSLSA<br>SVGDRVTITC | RAS--QGIR--<br>----NDLG | WYQQKPGK<br>APKRLIY | A--------<br>ASSFQS | GVPSRFSGSGSG--<br>TEFTLTISSLQPEDFASYYC | LQHNS-----------<br>------------YPLT | FGGGT<br>KVEIK |
| iPS:392 654 | 21-225_17A10 | VK1\|A30/JK4 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVEIK |
| iPS:392 656 | 21-225_1F2 | VK1\|A30/JK4 | | DIQMTQSPSSLSA<br>SVGDRVTITC | RAS--QGIR--<br>----NDLG | WYQQKPGK<br>APKRLIY | A--------<br>ASSVQS | GVPSRFSGSGSG--<br>TEFTLTISSLQPEDFAIYYC | LQIINS-----------<br>------------YPLT | FGGGT<br>KVEIK |
| iPS:392 658 | 21-225_18E8 | VK1\|A30/JK4 | | DIQMTQAPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVEIK |
| iPS:392 666 | 21-225_16F11 | VK1\|A30/JK4 | | DIQMTQSPSSLSA<br>SVGDRVTITC | RAS--QGIR--<br>----NDLG | WYQQKPGK<br>APKRLIY | A--------<br>ASSVQS | GVPSRFSGSGSG--<br>TEFTLTISSLQPEDFATYYC | LQHNS-----------<br>------------YPLT | FGGGT<br>KVEIK |
| iPS:392 676 | 21-225_19F3 | VK1\|A30/JK4 | | DIQMTQSPSSLSA<br>SVRDRVTITC | RAS--QGIR--<br>----NDLG | WYQQKPGK<br>APKRLIY | A--------<br>VSSLQS | GVPSRFSGSGSG--<br>TEFTLTISSLQPEDFATYYC | LQHAS-----------<br>------------YPLT | FGGGT<br>KVEIK |
| iPS:392 680 | 21-225_20A7 | VK1\|A30/JK4 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFSLTISSLQPEDFATYYC | LQHNS-----------<br>------------YPLT | FGGGT<br>KVEIK |
| iPS:392 700 | 21-225_16E12 | VK1\|A30/JK4 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVEIK |
| iPS:392 706 | 21-225_18A3 | VK1\|A30/JK4 | | DIQMTQSPSSLSA<br>SVGDRVTITC | RAS--QGIR--<br>----NDLG | WYQQKPGK<br>APKRLIY | V--------<br>ASSLQS | GVPSRFNGSGSG--<br>TEFTLTISSLQPEDFATYYC | LQHSS-----------<br>------------YPLT | FGGGT<br>KVEIK |
| iPS:392 716 | 21-225_17B5 | VK1\|A30/JK4 | | DIQMTQSPSSLSA<br>SVGDRVTITC | RAS--QGIR--<br>----NDLG | WYQQKPGK<br>APKRLIY | A--------<br>ASNLQS | GVPSRFSGSGSG--<br>TEFSFTISSLQPEDFATYYC | LQHNS-----------<br>------------YPLT | FGGGT<br>KVEIK |
| iPS:392 744 | 21-225_20D5 | VK1\|A30/JK4 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPFT | FGGGT<br>KVEIK |
| iPS:392 750 | 21-225_20A10 | VK1\|A30/JK4 | | DIQMTQSPSSLSA<br>SVGDRVTITC | RAS--QGIR--<br>----NDLG | WYQQKQGK<br>APKRLIY | A--------<br>ASSLQS | GVPSRFSGSGSG--<br>TEFTLTISSLQPEDFATYYC | LQHNR-----------<br>------------YPLT | FGGGT<br>KVEIK |
| iPS:392 772 | 21-225_20E12 | VK1\|A30/JK4 | | DIQMTQSPSSLSA<br>SVGDRVTITC | RAS--QGIR--<br>----NDLG | WYQQKPGK<br>APKRLIF | A--------<br>ASSLQS | GVPSRFSGSGSG--<br>TEFTLTISSLQPEDFATYYC | LQHNS-----------<br>------------YPFT | FGGGT<br>KVEIK |
| iPS:392 774 | 21-225_21F3 | VK1\|A30/JK4 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TDFILTISSLQPEDFATYYC | LQHSS-----------<br>------------YPLT | FGGGT<br>KVEIK |
| iPS:392 780 | 21-225_22B7 | VK1\|A30/JK4 | | DIQMTQSPSSLSA<br>SVGDRVTITC | RAS--QGIR--<br>----NDLG | WYQQKPGK<br>APKRLIY | T--------<br>ASSLQS | GVPSRFSGSGSG--<br>TEFTLTISSLQPEDFATYYC | LQHNT-----------<br>------------YPLT | FGGGT<br>KVEIK |
| iPS:392 788 | 21-225_20C8 | VK1\|A30/JK4 | | DIQMTQSPSSLSA<br>SVGDRVTITC | RAS--QGIR--<br>----NDLG | WYQKKPGK<br>APKRLIN | A--------<br>ASSLQS | GVPSRFSGSGSG--<br>TEFTLTISSLQPEDFATYYC | LQDNS-----------<br>------------YPFT | FGGGT<br>KVEIT |
| iPS:392 794 | 21-225_21H3 | VK1\|A30/JK4 | | DIQMTQSPSSLSA<br>SVGDRVTITC | RAS--QGIR--<br>----NDLG | WYQQKPGK<br>APKRLIY | A--------<br>ASSVQT | GVPSRFSGSGSG--<br>TEFTLTISSLQAEDLAIYYC | LQHNS-----------<br>------------YPLT | FGGGT<br>KVEIK |

| | | | | | | | | | | |
|---|---|---|---|---|---|---|---|---|---|---|
| iPS:392 800 | 21-225_22D12 | VK1\|A30/ JK4 | | DIQMTQSPSSLSA SVGDRVTITC | RAS--QGIR-- ----NDLG | WYQQKPGK APKRLIY | A-------- ASSLQS | GVPSRFSGSGSG-- TDFTLTISSLQPEDFATYYC | LQHST----------- ------------YPLT | FGGGT KVEIK |
| iPS:392 810 | 21-225_20H12 | VK1\|A30/ JK4 | | DIQMTQSPSSLSA SVGDRVTITC | RAS--QGIR-- ----NDLD | WYQQKPGK APKRLIY | A-------- ASSLQS | GVPSRFSGSGSG-- TEFTLTISSLQPEDFATYYC | LQHNS----------- ------------YPLT | FGGGT KVEIK |
| iPS:392 820 | 21-225_23D1 | VK1\|A30/ JK4 | | DIQMTQSPSSLSA SVGDRVTITC | RAS--QGIR-- ----NDLG | WYQQKPGK APKRLIY | A-------- ASSLQS | GVPSRFSGSGSG-- TEFTLTVSSLQPEDFATYYC | LQHSS----------- ------------YPLT | FGGGT KVEIK |
| iPS:392 822 | 21-225_23C8 | VK1\|A30/ JK4 | | DIQMTQSPSSRSA SVGDRVTITC | RAS--QDIR-- ----NDLG | WYQQKPGR APKRLIN | G-------- ASSVQS | GVPSRFSGSGSG-- TEFTLTISSLQPEDFVIYYC | LQHNS----------- ------------YPLT | FGGGT KVEIK |
| iPS:392 824 | 21-225_24E5 | VK1\|A30/ JK4 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHSN----------- ------------YPLT | FGGGT KVEIK |
| iPS:392 834 | 21-225_22C1 | VK1\|A30/ JK4 | | DIQMTQSPSSLSA SVGDRVTITC | RTS--QGIR-- ----NDLG | WYQQKPGK APKRLIY | A-------- ASSLQS | GVPSRFSGSGSG-- TEFTLTISSLQPEDFATYYC | LQHST----------- ------------YPLT | FGGGT KVEIK |
| iPS:392 838 | 21-225_22G8 | VK1\|A30/ JK4 | | DIQMIQSPSSLFA SVGDRVTITC | RTS--QGIR-- ----NDLG | WYQQKPGK APKRLIY | A-------- ASSLQS | GVPSKFSGSGSG-- TEFTLSISSLQPEDFATYYC | LQHNS----------- ------------YPLT | FGGGT KVEIK |
| iPS:392 850 | 21-225_20H10 | VK1\|A30/ JK4 | | GIQMTQSPSSLSA SVGDRVTITC | RAS--QGIK-- ----NNLG | WYQQKPGK GPKCLIY | A-------- ASSLQS | GVPSRFSGSGSG-- TEFTLTISSLQPEDFATYYC | LQHNS----------- ------------YPLT | FGGGT KVEIK |
| iPS:392 854 | 21-225_21E5 | VK1\|A30/ JK4 | | DIQMTQSPSSLSA SVGDRVTITC | RAS--QGIR-- ----NDLG | WYQQKPGK APKRLIY | A-------- TSSLQS | GVPSRFSGSGSG-- TEFTLTISSLQPEDFATYYC | LQIINS----------- ------------YPLT | FGGGT KVEIK |
| iPS:392 858 | 21-225_22H4 | VK1\|A30/ JK4 | | DIQMTQSPSSLSA SVGDRVTITC | RAS--QGIR-- ----NDLG | WYQQKPGK APKRLIY | A-------- ASSVQS | GVPSRFSGSGSG-- TEFTLTISSLQPEDFALYYC | LQHNS----------- ------------YPLT | FGGGT KVEIK |
| iPS:392 866 | 21-225_23H11 | VK1\|A30/ JK4 | | DIQMTQSPSSLSA SVGDRVTITC | RAS--QGIR-- ----NDLD | WYQQKPGK APKRLIY | A-------- ASSLQS | GVPSRFSGSGSG-- TEFTLTISSLQPEDFATYYC | LQHNR----------- ------------YPLT | FGGGT KVEIK |
| iPS:392 870 | 21-225_20G9 | VK1\|A30/ JK4 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHST----------- ------------YPLT | FGGGT KVEIK |
| iPS:392 880 | 21-225_22F9 | VK1\|A30/ JK4 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPLT | FGGGT KVEIK |
| iPS:392 882 | 21-225_23A3 | VK1\|A30/ JK4 | | DIQMTQSPSSLSA SVGDRVTITC | RAS--QGIR-- ----NDLG | WYQQKPGK APKRLIY | A-------- ASSVQS | GVPSRFSGSGSG-- TEFTLTISSLQPEDFAIYFC | LQHNS----------- ------------YPLT | FGGGT KVEIK |
| iPS:392 896 | 21-225_21G7 | VK1\|A30/ JK4 | | DIQMTQSPSSLSA SVGDRVTITC | RAS--QGVR-- ----NDLG | WYQQKPGK APQRLIY | A-------- ASSLQS | GVPSRFNGSGSG-- TEFTLTISSLQPEDFATYYC | LQHSS----------- ------------YPLT | FGGGT KVEIK |
| iPS:392 900 | 21-225_22F2 | VK1\|A30/ JK4 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPLT | FGGGT KVEIK |
| iPS:392 904 | 21-225_22G9 | VK1\|A30/ JK4 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHAS----------- ------------YPLT | FGGGT KVEIK |
| iPS:392 942 | 21-225_30E9 | VK1\|A30/ JK4 | | DIQMTQSPSSLSA SVGDRVTITC | RAS--QDIR-- ----DDLG | WYQQKPGK APRRLIY | G-------- AFSLQS | GVPSRFSGSGSG-- TEFTLTISSLQPEDFATYYC | LQHTS----------- ------------YPPT | FGGGT KVEIK |
| iPS:392 944 | 21-225_31H5 | VK1\|A30/ JK4 | | DIQMTQSPSSLSA SVGDRFTISF | RAS--QDIR-- ----SDLG | WYQQKPGK AHKRIIY | A-------- ASSLQS | GVPSRFSVSGSG-- TEFTFTISSMQPDDFSNYYC | IQHII----------- ------------YPPT | FGGGT KVEIK |
| iPS:392 964 | 21-225_31A8 | VK1\|A30/ JK4 | | DIQMTQSPSSLSA SVGDRVTITC | RAS--QDIR-- ----SDLG | WYQQKPGK APKRLIY | A-------- VSSLQS | GVPSRFSGSGSG-- TEFTLTISSLQPEDFATYYC | LQHTI----------- ------------YPPT | FGGGT KVEIK |
| iPS:392 980 | 21-225_29H6 | VK1\|A30/ JK4 | | DIQMTQSPSSLSA SVGDRVTITC | RAS--QGIR-- ----NDLG | WYQQKSGK TPKRLIY | A-------- ASSLQS | GVPSRFSGSGSG-- TEFTLTISSLQPEDFATYYC | LQHNS----------- ------------YPLT | FGGGT KVEIK |
| iPS:392 982 | 21-225_30D1 | VK1\|A30/ JK4 | | DIQMTQSPSSLSA SVGDRVTITC | RAS--QDIR-- ----SDLG | WYQQKPGK APERLIY | A-------- ASSLQS | GVPSRFSGSGSG-- TEFTLTISSLQPEDFTTYYC | LQHTI----------- ------------YPPT | FGGGT KVEIK |
| iPS:392 986 | 21-225_31B8 | VK1\|A30/ JK4 | | DIQMIQSPSSLSA SVGDRVTITC | RAS--QDIR-- ----SDLG | WYQQKPGK APKRLIY | G-------- ASSLQS | GVPSRFSGSGSG-- TEFTLTISSLQPEDFATYYC | LQHII----------- ------------YPPT | FGGGT KVEIK |

| | | | | | | | | | | |
|---|---|---|---|---|---|---|---|---|---|---|
| **iPS:392 988** | **21-225_25E6** | **VK1\|A30/ JK4** | | DIQMTQSPSSLSA<br>SVGDRVTITC | RAS--QGIR--<br>----NDLG | WYQRKPGK<br>APKRLIY | A--------<br>ASSLQS | GVPSRFRGSGSG--<br>TEFTLTINSLQPEDFATYYC | LQHNS-----------<br>------------YPLT | FGGGT<br>KVEIK |
| **iPS:392 990** | **21-225_25H10** | **VK1\|A30/ JK4** | | DIQMTQSPSSLSA<br>SVGDRVTITC | RAS--RGIR--<br>----NDLG | WYQQKPGK<br>APKRLIY | A--------<br>AFSLQS | GVPSRFSGSGSG--<br>TEFTLTISSLQPEDFATYYC | LQHNS-----------<br>------------YPLT | FGGGT<br>KVEIK |
| **iPS:393 004** | **21-225_30G11** | **VK1\|A30/ JK4** | | DIQMTQSPSSLSA<br>SVGDRVTITC | RAS--QDIR--<br>----SDLG | WYQQKPGK<br>APKRLIY | A--------<br>ASSLQS | GVPSRFSGSGSG--<br>TEFTLTIGSLQPEDFATYFC | LQHTI-----------<br>------------YPPT | FGGGT<br>KVEIK |
| **iPS:393 018** | **21-225_29B8** | **VK1\|A30/ JK4** | | DIQMTQSPSSLSA<br>SVGDRVTITC | RAS--QGIR--<br>----NDLG | WYQQKPGK<br>APKRLIY | A--------<br>ASSLQS | GVPSRFSGSRSG--<br>TEFTLTISSLQPEDFATYYC | LQHNS-----------<br>------------YPLT | FGGGT<br>KVEIK |
| **iPS:393 030** | **21-225_25H11** | **VK1\|A30/ JK4** | | DIQMTQSPSSLSA<br>SVGDRVTITC | RAS--QGIR--<br>----TDLG | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVEIK |
| **iPS:393 034** | **21-225_27F2** | **VK1\|A30/ JK4** | | DIQMTQSPSSLSA<br>SVGDRVTITC | RAS--QGIR--<br>----NDLG | WYQQKPGK<br>APKRLIY | V--------<br>ASSLQS | GVPSRFSGSGSG--<br>TEFTLTISSLQPEDFATYYC | LQHNS-----------<br>------------YPLT | FGGGT<br>KVEIK |
| **iPS:393 040** | **21-225_30E3** | **VK1\|A30/ JK4** | | DIQMTQSPSSLSA<br>SVGDRVTITC | RAS--QDIR--<br>----DDLG | WYQQKPGK<br>APRRLIY | A--------<br>AFSLQS | GVPSRFSGSGSG--<br>TEFTLTISSLQPEDFATYYC | LQHTS-----------<br>------------YPPT | FGGGT<br>KVEIK |
| **iPS:393 048** | **21-225_27C3** | **VK1\|A30/ JK4** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNR-----------<br>------------YPLT | FGGGT<br>KVEIK |
| **iPS:393 054** | **21-225_29G8** | **VK1\|A30/ JK4** | | DIQMTQSPSSLSA<br>SVGDRVTITC | RAS--QGIR--<br>----NDLG | WYQLKPGK<br>APKRLIY | A--------<br>ASSLQS | GVPSRFSGSGSG--<br>TEFTLTISSLQPEDFATYNC | LQHNS-----------<br>------------YPLT | FGGGT<br>KVEIK |
| **iPS:393 056** | **21-225_30F3** | **VK1\|A30/ JK4** | | DIQMTQSPSSLSA<br>SVGDRVTITC | RAS--QGIR--<br>----NDLG | WYQQKPGK<br>APKRLIY | T--------<br>ASSLQS | GVPSRFSGSGSG--<br>TDFTLTISSLQPEDFASYYC | LQHNS-----------<br>------------YPFT | FGGGT<br>KVEIK |
| **iPS:393 058** | **21-225_31H3** | **VK1\|A30/ JK4** | | DIQMTQSPSSLSA<br>SVGDRVTITC | RAS--QDIR--<br>----DDLG | WYQQKPGK<br>APRRLIY | A--------<br>AFSLQS | GVPSRFSGSGSG--<br>TEFTLTISSLQPEDFATYYC | LQHTS-----------<br>------------YPPT | FGGGT<br>KVEIK |
| **iPS:393 060** | **21-225_32G12** | **VK1\|A30/ JK4** | | DIQMTQSPSSLSA<br>SVGDRVTITC | RAS--QDIR--<br>----SDLG | WYQQRPGK<br>APKRLIY | A--------<br>ASSLQS | GVPSRFSGSGSG--<br>TEFTLTISSLQPEDFATYYC | LQHTI-----------<br>------------YPPT | FGGGT<br>KVEIK |
| **iPS:393 068** | **21-225_34G9** | **VK1\|A30/ JK4** | | DIQMTQSPSSLSA<br>SVGDRVTITC | RAS--QDIR--<br>----SDLG | WYQQKPGK<br>APKRLIY | A--------<br>ASSLQS | GVPSRFTGSGSG--<br>TEFTLTISSLQPEAFAIYYC | LQHTI-----------<br>------------YPPT | FGGGT<br>KVWIK |
| **iPS:393 072** | **21-225_36C5** | **VK1\|A30/ JK4** | | DIQMTQSPSSLSA<br>SVGDRVTITC | RAS--QDIR--<br>----SDLG | WYQQKPGK<br>APKRLIY | A--------<br>ASSLQS | GVPSRFTGSGSG--<br>TEFTLTISSVQPEDFATYYC | LHHPI-----------<br>------------YPPT | FGGGT<br>KVWIK |
| **iPS:393 074** | **21-225_33B1** | **VK1\|A30/ JK4** | | DIQMTQSPSSLSA<br>SVGDRVTITC | RTS--QGIR--<br>----NDLG | WFQQKPGK<br>APKRLIY | T--------<br>ASSLQS | GVPSRFSGSGSG--<br>TEFTLTISSLQPEDFATYYC | LQHNS-----------<br>------------YPLT | FGGGT<br>KVEVK |
| **iPS:393 076** | **21-225_33A4** | **VK1\|A30/ JK4** | | DIQMTQSPSSLSA<br>SVGDRVTITC | RAS--QDIR--<br>----NDVG | WYQQKPGK<br>APERLIY | A--------<br>ASSLQR | GVPSRFSGSGSG--<br>TEFTLTISSLQPEDFARYYC | LQHYS-----------<br>------------YPPT | FGGGT<br>KVEIK |
| **iPS:393 096** | **21-225_34D11** | **VK1\|A30/ JK4** | | DIQMTQSPSSLSA<br>SVGDRVTITC | RAS--QDIR--<br>----SDLG | WYQQKPGK<br>APKRLIY | A--------<br>ASSLQS | GVPSRFSGSGSG--<br>TEFTLTVISLQPEDFATYYC | LQHTI-----------<br>------------YPPT | FGGGT<br>KVGIK |
| **iPS:393 102** | **21-225_33F1** | **VK1\|A30/ JK4** | | DIQMTQSPSSLSA<br>SVGDRVTITC | RTS--QDIR--<br>----SDLG | WYQQKPGK<br>APKRLIY | A--------<br>ASSLQS | GVPSRFSGSGSG--<br>TEFTLTISSLQPEDFAIYYC | LQHTI-----------<br>------------YPPT | FGGGT<br>KVEIK |
| **iPS:393 104** | **21-225_33A7** | **VK1\|A30/ JK4** | | DIQMTQSPSSLSA<br>SVGDRVTITC | RAS--QDIR--<br>----SDLG | WYQQKPGK<br>APKRLIY | V--------<br>ASSLQS | GVPSRFSGSGSG--<br>TEFTLTISSLQPEDFAAYYC | LQHTI-----------<br>------------YPPT | FGGGT<br>KVEIK |
| **iPS:393 106** | **21-225_34A6** | **VK1\|A30/ JK4** | | DIQMTQSPSSLSA<br>SVGDRVTITC | RTS--QDIR--<br>----NDLG | WYQQKPGK<br>APKRLIF | A--------<br>ASSLQS | GVPSRFSGSGSG--<br>TEFTLTVSSLQPEDFATYYC | LQHNS-----------<br>------------YPPT | FGGGT<br>KVEIK |
| **iPS:393 110** | **21-225_35B7** | **VK1\|A30/ JK4** | | DIQMTQSPSSLSA<br>SVGDRITITC | RAS--QDIR--<br>----SDLG | WYQQKPGK<br>APERLIY | A--------<br>ASSLQS | GVPSRFSGSGSG--<br>TEFTLTISSLQPEDFATYYC | LQHTI-----------<br>------------YPPT | FGGGT<br>KVEIK |
| **iPS:393 118** | **21-225_34H11** | **VK1\|A30/ JK4** | | DIQMTQSPSSLSA<br>SVGDRVTITC | RAS--QDIR--<br>----NDLG | WYQQKPGK<br>APKRLIY | A--------<br>TSSLQS | GVPSRFSGSGSG--<br>TEFTLTISSLQPEDFATYYC | LQHNS-----------<br>------------YPPT | FGGGT<br>KVEIK |
| **iPS:393 124** | **21-225_33G7** | **VK1\|A30/ JK4** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHYS-----------<br>------------YPPT | FGGGT<br>KVEIK |

| | | | | | | | | | | |
|---|---|---|---|---|---|---|---|---|---|---|
| iPS:393 126 | 21-225_35D1 | VK1\|A30/ JK4 | | DIQMTQSPSSLSA<br>SVGDRVTITC | RAS--QDIR--<br>----SDLG | WYQQKPGK<br>APKRLIY | A--------<br>ASSLQS | GVPSRFSGSGSG--<br>TEFTLTISSLQPEDFATYYC | LQHTI-----------<br>------------YPPT | FGGGT<br>KVEIK |
| iPS:393 128 | 21-225_35F11 | VK1\|A30/ JK4 | | DIQMTQSPSSLSA<br>SVGDRVTITC | RAS--QDIR--<br>----SDLG | WYQQKPGK<br>APKRLIY | A--------<br>ASSLQS | GVPSRFSGSGSG--<br>TEFTLTISSLQPEDFATYYC | LQHTV-----------<br>------------YPPT | FGGGT<br>KVEIK |
| iPS:393 146 | 21-225_34G8 | VK1\|A30/ JK4 | | DIQMTQSPSSLSA<br>SVGDRVTITC | RAS--QDIR--<br>----SDLG | WYQQKPGK<br>APKRLIY | A--------<br>ASSLQS | GVPSRFSGSGSG--<br>TEFTLTISSLQPEDFATYYC | LQHTI-----------<br>------------YPPT | FGGGT<br>KVEIK |
| iPS:393 150 | 21-225_36A5 | VK1\|A30/ JK4 | | DIQMTQSPSSLSA<br>SVGDRVTITC | RTS--QDIR--<br>----NDLG | WYQQKPGK<br>APKRLIF | A--------<br>ASSLQS | GVPSRFSGSGSG--<br>TEFTLTISSLQPEDFATYYC | LHHNS-----------<br>------------YPPK | FGGGI<br>KVEIT |
| iPS:393 804 | 21-225_5H7 | VK1\|A30/ JK4 | | DIQMTQSPSSLSA<br>SVGDRVTITC | RAS--QGIR--<br>----NDLG | WYQQKPGK<br>APKRLIY | V--------<br>TSSLQG | GVPSRFSGSGSG--<br>TEFTLTISSLQPEDFATYYC | LQHNS-----------<br>------------YPLT | FGGGT<br>KVEIK |
| iPS:393 806 | 21-225_6A6 | VK1\|A30/ JK4 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHSS-----------<br>------------YPLT | FGGGT<br>KVEIK |
| iPS:393 808 | 21-225_1A2 | VK1\|A30/ JK4 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------HPLT | FGGGT<br>KVEIK |
| iPS:393 814 | 21-225_7F4 | VK1\|A30/ JK4 | | DIQMTQSPSSLSA<br>SVGDRVTITC | RTS--QGIR--<br>----NDLG | WYQQKPGK<br>APKRLIY | A--------<br>ASNLQS | GVPSRFSGSGSG--<br>TEFTLTISSLQPEDFATYYC | LQHSA-----------<br>------------YPLT | FGGGT<br>KVEIK |
| iPS:393 816 | 21-225_6D4 | VK1\|A30/ JK4 | | DIQMTQSPSSLSA<br>SVGDRVTITC | RAS--QAIR--<br>----NDLG | WYQQKPGK<br>APKRLIY | A--------<br>ASSLQS | GVPSRFSGSGSG--<br>TEFTLTISSLQPEDFATYYC | LQHSS-----------<br>------------YPLT | FGGGT<br>KVEIK |
| iPS:393 818 | 21-225_6G12 | VK1\|A30/ JK4 | | DIQMTQSPSSLSA<br>SVGDRVTITC | RAS--QGIR--<br>----NDLG | WYQQKPGK<br>APKRLIY | A--------<br>ASTLQS | GVPSRFSGSGSG--<br>TEFTLTISSLQPEDFATYYC | LQHNS-----------<br>------------YPLT | FGGGT<br>KVEIK |
| iPS:393 820 | 21-225_8H7 | VK1\|A30/ JK4 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPFT | FGGGT<br>KVEIK |
| iPS:393 826 | 21-225_10G5 | VK1\|A30/ JK4 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVEIK |
| iPS:393 828 | 21-225_10H12 | VK1\|A30/ JK4 | | DIQMTQSPSSLSA<br>SVGDRVTITC | RAS--QGIR--<br>----NDLG | WYQQKPGK<br>APKRLIY | G--------<br>ASSLQS | GVPSRFSGSGSG--<br>TEFTLTISSLQPEDFATYYC | LQHNS-----------<br>------------YPLT | FGGGT<br>KVEIK |
| iPS:393 830 | 21-225_12A1 | VK1\|A30/ JK4 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVEIK |
| iPS:393 832 | 21-225_14B2 | VK1\|A30/ JK4 | | DIQMTQSPSSLSA<br>SVGDRVTITC | RAS--QGIR--<br>----NDLG | WYQQKPGK<br>APKRLIY | V--------<br>TSSLQG | GVPSRFSGSGSG--<br>TEFTLTISSLQPEDFATYYC | LQHNS-----------<br>------------YPLT | FGGGT<br>KVEIK |
| iPS:393 854 | 21-225_7H11 | VK1\|A30/ JK4 | | DIQMTQSPSSASA<br>SVGVRVTIIC | LAS--QGIR--<br>----NDLG | WYQQKPGK<br>APKRIIY | V--------<br>ACSFQS | GVPSRFSGSGYG--<br>TEFTLTISIMQPEDFATYYC | LQHNL-----------<br>------------YPLT | FGGGT<br>KVEIK |
| iPS:393 856 | 21-225_14C2 | VK1\|A30/ JK4 | | DIQMIQSPSSLFA<br>CVGDRVIITC | RAS--QGIR--<br>----NDLD | WYQQKPGK<br>APKRLIY | A--------<br>ASSLQS | GVPSRFSGSGSG--<br>TEFTLTISSVQREDFATYYC | VQHNS-----------<br>------------YPLT | FGGGT<br>KVEIR |
| iPS:393 866 | 21-225_14E3 | VK1\|A30/ JK4 | | DIQMTQSPSSLSA<br>SVGDRVTITC | RAS--QGIR--<br>----NDLG | WYQQKPGK<br>APKRIIY | S--------<br>ASSLQS | GVPSRFIGSGCG--<br>TEFTLTISSLQREDFAAYYS | VQHYS-----------<br>------------YPFT | FGGGT<br>KVEIK |
| iPS:393 872 | 21-225_2A11 | VK1\|A30/ JK4 | | DIQMTQSPSSLSP<br>SVGDRVTITC | RAS--QGIR--<br>----NDLG | WYQQKPGK<br>APQRLIS | A--------<br>ASSLQS | GVPSRFSGSGSG--<br>TDFTLTISSLQPEDFATYYC | LQHNS-----------<br>------------YPLT | FGGGT<br>KMEIK |
| iPS:393 874 | 21-225_4C8 | VK1\|A30/ JK4 | | DIQMIQSPSFLFA<br>CVGDRVTITC | RAS--QGIR--<br>----NDLG | WYQQKPGK<br>APKRLIY | A--------<br>ASSLQS | GVPSRFSGSVSG--<br>TEFTLTISSLQPEDFATYYC | LQHNS-----------<br>------------YPLT | FGGGT<br>KVEIK |
| iPS:393 880 | 21-225_15A1 | VK1\|A30/ JK4 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTVSSLQPEDFASYYC | LHNSS-----------<br>------------YPVK | FGGGI<br>KVEIT |
| iPS:393 882 | 21-225_15E3 | VK1\|A30/ JK4 | | DIQMTQSPSSLSA<br>SVGDRVTITC | RAS--QGIR--<br>----NDLG | WYQQKPGK<br>APKRLIY | A--------<br>ASSSQS | GVPSRFSGSRSG--<br>TEFTLTISSLQPEDLAAYYC | LQHHS-----------<br>------------YPLT | FGGGT<br>EVEIY |
| iPS:393 884 | 21-225_16F4 | VK1\|A30/ JK4 | | DIQMIQSPSSPSA<br>SVGDRVTITC | RAS--QGIR--<br>----NDLG | WYQQKSGK<br>APKRLIY | V--------<br>ASSLQS | GVPSRFSGSGSG--<br>TEFTITISSVQPEDFATYYC | IQHNS-----------<br>------------YPFT | FGGGT<br>KVEIK |

| | | | | | | | | | | |
|---|---|---|---|---|---|---|---|---|---|---|
| iPS:393 886 | 21-225_2G9 | VK1\|A30/JK4 | | DIQMTQSPSSLFASVGDRVTITC | RAS--QGIR------NDLG | WYQQKPGKAPQRLIY | A--------ASSLQS | GVPSRFSGSGFG--TEFTLTISSLQLEDFATYYC | LQHES-----------------------YPLT | FGGGTKVEIK |
| iPS:393 922 | 21-225_2B2 | VK1\|A30/JK4 | | DIQMTQSPSSLSASVGDRVTITC | RAS--QGIR------NDLG | WYQQKPGQAPKRLIY | A--------ASSLQS | GVPSRFSGSGSG--TEFTLTISSLQPEDLATYHC | LQHNS-----------------------YPLT | FGGGTKVEIK |
| iPS:393 928 | 21-225_4E10 | VK1\|A30/JK4 | | DIQMTQSPSSLFASVGDRVTITC | RAS--QGIR------NDLG | WYQQKPGKAPKRLIY | A--------ASSLQS | GVPSRFSGSGFG--TEFTLTISSLQLEDFATYYC | LQHDN-----------------------YPLT | FGGGTKVEIK |
| iPS:393 934 | 21-225_13E6 | VK1\|A30/JK4 | | DIQVTQSPSSLSASVGDRVTITS | RAS--QGIR------NDLG | WYQQKPGKAPKRLIY | A--------ASSLQS | GVPSRFSGSGSG--TEFTLTISSLQPEDFATYYC | VQHNS-----------------------YPLT | FGGGTKVAIK |
| iPS:393 958 | 21-225_5H2 | VK1\|A30/JK4 | | DIQMTQSPSSLSASVTDRVTITC | RAS--QGIR------NDLG | WYQQKPGKAPKRLIY | A--------ASSLQS | GVPSRFSGSGSG--TEFSLTISSLQPEDFATYYC | LQHNS-----------------------YPLT | FGGGTKVEIK |
| iPS:393 962 | 21-225_7H7 | VK1\|A30/JK4 | | DIQMTQSPSSLSASVGDRVTITC | RAS--QGIR------NDLG | WYQQKPGKAPKRLIY | A--------ASSLQS | GVTSRFSGSGSG--TEFTLTISSLQPEDFATYYC | LQHSS-----------------------YPFT | FVGGTKVEIK |
| iPS:393 974 | 21-225_7C4 | VK1\|A30/JK4 | | DIQMTQSPSSLFASVGDRVTITC | RAS--QGIR------NDLG | WYQQKPGKAHKRLIY | A--------ASSLQS | GVPSRFSGSGSG--TEFTLTISSLQPEDFATYYC | LQHNS-----------------------YPLT | FGGGTKVEIK |
| iPS:393 976 | 21-225_7E9 | VK1\|A30/JK4 | | DIQMTQSPSSLFASVGDRVTITC | RAS--QGIR------NDLG | WYEQKPGKAPKRLIY | T--------ASSLQS | GVPSRFSGSGSG--TEFTLTISSLQPEDFATYYC | LQHNS-----------------------YPLT | FGGGTKVEIK |
| iPS:393 982 | 21-225_6C12 | VK1\|A30/JK4 | | DIQMTQSPSSLSASVGDRGTITC | RAS--QGIR------SNLG | WYQQKPGKAPKRLIY | A--------ASSLES | GVPSRFSGSGSG--TEFTLTISSLQPEDFATYFC | LQDNS-----------------------YPFT | FGGGTKVEIK |
| iPS:393 984 | 21-225_4F12 | VK1\|A30/JK4 | | DIQMTQSPSSLSASVGDRVTITC | RAS--QGIR------NDLG | WYQQKPGKAPKRLIY | A--------ASSLQS | GVPSRFSGGGSG--TIFTLTISSLQPEDFATYYC | LQHNS-----------------------YALT | FGGGTKVEIK |
| iPS:393 990 | 21-225_11G7 | VK1\|A30/JK4 | | DIQMTQSPSSLSASVGDRVTITC | RAS--QGIR------NDLG | WYQQKPGKAPKRLIY | A--------ASSLQS | GVPSRFSGSGSG--TEFTLTISSLQPEDFATYYC | LQHSN-----------------------YPLT | FGGGTMVEIR |
| iPS:393 994 | 21-225_8C9 | VK1\|A30/JK4 | | DIQMTQSPSSLSASIGDRVTITC | RAS--QAIR------NDLD | WYQQKPGKAPKRLIY | A--------ASSLQS | GVPSRFSGSGSG--TEFTLTISSLQTEDFATYYC | LQHNS-----------------------YPLT | FGGGTKVEIK |
| iPS:394 002 | 21-225_15G7 | VK1\|A30/JK4 | | DIQMTQSPSSLSASVGDRVTITC | RAS--QGIR------NDLG | WYQQKPGKAPKRLIY | A--------ASSLQS | GIPSRFSGSGFG--TEFTLTISSLQPEDFATYYC | LQHSN-----------------------YPLT | FGGGTKVEIK |
| iPS:394 008 | 21-225_15H8 | VK1\|A30/JK4 | | DIQMTQSPSSLSASVGDRVTITC | RAS--QGIR------NDLG | WYQQKPGKAPKRLIF | A--------ASSLQS | GVPSRFSGSGSG--TEFTLTISSLQPEDFATYYC | LQHNS-----------------------YPLT | FGGGTKVEIR |
| iPS:394 020 | 21-225_15H10 | VK1\|A30/JK4 | | DIQMTQSPSSLSASVGDRVTITC | RAS--QGIR------NDLG | WYQQKPGKAPKRLIY | A--------ASSLQS | GVPSRFSGSGSG--TEFTLTISSLQPEDFASYYC | LQHNS-----------------------YPLT | FGGGTKVEIN |
| iPS:394 024 | 21-225_16B7 | VK1\|A30/JK4 | | DIQMTQSPSSLSASVGDRVTITC | RAS--QGIR------NDLG | WYQQKPGKAPKRLIY | A--------ASSLQS | GVPSRFSGSGSG--TEFTLTISSLQPEDFATYYC | LQHNS-----------------------YPLT | FGGGTKVEFN |
| iPS:394 037 | 21-225_4F4 | VK1\|A30/JK4 | | DIQMTQSPSSLSASVGDRVTITC | RAS--QGIR------NDLG | WYQQKPGKAPKRLIY | A--------ASSVQT | GVPSRFSGSGSG--TEFTLTISSLQPEDFAIYYC | LQHNS-----------------------YPLT | FGGGTKVEIK |
| iPS:394 045 | 21-225_4H4 | VK1\|A30/JK4 | | DIQMTQSPSSLSASVGDRVTITC | RAS--QGIR------NDLG | WYQQKPGKAPKRLIY | A--------ASSVQS | GVPSRFSGSGSG--TEFTLTISSLQPEDFAIYYC | LQHNS-----------------------YPLT | FGGGTKVEIK |
| iPS:394 049 | 21-225_13H5 | VK1\|A30/JK4 | | DIQMTQSPSSLSASVGDRVTITC | RAS--QGIR------NDLG | WYQQKPGKAPKRLIY | A--------ASSLQT | GVPSRFSGSGSG--TEFILTISSLQPEDFATYYC | LQHNS-----------------------YPLT | FGGGTKVEIK |
| iPS:394 053 | 21-225_11F10 | VK1\|A30/JK4 | | DIQMTQSPSSLSASVGDRVTITC | RAS--QGIR------NDLG | WYQQKPGKAPKRLIY | A--------ASSLQS | GVPSRFSGSGSG--TEFTLTVSSLQPEDFATYYC | LQHSS-----------------------YPLT | FGGGTKVEIK |
| iPS:394 057 | 21-225_15H1 | VK1\|A30/JK4 | | DIQMTQSPSSLSASVGDRVTITC | RAS--QGIR------NDLG | WYQQKPGKAPKRLIY | T--------ASSLQS | GVPSRFNGSGSG--TEFTLTISSLQPEDFATYYC | LQHSS-----------------------YPLT | FGGGTKVEIK |
| iPS:394 059 | 21-225_9E8 | VK1\|A30/JK4 | | DIQMTQSPSSLFASVGDRVTITC | RAS--QGIR------NDLG | WYQQKPGKAPKRLIY | A--------ASSLQS | GVPSRFSGSGSG--TEFTLTISSLQPEDFATYYC | LQHNS-----------------------YPLT | FGGGTKVEIK |
| iPS:394 063 | 21-225_16A1 | VK1\|A30/JK4 | | DIQMTQSPSSLSASVGDRVTITC | RAS--QGIR------NDLG | WYQQKPGKAPKRLIY | A--------ASSLQS | GVPSRFSGSGSG--TEFTLTISSLQPEDFATYYC | LHHSN-----------------------YPLT | FGGGTKVEIE |

| | | | | | | | | | | |
|---|---|---|---|---|---|---|---|---|---|---|
| **iPS:394<br>073** | **21-<br>225_15C9** | **VK1\|A30/<br>JK4** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTVSSLQPEDFATYYC | LQHTS-----------<br>------------YPLT | FGGGT<br>KVEIK |
| **iPS:394<br>075** | **21-<br>225_8D12** | **VK1\|A30/<br>JK4** | | DIQMTQSPSSLSA<br>SVGDRVTITC | RAS--QGIR--<br>----NDLG | WYQQKPGK<br>APKRLIY | A--------<br>ASSLQS | GVPSRFNGSGSG--<br>TEFTLTISSLQPEDFATYYC | LQHSS-----------<br>------------YPLT | FGGGT<br>KVEIK |
| **iPS:394<br>079** | **21-<br>225_11F5** | **VK1\|A30/<br>JK4** | | DIQMTQSPSSLSA<br>SVGDRVTITC | RAS--QGIR--<br>----NDLG | WYQQKPGK<br>APKRLIY | A--------<br>ASSVQS | GVPSRFSGSGSG--<br>TEFTLTISSLQPEDFAIYYC | LQHNS-----------<br>------------YPLT | FGGGT<br>KVEIK |
| **iPS:394<br>091** | **21-<br>225_13H3** | **VK1\|A30/<br>JK4** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVEIK |
| **iPS:398<br>528** | **21-<br>225_32G1** | **VK1\|A30/<br>JK4** | | DIQMTQSPSSLSA<br>SVGDRVTITC | RAS--QDMR--<br>----SDLG | WYQQKPGK<br>APKRLIY | A--------<br>ASSLQS | GVPSRFSGSGSG--<br>TEFTLTISSLQPEDFATYYC | LQHTI-----------<br>------------SPPT | FGGGT<br>KVEIK |
| **iPS:398<br>534** | **21-<br>225_33B8** | **VK1\|A30/<br>JK4** | | DIQMTQSPSSLSA<br>SVGDRVTITC | RAS--QDIR--<br>----SDLG | WYQQKPGK<br>APKRLIY | A--------<br>ASSLQS | GVPSRFSGSGSG--<br>TEFTLTISSLQPEDFATYFC | LQHTI-----------<br>------------YPPT | FGGGT<br>KVEIK |
| **iPS:398<br>540** | **21-<br>225_35A6** | **VK1\|A30/<br>JK4** | | DIQMTQSPSSLSA<br>SVGDRVTITC | RAS--QDIR--<br>----SDLG | WYQQKPGK<br>APKRLIY | A--------<br>TSSLQS | GVPSRFSGSGSG--<br>TEFTLTISSLQPEDFATYYC | LQHTI-----------<br>------------YPPT | FGGGT<br>KVEIK |
| **iPS:402<br>219** | **21-<br>225_1C12** | **VK1\|A30/<br>JK4** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPLT | FGGGT<br>KVAIK |
| **iPS:403<br>868** | **21-<br>225_19D11** | **VK1\|A30/<br>JK4** | | DIQMTQSPSSLSA<br>SVGDRVTITC | RAS--QGIR--<br>----NDLG | WYQQKPGK<br>APKRLIY | A--------<br>TSSLQS | GVPSRFSGSGSG--<br>TEFTLTISSLQPEDFATYYC | LQYYS-----------<br>------------YPLT | FGGGT<br>EVEIK |
| **iPS:403<br>872** | **21-<br>225_8F11** | **VK1\|A30/<br>JK4** | | DIQMTQSPSSLSA<br>SVGDRVTITC | RAS--QGIR--<br>----SDLG | WFQQKPGK<br>APKRLIF | D--------<br>ASSVQS | GVPSRFSGSGSG--<br>TEFTLTISSLQPEDFAIYYC | LQHYT-----------<br>------------YPLT | FGGGT<br>KVEIK |
| | | **Germline** | | **K_FR1** | **K_CDR1** | **K_FR2** | **K_CDR2** | **K_FR3** | **K_CDR3** | **K_FR4** |
| | **VK2\|A18/JK<br>5** | | 32137 | DIVMTQTPLSLSV<br>TPGQPASISC | KSS--<br>QSLLHSD-<br>GKTYLY | WYLQKPGQ<br>SPQLLIY | E--------<br>VSSRFS | GVPDRFSGSGSG--<br>TDFTLKISRVEAEDVGVYYC | MQGIH-----------<br>------------LPIT | FGQGT<br>RLEIK |
| **iPS:468<br>816** | **21-<br>225_52G8** | **VK2\|A18/<br>JK5** | | DIVMTQTPLSLSV<br>TPGQPASMSC | KSS--<br>QSLLHSE-<br>GKTYLY | WYLQKTGQ<br>PPHLLIY | E--------<br>VSKRLS | GVPDRFSGSGSG--<br>TDFTLKISRMEAEDVGVYYC | MQSMQ-----------<br>------------LPII | FGQGT<br>RLEIK |
| **iPS:434<br>021** | **21-<br>225_49C1** | **VK2\|A18/<br>JK5** | | DIVMTQTPLSLSV<br>TPGQPASISC | KSS--<br>QSLLHRE-<br>GKTYLY | WYLQKPGQ<br>APQFLIF | E--------<br>VSHRFS | GVPDRFSGSGSG--<br>TDFTLKISRVEAEDVGVYYC | MQSIQ-----------<br>------------IPIT | LGQGT<br>RLEIK |
| **iPS:434<br>025** | **21-<br>225_49G3** | **VK2\|A18/<br>JK5** | | DIVMTQTPLSLSV<br>TPGQPASMSC | KSS--<br>QSLLHSE-<br>GKTYLY | WYLQKTGQ<br>PPHLLIY | E--------<br>VSNRLS | GVPDRFSGSGSG--<br>TDFTLKISRMEAEDVGVYFC | MQSMQ-----------<br>------------LPIT | FGQGT<br>RLEIK |
| **iPS:434<br>031** | **21-<br>225_49E7** | **VK2\|A18/<br>JK5** | | DIVMTQTPLSLSV<br>TPGQPAFMSC | KSS--<br>QIFLHSE-<br>GKTYLY | WYLQKTGQ<br>PPHLLIY | E--------<br>VSKRLS | GVPDRFSGSGSG--<br>TDFTLKISRMEAEDVGVYYC | MQSMQ-----------<br>------------LPII | FGQGT<br>RLEIK |
| **iPS:434<br>033** | **21-<br>225_49F9** | **VK2\|A18/<br>JK5** | | DIVMTQTPLSLSV<br>TPGQPASISC | KSN--<br>QSLVHNE-<br>GKTYLY | WYLQKPGQ<br>PPQLLIF | E--------<br>VSNRFS | GVPDRFSGSGSG--<br>TDFTLKISRVEAEDVGVYYC | MQSIQ-----------<br>------------YPIT | FGQGT<br>RLEIK |
| **iPS:434<br>093** | **21-<br>225_52D10** | **VK2\|A18/<br>JK5** | | DVMMTQIPLSLSV<br>TPGQPASISC | KSS--<br>QSLLHSE-<br>GKTYLY | WYLQKPGQ<br>PPQLLIF | E--------<br>VSNRVS | GVPDRFSGRGSG--<br>TDFTLKISRVEAEDVGVYYC | MQSIQ-----------<br>------------YPIT | FGQGT<br>RLEIK |
| **iPS:434<br>151** | **21-<br>225_55C2** | **VK2\|A18/<br>JK5** | | DVMMTQIPLSLSV<br>TPGQPASISC | KSS--<br>QSLVHSE-<br>GKTYLY | WYLQKPGQ<br>PPQLLIF | E--------<br>VSNRVS | GVPDRFSGRGSG--<br>TDFTLKISRVEAEDVGVYYC | MQSIL-----------<br>------------YPIT | FGQGT<br>RLEIK |
| **iPS:434** | **21-** | **VK2\|A18/** | | DIVMTQTPLSLSV | KSS-- | WYLQKPGQ | E-------- | GVPDRFSGSGSG-- | IQSIQ----------- | FGQGT |

| | | | | | | | | | | |
|---|---|---|---|---|---|---|---|---|---|---|
| iPS:435 329 | 21-225_147A8 | VK2\|A18/ JK5 | | DIVMTQTPLSLSV TPGQPASISC | KTS-- QSLLHSE- GKTYLY | WYLQKPGQ PPQLLIY | E-------- VSNRFS | GVPDRFSGSGSG-- TDFTLKISRVEAEDVGVYYC | MQSI------------ ------------QLIT | FGQGT RLEIK |
| iPS:392 924 | 21-225_32H2 | VK2\|A18/ JK5 | | DIVMTQTPLSLSV TPGQPASISC | RSS-- QSLLHSD- GRTYLY | WYLQKPGQ PPQLLIY | E-------- LSNRFS | GVPDRFSGSGSG-- TDFTLKISRVEAEDVGIYSC | LQSIQ----------- ------------YPIT | FGQGT RLEIK |
| | | Germline | | K_FR1 | K_CDR1 | K_FR2 | K_CDR2 | K_FR3 | K_CDR3 | K_FR4 |
| | VK2\|A18/JK 1 | | 32136 | DIVMTQTPLSLSV TPGQPASISC | KSS-- QSLLHSD- GKTYLY | WYLQKPGQ SPQLLIY | E-------- VSSRFS | GVPDRFSGSGSG-- TDFTLKISRVEAEDVGVYYC | MQGIH----------- ------------LPWT | FGQGT KVEIK |
| iPS:468 822 | 21-225_147E1 0 | VK2\|A18/ JK1 | | AIVMTQTPLSLSV TPGQPASISC | KSS-- QRLLHGD- GKTYLY | WYLQKPGQ PPHLLIS | E-------- VSNRFS | GVPDRFSGSGSG-- TDFTLKISRVEAEDVGVYYC | MQSIQ----------- ------------VPWT | FGQGT KVEIK |
| iPS:433 917 | 21-225_43E11 | VK2\|A18/ JK1 | | DIVMTQTPLSLSV TPGQPASISC | KSS-- QSLLHSD- GRTYLY | WYLQKPGQ PPQLLIY | E-------- VSNRFS | GVPDRFSGSGSG-- TDFTLKISRVEAEDVGVYYC | MQSIQ----------- ------------LPWT | FGQGT KVEIK |
| iPS:433 965 | 21-225_46F2 | VK2\|A18/ JK1 | | DIVMTQTPLSLTV TPGQPASISC | KSS-- QSLLHGD- GKTYLY | WYLQKPGQ PPQVLIY | E-------- VSNRFS | GVPDRFSGSGSG-- TDFTLKLSRVEAEDVGVYYC | MQSIQ----------- ------------LPWT | FGQGT KVEIK |
| iPS:433 985 | 21-225_47C1 | VK2\|A18/ JK1 | | DIVMTQTPLSLSV TPGQPASISC | TSS-- QSLLHSE- GKTYLY | WYLQKPGQ PPQLLIY | E-------- VSNRFS | GVPDRFSGSGSG-- TDFTLKISRVEAEDVGVFYC | MQSIQ----------- ------------LPWT | FGQGT KVEIK |
| iPS:433 991 | 21-225_47E7 | VK2\|A18/ JK1 | | DIVMTQTPLSLSV TPGQPASISC | KSS-- QSLLHSD- GRTYLY | WYLQKPGQ PPQLLIY | E-------- VSSRFS | GVPDRFSGSGSG-- TDFALKISRVEAEDVGVYYC | MQSTQ----------- ------------LPWT | FGQGT KAEIK |
| iPS:434 345 | 21-225_64H9 | VK2\|A18/ JK1 | | EIVMTQTPLSLSV TPGQPASISC | KSS-- QSLLHGD- GKTYLF | WYLQKPGQ PPQVLIY | E-------- VSNRLC | GVPDRFSGSGSG-- TDFSLKISRVEAEDVGVYYC | MQSIQ----------- ------------VPWT | FGQGT KVEIT |
| iPS:435 297 | 21-225_146B3 | VK2\|A18/ JK1 | | DIVMTQTPLSLSV TPGQPASISC | KSS-- QSLLHGD- GKTYLY | WYLQKPGQ PPQLLIY | E-------- VSNRFS | GVPDRFSGSGSG-- TDFTLKISRVEAEDVGLYHC | MQSIQ----------- ------------LPWT | FGQGT KVEIK |
| iPS:435 341 | 21-225_148B2 | VK2\|A18/ JK1 | | AIVMTQTPLSLSV TPGQPASISC | KSS-- QSLLHGD- GKTYFY | WYLQKPGQ PPQLLIY | E-------- VSHRFS | GVPDRFSGSGSG-- TDFTLKISRVEAEDVGVYYC | MQSIQ----------- ------------IPWT | FGQGT KVEIK |
| iPS:435 357 | 21-225_148G1 0 | VK2\|A18/ JK1 | | DIVMTQTPLSLSV TPGQPASISC | KSS-- QSLLHGD- GKTYLY | WYLQRPGQ PPQLLIY | E-------- VSNRFS | GVPDRFSGSGSG-- TDFTLKISRVEAEDVGVYYC | MQSIQ----------- ------------LPWT | FGQGT KVEIK |
| iPS:435 365 | 21-225_149F1 | VK2\|A18/ JK1 | | DIVMTQSPLSLFV TPGQPASISY | KSS-- QSLLHGD- GKTYFY | WYLQKPGQ PPQLLIY | E-------- VSHRFS | GVPDRFSGSGSG-- TDFTLKISRVEAEDVGVYYC | MQSIQ----------- ------------IPWT | FGQGT KVEIK |
| iPS:435 413 | 21-225_150B1 1 | VK2\|A18/ JK1 | | DIVMTQTPLSLSV TPGQPASISC | KSS-- QSLVHGD- GKTYLY | WYLQRPGQ PPQLLIY | E-------- VSNRFS | GVPDRFSGSGSG-- TDFTLKISRVEAEDVGVYYC | MQSIQ----------- ------------LPWT | FGQGT KVEIK |
| iPS:435 423 | 21-225_151G5 | VK2\|A18/ JK1 | | DIVMTQTPLSLSV TPGQPASISC | KSS-- QRLLHGD- GKTYLY | WYLQKPGQ PPQLLLY | E-------- VSNRFS | GVPDRFSGSGSG-- TDFTLKISRVEAEDVGVYYC | MQSIQ----------- ------------VPWT | FGQGT KVEIK |

| | | | | | | | | | | | |
|---|---|---|---|---|---|---|---|---|---|---|---|
| **iPS:435 429** | **21-225_151A10** | **VK2\|A18/ JK1** | | DIVMTQTPLSLSV TPGQPASISC | KSS-- QSLLHGD- GKTYLY | WYLQKPGQ PPQLLIY | E-------- VSHRFS | GVPDRFSGSGSG-- TDFTLKISRVEAEDVGVYYC | MQSIQ----------- ------------IPWT | FGQGT KVEIK |
| **iPS:435 441** | **21-225_152F6** | **VK2\|A18/ JK1** | | DIVMTQTPLSLSV TPGQPASISC | KSS-- QSLRHGD- GKTYLT | WYLQRPGQ PPQVLIH | E-------- ISKRFT | GVPDRFSGSGSG-- TDFTLNISRVEAEDVGFYYC | MQSIQ----------- ------------VPWT | FGQGT KVEIK |
| **iPS:435 457** | **21-225_152C11** | **VK2\|A18/ JK1** | | DIVMTQAPLSLSV TPGQPASISC | KSS-- QSLLHGD- GKTYLY | WYLQKPGQ PPQILIY | E-------- VSNRFS | GVPDRFSGSGSG-- TDFTLNISRVEAEDFGFYYC | MQSIQ----------- ------------IPWT | FGQGT KVDIK |
| **iPS:435 463** | **21-225_153D2** | **VK2\|A18/ JK1** | | DIVMTQTPLSLSV TPGQPASISC | KSS-- QSLRHGD- GKTYLT | WYLQRPGQ PPQVLIH | E-------- VSKRFT | GVPDRFSGSGSG-- TDFTLNISRVEAEDVGFYYC | MQSIQ----------- ------------VPWT | FGQGT KVEIK |
| **iPS:435 489** | **21-225_155A5** | **VK2\|A18/ JK1** | | DIVMTQTPLSLSV TPGQPASISC | KSS-- QSLLHGD- GKTYLY | WYLQKPGQ PPQLLIY | E-------- VSNRFS | GVPDRFSGSGSG-- TDFTLKISRVEAEDVGFYYC | MQSIQ----------- ------------VPWT | FGQGT KVEIK |
| **iPS:435 531** | **21-225_157G8** | **VK2\|A18/ JK1** | | DIVMTQTPLSLSV TPGQPASISC | KSS-- QSLLHGD- GKTYLY | WYLQKPGQ SPQLLIY | E-------- ISKRFS | GVPDRFSGSGSG-- TDFTLKISRVEAEDVGVYYC | MQSIQ----------- ------------VPWT | FGQGT KVEIK |
| **iPS:435 577** | **21-225_160B1** | **VK2\|A18/ JK1** | | DIVMTQTPLSLSV TPGQPASISC | KSS-- QSLLHGD- GKTYFY | WYLQKPGQ PPQVLIY | E-------- VSKRFS | GVSERFSGSGSG-- TDFTLKISRVEAEDVGVYYC | MQSIQ----------- ------------IPWT | FGQGT KVEIK |
| **iPS:435 601** | **21-225_160G10** | **VK2\|A18/ JK1** | | AIVMTQTPLSLSV TPGQPASISC | KSS-- QSLLHGD- GKTYLY | WYLQKPGQ PPHLLIY | E-------- VSKRFS | GVPDRFSGSGSG-- TDFTLKISRVEAEDVGLYYC | MQSIQ----------- ------------LPWT | FVQGT KVEIT |
| **iPS:435 629** | **21-225_162H6** | **VK2\|A18/ JK1** | | DIVMTQTPLSLSV TPGQPASISC | KST-- QSLLHGD- GKTYLY | WYLQKPGQ PPQLLIY | E-------- VSKRFS | GVPERFSGSGSG-- TDFTLKISRVEAEDVGVYYC | KQSIQ----------- ------------LPWT | FGQGT KVEIK |
| **iPS:435 655** | **21-225_167E2** | **VK2\|A18/ JK1** | | DIVMTQTPLSLSV TPGQPASISC | KSS-- QSLLHGD- GKTYLY | WYLQKPGQ PPHLLIY | E-------- VSKRFS | GVPDRFSGSGSG-- TDFTLKISRVEAEDVGLYHC | MQSIQ----------- ------------LPWT | FGQGT KVEIK |
| **iPS:435 657** | **21-225_167H10** | **VK2\|A18/ JK1** | | DIVMTQTPLSLSV TPGQPASISC | KSS-- QSLLHGD- GKTYLY | WYLQKPGQ PPHLLIY | E-------- VSKRFS | GVPDRFSGSGSG-- TDFTLKISRVEAEDVGLYHC | MQSIQ----------- ------------LPWT | FGQGT KVEIK |
| **iPS:435 683** | **21-225_170A1** | **VK2\|A18/ JK1** | | EIVMTQTPLFLSV TPGQPASISC | KSS-- QSLLHGD- GKTYLF | WYLQKPGQ PPQVLIY | E-------- VSNRFS | GVPDRFSGSGSG-- TDFTLKISRVEAEDVGVYYC | MQSIQ----------- ------------LPWT | FGQGT KVEIK |
| **iPS:435 723** | **21-225_172B7** | **VK2\|A18/ JK1** | | DIVMTQTPLSLSV TPGQPASISC | KSS-- QSLLHGD- GKTYFY | WYLQKPGQ PPQVLLF | E-------- VSHRFS | GVPDRFSGGGSG-- TDFTLKISRVEAEDVGVYYC | MQSIQ----------- ------------FPWT | FGQGT RVDIK |
| **iPS:435 731** | **21-225_173A11** | **VK2\|A18/ JK1** | | DIVMTQTPLSLSV TPGQPASISC | KSS-- QSLLHGD- GKTYLY | WYLQKPGQ PPQLLIY | E-------- VSNRFS | GVPDRFSGSGSG-- TDFTLKISRVEAEDVGVFFC | MQSIQ----------- ------------VPWT | FGQGT KVEIK |
| **iPS:435 755** | **21-225_176H4** | **VK2\|A18/ JK1** | | DIVMTQTPLSLSV TPGQPASISC | KSS-- QSLLHGD- GKTYLY | WYLQKPGQ PPQLLIY | E-------- VSNRFS | GVPDRFSGSGSG-- TDFTLKISRVEAEDVGIYYC | MQSIQ----------- ------------IPWT | FGQGT RVEIK |
| **iPS:435** | **21-** | **VK2\|A18/** | | DIVMTQTPLSLSV | KSS-- | WYLQKPGQ | E-------- | GVPDRFSGSGSG-- | MQSIQ----------- | FGQGT |

| | | | | | | | | | | |
|---|---|---|---|---|---|---|---|---|---|---|
| **iPS:435 781** | **21-225_178G10** | **VK2\|A18/ JK1** | | HIVMTQTPLSLSV TPGQPASISC | KSS-- QSLLHGD- GKTYLY | WYLQKPGQ PPQLLIY | E-------- VSNRFS | GVPDRLSGGGSG-- TDFTLKISRVEAEDVGIYYC | MQSIQ----------- ------------VPWT | FGQGT KVEIK |
| **iPS:435 789** | **21-225_180C4** | **VK2\|A18/ JK1** | | DIVMTQTPLSLSV TPGQPASISC | KSS-- QSLLHGD- GKTYLY | WYLQKPGQ PPQLLIY | A-------- TSNRFP | GVSDRFSGSGSG-- TDFTLKISRVEAEDVGVYYC | MQSIQ----------- ------------VPWT | FGQGT KVEIK |
| **iPS:435 795** | **21-225_181C2** | **VK2\|A18/ JK1** | | EIVMTQTPLSLSV TPGQPASISC | KSS-- QSLLHGD- GKTYLY | WYLQKPGQ PPQLLIH | E-------- VSNRFS | GVPDRFSGSGSG-- TDFTLKISRVEAEDVGVYYC | MQSIQ----------- ------------VPWT | FGQGT KVEIK |
| **iPS:435 807** | **21-225_181C10** | **VK2\|A18/ JK1** | | DIVMTQTPLSLSV TPGQPASISC | KSS-- QSLLHGD- GKTYLY | WYLQKPGQ PPQLLIY | E-------- VSNRFS | GVPDRFSGSGSG-- TDFTLKISRVEAEDVGVYHC | MQSIQ----------- ------------IPWT | FGQGT KVEIK |
| **iPS:435 827** | **21-225_190H11** | **VK2\|A18/ JK1** | | DIVMTQTPLSLSV TPGQPASISC | KSS-- QSLLHSD- GRTYLY | WYLQKPGQ PPQLLIC | E-------- VSNRFA | GVTDRFSGSGSG-- TDFTLKISRVEAGDVGVYYC | MQSIQ----------- ------------FPWT | FGQGT KVEIK |
| **iPS:435 839** | **21-225_191B1** | **VK2\|A18/ JK1** | | DIVMTQTPLSLSV TPGQPASISC | RSS-- QSLLHSD- GKTYLF | WYLQKPGQ PPQVLIY | E-------- LSNRFS | GVPDRFSGSGSG-- TDFTLKISRVEAEDVGVYYC | MQSFQ----------- ------------LPWT | FGQGT KVEIN |
| **iPS:435 853** | **21-225_191E3** | **VK2\|A18/ JK1** | | DIVMTQTPLSLSV TPGQPASISC | KSS-- QSLLHSD- GRTYLY | WYLQKPGQ PPQLLIC | E-------- VSNRFA | GVTDRFSGSGSG-- TDFTLKISRVEAGDVGVYYC | MQSIQ----------- ------------LPWT | FGQGT KVEIK |
| **iPS:435 871** | **21-225_191E6** | **VK2\|A18/ JK1** | | DIVMTQTPLSLSV TPGQPASISC | KSS-- QSLLHSD- GRTYLY | WYLQKPGQ PPQLLIC | E-------- VSNRFA | GVTDRFSGSGSG-- TDFTLKISRVEAGDVGIYYC | MQSIH----------- ------------FPWT | FGQGT KVEIK |
| **iPS:435 887** | **21-225_186F7** | **VK2\|A18/ JK1** | | DVVMAQTPLSLSV TPGQPASISC | KSS-- QSLLHGD- GKTYLC | WYLQKPGQ PPQLLIY | E-------- VSKRFS | GVPDRFSGSGSG-- TDFTLKISRVEAEDVGVYYC | MQSIQ----------- ------------VPWT | FGQGT KVEIK |
| **iPS:435 899** | **21-225_188G11** | **VK2\|A18/ JK1** | | DIVMTQTPLSLSV TPGQPASISC | MSS-- QSLLHGD- GKTYLY | WYLQKPGQ PPQLLIY | E-------- VSNRFS | GVPDTFSGSGSG-- TDFTLKISRVEAEDVGVYYC | MQSIQ----------- ------------IPWT | FGQGT KVEIK |
| **iPS:435 901** | **21-225_189G2** | **VK2\|A18/ JK1** | | DIVMTQTPLSLSV TPGQPASISC | KSS-- QSLLHGD- GKTYLF | WYLQKPGQ PPQLLIY | E-------- VSNRFS | GVSDRFSGSGSG-- TDFTLKISRVEAEDVGVYYC | MQSIQ----------- ------------IPWT | FGQGT KVEIK |
| **iPS:435 927** | **21-225_190E7** | **VK2\|A18/ JK1** | | DIVLTQTPLSLSV TPGQPASISC | KSS-- QSLLHSD- GRTYLY | WYLQKPGQ PPQVLIC | E-------- VSNRFS | GVPDRFSGSGSG-- TDFTLKISRVEAGDVGVYYC | MQSIQ----------- ------------LPWT | FGQGT KVEIK |
| **iPS:435 999** | **21-225_192F9** | **VK2\|A18/ JK1** | | DIVMTQTPLSLSV TPGQPASISC | KSS-- QSLLHSD- GRTYLY | WYLQRPGQ PPQLLIC | E-------- VSNRFA | GVTDRFSGSGSG-- TDFTLKISRVEAGDVGVYYC | MQSIQ----------- ------------LPWT | FGQGT KVEIK |
| **iPS:436 060** | **21-225_194F4** | **VK2\|A18/ JK1** | | DIVMTQTPLSLSV TPGQPASISC | KSS-- QSLLHSD- GRTYLY | WYLQKPGQ PPQLLIC | E-------- VSNRFS | GVPDRFSGSGSG-- TDFTLKISRVEAGDVGVYYC | MQSIQ----------- ------------LPWT | FGQGT KVEIK |
| **iPS:436 158** | **21-225_197G8** | **VK2\|A18/ JK1** | | EIVMTQTPLSLSV IPGQPASISC | KSS-- QNLLHSD- GKTYLY | WYLQKPGQ PPQVLIY | E-------- VSNRFS | GVPDRFSGSGSG-- TDFTLKISRVEAEDVGVYSC | MQSIQ----------- ------------LPWT | FAQGS KVEIK |
| **iPS:436** | **21-** | **VK2\|A18/** | | DIVLTQTPLSLSV | KSS-- | WYLQKPGQ | E-------- | GVPDRFSGSGSG-- | MQSIQ----------- | FGQGT |

| | | | | | | | | | | |
|---|---|---|---|---|---|---|---|---|---|---|
| **iPS:436<br>536** | **21-<br>225_224G1** | **VK2\|A18/<br>JK1** | | DIVMTQTPLSLSV<br>TPGQPASISC | KSG--<br>QSLLHSD-<br>GKTFLS | WYLQKPGQ<br>PPQLLIY | E--------<br>ISNRFS | GVPDRFSGSGSG--<br>TDFTLKISRVEAEDVGIFYC | MQSTQ-----------<br>------------LPRT | FGQGT<br>KVEIK |
| **iPS:436<br>548** | **21-<br>225_224A7** | **VK2\|A18/<br>JK1** | | DIVMTQTPLSLSV<br>TPGQPASISC | KSS--<br>QSLLHSD-<br>GKTFLY | WFLQKPGQ<br>PPQLLIY | E--------<br>ISNRFS | GVPDRFSGSGSG--<br>TDFTLKISRVEAEDVGIYYC | MQSTQ-----------<br>------------LPRT | FGQGT<br>KVEIK |
| **iPS:436<br>558** | **21-<br>225_224C1<br>1** | **VK2\|A18/<br>JK1** | | DFVMTQTPLSLSV<br>TPGQPASISC | KSS--<br>QSLLHSD-<br>GKTFLY | WYLQKPGQ<br>PPQLLIY | E--------<br>ISNRFS | GVPDRFSGSGSG--<br>TDFTLKISRVEAEDVGIYYC | MQSTQ-----------<br>------------LPRT | FGQGT<br>KVEIK |
| **iPS:436<br>562** | **21-<br>225_224H1<br>1** | **VK2\|A18/<br>JK1** | | DIVMTQTPLSLSV<br>TPGQPASISC | KSS--<br>QSLLHSD-<br>GKTFLY | WYLQKPGQ<br>PPQLLIY | E--------<br>ISNRFS | GVPDRFSGSGSG--<br>TDFTLKISRVEAEDVGIYYC | MQSTQ-----------<br>------------LPRT | FGQGT<br>KVEIK |
| **iPS:436<br>572** | **21-<br>225_225G4** | **VK2\|A18/<br>JK1** | | DIVMTQTPLSLSV<br>TPGQPASISC | KSS--<br>QSLLHSD-<br>GKTFLY | WYLQKPGQ<br>PPQLLIY | E--------<br>ISNRFS | GVPDRFSGSGSG--<br>TDFTLKISRVEAEDVGIYYC | MQSTQ-----------<br>------------LPRT | FGQGT<br>KVEIK |
| **iPS:436<br>592** | **21-<br>225_226B1** | **VK2\|A18/<br>JK1** | | DIVMTQTPLSLSV<br>TPGQPASISC | KSS--<br>QSLLHGD-<br>GKTYLY | WYLQRPGQ<br>PPQLLIN | E--------<br>VSIRFS | GVPDRFSGSGSG--<br>TDFTLKISRVEAEDVGVYYC | MQSIQ-----------<br>------------IPWT | FGQGT<br>KVDIK |
| **iPS:436<br>594** | **21-<br>225_226A5** | **VK2\|A18/<br>JK1** | | DIVMTQTPLSLSV<br>TPGQPASISC | KSS--<br>QSLLHGD-<br>GKTYLY | WYLQKPGQ<br>PPQLLIY | E--------<br>VSNRFS | GVPDRFSGSGSG--<br>TDFTLKISRVEAEDVGVYYC | MQSIQ-----------<br>------------IPWT | FGQGT<br>KVEIK |
| **iPS:436<br>602** | **21-<br>225_226E7** | **VK2\|A18/<br>JK1** | | DIVMTQTPLSLSV<br>TPGQPASISC | KSS--<br>QSLLHGD-<br>GKTYLY | WYQQKPGQ<br>PPQILIY | E--------<br>VSNRFS | GVPDRFSGSGSG--<br>TDFTLKISRVEAEDVGVYYC | MQSIQ-----------<br>------------VPWT | FGQGT<br>KVEIK |
| **iPS:436<br>606** | **21-<br>225_226G8** | **VK2\|A18/<br>JK1** | | DIVMTQTPLSLSV<br>TPGQPASISC | KSS--<br>QSLLHSD-<br>GKTFLY | WYLQKPGQ<br>PPQLLIY | E--------<br>ISNRFS | GVPDRFSGSGSG--<br>TDFTLKISRVEAEDVGIYYC | MQSTQ-----------<br>------------LPRT | FGQGT<br>KVEIK |
| **iPS:436<br>610** | **21-<br>225_226F9** | **VK2\|A18/<br>JK1** | | DIVMTQTPLSLSV<br>TPGQPASISC | KSS--<br>QSLLHSD-<br>GKTFLY | WYLQKPGQ<br>PPQLLIY | E--------<br>ISNRFS | GVPDRFSGSGSG--<br>TDFTLKISRVEAEDVGIYYC | MQSTQ-----------<br>------------LPRT | FGQGT<br>KVEIK |
| **iPS:436<br>612** | **21-<br>225_226H9** | **VK2\|A18/<br>JK1** | | DIVMTQTPLSLSV<br>IPGQPASISC | KSS--<br>QSLLHSD-<br>GKTFLY | WYLQRPGQ<br>PPQLLIY | E--------<br>VSNRFS | GVPDRFSGSGSG--<br>TDFTLKISRVEAEDVGIYYC | MQSTQ-----------<br>------------LPRT | FGQGT<br>KVEIK |
| **iPS:436<br>614** | **21-<br>225_226F10** | **VK2\|A18/<br>JK1** | | DIVMTQTPLSLSV<br>TPGQPASISC | KSS--<br>QSLLHSD-<br>GKTFLY | WYLQKPGQ<br>PPQLLIY | E--------<br>ISNRFS | GVPDRFSGSGSG--<br>TDFTLKISRVEAEDVGIYYC | MQSTQ-----------<br>------------LPRT | FGQGT<br>KVEIK |
| **iPS:436<br>618** | **21-<br>225_226E1<br>1** | **VK2\|A18/<br>JK1** | | DIVMTQTPLSLSV<br>TPGQPASISC | KSS--<br>QSLLHSD-<br>GKTFLY | WYLHKPGQ<br>PPHLLIY | E--------<br>ISNRFS | GVPDRFSGSGSG--<br>TDFTLKISRVEAEDVGIYYC | MQSTQ-----------<br>------------LPRT | FGQGT<br>KVEIK |
| **iPS:436<br>624** | **21-<br>225_226H1<br>2** | **VK2\|A18/<br>JK1** | | DIVMTQTPLSLSV<br>TPGQPASISC | KSS--<br>KTLLHSD-<br>GKTFLY | WYLQKPGQ<br>PPQPLIY | E--------<br>ISNRFS | GVPDRFSGSGSG--<br>TDFTLKISRVEAEDVGIYYC | MQSTQ-----------<br>------------LPRT | FGQGT<br>KVEIK |
| **iPS:436<br>626** | **21-<br>225_227C1** | **VK2\|A18/<br>JK1** | | DIVMTQTPLSLSV<br>TPGQPASISC | KSS--<br>QSLLHSD-<br>GKTFLY | WYLQKPGQ<br>PPQLLIY | E--------<br>ISNRFS | GVPDRFSGSGSG--<br>TDFTLKISRVEAEDVGIYYC | MQSTQ-----------<br>------------LPRT | FGQGT<br>KVEIK |
| **iPS:436** | **21-** | **VK2\|A18/** | | DIVMTQTPLSLSV | KSS-- | WYLQKPGQ | E-------- | GVPDRFSGSGSG-- | MQSTQ----------- | FGQGT |

| | | | | | | | | | | |
|---|---|---|---|---|---|---|---|---|---|---|
| iPS:436 640 | 21-225_227A8 | VK2\|A18/JK1 | | DIVMTQTPLSLSV TPGQPASISC | KSS-- QSLLHSD-GKTFLY | WYLQKPGQ PPQLLIY | E-------- ISNRFS | GVPDRFSGSGSG-- TDFTLKISRVEAEDVGIYYC | MQSTQ----------- ------------LPRT | FGQGT RVEIK |
| iPS:392 814 | 21-225_22A1 | VK2\|A18/JK1 | | DIVMTQTPLSLSV TPGQPASISC | KSS-- QSLLHSG-GKTYLY | WYLQKPGQ PPQLLIY | E-------- VSNRFS | GLPDRFSGSGSG-- TDFTLKISRVEAADVGVYYC | MQTLH----------- ------------LPWT | FGQGT KVEIK |
| iPS:392 930 | 21-225_25H9 | VK2\|A18/JK1 | | DIVMTQTPLSLSV TPGQPASISC | KSS-- QSLLHGD-GKTYLF | WYLQKPGQ PPQLLIY | E-------- VSNRFS | GVPDRFSGSGSG-- TDFTLKISRVEAEDVGVYYC | MQSIQ----------- ------------LPWT | FGQGT KVEIK |
| iPS:393 032 | 21-225_26F8 | VK2\|A18/JK1 | | DIVMTQTPLSLSV TPGQPASISC | KSS-- QSLLHGD-GKTYLY | WYLQKPGQ PPQLLIY | E-------- VSNRFS | GVPDRFSGSGSG-- TDFTLKISRVEAEDVGVYYC | MQSIQ----------- ------------LPWT | FGQGT KVEIK |
| iPS:393 036 | 21-225_28G3 | VK2\|A18/JK1 | | EIVMTQTPLSLSV TPGQPASISC | KSS-- QSLLHGD-GKTYLY | WYLQKPGQ PPQLLIY | E-------- VSNRFS | GVPERFSGSGSG-- TDFTLKISRVEAEDVGVYYC | MQSIQ----------- ------------IPWT | FGQGT KVEIK |
| | | Germline | | **K_FR1** | **K_CDR1** | **K_FR2** | **K_CDR2** | **K_FR3** | **K_CDR3** | **K_FR4** |
| | VK1\|A30/JK1 | | 32139 | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPWT | FGQGT KVEIK |
| iPS:468 826 | 21-225_201C5 | VK1\|A30/JK1 | | DIQMTQSPSSLSA SVGDRVTITC | RAS--QGIR-- ----HDLG | WYQQKPGK APKRLIY | A-------- ASSLQS | GVPSRFSGSGSG-- TEFTLTISSLQPEDFATYYC | LQHYS----------- ------------FPRT | FGQGT KVEIK |
| iPS:468 842 | 21-225_50H4 | VK1\|A30/JK1 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHYS----------- ------------YPRT | FGQGT KVEIK |
| iPS:468 858 | 21-225_148C9 | VK1\|A30/JK1 | | DIQMTQSPSSLSA SVGDRVTITC | RAS--RGIR-- ----DDLG | WYQQKPGK APKRLIY | A-------- ASSLQS | GVPSRFSGSGSG-- TEFTLTISSLQPEDFATYYC | LQHYS----------- ------------YPRT | FGQGT KVEIK |
| iPS:468 860 | 21-225_224E7 | VK1\|A30/JK1 | | DIQMTQSPSSLSA SVGDRVTITC | RAS--QGIR-- ----NDLG | WYQQKPGK APTRLIY | A-------- ASSLQS | GVPSRFSGSGSG-- TEFTLTISSLQPEDFATYYC | LQHYS----------- ------------YPRT | FGQGT KVEIK |
| iPS:433 919 | 21-225_44B3 | VK1\|A30/JK1 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LLHYN----------- ------------YPRT | FGQGT KVEIK |
| iPS:433 923 | 21-225_44D3 | VK1\|A30/JK1 | | DIQMTQSPSSLSA SVGDRVTFTC | RAS--QGIR-- ----DDLG | WYQQKPGK APKRLIY | A-------- ASSLQS | GVPSRFSGSRSG-- REFTLTISSLQPEDFATYYC | LQHYS----------- ------------YPRT | FGQGT KVEIK |
| iPS:433 929 | 21-225_44D5 | VK1\|A30/JK1 | | DIQMTQSPSSLSA SVGDRVTITC | RAS--QDIR-- ----KDLG | WYQQKPGK APKRLIY | A-------- ASTLES | GVPSRFSGSGSG-- TEFTLTISSLQPEDFATYYC | LQHYS----------- ------------FPWT | FGQGT KVEIK |
| iPS:433 935 | 21-225_44F9 | VK1\|A30/JK1 | | DVQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LHHYN----------- ------------YPRT | FGQGT KVEIT |
| iPS:433 939 | 21-225_44C10 | VK1\|A30/JK1 | | DIQMTQSPSSLSA SVGDRVTFTC | RAS--QGIR-- ----DDLG | WYQQKPGK APKRLIY | A-------- TSSLQS | GVPSRFSGSGSG-- TEFTLTISSLQPEDFATYYC | LQHYS----------- ------------YPRT | FGQGT KVEIK |
| iPS:433 951 | 21-225_45B4 | VK1\|A30/JK1 | | DIQMTQSPSSLSA SVGDRVTFTC | RAS--QGIR-- ----DDLG | WYQQKPGK APKRLIY | A-------- ASSLQS | GVPSRFSGSGSG-- TEFTLTISSLQPEDFATYYC | LQHYS----------- ------------YPRT | FGQGT KVEIK |
| iPS:433 955 | 21-225_45B8 | VK1\|A30/JK1 | | DIQMTQSPSSLSA SVGDRVTFTC | RAS--QDIR-- ----DDLG | WYQQKPGK APKRLIY | A-------- ASSLQS | GVPSRFSGSGSG-- TEFTLTISSLQPEDFATYYC | LQHYN----------- ------------YPRT | FGQGT KVEIK |
| iPS:433 967 | 21-225_46C3 | VK1\|A30/JK1 | | DIQMTQSPSSLSA SVGDRVTFTC | RAS--QGIR-- ----DDLG | WYQQKPGK APKRLIY | A-------- ASSLQS | GVPSRFSGSRSG-- TEFTLTISSLQPEDFATYYC | LQHYS----------- ------------YPRT | FGQGT KVEIK |
| iPS:433 971 | 21-225_46D4 | VK1\|A30/JK1 | | DIQMTQSPSSLSA SVGDRVTITC | RTS--QDIR-- ----KDLG | WYQQKVGK APKRLIY | A-------- ASSLES | GVPSRFSGSGSG-- TEFTLTISSLQPEDFATYYC | LQHYS----------- ------------FPWT | FGQGT KVEIK |
| iPS:433 997 | 21-225_48C1 | VK1\|A30/JK1 | | DIQMTQSPSSLSA SVGDRVTITC | RTS--QGIR-- ----NDLG | WYQQKPGK APKRLIY | R-------- ASSLQS | GVPARFSGSGSG-- TEFTLTISSLQPEDFAIYYC | LQHNF----------- ------------YPWT | FGQGT KVEIK |

| | | | | | | | | | | |
|---|---|---|---|---|---|---|---|---|---|---|
| iPS:434<br>001 | 21-<br>225_48F2 | VK1\|A30/<br>JK1 | | DIQMTQSPSSLSA<br>SVGDRVTITC | RAS--RGIR--<br>----DDLG | WYQQKPGK<br>PPKRLIY | A--------<br>ASSLQS | GVPSRFNGSGSG--<br>TEFTLTISSLQSEDLATYYC | LQQYS-----------<br>------------YPRT | FGQGT<br>KVEIK |
| iPS:434<br>009 | 21-<br>225_48A9 | VK1\|A30/<br>JK1 | | DIQMTQSPSSLSA<br>SVGDRVTITC | RAS--QGIR--<br>----NDLG | WYQQKPGK<br>APKRLIY | I--------<br>ASSLQS | GVPSRFSGSGSG--<br>TEFTLTISGLQPEDFAIYYC | LQHNR-----------<br>------------YPWT | FGQGT<br>KVEIK |
| iPS:434<br>047 | 21-<br>225_50A12 | VK1\|A30/<br>JK1 | | DIQMTQSPSSLSA<br>SVGDRVTITC | RAS--QGIR--<br>----NDLG | WYQQKPGK<br>APKRLIY | T--------<br>ASNLQS | GVPSRFSGSGSG--<br>TEFTLTISSLQPEDFATYYC | LQHNS-----------<br>------------YPWT | FGQGT<br>KVEIK |
| iPS:434<br>067 | 21-<br>225_51H8 | VK1\|A30/<br>JK1 | | DIQMTQSPSSLSA<br>SVGDRVTITC | RAS--QGIR--<br>----NDLG | WYQQKPGK<br>APKRLIY | A--------<br>ASSLQS | GVPSRFSGGGSG--<br>THFTLTISSLQPEDFATYYC | LQHNS-----------<br>------------YPWT | FGQGT<br>KVEIK |
| iPS:434<br>135 | 21-<br>225_54H3 | VK1\|A30/<br>JK1 | | DIQMTQSPSSLSA<br>SVGDRVTITC | RAS--QDIR--<br>----NILG | WYQQKPGK<br>APKRLIY | A--------<br>ASNLQS | GVPSRFSGSGSG--<br>TEFTLTISSLQPEDFATYYC | LQYNS-----------<br>------------YPWT | FGQGT<br>KVEIK |
| iPS:434<br>197 | 21-<br>225_56C7 | VK1\|A30/<br>JK1 | | DIQMTQSPSSLSA<br>SVGDRVTITC | RAS--QGIR--<br>----NDLG | WYQQKPGK<br>APKRLIY | T--------<br>ASNLQS | GVPSRFSGSGSG--<br>TEFTLTISSLQPEDFATYYC | LQHNS-----------<br>------------YPWT | FGQGT<br>KVEIK |
| iPS:434<br>203 | 21-<br>225_60E2 | VK1\|A30/<br>JK1 | | DIQMTQSPSSLSA<br>SVGDRVTITC | RAS--QGIR--<br>----KDLG | WYQQKPGK<br>APKRLIY | A--------<br>ASSLQS | GVPSRFSGSGSG--<br>TEFTLTISSLQPEDFATYYC | LQHNS-----------<br>------------YPWT | FGQGT<br>KVEIK |
| iPS:434<br>209 | 21-<br>225_60C3 | VK1\|A30/<br>JK1 | | DIQMTQSPSSLSA<br>SVGDRVTITC | RAS--QGIR--<br>----NDLG | WYQQKPGK<br>APKRLIY | S--------<br>ASSLQS | GVPSRFSGSGSG--<br>TEFTLTISSLQPEDFATYYC | LQHNS-----------<br>------------YPWT | FGLGT<br>KVEIK |
| iPS:434<br>229 | 21-<br>225_61H1 | VK1\|A30/<br>JK1 | | DIQMTQSPSSLSA<br>SVGDRVTITC | RAS--QGIR--<br>----KDLG | WYQQKPGK<br>APKRLIF | A--------<br>ASSLQS | GVPSRFSGSGSG--<br>TEFTLTISSLQPEDFATYYC | LQIINS-----------<br>------------YPWT | FGQGT<br>KVEIK |
| iPS:434<br>241 | 21-<br>225_61E6 | VK1\|A30/<br>JK1 | | DIQMTQSPSSLSA<br>SVGDRITITC | RAS--QGIG--<br>----NDLG | WYQQKPGK<br>APERLIY | A--------<br>ASSLQS | GVPSRFSGSGSG--<br>TEFTLTISSLQPEDFATYYC | LQHNS-----------<br>-----------FPPWT | FGQGT<br>KVEIK |
| iPS:434<br>257 | 21-<br>225_62F7 | VK1\|A30/<br>JK1 | | DIQMTQSPSSLSA<br>SVGDRVTITC | RAS--QAIR--<br>----NDLG | WYQQKPGK<br>APKRLIY | P--------<br>ASRLQS | GVPSRFSGSGSG--<br>TEFTLTISSLQPEDFATYYC | LQYNS-----------<br>-----------YPPWT | FGQGS<br>KVEIK |
| iPS:434<br>281 | 21-<br>225_57B8 | VK1\|A30/<br>JK1 | | DIQMTQSPSSLSA<br>SVGDRITITC | RAS--QGIG--<br>----NDLG | WYQQKPGK<br>APERLIY | A--------<br>ASSLQS | GVPSRFSGSGSG--<br>TEFTLTISSLQPEDFATYYC | LQHNS-----------<br>-----------FPPWT | FGQGT<br>KVEIK |
| iPS:434<br>315 | 21-<br>225_59G7 | VK1\|A30/<br>JK1 | | DIQMTQSPSSLSA<br>SVGDRVTITC | RAS--QGIR--<br>----NDLG | WYQQKPGK<br>APKRLIY | S--------<br>ASSLQS | GVPSRFSGSGSG--<br>TEFTLTISSLQPEDFATYYC | LQHNS-----------<br>------------YPWT | FGQGT<br>KVEIK |
| iPS:434<br>319 | 21-<br>225_59B9 | VK1\|A30/<br>JK1 | | DIQMTQSPSSLSA<br>SVGDRVTITC | RAS--QDIR--<br>----NDLG | WYQQHPGK<br>APKRLIY | A--------<br>ASSLQS | GVPSRFSGTRSG--<br>TEFTLTISSLQPEDFATYYC | LQHNS-----------<br>------------YPWT | FGQGT<br>KVEIK |
| iPS:434<br>339 | 21-<br>225_64A4 | VK1\|A30/<br>JK1 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDLATYYC | LQHYS-----------<br>------------YPRT | FGQGT<br>KVEIK |
| iPS:434<br>343 | 21-<br>225_64C8 | VK1\|A30/<br>JK1 | | DIQMTQSPSSLSA<br>AVGDRVTITC | RAS--QGIR--<br>----NDLG | WYQQKPGK<br>APKCLIY | A--------<br>ASSLQS | GVPSRFSGSGSG--<br>TEFTLTISSLQPEDFATYYC | LHHYS-----------<br>------------YPRT | FGQGT<br>KVEIK |
| iPS:434<br>385 | 21-<br>225_66C10 | VK1\|A30/<br>JK1 | | DIQMTQSPSSLSA<br>SVGDRVTITC | RAS--QAIR--<br>----NDLG | WYQQKPGK<br>APKRLIY | P--------<br>ASSLQS | GVPSRFSGSGSG--<br>TEFTLTISSLQPEDFATYYC | LQYNS-----------<br>-----------YPPWT | FGQGS<br>KVEIK |
| iPS:434<br>387 | 21-<br>225_66D11 | VK1\|A30/<br>JK1 | | DIQMTQFPSSQSA<br>SVGDRVTITC | RAS--QGIR--<br>----NDLG | WYQQKPGK<br>AHKRLIY | A--------<br>ASSCQS | GVPSRFSGSGCG--<br>TEFTISISSMQREDFATYYC | IVHNS-----------<br>------------YPRT | FGQGT<br>KVEIK |
| iPS:434<br>441 | 21-<br>225_71A2 | VK1\|A30/<br>JK1 | | DIQMTQSPSSRSA<br>SVGDRVTITC | RAS--QGIR--<br>----NDLG | WYQQKPGK<br>APKRLIY | I--------<br>AFRLQI | GVPSRFSGSGSG--<br>TEFTLTISSLQPEDFATYYC | IHHNS-----------<br>------------YPWT | FGQGT<br>KVEIK |
| iPS:434<br>469 | 21-<br>225_73C9 | VK1\|A30/<br>JK1 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHYS-----------<br>------------YPRT | FGQGT<br>KVEIK |
| iPS:435<br>197 | 21-<br>225_94F3 | VK1\|A30/<br>JK1 | | DIQMTQSPSSLSA<br>SVGDRVTITC | RAS--QAIR--<br>----DDLG | WYQQKPGK<br>APQRLIF | A--------<br>ASSLQS | GVPSRFSGSGSG--<br>TEFTLTISSLQPEDFATYYC | LQHYS-----------<br>------------YPRT | FGRGT<br>KVAIK |
| iPS:435<br>325 | 21-<br>225_147H5 | VK1\|A30/<br>JK1 | | DIQMTQSPSSLSA<br>SVGDRVTITC | RAS--RGIR--<br>----DDLG | WYQQKPGK<br>APKRLIY | A--------<br>ASSLQS | GVPSRFSGSGSG--<br>TEFTLTISSLQPEDFATYYC | LQHYS-----------<br>------------YPRT | FGQGT<br>KVEIK |
| iPS:435<br>393 | 21-<br>225_149D1 | VK1\|A30/<br>JK1 | | DIQMTQSPSSLSA<br>SVGDRVTITC | RAS--RGIR--<br>----NDLG | WYQQKPGK<br>APKRLIY | A--------<br>ASSLQS | GVPSRFSGSGSG--<br>TEFTLTISSLQPEDFATYYC | LQHYS-----------<br>------------YPRT | FGQGT<br>KVEIK |

| | | | | | | | | | | |
|---|---|---|---|---|---|---|---|---|---|---|
| **iPS:435<br>539** | **21-<br>225_158G1** | **VK1\|A30/<br>JK1** | | DIQMTQSPSSLSA<br>SVGDRVTITC | RAS--QGIR--<br>----NDLG | WYQQKPGK<br>APKRLIY | T--------<br>ASNLQS | GVPSRFSGSGCG--<br>TEFTLTVSSLQPEDFATYYC | LQHNS-----------<br>------------YPWT | FGQGT<br>KVEIK |
| **iPS:435<br>543** | **21-<br>225_158D4** | **VK1\|A30/<br>JK1** | | DIQMTQSPSSLSA<br>SVGDRVTITC | RAS--QDIR--<br>----KDLG | WYQQKPGK<br>APKRLIY | A--------<br>ASSLQS | GVPSRFSGSGSG--<br>TEFTLTFSSLQPEDFATYYC | LQHYS-----------<br>------------YPRT | FGQGT<br>KVEIK |
| **iPS:435<br>571** | **21-<br>225_159C8** | **VK1\|A30/<br>JK1** | | DIQMTQSPSSLSA<br>SVGDRVTITC | RAS--QDIR--<br>----KDLG | WYQQKPGK<br>APNRLIY | A--------<br>ASSLQS | GVPSRFSGSGSG--<br>TEFTLTFSSLQPEDFATYYC | LQHHS-----------<br>------------YPRT | FGQGT<br>KVEIK |
| **iPS:435<br>573** | **21-<br>225_159D8** | **VK1\|A30/<br>JK1** | | DIQMTQSPSSLSA<br>SVGDRVTITC | RAS--RDIG--<br>----NDLG | WYQQKPGK<br>APKRLIS | A--------<br>ASSLQS | GVPSRFSGSGSG--<br>TEFTLTFSSLQPEDFATYYC | LQHYS-----------<br>------------YPRT | FGQGT<br>KVEIK |
| **iPS:435<br>581** | **21-<br>225_160H1** | **VK1\|A30/<br>JK1** | | DIQMTQSPSSLSA<br>SVGDRVTITC | RAS--QDIR--<br>----NDLG | WYQQKPGK<br>APKRLIY | A--------<br>ASSLQN | GVPSRFSGSGSG--<br>TEFTLTISSLQPEDFTTYYC | LQHNS-----------<br>------------FPWT | FGQGT<br>KVEIK |
| **iPS:435<br>583** | **21-<br>225_160F2** | **VK1\|A30/<br>JK1** | | DIQMTQSPSSLSA<br>SVGDRVTITC | RAS--QGIR--<br>----NDLG | WYQQKPGK<br>APKRLIY | T--------<br>ASNLQS | GVPSRFSGSGSG--<br>TEFTLTVSSLQPEDFATYYC | LQHNS-----------<br>------------YPWT | FGQGT<br>KVEIK |
| **iPS:435<br>591** | **21-<br>225_160C4** | **VK1\|A30/<br>JK1** | | DIQMTQSPSSLSA<br>SVGDRVTITC | RAS--QDIR--<br>----KDLG | WYQQKPGK<br>APNRLIY | A--------<br>ASSLQS | GVPSRFSGSGSG--<br>TEFTLTFSSLQPEDFATYYC | LQHYS-----------<br>------------YPRT | FGQGT<br>KVEIK |
| **iPS:435<br>615** | **21-<br>225_161G1<br>2** | **VK1\|A30/<br>JK1** | | DIQMTQSPSSRSA<br>SVGDRVTITC | RAS--QDIR--<br>----KDLG | WYQQKPGK<br>APNRLIY | A--------<br>ASSLQS | GVPSRFSGSGSG--<br>TEFTLTFSSLQPEDFATYYC | LQHHS-----------<br>------------YPRT | FGQGT<br>KVEIK |
| **iPS:435<br>675** | **21-<br>225_169D7** | **VK1\|A30/<br>JK1** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHHS-----------<br>------------CPWT | FGQGT<br>KVEIK |
| **iPS:435<br>681** | **21-<br>225_169D1<br>1** | **VK1\|A30/<br>JK1** | | DIQMTQSPSSLSA<br>SVGDRVTITC | RAS--QGIR--<br>----DDLG | WYQQKPGK<br>VPKRLIY | A--------<br>ASSLQS | GVPSRFSGSGSG--<br>TEFTLTISSLQPEDFATYYC | LQHYS-----------<br>------------YPRT | FGQGT<br>KVEIK |
| **iPS:435<br>687** | **21-<br>225_170H1** | **VK1\|A30/<br>JK1** | | DIQMTQSPSSLSA<br>SVGDRVTITC | RAS--QGIR--<br>----NDLG | WYQQKPGK<br>APKRLIY | A--------<br>TSSLQS | GVPSRFSGSGSG--<br>TEFTLTISSLQPEDFATYYC | LQHSS-----------<br>------------NPWT | FGQGT<br>KVESK |
| **iPS:435<br>689** | **21-<br>225_170F3** | **VK1\|A30/<br>JK1** | | DIQMTQSPSSLSA<br>SVQDRVTITC | RAS--RGIR--<br>----NDLG | WYQQKPGK<br>APKRLIY | A--------<br>ASSLQS | GVPSRFSGSGSG--<br>TEFTLTINSLQPEDFATYYC | LQHYS-----------<br>------------YPRT | FGQGT<br>KVEIK |
| **iPS:435<br>741** | **21-<br>225_174G1<br>0** | **VK1\|A30/<br>JK1** | | DIQMTQSPSSLSA<br>SVGDRVTITC | RAS--QGIR--<br>----DDLG | WYQQKPGK<br>VPKRLIY | A--------<br>ASSLQS | GVPSRFSGSGSG--<br>TEFTLTISSLQPEDFATYYC | LQHHS-----------<br>------------YPRT | FGQGT<br>KVEIK |
| **iPS:435<br>831** | **21-<br>225_190C1<br>2** | **VK1\|A30/<br>JK1** | | DIQMTQSPSSLSA<br>SVGDRVTITC | RAS--QGIR--<br>----KDLG | WYQQKPGK<br>APKRLIH | T--------<br>ASSLQS | GVPLRFSGSGSG--<br>TEFTLTISSLQPEDFATYYC | LQHNS-----------<br>------------YPWT | FGQGT<br>KVEIK |
| **iPS:435<br>857** | **21-<br>225_191A4** | **VK1\|A30/<br>JK1** | | DIQMTQSPSSLSA<br>SVGDRVTITC | RAS--QGIR--<br>----KDLG | WYQQKPGK<br>APKRLIH | T--------<br>ASSLQN | GVPLRFSGSGSG--<br>TEFTLTISSLQPEDFATYYC | LQHNS-----------<br>------------YPWT | FGQGT<br>KVEIK |
| **iPS:435<br>907** | **21-<br>225_190G3** | **VK1\|A30/<br>JK1** | | DIQMTQSPSSLSA<br>SVRDRVTITC | RAS--QGIR--<br>----KDLG | WYQQKPGK<br>APKRLIH | T--------<br>ASSLQS | GVPLRFSGSGSG--<br>TEFTLTISSLQPEDFATYYC | LQHNS-----------<br>------------YPWT | FGQGT<br>KVEIK |
| **iPS:435<br>919** | **21-<br>225_190H5** | **VK1\|A30/<br>JK1** | | DIQMTQSPSSLSA<br>SVGDRVTITC | RAS--QGIR--<br>----KDLG | WYQQKPGK<br>APKRLIH | T--------<br>ASSLQS | GVPLRFSGSGSG--<br>TEFTLTISSLQPEDFATYYC | LQHNN-----------<br>------------YPWT | FGQGT<br>KVEIK |
| **iPS:435<br>989** | **21-<br>225_192F7** | **VK1\|A30/<br>JK1** | | DIQMTQSPSSLSA<br>SVGDRVTITC | RAS--QGIR--<br>----KDLG | WYQQKPGK<br>APKRLIH | T--------<br>ASSLQS | GVPLRFSGSGSG--<br>TEFTLTISSLQPEDFATYYC | LQHTS-----------<br>------------YPWT | FGQGT<br>KVEIY |
| **iPS:436<br>132** | **21-<br>225_196C1<br>2** | **VK1\|A30/<br>JK1** | | DIQMTLSPSSLFA<br>CVGDRVIITC | RAS--QGIR--<br>----NDLG | WSQQNPGK<br>APKRLIY | A--------<br>ASSLQS | GVPSRFSGSGSG--<br>TEFTITISSLQPEDFETYYC | LQHND-----------<br>------------FPFT | FGRGT<br>KVEIK |
| **iPS:436<br>222** | **21-<br>225_200C9** | **VK1\|A30/<br>JK1** | | DIQMTQSPSSLSA<br>SVGDRVTITC | RAT--QGIR--<br>----KDLG | WYQQKPGK<br>APKRLIH | T--------<br>ASSLQS | GVPLRFSGSGSG--<br>TEFTLTISSLQPEDFATYYC | LQHNS-----------<br>------------YPWT | FGQGT<br>KVEIK |
| **iPS:436** | **21-** | **VK1\|A30/** | | DIQMTQSPSSRFA | RAS--QGIR-- | WYQQKPGK | A-------- | GVPSRFSGSGCG-- | LQHYS----------- | FGQGT |

| | | | | | | | | | | |
|---|---|---|---|---|---|---|---|---|---|---|
| iPS:436 274 | 21-225_204H3 | VK1\|A30/JK1 | | DIQMTQSPSSLSA SVGDRVTITC | RAS--QGIR-- ----NDLG | WYQQKPGK APELLIY | A-------- AASLQG | GVPSRFSGSGSG-- TEFTLTISSLQPEDFATYYC | LQHYS----------- ------------YPRT | FGQGT KVELQ |
| iPS:436 332 | 21-225_208B2 | VK1\|A30/JK1 | | DIQMTQSPSSLSA SVGDRVTITC | RAS--QGIR-- ----HDLG | WYQQKPGK APKRLIY | A-------- ASSLQS | GVPSGFSGSGSG-- TEFTLTISSLQPEDFATYYC | LQHYS----------- ------------YPRT | FGQGT KVEIK |
| iPS:436 352 | 21-225_210G5 | VK1\|A30/JK1 | | DIQMTQSPSSLSA SVGDRVTITC | RAS--QGIR-- ----HDLG | WYQQKPGK APKRLIY | A-------- ASSLQS | GVPSGFSGSGSG-- TEFTLTISSLQPEDFATYYC | LHHYS----------- ------------YPRT | FGQGT KVEIK |
| iPS:436 386 | 21-225_212B11 | VK1\|A30/JK1 | | DIQMTQSPSSLPA SVGDRVTITC | RAS--QGIR-- ----NDLG | WYQQKPGK APKRLIY | A-------- ASSLQS | GVPSRFSGSGSG-- TEFILTISSLQPEDFATYYC | LLHYS----------- ------------YPRT | FGQGT KVEIK |
| iPS:436 412 | 21-225_214H9 | VK1\|A30/JK1 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHYS----------- ------------YPRT | FGQGT KVEIK |
| iPS:436 414 | 21-225_214G10 | VK1\|A30/JK1 | | DIQMTLCPSSPPA SVGDRVTITC | RAS--QGIR-- ----NDLG | WYQQKPGK APKRLIY | A-------- ASSLQS | GVPSRFSGSGSG-- TEFILTISSLQPEDFATYYC | LLHNS----------- ------------YPRT | FGQGT KVEIK |
| iPS:436 416 | 21-225_214G12 | VK1\|A30/JK1 | | DIQMTQSPSSPPA SVGDRVTITC | RAS--QGIR-- ----NDLG | WYQQKPGK APKRLIY | A-------- ASSLQS | GVPSRFSGSGSG-- TEFILTISSLQPEDFATYYC | VMHYS----------- ------------YPRT | FGQGT KVEIK |
| iPS:436 418 | 21-225_215E3 | VK1\|A30/JK1 | | DIQMTQSPSSPPA SVGDRVTITC | RAS--QGIR-- ----NDLG | WYQQKPGK APKRLIY | A-------- ASSLQS | GVPSRFSGSGSG-- TEFTLTISSLQPEDFATYYC | VMHNS----------- ------------YPRT | FGQGT KVEIK |
| iPS:436 428 | 21-225_215E11 | VK1\|A30/JK1 | | DIQMTQCPSSPPA SVGDRVTITC | RAS--QGIR-- ----NDLG | WYQQKPGK APKRLIY | A-------- ASSLQS | GVPSRFSGSGSG-- TEFILTISSVQREDFATYYC | VMHYS----------- ------------YPRT | FGQGT KVEIK |
| iPS:436 438 | 21-225_216E8 | VK1\|A30/JK1 | | DIQMTQSPSSLPA SVGDRVTITC | RAS--QGIR-- ----NDLG | WYQQKPGK APKRLIY | A-------- ASSLQS | GVPSRFSGSGSG-- TEFILTISSLQPEDFATYYC | LMHYS----------- ------------YPRT | FGQGT KVEIK |
| iPS:436 440 | 21-225_216H12 | VK1\|A30/JK1 | | DIQMTLSPSSLPA SVGDRVTITC | RAS--QGIR-- ----NDLG | WYQQKPGK APKRLIY | G-------- ASSLQS | GVPSRFSGSGSG-- TEFILTISSLQPEDFATYYC | VMHNS----------- ------------YPRT | FGQGT KVEIK |
| iPS:436 450 | 21-225_217E5 | VK1\|A30/JK1 | | DIQMTQCPSSPPA FVGDRVTITC | RAS--QGIR-- ----NDLG | WYQQKPGK APKRLIC | A-------- ASSLQS | GVPSRFSGSGSG-- TEFILTISSLQPEDFATYYC | VMHNS----------- ------------YPRT | FGQGT KVEIK |
| iPS:436 456 | 21-225_217G10 | VK1\|A30/JK1 | | DIQMTQSPSSPPA SVGDRVTITC | RAS--QGIR-- ----NDLG | WYQQKPGK APKRLIY | A-------- ASSLQS | GVPSRFSGSGSG-- TEFILTISSLQPEDFATYYC | VMHNS----------- ------------YPRT | FGQGT KVEIK |
| iPS:436 458 | 21-225_217H12 | VK1\|A30/JK1 | | DIQMTQSPSSPPA SVGDRVTITC | RAS--QGIR-- ----NDLG | WYQQKPGK APKRLIY | A-------- ASSLQS | GVPSRFSGSGSG-- TEFILTISSLQPEDFATYYC | LMHYS----------- ------------YPRT | FGQGT KVEIK |
| iPS:436 462 | 21-225_218C4 | VK1\|A30/JK1 | | DIQMTQCPSSPPA FVGDRVTITC | RAS--QGIR-- ----NDLG | WYQQKPGK APKRLIY | A-------- ASSLQS | GVPSRFSGSGSG-- TEFILTISSVQREDFATYYC | VMHYS----------- ------------YPRT | FGQGT KVEIK |
| iPS:436 480 | 21-225_220F8 | VK1\|A30/JK1 | | DIQMTLSPSSPPA FVGDRVTITR | RAS--QGIR-- ----NDLG | WYQQKPGK APKRLIC | G-------- ASSLQS | GVPSRFSGSGSG-- TEFILTISSLQREDFATYYC | VMHNS----------- ------------YPRT | FGQGT KVEIK |
| iPS:436 534 | 21-225_224F1 | VK1\|A30/JK1 | | AIQMTQSPSSLSA SVGDRVTITC | RAS--QGIR-- ----DDLG | WYQQKPGK APKRLIY | A-------- ASSLQS | GVPSRISGSGSG-- TEFTLTISSLQPEDFAIYYC | LQHYS----------- ------------YPRT | FGQGT KVEIK |
| iPS:436 540 | 21-225_224F3 | VK1\|A30/JK1 | | DIQMTQSPSSLSA SVGDRVTITC | RAS--QGIR-- ----NDLG | WYQQKPGK APERLIY | A-------- ASSLQS | GVPSRFSGSGSG-- TEFTLTISSLQPEDFATYYC | LQHYN----------- ------------YPRA | FGQGT KVEIK |
| iPS:436 564 | 21-225_225A1 | VK1\|A30/JK1 | | DIQMTQSPSSLSA SVGDRVTITC | RAS--QGIR-- ----DDLG | WYQQIPGK APKRLIY | A-------- ASSLQS | GVPSRFSGSGSG-- TEFTLTISSLQPEDFATYYC | LQHYS----------- ------------YPRT | FGQGT KVEIK |
| iPS:436 | 21- | VK1\|A30/ | | DIQMTQSPSSLSA | RAS--QGIR-- | WYQQKPGK | A-------- | GLPSRFSGSGSG-- | LQHYN----------- | FGQGT |

| | | | | | | | | | | |
|---|---|---|---|---|---|---|---|---|---|---|
| iPS:436<br>604 | 21-<br>225_226F7 | VK1\|A30/<br>JK1 | | DIQMTQSPSSLSA<br>SVGDRVTITC | RAS--QGIG--<br>----NDLG | WYQQKPGK<br>APKRLIY | A--------<br>ASSLQS | GVPSRFSGSVSG--<br>TEFILTISSLQPEDFATYYC | LHHYS-----------<br>------------YPRT | FGQGT<br>KVEIK |
| iPS:436<br>620 | 21-<br>225_226H1<br>1 | VK1\|A30/<br>JK1 | | DIQMTQSPSSLSA<br>SVGDRVTITC | RTS--QGIR--<br>----NDLG | WYQQKPGK<br>APKRLIY | A--------<br>ASSLQS | GVPSRFSGSGSG--<br>TDFTLTISSLQPEDFATYYC | LQHYS-----------<br>------------YPRT | FGQGT<br>KVEIK |
| iPS:437<br>262 | 21-<br>225_170E4 | VK1\|A30/<br>JK1 | | DIQMTQSPSSLSA<br>SVGDRVTITC | RAS--QGIR--<br>----NDLG | YYQQKPGK<br>APKRLIY | V--------<br>ASGLQS | GVPSRFSGSGSG--<br>TEFTLTISSLQPEDFATYYC | LQHNS-----------<br>-----------YPPWT | FGQGT<br>KVDIK |
| iPS:437<br>280 | 21-<br>225_203C1<br>0 | VK1\|A30/<br>JK1 | | DIQMTQSPSSLSA<br>SVGDRVTITC | RAS--QDIR--<br>----NDLG | WYQQKPAK<br>APKRLIY | R--------<br>ASSLQS | GVPSRFSGSGSG--<br>TEFTLTISSLQPEDFAAYYC | LQHNS-----------<br>------------YPWT | FGQGT<br>KVEIK |
| iPS:437<br>286 | 21-<br>225_208F1 | VK1\|A30/<br>JK1 | | DIQMTQSPSSLSA<br>SVGDRVTITC | RAS--QGIR--<br>----HDLG | WYQQKPGK<br>APKRLIF | A--------<br>ASSLQS | GVPSRFSGSGSG--<br>TEFTLTISSLQPEDFATYYC | LQHYS-----------<br>------------FPRT | FGQGT<br>KVEIK |
| iPS:437<br>290 | 21-<br>225_210G6 | VK1\|A30/<br>JK1 | | DIQMTQSPSSRFA<br>FVGDRVTITC | RAS--QGIR--<br>----HDLG | WYQQKPGK<br>ALKRLIY | A--------<br>ASSSQS | GVPSRFSGSGCG--<br>TEFTLTISSVQREDFANYYC | VQHYS-----------<br>------------FPRT | FGQGT<br>KVEIK |
| iPS:451<br>114 | 21-<br>225_159A3 | VK1\|A30/<br>JK1 | | DIQMTQSPSSLSA<br>SVGDRVTITC | RAS--QDIR--<br>----KDLG | WYQQKPGK<br>APNRLIY | A--------<br>ASSLQS | GVPSRFSGSGSG--<br>TEFTLTFSSLQPEDFATYYC | LQHHS-----------<br>------------YPRT | FGQGT<br>KVEIK |
| iPS:392<br>626 | 21-<br>225_18A5 | VK1\|A30/<br>JK1 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPWT | FGQGT<br>KVEIK |
| iPS:392<br>634 | 21-<br>225_17H3 | VK1\|A30/<br>JK1 | | DIQMTQSPSSLSA<br>SVGDRVTFTC | RAS--QGIR--<br>----SDLG | WYQQKPGK<br>APKRLIY | A--------<br>ASSLQS | GVPSRFSGSGSG--<br>TEFTLTISSLQPEDFATYYC | LQQYS-----------<br>------------YPRT | FGQGT<br>KVEIK |
| iPS:392<br>674 | 21-<br>225_18C2 | VK1\|A30/<br>JK1 | | DIQMTQSPSSLSA<br>SVGDRVTITC | RAS--QGIR--<br>----NDLG | WYQQKPGK<br>APKRLIY | T--------<br>ASSLQS | GVPSRFSGSGFG--<br>TEFTLTISSLQPEDFATYYC | LQHNS-----------<br>------------YPWT | FGLGT<br>KVVIK |
| iPS:392<br>686 | 21-<br>225_17C7 | VK1\|A30/<br>JK1 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPWT | FGQGT<br>KVEIK |
| iPS:392<br>690 | 21-<br>225_18F2 | VK1\|A30/<br>JK1 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPWT | FGQGT<br>KVEIK |
| iPS:392<br>710 | 21-<br>225_19A10 | VK1\|A30/<br>JK1 | | DIQMTQSPSSLSA<br>SVGDRVTITC | RAS--QGIR--<br>----TDLG | WYQQRPGK<br>APKRLIY | T--------<br>ASSLQS | GVPSRFSGSGSG--<br>TEFTLTISRLQPEDFATYYC | LQHNG-----------<br>------------YPWT | FGQGT<br>KVEIK |
| iPS:392<br>740 | 21-<br>225_18H12 | VK1\|A30/<br>JK1 | | DIQMTQSPSSLSA<br>SVGDRVTITC | RAS--QGIR--<br>----NDLG | WYQQKPGK<br>APKRLIY | T--------<br>ASNLQS | GVPSRFSGSGSG--<br>TEFTLTISSLQPEDFATYYC | LQHNN-----------<br>------------YPWT | FGLGT<br>KVVIK |
| iPS:392<br>742 | 21-<br>225_20B2 | VK1\|A30/<br>JK1 | | DIQMTQSPSSLSA<br>SVGDRVNITC | RAS--QDIR--<br>----NDLG | WYQQKPGK<br>APKRLIY | A--------<br>ASSLQS | GVPSRFSGSGSG--<br>TEFTLTISSLQPEDFATYYC | LQHYN-----------<br>------------YPRA | FGQGT<br>KVDIK |
| iPS:392<br>758 | 21-<br>225_21G11 | VK1\|A30/<br>JK1 | | DIQMTQSPSSLSA<br>SVGDRVTITC | RAS--QGIR--<br>----NDLG | WYQQKPGK<br>APKRLIY | T--------<br>ASSLQS | GVPSRFSGSGSG--<br>TEFTLTISSLQPEDFATYYC | LQHNN-----------<br>------------YPWT | FGLGT<br>KVVIK |
| iPS:392<br>790 | 21-<br>225_20D10 | VK1\|A30/<br>JK1 | | DIQMTQSPSSLSA<br>FVGDRVTITC | RAS--QGIR--<br>----NDLG | WYQQKPGK<br>ARKRLIY | V--------<br>AYSLQS | GVPSRFSGSGYG--<br>TEFTLTISSLQPEDFATYYC | IQQNS-----------<br>------------YPWT | FGQGT<br>KVEIK |
| iPS:392<br>796 | 21-<br>225_22A4 | VK1\|A30/<br>JK1 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPWT | FGQGT<br>KVEIK |
| iPS:392<br>832 | 21-<br>225_21H8 | VK1\|A30/<br>JK1 | | DIQMTQSPSSLSA<br>SVGDRVTITC | RAS--QGIR--<br>----NDLG | WYQQKPGK<br>APKRLIY | A--------<br>ASSLQS | GVPSRFRGSGSG--<br>TEFTLTISSLQPEDFATYYC | LQHNS-----------<br>------------YPWT | FGQGT<br>KVEIK |
| iPS:392<br>836 | 21-<br>225_22F4 | VK1\|A30/<br>JK1 | | DIQMTQSPSSLSA<br>SVGDRVTITC | RAS--QVIR--<br>----DDLG | WYQQKPGK<br>APKRLIY | A--------<br>ASSLQS | GVPSRFSGSRSG--<br>TDFTLTISSLQPEDFATYYC | LHHYS-----------<br>------------YPRT | FGQGT<br>KVEIK |
| iPS:392<br>844 | 21-<br>225_23E11 | VK1\|A30/<br>JK1 | | DIQMTQSPSSLSA<br>SVGDRITITC | RAS--QGIR--<br>----NDLG | WYQQKPGK<br>APKRLIY | P--------<br>ASSLQS | GVPSRFSGSGSG--<br>TEFTLTISSLQPEDFATYYC | LQHNS-----------<br>------------YPWT | FGQGT<br>KVEIK |
| iPS:392<br>846 | 21-<br>225_24B6 | VK1\|A30/<br>JK1 | | DIQMTQSPSSLSA<br>SVGDRVTITC | RAS--QDIR--<br>----NDLG | WYQQKPGK<br>APKRLIY | A--------<br>ASSLHS | GVPSRFSGSGSG--<br>TEFTLTISSLQTEDFATYYC | LQHYS-----------<br>------------YPRT | FGQGT<br>KVEVK |

| | | | | | | | | | | |
|---|---|---|---|---|---|---|---|---|---|---|
| **iPS:392 872** | **21-225_20B11** | **VK1\|A30/ JK1** | | DIQMTQSPSSLSA<br>SVGDRVTITC | RAS--QGIG--<br>----NDLG | WYQQKPEK<br>APKRLIY | A--------<br>ASSLHS | GVPSRFSGSGSG--<br>TEFTLTISSLQPEDFATYYC | LQHYS-----------<br>------------YPRT | FGQGT<br>KVEIK |
| **iPS:392 876** | **21-225_21F7** | **VK1\|A30/ JK1** | | DIQMTQSPSSLSA<br>SVGDRVTITC | RAS--QDIR--<br>----NDLG | WYQQKPGK<br>APKRLIY | A--------<br>ASNFQS | GVPSRFSGSGSG--<br>TEFTLTISSLQPEDFATYYC | LQHNN-----------<br>------------YPWT | FGQGT<br>KVEIK |
| **iPS:392 884** | **21-225_23A10** | **VK1\|A30/ JK1** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTIGSLQPEDFATYYC | LQHYS-----------<br>------------YPRT | FGLGT<br>KVEIK |
| **iPS:392 894** | **21-225_21G2** | **VK1\|A30/ JK1** | | DIQMTQSPSSLSA<br>SVGDRVTITC | RAS--QGIR--<br>----NDLG | WYQQKPGK<br>APKRLIY | T--------<br>SSSLQS | GVPSRFSGSGSG--<br>TEFTLTISSLQPEDFATYYC | LQHNS-----------<br>------------YPWT | FGLGT<br>KVVIK |
| **iPS:392 908** | **21-225_23F12** | **VK1\|A30/ JK1** | | DIQMTQSPSSLSA<br>SVGDRVTITC | RAS--QGIR--<br>----NDLG | WYQQKPGK<br>APKRLIY | V--------<br>ASSLQS | GVPSRFSGSGSG--<br>TEFTLTISSLQPEDFASYYC | LQHNS-----------<br>------------YPWT | FGQGT<br>KVEIK |
| **iPS:392 914** | **21-225_25D12** | **VK1\|A30/ JK1** | | DIQMTQSPSSLSA<br>SVGDRVTITC | RTS--QGIR--<br>----NDLG | WYQQKPGK<br>APKRLIY | A--------<br>ASSLQS | GVPSRFSGSGSG--<br>TEFTLTISSLQPEDFATYYC | LQHYS-----------<br>------------FPRT | FGQGT<br>KVEIK |
| **iPS:392 918** | **21-225_28F5** | **VK1\|A30/ JK1** | | DIQMTQSPSSLSA<br>SVGDRVTITC | RAS--QGIR--<br>----NDLG | WYQQKPGK<br>APKRLIY | T--------<br>ASSLQS | GVPSRFSGSGSG--<br>TEFTLTISSLQPEDFATYYC | LQHNT-----------<br>------------YPWT | FGQGT<br>KVEVK |
| **iPS:392 958** | **21-225_28C7** | **VK1\|A30/ JK1** | | DIQMTQSPSSLSA<br>SVGDRVTITC | RAS--QGIR--<br>----NDLG | WYQQKPGK<br>APKRLIY | I--------<br>ASSLQS | GVPSRFSGSGSG--<br>TEFTLTISSLQPEDFATYYC | LQHNT-----------<br>------------YPWT | FGQGT<br>KVEIK |
| **iPS:392 972** | **21-225_26A2** | **VK1\|A30/ JK1** | | DIQMTQSPSSLSA<br>SVGDRVTITC | RAS--QGIR--<br>----SDLG | WYQQKPGK<br>APKRLIY | T--------<br>ASSLQS | GVPSRFSGSGSG--<br>TEFTLTFSSLQPEDFATYYC | LQHNR-----------<br>------------YPWT | FGQGT<br>KVEIK |
| **iPS:393 026** | **21-225_32B6** | **VK1\|A30/ JK1** | | DIQMTQSPSSLSA<br>SVGDRVTITC | RAS--QGIR--<br>----NDLG | WYQQKPGK<br>APKRLIY | I--------<br>ASSLQS | GVPSRFSGSGSG--<br>TEFTLTISSLQPEDFATYYC | LQHNS-----------<br>------------YPWT | FGQGT<br>KVEIK |
| **iPS:393 130** | **21-225_33C2** | **VK1\|A30/ JK1** | | DIQMTQSPSSLSA<br>SVGDRVTITC | RAS--QGIR--<br>----NDLG | WYQQKPVK<br>APKRLIY | A--------<br>APSLQS | GVPSRFSGSGSG--<br>TEFTLTISSLQPEHFATYYC | LQHNS-----------<br>------------YPWT | FGQGT<br>KVEIK |
| **iPS:393 812** | **21-225_6A11** | **VK1\|A30/ JK1** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPWT | FGQGT<br>KVEIK |
| **iPS:393 838** | **21-225_6G2** | **VK1\|A30/ JK1** | | DIQMTQSPSSRSA<br>FVGDRVTITY | RAS--QGIR--<br>----NDLG | WYQQKPGK<br>APQRLIY | A--------<br>ASSLQS | GVPSRFSGSGYG--<br>TEFNITISSLQPEDFAIYYC | IQHNS-----------<br>------------YLWT | FGQGT<br>KVEIT |
| **iPS:393 864** | **21-225_4C5** | **VK1\|A30/ JK1** | | DIQMTQSPSSLSA<br>SVGHRVHLTC | RAS--RGIR--<br>----GDLG | WYRQKPGK<br>APKRLIY | A--------<br>ASNLQS | GVPSRFSGSGYG--<br>TEFTLTIGSLQPEDFATYYC | LQHYS-----------<br>------------YPRT | FGQGT<br>KVEIK |
| **iPS:393 868** | **21-225_9C11** | **VK1\|A30/ JK1** | | DIQMTQSPSSLSA<br>SVGDRVTITC | RAS--QNIR--<br>----NYLN | WYQQKSGR<br>APKLLIY | V--------<br>ASSLQS | GVPSRFSGSGSG--<br>TEFTLTINSLQPEDFAIYHC | HQSNS-----------<br>------------TPLT | FGQGT<br>KVEIK |
| **iPS:393 876** | **21-225_9A1** | **VK1\|A30/ JK1** | | DIQMTQSPSSRSA<br>FVGDRVTITC | RAS--QGIR--<br>----NDLG | WYQQKPGK<br>ARKRLIY | T--------<br>ASSLQS | GVPSRFSGSGYG--<br>TEFTITISSLQPEDFATYYC | IQHNS-----------<br>------------YLWT | FGQGT<br>KVEIK |
| **iPS:393 902** | **21-225_14E10** | **VK1\|A30/ JK1** | | DIQMTQSPSSLSA<br>SVGDRVTITC | RTS--QGIR--<br>----NDLG | WYQHKPGQ<br>APKRLIY | A--------<br>ASSLQS | GVPSRFSGSGSG--<br>TEFTLTISSLQPEDFTAYYC | LQHYS-----------<br>------------YPRT | FGQGT<br>KVEIK |
| **iPS:393 908** | **21-225_10E9** | **VK1\|A30/ JK1** | | DIQMTQSPSSLSA<br>SVGDRVTFTC | RAS--QDIR--<br>----SDLG | WYQQKPGK<br>APTRLIF | A--------<br>ASSLQS | GVPSRFSGSGSG--<br>TEFTLTINSLQPEDFATYYC | LQHYS-----------<br>------------YPRT | FGQGT<br>KVEIK |
| **iPS:393 916** | **21-225_2G4** | **VK1\|A30/ JK1** | | DIQMTQSPSSLSA<br>SVGDRVTITC | RAS--QGIR--<br>----NDLG | WYQQKPGK<br>APTRLIY | A--------<br>ASSLHS | GVPSRFSGSGSG--<br>TEFTLTISSLQPEDLATYYC | LQHYS-----------<br>------------FPRT | FGRGT<br>KVEIK |
| **iPS:393 948** | **21-225_16A5** | **VK1\|A30/ JK1** | | DIQMTQSPSSLSA<br>SVGDRVTITC | RAS--QGIR--<br>----NDLG | CYQQKPGK<br>APKRLIY | A--------<br>ASSLQS | GVPSRFSGNGYG--<br>TEFTLTISSLQPEDFATYYC | LQHNS-----------<br>------------YPWT | FGQGT<br>KVEIK |
| **iPS:393 960** | **21-225_7G2** | **VK1\|A30/ JK1** | | DIQMTQSPSSLSA<br>SVGDRVTITC | RAS--QGIR--<br>----NDLG | WYQQKPGK<br>APKRLIY | T--------<br>ASSLQS | GVPSRFSGSGSG--<br>TEFTLTISSLQPEDFATYYC | LQHNS-----------<br>------------YPWT | FGLGT<br>KVVIK |
| **iPS:393 966** | **21-225_7F8** | **VK1\|A30/ JK1** | | DIQMTQSPSSLSA<br>SVGDRVTITC | RAS--QGIG--<br>----NDLG | WYQQKPGK<br>APKRLIY | A--------<br>ASSLQS | GVPSRFSGSVSG--<br>TEFTLPISSLQPEDFATYYC | LQHYT-----------<br>------------YPRT | FGQGT<br>KVEIK |
| **iPS:393 972** | **21-225_7C9** | **VK1\|A30/ JK1** | | DIQMTQSPSSLSA<br>SGGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQLYS-----------<br>------------YPRT | FGQGT<br>KVDIK |

| | | | | | | | | | | |
|---|---|---|---|---|---|---|---|---|---|---|
| iPS:393 978 | 21-225_4C12 | VK1\|A30/ JK1 | | DIQMTQSPSSLSA<br>SVGDRVTITC | RAS--QGIR--<br>----NDLG | WYQQKPGK<br>APTRLIY | A--------<br>ASSLHS | GVPSRFSGSGSG--<br>TEFTLTISSLQPEDLATYYC | LQHYS-----------<br>------------FPRT | FGQGT<br>KVEIK |
| iPS:393 986 | 21-225_7G4 | VK1\|A30/ JK1 | | DIQMTQSPSSLSA<br>SVGDRVTITC | RAS--QGIR--<br>----NDLG | WYQQRPGK<br>APKRLIY | A--------<br>ASSLQS | GVPSRFSGSGSG--<br>TEFTLTISSLQPEDSATYYC | LHQYS-----------<br>------------YPRT | FGQGT<br>KVEIK |
| iPS:393 996 | 21-225_15C11 | VK1\|A30/ JK1 | | DIQMTQSPSSLSA<br>SVGDRVTITC | RAS--QGIR--<br>----NDLG | WYQQKPGK<br>VPKRLIY | A--------<br>ASSLQS | GVPSRFSGSGSG--<br>TEFTLTISSLQPEDFATYYC | LLHYS-----------<br>------------YPRT | FGRGT<br>KVEIK |
| iPS:393 998 | 21-225_12B12 | VK1\|A30/ JK1 | | DIQMTQSPSSLSA<br>SVGDRVTITC | RAS--QGIR--<br>----NDLG | WYQQKPGK<br>APKRLIY | T--------<br>ASSLQS | GVPSRFSGSGSG--<br>TEFTLTISSLQPEDFATYYC | LQHNS-----------<br>------------YPWT | FGLGT<br>KVVIK |
| iPS:394 041 | 21-225_5E5 | VK1\|A30/ JK1 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHYS-----------<br>------------YPRT | FGQGT<br>KVEVK |
| iPS:394 067 | 21-225_12F2 | VK1\|A30/ JK1 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFVTYYC | LQHNS-----------<br>------------YPWT | FGQGT<br>KVEIK |
| iPS:394 089 | 21-225_12E6 | VK1\|A30/ JK1 | | DIQMTQSPSSLSA<br>SVGDRVTITC | RAS--QGIR--<br>----SDLG | WYQQKPGK<br>APKRLIY | A--------<br>ASSLQS | GVPSRFSGSGSG--<br>TEFTLTISSLQPEDFATYYC | LQHNS-----------<br>------------YPWT | FGQGT<br>KVEIK |
| iPS:394 093 | 21-225_9D12 | VK1\|A30/ JK1 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPWT | FGQGT<br>KVEIK |
| iPS:394 095 | 21-225_16H4 | VK1\|A30/ JK1 | | DIQMTQSPSSLSA<br>SVGDRVTITC | RAS--QGIR--<br>----NDLG | WYQQKPGK<br>APKRLIY | T--------<br>ASSLQS | GVPSRFSGSGSG--<br>TEFTLTISSLQPEDFATYYC | LQIINS-----------<br>------------YPWT | FGQGT<br>KVEIK |
| iPS:394 097 | 21-225_16G7 | VK1\|A30/ JK1 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPWT | FGQGT<br>KVEIK |
| | | Germline | | **K_FR1** | **K_CDR1** | **K_FR2** | **K_CDR2** | **K_FR3** | **K_CDR3** | **K_FR4** |
| | VK3\|L2/JK2 | | 32140 | EIVMTQSPATLSV<br>SPGERATLSC | RAS--QSVS--<br>----SNLA | WYQQKPGQ<br>APRLLIY | G--------<br>ASTRAT | GIPARFSGSGSG--<br>TEFTLTISSLQSEDFAVYYC | QQYNN-----------<br>------------WPYT | FGQGT<br>KLEIK |
| iPS:468 828 | 21-225_162A10 | VK3\|L2/J K2 | | EIVMTQSPATLSV<br>SPGERATLSC | RAS--QTVN--<br>----SNLA | WYQQKSGQ<br>APRLLIF | G--------<br>ASTRAT | VIPARINGSGSG--<br>TEFTLTISSLRSEDFAVYFC | QQYND-----------<br>------------WPCS | FGQGT<br>KLEIK |
| iPS:434 255 | 21-225_62E6 | VK3\|L2/J K2 | | EIVMTQSPATLSV<br>SPGERATLSC | RAS--QSVN--<br>----SNLA | WYQQKPGQ<br>APRLLIS | V--------<br>ASTRAT | GIPARFNGSGSG--<br>TEFTLTISSLQSEDFAVYYC | QQYND-----------<br>------------WPCS | FGQGT<br>KLEIK |
| iPS:434 269 | 21-225_57H3 | VK3\|L2/J K2 | | EIVMTQSPATLSV<br>SPGERATLSC | RAS--QSVS--<br>----SSLA | WYQQKPGQ<br>APRLLIY | G--------<br>ASTRAT | GFPARFNGSGSW--<br>TEFTLTISSLQSEDFAIYYC | QQYND-----------<br>------------WPCS | FGQGT<br>KLEIK |
| iPS:434 363 | 21-225_65A6 | VK3\|L2/J K2 | | EIVMTQSPVTLFV<br>SPGERATLSC | RAS--QSVN--<br>----SNLA | WYQQKPGQ<br>APRLLIY | G--------<br>ASTRAT | GIPARFNGSGSG--<br>TEFTLTISSLQSEDFAVYYC | QQYND-----------<br>------------WPCS | FGLET<br>KLEIK |
| iPS:434 393 | 21-225_67C3 | VK3\|L2/J K2 | | EIVMTQSPATLSV<br>SPGERATLSC | RAS--QSVN--<br>----SNLA | WYQQKPGQ<br>APRLLIS | I--------<br>ASTRAT | GIPARFNGSGSG--<br>TEFTLTISSLQSEDFAVYYC | QQYND-----------<br>------------WPCS | FGQGT<br>KLEIK |
| iPS:434 425 | 21-225_70A5 | VK3\|L2/J K2 | | EIVMTQSPATLSV<br>SPGERATLSC | RAS--QSVN--<br>----SNLA | WYQLKPGQ<br>APRLLIS | I--------<br>ASTRAT | GIPPRFNGSGSG--<br>TEFTLTISSLQSEDFAVYYC | QQYND-----------<br>------------WPCS | FGQGT<br>KLEIK |
| iPS:434 485 | 21-225_76D2 | VK3\|L2/J K2 | | EIVMTQSPATPSV<br>SPGERATLSC | RAS--VSVV--<br>----NSLA | WYQQKPGQ<br>APRLLIH | G--------<br>ASTRAT | GIPARFSGSGSG--<br>TEFTLTISSVQSEDFAIYYC | QQYND-----------<br>------------WPCS | FGQGT<br>KLEIK |
| iPS:434 537 | 21-225_74E11 | VK3\|L2/J K2 | | EIVMTQSPATLSV<br>SPGERATLSC | RAS--LSVV--<br>----NSLA | WYQQKPGQ<br>APRLLIH | G--------<br>ASTRAT | GIPARFSGSGSG--<br>TEFTLTISSLQSEDFAIYYC | QQYND-----------<br>------------WPCS | FGQGT<br>KLEIK |
| iPS:434 569 | 21-225_77H5 | VK3\|L2/J K2 | | EIVMTQSPVTLSV<br>SPGERATLSC | RAS--QSVS--<br>----SSLA | WYQQKPGL<br>APRLLIY | G--------<br>ASTRAT | GIPARFSGSGSG--<br>TEFSFTISSLQSEDFAVYFC | QQYND-----------<br>------------WPCS | FGQGS<br>KLEIQ |
| iPS:434 629 | 21-225_74C3 | VK3\|L2/J K2 | | EIVMTQSPATLSV<br>SPGERATLSC | RAS--QSVA--<br>----SSLA | WYQQKPGQ<br>APRLLIF | G--------<br>TSTRAT | GIPARFSGSGSG--<br>TEFTLIISSLQSEDFAVYYC | QQYND-----------<br>------------WPCS | FGLGT<br>KLEIK |
| iPS:434 673 | 21-225_74E3 | VK3\|L2/J K2 | | EIVMTQSPATLSL<br>SPGERATLSC | RAS--LSVV--<br>----NSLA | WYQQKPGQ<br>APRLLIY | G--------<br>ASTRAT | GIPARFSGSGSG--<br>TEFTLTISSLOSEDFAIYYC | QQYND-----------<br>------------WPCS | FGQGT<br>KLEIK |

| | | | | | | | | | | |
|---|---|---|---|---|---|---|---|---|---|---|
| iPS:435 109 | 21-225_92H5 | VK3\|L2/J K2 | | EIVMTQSPATLSV SPGERATLSC | RAS--QDVI-- ----TYLA | WYQQKPGQ APRLLIY | G-------- ASTRAT | GVPARFSGSGSG-- TEFTLTITSLQSEDFALYYC | QEYND----------- ------------WPCS | FGQGT KLEIK |
| iPS:435 221 | 21-225_95G2 | VK3\|L2/J K2 | | EIVMTQSPATLSL SPGERATLSC | RAS--MSVV-- ----NSLA | WYQQKPGQ APRLLIY | G-------- ASTRAT | GIPARFSGSGSG-- TEFTLTISSLQSEDFAIYYC | QQYND----------- ------------WPCS | FGQGT KLEIK |
| iPS:436 051 | 21-225_193G12 | VK3\|L2/J K2 | | EIVMTQSPATLSV SPGERATLSC | RAS--QSVS-- ----SSLA | WYQQKPGQ APRILIS | G-------- ASTRAT | GIPARFSGSGSG-- TEFTLTISSLQSADFAVYYC | QQYNN----------- ------------WPCS | FGQGT KLEIK |
| iPS:436 236 | 21-225_201F7 | VK3\|L2/J K2 | | EIVMTQSPATLSV SPGERATLSC | RAS--QNIK-- ----NNLA | WYQQKPGQ APRLLIF | G-------- ASTRAT | GIPARFSGSGSG-- TEFTLTISSLQSEDFAVYYC | QQFYN----------- ------------WLCS | FGQGT KLELK |
| iPS:436 250 | 21-225_201A4 | VK3\|L2/J K2 | | EIVMTQSPATLSV SPGESATLSC | RSS--QNIK-- ----SNLA | WYQQKPGQ APRLLIF | G-------- ASTRAT | GIPARFSGSGSG-- TEFTLTISSLQSEDFAVYYC | QQFYN----------- ------------WLCS | FGQGT KLELK |
| iPS:436 252 | 21-225_202A8 | VK3\|L2/J K2 | | EIVMTQSPATLSV SPGERATLSC | RAS--QRIN-- ----NNLA | WYQQNPGQ APRLLIY | G-------- ASTRAT | GVPARFSGSGSG-- TEFTLTISSLQSEDFTVYYC | QQYYN----------- ------------WLCS | FGQGT KLEIR |
| iPS:436 258 | 21-225_202F12 | VK3\|L2/J K2 | | EIVMTQSPATLSV SPGERATLSC | RAS--QSVL-- ----NNLA | WYQQRPGQ APRLLIY | G-------- ASTRAT | GIPARFSGSGSG-- TEFTLTISSLQSEDFAVYYC | QQYDN----------- -----------WPPCS | FGQGT KLEIK |
| iPS:436 278 | 21-225_201F2 | VK3\|L2/J K2 | | EIVMTQSPATLSV SPGERATLSC | RAS--QNIK-- ----SNLA | WYQQKPGQ APRLLIF | G-------- ASTRAT | GIPARFSGSGSG-- TEFTLTISSLQSEDFAVYYC | QQFYN----------- ------------WLCS | FGQGT KLELK |
| iPS:436 294 | 21-225_205G4 | VK3\|L2/J K2 | | EIVMTQSPATLSV SPGERATLSC | RAS--QNIK-- ----SNLA | WYQQKPGQ APRLLIF | G-------- ASTRAT | GIPARFSGSGSG-- TEFTLTISSLQSEDFAVYYC | QQFYN----------- ------------WLCS | FGQGT KLELK |
| iPS:436 306 | 21-225_201H4 | VK3\|L2/J K2 | | EIVMTQSPATLSV SPGERATLSC | RAS--QSVN-- ----SYLA | WYQQKPGQ APRLLIY | G-------- ASTRAT | GIPARFSGSGSG-- TEFTLTISSLQSEDFAVYYC | QEYND----------- ------------WPCS | FGQGT NLEIK |
| iPS:436 356 | 21-225_210H10 | VK3\|L2/J K2 | | EIVMTQSPATLSV SPGERATLSC | RAS--QSVK-- ----SNLA | WYQQKPGQ APRLLIY | G-------- ASTRAT | GIPARFSGSGSG-- TEFTLTISSLQSEDFAVYSC | QQYYN----------- ------------WLCS | FGQGT KLEIK |
| iPS:436 382 | 21-225_212C10 | VK3\|L2/J K2 | | EIVMTQSPATLSV SPGERATLSC | RAS--QSVA-- ----SSLA | WYQQKPGQ APRLLIH | G-------- TSTRAT | DVPARFSGFGSG-- SDFTLTISSLQSEDFAVYSC | QQYND----------- ------------WPCS | FGQGT KLEIK |
| iPS:436 434 | 21-225_216B10 | VK3\|L2/J K2 | | EIVMTESPATLSV SPGERATLSC | RAS--QSVN-- ----NNLA | WYRQKPGQ APRLLIY | G-------- ASTRAT | GIPPRFSGSGSG-- TEFTLSISSLQSEDFAVYYC | QQYND----------- ------------WPCS | FGQGT KLEIR |
| iPS:392 806 | 21-225_24H3 | VK3\|L2/J K2 | | EIVMTQSPATLSV SPGERATLSC | RAS--QSVS-- ----SNLA | WYQQKPGQ APRLLIY | F-------- ASIRAT | GIPARFSGSGSG-- TEFTLTISSLQSEDFAVYYC | QQYNN----------- -----------WPMCS | FGQGT KLEIK |
| | | Germline | | **K_FR1** | **K_CDR1** | **K_FR2** | **K_CDR2** | **K_FR3** | **K_CDR3** | **K_FR4** |
| | VK3\|L2/JK4 | | 32141 | EIVMTQSPATLSV SPGERATLSC | RAS--QSVS-- ----SNLA | WYQQKPGQ APRLLIY | G-------- ASTRAT | GIPARFSGSGSG-- TEFTLTISSLQSEDFAVYYC | QQYNN----------- ------------WPLT | FGGGT KVEIK |
| iPS:468 830 | 21-225_191G11 | VK3\|L2/J K4 | | EIVMTQSPATLSV SPGERANLSC | RTS--QSVW-- ----ISVA | WYHQKPGQ APRLLIY | G-------- AATRAT | GIPARFSGSGSG-- TEFTLTISSLQSEDFAVYYC | QQYNY----------- ------------WPLT | FGGGT KVEIK |
| iPS:434 147 | 21-225_55E1 | VK3\|L2/J K4 | | EIVMTQSPATLSV SPGERATLSC | RAS--QSVS-- ----SDLA | WYQLKPGQ APRLLIY | D-------- ASARAT | GIPARFSGSGSG-- TEFTLTISSLQSEDFAFYYC | QQYYN----------- ------------WPLT | FGGGT KVEIK |
| iPS:434 621 | 21-225_74D1 | VK3\|L2/J K4 | | EIVMTQSPATLSV SPGERATLSC | RAS--QSVS-- ----RNLA | WFQQKPGQ APRLLIY | G-------- ASIRAT | GIPARFSGSGSG-- TEFTLTIYSLQSEDFAVYYC | QQYNN----------- -----------WPPLT | FGGGT KVEIK |
| iPS:435 821 | 21-225_190E11 | VK3\|L2/J K4 | | EIVMTQSPATLSV SPGERATLSC | RAS--QSFR-- ----INLA | WYQQRPGQ APRLLIY | G-------- ASTRAT | GIPARFSGSGSG-- TEFTLTISSLQSEDFAVYYC | QQYNN----------- ------------WPLT | FGGGT KVEIK |
| iPS:435 | 21- | VK3\|L2/J | | EIVMTQSPATLSV | RPS--QSFS-- | WYQQKPGQ | G-------- | GIPSRFSGSGSG-- | QQYNN----------- | FGGGI |

| | | | | | | | | | | |
|---|---|---|---|---|---|---|---|---|---|---|
| iPS:436 203 | 21-225_199A6 | VK3\|L2/JK4 | | DIVMTQSPATLSV SPGDRATLSC | RPS--QSFS-- ----RNLA | WYQQKPGQ APRLLIY | G-------- ASTRAT | GIPARFSGSGSG-- TEFTLTISSLECEDFAVYYC | QQYNN----------- ------------WPLT | FGGGT KVEIK |
| iPS:437 334 | 21-225_75F11 | VK3\|L2/JK4 | | EIVMTQSPATLFV SPGERATLSC | RAS--QSVS-- ----RNLA | WFQQKPGQ APRLLFY | G-------- ASIRAT | GIPARFSGSGSG-- TEFTLTIYSLQYEDFAVYYC | QQYNN----------- -----------WPPLT | FGGGT KVEIK |
| iPS:448 904 | 21-225_65C12 | VK3\|L2/JK4 | | EIVMTQSPATLSV FPGEGATLSC | RAS--QSVS-- ----INLA | WYQQKPGQ APRLLIY | G-------- ASTRAT | GIPARFNASGSG-- TEFTLSISSLQSENFAVYYC | QQYNT----------- ------------WPLT | FGGGT KVEIK |
| | | Germline | | K_FR1 | K_CDR1 | K_FR2 | K_CDR2 | K_FR3 | K_CDR3 | K_FR4 |
| | VK1\|A30/JK3 | | 32142 | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPFT | FGPGT KVDIK |
| iPS:468 832 | 21-225_76H10 | VK1\|A30/JK3 | | DIQMTQSPSSLSA SAGDRVTITC | RAS--QDIR-- ----NYLG | WYQQKPGK APKRLIY | G-------- ASSLQS | GVPSRFSGSGSG-- TEFTLTISSLQPEDFATFYC | LQYNS----------- ------------YPFT | FGPGT KVDIK |
| iPS:468 836 | 21-225_198E3 | VK1\|A30/JK3 | | DIQMTQSPSSLSA SVGDRVTITC | RAS--QGIR-- ----KDLG | WYQQKPGK APKRLIY | A-------- ASSLQS | GVPSRFSGSGSG-- TEFTLTISSLQPEDFATYVC | LQHYR----------- ------------YPFT | FGPGT KVDFK |
| iPS:468 844 | 21-225_48E10 | VK1\|A30/JK3 | | DIQMTQSPSSLSA SVGDRVTITC | RAS--QGIR-- ----SDLG | WYQQKPGK APKRLIY | T-------- ASSLQS | GVPSRFSGSGSG-- TEFTLTISSLQPEDFATYYC | LQYNS----------- ------------YPFT | FGPGT KVDIK |
| iPS:468 846 | 21-225_53B10 | VK1\|A30/JK3 | | DIQMTQSPSSLSA SAGDRVTITC | RAS--QDIR-- ----NYLG | WYQQKPGK APKRLIY | G-------- ASSLQS | GVPSRFSGSGSG-- TEFTLTISSLQPEDFATFYC | LQYNS----------- ------------YPFT | FGPGT KVDIK |
| iPS:433 895 | 21-225_43E1 | VK1\|A30/JK3 | | DIQMTQSPSSLSA SVGDRVTITC | RAS--QGIR-- ----NDLG | WYQKKPGK APKRLIN | A-------- ASSLQS | GVPSRFSGSGSG-- TEFTLTISSLQPEDFATYYC | LQHNS----------- ------------YPFT | FGPGT KVDIK |
| iPS:433 905 | 21-225_43E5 | VK1\|A30/JK3 | | DIQMTQSPSSLSA SVGDRVTITC | RAS--QGIR-- ----NDLG | WYQQKPGK APKRLIY | G-------- ASNLQS | GVPSRFSGSGSG-- TEFTLTVSSLQPEDFATYFC | LQHTS----------- ------------FPFT | FGPGT KVDIK |
| iPS:433 913 | 21-225_43H8 | VK1\|A30/JK3 | | DIQMTQSPSSLSA SVGDRVTITC | RAS--QGIR-- ----NDLG | WHQQKPGK APKRLIY | A-------- ASSLQS | GVPSRFSGSGSG-- TEFTLSISSLQPEDFATYYC | LQHNS----------- ------------FPFT | FGPGT KVDIK |
| iPS:433 933 | 21-225_44C8 | VK1\|A30/JK3 | | DIQMTQSPSSLSA SVGDRVTITC | RAS--QGIR-- ----NDLG | WYQQKPGK APKRLIY | A-------- ASNLQS | GVPSRFSGSRSG-- TEFTLTISSLQPEDFATYYC | LQHNS----------- ------------YPFT | FGPGT KVDIK |
| iPS:433 949 | 21-225_45H2 | VK1\|A30/JK3 | | DIQMTQSPSSLSA SVGDRVTITC | RAS--QGIR-- ----NDLG | WYQQKPGK APKRLIY | G-------- ASNLQS | GVPSRFSGSGSG-- TEFTLTVSSLQPEDFATYFC | LQHTS----------- ------------FPFT | FGPGT KVDIK |
| iPS:433 981 | 21-225_46E9 | VK1\|A30/JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTVSSLQPEDFATYFC | LQHTS----------- ------------FPFT | FGPGT KVDIK |
| iPS:433 995 | 21-225_47H7 | VK1\|A30/JK3 | | DIQMTQSPSSLSA SVGDRVTITC | RTS--QGIR-- ----NDLG | WYQKKPGK APKRLIY | A-------- ASSLQS | GVPSRFSGSGSG-- TEFTLTVSSLQPEDFATYFC | LQHTS----------- ------------FPFT | FGPGT KVDIK |
| iPS:434 039 | 21-225_43B1 | VK1\|A30/JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTVSSLQPEDFATYFC | LQHTS----------- ------------FPFT | FGPGT KVDIK |
| iPS:434 057 | 21-225_51E4 | VK1\|A30/JK3 | | DIQMTQSPSSLSA SVGDRVTITC | RAS--QGIR-- ----NDLG | WYQQKPGK APQRLIY | A-------- ASSLQS | GVPSRFSGSGSG-- TEFTLTISSLQPEDFAAYYC | LQHNS----------- ------------YPFT | FGPGT KVDIK |
| iPS:434 071 | 21-225_51F9 | VK1\|A30/JK3 | | DIQMTQSPSSLSA SVGDRVTITC | RAS--QGIR-- ----TDLG | WYQQKPRK APQRLIY | A-------- ASSLQR | GVPSRFSGSGSG-- TDFTLTISSLQPEDFASYYC | LQHNS----------- ------------YPFT | FGPGT KVDIK |
| iPS:434 075 | 21-225_51B11 | VK1\|A30/JK3 | | AIQMTQSPSSLSA SVGDRVTITC | RTS--QGIR-- ----NDLG | WYQQKPRK APKRLIY | A-------- ASSLQS | GVPSRFSGSGSG-- TEFTLTISSLQPEDFATYYC | LQHNS----------- ------------YPFT | FGPGT KVDIK |
| iPS:434 091 | 21-225_52B9 | VK1\|A30/JK3 | | AIQMTQSPSSLSA SVGDRVTITC | RAS--QGIR-- ----NDLG | WYQQKPRK APKRLIY | A-------- ASSLQS | GVPLRFSGSGSG-- TEFTLTIRSLQPEDFATYYC | LQHNS----------- ------------YPFT | FGPGT KVDIK |
| iPS:434 101 | 21-225_52H12 | VK1\|A30/JK3 | | DIQMTQSPSSLSA SVGDRVTITC | RAS--QGIR-- ----NNLG | WYQQKPGK APKRLIY | G-------- ASSLQS | GVPSRFSGSGSG-- TEFTLTISSLQPEDFATYYC | LQYNS----------- ------------YPFT | FGPGT KVDIK |
| iPS:434 103 | 21-225_53G1 | VK1\|A30/JK3 | | DIQMTQSPSSLSA SVGDRVTITC | RAS--QGIR-- ----NDLG | WYQQKPGK APKRLIY | P-------- ASSLQS | GVPSRFSGSGSG-- TEFSLTISSLQPEDFATYYC | LQDNS----------- ------------YPFT | FGPGT KVDIK |
| iPS:434 | 21- | VK1\|A30/ | | DIQMTQSPSSLSA | RAS--QGIR-- | WYQQKPGK | A-------- | GVPSRFSGSGSG-- | LQHNS----------- | FGPGT |

| | | | | | | | | | | |
|---|---|---|---|---|---|---|---|---|---|---|
| iPS:434<br>131 | 21-<br>225_54D3 | VK1\|A30/<br>JK3 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPFT | FGPGT<br>KVDIK |
| iPS:434<br>143 | 21-<br>225_54G7 | VK1\|A30/<br>JK3 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LHHNT-----------<br>------------YPFT | FGPGT<br>KVDIK |
| iPS:434<br>155 | 21-<br>225_55B3 | VK1\|A30/<br>JK3 | | DIQMTQSPSSLSA<br>SVGDRVTITC | RAS--QGIR--<br>----NDLG | WYQQKPGK<br>APKRLIY | A--------<br>ASSLQS | GVPSRFSGSRSG--<br>TEFTLTISSLQPEDFASYYC | LQHNS-----------<br>------------YPFT | FGPGT<br>KVDIK |
| iPS:434<br>169 | 21-<br>225_50C4 | VK1\|A30/<br>JK3 | | DIQMTQSPSSLSA<br>SVGDRVTLTC | RAS--QGIR--<br>----NDLG | WYQQKPGI<br>APKRLIY | A--------<br>ASSLQS | GVPSRFSGSGSG--<br>TEFTLTISSLQPEDFATYYC | LQHNR-----------<br>------------YPFT | FGPGT<br>KVDIK |
| iPS:434<br>187 | 21-<br>225_56A5 | VK1\|A30/<br>JK3 | | DIQMTQSPSSLSA<br>SVGDRVTITC | RAS--QDIR--<br>----NLLG | WYQQKPGK<br>APKRLIY | A--------<br>ASSLQS | GVPSRFSGSGSG--<br>TEFTLTISSLQPEDFATYYC | LQYNS-----------<br>------------YPFT | FGPGT<br>KVDIR |
| iPS:434<br>199 | 21-<br>225_59F11 | VK1\|A30/<br>JK3 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNR-----------<br>------------YPFT | FGPGT<br>KVDFK |
| iPS:434<br>207 | 21-<br>225_60A3 | VK1\|A30/<br>JK3 | | DIQMTQSPSSLSA<br>SVGDRVTITC | RAS--QGIR--<br>----NDLG | WYQQKPGK<br>APKRLIY | A--------<br>AFSLQS | GVPSRFSGSGSG--<br>TEFTLTISSLQPEDFATYYC | LQHNS-----------<br>------------YPFT | FGPGT<br>KVDIK |
| iPS:434<br>251 | 21-<br>225_62G3 | VK1\|A30/<br>JK3 | | DIHMTQSPSSLSA<br>SVGDRVTITC | RAS--QDIR--<br>----NNLG | WYQQKPGK<br>APKRLIY | T--------<br>ASSLQS | GVPSRFSGSGSG--<br>TEFTLTISSLQPEDFATYYC | LQYNS-----------<br>------------YPFT | FGPGT<br>KVDIK |
| iPS:434<br>263 | 21-<br>225_56H7 | VK1\|A30/<br>JK3 | | DIQLTQSPSSLSA<br>SVGDRVTITC | RAS--QDIR--<br>----NDLG | WYQQRPGK<br>APKRLIY | P--------<br>ASSLLS | GVPSRFSGSGSG--<br>TEFTLTISSLQPEDFATYYC | LQDNS-----------<br>------------YPFT | FGPGT<br>KVDSK |
| iPS:434<br>265 | 21-<br>225_57B2 | VK1\|A30/<br>JK3 | | DIQMTQSPSSLSV<br>SVGDRVTITC | RAS--QGIR--<br>----NALG | WYQQKPGK<br>APKRLIY | A--------<br>ASSLQS | GVPSRFSGSGSG--<br>TEFTLTISSLQPEDFATYYC | LQHNS-----------<br>------------YPFT | FGPGT<br>KVDIK |
| iPS:434<br>271 | 21-<br>225_57A4 | VK1\|A30/<br>JK3 | | DIQMTQSPSSLSA<br>SVGDRVTITC | RAS--QGIR--<br>----NDLG | WYLQKPGK<br>APKRLIY | A--------<br>ASSLLS | GVPSRFSGSGSG--<br>TEFTLTISSLQPEDFATYYC | LQHNS-----------<br>------------YPFT | FGPGT<br>KVDIK |
| iPS:434<br>275 | 21-<br>225_57F4 | VK1\|A30/<br>JK3 | | DIQMTQSPSSLSA<br>SVGDRVTITC | RAS--QVIR--<br>----NDLG | WYQQKPGK<br>APKRLIY | A--------<br>ASSLQS | GVPSRFSGSGSG--<br>TEFTLTISSLQPEDFATYHC | LQYGS-----------<br>------------FPFT | FGPGT<br>KVDIK |
| iPS:434<br>293 | 21-<br>225_58F5 | VK1\|A30/<br>JK3 | | DIQMTQSPSSLSA<br>SVGDRVTITC | RAS--QGIR--<br>----NDLG | WFLQTPGK<br>APKRLIY | A--------<br>ASSLLS | GVPSRFGGSGSG--<br>TEFTLTISSLQPEDFATYYC | LQHNS-----------<br>------------YPFT | FGPGT<br>KVEIK |
| iPS:434<br>299 | 21-<br>225_58D11 | VK1\|A30/<br>JK3 | | DIQMTQSPSSLSA<br>SVGDRVTITC | RAS--QGIR--<br>----SDLD | WYQQKPGK<br>APKRLIY | A--------<br>ASSLQS | GVPSRFSGSGSG--<br>TEFTLAISSLRPEDFATYYC | LQHNN-----------<br>------------FPFT | FGPGT<br>KVDIK |
| iPS:434<br>351 | 21-<br>225_64A12 | VK1\|A30/<br>JK3 | | DIQMTQSPSSLSA<br>SVGDRVTITC | RAS--QGIR--<br>----NDLG | WYQQKPGK<br>APTRLIY | T--------<br>ASTLQS | GVPSRFSGSGSG--<br>TEFTLTISSLQPEDFATYYC | LQHNG-----------<br>------------YPFT | FGPGT<br>KVDIK |
| iPS:434<br>383 | 21-<br>225_66F9 | VK1\|A30/<br>JK3 | | DIQMTQSPSSLSA<br>SVGDRVTITC | RAS--QDIR--<br>----NVLG | WYQQKPGK<br>APKRLIY | T--------<br>ASSLQS | GVPSGFSGSGSG--<br>TEFTLTISSLQPEDFATYYC | LQYNS-----------<br>------------YPFT | FGPGT<br>KVDIK |
| iPS:434<br>399 | 21-<br>225_67B7 | VK1\|A30/<br>JK3 | | DIQMTQSPSSLSA<br>SVGDRVTITC | RAS--QGIR--<br>----NDLG | WFQQKPGK<br>APKRLIY | A--------<br>ASSLQS | GVPSRFSGSGSG--<br>TEFSLTISSLQPEDFATYYC | LHHNS-----------<br>------------YPFK | FGPGT<br>KVDIK |
| iPS:434<br>407 | 21-<br>225_68G8 | VK1\|A30/<br>JK3 | | DIQMTQSPSSLSA<br>SVGDRVTITC | RAS--QGIR--<br>----NNLG | WYQQRPGK<br>APKRLIY | A--------<br>ASSVQS | GVPSRFSGSGSG--<br>TEFTLTISSLQPEDFATYYC | LQYNS-----------<br>------------YPFT | FGPGT<br>KVDIK |
| iPS:434<br>447 | 21-<br>225_71B6 | VK1\|A30/<br>JK3 | | DIQMTQSPSSPSA<br>SVGDRVTITC | RAS--QGIR--<br>----NDLD | WYQQKPGK<br>APQRLIY | A--------<br>ASSLQS | GVPSRFSGSGSG--<br>TEFTLTISSLQPDDFATYYC | LQHNS-----------<br>------------YPFT | FGPGT<br>KVDIK |
| iPS:434<br>449 | 21-<br>225_71H6 | VK1\|A30/<br>JK3 | | DIQMTQSPSSLSA<br>SVGDRVTITC | RAS--QGIR--<br>----NVLG | WYQQKPGK<br>APKRLIY | A--------<br>ASSLQS | GVPSRFSGSGSG--<br>TEFTLTISSLQPEDFATYYC | LQYNS-----------<br>------------YPFT | FGPGT<br>KVDIK |
| iPS:434<br>453 | 21-<br>225_71B11 | VK1\|A30/<br>JK3 | | DIQMTQSPSSLSA<br>SVGDRVTITC | RAS--QGIR--<br>----NDLD | WYQQTPGK<br>APKRLIY | A--------<br>ASSLQS | GVPSRFSGGGSG--<br>TEFSLTISSLQPEDFTTYYC | LQHNT-----------<br>------------YPFT | FGPGT<br>KVDVK |
| iPS:434<br>463 | 21-<br>225_73A6 | VK1\|A30/<br>JK3 | | DIQMTQSPSSLSA<br>SVGDRVTITC | RAS--QGIR--<br>----NDLG | WFQQKPGK<br>APKRLIY | A--------<br>ASNLQS | GVPSRFSGSGSG--<br>TEFTLTISSLQPEDFATYYC | LQHNS-----------<br>------------YPFT | FGPGT<br>KVDIK |
| iPS:434<br>815 | 21-<br>225_74A11 | VK1\|A30/<br>JK3 | | DIQMTQSPSSLSA<br>SVGDRVTITC | RAS--QDIR--<br>----NDLG | WYQQKPGK<br>APKRLIC | A--------<br>ASSLQS | GVPSRFSGSGSG--<br>TEFTLTISSLQPEDFATYFC | LQHND-----------<br>------------YPFT | FGPGT<br>KVDIK |

| | | | | | | | | | | |
|---|---|---|---|---|---|---|---|---|---|---|
| **iPS:434 977** | **21-225_88A5** | **VK1\|A30/ JK3** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHND-----------<br>------------YPFT | FGPGT<br>KVEIK |
| **iPS:435 253** | **21-225_96A4** | **VK1\|A30/ JK3** | | DIQMTQSPSSLSA<br>SVGDRVTITC | RAS--QDIR--<br>----NDLG | WYQQKPGK<br>APKRLIY | G--------<br>VSSLQS | GVPSRFSGSGSG--<br>TEFTLTISSLQREDFATYYC | LQHND-----------<br>------------YPFT | FGRGT<br>KVDIK |
| **iPS:435 511** | **21-225_157C3** | **VK1\|A30/ JK3** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TDFTLTISSLQPDDFATYYC | LQHNS-----------<br>------------YPFT | FGPGT<br>KVDIK |
| **iPS:435 521** | **21-225_157H4** | **VK1\|A30/ JK3** | | DIQMTQSPSSLSA<br>SVGDRVTITC | RAS--QGIR--<br>----NDLG | WYQQKPGK<br>APKRLIY | P--------<br>ASSLQT | GVPSRFSGSGSG--<br>TEFTLTISSLQPEDFATYYC | LQDNS-----------<br>------------YPFT | FGPGT<br>KVDIK |
| **iPS:435 527** | **21-225_157G7** | **VK1\|A30/ JK3** | | DIQMTQSPSSLCA<br>SVGDRVTITC | RAS--QGIR--<br>----NDLG | WYQQKPGK<br>APKRLIN | V--------<br>ASSLQS | GVPSRFSGSGSG--<br>TEFTLTISSVQREDFATYYC | IQDNS-----------<br>------------HPFT | FGPGT<br>KVEIK |
| **iPS:435 533** | **21-225_157H8** | **VK1\|A30/ JK3** | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TDFTLTISSLQPDDFATYYC | LQHNS-----------<br>------------YPFT | FGPGT<br>KVDIK |
| **iPS:435 537** | **21-225_157H12** | **VK1\|A30/ JK3** | | DIQMTQSPSSLSA<br>SVGDRVTITC | RAS--QGIR--<br>----NDFG | WYQQRPGK<br>APKCLIH | A--------<br>ASSLQS | GVPSRFSGSGSG--<br>TEFTLTISSLQPEDFATYYC | LQHYS-----------<br>------------YPFT | FGPGT<br>KVDIK |
| **iPS:435 547** | **21-225_158F5** | **VK1\|A30/ JK3** | | DIQMTQSPSSLSA<br>SVGDRVTITC | RAS--QGIR--<br>----NDLG | WYQQKPGK<br>APKRLIN | A--------<br>ASSLQS | GVPSRFSGSGSG--<br>TEFTLTISSLQPEDFATYYC | LQDNS-----------<br>------------HPFT | FGPGT<br>KVEIK |
| **iPS:435 551** | **21-225_158H6** | **VK1\|A30/ JK3** | | DIQMTQSPSSLSA<br>SVGDRVTITC | RAS--QGIR--<br>----SDLG | WYQQKPGK<br>APKRLIY | T--------<br>ASSLQS | GVPSRFSGSGSG--<br>TEFTLTISSLQPEDFATYYC | LQHNS-----------<br>------------YPFT | FGPGT<br>KVDIK |
| **iPS:435 553** | **21-225_158G8** | **VK1\|A30/ JK3** | | DIQMTQSPSSLSA<br>SVGDRVTITC | RAS--QDIR--<br>----NDLG | WYQQKPGK<br>APKRLMY | T--------<br>ASSLQS | GVPSRFSGSGSG--<br>TEFTLTISSLQPEDFATYYC | LQHNS-----------<br>------------YPFT | FGPGT<br>KVDIK |
| **iPS:435 569** | **21-225_159C5** | **VK1\|A30/ JK3** | | DIQMTQSPSSLSA<br>SVGDRVTITC | RTS--QGIR--<br>----NDLG | WYQQKPGK<br>APKRLIY | A--------<br>ASSLQS | GVPSRFSGSGSG--<br>TDFTLTIISLQPDDFATYYC | LQHNS-----------<br>------------YPFT | FGPGT<br>KVDIK |
| **iPS:435 593** | **21-225_160F4** | **VK1\|A30/ JK3** | | DIQMTQSPSSPSA<br>SVGDRVTITC | RAS--QGIR--<br>----NDLG | WYQQKPGK<br>APKRLIN | V--------<br>ASSLQS | GVPSRFSGSGSG--<br>TEFTLTISSVQPEDFATYYC | IQDNS-----------<br>------------HPFT | FGPGT<br>KVEIK |
| **iPS:435 609** | **21-225_161F7** | **VK1\|A30/ JK3** | | DIQMTQSPSSLSA<br>SVGDRVTITC | RAS--QGIR--<br>----NDLG | WYQQKPGE<br>APKRLIY | A--------<br>ASTLQS | GVPSRFSGSGSG--<br>AEFTVTIGSVQREDFATYYG | LLYIR-----------<br>------------YPFT | FGRGT<br>KVDIK |
| **iPS:435 613** | **21-225_161D11** | **VK1\|A30/ JK3** | | DIQMTQSPSSLSA<br>SVGDRVTITC | RAS--QGIR--<br>----NDLG | WYQQKPGE<br>APKRLIY | A--------<br>ASTLQS | GVPSRFSGSGSG--<br>AEFTLTIGSVQREDFATYYG | LQYNR-----------<br>------------YPFT | FGRGT<br>KVDIK |
| **iPS:435 617** | **21-225_162F2** | **VK1\|A30/ JK3** | | DIQMTQSPSSLCA<br>SVGDRVTITC | RAS--QGIR--<br>----NDLG | WYQQKPGK<br>APKRLIN | A--------<br>ASSLQS | GVPSRFSGSGSG--<br>TEFTLTISSVQPEDFATYYC | IQDNS-----------<br>------------HPFT | FGPGT<br>KVEIK |
| **iPS:435 621** | **21-225_162H3** | **VK1\|A30/ JK3** | | DIQMTQSPSSLSA<br>SVGDRVTITC | RAS--QGIR--<br>----NDLG | WYQQKPGK<br>APKRLIN | A--------<br>ASSLQS | GVPSRFSGSGSG--<br>TEFTLTISSLQPEDFATYYC | LQDNS-----------<br>------------HPFT | FGPGT<br>KVEIK |
| **iPS:435 637** | **21-225_163E2** | **VK1\|A30/ JK3** | | DIQMTQSPSSLSA<br>SVGDRVTITC | RAS--QDIR--<br>----NDLG | WYQQKPGK<br>APTRLIY | P--------<br>ASSLQS | GVPSRFSGSGSG--<br>TEFTLSISSLQPEDFATYYC | LQHNS-----------<br>------------HPFT | FGPGT<br>KVDIK |
| **iPS:435 641** | **21-225_163F9** | **VK1\|A30/ JK3** | | DIQMTQSPSSLSA<br>SVGDRVTITC | RAS--QDIR--<br>----DDLG | WYQQKPGK<br>APKRLIY | P--------<br>ASSLQS | GVPSRFSGSGSG--<br>TEFTLTISSLQPEDFAAYYC | LQDNS-----------<br>------------YPFT | FGPGT<br>KVDIK |
| **iPS:435 643** | **21-225_163G10** | **VK1\|A30/ JK3** | | DIQMTQSPSSLSA<br>SVGDRVTITC | RAS--QDIR--<br>----NNLG | WYQQKPGK<br>APKRLIY | P--------<br>ASSLQS | GVPSRFSGSGSG--<br>TEFTLTISSLQPEDFATYYC | LQDYS-----------<br>------------YPFT | FGPGT<br>KVDIK |
| **iPS:435 719** | **21-225_171A11** | **VK1\|A30/ JK3** | | DIQMTQSPSSLSA<br>SVGDRVTITC | RAS--QGIR--<br>----NNLG | WYQQKPGK<br>APKRLIY | P--------<br>ASSLQS | GVPSRFSGSGSG--<br>TEFTLTISSLQPEDFATYYC | LQDHS-----------<br>------------YPFT | FGPGT<br>KVDIK |
| **iPS:435 769** | **21-225_177B6** | **VK1\|A30/ JK3** | | DIQMTQSPSSLSA<br>SVGDRVTITC | RAS--QDIS--<br>----NDLG | WYQQKPGK<br>APKRLIY | A--------<br>ASSLQS | GVPSRFSGSGSG--<br>TEFTLTINSLQPEDFATYYC | LQLNS-----------<br>------------YPFT | FGPGT<br>KVDIK |

| | | | | | | | | | | |
|---|---|---|---|---|---|---|---|---|---|---|
| iPS:435<br>791 | 21-<br>225_180H7 | VK1\|A30/<br>JK3 | | DIQMTQSPSSLSA<br>SVGDRVTITC | RAS--QGIR--<br>----NDLG | WYQQKPGK<br>APKRLIY | T--------<br>ASSLQS | GVPSRFSGSGSG--<br>TEFTLTISSLQPEDFASYYC | LQHNS-----------<br>------------YPFT | FGPGT<br>KVDFK |
| iPS:435<br>805 | 21-<br>225_181A8 | VK1\|A30/<br>JK3 | | DIQMTQSPSSLSA<br>SVGDRVTITC | RAS--QGIR--<br>----NDLG | WYQQKPGK<br>APKRLIY | T--------<br>ASSLQS | GVPSRFSGSGSG--<br>TDFTLTISSLQPEDFASYYC | LQHNS-----------<br>------------YPFT | FGPGT<br>KVDFK |
| iPS:435<br>879 | 21-<br>225_184H1<br>0 | VK1\|A30/<br>JK3 | | DIQMTQSPSSLSA<br>SVGDRVTITC | RAS--QGIR--<br>----NDLG | WYQQKPGK<br>APKRLIY | I--------<br>ASSLQS | GVPSRFSGSGSG--<br>TEFTLTISSLQPEDFATYYC | LQHNS-----------<br>------------YPFT | FGPGT<br>KVDIK |
| iPS:435<br>881 | 21-<br>225_184D1<br>1 | VK1\|A30/<br>JK3 | | DIQMTQSPSSLSA<br>SVGDRVTITC | RAS--QGIR--<br>----NDLG | WYQQKPGK<br>APKRLIY | I--------<br>ASSLQS | GVPSRFSGSGSG--<br>TEFTLTISSLQPEDFATYYC | LQHNS-----------<br>------------YPFT | FGPGT<br>KVDIK |
| iPS:435<br>921 | 21-<br>225_190D6 | VK1\|A30/<br>JK3 | | DIQMTQSPSSLSA<br>SVGDRVTITC | RAS--QGIR--<br>----NDLG | WYQLKPGK<br>APKRLIY | A--------<br>ASSLQS | GVPSRFSGSGSG--<br>PEFTLTISSLQPEDFATYYC | LQHYS-----------<br>------------FPFT | FGPGT<br>KVDIK |
| iPS:435<br>985 | 21-<br>225_192F6 | VK1\|A30/<br>JK3 | | DIQMTQSPSSLSA<br>SVGDRVTITC | RAS--QGIR--<br>----NDLG | WYQLKPGK<br>APKRLIY | A--------<br>ASSLQS | GVPSRFSGSGSG--<br>PEFTLTISSLQPEDFATYYC | LQHYS-----------<br>------------FPFT | FGPGT<br>KVDIK |
| iPS:436<br>074 | 21-<br>225_194F10 | VK1\|A30/<br>JK3 | | DIQMTQSPSSLSA<br>SVGDRVTITC | RAS--QGIR--<br>----NDLG | WYQLKPGK<br>APKRLIY | A--------<br>ASSLQS | GVPSRFSGSGSG--<br>PEFTLTISSLQPEDFATYYC | LQHYS-----------<br>------------FPFT | FGPGT<br>KVDIK |
| iPS:436<br>092 | 21-<br>225_195B9 | VK1\|A30/<br>JK3 | | DIQMTQSPSSLSA<br>SVGDRVTITC | RAS--QGIR--<br>----KDLG | WYQQKPGK<br>APKRLIY | A--------<br>ASDLQS | GVPSRFSGSGSG--<br>TEFTLTISSLQPEDFATYCC | LQHYR-----------<br>------------YPFT | FGPGT<br>KVDFK |
| iPS:436<br>164 | 21-<br>225_197G1<br>0 | VK1\|A30/<br>JK3 | | DIQMTQSPSSLSA<br>SVGDRVTITC | RAS--QGIR--<br>----NDLG | WYQQKPGK<br>APKRLIY | G--------<br>ASSLQS | GVPSRFSGSGSG--<br>TEFTLTISSLQPEDFATYYC | LQHYR-----------<br>------------YPFT | FGPGT<br>KVDIK |
| iPS:436<br>191 | 21-<br>225_198B9 | VK1\|A30/<br>JK3 | | DIQMTQSPSSLSA<br>SVGDRVTITC | RAS--QGIR--<br>----KDLG | WYQQKPGK<br>APKRLIY | A--------<br>ASSLQS | GVPSRFSGSGSG--<br>TEFTLTISSLQPEDFATYVC | LQHYR-----------<br>------------YPFT | FGPGT<br>KVDFK |
| iPS:436<br>205 | 21-<br>225_199A7 | VK1\|A30/<br>JK3 | | DIQMTQSPSSLSA<br>SVGDRVTITC | RAS--QGIR--<br>----KDLG | WYQQKPGK<br>APKRLIY | A--------<br>ASSLQS | GVPSRFSGSGSG--<br>TEFTLTISSLQPEDFATYVC | LQHYR-----------<br>------------YPFT | FGPGT<br>KVDFK |
| iPS:436<br>214 | 21-<br>225_200F6 | VK1\|A30/<br>JK3 | | DIQMTQSPSSLSA<br>SVGDRVTITC | RAS--QDIR--<br>----NDLG | WYQQKPGK<br>APKRLIY | A--------<br>ASSLQS | GVPSRFSGSGSG--<br>TEFTLTISSLQPEDFATYHC | LQHYR-----------<br>------------YPFT | FGPGT<br>KVDIK |
| iPS:436<br>248 | 21-<br>225_202A3 | VK1\|A30/<br>JK3 | | DIQMSQSPSSLSA<br>SVGDRVTITC | RAS--QGIR--<br>----NDLG | WYQQKPGK<br>APKRLIC | A--------<br>ASRLQS | GVPSRFSGSGSG--<br>TEFTLTISSLQPEDFAIYYC | LQHHD-----------<br>------------YPFT | FGPGT<br>KVDIK |
| iPS:436<br>268 | 21-<br>225_203B9 | VK1\|A30/<br>JK3 | | DIQMTQSPSSLSA<br>SVGDRVTITC | RAS--QGIR--<br>----NDLG | WYQQKPGK<br>APKRLIY | R--------<br>ASSVQN | GVPSRFSGSGSG--<br>TEFTLTISSLQPEDFATYHC | LQHNS-----------<br>------------YPFT | FGPGT<br>KVDIK |
| iPS:436<br>350 | 21-<br>225_210E4 | VK1\|A30/<br>JK3 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPFT | FGPGT<br>KVDIK |
| iPS:436<br>576 | 21-<br>225_225B6 | VK1\|A30/<br>JK3 | | DIQMTQSPSSLSA<br>SVGDRVIITC | RAS--QGMR--<br>----KDLG | WYQQKPGK<br>APKRLIY | A--------<br>ATSLQS | GVPSRFSGSGSG--<br>TEFTLTISSLQPEDFATYYC | LQHNS-----------<br>------------YPFT | FGPGT<br>KVDIK |
| iPS:436<br>578 | 21-<br>225_225D6 | VK1\|A30/<br>JK3 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYCC | LQHNT-----------<br>------------YPFT | FGPGT<br>KVDIK |
| iPS:436<br>582 | 21-<br>225_225F8 | VK1\|A30/<br>JK3 | | DIQMTQSPSSLSA<br>SVGDRVTITC | RAS--QGIR--<br>----NDLG | WYQQKPGK<br>APKRLIY | A--------<br>ASSLLG | GVPSRFSGSGSG--<br>TEFTLTISSLQPEDFATYYC | LQHNS-----------<br>------------YPFT | FGPGT<br>KVDIK |
| iPS:436<br>608 | 21-<br>225_226A9 | VK1\|A30/<br>JK3 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPFT | FGPGT<br>KVDIK |
| iPS:436<br>630 | 21-<br>225_227G3 | VK1\|A30/<br>JK3 | | DIQMTQSPSSLSA<br>SVGDRVTITC | RAS--QGIR--<br>----NDLG | WYQQKPGK<br>APKRLIY | A--------<br>ASSLQS | GVPSRFSGSGSG--<br>TEFTLTISSLQPADFATYYC | LHHNS-----------<br>------------YPFT | FGPGT<br>KVDIK |
| iPS:436<br>634 | 21-<br>225_227H5 | VK1\|A30/<br>JK3 | | DIQMTQSPSSLSA<br>SVGDRVTITC | RAS--QDIR--<br>----NDLG | WYQQKPGK<br>APKRLIY | A--------<br>ASSLQS | GVPSRFSGSGSG--<br>TEFTLTISSLQPEDFATYYC | LQHNS-----------<br>------------YPFT | FGPGT<br>KVDIK |

| | | | | | | | | | | |
|---|---|---|---|---|---|---|---|---|---|---|
| iPS:436 650 | 21-225_227C12 | VK1\|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPFT | FGPGT KVDIK |
| iPS:392 632 | 21-225_16A11 | VK1\|A30/ JK3 | | DIQMTQSPSSLSA SVGDSVTISC | RAS--QDIR-- ----NHLG | WYQRNPGK APKRLIY | A-------- ASSLQS | GVPSRFSGSGSG-- TEFTLTISSLQPEDFATYYC | LQYNS----------- ------------YPFT | FGPGT KVDIK |
| iPS:392 684 | 21-225_17F4 | VK1\|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPFT | FGPGT KVDIK |
| iPS:392 732 | 21-225_17E5 | VK1\|A30/ JK3 | | DIQMTQSPSSLSA SVGDRVTITC | RAS--QGIR-- ----NDLG | WYQQKPGK APKRLIY | T-------- ASSLQS | GVPSRFSGSGSG-- TEFTLTISSLQPEDFATYYC | LQHNS----------- ------------YPLT | FGPGT KVVIK |
| iPS:392 778 | 21-225_22H3 | VK1\|A30/ JK3 | | DIQMTQSPSSLSA SVGDRVTITC | RAS--QDIR-- ----NNLG | WYQQKPGK APKRLIY | P-------- ASSLQT | GVPSRFSGSGSG-- TEFTLTISSLQPEDFATYFC | LQDNS----------- ------------YPFT | FGPGT KVDIK |
| iPS:392 912 | 21-225_25A9 | VK1\|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPFT | FGPGT KVDIK |
| iPS:392 934 | 21-225_27D5 | VK1\|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPFT | FGPGT KVDIK |
| iPS:392 940 | 21-225_29D9 | VK1\|A30/ JK3 | | DIQMTQSPSSLSA SVGDRVTITC | RAS--QGIR-- ----NDLG | WFQQKPGK APKRLIY | A-------- ASSLQS | GVPSRFSGSGSG-- TEFTLTISSLQPEDFATYYC | LQHNT----------- ------------YPFT | FGPGT KVDFK |
| iPS:392 948 | 21-225_25G5 | VK1\|A30/ JK3 | | DIQMTQSPSSLSA SVGDRVTITC | RAS--QDIR-- ----NDLG | WYQQKPGK APKRLIY | A-------- ASSLQS | GVPSRFSGSGSG-- TEFTLTVSSLQPEDFATYYC | LQHNS----------- ------------YPFT | FGPGT KVDIK |
| iPS:392 968 | 21-225_25B6 | VK1\|A30/ JK3 | | DIQMTQSPSSLSA SVGDRVTITC | RAS--QGIR-- ----NDLG | WYQQKPGK APKRLIY | R-------- ASSLQS | GVPSRFSGSGSG-- TEFTLTISSLQPEDFATYYC | LQHNS----------- ------------YPFT | FGPGT KVDIK |
| iPS:392 978 | 21-225_28B8 | VK1\|A30/ JK3 | | DIQMTQSPSSLSA SVGDRVTITC | RAS--QGIR-- ----NDLG | WYQHKPGK APKRLIY | A-------- ASSLQS | GVPSRFSGSGSG-- TEFTLTISSLQPEDFATYYC | LQHNS----------- ------------YPFT | FGPGT KVDIN |
| iPS:392 998 | 21-225_28A9 | VK1\|A30/ JK3 | | DIQMTQSPSSLSA SVGDRVTITC | RAS--QGIR-- ----NDLG | WYQQKPGK APKRLIY | A-------- ASSLQN | GVPSRFSGSGSG-- TEFTLTISSLQPEDFATYYC | LQHNR----------- ------------YPFT | FGPGT KVDIK |
| iPS:393 000 | 21-225_29D7 | VK1\|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPFT | FGPGT KVDIK |
| iPS:393 006 | 21-225_31G9 | VK1\|A30/ JK3 | | DIQMTQSPSSLSA SIGDRVTITC | RAS--QGIR-- ----NDLG | WYQQKPGK APKRLIY | P-------- ASSLQS | GVPSRFSGSGSG-- TEFTLTISSLQPEDFATYYC | LQDNS----------- ------------YPFT | FGPGT KVDIK |
| iPS:393 022 | 21-225_30H11 | VK1\|A30/ JK3 | | DIQMTQSPSSLSA SVGDRVTITC | RAS--QGIR-- ----NDLG | WYQQKPGK APKRLVY | P-------- ASSLQS | GVPSRFSGSGSG-- TEFTLTISSLQPEDFATYYC | LQDNS----------- ------------HPFT | FGPGT KVDIT |
| iPS:393 038 | 21-225_29D8 | VK1\|A30/ JK3 | | DIQMTQSPSSLSA SVGDRVTITC | RAS--QGIR-- ----NDLG | WYQQKPGK APKRLIY | T-------- ASSLQS | GVPSRFSGSGSG-- TEFTLTISSLQPEDFATYYC | LQHNS----------- ------------YPFT | FGPGT KVDIK |
| iPS:393 822 | 21-225_15B11 | VK1\|A30/ JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYYC | LQHNS----------- ------------YPFT | FGPGT KVDIK |
| iPS:393 944 | 21-225_14D6 | VK1\|A30/ JK3 | | DIQMTQSPSSLSA SVGDSVTITC | RAS--QDIR-- ----NHLG | WYQHKPGK APKRLIY | A-------- ASSLQS | GVPSRFSGSGSG-- TEFTLTISSLQPEDFATYYC | LQYNS----------- ------------YPFT | FGPGT KVDIN |
| iPS:394 033 | 21-225_5F4 | VK1\|A30/ JK3 | | DIQMTQSPSSLSA SVGDRVTITC | RAS--QGIR-- ----NHLG | WYQQKPGK APKRLIY | A-------- ASSLQS | GVPSRFSGSGSG-- TEFTLTVSSLQPEDFATYYC | LQYNG----------- ------------YPFT | FGPGT KVDIK |
| iPS:394 069 | 21-225_16H1 | VK1\|A30/ JK3 | | DIQMTQSPSSLSA SVGDRVTITC | RAS--QGIR-- ----DILG | WYQQKPGK APKRLIY | A-------- ASSLQN | GVPSRFSGSGSG-- TEFTLTISSLQPEDFATYYC | LQYHS----------- ------------YPFT | FGPGT KVDVK |
| iPS:402 229 | 21-225_16H9 | VK1\|A30/ JK3 | | DIQMTQSPSSLSA SVGDRVTITC | RAS--QGIR-- ----NYLG | WFQQKPGK APKRLIY | G-------- ASSLQS | GVPSRISGSGSG-- TEFTLTISSLQPEDFATYYC | LQYHS----------- ------------YLFT | FGPGT KVDIK |
| | | Germline | | K_FR1 | K_CDR1 | K_FR2 | K_CDR2 | K_FR3 | K_CDR3 | K_FR4 |
| | VK1\|O12/JK1 | | 32143 | DIQMTQSPSSLSA SVGDRVTITC | RAS--QSIS-- ----SYLN | WYQQKPGK APKLLIY | A-------- ASSLQS | GVPSRFSGSGSG-- TDFTLTISSLQPEDFATYYC | QQSYS----------- ------------TPWT | FGQGT KVEIK |

| | | | | | | | | | | |
|---|---|---|---|---|---|---|---|---|---|---|
| iPS:468 848 | 21-225_54B1 | VK1\|O12/JK1 | | DIQMTQSPSSLSA<br>SIGDRVTITC | RAS--QNIS--<br>----SYLN | WYQQKPGK<br>APKFLIY | A--------<br>ASSLHS | GVPPRFSGSGSG--<br>TDFTLTISSLQPEDFAIYYC | QQSYR-----------<br>-----------TPLWT | FGQGT<br>KVEIK |
| iPS:434 239 | 21-225_58F1 | VK1\|O12/JK1 | | DIQMTQSPSSLSA<br>SVGDRVTITC | RAS--QSIT--<br>----NFLN | WYQQKPGK<br>APKLLIF | A--------<br>ASSLQS | GIPSRFSGSGSG--<br>TDFTLTISSLQPEDFATYYC | QQSYS-----------<br>------------IPWT | FGQGT<br>KVEIK |
| iPS:435 513 | 21-225_157F3 | VK1\|O12/JK1 | | DIQMTQSPSSLSA<br>SVGDRVTITC | RAS--QNIS--<br>----SYLN | WYQLKPGK<br>APKLLIY | T--------<br>ASSLQS | GVPSRFSGSGSG--<br>TDFTLTISSLQPEDFATYYC | QQSYN-----------<br>-----------TPTWT | FGQGT<br>KVEIK |
| iPS:435 729 | 21-225_173E7 | VK1\|O12/JK1 | | DIQMTQSPSSRSA<br>CIGDRATITY | RAS--QTIS--<br>----NYLN | WYQQKPGK<br>APKLLIY | A--------<br>ASSLQI | GVPSRFSGSGSG--<br>TDFTLTISSVQPEDFATYFC | QQSYR-----------<br>-----------TPQWT | FGQGT<br>KVEIK |
| iPS:435 753 | 21-225_175G10 | VK1\|O12/JK1 | | DIQMTQSPSSLSA<br>SVGDRVIITC | RAS--QTIG--<br>----NYLN | WYQQKPGR<br>APKLLIY | A--------<br>ASSLHS | GVPSGFSGSGSG--<br>TDFTLTISSLQPEDFATYFC | QQSYR-----------<br>-----------TPQWT | FGQGT<br>KVEIK |
| iPS:435 799 | 21-225_181G3 | VK1\|O12/JK1 | | DIQMTQSPSSLSA<br>SVGDRVNITC | RAS--HSIS--<br>----NYLN | WYQQKAGK<br>APNLLIY | T--------<br>TLNLQS | GVPSRFSGSGSG--<br>TDFTLTISSLQPEDFATYYC | QQSYS-----------<br>-----------SPPWT | FGQGT<br>KVEIK |
| iPS:435 813 | 21-225_183A12 | VK1\|O12/JK1 | | DIQMTQSPSSLCA<br>SVGDRVTITC | RAS--RNIS--<br>----NYLN | WYQQKPGK<br>APKLLIY | V--------<br>VSSLQS | GVPSRFSGSGSG--<br>TDFTLTISSLQPEDFATYYC | QQSYS-----------<br>-----------SPPWT | FGQGT<br>KVDIR |
| iPS:436 003 | 21-225_192G10 | VK1\|O12/JK1 | | DIQMTQSPSSLSA<br>SVGDRVTITC | RAS--QSIS--<br>----NYLN | WYQQTPGK<br>APKLLIY | A--------<br>ESSLQS | GVPSRFSGSGSG--<br>TDFTLTISSLQPEDFATYSC | QQSYS-----------<br>-----------SPPWT | FGQGT<br>KVEIK |
| iPS:436 212 | 21-225_200G1 | VK1\|O12/JK1 | | DIQMTQSPSSLSA<br>SVGDRVTITC | RAS--QSIS--<br>----SYLN | WFQQKPGK<br>APKLLIY | A--------<br>ESSLQS | GVPSRFSGSGSG--<br>TDFTLTISSLQPEDFATYSC | QQSYS-----------<br>-----------SPPWT | FGQGT<br>KVEFK |
| iPS:392 730 | 21-225_17A1 | VK1\|O12/JK1 | | DIQMTQSPSSLSA<br>SVGDRVTITC | RAS--QNIN--<br>----NYLN | WYQQKPGK<br>GPKVLIF | T--------<br>TSSLQS | GVPSRFSGSGSG--<br>TDFTLTISSLQPEDFATYYC | QQSYT-----------<br>-----------TPTWT | FGQGT<br>KVEIK |
| iPS:392 736 | 21-225_17B12 | VK1\|O12/JK1 | | DIQMTQSPSSLSA<br>SVGDRVSITC | RAS--QNIN--<br>----NYLN | WYQQKPGK<br>GPKVLIL | T--------<br>ASSLQS | GVPSRFSGSGSG--<br>TDFTLTISSLQPEDFATYYC | QQTYT-----------<br>-----------TPTWT | FGQGT<br>KVEIK |
| iPS:392 766 | 21-225_23H4 | VK1\|O12/JK1 | | DIQMTQSPSSLSA<br>SVGDRVTITC | RAS--QSIS--<br>----RYLN | WYQQKPGR<br>APKLLIC | S--------<br>TSSLQS | GVPSRFSGSGSG--<br>TDFTLTISSLQPEDFATYYC | QQSYS-----------<br>-----------TPTWT | FGQGT<br>KVEIR |
| iPS:392 770 | 21-225_20C10 | VK1\|O12/JK1 | | DIHMTQSPSSLSA<br>SVGDKVSITC | RAS--HHIS--<br>----NYLN | WYQQKPGK<br>GPKVLIL | T--------<br>ASSLQS | GVPSRFSGSGSG--<br>TDFTLTISSLQPEDFATYYC | QQSYT-----------<br>-----------TPTWT | FGQGT<br>KVEIK |
| iPS:392 808 | 21-225_20F8 | VK1\|O12/JK1 | | DIQMTQSPSSLSA<br>SVGDRVTITC | RAS--QSIS--<br>----RYLN | WYQQKPGK<br>APELLIY | A--------<br>ASSLQS | GVPSRFSGSGSG--<br>TDFILTISSLQPEDFATYYC | QQSYN-----------<br>-----------TPTWT | FGQGT<br>KVEIK |
| iPS:392 954 | 21-225_26A10 | VK1\|O12/JK1 | | DIQMTQSPSSLSA<br>SVGDRITITC | RAS--QSIS--<br>----SYLN | WYQQKPGK<br>APKVLIY | A--------<br>ASSLQS | GVPSRFSGSGSG--<br>TDFTLTISSLQPEDFATYYC | QQSYS-----------<br>-----------TPTWT | FGQGT<br>KVEIK |
| iPS:393 878 | 21-225_7G12 | VK1\|O12/JK1 | | DIQMTQSPSSLSA<br>SVGDRVSITC | RAS--QNIN--<br>----NYLN | WYQQKPGK<br>GPKVLIL | T--------<br>ASSLQS | GVPSRFSGSGSG--<br>TDFTLTINSLQPEDFATYYC | QQSYT-----------<br>-----------TPTWT | FGQGT<br>KVEIK |
| iPS:398 474 | 21-225_17B10 | VK1\|O12/JK1 | | DIQMTQSPSSLSA<br>SVGDRVTITC | RSS--QSIN--<br>----SYLN | WYQQKPGK<br>APKLLIF | A--------<br>ASSLHS | GVPSRFSGSGSG--<br>TDFTLTISSLQPEDFATYYC | QQGYN-----------<br>-----------TPTWT | FGQGT<br>KVEIN |
| | | Germline | | K_FR1 | K_CDR1 | K_FR2 | K_CDR2 | K_FR3 | K_CDR3 | K_FR4 |
| | VK4\|B3/JK2 | | 32144 | DIVMTQSPDSLAV<br>SLGERATINC | KSS--<br>QSVLYSSNNKN<br>YLA | WYQQKPGQ<br>PPKLLIY | W--------<br>ASTRES | GVPDRFSGSGSG--<br>TDFTLTISSLQAEDVAVYYC | QQYYS-----------<br>------------TPYT | FGQGT<br>KLEIK |
| iPS:468 850 | 21-225_63F4 | VK4\|B3/JK2 | | DIVMTQSPDSLAV<br>SLGERATINC | KSS--<br>QSVLSSSNNNN<br>YLA | WYQQKPGQ<br>PPKLLIY | W--------<br>ASTRES | GIPDRFSGSGSG--<br>TGFTLTISSLQAEDVAVYYC | QQYYT-----------<br>------------TPCS | FGQGT<br>KLEIN |
| iPS:468 852 | 21-225_71F3 | VK4\|B3/JK2 | | DIVMTQSPDSLAV<br>SLGARATINC | KSS--<br>QSVLSNSNNNN | WYQQKPGQ<br>PPKLLIY | W--------<br>ASTRES | GIPDRFSGSGSG--<br>TDFTLTINSLQAEDVAVYYC | QQYYT-----------<br>------------TPCS | FGQGT<br>KLEIK |

| | | | | | | | | | | |
|---|---|---|---|---|---|---|---|---|---|---|
| **iPS:468 870** | **21-225_74A8** | **VK4\|B3/JK2** | | DIVMTQSPDSLAV SLGERATINC | KSS--QSVLYSSNSHN YLA | WYQQKPGQ PPKLLIY | W--------ASTRES | GVPDRFSGSGSG--TDFTLTISSLQAEDVAVYYC | QQYYS-----------------------TPCS | FGQGT KLEIK |
| **iPS:434 211** | **21-225_60F3** | **VK4\|B3/JK2** | | DIVMTQSPDSLTV SLGERATINC | KSS--QSVLYSSNNKN YLA | WYQQKPGQ PPKLLIY | W--------ASTRES | GVPDRFSGSGSG--TDFTLTISSLQAEDVAIYYC | QQYYS-----------------------TPCS | FGQGT KLEIN |
| **iPS:434 235** | **21-225_61E3** | **VK4\|B3/JK2** | | DIVMTQSPDSLAV SLGERATINC | KSS--QSVLHISNNNN YLA | WYQQQPGQ PPKLLIY | W--------ASIRES | GVPDRFSGSGSG--TDFTLTISSLQAEDVAVYYC | QQYYS-----------------------IPCS | FGQGT KLEIK |
| **iPS:434 287** | **21-225_57F12** | **VK4\|B3/JK2** | | DIVMTQSPDSLAV SLGERATINC | KSS--QSVLFSSNNYN YLA | WYQQKTGQ PPKLIIY | W--------ASTRES | GVPDRFSGSGSG--TDFTLTISSLQAEDVAIYYC | QQYYS-----------------------NPCS | FGQGT KLEIK |
| **iPS:434 305** | **21-225_59E1** | **VK4\|B3/JK2** | | DIVMTQSPDSLAV SLGERATINC | KSS--QSVLYSSNNNN YLA | WYQQRPGQ PPKLLIY | W--------SSTRES | GVPDRFSGSGSG--TDFTLTISSLQAEDVAVYYC | QQYFS-----------------------IPCS | FGQGT KLEIK |
| **iPS:434 443** | **21-225_71G3** | **VK4\|B3/JK2** | | DIVMTQSPDSLAV SLGERVAINC | KSS--QSVLHSSNNNN YLD | WYQQKPGQ LPKLLIF | W--------ASTREF | GVPDRFSGSGFG--TDFTLTISSLQAEDVADYYC | QQYYI-----------------------TPCS | FGQGT KLEIK |
| **iPS:434 483** | **21-225_74C12** | **VK4\|B3/JK2** | | DIVMTQSPDSLAV SLGERATINC | KSS--QSVLYSSNNAN YLA | WYQQKPGQ PPNLLIY | W--------ASTRES | GVPDRFSGSGSG--TDFTLTISSLQAEDVAVYYC | QQYYS-----------------------TPCS | FGQGT KLEIK |
| **iPS:434 613** | **21-225_77D12** | **VK4\|B3/JK2** | | DIVMTQSPDSLAV SLGARATINC | RSS--QSVLYSSNNYN YLA | WYQQKPGQ PPKLLIY | W--------ASTRDS | GVPDRFSGSGSG--TDFTLTISSLQAEDVAVYYC | QQYYT-----------------------TPCS | FGQGT KLEIK |
| **iPS:434 635** | **21-225_78E6** | **VK4\|B3/JK2** | | DIVMTQSPDSLAV SLGERATINC | KSS--QSVLYSSNSHN YLA | WYQQKPGQ PPKLLIY | W--------ASIRES | GVPDRFSGSGSG--TDFTLSISSLQAEDVAVYYC | QQYYS-----------------------TPCS | FGQGT KLEIK |
| **iPS:434 679** | **21-225_79G7** | **VK4\|B3/JK2** | | DIVMTQSPDSLAV SLGERATINC | KSS--QSVLYSSNSHN YLA | WYQQKPGQ PPKLLIF | W--------ASIRES | GVPDRFSGSGSG--TDFTLSISSMQAEDVAVYYC | QQYYS-----------------------TPCS | FGQGT KLEIK |
| **iPS:434 909** | **21-225_85C11** | **VK4\|B3/JK2** | | DIVMTQSPDSLAV SLGERATINC | KSS--QSVLYSSNSHN FLA | WYQQNPGQ PPKLLIF | W--------AFIRES | GVPEGFSGSGSG--ADFTLSISGLQAEDVAVYYC | QQYYS-----------------------TPCS | FGQGT KLEIK |
| **iPS:434 959** | **21-225_87E10** | **VK4\|B3/JK2** | | DIVMTQSPDSLAV SLGERATIKC | KSS--QSVLHSSNNMN YLA | WYQQKPGQ PPKLLIY | W--------ASTRKS | GVPDRFSGTGSG--TDFTLTISSLQAEDVAVYYC | QQYYS-----------------------SPCS | FGQGT KLKIK |
| **iPS:435 299** | **21-225_146D4** | **VK4\|B3/JK2** | | DIVMTQSPDSLAV SLGERATINC | KSS--QSVLYSSNNNN YLA | WYQQKPGQ PPKLVIY | W--------ASTRES | GVPDRFSGSGSG--TDFTLTISSLQAEDVAVYYC | QQYYS-----------------------TPCS | FGQGT KLEIK |
| **iPS:435 305** | **21-225_146C9** | **VK4\|B3/JK2** | | DIVMTQSPDSLAV SLGERATINC | KSS--QSVLHSSNNYN YLA | WYQQKPGQ PPKVLIY | W--------ASTRKS | GVPDRFSGSGSG--TDFTLTISSLQAEDVAVYYC | QQYYS-----------------------TPCS | FGQGT KLEIK |
| **iPS:435 309** | **21-225_146F9** | **VK4\|B3/JK2** | | DIVMTQSPDSLAV SLGERATINC | KSS--QNILHSSNNNN YLA | WYQQKPGQ PPYLLIY | W--------ASTRES | GVPDRFSGSGSG--TDFTLTITSLQAEDVAVYYC | QQYYT-----------------------TPCS | FGQGT KLEIT |
| **iPS:435 323** | **21-225_147D5** | **VK4\|B3/JK2** | | DIVMTQSPDSLAV SLGERATINC | KSS--QSVLYSSNNNN | WYQQKPGQ PPKLLIY | W--------ASTRES | GVPDRFSGSGSG--TDFTLSISSLQAEDVAVYYC | HQYYS-----------------------TPCS | FGQGT KLEIK |

| | | | | | | | | | |
|---|---|---|---|---|---|---|---|---|---|
| **iPS:435 399** | **21-225_150D2** | **VK4\|B3/J K2** | | DIVMTQSPDSLAV SLGERATIKC | KSS-- QSVLYRSNSKK YLT | WYQQKPGQ PPKLFIY | W-------- ASTRKS | GVPDRFSGSGSG-- TDFTLTISNLQAEDVAVYFC | QQYFS----------- ------------TPYN | FGQGT KREIK |
| **iPS:435 435** | **21-225_152H3** | **VK4\|B3/J K2** | | DIVMTQSPDSLAV SLGEKATINC | KSS-- QSVLHSSNNYN YLT | WYQQKPGQ PPKLLIY | W-------- ASTRKS | GVPDRFSGSGSG-- TDFTLTISSLQAEDVAVYYC | QQYYS----------- ------------TPCS | FGQGT KLEIK |
| **iPS:435 451** | **21-225_152D10** | **VK4\|B3/J K2** | | GIVMTQSPDSLAV SLGARATIDC | KSS-- QSVLYSSNNKN YLA | WYQQKPGQ PPKLLIY | W-------- ASTRES | GVPDRFSGSGSG-- TDFTLTIYSLQAEDVAVYYC | QQYY------------ ------------RSPS | FGQGT KLEIK |
| **iPS:435 459** | **21-225_152E12** | **VK4\|B3/J K2** | | AVVMTQSPDSLAV SLGERATINC | TSS-- QSILHSSNNYN YLA | WYQQKPGQ PPKLLIY | W-------- ASTRES | GVPDRFSGSGSG-- TDFTLTISSLQAEDVAVYYC | QQYYS----------- ------------GPCS | FGQGT KLEIK |
| **iPS:435 467** | **21-225_153B9** | **VK4\|B3/J K2** | | GIVMTQFPDSLAV SLGERATINC | KSS-- QSVLYSSNNKN YLA | WYQQKPGQ PHKLLIY | W-------- ASTREF | GVPDRFSGSGCG-- TDFTLTIYSVQAEDVAVYYC | QQYN------------ ------------RSLS | FGQGT KLEIK |
| **iPS:435 471** | **21-225_153F11** | **VK4\|B3/J K2** | | DIVMTQSPDSLAV SLGERATINC | KSS-- QSVLYSSNNYK YLA | WYQQKPGQ PPNLLIY | W-------- ASTRKS | GVPDRFSGSGSG-- TDFTLTISSLQAEDVAVYYC | QQYYS----------- ------------TPCS | FGQGT KLEIK |
| **iPS:435 475** | **21-225_154H6** | **VK4\|B3/J K2** | | DIVMTQSPDSLAV SLGERATINC | KSS-- QSVLHSSNNYN YLA | WYQQKPGQ PPKLLIY | W-------- TSTRKS | GVPDRFSGSGSG-- THFTLSISSLQAEDVAVYYC | QHYYS----------- ------------TPCS | FGQGT KLEIK |
| **iPS:435 491** | **21-225_155E5** | **VK4\|B3/J K2** | | DIVMTQSPDSLAV SLGERATINC | KSS-- QSVLSSSNNNN YLA | WYQQKPGQ PPKLLIY | W-------- ASTRKS | GVPDRFSGSGSG-- TDFTLTISSLQAEDVAVYYC | QQYYS----------- ------------TPCS | FGQGT KLEIK |
| **iPS:435 495** | **21-225_155B6** | **VK4\|B3/J K2** | | DIVMTQSPDSLAV SLGERATINC | KSS-- QSVLHSSNNYN YLA | WYQQKPGQ PPKLLIY | W-------- TSTRES | GVPDRFSGSGSG-- TDFTLTISSLQAEDVAVYYC | QQYYS----------- ------------TPCS | FGQGT KLEIK |
| **iPS:435 501** | **21-225_156H1** | **VK4\|B3/J K2** | | DIVMTQSPDSLAV SLGERATINC | KSS-- QSVLYSSNNNN YLA | WYQQKPGQ PPKLLIY | W-------- ASTRES | GVPARFSGSGSG-- TDFTLTISSLQAEDVAVYYC | HQYYS----------- ------------TPCS | FGQGT KLEIK |
| **iPS:435 589** | **21-225_160A4** | **VK4\|B3/J K2** | | DIVMTQSPDSLAV SLGERATINC | KSS-- QSVLHSSNNNN YLA | WYQQKPGQ PPKLLIY | W-------- ASTRES | GVPDRFSGSGSG-- TDFTLTISSLQAEDVAVYYC | QQYYN----------- ------------SPCS | FGQGT KLEIK |
| **iPS:435 727** | **21-225_172E11** | **VK4\|B3/J K2** | | DIVMTQSPDSLAV SLGERATINC | KSS-- QSVLHSSNNNN YLA | WYQQKPGQ PPKLLIY | W-------- ASTRES | GVPDRFSGSGSG-- TDFILTISGLQAEDVAVYYC | QQYFT----------- ------------TPCS | FGQGT KLEIK |
| **iPS:436 560** | **21-225_224F11** | **VK4\|B3/J K2** | | NIVMTQSPDSLAV SLDERATINC | KSS-- QSVLSSSNNHN YLA | WYQQKPGQ PPKMLIY | W-------- ASTRES | GVPDRFSGSGSG-- TDFTLTISSLQAEDVAVYYC | QQYYT----------- ------------TPCS | FGQGT KLEIK |
| **iPS:436 584** | **21-225_225B9** | **VK4\|B3/J K2** | | DIVMTQSPDSLAV SLGERATINC | KSS-- QSVLYSSNNNN YLA | WYQQKPGQ PPKLLIF | W-------- ASTRES | GVPDRFSGSGSG-- TDFTLTISSLQAEDVAVYYC | QHSKS----------- ------------IPGK | FGQGI KLEIQ |
| **iPS:436 588** | **21-225_225F12** | **VK4\|B3/J K2** | | DIVMTQSPDSLAV SLGERATINC | KSS-- QSVLYSSNNQN YLA | WYQQKPGQ PPRLLIY | W-------- TSTRES | GVPDRFSGSGSG-- TDFTLTISNLQAEDVAVYYC | QQYYI----------- ------------TPCS | FGQGT KLEIK |
| **iPS:436** | **21-** | **VK4\|B3/J** | | DIVMTQSPDSLAV | KSS-- | WYQQKPGQ | W-------- | GVPDRFSGSGSG-- | QQYYI----------- | FGQGT |

| | | | | | | | | | | |
|---|---|---|---|---|---|---|---|---|---|---|
| iPS:436 598 | 21-225_226D6 | VK4\|B3/JK2 | | DIVMTQSPDSLAV SLGERATINC | RSS-- QSILYISNNKN YLA | WYQQKPGQ PPKMLIY | W-------- ASTRES | GVPDRFSGSGSG-- TDFTLTISSLQAEDVAVYYC | QQYYS----------- ------------SPCS | FGQGT KLEIK |
| iPS:436 636 | 21-225_227E6 | VK4\|B3/JK2 | | DIVMTQSPDSLTV SLGERATINC | KSS-- QSVLYSSNDKN YLA | WYQQKPGQ PPKMLIY | W-------- ASTRES | GVPDRFSGSGSG-- TDFTLTISSLQAEDVAVYYC | QQYYI----------- ------------TPCS | FGQGT KLEIK |
| iPS:436 644 | 21-225_227G9 | VK4\|B3/JK2 | | DIVMTQSPDSLAV SLGERATINC | KSS-- QSVLHSSNNNN YLA | WYQQKPGQ PPKLLIY | W-------- GSTRES | GVPDRFSGSGSG-- TDFTLTISSLQAEDVAVYYC | QQYYS----------- ------------APYS | FGQGT KLEIK |
| iPS:436 646 | 21-225_227D11 | VK4\|B3/JK2 | | DIVMTQSPDSLAV SLDERATINC | KSS-- QSVLHSSNNNN YLA | WYQQKPGQ PPKLLIY | W-------- ASTRES | GIPDRFSGSGSG-- TDFTLTISSLQAEDVAVYYC | QQYYN----------- ------------TPCS | FGQGT KLEIK |
| iPS:437 363 | 21-225_74C10 | VK4\|B3/JK2 | | DIVMTQSPDSLAV SLGERATINC | KSS-- QSVLYSSNNAN YLA | WYQQKPGQ PPNLLIY | W-------- ASTRES | GVPDRFSGSGSG-- TDFTLTISSLQAEDVAVYYC | QQYYS----------- ------------TPCS | FGQGT KLEIK |
| iPS:451 131 | 21-225_160A7 | VK4\|B3/JK2 | | DIVLTQSPDSPAV SLGERATINC | KSS-- QSVLSNSHNNN YLA | WYQQRPGH PHKLLIF | W-------- ASTRES | GVPDRFSGSGYG-- TDFTLTISSLQAEDVAVYYC | QQYYS----------- ------------TPCS | FGQGT KLEIK |
| iPS:393 088 | 21-225_33D1 | VK4\|B3/JK2 | | DIVMTQSPDSLSV SLGERATINC | KSI-- QSVLYRSNNKN YLT | WYQQKPGQ PRKLFIY | W-------- ASTRES | GVLDRFSGSGCG-- TDFTLTIISLQAEDVALYYC | QQYYS----------- ------------SPCS | FGQGT KLEIK |
| iPS:394 085 | 21-225_8B11 | VK4\|B3/JK2 | | DIVMTQSPDSLAV SLGERATINC | KSS-- QNVLYNSNNNN YLA | WYQQKPGQ PPKLLIY | W-------- ASTRKS | GVPDRFSGSGSG-- TDFTLTISSLQAEDVAVYYC | QQYYT----------- ------------TPCS | FGQGT KLEIK |
| iPS:398 496 | 21-225_22D2 | VK4\|B3/JK2 | | DIVMTQSPDSLAV SLGERATITC | KSS-- QSVLHSSNNNN YLA | WYQQKPGQ PPKLLIY | W-------- ASTRKS | GVPDRFSGSGSG-- TDFTLTISSLQAEDVAVYFC | QQYYS----------- ------------TPCS | FGQGT KLEIK |
| iPS:398 512 | 21-225_25E12 | VK4\|B3/JK2 | | DIVLTQSPDFLAM SLGERATINC | KSS-- QSVLYHSNNYN YLA | WYQQKPKQ PPKLLIY | W-------- ASTRES | GVPDRFSGSGSG-- TYFTLTISSLQAEDVAVYYC | QQYYS----------- ------------TPCS | FGQGT NLEIK |
| iPS:398 522 | 21-225_32A1 | VK4\|B3/JK2 | | DIVMTQSPDSLAV SLGERATINC | KSS-- QSVLYSSNNYN YLA | WYQLKPGQ PPKLLIY | W-------- ASTRKS | GVPDRFSGSGSG-- TDFTLTISSLQTEDVALYYC | QQYYT----------- ------------SPCS | FGQGT KLEIK |
| iPS:398 524 | 21-225_32A5 | VK4\|B3/JK2 | | DIVMTQSPDSLAV SLGERATINC | KSS-- QSVLHSSNNKN YLA | WYQQKPGQ PPKLLIY | W-------- ASTRES | GVPDRFSGSGSG-- TDFTLAISSLQAEDVALYHC | QQYYS----------- ------------SPCS | FGQGT GLEIK |
| iPS:398 538 | 21-225_34H7 | VK4\|B3/JK2 | | DIVMTQSPDSLAV SLGERATINC | KSS-- QSVLYSSNNYN YLA | WYQLKPGQ PPKLLIY | W-------- ASTRKS | GVPDRFSGSGSG-- TDFTLTISSLQTEDVALYYC | QQYYT----------- ------------SPCS | FGQGT KLEIK |
| | | **Germline** | | **K_FR1** | **K_CDR1** | **K_FR2** | **K_CDR2** | **K_FR3** | **K_CDR3** | **K_FR4** |
| | **VK2\|A17/JK4** | | 32145 | DVVMTQSPLSLPV TLGQPASISC | RSS-- QSLVYSD- GNTYLN | WFQQRPGQ SPRRLIY | K-------- VSNRDS | GVPDRFSGSGSG-- TDFTLKISRVEAEDVGVYYC | MQGTH----------- ------------WPLT | FGGGT KVEIK |
| iPS:468 854 | 21-225_72C4 | VK2\|A17/JK4 | | DVVMTQSPLSLPV TLGQPASISC | RSG-- QSLVYSD- GNTYLN | WFQQRPGQ SPRRLIY | E-------- VSKWDS | GVPDRFSGSGSG-- TNFTLKISRVEAEDVGVFYC | MQGTH----------- ------------WPLT | FGGGT KVEIK |

| | | | | | | | | | | |
|---|---|---|---|---|---|---|---|---|---|---|
| **iPS:437 250** | **21-225_148C6** | **VK2\|A17/ JK4** | | DVVMTQSPLSLPV TLGQPASISC | RSS-- QSLVYSD- GNTYLN | WFQQRPGQ SPRRLIY | K-------- VSNWDS | GVPDRFSGSGSG-- TDFTLKISRVEAEDVGVYYC | MQGTH----------- ------------WSLT | FGGGT KVEIK |
| **iPS:437 252** | **21-225_148H1 1** | **VK2\|A17/ JK4** | | DVVMTQSPLSLPV TLGQPASISC | RSS-- QSLVYSD- GNTYLN | WFQQRPGQ SPRRLIY | K-------- VSNWDS | GVPDRFSGSGSG-- TDFTLKISRVEAEDVGVYYC | MQGTH----------- ------------WLLT | FGGGT KVEIK |
| **iPS:437 254** | **21-225_149F2** | **VK2\|A17/ JK4** | | DVVMTQSPLSLPV TLGQPASISC | RSS-- QSLVYSD- GNTSLN | WFQQRPGQ SPRRLIY | K-------- VSNWDS | GVPVRFSGSGSG-- TDFTLKISRVEAEDVGIYYC | MQGTH----------- ------------WPPT | FGGGT KVEIK |
| **iPS:437 256** | **21-225_150F11** | **VK2\|A17/ JK4** | | DVVMSQYPLSLPV TFGQPASISC | RSS-- QSLVYSD- GNTSLN | WFQQRPGQ YPRRLIY | K-------- VSNWDY | GVPVRFSGSGSG-- TDFTLKISRVEAEDVGIYYC | MQGTH----------- ------------WPPT | FGGGT KVEIK |
| **iPS:437 268** | **21-225_177D2** | **VK2\|A17/ JK4** | | DVVMTQSPLSLPV TLGQPASISC | RSS-- QSLVYSD- GNTYLN | WFQQRPGQ SPRRLIY | K-------- VSNWDS | GVPDRFSGSGSG-- TDFTLKISRVEAEDVGVYYC | MQGTH----------- ------------WPLT | FGGGT KVEIK |
| **iPS:443 005** | **21-225_43F11_ LC1** | **VK2\|A17/ JK4** | | DVVMTQSPLSLPV TLGQPASISC | RSS-- QSLVYSD- GNTYLN | WFQQRPGQ SPRRLIY | K-------- VSNWDS | GVPDRFSGSGSG-- TDFTLKISRVEAEDVGVYYC | MQGTH----------- ------------WPLT | FGGGT KVEIK |
| **iPS:398 530** | **21-225_32G4** | **VK2\|A17/ JK4** | | DVVMTQSPLSLSV TLGQPASISC | RSS-- QSLVYSD- GNTYLN | WFQQRPGQ SPRRLIY | K-------- VSNWDS | GVPDRFSGSGSG-- TDFTLKISRVEAEDVGVYYC | MQGI------------ ------------HWLT | FGGGT KVEIK |
| | | **Germline** | | **K_FR1** | **K_CDR1** | **K_FR2** | **K_CDR2** | **K_FR3** | **K_CDR3** | **K_FR4** |
| | **VK2\|A17/JK 3** | | 32146 | DVVMTQSPLSLPV TLGQPASISC | RSS-- QSLVYSD- GNTYLN | WFQQRPGQ SPRRLIY | K-------- VSNRDS | GVPDRFSGSGSG-- TDFTLKISRVEAEDVGVYYC | MQGTH----------- ------------WPFT | FGPGT KVDIK |
| **iPS:468 856** | **21-225_77C9** | **VK2\|A17/ JK3** | | DVVMTQSPLSLPV TLGQPASISC | RSS-- QSLVYSV- GNTSLS | WFQQRPGQ SPRRLIY | K-------- VSNWDS | GVPDRFSGSGSG-- TDFTLKISRVEAEDVGVYYR | MQGTH----------- ------------WPFT | FGPGT KVDIK |
| **iPS:392 936** | **21-225_28B6** | **VK2\|A17/ JK3** | | DVVMTQSPLSLPV TLGQPASISC | RSS-- QSLVYSD- GDTYLN | WFQQRPGQ SPRRQIY | K-------- VSNWDS | GVPDRFSGSGSG-- TDFTLNISRVEAEDVGIYFC | MHCT------------ ------------HWLL | FGPGT KVDIK |
| | | **Germline** | | **K_FR1** | **K_CDR1** | **K_FR2** | **K_CDR2** | **K_FR3** | **K_CDR3** | **K_FR4** |
| | **VK2\|A17/JK 5** | | 32147 | DVVMTQSPLSLPV TLGQPASISC | RSS-- QSLVYSD- GNTYLN | WFQQRPGQ SPRRLIY | K-------- VSNRDS | GVPDRFSGSGSG-- TDFTLKISRVEAEDVGVYYC | MQGTH----------- ------------WPIT | FGQGT RLEIK |
| **iPS:472 741** | **21-225_30D9_ LC1** | **VK2\|A17/ JK5** | | DVVMTQSPLSLPV TLGQPASISC | RSS-- QSLVSSD- GNTFLN | WFQQRPGQ SPRRLIY | K-------- VSNWDS | GVPDRFSGSGSG-- TDFTLKISRLEAEDVGVYYC | LQGTH----------- ------------WPLT | FGQGT RLEIK |
| **iPS:437 294** | **21-225_216D5** | **VK2\|A17/ JK5** | | DVVMTQSPLSLPV TLGQPASISC | RSS-- QSLVYSD- GNTYLN | WFQQRPGQ SPRRLIY | K-------- VSNWDS | GVPDRISGSGSG-- TDFTLKISRVEAEDVGIYYC | MQGA------------ ------------HWFT | FGQGT RLEIK |
| **iPS:472 732** | **21-225_2B10_ LC1** | **VK2\|A17/ JK5** | | DVVMTQSPLSLPV TLGQPASISC | RSS-- QSLVYSD- GNTFLN | WFQQRPGQ SPRRLIY | K-------- VSNWDS | GVPDRFSGSGSG-- TGFTLKISRVEAEDVGVYYC | IQGTH----------- ------------WPFP | FGQGT RLEIK |
| **iPS:398 508** | **21-225_24B1** | **VK2\|A17/ JK5** | | DVVMTQSPLSLPV TLGQPASISC | RSS-- QSLVYSD- | WFQQRPGQ SPRRLIY | K-------- VSNWDS | GVPDRFSGSGSG-- TDFTLKISWVEAEDVGVCYC | MQGAH----------- -----------WPPIT | FGQGT RLEIK |

| | | | | | | | | | | |
|---|---|---|---|---|---|---|---|---|---|---|
| **iPS:423 019** | **21-225_31D12 _LC1** | **VK2\|A17/ JK5** | | DVVMTQSPLSLPV TLGQPASISC | RSS-- QSLIYSD- GNTFLN | WFQQRPGQ SPRRLIY | K-------- VSNWDS | GVPDRFSGSGSG-- TDFTLKISRLEAEDVGIYYC | MQGTH----------- ------------WPLT | FGQGT RLEIK |
| | | **Germline** | | **K_FR1** | **K_CDR1** | **K_FR2** | **K_CDR2** | **K_FR3** | **K_CDR3** | **K_FR4** |
| | **VK1\|L5/JK1** | | 32148 | DIQMTQSPSSVSA SVGDRVTITC | RAS--QGIS-- ----SWLA | WYQQKPGK APKLLIY | A-------- ASSLQS | GVPSRFSGSGSG-- TDFTLTISSLQPEDFATYYC | QQANS----------- ------------FPWT | FGQGT KVEIK |
| **iPS:433 897** | **21-225_43C2** | **VK1\|L5/J K1** | | DIQMTQSPSSVSA SVGDRVTITC | RAS--QGIS-- ----DWLA | WYQQKPGK APRLLIY | A-------- ASSLQS | GVPSRFSGSGSG-- TDFTLTISNLQPEDFATYYC | QQTNS----------- ------------FPWT | FGQGT KVEIK |
| **iPS:433 903** | **21-225_43H4** | **VK1\|L5/J K1** | | DIQMTQSPSSVSA SVGDRVTITC | RAS--QGII-- ----NWLA | WYQQKPGK APKLLIY | A-------- ASSLQS | GVPSRFSGSGSG-- TDFTLTISNLQPEDFATYYC | QQTNS----------- ------------FPWT | FGQGT KVEIK |
| **iPS:433 911** | **21-225_43E8** | **VK1\|L5/J K1** | | DIQMNQSPSSVSA SVGDRVTITC | RAS--QGIS-- ----NWLA | WYQQKPGR APKLLIY | A-------- ASSLQS | GVPSRFSGSGSG-- TDFTLTISNLQPEDFATYYC | QQTNS----------- ------------FPWT | FGQGT KVEIK |
| **iPS:433 941** | **21-225_44D10** | **VK1\|L5/J K1** | | DIQMTQSPSSVSA SVGDRVTITC | RAS--QGIS-- ----DWLA | WYQQKPGK APRLLIY | A-------- ASSLQS | GVPSKFSGSGSG-- TDFTLTISNLQPEDFATYYC | QQTNS----------- ------------FPWT | FGQGT KVEIK |
| **iPS:433 945** | **21-225_44C12** | **VK1\|L5/J K1** | | DIQMTQSPSSVSA SVGDRVTITC | RAS--QGIS-- ----NWLA | WYQQKPGK APKLLIS | A-------- AFSLQS | GVPSRFSGSGSG-- TDFTLSISSLQPEDFATYYC | QQSNS----------- ------------FPWT | FGQGT KVEIK |
| **iPS:433 957** | **21-225_45F8** | **VK1\|L5/J K1** | | DIQMTQSPSSVSA SVGDRVTITC | RAS--QGIS-- ----DWLA | WYQQKPGK APRLLIY | G-------- ASSLQS | GVPSRFSGSGSG-- TDFTLTISNLQPEDFATYYC | QQTNS----------- ------------FPWT | FGQGT KVEIK |
| **iPS:433 973** | **21-225_46A6** | **VK1\|L5/J K1** | | DIQMNQSPSSVSA SVGDRVNITC | RAS--QGIS-- ----NWLA | WYQQKPGK VPKLLIY | A-------- ASSLQS | GVPSRFSGSGSG-- TDFTLTISNLQPEDFATYYC | QQTNS----------- ------------FPWT | FGQGT KVEIK |
| **iPS:433 993** | **21-225_47G7** | **VK1\|L5/J K1** | | DIQMTQSPSSVSA SVGDRVTITC | RAS--QGIS-- ----NWLA | WYQQKPGK APKLLIF | A-------- ASNLQS | GVPSRFSGSGSG-- TDFTLTISSLQPADFATYCC | QQVNS----------- ------------FPWT | FGQGT KVEIK |
| **iPS:434 007** | **21-225_48D7** | **VK1\|L5/J K1** | | DIQMTQSPSSVSA SVGDRVTITC | RAS--QNIT-- ----SWLA | WYQQKPGK APKLLIY | S-------- ASSLQN | GVPSRFSGSGSG-- TDFTLTISSLQPEDFVTYCC | QQANS----------- ------------FPWT | FGQGT KVEIK |
| **iPS:434 063** | **21-225_51G7** | **VK1\|L5/J K1** | | DIQMTQSPSSVSA SVGDRVTITC | RAS--QDIS-- ----SWLA | WYQQKPGK APKVLIY | A-------- PSNLQS | AVPSRFSGSGSG-- TDFTLTISSLQPEDFATYYC | QQAHS----------- ------------FPWT | FGQGT KVEIK |
| **iPS:434 083** | **21-225_52H2** | **VK1\|L5/J K1** | | DIQMTQSPSSVSA SVGDRVTITC | RAS--QNIT-- ----NWLA | WFQQKPGR APKLLIY | T-------- TSSLQS | GVPSRFSGSGSG-- TDFTLTISSLQPEDFATYCC | QQTNS----------- ------------FPWT | FGHGT KVEVK |
| **iPS:434 133** | **21-225_54G3** | **VK1\|L5/J K1** | | DIQMTQSPSSVSA SVGDRVTITC | RAS--QGIS-- ----SWLA | WYQQKPGK APKLLIY | D-------- ASSLQS | GVPSRFSGSGSE-- TDFTLTISSLQPEDFATYYC | QQANS----------- ------------FPWT | FGQGT KVEIK |
| **iPS:434 221** | **21-225_60A11** | **VK1\|L5/J K1** | | DIQMTQSPSSVSA SVGDRVTITC | RAS--QVIS-- ----NWLA | WYQLKPGK APKLLIY | T-------- ASSLQS | GVPSRFSGNESG-- TDFTLTISSLQPEDFATYYC | QQANS----------- ------------FPWT | FGQGT KVEIK |
| **iPS:434 283** | **21-225_57F8** | **VK1\|L5/J K1** | | DIQMTQSPSSVSA SVGDRVTITC | RAS--QGIS-- ----NWLA | WYQQKPGK APKLLIY | T-------- ASSLQS | GVPSRFSGNESG-- TDFTLTISSLQPEDFATYYC | QQANS----------- ------------FPWT | FGQGT KVEIK |
| **iPS:435 711** | **21-225_171G4** | **VK1\|L5/J K1** | | DIQMTQSPSSVSA SVGDRVTITC | RAS--QGVN-- ----DWLA | WYQQKPGR APKLLIY | D-------- ASSLQS | GVPSRFSGSGSG-- TDFTLTISSLQPEDFATYYC | QQANS----------- ------------FPWT | FGQGT KVEIK |
| **iPS:435 715** | **21-225_171A8** | **VK1\|L5/J K1** | | AIQMTQSPSSVSA SVGDRVTITC | RAS--QGIS-- ----NWLA | WYQQKPGK APNLMIH | A-------- AFSLQG | GVPSRFSGSGSG-- TDFTLTISSLQPEDFATYYC | QQTNS----------- ------------FPWT | FGQGT KVEIK |
| **iPS:435 717** | **21-225_171A9** | **VK1\|L5/J K1** | | DIQMTQSPSSVSA SVGDRVTITC | RAS--QDIT-- ----TWLA | WYQQKPGK APKLLIY | D-------- ASSLQS | AVPSRFSGSGSG-- TDFTLTVSSLQPEDFATYYC | LQTNS----------- ------------FPWT | FGQGT KVEIK |
| **iPS:435 739** | **21-225_174G7** | **VK1\|L5/J K1** | | AIQMTQSPSSVSA SVGDRVTITC | RAS--QGIS-- ----NWLA | WYQQKPGK APNLLIH | A-------- AFSLQG | GVPSRFSGSGSG-- TDFTLTISSLQPEDFATYYC | QQTNS----------- ------------FPWT | FGQGT KVEIK |
| **iPS:435 749** | **21-225_175C1 0** | **VK1\|L5/J K1** | | DIQMTQSPSSVSA SVGDRVTITC | RAS--QGIT-- ----DWLA | WYQQKPGK APKLLIY | A-------- ASSLQS | GVPSRFSGSGSG-- TDFTLTFSSLQPDDFATYYC | QQTNS----------- ------------FPWT | FGQGT KVEIK |
| **iPS:435** | **21-** | **VK1\|L5/J** | | DIQMTQSPSSVSA | RAS--QGIS-- | WYQQKPGK | A-------- | GVPSRFNGSGSG-- | QQANS----------- | FGQGT |

| | | | | | | | | | | |
|---|---|---|---|---|---|---|---|---|---|---|
| iPS:435 777 | 21-225_178F7 | VK1\|L5/JK1 | | DIQMTQSPSSVSA SVGDRVTITC | RAS--QDIT-- ----DWLA | WYQQKPGK APKLLIS | A-------- ASSLQS | GVPSRFSGSGSG-- TDFTLAISSLQPEDFATYYC | QQANS----------- ------------LPWT | FGQGT KVEIK |
| iPS:435 783 | 21-225_179G1 | VK1\|L5/JK1 | | DIQMTQSPSSVSA SVGDRVTITC | RAS--QDIS-- ----DWLA | WYQQKSGK APKLLIS | A-------- ASSLQS | GVPSRFGGSGSG-- TDFTLTISSLQPEDFATYYC | QQANS----------- ------------LPWT | FGQGT KVEIK |
| iPS:435 875 | 21-225_190B9 | VK1\|L5/JK1 | | DIQMTQSPSSVSA SVGDRVTITC | RAS--QGIN-- ----NWLA | WYQQKPGK APKLLIY | G-------- VSSLQS | GVPSRFSGSGSG-- TDFTLTISSLQPEDFATYSC | QQANS----------- ------------FPWT | FGQGT KVEIR |
| iPS:435 895 | 21-225_188E8 | VK1\|L5/JK1 | | DIQMTQSPSSVSA SVGDRVTITC | RAN--QDIS-- ----SWLA | WYQQKPGK APKLLIY | A-------- ASNLQS | GVPSGFSGSGSG-- TDFTLTISSLQPEDFATYYC | QQANS----------- ------------FPWT | FGQGT KVEIK |
| iPS:435 909 | 21-225_190H3 | VK1\|L5/JK1 | | DIQMTQSPSSVSA SVGDRVTITC | RAS--QGLN-- ----NWLA | WYQLKPGK APKLLIY | A-------- VSSLQS | GVPSRFSGSGSG-- SEFTLTISSLQPEDFATYHC | QQANS----------- ------------LPWT | FGQGT KVEIK |
| iPS:436 013 | 21-225_193F2 | VK1\|L5/JK1 | | DIQMTQSPSSVSA SVGDRVTITC | RAS--QGIN-- ----NWLA | WYQQKPGK APKLLIY | G-------- VSSLQS | GVPSRFSGSGSG-- TDFTLTISSLQPEDFATYSC | QQANS----------- ------------FPWT | FGQGT KVEIR |
| iPS:436 068 | 21-225_194F7 | VK1\|L5/JK1 | | DIQMTQSPSSVSA SVGDRVTITC | RAS--QGIS-- ----RWLA | WFQQKPGK APKILIY | A-------- ASSLQS | GVPSRFSGSGSG-- TDFTLTISSLQPEDFATYYC | QQANS----------- ------------FPWT | FGQGT KVEIK |
| iPS:436 100 | 21-225_195G12 | VK1\|L5/JK1 | | DIQMTQSPSSVSA SVGDRVTITC | RAS--QGIN-- ----NWLA | WYQQKPGK APKLLIY | G-------- VSSLQS | GVPSRFSGSGSG-- TDFTLTISSLQPEDFATYSC | QQANS----------- ------------FPWT | FGRGT KVENQ |
| iPS:436 116 | 21-225_196B9 | VK1\|L5/JK1 | | DIQMTQSPSSVSA SVGDRVTITC | RAS--QGIS-- ----NCLA | WYQQKPGK APKFLIC | A-------- ASSLQS | AVPSRFSGSGSG-- TDFTLTISSLQPEDFASYYC | QQGDS----------- ------------FPPT | FGQGT KVEFR |
| iPS:436 160 | 21-225_197C9 | VK1\|L5/JK1 | | DIQMTQSPSSVSA SVGDRVTITC | RAS--QGIS-- ----NWLA | WFQQKPGK APQLLIY | A-------- ASSLQS | GVPSRFSGSGSG-- TDFSLTISSLQPEDFATYYC | QQANS----------- ------------FPWT | FGQGT KVEIK |
| iPS:436 546 | 21-225_224D6 | VK1\|L5/JK1 | | DIQMTQSPSSVSA SVGDRVTITC | RAS--QGIS-- ----SWLA | WYQQKPGK APKLLIY | A-------- ASSLQS | GVPSRFSGSGSG-- TDFTLTISSLQPEDFATYYC | QQANS----------- ------------FPWT | FGPGT KVEIK |
| iPS:436 632 | 21-225_227E4 | VK1\|L5/JK1 | | DILMTQSPSSVSA SVGDRVTITC | RAS--QGII-- ----NWLA | WYQQKPGK APKLLIY | A-------- ASSLQS | GVPSRFSGSGSG-- TDFTLTISSLQPEDFAIYYC | QQANS----------- ------------FPWT | FGQGT KVEIK |
| iPS:392 650 | 21-225_17A4 | VK1\|L5/JK1 | | DIQMTQSPSSVSA SVGDRVTITC | RAS--QGIG-- ----NWLA | WYQQKPGK APKLLIF | A-------- ASSLQS | GVPSRFSGSGSG-- TDFTLTISSLQPEDFVTYYC | QQANS----------- ------------FPRT | FGQGT KVEIK |
| iPS:392 728 | 21-225_20F7 | VK1\|L5/JK1 | | DIQMTQSPSSVSA SVGDGVTITC | RAS--QGIS-- ----SWLA | WYQQKPGK APKLLIY | A-------- ASSLQS | GVPSRFSGSGSG-- TDFTLTISSLQPEDFATYYC | QQANS----------- ------------FPRT | FGQGT KVEIK |
| iPS:392 916 | 21-225_27C5 | VK1\|L5/JK1 | | DIQMTQSPSSVSA SVGDRVTITC | RAS--QGIS-- ----SWLA | WYQQKPGK APKFLIY | G-------- ASSLQS | GVPSRFSASGSG-- TEFTLTISSLQPEDFATYCC | QQYDS----------- ------------FPRT | FGQGT KVEIK |
| iPS:392 956 | 21-225_27A11 | VK1\|L5/JK1 | | DIQMTQSPSSVSA SVGDRVTITC | RAS--QGIS-- ----SWLA | WYQQKPGK APKLLIY | G-------- ASSLQS | GVPSRFSASGSG-- TDFTLTISSLQPEDFATYCC | QQSDS----------- ------------FPRT | FGQGT KVEIK |
| iPS:393 014 | 21-225_26D12 | VK1\|L5/JK1 | | DIQMTQSPSSVSA SVGDRVTITC | RAS--QGIS-- ----SWLA | WYQQKPGK APKLLIY | G-------- ASSLQS | GVPSRFSASGSG-- TDFTLTISSLQPEDFATYCC | QQSDS----------- ------------FPRT | FGQGT KVEIK |
| iPS:393 028 | 21-225_25D7 | VK1\|L5/JK1 | | DIQMTQSPSSVSA SVGDRVTFTC | RAS--QDIF-- ----DWLA | WYQQKPGT APKLLIY | A-------- ASSLQS | GVPSRFSGSGSG-- TNFTLTVSGLQPEDFATYYC | QQAYS----------- ------------FPWT | FGQGT KVEIK |
| iPS:393 152 | 21-225_25B3 | VK1\|L5/JK1 | | DIQMTQSPSSVSA SVGDRVTITC | RAS--QGIS-- ----SWLA | WYQQKPGK APKLLIY | G-------- ASSLQS | GVPSRFSASGSG-- TDFTLTISSLQPEDFATYCC | QQSDS----------- ------------FPRT | FGQGT KVEIK |
| iPS:393 810 | 21-225_5A4 | VK1\|L5/JK1 | | DIQMTQSPSSVSA SVGDRVTITC | RAS--QGIS-- ----TWLA | WYQQKPGK APKLLIY | D-------- ASSLQS | GVPSRFSGSGSG-- TDFTLTISSLQPEDFATYYC | QQANS----------- ------------FPWT | FGQGT KVEIK |
| | | Germline | | K_FR1 | K_CDR1 | K_FR2 | K_CDR2 | K_FR3 | K_CDR3 | K_FR4 |
| | VK2\|A19/JK3 | | 32149 | DIVMTQSPLSLPV TPGEPASISC | RSS-- QSLLHSN-GYNYLD | WYLQKPGQ SPQLLIY | L-------- GSNRAS | GVPDRFSGSGSG-- TDFTLKISRVEAEDVGVYYC | MQALQ----------- ------------IPFT | FGPGT KVDIK |

| | | | | | | | | | | |
|---|---|---|---|---|---|---|---|---|---|---|
| **iPS:433 943** | **21-225_44E10** | **VK2\|A19/ JK3** | | DIVMTQSPLSLPV<br>TPGEPASISC | RSS--<br>QSLLHSN-<br>GYSYLE | WYLQKPGQ<br>SPQLLIY | L--------<br>GSNRAS | GVPDRFSGSGSG--<br>TDFTLKISRVEAEDVGVYYC | MQTLQ-----------<br>------------TPFT | FGPGT<br>KVDIK |
| **iPS:433 989** | **21-225_47C7** | **VK2\|A19/ JK3** | | DIVMTQSPLSPPV<br>TPGEPASISC | RSS--<br>QSLLHSN-<br>GYNYLE | WYLQKSGQ<br>SPQFLIY | L--------<br>GFNRAS | GVPDRFTGSGSG--<br>TDFTLKISRVEAEDVGVYYC | MQVLQ-----------<br>------------TPFT | FGPGT<br>KVDIK |
| | | **Germline** | | **K_FR1** | **K_CDR1** | **K_FR2** | **K_CDR2** | **K_FR3** | **K_CDR3** | **K_FR4** |
| | **VK1\|O12/JK3** | | 32150 | DIQMTQSPSSLSA<br>SVGDRVTITC | RAS--QSIS--<br>----SYLN | WYQQKPGK<br>APKLLIY | A--------<br>ASSLQS | GVPSRFSGSGSG--<br>TDFTLTISSLQPEDFATYYC | QQSYS-----------<br>------------TPFT | FGPGT<br>KVDIK |
| **iPS:433 999** | **21-225_48D1** | **VK1\|O12/ JK3** | | DIQMTQSPSSLSA<br>SVGDRVNITC | RAS--QSIS--<br>----SYLI | WYQQKPGK<br>APKLLIY | A--------<br>ASSLQS | GVPSRFSASGSG--<br>TDFTLTISSLQPEDFASYYC | QQSNS-----------<br>------------IPFT | FGPGT<br>KVDIK |
| **iPS:434 003** | **21-225_48C3** | **VK1\|O12/ JK3** | | DIQMTQSPSSLSA<br>SVGDRVNITC | RAS--QSII--<br>----SYLI | WYQQKPGK<br>APRLLIY | A--------<br>ASSLQS | GVPSRFSASGSG--<br>TDFTLTISSLQPEDFASYYC | QQTNS-----------<br>------------IPFT | FGPGT<br>KVDIK |
| **iPS:434 037** | **21-225_49G12** | **VK1\|O12/ JK3** | | DIQMTQSPSSLSA<br>SVGDRVTITC | RSS--QSIS--<br>----TYLM | WYQQKPGK<br>APKLLIY | A--------<br>ASSLQI | GVPSEFSASGSG--<br>TDFTLTISSLQPEDFATYYC | QQSYS-----------<br>------------IPFT | FGPGT<br>KVDIK |
| **iPS:434 041** | **21-225_50H8** | **VK1\|O12/ JK3** | | DIQMTQSPSSLSA<br>SVGDRVNITC | RAS--QSIS--<br>----SYLI | WYQQKPGK<br>APKLLIY | A--------<br>ASSLQS | GVPSRFSASGSG--<br>TDFTLTISSLQPEDFASYYC | QQSNS-----------<br>------------LPFT | FGPGT<br>KVDIK |
| **iPS:434 045** | **21-225_50H10** | **VK1\|O12/ JK3** | | DIQMTQSPSSLSA<br>SVGDRVNITC | RAS--QSIY--<br>----SYLI | WYQQKPGK<br>APKLLIY | A--------<br>ASSLQS | GVPSRFSASGSG--<br>TDFTLTISSLQPEDFASYYC | QQSNS-----------<br>------------IPFT | FGPGT<br>KVDIK |
| **iPS:434 049** | **21-225_50B12** | **VK1\|O12/ JK3** | | DIQMTQSPSSLSA<br>SVGDRVTITC | RAS--QSIS--<br>----SYLN | WYQHKPGK<br>APRLLIY | A--------<br>ASSLQS | GVPSRFSGSESG--<br>TDFILTISSLQPEDFTTYYC | QQSYI-----------<br>------------APFT | FGPGT<br>KVDIK |
| **iPS:434 073** | **21-225_51H10** | **VK1\|O12/ JK3** | | DIQMTQSPSSLSA<br>SVGDRVTITC | RAS--QSIS--<br>----TYLM | WYQQKPGK<br>APKLLIY | A--------<br>ASSLQS | GVPSEFSASGSG--<br>TDFTLTISSLQPEDFATYYC | QQSYS-----------<br>------------IPFT | FGPGT<br>KVDIK |
| **iPS:434 107** | **21-225_53E2** | **VK1\|O12/ JK3** | | DIQMTQSPSSLSA<br>SVGDRVTITC | RAS--QSFS--<br>----HYLN | WYQQKPGK<br>APNLLIF | A--------<br>VSSLQS | GVPSRFSGSGSG--<br>SDFTLPISSLQPEDFAIYFC | QQSFS-----------<br>------------TPFT | FGPGT<br>KVDIK |
| **iPS:434 181** | **21-225_56B2** | **VK1\|O12/ JK3** | | DIQMTQSPSSLSA<br>SVGDRVTITC | RAS--QSFS--<br>----HYLN | WYQQKPGK<br>APNLLIF | A--------<br>VSSLQS | GVPSRFSGSGSG--<br>TDFTLTISSLQPEDFAIYFC | QQSYS-----------<br>------------TPFT | FGPGT<br>KVDIK |
| **iPS:434 225** | **21-225_60E12** | **VK1\|O12/ JK3** | | DIQMTQSPSSLSA<br>SVGDRVTITC | RAS--QSIS--<br>----SYLN | WYQQKPGK<br>APKLLIY | A--------<br>ASSLQS | GVPSRFSGSGSG--<br>TDFTLTISSLQPEDFAPYYC | QQSYN-----------<br>------------ISFT | FGPGT<br>KVDIK |
| **iPS:434 227** | **21-225_61A1** | **VK1\|O12/ JK3** | | DIQMTQSPSSLSA<br>SVGDRVTITC | RAS--QSIS--<br>----SYLN | WYQQKPGK<br>APKLLIY | A--------<br>ASSLQS | GVPSRFSGSGSG--<br>TDFTLTISSLQPEDFAPYYC | QQSYN-----------<br>------------ISFT | FGPGT<br>KVDIK |
| **iPS:434 245** | **21-225_62H1** | **VK1\|O12/ JK3** | | DIQMTQSPSSLSA<br>YVGDRVTITC | RAS--QNIF--<br>----SYLN | WYQQKPGK<br>APKVLIY | A--------<br>VFSLQS | GVPSRFSGSGSG--<br>TDFTLTISSLQPEDFATYYC | QQSYS-----------<br>------------TPFT | FGPGT<br>KVDIK |
| **iPS:434 267** | **21-225_57F2** | **VK1\|O12/ JK3** | | DIQMTQSPSSLSA<br>SVGDRVTITC | RAS--QSIS--<br>----SYLN | WYQQKPGK<br>APKLLIY | A--------<br>ASSLQS | GVPSRFSGSGSG--<br>TDFTLTISSLQPEDFAPYYC | QQSYN-----------<br>------------ISFT | FGPGT<br>KVDIK |
| **iPS:434 323** | **21-225_62H8** | **VK1\|O12/ JK3** | | DIQMTQSPSSLSA<br>SVGDRVTITC | RAS--QSIF--<br>----SYLN | WYQQNPGK<br>APKLLIY | A--------<br>SSSLQS | GVPSRLSGNGSG--<br>TDFILTISSLQPEDFATYYC | QQSYS-----------<br>------------TPFT | FGPGT<br>RVDIK |
| **iPS:434 379** | **21-225_66A9** | **VK1\|O12/ JK3** | | DIQMTQSPSSLSA<br>SVGDRVTITC | RAS--QNIF--<br>----SYLN | WYQQKPGK<br>APKLLIF | A--------<br>ASSLQS | GVPSRFSGSGSG--<br>TDFTLTISSLQPEDFATYYC | QQTYS-----------<br>------------VPFT | FGPGT<br>KVDFK |
| **iPS:434 417** | **21-225_69C8** | **VK1\|O12/ JK3** | | DIQMTQSPSSLSA<br>SVGDRVTFTC | RAG--QTIY--<br>----NYLN | WYQQKPGK<br>APKLLIH | V--------<br>ASSLQS | GVPSRFSGSGSG--<br>TDFTLVISSLQPEDFATYYC | QQSYS-----------<br>------------TPFT | FGPGT<br>KVDIK |
| **iPS:435 545** | **21-225_158F4** | **VK1\|O12/ JK3** | | DIQMTQSPSSLPA<br>SVGDRVTITC | RAS--QNIR--<br>----KYLH | WYQFLPGK<br>APKLLIY | T--------<br>ASTLQS | GVPSRFSGSGSG--<br>TDFTLTISSLQPEDFATYYC | QQSYN-----------<br>------------ISFT | FGPGT<br>KVDIK |
| **iPS:435 793** | **21-225_180F8** | **VK1\|O12/ JK3** | | DIQMTQSPSSLSA<br>SVGDGVTITC | RAS--QTIL--<br>----SYLN | WYQQKPGK<br>APKLLIY | G--------<br>VSSLQS | GVPSRFSGSGSG--<br>TDFSLTISSLQPEDFATYYC | QQSYS-----------<br>------------TPFT | FGPGT<br>KVDIK |
| **iPS:436** | **21-** | **VK1\|O12/** | | DIQMTHSPSSLSA | RAS--QNIS-- | WYQQKPGK | T-------- | GVSSRFSGSGSG-- | QQYYF----------- | FGRGT |

| | | | | | | | | | | |
|---|---|---|---|---|---|---|---|---|---|---|
| iPS:436<br>510 | 21-<br>225_222H8 | VK1\|O12/<br>JK3 | | DIQMTHSPSSLSA<br>SVGDRVTITC | RAS--QNIS--<br>----NYVN | WYQQKPGK<br>APKFLIY | I--------<br>ASSLQS | GVSSRFSGSGSG--<br>TDFTLTISSVHRDDFAIYYC | QQYYF-----------<br>------------TPFT | FGRGT<br>KVDIK |
| iPS:437<br>230 | 21-<br>225_62H10 | VK1\|O12/<br>JK3 | | DIQMTQSPSSLSA<br>SVGDRVTITC | RAS--QSIT--<br>----SYLN | WYQQKPGK<br>VPKLLIS | T--------<br>ASSLQS | GVPSRFSGSGSG--<br>TDFTLTISSLQPEDFATYYC | QQSHS-----------<br>------------FPFT | FGPGT<br>NVDFK |
| iPS:448<br>906 | 21-<br>225_72G9 | VK1\|O12/<br>JK3 | | DIQMTQSPSSLSA<br>SVGDRVTITC | RAS--QSIT--<br>----SYLN | WYQQKPGK<br>APKLLIY | T--------<br>ASSLQS | GVPSRFSGSGSG--<br>TDFTLTISSLQPEDFATYYC | QQSHS-----------<br>------------FPFT | FGPGT<br>KVDIK |
| iPS:392<br>652 | 21-<br>225_17C6 | VK1\|O12/<br>JK3 | | DIQMTQSPSSLSA<br>SVGDRVTITC | RAS--QSIN--<br>----TYLN | WYQQKPGK<br>APKLLIY | A--------<br>ASSLQS | GVPSRFSGSGSG--<br>TDFTLTISSLQPEDFATYFC | QQSYR-----------<br>-----------TPFFT | FGPGT<br>KVDIK |
| iPS:392<br>660 | 21-<br>225_19B3 | VK1\|O12/<br>JK3 | | DIQMTQSPSSLSA<br>SVGDRITITC | RAG--QNII--<br>----NYLN | WYQQKPGK<br>APNLLIY | V--------<br>ASSLQS | GVPSRFNGSGSG--<br>TDFTLTISSLQPEDFATYYC | QQSYS-----------<br>------------TPFT | FGPGT<br>KVDIK |
| iPS:392<br>668 | 21-<br>225_17B4 | VK1\|O12/<br>JK3 | | DIQMTQSPSSLSA<br>SVGDRVTITC | RAS--QNIS--<br>----SYLN | WYQQKPGK<br>APKLLIF | G--------<br>ASSLQT | GVPSRFSGSGSG--<br>TDFTLTINSLQPEDFATYFC | QQSYR-----------<br>-----------TPFFT | FGPGT<br>KVDIK |
| iPS:392<br>678 | 21-<br>225_20F3 | VK1\|O12/<br>JK3 | | DIQMTQSPSSLSA<br>SVGDRVTITC | RAS--QSIS--<br>----SYLY | WYQQKPGK<br>APKLLIY | A--------<br>ASSLQS | GVPSRFSGSGSG--<br>TDFTLTISSLQPEDFATYYC | QQSYS-----------<br>-----------APPFT | FGPGT<br>KVDIK |
| iPS:392<br>694 | 21-<br>225_19A5 | VK1\|O12/<br>JK3 | | DIQMTQSPSSLSA<br>PVGDRVSITC | RAS--QNII--<br>----NYLN | WYQQKPGK<br>APKLLID | V--------<br>ASNLQG | GVPSRFSGSGSG--<br>TDFTLTISSLQPEDFATYYC | QQSYS-----------<br>------------TPFT | FGPGT<br>KVDIK |
| iPS:392<br>696 | 21-<br>225_20A4 | VK1\|O12/<br>JK3 | | DIQMTQSPASLSA<br>SVGDRVTITC | RAS--QSII--<br>----NYLN | WYQQRPGK<br>SPKLLIY | A--------<br>ASSLHS | GVPSRFSGRGSG--<br>TDFTLTISSLQPEDFATYFC | QQSYR-----------<br>-----------TPLFT | FGPGT<br>KVDFK |
| iPS:392<br>702 | 21-<br>225_17F7 | VK1\|O12/<br>JK3 | | DIQMTQSPSSLSA<br>SVGDRVTITC | RAS--QTIS--<br>----SFLN | WYQQKPGK<br>APKLLIY | A--------<br>ASSLQS | GVPSRFSGSGSG--<br>TDFTLTISGLQPEDFATYFC | QQSYR-----------<br>-----------TPFFT | FGPGT<br>KVDIK |
| iPS:392<br>704 | 21-<br>225_17F11 | VK1\|O12/<br>JK3 | | DIQMTQSPSSLSA<br>SIGDRVSITC | RAS--RTIN--<br>----NYLN | WYQQKPGK<br>APKLLIF | A--------<br>TSSLQS | GVPSRFSGSGSG--<br>TDFTLTISSLQPEDFATYYC | QQTYS-----------<br>-----------TPLFA | FGPGT<br>KVDIK |
| iPS:392<br>720 | 21-<br>225_17A12 | VK1\|O12/<br>JK3 | | DIQMTQSPSSLSA<br>SVGDRVTITC | RAS--QSIS--<br>----SYLN | WYHQKPGK<br>APKLLIY | A--------<br>ASSLQS | GVPSRFSGSGSG--<br>TDFTLTISNLQPEDFATYYC | QQSYN-----------<br>-----------TPLFT | FGPGT<br>KVDIK |
| iPS:392<br>722 | 21-<br>225_18E12 | VK1\|O12/<br>JK3 | | DIQMTQSPSSLSA<br>SVGDRVTITC | RAS--QSIS--<br>----SYLN | WYQQKPGK<br>APTLLIY | A--------<br>ASSLQS | GVPSRFSGSGSG--<br>TDFTLTINSLQPEDFATYFC | QQSYR-----------<br>-----------TPFFT | FGPGT<br>KVDIK |
| iPS:392<br>760 | 21-<br>225_22G3 | VK1\|O12/<br>JK3 | | DIQMTQSPSSLSA<br>SVGDRVTITC | RAS--QSIS--<br>----NYLN | WYQQKPGK<br>APKLLIF | A--------<br>ASSLQS | GVPSRFSGSGSG--<br>TDFTLTISGLQPEDFATYFC | QQSFR-----------<br>-----------TPFFT | FGPGT<br>KVDIK |
| iPS:392<br>762 | 21-<br>225_22G5 | VK1\|O12/<br>JK3 | | DIQMTQSPSSLSA<br>SVGDRVTITC | RAS--QNIS--<br>----SYLN | WYQQKQGK<br>APKLLIY | A--------<br>ASSLQN | GVPSRFSGRGSG--<br>TDFTLTISSLQPEDFATYYC | QQSYR-----------<br>-----------TPLFT | FGPGT<br>KVDFK |
| iPS:392<br>764 | 21-<br>225_22G10 | VK1\|O12/<br>JK3 | | DIQMTQSPSSLSA<br>SVGDRVTITC | RAS--QSIF--<br>----SYLN | WYQQKPGK<br>APKLLIY | A--------<br>ASSLQS | GVPSRFSGSGSG--<br>TDFTLTISSLQPEDFATYFC | QQSFR-----------<br>-----------TPLFT | FGPGT<br>KVDFK |
| iPS:392<br>812 | 21-<br>225_21F4 | VK1\|O12/<br>JK3 | | DIQMTQSPSSLSA<br>SVGDRVTITC | RAS--QNIG--<br>----SYLN | WYQQKPGK<br>APKLLIF | A--------<br>ASSLQS | GVPSRFSGSGSG--<br>TDFTLTISSLQPEDFATFFC | QQSYR-----------<br>-----------TPFFT | FGPGT<br>KVDFK |
| iPS:392<br>816 | 21-<br>225_22E4 | VK1\|O12/<br>JK3 | | DIQMTQSPSSLSA<br>SVGDRVTITC | RAS--QNIS--<br>----SYLN | WYQQKPGK<br>APKLLIY | A--------<br>ASSLQS | GVPSRFSGSGSG--<br>TDFTLTISSLQPEDFATYYC | QQSYN-----------<br>-----------TPLFT | FGPGT<br>KVDIK |
| iPS:392<br>830 | 21-<br>225_21A5 | VK1\|O12/<br>JK3 | | DIQMTQSPSSLCA<br>SVGDRVTITC | RAS--QTIS--<br>----SHLN | WYQRKPGK<br>APKLLIY | A--------<br>ASSLQS | GVPSRFSGSGSG--<br>TDFTLTISSVQPEDFATYYC | QQSYN-----------<br>------------ISFT | FGPGT<br>KVDIK |
| iPS:392<br>852 | 21-<br>225_21A2 | VK1\|O12/<br>JK3 | | DIQMTQSPSSLSA<br>SVGDRVTITC | RAS--QSIS--<br>----SYLN | WYQQKPGK<br>APKLLIY | A--------<br>ASSLQS | GVPSRFSGSGSG--<br>TDFTLTISSLQPEDFATYFC | QQSYR-----------<br>-----------TPFFT | FGPGT<br>KVDIK |
| iPS:392<br>878 | 21-<br>225_22C5 | VK1\|O12/<br>JK3 | | DIQMTQSPASLSA<br>SVGDRVTITC | RAS--QNIS--<br>----SYLN | WYQQKPGK<br>APKLLIY | A--------<br>ASVLQH | GIPSRFSGRGSG--<br>TDFTLIISSLQPEDFATYYC | QQSYR-----------<br>-----------TPLFT | FGPGT<br>KVDFK |
| iPS:392<br>902 | 21-<br>225_22D5 | VK1\|O12/<br>JK3 | | DIQMTQSPSSLSA<br>SVGDRVTITC | RAS--QNIF--<br>----SYLN | WYHQKPGK<br>APKLLIY | A--------<br>ASSLQS | GVPSRFSGSGSG--<br>TDFTLTISSLQPEDFATYYC | QQSYS-----------<br>-----------TPLFT | FGPGT<br>KVDIK |
| iPS:392<br>984 | 21-<br>225_30F11 | VK1\|O12/<br>JK3 | | DIQMTQSPSSLSA<br>SVGDRVTITC | RAS--QSIS--<br>----NYLN | WYQQQTGK<br>APKFLIY | A--------<br>ASSLQS | GVPSRFSGSGSG--<br>TDFTLTISSLQPEDFATYYC | QQSYS-----------<br>------------TPFT | FGPGT<br>KVDIK |

| | | | | | | | | | | |
|---|---|---|---|---|---|---|---|---|---|---|
| iPS:393 114 | 21-225_33G12 | VK1\|O12/JK3 | | DIQMTQSPSSLSA SVGDRVTITC | RAS--QSIS-- ----NYLN | WYQQQTGK APKFLIY | A-------- ASSLQS | GVPSRFSGSGSG-- TDFTLTISSLQPEDFATYYC | QQSYS----------- ------------TPFT | FGPGT KVDIK |
| iPS:393 824 | 21-225_10F12 | VK1\|O12/JK3 | | DIQMTQSPSSLSA SVGDRVTITC | RAS--QNIS-- ----SYLN | WYQQKPGK APKLLIY | A-------- ASSLQS | GVPSRFSGSGSG-- TDFTLTISSLQPEDFATYYC | QQSYN----------- -----------TPFFT | FGPGT KVDIK |
| iPS:393 848 | 21-225_4H2 | VK1\|O12/JK3 | | DIQMTLSPSSLSA SVGDRVTITC | RAI--QNIS-- ----SYLN | WYQQKPGK APKLVIY | A-------- ASSLQS | GVPSRFSGRGSG-- TDFTLTIGCVQREDFATYYC | QQSYR----------- -----------TPLFT | FGPGT KVDIK |
| iPS:393 862 | 21-225_5G2 | VK1\|O12/JK3 | | DIQMTQSPSSPSA SVGDRVTITF | RAS--QNII-- ----SYLN | WYQQKPGK ARKLVIY | G-------- ASSLQS | GVPSRFSGSGSG-- TDFTLNIRSLQPEDFATYYC | QQSYS----------- -----------TPLFT | FGPGT KVDIK |
| iPS:393 888 | 21-225_3E3 | VK1\|O12/JK3 | | DIQMTQSPSSPSA SVGDRVTITF | RAS--QSIR-- ----SYLN | WYQQKPGK AHKLVIY | G-------- TSSLQS | GVPSRFSGSGSG-- TDFTLTISSLQPEDFATYYC | QQSYS----------- -----------TPLFT | FGPGT KVDIK |
| iPS:393 890 | 21-225_4B1 | VK1\|O12/JK3 | | DIQMTQSPSSLSA SVGDRVTITC | RAS--HTIR-- ----TYLN | WYQQKPGK APKLLIY | A-------- ASSLQS | GVPSRINGSGSG-- TDFTLTITNLQPEDFATYYC | QQSYN----------- ------------ISFT | FGPGT KVDIK |
| iPS:393 898 | 21-225_5F7 | VK1\|O12/JK3 | | DIQMTQSPSSPSA SVGDRVTITF | RAS--QTIS-- ----SYLN | WYQQKPGK APKLLIS | A-------- ASSLQS | GVPSRFSGSGSG-- TDFTLTISSLQPEDFATYYC | QQSYN----------- -----------TPLFT | FGPGT KVVIK |
| iPS:393 904 | 21-225_8H11 | VK1\|O12/JK3 | | DIQMTQSPSSLSA FVGDRVTITC | RAS--QNII-- ----SYLN | WYQQKPGK APNLMIY | V-------- TSSLHS | GVPSRFSGSGSG-- TDFSLTISSLQPEDFATYYC | QQSYS----------- ------------TPFT | FGPGT KVDIK |
| iPS:393 936 | 21-225_14A11 | VK1\|O12/JK3 | | DIQMTQSPSSLSA SIGDRVTITC | RAS--QSIS-- ----SYLN | WYQQKPGK APKLLIF | A-------- ASSLQN | GVPSRFSGSGSG-- TDFTLTISSLQPEDFATYYC | QQTYS----------- -----------SPPFT | FAPGT KVDIK |
| iPS:393 980 | 21-225_6D3 | VK1\|O12/JK3 | | DIQMTQSPSSLSA SVGDRVTITC | RTS--QSIS-- ----TYLN | WYQQKPGK APKLLIF | A-------- ASSLQS | GVPSRFSGSGSG-- TDFTLTISSLQPEDFATYFC | QQSYR----------- -----------TPFFT | FGPGT KVDIK |
| iPS:394 014 | 21-225_8G6 | VK1\|O12/JK3 | | DIQMTQSPSSLSA SVGDRVTITC | RAS--QSIS-- ----SYLN | WYQQKPGK APKLLIF | A-------- ASSLQS | GVPSRFSGSGSG-- TDFTLTISSLQPEDFATYFC | QQSYR----------- -----------TPFFT | FGPGT KVDIK |
| iPS:394 022 | 21-225_16H6 | VK1\|O12/JK3 | | DIQMTQSPSSLSA SVGDRVTITC | RAS--QNIS-- ----SYLN | WYQQKPGK APKLLIY | A-------- ASSLQS | GVPSRFSGRGSG-- TDFTLTISSLQPEDFATYYC | QQSYR----------- -----------TPLFT | FGPGT KVDFK |
| iPS:394 043 | 21-225_3B1 | VK1\|O12/JK3 | | DIQMTQSPSSLSA SVGDRVTITC | RAS--QSIN-- ----NYLN | WYQQKPGK APKLLIY | A-------- TSSLQN | GVPSRFSGSGSG-- TDFTLTISSLQPEDFATYYC | QQSYS----------- -----------TPLFT | FGPGT KVDIN |
| iPS:394 051 | 21-225_9E5 | VK1\|O12/JK3 | | DIQMTQSQSSLSA SVGDRVTITC | RAS--QSIA-- ----SYLN | WYQQKPGK APKLLIY | G-------- ASSLQS | GVPSRFSGSGSG-- TDFTLTISSLQPEDFATYYC | QQSYS----------- -----------TPLFS | FGPGT KVDIK |
| iPS:394 077 | 21-225_8E12 | VK1\|O12/JK3 | | DIQMTQSPSSLSA SVGDRVTITC | RAS--QSIS-- ----NYLN | WYQQKPGK APKLLIY | A-------- ASSLQS | GVPSRFSGSGSG-- TDFTLTISSLQPEDFATYFC | QQSYR----------- -----------TPFFT | FGPGT KVDIK |
| iPS:394 087 | 21-225_11A5 | VK1\|O12/JK3 | | DIQMTQSPSSLSA SVGDRVTITC | RAS--QNIY-- ----SYLN | WYQQKPGK APKLLIY | A-------- ASSLQS | GVPSRFSGSGSG-- TDFTLTISSLQPEDFATYYC | QQSYN----------- -----------TPLFT | FGPGT KVDIK |
| | | Germline | | K_FR1 | K_CDR1 | K_FR2 | K_CDR2 | K_FR3 | K_CDR3 | K_FR4 |
| | VK2\|A18/JK3 | | 32181 | DIVMTQTPLSLSV TPGQPASISC | KSS-- QSLLHSD- GKTYLY | WYLQKPGQ SPQLLIY | E-------- VSSRFS | GVPDRFSGSGSG-- TDFTLKISRVEAEDVGVYYC | MQGIH----------- ------------LPFT | FGPGT KVDIK |
| iPS:434 053 | 21-225_51E1 | VK2\|A18/JK3 | | DIVMTQTPLSLSV TPGQPASMSC | KSS-- QSLLHSE- GKTYLY | WYLRKPGQ PPQFLIF | E-------- VSNRFS | GVPDRFSGSGSG-- TEFTLKISRVEAEDVGVYYC | MQSIQ----------- ------------LPFT | FGPGT KVDIK |
| iPS:434 137 | 21-225_54D4 | VK2\|A18/JK3 | | DIVMTQTPLSLSV TPGQPASMSC | KSS-- QSLLHSE- GKTYLY | WYLRKPGQ PPQFLIF | E-------- VSNRFS | GVPDRFSGSGSG-- TEFTLKISRVEAEDVGIYYC | MQSIQ----------- ------------FPFT | FGPGT KVDIK |
| iPS:434 149 | 21-225_55H1 | VK2\|A18/JK3 | | DIVMTQTPLSLSV TPGQPASISC | KSS-- QSLLHSE- GKTYLY | WYLQKPGQ PPQFLIF | E-------- VSHRFS | GVPDRFSGSGSG-- TDFTLKISRVEAEDVGVYYC | MQSIQ----------- ------------LPFT | FGPGT KVDIK |

| | | | | | | | | | | |
|---|---|---|---|---|---|---|---|---|---|---|
| **iPS:435<br>315** | **21-<br>225_147B2** | **VK2\|A18/<br>JK3** | | DIVMTQTPLSLSV<br>TPGQPASISC | KSS--<br>QSLLHSE-<br>GKTYLY | WYLQKPGQ<br>PPQLLIY | E--------<br>VSHRVS | GVPDRFSGSGSG--<br>TDFTVKISRVEAEDVGVYYC | MQSTQ-----------<br>------------FPPT | FGPGT<br>KVDIK |
| | | **Germline** | | **K_FR1** | **K_CDR1** | **K_FR2** | **K_CDR2** | **K_FR3** | **K_CDR3** | **K_FR4** |
| | **VK1\|O18/J<br>K3** | | 32152 | DIQMTQSPSSLSA<br>SVGDRVTITC | QAS--QDIS--<br>----NYLN | WYQQKPGK<br>APKLLIY | D--------<br>ASNLET | GVPSRFSGSGSG--<br>TDFTFTISSLQPEDIATYYC | QQYDN-----------<br>------------LPFT | FGPGT<br>KVDIK |
| **iPS:434<br>055** | **21-<br>225_51B4** | **VK1\|O18/<br>JK3** | | DIQMTQSPSSLSA<br>SVGDRVTITC | QAS--RDIT--<br>----FYLN | WYQQKPGK<br>APKLLIY | D--------<br>ASNLET | GVPSRFSGSGSG--<br>TDFTFTISCVHPEDIATYLC | QQYDN-----------<br>------------LPFT | FGPGT<br>TVDIK |
| | | **Germline** | | **K_FR1** | **K_CDR1** | **K_FR2** | **K_CDR2** | **K_FR3** | **K_CDR3** | **K_FR4** |
| | **VK1\|O18/J<br>K4** | | 32153 | DIQMTQSPSSLSA<br>SVGDRVTITC | QAS--QDIS--<br>----NYLN | WYQQKPGK<br>APKLLIY | D--------<br>ASNLET | GVPSRFSGSGSG--<br>TDFTFTISSLQPEDIATYYC | QQYDN-----------<br>------------LPLT | FGGGT<br>KVEIK |
| **iPS:434<br>087** | **21-<br>225_52F6** | **VK1\|O18/<br>JK4** | | DIQMTQSPSSLSA<br>SVGDRVTITC | QAS--QDIS--<br>----NYLH | WYQQKPGK<br>APKLLIY | D--------<br>ASTLGT | GVPLRFSGSGSG--<br>TEFTFTINSLQPEDIATYSC | QQCDN-----------<br>------------LPLT | FGGGT<br>KVEIK |
| **iPS:434<br>111** | **21-<br>225_53H2** | **VK1\|O18/<br>JK4** | | DIQMTQSPSSLSA<br>SVGDRVTITC | QAS--QDIS--<br>----NYLH | WYQQKPGK<br>APQLLIY | D--------<br>ASNLET | GVPSRFTGSGSG--<br>TDFTFTISSLQPEDIATYYC | HQYDN-----------<br>------------LPLT | FGGGT<br>KVEIK |
| **iPS:434<br>121** | **21-<br>225_53F6** | **VK1\|O18/<br>JK4** | | DIQMTQSPSSLSA<br>SVGDRVTITC | QAS--QDIT--<br>----NYLD | WYQQKPGK<br>APKLLIY | D--------<br>ASNLGT | GVPSRFSGSGSG--<br>TDFTFTISSLQPEDIATYYC | QQCDN-----------<br>------------LPLT | FGGGT<br>KVEIK |
| **iPS:434<br>163** | **21-<br>225_50H1** | **VK1\|O18/<br>JK4** | | DIQMTQSPSSPSA<br>SVGDRVTITC | QAS--QDIS--<br>----NYLD | WYQQKPGK<br>APKLLIY | D--------<br>ASNLET | GVPSRFSGSGSG--<br>TDFAFTISSLQPEDIATYYC | QQCDN-----------<br>------------LPLT | FGGGT<br>KVEIK |
| **iPS:435<br>611** | **21-<br>225_161F10** | **VK1\|O18/<br>JK4** | | DIQMTQSPSSLSA<br>SVGDRVTITC | QAS--QDIY--<br>----NHLS | WYQQKPGK<br>APKLLIY | D--------<br>ASNWET | GVPSRFSGGGSG--<br>TDFTFTISSLQPEDFATYYC | QQYEN-----------<br>------------LPLT | FGGGT<br>KVEIK |
| **iPS:435<br>811** | **21-<br>225_183H6** | **VK1\|O18/<br>JK4** | | DIQMTQSPSSLSA<br>SVGDRVTIAC | QAS--QDIS--<br>----NYLN | WYQQTPGK<br>APKVLIY | D--------<br>ASNLET | GVPARFSGSGSG--<br>TDFTFTISSLQPEDIATYYC | QQYDN-----------<br>------------LPLT | FGGGT<br>KVDIK |
| **iPS:394<br>035** | **21-225_5G9** | **VK1\|O18/<br>JK4** | | DIQMTQSPSSLSA<br>SVGDRVTITC | QAS--QGIS--<br>----NSLN | WYQQKPGK<br>APKLLIY | D--------<br>ASNLET | GVPSRFSGSGAG--<br>TDFTFTISSLQPEDIATYYC | QQYDN-----------<br>------------LPLT | FGGGT<br>KVEIK |
| | | **Germline** | | **K_FR1** | **K_CDR1** | **K_FR2** | **K_CDR2** | **K_FR3** | **K_CDR3** | **K_FR4** |
| | **VK1\|L1/JK1** | | 32154 | DIQMTQSPSSLSA<br>SVGDRVTITC | RAS--QGIS--<br>----NYLA | WFQQKPGK<br>APKSLIY | A--------<br>ASSLQS | GVPSRFSGSGSG--<br>TDFTLTISSLQPEDFATYYC | QQYNS-----------<br>------------YPWT | FGQGT<br>KVEIK |
| **iPS:434<br>095** | **21-<br>225_52F10** | **VK1\|L1/J<br>K1** | | DIQMTQSPSSLSV<br>SVGDRVTITC | RAS--QGIS--<br>----NYLG | WFQQKPGK<br>APKSLIY | A--------<br>ASSLQS | GVPSRFSGSGSG--<br>TDFTLTISSLQPEDFATYYC | QQYNS-----------<br>------------YPPT | FGQGT<br>KVEIK |
| **iPS:392<br>848** | **21-<br>225_20F9** | **VK1\|L1/J<br>K1** | | DIQMTQSPSSLSA<br>SVGDRITITC | RAS--QGIS--<br>----NYLA | WFQQKPGK<br>APKSLIS | A--------<br>ASSLQS | GVPSKFSGSGSG--<br>TDFTLTISSLQPEDFATYCC | QQYHS-----------<br>------------YPWT | FGQGT<br>KVEIK |
| **iPS:393<br>078** | **21-<br>225_33H11** | **VK1\|L1/J<br>K1** | | DIQMTQSPSSLSA<br>FVGDRVTITC | WAS--QGIN--<br>----SYLA | WFQQRPGK<br>AHKSLIY | A--------<br>ASSLQG | GVPSKFSGSGSG--<br>TDFILTISSLQREDFATYYC | QQFNS-----------<br>------------YPLT | FGQGT<br>KVEIK |
| **iPS:393<br>142** | **21-<br>225_33A3** | **VK1\|L1/J<br>K1** | | DIQMTQSPSSLSA<br>SVGDRVTITC | RAS--QGIN--<br>----NYLA | WFQQKPGK<br>APKSLIY | A--------<br>ASSLQS | GVPSKFSGSGSG--<br>TDFTLTISSLQPEDFATYYC | QQFNS-----------<br>------------YPPT | FGQGT<br>KVEIK |
| **iPS:393<br>946** | **21-<br>225_16A4** | **VK1\|L1/J<br>K1** | | DIQMTQSPSSLSA<br>SVGDRVTITC | RAS--QDIS--<br>----NYLA | WFQQKPGK<br>APKSLIS | A--------<br>ASSLQS | GVPSKFSGSGSG--<br>TDFTLTISSLQPEDFATYYC | QQYHS-----------<br>------------YPWT | FGQGT<br>KVEIN |
| | | **Germline** | | **K_FR1** | **K_CDR1** | **K_FR2** | **K_CDR2** | **K_FR3** | **K_CDR3** | **K_FR4** |
| | **VK1\|O12/J<br>K5** | | 32155 | DIQMTQSPSSLSA<br>SVGDRVTITC | RAS--QSIS--<br>----SYLN | WYQQKPGK<br>APKLLIY | A--------<br>ASSLQS | GVPSRFSGSGSG--<br>TDFTLTISSLQPEDFATYYC | QQSYS-----------<br>------------TPIT | FGQGT<br>RLEIK |
| **iPS:434<br>117** | **21-<br>225_53C6** | **VK1\|O12/<br>JK5** | | DIQMTQSPSSLSA<br>SVGDRVTITC | RAS--QYSS--<br>----DYLN | WYQQKPGK<br>APKVLIF | A--------<br>ASSLKS | GVPSRFSGSGSG--<br>TDFTLTISSLEPEDFATYFC | QQSYS-----------<br>------------TPFT | FGQGT<br>RLEIK |
| **iPS:434<br>317** | **21-<br>225_59E8** | **VK1\|O12/<br>JK5** | | DIQMTQSPSSLSA<br>SVGDRVTITC | RAS--QSIS--<br>----SYLN | WYQQKPGK<br>APKLLIY | A--------<br>ASSLQS | GVPSRFSGSGSG--<br>TDFTLTISSLQPEDFATYFC | QQSFS-----------<br>------------NSIT | FGQGT<br>RLEIK |
| **iPS:434** | **21-** | **VK1\|O12/** | | DIQMTQSPSSLSA | RAS--QSIF-- | WYQVKPGK | D-------- | GVPSRFSGSGSG-- | QQSYG----------- | FGQGT |

| | | | | | | | | | | |
|---|---|---|---|---|---|---|---|---|---|---|
| iPS:434<br>455 | 21-<br>225_72F5 | VK1\|O12/<br>JK5 | | DIQMTQSPSSLSA<br>SVGDRVIIPC | RAS--QNIS--<br>----SYLN | WYQQKPGK<br>APKLLIY | A--------<br>ASSLQS | GVPSRFSGSGSG--<br>TDFTLTISSLQPEDFATYSC | QQTY------------<br>------------STPT | FGQGT<br>RLDIN |
| iPS:435<br>525 | 21-<br>225_157E7 | VK1\|O12/<br>JK5 | | DIQMTQSPSSLSA<br>SVGDRVTITC | RAS--QSFS--<br>----SYLN | WYQQKPGI<br>APKLLIY | A--------<br>ASSLQS | GVPSRFSGSGSG--<br>TDFTLTISSLQPEDFATYYC | QESYS-----------<br>------------IRFA | FGQGT<br>RLEIK |
| iPS:392<br>754 | 21-<br>225_21D3 | VK1\|O12/<br>JK5 | | DIQMTQSPSSLSA<br>SVGDRVTITC | RAS--QSIT--<br>----GYSN | WYQQKPGK<br>TPKLLIF | A--------<br>TYSLES | GVPSRFSGSGFG--<br>TNFTLTITSLQPEDFATYYC | QQSYS-----------<br>------------TSIT | FGQGT<br>RLEIK |
| iPS:392<br>818 | 21-<br>225_22D8 | VK1\|O12/<br>JK5 | | DIQMTQSPSSLSA<br>SVGDRVTITC | RTS--QNSN--<br>----SYLN | WYQQKPGK<br>APKLQIF | A--------<br>AYSLES | GVPSRFSGNRSG--<br>TEFTLTISSLQPEDFATYYC | QQTYG-----------<br>------------TSIT | FGQGT<br>RLEIK |
| iPS:393<br>064 | 21-<br>225_33A9 | VK1\|O12/<br>JK5 | | DIQMTQSPSSLSA<br>SVGDRVTITC | RAS--QSIS--<br>----RYLS | WYQQKPGR<br>APNLQIY | A--------<br>ASSLQS | GVPSRFSGSGSG--<br>TDFTLTISSLQPEDFATYYC | QQSYN-----------<br>------------IPIT | FGQGT<br>RLEIK |
| iPS:393<br>148 | 21-<br>225_35E5 | VK1\|O12/<br>JK5 | | DIQMIQSPSSLSA<br>SVGDRVTITY | RAS--QSIS--<br>----SYLN | WYQQKPAK<br>APKLHIY | G--------<br>ASSFQS | WVPSRFSGSGSS--<br>TDFTLTIISMQPGDYATYYC | HQSYN-----------<br>------------LPIT | FGQGT<br>RLEIK |
| iPS:398<br>536 | 21-<br>225_33D12 | VK1\|O12/<br>JK5 | | DVQMTQSPSSLSA<br>SLGDRVTITC | RAS--QSIR--<br>----SYLN | WYQQKPGK<br>APNLLIY | S--------<br>ASSLQS | GVPSRFSGSGSG--<br>TDFTLTISSLQPEDFANYYC | QQSYS-----------<br>------------IPIT | FGQGT<br>RLEIK |
| | | Germline | | **K_FR1** | **K_CDR1** | **K_FR2** | **K_CDR2** | **K_FR3** | **K_CDR3** | **K_FR4** |
| | VK3\|A27/JK<br>2 | | 32156 | EIVLTQSPGTLSL<br>SPGERATLSC | RAS--QSVSS-<br>----SYLA | WYQQKPGQ<br>APRLLIY | G--------<br>ASSRAT | GIPDRFSGSGSG--<br>TDFTLTISRLEPEDFAVYYC | QQYGS-----------<br>------------SPYT | FGQGT<br>KLEIK |
| iPS:434<br>127 | 21-<br>225_53H8 | VK3\|A27/<br>JK2 | | EIVLTQSPGTLSL<br>SPGERATLSC | RAS--QSITS-<br>----SYLA | WYQQKPGQ<br>APRLLIY | G--------<br>ASGRAT | GIPDRFSGSGSG--<br>TDFTLTISRLEPEDFAVYYC | QQFES-----------<br>-----------SPMCS | FGQGT<br>NLEIK |
| iPS:436<br>290 | 21-<br>225_205G3 | VK3\|A27/<br>JK2 | | EIVLTQSPGTLSL<br>SPGERATLSC | RAS--QNVSY-<br>----SYLA | WYQQKPGQ<br>APRLLIY | G--------<br>ASRRAT | GIPDRFSGSGSG--<br>TDFTLTISRLEPEDFAVYYC | QQYGS-----------<br>------------SPCS | FGQGT<br>KLEIK |
| iPS:436<br>568 | 21-<br>225_225B3 | VK3\|A27/<br>JK2 | | EIVLTQSPGTLSL<br>SPGERATLSC | RAS--QNLSS-<br>----SYLG | WYQQKPGQ<br>APRLLIY | D--------<br>TSSRAT | GIPDRFSGSGSG--<br>TDFTLTISRLEPEDFAVYYC | QEYGS-----------<br>-----------SLMCS | FGQGT<br>KLEIK |
| iPS:392<br>898 | 21-<br>225_21H10 | VK3\|A27/<br>JK2 | | EIVLTQSPGTLSL<br>SPGERATLSC | RAS--QSFSS-<br>----SYLA | WYQQKPGQ<br>APRLLIY | G--------<br>ASSRAT | GIPDRFSGSGSG--<br>TDFTLTISRLEPEDFAVYYC | QQYG------------<br>------------SSRS | FGQGT<br>KLEIK |
| iPS:393<br>802 | 21-<br>225_3D12 | VK3\|A27/<br>JK2 | | EIVLTQSPGTLSL<br>SPGERATLSC | RAS--QSVSS-<br>----SYLA | WYQQKPGQ<br>APRLLIY | G--------<br>TSSRAT | GIPDRFSGSGSG--<br>TDFTLTISRLEPEDFAVYYC | QQYG------------<br>------------SSRS | FGQGT<br>KLEIK |
| | | Germline | | **K_FR1** | **K_CDR1** | **K_FR2** | **K_CDR2** | **K_FR3** | **K_CDR3** | **K_FR4** |
| | VK1\|L1/JK4 | | 32157 | DIQMTQSPSSLSA<br>SVGDRVTITC | RAS--QGIS--<br>----NYLA | WFQQKPGK<br>APKSLIY | A--------<br>ASSLQS | GVPSRFSGSGSG--<br>TDFTLTISSLQPEDFATYYC | QQYNS-----------<br>------------YPLT | FGGGT<br>KVEIK |
| iPS:434<br>157 | 21-<br>225_55D4 | VK1\|L1/J<br>K4 | | DIQMTQSPSSLFA<br>SVGDRVTITC | RAS--QDIS--<br>----NYLI | WFQQKPGK<br>APKSLIY | T--------<br>ASSLQS | GVPSKFSGSGFG--<br>TDFTLTISNLQPEDFATYYC | QQYHS-----------<br>------------FPLT | FGGGT<br>RVEIR |
| iPS:434<br>175 | 21-<br>225_55A11 | VK1\|L1/J<br>K4 | | DIQMTQSPSSLSA<br>SVGDRVTITC | RAS--QDIN--<br>----IYLA | WFQQKPGK<br>APKSLIY | A--------<br>ASSLQS | GVPSKFSGSGSG--<br>TDFTLTISSLQPEDFATYYC | QQYNS-----------<br>------------YPLT | FGGGT<br>KVEIK |
| iPS:434<br>367 | 21-<br>225_65H11 | VK1\|L1/J<br>K4 | | DIQMTQSPSSLSA<br>SVGDRVTITC | RAS--QDIS--<br>----TYLA | WFQQKPGK<br>APKSLIY | A--------<br>ASSLQS | GVPSKFSGSGSG--<br>TDFTLTISSLQPEDFATYYC | QQYNS-----------<br>------------FPLT | FGGGT<br>KVEIK |
| iPS:434<br>429 | 21-<br>225_70H6 | VK1\|L1/J<br>K4 | | DIQMTQSPSSLSA<br>SLGDRVTITC | RTS--QSIF--<br>----NYLN | WFQRKPGK<br>APKVLIY | T--------<br>ASSLQS | GIPSRFSGSGSG--<br>TDFTLTISSLQPEDSATYYC | QQSYS-----------<br>------------IPLT | FGGGT<br>KVEIK |
| iPS:434<br>535 | 21-<br>225_74C8 | VK1\|L1/J<br>K4 | | DIQMTQSPSSLSA<br>SVGDRVTITC | RAS--QGIG--<br>----KYLA | WFQQKPGK<br>APKSLIY | T--------<br>TSNLQS | GAPSKFSGSGSG--<br>TDFTLTISSLQYEDFATYYC | QQYSN-----------<br>------------YPLT | FGGGT<br>KVEIN |
| iPS:434<br>573 | 21-<br>225_77E6 | VK1\|L1/J<br>K4 | | DIQMTQSPSSLSA<br>SVGDRVTITC | RAS--QGIS--<br>----KYLA | WFQQKPGK<br>APKSLIY | A--------<br>ASSLQG | GAPSKFSGSGSG--<br>TDFTLTISSLQPEDFATYYC | QQYSN-----------<br>------------YPLT | FGGGT<br>RVEIK |
| iPS:434<br>615 | 21-<br>225_76C5 | VK1\|L1/J<br>K4 | | DIQMTQSPSSLSA<br>SVGDRVTITC | RAS--QVIS--<br>----KYLA | WFQQKPGK<br>APKSLIY | A--------<br>ASSLQS | GAPSKFSGSGSG--<br>TDFTLTISSLQPEDFATYYC | QQYSN-----------<br>------------YPLT | FGGGT<br>KVEIK |
| iPS:434<br>669 | 21-<br>225_79F4 | VK1\|L1/J<br>K4 | | DIQMTQSPSSLSA<br>SVGDRVTITC | RAS--QGIS--<br>----KYLA | WFQQKPGK<br>APKSLIY | A--------<br>ASSLQG | GAPSKFSGSGSG--<br>TDFTLTISSLQPEDFATYYC | QQYSN-----------<br>------------YPLT | FGGGT<br>RVEIK |

| | | | | | | | | | | |
|---|---|---|---|---|---|---|---|---|---|---|
| iPS:434 737 | 21-225_74G6 | VK1\|L1/JK4 | | DIQMTQSPSSLSA<br>SVGDRVTITC | RAS--QGIG--<br>----KYLA | WFQQKPGK<br>APKSLIY | T--------<br>TSSLQS | GAPSKFSGSGSG--<br>TDFTLTISSLQYEDFATYYC | QQYSN-----------<br>------------YPLT | FGGGT<br>KVEIN |
| iPS:434 741 | 21-225_80C11 | VK1\|L1/JK4 | | DIQMTQSPSSLSA<br>SVGDRVTITC | RAS--QGIG--<br>----RYLA | WFQQKPGR<br>APKSLIY | T--------<br>ASSLQS | GAPSKFSGSGSG--<br>TDFTLTISSLQPEDFATYYC | QQYSN-----------<br>------------YPLT | FGGGT<br>KVEIN |
| iPS:434 867 | 21-225_79A12 | VK1\|L1/JK4 | | DIQMTQSPSSLSA<br>SVGDRVTITC | RAS--QVIS--<br>----KYLA | WFQQKPGK<br>APKSLIY | A--------<br>ASSLQS | GAPSKFSGSGSG--<br>TDFTLTISSLQPEDFATYYC | QQYSN-----------<br>------------YPLT | FGGGT<br>KVEIK |
| iPS:435 333 | 21-225_147E9 | VK1\|L1/JK4 | | DIQMTQSPSSLSA<br>SVGDRVTITC | RAS--QDIN--<br>----NYLA | WFQQKPGK<br>APKSLIV | A--------<br>ASSLQS | GVPSKFSGSGSG--<br>TDFTLTISSLQPEDFATYYC | QQYNS-----------<br>------------YPLT | FGGGT<br>KVEIK |
| iPS:435 409 | 21-225_150G8 | VK1\|L1/JK4 | | DIQMTQSPSSLSA<br>SVGDRVTITC | RAS--QGIS--<br>----HYLA | WFQQKPGK<br>APKSLIY | V--------<br>ASSLQN | GVPSRFSGSGSG--<br>TDFTLTISSLQPEDFATYYC | QQYNN-----------<br>------------YPLT | FGGGT<br>KVEIK |
| iPS:435 505 | 21-225_157C1 | VK1\|L1/JK4 | | DIQMTQSPSSLSA<br>SIGDRITLTC | RAS--QGIS--<br>----NYLA | WFQQRPGK<br>APKSLIY | A--------<br>ASSLLS | GVPSKFSGSGSG--<br>TDFTLTISSLQPEDFATYYC | QQYNS-----------<br>------------FPFT | FGGGT<br>KVELK |
| iPS:435 595 | 21-225_160H4 | VK1\|L1/JK4 | | DIQMTQSPSSLSA<br>SVGDRVTITC | RAS--QDIS--<br>----NYLV | WFQQKLGK<br>APKSLIY | V--------<br>ASSLQS | GVPSRFSGSGSG--<br>TDFTLTISSLQPEDFATYYC | QQYNS-----------<br>------------YPLT | FGGGT<br>KVEIK |
| iPS:435 639 | 21-225_163G6 | VK1\|L1/JK4 | | DIQMTQSPSSLSA<br>SVGDRVTITC | RAS--QDIS--<br>----NYLV | WFQQKPGK<br>APKSLIY | A--------<br>ASSLLS | GVPSKFSGSGSG--<br>TDFTLTISSLQPEDFATYYC | QQYHS-----------<br>------------YPLT | FGGGT<br>KVAIK |
| iPS:435 653 | 21-225_166H12 | VK1\|L1/JK4 | | DIQMTQSPSSLST<br>SVGDRVTITC | RAS--QDIS--<br>----HYLA | WFQQKPGK<br>APKSLIY | A--------<br>ASSLQS | GVPSKFSGSGSG--<br>TDFTLTISSLQPEDFATYYC | QQYNS-----------<br>------------FPLT | FGGGT<br>KVEIT |
| iPS:435 677 | 21-225_169C10 | VK1\|L1/JK4 | | DIQMTQSPSSLSA<br>SVGDRVTITC | RAS--QDIS--<br>----NYLA | WFQQKPGK<br>APKSLIF | S--------<br>ASSLQS | GVPSKFSGSGSG--<br>TDFNLTISSLQPEDFATYYC | QQSDS-----------<br>------------YPLT | FGGGT<br>KVEIK |
| iPS:435 699 | 21-225_170D6 | VK1\|L1/JK4 | | DIQMTQSPSSLSA<br>SVGDRVAITC | RAS--QDIG--<br>----NCLA | WFQQKPGK<br>APKSLIY | S--------<br>ASSLQS | GVPSKFSGSGSG--<br>TDFTLTISSLQPEDFATYYC | QQSDS-----------<br>------------YPLT | FGGGT<br>KVEIK |
| iPS:435 745 | 21-225_175G3 | VK1\|L1/JK4 | | DIQMTQSPSSLSA<br>SVGDRVTITC | RAS--QDIS--<br>----NDLA | WFQQKPGK<br>APKSLIF | S--------<br>ASSLQS | GVPSKFSGSGSG--<br>TDFTLTISSLQPEDFATYYC | QQSDS-----------<br>------------YPLT | FGGGT<br>KVEIK |
| iPS:435 819 | 21-225_190C11 | VK1\|L1/JK4 | | DIQMTQSPSSLSA<br>SVGDRVTITC | RTS--QGIG--<br>----NYLA | WFQQKPGK<br>APKSLLY | K--------<br>TSSLQS | GVPSKFSGSGSG--<br>TDFTLTISSLQPEDFATYYC | QQYMT-----------<br>------------YPLT | FGGGT<br>KVEIK |
| iPS:435 825 | 21-225_190G11 | VK1\|L1/JK4 | | DIQMTQSPSSLSA<br>SVGDRVTITC | RTS--QGIG--<br>----NYLA | WFQQKPGK<br>APKSLLY | K--------<br>ASSLQS | GVPSKFSGSGSG--<br>TDFTLTISSLQPEDFATYYC | QQYMT-----------<br>------------YPLT | FGGGT<br>KVEIK |
| iPS:435 837 | 21-225_198G3 | VK1\|L1/JK4 | | DIQMAQSPSSLSA<br>SVGDRVTITC | RTS--QGIG--<br>----KYLA | WFQQKPGK<br>APKSLLY | K--------<br>ASSLQG | GVPSKFSGSGSG--<br>TDFTLTISSLQPEDFATYYC | QQYMT-----------<br>------------YPLT | FGGGT<br>KVEIK |
| iPS:435 845 | 21-225_191G1 | VK1\|L1/JK4 | | DIQMTQSPSSLSA<br>SVGDRVTITC | RAS--QGIS--<br>----NYLA | WFQQKPGK<br>APKSLIY | A--------<br>ASSLQS | GVPSKFSGSGSG--<br>TDFTLTISSLQPEDFATYYC | QHYLT-----------<br>------------YPLT | FGGGT<br>KVEIK |
| iPS:435 859 | 21-225_190E6 | VK1\|L1/JK4 | | DIQMTQSPSSLSA<br>SVGDRVTITC | RTS--QGIG--<br>----NYLA | WFQQKPGK<br>APKSLLY | K--------<br>ASSLQS | GVPSKFSGSGSG--<br>TDFTLTISSLQPEDFATYYC | QQYMT-----------<br>------------YPLT | FGGGT<br>KVEIK |
| iPS:435 873 | 21-225_190G4 | VK1\|L1/JK4 | | DIQMTQSPSSLSA<br>SVGDRVTITC | RAS--QDIG--<br>----RYLA | WFQQKPGK<br>APKSLIY | A--------<br>ASSLQS | GVPSKFSGSGSG--<br>TDFTLTISSLQPENFATYYC | QQYST-----------<br>------------YPLT | FGGGT<br>KVEIK |
| iPS:435 933 | 21-225_190F8 | VK1\|L1/JK4 | | DIQMTQSPSSLSA<br>SVGDRVTITC | RTS--QGIG--<br>----NYLA | WFQQKPGK<br>APKSLLY | K--------<br>ASSLQS | GVPSKFSGSGSG--<br>TDFTLTISSLQPEDFATYYC | QQYMT-----------<br>------------YPLT | FGGGT<br>KVEIK |
| iPS:435 945 | 21-225_191A10 | VK1\|L1/JK4 | | DIQMTQSPSSLSA<br>SVGDRVTITC | RAS--QGIG--<br>----KYLA | WFQQKPGK<br>APKSLIY | A--------<br>ASSLQS | GVPSKFSGSGYG--<br>TDFTLTISSLQPENFATYYC | QQYST-----------<br>------------YPLT | FGGGT<br>KVEIK |

| | | | | | | | | | | |
|---|---|---|---|---|---|---|---|---|---|---|
| **iPS:435 947** | **21-225_191E10** | **VK1\|L1/JK4** | | DIQMTQSPSSLSA<br>SVGDRVTITC | RAS--QGIG--<br>----KYLA | WFQQKPGK<br>APKSLIY | A--------<br>ASSLQS | GVPSKFSGSGSG--<br>TDFTLTISSLQPENFATYYC | QQYST-----------<br>------------YPLT | FGGGT<br>KVEIK |
| **iPS:435 957** | **21-225_191G12** | **VK1\|L1/JK4** | | DIQMTQSPSSLSA<br>SVGDRVTITC | RTS--QGIG--<br>----NYLA | WFQQKPGK<br>APKSLLY | K--------<br>ASSLQS | GVPSKFSGSGSG--<br>TDFTLTISSLQPEDFATYYC | QQYIT-----------<br>------------YPLT | FGGGS<br>KVEIK |
| **iPS:435 963** | **21-225_192D2** | **VK1\|L1/JK4** | | DIQMIQSPSSLSA<br>SVGDRVTITC | RAS--QGIS--<br>----NYLA | WFQQKPGK<br>APKSLIY | A--------<br>ASSLQS | GVPSKFSGSGSG--<br>TDFTLTISSLQPEDFATYYC | QHYVT-----------<br>------------YPNT | FGGGT<br>KVEIK |
| **iPS:435 971** | **21-225_192D3** | **VK1\|L1/JK4** | | DIQMTQSPSSLSA<br>SVGDRVTITC | RAS--QGIS--<br>----NYLA | WFQQKPGK<br>APKSLIY | A--------<br>ASSLQS | GVPSKFSGSGSG--<br>TDFTLTISSLQPEDFATYYC | LHYLT-----------<br>------------YPLT | FGGGT<br>KVEIK |
| **iPS:435 979** | **21-225_192H4** | **VK1\|L1/JK4** | | DIQMTQSPSSLSA<br>SVGDRVTITC | RAS--QDIS--<br>----NYLA | WFQQKPGK<br>APKSLIS | A--------<br>ASSLQS | GVPSKFSGSRSG--<br>TDFTLTISSLQPEDFATYYG | LHYLN-----------<br>------------YPLT | FGGGT<br>RVEIR |
| **iPS:435 987** | **21-225_192G6** | **VK1\|L1/JK4** | | DIKMTQSPSSLSA<br>SVGDRVTITC | RTS--QGIG--<br>----NYLA | WFQQKPGK<br>APKSLLY | K--------<br>ASSLQS | GVPSKFSGSGSG--<br>TDFTLTISSLQPEDFATYYC | QQYMT-----------<br>------------YPLT | FGGGT<br>KVEIK |
| **iPS:435 993** | **21-225_192C8** | **VK1\|L1/JK4** | | DIQMTQSPSSLSA<br>SVGDRVTITC | RAS--QGIS--<br>----NYLA | WFQQKPGK<br>APKSLIY | A--------<br>ASSLQS | GVPSKFSGSGSG--<br>TDFTLTISSLQPEDFATYYC | QHYLT-----------<br>------------YPLT | FGGGT<br>KVEIK |
| **iPS:435 997** | **21-225_192G8** | **VK1\|L1/JK4** | | DIQMTQSPSSLSA<br>SVGDRVTITC | RTS--QGIG--<br>----NYLA | WFQQKPGK<br>APKSLLY | K--------<br>ASSLQS | GVPSKFSGSGSG--<br>TDFTLTISSLQPEDFATYYC | QQYIT-----------<br>------------YPLT | FGGGT<br>KVEIK |
| **iPS:436 005** | **21-225_192H10** | **VK1\|L1/JK4** | | DIQMTQSPSSLSA<br>SVGDRVTITC | RAS--QGIG--<br>----KYLA | WFQQKPGK<br>APKSLIY | A--------<br>ASSLQS | GVPSKFSGSGYG--<br>TDFTLTISSLQPENFATYYC | QQYST-----------<br>------------YPLT | FGGGT<br>KVEIK |
| **iPS:436 031** | **21-225_193C7** | **VK1\|L1/JK4** | | DIQMTQSPSSLSA<br>SVGDRVTITC | RAS--QGIG--<br>----KYLA | WFQQKPGK<br>APKSLIY | A--------<br>ASSLQS | GVPSKFSGSGSG--<br>TDFTLTISSLQPEDFATYYC | QQYST-----------<br>------------YPLT | FGGGT<br>KVEIK |
| **iPS:436 045** | **21-225_193A10** | **VK1\|L1/JK4** | | DIQMTQSPSSLSA<br>SVGDRVTITC | RAS--QGIS--<br>----NYLA | WFQQKPGK<br>APKSLIY | A--------<br>ASSLQS | GVPSKFSGSGSG--<br>TDFTLTISSLQPEDFATYFC | QHYLT-----------<br>------------YPLT | FGGGT<br>KVEIK |
| **iPS:436 054** | **21-225_194C1** | **VK1\|L1/JK4** | | DIQMTQSPSSLSA<br>SVGDRVTITC | RAS--QGIS--<br>----NYLA | WFQQKPGK<br>APKSLIY | A--------<br>ASSLQS | GVPSKFSGSGSG--<br>TDFTLTISSLQPEDFATYYC | LHYLT-----------<br>------------YPLT | FGGGT<br>KVEIK |
| **iPS:436 076** | **21-225_194H11** | **VK1\|L1/JK4** | | DIQMTQSPSSLSA<br>SVGDRVTITC | RAS--QGIS--<br>----NYLA | WFQQKPGK<br>APKSLIY | A--------<br>ASSLQS | GVPSKFSGSGSG--<br>TDFTLTISSLQPEDFATYYC | QHYLT-----------<br>------------YPLT | FGGGT<br>KVEIK |
| **iPS:436 086** | **21-225_191G10** | **VK1\|L1/JK4** | | DIQMTQSPSSLSA<br>SVGDRVTITC | RTS--QGIG--<br>----KYLA | WFQQKPGK<br>APKSLLY | K--------<br>VSSLQS | GVPSKFSGSGSG--<br>TDFTLTISSLQPEDFATYYC | QQYMT-----------<br>------------YPLT | FGGGT<br>KVEIK |
| **iPS:436 090** | **21-225_195A9** | **VK1\|L1/JK4** | | DIQMTQSPSSLSA<br>SVGDRVTITC | RAS--QGIS--<br>----NYLA | WFQQKPGK<br>APKSLIY | A--------<br>ASSLQS | GVPSKFSGSGSG--<br>TDFTLTISSLQPEDFATYYC | QHYLT-----------<br>------------YPLT | FGGGT<br>KVEIK |
| **iPS:436 112** | **21-225_196C7** | **VK1\|L1/JK4** | | DIQMTQSPSSLSA<br>SVGDRVTITC | RAN--QAIS--<br>----NYLA | WFQQKPGK<br>APKSLIY | A--------<br>ASSLQS | GVPSKFSGSGSG--<br>TDFTLTISSLQPEDFATYYC | QHYLT-----------<br>------------YPLT | FGGGT<br>KVEIK |
| **iPS:436 138** | **21-225_197F2** | **VK1\|L1/JK4** | | DIQMIQSPSSLSA<br>SVGDRVTITC | RTS--QGIG--<br>----NYLA | WFQQKPGK<br>APKSLLY | K--------<br>TSSLQS | GVPSKFSGSGSG--<br>TDFTLTISSLQPEDFATYYF | QQYIT-----------<br>------------YPLT | FGGGT<br>KVEIK |
| **iPS:436 152** | **21-225_197B6** | **VK1\|L1/JK4** | | DIQMTQSPSSLSA<br>SVGDRVTITC | RAS--QGIG--<br>----KYLA | WFQQKPGK<br>APKSLIY | G--------<br>ASSLQS | GVPSKFSGSGSG--<br>TDFTLTISSLQPENFATYYC | QQYST-----------<br>------------YPLT | FGGGT<br>KVEIK |
| **iPS:436 173** | **21-225_197G12** | **VK1\|L1/JK4** | | DIQMIQSPSSLSA<br>SVGDRVTITC | RTS--QGIG--<br>----NYLA | WFQQKPGK<br>APKSLLY | K--------<br>TSSLQS | GFPSKFSGSGSG--<br>TDFTLTISSLQPEDFATYYF | QQYMT-----------<br>------------YPLT | FGGGT<br>KVEIK |
| **iPS:436** | **21-** | **VK1\|L1/J** | | DIQMTQSPSSLSA | RAS--QGIG-- | WFQQKPGK | A-------- | GVPSKFSGNRSG-- | QQYST----------- | FGGGT |

| | | | | | | | | | | |
|---|---|---|---|---|---|---|---|---|---|---|
| iPS:436<br>201 | 21-<br>225_199C5 | VK1\|L1/J<br>K4 | | DIQMTQSPSSLSA<br>SVGDRVTITC | RAS--QGIS--<br>----KYLA | WFQQKPGK<br>APKSLIY | A--------<br>ASSLQS | GVPSKFSGSGSG--<br>TDFTLTISSLQPEDFAAYYC | QQYLT-----------<br>------------YPLT | FGGGT<br>KVEIK |
| iPS:436<br>260 | 21-<br>225_203H1 | VK1\|L1/J<br>K4 | | DIQMTQSPSSLSA<br>SVGDRVTITC | RAS--QGIG--<br>----NYLA | WFQQKPGK<br>APKSLIY | V--------<br>ASRLQS | GVPSKFSGTGSG--<br>SDFTLTISSLQPDDFATYYC | QRYHT-----------<br>------------YPLT | FGGGT<br>KVEIK |
| iPS:436<br>282 | 21-<br>225_204G6 | VK1\|L1/J<br>K4 | | DIRMTQSPSSLSA<br>SVGDRITITC | RTS--QGIG--<br>----NYLA | WFQQKPGK<br>APKSLIY | A--------<br>ASSLQS | GVPSRFSGSGSG--<br>TDFTLTISSLQPEDFATYYC | QHYLS-----------<br>------------YPLT | FGGGT<br>KVEIK |
| iPS:436<br>284 | 21-<br>225_204G8 | VK1\|L1/J<br>K4 | | DIQMTQSPSSLSA<br>SVGDRVTITC | RAS--QGIS--<br>----NHLA | WFQQKPGK<br>APKSLIF | A--------<br>ASSLQS | GVPSQFSGSGSG--<br>TEFTLTISSLQPEDFATYYC | QQYSN-----------<br>------------YPVT | FGGGT<br>KVEIK |
| iPS:436<br>292 | 21-<br>225_205H3 | VK1\|L1/J<br>K4 | | DIPMTQSPSSLSA<br>SVGDRVTITC | RAS--QAIS--<br>----NHLA | WFQLKPGK<br>APKSLIY | A--------<br>ASSLQS | GVPSKFSGSGSG--<br>SDFTLTISSLQPEDFATYYC | QQYSN-----------<br>------------YPLT | FGGGT<br>KVEIK |
| iPS:436<br>296 | 21-<br>225_205F5 | VK1\|L1/J<br>K4 | | DIQMTQSPSSLSA<br>SVGDRVTITC | RAS--QGIG--<br>----NYLA | WFQQKPGK<br>APKSLIY | G--------<br>VSSLQS | GVPSKFSGSGSG--<br>TDFTLTISSLQPEDFATYTC | QQYSN-----------<br>------------YPLT | FGGGT<br>KVDIR |
| iPS:436<br>324 | 21-<br>225_207G6 | VK1\|L1/J<br>K4 | | DIQMTQSPSSLSA<br>SVGDRVTITC | RAS--QGIR--<br>----NYLA | WLQQKPGK<br>APKSLIH | A--------<br>ASSLQS | GVPSKFSGNRSG--<br>TDFTLTISSLQPEDFATYYC | QQYSN-----------<br>------------YPLT | FGGGT<br>KVEIK |
| iPS:436<br>364 | 21-<br>225_211A1<br>1 | VK1\|L1/J<br>K4 | | DIQMTQSPSSLSA<br>SVGDRVTITC | RAS--QGIG--<br>----NYLA | WFQQKPGK<br>APRSLIY | D--------<br>ASSLES | GVPSKFSGSRSG--<br>TDFTLTIGSLQPEDFATYYC | QHYMT-----------<br>------------YPLT | FGAGT<br>KVEIK |
| iPS:436<br>366 | 21-<br>225_211A3 | VK1\|L1/J<br>K4 | | DIRMTQSPSSLSA<br>SVGDRVTITC | RAS--QAIG--<br>----KHLA | WFQQRPGK<br>APKSLIY | A--------<br>ASRLQS | GVPSKFSGSGSG--<br>TDFTLTISSLQPEDLATYYC | QQYSN-----------<br>------------YPLT | FGGGT<br>KVEIK |
| iPS:436<br>372 | 21-<br>225_211A8 | VK1\|L1/J<br>K4 | | DIEMTQSPPSLSA<br>FVGDRVTITC | RAS--QGIS--<br>----RYLA | WVQQKPGK<br>APKSLIY | A--------<br>ASSLQS | GVSSRFSGSGSG--<br>TDFTLTISSLQPEDFATYYC | LRYDT-----------<br>------------YPLI | FGGGT<br>KVEIK |
| iPS:436<br>376 | 21-<br>225_212E6 | VK1\|L1/J<br>K4 | | DIQMTQSPSSLSA<br>SVGDRVTITC | RAS--QGIS--<br>----NHLA | WFQQKPGQ<br>APKSLIY | A--------<br>ASSLQS | GVPSKFSGSRSG--<br>TDFTLTISSLQPEDFATYYC | QQYVT-----------<br>------------YPLT | FGGGT<br>KVEIK |
| iPS:436<br>378 | 21-<br>225_212D7 | VK1\|L1/J<br>K4 | | DIQMTQSPSSLSA<br>SVGDRVTITC | RAS--QGIS--<br>----NHLA | WFQQKPGK<br>APKSLIH | A--------<br>ASSLQS | GVPSKFSGNRSG--<br>TDFTLTINNLQPEDFVTYYC | QQYSN-----------<br>------------YPLT | FGGGT<br>KVEIK |
| iPS:436<br>380 | 21-<br>225_212H9 | VK1\|L1/J<br>K4 | | DIEMTQSPSSLSA<br>SVGDRVTITC | RAS--QGIS--<br>----SYLA | WLQQKPGK<br>APKSLIY | A--------<br>ASSLQS | GVPSRFSGSGSG--<br>TDFTLTISSLQPEDFATYYC | LRYDT-----------<br>------------YPLT | FGGGT<br>KVEIK |
| iPS:436<br>384 | 21-<br>225_212F10 | VK1\|L1/J<br>K4 | | DIQMTQSPSSLSA<br>SVGDRVTITC | RAS--QGIS--<br>----NYLD | WFQQKPGK<br>APKSLIY | S--------<br>ASNLQS | GVPSKFSGSRSG--<br>TDFTLTISSLQPEDFATYYC | QHYSN-----------<br>------------YPLT | FGGGT<br>KVEIK |
| iPS:436<br>388 | 21-<br>225_212H1<br>1 | VK1\|L1/J<br>K4 | | DIRMTQSPSSLSA<br>SVGDRVTITC | RAS--QAIG--<br>----KHLA | WFQQRPGK<br>APKSLIY | A--------<br>ASRLQS | GVPSKFSGSGSG--<br>TDFTLTISSLQPEDLATYYC | QHYSN-----------<br>------------YPLT | FVGGT<br>KVEIT |
| iPS:436<br>390 | 21-<br>225_213D2 | VK1\|L1/J<br>K4 | | DIQMTQSPSSLSA<br>SVGDRVTITC | RAS--QGIS--<br>----NHLA | WFQQKPGK<br>APKSLIH | A--------<br>ASSLQS | GVPSKFSGNRSG--<br>TDFTLTINNLQPEDFVTYYC | HQYSN-----------<br>------------YPLT | FGGGT<br>KVEIK |
| iPS:436<br>394 | 21-<br>225_213C4 | VK1\|L1/J<br>K4 | | DIHMTQSPSSLSA<br>SVGDRVTITC | RAS--QAIR--<br>----NYLA | WCQQKPGK<br>APKTLIY | A--------<br>ASSLQS | GVPSKFSGSGSG--<br>TDFTLTISSLQPEDFATYYC | QQYSN-----------<br>------------YPLT | FGGGT<br>KVEIK |
| iPS:436<br>396 | 21-<br>225_213E5 | VK1\|L1/J<br>K4 | | DIRMTQSPSSLSA<br>SVGDRVTITC | RAS--QAIG--<br>----KHLA | WFQQRPGK<br>APKSLIY | A--------<br>ASRLQS | GVPSKFSGSGSG--<br>TDFTLTISSLQPEDLATYYC | QHYSN-----------<br>------------YPLT | FGGGT<br>KVEIK |
| iPS:436<br>398 | 21-<br>225_213B8 | VK1\|L1/J<br>K4 | | DIQMTQSPSSLSA<br>SVGDRVTITC | RAS--QGIS--<br>----NHLA | WFQQKPGK<br>APKSLIY | A--------<br>ASSLQS | GVPSKFSGSGSG--<br>TDFTLTISSLQPEDFATYYC | QQYSN-----------<br>------------YPLT | FGGGT<br>KVEIK |
| iPS:436<br>410 | 21-<br>225_212E1<br>0 | VK1\|L1/J<br>K4 | | DIQMTQSPSSLSA<br>SVGDRVTITC | RAS--QGIS--<br>----NHLA | WFQQKPGK<br>APKSLIY | A--------<br>ASSLQS | GVPSKFSGSGSG--<br>TDFTLTISSLQPEDFATYYC | QQYSN-----------<br>------------YPLT | FGGGT<br>KVEIK |
| iPS:436<br>420 | 21-<br>225_215B5 | VK1\|L1/J<br>K4 | | DIQMTQSPSSLSA<br>SVGDRVTITC | RAS--QGIS--<br>----NHLA | WFQQKPGK<br>APKSLIH | A--------<br>ASSLQS | GVPSKFSGNRSG--<br>TDFTLTINNLQPEDFVTYYC | QQYIN-----------<br>------------YPLT | FGGGT<br>KVEIK |
| iPS:436 | 21- | VK1\|L1/J | | DIQMTQSPSSLSA | RAS--QGIS-- | WFQQKPGK | A-------- | GVPSKFSGSRSG-- | QQYVT----------- | FGGGT |

| | | | | | | | | | | |
|---|---|---|---|---|---|---|---|---|---|---|
| iPS:436 430 | 21-225_215A12 | VK1\|L1/JK4 | | DIQMTQSPSSLSA<br>SVGDRITITC | RTS--QDIG--<br>----NYLA | WFQQKPGK<br>APKSLIY | A--------<br>ASSLQS | GVPSKFSGSRSG--<br>TDFTLTISSLQSEDFATYYC | QQYVT-----------<br>------------YPLT | FGGGT<br>KVEIK |
| iPS:436 452 | 21-225_217G5 | VK1\|L1/JK4 | | DILMTQSPSSLSA<br>SVGDRVTITC | RAS--QGIG--<br>----NYLA | WFQQRPGK<br>APKSLIY | A--------<br>ASSLQS | GVPSKFSGTRSG--<br>TDFTLTISSLQPDDFATYYC | QQYVN-----------<br>------------YPLT | FGGGT<br>KVEIN |
| iPS:436 454 | 21-225_217B10 | VK1\|L1/JK4 | | DIRMTQSPSSLSA<br>SVGDRVTITC | RAS--QAIG--<br>----KHLA | WFQQRPGK<br>APKSLIY | A--------<br>ASRLQS | GVPSKFSGSGSG--<br>TDFTLTISSVQPEDLATYYR | QHTSK-----------<br>------------SPVQ | LVGGT<br>KVEIT |
| iPS:436 464 | 21-225_219H1 | VK1\|L1/JK4 | | DIQMTQSPSSQSA<br>SVGDRVTITC | RAS--QGIS--<br>----NYLD | WFQQKPGK<br>APKSLIY | S--------<br>ASNLQS | GVPSKFSGSRSG--<br>TDFTLTISSLQPEDFATYYC | QHYSN-----------<br>------------YPLT | FGGGT<br>KVEIK |
| iPS:436 490 | 21-225_221F6 | VK1\|L1/JK4 | | DIQMTQSPSSLSG<br>SVGDRVTITC | RAS--QGIS--<br>----NYLA | WFQQKPGK<br>APKSLIY | A--------<br>ASNLQS | GVPSKFSGSRSG--<br>TDFTLTISSLQPEDFATYYC | QQYMT-----------<br>------------YPLT | FGGGT<br>RVEIK |
| iPS:436 502 | 21-225_222A11 | VK1\|L1/JK4 | | DIQMTQSPSSLSA<br>SVGDRVTITC | RAS--QGIS--<br>----NYLA | WFQQKPGK<br>APKSLIY | A--------<br>ASSLQS | GVPSKFSGSGSG--<br>TDFTLTISSLQPEDFATYYC | LYYLN-----------<br>------------YPLT | FGGGT<br>KVEIK |
| iPS:436 514 | 21-225_222D10 | VK1\|L1/JK4 | | DIQMTQSPSSLSA<br>SVGDRVTITC | RAS--QGIS--<br>----NYLA | WFQQKPGK<br>APKSLIY | A--------<br>ASSLQS | GVPSKFSGSGSG--<br>TDFTLTISSLQPEDFATYYC | LHYLN-----------<br>------------YPLT | FGGGT<br>RVEIK |
| iPS:436 522 | 21-225_223H10 | VK1\|L1/JK4 | | DIQMTQSPSSLSA<br>SVGDRVTITC | RAS--QGIS--<br>----NYLA | WFQQKPGK<br>APKSLIY | A--------<br>ASNLQS | GVPSKFSGSGSG--<br>TDFTLTISSLQPEDFATYYC | LHYLN-----------<br>------------YPLT | FGGGT<br>KVEIK |
| iPS:437 258 | 21-225_153F9 | VK1\|L1/JK4 | | DIQMTQSPSSLSA<br>SVGDRVTITC | RAS--QGIS--<br>----NYLA | WFQQKPGK<br>APKSLIS | A--------<br>ASSLQS | GVPSKFSGSGSG--<br>TDFTLTISSLQPEDFATYYC | QQYNS-----------<br>------------YPLS | FGGGT<br>KVEIK |
| iPS:437 260 | 21-225_170D1 | VK1\|L1/JK4 | | DIQMTQSPSSLAA<br>SVGDRVTITC | RAS--QDIS--<br>----NYLA | WFQQKPGK<br>APKSLIY | A--------<br>ASSLQS | GVPSRFSGSGSG--<br>TDFTLTISSLQPEDFATYYC | QQCDS-----------<br>------------FPLT | FGGGT<br>KVEIK |
| iPS:437 264 | 21-225_171H12 | VK1\|L1/JK4 | | DIQMTQSPSSLSA<br>SVGDRVTITC | RAS--QDIS--<br>----NYLA | WFQQKPGK<br>APKSLIY | S--------<br>ASSLQS | GVPSKFSGSGSG--<br>TDFTLTISSLQPEDFATYYC | QQSDS-----------<br>------------YPLT | FGGGT<br>KVEIK |
| iPS:437 266 | 21-225_177A5 | VK1\|L1/JK4 | | DIQMTQSPSSLSA<br>SVGDRVTITC | RAS--QDIS--<br>----NYLA | WFQQKPGK<br>APKSLIY | S--------<br>ASSLQS | GVPSKFSGSGSG--<br>TDFTLTISSLQPEDFATYYC | QQSDS-----------<br>------------YPLT | FGGGT<br>KVEIK |
| iPS:437 270 | 21-225_178H4 | VK1\|L1/JK4 | | DIQMTQSPSSLSA<br>SVGDRVTITC | RAS--QDIS--<br>----NYLA | WFQQKPGK<br>APKSLIF | S--------<br>ASSLQS | GVPSKFSGSGSG--<br>TDFTLTISNLQPEDFATYYC | QQSNS-----------<br>------------YPLT | FGGGT<br>KVEIK |
| iPS:438 664 | 21-225_216G1 | VK1\|L1/JK4 | | DIEMTQSPSSLSA<br>SVGDRVTITC | RAS--QGIS--<br>----SYLA | WLQQKPGK<br>APKSLIY | A--------<br>ASSLQS | GVPSRFSGSGSG--<br>TDFTLTISSLQPEDFATYYC | LRYDT-----------<br>------------YPLT | FGGGT<br>KVEIK |
| iPS:451 120 | 21-225_197D3 | VK1\|L1/JK4 | | DIQMTQSPSSLSA<br>SVGDRVTITC | RAS--QGIR--<br>----NYLA | WFQQKPGK<br>APKSLIY | A--------<br>ASSLQS | GVPSKFSGSGSG--<br>TDFTLTISSLQPEDFATYYC | QHYLT-----------<br>------------YPLT | FGGGT<br>KVEIK |
| iPS:392 682 | 21-225_16A12 | VK1\|L1/JK4 | | DIQMTQSPSSLSA<br>SVGDRVTITC | RAS--QAIN--<br>----TYLA | WFQQKPGK<br>APKSLIS | A--------<br>ASSLQS | GVPSKFSGSGSG--<br>TEFTLTISSLQPEDFATYYC | QQYYS-----------<br>------------YPLT | FGGGT<br>KVEIK |
| iPS:392 856 | 21-225_22A2 | VK1\|L1/JK4 | | DIQMTQSPSSLSA<br>SVGDRVTITC | RAS--QDIS--<br>----NYLA | WVQQKPGK<br>APKSLIY | A--------<br>ASSLQS | GVPSKFSGSGSG--<br>TDFTLTISSLQPEDFASYYC | QQYNS-----------<br>------------FPLT | FGGGT<br>KVEIK |
| iPS:392 966 | 21-225_32G3 | VK1\|L1/JK4 | | DIQMTQSPTSLSA<br>SVGDRVTITC | RAS--QAIS--<br>----NYLA | WFQQKPGK<br>APKSLIY | D--------<br>TSSLQS | GVPSKFSGSGSG--<br>TDFTLTISTLQPEDFATYYC | QQYHS-----------<br>------------YPLT | FGGGT<br>KVEIK |
| iPS:393 952 | 21-225_1F1 | VK1\|L1/JK4 | | DIQMTQSPSSLSA<br>SVGDRVTITC | RAS--QGIN--<br>----NYLA | WFQQKSGK<br>APKSLIS | V--------<br>ASSLQT | GVPSRFSGSGSG--<br>TDFTLTISSLQPEDFATYYC | QQYNS-----------<br>------------YPLT | FGGGT<br>KVEIK |
| iPS:393 988 | 21-225_7F10 | VK1\|L1/JK4 | | DIQMTQSPSALSA<br>SVGDRVTITC | RAS--QDIR--<br>----NYLA | WFQQKPGK<br>APKSLIS | V--------<br>ASSLQS | GVPSRFSGSGSG--<br>TDFTLTISSLQPEDFATYYC | QQYNS-----------<br>------------YPLT | FGGGT<br>KVEIK |

| | | | | | | | | | | |
|---|---|---|---|---|---|---|---|---|---|---|
| iPS:402<br>233 | 21-<br>225_16D10 | VK1\|L1/J<br>K4 | | DIQMTQSPSSLSA<br>SVGDRVTITC | RAS--QDIS--<br>----NYLA | WFQQKPGK<br>APKSLIY | A--------<br>TPSLQS | GVPSKFSGSGSG--<br>TEFTLTISSLQPEDFATYYC | QQYNS-----------<br>------------YPLT | FGGGT<br>KVEIK |
| iPS:402<br>237 | 21-<br>225_23D11 | VK1\|L1/J<br>K4 | | DIQMTQSPSSLSA<br>SVGDRVTITC | RAS--QGIA--<br>----NYLA | WFQQRPGK<br>APKSLIS | A--------<br>ASSLQS | GVPSKFSGSGSG--<br>TEFTLTISSLQPEDFATYYC | QQYHS-----------<br>------------YPLT | FGGGS<br>KVEIK |
| | | **Germline** | | **K_FR1** | **K_CDR1** | **K_FR2** | **K_CDR2** | **K_FR3** | **K_CDR3** | **K_FR4** |
| | VK1\|O18/J<br>K5 | | 32158 | DIQMTQSPSSLSA<br>SVGDRVTITC | QAS--QDIS--<br>----NYLN | WYQQKPGK<br>APKLLIY | D--------<br>ASNLET | GVPSRFSGSGSG--<br>TDFTFTISSLQPEDIATYYC | QQYDN-----------<br>------------LPIT | FGQGT<br>RLEIK |
| iPS:434<br>171 | 21-<br>225_50G4 | VK1\|O18/<br>JK5 | | DIQMTQSPTSLSA<br>SVGDRVTITC | QAS--QDIT--<br>----NFLN | WYQQKPGK<br>APKLLIY | D--------<br>ASNLET | GVPSRFSGSGSG--<br>TDFTFTISSLQPEDIATYYC | QQYD------------<br>------------NLIT | FGQGT<br>RLEIK |
| iPS:435<br>565 | 21-<br>225_159C4 | VK1\|O18/<br>JK5 | | DIQMTQSPSSLSA<br>SVGDRVTITC | QAS--QDIS--<br>----DYLN | WYQQKPGK<br>APKLLIY | D--------<br>ASTLET | GVPSRFSGSGSG--<br>TDFTFTISSLQPEDIATYFC | QQYDN-----------<br>------------LPIT | FGQGT<br>RLEIK |
| iPS:435<br>607 | 21-<br>225_161G4 | VK1\|O18/<br>JK5 | | DIQMTQSPSSLSA<br>SVGDRVTITC | QAS--QDIY--<br>----NYLN | WYQQKPGK<br>APKLLIY | D--------<br>ASNLET | GVPSRFSGSGSG--<br>TDFTFAISSLQPEDIATYYC | QQYDI-----------<br>------------LPIT | FGQGT<br>RLEIK |
| iPS:437<br>302 | 21-<br>225_225B1<br>1 | VK1\|O18/<br>JK5 | | DIQMTQSPSSLSA<br>SIGDRVTITC | QAS--QDIF--<br>----NYLN | WYQQKPGK<br>APKLLIY | D--------<br>ASTLET | GVPSRFSGSGSG--<br>TDFTFTISSLQPEDIATYYC | QQYDN-----------<br>------------LPIT | FGQGT<br>RLEIK |
| iPS:392<br>868 | 21-<br>225_24D6 | VK1\|O18/<br>JK5 | | DIQMTQSPSSLSA<br>SVGDRVTITC | QAS--QDIN--<br>----NYLN | WYQQKPGK<br>ALKLLIY | D--------<br>ASDLET | GVPSRFSGSGSG--<br>TDFTFTISSLQPEDIAIYYC | QQYEN-----------<br>------------LPIT | FGQGT<br>RLEIK |
| iPS:393<br>020 | 21-<br>225_30E2 | VK1\|O18/<br>JK5 | | DIQMTQSPHSLSA<br>SVGDRVTITC | QAS--QYIS--<br>----NYLN | WYQQKSGK<br>APKLLIY | D--------<br>GSSLET | GVPSRFSGSGSG--<br>TDFTFTISSLQPEDLATYYC | QQYDN-----------<br>------------LPIT | FGQGT<br>RLEIK |
| iPS:393<br>138 | 21-<br>225_35E3 | VK1\|O18/<br>JK5 | | DIQMTQSPSSLSA<br>SVGDRVTITC | QAS--QDIF--<br>----NYLN | WYQQKPGK<br>APNLLIY | D--------<br>ASNLET | GVPSRFSGSGSG--<br>TDFTFTISSLQPEDIATYFC | QQYDN-----------<br>------------LPIT | FGQGT<br>RLDIR |
| iPS:393<br>892 | 21-225_6G7 | VK1\|O18/<br>JK5 | | DIQMTQSPSSRSA<br>SVGDRVTITC | QAS--QDIS--<br>----NYLN | WCQQKPGK<br>ALKLLIY | D--------<br>ASTLET | GVPSRFSGSGSG--<br>TDFTFTISSVQPEDIATYYC | QQYDN-----------<br>------------VPIT | FGQGT<br>RLEIK |
| iPS:393<br>910 | 21-<br>225_15F10 | VK1\|O18/<br>JK5 | | DIQMTQSPSSLSA<br>SVGDRVTITC | QAN--QDIT--<br>----NFLN | WYQLKPGK<br>APNLLIS | D--------<br>ASNLET | GVPSRFSGSGSG--<br>TDFTFTISSLQPEDVATYYC | QQYDN-----------<br>------------LPIT | FGQGT<br>RLEIK |
| iPS:393<br>912 | 21-<br>225_16F6 | VK1\|O18/<br>JK5 | | DIQMTQSPSSLSA<br>SVGDRVTITC | QAN--QDIT--<br>----NFLN | WYQLKPGK<br>APNLLIS | D--------<br>ASNLET | GVPSRFSGSGSG--<br>TDFTFTISSLQPEDVATYYC | QQYDN-----------<br>------------LPIT | FGQGT<br>RLEIK |
| iPS:394<br>000 | 21-<br>225_11A2 | VK1\|O18/<br>JK5 | | DIQMTQSPSSLSA<br>SVGDRVTITC | QAS--QDIS--<br>----NYLN | WYQQKPGK<br>APKLLIY | D--------<br>ASNLET | GVPSRFSGSGSG--<br>TDFTFTISSLQPEDVATYYC | QQYDN-----------<br>------------LPIT | FGQGT<br>RLDIK |
| iPS:394<br>004 | 21-<br>225_13A1 | VK1\|O18/<br>JK5 | | DIQMTQSPSSLSA<br>SVGDRVTITC | QAS--QDIN--<br>----NYLN | WYQQKLGT<br>APKLLIY | D--------<br>GSNLET | GVPSRFSGSGSG--<br>TDFTFTISSLQPEDIATYYC | QQYEN-----------<br>------------LPIT | FGQGT<br>RLEIK |
| iPS:394<br>006 | 21-<br>225_15C2 | VK1\|O18/<br>JK5 | | DIQMTQSPSSLSA<br>SVGDRVTITC | QAS--QDIT--<br>----NYLN | WYQQKPGK<br>APNLLIY | D--------<br>ASNLET | GVPSRFSGSGSG--<br>TDFTFTISSLQPEDIATYYC | QQYDN-----------<br>------------LPIT | FAQGT<br>RLEIK |
| iPS:394<br>029 | 21-<br>225_1B12 | VK1\|O18/<br>JK5 | | DIQMTQSPSSLSA<br>SVGDRVTITC | QAS--QDIN--<br>----NYLN | WYQQKPGK<br>APKLLIY | D--------<br>ASNLET | GVPSRFSGSGSG--<br>TDFTFTISSLQPEDITTYYC | QQYEN-----------<br>------------LPIT | FGQGT<br>RLEIK |
| iPS:394<br>047 | 21-225_5E6 | VK1\|O18/<br>JK5 | | DIQMTQSPSSLSA<br>SVGDRVTITC | QAS--QDIN--<br>----NYLN | WCQQKPGK<br>APKLLIY | D--------<br>ASNLET | GVPSRFSGSGSG--<br>TDFTFTISSLQPEDIATYYC | QQYDN-----------<br>------------LPIT | FGQGT<br>RLEIK |
| iPS:394<br>081 | 21-<br>225_16B3 | VK1\|O18/<br>JK5 | | DIQMTQSPSSLSA<br>SVGDRVTITC | QAS--QDIN--<br>----NYLN | WYQQKPGR<br>APKLLIY | D--------<br>ASNLET | GVPSRFSGSGSG--<br>TDFTFTISSLQPEDIATYYC | QQFDN-----------<br>------------LPIT | FGQGT<br>RLEIK |
| | | **Germline** | | **K_FR1** | **K_CDR1** | **K_FR2** | **K_CDR2** | **K_FR3** | **K_CDR3** | **K_FR4** |
| | VK3\|L2/JK3 | | 32159 | EIVMTQSPATLSV<br>SPGERATLSC | RAS--QSVS--<br>----SNLA | WYQQKPGQ<br>APRLLIY | G--------<br>ASTRAT | GIPARFSGSGSG--<br>TEFTLTISSLQSEDFAVYYC | QQYNN-----------<br>------------WPFT | FGPGT<br>KVDIK |
| iPS:434<br>219 | 21-<br>225_60E9 | VK3\|L2/J<br>K3 | | EIVMTQSPATLSV<br>SPGERATLSC | RAS--QSVS--<br>----SSLA | WYRQKPGQ<br>APRLLIY | G--------<br>ASTRAT | GIPARFSGSGSG--<br>TEFTLTISSLQSEDFAVYCC | QQYNN-----------<br>------------WPFT | FGPGT<br>KIDIK |
| iPS:434 | 21- | VK3\|L2/J | | EIVMTQSPATLSV | RAS--QSVS-- | WYQQKPGQ | G-------- | GMPARFSGGGSG-- | QQYSN----------- | FGPGT |

| | | | | | | | | | | |
|---|---|---|---|---|---|---|---|---|---|---|
| iPS:434<br>289 | 21-<br>225_57H12 | VK3\|L2/J<br>K3 | | EIVMTQSPATLSV<br>SPGERATLSC | RAS--QSVS--<br>----SDLA | WYQQKPGQ<br>APRLLIY | A--------<br>ASTRAT | GIPARFSGSGSG--<br>TEFTLTISSLQSEDFAVYYC | QQYDN-----------<br>------------WPFT | FGPGT<br>KVDNK |
| iPS:434<br>291 | 21-<br>225_58A4 | VK3\|L2/J<br>K3 | | EIVMTQSPATLSV<br>SPGERATLSC | RAS--QSVS--<br>----SDLA | WYQQRPGQ<br>APRLLIY | A--------<br>ASTRAT | GIPARFSGSGSG--<br>TEFTLTISSLQSEDFAVYYC | QQFNN-----------<br>------------WPFT | FGPGT<br>KVDIK |
| iPS:434<br>297 | 21-<br>225_58A10 | VK3\|L2/J<br>K3 | | EIVMTQSPATLSV<br>CPGERATLSC | RAS--QSVS--<br>----SSLA | WYQQKPGQ<br>APRLLIY | G--------<br>ASTRAT | GIPARFSGSGSG--<br>TEFTLTISSLQSEDFAVYYC | QQYNN-----------<br>------------WPFT | FGPGT<br>KVDIK |
| iPS:434<br>301 | 21-<br>225_58F11 | VK3\|L2/J<br>K3 | | EIVMTQSPATLSV<br>SPGERATLSC | RAS--QSVS--<br>----SDLV | WYQQKPGQ<br>APRLLIY | G--------<br>VSTRAT | GIPARFSGSGSG--<br>TEFTLTISSLQSEDFAVYYC | QQYNN-----------<br>------------WPFT | FGPGT<br>KVDIK |
| iPS:437<br>228 | 21-<br>225_60C11 | VK3\|L2/J<br>K3 | | EIVMTQSPATLSV<br>SPGERATLSC | RAS--QSVS--<br>----NDLA | WYQQKPGQ<br>APRLLIY | G--------<br>ASTRAT | GIPARFSGGGSG--<br>TEFTLTISALQSEHFAVYYC | QQYSN-----------<br>------------WPFT | FGPGT<br>KVDIK |
| | | Germline | | **K_FR1** | **K_CDR1** | **K_FR2** | **K_CDR2** | **K_FR3** | **K_CDR3** | **K_FR4** |
| | VK2\|A19/JK<br>5 | | 32160 | DIVMTQSPLSLPV<br>TPGEPASISC | RSS--<br>QSLLHSN-<br>GYNYLD | WYLQKPGQ<br>SPQLLIY | L--------<br>GSNRAS | GVPDRFSGSGSG--<br>TDFTLKISRVEAEDVGVYYC | MQALQ-----------<br>------------TPIT | FGQGT<br>RLEIK |
| iPS:434<br>243 | 21-<br>225_62C1 | VK2\|A19/<br>JK5 | | DIVMTQSPLSLPV<br>TPGEPASISC | RSS--<br>QSLLHSN-<br>GYNYLD | WYLQKPGQ<br>SPQLLIY | L--------<br>VSNRAS | GVPDRFSGSGSG--<br>TDFTLKISRVGAEDVGVYFC | LQALQ-----------<br>------------TPLT | FGQGT<br>RLEIK |
| iPS:436<br>648 | 21-<br>225_227F11 | VK2\|A19/<br>JK5 | | DVVMTQSPLSLPV<br>TPGEPASISC | WSS--<br>QSLLHSN-<br>GYNYLD | WYLQKPGQ<br>SPQVLIY | L--------<br>GSNRAS | GVPDRFSGSGSG--<br>TDFTLKISRVEAEDVGVYYC | MQALQ-----------<br>------------TPLT | FGQGT<br>RLEIK |
| iPS:394<br>061 | 21-<br>225_12D2 | VK2\|A19/<br>JK5 | | DIVMTQSPLSLPV<br>TPGEPASISC | RSS--<br>QSLLHSN-<br>GYNYLD | WYLQKPGQ<br>SPQVLIY | L--------<br>GSNRAS | GVPDRFSGSGSG--<br>TDFTLKISRVEAEDVGVYYC | MQALQ-----------<br>------------TPIT | FGQGT<br>RLEIK |
| iPS:394<br>071 | 21-<br>225_10C7 | VK2\|A19/<br>JK5 | | DIVMTQSPLSLPV<br>TPGEPASISC | RSS--<br>QSLLHSK-<br>GYNYLD | WYLQKPGQ<br>SPQVLIY | L--------<br>GSNRAS | GVPDRFSGSGSG--<br>TDFTLKISRVEAEDVGVYYC | MQALQ-----------<br>------------TPLT | FGQGT<br>RLEIK |
| | | Germline | | **K_FR1** | **K_CDR1** | **K_FR2** | **K_CDR2** | **K_FR3** | **K_CDR3** | **K_FR4** |
| | VK1\|L5/JK4 | | 32161 | DIQMTQSPSSVSA<br>SVGDRVTITC | RAS--QGIS--<br>----SWLA | WYQQKPGK<br>APKLLIY | A--------<br>ASSLQS | GVPSRFSGSGSG--<br>TDFTLTISSLQPEDFATYYC | QQANS-----------<br>------------FPLT | FGGGT<br>KVEIK |
| iPS:434<br>259 | 21-<br>225_62G7 | VK1\|L5/J<br>K4 | | DIQMTQSPSSVSA<br>SVGDRVTFTC | RAS--QDIS--<br>----SWLA | WYQQNPGK<br>APKLLIY | A--------<br>ASSLQS | GVPSRFSGSGSG--<br>TNFTLTISSLQPEDFATYYC | QQTNS-----------<br>------------FPLT | FGGGT<br>KVEIK |
| iPS:434<br>333 | 21-<br>225_63C9 | VK1\|L5/J<br>K4 | | DIQMTQSPSSVSA<br>SVGDRVTITC | RAS--QGIS--<br>----SWLA | WYQQKPGK<br>APNLLIY | A--------<br>ASSLQS | GVPSRFSGSGSG--<br>TDFTLTISGLQPEDFATYFC | QQINS-----------<br>------------FPLT | FGGGT<br>KVAIK |
| iPS:434<br>347 | 21-<br>225_64H10 | VK1\|L5/J<br>K4 | | DIQMTQSPSSVSA<br>SVGDRVTITC | RAS--QGIS--<br>----SWLA | WYQQKPGK<br>ALKLLIY | A--------<br>ASSLQS | GVPSRFSGSGSG--<br>TDFTLTISSLQPEDFATYYC | QQINS-----------<br>------------FPLT | FGGGT<br>KVEIK |
| iPS:434<br>359 | 21-<br>225_65G3 | VK1\|L5/J<br>K4 | | DIQMTQSPSSVSA<br>SVGDRVTITC | RAS--QGIS--<br>----SWLA | WYQQKPGK<br>APKLLIY | A--------<br>ASSLQS | GVPSRFSGSGSG--<br>TDFTLTISSLQPEDFATFYC | QQVNS-----------<br>------------FPLT | FGGGT<br>KVEIK |
| iPS:434<br>369 | 21-<br>225_66B1 | VK1\|L5/J<br>K4 | | DIQMTQSPSSVSA<br>SVGDRVTITC | RAS--QGIS--<br>----SWLA | WYQQKPGK<br>ALKLLIY | A--------<br>ASSLQS | GVPSRFSGSGSG--<br>TDFTLTISSLQPEDFATYYC | QQTNS-----------<br>------------FPLT | FGGGT<br>KVEIK |
| iPS:434<br>373 | 21-<br>225_66A7 | VK1\|L5/J<br>K4 | | DIQMTQSPSSVSA<br>SVGDRVTITC | RAS--QGIS--<br>----SWLA | WYQQKPGK<br>APKLLIY | A--------<br>ASSLQS | GVPSRFSGSGSG--<br>TDFTLTISSLQPEDFATYYC | QQINS-----------<br>------------FPLT | FGGGT<br>KVEIK |
| iPS:434<br>397 | 21-<br>225_67H4 | VK1\|L5/J<br>K4 | | DIQMTQSPSSVSA<br>SVGDRVTITC | RAS--QGIS--<br>----SWLA | WYQQKPGK<br>APKLLIY | A--------<br>ASSLQS | GVPSRFSGSGSG--<br>TDFTLTISSLQPEDFATYYC | QQINS-----------<br>------------FPLT | FGGGT<br>KVEIK |
| iPS:434<br>427 | 21-<br>225_70D6 | VK1\|L5/J<br>K4 | | DIQMTQSPSSVSA<br>SVGDRVTITC | RAS--QGIS--<br>----KWLA | WYQQNPGK<br>ALKLLIF | A--------<br>ASSLQS | GVPSRFSGSGSG--<br>TDFTLTISSLQPEDFANYYC | QQTNS-----------<br>------------FPLT | FGGGT<br>KVEIK |
| iPS:434 | 21- | VK1\|L5/J | | DIQMTQSPSSVSA | RAS--QDIS-- | WYQQNPGK | A-------- | GVPSRFSGSGSG-- | QQTNS----------- | FGGGT |

| | | | | | | | | | | |
|---|---|---|---|---|---|---|---|---|---|---|
| iPS:434 437 | 21-225_70A12 | VK1\|L5/JK4 | | DIQMTQSPSSVSA SVGDRVTITC | RAS--QGIS-- ----SWLA | WYQQKPGK ALKLLIY | A-------- ASSLQS | GVPSRFSGSGSG-- TDFTLTISSVQPEDFATYYC | QQINS----------- ------------FPLT | FGGGT KVEIK |
| iPS:434 451 | 21-225_71B7 | VK1\|L5/JK4 | | DIQMTQSPSSVSA SVGDRVTITC | RAS--QGIS-- ----SWLA | WYQQNPGE APKLLIY | A-------- ASSLQG | GVPSRFSGSGSG-- TDFTLTISSLQPEDFANYYC | QQTNS----------- ------------FPLT | FGGGT KVEIK |
| iPS:434 459 | 21-225_71A7 | VK1\|L5/JK4 | | DIQMTQSPSSVSA SVGDRVTITC | RAS--QGIS-- ----SWLA | WYQQKPGK APKLLIY | A-------- ASSLQS | GVPSRFSGSGSG-- TDFTLTISSLQPEDFATYYC | QQVNS----------- ------------FPLT | FGGGT KVELK |
| iPS:434 461 | 21-225_73A3 | VK1\|L5/JK4 | | DIQMTQSPSSVSA SIGDRVTITC | RAS--QGIS-- ----NWLA | WYQQKPGK APKLLIY | A-------- ASSLQS | GVPSRFSGSGSE-- TDFTLTISSLQPEDFATYYC | QQVNS----------- ------------FPLT | FGGGT KVEIK |
| iPS:435 479 | 21-225_154E9 | VK1\|L5/JK4 | | DIQMTLSPSSVYA SVGDRVTITC | RAS--QDIS-- ----NWLA | WYQQRPGK APKVLIY | A-------- ASSLQS | GVPSRFSGSGSG-- TDFTLTISSVQPEDFATYYC | QQGNS----------- ------------FPLT | FGGGT KVDIK |
| iPS:437 232 | 21-225_63E1 | VK1\|L5/JK4 | | DIQMTQSPSSVYA SVGDRVTITC | RAS--QGIS-- ----SYLA | WYQQKPGK APKLLIY | A-------- ASSLQS | GVPSRFSGSGSG-- TDFTLTISSLQPEDFATYYC | QQANS----------- ------------FPLT | FGGGT KVEIK |
| iPS:437 326 | 21-225_75C10 | VK1\|L5/JK4 | | DIQMTQSPSSVSA SVGDRVIITC | RAS--QGIS-- ----IWLA | WYQQKPGK APKLLIY | A-------- ASSLQS | GVPLRFSGSGSG-- TDFTLTISSLQPEDFATYYC | QQAKS----------- ------------FPLT | FGGGT KVEIK |
| | | Germline | | K_FR1 | K_CDR1 | K_FR2 | K_CDR2 | K_FR3 | K_CDR3 | K_FR4 |
| | VK1\|O12/JK2 | | 32162 | DIQMTQSPSSLSA SVGDRVTITC | RAS--QSIS-- ----SYLN | WYQQKPGK APKLLIY | A-------- ASSLQS | GVPSRFSGSGSG-- TDFTLTISSLQPEDFATYYC | QQSYS----------- ------------TPYT | FGQGT KLEIK |
| iPS:434 309 | 21-225_59B5 | VK1\|O12/JK2 | | DIQMTQSPSSLSA SVGDRVTITC | RAS--QSII-- ----SYLN | WYQQKPGK APKLLIY | G-------- ASSLQS | GVPSRFSGSGSG-- TDFTLTISSLQPEDFATYYC | QQSYS----------- -----------TPMFS | FGQGT KLEIK |
| iPS:392 874 | 21-225_21D2 | VK1\|O12/JK2 | | DIQMTQSPSSLFA SVGDRVTITC | RAS--QSIS-- ----DYLN | WYQQKPGR APKLLIY | D-------- TSSLQS | GVPSRFSGSGSG-- TDFTLTINSLQPEDFATYYC | QQTYNI---------- -----------LPERS | FGRGT KLEIK |
| iPS:393 940 | 21-225_16B2 | VK1\|O12/JK2 | | GVQMTQSPSSLSA SVGDRVTITC | RAS--QSIS-- ----GYLN | WYQQKPGK APKLLIY | A-------- ASSLQS | GVPSRFSGGGSG-- TDFTLTISSLQPEDFATYYC | QQTYNT---------- -----------PPERS | FGQGT KLEIK |
| iPS:393 956 | 21-225_4D7 | VK1\|O12/JK2 | | DIQMTQSPSSLSA SVGDRVTITC | RAS--QSIS-- ----DYLN | WYQQKPGK APKLLIF | D-------- TTSLQS | GVPSRFSGSGSG-- TDFTLTINSLQPEDFATYYC | QQTYNT---------- -----------PPERS | FGQGT KLEIK |
| iPS:398 476 | 21-225_17C1 | VK1\|O12/JK2 | | DIQMTQSPSSLSA SVGDRVTITC | RAS--QNIN-- ----DYLN | WYQQKPGK APKLLIY | A-------- ASNLQS | GVPARFSGSRSG-- TDFTLTISSLQPEDFATYYC | QQTYNT---------- -----------PPERS | FGQGT KLEIK |
| iPS:403 870 | 21-225_23G4 | VK1\|O12/JK2 | | DIQMTQSPSSLSA SVGDRVTITC | RAS--QNIY-- ----SYLN | WYQQKPGK APKLLIY | A-------- ASSLQS | GVPSRFSGSGSG-- TEFTLTISILQPEDFATYYC | QQSYNT---------- -----------PPECN | FGQGT KLEIK |
| | | Germline | | K_FR1 | K_CDR1 | K_FR2 | K_CDR2 | K_FR3 | K_CDR3 | K_FR4 |
| | VK3\|A27/JK3 | | 32163 | EIVLTQSPGTLSL SPGERATLSC | RAS--QSVSS- ----SYLA | WYQQKPGQ APRLLIY | G-------- ASSRAT | GIPDRFSGSGSG-- TDFTLTISRLEPEDFAVYYC | QQYGS----------- ------------SPFT | FGPGT KVDIK |
| iPS:434 311 | 21-225_59H5 | VK3\|A27/JK3 | | EIVLTQSPGTLSL SPGERATLSC | RAS--QSVSS- ----IYLA | WFLQKPGQ APRLLIY | G-------- ASSRAT | GIPDRFSGSGSG-- TDFTLTISRLEPEDFAVYYC | HQYGN----------- ------------SPFT | FGPGT KVDFK |
| iPS:435 301 | 21-225_146G4 | VK3\|A27/JK3 | | EIVLTQSPGTLSL FPGERATLSC | RAS--QNIIS- ----SYLA | WYQQKPGQ APRLLIF | G-------- VSSRAT | GIPDRFSGSGSG-- TDFTLTISRLEPEDFAVYYC | QQYGR----------- ------------SPFN | FGPGT KVDIN |
| iPS:435 317 | 21-225_147D2 | VK3\|A27/JK3 | | EIVLTQSPGTLSL SPGERATLSC | RAS--QSVGS- ----SYLA | WYQQKPGQ APRLLIY | G-------- ASSRAT | GIPDRFSGSGSG-- TDFTLTISRLEPEDFAVYYC | QQYG------------ ------------SLFT | FGPGT KVDIK |
| iPS:435 319 | 21-225_147E3 | VK3\|A27/JK3 | | EIVLTQSPGTLSL SPGERATLSC | RAS--QSVIS- ----SYLA | WYQQKPGQ APRLLIY | G-------- ASSRAT | AIPDRFSGSGSG-- TDFTLTISRLEPEDFAVYYC | QQYGR----------- ------------SPFN | FGPGT KVDIK |
| iPS:435 383 | 21-225_149D7 | VK3\|A27/JK3 | | EIVLTQSPGTLSL FPGERVTLSC | RAS--QSIIS- ----NYLA | WYQQKPGQ APRLLIF | G-------- VSSRAT | GIPDRFSGSGSG-- TDFTLTISRLEPEDFAVYYC | QQYGR----------- ------------SPFN | FGPGT KVDIK |
| iPS:435 443 | 21-225_152E7 | VK3\|A27/JK3 | | EIVLTQSPGTLSL FPGERATLSC | RAS--QSVIS- ----SYLA | WYQQKPGQ APRLLIF | G-------- VSSRAT | GIPDRFSGSGSG-- TDFTLTISRLEPEDFAVYYC | QQYGR----------- ------------SPFN | FGPGT KVDIK |
| iPS:435 465 | 21-225_153A6 | VK3\|A27/JK3 | | EIVLTQSPGTLSL FPGERAPLSC | RAS--QSVIS- ----SYLA | WYQQKPGQ APRLLIF | G-------- VSSRAT | GIPDRFSGSGSG-- TDFTLTISRLEPEDFAVYYC | QQYGR----------- ------------SPFN | FGPGT KVDIK |

| | | | | | | | | | | |
|---|---|---|---|---|---|---|---|---|---|---|
| **iPS:436 058** | **21-225_194A4** | **VK3\|A27/ JK3** | | EIVLTQSPGTLSL<br>SPGERATLSC | RAS--RGVSN-<br>----IYLA | WYQQKPGQ<br>APRLLIY | G--------<br>ASNRAT | GIPDRFSGSGSG--<br>TDFTLTISRLEPEDFAVYYC | QHNDY-----------<br>------------SMFT | FGPGT<br>KVDIK |
| **iPS:436 436** | **21-225_216F10** | **VK3\|A27/ JK3** | | EVVLTQSPGTLSL<br>SPGERATLSC | RAS--QSVSS-<br>----SFLA | WYQQKPGQ<br>APRLLIY | G--------<br>TSTRAT | GIPDRFSGSGSG--<br>TDFILTISRLEPEDFAVYYC | QQYDR-----------<br>------------SPFT | FGPGT<br>KVDIK |
| **iPS:442 568** | **21-225_149D8** | **VK3\|A27/ JK3** | | EIVLTQSPGTLSL<br>FPGERATLSC | RAS--QSVIS-<br>----SYLA | WYQQKPGQ<br>APRLLIF | G--------<br>VSSWAT | GIPDRFSGSGSG--<br>TDFTLTISRLEPEDFAVYYC | QQYGR-----------<br>------------SPFN | FGPGT<br>KVDIK |
| | | **Germline** | | **K_FR1** | **K_CDR1** | **K_FR2** | **K_CDR2** | **K_FR3** | **K_CDR3** | **K_FR4** |
| | **VK1\|O18/J K1** | | 32164 | DIQMTQSPSSLSA<br>SVGDRVTITC | QAS--QDIS--<br>----NYLN | WYQQKPGK<br>APKLLIY | D--------<br>ASNLET | GVPSRFSGSGSG--<br>TDFTFTISSLQPEDIATYYC | QQYDN-----------<br>------------LPWT | FGQGT<br>KVEIK |
| **iPS:434 353** | **21-225_64B12** | **VK1\|O18/ JK1** | | DIQMTQSPSSLSA<br>SVGDRVTITC | RAS--QDIS--<br>----NYLN | WYQQKPGT<br>APNLLIS | D--------<br>ASILET | GVPSTFSGSGSG--<br>TDFTFTISSLQPEDIATYYC | QQSDN-----------<br>------------LPCS | FGQGT<br>KVEIK |
| | | **Germline** | | **K_FR1** | **K_CDR1** | **K_FR2** | **K_CDR2** | **K_FR3** | **K_CDR3** | **K_FR4** |
| | **VK1\|A20/JK 4** | | 32165 | DIQMTQSPSSLSA<br>SVGDRVTITC | RAS--QGIS--<br>----NYLA | WYQQKPGK<br>VPKLLIY | A--------<br>ASTLQS | GVPSRFSGSGSG--<br>TDFTLTISSLQPEDVATYYC | QKYNS-----------<br>------------APLT | FGGGT<br>KVEIK |
| **iPS:434 357** | **21-225_65C1** | **VK1\|A20/ JK4** | | DIQLTQSPSSLSA<br>SVGDRVTITC | RAS--QVIS--<br>----SYLH | WYQQKPGK<br>VPKVLIY | S--------<br>ASNLQC | GVPSRFSGSGSG--<br>TDFTLTFSSLQPEDVATYYG | QRPYN-----------<br>------------APLT | FGGGT<br>KVEIK |
| **iPS:434 375** | **21-225_66C7** | **VK1\|A20/ JK4** | | DIQLTQSPSSLSA<br>SVGDRVTITR | RAS--QGIS--<br>----NYLH | WYQQKPGK<br>APKLLIY | C--------<br>ASNLQC | GVPSRFSGSGSG--<br>TDFTLTISSLQPEDVATYYC | QQHNN-----------<br>------------SPLT | FGGGT<br>KVEIK |
| **iPS:434 457** | **21-225_72G12** | **VK1\|A20/ JK4** | | DIQLTQSPSSLSA<br>SVGDRVTITC | RAS--QGIS--<br>----SYLN | WSQQKPGK<br>VPKLLIC | G--------<br>ASNLQS | GVPSRFSGSASG--<br>TEFILTISSLQPEDVTTYYG | QQNYN-----------<br>------------APLT | FGGGT<br>KVEIK |
| | | **Germline** | | **K_FR1** | **K_CDR1** | **K_FR2** | **K_CDR2** | **K_FR3** | **K_CDR3** | **K_FR4** |
| | **VK3\|A27/JK 4** | | 32166 | EIVLTQSPGTLSL<br>SPGERATLSC | RAS--QSVSS-<br>----SYLA | WYQQKPGQ<br>APRLLIY | G--------<br>ASSRAT | GIPDRFSGSGSG--<br>TDFTLTISRLEPEDFAVYYC | QQYGS-----------<br>------------SPLT | FGGGT<br>KVEIK |
| **iPS:434 479** | **21-225_76H1** | **VK3\|A27/ JK4** | | EFMLTQSPGTLYW<br>SPGERATLSC | RAS--QSVSS-<br>----SYLV | WYQQKPGQ<br>APRLLIY | G--------<br>ASTRAT | GIPDRFSGSGSG--<br>TDFTLTISRLEPEYFAVYYC | QQYGC-----------<br>------------SPLT | FGGGT<br>KVEIT |
| **iPS:434 513** | **21-225_76A6** | **VK3\|A27/ JK4** | | EIVLTQSPGTRSW<br>SPGERATLSC | RAS--QSVSS-<br>----SYLA | WYQQKPGQ<br>APRLLIY | G--------<br>ASTRAT | GIPDRFSGSGSG--<br>TDFTLTISRLEPEYFAVYYC | QQYGN-----------<br>------------SPLT | FGGGT<br>KVEIK |
| **iPS:434 515** | **21-225_74A5** | **VK3\|A27/ JK4** | | EFMLTQSPGTLSL<br>SPGERATLSC | RAS--QSVSS-<br>----SYLV | WYQQKPGQ<br>APRLLIY | G--------<br>ASTRAT | GIPDRFSGSGSG--<br>TDFTLTISRLEPEYFAVYYC | QQYGC-----------<br>------------SPLT | FGGGT<br>KVEIT |
| **iPS:434 529** | **21-225_76B9** | **VK3\|A27/ JK4** | | EFMLTQSPGTLSW<br>SPGERATLSC | RAS--QSVSS-<br>----SYLV | WYQQKPGQ<br>APRLLIY | G--------<br>ASTRAT | GIPDRFSGSGSG--<br>TDFTLTISRLEPEYFAVYYC | QQYGC-----------<br>------------SPLT | FGGGT<br>KVEIT |
| **iPS:434 583** | **21-225_74B6** | **VK3\|A27/ JK4** | | EIVLTQSPGTLSL<br>SPGERATLSC | RAS--QSVSS-<br>----SYLA | WYQQKPGQ<br>APRLLIY | G--------<br>ASTRAT | GIPDRFSGSGSG--<br>TDFTLTISRLEPEYFAVYYC | QQYGN-----------<br>------------SPLT | FGGGT<br>KVEIK |
| **iPS:434 587** | **21-225_74G3** | **VK3\|A27/ JK4** | | EFMLTQSPGTLCW<br>SPGERATLSC | RAS--QSVSS-<br>----SYLV | WYQQKPGQ<br>APRLLIY | G--------<br>ASTRAT | GIPDRFSGSGSG--<br>TDFTLTISRLEPEYFAVYYC | QQYGC-----------<br>------------SPLT | FGGGT<br>KVEIT |
| **iPS:434 603** | **21-225_77D11** | **VK3\|A27/ JK4** | | EFMLTQSPGTLYW<br>SPGERATISC | RAS--QSVSS-<br>----SYLV | WYQQKPGQ<br>APRLLIY | G--------<br>ASTRAT | GIPDRFSGSGSG--<br>TDFTLTISRLEPEYFAVYYC | QQYGC-----------<br>------------SPLT | FGGGT<br>KVEIT |
| **iPS:434 705** | **21-225_80A2** | **VK3\|A27/ JK4** | | EFMLTQSPGTLYL<br>SPGERATLSC | RAS--QSVSS-<br>----SYLV | WYQQKPGQ<br>APRLLIY | G--------<br>ASTRAT | GIPDRFSGSGSG--<br>TDFTLTISRLEPEYFAVYYC | QQYGC-----------<br>------------SPLT | FGGGT<br>KVEIT |
| **iPS:434 747** | **21-225_80C12** | **VK3\|A27/ JK4** | | EIVLTQSPGTLYL<br>SPGERATLSC | RAS--QSVSS-<br>----SYLA | WYQQKPGQ<br>APRLLIY | G--------<br>ASTRAT | GIPDRFSGSGSG--<br>TDFTLTISRLEPEYFAVYYC | QQYGN-----------<br>------------SPLT | FGGGT<br>KVEIK |
| **iPS:434 793** | **21-225_82A5** | **VK3\|A27/ JK4** | | EIVLTQSPGTLSW<br>SPGERATLSC | RAS--QSVSS-<br>----SYLA | WYQQKPGQ<br>APRLLIY | G--------<br>ASTRAT | GIPDRFSGSGSG--<br>TDFTLTISRLEPEYFAVYYC | QQYGN-----------<br>------------SPLT | FGGGT<br>KVEIK |
| **iPS:434 797** | **21-225_82G5** | **VK3\|A27/ JK4** | | EFMLTQSPGTLYW<br>SPGERATLSS | RAS--ESVSS-<br>----SYLV | WYQQKPGQ<br>APRLLIY | G--------<br>ASTRAT | GIPDRFSGSGSG--<br>TDFTLTISRLEPEYFAVYYC | QQYGC-----------<br>------------SPLT | FGGGT<br>KVEIT |
| **iPS:434** | **21-** | **VK3\|A27/** | | EFMLTQSPGTLSW | RAS--ESVSS- | WYQQKPGQ | G-------- | GIPDRFSGSGSG-- | QQYGC----------- | FGGGT |

| | | | | | | | | | | |
|---|---|---|---|---|---|---|---|---|---|---|
| iPS:434 813 | 21-225_82C12 | VK3\|A27/JK4 | | EIVLTQSPGTLSW SPGERATLSC | RAS--QSVSS- ----SYLA | WYQQKPGQ APRLLIY | G-------- ASTRAS | GIPDRFSGSGSG-- TDFTLTISRLEPEYFAVYYC | QQYGN----------- ------------SPLT | FGGGT KVEIK |
| iPS:434 825 | 21-225_83C2 | VK3\|A27/JK4 | | EFMLTQSPGTLCL SPGERATLSC | RAS--QSVSS- ----SYLV | WYQQKPGQ APRLLIY | G-------- ASTRAT | GIPDRFSGSGSG-- TDFTLTISRLEPEYFAVYYC | QQYGC----------- ------------SPLT | FGGGT KVEIT |
| iPS:434 833 | 21-225_83C5 | VK3\|A27/JK4 | | EFMLTQSPGTLCW SPGERATLSC | RAS--ESVSS- ----SYLV | WYQQKPGQ APRLLIY | G-------- ASTRAT | GIPDRFSGSGSG-- TDFTLTISRLEPEYFAVYYC | QQYGC----------- ------------SPLT | FGGGT KVEIT |
| iPS:434 883 | 21-225_85B5 | VK3\|A27/JK4 | | EFMLTQSPGTLSL SPGERATLSC | RSS--QSVSS- ----SYLV | WYQQKPGQ APRLLIY | G-------- ASTRAT | GIPDRFSGSGSG-- TDFTLTISRLEPEYFAVYYC | QQYGC----------- ------------SPLT | FGGGT KVEIT |
| iPS:434 911 | 21-225_85D11 | VK3\|A27/JK4 | | EFMLTQSPGTLSL STGERATLSC | RSS--QSVSS- ----SYLV | WYQQKPGQ APRLLIY | G-------- ASTRAT | GIPDRFSGSGSG-- TDFTLTISRLEPEYFAVYYC | QQYGC----------- ------------SPLT | FGGGT KVEIT |
| iPS:434 957 | 21-225_87A10 | VK3\|A27/JK4 | | EFVLTQSPGTLYL SPGERATLSC | RAS--QSVSS- ----SYLA | WYQQKPGQ APRLLIY | G-------- ASTRAS | GIPDRFSGSGSG-- TDFTLTISRLEPEYFAVYYC | QQYGN----------- ------------SPLT | FGGGT KVEIK |
| iPS:435 247 | 21-225_96G1 | VK3\|A27/JK4 | | EIVLTQSPGTLSL STGERATLSC | RAS--QSVSS- ----SYLA | WYQQKPGQ PPRLLIY | G-------- ASTRAS | GIPDRFSGSGSG-- TDFTLTISRLEPEYFAVYYC | QQYGN----------- ------------SPLT | FGGGT KVEIK |
| iPS:436 368 | 21-225_211G3 | VK3\|A27/JK4 | | EIVLTQSPGTLSL SPGERATLSC | RAS--QSVSS- ----SFLA | WYQQKPGQ APRLLIY | G-------- ASSRAT | GIPDRFSGSGSG-- TDFTLTISRLEPEDFAVYYC | QQYVS----------- ------------SPLT | FGGGT KVEIK |
| iPS:436 426 | 21-225_215C7 | VK3\|A27/JK4 | | EIVLTQSPGTLSL SPGERVTLSC | RAS--QRITT- ----NFLA | WYQQKPGQ APRLLIY | G-------- ASSRAT | GIPDRFSGSGSG-- TDFTLTISRLESEDFAVYYC | QQYVS----------- ------------SLLT | FGGGT KVEIK |
| iPS:436 432 | 21-225_215H12 | VK3\|A27/JK4 | | EIVLTQSPGTLSL SPGERATLSC | RAS--QSVSS- ----SFLA | WYQQKPGQ APRLLMY | G-------- ASSRAI | GIPDRFSGSGSG-- TDFTLTISRLEPEDFAVYYC | QQYVS----------- ------------SPLT | FGGGT KVEIK |
| iPS:437 322 | 21-225_75B1 | VK3\|A27/JK4 | | EFMLTQSPGTLYW SPGERATISS | RAS--QSVSS- ----SYLV | WYQQKPGQ APRLLIY | G-------- ASTRAT | GIPDRFSGSGSG-- TDFTLTISRLEPEYFAVYYC | QQYGC----------- ------------SPLT | FGGGT KVEIT |
| iPS:437 377 | 21-225_74G9 | VK3\|A27/JK4 | | EFMLTQSPGTLSL SPGERATLSC | RAS--QSVSS- ----SYLV | WYQQKPGQ APRLLIY | G-------- ASTRAT | GIPDRFSGSGSG-- TDFTLTISRLEPEYFAVYYC | QQYGC----------- ------------SPLT | FGGGT KVEIT |
| | | Germline | | **K_FR1** | **K_CDR1** | **K_FR2** | **K_CDR2** | **K_FR3** | **K_CDR3** | **K_FR4** |
| | VK4\|B3/JK4 | | 32167 | DIVMTQSPDSLAV SLGERATINC | KSS-- QSVLYSSNNKN YLA | WYQQKPGQ PPKLLIY | W-------- ASTRES | GVPDRFSGSGSG-- TDFTLTISSLQAEDVAVYYC | QQYYS----------- ------------TPLT | FGGGT KVEIK |
| iPS:434 511 | 21-225_74B11 | VK4\|B3/JK4 | | DIVMTQSPDSLTV SLGERATINC | KSS-- QSILYNSNNNN YLA | WYQQKPGQ PPKLLIY | W-------- ASTRES | GVPDRFSGSGSG-- TDFILTISSLQAEDVAVYYC | QQYYS----------- ------------TPPT | FGGGT KVEIK |
| iPS:434 729 | 21-225_80B12 | VK4\|B3/JK4 | | DIVLTQSPDSLAV SLGERATINC | KSR-- QSVLYSSNNYN YLT | WYQQKPGQ PPKLLIY | W-------- ASTRES | GVPDRFSGSGSG-- TDFTLTISSLQAEDVAVYYC | QQYYS----------- ------------SPPT | FGGGT KVEIK |
| iPS:434 851 | 21-225_75A6 | VK4\|B3/JK4 | | DIVMTQSPDSLAV SLGERATINC | KSR-- QSVLHSSNNYN YLA | WYQQKPGQ PPELLIY | W-------- ASTRES | GVPDRFSGSGSG-- TDFTLTISSLQAEDVAVYYC | QQYYS----------- ------------TPPT | FGGGT KVEIK |
| iPS:435 623 | 21-225_162D5 | VK4\|B3/JK4 | | DIVMTQSPDFRNV SMGERAIINF | KSN-- HSVLYRSNNNQ YLA | WYQRKPGQ PPKLLIY | R-------- TSIRKS | GVPDRFSGSGCG-- TDFTLTIDSLQAEDVAVYYC | QQYYS----------- ------------TPPT | FGGGT KVEIK |
| iPS:446 086 | 21-225_94D8 | VK4\|B3/JK4 | | DIVLTQSPDSLAV SLGERATINC | KSR-- QSVLYSSNNYN YLT | WYQQKPGQ PPKLLIY | W-------- ASTRES | GVPDRFSGSGSG-- TDFTLTISSLQAEDVAVYYC | QQYYS----------- ------------SPPT | FGGGT KVEIK |
| | | Germline | | **K_FR1** | **K_CDR1** | **K_FR2** | **K_CDR2** | **K_FR3** | **K_CDR3** | **K_FR4** |

| | | | | | | | | | | |
|---|---|---|---|---|---|---|---|---|---|---|
| | VK2\|A19/JK 4 | | 32168 | DIVMTQSPLSLPV TPGEPASISC | RSS-- QSLLHSN- GYNYLD | WYLQKPGQ SPQLLIY | L-------- GSNRAS | GVPDRFSGSGSG-- TDFTLKISRVEAEDVGVYYC | MQALQ----------- ------------TPLT | FGGGT KVEIK |
| iPS:434 539 | 21- 225_74A2 | VK2\|A19/ JK4 | | DIVMTQSPLSLPV TPGEPASISC | RSS-- QSLLHSN- GHNYLD | WYLQKPGR SPQLLIY | L-------- GSNRAS | GVPERFSGSGSG-- TDFTLKISRVEAEDVGVYYC | MQPLQ----------- ------------TPFT | FGGGT KVEIK |
| iPS:434 563 | 21- 225_75D8 | VK2\|A19/ JK4 | | DIVMTQSPLSLPV TPGEPASISC | RSS-- QSLLHSS- GYNYLD | WYLQKPGQ SPQLLIY | L-------- GSNRAS | GVPDRFSGSGSG-- SDFTLKISRVEAEDVGLYYC | MQALH----------- ------------PPLT | FGGGT KVEIK |
| iPS:435 009 | 21- 225_89G4 | VK2\|A19/ JK4 | | DIVMTQSPLSLPV TPGEPASISC | RSS-- QSLLHSS- GYNYLD | WYLQKPGQ SPQLLIY | L-------- GSNRAS | GVPDRFSGSGSG-- TDFTLKISRVEAEDIGVYYC | MQALH----------- ------------IPLT | FGGGT KVEIK |
| iPS:435 059 | 21- 225_90C11 | VK2\|A19/ JK4 | | DIVMTQSPLSLPV TPGEPASISC | RYS-- QSLVHSS- GYNYLD | WYLQKPGQ SPQLVIY | L-------- GSNRAS | GVPDRFSGSGSG-- SDFTLKISRVEAEDVGLYYC | MQALH----------- ------------PPLT | FGGGT KVEIK |
| iPS:435 103 | 21- 225_92B2 | VK2\|A19/ JK4 | | DIVMTQSPLSLPV TPGEPASISC | RSS-- QSLVHSS- GYNYLD | WYLQKPGQ SPQLVIY | L-------- GSNRAS | GVPDRFSGSGSV-- TDFTLRISRVEAEDIGIYYC | MQALH----------- ------------IPLT | FGGGT KVEIK |
| iPS:435 713 | 21- 225_171D7 | VK2\|A19/ JK4 | | DIVMTQSPLSLPV TPGEPASISC | RSS-- QSLLYHN- GYNYLD | WYLQKTGQ SPQLLIY | V-------- GSNRAS | GVPDRFSGSGSG-- TDFTLKISRVEAEDVGVYYC | MQTLQ----------- ------------TPLT | FGGGT KVEIK |
| iPS:436 246 | 21- 225_201G6 | VK2\|A19/ JK4 | | DIVMTQSPLSLPV TPGEPASISC | RSS-- QSLLHNN- RYNHLD | WYLQKPGQ SPQLLIY | L-------- GSNRAS | GVPDRFSGSGSG-- TDFTLKISRVEAEDVGVYYC | MQAL------------ ------------QTPT | FGGGT KVEIK |
| iPS:436 254 | 21- 225_202C1 2 | VK2\|A19/ JK4 | | DIVLTQSPLSLPV TPGEPASISC | RSS-- QSLLHNN- KYNHLD | WYLQKPGQ SPQLLIY | L-------- GSNRAS | GVPDRFSGSGSG-- TDFTLKISRVEAEDVGVYYC | MQAL------------ ------------QTPT | FGGGT KVEIK |
| iPS:436 304 | 21- 225_201F3 | VK2\|A19/ JK4 | | DIVMTQSPLSLPV TPGEPASISC | RSS-- QSLLHNN- RYNHLD | WYLQKPGQ SPQLLIY | L-------- GSNRAS | GVPDRFSGSGSG-- TDFTLKISRVEAEDVGVYYC | MQAL------------ ------------QTPT | FGGGT KVEIK |
| iPS:436 334 | 21- 225_208G3 | VK2\|A19/ JK4 | | DIVLTQSPLSLPV TPGEPASISC | RSS-- QSLLHNN- KYNHLD | WYLQKPGQ SPQLLIY | L-------- GSNRAS | GVPDRFSGSGSG-- TDFTLKISRVEAEDVGVYYC | MQAL------------ ------------QTPT | FGGGT KVEIK |
| iPS:437 248 | 21- 225_97H3 | VK2\|A19/ JK4 | | DIVMIQSPLSLPV TPGEPASISC | RSS-- QSLLHSN- GHNYLD | WYLQKPGR SPQLLIY | L-------- GSNRAS | GVPERFSGSGSG-- TDFTLKISRVEAEDVGVYYC | MQPLQ----------- ------------TPFT | FGGGT KVEIK |
| iPS:437 320 | 21- 225_75A1 | VK2\|A19/ JK4 | | DIVMTQSPLSLPV TPGEPASISC | RSS-- QSLLHSN- GHNYLD | WYLQKPGR SPQLLIY | L-------- GSNRAS | GVPERFSGSGSG-- TDFTLKISRVEAEDVGVYYC | MQPLQ----------- ------------TPFT | FGGGT KVEIK |
| iPS:437 371 | 21- 225_74D8 | VK2\|A19/ JK4 | | DIVMTQSPLSLPV TPGEPASISC | RSS-- QSLVHSS- GYNYLD | WYLQKPGQ SPQLVIY | L-------- GSNRAS | GVPDRFSGSGSG-- SDFTLKISRVEAEDVGLYYC | MQALH----------- ------------PPLT | FGGGT KVEIK |
| iPS:392 718 | 21- 225_17B8 | VK2\|A19/ JK4 | | DIVMTQSPLSLPV TPGEPASISC | RSS-- QSLLHSN- GNNSLD | WYLQKPGQ SPQLLIY | L-------- GSHRAS | GVPDRFSDSGSG-- TDFTLKISRVEAEDVGVYYC | MQVLQ----------- -----------TPPLT | FGGGT KVEIK |
| | | **Germline** | | **K_FR1** | **K_CDR1** | **K_FR2** | **K_CDR2** | **K_FR3** | **K_CDR3** | **K_FR4** |

| | | | | | | | | | | |
|---|---|---|---|---|---|---|---|---|---|---|
| **iPS:434 871** | **21-225_85H1** | **VK3\|L2/JK1** | | EIVMTQSPATLSV<br>SPGERATLSC | RAS--QDVI--<br>----TYLA | WYQQKPGQ<br>APRLLIY | G--------<br>ASTRAT | GVPARFSGSGSG--<br>TEFTLTISSLQSEDFALYYC | QEYND-----------<br>------------WPCS | FGQGT<br>KVEIK |
| **iPS:435 421** | **21-225_151F1** | **VK3\|L2/JK1** | | EMVMTQSPATLSV<br>SPGERVTLSC | RAS--QSIN--<br>----INIA | WYQQKPGQ<br>APRLLIY | G--------<br>ASTRAT | GIPARFSGSGSG--<br>TEFTLTISSLQSEDFAVYYC | QQYND-----------<br>-----------WPPWT | FGQGT<br>KVEIK |
| **iPS:435 497** | **21-225_155H9** | **VK3\|L2/JK1** | | EIVMTQSPATLSV<br>SPGERATLSC | RAS--QSVS--<br>----SNLA | WYQQKPGQ<br>APRLLIY | G--------<br>ASTRAT | GIPARFSGSGSG--<br>TEFTLTISSLQSEDFAVYYC | QQYDD-----------<br>-----------WPPWT | FGQGT<br>KVEIK |
| **iPS:435 605** | **21-225_161A4** | **VK3\|L2/JK1** | | EIVMTQSPATLSV<br>SPGERASLSC | RSS--QSVN--<br>----SNLA | WYQQRPGQ<br>ALRLLIY | G--------<br>ASIRAT | DIPARFNGSGSG--<br>TEFTLTISSLQSEDFAVYFC | QQYN------------<br>------------NWWT | FGQGT<br>TVEIK |
| **iPS:451 118** | **21-225_191C8** | **VK3\|L2/JK1** | | EIVMTQSPATLSV<br>SPGERATLSC | RAS--QSVR--<br>----SNLA | WYQQEPGQ<br>APRLLIY | G--------<br>ASTRAT | GIPARFSGSGSG--<br>TEFTLTISSLQSEDFAVYYC | QQSFT-----------<br>------------WLRT | FGQGT<br>KVEIK |
| **iPS:392 734** | **21-225_17D8** | **VK3\|L2/JK1** | | EIVMTQSPSTLSV<br>SPGERATLSC | RAS--QSVS--<br>----SNLA | WFQQKPGQ<br>APRLLIN | G--------<br>ASTRAS | GIPARFSGSGSG--<br>TEFTLTISSLQSEDFAVYYC | QQYNN-----------<br>------------WPLT | FGQGT<br>KVEIK |
| **iPS:392 768** | **21-225_20B8** | **VK3\|L2/JK1** | | EIVMTQSPSTLSV<br>SPGERVTLSC | RAS--QSVS--<br>----SNLA | WFQQKPGQ<br>APRLLIN | G--------<br>ASTRAS | GIPARFSGSGSG--<br>TEFTLTISSLQSEDFAVYYC | QQYNN-----------<br>------------CPLT | FGQGT<br>KVEIK |
| **iPS:393 044** | **21-225_25B8** | **VK3\|L2/JK1** | | EIVMTQSPATLSV<br>SPGERATLSC | RAS--QSVR--<br>----SNLA | WYQQKPGQ<br>APRLLIY | G--------<br>ASTRAT | GIPARFSGSGSG--<br>TEFTLTISSLQSEDFALYYC | QQYNN-----------<br>-----------WPPWP | FGQGT<br>KVEIK |
| **iPS:393 050** | **21-225_28C5** | **VK3\|L2/JK1** | | EIVMTQSPATLSV<br>SPGERATLSC | RAS--QSVS--<br>----SNLA | WYIIQKPGQ<br>APRLLIY | G--------<br>ASTRAT | GIPARFSGSGSG--<br>TEFTLTISSLQSEDFALYYC | QQYNN-----------<br>-----------WPPWP | FGQGT<br>KVEIK |
| **iPS:393 906** | **21-225_13D3** | **VK3\|L2/JK1** | | EIVMTQSPATLSV<br>SPGESATLSC | RAS--QTVS--<br>----SNLA | WFQQKPGQ<br>APRLLIN | G--------<br>ASTRAT | GIPARFSGSGSG--<br>TEFTLTISSLQSEDFAVYFC | QQYHD-----------<br>------------WPPT | FGQGT<br>KVEIK |
| | | **Germline** | | **K_FR1** | **K_CDR1** | **K_FR2** | **K_CDR2** | **K_FR3** | **K_CDR3** | **K_FR4** |
| | **VK1\|L1/JK5** | | 32170 | DIQMTQSPSSLSA<br>SVGDRVTITC | RAS--QGIS--<br>----NYLA | WFQQKPGK<br>APKSLIY | A--------<br>ASSLQS | GVPSRFSGSGSG--<br>TDFTLTISSLQPEDFATYYC | QQYNS-----------<br>------------YPIT | FGQGT<br>RLEIK |
| **iPS:434 947** | **21-225_87B7** | **VK1\|L1/JK5** | | DIQMTQSPSSLSA<br>SVGDRVTITC | RAS--QGIS--<br>----NYLA | WFQQKPGK<br>APKSLIY | A--------<br>ASSLHS | GVPSKFSGSGSG--<br>TDFTLTISSLQPEDSATYFC | LLYLT-----------<br>------------YPLT | FGQGT<br>RLEIK |
| **iPS:435 427** | **21-225_151C9** | **VK1\|L1/JK5** | | DIQMTQSPSSLSA<br>SVGDRVTITC | RAS--QGIS--<br>----KYLA | WFQQKPGK<br>APKSLIY | D--------<br>ASRLQS | GVPSKFSGSGSG--<br>TDFTLTISSLQPEDFASYYC | HQYKH-----------<br>------------YPIT | FGQGT<br>RLEIK |
| **iPS:435 529** | **21-225_157H7** | **VK1\|L1/JK5** | | DIQMTQSPSSLSA<br>SVGDRVTITC | RAS--QDIS--<br>----NFLA | WFQQKPGK<br>APKSLVS | T--------<br>ASSLQS | GVPSKFSGSGSG--<br>TDFTLTISSLQPEDFATYYC | QQYHS-----------<br>------------YPIT | FGQGT<br>RLEIK |
| **iPS:436 066** | **21-225_194B7** | **VK1\|L1/JK5** | | DIQMTQSPSSLSA<br>SVGDRVTITC | RAS--QGIS--<br>----KYLA | WFQQKPGK<br>APKSLIY | G--------<br>ASRLQS | GVPSKFSGSGSG--<br>TDFTLTISSLQPEDFATYYC | QHYLN-----------<br>------------YPLT | FGQGT<br>RLEIK |
| **iPS:437 274** | **21-225_196D4** | **VK1\|L1/JK5** | | DIQMTQSPSSLSA<br>SVGDRVTITC | RAS--QGIS--<br>----NYLA | WFQQNPGK<br>APKSLIY | A--------<br>ASSLQS | GVPSKFSGSGCG--<br>TDFTLTISSPQPEDVATYYC | QHYLN-----------<br>------------YPLT | FGQGT<br>RLEIK |
| **iPS:392 748** | **21-225_20A8** | **VK1\|L1/JK5** | | DIQMTQSPSSLSA<br>SVGDRVTITC | RAS--QGIN--<br>----NYLV | WFQQKPGK<br>APKSLIY | A--------<br>ASSLLS | GVPSKFSGSGSG--<br>TDFTLTISSLQPEDFATYYC | QQYNS-----------<br>------------YPIT | FGQGT<br>RLEIK |
| **iPS:393 062** | **21-225_33H3** | **VK1\|L1/JK5** | | DIQMTQSPSSLSA<br>SVGDRVTITC | QAS--QDIS--<br>----NFLN | WFRQKPGK<br>APNSLIY | D--------<br>ASNLVT | GVPSRFSGRGSG--<br>TDFTFTISSLQPEDFATYYC | QQYDN-----------<br>------------LPIT | FGQGT<br>RLEIK |
| **iPS:398 532** | **21-225_33B7** | **VK1\|L1/JK5** | | DIQMTQSPSSLSA<br>SVGDRVTITC | RAS--QDIS--<br>----NYLA | WFQQKPGK<br>APTSLIY | A--------<br>ASSLQS | GVPSKFSGSGSG--<br>TDFTLTISSLQPEDFATFYC | QQYHS-----------<br>------------YPLT | FGQGT<br>RLEIK |
| **iPS:402 221** | **21-225_2C12** | **VK1\|L1/JK5** | | DIQMTQSPSSLSA<br>SVGDRVTITC | RAS--QGIS--<br>----NYLA | WFQQKSGK<br>APKSLIS | A--------<br>ATSLQS | GVPSQFSGSGSG--<br>TDFTLTISSLQPEDFATYYC | QQYYS-----------<br>------------YPIT | FGQGT<br>RLEIK |
| | | **Germline** | | **K_FR1** | **K_CDR1** | **K_FR2** | **K_CDR2** | **K_FR3** | **K_CDR3** | **K_FR4** |
| | **VK4\|B3/JK5** | | 32171 | DIVMTQSPDSLAV<br>SLGERATINC | KSS--<br>QSVLYSSNNKN<br>YLA | WYQQKPGQ<br>PPKLLIY | W--------<br>ASTRES | GVPDRFSGSGSG--<br>TDFTLTISSLQAEDVAVYYC | QQYYS-----------<br>------------TPIT | FGQGT<br>RLEIK |

| | | | | | | | | | | |
|---|---|---|---|---|---|---|---|---|---|---|
| iPS:435665 | 21-225_169F2 | VK4\|B3/JK5 | | DIVMTQSPDSLAV SLGERATINC | KSS-- QSVLYISNNKN YLA | WYQQKPGQ PPKLLIY | W-------- ASTRES | GVPDRFSGSGSG-- TDFTLTISSLQAEDVAVYYC | QQYY------------ ------------RAPT | FGQGT RLEIK |
| iPS:435671 | 21-225_169H5 | VK4\|B3/JK5 | | DIVMTQSPDSLAV SLGERATINC | KSS-- QSVLYISNNKN YLA | WYQQKPGQ PPKLLIY | W-------- ASTRES | GVPDRFSGSGSG-- TDFTLTISSLQAEDVAVYYC | QQYY------------ ------------RAPT | FGQGT RLEIK |
| iPS:436554 | 21-225_224C10 | VK4\|B3/JK5 | | DIVMTQSPDSLAA SLGERATITC | KSS-- QSVLYNSNNKN YLA | WYQQKPGQ PPKLLIY | W-------- ASTRES | GVPDRFSGSGSG-- TDFTLTISSLQAEDVAIYYC | QQYYI----------- ------------NPCS | FGQGT RLEIK |
| iPS:398510 | 21-225_25A3 | VK4\|B3/JK5 | | DIVMTQSPDSLTV SLGERATINC | KSS-- QSVLYSSNNKN YLA | WYQQKPGQ PPKLLIY | W-------- ASTRES | GVPDRFSGSGSG-- TDFTLTISSLQAEDVAVYYC | QQYYS----------- ------------TPCS | FGQGT RLEIK |
| iPS:398516 | 21-225_26A9 | VK4\|B3/JK5 | | DIVMTQSPDSLAV SLGERATINC | KSS-- QSVLYSSNNKN YLA | WYQQKPGQ PPKLLIY | W-------- ASTRES | GVPDRFSGSGSG-- TDFTLTISSLQAEDVAVYYC | QQYYS----------- ------------SPCS | FGQGT RLEIK |
| | | Germline | | K_FR1 | K_CDR1 | K_FR2 | K_CDR2 | K_FR3 | K_CDR3 | K_FR4 |
| | VK2\|A19/JK1 | | 32172 | DIVMTQSPLSLPV TPGEPASISC | RSS-- QSLLHSN- GYNYLD | WYLQKPGQ SPQLLIY | L-------- GSNRAS | GVPDRFSGSGSG-- TDFTLKISRVEAEDVGVYYC | MQALQ----------- ------------TPWT | FGQGT KVEIK |
| iPS:435667 | 21-225_169E3 | VK2\|A19/JK1 | | DIVMTQSPLSLPV TPGEPASISC | RSS-- QSLLHNN- GYKYLD | WYLQKPGQ SPQLLIY | L-------- GSNRAS | GVPDRFSASGSG-- TDFTLKISRVEAEDVGVYYC | MQVLQ----------- ------------TPWT | FGQGT KVEIK |
| iPS:435673 | 21-225_169E6 | VK2\|A19/JK1 | | DIVMTQSPLSLPV TPGEPASISC | RSS-- QSLLHNN- GYKYLD | WYLQKPGQ SPQLLIY | L-------- GSNRAS | GVPDRFSASGSG-- TDFTLKISRVEAEDVGVYYC | MQVLQ----------- ------------TPWT | FGQGT KVEIK |
| iPS:435759 | 21-225_176E6 | VK2\|A19/JK1 | | DIVMTQSPLSLPV TPGEPASISC | RSS-- QSLLHNN- GYKYLD | WYLQKPGQ SPQLLIY | L-------- GSNRAS | GVPDRFSASGSG-- TDFTLKISRVEAEDVGVYYC | MQVLQ----------- ------------TPWT | FGQGT KVEIK |
| | | Germline | | K_FR1 | K_CDR1 | K_FR2 | K_CDR2 | K_FR3 | K_CDR3 | K_FR4 |
| | VK6\|A26/JK1 | | 32173 | EIVLTQSPDFQSV TPKEKVTITC | RAS--QSIG-- ----SSLH | WYQQKPDQ SPKLLIK | Y-------- ASQSFS | GVPSRFSGSGSG-- TDFTLTINSLEAEDAATYYC | HQSSS----------- ------------LPWT | FGQGT KVEIK |
| iPS:435817 | 21-225_190B11 | VK6\|A26/JK1 | | EIVLTQSPDFQSV APKEKVTITC | RAS--QSIG-- ----SNLH | WYQQKPDQ SPKLLIK | S-------- ASQSFS | GVPSRFSGSGSG-- TDFTLTINSLETEDAATYYC | QQSSS----------- ------------LPWT | FGQGT KVEIK |
| iPS:435823 | 21-225_190F11 | VK6\|A26/JK1 | | EIVLTQFPDSQSV TPKEKVTITC | RAS--QNIG-- ----SSLH | WYQQKPEQ SPKVLIK | Y-------- ASQSFS | GVPSRFSGSGSG-- TDFTLTINSLEAEDAATYYC | HQSSS----------- ------------FPRT | FGQGT KVEIK |
| iPS:435867 | 21-225_191E5 | VK6\|A26/JK1 | | EIVLTQFPDSQSV TPKEKVTITC | RAS--QSIG-- ----SSLH | WYQQKPDQ SPKLLIK | Y-------- ASQSFS | GVPSRFSGSGSG-- TDFTLTINSLEAEDAATYYC | HQSSS----------- ------------FPRT | FGQGT KVEIK |
| iPS:435917 | 21-225_190D5 | VK6\|A26/JK1 | | EIVLTQSPDFQSV APKEKVTITC | RAS--QSIG-- ----SNLH | WYQQKPDQ SPKLLIK | S-------- ASQSFS | GVPSRFSGSGSG-- TDFTLTINSLETEDAATYYC | QQSSS----------- ------------LPWT | FGQGT KVEIK |
| iPS:435929 | 21-225_190D9 | VK6\|A26/JK1 | | EIVLTQFPDSQSV TPKEKVTITC | RAS--QSIG-- ----SSLH | WYQQKPDQ SPKLLIK | Y-------- ASQSFS | GVPSRFSGSGSG-- TDFTLTINSLEAEDAATYYC | HQSSS----------- ------------FPRT | FGQGT KVEIK |
| iPS:435935 | 21-225_190H8 | VK6\|A26/JK1 | | EIVLTQFPDSQSV TPKEKVTITC | RAS--QSIG-- ----SSLH | WYQQKPDQ SPKLLIK | Y-------- ASQSFS | GVPSRFSGSGSG-- TDFTLTINSLEAEDAATYYC | HQSSS----------- ------------FPRT | FGQGT KVEIK |
| iPS:436056 | 21-225_194C3 | VK6\|A26/JK1 | | EIVLTQSPDFQSV APKEKVTITC | RAS--QSIG-- ----SNLH | WYQQKPDQ SPKLLIK | S-------- ASQSFS | GVPSRFSGSGSG-- TDFTLTINSLETEDAATYYC | QQSSS----------- ------------LPWT | FGQGT KVEIK |

| | | | | | | | | | | |
|---|---|---|---|---|---|---|---|---|---|---|
| iPS:436 220 | 21-225_200F8 | VK6\|A26/ JK1 | | EIVLTQSPDFQSV APKEKVTITC | RAS--QSIG-- ----SNLH | WYQQKPDQ SPKLLIK | S-------- ASQSFS | GVPSRFSGSGSG-- TDFTLTINSLETEDAATYYC | QQSSS----------- ------------LPWT | FGQGT KVEIK |
| iPS:436 448 | 21-225_217A3 | VK6\|A26/ JK1 | | EIVLTQSPDFKSV TPKEKVTITC | RAS--QSIG-- ----SSLH | WYQQKPDQ SPKLLVK | Y-------- ASQSFS | GVPSRFSGSGSG-- TDFTLTINSLEAEDGATYYC | HQSRS----------- ------------LPWT | FGQGT KVEIK |
| | | Germline | | **K_FR1** | **K_CDR1** | **K_FR2** | **K_CDR2** | **K_FR3** | **K_CDR3** | **K_FR4** |
| | VK6\|A26/JK 4 | | 32174 | EIVLTQSPDFQSV TPKEKVTITC | RAS--QSIG-- ----SSLH | WYQQKPDQ SPKLLIK | Y-------- ASQSFS | GVPSRFSGSGSG-- TDFTLTINSLEAEDAATYYC | HQSSS----------- ------------LPLT | FGGGT KVEIK |
| iPS:435 829 | 21-225_190B1 2 | VK6\|A26/ JK4 | | EIVLTQSPDFQSV TPKEKVTITC | RAS--QSIG-- ----SSLH | WYQQKPDQ SPKLLIK | Y-------- ASQSFS | GDPSRFSGSGSG-- TDFTLTINSLEAEDAATYYC | HQTRS----------- ------------LPLT | FGGGT KVEIK |
| iPS:435 863 | 21-225_191H4 | VK6\|A26/ JK4 | | EIVLTQSPDFQSV TPKEKVTITC | RAN--QSIG-- ----SSLH | WYQQKPDQ SPKLLIK | Y-------- ASQSLS | GVPSRFSASGSG-- TDFTLTINSLDAEDAATYYC | HQTGR----------- ------------LPLT | FGGGT KVEIK |
| iPS:435 943 | 21-225_191C9 | VK6\|A26/ JK4 | | EIVLTQSPDFQSV TPKEKVTITC | RAS--QSIG-- ----SSLH | WYQQKPDQ SPKLLIK | Y-------- ASQSFS | GDPSRFSGSGSG-- TDFTLTINSLEAEDAATYYC | HQTRS----------- ------------LPLT | FGGGT KVEIK |
| iPS:435 983 | 21-225_192E5 | VK6\|A26/ JK4 | | EIVLTQSPDFQSV TPKEKVTITC | RAS--QSIG-- ----RSLH | WYQQKPDQ SPKLLIK | Y-------- ASQSFS | GVPSRFSGSGSG-- TDFTLTINSLEAEDAATYYC | HQSSR----------- ------------LPLT | FGGGT KVEIK |
| iPS:436 043 | 21-225_193G9 | VK6\|A26/ JK4 | | EIVLTQSPDFQSV TPKEKVTITC | RAS--QSIG-- ----RSLH | WYQQRPDQ SLKLLIK | Y-------- ASQSFS | GVPSRFSGSGSG-- TDFTLTINSLEAEDAATYFC | HQSSR----------- ------------LPLT | FGGGT KVEIK |
| iPS:436 084 | 21-225_195F2 | VK6\|A26/ JK4 | | EIVLTQSPDFQSV TPKEKVTITC | RAS--QSIG-- ----SSLH | WYQQKPDQ SPKLLIK | Y-------- ASQSFS | GVPSRFSGSGSG-- TDFALTISSLEAEDAATYYC | HQSRT----------- ------------LPLT | FGGGT KVEIK |
| iPS:436 094 | 21-225_195B1 0 | VK6\|A26/ JK4 | | EIVLTQSPDFQSV TPKEKVTITC | RAS--QSIG-- ----SSLH | WYQQKPDQ SPKLLIK | Y-------- ASQSFS | GVPSRFSGSGSG-- TDFTLTINSLEAEDAATYYC | HQSGR----------- ------------LPLT | FGGGT KVEIK |
| iPS:436 240 | 21-225_201E8 | VK6\|A26/ JK4 | | EIVLTQSPAFQSV TPKEKVTITC | RAS--QNIG-- ----RSLH | WYQQKPDQ SPKLLIK | Y-------- ASQSFS | GVPSRFSGSGSG-- TNFTLTINSLEAEDAVTYYC | HQSRS----------- ------------LPLT | FGGGT KVEIR |
| iPS:436 314 | 21-225_206G4 | VK6\|A26/ JK4 | | EIVLTQSPDFQSV TPKEKVTITC | RAS--QSIG-- ----RSLH | WYQQKPDQ SPKLLIK | Y-------- ASQSFS | GVPSRFSGSGSG-- TDFTLTINSLEAEDAATYYC | HQSRS----------- ------------LPLT | FGGGT KVEIK |
| | | Germline | | **K_FR1** | **K_CDR1** | **K_FR2** | **K_CDR2** | **K_FR3** | **K_CDR3** | **K_FR4** |
| | VK1\|A20/JK 3 | | 32175 | DIQMTQSPSSLSA SVGDRVTITC | RAS--QGIS-- ----NYLA | WYQQKPGK VPKLLIY | A-------- ASTLQS | GVPSRFSGSGSG-- TDFTLTISSLQPEDVATYYC | QKYNS----------- ------------APFT | FGPGT KVDIK |
| iPS:435 833 | 21-225_190D1 2 | VK1\|A20/ JK3 | | DIQMTQSPSSLSA SVGDRVTITC | RAS--QGIS-- ----NYLA | WYQQKPGK VPKLLIY | V-------- ASTLQS | GVPSRFSGSGSG-- TDFTLTISSLQPEDVATYYC | QKYNS----------- ------------APFT | FGPGT KVDIK |
| iPS:436 019 | 21-225_193C4 | VK1\|A20/ JK3 | | DIQMTQSPSSLSA SVGDRVTITC | RPS--QGIS-- ----IYLA | WYQQKPGN VPKLLIY | A-------- ASTLQS | GVPSRFSGSGSG-- TDFTLTISSLQPEDVATYYC | QKYNS----------- ------------APFT | FGPGT KVDIK |
| iPS:394 010 | 21-225_12G5 | VK1\|A20/ JK3 | | DIQMTQSPSSLSA SVGDRVTIPC | RAS--QDIS-- ----NYLA | WYQQKPGK VPKLLIY | A-------- AYILQS | GVPSRFSGSGSG-- TDFTLTISSLQPEDVAAYYC | QKYDS----------- ------------APFT | FGPGT KVDIK |
| | | Germline | | **K_FR1** | **K_CDR1** | **K_FR2** | **K_CDR2** | **K_FR3** | **K_CDR3** | **K_FR4** |
| | VK6\|A26/JK 3 | | 32176 | EIVLTQSPDFQSV TPKEKVTITC | RAS--QSIG-- ----SSLH | WYQQKPDQ SPKLLIK | Y-------- ASQSFS | GVPSRFSGSGSG-- TDFTLTINSLEAEDAATYYC | HQSSS----------- ------------LPFT | FGPGT KVDIK |
| iPS:436 025 | 21-225_193B5 | VK6\|A26/ JK3 | | EIVLTQSPDFQSV TPKEKVTITC | RAS--QSIG-- ----SSLH | WYQQKPDQ SPKLLIK | Y-------- ASQSFS | GVPSRFSGSGSG-- TDFTLTINSLEAEDAATYYC | HQSSR----------- ------------LPFT | FGPGT KVDIK |
| iPS:436 096 | 21-225_195E1 0 | VK6\|A26/ JK3 | | EIVLTQSPDFQSV TPKEKVTITC | RAS--QSIG-- ----SSLH | WYQQKPDQ SPKLLIK | Y-------- ASQSFS | GVPSRFSGSGSG-- TDFTLTINSLEAEDAATYYC | HQSSR----------- ------------LPFT | FGPGT KVDIK |
| iPS:436 | 21- | VK6\|A26/ | | EIVLTQSPDFQSV | RAS--QSIG-- | WYQQKPDQ | Y-------- | GVPSRFSGSGSG-- | HQSRS----------- | FGPGS |

| | | | | | | | | | | |
|---|---|---|---|---|---|---|---|---|---|---|
| iPS:436 424 | 21-225_215H6 | VK6\|A26/JK3 | | EIVLTQSPDFQSV TPKEKVTITC | RAS--QSIG-- ----VSLH | WYQQKPDQ SPQLLIK | Y------- ASQSLS | GVPSRFSGSGSG-- TDFTLTINSLEAEDAATYYC | HQSRS----------- ------------LPFT | FGPGS KVDIK |
| | | Germline | | **K_FR1** | **K_CDR1** | **K_FR2** | **K_CDR2** | **K_FR3** | **K_CDR3** | **K_FR4** |
| | VK1\|L5/JK2 | | 32177 | DIQMTQSPSSVSA SVGDRVTITC | RAS--QGIS-- ----SWLA | WYQQKPGK APKLLIY | A------- ASSLQS | GVPSRFSGSGSG-- TDFTLTISSLQPEDFATYYC | QQANS----------- ------------FPYT | FGQGT KLEIK |
| iPS:436 082 | 21-225_195D9 | VK1\|L5/JK2 | | DIQMTQSPSSVSA SVGDRVTITC | RAS--QGIS-- ----RWLA | WYQQKPGK APKLLIY | A------- ASSLLG | GVPSRFSGSGSG-- TDFTLTISSLQPEDFATYYC | QRANS----------- ------------FPCS | FGQGT KLEIK |
| iPS:436 118 | 21-225_196A10 | VK1\|L5/JK2 | | DIQMTQYPSYVSA SVGDRVSITC | RAS--QGIS-- ----RWLA | WYQQKPGK AAKFLIY | A------- ASSLLG | GVSSRFSGSGSG-- TDFTLTISSLQPEDFAIYYC | QRDNS----------- ------------LPCS | FGQGT KLEIK |
| | | Germline | | **K_FR1** | **K_CDR1** | **K_FR2** | **K_CDR2** | **K_FR3** | **K_CDR3** | **K_FR4** |
| | VK2\|A19/JK2 | | 32178 | DIVMTQSPLSLPV TPGEPASISC | RSS-- QSLLHSN- GYNYLD | WYLQKPGQ SPQLLIY | L------- GSNRAS | GVPDRFSGSGSG-- TDFTLKISRVEAEDVGVYYC | MQALQ----------- ------------TPYT | FGQGT KLEIK |
| iPS:436 362 | 21-225_210C12 | VK2\|A19/JK2 | | DIVMTQSPLSLPV TPGEPASISC | RSS-- QSLLHYN- GHNFLD | WYLQKPGQ SPQLLIY | L------- VSNRAS | GVPDRFSGSGSG-- TDFTLKISRVEAEDVGVYYC | MQALQ----------- -----------TPMCS | FGQGT KLEIK |
| iPS:436 374 | 21-225_211C10 | VK2\|A19/JK2 | | DIVMTQSPLSLPV TPGEPASISC | RSS-- QSLLHSN- GYNYLD | WYLLKPGQ SPQLLIY | L------- GSNRAS | GVPDRFSGSGSG-- TDFTLKISRMEAEDVGIYYC | MQALL----------- -----------TPVCS | FGQGT KLEIK |
| | | Germline | | **K_FR1** | **K_CDR1** | **K_FR2** | **K_CDR2** | **K_FR3** | **K_CDR3** | **K_FR4** |
| | VK2\|A17/JK1 | | 32179 | DVVMTQSPLSLPV TLGQPASISC | RSS-- QSLVYSD- GNTYLN | WFQQRPGQ SPRRLIY | K------- VSNRDS | GVPDRFSGSGSG-- TDFTLKISRVEAEDVGVYYC | MQGTH----------- ------------WPWT | FGQGT KVEIK |
| iPS:437 226 | 21-225_57C2 | VK2\|A17/JK1 | | DVVMTQSPLSLPV TLGQPASISC | RSS-- QSLVYSD- GNTYLN | WFQQRPGQ SPRRLIY | E------- VSNWDS | GVPNRFSGSGSG-- TDFTLKISAVEAEDVGVYYC | VQGTH----------- ------------WPRT | FGQGT KVEIK |
| | | Germline | | **K_FR1** | **K_CDR1** | **K_FR2** | **K_CDR2** | **K_FR3** | **K_CDR3** | **K_FR4** |
| | VK1\|A20/JK5 | | 32180 | DIQMTQSPSSLSA SVGDRVTITC | RAS--QGIS-- ----NYLA | WYQQKPGK VPKLLIY | A------- ASTLQS | GVPSRFSGSGSG-- TDFTLTISSLQPEDVATYYC | QKYNS----------- ------------APIT | FGQGT RLEIK |
| iPS:392 726 | 21-225_20B5 | VK1\|A20/JK5 | | DIQMTQSPSSLSA SVGDRVTITC | RAS--QGIN-- ----NYLA | WYQQKPGK IPKLLIY | A------- ASTLQS | GVPSRFSGSGSG-- TDFTLTISSLQPEDVATYCC | QKYNS----------- -----------APPIT | FGQGT RLEIK |
| iPS:392 792 | 21-225_20G12 | VK1\|A20/JK5 | | DIQMTQSPSSLSA SVGDRVTITC | RAS--QGIS-- ----NYLA | WYQQKPGK VPKVLIY | T------- ASTLQS | GVPSRFSGSGSG-- TDFTLTVSSLQPEDVATYYC | QKYNS----------- -----------APPIT | FGQGT RLEIK |
| iPS:398 478 | 21-225_17C10 | VK1\|A20/JK5 | | DIQMTQSPSSQSA SVGDRVTITC | RAS--QGIS-- ----NYLA | WYQQKPGR VPKLLIY | A------- ASTLQS | GVPSRFSGSGSG-- TDFTLTISSLQPDDVATYYC | QKYNS----------- -----------APPLT | FGQGT RLEIK |
| | | Germline | | **K_FR1** | **K_CDR1** | **K_FR2** | **K_CDR2** | **K_FR3** | **K_CDR3** | **K_FR4** |
| | VK1\|L1/JK2 | | 32181 | DIQMTQSPSSLSA SVGDRVTITC | RAS--QGIS-- ----NYLA | WFQQKPGK APKSLIY | A------- ASSLQS | GVPSRFSGSGSG-- TDFTLTISSLQPEDFATYYC | QQYNS----------- ------------YPYT | FGQGT KLEIK |
| iPS:392 776 | 21-225_21A12 | VK1\|L1/JK2 | | DIQMTQSPSSLSA SVGDRVTITC | RAS--QGIS-- ----KYLA | WFQQKPGK APKSLIY | A------- ASSLQS | GVPSKFSGSGSG-- TDFTLTISSLQPEDFATYYC | QQYNS----------- ------------YPFR | FGQGT KLEIK |
| | | Germline | | **K_FR1** | **K_CDR1** | **K_FR2** | **K_CDR2** | **K_FR3** | **K_CDR3** | **K_FR4** |
| | VK1\|A30/JK3 | | 32182 | DIQMTQSPSSLSA SVGDRVTITC | RAS--QGIR-- ----NDLG | WYQQKPGK APKRLIY | A------- ASSLQS | GVPSRFSGSGSG-- TEFTLTISSLQPEDFATYYC | LQHNS----------- ------------YPFT | FGPGT KVDIK |
| iPS:437 240 | 21-225_84H12 | VK1\|A30/JK3 | | DIQMTQSPSYQSA SVGDRVTITC | RAS--QGIR-- ----NDLG | WFQQKPGK APKRLIY | A------- ASSLQS | GVPSRFSGSGSG-- TEFTLTISSLQPEDFATYYC | LQHND----------- ------------YPFT | FGPGT KVDIK |

| | | | | | | | | | | |
|---|---|---|---|---|---|---|---|---|---|---|
| iPS:434 577 | 21-225_75C11 | VK1\|A30/JK3 | | DIQMTQSPSSLSA SVGDRVTITC | RAS--QGIR-- ----NDLG | WFQQKPGK APKRLIY | A-------- ASSLQS | GVPSRFSGSGSG-- TEFTLTISSLQPEDFATYYC | LQHND----------- ------------YPFT | FGPGT KVDIK |
| iPS:434 553 | 21-225_76H12 | VK1\|A30/JK3 | | DIQMTQSPSSLSA SVGDRVTITC | RAS--QGIR-- ----NDLG | WFQQKPGK APKRLIY | A-------- ASRLQS | GVPSRFSGSGSG-- TEFTLTISSLQPEDFATYYC | LQHND----------- ------------YPFT | FGPGT KVDIK |
| iPS:434 927 | 21-225_86E5 | VK1\|A30/JK3 | | DIQMTQSPSSLSA SVGDRVTITC | RAS--QGIR-- ----NDLG | WYQQKPGK APKRLIY | A-------- ASSLQS | GVPSRFSGSGSG-- TEFTLTISSLQPEDFATYHC | LQHND----------- ------------YPFT | FGPGT KVEIK |
| | | Germline | | **K_FR1** | **K_CDR1** | **K_FR2** | **K_CDR2** | **K_FR3** | **K_CDR3** | **K_FR4** |
| | VK1\|L5/JK1 | | 32183 | DIQMTQSPSSVSA SVGDRVTITC | RAS--QGIS-- ----SWLA | WYQQKPGK APKLLIY | A-------- ASSLQS | GVPSRFSGSGSG-- TDFTLTISSLQPEDFATYYC | QQANS----------- ------------FPWT | FGQGT KVEIK |
| iPS:435 477 | 21-225_154E8 | VK1\|L5/JK1 | | DIQMTQSPSSVSA SIGDRVTITC | RAS--QFIS-- ----SWLA | WYQQKPGK APKLLIY | T-------- ASSLQS | GVPSRFSGSGSG-- TDFTLTISSLQPEDFATYCC | QQANS----------- ------------FPWT | FGQGT KVEFN |
| iPS:435 385 | 21-225_149G7 | VK1\|L5/JK1 | | DIQMTQSPSSVSA SVGDRVTITC | RAS--QFIS-- ----SWLA | WYQQKPGK APKFLIY | A-------- ASSLQS | GVPSRFSGSGSG-- TDFTLTISSLQPEDFATYYC | QQANS----------- ------------FPWT | FGQGT KVEIN |
| **LAMBDA_VARIABLE** | | | | | | | | | | |
| | | Germline | | **L_FR1** | **L_CDR1** | **L_FR2** | **L_CDR2** | **L_FR3** | **L_CDR3** | **L_FR4** |
| | VL3\|3r/JL2 | | 32184 | SYELTQP- PSVSVSPGQTASI TC | SGD---KLGD- ----KYAC | WYQQKPGQ SPVLVIY | Q-------- DSKRPS | GIPERFSGSNSG-- NTATLTISGTQAMDEADYYC | QAWDS----------- -----------STAVV | FGGGT KLTVL |
| iPS:4 53445 | 21-225_148E10 | VL3\|3r/JL2 | | SYELTQP- PSVSVSPGQTASI TC | SGD---KLGN- ----KYVC | WYQQRPGH AAVLIIY | Q-------- DSKRPS | GIPERFSGSNSG-- NTATLTISGTQAMDEADYFC | QAWDR----------- -----------NTYVV | FGGGT KLTVL |
| iPS:4 72742 | 21-225_30D9_LC2 | VL3\|3r/JL2 | | SYELTQP- PSVSVSPGQTASI TC | SGD---KLGD- ----KYVY | WFQQKPGQ SPVLVIY | Q-------- DRKRPS | GIPERFSGSNSG-- NTATLTISGTQALDEADYYC | QAWDN----------- ------------STAV | FGGGT KLTVL |
| iPS:4 72743 | 21-225_68G6 | VL3\|3r/JL2 | | SYEVTQP- PSVSVSPGQTASI TC | SGD---KLGD- ----KYTY | WYQQKAGQ SPFLVIY | Q-------- DRKRPS | GIPDRFSGSNSG-- NTATLTISGTQAMDAADFYC | QAWDN----------- ------------STAV | FGGGT KLTVL |
| iPS:4 36652 | 21-225_146B11 | VL3\|3r/JL2 | | SYELTQP- PSVSVSPGQTASI TC | SGD---KLGD- ----KYAS | WYQQKPGQ SPVLVIY | Q-------- DSKRPS | GIPERFSGSNSG-- NTATLTISGIQAMDEADYYC | QAWDS----------- ------------STVV | FGGGT KLTVL |
| iPS:4 36654 | 21-225_146C11 | VL3\|3r/JL2 | | SYELTQP- PSVSVSPGQTASI TC | SGD---KLGD- ----KYAS | WYQQKPGQ SPVLVIY | Q-------- DSKRPS | GIPERFSGSNSG-- NTATLTISGIQAMDEADYYC | QAWDS----------- ------------STVV | FGGGT KLTVL |
| iPS:4 36658 | 21-225_146A2 | VL3\|3r/JL2 | | SYELTQP- PSVSVSPGQTASI TC | SGD---KLGD- ----KYAS | WYQQKPGQ SPVLVIY | Q-------- DSKRPS | GIPERFSGSNSG-- NTATLTISGIQAMDEADYYC | QAWDS----------- ------------STVV | FGGGT KLTVL |
| iPS:4 36664 | 21-225_147E7 | VL3\|3r/JL2 | | SYELTQP- PSVSVSPGQTASI TC | SGD---KLGD- ----KYAS | WYQQKPGQ SPVLVIY | Q-------- DSKRPS | GIPERFSGSNSG-- NTATLTISGIQAMDEADYYC | QAWDS----------- ------------STVV | FGGGT KLTVL |
| iPS:4 36666 | 21-225_147B8 | VL3\|3r/JL2 | | SYELTQP- PSVSVSPGQTASI TC | SGD---KLGD- ----KYVC | WYQQKPGQ SPELVIY | Q-------- DRKRPS | GIPERFSGSNSG-- NTATLTISGTQAMDEADYYC | QAWGS----------- -----------NTAVV | FGGGT KLTVL |
| iPS:4 36668 | 21-225_147B9 | VL3\|3r/JL2 | | SYELTQP- PSVSVSPGQTASI TC | SGD---KLGD- ----KYVS | WYQQRPGQ SPVLVIY | Q-------- DRKRPS | GIPERFSGSNSG-- NTATLTISGTLAVDEADYYC | LAWDS----------- -----------STFVV | FGGGT KLTVL |
| iPS:4 | 21-225_147D9 | VL3\|3r/JL | | SYELTQP- | SGD---KLGN- | WYQQRPGQ | Q-------- | GIPERFSGSNSG-- | QAWDR----------- | FGGGT |

| | | | | | | | | | | |
|---|---|---|---|---|---|---|---|---|---|---|
| iPS:436672 | 21-225_147F9 | VL3\|3r/JL2 | | SYELTQP-PSVSVSPGQTASITC | SGD---ELGN-----KYAC | WYQQKPGQSPVLVIY | Q--------DSKRPS | GIPERFSGSNSG--NTATLTISGTQAMDEADYYC | QAWHS-----------------------STVV | FGGGTKLTVL |
| iPS:436674 | 21-225_147G9 | VL3\|3r/JL2 | | SYELTQP-PSVSVSPGQTASITC | SGD---KLGD-----KYAC | WYQQKPGQSPVLVIY | Q--------DRKRPS | GIPERFSGSNSG--NTATLTISGTQAMDEADYYC | QAWHS-----------------------STVV | FGGGTKLTVL |
| iPS:436676 | 21-225_147E11 | VL3\|3r/JL2 | | SYELTQP-PSVSVSPGQTASITC | SGD---KLGD-----KYAS | WYQQKPGQSPVLVIY | Q--------DSKRPS | GIPERFSGSNSG--NTATLTISGIQAMDEADYYC | QAWDS-----------------------STVV | FGGGTKLTVL |
| iPS:436678 | 21-225_147B12 | VL3\|3r/JL2 | | SYELTQP-PSVSVSPGQTASITC | SGD---KLGD-----KYAS | WYQQKPGQSPVLVIY | Q--------DSKRPS | GIPERFSGSNSG--NAATLTISGIQAMDEADYYC | QAWDS-----------------------STVV | FGGGTKLTVL |
| iPS:436686 | 21-225_148G6 | VL3\|3r/JL2 | | SYELTQP-PSVSVSPGQTASITC | SGD---KLGD-----KYAS | WYQQKPGQSPVLVIY | Q--------DSKRPS | GIPERFSGSNSG--NTATLTISGIQAMDEADYYC | QAWDS-----------------------STVV | FGGGTKLTVL |
| iPS:436688 | 21-225_148C8 | VL3\|3r/JL2 | | SYELTQP-PSVSVSPGQTASITC | SGD---KLGD-----KYVS | WYQQKPGQSPILVIY | Q--------DRKRPS | GTPERFSGSNSG--NTATLTISGTQAMDEADYYC | LAWDS----------------------STFVV | FGGGTKLTVL |
| iPS:436690 | 21-225_148A9 | VL3\|3r/JL2 | | SYELTQP-PSVSVSPGQTASITC | SGD---KLGN-----KYVC | WYQQKPGQSPVLVIY | Q--------DSKRPS | GIPERFSGSNSG--NTATLTISGTQAMDEADYYC | QAWHS-----------------------STVV | FGGGTKLTVL |
| iPS:436694 | 21-225_148G11 | VL3\|3r/JL2 | | SYELTQP-PSVSVSPGQTASITC | SGD---KLGD-----KFAS | WYQQKPGQSPVLVIY | Q--------DSKRPS | GIPERFSGSNSG--NTATLTISGIQAMDEADYYC | QAWDS-----------------------STVV | FGGGTKLTVL |
| iPS:436698 | 21-225_149B5 | VL3\|3r/JL2 | | SYELTQP-PSVSVSPGQTASITC | SGY---KLGY-----KYVC | WYQQKPGQSPVLVIF | Q--------NNQRPS | GIPERFSGSNSG--NTASLTISGTQAMDEADYYC | QAWDS-----------------------STVV | FGGGTKLTVL |
| iPS:436700 | 21-225_149C7 | VL3\|3r/JL2 | | SYELTQP-PSVSVSPGQTASITC | SGN---KLGD-----KYAS | WYQQKPGQSPVLVIY | Q--------DSKRPS | GIPERFSGSKSG--NTATLTISGIQAMDEADYYC | QAWDS-----------------------STVV | FGGGTKLTVP |
| iPS:436704 | 21-225_149C10 | VL3\|3r/JL2 | | SYELTQP-PSVSVSPGQTASITC | SGD---KLGD-----KYAS | WYQQKPGQSPVLVIY | Q--------DNKRPS | GIPERFSGSNSG--NTATLTIGGIQAMDEADYYC | QAWDS-----------------------STVV | FGGGTKLTVL |
| iPS:436706 | 21-225_149A11 | VL3\|3r/JL2 | | SYELTQP-PSVSVSPGQTASITC | SGD---KLGN-----KYVS | WYQQKPGQSPVLVIY | Q--------DSRRPS | GIPERFSGSNSG--NTATLTISGTQAMDEADYYC | LAWDS----------------------STFVV | FGGGTKLTVL |
| iPS:436708 | 21-225_150D3 | VL3\|3r/JL2 | | SYELTQP-PSVSVSPGQTASITC | SGD---ELGN-----KYAC | WYQQKPGQSPVLVIY | Q--------DNKRPS | GIPERFSGSSSG--NTATLTISGTQAMDEADYYC | QAWHS-----------------------STVV | FGGGTKLTVL |
| iPS:436710 | 21-225_150F6 | VL3\|3r/JL2 | | SYELTQP-PSVSVSPGQTASITC | SGD---KLGD-----KYAS | WYQQKPGQSPVLVIC | Q--------DSKRPS | GIPERFSGSNSG--NTATLTISGIQAMDEADYCC | QAWDS-----------------------STVV | FGGGTKLTVL |
| iPS:436714 | 21-225_150H11 | VL3\|3r/JL2 | | SYELTQP-PSVSVSPGQTASITC | SGD---KLGD-----KYAS | WYQQKPGQSPVLVIY | Q--------DSKRPS | GIPERFSGSNSG--NTATLTISGIQAMDEADFYC | QAWDS-----------------------STVV | FGGGTKLTVL |
| iPS:436716 | 21-225_151F3 | VL3\|3r/JL2 | | SYELTQP-PSVSVSPGQTASI | SGD---KLGD-----KYVC | WYQQKPGQSPVLVIY | Q--------DRKRPS | GIPERFSGSNSG--NTATLTISGTQAMDEADYYC | QAWHS-----------------------STVV | FGGGTKLTVL |

| | | | | | | | | | | |
|---|---|---|---|---|---|---|---|---|---|---|
| **iPS:4 36718** | **21-225_151H5** | **VL3\|3r/JL 2** | | SYELTQP- PSVSVSPGQTASI AC | SGD---KLGD- ----KYAS | WYQQKPGQ SPVLVIY | Q-------- DSKRPS | GIPERFSGSNSG-- NTATLTISGIQAMDEADYYC | QAWDS----------- ------------STVV | FGGGT KLTVL |
| **iPS:4 36722** | **21-225_151H7** | **VL3\|3r/JL 2** | | SYDLTQP- PSVSVSPGQTASI TC | SGD---KLGD- ----KYAS | WYQQKPGQ SPVLVIY | Q-------- DSKRPS | GIPERFSGSNSG-- NTATLTISGIQAMDEADYYC | QAWDS----------- ------------STVV | FGGGT KLTVL |
| **iPS:4 36724** | **21-225_151B9** | **VL3\|3r/JL 2** | | SYELTQP- PSVSVSPGQTASI AC | SGD---NLGD- ----KYAS | WYQQKPGQ SPVLVIY | Q-------- DSKRPS | GIPERFSGSNSG-- NTATLTISGIQAMDEADYYC | QAWDS----------- ------------STVV | FGGGT KLTVL |
| **iPS:4 36728** | **21-225_152G6** | **VL3\|3r/JL 2** | | SYELTQP- PSVSVSPGQTASI TC | SGD---KLGD- ----KYAS | WYQQKPGQ SPVLVIY | Q-------- DSKRPS | GIPERFSGSNSG-- NTATLTISGIQALDEADYYC | QAWDN----------- ------------STVV | FGGGT KLTVL |
| **iPS:4 36730** | **21-225_152D7** | **VL3\|3r/JL 2** | | SYELTQP- PSVSVSPGQTASI TC | SGD---KLGD- ----KYAS | WYQQKPGQ SPVLVIY | Q-------- DSKRPS | GIPERFSGSNSG-- NTATLTISGIQAMDEADYYC | QAWDS----------- ------------STVV | FGGGT KLTVL |
| **iPS:4 36736** | **21-225_153E8** | **VL3\|3r/JL 2** | | SYELTQP- PSVSVSPGQTASI TC | SGS---KLGN- ----KYVC | WYQQRPGQ SPVLVIY | Q-------- DNKRPS | GIPERFSGSNSG-- NTATLTISGTQAMDEAGYYC | QAWDS----------- -----------STYVI | FGGGT KLTVL |
| **iPS:4 36738** | **21-225_153D9** | **VL3\|3r/JL 2** | | SYELTQP- PSVSVSPGQTASI TC | SGD---KLGD- ----KYAC | WYQQKPGQ SPVLVIY | Q-------- DRKRPS | GIPERFSGSNSG-- NTATLTISGTQAMDEADYYC | QAWHS----------- ------------STVV | FGGGT KVTVL |
| **iPS:4 36740** | **21-225_154C3** | **VL3\|3r/JL 2** | | SYELTQP- PSVSVSPGQTASI TC | SGD---KLGD- ----KYVC | WYQQKPGQ SPVLVIY | Q-------- DRKRPS | GIPERFSGSNSG-- NTATLTITGTQAMDEADYYC | QAWHS----------- ------------STVV | FGGGT KLTVL |
| **iPS:4 36742** | **21-225_154C4** | **VL3\|3r/JL 2** | | SYELTQP- PSVSVSPGQTARI TC | SGD---KLGD- ----KYAS | WYQQKPGQ SPVLVIY | Q-------- DSKRPS | GIPERFSGSNSG-- NTATLTISGIQAMDEADYYC | QAWDS----------- ------------STVV | FGGGT KLTVL |
| **iPS:4 36744** | **21-225_154F4** | **VL3\|3r/JL 2** | | SYELTQP- PSVSVSPGQTASI TC | SGD---KLGN- ----KYVC | WYQQKPGQ SPVEVIY | K-------- DSKRPS | GIPERFSGSNSG-- NTGTLTISGTQAMDEADYYC | QAWDN----------- ------------STLV | FGGGT KLTVL |
| **iPS:4 36746** | **21-225_154E10** | **VL3\|3r/JL 2** | | SYELTQP- PSVSVSPGQTASI TC | SGD---KLGD- ----KYAS | WYQQKPGQ SPVLVIY | Q-------- DSKRPS | GIPERFSGSNSG-- NTATLTISGIQAMDEADYYC | QAWDN----------- ------------STVV | FGGGT KLTVL |
| **iPS:4 36748** | **21-225_154D11** | **VL3\|3r/JL 2** | | SYELTQP- PSVSVSPGQTASI TC | SGD---KLGD- ----KYAC | WYQQKPGQ SPVLVIY | Q-------- DKKRPS | GIPERFSGSNSG-- NIATLTISGTQAMDEADYYC | QAWHS----------- ------------SIVV | FGGGT KLTVL |
| **iPS:4 36756** | **21-225_146A10** | **VL3\|3r/JL 2** | | SYELTQP- PSVSVSPGQTASI TC | SGD---KLGD- ----KYVC | WYQQKPGQ SPVLVIF | Q-------- DRKRPS | GIPERFSGSNSG-- NTATLTISGTQAMDEADYYC | QAWDS----------- ------------STVV | FGGGT KVTVL |
| **iPS:4 36758** | **21-225_155C10** | **VL3\|3r/JL 2** | | SYELTQP- PSVSVSPGQTVSI TC | SGD---KLGD- ----KYVS | WYQQKPGQ SPVLVIY | Q-------- DSKRPS | GIPERFSGSNSG-- NTATLTISGIQAMDEADYYC | QAWDS----------- ------------STVV | FGGGT KLTVL |
| **iPS:4 36760** | **21-225_155E10** | **VL3\|3r/JL 2** | | SYELTQP- PSVSVSPGQTASI TC | SGD---KLGD- ----KYVS | WYQQKPGQ SPVLVIY | Q-------- DRKRPS | GIPERFSGSNSG-- NTATLTISGTQAMDEADYYC | LAWDS----------- -----------STFVV | FGGGT KLTVL |
| **iPS:4 36764** | **21-225_158E9** | **VL3\|3r/JL 2** | | SYDLTQP- PSVSVSPGQTASI | SGD---KLGD- ----KYVC | WYQQKPGQ SPVLVIY | Q-------- DSKRPS | GIPERFSGSNSG-- NTATLTITGTQAMDEADYYC | QAWDN----------- -----------SSFVL | FGGGT KLTVL |

| | | | | | | | | | | |
|---|---|---|---|---|---|---|---|---|---|---|
| **iPS:4 36766** | **21-225_158D10** | **VL3\|3r/JL 2** | | SYELTQP-PSVTVSPGQTASITC | SGD---KLGD-----KYVC | WYQQKPGQSPVLVIY | Q--------DRKRPS | GIPERLSGSNSG--NTATLTISGTQALDEADYYC | QAWGN----------------------SSFVV | FGGGTKLTVL |
| **iPS:4 36768** | **21-225_159H8** | **VL3\|3r/JL 2** | | SYELTQP-PSVSVSPGQTASITC | SGD---KLGD-----KYVC | WYQQKPGQSPVLVIY | Q--------DRKRPS | GIPERFSGSNSG--NTATLTISGTQALDEADYYC | QAWGN----------------------SSFVV | FGGGTKLTVL |
| **iPS:4 36770** | **21-225_160B12** | **VL3\|3r/JL 2** | | SDELTQS-PSVSVSPGQTASITC | SGD---KLGD-----KYVC | WYQQKPGQSPVLVIY | Q--------DSKRPS | GIPERFSGSNSG--NTATLTISGTQALDEADYYC | QAWGN----------------------SSFVV | FGGGTKLTVL |
| **iPS:4 36772** | **21-225_161H3** | **VL3\|3r/JL 2** | | SYELTQP-PSVSVSPGQTASITC | SGD---RLGD-----KYVC | WYQQKPGQSPVLVIF | Q--------DNKRPS | GIPERFSGSNSG--NTATLTISGTQALDEADYYC | QAWVN----------------------NTAVV | FGGGTKLTVL |
| **iPS:4 36774** | **21-225_161E10** | **VL3\|3r/JL 2** | | SFDLTQP-PSVSVSPGQTASITC | SGD---KLGD-----KYVC | WYQQKPGQSPVLVIY | Q--------DSKRPS | GIPERISGSNSG--NTATLTISGTQAMDEADYYC | QTWDN----------------------SSFAL | FGGGTKLTVL |
| **iPS:4 36776** | **21-225_161F12** | **VL3\|3r/JL 2** | | SYELTQP-PSVSVSPGQTASITC | SGD---KLGD-----KYAC | WYQQKPGQSPVLVIY | Q--------DTKRPS | GIPERFSGSNSG--NTATLTISGTQAMDEADYYC | QAWDS-----------------------TTLV | FGGGTKLTVL |
| **iPS:4 36780** | **21-225_165H3** | **VL3\|3r/JL 2** | | SYELTQP-PSVSVSPGQTASITC | SGD---KLGD-----KYAC | WYQQKPGQSPVLVIY | Q--------DSKRPS | GIPERFSGSNSG--NTATLTISGTQAMDEADYYC | QAWDS-----------------------TTLV | FGGGTKLTVL |
| **iPS:4 36782** | **21-225_166G11** | **VL3\|3r/JL 2** | | SYELSQP-PSVSVSPGQTASITC | SGD---KLGD-----KYVH | WYQQKPGQSPVLVIY | Q--------DSKRPS | GIPERFSGSNSG--NTATLTISGTQAMDEADYYC | QAWDN-----------------------STAV | FGGGTKLTVL |
| **iPS:4 36784** | **21-225_169C1** | **VL3\|3r/JL 2** | | SYELTQP-PSVSVSPGQTASITC | SGD---KLGD-----KYVC | WYQQKPGQSPLLVIY | K--------DIKRPS | GIPERFSGSNSG--NTATLTISGTQAMDEADYYC | QAWDT-----------------------NTVI | FGGGTKLTVL |
| **iPS:4 36786** | **21-225_169A6** | **VL3\|3r/JL 2** | | SYELTQP-PSVSVSPGQTASITC | SGD---KLGD-----KYVC | WYQRKPGQSPVLVIY | Q--------DYKRPS | GIPERFSGSNSG--NTATLTISGTQAMDEADYYC | QAWDT-----------------------NTVL | FGGGTKLTVL |
| **iPS:4 36788** | **21-225_169B7** | **VL3\|3r/JL 2** | | AYDLTQP-PSVSVSPGQTARITC | SGD---KLGG-----KYAS | WYQQKPGQSPVLVIY | Q--------DRKRPS | GIPERFSGSNSG--NTATLTISGTQAMDEADYYC | QAWDK-----------------------NTVV | FGGGTKLTVL |
| **iPS:4 36790** | **21-225_169G11** | **VL3\|3r/JL 2** | | SYELTQP-PSVSVSPGQTASITC | SGD---KLGD-----KYAC | WYQQKPGQSPVLVIY | Q--------DSKRPS | GIPERFSGSNSG--NTATLTISGTQAMDEADYYC | QAWDN-----------------------STAV | FGGGTKLTVL |
| **iPS:4 36794** | **21-225_170F1** | **VL3\|3r/JL 2** | | SYELSQP-PSVSVSPGQTASITC | SGD---KLGD-----KYSC | WYQQKPGQSPVLVIY | Q--------DSKRPS | GIPARFSGSNSG--NTATLTISGTQAMDEADYYC | QAWDS-----------------------NTAV | FGGGTKLTVL |
| **iPS:4 36796** | **21-225_170A5** | **VL3\|3r/JL 2** | | SYDLTQP-PSVSVSPGQTASITC | SGD---KLGD-----KYAC | WYQQKPGQSPILVIY | Q--------DYKRPS | GIPERFSGSNSG--NTATLTISGTQAMDEADYYC | QAWDN-----------------------STMV | FGGGTRLTVL |
| **iPS:4 36798** | **21-225_171F5** | **VL3\|3r/JL 2** | | AYDLTQP-PSVSVSPGQTASITC | SGD---KLGG-----KYAS | WYQQKPGQSPVLVIY | Q--------DRKRPS | GIPERFSGSNSG--NTATLTISGTQAMDEADYYC | QAWDK-----------------------NTVV | FGGGTKLTVL |
| **iPS:4 36802** | **21-225_171F12** | **VL3\|3r/JL 2** | | SYELTQP-PSVSVSPGQTASI | SGD---KLGD-----KYAC | WYQQKPGQSPVLVIY | Q--------DRKRPS | GIPERFSGSNSG--NTATLTISGTQAMDEADYYC | QAWDI----------------------STYVV | FGGGTKLTVL |

| | | | | | | | | | | |
|---|---|---|---|---|---|---|---|---|---|---|
| iPS:4 36808 | 21-225_173F8 | VL3\|3r/JL 2 | | SYELTQP-PSVSVSPGQTASITC | SGN---KLGN-----KYVC | WYQQRPGQSPVLVIS | Q--------DSRRPS | GIPERFSGSNSG--NTATLTISGTQAMDEADYYC | QAWDS-----------------------FTVV | FGGGTKLTVL |
| iPS:4 36812 | 21-225_175C6 | VL3\|3r/JL 2 | | SYDLTQP-PSVSVSPGQTASITC | SGD---KLGD-----KYAC | WYQQKPGQSPILVIY | Q--------DYKRPS | GIPERFSGSNSG--NTATLTISGTQAMDEADYYC | QAWDN-----------------------STMV | FGGGTRLTVL |
| iPS:4 36818 | 21-225_179C7 | VL3\|3r/JL 2 | | SYELTQP-PSVSVSPGQTASITC | SGD---KLGD-----KYVC | WYQQRPGQSPVLVIY | Q--------DSKRPS | GIPERFSGSNSG--NTATLTIRGTQAMDEADYYC | QAWDS----------------------NTAVV | FGGGTKLTVL |
| iPS:4 36822 | 21-225_180D4 | VL3\|3r/JL 2 | | SYELTQT-PSVSVSPGQTASITC | SGD---RLGD-----KYAC | WYQQKPGQSPVLVIY | E--------DRKRPS | GIPERFSGSNSG--NTATLTISGTQAMDEGDYYC | QAWDS-----------------------RKVV | FGGGTKLTVL |
| iPS:4 36824 | 21-225_180C5 | VL3\|3r/JL 2 | | SYELTQP-PSVSVSPGQTASITC | SGD---KLGE-----KYAC | WYQQKPGQSPVLVIY | Q--------DRKRPS | GIPERFSGSNSG--NTATLTISGTQAMDEADYYC | QAWDS-----------------------STAV | FGGGTKLTVL |
| iPS:4 36826 | 21-225_180G5 | VL3\|3r/JL 2 | | SYELTQP-PSVSVYPGQTASITC | SGD---KLGD-----KYVS | WYQQKPGQSPVLVIY | Q--------DSKRPS | GIPERFSGSNSG--NPASLTISGTQAMDEADYYC | QAWDI-----------------------TTAV | FGGGTKLTVL |
| iPS:4 36828 | 21-225_181H1 | VL3\|3r/JL 2 | | SYELTQT-PSVSVSPGQTASITC | SGD---KLGD-----KYAC | WYQQKPGQSPVLVIY | E--------DRKRPS | GIPERFSGSNSG--NTATLTISGTQAMDEADYYC | QAWDS-----------------------RKVV | FGGGTKLTVL |
| iPS:4 36836 | 21-225_52H1 | VL3\|3r/JL 2 | | SYELTQP-PSVFVSPGQTASITS | SGD---KLGD-----KYVS | WYQQKPGQSPVLVIY | Q--------DRKRPS | GIPERFSGSNSG--NTATLTISGTQAMDEADYYC | QAWDN-----------------------STVV | FGGGTKLTVL |
| iPS:4 36848 | 21-225_57F1 | VL3\|3r/JL 2 | | SYELTQP-PSASVSPGQTASITC | SGD---KLGE-----KYAC | WYQQKPGQSPVLVIY | Q--------DRKRPS | GIPERFSGSNSG--NTATLTISGTQAMDEADYYC | QAWDS-----------------------STVV | FGGGTKLTVL |
| iPS:4 36850 | 21-225_57D9 | VL3\|3r/JL 2 | | SYELTQP-PSVSVSPGQTASITC | SGE---KLGE-----KFAC | WSQQKPGQSPVLVIY | Q--------DSKRPS | GIPERFSGSNSG--NTATLTISGTQAMDEADYYC | QAWDS-----------------------STVV | FGGGTKLTVL |
| iPS:4 36852 | 21-225_57H11 | VL3\|3r/JL 2 | | SYALTQP-PSASVSPGQTASITC | SGD---KLGE-----KYAC | WYQQKPGQSPVLVIY | Q--------DRKRPS | GIPERFSGSNSG--NTATLTISGTQAMDEADYYC | QAWDS-----------------------STVV | FGGGTKLTVL |
| iPS:4 36854 | 21-225_58C1 | VL3\|3r/JL 2 | | SYELTQP-PSVSVSPGQTANITC | SGD---KLGN-----KYAC | WYQQKPGQSPVLVIY | Q--------DRKRPS | GIPERFSGSNSG--NTATLTISGTQAMDEADYYC | QAWD------------------------SSTA | FGGGTKLTVL |
| iPS:4 36858 | 21-225_58E7 | VL3\|3r/JL 2 | | SYELTQS-PSVSVSPGQTASITC | SGD---KLGD-----KYTC | WYQKKPGQSPVLVIY | Q--------DNKRPS | GIPERFSGSNSG--NTATLTISGTQAMDEADYFC | QAWNN-----------------------YTVV | FGGGTKLTAL |
| iPS:4 36860 | 21-225_58F7 | VL3\|3r/JL 2 | | SYELTQP-PSVSVSPGQTASITC | SGD---KLGD-----KYAC | WYQQKPGQSPVLVIY | Q--------DRKRPS | GIPERFSGSNSG--NTATLTISGTQAMDEADYYC | QAWDS-----------------------STVV | FGGGTKLTVL |
| iPS:4 36862 | 21-225_58F8 | VL3\|3r/JL 2 | | SYELTQP-PSVSVSPGQTASITC | SGD---KLGN-----KYAC | WYQQKPGQSPVLVIY | Q--------DSKRPS | GIPERFSGSNSG--NTATLTISGTQAMDEADYYC | QAWDS-----------------------STVV | FGGGTKLTVL |
| iPS:4 36864 | 21-225_58G11 | VL3\|3r/JL 2 | | SYELTQP-PSVSVSPGQTASI | SGD---KLGD-----KYAS | WYQQKPGQSPVLVIY | Q--------DNKRPS | GIPERFSGSNSG--NTATLTISGTQAMDEADYYC | QAWNN-----------------------NTVM | FGGGTKLTVL |

| | | | | | | | | | | |
|---|---|---|---|---|---|---|---|---|---|---|
| **iPS:4 36866** | **21-225_59F2** | **VL3\|3r/JL 2** | | SYELTQP-PSVSVSPGQTASITC | SGD---KLGD-----KYAS | WYQQRPGQSPVLVIY | Q--------DNKRPS | GIPERFSGSNSG--NTATLTISGTQAMDEADYYC | QAWDN-----------------------NTVV | FGGGTKLTVL |
| **iPS:4 36868** | **21-225_59B11** | **VL3\|3r/JL 2** | | SYELTQP-PSVSVSPGQTASITC | SGD---KLGD-----KYAC | WYQQKPGQSPVLVIY | Q--------DSKRPS | GILERFSGSNSG--NTATLTISGTQAMDEADYYC | QAWDS----------------------STYVV | FGGGTKLTVL |
| **iPS:4 36870** | **21-225_60B1** | **VL3\|3r/JL 2** | | SYELTQP-PSASVSPGQTASITC | SGD---KLGE-----KYAC | WYQQKPGQSPVLVIY | Q--------DRKRPS | GIPERFSGSNSG--NTATLTISGTQAMDEADYYC | QAWDS-----------------------STVV | FGGGTKLTVL |
| **iPS:4 36872** | **21-225_60D2** | **VL3\|3r/JL 2** | | SYELTQP-PSVSVSPGQTASITC | SGN---KLGD-----KYAS | WYQQRPGQSPVLVIY | Q--------DNKRPS | GIPERFSGSNSG--NTATLTISGTQTMDEADYYC | QAWDN-----------------------NTVV | FGGGTKLTVL |
| **iPS:4 36874** | **21-225_60A12** | **VL3\|3r/JL 2** | | SYELTQP-PSVSVSPGQTANITC | SGD---KLGN-----KYAC | WYQQKPGQSPVLVIY | Q--------DRKRPS | GIPERFSGSNSG--NTAILTISGTQAMDEADYYC | QAWD------------------------SSTA | FGGGTKLTVL |
| **iPS:4 36876** | **21-225_61F5** | **VL3\|3r/JL 2** | | SYELTQP-PSVSVSPGQTASITC | SGD---KLGE-----KYAC | WYQQKPGQSPVLVIY | Q--------DSKRPS | GIPERFSGSNSG--NTATLTISGTQAMDEADYYC | QAWDS-----------------------STVV | FGGGTKLTVL |
| **iPS:4 36878** | **21-225_62E3** | **VL3\|3r/JL 2** | | SYDLTQP-PSVSVSPGQTASITC | SGD---KLGN-----KYAC | WYQQKPGQSPVVVIY | Q--------DRKRPS | GIPERFSGSNSG--NTATLTISGTQAMDEADYYC | QAWDS-----------------------STAV | FGGGTKLTVL |
| **iPS:4 36880** | **21-225_62E8** | **VL3\|3r/JL 2** | | SYDLTQP-PSVSVSPGQTASITC | SGD---RLGN-----KYAS | WYQQKPGQSPVVVIY | Q--------DRKRPS | GIPERFSGSNSG--NTATLTISGTQAMDEADYYC | QAWDS-----------------------STAV | FGGGTKLTVL |
| **iPS:4 36882** | **21-225_62D10** | **VL3\|3r/JL 2** | | SYDLTQP-PSVSVSPGQTASITC | SGD---KLGN-----KYAC | WYQQKPGQSPVVVIY | Q--------DRKRPS | GIPERFSGSNSG--NTATLTISGTQAMDEADYYC | QAWDS-----------------------STAV | FGGGTKLTVL |
| **iPS:4 36884** | **21-225_62A12** | **VL3\|3r/JL 2** | | SYDLTQP-PSVSVSPGQTASITC | SGD---KLGN-----KYAC | WYQQKPGQSPVVVIY | Q--------DRKRPS | GIPERFSGSNSG--NTATLTISGTQAMDEADYYC | QAWDS-----------------------STAV | FGGGTKLTVL |
| **iPS:4 36886** | **21-225_62B12** | **VL3\|3r/JL 2** | | SYDLTQP-PSVSVSPGQTASITC | SGD---KLGN-----KYTC | WYQQKPGQSPVVVIY | Q--------DRKRPS | GIPERFSGSNSG--NTATLTISGTQAMDEADYYC | QAWDS-----------------------STAV | FGGGTKLTVL |
| **iPS:4 36892** | **21-225_65E9** | **VL3\|3r/JL 2** | | SYELTQP-PSVSVSPGQTASITC | SGD---KLGN-----KYDY | WYQQKPGQSPVLVIY | Q--------DRKRPS | GIPERFSGSNSG--NTATLTISGTQAMDEADYYC | QAWDN-----------------------STVV | FGGGTKLTVL |
| **iPS:4 36894** | **21-225_66G9** | **VL3\|3r/JL 2** | | SYDLTQP-PSVTVSPGQTASITC | SGD---KLGN-----KYAC | WYQQKPGQSPVVVIY | Q--------DRKRPS | GIPERFSGSNSG--NTATLTISGTQAMDEADYYC | QAWDI-----------------------NTAV | FGGGTKLTVL |
| **iPS:4 36896** | **21-225_67F10** | **VL3\|3r/JL 2** | | SYELTQP-PSVSVSPGQTASITC | SGD---KLGY-----KYAW | WYQQKPGQSPVLVIF | E--------DRKRPS | GIPERFSGSNSG--NTATLTISGTQAMDEADYYC | QAWDN-----------------------STVV | FGGGTRLTVL |
| **iPS:4 36898** | **21-225_68D8** | **VL3\|3r/JL 2** | | SYELTQP-PSVSVSPGQTASITC | SGD---KLGD-----KYAC | WYQQKPGQSPVLVIY | Q--------DSKRPS | GIPERFSGSNSG--NTATLTISGTQAMDEADYYC | QAWDN-----------------------STVV | FGGGTKLTVL |
| **iPS:4 36900** | **21-225_69B9** | **VL3\|3r/JL 2** | | SYELTQP-PSVSVSPGQTASI | SGD---KLGN-----KYDY | WYQQKPGQSPVLVIY | Q--------DRKRPS | GIPERFSGSNSG--NTATLTISGTQAMDEADYYC | QAWDN-----------------------STVV | FGGGTKLTVL |

| | | | | | | | | | | |
|---|---|---|---|---|---|---|---|---|---|---|
| iPS:436902 | 21-225_69B11 | VL3\|3r/JL2 | | SYELTQP-PSVSVSPGQAASITC | SGD---KLGD-----KYAW | WYQQKPGQSPVLVIY | E--------DRKRPS | GIPERFSGSNSG--NTATLTISGTQAMDEADYYC | QAWDN------------------------STVV | FGGGTKLTVL |
| iPS:436904 | 21-225_71D4 | VL3\|3r/JL2 | | SYELTQP-PSVSVSPGQTASITC | SGD---KLGD-----KYAY | WYQQKPGQSPVVVIY | Q--------DRKRPS | GIPERFSGSNSG--NTATLTISGTQAMDEADYYC | QAWVN------------------------STVV | FGGGTKLTVL |
| iPS:436906 | 21-225_72B4 | VL3\|3r/JL2 | | SYELTQP-PSVSVSPGQTASITC | SGD---KLGD-----KYAW | WYQQRPGQSPVLVIY | E--------DRKRPS | GIPERFSGSNSG--NTATLTISGTQAMDEADYYC | QAWDN------------------------STVV | FGGGTKLTVL |
| iPS:436908 | 21-225_72D5 | VL3\|3r/JL2 | | SYDLTQP-PSVSVSPGQTASITC | SGD---KLGN-----KYAC | WYQQKPGQSPVVVIY | Q--------DRKRPS | GIPERFSGSNSG--NTATLTISGTQAMDEADYYC | QAWDS------------------------STAV | FGGGTKLTVL |
| iPS:436912 | 21-225_73C4 | VL3\|3r/JL2 | | SYDLTQP-PSVSVSPGQTASITC | SGD---KLGN-----KYAC | WYQQKPGQSPVVVIY | Q--------DMKRPS | GIPERFSGSNSG--NTATLTISGTQAMDEADYYC | QAWDS------------------------STAV | FGGGTKLTVL |
| iPS:436914 | 21-225_76B4 | VL3\|3r/JL2 | | PYELNQT-PSVSVSPGQTASITC | SGD---RLGT-----KFAC | WYQQKPGQSPVLVIY | Q--------DSKRPS | GIPERFSGSNSG--NTATLTISGTQAMDEADYYC | QAWD-------------------------SSTV | FGGGTKLTVL |
| iPS:436916 | 21-225_74A9 | VL3\|3r/JL2 | | SYELTQP-PSVSVSPGQTASITC | SGD---KLGN-----KYVC | WYQQKPGQSPVLVIY | Q--------DNRRPS | GIPERFSGSNSG--NTATLTISGTQAMDEADYYC | QAWDS------------------------SPVI | FGGGTKLTVL |
| iPS:436918 | 21-225_77A2 | VL3\|3r/JL2 | | SYELTQP-PSVSVSPGQTASITC | SGD---RLGD-----KYAC | WYQQKPGQSPVLVIY | Q--------DRKRPS | GIPERFSGSNSG--NTATLTISGTQAMDEADYYC | QAWDS------------------------STAV | FGGGTKLTVL |
| iPS:436922 | 21-225_78E9 | VL3\|3r/JL2 | | SYELTQP-PSESVSPGQTASITC | SGD---KLGN-----KYVS | WYQQKPGQSPVLVIY | Q--------DNRRPS | GIPERFSGSNSG--STATLTISGTQAMDEADYYC | QAWDS------------------------SPVI | FGGGTKLTVL |
| iPS:436924 | 21-225_74B3 | VL3\|3r/JL2 | | SYELTQP-PSVSVSPGQTASITC | SGD---KLGD-----KYAC | WYQQKPGQSPVLVIY | Q--------DSKRPS | GIPERFSGSNSG--NTATLTISGTQAMDEADYYC | QAWDS------------------------TTVV | FGGGTKLTVL |
| iPS:436928 | 21-225_79E7 | VL3\|3r/JL2 | | SYELTQP-PSVSVSPGQTASITC | SGD---KLGN-----KYVS | WYQQKPGQSPVLVIY | Q--------DNRRPS | GIPERFSGSNSG--STATLTISGTQAMDEADYYC | QAWDS------------------------SPVI | FGGGTKLTVL |
| iPS:436932 | 21-225_92A4 | VL3\|3r/JL2 | | SYELTQP-PSVSVSPGQTANITC | SGD---KLGN-----KYVC | WYQQKPGQSPVLVIY | Q--------DNRRPS | GIPERFSGSNSG--NTATLTISGTQAMDEADYYC | QAWDS------------------------SPVI | FGGGTKLTVL |
| iPS:436934 | 21-225_96B5 | VL3\|3r/JL2 | | PYELNQT-PSVSVSPGQTASITC | SGD---RLGT-----KFAC | WYQQKPGQSPVLVIY | Q--------DSKRPS | GIPERFSGSNSG--NTATLTISGTQAMDEADYYC | QAWD-------------------------SSTV | FGGGTKLTVL |
| iPS:436936 | 21-225_97E6 | VL3\|3r/JL2 | | SYELTQP-PSVSVSPGQTVSITC | SGD---KLGN-----KYVS | WYQQKPGQSPVLVIY | Q--------DNRRPS | GIPERFSGSNSG--STATLTISGTQAMDEADYYC | QAWDS------------------------TPVI | FGGGTKLTVL |
| iPS:436938 | 21-225_146A3 | VL3\|3r/JL2 | | SYAMTQP-PSMSVSPGQTASITC | SGN---KLGN-----RYAC | WYQQKPGQSPVLVIY | Q--------DSKRPS | GIPERFSGSNSG--NIATLTISGTQAMDEADYYC | QAWDS------------------------STVV | FGGGTKLTVL |
| iPS:436940 | 21-225_146B8 | VL3\|3r/JL2 | | SYELTQP-PSVSVSPGQTASI | SGD---KLGD-----KYVC | WYQQKPGQSPVLVIY | Q--------DRKRPS | GIPERFSGSNSG--NTATLTISGTQAMDEADYYC | QAWHS------------------------STVV | FGGGTKLTVL |

| | | | | | | | | | | |
|---|---|---|---|---|---|---|---|---|---|---|
| **iPS:436942** | **21-225_146H8** | **VL3\|3r/JL2** | | SYELTQP-PSVSVSPGQTASITC | SGD---KLGD-----KYAC | WYQQKPGQSPVLVIY | Q--------DKKRPS | GIPERFSGSNSG--NTATLTISGTQVMDEADYYC | QAWDI-----------------------RTVV | FGGGTKLTVL |
| **iPS:436944** | **21-225_182D12** | **VL3\|3r/JL2** | | SYELTQP-PSVSVSPGQTASITC | SGD---KLGE-----KYAC | WYQQKSGQSPVLVIY | Q--------DRKRPS | GIPERFSGSNSG--NTATLTISGTQAMDEADYYC | QAWDS-----------------------RTAV | FGGGTKLTVL |
| **iPS:436946** | **21-225_183F4** | **VL3\|3r/JL2** | | SYELTQP-PSVSVSPGQTASITC | SGD---KLGD-----KYAC | WYQQKPGQSPVLVIY | Q--------DKKRPS | GIPERFSGSNSG--NTATLTISGTQAMDEADYYC | QAWDN----------------------STAVV | FGGGTKLTVL |
| **iPS:436952** | **21-225_185D2** | **VL3\|3r/JL2** | | SYELTQT-PSVSVSPGQTASITC | SGD---KLGD-----KYAC | WYQQKPGQSPVLVIY | E--------DRKRPS | GIPDRFSGSNSG--NTATLTISGTQAMDEADYYC | QAWDS-----------------------RKVV | FGGGTKLTVL |
| **iPS:436954** | **21-225_185G7** | **VL3\|3r/JL2** | | SYELTQP-PSVSVSPGQTASITC | SGD---KLGH-----KFVC | WYQQKPGQSPVLVIY | Q--------DRKRPS | GIPERFSGSNSG--NTATLTISGTQAMDEADYYC | QAWD------------------------SSTV | FGGGTKLTVL |
| **iPS:436956** | **21-225_186H6** | **VL3\|3r/JL2** | | SYELTQP-PSVSVSPGQTASITC | SGD---KMGE-----KYAC | WYQQKPGQSPVLVIY | Q--------DRKRPS | GIPERFSGSNSG--NTATLTISGTQAMDEADYYC | QAWDS-----------------------STAV | FGGGTKLTVL |
| **iPS:436962** | **21-225_190H1** | **VL3\|3r/JL2** | | SYELTQP-PSVSVSPGQTASITC | SGD---KLGD-----RFAY | WYQQKPGQSPVLVIY | Q--------DSKRPS | GIPERFSGSNSG--NTATLTISGTQAMDEADYYC | KAWDS-----------------------STVV | FGGGTKLTVL |
| **iPS:436978** | **21-225_190G9** | **VL3\|3r/JL2** | | SYELTQP-PSVSVSPGQTASITC | SGD---KLGD-----RFAY | WYQQKPGQSPVLVIY | Q--------DNKRPS | GIPERFSGSNSG--NTASLTISGTQAMDEADYYC | QAWDS-----------------------STVV | FGGGTRLTVL |
| **iPS:437018** | **21-225_193H5** | **VL3\|3r/JL2** | | SYELTQP-SSVSVSPGQTASITC | SGD---KLGD-----RFAC | WYQQKPGQSPVLVIY | Q--------DSKRPS | GIPERFSGSNSG--NTATLTISGTQAMDEADYYC | QAWDS-----------------------STAV | FGGGTKLTVL |
| **iPS:437030** | **21-225_195E3** | **VL3\|3r/JL2** | | SYELTQP-PSVSVSPGQTASITC | SGD---KLGY-----RSVC | WYQQKPGQSPVLVIY | E--------DSKRPS | GIPERFSGSNSG--NTATLTISGTQAMDEADYYC | QAWDS-----------------------VTVV | FGGGTKLIVL |
| **iPS:437034** | **21-225_195E9** | **VL3\|3r/JL2** | | SYELTQP-PSVSVSPGQTASITC | SGD---KLGN-----KYAY | WYQQKPGQSPVLVIY | Q--------DRKRPS | GIPERFSGSNSG--NTGTLTISGTQGMDEADYYC | QAWDR-----------------------GIVV | FGGGTKLTVL |
| **iPS:437070** | **21-225_201G11** | **VL3\|3r/JL2** | | SYELTQP-PSVSVSPGQTASITC | SGD---KLGD-----RFAC | WYQQKPGQSPVLVIY | Q--------DSKRPS | GIPERFSGSNSG--NTATLTISGTQAMDEADYYC | QAWDS-----------------------STVV | FGGGTKLTVL |
| **iPS:437076** | **21-225_203G6** | **VL3\|3r/JL2** | | SYELTQP-PSVSVSPGQTASITC | SGD---KLGD-----RFAC | WYQQKPGQSPVLVIY | Q--------DNKRPS | GIPERFSGSNSG--NTATLTISGTQSMDEADYYC | QAWDS-----------------------STVV | FGGGTKLTVL |
| **iPS:437144** | **21-225_215B3** | **VL3\|3r/JL2** | | SYELTQP-PSVSVSPGQTASITC | SGD---KLGD-----KFAC | WYQQKPGQSPVLVIY | Q--------DSKRPS | GIPERFSGSNSG--NTATLTISGTQAMDEADYYC | QAWDS-----------------------STVV | FGGGTKLTVL |
| **iPS:437186** | **21-225_224H2** | **VL3\|3r/JL2** | | SYELTQP-SSVSVSPGQTASITC | SGD---NLGV-----KYTY | WYQQKPGQSPVLVVY | Q--------DSKRPS | GIPERFSGSNSG--NTATLTISGTQAMDEADYYC | QAWDS-----------------------STVV | FGGGTKLTVL |
| **iPS:437192** | **21-225_225E9** | **VL3\|3r/JL2** | | SYDLTQP-PSVSVSPGQTASI | SGD---NLGN-----RYAC | WYQQKPGQSPVLVMY | Q--------DRKRPS | GIPERFSGSNSG--NTATLTISGTQAMDEADYYC | QAWDS----------------------RTAVV | FGGGTKLTVL |

| | | | | | | | | | | |
|---|---|---|---|---|---|---|---|---|---|---|
| **iPS:4 37194** | **21-225_226B2** | **VL3\|3r/JL 2** | | SYELTQP- PSVSVSPGQTASI TC | SGD---TLGG- ----KYAW | WYQQRPGQ SPVLVIY | Q-------- DRKRPS | GIPERFSGSSSG-- NTATLTISGTQAMDEADYYC | QAWDN----------- ------------GAAV | FGGGT KLTVL |
| **iPS:4 37200** | **21-225_226A10** | **VL3\|3r/JL 2** | | SYELTQP- PSVSVSPGQTASI TC | SGD---TLGG- ----KYAW | WYQQKPGQ SPVLVIY | Q-------- DRKRPS | GIPERFSGSSSG-- NTATLTISGTQAMDEADYYC | QAWDN----------- ------------GAAV | FGGGT KLTVL |
| **iPS:4 37204** | **21-225_227E5** | **VL3\|3r/JL 2** | | SYELSQP- PSVSVSPGQTASI TC | SGD---KLGE- ----KYAC | WYQQKPGQ SPVLVIY | Q-------- DRKRPS | GIPERFSGSNSG-- NKATLTISGTQAMDEADYYC | QAWVN----------- ------------NTMI | FGGGT KLTVL |
| **iPS:4 37210** | **21-225_227E12** | **VL3\|3r/JL 2** | | SYELTQP- PSVSVSPGQTASI TC | SGD---KLGD- ----KYVC | WYQQKPGQ SPVLVIY | Q-------- DSKRPS | GIPERFSGSNSG-- NTATLTISGTQAMDEADYYC | QAWNS----------- ------------SNVV | FGGGT KLTVL |
| **iPS:4 48908** | **21-225_50G9** | **VL3\|3r/JL 2** | | SYELTQP- PSVSVSPGQTASI TC | SGD---KLGD- ----KYAC | WYQQKPGQ SPVLVIY | Q-------- DSKRPS | GIPERFSGSNSG-- NTATLTISGTQARDEAEYYC | QARNS----------- ------------RRGV | FGGGT RLTVL |
| **iPS:4 51102** | **21-225_45F6** | **VL3\|3r/JL 2** | | SYELTQP- PSVSVSPGQTASI TC | SGD---KLGD- ----KYAS | WYQQKPGQ SPVLVIY | Q-------- DSKRPS | GIPERFSGSNSG-- NTATLTISGTQAMDEADYYC | QAWDN----------- ------------RTMV | FGGGT KLTVL |
| **iPS:4 51110** | **21-225_74C9** | **VL3\|3r/JL 2** | | SYELTQP- PSESVSPGQTASI TC | SGD---KSGN- ----KYVS | WYQQKPGQ SPVLVIY | Q-------- DNRRPS | GIPERFSGSNSG-- STATLTISGTQAMDEADYYC | QAWDS----------- ------------TPVI | FGGGT KLTVL |
| **iPS:4 51112** | **21-225_53D10** | **VL3\|3r/JL 2** | | SYELTQP- PSVSVSPGQTASI TC | SGD---KLGN- ----KYAC | WYQQRPGQ SPVLVIY | Q-------- DRKRPS | GIPERFSGSNSG-- NTATLTISGTQAMDEADYYC | QAWDS----------- ------------STVV | FGGGT KLTVL |
| **iPS:4 72731** | **21-225_14B1_LC 2** | **VL3\|3r/JL 2** | | SFELTQP- PSVSVSPGQTASI TC | SGD---KLGD- ----KYAY | WYQQKPGQ SPVLVIY | Q-------- DRKRPS | GIPERFSGSNSG-- NTATLTISGTQAMDEADYYC | QAWDN----------- ------------STVV | FGGGT KLTVL |
| **iPS:3 92583** | **21-225_10B10** | **VL3\|3r/JL 2** | | SYELTQP- PSVSVSPGQTASI TC | SGD---KLGN- ----KYAW | WYQQKPGQ SPVLVIY | Q-------- DRKRPS | GIPERFSGSNSG-- NTATLTISGTQAMDEADYYC | QAWDN----------- ------------STVV | FGGGT KLTVL |
| **iPS:3 92585** | **21-225_14H11** | **VL3\|3r/JL 2** | | TYELTQP- SSVSVSPGQTASI TC | SGD---KLGE- ----KYVC | WYQQKPGQ SPVLVIY | Q-------- DTKRPS | GIPERFSGSNSG-- NTATLTISGTQAMDEADYYC | QAWD------------ ------------SSTI | FGGGT KLTVL |
| **iPS:3 92587** | **21-225_18G5** | **VL3\|3r/JL 2** | | SYELTQP- PSVSVSPGQTASI TC | SGE---KLGD- ----KYVC | WYQQKPGQ SPVLVIY | Q-------- DSKRPS | GIPERFSGSNSG-- NTATLTISGTQAMDEADYYC | QAWNS----------- ------------SNVV | FGGGT KLTVL |
| **iPS:3 92589** | **21-225_27H2** | **VL3\|3r/JL 2** | | SYELTQP- PSVSVSPGQTASI TC | SGD---KLGD- ----KYAS | WYQQKPGQ SPVLVIY | Q-------- DGKRPS | GIPERFSGSNSG-- NTATLTLSGTQAMDEADYYC | QAWDS----------- -----------STYVV | FGGGT KLTVL |
| **iPS:3 92598** | **21-225_18E10** | **VL3\|3r/JL 2** | | SYELTQP- PSVSVSPGQTASI TC | SGD---RLGD- ----KYAW | WYQQKPGQ SPVLVIY | Q-------- DRKRPS | GIPERFSGSNSG-- NTATLTISGTQAMDEADYYC | QAWDS----------- ------------STVV | FGGGT KLTVL |
| **iPS:3 93166** | **21-225_27G6** | **VL3\|3r/JL 2** | | SYELTQP- PSVSVSPGQTASI TC | SGD---KLGD- ----KYAC | WYQQKPGQ SPVLVIY | Q-------- DRKRPS | GIPERFSGSNSG-- NTATLTISGTQTMDEADYYC | QAWDS----------- -----------SSYVV | FGGGT KLTVL |
| **iPS:3 93168** | **21-225_32B11** | **VL3\|3r/JL 2** | | SYELTQP- PSVSVSPGQTASI | SGD---KLGD- ----KYAY | WYQQKPGQ SPVLVIY | Q-------- DSKRSS | GIPERFSGSNSG-- NTATLTISGTQAMDEADYYC | QAWDN----------- ------------STVV | FGGGT KLTVL |

| | | | | | | | | | | |
|---|---|---|---|---|---|---|---|---|---|---|
| **iPS:3 93172** | **21-225_3B12** | **VL3\|3r/JL 2** | | SYELSQP-PSVSVSPGQTASI TC | SGD---KLGE-----KYAC | WYQQKPGQ SPVLVIY | Q--------DRKRPS | GIPERFSGSNSG--NKATLTISGTQAMDEADYYC | QAWVN-----------------------NTMI | FGGGT KLTVL |
| **iPS:3 93176** | **21-225_27E7** | **VL3\|3r/JL 2** | | SYELTQP-PSVSVSPGQTASI TC | SGD---KLGN-----KYAC | WYQQKPGQ SPVEVIY | Q--------DSKRPL | GIPERFSGSNSG--NTATLTISGTQAMDEADYYC | QAWDS-----------------------STVV | FGGGT KLTVL |
| **iPS:3 93178** | **21-225_34D7** | **VL3\|3r/JL 2** | | SYELTQP-PSVSVSPGQTASI TC | SGD---KLGE-----KYAY | WYQQKPGQ SPVLVLY | Q--------DSKRPS | GIPERFSGSNSG--NTATLTISGTQTMDEADFYC | QAWDN-----------------------TTVV | FGGGT KLTVL |
| **iPS:3 93182** | **21-225_4B3** | **VL3\|3r/JL 2** | | SYELTQP-PSVSVSPRQTVSI TC | SGD---KLGD-----KYAC | WYQQKPGQ SPVLVIY | Q--------DRKRPS | GIPERFSGSNSG--NTATLTISGTQAMDEADYYC | QAWDN-----------------------NTVI | FGGGT KLTVL |
| **iPS:3 93184** | **21-225_15H11** | **VL3\|3r/JL 2** | | SYELTQP-PSVSVSPGQTASI TC | SGD---KLGE-----KYAC | WYQQKPGQ SPVVVIY | Q--------DRKRPS | GIPERFSGSNSG--NTATLTISGTQAIDEADYYC | QAWDS-----------------------STAV | FGGGT KLTVL |
| **iPS:3 93186** | **21-225_27D9** | **VL3\|3r/JL 2** | | SYELTQP-PSMSVSPGQTASI TC | SGY---KLGD-----KYAC | WFQQKPGQ SPVLVIY | Q--------DSKRPS | GIPERFSGSNSG--NTATLTISGTQAMDEADYYC | QAWV------------------------NNTV | FGGGT KLTVL |
| **iPS:3 93188** | **21-225_34B9** | **VL3\|3r/JL 2** | | SYELTQA-PSVSVSPGQTASI TC | SGD---KLGE-----KYVS | WYQEKPGQ SPVLVIY | Q--------DSKRPS | GIPERFSGSNSG--NTATLTISGTQAMDEADYYC | QAWD------------------------SSTV | FGGGT KLTVL |
| **iPS:3 93192** | **21-225_12B1** | **VL3\|3r/JL 2** | | SYELTQP-PSVSVSPRQTVSI TC | SGD---KLGD-----KYAC | WYQQKPGQ SPVLVIY | Q--------DRKRPS | GIPERFSGSNSG--NTATLTISGTQAMDEADYYC | QAWDN-----------------------NTVI | FGGGT KLTVL |
| **iPS:3 93194** | **21-225_16D2** | **VL3\|3r/JL 2** | | SYELTQP-PSVSVSPGQTASI TC | SGD---KLGD-----KYAC | WYQQKPGQ SPVLVIY | Q--------DSKRPS | GIPERFSGSNSG--NTATLTISGTQAMDEADYYC | QAWDS----------------------STYVV | FGGGT KLTVL |
| **iPS:3 93196** | **21-225_16G8** | **VL3\|3r/JL 2** | | SYELSQP-PSVSVSPGQTASI TC | SGD---KLGE-----KYAC | WYQQKPGQ SPVLVIY | Q--------DRKRPS | GIPERFSGSNSG--NKATLTISGTQAMDEADYYC | QAWVN-----------------------NTMI | FGGGT KLTVL |
| **iPS:3 93198** | **21-225_28A11** | **VL3\|3r/JL 2** | | SYELTQP-PSVSVSPGQTASI TC | SGD---KLGD-----KYAC | WYQQKPGQ SPVLVIY | Q--------DSKRPS | GIPERFSGSNSG--NTATLTISGTQAMDEADYYC | QAWDS----------------------STYVV | FGGGT KLTVL |
| **iPS:3 93200** | **21-225_35E1** | **VL3\|3r/JL 2** | | SYELTQP-PSVSVSPGQTASI TC | SGD---KLGE-----KYAY | WFQQKPGQ SPVIVIY | Q--------DRKRPS | GIPERFSGSNSG--NTATLTISGTQAMDEADYYC | QAWDN-----------------------STAV | FGGGT KLTVL |
| **iPS:3 93202** | **21-225_6B4** | **VL3\|3r/JL 2** | | SYELTQP-PSVSVSPRQTVSI TC | SGD---KLGD-----KYAC | WYQQKPGQ SPVLVIY | Q--------DRKRPS | GIPERFSGSNSG--NTATLTISGTQAMDEADYYC | QAWDN-----------------------NTVI | FGGGT KLTVL |
| **iPS:3 93206** | **21-225_13F6** | **VL3\|3r/JL 2** | | SYELTQP-PSVSVSPGQTASI TC | SGD---KLGD-----KYAC | WYQQKPGQ SPVLVIY | Q--------DSKRPS | GIPERFSGSNSG--NTATLTISGTQAMDEADYYC | QAWGN----------------------STAVV | FGGGT KLTVL |
| **iPS:3 93210** | **21-225_17D3** | **VL3\|3r/JL 2** | | SYELTQS-PSVSVSPGQTASI TC | SGD---KLGD-----KYVY | WYQQKPGQ SPVVVIY | Q--------DRKRPS | GIPERFSGSNSG--NTATLTISGTQAMDEADYYC | QAWDS-----------------------ITAV | FGGGT KLTVR |
| **iPS:3 93212** | **21-225_30H6** | **VL3\|3r/JL 2** | | SYELTQP-PSVSVSPGQTASI | SGD---KLGN-----KYAC | WYQQKPGQ SPVLVIY | Q--------DSKRPS | GIPERFSGSNSG--NTATLTISGTQAMDEADYYC | QAWD------------------------SSTV | FGGGT KLTVL |

| | | | | | | | | | | |
|---|---|---|---|---|---|---|---|---|---|---|
| **iPS:3 93214** | **21-225_33A1** | **VL3\|3r/JL 2** | | SYELTQP-PSVSVSPGQTASITC | SGD---KLGD-----KFVY | WYQQKPGQSPVLVIY | Q--------DSKRPS | GIPERFSGSNSG--NTATLTISGTQAMDEADYYC | QAWDS-----------------------TTVV | FGGGTKLTVL |
| **iPS:3 93218** | **21-225_14G3** | **VL3\|3r/JL 2** | | SYELTQP-PSVSVSPGQTASITC | SGD---KLGD-----KYVC | WYQQKPGQSPVLVIY | Q--------DRKRPS | GIPERFSGSNSG--NTATLTISGTQAMDEADYYC | QAWGN----------------------STAVV | FGGGTKLTVL |
| **iPS:3 93222** | **21-225_17F5** | **VL3\|3r/JL 2** | | SYELTQP-PSVSVSPGQTASITC | SGD---KLGE-----KYAC | WYQQKPGQSPVLVIY | Q--------DRKRPS | GIPERFSGSNSG--NTATLTISGTQAMDEADYYC | QAWD------------------------SSTV | FGGGTKLTVL |
| **iPS:3 93224** | **21-225_31C2** | **VL3\|3r/JL 2** | | SYELTQP-PSVSVSPGQTASITC | SGD---KLGN-----KYAC | WYQQKPGQSPVLVIY | Q--------DSKRPS | GIPERFSGSNSG--NTATLTISGTQAMDEADYYC | QAWD------------------------SSTV | FGGGTKLTVL |
| **iPS:3 93226** | **21-225_33E6** | **VL3\|3r/JL 2** | | SYELTQP-PSVSVSPGQTASITC | SGD---KLGD-----KYAY | WFQQKPGQSPVIVIY | Q--------DRKRPS | GIPERFSGSNSG--NTATLTISGTQAMDEADYYC | QAWDN-----------------------STAV | FGGGTKLTVL |
| **iPS:3 93234** | **21-225_26C10** | **VL3\|3r/JL 2** | | SYEVTQP-PSMSVSPGQTASITC | SGD---KLGD-----KYVC | WFQQKPGQSPVLVIY | Q--------DSKRPS | GIPERFSGSNSG--NTATLTISGTQAMDEADYYC | QAWV------------------------NNTV | FGGGTKLTVL |
| **iPS:3 93345** | **21-225_5G7** | **VL3\|3r/JL 2** | | SYELTQP-PSVSVSPGQTASITC | SGD---KLGN-----KYAW | WYQQKPGQSPVLVIY | Q--------DRKRPS | GIPERFSGSNSG--NTATLTISGTQAMDEADYYC | QAWDN-----------------------STVV | FGGGTKLTVL |
| **iPS:3 93565** | **21-225_34B11** | **VL3\|3r/JL 2** | | SYELTQP-PSVSVSPGQTASITC | SGD---KLGD-----KYAC | WYQQKPGQSPVVVIY | Q--------DMKRPS | GIPERFSGSNSG--NTATLTISGTQAMDEADYYC | QAWDN-----------------------STAV | FGGGTKLTVL |
| **iPS:3 93950** | **21-225_3H10** | **VL3\|3r/JL 2** | | SYELSQP-PSVSVSPGQTASITC | SGD---KLGE-----KYAC | WYQQKPGQSPVLVIY | Q--------DRKRPS | GIPERFSGSNSG--NKATLTISGTQAMDEADYYC | QAWVN-----------------------NTMI | FGGGTKLTVL |
| **iPS:3 98470** | **21-225_14B7** | **VL3\|3r/JL 2** | | SYELTQP-PSVSVSPGRTASITC | SGD---KLGN-----KYAY | WYQQKPGQSPVLVIY | Q--------DRKRPS | GIPERFSGSNSG--NTATLTISGTQTMDEADYYC | QAWNN-----------------------STVV | FGGGTKLTVL |
| **iPS:3 98472** | **21-225_16E4** | **VL3\|3r/JL 2** | | PYELTQP-PSVSVSPGQTASITC | SGD---KLGD-----KYVY | WYQQKSGQSPVLVIY | Q--------DSKRPS | GIPERFSGSNSG--NTAALTISGTQAMDEADYYC | QAWDS-----------------------STVV | FGGGTKLTVL |
| **iPS:3 98488** | **21-225_19F6** | **VL3\|3r/JL 2** | | SYELTQP-PSVSVSPGQTVSITC | SGD---KLGD-----KYAC | WYQQKPGQSPVLVIY | Q--------DSKRPS | GTPERFSGSNSG--NTATLTISGTQAMDEADYYC | QAWDN-----------------------NTVV | FGGGTKLTVL |
| **iPS:3 98490** | **21-225_21D12** | **VL3\|3r/JL 2** | | SYELTQP-PSVSVSPGQTASITC | SGD---KLGN-----KYAY | WYQQKPGQSPVLVIY | Q--------DRKRPS | GIPERFSGSNSG--NTATLTISGTQAMDEADFYC | QAWDN-----------------------STVV | FGGGTRLTVL |
| **iPS:3 98498** | **21-225_22E6** | **VL3\|3r/JL 2** | | SYELTQP-PSVSVSPGQTASITC | SGD---KLGE-----KYAC | WYQQKPGQSPVLVIY | Q--------DRKRPS | GIPERYSGSNTG--NTATLTISGTQAMDEADYYC | QAWDS-----------------------STAV | FGGGTKLTVL |
| **iPS:3 98504** | **21-225_23D7** | **VL3\|3r/JL 2** | | SYELTQP-PSVSVSPGQTASITC | SGE---KLGD-----KYVC | WYQQKPGQSPVVVIY | Q--------DSKRPS | GIPERFSGSNSG--NTATLTISGTQAMDEADYYC | QAWNS-----------------------SNVV | FGGGTKLTVL |
| **iPS:3 [illegible]** | **21-225_9H10** | **VL3\|3r/JL 2** | | SYELTQP-PSVSVSPGQTASI | SGD---KLGD-----KYAC | WYQQKPGQSPVLVIY | Q--------DSKRPS | GIPERFSGSNSG--NTATLTISGTQAMDEADYYC | QAWDS----------------------[illegible] | FGGGTKLTVL |

| | | | | | | | | | | |
|---|---|---|---|---|---|---|---|---|---|---|
| **iPS:402225** | **21-225_2B1** | **VL3\|3r/JL2** | | SYELTQP-PSVSVSPGQTVSITC | SGD---KLGD-----KYAC | WYQQKPGQSPVLVIY | Q--------DRKRPS | GIPERFSGSNSG--NTATLTISGTQAMDEADYYC | QAWDN-----------------------NTVV | FGGGTKLTVL |
| **iPS:402231** | **21-225_6D9** | **VL3\|3r/JL2** | | SYELTQP-PSVSVSPGQTASITC | SGD---KLGE-----KYAC | WYQQKPGQSPVLVIY | Q--------DKKRPS | GIPERFSGSNSG--NTATLTISGTQAMDEADYYC | QAWD------------------------SSTV | FGGGTKLTVL |
| **iPS:404090** | **21-225_8D8** | **VL3\|3r/JL2** | | SYELTQP-PSVSVSPGQTASITC | SGD---KLGE-----KYAC | WYQQKPGQSPVVVIY | Q--------DRKRPS | GIPERFSGSNSG--NTATLTISGTQAIDEADYYC | QAWDS-----------------------STAV | FGGGTKLTVL |
| **iPS:423018** | **21-225_31D12_LC2** | **VL3\|3r/JL2** | | SYELTQP-PSVSVSPGQTASITC | SGD---KLGD-----KYAY | WFQQKPGQSPVIVIY | Q--------DRKRPS | GIPERFSGSNSG--NTATLTISGTQAMDEADYYC | QAWDN-----------------------STAV | FGGGTKLTVL |
| | | **Germline** | | **L_FR1** | **L_CDR1** | **L_FR2** | **L_CDR2** | **L_FR3** | **L_CDR3** | **L_FR4** |
| | **VL2\|2a2/JL3b** | | 32185 | QSALTQP-ASVSGSPGQSITISC | TGTS-SDVGGY----NYVS | WYQQHPGKAPKLMIY | E--------VSNRPS | GVSNRFSGSKSG--NTASLTISGLQAEDEADYYC | SSYTSS----------------------SILWV | FGGGTKLTVL |
| **iPS:468862** | **21-225_178H8** | **VL2\|2a2/JL3b** | | QSALTQS-ASVSGSPGQSITISC | TGTS-SDVGGY----NFVS | WYQQHPGKVPKFMIY | E--------VSNRPS | GVPNRFSGSKSG--NTASLTISGLQAEDEADYYC | SSYTS-----------------------SYTWV | FGGGTKLTVL |
| **iPS:436838** | **21-225_52H4** | **VL2\|2a2/JL3b** | | QSALTQP-ASVSGSPGQSITISC | TGTS-SDVGGY----NYVS | WYQQHPGKAPKLMIY | E--------VSNRPS | GVSNRFSGSKSG--NTASLTISGLQAEDEADYYC | NSYTS-----------------------NITWV | FGGGTKLTVL |
| **iPS:437094** | **21-225_210D12** | **VL2\|2a2/JL3b** | | QSALTQP-ASVSGSPGQSITISC | TGTS-SDVGGY----NYVS | WYQQHPVKAPKLLIY | E--------VSNRPS | GVSNRFSGSKSG--NTASLTISGLQAEDEADYYC | NSYTS-----------------------SITWV | FGGGTKLTVL |
| **iPS:437096** | **21-225_210E12** | **VL2\|2a2/JL3b** | | QSALTQP-ASVSGSPGQSITISC | TGTS-SDVGGY----NYVS | WYQQHPGKAPKLMIY | E--------VSNRPS | GVSNRFSGSKSG--NTASLTISGLQAEDEADYYC | GSYVK-----------------------GITWV | FGGGTSLTVL |
| **iPS:437098** | **21-225_211C1** | **VL2\|2a2/JL3b** | | QSALTQP-ASVSGSPGQSITISC | TGTS-SDVGSY----NYVS | WYQQYPGKAPKLMIY | E--------VSNRPS | GVSNRFSGSKSG--NTASLTISGLQAEDEADYYC | NSYTS-----------------------SITWV | FGGGTKLTVL |
| **iPS:437104** | **21-225_211G5** | **VL2\|2a2/JL3b** | | QSALTQP-ASVSGSPGQSITISC | TGTS-SDVGGY----NYVS | WYQQHPGKAPKLMIY | E--------VSNRPS | GVSNRFSGSKSG--NTASLTISGLQAEDEADYYC | NSYTR-----------------------SITWV | FGGGTKLTVL |
| **iPS:437112** | **21-225_212C2** | **VL2\|2a2/JL3b** | | QSALTQP-ASVSGSPGQSITISC | TGTS-SDVGGY----NYVS | WYQQHPGKAPKLMIY | E--------VRNRPS | GVSNRFSGSKSG--NTASLTISGLQAEDEADYYC | SSYTR-----------------------SITWV | FGGGTKLTVL |
| **iPS:437114** | **21-225_212A4** | **VL2\|2a2/JL3b** | | QSALTQP-ASVSGSPGQSITISC | TGTS-SDVGGY----NYVS | WYQQHPGKAPKLMIY | E--------VSNRPS | GVSNRFSGSKSG--NTASLTISGLQAEDEADYYC | GSYVK-----------------------GITWV | FGGGTSLTVL |
| **iPS:437116** | **21-225_212F6** | **VL2\|2a2/JL3b** | | QSALTQP-ASVSGSPGQSITISC | TGTS-SDVGGY----NYVS | WYQQYPGKAPKLMIY | E--------VSNRPS | GVSNRFSGSKSG--NTASLTISGLQAEDEADYYC | NSYTS-----------------------SITWV | FGGGTKLTVL |
| **iPS:437118** | **21-225_212G7** | **VL2\|2a2/JL3b** | | QSALTQP-ASVSGSPGQSITISC | TGTS-SDVGGY----NYVS | WYQQHPGKTPKLMIY | E--------VSNRPS | GVSNRFSGSKSG--NTASLTISGLQAEDEADYYC | NSYTS-----------------------SITWV | FGGGTKLTVL |

| | | | | | | | | | | |
|---|---|---|---|---|---|---|---|---|---|---|
| iPS:4 37128 | 21-225_213G3 | VL2\|2a2/JL3b | | LSALTQP-ASVSGSPGQSITISC | TGTS-SDVGGY----NYVS | WYQQHPGKAPKLMIS | E--------VRNRPS | GVSNRFSGSKSG--NTASLTISGLQAEDEADYYC | NSYTR----------------------SITWV | FGGGTKLTVL |
| iPS:4 37130 | 21-225_213D5 | VL2\|2a2/JL3b | | QSALTQP-ASVSGSPGQSITISC | TGTS-SDVGGY----NYVS | WYQQHPGKAPKLVIY | E--------VRNRPS | GVSTRFSGSKSG--NKASLTISGLQAEDEADYYC | CSYTR----------------------RITWV | FGGGTKLTVL |
| iPS:4 37146 | 21-225_215D3 | VL2\|2a2/JL3b | | QSALTQP-ASVSGSPGQSITISC | TGTS-SDIGGY----NYVS | WYQQHPGKAPTLMIY | E--------VSNRPS | GVSNRFSGSKSG--NTASLTISGLQAEDEADYYC | NSYKR----------------------GSTWV | FGGGTKVTVL |
| iPS:4 37150 | 21-225_216A3 | VL2\|2a2/JL3b | | QSALTQP-ASVSGSPGQSITISC | TGTS-SDVGGY----NYVS | WYQQHPGKAPKLMIY | E--------VSNRPS | GVSNRFSGSKSG--NTASLTISGLQAEDEADYYC | NSYTS----------------------SITWV | FGGGTKLTVL |
| iPS:4 37162 | 21-225_217B2 | VL2\|2a2/JL3b | | QSALTQP-ASVSGSPGQSITISC | TGTS-SDVGGY----NYVS | WYQQHPGKAPKLLIY | E--------VSNRPS | GVYNRFSGSKSG--NTASLTISGLQAEDEADYYC | GSYVK----------------------GITWV | FGGGTSLTVL |
| iPS:4 37172 | 21-225_219A7 | VL2\|2a2/JL3b | | QSALTQP-ASVSGSPGQSITISC | TGTS-SDVGGY----NYVS | WYQQHPGKAPKLMIY | E--------VRNRPS | GVSNRFSGSKSG--NTASLTISGLQAEDEADYYC | CSYTR----------------------SITWV | FGGGTKLTVL |
| iPS:4 37182 | 21-225_221H2 | VL2\|2a2/JL3b | | LSALTQP-ASVSGSPGQSITISC | TGTS-SDVGGY----NYVS | WYQQHPGKAPKLMIS | E--------VRNRPS | GVSNRFSGSKSG--NTASLTISGLQAEDEADYYC | NSYTR----------------------SITWV | FGGGTKLTVL |
| iPS:4 37184 | 21-225_221G4 | VL2\|2a2/JL3b | | QSALTQP-ASVSGSPGQSITISC | TGTS-SDVGGY----NYVS | WYQQHPGKAPKLMIY | E--------VRNRPS | GVSNRFSGSKSG--NTASLTISGLQAEDEADYYC | NSYTR----------------------SITWV | FGGGTKLTVL |
| | | Germline | | L_FR1 | L_CDR1 | L_FR2 | L_CDR2 | L_FR3 | L_CDR3 | L_FR4 |
| | VL1\|1c/JL2 | | 32186 | QSVLTQP-PSASGTPGQRVTISC | SGSS-SNIGS-----NTVN | WYQQLPGTAPKLLIY | S--------NNQRPS | GVPDRFSGSKSG--TSASLAISGLQSEDEADYYC | AAWDDS---------------------LNGVV | FGGGTKLTVL |
| iPS:4 68864 | 21-225_60D6 | VL1\|1c/JL2 | | QSVLTQP-PSASGTPGQRVTISC | SGSS-SNIGS-----NTVN | WYQQLPGTAPKLLIY | S--------NNQRPS | GVPDRFSGSKSG--TSASLAISGLQSEDEADYYC | AAWDD----------------------SLNGP | VGGGTKLTVL |
| iPS:4 36660 | 21-225_146D8 | VL1\|1c/JL2 | | QSVLTQP-PSTSGTPGQRVTISC | SGSS-SYIGS-----NTVD | WYQQLPGTAPKLLIY | S--------NNQRPS | GVPDRFSGSKSG--TSASLAISGLQSEDEADYYC | AAWDDS---------------------LNGVV | FGGGTKLTVL |
| iPS:4 36680 | 21-225_147H12 | VL1\|1c/JL2 | | QSVLTQP-PSASGTPGQRVTISC | SGSS-SNIGS-----YAVN | WYQQLPGTAPKLLIY | S--------NNHRPS | GVPDRFSGSKSG--TSASLAISGLQSEDEADYYC | EAWDDS---------------------LNGPV | FGGGTKLTVL |
| iPS:4 36682 | 21-225_146A8 | VL1\|1c/JL2 | | QSVLTQP-PSASGTPGQRVTISC | SGSS-SNIGS-----NSIN | WYQQLPRTAPKLLIY | S--------NDQRPS | GVPDRFSGSKSG--TSASLAISGLQSEDEADYYC | AAWDDS---------------------LNGVV | FGGGTKLTVL |
| iPS:4 36684 | 21-225_146B6 | VL1\|1c/JL2 | | QSVLTQP-PSASGTPGQRVTISC | SGSS-SNIGS-----NAVN | WYQQLPGTAPKLLIY | S--------NNQRPS | GVPDRFSGSKSG--TSASLAISGLQSEDEADYYC | AAWDDS---------------------LNGVV | FGGGTKLTVL |
| iPS:4 36696 | 21-225_149A1 | VL1\|1c/JL2 | | QSVLTQP-PSASGTPGQRVTISC | SGSS-SNIGS-----NAVN | WYQQLPGTAPKLLIY | S--------NNQRPS | GVPDRFSGSKSG--TSASLAISGLQSEDEADYYC | AAWDDS---------------------LNGVV | FGGGTKLTVL |

| | | | | | | | | | | |
|---|---|---|---|---|---|---|---|---|---|---|
| iPS:4 36712 | 21-225_150F9 | VL1\|1c/JL 2 | | QSVLTQP- PSASGTPGQRVTI SC | SGSS-SNIGS- ----NAVN | WYQQLPGT APKLLIY | S-------- NSQRPS | GVPDRFSGSKSG-- TSASLAISGLQSEDEADYFC | AAWDDS---------- -----------LNGVV | FGGGT KLTVL |
| iPS:4 36750 | 21-225_154G12 | VL1\|1c/JL 2 | | QSVLTQP- PSASGTPGQRVTI SC | SGSS-SNIGN- ----NAVS | WYQQLPGT APKLLIY | S-------- NDHRPS | GVPDRFSGSKSG-- TSASLAISGLQSEDEADYYC | AAWDDS---------- -----------LKGPV | FGGGT KLTVL |
| iPS:4 36762 | 21-225_156H2 | VL1\|1c/JL 2 | | QSVVTQP- PSASGTPGQRVTI SC | SGSS-SNIGS- ----NTVN | WYQQLPGT APKLLIY | S-------- SNQRPS | GVPDRLSGSKSG-- TSASLAISGLQSEDEADYYC | AAWDDS---------- -----------LNGVV | FGGGT KVTVL |
| iPS:4 37044 | 21-225_197F9 | VL1\|1c/JL 2 | | QSVLTQP- PSASGTPGQRVTI SC | SGSS-SNIGS- ----NTVN | WYQQLPGT APKLLIY | S-------- NNQRPS | GVPDRFSGSKSG-- TSASLAISGLQSEDEADYYC | AAWDDS---------- -----------MNGPV | FGGGT KLTVL |
| iPS:4 37060 | 21-225_199C3 | VL1\|1c/JL 2 | | QSVLTQP- PSASGTPGQRVTI SC | SGSS-SNIGS- ----NTVN | WYQQLPGT APKLLIY | S-------- NNQRPS | GVPDRFSGSKSG-- TSASLAISGLQSEDEADYYC | AAWDDS---------- -----------LNGPV | FGGGT KLTVL |
| iPS:3 93180 | 21-225_4G12 | VL1\|1c/JL 2 | | QSVLTQP- PSASGTPGQRVNM SC | SGTN-SNIGS- ----YTVN | WYQQLPGT APKLLIY | I-------- NNQRPS | GVPDRFSGSKSG-- TSASLAISGLQSEDEADYYC | AAWDDS---------- ----------LNGHVV | FGGRT KLTVL |
| iPS:3 93230 | 21-225_9G9 | VL1\|1c/JL 2 | | QSVLTQP- PSASGTPGQRVNM SC | SGTN-SNIGS- ----YTVN | WYQQLPGT APKLLIY | I-------- NNQRPS | GVPDRFSGSKSG-- TSASLAISGLQSEDEADYYC | AAWDDS---------- ----------LNGHVV | FGGRT KLTVL |
| | | **Germline** | | **L_FR1** | **L_CDR1** | **L_FR2** | **L_CDR2** | **L_FR3** | **L_CDR3** | **L_FR4** |
| | VL3\|3p/JL2 | | 32187 | SYELTQP- PSVSVSPGQTARI TC | SGD---ALPK- ----KYAY | WYQQKSGQ APVLVIY | E-------- DSKRPS | GIPERFSGSSSG-- TMATLTISGAQVEDEADYYC | YSTDSS---------- -----------GNHVV | FGGGT KLTVL |
| iPS:4 68866 | 21-225_190C1 | VL3\|3p/JL 2 | | SYELTQP- PSVSVSPGQTARI TC | TGD---AMPK- ----KYAY | WDQQKSGQ APVLVIS | E-------- DSKRPS | GIPERFSGSSSG-- TMAPLTISGAQVEDETDYDC | NSTDS----------- -----------SGNRV | FGGGT KLTVL |
| iPS:4 37214 | 21-225_48B12 | VL3\|3p/JL 2 | | SYELTQP- PSVSVSPGQTARI TC | SGD---ALPK- ----KYAY | WYQQKSGQ APVLVIY | E-------- DSKRPS | GIPERFSGSSSG-- TMATLTISGAQVEDEADYYC | NSTDSS---------- -----------GNHVV | FGGGT KLTVL |
| | | **Germline** | | **L_FR1** | **L_CDR1** | **L_FR2** | **L_CDR2** | **L_FR3** | **L_CDR3** | **L_FR4** |
| | VL1\|1c/JL3b | | 32188 | QSVLTQP- PSASGTPGQRVTI SC | SGSS-SNIGS- ----NTVN | WYQQLPGT APKLLIY | S-------- NNQRPS | GVPDRFSGSKSG-- TSASLAISGLQSEDEADYYC | AAWDDS---------- -----------LNGWV | FGGGT KLTVL |
| iPS:4 36234 | 21-225_51E3 | VL1\|1c/JL 3b | | QSVLTQP- PSASGTPGQRVTI SC | SGSN-SNIGS- ----NIVT | WYQQLPGT APKLLIY | S-------- NDQRPS | GVPDRFSGSKSG-- TSASLAISGLQSEDEADYYC | TAWDDS---------- -----------LNGWV | FGGGT TLTVL |
| iPS:4 36830 | 21-225_51F4 | VL1\|1c/JL 3b | | QSVLTQP- PSASGTPGQRVTI SC | SGSS-SNIGS- ----NIVT | WYQQLPGT APKLLIY | S-------- NDQRPS | GVPDRFSGSKSG-- TSASLAISGLQSEDETDYYC | TAWDDS---------- -----------LNGWV | FGGGT TLTVL |
| iPS:4 36834 | 21-225_52F1 | VL1\|1c/JL 3b | | QSVLTQP- PSASGTPGQRVTI SC | SGSS-SNIGS- ----NIVT | WYQQLPGT APKLLIY | S-------- NDQRPS | GVPDRFSGSKSG-- TSASLAISGLQSEDEADYYC | AAWDDS---------- -----------LNGWV | FGGGT TLTVL |
| iPS:4 36842 | 21-225_54E9 | VL1\|1c/JL 3b | | QSVLTQP- PSASGTPGQRVTI SC | SGSN-SNIGN- ----NIVT | WYQQLPGT APKLLIY | V-------- NDQRPS | GVPDRFSGSKSG-- TSASLAISGLQSEDEADYYC | AAWDDS---------- -----------LNGWV | FGGGT TLTVL |

| | | | | | | | | | | |
|---|---|---|---|---|---|---|---|---|---|---|
| iPS:4 36844 | 21-225_56G1 | VL1\|1c/JL 3b | | QSVLTQP- PSASGTPGQRVTI SC | SGSS-SNIGS- ----HIVT | WYQQLPGT APKLLIY | S-------- NDQRPS | GVPDRFSGSKSG-- TSASLAISGLQSEDEADYYC | AVWDDS---------- -----------LIGWV | FGGGT TLTVL |
| iPS:4 36846 | 21-225_56E3 | VL1\|1c/JL 3b | | QSVLTQP- PSASGTPGQRVTI SC | SGSS-SNIGS- ----NIVT | WYQQLPGT APKLLIY | S-------- NNQRPS | GVPDRFSGSKSG-- TSASLAISGLQSEDEADYCC | AAWDDS---------- -----------LNGWV | FGGGT TLTVL |
| iPS:4 37010 | 21-225_192G3 | VL1\|1c/JL 3b | | QSVLTQP- PSASGTPGQRVTM SC | SGSS-SNIGS- ----NTVN | WYQQFPGT APKLLIY | G-------- NKQRPS | RVPDRFSGSKSG-- TSASLAISGLQSEDETDYYC | AAWDDS---------- -----------LNGWV | FGGGT KLTVL |
| iPS:4 37032 | 21-225_195H6 | VL1\|1c/JL 3b | | QSVLTQP- PSASGTPGQRVTI SC | SGSS-SNIGS- ----HTVN | WYQQLPGT APKLLIY | N-------- NYQRPS | GVPDRFSGSKSG-- TSASLTISGLQSEDEADYYC | ATWDDS---------- -----------LSVWV | FGGGT KVTVL |
| iPS:4 51104 | 21-225_49C5 | VL1\|1c/JL 3b | | QSVLTQP- PSASGTPGQRVTI SC | SGSS-SNIGS- ----NIVT | WYQQLPGT APKLLIY | S-------- NDQRPS | GVPDRFSGSKSG-- TSASLAISGLQSEDEADYYC | TAWDDS---------- -----------LNGWV | FGGGT TLTVL |
| iPS:4 51106 | 21-225_49D10 | VL1\|1c/JL 3b | | QSVLTQP- PSASGTPGQRVTI SC | SGSN-SNIGS- ----NIVT | WYQQLPGT APKLLIY | S-------- NDQRPS | GVPDRFSGSKSG-- TSASLAISGLQSEDEADYYC | AAWDDS---------- -----------LNGWV | FGGGT TLTVL |
| iPS:4 51108 | 21-225_53E8 | VL1\|1c/JL 3b | | QSVLTQP- PSASGTPGQRVTI SC | SGSC-SNIGS- ----NIVT | WYQQLPGT APKLLIY | S-------- NDQRPS | GVPDRFSGSKSG-- TSASLAISGLQSEDEADYYC | TAWDDS---------- -----------LNDWV | FGGGT TLTVL |
| | | **Germline** | | **L_FR1** | **L_CDR1** | **L_FR2** | **L_CDR2** | **L_FR3** | **L_CDR3** | **L_FR4** |
| | VL3\|3r/JL1 | | 32189 | SYELTQP- PSVSVSPGQTASI TC | SGD---KLGD- ----KYAC | WYQQKPGQ SPVLVIY | Q-------- DSKRPS | GIPERFSGSNSG-- NTATLTISGTQAMDEADYYC | QAWDS----------- ------------STAYV | FGTGT KVTVL |
| iPS:4 36662 | 21-225_147D7 | VL3\|3r/JL 1 | | SYELTQP- PSVSVSPGQTASI TC | SGD---KLGD- ----KFAC | WYQQKPGQ SPVLVIY | Q-------- DRKRPS | GIPERFSGSNSG-- NTATLTISGTQAMDEADYYC | QAWDR----------- ------------NTAV | FGTGT KVTVL |
| iPS:4 36720 | 21-225_151H6 | VL3\|3r/JL 1 | | SYELTQP- PSVSVSPGQTASI TC | SGD---KLGD- ----KYAC | WYQQKPGQ SPVLVIY | Q-------- DTKRPS | GIPERFSGSNSG-- NTATLTISGTQAMDEADYYC | QAWDS----------- ------------STYV | FGTGT KVTVL |
| iPS:4 36726 | 21-225_152G5 | VL3\|3r/JL 1 | | SYEMTQP- PSVSVSPGQTAII TC | SGD---KLGD- ----KYAC | WYQQKPGQ SPVLVIY | Q-------- DSKRPS | GIPERFSGSNSG-- NTATLTISGTQAMDEADYYC | QAWDS----------- ------------STYV | FGTGT KVTVL |
| iPS:4 36732 | 21-225_152B12 | VL3\|3r/JL 1 | | SYELTQP- PSVSVSPGQTASI TC | SGD---KLGN- ----KYAC | WYQQKPGQ SPVLVIY | Q-------- DTKRPS | GIPERFSGSNSG-- NTATLTISGTQAMDEADYYC | QAWDS----------- ------------STYV | FGTGT KVTVL |
| iPS:4 36734 | 21-225_153A8 | VL3\|3r/JL 1 | | SYELTQP- PSVSVSPGQTASI TC | SGD---KLGD- ----KYAC | WYQQKPGQ SPVLVIY | Q-------- DTKRPS | GIPERFSGSNSG-- NTATLTISGTQAMDEADYYC | QAWDS----------- ------------STYV | FGTGT KVTVL |
| iPS:4 36754 | 21-225_155G3 | VL3\|3r/JL 1 | | SYELTQP- PSVSVSPGQTASI TC | SGD---KLGD- ----KYVC | WYQQKPGQ SPMLVIY | Q-------- DSKRPS | GIPERFSGSNSG-- NTATLTISGTQAMDEADYYC | QAWDN----------- ------------SIYV | FGTGT KVTVL |
| iPS:4 37190 | 21-225_225A9 | VL3\|3r/JL 1 | | SYELTQP- PSVSVSPGQTASI TC | SGD---KLGN- ----KYAC | WYQQKPGQ SPVLVIY | Q-------- DSKRPS | GIPERFSGSNSG-- NTATLTISGTQAMDEADYYC | QAWDS----------- -----------NTACV | FGTGT KVTVL |
| | | **Germline** | | **L_FR1** | **L_CDR1** | **L_FR2** | **L_CDR2** | **L_FR3** | **L_CDR3** | **L_FR4** |

| | | | | | | | | | | |
|---|---|---|---|---|---|---|---|---|---|---|
| | VL5\|5c/JL3b | | 32130 | QAVLTQP-ASLSASPGASASLTC | TLRS-GINVGT----YRIY | WYQQKPGSPPQYLLR | YKS----DSDKQQGS | GVPSRFSGSKDASANAGILLISGLQSEDEADYYC | MIWHS-----------------------SASWV | FGGGTKLTVL |
| iPS:4 36702 | 21-225_149E8 | VL5\|5c/JL3b | | QAVSTQP-SSLSASPGASASLTC | TLRS-GITVTT----YRIY | WYQQKPGSPPQFLLR | YTS----DSDKHQGS | GVPSRFSGSKDASANAGILFISGLQSEDEADYYC | MIWHS-----------------------SAWV | FGGGTKLTVL |
| | | Germline | | L_FR1 | L_CDR1 | L_FR2 | L_CDR2 | L_FR3 | L_CDR3 | L_FR4 |
| | VL1\|1e/JL2 | | 32131 | QSVLTQP-PSVSGAPGQRVTISC | TGSS-SNIGAG----YDVH | WYQQLPGTAPKLLIY | G--------NSNRPS | GVPDRFSGSKSG--TSASLAITGLQAEDEADYYC | QSYDSS---------------------LSGVV | FGGGTKLTVL |
| iPS:4 36752 | 21-225_155H1 | VL1\|1e/JL2 | | QSVLTQP-PSVSGAPGQRVTISC | TGSS-SNIGAG----YDVH | WYQQLPGTAPKLLIY | G--------NSNRPS | GVPDRFSGSKSG--TSASLAITGLQAEDEADYHC | QSYDSS--------------------LSGPVI | FGGGTKLTVL |
| iPS:4 36820 | 21-225_179D10 | VL1\|1e/JL2 | | QSVLTQP-PSVSGAPGQRVTISC | TGSS-SNFGTD----YDVH | WYQQFPGTAPKLLIY | G--------HSNRPS | GVPDRFSGSKSG--TSASLAITGLQAEDEADYYC | QSYDR----------------------SLNVV | FGGGTKLTVL |
| iPS:4 37188 | 21-225_224B11 | VL1\|1e/JL2 | | QSVLTQP-PSVSGAPGQRVTISC | TGSS-SNIGAG----YDVH | WYQQLPGTAPKLLIF | G--------NSNRPS | GVPDRFSGSKSG--TSASLAITGLQAEDEADYHC | QSYDN----------------------SLSGV | FGGGTKLTVL |
| iPS:4 37198 | 21-225_226F8 | VL1\|1e/JL2 | | QSVLTQP-PSVSGAPGQRVTISC | TGSS-SNIGAG----YDVH | WYQQLPGTAPKLLIY | G--------NSNRPS | GVPDRFSGSKSD--TSASLAITGLQAEDEADYYC | QSYDN----------------------SLSGV | FGGGTKLTVL |
| iPS:4 37202 | 21-225_227D3 | VL1\|1e/JL2 | | QSVLTQP-PSVSGAPGQRVTISC | TGSS-SNIGAG----YDVH | WYQQLPGTAPKLLIY | G--------NSNRPS | GVPDRFSGSKSD--TSASLAITGLQAEDEADYYC | QSYDN----------------------SLSGV | FGGGTKLTVL |
| iPS:4 37208 | 21-225_227C10 | VL1\|1e/JL2 | | QSVLTQP-PSVSGAPGQRVTISC | TGSS-SNIGAG----YDVH | WYQQLPGTAPKLLIY | G--------NSNRPS | GVPDRFSGSKSD--TSASLAITGLQAEDEADYYC | QSYDN----------------------NLSGV | FGGGTKLTVL |
| iPS:4 43003 | 21-225_43F11_LC2 | VL1\|1e/JL2 | | QSVLTQP-PSVSGAPGQRVTISC | TGSS-SNIGAG----YDVH | WYQQLPGTAPKLLIY | G--------NSNRPS | GVPDRFSGSKSG--TSASLAITGLQAEDEADYYC | QSYDNS---------------------LSGSV | FGGGTKLTVL |
| | | Germline | | L_FR1 | L_CDR1 | L_FR2 | L_CDR2 | L_FR3 | L_CDR3 | L_FR4 |
| | VL6\|6a/JL3b | | 32132 | NFMLTQP-HSVSESPGKTVTISC | TRSS-GSIAS-----NYVQ | WYQQRPGSSPTTVIY | E--------DNQRPS | GVPDRFSGSIDSSSNSASLTISGLKTEDEADYYC | QSYDS-----------------------SNWV | FGGGTKLTVL |
| iPS:4 36792 | 21-225_169D12 | VL6\|6a/JL3b | | NFMLTQP-HSVSESPGKTVTISC | TRSS-GSITG-----NYVQ | WHQQRPGNSPTTLIY | E--------DKKRPS | GVPDRFSGSIDSSSNSASLTISGLKTEDEADYYC | QSYYS-----------------------GNWV | FGGGTKLTVL |
| | | Germline | | L_FR1 | L_CDR1 | L_FR2 | L_CDR2 | L_FR3 | L_CDR3 | L_FR4 |
| | VL3\|3l/JL2 | | 32133 | SSELTQD-PAVSVALGQTVRITC | QGD---SLRS-----YYAS | WYQQKPGQAPVLVIY | G--------KNNRPS | GIPDRFSGSSSG--NTASLTITGAQAEDEADYYC | NSRDSS---------------------GNHVV | FGGGTKLTVL |
| iPS:4 36800 | 21-225_171D12 | VL3\|3l/JL2 | | SSELTQD-PAVSVALGQTVRITC | QGD---SLRS-----YYAS | WYQQKPGQAPILVIY | A--------KNNRPS | GIPDRFSGSNSG--NTASLTITGAQAEDEADYYC | NSRDSS---------------------GSHVV | FGGGTKLTVL |
| iPS:4 | 21-225_172C3 | VL3\|3l/JL | | SSELTQD- | QGD---SLRN- | WYQQKPGQ | T-------- | GIPDRFSGSTSG-- | NSRDSS---------- | FGGGT |

| | | | | | | | | | | |
|---|---|---|---|---|---|---|---|---|---|---|
| iPS:4 36806 | 21-225_172B12 | VL3\|3l/JL2 | | SSELTQD-PAVSVALGQTVRITC | QGD---SLRN-----YYAS | WYQQKPGQAPILVIY | T--------KNSRPS | GIPDRFSGSTSG--NTASLTITGAQAEDEADYYC | NSRDSS---------------------GNHVV | FGGGTKLTVL |
| iPS:4 36964 | 21-225_190B3 | VL3\|3l/JL2 | | SSELTQD-PAVSVALGQTVRITC | QGD---KLRT-----YYAS | WYQQKPGQAPVLVVY | G--------KNNRPS | GIPDRFSGSSSG--NTASLTITGAQAEDEADYYC | NSRDSS--------------------GNHLVL | FGGGTKLTVL |
| iPS:4 36970 | 21-225_190B8 | VL3\|3l/JL2 | | SSELTQD-PAVSVALGQTVRITC | QGD---TLRP-----YYVS | WYQQKPGQAPVLVIY | G--------KNNRPS | GIPDRFSGSSSG--NTASLTITGAQAEDEADYYC | NSRDSS--------------------GNHLVV | FGGGTKLTVL |
| iPS:4 36980 | 21-225_190C10 | VL3\|3l/JL2 | | SSELTQD-PAVSVALGQTVRITC | QGD---SLRP-----YYAS | WYQQKPGQAPVLVIY | G--------KNNRPS | GIPDRFSGSSSG--NTASLTITEAQAEDEADYYC | NSRDSS--------------------GNHLVV | FGGGTKLTVL |
| iPS:4 36992 | 21-225_191B8 | VL3\|3l/JL2 | | SSELTQD-PAVSVALGQTVRITC | QGD---TLRP-----YYAS | WYQQKPGQAPVLVIY | G--------KNNRPS | GISDRFSGSSSG--NTASLTITGAQAEDEADYYC | NSRDSS--------------------GNHLVV | FGGGTKLTVL |
| iPS:4 36994 | 21-225_191A9 | VL3\|3l/JL2 | | SSELTQD-PAVSVALGQTVRITC | QGD---SLRP-----YYAS | WYQQKPGQAPVLVIY | G--------KNNRPS | GIPDRFSGSSSG--NTASLTITEAQAEDEADYYC | NSRDSC--------------------GNHLVV | FGGGTKLTVL |
| iPS:4 37016 | 21-225_193A6 | VL3\|3l/JL2 | | SSELTQD-PAVSVALGQTVRITC | QGD---SLRS-----YYAN | WYQQKPGQAPVLFIY | A--------KNNRPS | GIPDRFSGSNSG--NTASLTITGAQAEDEADYYC | NSRDSS---------------------GNHLV | FGGGTKLTVL |
| | | Germline | | L_FR1 | L_CDR1 | L_FR2 | L_CDR2 | L_FR3 | L_CDR3 | L_FR4 |
| | VL2\|2b2/JL2 | | 32194 | QSALTQP-ASVSGSPGQSITISC | TGTS-SDVGSY----NLVS | WYQQHPGKAPKLMIY | E--------VSKRPS | GVSNRFSGSKSG--NTASLTISGLQAEDEADYYC | CSYAGS---------------------STFVV | FGGGTKLTVL |
| iPS:4 36810 | 21-225_175F4 | VL2\|2b2/JL2 | | QSALTQP-ASVSGSPGQSITISC | TGTS-SDVGRF----NLVS | WYQQHPGYAPKLMIY | E--------VSKRPS | GVSNRFSGSKSG--NTASLTISGLQAEDEADYYC | CSYAGS---------------------STYVV | FGGGTKLTVL |
| iPS:4 36814 | 21-225_178H10 | VL2\|2b2/JL2 | | QSALTQP-ASVSGSPGQSITISC | TGTS-SDVGRF----NLVS | WYQHHPGNAPKLMIY | E--------VSKRPS | GVSNRFSGSKSG--NTASLTISGLQAEDEADYYC | CSYAGS---------------------STFVV | FGGGTKLTVL |
| | | Germline | | L_FR1 | L_CDR1 | L_FR2 | L_CDR2 | L_FR3 | L_CDR3 | L_FR4 |
| | VL3\|3l/JL3b | | 32195 | SSELTQD-PAVSVALGQTVRITC | QGD---SLRS-----YYAS | WYQQKPGQAPVLVIY | G--------KNNRPS | GIPDRFSGSSSG--NTASLTITGAQAEDEADYYC | NSRDSS---------------------GNHWV | FGGGTKLTVL |
| iPS:4 36816 | 21-225_179H5 | VL3\|3l/JL3b | | SSELTQD-PAVSVALGQTVRITC | QGD---SLRN-----YYAS | WYQQKPGQAPVFVIY | G--------KNNRPS | GIPDRFSGSRSG--NTASLTITGAQAEDEADYYC | NSRDSS---------------------GNHWV | FGGGTKLTVL |
| | | Germline | | L_FR1 | L_CDR1 | L_FR2 | L_CDR2 | L_FR3 | L_CDR3 | L_FR4 |
| | VL1\|1e/JL1 | | 32196 | QSVLTQP-PSVSGAPGQRVTISC | TGSS-SNIGAG----YDVH | WYQQLPGTAPKLLIY | G--------NSNRPS | GVPDRFSGSKSG--TSASLAITGLQAEDEADYYC | QSYDSS---------------------LSGYV | FGTGTKVTVL |
| iPS:4 36832 | 21-225_51D8 | VL1\|1e/JL1 | | QSVLTQP-PSVSGAPGQRVTISC | TGSS-SNIGAG----FEVH | WYQQLPGTAPKLLIY | G--------NSNRPS | GVPDRFSGSKSG--TSASLAITGLQAEDEADYYC | QSYDSS---------------------LSGYV | FGTGTKVTVL |
| | | Germline | | L_FR1 | L_CDR1 | L_FR2 | L_CDR2 | L_FR3 | L_CDR3 | L_FR4 |

| | | | | | | | | | | |
|---|---|---|---|---|---|---|---|---|---|---|
| | **VL3\|3r/JL7** | | 32197 | SYELTQP- PSVSVSPGQTASI TC | SGD---KLGD- ----KYAC | WYQQKPGQ SPVLVIY | Q-------- DSKRPS | GIPERFSGSNSG-- NTATLTISGTQAMDEADYYC | QAWDS----------- ------------STAAV | FGGGT QLTVL |
| **iPS:4 36840** | **21-225_53E9** | **VL3\|3r/JL 7** | | SYELTQP- PSVSVSPGQTASI TC | SGT---KLGD- ----KYVC | WYQQKPGQ SPVLVIN | Q-------- DTMRPS | GIPERFSGSNSG-- NTATLTISGTQAMDEADYYC | QTWDS----------- ------------STAV | FGGGT TLTVL |
| **iPS:4 36950** | **21-225_184G4** | **VL3\|3r/JL 7** | | SYELTQP- PSVSVSPGQTASI TC | SGD---KLGD- ----KFAC | WYQQKPGQ SPVLVIY | E-------- DRKRPS | GIPERFSGSNSG-- NTATLTISGTQAMDEADYYC | QAWDS----------- ------------RTVV | FGGGT QLTVL |
| | | **Germline** | | **L_FR1** | **L_CDR1** | **L_FR2** | **L_CDR2** | **L_FR3** | **L_CDR3** | **L_FR4** |
| | **VL1\|1b/JL2** | | 32198 | QSVLTQP- PSVSAAPGQKVTI SC | SGSS-SNIGN- ----NYVS | WYQQLPGT APKLLIY | D-------- NNKRPS | GIPDRFSGSKSG-- TSATLGITGLQTGDEADYYC | GTWDSS---------- -----------LSAVV | FGGGT KLTVL |
| **iPS:4 36856** | **21-225_58C5** | **VL1\|1b/JL 2** | | QSVLTQP- PSVSAAPGQKVTI SC | SGSS-SNIGN- ----NYVS | WYQQLPGT APKLLIY | D-------- NNKRPS | GIPDRFSGSKSG-- TSATLGITGLQTGDEADYYC | GTWDIS---------- -----------LSVGV | FGGGT KLTVL |
| **iPS:4 36960** | **21-225_198D2** | **VL1\|1b/JL 2** | | QSVLTQP- PSVSAAPGQKVTI SC | SGSS-SNIGS- ----NYVS | WYQQLPGT APKVLIY | D-------- NNKRPS | GIPDRFSGSKSG-- TSATLGITGLQTGDEADYYC | GTWDSR---------- -----------LNVGV | FGGGT KLTVL |
| **iPS:4 36966** | **21-225_190C3** | **VL1\|1b/JL 2** | | QSVLTQP- PSVSAAPGQKVTI SC | SGSS-SNIGN- ----NYVS | WYQQLPGT APKLLLY | D-------- SNKRPS | GIPGRFSGSKSG-- TSATLGITGLQTGDEADYYC | GTWDSS---------- -----------LSTVV | FGGGT KLTVL |
| **iPS:4 36968** | **21- 225_190B10** | **VL1\|1b/JL 2** | | QSVLTQP- PSVSAAPGQKVTI SC | SGSS-SNIGN- ----NYVS | WYQHLPGT APKLLIY | D-------- NNKRPS | GIPDRFSGSKSG-- TSATLGITGLQTGDEADYYC | GTWDSS---------- -----------LSAGV | FGGGT KLTVL |
| **iPS:4 36974** | **21-225_190H7** | **VL1\|1b/JL 2** | | QSVLTQP- PSVSAAPGQKVTI SC | SGSS-SNIGS- ----NYVS | WYQQLPGT APKVLIY | D-------- NNKRPS | GIPDRFSGSKSG-- TSATLGITGLQTGDEADYYC | GTWDGR---------- -----------LNVGV | FGGGT KLTVL |
| **iPS:4 36976** | **21-225_190D8** | **VL1\|1b/JL 2** | | QSVLTQP- PSVSAAPGQKVTI SC | SGSS-SNIGN- ----HYVS | WYQQLPGT APKLLIY | D-------- SSKRPS | EIPDRFSGSKSG-- TSATLGITGLQTGDEADYYC | GTWDSS---------- -----------LSTVV | FGGGT KLTVL |
| **iPS:4 36982** | **21- 225_190D10** | **VL1\|1b/JL 2** | | QSVLTQP- PSVSAAPGQKVTI SC | SGSS-SNIGS- ----NYVS | WYQQLPGT VPKVLIY | D-------- NNKRPS | GIPDRFSGSKSG-- TSATLGITGLQTGDEADYYC | GTWDSR---------- -----------LNVGV | FGGGT KLTVL |
| **iPS:4 36986** | **21-225_191A1** | **VL1\|1b/JL 2** | | QSVLTQP- PSVSAAPGQRVTI SC | SGSS-SNLGN- ----NFVS | WYQQFPGT APKLLIY | D-------- NYKRPS | GIPDRFSVSKSG-- TSATLGITGLQTGDEADYYC | GTWDSS---------- -----------LNTGV | FGGGT KLTVL |
| **iPS:4 37006** | **21-225_192G2** | **VL1\|1b/JL 2** | | QSVLTQP- PSVSAAPGQKVTI SC | SGSS-SNIGN- ----NYVS | WYQQLPGT APKLLIY | D-------- NNKRPS | RIPDRFSGSKSG-- TSATLGITGLQTGDEADYYC | GTWDSS---------- -----------LSAGV | FGGGT KLTVL |
| **iPS:4 37024** | **21- 225_194F11** | **VL1\|1b/JL 2** | | QSVLTQP- PSVSAAPGQKVTI SC | SGSS-SNIGN- ----NYVS | WYQQLPGT APKLLIY | D-------- NNKRPS | GIPDRFSGSKSG-- TSATLGITGLQTGDEADYYC | GTWDSS---------- -----------LSAGV | FGGGT KLTVL |
| **iPS:4 37028** | **21- 225_194G12** | **VL1\|1b/JL 2** | | QSVLTQP- PSVSAAPGQKVTI SC | SGSS-SNIGN- ----NYVS | WYQQLPGT APKLLIY | D-------- NNKRPS | RIPDRFSGSKSG-- TSATLGITGLQTGEEADYYC | GTWDSS---------- -----------LSVGV | FGGGT KLTVL |

| | | | | | | | | | | |
|---|---|---|---|---|---|---|---|---|---|---|
| iPS:4 37042 | 21-225_197E8 | VL1\|1b/JL 2 | | QSVLTQP- PSVSAAPGQKVTI SC | SGSS-SNIGN- ----KYVS | WYQQFPGT APKLLIY | D-------- NNKRPS | KIPDRFSGSKSG-- TSATLGITGLLTGDEADYYC | GIWDRS---------- -----------LSVMV | FGGGT KLTVL |
| iPS:4 37064 | 21-225_200G8 | VL1\|1b/JL 2 | | QSVLTQP- PSVSAAPGQRVTI SC | SGSS-SNLGN- ----NFVS | WYQQFPGT APKLLIY | D-------- NYKRPS | GIPDRFSVSKSG-- TSATLGITGLQTGDEADYYC | GTWDSS---------- -----------LNTGV | FGGGT KLTVL |
| iPS:4 37086 | 21-225_209A8 | VL1\|1b/JL 2 | | QSVLTQP- PSVSAAPGQKVTI SC | SGSS-SNIGS- ----NFLS | WYQQLPGT APKLLIY | D-------- NNKRPS | GIPDRFSGSKSG-- TSATLGITGLQTGDEADYYC | GTWDSS---------- -----------LSAGV | FGGGT KLTVL |
| iPS:4 37138 | 21-225_214D8 | VL1\|1b/JL 2 | | QSVLTQP- PSVSAAPGQKVTI SC | SGSS-SNIGN- ----NYVS | WYQQFPGT APKLLIH | D-------- NNKRPS | GIPDRFSGSKSG-- TSATLGITGLQTGDEADYYC | GAWDSS---------- -----------LSAVV | IGGGS KLTVL |
| iPS:4 37168 | 21-225_218G4 | VL1\|1b/JL 2 | | QSVLTQP- PSVSAAPGQKVTI SC | SGSS-SNIGN- ----NYVS | WYQQLPGT APKLLLY | D-------- SNKRPS | GIPARFSGSKSG-- TSATLGITGLQTGDEADYYC | GTWDSS---------- -----------LNTVV | FGGGT KLTVL |
| | | Germline | | L_FR1 | L_CDR1 | L_FR2 | L_CDR2 | L_FR3 | L_CDR3 | L_FR4 |
| | VL6\|6a/JL2 | | 32199 | NFMLTQP- HSVSESPGKTVTI SC | TRSS-GSIAS- ----NYVQ | WYQQRPGS SPTTVIY | E-------- DNQRPS | GVPDRFSGSIDSSSNSASLT ISGLKTEDEADYYC | QSYDS----------- ------------SNVV | FGGGT KLTVL |
| iPS:4 36888 | 21-225_63G7 | VL6\|6a/JL 2 | | NFMLTQP- HSVSESPGKTVTI SC | TRSN-GSIVS- ----NYVQ | WYQQRPGS SPTTMIY | E-------- DSRRPS | GVPDRFSGSIDSSSNSASLT ISGLKTEDEADYSC | QSYDG----------- ------------INVV | FGGGT KLTVL |
| iPS:4 36890 | 21-225_63A10 | VL6\|6a/JL 2 | | NFMLTQP- HSVSESPGKTVTI SC | TRSN-GSIVS- ----NYVQ | WYQQRPGS SPTTVIY | E-------- DKRRPS | GVPDRFSGSIDSSSNSASLT ISGLKTEDEADYYC | QSYDS----------- ------------INVV | FGGGT KLTVL |
| | | Germline | | L_FR1 | L_CDR1 | L_FR2 | L_CDR2 | L_FR3 | L_CDR3 | L_FR4 |
| | VL8\|8a/JL3b | | 32200 | QTVVTQE- PSFSVSPGGTVTL TC | GLSS- GSVSTS---- YYPS | WYQQTPGQ APRTLIY | S-------- TNTRSS | GVPDRFSGSILG-- NKAALTITGAQADDESDYYC | VLYMG----------- -----------SGIWV | FGGGT KLTVL |
| iPS:4 36910 | 21-225_73G1 | VL8\|8a/JL 3b | | QTVVTQE- PSFSVSPGGTVTL TC | GLSS- GSVSTS---- YYPS | WYQQTPGQ APRTLIY | N-------- TNTRSS | GVPDRFSGSILG-- NKAALTITGAQADDESDYYC | VLYMG----------- -----------SAIWV | FGGGT KLTVL |
| iPS:4 36948 | 21-225_183F5 | VL8\|8a/JL 3b | | QTVVTQE- PSFSVSPGGTVTL TC | GLSS- GSVSTT---- FYPS | WYQQTPGQ APRTLIY | N-------- TNTRSS | GVPDRFSGSILG-- NKAALTITGAQADDESDYYC | VLYMG----------- -----------SGIWV | FGGGT KLTVL |
| | | Germline | | L_FR1 | L_CDR1 | L_FR2 | L_CDR2 | L_FR3 | L_CDR3 | L_FR4 |
| | VL7\|7a/JL2 | | 32201 | QTVVTQE- PSLTVSPGGTVTL TC | ASST- GAVTSG---- YYPN | WFQQKPGQ APRALIY | S-------- TSNKHS | WTPARFSGSLLG-- GKAALTLSGVQPEDEAEYYC | LLYYG----------- -----------GAQVV | FGGGT KLTVL |
| iPS:4 36920 | 21-225_74E5 | VL7\|7a/JL 2 | | QTVVTQE- PSLTVSPGGTVTL TC | ASST- ETVTSG---- SYPN | WFQQKPGQ APRALIY | S-------- TSNKHS | WTPARFSGSLLG-- GKAALTLSDVQPEDEAEYYC | LLYYG----------- -----------GAQLV | FGGGT KLTVL |
| iPS:4 36926 | 21-225_78D10 | VL7\|7a/JL 2 | | QTVVTQE- PSLTVSPGGTVTL TC | ASST- GAVTSG---- YFPN | WFQQKPGQ APRALIY | S-------- TDNKHS | WTPARFSGSLLG-- GKAALTLSGVQPEDEAEYYC | LLYYG----------- -----------GAQLM | FGGGT KLTVL |
| iPS:4 | 21-225_190D1 | VL7\|7a/JL | | QTVVTQE- | ASST- | WFQQKPGQ | S-------- | WTPARFSGSLLG-- | LLYYG----------- | FGGGT |

| | | | | | | | | | | |
|---|---|---|---|---|---|---|---|---|---|---|
| **iPS:4 36984** | **21-225_190F10** | **VL7\|7a/JL 2** | | QTVVTQE-PSLTVSPGGTVTLTC | VFST-GAVTSG----SFPN | WFQQKPGQAPRALIY | S--------TSNKHS | WTPARFSGSLLG--GKAALTLSGVQPEDEADYYC | LLYCG-----------------------GAQLV | FGGGTKLTVL |
| **iPS:4 36988** | **21-225_191A2** | **VL7\|7a/JL 2** | | QTVVTQE-PSLTVSPGGTVTLTC | VLST-GAVTSG----SFPN | WFQQKPGQAPRALIY | S--------TSNKHS | WTPARFSGSLLG--GKAALTLSGVQPEDEADYYC | MLYCG-----------------------GAQLV | FGGGTKLTVL |
| **iPS:4 37002** | **21-225_191H9** | **VL7\|7a/JL 2** | | QTVVTQE-PSLTVSPGGTVTLTC | ASST-GAVTSA----YYPN | WLQQKPGQAPRTLIY | S--------TNNKHS | WTPARFSGSLLG--GKAALTLSDVQPEDEAEYYC | LIFYG-----------------------GVHVI | FGGGTKLTVL |
| **iPS:4 37008** | **21-225_192E3** | **VL7\|7a/JL 2** | | QTVVTQE-PSLTVSPGGTVTLTC | AFST-GSVTSG----SYPN | WFQQKPGQAPRALIY | S--------TNNKHS | WTPARFSGSLLG--GKAALTLSGVQREDEAEYYC | LLYYG-----------------------GAQLV | FGGGTKLTVL |
| **iPS:4 37012** | **21-225_192G7** | **VL7\|7a/JL 2** | | QTVVTQE-PSLTVSPGGTVTLTC | ASST-GAVTSG----NYPQ | WFQQKPGQAPRALIY | S--------TTNRHS | WTPARFSGSLLG--GKAALTLSGVQPEDEAEYYC | LFYYG-----------------------GAQVI | FGGGTKLTVL |
| **iPS:4 37014** | **21-225_192H8** | **VL7\|7a/JL 2** | | QTVVTQE-PSLTVSPGGTVTLTC | AFST-GTVTSG----FYPN | WFQQKPGQAPRALIY | N--------TSNRHS | WTPARFSGSLLG--GMAALTLSGVQPEDEADYYC | LLYYG-----------------------GAQLM | FGGGTKLTVL |
| **iPS:4 37022** | **21-225_194G5** | **VL7\|7a/JL 2** | | QTVVTQE-PSLTVSPGGTVTLTC | ASST-GAVTSG----NYPN | WFQQKPGQTPRALIY | S--------TSNKHS | WTPARFSGSLLG--GKGALTLSGVQPEDEAEYYC | LIYYG-----------------------GAQLM | FGGGTKLTVL |
| **iPS:4 37026** | **21-225_194D12** | **VL7\|7a/JL 2** | | QTVVTQE-PSLTVSPGGTVTLTC | ASST-GAVTSG----SFPS | WFQQKPGQAPRALIY | S--------TSNRHS | STPARFSGSLLG--GKAALTLSGVQPEDEADYYC | LIYYG-----------------------GAQLA | FGGGTKLTVL |
| **iPS:4 37040** | **21-225_196E7** | **VL7\|7a/JL 2** | | QTVVTQE-PSLTVSPGGTVTLTC | ASST-GAVTSA----YYPN | WLQQKPGQAPRTLIY | S--------TNNKHS | WTPARFSGSLLG--GKAALTLSDVQPEDEAEYYC | LIFYG-----------------------GVHVI | FGGGTKLTVL |
| **iPS:4 37048** | **21-225_197B11** | **VL7\|7a/JL 2** | | QTVVTQE-PSLTVSPGGTVTLTC | AFST-GSVTSG----SYPN | WFQQKPGQAPRALIY | S--------TNNKHS | WTPARFSGSLLG--GKAALTLSGVQPEDEAEYYC | LLYYG-----------------------GAQLV | FGGGTKLTVL |
| **iPS:4 37050** | **21-225_197C11** | **VL7\|7a/JL 2** | | QTVVTQE-PSLTVSPGGTVTLTC | ASST-GAVTSA----YYPN | WLQQKPGQAPRTLIY | S--------TSNKHS | WTPARFSGSLLG--GKAALTLSDVQPEDEAEYYC | LIFYG-----------------------GVHVI | FGGGTKLTVL |
| **iPS:4 37056** | **21-225_198B8** | **VL7\|7a/JL 2** | | QTVVTQE-SSLTVSPGGTVTLTC | VLST-GAVTSG----SFPN | WFQQKPGQAPRALIY | S--------TSNKHS | WTPARFSGSLLG--GKAALTLSGVQPEDEADYYF | MLYSG-----------------------GAQMV | FGGGTKLTVL |
| **iPS:4 37062** | **21-225_200H1** | **VL7\|7a/JL 2** | | QTVVTQE-PSLTVSPGGTVTLTC | ASNT-GAVTSG----SYPN | WFQQKPGQAPRALIY | H--------TNNKHS | WTPARFSGSLLG--GKAALTLSGVQPEDEAEYYC | LIYYG-----------------------GAQLV | FGGGTKLTVL |
| **iPS:4 37066** | **21-225_200G9** | **VL7\|7a/JL 2** | | QTVVTQE-PSLTVSPGGTVTLTC | ASNT-GAVTSG----SYPN | WLQQKPGQAPRALIY | H--------TDNKHS | WTPARFSGSLLG--GKAALTLSGAQPEDEAEYYC | LIYYG-----------------------GAQLV | FGGGTKLTVL |
| **iPS:4 37068** | **21-225_200A11** | **VL7\|7a/JL 2** | | QTVVTQE-PSLTVSPGGTVTLTC | ASST-GAVTSG----YYPN | WFQQKPGQVPRALIY | S--------TNNKHS | WTPARFSGSLLG--GKAALTLSGVQPEDEADYYC | LLYYG-----------------------GAHLA | FGGGTKLTVL |
| **iPS:4 37090** | **21-225_210E11** | **VL7\|7a/JL 2** | | QTVVTQE-PSLTVSPGGTVTL | AFST-GAVTSG---- | WFQQKPGQAPRALIY | S--------TSNKHS | WTPARFSGSLLG--GKAALTLSGVQPEDEAEYYC | LLYYG-----------------------GAQLV | FGGGTKLTVL |

| | | | | | | | | | | |
|---|---|---|---|---|---|---|---|---|---|---|
| iPS:4 37106 | 21-225_211H7 | VL7\|7a/JL 2 | | QTVVTQE- PSLTVSPGGTVTL TC | AFST- GAVTSG---- NYPS | WFQQKPGQ VPRALIY | S-------- TSNRHS | WTPARFSGSLLG-- GKAALTLSGVQPEDEAEYYC | LLYYG----------- -----------GAQLV | FGGGT KLTVL |
| iPS:4 37108 | 21-225_211C9 | VL7\|7a/JL 2 | | QTVVTQE- PSLTVSPGGTVTL TC | GSST- GSVTSG---- YFPN | WFQQKPGQ APRPLIY | S-------- TNNKHS | WTPARFSGSLLG-- GKAALTLSDVQPEDEADYYC | LLYYG----------- -----------GAQLA | FGGGT KLTVL |
| iPS:4 37110 | 21-225_211E9 | VL7\|7a/JL 2 | | QTVVTQE- PSLTVSPGGTVTL TC | ASST- GAVTSG---- NYPN | WFQQKPGQ APRALIY | S-------- TINKHS | GTPARFTGFLLG-- GKAALTLSGVQPEDEAEYYC | LLYYG----------- -----------GAQLA | FGGGT KLTVL |
| iPS:4 37120 | 21-225_212A9 | VL7\|7a/JL 2 | | QTVVTQE- PSLTVSPGGTVTL TC | ASST- GAVTSG---- YYPN | WFQQKPGQ APRALIY | S-------- TNNKHS | WTPARFSGSLLG-- GKAALTLSGVQPDDEADYYC | LLYYG----------- -----------GAQVG | FGGGT KLTVL |
| iPS:4 37124 | 21-225_212H12 | VL7\|7a/JL 2 | | QTVVTQE- PSLTVSPGGTVTL TC | ASST- GAVTSG---- YYPN | WFQQKPGQ APRALIY | S-------- TSNKHS | WTPARFSGSLLG-- GKAALTLSGVQPEDEAEYYC | LLYYG----------- -----------GAHVV | FGGGT KLTVL |
| iPS:4 37132 | 21-225_213F5 | VL7\|7a/JL 2 | | QTVVTQE- PSLTVSPGGTVTL TC | GSST- GSVTSG---- YFPN | WFQQKPGQ TPRPLIY | S-------- TNNKHS | WTPARFSGSLLG-- GKTALTLSDVQPEDEADYYC | LLYFG----------- -----------GAQLA | FGGGT KLTVL |
| iPS:4 37136 | 21-225_214H3 | VL7\|7a/JL 2 | | QTVVTQE- PSLTVSPGGTVTL TC | ASST- GAVTSG---- YYPN | WFQQKPGQ APRALIY | S-------- TSNKHS | CTPARFSGSLLG-- GKAALTLSGVQPEDEAEYYC | LLYYG----------- -----------GAHVV | FGGGT KLTVL |
| iPS:4 37140 | 21-225_214E12 | VL7\|7a/JL 2 | | QTVVTQE- PSLTVSPGGTVTL TC | ASST- GAVTSG---- YYPN | WFQQKPGQ APRALIY | S-------- TSNKHS | WTPARFSGSLLG-- GKAALTLSDVQPEDEAEYYC | LLYCD----------- -----------GAQLV | FGGGT KLTVL |
| iPS:4 37142 | 21-225_215A3 | VL7\|7a/JL 2 | | QTVVTQE- PSLTVSPGGTVTL TC | ASST- EAVTSG---- NYPS | WFQQKPGQ APRALIY | S-------- TSNKHS | GTPARFTGSLLG-- GKAALTLSGVQPEDEAEYYC | LLYYG----------- -----------GAQLA | FGGGT KLAVL |
| iPS:4 37148 | 21-225_215H3 | VL7\|7a/JL 2 | | QTVVTQE- PSLTVSPGGTVTL TC | ASST- GAVTSG---- YYPN | WFQQKPGQ APRALIY | S-------- TNNKHS | CGPARFSGSLLG-- GKAALTLSGVQPDDEADYYC | LLYYG----------- -----------GAQVG | FGGGT KLTVL |
| iPS:4 37154 | 21-225_216A7 | VL7\|7a/JL 2 | | QTVVTQE- PSLTVSPGGTVTL TC | ASST- GAVTSG---- YYPN | WFQQKPGQ APRALIY | S-------- TNNKHS | CTPARFSGSLLG-- GKAALTLSGVQPDDEADYYC | LLYYG----------- -----------GAQVG | FGGGT KLTVL |
| iPS:4 37158 | 21-225_216H11 | VL7\|7a/JL 2 | | QTVVTQE- PSLTVSPGGTVTL TC | ASST- GAVTSG---- YYPN | WFQQKPGQ APRALIY | S-------- TSNKHS | WTPARFSGSLLG-- GKAALTLSDVQPEDEAEYYC | LLYCD----------- -----------GAQLV | FGGGT KLTVL |
| | | Germline | | L_FR1 | L_CDR1 | L_FR2 | L_CDR2 | L_FR3 | L_CDR3 | L_FR4 |
| | VL1\|1b/JL3b | | 32202 | QSVLTQP- PSVSAAPGQKVTI SC | SGSS-SNIGN- ----NYVS | WYQQLPGT APKLLIY | D-------- NNKRPS | GIPDRFSGSKSG-- TSATLGITGLQTGDEADYYC | GTWDSS----------- -----------LSAWV | FGGGT KLTVL |
| iPS:4 36972 | 21-225_190C7 | VL1\|1b/JL 3b | | QSVLTQP- PSVSAAPGQKVTI SC | SGGS-SNIGN- ----NYVS | WFQQFPGT APKFLIY | D-------- NNKRPS | GIPDRFSGSKSG-- TSATLGITGLQTGDEADYYC | GTWDRT----------- -----------LSDWV | FGGGT KLTVL |
| iPS:4 37020 | 21-225_193F11 | VL1\|1b/JL 3b | | QYVLTQP- PSVSAAPGQKVTI SC | FGGS-SNIGN- ----NYVS | WFQQFPGT APKFLIY | D-------- NNKRPS | GILDRLSGSKSG-- TSATLDITGLQNGDEADYYC | GTWDRT----------- -----------MSDWV | FGGGT KLTVL |

| | | | | | | | | | | |
|---|---|---|---|---|---|---|---|---|---|---|
| **iPS:4 37036** | **21-225_195H9** | **VL1\|1b/JL 3b** | | QYVLTQP-PSVSAAPGQKVTISC | SGGS-SNIGN-----NYVS | WFQQFPGTAPKFLIY | D--------NNKRPS | GILDRFSGSKSG--TSATLGITGLQTGDEADYYC | GTWDRT---------------------MSDWV | FGGGTKLTVL |
| | | **Germline** | | **L_FR1** | **L_CDR1** | **L_FR2** | **L_CDR2** | **L_FR3** | **L_CDR3** | **L_FR4** |
| | **VL1\|1b/JL1** | | 32203 | QSVLTQP-PSVSAAPGQKVTISC | SGSS-SNIGN-----NYVS | WYQQLPGTAPKLLIY | D--------NNKRPS | GIPDRFSGSKSG--TSATLGITGLQTGDEADYYC | GTWDSS---------------------LSAVV | FGTGTKVTVL |
| **iPS:4 36996** | **21-225_191B9** | **VL1\|1b/JL 1** | | QSVLTQP-PSVSAAPGQKVTISC | SGSS-SNIGN-----NYVS | WYQQLPGTAPKLLIY | D--------NKKRPS | GIPDRFSGSKSG--TSATLGITGLQTGDEADYYC | GTWDSS---------------------LSVCV | FGTGTKVTVL |
| **iPS:4 37054** | **21-225_194G3** | **VL1\|1b/JL 1** | | QSVLTQP-PSVSAAPGQKVTISC | SGSS-SNIGN-----NYIS | WYQQLPGTAPKLLIY | D--------NKKRPS | GIPDRFSGSKSG--TSATLGITGLQTGDEADYYC | GTWDSS---------------------LSVCV | FGTGTKVTVL |
| **iPS:4 37058** | **21-225_199F3** | **VL1\|1b/JL 1** | | QSVLTQP-PSVSAAPGQKVTISC | SGSS-SNIGN-----NYVS | WYQQLPGTAPKLLIY | D--------NNKRPS | GIPDRFSGSKSG--TSATLGITGLQTGDEADYYC | GTWDSS---------------------LSACV | FGTGTKVTVL |
| | | **Germline** | | **L_FR1** | **L_CDR1** | **L_FR2** | **L_CDR2** | **L_FR3** | **L_CDR3** | **L_FR4** |
| | **VL5\|5c/JL2** | | 32204 | QAVLTQP-ASLSASPGASASLTC | TLRS-GINVGT----YRIY | WYQQKPGSPPQYLLR | YKS----DSDKQQGS | GVPSRFSGSKDASANAGILLISGLQSEDEADYYC | MIWHS----------------------SASVV | FGGGTKLTVL |
| **iPS:4 37000** | **21-225_191G9** | **VL5\|5c/JL 2** | | QAVSTRP-SSLSASPGASASLTC | TLRS-GINVGT----YRIY | WYQQKPGSPPQYLLR | YKS----DSDKQQGS | GVPSRFSGSKDASANAGILLISGLQSEDEADYYC | MIWHS-----------------------SAVV | FGGGTKLTVL |
| | | **Germline** | | **L_FR1** | **L_CDR1** | **L_FR2** | **L_CDR2** | **L_FR3** | **L_CDR3** | **L_FR4** |
| | **VL3\|3j/JL2** | | 32205 | SYELTQP-LSVSVALGQTARITC | GGN---NIGS-----KNVH | WYQQKPGQAPVLVIY | R--------DSNRPS | GIPERFSGSNSG--NTATLTISRAQAGDEADYYC | QVWDS----------------------STAVV | FGGGTKLTVL |
| **iPS:4 37074** | **21-225_203B2** | **VL3\|3j/JL 2** | | SYELTQP-LSVSVALGQTARITC | GGN---NIGR-----KNVH | WYQQKPGQSPVLIIH | R--------DSDRPS | GIPERFSGSNSG--NTATLTISRAQAGDEADYYC | QVWDS-----------------------GTAV | FGGGTKLPVL |
| **iPS:4 37082** | **21-225_205E12** | **VL3\|3j/JL 2** | | SYELTQP-LSVSAALGQTARITC | GGN---NIGR-----KNVH | WYQQKPGQSPVLIIH | R--------DSDRPS | GIPERFSGSNSG--NTATLTISRAQAGDEADYYC | QVWDS-----------------------GTAV | FGGGTKLPVL |
| **iPS:4 37084** | **21-225_206B5** | **VL3\|3j/JL 2** | | SYELTQP-LSVSVALGQTARIAC | GGN---NIGR-----KNVH | WYQQKPGLAPVPVIX | R--------DSYRSS | GIPDRFSGSNCG--NTTTVTISRAQAGEEAEYYC | QDWDS-----------------------STVV | FGGGTKLTVL |
| **iPS:4 37088** | **21-225_209H10** | **VL3\|3j/JL 2** | | SYELTQP-LSVSVALGQTARIAC | GGN---NIGR-----KNVH | WYQQKPGLAPVPVIL | R--------DSYRSS | GIPDRFSGSNWG--NTATVTISRAQAGEEAEYYC | QDWDS-----------------------STVV | FGGGTKLTVL |
| **iPS:4 37100** | **21-225_211H2** | **VL3\|3j/JL 2** | | SYELTQP-LSVSVALGQTARITC | GGN---NIGR-----RNVH | WYQQKPGQAPILVIY | R--------DRDRPS | GIPERFSGSNSG--NTATLTISRAQAGDEADYFC | QVWDS-----------------------STAV | FGGGTKLTVL |
| **iPS:4 37160** | **21-225_216B12** | **VL3\|3j/JL 2** | | SYELTQP-LSVSVALGQTARITC | GGD---NIRR-----RNVH | WYQQKPGQAPVLVIY | R--------DSNRPS | GIPERFSGSNSG--NTATLTISRAQAGDEADYYC | QVWDS-----------------------STGV | FGGGTKLTVL |
| **iPS:3** | **21-225_3E10** | **VL3\|3j/JL** | | SYELTQP- | GGN---NIGS- | WYQQKPGQ | S-------- | GIPERFSGSNPG-- | QVWDSS---------- | FGGGT |

| | | | | | | | | | | |
|---|---|---|---|---|---|---|---|---|---|---|
| iPS:3 93204 | 21-225_8C12 | VL3\|3j/JL 2 | | SYELTQP- HSVSVATAQMARI TC | GGN---NIGS- ----KAVH | WYQQKPGQ DPVLVIY | S-------- DSNRPS | GIPERFSGSNPG-- NTATLTISRIEAGDEADYYC | QVWDSS---------- -----------SDHVV | FGGGT KLTVL |
| | | Germline | | L_FR1 | L_CDR1 | L_FR2 | L_CDR2 | L_FR3 | L_CDR3 | L_FR4 |
| | VL2\|2a2/JL2 | | 32206 | QSALTQP- ASVSGSPGQSITI SC | TGTS- SDVGGY---- NYVS | WYQQHPGK APKLMIY | E-------- VSNRPS | GVSNRFSGSKSG-- NTASLTISGLQAEDEADYYC | SSYTSS---------- -----------STLVV | FGGGT KLTVL |
| iPS:4 37092 | 21-225_210B12 | VL2\|2a2/J L2 | | QSALTQP- ASVSGSPGQSITI SC | TGTS- SDVGGY---- NYVS | WYQQHPGK APKFMIY | E-------- VRNRPS | GVSNRFSGSKSG-- NTASLTISGLQAEDEADYYC | SSYTS----------- -----------SRTLV | FGGGT KLTVL |
| iPS:4 37134 | 21-225_213A7 | VL2\|2a2/J L2 | | QSALTQP- ASVSGSPGQSITI SC | TGTS- SDVGGY---- NYVS | WYQQHPGK APKFMIY | E-------- VRNRPS | GVSNRFSGSKSG-- NTASLTISGLQAEDEADYYC | SSYTS----------- -----------SRTLV | FGGGT KLTVL |
| iPS:4 72733 | 21-225_2B10_LC 2 | VL2\|2a2/J L2 | | QSALTQP- ASVSGSPGQSITI SC | TGTS- SDVGGY---- NFVS | WYQQHPDK APKLMIY | E-------- VSNRPS | GVSNRFSGSKSG-- NTASLTISGLQAEDEADYYC | SSYTS----------- -----------TGTVV | IGGGT KLTVL |
| iPS:3 92573 | 21-225_15G2 | VL2\|2a2/J L2 | | QSALTQP- ASVSGSPGQSITI SC | TGTS- SDVGGY---- NYVS | WYQQHPGK APKLMIY | E-------- VSNRPS | GVSNRFSGSKSG-- NTASLTISGLQAEDEADYYC | TSYTS----------- -----------TSTVV | FGGGT KLTVL |
| iPS:3 93232 | 21-225_17F12 | VL2\|2a2/J L2 | | QSALTQP- ASVSGSPGQSITI SC | TGAS- SDVGDY---- NSVS | WYQQHPGK APKLMIY | E-------- VSNRPS | GVSNRFSGSKSG-- NTASLTISGLQAEDEADYYC | SSYTS----------- -----------SITVV | FGGGT KLTVL |
| iPS:3 98494 | 21-225_21H4 | VL2\|2a2/J L2 | | QSALTQP- ASVSGSPGQSITI SC | TGTS- SDVGGY---- NSVS | WYQQHPDK APKLMIY | E-------- VSNRPS | GVSNRFSGSKSG-- NTASLTISGLQAEDEADYYC | SSYTR----------- -----------SSTVV | FGGGT KLTVL |
| | | Germline | | L_FR1 | L_CDR1 | L_FR2 | L_CDR2 | L_FR3 | L_CDR3 | L_FR4 |
| | VL3\|3m/JL1 | | 32207 | SYELMQP- PSVSVSPGQTARI TC | SGD---ALPK- ----QYAY | WYQQKPGQ APVLVIY | K-------- DSERPS | GIPERFSGSSSG-- TTVTLTISGVQAEDEADYYC | QSADSS---------- -----------GTYVV | FGTGT KVTVL |
| iPS:4 37102 | 21-225_211E5 | VL3\|3m/J L1 | | SYELTQP- PSVSVSPGQTARI TC | SGD---ALPK- ----QYAY | WYQQKPGQ APVLVIY | K-------- DSARPS | GIPERFSGSRSG-- TTVTLTVSGVQAEDEADYYC | QLVYS----------- -----------SDTYV | FGTGT MLTVL |
| iPS:4 37164 | 21-225_217C6 | VL3\|3m/J L1 | | SYELTQP- PSVSVSPGQTARI TC | SGD---ALPK- ----QYAY | WYQQKPGQ APVLVIY | K-------- DSERPS | GIPERFSGSRSG-- TTVTLTIRGVQAEDEADYYC | QLIVS----------- -----------SDTYV | FGTGT KVTVL |
| iPS:4 37166 | 21-225_217G11 | VL3\|3m/J L1 | | SYELTQP- PSVSVSPGQTARI TC | SGD---ALPK- ----QYAY | WYQQKPGQ APVLVIY | K-------- DSERPS | GIPERFSGSRSG-- TTVTLTVSGVQAEDEADYYC | QLVYS----------- -----------SDTYV | FGTGT KVTVL |
| iPS:4 37170 | 21-225_218E5 | VL3\|3m/J L1 | | SYELTQP- PSVSVSPGQTARI TC | SRD---VLPK- ----QYAY | WYQQKPGQ APVLVIY | K-------- DSERPS | GIPERFSGSRSG-- TTVTLTIRGVQAEDEADYYC | QLVVS----------- -----------SDTYV | FGTGT KVTVL |
| iPS:4 37196 | 21-225_226B7 | VL3\|3m/J L1 | | SFELTQP- PSVSVSPGQTARI TC | SGD---ALPR- ----HYVY | WYQQNPGQ APVLVIY | K-------- DSERPS | GIPERFSGSSSG-- TTVTLTISGVQAEDEADYYC | QSADS----------- -----------SGTYV | FGTGT KVTVL |
| | | Germline | | L_FR1 | L_CDR1 | L_FR2 | L_CDR2 | L_FR3 | L_CDR3 | L_FR4 |
| | VL4\|4c/JL2 | | 32208 | LPVLTQP- | TLSS-EHSTY- | WYQQRPGR | VKS---- | GIPDRFMGSSSG-- | GESHTID--------- | FGGGT |

| | | | | | | | | | | |
|---|---|---|---|---|---|---|---|---|---|---|
| **iPS:3 92596** | **21-225_12D8** | **VL4\|4c/JL 2** | | LPVLTQP-PSASALLGASIKL TC | TLSS-EHSTY------TIE | WYQQRPGR SPQYIMK | VKS---- DGSHSKGD | GIPDRFMGSSSG-- ADRYLTFSNLQSDDEDEYHC | GESHTID--------- ----------GQVGVV | FGGGT KLTVL |
| **iPS:3 93174** | **21-225_15D8** | **VL4\|4c/JL 2** | | LPVLTQP-PSASALLGASIKL TC | TLSS-EHSTY------TIE | WYQQRPGR SPQYIMK | VKS---- DGSHSKGD | GIPDRFMGSSSG-- ADRYITFSNLQSDDEEEYHC | GESHTID--------- ----------GQVGVV | FGGGT KLTVL |
| **iPS:3 98544** | **21-225_7C8** | **VL4\|4c/JL 2** | | LPVLTQP-PSASALLGASIKL TC | TLSS-EHSTY------TIE | WYQQRPGR SPQYIMK | VKS---- DGSHSKGD | GIPDRFMGSSSG-- GDRYLTFSNLQSDDEDEYHC | GESHPID--------- ----------GQVGVV | FGGGT KLTVL |
| | | **Germline** | | **L_FR1** | **L_CDR1** | **L_FR2** | **L_CDR2** | **L_FR3** | **L_CDR3** | **L_FR4** |
| | **VL3\|3h/JL2** | | 32209 | SYVLTQP-PSVSVAPGKTARI TC | GGN---NIGS-----KSVH | WYQQKPGQ APVLVIY | Y-------- DSDRPS | GIPERFSGSNSG-- NTATLTISRVEAGDEADYYC | QVWDSS---------- -----------SDHVV | FGGGI KLTVL |
| **iPS:3 93208** | **21-225_16F3** | **VL3\|3h/JL 2** | | SYVLTQP-PSVSVAPGQTARI TC | GGN---NIGS-----KSVH | WYQQKPGQ APVLVVY | D-------- DTDRPS | GIPERFSGSNSG-- NTATLTISRVEAGDEADYYC | QVWDSS---------- -----------SDHVV | FGGGT KLTVL |
| **HEAVY_VARIABLE** | | | | | | | | | | |
| | | **Germline** | | **H_FR1** | **H_CDR1** | **H_FR2** | **H_CDR2** | **H_FR3** | **H_CDR3** | **H_FR4** |
| | **VH1\|1-08/D6\|6-19\|RF1/JH4** | | 32210 | QVQLVQS-GAEVKKPGASVKV SCKASG-YTFT | S-----YDIN | WVRQATGQ GLEWMG | WMNPN--- SGNTGYAQK FQG | RVTMTRNTSISTAYMELSSL RSEDTAVYYCAR | GYSSGW---------- ----------YYFDY | WGQGT LVTVS S |
| **iPS:4 26126** | **21-225_6G6** | **VH1\|1-08/D6\|6-19\|RF1/JH4** | | QVQLVQS-GAEVRKPGASVKV SCKASG-YTFT | N-----YDIN | WVRQATGQ GPEWMG | WMHPN--- SGNTGYAKK FQG | RVTMTRNTSISAAYMVLSSL RSEDTAVYYCAL | SSGWY----------- -----------YFDY | WGQGT LVTVS S |
| **iPS:4 12232** | **21-225_4A2** | **VH1\|1-08/D6\|6-19\|RF1/JH4** | | QVQLVQS-GTEVKKPGASVKV SCKASG-YTFT | N-----YDIN | WVRQATGQ GLEWMG | WMHPN--- SGNTGYAQK FQG | RVTLTRNTSISTAYMELSSL RSEDTAVYYCAS | SSGWY----------- -----------YFDY | WGQGT LVTVS S |
| **iPS:4 26112** | **21-225_12F12** | **VH1\|1-08/D6\|6-19\|RF1/JH4** | | QVQLLQS-GAEVKKPGASVKV SCKASG-YTFT | N-----YDIN | WVRQATGQ GLEWMG | WMYPN--- SGNTGYAQK FQG | RVTMTMNTSISTAYMELSSL RSEDTAVYYCAM | SSGWY----------- -----------YFDF | WGQGT LVTVS S |
| **iPS:4 51141** | **21-225_164B11** | **VH1\|1-08/D6\|6-19\|RF1/JH4** | | QVQLVQS-GAEVKKPGASVKV SCKASG-YTFT | N-----YDIN | WVRQATGQ GLEWMG | WMTPN--- SGNTGYAQK FQG | RVTMTRNTSMSTAYMELSSL RSEDSAVYYCSY | SSGWY----------- -----------MFDY | WGQGT LVTVS S |
| **iPS:4 68850** | **21-225_63F4** | **VH1\|1-08/D6\|6-19\|RF1/JH4** | | QVQLVQS-GAEVKKPGASVKV SCKASG-YTFT | N-----YDVN | WVRQATGQ GLEWMG | WMHPN--- SGNTGYAQK FRG | RVTMTRNTSLSTVYMELSSL RSEDTAVYYCAY | SSGWY----------- -----------VFDY | WGQGT LVTVS S |
| **iPS:4 68852** | **21-225_71F3** | **VH1\|1-08/D6\|6-19\|RF1/JH4** | | QVQLVQS-GAEVKKPGASVKV SCKASG-YTFT | N-----YDVN | WVRQATGQ GLEWMG | WMHPN--- SGNTGYAQK FQG | RVTMTRNTSISTAYMELSSL RSEDTAVYYCAY | SSGWY----------- -----------VFDS | WGQGT LVTVS S |

| | | | | | | | | | | |
|---|---|---|---|---|---|---|---|---|---|---|
| iPS:468854 | 21-225_72C4 | VH1\|1-08/D6\|6-19\|RF1/JH4 | | QVQLVQS-GAEVKKPGASVKVSCKASG-YTFT | N-----YDIN | WVRQVTGQGLEWMG | WMHPN---SGNTGYAQKFQG | RVTMTRNTSISTAYMELNSLRSEDTAVYYCSH | SSGWY-----------------------LFDY | WGQGTLVTVSS |
| iPS:468870 | 21-225_74A8 | VH1\|1-08/D6\|6-19\|RF1/JH4 | | QVQLVQS-GAEVKKPGASVKVSCKASG-YTFT | N-----YDIN | WVRQASGQGLEWMG | WMHPN---SGNTGYAQKFQG | RVTMTRNTSISTAYMELSSLRSEDSAVYYCAY | SSGWY-----------------------KFDY | WSQGTLVTVSS |
| iPS:423314 | 21-225_12F11 | VH1\|1-08/D6\|6-19\|RF1/JH4 | | QVQLVQS-GAEVKKPGASVKVSCKASG-YTFT | N-----YDIN | WVRQATGQGPEWMG | WMHPN---SGNTGYAKKFQG | RVTMTRNTSISAAYMVLSSLRSEDTAVYYCAL | SSGWY-----------------------YFDY | WGQGTLVTVSS |
| iPS:433909 | 21-225_43D8 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLTSEDTAVYYCAH | SSGWT-----------------------LFDY | WGQGTLVTVSA |
| iPS:434177 | 21-225_56A1 | VH1\|1-08/D6\|6-19\|RF1/JH4 | | QVQLVQS-GAEVKKPGASVKVSCKASG-YTFT | N-----YDIN | WVRQATGQGLEWLG | WMHPN---SGNTGYAQKFQG | RVTMTRNTSISTAYMELSSLRSEDTAVYYCAY | SSGWY-----------------------VFDY | WGQGTLVTVSS |
| iPS:434211 | 21-225_60F3 | VH1\|1-08/D6\|6-19\|RF1/JH4 | | QVLLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY-----------------------FFDY | WGQGTLVTVSS |
| iPS:434235 | 21-225_61E3 | VH1\|1-08/D6\|6-19\|RF1/JH4 | | QVQLVQS-GAEVKKPGASVKVSCKASG-YTFT | N-----YDIN | WVRQATGQGLEWMG | WMTPN---SGNTGYAQKFQG | RVTMTRNTSISTAYMELSSLKSEDTAVYYCAY | SSGWY-----------------------RFDY | WGQGTLVTVSS |
| iPS:434237 | 21-225_61B5 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGSTGYAQKFQG | RVTMTRNTSISTAYMELSSLRSEDTAVYYCAY | SSGWY-----------------------YFDY | WGQGTLVTVSS |
| iPS:434295 | 21-225_58B9 | VH1\|1-08/D6\|6-19\|RF1/JH4 | | QVQLVQS-GAEVKKPGASVKVSCKASG-YTFT | N-----YDIN | WVRQATGQGLEWMG | WMNPN---SGSTGYAQKFQG | RVTMTRNTSISTAYMELSSLRSEDTAVYYCAY | SSGWY-----------------------YFDY | WGQGTLVTVSS |
| iPS:434305 | 21-225_59E1 | VH1\|1-08/D6\|6-19\|RF1/JH4 | | QVQLVQS-GAEVKKPGASVKVSCKASG-YTFT | N-----YDIN | WVRQATGQGLEWMG | WMTPN---SGNTGYAQKFQG | RVTMTRTTSISTAYMELSSLRSEDTAVYYCAF | SSGWY-----------------------FFDY | WGQGTLVTVSS |
| iPS:434321 | 21-225_59F10 | VH1\|1-08/D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYNCAV | SSGWY-----------------------YFDY | WGQGTLVTVSS |
| iPS:434431 | 21-225_70E7 | VH1\|1-08/D6\|6- | | QVQLVQS-GAEVKKPGASVKV | N-----YDIN | WVRQATGQGLEWMG | WMHPN---SGNTGYAQK | RVTMTRNTSISTAYMELSSLRSEDTAVYYCAY | SSGWY-----------------------VFDY | WGQGTLVTVS |

| | | | | | | | | | | |
|---|---|---|---|---|---|---|---|---|---|---|
| iPS:434443 | 21-225_71G3 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGSTGYAQKFQG | RVTMTRDTSVSTAYMELSSLRSEDTAVYYCAY | SSGWY---------------------YFDY | WGQGTLVTVSS |
| iPS:434475 | 21-225_74F9 | VH1\|1-08/D6\|6-19\|RF1/JH4 | | QVQLVQS-GAEVKKPGASVKVSCKASG-YTFT | N-----YDIN | WVRQATGQGLEWMG | WMNPN---SGNTGCAQKFQG | RVTMTRNTSISTAYMELSSLRSEDTAVYYCAS | SSGWN---------------------FFDY | WGQGTLVTVSS |
| iPS:434477 | 21-225_74A6 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGYAQKFRG | RVTMTWNTSISTAYMELSSLRSEDTAVYYCAS | SSGWY---------------------YFDY | WGQGTLVTVSS |
| iPS:434487 | 21-225_76G2 | VH1\|1-08/D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAG | SSGWY---------------------MFDY | WGQGTLVTVSS |
| iPS:434511 | 21-225_74B11 | VH1\|1-08/D6\|6-19\|RF1/JH4 | | QVQLVQS-GAEVKKPGASVRVSCKASG-YTFT | N-----YDIN | WVRQATGQGLEWMG | WMHPN---SGNTGYAQKFQG | RVTMTRNTSISTAYMELSSLRSEDTAVYYCAY | SSGWY---------------------YFDY | WGQGTLVTVSS |
| iPS:434549 | 21-225_76E11 | VH1\|1-08/D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RLTMTRNTSISTAYMELSSLRSEDTAVFYCAY | SSGWY---------------------YFDY | WGQGTLVTVSS |
| iPS:434551 | 21-225_75C4 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAS | SSGWY---------------------YFDY | WGQGTLVTVSS |
| iPS:434635 | 21-225_78E6 | VH1\|1-08/D6\|6-19\|RF1/JH4 | | QVQLVQS-GAEVKKPGASVKVSCKASG-YTFT | N-----YDIN | WVRQASGQGLEWMG | WMHPN---SGNTGYAQKFQG | RVTMTRNTSISTAYMELSSLRSEDSAVYYCAY | SSGWY---------------------KFDY | WSQGTLVTVSS |
| iPS:434649 | 21-225_78E11 | VH1\|1-08/D6\|6-19\|RF1/JH4 | | QVQLVQS-GAEVKKPGASVKVSCKASG-YTFT | N-----YDIN | WVRQATGQGLEWMG | WMNPN---SGNTGCAQKFQG | RVTMTRNTSISTAYMELSSLRSEDTAVYYCAS | SSGWN---------------------FFDY | WGQGTLVTVSS |
| iPS:434665 | 21-225_74G4 | VH1\|1-08/D6\|6-19\|RF1/JH4 | | QVQLVQS-GAEVKKPGASVKVSCKASG-YTFT | N-----YDVN | WVRQATGQGLEWMG | WMHPN---SGNTGYAQKFQG | RVTMTRNTSIRTAYMELSSLRSEDTAVYYCAS | SSGWY---------------------HFDY | WGQGTLVTVSS |
| iPS:434679 | 21-225_79G7 | VH1\|1-08/D6\|6-19\|RF1/JH4 | | QVQLVQS-GAEVKKPGASVKVSCKASG-YTFT | N-----YDIN | WVRQASGQGLEWMG | WMHPN---SGNTGYAQKFQG | RVTMTRNTSISTAYMELSSLRSEDSAVYYCAY | SSGWY---------------------KFDY | WSQGTLVTVSS |
| iPS:434685 | 21-225_79E9 | VH1\|1-08/D6\|6- | | QVQLVQS-GAEVKKPGASVKV | N-----YDIN | WVRQATGQGLEWMG | WMHPN---SGNTGYAQK | RVTMTRNTSISTAYMELSSLRSEDTAVYYCAS | SSGWY---------------------YFDY | WGQGTLVTVS |

| | | | | | | | | | | |
|---|---|---|---|---|---|---|---|---|---|---|
| **iPS:434697** | **21-225_79F12** | **VH1\|1-08/D6\|6-19\|RF1/JH4** | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAS | SSGWY---------------------FFDY | WGQGTLVTLSS |
| **iPS:434729** | **21-225_80B12** | **VH1\|1-08/D6\|6-19\|RF1/JH4** | | QVQLVQS-GAEVKKPGASVKVSCKTSG-YTFT | N-----YDIN | WVRQATGQGLEWMG | WMNPN---SGNTGYAQKFQV | RVTMTRNTSISTAYMELSSLRSEDTAVYYCAY | SSGWY---------------------IFDY | WGQGTLVTVSS |
| **iPS:434851** | **21-225_75A6** | **VH1\|1-08/D6\|6-19\|RF1/JH4** | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAY | SSGWY---------------------IFDY | WGQGTLVTVSS |
| **iPS:434909** | **21-225_85C11** | **VH1\|1-08/D6\|6-19\|RF1/JH4** | | QVQLVQS-GAEVKKPGASVKVSCKASG-YTFT | N-----YDIN | WVRQASGQGLEWMG | WMHPN---SGNTGYAQKFQG | RVTMTRNTSISTAYMELSSLRSEDSAVYYCAY | SSGWY---------------------KFDY | WSQGTLVTVSS |
| **iPS:434959** | **21-225_87E10** | **VH1\|1-08/D6\|6-19\|RF1/JH4** | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAY | SSGWY---------------------FFDY | WGQGTLVTVSS |
| **iPS:434965** | **21-225_88A1** | **VH1\|1-08/D6\|6-19\|RF1/JH4** | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAY | SSGWY---------------------YFDY | WGQGTLVTVSS |
| **iPS:434973** | **21-225_88B4** | **VH1\|1-08/D6\|6-19\|RF1/JH4** | | QVQLVQS-GAEVKKPGASVKVSCKASG-YTFT | N-----YDIN | WVRQATGQGLEWMG | WMNPN---SGDTGYAQKFQG | SVTMTRNTSITTAYMELSSLRSEDTAVYYCSY | SSGWY---------------------YFDY | WGQGTLVTVSS |
| **iPS:434997** | **21-225_88C10** | **VH1\|1-08/D6\|6-19\|RF1/JH4** | | QVQLVQS-GAEVKKPGASVRVSCKASG-YTFS | N-----YDIN | WVRQATGQGLEWMG | WMTPN---SGNTGYAQKFQG | RVTMTRNTSISTAYMELSSLRSEDTAVYYCAS | SSGWY---------------------YFDS | WGQGTLVTVSL |
| **iPS:435053** | **21-225_75F9** | **VH1\|1-08/D6\|6-19\|RF1/JH4** | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY---------------------IFDY | WGQGTLVTVSS |
| **iPS:435113** | **21-225_92E6** | **VH1\|1-08/D6\|6-19\|RF1/JH4** | | QVQLVQS-GAEVKKPGASVRVSCKASG-YTFT | N-----YDIN | WVRQATGQGLEWMG | WMHPN---SGNTGYAQKFQG | RVTMTRNTSMSTAYMELSSLRSEDTAVYYCAH | SSGWY---------------------FFDY | WGQGTLVTVSS |
| **iPS:435209** | **21-225_75A10** | **VH1\|1-08/D6\|6-19\|RF1/JH4** | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAY | SSGWY---------------------YFDY | WGQGTLVTVSS |
| **iPS:435257** | **21-225_96H5** | **VH1\|1-08/D6\|6-** | | QVQLVQS-GAEVKKPGASVKV | N-----YDIN | WVRQASGQGLEWMG | WMHPN---SGNTGYAQK | RVTMTRNTSISTAYMELSSLRSEDSAVYYCAY | SSGWY---------------------KFDY | WSQGTLVTVS |

| | | | | | | | | | | |
|---|---|---|---|---|---|---|---|---|---|---|
| **iPS:4 35267** | **21-225_96D10** | **VH1\|1-08/D6\|6-19\|RF1/JH4** | | QVQLVQS-GAEVKKPGASVRVSCKASG-YTFT | N-----YDIN | WVRQATGQGLEWMG | WMHPN---SGNTGYAQKFQG | RVTMTRNTSMSTAYMELSSLRSEDTAVYYCAH | SSGWY---------------------FFDY | WGQGTLVTVSS |
| **iPS:4 35299** | **21-225_146D4** | **VH1\|1-08/D6\|6-19\|RF1/JH4** | | QVQLVQS-GAEVKKPGASVKVSCKASG-YTFT | N-----YDIN | WVRQATGQGLEWMG | WVHPN---SGNTGYAQKFQG | RVTMTRNTSISTAYMELSSLRSEDTAVYYCAG | SSGWY---------------------YFDY | WGQGTLVTVSS |
| **iPS:4 35305** | **21-225_146C9** | **VH1\|1-08/D6\|6-19\|RF1/JH4** | | QVQLVQS-GAEVKKPGASVKVSCKASG-YTFT | N-----YDIN | WVRQATGQGLEWMG | WMHPN---SGNTGYAQKFQG | RVTMTWNTSISTAYMALSSLRSEDTAVYYCAY | SSGWY---------------------SFDY | WGQGTLVTVSS |
| **iPS:4 35309** | **21-225_146F9** | **VH1\|1-08/D6\|6-19\|RF1/JH4** | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAS | SSGWY---------------------FFDY | WGQGTLVTVSS |
| **iPS:4 35321** | **21-225_147E4** | **VH1\|1-08/D6\|6-19\|RF1/JH4** | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAS | SSGWY---------------------FFDY | WGQGTLVTVSS |
| **iPS:4 35323** | **21-225_147D5** | **VH1\|1-08/D6\|6-19\|RF1/JH4** | | QVQLVQS-GAEVKKPGASVKVSCKASG-YTFT | N-----YDIN | WVRQATGQGLEWMG | WVHPN---SGNTGYAQKFQG | RVTMTRNTSISTAYMELSSLRSGDTAVYYCAG | SSGWY---------------------YFDY | WGQGTLVTVSS |
| **iPS:4 35345** | **21-225_148G3** | **VH1\|1-08/D6\|6-19\|RF1/JH4** | | QVQLVQS-GAEVKKPGASVKVSCKASG-YTFT | N-----YDIN | WVRQASGQGLEWMG | WMHPN---SGNTGYAQNFQG | RVTMTRNTSISTAYMELSSLRSEDTAVYYCAS | SSGWY---------------------FFDY | WGQGTLVTVSS |
| **iPS:4 35353** | **21-225_148F8** | **VH1\|1-08/D6\|6-19\|RF1/JH4** | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAS | SSGWY---------------------YFDY | WGQGTLVTVSS |
| **iPS:4 35369** | **21-225_149A2** | **VH1\|1-08/D6\|6-19\|RF1/JH4** | | QVQLVQS-GAEVKKPGASVKVSCKASG-YTFT | N-----YDIN | WVRQATGQGLEWMG | WVHPN---SGNTGYAQKFQG | RVTMTRNTSISTAYMELSSLRSEDTAVYYCAG | SSGWY---------------------YFDY | WGQGTLVTVSS |
| **iPS:4 35373** | **21-225_149E3** | **VH1\|1-08/D6\|6-19\|RF1/JH4** | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLTSEDTAVYYCAY | SSGWY---------------------WFDY | WGQGTLVTVSS |
| **iPS:4 35375** | **21-225_149H4** | **VH1\|1-08/D6\|6-19\|RF1/JH4** | | QVQLVQS-GAEVKKPGASVKVSCKASG-YTFT | N-----YDIN | WVRQATGQGLEWMG | WMHPN---SGNTDYAQKFQG | RVTMTRNTSITTVYMELSSLRSEDTAVYYCTF | SSGWY---------------------YFDY | WGQGTLVTVSS |
| **iPS:4 35399** | **21-225_150D2** | **VH1\|1-08/D6\|6-** | | QVQLVQS-GAEVKKPGASVKV | N-----YDIN | WVRQATGQGLEWMG | WMHPN---SGNTGYAQK | RVTMTRNTSISTAYMELSSLRSEDTAVYYCAY | SSGWY---------------------YFDY | WGQGTLVTVS |

| | | | | | | | | | | |
|---|---|---|---|---|---|---|---|---|---|---|
| iPS:4 35405 | 21-225_150B7 | VH1\|1-08/D6\|6-19\|RF1/JH4 | | QVQLVQS-GAEVKKPGASVTVSCKASG-FPFT | N-----YDIN | WVRQATGQGLEWMG | WMHPN---SGNTGYAQKFQG | RVTMTRNTSISTAYLELSSLRSEDTAVYYCAS | SSGWY--------------------FFDY | WGQGTLVTVSS |
| iPS:4 35433 | 21-225_152E3 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAS | SSGWY--------------------FFDY | WGQGTLVTVSS |
| iPS:4 35435 | 21-225_152H3 | VH1\|1-08/D6\|6-19\|RF1/JH4 | | QVQLVQS-GAEVKKPGASVKVSCKASG-YTFP | N-----YDIN | WVRQATGQGLEWMG | WMHPN---SGNTGYAQKFQG | RVTMTRNTSISTAYMELSSLRSEDTAVYYCAY | SSGWY--------------------WFDY | WGQGTLVTVSS |
| iPS:4 35459 | 21-225_152E12 | VH1\|1-08/D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAY | SSGWY--------------------YFDY | WGQGTLVTVSS |
| iPS:4 35471 | 21-225_153F11 | VH1\|1-08/D6\|6-19\|RF1/JH4 | | QVQLVQS-GAEVKEPGASVKVSCKASG-YTFT | N-----YDIN | WVRQATGQGLEWMG | WMHPN---SGNTGYAQKFQG | RVTMTRNTSINTAYMELSSLRSEDTAVYYCAY | SSGWY--------------------FFDN | WGQGTLVTVSS |
| iPS:4 35475 | 21-225_154H6 | VH1\|1-08/D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAY | SSGWY--------------------IFDY | WGQGTLVTVSS |
| iPS:4 35481 | 21-225_154A11 | VH1\|1-08/D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAS | SSGWY--------------------YFDY | WGQGTLVTVSS |
| iPS:4 35491 | 21-225_155E5 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGSTGYAQRFQG | RVTMTRNTSISTAYMELSSLRSEDTAVYYCAF | SSGWY--------------------YFDY | WGQGTLVTVSS |
| iPS:4 35495 | 21-225_155B6 | VH1\|1-08/D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAY | SSGWY--------------------YFDY | WGQGTLVTVSS |
| iPS:4 35501 | 21-225_156H1 | VH1\|1-08/D6\|6-19\|RF1/JH4 | | QVQLVQS-GAEVKKPGASVKVSCKASG-YTFT | N-----YDIN | WVRQATGQGLEWMG | WVHPN---SGNTGYAQKFQG | RVTMTRNTSISTAYMELSSLRSEDTAVYYCAG | SSGWY--------------------YFDY | WGQGTLVTVSS |
| iPS:4 35557 | 21-225_158B12 | VH1\|1-08/D6\|6-19\|RF1/JH4 | | QVQLVQS-GAEVRKPGASVKVSCKASG-YTFT | N-----YDIN | WVRQATGQGLEWMG | WMHPN---SGNTGYAQKFQG | RFTMTRNTSISTAYMELSSLRSEDTAVYYCAY | SSGWY--------------------RFDY | WGQGTLVTVSS |
| iPS:4 35589 | 21-225_160A4 | VH1\|1-08/D6\|6- | | QVQLVQS-GTEVKKPGASVKV | N-----YDIN | WVRQATGQGPEWMG | WMHPN---SGNTGYPQK | RVTMTRNTSISTAYMELSSLRSEDTAVYYCAY | SSGWY--------------------IFDY | WGQGTLVTVS |

| | | | | | | | | | | |
|---|---|---|---|---|---|---|---|---|---|---|
| **iPS:4 35623** | **21-225_162D5** | **VH1\|1-08/D6\|6-19\|RF1/J H4** | | QVQLVQS-GSEVKKPGASVKVSCKASG-YTFT | N-----YDIN | WVRQATGQGLEWMG | WMHPN---SGNTGYAQKFQG | RVTMTRNTSIDTAYMELSSLSSEDTAVYFCAF | SSGWY---------------------FFDY | WGQGTLVTVSS |
| **iPS:4 35627** | **21-225_162F6** | **VH1\|1-08/D6\|6-19\|RF1/J H4** | | QVQLVQS-GAEVRKPGASVKVSCKASG-YTFT | N-----YDIN | WVRQATGQGLEWMG | WMHPN---SGNTGYAQKFQG | RFTMTRNTSISTAYMELSSLRSEDTAVYYCAY | SSGWY---------------------RFDY | WGQGTLVTVSS |
| **iPS:4 35649** | **21-225_165H2** | **VH1\|1-08/D6\|6-19\|RF1/J H4** | | QVQLVQS-GAEVKKPGASVKVSCKASG-YTFT | H-----YDIN | WVRQATGQGLEWVG | WMHPN---SHKTGYAQKFQG | RVTMTRNTSNSTAYMDLSSLRSEDTAVYYCAY | SSGWY---------------------MFDY | WGQGTLVTVSS |
| **iPS:4 35727** | **21-225_172E11** | **VH1\|1-08/D6\|6-19\|RF1/J H4** | | QVQLVQS-GAEVKKPGASVMVSCKASG-YTFT | N-----YDIN | WVRQATGQGLEWMG | WMHPN---SGNTGYAQKFQG | RVTMTRNTSISTAYMELSSLRSEDTAVYYCAY | SSGWY---------------------RFDY | WGQGTLVTVSS |
| **iPS:4 35751** | **21-225_175D10** | **VH1\|1-08/D6\|6-19\|RF1/J H4** | | QVQLVQS-GAEVKKPGASVKVSCKASG-YTFT | N-----YDLN | WVRQATGQGLEWMG | WMHPN---SGNTGYAQKFQG | RVTMTRNTSISTVYMELSSLRSEDTAVYYCAY | SSGWY---------------------YFDY | WGQGTLVTVSS |
| **iPS:4 35773** | **21-225_177B12** | **VH1\|1-08/D6\|6-19\|RF1/J H4** | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY---------------------YFDF | WGQGTLVTVSS |
| **iPS:4 35801** | **21-225_181E5** | **VH1\|1-08/D6\|6-19\|RF1/J H4** | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAS | SSGWY---------------------IFDY | WGQGTLVTVSS |
| **iPS:4 35841** | **21-225_191D8** | **VH1\|1-08/D6\|6-19\|RF1/J H4** | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAH | SSGWY---------------------YFDY | WGQGTLVTVSS |
| **iPS:4 35855** | **21-225_191G3** | **VH1\|1-08/D6\|6-19\|RF1/J H4** | | QVQLVQS-GAEVKKPGASVKVSCKASG-YTFT | N-----YDIN | WVRQATGQGLEWMG | RMNPN---SGNTGYAQKFQG | RVTMTRNTSISTAYMELSSLRSEDTAVYYCAH | SSGWY---------------------IFDY | WGQGTLVTVSS |
| **iPS:4 35915** | **21-225_190H4** | **VH1\|1-08/D6\|6-19\|RF1/J H4** | | QVQLVQS-GAEVKKPGASVKVSCKASG-NTFT | N-----YDIN | WVRQATGQGLEWMG | WMNPN---SGNTGYAQKFQG | RVTMTRNTSISTAYMELSSLRSEDTAVYYCAH | SSGWY---------------------IFDY | WGQGTLVTVSS |
| **iPS:4 35925** | **21-225_190D7** | **VH1\|1-08/D6\|6-19\|RF1/J H4** | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAS | SSGWY---------------------FFDY | WGQGTLVTVSS |
| **iPS:4 36021** | **21-225_193G4** | **VH1\|1-08/D6\|6-** | | QVQLVQS-GAEVKKPGASVKV | N-----YDIN | WVRQATGQGLEWMG | WMNPN---SGNTGYAQK | RVTMTRNTSIRTAYMELNSLRSEDTAVYYCAS | SSGWY---------------------FFDY | WGQGTLVTVS |

| | | | | | | | | | | |
|---|---|---|---|---|---|---|---|---|---|---|
| iPS:4 36150 | 21-225_197H4 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAH | SSGWY--------------------YFDY | WGQGTLVTVSS |
| iPS:4 36154 | 21-225_197C6 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAH | SSGWY--------------------YFDY | WGQGTLVTVSS |
| iPS:4 36272 | 21-225_201F5 | VH1\|1-08/D6\|6-19\|RF1/JH4 | | QVQLVQS-GAAVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY--------------------YFDY | WGQGTLVTVSS |
| iPS:4 36550 | 21-225_224D8 | VH1\|1-08/D6\|6-19\|RF1/JH4 | | QVQLVQS-GAEVKKPGASVKVSCKASG-YTFT | N-----YDIN | WVRQATGQGLEWMG | WLYPN---SGNTGYAQKFQG | RVTMTRNTSISTAYMELSSLRSEDTAVYYCAY | SSGWY--------------------YFDY | WGQGTLVIVSS |
| iPS:4 36554 | 21-225_224C10 | VH1\|1-08/D6\|6-19\|RF1/JH4 | | QVQLVQS-GAEVKKPGASVKVSCKASG-STFT | N-----YDIN | WVRQATGQGLEWMG | WMHPN---SGNTGYAQKFQG | RVTMTRNTSISTAYMELSSLRSEDTAVYYCAY | SSGWY--------------------KFDY | WGQGTLVTVSS |
| iPS:4 36560 | 21-225_224F11 | VH1\|1-08/D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAY | SSGWY--------------------KFDY | WGQGTLVTVSS |
| iPS:4 36574 | 21-225_225F5 | VH1\|1-08/D6\|6-19\|RF1/JH4 | | QVQLVQS-GAEVKKPRASVKVSCKASG-HTFT | N-----YDIN | WVRQATGQGLEWMG | WMHPN---SGNTGFAQKFQG | RVTMTRNTSISTAYMELSSLRSEDTAVYYCAY | SSGWY--------------------RFDY | WGQGTLVTVSS |
| iPS:4 36584 | 21-225_225B9 | VH1\|1-08/D6\|6-19\|RF1/JH4 | | QVQLVQS-GTEVRKPGASVKVSCKASG-YTFT | N-----YDIN | WVRQATGQRLEWMG | WMHPN---SGNTGYAQKFRG | RVTMTRNTSINTAYMELNSLRSEDTAVYYCAY | SSGWT--------------------LFDY | WGQGTLVTVSS |
| iPS:4 36586 | 21-225_225F11 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSINTAYMELSSLRSEDTAVYYCAY | SSGWY--------------------RFDY | WGQGTLVTVSS |
| iPS:4 36588 | 21-225_225F12 | VH1\|1-08/D6\|6-19\|RF1/JH4 | | QVQLVQS-GAEVKKPGASVKVSCKASG-YTFT | H-----YDIN | WVRQATGQGLEWMG | WMHPN---SGNTGYAQKFQG | RVTMARNTSISTAYMELSSLRSEDTAVYYCAY | SSGWY--------------------KFDY | WGQGTLVTVSS |
| iPS:4 36590 | 21-225_225H12 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY--------------------KFDY | WGQGTLVTVSS |
| iPS:4 36598 | 21-225_226D6 | VH1\|1-08/D6\|6- | | QVQLVQS-GAEVKKPGASVKV | N-----YDIN | WVRQATGQGLEWMG | WMHPN---SGNTGYAQK | RVTMTRNTSISTAYMELSSLRSEDTAVYYCAY | SSGWY--------------------KFDY | WGQGTLVTVS |

| | | | | | | | | | | |
|---|---|---|---|---|---|---|---|---|---|---|
| iPS:4 36600 | 21-225_226F6 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSINTAYMELSSLRSEDTAVYYCAY | SSGWY---------------------RFDY | WGQGTLVTVSS |
| iPS:4 36616 | 21-225_226D11 | VH1\|1-08/D6\|6-19\|RF1/JH4 | | QVQLVQS-GAEVKKPGASVKVSCRSSG-YTFT | N-----YDIN | WVRQATGQGLEWMG | WMHPN---SGNTGYAQKFQG | RVTMTRNTSISTAYMELSSLRSEDTAVYYCAY | SSGWY---------------------YFDY | WGQGTLVTVSS |
| iPS:4 36622 | 21-225_226A12 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY---------------------YFDY | WGQGTLVTVSS |
| iPS:4 36636 | 21-225_227E6 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY---------------------KFDY | WGQGTLVTVSS |
| iPS:4 36638 | 21-225_227C7 | VH1\|1-08/D6\|6-19\|RF1/JH4 | | QVQLVQS-GAEVKKPRASVKVSCKASG-HTFT | N-----YDIN | WVRQATGQGLEWMG | WMHPN---SGNTGYAQKFQG | RVTMTRNTSISTAYMELSSLRSEDTAVYYCAY | SSGWY---------------------RFDY | WGQGTLVTVSS |
| iPS:4 36646 | 21-225_227D11 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY---------------------YFDY | WGQGTLVTVSS |
| iPS:4 46086 | 21-225_94D8 | VH1\|1-08/D6\|6-19\|RF1/JH4 | | QVQLVQS-GAEVKKPGASVKVSCKTSG-YTFT | N-----YDIN | WVRQATGQGLEWMG | WMNPN---SGNTGYAQKFQV | RVTMTRNTSISTAYMEVSSLRSEDTAVYYCAY | SSGWY---------------------IFDY | WGQGTLVTVSS |
| iPS:4 51116 | 21-225_164A4 | VH1\|1-08/D6\|6-19\|RF1/JH4 | | QVQLVQS-GAEVKKPGASVKVSCKASG-FTFP | N-----YDIN | WVRQATGQGLEWMG | WMHPN---SGNTGYAQKFQG | RVTMTRNTSISTAYMELSSLRSEDTAVYYCAS | SSGWY---------------------FFDY | WGQGTLVTVSS |
| iPS:4 51124 | 21-225_74F6 | VH1\|1-08/D6\|6-19\|RF1/JH4 | | QVQLVQS-GAEVKKPGASVRVSCKASG-YTFT | N-----YDIN | WVRQATGQGLEWMG | WMHPN---SGNTGYAQKFQG | RVTMTRNTSMSTAYMELSSLRSEDTAVYYCAH | SSGWY---------------------FFDY | WGQGTLVTVSS |
| iPS:4 51127 | 21-225_164A7 | VH1\|1-08/D6\|6-19\|RF1/JH4 | | QVQLVQS-GAEVKKPGASVKVSCKASG-YTFT | N-----YDVN | WVRQATGQGLEWMG | WMHPN---SGNTGYAQKFQG | RVTMTRNTSTSTAYMELSSLRSEDSAVYYCAS | SSGWY---------------------LFDY | WGQGTLVTVSS |
| iPS:4 51131 | 21-225_160A7 | VH1\|1-08/D6\|6-19\|RF1/JH4 | | QVQLVQS-GAEVKKPGASVKVSCKASG-YTFT | N-----YDIN | WVRQATGQGLEWMG | WMHPH---SGNTGYAQKFQG | RVTMTRNTSINTAYMELSSLRSEDTAVYYCAH | SSGWY---------------------YFDY | WGQGTLVTVSS |
| iPS:3 92786 | 21-225_24E1 | VH1\|1-08/D6\|6- | | QVQLVQS-GAEVKKPGASVKV | N-----YDIN | WVRQATGQGLEWMG | WMHPN---SGNTGYAQR | RVTMTRNTSISTAYMELSSLRSEDTAVYYCAT | SSGWE---------------------VFDY | WGQGTLVTVS |

| | | | | | | | | | | |
|---|---|---|---|---|---|---|---|---|---|---|
| **iPS:3 92886** | **21-225_23A12** | **VH1\|1-08/D6\|6-19\|RF1/JH4** | | QVQLVQS-GAEVKKPGASVKVSCKASG-YPFT | N-----YDIN | WVRQATGQGLEWMG | WMHPN---SGNTGYAQKFQG | RVTMTRNTSINTAYMELSSLRSEDTAVYYCAG | SSGWY---------------------YFDY | WGQGTLVTVSS |
| **iPS:3 92928** | **21-225_25A4** | **VH1\|1-08/D6\|6-19\|RF1/JH4** | | QVLLVQS-GAEVKRPGASVKVSCKASG-YTFT | N-----YDIN | WVRQATGQGLEWMG | WMYPN---SGNTGYAQKFQG | RVTMTRNTSISTAYMELSSLRSEDTAVYYCAS | SSGWY---------------------YFDY | WGQGTLVTVSS |
| **iPS:3 92936** | **21-225_28B6** | **VH1\|1-08/D6\|6-19\|RF1/JH4** | | QVLLVQS-GAEVKRPGASVKVSCKASG-YTFT | N-----YDIN | WVRQATGQGLEWMG | WMHPD---SGNTGYAQKFQG | RVTMTRNTSISTAYMELSSLRSEDTAVYYCAS | SSGWY---------------------YFDY | WGQGTLVTVSS |
| **iPS:3 92960** | **21-225_29E6** | **VH1\|1-08/D6\|6-19\|RF1/JH4** | | QVLLVQS-GAEVKRPGASVKVSCKASG-YTFT | N-----YDIN | WVRQATGQGLEWMG | WMHPN---SGNTGYAQKFQG | RVTMTRNTSISTAYMELSSLRSEDTAVYYCAS | SSGWY---------------------YFDY | WGQGTLVTVSS |
| **iPS:3 92992** | **21-225_26C4** | **VH1\|1-08/D6\|6-19\|RF1/JH4** | | QVQLVQS-GAEVKKPGASVKVSCKASG-YTFT | N-----YDIN | WVRQASGQGLEWMG | WMNPN---SGNTGYAQRFQG | RVTMTRNTSINTAYMELSSLRSEDTAVYYCAS | SSGWY---------------------YFDY | WGQGTLVTVSS |
| **iPS:3 93088** | **21-225_33D1** | **VH1\|1-08/D6\|6-19\|RF1/JH4** | | QVQLVQS-GAEVKKPGASVKVSCKASG-YTFT | N-----YDIN | WVRQATGQGLEWMG | WMHPN---SGNTGFAQKFRG | RVTMTRNTSISTAYMELSSLRSEDTAVYYCAS | SSGWY---------------------FFDY | WGQGTLVTVSS |
| **iPS:3 93144** | **21-225_34D2** | **VH1\|1-08/D6\|6-19\|RF1/JH4** | | QVQLVQS-GAEVKKPGASVKVSCKASG-YTFT | N-----YDIN | WVRQATGQGLEWVG | WLHPN---SGTTGFAQKFRG | RVTMTRNTSISTAYLELSSLRSEDTAVYYCAS | SSGWY---------------------FFDY | WGQGTLVTVSS |
| **iPS:3 93368** | **21-225_29H8** | **VH1\|1-08/D6\|6-19\|RF1/JH4** | | QVQLVQS-GAEVQKPGASVKVSCKASG-YTFT | N-----YDIN | WVRQATGQGLEWMG | WMNPN---SGNTGYAQRFQG | RVTMTRNTSISAAYMELSSLRSEDTAVYYCAS | SSGWY---------------------YFDY | WGQGTLVTVSS |
| **iPS:3 93942** | **21-225_11E5** | **VH1\|1-08/D6\|6-19\|RF1/JH4** | | QVQLVQS-GPEVKKPGASVKVSCKASG-YTFT | N-----YDIN | WVRQATGQGLEWMG | WMHPN---SGATGYAQRFQG | RVTMTRNTSISTAYMELSSLRSEDTAVYYCAT | SSGWE---------------------VFDY | WGQGTLVTVSS |
| **iPS:3 94085** | **21-225_8B11** | **VH1\|1-08/D6\|6-19\|RF1/JH4** | | QVQLVQS-GAEVKKPGASVKVSCKASG-YTFT | N-----YDIN | WVRQAAGQGLEWMG | WMHPN---SGNTGYAQKFQG | RVTMTRNTSRSTAYMELSSLRSEDTAVYYCAY | SSGWY---------------------FFDY | WGQGTLVTVSS |
| **iPS:3 98496** | **21-225_22D2** | **VH1\|1-08/D6\|6-19\|RF1/JH4** | | QVQLVQS-GAEVKKPGASVKVSCKASG-YTFI | N-----YDIN | WVRQATGQGLEWMG | WMHPD---SGNTGYAQKFQG | RVTMTRNTSISTAYMELSSLRSEDTAVYYCAY | SSGWY---------------------YFDY | WGQGTLVTVSS |
| **iPS:3 98522** | **21-225_32A1** | **VH1\|1-08/D6\|6-** | | QVQLVQS-GAEVKKPGASVKV | N-----YDIN | WVRQATGQGLEWMG | WMNPN---SGNTGYAQK | RVTMTRNTSISTAYMELSSLRSEDTAVYYCAS | SSGWY---------------------FFDY | WGQGTLVTVS |

| | | | | | | | | | | |
|---|---|---|---|---|---|---|---|---|---|---|
| iPS:398524 | 21-225_32A5 | VH1\|1-08/D6\|6-19\|RF1/JH4 | | QVQLVQS-GAEVKKPGASVKVSCKASG-YTFT | N-----YDIN | WVRQATGQGLEWMG | WMHPN---SGNTGFAQKFRG | RVTMTRNTSISTAYMELSSLRSEDTAVYYCSS | SSGWY---------------------FFDY | WGQGTLVTVSS |
| iPS:398538 | 21-225_34H7 | VH1\|1-08/D6\|6-19\|RF1/JH4 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELNSLRSEDTAVYYCAS | SSGWY---------------------FFDY | WGQGTLVTVSS |
| | | Germline | | H_FR1 | H_CDR1 | H_FR2 | H_CDR2 | H_FR3 | H_CDR3 | H_FR4 |
| | VH1\|1-02/D1\|1-1\|RF1/JH4 | | 32211 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | GTTGT---------------------YFDY | WGQGTLVTVSS |
| iPS:473253 | 21-225_7C3_LC1 | VH1\|1-02/D1\|1-1\|RF1/JH4 | | QVQLVQS-GAEVKKPGASVKVSCKASG-YTFN | D-----YYLH | WVRQAPGQGLEWMG | WIHPN---SGGTNYAQKFQG | RVTMTRDTSISTAYMELSSLRSDDTAVYSCAR | DGTS----------------------SFDY | WGQGTLVTVSS |
| iPS:473254 | 21-225_7C3_LC2 | VH1\|1-02/D1\|1-1\|RF1/JH4 | | QVQLVQS-GAEVKKPGASVKVSCKASG-YTFN | D-----YYLH | WVRQAPGQGLEWMG | WIHPN---SGGTNYAQKFQG | RVTMTRDTSISTAYMELSSLRSDDTAVYSCAR | DGTS----------------------SFDY | WGQGTLVTVSS |
| iPS:473255 | 21-225_9F12_LC1 | VH1\|1-02/D1\|1-1\|RF1/JH4 | | QVQLVQS-GAEVKKPGASVKVSCKASG-YTFA | D-----YYLH | WVRQAPGQGLEWMG | WIHPN---SGGTNFAQKFQG | RVTMTRDTSISTAYLELSSLRSDDTAFYYCAR | DGTS----------------------SFDY | WGQGTLVTVSS |
| iPS:473256 | 21-225_9F12_LC2 | VH1\|1-02/D1\|1-1\|RF1/JH4 | | QVQLVQS-GAEVKKPGASVKVSCKASG-YTFA | D-----YYLH | WVRQAPGQGLEWMG | WIHPN---SGGTNFAQKFQG | RVTMTRDTSISTAYLELSSLRSDDTAFYYCAR | DGTS----------------------SFDY | WGQGTLVTVSS |
| iPS:426108 | 21-225_10G6 | VH1\|1-02/D1\|1-1\|RF1/JH4 | | QVQLVQS-GAEVKKPGASVKVSCKASG-YTFT | A-----YHMH | WVRQAPGQGLEWMG | WINPN---NNGTNYAQKFQG | RVTMTRDTSISTAYMELSRLRSDDTAVYYCGR | DVTS----------------------SFDY | WGQGTLVTVSS |
| iPS:426110 | 21-225_12E9 | VH1\|1-02/D1\|1-1\|RF1/JH4 | | QVQLVQS-GAEVKKPGASVKVSCKASG-YTFT | D-----YYLH | WVRQAPGQGLEWMG | WVHPN---SGGTNFAQKFQD | RVTMTRDTSISTAYMELSSLRSDDTAIYSCAR | DGTS----------------------SFDY | WGQGTLVTVSS |
| iPS:453451 | 21-225_52G11 | VH1\|1-02/D1\|1-1\|RF1/JH4 | | QVQLVQS-GAEVKKPGASVKVSCKASG-YTFT | G-----YYLH | WVRQAPGQGLEWMG | WINPN---RNGTNYAQKFQG | RVTMTRDTSISTAFMELSRLRSDDTAVYYCAR | DGTS----------------------SFDY | WGQGTLVTVSS |
| iPS:453453 | 21-225_53F2 | VH1\|1-02/D1\|1-1\|RF1/JH4 | | QVQLVQS-GAEVKKPGASVKVSCKASG-YTFT | G-----YYLH | WVRQAPGQGLEWMG | WINPN---RNGTNYAQNFQG | RVTMTRDTSISTAYMELSRLKSDDTAVYYCAR | DGTS----------------------SFDY | WGQGTLVTVSS |
| iPS:434035 | 21-225_49F10 | VH1\|1-02/D1\|1- | | QVQLVQS-GAEVKKPGASVKV | G-----YHMH | WVRQAPGQGLEWMG | WINPN---NNATNYAQN | RVTLTRDTSISTAYMELSRLRSDDTAVYYCAR | DGTS----------------------SFDF | WGQGTLVTVS |

| | | | | | | | | | | |
|---|---|---|---|---|---|---|---|---|---|---|
| iPS:434065 | 21-225_50D4 | VH1\|1-02/D1\|1-1\|RF1/JH4 | | QVQLVQS-GAEVKKPGASVKVSCKASG-YTFT | G-----YHMH | WVRQAPGQGLEWMG | WINPN---NNATNYAQSFQG | RVTLTRDTSISTAYMELSRLRSDDTAVYYCAR | DGTS----------------------SFDF | WGQGTLVTVSS |
| iPS:434069 | 21-225_51E9 | VH1\|1-02/D1\|1-1\|RF1/JH4 | | QVQLVQS-GAEVKKPGASVKVSCKASR-YTFT | G-----YHIH | WVRQAPGQGLEWMG | WINPN---TNGTQYAQKFQG | RVTMTRDTSISTAYMELSRLRSDDTAVYYCAR | DGTS----------------------SFDY | WGQGTLVTVSS |
| iPS:434079 | 21-225_52B1 | VH1\|1-02/D1\|1-1\|RF1/JH4 | | QVQLVQS-GAEVKKPGASVKVSCKASG-YTFT | G-----YHMQ | WVRQAPGQGLEWMG | WINPN---SGATNYAQNFQG | RVTMTRDTSISTAYLDLSRLRSDDTAVYYCAR | DGTS----------------------SFDY | WGQGTLVTVSS |
| iPS:434097 | 21-225_52H10 | VH1\|1-02/D1\|1-1\|RF1/JH4 | | QVQLVQS-GAEVKKPGASVKVSCKASG-YTFT | G-----YHMQ | WVRQAPGQGLEWMG | WINPN---NGGTQYAQKFQG | RVTMTRDTSISTAYMELSRLRSDDTAVYYCAR | DGTS----------------------SFDY | WGQGTLVTVSS |
| iPS:434123 | 21-225_53F7 | VH1\|1-02/D1\|1-1\|RF1/JH4 | | QVQLVQS-GAEVKKPGASVKVSCKASG-YTFT | G-----YHMH | WVRQAPGQGLEWMG | WINPN---NNGTNYAQKFQG | RVTMTRDTSISTAYMELNRLTSDDTAVYYCAR | DGTS----------------------SFDY | WGQGTLVTVSS |
| iPS:434189 | 21-225_56E5 | VH1\|1-02/D1\|1-1\|RF1/JH4 | | QVQLVQS-GAEVKKPGASVKVSCQASG-YTFT | G-----YHMH | WVRQAPGQGLEWMG | WINPN---NNATNYAQKFQG | RVTMTRDTSISTAYMELRRLRSDDTAVYHCAR | DGTS----------------------SFDY | WGQGTLVTVSS |
| iPS:435677 | 21-225_169C10 | VH1\|1-02/D1\|1-1\|RF1/JH4 | | QVQLVQS-GAEVKKPGASVRVSCKASG-YTFT | G-----YFMH | WVRQAPGQGLEWMG | WIKPK---SGGTNSAQRFQG | RVTMTRDTSINTAYMELNRLRSDDTAVYYCAR | GGTTVAT-----------------WGVFDY | WGQGTLVTVSS |
| iPS:435699 | 21-225_170D6 | VH1\|1-02/D1\|1-1\|RF1/JH4 | | QVQLVQS-GAEVKKPGASVRVSCKASG-YTFT | G-----YFIH | WVRQAPGQGLEWMG | WIKPN---SGGTNSAQRFQG | RVTMTRDTSINTAYMELNRLRSDDTAVYYCAR | GGTTVAT-----------------WGVFDY | WGQGTLVTVSS |
| iPS:435797 | 21-225_181G2 | VH1\|1-02/D1\|1-1\|RF1/JH4 | | QVQLVQS-GAEVKTPGASVKVSCRASG-YTFT | S-----YNMH | WVRQVPGQGLEWMG | WINPN---NGGSNYTQKFQG | RVTMTRDTSISTAYMELSRLRSDDTAVYYCAR | KF--------------------------GD | WGQGTLVTVSS |
| iPS:435877 | 21-225_184E7 | VH1\|1-02/D1\|1-1\|RF1/JH4 | | QVQLVQS-GAEVKTPGASVKVSCRASG-YTFT | S-----YNMH | WVRQVPGQGLEWMG | WINPN---NGGSNYTQKFQG | RVTMTRDTSISTAYMELSRLRSDDTAVYYCAR | KF--------------------------GD | WGQGTLVTVSS |
| iPS:435885 | 21-225_185E10 | VH1\|1-02/D1\|1-1\|RF1/JH4 | | QVQLVQS-GAEVKTPGASVKVSCRASG-YTFT | S-----YNMH | WVRQVPGQGLEWMG | WINPN---NGGSNYTQKFQG | RVTMTRDTSISTAYMELSRLRSDDTAVYYCAR | KF--------------------------GD | WGQGTLVTVSS |
| iPS:435891 | 21-225_188H5 | VH1\|1-02/D1\|1- | | QVQLVQS-GAEVKTPGASVKV | S-----YNMH | WVRQVPGQGLEWMG | WINPN---SGGSNYTQK | RITMTRDTSISTAYMELSRLRSDDTAVYYCAR | KF--------------------------GD | WGQGTLVTVS |

| | | | | | | | | | | |
|---|---|---|---|---|---|---|---|---|---|---|
| iPS:435897 | 21-225_188B9 | VH1\|1-02/D1\|1-1\|RF1/JH4 | | QVQLVQS-GAEVKTPGASVKVSCRASG-YTFT | S-----YNMH | WVRQVPGQGLEWMG | WINPN---SGGSNYTQKFQG | RVTMTRDTSISTAYMELSRLRSDDTAVYYCAR | KF-------------------------GD | WGQGTLVTVSS |
| iPS:436400 | 21-225_213H7 | VH1\|1-02/D1\|1-1\|RF1/JH4 | | QVQLVQS-GAEVKKPGASVKVSCKASG-YTFT | G-----YHMH | WVRQAPGQGLEWMG | WINPK---SDGTNYAQKFQG | RVTMTRDTSISTAYMELSRLRSDDTAVYYCAR | EKPGS--------------------YYKY | WGQGTLVTVSS |
| iPS:436488 | 21-225_221A6 | VH1\|1-02/D1\|1-1\|RF1/JH4 | | QVQLVQS-GAEVKKPGASVKVSCKTSG-YTFT | G-----YYMH | WVRQAPGQGLEWMG | WIHPN---SGGTNYAQKFQG | RVTLTRDTSISTAYMDLSRLRSDDTAVYYCAR | DGTS----------------------SFDY | WGQGTLVTVSS |
| iPS:436494 | 21-225_221F12 | VH1\|1-02/D1\|1-1\|RF1/JH4 | | QVQLVQS-GAEVKKPGASVKVSCKTSG-YTFT | G-----YYMH | WVRQAPGQGLEWMG | WIHPN---SGGTNYAQKFQG | RVTLTRDTSISTAYMDLSRLRSDDTAVYYCAR | DGTS----------------------SFDY | WGQGTLVTVSS |
| iPS:436496 | 21-225_222E1 | VH1\|1-02/D1\|1-1\|RF1/JH4 | | QVQLVQS-GAEVKKPGASVKVSCKTSG-YTFT | G-----YYMH | WVRQAPGQGLEWMG | WIHPN---SGGTNYAQKFQG | RVTLTRDTSISTAYMDLSRLRSDDTAVYYCAR | DGTS----------------------SFDY | WGQGTLVTVSS |
| iPS:436508 | 21-225_222F7 | VH1\|1-02/D1\|1-1\|RF1/JH4 | | QVQLVQS-GAEVKKPGASVKVSCKTSG-YTFT | G-----YYMH | WVRQAPGQGLEWMG | WIHPN---SGGTNYAQKFQG | RVTLTRDTSISTAYMDLSRLRSDDTAVYYCAR | DGTS----------------------SFDY | WGQGTLVTVSS |
| iPS:436516 | 21-225_222C12 | VH1\|1-02/D1\|1-1\|RF1/JH4 | | QVQLVQS-GAEVKKPGASVKVSCKTSG-YTFT | G-----YYMH | WVRQAPGQGLEWMG | WIHPN---SGGTNYAQKFQG | RVTLTRDTSISTAYMDLSRLRSDDTAVYYCAR | DGTS----------------------SFDY | WGQGTLVTVSS |
| iPS:437264 | 21-225_171H12 | VH1\|1-02/D1\|1-1\|RF1/JH4 | | QVQLVQS-GAEVKKPGASVRVSCKASG-YTFT | G-----YFMH | WVRQAPGQGLEWMG | WIKPK---SGGTNSAQRFQG | RVTMTRDTSINTAYMELNRLRSDDTAVYYCAR | GGTTVAT-----------------WGVFDY | WGQGTLVTVSS |
| iPS:437266 | 21-225_177A5 | VH1\|1-02/D1\|1-1\|RF1/JH4 | | QVQLVQS-GAEVKKPGASVRVSCKASG-YTFT | G-----YFMH | WVRQAPGQGLEWMG | WIKPK---SGGTNSAQRFQG | RVTMTRDTSINTAYMELNWLRSDDTAVYYCAR | GGTTVAT-----------------WGVFDY | WGQGTLVTVSS |
| iPS:393080 | 21-225_34F3 | VH1\|1-02/D1\|1-1\|RF1/JH4 | | QVQLVQS-GAEVKKPGASVKVSCKASG-YTFT | G-----YHMH | WVRQAPGQGLDWMG | WINPN---SGGTNYAQKFQG | RVTMTRDTSISTAYMELSRLRSDDTAVYYCAR | DGTS----------------------SFDY | WGQGTLVTVSS |
| iPS:393084 | 21-225_35C6 | VH1\|1-02/D1\|1-1\|RF1/JH4 | | QVQLVQS-GTEVKKPGASVKVSCKASG-YTFT | G-----DYMH | WVRQAPGQGLEWMG | WISPK---NGGTNYAQKFQG | RVTMTRDTSISTAYMELNRLRSDDTAVYYCAR | DGTG----------------------SFDY | WGQGTLVTVSS |
| iPS:393086 | 21-225_36H5 | VH1\|1-02/D1\|1- | | QVQLVQS-GAEVKKPGASMKV | D-----YHMH | WVRQAPGQGLEWMG | WINPN---RGGTNYAQK | RVTMTRDTSISTAYMELSRLRSDDTAVYFCAR | DGTG----------------------SFDY | WGQGTLVTVS |

| | | | | | | | | | | |
|---|---|---|---|---|---|---|---|---|---|---|
| iPS:3 93098 | 21-225_35G6 | VH1\|1-02/D1\|1-1\|RF1/JH 4 | | QVQLVQS-GADVKKPGASVKV SCKASG-YTFT | D-----YHIH | WVRQAPGQ GLEWMG | WINPN---NGGTHYAQE FQG | RVTMTRDTSISTAYMELSSL RSDDTAVYYCAR | DGTG-----------------------SFDY | WGQGT LVTVS S |
| iPS:3 93112 | 21-225_33G1 | VH1\|1-02/D1\|1-1\|RF1/JH 4 | | QVQLAQS-GAEVKKPGASVKV SCKASG-YTFT | G-----YYMH | WVRQAPGQ GLEWMG | WISPN---NGGTNYAQK FQG | RVTMTRDTSISTAYMELSRL RSDDTAVYYCAR | DGTG-----------------------SFDY | WGQGT LVTVS S |
| iPS:3 93116 | 21-225_34G7 | VH1\|1-02/D1\|1-1\|RF1/JH 4 | | QVQLVQS-GAEVKKPGASVKV SCRASG-YTFT | D-----YHIH | WVRQAPGQ GLEWMG | WINPN---NGGTHYAQK FQG | RVTMTRDTSISTAYMELSRL RSDDTAVYYCAR | DGTG-----------------------SFDY | WGQGN LVTVS S |
| iPS:3 93132 | 21-225_33H7 | VH1\|1-02/D1\|1-1\|RF1/JH 4 | | QVQLVQS-GADVKKPGASVKV SCKASG-YTFT | D-----YHIH | WVRQAPGQ GLEWMG | WINPN---NGGTHYAQE FQG | RVTMTRDTSISTAYMELSSL RSDDTAVYHCAR | DGTG-----------------------SFDY | WGQGT LVTVS S |
| iPS:3 93140 | 21-225_35H12 | VH1\|1-02/D1\|1-1\|RF1/JH 4 | | QVQLVQS-GAEVKKPGASVKV SCKASG-YTFT | D-----YYIH | WVRQAPGQ GLEWMG | WINPN---RGGTNYAQK FQG | RVTMTRDTSISTAYMELSRL RSDDTAVYYCAR | DGTG-----------------------SFDY | WGQGT LVTVS S |
| iPS:3 93954 | 21-225_4H6 | VH1\|1-02/D1\|1-1\|RF1/JH 4 | | QVQLVQS-GAEVKKPGASVKV SCKASG-YTFT | D-----YYLH | WVRQAPGQ GLEWMG | WIHPN---SGGTNYAQK FQG | RVTMTRDTSISTAYMGLSSL RSDDTAVYYCAR | DGTS-----------------------SFDY | WGQGT LVTVS S |
| iPS:3 98484 | 21-225_18D4 | VH1\|1-02/D1\|1-1\|RF1/JH 4 | | QVQLVQS-GAEVKKPGASVKV SCKASG-YTFT | G-----YYLH | WVRQAPGQ GLEWLG | WINPN---SNGTISAQK FQG | RVTMTRDTSISTAYMELSRL ISDDTAVYYCAR | DGTS-----------------------SLDY | WGQGT LVTVS S |
| iPS:3 98502 | 21-225_23B11 | VH1\|1-02/D1\|1-1\|RF1/JH 4 | | QVQLVQS-GAEVKKPGASVKV SCKASG-YTFT | G-----YYLH | WVRQAPGQ GLEWMG | WINPN---NNGTNYAQK FQG | RVTMTRDTSISTAYMELSRL RSDDTAVYYCAR | DGTS-----------------------SFDY | WGQGT LVTVS S |
| iPS:3 98520 | 21-225_31C4 | VH1\|1-02/D1\|1-1\|RF1/JH 4 | | QVQLVQS-GTEVKKPGASVKV SCKASG-YTFT | G-----DYMH | WVRQAPGQ GLEWMG | WISPK---NGGTNYAQK FQG | RVTMTRDTSISTVYMELNRL RSDDTAVYYCAR | DGTG-----------------------SFDY | WGQGT LVTVS S |
| iPS:4 02223 | 21-225_30A11 | VH1\|1-02/D1\|1-1\|RF1/JH 4 | | QVQLVQS-GAEVKKPGASVKV SCKASG-YTFT | D-----YHMH | WVRQAPGQ GLEWMG | WINPN---RGGTNYAQK FQD | RVTMTRDTSISTAYMELSRL RSDDTAVYFCAR | DGTG-----------------------SFDY | WGQGT LVTVS S |
| | | **Germline** | | **H_FR1** | **H_CDR1** | **H_FR2** | **H_CDR2** | **H_FR3** | **H_CDR3** | **H_FR4** |
| | VH3\|3-33/D6\|6-6\|RF1/JH6 | | 32212 | QVQLVES-GGGVVQPGRSLRL SCAASG-FTFS | S-----YGMH | WVRQAPGK GLEWVA | VIWYD---GSNKYYADS VKG | RFTISRDNSKNTLYLQMNSL RAEDTAVYYCAR | EYSSSSYY-----------------YYYGMDV | WGQGT TVTVS S |
| iPS:4 26114 | 21-225_28H2 | VH3\|3-33/D6\|6- | | QVQLVES-GGGVVQPGRSLRL | N-----YVMH | WVRQAPGK GLEWVA | VIWYD---GSNKYYADS | RFTISRDNSKNTLYLQMNSL RAVDTAVYYCAR | EEYSSGW----------------YDYGMDV | WGQGT TVTVS |

| | | | | | | | | | | |
|---|---|---|---|---|---|---|---|---|---|---|
| **iPS:426116** | **21-225_29E2** | **VH3\|3-33/D6\|6-6\|RF1/JH6** | | QVQLVES-GGGVVQPGRSLRLSCAASG-FTFS | N-----CVMH | WVRQAPGKGLEWVA | VIWYD---GSNKYYADSVKG | RITISRDNSKNTLYLQMNSLRAEDTAVYYCAR | EEYSSGW----------------YDYGMDV | WGQGTTVTVSS |
| **iPS:468812** | **21-225_48H4** | **VH3\|3-33/D6\|6-6\|RF1/JH6** | | QVQLVES-GGGVVQPGRSLRLSCAASG-FTFS | D-----SLMH | WVRQAPGKGLEWVA | VIWYD---GSNKYYTDSVKG | RFTISRDNSKNTLYLQMNSLRAEDTAVYYCAR | ENYSSGW----------------YGYGMDV | WGQGTTVTVSS |
| **iPS:468816** | **21-225_52G8** | **VH3\|3-33/D6\|6-6\|RF1/JH6** | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTALYYCAR | RYSSSW------------------SGGMDV | WGQGTTVTVSS |
| **iPS:468826** | **21-225_201C5** | **VH3\|3-33/D6\|6-6\|RF1/JH6** | | QVQLVES-GGGVVQPGRSLRLSCAASG-FTFS | D-----YVMH | WVRQAPGKGLEWVA | VIWYD---GSNKYYVDSVKG | RFTISRDNSRNTLYLQMNSLRAEDTAVYYCAR | ERYSSGL----------------YDYGMDV | WGQGTTVTVSS |
| **iPS:468842** | **21-225_50H4** | **VH3\|3-33/D6\|6-6\|RF1/JH6** | | QVQLVES-GGGVVQPGRSLRLSCAASG-FTFS | S-----YVMH | WVRQAPGKGLEWVA | AIWYD---GSNKYYADSVKG | RFTISRDNSKNTLDLQMNSLRAEDTAVYYCAR | ELYSSNW----------------YDYGMDV | WGQGTTVTVSS |
| **iPS:468858** | **21-225_148C9** | **VH3\|3-33/D6\|6-6\|RF1/JH6** | | QVQLVES-GGGVAQPGRSLRLSCAASG-FTFS | D-----YGMH | WVRQAPGKGLEWVA | VIWYD---GSNKYYADSVKG | RLTISRDNSKNTLYLQMNSLRAEDTAVYYCAR | ERYSSGW----------------YDYGLDV | WGQGTTVTVSS |
| **iPS:468860** | **21-225_224E7** | **VH3\|3-33/D6\|6-6\|RF1/JH6** | | QVQLVES-GGGVVQPGRSLSLSCAASG-FTFS | S-----YVMH | WVRQAPGKGLEWVA | VIWYD---GSNKYYADSVKG | RFTISRDNSKNTLYLQMNSLRAEDTAVYYCAR | EQYSSSW----------------YDFGLDV | WGQGTTVTVSS |
| **iPS:433917** | **21-225_43E11** | **VH3\|3-33/D6\|6-6\|RF1/JH6** | | QVQLVES-GGGVVQPGRSLRLSCAASG-FSFS | D-----YGMH | WVRQAPGKGLEWVA | VIWYD---GSNKYYADSVKG | RLTISRDNSKNTLYLQMNSLRAEDTAVYYCAR | RYVRSW------------------VGGMDV | WGQGTTVTVSS |
| **iPS:433919** | **21-225_44B3** | **VH3\|3-33/D6\|6-6\|RF1/JH6** | | QVQLVES-GGGVVQPGRSLRLSCTASG-FTFS | D-----YGMH | WVRQAPGKGLEWVA | VIWYD---GSNKYYADSVKG | RFTISRDNSKNTLYLQMNSLRAEDTAVYYCAR | ERYSSGW----------------YDYGMDV | WGQGTTVTVSS |
| **iPS:433923** | **21-225_44D3** | **VH3\|3-33/D6\|6-6\|RF1/JH6** | | QVQLVES-GGGVVQPGRSLRLSCAASG-FTFS | S-----YVMH | WVRQAPGKGLEWVA | VIWYD---GSNKYYADSVKG | RFTISRDNSKNTLYLQMNSLRAEDTAVYYCAR | ERYSSGL----------------YDYGMDV | WGQGTTVTVSS |
| **iPS:433929** | **21-225_44D5** | **VH3\|3-33/D6\|6-6\|RF1/JH6** | | QVQLVES-GGGVVQPGRSLRLSCAASG-FTFS | S-----YVMH | WVRQAPGKGLEWVA | VIWYD---GSNKYYADSVKG | RFTISRDNSKNTLYLQMNSLRAEDTAVYYCAR | VPYSSSW----------------YDYGMDV | WGQGTTVTVSS |
| **iPS:433935** | **21-225_44F9** | **VH3\|3-33/D6\|6-** | | QVQLVES-GGGVVQPGRSLRL | S-----YVMH | WVRQAPGKGLEWVA | VIWYD---GSNKYYADS | RFTISRDNSKNTLYLQMNSLRAEDTAVYYCAR | EPYSSSW----------------YDYGMDV | GGQGTTVTVS |

| | | | | | | | | | | |
|---|---|---|---|---|---|---|---|---|---|---|
| iPS:433937 | 21-225_44B10 | VH3\|3-33/D6\|6-6\|RF1/JH6 | | QVQLVES-GGGVVQPGRSLRLSCVASG-FTFS | S-----YGMH | WVRQAPGKGLEWVA | VIWYD---GSNKYYADSVKG | RFTISRDNSKNTLYLQMNSLRAEDTAVYYCAR | RYSSSW------------------VGGMDV | WGQGTTVTVSS |
| iPS:433939 | 21-225_44C10 | VH3\|3-33/D6\|6-6\|RF1/JH6 | | QVQLVES-GGGVVQPGRSLRLSCAASG-FTFS | D-----CVMH | WVRQAPGKGLEWVA | VIWYD---GSNKYYADSVKG | RFTISRDNSKNTLYLQMNSLRAEDTAVYYCAR | ERYSSGL----------------YDYGMDV | WGQGTTVTVSS |
| iPS:433951 | 21-225_45B4 | VH3\|3-33/D6\|6-6\|RF1/JH6 | | QVQLVES-GGGVVQPGRSLRLSCAASG-FTFS | D-----CVMH | WVRQAPGKGLEWVA | VIWYD---GSNKYYADSVKG | RFTISRDNSKNTLYLQMNSLRAEDTAVYYCAR | ERYSSGL----------------YDYGMDV | WGQGTTVTVSS |
| iPS:433955 | 21-225_45B8 | VH3\|3-33/D6\|6-6\|RF1/JH6 | | QVQLVES-GGGVVQPGRSLRLSCAASG-FTFS | D-----CVMH | WVRQAPGKGLEWVA | VIWYD---GSNKYYADSVKG | RFTISRDNSKNTLYLQMNSLRAEDTAVYYCAR | ERYSSGL----------------YDYGMDV | WGQGTTVTVSS |
| iPS:433967 | 21-225_46C3 | VH3\|3-33/D6\|6-6\|RF1/JH6 | | QVQLVES-GGGVVQPGRSLRLSCAASG-FTFS | S-----YVMH | WVRQAPGKGLEWVA | VIWYD---GSNKYYADSVKG | RFTISRDNSKNTLYLQMNSLRAEDTAVYYCAR | ERYSSGL----------------YDYGMDV | WGQGTTVTVSS |
| iPS:433971 | 21-225_46D4 | VH3\|3-33/D6\|6-6\|RF1/JH6 | | QVQLVES-GGGVVQPGRSLRLSCAASG-FTFS | S-----YVMH | WVRQAPGKGLEWVA | VIWYD---GSNKYYADSVKG | RFTISRDNSKNTLYLQMNSLRAEDTAVYYCAR | VPYSSSW----------------YDYGMDV | WGQGTTVTVSS |
| iPS:433979 | 21-225_46B9 | VH3\|3-33/D6\|6-6\|RF1/JH6 | | QVQLVES-GGGVVQPGRSLRLSCSASG-FTFS | S-----YGMH | WVRQAPGKGLEWVA | VIWYD---GRNKYYADSVKG | RITISRDNSKNTLYLQMNSLRAEDTAVYYCAR | RYSSSW------------------MGGMDV | WGQGTTVTVSS |
| iPS:433985 | 21-225_47C1 | VH3\|3-33/D6\|6-6\|RF1/JH6 | | QVQLVES-GGGVVQPGRSLRLSCAASG-FTFS | D-----YGMH | WVRQTPGKGLEWVA | VIWYD---GSNKYYADSVKG | RFTISRDNSKNTLFLQMNSLRDEDTAVYYCAR | RYSRSW------------------VGGMDV | WGQGTTVTVSS |
| iPS:433991 | 21-225_47E7 | VH3\|3-33/D6\|6-6\|RF1/JH6 | | QVQLVES-GGGVVQPGRSLRLSCAASG-FTFS | I-----YGMH | WVRQAPGKGLEWVA | VIWYD---GSNKYYADSVKG | RFTISRDNSKNTLYLQMNSLRAEDTAVYYCAR | RYSRSW------------------VGGMDV | WGQGTTVTVSS |
| iPS:434001 | 21-225_48F2 | VH3\|3-33/D6\|6-6\|RF1/JH6 | | QVQLVES-GGGVVQPGRSLRLSCAASG-FTFS | N-----YVMH | WVRQAPGKGLEWVA | VIWYD---GSNKYYADSVKG | RFTISRDNSKNTLYLQMNSLRAEDTAVYYCAR | ERYSSSW----------------YDYGLDV | WGQGTTVTVSS |
| iPS:434021 | 21-225_49C1 | VH3\|3-33/D6\|6-6\|RF1/JH6 | | QVQLVES-GGGVVQPGRSLRLSCTASG-FTFS | S-----YGMH | WVRQAPGKGLEWVA | VIWYD---GSNKYYADSVKG | RFTISRDNSKNTLYLQMNSLRAEDTAVYYCAR | RYSSSW------------------SGGMDV | WGQGTTVTVSS |
| iPS:434025 | 21-225_49G3 | VH3\|3-33/D6\|6- | | QVQLVES-GGGVVQPGRSLRL | S-----YGMH | WVRQAPGKGLEWVA | VIWYD---GSNKYYADS | RFTISRDNSKNTLYLQMNSLRAEDTAVYYCAR | RYSSSW------------------SGGMDV | WGQGTTVTVS |

| | | | | | | | | | | |
|---|---|---|---|---|---|---|---|---|---|---|
| iPS:434031 | 21-225_49E7 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTALYYCAR | RYSSSW------------------SGGMDV | WGQGTTVTVSS |
| iPS:434033 | 21-225_49F9 | VH3\|3-33/D6\|6-6\|RF1/JH6 | | QVQLVES-GGGVVQPGRSLRLSCAASG-FTFS | S-----YGMH | WVRQAPGKGLEWVA | LIWYD---GRNKYYADSVKG | RFTISRDNPKNTLYLQMNSLRAEDTAVYHCAR | RYSSSW------------------SGGMDV | WGQGTTVTVSS |
| iPS:434053 | 21-225_51E1 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSSKYYADSVKG | RFTISRDNSKNTLYLQMNSLRAEDTAVYYCAR | RYSSSW------------------SGGMDV | WGQGTTVTVSS |
| iPS:434093 | 21-225_52D10 | VH3\|3-33/D6\|6-6\|RF1/JH6 | | QVQLVES-GGGVVQPGRSLRLSCAASG-FTFS | S-----YGMH | WVRQAPGKGLEWVA | LIWYD---GSNKYHADSVKG | RFTISRDNSKNTLYLQMNSLRAEDTAVYYCAR | RYSSSW------------------SGGMDV | WGQGTTVTVSS |
| iPS:434137 | 21-225_54D4 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSSSW------------------SGGMDV | WGQGTTVTVSS |
| iPS:434149 | 21-225_55H1 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSSSW------------------SGGMDV | WGQGTTVTVSS |
| iPS:434151 | 21-225_55C2 | VH3\|3-33/D6\|6-6\|RF1/JH6 | | QVQLVES-GGGVVQPGRSLRLSCAASG-FTFS | S-----YGMH | WVRQAPGKGLEWVA | LIWYD---GSNKYHADSVKG | RFTISRDNSKNTLYLQMNSLRAEDTAVYYCAR | RYSSSW------------------SGGMDV | WGQGTTVTVSS |
| iPS:434161 | 21-225_55F9 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GNGKYYADSVKG | RFTISRDNSKNTLYLQMNSLRAEDTAVYYCAR | RYSSSW------------------SGGMDV | WGQGTTVTVSS |
| iPS:434201 | 21-225_59A12 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSSSW------------------DGGMDV | WGQGTTVTVSS |
| iPS:434205 | 21-225_60G2 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSRSW------------------TGGMDV | WGQGTTVTVSS |
| iPS:434223 | 21-225_60C12 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYVDSVKG | RFTISRDNSKNTLYLQMNSLRAEDTAVYYCAR | RYSRSW------------------TGGMDV | WGQGTTVTVSS |
| iPS:434231 | 21-225_61F2 | VH3\|3-33/D6\|6- | | QVQLVES-GGGVVQPGRSLRL | S-----YGMH | WVRQAPGKGLEWVA | VIWYD---GSNKYYADS | RFTISRDNSKNTLYLQMNSLRAEDTAVYYCAR | ERYSSGW---------------YDYGMDV | WGQGTTVTVS |

| | | | | | | | | | | |
|---|---|---|---|---|---|---|---|---|---|---|
| iPS:434233 | 21-225_61B3 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSRSW-----------------AGGMDV | WGQGTTVTVSS |
| iPS:434303 | 21-225_58H11 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYHADSVKG | RFTISRDNSKNTLYLQMNSLRAEDTAVYYCAR | RYSSSW-----------------DGGMDV | WGQGTTVTVSS |
| iPS:434339 | 21-225_64A4 | VH3\|3-33/D6\|6-6\|RF1/JH6 | | QVQLVES-GGGVVQPGRSLRLSCAASG-FTFS | D-----YVMH | WVRQAPGKGLEWVA | VIWYD---GSNKYYADSVKG | RFTISRDNSQNTLYLQMNSLRAEDTAVYYCAR | ERYSSSW----------------YDYGMDV | WGQGTTVTVSS |
| iPS:434343 | 21-225_64C8 | VH3\|3-33/D6\|6-6\|RF1/JH6 | | QVQLVES-GGGVVQPGRSLRLSCAASG-FTFS | D-----YVMH | WVRQAPGKGLEWVA | VIWYD---GSNKYYADSMKG | RFTISRDNSKNTLYLQMNSLRAEDTAVYYCAR | ERYSSGW----------------YDYGMDV | WGQGTTVTVSS |
| iPS:434387 | 21-225_66D11 | VH3\|3-33/D6\|6-6\|RF1/JH6 | | QVQLVES-GGGVVQPGRSLRLSCGASG-FTFS | S-----YGMH | WVRQAPGKGLEWVA | VIWYD---GSNKYYADSVKG | RFTISRDNSKNTLSLQMNSLRAEDTALYYCAR | EMYSSNW----------------YDYGLDV | WGQGTTVTVSS |
| iPS:434469 | 21-225_73C9 | VH3\|3-33/D6\|6-6\|RF1/JH6 | | QVQLVES-GGGVVQPGRSLRLSCAASG-FTFS | N-----YVMH | WVRQAPGKGLEWVA | VIWYD---GSNKYYADSVKG | RFTISRDNSKNTLYLQMNSLRAEDTAVYYCAR | ERYSSSW----------------FDYGMDV | WGQGTTVTVSS |
| iPS:435197 | 21-225_94F3 | VH3\|3-33/D6\|6-6\|RF1/JH6 | | QVQLVES-GGGVVQPGRSLRLSCAASG-FTFS | N-----DIMH | WVRQAPGKGLEWVA | VIWYD---GSNKYYADSVKG | RFTISRDNSKNTLYLQINSLRAEDTAVYFCAR | EKYSSGW----------------YDYGMDV | WGQGTTVTVSS |
| iPS:435315 | 21-225_147B2 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSSSW-----------------SGGMDV | WGQGTTVTVSS |
| iPS:435325 | 21-225_147H5 | VH3\|3-33/D6\|6-6\|RF1/JH6 | | QVQLVES-GGGVAQPGRSLRLSCAASG-FTFS | D-----YGMH | WVRQAPGKGLEWVA | VIWYD---GSNKYYADSVKG | RLTISRDNSKNTLYLQMNSLSAEDTAVYYCAR | ERYSSGW----------------YDYGLDV | WGQGTTVTVSS |
| iPS:435329 | 21-225_147A8 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSSSW-----------------TGGMDV | WGQGTTVTVSS |
| iPS:435349 | 21-225_148F5 | VH3\|3-33/D6\|6-6\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSSSW-----------------SGGMDV | WGQGTTVTVSS |
| iPS:435359 | 21-225_148H10 | VH3\|3-33/D6\|6- | | QVQLVES-GGGVVQPGRSLRL | S-----YGMH | WVRQAPGKGLEWVA | VIWYD---GSNKYYGDS | RFTISRDNSKNTLYLQMNSLRAEDTAVYYCAR | RYSSSW-----------------SGGMDV | WGQGTTVTVS |

| | | | | | | | | | | |
|---|---|---|---|---|---|---|---|---|---|---|
| iPS:4 35393 | 21-225_149D10 | VH3\|3-33/D6\|6-6\|RF1/JH6 | | QVQLVES-GGGVAQPGRSLRLSCAASG-FTFS | D-----YGMH | WVRQAPGKGLEWVA | VIWYD---GSNKYYADSVKG | RLTISRDNSKNTLYLQMNSLRAEDTAVYYCAR | ERYSSGW----------------YDYGMDV | WGQGTTVTVSS |
| iPS:4 35401 | 21-225_150E2 | VH3\|3-33/D6\|6-6\|RF1/JH6 | | QVQLVES-GGGVVQPGRSLRLSCAASG-FTFS | S-----YVMH | WVRQAPGKGLEWVA | VIWYD---GSNKYYADSVKG | RFTISRDNSKNTLYLQMNSLRAEDTAVYYCAR | EEYSSSW----------------YGYGMDV | WGQGTTVTVSS |
| iPS:4 35417 | 21-225_150D11 | VH3\|3-33/D6\|6-6\|RF1/JH6 | | QVQLVES-GGGVVQPGRSLRLSCAASG-FTFS | S-----YGMH | WVRQAPGKGLEWVA | VIFYD---GSNKHYADSVKG | RFTISRDNSKNTLYLQMNSLRAEDSAVYYCTR | RFSSSW------------------SGGMDV | WGQGTTVTVSS |
| iPS:4 35445 | 21-225_152F7 | VH3\|3-33/D6\|6-6\|RF1/JH6 | | QVQLVES-GGGVVQPGRSLRLSCAASG-FTFS | S-----YIMH | WVRQAPGKGLEWVA | VIWYD---GSNKYYADSVKG | RFTISRDNSKNTLYLQMNSLRAEDTAVYYCAR | EEYSSSW----------------YGYGMDV | WGQGTTVTVSS |
| iPS:4 35469 | 21-225_153G9 | VH3\|3-33/D6\|6-6\|RF1/JH6 | | QVQLVES-GGGVVQPGRSLRLSCAASG-FTFS | S-----YGMH | WVRQAPGKGLEWVA | LIFYD---GSNKYYADSVKG | RFTISRDNSKNTLYLQINSLRAEDTAVYFCAR | RYSRSW------------------AGGMDV | WGQGTAVTVSS |
| iPS:4 35573 | 21-225_159D8 | VH3\|3-33/D6\|6-6\|RF1/JH6 | | QVQLVES-GGGVVQPGRSLRLSCAASG-FTFS | N-----YVMH | CVRQAPGKGLEWVA | VIWYD---GSNKYYADSVKG | RFTISRDNSKNTLYLQMNSLRAEDTAVYYCAR | EPYSSGW----------------YDYGMDV | WGQGTTVTVSS |
| iPS:4 35681 | 21-225_169D11 | VH3\|3-33/D6\|6-6\|RF1/JH6 | | QVQLVES-GGGVVQPGRSLRLSCAASG-FTFS | D-----YVMH | WVRQAPGKGLEWVA | VIWYD---GSNKYYADSVKG | RFTISRDNSKNTLYLQLNSLRAEDTAVYYCAR | ERYSSGW----------------YDYGMDV | WGQGTTVTVSS |
| iPS:4 35689 | 21-225_170F3 | VH3\|3-33/D6\|6-6\|RF1/JH6 | | QVQLVES-GGGVVQPGRSLRLSCAASG-FSFS | D-----YVMH | WVRQAPGKGLEWVA | VIWYD---GSNKYYEDSVKG | RFTISRDNSKNTLYLQMNSLRAEDTAVYYCAR | ETYSSSW----------------YDYGMDV | WGQGTTVTVSS |
| iPS:4 35733 | 21-225_173C11 | VH3\|3-33/D6\|6-6\|RF1/JH6 | | QVQLVES-GGGVVQPGRSLRLSCAASG-FTFS | S-----YGIH | WVRQAPGKGLEWVA | LIFYD---GSNKYYADSVKG | RFTISRDNSKNTLYLHMSSLRAEDTAVYYCAR | RYSSSW------------------SGGMDV | WGQGTTVTVSS |
| iPS:4 35741 | 21-225_174G10 | VH3\|3-33/D6\|6-6\|RF1/JH6 | | QVQLVES-GGGVVQPGRSLRLSCAASG-FTFS | D-----YVMH | WVRQAPGKGLEWVA | VIWYD---GSNKYYADSVKG | RFTISRDNSKNTLYLQLNSLRAEDTAVYYCAR | ERYSSGW----------------YDYGMDV | WGQGTTVTVSS |
| iPS:4 35763 | 21-225_176H12 | VH3\|3-33/D6\|6-6\|RF1/JH6 | | QVQLVES-GGGVVQPGRSLRLSCAASG-FTFS | D-----YVMH | WVRQAPGKGLEWVA | VIWYD---GSNKYYADSVKG | RFTISRDNSKNTLYLQMNSLRAEDTAVYYCAR | EKYSSNW----------------YDYGMDV | WGQGTTVTVSS |
| iPS:4 35767 | 21-225_177B4 | VH3\|3-33/D6\|6- | | QVQLVES-GGGVVQPGRSLRL | D-----YVMH | WVRQAPGKGLEWVA | VIWYD---GSNKYYADS | RFIVSRDNSKNTLYLQMNSLRAEDTAVYYCAR | EKYSSSW----------------YDYGLDV | WGQGTTVTVS |

| | | | | | | | | | | |
|---|---|---|---|---|---|---|---|---|---|---|
| **iPS:4 35785** | **21-225_179C2** | **VH3\|3-33/D6\|6-6\|RF1/JH 6** | | QVQLVES-<br>GGGVVQPGRSLRL<br>SCAASG-FTFS | S-----YGMH | WVRQAPGK<br>GLEWVA | LIFYD---<br>GSNKYYADS<br>VKG | RFTISRDNSKNTLFLQMNSL<br>RAEDTAVYYCAR | RYSGSW----------<br>---------SGGMDV | WGQGT<br>TVTVS<br>S |
| **iPS:4 35921** | **21-225_190D6** | **VH3\|3-33/D6\|6-6\|RF1/JH 6** | | QVQLVES-<br>GGGVVQPGRSLRL<br>SCAASG-FTFS | S-----FIMH | WVRQAPGR<br>GLEWVA | VIWYD---<br>GSNKYYADS<br>VKG | RFTISRDNSKNTLYLQMNSL<br>RAEDTAVYYCAR | EEYSSGW---------<br>-------FGYGMDV | WGQGT<br>TVTVS<br>S |
| **iPS:4 35961** | **21-225_192A2** | **VH3\|3-33/D6\|6-6\|RF1/JH 6** | | QVQLVES-<br>GGGVVQPGRSLRL<br>SCAASG-FTFS | S-----YGMH | WVRQAPGK<br>GLEWLA | VIWYD---<br>GSYKYYADS<br>VKG | RFTISRDNSKNTLYLQMNSL<br>RAEDTAVYYCAR | EDSPYSGYA-------<br>-----LDYFYGMDV | WGQGT<br>TVTVS<br>S |
| **iPS:4 35985** | **21-225_192F6** | **VH3\|3-33/D6\|6-6\|RF1/JH 6** | | QVQLVES-<br>GGGVVQPGRSLRL<br>SCAASG-FTFS | S-----FIMH | WVRQAPGR<br>GLEWVA | VIWYD---<br>GSNKYYVDS<br>VKG | RFTISRDNSKNTLYLQMNSL<br>RAEDTAVYYCAR | EEYSSGW---------<br>-------FGYGMDV | WGQGT<br>TVTVS<br>S |
| **iPS:4 36039** | **21-225_193F8** | **VH3\|3-33/D6\|6-6\|RF1/JH 6** | | QVQLVES-<br>GGGVVQPGRSLRL<br>SCAASG-FTFS | I-----YGMD | WVRQAPGK<br>GLEWVA | VIWYD---<br>GSYKYYADS<br>VKG | RFTISRDNSKNTLYLQMNSL<br>RAEDTAVYYCVR | EDSPYSGYG-------<br>-----LDYYYGMDV | WGQGT<br>TVTVS<br>S |
| **iPS:4 36074** | **21-225_194F10** | **VH3\|3-33/D6\|6-6\|RF1/JH 6** | | QVQLVES-<br>GGGVVQPGRSLRL<br>SCAASG-FTFS | S-----FIMH | WVRQAPGR<br>GLEWVA | VIWYD---<br>GSNKYYADS<br>VKG | RFTISRDNSKNTLYLEMNSL<br>RVEDTAVYYCAR | EEYSSGW---------<br>-------FGYGMDV | WGQGT<br>TVTVS<br>S |
| **iPS:4 36264** | **21-225_203F7** | **VH3\|3-33/D6\|6-6\|RF1/JH 6** | | QVQLVES-<br>GGGVVQPGRSLRL<br>SCAASG-FTFS | D-----YVMH | WVRQAPGK<br>GLEWVA | VIWYD---<br>GSNKYYVDS<br>VKG | RFTISRDNSRNTLYLQMNSL<br>RAEDTAVYYCAR | ERYSSGL---------<br>-------YDYGMDV | WGQGT<br>TVTVS<br>S |
| **iPS:4 36274** | **21-225_204H3** | **VH3\|3-33/D6\|6-6\|RF1/JH 6** | | QVQLVES-<br>GGGVGQPGRSLRL<br>SCTASG-FTFS | S-----YVMH | WVRQAPGK<br>GLEWVA | VIWYD---<br>GSNKYNADS<br>VKG | RFTISRDNSKNTLYLQMNSL<br>RAEDTAVYYCAR | EPYSSSW---------<br>-------YDYGMDV | SGQGT<br>TVTVS<br>S |
| **iPS:4 36332** | **21-225_208B2** | **VH3\|3-33/D6\|6-6\|RF1/JH 6** | | QVQLVES-<br>GGGVVQPGRSLRL<br>SCAASG-FTFS | D-----CVMH | WVRQAPGK<br>GLEWVA | VIWYD---<br>GSNKYYVDS<br>VKG | RFTISRDNSRNTLYLQMNSL<br>RAEDTAVYYCAR | ERYSSGL---------<br>-------YDYGLDV | WGQGT<br>TVTVS<br>S |
| **iPS:4 36352** | **21-225_210G5** | **VH3\|3-33/D6\|6-6\|RF1/JH 6** | | QVQLVES-<br>GGGVVQPGRSLRL<br>SCAASG-FTFS | D-----CVMH | WVRQAPGK<br>GLEWVA | VIWYD---<br>GSNKYYVDS<br>VKG | RFTISRDNSRNTLYLQMNSL<br>RAEDTAVYYCAR | ERYSSGL---------<br>-------YDYGLDV | WGQGT<br>TVTVS<br>S |
| **iPS:4 36386** | **21-225_212B11** | **VH3\|3-33/D6\|6-6\|RF1/JH 6** | | QVQLVES-<br>GGGVVQPGRSLRL<br>SCAASG-FTFS | D-----YVMH | WVRQAPGK<br>GLEWVT | VIWYD---<br>GSNKYYADS<br>VKG | RFTISRDNSKNTLYLQMNSL<br>RAEDTAVYYCAR | ERYSSGW---------<br>-------YDYGMDV | WGQGT<br>TVTVS<br>S |
| **iPS:4 36412** | **21-225_214H9** | **VH3\|3-33/D6\|6-** | | QVQLVES-<br>GGGVVQPGRSLRL | S-----YVMH | WVRQAPGK<br>GLEWVA | VIWYD---<br>GSNKYYADS | RFTISRDNSKNTLYLQMNSL<br>RAEDTAVFYCAR | ERYTSSW---------<br>-------YDYGMDV | WGQGT<br>TVTVS |

| | | | | | | | | | | |
|---|---|---|---|---|---|---|---|---|---|---|
| **iPS:4 36414** | **21-225_214G10** | **VH3\|3-33/D6\|6-6\|RF1/JH6** | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW---------------YDYGMDV | WGQGTTVTVSS |
| **iPS:4 36416** | **21-225_214G12** | **VH3\|3-33/D6\|6-6\|RF1/JH6** | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW---------------YDYGMDV | WGQGTTVTVSS |
| **iPS:4 36418** | **21-225_215E3** | **VH3\|3-33/D6\|6-6\|RF1/JH6** | | QVQLVES-GGGVVQPGRSLRLSCAASG-FTFS | D-----YVIH | WVRQAPGKGLEWVT | VIWYD---GSNKYYADSVKG | RFTISRDNSKNTLYLQMNSVRAEDTAVYYCAR | ERYSSGW---------------YDYGMDV | WGQGTTVTVSS |
| **iPS:4 36428** | **21-225_215E11** | **VH3\|3-33/D6\|6-6\|RF1/JH6** | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW---------------YDYGMDV | WGQGTTVTVSS |
| **iPS:4 36438** | **21-225_216E8** | **VH3\|3-33/D6\|6-6\|RF1/JH6** | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW---------------YDYGMDV | WGQGTTVTVSS |
| **iPS:4 36440** | **21-225_216H12** | **VH3\|3-33/D6\|6-6\|RF1/JH6** | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW---------------YDYGMDV | WGQGTTVTVSS |
| **iPS:4 36450** | **21-225_217E5** | **VH3\|3-33/D6\|6-6\|RF1/JH6** | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW---------------YDYGMDV | WGQGTTVTVSS |
| **iPS:4 36456** | **21-225_217G10** | **VH3\|3-33/D6\|6-6\|RF1/JH6** | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW---------------YDYGMDV | WGQGTTVTVSS |
| **iPS:4 36458** | **21-225_217H12** | **VH3\|3-33/D6\|6-6\|RF1/JH6** | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW---------------YDYGMDV | WGQGTTVTVSS |
| **iPS:4 36462** | **21-225_218C4** | **VH3\|3-33/D6\|6-6\|RF1/JH6** | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW---------------YDYGMDV | WGQGTTVTVSS |
| **iPS:4 36480** | **21-225_220F8** | **VH3\|3-33/D6\|6-6\|RF1/JH6** | | QVQLVES-GGGVVQPGRSLRLSCAASG-FTFS | D-----YVMH | WVRQAPGKGLEWVT | VIWYD---GSNKYYADSVKG | RFTISRDNSKNTLYLQMNSLRAEDTAVYYCAR | ERYSSGW---------------YDYGMDV | WGQGTTVTVSS |
| **iPS:4 36534** | **21-225_224F1** | **VH3\|3-33/D6\|6-** | | QVQLVES-GGGVVQPGRSLRL | S-----YIMH | WVRQAPGKGLEWVA | VIWYD---GSNKYYADS | RFTISRDNSKNTLYLQMNSLRAEDTAVYYCAR | ERYSSNW---------------YDYGMDV | WGQGTTVTVS |

| | | | | | | | | | | |
|---|---|---|---|---|---|---|---|---|---|---|
| **iPS:4 36540** | **21-225_224F3** | **VH3\|3-33/D6\|6-6\|RF1/JH 6** | | QVQLVES-GGGVVQPGRSLRLSCAASG-FSFS | S-----YGMH | WVRQAPGKGLEWVA | VIWYD---GSNKYYADSVKG | RFTISRDNSKNTLYLQMNSLRAEDTAVYYCAR | ERYSSSW---------------YDYGMDV | WGQGTTVTVSS |
| **iPS:4 36564** | **21-225_225A1** | **VH3\|3-33/D6\|6-6\|RF1/JH 6** | | QVQLVES-GGGVVQPGRSLRLSCAASG-FTFS | D-----YVIH | WVRQAPGKGLEWVA | VIWYD---GSNKYYADSVKG | RFTISRDNSKNTLYLQMNSLRAEDTAVYYCAR | ERYSSGW---------------YDYGMDV | WGQGTTVTVSS |
| **iPS:4 36596** | **21-225_226C6** | **VH3\|3-33/D6\|6-6\|RF1/JH 6** | | QVQLVES-GGGVVQPGRSLRLSCAASG-FTFS | D-----YGMH | WVRQAPGKGLEWVA | VIWYD---GSNKYYADSVKG | RFTISRDNSKNTLYLQTNSLRAEDTAVYYCAR | ERYSSSW---------------YDYGMDV | WGQGTTVTVSS |
| **iPS:4 36620** | **21-225_226H11** | **VH3\|3-33/D6\|6-6\|RF1/JH 6** | | QVQLVES-GGGVVQPGRSLRLSCAASG-FTFS | N-----CGMH | WVRQAPGKGLEWVT | VIWYD---GSNKYYADSVKG | RFTISRDNSKNTLYLQMNSLRAEDTAVYYCAR | ELYSSSW---------------YDYGLDV | WGQGTTVTVSS |
| **iPS:4 36744** | **21-225_154F4** | **VH3\|3-33/D6\|6-6\|RF1/JH 6** | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | EDSYCSGTSC----------PYYYYYGMDV | WGQGTTVTVSS |
| **iPS:4 36946** | **21-225_183F4** | **VH3\|3-33/D6\|6-6\|RF1/JH 6** | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | ERTYCSGTTC----------PYYYYYGLGV | WGQGTTVTVSS |
| **iPS:4 37286** | **21-225_208F1** | **VH3\|3-33/D6\|6-6\|RF1/JH 6** | | QVQLVES-GGGVVQPGRSLRLSCAASR-FTFS | D-----YVMH | WVRQAPGKGLEWVA | VIWYD---GSNKYYVDSVKG | RFTISRDNSRNTLYLQMNSLRAEDTAVYYCAR | ERYSSGL---------------YDYGMDV | WGQGTTVTVSS |
| **iPS:4 37290** | **21-225_210G6** | **VH3\|3-33/D6\|6-6\|RF1/JH 6** | | QVQLVES-GGGVVQPGRSLRLSCAASG-FTFS | D-----YVMH | WVRQAPGKGLEWVA | VIWYD---GSNKYYVDSVKG | RFTISRDNSRNTLYLQMNSLRAEDTAVYYCAR | ERYSSGL---------------YDYGMDV | WGQGTTVTVSS |
| **iPS:3 92634** | **21-225_17H3** | **VH3\|3-33/D6\|6-6\|RF1/JH 6** | | QVQLVES-GGGVVQPGRSLRLSCAASG-FTFS | N-----CVMH | WVRQAPGKGLEWVA | VIWYD---GSNKYYADSVKG | RFTISRDNSKNTLYLQMNSLRAEDTAVYYCAR | EKYSSSW---------------YDYGMDV | WGQGTTVTVSS |
| **iPS:3 92742** | **21-225_20B2** | **VH3\|3-33/D6\|6-6\|RF1/JH 6** | | QVQLVES-GGGVVQPGRSLRLSCAASG-FTFS | N-----YVIH | WVRQAPGKGLEWVA | VIWYD---GSNKYYADSVKG | RFTISRDNSKNTLYLQMNSLRAEDTAVYSCAR | EKYSSSW---------------YDYGMDV | WGQGTTVTVSS |
| **iPS:3 92836** | **21-225_22F4** | **VH3\|3-33/D6\|6-6\|RF1/JH 6** | | QVQLVES-GGGVVQPGRSLRLSCAASG-FTFS | D-----YGMH | WVRQAPGKGLEWVA | VIWYD---GSNKYYVDSVKG | RFTISRDNSKNTLYLQMNSLRAEDTAVYYCAR | EKYSSSW---------------YDYGMDV | WGQGTTVTVSS |
| **iPS:3 92846** | **21-225_24B6** | **VH3\|3-33/D6\|6-** | | QVQLVES-GGGVVQPGRSPRL | N-----YVMH | WVRQAPGKGLEWVA | VIWYD---GSNKYYADS | RFTISRDNSKNTLYLQMNSLRAEDTAVYYCAR | EEYSSGW---------------YDYGMDV | WGQGTTVTVS |

| | | | | | | | | | | |
|---|---|---|---|---|---|---|---|---|---|---|
| **iPS:3 92884** | **21-225_23A10** | **VH3\|3-33/D6\|6-6\|RF1/JH6** | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | ERYSSGW----------------HDYGMDV | WGQGTTVTVSS |
| **iPS:3 92888** | **21-225_25A2** | **VH3\|3-33/D6\|6-6\|RF1/JH6** | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSSSW------------------SGGMDV | WGQGTTVTVSS |
| **iPS:3 92914** | **21-225_25D12** | **VH3\|3-33/D6\|6-6\|RF1/JH6** | | QVQLVES-GGGVVQPGRSLRLSCAASG-FTFS | S-----DGMH | WVRQAPGKGLEWVA | VIWYD---GSNKYYADSVKG | RFTISRDNSKNTLYLQMNSLRAEDTAVYYCAR | ERYSSSW----------------YDYGMDV | WGQGTTVTVSS |
| **iPS:3 92924** | **21-225_32H2** | **VH3\|3-33/D6\|6-6\|RF1/JH6** | | QVQLVES-GGGVVQPGRSLRLSCAASG-FTFS | S-----YGMH | WVRQAPGKGLEWVA | VIWYD---GSNKYYADSVKG | RFTISRDNSKNTLNLQMNSLRAEDTGVYYCAR | RYSSSW------------------TGGMDV | WGQGTTVTVSS |
| **iPS:3 92938** | **21-225_29H4** | **VH3\|3-33/D6\|6-6\|RF1/JH6** | | QVQLVES-GGGVVQPGRSLRLSCAASG-FTFS | S-----YGIH | WVRQAPGKGLEWVA | VIWYD---GSNKYYADSVKG | RFTISRDNSKNTLYLQMNSLRAEDTAVYYCAR | RYSSSW------------------SGGMDV | WGQGTTVTVSS |
| **iPS:3 92974** | **21-225_26A11** | **VH3\|3-33/D6\|6-6\|RF1/JH6** | | QVQLVES-GGGVVQPGRSLRLSCAASG-FTFS | N-----CVMH | WVRQAPGKGLEWVA | VIWYD---GSNKYYADSVKG | RFTISRDNSKNTLYLQMNSLRAEDTAVYYCAR | EEYSSGW----------------YDYGMDV | WGQGTTVTVSS |
| **iPS:3 93012** | **21-225_26G7** | **VH3\|3-33/D6\|6-6\|RF1/JH6** | | QVQLVES-GGGVVQPGRSLRLSCAASG-FTFS | S-----YGMH | WVRQAPGKGLEWVA | VIWYD---GSNKYYADSVKG | RFTISRDNSNNTLYLQMNSLRAEDTAVYYCAR | RYSSSW------------------SGGMDV | WGQGTTVTVSS |
| **iPS:3 93176** | **21-225_27E7** | **VH3\|3-33/D6\|6-6\|RF1/JH6** | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | EDSYCSSTSC----------PYYYYYGMDV | WGQGTTVTVSS |
| **iPS:3 93864** | **21-225_4C5** | **VH3\|3-33/D6\|6-6\|RF1/JH6** | | QVQLVES-GGGVVQPGRSLRLSCAASG-FTFS | S-----YVLH | WVRQAPGKGLEWVA | VIWYD---GSNKYYADSVKG | RFTISRDNSKNTLYLQMNSLRAEDTAVYYCAR | EKYTSSW----------------YDYGMDV | WGQGTTVTVSS |
| **iPS:3 93902** | **21-225_14E10** | **VH3\|3-33/D6\|6-6\|RF1/JH6** | | QVQLVES-GGGVVQPGRSLRLSCAASG-FTFS | D-----YGMH | WVRQAPGKGLEWVA | VIWYD---GSNKYYADSVKG | RFTISRDNSKNTLYLQMNSLRAEDTAVYYCAR | EKYSSSW----------------YDYGMDV | WGQGTTVTVSS |
| **iPS:3 93908** | **21-225_10E9** | **VH3\|3-33/D6\|6-6\|RF1/JH6** | | QVQLVES-GGGVVQPGRSLRLSCAASG-FTFS | N-----YVIH | WVRQAPGKGLEWVA | VIWYD---GSNKYYADSVKG | RFTISRDNSKNTLYLQMNSLRAEDTAVYYCAR | EKYSSSW----------------YDYGMDV | WGQGTTVTVSS |
| **iPS:3 93916** | **21-225_2G4** | **VH3\|3-33/D6\|6-** | | QVQLVES-GGGVVQPGRSLRL | S-----YVMH | WVRQAPGKGLEWVA | VIWYD---GSNKYYADS | RFTISRDNSKNTLYLQMNSLRAEDTAVYYCAR | EKYSSSW----------------YDYGMDV | WGHGTTVTVS |

| | | | | | | | | | | |
|---|---|---|---|---|---|---|---|---|---|---|
| iPS:393950 | 21-225_3H10 | VH3\|3-33/D6\|6-6\|RF1/JH6 | | QVQLVES-GGGVVQPGRSLRLSCAASG-FTFS | S-----YVMH | WVRQAPGKGLEWVA | VIWYD---GSNKYYADSVKG | RFTISRDNSKNTLYLQMNSLRAEDTAVYYCAR | ERYSSGW----------------YDYGLDV | WGQGTTVTVSS |
| iPS:393972 | 21-225_7C9 | VH3\|3-33/D6\|6-6\|RF1/JH6 | | QVQLVES-GGGVVQPGRSLRLSCAASG-LTFS | N-----YVMH | WVRQAPGKGLEWVA | VIWYD---GSNKYYADSVKG | RFTISRDNSKNTLYLQMNSLRAEDTAVYYCAR | EKYSSSW----------------YDYGMDV | WGQGTTVTVSS |
| iPS:393978 | 21-225_4C12 | VH3\|3-33/D6\|6-6\|RF1/JH6 | | QVQLVES-GGGVVQPGRSLRLSCAASG-FTFS | S-----YVMH | WVRQAPGKGLEWVA | VIWYD---GSNKYYADSVKG | RFTISRDNSKNTLYLQMNSLRAEDTAVYYCAR | EKYSSSW----------------YDYGMDV | WGHGTTVTVSS |
| iPS:393986 | 21-225_7G4 | VH3\|3-33/D6\|6-6\|RF1/JH6 | | QVQLVES-GGGVVQPGRSLRLSCAASG-FTFN | N-----YVMH | WVRQAPGKGLEWVA | VIWYD---GSNKYYADSVKG | RFTISRDNSKNTLYLQMTSLRAEDTAVYYCAR | EKYSSNW----------------YDYGMDV | WGQGTTVTVSS |
| iPS:393996 | 21-225_15C11 | VH3\|3-33/D6\|6-6\|RF1/JH6 | | QVQLVES-GGGVVQPGRSLRLSCAASG-FTFS | D-----YVMH | WVRQAPGKGLEWVA | VIWYD---GSNKYYADSVKG | RFTISRDNSKNTLYLQMNSLRAEDTAVYYCAR | EKYSSSW----------------YDYGLDV | WGQGTTVTVSS |
| iPS:394041 | 21-225_5E5 | VH3\|3-33/D6\|6-6\|RF1/JH6 | | QVQLVES-GGGVVQPGRSLRLSCAASG-FTFS | N-----YVMH | WVRQAPGKGLEWVA | VIWYD---GSNKYYADSVKG | RFTISRDNSKNTLYLQMNSLRAEDTAVYYCTR | EVYSSGW----------------YDYGMDV | WGQGTTVTVSS |
| | | Germline | | H_FR1 | H_CDR1 | H_FR2 | H_CDR2 | H_FR3 | H_CDR3 | H_FR4 |
| | VH3\|3-33/D2\|2-8\|RF3/JH4 | | 32213 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DIVLMVY-----------------AIYFDY | WGQGTLVTVSS |
| iPS:426118 | 21-225_7A10 | VH3\|3-33/D2\|2-8\|RF3/JH4 | | QVQLVES-GGGVVQPGRSLRLSCAASG-FNFS | S-----YGMH | WVRQAPGKGLEWMA | VIWYD---GSNKYYADSVKG | RFTISRDNSKNTLYLQMHSLRAEDTAVYYCAR | DERLG---------------------IFDY | WGQGTLVTVSS |
| iPS:393844 | 21-225_3G7 | VH3\|3-33/D2\|2-8\|RF3/JH4 | | QVQLVES-GGGVVQPGRSLRLSCAASG-FNFS | N-----YGMH | WVRQAPGKGLEWVA | VIWHD---GSNKYYVDSVKG | RFTISRDNSKNTVYLQMNSLRAEDTAVYYCAR | DERLG---------------------IFDY | WGQGTLVTVSS |
| iPS:393852 | 21-225_12A10 | VH3\|3-33/D2\|2-8\|RF3/JH4 | | QVQLVES-GGGVVQPGRSLRLSCAASG-FTFS | S-----YGMH | WVRQAPGKGLEWVA | VIWHD---ESNKYYTDSVKG | RFTISRDNSKNTLYLQMNSLRAEDTAVYYCAR | DERLG---------------------IFDY | WGQGTLVTVSS |
| iPS:393868 | 21-225_9C11 | VH3\|3-33/D2\|2-8\|RF3/JH4 | | QVQLVES-GGGVVQPGRSLRLSCAASG-FTFS | S-----YGMH | WVRQAPGKGLEWVA | VIWHD---ETNKYYADSVKG | RFTISRDNSKNTLSLQMNSLRAEDTAVYYCAR | DERLG---------------------IFDY | WGQGTLVTVSS |
| iPS:393900 | 21-225_10E12 | VH3\|3-33/D2\|2- | | QVQLVES-GGGVVQPGRSLRL | N-----YGMH | WVRQVPGKGLEWVA | VIWHD---GSNKYYVDS | RFTISRDNSKNTLSLQMNSLRAEDTAVYCCAR | DERLG---------------------IFDY | WGQGTLVTVS |

| | | | | | | | | | | |
|---|---|---|---|---|---|---|---|---|---|---|
| iPS:3 93920 | 21-225_1H12 | VH3\|3-33/D2\|2-8\|RF3/JH4 | | QVQLVES-GGGVVQSGRSLRLSCAASG-FNFS | S-----YGMH | WVRQAPGKGLEWVA | IIWHD---GSNKYYVDSVKG | RFTISRDNSKNTLYLQMNSLRAEDTAVYYCAR | DERLG-----------------------IFDY | WGQGTLVTVSS |
| iPS:3 93932 | 21-225_10F5 | VH3\|3-33/D2\|2-8\|RF3/JH4 | | QVQLVES-GGGVVQPGRSLRLSCAASG-FNFS | S-----YGMH | WVRQAPGKGLEWVS | IIWHD---GSNKYYVDSVKG | RFTISRDNSKNTLYLQMNSLRAEDTAVYYCAR | DERLG-----------------------IFDY | WGQGTLVTVSS |
| | | Germline | | H_FR1 | H_CDR1 | H_FR2 | H_CDR2 | H_FR3 | H_CDR3 | H_FR4 |
| | VH3\|3-33/D6\|6-6\|RF1/JH4 | | 32214 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | EYSSS--------------------SYFDY | WGQGTLVTVSS |
| iPS:4 26124 | 21-225_32D6 | VH3\|3-33/D6\|6-6\|RF1/JH4 | | QVQLVES-GGGVVQPGRSLRLSCAASG-FTFS | S-----YGMH | WVRQAPGKGLEWVT | VIWHD---GSNAYYADSVKG | RFTISRDNSKNTLYLQMNSLRAEDTAVYYCAR | ENSSS--------------------YYFDY | WGQGTLVTVSS |
| iPS:3 92922 | 21-225_30G4 | VH3\|3-33/D6\|6-6\|RF1/JH4 | | QVQLVES-GGGVVQPGRSLRLSCAASG-FTFS | S-----YGMH | WVRQAPGRGLEWVA | VIWYD---GTDKYYVDSVKG | RFTISRDNSKNTLYLQMNSLRAEDSAVYYCAR | ENSSS--------------------YYFDY | WGQGTLVTVSS |
| iPS:3 93002 | 21-225_30G1 | VH3\|3-33/D6\|6-6\|RF1/JH4 | | QVQLVES-GGGVVQPGRSLRLSCAASG-FTFS | Y-----YGMH | WVRQAPGKGLEWVA | VIWHD---GSNKYYVDSVKG | RFTISRDNSKNTLYLQMNSLRAEDTAVYYCAR | ENSSS--------------------YYFDY | WGQGTLVTVSS |
| iPS:3 93066 | 21-225_34D3 | VH3\|3-33/D6\|6-6\|RF1/JH4 | | QVQLVES-GGGVVQPGRSLRLSCAASG-FTFS | Y-----YGMH | WVRQAPGKGLEWVA | VIWHD---GSNKYYVDSVKG | RFTISRDNSKNTLYLQMNSLRAEDTAVYYCAR | ENSSS--------------------FYFDY | WGQGTLVTVSS |
| iPS:3 93092 | 21-225_33C12 | VH3\|3-33/D6\|6-6\|RF1/JH4 | | QVQLVES-GGGVVQPGRSLRLSCAASG-FTFS | H-----YGMH | WVRQAPGKGLEWVA | VIWYD---GSNKYYADSVKG | RFTISRDNSKNTLYLQMNSLRAEDTAVYYCAR | ENSSS--------------------YYFDY | WGQGTLVTVSS |
| iPS:3 93100 | 21-225_36B8 | VH3\|3-33/D6\|6-6\|RF1/JH4 | | QVQLVES-GGGVVQPGRSLRLSCAASG-FTFS | N-----YGMH | WVRQAPGKGLEWVA | VIWHD---GSNKYYGDSVKG | RFTISRDNSKNTLYLQMNSLRAEDTAVYYCAR | ENSSS--------------------YYFDY | WGQGTLVTVSS |
| iPS:3 93122 | 21-225_33B2 | VH3\|3-33/D6\|6-6\|RF1/JH4 | | QVQLVES-GGGVVQPGRSLRLSCAASG-FTFS | N-----YGMH | WVRQAPGKGLEWVA | VIWHD---GSNKYYADSVKG | RFTISRDNSKNTLYLQMNSLRAEDTAVYYCAR | ENSSS--------------------YYFDY | WGQGTLVTVSS |
| iPS:3 93134 | 21-225_34C2 | VH3\|3-33/D6\|6-6\|RF1/JH4 | | QVQLVES-GGGVVQPGRSLRLSCAASG-FTFS | N-----YVMH | WVRQAPGKGLEWVA | LIWYD---GSNKYYADSVKG | RFTISRDNSKNTLYLQMNSLRAEDTAVYYCAR | ENSSS--------------------YYFDY | WGQGTLVTVSS |
| iPS:3 93136 | 21-225_34D8 | VH3\|3-33/D6\|6- | | QVQLVES-GGGVVQPGRSLRL | N-----YGMH | WVRQAPGKGLEWVA | VIWYD---GSNKYYADS | RFTISRDNSKNTLYLQMNSLRAEDTAVYYCAR | ENSSS--------------------YYFDY | WGQGTLVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH3\|3-30.3/D6\|6-19\|RF2/JH6 | | 32215 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | GIAVAGYY---------------YYYGMDV | WGQGTTVTVSS |
| iPS:451135 | 21-225_64A11 | VH3\|3-30.3/D6\|6-19\|RF2/JH6 | | QVQLVES-GGGVVQPGRSLRLSCAASG-FTFS | N-----YGMH | WVRQAPGKGLEWVA | VISYV---GSNKYYADSVKG | RFTISRDNSKNTLYLQMNTLRAEDTAVYYCAR | RGAVAP------------------YYGMDV | WGQGTTVTVSS |
| | | Germline | | H_FR1 | H_CDR1 | H_FR2 | H_CDR2 | H_FR3 | H_CDR3 | H_FR4 |
| | VH1\|1-08/D6\|6-19\|RF1/JH5 | | 32216 | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | GYSSGW------------------YNWFDP | WGQGTLVTVSS |
| iPS:451137 | 21-225_74A7 | VH1\|1-08/D6\|6-19\|RF1/JH5 | | QVQLVQS-GAEVKKPGASVKVSCKASG-YTFT | N-----YDIN | WVRQATGQGLEWMG | WMNPN---SGNTGYAQKFQG | RVTMTRNTSTSTAHMELSSLRSEDTAVYYCAV | SSGWN---------------------WFDP | WGQGTLVTVSS |
| iPS:434285 | 21-225_57A11 | VH1\|1-08/D6\|6-19\|RF1/JH5 | | QVQLVQS-GAEVKKPGASVKVSCKASG-YTFT | N-----YDIN | WVRQATGQGLEWMG | WMNPN---SVNTGYAQKFQG | RVTMTRDTSISTAYMELSSLRSEDTAVYYCAI | SSGWN---------------------WFDP | WGQGTLVTVSS |
| iPS:434287 | 21-225_57F12 | VH1\|1-08/D6\|6-19\|RF1/JH5 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAI | SSGWY---------------------RFDP | WGQGTLVTVSS |
| iPS:434479 | 21-225_76H1 | VH1\|1-08/D6\|6-19\|RF1/JH5 | | QVQLVQS-GAEVKKPGASVKVSCKASG-YTFT | N-----YDIN | WVRQVPGQGLEWMG | WMHPN---SGNTGYAQKFQG | RVTMTRNTSISTAYMELSSLRSEDTAVYYCAI | SSGWY---------------------WFDP | WGQGTLVTVSS |
| iPS:434481 | 21-225_74B10 | VH1\|1-08/D6\|6-19\|RF1/JH5 | | QVQLVQS-GAEVKKPGASVKVSCKASG-FTFT | N-----YDIN | WVRQATGQGLEWMG | WMHPN---SGNTGFAQKFQG | RVTMTRNTSISTAYMELSSLRSEDTAVYYCAV | SSGWY---------------------WFDP | WGQGTLVTVSS |
| iPS:434483 | 21-225_74C12 | VH1\|1-08/D6\|6-19\|RF1/JH5 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAI | SSGWY---------------------WFDP | WGQGTLVTVSS |
| iPS:434493 | 21-225_76F3 | VH1\|1-08/D6\|6-19\|RF1/JH5 | | QVQLVQS-GAEVKKPGASVKVSCKASG-YTFT | N-----YDIN | WVRQATGQGLEWMG | WMNPN---SGNTGYAQKFQG | RVTMTRNTSTSTAHMELSSLRSEDTAVYYCAV | SSGWN---------------------WFDP | WGQGTLVTVSS |
| iPS:434509 | 21-225_76F5 | VH1\|1-08/D6\|6-19\|RF1/JH5 | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAS | SSGWY---------------------WFDP | WGQGTLVTVSS |
| iPS:434513 | 21-225_76A6 | VH1\|1-08/D6\|6- | | QVQLVQS-GAEVKKPGASVKV | N-----YDIN | WVRQATGQGLEWMG | WMHPN---NGNTGYAQK | RVTMTRNTSISTAYMELNSLRSEDTAVYYCAI | SSGWY---------------------WFDP | WGQGTLVTVS |

<table>
<tr><td>iPS:434515</td><td>21-225_74A5</td><td>VH1|1-08/D6|6-19|RF1/JH5</td><td></td><td>QVQLVQS-GAEVKKPGASVKVSCKASG-YTFT</td><td>N-----YDIN</td><td>WVRQVPGQGLEWMG</td><td>WMHPN---SGNTGYAQKFQG</td><td>RVTMTRNTSISTAYMELSSLRSEDTAVYYCAI</td><td>SSGWY--------------------WFDP</td><td>WGQGTLVTVSS</td></tr>
<tr><td>iPS:434525</td><td>21-225_76E8</td><td>VH1|1-08/D6|6-19|RF1/JH5</td><td></td><td>QVQLVQS-GAEVKKPGASVKVSCKASG-YTFP</td><td>N-----YDIN</td><td>WVRQATGQGLEWMG</td><td>WMNPN---SGNTGYAQKFQG</td><td>RVTMTRNTSISTAYMELSSLRSEDTAVYYCAV</td><td>SSGWH--------------------WFDP</td><td>WGQGTLVTVAS</td></tr>
<tr><td>iPS:434529</td><td>21-225_76B9</td><td>VH1|1-08/D6|6-19|RF1/JH5</td><td></td><td>QVQLVQS-GAEVKKPGASVKVSCKASG-YTFT</td><td>N-----YDIN</td><td>WVRQVPGQGLEWMG</td><td>WMHPN---SGNTGYAQKFQG</td><td>RVTMTRNTSISTAYMELSSLRSEDTAVYYCAI</td><td>SSGWY--------------------WFDP</td><td>WGQGTLVTVSS</td></tr>
<tr><td>iPS:434575</td><td>21-225_77C7</td><td>VH1|1-08/D6|6-19|RF1/JH5</td><td></td><td>QVQLVQS-GAEVKKPGASVKVSCKASG-YTFP</td><td>N-----YDIN</td><td>WVRQATGQGLEWMG</td><td>WMNPN---SGNTGYAQKFQG</td><td>RVTMTRNTSISTAYMELSSLRSEDTAVYYCAV</td><td>SSGWH--------------------WFDP</td><td>WGQGTLVTVAS</td></tr>
<tr><td>iPS:434583</td><td>21-225_74B6</td><td>VH1|1-08/D6|6-19|RF1/JH5</td><td></td><td>QVQLVQS-GAEVKKPGASVKVSCKASG-YTFT</td><td>N-----YDIN</td><td>WVRQATGQGLEWMG</td><td>WMHPN---NGNTGYAQKFQG</td><td>RVTMTRNTSISTAYMELNSLRSEDTAVYYCAI</td><td>SSGWY--------------------WFDP</td><td>WGQGTLVTVSL</td></tr>
<tr><td>iPS:434587</td><td>21-225_74G3</td><td>VH1|1-08/D6|6-19|RF1/JH5</td><td></td><td>QVQLVQS-GAEVKKPGASVKVSCKASG-YTFT</td><td>N-----YDIN</td><td>WVRQVPGQGLEWMG</td><td>WMHPN---SGNTGYAQKFQG</td><td>RVTMTRNTSISTAYMELSSLRSEDTAVYYCAI</td><td>SSGWY--------------------WFDP</td><td>WGQGTLVTVSS</td></tr>
<tr><td>iPS:434597</td><td>21-225_77C10</td><td>VH1|1-08/D6|6-19|RF1/JH5</td><td></td><td>QVQLVQS-GAEVKKPGASVKVSCKASG-YTFT</td><td>N-----YDIN</td><td>WVRQATGQGLEWMG</td><td>WMHPN---SGNTGYAQKFQG</td><td>RVTMTRNTSISTAYMELSSLRSEDTAVYYCAI</td><td>SSGWY--------------------WFDP</td><td>WGQGTLVTVSS</td></tr>
<tr><td>iPS:434603</td><td>21-225_77D11</td><td>VH1|1-08/D6|6-19|RF1/JH5</td><td></td><td>QVQLVQS-GAEVKKPGASVKVSCKASG-YTFT</td><td>N-----YDIN</td><td>WVRQVPGQGLEWMG</td><td>WMHPN---SGNTGYAQKFQG</td><td>RVTMTRNTSISTAYMELSSLRSEDTAVYYCAI</td><td>SSGWY--------------------WFDP</td><td>WGQGTLVTVSS</td></tr>
<tr><td>iPS:434613</td><td>21-225_77D12</td><td>VH1|1-08/D6|6-19|RF1/JH5</td><td></td><td>QVQLVQS-GAEVKKPGASVKVSCKASG-YTFT</td><td>N-----YDIN</td><td>WVRQATGQGLEWMG</td><td>WMNPN---SGNTGYAQKFQG</td><td>RVTMTRNTSINTAYMELSSLRSEDTAVYYCAI</td><td>SSGWY--------------------WFDP</td><td>WGQGTLVTVSS</td></tr>
<tr><td>iPS:434617</td><td>21-225_74B8</td><td>VH1|1-08/D6|6-19|RF1/JH5</td><td></td><td>QVQLVQS-GAEVKKPGASVKVSCKASG-YTFT</td><td>N-----YDIN</td><td>WVRQATGQGLEWMG</td><td>WMHPN---SGNTGYAQKFQG</td><td>RVTLTRNTSISTAYMELSSLRSEDTAVYYCAV</td><td>SSGWY--------------------WFDP</td><td>WGQGTLVTVSS</td></tr>
<tr><td>iPS:434619</td><td>21-225_78C1</td><td>VH1|1-08/D6|6-19|RF1/JH5</td><td></td><td>QVQLVQS-GAEVKKPGASVKVSCKASG-YTFT</td><td>N-----YDIN</td><td>WVRQATGQGLEWMG</td><td>WMHPN---SGNTGYAQKFQG</td><td>RVTMTRNTSISTAYMELSSLRSEDTAVYYCAI</td><td>SSGWY--------------------WFDP</td><td>WGQGTLVTVSS</td></tr>
<tr><td>iPS:434639</td><td>21-225_74B7</td><td>VH1|1-08/D6|6-</td><td></td><td>QVQLVQS-GAEVKKPGASVKV</td><td>N-----YDIN</td><td>WVRQATGQGLEWMG</td><td>WMNPN---SGNTGYAQK</td><td>RVTMTRNTSISTAYMELSSLRSEDTAVYYCAV</td><td>SSGWH--------------------WFDP</td><td>WGQGTLVTVA</td></tr>
</table>

| | | | | | | | | | | |
|---|---|---|---|---|---|---|---|---|---|---|
| **iPS:434653** | **21-225_74B5** | **VH1\|1-08/D6\|6-19\|RF1/JH5** | | QVQLVQS-GAEVKKPGASVKVSCKASG-YTFT | N-----YDIN | WVRQATGQGLEWMG | WMNPN---SGNTGYAQKFQG | RVTMTRNTSTSTAHMELSSLRSEDTAVYYCAV | SSGWN-----------------------WFDP | WGQGTLVTVSS |
| **iPS:434655** | **21-225_78H12** | **VH1\|1-08/D6\|6-19\|RF1/JH5** | | QVQLVQS-GAEVKKPGASVKVSCKASG-YTFP | N-----YDIN | WVRQATGQGLEWMG | WMNPN---SGNTGYAQKFQG | RVTMTRNTSISTAYMELSSLRSEDTAVYYCAV | SSGWH-----------------------WFDP | WGQGTLVTVAS |
| **iPS:434675** | **21-225_79G6** | **VH1\|1-08/D6\|6-19\|RF1/JH5** | | QVQLVQS-GAEVKKPGASVKVSCKASG-FTFT | N-----YDIN | WVRQATGQGLEWMG | WMHPN---SGNTGFAQKFQG | RVTMTRNTSISTAYMELSSLRSEDTAVYYCAV | SSGWY-----------------------WFDP | WGQGTLVTVSS |
| **iPS:434689** | **21-225_79G10** | **VH1\|1-08/D6\|6-19\|RF1/JH5** | | QVQLVQS-GAEVKKPGASVKVSCKASG-YTFT | N-----YDIN | WVRQATGQGLEWMG | WMNPN---SGNTGYAQKFQG | RVTMTRNTSTSTAHMELSSLRSEDTAVYYCAV | SSGWN-----------------------WFDP | WGQGTLVTVSS |
| **iPS:434705** | **21-225_80A2** | **VH1\|1-08/D6\|6-19\|RF1/JH5** | | QVQLVQS-GAEVKKPGASVKVSCKASG-YTFT | N-----YDIN | WVRQVPGQGLEWMG | WMHPN---SGNTGYAQKFQG | RVTMTRNTSISTAYMELSSLRSEDTAVYYCAI | SSGWY-----------------------WFDP | WGQGTLVTVSS |
| **iPS:434707** | **21-225_80D3** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAI | SSGWY-----------------------WFDP | WGQGTLVTVSS |
| **iPS:434731** | **21-225_80E9** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAI | SSGWY-----------------------WFDP | WGQGTLVTVSS |
| **iPS:434747** | **21-225_80C12** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---NGNTGYAQKFQG | RVTMTRNTSISTAYMELNSLRSEDTAVYYCAI | SSGWY-----------------------WFDP | WGQGTLVTVSL |
| **iPS:434761** | **21-225_81E5** | **VH1\|1-08/D6\|6-19\|RF1/JH5** | | QVQLVQS-GAEVKKPGASVKVSCKASG-YTFT | N-----YDIN | WVRQATGQGLEWMG | WMNPN---SGNTGYAQKFQG | RVTMTRNTSTSTAHMELSSLRSEDTAVYYCAV | SSGWN-----------------------WFDP | WGQGTLVTVSS |
| **iPS:434771** | **21-225_81F9** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAI | SSGWY-----------------------WFDP | WGQGTLVTVSS |
| **iPS:434793** | **21-225_82A5** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---NGNTGYAQKFQG | RVTMTRNTSISTAYMELNSLRSEDTAVYYCAI | SSGWY-----------------------WFDP | WGQGTLVTVSL |
| **iPS:434797** | **21-225_82G5** | **VH1\|1-08/D6\|6-** | | QVQLVQS-GAEVKKPGASVKV | N-----YDIN | WVRQVPGQGLEWMG | WMHPN---SGNTGYAQK | RVTMTRNTSISTAYMELSSLRSEDTAVYYCAI | SSGWY-----------------------WFDP | WGQGTLVTVS |

| | | | | | | | | | | |
|---|---|---|---|---|---|---|---|---|---|---|
| **iPS:434805** | **21-225_82D9** | **VH1\|1-08/D6\|6-19\|RF1/JH5** | | QVQLVQS-GAEVKKPGASVKVSCKASG-YTFT | N-----YDIN | WVRQVPGQGLEWMG | WMHPN---SGNTGYAQKFQG | RVTMTRNTSISTAYMELSSLRSEDTAVYYCAI | SSGWY---------------------WFDP | WGQGTLVTVSS |
| **iPS:434813** | **21-225_82C12** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---NGNTGYAQKFQG | RVTMTRNTSISTAYMELNSLRSEDTAVYYCAI | SSGWY---------------------WFDP | WGQGTLVTVSL |
| **iPS:434825** | **21-225_83C2** | **VH1\|1-08/D6\|6-19\|RF1/JH5** | | QVQLVQS-GAEVKKPGASVKVSCKASG-YTFT | N-----YDIN | WVRQVPGQGLEWMG | WMHPN---SGNTGYAQKFQG | RVTMTRNTSISTAYMELSSLRSEDTAVYYCAI | SSGWY---------------------WFDP | WGQGTLVTVSS |
| **iPS:434827** | **21-225_83F3** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAI | SSGWY---------------------WFDP | WGQGTLVTVSS |
| **iPS:434829** | **21-225_83G3** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAI | SSGWY---------------------WFDP | WGQGTLVTVSS |
| **iPS:434833** | **21-225_83C5** | **VH1\|1-08/D6\|6-19\|RF1/JH5** | | QVQLVQS-GAEVKKPGASVKVSCKASG-YTFT | N-----YDIN | WVRQVPGQGLEWMG | WMHPN---SGNTGYAQKFQG | RVTMTRNTSISTAYMELSSLRSEDTAVYYCAI | SSGWY---------------------WFDP | WGQGTLVTVSS |
| **iPS:434841** | **21-225_83G7** | **VH1\|1-08/D6\|6-19\|RF1/JH5** | | QVQLVQS-GAEVKKPGASVKVSCKASG-YTFP | N-----YDIN | WVRQATGQGLEWMG | WMNPN---SGNTGYAQKFQG | RVTMTRNTSISTAYMELSSLRSEDTAVYYCAV | SSGWH---------------------WFDP | WGQGTLVTVAS |
| **iPS:434863** | **21-225_84G7** | **VH1\|1-08/D6\|6-19\|RF1/JH5** | | QVQLVQS-GAEVKKPGASVKVSCKASG-YTFP | N-----YDIN | WVRQATGQGLEWMG | WMNPN---SGNTGYAQKFQD | RVTMTRNTSISTAYMELSSLRSEDTAVYYCAV | SSGWH---------------------WFDP | WGQGTLVTVAS |
| **iPS:434877** | **21-225_85H2** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---SGNTGYAQKFQG | RVTLTRNTSISTAYMELSSLRSEDTAVYYCAV | SSGWY---------------------WFDP | WGQGTLVTVSS |
| **iPS:434883** | **21-225_85B5** | **VH1\|1-08/D6\|6-19\|RF1/JH5** | | QVQLVQS-GAEVKKPGASVKVSCKASG-YTFT | N-----YDIN | WVRQVPGQGLEWMG | WMHPN---SGNTGYAQKFQG | RVTMTRNTSISTAYMELSSLRSEDTAVYYCAI | SSGWY---------------------WFDP | WGQGTLVTVSS |
| **iPS:434911** | **21-225_85D11** | **VH1\|1-08/D6\|6-19\|RF1/JH5** | | QVQLVQS-GAEVKKPGASVKVSCKASG-YTFT | N-----YDIN | WVRQVPGQGLEWMG | WMHPN---SGNTGYAQKFQG | RVTMTRNTSISTAYMELSSLRSEDTAVYYCAI | SSGWY---------------------WFDP | WGQGTLVTVSS |
| **iPS:434935** | **21-225_86E9** | **VH1\|1-08/D6\|6-** | | QVQLVQS-GAEVKKPGASVKV | N-----YDIN | WVRQATGQGLEWMG | WMNPN---SGNTGYAQK | RVTMTRSTSTSTAHMELSSLRSEDTAVYYCAV | SSGWS---------------------WFDP | WGQGTLVTVS |

| | | | | | | | | | | |
|---|---|---|---|---|---|---|---|---|---|---|
| **iPS:434957** | **21-225_87A10** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---NGNTGYAQKFQG | RVTMTRNTSISTAYMELNSLRSEDTAVYYCAI | SSGWY---------------------WFDP | WGQGTLVTVSL |
| **iPS:434971** | **21-225_88G2** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAI | SSGWY---------------------WFDP | WGQGTLVTVSS |
| **iPS:435051** | **21-225_90D9** | **VH1\|1-08/D6\|6-19\|RF1/JH5** | | QVQLVQS-GAEVKKPGASVKVSCKASG-YTFP | N-----YDIN | WVRQATGQGLEWMG | WMNPN---SGNTGYAQKFQG | RVTMTRNTSISTAYMELSSLRSEDTAVYYCAV | SSGWH---------------------WFDP | WGQGTLVTVAS |
| **iPS:435071** | **21-225_91F1** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAI | SSGWY---------------------WFDP | WGQGTLVTVSS |
| **iPS:435087** | **21-225_91G8** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAI | SSGWY---------------------WFDP | WGQGTLVTVSS |
| **iPS:435203** | **21-225_75A7** | **VH1\|1-08/D6\|6-19\|RF1/JH5** | | QVQLVQS-GAEVKKPGASVKVSCKASG-YTFP | N-----YDIN | WVRQATGQGLEWMG | WMNPN---SGNTGYAQKFQD | RVTMTRNTSISTAYMELSSLRSEDTAVYYCAV | SSGWH---------------------WFDP | WGQGTLVTVAS |
| **iPS:435211** | **21-225_94E11** | **VH1\|1-08/D6\|6-19\|RF1/JH5** | | QVQLVQS-GAEVKKPGASVKVSCKASG-YTFT | N-----YDIN | WVRQATGQGLEWMG | WMNPN---SGNTGYAQKFQG | RVTMTRNTSTSTAHMELSSLRSEDTAVYYCAV | SSGWK---------------------WFDP | WGQGTLVTVSS |
| **iPS:435227** | **21-225_95G4** | **VH1\|1-08/D6\|6-19\|RF1/JH5** | | QVQLVQS-GAEVKKPGASVKVSCKASG-YTFT | N-----YDIN | WVRQATGQGLEWMG | WMNPN---SGNTGYAQKFQG | RVTMTRNTSTSTAHMELSSLRSEDTAVYYCAV | SSGWN---------------------WFDP | WGQGTLVTVSS |
| **iPS:435245** | **21-225_95E12** | **VH1\|1-08/D6\|6-19\|RF1/JH5** | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAI | SSGWY---------------------WFDP | WGQGTLVTVSS |
| **iPS:435247** | **21-225_96G1** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---NGNTGYAQKFQG | RVTMTRNTSISTAYMELNSLRSEDTAVYYCAI | SSGWY---------------------WFDP | WGQGTLVTVSL |
| **iPS:435249** | **21-225_96E2** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---SGNTGYAQKFQG | RVTLTRNTSISTAYMELSSLRSEDTAVYYCAV | SSGWY---------------------WFDP | WGQGTLVTVSS |
| **iPS:435255** | **21-225_96D5** | **VH1\|1-08/D6\|6-** | | QVQLVQS-GAEVKKPGASVKV | N-----YDIN | WVRQATGQGLEWMG | WMNPN---SGNTGYAQK | RVTMTRSTSTSTAHMELSSLRSEDTAVYYCAV | SSGWS---------------------WFDP | WGQGTLVTVS |

| | | | | | | | | | | |
|---|---|---|---|---|---|---|---|---|---|---|
| **iPS:4 35279** | **21-225_97H4** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAI | SSGWY---------------------WFDP | WGQGTLVTVSS |
| **iPS:4 35327** | **21-225_147G6** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAY | SSGWY-----------------------WFDP | WGQGTLVTVSS |
| **iPS:4 35437** | **21-225_152F4** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSDDTAVYYCAY | SSGWY-----------------------WFDP | WGQGTLVTVSS |
| **iPS:4 35701** | **21-225_170F6** | **VH1\|1-08/D6\|6-19\|RF1/JH5** | | QVQLVQS-GAEVKKPGASVKVSCKASG-YTFT | N-----YDIN | WVRQATGQGLEWMG | WMNPN---SGNTGYAQKFQD | RVTMTRHTSISTAYMELSSLRSEDTAVYYCAI | SSGWY-----------------------WFDP | WGQGTLVTVSS |
| **iPS:4 35737** | **21-225_174G5** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---SGNTGYAQKFQG | RVTMTRNTSISTAYMELSSLRSEDTAVYYCAV | SSGWY-----------------------WFDP | WGQGTLVTVSS |
| **iPS:4 36544** | **21-225_224H5** | **VH1\|1-08/D6\|6-19\|RF1/JH5** | | QVQLVQS-GAEVKKPGASVKVSCKASG-YTFT | N-----YDIN | WVRQATGQGLEWMG | WMNPN---SGNTGYAQKFQG | RVTMTRNTSISTAYMELSSLRSEDTAVYYCAS | SSGWN-----------------------WFDP | WGQGTLVTVSS |
| **iPS:4 36570** | **21-225_225F4** | **VH1\|1-08/D6\|6-19\|RF1/JH5** | | QVQLVQS-GAEVKKPGASVKVSCKASG-YTFT | N-----YDIN | WVRQATGQGLEWMG | WMHPN---SGSTGYAQKFQG | RLTMTRNTSISTVYMELNSLRSEDTAVYYCAS | SSGWY-----------------------WFDP | WGQGTLVTVSS |
| **iPS:4 36644** | **21-225_227G9** | **VH1\|1-08/D6\|6-19\|RF1/JH5** | | QVQLVQS-GAEVKKPGASVKVSCRASG-YTFT | N-----YDIN | WVRQATGRGLEWMG | WMYPN---SGNTGYAQKFQG | RVTMTRNTSISTAYMELSSLRSEDTAVYYCAL | SSGWY-----------------------WFDP | WGQGTLVTVSS |
| **iPS:4 37322** | **21-225_75B1** | **VH1\|1-08/D6\|6-19\|RF1/JH5** | | QVQLVQS-GAEVKKPGASVKVSCKASG-YTFT | N-----YDIN | WVRQVPGQGLEWMG | WMHPN---SGNTGYAQKFQG | RVTMTRNTSISTAYMELSSLRSEDTAVYYCAI | SSGWY-----------------------WFDP | WGQGTLVTVSS |
| **iPS:4 37361** | **21-225_74C1** | **VH1\|1-08/D6\|6-19\|RF1/JH5** | | QVQLVQS-GAEVKKPGASVKVSCKASG-YTFT | N-----YDIN | WVRQATGQGLEWMG | WMNPD---SGNTGFAQKFQG | RVTMTRNTSISTAYMELSSLRSEDTAVYYCAV | SSGWY-----------------------WFDP | WGQGTLVTVSS |
| **iPS:4 37363** | **21-225_74C10** | **VH1\|1-08/D6\|6-19\|RF1/JH5** | | QVQLVQS-GAEVKKPGASVKVSCKASG-YTFT | N-----YDIN | WVRQATGQGLEWMG | WMNPN---SGNIGYAQKFQG | RVTMTRNTSISTAYMELSSLRSEDTAVYYCAI | SSGWY-----------------------WFDP | WGQGTLVTVSS |
| **iPS:4 37379** | **21-225_74H2** | **VH1\|1-08/D6\|6-** | | QVQLVQS-GAEVKKPGASVKV | N-----YDIN | WVRQATGQGLEWMG | WMHPN---SGNTGFAQK | RVTMTRNTSISTAYMELSSLRSEDTAVYYCAV | SSGWY-----------------------WFDP | WGQGTLVTVS |

| | | | | | | | | | | |
|---|---|---|---|---|---|---|---|---|---|---|
| iPS:446094 | 21-225_77E1 | VH1\|1-08/D6\|6-19\|RF1/JH5 | | QVQLVQS-GAEVKKPGASVKVSCKASG-YTFP | N-----YDIN | WVRQATGQGLEWMG | WMNPN---SGNTGYAQKFQG | RVTMTRNTSISTAYMELSSLRSEDTAVYYCAV | SSGWH--------------------WFDP | WGQGTLVTVAS |
| iPS:451129 | 21-225_94D2 | VH1\|1-08/D6\|6-19\|RF1/JH5 | | QVQLVQS-GAEVKKPGASVKVSCKASG-FTFT | N-----YDIN | WVRQATGQGLEWMG | WMHPN---SGNTGFAQKFQG | RVTMTRNTSISTAYMELSSLRSEDTAVYYCAV | SSGWY--------------------WFDP | WGQGTLVTVSS |
| iPS:451133 | 21-225_95H4 | VH1\|1-08/D6\|6-19\|RF1/JH5 | | QVQLVQS-GAEVKKPGASVKVSCKASG-YTFT | N-----YDIN | WVRQATGQGLEWMG | WMNPN---SGNTGYAQKFQG | RVTMTRNTSTSTAHMELSSLRSEDTAVYYCAV | SSGWN--------------------WFDP | WGQGTLVTVSS |
| iPS:398510 | 21-225_25A3 | VH1\|1-08/D6\|6-19\|RF1/JH5 | | QVQLVQS-GAEVKKPGASVKVSCKASG-YIFT | N-----YDIN | WVRQATGQGLEWMG | WMHPN---SGNTGYAQKFQG | RVTMTWNTSISTANMELSSLRSEDTAVYYCAS | SSGWY--------------------WFDP | WGQGTLVTVSS |
| iPS:398516 | 21-225_26A9 | VH1\|1-08/D6\|6-19\|RF1/JH5 | | QVQLVQS-GAEVKKPGASVKVSCKASG-YTFT | N-----YDIN | WVRQATGQGLEWMG | WMHPN---SGNTGCAQKFQG | RVTMTWNMSISTAYMELSSLRSEDTAVYYCAS | SSGWY--------------------WFDP | WGQGTLVTVSS |
| | | Germline | | H_FR1 | H_CDR1 | H_FR2 | H_CDR2 | H_FR3 | H_CDR3 | H_FR4 |
| | VH3\|3-30.3/D5\|5-18\|RF3/JH4 | | 32217 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | GYSYG-------------------YYFDY | WGQGTLVTVSS |
| iPS:451139 | 21-225_71A6 | VH3\|3-30.3/D5\|5-18\|RF3/JH4 | | QVQLVES-GGGVVQPGRSLRLSCAASG-FTFS | N-----YGMH | WVRQAPGKGLEWVA | VISYD---GSNEYYADSVKG | RFTISRDNSKNTLYLQMNSLRAEDTAVYYCAR | DHRYGV------------------RGGFDY | WGQGTLVTVSS |
| | | Germline | | H_FR1 | H_CDR1 | H_FR2 | H_CDR2 | H_FR3 | H_CDR3 | H_FR4 |
| | VH1\|1-18/D6\|6-19\|RF2/JH5 | | 32218 | QVQLVQS-GAEVKKPGASVKVSCKASG-YIFT | S-----YGIS | WVRQAPGQGLEWMG | WISAY---NGNTNYAQKLQG | RVTMTTDTSTSTAYMELRSLRSDDTAVYYCAR | GIAVAG-------------------NWFDP | WGQGTLVTVSS |
| iPS:451143 | 21-225_66H11 | VH1\|1-18/D6\|6-19\|RF2/JH5 | | QVQLVQS-GAEVKKPGASVKVSCKASG-YTFA | T-----YGIS | WVRQAPGQGLEWMG | WISAY---NGNTNYAQKLQG | RVTMTTDTSTSTAYMELRSLRSDDTAVYYCAR | GEAVA--------------------VFDP | WGQGTLVTVSS |
| iPS:434361 | 21-225_65D5 | VH1\|1-18/D6\|6-19\|RF2/JH5 | | QVQLVQS-GAEVKKPGASVKVSCKASG-YTFP | S-----YGIS | WVRQAPGQGLEWMG | WISAY---SGNTNYAQKLQG | RVTMTTDTSTSTAYMELRSLRSDDTAVYFCAR | GEAVA--------------------VFDP | WGQGTLVTVSS |
| | | Germline | | H_FR1 | H_CDR1 | H_FR2 | H_CDR2 | H_FR3 | H_CDR3 | H_FR4 |
| | VH3\|3-33/D4\|4-23\|RF2/JH6 | | 32219 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DYGGNSYY---------------YYYGMDV | WGQGTTVTVSS |
| iPS:453445 | 21-225_148E10 | VH3\|3-33/D4\|4- | | QVQLVES-GGGVVQPGRSLRL | S-----YGMH | WVRQAPGKGLEWVA | VIWFD---GSNKYYVDS | RFTISRDNSKNTLYLQMNSLRAEDTAVYYCAR | DRVEGSGTP-------------YYYYGMDV | WGQGTTVTVS |

| | | | | | | | | | | |
|---|---|---|---|---|---|---|---|---|---|---|
| **iPS:436082** | **21-225_195D9** | **VH3\|3-33/D4\|4-23\|RF2/JH6** | | QVQLVES-GGGVVQPGRSLRLSCAASG-FTFS | H-----YVMH | WVRQAPGKGLEWVA | VIWYD---GTNKYYADSVKG | RFTISRDNSKNTLYLQMNSLRAEDTAVYYCAR | DWFGEGN-----------------YYGMDV | WGQGTTVTVSS |
| **iPS:436118** | **21-225_196A10** | **VH3\|3-33/D4\|4-23\|RF2/JH6** | | QVQLVES-GGGVVQPGRSLRLSCAASG-FTFS | H-----YVMH | WVRQAPGKGLEWVA | VIWYD---GTNKYYADSVKG | RFTISRDNSKNTLYLQMNSLRAEDTAVYYCAR | DWFGEGN-----------------YYGMDV | WGQGTTVTVSS |
| **iPS:436670** | **21-225_147D9** | **VH3\|3-33/D4\|4-23\|RF2/JH6** | | QVQLVES-GGGVVQPGRSLRLSCAASG-FTFS | S-----YGMH | WVRQAPGKGLEWVA | DIWFD---GSNKYYVDSVKD | RFTISRDNSKNTLYLQMNSLRAEDTAVYYCAR | DRVEGSGTP-------------YYYGMDV | WGQGTTVTVSS |
| **iPS:436720** | **21-225_151H6** | **VH3\|3-33/D4\|4-23\|RF2/JH6** | | QVQLVES-GGGVVQPGRSLRLSCAASG-FTFS | D-----YGMH | WVRQAPGKGLEWVA | LIWYD---GSNKYYADSVKG | RFTISRDNSKNTLYLQMNSLRAEDTAVYYCAR | DDRSCSRTSC----------PYYYYYGLDV | WGQGTTVIVSS |
| **iPS:436726** | **21-225_152G5** | **VH3\|3-33/D4\|4-23\|RF2/JH6** | | QVQLVES-GGGVVQPGRSLRLSCAASG-FTFS | D-----YGMH | WVRQAPGKGLEWVA | LIWYD---GSNKYYADSVKG | RFTISRDNSKNTLYLQMNSLRAEDTAVYYCAR | DDRSCSRTSC----------PYYYYYGLDV | WGQGTTVIVSS |
| **iPS:436732** | **21-225_152B12** | **VH3\|3-33/D4\|4-23\|RF2/JH6** | | QVQVVES-GGGVVQPGRSLRLSCAASG-FTFS | D-----YGMH | WVRQAPGKGLEWVA | VIWYD---GSNKYYADSVKG | RFTISRDNSKNTLYLQMNSLRAEDTAVYYCAR | DDRSCSSTSC----------PYYYYYGLDV | WGQGTTVTVSS |
| **iPS:436734** | **21-225_153A8** | **VH3\|3-33/D4\|4-23\|RF2/JH6** | | QVQLMES-GGGVVQPGRSLRLSCAASG-FTFS | D-----YGMH | WVRQAPGKGLEWVA | LIWYD---GSNKYYADSVKG | RFTISRDNSKNTLYLQMNSLRAEDTAVYYCAR | DDRSCSRTSC----------PYYYYYGLDV | WGQGTTVIVSS |
| **iPS:436736** | **21-225_153E8** | **VH3\|3-33/D4\|4-23\|RF2/JH6** | | QVQLVES-GGGVVQPGRSLRLSCAASG-FTLS | N-----YGMH | WVRQAPGKGLEWVA | VIWFD---GSNKYYVDSVKD | RFTISRDNSKNTLYLQMNSLRAEDTAVYYCAR | DRVEGSGTP-------------YYYGMDV | WGQGTTVTVSS |
| **iPS:436756** | **21-225_146A10** | **VH3\|3-33/D4\|4-23\|RF2/JH6** | | QVQLEES-GGGVVQPGRSLRLSCAASG-FTFS | G-----YGMH | WVRQAPGKGLEWMT | LIRYD---GSDKNYADSVKG | RFTISRDNSKNTLYLQMNSLRAEDTAVYYCAR | DRVFCSSTSCL---------SYYYYGMDV | WGQGTTVTVSS |
| **iPS:436766** | **21-225_158D10** | **VH3\|3-33/D4\|4-23\|RF2/JH6** | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DRVSCSSTSC----------PYYYYYGMDV | WGQGTTVTVSS |
| **iPS:436768** | **21-225_159H8** | **VH3\|3-33/D4\|4-23\|RF2/JH6** | | QVQLVES-GGGVVQPGRSLRLSCAASG-FTFS | T-----YGMH | WVRQAPGKGLEWVA | VIWYD---GSNKYYADSVKG | RFTISRDNSKNTLYLQMNSLRAEDTAVYYCAR | DRVSCSSTSC----------PYYYYYGMDV | WGQGTTVTVSS |
| **iPS:436770** | **21-225_160B12** | **VH3\|3-33/D4\|4-** | | QVQLVES-GGGVVQPGRSLRL | S-----YGMH | WVRQAPGKGLEWVA | VIWYD---GSNKYYADS | RFTISRDNSKNTLYLQMNSLRAEDTAVYYCAR | DRVSCSSTSC----------PYYYYYGMDV | WGQGTTVTVS |

| | | | | | | | | | | |
|---|---|---|---|---|---|---|---|---|---|---|
| **iPS:436782** | **21-225_166G11** | **VH3\|3-33/D4\|4-23\|RF2/JH6** | | QVQLVES-GGGVVQPGRSLRLSCAASG-FTFN | G-----YGMH | WVRQAPGKGLEWVA | VIWYD---GSNKYYADSVKG | RFTISRDTSKNTLFLQMNSLTAEDTAVYYCAR | DDRYCSSPTCH---------PYYYYYGLDV | WGQGTTVTVSS |
| **iPS:436794** | **21-225_170F1** | **VH3\|3-33/D4\|4-23\|RF2/JH6** | | QVQLVES-GGGVVQPGRSLRLSCAASG-FTFS | G-----YGMN | WVRQAPGKGLEWVA | IIWYD---GNNKYYADSVKG | RFTISRDNSKNTLYLQMNSLRAEDTAVYYCAR | DRVYCSSTSCH---------PYYYYYAMDV | WGQGTTVTVSS |
| **iPS:436836** | **21-225_52H1** | **VH3\|3-33/D4\|4-23\|RF2/JH6** | | QVQLVES-GGGVVQPGRSLRLSCAASG-FTFS | G-----YGMH | WVRQAPGKGLEWVA | VIWYD---GSNKYYADSVKG | RFTISRDNSKNTLYLQMNSLRAEDTAVYYCAR | DRVYCSSSSCS---------YYYYYYGMDV | WGQGTTVTVSS |
| **iPS:436922** | **21-225_78E9** | **VH3\|3-33/D4\|4-23\|RF2/JH6** | | QVQLVES-GGGVVQPGRSLRLSCAASG-FTFS | S-----YGMH | WVRQAPGKGLEWVA | VIWYD---GNNKSYADSVKG | RFTISRDISKNTLYLQMNSLRAEDTAVYYCAR | DRDYCSSTSC----------PYYYYYGMDV | WGQGTTVTVSS |
| **iPS:436924** | **21-225_74B3** | **VH3\|3-33/D4\|4-23\|RF2/JH6** | | QVQLVES-GGGVVQPGRSLRLSCAASG-FTFS | R-----YGMH | WVRQAPGKGLEWVA | VFWYD---GSNKDYADSVKG | RFTISRDNSKNTLYLQMNSLRAEDTAVYYCAR | DRDYCSSTSC----------PYYYYYGMDV | WGQGTTVTVSS |
| **iPS:436928** | **21-225_79E7** | **VH3\|3-33/D4\|4-23\|RF2/JH6** | | QVQLVES-GGGVVQPGRSLRLSCAASG-FTFS | S-----YGMH | WVRQAPGKGLEWVA | VIWYD---GNNKSYADSVKG | RFTISRDISKNTLYLQMNSLRAEDTAVYYCAR | DRDYCSSTSC----------PYYYYYGMDV | WGQGTTVTVSS |
| **iPS:436932** | **21-225_92A4** | **VH3\|3-33/D4\|4-23\|RF2/JH6** | | QVQLVES-GGGVVQPGRSLRLSCAASG-FTFS | S-----YGMH | WVRQAPGKGLEWVA | VIWYD---GNNKSYADSVKG | RFTIYRDISKNTLYLQMNSLRAEDTAVYYCAR | DRDYCSSTSC----------PYYYYYGMDV | WGQGTTVTVSS |
| **iPS:436936** | **21-225_97E6** | **VH3\|3-33/D4\|4-23\|RF2/JH6** | | QVQLVES-GGGVVQPGRSLRLSCAASG-FTFS | S-----YGMH | WVRQAPGKGLEWVA | VIWYD---GNNKSYADSVKG | RFTISRDISKNTLYLQMNSLRAEDTAVYYCAR | DRDYCSSTSC----------PYYYYYGMDV | WGQGTTVTVSS |
| **iPS:437190** | **21-225_225A9** | **VH3\|3-33/D4\|4-23\|RF2/JH6** | | QVQLVES-GGGVVQPGGSLRLSCAASG-FTFS | T-----YGMH | WVRLAPGKGLEWVA | VIWYD---GSNKYYADSVKG | RFTISRDISQNTLYLQMNSLRAEDTAVYYCAR | DNHYCSSTSCS---------PYYYYFGMDV | WGQGTTVTVSS |
| **iPS:437254** | **21-225_149F2** | **VH3\|3-33/D4\|4-23\|RF2/JH6** | | QVQLGEA-GGGVVQPGRSLRLSCAASG-FTFS | R-----YGMH | WVRQAPGKGLEWVA | FIWYD---GSENYYADSVKG | RFTISRVNSRNTLYLQMNSLRAEDTAVYYCAR | DRVEGSGTP-------------YYYYGMDV | WGQGTTVTVSS |
| **iPS:437256** | **21-225_150F11** | **VH3\|3-33/D4\|4-23\|RF2/JH6** | | QVQLGEA-GGGVVQPGRSLRLSCAASG-FTFS | R-----YGMH | WVRQAPGKGLEWVA | FIWYD---GSENYYADSVKG | RFTISRVNSRNTLYLQMNSLRAEDTAVYYCAR | DRVEGSGTP-------------YYYYGMDV | WGQGTTVTVSS |
| **iPS:451110** | **21-225_74C9** | **VH3\|3-33/D4\|4-** | | QVQLVES-GGGVVQPGRSLRL | S-----YGMH | WVRQAPGKGLEWVA | VIWYD---GNNKSYADS | RFTISRDISKNTLYLQMNSLRAEDTAVYYCAR | DRDYCSSTSC----------PYYYYYGMDV | WGQGTTVTVS |

| | | | | | | | | | | |
|---|---|---|---|---|---|---|---|---|---|---|
| iPS:392589 | 21-225_27H2 | VH3\|3-33/D4\|4-23\|RF2/JH6 | | QVQLVES-GGGVVQPGRSLRLSCAASG-FTFS | G-----YGMH | WVRQAPGKGLEWVA | VIWYD---GSNKYYADSVKG | RFTISRDNSKNTLFLQMNSLRAEDTAVYYCAR | DRVYCSSTSCS---------PYYYYYGMDV | WGQGTTVTVSS |
| iPS:393166 | 21-225_27G6 | VH3\|3-33/D4\|4-23\|RF2/JH6 | | QVQLVES-GGGVVQPGRSLRLSCAASG-FTFS | G-----YGMH | WVRQAPGKGLEWVA | IIWYD---GSKKYNADSVKG | RFTISRDNSKNTLYLQMNSLRAEDTAVYYCAR | DRVYCSSTSCS---------PYYYYYGMDV | WGQGTTVTVSS |
| iPS:393198 | 21-225_28A11 | VH3\|3-33/D4\|4-23\|RF2/JH6 | | QVQLVES-GGGVVQPGRSLRLSCAASG-FTFS | G-----YGLH | WVRQAPGKGLEWVA | LIWYD---GNNTYYADSVKG | RFTISRDNSKNTLYLQMNSLRAEDTAVYYCAR | DRVYCSSTSCS---------PYYYYYGMDV | WGQGTTVTVSS |
| iPS:393204 | 21-225_8C12 | VH3\|3-33/D4\|4-23\|RF2/JH6 | | QVQLVES-GGGVVQPGRSLRLSCAASG-FTFG | S-----YGMH | WVRQAPGKGLEWVA | LIWYD---GSNKYYADSVKG | RFTISRDNSKNTLYLQMNSLRAEDTAVYYCAR | DRVSCSSSSCY---------PYYYYYGMDV | WGQGTTVTVSS |
| | | Germline | | H_FR1 | H_CDR1 | H_FR2 | H_CDR2 | H_FR3 | H_CDR3 | H_FR4 |
| | VH1\|1-02/D4\|4-11\|RF2/JH4 | | 32220 | QVQLVQS-GAEVKKPGASVKVSCKASG-YIFT | G-----YYMH | WVRQAPGQGLEWMG | WINPN---SGGTNYAQKFQG | RVTMTRDTSISTAYMELSRLRSDDTAVYYCAR | DYSNY--------------------YFDY | WGQGTLVTVSS |
| iPS:453447 | 21-225_65F10 | VH1\|1-02/D4\|4-11\|RF2/JH4 | | QVQLVQS-GAEVKKPGASVKVSCKASG-YTFT | G-----YHMH | WVRQAPGQGLEWMG | WINPN---NGGTSYAQKFQD | RVNMTRDTSISTAYMELSRLRSDDTAVYYCAR | DSRS--------------------SWDY | WGQGTLVTVSS |
| iPS:434145 | 21-225_55B1 | VH1\|1-02/D4\|4-11\|RF2/JH4 | | QVQLVQS-GAEVKKPGASVKVSCKASG-YTFT | G-----YYFH | WVRQAPGQGLEWMG | WIHPN---NNATNYAQKFQG | RVTMTRDTSISTAYMELSRLRSDDTAVYYCAK | DGRS--------------------SFDY | WGQGTLVTVSS |
| iPS:434277 | 21-225_57A7 | VH1\|1-02/D4\|4-11\|RF2/JH4 | | QVQLVQS-GAEVKKPGASVKVSCKASG-YTFT | G-----YHIH | WVRQAPGQDLEWMG | WINPN---NNGTNYAQKFQG | RVTMTRDTSISTAYMELSRLRSDDTAVYYCAR | DGRS--------------------GFDY | WGQGTLVTVSS |
| iPS:434389 | 21-225_66F11 | VH1\|1-02/D4\|4-11\|RF2/JH4 | | QVQLVQS-GAEVKKPGASVKVSCKASG-YTFT | G-----YHMH | WVRQAPGQGLEWMG | WINPN---NGGTHYAQKFQD | WVTMTRDTSISTAYMELSRLRSDDTAVYYCAR | DSRS--------------------SWDY | WGQGTLVSVSS |
| iPS:434423 | 21-225_70D1 | VH1\|1-02/D4\|4-11\|RF2/JH4 | | QVQLVQS-GAEVKKPGASVKVSCKASG-YTFT | G-----YHMH | WVRQAPGQGLEWMG | WINPN---SNATNYAQKFQG | RVTMTRDTSISTAYMELSRLRSDDTAVYYCAR | DSIS--------------------SWDY | WGQGTLVTVSS |
| iPS:437234 | 21-225_64E2 | VH1\|1-02/D4\|4-11\|RF2/JH4 | | QVQLVQS-GAEVKKPGASVKVSCKASG-YTFT | G-----YYMH | WVRQAPGQGLEWMG | WINPN---NNGTNYAQKFQG | RVTMTRDTSISTAYMELSRLRSDDTAVYYCAR | DGSS--------------------GFDY | WGQGTLVTVSS |
| | | Germline | | H_FR1 | H_CDR1 | H_FR2 | H_CDR2 | H_FR3 | H_CDR3 | H_FR4 |
| | VH4\|4-59/D7\|7- | | 32221 | QVQLQES- | S-----YYWS | WIRQPPGK | YIYY---- | RVTISVDTSKNQFSLKLSSV | LIGY------------ | WGQGT |

| | | | | | | | | | | |
|---|---|---|---|---|---|---|---|---|---|---|
| **iPS:4 53449** | **21-225_208A2** | **VH4\|4-59/D7\|7-27\|RF1/JH4** | | QVQLQES-<br>GPGLVKPSETLSL<br>NCTVSG-GSIR | S-----YYWS | WIRQPAGK<br>GLEWIG | RIYT----<br>SGSTDYNPS<br>LKS | RITMSVDTSKNQFSLKLSSV<br>TAADTAVYYCAR | GFGD-----------<br>-----------WDY | WGQGT<br>LVTVS<br>S |
| **iPS:4 35451** | **21-225_152D10** | **VH4\|4-59/D7\|7-27\|RF1/JH4** | | QVQLQES-<br>GPGLVKPSETLSL<br>TCTVSG-GSIS | N-----YYWS | WIRQPAGK<br>GLEWIG | RIDT----<br>SGITNYNPS<br>LKS | RVTMSVDTSKNQFSLKLTSV<br>TAADTAVYYCAR | EGGLGA---------<br>---------TFFDY | WGQGT<br>LVTVS<br>S |
| **iPS:4 35467** | **21-225_153B9** | **VH4\|4-59/D7\|7-27\|RF1/JH4** | | QVQLQES-<br>GPGLVKPSETLSL<br>TCTVSG-GSIS | S-----YYWS | WIRQPAGK<br>GLEWIG | RIDT----<br>SGITNYNPS<br>LKS | RVTMSVDTSKNQFSLKLTSV<br>TAADTAVYYCAR | EGGVGA---------<br>---------TYFDY | WGQGT<br>LVTVS<br>S |
| **iPS:4 35545** | **21-225_158F4** | **VH4\|4-59/D7\|7-27\|RF1/JH4** | | QVQLQES-<br>GPGLVKPSETLSL<br>TCTVSG-GSIS | S-----HFWS | WIRQPAGK<br>GLEWIG | RIYT----<br>SGTTNYTPS<br>LKS | RVTMSVDTSKNQFSLKLSSV<br>TAADTAVYYCAR | LSSG-----------<br>----------WFDY | WGQGT<br>LVTVS<br>S |
| **iPS:4 35665** | **21-225_169F2** | **VH4\|4-59/D7\|7-27\|RF1/JH4** | | QVQLQES-<br>GPGLVKPSETLSL<br>TCTVSG-GSIS | S-----YYWS | WIRQPAGK<br>GLEWIG | RIDT----<br>SGITNYNPS<br>LKS | RVTMSIDTSKSQISLKLSSV<br>TAADTAVYYCAR | EGGVGA---------<br>---------TYFDY | WGQGT<br>LVTVS<br>S |
| **iPS:4 35671** | **21-225_169H5** | **VH4\|4-59/D7\|7-27\|RF1/JH4** | | QVQLQES-<br>GPGLVKPSETLSL<br>TCTVSG-GSIS | S-----YYWS | WIRQPAGK<br>GLEWIG | RIDT----<br>SGITNYNPS<br>LKS | RVTMSVDTSKSQISLKLSSV<br>TAADTAVYYCAR | EGGVGA---------<br>---------TYFDY | WGQGT<br>LVTVS<br>S |
| **iPS:4 36354** | **21-225_210G10** | **VH4\|4-59/D7\|7-27\|RF1/JH4** | | QVQLQES-<br>GPGLVKPSETLSL<br>NCTVSG-GSIR | S-----YYWS | WIRQPAGK<br>GLEWIG | RIYT----<br>SGSTDYNPS<br>LKS | RITMSVDTSKNQFSLKLSSV<br>TAADTAVYYCAR | GFGD-----------<br>-----------WDY | WGQGT<br>LVTVS<br>S |
| | | **Germline** | | **H_FR1** | **H_CDR1** | **H_FR2** | **H_CDR2** | **H_FR3** | **H_CDR3** | **H_FR4** |
| | **VH4\|4-34/D4\|4-17\|RF2/JH6** | | 32222 | QVQLQQW-<br>GAGLLKPSETLSL<br>TCAVYG-GSFS | G-----YYWS | WIRQPPGK<br>GLEWIG | EINH----<br>SGSTNYNPS<br>LKS | RVTISVDTSKNQFSLKLSSV<br>TAADTAVYYCAR | DYGDYYY---------<br>--------YYYGMDV | WGQGT<br>TVTVS<br>S |
| **iPS:4 68810** | **21-225_74D5** | **VH4\|4-34/D4\|4-17\|RF2/JH6** | | QVQLQQW-<br>GAGLLKPSETLSL<br>TCAVNG-GPFS | G-----CYWS | WIRQPPGK<br>GLEWIG | EINY----<br>SGRTNFNPS<br>LKS | RVTISVDTSKNQFSLKLSSV<br>TAADTAVYYCAR | DYGG-----------<br>-----------MDV | WGQGT<br>TVTVS<br>S |
| **iPS:4 68832** | **21-225_76H10** | **VH4\|4-34/D4\|4-17\|RF2/JH6** | | QVQLQQW-<br>GAGLLKPSETLSL<br>TCAVNG-GPFS | G-----CYWS | WIRQPPGK<br>GLEWIG | EINY----<br>SGRTNFNPS<br>LKS | RVTISVDTSKNQFSLKLSSV<br>TAADTAVYYCAR | DYGG-----------<br>-----------MDV | WGQGT<br>TVTVS<br>S |
| **iPS:4 68834** | **21-225_94G10** | **VH4\|4-34/D4\|4-17\|RF2/JH6** | | QVQLQQW-<br>GAGLLKPSETLSL<br>TCAVNG-GPFS | G-----CYWS | GIRQPPGK<br>GREWIG | EINY----<br>SGRTNFNPS<br>LKS | RVTISVDTSKNQFSLKLSSV<br>TAADTAVYYCAR | DYGG-----------<br>-----------MDV | WGQGT<br>TVTVS<br>S |
| **iPS:4 68838** | **21-225_80E12** | **VH4\|4-34/D4\|4-** | | QVQLQQW-<br>GAGLLKPSETLSL | G-----CYWS | WIRQPPGK<br>GLEWIG | EINY----<br>SGRTNFNPS | RVTISVDTSKNQFSLKLSSV<br>TAADTAVYYCAR | DYGG-----------<br>-----------MDV | WGQGT<br>TVTVS |

| | | | | | | | | | | |
|---|---|---|---|---|---|---|---|---|---|---|
| iPS:468820 | 21-225_76E10 | VH4\|4-34/D4\|4-17\|RF2/JH6 | | QVQLQQW-GAGLLKPSETLSLTCAVNG-GPFS | G-----SYWS | WIRQPPGKGLEWIG | EINY----SGRTNFNPSLKS | RVTISVDTSKNQFSLKLSSVTAADTAVYYCAR | DYGG-----------------------MDV | WGQGTTVTVSS |
| iPS:434473 | 21-225_76D1 | VH4\|4-34/D4\|4-17\|RF2/JH6 | | QVQLQQW-GAGLLKPSETLSLTCAVYS-GSFS | G-----CYWS | WIRQPPGKGLEWIG | EINH----SGRTNYNPSLKS | RVTISVDTSKNQFSLKLSSVTAADTAVYYCAR | DYGG-----------------------MDV | WGQGTTVTVSS |
| iPS:434495 | 21-225_74B2 | VH4\|4-34/D4\|4-17\|RF2/JH6 | | QVQLQQW-GAGLLKPSEPLSLTCAVYG-GSFS | G-----PYWS | WIRQPPGKGLEWIG | EINH----SGSTNYNPSLTS | RVTISVDTSKNQFSLKLTSVTAADSAVYYCAR | DYGG-----------------------LDV | WGQGTTVTVSS |
| iPS:434497 | 21-225_76A4 | VH4\|4-34/D4\|4-17\|RF2/JH6 | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| iPS:434501 | 21-225_76G4 | VH4\|4-34/D4\|4-17\|RF2/JH6 | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| iPS:434507 | 21-225_74C5 | VH4\|4-34/D4\|4-17\|RF2/JH6 | | QVQLQQW-GAGLLKPSETLSLTCAVYG-GSFS | G-----CYWS | WIRQPPGKGLEWIG | EINH----SGCTNFNPSLKS | RVTISVDTSKNQFSLKLSSVTAADTAVYYCAR | DYGG-----------------------MDV | WGQGTTVTVSS |
| iPS:434523 | 21-225_75C3 | VH4\|4-34/D4\|4-17\|RF2/JH6 | | QVQLQQG-GAGPLKPSETLSLTCAVYG-GSFS | G-----CYWS | WIRQPPGKGLEWIG | EINY----SGRTNYNPSLKS | RVTISVDTSKNQFSLKLSSVTAADTAVYYCAR | DYGG-----------------------MDV | WGQGTTVTVSS |
| iPS:434533 | 21-225_85F7 | VH4\|4-34/D4\|4-17\|RF2/JH6 | | QVQLQQW-GAGLLKPSEPLSLTCAVYG-GSFS | G-----PYWS | WIRQPPGKGLEWIG | EINH----SGSTNYNPSLKS | RVTISVDTSKNQFSLKLTSVTAADTAVYYCAR | DYGG-----------------------LDV | WGQGTTVTVSS |
| iPS:434547 | 21-225_74H5 | VH4\|4-34/D4\|4-17\|RF2/JH6 | | QVQLQQW-GAGLLKPSETLSLTCAVNG-GPFS | G-----CYWS | WIRQPPGKGLEWIG | EINH----SGRTNFNPSLKS | RVTISVDTSKNQFSLKLSSVTAADTAVYYCAR | DYGG-----------------------MDV | WGQGTTVTVSS |
| iPS:434559 | 21-225_74D11 | VH4\|4-34/D4\|4-17\|RF2/JH6 | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| iPS:434561 | 21-225_77G1 | VH4\|4-34/D4\|4-17\|RF2/JH6 | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| iPS:434565 | 21-225_75B10 | VH4\|4-34/D4\|4- | | QVQLQQW-GAGLLKPSETLSL | G-----YYWS | WIRQPPGKGLEWIG | EINH----SGSTNYNPS | RVTISVDTSKNQFSLKLSSVTAADTAVYYCAR | DYGG-----------------------LDV | WGQGTTVTVS |

| | | | | | | | | | | |
|---|---|---|---|---|---|---|---|---|---|---|
| iPS:434579 | 21-225_77F7 | VH4\|4-34/D4\|4-17\|RF2/JH6 | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| iPS:434581 | 21-225_74B12 | VH4\|4-34/D4\|4-17\|RF2/JH6 | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| iPS:434585 | 21-225_75A12 | VH4\|4-34/D4\|4-17\|RF2/JH6 | | QVQLQQG-GAGPLKPSETLSLTCAVYG-GSFS | G-----CYWS | WIRQPPGKGLEWIG | EINY----SGRTNYNPSLKS | RVTISVDTSKNQFSLKLSSVTAADTAVYYCAR | DYGG-----------------------MDV | WGQGTTVTVSS |
| iPS:434595 | 21-225_77A10 | VH4\|4-34/D4\|4-17\|RF2/JH6 | | QVQLQQG-GAGPLKPSETLSLTCAVYG-GSFS | G-----CYWS | WIRQPPGKGLEWIG | EINY----SGRTNYNPSLKS | RVTISVDTSKNQFSLKLSSVTAADTAVYYCAR | DYGG-----------------------MDV | WGQGTTVTVSS |
| iPS:434611 | 21-225_77C12 | VH4\|4-34/D4\|4-17\|RF2/JH6 | | QVQLQQW-GAGLLKPSETLSLTCAVYG-GAFS | G-----SYWS | WIRQSPGKGLEWIG | EINY----RGSTNYNPSLKS | RVTISVDTSKNQFSLNLSSVTAADTAVYYCAR | DYGG-----------------------MDV | WGQGTTVTVSS |
| iPS:434657 | 21-225_79G1 | VH4\|4-34/D4\|4-17\|RF2/JH6 | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| iPS:434663 | 21-225_79F3 | VH4\|4-34/D4\|4-17\|RF2/JH6 | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| iPS:434687 | 21-225_75A5 | VH4\|4-34/D4\|4-17\|RF2/JH6 | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| iPS:434691 | 21-225_75G7 | VH4\|4-34/D4\|4-17\|RF2/JH6 | | QVQLQQW-GAGLLKPSETLSLTCTVYG-GAFS | G-----SYWS | WIRQSPGKGLEWIG | EINY----RGSTNYNPSLKS | RVTISVDTSKNQFSLNLSSVTAADTAVYYCAR | DYGG-----------------------MDV | WGQGTTVTVSS |
| iPS:434693 | 21-225_79F11 | VH4\|4-34/D4\|4-17\|RF2/JH6 | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| iPS:434699 | 21-225_79G12 | VH4\|4-34/D4\|4-17\|RF2/JH6 | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| iPS:434701 | 21-225_80A1 | VH4\|4-34/D4\|4- | | QVQLQQW-GAGLLKPSETLSL | G-----CYWS | WIRQPPGKGLEWIG | EINH----SGSTNYNPS | RVTISVDTSKNQFSLKLSSVTAADTAVYYCAR | DYGG-----------------------MDV | WGQGTTVTVS |

| | | | | | | | | | | |
|---|---|---|---|---|---|---|---|---|---|---|
| **iPS:434703** | **21-225_80C1** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:434709** | **21-225_80E3** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:434715** | **21-225_80D5** | **VH4\|4-34/D4\|4-17\|RF2/JH6** | | QVQLQQW-GAGLLKPSEPLSLTCAVYG-GSFS | G-----PYWS | WIRQPPGKGLEWIG | EINH----SGSTNYNPSLKS | RVTISVDTSKNQFSLKLTSVTAADMAVYYCAR | DYGG-----------------------LDV | WGQGTTVTVSS |
| **iPS:434725** | **21-225_80H7** | **VH4\|4-34/D4\|4-17\|RF2/JH6** | | QVQLQQW-GAGLLKPSETLSLTCAVYV-GSFS | G-----SYWS | WIRQPPGKGLEWIG | EINQ----SGRTNYNPSLKS | RVTISVDTSKNQFSLKLSSVTAADTAVYYCAR | DYGG-----------------------IDV | WGQGTTVTVSS |
| **iPS:434743** | **21-225_74A4** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:434751** | **21-225_80H12** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:434759** | **21-225_81C5** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:434773** | **21-225_75D9** | **VH4\|4-34/D4\|4-17\|RF2/JH6** | | QVQLQQW-GAGLLKPSETLSLTCAVYG-GSFS | G-----PYWS | WIRQPPGKGLEWIG | EINY----RGSTNYNPSLKS | RVTISVDTSKNQFSLNLSSVTAADTAVYYCAR | DYGG-----------------------MDV | WGQGTTVTVSS |
| **iPS:434777** | **21-225_81C11** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:434809** | **21-225_74F5** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:434821** | **21-225_83G1** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:434839** | **21-225_83B7** | **VH4\|4-34/D4\|4-** | | QVQLQQW-GAGLLKPSETLSL | G-----CYWS | WIRQPPGKGLEWIG | EINH----SGSTNYNPS | RVTISVDTSKNQFSLKLSSVTAADTAVYYCAR | DYGG-----------------------MDV | WGQGTTVTVS |

| | | | | | | | | | | |
|---|---|---|---|---|---|---|---|---|---|---|
| **iPS:4 34869** | **21-225_84E12** | **VH4\|4-34/D4\|4-17\|RF2/J H6** | | QVQLQQW-GAGLLKPSETLSLTCAVYG-GSFS | G-----SYWS | WIRQPPGKGLEWIG | EINQ----SGRTNYNPSLKS | RVTISVDTSKNQFSLKLSSVTAADTAVYYCAR | DYGG-----------------------IDV | WGQGTTVTVSS |
| **iPS:4 34879** | **21-225_85A3** | **VH4\|4-34/D4\|4-17\|RF2/J H6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:4 34881** | **21-225_85B4** | **VH4\|4-34/D4\|4-17\|RF2/J H6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:4 34887** | **21-225_85D6** | **VH4\|4-34/D4\|4-17\|RF2/J H6** | | QVQLQQG-GAGPLKPSETLSLTCAVYG-GSFS | G-----CYWS | WIRQPPGKGLEWIG | EINY----SGRTNYNPSLKS | RVTISVDTSKNQFSLKLSSVTAADTAVYYCAR | DYGG-----------------------MDV | WGQGTTVTVSS |
| **iPS:4 34895** | **21-225_74H7** | **VH4\|4-34/D4\|4-17\|RF2/J H6** | | QVQLQQW-GAGLLKPSEPLSLTCAVYG-GSFS | G-----PYWS | WIRQPPGKGLEWIG | EINH----SGSTNYNPSLKS | RVTISVDTSKNQFSLKLTSVTAADTAVYYCAR | DYGG-----------------------LDV | WGQGTTVTVSS |
| **iPS:4 34899** | **21-225_85B9** | **VH4\|4-34/D4\|4-17\|RF2/J H6** | | QVQLQQW-GAGLLKPSETLSLTCAVNG-GPFS | G-----CYWS | WIRQPPGKGLEWIG | EINH----SGRTNFNPSLKS | RVTISVDTSKNQFSLKLSSVTAADTAVYYCAR | DYGG-----------------------MDV | WGQGTTVTVSS |
| **iPS:4 34907** | **21-225_85G10** | **VH4\|4-34/D4\|4-17\|RF2/J H6** | | QVQLQQR-GAGLLKPSETLSLTCAVYG-GSFS | G-----CYWS | WIRQPPGKGLEWIG | EINH----SGITNYNPSLKS | RVTISVDTSKNQFSLKLTSVTAADTAVYYCAR | DYGG-----------------------LDV | WGQGTTVTVSS |
| **iPS:4 34913** | **21-225_86C1** | **VH4\|4-34/D4\|4-17\|RF2/J H6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:4 34921** | **21-225_86E4** | **VH4\|4-34/D4\|4-17\|RF2/J H6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:4 34939** | **21-225_86C11** | **VH4\|4-34/D4\|4-17\|RF2/J H6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:4 34943** | **21-225_87H1** | **VH4\|4-34/D4\|4-17\|RF2/J H6** | | QVQLQPW-GAGLLKPSETLSLTCAVYG-GSFS | G-----YYWS | WIRQPPGKGLEWIG | EINH----SGRTNYNPSLKS | RVTISVDTSKDQFSLKLSSVTAADTAVYYCAR | DYGG-----------------------LDV | WGQGTTVTVSS |
| **iPS:4 34945** | **21-225_87E5** | **VH4\|4-34/D4\|4-** | | QVQLQQW-GAGLLKPSETLSL | G-----CYWS | WIRQPPGKGLEWIG | EINH----SGSTNYNPS | RVTISVDTSKNQFSLKLSSVTAADTAVYYCAR | DYGG-----------------------MDV | WGQGTTVTVS |

| | | | | | | | | | | |
|---|---|---|---|---|---|---|---|---|---|---|
| **iPS:434955** | **21-225_87C9** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG----------------------MDV | WGQGTTVTVSS |
| **iPS:434961** | **21-225_87A12** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG----------------------MDV | WGQGTTVTVSS |
| **iPS:434969** | **21-225_88H1** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG----------------------MDV | WGQGTTVTVSS |
| **iPS:434981** | **21-225_88E7** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG----------------------MDV | WGQGTTVTVSS |
| **iPS:434983** | **21-225_88F7** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG----------------------MDV | WGQGTTVTVSS |
| **iPS:434995** | **21-225_88G9** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG----------------------MDV | WGQGTTVTVSS |
| **iPS:434999** | **21-225_75A8** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG----------------------MDV | WGQGTTVTVSS |
| **iPS:435013** | **21-225_89D5** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG----------------------MDV | WGQGTTVTVSS |
| **iPS:435015** | **21-225_89H5** | **VH4\|4-34/D4\|4-17\|RF2/JH6** | | QVQLQQW-GAGLLKPSETLSLTCAVYG-GSFS | G-----CYWS | WIRQPPGKGLEWIG | EINY----SGSTNFNPSLKS | RVTISADTSKNQFSLKLSSVTAADTAVYYCAR | DYGG----------------------MDV | WGQGTTVTVSS |
| **iPS:435025** | **21-225_89E10** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG----------------------MDV | WGQGTTVTVSS |
| **iPS:435029** | **21-225_89A11** | **VH4\|4-34/D4\|4-17\|RF2/JH6** | | QVQLQPW-GAGLLKPSETLSLTCAVYG-GSFS | G-----YYWS | WIRQPPGKGLEWIG | EINH----SGRTSYNPSLKS | RVTISVDTSKNQFSLKLSSVTAADTAVYYCAR | DYGG----------------------LDV | WGQGTTVTVSS |
| **iPS:435039** | **21-225_90G4** | **VH4\|4-34/D4\|4-** | | QVQLQQW-GAGLLKPSETLSL | G-----CYWS | WIRQPPGKGLEWIG | EINH----SGSTNYNPS | RVTISVDTSKNQFSLKLSSVTAADTAVYYCAR | DYGG----------------------MDV | WGQGTTVTVS |

| | | | | | | | | | |
|---|---|---|---|---|---|---|---|---|---|
| **iPS:4 35041** | **21-225_90A5** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG----------------------MDV | WGQGTTVTVSS |
| **iPS:4 35043** | **21-225_90G5** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG----------------------MDV | WGQGTTVTVSS |
| **iPS:4 35055** | **21-225_90F10** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG----------------------MDV | WGQGTTVTVSS |
| **iPS:4 35073** | **21-225_91B2** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG----------------------MDV | WGQGTTVTVSS |
| **iPS:4 35075** | **21-225_91B3** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG----------------------MDV | WGQGTTVTVSS |
| **iPS:4 35077** | **21-225_91F3** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG----------------------MDV | WGQGTTVTVSS |
| **iPS:4 35079** | **21-225_91B4** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG----------------------MDV | WGQGTTVTVSS |
| **iPS:4 35089** | **21-225_91E9** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG----------------------MDV | WGQGTTVTVSS |
| **iPS:4 35097** | **21-225_92B1** | **VH4\|4-34/D4\|4-17\|RF2/JH6** | | QVQLQQW-GAGLLKPSETLSLTCAVYG-GSFS | G-----SYWS | WIRQPPGKGLEWIG | EINY----RGSTNYNPSLKS | RVAISVDTSKNQFSLNLTSVTAADTAVYYCAR | DYGG----------------------LDV | WGQGTTVTVSS |
| **iPS:4 35111** | **21-225_92D6** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG----------------------MDV | WGQGTTVTVSS |
| **iPS:4 35115** | **21-225_77C5** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG----------------------MDV | WGQGTTVTVSS |
| **iPS:4 35171** | **21-225_93C2** | **VH4\|4-34/D4\|4-** | | QVQLQQW-GAGLLKPSETLSL | G-----CYWS | WIRQPPGKGLEWIG | EINH----SGSTNYNPS | RVTISVDTSKNQFSLKLSSVTAADTAVYYCAR | DYGG----------------------MDV | WGQGTTVTVS |

| | | | | | | | | | | |
|---|---|---|---|---|---|---|---|---|---|---|
| **iPS:435177** | **21-225_93E4** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:435195** | **21-225_94D3** | **VH4\|4-34/D4\|4-17\|RF2/JH6** | | QVQLQQG-GAGPLKPSETLSLTCAVYG-GSFS | G-----CYWS | WIRQPPGKGLEWIG | EINY----SGRTNYNPSLKS | RVTISVDTSKNQFSLKLSSVTAADTAVYYCAR | DYGG-----------------------MDV | WGQGTTVTVSS |
| **iPS:435217** | **21-225_94F12** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:435219** | **21-225_95D2** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:435235** | **21-225_95F9** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:435237** | **21-225_95G9** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:435239** | **21-225_95H10** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:435273** | **21-225_97A2** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:435281** | **21-225_97E5** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:437324** | **21-225_75C2** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:437328** | **21-225_75D3** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:437332** | **21-225_75F3** | **VH4\|4-34/D4\|4-** | | QVQLQQW-GAGLLKPSETLSL | G-----CYWS | WIRQPPGKGLEWIG | EINH----SGSTNYNPS | RVTISVDTSKNQFSLKLSSVTAADTAVYYCAR | DYGG-----------------------MDV | WGQGTTVTVS |

| | | | | | | | | | | |
|---|---|---|---|---|---|---|---|---|---|---|
| **iPS:437344** | **21-225_75G12** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:437350** | **21-225_74A3** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| **iPS:437369** | **21-225_74D6** | **VH4\|4-34/D4\|4-17\|RF2/JH6** | | QVQLQQW-GAGLLKPSETLSLTCAVHG-GSFS | G-----CYWS | WIRQPPGKGLEWIG | EINH----SGSTNYNPSLKS | RVTISVDTSKNQFSLKLSSVTAADTAVYYCAR | DYGG-----------------------MDV | WGQGTTVTVSS |
| | | **Germline** | | **H_FR1** | **H_CDR1** | **H_FR2** | **H_CDR2** | **H_FR3** | **H_CDR3** | **H_FR4** |
| | **VH3\|3-33/D4\|4-17\|RF2/JH6** | | 32223 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DYGDYYY-----------YYYGMDV | WGQGTTVTVSS |
| **iPS:468814** | **21-225_223D11** | **VH3\|3-33/D4\|4-17\|RF2/JH6** | | QVQLVES-GGGVVQPGRSLRLSCAASG-FTFS | N-----YGMD | WVRQAPGKGLEWVA | VIWYD---GSNDYYADSVKG | RFTISRDNSKNTLFLQMNSLRAEDTAVYYCAR | DRGIGY-------------------NDMDV | WGQGTTVTVSS |
| **iPS:434621** | **21-225_74D1** | **VH3\|3-33/D4\|4-17\|RF2/JH6** | | QVQLVES-GGGVVQPGRSLRLSCAASG-FTFS | S-----YGMH | WVRQAPGKGLEWVA | VIWYD---GSNKYHADSVKG | RFTISRDNSKNTLYLQMNSLRAEDTAMYYCAR | DEGFGEFD--------------YYNYGMDV | WGQGTTVTVSS |
| **iPS:434947** | **21-225_87B7** | **VH3\|3-33/D4\|4-17\|RF2/JH6** | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DFGVGY-------------------YGMDV | WGQGTTVTVSS |
| **iPS:435819** | **21-225_190C11** | **VH3\|3-33/D4\|4-17\|RF2/JH6** | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DQGVGY-------------------DGLDV | WGQGTTVTVSS |
| **iPS:435825** | **21-225_190G11** | **VH3\|3-33/D4\|4-17\|RF2/JH6** | | QVQLVES-GGGVVQPGRSLRLSCAASG-FTFS | I-----YGMH | WVRQAPGKGLEWVA | VIWYD---GSNKNYADSVKG | RFTISRDNSKNTLYLQMNSLRAEDTAVYYCAR | DQGVGY-------------------DGLDV | WGQGTTVTVSS |
| **iPS:435837** | **21-225_198G3** | **VH3\|3-33/D4\|4-17\|RF2/JH6** | | QVQLVES-GGGVVQPGRSLKLSCAASG-FTFS | T-----YGMH | WVRQAPGKGLEWVA | VIWYD---GTNKNYADSVKG | RFTISRDNSKNTLCLQMNSLRAEDTAVYYCAR | DQGVGY-------------------DGLDV | WGQGTTVTVSS |
| **iPS:435845** | **21-225_191G1** | **VH3\|3-33/D4\|4-17\|RF2/JH6** | | QVQLVES-GGGVVQPGRSLRLSCAASG-FTFS | S-----YGMH | WVRQAPGKGLEWVA | VIWYD---GSNEHYADSVKG | RFTISRDNSKNTLYLQMNSLRAEDTAVYYCAR | DRGVGY-------------------YGLDV | WGQGTTVTVSS |
| **iPS:435859** | **21-225_190E6** | **VH3\|3-33/D4\|4-** | | QVQLVES-GGGVVQPGRSLRL | S-----YGMH | WVRQAPGKGLEWVA | VIWYD---GSNKNYADS | RFTISRDNSKNTLYLQMNSLRAEDTAVYYCAR | DQGVGY-------------------DGLDV | WGQGTTVTVS |

| | | | | | | | | | |
|---|---|---|---|---|---|---|---|---|---|
| iPS:4 35873 | 21-225_190G4 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAMYYCAR | DQGVGY-------------------DGLDV | WGQGTSVTVSS |
| iPS:4 35933 | 21-225_190F8 | VH3\|3-33/D4\|4-17\|RF2/JH6 | | QVQLVES-GGGVVQPGRSLRLSCAASG-FTFS | T-----YGMH | WVRQAPGKGLEWVA | VIWYD---GSNKNYADSVKG | RFTISRDNSKNTLYLQMNSLRAEDTAVYYCAR | DQGVGY-------------------DGLDV | WGQGTTVTVSS |
| iPS:4 35941 | 21-225_191E8 | VH3\|3-33/D4\|4-17\|RF2/JH6 | | QVQLVES-GGGVVQPGRSLRLSCAASG-FTFS | N-----YGMH | WVRQAPGKGLEWVA | IIWFD---GSNQYYADSVKG | RFTISRDNSKNTLYLQLNSLRAEDTAVYYCAR | AHGVYY-------------------YAMDV | WGQGTTVTVSS |
| iPS:4 35945 | 21-225_191A10 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAMYYCAR | DQGVGY-------------------DGLDV | WGQGTSVTVSS |
| iPS:4 35947 | 21-225_191E10 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAMYYCAR | DQGVGY-------------------DGLDV | WGQGTSVTVSS |
| iPS:4 35957 | 21-225_191G12 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DQGVGY-------------------DGLDV | WGQGTTVTVSS |
| iPS:4 35963 | 21-225_192D2 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNMLYLQMNSLRAEETAVYYCAR | DRGVGY-------------------YGMDV | WGQGTTVTVSS |
| iPS:4 35971 | 21-225_192D3 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNEHYADSVKG | RFTISRDNSKNTLYLQMNSLRAEDTAVYYCAR | DRGVGY-------------------YGLDV | WGQGTTVTVSS |
| iPS:4 35979 | 21-225_192H4 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DQGVGY-------------------YGMDV | WGQGTTVTVSS |
| iPS:4 35987 | 21-225_192G6 | VH3\|3-33/D4\|4-17\|RF2/JH6 | | QVQLVES-GGGVVQPGRSLRLSCAASG-FTFS | S-----YGMH | WVRQAPGKGLEWVA | VLWYD---GTNKNYADSVKG | RFTISRDNSKNTLYLQMNSLRAEDTAVYYCAR | DQGVGY-------------------DGLDV | WGQGTTVTVSS |
| iPS:4 35993 | 21-225_192C8 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNEHYADSVKG | RFMISRDNSKNTLYLQMNSLRAEDTAVYYCAR | DRGVGY-------------------YGMDV | WGQGTTVTVSS |
| iPS:4 35997 | 21-225_192G8 | VH3\|3-33/D4\|4- | | QVQLVES-GGGVVQPGRSLRL | S-----YGMH | WVRQAPGKGLEWVA | VIWYD---GSNKNYADS | RFTISRDNSKNTLYLQMNSLRAEDTAVYYCAR | DQGVGY-------------------DGLDV | WGQGTTVTVS |

| | | | | | | | | | | |
|---|---|---|---|---|---|---|---|---|---|---|
| **iPS:4 36005** | **21-225_192H10** | **VH3\|3-33/D4\|4-17\|RF2/JH6** | | QVQLVES- GGGVVQPGRSLRL SCAASG-FTFS | S-----YGMH | WVRQAPGK GLEWVA | VIWYD--- GSNKNYADS VKG | RFTISRDNSKNTLYLQMNSL RAEDTAMYYCAR | DQGVGY---------- ---------DGLDV | WGQGT SVTVS S |
| **iPS:4 36031** | **21-225_193C7** | **VH3\|3-33/D4\|4-17\|RF2/JH6** | | QVQLVES- GGGVVQPGRSLRL SCAASG-FTFS | S-----YGMH | WVRQAPGK GLEWVA | VIWYD--- GSNKNYADS VKG | RFTISRDNSKNTLYLQMNSL RAEDTAMYYCAR | DQGVGY---------- ---------DGLDV | WGQGT SVTVS S |
| **iPS:4 36045** | **21-225_193A10** | **VH3\|3-33/D4\|4-17\|RF2/JH6** | | QVQLVES- GGGVVQPGRSLRL SCAASG-FTFS | S-----YGMH | WVRQAPGK GLEWVA | VIWYD--- GSNEHYADS VKG | RFTISRDNSKNTLYLQMNSL RAEDTAVYYCAR | DRGVGY---------- ---------YGLDV | WGQGT TVTVS S |
| **iPS:4 36076** | **21-225_194H11** | **VH3\|3-33/D4\|4-17\|RF2/JH6** | | QVQLVES- GGGVVQPGRSLRL SCAASG-FTFS | S-----YGMH | WVRQAPGK GLEWVA | VIWYD--- GSNEHYADS VKG | RFTISRDNSKNTLSLQMNSL RAEDTAVYYCAR | DRGVGY---------- ---------YGLDV | WGQGT TVTVS S |
| **iPS:4 36086** | **21-225_191G10** | **VH3\|3-33/D4\|4-17\|RF2/JH6** | | QVQLVES- GGGVVQPGRSLRL SCAASG-FTFS | S-----YGMH | WVRQAPGK GLEWVA | VIWYD--- GSNKNYADS VKG | RFTISRDNSKNTLYLQMNSL RAEDTAVYYCAR | DQGVGY---------- ---------DGLDV | WGQGT TVTVS S |
| **iPS:4 36090** | **21-225_195A9** | **VH3\|3-33/D4\|4-17\|RF2/JH6** | | QVQLVES- GGGVVQPGRSLRL SCAASG-FTFS | S-----YGMH | WVRQAPGK GLQWVA | VIWYD--- GSNEHYADS VKG | RFTISRDNSKNTLYLQMNSL RAEDTAVYYCAR | DRGVGY---------- ---------YGLDV | WGQGT TVTVS S |
| **iPS:4 36112** | **21-225_196C7** | **VH3\|3-33/D4\|4-17\|RF2/JH6** | | QVQLVES- GGGVVQPGRSLRL SCAASG-FTFS | S-----YGMH | WVRQAPGK GLEWVA | VIWYD--- GSNEHYADS VKG | RFTISRDNSKNTLYLQMNSL RAEDTAVYYCAR | DRGVGY---------- ---------YGLDV | WGQGT TVTVS S |
| **iPS:4 36138** | **21-225_197F2** | **VH3\|3-33/D4\|4-17\|RF2/JH6** | | QVQLVES- GGGVVQPGRSLRL SCAASG-FTFS | S-----YGMH | WVRQAPGK GLEWVA | VIWYD--- GSNKNYADS VKG | RFTISRDNSKNTLYLQMNSL RAEDTAVYYCAR | DQGVGY---------- ---------DGLDV | WGQGT TVTVS S |
| **iPS:4 36152** | **21-225_197B6** | **VH3\|3-33/D4\|4-17\|RF2/JH6** | | QVQLVES- GGGVVQPGRSLRL SCAASG-FTFS | S-----YGMH | WVRQAPGK GLEWVA | VIWYD--- GSNKNYADS VKG | RFTISRDNSKNMLYLQMNSL RAEDTAMYYCAR | DQGVGY---------- ---------DGLDV | WGQGT SVTVS S |
| **iPS:4 36173** | **21-225_197G12** | **VH3\|3-33/D4\|4-17\|RF2/JH6** | | QVQLVES- GGGVVQPGRSLRL SCAASG-FTFS | S-----YGMH | WVRQAPGK GLEWVA | VIWYD--- GSNKNYADS VKG | RFTISRDNSKNTLYLQMNSL RAEDTAVYYCAR | DQGVGY---------- ---------DGLDV | WGQGT TVTVS S |
| **iPS:4 36189** | **21-225_198B6** | **VH3\|3-33/D4\|4-17\|RF2/JH6** | | QVQLVES- GGGVVQPGRSLRL SCAASG-FTFS | S-----YGMH | WVRQAPGK GLEWVA | VIWYD--- GSNKHYADS VKG | RFTISRDNSKNTLYLQMNSL RAEDTAVYYCAR | DQGVGY---------- ---------YGMDV | WGQGT TVTVS S |
| **iPS:4 36201** | **21-225_199C5** | **VH3\|3-33/D4\|4-** | | QVQLVES- GGGVVQPGRSLRL | S-----YGMH | WVRQAPGK GLEWVA | VIWYD--- GSNKNYADS | RFTISRDNSKNTLYLQMNSL RAEDTAVYYCAR | DQGVGY---------- ---------YGMDV | WGQGT TVTVS |

| | | | | | | | | | |
|---|---|---|---|---|---|---|---|---|---|
| iPS:436203 | 21-225_199A6 | VH3\|3-33/D4\|4-17\|RF2/JH6 | | QVQLVES-GGGVVQPGRSLRLSCAASG-FTFS | N-----YGMH | WVRQAPGKGLEWVA | IIWFD---GSNQYYADSVKG | RFTISRDNSKNTLYLQLNSLRAEDTAVYYCAR | AHGVYY-------------------YAMDV | WGQGTTVTVSS |
| iPS:436282 | 21-225_204G6 | VH3\|3-33/D4\|4-17\|RF2/JH6 | | QVQLVES-GGGVVQPGRSLRLSCTASG-FTFS | S-----YGMH | WVRQAPGKGLEWVA | VIWYD---GSNKNYADSVKG | RFTISRDNSKNTLYLQMNSLRAEDTAVYYCAR | DRGVGY-------------------DGMDV | WGQGTTVTVSS |
| iPS:436296 | 21-225_205F5 | VH3\|3-33/D4\|4-17\|RF2/JH6 | | QVQLVES-GGGVVQPGRSLRLSCAASG-FTFS | R-----YGMH | WVRQAPGKGLEWVA | VIWYD---GSNENYVDSVKG | RFTISRDTSKKMLFLQMNSLRTDDTAVYYCAR | DMGIGY-------------------YGMDV | WGQGTTVTVSS |
| iPS:436324 | 21-225_207G6 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNKNYAESVKG | RFTISRDNSKNTLYLQMNSLRAEDTAVYYCAR | DAGIGY-------------------YGIDV | WGQGTTVTVSS |
| iPS:436364 | 21-225_211A11 | VH3\|3-33/D4\|4-17\|RF2/JH6 | | QVQLVGS-GGGVVQPGRSLRLSCAASG-FTFS | S-----YGMH | WVRQAPGKGLEWVA | VLWFD---GSNRNYADSVKG | RFTISRDNSKNTLYLQMNSLRAEDTAVYYCAR | DRGVGY-------------------YGTDV | WGQGTTVTVSS |
| iPS:436372 | 21-225_211A8 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNEHYADSVKG | RFTISRDNSKKTLYLQMNSLRAEDTAVYYCAR | DHGVGY-------------------YGMDV | WGQGTTVTVSS |
| iPS:436376 | 21-225_212E6 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNKNYVDSVKG | RFTISRDNSKNTLYLQMNSLRAEDTAVYYCAR | DYGVGY-------------------YGTDV | WGQGTTVTVSS |
| iPS:436378 | 21-225_212D7 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLSLQMNSLRAEDTAVYYCAR | DYGVGY-------------------YGTDV | WGQGTTVTVSS |
| iPS:436380 | 21-225_212H9 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNEHYADSVKG | RFTISRDNSKKTLYLQMNSLRAEDTAVYYCAR | DHGVGY-------------------YGMDV | WGQGTTVTVSS |
| iPS:436384 | 21-225_212F10 | VH3\|3-33/D4\|4-17\|RF2/JH6 | | QVQLVES-GGGVVQPGRSLRLSCAASG-FTFS | R-----YGMH | WVRQAPGKGLEWVA | VIWYD---GSNKHYADSVKG | RFTISRDNSKNTLYLQMNSLRGEDTAVYYCAR | DRGVGY-------------------NGMDV | WGQGTTVTVSS |
| iPS:436390 | 21-225_213D2 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLSLQMNSLRAEDTAVYYCAR | DYGVGY-------------------YGTDV | WGQGTTVTVSS |
| iPS:436394 | 21-225_213C4 | VH3\|3-33/D4\|4- | | QVQLVES-GGGVVQPGRSLRL | S-----YGMH | WVRQAPGKGLEWVA | VIWYD---GSNKHYADS | RFTISRDNSKNTLYLQMNSLRAEDTAVYYCAR | DYGVGY-------------------DGMDV | WGQGTTVTVS |

| | | | | | | | | | | |
|---|---|---|---|---|---|---|---|---|---|---|
| iPS:436398 | 21-225_213B8 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNKHYADSVKG | RFTISRDNSKNTLYLQMNSLRAEDTAVYYCAR | DYGVGY-------------------YGTDV | WGQGTTVTVSS |
| iPS:436404 | 21-225_214C3 | VH3\|3-33/D4\|4-17\|RF2/JH6 | | QVQLVES-GGGVVQPGRSLRLSCEASG-FTFS | S-----YGMH | WVRQAPGKGLEWVA | VIWYD---GSNKNYGDSVKG | RFTISRDNSKNTLYLQMNSLRAEDTAVYYCAR | DRGVGY-------------------DGMDV | WGQGTTVTVSS |
| iPS:436410 | 21-225_212E10 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLSLQMNSLRAEDTAVYYCAR | DYGVGY-------------------YGTDV | WGQGTTVTVSS |
| iPS:436420 | 21-225_215B5 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLSLQMNSLRAEDTAVYYCAR | DYGVGY-------------------YGTDV | WGQGTTVTVSS |
| iPS:436422 | 21-225_215D6 | VH3\|3-33/D4\|4-17\|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DCGVGY-------------------YGTDV | WGQGTTVTVSS |
| iPS:436430 | 21-225_215A12 | VH3\|3-33/D4\|4-17\|RF2/JH6 | | QVQLVES-GGGVVQPGRSLRLSCAASG-LTFS | S-----YGMH | WVRQAPGKGLEWVA | VIWYD---GSNEHYADSVKG | RFTISRDNSKNTLYLQMNSLRAEDTAVYYCAR | DRGVGY-------------------YGMDV | WGQGTTVTVSS |
| iPS:436452 | 21-225_217G5 | VH3\|3-33/D4\|4-17\|RF2/JH6 | | QVQLVES-GGGVVQPGRSLRLSCAASG-FTFS | S-----NGMH | WVRQAPGKGLEWVA | VIWYD---GSNKNYADSVKG | RFTISRDNSKNTLYLQMNSLRAEDTAVYYCAR | DYGVGY-------------------YGLDV | WGQGTTVTVSS |
| iPS:436464 | 21-225_219H1 | VH3\|3-33/D4\|4-17\|RF2/JH6 | | QVQLVES-GGGVVQPGRSLRLSCAASG-FTFS | R-----YGMH | WVRQAPGKGLEWVA | VIWYD---GSNKHYADSVKG | RFTISRDNSKNTLYLQMNSLRGEDTAVYYCAR | DRGVGY-------------------NGMDV | WGQGTTVTVSS |
| iPS:451120 | 21-225_197D3 | VH3\|3-33/D4\|4-17\|RF2/JH6 | | QVQLVES-GGGVVQPGRSLRLSCAASG-FIFS | S-----HGMH | WVRQAPGKGLEWVA | VIWYD---GSNEHYADSVKG | RFTISRDNSKNTLYLQMNSLRAEDTAVYYCAR | DQGVGY-------------------YGMDV | WGQGTTVTVSS |
| | | Germline | | H_FR1 | H_CDR1 | H_FR2 | H_CDR2 | H_FR3 | H_CDR3 | H_FR4 |
| | VH3\|3-33/D4\|4-17\|RF2/JH4 | | 32224 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DYGDY---------------------YFDY | WGQGTLVTVSS |
| iPS:468822 | 21-225_147E10 | VH3\|3-33/D4\|4-17\|RF2/JH4 | | QVQLVES-GGGVVQPGKSLRLSCAASG-FTFS | N-----YGLH | WVRQAPGKGLEWVA | IIWYD---GSNKYYADSVKG | RFTISRDNSKNTLYLQMNSLRAEDTAVYYCAR | DHYDFW------------------SGHFDY | WGQGTLVTVSS |
| iPS:433965 | 21-225_46F2 | VH3\|3-33/D4\|4- | | QVQLVES-GGGVVQPGRSLRL | S-----YGMH | WVRQAPGKGLEWVA | IIWYD---GSNKYYVDS | RFTISRDNSKNTLYLQMNSLRAEDTAVYYCAR | DRYDFW------------------SGYFDY | WGQGTLVTVS |

| | | | | | | | | | | |
|---|---|---|---|---|---|---|---|---|---|---|
| **iPS:4<br>34255** | **21-225_62E6** | **VH3\|3-<br>33/D4\|4-<br>17\|RF2/J<br>H4** | | QVQLVES-<br>GGGVVQPGRSLRL<br>SCAASG-FTFS | S-----YGMH | WVRQAPGK<br>GLEWVA | AIWYD---<br>GSNKYYGDS<br>VKG | RVTISRDNSKNSLHLQMNSL<br>RAEDTAVYYCAR | DQGIVG----------<br>--------ATWFDY | WGQGT<br>LVTVS<br>S |
| **iPS:4<br>34269** | **21-225_57H3** | **VH3\|3-<br>33/D4\|4-<br>17\|RF2/J<br>H4** | | QVQLVES-<br>GGGVVQPGRSLRL<br>SCAASG-FTFS | S-----YGMH | WVRQAPGK<br>GLEWVA | AIWYD---<br>GSNKYYADS<br>VKG | RFTISRDNSKNTLYLQMNSL<br>RAEDTAVYYCAR | DYGIVG----------<br>--------ATYFDY | WGQGT<br>LVTVS<br>S |
| **iPS:4<br>34345** | **21-225_64H9** | **VH3\|3-<br>33/D4\|4-<br>17\|RF2/J<br>H4** | | QVQLVES-<br>GGGVVQPGRSLRL<br>SCAASG-FTFS | T-----YGMH | WVRQAPGK<br>GLEWVA | IIWYD---<br>GSNKYYADS<br>VKG | RFTISRDNSKNTLYLQMNSL<br>RAEDTAVYYCAR | DTYDFW----------<br>--------SGYLGY | WGQGT<br>LVTVS<br>S |
| **iPS:4<br>34363** | **21-225_65A6** | **VH3\|3-<br>33/D4\|4-<br>17\|RF2/J<br>H4** | | QVQLVES-<br>GGGVVQPGRSLRL<br>SCAASG-FTFS | N-----YGMH | WVRQAPGK<br>GLEWVA | VIWYD---<br>GSNKYYGDS<br>VKG | RFTISRDNSKNTLYLQMNSL<br>RAEDTAVYYCAR | DQGIVG----------<br>--------ATWFDY | WGQGT<br>LVTVS<br>S |
| **iPS:4<br>34393** | **21-225_67C3** | **VH3\|3-<br>33/D4\|4-<br>17\|RF2/J<br>H4** | | QVQLVES-<br>GGGVVQPGRSLRL<br>SCAASG-FTFS | S-----YGMH | WVRQAPGK<br>GLEWVA | AIWYD---<br>GSNKYYGDS<br>VKG | RVTISRDNSKNSLHLQMNSL<br>RAEDTAVYYCAR | DQGIVG----------<br>--------ATWFDY | WGQGT<br>LVTVS<br>S |
| **iPS:4<br>34425** | **21-225_70A5** | **VH3\|3-<br>33/D4\|4-<br>17\|RF2/J<br>H4** | | QVQLVES-<br>GGGVVQPGRSLRL<br>SCAASG-FTFS | S-----YGMH | WVRQAPGK<br>GLEWVA | VIWYD---<br>GSNKYYADS<br>VKG | RFTISRDNSKNSLYLQMNSL<br>SAEDTAVYYCAR | DQGIVG----------<br>--------ATWFDY | WGQGT<br>LVTVS<br>S |
| **iPS:4<br>35341** | **21-225_148B2** | **VH3\|3-<br>33/D4\|4-<br>17\|RF2/J<br>H4** | | QVQLVES-<br>GGGVVQPGRSLRL<br>SCAASG-FTFS | S-----YGMH | WVRQAPGK<br>GLEWVA | IIWYD---<br>GSYKYYADS<br>VKG | RFTISRDNSKNTLYLQMNSL<br>SAEDTAVYYCAR | DHFDFW----------<br>--------SGHFDY | WGQGT<br>LVTVS<br>S |
| **iPS:4<br>35357** | **21-<br>225_148G10** | **VH3\|3-<br>33/D4\|4-<br>17\|RF2/J<br>H4** | | QVQLVES-<br>GGGVVQPGRSLRL<br>SCAASG-FTFS | S-----YGMH | WVRQAPGK<br>GLEWVA | IIWYD---<br>GSNKYYADS<br>VKG | RFTISRDNSKNTLYLQMNSL<br>RAEDTAVYYCAR | DRYDFW----------<br>--------SGHFDY | WGQGT<br>LVTVS<br>S |
| **iPS:4<br>35365** | **21-225_149F1** | **VH3\|3-<br>33/D4\|4-<br>17\|RF2/J<br>H4** | | QVQLVES-<br>GGGVVQPGRSLRL<br>SCAASG-FTFS | S-----YGMH | WVRQAPGK<br>GLEWVA | IIWYD---<br>GSYKYYADS<br>VKG | RFTISRDNSKNTLYLQMNSL<br>SAEDTAVYYCAR | DHFDFW----------<br>--------SGHFDY | WGQGT<br>LVTVS<br>S |
| **iPS:4<br>35413** | **21-<br>225_150B11** | **VH3\|3-<br>33/D4\|4-<br>17\|RF2/J<br>H4** | | QVQLVES-<br>GGGVVQPGRSLRL<br>SCAASG-FTFS | S-----YGMH | WVRQAPGK<br>GLEWVA | IIWYD---<br>GSNKYYADS<br>VKG | RFTISRDNSKNTLYLQMNSL<br>RAEDTAVYYCAR | DRYDFW----------<br>--------SGHFDY | WGQGT<br>LVTVS<br>S |
| **iPS:4<br>35423** | **21-225_151G5** | **VH3\|3-<br>33/D4\|4-<br>17\|RF2/J<br>H4** | | QVQLMES-<br>GGGVVQPGRSLRL<br>SCAASG-FIFS | S-----YGMH | WVRQAPGK<br>GLEWVA | IIWYD---<br>GSNKYYADS<br>VKG | RFTISRDNSKNTLYLQMNSL<br>RAEDTAVYYCAR | DRYDFW----------<br>--------SGHFDY | WGQGT<br>LVTVS<br>S |
| **iPS:4<br>35429** | **21-<br>225_151A10** | **VH3\|3-<br>33/D4\|4-** | | QVQLVES-<br>GGGVVQPGRSLRL | N-----YGMH | WVRQAPGK<br>GLEWLA | IIWYD---<br>GSNKYYADS | RFTISRDNSKNTLYLQMNSL<br>RAEDTAVYYCAR | DHYDFW----------<br>--------SGHFDY | WGQGT<br>LVTVS |

| | | | | | | | | | | |
|---|---|---|---|---|---|---|---|---|---|---|
| **iPS:4<br>35489** | **21-225_155A5** | **VH3\|3-<br>33/D4\|4-<br>17\|RF2/J<br>H4** | | QVQLVES-<br>GGDVVQPGRSLRL<br>SCAASG-FTFS | S-----YGMH | WVRQAPGK<br>GLEWVA | IIWYD---<br>GSSKYYADS<br>VKG | RFTISRDNSKNTLYLHMNSL<br>RAEDTAVYYCAR | DRYDFW----------<br>--------SGHFDY | WGQGT<br>LVTVS<br>S |
| **iPS:4<br>35683** | **21-225_170A1** | **VH3\|3-<br>33/D4\|4-<br>17\|RF2/J<br>H4** | | QVQLVES-<br>GGGVVQPGRSLRL<br>SCAASG-FTFS | S-----YGMH | WVRQAPGK<br>GLEWVA | IIWYD---<br>GSYKYYADS<br>VKG | RFTISRDNSKNTLYLQMNSL<br>RAEDTAVYYCAR | DAHDFW----------<br>--------SGYFDS | WGQGT<br>LVTVS<br>S |
| **iPS:4<br>35755** | **21-225_176H4** | **VH3\|3-<br>33/D4\|4-<br>17\|RF2/J<br>H4** | | QVQLVES-<br>GGGVVQPGRSLRL<br>SCAASG-FTFS | S-----YGMH | WVRQAPGK<br>GLEWVA | IIWYD---<br>GSYKYYADS<br>VKG | RFTISRDNSKNTLYLQMSSL<br>RAEDTAVYYCAR | DAHDFW----------<br>--------SGYFAY | WGQGA<br>LVTVS<br>S |
| **iPS:4<br>35795** | **21-225_181C2** | **VH3\|3-<br>33/D4\|4-<br>17\|RF2/J<br>H4** | | QVQLVES-<br>GGGVVQPGRSLRL<br>SCVASG-FTFS | S-----YGMH | WVRQAPGK<br>GLEWVT | IIWYD---<br>GSYKYYADS<br>VKG | RFTISRDNSKNTLYLQMNSL<br>RAEDTAVYYCAR | DHYDFW----------<br>--------SGHFDF | WGQGT<br>LVTVS<br>S |
| **iPS:4<br>35807** | **21-<br>225_181C10** | **VH3\|3-<br>33/D4\|4-<br>17\|RF2/J<br>H4** | | QVQLVES-<br>GGGVVQPGRSLRL<br>SCAASG-FTFS | S-----YGMH | WVRQAPGK<br>GLEWMA | IIWYD---<br>GSYKYYADS<br>VKG | RFTISRDNSKNTLYLQMNSL<br>RAEDTAVYYCAR | DHYDFW----------<br>--------SGHFDY | WGQGT<br>LVTVS<br>S |
| **iPS:4<br>35887** | **21-225_186F7** | **VH3\|3-<br>33/D4\|4-<br>17\|RF2/J<br>H4** | | QVQLVES-<br>GGGVVQPGRSLRL<br>SCAASG-FTFS | T-----YGMH | WVRQAPGK<br>GLEWVA | IIWYD---<br>GSYKYYADS<br>VKG | RFTISRDNSKNTLYLQMNSL<br>RAEDTAVYYCAR | DHYDFW----------<br>--------SGHFDY | WGQGT<br>LVTVS<br>S |
| **iPS:4<br>35901** | **21-225_189G2** | **VH3\|3-<br>33/D4\|4-<br>17\|RF2/J<br>H4** | | QVQLVES-<br>GGGVVQPGRSLRL<br>SCAASG-FTFS | N-----YGMH | WVRQAPGK<br>GLEWVA | IIWYD---<br>GSYKYYADS<br>VKG | RFTISRDNSKNTLYLQMNSL<br>RAEDTAVYYCAR | DRFDFW----------<br>--------SGYSDY | WGQGT<br>LVTVS<br>S |
| **iPS:4<br>36594** | **21-225_226A5** | **VH3\|3-<br>33/D4\|4-<br>17\|RF2/J<br>H4** | | QVQLVES-<br>GGGVVQPGRSLRL<br>SCAASG-FTFS | N-----YGMH | WVRQAPGK<br>GLEWVA | IIWYD---<br>GTNKYYTDS<br>VKG | RFTISRDNSKNTLYLQMNSL<br>RAEDTAVYYCAR | EGHDFW----------<br>--------SGFFCY | WGQGT<br>LVTVS<br>S |
| **iPS:3<br>92814** | **21-225_22A1** | **VH3\|3-<br>33/D4\|4-<br>17\|RF2/J<br>H4** | | QVQLVES-<br>GGGVVQPGRSLRL<br>SCAASG-FTFS | S-----YGMH | WVRQAPGK<br>GLEWVA | VMWYD---<br>GSNKYYADS<br>VKG | RFTISRDNSKNTLYLQMNSL<br>RAEDTAVYYCAR | DGGFL-----------<br>---------EWLDY | WGQGT<br>LVTVS<br>S |
| **iPS:3<br>93036** | **21-225_28G3** | **VH3\|3-<br>33/D4\|4-<br>17\|RF2/J<br>H4** | | QVQLVES-<br>GGGVVQPGRSLRL<br>SCAASG-FIFS | T-----YGMH | WVRQAPGK<br>GLEWVA | VIWYD---<br>GSNKYYADS<br>VKG | RFTISRDNSKNTLYLQMNSL<br>RAEDTAVYYCAR | DRYDFW----------<br>--------SGYFDY | WGQGT<br>LVTVS<br>S |
| | | **Germline** | | **H_FR1** | **H_CDR1** | **H_FR2** | **H_CDR2** | **H_FR3** | **H_CDR3** | **H_FR4** |
| | **VH3\|3-33/D7\|7-<br>27\|RF1/JH4** | | 32225 | QVQLVES-<br>GGGVVQPGRSLRL<br>SCAASG-FTFS | S-----YGMH | WVRQAPGK<br>GLEWVA | VIWYD---<br>GSNKYYADS<br>VKG | RFTISRDNSKNTLYLQMNSL<br>RAEDTAVYYCAR | LIGY------------<br>-----------FDY | WGQGT<br>LVTVS<br>S |
| **iPS:4<br>68824** | **21-225_73G6** | **VH3\|3-<br>33/D7\|7-** | | QVQLVES-<br>GGGVVQPGRSLRL | S-----YGMH | WVRQAPGK<br>GLEWVA | VIWYD---<br>VSNKYYGDS | RFTISRDNSKNTLYLQMNSL<br>RAEDTAVYYCAR | EVGM------------<br>----------TSDY | WGQGT<br>LVTVS |

| | | | | | | | | | | |
|---|---|---|---|---|---|---|---|---|---|---|
| iPS:4 34169 | 21-225_50C4 | VH3\|3-33/D7\|7-27\|RF1/JH4 | | QVQLVES-GGGVVQPGRSLRLSCAASG-FTFS | D-----YGMH | WVRQAPGKGLEWVA | VIWYE---ETNKYYADSVKG | RFTISRDNSKNTLYLQMNSLRGEDTAVYYCAR | EVGF------------ ----------LNDY | WGQGILVTVSS |
| iPS:4 35045 | 21-225_90H5 | VH3\|3-33/D7\|7-27\|RF1/JH4 | | QVQLVES-GGGVVQPGRSLRLSCAASG-FTFS | D-----YGMH | WVRQSPGKGLEWVA | VIWYE---GSNTYYADSVKG | RFTISRDNSKNTLYLQMNSLRAEDTAVYYCVR | EMGW------------ ----------LDDY | WGQGTLVTVSS |
| iPS:4 35367 | 21-225_149G1 | VH3\|3-33/D7\|7-27\|RF1/JH4 | | QVQLVES-GGGVVQPGRSLRLSCAASG-FTFS | D-----YGMH | WVRQAPGKGLEWVA | VIWYE---GSNKYYADSVKG | RFTISRDNSKNMLYLQMNSLRAEDTAVYYCAR | EIGF------------ ----------SEDY | WGQGTLVTVSS |
| iPS:4 35397 | 21-225_149F12 | VH3\|3-33/D7\|7-27\|RF1/JH4 | | QVQLVES-GGGVVQPGRSLRLSCAASG-FTFS | D-----YGMH | WVRQAPGKGLEWVA | VIWYE---ENNKYYADSVKG | RFTISRDNSKNTLFLQMNSLRAEDTAVYYCAR | EIGF------------ ----------SEDY | WGQGTLVTVSS |
| iPS:4 35407 | 21-225_150E7 | VH3\|3-33/D7\|7-27\|RF1/JH4 | | QVQLVES-GGGVVQPGRSLRLSCAASG-FTFS | D-----YGMH | WVRQAPGKGLEWVA | VIWYE---ENNKYYADSVKG | RFTISRDNSKNTLFLQMNSLRAEDTAVYYCAR | EIGF------------ ----------SEDY | WGQGTLVTVSS |
| iPS:4 35609 | 21-225_161F7 | VH3\|3-33/D7\|7-27\|RF1/JH4 | | QVQLVES-GGGVVQPGRSLRLSCAASG-FTFS | D-----FGLH | WVRQAPGQGLEWVA | VIWFD---GSNKYYADSVKG | RFTIFRDNSKNTLYLQMNSLRAEDTAVYYCAR | EIGW------------ ----------LSDY | WGQGTLVTVSS |
| iPS:4 35613 | 21-225_161D11 | VH3\|3-33/D7\|7-27\|RF1/JH4 | | QVQLVES-GGGVVQPGRSLRLSCAASG-FTFS | D-----FGLH | WVRQAPGQGLEWVA | VIWFD---GSNKYYADSVKG | RFTIFRDNSKNTLYLQMNSLRAEDTAVYYCAR | EIGW------------ ----------LSDY | WGQGTLVTVSS |
| iPS:4 35791 | 21-225_180H7 | VH3\|3-33/D7\|7-27\|RF1/JH4 | | QVQLVES-GGGVVQPGRSLRLSCAASG-FTFS | D-----YGMH | WVRQAPGKGLEWVA | VIWYD---ENNKHYADSAKG | RFTISRDNSKNTLYLQMNSLRVEDTAVYYCAR | EVGW------------ ----------SDDY | WGQGTLVTVSS |
| iPS:4 35805 | 21-225_181A8 | VH3\|3-33/D7\|7-27\|RF1/JH4 | | QVQLVES-GGGVVQPGRSLRLSCAASG-FTFS | D-----YGMH | WVRQAPGKGLEWVA | VIWYD---ENNKHYADSAKG | RFTISRDNSKNTLYLQMNSLRAEDTAVYYCAR | EVGW------------ ----------SDDY | WGQGTLVIVSS |
| iPS:4 35879 | 21-225_184H10 | VH3\|3-33/D7\|7-27\|RF1/JH4 | | QVQLVES-GGGVVQPGRSLRLSCVASG-FTFS | D-----YGMH | WVRQAPGKGLEWVA | VIWYD---ETNKHYGDSVKG | RFTISRDNSKDTLYLQMNSLRAEDTAVYYCAR | EVGW------------ ----------HDDY | WGQGTLVTVSS |
| iPS:4 35881 | 21-225_184D11 | VH3\|3-33/D7\|7-27\|RF1/JH4 | | QVQLVES-GGGVVQPGRSLRLSCAASG-FTFS | D-----YGMH | WVRQAPGKGLEWVA | VIWYD---ETNKHYGDSVKG | RFTISRDNSKDTLYLQMNSLRAEDTAVYYCAR | EVGW------------ ----------HDDY | WGQGTLVTVSS |
| iPS:4 36350 | 21-225_210E4 | VH3\|3-33/D7\|7- | | QVQLVES-GGGVVQPGRSLRL | N-----YGMH | WVRQAPGKGLEWVA | VIWYD---ENNKYYVDS | RFTISRDDSKNTLYLQMNSLRAEDSAVYYCAR | ETGF------------ ----------LSDY | WGQGTLVTVS |

| | | | | | | | | | | |
|---|---|---|---|---|---|---|---|---|---|---|
| iPS:4 36576 | 21-225_225B6 | VH3\|3-33/D7\|7-27\|RF1/JH4 | | QVRLVES-GGGVVQPGRSLRLSCAASG-FTFS | D-----YGMH | WVRQAPGKGLEWVA | VIWYD---ENNKYYADSVKG | RFTISRDNSKNTLYLQMNSLRAEDTAVYYCAR | EVGF----------------------TEDY | WGQGTLVTVSS |
| iPS:4 36578 | 21-225_225D6 | VH3\|3-33/D7\|7-27\|RF1/JH4 | | QVQLVES-GGGVVQPGRSLRLSCAASG-FTFS | N-----YGMH | WVRQAPGKGLEWVA | VIWYD---ENNKYYADSVKG | RFTISRDNSQNTLYLQMTSLRAEDTAVYYCAR | EVGF----------------------TEDY | WGQGTLVTVSS |
| iPS:4 36582 | 21-225_225F8 | VH3\|3-33/D7\|7-27\|RF1/JH4 | | QVQLVES-GGGVVQPGRSLRLSCAASG-FTFS | S-----YGMH | WVRQAPGKGLEWVA | VIWYD---ENNKYYVDSVKG | RFTISRDNSKNTLYLQMNSLRAEDTAVYYCAR | EVGF----------------------TEDY | WGQGTLVTVSS |
| iPS:4 36608 | 21-225_226A9 | VH3\|3-33/D7\|7-27\|RF1/JH4 | | QVQLVES-GGGVVQPGRSLRLSCAASG-FTFS | N-----YGMH | WVRQAPGKGLEWVA | VIWYE---ESNKYYTDSVKG | RFTISRDNSKNTLFLQMNSLRAEDTAVYYCAR | EVGF----------------------TEDY | WGQGTLVTVSS |
| iPS:4 36630 | 21-225_227G3 | VH3\|3-33/D7\|7-27\|RF1/JH4 | | QVQLVES-GGGVVQPGRSLRLSCAASG-FTFS | S-----YGMH | WVRQAPGKGLEWVA | VIWYV---GSNQYYADSVKG | RFTISRDNSKNTLYLQMSSLRAEDTAVYYCAR | EVGF----------------------TEDY | WGQGTLVTVSS |
| iPS:4 36634 | 21-225_227H5 | VH3\|3-33/D7\|7-27\|RF1/JH4 | | QVQLVES-GGGVVQPGRSLRLSCAASG-FTFS | N-----YGMH | WVRQAPGKGLDWVA | VIWYE---ESNKYYADSVKG | RFTISRDNSKNTLYLQMNSLRAEDTAMYYCAR | EVGF----------------------TEDY | WGQGTLVTVSS |
| iPS:4 36650 | 21-225_227C12 | VH3\|3-33/D7\|7-27\|RF1/JH4 | | QVQLVES-GGGVVQPGKSLRLSCAASG-FTFS | N-----YGMH | WVRQAPGKGLEWVA | VIWYI---GSNQYYADSVKG | RFTISRDNSKNTLYLQMSSLRAEDTAVYYCAR | EVGF----------------------TEDY | WGQGTLVTVSS |
| iPS:4 37280 | 21-225_203C10 | VH3\|3-33/D7\|7-27\|RF1/JH4 | | QVQLVES-GGGVVQPGRSLRLSCAASG-FTFS | D-----YGMH | WVRQAPGKGLEWVA | VIWYD---GGNTHYTDSVKG | RFTISRDNSKNTLYLQMNSLRAEDTAVYYCAR | EVGW----------------------LDDY | WGQGTLVTVSS |
| iPS:3 92740 | 21-225_18H12 | VH3\|3-33/D7\|7-27\|RF1/JH4 | | QVQLVES-GGGVVQPGRSLRLSCAASG-FTFS | D-----YGMH | WVRQAPGKGLEWMA | VIWYD---VTNKYYADSVKG | RFTISRDNSKNTLYLQMNSLRAEDTAVYYCAR | EVGW----------------------YEDY | WGQGTLVTVSS |
| iPS:3 92780 | 21-225_22B7 | VH3\|3-33/D7\|7-27\|RF1/JH4 | | QVQLVES-GGGVVQPGRSLRLSCAASG-FTFS | S-----YGMH | WVRQAPGKGLEWVA | VIWYE---ENNQYYADSVKG | RFTISRDNSKNTLYLQMNSLRAEDTAVYYCAR | EVGF----------------------RSDY | WGQGTLVTVSS |
| iPS:3 92912 | 21-225_25A9 | VH3\|3-33/D7\|7-27\|RF1/JH4 | | QVQLVES-GGGVVQPGRSLRLSCAASG-FTFS | S-----YGMH | WVRQAPGKGLEWVA | VIWYD---VTNKYYTGSVKG | RFTISRDNSKNTLYLQMNSLRAEDTAVYYCAR | EIGW----------------------LDDY | WGQGTLVTVSS |
| iPS:3 92940 | 21-225_29D9 | VH3\|3-33/D7\|7- | | QVQLVES-GGGVVQPGRSLKL | D-----YGIH | WVRQAPGKGLEWVA | VIWYD---ESNNYYADS | RFTISRDNSKNTLYLQMNSLRAEDTAVYYCAR | EIGW----------------------LDDY | WGQGTQVTVS |

| | | | | | | | | | | |
|---|---|---|---|---|---|---|---|---|---|---|
| **iPS:392948** | **21-225_25G5** | **VH3\|3-33/D7\|7-27\|RF1/JH4** | | QVQLVES-GGGVVQPGRSLRLSCAASG-FTFS | D-----YGIH | WVRQAPGKGLEWVA | VIWYD---GNNKYYADSVKG | RFTISRDNSKNTLYLQMNNLRAEDTAVYYCAR | EIGW---------------------LDDY | WGQGTLVTVSS |
| **iPS:392978** | **21-225_28B8** | **VH3\|3-33/D7\|7-27\|RF1/JH4** | | QVQLVES-GGGVVQPGRSLRLSCAASG-FTFS | D-----YGMH | WVRQAPGKGLEWVT | VIWYD---ANNKYYADSVKG | RFTISRDNFKNTVYLQMNSLRAEDTAVYYCAR | EIGW---------------------LDDY | WGQGTLVTVSS |
| **iPS:392998** | **21-225_28A9** | **VH3\|3-33/D7\|7-27\|RF1/JH4** | | QVQLVES-GGGVVQPGRSLRLSCAASG-FTFS | S-----YGIH | WVRQAPGKGLEWVA | VIWFD---GSNKYYADSVKG | RFTISRDNSKNTLFLQMNSLRVEDTAVYYCAR | EIGW---------------------LDDY | WGQGTLVTVSS |
| **iPS:393038** | **21-225_29D8** | **VH3\|3-33/D7\|7-27\|RF1/JH4** | | QVQLVES-GGGVVQPGRSLRLSCAASG-FTFR | D-----YGIH | WVRQAPGKGLEWVA | VIWFD---GTNKYYADSVKG | RFTISRDNSKNTLYLQMNSLRAEDTAVYYCGR | EIGW---------------------LDDY | WGQGTLVTVSS |
| **iPS:393056** | **21-225_30F3** | **VH3\|3-33/D7\|7-27\|RF1/JH4** | | QVQLVES-GGGVVQPGRSLRLSCAASG-FTFS | S-----YGMH | WVRQAPGKGLEWVA | VIWYD---VSNKYYADSVKG | RFTISRDNSKNTLYLQMNSLRAEDTAVYYCAR | EMGW---------------------YDDY | WGQGTLVTVSS |
| **iPS:393074** | **21-225_33B1** | **VH3\|3-33/D7\|7-27\|RF1/JH4** | | QVQLVES-GGGVVQPGRSLRLSCAASG-FTFS | D-----YGMH | WVRQAPAKGLEWVA | VIWYD---RNNKYYADSVKG | RFTISRDNSKNTLYLQMNSLRAEDTAVYYCAR | EMGW---------------------YDDY | WGQGTLVTVSS |
| **iPS:393822** | **21-225_15B11** | **VH3\|3-33/D7\|7-27\|RF1/JH4** | | QVQLVES-GGGVVQPGRSLRLSCAASG-FTFS | N-----YGMH | WVRQAPGKGLEWVA | VIWYE---ESNKYYTDSVKG | RFTISRDNSKNTLYLQMNSLRPEDTAVYYCAR | EVGF---------------------TEDY | WGQGTLVTVSS |
| **iPS:393856** | **21-225_14C2** | **VH3\|3-33/D7\|7-27\|RF1/JH4** | | QVQLVES-GGGVVQPGRSLRLSCAASG-FTFS | D-----YGMH | WVRQAPGKGLEWVA | VIWYD---ESNKYYEDSVKG | RFTISRDNSKNTLYLQMKSLRAEDTGVYYCAR | EVGF---------------------RSDY | WGQGTLVTVSS |
| **iPS:393874** | **21-225_4C8** | **VH3\|3-33/D7\|7-27\|RF1/JH4** | | QVQLVES-GGGVVQPGRSLRLSCAASG-FTFS | N-----YGMH | WVRQAPGKGLEWVA | VIWYE---ENNQYYADSVKG | RFTISRDNSKNTLYLQMNSLTAEDTAVYYSPR | EMGF---------------------LSDY | WGQGTLVTVSS |
| **iPS:393984** | **21-225_4F12** | **VH3\|3-33/D7\|7-27\|RF1/JH4** | | QVQLVES-GGGVVQPGRSLRLSCAASG-FTFS | S-----YGMH | WVRQAPGKGLEWVA | VIWYD---VTNKKYADSVKG | RFTISRDNSKNTLYLQMNSLTPENTGGYENQR | EKGG---------------------LFDY | WGQGTLVTVSS |
| **iPS:394020** | **21-225_15H10** | **VH3\|3-33/D7\|7-27\|RF1/JH4** | | QVQLVES-GGGVVQPGRSLRLSCAASG-FTFS | N-----YGMH | WVRQAPGKGLEWVA | VIWYD---ESNKYYEDSVKG | RFTISRDNSKNTLYLQMNSLRAEDTAVYYCVR | EVGF---------------------LSDY | WGQGILVTVSS |
| **iPS:394095** | **21-225_16H4** | **VH3\|3-33/D7\|7-** | | QVQLVES-GGGVVQPGRSLRL | N-----YGMH | WVRQAPGKGLEWVA | VIWYD---VSNKYYADS | RFTISRDNSKNMLYLQMNSLRAEDTAVYYCAR | EMGW---------------------TDDC | WGQGTLVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH1\|1-08/D5\|5-24\|RF3/JH6 | | 32226 | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | RDGYNYYY----------------YYYGMDV | WGQGTTVTVSS |
| iPS:468818 | 21-225_190C8 | VH1\|1-08/D5\|5-24\|RF3/JH6 | | QVQLVQS-GAEVKKPGASVKVSCKASG-YTFI | S-----YDIN | WVRQATGQGLEWMG | WMNPK---RGNTGYAQKFQG | RVTMTRDTSISTAYMELSSLRSEDTAVYYCAR | GDPYNWN-----------------SYAMDV | WGQGATVTVSS |
| iPS:436023 | 21-225_193A5 | VH1\|1-08/D5\|5-24\|RF3/JH6 | | QVQLVQS-GAEVKKPGASVKVSCKASG-YTFI | S-----YDIN | WVRQATGQGLEWMG | WMNPK---RGNTGYAQKFQG | RVTMTRDTSISTAYMELSSLRSEDTAVYYCAR | GDPYNWN-----------------SYAMDV | WGQGATVTVSS |
| iPS:436132 | 21-225_196C12 | VH1\|1-08/D5\|5-24\|RF3/JH6 | | QVQLVQS-GAEVKKPGASVKVSCKASG-YTFI | S-----YDIN | WVRQATGQGLEWMG | WMNPK---RGNTGYAQKFQG | RVTMTRDTSISTAYMELSSLRSEDTAVYYCAR | GDPYNWN-----------------SYAMDV | WGQGATVTVSS |
| | | Germline | | H_FR1 | H_CDR1 | H_FR2 | H_CDR2 | H_FR3 | H_CDR3 | H_FR4 |
| | VH3\|3-33/D2\|2-8\|RF3/JH5 | | 32227 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DIVLMVY----------------AINWFDP | WGQGTLVTVSS |
| iPS:468828 | 21-225_162A10 | VH3\|3-33/D2\|2-8\|RF3/JH5 | | QVQLVES-GGGVVQPGRSLRLSCAASG-FTFS | S-----CGMH | WVRQAPGKGLEWVA | AIWYD---GSNKYYADSVKG | RFTISRDNSKNTLFLQMNSLRAEDTAVYYCAR | DKNIMG------------------DTWFDF | WGQGTLVTVSS |
| | | Germline | | H_FR1 | H_CDR1 | H_FR2 | H_CDR2 | H_FR3 | H_CDR3 | H_FR4 |
| | VH1\|1-02/D5\|5-18\|RF3/JH4 | | 32228 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | GYSYG--------------------YYFDY | WGQGTLVTVSS |
| iPS:468830 | 21-225_191G11 | VH1\|1-02/D5\|5-18\|RF3/JH4 | | QVQLVQS-GAEVKKPGASVKVSCKASG-YTFT | G-----YYMH | WVRQAPGQGLEWMG | WINPN---SGGTNFAQKFQG | RVTLTRDTSINTAYMELSWLRSDDTAVYYCAR | GKNYG--------------------SYFDY | WGQGTLVTVSS |
| iPS:436896 | 21-225_67F10 | VH1\|1-02/D5\|5-18\|RF3/JH4 | | QVQLVQS-GAEVKKPGASVKVSCKASG-YTFT | G-----YYMH | WVRQAPGQGLEWMG | WINPN---SGGTNYGQKFQG | RVTMTRDTSISTAYMELSRLRSDDTAVYYCAR | TYFYGSGS---------------YYNGFDY | WGQGTLVTVSS |
| iPS:393218 | 21-225_14G3 | VH1\|1-02/D5\|5-18\|RF3/JH4 | | QVQLVQS-GAEVKKPGASVKVSCKASG-YTFT | G-----YYMY | WVRQAPGQGLEWMG | WINPN---SGGTNYAQKFQG | RVTMTRDTSISTAYMELSRLRSDDTAVYYCAR | SYFYGSGS---------------YYNEFDY | WGQGTLVTVSS |
| iPS:393565 | 21-225_34B11 | VH1\|1-02/D5\|5-18\|RF3/JH4 | | QVKLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | VYFYGSGS---------------YYNEFDY | WGQGTLVTVSS |
| iPS:398470 | 21-225_14B7 | VH1\|1-02/D5\|5- | | QVQLVQS-GAEVKKPGASVKV | G-----YYMH | WVRQAPGQGLEWMG | WINPN---SGGTNYVQK | RVTMTRDTSISTACMELSRLKSDDTAVYFCAR | SFFYGSGS---------------YYNEFDY | WGQGTLVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH3\|3-30.3/D1\|1-1\|RF1/JH6 | | 32229 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | GTTGTYY-----------------YYYGMDV | WGQGTTVTVSS |
| iPS:468836 | 21-225_198E3 | VH3\|3-30.3/D1\|1-1\|RF1/JH6 | | QVQLVES-GGGVVQPGRSLRLSCAASG-FTFS | S-----YGMH | WVRQAPGKGLEWVA | VISYD---GGYKNYADSVKG | RFTISRDNSKNTLYLQMNSLRAEDTAVYYCAR | GTHGYY-------------------YGVDV | WGQGTTVTVSS |
| iPS:435831 | 21-225_190C12 | VH3\|3-30.3/D1\|1-1\|RF1/JH6 | | QVQLVES-GGAVVQPGRSLRLSCAASG-FTFS | S-----YGMH | WVRQAPGKGLEWVA | VISYD---GGYKNYVDSVKG | RFTISRDNSKNTLYLQMNSLRAEDTAVYYCAR | GTHGYY-------------------YGVDV | WGQGTTVTVSS |
| iPS:435857 | 21-225_191A4 | VH3\|3-30.3/D1\|1-1\|RF1/JH6 | | QVQLVES-GGGVVQPGRSLRLSCAASG-FTFS | S-----YGMH | WVRQAPGKGLEWVA | VISYD---GGYKNYADSVKG | RFTISRDNSKNTLYLQMNSLRAEDTAVYYCAR | GTHGYY-------------------YGVDV | WGQGTTVTVSS |
| iPS:435907 | 21-225_190G3 | VH3\|3-30.3/D1\|1-1\|RF1/JH6 | | QVQLVES-GGGVVQPGRSLRLSCAASG-FTFS | S-----YGMH | WVRQAPGKGLEWVA | VISYD---GGYKNYVDSVKG | RFTISRDNSKNTLYLQMNSLRAEDTAVYYCAR | GTHGYY-------------------YGVDV | WGQGTTVTVSS |
| iPS:435919 | 21-225_190H5 | VH3\|3-30.3/D1\|1-1\|RF1/JH6 | | QVQLVES-GGGVVQPGRSLRLSCAASG-FTFS | S-----YGMH | WVRQAPGKGLEWVA | VISYD---GGYKNYADSVKG | RFTISRDNSKNTLYLQMNSLRAEDTAVYYCAR | GTHGYY-------------------YGVDV | WGQGTTVTVSS |
| iPS:435989 | 21-225_192F7 | VH3\|3-30.3/D1\|1-1\|RF1/JH6 | | QVQLVES-GGGVVQPGRSLRLSCAASG-FTFS | S-----YGMH | WVRQAPGKGLEWVT | VISYD---GGYKNYADSVKG | RFTISRDNSKNTLYLQMNSLRAEDTAVYYCAR | GTHGYY-------------------YGVDV | WGQGTTVTVSS |
| iPS:436222 | 21-225_200C9 | VH3\|3-30.3/D1\|1-1\|RF1/JH6 | | QVQLVES-GGGVVQPGRSLRLSCAASG-FTFS | S-----YGMH | WVRQAPGKGLEWVA | VISYD---GGYKNYIDSVKG | RFTISRDNSKNTLYLQMNSLRAEDTAVFYCAR | GTHGYY-------------------YGVDV | WGQGTTVTVSS |
| | | Germline | | H_FR1 | H_CDR1 | H_FR2 | H_CDR2 | H_FR3 | H_CDR3 | H_FR4 |
| | VH4\|4-30.1/D5\|5-24\|RF3/JH6 | | 32230 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | RDGYNYYY---------------YYYGMDV | WGQGTTVTVSS |
| iPS:468840 | 21-225_200H9 | VH4\|4-30.1/D5\|5-24\|RF3/JH6 | | QVQLQES-GPGLVKPSQTLSLTCIVSG-GSMR | SG---GDYWS | WIRQHPGKGLEWFG | YIYY----SGSTYYNPSLKS | RVTLSVDTSKNQFSLKLSSVTAADTAVYYCAR | MDYSNY------------------YYGMDV | WGQGTSVTVSS |
| iPS:436096 | 21-225_195E10 | VH4\|4-30.1/D5\|5-24\|RF3/JH6 | | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVAAADTAVYYCAR | GGYNWN-------------------HGMDV | WGQGTTVTVSS |
| iPS:436120 | 21-225_196C10 | VH4\|4-30.1/D5\|5- | | QVQLQES-GPGLVKPSQTLSL | SG---GDYWS | WIRQHPGKGLEWFG | FIYY----SGSTYYNPS | RVTLSVDTSKNQFSLKLSSVTAADTAVYYCAR | MDYSNY------------------YYGMDV | WGQGTTVTVS |

| | | | | | | | | | | |
|---|---|---|---|---|---|---|---|---|---|---|
| iPS:4 36216 | 21-225_200B7 | VH4\|4-30.1/D5\|5-24\|RF3/JH6 | | QVQLQES-GPGLVKPSQTLSLTCTVSG-GSIS | SG---GYYWS | WIRQHPGKGLEWIG | YIFY----SGSTNYNPSLRS | RVTISVDTSKNQFSLKLSSVTAADTAVYYCAR | AGYNWN-------------------NGMDV | WGQGTTVTVSS |
| | | Germline | | H_FR1 | H_CDR1 | H_FR2 | H_CDR2 | H_FR3 | H_CDR3 | H_FR4 |
| | VH3\|3-21/D6\|6-6\|RF2/JH4 | | 32231 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | SIAAR---------------------YFDY | WGQGTLVTVSS |
| iPS:4 68844 | 21-225_48E10 | VH3\|3-21/D6\|6-6\|RF2/JH4 | | EVQLVES-GGGLVKPGGSLRLSCAASG-FTFS | S-----YNMN | WVRQAPGKGLEWVS | SISGS---SSYIYYADSVKG | RFTISRDNAKNSLYLQMNSLRAEDTAVYYCAR | SL--------------------------DL | WGQGTLVTVSS |
| iPS:4 35537 | 21-225_157H12 | VH3\|3-21/D6\|6-6\|RF2/JH4 | | EVQWVES-GGGLVKPGGSLRLSCAASG-FTFS | S-----YSMN | WVRQAPGKGLEWVS | SISGS---GSYINYADSVKG | RFTISRDNAKTSLYLQVNGLRAEDTAVYYCAR | SKF-------------------------DS | WGQGTLVTVSS |
| iPS:4 35539 | 21-225_158G1 | VH3\|3-21/D6\|6-6\|RF2/JH4 | | EVQLVES-GGGLVKPGGSLRLSCAASG-FTFS | S-----YGMN | WVRQAPGKGLEWIS | SISGS---GSYIYYADSVKG | RFTISRDNAKNSLYLQMNSLRAEDTAVYYCAI | SSG-------------------------WS | WGQGTLVTVSS |
| iPS:4 35583 | 21-225_160F2 | VH3\|3-21/D6\|6-6\|RF2/JH4 | | EVQLVES-GGGLVKPGGSLRLSCAASG-FTFS | S-----YGMN | WVRQAPGKGLEWIS | SISGS---GSYIYYADSVKG | RFTISRDNAKNSLYLQMNSLRAEDTAVYYCAI | SSG-------------------------WS | WGQGTLVTVSS |
| | | Germline | | H_FR1 | H_CDR1 | H_FR2 | H_CDR2 | H_FR3 | H_CDR3 | H_FR4 |
| | VH3\|3-21/D1\|1-1\|RF2/JH5 | | 32232 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | VQLER--------------------NWFDP | WGQGTLVTVSS |
| iPS:4 68846 | 21-225_53B10 | VH3\|3-21/D1\|1-1\|RF2/JH5 | | EVQLVES-GGGLVKPGGSLRLSCAASG-FTFS | S-----YSMN | WVRQAPGKGLEWVS | SISGS---SSYIYYVDSVKG | RFTISRDNAKNSLYLQMNSLRAEDTAVYYCAR | VNS------------------------FDS | WGQGTLVTVSS |
| iPS:4 34251 | 21-225_62G3 | VH3\|3-21/D1\|1-1\|RF2/JH5 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | VNS------------------------FDS | WGQGTLVTVSS |
| iPS:4 34407 | 21-225_68G8 | VH3\|3-21/D1\|1-1\|RF2/JH5 | | EVQLVES-GGGLVKPGGSLRLSCAASG-FTFS | S-----YSMN | WVRQAPGKGLEWVS | SISGS---SSYIYYADSVMG | RFTISRDNAKNSLYLQMNSLRAEDTAVYYCAR | VNS------------------------FDS | WGQGTLVTVSS |
| iPS:4 35575 | 21-225_159H11 | VH3\|3-21/D1\|1-1\|RF2/JH5 | | EVQLVES-GGGLVKPGGSLRLSCAASG-FTFS | S-----YTMN | WVRQAPGKGLEWVS | SISGS---SSYIYYADSVKG | RFTISRDNAKNSLYLQMNSLRAEDTAVYYCAR | VSW------------------------ADC | WGQGTLVTVSS |
| | | Germline | | H_FR1 | H_CDR1 | H_FR2 | H_CDR2 | H_FR3 | H_CDR3 | H_FR4 |
| | VH3\|3-23/D1\|1- | | 32233 | EVQLLES- | S-----YAMS | WVRQAPGK | AISGS--- | RFTISRDNSKNTLYLQMNSL | VQLER------------ | WGQGT |

| | | | | | | | | | | |
|---|---|---|---|---|---|---|---|---|---|---|
| iPS:4 68848 | 21-225_54B1 | VH3\|3-23/D1\|1-1\|RF2/JH4 | | EVQLLES-GGGLVQPGGSLRLSCAASG-FTFS | S-----YAMS | WVRQAPGKGLEWVS | VLSGS---GGSTFYADSVKG | RFTISRENSKNTLYLQMSSLRAEDTAVYYCAR | RGREYSG-----------------YDYFDY | WGQGTLVTVSS |
| iPS:4 33993 | 21-225_47G7 | VH3\|3-23/D1\|1-1\|RF2/JH4 | | EVQLLDS-GGGLVQPGGSLRLSCAASG-FTFS | S-----YAMS | WVRQAPGKGLEWVS | AISGR---GGNTFYAESVRG | RFTISRDNSKNTLYLQMNSLRAEDTAVYYCAK | IIREQ-------------------WAFDY | WGQGTLVTVSS |
| iPS:4 34007 | 21-225_48D7 | VH3\|3-23/D1\|1-1\|RF2/JH4 | | EVQLLES-GGGLVQPGGSLRLSCAASG-FTFR | N-----SAMN | WVRQAPGKGLEWVS | AISGS---GGTTFYADSVKG | RFTISRDNSKNTLYLQINSLRAEDTAVYYCAK | CGREQ--------------------WLDY | WGQGTLVTVSS |
| iPS:4 34115 | 21-225_53E4 | VH3\|3-23/D1\|1-1\|RF2/JH4 | | EVQLLES-GGGLVQPGGSLRLSCAASG-FTFS | S-----YVMS | WVRQAPGKGLEWVS | GISGS---GGRTYYADSVKG | RFNISRDNSKNTLYLQMNSLRAEDTAVYYCAR | VAL-----------------------FDY | WGQGTLVTVSS |
| iPS:4 35679 | 21-225_169D10 | VH3\|3-23/D1\|1-1\|RF2/JH4 | | EVQLLES-GGGLVQSGGSLRLSCAASG-FTFS | S-----YVMS | WVRQAPGKGLEWVS | AISGS---GSRIYYADSVKG | RFTISRDNSKNTLYLQMNSLRAEDTALYYCAR | VAF-----------------------FDY | WGQGTLVTVSS |
| iPS:4 35685 | 21-225_170E1 | VH3\|3-23/D1\|1-1\|RF2/JH4 | | EVQLLES-GGGLVQPGGSLRLSCAASG-FTFS | S-----YVMS | WVRQAPGKGLEWVS | AISGS---GNRIYYADSVKG | RFTISRDNSKNTLYLQMNSLRAEDTALYYCAR | VAF-----------------------FDY | WGQGTLVTVSS |
| iPS:4 36632 | 21-225_227E4 | VH3\|3-23/D1\|1-1\|RF2/JH4 | | EGQLLES-GGGLVQPGGSLRLSCTASG-FTFS | T-----FAMT | WVRQAPGRGLEWVS | VISGR---GGSSFYADSVKG | RFTISRDNTKNTLYLQMNSLRAEDTAVYYCAK | DQLW----------------------FDY | WGQGTLVTVSS |
| | | Germline | | H_FR1 | H_CDR1 | H_FR2 | H_CDR2 | H_FR3 | H_CDR3 | H_FR4 |
| | VH4\|4-39/D4\|4-11\|RF2/JH5 | | 32234 | QLQLQES-GPGLVKPSETLSLTCTVSG-GSIS | SS---SYYWG | WIRQPPGKGLEWIG | SIYY----SGSTYYNPSLKS | RVTISVDTSKNQFSLKLSSVTAADTAVYYCAR | DYSNY-------------------NWFDP | WGQGTLVTVSS |
| iPS:4 68856 | 21-225_77C9 | VH4\|4-39/D4\|4-11\|RF2/JH5 | | QLQLQES-GPGLVTPSETLSLTCTVSG-GSIS | RS---SYYWG | WIRQPPGKGLEWIG | SIYY----SGSAYSNPSLKS | RVTISVDTSKNQFSLKLSSVTAADTALFYCAR | LDSNW--------------------GLDY | WGQGTLVTVSS |
| iPS:4 34489 | 21-225_74E4 | VH4\|4-39/D4\|4-11\|RF2/JH5 | | QLQLQES-GPGLVKPSETLSLICTVSG-GSIS | SS---NYYWG | WIRQPPGKGLEWIG | SIYY----SGYTSYNPSLKS | RVTISVDSSKNHFSLRLSSVTAADTAVYYCAR | LDSNW--------------------GLDY | WGQGTLVTVSS |
| iPS:4 35251 | 21-225_96A3 | VH4\|4-39/D4\|4-11\|RF2/JH5 | | QLQLQES-GPGLVKPSETLSLICTVSG-GSIS | SS---NYYWG | WIRQPPGKGLEWIG | SIYY----SGYTSYNPSLKS | RVTISVDSSKNHFSLRLSSVTAADTAVYYCAR | LDSNW--------------------GLDY | WGQGTLVTVSS |
| iPS:4 37346 | 21-225_75H7 | VH4\|4-39/D4\|4- | | QLQLQES-GPGLVTPSETLSL | RS---SYYWG | WIRQPPGKGLEWIG | SIYY----SGSAYSNPS | RVTISVDTSKNQFSLKLSSVTAADTALFYCAR | LDSNW--------------------GLDY | WGQGTLVTVS |

| | | | | | | | | | | |
|---|---|---|---|---|---|---|---|---|---|---|
| iPS:393886 | 21-225_2G9 | VH4\|4-39/D4\|4-11\|RF2/JH5 | | QLQLQES-GPGLVKPSETLSLTCTVSG-GSIR | PN----YYWG | WIRQPPGKGLEWIG | SIYY----SGSTSYNPSLNS | RVTISVDTSKNQFSLKLNSVTAADTAVYYCAR | LSSNW---------------------DFDN | WGQGTLVTVSS |
| iPS:393928 | 21-225_4E10 | VH4\|4-39/D4\|4-11\|RF2/JH5 | | QLQLQES-GPGLVKPSETLSLTCTVSG-GSIS | RS---SYYWG | WIRQPPGKGLEWIG | SVYY----SGATSYNPSLKS | RVTISVDTSKNQFSLKLNSVTAADTALYYCVR | LSSNW---------------------DFDY | WGQGTLFTVSS |
| | | Germline | | H_FR1 | H_CDR1 | H_FR2 | H_CDR2 | H_FR3 | H_CDR3 | H_FR4 |
| | VH1\|1-02/D6\|6-6\|RF1/JH6 | | 32235 | QVQLVQS-GAEVKKPGASVKVSCKASG-YIFT | G-----YYMH | WVRQAPGQGLEWMG | WINPN---SGGTNYAQKFQG | RVTMTRDTSISTAYMELSRLRSDDTAVYYCAR | EYSSSSYY---------------YYYGMDV | WGQGTTVTVSS |
| iPS:468862 | 21-225_178H8 | VH1\|1-02/D6\|6-6\|RF1/JH6 | | QVQLVQS-GAEVRTPGASVKVSCKASG-YTFT | D-----YYMH | WVRQAPGQGLEWMG | WINPN---RGGTNYAQKFQG | RVTMTRDTSISTAYMELSRLRSDDTAVYYCAR | EEDRSGWY---------------YYYGMDV | WGQGTTVTVSS |
| iPS:451112 | 21-225_53D10 | VH1\|1-02/D6\|6-6\|RF1/JH6 | | QVQLVQS-GAEVKKPGASVKVSCKASG-YIFT | G-----YYIH | WVRQAPGQGLEWMG | WINPN---SGGTNYAQKFQG | RVTMTRDTSISTAYMELIRLRSDDTAVYYCAR | ENESLATRP------------FYDYYGMDV | WGQGTTVTVSS |
| | | Germline | | H_FR1 | H_CDR1 | H_FR2 | H_CDR2 | H_FR3 | H_CDR3 | H_FR4 |
| | VH2\|2-05/D6\|6-6\|RF2/JH4 | | 32236 | QITLKES-GPTLVKPTQTLTLTCTFSG-FSLS | TS---GVGVG | WIRQPPGKALEWLA | LIYW----NDDKRYSPSLKS | RLTITKDTSKNQVVLTMTNMDPVDTATYYCAH | SIAAR---------------------YFDY | WGQGTLVTVSS |
| iPS:468864 | 21-225_60D6 | VH2\|2-05/D6\|6-6\|RF2/JH4 | | QITLKES-GPTLVKPTQTLTLTCTFSG-FSLS | TS---GVGVG | WIRQPPGKALEWLA | LIYW----KDDERYSPSLKS | RLTITKDTSKNQVVLTMTNMDPVDTATYYCAH | AVAV----------------------SFDY | WGQGTLVTVSS |
| iPS:436850 | 21-225_57D9 | VH2\|2-05/D6\|6-6\|RF2/JH4 | | QITLKES-GPMLVKPTQTLTLTCTFSG-FSLS | TS---GVGVG | WIRQPPGKALEWLA | LIYW----NDDKRYSPSLKS | RLTITEDTSKNQVVLTMTNMDPVDTATYYCAH | AVAV----------------------SFDY | WGQGTLVTVSS |
| iPS:436914 | 21-225_76B4 | VH2\|2-05/D6\|6-6\|RF2/JH4 | | QITLKES-GPTLVKPTQTLTLTCTFSG-FSLS | TG---GVGVG | WIRQPPGKALEWLA | LIYW----DDDKRYSPSLKS | RLTITKDTPKNQVVLTMTNMDPVDTATYYCAH | LIAV----------------------AFDY | WGQGTLVTVSS |
| iPS:436918 | 21-225_77A2 | VH2\|2-05/D6\|6-6\|RF2/JH4 | | QITLKES-GPSLVKPTQTLTLTCTFSG-FSLS | TS---GVGVG | WIRQPPGKALEWLV | FIYW----DDDKRYSPSLKS | RLTITKDTSKNQVVLTMTNMDPVDTATYYCAH | LIAV----------------------AFDY | WGQGTLVTVSS |
| iPS:436934 | 21-225_96B5 | VH2\|2-05/D6\|6-6\|RF2/JH4 | | QITLKES-GPTLVKPTQTLTLTCTFSG-FSLS | TG---GVGVG | WIRQPPGKALEWLA | LIYW----DDDKRYSPSLKS | RLTITKDTPKNQVVLTMTNMDPVDTATYYCAH | LIAV----------------------ACDY | WGQGTLVTVSS |
| iPS:437334 | 21-225_75F11 | VH2\|2-05/D6\|6- | | QITLKES-GPTLVKPTQTLTL | TG---GVGVG | WIRQPPGKALEWLA | LIYW----DDDKRYSPS | RLTITKDTPKNQVVLTMTNMDPVDTATYYCAH | LIAV----------------------AFDY | WGQGTLVTVS |

| | | | | | | | | | | |
|---|---|---|---|---|---|---|---|---|---|---|
| iPS:4 37377 | 21-225_74G9 | VH2\|2-05/D6\|6-6\|RF2/JH 4 | | QITLKES-GPTLVKPTQTLTL TCTFSG-FSLS | TG---GVGVG | WIRQPPGK ALEWLA | LIYW----DDDKRYSPS LKS | RLTITKDTPKNQVVLTMTNM DPVDTATYYCAH | LIAV------------ ----------AFDY | WGQGT LVTVS S |
| iPS:3 92583 | 21-225_10B10 | VH2\|2-05/D6\|6-6\|RF2/JH 4 | | QITLKES-GPTLVKPTQTLTL TCTFSG-FSLS | TG---GVGVG | WIRQPPGK ALEWLV | FIYW----SDDKRYSPS LKS | RLSITKDTSKNQVVLTMTNM DPVDTATYYCAR | IAAV------------ ----------AFDY | WGQGT LVTVS S |
| iPS:3 93184 | 21-225_15H11 | VH2\|2-05/D6\|6-6\|RF2/JH 4 | | QITLKES-GLTLMKPTQTLTL TCTFSG-FSLS | TG---GVGVG | WIRQPPGK ALEWLA | LIYW----HDDKRYSPS LRS | RLTITKDTSKNQVVLTMTNM DPVDTATYYCAR | IVAV------------ ----------AFDY | WGQGT LITVS S |
| iPS:3 93212 | 21-225_30H6 | VH2\|2-05/D6\|6-6\|RF2/JH 4 | | QITLKES-GPTLVKPTQTLTL TCTFSG-FSLS | TG---GVGVG | WIRQPPGK ALEWLA | LIYW----HDDKRYSPS LKS | RLAITKDTSKNQVVLTITNM DPVDTATYYCAH | LIAV------------ ----------AFDY | WGQGT LVTVS S |
| iPS:3 93222 | 21-225_17F5 | VH2\|2-05/D6\|6-6\|RF2/JH 4 | | QITLKES-GPSLVKPTQTLTL TCTFSG-FSLS | TS---GVGVG | WIRQPPGK ALEWLA | LIYW----DDDKRYSPS LKS | RLTITKDTSKNQVVLTMTNM DPVDTATYSCAH | IIAV------------ ----------AFDY | WGQGT LVTVS S |
| iPS:3 93224 | 21-225_31C2 | VH2\|2-05/D6\|6-6\|RF2/JH 4 | | QITLKES-GPTLVKPTQTLTL TCTFSG-FSLN | TG---GVGVG | WIRQPPGK ALEWLA | LIYW----NDDERYSPS LKS | RLTITKDTSKNQVVLTMTNM DPLDTASYYCAH | LIAV------------ ----------SFDY | WGQGA LVTVS S |
| | | Germline | | H_FR1 | H_CDR1 | H_FR2 | H_CDR2 | H_FR3 | H_CDR3 | H_FR4 |
| | VH1\|1-02/D6\|6-19\|RF2/JH6 | | 32237 | QVQLVQS-GAEVKKPGASVKV SCKASG-YTFT | G-----YYMH | WVRQAPGQ GLEWMG | WINPN---SGGTNYAQK FQG | RVTMTRDTSISTAYMELSRL RSDDTAVYYCAR | GIAVAGYY-------- -------YYYGMDV | WGQGT TVTVS S |
| iPS:4 68866 | 21-225_190C1 | VH1\|1-02/D6\|6-19\|RF2/J H6 | | QVQLVQS-GAEVKKPGASVKV SCKASG-YTFT | G-----YYMH | WVRQAPGQ GLEWMG | WINPY---SGGTNYAQK FQG | RVTMTRDTSISTAYMELSRL RSDDTAVYYCAR | DRAVAGNY-------- -------FYYGMDV | WGQGT TVTVS S |
| iPS:4 36972 | 21-225_190C7 | VH1\|1-02/D6\|6-19\|RF2/J H6 | | QVQLVQS-GAEVKKPGASVKV SCKASG-YTFT | G-----YYMH | WVRQAPGQ GLEWMG | WINPN---SGGTNYAQK FQG | RVTMTRDTSISTAYMELSRL RSDDTAVYYCAR | DRAVAGNY-------- -------FYYGMDV | WGQGT TVTVS S |
| iPS:4 37020 | 21-225_193F11 | VH1\|1-02/D6\|6-19\|RF2/J H6 | | QVQLVQS-GAEVKKPGASVKV SCKASG-YTFT | G-----YYMH | WVRQAPGQ GLEWMG | WINPY---SGGTNYAQK FQG | RVTMTRDTSISTAYMELSRL RSDDTAVYYCAR | DRAVAGNY-------- -------FYYGMDV | WGQGT TVTVS S |
| iPS:4 37036 | 21-225_195H9 | VH1\|1-02/D6\|6-19\|RF2/J H6 | | QVQLVQS-GAEVKKPGASVKV SCKASG-YTFT | G-----YYMH | WVRQAPGQ GLEWMG | WINPY---SGGTNYAQK FQD | RVTMTRDTSITTAYMELSRL RSDDTAVYYCAR | DRAVAGNY-------- -------FYYGMDV | WGQGT TVTVS S |
| iPS:4 37042 | 21-225_197E8 | VH1\|1-02/D6\|6- | | QVQLLQS-GAEVRKPGASVRV | G-----YYIH | WVRQAPGQ GLEWMG | WINPN---SGGTNYAQR | RVTMTRDTSISTAYMELSRL RSDDTAVYYCAR | EIAVAGNY-------- -------FYYGMGV | WGQGT TVAVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH4\|4-39/D1\|1-1\|RF2/JH4 | | 32238 | QLQLQES-GPGLVKPSETLSLTCTVSG-GSIS | SS---SYYWG | WIRQPPGKGLEWIG | SIYY----SGSTYYNPSLKS | RVTISVDTSKNQFSLKLSSVTAADTAVYYCAR | VQLER---------------------YFDY | WGQGTLVTVSS |
| iPS:468868 | 21-225_74A1 | VH4\|4-39/D1\|1-1\|RF2/JH4 | | QLQLQES-GPGLVKPSETLSLTCTVSG-GSIS | GS---SYYWG | WIRQPPGKGLEWIG | NIYY----SGSTYHNPSLKS | RVTISVDTSKNQFSLKLTSVTAADTAVFYCAR | HDLLW---------------------SLDF | WGQGILVTVSS |
| | | Germline | | H_FR1 | H_CDR1 | H_FR2 | H_CDR2 | H_FR3 | H_CDR3 | H_FR4 |
| | VH1\|1-02/D3\|3-22\|RF2/JH5 | | 32239 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | YYYDSSGY---------------YYNWFDP | WGQGTLVTVSS |
| iPS:472742 | 21-225_30D9_LC2 | VH1\|1-02/D3\|3-22\|RF2/JH5 | | QVKLVQS-GAEVEKPGASVKVSCKASG-YTFT | G-----YYLH | WVRQAPGQGLEWMG | WINPN---SGGTNYAQKFQG | RVTMTRDTSISTAYMELSRLRSDDTAVYYCAR | VYYYGSGS---------------YYNEFDN | WGQGTLVTVSS |
| iPS:472741 | 21-225_30D9_LC1 | VH1\|1-02/D3\|3-22\|RF2/JH5 | | QVKLVQS-GAEVEKPGASVKVSCKASG-YTFT | G-----YYLH | WVRQAPGQGLEWMG | WINPN---SGGTNYAQKFQG | RVTMTRDTSISTAYMELSRLRSDDTAVYYCAR | VYYYGSGS---------------YYNEFDN | WGQGTLVTVSS |
| iPS:437040 | 21-225_196E7 | VH1\|1-02/D3\|3-22\|RF2/JH5 | | QVQLVQS-GAEVKKPGASVKVSCKVSG-YTFT | G-----YNMH | WVRQAPGQGLEWMG | WINPN---SGGTNYAHKFQG | RVTMTRDTSISTAYMELSRLRSDDTAVYYCAR | DYYDTSG-----------------EGWFDP | WGQGTLVTVSS |
| iPS:437050 | 21-225_197C11 | VH1\|1-02/D3\|3-22\|RF2/JH5 | | QVQLVQS-GAEVKKPGASVKVSCKVSG-YTFT | G-----YNMH | WVRQAPGQGLEWMG | WINPN---SGGTNYAHKFQG | RVTMTRDTSISTAYMELSRLRSDDTAVYYCAR | DYYDSSG-----------------EGWFDP | WGQGTLVTVSS |
| iPS:393214 | 21-225_33A1 | VH1\|1-02/D3\|3-22\|RF2/JH5 | | QVQLVQS-GAEVKKPGASVKVSCKASG-YTFS | G-----YYMH | WVRQAPGQGLEWMG | WINPN---NGGTHYAQKFQG | RVTMTRDTSIRTASMELSRLRSDDTAVYFCAR | GYYYASGS---------------YYNDLDP | WGQGTLVTVSS |
| | | Germline | | H_FR1 | H_CDR1 | H_FR2 | H_CDR2 | H_FR3 | H_CDR3 | H_FR4 |
| | VH1\|1-02/D3\|3-22\|RF2/JH4 | | 32240 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | YYYDSSG----------------YYYYFDY | WGQGTLVTVSS |
| iPS:472743 | 21-225_68G6 | VH1\|1-02/D3\|3-22\|RF2/JH4 | | QVQLVQF-GGEVKKPGSSVKVSCKASG-YTFT | G-----YYMH | WVRQAPGQGLEWMG | SIYRN---SGGTNYAQKFQG | RVTMTRDKSISTAYMEKSRIRSDDTAVYYCAR | AFYYGSGT---------------YYNEFDY | WGQGTLVTVSS |
| iPS:436902 | 21-225_69B11 | VH1\|1-02/D3\|3-22\|RF2/JH4 | | QVQLVQS-GAEVKKPGASVRVSCKASG-YTFT | G-----YYMH | WVRQAPGQGLEWMG | WINPN---SGGTNYGQKFQD | RVTMTRDTSISTAFMELSRLRSDDTAAYYCAR | TYYYGSGS---------------YYNGFDY | WGQGTLVTVSS |
| iPS:436904 | 21-225_71D4 | VH1\|1-02/D3\|3- | | QVQLVQS-GAEVKKPGASVKV | G-----YCMH | WVRQAPGQGLEWMG | WINPN---SGGTNYAQK | RVTMTRDTSVSTVYMDLSRLRSDDTAVYYCAR | AYYYGSGT---------------YHNEFDY | WGQGSLVTVS |

| | | | | | | | | | | |
|---|---|---|---|---|---|---|---|---|---|---|
| iPS:436906 | 21-225_72B4 | VH1\|1-02/D3\|3-22\|RF2/JH4 | | QVQLVQS-GAEVKKPGASVKVSCKASG-YTFT | G-----YYMH | WVRQAPGQGLEWMG | WINPN---SGGTNYGQKFQG | RVTMTRDTSISTAYMELSRLRSDDTAVYYCAR | TYYYGSGS---------------YYNGFDY | WGQGTLVTVSS |
| iPS:437034 | 21-225_195E9 | VH1\|1-02/D3\|3-22\|RF2/JH4 | | QVQVVQS-GAEVKKPGASVKVSCKASG-YTFT | G-----YYMH | WVRQAPGQGLEWMG | WINPN---SGATNYAQKFQG | RVTMTRDTSISTAYMELNRLRSDDTAVYYCAR | AYYYGSGT---------------YYNEFDY | WGQGTLVTVSS |
| iPS:392598 | 21-225_18E10 | VH1\|1-02/D3\|3-22\|RF2/JH4 | | QVQLVQS-GAEVKKPGASVKVSCKASG-YTFT | G-----YYMH | WVRQAPGQGLEWMG | WINPN---SGGTNYAQKFQG | RVTMTRDSSINTAYMELSRLRSDDTAVYYCAR | SYYYGSGS---------------YYNEFDY | WGQGTLVTVSS |
| iPS:393182 | 21-225_4B3 | VH1\|1-02/D3\|3-22\|RF2/JH4 | | QVQLVQS-GAEVKKPGASVKVSCKASG-YTFT | G-----YYMH | WVRQAPGQGLEWMG | WINPN---SGGTNSAQKFQG | RVTMTRDTSISTAYMELSRLRSDDTAVYYCAR | SYYYGSGS---------------YYNEFDY | WGQGTLVTVSS |
| iPS:393200 | 21-225_35E1 | VH1\|1-02/D3\|3-22\|RF2/JH4 | | QVKLVQS-GAEVKKPGASVKVSCKASG-YTFT | G-----YYMH | WVRQAPGQGLEWMG | WINPK---SGGTNYAQKFQG | RVTMTRDTSISTVYMEPSRLRSDDTAVYYCAR | VYYHGSGS---------------YYNEFDY | WGQGTLVTVSS |
| iPS:393206 | 21-225_13F6 | VH1\|1-02/D3\|3-22\|RF2/JH4 | | QVQLVQS-GAEVKKPGASVKVSCKASG-YTFT | G-----YYMH | WVRQAPGQGLEWMG | WINPN---SGGANYAQKFQG | RVTMTRDTSISTAYMELSRLRSDDTAVYFCAR | SFYYGSGT---------------YYNEFDY | WGQGTLVTVSS |
| iPS:393208 | 21-225_16F3 | VH1\|1-02/D3\|3-22\|RF2/JH4 | | QVQLVQS-GAEVKKPGASVKVSCKASG-YTFT | G-----HYMH | WVRQAPGQGLEWMG | WINPN---SGGTNYAQKFQG | RVTMTRDTSISTAYMELSRLRSDDTAVYYCAR | SYYYGSGT---------------YYNEFDY | WGQGTLVTVSS |
| iPS:393210 | 21-225_17D3 | VH1\|1-02/D3\|3-22\|RF2/JH4 | | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | ANYYGSGS---------------YYNDFDY | WGQGTLVTVSS |
| iPS:393226 | 21-225_33E6 | VH1\|1-02/D3\|3-22\|RF2/JH4 | | QVKLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | VYYYGSGS---------------YYNEFDY | WGQGTLVTVSS |
| iPS:393230 | 21-225_9G9 | VH1\|1-02/D3\|3-22\|RF2/JH4 | | QVQLVQS-GAEVKKPGASVKVSCKASG-YTFT | G-----YYIH | WVRQAPGQGLEWMG | WINPN---SGGTDYAQKFQG | RVTMTRDTSISTAYMELSRLRSDDTAVYYCAR | SYYYGSGT---------------YYNEFDY | WGQGTLVTVSS |
| iPS:398490 | 21-225_21D12 | VH1\|1-02/D3\|3-22\|RF2/JH4 | | QVQLVQS-GAEVKKPGASVKVSCKASG-YTFT | D-----YYIH | WVRQAPGQGLEWMG | WINPN---SGGTNNAQKFQG | RVTMTRDTSISTAYMELSRLRSDDTAVYSCAR | SYYYGSGT---------------YYNEFDY | WGQGTLVTVSS |
| iPS:423018 | 21-225_31D12_L | VH1\|1-02/D3\|3- | | QVKLVQS-GAEVKKPGASVKV | G-----YYMH | WVRQAPGQGLEWMG | WINPN---SGGTNYVQK | RVTMTRDTSISTAYMELSRLRSDDTAVYYCAR | VYYYGSGS---------------YYNEFDY | WGQGTLVTVS |

| | | | | | | | | | | |
|---|---|---|---|---|---|---|---|---|---|---|
| iPS:423019 | 21-225_31D12_LC1 | VH1\|1-02/D3\|3-22\|RF2/JH4 | | QVKLVQS-GAEVKKPGASVKVSCKASG-YTFT | G-----YYMH | WVRQAPGQGLEWMG | WINPN---SGGTNYVQKFQG | RVTMTRDTSISTAYMELSRLRSDDTAVYYCAR | VYYYGSGS---------------YYNEFDY | WGQGTLVTVSS |
| | | Germline | | H_FR1 | H_CDR1 | H_FR2 | H_CDR2 | H_FR3 | H_CDR3 | H_FR4 |
| | VH3\|3-33/D7\|7-27\|RF2/JH4 | | 32241 & 32242 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | *LGY---------------------FDY | WGQGTLVTVSS |
| iPS:392920 | 21-225_29G4 | VH3\|3-33/D7\|7-27\|RF2/JH4 | | QVQLVES-GGGVVQPGRSLRLTCAASG-FTFS | D-----YGIH | WVRQAPGKGLEWVA | VIWYD---ESNKYYADSMKG | RFTISRDNSKNTLYLQMNSLRAEDTAVYYCAR | ELGM---------------------TGDY | WGQGTLVTVSS |
| iPS:433899 | 21-225_43C3 | VH3\|3-33/D7\|7-27\|RF2/JH4 | | QVQLVES-GGGVVQPGRSLRLSCAASG-FTFS | S-----YGIH | WVRQAPGKGLEWVA | VIWYD---ENNKYYADSVKG | RFTISRDNSKNTLYLQMNSLRAEDTAVYYCAR | ELGF---------------------SNDY | WGQGTLVTVSS |
| iPS:433921 | 21-225_44C3 | VH3\|3-33/D7\|7-27\|RF2/JH4 | | QVQLVES-GGGVVQPGRSLRLSCAASG-FTFS | S-----YGMH | WVRQAPGKGLEWVA | VIWFE---GSNKYYADSVKG | RFTISRDNSKNTLYLQMSSLRAEDTAVYYCVR | ELGF---------------------STDY | WGQGTLVTVSS |
| iPS:433933 | 21-225_44C8 | VH3\|3-33/D7\|7-27\|RF2/JH4 | | QVQLVES-GGGVVQPGRSLRLSCAASG-FTFN | N-----YGMH | WVRQAPGKGLEWVA | VIWYE---GSNKYYADSVKG | RFTISRDNSKNTLYLQMNSLRAEDTAVYYCVR | ELGF---------------------LSDY | WGQGTLVTVSS |
| iPS:433969 | 21-225_46F3 | VH3\|3-33/D7\|7-27\|RF2/JH4 | | QVQLVES-GGGVVQPGRSLRLSCAASG-FTFS | D-----YGMH | WVRQAPGKGLEWVA | VIWFE---GSNKYYADSVKG | RFTISRDNSKNTLYVQMNSLRAEDTAVYYCVR | ELGF---------------------SNDY | WGQGTLVTVSS |
| iPS:433975 | 21-225_46C6 | VH3\|3-33/D7\|7-27\|RF2/JH4 | | QVQLVES-GGGVVQPGRSLRLSCAASG-FTFS | S-----YGIH | WVRQAPGKGLEWVA | VIWYD---ENNKYYADSVKG | RFTISRDNSKNTLYLQMNSLRAEDTAVYYCAR | ELGF---------------------SNDY | WGQGTLVTVSS |
| iPS:433977 | 21-225_46D8 | VH3\|3-33/D7\|7-27\|RF2/JH4 | | QVQLVES-GGGVVQPGRSLRLSCAASG-FTFS | D-----YGIH | WVRQAPGKGLEWVA | VIWFE---GSNKYYADSVKG | RFTISRDNSKNTLYVQMNSLRAEDTAVYYCVR | ELGF---------------------SNDY | WGQGTLVTVSS |
| iPS:433983 | 21-225_47A1 | VH3\|3-33/D7\|7-27\|RF2/JH4 | | QVQLVES-GGGVVQPGRSLRLSCAASG-FTFS | D-----YGMH | WVRQAPGKGLEWVA | VIWYD---DYNKKYADSVKG | RFTISRDNAKNTLYLQVNSLRVEDTAVYYCAT | ELGM---------------------LFDY | WGQGTLVTVSS |
| iPS:433997 | 21-225_48C1 | VH3\|3-33/D7\|7-27\|RF2/JH4 | | QVQLVES-GGGVVQPGRSLRLSCAASG-FTFS | S-----YGMH | WVRQAPGKGLEWVA | VVWYD---EINKKYADSVKG | RVTISRDNSKNTLYLQMNSLRAEDTAMYYCAR | ELGW---------------------EADY | WGQGTLVTVSS |
| iPS:434009 | 21-225_48A9 | VH3\|3-33/D7\|7- | | QVQLVES-GGGVVQPGRSLRL | S-----YGMH | WVRQAPGKGLEWVA | VIWYE---ENKKYYADS | RFTISRDNSKNTLFLQMNSLRAEDTAMYFCAR | ELAW---------------------YEDY | WGQGTLVTVS |

| | | | | | | | | | |
|---|---|---|---|---|---|---|---|---|---|
| **iPS:434013** | **21-225_48D12** | **VH3\|3-33/D7\|7-27\|RF2/JH4** | | QVQLVES-GGGVVQPGRSLRLSCAASG-FTFS | D-----YGMH | WVRQAPGRGLEWVA | VIWYD---VSNKYYVDSVKG | RFTISRDNSKNTLYLQMNSLRAEDTAVYYCAR | ELGM----------------------RSDY | WGQGTLVTVSS |
| **iPS:434019** | **21-225_49A1** | **VH3\|3-33/D7\|7-27\|RF2/JH4** | | QVQLVES-GGGVVQPGRSLRLSCAASG-FTFS | D-----YGMH | WVRQAPGKGLEWVA | VIWYD---EDNKYYVDSVKG | RFTISRDNSKNTLYLQMNSLRAEDTAVYYCAR | ELGF----------------------LSDY | WGQGTLVTVSS |
| **iPS:434029** | **21-225_49C6** | **VH3\|3-33/D7\|7-27\|RF2/JH4** | | QVQLVES-GGGVVQPGRSLRLSCAASG-FTFS | N-----YGMH | WVRQAPGKGLEWVA | VIWFD---VSNKKYVDSVKG | RFTISRDNSKNTLYLQMSSLRAEDTAVYYCAR | DLGM----------------------IEDY | WGQGTLVTVSS |
| **iPS:434057** | **21-225_51E4** | **VH3\|3-33/D7\|7-27\|RF2/JH4** | | QVQLVES-GGGVVQPGRSLRLSCAASG-FTFS | S-----YGMH | WVRQAPGKGLEWVA | VIWYD---ESNKYYADSVKG | RFTISRDNSKNTLYLQMNSLRAEDTAVYYCAR | ELGF----------------------LSDY | WGQGTLVTVSS |
| **iPS:434071** | **21-225_51F9** | **VH3\|3-33/D7\|7-27\|RF2/JH4** | | QVQLVES-GGGVVQPGRSLRLSCAASG-FTFS | D-----YGMH | WVRQAPGKGLEWVA | VIWFD---GNNKYYADSVKG | RFTISRDNSKNTLYLQMNSLSAEDTAVYYCAR | ELGF----------------------LSDY | WGQGTLVTVSS |
| **iPS:434075** | **21-225_51B11** | **VH3\|3-33/D7\|7-27\|RF2/JH4** | | QVQLVES-GGGVVQPGRSLRLSCAASG-FTFS | D-----YGMH | WVRQAPGKGLEWVA | VIWFG---GNNKYYGDSVKG | RFTISRDNSKNTLYLQMNSLSAEDTAVYYCAR | ELGF----------------------LSDY | WGQGTLVTVSS |
| **iPS:434077** | **21-225_51F11** | **VH3\|3-33/D7\|7-27\|RF2/JH4** | | QVQLVES-GGGVVQPGRSLRLSCAASG-FTFS | N-----FGMH | WVRQAPGKGLEWVA | VIWYE---ESNKYYADSVKG | RFTISRDNSKNTLYLQMNSLRAEDTAVYYCAR | ELGF----------------------LSDF | WGQGTLVTVSS |
| **iPS:434081** | **21-225_52B2** | **VH3\|3-33/D7\|7-27\|RF2/JH4** | | QVQLVES-GGGVVQPGRSLRLSCAASG-FTFS | N-----YGMH | WVRQAPGKGLEWVA | VTWFD---GSNQRYADSVKG | RFTISRDISKNTLYLQMNSLSAEDTAVYYCAR | DLGM----------------------IEDF | WGQGTLVTVSS |
| **iPS:434091** | **21-225_52B9** | **VH3\|3-33/D7\|7-27\|RF2/JH4** | | QVQLVES-GGGVVQPGRSLRLSCAASG-FTFS | D-----YGMH | WVRQAPGKGLEWVA | VIWFD---GNNKYYADSVKG | RFTISRDNSKNTLYLQMNSLSAEDTAVYYCAR | ELGF----------------------LSDY | WGQGTLVTVSS |
| **iPS:434105** | **21-225_53D2** | **VH3\|3-33/D7\|7-27\|RF2/JH4** | | QVQLVES-GGGVVQPGRSLRLSCAASG-FTFS | N-----YGMH | WVRQAPGKGLEWVT | VVWDD---GSNKYYADSVKG | RFTISRDNSKNTLYLQMNSLRAEDTAVYYCTR | GLGF----------------------TGDY | WGQGALVTVSS |
| **iPS:434119** | **21-225_53E6** | **VH3\|3-33/D7\|7-27\|RF2/JH4** | | QVQLVES-GGGVVQPGRSLRLSCTTSG-FTFS | D-----YGIH | WVRQAPGKGLEWVA | VIWYD---ESNKYYGDSVKG | RFAISRDNSKNTLYLQMNSLRAEDTAVYYCVR | ELGM----------------------TSDY | WGQGTLVTVSS |
| **iPS:434129** | **21-225_53B12** | **VH3\|3-33/D7\|7-** | | QVQLVES-GGGVVQPGRSLRL | N-----FGMH | WVRQAPGKGLEWVA | VVWYD---GNNRYYADS | RFTISRDNSKNTLYLQMHSLRAEDTAVYYCAR | ELGF----------------------LSDF | WGQGTLVTVS |

| | | | | | | | | | | |
|---|---|---|---|---|---|---|---|---|---|---|
| iPS:4 34131 | 21-225_54D3 | VH3\|3-33/D7\|7-27\|RF2/J H4 | | QVQLVES-GGGVVQPGRSLRLSCAASG-FTFS | N-----YGMH | WVRQAPGKGLEWVA | VTWFD---GNNNYYADSVKG | RFTISRDNSKNTLYLQMNSLRAEDTAVYYCAR | ELGF--------------------LSDY | WGQGTLVTVSS |
| iPS:4 34141 | 21-225_54C6 | VH3\|3-33/D7\|7-27\|RF2/J H4 | | QVQLVES-GGGVVQPGRSLRLSCTTSG-FTFS | D-----YGIH | WVRQAPGKGLDWVA | VIWYD---ENNKYYADSVKG | RFAISRDNSKNTLYLQMNSLRAEDTAVYYCVR | ELGM--------------------TSDY | WGQGTLVTVSS |
| iPS:4 34143 | 21-225_54G7 | VH3\|3-33/D7\|7-27\|RF2/J H4 | | QVQLVES-GGGVVQPGRSLRLSCAASG-FTFS | N-----YGMH | WVRQAPGKGLEWVA | VIWYE---ESNKYYGDSVKG | RFTISRDNSKNTLYLQMNSLRAEDTAVYYCAR | ELGF--------------------LSDY | WGQGTLVTVSS |
| iPS:4 34155 | 21-225_55B3 | VH3\|3-33/D7\|7-27\|RF2/J H4 | | QVQLVES-GGGVVQPGRSLRLSCAASG-FTFS | S-----YGMH | WVRQAPGKGLEWVA | VIWFD---GNNKYYEDSVKG | RFTISRDNSKNTLYLQMNSLRAEDTAVYYCVR | ELGF--------------------LSDY | WGQGTLVTVSS |
| iPS:4 34199 | 21-225_59F11 | VH3\|3-33/D7\|7-27\|RF2/J H4 | | QVQLVES-GGGVVQPGRSLRLSCAASG-FTFS | N-----YGMH | WVRQAPGKGLEWVA | VIWYD---ESNKHYADSVKG | RFTISRDNSKTTLYLQMSSLRAEDTAVYYCSR | ELGM--------------------NGDY | WGQGTLVTVSS |
| iPS:4 34207 | 21-225_60A3 | VH3\|3-33/D7\|7-27\|RF2/J H4 | | QVQLVES-GGGVVQPGRSLRLSCAASG-FTFS | S-----YGMH | WVRQAPGKGLEWVA | VIWYE---ESNKYYADSVKG | RFTISRDNSKNTLYLQMNSLRAEDTAVYYCAR | ELGM--------------------TGDY | WGQGTLVTVSS |
| iPS:4 34253 | 21-225_62E4 | VH3\|3-33/D7\|7-27\|RF2/J H4 | | QVQLVES-GGGVVQPGRSLRLSCAASG-FTFS | D-----YGMH | WVRQAPGKGLEWVA | VIWYD---RSNKYYADSVKG | RFTISRDNSKNTLYLQMNSLRVEDTAVYHCAR | ELGF--------------------SSDY | WGQGTLVTVSS |
| iPS:4 34271 | 21-225_57A4 | VH3\|3-33/D7\|7-27\|RF2/J H4 | | QVQLVES-GGGVVQPGRSLRLSCVASG-FTFS | D-----YGMH | WVRQAPGKGLEWVA | VIWYA---GSNKYYVDSVKG | RFTISRDNSKNTLYLQMNSLRAEDTAVYYCAR | ELGM--------------------RSDY | WGQGTLVTVSS |
| iPS:4 34293 | 21-225_58F5 | VH3\|3-33/D7\|7-27\|RF2/J H4 | | QVQLVES-GGGVVQPGRSLRLSCVASG-FTFS | D-----YGMH | WVRQAPGKGLEWVA | VIWYA---GSNKYHVDSVKG | RFTISRDNSKNTLYLQMNSLRAEDTAVYYCAR | ELGM--------------------RSDY | WGQGTLVTVSS |
| iPS:4 34337 | 21-225_64E1 | VH3\|3-33/D7\|7-27\|RF2/J H4 | | QVQLVES-GGGVVQPGRSLRLSCVASG-FTFS | S-----YGMH | WVRQAPGKGLEWVA | VIWFD---ETNKYYGDSVKG | RFTISRDNSKNTLYLQMNSLRAEDTAVYYCAR | ELGF--------------------SSDY | WGQGTLVTVSS |
| iPS:4 34357 | 21-225_65C1 | VH3\|3-33/D7\|7-27\|RF2/J H4 | | QVQLVES-GGGVVQPGRSLRLSCAASG-FTFS | D-----YGMH | WVRQAPGKGLEWVA | VIWFE---GSNKHYTDSVKG | RFTISRDNSKNTLYLQMNSLRAEDTAVYYCAR | ELGF--------------------SSDY | WGQGTLVTVSS |
| iPS:4 34375 | 21-225_66C7 | VH3\|3-33/D7\|7- | | QVQLVES-GGGVVQPGRSLRL | D-----YGMH | WVRQAPGKGLEWVA | VIWFE---GSHKYYTDS | RFTISRDNSKNTLYLQMNSLRAEDTAVYYCAR | ELGF--------------------SSDY | WGQGTLVTVS |

| | | | | | | | | | | |
|---|---|---|---|---|---|---|---|---|---|---|
| iPS:434411 | 21-225_68F11 | VH3\|3-33/D7\|7-27\|RF2/JH4 | | QVQLVES-GGGVVQPGRSLRLSCAASG-FTFS | D-----YGMH | WVRQAPGKGLEWVA | VIWYD---VSNKYYADSVKG | RFTISRDNSKNTLYLQMNSLRAEDTAVYYCVR | ELGM----------------------TSDC | WGQGTLVTVSS |
| iPS:434441 | 21-225_71A2 | VH3\|3-33/D7\|7-27\|RF2/JH4 | | QVQLVES-GGGVVQPGRSLRLSCATSG-FTFS | D-----YGMH | WVRQAPGKGLEWVA | VIWYD---ESNKYYADSVKG | RFTISRDNSKNTLYLQMNSLRAEDTAVYYCAR | ELGW----------------------QDDY | WGQGTLVTVSS |
| iPS:434447 | 21-225_71B6 | VH3\|3-33/D7\|7-27\|RF2/JH4 | | QVQLVES-GGGVVQPGRSLRLSCAASG-FTFS | N-----YGMH | WVRQAPGKGLEWVA | VIWYD---RTNKYYADSVKG | RFTISRDNSKNTLHLQMNSLRAEDTAVYYCAR | ELGM----------------------LSDY | WGQGTLVTVSS |
| iPS:434453 | 21-225_71B11 | VH3\|3-33/D7\|7-27\|RF2/JH4 | | QVQLVES-GGGVVQPGRSLRLSCAASG-FTFS | D-----YGMH | WVRQAPGKGLEWVA | VIWYD---RNNKYYGDSVKG | RFTISRDNSKNTLYLQMNSLRAEDTAVYYCAR | ELGM----------------------LSDY | WGQGTLVTVSS |
| iPS:434457 | 21-225_72G12 | VH3\|3-33/D7\|7-27\|RF2/JH4 | | QVQLVES-GGGVVQPGRSLRLSCAASG-FTFS | S-----YGMH | WVRQAPDTGLEWVA | VIWFD---ESNKYYADSVKG | RFTISRDNSKNTLYLQMNSLRAEDTAVYYCAR | ELGF----------------------SSDY | WGQGTLVTVSS |
| iPS:435311 | 21-225_146H9 | VH3\|3-33/D7\|7-27\|RF2/JH4 | | QVQLVES-GGGVVQPGRSLRLSCAASG-FSFS | S-----YGMH | WVRQAPGKGLEWVA | VIWFD---ESNKHYGDSVKG | RFTISRDNSKNTLYLQMNSLRAEDTAVYYCAR | ELGF----------------------LSDY | WGQGTLVTVSS |
| iPS:435511 | 21-225_157C3 | VH3\|3-33/D7\|7-27\|RF2/JH4 | | QVQLVES-GGGVVQPGRSLRLSCAASG-FTFS | T-----YGMH | WVRQAPGKGLEWVA | VIWYD---VNNKYYADSVKG | RFTISRDNSKNTLYLQMNSLRAEDTAVYYCAR | ELGF----------------------LSDY | WGQGTLVTVSS |
| iPS:435533 | 21-225_157H8 | VH3\|3-33/D7\|7-27\|RF2/JH4 | | QVQLVES-GGGVVQPGRSLRLSCAASG-FTFS | S-----YGMH | WVRQAPGKGLEWVA | VIWYD---VNNKYYADSVKG | RFTISRDNSKNTLYLQMNSLRAEDTAVYYCAR | ELGF----------------------LSDY | WGQGTLVTVSS |
| iPS:435551 | 21-225_158H6 | VH3\|3-33/D7\|7-27\|RF2/JH4 | | QVQLVES-GGGVVQPGRSLRLSCAVSG-FTFS | S-----YGMH | WVRQAPGKGLEWVA | VIWYD---VTNKYYADSVKG | RFTISRDNSKNTLYLQMNSLRDEDTAVYYCVR | ELGW----------------------AEDY | WGQGTLVTVSS |
| iPS:435569 | 21-225_159C5 | VH3\|3-33/D7\|7-27\|RF2/JH4 | | QVQLVES-GGGVVQPGRSLRLSCAASG-FTFS | S-----YGIH | WVRQAPGKGLEWVA | VVWYD---VNNKYYADSVKG | RFTISRDNSKNTLYLQMNSLRAEDTAVYYCAR | ELGF----------------------LSDY | WGQGTLVTVSS |
| iPS:436268 | 21-225_203B9 | VH3\|3-33/D7\|7-27\|RF2/JH4 | | QVQLVES-GGGVVQPGRSLRLSCAASG-FTLS | S-----FGMH | WVRQAPGKGLEWVA | VIWYD---VNNKYYADSVKG | RFTISRDNSKNTLYLQMNSLRPEDTAVYYCAR | ELGF----------------------LSDY | WGQGILVTVSS |
| iPS:436328 | 21-225_207F12 | VH3\|3-33/D7\|7- | | QVQLVES-GGGVVQPGRSLRL | N-----YGMH | WVRQAPGKGLEWVA | VIWYD---RNNKYYGDS | RFTISRDNSKNTLYLQMNSLRAEDTAVYYCAR | ELGF----------------------LFDY | WGQGTLVTVS |

| | | | | | | | | | | |
|---|---|---|---|---|---|---|---|---|---|---|
| iPS:436556 | 21-225_224D10 | VH3\|3-33/D7\|7-27\|RF2/JH4 | | QVQLVES-GGGLVQPGKSLRLSCAASG-FTFS | S-----YGMH | WVRQAPGKGLEWVA | VIWYE---GSNKYYVDSVRG | RFTISRDNSKSTLYLQMNSLRAEDTAVYYCVR | ELGF----------------------QSDY | WGQGTPVTVSS |
| iPS:392618 | 21-225_16F10 | VH3\|3-33/D7\|7-27\|RF2/JH4 | | QVQLVES-GGGVVQPGRSLRLSCAASG-FTFS | D-----YGMH | WVRQAPGKGLEWVA | VIWYD---GNNKYYVDSVKG | RFTISRDNSKNTLYLQMNSLRAEDTAVYYCAR | ELAW----------------------YEDY | WGQGTLVTVSS |
| iPS:392626 | 21-225_18A5 | VH3\|3-33/D7\|7-27\|RF2/JH4 | | QVQLVES-GGGVVQPGRSLRLSYTASG-FTFS | D-----YGMH | WVRQAPGKGLEWVA | VIWFD---GSNKYYADSVKG | RFTISRDNSKNTLYLQMNSLRAEDTAVYYCAR | DLGW----------------------TEEY | WGQGTLVTVSS |
| iPS:392630 | 21-225_20E5 | VH3\|3-33/D7\|7-27\|RF2/JH4 | | QVQLVES-GGGVVQPGRSLRLSCAASG-FTFS | D-----YGMH | WVRQAPGKGLEWVA | VIWYE---ENNQYYADSVKG | RFTISRDNSKNTLYLQMNSLRAEDTAVYYCAR | ELGF----------------------RSDY | WGQGTLVTVSS |
| iPS:392640 | 21-225_18A1 | VH3\|3-33/D7\|7-27\|RF2/JH4 | | QVQLVES-GGGVVQPGRSLRLSCAVSG-FTFS | S-----YGMH | WVRQAPGKGLEWVA | VIWYE---ENNKYYVDSVKG | RFTISRDNSKSTLYLQMNSLRAEDTAVYYCVR | ELGF----------------------QSDY | WGQGTPVTVSS |
| iPS:392644 | 21-225_19E1 | VH3\|3-33/D7\|7-27\|RF2/JH4 | | QVQLVES-GGGVVQPGRSLRLSCAASG-FTFS | N-----YGMH | WVRQAPGKGLEWVA | VIWYE---ENNQYYADSVKG | RFTISRDNSKNTLYLQMNSLRAEDTAVYYCAR | ELGF----------------------RSDY | WGQGTLVTVSS |
| iPS:392654 | 21-225_17A10 | VH3\|3-33/D7\|7-27\|RF2/JH4 | | QVQLVES-GGGVVQPGRSLRLSCAASG-FTFS | N-----YGMH | WVRQAPAKGLEWVA | VIWYE---ENNQYYADSVKG | RFTISRDNSKNTLYLQMNSLRAEDTAVYYCAR | ELGF----------------------RSDY | WGQGTLVTVSS |
| iPS:392658 | 21-225_18E8 | VH3\|3-33/D7\|7-27\|RF2/JH4 | | QVQLVES-GGGVVQPGRSLRLSCAASG-FTFS | N-----YGMH | WVRQAPGKGLEWVA | VIWYE---ENNQYYADSVKG | RFTVSRDNSKNTLFLQMNSLRAEDTAVYYCAR | ELGF----------------------RSDY | WGQGTLVTVSS |
| iPS:392666 | 21-225_16F11 | VH3\|3-33/D7\|7-27\|RF2/JH4 | | QVQMVES-GGGVVQPGRSLRLSCEASG-FTFS | S-----YGMH | WVRQAPGKGLEWVA | VIWYE---ENNKYYVDSVKG | RFTISRDNSKSTLYLQMNSLRAEDTAVYYCVR | ELGF----------------------QSDY | WGQGTPVTVSS |
| iPS:392674 | 21-225_18C2 | VH3\|3-33/D7\|7-27\|RF2/JH4 | | QVQLVES-GGGVVQPGRSLRLSCVASG-FTFS | D-----YGMH | WVRQAPGKGLEWMA | VIWYD---VTNKYYADSVKG | RFTISRDNSKNTLYLQMNSLSAEDTAVYYCAR | ELGW----------------------YEDY | WGQGTLVTVSS |
| iPS:392680 | 21-225_20A7 | VH3\|3-33/D7\|7-27\|RF2/JH4 | | QVQLVES-GGGVVQPGRSLRLSCAASG-FTFS | N-----YGMH | WVRQAPGKGLEWVA | VIWYE---ENNQYYADSVKG | RFTISRDNSKNTLYLQMNSLRAEDTAIYYCAR | ELGF----------------------RSDY | WGQGTLVTVSS |
| iPS:392686 | 21-225_17C7 | VH3\|3-33/D7\|7- | | QVQLVES-GGGVVQPGKSLRL | D-----YGMH | WVRQAPGKGLEWVA | VIWFD---GSNKYYADS | RFTISRDNSKNTLYLQMNSLRAEGTAVYYCAR | DLGW----------------------TEEY | WGQGTLVTVS |

| | | | | | | | | | | |
|---|---|---|---|---|---|---|---|---|---|---|
| iPS:392690 | 21-225_18F2 | VH3\|3-33/D7\|7-27\|RF2/JH4 | | QVQLVES-GGGVVQPGRSLRLSCAASG-FTFS | D-----YGMH | WVRQAPGKGLEWVA | VIWFD---GSNKYYVDSVKG | RFTISRDNSKNTLYLQMNSLRVEDTAVYYCAR | DLGW---------------------TEEY | WGQGTLVTVSS |
| iPS:392716 | 21-225_17B5 | VH3\|3-33/D7\|7-27\|RF2/JH4 | | QVQLVES-GGGVVQPGRSLRLSCAASG-FTFS | D-----YGMH | WVRQAPGKGLEWVA | VIWYD---ESNKHYIDSVKG | RFTISRDNSKNTLYLQMNSLRAEDTAVYYCVR | ELGF---------------------RFDY | WGQGTLVTVSS |
| iPS:392732 | 21-225_17E5 | VH3\|3-33/D7\|7-27\|RF2/JH4 | | QVQLVES-GGGVVQPGRSLRLSCAASG-FTFS | D-----YGMH | WVRQAPGKGLEWVA | LIWYD---VTNKYYGDSVKG | RFTISRDNSQNTLYLQLNSLRAEDTAVYYCAR | ELGW---------------------YEDY | WGQGTLVTVSS |
| iPS:392744 | 21-225_20D5 | VH3\|3-33/D7\|7-27\|RF2/JH4 | | QVQLVES-GGGVVQPGRSLRLSCAASG-FTFS | S-----YGMH | WVRQAPGKGLEWVA | VIWYE---ENNQYYADSVKG | RFTISRDNSKNTLYLQMNSLRADDTAVYYCAR | ELGF---------------------RSDY | WGQGTLVTVSS |
| iPS:392758 | 21-225_21G11 | VH3\|3-33/D7\|7-27\|RF2/JH4 | | QVQLVES-GGGVVQPGRSLRLSCAASG-FTFS | D-----YGMH | WVRQAPGKGLEWMA | VIWYD---VTNEYYADSVKG | RFTISRDNSKNTLYLQMNSLRAEDTAVYYCAR | ELGW---------------------YEDY | WGQGTLVTVSS |
| iPS:392772 | 21-225_20E12 | VH3\|3-33/D7\|7-27\|RF2/JH4 | | QVQLVES-GGGVVQPGRSLRLSCAASG-FTFS | S-----YGMH | WVRQAPGKGLEWVA | VMWYD---ESNKHYADSVKG | RFTISRDNSRNTLYLQMNSLRAEDTAVYYCAR | ELGF---------------------RFDY | WGQGTLVTVSS |
| iPS:392790 | 21-225_20D10 | VH3\|3-33/D7\|7-27\|RF2/JH4 | | QVQLVES-GGGVVQPGRSLRLSCAASG-FTFS | D-----YGMH | WVRQAPGKGLEWVA | VIWFD---GSNKYYVDSVKG | RFTISRDDSKNTLYLQMNSLRAEDTAVYYCAR | DLGW---------------------TEEY | WGQGTLVTVSS |
| iPS:392796 | 21-225_22A4 | VH3\|3-33/D7\|7-27\|RF2/JH4 | | QVQLVES-GGGVVQPGRSLRLSCAASG-FTFS | D-----YGIH | WVRQAPGKGLEWVA | VIWFD---GSNKYYADSVKG | RITISRDNSKNTLYLQMNSLRAEDTAVYYCAR | DLGW---------------------TEEY | WGQGTLVTVSS |
| iPS:392810 | 21-225_20H12 | VH3\|3-33/D7\|7-27\|RF2/JH4 | | QVQLVES-GGGVVQPGRSLRLSCAASG-FTFS | N-----YGMH | WVRQAPGKGLEWVA | VIWYD---ENNKYYVDSVKG | RFTISRDNSKNTLYLQMNSLRAEDTAVYYCAR | ELGF---------------------RSDY | WGQGTLVTVSS |
| iPS:392832 | 21-225_21H8 | VH3\|3-33/D7\|7-27\|RF2/JH4 | | QVQLVES-GGGVVQSGRSLRLSCAASG-FTFS | D-----YGMH | WVRQAPGKGLEWVA | VIWFD---GSNKYYADSVKG | RFTISRDNSKNTLYLQMNSLRAEDTAVYYCAR | DLGW---------------------TEEY | WGQGTLVTVSS |
| iPS:392854 | 21-225_21E5 | VH3\|3-33/D7\|7-27\|RF2/JH4 | | QVQLVES-GGGVVQPGRSLRLSCSASG-FTFS | D-----YGMH | WVRQAPGKGLEWVA | VIWYD---ESNKYYADSVKG | RFTISRDNSKNTLYLQMNSLRAEDTAMYYCTR | ELGF---------------------RSDY | WGQGTLVTVSS |
| iPS:392860 | 21-225_22H8 | VH3\|3-33/D7\|7- | | QVQLVES-GGGVVQPGRSLRL | S-----YGMH | WVRQAPGKGLEWVA | VIWYD---GNNKYYADS | RFTISRDNSKNTLYLQMNSLRAEDTAVYYCAR | ELAW---------------------YEDY | WGQGTLVTVS |

<table>
<tr><td>iPS:392866</td><td>21-225_23H11</td><td>VH3|3-33/D7|7-27|RF2/JH4</td><td></td><td>QEQLVES-GGGVVQPGRSLRLSCAASG-FTFS</td><td>S-----YGMH</td><td>WVRQAPGKGLEWVA</td><td>VIWYD---ENNKYYVDSVKG</td><td>RFTISRDNSKNTLYLQMNSLRAEDTAVYYCAR</td><td>ELGF------------ ----------RSDY</td><td>WGQGTLVTVSS</td></tr>
<tr><td>iPS:392876</td><td>21-225_21F7</td><td>VH3|3-33/D7|7-27|RF2/JH4</td><td></td><td>QVQLVES-GGGVVQPGRSLRLSCAASG-FTFS</td><td>D-----YGMH</td><td>WVRQAPGKGLEWVA</td><td>VIWFD---GNNKYYVDSVKG</td><td>RFTISRDNSKNTLYLQMNSLRAEDTAVYYCAR</td><td>DLGW------------ ----------TEEY</td><td>WGQGTLVTVSS</td></tr>
<tr><td>iPS:392880</td><td>21-225_22F9</td><td>VH3|3-33/D7|7-27|RF2/JH4</td><td></td><td>QVQMVES-GGGVVQPGRSLRLSCAASG-FTFS</td><td>S-----YGMH</td><td>WVRQAPGKGLEWVA</td><td>VIWYE---ENNKDYVDSVKG</td><td>RFTISRDNSKSTLYLQMNSLRAEDTAVYYCVR</td><td>ELGF------------ ----------QSDY</td><td>WGQGTPVTVSS</td></tr>
<tr><td>iPS:392894</td><td>21-225_21G2</td><td>VH3|3-33/D7|7-27|RF2/JH4</td><td></td><td>QVQLVES-GGGVVQPGRSLRLSCAASG-FTFS</td><td>D-----YGMH</td><td>WVRQAPGKGLEWMA</td><td>VIWYD---VTNKYYADSVKG</td><td>RFTISRDNSKNTLYLEMNSLRAEDTAVYYCAR</td><td>ELGW------------ ----------YEDY</td><td>WGQGTLVTVSS</td></tr>
<tr><td>iPS:392900</td><td>21-225_22F2</td><td>VH3|3-33/D7|7-27|RF2/JH4</td><td></td><td>QVQLVES-GGGVVQPGRSLRLSCAASG-FTFS</td><td>D-----YGMH</td><td>WVRQAPGKGLEWVA</td><td>VIWYE---GSNKYYVDSVRG</td><td>RFTISRDNSKSTLYLQMNSLRAEDTAVYYCVR</td><td>ELGF------------ ----------QSDY</td><td>WGQGTPVTVSS</td></tr>
<tr><td>iPS:392908</td><td>21-225_23F12</td><td>VH3|3-33/D7|7-27|RF2/JH4</td><td></td><td>QVQLVES-GGGVVQPGRSLRLSCVASG-FTFS</td><td>S-----YGMH</td><td>WVRQAPGKGLEWVA</td><td>VIWYD---ETNKYYADSVKG</td><td>RFTISRDNSKNTLYLQMNSLRAEDTAIYYCAR</td><td>ELAW------------ ----------YEDY</td><td>WGQGSLVTVSS</td></tr>
<tr><td>iPS:392918</td><td>21-225_28F5</td><td>VH3|3-33/D7|7-27|RF2/JH4</td><td></td><td>QVQLVES-GGGVVQPGRSLRLSCAASG-FTFS</td><td>S-----YGMH</td><td>WVRQAPGKGLEWVA</td><td>VIWYD---ENNKYYADSVKG</td><td>RFTISRDNSKNTLYLQMNSLRAEDTAVYYCAR</td><td>ELGW------------ ----------YDDY</td><td>WGQGTLVTVSS</td></tr>
<tr><td>iPS:392934</td><td>21-225_27D5</td><td>VH3|3-33/D7|7-27|RF2/JH4</td><td></td><td>QVQLVES-GGGVVQPGRSLRLSCAASG-FTFS</td><td>S-----YGMH</td><td>WVRQAPGKGLEWVA</td><td>VIWYD---ESNKYYGDSVKG</td><td>RFTISRDNSKNMLYLQMNSLRGEDTALYYCAR</td><td>ELGF------------ ----------LSDY</td><td>WGQGTLVTVSS</td></tr>
<tr><td>iPS:392958</td><td>21-225_28C7</td><td>VH3|3-33/D7|7-27|RF2/JH4</td><td></td><td>QVQLVES-GGGVVQPGRSLRLSCAASG-FTFS</td><td>S-----YGMH</td><td>WVRQAPGKGLEWVA</td><td>VIWYD---ESNKYYADSVKG</td><td>RFTISRDNSKNTLYLQMNSLRAEDTAVYYCAR</td><td>ELGW------------ ----------YDDY</td><td>WGQGTLVTVSS</td></tr>
<tr><td>iPS:392968</td><td>21-225_25B6</td><td>VH3|3-33/D7|7-27|RF2/JH4</td><td></td><td>QVQLVES-GGGVVQPGRSLRVSCTASG-FTLR</td><td>N-----YGMH</td><td>WVRQAPGKGLEWVA</td><td>VIWYE---ESNKYYTESVKG</td><td>RFTISRDNSKNTLYLQMNSLRAEDTAVYYCAR</td><td>ELGF------------ ----------LSDY</td><td>WGQGTLVTVSS</td></tr>
<tr><td>iPS:392972</td><td>21-225_26A2</td><td>VH3|3-33/D7|7-27|RF2/JH4</td><td></td><td>QVQLVES-GGGVVQPGRSLRLSCAASG-FTFS</td><td>S-----YGMH</td><td>WVRQAPGKGLEWVA</td><td>VIWYE---GSNKYYVDSVKG</td><td>RFTISRDNSKNTLYLQMNSLRAEDTAVYYCAR</td><td>ELGW------------ ----------YDDY</td><td>WGQGTLVTVSS</td></tr>
<tr><td>iPS:392980</td><td>21-225_29H6</td><td>VH3|3-33/D7|7-</td><td></td><td>QVQLVES-GGGVVQPGRSLRL</td><td>D-----YGMH</td><td>WVRQAPGKGLEWVT</td><td>VIWYN---ENNKYYADS</td><td>RFTISRDNSKNTLYLQMNSLRAEDTAVYYCAR</td><td>ELGM------------ ----------TGDS</td><td>WGQGTLVTVS</td></tr>
</table>

| | | | | | | | | | | |
|---|---|---|---|---|---|---|---|---|---|---|
| **iPS:392988** | **21-225_25E6** | **VH3\|3-33/D7\|7-27\|RF2/JH4** | | QVQLVES-GGGVVQPGRSLRLSCAASG-FTFS | D-----YGMH | WVRQAPGKGLEWVA | VIWYD---ENNKYYADSVKG | RFTISRDNSKNTLYLQMNSLRAEDTALYYCAT | ELGM----------------------TGDS | WGQGTLVTVSS |
| **iPS:392990** | **21-225_25H10** | **VH3\|3-33/D7\|7-27\|RF2/JH4** | | QVQLVES-GGGVVQPGRSLRLSCAASG-FTFS | D-----YGMH | WVRQAPGKGLEWVA | VIWYD---ESNKYYADSMKG | RFTISRDNSKNTLYLQMNSLRAEDTAVYYCAR | ELGM----------------------TGDY | WGQGTLVTVSS |
| **iPS:393000** | **21-225_29D7** | **VH3\|3-33/D7\|7-27\|RF2/JH4** | | QVQLVES-GGGVVQPGRSLRLSCAASG-FTFS | S-----YGMH | WVRQAPGKGLEWVA | VIWYD---ESNKYYGDSVKG | RFTISRDNSKNTLYLQMNSLRGEDTALYYCAR | ELGF----------------------LSDY | WGQGTLVTVSS |
| **iPS:393018** | **21-225_29B8** | **VH3\|3-33/D7\|7-27\|RF2/JH4** | | QVQLVES-GGGVVQPGRSLRLSCAASG-FTFS | D-----YGMH | WVRQAPGKGLEWVA | VIWYD---ENNKYYADSVKG | RFTISRDNSKNTLYLQMNSLRAEDTAVYYCAR | ELGM----------------------TGDS | WGQGTLVTVSS |
| **iPS:393030** | **21-225_25H11** | **VH3\|3-33/D7\|7-27\|RF2/JH4** | | QVQLVES-GGGVVQPGRSLRLSCAASG-FTFS | D-----YGMH | WVRQAPGKGLEWVA | VIWYD---ENNEYYADSVKG | RFTISRDNSKNTLYLQMNSLRAEDTAVYYCAR | ELGM----------------------TGDS | WGQGTLVTVSS |
| **iPS:393034** | **21-225_27F2** | **VH3\|3-33/D7\|7-27\|RF2/JH4** | | QEQLVES-GGGVVQPGRSLRLSCAASG-FIFS | D-----YGMH | WVRQAPGKGLEWVA | VIWYD---ENNKYYVDSVRG | RFTISRDNSKNTLYLQMNSLRAEDTAVYYCAR | ELGM----------------------TGDS | WGQGTLVTVSS |
| **iPS:393048** | **21-225_27C3** | **VH3\|3-33/D7\|7-27\|RF2/JH4** | | QVQLVES-GGGVVQPGRSLRLSCAASG-FTFS | D-----YGMH | WVRQAPGKGLEWVA | VIWYD---ENNKSYADSVKG | RFTISRDNSKNTLYLQMNSLRAEDTAVYYCAR | ELGM----------------------TGDS | WGQGTLVTVSS |
| **iPS:393054** | **21-225_29G8** | **VH3\|3-33/D7\|7-27\|RF2/JH4** | | QVQLVES-GGGVVQPGRSLRLSCAASG-FTFS | D-----YGMH | WVRQAPGKGLEWVA | VIWYD---ETNKYYADSVKG | RFTISRDNSKNTLYLQMNSLRAEDTAVYYCAR | ELGM----------------------TSDY | WGQGTLVTVSS |
| **iPS:393812** | **21-225_6A11** | **VH3\|3-33/D7\|7-27\|RF2/JH4** | | QVQLVES-GGGVVQPGRSLRLSCAASG-FTFS | D-----YGMH | WVRQAPGKGLEWVA | VIWFD---GSNKYYADSVKG | RFTISRDNSKNTLYLQMNSLRAEDTAVYDCAR | DLGW----------------------TEEY | WGQGTLVTVSS |
| **iPS:393818** | **21-225_6G12** | **VH3\|3-33/D7\|7-27\|RF2/JH4** | | QVQLVES-GGGVVQPGRSLRLSCAASG-FTFS | S-----YGMH | WVRQAPGKGLEWVA | VIWYD---RSNNYYADSVKG | RFTISRDNSKNTLYLQMNSLRAEDTAVYYCAR | ELGF----------------------RSDY | WGQGTLVTVSS |
| **iPS:393820** | **21-225_8H7** | **VH3\|3-33/D7\|7-27\|RF2/JH4** | | QVQLVES-GGGVVQPGRSLRLSCPASG-FTFS | D-----FGMH | WVRQAPGKGLEWVA | VIWYE---ENNQYYADSVKG | RFTISRDNSKNTLYLQMNSLRAEDTAVYYCAR | ELGF----------------------RSDY | WGQGTLVTVSS |
| **iPS:393826** | **21-225_10G5** | **VH3\|3-33/D7\|7-** | | QVQLVES-GGGVVQPGRSLRL | N-----YGMH | WVRQAPGKGLEWVA | VIWYE---ENNQYYADS | RFTISRDNSKNTLYLQMNSLRAEDTAVYYCTR | ELGF----------------------RSDY | WGQGTLVTVS |

| | | | | | | | | | | |
|---|---|---|---|---|---|---|---|---|---|---|
| **iPS:393828** | **21-225_10H12** | **VH3\|3-33/D7\|7-27\|RF2/JH4** | | QVQLVES-GGGVVQPGRSLRLSCAASG-FTFS | D-----YGMH | WVRQAPGKGLEWVA | VIWYE---DNNQYYADSVKG | RFTISRDNSKNTLYLQMNSLRAEDTAVYYCAR | ELGF---------------------RSDY | WGQGTLVTVSS |
| **iPS:393830** | **21-225_12A1** | **VH3\|3-33/D7\|7-27\|RF2/JH4** | | QVQLVES-GGGVVQPGRSLRLSCAASG-FTFS | S-----YGMH | WVRQAPGKGLEWVA | VIWYE---ENNQYYADSVKG | RFTISRDNSKNTLYLQMNSLRAEDTAVYYCAR | ELGF---------------------RSDY | WGQGTLVTVSS |
| **iPS:393838** | **21-225_6G2** | **VH3\|3-33/D7\|7-27\|RF2/JH4** | | LVQLVES-GGGVVQPGKSLRLSCAASG-FTFS | D-----YGIH | WVRQAPGKGLEWVA | VIWFD---GSNKYYADSVKG | RFTISSDNSKNTLYLQMNSLRAEDTAVYYCAR | DLGW---------------------TEEY | WGQGTLVTVSS |
| **iPS:393854** | **21-225_7H11** | **VH3\|3-33/D7\|7-27\|RF2/JH4** | | QVQLVES-GGGVVQPGRSLRLSCSASG-FTFS | D-----YGMH | WVRQAPGKGLEWVA | VIWYD---ENNKYYADSVKG | RFTISRDNSKNTLYLQMNSLRAEDTAVYYCAR | ELGF---------------------RSDY | WGQGTLVTVSS |
| **iPS:393866** | **21-225_14E3** | **VH3\|3-33/D7\|7-27\|RF2/JH4** | | QVQLVES-GGGVVQPGRSLRLSCAASG-FTFS | D-----FGMH | WVRQAPGKGLEWVA | VIWYE---ENNQYYADSVKG | RFTISRDNSKNTLYLQMNSLRAEDTAFYYCAR | ELGF---------------------RSDY | WGQGTLVTVSS |
| **iPS:393876** | **21-225_9A1** | **VH3\|3-33/D7\|7-27\|RF2/JH4** | | QVQVVES-GGGVVQPGRSLRLSCAASG-FTFS | D-----YGMH | WVRQAPGKGLEWVA | VIWFD---GSNKYYADSVKG | RFTISRDNSKNTLYLQMNSLRAEDTAVYYCAR | DLGW---------------------TEEY | WGQGTPVTVSS |
| **iPS:393882** | **21-225_15E3** | **VH3\|3-33/D7\|7-27\|RF2/JH4** | | QVQLVES-GGTVVQPGRSLRLSCAASG-FTFS | S-----YGMH | WVRQAPGKGLEWVA | VIWYE---ENNKHYADSVKG | RFTISRDNSKNTLYLQMNSLRAEDTAVYYCAR | ELGF---------------------LSDY | WGQGTLVTVSS |
| **iPS:393884** | **21-225_16F4** | **VH3\|3-33/D7\|7-27\|RF2/JH4** | | QVQLVES-GGGVVQPGRSLRLSCAASG-FTFS | N-----YGMH | WVRQAPGKGLEWVA | VIWYE---GSNQYYGDSVKG | RFTISRDNSKNTVYLQMHSLRAEDTAVYYCAR | ELGF---------------------LSDY | WGQGTLVTVSS |
| **iPS:393912** | **21-225_16F6** | **VH3\|3-33/D7\|7-27\|RF2/JH4** | | QVQLVES-GGGVVQPGRSLRLSCAASG-FTFS | N-----YGMH | RVRQAPGKGLEWVA | VIWYE---GSNQYYGDSVKG | RFTISRDNSKNTVYLQMHSLRAEDTAVYYCAR | ELGF---------------------LSDY | WGQGTLVTVSS |
| **iPS:393922** | **21-225_2B2** | **VH3\|3-33/D7\|7-27\|RF2/JH4** | | QVQLVES-GGGVVQPGRSLRLSCAASG-FTFS | S-----YGMH | WVRQAPGKGLEWVA | VIWYE---ENNKYYVDSVKG | RFTISRDNSKSTLYLQMNSLRAEDTAVYYCVR | ELGF---------------------QSDY | WGQGTPVTVSS |
| **iPS:393934** | **21-225_13E6** | **VH3\|3-33/D7\|7-27\|RF2/JH4** | | QVQLVES-GGGVVQPGRSLRLSCAASG-FTFS | N-----YGMH | WVRQAPGKGLEWVA | VIWYD---ENNKYYIDSVKG | RFTISRDNSKNTLYLQMNSLRAEDTAVYYCAR | ELGF---------------------RSDY | WGQGTLVTVSS |
| **iPS:393948** | **21-225_16A5** | **VH3\|3-33/D7\|7-** | | QVQLVES-GGGVVQPGRSLRL | D-----YGMH | WVRQAPGKGLEWVA | VIWFD---GSNKYYVDS | RFTISRDNSKNTLYLQMSSLRAEDTAVYYCAR | DLGW---------------------TEEY | WGQGTLVTVS |

| | | | | | | | | | | |
|---|---|---|---|---|---|---|---|---|---|---|
| iPS:3 93960 | 21-225_7G2 | VH3\|3-33/D7\|7-27\|RF2/JH4 | | QVQLVES-GGGVVQPGRSLRLSCAASG-FTFS | D-----YGMH | WVRQAPGKGLEWMA | VIWYD---VTNKYYADSVKG | RFTISRDNSKNTLYLQLNSLRAEDTAVYYCAR | ELGW-----------------------YEDY | WGQGTLVTVSS |
| iPS:3 93974 | 21-225_7C4 | VH3\|3-33/D7\|7-27\|RF2/JH4 | | QVQLVES-GGGVVQPGKSLRLSCAASG-FTFS | N-----YGMH | WVRQAPGKGLEWVA | VIWYE---ENNQYYADSVKG | RFTISRDNSKNTLYLQMNSLRAEDTAVYYCAR | ELGF-----------------------RSDY | WGQGTLVTVSS |
| iPS:3 93976 | 21-225_7E9 | VH3\|3-33/D7\|7-27\|RF2/JH4 | | QVQLVES-GGGVVQPGRSLRLSCAASG-FTFS | D-----YGMH | WVRQAPGKGLEWVA | VIWYD---ENNKYYVDSVKG | RFTISRDNSKNTLYLQMNSLRAEDTAVYYCAR | ELGF-----------------------RSDY | WGQGTLVTVSS |
| iPS:3 93994 | 21-225_8C9 | VH3\|3-33/D7\|7-27\|RF2/JH4 | | QVQLVES-GGGVVQPGRSLRLSCAASG-FTFS | S-----YGMH | WVRQAPGKGLEWVA | VIWYD---ENNKYYVDSVKG | RFTISRDNSKNTLYLQMNSLRAEDTAVYYCAR | ELGF-----------------------RSDY | WGQGTLVTVSS |
| iPS:3 93998 | 21-225_12B12 | VH3\|3-33/D7\|7-27\|RF2/JH4 | | QVQLVES-GGGVVQPGRSLRLSCAASG-FTFS | D-----YGMH | WVRQAPGKGLEWMA | VIWYD---VTNKYYADSVKG | RFTISRDNSKNTLYLQMNSLRAEDTAVYYCAR | ELGW-----------------------YEDY | WGQGTLVTVSS |
| iPS:3 94024 | 21-225_16B7 | VH3\|3-33/D7\|7-27\|RF2/JH4 | | QVQLVES-GGGVVQPGRSLRLSCAASG-FTFS | S-----YGMH | WVRQAPGKGLEWVA | VIWYD---ESNKYYADSVKG | RFTISRDNSKNTLYLQMNSLRAEDTAVYYCAR | ELGF-----------------------LSDY | WGQGTLVTVSS |
| iPS:3 94059 | 21-225_9E8 | VH3\|3-33/D7\|7-27\|RF2/JH4 | | QVQLVES-GGGVVQPGRSLRLSCEASG-FTFS | S-----YGMH | WVRQAPGKGLEWVA | VIWYE---ENNQYYADSVKG | RFTISRDNSKNTLYLQMNSLRAEDTAVYYCAR | ELGF-----------------------RSDY | WGQGTLVTVSS |
| iPS:3 94067 | 21-225_12F2 | VH3\|3-33/D7\|7-27\|RF2/JH4 | | QVQLVES-GGGVVQPGRSLRLSCAASG-FTFS | D-----YGMH | WVRQAPGKGLEWVA | VIWFD---GNNKYYVDSVKG | RFTISRDNSKNTLFLQMNSLRAEDTAVYYCVR | ELAW-----------------------SEDY | WGQGTLVTVSS |
| iPS:3 94089 | 21-225_12E6 | VH3\|3-33/D7\|7-27\|RF2/JH4 | | QVQLVES-GGGVVQPGRSLRLSCAASG-FTFS | D-----YGMH | WVRQAPGKGLEWVT | VIWYD---ESNKYYADSVKG | RFTISRDDSKNTLYLQMNSLRAEDTAVYYCAR | ELAW-----------------------YEDY | WGQGTLVTVSS |
| iPS:3 94097 | 21-225_16G7 | VH3\|3-33/D7\|7-27\|RF2/JH4 | | QVQLVES-GGGVVQPGRSLRLSCAASG-FTFT | D-----YGMH | WVRQAPGKGLEWVA | VIWYD---ENNEYYADSVKG | RFTISRANSKNTLYLQMNSLRAEDTAVYYCAR | ELAW-----------------------YEDY | WGQGTLVTVSS |
| iPS:4 02219 | 21-225_1C12 | VH3\|3-33/D7\|7-27\|RF2/JH4 | | QVQLVES-GGGVVQPGRSLRLSCAASG-FTFS | N-----YGMH | WVRQAPGKGLEWVA | VIWYD---ENNKYYVDSVKG | RFTISRDNSKNTLYLQMNSLRAEDTAVYYCAR | ELGF-----------------------RSDY | WGQGTLVTVSS |
| | | Germline | | H_FR1 | H_CDR1 | H_FR2 | H_CDR2 | H_FR3 | H_CDR3 | H_FR4 |
| | VH3\|3-21/D4\|4- | | 32243 | EVQLVES- | S-----YSMN | WVRQAPGK | SISSS--- | RFTISRDNAKNSLYLQMNSL | DYSNY----------- | WGQGT |

| | | | | | | | | | | |
|---|---|---|---|---|---|---|---|---|---|---|
| iPS:4 33895 | 21-225_43E1 | VH3\|3-21/D4\|4-11\|RF2/J H4 | | EVQLVES- GGGLVKPGGSLRL SCAASG-FTFS | S-----YSMN | WVRQAPGK GLEWVS | AISGN--- STYIYYADS LKG | RFTISRDNAKNSLFLQLNSL RAEDTAVYYCAR | DRG------------- ------------SE | WGQGT LVTVS S |
| iPS:4 34103 | 21-225_53G1 | VH3\|3-21/D4\|4-11\|RF2/J H4 | | EVQLVES- GGGLVKPGESLRL SCAASG-FTFS | S-----YSMN | WVRQAPGK GLEWVS | SISGS--- SSYIYYADS VKG | RFTISRDNAKNSLYLQMNSL RAEDTAVYYCAR | DRG------------- ------------ST | WGQGT LVTVS S |
| iPS:4 34179 | 21-225_56F1 | VH3\|3-21/D4\|4-11\|RF2/J H4 | | EVQLVES- GGGLVKFGGSLRL SCAASG-FTFS | S-----YSMN | WVRQAPGK GLEWVS | SISSS--- STYIYYGDS VKG | RFTISRDNAKNSLYLQMNSL RAEDTAVYYCAR | DRG------------- ------------SS | WGQGT LVTVS S |
| iPS:4 34263 | 21-225_56H7 | VH3\|3-21/D4\|4-11\|RF2/J H4 | | EVQLVES- GGGLVKPGGSLRL SCAASG-FSFS | S-----YSMN | WVRQAPGK GLEWVS | SISSS--- STYIYYGDS VKG | RFTISRDNAKNSLYLQMNSL RAEDTAVYYCAR | DRG------------- ------------SS | WGQGT LVTVS S |
| iPS:4 35521 | 21-225_157H4 | VH3\|3-21/D4\|4-11\|RF2/J H4 | | EVQLVES- GGGLVKPGGSLRL SCAASG-FTFS | S-----YSMN | WVRQAPGK GLEWVS | SISSS--- STYIYYADS VKG | RFTISRDNAKNSLYLQMNSL RAEDTAVYYCAR | DRG------------- ------------SI | WGQGT LVTVS S |
| iPS:4 35527 | 21-225_157G7 | VH3\|3-21/D4\|4-11\|RF2/J H4 | | EVQLVES- GGGLVKPGGSLRF SCAASG-FTFS | S-----YSMN | WVRQAPGK GLEWVS | SISGS--- STYIYYADS VKG | RFTISRDNAKNSLYLQMNSL RAEDTAVYYCAR | DRG------------- ------------SS | WGQGT LVTVS S |
| iPS:4 35529 | 21-225_157H7 | VH3\|3-21/D4\|4-11\|RF2/J H4 | | EVQLVES- GGGLVKPGGSLRL SCAASG-FTFS | S-----YSMN | WVRQAPGK GLEWVS | CISGS--- SSYIYYADS VKG | RFTMSRDNAKNSLYLQMNSL RAEDTAVYYCVR | DRG------------- ------------GY | WGQGT LVTVS S |
| iPS:4 35547 | 21-225_158F5 | VH3\|3-21/D4\|4-11\|RF2/J H4 | | EVQLVES- GGGLVKPGGSLRF SCAASG-FTFS | S-----YSMN | WVRQAPGK GLEWVS | SISGS--- STYIYYADS VKG | RFTISRDNAKNSLYLQMNSL RAEDTAVYYCAR | DRG------------- ------------SS | WGQGT LVTVS S |
| iPS:4 35549 | 21-225_158H5 | VH3\|3-21/D4\|4-11\|RF2/J H4 | | EVQLVES- GGGLVKPGGSLRL SCAASG-FTFS | S-----YSMN | WVRQAPGK GLEWVS | SISGS--- STYIYYADS VKG | RFTISRDNAKNSLHLQMNSL RAEDTAVYYCAR | DRG------------- ------------SS | WGQGT LVTVS S |
| iPS:4 35553 | 21-225_158G8 | VH3\|3-21/D4\|4-11\|RF2/J H4 | | EVQLVES- GGGLVTPGGSLRL SCAASG-FTFS | S-----YTMN | WVRQAPGK GLEWVS | LISGS--- SSYIYYADS VKG | RFTISRDNAKNSLYLQMNSL RAEDTAVYYCAR | DRG------------- ------------SL | WGQGT LVTVS S |
| iPS:4 35581 | 21-225_160H1 | VH3\|3-21/D4\|4-11\|RF2/J H4 | | EVQLVES- GGGLVKPGGSLRL SCAASG-FTFS | S-----YRMN | WVRQAPGK GLEWVS | SISSS--- TGYMYYADS VKG | RFTISRDNAKNSLYLQMNSL RAEDTAVYFCAR | DK-------------- ------------DY | WGQGT LVTVS S |
| iPS:4 35593 | 21-225_160F4 | VH3\|3-21/D4\|4- | | EVQLVES- GGGLVKPGGSLRF | S-----YSMN | WVRQAPGK GLEWVS | SISGS--- STYIYYADS | RFTISRDNAKNSLYLQMNSL RAEDTAVYYCAR | DRG------------- ------------SS | WGQGT LVTVS |

| | | | | | | | | | | |
|---|---|---|---|---|---|---|---|---|---|---|
| iPS:435617 | 21-225_162F2 | VH3\|3-21/D4\|4-11\|RF2/JH4 | | EVQLVES-GGGLVKPGGSLRFSCAASG-FTFS | S-----YSMN | WVRQAPGKGLEWVS | SISGS---STYIYYADSVKG | RFTISRDNAKNSLYLQMNSLRAEDTAVYYCAR | DRG-------------------------SS | WGQGTLVTVSS |
| iPS:435621 | 21-225_162H3 | VH3\|3-21/D4\|4-11\|RF2/JH4 | | EVQLVES-GGGLVKPGGSLRFSCAASG-FTFS | S-----YSMN | RVRQAPGKGLEWVS | SISGS---STYIYYADSVKG | RFTISRDNAKNSLYLQMNSLRAEDTAVYYCAR | DRG-------------------------SS | WGQGTLVTVSS |
| iPS:435641 | 21-225_163F9 | VH3\|3-21/D4\|4-11\|RF2/JH4 | | EVQLVES-GGGLVQPGGSLRLSCAASG-FTFS | S-----YSMN | WVRQAPGKGLEWVS | SISGS---STYIYYADSVKG | RFTISRDNAKNSLYLQMNSLRAEDTAVYYCAR | DRG-------------------------SL | WGQGTLVTVSS |
| iPS:435719 | 21-225_171A11 | VH3\|3-21/D4\|4-11\|RF2/JH4 | | EVQLVES-GGGLVKPGGSLRLSCAASG-FTFS | S-----YSMN | WVRQAPGKGLEWVS | SISGS---SSYIYYADSVKG | RFTISRDNAKNSLYLQMNSLRAEDTAVYYCAR | DRG-------------------------SS | WGQGILVTVSS |
| iPS:436856 | 21-225_58C5 | VH3\|3-21/D4\|4-11\|RF2/JH4 | | EVQLVES-GGGLVKPGGSLRLSCAASG-FTFN | S-----YSMN | WVRQAPGKGLEWIS | SISSS---SYYLYYADSVKG | RFTISRDSAKNSLYLQMNSLRAEDTAVYYCAR | TYSG----------------------SFDY | WGQGTLVTVSS |
| iPS:448904 | 21-225_65C12 | VH3\|3-21/D4\|4-11\|RF2/JH4 | | EVQLVES-GGGLVKPGGSLRLSCAASG-FTFR | S-----FSLN | WVRQAPGKGLEWVS | SISSS---SSYIYYADSVKG | RFTISRDNAKNSLYLQMNSLRAEDTAVYYCAR | DAY------------------------SHY | WGQGTLVTVSS |
| iPS:472730 | 21-225_14B1_LC1 | VH3\|3-21/D4\|4-11\|RF2/JH4 | | EVQLVES-GGGLVKPGGSLRLSCAASG-FTFS | S-----YTMN | WVRQAPGKGLEWVS | SISGS---SSYLYYPDSVKG | RFTISRDNAKNSLYLQMNSLRAEDTAVYYCAR | DRG-------------------------SS | WGQGTLVTVSS |
| iPS:472731 | 21-225_14B1_LC2 | VH3\|3-21/D4\|4-11\|RF2/JH4 | | EVQLVES-GGGLVKPGGSLRLSCAASG-FTFS | S-----YTMN | WVRQAPGKGLEWVS | SISGS---SSYLYYPDSVKG | RFTISRDNAKNSLYLQMNSLRAEDTAVYYCAR | DRG-------------------------SS | WGQGTLVTVSS |
| iPS:392726 | 21-225_20B5 | VH3\|3-21/D4\|4-11\|RF2/JH4 | | EVQLVEA-GGGLVKPGGSLRLSCAASG-FTFT | S-----YSMN | WVRQAPGKGLEWVS | SISGS---SSYIYYADSVKG | RFTISRDNAKNSLYLQMNSLRAEDTAVYYCAR | DRG-------------------------SY | WGQGTLVTVSS |
| iPS:392734 | 21-225_17D8 | VH3\|3-21/D4\|4-11\|RF2/JH4 | | EVQLVES-GGGLVKPGGSLRLSCAASG-FTFS | S-----YGLN | WVRQAPGKGLEWVS | SISGS---GSHISYADSVKG | RFTISRDNAKNSLYLQLNSLRAEDTAVYYCAR | DRG-------------------------SG | WGQGTLVTVSS |
| iPS:392768 | 21-225_20B8 | VH3\|3-21/D4\|4-11\|RF2/JH4 | | EVQLVES-GGGLVKPGGSLRLSCAASG-FTFS | S-----YSMN | WVRQAPGKGLEWVS | SISGS---GSHIYYADSVKG | RFTISRDNAKNSLYLQMNSLRAEDTAVYYCAR | DRG-------------------------SG | WGQGTLVTVSS |
| iPS:392778 | 21-225_22H3 | VH3\|3-21/D4\|4- | | AVQLVES-GGGLVKPGGSLRL | S-----YSMN | WVRQAPGKGLEWVS | SISSS---SSYIYYADS | RFTISRDNAKNSLYLQMNSLRAEDTAVFYCAR | DRG-------------------------SL | WGQGTLVTVS |

| | | | | | | | | | | |
|---|---|---|---|---|---|---|---|---|---|---|
| **iPS:3 92788** | **21-225_20C8** | **VH3\|3-21/D4\|4-11\|RF2/J H4** | | EVQLVES-GGGLVKPGGSLRLSCAASG-FTFS | S-----YSMN | WVRQAPGKGLEWVS | SISGS---SSYIYYADSVKA | RFTISRDNAKNSLYLQMNSLRAEDTAVYYCAR | DRG-------------------------SL | WGQGTLVTVSS |
| **iPS:3 92792** | **21-225_20G12** | **VH3\|3-21/D4\|4-11\|RF2/J H4** | | EVQLVES-GGGLVKPGGSLRLSCAASG-FTFS | S-----YSMN | WVRQAPGKGLEWVS | SISGS---SSYIYYADSLKG | RFTISRDNAKNSLYLQMNSLRAEDTAVYYCAR | DRG-------------------------SY | WGQGTLVTVSS |
| **iPS:3 92844** | **21-225_23E11** | **VH3\|3-21/D4\|4-11\|RF2/J H4** | | EVQLVES-GGGLVKPGGSLRLSCAASG-FTFS | S-----YTMN | WVRQAPGKGLEWVS | SISGS---SSYIWYVDSVKG | RFTISRDNAKNSLYLQMNSLRAEDTAVYYCAR | DRG-------------------------SL | WGQGTLVTVSS |
| **iPS:3 92848** | **21-225_20F9** | **VH3\|3-21/D4\|4-11\|RF2/J H4** | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | DRG-------------------------SC | WGQGTLVTISS |
| **iPS:3 92850** | **21-225_20H10** | **VH3\|3-21/D4\|4-11\|RF2/J H4** | | EVQLVES-GGGLVKPGGSLRLSCAASG-FTFS | T-----YSMN | WVRQAPGKGLEWVS | SISSS---SSYIYYADSVKG | RFTISRDNAKNSLYLQMNSLRAEDTAIYYCAR | DRG-------------------------SL | WGQGTLVTVSS |
| **iPS:3 93006** | **21-225_31G9** | **VH3\|3-21/D4\|4-11\|RF2/J H4** | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | DRG-------------------------SS | WGQGTLVTVSS |
| **iPS:3 93022** | **21-225_30H11** | **VH3\|3-21/D4\|4-11\|RF2/J H4** | | EVQLVES-GGGLVKPGGSLRLSCAASG-FTFS | S-----YSMN | WVRQAPGKGLEWVS | SISGS---SSYIYYADSVKG | RFTISRDNAKNSLYLQMNSLRAEDTAVYYCAR | DRG-------------------------SL | WGQGTLVTVSS |
| **iPS:3 93130** | **21-225_33C2** | **VH3\|3-21/D4\|4-11\|RF2/J H4** | | EVQLVES-GGGLVKPGGSLRLSCAASG-FTFS | S-----YTMN | WVRQAPGKGLQWVS | SISGS---SSYIYYADSVKG | RFTISRDNAKNSLFLQMNSLRAEDTAIFYCAR | DRG-------------------------GT | WGQGTLVTVSS |
| **iPS:3 93906** | **21-225_13D3** | **VH3\|3-21/D4\|4-11\|RF2/J H4** | | EVQLVES-GGGLVKPGGSLRLSCAASG-FTFS | S-----YTMN | WVRQAPGKGLDWVS | SISGS---SSYIYYADSVKG | RFTISRDNAKNSLYLQMNSLRAEDTAVYYCAR | DRG-------------------------SG | WGQGTLVTVSS |
| **iPS:3 93982** | **21-225_6C12** | **VH3\|3-21/D4\|4-11\|RF2/J H4** | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | DRG-------------------------SL | WGQGTLVTVSS |
| **iPS:3 98478** | **21-225_17C10** | **VH3\|3-21/D4\|4-11\|RF2/J H4** | | EVQLVES-GGGLVKPGGSLRLSCAASG-FTFS | S-----YSMN | WVRQAPGKGLEWVS | SISGS---SSYMYYADSVKG | RFTISRDNAKNSLYLQMNSLRAEDTALYYCAR | DRG-------------------------SS | WGQGTLVTVSS |
| | | **Germline** | | **H_FR1** | **H_CDR1** | **H_FR2** | **H_CDR2** | **H_FR3** | **H_CDR3** | **H_FR4** |
| | **VH3\|3-23/D6\|6-** | | 32244 | EVQLLES- | S-----YAMS | WVRQAPGK | AISGS--- | RFTISRDNSKNTLYLQMNSL | EYSSS----------- | WGQGT |

| | | | | | | | | | | |
|---|---|---|---|---|---|---|---|---|---|---|
| iPS:4 33897 | 21-225_43C2 | VH3\|3-23/D6\|6-6\|RF1/JH4 | | EVQLLES-GGGLVQPGGSLRLSCAASG-FTFS | S-----YAMS | WVRQAPGKGLEWVS | AISGR---GVNTFDADSVKG | RFTISRDNSKNTLYLQMNSLRAEDTAVYYCAK | ERSGS--------------------YFDY | WGQGTLVTVSS |
| iPS:4 33903 | 21-225_43H4 | VH3\|3-23/D6\|6-6\|RF1/JH4 | | EVQLLES-GGGLVQPGGSLRLSCAASG-FTFS | S-----YAMS | WVRQAPGKGLEWVS | AISGR---GINTFDADSVKG | RFTISRDNSKNTLYLQMNSLRAEDTAVYYCAK | ERSGS--------------------YFDY | WGQGTLVTVSS |
| iPS:4 33911 | 21-225_43E8 | VH3\|3-23/D6\|6-6\|RF1/JH4 | | EVQLLES-GGGLVQPGGSLRLSCAASG-FTFS | S-----YAMS | WVRQAPGKGLEWVS | AISGR---GINTFDADSVKG | RFTISRDNSKNTLYLQMNSLRAEDTAVYYCAK | ERSGS--------------------YFDY | WGQGTLVTVSS |
| iPS:4 33941 | 21-225_44D10 | VH3\|3-23/D6\|6-6\|RF1/JH4 | | EVQLLES-GGGLVQPGGSLRLSCAASG-FTFS | S-----YAMS | WVRQAPGKGLEWVS | AISGR---GVNTFDADSVKG | RFTISRDNSKNTLYLQMNSLRAEDTAVYYCAK | ERSGS--------------------YFDY | WGQGTLVTVSS |
| iPS:4 33957 | 21-225_45F8 | VH3\|3-23/D6\|6-6\|RF1/JH4 | | EVQLLES-GGGLVQPGGSLRLSCAASG-FTFS | S-----YAMS | WVRQAPGKGLEWVS | AISGR---GVNTFDADSVKG | RFTISRDNSKNTLYLQMNSLRAEDTAVYYCAK | ERSGS--------------------YFDY | WGQGTLVTVSS |
| iPS:4 33973 | 21-225_46A6 | VH3\|3-23/D6\|6-6\|RF1/JH4 | | EVQLLES-GGGLVQPGGSLRLSCAASG-FTFS | S-----YAMS | WVRQAPGKGLEWVS | AISGR---GINTFDADSVKG | RFTISRDNSKNTLYLQMNSLRAEDTAVYYCAK | ERSGS--------------------YFDY | WGQGTLVTVSS |
| iPS:4 35715 | 21-225_171A8 | VH3\|3-23/D6\|6-6\|RF1/JH4 | | EVQLLES-GGGLVQPGGSLRLSCAASG-FTFR | S-----SAMS | WVRQAPGKGLEWVS | VISGS---GGSTFYTDSVKG | RFTISRDNSKNTLYLQMNSLRAEDTAVYYCAK | SNSSG--------------------WFDY | WGQGTLVTVSS |
| iPS:4 35739 | 21-225_174G7 | VH3\|3-23/D6\|6-6\|RF1/JH4 | | EVQLLES-GGGLVQPGGSLRLSCAASG-FTFR | S-----SAMS | WVRQAPGKGLEWVS | VISGS---GGSTFYTDSVKG | RFTISRDNSKNTLYLQMNSLRAEDTAVYYCAK | SNSSG--------------------WFDY | WGQGTLVTVSS |
| iPS:4 35749 | 21-225_175C10 | VH3\|3-23/D6\|6-6\|RF1/JH4 | | EVQLLES-GGGLVQPGGSLRLSCAASG-FTFS | S-----YAMS | WVRQAPGKGLEWVS | SISGR---GGSTFYADSVKG | RFTVSRDNSKNTLYLQMNSLRAEDTAVYYCAK | SNSSG--------------------WFDY | WGQGTLVTVSS |
| | | Germline | | H_FR1 | H_CDR1 | H_FR2 | H_CDR2 | H_FR3 | H_CDR3 | H_FR4 |
| | VH3\|3-21/D7\|7-27\|RF1/JH4 | | 32245 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | LTGY---------------------FDY | WGQGTLVTVSS |
| iPS:4 33901 | 21-225_43A4 | VH3\|3-21/D7\|7-27\|RF1/JH4 | | EVQLVES-GGGLVKPGGSLRLSCAASG-FTFS | S-----YTMN | WVRQVPGKGLEWVS | SISGS---STYIYYADSVKG | RFTISRDDAQNSLYLQMNSLRGEDTAVYYCAR | VTS-----------------------FDY | WGQGALVTVSS |
| iPS:4 33961 | 21-225_45D9 | VH3\|3-21/D7\|7- | | EVQLVES-GGGLVKPGGSLRL | S-----YTMN | WVRQVPGKGLEWVS | SISGS---STYIYYADS | RFTISRDDAQNSLYLQMNSLRGEDTAVYYCAR | VTS-----------------------FDY | WGQGALVTVS |

| | | | | | | | | | | |
|---|---|---|---|---|---|---|---|---|---|---|
| **iPS:434135** | **21-225_54H3** | **VH3\|3-21/D7\|7-27\|RF1/JH4** | | EVQLVES-<br>GGGLVKPGGSLRL<br>SCAASG-FTFS | S-----YSMI | WVRQAPGK<br>GLEWVS | SISGT---<br>SSYIYYADS<br>VKG | RFTISRDNAKNSLYLQMNSL<br>RAEDTAVYYCAG | MTT-------------<br>------------VI | WGQGT<br>LVTVS<br>S |
| **iPS:434331** | **21-225_63H8** | **VH3\|3-21/D7\|7-27\|RF1/JH4** | | EVQLVES-<br>GGGLVKPGGSLRL<br>SCAASG-FTFS | S-----YNMN | WVRQAPGK<br>GLEWVS | SISGS---<br>STYMNYTDS<br>VKG | RFTISRDNAKNSLYLQMNSL<br>RAEDTAVYYCAR | LRN-------------<br>-----------FDY | WGQGT<br>LVTVS<br>S |
| **iPS:435421** | **21-225_151F1** | **VH3\|3-21/D7\|7-27\|RF1/JH4** | | EVQLVES-<br>GGGLVKPGGSLRL<br>SCAASG-YTFR | S-----FSMN | WVRQAPGK<br>GLEWVS | SISSS---<br>SYYIYYADS<br>VKG | RFTISRDNAKNSLYLQMNSL<br>RAEDTAVYYCAR | DTP-------------<br>-----------LVY | WGQGT<br>LVTVS<br>S |
| **iPS:435653** | **21-225_166H12** | **VH3\|3-21/D7\|7-27\|RF1/JH4** | | EVQLVES-<br>GGALVKPGGSLRL<br>SCAASG-FTFS | S-----YSMS | WVRQAPGK<br>GLGWVS | SISGS---<br>SSYSYYADS<br>VKG | RFTISRDNAKNSLYLQMNSL<br>RAEDTAVYYCAR | LTG-------------<br>-----------FDY | WGQGT<br>LVTVS<br>S |
| **iPS:436648** | **21-225_227F11** | **VH3\|3-21/D7\|7-27\|RF1/JH4** | | EVQLVES-<br>GGGLVKPGGSLRL<br>SCAVSG-FTFS | T-----YSMN | WVRQAPGK<br>GLEWVS | SISSS---<br>INYMYYADS<br>VKG | RFTISRDNAKNSLYLQMNSL<br>RAEDSAVYYCAR | LG--------------<br>------------VY | WGQGT<br>LVTVS<br>S |
| **iPS:392952** | **21-225_26G1** | **VH3\|3-21/D7\|7-27\|RF1/JH4** | | EVQLVES-<br>GGGLVKPGGSLRL<br>SCAASG-FTFS | S-----YGMN | WVRQAPGK<br>GLEWVS | SISGS---<br>SSYIYYADS<br>VKG | RFTISRDNAKNSLYLQMNSL<br>RAEDTAVYYCAR | LTT-------------<br>-----------FDF | WGQGT<br>LVTVS<br>S |
| **iPS:393082** | **21-225_34C11** | **VH3\|3-21/D7\|7-27\|RF1/JH4** | | EVQLVES-<br>GGGLVKPGGSLRL<br>SCAASG-FTFS | S-----YTMS | WVRQAPGK<br>GLEWVS | SISGS---<br>SNYIYYADS<br>VKG | RFTISRDNAKNSLYLQMNSL<br>RAEDTAVYYCAR | LTG-------------<br>-----------FDY | WGQGT<br>LVTVS<br>S |
| **iPS:394061** | **21-225_12D2** | **VH3\|3-21/D7\|7-27\|RF1/JH4** | | EVQLVES-<br>GGGLVKPGGSLRL<br>SCAASG-FTFS | S-----YSMN | WVRQAPGK<br>GLEWVS | SISSS---<br>SSYIYYADS<br>VKG | RFTISRDNAKNSLYLQMNSL<br>RAEDTAVYYCAR | LG--------------<br>------------DY | WGQGT<br>LVAVS<br>S |
| **iPS:394071** | **21-225_10C7** | **VH3\|3-21/D7\|7-27\|RF1/JH4** | | EVQLVES-<br>GGGLVKPGGSLRL<br>SCAASG-FTFS | S-----YSMN | WVRQAPGK<br>GLEWVS | SISSS---<br>NNYIYYADS<br>VKG | RFTISRDNAKNSLYLQMNSL<br>RAEDSAVYYCAR | LG--------------<br>------------VY | WGQGT<br>LVTVS<br>S |
| | | | | | | | | | | |
| **iPS:398532** | **21-225_33B7** | **VH3\|3-21/D7\|7-27\|RF1/JH4** | | EVQLVES-<br>GGGLVKPGGSLRL<br>SCAASG-FTFS | S-----YNMN | WVRQAPGK<br>GLEWVS | SISGS---<br>SSYIYYADS<br>VKG | RFTISRDNAKNSLYLQMNSL<br>RAEDTAVYYCAR | LNG-------------<br>-----------FDY | WGQGT<br>LVTVS<br>S |
| **iPS:402225** | **21-225_2B1** | **VH3\|3-21/D7\|7-27\|RF1/JH4** | | EVQLVES-<br>GGGLVKPGGSLRL<br>SCAASG-FTFS | S-----YSMN | WVRQAPGK<br>GLEWVS | SISSS---<br>SSYIYYADS<br>VKG | RFTISRDNAKNSLYLQMNSL<br>RAEDTAVYYCAR | LG--------------<br>------------NY | WGQGT<br>LVTVS<br>S |

| | | | | | | | | | |
|---|---|---|---|---|---|---|---|---|---|
| iPS:404090 | 21-225_8D8 | VH3\|3-21/D7\|7-27\|RF1/JH4 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | LG------------------------NY | WGQGTLVTVSS |
| | | Germline | | H_FR1 | H_CDR1 | H_FR2 | H_CDR2 | H_FR3 | H_CDR3 | H_FR4 |
| | VH3\|3-11/D4\|4-11\|RF2/JH4 | | 32246 | QVQLVES-GGGLVKPGGSLRLSCAASG-FTFS | D-----YYMS | WIRQAPGKGLEWVS | YISSS---GSTIYYADSVKG | RFTISRDNAKNSLYLQMNSLRAEDTAVYYCAR | DYSNY--------------------YFDY | WGQGTLVTVSS |
| iPS:433905 | 21-225_43E5 | VH3\|3-11/D4\|4-11\|RF2/JH4 | | QVQLVES-GGGLVKPGGSLRLSCAASG-FTFS | D-----YYMI | WIRQAPGKGLEWVS | YISSS---GITKYYADSMKG | RFTISRDNAKNSLYLQMNSLRAEDTAVYYCAA | DT------------------------IY | WGQGTLVTVSS |
| iPS:433913 | 21-225_43H8 | VH3\|3-11/D4\|4-11\|RF2/JH4 | | QVQLVES-GGGLVKPGGSLRLSCAASG-ITFS | D-----YYMN | WIRQAPGKGLEWVS | YISSS---GRTIFYADSLKG | RFTISRDNAKNSLYLQMNSLRAEDTAVYYCAA | DT------------------------IY | WGQGTLVTVSS |
| iPS:433949 | 21-225_45H2 | VH3\|3-11/D4\|4-11\|RF2/JH4 | | QVQLVES-GGGLVKPGGSLRLSCAASG-FTFS | D-----YYMN | WIRQAPGKGLEWVS | YISSS---GITKYYADSVKG | RFTISRDNAKNSLYLQMNSLRAEDTAVYYCAA | DT------------------------IY | WGQGTLVTVSS |
| iPS:433981 | 21-225_46E9 | VH3\|3-11/D4\|4-11\|RF2/JH4 | | QVQLVES-GGGLVKPGGSLRLSCAASG-FTFS | D-----YYMN | WIRQAPGKGLEWVS | YINSN---GFTIYYADSVKG | RFTISRDNAKNSLYLQMNSLRAEDTAVYYCAA | DT------------------------IY | WGQGTLVTVSS |
| iPS:433995 | 21-225_47H7 | VH3\|3-11/D4\|4-11\|RF2/JH4 | | QVQLVES-GGGLVKPGGSLRLSCAASG-FTFS | D-----YYMI | WIRQAPGKGLEWVS | YINSN---GFTKYYADSVKG | RFTISRDNAKNSLYLQMNSLRAEDTAVYYCAA | DT------------------------VY | WGQGTLVTVSS |
| iPS:434039 | 21-225_43B1 | VH3\|3-11/D4\|4-11\|RF2/JH4 | | QVQLVES-GGGLVKPGGSLRLSCAASG-FTFS | D-----YYMN | WIRQAPGKGLEWVS | YINSN---GFTIYYADSVKG | RFTISRDNAKNSLYLQMNSLRAEDTAVYYCAA | DT------------------------IY | WGQGTRVTVSS |
| iPS:434275 | 21-225_57F4 | VH3\|3-11/D4\|4-11\|RF2/JH4 | | QVQLVES-GGGLVKPGGSLRLSCAASG-FIFS | D-----YYMN | WIRQAPGKGLEWVS | YISSS---GSTIYYADSVKG | RFTISRDNAKNSLYLQMNSLRAEDTAVYYCAR | DM------------------------IT | WGQGTLVTVSS |
| | | Germline | | H_FR1 | H_CDR1 | H_FR2 | H_CDR2 | H_FR3 | H_CDR3 | H_FR4 |
| | VH3\|3-23/D7\|7-27\|RF1/JH3 | | 32247 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | LTGD--------------------AFDI | WGQGTMVTVSS |
| iPS:433915 | 21-225_43H9 | VH3\|3-23/D7\|7-27\|RF1/JH3 | | EVQLLES-GGGLVQPGGSLRLSCAASG-FTFS | S-----YAMS | WVRQAPGMGLEWVS | AISGS---GSNTFYADSVKG | RFTISRDNSKNTLYLHMNSLRAEDTAVYFCAK | RTPSD-------------------VFDI | WGQGTMVTVSS |
| iPS:433925 | 21-225_44F3 | VH3\|3-23/D7\|7- | | EVHLLES-GGGLVQPGGSLRL | S-----YAMS | WVRQAPGKGLEWVS | ILSGG---GKTTYYADS | RFTISRDNSKNTLFLQMNSLRAEDTAVYYCAK | RTPSD-------------------AFDI | WGQGTMVTVS |

| | | | | | | | | | | |
|---|---|---|---|---|---|---|---|---|---|---|
| iPS:433953 | 21-225_45H4 | VH3\|3-23/D7\|7-27\|RF1/JH3 | | EVQLLES-GGGLVQPGGSLRLSCAASG-FTFS | S-----YAMS | WVRQAPGMGLEWVS | AISGS---GSNTFYADSVKG | RFTISRDNSKNTLYLHMNSLRAEDTAVYFCAK | RTPSD---------------------VFDI | WGQGTMVTVSS |
| iPS:433959 | 21-225_45C9 | VH3\|3-23/D7\|7-27\|RF1/JH3 | | EVQLLES-GGGLVQPGGSLRLSCAASG-FTFN | S-----YAMS | WVRQAPGKGLEWVS | VISGR---GGTTYYADSVKG | RFTISRDNSKNTLYLQMNSLRAEDTAVYYCAK | RTPSD---------------------AFDI | WGQGTMVTVSS |
| iPS:435379 | 21-225_149B6 | VH3\|3-23/D7\|7-27\|RF1/JH3 | | EVQLLES-GGGLVQPGGSLRLSCAASG-FTFS | S-----YAMS | WVRQAPGKGLEWVS | VISGS---GGSTFYADSVKG | RFTISRDNSKNTLFLQMNSLRAEDTAVYYCAK | RTPED---------------------VFDI | WGQGTMVTVSS |
| iPS:435787 | 21-225_180A3 | VH3\|3-23/D7\|7-27\|RF1/JH3 | | EVQLLES-GGGLEQPGGSLRLSCAASG-FTFS | S-----FAMN | WVRQAPGKGLEWVS | VISGR---GGNTFYADSVKG | RFTISRDNSKNTLFLQMNSLRAEDTAVYFCAK | RTGDD---------------------VFDV | WGQGTMVTVSS |
| iPS:435809 | 21-225_182H5 | VH3\|3-23/D7\|7-27\|RF1/JH3 | | EVQLLES-GGGLVQPGGSLRLSCAASG-FTFS | S-----YAMS | WVRQAPGKGLEWVS | VISGR---GGTTFYADSVKG | RFTISRDNSKNTLYLQMNSLRAEDTAVYYCAK | RTGDD---------------------VFDI | WGQGTMVTVSS |
| iPS:435889 | 21-225_186A11 | VH3\|3-23/D7\|7-27\|RF1/JH3 | | EVQLLES-GGGLVQPGGSLRLSCAASG-FTFS | S-----YAMS | WVRQAPGKGLEWVS | VISGR---GGTTFYADSVKG | RFTISRDNSKNTLYLQMNSLRAEDTAVYYCAK | RTGDD---------------------VFDI | WGQGTMVTVSS |
| | | Germline | | H_FR1 | H_CDR1 | H_FR2 | H_CDR2 | H_FR3 | H_CDR3 | H_FR4 |
| | VH4\|4-59/D6\|6-13\|RF2/JH4 | | 32248 | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAR | GIAAA--------------------GYFDY | WGQGTLVTVSS |
| iPS:433931 | 21-225_44F6 | VH4\|4-59/D6\|6-13\|RF2/JH4 | | QVHLQES-GPGLVKPSETLSLTCTVSG-GSIS | S-----YYWS | WIRQPPGKGLEWIG | YIYY----SGNTNYNPSLKS | RVTISVDTSKNQFSLKLSSVTAADTAVYYCVR | GVAI-----------------------KNY | WGQGILVTVSS |
| | | Germline | | H_FR1 | H_CDR1 | H_FR2 | H_CDR2 | H_FR3 | H_CDR3 | H_FR4 |
| | VH3\|3-23/D4\|4-17\|RF2/JH6 | | 32248 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | DYGDYYY-----------------YYYGMDV | WGQGTTVTVSS |
| iPS:433943 | 21-225_44E10 | VH3\|3-23/D4\|4-17\|RF2/JH6 | | EVQLLES-GGGLVQSGGSLRLSCAASG-FTFS | S-----YAMN | WVRQAPGKGLEWVS | GVVGS---GGRTYYADSVKG | RFIISRDNSKNTLYLQMNSLRAEDTAVYYCAK | DRGQWL-------------------LGGMDV | WGQGTTVTVSS |
| iPS:433989 | 21-225_47C7 | VH3\|3-23/D4\|4-17\|RF2/JH6 | | EVQLLES-GGGLVQPGGSLRLSCAASG-FTFS | N-----YAMS | WVRQAPGKGLEWVS | GISGS---GSRTYYADSVKG | RFTISRDNSKNTLYLQMNSLRAEDTAVYYCAK | DRGQWL-------------------IGGMDV | WGQGTTVTVSS |
| iPS:434133 | 21-225_54G3 | VH3\|3-23/D4\|4- | | EVQLLES-GGGLVQPGGSLRL | T-----YAMS | WVRQAPGKGLEWVS | AISGS---GVNTFYADS | RFTISRDNSKNTLFLQMNSLRAEDTAVYYCAK | LGKDYY-------------------YYGMDV | WGQGTTVTVS |

| | | | | | | | | | | |
|---|---|---|---|---|---|---|---|---|---|---|
| iPS:434221 | 21-225_60A11 | VH3\|3-23/D4\|4-17\|RF2/JH6 | | EVQLLES-GGGLVQPGGSLRLSCAASG-FTFS | N-----YAMT | WVRQAPGKGLEWVS | AISGS---GGNTFYADSVTG | RVTISRDNSKNTLYLQMNSLRAEDTAVYYCAK | LGKDYH-----------------YYGMDV | WGQGTTVTVSS |
| iPS:434257 | 21-225_62F7 | VH3\|3-23/D4\|4-17\|RF2/JH6 | | EVQLLES-GGGLVQPGGSLRLSCAASG-FTFS | S-----YVMS | WVRQAPGKGLEWVS | GISGS---GAKTYYADSVKG | RFTISRDNSKNTLYLQMNSLRAEDTAVYYCAE | LGIDYY------------------YGMDV | WGQGTTVTVSS |
| iPS:434283 | 21-225_57F8 | VH3\|3-23/D4\|4-17\|RF2/JH6 | | EVQLLES-GGGLVQPGGSLRLSCVVSG-FTFS | N-----YAMS | WVRQAPGKGLEWVS | ASSGS---GGNTFYADSVTG | RVTISRDNSKNTLYLQMNSLRAEDTAVYYCAK | LGKDYH-----------------YYGMDV | WGQGTTVTVSS |
| iPS:434385 | 21-225_66C10 | VH3\|3-23/D4\|4-17\|RF2/JH6 | | EVQLLES-GGGLVQPGGSLRLSCAASG-FTFS | S-----YVMS | WVRQAPGKGLEWVS | GISGS---GARTYYADSVKG | RFTISRDNSKNTLYLQMNSLRAEDTAVYYCAE | LGIDYY------------------YGMDV | WGQGTTVTVSS |
| iPS:435717 | 21-225_171A9 | VH3\|3-23/D4\|4-17\|RF2/JH6 | | EVQLLES-GGGLAQPGGSLRLSCAASG-FTFS | S-----YAMT | WVRQAPGKGLEWVS | AISGS---GGNTFNADSVKG | RFTISRDNSKNTLYLQMNSLRAEDTAVYYCAK | LGIDYY-----------------YYGMDV | WGQGTTVTVSS |
| iPS:436528 | 21-225_224B1 | VH3\|3-23/D4\|4-17\|RF2/JH6 | | EVQVLES-GGGLVQPGGSLRLSCAASG-FTFS | S-----YAMS | WVRQAPGKGLEWVS | AISGS---GGNTYYADSVKG | RFTISRDNSKNTLYLHMNSLRAEDTAVYYCAG | EGGYYY-----------------YYGVDV | WGQGTTVTVSS |
| iPS:393810 | 21-225_5A4 | VH3\|3-23/D4\|4-17\|RF2/JH6 | | EVQVLES-GGGLVQPGGSLRLSCAASG-LTFS | S-----SAMS | WVRQAPGKGLEWVS | AISGR---GGNTFYTDSVKG | RFTISRDNSKNTLYLQMNSLRAEDTAVYYCAK | LGKDYY-----------------YYGMDV | WGQGTTVTVSS |
| | | Germline | | H_FR1 | H_CDR1 | H_FR2 | H_CDR2 | H_FR3 | H_CDR3 | H_FR4 |
| | VH3\|3-48/D5\|5-24\|RF3/JH6 | | 32250 | EVQLVES-GGGLVQPGGSLRLSCAASG-FTFS | S-----YSMN | WVRQAPGKGLEWVS | YISSS---SSTIYYADSVKG | RFTISRDNAKNSLYLQMNSLRDEDTAVYYCAR | RDGYNYYY---------------YYYGMDV | WGQGTTVTVSS |
| iPS:433945 | 21-225_44C12 | VH3\|3-48/D5\|5-24\|RF3/JH6 | | EVQLVES-GGGLVQPGGSLRLSCAASG-FTFS | S-----YSVN | WVRQAPGKGLEWVS | YISSS---SSTIYYADSVKG | RFTISRDNAKNSLYLQMNSLRDEDTAVYYCAR | SGYSYAYY---------------YYYGMDV | WGQGTTVTVSS |
| | | Germline | | H_FR1 | H_CDR1 | H_FR2 | H_CDR2 | H_FR3 | H_CDR3 | H_FR4 |
| | VH3\|3-33/D6\|6-13\|RF2/JH4 | | 32251 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | GIAAA--------------------GYFDY | WGQGTLVTVSS |
| iPS:433947 | 21-225_44E12 | VH3\|3-33/D6\|6-13\|RF2/JH4 | | QVHLAES-GGGVVQPGRSLRLSCEASG-FTLS | S-----DDTH | WVRQPPGKGLEWVA | VIWFD---EYNKYYADSVKG | RFTISRDNSKNTLYLQMNSLRAEDAAVYYCAR | DLIAAA-------------------GTGDY | WGQGTLVTVSS |
| iPS:433963 | 21-225_46B1 | VH3\|3-33/D6\|6- | | QVHLAES-GGGVVQPGRSLRL | S-----DDSH | WVRQPPGKGLEWVA | VIWFD---EYTKYYADS | RFTISRDNSKNTLYLQMNNLRAEDAAVYYCAR | DLIAAT-------------------GTGDY | WGQGTLVTVS |

| | | | | | | | | | | |
|---|---|---|---|---|---|---|---|---|---|---|
| **iPS:433987** | **21-225_47A5** | **VH3\|3-33/D6\|6-13\|RF2/JH4** | | QVQLVES-GGGVVQPGRSLRLSCAASG-FTFS | D-----DDTH | WVRQAPGKGLEWVA | VIWFD---GSNKYYADSVKG | RFTISRDNSKNTLYLQMNSLSAEDTAVYYCAR | DLIAAA-------------------GTVDY | WGQGTLVTVSS |
| **iPS:436258** | **21-225_202F12** | **VH3\|3-33/D6\|6-13\|RF2/JH4** | | QVQLVES-GGGVVQPGRSLRLSCEASG-FTFS | Y-----YGMH | WVRQAPGKGLEWVA | IIWYD---GSNKFYADSVKG | RFTISRDSSKNTLYLQMNSLRAEDTAVYYCAS | NIAAAA-------------------PYFDY | WGQGTLVTVSS |
| **iPS:392646** | **21-225_20G2** | **VH3\|3-33/D6\|6-13\|RF2/JH4** | | QVQLVES-GGGVVQPGRSLRLSCAASG-FTLS | S-----DDMH | WVRQEPGKGLEWVA | VIWFD---GSNKYYADSVKG | RFIMSRDNSKNTLYLQMNSLRAGDTAVYYCAR | DLIAAA-------------------GTVDY | WGQGTLVTVSS |
| **iPS:392750** | **21-225_20A10** | **VH3\|3-33/D6\|6-13\|RF2/JH4** | | QVQLVES-GGGVVQPGRSLRLSCAASG-FTLS | S-----DDMH | WVRQAPGKGLEWVA | VIWFD---GSNKYYADSVKG | RFIISRDNSKNTLYLQMNSLRAEDTAMYYCAR | DLIAAA-------------------GTVDY | WGQGTLVTVSS |
| | | **Germline** | | **H_FR1** | **H_CDR1** | **H_FR2** | **H_CDR2** | **H_FR3** | **H_CDR3** | **H_FR4** |
| | **VH3\|3-23/D6\|6-19\|RF2/JH3** | | 32252 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GIAVAG------------------DAFDI | WGQGTMVTVSS |
| **iPS:433999** | **21-225_48D1** | **VH3\|3-23/D6\|6-19\|RF2/JH3** | | EVQLLES-GGGLVQPGGSLRLSCAASR-FTFS | S-----YAMS | WVRQAPGKGLEWVS | VISGR---GGSTFYADSVKG | RFTISRDNSKNTLYLQMNSLRAEDTAVYYCAR | RIAVAG------------------NEAFDI | WGQGTMVTVSS |
| **iPS:434003** | **21-225_48C3** | **VH3\|3-23/D6\|6-19\|RF2/JH3** | | EVQLLES-GGGLVQPGGSLRLSCAASR-FTFS | S-----YAMS | WVRQAPGKGLEWVS | VISGR---GGSTFYADSVKG | RFTISRDNSKNTLYLQMNSLRAEDTAVYYCAR | RIAVAG------------------NEAFDI | WGQGTMVTVSS |
| **iPS:434037** | **21-225_49G12** | **VH3\|3-23/D6\|6-19\|RF2/JH3** | | EVQLLES-GGGLVQPGGSLRLSCTASG-FTFS | S-----YAMS | WVRQAPGKGLEWVS | VISGS---GGTTFYADSVKG | RLTISRDNSKNTLYLQMNSLRAEDTAVYYCAR | RIAVAG------------------NDAFDI | WGQGTMVTVSS |
| **iPS:434041** | **21-225_50H8** | **VH3\|3-23/D6\|6-19\|RF2/JH3** | | EVQLLES-GGGLVQPGGSLRLSCAASR-FTFS | S-----YAMS | WVRQAPGKGLEWVS | VISGR---GGTTFYADSVKG | RFTISRDNSKNTLYLQMNSLRAEDTAVYYCAR | RIAVAG------------------NEAFDI | WGQGTMVTVSS |
| **iPS:434045** | **21-225_50H10** | **VH3\|3-23/D6\|6-19\|RF2/JH3** | | EGQLLES-GGGLVQPGGSLRLSCAASR-FTFS | S-----YAMS | WVRQAPGKGLEWVS | VISGR---GGSTFYADSVKG | RFTISRDNSKNTLYLQMNSLRAEDTAVYYCAR | RIAVAG------------------NEAFDI | WGQGTMVTVSS |
| **iPS:434073** | **21-225_51H10** | **VH3\|3-23/D6\|6-19\|RF2/JH3** | | EVQLLES-GGGLVQPGGSLRLSCTASG-FTFS | S-----YAMS | WVRQAPGKGLEWVS | VISGS---GGTTFYADSVKG | RLTISRDNSKNTLYLQMNSLRAEDTAVYYCAR | RIAVAG------------------NDAFDI | WGQGTMVTVSS |
| **iPS:436212** | **21-225_200G1** | **VH3\|3-23/D6\|6-** | | EVQLLES-GGGLVQPGGSLRL | S-----YAMS | WVRQAPGKGLEWVS | AISGR---GGNTFYADS | RFTISRDNSKNTLYLQMNSLRAEDTAVYYCAR | RIAVDG------------------YDAFDV | WGQGTMVTVS |

<table>
<tr><td>iPS:392652</td><td>21-225_17C6</td><td>VH3|3-23/D6|6-19|RF2/JH3</td><td></td><td>EVQLLES-GGGLVQPGGSLRLSCAASE-FTFS</td><td>S-----YAMN</td><td>WVRQAPGKGLEWVS</td><td>VISGR---GGNTFYADSVKG</td><td>RFTISRDNSKNTLFLQMNSLRAEDTAVYYCAS</td><td>RLAVAG------------------SEAFDI</td><td>WGQGTMVTVSS</td></tr>
<tr><td>iPS:392668</td><td>21-225_17B4</td><td>VH3|3-23/D6|6-19|RF2/JH3</td><td></td><td>EVQLLES-GGGLVQPGGSLRLSCAASE-FTFS</td><td>S-----YAMN</td><td>WVRQAPGKGLEWVS</td><td>VISGR---GGNTFYADSVKG</td><td>RFTISRDNSKNTLYLQMNSLRAEDTAVYYCAS</td><td>RLAVAG------------------SEAFDI</td><td>WGQGTMVTVSS</td></tr>
<tr><td>iPS:392696</td><td>21-225_20A4</td><td>VH3|3-23/D6|6-19|RF2/JH3</td><td></td><td>EVQLLES-GGGLVQPGGSLRLSCAASG-FTFS</td><td>S-----YAMT</td><td>WVRQGPGMGLEWVS</td><td>VISGS---GGYTYNADSVKG</td><td>RFTISRDNSKNTLYLQMNSLRVEDTAVYYCAS</td><td>RIAVAG------------------SEAFDI</td><td>WGQGTMVTVSS</td></tr>
<tr><td>iPS:392702</td><td>21-225_17F7</td><td>VH3|3-23/D6|6-19|RF2/JH3</td><td></td><td>EVQLLES-GGGLVQPGGSLRLSCAASE-FTFS</td><td>S-----YAMN</td><td>WVRQAPGKGLEWVS</td><td>IISGR---GGNTFYADSVKG</td><td>RFTISRDNSKNTLYLQMNSLRAEDTAVYYCAS</td><td>RLAVAG------------------SEAFDI</td><td>WGQGTMVTVSS</td></tr>
<tr><td>iPS:392704</td><td>21-225_17F11</td><td>VH3|3-23/D6|6-19|RF2/JH3</td><td></td><td>EAQLLES-GGGLEQPGGSLRLSCAASG-FTFS</td><td>S-----YAMS</td><td>WVRQAPGKGLEWVS</td><td>VISGR---GGNTYSADSVKG</td><td>RFTISRDNSKNTLYLQMNSLRAEDTAVYYCAS</td><td>RLAVAG------------------SEAFHI</td><td>WGQGTMVTVSS</td></tr>
<tr><td>iPS:392720</td><td>21-225_17A12</td><td>VH3|3-23/D6|6-19|RF2/JH3</td><td></td><td>EVQLLES-GGGLIQPGGSLRLSCAASE-FTFS</td><td>S-----YAMS</td><td>WVRQDPGKGLEWVS</td><td>IISGR---GGNAFYADSVKG</td><td>RFTISRDNSKNTLYLQMNSLRAEDTAVYYCAS</td><td>RIAVAG------------------SEAFDI</td><td>WGQGTMVTVSS</td></tr>
<tr><td>iPS:392722</td><td>21-225_18E12</td><td>VH3|3-23/D6|6-19|RF2/JH3</td><td></td><td>EVQLLES-GGGLVQPGGSLRLSCAASE-FTFS</td><td>S-----YAMS</td><td>WVRQAPGTGLEWVS</td><td>IISGR---GGNTFYADSVKG</td><td>RFTISRDNSKNTLYLQMNSLRAEDTAVYYCAS</td><td>RLAVAG------------------SEAFDI</td><td>WGQGTMVTVSS</td></tr>
<tr><td>iPS:392760</td><td>21-225_22G3</td><td>VH3|3-23/D6|6-19|RF2/JH3</td><td></td><td>EVQLLES-GGGLVQPGGSLRLSCAASE-FTFS</td><td>S-----YAMN</td><td>WVRQAPGKGLEWVS</td><td>IISGR---GVNTFYADSVKG</td><td>RFTISRDNSKNTLYLQMNSLRAEDTAVYYCAS</td><td>RLAVAG------------------SEAFDI</td><td>WGQGTMVTVSS</td></tr>
<tr><td>iPS:392764</td><td>21-225_22G10</td><td>VH3|3-23/D6|6-19|RF2/JH3</td><td></td><td>EVQLLES-GGDLVQPGGSLRLSCTASG-FTFS</td><td>S-----YAMN</td><td>WVRQAPGKGLEWVS</td><td>VISGS---GGNTFYADSVKG</td><td>RFTISRDNSKNTLFLHMNSLRAEDTAVYYCAS</td><td>RMAVAG------------------SEAFDI</td><td>WGQGTMVTVSS</td></tr>
<tr><td>iPS:392812</td><td>21-225_21F4</td><td>VH3|3-23/D6|6-19|RF2/JH3</td><td></td><td>EVQLLES-GGGLVQPGGSLRLSCAASE-FTFS</td><td>S-----YAMN</td><td>WVRQAPGKGLEWVS</td><td>VISGR---GGNTFYADSVKG</td><td>RFTISRDNSKNTLYLQMNSLRAEDTAVYYCAS</td><td>RLAVAG------------------SEACDI</td><td>WGQGTMVTVSS</td></tr>
<tr><td>iPS:392816</td><td>21-225_22E4</td><td>VH3|3-23/D6|6-19|RF2/JH3</td><td></td><td>EVQLLES-GGGLVQPGGSLRLSCAASE-FTFS</td><td>S-----YAMS</td><td>WVRQAPGKGLEWVS</td><td>IISGR---GTNTFYADSVKG</td><td>RFTISRVNSKNTLYLQMNSLRAEDTAVYYCAS</td><td>RIAVAG------------------SEAFDI</td><td>WGQGTMVTVSS</td></tr>
<tr><td>iPS:392852</td><td>21-225_21A2</td><td>VH3|3-23/D6|6-</td><td></td><td>EVQLLES-GGGLVQPGGSLRL</td><td>S-----YAMN</td><td>WVRQAPGKGLEWIS</td><td>IISGR---GGNTFYADS</td><td>RFTISRDNSKNTLYLQMNSLRAEDTAVYYCAS</td><td>RLAVAG------------------SEAFDI</td><td>WGQGTMVTVS</td></tr>
</table>

| | | | | | | | | | | |
|---|---|---|---|---|---|---|---|---|---|---|
| **iPS:392878** | **21-225_22C5** | **VH3\|3-23/D6\|6-19\|RF2/JH3** | | EVQLLES-GGGLVQVGGSLRLSCAASG-FTFS | S-----YAMS | WVRQAPGMGLEWVS | IISGS---GGYTYYADSVKG | RFTISRDNSKNTLYLQMNSLRAEDTAVYYCAS | RIAVAG-----------------SEAFDI | WGQGTMVTVSS |
| **iPS:392902** | **21-225_22D5** | **VH3\|3-23/D6\|6-19\|RF2/JH3** | | EVQLLES-GGGLVQPGGSLRLSCAASE-FTFS | S-----YAMS | WVRQAPGKGLEWVS | VISGR---GGNTFYADSVKG | RFTISRDNSKNTLYLQMNSLRAEDTAVYYCAS | RIAVAG-----------------SEAFDI | WGQGTMVTVSS |
| **iPS:393824** | **21-225_10F12** | **VH3\|3-23/D6\|6-19\|RF2/JH3** | | EVQLLES-GGGLVQPGGSLRLSCAASE-FTFS | S-----YAMS | WVRQAPGKGLEWVS | IISGR---GGNTFYADSVKG | RFTISRDNSKNTLYLQMNSLRAEDTAVYYCAS | RVAVAG-----------------SEAFAI | WGQGTMVTVSS |
| **iPS:393848** | **21-225_4H2** | **VH3\|3-23/D6\|6-19\|RF2/JH3** | | EVQLLES-GGGLVQPGGSLRLSCAASG-FTFS | G-----YAMN | WVRQAPGMGLEWVS | VISRS---GGYTYYADSVKG | RFTISRDNSKNTLYLQMNSLRAEDTAVYYCAS | RLAVAG-----------------SEAFDI | WGQGTMVTVSS |
| **iPS:393862** | **21-225_5G2** | **VH3\|3-23/D6\|6-19\|RF2/JH3** | | EVQLLES-GGGLVQPGGSLRLSCAASE-FTFS | S-----YAMS | WVRQAPGKGLEWVS | VISGR---GVNTFYADSVKG | RFTISRDNSKNTLYLQMNSLRAEDTAVYYCAS | RIAVAG-----------------SEAFDI | WGQGTMVTVSS |
| **iPS:393888** | **21-225_3E3** | **VH3\|3-23/D6\|6-19\|RF2/JH3** | | EVQLLES-GGTLVQPGGSLRLSCAASE-FTFS | S-----YVMS | WVRQAPGKGLEWVS | IISGR---GGNTFYADSVKG | RFTISRDNSKNTLYLQMNSLRAEDTALYYCAS | RLAVAG-----------------SEAFDI | WGQGTMVTVSS |
| **iPS:393898** | **21-225_5F7** | **VH3\|3-23/D6\|6-19\|RF2/JH3** | | EVQLLES-GGGLVQPGGSLRLSCAASE-FTFS | S-----YAMS | WVRQAPGKGLEWVS | IISGR---GGNTFYADSVKG | RFTISRDNSKNTLYLQMNSLRAEDTAVYYCAS | RIAVAG-----------------SEAFAI | WGQGTMVTVSS |
| **iPS:393980** | **21-225_6D3** | **VH3\|3-23/D6\|6-19\|RF2/JH3** | | EVQLLES-GGGLVQPGGSLRLSCAASE-FTFS | S-----YAMN | WVRQAPGKGLEWVS | VISGR---GGNTFYADSVKG | RFTISRDNSKNTLYLQMNSLRAEDTAVYFCAS | RLAVAG-----------------SEAFDI | WGQGTMVTVSS |
| **iPS:394014** | **21-225_8G6** | **VH3\|3-23/D6\|6-19\|RF2/JH3** | | EVQLLES-GGGLVQPGGSLRLSCVASE-FTFS | S-----YVMN | WVRQAPGKGLEWVS | VISGR---GGNTFYADSVKG | RFTISRDNSKNTLYLQMNSLRAEDTAVYYCAS | RLAVAG-----------------SEAFDI | WGQGTMVSVSS |
| **iPS:394022** | **21-225_16H6** | **VH3\|3-23/D6\|6-19\|RF2/JH3** | | EVQLLES-GGGLVQPGGSLRLSCAASG-FTFS | S-----YAMN | WVRQAPGMGLEWVS | VISRS---GGYTYYADSVKG | RFTISRDNSKNTLYLQMNSLRAEDTAVYYCAS | RLAVAG-----------------SEAFDI | WGQGTMVTVSS |
| **iPS:394043** | **21-225_3B1** | **VH3\|3-23/D6\|6-19\|RF2/JH3** | | EVQLLES-GGGLVQPGGSLRLSCAASE-FTFS | S-----YAMN | WVRQAPGKGLEWVS | VISGR---GINTFYADSVKG | RFTISRDNSKNTLYLQMNSLRAEDTAVYYCAS | RLAVAG-----------------SEAFDI | WGQGTMVTVSS |
| **iPS:394077** | **21-225_8E12** | **VH3\|3-23/D6\|6-** | | EVQLLES-GGGLVQPGGSLRL | S-----YAMS | WVRQAPGKGLEWVS | IISGR---GGNTFYADS | RFTISRDNSKNTLYLQMNSLRAEDTAVYYCAS | RMAVAG-----------------SEAFDI | WGQGTMVTVS |

| | | | | | | | | | | |
|---|---|---|---|---|---|---|---|---|---|---|
| iPS:394087 | 21-225_11A5 | VH3\|3-23/D6\|6-19\|RF2/JH3 | | EVQLLES-GGDLVQPGGSLRLSCAASE-FTFS | S-----YAMS | WVRQAPGKGLEWIS | VISGR---GVNTFYADSVKG | RFTISRDNSKNTLYLQMNSLRAEDTAVYYCAS | RIAVAG-----------------SEAFDI | WGQGTMVTVSS |
| | | Germline | | H_FR1 | H_CDR1 | H_FR2 | H_CDR2 | H_FR3 | H_CDR3 | H_FR4 |
| | VH3\|3-11/D6\|6-6\|RF2/JH3 | | 32253 | QVQLVES-GGGLVKPGGSLRLSCAASG-FTFS | D-----YYMS | WIRQAPGKGLEWVS | YISSS---GSTIYYADSVKG | RFTISRDNAKNSLYLQMNSLRAEDTAVYYCAR | SIAAR-------------------DAFDI | WGQGTMVTVSS |
| iPS:434011 | 21-225_48B10 | VH3\|3-11/D6\|6-6\|RF2/JH3 | | QVQLVES-GGGLVKPGGSLRLSCAASG-FTFS | D-----YFMT | WIRQAPGQGLEWVS | YISSA---GGAIYYADSVKG | RFTISRDNAKNSLYLQMNSLRAEDTAVYYCAI | AVAAP-------------------GVFDI | WGQGTMVTVSS |
| | | Germline | | H_FR1 | H_CDR1 | H_FR2 | H_CDR2 | H_FR3 | H_CDR3 | H_FR4 |
| | VH3\|3-11/D6\|6-6\|RF2/JH4 | | 32254 | QVQLVES-GGGLVKPGGSLRLSCAASG-FTFS | D-----YYMS | WIRQAPGKGLEWVS | YISSS---GSTIYYADSVKG | RFTISRDNAKNSLYLQMNSLRAEDTAVYYCAR | SIAAR--------------------YFDY | WGQGTLVTVSS |
| iPS:434015 | 21-225_48F12 | VH3\|3-11/D6\|6-6\|RF2/JH4 | | QVQLVES-GGGLVKPGGSLRLSCAASG-FTFS | D-----YFMT | WIRQAPGQGLEWVS | YISSA---GGAIYYADSVKG | RFTISRDNAKNSLFLQMNSLRAEDTAVYYCAI | AVAAP-------------------GAFDI | WGQGTLVTVSS |
| iPS:434017 | 21-225_48G12 | VH3\|3-11/D6\|6-6\|RF2/JH4 | | QVQLVES-GGGLVKPGGSLRLSCAASG-FTFS | D-----YFMT | WIRQAPGQGLEWVS | YISSA---GGAIYYADSVKG | RFTISRDNAKNSLFLQMNSLRAEDTAVYYCAI | AVAAP-------------------GAFDI | WGQGTLVTVSS |
| | | Germline | | H_FR1 | H_CDR1 | H_FR2 | H_CDR2 | H_FR3 | H_CDR3 | H_FR4 |
| | VH3\|3-23/D6\|6-13\|RF2/JH4 | | 32255 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GIAAA-------------------GYFDY | WGQGTLVTVSS |
| iPS:434023 | 21-225_49F1 | VH3\|3-23/D6\|6-13\|RF2/JH4 | | EVQLLES-GGGLVQPGGSLRLSCAASG-FTFS | S-----YAMS | WVRQAPGKGLDWVS | VISGS---GGSTFYADSVKG | RFTISRDNSKSTLYLQMNSLRAEDTAVYYCAS | AIAAAG-----------------AHYFDY | WGQGTLVTVSS |
| iPS:436246 | 21-225_201G6 | VH3\|3-23/D6\|6-13\|RF2/JH4 | | EVQLLES-GGGLVQPGGSLRLSCVASG-FTFS | S-----YAMS | WVRQAPGKGLEWVS | TISGS---GVRTYYADSVKG | RFTISRDNSKNTLFLQMNSLRAEDTAVYYCAK | GGARSSG-----------------WFHFDY | WGQGTLVTVSS |
| iPS:436254 | 21-225_202C12 | VH3\|3-23/D6\|6-13\|RF2/JH4 | | EVQLLES-GGGLVQPGGSLRLSCAASG-FTFS | S-----YAMS | WVRQAPGKGLEWVS | TISGS---GVRTYYADSVKG | RSTISRDNSKNTLFLQMNSLRAEDTAVYYCAK | GGARSSG-----------------WFHFDY | WGQGTLVTVSS |
| iPS:436304 | 21-225_201F3 | VH3\|3-23/D6\|6-13\|RF2/JH4 | | EVQLLES-GGGLVQPGGSQRLSCAASG-FTFS | S-----YAMS | WVRQAPGKGLEWVS | TISGS---GVRTYYADSVKG | RFTISRDNSNNTLFLQMNSLRAEDTAVYYCAK | GGARSSG-----------------WFHFDY | WGQGTLVTVSS |
| iPS:436334 | 21-225_208G3 | VH3\|3-23/D6\|6- | | EVQLLES-GGGLVQPGGSLRL | S-----YAMS | WVRQAPGKGLEWVS | TISGS---GVRTYYADS | RSTISRDNSKNTLFLQMNSLRAEDKAVYYCAK | GGARSSG-----------------WFHFDY | WGQGTLVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH3\|3-23/D6\|6-19\|RF2/JH5 | | 32256 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GIAVAG-----------------NWFDP | WGQGTLVTVSS |
| iPS:434027 | 21-225_49H5 | VH3\|3-23/D6\|6-19\|RF2/JH5 | | EVQLLES-GGGLVQPGGSLRLSCAASG-FTFS | S-----YAMT | WVRQTPGKGLEWVS | AISGS---GGNSFYADSVKG | RFTISRDNSENTLYLQMNSLRAEDTAVYYCAK | ARAVAG-----------------SHWFDP | WGQGTLVTVSS |
| iPS:434061 | 21-225_51C7 | VH3\|3-23/D6\|6-19\|RF2/JH5 | | EVQLLES-GGGLVQPGGSLRLSCAASG-FTIS | N-----YAMT | WVRQTPGKGLEWVS | VISAS---GGNSFYADSVKG | RFTISRDNSENTFYLQMNSLRAEDTAVYYCAK | ARAVAG-----------------SHWFDP | WGQGTLVTVSS |
| iPS:434167 | 21-225_50F3 | VH3\|3-23/D6\|6-19\|RF2/JH5 | | EVQLLES-GGGLVQPGGSLRLSCAASG-FTFR | T-----YAMT | WVRQAPGKGLEWVS | AISGS---GVNSFYADSVKG | RFTISRDNSENTLYLQMNSLRAEDTAVYYCAK | ARAVAG-----------------SHWFDP | WGQGTLVTVSS |
| iPS:434455 | 21-225_72F5 | VH3\|3-23/D6\|6-19\|RF2/JH5 | | EVQLLES-GGGLVQPGGSLRLSCAASG-FTFS | S-----YAMI | WVRQAPGKGLEWVS | TISGS---GGYTYSADSVKG | RFTISRDNSKNTLYLQMNSLRAEDTAVYYCAR | RIAVTG-----------------TEWYDP | WGQGTLVTVSS |
| iPS:437232 | 21-225_63E1 | VH3\|3-23/D6\|6-19\|RF2/JH5 | | EVQMLES-GGGLGQSGGSLRLSCTASG-FTFT | T-----SAMS | WVRQAPGKGLEWVS | AISGS---GANTFYADSVKG | RFTVTRDNSKNTLYLQMNSLTAEDTAVYYCVK | VIAVAG-----------------GHFFDP | WGQGTLVTVSS |
| | | Germline | | H_FR1 | H_CDR1 | H_FR2 | H_CDR2 | H_FR3 | H_CDR3 | H_FR4 |
| | VH3\|3-21/D1\|1-1\|RF2/JH4 | | 32257 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | VQLER---------------------YFDY | WGQGTLVTVSS |
| iPS:434043 | 21-225_50G10 | VH3\|3-21/D1\|1-1\|RF2/JH4 | | EVQLVES-GGGLVKPGGSLRLSCAASG-FTFS | S-----YSMN | WVRQAPGKGLEWVS | SISGS---SSYIYYADSVKG | RFTISRDNAKNSLYLQMNSLRAEDTAVYYCAR | VAT------------------------FDY | WGQGTLVTVSS |
| iPS:434085 | 21-225_52E3 | VH3\|3-21/D1\|1-1\|RF2/JH4 | | EVQLVES-GGGLVKPGGSLRLSCAASG-FTFS | S-----YKMN | WVRQAPGKGLEWVS | SISSG---NSSIYYADSVKG | RFTISRDNAENSLYLQMNSLRAEDTAVYYCAR | VSS------------------------NDY | WGQGTLVTVSS |
| iPS:434101 | 21-225_52H12 | VH3\|3-21/D1\|1-1\|RF2/JH4 | | EVQLVES-GGGLVKPGGSLRLSCAASG-FTFS | S-----YSMN | WVRQAPGKGLEWVS | SISGS---STYIYYADSVKG | RFTVSRDNAKNSLYLQVNSLRAEDTAVYYCAR | VNS------------------------FDY | WGQGTLVTVSS |
| iPS:434187 | 21-225_56A5 | VH3\|3-21/D1\|1-1\|RF2/JH4 | | EVQLVES-GGGLVKPGGSLRLSCAASG-FTFS | S-----YSMN | WVRQAPGKGLEWVS | SISGS---SSYIYYADSVKG | RFTISRDNAKNSLYLQMNSLRAEDTAVYYCAR | VAT------------------------FDY | WGQGTLVTVSS |
| iPS:434265 | 21-225_57B2 | VH3\|3-21/D1\|1- | | EVQLVES-GGGLVKPGGSLRL | S-----YSMN | WVRQAPGKGLEWVS | SISGS---SSYINYTDS | RFTISRDNAKNSLYLQMNSLRAEDTAVYYCAR | VAG------------------------FDY | WGQGTLVTVS |

| | | | | | | | | | | |
|---|---|---|---|---|---|---|---|---|---|---|
| iPS:434439 | 21-225_70E12 | VH3\|3-21/D1\|1-1\|RF2/JH4 | | EVQLVES-GGGLVKPGGSLRLSCAASG-FTFS | S-----YSMN | WVRQAPGKGLEWVS | SISGN---STYIYYTDSVKG | RFTISRDNAKNSLYLQMDSLTAEDTAVYYCAR | VAA-----------------------FDC | WGQGTLVTVSS |
| iPS:435515 | 21-225_157E4 | VH3\|3-21/D1\|1-1\|RF2/JH4 | | EVQLVES-GGGLVKPGGSLRLSCAASG-FTFS | S-----YTMN | WVRQAPGKGLEWVS | SISGS---SSYIYYADSVKG | RFTISRDNAKNSLYLQMNSLKAEDTAVYYCAR | VAT-----------------------FDY | WGQGTLVTVSS |
| iPS:435535 | 21-225_157H10 | VH3\|3-21/D1\|1-1\|RF2/JH4 | | EVQLVES-GGGLVKPGGSLRLSCAASG-FTFS | S-----YTMN | WVRQAPGKGLEWVS | SISGS---SSYINYADSVKG | RFTISRDNAKNSLYLQMNSLRAEDTAVYYCAR | VAH-----------------------FDY | WGQGTLVTVSS |
| iPS:437224 | 21-225_56H1 | VH3\|3-21/D1\|1-1\|RF2/JH4 | | EVQLVES-GGGLVKPGGSLRLSCAASG-FTFS | N-----YRMN | WVRQGPGKGLEWIS | SISGS---STDIYYADSVKG | RFTISRDNAKNSLYLQMNSLRAEDTAVYYCAR | VAS-----------------------FDY | WGQGTLVTVSS |
| iPS:392620 | 21-225_17H5 | VH3\|3-21/D1\|1-1\|RF2/JH4 | | EVQLVES-GGGLVKPGGSLRLSCAASG-FTFS | S-----YSMN | WVRQAPGKGLEWVS | SISSS---STYIYYADSVKG | RFTISRDNAKNSLYLQMNSLRAEDTAVYYCAR | VAS-----------------------FDY | WGQGTLVTVSS |
| iPS:392632 | 21-225_16A11 | VH3\|3-21/D1\|1-1\|RF2/JH4 | | EVQLVES-GGGLVKPGGSLRLSCAASG-FTFS | S-----YSMN | WVRQAPGKGLEWVS | SISGS---SSLIYYADSVKG | RFTISRDNAKNSLYLQMNSLRAEDTAVYYCAR | VAA-----------------------FDY | WGQGTLVTVSS |
| iPS:392746 | 21-225_20H7 | VH3\|3-21/D1\|1-1\|RF2/JH4 | | EVHLVES-GGGLVKPGGSLRLSCAASG-FTFS | S-----YSMN | WVRQAPGKGLEWVS | SISGS---SSFIYYADSVKG | RFTISRDNAKNSLYLQMNSLRAEDTAVYYCAR | VAA-----------------------LDY | WGQGTLVTVSS |
| iPS:392782 | 21-225_22B12 | VH3\|3-21/D1\|1-1\|RF2/JH4 | | EVQLVES-GGGLVKPGGSLRLSCAASG-FTFS | S-----YSMN | WVRQAPGKGLEWVS | SISGS---SSYTYYADSVKG | RFTISRDNAKNSLYLQMNSLRAEDTAVYYCAR | VAA-----------------------LDS | WGQGTLVTVSS |
| iPS:392916 | 21-225_27C5 | VH3\|3-21/D1\|1-1\|RF2/JH4 | | EVQLVES-GGGLVKPGGSLRLSCAASG-FTFS | S-----YSMN | WVRQAPGKGLEWVS | STSSS---DSYIYYADSVKG | RFTISRDNAKNSLYLQMNSLRAEDTAVYYCAR | VAS-----------------------FDC | WGQGTLVTVSS |
| iPS:392976 | 21-225_27H12 | VH3\|3-21/D1\|1-1\|RF2/JH4 | | EVQLVES-GGGLVKPGGSLRLSCAASG-FTFS | S-----YSLN | WVRQAPGKGLEWVS | SISGS---SSNIYYTDSVKG | RFTISRDNAKNSLFLQMNSLRAEDTAVYYCAR | VAS-----------------------FDY | WGQGTLVTVSS |
| iPS:393120 | 21-225_35H8 | VH3\|3-21/D1\|1-1\|RF2/JH4 | | EVQLVES-GGGLVKPGGSLRLSCAASG-FTFS | S-----YSMN | WVRQAPGKGLEWVS | SISGT---GSFIYYADSVKG | RFTISRDNAKKSVYLQMNSLRAEDTAVYYCAR | VSG-----------------------FDY | WGQGTLVTVSS |
| iPS:393836 | 21-225_15A2 | VH3\|3-21/D1\|1- | | EVQLVES-GGGLVKPGGSLRL | S-----YTMN | WVRQAPGKGLEWVS | SISGS---GSYIYYADS | RFTISRDNAKNSLYLQMNALRAEDTAVYYCAR | VAS-----------------------FDY | WGQGTLVTVS |

| | | | | | | | | | | |
|---|---|---|---|---|---|---|---|---|---|---|
| **iPS:393894** | **21-225_5E11** | **VH3\|3-21/D1\|1-1\|RF2/JH4** | | EVQLVES-GGGLVKPGGSLRLSCAASG-FTFS | S-----YSMN | WVRQAPGKGLEWVS | SISGS---STYIYYADSVKG | RFTISRDNAKNSLYLQMNSLRAEDTAVFYCAR | VAS------------------------FDY | WGQGTLVTVSS |
| **iPS:393896** | **21-225_2A4** | **VH3\|3-21/D1\|1-1\|RF2/JH4** | | EVQLVES-GGGLVKPGGSLRLSCAASG-FTFS | S-----YSMN | WVRQAPGKGLEWVS | SISGS---STYIYYADSVKG | RIAISRDNAKNSLYLQMNSLRAEDTAVYYCAR | VAS------------------------FDY | WGQGTLVTVSS |
| **iPS:393914** | **21-225_16B8** | **VH3\|3-21/D1\|1-1\|RF2/JH4** | | EVQLVES-GGGLVKPGGSLRLSCAASG-FTFS | S-----YSMN | WVRQAPGKGLEWIS | SISGS---STYIYYADSVKG | RFTISRDNAKNSLYLQMNSLRAEDTAVYYCAR | VAS------------------------FDY | WGQGTLVTVSS |
| **iPS:393944** | **21-225_14D6** | **VH3\|3-21/D1\|1-1\|RF2/JH4** | | EVQLVES-GGGLVKPGGSLRLSCAASG-FTFS | S-----YTMN | WVRQAPGKGLEWVS | SISGS---GSYIYYADSVKG | RFTMSRDNAKNSLYLQMNSLRAEDTAVYYCAR | VAA------------------------FDS | WGQGTLVSVSS |
| **iPS:393952** | **21-225_1F1** | **VH3\|3-21/D1\|1-1\|RF2/JH4** | | EVQLVES-GGGLVKPGGSLRLSCAASG-FTFS | S-----YNMN | WVRQAPGKGLEWVS | SISSS---SSYIYYADSVKG | RFTISRDNAKNSLYLQMNSLRAEDTAVYYCAR | VNL------------------------FDY | WGQGTLVTVSS |
| **iPS:394033** | **21-225_5F4** | **VH3\|3-21/D1\|1-1\|RF2/JH4** | | EVQLVES-GGGLVKPGGSLRLSCVASG-FTFS | S-----YSMN | WVRQAPGKGLEWVS | SISGS---SSYIYYADSVKG | RFTISRDNAKNSLFLQMNSLRAEDTAVYYCAR | VNN------------------------FDY | WGQGTLVTVSS |
| **iPS:394069** | **21-225_16H1** | **VH3\|3-21/D1\|1-1\|RF2/JH4** | | EVQLVES-GGGLVKPGGSLRLSCAASG-FTFS | S-----YSMN | WVRQAPGKGLEWVS | SISGS---STYIYYADSVKG | RFTISRDNAKNSLYLQMNSLRAEDTAVYYCAR | VAA------------------------FDY | WGQGTLVTVSS |
| **iPS:398482** | **21-225_17H6** | **VH3\|3-21/D1\|1-1\|RF2/JH4** | | EVQLVES-GGGLVKPGGSLRLSCAASG-FTFS | S-----YSMN | WVRQAPGRGLEWVS | SISGS---SSYIYYADSVKG | RFTIFRDNAKNSLYLQMNSLRAEDTAVYYCAR | VAS------------------------FDY | WGQGTLVTVSS |
| **iPS:398492** | **21-225_21F12** | **VH3\|3-21/D1\|1-1\|RF2/JH4** | | EVQLVES-GGGLVKPGGSLRLSCAASG-FTFS | S-----YRMN | WVRQAPGKGLEWVS | SINSY---SSYIYYADSVKG | RFTISRDNAKNSLYLQMNSLRAEDTAVYYCAR | VAS------------------------FDY | WGQGTLVTVSS |
| **iPS:398500** | **21-225_23A11** | **VH3\|3-21/D1\|1-1\|RF2/JH4** | | EVQLVES-GGGLVKPGGSLRLSCAASG-FTFS | S-----YSMN | WIRQAPGKGLEWVS | SISGS---STYIYYADSVKG | RIAISRDNAKNSLYLQMNSLRAEDTAVYYCAR | VAS------------------------FDY | WGQGTLVTVSS |
| **iPS:398526** | **21-225_32B3** | **VH3\|3-21/D1\|1-1\|RF2/JH4** | | EVQLVES-GGGLVKPGGSLRLSCAASG-FTFS | S-----YSMN | WVRQAPGKGLEWVS | SISGS---STYIYYADSVKG | RFTISRDNAKNSLYLQMNSLRAEDTAVYYCAR | VAG------------------------FDY | WGQGTLVTVSS |
| **iPS:402221** | **21-225_2C12** | **VH3\|3-21/D1\|1-** | | EVQLVES-GGGLVKPGGSLRL | S-----YSMN | WVRQAPGKGLEWVS | SISGS---SSYMYYADS | RFTISRDNAKDSLYLQMNSLRAEDTAVYYCAR | VNL------------------------FDY | WGQGTLVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH1\|1-02/D2\|2-15\|RF2/JH6 | | 32258 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | GYCSGGSCYS-------------YYYYYGMDV | WGQGTTVTVSS |
| iPS:434047 | 21-225_50A12 | VH1\|1-02/D2\|2-15\|RF2/JH6 | | QVQLVQS-GAEVKKPGASVKVSCKASG-YTFT | G-----HYIN | WVRQAPGQGPEWMA | WVNPN---SGGTNSAQKFQG | RVTMTRDTSISTVYMELSRLRSDDTAVYYCAR | GGQLGGFN--------------YYYGMDV | WGQGTTVTVSS |
| iPS:434067 | 21-225_51H8 | VH1\|1-02/D2\|2-15\|RF2/JH6 | | QVQLVQS-GAEVKKPGASVKVSCKASG-YTFT | G-----HYMN | WVRQAPGQGLEWMG | WVNPN---SGGSNSAQQFQG | RVTMTRDTSISTVYMELSRLSSDDTAVYYCAR | GGQLGGFN--------------FYYYGMDV | WGQGTTVTVSS |
| iPS:434197 | 21-225_56C7 | VH1\|1-02/D2\|2-15\|RF2/JH6 | | QVQLVQS-GAEVKKPGASVKVSCKASG-YTFT | G-----HYIN | WVRQAPGQGLEWMA | WVNPN---SGGTNSAQKFQG | RVTMTRDTSISTVYMELSRLRSDDTAVYYCAR | GGQLGGFN--------------YYYYGMDV | WGQGTTVTVSS |
| | | Germline | | H_FR1 | H_CDR1 | H_FR2 | H_CDR2 | H_FR3 | H_CDR3 | H_FR4 |
| | VH3\|3-21/D2\|2-15\|RF3/JH6 | | 32259 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | DIVVVVAAT-------------YYYYYGMDV | WGQGTTVTVSS |
| iPS:434049 | 21-225_50B12 | VH3\|3-21/D2\|2-15\|RF3/JH6 | | EVQLVES-GGGLVKPGGSLRLSCAASG-FTFS | S-----HSMN | WVRQAPGKGLEWVS | SISSS---SNYIYYADSVKG | RFTISRDYAKNSLYLQMNSLRAEDTAVYYCAR | DRSIVVAGP-------------WDYYGMDV | WGQGTTVTVSS |
| | | Germline | | H_FR1 | H_CDR1 | H_FR2 | H_CDR2 | H_FR3 | H_CDR3 | H_FR4 |
| | VH3\|3-23/D1\|1-20\|RF1/JH3 | | 32260 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GITGT-------------------DAFDI | WGQGTMVTVSS |
| iPS:434055 | 21-225_51B4 | VH3\|3-23/D1\|1-20\|RF1/JH3 | | EVQLLES-GGGLVQPGGSLSLSCAASG-FTFR | S-----YVMS | WVRQAPGKGLEWVS | AISGR---GSNTFYTDSVKG | RFTISRDNSKNTLYLQMNSLRAEDTAVYYCAK | GITGSH------------------GAFDI | WGQGTMVTVSS |
| | | Germline | | H_FR1 | H_CDR1 | H_FR2 | H_CDR2 | H_FR3 | H_CDR3 | H_FR4 |
| | VH3\|3-23/D7\|7-27\|RF1/JH4 | | 32261 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | LTGY----------------------FDY | WGQGTLVTVSS |
| iPS:434059 | 21-225_51C5 | VH3\|3-23/D7\|7-27\|RF1/JH4 | | EVQLLES-GGGLVQPGGSLRLSCAASG-FTFS | S-----YVMS | WVRQTPGKGLEWVS | TMSGS---GGRTYYADSVNG | RFTVSRDNSKNTLYLQMSSLRAEDTAVYYCAR | VTA------------------------FDY | WGQGTLVTVSS |
| iPS:434213 | 21-225_60A4 | VH3\|3-23/D7\|7-27\|RF1/JH4 | | EVQLLES-GGGLVQPGGSLRLSCAASG-FTFS | S-----YVMS | WVRQAPGKGLEWVS | SISGS---GGWTNYADSVKG | RFTTSRDNSKNTLYLQMNSLRAEDTAVYYCAR | LTG------------------------FDY | WGQGTLVTVSS |
| iPS:434215 | 21-225_60F7 | VH3\|3-23/D7\|7- | | EVQLLES-GGGLVQPGGSLRL | S-----YVMS | WVRQAPGKGLEWVS | GISGS---GNRTYYADS | RFTISRDNSKNTLYLQMNSLRAEDSAVYYCGS | LG--------------------------ID | WGQGTLVTVS |

| | | | | | | | | | | |
|---|---|---|---|---|---|---|---|---|---|---|
| iPS:434241 | 21-225_61E6 | VH3\|3-23/D7\|7-27\|RF1/JH4 | | EVQLLES-GGGLVQPGGSLRLSCAASG-FTFR | S-----YAMS | WVRQAPGKGLEWVS | ATSGS---GVNTFYADSVKG | RFTISRDNSKNTLYLQMNSLRAEDTAVYYCAK | LELG---------------------IFDY | WGQGTLVTVSS |
| iPS:434281 | 21-225_57B8 | VH3\|3-23/D7\|7-27\|RF1/JH4 | | EVQLLES-GGGLVQPGGSLRLSCAASG-FTFR | S-----YAMS | WVRQAPGKGLEWVS | AISGS---GGNTFYADSVKG | RFTISRDNSKNTLYLQMNSLRAEDTAVYYCAK | LELG---------------------IFDY | WGQGTLVTVSS |
| iPS:434301 | 21-225_58F11 | VH3\|3-23/D7\|7-27\|RF1/JH4 | | EVQLLES-GGGLVQPGGSLRLSCAASG-FTFS | S-----YAMS | WVRQAPGKGLEWVS | AISGS---GGNTFYADSVKG | RFTISRYNSKNTLYLQMNSLRAEDTAVYYCAK | FFGMVG------------------AGFFDY | WGQGTLVTVSS |
| iPS:435523 | 21-225_157G5 | VH3\|3-23/D7\|7-27\|RF1/JH4 | | EVQLLES-GGGLVQPGGSLRLSCAASG-FTFS | S-----YVMS | WVRQAPGKGLDWVS | AMSGS---GGRTYYADSVKG | RFTISRDNSKNTLYLQMNSLRAEDTAVYYCAK | YTW-----------------------NGY | WGQGTLVTVSS |
| iPS:435659 | 21-225_167D12 | VH3\|3-23/D7\|7-27\|RF1/JH4 | | EVQLLES-GGGLVQPGGSLRLSCAASG-FTFS | S-----YVMS | WVRQAPGKGLEWVS | AMSGS---GGRTYYADSVKG | RFTISRDNSKNTLYLQMNSLRAEDTAVYYCAK | YTW-----------------------NGY | WGQGTLVTVSS |
| iPS:435765 | 21-225_177D3 | VH3\|3-23/D7\|7-27\|RF1/JH4 | | EVQLLES-GGGLVQPGGSLRLSCAASG-FTFS | S-----YVMN | WVRQAPGKGLEWVS | GMSGS---GGRTYYADSVKD | RFTISRDNSKNTLSLQMNSLRAEDTAVYYCAR | VTF-----------------------FDY | WGQGTLVTVSS |
| iPS:437216 | 21-225_51D5 | VH3\|3-23/D7\|7-27\|RF1/JH4 | | EVQLLES-GGGLVQPGGSLRLSCAASG-FTFS | S-----YVMS | WVRQTPGKGLEWVS | TMSGS---GGRTYYADSVNG | RFTVSRDNSKNTLYLQMSSLRAEDTAVYYCAR | VTA-----------------------FDY | WGQGTLVTVSS |
| | | Germline | | H_FR1 | H_CDR1 | H_FR2 | H_CDR2 | H_FR3 | H_CDR3 | H_FR4 |
| | VH3\|3-21/D5\|5-24\|RF2/JH4 | | 32262 & 32263 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | *RWLQ--------------------LYFDY | WGQGTLVTVSS |
| iPS:434063 | 21-225_51G7 | VH3\|3-21/D5\|5-24\|RF2/JH4 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | ARL------------------------DY | WGQGTLVTVSS |
| | | Germline | | H_FR1 | H_CDR1 | H_FR2 | H_CDR2 | H_FR3 | H_CDR3 | H_FR4 |
| | VH3\|3-23/D7\|7-27\|RF3/JH4 | | 32264 | EVQLLES-GGGLVQPGGSLRLSCAASG-FTFS | S-----YAMS | WVRQAPGKGLEWVS | AISGS---GGSIYYADSVKG | RFTISRDNSKNTLYLQMNSLRAEDTAVYYCAK | NWGY----------------------FDY | WGQGTLVTVSS |
| iPS:434083 | 21-225_52H2 | VH3\|3-23/D7\|7-27\|RF3/JH4 | | EVQLLES-GGGLVQPGGSLRLSCAASG-FTFS | R-----NAMS | WVRQAPGMGLEWVS | AISGR---GGNTFYADSVKG | RFTVSRDNSKNTLFLQMNSLRAEDTAVYYCAK | NGREQ--------------------WLDY | WGQGTLVTVSS |
| | | Germline | | H_FR1 | H_CDR1 | H_FR2 | H_CDR2 | H_FR3 | H_CDR3 | H_FR4 |
| | VH3\|3- | | 32265 | QVQLVES- | S-----YAMH | WVRQAPGK | VISYD--- | RFTISRDNSKNTLYLQMNSL | SIAARYY---------- | WGQGT |

| | | | | | | | | | | |
|---|---|---|---|---|---|---|---|---|---|---|
| **iPS:434087** | **21-225_52F6** | **VH3\|3-30.3/D6\|6-6\|RF2/JH6** | | QVQLVES-GGGVVQPGRSLRLSCAASG-FTFS | S-----YGMH | WVRQAPGKGLEWVA | IISYG---GSNKYYADSVKG | RFTISRDNSKNTLYLQMNSLRADDTAVYYCAR | RSAARP----------------GYGMDV | WGQGTTVTVSS |
| **iPS:434111** | **21-225_53H2** | **VH3\|3-30.3/D6\|6-6\|RF2/JH6** | | QVQLVES-GGGVVQPGRSLRLSCAASG-FTFS | S-----YGMH | WVRQAPGKGPEWVA | VISYG---GSNKYHADSVKG | RFTISRDNSKNTLYLQMNSLRAEDTAVYYCAR | RGAARP----------------GYGMDV | WGQGTTVTVSS |
| **iPS:434121** | **21-225_53F6** | **VH3\|3-30.3/D6\|6-6\|RF2/JH6** | | QVQLVES-GGGVVQPGRSLRLSCAASG-FTFS | S-----YGMH | WVRQAPGKGLEWVA | VISYG---GSNKYDADSVKG | RFTISRDNSKNTLYLQMTSLRAEDTAVYYCAR | RRAARP----------------GYGMDV | WGQGTTVTVSS |
| **iPS:434163** | **21-225_50H1** | **VH3\|3-30.3/D6\|6-6\|RF2/JH6** | | QVQLVES-GGGVVQPGRSLRLSCAASG-FTFS | S-----YGMH | WVRQAPGKGLEWVS | IISYG---GSNKYDADSVKG | RFTISRDNSKNTLYLQMTSLRAEDTAVYYCAR | RRAARP----------------GYGMDV | WGQGITVTVSS |
| **iPS:394035** | **21-225_5G9** | **VH3\|3-30.3/D6\|6-6\|RF2/JH6** | | QVQLVES-GGGVVQPGRSLRLSCAASG-FTFS | N-----YGMH | WVRQAPGKGLEWVA | IISYA---GSNKYYADSVKG | RFTISRDNSKNTLYLQMNSLRAEDTAVYYCVR | RITARL----------------YYGMDV | WGQGTTVTVSS |
| | | **Germline** | | **H_FR1** | **H_CDR1** | **H_FR2** | **H_CDR2** | **H_FR3** | **H_CDR3** | **H_FR4** |
| | **VH3\|3-33/D2\|2-8\|RF1/JH4** | | 32266 & 32267 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RILY*WC----------------MLYYFDY | WGQGTLVTVSS |
| **iPS:434095** | **21-225_52F10** | **VH3\|3-33/D2\|2-8\|RF1/JH4** | | QVQLVES-GGGVVQPGRSLRLSCAASG-FTFS | F-----YGMH | WVRQAPGKGLEWVA | VIWDD---GSNKYYADSVKG | RFTISRDNSKNTLFLQMMSLRAEDTAVYYCAR | DSLYSS----------------SWLFDY | WGQGTLVTVSS |
| | | **Germline** | | **H_FR1** | **H_CDR1** | **H_FR2** | **H_CDR2** | **H_FR3** | **H_CDR3** | **H_FR4** |
| | **VH3\|3-23/D4\|4-23\|RF3/JH4** | | 32268 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | TTVVT----------------------YFDY | WGQGTLVTVSS |
| **iPS:434107** | **21-225_53E2** | **VH3\|3-23/D4\|4-23\|RF3/JH4** | | EVQLLES-GGGLIQPGGSLRLSCAASG-FTFR | S-----YAMS | WVRQAPGKGLEWVS | AISGS---GGNTFYADSVKG | RFTISRDNSKNTLYLQMNTLRADDTAVYYCAK | KVVDTA-------------------MALDY | WGQGTLVTVSS |
| **iPS:434181** | **21-225_56B2** | **VH3\|3-23/D4\|4-23\|RF3/JH4** | | EVQLLES-GGGLIQPGGSLRLSCAASG-FTFR | S-----YAMS | WVRQAPGKGLEWVS | AISGS---GGNTFYADSVKG | RFTISRDNSKNTLYLQMNTLRADDTAVYYCAK | KVVDTA-------------------MALDY | WGQGTLVTVSS |
| | | **Germline** | | **H_FR1** | **H_CDR1** | **H_FR2** | **H_CDR2** | **H_FR3** | **H_CDR3** | **H_FR4** |
| | **VH3\|3-23/D1\|1-26\|RF1/JH3** | | 32269 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GIVGAT-------------------DAFDI | WGQGTMVTVSS |
| **iPS:434117** | **21-225_53C6** | **VH3\|3-23/D1\|1-** | | EVQLLES-GGGLVQPGGSLRL | S-----YAMN | WVRQAPGKGLEWVS | AISGS---GGATYYADS | RFTISRDNSKNTLYLQMNSLRAEDTAVYYCAK | PLVGAH-------------------DAFEI | WGQGTMVTVS |

| | | | | | | | | | | |
|---|---|---|---|---|---|---|---|---|---|---|
| iPS:392984 | 21-225_30E11 | VH3\|3-23/D1\|1-26\|RF1/JH3 | | EVQLLES-GGDMVQPGGSLRLSCAASG-FTFS | I-----YAMS | WVRQAPGKGLEWVS | VISGS---GGSSFYADSVKG | RFTISRDNSKNTLYLQMNSLRAEDTAVYYCTK | DRVKAH-------------------DGFDI | WGQGTMVTVSS |
| iPS:393114 | 21-225_33G12 | VH3\|3-23/D1\|1-26\|RF1/JH3 | | EVQLLES-GGDMVQPGGSLRLSCAASG-FTFS | S-----YAMS | WVRQAPGKGLEWVS | VISGS---GGSSFYADSVKG | RFTISRDNSKNTLYLQMNSLRAEDTAVYYCAK | DRVRAH-------------------DGFDI | WGQGTMVTVSS |
| | | Germline | | H_FR1 | H_CDR1 | H_FR2 | H_CDR2 | H_FR3 | H_CDR3 | H_FR4 |
| | VH3\|3-33/D5\|5-24\|RF2/JH4 | | 322/0 & 322/1 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | *RWLQ--------------------LYFDY | WGQGTLVTVSS |
| iPS:434127 | 21-225_53H8 | VH3\|3-33/D5\|5-24\|RF2/JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | ARIG---------------------YFDS | WGQGTLVTVSS |
| | | Germline | | H_FR1 | H_CDR1 | H_FR2 | H_CDR2 | H_FR3 | H_CDR3 | H_FR4 |
| | VH3\|3-23/D4\|4-17\|RF2/JH4 | | 322/2 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | DYGDY--------------------YFDY | WGQGTLVTVSS |
| iPS:434147 | 21-225_55E1 | VH3\|3-23/D4\|4-17\|RF2/JH4 | | EVQLLES-GGGLVQPGGSLRLSCAASG-FTFS | S-----YAMS | WVRQAPGKGLEWVS | AISGR---GSSTFYADSVKG | RFTISRDNSKNTLYLQMNSLRAEDTAVFYCAK | DHGIVG------------------TIYFDY | WGQGTLVTVSS |
| iPS:435303 | 21-225_146A6 | VH3\|3-23/D4\|4-17\|RF2/JH4 | | EVQLLES-GGGLVQPGGSLRLSCAASG-FTFN | S-----YAMN | WVRQAPGKGLEWVS | AISGS---GGNTFYADSVKG | RFTISRDNSKNTLYLQMNSLRAEDTAVYYCAK | KDNDYVW----------------GSPYFDY | WGQGTLVTVSS |
| iPS:435335 | 21-225_147D10 | VH3\|3-23/D4\|4-17\|RF2/JH4 | | EVQLLES-GGGLVQPGGSLRLSCAASG-FTFS | S-----YAMS | WVRQAPGKGLEWVS | AISGR---GGNTFYADSVKG | RFTISRDNSKNTLYLQMNSLRAEDTAVYYCAK | KDYDYVW----------------GSPYFDY | WGQGTLVTVSS |
| iPS:435339 | 21-225_147D12 | VH3\|3-23/D4\|4-17\|RF2/JH4 | | EVQLLES-GGGLVQSGGSLRLSCAASG-FTFS | S-----YAMS | WVRQAPGKGLEWVS | AISGR---GGNTFYADSVKG | RFTISRDNSKNTLYLQMNSLRAEDTAVYYCAK | KDYDYVW----------------GSPYFDY | WGQGTLVTVSS |
| iPS:435343 | 21-225_148E2 | VH3\|3-23/D4\|4-17\|RF2/JH4 | | EVQLLES-GGGLVQPGGSLRLSCAASG-FTFS | S-----YAMS | WVRQAPGKGLEWVS | AISGR---GGNTFYADSVKG | RFTISRDNSKNTLYLQMNSLRAEDTAVYYCAK | KDYDYVW----------------GSPYFDY | WGQGTLVTVSS |
| iPS:435381 | 21-225_149C6 | VH3\|3-23/D4\|4-17\|RF2/JH4 | | EVQLLES-GGGLVQPGGSLRLSCAASG-FTFS | S-----YAMS | WVRQAPGKGLEWVS | AISGS---GGNTFYADSVKG | RFTISRDNSKNTLYLHMNSLRAEDTAVYYCAK | KDYDYVW----------------GSPYFDY | WGQGTLVTVSS |
| iPS:435391 | 21-225_149F8 | VH3\|3-23/D4\|4- | | EVQLLES-GGGLVQPGGSLRL | S-----YAMS | WVRQAPGKGLEWVS | AISGR---GGNTFYADS | RFTISRDNSKNTLYLQMNSLRAEDTAVYYCAK | KDYDYVW----------------GSPYFDY | WGQGTLVTVS |

| | | | | | | | | | | |
|---|---|---|---|---|---|---|---|---|---|---|
| iPS:4 35395 | 21-225_149D11 | VH3\|3-23/D4\|4-17\|RF2/JH4 | | EVQLLES-GGGLVQPGGSLRLSCAASG-FTFS | S-----YAMS | WVRQAPGKGLEWVS | AISGR---GGNTFYADSVKG | RFTISRDNSKNTLYRQMNSLRAEDTAVYYCAK | KDYDYVW----------------GSPYFDY | WGQGTLVTVSS |
| iPS:4 35403 | 21-225_150C5 | VH3\|3-23/D4\|4-17\|RF2/JH4 | | EVQLLES-GGGLVQPGGSLRLSCAASG-FTFS | S-----YAMS | WVRQAPGKGLEWVS | AISGS---GGNTFYADSVKG | RFTISRDNSKNTLYLQMNSLRAEDTAVYYCAK | KDYDYVW----------------GSPYFDY | WGQGTLVTVSS |
| iPS:4 35447 | 21-225_152H7 | VH3\|3-23/D4\|4-17\|RF2/JH4 | | EVQLLES-GGGLVQPGGSLRLSCAASG-FTFN | S-----YAMN | WVRQAPGKGLEWVS | AISGS---GGNTFYADSVKG | RFTISRDNSKNTLYLQMNSLRAEDTAVYYCAK | KDNDYVW----------------GSPYFDY | WGQGTLVTVSS |
| iPS:4 35453 | 21-225_152G10 | VH3\|3-23/D4\|4-17\|RF2/JH4 | | EVQLLES-GGGLVQPGGSLRLSCAASG-FTFS | S-----YAMS | WVRQAPGKGLEWVS | AISGR---GGNTFYADSVKG | RFTISRDNSKNTLYLQMNSLRAEDTAVYYCAK | KDYDYVW----------------GSPYFDY | WGQGTLVTVSS |
| iPS:4 35483 | 21-225_155A4 | VH3\|3-23/D4\|4-17\|RF2/JH4 | | EVQLLES-GGGLVQPGGSLRLSCAASG-FTFN | S-----YAMS | WVRQAPGKGLEWVS | AISGS---GGNTFYADSVKG | RFTISRDNSKNTLYLQMNSLRAEDTAVYYCAK | KDYDYVW----------------GSPYFDY | WGQGTLVTVSS |
| iPS:4 35485 | 21-225_155B4 | VH3\|3-23/D4\|4-17\|RF2/JH4 | | EVQLLES-GGGLVQPGGSLRLSCAASG-FTFS | S-----YAMS | WVRQAPGKGLEWVS | AISGS---GGNTFYADSVKG | RFTISRDNSKNTLYLQMNSLRAEDTAVYYCAK | KDYDYVW----------------GSPYFDY | WGQGTLVTVSS |
| iPS:4 35777 | 21-225_178F7 | VH3\|3-23/D4\|4-17\|RF2/JH4 | | EVHLLES-GGGLVQTGGSLRLSCAASG-FTFS | S-----YAMT | WVRQAPGKGLEWVS | VISGS---GGNTFYADSVKG | RFTISRDNSKNTLYLQMNSLRAEDTAVYYCAR | RYGD----------------------YFDY | WGQGTLVTVSS |
| iPS:4 35783 | 21-225_179G1 | VH3\|3-23/D4\|4-17\|RF2/JH4 | | EVHLLES-GGGLVQTGGSLRLSCAASG-FTFS | S-----YAMT | WVRQAPGKGLEWVS | VISGF---GGNTFYADSVKG | RFTISRDNSKNTLYLQMNSLRAEDTAVYYCAR | RYGD----------------------YFDY | WGQGTLVTVSS |
| iPS:4 35833 | 21-225_190D12 | VH3\|3-23/D4\|4-17\|RF2/JH4 | | EVQLLES-GGGLVQPGGSLRLSCAASG-FTFS | S-----YAMS | WVRQAPGKGLEWDS | AIIGN---GGRTYYADSVKG | RFTISRDNSKNTLYLQMNSLRAEDTAVYYCAK | DMGRYS-----------------YGFFDY | WGQGTLVTVSS |
| iPS:4 36156 | 21-225_197C8 | VH3\|3-23/D4\|4-17\|RF2/JH4 | | EVQLLES-GGGLVQPGGSLRLSCAASG-FTFS | S-----SAMT | WVRQAPGKGLEWVS | AIIGN---GGRAYYADSVKG | RFTISRDNSKNTLYLQMNSLRTEDTAVYYCAK | DRGYSRIA---------------VAGTFDY | WGQGTLVTVSS |
| | | Germline | | H_FR1 | H_CDR1 | H_FR2 | H_CDR2 | H_FR3 | H_CDR3 | H_FR4 |
| | VH3\|3-21/D1\|1-1\|RF1/JH4 | | 32273 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | GTTGT---------------------YFDY | WGQGTLVTVSS |
| iPS:4 34157 | 21-225_55D4 | VH3\|3-21/D1\|1- | | EVQLVES-GGGLVKPGGSLRL | S-----YRMN | WVRQAPGKGLEWVS | SISSS---SNHIDYADS | RFTISRDNAKNSLYLQMNSLRAEDTALYYCAR | GT--------------------------DY | WGQGTLVSVS |

| | | | | | | | | | | |
|---|---|---|---|---|---|---|---|---|---|---|
| iPS:434243 | 21-225_62C1 | VH3\|3-21/D1\|1-1\|RF1/JH4 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAI | FG-------------------------VD | WGQGTLVTVSS |
| iPS:435505 | 21-225_157C1 | VH3\|3-21/D1\|1-1\|RF1/JH4 | | EVQLVES-GGGLVKPGGSLRLSCAASG-FTFS | S-----YRMN | WVRQAPGKGLEWVS | SMSNS---SSSIYYADSVKG | RFTISRDNAKNSLYLQMNSLRAEDTAVYYCAR | QAA------------------------QDY | WGQGTLVTVSS |
| iPS:392966 | 21-225_32G3 | VH3\|3-21/D1\|1-1\|RF1/JH4 | | EVQLVES-GGGLVKPGGSLRLSCAASG-FTFS | S-----YSMN | WVRQAPGKGLEWVS | SISGS---SSYIYYADSVKG | RFTISRDNAKNSLYLQINSLRAEDTAVYYCAR | GNIA-----------------------RDY | WGQGTLVTVSS |
| | | Germline | | H_FR1 | H_CDR1 | H_FR2 | H_CDR2 | H_FR3 | H_CDR3 | H_FR4 |
| | VH3\|3-33/D7\|7-27\|RF3/JH4 | | 32274 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | NWGY-----------------------FDY | WGQGTLVTVSS |
| iPS:434159 | 21-225_55B8 | VH3\|3-33/D7\|7-27\|RF3/JH4 | | QVQLVES-GGGVVQPGRSLRLSCAASG-FTFS | D-----YGMH | WVRQAPGKGLEWVA | VIWYD---ENNKYYADSVKG | RFTISRDNSKNTLYLQMNSLRAEDTAVYYCTR | EWF-------------------------DY | WGQGTLVTVSS |
| iPS:393026 | 21-225_32B6 | VH3\|3-33/D7\|7-27\|RF3/JH4 | | QVQLVES-GGGVVQPGRSLRLSCAASG-FTFS | D-----YGMH | WVRQAPDKGLEWVA | VIWYD---ENTKYYADSVKG | RFTISRDNSKNTLYLQMNSLRAEDTAVYYCAR | EWG-------------------------DY | WGQGTLVTVSS |
| | | Germline | | H_FR1 | H_CDR1 | H_FR2 | H_CDR2 | H_FR3 | H_CDR3 | H_FR4 |
| | VH3\|3-33/D6\|6-6\|RF3/JH4 | | 32275 & 32276 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | V*QLV---------------------YFDY | WGQGTLVTVSS |
| iPS:434165 | 21-225_50F2 | VH3\|3-33/D6\|6-6\|RF3/JH4 | | QVQLVES-GGGVVQPGRSLRLSCAASA-FTFS | S-----YGMH | WVRQTPGKGLEWVA | VIWHD---GSNKYYADSVKG | RFTISRDNSKNTLYLQMNSLRAEDTAVYYCAR | DEQLG---------------------TFDY | WGQGTLVTVSS |
| iPS:434191 | 21-225_56B6 | VH3\|3-33/D6\|6-6\|RF3/JH4 | | QVQLVES-GGGVVQPGRSLRLSCAASG-FTFS | S-----YGMH | WVRQAPGKGLEWVA | VIWHD---GSNKYYVDSVKG | RFTISRDNSKNTLYLQMNSLRAEDTAVYYCAR | DEQLG---------------------TFDY | WGQGTLVTVSS |
| | | Germline | | H_FR1 | H_CDR1 | H_FR2 | H_CDR2 | H_FR3 | H_CDR3 | H_FR4 |
| | VH1\|1-46/D4\|4-17\|RF2/JH6 | | 32277 | QVQLVQS-GAEVKKPGASVKVSCKASG-YTFT | S-----YYMH | WVRQAPGQGLEWMG | IINPS---GGSTSYAQKFQG | RVTMTRDTSTSTVYMELSSLRSEDTAVYYCAR | DYGDYYY---------------YYYGMDV | WGQGTTVTVSS |
| iPS:434171 | 21-225_50G4 | VH1\|1-46/D4\|4-17\|RF2/JH6 | | QVQLVQS-GAEVKEPGASVKVSCKASG-YTFT | S-----YYIH | WVRQAPGQGLEWMG | VINPS---NGRTSYAQKFQG | RVTMTRDTSTSTVYMELSSLRSEDTAVYYCAR | DRGDGYY---------------FYYGMDV | WGQGTTVTVSS |
| | | Germline | | H_FR1 | H_CDR1 | H_FR2 | H_CDR2 | H_FR3 | H_CDR3 | H_FR4 |
| | VH3\|3- | | 32278 | QVQLVES- | S-----YAMH | WVRQAPGK | VISYD--- | RFTISRDNSKNTLYLQMNSL | DYGDY----------- | WGQGT |

| | | | | | | | | | | |
|---|---|---|---|---|---|---|---|---|---|---|
| iPS:434175 | 21-225_55A11 | VH3\|3-30.3/D4\|4-17\|RF2/JH3 | | QVQLVES-GGGVVQPGRSLRLSCAASG-FTFS | S-----YGMH | WVRQTPGKGLEWVA | VISYV---GSTKYYADSVRG | RFTISRDNSKNTLYLQMNSLRTEDTAVYYCAR | GRGRYSDY---------------GHDAFDI | WGQGTMVTVSS |
| | | Germline | | H_FR1 | H_CDR1 | H_FR2 | H_CDR2 | H_FR3 | H_CDR3 | H_FR4 |
| | VH1\|1-02/D1\|1-1\|RF1/JH2 | | 32279 | QVQLVQS-GAEVKKPGASVKVSCKASG-YIFT | G-----YYMH | WVRQAPGQGLEWMG | WINPN---SGGTNYAQKFQG | RVIMTRDTSISTAYMELSRLRSDDTAVYYCAR | GTTGTY-------------------WYFDL | WGRGTLVTVSS |
| iPS:434193 | 21-225_56C6 | VH1\|1-02/D1\|1-1\|RF1/JH2 | | QVQLVQS-GAEVKKPGASVKVSCKASG-YTFT | D-----YHMH | WVRQAPGQGLEWMG | WINPN---RGGTNYVQKFQG | RVAMTNDTSISTAYMELSGLRSDDTAVYYCAR | DGTS----------------------SFDY | WGRGTLVTVSS |
| | | Germline | | H_FR1 | H_CDR1 | H_FR2 | H_CDR2 | H_FR3 | H_CDR3 | H_FR4 |
| | VH1\|1-02/D5\|5-24\|RF1/JH4 | | 32280 | QVQLVQS-GAEVKKPGASVKVSCKASG-YIFT | G-----YYMH | WVRQAPGQGLEWMG | WINPN---SGGTNYAQKFQG | RVTMTRDTSISTAYMELSRLRSDDTAVYYCAR | VEMAT--------------------IYFDY | WGQGTLVTVSS |
| iPS:434195 | 21-225_56F6 | VH1\|1-02/D5\|5-24\|RF1/JH4 | | QVQLVQS-GAEVKKPGASVKVSCKASG-FTFT | D-----YYMH | WVRQAPGQGLEWMG | WINPN---SGGTNYAQRFQG | RVTMTRDTSISTAYMELSRLRSDDTAVYYCTR | EGATRP-------------------TGFDY | WGQGTLVTVSS |
| | | Germline | | H_FR1 | H_CDR1 | H_FR2 | H_CDR2 | H_FR3 | H_CDR3 | H_FR4 |
| | VH3\|3-33/D1\|1-1\|RF2/JH4 | | 32281 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | VQLER---------------------YFDY | WGQGTLVTVSS |
| iPS:434203 | 21-225_60E2 | VH3\|3-33/D1\|1-1\|RF2/JH4 | | QVQLVES-GGGVVQPGRSLRLSCAASG-FTFN | D-----YGMH | WVRQAPGKGLEWVA | IIWYD---ENNKYYADSVKG | RFTISRDNSKNTLYLQMSSLRAEDTAVYYCAR | DVLD----------------------PFDY | WGQGTLVTVSS |
| iPS:434229 | 21-225_61H1 | VH3\|3-33/D1\|1-1\|RF2/JH4 | | QVQLVES-GGGVVQPGRSLRLSCAASG-FTFN | D-----YGMH | WVRQAPGKGLEWVA | IIWYD---ESNKYYADSVKG | RFTISRDNSKNTLYLQMNSLRAEDTAVYYCAR | DVLD----------------------PFDY | WGQGTLVTVSS |
| | | Germline | | H_FR1 | H_CDR1 | H_FR2 | H_CDR2 | H_FR3 | H_CDR3 | H_FR4 |
| | VH1\|1-02/D5\|5-18\|RF3/JH6 | | 32282 | QVQLVQS-GAEVKKPGASVKVSCKASG-YIFT | G-----YYMH | WVRQAPGQGLEWMG | WINPN---SGGTNYAQKFQG | RVTMTRDTSISTAYMELSRLRSDDTAVYYCAR | GYSYGYYY---------------YYYGMDV | WGQGTTVTVSS |
| iPS:434209 | 21-225_60C3 | VH1\|1-02/D5\|5-18\|RF3/JH6 | | QVQLVQS-GAEVKKPGASVKVSCKASG-YSFT | G-----HYIH | WVRQAPGQGLEYMG | WINPN---SGGTNYVQKFQG | RVTMTRDTSISTAYMELSRLRSDDTAIYYCSR | GGLLGATN--------------YYYYGMDV | WGQGTTVTVSS |
| iPS:434315 | 21-225_59G7 | VH1\|1-02/D5\|5-18\|RF3/JH6 | | QVQLVQS-GAEVKKPGASVKVSCKASG-YSFT | G-----HYIH | WVRQAPGQGLEYMG | WINPN---SGGTNYVQKFQG | RVTMTRDTSISTAYMELSRLRSDDTAIYYCSR | GGLLGATN--------------YYYYGMDV | WGQGTTVTVTS |
| iPS:434319 | 21-225_59B9 | VH1\|1-02/D5\|5- | | QVQLVQS-GPEVKKPGASVKV | G-----NYIH | WVRQAPGQGLEYMG | WINPN---SGGTNYVQK | RVTMTRDTSISTANMELTSLRSDDTAVYYCSR | GGLLGATY--------------YYYYGMDV | WGQGTTVTVS |

| | | | | | | | | | | |
|---|---|---|---|---|---|---|---|---|---|---|
| iPS:4 43003 | 21-225_43F11_LC 2 | VH1\|1-02/D5\|5-18\|RF3/J H6 | | QVQLVQS-GAEVKKPGASVKVSCKASG-YTFT | G-----YYIH | WVRQAPGQGLEWMG | WINPN---SGGTNYAQKFQG | GVTMTRLTSINTAYMDLSRLRSDDTAVYYCAR | GGNYFY------------------NHVMDV | WGQGTPVTVSS |
| iPS:4 43005 | 21-225_43F11_LC 1 | VH1\|1-02/D5\|5-18\|RF3/J H6 | | QVQLVQS-GAEVKKPGASVKVSCKASG-YTFT | G-----YYIH | WVRQAPGQGLEWMG | WINPN---SGGTNYAQKFQG | GVTMTRLTSINTAYMDLSRLRSDDTAVYYCAR | GGNYFY------------------NHVMDV | WGQGTPVTVSS |
| | | Germline | | H_FR1 | H_CDR1 | H_FR2 | H_CDR2 | H_FR3 | H_CDR3 | H_FR4 |
| | VH4\|4-30.4/D5\|5-12\|RF3/JH4 | | 32283 | QVQLQES-GPGLVKPSQTLSLTCTVSG-GSIS | SG---DYYWS | WIRQPPGKGLEWIG | YIYY----SGSTYYNPSLKS | RVTISVDTSKNQFSLKLSSVTAADTAVYYCAR | GYSGYD---------------------YYFDY | WGQGTLVTVSS |
| iPS:4 34217 | 21-225_60E8 | VH4\|4-30.4/D5\|5-12\|RF3/J H4 | | QVQLQES-GPGLVRPSATLSLTCTVSG-GSIS | RS---SYYWG | WIRQPPGKGLEWIG | SIYY----SGSASYNPSLKS | RVTISVDTSENQFSLRLSSVTAADTAVYYCAR | LDSGW-------------------SFDY | WGQGTLVTVSS |
| | | Germline | | H_FR1 | H_CDR1 | H_FR2 | H_CDR2 | H_FR3 | H_CDR3 | H_FR4 |
| | VH3\|3-23/D3\|3-22\|RF3/JH4 | | 32284 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | ITMIVVV----------------ITYFDY | WGQGTLVTVSS |
| iPS:4 34219 | 21-225_60E9 | VH3\|3-23/D3\|3-22\|RF3/J H4 | | EVQLLES-GGGLVQPGGSLRLSCAASG-FTFS | S-----YAMS | WVRQAPGKGLEWVS | AISGS---GGNTFYADSVKG | RFTISRDNSKNTLFLQMNSLRAEDTAVYYCAK | FFGIVG------------------AGYFDY | WGQGTLVTVSS |
| iPS:4 34289 | 21-225_57H12 | VH3\|3-23/D3\|3-22\|RF3/J H4 | | EVQLLES-GGGLVQPGGSLRLSCAASG-LTFS | S-----YAMS | WVRQDPGKGLEWVS | AISGS---GGNTFYGDSVKG | RFTISRDNSKKTLYLQMNSLRAEDTAVYYCAK | FFGIVG------------------AGYFDY | WGQGTLVTVSS |
| iPS:4 34297 | 21-225_58A10 | VH3\|3-23/D3\|3-22\|RF3/J H4 | | EVQLLES-GGGLVQPGGSLRLSCAASG-FTFR | S-----YAMS | WVRQAPGKGLEWVS | AISGS---GGNTFYADSVKG | RFTISRDNSKNTLFLQMNSLRAEDTAVYYCAK | FFGIVG------------------AGYFDY | WGQGTLVTVSS |
| iPS:3 92996 | 21-225_28B1 | VH3\|3-23/D3\|3-22\|RF3/J H4 | | EVQLLES-GGGLVQPGGSLRLSCAASG-FTFS | S-----YAMS | WVRQAPGKGLEWVS | AISGS---GGNTFYADSVKG | RFTISRDNSKNTLYLQMNSLRAEDTAVYYCAK | LGRIAVT-----------------GPYFDY | WGQGTLVTVSS |
| | | Germline | | H_FR1 | H_CDR1 | H_FR2 | H_CDR2 | H_FR3 | H_CDR3 | H_FR4 |
| | VH4\|4-59/D4\|4-11\|RF3/JH4 | | 32285 | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAR | TTVT----------------------YFDY | WGQGTLVTVSS |
| iPS:4 34225 | 21-225_60E12 | VH4\|4-59/D4\|4-11\|RF3/J H4 | | QVQLQES-GPGLVKPSETLSLTCTVSG-GSIS | S-----YFWS | WIRQPAGKGLEWIG | RIYT----RGSTNYNPSLKS | RVTMSVDTSKNQFSLKLSSVTAADTAVYYCAR | EGKTGG------------------VSYFDY | WGQGTLVTVSS |
| iPS:4 34227 | 21-225_61A1 | VH4\|4-59/D4\|4- | | QVQLQES-GPGLVKPSETLSL | S-----HFWS | WIRQPAGKGLEWIG | RIYI----RGSTNYNPS | RVTMSVDTSKNQFSLKLSSVTAADTAVYYCAR | EGKTGG------------------VSYFDY | WGQGTLVTVS |

| | | | | | | | | | | |
|---|---|---|---|---|---|---|---|---|---|---|
| iPS:434267 | 21-225_57F2 | VH4\|4-59/D4\|4-11\|RF3/JH4 | | QVQLQES-GPGLVKPSETLSLTCTVSG-GSIS | S-----YYWS | WIRQPAGKGLEWIG | RIYT----RGSTNYNPSLKS | RVTMSIDTSKNQFSLKLSSVTAADTAVYYCAR | EGKTGG-----------------VSYFDY | WGQGTLVTVSS |
| | | Germline | | H_FR1 | H_CDR1 | H_FR2 | H_CDR2 | H_FR3 | H_CDR3 | H_FR4 |
| | VH3\|3-23/D5\|5-18\|RF3/JH3 | | 32236 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GYSYGY-------------------DAFDI | WGQGTMVTVSS |
| iPS:434239 | 21-225_58F1 | VH3\|3-23/D5\|5-18\|RF3/JH3 | | EVQLLES-GGGLVQPGGSLRLSCAASG-FTFS | S-----YAMS | WVRQAPGKGLEWVS | AISTG---GGNTYYADSVKG | RFTISRDNSKNTLYLQMNSLRAEDTAVYYCAK | RGVYGD-----------------FDAFDI | WGQGTMVTVSS |
| iPS:434309 | 21-225_59B5 | VH3\|3-23/D5\|5-18\|RF3/JH3 | | EVQLLES-GGGLVQPGGSLRLSCAASG-FTFS | S-----YAMN | WVRQAPGKGLEWVS | AISGS---GGNTFYADSVKG | RFTISRDNSKNTLYLQMNSLRAEDTAVYYCAK | RGVYGD-----------------YEAFDI | WGQGTMVTVSS |
| | | Germline | | H_FR1 | H_CDR1 | H_FR2 | H_CDR2 | H_FR3 | H_CDR3 | H_FR4 |
| | VH3\|3-33/D5\|5-18\|RF1/JH4 | | 32237 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | VDTAM--------------------VYFDY | WGQGTLVTVSS |
| iPS:434245 | 21-225_62H1 | VH3\|3-33/D5\|5-18\|RF1/JH4 | | QVQLVES-GGGVVQPGRSLRLSCAASG-FTFS | S-----YGMH | WVRQAPGQGLEWMA | IIWYD---GSNKYYADSVKG | RFTISRDNSKNTLYLQINSLRAEDTAVYYCAR | EDPRT--------------------SCSDY | WGQGTLVTVSS |
| iPS:434323 | 21-225_62H8 | VH3\|3-33/D5\|5-18\|RF1/JH4 | | QVQLVES-GGGVVQPGRSLRLSCAASG-FTFS | T-----YGMH | WVRQAPGKGLEWVA | VIWHD---GSDKYYVDSVKG | RFTISRDNSKNTLYLQMNSLRAEDTAVYYCAR | EDPRT--------------------SCSDY | WGQGTLVTVSS |
| iPS:434379 | 21-225_66A9 | VH3\|3-33/D5\|5-18\|RF1/JH4 | | QVQLVES-GGGVVQPGRSLRLSCAASG-FTFS | S-----YGMH | WVRQAPGKGLEWVA | VIWHD---GSDKYYADSVKG | RFTISRDNSKNTLYLQMNSLRAEDTAVYYCAR | EDPRT--------------------SCSDY | WGQGTLVTVSS |
| | | Germline | | H_FR1 | H_CDR1 | H_FR2 | H_CDR2 | H_FR3 | H_CDR3 | H_FR4 |
| | VH3\|3-33/D1\|1-1\|RF3/JH4 | | 32238 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YNWND---------------------YFDY | WGQGTLVTVSS |
| iPS:434247 | 21-225_62D2 | VH3\|3-33/D1\|1-1\|RF3/JH4 | | QVYLVES-GGGVVQPGRSLRLSCAASG-FTFS | S-----YGMH | WVRQAPGKGLEWVA | VIWYD---GSNKYSADSVKG | RFTISRDNSKNTLDLQMNSLRAEDTAVYYCAR | DNGNW--------------------NYLDY | WGQGTLVTVSS |
| iPS:436838 | 21-225_52H4 | VH3\|3-33/D1\|1-1\|RF3/JH4 | | QVQLVES-GGGVVQPGRSLRLSCAASG-FTFS | H-----FGMH | WVRQAPGKGLKWVA | VIWYD---GSNKYYADSVKG | RFTISRDNSKNTLYLQMNSLRAEDTAVYYCAR | GDWNY--------------------EGFDY | WGQGTLVTVSS |
| iPS:436948 | 21-225_183F5 | VH3\|3-33/D1\|1- | | QVQLVES-GGGVVQPGRSLRL | S-----YGML | WVRQAPGKGLEWVT | VIWYD---GSGKYYADS | RFTISRDNSKNTLYLQMNSLRAEDTAVYYCAR | ENFW----------------------SGDY | WGQGTLVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH4\|4-39/D5\|5-12\|RF3/JH4 | | 32239 | QLQLQES-GPGLVKPSETLSLTCTVSG-GSIS | SS---SYYWG | WIRQPPGKGLEWIG | SIYY----SGSTYYNPSLKS | RVTISVDTSKNQFSLKLSSVTAADTAVYYCAR | GYSGYD--------------------YYFDY | WGQGTLVTVSS |
| iPS:434249 | 21-225_62E2 | VH4\|4-39/D5\|5-12\|RF3/JH4 | | QLQLQES-GPGLVKPSETLSLTCIVSG-GSIS | RS---SYYWG | WIRQPPGKGLEWIG | SIYY----SGIASYNPSLKS | RVTISVDTSKNQFSLKLNSVTATDTAVYYCAR | LSSGW--------------------SFDY | WGQGTLVTVSS |
| iPS:434353 | 21-225_64B12 | VH4\|4-39/D5\|5-12\|RF3/JH4 | | QLQLQES-GPGLVKPSETLSLTCTVSG-VSIS | RS---SYYWG | WIRQPPGKGLEWIG | SIYY----SGSTSYNPSLKS | RVTISVDTSKNQFSLKLSSVTAADTAVYYCAR | LDSGW--------------------SFDY | WGQGTLVTVSS |
| iPS:394073 | 21-225_15C9 | VH4\|4-39/D5\|5-12\|RF3/JH4 | | QLQLQES-GPGLVKPSETLSLTCTVSG-GSIS | RS---SYYWG | WIRQPPGKGLEWIG | NIYY----SGSTYNNPSLKS | RVTISVDTSKNQFSLKLSSVTAADTAVYYCAR | QGSGW--------------------EVDY | WGQGTLVTVSS |
| | | Germline | | H_FR1 | H_CDR1 | H_FR2 | H_CDR2 | H_FR3 | H_CDR3 | H_FR4 |
| | VH1\|1-02/D7\|7-27\|RF1/JH4 | | 32290 | QVQLVQS-GAEVKKPGASVKVSCKASG-YIFT | G-----YYMH | WVRQAPGQGLEWMG | WINPN---SGGTNYAQKFQG | RVTMTRDTSISTAYMELSRLRSDDTAVYYCAR | LTGY----------------------FDY | WGQGTLVTVSS |
| iPS:434259 | 21-225_62G7 | VH1\|1-02/D7\|7-27\|RF1/JH4 | | QVQLVQS-GAEVKKPGASVKVSCKASG-YTFT | G-----YYMH | WVRQAPGQGLEWMG | WIKPK---SGGTNQAQKFQG | RVTMTRDTSISTAYMELSSLRSDDTAVYYCAR | APGIAAAG---------------TWGYFDY | WGQGTLVTVSS |
| iPS:434347 | 21-225_64H10 | VH1\|1-02/D7\|7-27\|RF1/JH4 | | QVQLVQS-GAEVKKPGASVKVSCKASG-YTFT | G-----YYMH | WVRQAPGQGLEWMG | WIKPN---SGGTNQAQKFQG | RVTMTRDTSISTAYMELSGLRSDDTAVYYCAR | APGTAATG---------------TWGYFDY | WGQGTLVTVSS |
| iPS:434359 | 21-225_65G3 | VH1\|1-02/D7\|7-27\|RF1/JH4 | | QVQLVQS-GAEVKKPGASVKVSCKASG-YTFT | G-----YYIH | WVRQAPGQGLEWMG | WINPN---SGGTNSAQKFQG | RVTMTRDTSISTAYMELSRLRSDDTAVYYCAR | APGKAAAG---------------TWGYFDY | WGQGTLVTVSS |
| iPS:434369 | 21-225_66B1 | VH1\|1-02/D7\|7-27\|RF1/JH4 | | QVQLVQS-GAEVKKPGASVKVSCKASG-YTFT | G-----YYMH | WVRQAPGQGLEWMG | WINPN---SGGTNNAQKFQG | RVTMTRATSISTAYMELSRLRSDDTAVYYCAR | APGTAAAG---------------TWGYFDY | WGQGTLVTVSS |
| iPS:434373 | 21-225_66A7 | VH1\|1-02/D7\|7-27\|RF1/JH4 | | QVQLVQS-GAEVKKPGASVKVSCKASG-YTFT | G-----YYMH | WVRQAPGQGLEWMG | WIKPN---SGGTNQAQKFQG | RVTMTRDTSISTGYMELSRLRSDDTAVYYCAR | APGTVAAG---------------TWGYFDY | WGQGTLVTVSS |
| iPS:434397 | 21-225_67H4 | VH1\|1-02/D7\|7-27\|RF1/JH4 | | QVQLVQS-GAEVKKPGASVKVSCKASG-YTFT | G-----YYMH | WVRQAPGQGLEWMG | WIKPN---SGGTNQAQKFQG | RVTMTRDTSISTAYMELSGLRSDDTAVYYCAR | APGTAATG---------------TWGYFDY | WGQGTLVTVSS |
| iPS:434427 | 21-225_70D6 | VH1\|1-02/D7\|7- | | QVQLVQS-GAEVKKPGASVKV | G-----YYMH | WVRQAPGQGLEWMG | WINPK---SGGTNSAQK | RVSMTRDTSIGTAYMELRGLRSDDTAEYYCAR | APGKAAAG---------------TWGFFDY | WGQGTLVTVS |

| | | | | | | | | | | |
|---|---|---|---|---|---|---|---|---|---|---|
| iPS:434435 | 21-225_70G9 | VH1\|1-02/D7\|7-27\|RF1/JH4 | | QVQLVQS-GAEVKKPGASVKVSCKASG-YTFT | G-----YYMH | WVRQAPGQGLEWMG | WIKPN---SGGTNQAQKFQG | RVTMTRDTSISTAYMELSSLRSDDTAVYYCAR | APGIAAAG---------------TWGYFDY | WGQGTLVTVSS |
| iPS:434437 | 21-225_70A12 | VH1\|1-02/D7\|7-27\|RF1/JH4 | | QVQLVQS-GAEVKKPGASVKVSCKASG-YTFT | G-----YYMH | WVRQAPGQGLEWMG | WIKPN---SGGTNQAQKFQG | RVTMTRDTSISTAYMELSGLRSDDTAVYYCAR | APGTAATG---------------TWGYFDY | WGQGTLVTVSS |
| iPS:434451 | 21-225_71B7 | VH1\|1-02/D7\|7-27\|RF1/JH4 | | QVQLVQS-GAEVKKPGASVKVSCKASG-YIFT | G-----YYMH | WVRQAPGQGLEWMG | WINPN---SGGTNSAQKFQG | RVTMTRDTSIGTAYMELSGLRSDDTAVYYCAR | APGKAAAG---------------TWGFFDY | WGQGTLVTVSS |
| iPS:434459 | 21-225_71A7 | VH1\|1-02/D7\|7-27\|RF1/JH4 | | QVQLVQS-GAEVKKPGASVKVSCKASG-YTFT | G-----YYMH | WVRQAPGQGLEWMG | WINPK---SGGTNYVQKFQG | RVTMTRDTSISTAYMELSSLRSDDTAVYYCAR | APGTAPAG---------------SWGYFDY | WGQGTLVTVSS |
| iPS:434461 | 21-225_73A3 | VH1\|1-02/D7\|7-27\|RF1/JH4 | | QVQLVQS-GAEVKKPGASVKVSCKASG-YTFT | G-----YYMH | WVRQAPGQGLDWMG | WINPK---SGGTNHVQKFQG | RVAMTRDTSISTAYMELSSLRSDDTAVYYCAR | APGTAAAG---------------SWGCFDY | WGQGTLVTVSS |
| | | Germline | | H_FR1 | H_CDR1 | H_FR2 | H_CDR2 | H_FR3 | H_CDR3 | H_FR4 |
| | VH3\|3-23/D4\|4-11\|RF3/JH4 | | 32291 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | TTVT-----------------------YFDY | WGQGTLVTVSS |
| iPS:434261 | 21-225_56F7 | VH3\|3-23/D4\|4-11\|RF3/JH4 | | EVQLLES-GGGLVQPGGSLRLSCAASG-FTFS | S-----YVLN | WVRQAPGKGLEWVS | AMSGS---GGRTYYADSVKG | RFTISRDNSKNTLYLQMNSLRAEDTAVYFCAM | TTH------------------------FDY | WGQGTLVTVSS |
| iPS:435461 | 21-225_153A1 | VH3\|3-23/D4\|4-11\|RF3/JH4 | | EVQLLES-GGGLVQPGGSLRLSCAASG-FSFS | S-----YVMS | WVRQGPGKGLEWVS | AISGS---GDRTYYADSVKG | RFTISRDNSKNTLYLQMNSLRAEDTAVYYCAR | TAT------------------------KDY | WGQGTLVTVSS |
| iPS:435509 | 21-225_157H1 | VH3\|3-23/D4\|4-11\|RF3/JH4 | | EVQLLES-GGGLVQPGGSLRLSCAASG-FTFS | S-----YAMR | WIRQAPGKGLQWVS | DISGS---GGTTYYADSVKG | RFTISRDNSKNTLYLQMNSLRAEDTAVYYCAK | TY---------------------------L | WGQGTLVTVSS |
| iPS:435747 | 21-225_175C4 | VH3\|3-23/D4\|4-11\|RF3/JH4 | | EVQLLES-GGGLVQPGGSLRLSCAASA-FTFS | S-----YVMS | WVRQAPGKGLEWVS | AISGS---GDRTYYADSVKG | RFTISRDDSNTTLYLQMNSLRAEDTAVYYCAR | TAG------------------------FDY | WGQGTLVTVSS |
| iPS:392784 | 21-225_23C7 | VH3\|3-23/D4\|4-11\|RF3/JH4 | | EVQLLES-GGGLVQPGGSLRLSCAASG-FTFS | S-----YVMS | WVRQAPGKGLEWVS | AMSGS---GGSTYYVDSVKG | RFTISRDNSKNTLYLQMNSLRAEDTAVYYCAR | TGV------------------------FDY | WGQGTLVIVSS |
| iPS:392802 | 21-225_23E7 | VH3\|3-23/D4\|4- | | EVQLLES-GGGLVQPGGSLRL | S-----YAMN | WVRQAPGKGLEWVS | AISGS---GGFTYYADS | RFTISRDNSRNTLYLQMNSLRAEDTAVYYCAR | TSG------------------------FDY | WGQGTLVTVS |

| | | | | | | | | | | |
|---|---|---|---|---|---|---|---|---|---|---|
| **iPS:3 92962** | **21-225_30A1** | **VH3\|3-23/D4\|4-11\|RF3/JH4** | | EVQLLES- GGGLVQPGGSLRL SCAASG-FTFS | S-----YVMN | WVRQAPGK GLEWVS | AISGS--- GGSTYYADS VKG | RFTISRNNSKNTLYLQMNSL RAEDTAVYYCAR | TGV------------- -----------FDY | WGQGT LVTVS S |
| **iPS:3 93090** | **21-225_33A5** | **VH3\|3-23/D4\|4-11\|RF3/JH4** | | EVQLLES- GGGLVQPGGSLRL SCAASG-FTFS | S-----YVIN | WVRQAPGK GLEWVS | AISGS--- GVSTYYADS VKG | RFTISRDNSKNTLYLQMNSL RAEDTAVYYCAR | TSL------------- -----------FDY | WGQGT LVTVS S |
| | | **Germline** | | **H_FR1** | **H_CDR1** | **H_FR2** | **H_CDR2** | **H_FR3** | **H_CDR3** | **H_FR4** |
| | **VH3\|3-23/D1\|1-1\|RF3/JH4** | | 32292 | EVQLLES- GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | YNWND----------- ----------YFDY | WGQGT LVTVS S |
| **iPS:4 34273** | **21-225_57E4** | **VH3\|3-23/D1\|1-1\|RF3/JH4** | | EVQLLES- GGGLVQPGGSLRL SCAASG-FTFS | S-----YAMS | WVRQAPGK GLEWVS | VISGS--- GGSTFYADS VKG | RFTISRDNSKTTLYLQMNSL RAEDTAVYYCAK | RDWND----------- ----------VFDY | WGQGT LVTVS S |
| | | **Germline** | | **H_FR1** | **H_CDR1** | **H_FR2** | **H_CDR2** | **H_FR3** | **H_CDR3** | **H_FR4** |
| | **VH3\|3-23/D3\|3-3\|RF3/JH4** | | 32293 | EVQLLES- GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | ITIFGVV--------- --------IIYFDY | WGQGT LVTVS S |
| **iPS:4 34279** | **21-225_57F7** | **VH3\|3-23/D3\|3-3\|RF3/JH4** | | EVQLLES- GGGLVQPGGSLRL SCAASG-FTFS | S-----YAMS | WVRQAPGK GLEWVS | AISGS--- GGNTFYADS VKG | RFTISRDNSKNTLYLQMNSL RAEDTAVYYCAK | FFGVVG---------- --------VGCFDY | WGQGT LVTVS S |
| **iPS:4 37228** | **21-225_60C11** | **VH3\|3-23/D3\|3-3\|RF3/JH4** | | EVQLLES- GGGLVQPGGSLRL SCAASG-FPFS | S-----YAMS | WVRQAPGK GLEWVS | AISGS--- GGNTFYADS VKG | RFTISRDNSKNTLYLQMNSL RAEDTAVYYCAK | FFGVVG---------- --------VGCFDY | WGQGT LVTVS S |
| | | **Germline** | | **H_FR1** | **H_CDR1** | **H_FR2** | **H_CDR2** | **H_FR3** | **H_CDR3** | **H_FR4** |
| | **VH3\|3-23/D3\|3-22\|RF3/JH1** | | 32294 | EVQLLES- GGGLVQPGGSLRL SCAASG-FTFS | S-----YAMS | WVRQAPGK GLEWVS | AISGS--- GGSTYYADS VKG | RFTISRDNSKNTLYLQMNSL RAEDTAVYYCAK | ITMIVVVI-------- -------TAEYFQH | WGQGT LVTVS S |
| **iPS:4 34291** | **21-225_58A4** | **VH3\|3-23/D3\|3-22\|RF3/JH1** | | EVQLLES- GGGLVQPGGSLRL SCAASG-LTFS | S-----YAMS | WVRQDPGK GLEWVS | AISGS--- GGNTFYGDS VKG | RFTISRDNSKKTLYLQMNSL RAEDTAVYYCAK | FFGIVG---------- --------AGFFDS | WGQGT LVTVS S |
| | | **Germline** | | **H_FR1** | **H_CDR1** | **H_FR2** | **H_CDR2** | **H_FR3** | **H_CDR3** | **H_FR4** |
| | **VH3\|3-33/D2\|2-15\|RF3/JH4** | | 32295 | QVQLVES- GGGVVQPGRSLRL SCAASG-FTFS | S-----YGMH | WVRQAPGK GLEWVA | VIWYD--- GSNKYYADS VKG | RFTISRDNSKNTLYLQMNSL RAEDTAVYYCAR | DIVVVVA--------- --------AIYFDY | WGQGT LVTVS S |
| **iPS:4 34299** | **21-225_58D11** | **VH3\|3-33/D2\|2-15\|RF3/JH4** | | QVQLVES- GGGVVQPGRSLRL SCAASG-FTFS | D-----YDIH | WVRQSPGK GLEWVA | VIWYD--- GSKKYYADS VKG | RFTISRDNSKNTLYLQMNSL RAEDTALYYCAR | DRVT------------ -----------FDY | WGQGT LVTVS S |
| **iPS:4 34871** | **21-225_85H1** | **VH3\|3-33/D2\|2-** | | QVQLVES- GGGVVQPGRSLRL | S-----YGIH | WVRQAPGK GLEWVA | VIWYD--- GSNKYYADS | RFTISRDNSKNTLYLQMNSL RAEDTAVYYCAR | DPFIVG---------- --------ATYFDY | WGQGT LVTVS |

| | | | | | | | | | | |
|---|---|---|---|---|---|---|---|---|---|---|
| iPS:435109 | 21-225_92H5 | VH3\|3-33/D2\|2-15\|RF3/JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DPFIVG------------------ATYFDY | WGQGTLVTVSS |
| iPS:436434 | 21-225_216B10 | VH3\|3-33/D2\|2-15\|RF3/JH4 | | QVQLVES-GGGVVQPGRSLRLSCAASG-FTFS | S-----YGMH | WVRQAPGKGLEWVA | AIWYD---GSNKYYADSVKG | RFTISRDNSKRTLYLQMNSLRAEDTAVYYCAR | DPNIVG------------------ATWFDY | WGQGTLVTVSS |
| | | Germline | | H_FR1 | H_CDR1 | H_FR2 | H_CDR2 | H_FR3 | H_CDR3 | H_FR4 |
| | VH1\|1-02/D4\|4-17\|RF2/JH4 | | 32296 | QVQLVQS-GAEVKKPGASVKVSCKASG-YIFI | G-----YYMH | WVRQAPGQGLEWMG | WINPN---SGGTNYAQKFQG | RVTMTRDTSISTAYMELSRLRSDDTAVYYCAR | DYGDY---------------------YFDY | WGQGTLVTVSS |
| iPS:434307 | 21-225_59B2 | VH1\|1-02/D4\|4-17\|RF2/JH4 | | QVQLVQS-GAEVKKPGASVKVSCKASG-YTFA | G-----YYIH | WVRQAPGQGLEWLG | WINPN---SGGTNYAQKFQG | RVTMTRDTSISTAYMELSRLRFDDTAVYYCAR | DPGP-----------------------FDY | WGQGTLVTVSS |
| | | Germline | | H_FR1 | H_CDR1 | H_FR2 | H_CDR2 | H_FR3 | H_CDR3 | H_FR4 |
| | VH3\|3-33/D3\|3-22\|RF3/JH6 | | 32297 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | IIMIVVVIT------------YYYYYGMDV | WGQGTTVTVSS |
| iPS:434311 | 21-225_59H5 | VH3\|3-33/D3\|3-22\|RF3/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | ERGIAVG-----------------YYGMDV | WGQGTTVTVSS |
| | | Germline | | H_FR1 | H_CDR1 | H_FR2 | H_CDR2 | H_FR3 | H_CDR3 | H_FR4 |
| | VH4\|4-39/D1\|1-26\|RF3/JH4 | | 32298 | QLQLQES-GPGLVKPSETLSLTCTVSG-GSIS | SS---SYYWG | WIRQPPGKGLEWIG | SIYY----SGSTYYNPSLKS | RVTISVDTSKNQFSLKLSSVTAADTAVYYCAR | YSGSY--------------------YYFDY | WGQGTLVTVSS |
| iPS:434313 | 21-225_59E6 | VH4\|4-39/D1\|1-26\|RF3/JH4 | | QLQLQES-GPGLVKPSETLSLTCTVSG-GSIS | RS---SYYWG | WIRQPPGKGLEWIG | NIYY----SGSTYYNPSLKS | RVTISVDTSKNQFSLKLSSVTAADTALYYCAR | HSSSW---------------------SLDY | WGQGTLVTVSS |
| iPS:434413 | 21-225_68D12 | VH4\|4-39/D1\|1-26\|RF3/JH4 | | QLQLQES-GPGLVKPSETLSLTCTVSG-GSIS | RS---SYYWG | WIRQPPGKGLEWIG | NIYY----SGSTYYIPSLKS | RVTISVDTSKNQFSLKLTSVTAADTAVYYCAR | HSTSW---------------------SIDY | WGQGTLVTVSS |
| iPS:392628 | 21-225_20C2 | VH4\|4-39/D1\|1-26\|RF3/JH4 | | QLQLQES-GPGLVKPSETLSLTCTVSG-GSIS | RS---SYYWG | WIRQPPGKGLEWIG | NIYY----SGTAYCNSSLKS | RVIISVDTSKNQFSLKLSSVTATDTAVYYCAR | HSSSW---------------------SLDN | WGQGTLVTVSS |
| iPS:392642 | 21-225_18C6 | VH4\|4-39/D1\|1-26\|RF3/JH4 | | QLQLQES-GPGLVKPSETLSLTCTVSG-GSIS | RS---SYYWG | WIRQPPGKGLEWIG | NIYY----SGYTYYNPSLKS | RVIISVDTSKNQFSLKLSSVTAADTALYYCAR | HSSSW---------------------SLDD | WGQGTLVTVSS |
| iPS:392706 | 21-225_18A3 | VH4\|4-39/D1\|1- | | QLQLQES-GPGLVKFSETLSL | RS---SYYWG | WIRQPPGKGLEWIG | NIYY----SGYTYYTPS | RVTISVDTSKNQFSLKLSSVTAADTAVYYCAR | HSTSW---------------------SLDY | WGQGTLVTVS |

| | | | | | | | | | | |
|---|---|---|---|---|---|---|---|---|---|---|
| **iPS:392800** | **21-225_22D12** | **VH4\|4-39/D1\|1-26\|RF3/JH4** | | QLQLQES-GPGLVKPSETLSLTCTVSG-GSIS | RS---SYYWG | WIRQPPGKGLEWIG | NIYY----SGTTSYNPSLKS | RVTISVDTSRNQFSLKLSSVTAADTAVFYCAR | LSSSW---------------------SVDY | WGQGTLVTVSS |
| **iPS:392820** | **21-225_23D1** | **VH4\|4-39/D1\|1-26\|RF3/JH4** | | QLQLQES-GPGLVKPSETLSLTCTVSG-GSIS | RS---SYYWG | WIRQPPGKGLEWIG | SIYY----SGSAQYNPSLKS | RVTISVDTSKNQFSLTLSSVTAADTALYYCAR | LSSSW---------------------SFDY | WGQGTLVTVSS |
| **iPS:392824** | **21-225_24E5** | **VH4\|4-39/D1\|1-26\|RF3/JH4** | | QLQLQES-GPGLVKPSETLSLTCTVSG-GAIS | RS---SYYWG | WIRQPPGKGLEWIG | SIYY----SGSANYNPSLKS | RVTISVDTSKNQFSLKLSSVTAADTAVYYCAR | LSSSW---------------------SIDN | WGQGTLVTVSS |
| **iPS:392834** | **21-225_22C1** | **VH4\|4-39/D1\|1-26\|RF3/JH4** | | QLQLQES-GPGLVKPSETLSLTCTVSG-GSIN | RS---SYYWG | WIRQPPGKGLEWIG | NIYY----SGATYYNSSLKS | RVTISVDTSTNQFSLKLSSVTAADTAVYYCAR | HSGSW---------------------SLDY | WGQGTLVTVSS |
| **iPS:392870** | **21-225_20G9** | **VH4\|4-39/D1\|1-26\|RF3/JH4** | | QLQLQES-GPGLVKPSETLSLTCTVSG-GSIS | RS---SYYWG | WIRQPPGKGLEWIG | SIYY----SGSASYNPSLKS | RVTISVDTSRNQFSLKLSSVTAADTAAYYCAR | LSSSW---------------------SFDY | WGQGTLVTVSS |
| **iPS:392896** | **21-225_21G7** | **VH4\|4-39/D1\|1-26\|RF3/JH4** | | QLQLQES-GPGLVKFSETLSLTCTVSG-GSIS | RS---SYYWG | WIRQPPGKGQEWIG | NIYY----SGYSYYNPSLKS | RVTISVDTSKNQFSLKLSSVTAADTAVYYCAR | HSTSW---------------------SLDY | WGQGTLVTVSS |
| **iPS:392904** | **21-225_22G9** | **VH4\|4-39/D1\|1-26\|RF3/JH4** | | QLQLQES-GPGLVKPSETLSLTCTVSG-GAIS | GS---NYYWG | WIRQPPGKELEWIG | NIYY----SGSTYYNPSLKS | RVTISVDTSKNQFSLKLRSVTAEDTAVYYCAR | HSSSW---------------------SLDY | WGQGTLVTVSS |
| **iPS:393094** | **21-225_34C4** | **VH4\|4-39/D1\|1-26\|RF3/JH4** | | QLQLQES-GPGLVKPSETLSLTCTVSG-GSIS | RS---SYYWG | WIRQSPGKGLEWIG | SIYY----SGSTAYNPSLKS | RVTISVDTSKNQVSLKLSSVTAADTAVYYCAR | LSSSW---------------------SFDY | WGQGTLVTVSS |
| **iPS:393806** | **21-225_6A6** | **VH4\|4-39/D1\|1-26\|RF3/JH4** | | QLQLQES-GPGLVKPSETLSLTCTVSG-GSIS | RS---SYYWG | WIRQPPGKGLEWIG | NIYY----SGIPYYNPSLKS | RVNISVDTSKNQFSLKLNSVTAADTAVYYCAR | HSSSW---------------------SLDY | WGQGTLVTVSS |
| **iPS:393814** | **21-225_7F4** | **VH4\|4-39/D1\|1-26\|RF3/JH4** | | QLQLQES-GPGLVKPSETLSLTCTVSG-GSIS | RS---SYYWG | WIRQPPGKGLEWIG | NIYY----SGATYYNPSLKS | RVTISVDTSTNQFSLKLSSVTAADTAVYYCAR | HSGSW---------------------SLDY | WGQGTLVTVSS |
| **iPS:393816** | **21-225_6D4** | **VH4\|4-39/D1\|1-26\|RF3/JH4** | | QLQLQES-GPGLVKPSETLSLTCTVSG-GSIS | RS---SSYWG | WIRQPPGKGLEWIG | NIYY----SGSAYYIPSLKS | RVTISVATSKNQFSLNLTSVTAADTAVYYCAR | HSSSW---------------------SLDC | WGQGTLVTVSS |
| **iPS:393880** | **21-225_15A1** | **VH4\|4-39/D1\|1-** | | QLHLQES-GPGLVKPSETLSL | RS---SYYWG | WIRQPPGKGLEWIG | SIYY----SGSAQYNPS | RVTISVDTTKNQFSLTLSSVTAADTAVYYCAR | LSSSW---------------------SFDY | WGQGTLVTVS |

| | | | | | | | | | | |
|---|---|---|---|---|---|---|---|---|---|---|
| iPS:3 94002 | 21-225_15G7 | VH4\|4-39/D1\|1-26\|RF3/JH4 | | QLQLQES-GPGLVKPSETLSLTCTVSG-GSIS | RS---SSYWG | WIRQPPGKGLEWIG | SIYY----SGYTYYTPSLKS | RVTISVDTSKNQFSLRLSSVTAADTASYYCAR | LSSSW-----------<br>----------SFDF | WGQGTLVTVSS |
| iPS:3 94053 | 21-225_11F10 | VH4\|4-39/D1\|1-26\|RF3/JH4 | | QLHLQES-GPGLVKPSETLSLTCTVSG-ASIS | RS---SYYWG | WIRQPPGKGLEWIG | SIYY----SGSAQYNPSLKS | RVTISVDTSKNQFSLTLSSVTAADTAVYYCAR | LSSSW-----------<br>----------SFDY | WGQGTLVTVSS |
| iPS:3 94057 | 21-225_15H1 | VH4\|4-39/D1\|1-26\|RF3/JH4 | | QLQLQES-GPGLVKFSETLSLTCTVSG-GSIS | RS---SYYWG | WIRQPPGKGLEWIG | NIYY----SGYPYYNPSLKS | RVTISVDTSKNQFSLKLSSVTAADTAVYYCAR | HSTSW-----------<br>----------SLDY | WGQGTLVTVSS |
| iPS:3 94063 | 21-225_16A1 | VH4\|4-39/D1\|1-26\|RF3/JH4 | | QLQLQES-GPGLVKPSETLSLICTVSG-GSID | RS---SYYWG | WIRQPPGKGLEWIG | SIYY----SGSAYHNPSLKS | RGTISVDTSKNQFSLKLSSVTAADTAAYYCAR | LSSSW-----------<br>----------SFDY | WGQGTLVTVSS |
| iPS:3 94075 | 21-225_8D12 | VH4\|4-39/D1\|1-26\|RF3/JH4 | | QLQLQES-GPGLVKFSETLSLTCTVSG-GSIS | RS---SYYWG | WIRQPPGKGLEWIG | NIYY----SGYPYYNPSLKS | RVTISIDTSKNQFSLKLSSVTAADTAVYYCAR | HSTSW-----------<br>----------SLDY | WGQGTLVTVSS |
| | | Germline | | H_FR1 | H_CDR1 | H_FR2 | H_CDR2 | H_FR3 | H_CDR3 | H_FR4 |
| | VH3\|3-48/D7\|7-27\|RF3/JH4 | | 32299 | EVQLVES-GGGLVQPGGSLRLSCAASG-FTFS | S-----YSMN | WVRQAPGKGLEWVS | YISSS---SSTIYYADSVKG | RFTISRDNAKNSLYLQMNSLRDEDTAVYYCAR | NWGY------------<br>-----------FDY | WGQGTLVTVSS |
| iPS:4 34317 | 21-225_59E8 | VH3\|3-48/D7\|7-27\|RF3/JH4 | | EVQLVES-GGGLVQPGGSLRLSCAASG-FTFS | S-----YSMN | WVRQAPGKGLGWVS | YISSS---SGTIYYADSVKG | RFTISRDNAKNSLYLQMNSLRDEDTAVYYCAR | EWGMAV----------<br>--------AGPFDY | WGQGTLVTVSS |
| | | Germline | | H_FR1 | H_CDR1 | H_FR2 | H_CDR2 | H_FR3 | H_CDR3 | H_FR4 |
| | VH3\|3-33/D1\|1-26\|RF3/JH4 | | 32300 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YSGSY-----------<br>---------YYFDY | WGQGTLVTVSS |
| iPS:4 34327 | 21-225_63G6 | VH3\|3-33/D1\|1-26\|RF3/JH4 | | QVQLVES-GGGVVQPGRSLRLSCAASG-FTFS | S-----YGMH | WVRQAPGKGLEWVT | VIWYD---GSNKYYADSVKG | RFTISRDNSKNTLYLQMNSLRAEDTAVYYCAR | DSLSGI----------<br>--------AAAFDY | WGQGTLVTVSS |
| iPS:4 37084 | 21-225_206B5 | VH3\|3-33/D1\|1-26\|RF3/JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYNCAR | EGGSY-----------<br>----------HLDY | WGQGILVTVSS |
| iPS:4 37088 | 21-225_209H10 | VH3\|3-33/D1\|1-26\|RF3/JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYNCAR | EGGSY-----------<br>----------HLDY | WGQGILVTVSS |
| iPS:3 92684 | 21-225_17F4 | VH3\|3-33/D1\|1- | | QVQLVES-GGGVVQPGRSLRL | N-----YGMN | WVRQAPGKGLEWVA | VIWYD---GNNKHYADS | RFTISRDNSKNTLYLQMNSLRAEDTAVYYCAS | SGSY------------<br>----------FFDY | WGQGTLVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH1\|1-02/D6\|6-19\|RF2/JH4 | | 32301 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | GIAVA------------------GYFDY | WGQGTLVTVSS |
| iPS:434333 | 21-225_63C9 | VH1\|1-02/D6\|6-19\|RF2/JH4 | | QVQLVQS-GAEVKKPGASVKVSCKASG-YTFT | G-----YYMH | WVRQAPGQGLEWMG | WINPN---SGGTNFAQKFQG | RVTMTRDASINTAYMELRSLISDDTAVYYCAR | APGVAAAG---------------SWGYFDY | WGQGTLVTVSS |
| | | Germline | | H_FR1 | H_CDR1 | H_FR2 | H_CDR2 | H_FR3 | H_CDR3 | H_FR4 |
| | VH3\|3-33/D4\|4-11\|RF2/JH4 | | 32302 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DYSNY-------------------YFDY | WGQGTLVTVSS |
| iPS:434335 | 21-225_63C10 | VH3\|3-33/D4\|4-11\|RF2/JH4 | | QVQLVES-GGGVVQPGRSLRLSCAASG-FTFS | S-----YGMH | WVRQAPGKGLDWVA | VIWYD---GSNKYYADSVKG | RFTISRDNSKNTLYLQMNSLRAEDTAVYYCAR | DDPRS-------------------SAGDY | WGQGTLVTVSS |
| iPS:434429 | 21-225_70H6 | VH3\|3-33/D4\|4-11\|RF2/JH4 | | QVQLVES-GGGVVQPGRSLRLSCAASG-FTFS | S-----YGMH | WVRQTPGKGLDWVA | VIWHD---GSNKYYADSVKG | RFTISRDNSKNTLYLQMNSLRAEDTAVYYCAR | DDPRS-------------------SAGDY | WGQGTLVTVSS |
| iPS:434569 | 21-225_77H5 | VH3\|3-33/D4\|4-11\|RF2/JH4 | | QVQLVES-GGGVVQPGRSLRLSCAASG-FTFS | N-----YGMH | WVRQAPGKGLEWVA | VIWYD---GRNKYYADSVKG | RFTISRDNSKNTLYLQMNSLRAEDTALYYCAR | DRSILG------------------ATFFDY | WGQGTLVTVSS |
| iPS:434629 | 21-225_74C3 | VH3\|3-33/D4\|4-11\|RF2/JH4 | | QVQLVES-GGGVVQPGRSLRLSCAASG-FTFS | S-----FGMH | WARQAPGKGLEWVA | AIWYD---GSNKYCADSVKG | RFTISRDNSKNTLSLQMNSLRAEDSAVYYCAR | DRSILG------------------AAFFDY | WGQGTLVTVSS |
| iPS:435793 | 21-225_180F8 | VH3\|3-33/D4\|4-11\|RF2/JH4 | | QVQLVES-GGGVVQPGRSLRLSCAASG-FTFS | S-----YGMH | WVRQAPGKGLEWVS | VIWYD---GSDKYYEDSVKG | RFTISRDNSKNTLYLQMNSLRAEDTAVYYCAR | DHPRW-------------------SYGDY | WGQGTLVTVSS |
| iPS:436382 | 21-225_212C10 | VH3\|3-33/D4\|4-11\|RF2/JH4 | | QVQLVES-GGGVVQPGRSLRLSCAASG-FTFS | S-----YGMH | WVRQAPGKGLEWVT | AIWYD---GSHKYYTDSVKG | RFTISRDNSKNTLYLQMNSLRAEDTAVYYCAR | DRSIVG------------------ATYFDY | WGQGTLVTVSS |
| iPS:392660 | 21-225_19B3 | VH3\|3-33/D4\|4-11\|RF2/JH4 | | QVQLVES-GGGVVQPGRSLRLSCAASG-FTFS | S-----YDMH | WVRQAPGKGLEWVA | VIWYD---GSDKYYADSVKG | RFTISRDNSKNTLFLQMNSLRAEDTAVYYCAR | DRAYS-------------------SSSDY | WGQGTLVTVSS |
| iPS:393904 | 21-225_8H11 | VH3\|3-33/D4\|4-11\|RF2/JH4 | | QVQLVES-GGGVVQPGRSLRLSCAASG-FTFS | T-----YNMH | WVRQAPGKGLEWVA | VIWYD---GSDRYSADSVKG | RFTISRDNSKNTLYLQMNSLRAEDTAVYYCAR | DRAYS-------------------SSSDF | WGQGTLVTVSS |
| | | Germline | | H_FR1 | H_CDR1 | H_FR2 | H_CDR2 | H_FR3 | H_CDR3 | H_FR4 |
| | VH4\|4-59/D4\|4- | | 32303 | QVQLQES- | S-----YYWS | WIRQPPGK | YIYY---- | RVTISVDTSKNQFSLKLSSV | DYSNY----------- | WGQGT |

| | | | | | | | | | | |
|---|---|---|---|---|---|---|---|---|---|---|
| iPS:434341 | 21-225_64F7 | VH4\|4-59/D4\|4-11\|RF2/JH4 | | QVQLQES-GPGLVKPSETLSLTCTVSG-GSIS | S-----YFWS | WIRQPAGKGLEWIG | RIYT----SGISNYNPSLKS | RVTMSVDTSKNQFSLKLSSVTAADTAVYYCAR | FSSG-----------------------FFDY | WGQGTLVTVSS |
| | | Germline | | H_FR1 | H_CDR1 | H_FR2 | H_CDR2 | H_FR3 | H_CDR3 | H_FR4 |
| | VH3\|3-33/D7\|7-27\|RF2/JH1 | | 32304 & 32305 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | *LGAE----------------------YFQH | WGQGTLVTVSS |
| iPS:434351 | 21-225_64A12 | VH3\|3-33/D7\|7-27\|RF2/JH1 | | QVQLVES-GGGVVQPGRSLRLSCAASG-FTFS | S-----YGMH | WVRQAPGKGLEWVA | VIWYD---ESNKYYVDSVKG | RFTISRDNSKNTLYLQMNSLRAEDTAVYYCAR | ELGF-----------------------LSDH | WGQGTLVTVSS |
| iPS:392700 | 21-225_16E12 | VH3\|3-33/D7\|7-27\|RF2/JH1 | | QVQLVES-GGGLVQPGRSLRLSCAASG-FTFS | N-----YGMH | WVRQAPGKGLEWVA | VIWYE---GSNKYYVDSVRG | RFTISRDNSKSTLYLQMNSLRAEDTAVYYCVR | ELGF-----------------------QSDH | WGQGTPVTVSS |
| iPS:392710 | 21-225_19A10 | VH3\|3-33/D7\|7-27\|RF2/JH1 | | QVQLVES-GGGVVQPGRSLRLSCAASG-FTFS | S-----YGMH | WVRQAPGKGLEWVA | VIWYD---ESNKYYADSVKG | RFTISRDNSKNTLYLQMNSLRAEDTAVYYCAR | ELAW-----------------------YEDS | WGQGTLVTVSS |
| iPS:394093 | 21-225_9D12 | VH3\|3-33/D7\|7-27\|RF2/JH1 | | QVQLVES-GGGVVQPGRSLRLSCAASG-FTFS | D-----YGMH | WVRQAPGKGLEWVA | VIWYD---GNNNYYADSVKG | RFTISRDNSKNTLYLQMNSLRAEDTAVYYCAR | ELAW-----------------------YEDF | WGQGTLVTVSS |
| | | Germline | | H_FR1 | H_CDR1 | H_FR2 | H_CDR2 | H_FR3 | H_CDR3 | H_FR4 |
| | VH3\|3-23/D3\|3-22\|RF2/JH3 | | 32306 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | YYYDSSGY---------------YYDAFDI | WGQGTMVTVSS |
| iPS:434355 | 21-225_64G12 | VH3\|3-23/D3\|3-22\|RF2/JH3 | | EVQLLES-GGGLVQPGGSLRLSCAASG-FTFS | S-----NAMS | WVRQAPGKGLEWVS | VISGS---GGSTYYADSVKG | RFTISRDNSKNTLYLQMNSLRAEDTALYYCAK | RNYDD----------------------AFDI | WGQGTMVTVSS |
| | | Germline | | H_FR1 | H_CDR1 | H_FR2 | H_CDR2 | H_FR3 | H_CDR3 | H_FR4 |
| | VH3\|3-21/D4\|4-11\|RF3/JH4 | | 32307 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | ITVT-----------------------YFDY | WGQGTLVTVSS |
| iPS:434367 | 21-225_65H11 | VH3\|3-21/D4\|4-11\|RF3/JH4 | | EVQLVES-GGGLVKPGGSLRLSCAASG-FTFS | S-----YRMN | WVRQAPGKGLEWVS | SISSS---NSSIYYADSVKG | RFTTSRDNAKNSLYLQMNSLRAEDTAVYYCTS | TS--------------------------GS | WGQGTLVTVSS |
| iPS:437220 | 21-225_55H6 | VH3\|3-21/D4\|4-11\|RF3/JH4 | | EVHLVES-GGGLVKPGGSLRLSCAASG-FTFS | S-----YSMN | WVRQAPGKGLEWVS | SISGS---STYIYYADSVKG | RFTISRDNAKNSLYLQMNSLRAEDTAVYYCAR | TGV-------------------------FDY | WGQGTLVTVSS |
| iPS:402237 | 21-225_23D11 | VH3\|3-21/D4\|4- | | EVQLVES-GGGLVKPGGSLRL | S-----YNIN | WVRQAPGKGLEWVS | SISGN---SGYIYYADS | RFTISRDNAKNSLYLQMNSLRAEDTAVYYCAR | TNL-------------------------FDY | WGQGTLVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH3\|3-21/D4\|4-11\|RF3/JH3 | | 32308 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | TTVID-----------------------AFDI | WGQGTMVTVSS |
| iPS:434383 | 21-225_66F9 | VH3\|3-21/D4\|4-11\|RF3/JH3 | | EVQLVES-GGGLVKPGGSLRLSCAASG-FTFS | S-----YSMN | WVRQAPGKGLEWVS | SISGT---SSYIYYADSVKG | RFTISRDNAKNSLYLQMNGLRAEDTAVYFCAR | TNA------------------------FDI | WGQGTMVTVSS |
| iPS:434449 | 21-225_71H6 | VH3\|3-21/D4\|4-11\|RF3/JH3 | | EVQLVES-GGGLVKPGGSLRLSCAASG-FTFS | S-----YSMN | WVRQAPGKGLEWVS | SISGT---SSYIYYADSVKG | RFTISRDNAKNSLYLQMNGLRAEDTAVYFCAK | TNA------------------------FDI | WGQGTMVTVSS |
| | | Germline | | H_FR1 | H_CDR1 | H_FR2 | H_CDR2 | H_FR3 | H_CDR3 | H_FR4 |
| | VH3\|3-33/D6\|6-6\|RF2/JH4 | | 32309 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | SIAAR---------------------YFDY | WGQGTLVTVSS |
| iPS:434399 | 21-225_67B7 | VH3\|3-33/D6\|6-6\|RF2/JH4 | | QVQLVES-GGGVVQPGRSLRLSCAASG-FTFS | N-----YGMH | WVRQAPGKGLEWVA | VILYD---GSKKYYADSVKG | RFTISRDNSKNTLYLQMNSLRAEDTAVYYCAR | SIPE-----------------------FDY | WGQGTLVTVSS |
| iPS:434463 | 21-225_73A6 | VH3\|3-33/D6\|6-6\|RF2/JH4 | | QVQLVES-GGGVVQPGRSLRLSCAASG-FTFS | Y-----YGMH | WVRQAPGKGLEWVA | VILYD---GSKKYYAASVKG | RFTISRDNSKNTLYLQMNSLRAEDTAVYYCAR | SIPD-----------------------FDY | WGQGTLVTVSS |
| | | Germline | | H_FR1 | H_CDR1 | H_FR2 | H_CDR2 | H_FR3 | H_CDR3 | H_FR4 |
| | VH3\|3-21/D1\|1-26\|RF3/JH4 | | 32310 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | YSGSY--------------------YYFDY | WGQGTLVTVSS |
| iPS:434405 | 21-225_68E6 | VH3\|3-21/D1\|1-26\|RF3/JH4 | | EVQLVES-GGGLVKPGGSLRLSCAASG-FTLS | S-----FGMN | WVRQAPGKGLEWVS | YISRS---SSHIYYADSVKG | RFTISRDNAKNSLYLQMNSLRAEDTAVYYCAV | SSGS----------------------PFDY | WGQGTLVTVSS |
| iPS:435595 | 21-225_160H4 | VH3\|3-21/D1\|1-26\|RF3/JH4 | | EVQLVES-GGGLVKSGGSLRLSCAASG-FTFS | S-----YRMN | WVRQAPGKGLEWVS | SISGS---SSYIDYADSVKG | RFTISRDNAKNSLYLQMNSLRAEDTAVYYCAR | KSW------------------------FDY | WGQGTLVTVSS |
| iPS:435635 | 21-225_163F1 | VH3\|3-21/D1\|1-26\|RF3/JH4 | | EVQLVDS-GGGLVKPGGSLRLSCAASG-FTFS | S-----YSMN | WVRQAPGKGLEWVS | SISGS---GSYIYYADSVKG | RFTTSRDNAKNSLYLQMNSLSADDTAVYYCTL | YSS------------------------SHY | WGQGTLVTVSS |
| | | Germline | | H_FR1 | H_CDR1 | H_FR2 | H_CDR2 | H_FR3 | H_CDR3 | H_FR4 |
| | VH3\|3-30.3/D2\|2-15\|RF3/JH4 | | 32311 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | DIVVVVA-----------------ATYFDY | WGQGTLVTVSS |
| iPS:434417 | 21-225_69C8 | VH3\|3-30.3/D2\|2- | | QVQLVES-GGGVVQPGRSLRL | N-----YAMH | WVRQAPGKGLEWVA | VIWYD---GSDKYYADS | RFTISRDNSKNTLYLQMISLRAEDTAVYYCAR | DIPSN-------------------SAGDY | WGQGTLVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH3\|3-33/D3\|3-10\|RF1/JH4 | | 32312 & 32313 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | VLLWFGE---------------LL*YFDY | WGQGTLVTVSS |
| iPS:434433 | 21-225_70E8 | VH3\|3-33/D3\|3-10\|RF1/JH4 | | QVQLVES-GGGVVQPGRSLRLSCAASG-FTFS | S-----YGML | WVRQAPGKGLEWVA | IIWYD---ESNKYYADSVKG | RFTISRDNSKNTLYLQMNSLRAEDTAVYYCAR | DLLD--------------------PRDY | WGQGTLVTVSS |
| | | Germline | | H_FR1 | H_CDR1 | H_FR2 | H_CDR2 | H_FR3 | H_CDR3 | H_FR4 |
| | VH3\|3-23/D6\|6-13\|RF1/JH4 | | 32314 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GYSSSW------------------YYFDY | WGQGTLVTVSS |
| iPS:434467 | 21-225_73H8 | VH3\|3-23/D6\|6-13\|RF1/JH4 | | EVQLLES-GGGWVQSGGSLRLSCAASG-FTFS | S-----NAMS | WVRQAPGKGLEWVS | DISRS---GGTTFYADSVKG | RFTISRDNSKNTLYLQMNSLRAEDTAVYYCAK | WDSSSWY----------------DVTPFDY | WGQGTLVTVSS |
| | | Germline | | H_FR1 | H_CDR1 | H_FR2 | H_CDR2 | H_FR3 | H_CDR3 | H_FR4 |
| | VH4\|4-34/D4\|4-17\|RF2/JH4 | | 32315 | QVQLQQW-GAGLLKPSETLSLTCAVYG-GSFS | G-----YYWS | WIRQPPGKGLEWIG | EINH----SGSTNYNPSLKS | RVTISVDTSKNQFSLKLSSVTAADTAVYYCAR | DYGDY---------------------YFDY | WGQGTLVTVSS |
| iPS:434471 | 21-225_75G3 | VH4\|4-34/D4\|4-17\|RF2/JH4 | | QVQLQQW-GAGLLKPSETLSLTCAVYG-GSLS | G-----SYWS | WIRQPPGKGLEWIG | EINL----SGSTNYNPSLKS | RVTISVDTSKSQFSLTLRSVTAADTAVYYCAR | DYGG----------------------LDY | WGQGTLVTVSS |
| iPS:434517 | 21-225_76A7 | VH4\|4-34/D4\|4-17\|RF2/JH4 | | QVQLQQW-GAGLLKPSETLSLTCAVYG-GSFS | G-----CYWS | WIRQPPGKGLEWIG | EINH----SGRTNYNPSLKS | RVTISVDTSENQFSLKLSSVTAADTAVYYCAR | DYGG----------------------LDY | WGQGALVTVSS |
| iPS:434519 | 21-225_74C7 | VH4\|4-34/D4\|4-17\|RF2/JH4 | | QVQLQQW-GAGLLKPSETLSLTCAVYG-GSLS | G-----SYWS | WIRQPPGKGLEWIG | EINL----SGSTNYNPSLKS | RVTISVDTSKSQFSLTLRSVTAADTAVYYCAR | DYGG----------------------LDY | WGQGTLVTVSS |
| iPS:434571 | 21-225_74D2 | VH4\|4-34/D4\|4-17\|RF2/JH4 | | QVQLQQW-GAGLLKPSETLSLTCAVYG-GSFS | G-----CYWS | WIRQPPGKGLEWIG | EINH----SGRTNYNPSLKS | RVTISVDTSENQFSLKLSSVTAADTAVYYCAR | DYGG----------------------LDY | WGQGTLVTVSS |
| iPS:434637 | 21-225_78E7 | VH4\|4-34/D4\|4-17\|RF2/JH4 | | QVQLQQW-GAGLLKPSETLSLTCAVYG-GSLS | G-----SYWS | WIRQPPGKGLEWIG | EINL----SGSTNYNPSLKS | RVTISVDTSKSQFSLTLRSVTAADTAVYYCAR | DYGG----------------------LDY | WGQGTLVTVSS |
| iPS:434717 | 21-225_80A6 | VH4\|4-34/D4\|4-17\|RF2/JH4 | | QVQLQQW-GAGLLKPSETLSLTCAVYG-GSFS | G-----CYWS | WIRQPPGKGLEWIG | EINH----SGRTNYNPSLKS | RVTISVDTSEKQFSLKLSSVTAADTAVYYCAR | DYGG----------------------LDY | WGQGTLVTVSS |
| iPS:434735 | 21-225_80B10 | VH4\|4-34/D4\|4- | | QVQLQQW-GAGLLKPSETLSL | G-----CYWS | WIRQPPGKGLEWIG | EINH----SGSTNYNPS | RVTISVDTSKNQFSLKLSSVTAADTAVYYCAR | DYGG----------------------LDY | WGQGTLVTVS |

| | | | | | | | | | | |
|---|---|---|---|---|---|---|---|---|---|---|
| iPS:4 34835 | 21-225_83B6 | VH4\|4-34/D4\|4-17\|RF2/J H4 | | QVQLQQW-GAGLLKPSETLSLTCAVYG-GSFS | G-----CYWS | WIRQPPGKGLEWIG | EINH----SGRTNYNPSLKS | RVTISVDTSENQFSLKLSSVTAADTAVYYCAR | DYGG-----------------------LDY | WGQGTLVTVSS |
| iPS:4 34849 | 21-225_83C10 | VH4\|4-34/D4\|4-17\|RF2/J H4 | | QVQLQQW-GAGLLKPSETLSLTCTVYG-GSFS | G-----YYWS | WIRQPPGKGLEWIG | EINH----SGSTNFNPSLKS | RVTISVDTSKNQFSLKLSSVTAADTAVFYCAR | DYGG-----------------------LDY | WGQGTLVTVSS |
| iPS:4 34891 | 21-225_85G6 | VH4\|4-34/D4\|4-17\|RF2/J H4 | | QVQLQQW-GAGLLKPSETLSLTCAVYG-GSFS | D-----CYWS | WIRQPPGKGLEWIG | EINH----SGRTNYNPSLKS | RVTISVDTSENQFSLKLSSVTAADTAVYYCAR | DYGG-----------------------LDY | WGQGTLVTVSS |
| iPS:4 35183 | 21-225_93E9 | VH4\|4-34/D4\|4-17\|RF2/J H4 | | QVQLQQW-GAGLLKPSETLSLTCAVYG-GSFS | G-----CYWS | WIRQPPGKGLEWIG | EINH----SGRTNYNPSLKS | RVTISVDTSENKFSLKLSSVTAADTAVYYCAR | DYGG-----------------------LDY | WGQGTLVTVSS |
| iPS:4 35331 | 21-225_147G8 | VH4\|4-34/D4\|4-17\|RF2/J H4 | | QVQLQQW-GAGLLKPSETLSLTCGVYG-GSFS | A-----YYWS | WIRQPPGKGLEWIG | EINH----SGSTNYKPSLKS | RVTISVDTSKNQFSLKLSSVTAADTAVYYCAR | DYGV-----------------------FDY | WGQGTLVTVSS |
| iPS:4 35995 | 21-225_192F8 | VH4\|4-34/D4\|4-17\|RF2/J H4 | | QVQLQQW-GAGLLKPSETLSLTCAVYG-GSFS | G-----CYWS | WIRQPPGKGLEWIG | EINQ----SGRSNYNPSLKS | RVTISVDTSTNQFSLKLRSVTAADTAVYYCAR | DYGV-----------------------FDY | WGQGTLVTVSS |
| iPS:4 36027 | 21-225_193E6 | VH4\|4-34/D4\|4-17\|RF2/J H4 | | QVQLQQW-GAGLLKPSETLSLTCAVFG-GSFS | G-----PYWS | WIRQPPGKGLEWIG | ESNH----SGRTNYNPSLKS | RVTISVDTSKNQFSLRLSSVTAADTAVYYCAR | DYGG-----------------------LDY | WGQGTLVTVSS |
| iPS:4 36080 | 21-225_195B1 | VH4\|4-34/D4\|4-17\|RF2/J H4 | | QVQLQQW-GAGLLKPSETLSLTCAVSG-GSFR | Y-----YFWS | WIRQSPGKGLEWFG | EINH----SGRTNYNPSLKS | RVTISVDTSKNQFSLKLRSVTAADTAVYYCAR | DYGA-----------------------FDI | WGQGTLVTVSS |
| iPS:4 36232 | 21-225_201E1 | VH4\|4-34/D4\|4-17\|RF2/J H4 | | QVQLQQW-GAGLLKPSETLSLTCAVFD-GSFS | P-----YYWS | WIRQPPGKGLEWIG | EVNH----SGSTNYNPSLKS | RVTISVDTSKNQFSLKLSSVTAADTAVYYCAR | DYGG-----------------------LDY | WGQGALVTVSS |
| iPS:4 36238 | 21-225_201B2 | VH4\|4-34/D4\|4-17\|RF2/J H4 | | QVQLQQW-GAGLLKPSETLSLTCAVFG-GSIS | V-----YYWT | WIRQPPGKGLEWIG | EINH----SGSTNYNPSLKS | RVTISVDTSKNQFSLKLSSVTAADTAVYYCAR | DYGV-----------------------FDY | WGQGTLVTVSS |
| iPS:4 36256 | 21-225_202D9 | VH4\|4-34/D4\|4-17\|RF2/J H4 | | QVQLQQW-GAGLLKPSETLSLTCAVYD-GSFS | P-----YYWS | WIRQPPGKGLEWIG | EINH----SGSTNYNPSLKS | RVTISVDTSKNQFSLKLSSVTAADTAVYYCAR | DYGG-----------------------LDY | WGQGTLVTVSS |
| iPS:4 36302 | 21-225_205G7 | VH4\|4-34/D4\|4- | | QVQLQQW-GAGLLKPSETLSL | V-----YYWS | WIRQPPGKGLEWIG | ESNQ----SGRTTYNPS | RVTISVDTSKNQFSLNLISVTAADTAVYYCAR | DYGV-----------------------FDY | WGQGTLVTVS |

| | | | | | | | | | | |
|---|---|---|---|---|---|---|---|---|---|---|
| iPS:4 36310 | 21-225_202D11 | VH4\|4-34/D4\|4-17\|RF2/JH4 | | QVQLQQR-GAGLLKPSETLSLTCAAYG-GSFS | G-----PYWS | WIRQPPGKGLEWIG | EINH----SGSTNYNPSLKS | RVTISVDTSKNQFSLKLSSVTAADTAVYYCAR | DYGV----------------------LDY | WGQGTLVTVSS |
| iPS:4 36336 | 21-225_208B5 | VH4\|4-34/D4\|4-17\|RF2/JH4 | | QVQLQQW-GAGLLKPSETLSLTCAVFG-GSIS | V-----YYWT | WIRQPPGKGLEWIG | EINH----SGSTNYNPSLKS | RVTISVDTSKNQFSLKLSSVTAADTAVYYCAR | DYGV----------------------FDY | WGQGTLVTVSS |
| iPS:4 36340 | 21-225_208A9 | VH4\|4-34/D4\|4-17\|RF2/JH4 | | QVQLQQW-GAGLLKPSEPLSLTCAVYG-GSFS | V-----SYWS | WIRQPPGKGLEWIG | EINH----SGRANYNPSLKS | RVTISIDTSKNQFSLKLSSVTAADTAVYYCAR | DYGV----------------------LDY | WGQGTLVTVSS |
| iPS:4 37340 | 21-225_75G9 | VH4\|4-34/D4\|4-17\|RF2/JH4 | | QVQLQQW-GAGLLKPSETLSLTCAVYG-GSFS | G-----CYWS | WIRQPPGKGLEWIG | EINH----SGRTNYNPSLKS | RVTISVDTSENQFSLKLSSVTAADTAVYYCAR | DYGG----------------------LDY | WGQGTLVTVSS |
| iPS:4 51122 | 21-225_200A1 | VH4\|4-34/D4\|4-17\|RF2/JH4 | | QVQLQQW-GAGLLKPSETLSLTCAVYG-GSFS | V-----YYWS | WIRQPPGKGLEWIG | EINH----SGSTNYNPSLKS | RVTISLDTSKNQFSLKLSSVTAADTAVYYCAR | DYGV----------------------FDY | WGQGTLVTVSS |
| | | Germline | | H_FR1 | H_CDR1 | H_FR2 | H_CDR2 | H_FR3 | H_CDR3 | H_FR4 |
| | VH3\|3-33/D2\|2-15\|RF3/JH1 | | 32316 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DIVVVVAA---------------TAEYFQH | WGQGTLVTVSS |
| iPS:4 34485 | 21-225_76D2 | VH3\|3-33/D2\|2-15\|RF3/JH1 | | QVQLVES-GGGVVQPGRSLRLSCAASG-FTFS | S-----YGMH | WVRQAPGKGLEWVA | VIWYD---GSNKYYADSVKG | RFTISRDNSKNTLFLQMNSLRAEDTAVYYCAS | DRNIVG------------------ATYFES | WGQGTLVTVSS |
| iPS:4 34537 | 21-225_74E11 | VH3\|3-33/D2\|2-15\|RF3/JH1 | | QVQLVES-GGGVVQPGRSLRLSCAASG-FTFS | S-----YGMH | WVRQAPGKGLEWVA | VIWYD---GSNKYYADSVKG | RFTISRDNSKNTLFLQMNSLRAEDTAVYYCAS | DRNIVG------------------ATYFES | WGQGTLVTVSS |
| iPS:4 34673 | 21-225_74E3 | VH3\|3-33/D2\|2-15\|RF3/JH1 | | QVQLVES-GGGVVQPGRSLRLSCTASG-FTFS | S-----YGMH | WVRQAPGKGLEWVA | VIWYD---GSNKYYADSVKG | RFTISRDNSKNTLFLQMNSLRAEDTAVYYCAS | DRNIVG------------------ATYFES | WGQGTLVTVSS |
| iPS:4 35221 | 21-225_95G2 | VH3\|3-33/D2\|2-15\|RF3/JH1 | | QVQLVES-GGGVVQPGRSLRLSCTASG-FTFS | S-----YGMH | WVRQAPGKGLEWVA | VIWYD---GSNKYYADSVKG | RFTISRDNSKNTLFLQMNSLRAEDTAVYYCAS | DRNIVG------------------ATYFES | WGQGTLVTVSS |
| | | Germline | | H_FR1 | H_CDR1 | H_FR2 | H_CDR2 | H_FR3 | H_CDR3 | H_FR4 |
| | VH4\|4-39/D7\|7-27\|RF1/JH5 | | 32317 | QLQLQES-GPGLVKPSETLSLTCTVSG-GSIS | SS---SYYWG | WIRQPPGKGLEWIG | SIYY----SGSTYYNPSLKS | RVTISVDTSKNQFSLKLSSVTAADTAVYYCAR | LIGN---------------------WFDP | WGQGTLVTVSS |
| iPS:4 34503 | 21-225_74D7 | VH4\|4-39/D7\|7- | | QLQLQES-GPGLVKPSETLSL | RS---SYYWG | WIRQPPGKGLEWIG | GIYY----SGSTSYNPS | RVTISVDTSENQFSLKLSSVTAADTAVYYCAR | LRPNW---------------------DFDY | WGQGTLVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH3\|3-15/D1\|1-1\|RF2/JH4 | | 32318 | EVQLVES-GGGLVKPGGSLRLSCAASG-FTFS | N-----AWMS | WVRQAPGKGLEWVG | RIKSKT-DGGTTDYAAPVKG | RFTISRDDSKNTLYLQMNSLKTEDTAVYYCTT | VQLER---------------------YFDY | WGQGTLVTVSS |
| iPS:434531 | 21-225_76C9 | VH3\|3-15/D1\|1-1\|RF2/JH4 | | EVQLVES-GGGLVKPGGSLRLSCAASG-FSFS | N-----AWMN | WVRQAPGKGLEWVG | RIKNKA-DGGTTDFAAPVKG | RFTISRDDSKHTLYLQMNSLKTEDTAVYYCTT | VGPT----------------------TDY | WGQGTLVTVSS |
| iPS:434633 | 21-225_74G8 | VH3\|3-15/D1\|1-1\|RF2/JH4 | | EVQLVES-GGGLVKPGGSLRLSCAASG-FTFS | N-----AWMN | WVRQAPGKGLEWVG | RIKNKA-DGGTTDYAAPVKG | RFTISRDDSKNTLYLQMNSLKTEDTAVYYCTT | VGAT----------------------TDY | WGQGTLVTVSS |
| iPS:434671 | 21-225_74F4 | VH3\|3-15/D1\|1-1\|RF2/JH4 | | EVQLVES-GGGLVKPGGSLRLSCAASG-FTFS | N-----AWMN | WVRQAPGKGLEWVG | RIKNKI-DGGTTDYAAPVKG | RFTISRDDSKNTLYLQMNSLKTEDTAVYYCTT | VGAT----------------------TDY | WGQGTLVTVSS |
| iPS:437383 | 21-225_74H8 | VH3\|3-15/D1\|1-1\|RF2/JH4 | | EVHLVES-GGGLVKPGGSLRLSCAASG-FTFS | N-----AWMN | WVRQAPGKGLEWVG | RIKSKT-DGGTTDYAAPVKG | RFTISRDDSKNTLYLQMNSLKTEDTAVYYCTT | VGAT----------------------TDY | WGQGTLVTVSS |
| | | Germline | | H_FR1 | H_CDR1 | H_FR2 | H_CDR2 | H_FR3 | H_CDR3 | H_FR4 |
| | VH3\|3-33/D5\|5-18\|RF3/JH4 | | 32319 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | GYSYG-------------------YYFDY | WGQGTLVTVSS |
| iPS:434535 | 21-225_74C8 | VH3\|3-33/D5\|5-18\|RF3/JH4 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DGSYGY-------------------DGLDY | WGQGTLVTVSS |
| iPS:434573 | 21-225_77E6 | VH3\|3-33/D5\|5-18\|RF3/JH4 | | QVQLVES-GGGVVQSGRSLRLSCAASG-FTFS | S-----YGMH | WVRQAPGKGLEWVA | VIWYD---GSNQNYADSVKG | RFTISRDNSKNTLFLQMNSLRAEDTAVYYCAR | DGSYGY-------------------DGLDY | WGQGTLVTVSS |
| iPS:434615 | 21-225_76C5 | VH3\|3-33/D5\|5-18\|RF3/JH4 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DGSYGY-------------------DGLDY | WGQGTLVTVSS |
| iPS:434669 | 21-225_79F4 | VH3\|3-33/D5\|5-18\|RF3/JH4 | | QVQLVES-GGGVVQSGRSLRLSCAASG-FTFS | N-----YGMH | WVRQAPGKGLEWVA | VIWYD---GSNQNYADSVKG | RFTISRDNSKNTLFLQMNSLRAEDTAVYYCAR | DGSYGY-------------------DGLDY | WGQGTLVTVSS |
| iPS:434737 | 21-225_74G6 | VH3\|3-33/D5\|5-18\|RF3/JH4 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DGSYGY-------------------DGLDY | WGQGTLVTVSS |
| iPS:434741 | 21-225_80C11 | VH3\|3-33/D5\|5- | | QVQLVES-GGGVVQPGRSLRL | S-----YGMH | WVRQAPGKGLEWVA | VIWYD---GSNKNYADS | RFTISRDNSKNTLSLQMNSLRAEDTAVYYCAR | DGSYGY-------------------DGLDY | WGQGTLVTVS |

| | | | | | | | | | | |
|---|---|---|---|---|---|---|---|---|---|---|
| iPS:4 34867 | 21-225_79A12 | VH3\|3-33/D5\|5-18\|RF3/JH4 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DGSYGY-------------------DGLDY | WGQGTLVAVSS |
| | | Germline | | H_FR1 | H_CDR1 | H_FR2 | H_CDR2 | H_FR3 | H_CDR3 | H_FR4 |
| | VH4\|4-34/D3\|3-10\|RF2/JH6 | | 32320 | QVQLQQW-GAGLLKPSETLSLTCAVYG-GSFS | G-----YYWS | WIRQPPGKGLEWIG | EINH----SGSTNYNPSLKS | RVTISVDTSKNQFSLKLSSVTAADTAVYYCAR | YYYGSGSYYN-----------YYYYYGMDV | WGQGTTVTVSS |
| iPS:4 34539 | 21-225_74A2 | VH4\|4-34/D3\|3-10\|RF2/JH6 | | QVQVQQW-GAGLLKPSETLSLTCAVYG-GSFT | D-----YYWS | WIRQPPGKGLEWIG | EINH----SGDTNYNPSLKS | RVTISVDTSKNQFSLKLSSVTAADTAVYYCAR | EFPYSGSY--------------LYYYGMDV | WGQGTTVTVSS |
| iPS:4 37248 | 21-225_97H3 | VH4\|4-34/D3\|3-10\|RF2/JH6 | | QVQVQQW-GAGLLKPSETLSLTCAVYG-GSFT | D-----YYWS | WIRQPPGKGLEWIG | EINH----SGDTNYNPSLKS | RVTISVDTSKNQFSLKLSSVTAADTAVYYCAR | EFPYSGSY--------------LYYYGMDV | WGQGTTVTVSS |
| iPS:4 37320 | 21-225_75A1 | VH4\|4-34/D3\|3-10\|RF2/JH6 | | QVQVQQW-GAGLLKHSETLSLTCAVYG-GSFT | D-----YYWS | WIRQPPGKGLEWIG | EINH----SGDTNYNPSLKS | RVTISVDTSKNQFSLKLSSVTAADTAVYYCAR | EFPYSGSY--------------LYYYGMDV | WGQGTTVTVSS |
| | | Germline | | H_FR1 | H_CDR1 | H_FR2 | H_CDR2 | H_FR3 | H_CDR3 | H_FR4 |
| | VH3\|3-13/D3\|3-9\|RF1/JH6 | | 32321 & 32322 | EVQLVES-GGGLVQPGGSLRLSCAASG-FTFS | S-----YDMH | WVRQATGKGLEWVS | AIGT----AGDTYYPGSVKG | RFTISRENAKNSLYLQMNSLRAGDTAVYYCAR | VLRYFDWLL*-----------YYYYYGMDV | WGQGTTVTVSS |
| iPS:4 34563 | 21-225_75D8 | VH3\|3-13/D3\|3-9\|RF1/JH6 | | EVQLVES-GGGLVQPGGSLRLSCAASG-FTFS | N-----YDMH | WVRQATGKGLEWVS | AIGT----AGDTYYPGSVKG | RFTISRENAKNSLYLQMNSLRAGDTAVYYCAR | VLDYGDSLG-------------YYYYGMDV | WGQGTTVTVSS |
| iPS:4 35009 | 21-225_89G4 | VH3\|3-13/D3\|3-9\|RF1/JH6 | | EVQLVES-GGGLVQPGGSLRLSCAASG-FTFS | S-----YDMH | WVRQATGKGLEWVS | AIGT----AGDTYYPGSVKG | RFTISRENAKNSLYLQMNSLRAGDTAVYFCAR | ALDYGDSLG-------------YYYYGMDV | WGQGTTVTVSS |
| iPS:4 35059 | 21-225_90C11 | VH3\|3-13/D3\|3-9\|RF1/JH6 | | EVQLVES-GGGLVQPGGSLRLSCAASG-FTFS | N-----YDMH | WVRQATGKGLEWVS | AIGT----AGDTYYPGSVKG | RFTISRENAKNSLYLQMNSLRAGDTAVYYCAR | VLDYGDSLG-------------YYYYGMDV | WGQGTTVTVSS |
| iPS:4 35103 | 21-225_92B2 | VH3\|3-13/D3\|3-9\|RF1/JH6 | | EVQLVES-GGGLVQPGGSLRLSCAASG-FTFS | N-----YDMH | WVRQATGKGLEWVS | AIGT----AGDTYYPGSVKG | RFTISRENAKNSLYLQMNSLRAGDTAVYFCAR | ALDYGDSLG-------------YYYYGMDV | WGQGTTVTVSS |
| iPS:4 37371 | 21-225_74D8 | VH3\|3-13/D3\|3-9\|RF1/JH6 | | EVQLVES-GGGLVQPGGSLRLSCAASG-FTFS | N-----YDMH | WVRQATGKGLEWVS | AIGT----AGDTYYPGSVKG | RFTISRENAKNSLYLQMNSLRAGDTAVYYCAR | VLDYGDSLG-------------YYYYGMDV | WGQGTTVTVSS |
| | | Germline | | H_FR1 | H_CDR1 | H_FR2 | H_CDR2 | H_FR3 | H_CDR3 | H_FR4 |
| | VH1\|1-08/D1\|1- | | 32323 | QVQLVQS- | S-----YDIN | WVRQATGQ | WMNPN--- | RVTMTRNTSISTAYMELSSL | YSGSY------------ | WGQGT |

<table>
<tr><td>iPS:434711</td><td>21-225_80H3</td><td>VH1|1-08/D1|1-26|RF3/JH4</td><td></td><td>QVQLVQS-GAEVKKPGASVKVSCKASG-YTLT</td><td>N-----YDIN</td><td>WVRQATGQGLEWMG</td><td>WMNPN---SGNTGYAQKFQG</td><td>RVTMTRNTSISTAYMELSSLRSEDTAVYYCGS</td><td>TSGWN---------------------FFDY</td><td>WGQGTLVTVSS</td></tr>
<tr><td>iPS:434901</td><td>21-225_85H9</td><td>VH1|1-08/D1|1-26|RF3/JH4</td><td></td><td>QVQLVQS-GAEVKKPGASVKVSCKASG-YTLT</td><td>N-----YDIN</td><td>WVRQATGQGLEWMG</td><td>WMNPN---SGNTGYAQKFQG</td><td>RVTMTRNTSISTAYMELSSLRSEDTAVYYRGS</td><td>TSGWN---------------------FFDY</td><td>WGQGTLVTVSS</td></tr>
<tr><td>iPS:435167</td><td>21-225_92F12</td><td>VH1|1-08/D1|1-26|RF3/JH4</td><td></td><td>QVQLVQS-GAEVKKPGASVKVSCKASG-YTLT</td><td>N-----YDIN</td><td>WVRQATGQGLEWMG</td><td>WMNPN---SGNTGYAQKFQG</td><td>RVTMTRNTSISTAYMELSSLRSEDTAVYYRGS</td><td>TSGGK---------------------FFDY</td><td>WGQGTLVTVSS</td></tr>
<tr><td>iPS:435215</td><td>21-225_94E12</td><td>VH1|1-08/D1|1-26|RF3/JH4</td><td></td><td>QVQLVQS-GAEVKKPGASVKVSCKASG-YTLT</td><td>N-----YDIN</td><td>WVRQATGQGLEWMG</td><td>WMNPN---SGNTGYAQKFQG</td><td>RVTMTRNTSISTAYMELSSLRSEDTAVYYRGS</td><td>TSGWK---------------------FFDY</td><td>WGQGTLVTVSS</td></tr>
<tr><td>iPS:437356</td><td>21-225_74B1</td><td>VH1|1-08/D1|1-26|RF3/JH4</td><td></td><td>QVQLVQS-GAEVKKPGASVKVSCKASG-YTLT</td><td>N-----YDIN</td><td>WVRQATGQGLEWMG</td><td>WMNPN---SGNTGYAQKFQG</td><td>RVTMTRNTSISTAYMELSSLRSEDTAVYYCGS</td><td>TSGWN---------------------FFDY</td><td>WGQGTLVTVSS</td></tr>
<tr><td></td><td></td><td>Germline</td><td></td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH1|1-08/D1|1-1|RF1/JH6</td><td></td><td>32324</td><td>QVQLVQS-GAEVKKPGASVKVSCKASG-YTFT</td><td>S-----YDIN</td><td>WVRQATGQGLEWMG</td><td>WMNPN---SGNTGYAQKFQG</td><td>RVTMTRNTSISTAYMELSSLRSEDTAVYYCAR</td><td>GTTGTYY----------------YYYGMDV</td><td>WGQGTTVTVSS</td></tr>
<tr><td>iPS:434815</td><td>21-225_74A11</td><td>VH1|1-08/D1|1-1|RF1/JH6</td><td></td><td>QVQLVQS-GAEVKKPGASVKVSCKASG-YTFT</td><td>S-----YDIN</td><td>WVRQATGQGLEWMG</td><td>WMNPN---SGNTGYAQKFQG</td><td>RVTMTWNTSKSTAYMELSSLRSEDTAVYYCAR</td><td>GFYDTLTGS-------------GYYYVMDV</td><td>WGQGTTVTVSS</td></tr>
<tr><td>iPS:435253</td><td>21-225_96A4</td><td>VH1|1-08/D1|1-1|RF1/JH6</td><td></td><td>QVQLVQS-GAEVKKPGASVKVSCKASG-YTFT</td><td>S-----YDIN</td><td>WVRQATGHGLEWMG</td><td>WMNPN---SGNTGYAQKFQG</td><td>RVTMTWNTSKSTAYMELSSLRSEDTAVYYCAR</td><td>GFYDTLTGS-------------GYYYVMDV</td><td>WGQGTTVTVSS</td></tr>
<tr><td></td><td></td><td>Germline</td><td></td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH1|1-08/D3|3-22|RF2/JH6</td><td></td><td>32325</td><td>QVQLVQS-GAEVKKPGASVKVSCKASG-YTFT</td><td>S-----YDIN</td><td>WVRQATGQGLEWMG</td><td>WMNPN---SGNTGYAQKFQG</td><td>RVTMTRNTSISTAYMELSSLRSEDTAVYYCAR</td><td>YYYDSSGYYY-----------YYYYYGMDV</td><td>WGQGTTVTVSS</td></tr>
<tr><td>iPS:434977</td><td>21-225_88A5</td><td>VH1|1-08/D3|3-22|RF2/JH6</td><td></td><td>QVQLVQS-GAEVKKPGASVKVSCKASG-YTFT</td><td>S-----YDIN</td><td>WVRQATGQGLEWMG</td><td>WMNPN---SGNTGYAQKFQG</td><td>RVTMTWNTSIRTAYMELSSLRSEDTAVYYCAR</td><td>GFYDFLTGYS----------PTYYYYDMDV</td><td>WGQGTTVTVSS</td></tr>
<tr><td>iPS:435259</td><td>21-225_96C6</td><td>VH1|1-08/D3|3-22|RF2/JH6</td><td></td><td>QVQLVQS-GAEVKKPGASVKVSCKASG-YTFT</td><td>S-----YDIN</td><td>WVRQATGQGLEWMG</td><td>WMNPN---SRNTGYAQKFQG</td><td>RVTMTWNTSISTAYMELSSLRSEDTAVYYCAR</td><td>GGYDVLPGN-------------NYYYDMDV</td><td>WGQGTTVTVSS</td></tr>
<tr><td></td><td></td><td>Germline</td><td></td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH3|3-33/D2|2-</td><td></td><td>32326</td><td>QVQLVES-</td><td>S-----YGMH</td><td>WVRQAPGK</td><td>VIWYD---</td><td>RFTISRDNSKNTLYLQMNSL</td><td>DIVVVVA----------</td><td>WGQGT</td></tr>
</table>

| | | | | | | | | | | |
|---|---|---|---|---|---|---|---|---|---|---|
| iPS:4 35291 | 21-225_146E1 | VH3\|3-33/D2\|2-15\|RF3/JH3 | | QVQLVES-GGGVVQPGRSLRLSCEASG-ITFS | S-----YGMH | WVRQAPGKGLEWVA | IIWYD---GSNKYYADSVKG | RFTISRDNSKNTLFLQMNSLRAEDTAVYYCAR | DRLVGAT-----------------ADAFDI | WGQGTMVTVSS |
| iPS:4 36360 | 21-225_210H11 | VH3\|3-33/D2\|2-15\|RF3/JH3 | | QVQLVES-GGGVVQPGRSLRLSCAASG-FTFS | S-----HGMH | WVRQAPGKGLEWVA | VTWYD---GSDKYYADSVKG | RFTISRDNSKNTLYLQMNSLRAEDTAVYYCAR | DRLVGAT-----------------TDAFDI | WGQGTMVTVSS |
| iPS:4 36370 | 21-225_211A6 | VH3\|3-33/D2\|2-15\|RF3/JH3 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVFYCAR | DRTVGY-------------------DGFDI | WGQGTMVTVSS |
| iPS:4 36392 | 21-225_213B3 | VH3\|3-33/D2\|2-15\|RF3/JH3 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DRTVGY-------------------DGFDI | WGQGTMVTVSS |
| iPS:4 36406 | 21-225_214E4 | VH3\|3-33/D2\|2-15\|RF3/JH3 | | QVQLVES-GGGVVQPGRSLRLSCAASG-FTFS | S-----YGMH | WVRQAPGKGLEWVA | VIWYD---GSNENYADSVKG | RITISRDNSKNTLYLQMNSLRAEDTAVYYCAR | DRTVGY-------------------DGCDI | WGQGAMVTVSS |
| iPS:4 37326 | 21-225_75C10 | VH3\|3-33/D2\|2-15\|RF3/JH3 | | QVQLVES-GGGVVQPGRSLRLSCAASG-FTFS | S-----YGMH | WVRQAPGKGLEWVA | VIWYD---GSDKYYADSVKG | RFTISRDNSKNTLYLQMNSLRAEDTAVYYCAR | DRLVGAT-----------------VDAFDI | WGQGTMVTVSS |
| | | Germline | | H_FR1 | H_CDR1 | H_FR2 | H_CDR2 | H_FR3 | H_CDR3 | H_FR4 |
| | VH4\|4-39/D7\|7-27\|RF1/JH4 | | 3327 | QLQLQES-GPGLVKPSETLSLTCTVSG-GSIS | SS---SYYWG | WIRQPPGKGLEWIG | SIYY----SGSTYYNPSLKS | RVTISVDTSKNQFSLKLSSVTAADTAVYYCAR | LIGY----------------------FDY | WGQGTLVTVSS |
| iPS:4 35293 | 21-225_146F1 | VH4\|4-39/D7\|7-27\|RF1/JH4 | | QLQLQES-GPGLVKPSETLSLTCTVSG-GSIS | RS---SYYWG | WIRQPPGKGLEWIG | SIYY----SGSTSYNPSLKS | RVTISVDTSKNQFSLKLSSVTAADTAVYYCAR | LDLLW---------------------SFDY | WGQGTLVTVSS |
| iPS:4 35361 | 21-225_148E11 | VH4\|4-39/D7\|7-27\|RF1/JH4 | | QLQLQES-GPGLVKPSETLSLTCTVSG-VSIS | RS---SYYWG | WIRQPPGKGLEWIG | SIYY----SGSTSYNPSLKS | RVTISVDTSKNQFSLKLSSVTAADTAVFYCAR | LDPQW---------------------SFDY | WGQGILVTVSS |
| iPS:4 35449 | 21-225_152H9 | VH4\|4-39/D7\|7-27\|RF1/JH4 | | QLQLQES-GPGLVKPSETLSLTCTVSG-GSIS | RS---SYYWG | WIRQPPGKGLEWIG | SIYY----SGSASYNPSLKS | RVTISVDTSKNQFSLKLSSVTAADTAVFYCAR | LDLQW---------------------SFDF | WGQGTLVTVSS |
| iPS:4 35499 | 21-225_156G1 | VH4\|4-39/D7\|7-27\|RF1/JH4 | | QLQLQES-GPGLVKPSETLSLTCTVSG-GSIS | RS---SYYWG | WIRQPPGKGLEWIG | SIYY----SGSASYNPSLKS | RVTISVDTSKNQFSLKLSSVTAADTAVFYCAR | LDLQW---------------------SFDF | WGQGTLVTVSS |
| iPS:4 35587 | 21-225_160H3 | VH4\|4-39/D7\|7- | | QLQLQES-GPGLVKPSETLSL | RS---SYYWG | WIRQPPGKGLEWIG | SIYY----SGSTSYNPS | RVTISVDTSKNQFSLKLSSVTAADTAVFYCAR | LSQRW---------------------DFDY | WGQGTLVTVS |

| | | | | | | | | | | |
|---|---|---|---|---|---|---|---|---|---|---|
| iPS:403868 | 21-225_19D11 | VH4\|4-39/D7\|7-27\|RF1/JH4 | | QLQLQES-GPGLVKPSETLSLTCTVSG-GSIS | RS---SYYWG | WIRQPPGKGLDWIG | SIYY----SGSANYNPSLKS | RVTISVDTSKNQFSLKLSSVTAADAAVYYCAR | LDRGW---------------------SFDY | WGQGTLVTVSS |
| | | Germline | | H_FR1 | H_CDR1 | H_FR2 | H_CDR2 | H_FR3 | H_CDR3 | H_FR4 |
| | VH3\|3-23/D4\|4-17\|RF2/JH5 | | 32328 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | DYGDY-------------------NWFDP | WGQGTLVTVSS |
| iPS:435295 | 21-225_146H1 | VH3\|3-23/D4\|4-17\|RF2/JH5 | | EVQLLES-GGGLVQPGGSLRLSCAASG-FTFS | S-----YAMS | WVRQAPGKGLEWVS | AISGR---GGNTFYADSVKG | RFTISRDNSKNTLYLQMNSLRAEDTAVYYCAK | RVTDYGG-----------------NDWFDP | WGQGTLVTVSS |
| iPS:435307 | 21-225_146E9 | VH3\|3-23/D4\|4-17\|RF2/JH5 | | EVQLLES-GGGLVQPGGSLRLSCAASG-FTFS | S-----YAMS | WVRQAPGKGLEWVS | AISGR---GGNTFYADSVKG | RFTISRDNSKKTLYLQMNSLRAEDTAVYYCAK | RVTDYGG-----------------NDWFDP | WGQGTLVTVSS |
| iPS:435347 | 21-225_148C4 | VH3\|3-23/D4\|4-17\|RF2/JH5 | | EVQLLES-GGGLVQPGGSLRLSCAASG-FTFS | S-----YAMN | WVRQAPGKGLEWVS | AISGS---GGNTFYADSVKG | RFTISRDNSKNTLYLQMNSLRAEDTAVYYCAK | RVTDYGG-----------------NDWFDP | WGQGTLVTVSS |
| iPS:435355 | 21-225_148H9 | VH3\|3-23/D4\|4-17\|RF2/JH5 | | EVQLLES-GGGLVQPGGSLRLSCAASG-FTFS | S-----YAMS | WVRQAPGKGLEWVS | AISGR---GGNTFYADSVKG | RFTISRDNSKNTLYLQMNSLRAEDTAVYYCAK | RVTDYGG-----------------NDWFDP | WGQGTLVTVSS |
| iPS:435371 | 21-225_149A3 | VH3\|3-23/D4\|4-17\|RF2/JH5 | | EVQLLES-GGGLVQPGGSLRLSCAASG-FTFS | N-----YAMT | WVRQAPGKGLEWVS | AISGR---GGNTFYADSVKG | RFTISRDNSKNTLYLQMNSLRAEDTAVYYCTK | RVTDYGG-----------------NDWFDP | WGQGTLVTVSS |
| iPS:435415 | 21-225_150C11 | VH3\|3-23/D4\|4-17\|RF2/JH5 | | EVQLLES-GGGLVQPGGSLRLSCAASG-FTFS | S-----YAMN | WVRQAPGKGLEWVS | AISGS---GGNTFYADSVKG | RFTISRDNSKNTLNLQMSSLRAEDTAVYYCAK | RVTDYGG-----------------NDWFDP | WGQGTLVTVSS |
| iPS:435419 | 21-225_150C12 | VH3\|3-23/D4\|4-17\|RF2/JH5 | | EVQLLES-GGGLVQPGGSLRLSCAASG-FTFS | S-----YAMS | WVRQAPGKGLEWVS | AISGR---GGNTFYADSVKG | RFTISRDNSKNTLYLQMNSLRAEDTAVYYCAK | RVTDYGG-----------------NDWFDP | WGQGTLVTVSS |
| iPS:435425 | 21-225_151B12 | VH3\|3-23/D4\|4-17\|RF2/JH5 | | EVQLLES-GGGLVQPGGSLRLSCAASG-FTFS | S-----YAMN | WVRHAPGKGLEWVS | AISGS---GKNTFYADSVKG | RFTISRDNSKNTLYLQMNSLRAEDTAVYYCAK | RVTDYGG-----------------NDWFDP | WGQGTLVTVSS |
| iPS:435431 | 21-225_152D2 | VH3\|3-23/D4\|4-17\|RF2/JH5 | | EVQLLES-GGGLVQPGGSLRLSCAASG-FTFS | S-----YAMS | WVRQAPGKGLEWVS | AISGR---GGNTFYADSVKG | RFTISRDNSKKTLYLQMNSLRAEDTAVYYCAK | RVTDYGG-----------------NDWFDP | WGQGTLVTVSS |
| iPS:435439 | 21-225_152G4 | VH3\|3-23/D4\|4- | | EVQLLES-GGGLVQPGGSLRL | S-----YAMS | WVRQAPGKGLEWVS | AISGR---GGNTFYADS | RFTISRDNSKNTLYLQMNSLRAEDTAVYYCAK | RVTDYGG-----------------NDWFDP | WGQGTLVTVS |

<table>
<tr><td>iPS:435455</td><td>21-225_152B11</td><td>VH3|3-23/D4|4-17|RF2/JH5</td><td></td><td>EVQLLES-GGGLVQPGGSLRLSCAASG-FTFS</td><td>S-----YAMN</td><td>WVRQAPGKGLEWVS</td><td>AISGR---GGNTFYADSVKG</td><td>RFTISRDNSKNTLYLQMNSLRAEDTAVYYCAK</td><td>RVTDYGG-----------------NDWFDP</td><td>WGQGTLVTVSS</td></tr>
<tr><td>iPS:435487</td><td>21-225_155C4</td><td>VH3|3-23/D4|4-17|RF2/JH5</td><td></td><td>EVQLLES-GGGLVQPGGSLRLSCAASG-FTFS</td><td>S-----YAMS</td><td>WVRQAPGKGLEWVS</td><td>AISGR---GGNTFYADSVKG</td><td>RFTISRDNSKNTLYLQMNSLRAEDTAVYYCAK</td><td>RVTDYGG-----------------NDWFDP</td><td>WGQGTLVTVSS</td></tr>
<tr><td>iPS:435503</td><td>21-225_156E4</td><td>VH3|3-23/D4|4-17|RF2/JH5</td><td></td><td>EVQLLES-GGGLVQPGGSLRLSCAASG-FTFS</td><td>S-----YAMS</td><td>WVRQAPGKGLEWVS</td><td>AISGR---GGNTFYADSVKG</td><td>RFTISRDNSKNTLYLQMNSLRAEDTAVYYCAK</td><td>RVTDYGG-----------------NDWFDP</td><td>WGQGTLVTVSS</td></tr>
<tr><td></td><td></td><td>Germline</td><td></td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH3|3-30/D3|3-22|RF3/JH6</td><td></td><td>32329</td><td>QVQLVES-GGGVVQPGRSLRLSCAASG-FTFS</td><td>S-----YGMH</td><td>WVRQAPGKGLEWVA</td><td>VISYD---GSNKYYADSVKG</td><td>RFTISRDNSKNTLYLQMNSLRAEDTAVYYCAK</td><td>ITMIVVVIT------------YYYYYGMDV</td><td>WGQGTTVTVSS</td></tr>
<tr><td>iPS:435297</td><td>21-225_146B3</td><td>VH3|3-30/D3|3-22|RF3/JH6</td><td></td><td>QVQLVES-GGGVVQPGRSLRLSCAASG-FTFS</td><td>S-----YGMH</td><td>WVRQAPGKGLEWVA</td><td>VIWYD---GSYKYYADSVKG</td><td>RFTISRDNSKNTLDLQMNSLRAEDTAVYYCVK</td><td>MGIEVAVD---------------YYYGMDV</td><td>WGQGTTVTVSS</td></tr>
<tr><td></td><td></td><td>Germline</td><td></td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH4|4-30.1/D1|1-1|RF1/JH3</td><td></td><td>32330</td><td>QVQLQES-GPGLVKPSQTLSLTCTVSG-GSIS</td><td>SG---GYYWS</td><td>WIRQHPGKGLEWIG</td><td>YIYY----SGSTYYNPSLKS</td><td>RVTISVDTSKNQFSLKLSSVTAADTAVYYCAR</td><td>GITGI-------------------DAFDI</td><td>WGQGTMVTVSS</td></tr>
<tr><td>iPS:435301</td><td>21-225_146G4</td><td>VH4|4-30.1/D1|1-1|RF1/JH3</td><td></td><td>QVQLQES-GPGLVKPSQTLSLNCTVSG-GSIS</td><td>NS---GYYWS</td><td>WIRQHPGKGLEWIG</td><td>YSYY----SGSTYYNPSLKS</td><td>RITISVDTSNNQFSLKLTSVTAADTAVYYCAR</td><td>GKYNWN------------------HAFDI</td><td>WGQGTMVTVSS</td></tr>
<tr><td></td><td></td><td>Germline</td><td></td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH3|3-21/D6|6-13|RF1/JH4</td><td></td><td>32331</td><td>EVQLVES-GGGLVKPGGSLRLSCAASG-FTFS</td><td>S-----YSMN</td><td>WVRQAPGKGLEWVS</td><td>SISSS---SSYIYYADSVKG</td><td>RFTISRDNAKNSLYLQMNSLRAEDTAVYYCAR</td><td>GYSSSW------------------YYFDY</td><td>WGQGTLVTVSS</td></tr>
<tr><td>iPS:435313</td><td>21-225_146G11</td><td>VH3|3-21/D6|6-13|RF1/JH4</td><td></td><td>EVQLVES-GGGLVKPGGSLRLSCAASG-FTFS</td><td>S-----YSMN</td><td>WVRQAPGKGLEWVS</td><td>SISGS---GSYTYYADSVKG</td><td>RFTISRDNAKNSLYLQMNSLRAEDTAVYYCAR</td><td>GSSSS---------------------GFDY</td><td>WGQGTLVTVSS</td></tr>
<tr><td>iPS:393808</td><td>21-225_1A2</td><td>VH3|3-21/D6|6-13|RF1/JH4</td><td></td><td>EVQLVES-GGGLVKPGGSLRLSCAASG-FTFS</td><td>S-----YTMN</td><td>WVRQAPGKGLEWVS</td><td>SISGS---GSYIYYADSVKG</td><td>RFTISRDNAKNSLYLQMNSLRAEDTAVYYCAR</td><td>GSSSS---------------------GFDY</td><td>WGQGTLVTVSS</td></tr>
<tr><td>iPS:393958</td><td>21-225_5H2</td><td>VH3|3-21/D6|6-13|RF1/JH4</td><td></td><td>EVQLVES-GGGLVKPGGSLRLSCAASG-FTFS</td><td>S-----YTMN</td><td>WVRQAPGKGLEWVS</td><td>SISGS---SSYIYYADSVKG</td><td>RFTISRANAKNSLYLQMNSLRAEDTAVYYCAR</td><td>GSSSS---------------------GFDY</td><td>WGQGTLVTVSS</td></tr>
<tr><td></td><td></td><td>Germline</td><td></td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH4|4-</td><td></td><td>32332</td><td>QVQLQES-</td><td>SG---DYYWS</td><td>WIRQPPGK</td><td>YIYY----</td><td>RVTISVDTSKNQFSLKLSSV</td><td>GYSYGYYY--------</td><td>WGQGT</td></tr>
</table>

| | | | | | | | | | | |
|---|---|---|---|---|---|---|---|---|---|---|
| iPS:4 35317 | 21-225_147D2 | VH4\|4-30.4/D5\|5-18\|RF3/JH6 | | QVLLQES-GPGLVKPSQTLSLTCAVSG-GPIS | SG---DYYWN | WIRQRPGKGLEWIG | FIYY----TGSTYYNPSLKS | RVSISEDTSENQFSLNLSSVTAADTAVYYCAR | GGAYYS------------------YYGMDV | WGQGTTVTVSS |
| | | Germline | | H_FR1 | H_CDR1 | H_FR2 | H_CDR2 | H_FR3 | H_CDR3 | H_FR4 |
| | VH4\|4-30.1/D5\|5-24\|RF3/JH3 | | 32333 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | RDGYNY-------------------DAFDI | WGQGTMVTVSS |
| iPS:4 35319 | 21-225_147E3 | VH4\|4-30.1/D5\|5-24\|RF3/JH3 | | QVQLQES-GPGLVKPSQTLSLTCTVSG-GSIT | NS---GYYYS | WIRQHPGKGLEWIG | YIYY----SGGTYYNPSLKS | RITISVDTSNNQFSLKLSSVTAADTAVYYCAR | GGYNWN-------------------HAFDF | WGQGTMVTVSS |
| iPS:4 35383 | 21-225_149D7 | VH4\|4-30.1/D5\|5-24\|RF3/JH3 | | QVQLQES-GPGLVKPSQTLSLNCTVSG-GSIS | NS---GYYWS | WIRQHPGKGLEWIG | YSYY----SGSTYYNPSLKS | RITISVDTSNNQFSLKLSSVTAADTAVYYCAR | GGYNWN-------------------HAFDI | WGQGTMVIVSS |
| iPS:4 35443 | 21-225_152E7 | VH4\|4-30.1/D5\|5-24\|RF3/JH3 | | QVQLQES-GPGLVKPSQTLSLNCTVSG-GSIS | NS---GYYWS | WIRQHPGKGLEWIG | YSYY----SGSTYYNPSLKS | RITISVDTSNNQFSLNLSSVTAADTAVYYCAR | GGYNWN-------------------HAFDI | WGQGTMVTVSS |
| iPS:4 35465 | 21-225_153A6 | VH4\|4-30.1/D5\|5-24\|RF3/JH3 | | QVQLQES-GPGLVKPSQTLSLNCTVSG-GSIS | NS---GYYWS | WIRQHPGKGLEWIG | YSYY----SGSTYYNPSLKS | RITISVDTSNNQFSLNLSSVTAADTAVYYCAR | GGYNWN-------------------HAFDI | WGQGTMVTVSS |
| iPS:4 42568 | 21-225_149D8 | VH4\|4-30.1/D5\|5-24\|RF3/JH3 | | QVQLQES-GPGLVKPSQTLSLNCTVSG-GSIS | NS---GYYWS | WIRQHPGKGLEWIG | YSYY----SGSTYYNPSLKS | RITISVDTSNNQFSLKLSSVTAADTAVYYCAR | GGYNWN-------------------HAFDI | WGQGTMVTVSS |
| | | Germline | | H_FR1 | H_CDR1 | H_FR2 | H_CDR2 | H_FR3 | H_CDR3 | H_FR4 |
| | VH3\|3-48/D4\|4-11\|RF2/JH4 | | 32334 | EVQLVES-GGGLVQPGGSLRLSCAASG-FTFS | S-----YSMN | WVRQAPGKGLEWVS | YISSS---SSTIYYADSVKG | RFTISRDNAKNSLYLQMNSLRDEDTAVYYCAR | DYSNY---------------------YFDY | WGQGTLVTVSS |
| iPS:4 35333 | 21-225_147E9 | VH3\|3-48/D4\|4-11\|RF2/JH4 | | EVQLVES-GGGLVQPGGSLRLSCAASG-FTFS | S-----YSMN | WVRQAPGKGLEWVS | SISGR---NTTIYYADSVKG | RFTISRDNAKNSLYLQMNSLRDEDTAVYYCSR | DRG-------------------------SC | WGQGTLVTVSS |
| iPS:4 35637 | 21-225_163E2 | VH3\|3-48/D4\|4-11\|RF2/JH4 | | EVQLVES-GGGLVQPGGSLRLSCAASG-FTFS | S-----YSMN | WVRQAPGKGLEWVS | STSGS---STYIYYADSVKG | RFTISRDNAKNLVYLQMNSLRPEDTAVYYCAR | DRG-------------------------SL | WGQGTLVTVSS |
| | | Germline | | H_FR1 | H_CDR1 | H_FR2 | H_CDR2 | H_FR3 | H_CDR3 | H_FR4 |
| | VH1\|1-02/D2\|2-15\|RF3/JH4 | | 32335 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | DIVVVVA-----------------AIYFDY | WGQGTLVTVSS |
| iPS:4 35351 | 21-225_148B6 | VH1\|1-02/D2\|2- | | QVQLVQS-GAEVKKPGASVKV | G-----YYMH | WVRQAPGQGLEWMG | WIHPN---NGGTNYAQT | RVTMTRDTSISTVYMELSRLRSDDTAVYYCAR | DPVVVP------------------AAPFDY | WGQGTLVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH4\|4-30.1/D5\|5-12\|RF3/JH6 | | 32336 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | GYSGYDYY---------------YYYYGMDV | WGQGTTVTVSS |
| iPS:435363 | 21-225_148F12 | VH4\|4-30.1/D5\|5-12\|RF3/JH6 | | QVQLQES-GPGLVKPSQTLSLTCTVSG-GSIS | NG---GYYWN | WIRQHPGKGLEWIG | YIYY----SGSTYYNPSLKS | QITISVDTSKDQFSLRLSSVTAADTAVYYCAR | YSTYDY------------------YYGMDV | WGQGTTVTVSS |
| iPS:435377 | 21-225_149G5 | VH4\|4-30.1/D5\|5-12\|RF3/JH6 | | QVQLQES-GPGLVKPSQTLSLTCTVSG-GSIS | NG---GYYWN | WIRQHPGKGLEWIG | YIYY----SGSTYYNPSLKS | RVTISVDTSKDQFSLRLSSVTAADTAVYYCAR | YSTYDY------------------YYGMDV | WGQGTTVTVSS |
| | | Germline | | H_FR1 | H_CDR1 | H_FR2 | H_CDR2 | H_FR3 | H_CDR3 | H_FR4 |
| | VH3\|3-48/D6\|6-6\|RF2/JH4 | | 32337 | EVQLVES-GGGLVQPGGSLRLSCAASG-FTFS | S-----YSMN | WVRQAPGKGLEWVS | YISSS---SSTIYYADSVKG | RFTISRDNAKNSLYLQMNSLRDEDTAVYYCAR | SIAAR--------------------YFDY | WGQGTLVTVSS |
| iPS:435409 | 21-225_150G8 | VH3\|3-48/D6\|6-6\|RF2/JH4 | | EVQLVES-GGGLVQPGGSLRLSCAASG-FTFS | T-----YSMT | WVRQAPGKGLDWVS | YISRS---SSTIYYADSVKG | RFSISRDNAKNSLYLQMNSLRDEDTALYYCAR | SAFS---------------------PFDY | WGQGTLVTVSS |
| | | Germline | | H_FR1 | H_CDR1 | H_FR2 | H_CDR2 | H_FR3 | H_CDR3 | H_FR4 |
| | VH3\|3-30.3/D5\|5-18\|RF2/JH5 | | 32338 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | WIQLWL-------------------NWFDP | WGQGTLVTVSS |
| iPS:435427 | 21-225_151C9 | VH3\|3-30.3/D5\|5-18\|RF2/JH5 | | QVQLVES-GGGVVQPGRSLRLSCAASG-FTFS | S-----YGMH | WVRQAPGKGLEWVA | VISYD---GSNKYYADSVKG | RFTISRDNSKNTLYLQMNSLRAEDTAVYYCAR | GVLLWFGE--------------LEDDWFDP | WGQGTLVTVSS |
| | | Germline | | H_FR1 | H_CDR1 | H_FR2 | H_CDR2 | H_FR3 | H_CDR3 | H_FR4 |
| | VH3\|3-33/D3\|3-22\|RF2/JH4 | | 32339 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YYYDSSG-----------------YYYYFDY | WGQGTLVTVSS |
| iPS:435441 | 21-225_152F6 | VH3\|3-33/D3\|3-22\|RF2/JH4 | | QVQLVES-GGGVVQPGRSLRLSCAASG-FTFS | S-----YGMH | WVRQAPGKGLEWVA | IIWYD---GSYKYYADSVKG | RFTISRDNSKNTLYLQMNSLRAEDTAVYYCAR | EGYDFW------------------SGYLGY | WGQGTLVTVSS |
| iPS:435457 | 21-225_152C11 | VH3\|3-33/D3\|3-22\|RF2/JH4 | | QVQLVES-GGGVVQPGRSLRLSCAASG-FTFR | N-----YGMH | WVRQAPGKGLEWVA | IIWYD---GSNKYYADSVKG | RFTISRDNSKNTLYLQMNSLRAEDTAVYYCAR | EAYDFW------------------SGYFDY | WGQGILVTVSS |
| iPS:435463 | 21-225_153D2 | VH3\|3-33/D3\|3-22\|RF2/JH4 | | QVQLVES-GGGVVQPGRSLRLSCAASG-FTFS | S-----YGMH | WVRQAPGKGLEWVA | IIWYD---GSYKYYADSVKG | RFTISRDNSKNTVYLQMNSLRAEDTAVYYCAR | EGYDFW------------------SGYLGY | WGQGTLVTVSS |
| iPS:435531 | 21-225_157G8 | VH3\|3-33/D3\|3- | | QVQLVES-GGGVVQPGRSLRL | N-----YGMH | WVRQAPGKGLEWVA | IIWYD---GSYKYYADS | RFTVSRDNSKNTLYLQMNSLRAEDTAVYYCAR | EGYDFW------------------SGFFDS | WGQGTLVTVS |

| | | | | | | | | | |
|---|---|---|---|---|---|---|---|---|---|
| iPS:435577 | 21-225_160B1 | VH3\|3-33/D3\|3-22\|RF2/JH4 | | QVQLVES-GGGVVQPGRSLRLSCAASG-FTFS | S-----YGMH | WVRQAPGKGLEWVA | VIWYD---GSYKYYADSVKG | RFTISRDNSKNTLYLQMNSLRAEDTAVYYCAR | EAYDFW-----------------SGYYDY | WGQGTLVTVSS |
| iPS:435723 | 21-225_172B7 | VH3\|3-33/D3\|3-22\|RF2/JH4 | | QVQLVES-GGGVVQPGRSLRLSCAASG-FTFS | N-----YGMH | WVRQAPGKGLEWVA | IIWYD---GSNKYYVDSVKG | RFTISRDNSKNTLYLQMNSLRAEDTAVYYCAR | EAYDFW-----------------SGYWDY | WGQGTLVTVSS |
| iPS:435731 | 21-225_173A11 | VH3\|3-33/D3\|3-22\|RF2/JH4 | | QVQLVES-GGGVVQPGRSLRLSCAASG-FTFS | S-----YGMH | WVRQAPGKGLEWVA | IIWYD---GSNKYYADSVKG | RFTISRDNSKNTLYLQMNSLRAEDTAVYYCAR | EAYDFW-----------------SGFFDS | WGQGTLVTVSS |
| iPS:435781 | 21-225_178G10 | VH3\|3-33/D3\|3-22\|RF2/JH4 | | QVQLVES-GGGVVQPGRSLRLSCAASG-FTFS | T-----YGMH | WVRQAPGKGLEWVA | VIWYD---GSNKYYADSVKG | RFTISRDNFKNTLYLQMNSLRAEDTAVYYCAR | ERYDFW-----------------SGHFDY | WGQGTLVTVSS |
| iPS:435899 | 21-225_188G11 | VH3\|3-33/D3\|3-22\|RF2/JH4 | | QVQLVES-GGGVVQPGRSLRLSCAASG-FTFS | S-----YGMH | WVRQAPGKGLEWVT | IIWYD---GSYKYYADSVKG | RFTISRDNSKNTLFLQMNSLRAEDTAVYYCAR | ERYDFW-----------------SGHFDY | WGQGTLVTVSS |
| iPS:436602 | 21-225_226E7 | VH3\|3-33/D3\|3-22\|RF2/JH4 | | QVQLVES-GGGVVQPGRSLRLSCAASG-FTFS | T-----YGMH | WVRQAPGKGLEWVA | IIWYD---GSNKYYADSVKG | RFTISRDNFKNTLYLQMHSLRADDTAVYYCAR | ENYDFW-----------------SGYYGY | WGQGTLVTVSS |
| iPS:392930 | 21-225_25H9 | VH3\|3-33/D3\|3-22\|RF2/JH4 | | QVQLVES-GGGVVQPGRSLRLSCAASG-FPFN | N-----YGMH | WVRQAPGKGLEWVS | IIWYD---GSYKYYADSVKG | RFTISRDNSKNTLYLQMNSLRAEDTAVYYCAR | EGYDFW-----------------SGFFDS | WGQGTLVTVSS |
| | | Germline | | H_FR1 | H_CDR1 | H_FR2 | H_CDR2 | H_FR3 | H_CDR3 | H_FR4 |
| | VH3\|3-23/D1\|1-26\|RF2/JH4 | | 32340 & 32341 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | V*WEL-------------------LYFDY | WGQGTLVTVSS |
| iPS:435479 | 21-225_154E9 | VH3\|3-23/D1\|1-26\|RF2/JH4 | | EVKLLES-GGGLVQPGGSLRLSCAASG-FTFS | S-----YAMS | WVRQAPGKGLEWVS | AISGR---GGNTFYADSVKG | RFTISRDNSKNTLFLQMNSLRAEDTAVYYCAK | RGFRFLE----------------WLGGFDY | WGQGTLVTVSS |
| | | Germline | | H_FR1 | H_CDR1 | H_FR2 | H_CDR2 | H_FR3 | H_CDR3 | H_FR4 |
| | VH3\|3-23/D5\|5-18\|RF3/JH4 | | 32342 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GYSYG-------------------YYFDY | WGQGTLVTVSS |
| iPS:435497 | 21-225_155H9 | VH3\|3-23/D5\|5-18\|RF3/JH4 | | EVQLLES-GGGLVQPGGSLRLSCAASG-FTFS | S-----YAMN | WVRQAPGKGLEWVS | TISGR---GLGTYYADSVKG | RFTISRDNSKNTLYLQLNSLRAEDTAVYYCAK | DHDYGDY----------------NIYFDY | WGQGTLVTVSS |
| | | Germline | | H_FR1 | H_CDR1 | H_FR2 | H_CDR2 | H_FR3 | H_CDR3 | H_FR4 |
| | VH3\|3-23/D6\|6- | | 32343 | EVQLLES- | S-----YAMS | WVRQAPGK | AISGS--- | RFTISRDNSKNTLYLQMNSL | GYSSGW----------- | WGQGT |

| | | | | | | | | | | |
|---|---|---|---|---|---|---|---|---|---|---|
| iPS:435513 | 21-225_157F3 | VH3\|3-23/D6\|6-19\|RF1/JH3 | | EVQLLES-GGGLVQPGGSLRLSCAASE-FTFS | T-----YAMS | WVRQAPGKGLEWVS | VISGS---GGSTYYADSVKG | RFTISRDNSKNTLYLQMNSLRAEDTAVYYCAK | RSSGWY-----------------EDALDI | WGQGTMVTVSS |
| iPS:392766 | 21-225_23H4 | VH3\|3-23/D6\|6-19\|RF1/JH3 | | EVQLLES-GGGLVQPGGSLRLSCAASG-FTFS | S-----YAMN | WVRQAPGKGLEWVS | VISGS---GGTTYFADSVKG | RFTISRDNSKNTLYLQMNSLRAEDTAVYYCAK | RNSSGW-----------------HDVFDI | WGQGTKVTVSS |
| iPS:392808 | 21-225_20F8 | VH3\|3-23/D6\|6-19\|RF1/JH3 | | EVQLLES-GGGLVQPGGSLRLSCAASG-FTFR | S-----YAMS | WIRQAPGRGLEWSS | VISGS---GGSTYYADSVKG | RFTISRDNSKNTLYLQMSSLRAEDTAVYYCAK | RYNSGW-----------------HDVFDI | WGQGTMVTVSS |
| | | Germline | | H_FR1 | H_CDR1 | H_FR2 | H_CDR2 | H_FR3 | H_CDR3 | H_FR4 |
| | VH4\|4-39/D2\|2-21\|RF3/JH4 | | 32344 | QLQLQES-GPGLVKPSETLSLTCTVSG-GSIS | SS---SYYWG | WIRQPPGKGLEWIG | SIYY----SGSTYYNPSLKS | RVTISVDTSKNQFSLKLSSVTAADTAVYYCAR | HIVVVT-----------------AIYFDY | WGQGTLVTVSS |
| iPS:435525 | 21-225_157E7 | VH4\|4-39/D2\|2-21\|RF3/JH4 | | QLHLQES-GPGLVKPSETLSLTCTVSG-GSIS | SG---SYYWG | WIRQPPGKGLEWIG | SIYY----SGSTYYNPSLKS | RVTISVDTSKNQFSLNLSSVTAADTAVYYCAR | HKVAG-------------------PFDY | WGQGTLVTVSS |
| | | Germline | | H_FR1 | H_CDR1 | H_FR2 | H_CDR2 | H_FR3 | H_CDR3 | H_FR4 |
| | VH3\|3-33/D3\|3-10\|RF2/JH6 | | 32345 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YYYGSGSYYN-----------YYYYYGMDV | WGQGTTVTVSS |
| iPS:435543 | 21-225_158D4 | VH3\|3-33/D3\|3-10\|RF2/JH6 | | QVQLVES-GGGVVQPGRSLRLSCAASG-FTFS | D-----YVMH | WVRQAPGKGLEWVS | VIWYD---GSNKYYADSVKG | RFTISRDNSKNTLYLQMNSLRAEDTAVYYCAR | EPYTSGW----------------YDYGMDV | WGQGTTVTVSS |
| iPS:435571 | 21-225_159C8 | VH3\|3-33/D3\|3-10\|RF2/JH6 | | QVQLVES-GGGVVQPGRSLRLSCAASG-FTFS | D-----YVMQ | WVRQAPGKGLEWVA | VIWYD---GSNKYYADSVKG | RFTISRDNSKNTLYLQMNSLRAEDTAVYYCAR | EPYNSGW----------------YDYGMDV | WGQGTTVTVSS |
| iPS:435591 | 21-225_160C4 | VH3\|3-33/D3\|3-10\|RF2/JH6 | | QVQLVES-GGGVVQPGRSLRLSCAASG-FTFS | D-----YVMQ | WVRQAPGKGLEWVA | VIWYD---GSNKYYADSVKG | RFTISRDNSKNTLYLQMNSLRAEDTAVYYCAR | EPYNSGW----------------YDYGMDV | WGQGTTVTVSS |
| iPS:435615 | 21-225_161G12 | VH3\|3-33/D3\|3-10\|RF2/JH6 | | QVQLVES-GGGVVQPGRSLRLSCAASG-FTFS | D-----YVMQ | WVRQAPGKGLEWVA | VIWYD---GSNKYYADSVKG | RFTISRDNSKNTLYLQMNSLRAEDTAVYYCAR | EPYNSGW----------------YDYGMDV | WGQGTTVTVSS |
| iPS:436604 | 21-225_226F7 | VH3\|3-33/D3\|3-10\|RF2/JH6 | | QVQLVES-GGGVVQPGRSLRLSCAASG-FTFS | S-----YGMH | WVRQAPGKGLEWVA | IIWYD---GSNKYYADSVKG | RFTISRDNSKNTLYLQMNSLRAEDTAVYYCAR | ERYNSGW----------------YDYGLDV | WGQGTTVTVSS |
| iPS:451114 | 21-225_159A3 | VH3\|3-33/D3\|3- | | QVQLVES-GGGVVQPGRSLRL | D-----YVMQ | WVRQAPGKGLEWVA | VIWYD---GSNKYYADS | RFTISRDISKNTLYLQMNSLRAEDTAVYYCAR | EPYNSGW----------------YDYGMDV | WGQGTTVTVS |

| | | | | | | | | | | |
|---|---|---|---|---|---|---|---|---|---|---|
| iPS:472732 | 21-225_2B10_LC1 | VH3\|3-33/D3\|3-10\|RF2/JH6 | | QVQLVES-GGGVVQPGRSLRLSCAASG-FTFS | S-----YVMH | WVRQAPGKGLEWVA | VIWYD---GSNKYYADSVKV | RFTVSRDNSKNTLSLQMNSLRAEDTAVYYCAR | ERYTSGW----------------YDYGMDV | WGQGTTVTVSS |
| iPS:472733 | 21-225_2B10_LC2 | VH3\|3-33/D3\|3-10\|RF2/JH6 | | QVQLVES-GGGVVQPGRSLRLSCAASG-FTFS | S-----YVMH | WVRQAPGKGLEWVA | VIWYD---GSNKYYADSVKV | RFTVSRDNSKNTLSLQMNSLRAEDTAVYYCAR | ERYTSGW----------------YDYGMDV | WGQGTTVTVSS |
| iPS:392872 | 21-225_20B11 | VH3\|3-33/D3\|3-10\|RF2/JH6 | | QVQLVES-GGGVVQPGRSLRLSCAASG-FTFS | S-----YVMH | WVRQAPGKGLEWVA | VIWYD---GSNKYYADSVKV | RFTVSRDNSKNTLSLQMNSLRAEDTAVYYCAR | ERYTSGW----------------YDYGMDV | WGQGTTVTVSS |
| iPS:393966 | 21-225_7F8 | VH3\|3-33/D3\|3-10\|RF2/JH6 | | QVQLVES-GGGVVQPGRSLRLSCAASG-FTFS | S-----CVMH | WVRQAPGKGLEWVA | VIWYD---GSNKYYADSVKG | RFTISRDNSNNTLYLQMNSLRAEDTAVYYCAR | ERYTSGW----------------HDYGMDV | WGQGTTVTVSS |
| | | Germline | | H_FR1 | H_CDR1 | H_FR2 | H_CDR2 | H_FR3 | H_CDR3 | H_FR4 |
| | VH3\|3-23/D1\|1-1\|RF1/JH6 | | 32346 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GTIGTYY----------------YYYGMDV | WGQGTTVTVSS |
| iPS:435559 | 21-225_158H12 | VH3\|3-23/D1\|1-1\|RF1/JH6 | | EVQLVES-GGGLVQPGGSLRLSCAASG-FTFS | S-----YVMS | WVRQAPGKGLEWVS | AISGS---GGRTDYADSVKG | RFTISRDNSKNTLYLQMNSLRAEDTAVYYCAK | GGW------------------------NHD | WGQGTTVTVSS |
| | | Germline | | H_FR1 | H_CDR1 | H_FR2 | H_CDR2 | H_FR3 | H_CDR3 | H_FR4 |
| | VH3\|3-21/D1\|1-1\|RF1/JH6 | | 32347 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | GTIGTYY----------------YYYGMDV | WGQGTTVTVSS |
| iPS:435561 | 21-225_159F1 | VH3\|3-21/D1\|1-1\|RF1/JH6 | | EVQLVES-GGGLVKPGGSLRLSCAASG-FTFS | S-----YRMN | WVRQAPGKGLEWVS | SISGS---GNYIDYADSVKG | RFTISRDNAKNSLYLQMNSLRAEDTAVYYCAR | GW--------------------------DV | WGQGTTVTVSS |
| | | Germline | | H_FR1 | H_CDR1 | H_FR2 | H_CDR2 | H_FR3 | H_CDR3 | H_FR4 |
| | VH1\|1-08/D1\|1-1\|RF1/JH4 | | 32348 | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | GTIGT---------------------YFDY | WGQGTLVTVSS |
| iPS:435563 | 21-225_159H2 | VH1\|1-08/D1\|1-1\|RF1/JH4 | | QVQLVQS-GAEVKKPGASVKVSCKASG-YTFT | S-----YDIN | WVRQATGQGLEWMG | WMNPN---SGNTGYVQKFQG | RVTMTRNTSISTAYMELSSLRSEDTAVYYCAR | KKT------------------------GDY | WGQGTLVTVSS |
| iPS:392718 | 21-225_17B8 | VH1\|1-08/D1\|1-1\|RF1/JH4 | | QVQLVQS-GAEVKKPGASVKVSCKASG-YTFT | S-----YAIN | WVRQATGQGLEWMG | WMNPN---TGNTGYAQKFQG | RVTMTRNTSISTAYMELSSLRSEDTAVYFCTR | KAG------------------------FDY | WGQGTLVTVSS |
| iPS:393064 | 21-225_33A9 | VH1\|1-08/D1\|1- | | QVQLVQS-GAEVKKPGASVKV | S-----YDIN | WVRQATGQGLEWMG | WMNPN---SGNTGYAQK | RVTMTRNTSISTAYMELSSLGSEDTAVYYCAR | KKA------------------------NDY | WGQGTLVTVS |

| | | | | | | | | | | |
|---|---|---|---|---|---|---|---|---|---|---|
| iPS:393148 | 21-225_35E5 | VH1\|1-08/D1\|1-1\|RF1/JH4 | | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | KKS-----------------------NDY | WGQGTLVTVSS |
| iPS:398530 | 21-225_32G4 | VH1\|1-08/D1\|1-1\|RF1/JH4 | | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | KKA-----------------------NDY | WGQGTLVTVSS |
| | | Germline | | H_FR1 | H_CDR1 | H_FR2 | H_CDR2 | H_FR3 | H_CDR3 | H_FR4 |
| | VH3\|3-30.3/D1\|1-26\|RF3/JH6 | | 32349 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | YSGSYYY---------------YYYGMDV | WGQGTTVTVSS |
| iPS:435565 | 21-225_159C4 | VH3\|3-30.3/D1\|1-26\|RF3/JH6 | | QVQLVES-GGGVVQPGRSLRLSCAASG-FTFS | N-----YGMH | WVRQAPGKGLEWVA | VISYS---GNNKYYADSVKG | RFTISRDNSKNTLYLQLNSLRAEDMAVYYCAR | RSSSWG-----------------GYGMDV | WGHGTTVTVSS |
| iPS:393892 | 21-225_6G7 | VH3\|3-30.3/D1\|1-26\|RF3/JH6 | | QVQLVES-GGGVVQPGRSLRLSCAASG-FTFS | S-----YGMH | WVRQTPGKGLEWVA | IISYV---GKNKYYADSVKG | RFTISRDNSKNTLYLQMNSLRAEDTAVYYCAR | RGNSYG-----------------GYGMDV | WGQGTTVTVSS |
| | | Germline | | H_FR1 | H_CDR1 | H_FR2 | H_CDR2 | H_FR3 | H_CDR3 | H_FR4 |
| | VH3\|3-23/D2\|2-21\|RF3/JH4 | | 32350 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | HIVVVT-----------------AIYFDY | WGQGTLVTVSS |
| iPS:435579 | 21-225_160G1 | VH3\|3-23/D2\|2-21\|RF3/JH4 | | EVQLLES-GGGLVQPGGSLRLSCAASG-FTFS | S-----YVMS | WVRQAPGKGLEWVS | AMSGS---GGHTYYADSVKG | RFTISRDNSKNTVYLQMNSLRAEDTAVYYCVK | HG--------------------------YS | WGQGTLVTVSS |
| iPS:435585 | 21-225_160G3 | VH3\|3-23/D2\|2-21\|RF3/JH4 | | EVQLLES-GGGLVQPGGSLRLSCAASG-FTFS | S-----YVMS | WVRQTPGKGLEWVS | AMSGS---GGHTYYADSVKG | RFTISRDNSKNTLYLQMNSLRAEDTAIYYCVK | HG--------------------------YS | WGQGTLVTVSS |
| | | Germline | | H_FR1 | H_CDR1 | H_FR2 | H_CDR2 | H_FR3 | H_CDR3 | H_FR4 |
| | VH4\|4-39/D1\|1-1\|RF3/JH5 | | 32351 | QLQLQES-GPGLVKPSETLSLTCTVSG-GSIS | SS---SYYWG | WIRQPPGKGLEWIG | SIYY----SGSTYYNPSLKS | RVTISVDTSKNQFSLKLSSVTAADTAVYYCAR | YNWND-------------------NWFDP | WGQGTLVTVSS |
| iPS:435599 | 21-225_160B10 | VH4\|4-39/D1\|1-1\|RF3/JH5 | | QLQLQES-GPGLVKPSETLSLTCTVSG-GSIS | RS---SYYWG | WIRQYPGKGLEWIG | NIYY----SGSAYHIPSLKS | RVTISVDTSKNQFSLKLNSVTAADTAVYYCVR | HDPNW--------------------GVDY | WGQGTLVTVSS |
| | | Germline | | H_FR1 | H_CDR1 | H_FR2 | H_CDR2 | H_FR3 | H_CDR3 | H_FR4 |
| | VH3\|3-33/D1\|1-1\|RF2/JH6 | | 32352 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | VQLERYY---------------YYYGMDV | WGQGTTVTVSS |
| iPS:435601 | 21-225_160G10 | VH3\|3-33/D1\|1- | | QVQLVES-GGGVVQPGRSLRL | S-----FGMH | WVRQAPGKGLEWVA | VIWYD---GSYKYYADS | RFTISRDNSKNTLYLQMNSLRAEDTAVYYCAR | VGIEVAGD---------------YYFGMEV | WGQGTTVTVS |

| | | | | | | | | | |
|---|---|---|---|---|---|---|---|---|---|
| iPS:4 35655 | 21-225_167E2 | VH3\|3-33/D1\|1-1\|RF2/JH6 | | QVQLVES-GGGVVQPGRSLRLSCAASG-FTFS | S-----FGMH | WVRQAPGKGLEWVA | VIWYD---GTYKYYADSVKG | RFTISRDNSKNTLYLQMNSLRAEDTAVYYCAR | VGIEVAGD---------------YYYGMEV | WGQGTTVTVSS |
| iPS:4 35657 | 21-225_167H10 | VH3\|3-33/D1\|1-1\|RF2/JH6 | | QVQLVES-GGGVVQPGRSQRLSCAASG-FTFS | S-----FGMH | WVRQAPGKGLEWVA | VIWYD---GSYKYHADSVKG | RFTISRDNSKNTLYLQMNSLRAEDTAVYYCAR | VGIEVAGD---------------YYYGMEV | WGQGTTVTVSS |
| | | Germline | | H_FR1 | H_CDR1 | H_FR2 | H_CDR2 | H_FR3 | H_CDR3 | H_FR4 |
| | VH3\|3-53/D7\|7-27\|RF3/JH4 | | 32353 | EVQLVET-GGGLIQPGGSLRLSCAASG-FIVS | S-----NYMS | WVRQAPGKGLEWVS | VIYS----GGSTYYADSVKG | RFTISRDNSKNTLYLQMNSLRAEDTAVYYCAR | NWGY-----------------------FDY | WGQGTLVTVSS |
| iPS:4 35605 | 21-225_161A4 | VH3\|3-53/D7\|7-27\|RF3/JH4 | | EVQLVES-GGGLIQPGGSLRLSCAASG-FTVS | S-----NYMS | WVRQAPGKGLEWVS | VIYT----GGSTYNADSVKG | RFTISRDNSKNTLYLQMNSLRAEDTAVYYCAR | NWGMA--------------------GPFDY | WGQGTLVTVSS |
| | | Germline | | H_FR1 | H_CDR1 | H_FR2 | H_CDR2 | H_FR3 | H_CDR3 | H_FR4 |
| | VH3\|3-30.3/D6\|6-6\|RF1/JH6 | | 32354 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | EYSSSSYY---------------YYYGMDV | WGQGTTVTVSS |
| iPS:4 35607 | 21-225_161G4 | VH3\|3-30.3/D6\|6-6\|RF1/JH6 | | QVQLVES-GGGVVQPGRSLRLSCAASG-FTFS | S-----YGMH | WVRQAPGKGLEWVA | VISYG---GSNKYHADSVKG | RFTISRDNSKNTLYLQMNSLRAEDTAVYYCVR | RSSSSG------------------GYGMDV | WGQGTTVTVSS |
| iPS:3 93020 | 21-225_30E2 | VH3\|3-30.3/D6\|6-6\|RF1/JH6 | | QVQLVES-GGGVVQPGRSLRLSCAASG-FTFS | S-----YGMH | WVRQAPGKGLEWVA | VISYG---GSNKFYAVSVKG | RFNISRDNSKNTLYLQMNSLRAEDTAVYYCVR | RGYSSG------------------GYGMDV | WGQGTTVTVSS |
| iPS:3 93062 | 21-225_33H3 | VH3\|3-30.3/D6\|6-6\|RF1/JH6 | | QVQLVES-GGGVVQPGRSLRLSCAASG-FTFS | S-----YGMH | WVRRAPGKGLEWVA | IISYG---GSNNFYADSVKG | RFTISRDNSKNTLYLQMNSLRAEDTAVYYCAR | RGYSSG------------------GYGMDV | WGQGTTVTVSS |
| iPS:3 93138 | 21-225_35E3 | VH3\|3-30.3/D6\|6-6\|RF1/JH6 | | QVQLVES-GGGVVQPGRSLRLSCAASG-FTFS | S-----YGMH | WVRQAPGKGLEWVA | VISYG---GSNKYYADSVKG | RFTISRDNSKNTLYLQMNSLRAEDTAVYYCVR | RGYSSG------------------GYGMDV | WGQGTTVTVSS |
| | | Germline | | H_FR1 | H_CDR1 | H_FR2 | H_CDR2 | H_FR3 | H_CDR3 | H_FR4 |
| | VH3\|3-30.3/D6\|6-13\|RF2/JH6 | | 32355 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | GIAAAGYY---------------YYYGMDV | WGQGTTVTVSS |
| iPS:4 35611 | 21-225_161F10 | VH3\|3-30.3/D6\|6-13\|RF2/JH6 | | QVQLVES-GGGVVQPGRSLRLSCAASG-FTFS | S-----YGMH | WVRQAPGKGLEWVA | IISYS---GRNDFYADSVKG | RFTISRDNSKNTLYLQMNSLRAEDTAVYYCAR | RIAAAG------------------HYGMDV | WGQGTTVTVSS |
| | | Germline | | H_FR1 | H_CDR1 | H_FR2 | H_CDR2 | H_FR3 | H_CDR3 | H_FR4 |
| | VH3\|3-33/D5\|5- | | 32356 | QVQLVES- | S-----YGMH | WVRQAPGK | VIWYD--- | RFTISRDNSKNTLYLQMNSL | VDIAMVYY-------- | WGQGT |

| | | | | | | | | | | |
|---|---|---|---|---|---|---|---|---|---|---|
| iPS:435629 | 21-225_162H6 | VH3\|3-33/D5\|5-18\|RF1/JH6 | | QVQLVES-GGGVVQPGRSLRLSCAASG-FTFN | N-----YGMH | WVRQAPGKGLEWVA | VIWYD---GSNKYYADSVKG | RFTISRDNSKNTLYLQMNSLRAEDTAVYYCAR | KGIAAVGD---------------YYYGMDV | WGQGTTVTVSS |
| | | Germline | | H_FR1 | H_CDR1 | H_FR2 | H_CDR2 | H_FR3 | H_CDR3 | H_FR4 |
| | VH3\|3-21/D7\|7-27\|RF1/JH6 | | 32357 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | LTGYYY-----------------YYGMDV | WGQGTTVTVSS |
| iPS:435639 | 21-225_163G6 | VH3\|3-21/D7\|7-27\|RF1/JH6 | | EVQLVES-GGGLVKPGGSLRLSCAASG-FTFS | S-----YSMS | WVRQAPGKGLEWVS | SISGS---SAYIYYADSVKG | RFTISRDNAKNSLYLQMNSLRAEDTAVYYCAR | LSG------------------------MDV | WGQGTTVTVSS |
| | | Germline | | H_FR1 | H_CDR1 | H_FR2 | H_CDR2 | H_FR3 | H_CDR3 | H_FR4 |
| | VH3\|3-21/D5\|5-24\|RF2/JH6 | | 32358 & 32359 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | *RWLQLYY---------------YYYGMDV | WGQGTTVTVSS |
| iPS:435643 | 21-225_163G10 | VH3\|3-21/D5\|5-24\|RF2/JH6 | | EVQLVES-GGGLVKPGGSLRLSCAASG-FTFS | S-----YSMN | WVRQAPGKGLEWVS | SISGS---STYIYYADSVKG | RFTISRDNAKNSLYLQMNSLRAEDTAVYYCAR | ARM-------------------------DV | WGQGTTVTVSS |
| | | Germline | | H_FR1 | H_CDR1 | H_FR2 | H_CDR2 | H_FR3 | H_CDR3 | H_FR4 |
| | VH3\|3-33/D3\|3-9\|RF2/JH4 | | 32360 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YYDILTG---------------YYNYFDY | WGQGTLVTVSS |
| iPS:435663 | 21-225_169B1 | VH3\|3-33/D3\|3-9\|RF2/JH4 | | QVQLVES-GGGVVQPGRSLRLSCAASG-FTFS | S-----YGMH | WVRQAPGKGLEWVA | VIWYD---GSNKYYADSVRG | RFTISRDNSKNTLYLQMNSLRAEDTAVYYCAR | DPLRGYN-----------------DPVMDY | WGQGTLVTVSS |
| iPS:435669 | 21-225_169F9 | VH3\|3-33/D3\|3-9\|RF2/JH4 | | QVQLVES-GGGVVQPGRSLRLSCAASG-FTFS | T-----YGMH | WVRQAPGKGLEWVS | IIWYD---GTNKYYADSVKG | RFTISRDNSKNTLYLQLNSLRAEDTAVYYCAR | DPLRGYN-----------------DPVMDY | WGQGTLVTVSS |
| iPS:435693 | 21-225_170G4 | VH3\|3-33/D3\|3-9\|RF2/JH4 | | QVQLVES-GGGVVQPGRSLRLSCTASG-FTFS | T-----YGMH | WVRQAPGKGLEWVS | IIWYD---GTNKYYADSVKG | RFTISRDNSKNTLFLQLNSLRAEDTAMYYCAR | DPLRGYN-----------------DPVMDY | WGQGTLVTVSS |
| iPS:435695 | 21-225_170D5 | VH3\|3-33/D3\|3-9\|RF2/JH4 | | QVQLVES-GGGVVQPGRSLRLSCAASG-FTFS | S-----YGMH | WVRQAPGKGLEWVA | IIWYD---GTNKYYADSVKG | RFTISRDNSKNTLYLQLNSLRAEDTAVYYCAR | DPLRGYN-----------------DPVMDY | WGQGTLVTVSS |
| iPS:435697 | 21-225_170G5 | VH3\|3-33/D3\|3-9\|RF2/JH4 | | QVQLVES-GGGVVQPGGSLRLSCAASG-FTFS | T-----YGMH | WVRQAPGKGLEWVT | IIWYD---GTNKYYADSVKG | RFTISRDNSKSTLYLQMNSLRAEDTAVYFCAR | DPLRGYN-----------------DPVMDY | WGQGTLVTVSS |
| iPS:435703 | 21-225_170D11 | VH3\|3-33/D3\|3- | | QVQLVES-GGGVVQPGRSLRL | S-----YGMH | WVRQAPGKGLEWVA | IIWYD---GTNKYYADS | RFTISRDNSKNTLYLQLNSLRAEDTAVYYCAR | DPLRGYN-----------------DPVMDY | WGQGTLVTVS |

| | | | | | | | | | | |
|---|---|---|---|---|---|---|---|---|---|---|
| iPS:4 35705 | 21-225_171C3 | VH3\|3-33/D3\|3-9\|RF2/JH4 | | QVQLVES-GGGVVQPGGSLRLSCAASG-FTFS | T-----YGMH | WVRQAPGKGLEWVT | IIWYD---GTNKYYADSVKG | RFTISRDNSKNTLYLQMNSLRAEDTAVYYCAR | DPLRGYN-----------------DPVMDY | WGQGTLVTVSS |
| iPS:4 35709 | 21-225_171A4 | VH3\|3-33/D3\|3-9\|RF2/JH4 | | QVQLVES-GGGVVQPGGSLRLSCAASG-FTFS | T-----YGMH | WVRQAPGKGLEWVA | IIWYD---GSNKYYADSVKG | RFTISRDNSKNTLYLQMNSLRAEDTAVYYCAR | DPLRGYN-----------------DPVMDY | WGQGTLVTVSS |
| iPS:4 35721 | 21-225_172B3 | VH3\|3-33/D3\|3-9\|RF2/JH4 | | QVQLVES-GGGVVQPGRPLRLSCAASG-FTFS | T-----YGMH | WVRQAPGKGLEWVA | VIWYD---GSNKYYADSVKG | RFTISRDNSKNTLYLQMNSLRAEDTAVYYCAR | DPLRGYN-----------------DPVMDY | WGQGTLVTVSS |
| iPS:4 35725 | 21-225_172G8 | VH3\|3-33/D3\|3-9\|RF2/JH4 | | QVQMVES-GGGVVQPGRSLRLSCAASG-FTFS | T-----YGMH | WVRQAPGKGLEWMA | IIWYD---GTNKYYADSVKG | RFTISRDNSKNTLYLQLNSLRAEDTAVYYCAR | DPLRGYN-----------------DPVMDY | WGQGTLVTVSS |
| iPS:4 35735 | 21-225_173H12 | VH3\|3-33/D3\|3-9\|RF2/JH4 | | QVQLVES-GGGVVQPGRSLRLSCAASG-FTFS | S-----YGMH | WVRQAPGKGLEWVA | IIWYD---GTNKYYADSVKG | RFTISRDNSKNTLYLQLNSLRAEDTAVYYCAR | DPLRGYN-----------------DPVMDY | WGQGTLVTVSS |
| iPS:4 35743 | 21-225_175G1 | VH3\|3-33/D3\|3-9\|RF2/JH4 | | QVQLVES-GGGVVQPGGSLRLSCAASG-FTFS | T-----YGMH | WVRQAPGKGLEWVA | VIWYD---GSNKYYADSVKG | RFTISRDNSKNTLYVQMNSLRAEDTAVYYCAR | DPLRGYN-----------------DPVMDY | WGQGTLVTVSS |
| iPS:4 35761 | 21-225_176B11 | VH3\|3-33/D3\|3-9\|RF2/JH4 | | QVQLVES-GGGVVQPGRSLRLSCAASG-FTFS | T-----YGMH | WVRQAPGKGLEWVS | IIWYD---GTNKYYADSVKG | RFTISRDNSKNTLFLQLNSLRAEDTAVYYCAR | DPLRGYN-----------------DPVLDY | WGQGTLVTVSS |
| iPS:4 35779 | 21-225_178B10 | VH3\|3-33/D3\|3-9\|RF2/JH4 | | QVQLVES-GGGVVQPGRSLRLSCVASG-FTSS | T-----YGMH | WVRQAPGKGLEWMA | IIWYD---GTNKYYADSVKG | RFTISRDNSKNTLYLQLNSLRAEDTAVYYCAR | DPLRGYN-----------------DPVMDY | WGQGTLVTVSS |
| | | Germline | | H_FR1 | H_CDR1 | H_FR2 | H_CDR2 | H_FR3 | H_CDR3 | H_FR4 |
| | VH3\|3-48/D2\|2-15\|RF3/JH6 | | 32361 | EVQLVES-GGGLVQPGGSLRLSCAASG-FTFS | S-----YSMN | WVRQAPGKGLEWVS | YISSS---SSTIYYADSVKG | RFTISRDNAKNSLYLQMNSLRDEDTAVYYCAR | DIVVVVAAT------------YYYYYGMDV | WGQGTTVTVSS |
| iPS:4 35667 | 21-225_169E3 | VH3\|3-48/D2\|2-15\|RF3/JH6 | | EVQLVES-GGGLVQPGGSLRLSCAASG-FTFS | G-----HSMN | WVRQAPGKGLEWVA | YISLS---GSTIKYADSVKG | RFTISRDNARDSLYLQMNSLRDEDTAVYYCAR | RGITVVR----------------NEDGLDV | WGQGTTVTVSS |
| iPS:4 35673 | 21-225_169E6 | VH3\|3-48/D2\|2-15\|RF3/JH6 | | EVQLVES-GGGLVQPGGSLRLSCAASG-FTFS | G-----HSMN | WVRQAPGKGLEWVA | YISIS---SSTIKYADSVKG | RFTISRDNARDSLYLQMNSLRDEDTAVYYCAR | RGITVVR----------------NEDGLDV | WGQGTTVTVSS |
| iPS:4 35759 | 21-225_176E6 | VH3\|3-48/D2\|2- | | EVQLVES-GGGLVQPGGSLRL | G-----HSMN | WVRQAPGKGLEWVA | YISIS---GSTIKYADS | RFIISRDNARDSLYLQMNSLRDEDTAVYYCAR | RGITVVR----------------NEDGLDV | WGQGTTVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH4\|4-59/D3\|3-9\|RF1/JH6 | | 32362 & 32363 | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAR | VLRYFDWLL*-----------YYYYYGMDV | WGQGTTVTVSS |
| iPS:435675 | 21-225_169D7 | VH4\|4-59/D3\|3-9\|RF1/JH6 | | QVQLQES-GPGLVKPSETLSLTCTVSG-GSIS | S-----YYWT | WIRQPAGKGLEWIG | RIYT----SGSTNYNPSLKS | RVTMSVDTSKNQFSLKLSSVTAADTAVYFCAK | VGRYY-------------------YGMDV | WGQGTTVTVSS |
| iPS:435687 | 21-225_170H1 | VH4\|4-59/D3\|3-9\|RF1/JH6 | | QVQLQES-GPGLVKPSETLSLTCTVSG-GSIS | S-----YYWS | WIRQPAGKGLEWIG | RIYT----SGSTNYNPSLKS | RVTMSVDTSKNQFSLKLSSVTAADTAVYYCAR | VGRYY-------------------YGMDV | WGQGTTVTVSS |
| | | Germline | | H_FR1 | H_CDR1 | H_FR2 | H_CDR2 | H_FR3 | H_CDR3 | H_FR4 |
| | VH3\|3-23/D4\|4-23\|RF2/JH4 | | 32364 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | DYGGN-------------------SYFDY | WGQGTLVTVSS |
| iPS:435711 | 21-225_171G4 | VH3\|3-23/D4\|4-23\|RF2/JH4 | | EVQLLES-GGDLVQPGGSLRLSCAASG-FTFS | S-----CAMT | WVRQAPGKGLEWVS | AISGR---GGTTFYADSVRG | RFTISRDNSKNTLFLQMNSLRAEDTAVYYCAK | DLIGGA-------------------TYFDY | WGQGTLVTVSS |
| iPS:435875 | 21-225_190B9 | VH3\|3-23/D4\|4-23\|RF2/JH4 | | EVQLLES-GGGLVQPGGSLRLSCAASG-FTFS | T-----YAMS | WVRQAPGKGLEWVS | AISRS---GGNTHYADSVKG | RFTISRDNSKNTLYLQMNSLRAEDTAVYSCAK | DGFGGS-------------------SYFDY | WGQGTLVTVSS |
| iPS:435909 | 21-225_190H3 | VH3\|3-23/D4\|4-23\|RF2/JH4 | | EVQLLES-GGGLVQPGGSLRLSCAASG-FTFS | S-----YAMS | WVRQAPGKGLEWVS | AISGR---GGNTYYADSVKG | RFTISRDNSKNTLYLQMNSLRAEDTAVYYCAK | DGFGGS-------------------SYFDY | WGQGTLVTVSS |
| iPS:436013 | 21-225_193F2 | VH3\|3-23/D4\|4-23\|RF2/JH4 | | EVQLLES-GGGLVQPGGSLRLSCAASG-FTFS | T-----FAMS | WVRQAPGKGLEWVS | AISRS---GGNTHYADSVKG | RFTISRDNSKNTLYLQMNSLRAEDTAVYSCAK | DGFGGS-------------------SYFDY | WGQGTLVTVSS |
| iPS:436100 | 21-225_195G12 | VH3\|3-23/D4\|4-23\|RF2/JH4 | | EVQLLES-GGGLVQPGGSLRLSCAASG-FTFS | T-----YAMS | WVRQAPGKGLEWVS | AISRS---GGNTHYADSVKG | RFTISRDNSKNTLYLQMNSLRAEDTAVYSCAK | DGFGGS-------------------SYFDY | WGQGTLVTVSS |
| | | Germline | | H_FR1 | H_CDR1 | H_FR2 | H_CDR2 | H_FR3 | H_CDR3 | H_FR4 |
| | VH3\|3-30.3/D1\|1-7\|RF2/JH6 | | 32365 & 32366 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | V*LELYY-----------------YYYGMDV | WGQGTTVTVSS |
| iPS:435713 | 21-225_171D7 | VH3\|3-30.3/D1\|1-7\|RF2/JH6 | | QVQLVES-RGGVVQPGRSLRLSCAASG-FTFS | S-----YGMH | WVRQAPGKGLEWVA | VISYD---GNNRHYADSVQG | RFTISRDNSKNTLSLQMNSLGAEDTAVYYCAR | DRHRLD------------------YYALDV | WGQGTTVTVSS |
| | | Germline | | H_FR1 | H_CDR1 | H_FR2 | H_CDR2 | H_FR3 | H_CDR3 | H_FR4 |
| | VH3\|3-23/D5\|5- | | 32367 | EVQLLES- | S-----YAMS | WVRQAPGKG | AISGS--- | RFTISRDNSKNTLYLQMNSL | RDGYNY---------- | WGQGT |

| | | | | | | | | | | |
|---|---|---|---|---|---|---|---|---|---|---|
| iPS:4 35729 | 21-225_173E7 | VH3\|3-23/D5\|5-24\|RF3/JH3 | | EVQLLES-GGGSVQPGGSLRLSCAASG-FTFS | S-----YAMS | WVRQAPGKGLEWVS | FISGS---GGNTFYADSVKG | RFTISRDNSKNTLYLQMNSLQAEDTAVYYCTK | RDTYNG-----------------WDAFDI | WGQGTMVTVSS |
| iPS:4 35753 | 21-225_175G10 | VH3\|3-23/D5\|5-24\|RF3/JH3 | | EVQLLES-GGGLVQPGGSLRLSCAASG-FTFS | S-----YAMS | WVRQTPGKGLEWVS | IISGS---GGNTYYADSVKG | RFTISRDNSKNTLYLQMNSLRAEDTAVYYCAK | RDTWNG-----------------WDAFDI | WGQGTMVTVSL |
| iPS:3 93024 | 21-225_31H9 | VH3\|3-23/D5\|5-24\|RF3/JH3 | | EVQLLES-GGGLVQPGGSLRLSCAASG-FTFS | S-----CAMN | WVRQAPGKGLEWVS | AISGS---GGSSFYADSVKG | RFTISRDNSKNTLYLQMNSLRAEDTAVYYCAK | RTPYD---------------------VFDI | WGQGTMVTVSS |
| iPS:3 98474 | 21-225_17B10 | VH3\|3-23/D5\|5-24\|RF3/JH3 | | EVQLLES-GGGLVQPGGSLRLSCAASG-FTFR | S-----YAMS | WVRQAPGKGLEWVS | VISGS---GGNTYFADSVKG | RFTISRDNSKNTLYLQMDSLRAEDTAVYYCAK | RGIPEA------------------DAFDI | WGQGTMVTVSS |
| | | Germline | | H_FR1 | H_CDR1 | H_FR2 | H_CDR2 | H_FR3 | H_CDR3 | H_FR4 |
| | VH1\|1-02/D1\|1-1\|RF1/JH3 | | 32368 | QVQLVQS-GAEVKKPGASVKVSCKASG-YIFT | G-----YYMH | WVRQAPGQGLEWMG | WINPN---SGGTNYAQKFQG | RVTMTRDTSISTAYMELSRLRSDDTAVYYCAR | GTTGT--------------------DAFDI | WGQGTMVTVSS |
| iPS:4 35745 | 21-225_175G3 | VH1\|1-02/D1\|1-1\|RF1/JH3 | | QVQVVQS-GAEVKKPGASVKVSCKASG-YTFT | G-----YFMH | WVRQAPGQGLEWMG | WIKPK---SGGTNCAQRFQG | RVTMTRDTSITTAYMELSRLRSDDTAVYYCVR | GGTTVTT-----------------WGVFDY | WGQGTMVTVSS |
| iPS:4 37270 | 21-225_178H4 | VH1\|1-02/D1\|1-1\|RF1/JH3 | | QVQLVQS-GAEVKKPGASVKVSCKASG-YTFT | G-----YFMH | WVRQAPGQGLEWMG | WIKPK---SGGTNCAQKFQG | RVTMTRDTSISTAYMELSRLRSDDTAVYYCVR | GGTTVTT-----------------WGVFDY | WGQGTMVTVSS |
| | | Germline | | H_FR1 | H_CDR1 | H_FR2 | H_CDR2 | H_FR3 | H_CDR3 | H_FR4 |
| | VH3\|3-23/D3\|3-22\|RF2/JH4 | | 32369 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | YYYDSSG---------------YYYYFDY | WGQGTLVTVSS |
| iPS:4 35769 | 21-225_177B6 | VH3\|3-23/D3\|3-22\|RF2/JH4 | | EVQLLES-GGGLVQPGGSRRLSCEASG-FTFS | S-----YAMS | WVRQAPGKGLEWVS | VISGS---GSNTYYVDSVKG | RFTISRDNSKNTLNLQMNSLRAEDSAVYYCTK | GYYDSSG----------------YYYPFDF | WGQGTLVTVSS |
| | | Germline | | H_FR1 | H_CDR1 | H_FR2 | H_CDR2 | H_FR3 | H_CDR3 | H_FR4 |
| | VH3\|3-33/D3\|3-22\|RF2/JH1 | | 32370 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YYYDSSGY--------------YYAEYFQH | WGQGTLVTVSS |
| iPS:4 35771 | 21-225_177B11 | VH3\|3-33/D3\|3-22\|RF2/JH1 | | QVQLVES-GGGVVQPGRSLRLTCAASG-FTFS | S-----YGMH | WVRQAPGKGLEWVA | IIWYD---GSYKYYTDSVKG | RFTISRDNSKNTLYLQMNSLRAEDTAVYYCAR | ETYDFW------------------SGYFVF | WGQGTLVTVSS |
| | | Germline | | H_FR1 | H_CDR1 | H_FR2 | H_CDR2 | H_FR3 | H_CDR3 | H_FR4 |
| | VH3\|3-23/D5\|5- | | 32371 | EVQLLES- | S-----YAMS | WVRQAPGK | AISGS--- | RFTISRDNSKNTLYLQMNSL | RDGYN----------- | WGQGT |

| | | | | | | | | | | |
|---|---|---|---|---|---|---|---|---|---|---|
| **iPS:4 35775** | **21-225_178A5** | **VH3\|3-23/D5\|5-24\|RF3/JH4** | | EVHLLES-GGGLVQTGGSLRLSCAASG-FTFS | S-----YAMT | WVRQAPGKGLEWVS | VISGS---GGNTFYADSVKG | RFTISRDNSKNTLYLQMNSLRAEDTAVYYCAR | RDGD-----------<br>----------YFDY | WGQGTLVTVSS |
| **iPS:4 37214** | **21-225_48B12** | **VH3\|3-23/D5\|5-24\|RF3/JH4** | | EVQLLES-GGGLVQPGGSLRLSCAASG-FTFS | S-----YAMN | WVRQAPGKGLEWVS | AISGR---GGNTFYADSVKG | RFTISRDNSKNTLYLQMNSLRAEDTAVYYCAK | RETYNWN---------<br>--------YEGFDY | WGQGTLVTVSS |
| **iPS:3 93028** | **21-225_25D7** | **VH3\|3-23/D5\|5-24\|RF3/JH4** | | EVQLLES-GGGLVQPGGSLRLSCAASG-FTFS | S-----YAMS | WVRQTPGQGLEWVS | AISGR---GGTTFYADSVKG | RFTISRDNSKNTLYLQMNSLRAEDTAVYYCAK | DGYGGN----------<br>---------SFFDY | WGQGTLVTVSS |
| | | **Germline** | | **H_FR1** | **H_CDR1** | **H_FR2** | **H_CDR2** | **H_FR3** | **H_CDR3** | **H_FR4** |
| | **VH3\|3-33/D4\|4-11\|RF3/JH6** | | 323/2 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | TTVTYYY---------<br>--------YYGMDV | WGQGTTVTVSS |
| **iPS:4 35789** | **21-225_180C4** | **VH3\|3-33/D4\|4-11\|RF3/JH6** | | QVQLVES-GGGVVQPGRSLRLSCAASG-FTFS | A-----YGMH | WVRQAPGKGLEWVT | IIWYD---GSYKYYADSVKG | RFAISRDNSKNTLYLQMNSLRAEDTAVYYCAR | TGVDPWD---------<br>-------YYNGMDV | WGQGTTVTVSS |
| | | **Germline** | | **H_FR1** | **H_CDR1** | **H_FR2** | **H_CDR2** | **H_FR3** | **H_CDR3** | **H_FR4** |
| | **VH3\|3-23/D2\|2-8\|RF1/JH3** | | 323/3 & 323/4 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | RILY*WCM--------<br>-------LYDAFDI | WGQGTMVTVSS |
| **iPS:4 35799** | **21-225_181G3** | **VH3\|3-23/D2\|2-8\|RF1/JH3** | | EVQLLES-GGGLVQPGGSLRLSCAASG-FTFS | S-----YAMS | WVRQAPGKGLEWVS | VISGS---GGNTFYGDSVKG | RFTISRDNSKNTLYLQMNSLRAEDTAIYYCAK | RETYDWG---------<br>--------SDAFDI | WGQGTMVTVSS |
| | | **Germline** | | **H_FR1** | **H_CDR1** | **H_FR2** | **H_CDR2** | **H_FR3** | **H_CDR3** | **H_FR4** |
| | **VH3\|3-30.3/D5\|5-18\|RF2/JH6** | | 323/5 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | WIQLWLYY--------<br>-------YYYGMDV | WGQGTTVTVSS |
| **iPS:4 35811** | **21-225_183H6** | **VH3\|3-30.3/D5\|5-18\|RF2/JH6** | | QVQLVES-GGGVVQPGRSLRLSCAASG-FTFS | S-----YGMH | WVRQAPGKGLEWVA | IISYA---GSTKFYADSVKG | RFTISRDNSKNTLYLQMNSLRAEDTAVYYCVR | RPPQWLV---------<br>-------EGYGMDV | WGQGTTVTVSS |
| **iPS:4 36754** | **21-225_155G3** | **VH3\|3-30.3/D5\|5-18\|RF2/JH6** | | QVQLVES-GGGVVQPGRSLRLSCTASG-FTFS | S-----YGMH | WVRQAPGKGLEWVA | VISYD---GSNKYYADSVKG | RFTISRDNSKNTLYLQMNSLRAEDTAVYYCAR | DTERWLP---------<br>-------YSYGMDV | WGQGTTVTVSS |
| **iPS:4 48908** | **21-225_50G9** | **VH3\|3-30.3/D5\|5-18\|RF2/JH6** | | QVQLVES-GGGVVQPGRSLRLSCAASG-FIFS | S-----YGMH | WVRQAPGKGLEWVA | VISQD---GIIRYYADSVKG | RFTISRDNSKNTLYLQMNSLRAEDTAVYYCAR | DVKQWLV---------<br>-------RTYGMDV | WGQGTTVTVSS |
| | | **Germline** | | **H_FR1** | **H_CDR1** | **H_FR2** | **H_CDR2** | **H_FR3** | **H_CDR3** | **H_FR4** |
| | **VH3\|3-** | | 323/6 | QVQLVES- | S-----YAMH | WVRQAPGK | VISYD--- | RFTISRDNSKNTLYLQMNSL | GYSSGW---------- | WGQGT |

| | | | | | | | | | | |
|---|---|---|---|---|---|---|---|---|---|---|
| iPS:435813 | 21-225_183A12 | VH3\|3-30.3/D6\|6-19\|RF1/JH5 | | QVQLVES-GGGVVQPGRSLRLSCAASG-FTFS | S-----YGMH | WVRQAPGKGLEWVA | VISSA---GSNKYYADSVKG | RFTISRDNSKNTLYLQMNSLRAEDTAVYYCAR | RYSSGW-------------------DWFDP | WGQGTLVTVSS |
| | | Germline | | H_FR1 | H_CDR1 | H_FR2 | H_CDR2 | H_FR3 | H_CDR3 | H_FR4 |
| | VH3\|3-21/D3\|3-10\|RF3/JH4 | | 32377 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | ITMVRGV------------------IIYFDY | WGQGTLVTVSS |
| iPS:435815 | 21-225_190G10 | VH3\|3-21/D3\|3-10\|RF3/JH4 | | EVQLVES-GGGLVKPGGSLRLSCAASG-FTFR | D-----YSMN | WVRQAPGKGLEWVS | SISSG---SGYIHYADSVKG | RFTISRDNAKNSLYLQMNSLRAEDTAVYYCAR | ATMA-----------------------LDY | WGQGTLVTVSS |
| iPS:435865 | 21-225_191A5 | VH3\|3-21/D3\|3-10\|RF3/JH4 | | EIQVVES-GGGLVKPGGSLRLSCAASG-FTFR | D-----YSMN | WVRQAPGKGLEWVS | SISSG---SGYIYYADSVKG | RFTISRDNAKNSLYLQMNSLRAEDTAVYYCAR | ATMA-----------------------LDY | WGQGALVTVSS |
| iPS:436047 | 21-225_193B10 | VH3\|3-21/D3\|3-10\|RF3/JH4 | | EVQLVES-GGGLVKPGGSLRLSCAASG-FTFR | D-----YSMN | WVRQAPGKGLEWVS | SISSA---GGYIYYADSLKG | RFTISRDNAKNSLYLQMNSLRAEDTAVYYCAR | ATMA-----------------------LDY | WGQGTLVTVSS |
| iPS:436122 | 21-225_196G10 | VH3\|3-21/D3\|3-10\|RF3/JH4 | | EVQLVES-GGGLVKPGGSLRLSCAASG-FTFR | D-----YSMN | WVRQAPGKGLEWVS | SISSG---SGYIHYADSVKG | RFTISRDNAKNSLYLQMNSLRAEDTAVYYCAR | ATMA-----------------------LDY | WGQGTLVTVSS |
| | | Germline | | H_FR1 | H_CDR1 | H_FR2 | H_CDR2 | H_FR3 | H_CDR3 | H_FR4 |
| | VH4\|4-59/D4\|4-17\|RF2/JH6 | | 32378 | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAR | DYGDYYY-----------------YYYGMDV | WGQGTTVTVSS |
| iPS:435817 | 21-225_190B11 | VH4\|4-59/D4\|4-17\|RF2/JH6 | | QVQLQES-GPGLVKPSETLSLTCTVSG-GSIN | N-----YYWS | WIRQPAGKGLEWIG | RIYT----SGSTNYNPSLKS | RVTMSVDTSKNQFSLKLSSVTAADTAVHYCAR | DRGYYG------------------YYGMDV | WGQGTTVTVSS |
| iPS:435917 | 21-225_190D5 | VH4\|4-59/D4\|4-17\|RF2/JH6 | | QVQLQES-GPGLVKPSETLSLTCTVSG-GSIN | N-----YYWS | WIRQPAGKGLEWIG | RIYA----SGSTNYNPSLKS | RVTMSIDTSKNQFSLKLSSVTAADTAVYYCAR | DRGYYG------------------YYGMDV | WGQGTTVTISS |
| iPS:436056 | 21-225_194C3 | VH4\|4-59/D4\|4-17\|RF2/JH6 | | QVQLQES-GPGLVKPSETLSLTCTVSG-GSIN | N-----YYWS | WIRQPAGKGLEWIG | RIYA----SGSTNYNPSLKS | RVTMSIDTSKNQFSLKLSSVTAADTAVHYCAR | DRGYYG------------------YYGMDV | WGQGTTVTISS |
| iPS:436220 | 21-225_200F8 | VH4\|4-59/D4\|4-17\|RF2/JH6 | | QVQLQES-GPGLVKPSETLSLTCTVSG-GSIN | N-----YYWS | WIRQPAGKGLEWIG | RIYT----SGSTNYNPSLKS | RVTMSVDTSKNQFSLKLSSVTAADTAVYYCAR | DRGYYG------------------YYGMDV | WGQGTTVTVSS |
| | | Germline | | H_FR1 | H_CDR1 | H_FR2 | H_CDR2 | H_FR3 | H_CDR3 | H_FR4 |
| | VH3\|3-30/D4\|4- | | 32379 | QVQLVES- | S-----YGMH | WVRQAPGK | VISYD--- | RFTISRDNSKNTLYLQMNSL | DYGDYYY---------- | WGQGT |

| | | | | | | | | | | |
|---|---|---|---|---|---|---|---|---|---|---|
| iPS:435821 | 21-225_190E11 | VH3\|3-30/D4\|4-17\|RF2/JH6 | | QVQLVES-GGGVVQPGRSLRLSCTASG-FTFS | N-----YGMH | WVRQAPGKGLEWVA | IIWFD---GSNKYYADSVKG | RFTISRDNSNNTLYLQMNSLRAEDTAVYYCAK | AQGVYY-------------------YVMDV | WGQGTTVTVSS |
| | | Germline | | H_FR1 | H_CDR1 | H_FR2 | H_CDR2 | H_FR3 | H_CDR3 | H_FR4 |
| | VH3\|3-23/D5\|5-12\|RF3/JH5 | | 32330 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GYSGYD-----------------YNWFDP | WGQGTLVTVSS |
| iPS:435823 | 21-225_190F11 | VH3\|3-23/D5\|5-12\|RF3/JH5 | | EVQLLDS-GGGLGQPGGSLRLSCAASG-FTFD | S-----YAMN | WVRQAPGKGLEWVS | TISGT---GRRTYYADSVKG | RFTISRDNSKNTLYLQMNSLRAEDTAVYYCAK | EEDYYDS----------------SGPGFDP | WGQGTLVTVSS |
| iPS:435867 | 21-225_191E5 | VH3\|3-23/D5\|5-12\|RF3/JH5 | | EVQLLDS-GGGLGQPGGSLRLSCAASG-FTFD | S-----YAMN | WVRQAPGKGLEWVS | TISGT---GRRTYYADSVKG | RFTISRDNSKNTLYLQMNSLRAEDTAVYYCAK | EEDYYDS----------------SGPGFDP | WGQGTLVTVSS |
| iPS:435929 | 21-225_190D9 | VH3\|3-23/D5\|5-12\|RF3/JH5 | | EVQLLDS-GGGLGQPGGSLRLSCAASG-FTFD | S-----YAMS | WVRQAPGKGLEWVS | TISGT---GRRTYYADSVKG | RFTISRDNSKNTLYLQMNSLRAEDTAVYYCAK | EEDYYDS----------------SGPGFDP | WGQGTLVTVSS |
| iPS:435935 | 21-225_190H8 | VH3\|3-23/D5\|5-12\|RF3/JH5 | | GVQLLDS-GGGLGQPGGSLRLSCAASG-FTFD | S-----YAMS | WVRQAPGKGLEWVS | TISGT---GRRTYYADSVKG | RFTISRDNSKNTLYLQMNSLRAEDTAVYYCAK | EEDYYDS----------------SGPGFDP | WGQGTLVTVSS |
| | | Germline | | H_FR1 | H_CDR1 | H_FR2 | H_CDR2 | H_FR3 | H_CDR3 | H_FR4 |
| | VH4\|4-39/D3\|3-9\|RF1/JH4 | | 32381 & 32382 | QLQLQES-GPGLVKPSETLSLTCTVSG-GSIS | SS---SYYWG | WIRQPPGKGLEWIG | SIYY----SGSTYYNPSLKS | RVTISVDTSKNQFSLKLSSVTAADTAVYYCAR | VLRYFDW-----------------LL*YFDY | WGQGTLVTVSS |
| iPS:435827 | 21-225_190H11 | VH4\|4-39/D3\|3-9\|RF1/JH4 | | QLQLQES-GPGLVKPSETLSLTCTVSG-GSIR | S-----YHWS | WIRQPAGKGLEWIG | LIYT----SRSTIYNPSLKS | RVTLSVDTSKNQFSLKLSSVTAADTAVYYCAR | LRYNWN-------------------FPYFDY | WGQGTLVTVSS |
| iPS:435853 | 21-225_191E3 | VH4\|4-39/D3\|3-9\|RF1/JH4 | | QLQLQES-GPGLVKPSETLSLTCTVSG-GSIR | S-----YHWS | WIRQPAGKGLEWIG | LIYT----SRSTNYNPSLKS | RVTMSVDTSKNQFSLKLNSVTAADTAVYYCAR | LRYNWN-------------------FPYFDY | WGQGTLVTVSS |
| iPS:435871 | 21-225_191E6 | VH4\|4-39/D3\|3-9\|RF1/JH4 | | QLQLQES-GPGLVKPSETLSLTCTVSG-GSIR | S-----YHWS | WIRQPAGKGLEWIG | LIYT----SRSTNYNPSLKS | RVTMSVDTSKNQFSLKLSSVTAADTAVYYCAR | LRYNWN-------------------FPYFDF | WGQGTLVTVSS |
| iPS:435927 | 21-225_190E7 | VH4\|4-39/D3\|3-9\|RF1/JH4 | | QLQLQES-GPGLVKPSETLSLTCTVSG-GSIR | S-----YHWS | WIRQPAGKGLEWIG | HIYT----SRSTNYNPSLKS | RVTISVDTSKNQFSLKLSSVTATDTAVYYCAR | LRYNWN-------------------FPYFDY | WGQGTLVTVSS |
| iPS:435999 | 21-225_192F9 | VH4\|4-39/D3\|3- | | QLQLQES-GPGLVKPSETLSL | S-----YHWS | WIRQPAGKGLEWIG | LIYT----SRSTNYNPS | RVTMSVDTSKNQFSLKLNSVTAADTAVYYCAR | LRYNWN-------------------FPYFDY | WGQGTLVTVS |

| | | | | | | | | | | |
|---|---|---|---|---|---|---|---|---|---|---|
| iPS:436060 | 21-225_194F4 | VH4\|4-39/D3\|3-9\|RF1/JH4 | | QLQLQES-GPGLVKPSETLSLTCTVSG-GSIS | S-----YHWS | WIRQPAGKGLEWIG | LIYT----SRSTNYNPSLKS | RVTMSVDRSKSQFSLKLSSVTAADTAVYYCAR | LRYNWN-----------------FPYFDY | WGQGTLVTVSS |
| iPS:436193 | 21-225_198A10 | VH4\|4-39/D3\|3-9\|RF1/JH4 | | QLQLQES-GPGLVKPSETLSLTCTVSG-GSIR | S-----YHWS | WIRQPAGKGLEWIG | HIYT----SRSTNYNPSLKS | RVTISVDTSKNQFSLKLSSVTATDTAVYYCAR | LRYNWN-----------------FPYFDY | WGQGTLVTVSS |
| | | Germline | | **H_FR1** | **H_CDR1** | **H_FR2** | **H_CDR2** | **H_FR3** | **H_CDR3** | **H_FR4** |
| | VH4\|4-30.1/D5\|5-24\|RF3/JH2 | | 32333 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | RDGYNY-----------------YWYFDL | WGRGTLVTVSS |
| iPS:435829 | 21-225_190B12 | VH4\|4-30.1/D5\|5-24\|RF3/JH2 | | QVQLQES-GPGLVKPSQTLSLTCTVSG-GSIS | SG---GYYWN | WIRQHPGKGLEWIG | YIYY----SGSTYYNPSLKS | RVTISVDTSKNQFFLKLNSVTAADTAVYYCAR | SGYNWD------------------AGVDP | WGRGTLVTVSS |
| | | Germline | | **H_FR1** | **H_CDR1** | **H_FR2** | **H_CDR2** | **H_FR3** | **H_CDR3** | **H_FR4** |
| | VH3\|3-33/D4\|4-11\|RF2/JH6 | | 32334 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DYSNYY----------------YYYGMDV | WGQGTTVTVSS |
| iPS:435835 | 21-225_190F12 | VH3\|3-33/D4\|4-11\|RF2/JH6 | | QVQLVES-GGGVVQPGRSLRLSCAASG-FTFR | N-----YGMH | WVRQAPGKGLEWVA | VIWFD---GSNDYYADSVKG | RFTISRDNSKNTLSLQMNSLRAEDTAVYYCAR | DRSVGY------------------DGLDV | WGQGTTVTVSS |
| iPS:435861 | 21-225_190A5 | VH3\|3-33/D4\|4-11\|RF2/JH6 | | QVQLVES-GGGVGQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DFSVGY------------------DGMDV | WGQGTTVTVSS |
| iPS:435937 | 21-225_190H9 | VH3\|3-33/D4\|4-11\|RF2/JH6 | | QVQLVES-GGGVVQPGRSLRLSCAASG-FTFR | N-----YGMH | WVRQAPGKGLEWVA | VIWFD---GSNDYYADSVKG | RFTISRDNSKNTLSLQMNSLRAEDTAVYYCAR | DRSVGY------------------DGLDV | WGQGTTVTVSS |
| iPS:435977 | 21-225_192E4 | VH3\|3-33/D4\|4-11\|RF2/JH6 | | QVQLVES-GGGVVQPGRSLRLSCAASG-FTFS | S-----YGMH | WVRQAPGKGLEWVA | VIWYD---GSNKNYVDSVRG | RFTISRDNSKNTLYLQMNSLRAEDTAVYYCAR | DRSVGY------------------DGMDV | WGQGTTVTVSS |
| iPS:436001 | 21-225_192C10 | VH3\|3-33/D4\|4-11\|RF2/JH6 | | QVQLVES-GGGVVQPGRSLRLSCASSG-FTFR | N-----YGMH | WVRQAPGKGLKWVA | VIWFD---GSNDYYADSVKG | RFTISRDNSKNTLSLQMNSLRAEDTAVYYCAR | DRSVGY------------------DGLDV | WGQGTTVTVSS |
| iPS:436066 | 21-225_194B7 | VH3\|3-33/D4\|4-11\|RF2/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DRSKGY------------------DGMDV | WGQGTTVTVSS |
| iPS:436078 | 21-225_194H12 | VH3\|3-33/D4\|4- | | QVQLVES-GGGVVQPGRSLRL | N-----YGMH | WVRQAPGKGLEWVA | VIWFD---GSNDYYADS | RFTISRDNSKNTLSLQMNSLRAEDTAVYYCAR | DRSVGY------------------DGLDV | WGQGTTVTVS |

| | | | | | | | | | | |
|---|---|---|---|---|---|---|---|---|---|---|
| **iPS:4 36140** | **21-225_197G3** | **VH3\|3-33/D4\|4-11\|RF2/JH6** | | QVQLVES-GGGVVQPGRSLRLSCVASG-FTFS | S-----HGMH | WVRQAPGKGLEWVA | VIWYD---GSNKNYADSVKG | RFTISRDNSKNTLYLQMNSLRAEDTAVYYCAR | DPSVGY-------------------DGMDV | WGQGTTVTVSS |
| **iPS:4 36167** | **21-225_197E11** | **VH3\|3-33/D4\|4-11\|RF2/JH6** | | QVQLVES-GGGVVQPGRSLRLSCAASG-FTFR | N-----YGMH | WVRQAPGKGLEWVA | VIWFD---GSNDYYADSVKG | RFTISRDNSKNTLSLQMNSLRAEDTAVYYCAR | DRSVGY-------------------DGLDV | WGQGTTVTVSS |
| **iPS:4 36292** | **21-225_205H3** | **VH3\|3-33/D4\|4-11\|RF2/JH6** | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DRSVGY-------------------DGTDV | WGQGTTVTVSS |
| **iPS:4 36802** | **21-225_171E12** | **VH3\|3-33/D4\|4-11\|RF2/JH6** | | QVQLVES-GGGVVQPGRSLRLSCAASG-FTFS | S-----YGMH | WVRQAPGKGLEWVA | VIWND---GGNKYNGDSVKG | RFTISRDNSKNTLYLQMNSLRAEDTAVYYCAR | DRTYYSGSGSP---------PYYYYYGMDV | WGQGTTVTVSS |
| **iPS:4 36816** | **21-225_179H5** | **VH3\|3-33/D4\|4-11\|RF2/JH6** | | QVQLVES-GGGVVQPGRSLRLSCAASG-FTFS | S-----YGMH | WVRQAPGKGLEWVA | VIWYD---GSNEYYADSVKG | RFTISRDNSKNTLYLQMNSLRAEDTAVYYCAR | DIRNYY-------------------YGLDV | WGQGTTVTVSS |
| **iPS:4 36960** | **21-225_198D2** | **VH3\|3-33/D4\|4-11\|RF2/JH6** | | QVQLVES-GGGVVQPGRSLRLSCAASG-FTFR | S-----YGMH | WVRQAPGKGLEWVA | VIIYD---GSYKYYADSVKG | RFTISRDNSKNTLYLQMNSLRAEDTAVYYCAR | TYSG----------------------GMDV | WGQGTTVTVSS |
| **iPS:4 36974** | **21-225_190H7** | **VH3\|3-33/D4\|4-11\|RF2/JH6** | | QVQLVES-GGGVVQPGRSLRLSCAASG-FNFR | S-----YGMH | WVRQAPGKGLEWVA | VIIYD---GSYKYYADSVKG | RFTISRDNSKNTLYLQMNSLRAEDTAVYYCAR | TYSG----------------------GMDV | WGQGTTVTVSS |
| **iPS:4 36982** | **21-225_190D10** | **VH3\|3-33/D4\|4-11\|RF2/JH6** | | QVQLVES-GGGVVQPGRSLRLSCAASG-FTFR | S-----YGMH | WVRQAPGKGLEWVA | VIIYD---GSYKYYADSVKG | RFTISRDNSKNTLYLQMNSLRAEDTAVYYCAR | TYSG----------------------GMDV | WGQGTTVTVSS |
| **iPS:4 37274** | **21-225_196D4** | **VH3\|3-33/D4\|4-11\|RF2/JH6** | | QVQVVES-GGGVVQPGRSLRLSCAASG-FTFS | S-----YGMH | WVRQAPGKGLEWVA | VIWYD---GSNRNYADSVKG | RFTISRDNSKNTLYLQMNSLRVEDTAVYYCAR | DRSKGY-------------------DGMDV | WGQGTTVTVSS |
| **iPS:3 92664** | **21-225_20F6** | **VH3\|3-33/D4\|4-11\|RF2/JH6** | | QVQLVES-GGGVVQPGRSLRLSCVASG-FTFS | S-----YGMH | WVRQAPGKGLEWGA | VIWHD---GSNKYYADSVKG | RFTISRDNAKNTLYLQMNSLRAEDTAVYYCAR | DLSMG---------------------GMDV | WGQGTTVTVSS |
| **iPS:3 92738** | **21-225_18G4** | **VH3\|3-33/D4\|4-11\|RF2/JH6** | | QVQLVES-GGGVVQPGRSLRLSCAASG-FTFS | S-----YGMH | WVRQAPGKGLEWVA | IIWYD---GSNKYYADSVKG | RFTISRDNSKNTLYLQMNSLRAEDTAVYYCAR | DLSMG---------------------GMDV | WGQGTTVTVSS |
| **iPS:3 92798** | **21-225_22C7** | **VH3\|3-33/D4\|4-** | | QVQLVES-GGGVVQPGRSLRL | S-----YGMH | WVRQAPGKGLEWGA | VIWHD---GSNKYYADS | RFTISRDNAKNTLYLQMNSLRAEDTAVYYCAR | DLSMG---------------------GMDV | WGQGTTVTVS |

| | | | | | | | | | | |
|---|---|---|---|---|---|---|---|---|---|---|
| iPS:392956 | 21-225_27A11 | VH3\|3-33/D4\|4-11\|RF2/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYHCAR | DSSPY---------------------GMDV | WGQGTTVTVSS |
| iPS:392994 | 21-225_26G11 | VH3\|3-33/D4\|4-11\|RF2/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RYSNSW------------------SGGMDV | WGQGTTVTVSS |
| iPS:393014 | 21-225_26D12 | VH3\|3-33/D4\|4-11\|RF2/JH6 | | QVQLVES-GGGVVQPGRSLRLSCAASG-FTFS | S-----YGMH | WVRQAPGKGLEWVA | VIWYD---GSNEYYADSVKG | RFTISRDNSKNTLYLQMNSLRAADTAVYYCAR | DSSPY---------------------GMDV | WGQGTTVTVSS |
| iPS:393152 | 21-225_25B3 | VH3\|3-33/D4\|4-11\|RF2/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DSSPY---------------------GMDV | WGQGTTVTVSS |
| iPS:393840 | 21-225_3F8 | VH3\|3-33/D4\|4-11\|RF2/JH6 | | QVQLVES-GGGVVQPGRSLRLSCAASG-FTFS | S-----YGMH | WVRQAPGKGLEWGA | VIWHD---GSNKYYADSVKG | RFTISRDNAKNTLYLQMNSLRAEDTAVYYCAR | DLSMG---------------------GMDV | WGQGTTVTVSS |
| iPS:393930 | 21-225_7E11 | VH3\|3-33/D4\|4-11\|RF2/JH6 | | QVQLVES-GGGVVQPGRSLRLSCAASG-FTFS | S-----FGMH | WVRQAPGKGLEWVA | IIWHD---GSNKYYADSVKG | RFTISRDNSNNTLYLQMSSLRAEDTAVYYCAR | DLSMG---------------------GMDV | WGQGTTVTVSS |
| iPS:393964 | 21-225_6G1 | VH3\|3-33/D4\|4-11\|RF2/JH6 | | QVQLVES-GGGVVQPGRSLRLSCAASG-FIFS | S-----YGMH | WVRQAPGKGLEWVA | IIWYD---GSNKYYADSVKG | RFTISRDNSKNTLYLQMNSLRAEDTAMYYCAR | DLSMG---------------------GMDV | WGQGTTVTVSS |
| iPS:394012 | 21-225_15A3 | VH3\|3-33/D4\|4-11\|RF2/JH6 | | QVQLVES-GGGVVQPGRSLRLSCAASG-FTFS | S-----YGIH | WVRQAPGKGLEWVA | VIWHD---GSNKYYADSVKG | RFTISRDNSKNTLYLQMNSLRAEDTAVYYCAR | DLSMG---------------------GMDV | WGQGTTVTVSS |
| iPS:394016 | 21-225_13D4 | VH3\|3-33/D4\|4-11\|RF2/JH6 | | QVQLVES-GGGVVQPGRSLRLSCAASG-FTFS | S-----YGMH | WVRQAPGKGLEWVA | VIWHD---GSNKYYADSVKG | RFTISRDNSKNTLYLQMNSLRAEDTAVYYCAR | DLSMG---------------------GMDV | WGQGTTVTVSS |
| iPS:394083 | 21-225_16E6 | VH3\|3-33/D4\|4-11\|RF2/JH6 | | QVQLVES-GGGVVQPGRSLRLSCAASG-FTFS | S-----YGMH | WVRQAPGKGLEWVA | VIWHD---GSNKYYVDSVKG | RFTISRDNSKNTLNLQMNSLRAEDTAVYYCAR | DLSMG---------------------GMDV | WGQGTTVTVSS |
| | | Germline | | H_FR1 | H_CDR1 | H_FR2 | H_CDR2 | H_FR3 | H_CDR3 | H_FR4 |
| | VH4\|4-59/D3\|3-9\|RF1/JH4 | | 32385 & 32386 | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAR | VLRYFDW-----------LL*YFDY | WGQGTLVTVSS |
| iPS:435839 | 21-225_191B1 | VH4\|4-59/D3\|3- | | QVQLQES-GPGLVKPSETLSL | S-----YHWS | WIRQPAGKGLEWIG | HIYT----SGSTKYNPS | RVTMSVDTSKNQFSLKLSSVTAADTAVYYCAR | LRYNWN------------------FPFFDY | WGQGTLVTVS |

| | | | | | | | | | | |
|---|---|---|---|---|---|---|---|---|---|---|
| iPS:4 36158 | 21-225_197G8 | VH4\|4-59/D3\|3-9\|RF1/JH4 | | QVHLQES-GPGLVKPSETLSLTCTVSG-GSIS | A-----YSWS | WIRQPAGKGLEWIG | RLSP----GGSTNFNPSLKS | RVTMSVDTSKNQFSLRLSSVTAADTAVYYCAR | LRYNWN-----------------FPYFDY | WGQGALVTVSS |
| | | Germline | | H_FR1 | H_CDR1 | H_FR2 | H_CDR2 | H_FR3 | H_CDR3 | H_FR4 |
| | VH4\|4-30.1/D3\|3-22\|RF2/JH6 | | 32337 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | YYYDSSGYYY-----------YYYYGMDV | WGQGTTVTVSS |
| iPS:4 35843 | 21-225_191F1 | VH4\|4-30.1/D3\|3-22\|RF2/JH6 | | QVQLQES-GPGLVKPSQTLSLTCTVSG-GSIN | SG---GYYWN | WIRQHPGKGLEWIG | YIFY----SGSTYYNPSLKS | RVTISVDTSKNQFSLKLSSVTAADTAVYYCAR | GDYDGSGSY-------------HYYYGMDV | WGQGTTVTVSS |
| iPS:4 35847 | 21-225_191A3 | VH4\|4-30.1/D3\|3-22\|RF2/JH6 | | QVQLQES-GPGLVKPSQTLSLTCTVSG-GSIS | SG---DYYWN | WIRQHPGKGLEWIG | YIFY----SGSTYYNPSLKS | RVTISVDTSKNQFSLKLSSVTAADTAVYYCAR | GDYDGSGSY-------------HYYYGMDV | WGQGTTVTVSS |
| iPS:4 35851 | 21-225_191D3 | VH4\|4-30.1/D3\|3-22\|RF2/JH6 | | QVQLKES-GPGLVKPSQTLSLTCTVSG-GSIN | SG---DYYWN | WIRQHPGKGLDWIG | YIFY----SGSTYYNPSLKS | RVTISVDTSKNQFSLKLSSVTAADTAVYYCAR | GDYDGSGSY-------------HYYYGMDV | WGQGTTVTVSS |
| iPS:4 35905 | 21-225_190A3 | VH4\|4-30.1/D3\|3-22\|RF2/JH6 | | QVQLQES-GPGLVKPSQTLSLTCTVSG-GSIN | SG---GYYWN | WIRQHPGKGLEWIG | FIFY----SGSTYYNPSLKS | RVTISVDTSKNQFSLKLNSVTAADTAVYYCAR | GDYDGSGSY-------------HYYYGMDV | WGQGTTVTVSS |
| iPS:4 35911 | 21-225_190B4 | VH4\|4-30.1/D3\|3-22\|RF2/JH6 | | QVQLQES-GPGLVKPSQTLSLTCTVSG-GSIS | SG---DYYWN | WIRQHPGKGLEWIG | YIFY----SGSTYYNPSLKS | RVTISVDTSKNQFSLKLSSVTAADTAVYYCAR | GDYDGSGSY-------------HYYYGMDV | WGQGTTVTVSS |
| iPS:4 35913 | 21-225_190A7 | VH4\|4-30.1/D3\|3-22\|RF2/JH6 | | QVQLQES-GPGLVNPSQTLSLTCTVSG-GSIS | SG---VYYWS | WIRQHPGKGLEWIG | NIYY----SGSTYNNPSLKS | RIIISVDTSKNQFSLKLSSVTAADTAVYYCAR | GDYDGSGSY-------------HYYYGLDV | WGQGTTVTVSS |
| iPS:4 35939 | 21-225_191H7 | VH4\|4-30.1/D3\|3-22\|RF2/JH6 | | QVQLQES-GPGLVKPSQTLSLTCTVSG-GSIN | SG---DYYWN | WIRQHPGKGLEWIG | YIFY----SGSTYYNPSLKS | RVTISVDTSKNQFSLKLSSVTAADTAVYYCAR | GDYDGSGSY-------------HYYYGMDV | WGQGTTVTVSS |
| iPS:4 35967 | 21-225_192B3 | VH4\|4-30.1/D3\|3-22\|RF2/JH6 | | QVQLQES-GPGLVKPSQTLSLTCTVSG-GSIS | SG---DYYWN | WIRQHPGKGLEWIG | FIFY----SGSTYYNPSLKS | RVTISVDTSKNQFSLKLSSVTVADTAVYYCAR | GDYDGSGSY-------------HHYYGMDV | WGQGTTVTVSS |
| iPS:4 35973 | 21-225_192H3 | VH4\|4-30.1/D3\|3-22\|RF2/JH6 | | QVQLQES-GPGLVKPSQTLSLTCTVSG-GSIS | SV---SYYWS | WIRQRPGKGLEWIG | NLYY----SGSTYYNPSLRS | RATISVDTSKNQFSLKLSSVTAADTAVYYCTR | GDYDGSGSY-------------HYYHGMDV | WGQGTTVTVSS |
| iPS:4 36007 | 21-225_192G12 | VH4\|4-30.1/D3\|3- | | QVQLQES-GPGLVKPSQTLSL | SG---VYHWS | WIRQHPGKGLEWIG | NIHY----SGSTYNNPS | RVTISVDTSKNQFSLKLSSVTAADTAVYYCAR | GDYDGSGSY-------------HYYYGMDV | WGQGTTVTVS |

| | | | | | | | | | | |
|---|---|---|---|---|---|---|---|---|---|---|
| iPS:4 36009 | 21-225_193A1 | VH4\|4-30.1/D3\|3-22\|RF2/J H6 | | QVQLQES-GPGLVKPSQTLSLTCTVSG-GSIS | SG---VYYWS | WIRQHPGKGLEWIG | NIYY----SGSTYNNPSLKS | RLTISADTSKNQFSLKLSSVTAADTAVYYCAR | GDYDGSGSY-------------HYYYGMDV | WGQGTTVTVSS |
| iPS:4 36011 | 21-225_193B1 | VH4\|4-30.1/D3\|3-22\|RF2/J H6 | | QVQLQES-GPGLVKPSQTLSLTCTVSG-GSIS | SG---VYYWS | WIRQHPGKGLEWIG | NIYY----SGSTYNNPSLKS | RVTISVDTSKNQFSLKLSSVTAADTAVYYCAR | GDYDGSGSY-------------HYYYGMDV | WGQGTTVTVSS |
| iPS:4 36017 | 21-225_193F3 | VH4\|4-30.1/D3\|3-22\|RF2/J H6 | | QVQLQES-GPGLVKPSQTLSLTCTVSG-GSIN | SG---DYYWN | WIRQHPGKGLEWIG | YIFY----SGSTYYNPSLKS | RVTISVDTSKNQFSLKLSSVTAADTAVYYCAR | GDYDGSGSY-------------HYYYGMDV | WGQGTTVTVSS |
| iPS:4 36029 | 21-225_193H6 | VH4\|4-30.1/D3\|3-22\|RF2/J H6 | | QVQLQES-GPGLVKPSQTLSLTCTVSG-GSIN | SG---DYYWN | WIRQHPGKGLEWIG | YIFY----SGSTYYNPSLKS | RVTISVDTSKNQFSLKLSSVTAADTAVYYCAR | GDYDGSGSY-------------HYYYGMDV | WGQGTTVTVSS |
| iPS:4 36035 | 21-225_193C8 | VH4\|4-30.1/D3\|3-22\|RF2/J H6 | | QVQLQES-GPGLVKPSQTLSLTCTVSG-GSIS | SG---DYYWN | WIRQHPGKGLEWIG | YIFY----SGSTYYNPSLKS | RVTISVDTSKNQFSLKLSSVTAADTAVYYCAR | GDYDGSGSY-------------HYYYGMDV | WGQGTTVTVSS |
| iPS:4 36037 | 21-225_193D8 | VH4\|4-30.1/D3\|3-22\|RF2/J H6 | | QVQLQES-GPGLVKPSQTLSLTCTVSG-GSIN | SG---GYYWN | WIRQHPGKGLEWIG | FIFY----SGSTYYNPSLKS | RVSISVDTSKNQFSLKLSSVTAADTAVYYCAR | GDYDGSGSY-------------HYYYGMDV | WGQGTTVTVSS |
| iPS:4 36041 | 21-225_193G8 | VH4\|4-30.1/D3\|3-22\|RF2/J H6 | | QVQLQEK-GPGLVKPSQTLSLTCTVSG-GSVS | SG---VYYWS | WIRQHPGKGLEWIG | NIYY----SGSTYNNPSLKS | RVTISVDTSKNQFSLKLNSVTAADTAVYYCAR | GDYDGSGSY-------------HFYYGLDV | WGHGTTVTVSS |
| iPS:4 36062 | 21-225_194E5 | VH4\|4-30.1/D3\|3-22\|RF2/J H6 | | QVQLQES-GPGLVKPSQTLSLTCTVSG-GSIN | SG---DYYWN | WIRHHPGKGLEWIG | YIFY----SGSTYYNPSLKS | RVTISVDTSKNQFSLKLSSVTAADTAVYYCAR | GDYDGSGSY-------------HYYYGMDV | WGQGTTVTVSS |
| iPS:4 36064 | 21-225_194E6 | VH4\|4-30.1/D3\|3-22\|RF2/J H6 | | QVQLQES-GPGLVKPSQTLSLTCTVSG-DSIN | SG---DYYWN | WIRQHPGKGLEWIG | FIFY----SGSTYYNPSLKS | RVTISIDTSKNQFSLKLSSVNVADTAVYYCAR | GDYDGSGSY-------------HYYYGMDV | WGQGTTVTVSS |
| iPS:4 36134 | 21-225_196H12 | VH4\|4-30.1/D3\|3-22\|RF2/J H6 | | QVQLQES-GPGLVNPSQTLSLTCTVSG-GSIS | SG---VYYWS | WIRQHPGKGLEWIG | NIYY----SGSTYNNPSLKS | RIIISVDTSKNQFSLKLSSVTAADTAVYYCAR | GDYDGSGSY-------------HYYYGLDV | WGQGTTVTVSS |
| iPS:4 36146 | 21-225_197F4 | VH4\|4-30.1/D3\|3-22\|RF2/J H6 | | QVQLQES-GPGLVKPSQTLSLTCTVSG-GSIS | SG---DYYWN | WIRQHPGKGLEWIG | YIFY----SGSTYYNPSLKS | RITISVDTSKNQFSLKLSSVTAADTAVYYCAR | GDYDGSGSY-------------HYYYGLDV | WGQGTTVTVSS |
| iPS:4 36177 | 21-225_198B1 | VH4\|4-30.1/D3\|3- | | QVQLKES-GPGLVKPSQTLSL | SG---DYYWN | WFRQHPGKGLEWIG | YIFH----SGSTYYNPS | RVTVSVDTSKNQFSLKLSSVTAADTAVYYCAR | GDYDGSGSY-------------HYYYGMDV | WGRGTTVTVS |

| | | | | | | | | | | |
|---|---|---|---|---|---|---|---|---|---|---|
| iPS:4 36179 | 21-225_198E1 | VH4\|4-30.1/D3\|3-22\|RF2/JH6 | | QVQLQES-GPGLVKPSQTLSLTCTVSG-GSIN | SG---GYYWN | WIRQHPGKGLEWIG | FIFY----SGSTYYNPSLKS | RLSISVDTSKNQFSLKLSSVTAADTAVYYCAR | GDYDGSGSY-------------HYYYGMDV | WGQGTTVTVSS |
| iPS:4 36197 | 21-225_199C2 | VH4\|4-30.1/D3\|3-22\|RF2/JH6 | | QVQLQES-GPGLVKPSQTLSLTCTVSG-GSIS | SG---DYYWN | WLRQHPEKGLEWIG | YIFY----SGSTYYNPSLKS | RVTISVDTSMTQFSLKLTSVTAADTAVYYCAR | GDYDGSGSY-------------HYYYGMDV | WGQGTTVTVSS |
| iPS:4 36207 | 21-225_199C7 | VH4\|4-30.1/D3\|3-22\|RF2/JH6 | | QVQLQES-GPGLVKPSQTLSLTCTVSG-GSIN | SG---GYYWN | WIRQHPGKGLEWIG | FIFY----SGSTYYNPSLKS | RVSISVDTSKNQFSLKLSSVTAADTAVYYCAR | GDYDGSGSY-------------HYYYGMDV | WGQGTTVTVSS |
| iPS:4 36226 | 21-225_200F10 | VH4\|4-30.1/D3\|3-22\|RF2/JH6 | | QVQLQES-GPGLVKPSQTLSLTCTVSG-GSIS | SG---DYYWN | WLRQHPEKGLEWIG | YIFY----SGSTYYNPSLKS | RITISVDTSMTQFSLKLTSVTAADTAVYYCAR | GDYDGSGSY-------------HYYYGMDV | WGQGTTVTVSS |
| | | Germline | | H_FR1 | H_CDR1 | H_FR2 | H_CDR2 | H_FR3 | H_CDR3 | H_FR4 |
| | VH4\|4-30.4/D3\|3-22\|RF2/JH6 | | 32338 | QVQLQES-GPGLVKPSQTLSLTCTVSG-GSIS | SG---DYYWS | WIRQPPGKGLEWIG | YIYY----SGSTYYNPSLKS | RVTISVDTSKNQFSLKLSSVTAADTAVYYCAR | YYYDSSGYYY-----------YYYYGMDV | WGQGTTVTVSS |
| iPS:4 35849 | 21-225_191C3 | VH4\|4-30.4/D3\|3-22\|RF2/JH6 | | QVQLQES-GPGLVKPSQTLSLTCTVSG-GSIS | SG---DYYWN | WIRQLPGKGLEWIG | YIFY----SGSTYYNPSLKS | RLTISVDTSKNQFSLKLNSVTAADTAVYYCAR | GDYDGSGSY-------------HFYYGMDV | WGQGTTVTVSS |
| iPS:4 36015 | 21-225_193D3 | VH4\|4-30.4/D3\|3-22\|RF2/JH6 | | QVQLQES-GPGLVKPSQTLSLTCTVSG-GSIS | SG---DYYWN | WIRQLPGKGLEWIG | YIFY----SGSTYYNPSLKS | RLTISVDTSKNQFSLKLSSVTAADTAVYYCTR | GDYDGSGSY-------------HFYYGMDV | WGQGTTVTVSS |
| iPS:4 36049 | 21-225_193B12 | VH4\|4-30.4/D3\|3-22\|RF2/JH6 | | QVQLQES-GPGLVKPSQTLSLTCTVSG-GSIS | SA---DYYWN | WIRQLPGKGLEWIG | YIFY----SGSTYYNPSLKS | RLTISVDTSKNQFSLKLSSVTAADTAVYYCAR | GDYDGSGSY-------------HFYYGMDV | WGQGTTVTVSS |
| iPS:4 36088 | 21-225_195C8 | VH4\|4-30.4/D3\|3-22\|RF2/JH6 | | QVQLQES-GPGLVKPSQTLSLTCTVSG-GSIS | SG---DYYWN | WIRQLPGKGLEWIG | YIFY----SGSTYYNPSLKS | RLTISVDTSKNQFSLKLSSVTAADTAVYYCAR | GDYDGSGSY-------------HFYYGMDV | WGQGTTVTVSS |
| iPS:4 36195 | 21-225_198G10 | VH4\|4-30.4/D3\|3-22\|RF2/JH6 | | QVQLQES-GPGLVKPSQTLSLTCTVSG-GSIS | SG---DYYWN | WIRQLPGKGLEWIG | YIFY----SGSTYYNPSLKS | RLTISVDTSKNQFSLKLSSVTAADTAVYYCAR | GDYDGSGSY-------------HFYYGMDV | WGQGTTVTVSS |
| | | Germline | | H_FR1 | H_CDR1 | H_FR2 | H_CDR2 | H_FR3 | H_CDR3 | H_FR4 |
| | VH4\|4-30.1/D5\|5-24\|RF3/JH5 | | 32339 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | RDGYNY-------------------NWFDP | WGQGTLVTVSS |
| iPS:4 35863 | 21-225_191H4 | VH4\|4-30.1/D5\|5- | | QVQLQES-GPGLVKPSQTLSL | SG---GYYWN | WIRQHPGKGLEWIG | YIFY----SGSTYYNPS | RLTISIDTSKNQFSLKLTSVTAADTAVYYCGR | SGYNWD-------------------NGVDP | WGQGTLVTVS |

| | | | | | | | | | | |
|---|---|---|---|---|---|---|---|---|---|---|
| iPS:4 35943 | 21-225_191C9 | VH4\|4-30.1/D5\|5-24\|RF3/JH5 | | QVQLQES-GPGLVKPSQTLSLTCTVSG-GSIS | SG---GYYWN | WIRQHPGKGLDWIG | YIYY----SGSTYYNPSLKS | RVTISVDTSKNQFSLKLNSVTAADTAVYYCAR | SGYNWD-------------------AGVDP | WGQGTLVTVSS |
| iPS:4 36094 | 21-225_195B10 | VH4\|4-30.1/D5\|5-24\|RF3/JH5 | | QVQLQES-GPGLVKPSQTLSLTCTVSG-GSIS | NS---GYYWS | WIRQHPGKGLEWIG | YMYY----SGSTYYNPSLKS | RVTISVDTSKNQFYLKLSAVTAADTAVYYCAK | GGYNWN-------------------NGFDC | WGQGTLVTVSS |
| | | Germline | | H_FR1 | H_CDR1 | H_FR2 | H_CDR2 | H_FR3 | H_CDR3 | H_FR4 |
| | VH3\|3-33/D2\|2-15\|RF3/JH6 | | 32390 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DIVVVVAAT------------YYYYYGMDV | WGQGTTVTVSS |
| iPS:4 35869 | 21-225_190B1 | VH3\|3-33/D2\|2-15\|RF3/JH6 | | QVQLVES-GGGVVQPGRSLRLSCATSG-FTFS | S-----YGMH | WVRQAPGKGLEWVA | VIWYD---GSNKHYADSVKG | RFTISRDNSKNTLYLQMNSLRAEDTAVYYCAR | DRTVGY-------------------SGMDV | WGQGTTVTVSS |
| iPS:4 36260 | 21-225_203H1 | VH3\|3-33/D2\|2-15\|RF3/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DRTVGY-------------------NGMDV | WGQGTTVTVSS |
| iPS:4 36490 | 21-225_221F6 | VH3\|3-33/D2\|2-15\|RF3/JH6 | | QVQLVES-GGGVVQPGRSLRLSCAASG-FTFS | S-----NGMH | WVRQAPGKGLEWVA | VIWYD---GSNENYADSVKG | RFTISRDNSKNTLYLQMNSLRAEDTAVYYCAR | DRTVGY-------------------NGMDV | WGQGTTVTVSS |
| iPS:4 36502 | 21-225_222A11 | VH3\|3-33/D2\|2-15\|RF3/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DRDVGY-------------------NGMDV | WGQGTTVTVSS |
| iPS:4 36514 | 21-225_222D10 | VH3\|3-33/D2\|2-15\|RF3/JH6 | | QVQLVES-GGGVVQPGRSLRLSCAASG-FTFS | S-----YGMH | WVRQAPGKGLEWVA | VIWYD---GSNKNYADSVKG | RFTISRDNSKNTLYLQMNSLRAEDTAVYYCAR | DRDVGY-------------------NGMDV | WGQGTTVTVSS |
| iPS:4 36522 | 21-225_223H10 | VH3\|3-33/D2\|2-15\|RF3/JH6 | | QVQLVES-GGGVVQPGRSLRLSCAASG-FTFS | S-----YGMH | WVRQAPGKGLEWVA | VIWYD---GSNKNYADSVKG | RFTISRDHSKNTLYLQMNSLRAEDTAVYYCAR | DRDVGY-------------------NGMDV | WGQGTTVTVSS |
| | | Germline | | H_FR1 | H_CDR1 | H_FR2 | H_CDR2 | H_FR3 | H_CDR3 | H_FR4 |
| | VH3\|3-21/D1\|1-1\|RF3/JH5 | | 32391 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | YNWND--------------------NWFDP | WGQGTLVTVSS |
| iPS:4 35883 | 21-225_185A1 | VH3\|3-21/D1\|1-1\|RF3/JH5 | | EVQLVES-GGGLVKPGGSLRLSCAASG-FTFN | S-----YSMN | WVRQAPGKGLEWVS | SISSS---GSYIYYADSVKG | RFTISRDNAKNSLYLQMHSLRAEDTAVYYCAR | SNL------------------------FDC | WGQGTPVTVSS |
| | | Germline | | H_FR1 | H_CDR1 | H_FR2 | H_CDR2 | H_FR3 | H_CDR3 | H_FR4 |
| | VH3\|3-23/D1\|1- | | 32392 | EVQLLES- | S-----YAMS | WVRQAPGK | AISGS--- | RFTISRDNSKNTLYLQMNSL | GTTGT------------ | WGQGT |

<table>
<tr><td>iPS:4 35895</td><td>21-225_188E8</td><td>VH3|3-23/D1|1-1|RF1/JH3</td><td></td><td>EVQLLES-GGGLVQPGGSLRLSCAASG-FTFS</td><td>S-----SAMN</td><td>WVRQAPGKGLEWVS</td><td>VISGS---GGYTYYADSVKG</td><td>RFTISRDNSKNTLYLQMNSLRAEDTAVYFCAK</td><td>RNTDD---------------------AFDI</td><td>WGQGTMVTVSS</td></tr>
<tr><td></td><td></td><td>Germline</td><td></td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH3|3-11/D7|7-27|RF3/JH4</td><td></td><td>32393</td><td>QVQLVES-GGGLVKPGGSLRLSCAASG-FTFS</td><td>D-----YYMS</td><td>WIRQAPGKGLEWVS</td><td>YISSS---GSTIYYADSVKG</td><td>RFTISRDNAKNSLYLQMNSLRAEDTAVYYCAR</td><td>NWGY----------------------FDY</td><td>WGQGTLVTVSS</td></tr>
<tr><td>iPS:4 35903</td><td>21-225_190E2</td><td>VH3|3-11/D7|7-27|RF3/JH4</td><td></td><td>QVQLVES-GGGLVKPGGSLRLSCAASG-FTFS</td><td>D-----YYMS</td><td>WIRQAPGKGLEWLS</td><td>YISSS---GTTVFYADSVKG</td><td>RFTISRDNAKNSLYLQMNSLRAEDTAVYYCAR</td><td>EWVG----------------------ADY</td><td>WGQGTLVTVSS</td></tr>
<tr><td>iPS:4 35923</td><td>21-225_190H6</td><td>VH3|3-11/D7|7-27|RF3/JH4</td><td></td><td>QVQLVES-GGGLVKPGGSLRLSCAASG-FTFS</td><td>D-----YYMS</td><td>WIRQAPGKGLEWLS</td><td>YISSS---GTTVFYADSVKG</td><td>RFTISRDNAKNSLYLQMNSLRAEDTAVYYCAR</td><td>EWVG----------------------ADY</td><td>WGQGTLVTVSS</td></tr>
<tr><td>iPS:4 35953</td><td>21-225_191B12</td><td>VH3|3-11/D7|7-27|RF3/JH4</td><td></td><td>QVQLVES-GGGLVKPGGSLRLSCAASG-FTFS</td><td>D-----YYMS</td><td>WIRQAPGKGLEWVS</td><td>YISSS---GTTVFYADSVKG</td><td>RFTISRDNAKNSLYLQMNSLRAEDTAVYYCAR</td><td>EWVG----------------------ADY</td><td>WGQGTLVTVSS</td></tr>
<tr><td>iPS:4 36098</td><td>21-225_195G11</td><td>VH3|3-11/D7|7-27|RF3/JH4</td><td></td><td>QVQLVES-GGGLVKPGGSLRLSCAASG-FTFS</td><td>D-----YYMS</td><td>WIRQAPGKGLEWLS</td><td>YISSS---GTTVFYADSVKG</td><td>RFTISRDNAKNSLYLQMNSLRAEDTAVYYCAR</td><td>EWVG----------------------ADY</td><td>WGQGTLVTVSS</td></tr>
<tr><td>iPS:4 36102</td><td>21-225_196B1</td><td>VH3|3-11/D7|7-27|RF3/JH4</td><td></td><td>QVQLVES-GGGLVKPGGSLRLSCAASG-FTFS</td><td>D-----YYMS</td><td>WIRQAPGKGLEWIS</td><td>YISSS---GITMYYADSVKG</td><td>RFTISRDNAKNSLYLQMNSLRAEDTAVYYCAR</td><td>EWVG----------------------ADY</td><td>WGQGTLVTVSS</td></tr>
<tr><td>iPS:4 36104</td><td>21-225_196C1</td><td>VH3|3-11/D7|7-27|RF3/JH4</td><td></td><td>QVQLVES-GGGLVKPGGSLRLSCAASG-FTFS</td><td>D-----YYMS</td><td>WIRQAPGKGLEWLS</td><td>YISSS---GTTVFYADSVKG</td><td>RFTISRDNAKNSLYLQMNSLRAEDTAVYYCAR</td><td>EWVG----------------------ADY</td><td>WGQGTLVTVSS</td></tr>
<tr><td></td><td></td><td>Germline</td><td></td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH3|3-23/D6|6-19|RF2/JH4</td><td></td><td>32394</td><td>EVQLLES-GGGLVQPGGSLRLSCAASG-FTFS</td><td>S-----YAMS</td><td>WVRQAPGKGLEWVS</td><td>AISGS---GGSTYYADSVKG</td><td>RFTISRDNSKNTLYLQMNSLRAEDTAVYYCAK</td><td>GIAVA--------------------GYFDY</td><td>WGQGTLVTVSS</td></tr>
<tr><td>iPS:4 35965</td><td>21-225_192H2</td><td>VH3|3-23/D6|6-19|RF2/JH4</td><td></td><td>EVQLLES-GGDLIQPGGSLRLSCAASG-FTFS</td><td>S-----SAMS</td><td>WVRQAPGKGLEWVS</td><td>AISGS---GGNTFYADSVKG</td><td>RFTISRDNSKNTLYLQMNSLRAEDTAVYYCAK</td><td>LIAVVG------------------SHYFDY</td><td>WGQGTLVTVSS</td></tr>
<tr><td>iPS:4 36160</td><td>21-225_197C9</td><td>VH3|3-23/D6|6-19|RF2/JH4</td><td></td><td>EVQLLES-GGGLAQPGGSLRLSCAASG-FTFR</td><td>S-----YAMS</td><td>WVRQAPGKGLEWVS</td><td>VISGR---GGNTYYADSVKG</td><td>RFTISRDNSKNTLYLQMNSLRAEDTAVYYCAK</td><td>GIAVAG------------------SHYFDY</td><td>WGQGTLVTVSS</td></tr>
<tr><td>iPS:3 92954</td><td>21-225_26A10</td><td>VH3|3-23/D6|6-</td><td></td><td>EVQLLES-GGGLVQPGGSLRL</td><td>S-----YAMS</td><td>WVRQAPGKGLEWVS</td><td>VISGS---GVNTFYADS</td><td>RFTISRDNSKNTLYLLMNSLRAEDTAVYYCAK</td><td>KIAVAG------------------THYFDY</td><td>WGQGTLVTVS</td></tr>
</table>

<table>
<tr><th></th><th></th><th>Germline</th><th></th><th>H_FR1</th><th>H_CDR1</th><th>H_FR2</th><th>H_CDR2</th><th>H_FR3</th><th>H_CDR3</th><th>H_FR4</th></tr>
<tr><td></td><td>VH4|4-30.1/D5|5-24|RF3/JH4</td><td></td><td>32395</td><td>QVQLQES-GPGLVKPSQTLSLTCTVSG-GSIS</td><td>SG---GYYWS</td><td>WIRQHPGKGLEWIG</td><td>YIYY----SGSTYYNPSLKS</td><td>RVTISVDTSKNQFSLKLSSVTAADTAVYYCAR</td><td>RDGYN--------------------YYFDY</td><td>WGQGTLVTVSS</td></tr>
<tr><td>iPS:435983</td><td>21-225_192E5</td><td>VH4|4-30.1/D5|5-24|RF3/JH4</td><td></td><td>QVQLQES-GPGLVKPSQTLSLTCTVSG-DSIN</td><td>NG---GYYWS</td><td>WIRQHPGKGLEWIG</td><td>YIFY----SGSTYYNPSLKS</td><td>RVTISVDTSKNQFSLKLNSVTAADTAVYFCAR</td><td>AGYNWD------------------NGFDY</td><td>WGQGTLVTVSS</td></tr>
<tr><td>iPS:436043</td><td>21-225_193G9</td><td>VH4|4-30.1/D5|5-24|RF3/JH4</td><td></td><td>QVQLQES-GPGLVKPSQTLSLTCTVSG-DSIN</td><td>NG---GYYWS</td><td>WIRQHPGKGLEWIG</td><td>YIFY----SGSTYYNPSLKS</td><td>RVTISVDTSKNQFSLKLNSVTAADTAVYFCAR</td><td>AGYNWD------------------NGFDY</td><td>WGQGTLVTVSS</td></tr>
<tr><td>iPS:436084</td><td>21-225_195F2</td><td>VH4|4-30.1/D5|5-24|RF3/JH4</td><td></td><td>QVQLQES-GPGLVKPSQTLSLTCTVSG-DSIS</td><td>SG---GYYWS</td><td>WIRQHPGKGLEWIG</td><td>YSYY----SGSTNYNPSLKS</td><td>RVTISVDMSKNQFSLKLSSVTDADTAVYYCAR</td><td>GGYNWN------------------NGFDY</td><td>WGQGALVTVSS</td></tr>
<tr><td>iPS:437138</td><td>21-225_214D8</td><td>VH4|4-30.1/D5|5-24|RF3/JH4</td><td></td><td>QVQLQES-GPGLVKPSQTLSLTCTVSG-GSIS</td><td>TA---FYYWS</td><td>WIRQHPGKGLEWIG</td><td>YIYF----SGSTYYNPSLKS</td><td>RVTISVDTSKNQFSLNLSSVTAADTAVYYCAR</td><td>ARGYHY------------------SIFDY</td><td>WGQGTLVTVSS</td></tr>
<tr><th></th><th></th><th>Germline</th><th></th><th>H_FR1</th><th>H_CDR1</th><th>H_FR2</th><th>H_CDR2</th><th>H_FR3</th><th>H_CDR3</th><th>H_FR4</th></tr>
<tr><td></td><td>VH3|3-23/D3|3-16|RF1/JH3</td><td></td><td>32396 & 32397</td><td>EVQLLES-GGGLVQPGGSLRLSCAASG-FTFS</td><td>S-----YAMS</td><td>WVRQAPGKGLEWVS</td><td>AISGS---GGSTYYADSVKG</td><td>RFTISRDNSKNTLYLQMNSLRAEDTAVYYCAK</td><td>VL*LRLGEL-------------SLYDAFDI</td><td>WGQGTMVTVSS</td></tr>
<tr><td>iPS:436003</td><td>21-225_192G10</td><td>VH3|3-23/D3|3-16|RF1/JH3</td><td></td><td>EVQLLES-GGGLVQPGGSLRLSCSASG-FTFS</td><td>S-----YAMS</td><td>WVRQAPGKGLEWVS</td><td>AISGR---GGSTFYADSVKG</td><td>RFTISRDNSKNTLYLQMNSLRAEDTAVYYCAR</td><td>RLALDG-----------------YDAFDI</td><td>WGQGTMVTVSS</td></tr>
<tr><th></th><th></th><th>Germline</th><th></th><th>H_FR1</th><th>H_CDR1</th><th>H_FR2</th><th>H_CDR2</th><th>H_FR3</th><th>H_CDR3</th><th>H_FR4</th></tr>
<tr><td></td><td>VH3|3-23/D7|7-27|RF2/JH4</td><td></td><td>32398 & 32399</td><td>EVQLLES-GGGLVQPGGSLRLSCAASG-FTFS</td><td>S-----YAMS</td><td>WVRQAPGKGLEWVS</td><td>AISGS---GGSTYYADSVKG</td><td>RFTISRDNSKNTLYLQMNSLRAEDTAVYYCAK</td><td>*LGY------------------------FDY</td><td>WGQGTLVTVSS</td></tr>
<tr><td>iPS:436019</td><td>21-225_193C4</td><td>VH3|3-23/D7|7-27|RF2/JH4</td><td></td><td>EVQLLES-GGGLAQPGGSLRLSCAASG-FTFS</td><td>S-----YAMN</td><td>WVRQAPGKGLEWVS</td><td>AIIGN---GGRTYYADSVKG</td><td>RFSISRDNSKNTLFLQMNSLRAEDTAVYYCAK</td><td>DLGRYS-----------------YGFFDY</td><td>WGQGTLVTVSS</td></tr>
<tr><th></th><th></th><th>Germline</th><th></th><th>H_FR1</th><th>H_CDR1</th><th>H_FR2</th><th>H_CDR2</th><th>H_FR3</th><th>H_CDR3</th><th>H_FR4</th></tr>
<tr><td></td><td>VH4|4-30.1/D1|1-1|RF1/JH6</td><td></td><td>32400</td><td>QVQLQES-GPGLVKPSQTLSLTCTVSG-GSIS</td><td>SG---GYYWS</td><td>WIRQHPGKGLEWIG</td><td>YIYY----SGSTYYNPSLKS</td><td>RVTISVDTSKNQFSLKLSSVTAADTAVYYCAR</td><td>GTTGTYY----------------YYYGMDV</td><td>WGQGTTVTVSS</td></tr>
<tr><td>iPS:436025</td><td>21-225_193B5</td><td>VH4|4-30.1/D1|1-1|RF1/JH6</td><td></td><td>QVQLQES-GPGLVKPSQTLSLTCTVSG-GSIS</td><td>SG---GYYWS</td><td>WIRQHPGKGLEWIG</td><td>YIYY----SGSTYYNPSLKS</td><td>RVTISVDTSKNQFSLKLSSVAAADTAVYYCAR</td><td>GEYNWN------------------HGMDV</td><td>WGQGTTVTVSS</td></tr>
<tr><th></th><th></th><th>Germline</th><th></th><th>H_FR1</th><th>H_CDR1</th><th>H_FR2</th><th>H_CDR2</th><th>H_FR3</th><th>H_CDR3</th><th>H_FR4</th></tr>
<tr><td></td><td>VH4|4-34/D4|4-</td><td></td><td>32401</td><td>QVQLQQW-</td><td>G-----YYWS</td><td>WIRQPPGK</td><td>EINH----</td><td>RVTISVDTSKNQFSLKLSSV</td><td>DYSNY-----------</td><td>WGQGT</td></tr>
</table>

| | | | | | | | | | | |
|---|---|---|---|---|---|---|---|---|---|---|
| **iPS:436033** | **21-225_193E7** | **VH4\|4-34/D4\|4-11\|RF2/JH4** | | QVQLQQW-GAGLLKPSETLSLTCAVYG-GSFS | G-----YFWT | WIRQPPGKGLEWIG | EINH----SGSTNYNPSLKS | RVTISVDTSKNQFSLKLSSVTAADTAVYYCAR | DYG-------------------------ADY | WGQGTLVAVSA |
| **iPS:436199** | **21-225_199E3** | **VH4\|4-34/D4\|4-11\|RF2/JH4** | | QVQLQQW-GAGLLKPSETLSLTCAVYG-GSFS | G-----YFWT | WIRQPPGKGLEWIG | EINH----SGSTNYNPSLKS | RVTISVDTSKNQFSLKLSSVTAADTAVYYCAR | DYG-------------------------ADY | WGQGTLVAVSA |
| **iPS:436228** | **21-225_200F12** | **VH4\|4-34/D4\|4-11\|RF2/JH4** | | QVQLQQW-GAGLLKPSETLSLTCAVYG-GSFS | G-----YFWT | WIRQPPGKGLEWIG | EISH----SGSTNYNPSLKS | RVTISVDTSKNQFSLKVSSVTAADTAVYYCAR | DYG-------------------------ADY | WGQGTLVTVSS |
| **iPS:436230** | **21-225_201A1** | **VH4\|4-34/D4\|4-11\|RF2/JH4** | | QVQLQQW-GAGLLKPSETLSLTCAVTG-GSFS | G-----YFWT | WIRQPPGKGLEWIG | EISH----SGRTNYNPSLKS | RVTISGDTSKNQFSLKLSSVTAADTAVYYCAR | DYG-------------------------ADY | WGQGTLVTVSS |
| **iPS:436242** | **21-225_201A10** | **VH4\|4-34/D4\|4-11\|RF2/JH4** | | QVQLQQW-GAGLLKPSETLSLTCAVYG-GSFS | G-----YFWT | WIRQPPGKGLEWIG | EISH----SGSTNYNPSLKS | RVTISVDTSKNQFSLKVNSVTAADTAVYYCAR | DYG-------------------------ADY | WGQGTLVTVSS |
| **iPS:436286** | **21-225_204H8** | **VH4\|4-34/D4\|4-11\|RF2/JH4** | | QVQLQQW-GAGLLKPSETLFLTCAVYG-GSFS | G-----YFWT | WVRQPPGKGLEWIG | EISH----SGSTSYNPSLKS | RVTISVDKSKNQFSLKLSSVTAADTAVYYCAR | DYG-------------------------ADY | WGQGTLVTVSS |
| **iPS:436308** | **21-225_205H8** | **VH4\|4-34/D4\|4-11\|RF2/JH4** | | QVQLQQW-GAGLLKPSETLSLTCAVYG-GSFS | G-----YFWS | WIRQPPGKGLEWIG | EISH----SGRTNYNPSLKS | RVTISVDTSKNQFSLKVSSVTAADTAVYYCAR | DYG-------------------------ADY | WGQGTLVTVSS |
| | | **Germline** | | **H_FR1** | **H_CDR1** | **H_FR2** | **H_CDR2** | **H_FR3** | **H_CDR3** | **H_FR4** |
| | **VH3\|3-30.3/D1\|1-1\|RF1/JH4** | | 32402 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | GTTGT-----------------YFDY | WGQGTLVTVSS |
| **iPS:436051** | **21-225_193G12** | **VH3\|3-30.3/D1\|1-1\|RF1/JH4** | | QVQLVES-GGGVVQPGRSLRLSCGASG-FTFS | S-----YAMH | WVRQAPGKGLEWVA | VIWYD---GTNKYYGDSVKG | RFTISRDNSKNTLNLQMNSLRAEDTAVYYCAR | DFTITG------------------ATYFDY | WGQGTLVTVSS |
| | | **Germline** | | **H_FR1** | **H_CDR1** | **H_FR2** | **H_CDR2** | **H_FR3** | **H_CDR3** | **H_FR4** |
| | **VH3\|3-33/D7\|7-27\|RF3/JH6** | | 32403 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | NWGYYY------------------YYGMDV | WGQGTTVTVSS |
| **iPS:436054** | **21-225_194C1** | **VH3\|3-33/D7\|7-27\|RF3/JH6** | | QVQLVES-GGGVVQPGRSLRLSCAASG-FTFS | S-----YGMH | WVRQAPGKGLEWVA | VIWYD---GSNEHYADSVKG | RFTISRDNSKNTLYLQMNSLRAEDTAVYYCAR | NRGVGY-------------------YGLDV | WGQGTTVTVSS |
| | | **Germline** | | **H_FR1** | **H_CDR1** | **H_FR2** | **H_CDR2** | **H_FR3** | **H_CDR3** | **H_FR4** |
| | **VH1\|1-02/D5\|5-** | | 32404 | QVQLVQS- | G-----YYMH | WVRQAPGQ | WINPN--- | RVTMTRDTSISTAYMELSRL | GYSYGY---------- | WGQGT |

| | | | | | | | | | | |
|---|---|---|---|---|---|---|---|---|---|---|
| iPS:4 36058 | 21-225_194A4 | VH1\|1-02/D5\|5-18\|RF3/JH1 | | QVQLVQS-GTEVKKPGASLKVSCKASG-YTFT | V-----YYLN | WVRQAPGQGLEWMG | WINPN---SGGTNYAQKFQG | RVTMTRDTSISTAYMELSRLRSDDTAVYYCAR | GYDI-----------------------LTG | WGQGTLVTVSS |
| | | Germline | | H_FR1 | H_CDR1 | H_FR2 | H_CDR2 | H_FR3 | H_CDR3 | H_FR4 |
| | VH1\|1-02/D1\|1-26\|RF3/JH6 | | 32405 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | YSGSYYYY---------------YYYGMDV | WGQGTTVTVSS |
| iPS:4 36068 | 21-225_194F7 | VH1\|1-02/D1\|1-26\|RF3/JH6 | | QVQLVQS-GAEVKKPGASVKVSCKASG-YTFT | D-----YYIH | WVRQAPGQGLEWMG | WINPN---NGGTNYAQKFQG | RVTMTRDTSISTAYMELSRLRSDDTAVYYCAR | EPLGYYGSG------------SYGAYGMDV | WGQGTTVTVSS |
| | | Germline | | H_FR1 | H_CDR1 | H_FR2 | H_CDR2 | H_FR3 | H_CDR3 | H_FR4 |
| | VH4\|4-34/D4\|4-17\|RF2/JH3 | | 32406 | QVQLQQW-GAGLLKPSETLSLTCAVYG-GSFS | G-----YYWS | WIRQPPGKGLEWIG | EINH----SGSTNYNPSLKS | RVTISVDTSKNQFSLKLSSVTAADTAVYYCAR | DYGDY-------------------DAFDI | WGQGTMVTVSS |
| iPS:4 36072 | 21-225_194C10 | VH4\|4-34/D4\|4-17\|RF2/JH3 | | QVQLQQW-GAGLLKPSETLSLTCAVSG-GSFR | Y-----YYWS | WIRQPPGKGLEWFG | EINH----SGSTNYNPSLKS | RVTISIDTSKNQFSLKLRSVTAADTAVYYCAR | DYGA----------------------FDI | WGQGTMVTVSS |
| iPS:4 36506 | 21-225_222C7 | VH4\|4-34/D4\|4-17\|RF2/JH3 | | QVQLQQW-GAGLLKPSETLSLTCAVYG-GSFS | G-----RYWS | WIRQPPGKGLEWIG | EINH----SGSANYNPSLKS | RVTISVDTSKNQFSLKLSSVTAADTAVYYCAR | DYGA----------------------LDF | WGQGTMVTVSS |
| | | Germline | | H_FR1 | H_CDR1 | H_FR2 | H_CDR2 | H_FR3 | H_CDR3 | H_FR4 |
| | VH3\|3-33/D5\|5-24\|RF2/JH6 | | 32407 & 32408 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | *RWLQLYY---------------YYYGMDV | WGQGTTVTVSS |
| iPS:4 36092 | 21-225_195B9 | VH3\|3-33/D5\|5-24\|RF2/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | EWLQFRY-----------------YYGMDV | WGQGTTVTVSS |
| iPS:4 36164 | 21-225_197G10 | VH3\|3-33/D5\|5-24\|RF2/JH6 | | QVQLVES-GGGVVQPGRSLRLSCVASG-FTFS | S-----YGMH | WVRQAPGKGLEWVA | VTWYD---GSNKYYADSVKG | RFTISRDNSKNTLYLQMNSLRAEDTAVYYCAR | EWLQFRY-----------------YYGIDV | WGQGTTVTVSS |
| iPS:4 36191 | 21-225_198B9 | VH3\|3-33/D5\|5-24\|RF2/JH6 | | QVQLVES-GGGVVQPGRSLRLSCAASG-FTFS | N-----YGMH | WVRQAPGKGLEWVA | IIWFD---GSNKYYVDSVKG | RFTISRDNSKNTLFLQMSSLRAEDTAVYYCAR | EWLQFRY-----------------YYGMDV | WGQGTTVTVSS |
| iPS:4 36205 | 21-225_199A7 | VH3\|3-33/D5\|5-24\|RF2/JH6 | | QVQLVES-GGGVVQPGRSLRLSCAASG-FTFS | N-----YGMH | WVRQAPGKGLEWVA | IIWFD---GSNKYYADSVKG | RFTISRDNSKNTLFLQMSSLRAEDTAVYYCAR | EWLQFRY-----------------YYGMDV | WGQGTTVTVSS |
| iPS:4 36214 | 21-225_200F6 | VH3\|3-33/D5\|5- | | QVQLVES-GGGVVQPGRSLRL | S-----YGMH | WVRQAPGKGLEWVA | VIWYD---GSNKYYADS | RFTISRDNSKNTLYLQMNSLRAEDTAVYYCTR | EWLQFRY-----------------YYGMDV | WGQGTTVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH3\|3-33/D6\|6-19\|RF3/JH6 | | 32409 & 32410 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | V*QWLVYY--------------YYGMDV | WGQGTTVTVSS |
| iPS:436106 | 21-225_196F2 | VH3\|3-33/D6\|6-19\|RF3/JH6 | | QVQLVES-GGGVVQPGRSLRLSCAASG-FTFS | S-----FGMH | WVRQAPGKGLEWVA | VILND---GSNKKCADSVKG | RCTISRDNSKNTLYLQMNSLRAEDTAVYYCAR | GQQWLV-------------------NGVDV | WGQGTTVTVSS |
| | | Germline | | H_FR1 | H_CDR1 | H_FR2 | H_CDR2 | H_FR3 | H_CDR3 | H_FR4 |
| | VH3\|3-23/D6\|6-6\|RF3/JH6 | | 32411 & 32412 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | V*QLVYY---------------YYYGMDV | WGQGTTVTVSS |
| iPS:436110 | 21-225_196F4 | VH3\|3-23/D6\|6-6\|RF3/JH6 | | EVQLLES-GGGLVQPGGSLRFSCAASG-FTFS | S-----CAMT | WVRQAPGKGLEWVS | AISGS---GGSTYYADSVKG | RFTISRDNSKNTLYLQMNSLRAEDTAVYYCAK | VGGLTGSY---------------YYYGMDV | WGQGTTVTVSS |
| | | Germline | | H_FR1 | H_CDR1 | H_FR2 | H_CDR2 | H_FR3 | H_CDR3 | H_FR4 |
| | VH1\|1-08/D2\|2-21\|RF1/JH5 | | 32413 & 32414 | QVQLVQS-GAEVKKPGASVKVSCKASG-YIFT | S-----YDIN | WVRQATGQGLEWMG | WMNPN---SGNTGYAQKFQG | RVTMTRNTSISTAYMELSSLRSEDTAVYYCAR | SILWW*L----------------LFNWFDP | WGQGTLVTVSS |
| iPS:436114 | 21-225_196G8 | VH1\|1-08/D2\|2-21\|RF1/JH5 | | QVQLVQS-GAEVKKPGASVKVSCKASG-YTFT | N-----YDIN | WMRQATGQGLEWMG | WMHLN---SGNTGYAPKFQG | RVTMTRDTSISTAFMELSSLRSEDTAVYYCAY | SGGWY---------------------VFDP | WGQGTLVTVSS |
| iPS:436218 | 21-225_200G7 | VH1\|1-08/D2\|2-21\|RF1/JH5 | | QVQLVQS-GAEVKKPGASVKVSCKASG-YTFT | N-----YDIN | WMRQATGQGLEWMG | WMHLN---SGNTGYAPKFQG | RVTMTRDTSISTAFMELSSLRSEDTAVYYCAY | SGGWY---------------------VFDP | WGQGTLVTVSS |
| | | Germline | | H_FR1 | H_CDR1 | H_FR2 | H_CDR2 | H_FR3 | H_CDR3 | H_FR4 |
| | VH1\|1-02/D3\|3-10\|RF3/JH6 | | 32415 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | IIMVRGVII------------YYYYGMDV | WGQGTTVTVSS |
| iPS:436116 | 21-225_196B9 | VH1\|1-02/D3\|3-10\|RF3/JH6 | | QVQLVQS-GAEVKKPGASMKVSCKASG-YTFT | G-----YYMH | WVRQAPGQGLEWMG | WINPN---SGGTNFAQKFRG | RVTMTRDTSISTAYMELSRLSSDDTAVYYCAR | GGVRGVPN---------------YYYVMDV | WGQGTTVTVSS |
| | | Germline | | H_FR1 | H_CDR1 | H_FR2 | H_CDR2 | H_FR3 | H_CDR3 | H_FR4 |
| | VH4\|4-30.1/D5\|5-18\|RF3/JH6 | | 32416 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | GYSYGYYY---------------YYGMDV | WGQGTTVTVSS |
| iPS:436181 | 21-225_198C2 | VH4\|4-30.1/D5\|5-18\|RF3/JH6 | | QVQLQES-GPGLVKPSQTLSLTCTVSG-GSIS | SG---GYYWS | WIRQHPGKGLEWIG | NIYY----SGSTYYNPSLKS | RVTISVDTSKNQFSLKLSSVTAADTAVYYCAR | GDYYGSGSY-------------HNYYGLDV | WGQGTTVTVSS |
| iPS:436210 | 21-225_199G11 | VH4\|4-30.1/D5\|5- | | QVQLQES-GPGLVKPSQTLSL | SG---GYYWS | WIRQHPGKGLEWIG | NIYY----SGSTYYNPS | RVTISVDTSKNQFSLKLSSVTAADTAVYYCAR | GDYYGSGSY-------------HNYYGLDV | WGQGTTVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH1\|1-18/D3\|3-3\|RF2/JH6 | | 32417 | QVQLVQS-GAEVKKPGASVKVSCKASG-YIFT | S-----YGIS | WVRQAPGQGLEWMG | WISAY---NGNTNYAQKLQG | RVTMTTDTSTSTAYMELRSLRSDDTAVYYCAR | YYDFWSGYYT------------YYYYGMDV | WGQGTTVTVSS |
| iPS:436234 | 21-225_51E3 | VH1\|1-18/D3\|3-3\|RF2/JH6 | | QVQLVQS-GAEVKKPGASVKVSCKASG-YTFN | S-----YGIS | WVRLAPGQGLEWMG | WISAY---NGNTKNAQRFQG | RVTMTTDTSTSTAYMELRSLRSDDTAVYYCAR | HDFWSGY-----------------YKGMDV | WGQGTTVTVSS |
| iPS:436830 | 21-225_51F4 | VH1\|1-18/D3\|3-3\|RF2/JH6 | | QVQLVQS-GAEVKKPGASVKVSCKASG-YTFN | S-----YGIS | WVRQAPGQGLEWMG | WISAY---NGNTKYAQKLQG | RVTMTTDTSTSTAYMELRSLRSDDTAVYYCAR | HDFWSGY-----------------YKGMDV | WGQGTTVTVSS |
| iPS:436834 | 21-225_52F1 | VH1\|1-18/D3\|3-3\|RF2/JH6 | | QVQLVQS-GAEVKKPGASVKVSCKASG-YTFN | S-----YGVS | WVRQAPGQGLEWMG | WISAY---NGNRKYAQKLQG | RVSMTTDTSTSTAYMELRSLRSDDTAVYYCAR | HDFWSGY-----------------YKGMDV | WGQGTTVTVSS |
| iPS:436842 | 21-225_54E9 | VH1\|1-18/D3\|3-3\|RF2/JH6 | | QVQLVQS-GAEVKKPGASVKVSCKASG-YTFN | S-----YGIS | WVRLAPGQGLEWMG | WISAY---NGNTKNAQKLQG | RVTMTTDTSTSTAYMELRSLRSDDTAVYYCAR | HDFWSGY-----------------YKGMDV | WGQGTTVTVSS |
| iPS:436844 | 21-225_56G1 | VH1\|1-18/D3\|3-3\|RF2/JH6 | | QVQLVQS-GAEVKKPGASVKVSCKASG-YTFN | S-----YGIS | WVRQAPGQGLEWMG | WISAY---NGNTKYAQKFQG | RVTMTTDTSTSTAYMELRSLRSDDTAVYYCAR | HDFWSGY-----------------YKGMDV | WGQGTTVTVSS |
| iPS:436846 | 21-225_56E3 | VH1\|1-18/D3\|3-3\|RF2/JH6 | | QVQLVQS-GAEVKKPGASVKVSCKASG-YTFN | S-----YGFS | WVRQAPGQGLEWMG | WISAY---NGNTKEAQKFQG | RVTMTTDTSTSTAYMELRSLRADDTAVYYCAR | HDFWSGY-----------------YKGMDV | WGQGTTVTVSS |
| iPS:451104 | 21-225_49C5 | VH1\|1-18/D3\|3-3\|RF2/JH6 | | QVQLVQS-GAEVKKPGASVKVSCKASG-YTFN | S-----YGIS | WVRQAPGQGLEWMG | WISAY---NGNTKYAQKLQG | RVTMTTDTSTSTAYMELRSLRSDDTAVYYCAR | HDFWSGY-----------------YKGMDV | WGQGTTVTVSS |
| iPS:451106 | 21-225_49D10 | VH1\|1-18/D3\|3-3\|RF2/JH6 | | QVQLVQS-GAEVKKPGASVKVSCKASG-YTFN | S-----YGIS | WVRLAPGQGFEWMG | WISAY---NGNTKNAQKLQG | RVTMTTDTSTSTAYMELRSLRSDDTAVYYCAR | HDFWSGY-----------------YKGMDV | WGQGTTVTVSS |
| iPS:451108 | 21-225_53E8 | VH1\|1-18/D3\|3-3\|RF2/JH6 | | QVQLVQS-GAEVKKPGASVKVSCKASG-YTFN | S-----YGIS | WVRQAPGQGLEWMG | WISAY---NGNTKFAQKLQG | RVTMTTDTSTSTAYMELRSLRSDDTAVYYCAR | HDFWSGY-----------------YKGMDV | WGQGTTVTVSS |
| | | Germline | | H_FR1 | H_CDR1 | H_FR2 | H_CDR2 | H_FR3 | H_CDR3 | H_FR4 |
| | VH6\|6-01/D3\|3-9\|RF1/JH6 | | 32418 & 32419 | QVQLQQS-GPGLVKPSQTLSLTCAISG-DSVS | SN---SAAWN | WIRQSPSRGLEWLG | RTYYR--SKWYNDYAVSVKS | RITINPDTSKNQFSLQLNSVTPEDTAVYYCAR | VLRYFDWLL*-----------YYYYGMDV | WGQGTTVTVSS |
| iPS:436236 | 21-225_201F7 | VH6\|6-01/D3\|3- | | QVQLQQS-GPGLVKPSQTLSL | SN---SAAWN | WIRQSPSRGLEWLG | RTYYR--SKWYNYYEV | RITINPDTSKNQFSLQLNSVTPEDTAVYFCAR | DQRYY---------------------GMDV | WGQGTTVTVS |

| | | | | | | | | | | |
|---|---|---|---|---|---|---|---|---|---|---|
| iPS:436250 | 21-225_201A4 | VH6\|6-01/D3\|3-9\|RF1/JH6 | | QVQLQQS-GPGLVKPSQTLSLTCAISG-DSVS | SN---SAAWN | WIRQSPSRGLEWLG | RTYYR--SKWYNYYEVSVKS | RITINPDTSKNQFSLQLNSVTPEDTAVYFCAR | DQRYY--------------------GMDV | WGQGTTVTVSS |
| iPS:436252 | 21-225_202A8 | VH6\|6-01/D3\|3-9\|RF1/JH6 | | QVQLQQS-GPGLVKPSQTLSLTCAISG-DSVS | SN---SAAWN | WIRQSPSRGLEWLG | RTYYR--SKWYNEYAVSVRS | RITINPDTSKNQFSLQLNSVTPEDTALYYCTR | DQRYY--------------------GMDV | WGQGTPVTVSS |
| iPS:436278 | 21-225_201F2 | VH6\|6-01/D3\|3-9\|RF1/JH6 | | QVQLQQS-GPGLVKPSQTLSLTCAISG-DSVS | SN---SAAWN | WIRQSPSRGLEWLG | RTYYR--SKWYNYYEVSVRS | RVTINPDTSKNQFSLQLNSVTPEDTAVYFCAR | DQRYY--------------------GMDV | WGQGTTVTVSS |
| iPS:436294 | 21-225_205G4 | VH6\|6-01/D3\|3-9\|RF1/JH6 | | QVQLQQS-GPGLVKPSQTLSLTCAISG-DSVS | SN---SAAWN | WIRQSPSRGLEWLG | RTYYR--SKWYNYYEVSVRS | RITINPDTSKNQFSLQLNSVTPEDTAVYFCAR | DQRYY--------------------GMDV | WGQGTTVTVSS |
| iPS:436356 | 21-225_210H10 | VH6\|6-01/D3\|3-9\|RF1/JH6 | | QVQLQQS-GPGLVKPSQTLSLTCAISG-DSVS | SN---SAAWN | WIRQSPSRGLEWLG | RTYYR--SKWYNYYPVSVRS | RITINPDTSKNQFSLLLNSVTPEDTAVYYCAR | DQRYY--------------------GMDV | WGQGTTVTVSS |
| | | Germline | | H_FR1 | H_CDR1 | H_FR2 | H_CDR2 | H_FR3 | H_CDR3 | H_FR4 |
| | VH1\|1-02/D4\|4-23\|RF2/JH4 | | 32420 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | DYGGN-------------------SYFDY | WGQGTLVTVSS |
| iPS:436240 | 21-225_201E8 | VH1\|1-02/D4\|4-23\|RF2/JH4 | | QVQLVQS-GAEVKKPGASVKVSCKASG-YTFT | G-----YYMH | WVRQAPGQGLEWMG | WIDPN---SGGTNYPQKFQG | RVTMTRDTSISTAYMELSRLRSDDTAVFYCAK | DQGYNW------------------NSFDY | WGQGTLVTVSS |
| iPS:436314 | 21-225_206G4 | VH1\|1-02/D4\|4-23\|RF2/JH4 | | QVQLVQS-GAEVKKPGASVKVSCKASG-YTFT | G-----YYIH | WVRQAPGQGLEWMG | WIDPN---SGGTNYAQKFQG | RITMTRDTSISTAYMELSRLRSDDTAVFYCAK | DQGYNW------------------NSFDY | WGQGTLVTVSS |
| | | Germline | | H_FR1 | H_CDR1 | H_FR2 | H_CDR2 | H_FR3 | H_CDR3 | H_FR4 |
| | VH1\|1-02/D5\|5-18\|RF3/JH5 | | 32421 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | GYSYGY------------------NWFDP | WGQGTLVTVSS |
| iPS:436244 | 21-225_201H10 | VH1\|1-02/D5\|5-18\|RF3/JH5 | | QVQLVQS-GAEVKKPGASVKVSCKASG-YTFT | G-----YYIH | WVRQAPGQGLEWMA | WINPN---SGGTNYAQKFQG | RVTMTRDTSITTTYMELSRLRSDDTAVYYCAR | GYSYGY------------------NWFDP | WGQGTLVTVSS |
| iPS:436262 | 21-225_203E3 | VH1\|1-02/D5\|5-18\|RF3/JH5 | | QVQLVQS-GAEVKKPGASVKVSCKASG-YTFT | G-----YYIH | WVRQAPGQGLEWMA | WINPN---SGGTNYAQKFQG | RVTMTRDTSITTAYMELSRLRSDDTAVYYCAR | GYSYGY------------------NWFDP | WGQGTLVTVSS |
| iPS:436276 | 21-225_204H4 | VH1\|1-02/D5\|5- | | QVQLVQS-GAEVKKPGASVKV | G-----YYIH | WVRQAPGQGLEWMA | WINPN---SGGTNYAQK | RVTMTRDTSITTAYMELSRLRSDDTAVYYCAR | GYSYGY------------------NWFDP | WGQGTLVTVS |

| | | | | | | | | | | |
|---|---|---|---|---|---|---|---|---|---|---|
| iPS:4 36312 | 21-225_206A4 | VH1\|1-02/D5\|5-18\|RF3/JH5 | | QVQLVQS-GAEVKKPGASVKVSCKASG-YTFT | G-----YYIH | WVRQAPGQGLEWMA | WINPN---SGGTNYAQKFQG | RVTMTRDTSITTAYMELSRLRSDDTAVYYCAR | GYSYGY-------------------NWFDP | WGQGTLVTVSS |
| iPS:4 36316 | 21-225_206A5 | VH1\|1-02/D5\|5-18\|RF3/JH5 | | QVQLVQS-GAEVKKPGASVKVSCKASG-YTFT | G-----YYIH | WVRQAPGQGLEWMA | WINPN---SGGTNYAQKFQG | RVTMTRDTSITTAYMELSRLRSDDTAVYYCAR | GYSYGY-------------------NWFDP | WGQGTLVTVSS |
| iPS:4 36338 | 21-225_208E8 | VH1\|1-02/D5\|5-18\|RF3/JH5 | | QVQLVQS-GAEVKKPGASVKVSCKASG-YTFT | G-----YYIH | WVRQAPGQGLEWMA | WINPN---SGGTNYAQKFQG | RVTMTRDTSITTAYMELSRLRSDDTAVYYCAR | GYSYGY-------------------NWFDP | WGQGTLVTVSS |
| iPS:4 36344 | 21-225_208B11 | VH1\|1-02/D5\|5-18\|RF3/JH5 | | QVQLVQS-GAEVKKPGASVKVSCKASG-YTFT | G-----YYIH | WVRQAPGQGLEWMA | WINPN---SGGTNYAQKFQG | RVTMTRDTSITTAYMELSRLRSDDTAVYYCAR | GYSYGY-------------------NWFDP | WGQGTLVTVSS |
| iPS:4 36358 | 21-225_210D11 | VH1\|1-02/D5\|5-18\|RF3/JH5 | | QVQLVQS-GAEVKKPGASVKVSCKASG-YTFT | G-----YYIH | WVRQAPGQGLEWMA | WINPN---SGGTNYAQKFQG | RVTMTRDTSITTAYMELSRLRSDDTAVYYCAR | GYSYGY-------------------NWFDP | WGQGTLVTVSS |
| iPS:4 36408 | 21-225_214H8 | VH1\|1-02/D5\|5-18\|RF3/JH5 | | QVQLVQS-GAEVKKPGASVKVSCKASG-YTFT | G-----HYIH | WVRQAPGQGLEWMG | WINSN---SGGTNYAQKFQG | RVTMTRDTSISTAYMELSRLRSDDTAVYYCAK | DGRYSYG-----------------YDWFDP | WGQGTLVTVSS |
| iPS:4 36424 | 21-225_215H6 | VH1\|1-02/D5\|5-18\|RF3/JH5 | | QVQLVQS-GAEVKKPGASVKVSCKASG-YTFT | G-----HYIH | WVRQAPGQGLEWMG | WINSN---SGGTNYAEKFQG | RVTMTRDTSISTAYMELSRLRSDDTAVYYCAK | DGRYSYG-----------------HDWFDP | WGQGTLVTVSS |
| iPS:4 37092 | 21-225_210B12 | VH1\|1-02/D5\|5-18\|RF3/JH5 | | QVQLVQS-GAEVKKPGASVKISCKASG-FTFT | D-----YYMN | WVRQAPGQGLEWMG | WINPN---SGGTNYAQKFQG | RVTMTRDTSISTAYMELSRLRSDDTAVYYCAR | GYDS-----------------------FAP | WGQGTLVTVSS |
| iPS:4 37134 | 21-225_213A7 | VH1\|1-02/D5\|5-18\|RF3/JH5 | | QVQLVQS-GAEVKKPGASVKISCRASG-FTFT | D-----YYMN | WVRQAPGQGLEWMG | WINPK---NGGTNYAQKFQG | RVTMTRDTSLSRAYMELSRLRSDDTAVYYCAK | GYDS-----------------------FAP | WGQGTLVTVSS |
| iPS:4 37194 | 21-225_226B2 | VH1\|1-02/D5\|5-18\|RF3/JH5 | | QVQLVQS-GAEVKKPGASVKVSCKASG-YTFT | G-----YFMH | WVRQAPGQGLEWMG | WINPN---SGDTNYAQKFQG | RVTMTRDTSLNTAYMELSRLRSDDTAIYYCAR | GTYYGSGS---------------YFNELDS | WGQGTLVTVSS |
| iPS:4 37196 | 21-225_226B7 | VH1\|1-02/D5\|5-18\|RF3/JH5 | | QVQLVQS-GAEVKKPGASVKVSCKASG-YTFT | G-----YYMH | WVRQAPGQGLEWMG | WINPN---SGGTNYAQKFQD | RVTMTRDTSISTAHMELSRLRSDDTAVYYCAR | GYYYGSGS---------------YYNWFDS | WGQGTLVTVSS |
| iPS:4 37200 | 21-225_226A10 | VH1\|1-02/D5\|5- | | QVQLVQS-GAEVKKPGASVKV | G-----YFMH | WVRQAPGQGLEWMG | WINPN---SGDTNYAQK | RVTMTRDTSLSTAYMELSRLRSDDTAIYYCAR | GTYYGSGS---------------YFNELDS | WGQGTLVTVS |

| | | | | | | | | | | |
|---|---|---|---|---|---|---|---|---|---|---|
| iPS:393168 | 21-225_32B11 | VH1\|1-02/D5\|5-18\|RF3/JH5 | | QVQLVQS-GAEVKKPGASVKVSCKASG-YTFT | G-----YYMH | WVRQAPGQGLEWMG | WINPN---SDGTNYAQKFQG | RVTMTRDTSISTAYMELNRLRSDDTAVYYCAR | GFYYGSGS---------------YYNDLDP | WGQGTLVTVSS |
| iPS:393178 | 21-225_34D7 | VH1\|1-02/D5\|5-18\|RF3/JH5 | | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | GYYYGSGS---------------YYNDLDP | WGQGTLVTVSS |
| iPS:398480 | 21-225_17G4 | VH1\|1-02/D5\|5-18\|RF3/JH5 | | QVQLVQS-GAEVKKPGASVKVSCKTSG-YTFT | D-----YYMH | WVRQAPGQGLEWMG | WINPN---SGGTNYEQKFQG | RVTMTRDTSISTAYMELSRLRSDDTAVYYCAS | GYSYGY-------------------NWFDP | WGQGTLVTVSS |
| iPS:398486 | 21-225_19A1 | VH1\|1-02/D5\|5-18\|RF3/JH5 | | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAS | GYSYGY-------------------NWFDP | WGQGTLVTVSS |
| | | Germline | | H_FR1 | H_CDR1 | H_FR2 | H_CDR2 | H_FR3 | H_CDR3 | H_FR4 |
| | VH1\|1-08/D6\|6-13\|RF1/JH6 | | 32422 | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | GYSSSWYY--------------YYYYGMDV | WGQGTTVTVSS |
| iPS:436248 | 21-225_202A3 | VH1\|1-08/D6\|6-13\|RF1/JH6 | | QVQLEQS-GAEVKKPGASVKVSCKASG-YTFT | S-----YDIN | WVRQATGQGLEWLG | WMNPK---RGNTGYAQKFQG | RVTMTRNTSISTAHMELSSLRSEDTAVYYCAR | GRYSREDY--------------YYYYDMDV | WGQGTTVTVSS |
| | | Germline | | H_FR1 | H_CDR1 | H_FR2 | H_CDR2 | H_FR3 | H_CDR3 | H_FR4 |
| | VH3\|3-11/D4\|4-17\|RF2/JH6 | | 32423 | QVQLVES-GGGLVKPGGSLRLSCAASG-FTFS | D-----YYMS | WIRQAPGKGLEWVS | YISSS---GSTIYYADSVKG | RFTISRDNAKNSLYLQMNSLRAEDTAVYYCAR | DYGDYYY-----------------YYGMDV | WGQGTTVTVSS |
| iPS:436270 | 21-225_203F10 | VH3\|3-11/D4\|4-17\|RF2/JH6 | | QVQLVES-GGGLVKPGGSLRLSCAASG-FTFS | D-----YYMS | WIRQAPGKGLEWVL | YISGS---GTTTYYADSVKG | RFTISRDNAKNSLYLQMNSLRAEDTAVYYCAR | DRGG----------------------LDV | WGQGTTVTVSS |
| | | Germline | | H_FR1 | H_CDR1 | H_FR2 | H_CDR2 | H_FR3 | H_CDR3 | H_FR4 |
| | VH3\|3-23/D1\|1-20\|RF1/JH6 | | 32424 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GITGTYY-----------------YYGMDV | WGQGTTVTVSS |
| iPS:436280 | 21-225_204D6 | VH3\|3-23/D1\|1-20\|RF1/JH6 | | EVQVLES-GGGLVQPGGSLRLSCAASG-FTFS | T-----YAMS | WVRQAPGKGLEWVS | AISGS---GGSTYYADSVKG | RFTISRDNSKNTLYLQMDSLRAEDTAVYYCAK | GISGTGSY-----------------YYGVDV | WGQGTTVTVSS |
| | | Germline | | H_FR1 | H_CDR1 | H_FR2 | H_CDR2 | H_FR3 | H_CDR3 | H_FR4 |
| | VH3\|3-33/D7\|7-27\|RF2/JH6 | | 32425 & 32426 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | *LGYYY-------------------YYGMDV | WGQGTTVTVSS |
| iPS:436284 | 21-225_204G8 | VH3\|3-33/D7\|7- | | QVQLVES-GGDVVQPGRSLRL | S-----YGMH | WGRQAPGKGLEWVA | VIWYD---GSNENYVAS | RFTIFRDNSKNTLYLQMNSLRAEDTAVYYCAR | DLGIGY-------------------YGMDV | WGQGTTVTVS |

| | | | | | | | | | | |
|---|---|---|---|---|---|---|---|---|---|---|
| iPS:4 36968 | 21-225_190B10 | VH3\|3-33/D7\|7-27\|RF2/JH6 | | QVQLVES-GGGVVQPGRSLRLSCVASG-FTFS | S-----YGMH | WVRQAPGKGLEWVA | VIWND---GSKKYHVDSVKG | RFTISRDNSKNTLYLQMNSLRAEDTALYYCAR | DLDKRNFPY-------------YYYGMDV | WGQGTTVTVSS |
| iPS:4 37006 | 21-225_192G2 | VH3\|3-33/D7\|7-27\|RF2/JH6 | | QVQLVES-GGGVVQPGRSLRLSCAASG-FTFS | S-----YGMH | WVRQAPGKGLEWVT | VIWND---GSNKYYADSVKG | RFTISRDNSKNTLYLQMNSLRAEDTALYYCAR | DLDKRNFPY-------------YYYGMDV | WGQGTTVTVSS |
| iPS:4 37024 | 21-225_194F11 | VH3\|3-33/D7\|7-27\|RF2/JH6 | | QVQLVES-GGGVVQPGRSLRLSCAASG-FTFS | S-----YGMH | WVRQAPGKGLEWVA | VIWND---GSKKYHVDSVKG | RFTISRDNSKNTLYLQMNSLRAEDTALYYCAR | DLDKRNFPY-------------YYYGMDV | WGQGTTVTVSS |
| iPS:4 37028 | 21-225_194G12 | VH3\|3-33/D7\|7-27\|RF2/JH6 | | QVQLVES-GGGVVQPGRSLRLSCAASG-FTFS | S-----YGMH | WVRQAPGKGLEWVT | VIWND---GSNKYYADSVKG | RFTISRDNSKNTLYLQMNSLRAEDTALYYCAR | DLDKRNFPY-------------YYYGMDV | WGQGTTVTVSS |
| | | Germline | | H_FR1 | H_CDR1 | H_FR2 | H_CDR2 | H_FR3 | H_CDR3 | H_FR4 |
| | VH4\|4-34/D6\|6-19\|RF3/JH3 | | 32427 & 32428 | QVQLQQW-GAGLLKPSETLSLTCAVYG-GSFS | G-----YYWS | WIRQPPGKGLEWIG | EINH----SGSTNYNPSLKS | RVTISVDTSKNQFSLKLSSVTAADTAVYYCAR | V*QWLV-------------------DAFDI | WGQGTMVTVSS |
| iPS:4 36290 | 21-225_205G3 | VH4\|4-34/D6\|6-19\|RF3/JH3 | | QVQLQQW-GAGLLKPSETLSLTCAVFG-GSFS | G-----HYWS | WIRQPPGKGLEWIG | EMYH----FGNTNYNPSLKS | RVTMSVDTSKKQFSLKLSSVTAADTAVYYCAR | VGQWL--------------------AFDI | WGQGTMVTVSS |
| | | Germline | | H_FR1 | H_CDR1 | H_FR2 | H_CDR2 | H_FR3 | H_CDR3 | H_FR4 |
| | VH3\|3-30.3/D4\|4-17\|RF2/JH1 | | 32429 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | DYGDYA-------------------EYFQH | WGQGTLVTVSS |
| iPS:4 36306 | 21-225_201H4 | VH3\|3-30.3/D4\|4-17\|RF2/JH1 | | QVQLVES-GGGVVQPGRSLRLSCAASG-FTFS | S-----YAMH | WVRQAPGKGLEWVA | AIWYD---GSNKYNADSVKG | RFTISRDNSKNTLYMQMNSLRAEDTAVYYCAR | DVGTVG------------------ATYFDC | WGPGTLVTVSS |
| | | Germline | | H_FR1 | H_CDR1 | H_FR2 | H_CDR2 | H_FR3 | H_CDR3 | H_FR4 |
| | VH1\|1-18/D1\|1-1\|RF1/JH6 | | 32430 | QVQLVQS-GAEVKKPGASVKVSCKASG-YTFT | S-----YGIS | WVRQAPGQGLEWMG | WISAY---NGNTNYAQKLQG | RVTMTTDTSTSTAYMELRSLRSDDTAVYYCAR | GTTGTYY----------------YYYGMDV | WGQGTTVTVSS |
| iPS:4 36362 | 21-225_210C12 | VH1\|1-18/D1\|1-1\|RF1/JH6 | | QVQLVQS-GAEVTKPGASVKVSCKASG-YTFT | N-----NGIS | WVRQAPGQGLEWMG | WINAY---NGHTNYAQKFQG | RVTMTTDTSTSTAYMELRSLRSDDTAVYYCAR | DPTVTHY----------------YYYGMDV | WGQGTTVTVSS |
| iPS:4 36374 | 21-225_211C10 | VH1\|1-18/D1\|1-1\|RF1/JH6 | | QVQLVQS-GAEVKKPGASVKVSCKASG-YTFT | R-----HGIS | WVRLAPGQGLEWMG | WISAY---NGLTNYAQKFQG | RVTMTTDTSTSTGYMELRSLRSDDTAVYYCAR | DPTVTHY----------------YYYGMDV | WGQGTTVTVSS |
| | | Germline | | H_FR1 | H_CDR1 | H_FR2 | H_CDR2 | H_FR3 | H_CDR3 | H_FR4 |
| | VH3\|3-33/D5\|5- | | 32431 | QVQLVES- | S-----YGMH | WVRQAPGK | VIWYD--- | RFTISRDNSKNTLYLQMNSL | GYSYGYYY--------- | WGQGT |

| | | | | | | | | | | |
|---|---|---|---|---|---|---|---|---|---|---|
| iPS:4 36366 | 21-225_211A3 | VH3\|3-33/D5\|5-18\|RF3/JH6 | | QVQLVES-GGGVVQPGRSLRLSCAVSG-ITFS | S-----YGMH | WARQAPGKGLEWVA | VIWYD---GSNKNYEDSVKG | RFTISRDNSKNTLYLQMNSLRAEDTAVYYCAR | DGSYGY-------------------DGMDV | WGQGTTVTVSS |
| iPS:4 36388 | 21-225_212H11 | VH3\|3-33/D5\|5-18\|RF3/JH6 | | QVQLVES-GGGVVQPGRSLRLSCAVSG-ITFS | S-----YGMH | WARQAPGKGLEWVA | VIWYD---GSNKNYEDSVKG | RFTISRDNSKNTLYLQMNSLRAEDTAVYYCAR | DGSYGY-------------------DGMDV | WGQGTTVTVSS |
| iPS:4 36396 | 21-225_213E5 | VH3\|3-33/D5\|5-18\|RF3/JH6 | | QVQLVES-GGGVVQPGRSLRLSCAVSG-ITFS | S-----YGMH | WARQAPGKGLEWVA | VIWYD---GSNKNYEDSVKG | RFTISRDNSKNTLYLQMNSLRAEDTAVYYCAR | DGSYGY-------------------DGMDV | WGQGTTVTVSS |
| iPS:4 36454 | 21-225_217B10 | VH3\|3-33/D5\|5-18\|RF3/JH6 | | QVQLVES-GGGVVQPGRSLRLSCAVSG-ITFS | S-----YGMH | WARQAPGKGLEWVA | VIWYD---GSNKNYEDSVKG | RFTISRDNSKNTLYLQMNSLRAEDTAVYYCAR | DGSYGY-------------------DGMDV | WGQGTTVTVSS |
| iPS:4 36668 | 21-225_147B9 | VH3\|3-33/D5\|5-18\|RF3/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DRDYGDPPY-------------YYYYGMDV | WGQGTTVTVSS |
| iPS:4 36688 | 21-225_148C8 | VH3\|3-33/D5\|5-18\|RF3/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DRDYGDPPY-------------YYYYGMDV | WGQGTTVTVSS |
| iPS:4 36706 | 21-225_149A11 | VH3\|3-33/D5\|5-18\|RF3/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DRDYGDPPY-------------YYYYGMDV | WGQGTTVTVSS |
| iPS:4 36760 | 21-225_155E10 | VH3\|3-33/D5\|5-18\|RF3/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DRDYGDPPY-------------YYYYGMDV | WGQGTTVTVSS |
| iPS:4 36966 | 21-225_190C3 | VH3\|3-33/D5\|5-18\|RF3/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAN | WYYYY--------------------YGMDV | WGQGTTVTVSS |
| iPS:4 36976 | 21-225_190D8 | VH3\|3-33/D5\|5-18\|RF3/JH6 | | QVQLVES-GGDVVQPGRSLRLSCEASG-FTFS | S-----YGLH | WVRQAPGKGLEWVA | VIWYD---GSNKYYADSVKG | RFTISRDNSKNTLYLQMNSLRAEDTAVYYCAN | WYYYY--------------------YGMDV | WGQGTTVTVSS |
| iPS:4 37168 | 21-225_218G4 | VH3\|3-33/D5\|5-18\|RF3/JH6 | | QVQLVES-GGGVVQPGRSLRLSCAASG-FTFS | S-----YGLH | WVRQAPGKGLEWVA | VIWYD---GSNKYYADSVKG | RFTISRDNSKNTLYLQMNSLRVEDTAVYYCAN | WYYYY--------------------YGMDV | WGQGTTVTVSS |
| | | Germline | | H_FR1 | H_CDR1 | H_FR2 | H_CDR2 | H_FR3 | H_CDR3 | H_FR4 |
| | VH3\|3-33/D5\|5- | | 32432 | QVQLVES- | S-----YGMH | WVRQAPGK | VIWYD--- | RFTISRDNSKNTLYLQMNSL | RDGYNY----------- | WGQGT |

| | | | | | | | | | | |
|---|---|---|---|---|---|---|---|---|---|---|
| iPS:436368 | 21-225_211G3 | VH3\|3-33/D5\|5-24\|RF3/JH5 | | QVQLVES-GGGVVQPGRSLRLSCAASG-FTFS | N-----YGMH | WVRQAPGKGLEWVA | VIWHD---GSNKYYADSVKG | RFTISRDNSKNTLYLQMNSLRAEDTAVYYCAR | LDYSNY-------------------GWFDP | WGQGTLVTVSS |
| iPS:436426 | 21-225_215C7 | VH3\|3-33/D5\|5-24\|RF3/JH5 | | QVQLVES-GGGVVQPGRSLRLSCAASG-FTFS | Y-----YGMH | WVRQAPGKGLEWVA | VIWHD---GSNKYYADSVKG | RFTISRDNSKNTLYLQMNSLRAEDTAVYYCAR | LDYSNY-------------------GWFDP | WGQGTLVTVSS |
| iPS:436432 | 21-225_215H12 | VH3\|3-33/D5\|5-24\|RF3/JH5 | | QVQLVES-GGGVVQPGRSLRLSCAASG-FTFS | N-----YGMH | WVRQAPGKGLEWVA | VIWHD---GSNKYYADSVKG | RFTISRDNSKNTLYLQMNSLRAEDTAVYYCAR | LDYSNY-------------------GWFDP | WGQGTLVTVSS |
| | | Germline | | H_FR1 | H_CDR1 | H_FR2 | H_CDR2 | H_FR3 | H_CDR3 | H_FR4 |
| | VH1\|1-18/D4\|4-11\|RF2/JH6 | | 32433 | QVQLVQS-GAEVKKPGASVKVSCKASG-YTFT | S-----YGIS | WVRQAPGQGLEWMG | WISAY---NGNTNYAQKLQG | RVTMTTDTSTSTAYMELRSLRSDDTAVYYCAR | DYSNYYY----------------YYYGMDV | WGQGTTVTVSS |
| iPS:436402 | 21-225_213H12 | VH1\|1-18/D4\|4-11\|RF2/JH6 | | QVQLVQS-GAEVKKPGASVKVSCKASG-FTFT | S-----YGIN | WVRQAPGQGLEWMG | WISVH---NGNTDYAQKFQG | RVTMTTDTSTSTAYMELRSLRSDDTAVYYCAR | DYYY----------------------GMDV | WGQGTKVTVSS |
| iPS:436500 | 21-225_222H3 | VH1\|1-18/D4\|4-11\|RF2/JH6 | | QVQLVQS-GAEVKKPGASVKVSCKASG-FTFT | S-----YGIN | WVRQAPGQGLEWMG | WISVY---NGNTNYAQKLQG | RVTMTTDTSTSTAYMELRSLRSDDTAVYYCAR | DYYY----------------------GFDV | WGQGTTVTVSS |
| iPS:436520 | 21-225_223G10 | VH1\|1-18/D4\|4-11\|RF2/JH6 | | QVQLVQS-GAEVKKPGASVKVSCKASG-FTFT | S-----YGIN | WVRQAPGQGLEWMG | WISVY---SGNTNYAQKLQG | RVTMTTDTSTSTAYMELRSLRSDDTAVYYCAR | DYYY----------------------GMDV | WGQGTTVTVSS |
| | | Germline | | H_FR1 | H_CDR1 | H_FR2 | H_CDR2 | H_FR3 | H_CDR3 | H_FR4 |
| | VH3\|3-48/D2\|2-21\|RF1/JH4 | | 32434 & 32435 | EVQLVES-GGGLVQPGGSLRLSCAASG-FTFS | S-----YSMN | WVRQAPGKGLEWVS | YISSS---SSTIYYADSVKG | RFTISRDNAKNSLYLQMNSLRDEDTAVYYCAR | SILWW*L------------------LFYFDY | WGQGTLVTVSS |
| iPS:436436 | 21-225_216F10 | VH3\|3-48/D2\|2-21\|RF1/JH4 | | EVQLVES-GGGLVQPGGSLRLSCAASG-FSFR | S-----YSMN | WVRQAPGKGLEWVS | YITGS---SSTIYYADSVKG | RFTISRDNAKNSLYLQMNSLRDEDTAVYYCAR | SGLA----------------------VEDY | WGQGTLVTVSS |
| | | Germline | | H_FR1 | H_CDR1 | H_FR2 | H_CDR2 | H_FR3 | H_CDR3 | H_FR4 |
| | VH3\|3-48/D6\|6-6\|RF1/JH4 | | 32436 | EVQLVES-GGGLVQPGGSLRLSCAASG-FTFS | S-----YSMN | WVRQAPGKGLEWVS | YISSS---SSTIYYADSVKG | RFTISRDNAKNSLYLQMNSLRDEDTAVYYCAR | EYSSS--------------------SYFDY | WGQGTLVTVSS |
| iPS:436448 | 21-225_217A3 | VH3\|3-48/D6\|6-6\|RF1/JH4 | | EVQLVES-GGGLVQPGGSLRLSCAASG-FTFS | S-----YNMN | WVRQAPGKGLEWVS | YISSS---RNIIYYADSVKG | RFTISRENAKNSLSLQMDSLRDEDTAVYYCAR | DGSYSSG----------------WYWGFDY | WGQGTLVTVSS |
| | | Germline | | H_FR1 | H_CDR1 | H_FR2 | H_CDR2 | H_FR3 | H_CDR3 | H_FR4 |
| | VH4\|4-59/D6\|6- | | 32437 & | QVQLQES- | S-----YYWS | WIRQPPGK | YIYY---- | RVTISVDTSKNQFSLKLSSV | V*QWLVYY---------- | WGQGT |

| | | | | | | | | | | |
|---|---|---|---|---|---|---|---|---|---|---|
| iPS:436472 | 21-225_220E1 | VH4\|4-59/D6\|6-19\|RF3/JH6 | | QVQLQES-GPGLVKPSETLSLTCTVSG-GSIS | T-----YYWS | WIRQPPGKGLEWIG | YIYY----SGTTNYNPSLKS | RVTISVDTSKNQFSLKLSSVTAADTAVYYCAR | DQQWLVRGR------------DNYYYGMDV | WGQGTTVTVSS |
| | | Germline | | H_FR1 | H_CDR1 | H_FR2 | H_CDR2 | H_FR3 | H_CDR3 | H_FR4 |
| | VH3\|3-23/D2\|2-15\|RF3/JH4 | | 32439 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | DIVVVVA-----------------ATYFDY | WGQGTLVTVSS |
| iPS:436504 | 21-225_222H4 | VH3\|3-23/D2\|2-15\|RF3/JH4 | | EVQLLES-GGGLVQPGGSLRLSCAASG-FTFS | N-----FAMS | WVRQAPGKGLEWVS | SIVGS---GGRTYYADSVKG | RFTISRDNSKNTLYLQMNSLRAEDTAVYYCAK | DPYRVAV-----------------AGAFDY | WGQGTLITVSS |
| iPS:436510 | 21-225_222H8 | VH3\|3-23/D2\|2-15\|RF3/JH4 | | EVQLLES-GGGLVQPGGSLRLSCAASG-FTFS | N-----FAMS | WVRQAPGKGLEWVS | SIVGS---GGRTYYADSVKG | RFTISRDNSKNTLYLQMNSLRAEDTAVYYCAK | DPYRVAV-----------------AGAFDY | WGQGTLITVSS |
| | | Germline | | H_FR1 | H_CDR1 | H_FR2 | H_CDR2 | H_FR3 | H_CDR3 | H_FR4 |
| | VH3\|3-23/D3\|3-22\|RF2/JH1 | | 32440 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | YYYDSSGY---------------YYAEYFQH | WGQGTLVTVSS |
| iPS:436526 | 21-225_224A1 | VH3\|3-23/D3\|3-22\|RF2/JH1 | | EVQVLES-GGGLVQPGGSLRLSCAASG-FTFS | S-----YAMS | WVRQAPGKGLEWVS | AISGR---GGSTYYADAVKG | RFTISRDNSKNTLYLQMNSLRAEDTAVYYCAK | GSYDSSG-----------------YYHYLDH | WGQGTLVTVSS |
| | | Germline | | H_FR1 | H_CDR1 | H_FR2 | H_CDR2 | H_FR3 | H_CDR3 | H_FR4 |
| | VH1\|1-02/D4\|4-23\|RF2/JH6 | | 32441 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | DYGGNSYY---------------YYYGMDV | WGQGTTVTVSS |
| iPS:436536 | 21-225_224G1 | VH1\|1-02/D4\|4-23\|RF2/JH6 | | QVQLVQS-GAEVKKPGASVKVSCKASG-YTFT | G-----YYIH | WVRQAPGQGLEWMG | WINPY---SGDTNYAQKFQG | RVTMTRDTSISTAYMELSRLRFDDTAVFYCAR | DWGGYSS----------------YYYGMDV | WGQGTTVTVSS |
| iPS:436548 | 21-225_224A7 | VH1\|1-02/D4\|4-23\|RF2/JH6 | | QVQLVQS-GAEVKKPGASVKVSCKASG-YTFT | G-----YYIH | WVRQAPGQGLEWMG | WINPY---SGDTNYAQKFQG | RVTMTRDTSITTAYMELSRLRFDDTAVFYCAR | DWGGYSS----------------YYYGMDV | WGQGTTVTVSS |
| iPS:436558 | 21-225_224C11 | VH1\|1-02/D4\|4-23\|RF2/JH6 | | QVQLVQS-GAEVKKPGASVKVSCKASG-YTFT | G-----YYIH | WVRQAPGQGLEWMG | WINPY---SGDTNYAQKFQG | RVTMTRDTSISTAYMELRRLRFDDTAVFYCAR | DWGGYSS----------------YYFGMDV | WGQGTTVTVSS |
| iPS:436562 | 21-225_224H11 | VH1\|1-02/D4\|4-23\|RF2/JH6 | | QVQLVQS-GAEVKKPGASVKVSCKASG-YTFT | G-----YYIH | WVRQTPGQGLEWMG | WINPY---SGDTNYAQKFQG | RVTMTRDTSISTAYMELRRLRFDDTAVFYCAR | DWGGYSS----------------YYYGMDV | WGQGTTVTVSS |
| iPS:436572 | 21-225_225G4 | VH1\|1-02/D4\|4- | | QVQLVQS-GAEVKKPGASVKV | G-----YYIH | WVRQAPGQGLEWMG | WINPY---SGDTNYAQK | RVTMTRDTSISTAYMELRRLRFDDTAVFYCAR | DWGGYSS----------------YYYGMDV | WGQGTTVTVS |

| | | | | | | | | | | |
|---|---|---|---|---|---|---|---|---|---|---|
| iPS:436606 | 21-225_226G8 | VH1\|1-02/D4\|4-23\|RF2/JH6 | | QVQLVQS-GAEVKKPGASVKVSCKASG-YTFT | G-----YYIH | WVRQAPGQGLEWMG | WINPY---SGDTKYAQKFQG | RVTMTRDTSISTAYMELSRLRFDDTAVFYCAR | DWGGYSS----------------YYYGMDV | WGQGTTVTVSS |
| iPS:436610 | 21-225_226F9 | VH1\|1-02/D4\|4-23\|RF2/JH6 | | QVQLVQS-GAEVKKPGASVKVSCKASG-YTFT | G-----YYIH | WVRQAPGQGLEWMG | WINPY---SGDTKYAQKFQG | RVTMTRATSISTAYMELSRLRFDDTAVFYCAR | DWGGYSS----------------YYYGMDV | WGQGTTVTVSS |
| iPS:436612 | 21-225_226H9 | VH1\|1-02/D4\|4-23\|RF2/JH6 | | QVQLVQS-GAEVKKPGASVKVSCKASG-YTFT | G-----YYIH | WVRQAPGQGLEWMG | WINPY---SGDTNSAQKFQG | RVTMTRDTSISTAYMELSRLRFDDTAVFYCAR | DWGGYSS----------------YYYGMDV | WGQGTTVTVSS |
| iPS:436614 | 21-225_226F10 | VH1\|1-02/D4\|4-23\|RF2/JH6 | | QVQLVQS-GGEVKKLRASVKVSCKASG-YTFT | G-----YYIH | WVRQAPGQGLEWMG | WINPY---SGDTNYAQKFQG | RVTMTRDTSVSTAYMELSRLRLDDTAVFYCAR | DWGGYSS----------------YYYGMDV | WGQGTPVTVSS |
| iPS:436618 | 21-225_226E11 | VH1\|1-02/D4\|4-23\|RF2/JH6 | | QVQLVQS-GAEVKKPGASVKVSCKASG-YTFT | G-----YYIH | WVRQAPGQGLEWMG | WINPY---SGDTNYAQKFQG | RVTMTRDTSISTAYMELSRLRFDDTAVFYCAR | DWGGYSS----------------YYYGMDV | WGQGTTVTVSS |
| iPS:436624 | 21-225_226H12 | VH1\|1-02/D4\|4-23\|RF2/JH6 | | QVQLVQS-GAEVKKPGASVKVSCKASG-YTFT | G-----YYIH | WVRQAPGQGLEWMG | WINPY---SGDTNYAQKFQG | RVTMTRDTSISTAYMELRRLRFDDTAVFYCAR | DWGGYSS----------------YYYGMDV | WGQGTTVTVSS |
| iPS:436626 | 21-225_227C1 | VH1\|1-02/D4\|4-23\|RF2/JH6 | | QVQLVQS-GAEVKKPGASVKVSCKASG-YTFT | G-----YYTH | WVRQAPGQGLEWMG | WINPY---SGGTNYAQKFQG | RVTMTRDTSISTAYMELSRLRFDDTAVYYCAR | DWGGYSS----------------YYYGMDV | WGQGTTVTVSS |
| iPS:436628 | 21-225_227F2 | VH1\|1-02/D4\|4-23\|RF2/JH6 | | QVHLVQS-GAEVKKPGASVKVSCKASG-YTFT | G-----YYIH | WVRQAPGQGLEWMG | WINPY---SGDTKYAQKFQG | RVTMTRDTSISTAYMELSRLRFDDTAVFYCAR | DWGGYSS----------------YYYGMDV | WGQGTTVTVSS |
| iPS:436640 | 21-225_227A8 | VH1\|1-02/D4\|4-23\|RF2/JH6 | | QVQLVQS-GAEVKKPGASVKVSCKASG-YTFT | G-----YYIH | WVRQAPGQGLEWMG | WINPY---SGDTNYAQKFQG | RVTMTRDTSVSTAYMELSRLRFDDTAVFYCAR | DWGGYSS----------------YYYGMDV | WGQGTTVTVSS |
| | | Germline | | H_FR1 | H_CDR1 | H_FR2 | H_CDR2 | H_FR3 | H_CDR3 | H_FR4 |
| | VH4\|4-39/D4\|4-17\|RF1/JH4 | | 32442 & 32443 & 32444 | QLQLQES-GPGLVKPSETLSLTCTVSG-GSIS | SS---SYYWG | WIRQPPGKGLEWIG | SIYY----SGSIYYNPSLKS | RVTISVDTSKNQFSLKLSSVTAADTAVYYCAR | *LR*L---------------------YFDY | WGQGTLVTVSS |
| iPS:436538 | 21-225_224C3 | VH4\|4-39/D4\|4-17\|RF1/JH4 | | QLQLQES-GPGLVKSSETLSLTCTVSG-GSIS | RS---SYYWG | WIRQPPGKGLEWIG | NIYY----SGSTYYNPSLKS | RVTISVDTSKNQFSLKLSSVTAADTAVYYCAR | QGRDW---------------------GVDY | GGQGTLVTVSS |
| | | Germline | | H_FR1 | H_CDR1 | H_FR2 | H_CDR2 | H_FR3 | H_CDR3 | H_FR4 |
| | VH3\|3-23/D4\|4- | | 32445 | EVQLLES- | S------YAMS | WVRQAPGK | AISGS--- | RFTISRDNSKNTLYLQMNSL | DYSNY----------- | WGQGT |

| | | | | | | | | | | |
|---|---|---|---|---|---|---|---|---|---|---|
| iPS:436546 | 21-225_224D6 | VH3\|3-23/D4\|4-11\|RF2/JH5 | | EVQVLES-GGGLVQPGGSLRLSCAASG-STFS | S-----DAMS | WVRQAPGKGLEWVS | AISGS---GDNTFYADSVKG | RFTISRDNSKNTLYLQMNSLRAEDTAVYYCAK | VYSAYD-----------------SHWFDP | WGQGTLVTVSS |
| | | Germline | | H_FR1 | H_CDR1 | H_FR2 | H_CDR2 | H_FR3 | H_CDR3 | H_FR4 |
| | VH1\|1-46/D6\|6-6\|RF2/JH6 | | 32446 | QVQLVQS-GAEVKKPGASVKVSCKASG-YTFT | S-----YYMH | WVRQAPGQGLEWMG | IINPS---GGSTSYAQKFQG | RVTMTRDTSTSTVYMELSSLRSEDTAVYYCAR | SIAARYY----------------YYYGMDV | WGQGTTVTVSS |
| iPS:436568 | 21-225_225B3 | VH1\|1-46/D6\|6-6\|RF2/JH6 | | QVQLVQS-GAEVKKPGASVKVSCKASG-YTFT | S-----YYMH | WVRQAPGQGLEWMG | IINPS---GGSTSYAQKFQG | RVTMTRDTSTSTVYMELSSLRSADTAVYYCAR | DLAARSYY---------------YYFGMDV | WGQGATVTVSS |
| | | Germline | | H_FR1 | H_CDR1 | H_FR2 | H_CDR2 | H_FR3 | H_CDR3 | H_FR4 |
| | VH4\|4-30.1/D4\|4-17\|RF2/JH6 | | 32447 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | DYGDYYY----------------YYYGMDV | WGQGTTVTVSS |
| iPS:436580 | 21-225_225E7 | VH4\|4-30.1/D4\|4-17\|RF2/JH6 | | QVQLQES-GPGLVKPSQTLSLTCTVSG-NSIS | SG---HYYWS | WIRQHPGKGLEWIG | FIYY----TGSTYYNPSLKS | RVTISVDTSKNQFSLKLSSVTAADTAVYYCAR | EAGDYG------------------YYGMDV | WGQGTTVTVSS |
| iPS:436926 | 21-225_78D10 | VH4\|4-30.1/D4\|4-17\|RF2/JH6 | | QVQLQES-GPGLVKPSQTLSLTCTVSG-GSIS | SG---GYYWS | WIRQHPGKGLEWIG | YIYY----IGSVYYNPSLKS | RVTISVDTSKNQFSLKLSSVTAADTAVYYCAR | DAPDF---------------------GMDV | WGQGTTVTVSS |
| | | Germline | | H_FR1 | H_CDR1 | H_FR2 | H_CDR2 | H_FR3 | H_CDR3 | H_FR4 |
| | VH3\|3-33/D4\|4-17\|RF2/JH1 | | 32448 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DYGDYA-----------------EYFQH | WGQGTLVTVSS |
| iPS:436592 | 21-225_226B1 | VH3\|3-33/D4\|4-17\|RF2/JH1 | | QVQLVES-GGGVVQPGRSLRLSCAASG-FTFS | T-----YGMH | WVRQAPGKGLEWVA | IIWYD---GGYKYYADSVKG | RFTISRDNSKNTLFLQMNSLRAEDTAVYYCAR | DHYDFW-----------------SGYLTH | WGQGTLVTVSS |
| | | Germline | | H_FR1 | H_CDR1 | H_FR2 | H_CDR2 | H_FR3 | H_CDR3 | H_FR4 |
| | VH3\|3-23/D7\|7-27\|RF1/JH6 | | 32449 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | LTGYYY-----------------YYGMDV | WGQGTTVTVSS |
| iPS:436652 | 21-225_146B11 | VH3\|3-23/D7\|7-27\|RF1/JH6 | | EVQLLES-GGGLVQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT-----------------DYGMDV | WGQGTTVTVSS |
| iPS:436654 | 21-225_146C11 | VH3\|3-23/D7\|7-27\|RF1/JH6 | | EVQLLES-GGGLVQPGGSLRLSCAASG-ITFS | S-----YAMS | WVRQAPGKGLEWVS | VISGG---GSSTYYADSVKG | RFTISRDNSKNTLYLQMNSLRAEDTAVYYCAK | WRGNPT-----------------DYGMDV | WGQGTTVTVSS |
| iPS:436658 | 21-225_146A2 | VH3\|3-23/D7\|7- | | EVQLLES-GGGLVQPGGSLRL | S-----YAMS | WVRQAPGKGLEWVS | VISGG---GSSTYYADS | RFTISRDNSKNTLHLQMNSLRAEDTAVYYCAK | WRGNPT-----------------DYGMDV | WGQGTTVTVS |

| | | | | | | | | | | |
|---|---|---|---|---|---|---|---|---|---|---|
| iPS:436664 | 21-225_147E7 | VH3\|3-23/D7\|7-27\|RF1/JH6 | | EVQLLES-GGGLVQPGGSLRLSCAASG-FTFS | S-----YAMS | WVRQAPGKGLEWVS | VISGG---GSSTYYADSVKG | RFTLSRDNSKNTLYLQMNSLRAEDTAVFYCAK | WRGNPT------------------DYGMDV | WGQGTTVTVSS |
| iPS:436676 | 21-225_147E11 | VH3\|3-23/D7\|7-27\|RF1/JH6 | | EVQLLES-GGGLVQPGGSLRLSCAASG-FTFS | N-----YVMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:436678 | 21-225_147B12 | VH3\|3-23/D7\|7-27\|RF1/JH6 | | EVQLLES-GGGLVQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:436686 | 21-225_148G6 | VH3\|3-23/D7\|7-27\|RF1/JH6 | | EVQLLES-GGGLVQPGGSLRLSCAASG-FTFS | S-----YAMS | WVRQAPGKGLEWVS | VISGG---GSSTYYADSVKG | RFTISRDNSKNMLHLQMNSLRAEDTAVYYCAK | WRGNPT------------------DYGMDV | WGQGTTVTVSS |
| iPS:436694 | 21-225_148G11 | VH3\|3-23/D7\|7-27\|RF1/JH6 | | EVQLLES-GGGLVQPGGSLRLSCAASG-FTFS | S-----YPMS | WVRQAPGKGLEWVS | VISGG---GSSAYYADSVKG | RFTISRDNSKNTLYLQMNSLRAEDTAVYYCAK | WRGNPT------------------DYGMDV | WGQGTTVTVSS |
| iPS:436700 | 21-225_149C7 | VH3\|3-23/D7\|7-27\|RF1/JH6 | | EVQLLES-GGGLVQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:436704 | 21-225_149C10 | VH3\|3-23/D7\|7-27\|RF1/JH6 | | EVQLLES-GGGLVQPGGSLRFSCAASG-FTFS | S-----HAMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:436710 | 21-225_150F6 | VH3\|3-23/D7\|7-27\|RF1/JH6 | | EVQLLES-GGGLVQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:436714 | 21-225_150H11 | VH3\|3-23/D7\|7-27\|RF1/JH6 | | EVQLLES-GGGLVQPGGSLRLSCAASG-FTFS | T-----YAMS | WVRQAPGKGLEWVS | IISGG---GSSTYYADSVKG | RFTISRDNSKNTLYLQMNSLRAEDTAVYYCAK | WRGNPT------------------DYGMDV | WGQGTTVTVSS |
| iPS:436718 | 21-225_151H5 | VH3\|3-23/D7\|7-27\|RF1/JH6 | | EVQLLES-GGGLVQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:436722 | 21-225_151H7 | VH3\|3-23/D7\|7-27\|RF1/JH6 | | EVQLLES-GGGLVQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:436724 | 21-225_151B9 | VH3\|3-23/D7\|7- | | EVQLLES-GGGLVQPGGSLRL | S-----YAMS | WVRQAPGKGLEWVS | VISGG---GSSTYYADS | RFTISRDNSKNTLYLQMNSLRAEDTAVYYCAK | WRGNPT------------------DYGMDV | WGQGTTVTVS |

| | | | | | | | | | | |
|---|---|---|---|---|---|---|---|---|---|---|
| iPS:4 36728 | 21-225_152G6 | VH3\|3-23/D7\|7-27\|RF1/JH6 | | EVQLLES-GGGLVQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:4 36730 | 21-225_152D7 | VH3\|3-23/D7\|7-27\|RF1/JH6 | | EVQLLES-GGGLVQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT------------------DSGMDV | WGQGTTVTVSS |
| iPS:4 36742 | 21-225_154C4 | VH3\|3-23/D7\|7-27\|RF1/JH6 | | EVQLLES-GGGLIQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:4 36746 | 21-225_154E10 | VH3\|3-23/D7\|7-27\|RF1/JH6 | | EVQLLES-GGGLVQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:4 36758 | 21-225_155C10 | VH3\|3-23/D7\|7-27\|RF1/JH6 | | EVQLLES-GGGLVQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:4 36938 | 21-225_146A3 | VH3\|3-23/D7\|7-27\|RF1/JH6 | | EVQLLES-GGGLVQPGGSLRLSCAASG-FTFS | S-----YAMS | WVRQAPGKGLEWVS | VISGG---GTTTYYADSVKG | RFTISRDNSKNTLYLQMNSLRAEDTAVYYCAK | WRGNPT------------------DYGMDV | WGQGTTVTVSS |
| iPS:4 37250 | 21-225_148C6 | VH3\|3-23/D7\|7-27\|RF1/JH6 | | EVQLLES-GGGLVQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:4 37252 | 21-225_148H11 | VH3\|3-23/D7\|7-27\|RF1/JH6 | | EVQLLES-GGGLVQPGGSLRLSCAASG-FTFS | S-----YAMS | WVRQAPGKGLEWVS | VISGG---GSSTYYADSVKG | RFTISRDNSKNTLYLQMNSLRAEDTAVYYCAK | WRGNPT------------------DYGMDV | WGQGTTVTVSS |
| iPS:4 37282 | 21-225_207C9 | VH3\|3-23/D7\|7-27\|RF1/JH6 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | AGGTTGSY---------------YYNGMDV | WGQGTTVTVSS |
| | | Germline | | H_FR1 | H_CDR1 | H_FR2 | H_CDR2 | H_FR3 | H_CDR3 | H_FR4 |
| | VH3\|3-48/D4\|4-11\|RF2/JH6 | | 32450 | EVQLVES-GGGLVQPGGSLRLSCAASG-FTFS | S-----YSMN | WVRQAPGKGLEWVS | YISSS---SSIIYYADSVKG | RFTISRDNAKNSLYLQMNSLRDEDTAVYYCAR | DYSNYYY---------------YYYGMDV | WGQGTTVTVSS |
| iPS:4 36660 | 21-225_146D8 | VH3\|3-48/D4\|4-11\|RF2/JH6 | | EVQLVES-GGGLVQPGGSLRLSCAASG-FTFS | N-----YNMN | WVRQAPGKGLEWVS | YISRS---SNTKYYVDSVKG | RFTISRDNAKNSLYLQMNSLRDEDTAVYYCAR | DRSGSYGY--------------FYYYGLDV | WGQGTTVTVSS |
| iPS:4 36682 | 21-225_146A8 | VH3\|3-48/D4\|4- | | EVKLVES-GGGLVQPGESLRL | N-----YNMN | WVRQAPGKGLEWVS | YISRS---SNTKYYADS | RFTISRDNAKNSLYLQMNSLRDEDTAVYYCAR | DRSGSYGY--------------FYYYGLDV | WGQGTTVTVS |

| | | | | | | | | | | |
|---|---|---|---|---|---|---|---|---|---|---|
| iPS:436684 | 21-225_146B6 | VH3\|3-48/D4\|4-11\|RF2/JH6 | | EVQLVES-GGGLVQPGGSLRLSCAASG-FTFS | S-----YNMN | WVRQAPGKGLEWVS | YISRS---SNTKHYADSVKG | RFTISRDNAKNSLYLQMDSLRDEDTAVYYCAR | DRSGSYGY--------------FYYYGLDV | WGQGTTVTVSS |
| iPS:436696 | 21-225_149A1 | VH3\|3-48/D4\|4-11\|RF2/JH6 | | EVQLVES-GGGLVQPGGSLRLSCAASG-FTFS | S-----YNMN | WVRQAPGKGLEWVS | YISRS---SNTKHYADSVKG | RFTISRDNAKNSLYLQMNSLRDEDTAVYYCAR | DRSGSYGY--------------FYYYGLDV | WGQGTTVTVSS |
| iPS:436712 | 21-225_150F9 | VH3\|3-48/D4\|4-11\|RF2/JH6 | | EMQLVES-GGGLVQPGGSLRLSCAASG-FTFS | S-----YNMN | WVRQVPGKGLEWVS | YISRS---SNTKHYADSVKG | RFTISRDNAKNSLYLQMNSLRDEDTAVYYCAR | DRSGSYGY--------------FYYYGLDV | WGQGTTVTVSS |
| iPS:436762 | 21-225_156H2 | VH3\|3-48/D4\|4-11\|RF2/JH6 | | EVQLVES-GGGLIQPGGSLRLSCAASG-FTFS | N-----YNMN | WVRQAPGKGLEWVS | YISRS---SNTKYYADSVKG | RFTISRDNAKNSLYLQMNSLRDEDTAVYYCAR | DRSGSYGY--------------FYYYGMDV | WGQGTTVTVSS |
| iPS:436820 | 21-225_179D10 | VH3\|3-48/D4\|4-11\|RF2/JH6 | | EVQLVES-GGGLVQPGGSLRFSCAASG-FTFS | S-----YSMN | WVRQAPGKGLEWVA | YISSS---GSTTYYADSVQG | RFTISRDNAKNSLYLQMNSLRDEDTAVYRCAR | DSRKGF------------------YYGLDV | WGQGITVTVSS |
| iPS:437262 | 21-225_170E4 | VH3\|3-48/D4\|4-11\|RF2/JH6 | | EVQLVES-GGGSVQPGGSLRLSCAASG-FTFS | S-----YSMN | WVRQAPGKGLEWVS | YISSS---GSTKYYADSVEG | RFTISRDNAKNSLDLQMNSLRDEDTAVYRCAR | DSRKGF------------------YYGLDV | WGQGTTVTVSS |
| | | Germline | | H_FR1 | H_CDR1 | H_FR2 | H_CDR2 | H_FR3 | H_CDR3 | H_FR4 |
| | VH1\|1-08/D5\|5-12\|RF1/JH6 | | 32451 | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | VDIVATIY--------------YYYYGMDV | WGQGTTVTVSS |
| iPS:436662 | 21-225_147D7 | VH1\|1-08/D5\|5-12\|RF1/JH6 | | QVQLVQS-GAEVKKPGASVKVSCKASG-YTFT | S-----YDIN | WVRQATGQGLEWMG | WMNPN---SGNTGYAQKFQG | RVTMTRNTSISTAYMELRSLRSEDTAVYYCAR | ADIVLVPAAI----------PYNYYFAMDV | WGQGTTVTVSS |
| | | Germline | | H_FR1 | H_CDR1 | H_FR2 | H_CDR2 | H_FR3 | H_CDR3 | H_FR4 |
| | VH1\|1-02/D3\|3-3\|RF2/JH6 | | 32452 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | YYDFWSGYYT-----------YYYYGMDV | WGQGTTVTVSS |
| iPS:436666 | 21-225_147B8 | VH1\|1-02/D3\|3-3\|RF2/JH6 | | QVQLVQS-GAEVKKPGASVKVSCKASG-YTFT | D-----YYLH | WVRQAPGQGLEWMG | WINPN---SGDTNYAQKFQG | RVTMTRDTSISTAYMELSRLRSDDTAVYYCAR | DRDSGSGSYP-----------YYYYGMDV | WGQGTTVTVSS |
| | | Germline | | H_FR1 | H_CDR1 | H_FR2 | H_CDR2 | H_FR3 | H_CDR3 | H_FR4 |
| | VH3\|3-33/D3\|3-3\|RF2/JH6 | | 32453 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YYDFWSGYYT-----------YYYYGMDV | WGQGTTVTVSS |
| iPS:436672 | 21-225_147F9 | VH3\|3-33/D3\|3- | | QVQLVES-GGGVVQPGRSLRL | T-----YGMH | WVRQAPGKGLEWVA | VIWYG---GSDKDYADS | RFTISRDISKNTLYLQMNSLRAEDTAVYYCAR | DRDYCSGGTC----------PYYYYYGMDV | WGQGTTVTVS |

| | | | | | | | | | | |
|---|---|---|---|---|---|---|---|---|---|---|
| iPS:4 36674 | 21-225_147G9 | VH3\|3-33/D3\|3-3\|RF2/JH6 | | QVQLVES-GGGVVQPGRSLRLSCAASG-FTFS | T-----YGMH | WVRQAPGKGLEWVA | VIWYG---GSDKDYADSVKG | RFTISRDISKNTLYLQMNSLRAEDTAVYYCAR | DRDYCSGGSC----------PYYYYYGMDV | WGQGTTVTVSS |
| iPS:4 36690 | 21-225_148A9 | VH3\|3-33/D3\|3-3\|RF2/JH6 | | QVQLVES-GGGVVQPGRSLRLSCAASG-FTFS | T-----YGMH | WVRQAPGKGLEWVA | VIWYG---GSDKDYADSVKG | RFTISRDISKNTLYLQMNSLRAEDTAVYYCAR | DRDYCSGGTC----------PYYYYYGMDV | WGQGTTVTVSS |
| iPS:4 36708 | 21-225_150D3 | VH3\|3-33/D3\|3-3\|RF2/JH6 | | QVQLVES-GGGVVQPGRSLRLSCAASG-FTFS | T-----YGMH | WVRQAPGKGLEWVA | VIWYG---GSNKDYADSVKG | RFTISRDNSKNTLYLQMNSLRAEDTAVYYCAR | DRDYCSGGTC----------PYYYYYGMDV | WGQGTTVTVSS |
| iPS:4 36716 | 21-225_151F3 | VH3\|3-33/D3\|3-3\|RF2/JH6 | | QVQLVES-GGGVVQPGRSLRLSCAASG-FTFS | T-----YGMH | WVRQAPGKGLEWVA | VIWYG---GSNTDYADSVKG | RFTISRDISKNTLYLQMNSLRAEDTAVYYCAR | DRDYCSGTSC----------PYYYYYGMDV | WGQGTTVTVSS |
| iPS:4 36738 | 21-225_153D9 | VH3\|3-33/D3\|3-3\|RF2/JH6 | | QVQLVES-GGGVVQPGRSLRLSCAASG-FTVS | T-----YGMH | WVRQAPGKGLEWVA | VIWYG---GSNKDYADSVKG | RFTISRDISKNTLYLQMNSLRAEDTAVYYCAR | DRDYCSGGSC----------PYYYYYGMDV | WGQGTTVTVSS |
| iPS:4 36740 | 21-225_154C3 | VH3\|3-33/D3\|3-3\|RF2/JH6 | | QVQLVES-GGGVVQPGRSLRLSCTASG-FTFS | T-----YGMH | WVRQAPGKGLEWVA | VVWYG---GNNKDYADSVKG | RFTISRDISKNTLYLQMNSLRAEDTAVYYCAR | DRDYCSGGSC----------PYYYYYGMDV | WGQGTTVTVSS |
| iPS:4 36748 | 21-225_154D11 | VH3\|3-33/D3\|3-3\|RF2/JH6 | | QVQLVES-GGGVVQPGRSLRLSCAASG-FNVS | T-----YGMH | WVRQAPGKGLEWVA | VIWYG---GSNKDYADSVKG | RFTISRDSSKNTLYLQMNSLRAEDTAVYYCAR | DRDYCSGGSC----------PYYYYYGMDV | WGQGTTVTVSS |
| iPS:4 36764 | 21-225_158E9 | VH3\|3-33/D3\|3-3\|RF2/JH6 | | QVQLVES-GGGVVQPGRSLRLSCAASG-FTFS | S-----YGMH | WVRQAPGKGLEWVA | VIWYD---GSSKYYADSVKG | RFTISRDNSKNTLYLQMNSLRAEDTAVYYCAR | DRVFCSGTSC----------PYYYYYGMDV | WGQGTTVTVSS |
| iPS:4 36774 | 21-225_161E10 | VH3\|3-33/D3\|3-3\|RF2/JH6 | | QVQLVES-GGGVVQPGRSLRLSCAASG-FTFS | S-----YGMH | WVRQAPGKGLEWVA | VIWYD---GSNKYYVDSVKG | RFTISRDNSKNTLYLQMNSLRAEDTAVYYCAR | DRVFCSGTSC----------PYYYYYGMDV | WGQGTTVTVSS |
| iPS:4 36916 | 21-225_74A9 | VH3\|3-33/D3\|3-3\|RF2/JH6 | | QVQLVES-GGGVVQPGRSLRLSCAASG-FTFS | S-----YGMH | WVRQAPGKGLEWVA | VIWYD---GNNKSYADSVKG | RFTISRDISKNTLFLQMNSLRADDTAVYYCAR | DRDYCSGTSC----------PYYYYYGMDV | WGQGTTVTVSS |
| iPS:4 36940 | 21-225_146B8 | VH3\|3-33/D3\|3-3\|RF2/JH6 | | QVQLVES-GGGVVQPGRSLRLSCAASG-FTFS | T-----YGMH | WVRQAPGKGLEWVA | VVWYG---GNDKDFADSVTG | RFTISRDISKNTLYLQMNSLRAEDTAVYYCAR | DRDYCSGGSC----------PYYYYYGMDV | WGQGTTVTVSS |
| iPS:4 37258 | 21-225_153F9 | VH3\|3-33/D3\|3- | | QVHLVES-GGGVVQPGRSLRL | T-----YGMH | WVRQGPGKGLEWVA | VIWYG---GSDTDYADS | RFTISRDISKNTLYLQMNSLRAEDTAVYYCAR | DRDYCSGGNC----------PYYYYYGMDV | WGQGTTVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH4\|4-30.1/D7\|7-27\|RF3/JH5 | | 32454 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | NWGN--------------------WFDP | WGQGTLVTVSS |
| iPS:436680 | 21-225_147H12 | VH4\|4-30.1/D7\|7-27\|RF3/JH5 | | QVQLQES-GPGLVNPSQTLSLTCAVSG-GSIS | SG---YYHWS | WIRQHPGKGLEWIG | YIYY----SGSTYYNPSLKS | RVTISVDTSKNQFSLKLTSVTAADTAVYYCAR | DWGGYDS-----------------SGWFDP | WGQGTLVTVSS |
| iPS:436750 | 21-225_154G12 | VH4\|4-30.1/D7\|7-27\|RF3/JH5 | | QVQLQES-GPGLVNPSQTLSLTCGVSG-GSIS | SG---YYYWS | WIRQHPGKGLEWIG | YIYY----SGSTYYNPSLKS | RVSISLDTPKNQFSLKLTSVTAADTAVYYCAR | DWGGYDS-----------------SGWFDP | WGQGTLVTVSS |
| | | Germline | | H_FR1 | H_CDR1 | H_FR2 | H_CDR2 | H_FR3 | H_CDR3 | H_FR4 |
| | VH3\|3-07/D6\|6-13\|RF1/JH4 | | 32455 | EVQLVES-GGGLVQPGGSLRLSCAASG-FTFS | S-----YWMS | WVRQAPGKGLEWVA | NIKQD---GSEKYYVDSVKG | RFTISRDNAKNSLYLQMNSLRAEDTAVYYCAR | GYSSSW-------------------YYFDY | WGQGTLVTVSS |
| iPS:436698 | 21-225_149B5 | VH3\|3-07/D6\|6-13\|RF1/JH4 | | EVQLVES-GGGLVQPGGSLRLSCAASG-FTFS | G-----YWMN | WVRQAPGKGLEWVA | NIKQD---GSEKYYVDSVKG | RFTISRDNAKNSLYLQMNSLRAEDTAVYYCAR | GMYSSG------------------WYVFDY | WGQGTLVTVSS |
| | | Germline | | H_FR1 | H_CDR1 | H_FR2 | H_CDR2 | H_FR3 | H_CDR3 | H_FR4 |
| | VH3\|3-21/D6\|6-19\|RF2/JH5 | | 32456 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | GIAVAG-------------------NWFDP | WGQGTLVTVSS |
| iPS:436702 | 21-225_149E8 | VH3\|3-21/D6\|6-19\|RF2/JH5 | | EVQLVES-GGGLVKPGGSLRLSCAASG-FTFS | S-----YSMN | WVRRAPGKGLEWVS | AISST---GSYIYYADSVKG | RFTISRDNAKNSLYLQMNSLRAEDTAVYYCAR | TAVAGT-------------------GWFDP | WGQGTLVTVSS |
| | | Germline | | H_FR1 | H_CDR1 | H_FR2 | H_CDR2 | H_FR3 | H_CDR3 | H_FR4 |
| | VH5\|5-51/D3\|3-22\|RF2/JH6 | | 32457 | EVQLVQS-GAEVKKPGESLKISCKGSG-YSFT | S-----YWIG | WVRQMPGKGLEWMG | IIYPG---DSDTRYSPSFQG | QVTISADKSISTAYLQWSSLKASDTAMYYCAR | YYYDSSGYYY-----------YYYYGMDV | WGQGTTVTVSS |
| iPS:436752 | 21-225_155H1 | VH5\|5-51/D3\|3-22\|RF2/JH6 | | EVQLVQS-GAEVKKPGESLKISCKGSG-YSFT | S-----YWIG | WVRQMPGKGLEWMG | LIYPG---ASDTRYSPSFQG | QVTISADKSISTAYLQWSSLKASDTAMYYCAR | QAIASRGR--------------YYYYGMDV | WGQGTTVTVSS |
| | | Germline | | H_FR1 | H_CDR1 | H_FR2 | H_CDR2 | H_FR3 | H_CDR3 | H_FR4 |
| | VH3\|3-33/D6\|6-19\|RF1/JH4 | | 32458 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | GYSSGW-------------------YYFDY | WGQGTLVTVSS |
| iPS:436772 | 21-225_161H3 | VH3\|3-33/D6\|6-19\|RF1/JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | VGYSGG------------------WYIFDY | WGQGTLVTVSS |
| | | Germline | | H_FR1 | H_CDR1 | H_FR2 | H_CDR2 | H_FR3 | H_CDR3 | H_FR4 |

| | | | | | | | | | | | |
|---|---|---|---|---|---|---|---|---|---|---|---|
| | VH4\|4-30.1/D2\|2-2\|RF2/JH6 | | 32459 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | GYCSSTSCYT-----------YYYYYGMDV | WGQGTTVTVSS |
| iPS:436776 | 21-225_161F12 | VH4\|4-30.1/D2\|2-2\|RF2/JH6 | | QVQLQES-GPGLVKPSQTLSLTCTVSG-GSIS | SG---GYYWS | WIRQHPGKGLEWIG | YIYY----SGSPYYNPSLKS | RITISIDTSKNQFSLKLNSVTAADTAVYYCAR | SNCSSANCYT----------VGFYYYGLDV | WGRGTTVTVSS |
| iPS:436780 | 21-225_165H3 | VH4\|4-30.1/D2\|2-2\|RF2/JH6 | | QVQLQES-GPGLVKPSQTLSLTCTVSG-GSIS | SG---GYYWS | WIRQHPGKGLEWIG | YIYY----SGSPYYNPSLKS | RITISIDTSKNQFSLKLNSVTAADTAVYYCAR | SNCSSANCYT----------VGFYYYGMDV | WGQGTTVTVSS |
| | | Germline | | **H_FR1** | **H_CDR1** | **H_FR2** | **H_CDR2** | **H_FR3** | **H_CDR3** | **H_FR4** |
| | VH3\|3-33/D5\|5-24\|RF3/JH6 | | 32460 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RDGYNYYY---------------YYYGMDV | WGQGTTVTVSS |
| iPS:436784 | 21-225_169C1 | VH3\|3-33/D5\|5-24\|RF3/JH6 | | QVQLVES-GGGVVQPGRSLRLSCAASG-FTLS | S-----NGMH | WVRQAPGKGLEWVA | VIWYD---GSNKYYADSVKG | RFTISRDTSKNTLYLQMNSLRAEDTAVYYCAR | DQYNRNDGPP----------AYYYYYGLDV | WGQGTTVTVSS |
| iPS:436786 | 21-225_169A6 | VH3\|3-33/D5\|5-24\|RF3/JH6 | | QVQLVES-GGGVVQPGRSLRLSCAASG-FTLS | S-----NGMH | WVRQAPGKGLEWVA | VIWYD---GSNKYYADSVKG | RFTISRDNSKNTLSLQMNSLRAEDTAVYYCAR | DQYNRNDGPP----------AYYYYYGMDV | WGQGTTVTVSS |
| iPS:436796 | 21-225_170A5 | VH3\|3-33/D5\|5-24\|RF3/JH6 | | QVQLVES-GGGVVQPGRSLRLSCAASG-LTFS | N-----CGMH | WVRQAPGKGLEWVA | IIWYD---GSNKYYADSVKG | RFTISRDNSKNTLDLQMNSLRAEDTAVYYCAR | DQYNRNDGPP----------AYYYYYGLDV | WGQGTTVTVSS |
| iPS:436812 | 21-225_175C6 | VH3\|3-33/D5\|5-24\|RF3/JH6 | | QVQLVES-GGGVVQPGRSLRLSCAASG-LTFS | N-----CGMH | WVRQAPGKGLEWVA | IIWYD---GSNKYYADSVKG | RFTVSRDNSKNTLDLQMNSLRAEDTAVYYCAR | DQYNRNDGPP----------AYYYYYGLDV | WGQGTTVTVSS |
| | | Germline | | **H_FR1** | **H_CDR1** | **H_FR2** | **H_CDR2** | **H_FR3** | **H_CDR3** | **H_FR4** |
| | VH3\|3-48/D5\|5-18\|RF1/JH6 | | 32461 | EVQLVES-GGGLVQPGGSLRLSCAASG-FTFS | S-----YSMN | WVRQAPGKGLEWVS | YISSS---SSTIYYADSVKG | RFTISRDNAKNSLYLQMNSLRDEDTAVYYCAR | VDTAMVYY---------------YYYGMDV | WGQGTTVTVSS |
| iPS:436788 | 21-225_169B7 | VH3\|3-48/D5\|5-18\|RF1/JH6 | | EVQLVES-GGGLVQPGGSLRLSCAASG-FTFS | S-----YSLN | WVRQAPGKGLEWVS | YIGSS---GSIIFYADSVKG | RFTISRDNAKNSLYLQMNSLRDEDTAVYYCAR | GDTAGVT----------------YYYGMDV | WGQGTTVTVSS |
| iPS:436798 | 21-225_171F5 | VH3\|3-48/D5\|5-18\|RF1/JH6 | | EVQLVES-GGGLVQPGGSLRLSCAASG-FTFS | S-----YSLN | WVRQAPGKGLEWVS | YIGSS---GSIIFYADSVKG | RFTISRDNAKNSLYLQMNSLRDEDTAVYYCAR | GDTAGVT----------------YYYGMDV | WGQGTTVTVSS |
| iPS:436864 | 21-225_58G11 | VH3\|3-48/D5\|5-18\|RF1/J | | EVQLVES-GGGLVQPGGSLRLSCAASG-FTFS | S-----YSMN | WVRQAPGKGLEWIS | YISTS---SSTIFYADSVKG | RFTISRDSAKNSLYLQMNSLRDEDTAVYYCAR | GDTAMVL----------------YYYGMDV | WGQGTTVTVSS |

| | | | | | | | | | | |
|---|---|---|---|---|---|---|---|---|---|---|
| iPS:4 36866 | 21-225_59F2 | VH3\|3-48/D5\|5-18\|RF1/JH6 | | EVQLVES-GGGLVQPGGSLRLSCGASG-FTFS | S-----YSMN | WVRQAPGKGLEWVS | YISGS---SNIIYYTDSVKG | RFTISRDNAKNSLYLQMNSLRDEDTAVYYCAR | ADTPMVL----------------YFYGMDV | WGQGTTVTVSS |
| iPS:4 36872 | 21-225_60D2 | VH3\|3-48/D5\|5-18\|RF1/JH6 | | EVQLVES-GGGLVQPGGSLRLSCGASG-FTFS | S-----YSMN | WVRQAPGKGLEWVS | YISES---SNIIYYTDSVKG | RFTISRDNAMNSLYLQMNSLRDEDTAVYYCAR | ADTPMVL----------------YFYGMDV | WGQGTTVTVSS |
| | | Germline | | H_FR1 | H_CDR1 | H_FR2 | H_CDR2 | H_FR3 | H_CDR3 | H_FR4 |
| | VH3\|3-33/D5\|5-24\|RF1/JH6 | | 32462 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | VEMATIYY----------------YYYGMDV | WGQGTTVTVSS |
| iPS:4 36790 | 21-225_169G11 | VH3\|3-33/D5\|5-24\|RF1/JH6 | | QVQLVES-GGGVVQPGRSLRLSCAASG-FTFS | S-----YGMH | WVRQAPGKGLEWVA | IIWYD---GSNKYYADSVKG | RFTISRDNSKNTLYLQMNSLRAEDTGVYYCAR | EGATYYHGSGS--------YYPATNYGMDV | WGQGTTVTVSS |
| | | Germline | | H_FR1 | H_CDR1 | H_FR2 | H_CDR2 | H_FR3 | H_CDR3 | H_FR4 |
| | VH3\|3-33/D3\|3-10\|RF1/JH6 | | 32463 & 32464 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | VLLWFGELL*-----------YYYYYGMDV | WGQGTTVTVSS |
| iPS:4 36792 | 21-225_169D12 | VH3\|3-33/D3\|3-10\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAS | PLYDMGL-----------------YYDMDV | WGQGTTVTVSS |
| | | Germline | | H_FR1 | H_CDR1 | H_FR2 | H_CDR2 | H_FR3 | H_CDR3 | H_FR4 |
| | VH1\|1-46/D7\|7-27\|RF3/JH4 | | 32465 | QVQLVQS-GAEVKKPGASVKVSCKASG-YTFT | S-----YYMH | WVRQAPGQGLEWMG | IINPS---GGSTSYAQKFQG | RVTMTRDTSTSTVYMELSSLRSEDTAVYYCAR | NWGY-----------------------FDY | WGQGTLVTVSS |
| iPS:4 36800 | 21-225_171D12 | VH1\|1-46/D7\|7-27\|RF3/JH4 | | QVQLVQS-GAEVKKPGASVKVSCKASG-YTFN | S-----YYMY | WVRQAPGQGLEWMG | IINPS---GGSTNYAQKFQG | RVTMTRDTSTSTLYMELNSLRSEDTAVYYCAS | GWE-----------------------LNY | WGQGTLVTVSS |
| iPS:4 36804 | 21-225_172C3 | VH1\|1-46/D7\|7-27\|RF3/JH4 | | QVQLVQS-GAEVKKPGASVKVSCKASG-YTFR | S-----YYMY | WVRQAPGQGLEWVG | TINPS---GGSTNYAQKFQG | RVTMTRDTSTSTLYMELNSLRSEDTAVYYCAS | GWE-----------------------LNY | WGQGTLVTVSS |
| iPS:4 36806 | 21-225_172B12 | VH1\|1-46/D7\|7-27\|RF3/JH4 | | QVQLVQS-GAEVKKPGASVTVSCKASG-YTFR | S-----YYMY | WVRQAPGQGLEWVG | TINPS---GGSTDYAQKFQG | RVTMTRDTSTSTLYMELNSLRSEDTAVYYCAS | GWE-----------------------LNY | WGQGTLVTVSS |
| | | Germline | | H_FR1 | H_CDR1 | H_FR2 | H_CDR2 | H_FR3 | H_CDR3 | H_FR4 |
| | VH3\|3-30.3/D4\|4-23\|RF1/JH6 | | 32466 & 32467 & 32468 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | *LRW*LYY----------------YYYGMDV | WGQGTTVTVSS |
| iPS:4 36808 | 21-225_173F8 | VH3\|3-30.3/D4\|4- | | QVQLVES-GGGVVQPGRSLRL | S-----YGMH | WVRQAPGKGLEWVA | VISYD---GSPKYCADS | RFTISRDNSKNTLFLQMNSLRAEDTAVYYCAR | DERQWLP----------------APYGMDV | WGQGTTVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH6\|6-01/D6\|6-6\|RF2/JH6 | | 32469 | QVQLQQS-GPGLVKPSQTLSLTCAISG-DSVS | SN---SAAWN | WIRQSPSRGLEWLG | RTYYR--SKWYNDYAVSVKS | RITINPDTSKNQFSLQLNSVTPEDTAVYYCAR | SIAARYY---------------YYYGMDV | WGQGTTVTVSS |
| iPS:436810 | 21-225_175F4 | VH6\|6-01/D6\|6-6\|RF2/JH6 | | QVQLQQS-GPGLVKPSQTLSLTCAISG-DSVS | SN---SAAWN | WIRQSPSRGLEWLG | RTYYR--SKWYNAYPVSMES | RISINPDTSKNQFSLQLNSVTPEDTAVYYCAR | DKAAGRND--------------FYYYGMDV | WGQGTTVTVSS |
| iPS:436814 | 21-225_178H10 | VH6\|6-01/D6\|6-6\|RF2/JH6 | | QVQLQQS-GPGLVKPSQTLSLTCAISG-DSVS | SN---SAAWN | WIRQSPSRGLEWLG | RTYYR--SKWYSAYPVSMES | RVSINPDTSKNQFSLQLNSVTPEDTAVYYCAR | DKAAGRND--------------FYYYGMDV | WGQGTTVTVSS |
| | | Germline | | H_FR1 | H_CDR1 | H_FR2 | H_CDR2 | H_FR3 | H_CDR3 | H_FR4 |
| | VH3\|3-33/D1\|1-1\|RF3/JH6 | | 32470 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YNWNDYY---------------YYYGMDV | WGQGTTVTVSS |
| iPS:436818 | 21-225_179C7 | VH3\|3-33/D1\|1-1\|RF3/JH6 | | QAQLVES-GGGVVQPGRSLRLSCAASG-FTFS | N-----SGMH | WVRQGPGKGLEWVA | IIYYD---GSYKYNADSVKG | RFTISRDNSKNTLYLQMNSLRAEDTAVYYCAR | DRHYDFHVP------------YYYYYGMDV | WGQGTTVTVSS |
| iPS:437094 | 21-225_210D12 | VH3\|3-33/D1\|1-1\|RF3/JH6 | | QVQLVES-GGGVVQPGRSLRLSCAASG-FTFS | H-----YGMH | WVRQAPGKGLEWVA | VIWYD---GSNKYYADSVKG | RFTISRDNSKNTLYLQMNSLRAEDTAVYYCAR | GDWNP--------------------EGLDV | WGQGTTVTVSS |
| iPS:437096 | 21-225_210E12 | VH3\|3-33/D1\|1-1\|RF3/JH6 | | QVHLVES-GGGVVQPGRSLRLSCAASG-FTFS | N-----YGMH | WVRQAPGKGLEWVA | VIWYD---GSNKYYADSVKG | RFTISRDNSKNTLYLQMNSLRAEDTAVYYCAR | GDWNP--------------------EGMDV | WGQGTTVIVSS |
| iPS:437098 | 21-225_211C1 | VH3\|3-33/D1\|1-1\|RF3/JH6 | | QVQLVES-GGGVVQPGRSLRLSCAASG-FTFS | H-----YGMH | WVRQAPGKGLEWVA | VIWYD---GSNKYYADSVKG | RFTISRDNSKNTLYLQMNTLRAEDTAIYYCAR | GDWNP--------------------EGLDV | WGQGTTVTVSS |
| iPS:437104 | 21-225_211G5 | VH3\|3-33/D1\|1-1\|RF3/JH6 | | QVQLVES-GGGVVQPGRSLRLSCAASG-FTFS | H-----YGMH | WVRQAPGKGLEWVA | VIWYD---GSNKYYADSVKG | RFTISRDNSKNTLYLQMNSLRAEDTAVYYCAR | GDWNP--------------------EGLDV | WGQGTTVTVSS |
| iPS:437112 | 21-225_212C2 | VH3\|3-33/D1\|1-1\|RF3/JH6 | | QVQLVES-GGGVVQPGRSLRLSCAASG-FTFS | H-----YGMH | WVRQAPGKGLEWVA | VIWYD---GSNKYYADSVKG | RFTISRDNSKNTLYLQMNSLRAEDTAVYYCAR | GDWNP--------------------EGMDV | WGQGTSVTVSS |
| iPS:437114 | 21-225_212A4 | VH3\|3-33/D1\|1-1\|RF3/JH6 | | QVHLVES-GGGVVQAGRSLRLSCAASG-FTFS | N-----YGMH | WVRQAPGKGLEWVA | VIWYD---GSNKYYADSVKG | RFTISRDNSKNTLYLQMNSLRAEDTAVYYCAR | GDWNP--------------------EGMDV | WGQGTTVIVSS |
| iPS:437116 | 21-225_212F6 | VH3\|3-33/D1\|1- | | QVQLVES-GGGVVQPGRSLRL | H-----YGMH | WVRQAPGKGLEWVA | VIWYD---GSNKYYADS | RFTISRDNSKNTLYLQMNSLRAEDTAVYYCAR | GDWNP--------------------EGLDV | WGQGTTVTVS |

| | | | | | | | | | | |
|---|---|---|---|---|---|---|---|---|---|---|
| iPS:4 37118 | 21-225_212G7 | VH3\|3-33/D1\|1-1\|RF3/JH6 | | QVQLVES-GGGVVQPGRSLRLSCAASG-FTFS | H-----YGMH | WVRQAPGKGLEWVA | VIWYD---GSNKYCADSVKG | RFTISRDNSTNTLYLQMNSLRAEDTAVYYCAR | GDWNP-------------------EGLDV | WGQGTTVTVSS |
| iPS:4 37128 | 21-225_213G3 | VH3\|3-33/D1\|1-1\|RF3/JH6 | | QVQLVES-GGGVVQPGRSLRLSCAASG-FTFS | H-----YGMH | WVRQAPGKGLEWVA | VIWYD---GSNKYYADSVKG | RFTISRDNSKNTLHLQMNSLRAEDTAVYYCAR | GDWNP-------------------EGMDV | WGQGTSVTVSS |
| iPS:4 37130 | 21-225_213D5 | VH3\|3-33/D1\|1-1\|RF3/JH6 | | QVQLVES-GGGVVQPGRSLRLSCAASG-FTFS | H-----YGMH | WVRQAPGKGLEWVA | VIWYD---GSNKYYADSVKG | RFTISRDNSKNTLFLQMNSLRVEDTAVYYCAR | GDWNP-------------------EGMDV | WGQGTTVTVSS |
| iPS:4 37146 | 21-225_215D3 | VH3\|3-33/D1\|1-1\|RF3/JH6 | | QTQLVES-GGGVVQPGRSLRLSCAASG-FTFS | H-----YGMH | WVRQAPGKGLEWVA | VIWYD---GSNEYYADSVKG | RFTISRDNSKNTLYLQMNSLRAEDTAVYYCAR | GDWNP-------------------EGMDV | WGQGTTVTVSS |
| iPS:4 37150 | 21-225_216A3 | VH3\|3-33/D1\|1-1\|RF3/JH6 | | QVQLVES-GGGVVQPGRSLRLSCAASG-FTFS | H-----YGMH | WVRQAPGKGLEWVA | VIWYD---GSNKYYADSVKG | RFTISRDNSKNTLYLQMNSLRAEDTAVYYCAR | GDWNP-------------------EGLDV | WGQGTTVTVSS |
| iPS:4 37162 | 21-225_217B2 | VH3\|3-33/D1\|1-1\|RF3/JH6 | | QVHLVES-GGGVVQPGRSLRLSCAASG-FTFS | N-----YGMH | WVRQAPGKGLEWVA | VIWYD---GSNKYYADSVKG | RFTISRDNSKNTLYLQMNSLRAEDTAVYYCAR | GDWNP-------------------EGMDV | WGQGTTVIVSS |
| iPS:4 37172 | 21-225_219A7 | VH3\|3-33/D1\|1-1\|RF3/JH6 | | QVQLVES-GGGVVQPGRSLRLSCPASG-FTFS | H-----YGMH | WVRQAPGKGLEWVA | VIWYD---GSNKYYADSVKG | RFTISRDNSKNMLYLQMNSLRAEDTAVYYCAR | GDWNP-------------------EGMDV | WGQGTTVTVSS |
| iPS:4 37182 | 21-225_221H2 | VH3\|3-33/D1\|1-1\|RF3/JH6 | | QVQLVES-GGGVVQPGRSLRLSCAASG-FTFS | H-----YGMH | WVRQAPGKGLEWVA | VIWYD---GSNKYYADSVKG | RFTISRDNSKNTLHLQMNSLRAEDTAVYYCAR | GDWNP-------------------EGMDV | WGQGTTVTVSS |
| iPS:4 37184 | 21-225_221G4 | VH3\|3-33/D1\|1-1\|RF3/JH6 | | QVQLVES-GGGVVQPGRSLRLSCAASG-FTFS | H-----YGMH | WVRQAPGKGLEWVA | VIWYD---GSNKYYADSVKG | RFTISRDNSKNTLHLQMNSLRAEDTAVYYCAR | GDWNP-------------------EGMDV | WGQGTSVTVSS |
| iPS:4 38664 | 21-225_216G1 | VH3\|3-33/D1\|1-1\|RF3/JH6 | | QVHLVES-GGGVVQPGRSLRLSCAASG-FTFS | N-----YGMH | WVRQAPGKGLEWVA | VIWYD---GSNKYYADSVKG | RFTISRDNSKNTLYLQMNSLRAEDTAVYYCAR | GDWNP-------------------EGMDV | WGQGTTVIVSS |
| | | Germline | | H_FR1 | H_CDR1 | H_FR2 | H_CDR2 | H_FR3 | H_CDR3 | H_FR4 |
| | VH3\|3-33/D1\|1-1\|RF1/JH4 | | 33471 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | GTTGT--------------------YFDY | WGQGTLVTVSS |
| iPS:4 36822 | 21-225_180D4 | VH3\|3-33/D1\|1- | | QVQLVES-GGGVVQPGRSLRL | N-----FGMH | WVRQAPGKGLEWVA | IIWYD---GSDKYYADS | RFTISRDNSKNTLYLQMNTLRAEDTAVYFCAR | GGPPFST----------------VTMYFDY | WGQGTLVTVS |

| | | | | | | | | | | |
|---|---|---|---|---|---|---|---|---|---|---|
| iPS:4 36828 | 21-225_181H1 | VH3\|3-33/D1\|1-1\|RF1/JH4 | | QVQLVES-GGGVVQPGRSLRLSCAASG-FTFS | N-----YGMH | WVRQAPGKGLEWVA | IIWYD---GSDKYYADSVKG | RITISRDNSKNTLYLQMNTLRAEDTAVYFCAR | GGPPFST---------------VTMYFDY | WGQGTLVTVSS |
| iPS:4 36950 | 21-225_184G4 | VH3\|3-33/D1\|1-1\|RF1/JH4 | | QVQLVES-GGGVVQPGRSLRLSCAVSG-FTFS | S-----YGMH | WVRQAPGKGLEWVA | IIWYD---GSNKYYADSVKG | RFTISRDNSKNTLYLQMNSLRAEDTAVYYCAR | GGPPFST---------------VTMYFDY | WGQGTLVTVSS |
| iPS:4 36952 | 21-225_185D2 | VH3\|3-33/D1\|1-1\|RF1/JH4 | | QVQLVES-GGGVVQPGRSLRLSCAASG-FTFS | N-----YGMH | WVRQAPGKGLEWVA | IIWYD---GSDKYYADSVKG | RFTISRDNSKNTLYLQMNTLRAEDTAVYFCAR | GGPPFST---------------VTMYFDY | WGQGTLVTVSS |
| | | Germline | | H_FR1 | H_CDR1 | H_FR2 | H_CDR2 | H_FR3 | H_CDR3 | H_FR4 |
| | VH2\|2-05/D6\|6-6\|RF2/JH3 | | 324/2 | QITLKES-GPTLVKPTQTLTLTCTFSG-FSLS | TS---GVGVG | WIRQPPGKALEWLA | LIYW----NDDKRYSPSLKS | RLTITKDTSKNQVVLTMTNMDPVDTATYYCAH | SIAAR-------------------DAFDI | WGQGTMVTVSS |
| iPS:4 36824 | 21-225_180C5 | VH2\|2-05/D6\|6-6\|RF2/JH3 | | QITLKES-GPTLVKPTQTLTLTCTFSG-FSLS | TS---GVGVG | WIRQPPGKALEWLG | FISW----NDDKRYSPSLKS | SLTITKDTSKNQVVLIMTNMDPVDTATYYCAH | KAAAV---------------------AFDI | WGQGTMVTVSS |
| iPS:4 36956 | 21-225_186H6 | VH2\|2-05/D6\|6-6\|RF2/JH3 | | QITLKES-GPTLVKPTQTLTLTCTFSG-FSLS | TS---GVGVG | WIRQPPGKALEWLG | FISW----NDDKRYSPSLKS | SLTITKDTSKNQVVLTMTNMDPVDTATYYCAH | KAAAV---------------------AFDI | WGQGTMVTVSS |
| | | Germline | | H_FR1 | H_CDR1 | H_FR2 | H_CDR2 | H_FR3 | H_CDR3 | H_FR4 |
| | VH1\|1-08/D5\|5-18\|RF3/JH6 | | 324/3 | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | GYSYGYYY---------------YYYGMDV | WGQGTTVTVSS |
| iPS:4 36826 | 21-225_180G5 | VH1\|1-08/D5\|5-18\|RF3/JH6 | | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | GFYYYGSGSHV---------PYHYYYGLDV | WGQGTTVTVSS |
| | | Germline | | H_FR1 | H_CDR1 | H_FR2 | H_CDR2 | H_FR3 | H_CDR3 | H_FR4 |
| | VH6\|6-01/D5\|5-24\|RF3/JH2 | | 324/4 | QVQLQQS-GPGLVKPSQTLSLTCAISG-DSVS | SN---SAAWN | WIRQSPSRGLEWLG | RTYYR--SKWYNDYAVSVKS | RITINPDTSKNQFSLQLNSVTPEDTAVYYCAR | RDGYNY------------------YWYFDL | WGRGTLVTVSS |
| iPS:4 36832 | 21-225_51D8 | VH6\|6-01/D5\|5-24\|RF3/JH2 | | QVQLQQS-GPGLVKPSQTLSLTCAISG-DSVS | SN---SAAWN | WIRQSPSRGLEWLG | RTYYR--SKWYNDYAVSVKS | RITFNPDTSKNQFSLRLNSVTPEDTAVYYCAR | DRYNWNY----------------PYWYFDL | WGRGTLVTVSS |
| | | Germline | | H_FR1 | H_CDR1 | H_FR2 | H_CDR2 | H_FR3 | H_CDR3 | H_FR4 |
| | VH1\|1-02/D5\|5-24\|RF3/JH5 | | 324/5 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | RDGYNY------------------NWFDP | WGQGTLVTVSS |
| iPS:4 36840 | 21-225_53E9 | VH1\|1-02/D5\|5- | | QVQLVQS-GAEVKKPGASVKV | G-----YYMH | WVRQAPGQGLEWMG | WIIPN---SGDTNYAQK | RVTMTRDTSISTAYMELSRLRSDDTAVYYCAR | DGYSSGW-----------------FNWFDP | WGQGTLVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH2\|2-05/D7\|7-27\|RF1/JH4 | | 32476 | QITLKES-GPTLVKPTQTLTLTCTFSG-FSLS | TS---GVGVG | WIRQPPGKALEWLA | LIYW----NDDKRYSPSLKS | RLTITKDTSKNQVVLTMTNMDPVDTATYYCAH | LTGY-----------------------FDY | WGQGTLVTVSS |
| iPS:436848 | 21-225_57F1 | VH2\|2-05/D7\|7-27\|RF1/JH4 | | QITLKES-GPTLVKPTQTLTLTCTFSG-FSLS | TS---GVGVG | WIRQPPGKALEWLA | LIYW----HEDKRYSPSLKS | RLTITEDTSKNQVDLTMTNMAPVDTATYYCAH | VTGI----------------------AAPY | WGQGTLVTVSS |
| iPS:436852 | 21-225_57H11 | VH2\|2-05/D7\|7-27\|RF1/JH4 | | QITLKES-GPTLVKPTQTLTLTCTFSG-FSLT | TS---GVGVG | WIRQPPGKALEWLA | LIYW----HEDRRYSPSLKS | RLTITEDTSKNQVDLTMTNMAPVDTATYYCAH | VTGI----------------------AAPY | WGQGTLVTVSS |
| iPS:436870 | 21-225_60B1 | VH2\|2-05/D7\|7-27\|RF1/JH4 | | QITLKES-GPTLVKPTQTLTLTCTFSG-FSLS | TS---GVGVG | WIRQPPGKALEWLA | LIYW----HEDKRYSPSLKS | RLTITEDTSKNQVDLTMTNMAPVDTATYYCAH | VTYI----------------------AAPY | WGQGTLVTVSS |
| iPS:436876 | 21-225_61F5 | VH2\|2-05/D7\|7-27\|RF1/JH4 | | QITLKES-GPTLVKPTQTLTLTCTFSG-FSLS | TS---GLGVG | WIRQPPGKALEWLA | LIYS----HEDKRYSPSLKS | RLTITEDTSKNQVDLTMTNMAPVDTATYYCAH | VTGI----------------------AAPY | WGQGTLVTVSS |
| iPS:392593 | 21-225_3E10 | VH2\|2-05/D7\|7-27\|RF1/JH4 | | QITLKES-GPTLVKPTQTLTLTCTFSG-FSLN | TG---GVGVG | WIRQPPGKALEWLA | LIYW----NDDKRHSPSLKS | RLTITKDTSKNQVVLTMTHMAPVDTATYYCAH | LIEV----------------------AFDY | WGQGTLVTVSS |
| | | Germline | | H_FR1 | H_CDR1 | H_FR2 | H_CDR2 | H_FR3 | H_CDR3 | H_FR4 |
| | VH2\|2-05/D6\|6-6\|RF2/JH1 | | 32477 | QITLKES-GPTLVKPTQTLTLTCTFSG-FSLS | TS---GVGVG | WIRQPPGKALEWLA | LIYW----NDDKRYSPSLKS | RLTITKDTSKNQVVLTMTNMDPVDTATYYCAH | SIAARA--------------------EYFQH | WGQGTLVTVSS |
| iPS:436854 | 21-225_58C1 | VH2\|2-05/D6\|6-6\|RF2/JH1 | | QITLKES-GPTLVKPTQTLTLTCTFSG-FSLS | TS---GVGVG | WIRQPPGKALEWLA | LIYW----DDDKRYSPSLKS | RLTITEDTSKNQVVLTMTNMDPVDTATYYCAH | LIAV----------------------AFDS | WGQGTLVTVSS |
| iPS:436874 | 21-225_60A12 | VH2\|2-05/D6\|6-6\|RF2/JH1 | | QITLKES-GPTLVKPTQTLTLTCTFSG-FSLS | TS---GVGVG | WIRQPPGKALEWLA | LIYW----DDDKRYSPSLKS | RLTITEDTSKNQVVLTMTNMDPVDTATYYCAH | LIAV----------------------AFDS | WGQGTLVTVSS |
| iPS:436954 | 21-225_185G7 | VH2\|2-05/D6\|6-6\|RF2/JH1 | | QITLKES-GPTLVKPTQTLTLTCTFSG-FSLT | TG---GVGVG | WIRQPPGKALEWLA | LIYW----NDDERYSPSLKS | RLTITKDTSKNQVVLTMTNMDPVDTATYYCAH | IIAV----------------------AFQH | WGQGTLVTVSS |
| iPS:393188 | 21-225_34B9 | VH2\|2-05/D6\|6-6\|RF2/JH1 | | QITLRES-GPTLVKPTQTLTLTCTFSG-FSLS | TS---GVGVG | WIRQPPGKALEWLA | LIYW----NDDKRYSPSLKS | RLTITKDTSKNQVVLTMTNMDPVDTATYYCAH | LIAV----------------------TFDS | WGQGSLVTVSS |
| | | Germline | | H_FR1 | H_CDR1 | H_FR2 | H_CDR2 | H_FR3 | H_CDR3 | H_FR4 |
| | VH3\|3- | | 32478 | QVQLVES- | S-----YAMH | WVRQAPGK | VISYD--- | RFTISRDNSKNTLYLQMNSL | YYYGSGSYYN------- | WGQGT |

| | | | | | | | | | | |
|---|---|---|---|---|---|---|---|---|---|---|
| iPS:436858 | 21-225_58E7 | VH3\|3-30.3/D3\|3-10\|RF2/JH6 | | QVQLVES-GGGVVQPGRSLRLSCAASG-FTFS | F-----YGMH | WVRQAPGKGLEWVA | VTSYD---GSDKYYADSVKG | RFSISRDNSKNTLYLQMSSLRAEDTAMYYCAR | DDYGSGSP---------------LYYGMDV | WGQGTTVTVSS |
| | | Germline | | H_FR1 | H_CDR1 | H_FR2 | H_CDR2 | H_FR3 | H_CDR3 | H_FR4 |
| | VH3\|3-07/D5\|5-12\|RF3/JH6 | | 32479 | EVQLVES-GGGLVQPGGSLRLSCAASG-FTFS | S-----YWMS | WVRQAPGKGLEWVA | NIKQD---GSEKYYVDSVKG | RFTISRDNAKNSLYLQMNSLRAEDTAVYYCAR | GYSGYDYY--------------YYYGMDV | WGQGTTVTVSS |
| iPS:436860 | 21-225_58F7 | VH3\|3-07/D5\|5-12\|RF3/JH6 | | EVQLVES-GGGLVQPGGSLRLSCAASG-FTFS | S-----FWMS | WVRQAPGKGLEWVA | HIKQD---GSEKYYVDSVKG | RFTISRDNAKNSLYLQMNSLRAEDTAVYYCAR | GDLPYSSG---------------YYYGMDV | WGQGTTVTVSS |
| | | Germline | | H_FR1 | H_CDR1 | H_FR2 | H_CDR2 | H_FR3 | H_CDR3 | H_FR4 |
| | VH3\|3-30.3/D4\|4-17\|RF2/JH6 | | 32480 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | DYGDYYY---------------YYYGMDV | WGQGTTVTVSS |
| iPS:436862 | 21-225_58F8 | VH3\|3-30.3/D4\|4-17\|RF2/JH6 | | QVQLVES-GGGVVQPGRSLRLSCAASG-FTFS | S-----YGMH | WVRQAPGKGLEWVA | VISYD---GSNKYYADSVKG | RFTISRDNSKNTLYLQMNSLRAEDTAVYYCAR | DEGRGYGGYER--------GYYYYYGMDV | WGQGTTVTVSS |
| | | Germline | | H_FR1 | H_CDR1 | H_FR2 | H_CDR2 | H_FR3 | H_CDR3 | H_FR4 |
| | VH3\|3-33/D3\|3-16\|RF2/JH6 | | 32481 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YYDYVWGSYRY---------TYYYYYGMDV | WGQGTTVTVSS |
| iPS:436868 | 21-225_59B11 | VH3\|3-33/D3\|3-16\|RF2/JH6 | | QVQLVES-GGGVVQPGRSLRLSCAASG-FTFS | S-----YGVH | WVRQAPGKGLEWVA | AIWYD---GSNKYYADSVKG | RFTISRDNSKNTLYLLMNSLRAEDTAVYYCAR | DRDYCSSSSC----------PYYYYYGMDV | WGQGTTVTVSS |
| | | Germline | | H_FR1 | H_CDR1 | H_FR2 | H_CDR2 | H_FR3 | H_CDR3 | H_FR4 |
| | VH2\|2-05/D1\|1-1\|RF1/JH3 | | 32482 | QITLKES-GPTLVKPTQTLTLTCTFSG-FSLS | TS---GVGVG | WIRQPPGKALEWLA | LIYW----NDDKRYSPSLKS | RLTITKDTSKNQVVLTMTNMDPVDTATYYCAH | GTTGT---------------------DAFDI | WGQGTMVTVSS |
| iPS:436878 | 21-225_62E3 | VH2\|2-05/D1\|1-1\|RF1/JH3 | | QITLKES-GPTLVKPTQTLTLTCTFSG-FSLS | TS---GVGVG | WIRQPPGKALEWLA | LINW----NDDKRYSPSLKS | RFTITRDTSKDQVVLTMTNMDPVDTATYYCAH | KATWV---------------------AFDI | WGQGTMVTVSS |
| iPS:436880 | 21-225_62E8 | VH2\|2-05/D1\|1-1\|RF1/JH3 | | QITLKES-GPTLVKPTQTLTLTCTFSG-FSLS | TS---GVGVG | WIRQPPGKALEWLA | LINW----NDDKRYSPSLKS | RFTITRDTSKDQVVLTMTNMDPVDTATYYCAH | KATWV---------------------AFDI | WGQGTMVTVSS |
| iPS:436882 | 21-225_62D10 | VH2\|2-05/D1\|1-1\|RF1/JH3 | | QITLKES-GPTLVKSTQTLTLTCTFSG-FSLS | TS---GVGVG | WIRQPPGKALEWLA | LINW----NDDKRYSPSLKS | RFTITRDTSKDQVVLTMTNMDPVDTATYYCAH | KATWV---------------------AFDI | WGQGTMVTVSS |
| iPS:436884 | 21-225_62A12 | VH2\|2-05/D1\|1- | | QITLKES-GPTLVKPTQTLTL | TS---GVGVG | WIRQPPGKALEWLA | LINW----NDDKRYSPS | RFTITRDTSKDQVVLTMTNMDPLDTATYYCAH | KTTWV---------------------AFDI | WGQGTMVTVS |

| | | | | | | | | | | |
|---|---|---|---|---|---|---|---|---|---|---|
| iPS:4 36886 | 21-225_62B12 | VH2\|2-05/D1\|1-1\|RF1/JH3 | | QITLKES-GPTLVKPTQTLTLTCTFSG-FSLN | TS---GVGVG | WIRQPPGKALEWLA | LINW----NDDKRYSPSLKS | RFTITRDTSKDQVVLTMTNMDPVDTATYYCAH | KATWV--------------------AFDI | WGQGTMVTVSS |
| iPS:4 36894 | 21-225_66G9 | VH2\|2-05/D1\|1-1\|RF1/JH3 | | QITLKES-GPTLVKPTQTLTLTCTFSG-FSLS | TS---GVGVG | WIRQPPGKALEWLA | LINW----NDDKRFSPSLKS | RFTITRDTSKDQVVLTMTNMDPVDTATYYCAH | KATWV--------------------AFDI | WGQGTMVTVSS |
| iPS:4 36908 | 21-225_72D5 | VH2\|2-05/D1\|1-1\|RF1/JH3 | | QITLKES-GPTLVKPTQTLTLTCTFSG-FSLS | TS---GVGVG | WIRQPPGKALEWLA | LINW----NDDKRYSPSLKS | RFTITRDTSKDQVVFTMTNMDPVDTGTYYCAH | KATWV--------------------AFDI | WGQGTMVTVSS |
| iPS:4 36912 | 21-225_73C4 | VH2\|2-05/D1\|1-1\|RF1/JH3 | | QITLKES-GPTLVKPTQTLTLTCTFSG-FSLS | TS---GVGVG | WIRQPPGKALEWLA | LINW----NDDKRYSPSLKS | RFTITRDTSKDQVVLTMTNMDPVDTATYYCAH | KTTWV--------------------AFDI | WGQGTMVTVSS |
| | | Germline | | H_FR1 | H_CDR1 | H_FR2 | H_CDR2 | H_FR3 | H_CDR3 | H_FR4 |
| | VH3\|3-48/D4\|4-11\|RF2/JH3 | | 32483 | EVQLVES-GGGLVQPGGSLRLSCAASG-FTFS | S-----YSMN | WVRQAPGKGLEWVS | YISSS---SSTIYYADSVKG | RFTISRDNAKNSLYLQMNSLRDEDTAVYYCAR | DYSNY--------------------DAFDI | WGQGTMVTVSS |
| iPS:4 36888 | 21-225_63G7 | VH3\|3-48/D4\|4-11\|RF2/JH3 | | EVQLVES-GGGLVQPGGSLRLSCAASG-FTFS | S-----YSMN | WVRQAPGKGLEWVS | YISSS---TSTIYYAASVKG | RFTISRDNAKNSLYLQMNSLRDEDTAVYYCAR | DHRYYDSSG-------------YYSDAFDI | WGQGTMVTVSS |
| iPS:4 36890 | 21-225_63A10 | VH3\|3-48/D4\|4-11\|RF2/JH3 | | EVQLVES-GGGLVQPGGSLRLSCAASG-FTFS | S-----YSMN | WVRQAPGKGLEWVS | YISSS---TSTIYYAASVKG | RFTISRDNAKNSLYLQMNSLRDEDTAVYYCAR | DHRYYDSSG-------------YYSDAFDI | WGQGTMVTVSS |
| | | Germline | | H_FR1 | H_CDR1 | H_FR2 | H_CDR2 | H_FR3 | H_CDR3 | H_FR4 |
| | VH1\|1-02/D3\|3-22\|RF2/JH3 | | 32484 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | YYYDSSGY---------------YYDAFDI | WGQGTMVTVSS |
| iPS:4 36892 | 21-225_65E9 | VH1\|1-02/D3\|3-22\|RF2/JH3 | | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | AYYYGSGS---------------YYNEFDM | WGQGTMVTVSS |
| iPS:4 36900 | 21-225_69B9 | VH1\|1-02/D3\|3-22\|RF2/JH3 | | QVQMVQS-GDEVKKPGASVKVSCKASG-YTFT | G-----YHMH | WVRQAPGQGLEWMG | WINPN---SGGTNYAQKFQG | RVTMTRDTSISTAYMELSRMRSDDTAVYYCAR | AYYYGSGS---------------YYNESDM | WGQGTMVTVSS |
| | | Germline | | H_FR1 | H_CDR1 | H_FR2 | H_CDR2 | H_FR3 | H_CDR3 | H_FR4 |
| | VH6\|6-01/D4\|4-17\|RF2/JH6 | | 32485 | QVQLQQS-GPGLVKPSQTLSLTCAISG-DSVS | SN---SAAWN | WIRQSPSRGLEWLG | RTYYR--SKWYNDYAVSVKS | RITINPDTSKNQFSLQLNSVTPEDTAVYYCAR | DYGDYYY---------------YYYGMDV | WGQGTTVTVSS |
| iPS:4 36898 | 21-225_68D8 | VH6\|6-01/D4\|4- | | QVQLQQS-GPGLVKPSQTLSL | SN---SAAWN | WIRQSPSRGLEWLG | RTYYR--SECYNDYAV | RITINPDTSKNQFSLHLNSVTPEDTAVYFCAR | DRGHRG-----------------FYGMDV | WGQGTTVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH3\|3-33/D7\|7-27\|RF1/JH3 | | 32486 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | LTGD---------------------AFDI | WGQGTMVTVSS |
| iPS:436910 | 21-225_73G1 | VH3\|3-33/D7\|7-27\|RF1/JH3 | | QVQLVES-GGGVVQPGRSLRLSCAGTG-FTFS | Y-----YGMH | WVRQAPGKGLEWVA | VTTYD---GSNKYYADSVKG | RFTSSRDNSKNTLYLQMNSLRAEDTAVYHCAR | ETGTW--------------------AFDI | WGQGTMVTVSL |
| | | Germline | | H_FR1 | H_CDR1 | H_FR2 | H_CDR2 | H_FR3 | H_CDR3 | H_FR4 |
| | VH4\|4-30.1/D2\|2-15\|RF3/JH4 | | 32487 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | DIVVVVA-----------------AIYFDY | WGQGTLVTVSS |
| iPS:436920 | 21-225_74E5 | VH4\|4-30.1/D2\|2-15\|RF3/JH4 | | QVQLQES-GPGLVKPSQTLSLTCTVSG-GSIR | SG---GYYWS | WIRQHPGKGLEWIG | YIYY----SGSTYYNPSLRS | RASISVDTSKNQFSLKLSSVTAADTAVYYCAR | DSPVA--------------------GTDY | WGQGTLVTVSS |
| iPS:437012 | 21-225_192G7 | VH4\|4-30.1/D2\|2-15\|RF3/JH4 | | QVQLQES-GPGLVKPSQTLSLTCTVSG-GSIS | SG---GYYWS | WIRQHPGKGLEWIG | YIYY----RGSTYYNPSLKS | RVTISVDTSKNQFSLKLNSVTAADTAVYYCAR | DSPVT--------------------GFDY | WGQGILVTVSS |
| | | Germline | | H_FR1 | H_CDR1 | H_FR2 | H_CDR2 | H_FR3 | H_CDR3 | H_FR4 |
| | VH1\|1-08/D5\|5-12\|RF3/JH6 | | 32488 | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | GYSGYDYY--------------YYYYGMDV | WGQGTTVTVSS |
| iPS:436942 | 21-225_146H8 | VH1\|1-08/D5\|5-12\|RF3/JH6 | | QVQLVQS-GAEVKKPGASVKVSCKASG-YTFI | S-----YDIN | WVRQATGQGLEWMG | WMNPN---SGNTGYAQKFQG | RVTMTRNTSKSTAYMELSSLRSEDTAVYYCAR | GDYYYDSSGHQ---------PYYYYYGMDV | WGQGTTVTVSS |
| | | Germline | | H_FR1 | H_CDR1 | H_FR2 | H_CDR2 | H_FR3 | H_CDR3 | H_FR4 |
| | VH2\|2-05/D6\|6-6\|RF3/JH4 | | 32489 & 32490 | QITLKES-GPTLVKPTQTLTLTCTFSG-FSLS | TS---GVGVG | WIRQPPGKALEWLA | LIYW----NDDKRYSPSLKS | RLTITKDTSKNQVVLTMTNMDPVDTATYYCAH | V*QLV--------------------YFDY | WGQGTLVTVSS |
| iPS:436944 | 21-225_182D12 | VH2\|2-05/D6\|6-6\|RF3/JH4 | | QITLKES-GPTLVKPTQPLTLTCTFSG-FSLS | TT---GVGVG | WIRQPPGKALEWLG | ILFW----NDDERYSPSLKS | RLTITKDTSKNQVVLTMTNMDPVDTATYYCAH | KSQLV--------------------YFDY | WGQGTLVTVSS |
| | | Germline | | H_FR1 | H_CDR1 | H_FR2 | H_CDR2 | H_FR3 | H_CDR3 | H_FR4 |
| | VH4\|4-30.1/D2\|2-8\|RF3/JH4 | | 32491 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | DIVLMVY-----------------AIYFDY | WGQGTLVTVSS |
| iPS:436958 | 21-225_190D1 | VH4\|4-30.1/D2\|2-8\|RF3/JH4 | | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | DSPLR--------------------GFDY | WGQGTLVTVSS |
| | | Germline | | H_FR1 | H_CDR1 | H_FR2 | H_CDR2 | H_FR3 | H_CDR3 | H_FR4 |

| | | | | | | | | | | |
|---|---|---|---|---|---|---|---|---|---|---|
| | VH6\|6-01/D4\|4-17\|RF2/JH4 | | 32432 | QVQLQQS-GPGLVKPSQTLSLTCAISG-DSVS | SN---SAAWN | WIRQSPSRGLEWLG | RTYYR--SKWYNDYAVSVKS | RITINPDTSKNQFSLQLNSVTPEDTAVYYCAR | DYGDY---------------------YFDY | WGQGTLVTVSS |
| iPS:436962 | 21-225_190H1 | VH6\|6-01/D4\|4-17\|RF2/JH4 | | QVQLQQS-GPGLVKPSQTLSLTCDISG-DSVS | RK---SATWN | WIRQSPSRGLEWLG | KTYYR--SKWYNDYAVSVKS | RITINPDTSKNQFSLQLNSVTPEDTAVYYCAR | DPGG---------------------LFDY | WGQGTLVTVSS |
| iPS:436978 | 21-225_190G9 | VH6\|6-01/D4\|4-17\|RF2/JH4 | | QVQLQQS-GPGLVKPSQTLSLTCAISG-DSVS | RK---SATWN | WIRQSPSRGLEWLG | RTYYR--SKWYNDYAVSVKS | RIIINPDTSKNQFSLQLNSVTPEDTAVYYCAR | DPGG---------------------LFDY | WGQGTLVTVSS |
| iPS:437070 | 21-225_201G11 | VH6\|6-01/D4\|4-17\|RF2/JH4 | | QVQLQQS-GPGLVKPSQTLSLTCAISG-DSVS | RI---NPTWN | WIRQSPSRGLEWLG | RTYYR--SKWYHVYAVSVKS | RITINPDTSKNQFSLQLNSVTPEDTAVYYCAR | DPGG---------------------LFDY | WGQGTLVTVSS |
| iPS:437076 | 21-225_203G6 | VH6\|6-01/D4\|4-17\|RF2/JH4 | | QVQLQQS-GPGLVKPSQTLSLTCAISG-DSVS | RT---NPTWN | WIRQSPSRGLEWLG | RTYYR--SKWYHVYALSVKS | RITITPDTSKNQFSLQLNSVTPEDTAVYYCAR | DPGG---------------------LFDY | WGQGTLVTVSS |
| | | Germline | | H_FR1 | H_CDR1 | H_FR2 | H_CDR2 | H_FR3 | H_CDR3 | H_FR4 |
| | VH3\|3-33/D1\|1-7\|RF3/JH6 | | 32433 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YNWNYYY----------------YYYGMDV | WGQGTTVTVSS |
| iPS:436964 | 21-225_190B3 | VH3\|3-33/D1\|1-7\|RF3/JH6 | | QVQLVES-GGGVVQPGRSLRLSCAASG-FTFN | N-----YGIH | WVRQAPGKGLEWVA | VIWFD---GDNKYYADSVKG | RFTISRDNSKNTLYLQMNSLRAEDTAVYYCAR | DNWNYGDH--------------YYYFGMDV | WGQGTTVTVSS |
| iPS:436970 | 21-225_190B8 | VH3\|3-33/D1\|1-7\|RF3/JH6 | | QVQLVES-GGGVVQPGRSLRLSCAASG-FTFS | S-----YGMH | WVRQAPGKGLEWVA | VIWFD---GSNKYYTDSVKG | RFTISRDNSKNTLYLQMNSLRAEDTAVYYCAR | DNWNYGDY--------------YYYYGMDV | WGQGTTVTVSS |
| iPS:436980 | 21-225_190C10 | VH3\|3-33/D1\|1-7\|RF3/JH6 | | QVQLVES-GGGVVQPGRSLRLSCAASG-FTLS | N-----YGMH | WVRQAPGKGLEWVA | VIWFG---GDNKYYADSVRG | RFTISRDNSKNTLYLQMNSLRAEDTAVFYCAR | DNWNYGDH--------------YYYYGMDV | WGQGTTVTVSS |
| iPS:436992 | 21-225_191B8 | VH3\|3-33/D1\|1-7\|RF3/JH6 | | QVQLVES-GGGVVQPGRSLRLSCAASG-FTLS | S-----YGMH | WVRQAPGKGLEWVA | VIWFD---GSNKYYADSVKG | RFTISRDNSKNTLYLQMNSLRAEDTAVYYCAR | DNWNYGDH--------------YYYYGMDV | WGQGTTVTVSS |
| iPS:436994 | 21-225_191A9 | VH3\|3-33/D1\|1-7\|RF3/JH6 | | QVQLVES-GGGVVQPGRSLRLSCAASG-FTLS | N-----YGMH | WVRQAPGKGLEWVA | VIWFG---GDNKYYADSVKG | RFTISRDNSKNTLYLQMNSLRAEDTAVFYCAR | DNWNYGDH--------------YYYYGMDV | WGQGTTVTVSS |
| | | Germline | | H_FR1 | H_CDR1 | H_FR2 | H_CDR2 | H_FR3 | H_CDR3 | H_FR4 |
| | VH4\|4-30.1/D4\|4- | | 32494 | QVQLQES-GPGLVKPSQTLSL | SG---GYYWS | WIRQHPGKGLEWIG | YIYY----SGSTYYNPS | RVTISVDTSKNQFSLKLSSVTAADTAVYYCAR | DYSNYY---------------YYYGMDV | WGQGTTVTVS |

| | | | | | | | | | | |
|---|---|---|---|---|---|---|---|---|---|---|
| iPS:436984 | 21-225_190F10 | VH4\|4-30.1/D4\|4-11\|RF2/JH6 | | QVQLQES-GPGLVKPSQTLSLTCTVSG-GSIR | SG---GDYWS | WIRQHPGEGLEWIG | YIYY----SGITYYNPSLKS | RVTISVDTSKNQFSLKLSSVTAADTAVYYCAR | DSSSR---------------------GMDV | WGQGTTVTVSS |
| iPS:436988 | 21-225_191A2 | VH4\|4-30.1/D4\|4-11\|RF2/JH6 | | QVQLQES-GPGLVKPSQTLSLTCTVSG-GSIR | SG---GDYWS | WIRQHPGEGLEWIG | YIYY----SGITYYNPSLKS | RVTISVDTSKNQFSLKLSSVTAADTAVYYCAR | DSSSR---------------------GMDV | WGQGTTVTVSS |
| iPS:437014 | 21-225_192H8 | VH4\|4-30.1/D4\|4-11\|RF2/JH6 | | QVQLQES-GPGVVKPSQTLSLTCTVSG-GSIS | SG---GDYWS | WIRQHPGKGLEWIG | YIYY----SGPTYYNPSLKS | RLTMSADTSKNQFSLKLSSVTAADTAVYYCAR | DSSLY---------------------GMDV | WGQGTTVTVSS |
| iPS:437022 | 21-225_194G5 | VH4\|4-30.1/D4\|4-11\|RF2/JH6 | | QVQLQES-GPGLVKPSQTLSLICTVSG-GSIR | SG---GDYWS | WIRQHPGKGLEWIG | YIYY----SGSTYYNPSLKS | RVTISVDTSKNQFSLKLSSVTAADTAVYYCAR | DHSLY---------------------GMDV | WGQGTTVTVSS |
| iPS:437026 | 21-225_194D12 | VH4\|4-30.1/D4\|4-11\|RF2/JH6 | | QVQLQES-GPGLVKPSQTLSLTCTVSG-GSIS | SG---GDYWS | WIRQHPGKGLEWIG | YIYY----SGSTYYNPSLKS | RVTISVDTSKNQFSLKLSSVTAADTAVYYCAR | DGARH---------------------GMDV | WGQGTTVTVSS |
| iPS:437056 | 21-225_198B8 | VH4\|4-30.1/D4\|4-11\|RF2/JH6 | | QVQLQES-GPGLVKPSQTLSLTCTVSG-GSIR | SG---GDYWS | WIRQHPGEGLEWIG | YIYY----SGITYYNPSLKS | RVTISVDTSKNQFSLKLSSVTAADTAVYYCAR | DSSSR---------------------GMDV | WGQGTTVTVSS |
| iPS:437124 | 21-225_212H12 | VH4\|4-30.1/D4\|4-11\|RF2/JH6 | | QVQLQES-GPGLVKPSQTLSLTCTVSG-GSIR | SG---GDYWS | WIRQHPGKGLEWIG | YIYY----SGSTYYNPSLKS | RVTISGDTSKNQFSLKLSSVTAADTAVYYCAR | DSSSY---------------------GMDV | WGQGTTVTVSS |
| iPS:437136 | 21-225_214H3 | VH4\|4-30.1/D4\|4-11\|RF2/JH6 | | QVQLQES-GPGLVKPSQTLSLTCTVSG-GSIR | SG---GDYWS | WIRQHPGKGLEWIG | YIYY----SGSTYYNPSLKS | RVTISGDTSKNQFSLKLSSVTAADTAVYYCAR | DSSSY---------------------GMDV | WGQGTTVTVSS |
| | | Germline | | H_FR1 | H_CDR1 | H_FR2 | H_CDR2 | H_FR3 | H_CDR3 | H_FR4 |
| | VH4\|4-59/D1\|1-26\|RF1/JH4 | | 32495 | QVQLQES-GPGLVKPSETLSLTCTVSG-GSIS | S-----YYWS | WIRQPPGKGLEWIG | YIYY----SGSINYNPSLKS | RVTISVDTSKNQFSLKLSSVTAADTAVYYCAR | GIVGA--------------------TYFDY | WGQGTLVTVSS |
| iPS:436986 | 21-225_191A1 | VH4\|4-59/D1\|1-26\|RF1/JH4 | | QVQLQES-GPGLVKPSETLSLTCTVSG-GSIR | S-----YYWI | WIRQPPGKGLEWIG | YIYY----SGSTKYNPSLKS | RVTISVDTSKNQFSLKLSSVTAADTAVYYCAR | KGVGT--------------------IHFDY | WGQGTLVTVSS |
| iPS:437064 | 21-225_200G8 | VH4\|4-59/D1\|1-26\|RF1/JH4 | | QVQLQES-GPGLVKPSETLSLTCTVSG-GSIR | S-----YYWS | WIRQPPGKGLEWIG | YIYY----SGSTKYNPSLKS | RVTISVDTSKNQFSLKLSSVTAADTAVYYCAR | KGVGT--------------------IHFDY | WGQGTLVTVSS |
| | | Germline | | H_FR1 | H_CDR1 | H_FR2 | H_CDR2 | H_FR3 | H_CDR3 | H_FR4 |
| | VH3\|3-30/D6\|6- | | 32496 | QVQLVES- | S-----YGMH | WVRQAPGK | VISYD--- | RFTISRDNSKNTLYLQMNSL | EYSSS----------- | WGQGT |

| | | | | | | | | | | |
|---|---|---|---|---|---|---|---|---|---|---|
| iPS:436996 | 21-225_191B9 | VH3\|3-30/D6\|6-6\|RF1/JH4 | | QVQLVES-GGGVVQPGRSLRLSCAASG-FTFS | F-----HGMH | WVRQAPGKGLEWVA | VIWYD---GSKKYYADSVKG | RFTISRDNSKNTLHLQMNSLRAEDTAVYYCAK | EGYSSGF-----------------YRGFDN | WGQGTLVTVSS |
| iPS:437054 | 21-225_194G3 | VH3\|3-30/D6\|6-6\|RF1/JH4 | | QVQLVES-GGGVVQPGRSLRLSCAASG-FTFS | F-----HGMH | WVRQAPGKGLEWVA | VIWYD---GSKKYYADSVKG | RFTISRDNSKNTLHLQMNSLRAEDTAVYYCAK | EGFSSGF-----------------YRGFDN | WGQGTLVTVSS |
| | | Germline | | **H_FR1** | **H_CDR1** | **H_FR2** | **H_CDR2** | **H_FR3** | **H_CDR3** | **H_FR4** |
| | VH3\|3-33/D1\|1-26\|RF1/JH6 | | 32497 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | GIVGATYY---------------YYYGMDV | WGQGTTVTVSS |
| iPS:437000 | 21-225_191G9 | VH3\|3-33/D1\|1-26\|RF1/JH6 | | QVQLVES-GGGVVQPGRSLRLSCAASG-FTFS | T-----YGMH | WVRQAPGKGLEWVT | LIWFD---GSNKYYADSVKG | RFTISRDNSKNTLYLQMNSLRAEDTAVYYCAR | DRVGGTSPP------------YYYYYGMDV | WGQGTTVTVSS |
| iPS:393192 | 21-225_12B1 | VH3\|3-33/D1\|1-26\|RF1/JH6 | | QVQLVES-GGGVVQPGRSLRLSCAASG-FTFS | S-----YGMH | WVRQAPGKGLEWVA | VIWND---GSNKYYADSVKG | RFTISRDNSKNTLYLQMNSLRAEDTAVYYCAR | DRVAAAGTP-------------YYYYGMDV | WGQGTTVTVSS |
| | | Germline | | **H_FR1** | **H_CDR1** | **H_FR2** | **H_CDR2** | **H_FR3** | **H_CDR3** | **H_FR4** |
| | VH1\|1-02/D3\|3-10\|RF2/JH5 | | 32498 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | YYYGSGSY--------------YNNWFDP | WGQGTLVTVSS |
| iPS:437002 | 21-225_191H9 | VH1\|1-02/D3\|3-10\|RF2/JH5 | | QVQLVQS-GAEVKKPGASVKVSCKVSG-YTFT | G-----YNMH | WVRQAPGQGLEWMG | WINPN---SGGTNYAHKFQG | RVTMTRDTSISTAYMELSRLRSDDTAVYYCAR | DFYDSGG-----------------EGWFDP | WGQGTLVTVSS |
| | | Germline | | **H_FR1** | **H_CDR1** | **H_FR2** | **H_CDR2** | **H_FR3** | **H_CDR3** | **H_FR4** |
| | VH4\|4-30.4/D2\|2-8\|RF3/JH6 | | 32499 | QVQLQES-GPGLVKPSQTLSLTCTVSG-GSIS | SG---DYYWS | WIRQPPGKGLEWIG | YIYY----SGSTYYNPSLKS | RVTISVDTSKNQFSLKLSSVTAADTAVYYCAR | DIVLMVYAI------------YYYYYGMDV | WGQGTTVTVSS |
| iPS:437008 | 21-225_192E3 | VH4\|4-30.4/D2\|2-8\|RF3/JH6 | | QVQLQES-GPGLVKPSQTLSLTCTVSG-GSIS | SG---GYYWS | WIRQPPGKGLEWIG | YIYY----TGSTYYNPSLKS | RVTISVDTSKNQFSLKLSSVTAADTAVYYCAR | DDPLY---------------------GMDV | WGQGTTVTVSS |
| iPS:437048 | 21-225_197B11 | VH4\|4-30.4/D2\|2-8\|RF3/JH6 | | QVQLQES-GPGLVKPSQTLSLTCTVSG-GSIS | SG---GYYWS | WIRQPPGKGLEWIG | YIYY----TGSTYYNPSLKS | RVTISVDTSKNQFSLKLSSVTAADTAVYYCAR | DDPLY---------------------GMDV | WGQGTTVTVSS |
| | | Germline | | **H_FR1** | **H_CDR1** | **H_FR2** | **H_CDR2** | **H_FR3** | **H_CDR3** | **H_FR4** |
| | VH4\|4-59/D7\|7-27\|RF3/JH4 | | 32500 | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAR | NWGY-----------------------FDY | WGQGTLVTVSS |
| iPS:437010 | 21-225_192G3 | VH4\|4-59/D7\|7- | | QVQLQES-GPGLVKPSETLSL | N-----YYWS | WIRQPAGKGLEWIG | RIYS----SGSTNYNPS | RVTMSVDTSKNQFSLKLNSVTAADTAVYYCAK | GWE------------------------LNY | WGQGTLVTVS |

| | | | | | | | | | | |
|---|---|---|---|---|---|---|---|---|---|---|
| iPS:437016 | 21-225_193A6 | VH4\|4-59/D7\|7-27\|RF3/JH4 | | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAG | GWE-------------------------LNY | WGQGTLVTVSS |
| | | Germline | | H_FR1 | H_CDR1 | H_FR2 | H_CDR2 | H_FR3 | H_CDR3 | H_FR4 |
| | VH3\|3-15/D4\|4-17\|RF2/JH4 | | 32501 | EVQLVES-GGGLVKPGGSLRLSCAASG-FTFS | N-----AWMS | WVRQAPGKGLEWVG | RIKSKT-DGGTTDYAAPVKG | RFTISRDDSKNTLYLQMNSLKTEDTAVYYCTT | DYGDY---------------------YFDY | WGQGTLVTVSS |
| iPS:437018 | 21-225_193H5 | VH3\|3-15/D4\|4-17\|RF2/JH4 | | EVQLVES-GGGLVMPGGSLSLSCAASG-FTFS | N-----AYMT | WVRQAPGKGLEWVG | RIKSKT-DGGTTDYAAPVKG | RFTISRDDSKNTLYLQMNSLKTEDTAVYYCTT | DPGG----------------------IFDY | WGQGTLVTVSS |
| iPS:437144 | 21-225_215B3 | VH3\|3-15/D4\|4-17\|RF2/JH4 | | EVQLVES-GGGLVKPGGSLRLSCAASG-FTFS | N-----AWMH | WVRQAPGKGLEWVG | RIKSKT-NGGTTDYAAPVKG | RFTISRDDSKNTLYLQMNSLKTEDTAVYYCTT | DPGG----------------------IFDY | WGQGTLVTVSS |
| | | Germline | | H_FR1 | H_CDR1 | H_FR2 | H_CDR2 | H_FR3 | H_CDR3 | H_FR4 |
| | VH3\|3-11/D3\|3-9\|RF1/JH4 | | 32502 & 32503 | QVQLVES-GGGLVKPGGSLRLSCAASG-FTFS | D-----YYMS | WIRQAPGKGLEWVS | YISSS---GSTIYYADSVKG | RFTISRDNAKNSLYLQMNSLRAEDTAVYYCAR | VLRYFDW------------------LL*YFDY | WGQGTLVTVSS |
| iPS:437030 | 21-225_195E3 | VH3\|3-11/D3\|3-9\|RF1/JH4 | | QVQLVES-GGGLVKPGGSLRLSCAASG-FTFS | D-----YYMS | WIRQAPGKGLEWVS | YITSS---GNTIYYADSVKG | RFTISRDNAKNSLYLQMNSLRAEDTAVYYCAR | DSRYF--------------------DWFDY | WGQGTLVTVSS |
| | | Germline | | H_FR1 | H_CDR1 | H_FR2 | H_CDR2 | H_FR3 | H_CDR3 | H_FR4 |
| | VH4\|4-59/D7\|7-27\|RF3/JH1 | | 32504 | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAR | NWGAE---------------------YFQH | WGQGTLVTVSS |
| iPS:437032 | 21-225_195H6 | VH4\|4-59/D7\|7-27\|RF3/JH1 | | QVQLQES-GPGLVKPSETLSLTCTVSG-GSIR | N-----YYWS | WIRQPAGKGLEWIG | RIYS----SGSTNYNPSLKS | RVSMSVDTSKNQFSLKLSSVTAADTAVYYCTR | GWE-------------------------LNN | WGQGTLVTVSS |
| | | Germline | | H_FR1 | H_CDR1 | H_FR2 | H_CDR2 | H_FR3 | H_CDR3 | H_FR4 |
| | VH4\|4-59/D6\|6-6\|RF1/JH6 | | 32505 | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAR | EYSSSSYY---------------YYYGMDV | WGQGTTVTVSS |
| iPS:437044 | 21-225_197F9 | VH4\|4-59/D6\|6-6\|RF1/JH6 | | QVQLQES-GPGLVKPSETLSLTCTVSG-GSIR | I-----YYWS | WIRQPPGKGLEWIG | YVYY----SGSTTYNPSLKS | RVTISVDTSKNQFSLKLNSVTAADTAVYYCVR | ERGSSHRW--------------GDYYGMDV | WGRGTTVTVSS |
| iPS:437060 | 21-225_199C3 | VH4\|4-59/D6\|6-6\|RF1/JH6 | | QVQLQES-GPGLVKPSETLSLTCTVSG-GSIR | I-----YYWS | WIRQTPGKGLEWIG | YIYY----SGSTTYNPSLKS | RVTISVDTSKNQFSLKLNSVTAADTAVYYCVR | ERGSSHRW--------------GDYYGMDV | WGRGTTVTVSS |
| | | Germline | | H_FR1 | H_CDR1 | H_FR2 | H_CDR2 | H_FR3 | H_CDR3 | H_FR4 |
| | VH3\|3-30/D6\|6- | | 32506 | QVQLVES- | S-----YGMH | WVRQAPGKG | VISYD--- | RFTISRDNSKNTLYLQMNSL | EYSSSS----------- | WGQGT |

| | | | | | | | | | | |
|---|---|---|---|---|---|---|---|---|---|---|
| iPS:437058 | 21-225_199F3 | VH3\|3-30/D6\|6-6\|RF1/JH1 | | QVQLVES-GGGVVQPGRSLRLSCAASG-FTFS | F-----YGMH | WVRQAPGKGLEWVA | VIWYD---GSSKYYADSVKG | RFTVSRDNSKNTLYLQMNSLRAEDTAVYYCAK | EGYSSGF---------------YRGFAN | WGQGTLVTVSS |
| | | Germline | | H_FR1 | H_CDR1 | H_FR2 | H_CDR2 | H_FR3 | H_CDR3 | H_FR4 |
| | VH4\|4-30.1/D6\|6-6\|RF2/JH6 | | 32507 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | SIAARYY---------------YYYGMDV | WGQGTTVTVSS |
| iPS:437062 | 21-225_200H1 | VH4\|4-30.1/D6\|6-6\|RF2/JH6 | | QVQLQES-GPGLVKPSQTLSLTCTVSG-GSIS | SG---GDYWS | WIRQHPGKGLEWIG | YIYY----SGSTYYNPSLKS | RVTISVDTSKNQFSLKLNSVTAADTAVYYCAR | DGAAL---------------------GMDV | WGQGTTVTVSS |
| iPS:437066 | 21-225_200G9 | VH4\|4-30.1/D6\|6-6\|RF2/JH6 | | QVQLQES-GPGLVKPSQTLSLTCTVSG-GSIS | SG---GDYWS | WIRQHPGKGLEWIG | YIYY----SGSTYYNPSLKS | RVTISVDTSKNQFSLKLNSVTAADTAVYYCAR | DGAAL---------------------GMDV | WGQGTTVTVSS |
| iPS:437068 | 21-225_200A11 | VH4\|4-30.1/D6\|6-6\|RF2/JH6 | | QVQLQES-GPGLVKPSQTLSLTCTVSG-GSIS | SG---GDYWS | WIRQHPGKGLEWIG | YIYY----RGSTYYNPSLKS | RVTISVDTSKNQFSLKLSSVTAADTAVYYCAR | DAAAH---------------------GMDV | WGQGTTVTVSS |
| iPS:437140 | 21-225_214E12 | VH4\|4-30.1/D6\|6-6\|RF2/JH6 | | QVQLQES-GPGLVKPSQTLSLTCTVSG-GSIS | SG---GDYWS | WIRQHPGKGLEWIG | YIYY----SGPTYYNPSLKS | RVTISVDTSKNQFSLKLSSVTAADTAVYYCAR | DGAAE---------------------GMDV | WGQGTTVTVSS |
| iPS:437158 | 21-225_216H11 | VH4\|4-30.1/D6\|6-6\|RF2/JH6 | | QVQLQES-GPGLVKPSQTLSLTCTVSG-GSIS | SG---GDYWS | WIRQHPGKGLEWIG | YIYY----SGPTYYNPSLKS | RVTISVDTSKNQFSLKLSSVTAADTAVYYCAR | DGAAE---------------------GLDV | WGQGTTVTVSS |
| | | Germline | | H_FR1 | H_CDR1 | H_FR2 | H_CDR2 | H_FR3 | H_CDR3 | H_FR4 |
| | VH3\|3-33/D1\|1-26\|RF3/JH3 | | 32508 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YSGSYY---------------------DAFDI | WGQGTMVTVSS |
| iPS:437074 | 21-225_203B2 | VH3\|3-33/D1\|1-26\|RF3/JH3 | | QVQLVES-GGGVVQPGRSLRLSCAASG-FTFS | N-----YGMH | WVRQAPGKGLEWVA | IIWFD---GSNEYYADSVKG | RFTISRDNSMSTLYLQMNSLRAEDTAVYYCAR | ESGSY---------------------ALYI | WGQGTMVTVSS |
| iPS:437082 | 21-225_205E12 | VH3\|3-33/D1\|1-26\|RF3/JH3 | | QVQLVES-GGGVVQPGRSLRLSCAASG-FTFS | N-----YGMH | WVRQAPGKGLEWVA | IIWFD---GSNEYYADSVKG | RFTISRDNSMSTLYLQMNSLRAEDTAVYYCAR | ESGSY---------------------ALYI | WGQGTMVTVSS |
| | | Germline | | H_FR1 | H_CDR1 | H_FR2 | H_CDR2 | H_FR3 | H_CDR3 | H_FR4 |
| | VH3\|3-48/D4\|4-17\|RF2/JH4 | | 32509 | EVQLVES-GGGLVQPGGSLRLSCAASG-FTFS | S-----YSMN | WVRQAPGKGLEWVS | YISSS---SSTIYYADSVKG | RFTISRDNAKNSLYLQMNSLRDEDTAVYYCAR | DYGDY---------------------YFDY | WGQGTLVTVSS |
| iPS:437086 | 21-225_209A8 | VH3\|3-48/D4\|4- | | EVQLVES-GGGLVQPGGSLRL | S-----YSMN | WVRQAPGKGLEWVL | YISSS---SSIKKYADS | RFTISRDNAKNSLYLQMNSLRDEDTAVYYCVR | DDGSY---------------------YFDY | WGQGTLVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH4\|4-30.1/D2\|2-8\|RF3/JH6 | | 32510 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | DIVLMVYAI-------------YYYYYGMDV | WGQGTTVTVSS |
| iPS:437090 | 21-225_210F11 | VH4\|4-30.1/D2\|2-8\|RF3/JH6 | | QVQLQES-GPGLVKPSQTLSLTCTVSG-GSIS | SG---GSYWS | WIRQHPGKGLEWIG | YIYY----IGTTYYNPSLKS | RVTISVDTSTNHFSLKLSSVTAADTAVYYCAR | DEPLT---------------------GMDV | WGQGTTVTVSS |
| iPS:437106 | 21-225_211H7 | VH4\|4-30.1/D2\|2-8\|RF3/JH6 | | QVQLQES-GPGLVKPSQTLSLTCTVSG-GSIS | SG---GYYWS | WTRQHPGKGLEWIG | YIYY----VGSTYYNPSLKS | RVTISVDTSKNQFSLKLSSVTAADTAVYYCAR | DGPLS---------------------GMDV | WGQGTTVTVSS |
| | | Germline | | H_FR1 | H_CDR1 | H_FR2 | H_CDR2 | H_FR3 | H_CDR3 | H_FR4 |
| | VH3\|3-30.3/D4\|4-17\|RF2/JH5 | | 32511 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | DYGDY--------------------NWFDP | WGQGTLVTVSS |
| iPS:437100 | 21-225_211H2 | VH3\|3-30.3/D4\|4-17\|RF2/JH5 | | QVQLVES-GGGVVQPGRSLRLSCAASG-FTFS | S-----YAMH | WVRQAPGKGLEWVA | VIWYD---GSNKYYADSVKG | RFTIARDNSKNTLYLQMNSLRAEDTAVYYCAR | DPGSY---------------------GFDP | WGQGTLVTVSS |
| | | Germline | | H_FR1 | H_CDR1 | H_FR2 | H_CDR2 | H_FR3 | H_CDR3 | H_FR4 |
| | VH3\|3-33/D5\|5-12\|RF3/JH6 | | 32512 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | GYSGYDYY--------------YYYYGMDV | WGQGTTVTVSS |
| iPS:437102 | 21-225_211E5 | VH3\|3-33/D5\|5-12\|RF3/JH6 | | QVQLVES-GGGVVQPGRSLRLSCAASG-FTFR | N-----YGMH | WVRQAPGKGLEWVS | IIWFD---GSDQYYADSVKG | RFTISRDNSKNTLYLQMNSLRAEDTAVYYCAR | GLSVYY-------------------YGMGV | WGQGTTVTVSS |
| iPS:437164 | 21-225_217C6 | VH3\|3-33/D5\|5-12\|RF3/JH6 | | QVQLVES-GGGVVQPGRSLRLSCAASG-FTFS | N-----YGMH | WVRQAPGKGLEWMA | IIWFD---GSDEYYADSVKG | RFTISRDNSKNTMYLQMNSLRAEDTAVYYCAR | GLSVYY-------------------YGMDV | WGQGTTVTVSS |
| iPS:437166 | 21-225_217G11 | VH3\|3-33/D5\|5-12\|RF3/JH6 | | QVQLVES-GGGVVQPGRSLRLSCAASG-FTFR | N-----YGMH | WVRQAPGKGLEWVS | IIWFD---GSDQYYADSVKG | RFTISRDNSKNTLYLQMNSLRAEDTAVYYCAR | GLSVYY-------------------YGMDV | WGQGTTVTVSS |
| iPS:437170 | 21-225_218E5 | VH3\|3-33/D5\|5-12\|RF3/JH6 | | QVQLVES-GGGVVQPGRSLRLSCAASG-FTFS | N-----YGMH | WVRQAPGKGLEWMA | IIWFD---GSDEYYADSVKG | RFTISRDNSKNTLYLQMNSLRAEDTAVYYCAR | GLSVYY-------------------YGMDV | WGQGTTVTVSS |
| | | Germline | | H_FR1 | H_CDR1 | H_FR2 | H_CDR2 | H_FR3 | H_CDR3 | H_FR4 |
| | VH4\|4-30.1/D6\|6-19\|RF2/JH6 | | 32513 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | GIAVAGYY---------------YYYGMDV | WGQGTTVTVSS |

| | | | | | | | | | | |
|---|---|---|---|---|---|---|---|---|---|---|
| iPS:4 37108 | 21-225_211C9 | VH4\|4-30.1/D6\|6-19\|RF2/JH6 | | QVQLQES-GPGLVKPSQTLSLTCTVSG-GSIR | SG---GDYWS | WIRQHPGKGLEWIG | YIYY----SGSTYYNPSLKS | RVTILLDTSKNQFSLKLSSVTVADTAVYYCAR | DSAVY-----------<br>----------NMDV | WGQGTTVTVSS |
| iPS:4 37110 | 21-225_211E9 | VH4\|4-30.1/D6\|6-19\|RF2/JH6 | | QVQLQES-GPGLVKPSQTLSLTCTVSG-GSIS | SG---GDYWS | WIRQHPGKGLEWIG | YIYY----TGSNYYNPSLKS | RVTISVDTSKNQFSLNLISVTAADTAVYYCAR | DSAVY-----------<br>----------GMDV | WGQGTTVTVSS |
| iPS:4 37120 | 21-225_212A9 | VH4\|4-30.1/D6\|6-19\|RF2/JH6 | | QVQLQES-GPGLVKPSQTLSLTCSVSG-GSIR | SG---GDYWS | WIRQHPGKGLEWIG | YMYY----SGSTYYNPSLKS | RVTISVDTSKNQFSLKLSSVTVADTAVYYCAR | DSAVY-----------<br>----------GMDV | WGQGTTVTVSS |
| iPS:4 37132 | 21-225_213F5 | VH4\|4-30.1/D6\|6-19\|RF2/JH6 | | QVQLQES-GPGLVKPSQTLSLTCTVSG-GSIR | SG---GDYWS | WIRQHPGKGLEWIG | YIYY----SGSTYYNPSLKS | RVTISLDTSKNQFSLKLSSVTVADTAVYYCAR | DSAVY-----------<br>----------NMDV | WGQGTTVTVSS |
| iPS:4 37142 | 21-225_215A3 | VH4\|4-30.1/D6\|6-19\|RF2/JH6 | | QVQLQES-GPGLVKPSQTLSLTCTVSG-GSIS | SG---GDYWS | WIRQHPGKGLEWIG | YIYY----TGSNYYNPSLKS | RVTISVDTSKNQFSLKVISVTAADTAVYYCAR | DSAVY-----------<br>----------GMDV | WGQGTTVTVSS |
| iPS:4 37148 | 21-225_215H3 | VH4\|4-30.1/D6\|6-19\|RF2/JH6 | | QVQLQES-GPGLVKPSQTLSLTCSVSG-GSIR | SG---GDYWS | WIRQHPGKGLEWIG | YMYY----SGSTYYNPSLKS | RVTISVDTSKNQFSLKLSSVTVADTAVYYCAR | DSAVY-----------<br>----------GMDV | WGQGTTVTVSS |
| iPS:4 37154 | 21-225_216A7 | VH4\|4-30.1/D6\|6-19\|RF2/JH6 | | QVQLQES-GPGLVKPSQTLSLTCSVSG-GSIR | SG---GDYWS | WIRQHPGKGLEWIG | YMYY----SGSTYYNPSLKS | RVTISVDTSKNQFSLKLSSVTVADTAVYYCAR | DSAVY-----------<br>----------GMDV | WGQGTTVTVSS |
| | | Germline | | H_FR1 | H_CDR1 | H_FR2 | H_CDR2 | H_FR3 | H_CDR3 | H_FR4 |
| | VH3\|3-30.3/D7\|7-27\|RF2/JH4 | | 32514 & 32515 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | *LGY------------<br>-----------FDY | WGQGTLVTVSS |
| iPS:4 37160 | 21-225_216B12 | VH3\|3-30.3/D7\|7-27\|RF2/JH4 | | QVQLVES-GGGVVQPGRSLRLSCAASG-FTFS | S-----YAMH | WVRQAPGKGLEWVA | VIWYD---GSNKYYADSVKG | RFTISRDNSKNTLYLQMNSLRAEDTAVYYCAR | DLGLG-----------<br>---------YFFDY | WGQGTLVTVSS |
| | | Germline | | H_FR1 | H_CDR1 | H_FR2 | H_CDR2 | H_FR3 | H_CDR3 | H_FR4 |
| | VH6\|6-01/D2\|2-21\|RF2/JH5 | | 32516 | QVQLQQS-GPGLVKPSQTLSLTCAISG-DSVS | SN---SAAWN | WIRQSPSRGLEWLG | RTYYR--SKWYNDYAVSVKS | RITINPDTSKNQFSLQLNSVTPEDTAVYYCAR | AYCGGDC---------<br>-------YSNWFDP | WGQGTLVTVSS |
| iPS:4 37186 | 21-225_224H2 | VH6\|6-01/D2\|2-21\|RF2/JH5 | | QVQLQQS-GPGLVKPSQTLSLTCDISG-DSVS | SN---SAAWN | WIRQSPSRGLEWLG | RTYYR--SKWYNDYAVSVKS | RVTINPDTSKNQFSLQLNSVTPEDTAVYYCAR | EGGLGYCSST------<br>-----SCYGGWFDP | WGQGTLVTVSS |
| | | Germline | | H_FR1 | H_CDR1 | H_FR2 | H_CDR2 | H_FR3 | H_CDR3 | H_FR4 |
| | VH1\|1-02/D4\|4- | | 32517 | QVQLVQS- | G-----YYMH | WVRQAPGQ | WINPN--- | RVTMTRDTSISTAYMELSRL | DYGDYYY--------- | WGQGT |

| | | | | | | | | | | |
|---|---|---|---|---|---|---|---|---|---|---|
| iPS:4 37188 | 21-225_224B11 | VH1\|1-02/D4\|4-17\|RF2/JH6 | | QVHLVQS-GAEVKKPGASVKVSCKASG-YTFT | G-----YYIH | WVRQAPGQGLEWMG | WINPK---NGGTNYAQKFQG | RVTMTRDASISTTYMELSRLRSDDTAVYYCAR | GAFDYFY-----------------YYAMDV | WGHGTTVTVSS |
| iPS:4 37198 | 21-225_226F8 | VH1\|1-02/D4\|4-17\|RF2/JH6 | | QVQLVQS-GAEVKKPGASVKVSCKASG-YTFT | G-----YYIH | WVRQAPGQGLEWMG | WINPN---SGGTNYARKFQG | RVTMTRDTSISTAYMELSRLRSDDTAVYYCAR | GAFDYYY-----------------YYALDV | WGQGTTVTVSS |
| iPS:4 37202 | 21-225_227D3 | VH1\|1-02/D4\|4-17\|RF2/JH6 | | QVQLVQS-GAEVKKPGASVKVSCKASG-YTFT | G-----YYMH | WVRQAPGQGLERMG | WINPK---SGGTNFAQKFQG | RVTMTRDTSISTAYMELSRLRSDDTAVYYCAR | GAFDYFY-----------------YYGMDV | WGQGTTVTVSS |
| iPS:4 37208 | 21-225_227C10 | VH1\|1-02/D4\|4-17\|RF2/JH6 | | QVQLVQS-GAEVKKPGASVKVSCKASG-YTFT | G-----YYMH | WVRQAPGQGLERMG | WINPK---SGGTNYAQKFQG | RVTMTRDTSISTAYMELSRLRSDDTAVYYCAR | GAFDYFY-----------------YYGMDV | WGQGTTVTVSS |
| | | **Germline** | | **H_FR1** | **H_CDR1** | **H_FR2** | **H_CDR2** | **H_FR3** | **H_CDR3** | **H_FR4** |
| | VH3\|3-33/D2\|2-2\|RF2/JH6 | | 32518 | QVQLVES-GGGVVQPGRSLRLSCAASG-FIFS | S-----YGMH | WVRQAPGKGLEWVA | VIWYD---GSNKYYADSVKG | RFTISRDNSKNTLYLQMNSLRAEDTAVYYCAR | GYCSSTSCYT-----------YYYYGMDV | WGQGTTVTVSS |
| iPS:4 37192 | 21-225_225E9 | VH3\|3-33/D2\|2-2\|RF2/JH6 | | QVQLVES-GGGVVQPGRSLRLSCEASG-FIFS | S-----YGMH | WVRQAPGKGLEWVA | VMWYD---GGNKDYADSVKG | RFTISRDNSKNTLYLQMNSLRAEDTAVYYCAR | DREYCTSTSC----------PYYYYYGMDV | WGQGTTVTVSS |
| | | **Germline** | | **H_FR1** | **H_CDR1** | **H_FR2** | **H_CDR2** | **H_FR3** | **H_CDR3** | **H_FR4** |
| | VH3\|3-23/D2\|2-21\|RF2/JH6 | | 32519 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | AYCGGDCYS------------YYYYGMDV | WGQGTTVTVSS |
| iPS:4 37204 | 21-225_227E5 | VH3\|3-23/D2\|2-21\|RF2/JH6 | | EVQLLES-GGGLVQPEGSLRLSCAASG-FTFS | S-----YAMS | WVRQAPGKGLEWVS | AISGS---GGSTYYADSVKG | RFTISRDNSKNTLCLQMNSLRAEDTAVYYCAK | EYCGGDCYSP-----------YYYYGMDV | WGQGTTVTVSS |
| iPS:3 93196 | 21-225_16G8 | VH3\|3-23/D2\|2-21\|RF2/JH6 | | EVQLLES-GGGLVQPEGSLRLSCAASG-FTFS | S-----YAMS | WVRQAPGKGLEWVS | GISGS---GGSTYYADSVKG | RFTISRDNSKNTLCLHMNSLRAEDTAVYYCAK | EYCGGDCYSP-----------YYYYGMDV | WGQGTTVTVSS |
| iPS:3 93202 | 21-225_6B4 | VH3\|3-23/D2\|2-21\|RF2/JH6 | | EVQLLES-GGGLVQPEGSLRLSCAASG-FTFS | S-----YAMS | WVRQAPGKGLEWVS | AISGS---GSSTYYADSVKG | RFTISRDNSKNTLCLQMNSLRAEDTAVYYCAK | EYCGGDCYSP-----------YYYYGMDV | WGQGTTVTVSS |
| iPS:3 93345 | 21-225_5G7 | VH3\|3-23/D2\|2-21\|RF2/JH6 | | EVQLLES-GGGLVQPEGSLRLSCAASG-FTFS | S-----YAMS | WVRQAPGKGLEWVS | AISGS---GGSTYYADSVKG | RFTISRDNSKNTLCLQMNSLRAEDTAVYYCAK | EYCGGDCYSP-----------YYYYGMDV | WGQGTTVTVSS |
| | | **Germline** | | **H_FR1** | **H_CDR1** | **H_FR2** | **H_CDR2** | **H_FR3** | **H_CDR3** | **H_FR4** |
| | VH2\|2-05/D6\|6- | | 32520 & | QITLKES- | TS---GVGVG | WIRQPPGK | LIYW---- | RLTITKDTSKNQVVLTMTNM | V*QQL------------ | WGQGT |

| | | | | | | | | | | |
|---|---|---|---|---|---|---|---|---|---|---|
| iPS:4 37210 | 21-225_227E12 | VH2\|2-05/D6\|6-13\|RF3/JH4 | | QITLKES-GPTLVKPTQTLTLTCTFSG-FSLS | TS---GVGVG | WIRQPPGKALECLS | LIYW----NDDKVYSPSLKS | RLTITKYTSKNQVVLTMTNMDPVDTATYYCAH | RGQQL--------------------ALDY | WGQGTLVTVSS |
| iPS:3 92587 | 21-225_18G5 | VH2\|2-05/D6\|6-13\|RF3/JH4 | | QITLKES-GPTLVKPTQTLTLTCTFSG-FSLS | TS---GVGVG | WIRQPPGKALECLS | LIYW----NDDKVYSPSLKS | RLTITKYTSKNQVVLTMTNMDPVDTATYYCAH | RGQQL--------------------ALDY | WGQGTLVTVSS |
| iPS:3 98504 | 21-225_23D7 | VH2\|2-05/D6\|6-13\|RF3/JH4 | | QITLKES-GPTLVKPTQTLTLTCTFSG-FSLS | TS---GVGVG | WIRQPPGKALECLS | LIYW----NNDKVYSPSLKS | RLTITKYTSKNQVVLTMSNMDPVDTATYYCAH | RGQQL--------------------ALDY | WGQGTLVTVSS |
| | | Germline | | H_FR1 | H_CDR1 | H_FR2 | H_CDR2 | H_FR3 | H_CDR3 | H_FR4 |
| | VH3\|3-21/D4\|4-11\|RF2/JH6 | | 32522 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | DYSNYYY----------------YYYGMDV | WGQGTTVTVSS |
| iPS:4 37226 | 21-225_57C2 | VH3\|3-21/D4\|4-11\|RF2/JH6 | | EVQLVES-GGGLVKPGGSLRLSCAASG-FTFS | S-----FGMN | WVRQAPGKGLEWVS | SISSS---TGYIYNADSVKG | RFTISRDNAKNSLYLQMNSLRAEDTAVYYCAR | TYSG--------------------SLDV | WGQGTTVTVSS |
| | | Germline | | H_FR1 | H_CDR1 | H_FR2 | H_CDR2 | H_FR3 | H_CDR3 | H_FR4 |
| | VH3\|3-21/D5\|5-18\|RF3/JH5 | | 32523 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | GYSYGY--------------------NWFDP | WGQGTLVTVSS |
| iPS:4 37230 | 21-225_62H10 | VH3\|3-21/D5\|5-18\|RF3/JH5 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | GGSR----------------------GFDP | WGQGTLVTVSS |
| iPS:4 48906 | 21-225_72G9 | VH3\|3-21/D5\|5-18\|RF3/JH5 | | EVQLVES-GGGLVKPGGSLRLSCAASG-FTFS | S-----YSMN | WVRQAPGKGLEWVS | SISGS---SSYIYYADSVKG | RFTISRDNAKNSLYLQMNSLRAEDTAVYFCAR | GGSR----------------------GFDP | WGQGTLVTVSS |
| | | Germline | | H_FR1 | H_CDR1 | H_FR2 | H_CDR2 | H_FR3 | H_CDR3 | H_FR4 |
| | VH1\|1-02/D1\|1-26\|RF1/JH4 | | 32524 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | GIVGA--------------------TYFDY | WGQGTLVTVSS |
| iPS:4 37260 | 21-225_170D1 | VH1\|1-02/D1\|1-26\|RF1/JH4 | | QVQLVQS-GAEVKKPGASVKVSCKASG-YTFT | G-----YFMH | WVRQAPGQGLEWMG | WIKPK---SGGTNCAQKFQG | RVTMTRDTSSSTAYMELSRLTSDDTAVYYCAR | GGATVTT------------------WGVFDY | WGQGTLVTVSS |
| | | Germline | | H_FR1 | H_CDR1 | H_FR2 | H_CDR2 | H_FR3 | H_CDR3 | H_FR4 |
| | VH3\|3-33/D2\|2-21\|RF2/JH6 | | 32525 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | AYCGGDCYS-------------YYYYYGMDV | WGQGTTVTVSS |
| iPS:4 37268 | 21-225_177D2 | VH3\|3-33/D2\|2- | | QVQLVES-GGGVVQPGRSLRL | S-----YGMD | WVRQAPGKGLEWVA | IIWFD---GSNKYYADS | RFTISRDNSKNTLYLQMNSLRAEDTAVYYCAR | AYCGGDCYFP-----------HLHYYGMDV | WGQGTTVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH4\|4-30.1/D4\|4-17\|RF2/JH4 | | 32526 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | DYGDY-----------------------YFDY | WGQGTLVTVSS |
| iPS:437294 | 21-225_216D5 | VH4\|4-30.1/D4\|4-17\|RF2/JH4 | | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLNSVTAADTAVYFCAR | DSPDR---------------------GFDY | WGQGTLVTVSS |
| | | Germline | | H_FR1 | H_CDR1 | H_FR2 | H_CDR2 | H_FR3 | H_CDR3 | H_FR4 |
| | VH3\|3-30.3/D5\|5-24\|RF3/JH6 | | 32527 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | RDGYNYYY---------------YYYGMDV | WGQGTTVTVSS |
| iPS:437302 | 21-225_225B11 | VH3\|3-30.3/D5\|5-24\|RF3/JH6 | | QVQLVES-GGGVVQPGRSLRLSCAASG-FTFS | S-----YGMH | WVRQAPGKGLEWVT | IISYS---GSNKYYADSVKG | RFTISRDNSKNTLYLQMNSLRAEDTAVYYCAR | RGYSYG------------------GYGMDV | WGQGTTVTVSS |
| iPS:451102 | 21-225_45F6 | VH3\|3-30.3/D5\|5-24\|RF3/JH6 | | QVQLVES-GGGVVQPGRSLRLSCAASG-FTFS | Y-----YGLH | WVRQAPGKGLEWVA | VISYD---GSNKYYADSVKG | RFTISRDNSKNTLYLQMNNLRAEDTAVFYCAR | EDRYCSGTSC----------PYYYYYGMDV | WGQGTTVTVSS |
| iPS:392868 | 21-225_24D6 | VH3\|3-30.3/D5\|5-24\|RF3/JH6 | | QVQLVES-GGGVVQPGRSLRLSCAASG-FTFS | S-----YGMH | WVRQVPGKGLEWVA | IISYA---GSNKSYADSVKG | RFTISRDNSKNTLYLQMNSLRAEDTAVYYCVR | RGYSYG------------------GYGMDV | WGQGATVTVSS |
| iPS:393910 | 21-225_15F10 | VH3\|3-30.3/D5\|5-24\|RF3/JH6 | | QVQLVES-GGGVVQPGRSLRLSCAASG-FTFS | S-----YGMH | WVRQAPGKGLEWVS | VISYG---GSNNYYADSVKG | RFTISRDNSKNTLYLQMNSLRAEDTAVYYCVR | RGYSYG------------------GYGMDV | WGQGTTVTVSS |
| iPS:394000 | 21-225_11A2 | VH3\|3-30.3/D5\|5-24\|RF3/JH6 | | QVQLVES-GGGVVQPGRSLRLSCAASG-FTFS | S-----YGMH | WVRQAPGKGLEWVA | VISYG---GSNKDSADSVKG | RFIISRDNSKNTLYLQMNSLRAEDTAVYYCVR | RGYSYG------------------GYGMDV | WGQGTTVTVSS |
| iPS:394004 | 21-225_13A1 | VH3\|3-30.3/D5\|5-24\|RF3/JH6 | | QVQLVES-GGGVVQPGRSLRLSCAASG-FTFS | S-----YGMH | WVRQAPGKGLEWVA | VISYA---GTNQYYADSVKG | RFTISRDNSKNTLYLQMNSLRAEDTAVYYCVR | RGYSYG------------------GYGMDV | WGQGTTVTVSS |
| iPS:394006 | 21-225_15C2 | VH3\|3-30.3/D5\|5-24\|RF3/JH6 | | QVQLVES-GGGVVQPGRSLRLSCAASG-FTFS | S-----YGMH | WVRQAPGKGLEWVS | IISYG---GRNNHYADSVKG | RFTISRDNSKNTLYLQMNSLRAEDTAVYYCVR | RGYSYG------------------GYGMDV | WGQGTTVTVSS |
| iPS:394029 | 21-225_1B12 | VH3\|3-30.3/D5\|5-24\|RF3/JH6 | | QVQLVES-GGGVVQPGRSLRLSCAASG-FTFS | S-----YGMH | WVRQAPGKGLEWVA | IISYA---GSNKSYADSVKG | RFTISRDNSKNMLYLQMNSLRAEDTAVYYCVR | RGYSYG------------------GYGMDV | WGQGATVTVSS |
| iPS:394047 | 21-225_5E6 | VH3\|3-30.3/D5\|5- | | QVQLVES-GGGVVQPGRSLRL | S-----YGMH | WVRQAPGKGLEWVA | IISYV---GNNKYYADS | RFTISRDNSKNTLYLQMNSLRVEDTAVYYCAR | RGYSYG------------------GYGMDV | WGQGTTVTVS |

| | | | | | | | | | |
|---|---|---|---|---|---|---|---|---|---|
| iPS:394081 | 21-225_16B3 | VH3\|3-30.3/D5\|5-24\|RF3/JH6 | | QVQLVES-GGGVVQPGRSLRLSCVASG-FTFS | S-----YGMH | WVRQAPGKGLEWVA | VISYA---GINKSYADSVKG | RFTISRDNSNNTLYLQMNSLRAEDTAVYYCVR | RGYSYG-----------------GYGMDV | WGQGATVTVSS |
| | | Germline | | H_FR1 | H_CDR1 | H_FR2 | H_CDR2 | H_FR3 | H_CDR3 | H_FR4 |
| | VH4\|4-61/D3\|3-9\|RF1/JH4 | | 32528 & 32529 | QVQLQES-GPGLVKPSETLSLTCTVSG-GSVS | SG---SYYWS | WIRQPPGKGLEWIG | YIYY----SGSTNYNPSLKS | RVTISVDTSKNQFSLKLSSVTAADTAVYYCAR | VLRYFDW---------------LL*YFDY | WGQGTLVTVSS |
| iPS:451118 | 21-225_191C8 | VH4\|4-61/D3\|3-9\|RF1/JH4 | | QVQLQES-GPGLVKPSETLSLTCTVSS-GSVS | SG---GYYWS | WIRQPPGKGLEWIG | YIYY----SGTTIYNPSLKS | RVTISVDTSKNQFSLKLTSVTVADTAVYYCAR | DTFCFDG----------------CGYFFDS | WGQGTLVTVSS |
| | | Germline | | H_FR1 | H_CDR1 | H_FR2 | H_CDR2 | H_FR3 | H_CDR3 | H_FR4 |
| | VH2\|2-05/D4\|4-11\|RF3/JH4 | | 32530 | QITLKES-GPTLVKPTQTLTLTCTFSG-FSLS | TS---GVGVG | WIRQPPGKALEWLA | LIYW----NDDKRYSPSLKS | RLTITKDTSKNQVVLTMTNMDPVDTATYYCAH | TTVT---------------------YFDY | WGQGTLVTVSS |
| iPS:392573 | 21-225_15G2 | VH2\|2-05/D4\|4-11\|RF3/JH4 | | QITLKES-GPTLVKPTQTLTLTCTFSG-FSLS | TS---GVGVG | WIRQPPGKALEWLA | LIYW----NDDKRYSPSLKS | RLTITKDTSKNQVVLTMTNMDPVDTATYYCAD | TGVSC--------------------CYFHY | WGQGTLVTVSS |
| | | Germline | | H_FR1 | H_CDR1 | H_FR2 | H_CDR2 | H_FR3 | H_CDR3 | H_FR4 |
| | VH1\|1-02/D2\|2-2\|RF2/JH6 | | 32531 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | GYCSSTSCYT-----------YYYYGMDV | WGQGTTVTVSS |
| iPS:392585 | 21-225_14H11 | VH1\|1-02/D2\|2-2\|RF2/JH6 | | QVQLVQS-GAEVKKPGASVKVSCQGSG-YTFT | G-----HYMC | WVRQAPGQGLEWMG | WINPN---SGGTNYAQKFQG | RVTMTRDTSISTAYMELSRLRSDDTAVYYCAA | GYCSSSSCYL-----------QPGYYGMDV | WGQGTTVTVSS |
| iPS:393186 | 21-225_27D9 | VH1\|1-02/D2\|2-2\|RF2/JH6 | | QVQLVQS-GAEVKKPGASVKVSCKASG-YTFT | G-----YYMH | WVRQAPGQGLEWMG | WINPN---SGGTKYAQKFQG | RVTMTRDTSISTAYMELNRLRSDDTAVYYCAR | ERCSTTSCYL----------GITGYYGMDV | WGQGTTVTVSS |
| iPS:393234 | 21-225_26C10 | VH1\|1-02/D2\|2-2\|RF2/JH6 | | QVQLVQS-GAEVKKPGASVKVSCKASG-YTFT | G-----YYVH | WVRQAPGQGLEWMG | WINPN---SGGTNYAQKFQG | RVTMTRDTSISTAYMELSRLRSDDTAVYYCAR | ERCSTTSCYL----------GITGYYGMDV | WGQGTTVTVSS |
| | | Germline | | H_FR1 | H_CDR1 | H_FR2 | H_CDR2 | H_FR3 | H_CDR3 | H_FR4 |
| | VH3\|3-23/D5\|5-12\|RF3/JH6 | | 32532 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GYSGYDYY-------------YYYYGMDV | WGQGTTVTVSS |
| iPS:392596 | 21-225_12D8 | VH3\|3-23/D5\|5-12\|RF3/JH6 | | EVQLLES-GGGLVQPGGSLRLSCAASG-FTFS | S-----YVMS | WVRQAPGKGLEWVS | TISVG---GGSTYYADSVKG | RFTISRDNSKTTLYLQMNSLRAEDTAVYYCAK | WGRGYSYE---------------YYYGMDV | WGQGTTVTVSS |
| iPS:392942 | 21-225_30E9 | VH3\|3-23/D5\|5- | | EVQVLES-GGGLVQPGGSLRL | S-----CAMN | WVRQAPGKGLEWVS | AISGR---GGSTFYADS | RFTISRDNSKNTLYLQMNSLRAEDTAVYYCAK | GELLEDY---------------YYYGMDV | WGQGTTVTVS |

| | | | | | | | | | | |
|---|---|---|---|---|---|---|---|---|---|---|
| **iPS:392944** | **21-225_31H5** | **VH3\|3-23/D5\|5-12\|RF3/JH6** | | EVQLLES-GGGLVQPGGSLRLSCAASG-FTFS | S-----YAMS | WVRQAPGKGLEWVS | AISGR---GGSIFHADSVKG | RFTISRDNSKNTLYLQMNSLRAEDTAVYYCVK | GELLEDY----------------YFYGMDV | WGQGTTVTVSS |
| **iPS:392964** | **21-225_31A8** | **VH3\|3-23/D5\|5-12\|RF3/JH6** | | EVQLLES-GGGLVQPGGSLRLSCAASG-FTFS | S-----YAMS | WVRQAPGKGLEWVS | AISGR---GGSTFHADSVKG | RFTISRDNSKNTLYLQMNSLRDEDTAVYYCVK | GELLEDY----------------YFYGMDV | WGQGTTVTVSS |
| **iPS:392982** | **21-225_30D1** | **VH3\|3-23/D5\|5-12\|RF3/JH6** | | EVQVLES-GGGLVQPGGSLRLSCAASG-FTFS | S-----YAMS | WVRQAPGKGLEWVS | AISGR---GGSTFHADSVKG | RFTISRDNSKNTLYLQMNSLRAEDTAVYYCVK | GELLEDY----------------YFYGLDV | WGQGTTVTVSS |
| **iPS:392986** | **21-225_31B8** | **VH3\|3-23/D5\|5-12\|RF3/JH6** | | EVQLLES-GGGLVQPGGSLRLSCAASG-FTFS | S-----YAMS | WVRQAPGKGLEWVS | AISGR---GGSTFHADSVKG | RFTISRDNSKNTLYLQLNSLRADDTAVYYCVK | GELLEDY----------------YFYGMDV | WGQGTTVTVSS |
| **iPS:393004** | **21-225_30G11** | **VH3\|3-23/D5\|5-12\|RF3/JH6** | | EVQLLES-GGGLVQPGGSLRLSCAASG-FTFS | S-----YAMS | WVRQAPGKGLEWVS | AISGR---GGSTFNADSVKG | RFTISRDNSKTTLYLQMNSLRAEDTAVYYCVK | GELLEDY----------------YFYGMDV | WGQGTTVTVSS |
| **iPS:393040** | **21-225_30E3** | **VH3\|3-23/D5\|5-12\|RF3/JH6** | | EVQVLES-GGGLVQPGGSLRLSCAASG-FTFS | S-----YAMN | WVRQAPGKGLEWVS | AISGR---GGSTFYADSEKG | RFTISRDNSKNTLYLQMNSLRAEDTALYYCAK | GELLEDY----------------YYYGMDV | WGQGTTVTVSS |
| **iPS:393058** | **21-225_31H3** | **VH3\|3-23/D5\|5-12\|RF3/JH6** | | EVQVLES-GGGLVQPGGSLRLSCAASG-FTFS | S-----YAMN | WVRQAPGKGLEWVS | AISGR---GGNTFYADSVKG | RFTISRDNSKNTLYLQMNSLRAEDTAVYYCAK | GELLEDY----------------YYYGMDV | WGQGTTVTVSS |
| **iPS:393060** | **21-225_32G12** | **VH3\|3-23/D5\|5-12\|RF3/JH6** | | EVQLLES-GGGLLQPGGSLRLSCAASG-FTFN | S-----YAMS | WVRQAPGKGLEWVS | SISGR---GGSTFHADSVKG | RFTISRDNSRNTLYLQMNSLRAEDTAVYYCVK | GELLEDY----------------YFYGMDV | WGQGTTVTVSS |
| **iPS:393068** | **21-225_34G9** | **VH3\|3-23/D5\|5-12\|RF3/JH6** | | EVQLVES-GGGLVQPGGSLRLSCAASG-FTFS | S-----YAMS | WVRQAPGKGLEWVS | AISGR---GGSTFHADSVKG | RFTISRDNSKNTLYLQMNSLRAEDTAVYYCVK | GELLEDY----------------YFYGMDV | WGQGTTVTVSS |
| **iPS:393072** | **21-225_36C5** | **VH3\|3-23/D5\|5-12\|RF3/JH6** | | EVQLVES-GGGLVQPGGSLRLSCAASG-FTFS | S-----YAMN | WVRQAPGKGLEWVS | AISGR---GGSTFHADSVKG | RFTISRDNSKNTLYLQMNSLRAEDTAVYYCVK | GELLEDY----------------YFYGMDV | WGQGTTVTVSS |
| **iPS:393076** | **21-225_33A4** | **VH3\|3-23/D5\|5-12\|RF3/JH6** | | EVQLLES-GGGLVQSGGSLRLSCEASG-FIFS | S-----YAMN | WVRQVPGKGLEWVS | AISRR---GGSTFYADSVKG | RFTISRDNSKNTLYLQMNSLRAEDTAVYFCAK | GELLEDY----------------SYYGIDV | WGQGTTVTVSS |
| **iPS:393102** | **21-225_33F1** | **VH3\|3-23/D5\|5-** | | EVQLLES-GGGLLQPGGSLRL | S-----YAMS | WVRQAPGKGLEWVS | SISGR---GGSTFHADS | RFTISRDNSKNTLYLQMNSLRAEDTAVYYCVK | GELLEDY----------------YFYGMDV | WGQGTTVTVS |

| | | | | | | | | | | |
|---|---|---|---|---|---|---|---|---|---|---|
| **iPS:3 93104** | **21-225_33A7** | **VH3\|3-23/D5\|5-12\|RF3/JH6** | | EVQLLES-GGGLVQPGGSLRLSCAASG-FTFS | S-----CAMS | WVRQAPGKGLEWVS | AISGR---GGSTFHADSVKG | RFTISRDNSKNTLYLQMNSLRAEDTAVYYCVK | GELLEDY---------------YFYGMDV | WGQGTTVTVSS |
| **iPS:3 93106** | **21-225_34A6** | **VH3\|3-23/D5\|5-12\|RF3/JH6** | | EVQLLES-GGGLVQPGGSLRLSCAASG-FTFN | N-----YAMN | WVRQAPGKGLEWVS | AISRR---GGSTFYADSVKG | RFTISRDNSKNTLYLQMNSLRAEDTAVYYCAK | GELLEDY---------------YYFAMDV | WGQGTTVTVSS |
| **iPS:3 93110** | **21-225_35B7** | **VH3\|3-23/D5\|5-12\|RF3/JH6** | | EVQLLES-GGGLVQPGGSLRLSCAASG-FTFS | S-----YAMS | WVRQAPGKGLEWVS | AISGR---GGSTFHADSVKG | RFTISRDNSKNTLYLQMNSLRAEDTAVYYCVK | GELLEDY---------------YFYGMDV | WGQGATVTVSS |
| **iPS:3 93118** | **21-225_34H11** | **VH3\|3-23/D5\|5-12\|RF3/JH6** | | EVQLLES-GGGLVQPGGSLRLSCAASG-FTFS | S-----YAMN | WVRQAPGEGLEWVS | AISGR---GGSTFYADSVKG | RFTISRDNSKNTLFLQMNSLRAEDTAVYYCAK | GELLEDY---------------YYYGMDV | WGQGTTVTVSS |
| **iPS:3 93124** | **21-225_33G7** | **VH3\|3-23/D5\|5-12\|RF3/JH6** | | EVQLLES-GGGLVQPGGSLRLSCAASG-FTFS | N-----YAMS | WVRQAPGKGLEWVS | AISRR---GGSTFYADSVKG | QFTISRDNSKNTLYLQMNSLRAEDTAVYYCAK | GELLEDY---------------SYYGMDV | WGQGTTVTVSS |
| **iPS:3 93126** | **21-225_35D1** | **VH3\|3-23/D5\|5-12\|RF3/JH6** | | EVQLLES-GGGLVQPGGSLRLSCAASG-FTFS | S-----YAMS | WVRQAPGKGLEWVS | AISGR---GGSTFHADSVKG | RFTISRDNSNNTLYLQMNSLRAEDTAVYYCVK | GELLEDY---------------YFYGMDV | WGQGATVTVSS |
| **iPS:3 93128** | **21-225_35F11** | **VH3\|3-23/D5\|5-12\|RF3/JH6** | | EVQLLES-GGGLVQPGGSLRLSCAASG-FTFS | S-----YAMS | WVRQAPGKGLEWVS | AISGR---GGSTFHADSMKG | RFTISRDNSKNTLYLQMNSLRAEDTAVYYCVK | GELLEDY---------------YFYGMDV | WGQGTTVTVSS |
| **iPS:3 93146** | **21-225_34G8** | **VH3\|3-23/D5\|5-12\|RF3/JH6** | | EVQLLES-GGGLVQPGGSLRLSCAASG-FTFS | S-----YAMS | WVRQAPGKGLEWVS | AISGR---GGSTFHADSVKG | RFTISRDNSKNTLYLQINSLRAEDTAVYYCVK | GELLEDY---------------YFYGMDV | WGQGTTVTVSS |
| **iPS:3 93150** | **21-225_36A5** | **VH3\|3-23/D5\|5-12\|RF3/JH6** | | EVQLLES-GGGLVQPGGSLRLSCAASG-FTFN | N-----YAMN | WVRQAPGKGLEWVS | AISRR---GGNTFYADSVKG | RFTISRDNSKNTLYLQMNSLRAEDTAVYYCAK | GELLEDY---------------YYYAMDV | WGQGTTVTVSS |
| **iPS:3 93180** | **21-225_4G12** | **VH3\|3-23/D5\|5-12\|RF3/JH6** | | EVQLLES-GGGLVQPGGSLRLSCAASG-FTFS | S-----YAMS | WVRQAPGKGLEWVS | TLSGR---GGSTYYADSVKG | RSTISRDNSKNTLYLQMSSLRAEDTAVYYCAK | WGRGYSYE---------------YYYGMDV | WGQGTTVTVSS |
| **iPS:3 93232** | **21-225_17F12** | **VH3\|3-23/D5\|5-12\|RF3/JH6** | | EVQLLES-GGGLVQPGGSLRLSCAASG-FTFS | S-----YAMS | WVRQAPGKGLEWVS | AISGG---GGSTYYADSVKG | RVTISRDNSKNTLYLQMNSLRAEDTAVYYCAK | WGRGYNYE---------------YYYGMDV | WGQGTTVTVSS |
| **iPS:3 98494** | **21-225_21H4** | **VH3\|3-23/D5\|5-** | | EVQLLES-GGGLVQPGGSLRL | S-----YAMS | WVRQAPGKGLEWVS | ALSGR---GGSTYYADS | RFTISRDNSKNTLYLQMNSLRAEDTAVYYCAK | WGRGYSYE---------------YYYGMDV | WGQGTTVTVS |

| | | | | | | | | | | |
|---|---|---|---|---|---|---|---|---|---|---|
| iPS:398508 | 21-225_24B1 | VH3\|3-23/D5\|5-12\|RF3/JH6 | | EVQLLES-GGGLVQPGGSLRLSCAASG-FTFS | S-----YAMS | WVRQAPGKGLEWVS | AISGR---GGSTYYADSVKG | RFTISRDNSKNTLYLQMNSLRAEDTAVYYCAK | WGRGYSYE---------------YYYGMDV | WGQGTTVTVSS |
| iPS:398528 | 21-225_32G1 | VH3\|3-23/D5\|5-12\|RF3/JH6 | | EVQLLES-GGGLAQPGGSLRLSCAASG-FTFS | S-----YAMS | WVRQAPGKGLEWVS | AISGR---GGSTFHADSVKG | RFTISRDNSKNTLYLQMNSLRAEDTAVYYCVK | GELLEDY----------------YFYGMDV | WGQGTTVTVSS |
| iPS:398534 | 21-225_33B8 | VH3\|3-23/D5\|5-12\|RF3/JH6 | | EVQLLES-GGGLVQPGGSLRLSCAASG-FTFS | S-----YAMS | WVRQAPGKGLEWVS | AISGR---GGSTFHADSVKG | RFTISRDNSKNTLYLQMNSLRAEDTAVYYCVK | GELLEDY----------------YFYGMDV | WGQGTTVTVSS |
| iPS:398540 | 21-225_35A6 | VH3\|3-23/D5\|5-12\|RF3/JH6 | | EVQLLES-GGGLVQPGGSLRLSCAASG-FTFS | S-----YAMS | WVRQAPGKGLEWVS | TISGR---GGSTFHADSVKG | RFTISRDNSKNTLYLQMNSLRAEDTAVYYCVK | GELLEDY----------------YFYGMDV | WGQGTTVTVSS |
| | | Germline | | H_FR1 | H_CDR1 | H_FR2 | H_CDR2 | H_FR3 | H_CDR3 | H_FR4 |
| | VH4\|4-39/D4\|4-17\|RF2/JH4 | | 32533 | QLQLQES-GPGLVKPSETLSLTCTVSG-GSIS | SS---SYYWG | WIRQPPGKGLEWIG | SIYY----SGSTYYNPSLKS | RVTISVDTSKNQFSLKLSSVTAADTAVYYCAR | DYGDY---------------------YFDY | WGQGTLVTVSS |
| iPS:392622 | 21-225_17H8 | VH4\|4-39/D4\|4-17\|RF2/JH4 | | QLQLQES-GPGLVKPSETLSLTCTVSG-GSIS | RS---SYYWG | WIRQPPGRGLEWIG | NIYY----GGNTYYNPSLKS | RVTISVDTSKNQFSLKLSSVTAADTAVYYCAR | HGKDW--------------------GLDY | WGQGTLVTVSS |
| iPS:392638 | 21-225_17F9 | VH4\|4-39/D4\|4-17\|RF2/JH4 | | QLQLQES-GPGLVKPSETLSLTCTVSG-GSIS | RS---SYYWG | WIRQPPGKGLEWIG | NIYY----SGSTYYNPSLKS | RVTISVDSSKNQFSLNLNSVTAADTAVYSCAR | HGKDW--------------------GLDY | WGQGTLVTVSS |
| iPS:392656 | 21-225_1F2 | VH4\|4-39/D4\|4-17\|RF2/JH4 | | QLQLQES-GPGLVKPSETLSLTCTVSG-GSIS | RS---SYYWG | WIRQPPGKGLEWIG | NIYY----SGSAYNNPSLKG | RVTISVDTSKNQFSLKLNSVTAADTAVYYCGR | HGKDW--------------------GLDY | WGQGTLVTVSS |
| iPS:392794 | 21-225_21H3 | VH4\|4-39/D4\|4-17\|RF2/JH4 | | QLQLQES-GPGLVKPSETLSLTCTVSG-GSIS | RS---SYYWG | WIRQPPGKGLEWIG | NIYY----SGSTYDNPSLKS | RVTISVDTSKNQFSLKLSSVTAADTAVYYCGR | HGKDW--------------------GLDY | WGQGTLVTVSS |
| iPS:392822 | 21-225_23C8 | VH4\|4-39/D4\|4-17\|RF2/JH4 | | QLQLQES-GPGLVKPSETLSLTCTVSG-GSIS | RS---SYYWG | WIRQPPGKGLEWIG | NIYY----SGTTYNNPSLKS | RVTISVDTSKNHFSLKLSSVTAADTAVYYCGR | HGKDW--------------------GLDY | WGQGTLVTVSS |
| iPS:392838 | 21-225_22G8 | VH4\|4-39/D4\|4-17\|RF2/JH4 | | QLQLQES-GPGLVKPSETLSLTCTVSG-GSIN | RS---SYYWG | WIRQPPGKGLDWIG | NIYY----SGSTYYNPSVKS | RFTISVDTSKNQFSLKLSSVTAADTAVYYCAR | HGKDW--------------------GLDY | WGQGTLVTVSS |
| iPS:392858 | 21-225_22H4 | VH4\|4-39/D4\|4- | | QLQLQES-GPGLVKPSETLSL | RS---SYYWG | WIRQPPGKGLEWIG | NIYY----SGSTYHNPS | RVTISVDTSMNQFSLKLTSVTAADTAVYFCGR | HGKDW--------------------GLDY | WGQGTLVTVS |

| | | | | | | | | | | |
|---|---|---|---|---|---|---|---|---|---|---|
| iPS:392882 | 21-225_23A3 | VH4\|4-39/D4\|4-17\|RF2/JH4 | | QLQLQES-GPGLVKPSETLSLTCTVSG-GSIS | RS---SYYWG | WIRQPPGQGLEWIG | NIYY----SGSTYNNPSLKS | RVSISVDTSKNQFSLNLSSVTAADTAVYYCGR | HGKDW--------------------GLDF | WGQGTLVTVSS |
| iPS:393804 | 21-225_5H7 | VH4\|4-39/D4\|4-17\|RF2/JH4 | | QLQLQES-GPGLVKPSETLSLTCTVSG-GSIN | RS---SYYWG | WIRQPPGKGLEWIG | NIYY----SGTTYYNPSLKS | RVTISVDTSKNQFSLNLSSVTAADTAVYSCAR | HGKDW--------------------GLDY | WGQGTLVTVSS |
| iPS:393832 | 21-225_14B2 | VH4\|4-39/D4\|4-17\|RF2/JH4 | | QLQLQES-GPGLVKPSETLSLTCTVSG-GSIN | RS---SYYWG | WIRQPPGKGLEWIG | NIYY----SGTTYYNPSLKS | RVTISVDTSKNQFSLNLSSVTAADTAVYSCAR | HGKDW--------------------GLDY | WGQGALVTVSS |
| iPS:394037 | 21-225_4F4 | VH4\|4-39/D4\|4-17\|RF2/JH4 | | QLQLQES-GPGLVRPSETLSLTCTVSG-GSIS | RS---SYYWG | WIRQPPGKGLEWIG | NIYY----SGSTYDNPSLKS | RVTISVDTSKNQFSLKLSSVTAADTAVYYCGR | HGKDW--------------------GLDY | WGQGTLVTVSS |
| iPS:394045 | 21-225_4H4 | VH4\|4-39/D4\|4-17\|RF2/JH4 | | QLQLQES-GPGLVKPSETLSLTCTVSG-GSIS | RS---SYYWG | WIRQPPGMGLEWIG | NIYY----SGNTYNNPSLKS | RVTISVDTSKNQFSLKLNSVTAADTAVYYCGR | HGKDW--------------------GLDY | WGQGTLVIVSS |
| iPS:394079 | 21-225_11F5 | VH4\|4-39/D4\|4-17\|RF2/JH4 | | QLQLQES-GPGLVKPSETLSLTCTVSG-GSIS | RS---SYYWG | WIRQPPGKGLEWIG | NIYY----SGSTYTNPSLKS | RVTISVDTSKNHFSLKLSSVTAADTAVYYCGR | HGKDW--------------------GLDN | WGQGTLVTVSS |
| | | Germline | | H_FR1 | H_CDR1 | H_FR2 | H_CDR2 | H_FR3 | H_CDR3 | H_FR4 |
| | VH3\|3-21/D4\|4-11\|RF2/JH3 | | 32534 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | DYSNY--------------------DAFDI | WGQGTMVTVSS |
| iPS:392624 | 21-225_17H12 | VH3\|3-21/D4\|4-11\|RF2/JH3 | | EVQLVES-GGGLVKPGGSLRLSCAASG-FTFS | S-----YTMN | WVRQAPGKGLEWVS | SISGS---SSYIYYADSVKG | RFTISRDNAKNSLYLQMNSLRAEDTAVYYCAR | DRG-------------------------SI | WGQGTMVTVSS |
| iPS:393946 | 21-225_16A4 | VH3\|3-21/D4\|4-11\|RF2/JH3 | | EVQLVES-GGGLVKPGGSLRLSCAASG-FTFS | S-----YSMN | WVRQAPGKGLEWVS | SISGS---STYKYYADSVKG | RFTISRDNAKNSLYLQMNSLRTEDTAVYYCAR | DRG-------------------------SY | WGQGTQVTVSS |
| iPS:394008 | 21-225_15H8 | VH3\|3-21/D4\|4-11\|RF2/JH3 | | EVQLVES-GGGLVKPGGSLRLSCAASG-FTFS | S-----YVMN | WVRQAPGKGLEWVS | SISGS---STYIYCADSIKG | RFTISRDNAKNSLYLQMNSLRADDTAVYYCAR | DRG-------------------------SI | WGQGTMVTVSS |
| | | Germline | | H_FR1 | H_CDR1 | H_FR2 | H_CDR2 | H_FR3 | H_CDR3 | H_FR4 |
| | VH6\|6-01/D6\|6-19\|RF1/JH6 | | 32535 | QVQLQQS-GPGLVKPSQTLSLTCAISG-DSVS | SN---SAAWN | WIRQSPSRGLEWLG | RTYYR--SKWYNDYAVSVKS | RITINPDTSKNQFSLQLNSVTPEDTAVYYCAR | GYSSGWYY--------------YYYYGMDV | WGQGTTVTVSS |
| iPS:392636 | 21-225_17A6 | VH6\|6-01/D6\|6- | | QVQLQQS-GPGLVKPSQTLSL | RN---TAAWS | WIRQSPSRGLEWLG | RTYYR--SKWYNDYAV | RVTINPDTSKNQFSLQLNSVTPEDTAVYYCAR | VSSGWSHH--------------YYYYGMDV | WGQGTTVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH6\|6-01/D2\|2-15\|RF2/JH6 | | 32536 | QVQLQQS-GPGLVKPSQTLSLTCAISG-DSVS | SN---SAAWN | WIRQSPSRGLEWLG | RTYYR--SKWYNDYAVSVKS | RITINPDTSKNQFSLQLNSVTPEDTAVYYCAR | GYCSGGSCYS-------------YYYYGMDV | WGQGTTVTVSS |
| iPS:392648 | 21-225_16D11 | VH6\|6-01/D2\|2-15\|RF2/JH6 | | QVQLQQS-GPGLVKPSQTLSLTCAISG-DSVS | RN---TAAWS | WIRQSPSRGLEWLG | RTYYR--SKWYNDYAVSVKS | RITINPDTSKNQFSLQLNSVTPEDTAVYYCAR | VNSGWSHH--------------YYYYGMDV | WGQGTTVTVSS |
| | | Germline | | H_FR1 | H_CDR1 | H_FR2 | H_CDR2 | H_FR3 | H_CDR3 | H_FR4 |
| | VH3\|3-11/D1\|1-1\|RF3/JH2 | | 32537 | QVQLVES-GGGLVKPGGSLRLSCAASG-FTFS | D-----YYMS | WIRQAPGKGLEWVS | YISSS---GSTIYYADSVKG | RFTISRDNAKNSLYLQMNSLRAEDTAVYYCAR | YNWNDY---------------------WYFDL | WGRGTLVTVSS |
| iPS:392650 | 21-225_17A4 | VH3\|3-11/D1\|1-1\|RF3/JH2 | | QVQLVES-GGGLVKPGGSLRLSCAASG-FTFS | D-----YYMS | WIRQAPGKGLEWVS | HISSS---GSTIYYADSVKG | RFTISRDNAKNSLYLQMNSLRAEDTAVYYCAR | YRNNR--------------------GYFDL | WGRGTLVTVSS |
| iPS:392728 | 21-225_20F7 | VH3\|3-11/D1\|1-1\|RF3/JH2 | | QVQLVES-GGGLVKPGGSLRLSCAASG-FTFS | D-----YYMS | WIRQAPGKGLEWVS | HISSS---GSTIYYADSVKG | RFTISRDNGENSLYLQMNSLRAEDTAVYYCAR | YRNNR--------------------GYFDL | WGRGSLVTVSS |
| | | Germline | | H_FR1 | H_CDR1 | H_FR2 | H_CDR2 | H_FR3 | H_CDR3 | H_FR4 |
| | VH4\|4-59/D1\|1-26\|RF3/JH4 | | 32538 | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAR | YSGSY--------------------YYFDY | WGQGTLVTVSS |
| iPS:392676 | 21-225_19F3 | VH4\|4-59/D1\|1-26\|RF3/JH4 | | QVQLQES-GPGLVKPSETLSLTCTVSG-GAIS | GS---SYYWG | WIRQPPGKQLEWIG | NIYY----SGSTYYNPSFKS | RVTISVDTSKNQFSLKLSSVTAEDTAVYYCAR | HSSSW---------------------SLDY | WGQGTLVTVSS |
| | | Germline | | H_FR1 | H_CDR1 | H_FR2 | H_CDR2 | H_FR3 | H_CDR3 | H_FR4 |
| | VH3\|3-23/D6\|6-13\|RF2/JH2 | | 32539 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GIAAAG-------------------YWYFDL | WGRGTLVTVSS |
| iPS:392678 | 21-225_20F3 | VH3\|3-23/D6\|6-13\|RF2/JH2 | | EVQLLES-GGGLVQPGGSLRLSCAASG-FTFS | S-----YAMN | WVRQAPGKGLEWVS | VISGS---GGSTYYADSVKG | RFTISRDNSKNTLYLQMNSLRAEDTAVYYCAK | RIAAAG------------------TEYFDL | WGRGTLVTVSS |
| | | Germline | | H_FR1 | H_CDR1 | H_FR2 | H_CDR2 | H_FR3 | H_CDR3 | H_FR4 |
| | VH3\|3-21/D5\|5-24\|RF3/JH4 | | 32540 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | RDGYN--------------------YYFDY | WGQGTLVTVSS |
| iPS:392682 | 21-225_16A12 | VH3\|3-21/D5\|5-24\|RF3/JH4 | | EVQLVES-GGGLVKPGGSLRLSCAASG-FTFS | S-----YRMN | WFRQAPGKGLEWVS | SISGS---STDIYYADSVKG | RFTISRDNAENSLYLQMNSLRAEDTAVYYCAR | RD---------------------------F | WGQGTLVTVSS |
| | | Germline | | H_FR1 | H_CDR1 | H_FR2 | H_CDR2 | H_FR3 | H_CDR3 | H_FR4 |
| | VH3\|3-48/D7\|7- | | 32541 | EVQLVES- | S-----YSMN | WVRQAPGKG | YISSS--- | RFTISRDNAKNSLYLQMNSL | LTGY------------ | WGQGT |

| | | | | | | | | | | |
|---|---|---|---|---|---|---|---|---|---|---|
| iPS:392692 | 21-225_18G10 | VH3\|3-48/D7\|7-27\|RF1/JH4 | | EVQLVES-GGGLVQPGGSLRLLCAASG-ITFS | T-----YSMN | WVRQAPGKGLEWVS | YISRS---SSTIDYADSVKG | RFTISRDNAKNSLYLQMNSLRDEDTAVYYCAR | GGGS-----------------------PFDY | WGQGTLVTVSS |
| iPS:392708 | 21-225_18D11 | VH3\|3-48/D7\|7-27\|RF1/JH4 | | EVQLVES-GGGLVQPGGSLRLSCAASG-FTFR | S-----YSMN | WVRQAPGKGLEWLS | YISSS---SGTIYYADSVKG | RFTISRDNARNSLNLQMNSLRDEDTAVYYCAR | GGGS-----------------------PFDY | WGQGILVTVSS |
| iPS:393992 | 21-225_14H8 | VH3\|3-48/D7\|7-27\|RF1/JH4 | | EVQLVES-GGGLVQPGGSLRLSCAASG-FTFS | S-----NSMN | WVRQAPGKGLEWVS | YISSS---SSTIYYADSVKG | RFTIARDNAKNSLYLQMNSLRDEDTAVYYCAR | GGGS-----------------------PFDY | WGQGTLVTVSS |
| iPS:394055 | 21-225_9C8 | VH3\|3-48/D7\|7-27\|RF1/JH4 | | EVQLVES-GGGLVQPGGSLRLSCVVSG-FTFS | S-----QSMN | WVRQAPGKGLEWVS | YISI----SSTIYYADSVKG | RFTISRDNAKNSLYLQMNSLRDEDTAVYYCAR | GGGS-----------------------PFDS | WGQGTLVTVSS |
| | | Germline | | H_FR1 | H_CDR1 | H_FR2 | H_CDR2 | H_FR3 | H_CDR3 | H_FR4 |
| | VH3\|3-30.3/D4\|4-11\|RF2/JH4 | | 32542 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | DYSNY----------------------YFDY | WGQGTLVTVSS |
| iPS:392694 | 21-225_19A5 | VH3\|3-30.3/D4\|4-11\|RF2/JH4 | | QVQLVES-GGGVVQPGRSLRLSCAASG-FTFS | S-----YAMH | WVRQAPGKGLEWVA | VIWFD---GSDKYYADSVKG | RFTISRDNSKNTLYLQMNSLRAEDTAVYYCAR | DRAYS---------------------SSSDY | WGQGTLVTVSS |
| | | Germline | | H_FR1 | H_CDR1 | H_FR2 | H_CDR2 | H_FR3 | H_CDR3 | H_FR4 |
| | VH3\|3-23/D1\|1-1\|RF1/JH4 | | 32543 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GTTGT----------------------YFDY | WGQGTLVTVSS |
| iPS:392714 | 21-225_16G12 | VH3\|3-23/D1\|1-1\|RF1/JH4 | | EVQLLES-GGGLVQPGGSLRLSCAASG-FSFS | S-----YAMT | WVRQAPGKGLEWVS | TISGR---GGHTYYADSVRG | RFAISRDSSKNTLYLQMNSLRAEDTAVYYCAK | QD---------------------------C | WGQGTLVTVSS |
| iPS:392890 | 21-225_20H9 | VH3\|3-23/D1\|1-1\|RF1/JH4 | | EVQLLES-GGGLVQPGGSLRLSCAASG-FTFS | S-----YAMS | WVRQAPGKGLEWVS | AISGS---GGYTYYADSVKG | RFTISRDNSENTLYLQMSSLRAEDTAVYYCAK | GGS-----------------------LFY | WGQGTLVTVSS |
| iPS:392892 | 21-225_20C11 | VH3\|3-23/D1\|1-1\|RF1/JH4 | | EVQLLES-GGGLVQPGGSLRLSCAASG-FSFS | S-----YAMS | WVRQAPGKGLEWVS | TISGR---GGHTYYADSVKG | RFAISRDSSKNTLYLQMNSLRAEDTAVYYCAK | QD---------------------------C | WGQGTLVTVSS |
| iPS:393968 | 21-225_5A5 | VH3\|3-23/D1\|1-1\|RF1/JH4 | | EVQLWES-GGGLVQPGGSLRLSCAASG-FTFS | S-----YAMN | WVRQAPGKGLEWVS | AISGS---GGYTYYADSVKG | RFTISRDNSKNTLYLQMSSLRAEDTAVYYCAK | GGS-----------------------LFY | WGQGTLVTVSS |
| | | Germline | | H_FR1 | H_CDR1 | H_FR2 | H_CDR2 | H_FR3 | H_CDR3 | H_FR4 |
| | VH3\|3-23/D6\|6- | | 32544 | EVQLLES- | S-----YAMS | WVRQAPGK | AISGS--- | RFTISRDNSKNTLYLQMNSL | EYSSSS----------- | WGQGT |

<table>
<tr><td>iPS:392730</td><td>21-225_17A1</td><td>VH3|3-23/D6|6-6|RF1/JH3</td><td></td><td>EVQLLES-GGGLVQPGGSLRLSCAASG-FTFS</td><td>S-----YAMN</td><td>WVRQAPGKGLEWVS</td><td>VISGS---GSNTYYADSVKG</td><td>RFTISRDNSKNTLYLQMNSLRAEDTAVYYCAK</td><td>RYTSDW-----------------HDAFDI</td><td>WGQGTMVTVSS</td></tr>
<tr><td>iPS:392736</td><td>21-225_17B12</td><td>VH3|3-23/D6|6-6|RF1/JH3</td><td></td><td>EVQLLES-GGGLVQPGGSLRLSCVASG-FTFS</td><td>S-----YAMN</td><td>WVRQAPGKGLEWVS</td><td>VISGS---GGSTYYADSVKG</td><td>RFTISRDNSKNTLYLQMNTLRAEDTAVYYCAK</td><td>RYTSDW-----------------HDAFDI</td><td>WGQGTMVTVSS</td></tr>
<tr><td>iPS:392770</td><td>21-225_20C10</td><td>VH3|3-23/D6|6-6|RF1/JH3</td><td></td><td>EVQLLES-GGGLVQPGGSLRLSCAASG-FTFS</td><td>S-----YAMN</td><td>WVRQAPGKGLEWVS</td><td>VISGS---GGTTYYADSVKG</td><td>RFTISRDNSKNTLYLQMNTLRAEDTAVYYCAK</td><td>RYTSDW-----------------HDAFDI</td><td>WGQGTMVTVSS</td></tr>
<tr><td>iPS:393878</td><td>21-225_7G12</td><td>VH3|3-23/D6|6-6|RF1/JH3</td><td></td><td>EVQLLES-GGGLVQPGGSLRLSCAASG-FTFS</td><td>S-----YAMN</td><td>WVRQAPGKGLEWVS</td><td>VISGS---GGSTYYADSVKG</td><td>RFTISRDNSKNTLYLQMNTLRAEDTAVYYCAK</td><td>RYTSDW-----------------HDAFDI</td><td>WGQGTMVTVSS</td></tr>
<tr><td></td><td></td><td>Germline</td><td></td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH3|3-21/D7|7-27|RF3/JH5</td><td></td><td>32545</td><td>EVQLVES-GGGLVKPGGSLRLSCAASG-FTFS</td><td>S-----YSMN</td><td>WVRQAPGKGLEWVS</td><td>SISSS---SSYIYYADSVKG</td><td>RFTISRDNAKNSLYLQMNSLRAEDTAVYYCAR</td><td>NWGN-----------------------WFDP</td><td>WGQGTLVTVSS</td></tr>
<tr><td>iPS:392748</td><td>21-225_20A8</td><td>VH3|3-21/D7|7-27|RF3/JH5</td><td></td><td>EVQLVES-GGGLVKPGGSLRLSCAASG-FTFS</td><td>S-----YSVN</td><td>WVRRAPGKGLEWVS</td><td>SISSS---SSFLYYADSVKG</td><td>RFTISRDNAKNSVYLQMNSLRAEDTAVYYCAR</td><td>NW--------------------------DY</td><td>WGQGTLVTVSS</td></tr>
<tr><td></td><td></td><td>Germline</td><td></td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH3|3-30.3/D1|1-1|RF1/JH5</td><td></td><td>32546</td><td>QVQLVES-GGGVVQPGRSLRLSCAASG-FTFS</td><td>S-----YAMH</td><td>WVRQAPGKGLEWVA</td><td>VISYD---GSNKYYADSVKG</td><td>RFTISRDNSKNTLYLQMNSLRAEDTAVYYCAR</td><td>GTTGT------------------NWFDP</td><td>WGQGTLVTVSS</td></tr>
<tr><td>iPS:392754</td><td>21-225_21D3</td><td>VH3|3-30.3/D1|1-1|RF1/JH5</td><td></td><td>QVQLVES-GGGVVQPGRSLRLSCVASG-FTFS</td><td>S-----YGMH</td><td>WVRQAPGKGLEWVT</td><td>VISYD---GSNKYYADSVKG</td><td>RFTISRDNSKNTLYLQMNSLRAEDTAVYYCAR</td><td>GVWF----------------------GDL</td><td>WGQGTLVTVSS</td></tr>
<tr><td>iPS:392818</td><td>21-225_22D8</td><td>VH3|3-30.3/D1|1-1|RF1/JH5</td><td></td><td>QVQLVES-GGGVVQPGRSLRLSCVASG-FTFR</td><td>S-----YGMH</td><td>WVRQAPGKGLEWVT</td><td>IISYD---GSNKYYADSVKG</td><td>RFTISRDNSKNTLYLQMNSLRAEDTAVYFCAR</td><td>GVWF----------------------GDF</td><td>WGQGTLVTVSS</td></tr>
<tr><td></td><td></td><td>Germline</td><td></td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH3|3-23/D6|6-19|RF2/JH1</td><td></td><td>32547</td><td>EVQLLES-GGGLVQPGGSLRLSCAASG-FTFS</td><td>S-----YAMS</td><td>WVRQAPGKGLEWVS</td><td>AISGS---GGSTYYADSVKG</td><td>RFTISRDNSKNTLYLQMNSLRAEDTAVYYCAK</td><td>GIAVAG-----------------AEYFQH</td><td>WGQGTLVTVSS</td></tr>
<tr><td>iPS:392762</td><td>21-225_22G5</td><td>VH3|3-23/D6|6-19|RF2/JH1</td><td></td><td>EVQLLES-GGGLVQPGGSLRLSCAASG-FTFS</td><td>S-----YAMN</td><td>WVRQAPGMGLEWVS</td><td>VISRS---GGYTYYADSVKG</td><td>RFTISRDNSKNTLYLQMNSLRAEDTAVYYCAS</td><td>RLAVAG-----------------SEAFDI</td><td>WGQGTLVTVSS</td></tr>
<tr><td>iPS:394051</td><td>21-225_9E5</td><td>VH3|3-23/D6|6-</td><td></td><td>EVQLLES-GGGLVQPGGSLRL</td><td>N-----YAMN</td><td>WVRQAPGKGLEWVS</td><td>AISGG---GGNTFYADS</td><td>RFTISRDNSKNTLYLQMNGLRAEDTAVYYCAS</td><td>RIAVAG-----------------SEAFAI</td><td>WGQGTLVTVS</td></tr>
</table>

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH4\|4-39/D1\|1-26\|RF3/JH1 | | 32548 | QLQLQES-GPGLVKPSETLSLTCTVSG-GSIS | SS---SYYWG | WIRQPPGKGLEWIG | SIYY----SGSTYYNPSLKS | RVTISVDTSKNQFSLKLSSVTAADTAVYYCAR | YSGSYY-------------------AEYFQH | WGQGTLVTVSS |
| iPS:392774 | 21-225_21F3 | VH4\|4-39/D1\|1-26\|RF3/JH1 | | QLQLQES-GPGLVKPAETLSLTCTVSG-GSIS | RS---SYYWG | WIRQPPGKVLEWIG | SIYY----SGSTYYNPSLKS | RVTISVDTSKNQFSLKLSSVTAADTAEYYCAS | LSSSW--------------------DFQH | WGQGTLVTVSS |
| iPS:393962 | 21-225_7H7 | VH4\|4-39/D1\|1-26\|RF3/JH1 | | QLQLQES-GPGLVKPSETLSLTCTVSG-GSIS | RS---SYYWG | WIRQPPGKGLEWIG | NIYY----SGSTYYIPSLKS | RVTISVDTSKNQFSLKLSSVTAADTAVYYCAR | HSTSW--------------------SLDH | WGQGTLVTVSS |
| iPS:394049 | 21-225_13H5 | VH4\|4-39/D1\|1-26\|RF3/JH1 | | QLQLQES-GPGLVKPAETLSLTCTVSG-GSIS | RS---SYYWG | WIRQPPGKGLEWIG | SIYY----SGSTYYNPSLKS | RVTISVDTSKNQFSLKLSSVTAADTAVYYCAS | LSSSW--------------------DFQH | WGQGTLVTVSS |
| | | Germline | | H_FR1 | H_CDR1 | H_FR2 | H_CDR2 | H_FR3 | H_CDR3 | H_FR4 |
| | VH3\|3-21/D2\|2-21\|RF2/JH4 | | 32549 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | AYCGGDC-----------------YSYFDY | WGQGTLVTVSS |
| iPS:392776 | 21-225_21A12 | VH3\|3-21/D2\|2-21\|RF2/JH4 | | EVQLVES-GGGLVKPGGSLRLSCAASG-FTFN | S-----YSMN | WVRQAPGKGLEWVS | SISGS---SSYIYYADSVKG | RFTISRDNAKNSLYLQMNSLRAEDTALYYCAR | AAG------------------------FDY | WGQGTLVTVSS |
| | | Germline | | H_FR1 | H_CDR1 | H_FR2 | H_CDR2 | H_FR3 | H_CDR3 | H_FR4 |
| | VH3\|3-33/D6\|6-19\|RF2/JH6 | | 32550 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | GIAVAGYY---------------YYYGMDV | WGQGTTVTVSS |
| iPS:392806 | 21-225_24H3 | VH3\|3-33/D6\|6-19\|RF2/JH6 | | QVQLVES-GGGVVQPGRSLRLSCGASG-FTFS | S-----YGMH | WVRQAPGKGLEWVA | VIWYD---GSNKYYADSVKG | RFTISRDNSKNTLYLQMNSLRAEDTAVYYCAR | VAVAG--------------------GMDV | WGQGTTVTVSS |
| | | Germline | | H_FR1 | H_CDR1 | H_FR2 | H_CDR2 | H_FR3 | H_CDR3 | H_FR4 |
| | VH3\|3-48/D7\|7-27\|RF2/JH4 | | 32551 & 32552 | EVQLVES-GGGLVQPGGSLRLSCAASG-FTFS | S-----YSMN | WVRQAPGKGLEWVS | YISSS---SSTIYYADSVKG | RFTISRDNAKNSLYLQMNSLRDEDTAVYYCAR | *LGY-----------------------FDY | WGQGTLVTVSS |
| iPS:392826 | 21-225_20B9 | VH3\|3-48/D7\|7-27\|RF2/JH4 | | EVQLVES-GGGLVQPGGSLRLSCAASG-FTFS | S-----YSMN | WVRQAPGKGLEWVS | YISSS---SSTIYYADSVKG | RFTISRDNAKNSLYLQMNSLRDEDTAVYYCAR | SLWS---------------------PFDY | WGQGTLVTVSS |
| | | Germline | | H_FR1 | H_CDR1 | H_FR2 | H_CDR2 | H_FR3 | H_CDR3 | H_FR4 |
| | VH4\|4-59/D1\|1-1\|RF1/JH5 | | 32553 | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAR | GTTGT-------------------NWFDP | WGQGTLVTVSS |
| iPS:392830 | 21-225_21A5 | VH4\|4-59/D1\|1- | | QVQLQES-GPGLVKPSETLSL | S-----YFWS | WIRQPAGKGLEWIG | RIYT----SGITNYNPS | RVTMSVDTSKNQFSLKLSSVTAADTAIYYCAR | GPTSG--------------------WFDP | WGQGTLVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH3\|3-23/D6\|6-6\|RF2/JH4 | | 32554 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | SIAAR-----------------------YFDY | WGQGTLVTVSS |
| iPS:392840 | 21-225_23G1 | VH3\|3-23/D6\|6-6\|RF2/JH4 | | EVQLLES-GGGLVQPGGSLRLSCAASG-FTFS | S-----YAMS | WVRQAPGRGLEWVS | VISGS---GGTTYNTDSVKG | RFTISRDNSKNTLYLQMNSLRAEDTAVYYCAR | SSL------------------------FDY | WGQGTLVTVSS |
| iPS:394018 | 21-225_15B1 | VH3\|3-23/D6\|6-6\|RF2/JH4 | | EVQLLES-GGGLVQPGGSLRLSCATSG-FTFS | S-----YVMS | WVRQAPGKGLEWVS | GISGS---GGSTNNADSVKG | RFTISRDNSKNTLYLQVNSLRAEDTAVYYCAR | SSL------------------------FDY | WGQGTLVTVSS |
| iPS:394026 | 21-225_16C7 | VH3\|3-23/D6\|6-6\|RF2/JH4 | | EVQLLES-GGGLVQPGGSLRLSCAASG-FTFS | S-----YVMT | WVRQAPGKGLEWVS | TISGS---GGWTYYADSVKG | RFTISRDNSKNTLYLQMNSLRAEDTAEYYCAR | SSL------------------------FDY | WGQGTLVTVSS |
| | | Germline | | H_FR1 | H_CDR1 | H_FR2 | H_CDR2 | H_FR3 | H_CDR3 | H_FR4 |
| | VH3\|3-23/D6\|6-6\|RF2/JH5 | | 32555 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | SIAAR---------------------NWFDP | WGQGTLVTVSS |
| iPS:392842 | 21-225_23G8 | VH3\|3-23/D6\|6-6\|RF2/JH5 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAV | SSG------------------------WFA | WGQGTLVTVSS |
| | | Germline | | H_FR1 | H_CDR1 | H_FR2 | H_CDR2 | H_FR3 | H_CDR3 | H_FR4 |
| | VH3\|3-23/D1\|1-1\|RF2/JH2 | | 32556 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | VQLERY--------------------WYFDL | WGRGTLVTVSS |
| iPS:392856 | 21-225_22A2 | VH3\|3-23/D1\|1-1\|RF2/JH2 | | EVQLLES-GGGLVQPGGSLRLSCAASG-FTFS | S-----YAMS | WVRQAPGKGLEWVS | GISGS---GGNTPYADSVKG | RFTISRDISKNTLYLQMNSLRAEDTAVYYCAK | VVG------------------------AVH | WGRGTLVTVSS |
| | | Germline | | H_FR1 | H_CDR1 | H_FR2 | H_CDR2 | H_FR3 | H_CDR3 | H_FR4 |
| | VH4\|4-30.1/D5\|5-18\|RF1/JH6 | | 32557 | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | VDTAMVYY---------------YYYGMDV | WGQGTTVTVSS |
| iPS:392864 | 21-225_23B9 | VH4\|4-30.1/D5\|5-18\|RF1/JH6 | | QVQLQAS-GPGLVKPSQTLSLTCTVSD-GSIS | SG---GYYWS | WIRQHPGKGLEWIG | YIYY----SGSTYYNPSLKS | RVTISVDTSKNQFSLKLSSVTAADTAVYYCAR | EDGAFG------------------YYGMDV | WGQGTTVTVSS |
| | | Germline | | H_FR1 | H_CDR1 | H_FR2 | H_CDR2 | H_FR3 | H_CDR3 | H_FR4 |
| | VH3\|3-23/D2\|2-2\|RF2/JH3 | | 32558 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GYCSSTSC----------------YIDAFDI | WGQGTMVTVSS |
| iPS:392874 | 21-225_21D2 | VH3\|3-23/D2\|2- | | EVKLLES-GGGLVQPGGSLRL | N-----YAMS | WVRQAPGKGLEWVS | VLSGS---GGSTFYADS | RFTISRDNSKNTLYLQMSSLRAGDTAVYFCAR | YCSSARC-----------------PYDAFDI | WGQGTMVTVS |

| | | | | | | | | | | |
|---|---|---|---|---|---|---|---|---|---|---|
| iPS:393940 | 21-225_16B2 | VH3\|3-23/D2\|2-2\|RF2/JH3 | | EVQLLES-GGGLVQPGGSLRLSCAASG-FTFS | S-----YAMT | WVRQAPGKGPEWVS | VISGS---GGSTFYADSVKG | RFTISRDNSKNTLYLQMSSLRAEDTAVYFCAR | YCSSTRC---------------PYDAFDI | WGQGTMVTVSS |
| iPS:393956 | 21-225_4D7 | VH3\|3-23/D2\|2-2\|RF2/JH3 | | EVKLLES-GGGLVQPGGSLRLSCAASG-FTFS | S-----YAMS | WVRQAPGKGLEWVS | VLSGS---GGSTFYADSVKG | RFTISRDNSKNTLYLQMSSLRAGDTAVYFCAR | YCSSARC---------------PYDAFDI | WGQGTMVTVSS |
| iPS:398476 | 21-225_17C1 | VH3\|3-23/D2\|2-2\|RF2/JH3 | | EVQLLES-GGGLEQPGGSLRLSCAASG-FTFS | S-----YAMS | WVRQAPGKGLEWVS | VISGS---GGTTFYADSVKG | RFTISRDNSKNTLYLQMSSLRAEDTAVYFCAR | YCSSTRC---------------PYDAFDI | WGQGTMVTVSS |
| | | Germline | | H_FR1 | H_CDR1 | H_FR2 | H_CDR2 | H_FR3 | H_CDR3 | H_FR4 |
| | VH3\|3-15/D1\|1-1\|RF3/JH4 | | 32559 | EVQLVES-GGGLVKPGGSLRLSCAASG-FTFS | N-----AWMS | WVRQAPGKGLEWVG | RIKSKT-DGGTTDYAAPVKG | RFTISRDDSKNTLYLQMNSLKTEDTAVYYCTT | YNWND---------------------YFDY | WGQGTLVTVSS |
| iPS:392898 | 21-225_21H10 | VH3\|3-15/D1\|1-1\|RF3/JH4 | | EVQLVES-GGGLVKPGGSLRLSCAASG-FTFS | N-----AWMN | WVRQAPGKGLEWVG | RIKSKT-DGGTTDYAAPVKG | RFTISRDDSKNTLYLQMNSLKTEDTAVYYCTT | EGWN----------------------TDY | WGQGTLVTVSS |
| iPS:393802 | 21-225_3D12 | VH3\|3-15/D1\|1-1\|RF3/JH4 | | EVQLVES-GGGLVKPGGSLRLSCAASG-VTFS | T-----AWMN | WVRQAPGKGLEWVG | RIKNKI-DGGTTDYVAPVKG | RFTISRDDSKNTLYLQMNSLKTEDTAVYYCTT | EGWN----------------------TDY | WGQGTLVTVSS |
| | | Germline | | H_FR1 | H_CDR1 | H_FR2 | H_CDR2 | H_FR3 | H_CDR3 | H_FR4 |
| | VH3\|3-48/D4\|4-11\|RF3/JH4 | | 32560 | EVQLVES-GGGLVQPGGSLRLSCAASG-FTFS | S-----YSMN | WVRQAPGKGLEWVS | YISSS---SSTIYYADSVKG | RFTISRDNAKNSLYLQMNSLRDEDTAVYYCAR | ITVT----------------------YFDY | WGQGTLVTVSS |
| iPS:392950 | 21-225_25C10 | VH3\|3-48/D4\|4-11\|RF3/JH4 | | EVQLVES-GGGLVQPGGSLRLSCAASG-FTFS | S-----YRMN | WVRQAPGKGLEWVS | SISSS---SSTIYYADSVKG | RFTISRDNAKNSLYLQMNSLRDEDTAVYYCAR | TAG-----------------------FDY | WGQGTLVTVSS |
| | | Germline | | H_FR1 | H_CDR1 | H_FR2 | H_CDR2 | H_FR3 | H_CDR3 | H_FR4 |
| | VH3\|3-23/D1\|1-26\|RF3/JH4 | | 32561 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | YSGSY-------------------YYFDY | WGQGTLVTVSS |
| iPS:393010 | 21-225_25E11 | VH3\|3-23/D1\|1-26\|RF3/JH4 | | EVQLLES-GGGLVQPGGSLRLSCAASG-FTFS | S-----YAMS | WVRQAPGKGLEWVS | VISGG---GGSTYYADSVKG | RFTISRDNSKNTLYLQMNSLRAEDTAVYYCAK | RGYSGYE---------------DLLYFDC | WGQGTLVTVSS |
| | | Germline | | H_FR1 | H_CDR1 | H_FR2 | H_CDR2 | H_FR3 | H_CDR3 | H_FR4 |
| | VH3\|3-23/D3\|3-3\|RF3/JH3 | | 32562 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | ITIFGVV---------------IIDAFDI | WGQGTMVTVSS |
| iPS:393016 | 21-225_28F11 | VH3\|3-23/D3\|3- | | EVQLLES-GGGLVQPGGSLRL | S-----YAMS | WVRQAPGKGLEWVS | VTSGS---GGTTFYADS | RFTISRDNSKNTLYLQMNSLRAEDTAVYYCAK | RTQFD-------------------DFDI | WGQGTMVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH3\|3-33/D3\|3-22\|RF2/JH6 | | 32563 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YYYDSSGYYY------------YYYYYGMDV | WGQGTTVTVSS |
| iPS:393032 | 21-225_26F8 | VH3\|3-33/D3\|3-22\|RF2/JH6 | | QVQLVES-GGGVVQPGRSLRLSCAASG-FTFS | G-----YGMH | WVRQAPGKGLEWVA | IIWYD---GSNKYYADSVKG | RFTISRDNSKNTLYLQMNSLRAEDTAVYYCAR | ERYDFW------------------SGCMDV | WGQGTTVTVSS |
| | | Germline | | H_FR1 | H_CDR1 | H_FR2 | H_CDR2 | H_FR3 | H_CDR3 | H_FR4 |
| | VH1\|1-02/D4\|4-11\|RF2/JH6 | | 32564 | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | DYSNYYY-----------------YYYGMDV | WGQGTTVTVSS |
| iPS:393042 | 21-225_31F1 | VH1\|1-02/D4\|4-11\|RF2/JH6 | | QVQLVQS-GAEVKKPGASVKVSCKASG-YTFT | D-----YYMH | WVRQAPGQGLEWMG | WINPN---SGGTNYAQKFQG | RVTMTRDTSIMTAYMELSRLRSDDTAVYYCAR | DSSNFSNW--------------YDYYGMDV | WGQGTTVTVSS |
| iPS:393108 | 21-225_34G11 | VH1\|1-02/D4\|4-11\|RF2/JH6 | | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | DISNFSSW--------------YDYYAMDV | WGQGTTVTVSS |
| | | Germline | | H_FR1 | H_CDR1 | H_FR2 | H_CDR2 | H_FR3 | H_CDR3 | H_FR4 |
| | VH1\|1-18/D5\|5-12\|RF3/JH4 | | 32565 | QVQLVQS-GAEVKKPGASVKVSCKASG-YTFT | S-----YGIS | WVRQAPGQGLEWMG | WISAY---NGNTNYAQKLQG | RVTMTTDTSTSTAYMELRSLRSDDTAVYYCAR | GYSGYD-------------------YYFDY | WGQGTLVTVSS |
| iPS:393044 | 21-225_25B8 | VH1\|1-18/D5\|5-12\|RF3/JH4 | | QVQLVQS-GAEVKKPGASVKVSCKASG-YTFT | S-----YGIS | WVRQAPGQGLEWMG | WISAY---NGNTTYAQKLRG | RVTMTTDTSTSTAYMDLRSLRSDDTAVYYCAR | TAAGYS------------------SSWFDY | WGQGTLVTVSS |
| iPS:393050 | 21-225_28C5 | VH1\|1-18/D5\|5-12\|RF3/JH4 | | QVQLVQS-GAEVKKPGASVKVSCKASD-YTFT | S-----YGIS | WVRQAPGQGLEWMG | WISAY---NGNTTYAQKLRG | RVTMTTDTSTSTAYMDLRSLRSDDTAVYYCAR | TAAGYS------------------SSWFDY | WGQGTLVTVSS |
| | | Germline | | H_FR1 | H_CDR1 | H_FR2 | H_CDR2 | H_FR3 | H_CDR3 | H_FR4 |
| | VH3\|3-33/D6\|6-6\|RF1/JH3 | | 32566 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | EYSSSS--------------------DAFDI | WGQGTMVTVSS |
| iPS:393046 | 21-225_25A12 | VH3\|3-33/D6\|6-6\|RF1/JH3 | | QVQLVES-GGGVVQPGRSLRLSCAASG-FTFS | N-----CVMH | WVRQAPGKGLEWVA | VIWYD---GSNKYYADSVKG | RFTISRDNSKNTLYLQMNSLRAEDTAVYYCAR | EEYSSGW----------------YDYGMDV | WGQGTMVTVSS |
| | | Germline | | H_FR1 | H_CDR1 | H_FR2 | H_CDR2 | H_FR3 | H_CDR3 | H_FR4 |
| | VH3\|3-21/D4\|4-11\|RF3/JH6 | | 32567 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | IIVTYYY-----------------YYGMDV | WGQGTTVTVSS |
| iPS:393078 | 21-225_33H11 | VH3\|3-21/D4\|4- | | EVQLVES-GGGLVKPGGSLRL | S-----YSMN | WVRQAPGKGLEWVS | SISGS---SSYIYYADS | RFTISRDNAKNSLYLQMSSLRAEDTAVYYCAR | TNG----------------------MDV | WGQGTTVTVS |

| | | | | | | | | | | |
|---|---|---|---|---|---|---|---|---|---|---|
| iPS:393142 | 21-225_33A3 | VH3\|3-21/D4\|4-11\|RF3/JH6 | | EVQLVES-GGGLVKPGGSLRLSCAASG-FTFS | S-----YGMN | WVRQAPGKGLEWVS | SISGS---STYIYYADSVKG | RFTISRDNAKNSLYLQMNSLRAEDTAVYYCAR | TNG-------------------------MDV | WGQGTTVTVSS |
| | | Germline | | H_FR1 | H_CDR1 | H_FR2 | H_CDR2 | H_FR3 | H_CDR3 | H_FR4 |
| | VH3\|3-23/D6\|6-19\|RF2/JH6 | | 32568 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GIAVAGYY---------------YYYGMDV | WGQGTTVTVSS |
| iPS:393096 | 21-225_34D11 | VH3\|3-23/D6\|6-19\|RF2/JH6 | | EVQLSES-GGGLVQPGGSLRLSCAASG-FTFS | S-----YAMN | WVRQAPGKGLEWVS | AISGR---GGSTFHADSVKG | RFTISRDNSKNTLYLQMNSLRAEDTAVYYCVK | GELVEDY----------------YFYGMDV | WGQGTTVTVSS |
| | | Germline | | H_FR1 | H_CDR1 | H_FR2 | H_CDR2 | H_FR3 | H_CDR3 | H_FR4 |
| | VH3\|3-30.3/D4\|4-23\|RF2/JH3 | | 32569 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | DYGGNS-------------------DAFDI | WGQGTMVTVSS |
| iPS:393172 | 21-225_3B12 | VH3\|3-30.3/D4\|4-23\|RF2/JH3 | | QVHLVES-GGGVVQPGRSLRLSCAASG-FTFS | Y-----YGMH | WVRQAPGKGLEWVA | VISYD---GSNKYYADSVKG | RFTISRDNSKNTLHLQMNSLRAEDTAVYYCAR | DRRGGYG---------------VPDAFDI | WGQGTMVTVSS |
| | | Germline | | H_FR1 | H_CDR1 | H_FR2 | H_CDR2 | H_FR3 | H_CDR3 | H_FR4 |
| | VH3\|3-30.3/D4\|4-23\|RF2/JH6 | | 32570 | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | DYGGNSYY---------------YYYGMDV | WGQGTTVTVSS |
| iPS:393174 | 21-225_15D8 | VH3\|3-30.3/D4\|4-23\|RF2/JH6 | | QVQLVES-GGGVVQTGRSLRLSCAASG-FTFS | G-----YGMH | WVRQAPGKGLEWVS | VISYD---GSNKYYADSVKG | RFTISRDNSKNTLYLQMNSLRAEDTAVYYCAR | DRVYCSSTSCV---------PYYDYYGMDV | WGQGTTVTVSS |
| | | Germline | | H_FR1 | H_CDR1 | H_FR2 | H_CDR2 | H_FR3 | H_CDR3 | H_FR4 |
| | VH3\|3-15/D7\|7-27\|RF1/JH6 | | 32571 | EVQLVES-GGGLVKPGGSLRLSCAASG-FTFS | N-----AWMS | WVRQAPGKGLEWVG | RIKSKT-DGGTTDYAAPVKG | RFTISRDDSKNTLYLQMNSLKTEDTAVYYCTT | LTGYYY-----------------YYGMDV | WGQGTTVTVSS |
| iPS:393194 | 21-225_16D2 | VH3\|3-15/D7\|7-27\|RF1/JH6 | | EVQLVES-GGGLVKPGGSLRLSCAASG-FTFS | N-----AWMN | WVRQAPGKGLEWVG | RIKSKT-DGGTTDYAAPVKG | RFTISRDDSKNTLYLQMHSLKTEDTAVYYCTT | DTGPIAARLA-----------YYYYYAMDV | WGQGTTVTVSS |
| iPS:398488 | 21-225_19F6 | VH3\|3-15/D7\|7-27\|RF1/JH6 | | EVQLVES-GGGLVKPGGSLRLSCAASG-FTFS | N-----AWMN | WVRQAPGKGLEWVG | RIKSKT-DGGTTDYAAPVKG | RFTISRDDSKNTLYLQMNSLKTEDTAVYYCTT | DTGPIAARLA-----------YYYYYAMDV | WGHGTTVTVSS |
| iPS:398544 | 21-225_7C8 | VH3\|3-15/D7\|7-27\|RF1/JH6 | | EVQLVES-GGGLVKPGGSLRLSCAASG-FTFS | N-----ARMN | WVRLAPGKGLEWVG | RIKSKT-DGGTTDYAAPVKG | RFTISRDESENTLYLQMNSLKTEDTGVYYCST | DTGPIAARLA-----------YYYYYAMDV | WGQGTTVTVSS |
| iPS:402231 | 21-225_6D9 | VH3\|3-15/D7\|7- | | EVQLVES-GGGLVKPGGSLRL | N-----AWMN | WVRLAPGKGLEWVG | RIKSKT-DGGTTDYAA | RFTISRDDSENTLYLQMNSLKTEDTAVYYCST | DTGPIAARLA-----------YYYYYAMDV | WGQGTTVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH3\|3-23/D4\|4-11\|RF2/JH4 | | 32572 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | DYSNY-----------------------YFDY | WGQGTLVTVSS |
| iPS:393870 | 21-225_7B1 | VH3\|3-23/D4\|4-11\|RF2/JH4 | | EVQLLES-GGGLVQPGGSLRLSCAASG-FTFS | S-----YDMS | WVRQAPGKGLEWVS | TISGS---GGITYYADSVKG | RFTISRDNSKKTLYLQMNSLRAEDTAVYFCAR | DRG------------------------SV | WGQGTLVTVSS |
| | | Germline | | H_FR1 | H_CDR1 | H_FR2 | H_CDR2 | H_FR3 | H_CDR3 | H_FR4 |
| | VH4\|4-39/D4\|4-17\|RF2/JH1 | | 32573 | QLQLQES-GPGLVKPSETLSLTCTVSG-GSIS | SS---SYYWG | WIRQPPGKGLEWIG | SIYY----SGSTYYNPSLKS | RVTISVDTSKNQFSLKLSSVTAADTAVYYCAR | DYGDYA-------------------EYFQH | WGQGTLVTVSS |
| iPS:393872 | 21-225_2A11 | VH4\|4-39/D4\|4-17\|RF2/JH1 | | QLQLQES-GPGLVKPSETLSLTCTVSG-GSIS | RS---SYYWG | WIRQPPGKGLEWIG | NIYY----SGSTYYNPSVKS | RVTISVDTSKNQFSLKLSTVTAADTAVYYCAR | HGKDW--------------------GLED | WGQGTLVTVSS |
| | | Germline | | H_FR1 | H_CDR1 | H_FR2 | H_CDR2 | H_FR3 | H_CDR3 | H_FR4 |
| | VH4\|4-59/D6\|6-6\|RF1/JH4 | | 32574 | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAR | EYSSS--------------------SYFDY | WGQGTLVTVSS |
| iPS:393890 | 21-225_4B1 | VH4\|4-59/D6\|6-6\|RF1/JH4 | | QVQLQES-GPGLVKPSETLSLTCSVSG-DSIS | S-----YSWS | WIRQPAGKGLEWIG | RIYT----SGSTNYIPSLKS | RITMSVDTSKKQFSLKLSSVTAADTAVYYCAR | DLKSSG-----------------CLFFDY | WGQGTLVTVSS |
| | | Germline | | H_FR1 | H_CDR1 | H_FR2 | H_CDR2 | H_FR3 | H_CDR3 | H_FR4 |
| | VH3\|3-33/D4\|4-17\|RF1/JH6 | | 32575 & 32576 & 32577 | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | *LR*LYY----------------YYYGMDV | WGQGTTVTVSS |
| iPS:393926 | 21-225_4G4 | VH3\|3-33/D4\|4-17\|RF1/JH6 | | QVQLVES-GGGVVQPGRSLRLSCAASG-FTFS | S-----YGMH | WVRQAPGKGLEWGA | VIWHD---GSNKYYADSVKG | RFTISRDNAKNTLYLQMNSLRAEDTAVYYCAR | DLRMG--------------------GMDV | WGQGTTVTVSS |
| | | Germline | | H_FR1 | H_CDR1 | H_FR2 | H_CDR2 | H_FR3 | H_CDR3 | H_FR4 |
| | VH3\|3-23/D6\|6-6\|RF2/JH2 | | 32578 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | SIAARY-------------------WYFDL | WGRGTLVTVSS |
| iPS:393936 | 21-225_14A11 | VH3\|3-23/D6\|6-6\|RF2/JH2 | | EVQLLES-GGGLVQPGGSLRLSCAASG-FTFS | S-----YAMN | WVRQAPGKGLEWVS | VISGR---GGSTYYADSVKG | RFTISRDNSKNTLYLQMNSLRAEDTAVYYCAK | RIAAGM-------------------EYFDL | WGRGTLVTVSS |
| | | Germline | | H_FR1 | H_CDR1 | H_FR2 | H_CDR2 | H_FR3 | H_CDR3 | H_FR4 |
| | VH3\|3-21/D1\|1-1\|RF3/JH4 | | 32579 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | YNWND----------------------YFDY | WGQGTLVTVSS |
| iPS:393988 | 21-225_7F10 | VH3\|3-21/D1\|1- | | EVQLVES-GGGLVKPGGSLRL | S-----YSMN | WVRQAPGKGLEWVS | SISSS---SSYIYYADS | RFTISRDNAKNSLYLQMNSLRTEDTAVYYCAR | ANL------------------------FDY | WGQGTLVTVS |

<table>
<tr><th></th><th></th><th>Germline</th><th></th><th>H_FR1</th><th>H_CDR1</th><th>H_FR2</th><th>H_CDR2</th><th>H_FR3</th><th>H_CDR3</th><th>H_FR4</th></tr>
<tr><td></td><td>VH4|4-39/D6|6-6|RF1/JH4</td><td></td><td>32580</td><td>QLQLQES-GPGLVKPSETLSLTCTVSG-GSIS</td><td>SS---SYYWG</td><td>WIRQPPGKGLEWIG</td><td>SIYY----SGSTYYNPSLKS</td><td>RVTISVDTSKNQFSLKLSSVTAADTAVYYCAR</td><td>EYSSS--------------------SYFDY</td><td>WGQGTLVTVSS</td></tr>
<tr><td>iPS:393990</td><td>21-225_11G7</td><td>VH4|4-39/D6|6-6|RF1/JH4</td><td></td><td>QLQLQES-GPGLVKPSETLSLTCTVSG-GFIS</td><td>RS---TYYWG</td><td>WIRQPPGKGLEWIG</td><td>SIYY----SGSTSYSPSLKS</td><td>RVTISVDTSKNQFSLKLSSVTAADTAVYYCAR</td><td>LNSSW--------------------SFDY</td><td>WGQGTLVTVSS</td></tr>
<tr><td></td><td></td><td>Germline</td><td></td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH3|3-30.3/D2|2-15|RF3/JH6</td><td></td><td>32581</td><td>QVQLVES-GGGVVQPGRSLRLSCAASG-FTFS</td><td>S-----YAMH</td><td>WVRQAPGKGLEWVA</td><td>VISYD---GSNKYYADSVKG</td><td>RFTISRDNSKNTLYLQMNSLRAEDTAVYYCAR</td><td>DIVVVVAAT-----------YYYYGMDV</td><td>WGQGTTVTVSS</td></tr>
<tr><td>iPS:394010</td><td>21-225_12G5</td><td>VH3|3-30.3/D2|2-15|RF3/JH6</td><td></td><td>QVQLVES-GGGVVQPGRSLRLSCAASG-FTFS</td><td>N-----YGIY</td><td>WVRQAPGKGLEWVA</td><td>VISYD---GSNKYYADSVKG</td><td>RFTISRDNSKNTLYLQMNSLRAEDTAVYYCAR</td><td>DRGAVAAY---------------YYYGIDV</td><td>WGQGTTVTVSS</td></tr>
<tr><td></td><td></td><td>Germline</td><td></td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH1|1-08/D2|2-21|RF1/JH4</td><td></td><td>32582 & 32583</td><td>QVQLVQS-GAEVKKPGASVKVSCKASG-YTFT</td><td>S-----YDIN</td><td>WVRQATGQGLEWMG</td><td>WMNPN---SGNTGYAQKFQG</td><td>RVTMTRNTSISTAYMELSSLRSEDTAVYYCAR</td><td>SILWW*L------------------LFYFDY</td><td>WGQGTLVTVSS</td></tr>
<tr><td>iPS:394065</td><td>21-225_11E2</td><td>VH1|1-08/D2|2-21|RF1/JH4</td><td></td><td>QVQVVQS-GAEVKKPGASVKVSCKASG-YTFT</td><td>N-----YDIN</td><td>WVRQATGQGLEWMG</td><td>WMNTN---SGNTGYAQKFQG</td><td>RVTMTRNTSISTAYMDLSSLRSEDTAVYYCAY</td><td>SHGWF--------------------LFDY</td><td>WGQGILVTVSS</td></tr>
<tr><td>iPS:398506</td><td>21-225_23G12</td><td>VH1|1-08/D2|2-21|RF1/JH4</td><td></td><td>QVQLLQS-GAEVKKPGASVKVSCKASG-YTFT</td><td>N-----YDIN</td><td>WVRQATGQGLEWMG</td><td>WMYPN---SGNTGYAQKFQG</td><td>RVTMTMNTSISTAYMELSSLRSEDTAVYYCAI</td><td>SGGWY--------------------YFDY</td><td>WGQGTLVTVSS</td></tr>
<tr><td>iPS:398512</td><td>21-225_25E12</td><td>VH1|1-08/D2|2-21|RF1/JH4</td><td></td><td>QVQLVQS-GAEVKKPGASVKVSCKASG-YTFT</td><td>N-----YDIN</td><td>WVRQATGQGLEWMG</td><td>WMNPN---SGNTGYAQKFQG</td><td>RVTMTRNTSISTAYLELSSLRSEDTAVYYCAG</td><td>SNGWY--------------------YFDY</td><td>WGQGTLVTVSS</td></tr>
<tr><td></td><td></td><td>Germline</td><td></td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH3|3-33/D7|7-27|RF2/JH3</td><td></td><td>32584 & 32585</td><td>QVQLVES-GGGVVQPGRSLRLSCAASG-FTFS</td><td>S-----YGMH</td><td>WVRQAPGKGLEWVA</td><td>VIWYD---GSNKYYADSVKG</td><td>RFTISRDNSKNTLYLQMNSLRAEDTAVYYCAR</td><td>*LGD--------------------AFDI</td><td>WGQGTMVTVSS</td></tr>
<tr><td>iPS:394091</td><td>21-225_13H3</td><td>VH3|3-33/D7|7-27|RF2/JH3</td><td></td><td>QVQLVES-GGGLVQPGRSLRLSCAASG-FTFS</td><td>S-----YGMH</td><td>WVRQAPGKGLEWVA</td><td>VIWYE---ESNKYYVDSVRG</td><td>RFTISRDNSKSTLYLQMNSLRAEDTAVYYCVR</td><td>ELGF---------------------QSDF</td><td>WGQGTPVTVSS</td></tr>
<tr><td></td><td></td><td>Germline</td><td></td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH3|3-23/D4|4-23|RF2/JH6</td><td></td><td>32586</td><td>EVQLLES-GGGLVQPGGSLRLSCAASG-FTFS</td><td>S-----YAMS</td><td>WVRQAPGKGLEWVS</td><td>AISGS---GGSTYYADSVKG</td><td>RFTISRDNSKNTLYLQMNSLRAEDTAVYYCAK</td><td>DYGGNSYY---------------YYYGMDV</td><td>WGQGTTVTVSS</td></tr>
<tr><td>iPS:398472</td><td>21-225_16E4</td><td>VH3|3-23/D4|4-</td><td></td><td>EVQLLES-GGGLIQPGGSLRL</td><td>S-----YVMS</td><td>WVRQAPGKGLEWVS</td><td>TISVG---GGTTYYADS</td><td>RFTISRDNSKNTLYLQMNSLRAEDTAVYYCAK</td><td>WGRGNSYE---------------YYYGMDV</td><td>WGQGTTVTVS</td></tr>
</table>

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH2\|2-05/D6\|6-19\|RF2/JH4 | | 32587 | QITLKES-GPTLVKPTQTLTLTCTFSG-FSLS | TS---GVGVG | WIRQPPGKALEWLA | LIYW----NDDKRYSPSLKS | RLTITKDTSKNQVVLTMTNMDPVDTATYYCAH | GIAVA---------------------GYFDY | WGQGTLVTVSS |
| iPS:398498 | 21-225_22E6 | VH2\|2-05/D6\|6-19\|RF2/JH4 | | QITLKES-GPTLVKPTQTLTLTCTFSG-FSLS | TG---GVGVG | WIRQPPGKALEWLA | LIYW----NDDKRYSPSLKS | RLTITRDTSKNQVVLTMTNMDPVDTATYYCAH | TIAVR---------------------GFDY | WGQGTLVTVSS |
| | | Germline | | H_FR1 | H_CDR1 | H_FR2 | H_CDR2 | H_FR3 | H_CDR3 | H_FR4 |
| | VH1\|1-08/D5\|5-24\|RF2/JH4 | | 32588 & 32589 | QVQLVQS-GAEVKKPGASVKVSCKASG-YIFT | S-----YDIN | WVRQATGQGLEWMG | WMNPN---SGNTGYAQKFQG | RVTMTRNTSISTAYMELSSLRSEDTAVYYCAR | *RWLQ---------------------LYFDY | WGQGTLVTVSS |
| iPS:398536 | 21-225_33D12 | VH1\|1-08/D5\|5-24\|RF2/JH4 | | QVQLVQS-GAEVKKPGASVKVSCKASG-YTFT | S-----YDIS | WVRLATGQGLEWMG | WMNPN---SGNTGYAQKFQG | RVTMTRNTSISTAYMELSSLRSEDTAVYYCAR | KRA-----------------------NDY | WGQGTLVTVSS |
| | | Germline | | H_FR1 | H_CDR1 | H_FR2 | H_CDR2 | H_FR3 | H_CDR3 | H_FR4 |
| | VH2\|2-05/D6\|6-6\|RF1/JH4 | | 32590 | QITLKES-GPTLVKPTQTLTLTCTFSG-FSLS | TS---GVGVG | WIRQPPGKALEWLA | LIYW----NDDKRYSPSLKS | RLTITKDTSKNQVVLTMTNMDPVDTATYYCAH | EYSSS---------------------SYFDY | WGQGTLVTVSS |
| iPS:398546 | 21-225_9H10 | VH2\|2-05/D6\|6-6\|RF1/JH4 | | QITLKES-GPTLVKPTQTLTLTCTFSG-FSLT | TS---GVGVG | WIRQPPGKALEWLA | LIYW----SDDKRYSPSLKS | RLTITKDTSKNQVVLTMTNMAPVDTATYYCAH | TGSSC---------------------CYFDY | WGQGTLVTVSS |
| | | Germline | | H_FR1 | H_CDR1 | H_FR2 | H_CDR2 | H_FR3 | H_CDR3 | H_FR4 |
| | VH3\|3-21/D1\|1-1\|RF2/JH6 | | 32591 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | VQLERYY-----------------YYYGMDV | WGQGTTVTVSS |
| iPS:402229 | 21-225_16H9 | VH3\|3-21/D1\|1-1\|RF2/JH6 | | EVQLVES-GGGLVKPGGSLRLSCAASG-FTFS | S-----YSMN | WVRQAPGKGLEWVS | SISGS---SSYIYYADSVKG | RFTISRDNAKNSLYLQMNSLRAEDTAVYYCAR | VNG-----------------------MDV | WGQGTTVTVSS |
| | | Germline | | H_FR1 | H_CDR1 | H_FR2 | H_CDR2 | H_FR3 | H_CDR3 | H_FR4 |
| | VH3\|3-21/D4\|4-11\|RF3/JH5 | | 32592 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | TTVTN---------------------WFDP | WGQGTLVTVSS |
| iPS:402233 | 21-225_16D10 | VH3\|3-21/D4\|4-11\|RF3/JH5 | | EVQLVES-GGGLVKPGGSLRLSCAASG-FTFS | T-----YNLN | WVRQAPGKGLEWVS | SISGG---AGHIYYSDSVKG | RFTISRDNAKNSLYLQMNSLRAEDTAVYYCAR | TNG-----------------------FDF | WGQGTLVTVSS |
| | | Germline | | H_FR1 | H_CDR1 | H_FR2 | H_CDR2 | H_FR3 | H_CDR3 | H_FR4 |
| | VH3\|3-21/D1\|1-1\|RF1/JH3 | | 32593 | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | GTTGT---------------------DAFDI | WGQGTMVTVSS |
| iPS:402235 | 21-225_20F10 | VH3\|3-21/D1\|1- | | EVQLVES-GGGLVKPGGSLRL | S-----YSMN | WVRQAPGKGLEWVS | SIST----STFIYYADS | RFTISRDNAKNSLYLQMNSLRAEDTAVYYCAR | KAG-----------------------LDI | WGQGTMVTVS |

| | | Germline | | H_FR1 | H_CDR1 | H_FR2 | H_CDR2 | H_FR3 | H_CDR3 | H_FR4 |
|---|---|---|---|---|---|---|---|---|---|---|
| | VH3\|3-23/D5\|5-12\|RF1/JH3 | | 32594 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | VDIVAT-------------------IDAFDI | WGQGTMVTVSS |
| iPS:403870 | 21-225_23G4 | VH3\|3-23/D5\|5-12\|RF1/JH3 | | EVQLLES-GGGLVQPGGSLRLSCAASG-FTFS | S-----YAMS | WVRQAPGKGLEWVS | VISGR---GGSTYYADSVKG | RFTISRDNSKNTLYLQMNSLRAEDTAVYYCAK | RGIVGA------------------TEAFDI | WGQGTMVTVSS |
| | | Germline | | H_FR1 | H_CDR1 | H_FR2 | H_CDR2 | H_FR3 | H_CDR3 | H_FR4 |
| | VH4\|4-39/D4\|4-11\|RF1/JH4 | | 32595 & 32596 & 32597 | QLQLQES-GPGLVKPSETLSLTCTVSG-GSIS | SS---SYYWG | WIRQPPGKGLEWIG | SIYY----SGSTYYNPSLKS | RVTISVDTSKNQFSLKLSSVTAADTAVYYCAR | *LQ*L---------------------YFDY | WGQGTLVTVSS |
| iPS:403872 | 21-225_8F11 | VH4\|4-39/D4\|4-11\|RF1/JH4 | | QLQLQES-GPGLVQPSETLSLTCTVSG-VSIS | RT---SYYWG | WLRQPPGKGLEWIG | NIYY----SGSAYNNPSLKS | RVTISVDTSKNQFSLKLSSVTAADTAVYYCGR | HGQDW---------------------GLDY | WGQGTLVTVSS |
| | | Germline | | H_FR1 | H_CDR1 | H_FR2 | H_CDR2 | H_FR3 | H_CDR3 | H_FR4 |
| | VH1\|1-08/D3\|3-9\|RF2/JH6 | | 32598 | QVQLVQS-GAEVKKPGASVKVSCKASG-YIFT | S-----YDIN | WVRQATGQGLEWMG | WMNPN---SGNTGYAQKFQG | RVTMTRNTSISTAYMELSSLRSEDTAVYYCAR | -YYDILTGYYN-----------YYYYGMDV | WGQGTTVTVSS |
| iPS:437240 | 21-225_84H12 | VH1\|1-08/D3\|3-9\|RF2/JH6 | | QVQLVQS-GAEVKKPGASVKVSCKASG-YTFT | S-----YDIN | WVRQATGQGLEWMG | WLNPH---SGNTGYAQKFQG | RITMTWNTSIRTAYMELSSLRSEDTAVYYCAR | GFYDILTGYS-----------PTYYYYDMDV | WGQGTTVTVSS |
| iPS:434577 | 21-225_75C11 | VH1\|1-08/D3\|3-9\|RF2/JH6 | | QVQLVQS-GAEVKKPGASVKVSCKASG-YTFT | S-----YDIN | WVRQATGQGLEWMG | WLNPH---SGNTGYAQKFQG | RITMTWNTSIRTAYMELSSLRSEDTAVYYCAR | GFYDILTGYS-----------PTYYYYDMDV | WGQGTTVTVSS |
| iPS:434553 | 21-225_76H12 | VH1\|1-08/D3\|3-9\|RF2/JH6 | | QVQLVQS-GAEVKKPGASVKVSCKASG-YTFT | S-----YDIN | WVRQATGQGLEWMG | WLNPH---SGNTGYAQKFQG | RITMTWNTSIRTAYMELSSLRSEDTAVYYCAR | GFYDILTGYS-----------PTYYYYDMDV | WGQGTTVTVSS |
| iPS:434927 | 21-225_86E5 | VH1\|1-08/D3\|3-9\|RF2/JH6 | | QVQLVQS-GAEVKKPGASVKVSCKASG-YTFT | S-----YDIN | WVRQATGQGLEWMG | WMNPN---SGNTGYAQKFQG | RVTMTWNTSIRTAYMELSSLRSEDTAVYYCAR | GFYDILTGYS-----------PTYYYYDMDV | WGQGTTVTVSS |
| | | Germline | | H_FR1 | H_CDR1 | H_FR2 | H_CDR2 | H_FR3 | H_CDR3 | H_FR4 |
| | VH3\|3-23/D6\|6-19\|RF2/JH4 | | 32599 | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | -GIAVA--------------------GYFDY | WGQGTLVTVSS |
| iPS:435477 | 21-225_154E8 | VH3\|3-23/D6\|6-19\|RF2/JH4 | | EVQLLES-GGGLVQPGGSLRLSCAASG-FTFS | S-----YAMS | WVRQAPGRGLEWVS | AISGS---GGNTFYADSVKG | RFTISRDNSKNTLYLQMNSLRAEDTAVYYCAI | HGIAVAGT----------------GAHYFDY | WGQGTLATVSS |
| iPS:435385 | 21-225_149G7 | VH3\|3-23/D6\|6- | | EVQLLES-GGGLVQPGGSLRL | S-----YAMS | WVRQAPGKGLEWVS | AISGS---GGNTFYADS | RFTISRDNSKNTLYLQMNSLRAEDTAVYYCAI | HGIAVAGT----------------GAHYFDY | WGQGTLATVS |

## Figure 52 - Table 5
## Standard IgG Antibody Variable Region Consensus Protein Alignment
## CDRs defined by Kabat
## Alignment numbering defined by Amgen Reference (AHo)

| KAPPA_VARIABLE | | | | | | | | | |
|---|---|---|---|---|---|---|---|---|---|
| | | Germline | K_FR1 | K_CDR1 | K_FR2 | K_CDR2 | K_FR3 | K_CDR3 | K_FR4 |
| | VK4\|B3/JK3 | | DIVMTQSPDSLA<br>VSLGERATINC | KSS--<br>QSVLYSSNNKN<br>YLA | WYQQKPGQ<br>PPKLLIY | W-------<br>-ASTRES | GVPDRFSGSGSG--<br>TDFTLTISSLQAEDVAV<br>YYC | QQYYS-------------<br>----------TPFT | FGPGTK<br>VDIK |
| iPS:42<br>6126 | 21-225_6G6 | VK4\|B3/J<br>K3 | ............<br>........... | .........HN<br>...Y.... | ........<br>..N...F | ........<br>....... | ..........F......<br>..N.............. | ....D.............<br>............... | ..H...<br>.... |
| iPS:41<br>2232 | 21-225_4A2 | VK4\|B3/J<br>K3 | ............<br>........... | .......I.H.<br>...N.... | .F......<br>.....L. | ........<br>....... | .................<br>.......P........ | ....N.............<br>...........V. | ......<br>.G.. |
| iPS:45<br>1141 | 21-<br>225_164B11 | VK4\|B3/J<br>K3 | ............<br>........... | .......L.K.<br>....S... | S.......<br>L...... | ........<br>...S... | .................<br>............L... | ..................<br>.........I.P. | ..H..N<br>...T |
| iPS:42<br>3314 | 21-<br>225_12F11 | VK4\|B3/J<br>K3 | ............<br>........... | .........H.<br>...Y.... | ........<br>..N...F | ........<br>....... | ..........F......<br>..N.............. | ....D.............<br>............... | ......<br>.... |
| iPS:43<br>5327 | 21-<br>225_147G6 | VK4\|B3/J<br>K3 | ............<br>........... | ...........<br>..SN.... | ........<br>....... | ........<br>...A... | .................<br>................ | ....T.............<br>...........P. | ......<br>.... |
| iPS:43<br>5345 | 21-<br>225_148G3 | VK4\|B3/J<br>K3 | ............<br>........H. | ......R..H.<br>...Y.... | ........<br>....... | ........<br>.....D. | ..............A..<br>............L... | ..................<br>............... | ......<br>.... |
| iPS:43<br>5405 | 21-<br>225_150B7 | VK4\|B3/J<br>K3 | ............<br>........... | ......N....<br>.H.N.... | ........<br>....... | ........<br>.....K. | .................<br>....T........... | ..................<br>............... | ......<br>.... |
| iPS:43<br>5433 | 21-<br>225_152E3 | VK4\|B3/J<br>K3 | ............<br>........... | .........H.<br>...Y...V | ........<br>S..R... | ........<br>....... | .................<br>S............... | ..................<br>............... | ......<br>.... |
| iPS:43<br>5437 | 21-<br>225_152F4 | VK4\|B3/J<br>K3 | ............<br>........... | ...........<br>...Y.... | ........<br>....... | ........<br>.....K. | .................<br>...V............ | ...FN.............<br>...........P. | ......<br>.... |
| iPS:43<br>5649 | 21-<br>225_165H2 | VK4\|B3/J<br>K3 | ...........T<br>..P........ | .........H.<br>.......T | ........<br>....... | ........<br>....... | ...V.............<br>.VP...M.DD.....R | ..S...............<br>.........I.P. | .....N<br>.... |
| iPS:43<br>5855 | 21-<br>225_191G3 | VK4\|B3/J<br>K3 | ............<br>.........D. | .........H.<br>..SY.... | .....L..<br>....... | ........<br>.....K. | .................<br>............F... | ..................<br>.........S.P. | ......<br>M... |
| iPS:43<br>5903 | 21-<br>225_190E2 | VK4\|B3/J<br>K3 | ............<br>........... | .........FN<br>........ | ........<br>..N.... | ........<br>....... | .................<br>.....M.......... | ...C..............<br>.........L... | ......<br>...R |
| iPS:43<br>5915 | 21-<br>225_190H4 | VK4\|B3/J<br>K3 | AN..........<br>......T.... | .........H.<br>...Y.... | ..R.....<br>....... | ........<br>....... | .................<br>................ | ..................<br>.........I.P. | ......<br>.... |
| iPS:43<br>5923 | 21-<br>225_190H6 | VK4\|B3/J<br>K3 | ............<br>........... | .........FN<br>........ | ........<br>..N.... | ........<br>....... | ..........C......<br>................ | ...C..............<br>.........L... | ......<br>...R |
| iPS:43<br>5953 | 21-<br>225_191B12 | VK4\|B3/J<br>K3 | ............<br>........... | .........FN<br>........ | ........<br>....... | ........<br>....... | .................<br>....N......I... | ...S..............<br>.........L... | ......<br>.... |
| iPS:43 | 21- | VK4\|B3/J | ............ | .........FN | ........ | ........ | ..........C...... | ...C.............. | ...... |

| | | | | | | | | | |
|---|---|---|---|---|---|---|---|---|---|
| iPS:43 6102 | 21-225_196B1 | VK4\|B3/JK3 | ............<br>.F......... | ........I.F.<br>....R... | ........<br>....... | ........<br>...I... | .................<br>................. | ...S..............<br>..........L... | ......<br>.... |
| iPS:43 6104 | 21-225_196C1 | VK4\|B3/JK3 | ............<br>........... | ..........FN<br>........ | ........<br>L.N.... | ........<br>....L.. | ...........C.....<br>.....M........... | ...C..............<br>..........F... | ......<br>...R |
| iPS:43 6156 | 21-225_197C8 | VK4\|B3/JK3 | .....P......<br>........... | ..........H.<br>........ | ........<br>....... | ........<br>....... | .................<br>..S.............. | ..S.T.............<br>..........I... | ......<br>..N. |
| iPS:43 6270 | 21-225_203F10 | VK4\|B3/JK3 | ............<br>........... | .........FFH<br>........ | ........<br>....... | ........<br>....... | .................<br>............T.... | ...F..............<br>..........L... | ......<br>...T |
| iPS:43 6570 | 21-225_225F4 | VK4\|B3/JK3 | ............<br>........... | ............<br>........ | ........<br>....... | ........<br>....... | ......T..........<br>.....CV.P....... | H..HN.............<br>..........S.P. | ..H..E<br>.... |
| iPS:39 4065 | 21-225_11E2 | VK4\|B3/JK3 | ............<br>........... | ..N...R..S.<br>...H.... | ....R...<br>....... | ........<br>....... | .................<br>................. | ...F..............<br>.............. | ......<br>.... |
| | | Germline | **K_FR1** | **K_CDR1** | **K_FR2** | **K_CDR2** | **K_FR3** | **K_CDR3** | **K_FR4** |
| | VK1\|A30/JK5 | | DIQMTQSPSSLS<br>ASVGDRVTITC | RAS--QGIR--<br>----NDLG | WYQQKPGK<br>APKRLIY | A-------<br>-ASSLQS | GVPSRFSGSGSG--<br>TEFTLTISSLQPEDFAT<br>YYC | LQHNS------------<br>----------YPIT | FGQGTR<br>LEIK |
| iPS:47 3253 | 21-225_7C3_LC1 | VK1\|A30/JK5 | ............<br>........... | ...........<br>....S... | ....N.V.<br>....... | ........<br>....... | .................<br>...V........F... | ..................<br>.........YL... | ......<br>.... |
| iPS:47 3256 | 21-225_9F12_LC2 | VK1\|A30/JK5 | ............<br>........... | ...........<br>........ | ......V.<br>....... | ........<br>....... | .................<br>................. | ..................<br>.........YL... | ......<br>.... |
| iPS:45 3449 | 21-225_208A2 | VK1\|A30/JK5 | ............<br>........... | .T.........<br>........ | ....Q...<br>T...... | ........<br>.....L. | .........R.....D.<br>................. | ..Y...............<br>...........P. | ......<br>.... |
| iPS:43 4467 | 21-225_73H8 | VK1\|A30/JK5 | ...........Y<br>..........R | ......D....<br>........ | ........<br>.L..V.. | ........<br>....... | ....S............<br>................G | I.................<br>.........YP... | V.....<br>.... |
| iPS:43 5045 | 21-225_90H5 | VK1\|A30/JK5 | ............<br>........... | ...........<br>........ | ........<br>....... | I.......<br>....... | .................<br>................. | ..................<br>.............. | ......<br>.... |
| iPS:43 5561 | 21-225_159F1 | VK1\|A30/JK5 | ............<br>........... | ......RV...<br>........ | ......A.<br>....I.F | D.......<br>...N... | .................<br>................. | ...H..............<br>..........F... | ......<br>.... |
| iPS:43 6328 | 21-225_207F12 | VK1\|A30/JK5 | ............<br>........... | ...........<br>........ | ........<br>....... | ........<br>....... | .................<br>...............F. | ..................<br>...........L. | ......<br>.... |
| iPS:43 6354 | 21-225_210G10 | VK1\|A30/JK5 | ............<br>..........F | .T.........<br>........ | ....Q...<br>T...M.. | ........<br>.....F. | .........R.....D.<br>................. | ..Y...............<br>...........P. | ......<br>.... |
| iPS:39 3094 | 21-225_34C4 | VK1\|A30/JK5 | ............<br>........... | ...........<br>........ | ........<br>....... | T.......<br>...N... | .................<br>................. | ...S..............<br>.............. | ......<br>.... |
| iPS:39 8484 | 21-225_18D4 | VK1\|A30/JK5 | ............<br>........... | ...........<br>........ | ........<br>....... | ........<br>.....E. | .................<br>...V............. | .H..N.............<br>.........YL... | ......<br>.... |
| | | Germline | **K_FR1** | **K_CDR1** | **K_FR2** | **K_CDR2** | **K_FR3** | **K_CDR3** | **K_FR4** |
| | VK1\|L5/JK3 | | DIQMTQSPSSVS<br>ASVGDRVTITC | RAS--QGIS--<br>----SWLA | WYQQKPGK<br>APKLLIY | A-------<br>-ASSLQS | GVPSRFSGSGSG--<br>TDFTLTISSLQPEDFAT<br>YYC | QQANS------------<br>----------FPFT | FGPGTK<br>VDIK |

| | | | | | | | | | |
|---|---|---|---|---|---|---|---|---|---|
| iPS:47 3254 | 21-225_7C3_LC2 | VK1\|L5/JK3 | .............<br>..L........ | ...........<br>........ | .....Q..<br>......F | ........<br>...R... | .A...............<br>................. | ...................<br>............... | ......<br>.... |
| iPS:47 3255 | 21-225_9F12_LC1 | VK1\|L5/JK3 | .............<br>........... | ...........<br>....R... | ........<br>....... | ........<br>...R... | .................<br>.....G.L.....I... | ...................<br>............... | ......<br>..F. |
| iPS:42 6108 | 21-225_10G6 | VK1\|L5/JK3 | .............<br>........... | ...........<br>....K... | ........<br>...I... | ........<br>..Y.... | ...A.............<br>....R............ | ...................<br>............... | ......<br>.... |
| iPS:42 6110 | 21-225_12E9 | VK1\|L5/JK3 | .............<br>...R....... | ...........<br>........ | .......E<br>....... | ........<br>...R... | .................<br>................. | ...................<br>............... | ......<br>.... |
| iPS:45 3447 | 21-225_65F10 | VK1\|L5/JK3 | .............<br>........... | .GG........<br>....T... | ........<br>....... | ........<br>...I... | .....R.R........<br>................. | ..G.I..............<br>............... | ..R...<br>.... |
| iPS:45 3451 | 21-225_52G11 | VK1\|L5/JK3 | .............<br>........... | ...........<br>....K... | ........<br>.....L. | ........<br>....... | .......A.........<br>................. | ...................<br>............... | ......<br>..V. |
| iPS:45 3453 | 21-225_53F2 | VK1\|L5/JK3 | .............<br>........... | ...........<br>....K... | ........<br>..N..L. | ........<br>....... | ....K............<br>................. | ...................<br>............... | ......<br>..V. |
| iPS:43 3915 | 21-225_43H9 | VK1\|L5/JK3 | .............<br>........... | ......D....<br>........ | ...K....<br>....... | D.......<br>....... | .................<br>I.............F. | ...................<br>.........L... | ......<br>.... |
| iPS:43 3925 | 21-225_44F3 | VK1\|L5/JK3 | .............<br>........... | ...........<br>....D... | ........<br>....... | ........<br>....... | ..........F......<br>................. | ...................<br>............... | ......<br>.... |
| iPS:43 3953 | 21-225_45H4 | VK1\|L5/JK3 | .............<br>........... | ......D....<br>........ | ...K....<br>...Y... | D.......<br>....... | .................<br>I..........I.F. | ...................<br>.........L... | ......<br>.... |
| iPS:43 3959 | 21-225_45C9 | VK1\|L5/JK3 | .............<br>V.......... | ......D....<br>....D... | ....R...<br>....... | ........<br>.....E. | .................<br>................. | ...................<br>............... | ......<br>.... |
| iPS:43 4023 | 21-225_49F1 | VK1\|L5/JK3 | .............<br>........... | .....RD.N..<br>....G... | ........<br>....... | T.......<br>.V..... | .................<br>................. | ..S................<br>............... | ......<br>.... |
| iPS:43 4027 | 21-225_49H5 | VK1\|L5/JK3 | .............<br>........... | .......F...<br>....T... | .F......<br>....... | ........<br>......D | .................<br>................. | ..T................<br>............... | ......<br>.... |
| iPS:43 4035 | 21-225_49F10 | VK1\|L5/JK3 | .............<br>........... | ...........<br>....R... | ........<br>...V... | ........<br>...T... | .................<br>................. | ...................<br>............... | ......<br>.... |
| iPS:43 4061 | 21-225_51C7 | VK1\|L5/JK3 | .............<br>........... | ......DVN..<br>....NY.. | .F......<br>....... | ........<br>......N | .................<br>.......L......... | ..T................<br>............... | ......<br>.... |
| iPS:43 4065 | 21-225_50D4 | VK1\|L5/JK3 | .............<br>......L.... | ...........<br>....R... | ........<br>...V... | ........<br>...T... | .................<br>................. | ...................<br>............... | ......<br>...R |
| iPS:43 4069 | 21-225_51E9 | VK1\|L5/JK3 | .............<br>........... | ...........<br>........ | ........<br>....... | V.......<br>....... | .................<br>....R............ | ...K...............<br>............... | ......<br>.... |
| iPS:43 4079 | 21-225_52B1 | VK1\|L5/JK3 | .............<br>TF....I.... | ......D.R..<br>....T... | ........<br>....... | ........<br>......N | .................<br>...............F. | ...K...............<br>............... | ......<br>.... |
| iPS:43 4097 | 21-225_52H10 | VK1\|L5/JK3 | .............<br>........... | ......D.N..<br>........ | ........<br>....... | V.......<br>....... | .A...............<br>....R............ | ...K...............<br>............... | ......<br>.... |
| iPS:43 4123 | 21-225_53F7 | VK1\|L5/JK3 | ....S........<br>........... | ...........<br>....R... | ........<br>..N.... | ........<br>....... | .................<br>.............I... | ...................<br>............... | ......<br>.... |
| iPS:43 | | VK1\|L5/J | | V | | | | | |

| | | | | | | | | | |
|---|---|---|---|---|---|---|---|---|---|
| iPS:43 4167 | 21-225_50F3 | VK1\|L5/J K3 | ............<br>........... | ......D....<br>........ | .F......<br>....... | ........<br>......N | ..................<br>.................. | ..T................<br>............... | ......<br>.... |
| iPS:43 4189 | 21-225_56E5 | VK1\|L5/J K3 | ............<br>........... | ........R..<br>....K... | ........<br>....... | ........<br>....... | ..................<br>.................. | ...................<br>............... | ......<br>.... |
| iPS:43 4193 | 21-225_56C6 | VK1\|L5/J K3 | ............<br>........... | ...........<br>........ | .....S.N<br>....... | ........<br>...R... | ................Y.<br>..I.....S......... | ...................<br>............... | ......<br>.... |
| iPS:43 4195 | 21-225_56F6 | VK1\|L5/J K3 | ...........C<br>.Y......... | .V...D....<br>....K... | .F......<br>...F... | V.......<br>...G... | ..................<br>.F................ | ...................<br>............... | ......<br>.... |
| iPS:43 4273 | 21-225_57E4 | VK1\|L5/J K3 | ............<br>.......S... | ......D....<br>....N... | ........<br>....... | ........<br>....... | ..................<br>................S. | ..G................<br>............... | ......<br>.... |
| iPS:43 4277 | 21-225_57A7 | VK1\|L5/J K3 | ............<br>........... | ...........<br>....R... | ........<br>..N.... | ........<br>...N... | ..................<br>.................. | ...................<br>............... | ......<br>.... |
| iPS:43 4355 | 21-225_64G12 | VK1\|L5/J K3 | ............<br>........... | ......N.T..<br>....T... | ........<br>......S | ........<br>....... | ..................<br>................I. | ...................<br>............... | ......<br>L... |
| iPS:43 4389 | 21-225_66F11 | VK1\|L5/J K3 | ...........C<br>........... | .E.........<br>....I... | ........<br>....... | ........<br>....... | ...........Y......<br>.......V.......... | ...................<br>............... | ......<br>.... |
| iPS:43 4423 | 21-225_70D1 | VK1\|L5/J K3 | ............<br>........... | .......V...<br>....R... | ........<br>....... | ........<br>....... | ..................<br>.V................ | ...................<br>............... | ......<br>.... |
| iPS:43 5291 | 21-225_146E1 | VK1\|L5/J K3 | ..K.........<br>........... | ........N..<br>....N..V | ........<br>....... | ........<br>....... | ......R...........<br>.................. | ...................<br>............... | ......<br>.... |
| iPS:43 5303 | 21-225_146A6 | VK1\|L5/J K3 | ........A...<br>........... | ...........<br>....N... | ........<br>....... | ........<br>......G | ..................<br>..S............... | ..TD...............<br>............... | ......<br>.... |
| iPS:43 5335 | 21-225_147D10 | VK1\|L5/J K3 | ........A...<br>........... | ......N....<br>....N..T | ........<br>....... | ........<br>....... | ..................<br>..S............... | ..TD...............<br>............... | ......<br>..V. |
| iPS:43 5339 | 21-225_147D12 | VK1\|L5/J K3 | ........A...<br>........... | ...........<br>....N... | ...E....<br>....... | ........<br>....... | ..................<br>..S............... | ..TD...............<br>............... | ......<br>..V. |
| iPS:43 5343 | 21-225_148E2 | VK1\|L5/J K3 | ........A...<br>........... | ...........<br>....N... | ........<br>....... | ........<br>....... | ........NE........<br>..S............... | ..TD...............<br>............... | ......<br>..V. |
| iPS:43 5379 | 21-225_149B6 | VK1\|L5/J K3 | ............<br>........... | ........I..<br>........ | ........<br>....... | ........<br>....... | ..................<br>.................. | ..G................<br>............... | ......<br>.... |
| iPS:43 5381 | 21-225_149C6 | VK1\|L5/J K3 | ........A...<br>........... | ...........<br>....N... | .......R<br>....... | ........<br>......G | .................Y<br>..S............... | ..TD...............<br>............... | ......<br>.... |
| iPS:43 5391 | 21-225_149F8 | VK1\|L5/J K3 | ........A...<br>........... | ...........<br>....N... | ........<br>....... | ........<br>....... | ........NE........<br>..S..........I.... | ..TD...............<br>............... | ......<br>..V. |
| iPS:43 5395 | 21-225_149D11 | VK1\|L5/J K3 | ........A...<br>........... | ......N....<br>....N..T | ........<br>....... | ........<br>....... | ..................<br>..S............... | ..TD...............<br>............... | ......<br>..V. |
| iPS:43 5403 | 21-225_150C5 | VK1\|L5/J K3 | ........G...<br>........... | ........N..<br>....N... | ........<br>....... | ........<br>......G | ..................<br>..S............... | ..TD...............<br>............... | ......<br>.... |
| iPS:43 5447 | 21-225_152H7 | VK1\|L5/J K3 | ........A...<br>........... | ......D....<br>....N... | ........<br>....... | ........<br>......G | ..................<br>..S..T............ | ..TD...............<br>............... | ......<br>.... |
| iPS:43 5453 | 21-225_152G10 | VK1\|L5/J K3 | ........A...<br>........... | ...........<br>....N... | ........<br>......S | ........<br>....... | ........NE........<br>..S............... | ..TD...............<br>............... | ......<br>..V. |

| | | | | | | | | | |
|---|---|---|---|---|---|---|---|---|---|
| iPS:43 5483 | 21-225_155A4 | VK1\|L5/J K3 | ........A...<br>........... | ............<br>....N... | ..H.....<br>....... | ........<br>......G | .................<br>..S.............. | H.TD...............<br>............... | ......<br>.... |
| iPS:43 5485 | 21-225_155B4 | VK1\|L5/J K3 | ........A...<br>........... | ......D.....<br>....N... | ........<br>....... | ........<br>......G | ......N..........<br>..S.............. | H.TD...............<br>............... | ......<br>.... |
| iPS:43 5787 | 21-225_180A3 | VK1\|L5/J K3 | ............<br>........... | ......D.T...<br>........ | ........<br>....... | ........<br>....... | .................<br>I............F.H. | ...................<br>..........I.... | ......<br>...N |
| iPS:43 5809 | 21-225_182H5 | VK1\|L5/J K3 | ...........Y<br>........... | ......D.T...<br>........ | ........<br>....... | ........<br>....... | .................<br>I...T.V..D....... | ..V................<br>............... | ..H...<br>.... |
| iPS:43 5889 | 21-225_186A11 | VK1\|L5/J K3 | ............<br>........... | ......D.T...<br>........ | ........<br>....... | ........<br>....... | .................<br>I...T.V..D....... | ..V................<br>............... | ..H...<br>.... |
| iPS:43 5965 | 21-225_192H2 | VK1\|L5/J K3 | ............<br>........... | ............<br>......I. | ........<br>....... | G.......<br>....... | .................<br>................. | ..S................<br>............... | ......<br>..V. |
| iPS:43 6106 | 21-225_196F2 | VK1\|L5/J K3 | ............<br>........... | ............<br>........ | ........<br>....... | ........<br>....... | .................<br>................. | ..T................<br>..........V.... | ......<br>.... |
| iPS:43 6360 | 21-225_210H11 | VK1\|L5/J K3 | ............<br>........... | ............<br>....I... | ........<br>..N.... | ........<br>....... | ........R........<br>................. | ...K...............<br>............... | ......<br>.... |
| iPS:43 6488 | 21-225_221A6 | VK1\|L5/J K3 | ............<br>........... | ............<br>........ | ........<br>....... | T.......<br>...N... | .................<br>................. | ...................<br>............... | ......<br>.... |
| iPS:43 6494 | 21-225_221F12 | VK1\|L5/J K3 | ............<br>........... | ............<br>........ | ........<br>....... | T.......<br>...N... | .................<br>........R........ | ...................<br>............... | ......<br>.... |
| iPS:43 6496 | 21-225_222E1 | VK1\|L5/J K3 | ............<br>........... | ............<br>........ | ........<br>....... | T.......<br>...N... | .................<br>................. | ...................<br>............... | ..R...<br>.... |
| iPS:43 6508 | 21-225_222F7 | VK1\|L5/J K3 | ............<br>........... | ............<br>........ | ........<br>....... | T.......<br>...N... | .................<br>......V.......... | ...................<br>............... | ......<br>.... |
| iPS:43 6516 | 21-225_222C12 | VK1\|L5/J K3 | ......C.....<br>........... | .V..........<br>........ | ........<br>.L..V.. | T.......<br>...N... | .................<br>......V.R........ | ..D................<br>............... | ..R...<br>.... |
| iPS:43 7234 | 21-225_64E2 | VK1\|L5/J K3 | ............<br>........... | ............<br>....R... | ........<br>....... | ........<br>....... | .................<br>................. | ...................<br>............... | ......<br>.... |
| iPS:39 2996 | 21-225_28B1 | VK1\|L5/J K3 | ............<br>........... | ......A.N...<br>....D... | ........<br>....... | ........<br>....F.. | .................<br>....T............ | ...S...............<br>............... | ......<br>.... |
| iPS:39 3010 | 21-225_25E11 | VK1\|L5/J K3 | ............<br>........... | ............<br>....N... | ........<br>....... | T.......<br>......G | .................<br>.IS.............. | ...................<br>............I.. | ......<br>.... |
| iPS:39 3016 | 21-225_28F11 | VK1\|L5/J K3 | ............<br>........... | ............<br>....N... | ........<br>......S | ........<br>...N... | ......R..........<br>....T..........C. | ...................<br>..........L.... | ......<br>.... |
| iPS:39 3024 | 21-225_31H9 | VK1\|L5/J K3 | ............<br>........... | ........T...<br>.......T | ....R...<br>....... | D.......<br>.T..... | .................<br>IF............... | ..G................<br>............... | ..Q...<br>.... |
| iPS:39 3080 | 21-225_34F3 | VK1\|L5/J K3 | ............<br>.T......S.. | ............<br>....K... | ........<br>....... | ........<br>....... | .................<br>...........S..... | ...................<br>............... | ......<br>.... |
| iPS:39 3084 | 21-225_35C6 | VK1\|L5/J K3 | ............<br>........... | ............<br>....K... | ........<br>...P... | ........<br>....... | ...T.............<br>.............I... | ...................<br>............... | ......<br>.... |
| iPS:39 3086 | 21-225_36H5 | VK1\|L5/J K3 | ............<br>........... | ............<br>....R... | ........<br>..E.... | ........<br>...R... | .I...............<br>................. | ...................<br>............... | ......<br>.... |

| | | | | | | | | | |
|---|---|---|---|---|---|---|---|---|---|
| iPS:39 3098 | 21-225_35G6 | VK1\|L5/J K3 | ............<br>......L.... | ...........<br>....R... | .....V..<br>V...... | ........<br>...R... | ...............A.<br>....G.......... | ..................<br>.............. | ......<br>..L. |
| iPS:39 3112 | 21-225_33G1 | VK1\|L5/J K3 | ............<br>V.......... | ...........<br>....R... | ........<br>....... | G.......<br>..Y.... | ................<br>................ | ..................<br>.............. | ......<br>.... |
| iPS:39 3116 | 21-225_34G7 | VK1\|L5/J K3 | ............<br>........... | ......L....<br>....K... | ........<br>....... | ........<br>....... | ...L............<br>................ | ..................<br>.............. | ......<br>.... |
| iPS:39 3132 | 21-225_33H7 | VK1\|L5/J K3 | ............<br>........... | ...........<br>....R... | .....V..<br>V...... | ........<br>...R... | ................<br>....G.......... | ....I.............<br>.............. | ......<br>..L. |
| iPS:39 3140 | 21-225_35H12 | VK1\|L5/J K3 | ............<br>........... | ...........<br>....R... | ........<br>..E.... | ........<br>...R... | .I..............<br>................ | ..................<br>.............. | ......<br>.... |
| iPS:39 3954 | 21-225_4H6 | VK1\|L5/J K3 | ............<br>........... | ...........<br>....R... | ........<br>....... | G.......<br>....... | ................<br>.............I... | ..................<br>.............. | ......<br>.... |
| iPS:39 8502 | 21-225_23B11 | VK1\|L5/J K3 | ............<br>........... | ........T..<br>....K... | ........<br>...V... | ........<br>....... | R........R.......<br>................ | ..................<br>.............. | ......<br>.... |
| iPS:39 8520 | 21-225_31C4 | VK1\|L5/J K3 | ............<br>........... | ...........<br>....K... | ........<br>...P... | ........<br>....... | ...T............<br>.............I... | ..................<br>.............. | ......<br>.... |
| iPS:40 2223 | 21-225_30A11 | VK1\|L5/J K3 | ............<br>........... | ...........<br>....R... | .......R<br>..E.... | ........<br>...R... | .I..............<br>................ | ..................<br>.............. | ......<br>..N. |
| | | Germline | K_FR1 | K_CDR1 | K_FR2 | K_CDR2 | K_FR3 | K_CDR3 | K_FR4 |
| | VK4\|B3/JK1 | | DIVMTQSPDSLA VSLGERATINC | KSS--QSVLYSSNNKNYLA | WYQQKPGQPPKLLIY | W-------ASTRES | GVPDRFSGSGSG--TDFTLTISSLQAEDVAVYYC | QQYYS-----------------------TPWT | FGQGTKVEIK |
| iPS:42 6112 | 21-225_12F12 | VK4\|B3/J K1 | ............<br>........... | ......T..F.<br>...NH... | ........<br>..N.... | ........<br>.....A. | ................<br>................ | ..................<br>..........S... | ......<br>.... |
| iPS:45 1137 | 21-225_74A7 | VK4\|B3/J K1 | ............<br>........... | .........F.<br>...Y.... | ....R...<br>..N.... | ........<br>....... | ................<br>.........A...... | ...H..............<br>..........S.P. | .....T<br>.Q.. |
| iPS:43 3909 | 21-225_43D8 | VK4\|B3/J K1 | ............<br>........... | .........MT<br>..D....T | ....R...<br>....... | ........<br>....... | ........G.......<br>.....G.......... | ..................<br>............P. | ......<br>.... |
| iPS:43 4177 | 21-225_56A1 | VK4\|B3/J K1 | ............<br>........... | .........H.<br>.......V | ....R...<br>..N.... | ........<br>....... | ................<br>................ | ..................<br>............P. | ......<br>.... |
| iPS:43 4237 | 21-225_61B5 | VK4\|B3/J K1 | ............<br>........... | ...........<br>...N.S.T | ...L....<br>...K... | ........<br>....... | ................<br>................ | ..................<br>............P. | ....S.<br>.... |
| iPS:43 4285 | 21-225_57A11 | VK4\|B3/J K1 | ...........T<br>........... | .........H.<br>...Y.... | ....R...<br>....V.. | ........<br>.....A. | ................<br>...........M.... | ..................<br>.............. | ......<br>..F. |
| iPS:43 4295 | 21-225_58B9 | VK4\|B3/J K1 | ............<br>........... | ..G....I...<br>...N.... | ........<br>...K... | ........<br>.....D. | ...A............<br>................ | ..................<br>............P. | ....S.<br>.... |
| iPS:43 4321 | 21-225_59F10 | VK4\|B3/J K1 | ......F.....<br>........... | ......T...R<br>...Y.... | ........<br>....... | ........<br>....... | ................<br>................ | ...F..............<br>............P. | ......<br>.... |
| iPS:43 4431 | 21-225_70E7 | VK4\|B3/J K1 | ............<br>........L.. | ...........<br>...N.... | ........<br>....... | ........<br>....... | ................<br>................ | ....N.............<br>..........I.P. | ......<br>.... |
| iPS:43 4475 | 21-225_74F9 | VK4\|B3/J K1 | ............<br>........... | ...........<br>...Y.... | ........<br>...K... | ........<br>....... | ................<br>..............S. | ..................<br>..........S.P. | ......<br>.... |

| | | | | | | | | | |
|---|---|---|---|---|---|---|---|---|---|
| iPS:43 4477 | 21-225_74A6 | VK4\|B3/J K1 | ............<br>..P........ | .........H.<br>...N.... | ........<br>..D.... | ........<br>....... | .................<br>................ | ...F..............<br>.............. | .....Q<br>.... |
| iPS:43 4481 | 21-225_74B10 | VK4\|B3/J K1 | ............<br>........... | .........H.<br>.......T | ........<br>....... | ........<br>....... | .................<br>S..G............ | ..................<br>..........I.P. | ......<br>.... |
| iPS:43 4487 | 21-225_76G2 | VK4\|B3/J K1 | ............<br>........... | .........H.<br>...Y.... | ....R...<br>..R.... | ........<br>....... | .................<br>........V....... | ..................<br>..........S.P. | ......<br>.... |
| iPS:43 4493 | 21-225_76F3 | VK4\|B3/J K1 | ......C...P.<br>........... | .........F.<br>...Y.... | ....R...<br>.HD.... | ........<br>....... | .................<br>.........A...... | ...H..............<br>..........S.L. | .....T<br>.Q.. |
| iPS:43 4509 | 21-225_76F5 | VK4\|B3/J K1 | V..L........<br>........... | .........H.<br>..SY.... | ........<br>S..V... | ........<br>.T..... | .................<br>................ | ..................<br>..........S.P. | ......<br>.... |
| iPS:43 4525 | 21-225_76E8 | VK4\|B3/J K1 | ............<br>........... | ......T..H.<br>...Y.... | ........<br>...V... | ........<br>.T..... | .................<br>................ | ...F..............<br>..........S.L. | ......<br>.... |
| iPS:43 4549 | 21-225_76E11 | VK4\|B3/J K1 | ............<br>.....G..... | ..R......H.<br>...Y.... | .....A..<br>....... | ........<br>....... | .................<br>................ | ..................<br>............P. | ......<br>...R |
| iPS:43 4551 | 21-225_75C4 | VK4\|B3/J K1 | ............<br>........... | ........I...<br>...N.... | .F......<br>....... | ........<br>....... | .................<br>................ | ....I.............<br>............P. | ......<br>.... |
| iPS:43 4575 | 21-225_77C7 | VK4\|B3/J K1 | ............<br>........... | ......T..H.<br>...Y.... | ........<br>...V.L. | ........<br>.T..... | .................<br>................ | ...F..............<br>..........S.P. | .....R<br>.... |
| iPS:43 4597 | 21-225_77C10 | VK4\|B3/J K1 | .........C..<br>........... | ..........T<br>...N.... | ........<br>....... | ........<br>....... | .................<br>................ | .H..N.............<br>.............K | .V....<br>...T |
| iPS:43 4617 | 21-225_74B8 | VK4\|B3/J K1 | .S..........<br>........... | .........H.<br>..K..... | ........<br>....... | ........<br>....... | .................<br>................ | ....R.............<br>.............. | ......<br>.... |
| iPS:43 4619 | 21-225_78C1 | VK4\|B3/J K1 | .........C..<br>........... | ..........T<br>...N.... | ........<br>....... | ........<br>....... | .................<br>................ | .H..N.............<br>.............K | .V....<br>.... |
| iPS:43 4639 | 21-225_74B7 | VK4\|B3/J K1 | ............<br>........... | ......T..H.<br>...Y.... | ........<br>...V... | ........<br>.T..... | .................<br>................ | ...F..............<br>..........S.P. | ......<br>.... |
| iPS:43 4649 | 21-225_78E11 | VK4\|B3/J K1 | ............<br>........... | ...........<br>F..Y.... | ........<br>...K... | ........<br>....... | .................<br>..............S. | ..................<br>..........S.P. | ......<br>.... |
| iPS:43 4653 | 21-225_74B5 | VK4\|B3/J K1 | ............<br>........... | .........F.<br>...Y.... | ....R...<br>..N.... | ........<br>....... | .................<br>.........A...... | ..................<br>..........S.P. | .....T<br>.Q.. |
| iPS:43 4655 | 21-225_78H12 | VK4\|B3/J K1 | ............<br>........... | ......T..H.<br>F..Y.... | ........<br>...V... | ........<br>.T..... | .................<br>................ | ...F..............<br>..........S.P. | ......<br>.... |
| iPS:43 4665 | 21-225_74G4 | VK4\|B3/J K1 | ............<br>........... | ...........<br>...N.... | .....A..<br>....... | ........<br>....... | .................<br>................ | ..................<br>..........I.P. | ......<br>.... |
| iPS:43 4675 | 21-225_79G6 | VK4\|B3/J K1 | .........C..<br>.........S. | M........H.<br>F......T | ........<br>....... | ........<br>....W.. | .................<br>S.P.G........... | ..................<br>..........I.P. | ......<br>.... |
| iPS:43 4685 | 21-225_79E9 | VK4\|B3/J K1 | ............<br>........... | ........I...<br>...N.... | .F......<br>....... | ........<br>....... | ..........C......<br>................ | ....I.............<br>............P. | ......<br>.... |
| iPS:43 4689 | 21-225_79G10 | VK4\|B3/J K1 | ............<br>........... | .........F.<br>...Y.... | ....R...<br>.HN.... | ........<br>....... | .................<br>.........A...... | ...H..............<br>..........S.L. | .....T<br>.Q.. |
| iPS:43 4697 | 21-225_79F12 | VK4\|B3/J K1 | ............<br>........... | ........I...<br>...Y.... | ........<br>....... | ........<br>....... | .................<br>................ | ..................<br>.............. | ......<br>.... |

| | | | | | | | | | |
|---|---|---|---|---|---|---|---|---|---|
| iPS:43<br>4707 | 21-225_80D3 | VK4\|B3/J<br>K1 | .........C..<br>........... | ..........T<br>...N.... | ........<br>....... | ........<br>....... | ..................<br>.................. | .H.NE.............<br>..............GK | .V.V..<br>...T |
| iPS:43<br>4711 | 21-225_80H3 | VK4\|B3/J<br>K1 | ............<br>........T.. | .........HR<br>...Y.... | ........<br>....... | ........<br>....... | ..................<br>.................. | ..................<br>..............P. | ......<br>.... |
| iPS:43<br>4731 | 21-225_80E9 | VK4\|B3/J<br>K1 | .........C..<br>........... | ..........T<br>...N.... | ........<br>....... | ........<br>....... | ..................<br>.................. | ....N.............<br>.............. | .V....<br>.... |
| iPS:43<br>4761 | 21-225_81E5 | VK4\|B3/J<br>K1 | ............<br>........... | .........F.<br>...Y.... | ....R...<br>..N.... | ........<br>....... | ..................<br>.........A....... | ..................<br>..........S.L. | .....T<br>.Q.. |
| iPS:43<br>4771 | 21-225_81F9 | VK4\|B3/J<br>K1 | .........C..<br>........... | ..........T<br>...N.... | ........<br>....... | ........<br>....... | ..................<br>................R | .H.ND.............<br>.............GK | .V..IM<br>...T |
| iPS:43<br>4827 | 21-225_83F3 | VK4\|B3/J<br>K1 | .........C..<br>........... | ..........T<br>...N.... | ........<br>....... | ........<br>....... | ..................<br>.................. | .H.ND.............<br>..............K | .V..I.<br>.... |
| iPS:43<br>4829 | 21-225_83G3 | VK4\|B3/J<br>K1 | .........C..<br>........... | ..........T<br>...N.... | ........<br>....... | ........<br>....... | ..................<br>.................. | .H..N.............<br>.............. | .V....<br>.... |
| iPS:43<br>4841 | 21-225_83G7 | VK4\|B3/J<br>K1 | ............<br>.........I. | ......T..H.<br>...Y.... | ........<br>...V... | ........<br>.T..... | ..................<br>.................. | ...F..............<br>..........S.L. | ......<br>.... |
| iPS:43<br>4863 | 21-225_84G7 | VK4\|B3/J<br>K1 | ..........P.<br>.........I. | ......T..H.<br>...Y.... | ........<br>...V... | ........<br>.T..... | ..................<br>.................. | ...F..............<br>..........S.P. | ......<br>.... |
| iPS:43<br>4877 | 21-225_85H2 | VK4\|B3/J<br>K1 | .SM.........<br>........... | .........H.<br>..K.... | ........<br>....... | ........<br>....... | ..................<br>.................. | ....R.............<br>.............. | ......<br>.... |
| iPS:43<br>4901 | 21-225_85H9 | VK4\|B3/J<br>K1 | ............<br>........T.. | .........HR<br>...Y.... | ........<br>....... | ........<br>....... | ..................<br>.................. | ..................<br>..............P. | ......<br>.... |
| iPS:43<br>4935 | 21-225_86E9 | VK4\|B3/J<br>K1 | ......C...P.<br>........... | .........HR<br>...Y.... | ........<br>..N.... | ........<br>....... | ..................<br>.........A....... | ...H..............<br>..........S.L. | .....T<br>.... |
| iPS:43<br>4965 | 21-225_88A1 | VK4\|B3/J<br>K1 | N...........<br>....A...... | .........H.<br>...Y...T | ........<br>.....L. | ........<br>.....K. | ..................<br>...............F. | ..................<br>..........S.P. | ......<br>.... |
| iPS:43<br>4971 | 21-225_88G2 | VK4\|B3/J<br>K1 | .........C..<br>........... | ..........T<br>...N.... | ........<br>....... | ........<br>....... | ..................<br>.................. | ....N.............<br>.............. | .V....<br>.... |
| iPS:43<br>4973 | 21-225_88B4 | VK4\|B3/J<br>K1 | ............<br>........... | ..........I<br>...N.... | ........<br>....... | ........<br>....... | ...A..............<br>.................. | ..................<br>..............P. | ......<br>.... |
| iPS:43<br>4997 | 21-<br>225_88C10 | VK4\|B3/J<br>K1 | ............<br>........... | .........H.<br>...W.... | .H......<br>......H | ........<br>..F..K. | ........G......N.<br>.................. | ....R.............<br>..........A.P. | ......<br>.... |
| iPS:43<br>5051 | 21-225_90D9 | VK4\|B3/J<br>K1 | ............<br>.........I. | ......T..H.<br>...Y.... | ........<br>...V... | ........<br>.T..... | ..................<br>.................. | ...L..............<br>..........S.L. | ......<br>.... |
| iPS:43<br>5053 | 21-225_75F9 | VK4\|B3/J<br>K1 | ...........P<br>........V.. | .........HN<br>...N.... | ........<br>....... | ........<br>....... | ..................<br>.................. | ..................<br>..........S.P. | ......<br>.... |
| iPS:43<br>5071 | 21-225_91F1 | VK4\|B3/J<br>K1 | .........C..<br>........... | ..........T<br>...N.... | ........<br>....... | ........<br>....... | ..................<br>.................. | .H..N.............<br>..............K | .V....<br>.... |
| iPS:43<br>5087 | 21-225_91G8 | VK4\|B3/J<br>K1 | .........C..<br>........... | ..........T<br>...N.... | ........<br>....... | ........<br>....... | ..................<br>.................. | ....T.............<br>.............. | ......<br>.... |
| iPS:43<br>5113 | 21-225_92E6 | VK4\|B3/J<br>K1 | ............<br>A.......... | ......NI.S.<br>.......T | ........<br>...I... | ........<br>.T..... | ..........F.......<br>.................. | ...F..............<br>..........V.P. | ......<br>.... |

| | | | | | | | | | |
|---|---|---|---|---|---|---|---|---|---|
| iPS:43 5167 | 21-225_92F12 | VK4\|B3/JK1 | ............<br>........T.. | ..........HR<br>...Y.... | ........<br>....... | ........<br>....... | ...................<br>.................. | ....................<br>.............P. | ......<br>.... |
| iPS:43 5203 | 21-225_75A7 | VK4\|B3/JK1 | ............<br>........... | ......T..H.<br>...Y.... | ........<br>...V... | ........<br>.T..... | ...................<br>.................. | ...F................<br>..........S.P. | ......<br>.... |
| iPS:43 5209 | 21-225_75A10 | VK4\|B3/JK1 | N...........<br>....A...... | ..........HN<br>...Y...T | ........<br>.....L. | ........<br>.....K. | ...................<br>..............F. | ....................<br>..........S.P. | ......<br>.... |
| iPS:43 5211 | 21-225_94E11 | VK4\|B3/JK1 | ............<br>........... | ..........F.<br>...Y.... | ....R...<br>..N.... | ........<br>....... | ...................<br>........A....... | ...H................<br>..........S.L. | .....T<br>.Q.. |
| iPS:43 5215 | 21-225_94E12 | VK4\|B3/JK1 | ............<br>........T.. | ..........HR<br>...Y.... | ........<br>....... | ........<br>....... | ...................<br>.................. | ....................<br>.............P. | ......<br>.... |
| iPS:43 5227 | 21-225_95G4 | VK4\|B3/JK1 | ......C.....<br>........... | ..........FR<br>...Y.... | ....R...<br>.HN.... | ........<br>....... | ..........Y......<br>.....V..A....... | ...H................<br>..........S.L. | .....T<br>.Q.. |
| iPS:43 5245 | 21-225_95E12 | VK4\|B3/JK1 | ............<br>........... | ...........<br>...A.... | ........<br>..N.F.. | ........<br>....... | ...................<br>.................. | ....................<br>............CS | ......<br>.... |
| iPS:43 5249 | 21-225_96E2 | VK4\|B3/JK1 | .S..........<br>........... | ..........H.<br>..K..... | ........<br>....... | ........<br>....W.. | ...................<br>.................. | ....R...............<br>.............. | ......<br>.... |
| iPS:43 5255 | 21-225_96D5 | VK4\|B3/JK1 | ............<br>........... | ..........H.<br>...Y.... | ........<br>..N.... | ........<br>....... | ...................<br>........A....... | ....................<br>..........S.P. | .....T<br>.... |
| iPS:43 5257 | 21-225_96H5 | VK4\|B3/JK1 | ............<br>........... | ...........<br>..SH.... | ........<br>....... | ........<br>...I... | ...................<br>..S...M.......... | ....................<br>............CS | ......<br>.... |
| iPS:43 5267 | 21-225_96D10 | VK4\|B3/JK1 | ............<br>........... | ......NI.S.<br>.......T | ........<br>...I... | ........<br>.T..... | ..........F......<br>.................. | ...F................<br>..........V.P. | ......<br>.... |
| iPS:43 5279 | 21-225_97H4 | VK4\|B3/JK1 | .........C..<br>........... | ...........T<br>...N.... | ........<br>....... | ........<br>....... | ...................<br>.................. | .H.ND...............<br>.............K | .V....<br>...T |
| iPS:43 5321 | 21-225_147E4 | VK4\|B3/JK1 | ............<br>........... | ..........H.<br>...Y.... | ...L..R.<br>....... | ........<br>....... | ........R........<br>.............I... | ....................<br>.............S. | ..P...<br>.... |
| iPS:43 5353 | 21-225_148F8 | VK4\|B3/JK1 | .......L....<br>........... | ........A.H.<br>...Y.... | ........<br>....... | ........<br>.....K. | ...................<br>.................. | ....................<br>..........I.P. | ......<br>.... |
| iPS:43 5369 | 21-225_149A2 | VK4\|B3/JK1 | ............<br>........... | ...........<br>P..N.... | ........<br>....... | ........<br>....... | ...................<br>.................. | ....................<br>............CS | ......<br>.... |
| iPS:43 5373 | 21-225_149E3 | VK4\|B3/JK1 | ............<br>.........S. | ......T..HN<br>...H..F. | ........<br>....... | ........<br>....LR. | ...................<br>.................. | ....................<br>.............P. | ......<br>.... |
| iPS:43 5375 | 21-225_149H4 | VK4\|B3/JK1 | ............<br>........... | ..........S.<br>..DN.... | .......R<br>....... | ........<br>.S..... | ...................<br>.................. | H...................<br>..........Y.P. | ......<br>.... |
| iPS:43 5481 | 21-225_154A11 | VK4\|B3/JK1 | ............<br>........... | R.........H.<br>...Y.... | ........<br>....... | ........<br>...K.D. | ...................<br>.................. | ...F................<br>..........S.R. | ......<br>.... |
| iPS:43 5557 | 21-225_158B12 | VK4\|B3/JK1 | ..........P.<br>........... | ......N..H.<br>...N...T | ....R...<br>....... | ........<br>....... | ...............E.<br>.................. | ....................<br>.............P. | ......<br>.... |
| iPS:43 5627 | 21-225_162F6 | VK4\|B3/JK1 | ............<br>........... | ......N..H.<br>...N...T | ....R...<br>....... | ........<br>....... | ...............E.<br>.................. | ....................<br>.............P. | ......<br>.... |
| iPS:43 5701 | 21-225_170F6 | VK4\|B3/JK1 | ............<br>....A...... | ..........H.<br>...Y.... | ....R...<br>...V..H | ........<br>.....K. | .........V.......<br>....N.......... | ....................<br>.............. | ......<br>.... |

| | | | | | | | | | |
|---|---|---|---|---|---|---|---|---|---|
| iPS:43 5737 | 21-225_174G5 | VK4\|B3/JK1 | ............<br>....A...... | .........H.<br>...Y...T | .....S..<br>....... | ........<br>....... | ..................<br>................. | ....R.............<br>............... | ......<br>.... |
| iPS:43 5751 | 21-225_175D10 | VK4\|B3/JK1 | ............<br>........... | ...........<br>...N.... | ........<br>..N.... | ........<br>.T..... | ...............N.<br>................. | ..................<br>............P. | ......<br>.... |
| iPS:43 5773 | 21-225_177B12 | VK4\|B3/JK1 | ............<br>........V.. | .........H.<br>...N...T | ........<br>....... | ........<br>....... | ..................<br>................. | ..................<br>..........S.P. | ......<br>.... |
| iPS:43 5801 | 21-225_181E5 | VK4\|B3/JK1 | ............<br>........... | .........H.<br>...Y...T | ........<br>....... | ........<br>....... | ..................<br>................. | H..FI.............<br>............... | ......<br>.... |
| iPS:43 5841 | 21-225_191D8 | VK4\|B3/JK1 | ..M.........<br>.......I.S. | R........H.<br>...Y.... | .......H<br>....... | ........<br>....... | ..................<br>................. | ..................<br>............P. | ..L...<br>.... |
| iPS:43 5925 | 21-225_190D7 | VK4\|B3/JK1 | ............<br>........... | .........S.<br>...Y...V | ........<br>....... | ........<br>.....K. | ..................<br>.........D....... | ....R.............<br>............... | ......<br>.... |
| iPS:43 6021 | 21-225_193G4 | VK4\|B3/JK1 | ............<br>........... | ...........<br>...Y...T | ........<br>....... | ........<br>.....K. | ..................<br>.........D....... | ....I.............<br>............... | ......<br>.D.. |
| iPS:43 6114 | 21-225_196G8 | VK4\|B3/JK1 | ...........T<br>........V.. | .........H.<br>...N.... | ........<br>....... | ........<br>....... | ..................<br>..A.............. | ....N.............<br>............P. | ......<br>.... |
| iPS:43 6150 | 21-225_197H4 | VK4\|B3/JK1 | ..M....S...T<br>.......I.S. | R........H.<br>F..Y.... | .....A.H<br>..N.... | ........<br>....... | ..................<br>..P.............. | ..................<br>............P. | ..L...<br>.... |
| iPS:43 6154 | 21-225_197C6 | VK4\|B3/JK1 | ..M.........<br>.......I.S. | R........H.<br>...Y.... | .......H<br>..N.... | ........<br>....... | ..................<br>................. | ..................<br>............P. | ..L...<br>.... |
| iPS:43 6218 | 21-225_200G7 | VK4\|B3/JK1 | ..........PT<br>A.......VK. | .........H.<br>...N.... | ........<br>....... | ........<br>....... | ..................<br>..A.............. | ....N.............<br>............P. | ......<br>.... |
| iPS:43 6272 | 21-225_201F5 | VK4\|B3/JK1 | ........E...<br>........... | ...........<br>.......V | ........<br>....... | ........<br>....... | ..................<br>................. | ..................<br>............P. | ......<br>.... |
| iPS:43 6400 | 21-225_213H7 | VK4\|B3/JK1 | ............<br>........... | ......N..DI<br>.....S.G | .F......<br>......N | ........<br>....... | ..................<br>........T.....H. | ....N.............<br>..........I.P. | ..R...<br>.... |
| iPS:43 6402 | 21-225_213H12 | VK4\|B3/JK1 | ............<br>.........T. | ......N..KT<br>...R.... | ........<br>...V... | ........<br>....... | ..................<br>..I.............. | H.................<br>..........I... | ......<br>.... |
| iPS:43 6500 | 21-225_222H3 | VK4\|B3/JK1 | ............<br>........... | .........K.<br>..HR.... | ........<br>..Q.... | ........<br>......T | ..................<br>............S..S. | ...S..............<br>..........I... | ......<br>...N |
| iPS:43 6520 | 21-225_223G10 | VK4\|B3/JK1 | ............<br>........... | .......I.L.<br>........ | .H......<br>....... | ........<br>....... | ..................<br>................. | L..F..............<br>............... | ......<br>.... |
| iPS:43 6544 | 21-225_224H5 | VK4\|B3/JK1 | ............<br>........... | ...........<br>...F...T | ........<br>....... | ........<br>....... | ..................<br>....N............ | ..................<br>............P. | ......<br>.... |
| iPS:43 6550 | 21-225_224D8 | VK4\|B3/JK1 | ............<br>.......A... | ...........<br>........ | ........<br>....... | ........<br>.S...K. | ..................<br>................. | ...F..............<br>............P. | ......<br>.... |
| iPS:43 6574 | 21-225_225F5 | VK4\|B3/JK1 | ............<br>........... | ......N...N<br>...N.... | ........<br>..N.... | ........<br>....... | ..................<br>...............F. | ..................<br>..........S.P. | ......<br>...N |
| iPS:43 6586 | 21-225_225F11 | VK4\|B3/JK1 | ............<br>........... | ...........<br>...Y.... | ....R...<br>....... | ........<br>....... | ..............P..<br>................. | ....T.............<br>............P. | ......<br>.... |
| iPS:43 6600 | 21-225_226F6 | VK4\|B3/JK1 | ............<br>........... | .......I...<br>...Y.... | ........<br>....... | ........<br>....... | ..............P..<br>................. | ....T.............<br>............P. | ......<br>.... |

| | | | | | | | | | |
|---|---|---|---|---|---|---|---|---|---|
| iPS:436616 | 21-225_226D11 | VK4\|B3/JK1 | ............<br>........... | ......N..H.<br>..SN...V | ........<br>....... | ........<br>....... | ......R..........<br>................. | ....K.............<br>.............. | ......<br>.... |
| iPS:436622 | 21-225_226A12 | VK4\|B3/JK1 | ............<br>........... | ......N....<br>...N.... | ....T...<br>.....F. | ........<br>.....K. | .................<br>................. | ..................<br>..........S.P. | ......<br>.... |
| iPS:436638 | 21-225_227C7 | VK4\|B3/JK1 | ............<br>........... | R.....I..SD<br>...N.... | ........<br>..N.... | ........<br>....... | .................<br>...............F. | ..................<br>..........S.P. | ......<br>.... |
| iPS:437356 | 21-225_74B1 | VK4\|B3/JK1 | .........F..<br>........T.. | .........HR<br>...Y.... | ........<br>....... | ........<br>....... | .................<br>................. | ..................<br>............P. | ......<br>.... |
| iPS:437361 | 21-225_74C1 | VK4\|B3/JK1 | ......C...P.<br>.......S... | .......I.H.<br>...Y.... | .......H<br>.H..... | ........<br>....... | ..........Y......<br>................. | ..................<br>.............. | ......<br>.... |
| iPS:437379 | 21-225_74H2 | VK4\|B3/JK1 | ............<br>........... | .........H.<br>.......T | ........<br>.H..... | ........<br>....... | .................<br>S...G............ | ..................<br>..........I.P. | ......<br>.... |
| iPS:446094 | 21-225_77E1 | VK4\|B3/JK1 | ............<br>........... | ......T..H.<br>...Y.... | ........<br>...V... | ........<br>.T..... | ..H..............<br>................. | H..L..............<br>..........S.L. | ......<br>.... |
| iPS:451116 | 21-225_164A4 | VK4\|B3/JK1 | ......Y...R.<br>.........K. | ...........<br>.......T | ........<br>....F.. | ........<br>....... | ..........C......<br>......V.......... | ...F..............<br>.............. | ......<br>.... |
| iPS:451124 | 21-225_74F6 | VK4\|B3/JK1 | ............<br>........... | ......NI.S.<br>.......T | ........<br>...I... | ........<br>.T..... | ..........F......<br>................. | ...F..............<br>..........V.L. | ......<br>.... |
| iPS:451127 | 21-225_164A7 | VK4\|B3/JK1 | ......C.....<br>........... | .........H.<br>...N.... | ........<br>.H..... | ........<br>.T..... | ..........Y......<br>S...A............ | ..................<br>..........I.L. | ......<br>.... |
| iPS:451129 | 21-225_94D2 | VK4\|B3/JK1 | ............<br>........... | .........H.<br>.......T | ........<br>.H..... | ........<br>....... | .................<br>S...G...H........ | ...H..............<br>..........I.P. | ..H...<br>.... |
| iPS:451133 | 21-225_95H4 | VK4\|B3/JK1 | ......C.....<br>........... | .........F.<br>...Y.... | ....R...<br>.HN.... | ........<br>....... | .................<br>.........A....... | ...H..............<br>..........S.L. | .....T<br>.Q.. |
| iPS:392786 | 21-225_24E1 | VK4\|B3/JK1 | ............<br>........... | ..........T<br>...N...T | ........<br>R.N.... | ........<br>....... | .................<br>................. | ..F...............<br>............P. | ......<br>.... |
| iPS:392886 | 21-225_23A12 | VK4\|B3/JK1 | ............<br>........... | ...........<br>...N.... | ........<br>....... | ........<br>.T..... | .................<br>................. | ....D.............<br>............P. | ......<br>.... |
| iPS:392928 | 21-225_25A4 | VK4\|B3/JK1 | ......F.....<br>.........K. | ...........<br>.H.N.... | ........<br>.....L. | ........<br>....... | ...............E.<br>.......E......... | ..................<br>............P. | ......<br>.... |
| iPS:392960 | 21-225_29E6 | VK4\|B3/JK1 | ......F.....<br>........... | ...........<br>.H.NY..T | ........<br>.H...L. | ........<br>...S... | ...............E.<br>................. | ..................<br>............P. | ......<br>.... |
| iPS:392992 | 21-225_26C4 | VK4\|B3/JK1 | ............<br>........... | ..........R<br>...Y.... | ...H....<br>....... | ........<br>....... | .................<br>................. | ..................<br>............P. | ......<br>..F. |
| iPS:393368 | 21-225_29H8 | VK4\|B3/JK1 | ............<br>........... | R.....TI.H.<br>...Y.... | ........<br>....... | ........<br>....... | .................<br>................. | ....C.............<br>............P. | ......<br>.... |
| iPS:393942 | 21-225_11E5 | VK4\|B3/JK1 | ............<br>........L.. | ...........<br>...N...T | ........<br>R...... | ........<br>....... | ...............N.<br>................. | ..................<br>............P. | ......<br>.... |
| iPS:398506 | 21-225_23G12 | VK4\|B3/JK1 | ............<br>........... | .......I.F.<br>...N.... | ........<br>....... | ........<br>....... | .................<br>...............F. | ...S..............<br>.............. | ......<br>.... |
| | | Germline | K_FR1 | K_CDR1 | K_FR2 | K_CDR2 | K_FR3 | K_CDR3 | K_FR4 |

| | VK1\|A30/JK2 | | DIQMTQSPSSLS<br>ASVGDRVTITC | RAS--QGIR--<br>----NDLG | WYQQKPGK<br>APKRLIY | A-------<br>-ASSLQS | GVPSRFSGSGSG--<br>TEFTLTISSLQPEDFAT<br>YYC | LQHNS-----------<br>----------YPYT | FGQGTK<br>LEIK |
|---|---|---|---|---|---|---|---|---|---|
| iPS:426114 | 21-225_28H2 | VK1\|A30/JK2 | ............<br>..I........ | ...........<br>........ | ........<br>....... | ........<br>....... | ................<br>................ | ...Y...............<br>............RS | ......<br>.... |
| iPS:426116 | 21-225_29E2 | VK1\|A30/JK2 | ............<br>........... | ......A....<br>........ | ....R...<br>....... | ........<br>....... | ................<br>I............... | ...YN.............<br>............RS | ......<br>.... |
| iPS:434231 | 21-225_61F2 | VK1\|A30/JK2 | ............<br>........V.. | ...........<br>....D... | ........<br>..E.... | ........<br>....... | ................<br>................ | ...Y...............<br>............RS | ......<br>.... |
| iPS:435401 | 21-225_150E2 | VK1\|A30/JK2 | ............<br>.........S. | ........G..<br>........ | ........<br>..T.... | ........<br>....... | ................<br>.........A...... | ...Y...............<br>.........F..S | ......<br>.... |
| iPS:435445 | 21-225_152F7 | VK1\|A30/JK2 | ............<br>........... | ...........<br>........ | ........<br>....... | ........<br>.T..... | ................<br>................ | ...YN.............<br>.........F..S | ......<br>.... |
| iPS:435763 | 21-225_176H12 | VK1\|A30/JK2 | ............<br>........... | ...........<br>........ | ........<br>..N.... | ........<br>....... | ................<br>........R......G | ...................<br>............RS | ......<br>.... |
| iPS:435767 | 21-225_177B4 | VK1\|A30/JK2 | ............<br>........... | ...........<br>........ | ........<br>....... | ........<br>....... | ................<br>................ | ...Y...............<br>.........F.RS | ......<br>.... |
| iPS:392974 | 21-225_26A11 | VK1\|A30/JK2 | ........I...<br>........... | ......A....<br>........ | ....R...<br>....... | ........<br>....... | ................<br>I............... | ...YN.............<br>............RS | ......<br>...R |
| iPS:393046 | 21-225_25A12 | VK1\|A30/JK2 | ............<br>........V.. | ......A....<br>....D... | ....R...<br>....... | ........<br>....... | ................<br>I............... | ...YN.............<br>............RS | ......<br>.... |
| | | Germline | K_FR1 | K_CDR1 | K_FR2 | K_CDR2 | K_FR3 | K_CDR3 | K_FR4 |
| | VK1\|O12/JK4 | | DIQMTQSPSSLS<br>ASVGDRVTITC | RAS--QSIS--<br>----SYLN | WYQQKPGK<br>APKLLIY | A-------<br>-ASSLQS | GVPSRFSGSGSG--<br>TDFTLTISSLQPEDFAT<br>YYC | QQSYS-----------<br>----------TPLT | FGGGTK<br>VEIK |
| iPS:426118 | 21-225_7A10 | VK1\|O12/JK4 | ............<br>........... | ......N.Y..<br>........ | .......R<br>....V.. | S.......<br>.T..... | ................<br>S...N.....S... | ...................<br>.........P... | ......<br>...R |
| iPS:426124 | 21-225_32D6 | VK1\|O12/JK4 | ............<br>T.......... | ......N.I..<br>........ | ........<br>.....M. | V.......<br>...R... | ................<br>........A....... | ...................<br>............Y. | ......<br>.A.. |
| iPS:451135 | 21-225_64A11 | VK1\|O12/JK4 | ........F...<br>........... | .....R.V...<br>....R... | ....TL..<br>.L....S | V.......<br>...R... | ................<br>......V.R......F. | ...D...............<br>.........F... | ......<br>.... |
| iPS:434011 | 21-225_48B10 | VK1\|O12/JK4 | ............<br>........... | ........R..<br>....K... | ...KT...<br>....... | ........<br>....... | ................<br>......V......... | ..T................<br>.........N... | ......<br>..FT |
| iPS:434015 | 21-225_48F12 | VK1\|O12/JK4 | ............<br>........... | ........R..<br>....K... | ...KT...<br>....... | ........<br>....... | ................<br>.............N... | ..T................<br>.........N... | .....E<br>...T |
| iPS:434017 | 21-225_48G12 | VK1\|O12/JK4 | ............<br>........... | ........R..<br>....K... | ...KT...<br>....... | ........<br>....... | ................<br>......V......N... | ..T................<br>.........N... | .....E<br>...T |
| iPS:434165 | 21-225_50F2 | VK1\|O12/JK4 | ............<br>......I.... | ........L..<br>........ | ........<br>....... | V.......<br>....F.. | ................<br>.........D....... | ...................<br>.........P... | ......<br>.... |
| iPS:434191 | 21-225_56B6 | VK1\|O12/JK4 | ............<br>V.......... | ........F..<br>....R... | .......R<br>......F | ........<br>....F.. | ................<br>................ | ..T................<br>.........P... | ......<br>.... |
| iPS:43 | 21-225_62D2 | VK1\|O12/ | ............ | ........I.. | ........ | ........ | ................ | ..T................ | ...... |

| | | | | | | | | | |
|---|---|---|---|---|---|---|---|---|---|
| iPS:43 4335 | 21-225_63C10 | VK1\|O12/ JK4 | ............ T.......... | ........F.. .......H | ........ ......S | ........ ....... | .................. ..............F.. | ..T............... ..........P... | ...... .... |
| iPS:43 4341 | 21-225_64F7 | VK1\|O12/ JK4 | ............ ........... | ......N.K.. ....K... | ........ ...F... | G....... ....... | .................. .............A... | ....N............. ..........ISF. | ...... ..L. |
| iPS:43 5295 | 21-225_146H1 | VK1\|O12/ JK4 | ............ ........... | ........... ....D... | ........ ...V... | T....... .T..... | .................. .................. | ....– .................. .....STP. | ...... .... |
| iPS:43 5307 | 21-225_146E9 | VK1\|O12/ JK4 | ............ ........... | ........... ....D... | ...L.... ...V... | T....... .T..... | .................. ...............F. | ....– .................. .....STP. | ...... .... |
| iPS:43 5347 | 21-225_148C4 | VK1\|O12/ JK4 | ............ ........... | ........I.. ....N... | ........ ...V... | T....... ....... | .................. ............T.... | ....– .................. .....STP. | ...... ...E |
| iPS:43 5355 | 21-225_148H9 | VK1\|O12/ JK4 | ............ ........... | ........... ....N... | ........ ...V... | I....... ....... | .................. .................. | ....– .................. .....STP. | ...... .... |
| iPS:43 5371 | 21-225_149A3 | VK1\|O12/ JK4 | ............ ........... | ........... ........ | ........ ...VM.. | T....... ....... | .................. ....N............ | ....– .................. .....SIP. | ...... .... |
| iPS:43 5415 | 21-225_150C11 | VK1\|O12/ JK4 | ............ ........... | ........... ........ | ........ ...... | T....... ....... | .................. .................. | ....– .................. .....SIY. | ....S. .... |
| iPS:43 5419 | 21-225_150C12 | VK1\|O12/ JK4 | ............ ........... | ........... ....D... | ........ ...V... | T....... .T..... | .................. .................. | ...F– .................. .....STP. | ...... .... |
| iPS:43 5425 | 21-225_151B12 | VK1\|O12/ JK4 | ............ ........... | ........... ....NF.. | ........ ...V... | T....... .....E. | .........E....... .................. | ....– .................. .....STP. | .....R .... |
| iPS:43 5431 | 21-225_152D2 | VK1\|O12/ JK4 | .....L...... ........... | ........... ....D... | ...L.... ...V... | T....... .T..... | .................. ...............F. | ....– .................. .....STP. | ...... .... |
| iPS:43 5439 | 21-225_152G4 | VK1\|O12/ JK4 | ............ ........... | ........... ....D... | ........ ...V... | T....... .T..... | .................. ...............F. | ....– .................. .....STP. | ...... .... |
| iPS:43 5455 | 21-225_152B11 | VK1\|O12/ JK4 | ............ ........... | ........... ....D... | ........ ...V... | T....... .T..... | .................. ...............F. | ....– .................. .....STP. | ...... .... |
| iPS:43 5487 | 21-225_155C4 | VK1\|O12/ JK4 | ............ ........... | ........... ........ | ........ ...V..F | T....... ....... | .................. .................. | ....– .................. .....STP. | .....R .... |
| iPS:43 5503 | 21-225_156E4 | VK1\|O12/ JK4 | ............ ........... | ........... ........ | ........ ...V... | T....... ....... | .................. .................. | ....– .................. .....SAP. | ...... .... |

| | | | | | | | | | |
|---|---|---|---|---|---|---|---|---|---|
| iPS:43<br>5563 | 21-<br>225_159H2 | VK1\|O12/<br>JK4 | ...L........<br>........... | ...........<br>....K... | ........<br>..E.... | ........<br>.T.N... | .................<br>.....T......V.... | ..................<br>..........L.V. | ......<br>.... |
| iPS:43<br>6110 | 21-<br>225_196F4 | VK1\|O12/<br>JK4 | .F..I.......<br>........... | ......R.H..<br>........ | ........<br>....... | T.......<br>......G | .................<br>................. | ....G.............<br>..........S... | ......<br>.... |
| iPS:43<br>6244 | 21-<br>225_201H10 | VK1\|O12/<br>JK4 | ............<br>........... | .....HN.N..<br>........ | .....S..<br>....... | ........<br>....... | .................<br>...F............. | ..................<br>..........F... | ......<br>..MR |
| iPS:43<br>6262 | 21-<br>225_203E3 | VK1\|O12/<br>JK4 | ..........P.<br>..........R | .....HN.N..<br>........ | .....S..<br>....... | ........<br>....... | .................<br>...F............. | ..................<br>..........F... | ......<br>..MR |
| iPS:43<br>6276 | 21-<br>225_204H4 | VK1\|O12/<br>JK4 | .....L....P.<br>.F........R | .....HN.N..<br>........ | .....S..<br>....... | ........<br>....... | .................<br>...F............. | ..................<br>..........F... | ......<br>..MR |
| iPS:43<br>6280 | 21-<br>225_204D6 | VK1\|O12/<br>JK4 | .S..........<br>........... | .....R.VH..<br>....T... | ........<br>...V... | G.......<br>......R | .................<br>..........V...... | ..................<br>..........S... | ......<br>...Q |
| iPS:43<br>6312 | 21-<br>225_206A4 | VK1\|O12/<br>JK4 | .....L....P.<br>.F........R | .....HN.N..<br>........ | .....S..<br>......C | ........<br>....... | .................<br>...F............. | ..................<br>..........F... | ......<br>..MR |
| iPS:43<br>6316 | 21-<br>225_206A5 | VK1\|O12/<br>JK4 | ..........P.<br>..........R | .....HN.N..<br>........ | .....S..<br>......C | ........<br>....... | .................<br>...F............. | ..................<br>..........F... | ......<br>..MR |
| iPS:43<br>6338 | 21-<br>225_208E8 | VK1\|O12/<br>JK4 | ..........P.<br>.F........R | .....HN.N..<br>........ | .....S..<br>....... | ........<br>....... | .................<br>...F............. | ..................<br>..........F... | ......<br>..MR |
| iPS:43<br>6344 | 21-<br>225_208B11 | VK1\|O12/<br>JK4 | ..........P.<br>........... | .....HN.N..<br>........ | .....S..<br>....... | ........<br>....... | .................<br>...F............. | ..................<br>..........F... | ......<br>..MR |
| iPS:43<br>6358 | 21-<br>225_210D11 | VK1\|O12/<br>JK4 | ............<br>........... | .....HN.N..<br>........ | .....S..<br>....... | ........<br>....... | .................<br>...F............. | ..................<br>..........F... | ......<br>..MR |
| iPS:43<br>7282 | 21-<br>225_207C9 | VK1\|O12/<br>JK4 | .N..........<br>........... | ......RF...<br>....N... | ........<br>....... | T.......<br>....... | .......A.V.......<br>................. | ..................<br>..........I... | ......<br>.... |
| iPS:39<br>2636 | 21-225_17A6 | VK1\|O12/<br>JK4 | ............<br>........... | ......T....<br>....N... | ..H.....<br>....... | ........<br>....... | .................<br>................. | ...HT.............<br>..........S... | ......<br>.... |
| iPS:39<br>2648 | 21-<br>225_16D11 | VK1\|O12/<br>JK4 | ............<br>........... | ......T....<br>....N... | ..H.....<br>....... | ........<br>....... | .................<br>................. | ...H..............<br>..........S... | ......<br>.... |
| iPS:39<br>2664 | 21-225_20F6 | VK1\|O12/<br>JK4 | ............<br>........... | ........I..<br>....T... | ........<br>...V..H | T.......<br>....... | .................<br>................. | ..T...............<br>..........P... | ......<br>.... |
| iPS:39<br>2738 | 21-225_18G4 | VK1\|O12/<br>JK4 | ..H.........<br>........... | ........I..<br>........ | ........<br>...V... | T.......<br>......T | ....G............<br>................. | ..T...............<br>..........P... | ......<br>.... |
| iPS:39<br>2798 | 21-225_22C7 | VK1\|O12/<br>JK4 | ............<br>........... | ......N.I..<br>........ | ......E.<br>......H | I.......<br>....... | .................<br>................. | ..T...............<br>.............. | ......<br>.... |
| iPS:39<br>2922 | 21-225_30G4 | VK1\|O12/<br>JK4 | ........P...<br>T.......... | ..T...N.F..<br>........ | .H......<br>......H | T.......<br>......G | .................<br>....I.M.....S.... | .L................<br>..........P.Y. | ......<br>.... |
| iPS:39<br>3002 | 21-225_30G1 | VK1\|O12/<br>JK4 | ............<br>........... | ......N.Y..<br>........ | ........<br>D...... | ........<br>.....H. | .................<br>................. | ..................<br>.............. | ......<br>.... |
| iPS:39<br>3042 | 21-225_31F1 | VK1\|O12/<br>JK4 | ............<br>........... | ......R....<br>........ | ........<br>......F | ........<br>....S.. | .................<br>................. | ....I.............<br>.............. | .....T<br>...R |
| iPS:39<br>3066 | 21-225_34D3 | VK1\|O12/<br>JK4 | ............<br>........... | ......N.Y..<br>........ | ........<br>D...... | ........<br>.....H. | .................<br>..............F.. | ..................<br>.............. | ......<br>.... |

| | | | | | | | | | |
|---|---|---|---|---|---|---|---|---|---|
| iPS:39 3082 | 21-225_34C11 | VK1\|O12/ JK4 | ......F.....<br>T.......S.. | ......N.R..<br>....NF.. | ......E.<br>D...Q.. | G.......<br>...T... | ..........F......<br>.........K....... | ..TC..............<br>.............. | ......<br>.... |
| iPS:39 3092 | 21-225_33C12 | VK1\|O12/ JK4 | ............<br>T.......... | ........I..<br>........ | ........<br>....... | V.......<br>......G | ......N..........<br>................. | ..................<br>............Y. | ......<br>.... |
| iPS:39 3100 | 21-225_36B8 | VK1\|O12/ JK4 | ............<br>T.......... | ........I..<br>........ | ........<br>....... | V.......<br>....... | .................<br>................. | ..................<br>............Y. | ......<br>M... |
| iPS:39 3108 | 21-225_34G11 | VK1\|O12/ JK4 | ............<br>........... | ......N.N..<br>....R... | ........<br>....... | G.......<br>....... | .................<br>.....T........... | ..T.I.............<br>.............. | ......<br>.... |
| iPS:39 3122 | 21-225_33B2 | VK1\|O12/ JK4 | ............<br>T....K..... | ........I..<br>........ | ........<br>....... | V.......<br>....... | .................<br>................. | ..................<br>............Y. | ......<br>.... |
| iPS:39 3134 | 21-225_34C2 | VK1\|O12/ JK4 | ............<br>TF......... | ......R.I..<br>........ | .F......<br>....... | V.......<br>....... | ...............Y.<br>................. | ..................<br>............Y. | ......<br>M... |
| iPS:39 3136 | 21-225_34D8 | VK1\|O12/ JK4 | ............<br>T.......... | ......I.I..<br>........ | ........<br>......F | V.......<br>....... | .................<br>................. | ..................<br>............Y. | ......<br>.... |
| iPS:39 3840 | 21-225_3F8 | VK1\|O12/ JK4 | .F..........<br>........... | ........L..<br>........ | ..R....R<br>..QV..H | T.......<br>.T..... | ...............V.<br>................. | ..T...............<br>.............. | .....R<br>...N |
| iPS:39 3844 | 21-225_3G7 | VK1\|O12/ JK4 | ............<br>........V.. | ......N.Y..<br>....R... | ...GR..R<br>....M.. | ........<br>....S.. | .................<br>.V............... | ..................<br>..........P.F. | ....A.<br>...D |
| iPS:39 3852 | 21-225_12A10 | VK1\|O12/ JK4 | .V..........<br>..A........ | ......N.Y..<br>........ | .......R<br>...V..H | T.......<br>....... | ..............A..<br>....N............ | ..................<br>..........P... | ......<br>.... |
| iPS:39 3900 | 21-225_10E12 | VK1\|O12/ JK4 | ............<br>........... | ......N.Y..<br>........ | ...E...R<br>....... | ........<br>.T..... | ......G..........<br>................. | ..N...............<br>..........P... | ......<br>...E |
| iPS:39 3920 | 21-225_1H12 | VK1\|O12/ JK4 | ............<br>........... | ......N.Y..<br>....R... | ...E...R<br>....... | T.......<br>....... | .........D.......<br>................. | ..................<br>..........P... | ......<br>.... |
| iPS:39 3926 | 21-225_4G4 | VK1\|O12/ JK4 | ...........A<br>........... | ......T.I..<br>........ | ........<br>......H | T.......<br>....... | .................<br>..S.............. | ..T...............<br>.............. | ......<br>.... |
| iPS:39 3930 | 21-225_7E11 | VK1\|O12/ JK4 | ............<br>.........A. | ......N.I..<br>........ | ........<br>...F... | T.......<br>....... | .................<br>.............I... | ..T...............<br>.............. | ......<br>.... |
| iPS:39 3932 | 21-225_10F5 | VK1\|O12/ JK4 | ............<br>........... | ......N.Y..<br>....R... | ...E...R<br>....... | T.......<br>....... | .........D.......<br>................. | ..................<br>..........P... | ......<br>.... |
| iPS:39 3964 | 21-225_6G1 | VK1\|O12/ JK4 | ............<br>........... | .T...N.I..<br>........ | ........<br>...V... | T.......<br>...N..T | ....G............<br>................. | ..PH..............<br>..........P... | ......<br>.... |
| iPS:39 4012 | 21-225_15A3 | VK1\|O12/ JK4 | ............<br>........... | ........I..<br>........ | ..L.....<br>...F... | T.......<br>....... | .................<br>................. | ..T...............<br>.............. | ......<br>.... |
| iPS:39 4016 | 21-225_13D4 | VK1\|O12/ JK4 | .L..........<br>........... | ........F..<br>........ | ........<br>......C | T.......<br>......N | .................<br>................. | ..T...............<br>..........L... | ......<br>.... |
| iPS:39 4083 | 21-225_16E6 | VK1\|O12/ JK4 | ............<br>........... | ........I..<br>........ | ........<br>...F... | T.......<br>.T..... | .................<br>................. | ..T...............<br>.............. | ......<br>.... |
| iPS:39 8480 | 21-225_17G4 | VK1\|O12/ JK4 | ............<br>..A........ | .T...N.....<br>....N... | ........<br>....... | V.......<br>....FP. | ...............E.<br>................. | ...NF.............<br>..........F... | ......<br>...I |
| iPS:39 8486 | 21-225_19A1 | VK1\|O12/ JK4 | ............<br>........... | .....HT.T..<br>........ | ........<br>...F... | ........<br>.T.N... | .................<br>.F...........I... | ....N.............<br>..........F... | ......<br>.... |

| | VK2\|A18/JK4 | | DIVMTQTPLSLS VTPGQPASISC | KSS-- QSLLHSD- GKTYLY | WYLQKPGQ SPQLLIY | E------- -VSSRFS | GVPDRFSGSGSG-- TDFTLKISRVEAEDVGV YYC | MQGIH------------- ----------LPLT | FGGGTK VEIK |
|---|---|---|---|---|---|---|---|---|---|
| iPS:451139 | 21-225_71A6 | VK2\|A18/JK4 | ............<br>........... | .........R.<br>.....H.. | ........<br>P...... | ........<br>...N... | ..S..............<br>................. | ..SKQ............<br>.............. | ......<br>..F. |
| iPS:433937 | 21-225_44B10 | VK2\|A18/JK4 | H...........<br>........... | ...........<br>E..R.... | ........<br>P...... | ........<br>.I.H... | .................<br>................. | ..S..............<br>............F. | ......<br>.... |
| iPS:433979 | 21-225_46B9 | VK2\|A18/JK4 | ............<br>........... | ...........<br>E....... | ........<br>P...... | ........<br>...Y... | .................<br>......M.......... | .HS.Q............<br>..........Y... | ......<br>...Q |
| iPS:434201 | 21-225_59A12 | VK2\|A18/JK4 | ............<br>........... | ........Q.G<br>E....... | ..V.....<br>P...... | ........<br>...Y... | .................<br>........V........ | ..STQ............<br>.............. | ......<br>.... |
| iPS:434205 | 21-225_60G2 | VK2\|A18/JK4 | ............<br>.....S..... | ...........<br>E....... | ........<br>P...... | ........<br>...N.I. | .................<br>................. | ..S.Q............<br>.............. | ......<br>.... |
| iPS:434223 | 21-225_60C12 | VK2\|A18/JK4 | ............<br>........... | ...........<br>E....... | ........<br>P..F... | ........<br>...N... | .................<br>.............I... | ..S.K............<br>..........Y... | ......<br>.... |
| iPS:434233 | 21-225_61B3 | VK2\|A18/JK4 | ............<br>........... | ...........<br>E....... | ........<br>P...... | ........<br>...N.I. | .................<br>................. | ..S.Q............<br>.............. | ......<br>.... |
| iPS:434303 | 21-225_58H11 | VK2\|A18/JK4 | ............<br>........... | ...........<br>E....... | ..V.....<br>P...... | ........<br>...Y... | .................<br>................. | ..S.Q............<br>.............. | ......<br>.... |
| iPS:435349 | 21-225_148F5 | VK2\|A18/JK4 | ............<br>........... | ...........<br>E....... | ........<br>P...... | ........<br>...Y.V. | .................<br>..............F. | ..S.Q............<br>.............. | ......<br>.... |
| iPS:435359 | 21-225_148H10 | VK2\|A18/JK4 | ............<br>........... | ...........<br>E....... | ........<br>P...... | ........<br>...Y.V. | .................<br>................. | ..S.Q............<br>.............. | ......<br>.... |
| iPS:435417 | 21-225_150D11 | VK2\|A18/JK4 | ............<br>........... | ...........<br>E....... | ........<br>P...... | ........<br>...Y... | .................<br>................. | ....Q............<br>.............. | ......<br>.... |
| iPS:435469 | 21-225_153G9 | VK2\|A18/JK4 | ........F...<br>........... | ...........<br>........ | ........<br>P..F... | ........<br>...N... | .................<br>.........D...... | ..N.K............<br>.........Y... | ......<br>.... |
| iPS:435733 | 21-225_173C11 | VK2\|A18/JK4 | ............<br>........... | ...........<br>E....... | ........<br>P...... | ........<br>...H... | .................<br>................. | ..S.-<br>...................<br>.....QL.. | ......<br>.... |
| iPS:435785 | 21-225_179C2 | VK2\|A18/JK4 | ............<br>........... | ...........<br>E....... | ........<br>P...... | ........<br>...H... | .................<br>................. | ..S.-<br>...................<br>.....QV.. | ......<br>.... |
| iPS:392618 | 21-225_16F10 | VK2\|A18/JK4 | ............<br>.I......... | ...........<br>.....H.N | ........<br>P...... | ........<br>...Y... | ..............V.<br>..E.....A...... | F.S.Q............<br>.............. | ......<br>.... |
| iPS:392860 | 21-225_22H8 | VK2\|A18/JK4 | ............<br>........... | ...........<br>.....F.. | .......H<br>P...... | ........<br>...N... | .................<br>.............I... | ..S.Q............<br>.............S | ......<br>...N |
| iPS:392888 | 21-225_25A2 | VK2\|A18/JK4 | ....N.......<br>........... | ...........<br>E....... | ........<br>P...... | ........<br>.I.N... | ...A.L...........<br>................. | ..STQ............<br>..........F... | ......<br>.... |
| iPS:392938 | 21-225_29H4 | VK2\|A18/JK4 | ............<br>........F.. | ...........<br>........ | ........<br>P.....F | ........<br>...H... | .L...............<br>................. | ..S.Q............<br>.........H.F. | .....R<br>.... |
| iPS:39 | 21- | VK2\|A18/ | ............ | ......T...G | ........ | ........ | ................. | ..S.K............ | ...... |

| | | | | | | | | | |
|---|---|---|---|---|---|---|---|---|---|
| iPS:39 3012 | 21-225_26G7 | VK2\|A18/ JK4 | ..L.........<br>........... | ...........<br>E....... | ........<br>P..F... | ........<br>...H.L. | .................<br>................. | ..S.Q.............<br>.............. | ......<br>.... |
| iPS:39 3144 | 21-225_34D2 | VK2\|A18/ JK4 | ............<br>........... | ...........<br>........ | ........<br>P...... | ........<br>...N... | .................<br>................. | ..SK-<br>.................<br>.....QLPP | ......<br>...R |
| | | Germline | **K_FR1** | **K_CDR1** | **K_FR2** | **K_CDR2** | **K_FR3** | **K_CDR3** | **K_FR4** |
| | **VK1\|L1/JK3** | | DIQMTQSPSSLS<br>ASVGDRVTITC | RAS--QGIS--<br>----NYLA | WFQQKPGK<br>APKSLIY | A-------<br>-ASSLQS | GVPSRFSGSGSG--<br>TDFTLTISSLQPEDFAT<br>YYC | QQYNS-----------<br>----------YPFT | FGPGTK<br>VDIK |
| iPS:45 1143 | 21-225_66H11 | VK1\|L1/J K3 | ......F....F<br>.F......... | P..........<br>........ | ........<br>....... | G.......<br>..FN.H. | ....K.....F......<br>....N.......N... | ...SC.............<br>.............. | ..H...<br>.... |
| iPS:46 8814 | 21-225_223D11 | VK1\|L1/J K3 | ............<br>........... | ...........<br>........ | ........<br>....... | ........<br>...T... | ....K....R.......<br>N...N........... | ...SG.............<br>.............. | ......<br>..T. |
| iPS:43 3901 | 21-225_43A4 | VK1\|L1/J K3 | ............<br>........... | ........N..<br>........ | ........<br>......N | ........<br>....... | .................<br>..A.............. | ...Y..............<br>.............. | ......<br>.... |
| iPS:43 3961 | 21-225_45D9 | VK1\|L1/J K3 | ............<br>........... | ........N..<br>........ | ........<br>......N | ........<br>....... | .................<br>..A.............. | .H.Y..............<br>.............. | ..R...<br>.... |
| iPS:43 4059 | 21-225_51C5 | VK1\|L1/J K3 | ............<br>........... | ...........<br>........ | .......E<br>....... | ........<br>.....R. | ....Q............<br>I................ | ...Y..............<br>.............. | ......<br>.... |
| iPS:43 4085 | 21-225_52E3 | VK1\|L1/J K3 | ............<br>........... | ...........<br>........ | ........<br>......N | ........<br>....... | ....K............<br>................. | ..................<br>.........F... | ......<br>.... |
| iPS:43 4115 | 21-225_53E4 | VK1\|L1/J K3 | ............<br>........... | ......V....<br>........ | ........<br>......S | ........<br>....... | ....K............<br>................. | ...H..............<br>............L. | ..R...<br>.... |
| iPS:43 4213 | 21-225_60A4 | VK1\|L1/J K3 | ............<br>........... | ...........<br>........ | ........<br>....... | ........<br>....... | ....K............<br>........S........ | ...K..............<br>..........H... | ......<br>.... |
| iPS:43 4215 | 21-225_60F7 | VK1\|L1/J K3 | ............<br>.........S. | ......V.K..<br>.......V | .V......<br>....... | ........<br>....... | ....T............<br>................. | L.FH..............<br>.............. | ......<br>M... |
| iPS:43 4261 | 21-225_56F7 | VK1\|L1/J K3 | ..L.........<br>........... | ...........<br>....T... | ....T..T<br>....... | ........<br>......G | ....K............<br>................. | H.................<br>.........F..K | ..R...<br>...T |
| iPS:43 4331 | 21-225_63H8 | VK1\|L1/J K3 | ............<br>........... | ...........<br>........ | ........<br>.H..... | ........<br>....... | ....K............<br>................. | ...H..............<br>.............. | ......<br>...R |
| iPS:43 4361 | 21-225_65D5 | VK1\|L1/J K3 | ............<br>........... | ......D.N..<br>........ | ........<br>..R.... | ........<br>.....H. | ....Q..A......S..<br>................. | PL.K..............<br>............L. | ......<br>.... |
| iPS:43 4405 | 21-225_68E6 | VK1\|L1/J K3 | ............<br>........... | ...........<br>....Y... | .......R<br>....... | V.......<br>....... | .................<br>.............I... | ...D..............<br>.............. | ......<br>...R |
| iPS:43 5259 | 21-225_96C6 | VK1\|L1/J K3 | ............<br>........... | ...........<br>........ | ........<br>....... | ........<br>....... | ....K............<br>....N............ | H...D.............<br>.............. | ......<br>.... |
| iPS:43 5351 | 21-225_148B6 | VK1\|L1/J K3 | ............<br>.......S... | ...........<br>....K... | ........<br>......F | ........<br>....... | ....K............<br>................. | ..................<br>.........F... | ......<br>.... |
| iPS:43 5461 | 21-225_153A1 | VK1\|L1/J K3 | ............<br>........... | ......V....<br>........ | ........<br>......S | ........<br>.....R. | ....N............<br>................. | ...H..............<br>.............. | ......<br>..F. |
| iPS:43 | 21- | VK1\|L1/J | ............ | ......D.... | ....R... | ........ | ....K............ | ...H.............. | ...... |

| | | | | | | | | | |
|---|---|---|---|---|---|---|---|---|---|
| iPS:43<br>5515 | 21-<br>225_157E4 | VK1\|L1/J<br>K3 | ............<br>........F.. | ...........<br>........ | .......R<br>....... | ........<br>.....R. | ....Q.............<br>....N............. | ...H...............<br>............... | ......<br>.... |
| iPS:43<br>5523 | 21-<br>225_157G5 | VK1\|L1/J<br>K3 | ............<br>........... | ........N..<br>........ | ........<br>..E.... | ........<br>....... | ....Q.............<br>.................. | ...................<br>............... | ......<br>.... |
| iPS:43<br>5535 | 21-<br>225_157H10 | VK1\|L1/J<br>K3 | ............<br>........... | ........T..<br>........ | ........<br>....... | T.......<br>...N... | ....K.............<br>.................. | ...H...............<br>............... | ......<br>.... |
| iPS:43<br>5559 | 21-<br>225_158H12 | VK1\|L1/J<br>K3 | ............<br>........... | ........N..<br>........ | ........<br>......S | ........<br>....... | ....K.............<br>..S............... | ...H...............<br>............... | ......<br>.... |
| iPS:43<br>5575 | 21-<br>225_159H11 | VK1\|L1/J<br>K3 | ............<br>........... | ...........<br>....K..V | ........<br>....... | ........<br>....... | ....K.............<br>.....N............ | ...................<br>............... | ......<br>.... |
| iPS:43<br>5579 | 21-<br>225_160G1 | VK1\|L1/J<br>K3 | ............<br>........... | ......D.N..<br>........ | ........<br>..T.... | ........<br>.S..... | ....K.............<br>.................. | ...H...............<br>............... | ......<br>...R |
| iPS:43<br>5585 | 21-<br>225_160G3 | VK1\|L1/J<br>K3 | ............<br>........... | ......D.N..<br>........ | ....R...<br>..T.... | ........<br>.S..... | ....K.............<br>.................. | ...H...............<br>............... | ......<br>.... |
| iPS:43<br>5635 | 21-<br>225_163F1 | VK1\|L1/J<br>K3 | ............<br>........... | ......D....<br>........ | ........<br>....... | ........<br>....... | ....K.............<br>..............A... | ...................<br>............... | .....Q<br>..AQ |
| iPS:43<br>5659 | 21-<br>225_167D12 | VK1\|L1/J<br>K3 | ............<br>........... | ........N..<br>........ | ........<br>..E.... | ........<br>....... | ....K.............<br>................F. | ...................<br>............... | ......<br>.... |
| iPS:43<br>5679 | 21-<br>225_169D10 | VK1\|L1/J<br>K3 | ............<br>........... | ......D....<br>........ | ........<br>....... | ........<br>....... | ....K.............<br>.................. | ...H...............<br>............... | ......<br>.... |
| iPS:43<br>5685 | 21-<br>225_170E1 | VK1\|L1/J<br>K3 | ............<br>..E........ | ...........<br>........ | ........<br>....... | ........<br>....... | ....K.............<br>.................. | ...H...............<br>............... | ......<br>.... |
| iPS:43<br>5747 | 21-<br>225_175C4 | VK1\|L1/J<br>K3 | ............<br>........... | ........G..<br>........ | ........<br>....... | ........<br>...G... | .F..K.............<br>.................. | ...Y...............<br>............... | ......<br>.... |
| iPS:43<br>5765 | 21-<br>225_177D3 | VK1\|L1/J<br>K3 | ....S.......<br>........... | ........T..<br>........ | ........<br>....... | ........<br>....... | ....K.............<br>.................. | ...................<br>............... | ......<br>.... |
| iPS:43<br>5797 | 21-<br>225_181G2 | VK1\|L1/J<br>K3 | ............<br>..I.E...... | ...........<br>........ | .I.....T<br>....... | ........<br>....... | ..S.......F.......<br>.................. | ....G..............<br>............... | ......<br>.... |
| iPS:43<br>5835 | 21-<br>225_190F12 | VK1\|L1/J<br>K3 | ............<br>........... | ........G..<br>....K... | ........<br>....... | ........<br>....... | ....K.............<br>.........D........ | .R.DT..............<br>............... | ......<br>.... |
| iPS:43<br>5861 | 21-<br>225_190A5 | VK1\|L1/J<br>K3 | ............<br>........... | ........G..<br>.....H.. | ........<br>......H | ........<br>....... | ....K.............<br>.................. | ...SN..............<br>.............V. | ......<br>.... |
| iPS:43<br>5869 | 21-<br>225_190B1 | VK1\|L1/J<br>K3 | ............<br>........... | ........R..<br>........ | ........<br>....... | V.......<br>.....E. | ....K...........E.<br>............G..... | ...LN..............<br>.............V. | ......<br>...R |
| iPS:43<br>5877 | 21-<br>225_184E7 | VK1\|L1/J<br>K3 | ..........R.<br>..I.E...... | ...........<br>........ | .I.....T<br>....... | ........<br>....... | ..S.......F.......<br>.I.....V.R........ | ....G..............<br>............... | ..H...<br>.... |
| iPS:43<br>5883 | 21-<br>225_185A1 | VK1\|L1/J<br>K3 | ............<br>........... | ...........<br>........ | ....T...<br>......S | V.......<br>....... | .......A..........<br>.................. | R..H...............<br>............... | ......<br>.... |
| iPS:43<br>5885 | 21-<br>225_185E10 | VK1\|L1/J<br>K3 | ..........R.<br>..I.E...... | ...........<br>........ | .I.....T<br>....... | ........<br>....... | ..S.......F.......<br>.................. | ....G..............<br>............... | ......<br>.... |
| iPS:43<br>5891 | 21-<br>225_188H5 | VK1\|L1/J<br>K3 | ............<br>..I.E...... | ...........<br>........ | .L.....T<br>....... | ........<br>....... | ..S.......F.......<br>.................. | ...................<br>............... | ......<br>.... |

| | | | | | | | | | |
|---|---|---|---|---|---|---|---|---|---|
| iPS:43 5897 | 21-225_188B9 | VK1\|L1/JK3 | ............<br>..I.E...... | ...........<br>........ | .L.....T<br>....... | ........<br>....... | ..S.......F......<br>................. | ..................<br>.............. | ......<br>.... |
| iPS:43 5937 | 21-225_190H9 | VK1\|L1/JK3 | ............<br>........... | ........G..<br>....K... | ........<br>.L..... | ........<br>....... | ....K............<br>.........D....... | .R.DT.............<br>.............. | ......<br>.... |
| iPS:43 5961 | 21-225_192A2 | VK1\|L1/JK3 | ............<br>........... | ..N.....N..<br>........ | ........<br>....... | ........<br>....... | ....K............<br>................. | ..................<br>.............. | ......<br>.... |
| iPS:43 5977 | 21-225_192E4 | VK1\|L1/JK3 | ............<br>........... | ........G..<br>........ | ........<br>....... | V.......<br>.V..... | .................<br>................. | .R.DT.............<br>.............. | ......<br>.... |
| iPS:43 6001 | 21-225_192C10 | VK1\|L1/JK3 | ............<br>........... | ........G..<br>....K... | ........<br>....... | ........<br>....... | ....K............<br>.........D....... | .R.DT.............<br>.............. | ......<br>..F. |
| iPS:43 6039 | 21-225_193F8 | VK1\|L1/JK3 | ....I.......<br>........... | .......V...<br>.....H.. | .......R<br>....... | ........<br>....... | ....K.........A..<br>................. | ..................<br>.............. | ......<br>.... |
| iPS:43 6078 | 21-225_194H12 | VK1\|L1/JK3 | ............<br>..........R | ........G..<br>....K... | ........<br>.L..... | ........<br>....... | ....K............<br>.........D....... | .R.DT.............<br>.............. | ......<br>.... |
| iPS:43 6140 | 21-225_197G3 | VK1\|L1/JK3 | ............<br>........... | ........G..<br>.....H.. | ........<br>....... | ........<br>....... | ....K....R.......<br>S................ | ...SN.............<br>............V. | ......<br>.... |
| iPS:43 6167 | 21-225_197E11 | VK1\|L1/JK3 | ............<br>........... | ........N..<br>....K..S | ........<br>....... | ........<br>....V.. | ....K............<br>.....R...D......G | .R.DT.............<br>.............. | ......<br>.... |
| iPS:43 6370 | 21-225_211A6 | VK1\|L1/JK3 | ............<br>.....S..... | ........G..<br>....K... | ........<br>....... | ........<br>.....L. | ....K............<br>................. | .K.DT.............<br>.............. | ......<br>.... |
| iPS:43 6392 | 21-225_213B3 | VK1\|L1/JK3 | ............<br>........... | ........G..<br>....K... | ........<br>......S | ........<br>....VL. | ....K............<br>................. | .K.DT.............<br>.............. | ......<br>.... |
| iPS:43 6404 | 21-225_214C3 | VK1\|L1/JK3 | ............<br>........... | ........G..<br>........ | ........<br>V...... | ........<br>....... | ....K............<br>............T.... | ...MT.............<br>............I. | ......<br>.... |
| iPS:43 6406 | 21-225_214E4 | VK1\|L1/JK3 | ............<br>........... | ...........<br>........ | ........<br>....... | ........<br>....... | ....K............<br>................. | ...LT.............<br>.............. | ......<br>.... |
| iPS:43 7216 | 21-225_51D5 | VK1\|L1/JK3 | ............<br>........... | ........N..<br>........ | .......E<br>....... | ........<br>.....R. | ....Q............<br>I................ | ...Y..............<br>.............. | ......<br>.... |
| iPS:43 7224 | 21-225_56H1 | VK1\|L1/JK3 | ............<br>........... | ...........<br>....H... | ........<br>..Q..MS | ........<br>...G... | ....K............<br>................. | ...QN.............<br>.............. | ......<br>.... |
| iPS:39 2620 | 21-225_17H5 | VK1\|L1/JK3 | ............<br>........... | ...........<br>........ | ........<br>......N | ........<br>....... | ....K............<br>................. | ...H..............<br>.............. | ......<br>.... |
| iPS:39 2692 | 21-225_18G10 | VK1\|L1/JK3 | ............<br>........... | ......D....<br>....Y... | ........<br>....... | V.......<br>....... | ....K.G...F......<br>................. | L.................<br>.............. | ......<br>.... |
| iPS:39 2708 | 21-225_18D11 | VK1\|L1/JK3 | ...........F<br>.F......... | ...........<br>....Y... | ........<br>....... | V.......<br>....... | ....K.....F......<br>................. | ....T.............<br>.............. | .....T<br>.... |
| iPS:39 2714 | 21-225_16G12 | VK1\|L1/JK3 | ............<br>........... | ......D....<br>........ | ........<br>....... | ........<br>....... | ....K.R..........<br>.....N.......S... | ...H..............<br>..........F... | ......<br>.... |
| iPS:39 2746 | 21-225_20H7 | VK1\|L1/JK3 | ............<br>........... | .T......N..<br>.......V | ........<br>...R... | ........<br>....... | ....K............<br>................. | ...Y..............<br>.............. | ......<br>M.F. |
| iPS:39 2782 | 21-225_22B12 | VK1\|L1/JK3 | ............<br>.........I. | ......D....<br>........ | ...E....<br>.H..... | G.......<br>.....R. | ....K............<br>N..........L..... | ...H..............<br>.............. | ......<br>..F. |

| | | | | | | | | | |
|---|---|---|---|---|---|---|---|---|---|
| iPS:39 2784 | 21-225_23C7 | VK1\|L1/J K3 | ............<br>........... | ........G..<br>....I... | ........<br>......S | ........<br>....... | ....K..........E.<br>................. | ..................<br>.............. | ......<br>.... |
| iPS:39 2802 | 21-225_23E7 | VK1\|L1/J K3 | ............<br>........... | ...........<br>........ | ....I...<br>....... | ........<br>....... | ....K............<br>................. | ..FY..............<br>.............. | ......<br>...N |
| iPS:39 2826 | 21-225_20B9 | VK1\|L1/J K3 | ............<br>........... | ...........<br>........ | ........<br>....... | V.......<br>....... | ....K............<br>................. | ....T.............<br>.............. | ......<br>.... |
| iPS:39 2840 | 21-225_23G1 | VK1\|L1/J K3 | ............<br>........... | ...........<br>........ | ........<br>......S | ........<br>....... | ....Q.....F......<br>................. | ..................<br>.............. | ......<br>.... |
| iPS:39 2842 | 21-225_23G8 | VK1\|L1/J K3 | ............<br>........... | ........R..<br>........ | ........<br>....... | ........<br>....... | ....N............<br>................. | ..................<br>.............. | I.....<br>.... |
| iPS:39 2890 | 21-225_20H9 | VK1\|L1/J K3 | ............<br>........... | ...........<br>........ | ........<br>......S | ........<br>....... | ....K............<br>................. | ..................<br>.............. | ......<br>.... |
| iPS:39 2892 | 21-225_20C11 | VK1\|L1/J K3 | ............<br>........... | ......D....<br>........ | ........<br>....... | ........<br>....... | ....K.R..........<br>................. | ...H..............<br>..........F... | ......<br>..V. |
| iPS:39 2950 | 21-225_25C10 | VK1\|L1/J K3 | ............<br>........... | ...........<br>........ | ........<br>....... | ........<br>....... | .F..K............<br>................. | ...H..............<br>.............. | ......<br>.... |
| iPS:39 2952 | 21-225_26G1 | VK1\|L1/J K3 | ............<br>........... | ......D....<br>........ | ........<br>....... | ........<br>.....R. | ....N............<br>..........N...... | ...H..............<br>.............. | ......<br>.... |
| iPS:39 2962 | 21-225_30A1 | VK1\|L1/J K3 | ............<br>........... | ...........<br>........ | ........<br>......S | ........<br>......T | ....K............<br>................. | ..................<br>.............. | ......<br>.... |
| iPS:39 2976 | 21-225_27H12 | VK1\|L1/J K3 | ............<br>........... | ...........<br>........ | ........<br>......N | G.......<br>....... | ....K............<br>................. | ...Y..............<br>.............. | ......<br>.N.N |
| iPS:39 3090 | 21-225_33A5 | VK1\|L1/J K3 | ............<br>........... | ...........<br>........ | ........<br>......S | ........<br>....... | ....K............<br>................. | ..................<br>.............. | .....N<br>.... |
| iPS:39 3120 | 21-225_35H8 | VK1\|L1/J K3 | ............<br>........... | ......A....<br>........ | ........<br>....... | G.......<br>...G... | ....K............<br>................. | ..................<br>.............. | ......<br>..F. |
| iPS:39 3836 | 21-225_15A2 | VK1\|L1/J K3 | ...........F<br>.FI........ | ...........<br>........ | ........<br>......F | ........<br>....... | ....K.....F......<br>.FP..........N... | ...Y..............<br>.............. | .....Q<br>..V. |
| iPS:39 3870 | 21-225_7B1 | VK1\|L1/J K3 | ......A.....<br>........... | ......D....<br>.....H.V | ........<br>......F | ........<br>....... | ....Q............<br>.....I........... | H.................<br>.............. | ......<br>..F. |
| iPS:39 3894 | 21-225_5E11 | VK1\|L1/J K3 | V...........<br>........... | ...........<br>........ | ........<br>......N | ........<br>....V.. | ....K...N........<br>................. | H..H..............<br>.............. | ......<br>.... |
| iPS:39 3896 | 21-225_2A4 | VK1\|L1/J K3 | ...........F<br>........... | ...........<br>........ | ........<br>...R... | T.......<br>....... | ....K.....F......<br>................. | ..................<br>.............. | ......<br>.... |
| iPS:39 3914 | 21-225_16B8 | VK1\|L1/J K3 | ............<br>........... | ........N..<br>........ | ........<br>.L....N | ........<br>....V.. | ....K............<br>................. | H..H..............<br>.............. | ......<br>...V |
| iPS:39 3968 | 21-225_5A5 | VK1\|L1/J K3 | ............<br>........... | ...........<br>........ | ........<br>......S | ........<br>....... | ....K............<br>................. | ..................<br>.............. | ......<br>.... |
| iPS:39 3992 | 21-225_14H8 | VK1\|L1/J K3 | ............<br>........... | ...........<br>....Y... | ........<br>....... | V.......<br>....... | ....K............<br>................. | ..................<br>.............. | ......<br>...N |
| iPS:39 4018 | 21-225_15B1 | VK1\|L1/J K3 | ............<br>........... | ...........<br>........ | ........<br>......S | ........<br>....... | ....N............<br>................. | ...H..............<br>.............. | ......<br>.... |

| | | | | | | | | | |
|---|---|---|---|---|---|---|---|---|---|
| iPS:394026 | 21-225_16C7 | VK1\|L1/JK3 | ............<br>........... | ...........<br>........ | ........<br>......S | ........<br>....... | ....K............<br>................. | ..................<br>.............. | ......<br>.... |
| iPS:394055 | 21-225_9C8 | VK1\|L1/JK3 | ............<br>........... | ...........<br>....Y... | ........<br>....... | V.......<br>....... | ....K............<br>................. | ...D..............<br>.............. | ......<br>.... |
| iPS:398482 | 21-225_17H6 | VK1\|L1/JK3 | ............<br>........... | .....RD....<br>........ | ........<br>......S | T.......<br>....... | ....K............<br>.............I... | ...H..............<br>.............. | ......<br>...Q |
| iPS:398492 | 21-225_21F12 | VK1\|L1/JK3 | ............<br>........... | ........R..<br>.....F.. | ........<br>......S | ........<br>......T | ....K............<br>................. | ..................<br>..........F... | ......<br>.... |
| iPS:398500 | 21-225_23A11 | VK1\|L1/JK3 | ............<br>T.......... | ......D....<br>........ | ........<br>...R... | ........<br>...T... | ....K............<br>................. | ..................<br>.............. | ......<br>..L. |
| iPS:398526 | 21-225_32B3 | VK1\|L1/JK3 | ............<br>..I........ | ...........<br>........ | ........<br>..R.... | ........<br>....... | ....T............<br>................. | ..................<br>.............. | ......<br>.... |
| iPS:402235 | 21-225_20F10 | VK1\|L1/JK3 | ...L........<br>........... | ........N..<br>........ | ........<br>....... | ........<br>....... | ..........F......<br>..............F.. | ..................<br>.............. | ......<br>..N. |
| | | Germline | K_FR1 | K_CDR1 | K_FR2 | K_CDR2 | K_FR3 | K_CDR3 | K_FR4 |
| | VK3\|A27/JK1 | | EIVLTQSPGTLS<br>LSPGERATLSC | RAS--QSVSS-<br>----SYLA | WYQQKPGQ<br>APRLLIY | G-------<br>-ASSRAT | GIPDRFSGSGSG--<br>TDFTLTISRLEPEDFAV<br>YYC | QQYGS------------<br>----------SPWT | FGQGTK<br>VEIK |
| iPS:468810 | 21-225_74D5 | VK3\|A27/JK1 | ............<br>........... | ........N..<br>....N... | ..R.....<br>....... | ........<br>....... | .................<br>I.I.............. | ...E..............<br>.............. | ......<br>.... |
| iPS:468834 | 21-225_94G10 | VK3\|A27/JK1 | ............<br>........... | ........N..<br>....N... | ..R.....<br>....... | ........<br>....... | .................<br>I.I.............. | ...E..............<br>.............. | ......<br>.... |
| iPS:468838 | 21-225_80E12 | VK3\|A27/JK1 | ......C.....<br>........V.. | ........N..<br>....N... | ..R...D.<br>....... | ........<br>....... | .................<br>I.I.............. | ...E..............<br>.............. | ......<br>.... |
| iPS:468820 | 21-225_76E10 | VK3\|A27/JK1 | ............<br>........... | ........N..<br>....N... | ..R.....<br>....... | ........<br>....... | .................<br>I.I.............. | ...E..............<br>.............. | ......<br>.... |
| iPS:433931 | 21-225_44F6 | VK3\|A27/JK1 | ............<br>........... | .........G.<br>........ | ........<br>....... | ........<br>....... | .................<br>................. | ..................<br>.............. | ......<br>.... |
| iPS:434307 | 21-225_59B2 | VK3\|A27/JK1 | ............<br>........... | W.......Y..<br>.....F.. | .F...S..<br>....... | ........<br>....... | .................<br>..............F.. | ....T.............<br>.............. | ......<br>.... |
| iPS:434471 | 21-225_75G3 | VK3\|A27/JK1 | ............<br>........... | ..R...N.D..<br>........ | ........<br>....... | ........<br>....... | .................<br>..............F.. | ...ER.............<br>.............. | ......<br>.... |
| iPS:434473 | 21-225_76D1 | VK3\|A27/JK1 | ............<br>........... | ......NIY..<br>....N... | ...E....<br>....... | ........<br>....... | .................<br>............V.... | ...E..............<br>.............. | ......<br>.... |
| iPS:434495 | 21-225_74B2 | VK3\|A27/JK1 | .L..........<br>........... | ......NIY..<br>........ | ........<br>....... | ........<br>....... | .................<br>...............C. | ...E..............<br>.............. | ......<br>.... |
| iPS:434497 | 21-225_76A4 | VK3\|A27/JK1 | ...........Y<br>........... | ........Y..<br>........ | ........<br>....... | ........<br>....... | .................<br>A................ | .HSDN.............<br>.............. | ......<br>.... |
| iPS:434501 | 21-225_76G4 | VK3\|A27/JK1 | ............<br>........... | ........Y..<br>........ | ........<br>....... | ........<br>....... | .................<br>A..........C..... | .HSDN.............<br>.............. | ......<br>.... |
| iPS:434507 | 21-225_74C5 | VK3\|A27/JK1 | ............<br>........... | ........N..<br>....N... | ........<br>....... | ........<br>..F.... | .....V...........<br>N.I.............. | ...E..............<br>.............. | ......<br>.... |

| | | | | | | | | | |
|---|---|---|---|---|---|---|---|---|---|
| iPS:43 4517 | 21-225_76A7 | VK3\|A27/ JK1 | ..A.........<br>........... | .....P..D..<br>........ | ....R...<br>....... | ........<br>......P | .................<br>................. | ...E..............<br>.............. | .......<br>.... |
| iPS:43 4519 | 21-225_74C7 | VK3\|A27/ JK1 | ............<br>........... | .T...PN.D..<br>........ | ........<br>....... | ........<br>....... | .................<br>..............F.. | ...ER.............<br>.............. | .......<br>.... |
| iPS:43 4523 | 21-225_75C3 | VK3\|A27/ JK1 | ............<br>..Q........ | ...........<br>....R... | ........<br>....... | ........<br>....... | .................<br>................. | .H.D..............<br>.............. | .......<br>.... |
| iPS:43 4531 | 21-225_76C9 | VK3\|A27/ JK1 | ............<br>........... | ...........<br>.......S | ........<br>....... | ........<br>....... | .................<br>................. | ....–<br>..................<br>.....RSR. | .......<br>...R |
| iPS:43 4533 | 21-225_85F7 | VK3\|A27/ JK1 | .P..........<br>........... | ......NIY..<br>....N... | ........<br>....... | ........<br>....... | .................<br>...............C. | ...E..............<br>.............. | .......<br>.... |
| iPS:43 4547 | 21-225_74H5 | VK3\|A27/ JK1 | ............<br>........... | ........N..<br>....N... | ..R.....<br>....... | ........<br>....... | .................<br>I.I.............. | ...E..............<br>.............. | .......<br>.... |
| iPS:43 4559 | 21-225_74D11 | VK3\|A27/ JK1 | ............<br>........... | ........Y..<br>........ | ........<br>....... | ........<br>....... | .................<br>A................ | .H.DN.............<br>.............. | .......<br>.... |
| iPS:43 4561 | 21-225_77G1 | VK3\|A27/ JK1 | ...........Y<br>........... | ........Y..<br>........ | ........<br>....... | ........<br>....... | .................<br>A..........C..... | .H.DN.............<br>.............. | .......<br>.... |
| iPS:43 4565 | 21-225_75B10 | VK3\|A27/ JK1 | ............<br>........... | .....P..N..<br>....Y... | ........<br>....... | ........<br>..T.... | .................<br>...............F. | ...ED.............<br>.............. | .......<br>.... |
| iPS:43 4571 | 21-225_74D2 | VK3\|A27/ JK1 | ............<br>........... | ........D..<br>....N... | ........<br>....... | ........<br>......P | .................<br>....N..........F. | ...E..............<br>.............. | .......<br>.... |
| iPS:43 4579 | 21-225_77F7 | VK3\|A27/ JK1 | ...........Y<br>........... | ........Y..<br>........ | ........<br>....I.. | ........<br>....... | ..........C......<br>A.....V.H..C..... | .H.DN.............<br>.............. | .......<br>.... |
| iPS:43 4581 | 21-225_74B12 | VK3\|A27/ JK1 | ...........Y<br>........... | ........Y..<br>........ | ........<br>....... | ........<br>.....S. | .................<br>A................ | .H.DN.............<br>.............. | .......<br>.... |
| iPS:43 4585 | 21-225_75A12 | VK3\|A27/ JK1 | ............<br>........... | ...........<br>....R... | ........<br>....... | ........<br>....... | .................<br>.........A....... | .H.D..............<br>.............. | .......<br>.... |
| iPS:43 4595 | 21-225_77A10 | VK3\|A27/ JK1 | ............<br>........... | ........H..<br>....R... | ........<br>..K..F | ........<br>....... | .................<br>................. | .H.D..............<br>.............. | .......<br>.... |
| iPS:43 4611 | 21-225_77C12 | VK3\|A27/ JK1 | ............<br>........... | ..R.....D..<br>........ | .....R..<br>....... | ........<br>....... | .................<br>................. | ...E..............<br>.............. | .......<br>.... |
| iPS:43 4633 | 21-225_74G8 | VK3\|A27/ JK1 | ............<br>........... | .......F...<br>....A... | ........<br>....... | ........<br>.T..... | .................<br>....G............ | ....–<br>..................<br>.....NSR. | .......<br>.... |
| iPS:43 4637 | 21-225_78E7 | VK3\|A27/ JK1 | ............<br>........... | ......N.D..<br>....N... | ........<br>....... | ........<br>....... | .................<br>..............F.. | ...ER.............<br>.............. | .......<br>.... |
| iPS:43 4657 | 21-225_79G1 | VK3\|A27/ JK1 | ...........Y<br>..S........ | ........Y..<br>........ | ......A.<br>....... | ........<br>.....S. | .................<br>A................ | .H.DN.............<br>.............. | .......<br>.... |
| iPS:43 4663 | 21-225_79F3 | VK3\|A27/ JK1 | ...........Y<br>........... | ........Y..<br>........ | ......A.<br>....... | ........<br>.....S. | .................<br>A................ | .H.DN.............<br>.............. | .......<br>.... |
| iPS:43 4671 | 21-225_74F4 | VK3\|A27/ JK1 | ............<br>........... | ......IF...<br>........ | ........<br>S...... | ........<br>....... | .................<br>................. | ....–<br>.................. | .......<br>.... |

| | | | | | | | | | |
|---|---|---|---|---|---|---|---|---|---|
| iPS:43 4687 | 21-225_75A5 | VK3\|A27/ JK1 | ...........Y<br>........... | ........Y..<br>........ | ........<br>....... | ........<br>....... | .................<br>A................ | .H.DN.............<br>................ | ......<br>.... |
| iPS:43 4691 | 21-225_75G7 | VK3\|A27/ JK1 | ............<br>........... | ..R.....D..<br>........ | .....R..<br>....... | ........<br>....... | .................<br>................. | ...E..............<br>................ | ......<br>.... |
| iPS:43 4693 | 21-225_79F11 | VK3\|A27/ JK1 | ............<br>........... | ........Y..<br>........ | ........<br>....... | ........<br>....... | .................<br>A................ | .HSDN.............<br>................ | ......<br>.... |
| iPS:43 4699 | 21-225_79G12 | VK3\|A27/ JK1 | ...........Y<br>........... | ........Y..<br>........ | ........<br>....... | ........<br>.....S. | .................<br>A.........C..... | .HSDN.............<br>................ | ......<br>.... |
| iPS:43 4701 | 21-225_80A1 | VK3\|A27/ JK1 | ..........RY<br>........... | ........Y..<br>........ | ........<br>....... | ........<br>.....S. | ..........C......<br>A....V...C..... | .HSDN.............<br>................ | ......<br>.... |
| iPS:43 4703 | 21-225_80C1 | VK3\|A27/ JK1 | ...........Y<br>........... | ........Y..<br>........ | ........<br>....... | ........<br>.....S. | .................<br>A....V...C..... | .HSDN.............<br>................ | ......<br>.... |
| iPS:43 4709 | 21-225_80E3 | VK3\|A27/ JK1 | ...........Y<br>..S........ | ........Y..<br>........ | ........<br>....... | ........<br>.....S. | ..........C......<br>A....V.......... | .HSDN.............<br>................ | ......<br>.... |
| iPS:43 4715 | 21-225_80D5 | VK3\|A27/ JK1 | .L..........<br>....K.V.... | ......NIY..<br>........ | ........<br>....... | ........<br>....... | .................<br>....T.........C. | ...E..............<br>................ | ......<br>.... |
| iPS:43 4717 | 21-225_80A6 | VK3\|A27/ JK1 | ............<br>.....IP.... | ........D..<br>....G... | ........<br>....... | ........<br>......P | .................<br>................. | ...E..............<br>................ | ......<br>.... |
| iPS:43 4725 | 21-225_80H7 | VK3\|A27/ JK1 | ............<br>........... | .......IN..<br>....N... | ........<br>....... | ........<br>....... | .................<br>.............F.. | ...E..............<br>................ | ......<br>.... |
| iPS:43 4735 | 21-225_80B10 | VK3\|A27/ JK1 | ............<br>........... | ........D..<br>........ | ........<br>....... | ........<br>......P | .................<br>................. | ...E..............<br>................ | ......<br>.... |
| iPS:43 4743 | 21-225_74A4 | VK3\|A27/ JK1 | ...........Y<br>........... | ........Y..<br>........ | ........<br>....... | ........<br>.....S. | ..........C......<br>A.........C..... | .HSDN.............<br>................ | ......<br>.... |
| iPS:43 4751 | 21-225_80H12 | VK3\|A27/ JK1 | ............<br>........... | ........Y..<br>........ | ........<br>....... | ........<br>.....S. | .................<br>A................ | .HSDN.............<br>................ | ......<br>.... |
| iPS:43 4759 | 21-225_81C5 | VK3\|A27/ JK1 | ...........Y<br>..S........ | ........Y..<br>........ | ........<br>....... | ........<br>.....S. | ..........C......<br>A....V...C..... | .HSDN.............<br>................ | ......<br>.... |
| iPS:43 4773 | 21-225_75D9 | VK3\|A27/ JK1 | ............<br>........... | ...........<br>........ | .....R..<br>....... | ........<br>....... | .................<br>................. | ...E..............<br>................ | ......<br>.... |
| iPS:43 4777 | 21-225_81C11 | VK3\|A27/ JK1 | ..........RY<br>...V....... | ........Y..<br>........ | ........<br>....... | ........<br>.....S. | .L.......C......<br>A....V...C..... | .HSDN.............<br>................ | ......<br>.... |
| iPS:43 4809 | 21-225_74F5 | VK3\|A27/ JK1 | ...........Y<br>........... | ........Y..<br>........ | ........<br>....... | ........<br>.....S. | .................<br>A................ | .HSDN.............<br>................ | ......<br>.... |
| iPS:43 4821 | 21-225_83G1 | VK3\|A27/ JK1 | ..........RY<br>........... | ........Y..<br>........ | ........<br>....... | ........<br>.....S. | VL...............<br>A....V...C..... | .HSDN.............<br>................ | ......<br>.... |
| iPS:43 4835 | 21-225_83B6 | VK3\|A27/ JK1 | ............<br>........... | ........D..<br>....G... | ........<br>....... | ........<br>.....TP | .................<br>................. | ...E..............<br>................ | ......<br>.... |
| iPS:43 4839 | 21-225_83B7 | VK3\|A27/ JK1 | ..........RY<br>..SV....... | ........Y..<br>........ | ........<br>....... | ........<br>.....S. | .................<br>A.........C..... | .HSDN.............<br>................ | ......<br>.... |
| iPS:43 4849 | 21-225_83C10 | VK3\|A27/ JK1 | ............<br>........... | .....P..H..<br>....N... | ........<br>....... | ........<br>....... | .................<br>I................ | ...E..............<br>................ | ......<br>.... |

| | | | | | | | | | |
|---|---|---|---|---|---|---|---|---|---|
| iPS:43 4869 | 21-225_84E12 | VK3\|A27/ JK1 | ............<br>........... | ........IN..<br>....N... | .........<br>....... | ........<br>....... | .................<br>.........D....F.. | ...E..............<br>............... | ......<br>.... |
| iPS:43 4879 | 21-225_85A3 | VK3\|A27/ JK1 | ...........F<br>........... | ........Y..<br>........ | ......A.<br>....... | ........<br>......S | .................<br>A................ | .H.DN.............<br>............... | ......<br>.... |
| iPS:43 4881 | 21-225_85B4 | VK3\|A27/ JK1 | ...........F<br>........... | ........Y..<br>........ | .........<br>....... | ........<br>....... | .................<br>A................ | .H.DN.............<br>............... | ......<br>.... |
| iPS:43 4887 | 21-225_85D6 | VK3\|A27/ JK1 | ...........F<br>..Q........ | ...........<br>....R... | .........<br>....... | ........<br>....... | .................<br>................. | .H.D..............<br>............... | ......<br>.... |
| iPS:43 4891 | 21-225_85G6 | VK3\|A27/ JK1 | ..A.........<br>........... | .....P..D..<br>........ | .........<br>....... | ........<br>..A...P | .................<br>...........V.... | ...E..............<br>............... | ......<br>.... |
| iPS:43 4895 | 21-225_74H7 | VK3\|A27/ JK1 | .L..........<br>........... | ......NIY..<br>........ | .........<br>....... | ........<br>....... | .................<br>..............C. | ...E..............<br>............... | ......<br>.... |
| iPS:43 4899 | 21-225_85B9 | VK3\|A27/ JK1 | ...........F<br>........... | ........N..<br>....N... | ..R......<br>....... | ........<br>....... | .................<br>I.I.............. | ...E..............<br>............... | ......<br>.... |
| iPS:43 4907 | 21-225_85G10 | VK3\|A27/ JK1 | .........S..<br>........... | ........W..<br>....G... | .........<br>....... | ........<br>....... | .................<br>..............F. | ...E..............<br>............... | ......<br>.... |
| iPS:43 4913 | 21-225_86C1 | VK3\|A27/ JK1 | ...........Y<br>........... | ........Y..<br>........ | ......A.<br>....... | ........<br>....... | .................<br>A.........C..... | .H.DN.............<br>............... | ......<br>.... |
| iPS:43 4921 | 21-225_86E4 | VK3\|A27/ JK1 | ..........RY<br>........... | ........Y..<br>........ | .........<br>....... | ........<br>.....S. | .................<br>A.........C..... | .HSDN.............<br>............... | ......<br>.... |
| iPS:43 4939 | 21-225_86C11 | VK3\|A27/ JK1 | ...........Y<br>........... | ........Y..<br>........ | .........<br>....... | ........<br>.....S. | .L...............<br>A.........C..... | .HSDN.............<br>............... | ......<br>.... |
| iPS:43 4943 | 21-225_87H1 | VK3\|A27/ JK1 | ............<br>........... | ........D..<br>....N... | .........<br>....... | ........<br>...A.T. | .................<br>..............S. | ...E..............<br>............... | ......<br>.... |
| iPS:43 4945 | 21-225_87E5 | VK3\|A27/ JK1 | .F.........Y<br>........... | ........Y..<br>........ | .........<br>....... | ........<br>.....S. | .................<br>A....V....C..... | .HSDN.............<br>............... | ......<br>.... |
| iPS:43 4955 | 21-225_87C9 | VK3\|A27/ JK1 | ...........Y<br>...V....... | ........Y..<br>........ | .........<br>....... | ........<br>.....S. | .................<br>A.........C..... | .HSDN.............<br>............... | ......<br>.... |
| iPS:43 4961 | 21-225_87A12 | VK3\|A27/ JK1 | ..........RY<br>........... | ........Y..<br>........ | .........<br>....... | ........<br>.....S. | V.........C......<br>A....V....C..... | .HSDN.............<br>............... | ......<br>.... |
| iPS:43 4969 | 21-225_88H1 | VK3\|A27/ JK1 | ...........Y<br>........... | ........Y..<br>........ | .........<br>....... | ........<br>.....S. | V.........C......<br>A....V....C..... | .HSDN.............<br>............... | ......<br>.... |
| iPS:43 4981 | 21-225_88E7 | VK3\|A27/ JK1 | ............<br>........... | ........Y..<br>........ | .........<br>....... | ........<br>.....S. | .................<br>A....V....C..... | .HSDN.............<br>............... | ......<br>.... |
| iPS:43 4983 | 21-225_88F7 | VK3\|A27/ JK1 | ............<br>........... | ........Y..<br>........ | .........<br>....... | ........<br>.....S. | .................<br>A.........C..... | .HSDN.............<br>............... | ......<br>.... |
| iPS:43 4995 | 21-225_88G9 | VK3\|A27/ JK1 | ..........RY<br>........... | ........Y..<br>........ | .........<br>....... | ........<br>.....S. | .................<br>A....V....C..... | .HSDN.............<br>............... | ......<br>.... |
| iPS:43 4999 | 21-225_75A8 | VK3\|A27/ JK1 | ...........Y<br>........... | ........Y..<br>........ | .........<br>....... | ........<br>....... | .................<br>A....V....C..... | .HSDN.............<br>............... | ......<br>.... |
| iPS:43 5013 | 21-225_89D5 | VK3\|A27/ JK1 | ...........Y<br>..S........ | ........Y..<br>........ | .........<br>....... | ........<br>.....S. | .................<br>A................ | .H.DN.............<br>............... | ......<br>.... |

| | | | | | | | | | |
|---|---|---|---|---|---|---|---|---|---|
| iPS:43 5015 | 21-225_89H5 | VK3\|A27/ JK1 | ............<br>........... | ........N..<br>....N... | .........<br>....... | ........<br>..F.... | .....V...........<br>N.I.............. | ...E..............<br>...........V.. | ......<br>.... |
| iPS:43 5025 | 21-225_89E10 | VK3\|A27/ JK1 | ...........Y<br>..S........ | ........Y..<br>........ | .........<br>....... | ........<br>.....S. | V................<br>A.....V.......... | .HSDN.............<br>.............. | ......<br>.... |
| iPS:43 5029 | 21-225_89A11 | VK3\|A27/ JK1 | ............<br>........... | ........D..<br>....NF.. | .........<br>....... | ........<br>...A.T. | .................<br>...............S. | ...EI.............<br>.............. | ......<br>.... |
| iPS:43 5039 | 21-225_90G4 | VK3\|A27/ JK1 | ...........Y<br>........... | ........Y..<br>........ | .........<br>....... | ........<br>.....S. | V................<br>A.....V....C..... | .HSDN.............<br>.............. | ......<br>.... |
| iPS:43 5041 | 21-225_90A5 | VK3\|A27/ JK1 | ...........Y<br>........... | ........Y..<br>........ | .........<br>....... | ........<br>....... | .................<br>A.....V.H........ | .H.DN.............<br>.............. | ......<br>.... |
| iPS:43 5043 | 21-225_90G5 | VK3\|A27/ JK1 | ...........Y<br>........... | ........Y..<br>........ | .........<br>....I.. | ........<br>....... | .................<br>A.....V.H........ | .H.DN.............<br>.............. | ......<br>.... |
| iPS:43 5055 | 21-225_90F10 | VK3\|A27/ JK1 | ...........Y<br>........... | ........Y..<br>........ | .........<br>....... | ........<br>.....S. | .................<br>A..........C..... | .H.DN.............<br>.............. | ......<br>.... |
| iPS:43 5073 | 21-225_91B2 | VK3\|A27/ JK1 | ...........Y<br>........... | ........Y..<br>........ | .........<br>....... | ........<br>.....S. | .................<br>A.....V....C..... | .H.DN.............<br>.............. | ......<br>.... |
| iPS:43 5075 | 21-225_91B3 | VK3\|A27/ JK1 | ...........Y<br>........... | ........Y..<br>........ | .........<br>....... | ........<br>.....S. | .................<br>A.....V.H..C..... | .H.DN.............<br>.............. | ......<br>.... |
| iPS:43 5077 | 21-225_91F3 | VK3\|A27/ JK1 | ...........Y<br>........... | ........Y..<br>........ | ......A..<br>....... | ........<br>.....S. | .................<br>A.....V....C..... | .HSDN.............<br>.............. | ......<br>.... |
| iPS:43 5079 | 21-225_91B4 | VK3\|A27/ JK1 | ...........Y<br>........... | ........Y..<br>........ | .........<br>....I.. | ........<br>.....S. | .................<br>A.....V....C..... | .H.DN.............<br>.............. | ......<br>.... |
| iPS:43 5089 | 21-225_91E9 | VK3\|A27/ JK1 | ...........Y<br>........... | ........Y..<br>........ | .........<br>....... | ........<br>....... | ..........C......<br>A.....V.H..C..... | .HSDN.............<br>.............. | ......<br>.... |
| iPS:43 5097 | 21-225_92B1 | VK3\|A27/ JK1 | ............<br>........... | ........G..<br>....N... | .....R...<br>....... | ........<br>....... | D................<br>................. | ...E..............<br>.............. | ......<br>.... |
| iPS:43 5111 | 21-225_92D6 | VK3\|A27/ JK1 | ...........Y<br>........... | ........Y..<br>........ | .........<br>....... | ........<br>.....S. | V.........C......<br>A..........C..... | .H.DN.............<br>.............. | ......<br>.... |
| iPS:43 5115 | 21-225_77C5 | VK3\|A27/ JK1 | ............<br>........... | ........Y..<br>........ | .........<br>....... | ........<br>.....S. | .................<br>A................ | .H.DN.............<br>.............. | ......<br>.... |
| iPS:43 5171 | 21-225_93C2 | VK3\|A27/ JK1 | ...........Y<br>........... | ........Y..<br>........ | .........<br>....... | ........<br>....... | .................<br>A.......H..C..... | .H.DN.............<br>.............. | ......<br>.... |
| iPS:43 5177 | 21-225_93E4 | VK3\|A27/ JK1 | ...........Y<br>........... | ........Y..<br>........ | .........<br>....... | ........<br>.....S. | ..........C......<br>A.....V.......... | .HSDN.............<br>.............. | ......<br>.... |
| iPS:43 5183 | 21-225_93E9 | VK3\|A27/ JK1 | ............<br>........... | ........D..<br>........ | .....T...<br>....... | ........<br>......P | .................<br>................. | ...E..............<br>.............. | ......<br>.... |
| iPS:43 5195 | 21-225_94D3 | VK3\|A27/ JK1 | ............<br>..Q........ | ...........<br>....R... | .........<br>....... | ........<br>....... | .................<br>................. | .H.D..............<br>.............. | ......<br>..NQ |
| iPS:43 5217 | 21-225_94F12 | VK3\|A27/ JK1 | ...........Y<br>........F.. | ........Y..<br>........ | .........<br>....... | ........<br>.....S. | .................<br>A................ | .H.DN.............<br>.............. | ......<br>.... |
| iPS:43 5219 | 21-225_95D2 | VK3\|A27/ JK1 | ............<br>........... | ........Y..<br>........ | .........<br>....... | ........<br>....... | .................<br>A..........C..... | .H.DN.............<br>.............. | ......<br>.... |

| | | | | | | | | | |
|---|---|---|---|---|---|---|---|---|---|
| iPS:43 5235 | 21-225_95F9 | VK3\|A27/ JK1 | ...........Y<br>........... | ........Y..<br>........ | ........<br>....... | ........<br>.....S. | ..........C......<br>A................ | .H.DN.............<br>.............. | ......<br>.... |
| iPS:43 5237 | 21-225_95G9 | VK3\|A27/ JK1 | ...........Y<br>..S........ | ........Y..<br>........ | ........<br>....... | ........<br>.....S. | .................<br>A.....V.H..C..... | .H.DN.............<br>.............. | ......<br>.... |
| iPS:43 5239 | 21-225_95H10 | VK3\|A27/ JK1 | ....S....I.Y<br>..S........ | ........Y..<br>........ | ........<br>....... | ........<br>....... | .................<br>A................ | .H.DN.............<br>.............. | ......<br>.... |
| iPS:43 5273 | 21-225_97A2 | VK3\|A27/ JK1 | ...........Y<br>..S........ | ........Y..<br>........ | ........<br>....... | ........<br>.....S. | ..........C......<br>A..........C..... | .HSDN.............<br>.............. | ......<br>.... |
| iPS:43 5281 | 21-225_97E5 | VK3\|A27/ JK1 | ............<br>........... | ........Y..<br>........ | ........<br>....... | ........<br>....... | ..........C......<br>A.....V....C..... | .HSDN.............<br>.............. | ......<br>.... |
| iPS:43 5331 | 21-225_147G8 | VK3\|A27/ JK1 | Q...........<br>........... | ......RIF..<br>....N... | ........<br>...I... | ........<br>....... | .....I...........<br>....T............ | ...D..............<br>.............. | ......<br>...N |
| iPS:43 5815 | 21-225_190G10 | VK3\|A27/ JK1 | ............<br>........... | .....P.....<br>....RF.. | ........<br>....... | ........<br>....... | .................<br>...N............. | ..................<br>.........SP... | ......<br>.... |
| iPS:43 5843 | 21-225_191F1 | VK3\|A27/ JK1 | ............<br>.......A... | .......I.L.<br>....NF.. | ........<br>....... | ........<br>....... | .................<br>....N............ | ....R.............<br>.............. | ......<br>..V. |
| iPS:43 5847 | 21-225_191A3 | VK3\|A27/ JK1 | ............<br>........... | .......IR..<br>.....F.. | ........<br>....... | ........<br>....... | .................<br>................. | ....N.............<br>.............A | ......<br>..V. |
| iPS:43 5849 | 21-225_191C3 | VK3\|A27/ JK1 | ............<br>........... | .......IR..<br>.....F.. | ........<br>....... | ........<br>....... | .................<br>................. | ....N.............<br>.............A | ......<br>.... |
| iPS:43 5851 | 21-225_191D3 | VK3\|A27/ JK1 | ............<br>.....S..... | ..G....IRT.<br>....NF.. | ....Q...<br>....... | ........<br>....... | .................<br>....N............ | ..................<br>.............. | ......<br>.... |
| iPS:43 5865 | 21-225_191A5 | VK3\|A27/ JK1 | ............<br>........... | ...........<br>....RF.. | ........<br>....... | ........<br>...N... | .................<br>................. | ....G.............<br>.........SP... | .V....<br>.... |
| iPS:43 5905 | 21-225_190A3 | VK3\|A27/ JK1 | ..M.........<br>........... | ......NIR..<br>....NF.. | ........<br>....... | ........<br>....... | .........V.......<br>....N............ | ....N.............<br>.............. | ......<br>.... |
| iPS:43 5911 | 21-225_190B4 | VK3\|A27/ JK1 | ............<br>........... | .......IR..<br>.....F.. | ........<br>....... | ........<br>....... | .................<br>................. | ....N.............<br>.............A | ......<br>.... |
| iPS:43 5913 | 21-225_190A7 | VK3\|A27/ JK1 | ............<br>........... | ........R..<br>....NF.. | .H......<br>....... | ........<br>..YR... | .................<br>....N............ | ....N.............<br>.............. | ......<br>.... |
| iPS:43 5939 | 21-225_191H7 | VK3\|A27/ JK1 | ............<br>.....S..... | ..G....IRT.<br>....DF.. | ....Q...<br>....... | ........<br>.P..... | .................<br>....N..........F. | ..................<br>.............. | ......<br>...N |
| iPS:43 5967 | 21-225_192B3 | VK3\|A27/ JK1 | ............<br>........... | ........R..<br>.....F.. | ........<br>....... | ........<br>....... | .................<br>................. | ....N.............<br>.............A | ......<br>.... |
| iPS:43 5973 | 21-225_192H3 | VK3\|A27/ JK1 | ............<br>........... | ...........<br>....NF.. | ........<br>......F | ........<br>....... | .................<br>....N............ | ....I.............<br>.............. | ......<br>.... |
| iPS:43 5995 | 21-225_192F8 | VK3\|A27/ JK1 | ............<br>........... | .......I...<br>........ | ........<br>....... | ........<br>....... | ...A.............<br>................. | ...E..............<br>.............. | ......<br>.... |
| iPS:43 6007 | 21-225_192G12 | VK3\|A27/ JK1 | ............<br>........... | ........R..<br>....DF.. | .L......<br>....... | ........<br>.V.R... | .................<br>....N............ | ....N.............<br>.............. | ......<br>.... |
| iPS:43 6009 | 21-225_193A1 | VK3\|A27/ JK1 | ............<br>........... | ........R..<br>....NF.. | .H......<br>....F.. | ........<br>...R... | .................<br>....N............ | ....N.............<br>.............. | ......<br>.... |

| | | | | | | | | | |
|---|---|---|---|---|---|---|---|---|---|
| iPS:43 6011 | 21-225_193B1 | VK3\|A27/JK1 | ............<br>....D...... | ........R..<br>....NF.. | .H......<br>....... | ........<br>...R... | .................<br>....N..........F. | ....N.............<br>.............. | ......<br>.... |
| iPS:43 6015 | 21-225_193D3 | VK3\|A27/JK1 | ............<br>........... | .......IR..<br>.....F.. | ........<br>....... | ........<br>...N... | .................<br>................. | ....N.............<br>.............A | ......<br>.... |
| iPS:43 6017 | 21-225_193F3 | VK3\|A27/JK1 | ............<br>.....S..... | ..G....IRT.<br>....DF.V | ....Q...<br>....... | ........<br>....... | .F.E.............<br>....N............ | ..................<br>.............. | ......<br>...N |
| iPS:43 6027 | 21-225_193E6 | VK3\|A27/JK1 | ....A.......<br>........... | ........R..<br>....G... | ........<br>....... | ........<br>....... | ......G..........<br>................. | ...E..............<br>.............. | ......<br>.... |
| iPS:43 6029 | 21-225_193H6 | VK3\|A27/JK1 | ............<br>.....S..... | ..G....IRT.<br>....NF.. | ....Q...<br>....... | ........<br>....... | .................<br>....N..........F. | ..................<br>.............. | ......<br>...N |
| iPS:43 6035 | 21-225_193C8 | VK3\|A27/JK1 | ...........F<br>........... | .......IR..<br>.....F.. | ........<br>....... | ........<br>....... | .................<br>................. | ....N.............<br>.............A | ....I.<br>..V. |
| iPS:43 6037 | 21-225_193D8 | VK3\|A27/JK1 | ....K......F<br>........... | .......IRT.<br>....NF.. | ........<br>....... | ........<br>....... | .................<br>....N............ | ....N.............<br>.............. | ......<br>.... |
| iPS:43 6041 | 21-225_193G8 | VK3\|A27/JK1 | ............<br>........... | ........RT.<br>....NF.. | .H......<br>....... | ........<br>...R... | .................<br>....N........L... | ....N.............<br>.............. | ......<br>.... |
| iPS:43 6047 | 21-225_193B10 | VK3\|A27/JK1 | ............<br>........... | .....P.....<br>........ | ........<br>....V.. | ........<br>...R... | ......R..........<br>................. | ..................<br>.........SP... | ......<br>.... |
| iPS:43 6049 | 21-225_193B12 | VK3\|A27/JK1 | ............<br>........... | .......IR..<br>.....F.. | ........<br>....... | D.......<br>....... | .................<br>................. | ....N.............<br>.............A | ......<br>.... |
| iPS:43 6062 | 21-225_194E5 | VK3\|A27/JK1 | ............<br>.....S..... | ..G....IRT.<br>....NF.. | ........<br>....... | ........<br>....... | .................<br>....N....K....... | ..................<br>.............. | ......<br>.... |
| iPS:43 6064 | 21-225_194E6 | VK3\|A27/JK1 | ............<br>........... | .......IR..<br>....NF.. | ........<br>....... | ........<br>....... | .........V.......<br>....N............ | ..................<br>.............. | ......<br>.... |
| iPS:43 6072 | 21-225_194C10 | VK3\|A27/JK1 | ............<br>........... | .....P..N..<br>....G... | ........<br>T.....F | ........<br>....... | .......A......A..<br>...............F. | ...E..............<br>.............. | ......<br>.... |
| iPS:43 6080 | 21-225_195B1 | VK3\|A27/JK1 | ............<br>..S........ | .....P..N..<br>....N... | ........<br>T...... | ........<br>...N... | .V.....A.........<br>....R..........F. | ...E..............<br>.............. | ......<br>.... |
| iPS:43 6088 | 21-225_195C8 | VK3\|A27/JK1 | ............<br>........... | .......IR..<br>.....F.. | ........<br>....... | ........<br>..F.... | .................<br>................. | ....N.............<br>.............A | ......<br>.... |
| iPS:43 6122 | 21-225_196G10 | VK3\|A27/JK1 | ...........L<br>........... | .....P...N.<br>.....F.. | ........<br>....... | ........<br>....... | .................<br>...N............. | ..................<br>.........SP... | ......<br>..L. |
| iPS:43 6134 | 21-225_196H12 | VK3\|A27/JK1 | ...........F<br>........... | ........R..<br>....NF.. | .H......<br>....... | ........<br>..YR... | .................<br>....N............ | ....N.............<br>.............. | ......<br>.... |
| iPS:43 6146 | 21-225_197F4 | VK3\|A27/JK1 | ............<br>.....G..... | .......IR..<br>.....F.. | ..L.....<br>....... | ........<br>....... | .................<br>................. | ....N.............<br>.............A | ......<br>.... |
| iPS:43 6177 | 21-225_198B1 | VK3\|A27/JK1 | ............<br>.....S..... | ..G....IRT.<br>....NF.. | ....Q...<br>....... | ........<br>....... | .................<br>....N............ | ..................<br>.............. | ......<br>.... |
| iPS:43 6179 | 21-225_198E1 | VK3\|A27/JK1 | ............<br>........... | .......IR..<br>....NF.. | ........<br>....... | ........<br>..F.... | .................<br>....N............ | ....N.............<br>.............. | ......<br>.... |
| iPS:43 6181 | 21-225_198C2 | VK3\|A27/JK1 | ...V.......L<br>.YSE..S.... | ........R..<br>........ | ........<br>......C | ........<br>..F...S | .................<br>................. | ....N.............<br>.............. | L.HA..<br>.... |

| | | | | | | | | | |
|---|---|---|---|---|---|---|---|---|---|
| iPS:43 6195 | 21-225_198G10 | VK3\|A27/ JK1 | ............<br>........... | .......IR..<br>.....F.. | ........<br>....... | ........<br>....... | .................<br>................. | ....N..............<br>..............A | ......<br>.... |
| iPS:43 6197 | 21-225_199C2 | VK3\|A27/ JK1 | ............<br>........... | .......IR..<br>.....F.. | ........<br>....... | ........<br>....... | .................<br>...N............. | ....N..............<br>..............A | ......<br>.... |
| iPS:43 6207 | 21-225_199C7 | VK3\|A27/ JK1 | ............<br>........... | .......IRT.<br>....NF.. | ........<br>....... | ........<br>....... | .................<br>...N............. | ....N..............<br>............... | ......<br>.... |
| iPS:43 6210 | 21-225_199G11 | VK3\|A27/ JK1 | ...V........<br>........... | ........R..<br>........ | ........<br>....... | ........<br>..F.... | .................<br>................. | ....N..............<br>............... | ..H...<br>.... |
| iPS:43 6226 | 21-225_200F10 | VK3\|A27/ JK1 | ............<br>........... | ......NIR..<br>.....F.. | ........<br>....... | ........<br>....... | .................<br>...N............. | ....N..............<br>..............A | ......<br>.... |
| iPS:43 6232 | 21-225_201E1 | VK3\|A27/ JK1 | ........D...<br>........... | .....P.IN..<br>....GF.. | ........<br>....... | ........<br>....... | .................<br>..............MFH. | H..ET..............<br>............... | ......<br>.... |
| iPS:43 6238 | 21-225_201B2 | VK3\|A27/ JK1 | ............<br>........... | ...........<br>....N... | ....R...<br>....... | ........<br>....... | .................<br>...............F. | ...EN..............<br>............... | ......<br>.... |
| iPS:43 6256 | 21-225_202D9 | VK3\|A27/ JK1 | ........D...<br>........... | ........N..<br>....G... | ........<br>S...... | ........<br>....... | .................<br>..............H.. | ...ET..............<br>............... | ......<br>.... |
| iPS:43 6302 | 21-225_205G7 | VK3\|A27/ JK1 | ............<br>........... | ........F..<br>....N... | ........<br>....... | ........<br>......A | .................<br>..........N...... | ...E...............<br>............... | ......<br>.... |
| iPS:43 6310 | 21-225_202D11 | VK3\|A27/ JK1 | ............<br>........... | .......IN..<br>....N... | ...R....<br>...V... | ........<br>....... | .................<br>..............F.. | ...EN..............<br>............... | ......<br>.... |
| iPS:43 6336 | 21-225_208B5 | VK3\|A27/ JK1 | ............<br>........... | ...........<br>....N... | ....R...<br>....... | ........<br>....... | .................<br>...............F. | .H.EN..............<br>............... | ......<br>.... |
| iPS:43 6340 | 21-225_208A9 | VK3\|A27/ JK1 | ............<br>........... | .........N.<br>....N... | ........<br>...V... | ........<br>....... | .................<br>................. | .H.H...............<br>............... | ......<br>.... |
| iPS:43 6472 | 21-225_220E1 | VK3\|A27/ JK1 | ............<br>........... | .......I.R.<br>.....H.V | ......N.<br>.....L. | V.......<br>.T..... | .................<br>....RS.......M... | ...................<br>............... | ......<br>.... |
| iPS:43 6506 | 21-225_222C7 | VK3\|A27/ JK1 | ............<br>........... | ........Y..<br>....N... | ........<br>....... | ........<br>....... | ......G..........<br>..A.........TI... | ...ED..............<br>............... | ......<br>.... |
| iPS:43 6580 | 21-225_225E7 | VK3\|A27/ JK1 | ............<br>........... | ........Y..<br>........ | ........<br>....... | ........<br>....... | .................<br>................. | ....T..............<br>............R. | ......<br>.... |
| iPS:43 7324 | 21-225_75C2 | VK3\|A27/ JK1 | ..........RY<br>........... | ........Y..<br>........ | ........<br>....... | ........<br>....... | .................<br>A.........C...... | .H.DN..............<br>............... | ..R...<br>.... |
| iPS:43 7328 | 21-225_75D3 | VK3\|A27/ JK1 | ...........Y<br>........... | ........Y..<br>........ | ........<br>....I.. | ........<br>.....S. | ..........C......<br>A.....V.H..C..... | .H.DN..............<br>............... | ......<br>.... |
| iPS:43 7332 | 21-225_75F3 | VK3\|A27/ JK1 | ...........Y<br>........... | ........Y..<br>........ | ........<br>....... | ........<br>....... | ..........C......<br>A.......H........ | .H.DN..............<br>............... | ......<br>.... |
| iPS:43 7340 | 21-225_75G9 | VK3\|A27/ JK1 | ..A.........<br>........... | .....P..D..<br>........ | ........<br>....... | ........<br>......P | .................<br>................. | ...E...............<br>............... | ......<br>.... |
| iPS:43 7344 | 21-225_75G12 | VK3\|A27/ JK1 | ...........Y<br>........... | ........Y..<br>........ | ........<br>....... | ........<br>....... | .................<br>A.....V....C..... | .H.DN..............<br>............... | ......<br>.... |
| iPS:43 7350 | 21-225_74A3 | VK3\|A27/ JK1 | ............<br>........... | ........Y..<br>........ | ........<br>....... | ........<br>.....S. | ..........C......<br>A................ | .HSDN..............<br>............... | ......<br>.... |

| | | | | | | | | | |
|---|---|---|---|---|---|---|---|---|---|
| iPS:43 7369 | 21-225_74D6 | VK3\|A27/ JK1 | ............<br>........... | .........Y..<br>........ | ........<br>....... | ........<br>....... | ...........C......<br>A................. | .H.DN.............<br>.............. | ......<br>.... |
| iPS:43 7383 | 21-225_74H8 | VK3\|A27/ JK1 | ............<br>........... | .......F...<br>........ | ........<br>....... | ........<br>....... | ..................<br>................. | ....-<br>.................<br>......SR. | ......<br>.... |
| iPS:45 1122 | 21-225_200A1 | VK3\|A27/ JK1 | .........I..<br>.Y......... | ........N..<br>....N... | ....R...<br>..S.... | ........<br>....... | C.L.......C......<br>...............C. | ...EI............<br>.............. | ......<br>..S. |
| iPS:39 2864 | 21-225_23B9 | VK3\|A27/ JK1 | ............<br>........... | ......N.Y..<br>........ | ........<br>T...... | ........<br>......S | ..................<br>................. | .................<br>............R. | ......<br>.... |
| | | Germline | **K_FR1** | **K_CDR1** | **K_FR2** | **K_CDR2** | **K_FR3** | **K_CDR3** | **K_FR4** |
| | VK1\|A30/JK 4 | | DIQMTQSPSSLS<br>ASVGDRVTITC | RAS--QGIR--<br>----NDLG | WYQQKPGK<br>APKRLIY | A-------<br>-ASSLQS | GVPSRFSGSGSG--<br>TEFTLTISSLQPEDFAT<br>YYC | LQHNS------------<br>----------YPLT | FGGGTK<br>VEIK |
| iPS:46 8812 | 21-225_48H4 | VK1\|A30/ JK4 | ......F.....<br>........... | .....RD....<br>........ | ........<br>....... | ........<br>....... | ..................<br>................. | ...Y.............<br>.............. | ......<br>.... |
| iPS:46 8824 | 21-225_73G6 | VK1\|A30/ JK4 | ............<br>........... | ...........<br>........ | ........<br>....... | ........<br>....... | ..................<br>................. | .................<br>.............. | ......<br>.... |
| iPS:46 8818 | 21-225_190C8 | VK1\|A30/ JK4 | ............<br>........... | ...........<br>........ | ........<br>....... | ........<br>....... | ..................<br>.............E.... | ....D............<br>............F. | ......<br>.... |
| iPS:46 8840 | 21-225_200H9 | VK1\|A30/ JK4 | ............<br>........... | ...........<br>........ | ........<br>....... | ........<br>....... | .I................<br>................. | .................<br>.............. | ......<br>.... |
| iPS:46 8868 | 21-225_74A1 | VK1\|A30/ JK4 | ............<br>........... | ...........<br>........ | ........<br>....... | ........<br>....... | ..................<br>................. | ...D.............<br>.............. | ....A.<br>.... |
| iPS:39 2920 | 21-225_29G4 | VK1\|A30/ JK4 | ............<br>........... | ...........<br>........ | ........<br>....... | ........<br>....... | ..................<br>................. | .................<br>.............. | ......<br>...T |
| iPS:43 3899 | 21-225_43C3 | VK1\|A30/ JK4 | ............<br>........... | ...........<br>........ | ........<br>....... | ........<br>....... | ..................<br>................. | .................<br>.............. | ......<br>.G.T |
| iPS:43 3921 | 21-225_44C3 | VK1\|A30/ JK4 | ............<br>........... | ...........<br>........ | ........<br>....... | ........<br>....... | ........G.........<br>................. | ...S.............<br>.............. | ......<br>.... |
| iPS:43 3947 | 21-225_44E12 | VK1\|A30/ JK4 | ............<br>........... | .T.........<br>........ | ........<br>....... | ........<br>....... | ..................<br>................. | .................<br>.............. | ......<br>.... |
| iPS:43 3963 | 21-225_46B1 | VK1\|A30/ JK4 | ............<br>........... | ...........<br>........ | ........<br>....... | ........<br>......G | ..................<br>................. | .................<br>.............. | ......<br>.... |
| iPS:43 3969 | 21-225_46F3 | VK1\|A30/ JK4 | ............<br>........... | ...........<br>....K... | ........<br>....... | ........<br>....... | ..................<br>S................ | .................<br>.............. | ......<br>.... |
| iPS:43 3975 | 21-225_46C6 | VK1\|A30/ JK4 | ............<br>........... | ...........<br>........ | ........<br>....... | ........<br>....... | ..................<br>................. | .................<br>.............. | ......<br>...T |
| iPS:43 3977 | 21-225_46D8 | VK1\|A30/ JK4 | ............<br>........... | ...........<br>....K... | ........<br>....... | ........<br>....... | ..................<br>S................ | .................<br>.............. | ......<br>.... |
| iPS:43 3983 | 21-225_47A1 | VK1\|A30/ JK4 | ............<br>........... | ......D....<br>........ | ........<br>....... | ........<br>..F.... | .........R........<br>................. | .................<br>.............. | ......<br>.... |
| iPS:43 | 21-225_47A5 | VK1\|A30/ | ............ | ........... | ........ | ........ | .................. | ................. | ...... |

| | | | | | | | | | |
|---|---|---|---|---|---|---|---|---|---|
| iPS:43 4013 | 21-225_48D12 | VK1\|A30/ JK4 | ............<br>........... | ...........<br>........ | ........<br>....... | ........<br>...T... | .....I...........<br>................. | ..................<br>.............. | ......<br>.... |
| iPS:43 4019 | 21-225_49A1 | VK1\|A30/ JK4 | ............<br>........... | ...........<br>.......D | ........<br>....... | ........<br>....... | .................<br>................. | ..................<br>.............. | ......<br>.... |
| iPS:43 4029 | 21-225_49C6 | VK1\|A30/ JK4 | ............<br>........... | ...........<br>........ | ........<br>....... | ........<br>....... | .F...............<br>................. | ..................<br>.............. | ......<br>.... |
| iPS:43 4043 | 21-225_50G10 | VK1\|A30/ JK4 | ............<br>........... | ...........<br>.....N.. | ........<br>V...... | ........<br>....... | .................<br>................. | ..Y...............<br>............F. | ......<br>..S. |
| iPS:43 4077 | 21-225_51F11 | VK1\|A30/ JK4 | .........A..<br>........... | ...........<br>........ | ........<br>....... | ........<br>....... | .................<br>................. | ..................<br>............F. | ......<br>.... |
| iPS:43 4081 | 21-225_52B2 | VK1\|A30/ JK4 | ............<br>........... | ...........<br>........ | ........<br>..R.... | ........<br>...F... | ....T............<br>................. | ..................<br>.............. | ......<br>.... |
| iPS:43 4105 | 21-225_53D2 | VK1\|A30/ JK4 | ............<br>........... | ...........<br>........ | ........<br>....... | ........<br>....... | .................<br>................. | ....R.............<br>.............. | ......<br>.... |
| iPS:43 4119 | 21-225_53E6 | VK1\|A30/ JK4 | ............<br>....A...... | ...........<br>........ | ....N...<br>....... | ........<br>...N... | .................<br>...............F. | ....R.............<br>.............. | ......<br>.... |
| iPS:43 4141 | 21-225_54C6 | VK1\|A30/ JK4 | ............<br>....A...... | ...........<br>........ | ....N...<br>....... | ........<br>...N... | .................<br>...............F. | ....R.............<br>.............. | ......<br>.... |
| iPS:43 4159 | 21-225_55B8 | VK1\|A30/ JK4 | ............<br>S.......... | ......A....<br>........ | ........<br>......H | ........<br>..FR... | .................<br>................. | ..................<br>.............. | ......<br>.G.. |
| iPS:43 4179 | 21-225_56F1 | VK1\|A30/ JK4 | ............<br>........... | ......D....<br>.....N.. | ........<br>......N | ........<br>....... | .................<br>................. | ..D...............<br>..........H.F. | ......<br>.... |
| iPS:43 4217 | 21-225_60E8 | VK1\|A30/ JK4 | ............<br>........F.. | ...........<br>........ | .F......<br>....... | ........<br>....... | .................<br>................. | ...S..............<br>.............. | ......<br>.... |
| iPS:43 4249 | 21-225_62E2 | VK1\|A30/ JK4 | ............<br>........... | ...........<br>........ | ........<br>....... | ........<br>...R... | .................<br>................. | ...SN.............<br>.............. | .....R<br>.... |
| iPS:43 4253 | 21-225_62E4 | VK1\|A30/ JK4 | ............<br>........... | ...........<br>........ | ........<br>....... | ........<br>..F.... | .................<br>................. | ..................<br>.............. | ......<br>.... |
| iPS:43 4313 | 21-225_59E6 | VK1\|A30/ JK4 | ............<br>........... | ...........<br>........ | ........<br>....... | ........<br>....... | .................<br>................. | ..................<br>.............. | .....R<br>.... |
| iPS:43 4337 | 21-225_64E1 | VK1\|A30/ JK4 | ............<br>........... | ...........<br>........ | ........<br>....... | ........<br>....... | .........K.......<br>...........L..... | ..................<br>.............. | ......<br>.... |
| iPS:43 4411 | 21-225_68F11 | VK1\|A30/ JK4 | ............<br>........... | ...........<br>........ | ........<br>....... | ........<br>....... | ..............P..<br>..S.............. | ..................<br>............F. | ......<br>.... |
| iPS:43 4413 | 21-225_68D12 | VK1\|A30/ JK4 | ............<br>........... | ...........<br>........ | ........<br>....... | ........<br>....... | .................<br>................. | ...ST.............<br>.............. | ......<br>.... |
| iPS:43 4433 | 21-225_70E8 | VK1\|A30/ JK4 | ............<br>T.......... | ...........<br>....K... | ........<br>....... | ........<br>....... | .................<br>................. | ....R.............<br>.............. | ......<br>.... |
| iPS:43 4439 | 21-225_70E12 | VK1\|A30/ JK4 | ............<br>........... | ........N..<br>.....N.N | .F......<br>....... | ........<br>....... | .................<br>................. | ..................<br>.............. | ....S.<br>.... |
| iPS:43 4489 | 21-225_74E4 | VK1\|A30/ JK4 | ............<br>........... | ...........<br>........ | ........<br>....... | ........<br>...T... | .................<br>.....G.........H. | ...SN.............<br>.............. | ......<br>.... |

| | | | | | | | | | |
|---|---|---|---|---|---|---|---|---|---|
| iPS:43 4503 | 21-225_74D7 | VK1\|A30/ JK4 | ............<br>........... | ...........<br>........ | ........<br>....... | ........<br>....... | ..........F......<br>................. | ...SN.............<br>.............. | ......<br>.... |
| iPS:43 5251 | 21-225_96A3 | VK1\|A30/ JK4 | ............<br>........... | ...........<br>........ | ........<br>....... | ........<br>...T... | .................<br>.....G..R......H. | ...SN.............<br>.............. | ......<br>.... |
| iPS:43 5293 | 21-225_146F1 | VK1\|A30/ JK4 | ............<br>........... | ...........<br>........ | ........<br>....... | ........<br>....... | ........G........<br>................. | ...ST.............<br>.............. | ......<br>.... |
| iPS:43 5311 | 21-225_146H9 | VK1\|A30/ JK4 | ............<br>........... | ...........<br>........ | ........<br>....... | ........<br>....... | .................<br>................. | ..................<br>.............. | ......<br>.... |
| iPS:43 5313 | 21-225_146G11 | VK1\|A30/ JK4 | ............<br>........... | ......D....<br>.....NF. | ........<br>....... | ........<br>....... | ...L.............<br>................. | ...D..............<br>.............. | ......<br>.... |
| iPS:43 5361 | 21-225_148E11 | VK1\|A30/ JK4 | ............<br>........... | ...........<br>........ | ........<br>......S | ........<br>....... | ......T..........<br>.F....V.......... | ...RN.............<br>.............. | ......<br>.... |
| iPS:43 5363 | 21-225_148F12 | VK1\|A30/ JK4 | ............<br>........... | ...........<br>.....A.. | ........<br>....... | ........<br>....... | .................<br>...............F. | ..................<br>.............I | ......<br>.... |
| iPS:43 5367 | 21-225_149G1 | VK1\|A30/ JK4 | ............<br>....A...... | ...........<br>........ | ........<br>....... | ........<br>...N... | .................<br>................. | ..................<br>.............. | ......<br>.... |
| iPS:43 5377 | 21-225_149G5 | VK1\|A30/ JK4 | ............<br>........... | ...........<br>.....A.. | .F......<br>....... | ........<br>....... | .................<br>................. | ..................<br>.............. | ......<br>.... |
| iPS:43 5397 | 21-225_149F12 | VK1\|A30/ JK4 | ............<br>........... | ...........<br>........ | ........<br>....... | ........<br>...N... | .................<br>.............I... | ..................<br>.............. | ......<br>.... |
| iPS:43 5407 | 21-225_150E7 | VK1\|A30/ JK4 | ............<br>........... | ...........<br>........ | ........<br>....... | ........<br>...N... | ...L...........D.<br>.............A... | ..................<br>.............. | ......<br>.... |
| iPS:43 5449 | 21-225_152H9 | VK1\|A30/ JK4 | ............<br>........... | ...........<br>........ | ........<br>....... | ........<br>....... | ......T..........<br>.F............... | ...SN.............<br>.............. | ......<br>.... |
| iPS:43 5499 | 21-225_156G1 | VK1\|A30/ JK4 | ............<br>........... | ...........<br>........ | ........<br>....... | ........<br>....... | .................<br>.F............... | ...SN.............<br>.............. | ......<br>.... |
| iPS:43 5549 | 21-225_158H5 | VK1\|A30/ JK4 | ....I....F.F<br>........... | .......M...<br>....I... | ........<br>....... | R.......<br>....... | ..........C......<br>......V.R....S... | V.................<br>.............. | ......<br>.... |
| iPS:43 5587 | 21-225_160H3 | VK1\|A30/ JK4 | ............<br>........... | ...........<br>........ | ........<br>....... | ........<br>....... | .................<br>................. | ...SN.............<br>.............. | .....Q<br>..S. |
| iPS:43 5599 | 21-225_160B10 | VK1\|A30/ JK4 | ..........P.<br>........... | ...........<br>........ | .......T<br>....... | ........<br>....... | .................<br>.....N........... | ...S..............<br>.............. | ......<br>.... |
| iPS:43 5663 | 21-225_169B1 | VK1\|A30/ JK4 | ............<br>........... | ...........<br>........ | ........<br>......G | ........<br>.E..... | .................<br>.....G........... | ...Y..............<br>.............. | ......<br>.... |
| iPS:43 5669 | 21-225_169F9 | VK1\|A30/ JK4 | ............<br>........... | ...........<br>........ | ........<br>......F | ........<br>....... | .................<br>................. | ...Y..............<br>.............. | ......<br>.... |
| iPS:43 5693 | 21-225_170G4 | VK1\|A30/ JK4 | ............<br>........... | ...........<br>........ | ..H.....<br>....... | ........<br>...T... | .................<br>................. | ...Y..............<br>.............. | ......<br>.... |
| iPS:43 5695 | 21-225_170D5 | VK1\|A30/ JK4 | ............<br>........... | ...........<br>........ | ...E....<br>...H... | ........<br>......N | .................<br>................. | ...Y..............<br>..........F... | ......<br>.... |
| iPS:43 5697 | 21-225_170G5 | VK1\|A30/ JK4 | ............<br>........... | ...........<br>....T... | .F......<br>....... | T.......<br>....... | .................<br>................. | ...Y..............<br>.............. | ......<br>.... |

| | | | | | | | | | |
|---|---|---|---|---|---|---|---|---|---|
| iPS:43<br>5703 | 21-<br>225_170D11 | VK1\|A30/<br>JK4 | ............<br>........... | ...........<br>........ | ........<br>...H... | ........<br>......N | .................<br>................. | ...Y...............<br>..........F... | ......<br>...R |
| iPS:43<br>5705 | 21-<br>225_171C3 | VK1\|A30/<br>JK4 | ............<br>........... | ...........<br>....T... | .F......<br>....... | T.......<br>....... | .................<br>................. | ...Y...............<br>............... | ......<br>.... |
| iPS:43<br>5709 | 21-<br>225_171A4 | VK1\|A30/<br>JK4 | ............<br>........... | ...........<br>........ | ........<br>....... | T.......<br>....... | .................<br>................. | ...Y...............<br>............... | ......<br>.... |
| iPS:43<br>5721 | 21-<br>225_172B3 | VK1\|A30/<br>JK4 | ............<br>........... | ...........<br>........ | ........<br>......G | ........<br>.E..... | .................<br>....G............ | ...Y...............<br>............... | ......<br>.... |
| iPS:43<br>5725 | 21-<br>225_172G8 | VK1\|A30/<br>JK4 | ............<br>........... | .......V...<br>........ | ........<br>...H... | ........<br>......N | .................<br>................. | .H.Y...............<br>..........F... | ......<br>.... |
| iPS:43<br>5735 | 21-<br>225_173H12 | VK1\|A30/<br>JK4 | ............<br>........... | ...........<br>........ | ........<br>....... | T.......<br>.T..... | .................<br>..............S... | ...Y...............<br>..........F.N. | ......<br>.... |
| iPS:43<br>5743 | 21-<br>225_175G1 | VK1\|A30/<br>JK4 | ............<br>........... | ...........<br>....T... | ........<br>....... | T.......<br>....... | .................<br>................. | ...Y...............<br>............... | ......<br>.... |
| iPS:43<br>5761 | 21-<br>225_176B11 | VK1\|A30/<br>JK4 | ............<br>........... | ...........<br>........ | .F......<br>....... | ........<br>....... | .................<br>...L............. | ...Y...............<br>............... | ......<br>.... |
| iPS:43<br>5779 | 21-<br>225_178B10 | VK1\|A30/<br>JK4 | ............<br>........... | ...........<br>........ | ........<br>...H... | ........<br>......N | .................<br>................. | .H.Y...............<br>..........F... | ......<br>.... |
| iPS:43<br>6023 | 21-<br>225_193A5 | VK1\|A30/<br>JK4 | ..........F<br>.......I.S. | ...........<br>........ | ....Y...<br>....V.. | ........<br>....... | ..........F.....<br>.I....V....E.... | ....D..............<br>..........F.F. | ......<br>.... |
| iPS:43<br>6033 | 21-<br>225_193E7 | VK1\|A30/<br>JK4 | ............<br>........... | ...........<br>........ | ........<br>....... | S.......<br>......R | .................<br>................. | ...KR..............<br>............... | ......<br>.... |
| iPS:43<br>6120 | 21-<br>225_196C10 | VK1\|A30/<br>JK4 | ............<br>........... | ...........<br>........ | ........<br>....... | ........<br>....... | .................<br>................. | ..Y................<br>............... | ......<br>.... |
| iPS:43<br>6199 | 21-<br>225_199E3 | VK1\|A30/<br>JK4 | ............<br>........... | ...........<br>........ | ........<br>......S | S.......<br>......R | .................<br>................. | ...KR..............<br>............... | ......<br>.... |
| iPS:43<br>6228 | 21-<br>225_200F12 | VK1\|A30/<br>JK4 | ............<br>........... | ...........<br>........ | .......R<br>....... | S.......<br>.....HT | .................<br>................. | ...K...............<br>............... | ......<br>.... |
| iPS:43<br>6230 | 21-<br>225_201A1 | VK1\|A30/<br>JK4 | ............<br>........... | ...........<br>........ | ........<br>....... | S.......<br>...I..R | .................<br>................. | ...K...............<br>............... | ......<br>..V. |
| iPS:43<br>6242 | 21-<br>225_201A10 | VK1\|A30/<br>JK4 | ............<br>........... | ...........<br>........ | .......R<br>....... | S.......<br>.T...H. | .................<br>................. | ...................<br>............... | ......<br>.... |
| iPS:43<br>6286 | 21-<br>225_204H8 | VK1\|A30/<br>JK4 | ............<br>........... | ...........<br>........ | ........<br>....... | S.......<br>....... | ........R.......<br>...V............. | ...................<br>............... | ......<br>.... |
| iPS:43<br>6308 | 21-<br>225_205H8 | VK1\|A30/<br>JK4 | ............<br>........... | ...........<br>........ | .....Q..<br>...L... | S.......<br>...F..R | .................<br>...........S.A... | ...................<br>............... | .....T<br>.K.. |
| iPS:43<br>6526 | 21-<br>225_224A1 | VK1\|A30/<br>JK4 | ............<br>........... | ........E..<br>........ | ........<br>....... | ........<br>....... | .................<br>................. | ...................<br>............... | ......<br>.... |
| iPS:43<br>6528 | 21-<br>225_224B1 | VK1\|A30/<br>JK4 | ............<br>........... | ........S..<br>........ | ........<br>....... | ........<br>....... | .................<br>...............H. | ...................<br>..........P. | ......<br>.... |
| iPS:43<br>6538 | 21-<br>225_224C3 | VK1\|A30/<br>JK4 | ............<br>........... | ......D....<br>........ | ........<br>....... | ........<br>.T..... | .................<br>................. | ...................<br>............... | ......<br>.... |

| | | | | | | | | | |
|---|---|---|---|---|---|---|---|---|---|
| iPS:43 6556 | 21-225_224D10 | VK1\|A30/ JK4 | ..L.........<br>........... | ...........<br>........ | ........<br>....... | ........<br>....... | .................<br>................. | ..................<br>.............. | ......<br>.... |
| iPS:43 7220 | 21-225_55H6 | VK1\|A30/ JK4 | ............<br>........... | ...........<br>........ | ........<br>....... | G.......<br>....... | .................<br>.............A... | ..RD..............<br>............F. | ......<br>.... |
| iPS:43 7346 | 21-225_75H7 | VK1\|A30/ JK4 | ..........RY<br>.........NS | ...........<br>........ | ........<br>S.Q.... | D.......<br>....... | .................<br>......V.....GV... | I..SN.............<br>.............. | ......<br>.... |
| iPS:47 2730 | 21-225_14B1_LC1 | VK1\|A30/ JK4 | .........Y..<br>........... | ......D....<br>....DN.. | ........<br>....... | T.......<br>..Y.... | .................<br>................. | ...YN.............<br>.............. | ......<br>.... |
| iPS:39 2622 | 21-225_17H8 | VK1\|A30/ JK4 | ............<br>........... | ...........<br>........ | ........<br>....... | G.......<br>....... | .................<br>............T.... | ..................<br>.............. | ......<br>.... |
| iPS:39 2624 | 21-225_17H12 | VK1\|A30/ JK4 | ............<br>........... | ......D....<br>........ | .....A..<br>......N | ........<br>....... | ........I........<br>....TG........... | ...Y..............<br>...........MF. | ......<br>.... |
| iPS:39 2628 | 21-225_20C2 | VK1\|A30/ JK4 | ............<br>...Q....... | ...........<br>........ | ........<br>......F | ........<br>....... | .................<br>................. | ...A..............<br>.............. | ......<br>...E |
| iPS:39 2630 | 21-225_20E5 | VK1\|A30/ JK4 | ............<br>........... | ...........<br>........ | ........<br>....... | ........<br>....... | .................<br>................. | ..................<br>.............. | ......<br>.... |
| iPS:39 2638 | 21-225_17F9 | VK1\|A30/ JK4 | ............<br>........... | ......V....<br>........ | ........<br>....... | ........<br>.V..... | .................<br>....N............ | ....T.............<br>.............. | ......<br>..F. |
| iPS:39 2640 | 21-225_18A1 | VK1\|A30/ JK4 | ............<br>........... | ...........<br>........ | ........<br>....... | ........<br>....... | .................<br>................. | ..................<br>.............. | ......<br>.... |
| iPS:39 2642 | 21-225_18C6 | VK1\|A30/ JK4 | ............<br>........... | .T.........<br>........ | ........<br>....... | ........<br>....... | .................<br>................. | ...S..............<br>.............. | ......<br>.... |
| iPS:39 2644 | 21-225_19E1 | VK1\|A30/ JK4 | ............<br>........... | ...........<br>........ | ........<br>....... | ........<br>...N... | .................<br>................. | ..................<br>..........F... | ......<br>.... |
| iPS:39 2646 | 21-225_20G2 | VK1\|A30/ JK4 | ............<br>........... | ...........<br>........ | ........<br>....... | ........<br>....F.. | .................<br>.............S... | ..................<br>.............. | ......<br>.... |
| iPS:39 2654 | 21-225_17A10 | VK1\|A30/ JK4 | ............<br>........... | ...........<br>........ | ........<br>....... | ........<br>....... | .................<br>................. | ..................<br>.............. | ......<br>.... |
| iPS:39 2656 | 21-225_1F2 | VK1\|A30/ JK4 | ............<br>........... | ...........<br>........ | ........<br>....... | ........<br>....V.. | .................<br>.............I... | ..................<br>.............. | ......<br>.... |
| iPS:39 2658 | 21-225_18E8 | VK1\|A30/ JK4 | ......A.....<br>........... | ...........<br>........ | ........<br>....... | ........<br>....... | .................<br>................. | ..................<br>.............. | ......<br>.... |
| iPS:39 2666 | 21-225_16F11 | VK1\|A30/ JK4 | ............<br>........... | ...........<br>........ | ........<br>....... | ........<br>....V.. | .................<br>................. | ..................<br>.............. | ......<br>.... |
| iPS:39 2676 | 21-225_19F3 | VK1\|A30/ JK4 | ............<br>...R....... | ...........<br>........ | ........<br>....... | ........<br>.V..... | .................<br>................. | ...A..............<br>.............. | ......<br>.... |
| iPS:39 2680 | 21-225_20A7 | VK1\|A30/ JK4 | ............<br>........... | ...........<br>........ | ........<br>....... | ........<br>....... | .................<br>S................ | ..................<br>.............. | ......<br>.... |
| iPS:39 2700 | 21-225_16E12 | VK1\|A30/ JK4 | ............<br>........... | ...........<br>........ | ........<br>....... | ........<br>....... | .................<br>................. | ..................<br>.............. | ......<br>.... |
| iPS:39 | 21-225_18A3 | VK1\|A30/ | ............ | ........... | ........ | V....... | ......N.......... | ...S.............. | ...... |

| | | | | | | | | | |
|---|---|---|---|---|---|---|---|---|---|
| iPS:39 2716 | 21-225_17B5 | VK1\|A30/ JK4 | ............<br>........... | ...........<br>........ | ........<br>....... | ........<br>...N... | ..................<br>SF................ | ...................<br>............... | ......<br>.... |
| iPS:39 2744 | 21-225_20D5 | VK1\|A30/ JK4 | ............<br>........... | ...........<br>........ | ........<br>....... | ........<br>....... | ..................<br>.................. | ...................<br>.............F. | ......<br>.... |
| iPS:39 2750 | 21-225_20A10 | VK1\|A30/ JK4 | ............<br>........... | ...........<br>........ | .....Q..<br>....... | ........<br>....... | ..................<br>.................. | ....R..............<br>............... | ......<br>.... |
| iPS:39 2772 | 21-225_20E12 | VK1\|A30/ JK4 | ............<br>........... | ...........<br>........ | ........<br>......F | ........<br>....... | ..................<br>.................. | ...................<br>.............F. | ......<br>.... |
| iPS:39 2774 | 21-225_21F3 | VK1\|A30/ JK4 | ............<br>........... | ...........<br>........ | ........<br>....... | ........<br>....... | ................D.<br>I................. | ...S...............<br>............... | ......<br>.... |
| iPS:39 2780 | 21-225_22B7 | VK1\|A30/ JK4 | ............<br>........... | ...........<br>........ | ........<br>....... | T.......<br>....... | ..................<br>.................. | ....T..............<br>............... | ......<br>.... |
| iPS:39 2788 | 21-225_20C8 | VK1\|A30/ JK4 | ............<br>........... | ...........<br>........ | ...K....<br>......N | ........<br>....... | ..................<br>.................. | ..D................<br>.............F. | ......<br>...T |
| iPS:39 2794 | 21-225_21H3 | VK1\|A30/ JK4 | ............<br>........... | ...........<br>........ | ........<br>....... | ........<br>....V.T | ..................<br>.........A..L.I... | ...................<br>............... | ......<br>.... |
| iPS:39 2800 | 21-225_22D12 | VK1\|A30/ JK4 | ............<br>........... | ...........<br>........ | ........<br>....... | ........<br>....... | ................D.<br>.................. | ...ST..............<br>............... | ......<br>.... |
| iPS:39 2810 | 21-225_20H12 | VK1\|A30/ JK4 | ............<br>........... | ...........<br>.......D | ........<br>....... | ........<br>....... | ..................<br>.................. | ...................<br>............... | ......<br>.... |
| iPS:39 2820 | 21-225_23D1 | VK1\|A30/ JK4 | ............<br>........... | ...........<br>........ | ........<br>....... | ........<br>....... | ..................<br>...V.............. | ...S...............<br>............... | ......<br>.... |
| iPS:39 2822 | 21-225_23C8 | VK1\|A30/ JK4 | ..........R.<br>........... | ......D....<br>........ | .......R<br>......N | G.......<br>....V.. | ..................<br>.............VI... | ...................<br>............... | ......<br>.... |
| iPS:39 2824 | 21-225_24E5 | VK1\|A30/ JK4 | ............<br>........... | ...........<br>........ | ........<br>....... | ........<br>....... | ..................<br>.................. | ...SN..............<br>............... | ......<br>.... |
| iPS:39 2834 | 21-225_22C1 | VK1\|A30/ JK4 | ............<br>........... | .T.........<br>........ | ........<br>....... | ........<br>....... | ..................<br>.................. | ...ST..............<br>............... | ......<br>.... |
| iPS:39 2838 | 21-225_22G8 | VK1\|A30/ JK4 | ....I......F<br>........... | .T.........<br>........ | ........<br>....... | ........<br>....... | ....K.............<br>..S............... | ...................<br>............... | ......<br>.... |
| iPS:39 2850 | 21-225_20H10 | VK1\|A30/ JK4 | G...........<br>........... | ........K..<br>.....N.. | ........<br>G..C... | ........<br>....... | ..................<br>.................. | ...................<br>............... | ......<br>.... |
| iPS:39 2854 | 21-225_21E5 | VK1\|A30/ JK4 | ............<br>........... | ...........<br>........ | ........<br>....... | ........<br>.T..... | ..................<br>.................. | ...................<br>............... | ......<br>.... |
| iPS:39 2858 | 21-225_22H4 | VK1\|A30/ JK4 | ............<br>........... | ...........<br>........ | ........<br>....... | ........<br>....V.. | ..................<br>..............L... | ...................<br>............... | ......<br>.... |
| iPS:39 2866 | 21-225_23H11 | VK1\|A30/ JK4 | ............<br>........... | ...........<br>.......D | ........<br>....... | ........<br>....... | ..................<br>.................. | ....R..............<br>............... | ......<br>.... |
| iPS:39 2870 | 21-225_20G9 | VK1\|A30/ JK4 | ............<br>........... | ...........<br>........ | ........<br>....... | ........<br>....... | ..................<br>.................. | ...ST..............<br>............... | ......<br>.... |
| iPS:39 2880 | 21-225_22F9 | VK1\|A30/ JK4 | ............<br>........... | ...........<br>........ | ........<br>....... | ........<br>....... | ..................<br>.................. | ...................<br>............... | ......<br>.... |

| | | | | | | | | | |
|---|---|---|---|---|---|---|---|---|---|
| iPS:39 2882 | 21-225_23A3 | VK1\|A30/ JK4 | .............<br>............ | ............<br>........ | .........<br>........ | .........<br>....V.. | ..................<br>.............I.F. | ...................<br>............... | .......<br>.... |
| iPS:39 2896 | 21-225_21G7 | VK1\|A30/ JK4 | .............<br>............ | .......V...<br>........ | .........<br>..Q.... | .........<br>........ | ......N..........<br>.................. | ...S...............<br>............... | .......<br>.... |
| iPS:39 2900 | 21-225_22F2 | VK1\|A30/ JK4 | .............<br>............ | ............<br>........ | .........<br>........ | .........<br>........ | ..................<br>.................. | ...................<br>............... | .......<br>.... |
| iPS:39 2904 | 21-225_22G9 | VK1\|A30/ JK4 | .............<br>............ | ............<br>........ | .........<br>........ | .........<br>........ | ..................<br>.................. | ...A...............<br>............... | .......<br>.... |
| iPS:39 2942 | 21-225_30E9 | VK1\|A30/ JK4 | .............<br>............ | ......D....<br>....D... | .........<br>..R.... | G.......<br>..F.... | ..................<br>.................. | ...T...............<br>...........P. | .......<br>.... |
| iPS:39 2944 | 21-225_31H5 | VK1\|A30/ JK4 | .............<br>......F..SF | ......D....<br>....S... | .........<br>.H..I.. | .........<br>........ | .......V.........<br>.F...M..D..SN... | I..II..............<br>...........P. | .......<br>.... |
| iPS:39 2964 | 21-225_31A8 | VK1\|A30/ JK4 | .............<br>............ | ......D....<br>....S... | .........<br>........ | .........<br>.V..... | ..................<br>.................. | ...TI..............<br>...........P. | .......<br>.... |
| iPS:39 2980 | 21-225_29H6 | VK1\|A30/ JK4 | .............<br>............ | ............<br>........ | .....S..<br>T...... | .........<br>........ | ..................<br>.................. | ...................<br>............... | .......<br>.... |
| iPS:39 2982 | 21-225_30D1 | VK1\|A30/ JK4 | .............<br>............ | ......D....<br>....S... | .........<br>..E.... | .........<br>........ | ..................<br>...........T... | ...TI..............<br>...........P. | .......<br>.... |
| iPS:39 2986 | 21-225_31B8 | VK1\|A30/ JK4 | ....I........<br>............ | ......D....<br>....S... | .........<br>........ | G.......<br>........ | ..................<br>.................. | ...II..............<br>...........P. | .......<br>.... |
| iPS:39 2988 | 21-225_25E6 | VK1\|A30/ JK4 | .............<br>............ | ............<br>........ | ...R....<br>........ | .........<br>........ | ......R..........<br>....N............. | ...................<br>............... | .......<br>.... |
| iPS:39 2990 | 21-225_25H10 | VK1\|A30/ JK4 | .............<br>............ | .....R.....<br>........ | .........<br>........ | .........<br>..F.... | ..................<br>.................. | ...................<br>............... | .......<br>.... |
| iPS:39 3004 | 21-225_30G11 | VK1\|A30/ JK4 | .............<br>............ | ......D....<br>....S... | .........<br>........ | .........<br>........ | ..................<br>....G........F. | ...TI..............<br>...........P. | .......<br>.... |
| iPS:39 3018 | 21-225_29B8 | VK1\|A30/ JK4 | .............<br>............ | ............<br>........ | .........<br>........ | .........<br>........ | .........R......<br>.................. | ...................<br>............... | .......<br>.... |
| iPS:39 3030 | 21-225_25H11 | VK1\|A30/ JK4 | .............<br>............ | ............<br>....T... | .........<br>........ | .........<br>........ | ..................<br>.................. | ...................<br>............... | .......<br>.... |
| iPS:39 3034 | 21-225_27F2 | VK1\|A30/ JK4 | .............<br>............ | ............<br>........ | .........<br>........ | V.......<br>........ | ..................<br>.................. | ...................<br>............... | .......<br>.... |
| iPS:39 3040 | 21-225_30E3 | VK1\|A30/ JK4 | .............<br>............ | ......D....<br>....D... | .........<br>..R.... | .........<br>..F.... | ..................<br>.................. | ...T...............<br>...........P. | .......<br>.... |
| iPS:39 3048 | 21-225_27C3 | VK1\|A30/ JK4 | .............<br>............ | ............<br>........ | .........<br>........ | .........<br>........ | ..................<br>.................. | ....R..............<br>............... | .......<br>.... |
| iPS:39 3054 | 21-225_29G8 | VK1\|A30/ JK4 | .............<br>............ | ............<br>........ | ...L....<br>........ | .........<br>........ | ..................<br>...............N. | ...................<br>............... | .......<br>.... |
| iPS:39 3056 | 21-225_30F3 | VK1\|A30/ JK4 | .............<br>............ | ............<br>........ | .........<br>........ | T.......<br>........ | ...............D.<br>............S... | ...................<br>...........F. | .......<br>.... |
| iPS:39 3058 | 21-225_31H3 | VK1\|A30/ JK4 | .............<br>............ | ......D....<br>....D... | .........<br>..R.... | .........<br>..F.... | ..................<br>.................. | ...T...............<br>...........P. | .......<br>.... |

| | | | | | | | | | |
|---|---|---|---|---|---|---|---|---|---|
| iPS:39 3060 | 21-225_32G12 | VK1\|A30/ JK4 | ............<br>........... | ......D....<br>....S... | ....R...<br>....... | ........<br>....... | ..................<br>................. | ...TI..............<br>............P. | ......<br>.... |
| iPS:39 3068 | 21-225_34G9 | VK1\|A30/ JK4 | ............<br>........... | ......D....<br>....S... | ........<br>....... | ........<br>....... | ......T...........<br>..........A..I... | ...TI..............<br>............P. | ......<br>.W.. |
| iPS:39 3072 | 21-225_36C5 | VK1\|A30/ JK4 | ............<br>........... | ......D....<br>....S... | ........<br>....... | ........<br>....... | ......T...........<br>......V.......... | .H.PI..............<br>............P. | ......<br>.W.. |
| iPS:39 3074 | 21-225_33B1 | VK1\|A30/ JK4 | ............<br>........... | .T.........<br>........ | .F......<br>....... | T.......<br>....... | ..................<br>................. | ...................<br>.............. | ......<br>..V. |
| iPS:39 3076 | 21-225_33A4 | VK1\|A30/ JK4 | ............<br>........... | ......D....<br>......V. | ........<br>..E.... | ........<br>......R | ..................<br>............R... | ...Y...............<br>............P. | ......<br>.... |
| iPS:39 3096 | 21-225_34D11 | VK1\|A30/ JK4 | ............<br>........... | ......D....<br>....S... | ........<br>....... | ........<br>....... | ..................<br>...VI............ | ...TI..............<br>............P. | ......<br>.G.. |
| iPS:39 3102 | 21-225_33F1 | VK1\|A30/ JK4 | ............<br>........... | .T....D....<br>....S... | ........<br>....... | ........<br>....... | ..................<br>.............I... | ...TI..............<br>............P. | ......<br>.... |
| iPS:39 3104 | 21-225_33A7 | VK1\|A30/ JK4 | ............<br>........... | ......D....<br>....S... | ........<br>....... | V.......<br>....... | ..................<br>.............A... | ...TI..............<br>............P. | ......<br>.... |
| iPS:39 3106 | 21-225_34A6 | VK1\|A30/ JK4 | ............<br>........... | .T....D....<br>........ | ........<br>......F | ........<br>....... | ..................<br>...V............. | ...................<br>............P. | ......<br>.... |
| iPS:39 3110 | 21-225_35B7 | VK1\|A30/ JK4 | ............<br>......I.... | ......D....<br>....S... | ........<br>..E.... | ........<br>....... | ..................<br>................. | ...TI..............<br>............P. | ......<br>.... |
| iPS:39 3118 | 21-225_34H11 | VK1\|A30/ JK4 | ............<br>........... | ......D....<br>........ | ........<br>....... | ........<br>.T..... | ..................<br>................. | ...................<br>............P. | ......<br>.... |
| iPS:39 3124 | 21-225_33G7 | VK1\|A30/ JK4 | ............<br>........... | ...........<br>........ | ........<br>....... | ........<br>....... | ..................<br>................. | ...Y...............<br>............P. | ......<br>.... |
| iPS:39 3126 | 21-225_35D1 | VK1\|A30/ JK4 | ............<br>........... | ......D....<br>....S... | ........<br>....... | ........<br>....... | ..................<br>................. | ...TI..............<br>............P. | ......<br>.... |
| iPS:39 3128 | 21-225_35F11 | VK1\|A30/ JK4 | ............<br>........... | ......D....<br>....S... | ........<br>....... | ........<br>....... | ..................<br>................. | ...TV..............<br>............P. | ......<br>.... |
| iPS:39 3146 | 21-225_34G8 | VK1\|A30/ JK4 | ............<br>........... | ......D....<br>....S... | ........<br>....... | ........<br>....... | ..................<br>................. | ...TI..............<br>............P. | ......<br>.... |
| iPS:39 3150 | 21-225_36A5 | VK1\|A30/ JK4 | ............<br>........... | .T....D....<br>........ | ........<br>......F | ........<br>....... | ..................<br>................. | .H.................<br>............PK | ....I.<br>...T |
| iPS:39 3804 | 21-225_5H7 | VK1\|A30/ JK4 | ............<br>........... | ...........<br>........ | ........<br>....... | V.......<br>.T....G | ..................<br>................. | ...................<br>.............. | ......<br>.... |
| iPS:39 3806 | 21-225_6A6 | VK1\|A30/ JK4 | ............<br>........... | ...........<br>........ | ........<br>....... | ........<br>....... | ..................<br>................. | ...S...............<br>.............. | ......<br>.... |
| iPS:39 3808 | 21-225_1A2 | VK1\|A30/ JK4 | ............<br>........... | ...........<br>........ | ........<br>....... | ........<br>....... | ..................<br>................. | ...................<br>..........H... | ......<br>.... |
| iPS:39 3814 | 21-225_7F4 | VK1\|A30/ JK4 | ............<br>........... | .T.........<br>........ | ........<br>....... | ........<br>...N... | ..................<br>................. | ...SA..............<br>.............. | ......<br>.... |
| iPS:39 3816 | 21-225_6D4 | VK1\|A30/ JK4 | ............<br>........... | ......A....<br>........ | ........<br>....... | ........<br>....... | ..................<br>................. | ...S...............<br>.............. | ......<br>.... |

| | | | | | | | | | |
|---|---|---|---|---|---|---|---|---|---|
| iPS:39 3818 | 21-225_6G12 | VK1\|A30/ JK4 | ............ ........... | ........... ........ | ........ ....... | ........ ...T... | ................. ................ | ................... .............. | ...... .... |
| iPS:39 3820 | 21-225_8H7 | VK1\|A30/ JK4 | ............ ........... | ........... ........ | ........ ....... | ........ ....... | ................. ................ | ................... ............F. | ...... .... |
| iPS:39 3826 | 21-225_10G5 | VK1\|A30/ JK4 | ............ ........... | ........... ........ | ........ ....... | ........ ....... | ................. ................ | ................... .............. | ...... .... |
| iPS:39 3828 | 21-225_10H12 | VK1\|A30/ JK4 | ............ ........... | ........... ........ | ........ ....... | G....... ....... | ................. ................ | ................... .............. | ...... .... |
| iPS:39 3830 | 21-225_12A1 | VK1\|A30/ JK4 | ............ ........... | ........... ........ | ........ ....... | ........ ....... | ................. ................ | ................... .............. | ...... .... |
| iPS:39 3832 | 21-225_14B2 | VK1\|A30/ JK4 | ............ ........... | ........... ........ | ........ ....... | V....... .T....G | ................. ................ | ................... .............. | ...... .... |
| iPS:39 3854 | 21-225_7H11 | VK1\|A30/ JK4 | ..........A. ....V....I. | L.......... ........ | ........ ....I.. | V....... ..C.F.. | ..........Y...... .....IM......... | ....L.............. .............. | ...... .... |
| iPS:39 3856 | 21-225_14C2 | VK1\|A30/ JK4 | ....I......F .C.....I... | ........... .......D | ........ ....... | ........ ....... | ................. ......V.R....... | V.................. .............. | ...... ...R |
| iPS:39 3866 | 21-225_14E3 | VK1\|A30/ JK4 | ............ ........... | ........... ........ | ........ ....I.. | S....... ....... | ......I...C...... ........R...A..S | V..Y............... ............F. | ...... .... |
| iPS:39 3872 | 21-225_2A11 | VK1\|A30/ JK4 | ............ P.......... | ........... ........ | ........ ..Q...S | ........ ....... | ...............D. ................ | ................... .............. | ...... M... |
| iPS:39 3874 | 21-225_4C8 | VK1\|A30/ JK4 | ....I....F.F .C......... | ........... ........ | ........ ....... | ........ ....... | .........V....... ................ | ................... .............. | ...... .... |
| iPS:39 3880 | 21-225_15A1 | VK1\|A30/ JK4 | ............ ........... | ........... ........ | ........ ....... | ........ ....... | ................. ...V.........S... | .HNS............... ...........VK | ....I. ...T |
| iPS:39 3882 | 21-225_15E3 | VK1\|A30/ JK4 | ............ ........... | ........... ........ | ........ ....... | ........ ....S.. | .........R....... ...........L.A... | ...H............... .............. | .....E ...Y |
| iPS:39 3884 | 21-225_16F4 | VK1\|A30/ JK4 | ....I.....P. ........... | ........... ........ | .....S.. ....... | V....... ....... | ................. .I....V......... | I.................. ............F. | ...... .... |
| iPS:39 3886 | 21-225_2G9 | VK1\|A30/ JK4 | ...........F ........... | ........... ........ | ........ ..Q.... | ........ ....... | ..........F...... ........L....... | ...E............... .............. | ...... .... |
| iPS:39 3922 | 21-225_2B2 | VK1\|A30/ JK4 | ............ ........... | ........... ........ | .......Q ....... | ........ ....... | ................. ...........L...H. | ................... .............. | ...... .... |
| iPS:39 3928 | 21-225_4E10 | VK1\|A30/ JK4 | ...........F ........... | ........... ........ | ........ ....... | ........ ....... | ..........F...... ........L....... | ...DN.............. .............. | ...... .... |
| iPS:39 3934 | 21-225_13E6 | VK1\|A30/ JK4 | ...V........ ..........S | ........... ........ | ........ ....... | ........ ....... | ................. ................ | V.................. .............. | ...... .A.. |
| iPS:39 3958 | 21-225_5H2 | VK1\|A30/ JK4 | ............ ...T....... | ........... ........ | ........ ....... | ........ ....... | ................. S............... | ................... .............. | ...... .... |
| iPS:39 3962 | 21-225_7H7 | VK1\|A30/ JK4 | ............ ........... | ........... ........ | ........ ....... | ........ ....... | ..T.............. ................ | ...S............... ............F. | .V.... .... |
| iPS:39 3974 | 21-225_7C4 | VK1\|A30/ JK4 | ...........F ........... | ........... ........ | ........ .H..... | ........ ....... | ................. ................ | ................... .............. | ...... .... |

| | | | | | | | | | |
|---|---|---|---|---|---|---|---|---|---|
| iPS:39 3976 | 21-225_7E9 | VK1\|A30/ JK4 | ...........F<br>........... | ...........<br>........ | ..E.....<br>....... | T.......<br>....... | ..................<br>.................. | ...................<br>............... | ......<br>.... |
| iPS:39 3982 | 21-225_6C12 | VK1\|A30/ JK4 | ............<br>......G.... | ...........<br>....SN.. | ........<br>....... | ........<br>.....E. | ..................<br>................F. | ..D................<br>.............F. | ......<br>.... |
| iPS:39 3984 | 21-225_4F12 | VK1\|A30/ JK4 | ............<br>........... | ...........<br>........ | ........<br>....... | ........<br>....... | ........G.......I.<br>.................. | ...................<br>............A.. | ......<br>.... |
| iPS:39 3990 | 21-225_11G7 | VK1\|A30/ JK4 | ............<br>........... | ...........<br>........ | ........<br>....... | ........<br>....... | ..................<br>.................. | ...SN..............<br>............... | .....M<br>...R |
| iPS:39 3994 | 21-225_8C9 | VK1\|A30/ JK4 | ............<br>..I........ | ......A....<br>.......D | ........<br>....... | ........<br>....... | ..................<br>........T......... | ...................<br>............... | ......<br>.... |
| iPS:39 4002 | 21-225_15G7 | VK1\|A30/ JK4 | ............<br>........... | ...........<br>........ | ........<br>....... | ........<br>....... | .I........F.......<br>.................. | ...SN..............<br>............... | ......<br>.... |
| iPS:39 4008 | 21-225_15H8 | VK1\|A30/ JK4 | ............<br>........... | ...........<br>........ | ........<br>......F | ........<br>....... | ..................<br>.................. | ...................<br>............... | ......<br>...R |
| iPS:39 4020 | 21-225_15H10 | VK1\|A30/ JK4 | ............<br>........... | ...........<br>........ | ........<br>....... | ........<br>....... | ..................<br>..............S... | ...................<br>............... | ......<br>...N |
| iPS:39 4024 | 21-225_16B7 | VK1\|A30/ JK4 | ............<br>........... | ...........<br>........ | ........<br>....... | ........<br>....... | ..................<br>.................. | ...................<br>............... | ......<br>..FN |
| iPS:39 4037 | 21-225_4F4 | VK1\|A30/ JK4 | ............<br>........... | ...........<br>........ | ........<br>....... | ........<br>....V.T | ..................<br>..............I... | ...................<br>............... | ......<br>.... |
| iPS:39 4045 | 21-225_4H4 | VK1\|A30/ JK4 | ............<br>........... | ...........<br>........ | ........<br>....... | ........<br>....V.. | ..................<br>..............I... | ...................<br>............... | ......<br>.... |
| iPS:39 4049 | 21-225_13H5 | VK1\|A30/ JK4 | ............<br>........... | ...........<br>........ | ........<br>....... | ........<br>......T | ..................<br>I................. | ...................<br>............... | ......<br>.... |
| iPS:39 4053 | 21-225_11F10 | VK1\|A30/ JK4 | ............<br>........... | ...........<br>........ | ........<br>....... | ........<br>....... | ..................<br>...V.............. | ...S...............<br>............... | ......<br>.... |
| iPS:39 4057 | 21-225_15H1 | VK1\|A30/ JK4 | ............<br>........... | ...........<br>........ | ........<br>....... | T.......<br>....... | ......N...........<br>.................. | ...S...............<br>............... | ......<br>.... |
| iPS:39 4059 | 21-225_9E8 | VK1\|A30/ JK4 | ...........F<br>........... | ...........<br>........ | ........<br>....... | ........<br>....... | ..................<br>.................. | ...................<br>............... | ......<br>.... |
| iPS:39 4063 | 21-225_16A1 | VK1\|A30/ JK4 | ............<br>........... | ...........<br>........ | ........<br>....... | ........<br>....... | ..................<br>.................. | .H.SN..............<br>............... | ......<br>...E |
| iPS:39 4073 | 21-225_15C9 | VK1\|A30/ JK4 | ............<br>........... | ...........<br>........ | ........<br>....... | ........<br>....... | ..................<br>...V.............. | ...T...............<br>............... | ......<br>.... |
| iPS:39 4075 | 21-225_8D12 | VK1\|A30/ JK4 | ............<br>........... | ...........<br>........ | ........<br>....... | ........<br>....... | ......N...........<br>.................. | ...S...............<br>............... | ......<br>.... |
| iPS:39 4079 | 21-225_11F5 | VK1\|A30/ JK4 | ............<br>........... | ...........<br>........ | ........<br>....... | ........<br>....V.. | ..................<br>..............I... | ...................<br>............... | ......<br>.... |
| iPS:39 4091 | 21-225_13H3 | VK1\|A30/ JK4 | ............<br>........... | ...........<br>........ | ........<br>....... | ........<br>....... | ..................<br>.................. | ...................<br>............... | ......<br>.... |
| iPS:39 8528 | 21-225_32G1 | VK1\|A30/ JK4 | ............<br>........... | ......DM...<br>....S... | ........<br>....... | ........<br>....... | ..................<br>.................. | ...TI..............<br>...........S.P. | ......<br>.... |

| | | | | | | | | | |
|---|---|---|---|---|---|---|---|---|---|
| iPS:39 8534 | 21-225_33B8 | VK1\|A30/ JK4 | ............<br>........... | ......D....<br>....S... | ........<br>....... | ........<br>....... | .................<br>...............F. | ...TI..............<br>............P. | ......<br>.... |
| iPS:39 8540 | 21-225_35A6 | VK1\|A30/ JK4 | ............<br>........... | ......D....<br>....S... | ........<br>....... | ........<br>.T..... | .................<br>................ | ...TI..............<br>............P. | ......<br>.... |
| iPS:40 2219 | 21-225_1C12 | VK1\|A30/ JK4 | ............<br>........... | ...........<br>....... | ........<br>....... | ........<br>....... | .................<br>................ | ..................<br>.............. | ......<br>.A.. |
| iPS:40 3868 | 21-225_19D11 | VK1\|A30/ JK4 | ............<br>........... | ...........<br>....... | ........<br>....... | ........<br>.T..... | .................<br>................ | ..YY..............<br>.............. | .....E<br>.... |
| iPS:40 3872 | 21-225_8F11 | VK1\|A30/ JK4 | ............<br>........... | ...........<br>....S... | .F......<br>......F | D.......<br>....V.. | .................<br>.............I... | ...YT.............<br>.............. | ......<br>.... |
| | | Germline | **K_FR1** | **K_CDR1** | **K_FR2** | **K_CDR2** | **K_FR3** | **K_CDR3** | **K_FR4** |
| | VK2\|A18/JK 5 | | DIVMIQTPLSLS<br>VTPGQPASISC | KSS--<br>QSLLHSD-<br>GKTYLY | WYLQKPGQ<br>SPQLLIY | E-------<br>-VSSRFS | GVPDRFSGSGSG--<br>TDFTLKISRVEAEDVGV<br>YYC | MQGIH-------------<br>----------LPIT | FGQGTR<br>LEIK |
| iPS:46 8816 | 21-225_52G8 | VK2\|A18/ JK5 | ............<br>........M.. | ...........<br>E....... | .....T..<br>P.H.... | ........<br>...K.L. | .................<br>......M......... | ..SMQ.............<br>.............I | ......<br>.... |
| iPS:43 4021 | 21-225_49C1 | VK2\|A18/ JK5 | ............<br>........... | ..........R<br>E....... | ........<br>A..F..F | ........<br>...H... | .................<br>................ | ..S.Q.............<br>..........I... | L.....<br>.... |
| iPS:43 4025 | 21-225_49G3 | VK2\|A18/ JK5 | ............<br>........M.. | ...........<br>E....... | .....T..<br>P.H.... | ........<br>...N.L. | .................<br>......M......F. | ..SMQ.............<br>.............. | ......<br>.... |
| iPS:43 4031 | 21-225_49E7 | VK2\|A18/ JK5 | ............<br>.......FM.. | ......IF...<br>E....... | .....T..<br>P.H.... | ........<br>...K.L. | .................<br>......M......... | ..SMQ.............<br>.............I | ......<br>.... |
| iPS:43 4033 | 21-225_49F9 | VK2\|A18/ JK5 | ............<br>........... | ..N.....V.N<br>E....... | ........<br>P.....F | ........<br>...N... | .................<br>................ | ..S.Q.............<br>..........Y... | ......<br>.... |
| iPS:43 4093 | 21-225_52D10 | VK2\|A18/ JK5 | .VM...I.....<br>........... | ...........<br>E....... | ........<br>P.....F | ........<br>...N.V. | ........R........<br>................ | ..S.Q.............<br>..........Y... | ......<br>.... |
| iPS:43 4151 | 21-225_55C2 | VK2\|A18/ JK5 | .VM...I.....<br>........... | .........V..<br>E....... | ........<br>P.....F | ........<br>...N.V. | ........R........<br>................ | ..S.L.............<br>..........Y... | ......<br>.... |
| iPS:43 4161 | 21-225_55F9 | VK2\|A18/ JK5 | ............<br>.....S..... | ...........<br>E....... | ........<br>P...... | ........<br>...N... | .................<br>................ | I.S.Q.............<br>.............. | ......<br>.... |
| iPS:43 5329 | 21-225_147A8 | VK2\|A18/ JK5 | ............<br>........... | .T.........<br>E....... | ........<br>P...... | ........<br>...N... | .................<br>................ | ..S.-<br>..................<br>.....QL.. | ......<br>.... |
| iPS:39 2924 | 21-225_32H2 | VK2\|A18/ JK5 | ............<br>........... | R..........<br>...R.... | ........<br>P...... | ........<br>.L.N... | .................<br>.............I.S. | L.S.Q.............<br>..........Y... | ......<br>.... |
| | | Germline | **K_FR1** | **K_CDR1** | **K_FR2** | **K_CDR2** | **K_FR3** | **K_CDR3** | **K_FR4** |
| | VK2\|A18/JK 1 | | DIVMTQTPLSLS<br>VTPGQPASISC | KSS--<br>QSLLHSD-<br>GKTYLY | WYLQKPGQ<br>SPQLLIY | E-------<br>-VSSRFS | GVPDRFSGSGSG--<br>TDFTLKISRVEAEDVGV<br>YYC | MQGIH-------------<br>----------LPWT | FGQGTK<br>VEIK |
| iPS:46 8822 | 21-225_147E10 | VK2\|A18/ JK1 | A...........<br>........... | ......R...G<br>....... | ........<br>P.H...S | ........<br>...N... | .................<br>................ | ..S.Q.............<br>..........V... | ......<br>.... |
| iPS:43 | 21- | VK2\|A18/ | ............ | ........... | ........ | ........ | ................. | ..S.Q............. | ...... |

| | | | | | | | | | |
|---|---|---|---|---|---|---|---|---|---|
| iPS:43 3965 | 21-225_46F2 | VK2\|A18/ JK1 | ...........T<br>........... | ..........G<br>........ | ........<br>P..V... | ........<br>...N... | .................<br>...L............. | ..S.Q.............<br>.............. | ......<br>.... |
| iPS:43 3985 | 21-225_47C1 | VK2\|A18/ JK1 | ............<br>........... | T..........<br>E....... | ........<br>P...... | ........<br>...N... | .................<br>..............F.. | ..S.Q.............<br>.............. | ......<br>.... |
| iPS:43 3991 | 21-225_47E7 | VK2\|A18/ JK1 | ............<br>........... | ..........<br>...R.... | ........<br>P...... | ........<br>....... | .................<br>A................ | ..STQ.............<br>.............. | ......<br>A... |
| iPS:43 4345 | 21-225_64H9 | VK2\|A18/ JK1 | E...........<br>........... | ..........G<br>.......F | ........<br>P..V... | ........<br>...N.LC | .................<br>S................ | ..S.Q.............<br>..........V... | ......<br>...T |
| iPS:43 5297 | 21-225_146B3 | VK2\|A18/ JK1 | ............<br>........... | ..........G<br>........ | ........<br>P...... | ........<br>...N... | .................<br>.............L.H. | ..S.Q.............<br>.............. | ......<br>.... |
| iPS:43 5341 | 21-225_148B2 | VK2\|A18/ JK1 | A...........<br>........... | ..........G<br>......F. | ........<br>P...... | ........<br>...H... | .................<br>................. | ..S.Q.............<br>..........I... | ......<br>.... |
| iPS:43 5357 | 21-225_148G10 | VK2\|A18/ JK1 | ............<br>........... | ..........G<br>........ | ....R...<br>P...... | ........<br>...N... | .................<br>................. | ..S.Q.............<br>.............. | ......<br>.... |
| iPS:43 5365 | 21-225_149F1 | VK2\|A18/ JK1 | ......S....F<br>..........Y | ..........G<br>......F. | ........<br>P...... | ........<br>...H... | .................<br>................. | ..S.Q.............<br>..........I... | ......<br>.... |
| iPS:43 5413 | 21-225_150B11 | VK2\|A18/ JK1 | ............<br>........... | ........V.G<br>........ | ....R...<br>P...... | ........<br>...N... | .................<br>................. | ..S.Q.............<br>.............. | ......<br>.... |
| iPS:43 5423 | 21-225_151G5 | VK2\|A18/ JK1 | ............<br>........... | ......R...G<br>........ | ........<br>P....L. | ........<br>...N... | .................<br>................. | ..S.Q.............<br>..........V... | ......<br>.... |
| iPS:43 5429 | 21-225_151A10 | VK2\|A18/ JK1 | ............<br>........... | ..........G<br>........ | ........<br>P...... | ........<br>...H... | .................<br>................. | ..S.Q.............<br>..........I... | ......<br>.... |
| iPS:43 5441 | 21-225_152F6 | VK2\|A18/ JK1 | ............<br>........... | ........R.G<br>.......T | ....R...<br>P..V..H | ........<br>.I.K..T | .................<br>..N..........F... | ..S.Q.............<br>..........V... | ......<br>.... |
| iPS:43 5457 | 21-225_152C11 | VK2\|A18/ JK1 | ......A.....<br>........... | ..........G<br>........ | ........<br>P..I... | ........<br>...N... | .................<br>..N........F.F... | ..S.Q.............<br>..........I... | ......<br>.D.. |
| iPS:43 5463 | 21-225_153D2 | VK2\|A18/ JK1 | ............<br>........... | ........R.G<br>.......T | ....R...<br>P..V..H | ........<br>...K..T | .................<br>..N..........F... | ..S.Q.............<br>..........V... | ......<br>.... |
| iPS:43 5489 | 21-225_155A5 | VK2\|A18/ JK1 | ............<br>........... | ..........G<br>........ | ........<br>P...... | ........<br>...N... | .................<br>.............F... | ..S.Q.............<br>..........V... | ......<br>.... |
| iPS:43 5531 | 21-225_157G8 | VK2\|A18/ JK1 | ............<br>........... | ..........G<br>........ | ........<br>....... | ........<br>.I.K... | .................<br>................. | ..S.Q.............<br>..........V... | ......<br>.... |
| iPS:43 5577 | 21-225_160B1 | VK2\|A18/ JK1 | ............<br>........... | ..........G<br>......F. | ........<br>P..V... | ........<br>...K... | ..SE.............<br>................. | ..S.Q.............<br>..........I... | ......<br>.... |
| iPS:43 5601 | 21-225_160G10 | VK2\|A18/ JK1 | A...........<br>........... | ..........G<br>........ | ........<br>P.H.... | ........<br>...K... | .................<br>.............L... | ..S.Q.............<br>.............. | .V....<br>...T |
| iPS:43 5629 | 21-225_162H6 | VK2\|A18/ JK1 | ............<br>........... | ..T.......G<br>........ | ........<br>P...... | ........<br>...K... | ...E.............<br>................. | K.S.Q.............<br>.............. | ......<br>.... |
| iPS:43 5655 | 21-225_167E2 | VK2\|A18/ JK1 | ............<br>........... | ..........G<br>........ | ........<br>P.H.... | ........<br>...K... | .................<br>.............L.H. | ..S.Q.............<br>.............. | ......<br>.... |
| iPS:43 5657 | 21-225_167H10 | VK2\|A18/ JK1 | ............<br>........... | ..........G<br>........ | ........<br>P.H.... | ........<br>...K... | .................<br>.............L.H. | ..S.Q.............<br>.............. | ......<br>.... |

| | | | | | | | | | |
|---|---|---|---|---|---|---|---|---|---|
| iPS:43<br>5683 | 21-<br>225_170A1 | VK2\|A18/<br>JK1 | E........F..<br>........... | ..........G<br>.......F | ........<br>P..V... | ........<br>...N... | .................<br>................. | ..S.Q............<br>.............. | ......<br>.... |
| iPS:43<br>5723 | 21-<br>225_172B7 | VK2\|A18/<br>JK1 | ............<br>........... | ..........G<br>......F. | ........<br>P..V.LF | ........<br>...H... | ........G........<br>................. | ..S.Q............<br>..........F... | .....R<br>.D.. |
| iPS:43<br>5731 | 21-<br>225_173A11 | VK2\|A18/<br>JK1 | ............<br>........... | ..........G<br>........ | ........<br>P...... | ........<br>...N... | .................<br>..............FF. | ..S.Q............<br>..........V... | ......<br>.... |
| iPS:43<br>5755 | 21-<br>225_176H4 | VK2\|A18/<br>JK1 | ............<br>........... | ..........G<br>........ | ........<br>P...... | ........<br>...N... | .................<br>.............I... | ..S.Q............<br>..........I... | .....R<br>.... |
| iPS:43<br>5771 | 21-<br>225_177B11 | VK2\|A18/<br>JK1 | ............<br>........... | ......R...G<br>........ | ........<br>P..I... | ........<br>...N... | ...............E.<br>................. | ..S.Q............<br>..........I... | ......<br>.... |
| iPS:43<br>5781 | 21-<br>225_178G10 | VK2\|A18/<br>JK1 | H...........<br>........... | ..........G<br>........ | ........<br>P...... | ........<br>...N... | .....L..G........<br>.............I... | ..S.Q............<br>..........V... | ......<br>.... |
| iPS:43<br>5789 | 21-<br>225_180C4 | VK2\|A18/<br>JK1 | ............<br>........... | ..........G<br>........ | ........<br>P...... | A.......<br>.T.N..P | ..S..............<br>................. | ..S.Q............<br>..........V... | ......<br>.... |
| iPS:43<br>5795 | 21-<br>225_181C2 | VK2\|A18/<br>JK1 | E...........<br>........... | ..........G<br>........ | ........<br>P.....H | ........<br>...N... | .................<br>................. | ..S.Q............<br>..........V... | ......<br>.... |
| iPS:43<br>5807 | 21-<br>225_181C10 | VK2\|A18/<br>JK1 | ............<br>........... | ..........G<br>........ | ........<br>P...... | ........<br>...N... | .................<br>...............H. | ..S.Q............<br>..........I... | ......<br>.... |
| iPS:43<br>5827 | 21-<br>225_190H11 | VK2\|A18/<br>JK1 | ............<br>........... | ...........<br>...R.... | ........<br>P.....C | ........<br>...N..A | ..T..............<br>.........G....... | ..S.Q............<br>..........F... | ......<br>.... |
| iPS:43<br>5839 | 21-<br>225_191B1 | VK2\|A18/<br>JK1 | ............<br>........... | R..........<br>.......F | ........<br>P..V... | ........<br>.L.N... | .................<br>................. | ..SFQ............<br>.............. | ......<br>...N |
| iPS:43<br>5853 | 21-<br>225_191E3 | VK2\|A18/<br>JK1 | ............<br>........... | ...........<br>...R.... | ........<br>P.....C | ........<br>...N..A | ..T..............<br>.........G....... | ..S.Q............<br>.............. | ......<br>.... |
| iPS:43<br>5871 | 21-<br>225_191E6 | VK2\|A18/<br>JK1 | ............<br>........... | ...........<br>...R.... | ........<br>P.....C | ........<br>...N..A | ..T..............<br>.........G...I... | ..S..............<br>..........F... | ......<br>.... |
| iPS:43<br>5887 | 21-<br>225_186F7 | VK2\|A18/<br>JK1 | .V..A.......<br>........... | ..........G<br>.......C | ........<br>P...... | ........<br>...K... | .................<br>................. | ..S.Q............<br>..........V... | ......<br>.... |
| iPS:43<br>5899 | 21-<br>225_188G11 | VK2\|A18/<br>JK1 | ............<br>........... | M.........G<br>........ | ........<br>P...... | ........<br>...N... | ....T............<br>................. | ..S.Q............<br>..........I... | ......<br>.... |
| iPS:43<br>5901 | 21-<br>225_189G2 | VK2\|A18/<br>JK1 | ............<br>........... | ..........G<br>.......F | ........<br>P...... | ........<br>...N... | ..S..............<br>................. | ..S.Q............<br>..........I... | ......<br>.... |
| iPS:43<br>5927 | 21-<br>225_190E7 | VK2\|A18/<br>JK1 | ...L........<br>........... | ...........<br>...R.... | ........<br>P..V..C | ........<br>...N... | .................<br>.........G....... | ..S.Q............<br>.............. | ......<br>.... |
| iPS:43<br>5999 | 21-<br>225_192F9 | VK2\|A18/<br>JK1 | ............<br>........... | ...........<br>...R.... | ....R...<br>P.....C | ........<br>...N..A | ..T..............<br>.........G....... | ..S.Q............<br>.............. | ......<br>.... |
| iPS:43<br>6060 | 21-<br>225_194F4 | VK2\|A18/<br>JK1 | ............<br>........... | ...........<br>...R.... | ........<br>P.....C | ........<br>...N... | .................<br>.........G....... | ..S.Q............<br>.............. | ......<br>.... |
| iPS:43<br>6158 | 21-<br>225_197G8 | VK2\|A18/<br>JK1 | E...........<br>.I......... | ......N....<br>........ | ........<br>P..V... | ........<br>...N... | .................<br>...............S. | ..S.Q............<br>.............. | .A..S.<br>.... |
| iPS:43<br>6193 | 21-<br>225_198A10 | VK2\|A18/<br>JK1 | ...L........<br>........... | .........Y.<br>...R.... | ........<br>P..V..C | ........<br>...N... | .................<br>.........G....... | ..S.Q............<br>.............. | ......<br>.... |

| | | | | | | | | | |
|---|---|---|---|---|---|---|---|---|---|
| iPS:43 6536 | 21-225_224G1 | VK2\|A18/ JK1 | ............<br>........... | ..G........<br>.....F.S | ........<br>P...... | ........<br>.I.N... | .................<br>..............IF.. | ..STQ.............<br>............R. | ......<br>.... |
| iPS:43 6548 | 21-225_224A7 | VK2\|A18/ JK1 | ............<br>........... | ...........<br>.....F.. | .F......<br>P...... | ........<br>.I.N... | .................<br>..............I... | ..STQ.............<br>............R. | ......<br>.... |
| iPS:43 6558 | 21-225_224C11 | VK2\|A18/ JK1 | .F..........<br>........... | ...........<br>.....F.. | ........<br>P...... | ........<br>.I.N... | .................<br>..............I... | ..STQ.............<br>............R. | ......<br>.... |
| iPS:43 6562 | 21-225_224H11 | VK2\|A18/ JK1 | ............<br>........... | ...........<br>.....F.. | ........<br>P...... | ........<br>.I.N... | .................<br>..............I... | ..STQ.............<br>............R. | ......<br>.... |
| iPS:43 6572 | 21-225_225G4 | VK2\|A18/ JK1 | ............<br>........... | ...........<br>.....F.. | ........<br>P...... | ........<br>.I.N... | .................<br>..............I... | ..STQ.............<br>............R. | ......<br>.... |
| iPS:43 6592 | 21-225_226B1 | VK2\|A18/ JK1 | ............<br>........... | ..........G<br>........ | ....R...<br>P.....N | ........<br>...I... | .................<br>................. | ..S.Q.............<br>.........I... | ......<br>.D.. |
| iPS:43 6594 | 21-225_226A5 | VK2\|A18/ JK1 | ............<br>........... | ..........G<br>........ | ........<br>P...... | ........<br>...N... | .................<br>................. | ..S.Q.............<br>.........I... | ......<br>.... |
| iPS:43 6602 | 21-225_226E7 | VK2\|A18/ JK1 | ............<br>........... | ..........G<br>........ | ..Q.....<br>P..I... | ........<br>...N... | .................<br>................. | ..S.Q.............<br>.........V... | ......<br>.... |
| iPS:43 6606 | 21-225_226G8 | VK2\|A18/ JK1 | ............<br>........... | ...........<br>.....F.. | ........<br>P...... | ........<br>.I.N... | .................<br>..............I... | ..STQ.............<br>............R. | ......<br>.... |
| iPS:43 6610 | 21-225_226F9 | VK2\|A18/ JK1 | ............<br>........... | ...........<br>.....F.. | ........<br>P...... | ........<br>.I.N... | .................<br>..............I... | ..STQ.............<br>............R. | ......<br>.... |
| iPS:43 6612 | 21-225_226H9 | VK2\|A18/ JK1 | ............<br>.I......... | ...........<br>.....F.. | ....R...<br>P...... | ........<br>...N... | .................<br>..............I... | ..STQ.............<br>............R. | ......<br>.... |
| iPS:43 6614 | 21-225_226F10 | VK2\|A18/ JK1 | ............<br>........... | ...........<br>.....F.. | ........<br>P...... | ........<br>.I.N... | .................<br>..............I... | ..STQ.............<br>............R. | ......<br>.... |
| iPS:43 6618 | 21-225_226E11 | VK2\|A18/ JK1 | ............<br>........... | ...........<br>.....F.. | ...H....<br>P.H.... | ........<br>.I.N... | .................<br>..............I... | ..STQ.............<br>............R. | ......<br>.... |
| iPS:43 6624 | 21-225_226H12 | VK2\|A18/ JK1 | ............<br>........... | .....KT....<br>.....F.. | ........<br>P..P... | ........<br>.I.N... | .................<br>..............I... | ..STQ.............<br>............R. | ......<br>.... |
| iPS:43 6626 | 21-225_227C1 | VK2\|A18/ JK1 | ............<br>........... | ...........<br>.....F.. | ........<br>P...... | ........<br>.I.N... | .................<br>..............I... | ..STQ.............<br>............R. | ......<br>.... |
| iPS:43 6628 | 21-225_227F2 | VK2\|A18/ JK1 | ............<br>........... | ...........<br>.....F.. | ........<br>P...... | ........<br>.I.N... | .................<br>..............I... | ..STQ.............<br>............R. | ......<br>.... |
| iPS:43 6640 | 21-225_227A8 | VK2\|A18/ JK1 | ............<br>........... | ...........<br>.....F.. | ........<br>P...... | ........<br>.I.N... | .................<br>..............I... | ..STQ.............<br>............R. | .....R<br>.... |
| iPS:39 2814 | 21-225_22A1 | VK2\|A18/ JK1 | ............<br>........... | ...........<br>G...... | ........<br>P...... | ........<br>...N... | .L...............<br>........A...... | ..TL..............<br>.............. | ......<br>.... |
| iPS:39 2930 | 21-225_25H9 | VK2\|A18/ JK1 | ............<br>........... | ..........G<br>.......F | ........<br>P...... | ........<br>...N... | .................<br>................. | ..S.Q.............<br>.............. | ......<br>.... |
| iPS:39 3032 | 21-225_26F8 | VK2\|A18/ JK1 | ............<br>........... | ..........G<br>........ | ........<br>P...... | ........<br>...N... | .................<br>................. | ..S.Q.............<br>.............. | ......<br>.... |
| iPS:39 3036 | 21-225_28G3 | VK2\|A18/ JK1 | E...........<br>........... | ..........G<br>........ | ........<br>P...... | ........<br>...N... | ...E.............<br>................. | ..S.Q.............<br>.........I... | ......<br>.... |

| | VK1\|A30/JK1 | | DIQMTQSPSSLS<br>ASVGDRVTITC | RAS--QGIR--<br>----NDLG | WYQQKPGK<br>APKRLIY | A-------<br>-ASSLQS | GVPSRFSGSGSG--<br>TEFTLTISSLQPEDFAT<br>YYC | LQHNS-------------<br>----------YPWT | FGQGTK<br>VEIK |
|---|---|---|---|---|---|---|---|---|---|
| iPS:468826 | 21-225_201C5 | VK1\|A30/JK1 | ............<br>........... | ...........<br>....H... | ........<br>....... | ........<br>....... | ..............<br>................. | ...Y..............<br>..........F.R. | ......<br>.... |
| iPS:468842 | 21-225_50H4 | VK1\|A30/JK1 | ............<br>........... | ...........<br>........ | ........<br>....... | ........<br>....... | ..............<br>................. | ...Y..............<br>............R. | ......<br>.... |
| iPS:468858 | 21-225_148C9 | VK1\|A30/JK1 | ............<br>........... | .....R.....<br>....D... | ........<br>....... | ........<br>....... | ..............<br>................. | ...Y..............<br>............R. | ......<br>.... |
| iPS:468860 | 21-225_224E7 | VK1\|A30/JK1 | ............<br>........... | ...........<br>........ | ........<br>..T.... | ........<br>....... | ..............<br>................. | ...Y..............<br>............R. | ......<br>.... |
| iPS:433919 | 21-225_44B3 | VK1\|A30/JK1 | ............<br>........... | ...........<br>........ | ........<br>....... | ........<br>....... | ..............<br>................. | .L.YN.............<br>............R. | ......<br>.... |
| iPS:433923 | 21-225_44D3 | VK1\|A30/JK1 | ............<br>.......F... | ...........<br>....D... | ........<br>....... | ........<br>....... | ........R...R.<br>................. | ...Y..............<br>............R. | ......<br>.... |
| iPS:433929 | 21-225_44D5 | VK1\|A30/JK1 | ............<br>........... | ......D....<br>....K... | ........<br>....... | ........<br>...T.E. | ..............<br>................. | ...Y..............<br>..........F... | ......<br>.... |
| iPS:433935 | 21-225_44F9 | VK1\|A30/JK1 | .V..........<br>........... | ...........<br>........ | ........<br>....... | ........<br>....... | ..............<br>................. | .H.YN.............<br>............R. | ......<br>...T |
| iPS:433939 | 21-225_44C10 | VK1\|A30/JK1 | ............<br>.......F... | ...........<br>....D... | ........<br>....... | ........<br>.T..... | ..............<br>................. | ...Y..............<br>............R. | ......<br>.... |
| iPS:433951 | 21-225_45B4 | VK1\|A30/JK1 | ............<br>.......F... | ...........<br>....D... | ........<br>....... | ........<br>....... | ..............<br>................. | ...Y..............<br>............R. | ......<br>.... |
| iPS:433955 | 21-225_45B8 | VK1\|A30/JK1 | ............<br>.......F... | ......D....<br>....D... | ........<br>....... | ........<br>....... | ..............<br>................. | ...YN.............<br>............R. | ......<br>.... |
| iPS:433967 | 21-225_46C3 | VK1\|A30/JK1 | ............<br>.......F... | ...........<br>....D... | ........<br>....... | ........<br>....... | ........R.....<br>................. | ...Y..............<br>............R. | ......<br>.... |
| iPS:433971 | 21-225_46D4 | VK1\|A30/JK1 | ............<br>........... | .T...D.....<br>....K... | ......V.<br>....... | ........<br>.....E. | ..............<br>................. | ...Y..............<br>..........F... | ......<br>.... |
| iPS:433997 | 21-225_48C1 | VK1\|A30/JK1 | ............<br>........... | .T.........<br>........ | ........<br>....... | R.......<br>....... | ...A..........<br>..............I.. | ....F.............<br>.............. | ......<br>.... |
| iPS:434001 | 21-225_48F2 | VK1\|A30/JK1 | ............<br>........... | .....R.....<br>....D... | ........<br>P...... | ........<br>....... | .....N........<br>........S..L..... | ..QY..............<br>............R. | ......<br>.... |
| iPS:434009 | 21-225_48A9 | VK1\|A30/JK1 | ............<br>........... | ...........<br>........ | ........<br>....... | I.......<br>....... | ..............<br>....G......I..... | ....R.............<br>.............. | ......<br>.... |
| iPS:434047 | 21-225_50A12 | VK1\|A30/JK1 | ............<br>........... | ...........<br>........ | ........<br>....... | T.......<br>...N... | ..............<br>................. | ..................<br>.............. | ......<br>.... |
| iPS:434067 | 21-225_51H8 | VK1\|A30/JK1 | ............<br>........... | ...........<br>........ | ........<br>....... | ........<br>....... | ........G....H<br>................. | ..................<br>.............. | ......<br>.... |
| iPS:434135 | 21-225_54H3 | VK1\|A30/JK1 | ............<br>........... | ......D....<br>.....I.. | ........<br>....... | ........<br>...N... | ..............<br>................. | ..Y...............<br>.............. | ......<br>.... |
| iPS:43 | 21-225_56C7 | VK1\|A30/ | ............ | ........... | ........ | T....... | .............. | .................. | ...... |

| | | | | | | | | | |
|---|---|---|---|---|---|---|---|---|---|
| iPS:43 4203 | 21-225_60E2 | VK1\|A30/ JK1 | ............ ........... | ........... ....K... | ........ ....... | ........ ....... | ................. ................. | .................. .............. | ...... .... |
| iPS:43 4209 | 21-225_60C3 | VK1\|A30/ JK1 | ............ ........... | ........... ........ | ........ ....... | S....... ....... | ................. ................. | .................. .............. | ..L... .... |
| iPS:43 4229 | 21-225_61H1 | VK1\|A30/ JK1 | ............ ........... | ........... ....K... | ........ ......F | ........ ....... | ................. ................. | .................. .............. | ...... .... |
| iPS:43 4241 | 21-225_61E6 | VK1\|A30/ JK1 | ............ ......I.... | ........G.. ........ | ........ ..E.... | ........ ....... | ................. ................. | .................. .........FP... | ...... .... |
| iPS:43 4257 | 21-225_62F7 | VK1\|A30/ JK1 | ............ ........... | ......A.... ........ | ........ ....... | P....... ...R... | ................. ................. | ..Y............... .........YP... | ....S. .... |
| iPS:43 4281 | 21-225_57B8 | VK1\|A30/ JK1 | ............ ......I.... | ........G.. ........ | ........ ..E.... | ........ ....... | ................. ................. | .................. .........FP... | ...... .... |
| iPS:43 4315 | 21-225_59G7 | VK1\|A30/ JK1 | ............ ........... | ........... ........ | ........ ....... | S....... ....... | ................. ................. | .................. .............. | ...... .... |
| iPS:43 4319 | 21-225_59B9 | VK1\|A30/ JK1 | ............ ........... | ......D.... ........ | ....H... ....... | ........ ....... | ........TR....... ................. | .................. .............. | ...... .... |
| iPS:43 4339 | 21-225_64A4 | VK1\|A30/ JK1 | ............ ........... | ........... ........ | ........ ....... | ........ ....... | ................. ...........L..... | ...Y.............. ............R. | ...... .... |
| iPS:43 4343 | 21-225_64C8 | VK1\|A30/ JK1 | ............ .A......... | ........... ........ | ........ ...C... | ........ ....... | ................. ................. | .H.Y.............. ............R. | ...... .... |
| iPS:43 4385 | 21-225_66C10 | VK1\|A30/ JK1 | ............ ........... | ......A.... ........ | ........ ....... | P....... ....... | ................. ................. | ..Y............... .........YP... | ....S. .... |
| iPS:43 4387 | 21-225_66D11 | VK1\|A30/ JK1 | ......F...Q. ........... | ........... ........ | ........ .H..... | ........ ....C.. | ..........C...... .IS...M.R........ | IV................ ............R. | ...... .... |
| iPS:43 4441 | 21-225_71A2 | VK1\|A30/ JK1 | ..........R. ........... | ........... ........ | ........ ....... | I....... ..FR..I | ................. ................. | IH................ .............. | ...... .... |
| iPS:43 4469 | 21-225_73C9 | VK1\|A30/ JK1 | ............ ........... | ........... ........ | ........ ....... | ........ ....... | ................. ................. | ...Y.............. ............R. | ...... .... |
| iPS:43 5197 | 21-225_94F3 | VK1\|A30/ JK1 | ............ ........... | ......A.... ....D... | ........ ..Q...F | ........ ....... | ................. ................. | ...Y.............. ............R. | ..R... .A.. |
| iPS:43 5325 | 21-225_147H5 | VK1\|A30/ JK1 | ............ ........... | .....R..... ....D... | ........ ....... | ........ ....... | ................. ................. | ...Y.............. ............R. | ...... .... |
| iPS:43 5393 | 21-225_149D10 | VK1\|A30/ JK1 | ............ ........... | .....R..... ........ | ........ ....... | ........ ....... | ................. ................. | ...Y.............. ............R. | ...... .... |
| iPS:43 5539 | 21-225_158G1 | VK1\|A30/ JK1 | ............ ........... | ........... ........ | ........ ....... | T....... ...N... | ..........C...... ...V............. | .................. .............. | ...... .... |
| iPS:43 5543 | 21-225_158D4 | VK1\|A30/ JK1 | ............ ........... | ......D.... ....K... | ........ ....... | ........ ....... | ................. ...F............. | ...Y.............. ............R. | ...... .... |
| iPS:43 5571 | 21-225_159C8 | VK1\|A30/ JK1 | ............ ........... | ......D.... ....K... | ........ ..N.... | ........ ....... | ................. ...F............. | ...H.............. ............R. | ...... .... |
| iPS:43 5573 | 21-225_159D8 | VK1\|A30/ JK1 | ............ ........... | .....RD.G.. ........ | ........ ......S | ........ ....... | ................. ...F............. | ...Y.............. ............R. | ...... .... |

| | | | | | | | | | |
|---|---|---|---|---|---|---|---|---|---|
| iPS:43 5581 | 21-225_160H1 | VK1\|A30/ JK1 | ............<br>........... | ......D....<br>........ | ........<br>....... | ........<br>......N | .................<br>............T.... | ..................<br>..........F... | ......<br>.... |
| iPS:43 5583 | 21-225_160F2 | VK1\|A30/ JK1 | ............<br>........... | ...........<br>........ | ........<br>....... | T.......<br>...N... | .................<br>...V............. | ..................<br>.............. | ......<br>.... |
| iPS:43 5591 | 21-225_160C4 | VK1\|A30/ JK1 | ............<br>........... | ......D....<br>....K... | ........<br>..N.... | ........<br>....... | .................<br>...F............. | ...Y..............<br>............R. | ......<br>.... |
| iPS:43 5615 | 21-225_161G12 | VK1\|A30/ JK1 | ..........R.<br>........... | ......D....<br>....K... | ........<br>..N.... | ........<br>....... | .................<br>...F............. | ...H..............<br>............R. | ......<br>.... |
| iPS:43 5675 | 21-225_169D7 | VK1\|A30/ JK1 | ............<br>........... | ...........<br>........ | ........<br>....... | ........<br>....... | .................<br>................. | ...H..............<br>..........C... | ......<br>.... |
| iPS:43 5681 | 21-225_169D11 | VK1\|A30/ JK1 | ............<br>........... | ...........<br>....D... | ........<br>V...... | ........<br>....... | .................<br>................. | ...Y..............<br>............R. | ......<br>.... |
| iPS:43 5687 | 21-225_170H1 | VK1\|A30/ JK1 | ............<br>........... | ...........<br>........ | ........<br>....... | ........<br>.T..... | .................<br>................. | ...S..............<br>..........N... | ......<br>..S. |
| iPS:43 5689 | 21-225_170F3 | VK1\|A30/ JK1 | ............<br>...Q....... | .....R.....<br>........ | ........<br>....... | ........<br>....... | .................<br>....N............ | ...Y..............<br>............R. | ......<br>.... |
| iPS:43 5741 | 21-225_174G10 | VK1\|A30/ JK1 | ............<br>........... | ...........<br>....D... | ........<br>V...... | ........<br>....... | .................<br>................. | ...H..............<br>............R. | ......<br>.... |
| iPS:43 5831 | 21-225_190C12 | VK1\|A30/ JK1 | ............<br>........... | ...........<br>....K... | ........<br>......H | T.......<br>....... | ...L.............<br>................. | ..................<br>.............. | ......<br>.... |
| iPS:43 5857 | 21-225_191A4 | VK1\|A30/ JK1 | ............<br>........... | ...........<br>....K... | ........<br>......H | T.......<br>......N | ...L.............<br>................. | ..................<br>.............. | ......<br>.... |
| iPS:43 5907 | 21-225_190G3 | VK1\|A30/ JK1 | ............<br>...R....... | ...........<br>....K... | ........<br>......H | T.......<br>....... | ...L.............<br>................. | ..................<br>.............. | ......<br>.... |
| iPS:43 5919 | 21-225_190H5 | VK1\|A30/ JK1 | ............<br>........... | ...........<br>....K... | ........<br>......H | T.......<br>....... | ...L.............<br>................. | ....N.............<br>.............. | ......<br>.... |
| iPS:43 5989 | 21-225_192F7 | VK1\|A30/ JK1 | ............<br>........... | ...........<br>....K... | ........<br>......H | T.......<br>....... | ...L.............<br>................. | ...T..............<br>.............. | ......<br>...Y |
| iPS:43 6132 | 21-225_196C12 | VK1\|A30/ JK1 | .....L.....F<br>.C.....I... | ...........<br>........ | .S..N...<br>....... | ........<br>....... | .................<br>.I.........E.... | ....D.............<br>..........F.F. | ..R...<br>.... |
| iPS:43 6222 | 21-225_200C9 | VK1\|A30/ JK1 | ............<br>........... | ..T........<br>....K... | ........<br>......H | T.......<br>....... | ...L.............<br>................. | ..................<br>.............. | ......<br>.... |
| iPS:43 6264 | 21-225_203F7 | VK1\|A30/ JK1 | ..........RF<br>........... | ...........<br>....H... | ........<br>.L..... | ........<br>....... | ..........C......<br>......V.....N... | ...Y..............<br>..........F.R. | ......<br>.... |
| iPS:43 6274 | 21-225_204H3 | VK1\|A30/ JK1 | ............<br>........... | ...........<br>........ | ........<br>..EL... | ........<br>..A...G | .................<br>................. | ...Y..............<br>............R. | ......<br>..LQ |
| iPS:43 6332 | 21-225_208B2 | VK1\|A30/ JK1 | ............<br>........... | ...........<br>....H... | ........<br>....... | ........<br>....... | ....G............<br>................. | ...Y..............<br>............R. | ......<br>.... |
| iPS:43 6352 | 21-225_210G5 | VK1\|A30/ JK1 | ............<br>........... | ...........<br>....H... | ........<br>....... | ........<br>....... | ....G............<br>................. | .H.Y..............<br>............R. | ......<br>.... |
| iPS:43 6386 | 21-225_212B11 | VK1\|A30/ JK1 | ..........P<br>........... | ...........<br>........ | ........<br>....... | ........<br>....... | .................<br>I................ | .L.Y..............<br>............R. | ......<br>.... |

| | | | | | | | | | |
|---|---|---|---|---|---|---|---|---|---|
| iPS:43 6412 | 21-225_214H9 | VK1\|A30/JK1 | ............<br>........... | ...........<br>........ | ........<br>....... | .......<br>....... | ................<br>................ | ...Y............<br>............R. | ......<br>.... |
| iPS:43 6414 | 21-225_214G10 | VK1\|A30/JK1 | .....LC...PP<br>........... | ...........<br>........ | ........<br>....... | .......<br>....... | ................<br>I............... | .L..............<br>............R. | ......<br>.... |
| iPS:43 6416 | 21-225_214G12 | VK1\|A30/JK1 | ..........PP<br>........... | ...........<br>........ | ........<br>....... | .......<br>....... | ................<br>I............... | VM.Y............<br>............R. | ......<br>.... |
| iPS:43 6418 | 21-225_215E3 | VK1\|A30/JK1 | ..........PP<br>........... | ...........<br>........ | ........<br>....... | .......<br>....... | ................<br>................ | VM..............<br>............R. | ......<br>.... |
| iPS:43 6428 | 21-225_215E11 | VK1\|A30/JK1 | ......C...PP<br>........... | ...........<br>........ | ........<br>....... | .......<br>....... | ................<br>I....V.R........ | VM.Y............<br>............R. | ......<br>.... |
| iPS:43 6438 | 21-225_216E8 | VK1\|A30/JK1 | ...........P<br>........... | ...........<br>........ | ........<br>....... | .......<br>....... | ................<br>I............... | .M.Y............<br>............R. | ......<br>.... |
| iPS:43 6440 | 21-225_216H12 | VK1\|A30/JK1 | .....L.....P<br>........... | ...........<br>........ | ........<br>....... | G......<br>....... | ................<br>I............... | VM..............<br>............R. | ......<br>.... |
| iPS:43 6450 | 21-225_217E5 | VK1\|A30/JK1 | ......C...PP<br>.F......... | ...........<br>........ | ........<br>......C | .......<br>....... | ................<br>I............... | VM..............<br>............R. | ......<br>.... |
| iPS:43 6456 | 21-225_217G10 | VK1\|A30/JK1 | ..........PP<br>........... | ...........<br>........ | ........<br>....... | .......<br>....... | ................<br>I............... | VM..............<br>............R. | ......<br>.... |
| iPS:43 6458 | 21-225_217H12 | VK1\|A30/JK1 | ..........PP<br>........... | ...........<br>........ | ........<br>....... | .......<br>....... | ................<br>I............... | .M.Y............<br>............R. | ......<br>.... |
| iPS:43 6462 | 21-225_218C4 | VK1\|A30/JK1 | ......C...PP<br>.F......... | ...........<br>........ | ........<br>....... | .......<br>....... | ................<br>I....V.R........ | VM.Y............<br>............R. | ......<br>.... |
| iPS:43 6480 | 21-225_220F8 | VK1\|A30/JK1 | .....L....PP<br>.F........R | ...........<br>........ | ........<br>......C | G......<br>....... | ................<br>I......R........ | VM..............<br>............R. | ......<br>.... |
| iPS:43 6534 | 21-225_224F1 | VK1\|A30/JK1 | A...........<br>........... | ...........<br>....D... | ........<br>....... | .......<br>....... | ....I...........<br>...........I.... | ...Y............<br>............R. | ......<br>.... |
| iPS:43 6540 | 21-225_224F3 | VK1\|A30/JK1 | ............<br>........... | ...........<br>........ | ........<br>..E.... | .......<br>....... | ................<br>................ | ...YN...........<br>............RA | ......<br>.... |
| iPS:43 6564 | 21-225_225A1 | VK1\|A30/JK1 | ............<br>........... | ...........<br>....D... | ....I...<br>....... | .......<br>....... | ................<br>................ | ...Y............<br>............R. | ......<br>.... |
| iPS:43 6596 | 21-225_226C6 | VK1\|A30/JK1 | ............<br>..I........ | ...........<br>........ | ........<br>....... | .......<br>....... | .L..............<br>....N........... | ...YN...........<br>............RA | ......<br>...Q |
| iPS:43 6604 | 21-225_226F7 | VK1\|A30/JK1 | ............<br>........... | .........G..<br>........ | ........<br>....... | .......<br>....... | ........V.......<br>I............... | .H.Y............<br>............R. | ......<br>.... |
| iPS:43 6620 | 21-225_226H11 | VK1\|A30/JK1 | ............<br>........... | .T.........<br>........ | ........<br>....... | .......<br>....... | .............D..<br>................ | ...Y............<br>............R. | ......<br>.... |
| iPS:43 7262 | 21-225_170E4 | VK1\|A30/JK1 | ............<br>........... | ...........<br>........ | Y.......<br>....... | V......<br>...G... | ................<br>................ | ................<br>.........YP... | ......<br>.D.. |
| iPS:43 7280 | 21-225_203C10 | VK1\|A30/JK1 | ............<br>........... | ......D....<br>........ | ......A.<br>....... | R......<br>....... | ................<br>.............A.. | ................<br>............. | ......<br>.... |
| iPS:43 7286 | 21-225_208F1 | VK1\|A30/JK1 | ............<br>........... | ...........<br>....H... | ........<br>......F | .......<br>....... | ................<br>................ | ...Y............<br>..........F.R. | ......<br>.... |

| | | | | | | | | | |
|---|---|---|---|---|---|---|---|---|---|
| iPS:43 7290 | 21-225_210G6 | VK1\|A30/ JK1 | ..........RF<br>.F......... | ............<br>....H... | ........<br>.L..... | ........<br>....S.. | ..........C......<br>......V.R....N... | V..Y..............<br>..........F.R. | ......<br>.... |
| iPS:45 1114 | 21-225_159A3 | VK1\|A30/ JK1 | ............<br>........... | ......D.....<br>....K... | ........<br>..N.... | ........<br>....... | .................<br>...F............. | ...H..............<br>............R. | ......<br>.... |
| iPS:39 2626 | 21-225_18A5 | VK1\|A30/ JK1 | ............<br>........... | ............<br>........ | ........<br>....... | ........<br>....... | .................<br>................. | ..................<br>.............. | ......<br>.... |
| iPS:39 2634 | 21-225_17H3 | VK1\|A30/ JK1 | ............<br>........F.. | ............<br>....S... | ........<br>....... | ........<br>....... | .................<br>................. | ..QY..............<br>............R. | ......<br>.... |
| iPS:39 2674 | 21-225_18C2 | VK1\|A30/ JK1 | ............<br>........... | ............<br>........ | ........<br>....... | T.......<br>....... | ..........F......<br>................. | ..................<br>.............. | ..L...<br>.V.. |
| iPS:39 2686 | 21-225_17C7 | VK1\|A30/ JK1 | ............<br>........... | ............<br>........ | ........<br>....... | ........<br>....... | .................<br>................. | ..................<br>.............. | ......<br>.... |
| iPS:39 2690 | 21-225_18F2 | VK1\|A30/ JK1 | ............<br>........... | ............<br>........ | ........<br>....... | ........<br>....... | .................<br>................. | ..................<br>.............. | ......<br>.... |
| iPS:39 2710 | 21-225_19A10 | VK1\|A30/ JK1 | ............<br>........... | ............<br>....T... | ....R...<br>....... | T.......<br>....... | .................<br>.....R........... | ....G.............<br>.............. | ......<br>.... |
| iPS:39 2740 | 21-225_18H12 | VK1\|A30/ JK1 | ............<br>........... | ............<br>........ | ........<br>....... | T.......<br>...N... | .................<br>................. | ....N.............<br>.............. | ..L...<br>.V.. |
| iPS:39 2742 | 21-225_20B2 | VK1\|A30/ JK1 | ............<br>.......N... | ......D.....<br>........ | ........<br>....... | ........<br>....... | .................<br>................. | ...YN.............<br>............RA | ......<br>.D.. |
| iPS:39 2758 | 21-225_21G11 | VK1\|A30/ JK1 | ............<br>........... | ............<br>........ | ........<br>....... | T.......<br>....... | .................<br>................. | ....N.............<br>.............. | ..L...<br>.V.. |
| iPS:39 2790 | 21-225_20D10 | VK1\|A30/ JK1 | ............<br>.F......... | ............<br>........ | ........<br>.R..... | V.......<br>..Y.... | ..........Y......<br>................. | I.Q...............<br>.............. | ......<br>.... |
| iPS:39 2796 | 21-225_22A4 | VK1\|A30/ JK1 | ............<br>........... | ............<br>........ | ........<br>....... | ........<br>....... | .................<br>................. | ..................<br>.............. | ......<br>.... |
| iPS:39 2832 | 21-225_21H8 | VK1\|A30/ JK1 | ............<br>........... | ............<br>........ | ........<br>....... | ........<br>....... | ......R..........<br>................. | ..................<br>.............. | ......<br>.... |
| iPS:39 2836 | 21-225_22F4 | VK1\|A30/ JK1 | ............<br>........... | ......V.....<br>....D... | ........<br>....... | ........<br>....... | .........R.....D.<br>................. | .H.Y..............<br>............R. | ......<br>.... |
| iPS:39 2844 | 21-225_23E11 | VK1\|A30/ JK1 | ............<br>......I.... | ............<br>........ | ........<br>....... | P.......<br>....... | .................<br>................. | ..................<br>.............. | ......<br>.... |
| iPS:39 2846 | 21-225_24B6 | VK1\|A30/ JK1 | ............<br>........... | ......D.....<br>........ | ........<br>....... | ........<br>.....H. | .................<br>........T........ | ...Y..............<br>............R. | ......<br>..V. |
| iPS:39 2872 | 21-225_20B11 | VK1\|A30/ JK1 | ............<br>........... | ........G...<br>........ | ......E.<br>....... | ........<br>.....H. | .................<br>................. | ...Y..............<br>............R. | ......<br>.... |
| iPS:39 2876 | 21-225_21F7 | VK1\|A30/ JK1 | ............<br>........... | ......D.....<br>........ | ........<br>....... | ........<br>...NF.. | .................<br>................. | ....N.............<br>.............. | ......<br>.... |
| iPS:39 2884 | 21-225_23A10 | VK1\|A30/ JK1 | ............<br>........... | ............<br>........ | ........<br>....... | ........<br>....... | .................<br>....G............ | ...Y..............<br>............R. | ..L...<br>.... |
| iPS:39 2894 | 21-225_21G2 | VK1\|A30/ JK1 | ............<br>........... | ............<br>........ | ........<br>....... | T.......<br>.S..... | .................<br>................. | ..................<br>.............. | ..L...<br>.V.. |

| | | | | | | | | | |
|---|---|---|---|---|---|---|---|---|---|
| iPS:39 2908 | 21-225_23F12 | VK1\|A30/ JK1 | ............<br>........... | ...........<br>........ | ........<br>....... | V.......<br>....... | .................<br>.............S... | ..................<br>.............. | ......<br>.... |
| iPS:39 2914 | 21-225_25D12 | VK1\|A30/ JK1 | ............<br>........... | .T.........<br>........ | ........<br>....... | ........<br>....... | .................<br>................. | ...Y..............<br>..........F.R. | ......<br>.... |
| iPS:39 2918 | 21-225_28F5 | VK1\|A30/ JK1 | ............<br>........... | ...........<br>........ | ........<br>....... | T.......<br>....... | .................<br>................. | ....T.............<br>.............. | ......<br>..V. |
| iPS:39 2958 | 21-225_28C7 | VK1\|A30/ JK1 | ............<br>........... | ...........<br>........ | ........<br>....... | I.......<br>....... | .................<br>................. | ....T.............<br>.............. | ......<br>.... |
| iPS:39 2972 | 21-225_26A2 | VK1\|A30/ JK1 | ............<br>........... | ...........<br>....S... | ........<br>....... | T.......<br>....... | .................<br>...F............. | ....R.............<br>.............. | ......<br>.... |
| iPS:39 3026 | 21-225_32B6 | VK1\|A30/ JK1 | ............<br>........... | ...........<br>........ | ........<br>....... | I.......<br>....... | .................<br>................. | ..................<br>.............. | ......<br>.... |
| iPS:39 3130 | 21-225_33C2 | VK1\|A30/ JK1 | ............<br>........... | ...........<br>........ | ......V.<br>....... | ........<br>..P.... | .................<br>..........H...... | ..................<br>.............. | ......<br>.... |
| iPS:39 3812 | 21-225_6A11 | VK1\|A30/ JK1 | ............<br>........... | ...........<br>........ | ........<br>....... | ........<br>....... | .................<br>................. | ..................<br>.............. | ......<br>.... |
| iPS:39 3838 | 21-225_6G2 | VK1\|A30/ JK1 | ..........R.<br>.F........Y | ...........<br>........ | ........<br>..Q.... | ........<br>....... | ..........Y......<br>NI.........I... | I.................<br>..........L.. | ......<br>...T |
| iPS:39 3864 | 21-225_4C5 | VK1\|A30/ JK1 | ............<br>....H..HL.. | .....R.....<br>....G... | ..R.....<br>....... | ........<br>...N... | ..........Y......<br>....G............ | ...Y..............<br>...........R. | ......<br>.... |
| iPS:39 3868 | 21-225_9C11 | VK1\|A30/ JK1 | ............<br>........... | ......N....<br>.....Y.N | .....S.R<br>...L... | V.......<br>....... | .................<br>....N.......I.H. | H.S...............<br>..........T.L. | ......<br>.... |
| iPS:39 3876 | 21-225_9A1 | VK1\|A30/ JK1 | ..........R.<br>.F......... | ...........<br>........ | ........<br>.R..... | T.......<br>....... | ..........Y......<br>.I............... | I.................<br>..........L.. | ......<br>.... |
| iPS:39 3902 | 21-225_14E10 | VK1\|A30/ JK1 | ............<br>........... | .T.........<br>........ | ...H..Q<br>....... | ........<br>....... | .................<br>............TA... | ...Y..............<br>...........R. | ......<br>.... |
| iPS:39 3908 | 21-225_10E9 | VK1\|A30/ JK1 | ............<br>.......F.. | ......D....<br>....S... | ........<br>..T...F | ........<br>....... | .................<br>....N............ | ...Y..............<br>...........R. | ......<br>.... |
| iPS:39 3916 | 21-225_2G4 | VK1\|A30/ JK1 | ............<br>........... | ...........<br>........ | ........<br>..T.... | ........<br>.....H. | .................<br>...........L..... | ...Y..............<br>..........F.R. | ..R...<br>.... |
| iPS:39 3948 | 21-225_16A5 | VK1\|A30/ JK1 | ............<br>........... | ...........<br>........ | C.......<br>....... | ........<br>....... | ........N.Y......<br>................. | ..................<br>.............. | ......<br>.... |
| iPS:39 3960 | 21-225_7G2 | VK1\|A30/ JK1 | ............<br>........... | ...........<br>........ | ........<br>....... | T.......<br>....... | .................<br>................. | ..................<br>.............. | ..L...<br>.V.. |
| iPS:39 3966 | 21-225_7F8 | VK1\|A30/ JK1 | ............<br>........... | ........G..<br>........ | ........<br>....... | ........<br>....... | .........V.......<br>..P.............. | ...YT.............<br>...........R. | ......<br>.... |
| iPS:39 3972 | 21-225_7C9 | VK1\|A30/ JK1 | ............<br>..G....... | ...........<br>........ | ........<br>....... | ........<br>....... | .................<br>................. | ..LY..............<br>...........R. | ......<br>.D.. |
| iPS:39 3978 | 21-225_4C12 | VK1\|A30/ JK1 | ............<br>........... | ...........<br>........ | ........<br>..T.... | ........<br>.....H. | .................<br>...........L..... | ...Y..............<br>..........F.R. | ......<br>.... |
| iPS:39 3986 | 21-225_7G4 | VK1\|A30/ JK1 | ............<br>........... | ...........<br>........ | ....R...<br>....... | ........<br>....... | .................<br>...........S..... | .HQY..............<br>...........R. | ......<br>.... |

| | | | | | | | | | |
|---|---|---|---|---|---|---|---|---|---|
| iPS:39 3996 | 21-225_15C11 | VK1\|A30/ JK1 | ............<br>........... | ...........<br>........ | ........<br>V...... | ........<br>....... | .................<br>................. | .L.Y..............<br>............R. | ..R...<br>.... |
| iPS:39 3998 | 21-225_12B12 | VK1\|A30/ JK1 | ............<br>........... | ...........<br>........ | ........<br>....... | T.......<br>....... | .................<br>................. | ..................<br>.............. | ..L...<br>.V.. |
| iPS:39 4041 | 21-225_5E5 | VK1\|A30/ JK1 | ............<br>........... | ...........<br>........ | ........<br>....... | ........<br>....... | .................<br>................. | ...Y..............<br>............R. | ......<br>..V. |
| iPS:39 4067 | 21-225_12F2 | VK1\|A30/ JK1 | ............<br>........... | ...........<br>........ | ........<br>....... | ........<br>....... | .................<br>............V.... | ..................<br>.............. | ......<br>.... |
| iPS:39 4089 | 21-225_12E6 | VK1\|A30/ JK1 | ............<br>........... | ...........<br>....S... | ........<br>....... | ........<br>....... | .................<br>................. | ..................<br>.............. | ......<br>.... |
| iPS:39 4093 | 21-225_9D12 | VK1\|A30/ JK1 | ............<br>........... | ...........<br>........ | ........<br>....... | ........<br>....... | .................<br>................. | ..................<br>.............. | ......<br>.... |
| iPS:39 4095 | 21-225_16H4 | VK1\|A30/ JK1 | ............<br>........... | ...........<br>........ | ........<br>....... | T.......<br>....... | .................<br>................. | ..................<br>.............. | ......<br>.... |
| iPS:39 4097 | 21-225_16G7 | VK1\|A30/ JK1 | ............<br>........... | ...........<br>........ | ........<br>....... | ........<br>....... | .................<br>................. | ..................<br>.............. | ......<br>.... |
| | | Germline | K_FR1 | K_CDR1 | K_FR2 | K_CDR2 | K_FR3 | K_CDR3 | K_FR4 |
| | VK3\|L2/JK2 | | EIVMTQSPATLS<br>VSPGERATLSC | RAS--QSVS--<br>----SNLA | WYQQKPGQ<br>APRLLIY | G-------<br>-ASTRAT | GIPARFSGSGSG--<br>TEFTLTISSLQSEDFAV<br>YYC | QQYNN-------------<br>----------WPYT | FGQGTK<br>LEIK |
| iPS:46 8828 | 21-225_162A10 | VK3\|L2/J K2 | ............<br>........... | ......T.N..<br>........ | .....S..<br>......F | ........<br>....... | V....IN..........<br>.......R.......F. | ....D.............<br>............CS | ......<br>.... |
| iPS:43 4255 | 21-225_62E6 | VK3\|L2/J K2 | ............<br>........... | ........N..<br>........ | ........<br>......S | V.......<br>....... | ......N..........<br>................. | ....D.............<br>............CS | ......<br>.... |
| iPS:43 4269 | 21-225_57H3 | VK3\|L2/J K2 | ............<br>........... | ...........<br>.....S.. | ........<br>....... | ........<br>....... | .F....N...W......<br>.............I... | ....D.............<br>............CS | ......<br>.... |
| iPS:43 4363 | 21-225_65A6 | VK3\|L2/J K2 | ........V..F<br>........... | ........N..<br>........ | ........<br>....... | ........<br>....... | ......N..........<br>................. | ....D.............<br>............CS | ..LE..<br>.... |
| iPS:43 4393 | 21-225_67C3 | VK3\|L2/J K2 | ............<br>........... | ........N..<br>........ | ........<br>......S | I.......<br>....... | ......N..........<br>................. | ....D.............<br>............CS | ......<br>.... |
| iPS:43 4425 | 21-225_70A5 | VK3\|L2/J K2 | ............<br>........... | ........N..<br>........ | ...L....<br>......S | I.......<br>....... | ...P..N..........<br>................. | ....D.............<br>............CS | ......<br>.... |
| iPS:43 4485 | 21-225_76D2 | VK3\|L2/J K2 | ..........P.<br>........... | .....V..V..<br>....NS.. | ........<br>......H | ........<br>....... | .................<br>......V......I... | ....D.............<br>............CS | ......<br>.... |
| iPS:43 4537 | 21-225_74E11 | VK3\|L2/J K2 | ............<br>........... | .....L..V..<br>....NS.. | ........<br>......H | ........<br>....... | .................<br>.............I... | ....D.............<br>............CS | ......<br>.... |
| iPS:43 4569 | 21-225_77H5 | VK3\|L2/J K2 | ........V...<br>........... | ...........<br>.....S.. | .......L<br>....... | ........<br>....... | .................<br>SF.............F. | ....D.............<br>............CS | ....S.<br>...Q |
| iPS:43 4629 | 21-225_74C3 | VK3\|L2/J K2 | ............<br>........... | ........A..<br>.....S.. | ........<br>......F | ........<br>.T..... | .................<br>..I.............. | ....D.............<br>............CS | ..L...<br>.... |
| iPS:43 4673 | 21-225_74E3 | VK3\|L2/J K2 | ............<br>L.......... | .....L..V..<br>....NS.. | ........<br>....... | ........<br>....... | .................<br>.............I... | ....D.............<br>............CS | ......<br>.... |

| | | | | | | | | | |
|---|---|---|---|---|---|---|---|---|---|
| iPS:43 5109 | 21-225_92H5 | VK3\|L2/J K2 | ............ ........... | ......D.I.. ....TY.. | ........ ....... | ........ ....... | .V............... ....T........L... | .E..D............ ............CS | ...... .... |
| iPS:43 5221 | 21-225_95G2 | VK3\|L2/J K2 | ............ L.......... | .....M..V.. ....NS.. | ........ ....... | ........ ....... | ................. .............I... | ....D............ ............CS | ...... .... |
| iPS:43 6051 | 21-225_193G12 | VK3\|L2/J K2 | ............ ........... | ........... .....S.. | ........ ...I..S | ........ ....... | ................. .........A....... | ................. ............CS | ...... .... |
| iPS:43 6236 | 21-225_201F7 | VK3\|L2/J K2 | ............ ........... | ......NIK.. ....N... | ........ ......F | ........ ....... | ................. ................. | ..FY............. ...........LCS | ...... ..L. |
| iPS:43 6250 | 21-225_201A4 | VK3\|L2/J K2 | ............ .....S..... | .S....NIK.. ........ | ........ ......F | ........ ....... | ................. ................. | ..FY............. ...........LCS | ...... ..L. |
| iPS:43 6252 | 21-225_202A8 | VK3\|L2/J K2 | ............ ........... | ......RIN.. ....N... | ....N... ....... | ........ ....... | .V............... ............T.... | ...Y............. ...........LCS | ...... ...R |
| iPS:43 6258 | 21-225_202F12 | VK3\|L2/J K2 | ............ ........... | ........L.. ....N... | ....R... ....... | ........ ....... | ................. ................. | ...D............. .........WP.CS | ...... .... |
| iPS:43 6278 | 21-225_201F2 | VK3\|L2/J K2 | ............ ........... | ......NIK.. ........ | ........ ......F | ........ ....... | ................. ................. | ..FY............. ...........LCS | ...... ..L. |
| iPS:43 6294 | 21-225_205G4 | VK3\|L2/J K2 | ............ ........... | ......NIK.. ........ | ........ ......F | ........ ....... | ................. ................. | ..FY............. ...........LCS | ...... ..L. |
| iPS:43 6306 | 21-225_201H4 | VK3\|L2/J K2 | ............ ........... | ........N.. .....Y.. | ........ ....... | ........ ....... | ................. ................. | .E..D............ ............CS | .....N .... |
| iPS:43 6356 | 21-225_210H10 | VK3\|L2/J K2 | ............ ........... | ........K.. ........ | ........ ....... | ........ ....... | ................. ...............S. | ...Y............. ...........LCS | ...... .... |
| iPS:43 6382 | 21-225_212C10 | VK3\|L2/J K2 | ............ ........... | ........A.. .....S.. | ........ ......H | ........ .T..... | DV......F.....SD. ...............S. | ....D............ ............CS | ...... .... |
| iPS:43 6434 | 21-225_216B10 | VK3\|L2/J K2 | .....E...... ........... | ........N.. ....N... | ..R..... ....... | ........ ....... | ...P............. ..S.............. | ....D............ ............CS | ...... ...R |
| iPS:39 2806 | 21-225_24H3 | VK3\|L2/J K2 | ............ ........... | ........... ........ | ........ ....... | F....... ...I... | ................. ................. | ................. .........WPMCS | ...... .... |
| | | Germline | K_FR1 | K_CDR1 | K_FR2 | K_CDR2 | K_FR3 | K_CDR3 | K_FR4 |
| | VK3\|L2/JK4 | | EIVMTQSPATLS VSPGERATLSC | RAS--QSVS-- ----SNLA | WYQQKPGQ APRLLIY | G------- -ASTRAT | GIPARFSGSGSG-- TEFTLTISSLQSEDFAV YYC | QQYNN------------ ----------WPLT | FGGGTK VEIK |
| iPS:46 8830 | 21-225_191G11 | VK3\|L2/J K4 | ............ .......N... | .T......W.. ....ISV. | ..H..... ....... | ........ ..A.... | ................. ................. | ....Y............ .............. | ...... .... |
| iPS:43 4147 | 21-225_55E1 | VK3\|L2/J K4 | ............ ........... | ........... .....D.. | ...L.... ....... | D....... ...A... | ................. .............F... | ...Y............. .............. | ...... .... |
| iPS:43 4621 | 21-225_74D1 | VK3\|L2/J K4 | ............ ........... | ........... ....R... | .F...... ....... | ........ ...I... | ................. ....Y............ | ................. .........WP... | ...... .... |
| iPS:43 5821 | 21-225_190E11 | VK3\|L2/J K4 | ............ ........... | .......FR.. ....I... | ....R... ....... | ........ ....... | ................. ................. | ................. .............. | ...... .... |
| iPS:43 5941 | 21-225_191E8 | VK3\|L2/J K4 | ............ ........... | .P.....F... ....R... | ........ ....... | ........ ....... | ...S............. .......E......... | ................. .............. | ....I. .... |

| | | | | | | | | | |
|---|---|---|---|---|---|---|---|---|---|
| iPS:43 6203 | 21-225_199A6 | VK3\|L2/JK4 | D...........<br>....D...... | .P.....F...<br>....R... | ........<br>....... | ........<br>....... | .................<br>.......EC....... | ...................<br>.............. | ......<br>.... |
| iPS:43 7334 | 21-225_75F11 | VK3\|L2/JK4 | ...........F<br>........... | ...........<br>....R... | .F......<br>.....F. | ........<br>...I... | .................<br>....Y...Y....... | ...................<br>.........WP... | ......<br>.... |
| iPS:44 8904 | 21-225_65C12 | VK3\|L2/JK4 | ............<br>.F...G..... | ...........<br>....I... | ........<br>....... | ........<br>....... | .......NA........<br>.S.......N...... | ....T..............<br>.............. | ......<br>.... |
| | | Germline | K_FR1 | K_CDR1 | K_FR2 | K_CDR2 | K_FR3 | K_CDR3 | K_FR4 |
| | VK1\|A30/JK3 | | DIQMTQSPSSLS<br>ASVGDRVTITC | RAS--QGIR--<br>----NDLG | WYQQKPGK<br>APKRLIY | A-------<br>-ASSLQS | GVPSRFSGSGSG--<br>TEFTLTISSLQPEDFAT<br>YYC | LQHNS-------------<br>----------YPFT | FGPGTK<br>VDIK |
| iPS:46 8832 | 21-225_76H10 | VK1\|A30/JK3 | ............<br>..A........ | ......D....<br>.....Y.. | ........<br>....... | G.......<br>....... | .................<br>.............F.. | ..Y................<br>.............. | ......<br>.... |
| iPS:46 8836 | 21-225_198E3 | VK1\|A30/JK3 | ............<br>........... | ...........<br>....K... | ........<br>....... | ........<br>....... | .................<br>..............V. | ...YR..............<br>.............. | ......<br>..F. |
| iPS:46 8844 | 21-225_48E10 | VK1\|A30/JK3 | ............<br>........... | ...........<br>....S... | ........<br>....... | T.......<br>....... | .................<br>................ | ..Y................<br>.............. | ......<br>.... |
| iPS:46 8846 | 21-225_53B10 | VK1\|A30/JK3 | ............<br>..A........ | ......D....<br>.....Y.. | ........<br>....... | G.......<br>....... | .................<br>.............F.. | ..Y................<br>.............. | ......<br>.... |
| iPS:43 3895 | 21-225_43E1 | VK1\|A30/JK3 | ............<br>........... | ...........<br>........ | ...K....<br>......N | ........<br>....... | .................<br>................ | ...................<br>.............. | ......<br>.... |
| iPS:43 3905 | 21-225_43E5 | VK1\|A30/JK3 | ............<br>........... | ...........<br>........ | ........<br>....... | G.......<br>...N... | .................<br>...V.........F. | ...T...............<br>.........F... | ......<br>.... |
| iPS:43 3913 | 21-225_43H8 | VK1\|A30/JK3 | ............<br>........... | ...........<br>........ | .H......<br>....... | ........<br>....... | .................<br>..S............. | ...................<br>.........F... | ......<br>.... |
| iPS:43 3933 | 21-225_44C8 | VK1\|A30/JK3 | ............<br>........... | ...........<br>........ | ........<br>....... | ........<br>...N... | .........R.......<br>................ | ...................<br>.............. | ......<br>.... |
| iPS:43 3949 | 21-225_45H2 | VK1\|A30/JK3 | ............<br>........... | ...........<br>........ | ........<br>....... | G.......<br>...N... | .................<br>...V.........F. | ...T...............<br>.........F... | ......<br>.... |
| iPS:43 3981 | 21-225_46E9 | VK1\|A30/JK3 | ............<br>........... | ...........<br>........ | ........<br>....... | ........<br>....... | .................<br>...V.........F. | ...T...............<br>.........F... | ......<br>.... |
| iPS:43 3995 | 21-225_47H7 | VK1\|A30/JK3 | ............<br>........... | .T.........<br>........ | ...K....<br>....... | ........<br>....... | .................<br>...V.........F. | ...T...............<br>.........F... | ......<br>.... |
| iPS:43 4039 | 21-225_43B1 | VK1\|A30/JK3 | ............<br>........... | ...........<br>........ | ........<br>....... | ........<br>....... | .................<br>...V.........F. | ...T...............<br>.........F... | ......<br>.... |
| iPS:43 4057 | 21-225_51E4 | VK1\|A30/JK3 | ............<br>........... | ...........<br>........ | ........<br>..Q.... | ........<br>....... | .................<br>............A... | ...................<br>.............. | ......<br>.... |
| iPS:43 4071 | 21-225_51F9 | VK1\|A30/JK3 | ............<br>........... | ...........<br>....T... | ......R.<br>..Q.... | ........<br>......R | ...............D.<br>.............S... | ...................<br>.............. | ......<br>.... |
| iPS:43 4075 | 21-225_51B11 | VK1\|A30/JK3 | A...........<br>........... | .T.........<br>........ | ......R.<br>....... | ........<br>....... | .................<br>................ | ...................<br>.............. | ......<br>.... |
| iPS:43 4091 | 21-225_52B9 | VK1\|A30/JK3 | A...........<br>........... | ...........<br>........ | ......R.<br>....... | ........<br>....... | ...L.............<br>....R........... | ...................<br>.............. | ......<br>.... |

| | | | | | | | | | |
|---|---|---|---|---|---|---|---|---|---|
| iPS:43 4101 | 21-225_52H12 | VK1\|A30/ JK3 | ............<br>........... | ...........<br>.....N.. | ........<br>....... | G.......<br>....... | .................<br>................. | ..Y...............<br>.............. | ......<br>.... |
| iPS:43 4103 | 21-225_53G1 | VK1\|A30/ JK3 | ............<br>........... | ...........<br>........ | ........<br>....... | P.......<br>....... | .................<br>S................ | ..D...............<br>.............. | ......<br>.... |
| iPS:43 4129 | 21-225_53B12 | VK1\|A30/ JK3 | ............<br>........... | ...........<br>........ | ........<br>....... | ........<br>....... | .................<br>................. | ..................<br>.............. | ......<br>.... |
| iPS:43 4131 | 21-225_54D3 | VK1\|A30/ JK3 | ............<br>........... | ...........<br>........ | ........<br>....... | ........<br>....... | .................<br>................. | ..................<br>.............. | ......<br>.... |
| iPS:43 4143 | 21-225_54G7 | VK1\|A30/ JK3 | ............<br>........... | ...........<br>........ | ........<br>....... | ........<br>....... | .................<br>................. | .H..T.............<br>.............. | ......<br>.... |
| iPS:43 4155 | 21-225_55B3 | VK1\|A30/ JK3 | ............<br>........... | ...........<br>........ | ........<br>....... | ........<br>....... | .........R.......<br>.............S... | ..................<br>.............. | ......<br>.... |
| iPS:43 4169 | 21-225_50C4 | VK1\|A30/ JK3 | ............<br>........L.. | ...........<br>........ | .......I<br>....... | ........<br>....... | .................<br>................. | ....R.............<br>.............. | ......<br>.... |
| iPS:43 4187 | 21-225_56A5 | VK1\|A30/ JK3 | ............<br>........... | ......D....<br>.....L.. | ........<br>....... | ........<br>....... | .................<br>................. | ..Y...............<br>.............. | ......<br>...R |
| iPS:43 4199 | 21-225_59F11 | VK1\|A30/ JK3 | ............<br>........... | ...........<br>........ | ........<br>....... | ........<br>....... | .................<br>................. | ....R.............<br>.............. | ......<br>..F. |
| iPS:43 4207 | 21-225_60A3 | VK1\|A30/ JK3 | ............<br>........... | ...........<br>........ | ........<br>....... | ........<br>..F.... | .................<br>................. | ..................<br>.............. | ......<br>.... |
| iPS:43 4251 | 21-225_62G3 | VK1\|A30/ JK3 | ..H.........<br>........... | ......D....<br>.....N.. | ........<br>....... | T.......<br>....... | .................<br>................. | ..Y...............<br>.............. | ......<br>.... |
| iPS:43 4263 | 21-225_56H7 | VK1\|A30/ JK3 | ...L........<br>........... | ......D....<br>........ | ....R...<br>....... | P.......<br>.....L. | .................<br>................. | ..D...............<br>.............. | ......<br>..S. |
| iPS:43 4265 | 21-225_57B2 | VK1\|A30/ JK3 | ............<br>V.......... | ...........<br>.....A.. | ........<br>....... | ........<br>....... | .................<br>................. | ..................<br>.............. | ......<br>.... |
| iPS:43 4271 | 21-225_57A4 | VK1\|A30/ JK3 | ............<br>........... | ...........<br>........ | ..L.....<br>....... | ........<br>.....L. | .................<br>................. | ..................<br>.............. | ......<br>.... |
| iPS:43 4275 | 21-225_57F4 | VK1\|A30/ JK3 | ............<br>........... | ......V....<br>........ | ........<br>....... | ........<br>....... | .................<br>...............H. | ..YG..............<br>..........F... | ......<br>.... |
| iPS:43 4293 | 21-225_58F5 | VK1\|A30/ JK3 | ............<br>........... | ...........<br>........ | .FL.T...<br>....... | ........<br>.....L. | ......G..........<br>................. | ..................<br>.............. | ......<br>.E.. |
| iPS:43 4299 | 21-225_58D11 | VK1\|A30/ JK3 | ............<br>........... | ...........<br>....S..D | ........<br>....... | ........<br>....... | .................<br>..A....R......... | ....N.............<br>..........F... | ......<br>.... |
| iPS:43 4351 | 21-225_64A12 | VK1\|A30/ JK3 | ............<br>........... | ...........<br>........ | ........<br>..T.... | T.......<br>...T... | .................<br>................. | ....G.............<br>.............. | ......<br>.... |
| iPS:43 4383 | 21-225_66F9 | VK1\|A30/ JK3 | ............<br>........... | ......D....<br>.....V.. | ........<br>....... | T.......<br>....... | ....G............<br>................. | ..Y...............<br>.............. | ......<br>.... |
| iPS:43 4399 | 21-225_67B7 | VK1\|A30/ JK3 | ............<br>........... | ...........<br>........ | .F......<br>....... | ........<br>....... | .................<br>S................ | .H................<br>.............K | ......<br>.... |
| iPS:43 4407 | 21-225_68G8 | VK1\|A30/ JK3 | ............<br>........... | ...........<br>.....N.. | ....R...<br>....... | ........<br>....V.. | .................<br>................. | ..Y...............<br>.............. | ......<br>.... |

| | | | | | | | | | |
|---|---|---|---|---|---|---|---|---|---|
| iPS:43 4447 | 21-225_71B6 | VK1\|A30/ JK3 | ..........P.<br>........... | ...........<br>.......D | ........<br>..Q.... | ........<br>....... | .................<br>.........D....... | ..................<br>.............. | ......<br>.... |
| iPS:43 4449 | 21-225_71H6 | VK1\|A30/ JK3 | ............<br>........... | ...........<br>.....V.. | ........<br>....... | ........<br>....... | .................<br>................. | ..Y...............<br>.............. | ......<br>.... |
| iPS:43 4453 | 21-225_71B11 | VK1\|A30/ JK3 | ............<br>........... | ...........<br>.......D | ....T...<br>....... | ........<br>....... | ........G........<br>S...........T.... | ....T.............<br>.............. | ......<br>..V. |
| iPS:43 4463 | 21-225_73A6 | VK1\|A30/ JK3 | ............<br>........... | ...........<br>........ | .F......<br>....... | ........<br>...N... | .................<br>................. | ..................<br>.............. | ......<br>.... |
| iPS:43 4815 | 21-225_74A11 | VK1\|A30/ JK3 | ............<br>........... | ......D....<br>........ | ........<br>......C | ........<br>....... | .................<br>...............F. | ....D.............<br>.............. | ......<br>.... |
| iPS:43 4977 | 21-225_88A5 | VK1\|A30/ JK3 | ............<br>........... | ...........<br>........ | ........<br>....... | ........<br>....... | .................<br>................. | ....D.............<br>.............. | ......<br>.E.. |
| iPS:43 5253 | 21-225_96A4 | VK1\|A30/ JK3 | ............<br>........... | ......D....<br>........ | ........<br>....... | G.......<br>.V..... | .................<br>........R........ | ....D.............<br>.............. | ..R...<br>.... |
| iPS:43 5511 | 21-225_157C3 | VK1\|A30/ JK3 | ............<br>........... | ...........<br>........ | ........<br>....... | ........<br>....... | ...............D.<br>.........D....... | ..................<br>.............. | ......<br>.... |
| iPS:43 5521 | 21-225_157H4 | VK1\|A30/ JK3 | ............<br>........... | ...........<br>........ | ........<br>....... | P.......<br>......T | .................<br>................. | ..D...............<br>.............. | ......<br>.... |
| iPS:43 5527 | 21-225_157G7 | VK1\|A30/ JK3 | ...........C<br>........... | ...........<br>........ | ........<br>......N | V.......<br>....... | .................<br>......V.R........ | I.D...............<br>..........H... | ......<br>.E.. |
| iPS:43 5533 | 21-225_157H8 | VK1\|A30/ JK3 | ............<br>........... | ...........<br>........ | ........<br>....... | ........<br>....... | ...............D.<br>.........D....... | ..................<br>.............. | ......<br>.... |
| iPS:43 5537 | 21-225_157H12 | VK1\|A30/ JK3 | ............<br>........... | ...........<br>......F. | ....R...<br>...C..H | ........<br>....... | .................<br>................. | ...Y..............<br>.............. | ......<br>.... |
| iPS:43 5547 | 21-225_158F5 | VK1\|A30/ JK3 | ............<br>........... | ...........<br>........ | ........<br>......N | ........<br>....... | .................<br>................. | ..D...............<br>..........H... | ......<br>.E.. |
| iPS:43 5551 | 21-225_158H6 | VK1\|A30/ JK3 | ............<br>........... | ...........<br>....S... | ........<br>....... | T.......<br>....... | .................<br>................. | ..................<br>.............. | ......<br>.... |
| iPS:43 5553 | 21-225_158G8 | VK1\|A30/ JK3 | ............<br>........... | ......D....<br>........ | ........<br>.....M. | T.......<br>....... | .................<br>................. | ..................<br>.............. | ......<br>.... |
| iPS:43 5569 | 21-225_159C5 | VK1\|A30/ JK3 | ............<br>........... | .T.........<br>........ | ........<br>....... | ........<br>....... | ...............D.<br>....I....D....... | ..................<br>.............. | ......<br>.... |
| iPS:43 5593 | 21-225_160F4 | VK1\|A30/ JK3 | ..........P.<br>........... | ...........<br>........ | ........<br>......N | V.......<br>....... | .................<br>......V.......... | I.D...............<br>..........H... | ......<br>.E.. |
| iPS:43 5609 | 21-225_161F7 | VK1\|A30/ JK3 | ............<br>........... | ...........<br>........ | .......E<br>....... | ........<br>...T... | ..............A..<br>.V..G.V.R.......G | .LYIR.............<br>.............. | ..R...<br>.... |
| iPS:43 5613 | 21-225_161D11 | VK1\|A30/ JK3 | ............<br>........... | ...........<br>........ | .......E<br>....... | ........<br>...T... | ..............A..<br>....G.V.R.......G | ..Y.R.............<br>.............. | ..R...<br>.... |
| iPS:43 5617 | 21-225_162F2 | VK1\|A30/ JK3 | ...........C<br>........... | ...........<br>........ | ........<br>......N | ........<br>....... | .................<br>......V.......... | I.D...............<br>..........H... | ......<br>.E.. |
| iPS:43 5621 | 21-225_162H3 | VK1\|A30/ JK3 | ............<br>........... | ...........<br>........ | ........<br>......N | ........<br>....... | .................<br>................. | ..D...............<br>..........H... | ......<br>.E.. |

| | | | | | | | | | |
|---|---|---|---|---|---|---|---|---|---|
| iPS:43 5637 | 21-225_163E2 | VK1\|A30/ JK3 | ............<br>........... | ......D....<br>........ | ........<br>..T.... | P.......<br>....... | ..................<br>..S.............. | ....................<br>.........H... | ......<br>.... |
| iPS:43 5641 | 21-225_163F9 | VK1\|A30/ JK3 | ............<br>........... | ......D....<br>....D... | ........<br>....... | P.......<br>....... | ..................<br>.............A... | ..D.................<br>.............. | ......<br>.... |
| iPS:43 5643 | 21-225_163G10 | VK1\|A30/ JK3 | ............<br>........... | ......D....<br>.....N.. | ........<br>....... | P.......<br>....... | ..................<br>.................. | ..DY................<br>.............. | ......<br>.... |
| iPS:43 5719 | 21-225_171A11 | VK1\|A30/ JK3 | ............<br>........... | ...........<br>.....N.. | ........<br>....... | P.......<br>....... | ..................<br>.................. | ..DH................<br>.............. | ......<br>.... |
| iPS:43 5769 | 21-225_177B6 | VK1\|A30/ JK3 | ............<br>........... | ......D.S..<br>........ | ........<br>....... | ........<br>....... | ..................<br>....N............. | ..L.................<br>.............. | ......<br>.... |
| iPS:43 5791 | 21-225_180H7 | VK1\|A30/ JK3 | ............<br>........... | ...........<br>........ | ........<br>....... | T.......<br>....... | ..................<br>.............S... | ....................<br>.............. | ......<br>..F. |
| iPS:43 5805 | 21-225_181A8 | VK1\|A30/ JK3 | ............<br>........... | ...........<br>........ | ........<br>....... | T.......<br>....... | ...............D.<br>.............S... | ....................<br>.............. | ......<br>..F. |
| iPS:43 5879 | 21-225_184H10 | VK1\|A30/ JK3 | ............<br>........... | ...........<br>........ | ........<br>....... | I.......<br>....... | ..................<br>.................. | ....................<br>.............. | ......<br>.... |
| iPS:43 5881 | 21-225_184D11 | VK1\|A30/ JK3 | ............<br>........... | ...........<br>........ | ........<br>....... | I.......<br>....... | ..................<br>.................. | ....................<br>.............. | ......<br>.... |
| iPS:43 5921 | 21-225_190D6 | VK1\|A30/ JK3 | ............<br>........... | ...........<br>........ | ...L....<br>....... | ........<br>....... | ..............P..<br>.................. | ...Y................<br>.........F... | ......<br>.... |
| iPS:43 5985 | 21-225_192F6 | VK1\|A30/ JK3 | ............<br>........... | ...........<br>........ | ...L....<br>....... | ........<br>....... | ..............P..<br>.................. | ...Y................<br>.........F... | ......<br>.... |
| iPS:43 6074 | 21-225_194F10 | VK1\|A30/ JK3 | ............<br>........... | ...........<br>........ | ...L....<br>....... | ........<br>....... | ..............P..<br>.................. | ...Y................<br>.........F... | ......<br>.... |
| iPS:43 6092 | 21-225_195B9 | VK1\|A30/ JK3 | ............<br>........... | ...........<br>....K... | ........<br>....... | ........<br>...D... | ..................<br>...............C. | ...YR...............<br>.............. | ......<br>..F. |
| iPS:43 6164 | 21-225_197G10 | VK1\|A30/ JK3 | ............<br>........... | ...........<br>........ | ........<br>....... | G.......<br>....... | ..................<br>.................. | ...YR...............<br>.............. | ......<br>.... |
| iPS:43 6191 | 21-225_198B9 | VK1\|A30/ JK3 | ............<br>........... | ...........<br>....K... | ........<br>....... | ........<br>....... | ..................<br>...............V. | ...YR...............<br>.............. | ......<br>..F. |
| iPS:43 6205 | 21-225_199A7 | VK1\|A30/ JK3 | ............<br>........... | ...........<br>....K... | ........<br>....... | ........<br>....... | ..................<br>...............V. | ...YR...............<br>.............. | ......<br>..F. |
| iPS:43 6214 | 21-225_200F6 | VK1\|A30/ JK3 | ............<br>........... | ......D....<br>........ | ........<br>....... | ........<br>....... | ..................<br>...............H. | ...YR...............<br>.............. | ......<br>.... |
| iPS:43 6248 | 21-225_202A3 | VK1\|A30/ JK3 | ....S.......<br>........... | ...........<br>........ | ........<br>......C | ........<br>...R... | ..................<br>.............I... | ...HD...............<br>.............. | ......<br>.... |
| iPS:43 6268 | 21-225_203B9 | VK1\|A30/ JK3 | ............<br>........... | ...........<br>........ | ........<br>....... | R.......<br>....V.N | ..................<br>...............H. | ....................<br>.............. | ......<br>.... |
| iPS:43 6350 | 21-225_210E4 | VK1\|A30/ JK3 | ............<br>........... | ...........<br>........ | ........<br>....... | ........<br>....... | ..................<br>.................. | ....................<br>.............. | ......<br>.... |
| iPS:43 6576 | 21-225_225B6 | VK1\|A30/ JK3 | ............<br>.......I... | .......M...<br>....K... | ........<br>....... | ........<br>..T.... | ..................<br>.................. | ....................<br>.............. | ......<br>.... |

| | | | | | | | | | |
|---|---|---|---|---|---|---|---|---|---|
| iPS:43 6578 | 21-225_225D6 | VK1\|A30/ JK3 | ............<br>........... | ...........<br>........ | ........<br>....... | ........<br>....... | .................<br>...............C. | ....T.............<br>.............. | ......<br>.... |
| iPS:43 6582 | 21-225_225F8 | VK1\|A30/ JK3 | ............<br>........... | ...........<br>........ | ........<br>....... | ........<br>.....LG | .................<br>................. | ..................<br>.............. | ......<br>.... |
| iPS:43 6608 | 21-225_226A9 | VK1\|A30/ JK3 | ............<br>........... | ...........<br>........ | ........<br>....... | ........<br>....... | .................<br>................. | ..................<br>.............. | ......<br>.... |
| iPS:43 6630 | 21-225_227G3 | VK1\|A30/ JK3 | ............<br>........... | ...........<br>........ | ........<br>....... | ........<br>....... | .................<br>.........A....... | .H................<br>.............. | ......<br>.... |
| iPS:43 6634 | 21-225_227H5 | VK1\|A30/ JK3 | ............<br>........... | .....D.....<br>........ | ........<br>....... | ........<br>....... | .................<br>................. | ..................<br>.............. | ......<br>.... |
| iPS:43 6650 | 21-225_227C12 | VK1\|A30/ JK3 | ............<br>........... | ...........<br>........ | ........<br>....... | ........<br>....... | .................<br>................. | ..................<br>.............. | ......<br>.... |
| iPS:39 2632 | 21-225_16A11 | VK1\|A30/ JK3 | ............<br>.....S...S. | .....D.....<br>.....H.. | ...RN...<br>....... | ........<br>....... | .................<br>................. | ..Y...............<br>.............. | ......<br>.... |
| iPS:39 2684 | 21-225_17F4 | VK1\|A30/ JK3 | ............<br>........... | ...........<br>........ | ........<br>....... | ........<br>....... | .................<br>................. | ..................<br>.............. | ......<br>.... |
| iPS:39 2732 | 21-225_17E5 | VK1\|A30/ JK3 | ............<br>........... | ...........<br>........ | ........<br>....... | T.......<br>....... | .................<br>................. | ..................<br>............L. | ......<br>.V.. |
| iPS:39 2778 | 21-225_22H3 | VK1\|A30/ JK3 | ............<br>........... | .....D.....<br>.....N.. | ........<br>....... | P.......<br>......T | .................<br>...............F. | ..D...............<br>.............. | ......<br>.... |
| iPS:39 2912 | 21-225_25A9 | VK1\|A30/ JK3 | ............<br>........... | ...........<br>........ | ........<br>....... | ........<br>....... | .................<br>................. | ..................<br>.............. | ......<br>.... |
| iPS:39 2934 | 21-225_27D5 | VK1\|A30/ JK3 | ............<br>........... | ...........<br>........ | ........<br>....... | ........<br>....... | .................<br>................. | ..................<br>.............. | ......<br>.... |
| iPS:39 2940 | 21-225_29D9 | VK1\|A30/ JK3 | ............<br>........... | ...........<br>........ | .F......<br>....... | ........<br>....... | .................<br>................. | ....T.............<br>.............. | ......<br>..F. |
| iPS:39 2948 | 21-225_25G5 | VK1\|A30/ JK3 | ............<br>........... | .....D.....<br>........ | ........<br>....... | ........<br>....... | .................<br>...V............. | ..................<br>.............. | ......<br>.... |
| iPS:39 2968 | 21-225_25B6 | VK1\|A30/ JK3 | ............<br>........... | ...........<br>........ | ........<br>....... | R.......<br>....... | .................<br>................. | ..................<br>.............. | ......<br>.... |
| iPS:39 2978 | 21-225_28B8 | VK1\|A30/ JK3 | ............<br>........... | ...........<br>........ | ...H....<br>....... | ........<br>....... | .................<br>................. | ..................<br>.............. | ......<br>...N |
| iPS:39 2998 | 21-225_28A9 | VK1\|A30/ JK3 | ............<br>........... | ...........<br>........ | ........<br>....... | ........<br>......N | .................<br>................. | ....R.............<br>.............. | ......<br>.... |
| iPS:39 3000 | 21-225_29D7 | VK1\|A30/ JK3 | ............<br>........... | ...........<br>........ | ........<br>....... | ........<br>....... | .................<br>................. | ..................<br>.............. | ......<br>.... |
| iPS:39 3006 | 21-225_31G9 | VK1\|A30/ JK3 | ............<br>..I........ | ...........<br>........ | ........<br>....... | P.......<br>....... | .................<br>................. | ..D...............<br>.............. | ......<br>.... |
| iPS:39 3022 | 21-225_30H11 | VK1\|A30/ JK3 | ............<br>........... | ...........<br>........ | ........<br>.....V. | P.......<br>....... | .................<br>................. | ..D...............<br>..........H... | ......<br>...T |
| iPS:39 3038 | 21-225_29D8 | VK1\|A30/ JK3 | ............<br>........... | ...........<br>........ | ........<br>....... | T.......<br>....... | .................<br>................. | ..................<br>.............. | ......<br>.... |

| | | | | | | | | | |
|---|---|---|---|---|---|---|---|---|---|
| iPS:39 3822 | 21-225_15B11 | VK1\|A30/JK3 | ............<br>........... | ...........<br>........ | ........<br>....... | ........<br>....... | ................<br>................ | ..................<br>............... | ......<br>.... |
| iPS:39 3944 | 21-225_14D6 | VK1\|A30/JK3 | ............<br>.....S..... | ......D....<br>.....H.. | ...H....<br>....... | ........<br>....... | ................<br>................ | ..Y...............<br>............... | ......<br>...N |
| iPS:39 4033 | 21-225_5F4 | VK1\|A30/JK3 | ............<br>........... | ...........<br>.....H.. | ........<br>....... | ........<br>....... | ................<br>...V............ | ..Y.G.............<br>............... | ......<br>.... |
| iPS:39 4069 | 21-225_16H1 | VK1\|A30/JK3 | ............<br>........... | ...........<br>....DI.. | ........<br>....... | ........<br>......N | ................<br>................ | ..YH..............<br>............... | ......<br>..V. |
| iPS:40 2229 | 21-225_16H9 | VK1\|A30/JK3 | ............<br>........... | ...........<br>.....Y.. | .F......<br>....... | G.......<br>....... | .....I..........<br>................ | ..YH..............<br>...........L.. | ......<br>.... |
| | | Germline | K_FR1 | K_CDR1 | K_FR2 | K_CDR2 | K_FR3 | K_CDR3 | K_FR4 |
| | VK1\|O12/JK1 | | DIQMTQSPSSLS<br>ASVGDRVTITC | RAS--QSIS--<br>----SYLN | WYQQKPGK<br>APKLLIY | A-------<br>-ASSLQS | GVPSRFSGSGSG--<br>TDFTLTISSLQPEDFAT<br>YYC | QQSYS-------------<br>----------TPWT | FGQGTK<br>VEIK |
| iPS:46 8848 | 21-225_54B1 | VK1\|O12/JK1 | ............<br>..I........ | ......N....<br>........ | ........<br>...F... | ........<br>.....H. | ...P............<br>.............I... | ....R.............<br>.........TPL.. | ......<br>.... |
| iPS:43 4239 | 21-225_58F1 | VK1\|O12/JK1 | ............<br>........... | ........T..<br>....NF.. | ........<br>......F | ........<br>....... | .I..............<br>................ | ..................<br>..........I... | ......<br>.... |
| iPS:43 5513 | 21-225_157F3 | VK1\|O12/JK1 | ............<br>........... | ......N....<br>........ | ...L....<br>....... | T.......<br>....... | ................<br>................ | ....N.............<br>.........TPT.. | ......<br>.... |
| iPS:43 5729 | 21-225_173E7 | VK1\|O12/JK1 | ..........R.<br>.CI...A...Y | ......T....<br>....N... | ........<br>....... | ........<br>......I | ................<br>......V.......F. | ....R.............<br>.........TPQ.. | ......<br>.... |
| iPS:43 5753 | 21-225_175G10 | VK1\|O12/JK1 | ............<br>.......I... | ......T.G..<br>....N... | .......R<br>....... | ........<br>.....H. | ....G...........<br>..............F. | ....R.............<br>.........TPQ.. | ......<br>.... |
| iPS:43 5799 | 21-225_181G3 | VK1\|O12/JK1 | ............<br>.......N... | .....H.....<br>....N... | .....A..<br>..N.... | T.......<br>.TLN... | ................<br>................ | ..................<br>.........SP... | ......<br>.... |
| iPS:43 5813 | 21-225_183A12 | VK1\|O12/JK1 | ...........C<br>........... | .....RN....<br>....N... | ........<br>....... | V.......<br>.V..... | ................<br>................ | ..................<br>.........SP... | ......<br>.D.R |
| iPS:43 6003 | 21-225_192G10 | VK1\|O12/JK1 | ............<br>........... | ...........<br>....N... | ....T...<br>....... | ........<br>.E..... | ................<br>...............S. | ..................<br>.........SP... | ......<br>.... |
| iPS:43 6212 | 21-225_200G1 | VK1\|O12/JK1 | ............<br>........... | ...........<br>........ | .F......<br>....... | ........<br>.E..... | ................<br>...............S. | ..................<br>.........SP... | ......<br>..F. |
| iPS:39 2730 | 21-225_17A1 | VK1\|O12/JK1 | ............<br>........... | ......N.N..<br>....N... | ........<br>G..V..F | T.......<br>.T..... | ................<br>................ | ....T.............<br>.........TPT.. | ......<br>.... |
| iPS:39 2736 | 21-225_17B12 | VK1\|O12/JK1 | ............<br>.......S... | ......N.N..<br>....N... | ........<br>G..V..L | T.......<br>....... | ................<br>................ | ..T.T.............<br>.........TPT.. | ......<br>.... |
| iPS:39 2766 | 21-225_23H4 | VK1\|O12/JK1 | ............<br>........... | ...........<br>....R... | .......R<br>......C | S.......<br>.T..... | ................<br>................ | ..................<br>.........TPT.. | ......<br>...R |
| iPS:39 2770 | 21-225_20C10 | VK1\|O12/JK1 | ..H.........<br>.....K.S... | .....HH....<br>....N... | ........<br>G..V..L | T.......<br>....... | ................<br>................ | ....T.............<br>.........TPT.. | ......<br>.... |
| iPS:39 2808 | 21-225_20F8 | VK1\|O12/JK1 | ............<br>........... | ...........<br>....R... | ........<br>..E.... | ........<br>....... | ................<br>I............... | ....N.............<br>.........TPT.. | ......<br>.... |

| | | | | | | | | | |
|---|---|---|---|---|---|---|---|---|---|
| iPS:39 2954 | 21-225_26A10 | VK1\|O12/JK1 | ............<br>......I.... | ...........<br>........ | ........<br>...V... | ........<br>....... | .................<br>................ | ...................<br>.........TPT.. | ......<br>.... |
| iPS:39 3878 | 21-225_7G12 | VK1\|O12/JK1 | ............<br>.......S... | ......N.N..<br>....N... | ........<br>G..V..L | T.......<br>....... | .................<br>....N........... | ....T..............<br>.........TPT.. | ......<br>.... |
| iPS:39 8474 | 21-225_17B10 | VK1\|O12/JK1 | ............<br>........... | .S.....N..<br>........ | ........<br>......F | ........<br>.....H. | .................<br>................ | ..G.N..............<br>.........TPT.. | ......<br>...N |
| | | Germline | K_FR1 | K_CDR1 | K_FR2 | K_CDR2 | K_FR3 | K_CDR3 | K_FR4 |
| | VK4\|B3/JK2 | | DIVMTQSPDSLA<br>VSLGERATINC | KSS--<br>QSVLYSSNNKN<br>YLA | WYQQKPGQ<br>PPKLLIY | W-------<br>-ASTRES | GVPDRFSGSGSG--<br>TDFTLTISSLQAEDVAV<br>YYC | QQYYS------------<br>----------TPYT | FGQGTK<br>LEIK |
| iPS:46 8850 | 21-225_63F4 | VK4\|B3/JK2 | ............<br>........... | .........S.<br>...N.... | ........<br>....... | ........<br>....... | .I...........G.<br>................ | ....T..............<br>............CS | ......<br>...N |
| iPS:46 8852 | 21-225_71F3 | VK4\|B3/JK2 | ............<br>....A...... | .........SN<br>...N.... | ........<br>....... | ........<br>....... | .I...............<br>....N........... | ....T..............<br>............CS | ......<br>.... |
| iPS:46 8870 | 21-225_74A8 | VK4\|B3/JK2 | ............<br>........... | ...........<br>..SH.... | ........<br>....... | ........<br>....... | .................<br>................ | ...................<br>............CS | ......<br>.... |
| iPS:43 4211 | 21-225_60F3 | VK4\|B3/JK2 | ...........T<br>........... | ...........<br>........ | ........<br>....... | ........<br>....... | .................<br>.............I... | ...................<br>............CS | ......<br>...N |
| iPS:43 4235 | 21-225_61E3 | VK4\|B3/JK2 | ............<br>........... | .........HI<br>...N.... | ....Q...<br>....... | ........<br>...I... | .................<br>................ | ...................<br>..........I.CS | ......<br>.... |
| iPS:43 4287 | 21-225_57F12 | VK4\|B3/JK2 | ............<br>........... | .........F.<br>...Y.... | .....T..<br>....I.. | ........<br>....... | .................<br>.............I... | ...................<br>..........N.CS | ......<br>.... |
| iPS:43 4305 | 21-225_59E1 | VK4\|B3/JK2 | ............<br>........... | ...........<br>...N.... | ....R...<br>....... | ........<br>.S..... | .................<br>................ | ...F...............<br>..........I.CS | ......<br>.... |
| iPS:43 4443 | 21-225_71G3 | VK4\|B3/JK2 | ............<br>......VA... | .........H.<br>...N...D | ........<br>L.....F | ........<br>......F | ..........F......<br>.............D... | ....I..............<br>............CS | ......<br>.... |
| iPS:43 4483 | 21-225_74C12 | VK4\|B3/JK2 | ............<br>........... | ...........<br>...A.... | ........<br>..N.... | ........<br>....... | .................<br>................ | ...................<br>............CS | ......<br>.... |
| iPS:43 4613 | 21-225_77D12 | VK4\|B3/JK2 | ............<br>....A...... | R..........<br>...Y.... | ........<br>....... | ........<br>.....D. | .................<br>................ | ....T..............<br>............CS | ......<br>.... |
| iPS:43 4635 | 21-225_78E6 | VK4\|B3/JK2 | ............<br>........... | ...........<br>..SH.... | ........<br>....... | ........<br>...I... | .................<br>..S............. | ...................<br>............CS | ......<br>.... |
| iPS:43 4679 | 21-225_79G7 | VK4\|B3/JK2 | ............<br>........... | ...........<br>..SH.... | ........<br>......F | ........<br>...I... | .................<br>..S...M......... | ...................<br>............CS | ......<br>.... |
| iPS:43 4909 | 21-225_85C11 | VK4\|B3/JK2 | ............<br>........... | ...........<br>..SH.F.. | ....N...<br>......F | ........<br>..FI... | ...EG........A..<br>..S..G.......... | ...................<br>............CS | ......<br>.... |
| iPS:43 4959 | 21-225_87E10 | VK4\|B3/JK2 | ............<br>.........K. | .........H.<br>...M.... | ........<br>....... | ........<br>.....K. | ........T........<br>................ | ...................<br>..........S.CS | ......<br>.K.. |
| iPS:43 5299 | 21-225_146D4 | VK4\|B3/JK2 | ............<br>........... | ...........<br>...N.... | ........<br>...V... | ........<br>....... | .................<br>................ | ...................<br>............CS | ......<br>.... |
| iPS:43 5305 | 21-225_146C9 | VK4\|B3/JK2 | ............<br>........... | .........H.<br>...Y.... | ........<br>...V... | ........<br>.....K. | .................<br>................ | ...................<br>............CS | ......<br>.... |

| | | | | | | | | | |
|---|---|---|---|---|---|---|---|---|---|
| iPS:43 5309 | 21-225_146F9 | VK4\|B3/J K2 | ............<br>........... | ......NI.H.<br>...N.... | ........<br>..Y.... | ........<br>....... | ..................<br>....T............ | ....T..............<br>............CS | ......<br>...T |
| iPS:43 5323 | 21-225_147D5 | VK4\|B3/J K2 | ............<br>........... | ...........<br>...N.... | ........<br>....... | ........<br>....... | ..................<br>..S.............. | H..................<br>............CS | ......<br>.... |
| iPS:43 5399 | 21-225_150D2 | VK4\|B3/J K2 | ............<br>.........K. | ..........R<br>..S.K..T | ........<br>....F.. | ........<br>.....K. | ..................<br>....N........F. | ...F...............<br>.............N | ......<br>R... |
| iPS:43 5435 | 21-225_152H3 | VK4\|B3/J K2 | ............<br>.....K..... | .........H.<br>...Y...T | ........<br>....... | ........<br>.....K. | ..................<br>................. | ...................<br>............CS | ......<br>.... |
| iPS:43 5451 | 21-225_152D10 | VK4\|B3/J K2 | G...........<br>....A....D. | ...........<br>........ | ........<br>....... | ........<br>....... | ..................<br>....Y............ | ....-<br>...................<br>.....RSPS | ......<br>.... |
| iPS:43 5459 | 21-225_152E12 | VK4\|B3/J K2 | AV..........<br>........... | T......I.H.<br>...Y.... | ........<br>....... | ........<br>....... | ..................<br>................. | ...................<br>..........G.CS | ......<br>.... |
| iPS:43 5467 | 21-225_153B9 | VK4\|B3/J K2 | G.....F.....<br>........... | ...........<br>........ | ........<br>.H..... | ........<br>......F | ..........C......<br>....Y.V.......... | ...N-<br>...................<br>.....RSLS | ......<br>.... |
| iPS:43 5471 | 21-225_153F11 | VK4\|B3/J K2 | ............<br>........... | ...........<br>...YK... | ........<br>..N.... | ........<br>.....K. | ..................<br>................. | ...................<br>............CS | ......<br>.... |
| iPS:43 5475 | 21-225_154H6 | VK4\|B3/J K2 | ............<br>........... | .........H.<br>...Y.... | ........<br>....... | ........<br>.T...K. | ...............H.<br>..S.............. | .H.................<br>............CS | ......<br>.... |
| iPS:43 5491 | 21-225_155E5 | VK4\|B3/J K2 | ............<br>........... | .........S.<br>...N.... | ........<br>....... | ........<br>.....K. | ..................<br>................. | ...................<br>............CS | ......<br>.... |
| iPS:43 5495 | 21-225_155B6 | VK4\|B3/J K2 | ............<br>........... | .........H.<br>...Y.... | ........<br>....... | ........<br>.T..... | ..................<br>................. | ...................<br>............CS | ......<br>.... |
| iPS:43 5501 | 21-225_156H1 | VK4\|B3/J K2 | ............<br>........... | ...........<br>...N.... | ........<br>....... | ........<br>....... | ...A..............<br>................. | H..................<br>............CS | ......<br>.... |
| iPS:43 5589 | 21-225_160A4 | VK4\|B3/J K2 | ............<br>........... | .........H.<br>...N.... | ........<br>....... | ........<br>....... | ..................<br>................. | ....N..............<br>..........S.CS | ......<br>.... |
| iPS:43 5727 | 21-225_172E11 | VK4\|B3/J K2 | ............<br>........... | .........H.<br>...N.... | ........<br>....... | ........<br>....... | ..................<br>I....G........... | ...FT..............<br>............CS | ......<br>.... |
| iPS:43 6560 | 21-225_224F11 | VK4\|B3/J K2 | N...........<br>...D....... | .........S.<br>...H.... | ........<br>...M... | ........<br>....... | ..................<br>................. | ....T..............<br>............CS | ......<br>.... |
| iPS:43 6584 | 21-225_225B9 | VK4\|B3/J K2 | ............<br>........... | ...........<br>...N.... | ........<br>......F | ........<br>....... | ..................<br>................. | .HSK...............<br>..........I.GK | ....I.<br>...Q |
| iPS:43 6588 | 21-225_225F12 | VK4\|B3/J K2 | ............<br>........... | ...........<br>...Q.... | ........<br>..R.... | ........<br>.T..... | ..................<br>....N............ | ....I..............<br>............CS | ......<br>.... |
| iPS:43 6590 | 21-225_225H12 | VK4\|B3/J K2 | ............<br>........... | ..........N<br>...N.... | ........<br>..N.... | ........<br>....... | ..................<br>.......T......... | ....I..............<br>............CS | ......<br>.... |
| iPS:43 6598 | 21-225_226D6 | VK4\|B3/J K2 | ............<br>........... | R......I..I<br>........ | ........<br>...M... | ........<br>....... | ..................<br>................. | ...................<br>..........S.CS | ......<br>.... |
| iPS:43 6636 | 21-225_227E6 | VK4\|B3/J K2 | ...........T<br>........... | ...........<br>..D..... | ........<br>...M... | ........<br>....... | ..................<br>................. | ....I..............<br>............CS | ......<br>.... |

| | | | | | | | | | |
|---|---|---|---|---|---|---|---|---|---|
| iPS:43 6644 | 21-225_227G9 | VK4\|B3/JK2 | ............<br>........... | .........H.<br>...N.... | ........<br>....... | ........<br>.G..... | .................<br>................. | ..................<br>..........A..S | ......<br>.... |
| iPS:43 6646 | 21-225_227D11 | VK4\|B3/JK2 | ............<br>...D....... | .........H.<br>...N.... | ........<br>....... | ........<br>....... | .I...............<br>................. | ....N.............<br>............CS | ......<br>.... |
| iPS:43 7363 | 21-225_74C10 | VK4\|B3/JK2 | ............<br>........... | ...........<br>...A.... | ........<br>..N.... | ........<br>....... | .................<br>................. | ..................<br>............CS | ......<br>.... |
| iPS:45 1131 | 21-225_160A7 | VK4\|B3/JK2 | ...L......P.<br>........... | .........SN<br>.H.N.... | ....R..H<br>.H....F | ........<br>....... | ..........Y......<br>................. | ..................<br>............CS | ......<br>.... |
| iPS:39 3088 | 21-225_33D1 | VK4\|B3/JK2 | ...........S<br>........... | ..I......R<br>.......T | ........<br>.R..F.. | ........<br>....... | ..L......C.......<br>....I........L... | ..................<br>..........S.CS | ......<br>.... |
| iPS:39 4085 | 21-225_8B11 | VK4\|B3/JK2 | ............<br>........... | ......N...N<br>...N.... | ........<br>....... | ........<br>.....K. | .................<br>................. | ....T.............<br>............CS | ......<br>.... |
| iPS:39 8496 | 21-225_22D2 | VK4\|B3/JK2 | ............<br>.........T. | .........H.<br>...N.... | ........<br>....... | ........<br>.....K. | .................<br>...............F. | ..................<br>............CS | ......<br>.... |
| iPS:39 8512 | 21-225_25E12 | VK4\|B3/JK2 | ...L.....F..<br>M.......... | ..........H<br>...Y.... | ......K.<br>....... | ........<br>....... | ...............Y.<br>................. | ..................<br>............CS | .....N<br>.... |
| iPS:39 8522 | 21-225_32A1 | VK4\|B3/JK2 | ............<br>........... | ...........<br>...Y.... | ...L....<br>....... | ........<br>.....K. | .................<br>........T....L... | ....T.............<br>..........S.CS | ......<br>.... |
| iPS:39 8524 | 21-225_32A5 | VK4\|B3/JK2 | ............<br>........... | .........H.<br>........ | ........<br>....... | ........<br>....... | .................<br>..A.........L.H. | ..................<br>..........S.CS | .....G<br>.... |
| iPS:39 8538 | 21-225_34H7 | VK4\|B3/JK2 | ............<br>........... | ...........<br>...Y.... | ...L....<br>....... | ........<br>.....K. | .................<br>........T....L... | ....T.............<br>..........S.CS | ......<br>.... |
| | | **Germline** | **K_FR1** | **K_CDR1** | **K_FR2** | **K_CDR2** | **K_FR3** | **K_CDR3** | **K_FR4** |
| | VK2\|A17/JK4 | | DVVMTQSPLSLP<br>VTLGQPASISC | RSS--<br>QSLVYSD-<br>GNTYLN | WFQQRPGQ<br>SPRRLIY | K-------<br>-VSNRDS | GVPDRFSGSGSG--<br>TDFTLKISRVEAEDVGV<br>YYC | MQGTH------------<br>----------WPLT | FGGGTK<br>VEIK |
| iPS:46 8854 | 21-225_72C4 | VK2\|A17/JK4 | ............<br>........... | ..G.......<br>........ | ........<br>....... | E.......<br>...KW.. | ...............N.<br>..............F.. | ..................<br>.............. | ......<br>.... |
| iPS:43 7250 | 21-225_148C6 | VK2\|A17/JK4 | ............<br>........... | ...........<br>........ | ........<br>....... | ........<br>....W.. | .................<br>................. | ..................<br>...........S.. | ......<br>.... |
| iPS:43 7252 | 21-225_148H11 | VK2\|A17/JK4 | ............<br>........... | ...........<br>........ | ........<br>....... | ........<br>....W.. | .................<br>................. | ..................<br>...........L.. | ......<br>.... |
| iPS:43 7254 | 21-225_149F2 | VK2\|A17/JK4 | ............<br>........... | ...........<br>.....S.. | ........<br>....... | ........<br>....W.. | ...V.............<br>.............I... | ..................<br>............P. | ......<br>.... |
| iPS:43 7256 | 21-225_150F11 | VK2\|A17/JK4 | ....S.Y.....<br>..F........ | ...........<br>.....S.. | ........<br>Y...... | ........<br>....W.Y | ...V.............<br>.............I... | ..................<br>............P. | ......<br>.... |
| iPS:43 7268 | 21-225_177D2 | VK2\|A17/JK4 | ............<br>........... | ...........<br>........ | ........<br>....... | ........<br>....W.. | .................<br>................. | ..................<br>.............. | ......<br>.... |
| iPS:44 3005 | 21-225_43F11_LC1 | VK2\|A17/JK4 | ............<br>........... | ...........<br>........ | ........<br>....... | ........<br>....W.. | .................<br>................. | ..................<br>.............. | ......<br>.... |
| iPS:39 | [illegible] | VK2\|A17/ | ...........S | ........... | ........ | ........ | ................. | ...I- | ...... |

| | | Germline | K_FR1 | K_CDR1 | K_FR2 | K_CDR2 | K_FR3 | K_CDR3 | K_FR4 |
|---|---|---|---|---|---|---|---|---|---|
| | VK2\|A17/JK 3 | | DVVMTQSPLSLP<br>VTLGQPASISC | RSS--<br>QSLVYSD-<br>GNTYLN | WFQQRPGQ<br>SPRRLIY | K-------<br>-VSNRDS | GVPDRFSGSGSG--<br>TDFTLKISRVEAEDVGV<br>YYC | MQGTH------------<br>----------WPFT | FGPGTK<br>VDIK |
| iPS:46 8856 | 21-225_77C9 | VK2\|A17/ JK3 | ............<br>........... | ...........<br>V....S.S | ........<br>....... | ........<br>....W.. | .................<br>...............R | ...................<br>............... | ......<br>.... |
| iPS:39 2936 | 21-225_28B6 | VK2\|A17/ JK3 | ............<br>........... | ...........<br>...D.... | ........<br>....Q.. | ........<br>....W.. | .................<br>..N.........I.F. | .HC.-<br>...................<br>.....HWLL | ......<br>.... |
| | | Germline | K_FR1 | K_CDR1 | K_FR2 | K_CDR2 | K_FR3 | K_CDR3 | K_FR4 |
| | VK2\|A17/JK 5 | | DVVMTQSPLSLP<br>VTLGQPASISC | RSS--<br>QSLVYSD-<br>GNTYLN | WFQQRPGQ<br>SPRRLIY | K-------<br>-VSNRDS | GVPDRFSGSGSG--<br>TDFTLKISRVEAEDVGV<br>YYC | MQGTH------------<br>----------WPIT | FGQGTR<br>LEIK |
| iPS:47 2741 | 21-225_30D9_L C1 | VK2\|A17/ JK5 | ............<br>........... | .........S.<br>.....F.. | ........<br>....... | ........<br>....W.. | .................<br>......L......... | L..................<br>............L. | ......<br>.... |
| iPS:43 7294 | 21-225_216D5 | VK2\|A17/ JK5 | ............<br>........... | ...........<br>........ | ........<br>....... | ........<br>....W.. | .....I...........<br>.............I... | ...A-<br>...................<br>.....HWF. | ......<br>.... |
| iPS:47 2732 | 21-225_2B10_L C1 | VK2\|A17/ JK5 | ............<br>........... | ...........<br>.....F.. | ........<br>....... | ........<br>....W.. | ...............G.<br>................. | I..................<br>............FP | ......<br>.... |
| iPS:39 8508 | 21-225_24B1 | VK2\|A17/ JK5 | ............<br>........... | ...........<br>........ | ........<br>....... | ........<br>....W.. | .................<br>.....W........C.. | ...A...............<br>.........WP... | ......<br>.... |
| iPS:42 3019 | 21-225_31D12_ LC1 | VK2\|A17/ JK5 | ............<br>........... | ........I..<br>.....F.. | ........<br>....... | ........<br>....W.. | .................<br>......L......I... | ...................<br>............L. | ......<br>.... |
| | | Germline | K_FR1 | K_CDR1 | K_FR2 | K_CDR2 | K_FR3 | K_CDR3 | K_FR4 |
| | VK1\|L5/JK1 | | DIQMTQSPSSVS<br>ASVGDRVTITC | RAS--QGIS--<br>----SWLA | WYQQKPGK<br>APKLLIY | A-------<br>-ASSLQS | GVPSRFSGSGSG--<br>TDFTLTISSLQPEDFAT<br>YYC | QQANS------------<br>----------FPWT | FGQGTK<br>VEIK |
| iPS:43 3897 | 21-225_43C2 | VK1\|L5/J K1 | ............<br>........... | ...........<br>....D... | ........<br>..R.... | ........<br>....... | .................<br>.....N.......... | ..T................<br>.............. | ......<br>.... |
| iPS:43 3903 | 21-225_43H4 | VK1\|L5/J K1 | ............<br>........... | ........I..<br>....N... | ........<br>....... | ........<br>....... | .................<br>.....N.......... | ..T................<br>.............. | ......<br>.... |
| iPS:43 3911 | 21-225_43E8 | VK1\|L5/J K1 | ....N.......<br>........... | ...........<br>....N... | .......R<br>....... | ........<br>....... | .................<br>.....N.......... | ..T................<br>.............. | ......<br>.... |
| iPS:43 3941 | 21-225_44D10 | VK1\|L5/J K1 | ............<br>........... | ...........<br>....D... | ........<br>..R.... | ........<br>....... | ....K............<br>.....N.......... | ..T................<br>.............. | ......<br>.... |
| iPS:43 3945 | 21-225_44C12 | VK1\|L5/J K1 | ............<br>........... | ...........<br>....N... | ........<br>......S | ........<br>..F.... | .................<br>..S.............. | ..S................<br>.............. | ......<br>.... |
| iPS:43 3957 | 21-225_45F8 | VK1\|L5/J K1 | ............<br>........... | ...........<br>....D... | ........<br>..R.... | G.......<br>....... | .................<br>.....N.......... | ..T................<br>.............. | ......<br>.... |

| | | | | | | | | | |
|---|---|---|---|---|---|---|---|---|---|
| iPS:43 3973 | 21-225_46A6 | VK1\|L5/J K1 | ....N.......<br>.......N... | ...........<br>....N... | .........<br>V...... | .........<br>....... | .................<br>.....N........... | ..T................<br>.............. | ......<br>.... |
| iPS:43 3993 | 21-225_47G7 | VK1\|L5/J K1 | ............<br>........... | ...........<br>....N... | .........<br>......F | .........<br>...N... | .................<br>.........A.....C. | ..V................<br>.............. | ......<br>.... |
| iPS:43 4007 | 21-225_48D7 | VK1\|L5/J K1 | ............<br>........... | ......N.T..<br>........ | .........<br>....... | S........<br>......N | .................<br>...........V..C. | ...................<br>.............. | ......<br>.... |
| iPS:43 4063 | 21-225_51G7 | VK1\|L5/J K1 | ............<br>........... | ......D....<br>........ | .........<br>...V... | .........<br>.P.N... | A................<br>................. | ...H...............<br>.............. | ......<br>.... |
| iPS:43 4083 | 21-225_52H2 | VK1\|L5/J K1 | ............<br>........... | ......N.T..<br>....N... | .F.....R<br>....... | T........<br>.T..... | .................<br>...............C. | ..T................<br>.............. | ..H...<br>..V. |
| iPS:43 4133 | 21-225_54G3 | VK1\|L5/J K1 | ............<br>........... | ...........<br>........ | .........<br>....... | D........<br>....... | ...........E.....<br>................. | ...................<br>.............. | ......<br>.... |
| iPS:43 4221 | 21-225_60A11 | VK1\|L5/J K1 | ............<br>........... | ......V....<br>....N... | ...L.....<br>....... | T........<br>....... | ........NE.......<br>................. | ...................<br>.............. | ......<br>.... |
| iPS:43 4283 | 21-225_57F8 | VK1\|L5/J K1 | ............<br>........... | ...........<br>....N... | .........<br>....... | T........<br>....... | ........NE.......<br>................. | ...................<br>.............. | ......<br>.... |
| iPS:43 5711 | 21-225_171G4 | VK1\|L5/J K1 | ............<br>........... | .......VN..<br>....D... | ........R<br>....... | D........<br>....... | .................<br>................. | ...................<br>.............. | ......<br>.... |
| iPS:43 5715 | 21-225_171A8 | VK1\|L5/J K1 | A...........<br>........... | ...........<br>....N... | .........<br>..N.M.H | .........<br>..F...G | .................<br>................. | ..T................<br>.............. | ......<br>.... |
| iPS:43 5717 | 21-225_171A9 | VK1\|L5/J K1 | ............<br>........... | ......D.T..<br>....T... | .........<br>....... | D........<br>....... | A................<br>...V............. | L.T................<br>.............. | ......<br>.... |
| iPS:43 5739 | 21-225_174G7 | VK1\|L5/J K1 | A...........<br>........... | ...........<br>....N... | .........<br>..N...H | .........<br>..F...G | .................<br>................. | ..T................<br>.............. | ......<br>.... |
| iPS:43 5749 | 21-225_175C10 | VK1\|L5/J K1 | ............<br>........... | ........T..<br>....D... | .........<br>....... | .........<br>....... | .................<br>...F.....D....... | ..T................<br>.............. | ......<br>.... |
| iPS:43 5775 | 21-225_178A5 | VK1\|L5/J K1 | ............<br>........... | ...........<br>....N... | .........<br>....... | .........<br>....... | ......N..........<br>...............C. | ...................<br>..........L... | ......<br>.... |
| iPS:43 5777 | 21-225_178F7 | VK1\|L5/J K1 | ............<br>........... | ......D.T..<br>....D... | .........<br>......S | .........<br>....... | .................<br>..A.............. | ...................<br>..........L... | ......<br>.... |
| iPS:43 5783 | 21-225_179G1 | VK1\|L5/J K1 | ............<br>........... | ......D....<br>....D... | ......S..<br>......S | .........<br>....... | ......G..........<br>................. | ...................<br>..........L... | ......<br>.... |
| iPS:43 5875 | 21-225_190B9 | VK1\|L5/J K1 | ............<br>........... | ........N..<br>....N... | .........<br>....... | G........<br>.V..... | .................<br>...............S. | ...................<br>.............. | ......<br>...R |
| iPS:43 5895 | 21-225_188E8 | VK1\|L5/J K1 | ............<br>........... | ..N...D....<br>........ | .........<br>....... | .........<br>...N... | ....G............<br>................. | ...................<br>.............. | ......<br>.... |
| iPS:43 5909 | 21-225_190H3 | VK1\|L5/J K1 | ............<br>........... | .......LN..<br>....N... | ...L.....<br>....... | .........<br>.V..... | ...............SE.<br>...............H. | ...................<br>..........L... | ......<br>.... |
| iPS:43 6013 | 21-225_193F2 | VK1\|L5/J K1 | ............<br>........... | ........N..<br>....N... | .........<br>....... | G........<br>.V..... | .................<br>...............S. | ...................<br>.............. | ......<br>...R |
| iPS:43 6068 | 21-225_194F7 | VK1\|L5/J K1 | ............<br>........... | ...........<br>....R... | .F.......<br>...I... | .........<br>....... | .................<br>................. | ...................<br>.............. | ......<br>.... |

| | | | | | | | | | |
|---|---|---|---|---|---|---|---|---|---|
| iPS:43 6100 | 21-225_195G12 | VK1\|L5/J K1 | ............<br>........... | ........N..<br>....N... | ........<br>....... | G.......<br>.V..... | .................<br>...............S. | ..................<br>.............. | ..R...<br>..NQ |
| iPS:43 6116 | 21-225_196B9 | VK1\|L5/J K1 | ............<br>........... | ...........<br>....NC.. | ........<br>...F..C | ........<br>....... | A................<br>.............S... | ..GD..............<br>...........P. | ......<br>..FR |
| iPS:43 6160 | 21-225_197C9 | VK1\|L5/J K1 | ............<br>........... | ...........<br>....N... | .F......<br>..Q.... | ........<br>....... | .................<br>S................ | ..................<br>.............. | ......<br>.... |
| iPS:43 6546 | 21-225_224D6 | VK1\|L5/J K1 | ............<br>........... | ...........<br>........ | ........<br>....... | ........<br>....... | .................<br>................. | ..................<br>.............. | ..P...<br>.... |
| iPS:43 6632 | 21-225_227E4 | VK1\|L5/J K1 | ..L.........<br>........... | ........I..<br>....N... | ........<br>....... | ........<br>....... | .................<br>.............I... | ..................<br>.............. | ......<br>.... |
| iPS:39 2650 | 21-225_17A4 | VK1\|L5/J K1 | ............<br>........... | ........G..<br>....N... | ........<br>......F | ........<br>....... | .................<br>...........V.... | ..................<br>...........R. | ......<br>.... |
| iPS:39 2728 | 21-225_20F7 | VK1\|L5/J K1 | ............<br>.....G..... | ...........<br>........ | ........<br>....... | ........<br>....... | .................<br>................. | ..................<br>...........R. | ......<br>.... |
| iPS:39 2916 | 21-225_27C5 | VK1\|L5/J K1 | ............<br>........... | ...........<br>........ | ........<br>...F... | G.......<br>....... | .......A.......E.<br>...............C. | ..YD..............<br>...........R. | ......<br>.... |
| iPS:39 2956 | 21-225_27A11 | VK1\|L5/J K1 | ............<br>........... | ...........<br>........ | ........<br>....... | G.......<br>....... | .......A.........<br>...............C. | ..SD..............<br>...........R. | ......<br>.... |
| iPS:39 3014 | 21-225_26D12 | VK1\|L5/J K1 | ............<br>........... | ...........<br>........ | ........<br>....... | G.......<br>....... | .......A.........<br>...............C. | ..SD..............<br>...........R. | ......<br>.... |
| iPS:39 3028 | 21-225_25D7 | VK1\|L5/J K1 | ............<br>........F.. | ......D.F..<br>....D... | .......T<br>....... | ........<br>....... | ...............N.<br>...V.G........... | ...Y..............<br>.............. | ......<br>.... |
| iPS:39 3152 | 21-225_25B3 | VK1\|L5/J K1 | ............<br>........... | ...........<br>........ | ........<br>....... | G.......<br>....... | .......A.........<br>...............C. | ..SD..............<br>...........R. | ......<br>.... |
| iPS:39 3810 | 21-225_5A4 | VK1\|L5/J K1 | ............<br>........... | ...........<br>....T... | ........<br>....... | D.......<br>....... | .................<br>................. | ..................<br>.............. | ......<br>.... |
| | | Germline | K_FR1 | K_CDR1 | K_FR2 | K_CDR2 | K_FR3 | K_CDR3 | K_FR4 |
| | VK2\|A19/JK 3 | | DIVMTQSPLSLP<br>VTPGEPASISC | RSS--<br>QSLLHSN-<br>GYNYLD | WYLQKPGQ<br>SPQLLIY | L-------<br>-GSNRAS | GVPDRFSGSGSG--<br>TDFTLKISRVEAEDVGV<br>YYC | MQALQ-------------<br>----------TPFT | FGPGTK<br>VDIK |
| iPS:43 3943 | 21-225_44E10 | VK2\|A19/ JK3 | ............<br>........... | ...........<br>....S..E | ........<br>....... | ........<br>....... | .................<br>................. | ..T...............<br>.............. | ......<br>.... |
| iPS:43 3989 | 21-225_47C7 | VK2\|A19/ JK3 | ..........P.<br>........... | ...........<br>.......E | .....S..<br>..F.... | ........<br>..F.... | ......T..........<br>................. | ..V...............<br>.............. | ......<br>.... |
| | | Germline | K_FR1 | K_CDR1 | K_FR2 | K_CDR2 | K_FR3 | K_CDR3 | K_FR4 |
| | VK1\|O12/JK 3 | | DIQMTQSPSSLS<br>ASVGDRVTITC | RAS--QSIS--<br>----SYLN | WYQQKPGK<br>APKLLIY | A-------<br>-ASSLQS | GVPSRFSGSGSG--<br>TDFTLTISSLQPEDFAT<br>YYC | QQSYS-------------<br>----------TPFT | FGPGTK<br>VDIK |
| iPS:43 3999 | 21-225_48D1 | VK1\|O12/ JK3 | ............<br>.......N... | ...........<br>.......I | ........<br>....... | ........<br>....... | .......A.........<br>.............S... | ...N..............<br>.........I... | ......<br>.... |
| iPS:43 4003 | 21-225_48C3 | VK1\|O12/ JK3 | ............<br>.......N... | ........I..<br>.......I | ........<br>..R.... | ........<br>....... | .......A.........<br>.............S... | ..TN..............<br>.........I... | ......<br>.... |

| | | | | | | | | | |
|---|---|---|---|---|---|---|---|---|---|
| iPS:43 4037 | 21-225_49G12 | VK1\|O12/ JK3 | ............<br>........... | .S.........<br>....T..M | ........<br>....... | ........<br>......I | ....E..A.........<br>................. | ..................<br>..........I... | ......<br>.... |
| iPS:43 4041 | 21-225_50H8 | VK1\|O12/ JK3 | ............<br>.......N... | ...........<br>.......I | ........<br>....... | ........<br>....... | .......A.........<br>.............S... | ...N..............<br>..........L... | ......<br>.... |
| iPS:43 4045 | 21-225_50H10 | VK1\|O12/ JK3 | ............<br>.......N... | ........Y..<br>.......I | ........<br>....... | ........<br>....... | .......A.........<br>.............S... | ...N..............<br>..........I... | ......<br>.... |
| iPS:43 4049 | 21-225_50B12 | VK1\|O12/ JK3 | ............<br>........... | ...........<br>........ | ...H....<br>..R.... | ........<br>....... | .........E.......<br>I...........T.... | ....I.............<br>..........A... | ......<br>.... |
| iPS:43 4073 | 21-225_51H10 | VK1\|O12/ JK3 | ............<br>........... | ...........<br>....T..M | ........<br>....... | ........<br>....... | ....E..A.........<br>................. | ..................<br>..........I... | ......<br>.... |
| iPS:43 4107 | 21-225_53E2 | VK1\|O12/ JK3 | ............<br>........... | .......F...<br>....H... | ........<br>..N...F | ........<br>.V..... | ..............S..<br>..P..........I.F. | ...F..............<br>.............. | ......<br>.... |
| iPS:43 4181 | 21-225_56B2 | VK1\|O12/ JK3 | ............<br>........... | .......F...<br>....H... | ........<br>..N...F | ........<br>.V..... | .................<br>.............I.F. | ..................<br>.............. | ......<br>.... |
| iPS:43 4225 | 21-225_60E12 | VK1\|O12/ JK3 | ............<br>........... | ...........<br>........ | ........<br>....... | ........<br>....... | .................<br>.............P... | ....N.............<br>..........IS.. | ......<br>.... |
| iPS:43 4227 | 21-225_61A1 | VK1\|O12/ JK3 | ............<br>........... | ...........<br>........ | ........<br>....... | ........<br>....... | .................<br>.............P... | ....N.............<br>..........IS.. | ......<br>.... |
| iPS:43 4245 | 21-225_62H1 | VK1\|O12/ JK3 | ............<br>.Y......... | ......N.F..<br>........ | ........<br>...V... | ........<br>.VF.... | .................<br>................. | ..................<br>.............. | ......<br>.... |
| iPS:43 4267 | 21-225_57F2 | VK1\|O12/ JK3 | ............<br>........... | ...........<br>........ | ........<br>....... | ........<br>....... | .................<br>.............P... | ....N.............<br>..........IS.. | ......<br>.... |
| iPS:43 4323 | 21-225_62H8 | VK1\|O12/ JK3 | ............<br>........... | ........F..<br>........ | ....N...<br>....... | ........<br>.S..... | .....L..N........<br>I................ | ..................<br>.............. | .....R<br>.... |
| iPS:43 4379 | 21-225_66A9 | VK1\|O12/ JK3 | ............<br>........... | ......N.F..<br>........ | ........<br>......F | ........<br>....... | .................<br>................. | ..T...............<br>..........V... | ......<br>..F. |
| iPS:43 4417 | 21-225_69C8 | VK1\|O12/ JK3 | ............<br>........F.. | ..G...T.Y..<br>....N... | ........<br>......H | V.......<br>....... | .................<br>..V.............. | ..................<br>.............. | ......<br>.... |
| iPS:43 5545 | 21-225_158F4 | VK1\|O12/ JK3 | ...........P<br>........... | ......N.R..<br>....K..H | ...FL...<br>....... | T.......<br>...T... | .................<br>................. | ....N.............<br>..........IS.. | ......<br>.... |
| iPS:43 5793 | 21-225_180F8 | VK1\|O12/ JK3 | ............<br>.....G..... | ......T.L..<br>........ | ........<br>....... | G.......<br>.V..... | .................<br>S................ | ..................<br>.............. | ......<br>.... |
| iPS:43 6504 | 21-225_222H4 | VK1\|O12/ JK3 | .....H......<br>........... | ......N....<br>....N.V. | ........<br>...F... | T.......<br>....... | ..S..............<br>......VHRD...I... | ..Y.F.............<br>.............. | ..R...<br>.... |
| iPS:43 6510 | 21-225_222H8 | VK1\|O12/ JK3 | .....H......<br>........... | ......N....<br>....N.V. | ........<br>...F... | I.......<br>....... | ..S..............<br>......VHRD...I... | ..Y.F.............<br>.............. | ..R...<br>.... |
| iPS:43 7230 | 21-225_62H10 | VK1\|O12/ JK3 | ............<br>........... | ........T..<br>........ | ........<br>V.....S | T.......<br>....... | .................<br>................. | ...H..............<br>..........F... | .....N<br>..F. |
| iPS:44 8906 | 21-225_72G9 | VK1\|O12/ JK3 | ............<br>........... | ........T..<br>........ | ........<br>....... | T.......<br>....... | .................<br>................. | ...H..............<br>..........F... | ......<br>.... |
| iPS:39 2652 | 21-225_17C6 | VK1\|O12/ JK3 | ............<br>........... | ........N..<br>....T... | ........<br>....... | ........<br>....... | .................<br>...............F. | ....R.............<br>.........TPF.. | ......<br>.... |

| | | | | | | | | | |
|---|---|---|---|---|---|---|---|---|---|
| iPS:39 2660 | 21-225_19B3 | VK1\|O12/ JK3 | ............<br>......I.... | ..G...N.I..<br>....N... | ........<br>..N.... | V.......<br>....... | ......N..........<br>................. | ..................<br>.............. | ......<br>.... |
| iPS:39 2668 | 21-225_17B4 | VK1\|O12/ JK3 | ............<br>........... | ......N....<br>........ | ........<br>......F | G.......<br>......T | .................<br>....N..........F. | ....R.............<br>.........TPF.. | ......<br>.... |
| iPS:39 2678 | 21-225_20F3 | VK1\|O12/ JK3 | ............<br>........... | ...........<br>.......Y | ........<br>....... | ........<br>....... | .................<br>................. | ..................<br>.........AP... | ......<br>.... |
| iPS:39 2694 | 21-225_19A5 | VK1\|O12/ JK3 | ............<br>.P.....S... | ......N.I..<br>....N... | ........<br>......D | V.......<br>...N..G | .................<br>................. | ..................<br>.............. | ......<br>.... |
| iPS:39 2696 | 21-225_20A4 | VK1\|O12/ JK3 | ........A...<br>........... | ........I..<br>....N... | ....R...<br>S...... | ........<br>.....H. | ........R........<br>...............F. | ....R.............<br>.........TPL.. | ......<br>..F. |
| iPS:39 2702 | 21-225_17F7 | VK1\|O12/ JK3 | ............<br>........... | ......T....<br>.....F.. | ........<br>....... | ........<br>....... | .................<br>.....G.........F. | ....R.............<br>.........TPF.. | ......<br>.... |
| iPS:39 2704 | 21-225_17F11 | VK1\|O12/ JK3 | ............<br>..I....S... | .....RT.N..<br>....N... | ........<br>......F | ........<br>.T..... | .................<br>................. | ..T...............<br>.........TPL.A | ......<br>.... |
| iPS:39 2720 | 21-225_17A12 | VK1\|O12/ JK3 | ............<br>........... | ...........<br>........ | ..H.....<br>....... | ........<br>....... | .................<br>.....N........... | ....N.............<br>.........TPL.. | ......<br>.... |
| iPS:39 2722 | 21-225_18E12 | VK1\|O12/ JK3 | ............<br>........... | ...........<br>........ | ........<br>..T.... | ........<br>....... | .................<br>....N..........F. | ....R.............<br>.........TPF.. | ......<br>.... |
| iPS:39 2760 | 21-225_22G3 | VK1\|O12/ JK3 | ............<br>........... | ...........<br>....N... | ........<br>......F | ........<br>....... | .................<br>.....G.........F. | ...FR.............<br>.........TPF.. | ......<br>.... |
| iPS:39 2762 | 21-225_22G5 | VK1\|O12/ JK3 | ............<br>........... | ......N....<br>........ | .....Q..<br>....... | ........<br>......N | ........R........<br>................. | ....R.............<br>.........TPL.. | ......<br>..F. |
| iPS:39 2764 | 21-225_22G10 | VK1\|O12/ JK3 | ............<br>........... | ........F..<br>........ | ........<br>....... | ........<br>....... | .................<br>...............F. | ...FR.............<br>.........TPL.. | ......<br>..F. |
| iPS:39 2812 | 21-225_21F4 | VK1\|O12/ JK3 | ............<br>........... | ......N.G..<br>........ | ........<br>......F | ........<br>....... | .................<br>..............FF. | ....R.............<br>.........TPF.. | ......<br>..F. |
| iPS:39 2816 | 21-225_22E4 | VK1\|O12/ JK3 | ............<br>........... | ......N....<br>........ | ........<br>....... | ........<br>....... | .................<br>................. | ....N.............<br>.........TPL.. | ......<br>.... |
| iPS:39 2830 | 21-225_21A5 | VK1\|O12/ JK3 | ...........C<br>........... | ......T....<br>.....H.. | ...R....<br>....... | ........<br>....... | .................<br>......V.......... | ....N.............<br>..........IS.. | ......<br>.... |
| iPS:39 2852 | 21-225_21A2 | VK1\|O12/ JK3 | ............<br>........... | ...........<br>........ | ........<br>....... | ........<br>....... | .................<br>...............F. | ....R.............<br>.........TPF.. | ......<br>.... |
| iPS:39 2878 | 21-225_22C5 | VK1\|O12/ JK3 | ........A...<br>........... | ......N....<br>........ | ........<br>....... | ........<br>...V..H | .I......R........<br>..I.............. | ....R.............<br>.........TPL.. | ......<br>..F. |
| iPS:39 2902 | 21-225_22D5 | VK1\|O12/ JK3 | ............<br>........... | ......N.F..<br>........ | ..H.....<br>....... | ........<br>....... | .................<br>................. | ..................<br>.........TPL.. | ......<br>.... |
| iPS:39 2984 | 21-225_30E11 | VK1\|O12/ JK3 | ............<br>........... | ...........<br>....N... | ....QT..<br>...F... | ........<br>....... | .................<br>................. | ..................<br>.............. | ......<br>.... |
| iPS:39 3114 | 21-225_33G12 | VK1\|O12/ JK3 | ............<br>........... | ...........<br>....N... | ....QT..<br>...F... | ........<br>....... | .................<br>................. | ..................<br>.............. | ......<br>.... |
| iPS:39 3824 | 21-225_10F12 | VK1\|O12/ JK3 | ............<br>........... | ......N....<br>........ | ........<br>....... | ........<br>....... | .................<br>................. | ....N.............<br>.........TPF.. | ......<br>.... |

| | | | | | | | | | |
|---|---|---|---|---|---|---|---|---|---|
| iPS:39 3848 | 21-225_4H2 | VK1\|O12/ JK3 | .....L......<br>........... | ..I...N....<br>........ | ........<br>....V.. | ........<br>....... | ........R........<br>....GCV.R........ | ....R.............<br>.........TPL.. | ......<br>.... |
| iPS:39 3862 | 21-225_5G2 | VK1\|O12/ JK3 | ..........P.<br>..........F | ......N.I..<br>........ | ........<br>.R..V.. | G.......<br>....... | .................<br>..N.R............ | ..................<br>.........TPL.. | ......<br>.... |
| iPS:39 3888 | 21-225_3E3 | VK1\|O12/ JK3 | ..........P.<br>..........F | ........R..<br>........ | ........<br>.H..V.. | G.......<br>.T..... | .................<br>................. | ..................<br>.........TPL.. | ......<br>.... |
| iPS:39 3890 | 21-225_4B1 | VK1\|O12/ JK3 | ............<br>........... | .....HT.R..<br>....T... | ........<br>....... | ........<br>....... | .....IN..........<br>....TN........... | ....N.............<br>..........IS.. | ......<br>.... |
| iPS:39 3898 | 21-225_5F7 | VK1\|O12/ JK3 | ..........P.<br>..........F | ......T....<br>........ | ........<br>......S | ........<br>....... | .................<br>................. | ....N.............<br>.........TPL.. | ......<br>.V.. |
| iPS:39 3904 | 21-225_8H11 | VK1\|O12/ JK3 | ............<br>.F......... | ......N.I..<br>........ | ........<br>..N.M.. | V.......<br>.T...H. | .................<br>S................ | ..................<br>.............. | ......<br>.... |
| iPS:39 3936 | 21-225_14A11 | VK1\|O12/ JK3 | ............<br>..I........ | ...........<br>........ | ........<br>......F | ........<br>......N | .................<br>................. | ..T...............<br>.........SP... | .A....<br>.... |
| iPS:39 3980 | 21-225_6D3 | VK1\|O12/ JK3 | ............<br>........... | .T.........<br>....T... | ........<br>......F | ........<br>....... | .................<br>...............F. | ....R.............<br>.........TPF.. | ......<br>.... |
| iPS:39 4014 | 21-225_8G6 | VK1\|O12/ JK3 | ............<br>........... | ...........<br>........ | ........<br>......F | ........<br>....... | .................<br>...............F. | ....R.............<br>.........TPF.. | ......<br>.... |
| iPS:39 4022 | 21-225_16H6 | VK1\|O12/ JK3 | ............<br>........... | ......N....<br>........ | ........<br>....... | ........<br>....... | ........R........<br>................. | ....R.............<br>.........TPL.. | ......<br>..F. |
| iPS:39 4043 | 21-225_3B1 | VK1\|O12/ JK3 | ............<br>........... | ........N..<br>....N... | ........<br>....... | ........<br>.T....N | .................<br>................. | ..................<br>.........TPL.. | ......<br>...N |
| iPS:39 4051 | 21-225_9E5 | VK1\|O12/ JK3 | .......Q....<br>........... | ........A..<br>........ | ........<br>....... | G.......<br>....... | .................<br>................. | ..................<br>.........TPL.S | ......<br>.... |
| iPS:39 4077 | 21-225_8E12 | VK1\|O12/ JK3 | ............<br>........... | ...........<br>....N... | ........<br>....... | ........<br>....... | .................<br>...............F. | ....R.............<br>.........TPF.. | ......<br>.... |
| iPS:39 4087 | 21-225_11A5 | VK1\|O12/ JK3 | ............<br>........... | ......N.Y..<br>........ | ........<br>....... | ........<br>....... | .................<br>................. | ....N.............<br>.........TPL.. | ......<br>.... |
| | | Germline | K_FR1 | K_CDR1 | K_FR2 | K_CDR2 | K_FR3 | K_CDR3 | K_FR4 |
| | VK2\|A18/JK 3 | | DIVMTQTPLSLS<br>VTPGQPASISC | KSS--<br>QSLLHSD-<br>GKTYLY | WYLQKPGQ<br>SPQLLIY | E-------<br>-VSSRFS | GVPDRFSGSGSG--<br>TDFTLKISRVEAEDVGV<br>YYC | MQGIH-------------<br>----------LPFT | FGPGTK<br>VDIK |
| iPS:43 4053 | 21-225_51E1 | VK2\|A18/ JK3 | ............<br>........M.. | ...........<br>E....... | ...R....<br>P..F..F | ........<br>...N... | ...............E.<br>................. | ..S.Q.............<br>.............. | ......<br>.... |
| iPS:43 4137 | 21-225_54D4 | VK2\|A18/ JK3 | ............<br>........M.. | ...........<br>E....... | ...R....<br>P..F..F | ........<br>...N... | ...............E.<br>..............I.. | ..S.Q.............<br>..........F... | ......<br>.... |
| iPS:43 4149 | 21-225_55H1 | VK2\|A18/ JK3 | ............<br>........... | ...........<br>E....... | ........<br>P..F..F | ........<br>...H... | .................<br>................. | ..S.Q.............<br>.............. | ......<br>.... |
| iPS:43 5315 | 21-225_147B2 | VK2\|A18/ JK3 | ............<br>........... | ...........<br>E....... | ........<br>P...... | ........<br>...H.V. | .................<br>.V............... | ..STQ.............<br>..........F.P. | ......<br>.... |
| | | Germline | K_FR1 | K_CDR1 | K_FR2 | K_CDR2 | K_FR3 | K_CDR3 | K_FR4 |

| | | | | | | | | | |
|---|---|---|---|---|---|---|---|---|---|
| | VK1\|O18/JK 3 | | DIQMTQSPSSLS<br>ASVGDRVTITC | QAS--QDIS--<br>----NYLN | WYQQKPGK<br>APKLLIY | D-------<br>-ASNLET | GVPSRFSGSGSG--<br>TDFTFTISSLQPEDIAT<br>YYC | QQYDN------------<br>----------LPFT | FGPGTK<br>VDIK |
| iPS:43 4055 | 21-225_51B4 | VK1\|O18/ JK3 | ............<br>........... | .....R..T..<br>....F... | ........<br>....... | ........<br>....... | .................<br>.....CVH......L. | ..................<br>.............. | .....T<br>.... |
| | | Germline | K_FR1 | K_CDR1 | K_FR2 | K_CDR2 | K_FR3 | K_CDR3 | K_FR4 |
| | VK1\|O18/JK 4 | | DIQMTQSPSSLS<br>ASVGDRVTITC | QAS--QDIS--<br>----NYLN | WYQQKPGK<br>APKLLIY | D-------<br>-ASNLET | GVPSRFSGSGSG--<br>TDFTFTISSLQPEDIAT<br>YYC | QQYDN------------<br>----------LPLT | FGGGTK<br>VEIK |
| iPS:43 4087 | 21-225_52F6 | VK1\|O18/ JK4 | ............<br>........... | ...........<br>.......H | ........<br>....... | ........<br>...T.G. | ...L..........E.<br>....N.........S. | ..C...............<br>.............. | ......<br>.... |
| iPS:43 4111 | 21-225_53H2 | VK1\|O18/ JK4 | ............<br>........... | ...........<br>.......H | ........<br>..Q.... | ........<br>....... | ......T.........<br>................. | H.................<br>.............. | ......<br>.... |
| iPS:43 4121 | 21-225_53F6 | VK1\|O18/ JK4 | ............<br>........... | ........T..<br>.......D | ........<br>....... | ........<br>......G. | .................<br>................. | ..C...............<br>.............. | ......<br>.... |
| iPS:43 4163 | 21-225_50H1 | VK1\|O18/ JK4 | ..........P.<br>........... | ...........<br>.......D | ........<br>....... | ........<br>....... | .................<br>A................ | ..C...............<br>.............. | ......<br>.... |
| iPS:43 5611 | 21- 225_161F10 | VK1\|O18/ JK4 | ............<br>........... | ........Y..<br>.....H.S | ........<br>....... | ........<br>....W.. | ........G.......<br>..........F.... | ...E..............<br>.............. | ......<br>.... |
| iPS:43 5811 | 21- 225_183H6 | VK1\|O18/ JK4 | ............<br>.........A. | ...........<br>........ | ....T...<br>...V... | ........<br>....... | ...A.............<br>................. | ..................<br>.............. | ......<br>.D.. |
| iPS:39 4035 | 21-225_5G9 | VK1\|O18/ JK4 | ............<br>........... | ......G....<br>.....S.. | ........<br>....... | ........<br>....... | ..........A.....<br>................. | ..................<br>.............. | ......<br>.... |
| | | Germline | K_FR1 | K_CDR1 | K_FR2 | K_CDR2 | K_FR3 | K_CDR3 | K_FR4 |
| | VK1\|L1/JK1 | | DIQMTQSPSSLS<br>ASVGDRVTITC | RAS--QGIS--<br>----NYLA | WFQQKPGK<br>APKSLIY | A-------<br>-ASSLQS | GVPSRFSGSGSG--<br>TDFTLTISSLQPEDFAT<br>YYC | QQYNS------------<br>----------YPWT | FGQGTK<br>VEIK |
| iPS:43 4095 | 21- 225_52F10 | VK1\|L1/J K1 | ............<br>V.......... | ...........<br>.......G | ........<br>....... | ........<br>....... | .................<br>................. | ..................<br>............P. | ......<br>.... |
| iPS:39 2848 | 21-225_20F9 | VK1\|L1/J K1 | ............<br>......I.... | ...........<br>........ | ........<br>......S | ........<br>....... | ....K...........<br>...............C. | ...H..............<br>.............. | ......<br>.... |
| iPS:39 3078 | 21- 225_33H11 | VK1\|L1/J K1 | ............<br>.F......... | W.......N..<br>....S... | ....R...<br>.H..... | ........<br>......G | ....K...........<br>I.......R....... | ..F...............<br>............L. | ......<br>.... |
| iPS:39 3142 | 21-225_33A3 | VK1\|L1/J K1 | ............<br>........... | ........N..<br>........ | ........<br>....... | ........<br>....... | ....K...........<br>................. | ..F...............<br>............P. | ......<br>.... |
| iPS:39 3946 | 21-225_16A4 | VK1\|L1/J K1 | ............<br>........... | ......D....<br>........ | ........<br>......S | ........<br>....... | ....K...........<br>................. | ...H..............<br>.............. | ......<br>...N |
| | | Germline | K_FR1 | K_CDR1 | K_FR2 | K_CDR2 | K_FR3 | K_CDR3 | K_FR4 |
| | VK1\|O12/JK 5 | | DIQMTQSPSSLS<br>ASVGDRVTITC | RAS--QSIS--<br>----SYLN | WYQQKPGK<br>APKLLIY | A-------<br>-ASSLQS | GVPSRFSGSGSG--<br>TDFTLTISSLQPEDFAT<br>YYC | QQSYS------------<br>----------TPIT | FGQGTR<br>LEIK |
| iPS:43 | 21-225_53C6 | VK1\|O12/ | ............ | ......YS... | ........ | ........ | ................. | .................. | ...... |

| | | | | | | | | | |
|---|---|---|---|---|---|---|---|---|---|
| iPS:43 4317 | 21-225_59E8 | VK1\|O12/ JK5 | ............<br>........... | ...........<br>........ | ........<br>....... | ........<br>....... | ..................<br>...............F. | ...F..............<br>..........NS.. | ......<br>.... |
| iPS:43 4327 | 21-225_63G6 | VK1\|O12/ JK5 | ............<br>.......I.S. | ........F..<br>........ | ...V....<br>....... | D.......<br>.T.T..T | ..................<br>....N............ | ....G.............<br>..........I... | ......<br>...Q |
| iPS:43 4455 | 21-225_72F5 | VK1\|O12/ JK5 | ............<br>.......I.P. | .....N....<br>........ | ........<br>....... | ........<br>....... | ..................<br>...............S. | ..T.-<br>..................<br>.....STP. | ......<br>.D.N |
| iPS:43 5525 | 21-225_157E7 | VK1\|O12/ JK5 | ............<br>........... | .......F...<br>........ | .......I<br>....... | ........<br>....... | ..................<br>.................. | .E................<br>..........IRFA | ......<br>.... |
| iPS:39 2754 | 21-225_21D3 | VK1\|O12/ JK5 | ............<br>........... | ........T..<br>....G.S. | ........<br>T.....F | ........<br>.TY..E. | .........F...N.<br>....T........... | ..................<br>..........S.. | ......<br>.... |
| iPS:39 2818 | 21-225_22D8 | VK1\|O12/ JK5 | ............<br>........... | .T....NSN..<br>........ | ........<br>....Q.F | ........<br>..Y..E. | ........NR.....E.<br>.................. | ..T.G.............<br>..........S.. | ......<br>.... |
| iPS:39 3064 | 21-225_33A9 | VK1\|O12/ JK5 | ............<br>........... | ...........<br>....R..S | .......R<br>..N.Q.. | ........<br>....... | ..................<br>.................. | ....N.............<br>..........I... | ......<br>.... |
| iPS:39 3148 | 21-225_35E5 | VK1\|O12/ JK5 | ....I.......<br>..........Y | ...........<br>........ | ......A.<br>....H.. | G.......<br>....F.. | W.........S.....<br>....I.M..G.Y..... | H...N.............<br>..........L... | ......<br>.... |
| iPS:39 8536 | 21-225_33D12 | VK1\|O12/ JK5 | .V..........<br>..L........ | ........R..<br>........ | ........<br>..N.... | S.......<br>....... | ..................<br>.............N... | ..................<br>..........I... | ......<br>.... |
| | | Germline | K_FR1 | K_CDR1 | K_FR2 | K_CDR2 | K_FR3 | K_CDR3 | K_FR4 |
| | VK3\|A27/JK2 | | EIVLTQSPGTLS<br>LSPGERATLSC | RAS--QSVSS-<br>----SYLA | WYQQKPGQ<br>APRLLIY | G-------<br>-ASSRAT | GIPDRFSGSGSG--<br>TDFTLTISRLEPEDFAV<br>YYC | QQYGS------------<br>----------SPYT | FGQGTK<br>LEIK |
| iPS:43 4127 | 21-225_53H8 | VK3\|A27/ JK2 | ............<br>........... | .......IT..<br>........ | ........<br>....... | ........<br>...G... | ..................<br>.................. | ..FE..............<br>.........SPMCS | .....N<br>.... |
| iPS:43 6290 | 21-225_205G3 | VK3\|A27/ JK2 | ............<br>........... | ......N..Y.<br>........ | ........<br>....... | ........<br>...R... | ..................<br>.................. | ..................<br>...........CS | ......<br>.... |
| iPS:43 6568 | 21-225_225B3 | VK3\|A27/ JK2 | ............<br>........... | ......NL...<br>.......G | ........<br>....... | D.......<br>.T..... | ..................<br>.................. | .E................<br>.........SLMCS | ......<br>.... |
| iPS:39 2898 | 21-225_21H10 | VK3\|A27/ JK2 | ............<br>........... | .......F...<br>........ | ........<br>....... | ........<br>....... | ..................<br>.................. | ....-<br>..................<br>......SRS | ......<br>.... |
| iPS:39 3802 | 21-225_3D12 | VK3\|A27/ JK2 | ............<br>........... | ...........<br>........ | ........<br>....... | ........<br>.T..... | ..................<br>.................. | ....-<br>..................<br>......SRS | ......<br>.... |
| | | Germline | K_FR1 | K_CDR1 | K_FR2 | K_CDR2 | K_FR3 | K_CDR3 | K_FR4 |
| | VK1\|L1/JK4 | | DIQMTQSPSSLS<br>ASVGDRVTITC | RAS--QGIS--<br>----NYLA | WFQQKPGK<br>APKSLIY | A-------<br>-ASSLQS | GVPSRFSGSGSG--<br>TDFTLTISSLQPEDFAT<br>YYC | QQYNS------------<br>----------YPLT | FGGGTK<br>VEIK |
| iPS:43 4157 | 21-225_55D4 | VK1\|L1/J K4 | ...........F<br>........... | ......D....<br>.......I | ........<br>....... | T.......<br>....... | ....K.....F......<br>.....N........... | ...H..............<br>..........F... | .....R<br>...R |
| iPS:43 | 21- | VK1\|L1/J | ............ | ......D.N.. | ........ | ........ | ....K........... | .................. | ...... |

| | | | | | | | | | |
|---|---|---|---|---|---|---|---|---|---|
| iPS:43 4367 | 21-225_65H11 | VK1\|L1/J K4 | ............<br>........... | ......D....<br>....T... | ........<br>....... | ........<br>....... | ....K...........<br>................ | ...................<br>..........F... | ......<br>.... |
| iPS:43 4429 | 21-225_70H6 | VK1\|L1/J K4 | ............<br>..L........ | .T....S.F..<br>.......N | ...R....<br>...V... | T.......<br>....... | .I..............<br>...........S.... | ..SY...............<br>..........I... | ......<br>.... |
| iPS:43 4535 | 21-225_74C8 | VK1\|L1/J K4 | ............<br>........... | ........G..<br>....K... | ........<br>....... | T.......<br>.T.N... | .A..K...........<br>........Y....... | ...SN..............<br>.............. | ......<br>...N |
| iPS:43 4573 | 21-225_77E6 | VK1\|L1/J K4 | ............<br>........... | ...........<br>....K... | ........<br>....... | ........<br>......G | .A..K...........<br>................ | ...SN..............<br>.............. | .....R<br>.... |
| iPS:43 4615 | 21-225_76C5 | VK1\|L1/J K4 | ............<br>........... | ......V....<br>....K... | ........<br>....... | ........<br>....... | .A..K...........<br>................ | ...SN..............<br>.............. | ......<br>.... |
| iPS:43 4669 | 21-225_79F4 | VK1\|L1/J K4 | ............<br>........... | ...........<br>....K... | ........<br>....... | ........<br>......G | .A..K...........<br>................ | ...SN..............<br>.............. | .....R<br>.... |
| iPS:43 4737 | 21-225_74G6 | VK1\|L1/J K4 | ............<br>........... | ........G..<br>....K... | ........<br>....... | T.......<br>.T..... | .A..K...........<br>........Y....... | ...SN..............<br>.............. | ......<br>...N |
| iPS:43 4741 | 21-225_80C11 | VK1\|L1/J K4 | ............<br>........... | ........G..<br>....R... | .......R<br>....... | T.......<br>....... | .A..K...........<br>................ | ...SN..............<br>.............. | ......<br>...N |
| iPS:43 4867 | 21-225_79A12 | VK1\|L1/J K4 | ............<br>........... | ......V....<br>....K... | ........<br>....... | ........<br>....... | .A..K...........<br>................ | ...SN..............<br>.............. | ......<br>.... |
| iPS:43 5333 | 21-225_147E9 | VK1\|L1/J K4 | ............<br>........... | ......D.N..<br>........ | ........<br>......V | ........<br>....... | ....K...........<br>................ | ...................<br>.............. | ......<br>.... |
| iPS:43 5409 | 21-225_150G8 | VK1\|L1/J K4 | ............<br>........... | ...........<br>....H... | ........<br>....... | V.......<br>......N | ................<br>................ | ....N..............<br>.............. | ......<br>.... |
| iPS:43 5505 | 21-225_157C1 | VK1\|L1/J K4 | ............<br>..I...I.L.. | ...........<br>........ | ....R...<br>....... | ........<br>.....L. | ....K...........<br>................ | ...................<br>..........F.F. | ......<br>..L. |
| iPS:43 5595 | 21-225_160H4 | VK1\|L1/J K4 | ............<br>........... | ......D....<br>.......V | .....L..<br>....... | V.......<br>....... | ................<br>................ | ...................<br>.............. | ......<br>.... |
| iPS:43 5639 | 21-225_163G6 | VK1\|L1/J K4 | ............<br>........... | ......D....<br>.......V | ........<br>....... | ........<br>.....L. | ....K...........<br>................ | ...H...............<br>.............. | ......<br>.A.. |
| iPS:43 5653 | 21-225_166H12 | VK1\|L1/J K4 | ............<br>T.......... | ......D....<br>....H... | ........<br>....... | ........<br>....... | ....K...........<br>................ | ...................<br>..........F... | ......<br>...T |
| iPS:43 5677 | 21-225_169C10 | VK1\|L1/J K4 | ............<br>........... | ......D....<br>........ | ........<br>......F | S.......<br>....... | ....K...........<br>N............... | ..SD...............<br>.............. | ......<br>.... |
| iPS:43 5699 | 21-225_170D6 | VK1\|L1/J K4 | ............<br>.......A... | ......D.G..<br>.....C.. | ........<br>....... | S.......<br>....... | ....K...........<br>................ | ..SD...............<br>.............. | ......<br>.... |
| iPS:43 5745 | 21-225_175G3 | VK1\|L1/J K4 | ............<br>........... | ......D....<br>.....D.. | ........<br>......F | S.......<br>....... | ....K...........<br>................ | ..SD...............<br>.............. | ......<br>.... |
| iPS:43 5819 | 21-225_190C11 | VK1\|L1/J K4 | ............<br>........... | .T......G..<br>........ | ........<br>.....L. | K.......<br>.T..... | ....K...........<br>................ | ...MT..............<br>.............. | ......<br>.... |
| iPS:43 5825 | 21-225_190G11 | VK1\|L1/J K4 | ............<br>........... | .T......G..<br>........ | ........<br>.....L. | K.......<br>....... | ....K...........<br>................ | ...MT..............<br>.............. | ......<br>.... |
| iPS:43 5837 | 21-225_198G3 | VK1\|L1/J K4 | ....A.......<br>........... | .T......G..<br>....K... | ........<br>.....L. | K.......<br>......G | ....K...........<br>................ | ...MT..............<br>.............. | ......<br>.... |

| | | | | | | | | | |
|---|---|---|---|---|---|---|---|---|---|
| iPS:43 5845 | 21-225_191G1 | VK1\|L1/J K4 | ............ ........... | ........... ........ | ........ ....... | ........ ....... | ....K........... ................ | .H.LT............ .............. | ...... .... |
| iPS:43 5859 | 21-225_190E6 | VK1\|L1/J K4 | ............ ........... | .T.....G... ........ | ........ .....L. | K....... ....... | ....K........... ................ | ...MT............ .............. | ...... .... |
| iPS:43 5873 | 21-225_190G4 | VK1\|L1/J K4 | ............ ........... | ......D.G.. ....R... | ........ ....... | ........ ....... | ....K........... .........N...... | ...ST............ .............. | ...... .... |
| iPS:43 5933 | 21-225_190F8 | VK1\|L1/J K4 | ............ ........... | .T.....G... ........ | ........ .....L. | K....... ....... | ....K........... ................ | ...MT............ .............. | ...... .... |
| iPS:43 5945 | 21-225_191A10 | VK1\|L1/J K4 | ............ ........... | ........G.. ....K... | ........ ....... | ........ ....... | ....K....Y...... .........N..I... | ...ST............ .............. | ...... .... |
| iPS:43 5947 | 21-225_191E10 | VK1\|L1/J K4 | ............ ........... | ........G.. ....K... | ........ ....... | ........ ....... | ....K........... .........N...... | ...ST............ .............. | ...... .... |
| iPS:43 5957 | 21-225_191G12 | VK1\|L1/J K4 | ............ ........... | .T.....G... ........ | ........ .....L. | K....... ....... | ....K........... ................ | ...IT............ .............. | ....S. .... |
| iPS:43 5963 | 21-225_192D2 | VK1\|L1/J K4 | ....I....... ........... | ........... ........ | ........ ....... | ........ ....... | ....K........... ................ | .H.VT............ ............N. | ...... .... |
| iPS:43 5971 | 21-225_192D3 | VK1\|L1/J K4 | ............ ........... | ........... ........ | ........ ....... | ........ ....... | ....K........... ................ | LH.LT............ .............. | ...... .... |
| iPS:43 5979 | 21-225_192H4 | VK1\|L1/J K4 | ............ ........... | ......D.... ........ | ........ ......S | ........ ....... | ....K...R....... ...............G | LH.LN............ .............. | .....R ...R |
| iPS:43 5987 | 21-225_192G6 | VK1\|L1/J K4 | ..K......... ........... | .T.....G... ........ | ........ .....L. | K....... ....... | ....K........... ................ | ...MT............ .............. | ...... .... |
| iPS:43 5993 | 21-225_192C8 | VK1\|L1/J K4 | ............ ........... | ........... ........ | ........ ....... | ........ ....... | ....K........... ................ | .H.LT............ .............. | ...... .... |
| iPS:43 5997 | 21-225_192G8 | VK1\|L1/J K4 | ............ ........... | .T.....G... ........ | ........ .....L. | K....... ....... | ....K........... ................ | ...IT............ .............. | ...... .... |
| iPS:43 6005 | 21-225_192H10 | VK1\|L1/J K4 | ............ ........... | ........G.. ....K... | ........ ....... | ........ ....... | ....K....Y...... .........N...... | ...ST............ .............. | ...... .... |
| iPS:43 6031 | 21-225_193C7 | VK1\|L1/J K4 | ............ ........... | ........G.. ....K... | ........ ....... | ........ ....... | ....K........... ................ | ...ST............ .............. | ...... .... |
| iPS:43 6045 | 21-225_193A10 | VK1\|L1/J K4 | ............ ........... | ........... ........ | ........ ....... | ........ ....... | ....K........... ..............F. | .H.LT............ .............. | ...... .... |
| iPS:43 6054 | 21-225_194C1 | VK1\|L1/J K4 | ............ ........... | ........... ........ | ........ ....... | ........ ....... | ....K........... ................ | LH.LT............ .............. | ...... .... |
| iPS:43 6076 | 21-225_194H11 | VK1\|L1/J K4 | ............ ........... | ........... ........ | ........ ....... | ........ ....... | ....K........... ................ | .H.LT............ .............. | ...... .... |
| iPS:43 6086 | 21-225_191G10 | VK1\|L1/J K4 | ............ ........... | .T.....G... ....K... | ........ .....L. | K....... .V..... | ....K........... ................ | ...MT............ .............. | ...... .... |
| iPS:43 6090 | 21-225_195A9 | VK1\|L1/J K4 | ............ ........... | ........... ........ | ........ ....... | ........ ....... | ....K........... ................ | .H.LT............ .............. | ...... .... |
| iPS:43 6112 | 21-225_196C7 | VK1\|L1/J K4 | ............ ........... | ..N..A..... ........ | ........ ....... | ........ ....... | ....K........... ................ | .H.LT............ .............. | ...... .... |

| | | | | | | | | | |
|---|---|---|---|---|---|---|---|---|---|
| iPS:43<br>6138 | 21-<br>225_197F2 | VK1\|L1/J<br>K4 | ....I.......<br>........... | .T......G..<br>........ | ........<br>.....L. | K.......<br>.T..... | ....K............<br>................F | ...IT.............<br>............... | ......<br>.... |
| iPS:43<br>6152 | 21-<br>225_197B6 | VK1\|L1/J<br>K4 | ............<br>........... | ........G..<br>....K... | ........<br>....... | G.......<br>....... | ....K............<br>..........N...... | ...ST.............<br>............... | ......<br>.... |
| iPS:43<br>6173 | 21-<br>225_197G12 | VK1\|L1/J<br>K4 | ....I.......<br>........... | .T......G..<br>........ | ........<br>.....L. | K.......<br>.T..... | .F..K............<br>................F | ...MT.............<br>............... | ......<br>.... |
| iPS:43<br>6189 | 21-<br>225_198B6 | VK1\|L1/J<br>K4 | ............<br>........... | ........G..<br>........ | ........<br>....... | ........<br>....... | ....K...NR.......<br>................. | ...ST.............<br>............... | ......<br>.... |
| iPS:43<br>6201 | 21-<br>225_199C5 | VK1\|L1/J<br>K4 | ............<br>........... | ...........<br>....K... | ........<br>....... | ........<br>....... | ....K............<br>.............A... | ...LT.............<br>............... | ......<br>.... |
| iPS:43<br>6260 | 21-<br>225_203H1 | VK1\|L1/J<br>K4 | ............<br>........... | ........G..<br>........ | ........<br>....... | V.......<br>...R... | ....K...T....S..<br>.........D....... | .R.HT.............<br>............... | ......<br>.... |
| iPS:43<br>6282 | 21-<br>225_204G6 | VK1\|L1/J<br>K4 | ..R.........<br>......I.... | .T......G..<br>........ | ........<br>....... | ........<br>....... | .................<br>................. | .H.L..............<br>............... | ......<br>.... |
| iPS:43<br>6284 | 21-<br>225_204G8 | VK1\|L1/J<br>K4 | ............<br>........... | ...........<br>.....H.. | ........<br>......F | ........<br>....... | ....Q..........E.<br>................. | ...SN.............<br>............V. | ......<br>.... |
| iPS:43<br>6292 | 21-<br>225_205H3 | VK1\|L1/J<br>K4 | ..P.........<br>........... | ......A....<br>.....H.. | ...L....<br>....... | ........<br>....... | ....K.........S..<br>................. | ...SN.............<br>............... | ......<br>.... |
| iPS:43<br>6296 | 21-<br>225_205F5 | VK1\|L1/J<br>K4 | ............<br>........... | ........G..<br>........ | ........<br>....... | G.......<br>.V..... | ....K............<br>..............T. | ...SN.............<br>............... | ......<br>.D.R |
| iPS:43<br>6324 | 21-<br>225_207G6 | VK1\|L1/J<br>K4 | ............<br>........... | ........R..<br>........ | .L......<br>......H | ........<br>....... | ....K...NR.......<br>................. | ...SN.............<br>............... | ......<br>.... |
| iPS:43<br>6364 | 21-<br>225_211A11 | VK1\|L1/J<br>K4 | ............<br>........... | ........G..<br>........ | ........<br>..R.... | D.......<br>.....E. | ....K....R.......<br>....G............ | .H.MT.............<br>............... | ..A...<br>.... |
| iPS:43<br>6366 | 21-<br>225_211A3 | VK1\|L1/J<br>K4 | ..R.........<br>........... | ......A.G..<br>....KH.. | ....R...<br>....... | ........<br>...R... | ....K............<br>..........L..... | ...SN.............<br>............... | ......<br>.... |
| iPS:43<br>6372 | 21-<br>225_211A8 | VK1\|L1/J<br>K4 | ..E.....P...<br>.F......... | ...........<br>....R... | .V......<br>....... | ........<br>....... | ..S..............<br>................. | LR.DT.............<br>.............I | ......<br>.... |
| iPS:43<br>6376 | 21-<br>225_212E6 | VK1\|L1/J<br>K4 | ............<br>........... | ...........<br>.....H.. | .......Q<br>....... | ........<br>....... | ....K....R.......<br>................. | ...VT.............<br>............... | ......<br>.... |
| iPS:43<br>6378 | 21-<br>225_212D7 | VK1\|L1/J<br>K4 | ............<br>........... | ...........<br>.....H.. | ........<br>......H | ........<br>....... | ....K...NR.......<br>....NN.....V.... | ...SN.............<br>............... | ......<br>.... |
| iPS:43<br>6380 | 21-<br>225_212H9 | VK1\|L1/J<br>K4 | ..E.........<br>........... | ...........<br>....S... | .L......<br>....... | ........<br>....... | .................<br>................. | LR.DT.............<br>............... | ......<br>.... |
| iPS:43<br>6384 | 21-<br>225_212F10 | VK1\|L1/J<br>K4 | ............<br>........... | ...........<br>.......D | ........<br>....... | S.......<br>...N... | ....K....R.......<br>................. | .H.SN.............<br>............... | ......<br>.... |
| iPS:43<br>6388 | 21-<br>225_212H11 | VK1\|L1/J<br>K4 | ..R.........<br>........... | ......A.G..<br>....KH.. | ....R...<br>....... | ........<br>...R... | ....K............<br>..........L..... | .H.SN.............<br>............... | .V....<br>...T |
| iPS:43<br>6390 | 21-<br>225_213D2 | VK1\|L1/J<br>K4 | ............<br>........... | ...........<br>.....H.. | ........<br>......H | ........<br>....... | ....K...NR.......<br>....NN.....V.... | H..SN.............<br>............... | ......<br>.... |
| iPS:43<br>6394 | 21-<br>225_213C4 | VK1\|L1/J<br>K4 | ..H.........<br>........... | ......A.R..<br>........ | .C......<br>...T... | ........<br>....... | ....K............<br>................. | ...SN.............<br>............... | ......<br>.... |

| | | | | | | | | | |
|---|---|---|---|---|---|---|---|---|---|
| **iPS:43 6396** | **21-225_213E5** | **VK1\|L1/J K4** | ..R.........<br>........... | ......A.G..<br>....KH.. | ....R...<br>....... | ........<br>...R... | ....K............<br>...........L..... | .H.SN.............<br>............... | ......<br>.... |
| **iPS:43 6398** | **21-225_213B8** | **VK1\|L1/J K4** | ............<br>........... | ...........<br>....H... | ........<br>....... | ........<br>....... | ....K............<br>................. | ...SN.............<br>............... | ......<br>.... |
| **iPS:43 6410** | **21-225_212E10** | **VK1\|L1/J K4** | ............<br>........... | ...........<br>....H... | ........<br>....... | ........<br>....... | ....K............<br>................. | ...SN.............<br>............... | ......<br>.... |
| **iPS:43 6420** | **21-225_215B5** | **VK1\|L1/J K4** | ............<br>........... | ...........<br>....H... | ........<br>......H | ........<br>....... | ....K...NR.......<br>....NN......V.... | ...IN.............<br>............... | ......<br>.... |
| **iPS:43 6422** | **21-225_215D6** | **VK1\|L1/J K4** | ............<br>........... | ...........<br>....H... | ........<br>....... | ........<br>.....H. | ....K....R.......<br>................. | ...VT.............<br>............... | ......<br>.... |
| **iPS:43 6430** | **21-225_215A12** | **VK1\|L1/J K4** | ............<br>.....I..... | .T....D.G..<br>........ | ........<br>....... | ........<br>....... | ....K....R.......<br>........S........ | ...VT.............<br>............... | ......<br>.... |
| **iPS:43 6452** | **21-225_217G5** | **VK1\|L1/J K4** | ..L.........<br>........... | ........G..<br>........ | ....R...<br>....... | ........<br>....... | ....K...TR.......<br>.........D....... | ...VN.............<br>............... | ......<br>...N |
| **iPS:43 6454** | **21-225_217B10** | **VK1\|L1/J K4** | ..R.........<br>........... | ......A.G..<br>....KH.. | ....R...<br>....... | ........<br>...R... | ....K............<br>......V....L....R | .HTSK.............<br>..........S.VQ. | LV....<br>...T |
| **iPS:43 6464** | **21-225_219H1** | **VK1\|L1/J K4** | ..........Q.<br>........... | ...........<br>......D. | ........<br>....... | S.......<br>...N... | ....K....R.......<br>................. | .H.SN.............<br>............... | ......<br>.... |
| **iPS:43 6490** | **21-225_221F6** | **VK1\|L1/J K4** | ............<br>G.......... | ...........<br>........ | ........<br>....... | ........<br>...N... | ....K....R.......<br>................. | ...MT.............<br>............... | .....R<br>.... |
| **iPS:43 6502** | **21-225_222A11** | **VK1\|L1/J K4** | ............<br>........... | ...........<br>........ | ........<br>....... | ........<br>....... | ....K............<br>................. | LY.LN.............<br>............... | ......<br>.... |
| **iPS:43 6514** | **21-225_222D10** | **VK1\|L1/J K4** | ............<br>........... | ...........<br>........ | ........<br>....... | ........<br>....... | ....K............<br>................. | LH.LN.............<br>............... | .....R<br>.... |
| **iPS:43 6522** | **21-225_223H10** | **VK1\|L1/J K4** | ............<br>........... | ...........<br>........ | ........<br>....... | ........<br>...N... | ....K............<br>................. | LH.LN.............<br>............... | ......<br>.... |
| **iPS:43 7258** | **21-225_153F9** | **VK1\|L1/J K4** | ............<br>........... | ...........<br>........ | ........<br>......S | ........<br>....... | ....K............<br>................. | ..................<br>............S.. | ......<br>.... |
| **iPS:43 7260** | **21-225_170D1** | **VK1\|L1/J K4** | ...........A<br>........... | ......D....<br>........ | ........<br>....... | ........<br>....... | .................<br>................. | ..CD..............<br>..........F.... | ......<br>.... |
| **iPS:43 7264** | **21-225_171H12** | **VK1\|L1/J K4** | ............<br>........... | ......D....<br>........ | ........<br>....... | S.......<br>....... | ....K............<br>................. | ..SD..............<br>............... | ......<br>.... |
| **iPS:43 7266** | **21-225_177A5** | **VK1\|L1/J K4** | ............<br>........... | ......D....<br>........ | ........<br>....... | S.......<br>....... | ....K............<br>................. | ..SD..............<br>............... | ......<br>.... |
| **iPS:43 7270** | **21-225_178H4** | **VK1\|L1/J K4** | ............<br>........... | ......D....<br>........ | ........<br>......F | S.......<br>....... | ....K............<br>.....N........... | ..S...............<br>............... | ......<br>.... |
| **iPS:43 8664** | **21-225_216G1** | **VK1\|L1/J K4** | ..E.........<br>........... | ...........<br>....S... | .L......<br>....... | ........<br>....... | .................<br>................. | LR.DT.............<br>............... | ......<br>.... |
| **iPS:45 1120** | **21-225_197D3** | **VK1\|L1/J K4** | ............<br>........... | ........R..<br>........ | ........<br>....... | ........<br>....... | ....K............<br>................. | .H.LT.............<br>............... | ......<br>.... |
| **iPS:39 2682** | **21-225_16A12** | **VK1\|L1/J K4** | ............<br>........... | ......A.N..<br>....T... | ........<br>......S | ........<br>....... | ....K.........E..<br>................. | ...Y..............<br>............... | ......<br>.... |

| | | | | | | | | | |
|---|---|---|---|---|---|---|---|---|---|
| iPS:39 2856 | 21-225_22A2 | VK1\|L1/JK4 | ............<br>........... | ......D....<br>........ | .V......<br>....... | ........<br>....... | ....K...........<br>..............S... | .................<br>..........F... | ......<br>.... |
| iPS:39 2966 | 21-225_32G3 | VK1\|L1/JK4 | ........T...<br>........... | ......A....<br>........ | ........<br>....... | D.......<br>.T..... | ....K...........<br>....T........... | ...H.............<br>.............. | ......<br>.... |
| iPS:39 3952 | 21-225_1F1 | VK1\|L1/JK4 | ............<br>........... | ........N..<br>........ | .....S..<br>......S | V.......<br>......T | ................<br>................ | .................<br>.............. | ......<br>.... |
| iPS:39 3988 | 21-225_7F10 | VK1\|L1/JK4 | ..........A..<br>........... | ......D.R..<br>........ | ........<br>......S | V.......<br>....... | ................<br>................ | .................<br>.............. | ......<br>.... |
| iPS:40 2233 | 21-225_16D10 | VK1\|L1/JK4 | ............<br>........... | ......D....<br>........ | ........<br>....... | ........<br>.TP.... | ....K.........E.<br>................ | .................<br>.............. | ......<br>.... |
| iPS:40 2237 | 21-225_23D11 | VK1\|L1/JK4 | ............<br>........... | ........A..<br>........ | ....R...<br>......S | ........<br>....... | ....K.........E.<br>................ | ...H.............<br>.............. | ....S.<br>.... |
| | | Germline | **K_FR1** | **K_CDR1** | **K_FR2** | **K_CDR2** | **K_FR3** | **K_CDR3** | **K_FR4** |
| | VK1\|O18/JK5 | | DIQMTQSPSSLS<br>ASVGDRVTITC | QAS--QDIS--<br>----NYLN | WYQQKPGK<br>APKLLIY | D-------<br>-ASNLET | GVPSRFSGSGSG--<br>TDFTFTISSLQPEDIAT<br>YYC | QQYDN------------<br>----------LPIT | FGQGTR<br>LEIK |
| iPS:43 4171 | 21-225_50G4 | VK1\|O18/JK5 | ........T...<br>........... | ........T..<br>.....F.. | ........<br>....... | ........<br>....... | ................<br>................ | ....-<br>.................<br>.....NL.. | ......<br>.... |
| iPS:43 5565 | 21-225_159C4 | VK1\|O18/JK5 | ............<br>........... | ...........<br>....D... | ........<br>....... | ........<br>...T... | ................<br>...............F. | .................<br>.............. | ......<br>.... |
| iPS:43 5607 | 21-225_161G4 | VK1\|O18/JK5 | ............<br>........... | ........Y..<br>........ | ........<br>....... | ........<br>....... | ................<br>..A............. | ....I............<br>.............. | ......<br>.... |
| iPS:43 7302 | 21-225_225B11 | VK1\|O18/JK5 | ............<br>..I........ | ........F..<br>........ | ........<br>....... | ........<br>...T... | ................<br>................ | .................<br>.............. | ......<br>.... |
| iPS:39 2868 | 21-225_24D6 | VK1\|O18/JK5 | ............<br>........... | ........N..<br>........ | ........<br>.L..... | ........<br>...D... | ................<br>.............I... | ...E.............<br>.............. | ......<br>.... |
| iPS:39 3020 | 21-225_30E2 | VK1\|O18/JK5 | ........H...<br>........... | ......Y....<br>........ | .....S..<br>....... | ........<br>.G.S... | ................<br>..........L..... | .................<br>.............. | ......<br>.... |
| iPS:39 3138 | 21-225_35E3 | VK1\|O18/JK5 | ............<br>........... | ........F..<br>........ | ........<br>..N.... | ........<br>....... | ................<br>...............F. | .................<br>.............. | ......<br>.D.R |
| iPS:39 3892 | 21-225_6G7 | VK1\|O18/JK5 | ..........R.<br>........... | ...........<br>........ | .C......<br>.L..... | ........<br>...T... | ................<br>.....V.......... | .................<br>..........V... | ......<br>.... |
| iPS:39 3910 | 21-225_15F10 | VK1\|O18/JK5 | ............<br>........... | ..N....T..<br>.....F.. | ...L....<br>..N..S | ........<br>....... | ................<br>..........V..... | .................<br>.............. | ......<br>.... |
| iPS:39 3912 | 21-225_16F6 | VK1\|O18/JK5 | ............<br>........... | ..N....T..<br>.....F.. | ...L....<br>..N..S | ........<br>....... | ................<br>..........V..... | .................<br>.............. | ......<br>.... |
| iPS:39 4000 | 21-225_11A2 | VK1\|O18/JK5 | ............<br>........... | ...........<br>........ | ........<br>....... | ........<br>....... | ................<br>..........V..... | .................<br>.............. | ......<br>.D.. |
| iPS:39 4004 | 21-225_13A1 | VK1\|O18/JK5 | ............<br>........... | ........N..<br>........ | .....L.T<br>....... | ........<br>.G..... | ................<br>................ | ...E.............<br>.............. | ......<br>.... |
| iPS:39 | 21-225_15C2 | VK1\|O18/ | ............ | ........T.. | ........ | ........ | ................ | ................. | .A.... |

| | | | | | | | | | |
|---|---|---|---|---|---|---|---|---|---|
| iPS:39 4029 | 21-225_1B12 | VK1\|O18/ JK5 | ............<br>........... | ........N..<br>........ | ........<br>....... | ........<br>....... | .................<br>............T.... | ...E.............<br>.............. | ......<br>.... |
| iPS:39 4047 | 21-225_5E6 | VK1\|O18/ JK5 | ............<br>........... | ........N..<br>........ | .C......<br>....... | ........<br>....... | .................<br>................. | .................<br>.............. | ......<br>.... |
| iPS:39 4081 | 21-225_16B3 | VK1\|O18/ JK5 | ............<br>........... | ........N..<br>........ | .......R<br>....... | ........<br>....... | .................<br>................. | ..F..............<br>.............. | ......<br>.... |
| | | Germline | K_FR1 | K_CDR1 | K_FR2 | K_CDR2 | K_FR3 | K_CDR3 | K_FR4 |
| | VK3\|L2/JK3 | | EIVMTQSPATLS<br>VSPGERATLSC | RAS--QSVS--<br>----SNLA | WYQQKPGQ<br>APRLLIY | G-------<br>-ASTRAT | GIPARFSGSGSG--<br>TEFTLTISSLQSEDFAV<br>YYC | QQYNN------------<br>----------WPFT | FGPGTK<br>VDIK |
| iPS:43 4219 | 21-225_60E9 | VK3\|L2/J K3 | ............<br>........... | ...........<br>.....S.. | ..R.....<br>....... | ........<br>....... | .................<br>...............C. | .................<br>.............. | ......<br>I... |
| iPS:43 4279 | 21-225_57F7 | VK3\|L2/J K3 | .........I..<br>.F......... | ...........<br>.....D.. | ........<br>....... | ........<br>....... | .M......G........<br>..........H...... | ...S.............<br>.............. | ......<br>.... |
| iPS:43 4289 | 21-225_57H12 | VK3\|L2/J K3 | ............<br>........... | ...........<br>.....D.. | ........<br>....... | A.......<br>....... | .................<br>................. | ...D.............<br>.............. | ......<br>..N. |
| iPS:43 4291 | 21-225_58A4 | VK3\|L2/J K3 | ............<br>........... | ...........<br>.....D.. | ....R...<br>....... | A.......<br>....... | .................<br>................. | ..F..............<br>.............. | ......<br>.... |
| iPS:43 4297 | 21-225_58A10 | VK3\|L2/J K3 | ............<br>.C......... | ...........<br>.....S.. | ........<br>....... | ........<br>....... | .................<br>................. | .................<br>.............. | ......<br>.... |
| iPS:43 4301 | 21-225_58F11 | VK3\|L2/J K3 | ............<br>........... | ...........<br>.....D.V | ........<br>....... | ........<br>.V..... | .................<br>................. | .................<br>.............. | ......<br>.... |
| iPS:43 7228 | 21-225_60C11 | VK3\|L2/J K3 | ............<br>........... | ...........<br>....ND.. | ........<br>....... | ........<br>....... | ........G........<br>.....A....H...... | ...S.............<br>.............. | ......<br>.... |

| | | | | | | | | | |
|---|---|---|---|---|---|---|---|---|---|
| | | Germline | K_FR1 | K_CDR1 | K_FR2 | K_CDR2 | K_FR3 | K_CDR3 | K_FR4 |
| | VK2\|A19/JK 5 | | DIVMTQSPLSLP<br>VTPGEPASISC | RSS--<br>QSLLHSN-<br>GYNYLD | WYLQKPGQ<br>SPQLLIY | L-------<br>-GSNRAS | GVPDRFSGSGSG--<br>TDFTLKISRVEAEDVGV<br>YYC | MQALQ------------<br>----------TPIT | FGQGTR<br>LEIK |
| iPS:43 4243 | 21-225_62C1 | VK2\|A19/ JK5 | ............<br>........... | ...........<br>........ | ........<br>....... | ........<br>.V..... | .................<br>.......G.......F. | L................<br>............L. | ......<br>.... |
| iPS:43 6648 | 21-225_227F11 | VK2\|A19/ JK5 | .V..........<br>........... | W..........<br>........ | ........<br>...V... | ........<br>....... | .................<br>................. | .................<br>............L. | ......<br>.... |
| iPS:39 4061 | 21-225_12D2 | VK2\|A19/ JK5 | ............<br>........... | ...........<br>........ | ........<br>...V... | ........<br>....... | .................<br>................. | .................<br>.............. | ......<br>.... |
| iPS:39 4071 | 21-225_10C7 | VK2\|A19/ JK5 | ............<br>........... | ...........<br>K....... | ........<br>...V... | ........<br>....... | .................<br>................. | .................<br>............L. | ......<br>.... |

| | | | | | | | | | |
|---|---|---|---|---|---|---|---|---|---|
| | | Germline | K_FR1 | K_CDR1 | K_FR2 | K_CDR2 | K_FR3 | K_CDR3 | K_FR4 |
| | VK1\|L5/JK4 | | DIQMTQSPSSVS<br>ASVGDRVTITC | RAS--QGIS--<br>----SWLA | WYQQKPGK<br>APKLLIY | A-------<br>-ASSLQS | GVPSRFSGSGSG--<br>TDFTLTISSLQPEDFAT<br>YYC | QQANS------------<br>----------FPLT | FGGGTK<br>VEIK |
| iPS:43 4259 | 21-225_62G7 | VK1\|L5/J K4 | ............<br>........F.. | ......D....<br>........ | ....N...<br>....... | ........<br>....... | ...............N.<br>................. | ..T..............<br>.............. | ......<br>.... |

| | | | | | | | | | |
|---|---|---|---|---|---|---|---|---|---|
| iPS:43 4333 | 21-225_63C9 | VK1\|L5/J K4 | ............<br>........... | ...........<br>........ | ........<br>..N.... | ........<br>...... | .................<br>.....G.........F. | ..I..............<br>.............. | ......<br>.A.. |
| iPS:43 4347 | 21-225_64H10 | VK1\|L5/J K4 | ............<br>........... | ...........<br>........ | ........<br>.L..... | ........<br>...... | .................<br>................. | ..I..............<br>.............. | ......<br>.... |
| iPS:43 4359 | 21-225_65G3 | VK1\|L5/J K4 | ............<br>........... | ...........<br>........ | ........<br>...... | ........<br>...... | .................<br>..............F.. | ..V..............<br>.............. | ......<br>.... |
| iPS:43 4369 | 21-225_66B1 | VK1\|L5/J K4 | ............<br>........... | ...........<br>........ | ........<br>.L..... | ........<br>...... | .................<br>................. | ..T..............<br>.............. | ......<br>.... |
| iPS:43 4373 | 21-225_66A7 | VK1\|L5/J K4 | ............<br>........... | ...........<br>........ | ........<br>...... | ........<br>...... | .................<br>................. | ..I..............<br>.............. | ......<br>.... |
| iPS:43 4397 | 21-225_67H4 | VK1\|L5/J K4 | ............<br>........... | ...........<br>........ | ........<br>...... | ........<br>...... | .................<br>................. | ..I..............<br>.............. | ......<br>.... |
| iPS:43 4427 | 21-225_70D6 | VK1\|L5/J K4 | ............<br>........... | ...........<br>....K... | ....N...<br>.L....F | ........<br>...... | .................<br>..............N... | ..T..............<br>.............. | ......<br>.... |
| iPS:43 4435 | 21-225_70G9 | VK1\|L5/J K4 | ............<br>........... | ......D....<br>........ | ....N...<br>...... | ........<br>...... | .................<br>................. | ..T..............<br>.............. | ......<br>.... |
| iPS:43 4437 | 21-225_70A12 | VK1\|L5/J K4 | ............<br>........... | ...........<br>........ | ........<br>.L..... | ........<br>...... | .................<br>.....V......... | ..I..............<br>.............. | ......<br>.... |
| iPS:43 4451 | 21-225_71B7 | VK1\|L5/J K4 | ............<br>........... | ...........<br>........ | ....N..E<br>...... | ........<br>......G | .................<br>..............N... | ..T..............<br>.............. | ......<br>.... |
| iPS:43 4459 | 21-225_71A7 | VK1\|L5/J K4 | ............<br>........... | ...........<br>........ | ........<br>...... | ........<br>...... | .................<br>................. | ..V..............<br>.............. | ......<br>..L. |
| iPS:43 4461 | 21-225_73A3 | VK1\|L5/J K4 | ............<br>..I....... | ...........<br>....N... | ........<br>...... | ........<br>...... | ...........E.....<br>................. | ..V..............<br>.............. | ......<br>.... |
| iPS:43 5479 | 21-225_154E9 | VK1\|L5/J K4 | .....L.....Y<br>........... | ......D....<br>....N... | ....R...<br>...V... | ........<br>...... | .................<br>.....V......... | ..G..............<br>.............. | ......<br>.D.. |
| iPS:43 7232 | 21-225_63E1 | VK1\|L5/J K4 | ...........Y<br>........... | ...........<br>.....Y.. | ........<br>...... | ........<br>...... | .................<br>................. | .................<br>.............. | ......<br>.... |
| iPS:43 7326 | 21-225_75C10 | VK1\|L5/J K4 | ............<br>.......I... | ...........<br>....I... | ........<br>...... | ........<br>...... | ...L.............<br>................. | ...K.............<br>.............. | ......<br>.... |
| | | Germline | K_FR1 | K_CDR1 | K_FR2 | K_CDR2 | K_FR3 | K_CDR3 | K_FR4 |
| | VK1\|O12/JK 2 | | DIQMTQSPSSLS<br>ASVGDRVTITC | RAS--QSIS--<br>-----SYLN | WYQQKPGK<br>APKLLIY | A-------<br>-ASSLQS | GVPSRFSGSGSG--<br>TDFTLTISSLQPEDFAT<br>YYC | QQSYS-----------<br>----------TPYT | FGQGTK<br>LEIK |
| iPS:43 4309 | 21-225_59B5 | VK1\|O12/ JK2 | ............<br>........... | ........I..<br>........ | ........<br>...... | G.......<br>...... | .................<br>................. | .................<br>.........TPMFS | ......<br>.... |
| iPS:39 2874 | 21-225_21D2 | VK1\|O12/ JK2 | ..........F<br>........... | ...........<br>....D... | .......R<br>...... | D.......<br>.T..... | .................<br>....N........... | ..T.NI...........<br>.........LPERS | ..R...<br>.... |
| iPS:39 3940 | 21-225_16B2 | VK1\|O12/ JK2 | GV..........<br>........... | ...........<br>....G... | ........<br>...... | ........<br>...... | .........G........<br>................. | ..T.NT...........<br>.........PPERS | ......<br>.... |
| iPS:39 3956 | 21-225_4D7 | VK1\|O12/ JK2 | ............<br>........... | ...........<br>....D... | ........<br>......F | D.......<br>.TT.... | .................<br>....N........... | ..T.NT...........<br>.........PPERS | ......<br>.... |

| | | | | | | | | | |
|---|---|---|---|---|---|---|---|---|---|
| iPS:39 8476 | 21-225_17C1 | VK1\|O12/ JK2 | ............<br>........... | ......N.N..<br>....D... | ........<br>....... | ........<br>...N... | ...A.....R.......<br>................. | ..T.NT...........<br>.........PPERS | ......<br>.... |
| iPS:40 3870 | 21-225_23G4 | VK1\|O12/ JK2 | ............<br>........... | ......N.Y..<br>........ | ........<br>....... | ........<br>....... | ...............E.<br>.....I........... | ....NT...........<br>.........PPECN | ......<br>.... |
| | | Germline | K_FR1 | K_CDR1 | K_FR2 | K_CDR2 | K_FR3 | K_CDR3 | K_FR4 |
| | VK3\|A27/JK 3 | | EIVLTQSPGTLS<br>LSPGERATLSC | RAS--QSVSS-<br>----SYLA | WYQQKPGQ<br>APRLLIY | G-------<br>-ASSRAT | GIPDRFSGSGSG--<br>TDFTLTISRLEPEDFAV<br>YYC | QQYGS------------<br>----------SPFT | FGPGTK<br>VDIK |
| iPS:43 4311 | 21-225_59H5 | VK3\|A27/ JK3 | ............<br>........... | ...........<br>....I... | .FL.....<br>....... | ........<br>....... | .................<br>................. | H...N............<br>.............. | ......<br>..F. |
| iPS:43 5301 | 21-225_146G4 | VK3\|A27/ JK3 | ............<br>.F......... | ......NII..<br>........ | ........<br>......F | ........<br>.V..... | .................<br>................. | ....R............<br>............N | ......<br>...N |
| iPS:43 5317 | 21-225_147D2 | VK3\|A27/ JK3 | ............<br>........... | ........G..<br>........ | ........<br>....... | ........<br>....... | .................<br>................. | ....-<br>.................<br>......L.. | ......<br>.... |
| iPS:43 5319 | 21-225_147E3 | VK3\|A27/ JK3 | ............<br>........... | ........I..<br>........ | ........<br>....... | ........<br>....... | A................<br>................. | ....R............<br>............N | ......<br>.... |
| iPS:43 5383 | 21-225_149D7 | VK3\|A27/ JK3 | ............<br>.F....V.... | .......II..<br>....N... | ........<br>......F | ........<br>.V..... | .................<br>................. | ....R............<br>............N | ......<br>.... |
| iPS:43 5443 | 21-225_152E7 | VK3\|A27/ JK3 | ............<br>.F......... | ........I..<br>........ | ........<br>......F | ........<br>.V..... | .................<br>................. | ....R............<br>............N | ......<br>.... |
| iPS:43 5465 | 21-225_153A6 | VK3\|A27/ JK3 | ............<br>.F.....P... | ........I..<br>........ | ........<br>......F | ........<br>.V..... | .................<br>................. | ....R............<br>............N | ......<br>.... |
| iPS:43 6058 | 21-225_194A4 | VK3\|A27/ JK3 | ............<br>........... | .....RG..N.<br>....I... | ........<br>....... | ........<br>...N... | .................<br>................. | .HNDY............<br>..........M.. | ......<br>.... |
| iPS:43 6436 | 21-225_216F10 | VK3\|A27/ JK3 | .V..........<br>........... | ...........<br>.....F.. | ........<br>....... | ........<br>.T.T... | .................<br>I................ | ...DR............<br>.............. | ......<br>.... |
| iPS:44 2568 | 21-225_149D8 | VK3\|A27/ JK3 | ............<br>.F......... | ........I..<br>........ | ........<br>......F | ........<br>.V..W.. | .................<br>................. | ....R............<br>............N | ......<br>.... |
| | | Germline | K_FR1 | K_CDR1 | K_FR2 | K_CDR2 | K_FR3 | K_CDR3 | K_FR4 |
| | VK1\|O18/JK 1 | | DIQMTQSPSSLS<br>ASVGDRVTITC | QAS--QDIS--<br>----NYLN | WYQQKPGK<br>APKLLIY | D-------<br>-ASNLET | GVPSRFSGSGSG--<br>TDFTFTISSLQPEDIAT<br>YYC | QQYDN------------<br>----------LPWT | FGQGTK<br>VEIK |
| iPS:43 4353 | 21-225_64B12 | VK1\|O18/ JK1 | ............<br>........... | R..........<br>........ | .......T<br>..N...S | ........<br>...I... | ....T............<br>................. | ..S..............<br>...........CS | ......<br>.... |
| | | Germline | K_FR1 | K_CDR1 | K_FR2 | K_CDR2 | K_FR3 | K_CDR3 | K_FR4 |
| | VK1\|A20/JK 4 | | DIQMTQSPSSLS<br>ASVGDRVTITC | RAS--QGIS--<br>----NYLA | WYQQKPGK<br>VPKLLIY | A-------<br>-ASTLQS | GVPSRFSGSGSG--<br>TDFTLTISSLQPEDVAT<br>YYC | QKYNS------------<br>----------APLT | FGGGTK<br>VEIK |
| iPS:43 4357 | 21-225_65C1 | VK1\|A20/ JK4 | ...L........<br>........... | ......V....<br>....S..H | ........<br>...V... | S.......<br>...N..C | .................<br>...F...........G | .RPYN............<br>.............. | ......<br>.... |
| iPS:43 | 21-225_66C7 | VK1\|A20/ | ...L........ | ........... | ........ | C....... | ................. | .QH.N............ | ...... |

| | | | | | | | | | |
|---|---|---|---|---|---|---|---|---|---|
| iPS:43 4457 | 21-225_72G12 | VK1\|A20/ JK4 | ...L........<br>........... | ...........<br>....S..N | .S......<br>......C | G.......<br>...N... | ..........A.....E.<br>I..........T...G | .QNYN.............<br>............... | ......<br>.... |
| | | Germline | K_FR1 | K_CDR1 | K_FR2 | K_CDR2 | K_FR3 | K_CDR3 | K_FR4 |
| | VK3\|A27/JK4 | | EIVLTQSPGTLS<br>LSPGERATLSC | RAS--QSVSS-<br>----SYLA | WYQQKPGQ<br>APRLLIY | G-------<br>-ASSRAT | GIPDRFSGSGSG--<br>TDFTLTISRLEPEDFAV<br>YYC | QQYGS-------------<br>----------SPLT | FGGGTK<br>VEIK |
| iPS:43 4479 | 21-225_76H1 | VK3\|A27/ JK4 | .FM........Y<br>W.......... | ...........<br>.......V | ........<br>....... | ........<br>...T... | ................<br>..........Y..... | ....C.............<br>............... | ......<br>...T |
| iPS:43 4513 | 21-225_76A6 | VK3\|A27/ JK4 | ..........R.<br>W.......... | ...........<br>........ | ........<br>....... | ........<br>...T... | ................<br>..........Y..... | ....N.............<br>............... | ......<br>.... |
| iPS:43 4515 | 21-225_74A5 | VK3\|A27/ JK4 | .FM.........<br>........... | ...........<br>.......V | ........<br>....... | ........<br>...T... | ................<br>..........Y..... | ....C.............<br>............... | ......<br>...T |
| iPS:43 4529 | 21-225_76B9 | VK3\|A27/ JK4 | .FM.........<br>W.......... | ...........<br>.......V | ........<br>....... | ........<br>...T... | ................<br>..........Y..... | ....C.............<br>............... | ......<br>...T |
| iPS:43 4583 | 21-225_74B6 | VK3\|A27/ JK4 | ............<br>........... | ...........<br>........ | ........<br>....... | ........<br>...T... | ................<br>..........Y..... | ....N.............<br>............... | ......<br>.... |
| iPS:43 4587 | 21-225_74G3 | VK3\|A27/ JK4 | .FM........C<br>W.......... | ...........<br>.......V | ........<br>....... | ........<br>...T... | ................<br>..........Y..... | ....C.............<br>............... | ......<br>...T |
| iPS:43 4603 | 21-225_77D11 | VK3\|A27/ JK4 | .FM........Y<br>W......I... | ...........<br>.......V | ........<br>....... | ........<br>...T... | ................<br>..........Y..... | ....C.............<br>............... | ......<br>...T |
| iPS:43 4705 | 21-225_80A2 | VK3\|A27/ JK4 | .FM........Y<br>........... | ...........<br>.......V | ........<br>....... | ........<br>...T... | ................<br>..........Y..... | ....C.............<br>............... | ......<br>...T |
| iPS:43 4747 | 21-225_80C12 | VK3\|A27/ JK4 | ...........Y<br>........... | ...........<br>........ | ........<br>....... | ........<br>...T... | ................<br>..........Y..... | ....N.............<br>............... | ......<br>.... |
| iPS:43 4793 | 21-225_82A5 | VK3\|A27/ JK4 | ............<br>W.......... | ...........<br>........ | ........<br>....... | ........<br>...T... | ................<br>..........Y..... | ....N.............<br>............... | ......<br>.... |
| iPS:43 4797 | 21-225_82G5 | VK3\|A27/ JK4 | .FM........Y<br>W.........S | .....E.....<br>.......V | ........<br>....... | ........<br>...T... | ................<br>..........Y..... | ....C.............<br>............... | ......<br>...T |
| iPS:43 4805 | 21-225_82D9 | VK3\|A27/ JK4 | .FM.........<br>W.......... | .....E.....<br>.......V | ........<br>....... | ........<br>...T... | ................<br>..........Y..... | ....C.............<br>............... | ......<br>...T |
| iPS:43 4813 | 21-225_82C12 | VK3\|A27/ JK4 | ............<br>W.......... | ...........<br>........ | ........<br>....... | ........<br>...T..S | ................<br>..........Y..... | ....N.............<br>............... | ......<br>.... |
| iPS:43 4825 | 21-225_83C2 | VK3\|A27/ JK4 | .FM........C<br>........... | ...........<br>.......V | ........<br>....... | ........<br>...T... | ................<br>..........Y..... | ....C.............<br>............... | ......<br>...T |
| iPS:43 4833 | 21-225_83C5 | VK3\|A27/ JK4 | .FM........C<br>W.......... | .....E.....<br>.......V | ........<br>....... | ........<br>...T... | ................<br>..........Y..... | ....C.............<br>............... | ......<br>...T |
| iPS:43 4883 | 21-225_85B5 | VK3\|A27/ JK4 | .FM.........<br>........... | .S.........<br>.......V | ........<br>....... | ........<br>...T... | ................<br>..........Y..... | ....C.............<br>............... | ......<br>...T |
| iPS:43 4911 | 21-225_85D11 | VK3\|A27/ JK4 | .FM.........<br>..T........ | .S.........<br>.......V | ........<br>....... | ........<br>...T... | ................<br>..........Y..... | ....C.............<br>............... | ......<br>...T |
| iPS:43 4957 | 21-225_87A10 | VK3\|A27/ JK4 | .F.........Y<br>........... | ...........<br>........ | ........<br>....... | ........<br>...T..S | ................<br>..........Y..... | ....N.............<br>............... | ......<br>.... |

| | | | | | | | | | |
|---|---|---|---|---|---|---|---|---|---|
| iPS:43 5247 | 21-225_96G1 | VK3\|A27/ JK4 | ............<br>..T........ | ............<br>........ | ........<br>P...... | ........<br>...T..S | .................<br>..........Y..... | ....N.............<br>............... | ......<br>.... |
| iPS:43 6368 | 21-225_211G3 | VK3\|A27/ JK4 | ............<br>........... | ............<br>.....F.. | ........<br>....... | ........<br>....... | .................<br>................. | ...V..............<br>............... | ......<br>.... |
| iPS:43 6426 | 21-225_215C7 | VK3\|A27/ JK4 | ............<br>......V.... | ......RITT.<br>....NF.. | ........<br>....... | ........<br>....... | .................<br>........S........ | ...V..............<br>...........L.. | ......<br>.... |
| iPS:43 6432 | 21-225_215H12 | VK3\|A27/ JK4 | ............<br>........... | ............<br>.....F.. | ........<br>.....M. | ........<br>......I | .................<br>................. | ...V..............<br>............... | ......<br>.... |
| iPS:43 7322 | 21-225_75B1 | VK3\|A27/ JK4 | .FM........Y<br>W.......I.S | ............<br>.......V | ........<br>....... | ........<br>...T... | .................<br>..........Y..... | ....C.............<br>............... | ......<br>...T |
| iPS:43 7377 | 21-225_74G9 | VK3\|A27/ JK4 | .FM.........<br>........... | ............<br>.......V | ........<br>....... | ........<br>...T... | .................<br>..........Y..... | ....C.............<br>............... | ......<br>...T |
| | | **Germline** | **K_FR1** | **K_CDR1** | **K_FR2** | **K_CDR2** | **K_FR3** | **K_CDR3** | **K_FR4** |
| | **VK4\|B3/JK4** | | DIVMTQSPDSLA VSLGERATINC | KSS-- QSVLYSSNNKN YLA | WYQQKPGQ PPKLLIY | W------- -ASTRES | GVPDRFSGSGSG-- TDFTLTISSLQAEDVAV YYC | QQYYS------------- ----------TPLT | FGGGTK VEIK |
| iPS:43 4511 | 21-225_74B11 | VK4\|B3/J K4 | ...........T<br>........... | .......I..N<br>...N.... | ........<br>....... | ........<br>....... | .................<br>I................ | .................<br>...........P. | ......<br>.... |
| iPS:43 4729 | 21-225_80B12 | VK4\|B3/J K4 | ...L........<br>........... | ..R.........<br>...Y...T | ........<br>....... | ........<br>....... | .................<br>................. | .................<br>.........S.P. | ......<br>.... |
| iPS:43 4851 | 21-225_75A6 | VK4\|B3/J K4 | ............<br>........... | ..R......H.<br>...Y.... | ........<br>..E.... | ........<br>....... | .................<br>................. | .................<br>...........P. | ......<br>.... |
| iPS:43 5623 | 21-225_162D5 | VK4\|B3/J K4 | .........FRN<br>..M....I..F | ..N..H....R<br>...NQ... | ...R....<br>....... | R.......<br>.T.I.K. | ..........C......<br>....D............ | .................<br>...........P. | ......<br>.... |
| iPS:44 6086 | 21-225_94D8 | VK4\|B3/J K4 | ...L........<br>........... | ..R.........<br>...Y...T | ........<br>....... | ........<br>....... | .................<br>................. | .................<br>.........S.P. | ......<br>.... |
| | | **Germline** | **K_FR1** | **K_CDR1** | **K_FR2** | **K_CDR2** | **K_FR3** | **K_CDR3** | **K_FR4** |
| | **VK2\|A19/JK 4** | | DIVMTQSPLSLP VTPGEPASISC | RSS-- QSLLHSN- GYNYLD | WYLQKPGQ SPQLLIY | L------- -GSNRAS | GVPDRFSGSGSG-- TDFTLKISRVEAEDVGV YYC | MQALQ------------- ----------TPLT | FGGGTK VEIK |
| iPS:43 4539 | 21-225_74A2 | VK2\|A19/ JK4 | ............<br>........... | ............<br>...H.... | .......R<br>....... | ........<br>....... | ...E.............<br>................. | ..P...............<br>...........F. | ......<br>.... |
| iPS:43 4563 | 21-225_75D8 | VK2\|A19/ JK4 | ............<br>........... | ............<br>S....... | ........<br>....... | ........<br>....... | ..............S..<br>..............L... | ....H.............<br>..........P... | ......<br>.... |
| iPS:43 5009 | 21-225_89G4 | VK2\|A19/ JK4 | ............<br>........... | ............<br>S....... | ........<br>....... | ........<br>....... | .................<br>............I.... | ....H.............<br>..........I... | ......<br>.... |
| iPS:43 5059 | 21-225_90C11 | VK2\|A19/ JK4 | ............<br>........... | .Y......V..<br>S....... | ........<br>....V.. | ........<br>....... | ..............S..<br>..............L... | ....H.............<br>..........P... | ......<br>.... |
| iPS:43 5103 | 21-225_92B2 | VK2\|A19/ JK4 | ............<br>........... | ........V..<br>S....... | ........<br>....V.. | ........<br>....... | ...........V.....<br>..R........I.I... | ....H.............<br>..........I... | ......<br>.... |
| iPS:43 5713 | 21-225_171D7 | VK2\|A19/ JK4 | ............<br>........... | .........YH<br>........ | .....T..<br>....... | V.......<br>....... | .................<br>................. | ..T...............<br>............... | ......<br>.... |

| | | | | | | | | | |
|---|---|---|---|---|---|---|---|---|---|
| iPS:43 6246 | 21-225_201G6 | VK2\|A19/ JK4 | ............<br>........... | ..........N<br>..R..H.. | ........<br>....... | ........<br>....... | .................<br>................. | ....-<br>..................<br>.....QTP. | ......<br>.... |
| iPS:43 6254 | 21-225_202C12 | VK2\|A19/ JK4 | ...L........<br>........... | ..........N<br>..K..H.. | ........<br>....... | ........<br>....... | .................<br>................. | ....-<br>..................<br>.....QTP. | ......<br>.... |
| iPS:43 6304 | 21-225_201F3 | VK2\|A19/ JK4 | ............<br>........... | ..........N<br>..R..H.. | ........<br>....... | ........<br>....... | .................<br>................. | ....-<br>..................<br>.....QTP. | ......<br>.... |
| iPS:43 6334 | 21-225_208G3 | VK2\|A19/ JK4 | ...L........<br>........... | ..........N<br>..K..H.. | ........<br>....... | ........<br>....... | .................<br>................. | ....-<br>..................<br>.....QTP. | ......<br>.... |
| iPS:43 7248 | 21-225_97H3 | VK2\|A19/ JK4 | ....I.......<br>........... | ...........<br>...H.... | .......R<br>....... | ........<br>....... | ...E.............<br>................. | ..P...............<br>..........F. | ......<br>.... |
| iPS:43 7320 | 21-225_75A1 | VK2\|A19/ JK4 | ............<br>........... | ...........<br>...H.... | .......R<br>....... | ........<br>....... | ...E.............<br>................. | ..P...............<br>..........F. | ......<br>.... |
| iPS:43 7371 | 21-225_74D8 | VK2\|A19/ JK4 | ............<br>........... | ........V..<br>S....... | ........<br>....V.. | ........<br>....... | ..............S..<br>.............L... | ....H.............<br>.........P... | ......<br>.... |
| iPS:39 2718 | 21-225_17B8 | VK2\|A19/ JK4 | ............<br>........... | ...........<br>...N.S.. | ........<br>....... | ........<br>...H... | .......D.........<br>................. | ..V...............<br>.........TP... | ......<br>.... |
| | | Germline | K_FR1 | K_CDR1 | K_FR2 | K_CDR2 | K_FR3 | K_CDR3 | K_FR4 |
| | VK3\|L2/JK1 | | EIVMTQSPATLS<br>VSPGERATLSC | RAS--QSVS--<br>----SNLA | WYQQKPGQ<br>APRLLIY | G-------<br>-ASTRAT | GIPARFSGSGSG--<br>TEFTLTISSLQSEDFAV<br>YYC | QQYNN-------------<br>----------WPWI | FGQGTK<br>VEIK |
| iPS:43 4871 | 21-225_85H1 | VK3\|L2/J K1 | ............<br>........... | ......D.I..<br>....TY.. | ........<br>....... | ........<br>....... | .V...............<br>.............L... | .E..D.............<br>............CS | ......<br>.... |
| iPS:43 5421 | 21-225_151F1 | VK3\|L2/J K1 | .M..........<br>.....V..... | .......IN..<br>....I.I. | ........<br>....... | ........<br>....... | .................<br>................. | ....D.............<br>.........WP... | ......<br>.... |
| iPS:43 5497 | 21-225_155H9 | VK3\|L2/J K1 | ............<br>........... | ...........<br>........ | ........<br>....... | ........<br>....... | .................<br>................. | ...DD.............<br>.........WP... | ......<br>.... |
| iPS:43 5605 | 21-225_161A4 | VK3\|L2/J K1 | ............<br>........S... | .S......N..<br>........ | ....R...<br>.L..... | ........<br>...I... | D....N...........<br>...............F. | ....-<br>..................<br>.....NW.. | .....T<br>.... |
| iPS:45 1118 | 21-225_191C8 | VK3\|L2/J K1 | ............<br>........... | ........R..<br>........ | ....E...<br>....... | ........<br>....... | .................<br>................. | ..SFT.............<br>...........LR. | ......<br>.... |
| iPS:39 2734 | 21-225_17D8 | VK3\|L2/J K1 | ........S...<br>........... | ...........<br>........ | .F......<br>......N | ........<br>......S | .................<br>................. | ..................<br>...........L. | ......<br>.... |
| iPS:39 2768 | 21-225_20B8 | VK3\|L2/J K1 | ........S...<br>.....V..... | ...........<br>........ | .F......<br>......N | ........<br>......S | .................<br>................. | ..................<br>..........C.L. | ......<br>.... |
| iPS:39 3044 | 21-225_25B8 | VK3\|L2/J K1 | ............<br>........... | ........R..<br>........ | ........<br>....... | ........<br>....... | .................<br>.............L... | ..................<br>.........WP..P | ......<br>.... |
| iPS:39 | 21-225_28C5 | VK3\|L2/J | ............ | ........... | ..H..... | ........ | ................. | .................. | ...... |

| | | | | | | | | | |
|---|---|---|---|---|---|---|---|---|---|
| iPS:39 3906 | 21-225_13D3 | VK3\|L2/J K1 | ............<br>.....S..... | ......T....<br>........ | .F......<br>......N | ........<br>....... | .................<br>...............F. | ...HD.............<br>............P. | ......<br>.... |
| | | Germline | K_FR1 | K_CDR1 | K_FR2 | K_CDR2 | K_FR3 | K_CDR3 | K_FR4 |
| | VK1\|L1/JK5 | | DIQMTQSPSSLS<br>ASVGDRVTITC | RAS--QGIS--<br>----NYLA | WFQQKPGK<br>APKSLIY | A-------<br>-ASSLQS | GVPSRFSGSGSG--<br>TDFTLTISSLQPEDFAT<br>YYC | QQYNS-------------<br>----------YPIT | FGQGTR<br>LEIK |
| iPS:43 4947 | 21-225_87B7 | VK1\|L1/J K5 | ............<br>........... | ...........<br>........ | ........<br>....... | ........<br>.....H. | ....K............<br>...........S..F. | LL.LT.............<br>............L. | ......<br>.... |
| iPS:43 5427 | 21-225_151C9 | VK1\|L1/J K5 | ............<br>........... | ...........<br>....K... | ........<br>....... | D.......<br>...R... | ....K............<br>.............S... | H..KH.............<br>............... | ......<br>.... |
| iPS:43 5529 | 21-225_157H7 | VK1\|L1/J K5 | ............<br>........... | ......D....<br>.....F.. | ........<br>.....VS | T.......<br>....... | ....K............<br>................. | ...H..............<br>............... | ......<br>.... |
| iPS:43 6066 | 21-225_194B7 | VK1\|L1/J K5 | ............<br>........... | ...........<br>....K... | ........<br>....... | G.......<br>...R... | ....K............<br>................. | .H.LN.............<br>............L. | ......<br>.... |
| iPS:43 7274 | 21-225_196D4 | VK1\|L1/J K5 | ............<br>........... | ...........<br>........ | ....N...<br>....... | ........<br>....... | ....K....C.......<br>.....P....V..... | .H.LN.............<br>............L. | ......<br>.... |
| iPS:39 2748 | 21-225_20A8 | VK1\|L1/J K5 | ............<br>........... | ........N..<br>.......V | ........<br>....... | ........<br>.....L. | ....K............<br>................. | ..................<br>............... | ......<br>.... |
| iPS:39 3062 | 21-225_33H3 | VK1\|L1/J K5 | ............<br>........... | Q.....D....<br>.....F.N | ..R.....<br>..N.... | D.......<br>...N.VT | ........R........<br>.F............... | ...DN.............<br>.........L... | ......<br>.... |
| iPS:39 8532 | 21-225_33B7 | VK1\|L1/J K5 | ............<br>........... | ......D....<br>........ | ........<br>..T.... | ........<br>....... | ....K............<br>...............F.. | ...H..............<br>............L. | ......<br>.... |
| iPS:40 2221 | 21-225_2C12 | VK1\|L1/J K5 | ............<br>........... | ...........<br>........ | .....S..<br>......S | ........<br>..T.... | ....Q............<br>................. | ...Y..............<br>............... | ......<br>.... |
| | | Germline | K_FR1 | K_CDR1 | K_FR2 | K_CDR2 | K_FR3 | K_CDR3 | K_FR4 |
| | VK4\|B3/JK5 | | DIVMTQSPDSLA<br>VSLGERATINC | KSS--<br>QSVLYSSNNKN<br>YLA | WYQQKPGQ<br>PPKLLIY | W-------<br>-ASTRES | GVPDRFSGSGSG--<br>TDFTLTISSLQAEDVAV<br>YYC | QQYYS-------------<br>----------TPIT | FGQGTR<br>LEIK |
| iPS:43 5665 | 21-225_169F2 | VK4\|B3/J K5 | ............<br>........... | ..........I<br>........ | ........<br>....... | ........<br>....... | .................<br>................. | ....-<br>..................<br>.....RAP. | ......<br>.... |
| iPS:43 5671 | 21-225_169H5 | VK4\|B3/J K5 | ............<br>........... | ..........I<br>........ | ........<br>....... | ........<br>....... | .................<br>................. | ....-<br>..................<br>.....RAP. | ......<br>.... |
| iPS:43 6554 | 21-225_224C10 | VK4\|B3/J K5 | ............<br>A.......T. | ..........N<br>........ | ........<br>....... | ........<br>....... | .................<br>.............I... | ....I.............<br>.........N.CS | ......<br>.... |
| iPS:39 8510 | 21-225_25A3 | VK4\|B3/J K5 | ...........T<br>........... | ...........<br>........ | ........<br>....... | ........<br>....... | .................<br>................. | ..................<br>............CS | ......<br>.... |
| iPS:39 8516 | 21-225_26A9 | VK4\|B3/J K5 | ............<br>........... | ...........<br>........ | ........<br>....... | ........<br>....... | .................<br>................. | ..................<br>.........S.CS | ......<br>.... |
| | | Germline | K_FR1 | K_CDR1 | K_FR2 | K_CDR2 | K_FR3 | K_CDR3 | K_FR4 |

| | | | | | | | | | |
|---|---|---|---|---|---|---|---|---|---|
| | VK2\|A19/JK 1 | | DIVMTQSPLSLP<br>VTPGEPASISC | RSS--<br>QSLLHSN-<br>GYNYLD | WYLQKPGQ<br>SPQLLIY | L-------<br>-GSNRAS | GVPDRFSGSGSG--<br>TDFTLKISRVEAEDVGV<br>YYC | MQALQ-------------<br>----------TPWT | FGQGTK<br>VEIK |
| iPS:43 5667 | 21-225_169E3 | VK2\|A19/ JK1 | ............<br>........... | ..........N<br>....K... | ........<br>....... | ........<br>....... | .......A.........<br>................ | ..V..............<br>.............. | ......<br>.... |
| iPS:43 5673 | 21-225_169E6 | VK2\|A19/ JK1 | ............<br>........... | ..........N<br>....K... | ........<br>....... | ........<br>....... | .......A.........<br>................ | ..V..............<br>.............. | ......<br>.... |
| iPS:43 5759 | 21-225_176E6 | VK2\|A19/ JK1 | ............<br>........... | ..........N<br>....K... | ........<br>....... | ........<br>....... | .......A.........<br>................ | ..V..............<br>.............. | ......<br>.... |
| | | Germline | K_FR1 | K_CDR1 | K_FR2 | K_CDR2 | K_FR3 | K_CDR3 | K_FR4 |
| | VK6\|A26/JK 1 | | EIVLTQSPDFQS<br>VTPKEKVTITC | RAS--QSIG--<br>----SSLH | WYQQKPDQ<br>SPKLLIK | Y-------<br>-ASQSFS | GVPSRFSGSGSG--<br>TDFTLTINSLEAEDAAT<br>YYC | HQSSS-------------<br>----------LPWT | FGQGTK<br>VEIK |
| iPS:43 5817 | 21-225_190B11 | VK6\|A26/ JK1 | ............<br>.A......... | ...........<br>.....N.. | ........<br>....... | S.......<br>....... | .................<br>........T....... | Q................<br>.............. | ......<br>.... |
| iPS:43 5823 | 21-225_190F11 | VK6\|A26/ JK1 | ......F..S..<br>........... | ......N....<br>........ | ......E.<br>...V... | ........<br>....... | .................<br>................ | .................<br>..........F.R. | ......<br>.... |
| iPS:43 5867 | 21-225_191E5 | VK6\|A26/ JK1 | ......F..S..<br>........... | ...........<br>........ | ........<br>....... | ........<br>....... | .................<br>................ | .................<br>..........F.R. | ......<br>.... |
| iPS:43 5917 | 21-225_190D5 | VK6\|A26/ JK1 | ............<br>.A......... | ...........<br>.....N.. | ........<br>....... | S.......<br>....... | .................<br>........T....... | Q................<br>.............. | ......<br>.... |
| iPS:43 5929 | 21-225_190D9 | VK6\|A26/ JK1 | ......F..S..<br>........... | ...........<br>........ | ........<br>....... | ........<br>....... | .................<br>................ | .................<br>..........F.R. | ......<br>.... |
| iPS:43 5935 | 21-225_190H8 | VK6\|A26/ JK1 | ......F..S..<br>........... | ...........<br>........ | ........<br>....... | ........<br>....... | .................<br>................ | .................<br>..........F.R. | ......<br>.... |
| iPS:43 6056 | 21-225_194C3 | VK6\|A26/ JK1 | ............<br>.A......... | ...........<br>.....N.. | ........<br>....... | S.......<br>....... | .................<br>........T....... | Q................<br>.............. | ......<br>.... |
| iPS:43 6216 | 21-225_200B7 | VK6\|A26/ JK1 | ............<br>........... | ......N....<br>....NT.. | ........<br>....... | ........<br>....... | .................<br>I............... | ...G.............<br>............Q. | ......<br>.... |
| iPS:43 6220 | 21-225_200F8 | VK6\|A26/ JK1 | ............<br>.A......... | ...........<br>.....N.. | ........<br>....... | S.......<br>....... | .................<br>........T....... | Q................<br>.............. | ......<br>.... |
| iPS:43 6448 | 21-225_217A3 | VK6\|A26/ JK1 | ..........K.<br>........... | ...........<br>........ | ........<br>.....V. | ........<br>....... | .................<br>...........G.... | ...R.............<br>.............. | ......<br>.... |
| | | Germline | K_FR1 | K_CDR1 | K_FR2 | K_CDR2 | K_FR3 | K_CDR3 | K_FR4 |
| | VK6\|A26/JK 4 | | EIVLTQSPDFQS<br>VTPKEKVTITC | RAS--QSIG--<br>----SSLH | WYQQKPDQ<br>SPKLLIK | Y-------<br>-ASQSFS | GVPSRFSGSGSG--<br>TDFTLTINSLEAEDAAT<br>YYC | HQSSS-------------<br>----------LPLT | FGGGTK<br>VEIK |
| iPS:43 5829 | 21-225_190B12 | VK6\|A26/ JK4 | ............<br>........... | ...........<br>........ | ........<br>....... | ........<br>....... | .D...............<br>................ | ..TR.............<br>.............. | ......<br>.... |
| iPS:43 5863 | 21-225_191H4 | VK6\|A26/ JK4 | ............<br>........... | ..N........<br>........ | ........<br>....... | ........<br>.....L. | .......A.........<br>.......D........ | ..TGR............<br>.............. | ......<br>.... |
| iPS:43 | 21- | VK6\|A26/ | ............ | ........... | ........ | ........ | .D............... | ..TR............. | ...... |

| | | | | | | | | | |
|---|---|---|---|---|---|---|---|---|---|
| iPS:43 5983 | 21-225_192E5 | VK6\|A26/JK4 | ............<br>........... | ...........<br>....R... | ........<br>....... | ........<br>....... | .................<br>................. | ....R...........<br>.............. | ......<br>.... |
| iPS:43 6043 | 21-225_193G9 | VK6\|A26/JK4 | ............<br>........... | ...........<br>....R... | ....R...<br>.L..... | ........<br>....... | .................<br>...............F. | ....R...........<br>.............. | ......<br>.... |
| iPS:43 6084 | 21-225_195F2 | VK6\|A26/JK4 | ............<br>........... | ...........<br>........ | ........<br>....... | ........<br>....... | .................<br>A...S............ | ...RT...........<br>.............. | ......<br>.... |
| iPS:43 6094 | 21-225_195B10 | VK6\|A26/JK4 | ............<br>........... | ...........<br>........ | ........<br>....... | ........<br>....... | .................<br>................. | ...GR...........<br>.............. | ......<br>.... |
| iPS:43 6240 | 21-225_201E8 | VK6\|A26/JK4 | ........A...<br>........... | ......N....<br>....R... | ........<br>....... | ........<br>....... | ...............N.<br>..........V.... | ...R............<br>.............. | ......<br>...R |
| iPS:43 6314 | 21-225_206G4 | VK6\|A26/JK4 | ............<br>........... | ...........<br>....R... | ........<br>....... | ........<br>....... | .................<br>................. | ...R............<br>.............. | ......<br>.... |
| | | Germline | K_FR1 | K_CDR1 | K_FR2 | K_CDR2 | K_FR3 | K_CDR3 | K_FR4 |
| | VK1\|A20/JK3 | | DIQMTQSPSSLS<br>ASVGDRVTITC | RAS--QGIS--<br>----NYLA | WYQQKPGK<br>VPKLLIY | A-------<br>-ASTLQS | GVPSRFSGSGSG--<br>TDFTLTISSLQPEDVAT<br>YYC | QKYNS-----------<br>----------APFT | FGPGTK<br>VDIK |
| iPS:43 5833 | 21-225_190D12 | VK1\|A20/JK3 | ............<br>........... | ...........<br>........ | ........<br>....... | V.......<br>....... | .................<br>................. | ................<br>.............. | ......<br>.... |
| iPS:43 6019 | 21-225_193C4 | VK1\|A20/JK3 | ............<br>........... | .P.........<br>....I... | .......N<br>....... | ........<br>....... | .................<br>................. | ................<br>.............. | ......<br>.... |
| iPS:39 4010 | 21-225_12G5 | VK1\|A20/JK3 | ............<br>.........P. | ......D....<br>........ | ........<br>....... | ........<br>..YI... | .................<br>.............A... | ...D............<br>.............. | ......<br>.... |
| | | Germline | K_FR1 | K_CDR1 | K_FR2 | K_CDR2 | K_FR3 | K_CDR3 | K_FR4 |
| | VK6\|A26/JK3 | | EIVLTQSPDFQS<br>VTPKEKVTITC | RAS--QSIG--<br>----SSLH | WYQQKPDQ<br>SPKLLIK | Y-------<br>-ASQSFS | GVPSRFSGSGSG--<br>TDFTLTINSLEAEDAAT<br>YYC | HQSSS-----------<br>----------LPFT | FGPGTK<br>VDIK |
| iPS:43 6025 | 21-225_193B5 | VK6\|A26/JK3 | ............<br>........... | ...........<br>........ | ........<br>....... | ........<br>....... | .................<br>................. | ....R...........<br>.............. | ......<br>.... |
| iPS:43 6096 | 21-225_195E10 | VK6\|A26/JK3 | ............<br>........... | ...........<br>........ | ........<br>....... | ........<br>....... | .................<br>................. | ....R...........<br>.............. | ......<br>.... |
| iPS:43 6408 | 21-225_214H8 | VK6\|A26/JK3 | ............<br>........... | ...........<br>....V... | ........<br>..Q.... | ........<br>....... | .................<br>................. | ...R............<br>.............. | ....S.<br>.... |
| iPS:43 6424 | 21-225_215H6 | VK6\|A26/JK3 | ............<br>........... | ...........<br>....V... | ........<br>..Q.... | ........<br>.....L. | .................<br>................. | ...R............<br>.............. | ....S.<br>.... |
| | | Germline | K_FR1 | K_CDR1 | K_FR2 | K_CDR2 | K_FR3 | K_CDR3 | K_FR4 |
| | VK1\|L5/JK2 | | DIQMTQSPSSVS<br>ASVGDRVTITC | RAS--QGIS--<br>----SWLA | WYQQKPGK<br>APKLLIY | A-------<br>-ASSLQS | GVPSRFSGSGSG--<br>TDFTLTISSLQPEDFAT<br>YYC | QQANS-----------<br>----------FPYT | FGQGTK<br>LEIK |
| iPS:43 6082 | 21-225_195D9 | VK1\|L5/JK2 | ............<br>........... | ...........<br>....R... | ........<br>....... | ........<br>.....LG | .................<br>................. | .R..............<br>............CS | ......<br>.... |
| iPS:43 6118 | 21-225_196A10 | VK1\|L5/JK2 | ......Y..Y..<br>.......S... | ...........<br>....R... | ........<br>.A.F... | ........<br>.....LG | ..S..............<br>.............I... | .RD.............<br>..........L.CS | ......<br>.... |

| | | | | | | | | | |
|---|---|---|---|---|---|---|---|---|---|
| | VK2\|A19/JK2 | | DIVMTQSPLSLP<br>VTPGEPASISC | RSS--<br>QSLLHSN-<br>GYNYLD | WYLQKPGQ<br>SPQLLIY | L-------<br>-GSNRAS | GVPDRFSGSGSG--<br>TDFTLKISRVEAEDVGV<br>YYC | MQALQ------------<br>----------TPYT | FGQGTK<br>LEIK |
| iPS:436362 | 21-225_210C12 | VK2\|A19/JK2 | ............<br>........... | ..........Y<br>...H.F.. | ........<br>....... | ........<br>.V..... | .................<br>................. | ..................<br>.........TPMCS | ......<br>.... |
| iPS:436374 | 21-225_211C10 | VK2\|A19/JK2 | ............<br>........... | ...........<br>........ | ...L....<br>....... | ........<br>....... | .................<br>......M......I... | ....L.............<br>.........TPVCS | ......<br>.... |
| | | Germline | K_FR1 | K_CDR1 | K_FR2 | K_CDR2 | K_FR3 | K_CDR3 | K_FR4 |
| | VK2\|A17/JK1 | | DVVMTQSPLSLP<br>VTLGQPASISC | RSS--<br>QSLVYSD-<br>GNTYLN | WFQQRPGQ<br>SPRRLIY | K-------<br>-VSNRDS | GVPDRFSGSGSG--<br>TDFTLKISRVEAEDVGV<br>YYC | MQGTH------------<br>----------WPWT | FGQGTK<br>VEIK |
| iPS:437226 | 21-225_57C2 | VK2\|A17/JK1 | ............<br>........... | ...........<br>........ | ........<br>....... | E.......<br>....W.. | ...N.............<br>.....A........... | V.................<br>...........R. | ......<br>.... |
| | | Germline | K_FR1 | K_CDR1 | K_FR2 | K_CDR2 | K_FR3 | K_CDR3 | K_FR4 |
| | VK1\|A20/JK5 | | DIQMTQSPSSLS<br>ASVGDRVTITC | RAS--QGIS--<br>----NYLA | WYQQKPGK<br>VPKLLIY | A-------<br>-ASTLQS | GVPSRFSGSGSG--<br>TDFTLTISSLQPEDVAT<br>YYC | QKYNS------------<br>----------APIT | FGQGTR<br>LEIK |
| iPS:392726 | 21-225_20B5 | VK1\|A20/JK5 | ............<br>........... | ........N..<br>........ | ........<br>I...... | ........<br>....... | .................<br>...............C. | ..................<br>.........AP... | ......<br>.... |
| iPS:392792 | 21-225_20G12 | VK1\|A20/JK5 | ............<br>........... | ...........<br>........ | ........<br>...V... | T.......<br>....... | .................<br>...V............. | ..................<br>.........AP... | ......<br>.... |
| iPS:398478 | 21-225_17C10 | VK1\|A20/JK5 | ..........Q.<br>........... | ...........<br>........ | .......R<br>....... | ........<br>....... | .................<br>.........D....... | ..................<br>.........AP.L. | ......<br>.... |
| | | Germline | K_FR1 | K_CDR1 | K_FR2 | K_CDR2 | K_FR3 | K_CDR3 | K_FR4 |
| | VK1\|L1/JK2 | | DIQMTQSPSSLS<br>ASVGDRVTITC | RAS--QGIS--<br>----NYLA | WFQQKPGK<br>APKSLIY | A-------<br>-ASSLQS | GVPSRFSGSGSG--<br>TDFTLTISSLQPEDFAT<br>YYC | QQYNS------------<br>----------YPYT | FGQGTK<br>LEIK |
| iPS:392776 | 21-225_21A12 | VK1\|L1/JK2 | ............<br>........... | ...........<br>....K... | ........<br>....... | ........<br>....... | ....K............<br>................. | ..................<br>............FR | ......<br>.... |
| | | Germline | K_FR1 | K_CDR1 | K_FR2 | K_CDR2 | K_FR3 | K_CDR3 | K_FR4 |
| | VK1\|A30/JK3 | | DIQMTQSPSSLS<br>ASVGDRVTITC | RAS--QGIR--<br>----NDLG | WYQQKPGK<br>APKRLIY | A-------<br>-ASSLQS | GVPSRFSGSGSG--<br>TEFTLTISSLQPEDFAT<br>YYC | LQHNS------------<br>----------YPFT | FGPGTK<br>VDIK |
| iPS:437240 | 21-225_84H12 | VK1\|A30/JK3 | .........YQ.<br>........... | ...........<br>........ | .F......<br>....... | ........<br>....... | .................<br>................. | ....D.............<br>.............. | ......<br>.... |
| iPS:434577 | 21-225_75C11 | VK1\|A30/JK3 | ............<br>........... | ...........<br>........ | .F......<br>....... | ........<br>....... | .................<br>................. | ....D.............<br>.............. | ......<br>.... |
| iPS:434553 | 21-225_76H12 | VK1\|A30/JK3 | ............<br>........... | ...........<br>........ | .F......<br>....... | ........<br>...R... | .................<br>................. | ....D.............<br>.............. | ......<br>.... |
| iPS:434927 | 21-225_86E5 | VK1\|A30/JK3 | ............<br>........... | ...........<br>........ | ........<br>....... | ........<br>....... | .................<br>...............H. | ....D.............<br>.............. | ......<br>.E.. |
| | | Germline | K_FR1 | K_CDR1 | K_FR2 | K_CDR2 | K_FR3 | K_CDR3 | K_FR4 |

| | | | | | | | | | |
|---|---|---|---|---|---|---|---|---|---|
| | VK1\|L5/JK1 | | DIQMTQSPSSVS ASVGDRVTITC | RAS--QGIS-- ----SWLA | WYQQKPGK APKLLIY | A------- -ASSLQS | GVPSRFSGSGSG-- TDFTLTISSLQPEDFAT YYC | QQANS----------- ---------FPWT | FGQGTK VEIK |
| iPS:43 5477 | 21-225_154E8 | VK1\|L5/JK1 | ............ ..I........ | ......F.... ........ | ........ ....... | T....... ....... | .................. ..............C. | .................. .............. | ...... ..FN |
| iPS:43 5385 | 21-225_149G7 | VK1\|L5/JK1 | ............ ........... | ......F.... ........ | ........ ...F... | ........ ....... | .................. ................. | .................. .............. | ...... ...N |
| LAMBDA_VARIABLE | | | | | | | | | |
| | | Germline | L_FR1 | L_CDR1 | L_FR2 | L_CDR2 | L_FR3 | L_CDR3 | L_FR4 |
| | VL3\|3r/JL2 | | SYELTQP- PSVSVSPGQTAS ITC | SGD---KLGD- ----KYAC | WYQQKPGQ SPVLVIY | Q------- -DSKRPS | GIPERFSGSNSG-- NTATLTISGTQAMDEAD YYC | QAWDS----------- ---------STAVV | FGGGTK LTVL |
| iPS:45 3445 | 21-225_148E10 | VL3\|3r/JL2 | ............ ........... | .........N. ......V. | ....R..H AA..I.. | ........ ....... | .................. ..............F. | ....R............ ........N.Y.. | ...... .... |
| iPS:47 2742 | 21-225_30D9_LC2 | VL3\|3r/JL2 | ............ ........... | ........... ......VY | .F...... ....... | ........ ..R.... | .................. .........L....... | ....N............ .........-STA. | ...... .... |
| iPS:47 2743 | 21-225_68G6 | VL3\|3r/JL2 | ...V........ ........... | ........... ......TY | .....A.. ..F.... | ........ ..R.... | ...D.............. ...........A..F.. | ....N............ .........-STA. | ...... .... |
| iPS:43 6652 | 21-225_146B11 | VL3\|3r/JL2 | ............ ........... | ........... .......S | ........ ....... | ........ ....... | .................. ......I.......... | .................. .........-ST.. | ...... .... |
| iPS:43 6654 | 21-225_146C11 | VL3\|3r/JL2 | ............ ........... | ........... .......S | ........ ....... | ........ ....... | .................. ......I.......... | .................. .........-ST.. | ...... .... |
| iPS:43 6658 | 21-225_146A2 | VL3\|3r/JL2 | ............ ........... | ........... .......S | ........ ....... | ........ ....... | .................. ......I.......... | .................. .........-ST.. | ...... .... |
| iPS:43 6664 | 21-225_147E7 | VL3\|3r/JL2 | ............ ........... | ........... .......S | ........ ....... | ........ ....... | .................. ......I.......... | .................. .........-ST.. | ...... .... |
| iPS:43 6666 | 21-225_147B8 | VL3\|3r/JL2 | ............ ........... | ........... ......V. | ........ ..E.... | ........ ..R.... | .................. ................. | ...G.............. ........N.... | ...... .... |
| iPS:43 6668 | 21-225_147B9 | VL3\|3r/JL2 | ............ ........... | ........... ......VS | ....R... ....... | ........ ..R.... | .................. .......L.V...... | L................. ..........F.. | ...... .... |
| iPS:43 6670 | 21-225_147D9 | VL3\|3r/JL2 | ............ ..........S | .........N. ......V. | ....R... ....... | ........ ....... | .................. ................. | ....R............ ........N.Y.. | ...... .... |
| iPS:43 6672 | 21-225_147F9 | VL3\|3r/JL2 | ............ ........... | ......E..N. ........ | ........ ....... | ........ ....... | .................. ................. | ...H.............. .........-ST.. | ...... .... |
| iPS:43 6674 | 21-225_147G9 | VL3\|3r/JL2 | ............ ........... | ........... ........ | ........ ....... | ........ ..R.... | .................. ................. | ...H.............. .........-ST.. | ...... .... |
| iPS:43 6676 | 21-225_147E11 | VL3\|3r/JL2 | ............ ........... | ........... .......S | ........ ....... | ........ ....... | .................. ......I.......... | .................. .........-ST.. | ...... .... |
| iPS:43 6678 | 21-225_147B12 | VL3\|3r/JL2 | ............ ........... | ........... .......S | ........ ....... | ........ ....... | ...............A. ......I.......... | .................. .........-ST.. | ...... .... |

| | | | | | | | | | |
|---|---|---|---|---|---|---|---|---|---|
| iPS:43 6688 | 21- 225_148C8 | VL3\|3r/JL 2 | .............<br>........... | ...........<br>......VS | ........<br>..I.... | ........<br>..R.... | .T................<br>................ | L..................<br>...........F.. | ......<br>.... |
| iPS:43 6690 | 21- 225_148A9 | VL3\|3r/JL 2 | .............<br>........... | .........N.<br>......V. | ........<br>....... | ........<br>....... | .................<br>................ | ...H..............<br>.........-ST.. | ......<br>.... |
| iPS:43 6694 | 21- 225_148G11 | VL3\|3r/JL 2 | .............<br>........... | ...........<br>.....F.S | ........<br>....... | ........<br>....... | .................<br>......I......... | ..................<br>.........-ST.. | ......<br>.... |
| iPS:43 6698 | 21- 225_149B5 | VL3\|3r/JL 2 | .............<br>........... | ..Y......Y.<br>......V. | ........<br>......F | ........<br>.NNQ... | .................<br>S................ | ..................<br>.........-ST.. | ......<br>.... |
| iPS:43 6700 | 21- 225_149C7 | VL3\|3r/JL 2 | .............<br>........... | ..N........<br>.......S | ........<br>....... | ........<br>....... | .........K.......<br>......I......... | ..................<br>.........-ST.. | ......<br>...P |
| iPS:43 6704 | 21- 225_149C10 | VL3\|3r/JL 2 | .............<br>........... | ...........<br>.......S | ........<br>....... | ........<br>..N.... | .................<br>....G.I......... | ..................<br>.........-ST.. | ......<br>.... |
| iPS:43 6706 | 21- 225_149A11 | VL3\|3r/JL 2 | .............<br>........... | .........N.<br>......VS | ........<br>....... | ........<br>...R... | .................<br>................ | L..................<br>...........F.. | ......<br>.... |
| iPS:43 6708 | 21- 225_150D3 | VL3\|3r/JL 2 | .............<br>........... | ......E..N.<br>........ | ........<br>....... | ........<br>..N.... | .........S.......<br>................ | ...H..............<br>.........-ST.. | ......<br>.... |
| iPS:43 6710 | 21- 225_150F6 | VL3\|3r/JL 2 | .............<br>........... | ...........<br>.......S | ........<br>......C | ........<br>....... | .................<br>......I.......C. | ..................<br>.........-ST.. | ......<br>.... |
| iPS:43 6714 | 21- 225_150H11 | VL3\|3r/JL 2 | .............<br>........... | ...........<br>.......S | ........<br>....... | ........<br>....... | .................<br>......I......F.. | ..................<br>.........-ST.. | ......<br>.... |
| iPS:43 6716 | 21- 225_151F3 | VL3\|3r/JL 2 | .............<br>........... | ...........<br>......V. | ........<br>....... | ........<br>..R.... | .................<br>................ | ...H..............<br>.........-ST.. | ......<br>.... |
| iPS:43 6718 | 21- 225_151H5 | VL3\|3r/JL 2 | .............<br>.........A. | ...........<br>.......S | ........<br>....... | ........<br>....... | .................<br>......I......... | ..................<br>.........-ST.. | ......<br>.... |
| iPS:43 6722 | 21- 225_151H7 | VL3\|3r/JL 2 | ..D.........<br>........... | ...........<br>.......S | ........<br>....... | ........<br>....... | .................<br>......I......... | ..................<br>.........-ST.. | ......<br>.... |
| iPS:43 6724 | 21- 225_151B9 | VL3\|3r/JL 2 | .............<br>.........A. | ......N....<br>.......S | ........<br>....... | ........<br>....... | .................<br>......I......... | ..................<br>.........-ST.. | ......<br>.... |
| iPS:43 6728 | 21- 225_152G6 | VL3\|3r/JL 2 | .............<br>........... | ...........<br>.......S | ........<br>....... | ........<br>....... | .................<br>......I..L...... | ....N.............<br>.........-ST.. | ......<br>.... |
| iPS:43 6730 | 21- 225_152D7 | VL3\|3r/JL 2 | .............<br>........... | ...........<br>.......S | ........<br>....... | ........<br>....... | .................<br>......I......... | ..................<br>.........-ST.. | ......<br>.... |
| iPS:43 6736 | 21- 225_153E8 | VL3\|3r/JL 2 | .............<br>........... | ..S......N.<br>......V. | ....R...<br>....... | ........<br>..N.... | .................<br>.............G... | ..................<br>...........Y.I | ......<br>.... |
| iPS:43 6738 | 21- 225_153D9 | VL3\|3r/JL 2 | .............<br>........... | ...........<br>........ | ........<br>....... | ........<br>..R.... | .................<br>................ | ...H..............<br>.........-ST.. | ......<br>V... |
| iPS:43 6740 | 21- 225_154C3 | VL3\|3r/JL 2 | .............<br>........... | ...........<br>......V. | ........<br>....... | ........<br>..R.... | .................<br>....T........... | ...H..............<br>.........-ST.. | ......<br>.... |
| iPS:43 6742 | 21- 225_154C4 | VL3\|3r/JL 2 | .............<br>.......R... | ...........<br>.......S | ........<br>....... | ........<br>....... | .................<br>......I......... | ..................<br>.........-ST.. | ......<br>.... |
| iPS:43 6744 | 21- 225_154F4 | VL3\|3r/JL 2 | .............<br>........... | .........N.<br>......V. | ........<br>...E... | K.......<br>....... | ................G<br>................ | ....N.............<br>.........-STL. | ......<br>.... |

| | | | | | | | | | |
|---|---|---|---|---|---|---|---|---|---|
| iPS:436746 | 21-225_154E10 | VL3\|3r/JL2 | ....................... | ...................S | ............... | ............... | ........................I........... | ....N....................-ST.. | .......... |
| iPS:436748 | 21-225_154D11 | VL3\|3r/JL2 | ....................... | ................... | ............... | .........K.... | ...............I................... | ...H...............-SI.. | .......... |
| iPS:436756 | 21-225_146A10 | VL3\|3r/JL2 | ....................... | .................V. | ..............F | ..........R.... | .................................... | ...................-ST.. | ......V... |
| iPS:436758 | 21-225_155C10 | VL3\|3r/JL2 | ..................V.... | .................VS | ............... | ............... | ........................I........... | ...................-ST.. | .......... |
| iPS:436760 | 21-225_155E10 | VL3\|3r/JL2 | ....................... | .................VS | ............... | ..........R.... | .T.................................. | L.....................F.. | .......... |
| iPS:436764 | 21-225_158E9 | VL3\|3r/JL2 | ..D.................... | .................V. | ............... | ............... | ......................T.........N... | ....N....................SF.L | .......... |
| iPS:436766 | 21-225_158D10 | VL3\|3r/JL2 | ...........T........... | .................V. | ............... | ..........R.... | .....L.............L....... | ...GN....................SF.. | .......... |
| iPS:436768 | 21-225_159H8 | VL3\|3r/JL2 | ....................... | .................V. | ............... | ..........R.... | ...........................L....... | ...GN....................SF.. | .......... |
| iPS:436770 | 21-225_160B12 | VL3\|3r/JL2 | .D....S................ | .................V. | ............... | ............... | ...........................L....... | ...GN....................SF.. | .......... |
| iPS:436772 | 21-225_161H3 | VL3\|3r/JL2 | ....................... | ......R..........V. | ..............F | ..........N.... | ...........................L....... | ...VN...................N.... | .......... |
| iPS:436774 | 21-225_161E10 | VL3\|3r/JL2 | .FD.................... | .................V. | ............... | ............... | .....I............................. | .T..N....................SFAL | .......... |
| iPS:436776 | 21-225_161F12 | VL3\|3r/JL2 | ....................... | ................... | ............... | ..........T.... | .................................... | ...................-.TL. | .......... |
| iPS:436780 | 21-225_165H3 | VL3\|3r/JL2 | ....................... | ................... | ............... | ............... | .................................... | ...................-.TL. | .......... |
| iPS:436782 | 21-225_166G11 | VL3\|3r/JL2 | ....S.................. | .................VH | ............... | ............... | .................................... | ....N....................-STA. | .......... |
| iPS:436784 | 21-225_169C1 | VL3\|3r/JL2 | ....................... | .................V. | ..........L.... | K.........I.... | .................................... | ....T....................-NT.I | .......... |
| iPS:436786 | 21-225_169A6 | VL3\|3r/JL2 | ....................... | .................V. | ...R........... | ..........Y.... | .................................... | ....T....................-NT.L | .......... |
| iPS:436788 | 21-225_169B7 | VL3\|3r/JL2 | A.D.................R... | .........G........S | ............... | ..........R.... | .................................... | ....K....................-NT.. | .......... |
| iPS:436790 | 21-225_169G11 | VL3\|3r/JL2 | ....................... | ................... | ............... | ............... | .................................... | ....N....................-STA. | .......... |
| iPS:436794 | 21-225_170F1 | VL3\|3r/JL2 | ....S.................. | .................S. | ............... | ............... | ...A................................ | ...................-NTA. | .......... |
| iPS:436796 | 21-225_170A5 | VL3\|3r/JL2 | ..D.................... | ................... | ..........I.... | ..........Y.... | .................................... | ....N....................-STM. | .....R.... |
| iPS:436798 | 21-225_171F5 | VL3\|3r/JL2 | A.D.................... | .........G........S | ............... | ..........R.... | .................................... | ....K....................-NT.. | .......... |

| | | | | | | | | | |
|---|---|---|---|---|---|---|---|---|---|
| iPS:43 6802 | 21-225_171E12 | VL3\|3r/JL 2 | ............ ........... | ........... ....... | ........ ....... | ........ ..R.... | ................ ................ | ....I............. ...........Y.. | ...... .... |
| iPS:43 6808 | 21-225_173F8 | VL3\|3r/JL 2 | ............ ........... | ..N......N. .....V. | ....R... ......S | ........ ...R... | ................ ................ | .................. .........-FT.. | ...... .... |
| iPS:43 6812 | 21-225_175C6 | VL3\|3r/JL 2 | ..D......... ........... | ........... ....... | ........ ..I.... | ........ ..Y.... | ................ ................ | ....N............. .........-STM. | .....R .... |
| iPS:43 6818 | 21-225_179C7 | VL3\|3r/JL 2 | ............ ........... | ........... .....V. | ....R... ....... | ........ ....... | ................ ....R........... | .................. .........N.... | ...... .... |
| iPS:43 6822 | 21-225_180D4 | VL3\|3r/JL 2 | ......T..... ........... | ......R.... ....... | ........ ....... | E....... ..R.... | ................ ............G... | .................. .........-RK.. | ...... .... |
| iPS:43 6824 | 21-225_180C5 | VL3\|3r/JL 2 | ............ ........... | .........E. ....... | ........ ....... | ........ ..R.... | ................ ................ | .................. .........-STA. | ...... .... |
| iPS:43 6826 | 21-225_180G5 | VL3\|3r/JL 2 | ............ .Y......... | ........... .....VS | ........ ....... | ........ ....... | ..............P. S............... | ....I............. .........-.TA. | ...... .... |
| iPS:43 6828 | 21-225_181H1 | VL3\|3r/JL 2 | ......T..... ........... | ........... ....... | ........ ....... | E....... ..R.... | ................ ................ | .................. .........-RK.. | ...... .... |
| iPS:43 6836 | 21-225_52H1 | VL3\|3r/JL 2 | ...........F ..........S | ........... .....VS | ........ ....... | ........ ..R.... | ................ ................ | ....N............. .........-ST.. | ...... .... |
| iPS:43 6848 | 21-225_57F1 | VL3\|3r/JL 2 | ..........A. ........... | .........E. ....... | ........ ....... | ........ ..R.... | ................ ................ | .................. .........-ST.. | ...... .... |
| iPS:43 6850 | 21-225_57D9 | VL3\|3r/JL 2 | ............ ........... | ..E......E. ....F.. | .S...... ....... | ........ ....... | ................ ................ | .................. .........-ST.. | ...... .... |
| iPS:43 6852 | 21-225_57H11 | VL3\|3r/JL 2 | ..A.......A. ........... | .........E. ....... | ........ ....... | ........ ..R.... | ................ ................ | .................. .........-ST.. | ...... .... |
| iPS:43 6854 | 21-225_58C1 | VL3\|3r/JL 2 | ............ .......N... | .........N. ....... | ........ ....... | ........ ..R.... | ................ ................ | ....- .................. ....-SSTA | ...... .... |
| iPS:43 6858 | 21-225_58E7 | VL3\|3r/JL 2 | ......S..... ........... | ........... ......T. | ...K.... ....... | ........ ..N.... | ................ ..............F. | ...NN............. .........-YT.. | ...... ..A. |
| iPS:43 6860 | 21-225_58F7 | VL3\|3r/JL 2 | ............ ........... | ........... ....... | ........ ....... | ........ ..R.... | ................ ................ | .................. .........-ST.. | ...... .... |
| iPS:43 6862 | 21-225_58F8 | VL3\|3r/JL 2 | ............ ........... | .........N. ....... | ........ ....... | ........ ....... | ................ ................ | .................. .........-ST.. | ...... .... |
| iPS:43 6864 | 21-225_58G11 | VL3\|3r/JL 2 | ............ ........... | ........... ......S | ........ ....... | ........ ..N.... | ................ ................ | ...NN............. .........-NT.M | ...... .... |
| iPS:43 6866 | 21-225_59F2 | VL3\|3r/JL 2 | ............ ........... | ........... ......S | ....R... ....... | ........ ..N.... | ................ ................ | ....N............. .........-NT.. | ...... .... |
| iPS:43 6868 | 21-225_59B11 | VL3\|3r/JL 2 | ............ ........... | ........... ....... | ........ ....... | ........ ....... | ..L............. ................ | .................. ...........Y.. | ...... .... |
| iPS:43 6870 | 21-225_60B1 | VL3\|3r/JL 2 | ..........A. ........... | .........E. ....... | ........ ....... | ........ ..R.... | ................ ................ | .................. .........-ST.. | ...... .... |
| iPS:43 | 21-225_60D2 | VL3\|3r/JL | ............ | ..N........ | ....R... | ........ | ................ | ....N............. | ...... |

| | | | | | | | | | |
|---|---|---|---|---|---|---|---|---|---|
| iPS:43 6874 | 21-225_60A12 | VL3\|3r/JL 2 | ............<br>.......N... | .........N.<br>........ | ........<br>....... | ........<br>..R.... | ..................<br>I............... | ....-<br>...................<br>....-SSTA | ......<br>.... |
| iPS:43 6876 | 21-225_61F5 | VL3\|3r/JL 2 | ............<br>........... | .........E.<br>........ | ........<br>....... | ........<br>....... | ..................<br>.................. | ...................<br>.........-ST.. | ......<br>.... |
| iPS:43 6878 | 21-225_62E3 | VL3\|3r/JL 2 | ..D.........<br>........... | .........N.<br>........ | ........<br>...V... | ........<br>..R.... | ..................<br>.................. | ...................<br>.........-STA. | ......<br>.... |
| iPS:43 6880 | 21-225_62E8 | VL3\|3r/JL 2 | ..D.........<br>........... | ......R..N.<br>.......S | ........<br>...V... | ........<br>..R.... | ..................<br>.................. | ...................<br>.........-STA. | ......<br>.... |
| iPS:43 6882 | 21-225_62D10 | VL3\|3r/JL 2 | ..D.........<br>........... | .........N.<br>........ | ........<br>...V... | ........<br>..R.... | ..................<br>.................. | ...................<br>.........-STA. | ......<br>.... |
| iPS:43 6884 | 21-225_62A12 | VL3\|3r/JL 2 | ..D.........<br>........... | .........N.<br>........ | ........<br>...V... | ........<br>..R.... | ..................<br>.................. | ...................<br>.........-STA. | ......<br>.... |
| iPS:43 6886 | 21-225_62B12 | VL3\|3r/JL 2 | ..D.........<br>........... | .........N.<br>......T. | ........<br>...V... | ........<br>..R.... | ..................<br>.................. | ...................<br>.........-STA. | ......<br>.... |
| iPS:43 6892 | 21-225_65E9 | VL3\|3r/JL 2 | ............<br>........... | .........N.<br>......DY | ........<br>....... | ........<br>..R.... | ..................<br>.................. | ....N..............<br>.........-ST.. | ......<br>.... |
| iPS:43 6894 | 21-225_66G9 | VL3\|3r/JL 2 | ..D........T<br>........... | .........N.<br>........ | ........<br>...V... | ........<br>..R.... | ..................<br>.................. | ....I..............<br>.........-NTA. | ......<br>.... |
| iPS:43 6896 | 21-225_67F10 | VL3\|3r/JL 2 | ............<br>........... | .........Y.<br>.......W | ........<br>......F | E.......<br>..R.... | ..................<br>.................. | ....N..............<br>.........-ST.. | .....R<br>.... |
| iPS:43 6898 | 21-225_68D8 | VL3\|3r/JL 2 | ............<br>........... | ...........<br>........ | ........<br>....... | ........<br>....... | ..................<br>.................. | ....N..............<br>.........-ST.. | ......<br>.... |
| iPS:43 6900 | 21-225_69B9 | VL3\|3r/JL 2 | ............<br>........... | .........N.<br>......DY | ........<br>....... | ........<br>..R.... | ..................<br>.................. | ....N..............<br>.........-ST.. | ......<br>.... |
| iPS:43 6902 | 21-225_69B11 | VL3\|3r/JL 2 | ............<br>.....A..... | ...........<br>.......W | ........<br>....... | E.......<br>..R.... | ..................<br>.................. | ....N..............<br>.........-ST.. | ......<br>.... |
| iPS:43 6904 | 21-225_71D4 | VL3\|3r/JL 2 | ............<br>........... | ...........<br>.......Y | ........<br>...V... | ........<br>..R.... | ..................<br>.................. | ...VN..............<br>.........-ST.. | ......<br>.... |
| iPS:43 6906 | 21-225_72B4 | VL3\|3r/JL 2 | ............<br>........... | ...........<br>.......W | ....R...<br>....... | E.......<br>..R.... | ..................<br>.................. | ....N..............<br>.........-ST.. | ......<br>.... |
| iPS:43 6908 | 21-225_72D5 | VL3\|3r/JL 2 | ..D.........<br>........... | .........N.<br>........ | ........<br>...V... | ........<br>..R.... | ..................<br>.................. | ...................<br>.........-STA. | ......<br>.... |
| iPS:43 6912 | 21-225_73C4 | VL3\|3r/JL 2 | ..D.........<br>........... | .........N.<br>........ | ........<br>...V... | ........<br>..M.... | ..................<br>.................. | ...................<br>.........-STA. | ......<br>.... |
| iPS:43 6914 | 21-225_76B4 | VL3\|3r/JL 2 | P...N.T.....<br>........... | ......R..T.<br>.....F.. | ........<br>....... | ........<br>....... | ..................<br>.................. | ....-<br>...................<br>....-SST. | ......<br>.... |
| iPS:43 6916 | 21-225_74A9 | VL3\|3r/JL 2 | ............<br>........... | .........N.<br>......V. | ........<br>....... | ........<br>..NR... | ..................<br>.................. | ...................<br>.........-SP.I | ......<br>.... |
| iPS:43 6918 | 21-225_77A2 | VL3\|3r/JL 2 | ............<br>........... | ......R....<br>........ | ........<br>....... | ........<br>..R.... | ..................<br>.................. | ...................<br>.........-STA. | ......<br>.... |

| | | | | | | | | | |
|---|---|---|---|---|---|---|---|---|---|
| iPS:43 6922 | 21-225_78E9 | VL3\|3r/JL 2 | ..........E.<br>........... | .........N.<br>......VS | ........<br>....... | ........<br>..NR... | ..............S..<br>................ | ....................<br>.........-SP.I | ......<br>.... |
| iPS:43 6924 | 21-225_74B3 | VL3\|3r/JL 2 | ............<br>........... | ...........<br>........ | ........<br>....... | ........<br>....... | ................<br>................ | ....................<br>.........-.T.. | ......<br>.... |
| iPS:43 6928 | 21-225_79E7 | VL3\|3r/JL 2 | ............<br>........... | .........N.<br>......VS | ........<br>....... | ........<br>..NR... | ..............S..<br>................ | ....................<br>.........-SP.I | ......<br>.... |
| iPS:43 6932 | 21-225_92A4 | VL3\|3r/JL 2 | ............<br>.......N... | .........N.<br>......V. | ........<br>....... | ........<br>..NR... | ................<br>................ | ....................<br>.........-SP.I | ......<br>.... |
| iPS:43 6934 | 21-225_96B5 | VL3\|3r/JL 2 | P...N.T.....<br>........... | ......R..T.<br>.....F.. | ........<br>....... | ........<br>....... | ................<br>................ | ....-<br>....................<br>....-SST. | ......<br>.... |
| iPS:43 6936 | 21-225_97E6 | VL3\|3r/JL 2 | ............<br>......V.... | .........N.<br>......VS | ........<br>....... | ........<br>..NR... | ..............S..<br>................ | ....................<br>.........-.P.I | ......<br>.... |
| iPS:43 6938 | 21-225_146A3 | VL3\|3r/JL 2 | ..AM......M.<br>........... | ..N......N.<br>....R... | ........<br>....... | ........<br>....... | ..............I.<br>................ | ....................<br>.........-ST.. | ......<br>.... |
| iPS:43 6940 | 21-225_146B8 | VL3\|3r/JL 2 | ............<br>........... | ...........<br>......V. | ........<br>....... | ........<br>..R.... | ................<br>................ | ...H................<br>.........-ST.. | ......<br>.... |
| iPS:43 6942 | 21-225_146H8 | VL3\|3r/JL 2 | ............<br>........... | ...........<br>........ | ........<br>....... | ........<br>..K.... | ................<br>.......V........ | ....I...............<br>.........-RT.. | ......<br>.... |
| iPS:43 6944 | 21-225_182D12 | VL3\|3r/JL 2 | ............<br>........... | .........E.<br>........ | .....S..<br>....... | ........<br>..R.... | ................<br>................ | ....................<br>.........-RTA. | ......<br>.... |
| iPS:43 6946 | 21-225_183F4 | VL3\|3r/JL 2 | ............<br>........... | ...........<br>........ | ........<br>....... | ........<br>..K.... | ................<br>................ | ....N...............<br>............... | ......<br>.... |
| iPS:43 6952 | 21-225_185D2 | VL3\|3r/JL 2 | ......T.....<br>........... | ...........<br>........ | ........<br>....... | E.......<br>..R.... | ...D............<br>................ | ....................<br>.........-RK.. | ......<br>.... |
| iPS:43 6954 | 21-225_185G7 | VL3\|3r/JL 2 | ............<br>........... | .........H.<br>.....FV. | ........<br>....... | ........<br>..R.... | ................<br>................ | ....-<br>....................<br>....-SST. | ......<br>.... |
| iPS:43 6956 | 21-225_186H6 | VL3\|3r/JL 2 | ............<br>........... | .......M.E.<br>........ | ........<br>....... | ........<br>..R.... | ................<br>................ | ....................<br>.........-STA. | ......<br>.... |
| iPS:43 6962 | 21-225_190H1 | VL3\|3r/JL 2 | ............<br>........... | ...........<br>....RF.Y | ........<br>....... | ........<br>....... | ................<br>................ | K...................<br>.........-ST.. | ......<br>.... |
| iPS:43 6978 | 21-225_190G9 | VL3\|3r/JL 2 | ............<br>........... | ...........<br>....RF.Y | ........<br>....... | ........<br>..N.... | ................<br>S............... | ....................<br>.........-ST.. | .....R<br>.... |
| iPS:43 7018 | 21-225_193H5 | VL3\|3r/JL 2 | ........S...<br>........... | ...........<br>....RF.. | ........<br>....... | ........<br>....... | ................<br>................ | ....................<br>.........-STA. | ......<br>.... |
| iPS:43 7030 | 21-225_195E3 | VL3\|3r/JL 2 | ............<br>........... | .........Y.<br>....RSV. | ........<br>....... | E.......<br>....... | ................<br>................ | ....................<br>.........-VT.. | ......<br>.I.. |
| iPS:43 7034 | 21-225_195E9 | VL3\|3r/JL 2 | ............<br>........... | .........N.<br>.......Y | ........<br>....... | ........<br>..R.... | ...............G<br>.......G........ | ....R...............<br>.........-GI.. | ......<br>.... |
| iPS:43 7070 | 21-225_201G11 | VL3\|3r/JL 2 | ............<br>........... | ...........<br>....RF.. | ........<br>....... | ........<br>....... | ................<br>................ | ....................<br>.........-ST.. | ......<br>.... |

| | | | | | | | | | |
|---|---|---|---|---|---|---|---|---|---|
| iPS:43 7076 | 21-225_203G6 | VL3\|3r/JL 2 | ............<br>........... | ...........<br>....RF.. | ........<br>....... | ........<br>..N.... | .................<br>........S........ | ..................<br>.........-ST.. | ......<br>.... |
| iPS:43 7144 | 21-225_215B3 | VL3\|3r/JL 2 | ............<br>........... | ...........<br>.....F.. | ........<br>....... | ........<br>....... | .................<br>................. | ..................<br>.........-ST.. | ......<br>.... |
| iPS:43 7186 | 21-225_224H2 | VL3\|3r/JL 2 | ........S...<br>........... | ......N..V.<br>......TY | ........<br>.....V. | ........<br>....... | .................<br>................. | ..................<br>.........-ST.. | ......<br>.... |
| iPS:43 7192 | 21-225_225E9 | VL3\|3r/JL 2 | ..D.........<br>........... | ......N..N.<br>....R... | ........<br>.....M. | ........<br>..R.... | .................<br>................. | ..................<br>.........R.... | ......<br>.... |
| iPS:43 7194 | 21-225_226B2 | VL3\|3r/JL 2 | ............<br>........... | ......T..G.<br>.......W | ....R...<br>....... | ........<br>..R.... | .........S.......<br>................. | ....N.............<br>.........-G.A. | ......<br>.... |
| iPS:43 7200 | 21-225_226A10 | VL3\|3r/JL 2 | ............<br>........... | ......T..G.<br>.......W | ........<br>....... | ........<br>..R.... | .........S.......<br>................. | ....N.............<br>.........-G.A. | ......<br>.... |
| iPS:43 7204 | 21-225_227E5 | VL3\|3r/JL 2 | ....S.......<br>........... | .........E.<br>........ | ........<br>....... | ........<br>..R.... | ...............K.<br>................. | ...VN.............<br>.........-NTMI | ......<br>.... |
| iPS:43 7210 | 21-225_227E12 | VL3\|3r/JL 2 | ............<br>........... | ...........<br>......V. | ........<br>....... | ........<br>....... | .................<br>................. | ...N..............<br>.........-SN.. | ......<br>.... |
| iPS:44 8908 | 21-225_50G9 | VL3\|3r/JL 2 | ............<br>........... | ...........<br>........ | ........<br>....... | ........<br>....... | .................<br>.........R...E... | ..RN..............<br>.........-RRG. | .....R<br>.... |
| iPS:45 1102 | 21-225_45F6 | VL3\|3r/JL 2 | ............<br>........... | ...........<br>.......S | ........<br>....... | ........<br>....... | .................<br>................. | ....N.............<br>.........-RTM. | ......<br>.... |
| iPS:45 1110 | 21-225_74C9 | VL3\|3r/JL 2 | ..........E.<br>........... | .......S.N.<br>......VS | ........<br>....... | ........<br>..NR... | ...............S.<br>................. | ..................<br>.........-.P.I | ......<br>.... |
| iPS:45 1112 | 21-225_53D10 | VL3\|3r/JL 2 | ............<br>........... | .........N.<br>........ | ....R...<br>....... | ........<br>..R.... | .................<br>................. | ..................<br>.........-ST.. | ......<br>.... |
| iPS:47 2731 | 21-225_14B1_LC2 | VL3\|3r/JL 2 | .F..........<br>........... | ...........<br>.......Y | ........<br>....... | ........<br>..R.... | .................<br>................. | ....N.............<br>.........-ST.. | ......<br>.... |
| iPS:39 2583 | 21-225_10B10 | VL3\|3r/JL 2 | ............<br>........... | .........N.<br>.......W | ........<br>....... | ........<br>..R.... | .................<br>................. | ....N.............<br>.........-ST.. | ......<br>.... |
| iPS:39 2585 | 21-225_14H11 | VL3\|3r/JL 2 | T.......S...<br>........... | .........E.<br>......V. | ........<br>....... | ........<br>..T.... | .................<br>................. | ....-<br>..................<br>....-SSTI | ......<br>.... |
| iPS:39 2587 | 21-225_18G5 | VL3\|3r/JL 2 | ............<br>........... | ..E........<br>......V. | ........<br>....... | ........<br>....... | .................<br>................. | ...N..............<br>.........-SN.. | ......<br>.... |
| iPS:39 2589 | 21-225_27H2 | VL3\|3r/JL 2 | ............<br>........... | ...........<br>.......S | ........<br>....... | ........<br>..G.... | .................<br>...L............. | ..................<br>...........Y.. | ......<br>.... |
| iPS:39 2598 | 21-225_18E10 | VL3\|3r/JL 2 | ............<br>........... | ......R....<br>.......W | ........<br>....... | ........<br>..R.... | .................<br>................. | ..................<br>.........-ST.. | ......<br>.... |
| iPS:39 3166 | 21-225_27G6 | VL3\|3r/JL 2 | ............<br>........... | ...........<br>........ | ........<br>....... | ........<br>..R.... | .................<br>........T........ | ..................<br>.........SY.. | ......<br>.... |
| iPS:39 3168 | 21-225_32B11 | VL3\|3r/JL 2 | ............<br>........... | ...........<br>.......Y | ........<br>....... | ........<br>.....S. | .................<br>................. | ....N.............<br>.........-ST.. | ......<br>.... |

| | | | | | | | | | |
|---|---|---|---|---|---|---|---|---|---|
| iPS:39 3176 | 21-225_27E7 | VL3\|3r/JL 2 | ............<br>........... | ..........N.<br>........ | ........<br>...E... | ........<br>......L | ..................<br>.................. | ....................<br>..........-ST.. | ......<br>.... |
| iPS:39 3178 | 21-225_34D7 | VL3\|3r/JL 2 | ............<br>........... | ..........E.<br>.......Y | ........<br>.....L. | ........<br>....... | ..................<br>.........T.....F.. | ....N...............<br>..........-.T.. | ......<br>.... |
| iPS:39 3182 | 21-225_4B3 | VL3\|3r/JL 2 | ............<br>...R..V.... | ...........<br>........ | ........<br>....... | ........<br>..R.... | ..................<br>.................. | ....N...............<br>..........-NT.I | ......<br>.... |
| iPS:39 3184 | 21-225_15H11 | VL3\|3r/JL 2 | ............<br>........... | ..........E.<br>........ | ........<br>...V... | ........<br>..R.... | ..................<br>..........I....... | ....................<br>..........-STA. | ......<br>.... |
| iPS:39 3186 | 21-225_27D9 | VL3\|3r/JL 2 | ..........M.<br>........... | ..Y........<br>........ | .F......<br>....... | ........<br>....... | ..................<br>.................. | ...V-<br>....................<br>....-NNT. | ......<br>.... |
| iPS:39 3188 | 21-225_34B9 | VL3\|3r/JL 2 | ......A.....<br>........... | ..........E.<br>......VS | ...E....<br>....... | ........<br>....... | ..................<br>.................. | ....-<br>....................<br>....-SST. | ......<br>.... |
| iPS:39 3192 | 21-225_12B1 | VL3\|3r/JL 2 | ............<br>...R..V.... | ...........<br>........ | ........<br>....... | ........<br>..R.... | ..................<br>.................. | ....N...............<br>..........-NT.I | ......<br>.... |
| iPS:39 3194 | 21-225_16D2 | VL3\|3r/JL 2 | ............<br>........... | ...........<br>........ | ........<br>....... | ........<br>....... | ..................<br>.................. | ....................<br>...........Y.. | ......<br>.... |
| iPS:39 3196 | 21-225_16G8 | VL3\|3r/JL 2 | ....S.......<br>........... | ..........E.<br>........ | ........<br>....... | ........<br>..R.... | ...............K.<br>.................. | ...VN...............<br>..........-NTMI | ......<br>.... |
| iPS:39 3198 | 21-225_28A11 | VL3\|3r/JL 2 | ............<br>........... | ...........<br>........ | ........<br>....... | ........<br>....... | ..................<br>.................. | ....................<br>...........Y.. | ......<br>.... |
| iPS:39 3200 | 21-225_35E1 | VL3\|3r/JL 2 | ............<br>........... | ..........E.<br>.......Y | .F......<br>...I... | ........<br>..R.... | ..................<br>.................. | ....N...............<br>..........-STA. | ......<br>.... |
| iPS:39 3202 | 21-225_6B4 | VL3\|3r/JL 2 | ............<br>...R..V.... | ...........<br>........ | ........<br>....... | ........<br>..R.... | ..................<br>.................. | ....N...............<br>..........-NT.I | ......<br>.... |
| iPS:39 3206 | 21-225_13F6 | VL3\|3r/JL 2 | ............<br>........... | ...........<br>........ | ........<br>....... | ........<br>....... | ..................<br>.................. | ...GN...............<br>............... | ......<br>.... |
| iPS:39 3210 | 21-225_17D3 | VL3\|3r/JL 2 | ......S.....<br>........... | ...........<br>......VY | ........<br>...V... | ........<br>..R.... | ..................<br>.................. | ....................<br>..........-ITA. | ......<br>...R |
| iPS:39 3212 | 21-225_30H6 | VL3\|3r/JL 2 | ............<br>........... | ..........N.<br>........ | ........<br>....... | ........<br>....... | ..................<br>.................. | ....-<br>....................<br>....-SST. | ......<br>.... |
| iPS:39 3214 | 21-225_33A1 | VL3\|3r/JL 2 | ............<br>........... | ...........<br>.....FVY | ........<br>....... | ........<br>....... | ..................<br>.................. | ....................<br>..........-.T.. | ......<br>.... |
| iPS:39 3218 | 21-225_14G3 | VL3\|3r/JL 2 | ............<br>........... | ...........<br>......V. | ........<br>....... | ........<br>..R.... | ..................<br>.................. | ...GN...............<br>............... | ......<br>.... |
| iPS:39 3222 | 21-225_17F5 | VL3\|3r/JL 2 | ............<br>........... | ..........E.<br>........ | ........<br>....... | ........<br>..R.... | ..................<br>.................. | ....-<br>....................<br>....-SST. | ......<br>.... |
| iPS:39 3224 | 21-225_31C2 | VL3\|3r/JL 2 | ............<br>........... | ..........N.<br>........ | ........<br>....... | ........<br>....... | ..................<br>.................. | ....-<br>.................... | ......<br>.... |

| | | | | | | | | | |
|---|---|---|---|---|---|---|---|---|---|
| iPS:39 3226 | 21-225_33E6 | VL3\|3r/JL 2 | ............<br>........... | ...........<br>.......Y | .F......<br>...I... | ........<br>..R.... | .................<br>................. | ....N.............<br>.........-STA. | ......<br>.... |
| iPS:39 3234 | 21-225_26C10 | VL3\|3r/JL 2 | ...V......M.<br>........... | ...........<br>......V. | .F......<br>....... | ........<br>....... | .................<br>................. | ...V-<br>..................<br>....-NNT. | ......<br>.... |
| iPS:39 3345 | 21-225_5G7 | VL3\|3r/JL 2 | ............<br>........... | .........N.<br>.......W | ........<br>....... | ........<br>..R.... | .................<br>................. | ....N.............<br>.........-ST.. | ......<br>.... |
| iPS:39 3565 | 21-225_34B11 | VL3\|3r/JL 2 | ............<br>........... | ...........<br>........ | ........<br>...V... | ........<br>..M.... | .................<br>................. | ....N.............<br>.........-STA. | ......<br>.... |
| iPS:39 3950 | 21-225_3H10 | VL3\|3r/JL 2 | ....S.......<br>........... | .........E.<br>........ | ........<br>....... | ........<br>..R.... | ...............K.<br>................. | ...VN.............<br>.........-NTMI | ......<br>.... |
| iPS:39 8470 | 21-225_14B7 | VL3\|3r/JL 2 | ............<br>....R...... | .........N.<br>.......Y | ........<br>....... | ........<br>..R.... | .................<br>.......T......... | ...NN.............<br>.........-ST.. | ......<br>.... |
| iPS:39 8472 | 21-225_16E4 | VL3\|3r/JL 2 | P...........<br>........... | ...........<br>......VY | .....S..<br>....... | ........<br>....... | .................<br>A................ | ..................<br>.........-ST.. | ......<br>.... |
| iPS:39 8488 | 21-225_19F6 | VL3\|3r/JL 2 | ............<br>......V.... | ...........<br>........ | ........<br>....... | ........<br>....... | .T...............<br>................. | ....N.............<br>.........-NT.. | ......<br>.... |
| iPS:39 8490 | 21-225_21D12 | VL3\|3r/JL 2 | ............<br>........... | .........N.<br>.......Y | ........<br>....... | ........<br>..R.... | .................<br>..............F.. | ....N.............<br>.........-ST.. | .....R<br>.... |
| iPS:39 8498 | 21-225_22E6 | VL3\|3r/JL 2 | ............<br>........... | .........E.<br>........ | ........<br>....... | ........<br>..R.... | .....Y....T......<br>................. | ..................<br>.........-STA. | ......<br>.... |
| iPS:39 8504 | 21-225_23D7 | VL3\|3r/JL 2 | ............<br>........... | ..E........<br>......V. | ........<br>...V... | ........<br>....... | .................<br>................. | ...N..............<br>.........-SN.. | ......<br>.... |
| iPS:39 8546 | 21-225_9H10 | VL3\|3r/JL 2 | ............<br>........... | ...........<br>........ | ........<br>....... | ........<br>....... | .................<br>................. | ..................<br>...........Y.. | ......<br>.... |
| iPS:40 2225 | 21-225_2B1 | VL3\|3r/JL 2 | ............<br>......V.... | ...........<br>........ | ........<br>....... | ........<br>..R.... | .................<br>................. | ....N.............<br>.........-NT.. | ......<br>.... |
| iPS:40 2231 | 21-225_6D9 | VL3\|3r/JL 2 | ............<br>........... | .........E.<br>........ | ........<br>....... | ........<br>..K.... | .................<br>................. | ....-<br>..................<br>....-SST. | ......<br>.... |
| iPS:40 4090 | 21-225_8D8 | VL3\|3r/JL 2 | ............<br>........... | .........E.<br>........ | ........<br>...V... | ........<br>..R.... | .................<br>.........I....... | ..................<br>.........-STA. | ......<br>.... |
| iPS:42 3018 | 21-225_31D12_LC2 | VL3\|3r/JL 2 | ............<br>........... | ...........<br>.......Y | .F......<br>...I... | ........<br>..R.... | .................<br>................. | ....N.............<br>.........-STA. | ......<br>.... |
| | | Germline | **L_FR1** | **L_CDR1** | **L_FR2** | **L_CDR2** | **L_FR3** | **L_CDR3** | **L_FR4** |
| | VL2\|2a2/JL3b | | QSALTQP-<br>ASVSGSPGQSIT<br>ISC | TGTS-<br>SDVGGY----<br>NYVS | WYQQHPGK<br>APKLMIY | E-------<br>-VSNRPS | GVSNRFSGSKSG--<br>NTASLTISGLQAEDEAD<br>YYC | SSYTSS------------<br>---------STLWV | FGGGTK<br>LTVL |
| iPS:46 8862 | 21-225_178H8 | VL2\|2a2/JL3b | ......S.....<br>........... | ...........<br>.....F.. | ........<br>V..F... | ........<br>....... | ..P..............<br>................. | .....-<br>..................<br>....YT.. | ......<br>.... |

| | | | | | | | | | |
|---|---|---|---|---|---|---|---|---|---|
| iPS:436838 | 21-225_52H4 | VL2\|2a2/JL3b | ............<br>........... | ...........<br>........ | ........<br>....... | ........<br>....... | .................<br>................. | N....-<br>..................<br>...NIT.. | ......<br>.... |
| iPS:437094 | 21-225_210D12 | VL2\|2a2/JL3b | ............<br>........... | ...........<br>........ | ......V.<br>....L.. | ........<br>....... | .................<br>................. | N....-<br>..................<br>....IT.. | ......<br>.... |
| iPS:437096 | 21-225_210E12 | VL2\|2a2/JL3b | ............<br>........... | ...........<br>........ | ........<br>....... | ........<br>....... | .................<br>................. | G..VK-<br>..................<br>...GIT.. | .....S<br>.... |
| iPS:437098 | 21-225_211C1 | VL2\|2a2/JL3b | ............<br>........... | .........S.<br>........ | ....Y...<br>....... | ........<br>....... | .................<br>................. | N....-<br>..................<br>....IT.. | ......<br>.... |
| iPS:437104 | 21-225_211G5 | VL2\|2a2/JL3b | ............<br>........... | ...........<br>........ | ........<br>....... | ........<br>....... | .................<br>................. | N...R-<br>..................<br>....IT.. | ......<br>.... |
| iPS:437112 | 21-225_212C2 | VL2\|2a2/JL3b | ............<br>........... | ...........<br>........ | ........<br>....... | ........<br>..R.... | .................<br>................. | ....R-<br>..................<br>....IT.. | ......<br>.... |
| iPS:437114 | 21-225_212A4 | VL2\|2a2/JL3b | ............<br>........... | ...........<br>........ | ........<br>....... | ........<br>....... | .................<br>................. | G..VK-<br>..................<br>...GIT.. | .....S<br>.... |
| iPS:437116 | 21-225_212F6 | VL2\|2a2/JL3b | ............<br>........... | ...........<br>........ | ....Y...<br>....... | ........<br>....... | .................<br>................. | N....-<br>..................<br>....IT.. | ......<br>.... |
| iPS:437118 | 21-225_212G7 | VL2\|2a2/JL3b | ............<br>........... | ...........<br>........ | ........<br>T...... | ........<br>....... | .................<br>................. | N....-<br>..................<br>....IT.. | ......<br>.... |
| iPS:437128 | 21-225_213G3 | VL2\|2a2/JL3b | L...........<br>........... | ...........<br>........ | ........<br>......S | ........<br>..R.... | .................<br>................. | N...R-<br>..................<br>....IT.. | ......<br>.... |
| iPS:437130 | 21-225_213D5 | VL2\|2a2/JL3b | ............<br>........... | ...........<br>........ | ........<br>....V.. | ........<br>..R.... | ...T...........K.<br>................. | C...R-<br>..................<br>...RIT.. | ......<br>.... |
| iPS:437146 | 21-225_215D3 | VL2\|2a2/JL3b | ............<br>........... | .......I...<br>........ | ........<br>..T.... | ........<br>....... | .................<br>................. | N..KR-<br>..................<br>...GST.. | ......<br>V... |
| iPS:437150 | 21-225_216A3 | VL2\|2a2/JL3b | ............<br>........... | ...........<br>........ | ........<br>....... | ........<br>....... | .................<br>................. | N....-<br>..................<br>....IT.. | ......<br>.... |
| iPS:437162 | 21-225_217B2 | VL2\|2a2/JL3b | ............<br>........... | ...........<br>........ | ........<br>....L.. | ........<br>....... | ..Y..............<br>................. | G..VK-<br>..................<br>...GIT.. | .....S<br>.... |

| | | | | | | | | | |
|---|---|---|---|---|---|---|---|---|---|
| iPS:43 7172 | 21-225_219A7 | VL2\|2a2/JL3b | .............<br>........... | ............<br>........ | ........<br>....... | ........<br>..R.... | ..................<br>.................. | C...R-<br>....................<br>....IT.. | ......<br>.... |
| iPS:43 7182 | 21-225_221H2 | VL2\|2a2/JL3b | L...........<br>........... | ............<br>........ | ........<br>......S | ........<br>..R.... | ..................<br>.................. | N...R-<br>....................<br>....IT.. | ......<br>.... |
| iPS:43 7184 | 21-225_221G4 | VL2\|2a2/JL3b | .............<br>........... | ............<br>........ | ........<br>....... | ........<br>..R.... | ..................<br>.................. | N...R-<br>....................<br>....IT.. | ......<br>.... |
| | | Germline | L_FR1 | L_CDR1 | L_FR2 | L_CDR2 | L_FR3 | L_CDR3 | L_FR4 |
| | VL1\|1c/JL2 | | QSVLTQP-<br>PSASGTPGQRVT<br>ISC | SGSS-SNIGS-<br>----NTVN | WYQQLPGT<br>APKLLIY | S-------<br>-NNQRPS | GVPDRFSGSKSG--<br>TSASLAISGLQSEDEAD<br>YYC | AAWDDS-----------<br>---------LNGVV | FGGGTK<br>LTVL |
| iPS:46 8864 | 21-225_60D6 | VL1\|1c/JL2 | .............<br>........... | ............<br>........ | ........<br>....... | ........<br>....... | ..................<br>.................. | .....-<br>....................<br>...SLNGP | V.....<br>.... |
| iPS:43 6660 | 21-225_146D8 | VL1\|1c/JL2 | ..........T.<br>........... | ......Y....<br>.......D | ........<br>....... | ........<br>....... | ..................<br>.................. | ....................<br>.............. | ......<br>.... |
| iPS:43 6680 | 21-225_147H12 | VL1\|1c/JL2 | .............<br>........... | ............<br>....YA.. | ........<br>....... | ........<br>...H... | ..................<br>.................. | E...................<br>...........P. | ......<br>.... |
| iPS:43 6682 | 21-225_146A8 | VL1\|1c/JL2 | .............<br>........... | ............<br>.....SI. | ......R.<br>....... | ........<br>..D.... | ..................<br>.................. | ....................<br>.............. | ......<br>.... |
| iPS:43 6684 | 21-225_146B6 | VL1\|1c/JL2 | .............<br>........... | ............<br>.....A.. | ........<br>....... | ........<br>....... | ..................<br>.................. | ....................<br>.............. | ......<br>.... |
| iPS:43 6696 | 21-225_149A1 | VL1\|1c/JL2 | .............<br>........... | ............<br>.....A.. | ........<br>....... | ........<br>....... | ..................<br>.................. | ....................<br>.............. | ......<br>.... |
| iPS:43 6712 | 21-225_150F9 | VL1\|1c/JL2 | .............<br>........... | ............<br>.....A.. | ........<br>....... | ........<br>..S.... | ..................<br>...............F. | ....................<br>.............. | ......<br>.... |
| iPS:43 6750 | 21-225_154G12 | VL1\|1c/JL2 | .............<br>........... | .........N.<br>.....A.S | ........<br>....... | ........<br>..DH... | ..................<br>.................. | ....................<br>.........K.P. | ......<br>.... |
| iPS:43 6762 | 21-225_156H2 | VL1\|1c/JL2 | ...V........<br>........... | ............<br>........ | ........<br>....... | ........<br>.S..... | .....L............<br>.................. | ....................<br>.............. | ......<br>V... |
| iPS:43 7044 | 21-225_197F9 | VL1\|1c/JL2 | .............<br>........... | ............<br>........ | ........<br>....... | ........<br>....... | ..................<br>.................. | ....................<br>........M..P. | ......<br>.... |
| iPS:43 7060 | 21-225_199C3 | VL1\|1c/JL2 | .............<br>........... | ............<br>........ | ........<br>....... | ........<br>....... | ..................<br>.................. | ....................<br>...........P. | ......<br>.... |
| iPS:39 3180 | 21-225_4G12 | VL1\|1c/JL2 | .............<br>.......NM.. | ..TN.......<br>....Y... | ........<br>....... | I.......<br>....... | ..................<br>.................. | ....................<br>........LNGH.. | ...R..<br>.... |
| iPS:39 3230 | 21-225_9G9 | VL1\|1c/JL2 | .............<br>.......NM.. | ..TN.......<br>....Y... | ........<br>....... | I.......<br>....... | ..................<br>.................. | ....................<br>........LNGH.. | ...R..<br>.... |
| | | Germline | L_FR1 | L_CDR1 | L_FR2 | L_CDR2 | L_FR3 | L_CDR3 | L_FR4 |

| | | | | | | | | | |
|---|---|---|---|---|---|---|---|---|---|
| | VL3\|3p/JL2 | | SYELTQP-<br>PSVSVSPGQTAR<br>ITC | SGD---ALPK-<br>----KYAY | WYQQKSGQ<br>APVLVIY | E-------<br>-DSKRPS | GIPERFSGSSSG--<br>TMATLTISGAQVEDEAD<br>YYC | YSTDSS------------<br>---------GNHVV | FGGGTK<br>LTVL |
| iPS:46 8866 | 21-225_190C1 | VL3\|3p/J L2 | ............<br>........... | T.....M...<br>........ | .D......<br>......S | ........<br>....... | .................<br>P.........T..D. | N....-<br>.................<br>...SGNR. | ......<br>.... |
| iPS:43 7214 | 21-225_48B12 | VL3\|3p/J L2 | ............<br>........... | ...........<br>........ | ........<br>....... | ........<br>....... | .................<br>................. | N................<br>.............. | ......<br>.... |
| | | Germline | L_FR1 | L_CDR1 | L_FR2 | L_CDR2 | L_FR3 | L_CDR3 | L_FR4 |
| | VL1\|1c/JL3b | | QSVLTQP-<br>PSASGTPGQRVT<br>ISC | SGSS-SNIGS-<br>----NTVN | WYQQLPGT<br>APKLLIY | S-------<br>-NNQRPS | GVPDRFSGSKSG--<br>TSASLAISGLQSEDEAD<br>YYC | AAWDDS------------<br>---------LNGWV | FGGGTK<br>LTVL |
| iPS:43 6234 | 21-225_51E3 | VL1\|1c/J L3b | ............<br>........... | ...N.......<br>.....I.T | ........<br>....... | ........<br>..D.... | .................<br>................. | T................<br>.............. | .....T<br>.... |
| iPS:43 6830 | 21-225_51F4 | VL1\|1c/J L3b | ............<br>........... | ...........<br>.....I.T | ........<br>....... | ........<br>..D.... | .................<br>............T.... | T................<br>.............. | .....T<br>.... |
| iPS:43 6834 | 21-225_52F1 | VL1\|1c/J L3b | ............<br>........... | ...........<br>.....I.T | ........<br>....... | ........<br>..D.... | .................<br>................. | .................<br>.............. | .....T<br>.... |
| iPS:43 6842 | 21-225_54E9 | VL1\|1c/J L3b | ............<br>........... | ...N.....N.<br>.....I.T | ........<br>....... | V.......<br>..D.... | .................<br>................. | .................<br>.............. | .....T<br>.... |
| iPS:43 6844 | 21-225_56G1 | VL1\|1c/J L3b | ............<br>........... | ...........<br>....HI.T | ........<br>....... | ........<br>..D.... | .................<br>................. | .V...............<br>..........I... | .....T<br>.... |
| iPS:43 6846 | 21-225_56E3 | VL1\|1c/J L3b | ............<br>........... | ...........<br>.....I.T | ........<br>....... | ........<br>....... | .................<br>..............C. | .................<br>.............. | .....T<br>.... |
| iPS:43 7010 | 21-225_192G3 | VL1\|1c/J L3b | ............<br>.......M.. | ...........<br>........ | ....F...<br>....... | G.......<br>..K.... | R................<br>...........T.... | .................<br>.............. | ......<br>.... |
| iPS:43 7032 | 21-225_195H6 | VL1\|1c/J L3b | ............<br>........... | ...........<br>....H... | ........<br>....... | N.......<br>..Y.... | .................<br>..T.............. | .T...............<br>..........SV.. | ......<br>V... |
| iPS:45 1104 | 21-225_49C5 | VL1\|1c/J L3b | ............<br>........... | ...........<br>.....I.T | ........<br>....... | ........<br>..D.... | .................<br>................. | T................<br>.............. | .....T<br>.... |
| iPS:45 1106 | 21-225_49D10 | VL1\|1c/J L3b | ............<br>........... | ...N.......<br>.....I.T | ........<br>....... | ........<br>..D.... | .................<br>................. | .................<br>.............. | .....T<br>.... |
| iPS:45 1108 | 21-225_53E8 | VL1\|1c/J L3b | ............<br>........... | ...C.......<br>.....I.T | ........<br>....... | ........<br>..D.... | .................<br>................. | T................<br>...........D.. | .....T<br>.... |
| | | Germline | L_FR1 | L_CDR1 | L_FR2 | L_CDR2 | L_FR3 | L_CDR3 | L_FR4 |
| | VL3\|3r/JL1 | | SYELTQP-<br>PSVSVSPGQTAS<br>ITC | SGD---KLGD-<br>----KYAC | WYQQKPGQ<br>SPVLVIY | Q-------<br>-DSKRPS | GIPERFSGSNSG--<br>NTATLTISGTQAMDEAD<br>YYC | QAWDS-------------<br>---------STAYV | FGTGTK<br>VTVL |
| iPS:43 6662 | 21-225_147D7 | VL3\|3r/JL 1 | ............<br>........... | ...........<br>.....F.. | ........<br>....... | ........<br>..R.... | .................<br>................. | ....R............<br>.........-NTA. | ......<br>.... |
| iPS:43 6720 | 21-225_151H6 | VL3\|3r/JL 1 | ............<br>........... | ...........<br>........ | ........<br>....... | ........<br>..T.... | .................<br>................. | .................<br>.........-ST.. | ......<br>.... |

| | | | | | | | | | |
|---|---|---|---|---|---|---|---|---|---|
| iPS:43 6726 | 21-225_152G5 | VL3\|3r/JL 1 | ...M........<br>.......I... | ............<br>........ | ........<br>....... | ........<br>....... | .................<br>................. | ..................<br>.........-ST.. | ......<br>.... |
| iPS:43 6732 | 21-225_152B12 | VL3\|3r/JL 1 | ............<br>........... | .........N.<br>........ | ........<br>....... | ........<br>..T.... | .................<br>................. | ..................<br>.........-ST.. | ......<br>.... |
| iPS:43 6734 | 21-225_153A8 | VL3\|3r/JL 1 | ............<br>........... | ............<br>........ | ........<br>....... | ........<br>..T.... | .................<br>................. | ..................<br>.........-ST.. | ......<br>.... |
| iPS:43 6754 | 21-225_155G3 | VL3\|3r/JL 1 | ............<br>........... | ............<br>......V. | ........<br>..M.... | ........<br>....... | .................<br>................. | ....N.............<br>.........-SI.. | ......<br>.... |
| iPS:43 7190 | 21-225_225A9 | VL3\|3r/JL 1 | ............<br>........... | .........N.<br>........ | ........<br>....... | ........<br>....... | .................<br>................. | ..................<br>.........N..C. | ......<br>.... |
| | | Germline | L_FR1 | L_CDR1 | L_FR2 | L_CDR2 | L_FR3 | L_CDR3 | L_FR4 |
| | VL5\|5c/JL3b | | QAVLTQP-<br>ASLSASPGASAS<br>LTC | TLRS-<br>GINVGT----<br>YRIY | WYQQKPGS<br>PPQYLLR | YKS-----<br>DSDKQQGS | GVPSRFSGSKDASANAG<br>ILLISGLQSEDEADYYC | MIWHS------------<br>---------SASWV | FGGGTK<br>LTVL |
| iPS:43 6702 | 21-225_149E8 | VL5\|5c/J L3b | ...S....S...<br>........... | .......T.T.<br>........ | ........<br>...F... | .T......<br>...H... | .................<br>..F.............. | ..................<br>.........-SA.. | ......<br>.... |
| | | Germline | L_FR1 | L_CDR1 | L_FR2 | L_CDR2 | L_FR3 | L_CDR3 | L_FR4 |
| | VL1\|1e/JL2 | | QSVLTQP-<br>PSVSGAPGQRVT<br>ISC | TGSS-<br>SNIGAG----<br>YDVH | WYQQLPGT<br>APKLLIY | G-------<br>-NSNRPS | GVPDRFSGSKSG--<br>TSASLAITGLQAEDEAD<br>YYC | QSYDSS-----------<br>---------LSGVV | FGGGTK<br>LTVL |
| iPS:43 6752 | 21-225_155H1 | VL1\|1e/J L2 | ............<br>........... | ............<br>........ | ........<br>....... | ........<br>....... | .................<br>...............H. | ..................<br>........LSGP.I | ......<br>.... |
| iPS:43 6820 | 21-225_179D10 | VL1\|1e/J L2 | ............<br>........... | .......F.TD<br>........ | ....F...<br>....... | ........<br>.H..... | .................<br>................. | ....R-<br>..................<br>...SLN.. | ......<br>.... |
| iPS:43 7188 | 21-225_224B11 | VL1\|1e/J L2 | ............<br>........... | ............<br>........ | ........<br>......F | ........<br>....... | .................<br>...............H. | ....N-<br>..................<br>...SLSG. | ......<br>.... |
| iPS:43 7198 | 21-225_226F8 | VL1\|1e/J L2 | ............<br>........... | ............<br>........ | ........<br>....... | ........<br>....... | ...........D.....<br>................. | ....N-<br>..................<br>...SLSG. | ......<br>.... |
| iPS:43 7202 | 21-225_227D3 | VL1\|1e/J L2 | ............<br>........... | ............<br>........ | ........<br>....... | ........<br>....... | ...........D.....<br>................. | ....N-<br>..................<br>...SLSG. | ......<br>.... |
| iPS:43 7208 | 21-225_227C10 | VL1\|1e/J L2 | ............<br>........... | ............<br>........ | ........<br>....... | ........<br>....... | ...........D.....<br>................. | ....N-<br>..................<br>...NLSG. | ......<br>.... |
| iPS:44 3003 | 21-225_43F11_LC2 | VL1\|1e/J L2 | ............<br>........... | ............<br>........ | ........<br>....... | ........<br>....... | .................<br>................. | ....N.............<br>............S. | ......<br>.... |
| | | Germline | L_FR1 | L_CDR1 | L_FR2 | L_CDR2 | L_FR3 | L_CDR3 | L_FR4 |
| | VL6\|6a/JL3b | | NFMLTQP- | TRSS-GSIAS- | WYQQRPGS | E------- | GVPDRFSGSIDSSSNSA | QSYDS------------ | FGGGTK |

| | | | | | | | | | |
|---|---|---|---|---|---|---|---|---|---|
| iPS:43 6792 | 21-225_169D12 | VL6\|6a/JL3b | ............. ............ | .........TG. ........ | .H.....N ....L.. | ........ ..KK... | ................. ................. | ...Y.............. ..........G... | ...... .... |
| | | Germline | L_FR1 | L_CDR1 | L_FR2 | L_CDR2 | L_FR3 | L_CDR3 | L_FR4 |
| | VL3\|3l/JL2 | | SSELTQD-PAVSVALGQTVRITC | QGD---SLRS-----YYAS | WYQQKPGQAPVLVIY | G--------KNNRPS | GIPDRFSGSSSG--NTASLTITGAQAEDEADYYC | NSRDSS-------------------GNHVV | FGGGTKLTVL |
| iPS:43 6800 | 21-225_171D12 | VL3\|3l/JL2 | ............. ............ | ........... ........ | ........ ..I.... | A....... ....... | .........N....... ................. | .................. ..........S... | ...... .... |
| iPS:43 6804 | 21-225_172C3 | VL3\|3l/JL2 | ............. ............ | .........N. ......V. | ........ ..I.... | T....... ...S... | .........T....... .....T.......... | .................. ............... | ...... .... |
| iPS:43 6806 | 21-225_172B12 | VL3\|3l/JL2 | ............. ............ | .........N. ........ | ........ ..I.... | T....... ...S... | .........T....... ................. | .................. ............... | ...... .... |
| iPS:43 6964 | 21-225_190B3 | VL3\|3l/JL2 | ............. ............ | ......K..T. ........ | ........ .....V. | ........ ....... | ................. ................. | .................. ........GNHL.L | ...... .... |
| iPS:43 6970 | 21-225_190B8 | VL3\|3l/JL2 | ............. ............ | ......T..P. ......V. | ........ ....... | ........ ....... | ................. ................. | .................. ........GNHL.. | ...... .... |
| iPS:43 6980 | 21-225_190C10 | VL3\|3l/JL2 | ............. ............ | .........P. ........ | ........ ....... | ........ ....... | ................. .....E.......... | .................. ........GNHL.. | ...... .... |
| iPS:43 6992 | 21-225_191B8 | VL3\|3l/JL2 | ............. ............ | ......T..P. ........ | ........ ....... | ........ ....... | ..S.............. ................. | .................. ........GNHL.. | ...... .... |
| iPS:43 6994 | 21-225_191A9 | VL3\|3l/JL2 | ............. ............ | .........P. ........ | ........ ....... | ........ ....... | ................. .....E.......... | .....C............ ........GNHL.. | ...... .... |
| iPS:43 7016 | 21-225_193A6 | VL3\|3l/JL2 | ............. ............ | ........... .......N | ........ ....F.. | A....... ....... | .........N....... ................. | .................. ............L. | ...... .... |
| | | Germline | L_FR1 | L_CDR1 | L_FR2 | L_CDR2 | L_FR3 | L_CDR3 | L_FR4 |
| | VL2\|2b2/JL2 | | QSALTQP-ASVSGSPGQSITISC | TGTS-SDVGSY----NLVS | WYQQHPGKAPKLMIY | E--------VSKRPS | GVSNRFSGSKSG--NTASLTISGLQAEDEADYYC | CSYAGS-------------------STFVV | FGGGTKLTVL |
| iPS:43 6810 | 21-225_175F4 | VL2\|2b2/JL2 | ............. ............ | .........RF ........ | .......Y ....... | ........ ....... | ................. ................. | .................. ...........Y.. | ...... .... |
| iPS:43 6814 | 21-225_178H10 | VL2\|2b2/JL2 | ............. ............ | .........RF ........ | ...H...N ....... | ........ ....... | ................. ................. | .................. ............... | ...... .... |
| | | Germline | L_FR1 | L_CDR1 | L_FR2 | L_CDR2 | L_FR3 | L_CDR3 | L_FR4 |
| | VL3\|3l/JL3b | | SSELTQD-PAVSVALGQTVRITC | QGD---SLRS-----YYAS | WYQQKPGQAPVLVIY | G--------KNNRPS | GIPDRFSGSSSG--NTASLTITGAQAEDEADYYC | NSRDSS-------------------GNHWV | FGGGTKLTVL |
| iPS:43 6816 | 21-225_179H5 | VL3\|3l/JL3b | ............. ............ | .........N. ........ | ........ ...F... | ........ ....... | .........R....... ................. | .................. ............... | ...... .... |
| | | Germline | L_FR1 | L_CDR1 | L_FR2 | L_CDR2 | L_FR3 | L_CDR3 | L_FR4 |
| | VL1\|1e/JL1 | | QSVLTQP-PSVSGAPGQRVTISC | TGSS-SNIGAG----YDVH | WYQQLPGTAPKLLIY | G--------NSNRPS | GVPDRFSGSKSG--TSASLAITGLQAEDEADYYC | QSYDSS-------------------LSGYV | FGTGTKVTVL |

| | | | | | | | | | |
|---|---|---|---|---|---|---|---|---|---|
| iPS:43 6832 | 21-225_51D8 | VL1\|1e/J L1 | .............<br>............ | ...........<br>....FE.. | ........<br>....... | ........<br>....... | .................<br>................. | .................<br>.............. | ......<br>.... |
| | | Germline | L_FR1 | L_CDR1 | L_FR2 | L_CDR2 | L_FR3 | L_CDR3 | L_FR4 |
| | VL3\|3r/JL7 | | SYELTQP-<br>PSVSVSPGQTAS<br>ITC | SGD---KLGD-<br>----KYAC | WYQQKPGQ<br>SPVLVIY | Q-------<br>-DSKRPS | GIPERFSGSNSG--<br>NTATLTISGTQAMDEAD<br>YYC | QAWDS------------<br>---------STAAV | FGGGTQ<br>LTVL |
| iPS:43 6840 | 21-225_53E9 | VL3\|3r/JL 7 | .............<br>............ | ..T........<br>......V. | ........<br>......N | ........<br>..TM... | .................<br>................. | .T...............<br>.........-ST.. | .....T<br>.... |
| iPS:43 6950 | 21-225_184G4 | VL3\|3r/JL 7 | .............<br>............ | ...........<br>.....F.. | ........<br>....... | E.......<br>..R.... | .................<br>................. | .................<br>.........-RTV. | ......<br>.... |
| | | Germline | L_FR1 | L_CDR1 | L_FR2 | L_CDR2 | L_FR3 | L_CDR3 | L_FR4 |
| | VL1\|1b/JL2 | | QSVLTQP-<br>PSVSAAPGQKVT<br>ISC | SGSS-SNIGN-<br>----NYVS | WYQQLPGT<br>APKLLIY | D-------<br>-NNKRPS | GIPDRFSGSKSG--<br>TSATLGITGLQTGDEAD<br>YYC | GTWDSS-----------<br>---------LSAVV | FGGGTK<br>LTVL |
| iPS:43 6856 | 21-225_58C5 | VL1\|1b/J L2 | .............<br>............ | ...........<br>........ | ........<br>....... | ........<br>....... | .................<br>................. | ....I............<br>...........VG. | ......<br>.... |
| iPS:43 6960 | 21-225_198D2 | VL1\|1b/J L2 | .............<br>............ | .........S.<br>........ | ........<br>...V... | ........<br>....... | .................<br>................. | .....R...........<br>.........NVG. | ......<br>.... |
| iPS:43 6966 | 21-225_190C3 | VL1\|1b/J L2 | .............<br>............ | ...........<br>........ | ........<br>.....L. | ........<br>.S..... | ...G.............<br>................. | .................<br>...........T.. | ......<br>.... |
| iPS:43 6968 | 21-225_190B10 | VL1\|1b/J L2 | .............<br>............ | ...........<br>........ | ...H....<br>....... | ........<br>....... | .................<br>................. | .................<br>............G. | ......<br>.... |
| iPS:43 6974 | 21-225_190H7 | VL1\|1b/J L2 | .............<br>............ | .........S.<br>........ | ........<br>...V... | ........<br>....... | .................<br>................. | ....GR...........<br>.........NVG. | ......<br>.... |
| iPS:43 6976 | 21-225_190D8 | VL1\|1b/J L2 | .............<br>............ | ...........<br>....H... | ........<br>....... | ........<br>.SS.... | E................<br>................. | .................<br>...........T.. | ......<br>.... |
| iPS:43 6982 | 21-225_190D10 | VL1\|1b/J L2 | .............<br>............ | .........S.<br>........ | ........<br>V..V... | ........<br>....... | .................<br>................. | .....R...........<br>.........NVG. | ......<br>.... |
| iPS:43 6986 | 21-225_191A1 | VL1\|1b/J L2 | .............<br>.....R..... | .......L...<br>.....F.. | ....F...<br>....... | ........<br>..Y.... | .......V.........<br>................. | .................<br>.........NTG. | ......<br>.... |
| iPS:43 7006 | 21-225_192G2 | VL1\|1b/J L2 | .............<br>............ | ...........<br>........ | ........<br>....... | ........<br>....... | R................<br>................. | .................<br>............G. | ......<br>.... |
| iPS:43 7024 | 21-225_194F11 | VL1\|1b/J L2 | .............<br>............ | ...........<br>........ | ........<br>....... | ........<br>....... | .................<br>................. | .................<br>............G. | ......<br>.... |
| iPS:43 7028 | 21-225_194G12 | VL1\|1b/J L2 | .............<br>............ | ...........<br>........ | ........<br>....... | ........<br>....... | R................<br>..........E...... | .................<br>...........VG. | ......<br>.... |
| iPS:43 7042 | 21-225_197E8 | VL1\|1b/J L2 | .............<br>............ | ...........<br>....K... | ....F...<br>....... | ........<br>....... | K................<br>.......L......... | .I..R............<br>...........VM. | ......<br>.... |
| iPS:43 7064 | 21-225_200G8 | VL1\|1b/J L2 | .............<br>.....R..... | .......L...<br>.....F.. | ....F...<br>....... | ........<br>..Y.... | .......V.........<br>................. | .................<br>.........NTG. | ......<br>.... |
| iPS:43 7086 | 21-225_209A8 | VL1\|1b/J L2 | .............<br>............ | .........S.<br>.....FL. | ........<br>....... | ........<br>....... | .................<br>................. | .................<br>............G. | ......<br>.... |

| | | | | | | | | | |
|---|---|---|---|---|---|---|---|---|---|
| iPS:43 7138 | 21-225_214D8 | VL1\|1b/J L2 | ............<br>........... | ...........<br>........ | ....F...<br>......H | ........<br>....... | ..................<br>.................. | .A................<br>............... | I...S.<br>.... |
| iPS:43 7168 | 21-225_218G4 | VL1\|1b/J L2 | ............<br>........... | ...........<br>........ | ........<br>.....L. | ........<br>.S..... | ...A..............<br>.................. | ..................<br>..........NT.. | ......<br>.... |
| | | Germline | **L_FR1** | **L_CDR1** | **L_FR2** | **L_CDR2** | **L_FR3** | **L_CDR3** | **L_FR4** |
| | VL6\|6a/JL2 | | NFMLTQP-<br>HSVSESPGKTVT<br>ISC | TRSS-GSIAS-<br>----NYVQ | WYQQRPGS<br>SPTTVIY | E-------<br>-DNQRPS | GVPDRFSGSIDSSSNSA<br>SLTISGLKTEDEADYYC | QSYDS------------<br>----------SNVV | FGGGTK<br>LTVL |
| iPS:43 6888 | 21-225_63G7 | VL6\|6a/J L2 | ............<br>........... | ...N....V..<br>........ | ........<br>....M.. | ........<br>..SR... | ..................<br>...............S. | ....G.............<br>..........I... | ......<br>.... |
| iPS:43 6890 | 21-225_63A10 | VL6\|6a/J L2 | ............<br>........... | ...N....V..<br>........ | ........<br>....... | ........<br>..KR... | ..................<br>.................. | ..................<br>..........I... | ......<br>.... |
| | | Germline | **L_FR1** | **L_CDR1** | **L_FR2** | **L_CDR2** | **L_FR3** | **L_CDR3** | **L_FR4** |
| | VL8\|8a/JL3b | | QTVVTQE-<br>PSFSVSPGGTVT<br>LTC | GLSS-<br>GSVSTS----<br>YYPS | WYQQTPGQ<br>APRTLIY | S-------<br>-TNTRSS | GVPDRFSGSILG--<br>NKAALTITGAQADDESD<br>YYC | VLYMG------------<br>---------SGIWV | FGGGTK<br>LTVL |
| iPS:43 6910 | 21-225_73G1 | VL8\|8a/J L3b | ............<br>........... | ...........<br>........ | ........<br>....... | N.......<br>....... | ..................<br>.................. | ..................<br>..........A... | ......<br>.... |
| iPS:43 6948 | 21-225_183F5 | VL8\|8a/J L3b | ............<br>........... | ..........T<br>....F... | ........<br>....... | N.......<br>....... | ..................<br>.................. | ..................<br>.............. | ......<br>.... |
| | | Germline | **L_FR1** | **L_CDR1** | **L_FR2** | **L_CDR2** | **L_FR3** | **L_CDR3** | **L_FR4** |
| | VL7\|7a/JL2 | | QTVVTQE-<br>PSLTVSPGGTVT<br>LTC | ASST-<br>GAVTSG----<br>YYPN | WFQQKPGQ<br>APRALIY | S-------<br>-TSNKHS | WTPARFSGSLLG--<br>GKAALTLSGVQPEDEAE<br>YYC | LLYYG------------<br>---------GAQVV | FGGGTK<br>LTVL |
| iPS:43 6920 | 21-225_74E5 | VL7\|7a/J L2 | ............<br>........... | .....ET....<br>....S... | ........<br>....... | ........<br>....... | ..................<br>....D.......... | ..................<br>............L. | ......<br>.... |
| iPS:43 6926 | 21-225_78D10 | VL7\|7a/J L2 | ............<br>........... | ...........<br>.....F.. | ........<br>....... | ........<br>..D.... | ..................<br>.................. | ..................<br>............LM | ......<br>.... |
| iPS:43 6958 | 21-225_190D1 | VL7\|7a/J L2 | ............<br>........... | ...........<br>....S... | ........<br>....... | ........<br>....... | ..................<br>..............D... | ..................<br>.............A | ......<br>.... |
| iPS:43 6984 | 21-225_190F10 | VL7\|7a/J L2 | ............<br>........... | VF.........<br>....SF.. | ........<br>....... | ........<br>....... | ..................<br>..............D... | ...C..............<br>............L. | ......<br>.... |
| iPS:43 6988 | 21-225_191A2 | VL7\|7a/J L2 | ............<br>........... | VL.........<br>....SF.. | ........<br>....... | ........<br>....... | ..................<br>..............D... | M..C..............<br>............L. | ......<br>.... |
| iPS:43 7002 | 21-225_191H9 | VL7\|7a/J L2 | ............<br>........... | ..........A<br>........ | .L......<br>...T... | ........<br>..N.... | ..................<br>....D.......... | .IF...............<br>..........VH.I | ......<br>.... |
| iPS:43 7008 | 21-225_192E3 | VL7\|7a/J L2 | ............<br>........... | .F....S....<br>....S... | ........<br>....... | ........<br>..N.... | ..................<br>.......R....... | ..................<br>............L. | ......<br>.... |
| iPS:43 7012 | 21-225_192G7 | VL7\|7a/J L2 | ............<br>........... | ...........<br>....N..Q | ........<br>....... | ........<br>..T.R.. | ..................<br>.................. | .F................<br>.............I | ......<br>.... |
| iPS:43 7014 | 21-225_192H8 | VL7\|7a/J L2 | ............<br>........... | .F...T....<br>....F... | ........<br>....... | N.......<br>....R.. | ...............M.<br>..............D... | ..................<br>............LM | ......<br>.... |

| | | | | | | | | | |
|---|---|---|---|---|---|---|---|---|---|
| iPS:43 7022 | 21-225_194G5 | VL7\|7a/J L2 | ............<br>........... | ...........<br>....N... | ........<br>T...... | ........<br>....... | ................G<br>................. | .I................<br>............LM | ......<br>.... |
| iPS:43 7026 | 21-225_194D12 | VL7\|7a/J L2 | ............<br>........... | ...........<br>....SF.S | ........<br>....... | ........<br>....R.. | S................<br>.............D... | .I................<br>............LA | ......<br>.... |
| iPS:43 7040 | 21-225_196E7 | VL7\|7a/J L2 | ............<br>........... | ..........A<br>........ | .L......<br>...T... | ........<br>..N.... | .................<br>.....D........... | .IF...............<br>..........VH.I | ......<br>.... |
| iPS:43 7048 | 21-225_197B11 | VL7\|7a/J L2 | ............<br>........... | .F....S....<br>....S... | ........<br>....... | ........<br>..N.... | .................<br>................. | ..................<br>............L. | ......<br>.... |
| iPS:43 7050 | 21-225_197C11 | VL7\|7a/J L2 | ............<br>........... | ..........A<br>........ | .L......<br>...T... | ........<br>....... | .................<br>.....D........... | .IF...............<br>..........VH.I | ......<br>.... |
| iPS:43 7056 | 21-225_198B8 | VL7\|7a/J L2 | ........S...<br>........... | VL.........<br>....SF.. | ........<br>....... | ........<br>....... | .................<br>.............D..F | M..S..............<br>............M. | ......<br>.... |
| iPS:43 7062 | 21-225_200H1 | VL7\|7a/J L2 | ............<br>........... | ..N........<br>....S... | ........<br>....... | H.......<br>..N.... | .................<br>................. | .I................<br>............L. | ......<br>.... |
| iPS:43 7066 | 21-225_200G9 | VL7\|7a/J L2 | ............<br>........... | ..N........<br>....S... | .L......<br>....... | H.......<br>..D.... | .................<br>......A.......... | .I................<br>............L. | ......<br>.... |
| iPS:43 7068 | 21-225_200A11 | VL7\|7a/J L2 | ............<br>........... | ...........<br>........ | ........<br>V...... | ........<br>..N.... | .................<br>.............D... | ..................<br>...........HLA | ......<br>.... |
| iPS:43 7090 | 21-225_210F11 | VL7\|7a/J L2 | ............<br>........... | .F.........<br>....N... | ........<br>....... | ........<br>....... | .................<br>................. | ..................<br>............L. | ......<br>.... |
| iPS:43 7106 | 21-225_211H7 | VL7\|7a/J L2 | ............<br>........... | .F.........<br>....N..S | ........<br>V...... | ........<br>....R.. | .................<br>................. | ..................<br>............L. | ......<br>.... |
| iPS:43 7108 | 21-225_211C9 | VL7\|7a/J L2 | ............<br>........... | G.....S....<br>.....F.. | ........<br>...P... | ........<br>..N.... | .................<br>.....D.......D... | ..................<br>............LA | ......<br>.... |
| iPS:43 7110 | 21-225_211E9 | VL7\|7a/J L2 | ............<br>........... | ...........<br>....N... | ........<br>....... | ........<br>..I.... | G.....T.F........<br>................. | ..................<br>............LA | ......<br>.... |
| iPS:43 7120 | 21-225_212A9 | VL7\|7a/J L2 | ............<br>........... | ...........<br>........ | ........<br>....... | ........<br>..N.... | .................<br>.........D...D... | ..................<br>.............G | ......<br>.... |
| iPS:43 7124 | 21-225_212H12 | VL7\|7a/J L2 | ............<br>........... | ...........<br>........ | ........<br>....... | ........<br>....... | .................<br>................. | ..................<br>...........H.. | ......<br>.... |
| iPS:43 7132 | 21-225_213F5 | VL7\|7a/J L2 | ............<br>........... | G.....S....<br>.....F.. | ........<br>T..P... | ........<br>..N.... | ................T<br>.....D.......D... | ...F..............<br>............LA | ......<br>.... |
| iPS:43 7136 | 21-225_214H3 | VL7\|7a/J L2 | ............<br>........... | ...........<br>........ | ........<br>....... | ........<br>....... | C................<br>................. | ..................<br>...........H.. | ......<br>.... |
| iPS:43 7140 | 21-225_214E12 | VL7\|7a/J L2 | ............<br>........... | ...........<br>........ | ........<br>....... | ........<br>....... | .................<br>.....D........... | ...CD.............<br>............L. | ......<br>.... |
| iPS:43 7142 | 21-225_215A3 | VL7\|7a/J L2 | ............<br>........... | .....E.....<br>....N..S | ........<br>....... | ........<br>....... | G.....T..........<br>................. | ..................<br>............LA | ......<br>.A.. |
| iPS:43 7148 | 21-225_215H3 | VL7\|7a/J L2 | ............<br>........... | ...........<br>........ | ........<br>....... | ........<br>..N.... | CG...............<br>.........D...D... | ..................<br>.............G | ......<br>.... |
| iPS:43 7154 | 21-225_216A7 | VL7\|7a/J L2 | ............<br>........... | ...........<br>........ | ........<br>....... | ........<br>..N.... | C................<br>.........D...D... | ..................<br>.............G | ......<br>.... |

| | | | | | | | | | |
|---|---|---|---|---|---|---|---|---|---|
| iPS:43<br>7158 | 21-<br>225_216H11 | VL7\|7a/J<br>L2 | .............<br>........... | ...........<br>........ | ........<br>....... | ........<br>....... | ..................<br>.....D........... | ...CD.............<br>............L. | ......<br>.... |
| | | Germline | L_FR1 | L_CDR1 | L_FR2 | L_CDR2 | L_FR3 | L_CDR3 | L_FR4 |
| | VL1\|1b/JL3b | | QSVLTQP-<br>PSVSAAPGQKVT<br>ISC | SGSS-SNIGN-<br>----NYVS | WYQQLPGT<br>APKLLIY | D-------<br>-NNKRPS | GIPDRFSGSKSG--<br>TSATLGITGLQTGDEAD<br>YYC | GTWDSS-----------<br>---------LSAWV | FGGGTK<br>LTVL |
| iPS:43<br>6972 | 21-<br>225_190C7 | VL1\|1b/J<br>L3b | .............<br>........... | ..G........<br>........ | .F..F...<br>...F... | ........<br>....... | ..................<br>................. | ....RT............<br>...........D.. | ......<br>.... |
| iPS:43<br>7020 | 21-<br>225_193F11 | VL1\|1b/J<br>L3b | .Y..........<br>........... | F.G........<br>........ | .F..F...<br>...F... | ........<br>....... | ..L..L............<br>..D.....N........ | ....RT............<br>.........M.D.. | ......<br>.... |
| iPS:43<br>7036 | 21-<br>225_195H9 | VL1\|1b/J<br>L3b | .Y..........<br>........... | ..G........<br>........ | .F..F...<br>...F... | ........<br>....... | ..L...............<br>................. | ....RT............<br>.........M.D.. | ......<br>.... |
| | | Germline | L_FR1 | L_CDR1 | L_FR2 | L_CDR2 | L_FR3 | L_CDR3 | L_FR4 |
| | VL1\|1b/JL1 | | QSVLTQP-<br>PSVSAAPGQKVT<br>ISC | SGSS-SNIGN-<br>----NYVS | WYQQLPGT<br>APKLLIY | D-------<br>-NNKRPS | GIPDRFSGSKSG--<br>TSATLGITGLQTGDEAD<br>YYC | GTWDSS-----------<br>---------LSAYV | FGTGTK<br>VTVL |
| iPS:43<br>6996 | 21-<br>225_191B9 | VL1\|1b/J<br>L1 | .............<br>........... | ...........<br>........ | ........<br>....... | ........<br>..K.... | ..................<br>................. | ..................<br>...........VC. | ......<br>.... |
| iPS:43<br>7054 | 21-<br>225_194G3 | VL1\|1b/J<br>L1 | .............<br>........... | ...........<br>......I. | ........<br>....... | ........<br>..K.... | ..................<br>................. | ..................<br>...........VC. | ......<br>.... |
| iPS:43<br>7058 | 21-<br>225_199F3 | VL1\|1b/J<br>L1 | .............<br>........... | ...........<br>........ | ........<br>....... | ........<br>....... | ..................<br>................. | ..................<br>.............C. | ......<br>.... |
| | | Germline | L_FR1 | L_CDR1 | L_FR2 | L_CDR2 | L_FR3 | L_CDR3 | L_FR4 |
| | VL5\|5c/JL2 | | QAVLTQP-<br>ASLSASPGASAS<br>LTC | TLRS-<br>GINVGT----<br>YRIY | WYQQKPGS<br>PPQYLLR | YKS----<br>DSDKQQGS | GVPSRFSGSKDASANAG<br>ILLISGLQSEDEADYYC | MIWHS------------<br>---------SASVV | FGGGTK<br>LTVL |
| iPS:43<br>7000 | 21-<br>225_191G9 | VL5\|5c/J<br>L2 | ...S.R..S...<br>........... | ...........<br>........ | ........<br>....... | ........<br>....... | ..................<br>................. | ..................<br>.........-SA.. | ......<br>.... |
| | | Germline | L_FR1 | L_CDR1 | L_FR2 | L_CDR2 | L_FR3 | L_CDR3 | L_FR4 |
| | VL3\|3j/JL2 | | SYELTQP-<br>LSVSVALGQTAR<br>ITC | GGN---NIGS-<br>----KNVH | WYQQKPGQ<br>APVLVIY | R-------<br>-DSNRPS | GIPERFSGSNSG--<br>NTATLTISRAQAGDEAD<br>YYC | QVWDS------------<br>---------STAVV | FGGGTK<br>LTVL |
| iPS:43<br>7074 | 21-<br>225_203B2 | VL3\|3j/JL<br>2 | .............<br>........... | .........R.<br>........ | ........<br>S...I.H | ........<br>...D... | ..................<br>................. | ..................<br>.........-GTA. | ......<br>.P.. |
| iPS:43<br>7082 | 21-<br>225_205E12 | VL3\|3j/JL<br>2 | .............<br>A.......... | .........R.<br>........ | ........<br>S...I.H | ........<br>...D... | ..................<br>................. | ..................<br>.........-GTA. | ......<br>.P.. |
| iPS:43<br>7084 | 21-<br>225_206B5 | VL3\|3j/JL<br>2 | .............<br>.........A. | .........R.<br>........ | .......L<br>...P..X | ........<br>...Y.S. | ...D......C.....T<br>.V.......E..E... | .D................<br>.........-ST.. | ......<br>.... |
| iPS:43<br>7088 | 21-<br>225_209H10 | VL3\|3j/JL<br>2 | .............<br>.........A. | .........R.<br>........ | .......L<br>...P..L | ........<br>...Y.S. | ...D......W......<br>.V.......E..E... | .D................<br>.........-ST.. | ......<br>.... |
| iPS:43<br>7100 | 21-<br>225_211H2 | VL3\|3j/JL<br>2 | .............<br>........... | .........R.<br>....R... | ........<br>..I.... | ........<br>..RD... | ..................<br>...............F. | ..................<br>.........-STA. | ......<br>.... |

| | | | | | | | | | |
|---|---|---|---|---|---|---|---|---|---|
| iPS:43 7160 | 21-225_216B12 | VL3\|3j/JL 2 | .............<br>............ | ..D.....RR.<br>....R... | ........<br>....... | ........<br>....... | ..................<br>.................. | ...................<br>..........-STG. | ......<br>.... |
| iPS:39 2593 | 21-225_3E10 | VL3\|3j/JL 2 | ........H...<br>..TA.M..... | ...........<br>.....A.. | ........<br>D...... | S.......<br>....... | ..........P.....<br>......IE......... | .....S............<br>..........DH.. | ......<br>.... |
| iPS:39 3204 | 21-225_8C12 | VL3\|3j/JL 2 | ........H...<br>..TA.M..... | ...........<br>.....A.. | ........<br>D...... | S.......<br>....... | ..........P.....<br>......IE......... | .....S............<br>..........DH.. | ......<br>.... |
| | | Germline | L_FR1 | L_CDR1 | L_FR2 | L_CDR2 | L_FR3 | L_CDR3 | L_FR4 |
| | VL2\|2a2/JL2 | | QSALTQP-<br>ASVSGSPGQSIT<br>ISC | TGTS-<br>SDVGGY----<br>NYVS | WYQQHPGK<br>APKLMIY | E-------<br>-VSNRPS | GVSNRFSGSKSG--<br>NTASLTISGLQAEDEAD<br>YYC | SSYTSS-----------<br>---------STLVV | FGGGTK<br>LTVL |
| iPS:43 7092 | 21-225_210B12 | VL2\|2a2/JL2 | ............<br>........... | ...........<br>........ | ........<br>...F... | ........<br>..R.... | ..................<br>.................. | .....-<br>...................<br>....RTL. | ......<br>.... |
| iPS:43 7134 | 21-225_213A7 | VL2\|2a2/JL2 | ............<br>........... | ...........<br>........ | ........<br>...F... | ........<br>..R.... | ..................<br>.................. | .....-<br>...................<br>....RTL. | ......<br>.... |
| iPS:47 2733 | 21-225_2B10_LC2 | VL2\|2a2/JL2 | ............<br>........... | ...........<br>.....F.. | ......D.<br>....... | ........<br>....... | ..................<br>.................. | .....-<br>...................<br>...TGT.. | I.....<br>.... |
| iPS:39 2573 | 21-225_15G2 | VL2\|2a2/JL2 | ............<br>........... | ...........<br>........ | ........<br>....... | ........<br>....... | ..................<br>.................. | T....-<br>...................<br>...TST.. | ......<br>.... |
| iPS:39 3232 | 21-225_17F12 | VL2\|2a2/JL2 | ............<br>........... | ..A.....D.<br>.....S.. | ........<br>....... | ........<br>....... | ..................<br>.................. | .....-<br>...................<br>....IT.. | ......<br>.... |
| iPS:39 8494 | 21-225_21H4 | VL2\|2a2/JL2 | ............<br>........... | ...........<br>.....S.. | ......D.<br>....... | ........<br>....... | ..................<br>.................. | ....R-<br>...................<br>....ST.. | ......<br>.... |
| | | Germline | L_FR1 | L_CDR1 | L_FR2 | L_CDR2 | L_FR3 | L_CDR3 | L_FR4 |
| | VL3\|3m/JL1 | | SYELMQP-<br>PSVSVSPGQTAR<br>ITC | SGD---ALPK-<br>----QYAY | WYQQKPGQ<br>APVLVIY | K-------<br>-DSERPS | GIPERFSGSSSG--<br>TTVTLTISGVQAEDEAD<br>YYC | QSADSS-----------<br>---------GTYYV | FGTGTK<br>VTVL |
| iPS:43 7102 | 21-225_211E5 | VL3\|3m/JL1 | ....T.......<br>........... | ...........<br>........ | ........<br>....... | ........<br>...A... | ..........R.......<br>...V............ | .LVY.-<br>...................<br>...SDT.. | .....M<br>L... |
| iPS:43 7164 | 21-225_217C6 | VL3\|3m/JL1 | ....T.......<br>........... | ...........<br>........ | ........<br>....... | ........<br>....... | ..........R.......<br>....R........... | .LIV.-<br>...................<br>...SDT.. | ......<br>.... |
| iPS:43 7166 | 21-225_217G11 | VL3\|3m/JL1 | ....T.......<br>........... | ...........<br>........ | ........<br>....... | ........<br>....... | ..........R.......<br>...V............ | .LVY.-<br>...................<br>...SDT.. | ......<br>.... |
| iPS:43 | 21- | VL3\|3m/J | ....T....... | .R....V.... | ........ | ........ | ..........R....... | .LVV.- | ...... |

| | | | | | | | | | |
|---|---|---|---|---|---|---|---|---|---|
| iPS:43 7196 | 21-225_226B7 | VL3\|3m/J L1 | .F..T.......<br>............ | ..........R.<br>....H.V. | ....N...<br>....... | ........<br>....... | .................<br>................. | .....-<br>..................<br>...SGT.. | ......<br>.... |
| | | Germline | L_FR1 | L_CDR1 | L_FR2 | L_CDR2 | L_FR3 | L_CDR3 | L_FR4 |
| | VL4\|4c/JL2 | | LPVLTQP-<br>PSASALLGASIK<br>LTC | TLSS-EHSTY-<br>-----TIE | WYQQRPGR<br>SPQYIMK | VKS-----<br>DGSHSKGD | GIPDRFMGSSSG--<br>ADRYLTFSNLQSDDEAE<br>YHC | GESHTID-----------<br>--------GQVGVV | FGGGTK<br>LTVL |
| iPS:39 2596 | 21-225_12D8 | VL4\|4c/J L2 | ............<br>........... | ...........<br>........ | ........<br>...... | ........<br>....... | .................<br>............D.... | ..................<br>.............. | ......<br>.... |
| iPS:39 3174 | 21-225_15D8 | VL4\|4c/J L2 | ............<br>........... | ...........<br>........ | ........<br>...... | ........<br>....... | .................<br>.I.........E.... | ..................<br>.............. | ......<br>.... |
| iPS:39 8544 | 21-225_7C8 | VL4\|4c/J L2 | ............<br>........... | ...........<br>........ | ........<br>...... | ........<br>....... | ..............G..<br>............D.... | ....P.............<br>.............. | ......<br>.... |
| | | Germline | L_FR1 | L_CDR1 | L_FR2 | L_CDR2 | L_FR3 | L_CDR3 | L_FR4 |
| | VL3\|3h/JL2 | | SYVLTQP-<br>PSVSVAPGKTAR<br>ITC | GGN---NIGS-<br>----KSVH | WYQQKPGQ<br>APVLVIY | Y-------<br>-DSDRPS | GIPERFSGSNSG--<br>NTATLTISRVEAGDEAD<br>YYC | QVWDSS-----------<br>---------SDHVV | FGGGTK<br>LTVL |
| iPS:39 3208 | 21-225_16F3 | VL3\|3h/J L2 | ............<br>....Q...... | ...........<br>........ | ........<br>.....V. | D.......<br>..T.... | .................<br>................. | ..................<br>.............. | ......<br>.... |
| HEAVY_VARIABLE | | | | | | | | | |
| | | Germline | H_FR1 | H_CDR1 | H_FR2 | H_CDR2 | H_FR3 | H_CDR3 | H_FR4 |
| | VH1\|1-08/D6\|6-19\|RF1/JH4 | | QVQLVQS-<br>GAEVKKPGASVK<br>VSCKASG-YTFT | S-----YDIN | WVRQATGQ<br>GLEWMG | WMNPN---<br>SGNTGYAQ<br>KFQG | RVTMTRNTSISTAYMEL<br>SSLRSEDTAVYYCAR | GYSSGW-----------<br>-------YYFDY | WGQGTL<br>VTVSS |
| iPS:42 6126 | 21-225_6G6 | VH1\|1-08/D6\|6-19\|RF1/J H4 | ............<br>R...........<br>........ | N......... | ........<br>.P.... | ..H.....<br>.......K<br>.... | ...........A...V.<br>..............L | SSGWY-<br>..................<br>.-.... | ......<br>..... |
| iPS:41 2232 | 21-225_4A2 | VH1\|1-08/D6\|6-19\|RF1/J H4 | .........T..<br>............<br>........ | N......... | ........<br>...... | ..H.....<br>........<br>.... | ...L.............<br>..............S | SSGWY-<br>..................<br>.-.... | ......<br>..... |
| iPS:42 6112 | 21-225_12F12 | VH1\|1-08/D6\|6-19\|RF1/J H4 | ....L.......<br>............<br>........ | N......... | ........<br>...... | ..Y.....<br>........<br>.... | .....M...........<br>..............M | SSGWY-<br>..................<br>.-...F | ......<br>..... |
| iPS:45 1141 | 21-225_164B11 | VH1\|1-08/D6\|6-19\|RF1/J H4 | ............<br>............<br>........ | N......... | ........<br>...... | ..T.....<br>........<br>.... | .........M.......<br>.......S.....SY | SSGWY-<br>..................<br>.-M... | ......<br>..... |
| iPS:46 8850 | 21-225_63F4 | VH1\|1-08/D6\|6-19\|RF1/J H4 | ............<br>............<br>........ | N.......V. | ........<br>...... | ..H.....<br>........<br>..R. | .........L..V....<br>..............Y | SSGWY-<br>..................<br>.-V... | ......<br>..... |

| | | | | | | | | | |
|---|---|---|---|---|---|---|---|---|---|
| iPS:46 8852 | 21-225_71F3 | VH1\|1-08/D6\|6-19\|RF1/JH4 | ............ ............ ........ | N.......V. | ........ ...... | ..H..... ........ .... | ................. ..............Y | SSGWY- .................... .-V..S | ...... ..... |
| iPS:46 8854 | 21-225_72C4 | VH1\|1-08/D6\|6-19\|RF1/JH4 | ............ ............ ........ | N......... | ....V... ...... | ..H..... ........ .... | ................. N............SH | SSGWY- .................... .-L... | ...... ..... |
| iPS:46 8870 | 21-225_74A8 | VH1\|1-08/D6\|6-19\|RF1/JH4 | ............ ............ ........ | N......... | .....S.. ...... | ..H..... ........ .... | ................. .......S......Y | SSGWY- .................... .-K... | .S.... ..... |
| iPS:42 3314 | 21-225_12F11 | VH1\|1-08/D6\|6-19\|RF1/JH4 | ............ ............ ........ | N......... | ........ .P.... | ..H..... .......K .... | ...........A...V. ..............L | SSGWY- .................... .-.... | ...... ..... |
| iPS:43 3909 | 21-225_43D8 | VH1\|1-08/D6\|6-19\|RF1/JH4 | ............ ............ ........ | N......... | ........ ...... | ..H..... ........ .... | ................. ...T..........H | SSGWT- .................... .-L... | ...... ....A |
| iPS:43 4177 | 21-225_56A1 | VH1\|1-08/D6\|6-19\|RF1/JH4 | ............ ............ ........ | N......... | ........ ....L. | ..H..... ........ .... | ................. ..............Y | SSGWY- .................... .-V... | ...... ..... |
| iPS:43 4211 | 21-225_60F3 | VH1\|1-08/D6\|6-19\|RF1/JH4 | ..L......... ............ ........ | N......... | ........ ...... | ..H..... ........ .... | ................. ..............Y | SSGWY- .................... .-F... | ...... ..... |
| iPS:43 4235 | 21-225_61E3 | VH1\|1-08/D6\|6-19\|RF1/JH4 | ............ ............ ........ | N......... | ........ ...... | ..T..... ........ .... | ................. ...K..........Y | SSGWY- .................... .-R... | ...... ..... |
| iPS:43 4237 | 21-225_61B5 | VH1\|1-08/D6\|6-19\|RF1/JH4 | ............ ............ ........ | N......... | ........ ...... | ..H..... ..S..... .... | ................. ..............Y | SSGWY- .................... .-.... | ...... ..... |
| iPS:43 4295 | 21-225_58B9 | VH1\|1-08/D6\|6-19\|RF1/JH4 | ............ ............ ........ | N......... | ........ ...... | ........ ..S..... .... | ................. ..............Y | SSGWY- .................... .-.... | ...... ..... |
| iPS:43 4305 | 21-225_59E1 | VH1\|1-08/D6\|6-19\|RF1/JH4 | ............ ............ ........ | N......... | ........ ...... | ..T..... ........ .... | ......T.......... ..............F | SSGWY- .................... .-F... | ...... ..... |
| iPS:43 | 21- | VH1\|1-08/D6\|6- | ............ ............ | N......... | ........ ...... | ........ ........ | ................. ...........N..V | SSGWY- .................... | ...... ..... |

| | | | | | | | | | |
|---|---|---|---|---|---|---|---|---|---|
| iPS:43 4431 | 21-225_70E7 | VH1\|1-08/D6\|6-19\|RF1/JH4 | ............<br>............<br>........ | N......... | ........<br>...... | ..H.....<br>........<br>.... | .................<br>..............Y | SSGWY-<br>...................<br>.-V... | ......<br>..... |
| iPS:43 4443 | 21-225_71G3 | VH1\|1-08/D6\|6-19\|RF1/JH4 | ............<br>............<br>........ | N......... | ........<br>...... | ..H.....<br>..S.....<br>.... | ......D..V.......<br>..............Y | SSGWY-<br>...................<br>.-.... | ......<br>..... |
| iPS:43 4475 | 21-225_74F9 | VH1\|1-08/D6\|6-19\|RF1/JH4 | ............<br>............<br>........ | N......... | ........<br>...... | ........<br>.....C..<br>.... | .................<br>..............S | SSGWN-<br>...................<br>.-F... | ......<br>..... |
| iPS:43 4477 | 21-225_74A6 | VH1\|1-08/D6\|6-19\|RF1/JH4 | ............<br>............<br>........ | N......... | ........<br>...... | ..H.....<br>........<br>..R. | .....W...........<br>..............S | SSGWY-<br>...................<br>.-.... | ......<br>..... |
| iPS:43 4487 | 21-225_76G2 | VH1\|1-08/D6\|6-19\|RF1/JH4 | ............<br>............<br>........ | N......... | ........<br>...... | ........<br>........<br>.... | .................<br>..............G | SSGWY-<br>...................<br>.-M... | ......<br>..... |
| iPS:43 4511 | 21-225_74B11 | VH1\|1-08/D6\|6-19\|RF1/JH4 | ............<br>.......R....<br>........ | N......... | ........<br>...... | ..H.....<br>........<br>.... | .................<br>..............Y | SSGWY-<br>...................<br>.-.... | ......<br>..... |
| iPS:43 4549 | 21-225_76E11 | VH1\|1-08/D6\|6-19\|RF1/JH4 | ............<br>............<br>........ | N......... | ........<br>...... | ........<br>........<br>.... | .L...............<br>..........F...Y | SSGWY-<br>...................<br>.-.... | ......<br>..... |
| iPS:43 4551 | 21-225_75C4 | VH1\|1-08/D6\|6-19\|RF1/JH4 | ............<br>............<br>........ | N......... | ........<br>...... | ..H.....<br>........<br>.... | .................<br>..............S | SSGWY-<br>...................<br>.-.... | ......<br>..... |
| iPS:43 4635 | 21-225_78E6 | VH1\|1-08/D6\|6-19\|RF1/JH4 | ............<br>............<br>........ | N......... | .....S..<br>...... | ..H.....<br>........<br>.... | .................<br>.......S......Y | SSGWY-<br>...................<br>.-K... | .S....<br>..... |
| iPS:43 4649 | 21-225_78E11 | VH1\|1-08/D6\|6-19\|RF1/JH4 | ............<br>............<br>........ | N......... | ........<br>...... | ........<br>.....C..<br>.... | .................<br>..............S | SSGWN-<br>...................<br>.-F... | ......<br>..... |
| iPS:43 4665 | 21-225_74G4 | VH1\|1-08/D6\|6-19\|RF1/JH4 | ............<br>............<br>........ | N.......V. | ........<br>...... | ..H.....<br>........<br>.... | ..........R......<br>..............S | SSGWY-<br>...................<br>.-H... | ......<br>..... |
| iPS:43 | 21-225_79G7 | VH1\|1-08/D6\|6- | ............<br>............ | N......... | .....S..<br>...... | ..H.....<br>........ | .................<br>.......S......Y | SSGWY-<br>................... | .S....<br>..... |

| | | | | | | | | | |
|---|---|---|---|---|---|---|---|---|---|
| iPS:43 4685 | 21-225_79E9 | VH1\|1-08/D6\|6-19\|RF1/JH4 | ............<br>............<br>........ | N......... | ........<br>...... | ..H.....<br>........<br>.... | .................<br>..............S | SSGWY-<br>...................<br>.-.... | ......<br>..... |
| iPS:43 4697 | 21-225_79F12 | VH1\|1-08/D6\|6-19\|RF1/JH4 | ............<br>............<br>........ | N......... | ........<br>...... | ........<br>........<br>.... | .................<br>..............S | SSGWY-<br>...................<br>.-F... | ......<br>..L.. |
| iPS:43 4729 | 21-225_80B12 | VH1\|1-08/D6\|6-19\|RF1/JH4 | ............<br>............<br>T....... | N......... | ........<br>...... | ........<br>........<br>...V | .................<br>..............Y | SSGWY-<br>...................<br>.-I... | ......<br>..... |
| iPS:43 4851 | 21-225_75A6 | VH1\|1-08/D6\|6-19\|RF1/JH4 | ............<br>............<br>........ | N......... | ........<br>...... | ........<br>........<br>.... | .................<br>..............Y | SSGWY-<br>...................<br>.-I... | ......<br>..... |
| iPS:43 4909 | 21-225_85C11 | VH1\|1-08/D6\|6-19\|RF1/JH4 | ............<br>............<br>........ | N......... | .....S..<br>...... | ..H.....<br>........<br>.... | .................<br>.......S......Y | SSGWY-<br>...................<br>.-K... | .S....<br>..... |
| iPS:43 4959 | 21-225_87E10 | VH1\|1-08/D6\|6-19\|RF1/JH4 | ............<br>............<br>........ | N......... | ........<br>...... | ........<br>........<br>.... | .................<br>..............Y | SSGWY-<br>...................<br>.-F... | ......<br>..... |
| iPS:43 4965 | 21-225_88A1 | VH1\|1-08/D6\|6-19\|RF1/JH4 | ............<br>............<br>........ | N......... | ........<br>...... | ........<br>........<br>.... | .................<br>..............Y | SSGWY-<br>...................<br>.-.... | ......<br>..... |
| iPS:43 4973 | 21-225_88B4 | VH1\|1-08/D6\|6-19\|RF1/JH4 | ............<br>............<br>........ | N......... | ........<br>...... | ........<br>..D.....<br>.... | S.........T.....<br>.............SY | SSGWY-<br>...................<br>.-.... | ......<br>..... |
| iPS:43 4997 | 21-225_88C10 | VH1\|1-08/D6\|6-19\|RF1/JH4 | ............<br>.......R....<br>.......S | N......... | ........<br>...... | ..T.....<br>........<br>.... | .................<br>..............S | SSGWY-<br>...................<br>.-...S | ......<br>....L |
| iPS:43 5053 | 21-225_75F9 | VH1\|1-08/D6\|6-19\|RF1/JH4 | ............<br>............<br>........ | N......... | ........<br>...... | ..H.....<br>........<br>.... | .................<br>..............Y | SSGWY-<br>...................<br>.-I... | ......<br>..... |
| iPS:43 5113 | 21-225_92E6 | VH1\|1-08/D6\|6-19\|RF1/JH4 | ............<br>.......R....<br>........ | N......... | ........<br>...... | ..H.....<br>........<br>.... | ..........M.......<br>..............H | SSGWY-<br>...................<br>.-F... | ......<br>..... |
| iPS:43 | 21- | VH1\|1-08/D6\|6- | ............<br>............ | N......... | ........<br>...... | ........<br>........ | .................<br>..............Y | SSGWY-<br>................... | ......<br>..... |

| | | | | | | | | | |
|---|---|---|---|---|---|---|---|---|---|
| iPS:43 5257 | 21-225_96H5 | VH1\|1-08/D6\|6-19\|RF1/J H4 | ............<br>............<br>........ | N......... | .....S..<br>...... | ..H.....<br>........<br>.... | .................<br>.......S......Y | SSGWY-<br>..................<br>.-K... | .S....<br>..... |
| iPS:43 5267 | 21-225_96D10 | VH1\|1-08/D6\|6-19\|RF1/J H4 | ............<br>.......R....<br>........ | N......... | ........<br>...... | ..H.....<br>........<br>.... | .........M.......<br>..............H | SSGWY-<br>..................<br>.-F... | ......<br>..... |
| iPS:43 5299 | 21-225_146D4 | VH1\|1-08/D6\|6-19\|RF1/J H4 | ............<br>............<br>........ | N......... | ........<br>...... | .VH.....<br>........<br>.... | .................<br>..............G | SSGWY-<br>..................<br>.-.... | ......<br>..... |
| iPS:43 5305 | 21-225_146C9 | VH1\|1-08/D6\|6-19\|RF1/J H4 | ............<br>............<br>........ | N......... | ........<br>...... | ..H.....<br>........<br>.... | .....W.........A.<br>..............Y | SSGWY-<br>..................<br>.-S... | ......<br>..... |
| iPS:43 5309 | 21-225_146F9 | VH1\|1-08/D6\|6-19\|RF1/J H4 | ............<br>............<br>........ | N......... | ........<br>...... | ..H.....<br>........<br>.... | .................<br>..............S | SSGWY-<br>..................<br>.-F... | ......<br>..... |
| iPS:43 5321 | 21-225_147E4 | VH1\|1-08/D6\|6-19\|RF1/J H4 | ............<br>............<br>........ | N......... | ........<br>...... | ........<br>........<br>.... | .................<br>..............S | SSGWY-<br>..................<br>.-F... | ......<br>..... |
| iPS:43 5323 | 21-225_147D5 | VH1\|1-08/D6\|6-19\|RF1/J H4 | ............<br>............<br>........ | N......... | ........<br>...... | .VH.....<br>........<br>.... | .................<br>.....G........G | SSGWY-<br>..................<br>.-.... | ......<br>..... |
| iPS:43 5345 | 21-225_148G3 | VH1\|1-08/D6\|6-19\|RF1/J H4 | ............<br>............<br>........ | N......... | .....S..<br>...... | ..H.....<br>........<br>N... | .................<br>..............S | SSGWY-<br>..................<br>.-F... | ......<br>..... |
| iPS:43 5353 | 21-225_148F8 | VH1\|1-08/D6\|6-19\|RF1/J H4 | ............<br>............<br>........ | N......... | ........<br>...... | ........<br>........<br>.... | .................<br>..............S | SSGWY-<br>..................<br>.-.... | ......<br>..... |
| iPS:43 5369 | 21-225_149A2 | VH1\|1-08/D6\|6-19\|RF1/J H4 | ............<br>............<br>........ | N......... | ........<br>...... | .VH.....<br>........<br>.... | .................<br>..............G | SSGWY-<br>..................<br>.-.... | ......<br>..... |
| iPS:43 5373 | 21-225_149E3 | VH1\|1-08/D6\|6-19\|RF1/J H4 | ............<br>............<br>........ | N......... | ........<br>...... | ..H.....<br>........<br>.... | .................<br>...T..........Y | SSGWY-<br>..................<br>.-W... | ......<br>..... |
| iPS:43 | 21- | VH1\|1-08/D6\|6- | ............<br>............ | N......... | ........<br>...... | ..H.....<br>....D... | ..........T.V....<br>.............TF | SSGWY-<br>.................. | ......<br>..... |

| | | | | | | | | | |
|---|---|---|---|---|---|---|---|---|---|
| iPS:43 5399 | 21-225_150D2 | VH1\|1-08/D6\|6-19\|RF1/JH4 | ............ ............ ........ | N......... | ........ ...... | ..H..... ........ .... | ................. ..............Y | SSGWY- ................... .-.... | ...... ..... |
| iPS:43 5405 | 21-225_150B7 | VH1\|1-08/D6\|6-19\|RF1/JH4 | ............ .......T.... ....FP.. | N......... | ........ ...... | ..H..... ........ .... | ..............L.. ..............S | SSGWY- ................... .-F... | ...... ..... |
| iPS:43 5433 | 21-225_152E3 | VH1\|1-08/D6\|6-19\|RF1/JH4 | ............ ............ ........ | N......... | ........ ...... | ..H..... ........ .... | ................. ..............S | SSGWY- ................... .-F... | ...... ..... |
| iPS:43 5435 | 21-225_152H3 | VH1\|1-08/D6\|6-19\|RF1/JH4 | ............ ............ .......P | N......... | ........ ...... | ..H..... ........ .... | ................. ..............Y | SSGWY- ................... .-W... | ...... ..... |
| iPS:43 5459 | 21-225_152E12 | VH1\|1-08/D6\|6-19\|RF1/JH4 | ............ ............ ........ | N......... | ........ ...... | ........ ........ .... | ................. ..............Y | SSGWY- ................... .-.... | ...... ..... |
| iPS:43 5471 | 21-225_153F11 | VH1\|1-08/D6\|6-19\|RF1/JH4 | ............ .E.......... ........ | N......... | ........ ...... | ..H..... ........ .... | ..........N...... ..............Y | SSGWY- ................... .-F..N | ...... ..... |
| iPS:43 5475 | 21-225_154H6 | VH1\|1-08/D6\|6-19\|RF1/JH4 | ............ ............ ........ | N......... | ........ ...... | ........ ........ .... | ................. ..............Y | SSGWY- ................... .-I... | ...... ..... |
| iPS:43 5481 | 21-225_154A11 | VH1\|1-08/D6\|6-19\|RF1/JH4 | ............ ............ ........ | N......... | ........ ...... | ........ ........ .... | ................. ..............S | SSGWY- ................... .-.... | ...... ..... |
| iPS:43 5491 | 21-225_155E5 | VH1\|1-08/D6\|6-19\|RF1/JH4 | ............ ............ ........ | N......... | ........ ...... | ..H..... ..S..... R... | ................. ..............F | SSGWY- ................... .-.... | ...... ..... |
| iPS:43 5495 | 21-225_155B6 | VH1\|1-08/D6\|6-19\|RF1/JH4 | ............ ............ ........ | N......... | ........ ...... | ........ ........ .... | ................. ..............Y | SSGWY- ................... .-.... | ...... ..... |
| iPS:43 5501 | 21-225_156H1 | VH1\|1-08/D6\|6-19\|RF1/JH4 | ............ ............ ........ | N......... | ........ ...... | .VH..... ........ .... | ................. ..............G | SSGWY- ................... .-.... | ...... ..... |
| iPS:43 | 21- | VH1\|1-08/D6\|6- | ............ R........... | N......... | ........ ...... | ..H..... ........ | .F............... ..............Y | SSGWY- ................... | ...... ..... |

| | | | | | | | | | |
|---|---|---|---|---|---|---|---|---|---|
| iPS:43 5589 | 21-225_160A4 | VH1\|1-08/D6\|6-19\|RF1/JH4 | .........T..<br>............<br>........ | N......... | ........<br>.P.... | ..H.....<br>......P.<br>.... | .................<br>..............Y | SSGWY-<br>...................<br>.-I... | ......<br>..... |
| iPS:43 5623 | 21-225_162D5 | VH1\|1-08/D6\|6-19\|RF1/JH4 | .........S..<br>............<br>........ | N......... | ........<br>...... | ..H.....<br>........<br>.... | ..........D......<br>...S.......F..F | SSGWY-<br>...................<br>.-F... | ......<br>..... |
| iPS:43 5627 | 21-225_162F6 | VH1\|1-08/D6\|6-19\|RF1/JH4 | ............<br>R...........<br>........ | N......... | ........<br>...... | ..H.....<br>........<br>.... | .F...............<br>..............Y | SSGWY-<br>...................<br>.-R... | ......<br>..... |
| iPS:43 5649 | 21-225_165H2 | VH1\|1-08/D6\|6-19\|RF1/JH4 | ............<br>............<br>........ | H......... | ........<br>....V. | ..H.....<br>.HK.....<br>.... | .........N.....D.<br>..............Y | SSGWY-<br>...................<br>.-M... | ......<br>..... |
| iPS:43 5727 | 21-225_172E11 | VH1\|1-08/D6\|6-19\|RF1/JH4 | ............<br>.......M....<br>........ | N......... | ........<br>...... | ..H.....<br>........<br>.... | .................<br>..............Y | SSGWY-<br>...................<br>.-R... | ......<br>..... |
| iPS:43 5751 | 21-225_175D10 | VH1\|1-08/D6\|6-19\|RF1/JH4 | ............<br>............<br>........ | N.......L. | ........<br>...... | ..H.....<br>........<br>.... | ............V....<br>..............Y | SSGWY-<br>...................<br>.-.... | ......<br>..... |
| iPS:43 5773 | 21-225_177B12 | VH1\|1-08/D6\|6-19\|RF1/JH4 | ............<br>............<br>........ | N......... | ........<br>...... | ..H.....<br>........<br>.... | .................<br>..............Y | SSGWY-<br>...................<br>.-...F | ......<br>..... |
| iPS:43 5801 | 21-225_181E5 | VH1\|1-08/D6\|6-19\|RF1/JH4 | ............<br>............<br>........ | N......... | ........<br>...... | ........<br>........<br>.... | .................<br>..............S | SSGWY-<br>...................<br>.-I... | ......<br>..... |
| iPS:43 5841 | 21-225_191D8 | VH1\|1-08/D6\|6-19\|RF1/JH4 | ............<br>............<br>........ | N......... | ........<br>...... | ..H.....<br>........<br>.... | .................<br>..............H | SSGWY-<br>...................<br>.-.... | ......<br>..... |
| iPS:43 5855 | 21-225_191G3 | VH1\|1-08/D6\|6-19\|RF1/JH4 | ............<br>............<br>........ | N......... | ........<br>...... | R.......<br>........<br>.... | .................<br>..............H | SSGWY-<br>...................<br>.-I... | ......<br>..... |
| iPS:43 5915 | 21-225_190H4 | VH1\|1-08/D6\|6-19\|RF1/JH4 | ............<br>............<br>....N... | N......... | ........<br>...... | ........<br>........<br>.... | .................<br>..............H | SSGWY-<br>...................<br>.-I... | ......<br>..... |
| iPS:43 | 21- | VH1\|1-08/D6\|6- | ............<br>............ | N......... | ........<br>...... | ........<br>........ | .................<br>..............S | SSGWY-<br>................... | ......<br>..... |

| | | | | | | | | | |
|---|---|---|---|---|---|---|---|---|---|
| iPS:43 6021 | 21-225_193G4 | VH1\|1-08/D6\|6-19\|RF1/JH4 | ............<br>............<br>........ | N......... | ........<br>...... | ........<br>........<br>.... | ..........R......<br>N.............S | SSGWY-<br>...................<br>.-F... | ......<br>..... |
| iPS:43 6150 | 21-225_197H4 | VH1\|1-08/D6\|6-19\|RF1/JH4 | ............<br>............<br>........ | N......... | ........<br>...... | ..H.....<br>........<br>.... | .................<br>..............H | SSGWY-<br>...................<br>.-.... | ......<br>..... |
| iPS:43 6154 | 21-225_197C6 | VH1\|1-08/D6\|6-19\|RF1/JH4 | ............<br>............<br>........ | N......... | ........<br>...... | ..H.....<br>........<br>.... | .................<br>..............H | SSGWY-<br>...................<br>.-.... | ......<br>..... |
| iPS:43 6272 | 21-225_201F5 | VH1\|1-08/D6\|6-19\|RF1/JH4 | ..........A.<br>............<br>........ | N......... | ........<br>...... | ..H.....<br>........<br>.... | .................<br>..............Y | SSGWY-<br>...................<br>.-.... | ......<br>..... |
| iPS:43 6550 | 21-225_224D8 | VH1\|1-08/D6\|6-19\|RF1/JH4 | ............<br>............<br>........ | N......... | ........<br>...... | .LY.....<br>........<br>.... | .................<br>..............Y | SSGWY-<br>...................<br>.-.... | ......<br>.I... |
| iPS:43 6554 | 21-225_224C10 | VH1\|1-08/D6\|6-19\|RF1/JH4 | ............<br>............<br>....S... | N......... | ........<br>...... | ..H.....<br>........<br>.... | .................<br>..............Y | SSGWY-<br>...................<br>.-K... | ......<br>..... |
| iPS:43 6560 | 21-225_224F11 | VH1\|1-08/D6\|6-19\|RF1/JH4 | ............<br>............<br>........ | N......... | ........<br>...... | ........<br>........<br>.... | .................<br>..............Y | SSGWY-<br>...................<br>.-K... | ......<br>..... |
| iPS:43 6574 | 21-225_225F5 | VH1\|1-08/D6\|6-19\|RF1/JH4 | ............<br>...R........<br>....H... | N......... | ........<br>...... | ..H.....<br>.....F..<br>.... | .................<br>..............Y | SSGWY-<br>...................<br>.-R... | ......<br>..... |
| iPS:43 6584 | 21-225_225B9 | VH1\|1-08/D6\|6-19\|RF1/JH4 | .........T..<br>R...........<br>........ | N......... | ........<br>R..... | ..H.....<br>........<br>..R. | ..........N......<br>N.............Y | SSGWT-<br>...................<br>.-L... | ......<br>..... |
| iPS:43 6586 | 21-225_225F11 | VH1\|1-08/D6\|6-19\|RF1/JH4 | ............<br>............<br>........ | N......... | ........<br>...... | ..H.....<br>........<br>.... | ..........N......<br>..............Y | SSGWY-<br>...................<br>.-R... | ......<br>..... |
| iPS:43 6588 | 21-225_225F12 | VH1\|1-08/D6\|6-19\|RF1/JH4 | ............<br>............<br>........ | H......... | ........<br>...... | ..H.....<br>........<br>.... | ....A............<br>..............Y | SSGWY-<br>...................<br>.-K... | ......<br>..... |
| iPS:43 | 21- | VH1\|1-08/D6\|6- | ............<br>............ | N......... | ........<br>...... | ..H.....<br>........ | .................<br>..............Y | SSGWY-<br>................... | ......<br>..... |

| | | | | | | | | | |
|---|---|---|---|---|---|---|---|---|---|
| iPS:43 6598 | 21-225_226D6 | VH1\|1-08/D6\|6-19\|RF1/J H4 | ............<br>............<br>........ | N......... | ........<br>...... | ..H.....<br>........<br>.... | .................<br>..............Y | SSGWY-<br>...................<br>.-K... | ......<br>..... |
| iPS:43 6600 | 21-225_226F6 | VH1\|1-08/D6\|6-19\|RF1/J H4 | ............<br>............<br>........ | N......... | ........<br>...... | ..H.....<br>........<br>.... | ..........N......<br>..............Y | SSGWY-<br>...................<br>.-R... | ......<br>..... |
| iPS:43 6616 | 21-225_226D11 | VH1\|1-08/D6\|6-19\|RF1/J H4 | ............<br>...........R<br>S....... | N......... | ........<br>...... | ..H.....<br>........<br>.... | .................<br>..............Y | SSGWY-<br>...................<br>.-.... | ......<br>..... |
| iPS:43 6622 | 21-225_226A12 | VH1\|1-08/D6\|6-19\|RF1/J H4 | ............<br>............<br>........ | N......... | ........<br>...... | ..H.....<br>........<br>.... | .................<br>..............Y | SSGWY-<br>...................<br>.-.... | ......<br>..... |
| iPS:43 6636 | 21-225_227E6 | VH1\|1-08/D6\|6-19\|RF1/J H4 | ............<br>............<br>........ | N......... | ........<br>...... | ..H.....<br>........<br>.... | .................<br>..............Y | SSGWY-<br>...................<br>.-K... | ......<br>..... |
| iPS:43 6638 | 21-225_227C7 | VH1\|1-08/D6\|6-19\|RF1/J H4 | ............<br>...R........<br>....H... | N......... | ........<br>...... | ..H.....<br>........<br>.... | .................<br>..............Y | SSGWY-<br>...................<br>.-R... | ......<br>..... |
| iPS:43 6646 | 21-225_227D11 | VH1\|1-08/D6\|6-19\|RF1/J H4 | ............<br>............<br>........ | N......... | ........<br>...... | ..H.....<br>........<br>.... | .................<br>..............Y | SSGWY-<br>...................<br>.-.... | ......<br>..... |
| iPS:44 6086 | 21-225_94D8 | VH1\|1-08/D6\|6-19\|RF1/J H4 | ............<br>............<br>T....... | N......... | ........<br>...... | ........<br>........<br>...V | ...............V<br>..............Y | SSGWY-<br>...................<br>.-I... | ......<br>..... |
| iPS:45 1116 | 21-225_164A4 | VH1\|1-08/D6\|6-19\|RF1/J H4 | ............<br>............<br>....F..P | N......... | ........<br>...... | ..H.....<br>........<br>.... | .................<br>..............S | SSGWY-<br>...................<br>.-F... | ......<br>..... |
| iPS:45 1124 | 21-225_74F6 | VH1\|1-08/D6\|6-19\|RF1/J H4 | ............<br>.......R....<br>........ | N......... | ........<br>...... | ..H.....<br>........<br>.... | .........M.......<br>..............H | SSGWY-<br>...................<br>.-F... | ......<br>..... |
| iPS:45 1127 | 21-225_164A7 | VH1\|1-08/D6\|6-19\|RF1/J H4 | ............<br>............<br>........ | N.......V. | ........<br>...... | ..H.....<br>........<br>.... | .........T.......<br>.......S......S | SSGWY-<br>...................<br>.-L... | ......<br>..... |
| iPS:45 | 21- | VH1\|1-08/D6\|6- | ............<br>............ | N......... | ........<br>...... | ..H.H...<br>........ | ..........N......<br>..............H | SSGWY-<br>................... | ......<br>..... |

| | | | | | | | | | |
|---|---|---|---|---|---|---|---|---|---|
| iPS:39 2786 | 21-225_24E1 | VH1\|1-08/D6\|6-19\|RF1/JH4 | ............<br>............<br>........ | N......... | ........<br>...... | ..H.....<br>........<br>R... | .................<br>..............T | SSGWE-<br>...................<br>.-V... | ......<br>..... |
| iPS:39 2886 | 21-225_23A12 | VH1\|1-08/D6\|6-19\|RF1/JH4 | ............<br>............<br>.....P.. | N......... | ........<br>...... | ..H.....<br>........<br>.... | ..........N......<br>..............G | SSGWY-<br>...................<br>.-.... | ......<br>..... |
| iPS:39 2928 | 21-225_25A4 | VH1\|1-08/D6\|6-19\|RF1/JH4 | ..L.........<br>.R..........<br>........ | N......... | ........<br>...... | ..Y.....<br>........<br>.... | .................<br>..............S | SSGWY-<br>...................<br>.-.... | ......<br>..... |
| iPS:39 2936 | 21-225_28B6 | VH1\|1-08/D6\|6-19\|RF1/JH4 | ..L.........<br>.R..........<br>........ | N......... | ........<br>...... | ..H.D...<br>........<br>.... | .................<br>..............S | SSGWY-<br>...................<br>.-.... | ......<br>..... |
| iPS:39 2960 | 21-225_29E6 | VH1\|1-08/D6\|6-19\|RF1/JH4 | ..L.........<br>.R..........<br>........ | N......... | ........<br>...... | ..H.....<br>........<br>.... | .................<br>..............S | SSGWY-<br>...................<br>.-.... | ......<br>..... |
| iPS:39 2992 | 21-225_26C4 | VH1\|1-08/D6\|6-19\|RF1/JH4 | ............<br>............<br>........ | N......... | .....S..<br>...... | ........<br>........<br>R... | ..........N......<br>..............S | SSGWY-<br>...................<br>.-.... | ......<br>..... |
| iPS:39 3088 | 21-225_33D1 | VH1\|1-08/D6\|6-19\|RF1/JH4 | ............<br>............<br>........ | N......... | ........<br>...... | ..H.....<br>.....F..<br>..R. | .................<br>..............S | SSGWY-<br>...................<br>.-F... | ......<br>..... |
| iPS:39 3144 | 21-225_34D2 | VH1\|1-08/D6\|6-19\|RF1/JH4 | ............<br>............<br>........ | N......... | ........<br>....V. | .LH.....<br>..T..F..<br>..R. | ..............L..<br>..............S | SSGWY-<br>...................<br>.-F... | ......<br>..... |
| iPS:39 3368 | 21-225_29H8 | VH1\|1-08/D6\|6-19\|RF1/JH4 | ............<br>Q...........<br>........ | N......... | ........<br>...... | ........<br>........<br>R... | ...........A.....<br>..............S | SSGWY-<br>...................<br>.-.... | ......<br>..... |
| iPS:39 3942 | 21-225_11E5 | VH1\|1-08/D6\|6-19\|RF1/JH4 | .........P..<br>............<br>........ | N......... | ........<br>...... | ..H.....<br>..A.....<br>R... | .................<br>..............T | SSGWE-<br>...................<br>.-V... | ......<br>..... |
| iPS:39 4085 | 21-225_8B11 | VH1\|1-08/D6\|6-19\|RF1/JH4 | ............<br>............<br>........ | N......... | .....A..<br>...... | ..H.....<br>........<br>.... | .........R.......<br>..............Y | SSGWY-<br>...................<br>.-F... | ......<br>..... |
| iPS:39 | 21-225_22D2 | VH1\|1-08/D6\|6- | ............<br>............ | N......... | ........<br>...... | ..H.D...<br>........ | .................<br>..............Y | SSGWY-<br>................... | ......<br>..... |

| | | | | | | | | | |
|---|---|---|---|---|---|---|---|---|---|
| **iPS:398522** | **21-225_32A1** | **VH1\|1-08/D6\|6-19\|RF1/JH4** | ............ ............ ........ | N......... | ........ ...... | ........ ........ .... | ................. ..............S | SSGWY- .................. .-F... | ...... ..... |
| **iPS:398524** | **21-225_32A5** | **VH1\|1-08/D6\|6-19\|RF1/JH4** | ............ ............ ........ | N......... | ........ ...... | ..H..... .....F.. ..R. | ................. .............SS | SSGWY- .................. .-F... | ...... ..... |
| **iPS:398538** | **21-225_34H7** | **VH1\|1-08/D6\|6-19\|RF1/JH4** | ............ ............ ........ | N......... | ........ ...... | ........ ........ .... | ................. N.............S | SSGWY- .................. .-F... | ...... ..... |
| | | **Germline** | **H_FR1** | **H_CDR1** | **H_FR2** | **H_CDR2** | **H_FR3** | **H_CDR3** | **H_FR4** |
| | **VH1\|1-02/D1\|1-1\|RF1/JH4** | | QVQLVQS- GAEVKKPGASVK VSCKASG-YTFT | G-----YYMH | WVRQAPGQ GLEWMG | WINPN--- SGGTNYAQ KFQG | RVTMTRDTSISTAYMEL SRLRSDDTAVYYCAR | GTTGT------------ --------YFDY | WGQGTL VTVSS |
| **iPS:473253** | **21-225_7C3_LC1** | **VH1\|1-02/D1\|1-1\|RF1/JH4** | ............ ............ .......N | D.......L. | ........ ...... | ..H..... ........ .... | ................. .S.........S... | DG.S- .................. ...S... | ...... ..... |
| **iPS:473254** | **21-225_7C3_LC2** | **VH1\|1-02/D1\|1-1\|RF1/JH4** | ............ ............ .......N | D.......L. | ........ ...... | ..H..... ........ .... | ................. .S.........S... | DG.S- .................. ...S... | ...... ..... |
| **iPS:473255** | **21-225_9F12_LC1** | **VH1\|1-02/D1\|1-1\|RF1/JH4** | ............ ............ .......A | D.......L. | ........ ...... | ..H..... .....F.. .... | ..............L.. .S.......F..... | DG.S- .................. ...S... | ...... ..... |
| **iPS:473256** | **21-225_9F12_LC2** | **VH1\|1-02/D1\|1-1\|RF1/JH4** | ............ ............ .......A | D.......L. | ........ ...... | ..H..... .....F.. .... | ..............L.. .S.......F..... | DG.S- .................. ...S... | ...... ..... |
| **iPS:426108** | **21-225_10G6** | **VH1\|1-02/D1\|1-1\|RF1/JH4** | ............ ............ ........ | A......H.. | ........ ...... | ........ NN...... .... | ................. .............G. | DV.S- .................. ...S... | ...... ..... |
| **iPS:426110** | **21-225_12E9** | **VH1\|1-02/D1\|1-1\|RF1/JH4** | ............ ............ ........ | D.......L. | ........ ...... | .VH..... .....F.. ...D | ................. .S.......I.S... | DG.S- .................. ...S... | ...... ..... |
| **iPS:453451** | **21-225_52G11** | **VH1\|1-02/D1\|1-1\|RF1/JH4** | ............ ............ ........ | ........L. | ........ ...... | ........ RN...... .... | .............F... ............... | DG.S- .................. ...S... | ...... ..... |

| | | | | | | | | | |
|---|---|---|---|---|---|---|---|---|---|
| iPS:45 3453 | 21-225_53F2 | VH1\|1-02/D1\|1-1\|RF1/JH4 | ............<br>............<br>........ | .........L. | ........<br>...... | ........<br>RN......<br>N... | ................<br>...K.......... | DG.S-<br>...................<br>...S... | ......<br>..... |
| iPS:43 4035 | 21-225_49F10 | VH1\|1-02/D1\|1-1\|RF1/JH4 | ............<br>............<br>........ | .......H.. | ........<br>...... | ........<br>NNA.....<br>N... | ...L............<br>.............. | DG.S-<br>...................<br>...S..F | ......<br>..... |
| iPS:43 4065 | 21-225_50D4 | VH1\|1-02/D1\|1-1\|RF1/JH4 | ............<br>............<br>........ | .......H.. | ........<br>...... | ........<br>NNA.....<br>S... | ...L............<br>.............. | DG.S-<br>...................<br>...S..F | ......<br>..... |
| iPS:43 4069 | 21-225_51E9 | VH1\|1-02/D1\|1-1\|RF1/JH4 | ............<br>............<br>..R..... | .......HI. | ........<br>...... | ........<br>TN..Q...<br>.... | ................<br>.............. | DG.S-<br>...................<br>...S... | ......<br>..... |
| iPS:43 4079 | 21-225_52B1 | VH1\|1-02/D1\|1-1\|RF1/JH4 | ............<br>............<br>........ | .......H.Q | ........<br>...... | ........<br>..A.....<br>N... | ..............LD.<br>.............. | DG.S-<br>...................<br>...S... | ......<br>..... |
| iPS:43 4097 | 21-225_52H10 | VH1\|1-02/D1\|1-1\|RF1/JH4 | ............<br>............<br>........ | .......H.Q | ........<br>...... | ........<br>N...Q...<br>.... | ................<br>.............. | DG.S-<br>...................<br>...S... | ......<br>..... |
| iPS:43 4123 | 21-225_53F7 | VH1\|1-02/D1\|1-1\|RF1/JH4 | ............<br>............<br>........ | .......H.. | ........<br>...... | ........<br>NN......<br>.... | ................<br>N..T.......... | DG.S-<br>...................<br>...S... | ......<br>..... |
| iPS:43 4189 | 21-225_56E5 | VH1\|1-02/D1\|1-1\|RF1/JH4 | ............<br>...........Q<br>........ | .......H.. | ........<br>...... | ........<br>NNA.....<br>.... | ................<br>R..........H... | DG.S-<br>...................<br>...S... | ......<br>..... |
| iPS:43 5677 | 21-225_169C10 | VH1\|1-02/D1\|1-1\|RF1/JH4 | ............<br>.......R....<br>........ | .......F.. | ........<br>...... | ..K.K...<br>.....S..<br>R... | ..........N......<br>N............. | .G.TVAT..........<br>......WGV... | ......<br>..... |
| iPS:43 5699 | 21-225_170D6 | VH1\|1-02/D1\|1-1\|RF1/JH4 | ............<br>.......R....<br>........ | .......FI. | ........<br>...... | ..K.....<br>.....S..<br>R... | ..........N......<br>N............. | .G.TVAT..........<br>......WGV... | ......<br>..... |
| iPS:43 5797 | 21-225_181G2 | VH1\|1-02/D1\|1-1\|RF1/JH4 | ............<br>.T.........R<br>........ | S......N.. | ....V...<br>...... | ........<br>N..S..T.<br>.... | ................<br>.............. | KF---<br>...................<br>...--GD | ......<br>..... |
| iPS:43 | 21- | VH1\|1-02/D1\|1- | ............<br>.T.........R | S......N.. | ....V...<br>...... | ........<br>N..S..T. | ................<br>.............. | KF---<br>................... | ......<br>..... |

| | | | | | | | | | |
|---|---|---|---|---|---|---|---|---|---|
| iPS:435885 | 21-225_185E10 | VH1\|1-02/D1\|1-1\|RF1/JH4 | .............T.........R........ | S......N.. | ....V......... | ........N..S..T..... | ................................ | KF---......................--GD | ........... |
| iPS:435891 | 21-225_188H5 | VH1\|1-02/D1\|1-1\|RF1/JH4 | .............T.........R........ | S......N.. | ....V......... | ...........S..T..... | .I.............................. | KF---......................--GD | ........... |
| iPS:435897 | 21-225_188B9 | VH1\|1-02/D1\|1-1\|RF1/JH4 | .............T.........R........ | S......N.. | ....V......... | ...........S..T..... | ................................ | KF---......................--GD | ........... |
| iPS:436400 | 21-225_213H7 | VH1\|1-02/D1\|1-1\|RF1/JH4 | ................................ | .......H.. | .............. | ....K....D.......... | ................................ | EKP.S.....................YK. | ........... |
| iPS:436488 | 21-225_221A6 | VH1\|1-02/D1\|1-1\|RF1/JH4 | ........................T....... | .......... | .............. | ..H................. | ...L..........D................. | DG.S-......................S... | ........... |
| iPS:436496 | 21-225_222E1 | VH1\|1-02/D1\|1-1\|RF1/JH4 | ........................T....... | .......... | .............. | ..H................. | ...L..........D................. | DG.S-......................S... | ........... |
| iPS:436508 | 21-225_222F7 | VH1\|1-02/D1\|1-1\|RF1/JH4 | ........................T....... | .......... | .............. | ..H................. | ...L..........D................. | DG.S-......................S... | ........... |
| iPS:436516 | 21-225_222C12 | VH1\|1-02/D1\|1-1\|RF1/JH4 | ........................T....... | .......... | .............. | ..H................. | ...L..........D................. | DG.S-......................S... | ........... |
| iPS:437264 | 21-225_171H12 | VH1\|1-02/D1\|1-1\|RF1/JH4 | ...................R............ | .......F.. | .............. | ..K.K.........S..R... | ..........N......N.............. | .G.TVAT................WGV... | ........... |
| iPS:437266 | 21-225_177A5 | VH1\|1-02/D1\|1-1\|RF1/JH4 | ...................R............ | .......F.. | .............. | ..K.K.........S..R... | ..........N......NW............. | .G.TVAT................WGV... | ........... |
| iPS:393080 | 21-225_34F3 | VH1\|1-02/D1\|1-1\|RF1/JH4 | ................................ | .......H.. | ..........D... | .................... | ................................ | DG.S-......................S... | ........... |
| iPS:39 | 21-225_35C6 | VH1\|1-02/D1\|1- | .........T.................. | ......D... | .............. | ..S.K...N....... | .................N.............. | DG..-................... | ........... |

| | | | | | | | | | |
|---|---|---|---|---|---|---|---|---|---|
| iPS:39 3086 | 21-225_36H5 | VH1\|1-02/D1\|1-1\|RF1/JH 4 | ............<br>......M.....<br>........ | D......H.. | ........<br>...... | ........<br>R.......<br>...D | .................<br>...........F... | DG..-<br>...................<br>...S... | ......<br>..... |
| iPS:39 3098 | 21-225_35G6 | VH1\|1-02/D1\|1-1\|RF1/JH 4 | ..........D.<br>............<br>........ | D......HI. | ........<br>...... | ........<br>N...H...<br>E... | .................<br>.S............. | DG..-<br>...................<br>...S... | ......<br>..... |
| iPS:39 3112 | 21-225_33G1 | VH1\|1-02/D1\|1-1\|RF1/JH 4 | ....A.......<br>............<br>........ | .......... | ........<br>...... | ..S.....<br>N.......<br>.... | .................<br>............... | DG..-<br>...................<br>...S... | ......<br>..... |
| iPS:39 3116 | 21-225_34G7 | VH1\|1-02/D1\|1-1\|RF1/JH 4 | ............<br>...........R<br>........ | D......HI. | ........<br>...... | ........<br>N...H...<br>.... | .................<br>............... | DG..-<br>...................<br>...S... | ....N.<br>..... |
| iPS:39 3132 | 21-225_33H7 | VH1\|1-02/D1\|1-1\|RF1/JH 4 | ..........D.<br>............<br>........ | D......HI. | ........<br>...... | ........<br>N...H...<br>E... | .................<br>.S.........H... | DG..-<br>...................<br>...S... | ......<br>..... |
| iPS:39 3140 | 21-225_35H12 | VH1\|1-02/D1\|1-1\|RF1/JH 4 | ............<br>............<br>........ | D.......I. | ........<br>...... | ........<br>R.......<br>.... | .................<br>............... | DG..-<br>...................<br>...S... | ......<br>..... |
| iPS:39 3954 | 21-225_4H6 | VH1\|1-02/D1\|1-1\|RF1/JH 4 | ............<br>............<br>........ | D.......L. | ........<br>...... | ..H.....<br>........<br>.... | ...............G.<br>.S............. | DG.S-<br>...................<br>...S... | ......<br>..... |
| iPS:39 8484 | 21-225_18D4 | VH1\|1-02/D1\|1-1\|RF1/JH 4 | ............<br>............<br>........ | ........L. | ........<br>....L. | ........<br>.N..IS..<br>.... | .................<br>...I........... | DG.S-<br>...................<br>...SL.. | ......<br>..... |
| iPS:39 8502 | 21-225_23B11 | VH1\|1-02/D1\|1-1\|RF1/JH 4 | ............<br>............<br>........ | ........L. | ........<br>...... | ........<br>NN......<br>.... | .................<br>............... | DG.S-<br>...................<br>...S... | ......<br>..... |
| iPS:39 8520 | 21-225_31C4 | VH1\|1-02/D1\|1-1\|RF1/JH 4 | .........T..<br>............<br>........ | ......D... | ........<br>...... | ..S.K...<br>N.......<br>.... | ............V....<br>N.............. | DG..-<br>...................<br>...S... | ......<br>..... |
| iPS:40 2223 | 21-225_30A11 | VH1\|1-02/D1\|1-1\|RF1/JH 4 | ............<br>............<br>........ | D......H.. | ........<br>...... | ........<br>R.......<br>...D | .................<br>...........F... | DG..-<br>...................<br>...S... | ......<br>..... |
| | | Germline | H_FR1 | H_CDR1 | H_FR2 | H_CDR2 | H_FR3 | H_CDR3 | H_FR4 |

| | VH3\|3-33/D6\|6-6\|RF1/JH6 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | EYSSSSYY----------<br>-----YYYGMDV | WGQGTT<br>VTVSS |
|---|---|---|---|---|---|---|---|---|---|
| iPS:426114 | 21-225_28H2 | VH3\|3-33/D6\|6-6\|RF1/JH6 | ............<br>............<br>........ | N......V.. | ........<br>...... | ........<br>........<br>.... | .................<br>.....V......... | .EY..GW-<br>...............D.<br>.... | ......<br>..... |
| iPS:426116 | 21-225_29E2 | VH3\|3-33/D6\|6-6\|RF1/JH6 | ............<br>............<br>........ | N.....CV.. | ........<br>...... | ........<br>........<br>.... | .I...............<br>............... | .EY..GW-<br>...............D.<br>.... | ......<br>..... |
| iPS:468812 | 21-225_48H4 | VH3\|3-33/D6\|6-6\|RF1/JH6 | ............<br>............<br>........ | D.....SL.. | ........<br>...... | ........<br>......T.<br>.... | .................<br>............... | .NY..GW-<br>...............G.<br>.... | ......<br>..... |
| iPS:468816 | 21-225_52G8 | VH3\|3-33/D6\|6-6\|RF1/JH6 | ............<br>............<br>........ | .......... | ........<br>...... | ........<br>........<br>.... | .................<br>.........L..... | R....W--<br>...............-<br>SG.... | ......<br>..... |
| iPS:468826 | 21-225_201C5 | VH3\|3-33/D6\|6-6\|RF1/JH6 | ............<br>............<br>........ | D......V.. | ........<br>...... | ........<br>......V.<br>.... | .........R.......<br>............... | .RY..GL-<br>...............D.<br>.... | ......<br>..... |
| iPS:468842 | 21-225_50H4 | VH3\|3-33/D6\|6-6\|RF1/JH6 | ............<br>............<br>........ | .......V.. | ........<br>...... | A.......<br>........<br>.... | .............D...<br>............... | .LY..NW-<br>...............D.<br>.... | ......<br>..... |
| iPS:468858 | 21-225_148C9 | VH3\|3-33/D6\|6-6\|RF1/JH6 | ............<br>A...........<br>........ | D......... | ........<br>...... | ........<br>........<br>.... | .L...............<br>............... | .RY..GW-<br>...............D.<br>.L.. | ......<br>..... |
| iPS:468860 | 21-225_224E7 | VH3\|3-33/D6\|6-6\|RF1/JH6 | ............<br>.......S....<br>........ | .......V.. | ........<br>...... | ........<br>........<br>.... | .................<br>............... | .QY...W-<br>...............DF<br>.L.. | ......<br>..... |
| iPS:433917 | 21-225_43E11 | VH3\|3-33/D6\|6-6\|RF1/JH6 | ............<br>............<br>.....S.. | D......... | ........<br>...... | ........<br>........<br>.... | .L...............<br>............... | R.VR.W--<br>...............-<br>VG.... | ......<br>..... |
| iPS:433919 | 21-225_44B3 | VH3\|3-33/D6\|6-6\|RF1/JH6 | ............<br>...........T<br>........ | D......... | ........<br>...... | ........<br>........<br>.... | .................<br>............... | .RY..GW-<br>...............D.<br>.... | ......<br>..... |
| iPS:433923 | 21-225_44D3 | VH3\|3-33/D6\|6-6\|RF1/JH | ............<br>............ | .......V.. | ........<br>...... | ........<br>........ | .................<br>............... | .RY..GL-<br>...............D. | ......<br>..... |

| | | | | | | | | | |
|---|---|---|---|---|---|---|---|---|---|
| iPS:43 3929 | 21-225_44D5 | VH3\|3-33/D6\|6-6\|RF1/JH6 | ............ ............ ........ | .......V.. | ........ ...... | ........ ........ .... | ................. ............... | VPY...W- ...............D. .... | ...... ..... |
| iPS:43 3935 | 21-225_44F9 | VH3\|3-33/D6\|6-6\|RF1/JH6 | ............ ............ ........ | .......V.. | ........ ...... | ........ ........ .... | ................. ............... | .PY...W- ...............D. .... | G..... ..... |
| iPS:43 3937 | 21-225_44B10 | VH3\|3-33/D6\|6-6\|RF1/JH6 | ............ ...........V ........ | .......... | ........ ...... | ........ ........ .... | ................. ............... | R....W-- ...............- VG.... | ...... ..... |
| iPS:43 3939 | 21-225_44C10 | VH3\|3-33/D6\|6-6\|RF1/JH6 | ............ ............ ........ | D.....CV.. | ........ ...... | ........ ........ .... | ................. ............... | .RY..GL- ...............D. .... | ...... ..... |
| iPS:43 3951 | 21-225_45B4 | VH3\|3-33/D6\|6-6\|RF1/JH6 | ............ ............ ........ | D.....CV.. | ........ ...... | ........ ........ .... | ................. ............... | .RY..GL- ...............D. .... | ...... ..... |
| iPS:43 3955 | 21-225_45B8 | VH3\|3-33/D6\|6-6\|RF1/JH6 | ............ ............ ........ | D.....CV.. | ........ ...... | ........ ........ .... | ................. ............... | .RY..GL- ...............D. .... | ...... ..... |
| iPS:43 3967 | 21-225_46C3 | VH3\|3-33/D6\|6-6\|RF1/JH6 | ............ ............ ........ | .......V.. | ........ ...... | ........ ........ .... | ................. ............... | .RY..GL- ...............D. .... | ...... ..... |
| iPS:43 3971 | 21-225_46D4 | VH3\|3-33/D6\|6-6\|RF1/JH6 | ............ ............ ........ | .......V.. | ........ ...... | ........ ........ .... | ................. ............... | VPY...W- ...............D. .... | ...... ..... |
| iPS:43 3979 | 21-225_46B9 | VH3\|3-33/D6\|6-6\|RF1/JH6 | ............ ...........S ........ | .......... | ........ ...... | ........ .R...... .... | .I............... ............... | R....W-- ...............- MG.... | ...... ..... |
| iPS:43 3985 | 21-225_47C1 | VH3\|3-33/D6\|6-6\|RF1/JH6 | ............ ............ ........ | D......... | ....T... ...... | ........ ........ .... | .............F... ....D.......... | R..R.W-- ...............- VG.... | ...... ..... |
| iPS:43 3991 | 21-225_47E7 | VH3\|3-33/D6\|6-6\|RF1/JH6 | ............ ............ ........ | I......... | ........ ...... | ........ ........ .... | ................. ............... | R..R.W-- ...............- VG.... | ...... ..... |
| iPS:43 | 21-225_48F2 | VH3\|3-33/D6\|6- | ............ ............ | N......V.. | ........ ...... | ........ ........ | ................. ............... | .RY...W- ...............D. | ...... ..... |

| | | | | | | | | | |
|---|---|---|---|---|---|---|---|---|---|
| iPS:43 4021 | 21-225_49C1 | VH3\|3-33/D6\|6-6\|RF1/JH6 | .............<br>............T<br>........ | ........... | ........<br>...... | ........<br>........<br>.... | .................<br>............... | R....W--<br>...............–<br>SG.... | ......<br>..... |
| iPS:43 4025 | 21-225_49G3 | VH3\|3-33/D6\|6-6\|RF1/JH6 | .............<br>.............<br>........ | ........... | ........<br>...... | ........<br>........<br>.... | .................<br>............... | R....W--<br>...............–<br>SG.... | ......<br>..... |
| iPS:43 4031 | 21-225_49E7 | VH3\|3-33/D6\|6-6\|RF1/JH6 | .............<br>.............<br>........ | ........... | ........<br>...... | ........<br>........<br>.... | .................<br>.........L..... | R....W--<br>...............–<br>SG.... | ......<br>..... |
| iPS:43 4033 | 21-225_49F9 | VH3\|3-33/D6\|6-6\|RF1/JH6 | .............<br>.............<br>........ | ........... | ........<br>...... | L.......<br>.R......<br>.... | ........P........<br>...........H... | R....W--<br>...............–<br>SG.... | ......<br>..... |
| iPS:43 4053 | 21-225_51E1 | VH3\|3-33/D6\|6-6\|RF1/JH6 | .............<br>.............<br>........ | ........... | ........<br>...... | ........<br>..S.....<br>.... | .................<br>............... | R....W--<br>...............–<br>SG.... | ......<br>..... |
| iPS:43 4093 | 21-225_52D10 | VH3\|3-33/D6\|6-6\|RF1/JH6 | .............<br>.............<br>........ | ........... | ........<br>...... | L.......<br>.....H..<br>.... | .................<br>............... | R....W--<br>...............–<br>SG.... | ......<br>..... |
| iPS:43 4137 | 21-225_54D4 | VH3\|3-33/D6\|6-6\|RF1/JH6 | .............<br>.............<br>........ | ........... | ........<br>...... | ........<br>........<br>.... | .................<br>............... | R....W--<br>...............–<br>SG.... | ......<br>..... |
| iPS:43 4149 | 21-225_55H1 | VH3\|3-33/D6\|6-6\|RF1/JH6 | .............<br>.............<br>........ | ........... | ........<br>...... | ........<br>........<br>.... | .................<br>............... | R....W--<br>...............–<br>SG.... | ......<br>..... |
| iPS:43 4151 | 21-225_55C2 | VH3\|3-33/D6\|6-6\|RF1/JH6 | .............<br>.............<br>........ | ........... | ........<br>...... | L.......<br>.....H..<br>.... | .................<br>............... | R....W--<br>...............–<br>SG.... | ......<br>..... |
| iPS:43 4161 | 21-225_55F9 | VH3\|3-33/D6\|6-6\|RF1/JH6 | .............<br>.............<br>........ | ........... | ........<br>...... | ........<br>.NG.....<br>.... | .................<br>............... | R....W--<br>...............–<br>SG.... | ......<br>..... |
| iPS:43 4201 | 21-225_59A12 | VH3\|3-33/D6\|6-6\|RF1/JH6 | .............<br>.............<br>........ | ........... | ........<br>...... | ........<br>........<br>.... | .................<br>............... | R....W--<br>...............–<br>DG.... | ......<br>..... |
| iPS:43 | 21-225_60G2 | VH3\|3-33/D6\|6- | .............<br>............. | ........... | ........<br>...... | ........<br>........ | .................<br>............... | R..R.W--<br>...............– | ......<br>..... |

| | | | | | | | | | |
|---|---|---|---|---|---|---|---|---|---|
| iPS:43 4223 | 21-225_60C12 | VH3\|3-33/D6\|6-6\|RF1/JH6 | ............ ............ ........ | .......... | ........ ...... | ........ ......V. .... | ................. ............... | R..R.W-- ...............- TG.... | ...... ..... |
| iPS:43 4231 | 21-225_61F2 | VH3\|3-33/D6\|6-6\|RF1/JH6 | ............ ............ ........ | .......... | ........ ...... | ........ ........ .... | ................. ............... | .RY..GW- ...............D. .... | ...... ..... |
| iPS:43 4233 | 21-225_61B3 | VH3\|3-33/D6\|6-6\|RF1/JH6 | ............ ............ ........ | .......... | ........ ...... | ........ ........ .... | ................. ............... | R..R.W-- ...............- AG.... | ...... ..... |
| iPS:43 4303 | 21-225_58H11 | VH3\|3-33/D6\|6-6\|RF1/JH6 | ............ ............ ........ | .......... | ........ ...... | ........ .....H.. .... | ................. ............... | R....W-- ...............- DG.... | ...... ..... |
| iPS:43 4339 | 21-225_64A4 | VH3\|3-33/D6\|6-6\|RF1/JH6 | ............ ............ ........ | D......V.. | ........ ...... | ........ ........ .... | .........Q....... ............... | .RY...W- ...............D. .... | ...... ..... |
| iPS:43 4343 | 21-225_64C8 | VH3\|3-33/D6\|6-6\|RF1/JH6 | ............ ............ ........ | D......V.. | ........ ...... | ........ ........ .M.. | ................. ............... | .RY..GW- ...............D. .... | ...... ..... |
| iPS:43 4387 | 21-225_66D11 | VH3\|3-33/D6\|6-6\|RF1/JH6 | ............ ...........G ........ | .......... | ........ ...... | ........ ........ .... | .............S... .........L..... | .MY..NW- ...............D. .L.. | ...... ..... |
| iPS:43 4469 | 21-225_73C9 | VH3\|3-33/D6\|6-6\|RF1/JH6 | ............ ............ ........ | N......V.. | ........ ...... | ........ ........ .... | ................. ............... | .RY...W- ..............FD. .... | ...... ..... |
| iPS:43 5197 | 21-225_94F3 | VH3\|3-33/D6\|6-6\|RF1/JH6 | ............ ............ ........ | N.....DI.. | ........ ...... | ........ ........ .... | ................I ...........F... | .KY..GW- ...............D. .... | ...... ..... |
| iPS:43 5315 | 21-225_147B2 | VH3\|3-33/D6\|6-6\|RF1/JH6 | ............ ............ ........ | .......... | ........ ...... | ........ ........ .... | ................. ............... | R....W-- ...............- SG.... | ...... ..... |
| iPS:43 5325 | 21-225_147H5 | VH3\|3-33/D6\|6-6\|RF1/JH6 | ............ A........... ........ | D......... | ........ ...... | ........ ........ .... | .L............... ...S........... | .RY..GW- ...............D. .L.. | ...... ..... |
| iPS:43 | 21- | VH3\|3-33/D6\|6- | ............ ............ | .......... | ........ ...... | ........ ........ | ................. ............... | R....W-- ...............- | ...... ..... |

| | | | | | | | | | |
|---|---|---|---|---|---|---|---|---|---|
| iPS:435349 | 21-225_148F5 | VH3\|3-33/D6\|6-6\|RF1/JH6 | ............ ............ ........ | ........... | ........ ...... | ........ ........ .... | ................. ............... | R....W-- ...............- SG.... | ...... ..... |
| iPS:435359 | 21-225_148H10 | VH3\|3-33/D6\|6-6\|RF1/JH6 | ............ ............ ........ | ........... | ........ ...... | ........ ......G. .... | ................. ............... | R....W-- ...............- SG.... | ...... ..... |
| iPS:435393 | 21-225_149D10 | VH3\|3-33/D6\|6-6\|RF1/JH6 | ............ A........... ........ | D......... | ........ ...... | ........ ........ .... | .L............... ............... | .RY..GW- ...............D. .... | ...... ..... |
| iPS:435401 | 21-225_150E2 | VH3\|3-33/D6\|6-6\|RF1/JH6 | ............ ............ ........ | .......V.. | ........ ...... | ........ ........ .... | ................. ............... | .EY...W- ...............G. .... | ...... ..... |
| iPS:435417 | 21-225_150D11 | VH3\|3-33/D6\|6-6\|RF1/JH6 | ............ ............ ........ | ........... | ........ ...... | ..F..... ....H... .... | ................. .......S.....T. | RF...W-- ...............- SG.... | ...... ..... |
| iPS:435445 | 21-225_152F7 | VH3\|3-33/D6\|6-6\|RF1/JH6 | ............ ............ ........ | .......I.. | ........ ...... | ........ ........ .... | ................. ............... | .EY...W- ...............G. .... | ...... ..... |
| iPS:435469 | 21-225_153G9 | VH3\|3-33/D6\|6-6\|RF1/JH6 | ............ ............ ........ | ........... | ........ ...... | L.F..... ........ .... | ................I ...........F... | R..R.W-- ...............- AG.... | .....A ..... |
| iPS:435573 | 21-225_159D8 | VH3\|3-33/D6\|6-6\|RF1/JH6 | ............ ............ ........ | N......V.. | C....... ...... | ........ ........ .... | ................. ............... | .PY..GW- ...............D. .... | ...... ..... |
| iPS:435681 | 21-225_169D11 | VH3\|3-33/D6\|6-6\|RF1/JH6 | ............ ............ ........ | D......V.. | ........ ...... | ........ ........ .... | ................L ............... | .RY..GW- ...............D. .... | ...... ..... |
| iPS:435689 | 21-225_170F3 | VH3\|3-33/D6\|6-6\|RF1/JH6 | ............ ............ .....S.. | D......V.. | ........ ...... | ........ ......E. .... | ................. ............... | .TY...W- ...............D. .... | ...... ..... |
| iPS:435733 | 21-225_173C11 | VH3\|3-33/D6\|6-6\|RF1/JH6 | ............ ............ ........ | .........I. | ........ ...... | L.F..... ........ .... | ...............H. S.............. | R....W-- ...............- SG.... | ...... ..... |
| iPS:43 | 21- | VH3\|3-33/D6\|6- | ............ ............ | D......V.. | ........ ...... | ........ ........ | ................L ............... | .RY..GW- ...............D. | ...... ..... |

| | | | | | | | | | |
|---|---|---|---|---|---|---|---|---|---|
| iPS:43 5763 | 21-225_176H12 | VH3\|3-33/D6\|6-6\|RF1/JH6 | .................................. | D......V.. | .............. | .................... | ................................ | .KY..NW-.................D..... | ........... |
| iPS:43 5767 | 21-225_177B4 | VH3\|3-33/D6\|6-6\|RF1/JH6 | .................................. | D......V.. | .............. | .................... | ..IV............................ | .KY...W-.................D..L.. | ........... |
| iPS:43 5785 | 21-225_179C2 | VH3\|3-33/D6\|6-6\|RF1/JH6 | .................................. | .......... | .............. | L.F................. | ..............F.................. | R..G.W--.................-SG.... | ........... |
| iPS:43 5921 | 21-225_190D6 | VH3\|3-33/D6\|6-6\|RF1/JH6 | .................................. | ......FI.. | .......R...... | .................... | ................................ | .EY..GW-...............FG..... | ........... |
| iPS:43 5961 | 21-225_192A2 | VH3\|3-33/D6\|6-6\|RF1/JH6 | .................................. | .......... | ............L. | ..........Y......... | ................................ | .D.PY.G.A.........…LD.F..... | ........... |
| iPS:43 5985 | 21-225_192F6 | VH3\|3-33/D6\|6-6\|RF1/JH6 | .................................. | ......FI.. | .......R...... | ..............V..... | ................................ | .EY..GW-...............FG..... | ........... |
| iPS:43 6039 | 21-225_193F8 | VH3\|3-33/D6\|6-6\|RF1/JH6 | .................................. | I.......D | .............. | ..........Y......... | ..............................V. | .D.PY.G.G............LD....... | ........... |
| iPS:43 6074 | 21-225_194F10 | VH3\|3-33/D6\|6-6\|RF1/JH6 | .................................. | ......FI.. | .......R...... | .................... | ...............E.....V.......... | .EY..GW-...............FG..... | ........... |
| iPS:43 6264 | 21-225_203F7 | VH3\|3-33/D6\|6-6\|RF1/JH6 | .................................. | D......V.. | .............. | ..............V..... | .........R..................... | .RY..GL-.................D..... | ........... |
| iPS:43 6274 | 21-225_204H3 | VH3\|3-33/D6\|6-6\|RF1/JH6 | .............G.........T........ | .......V.. | .............. | .............N...... | ................................ | .PY...W-.................D..... | S.......... |
| iPS:43 6332 | 21-225_208B2 | VH3\|3-33/D6\|6-6\|RF1/JH6 | .................................. | D.....CV.. | .............. | ..............V..... | .........R..................... | .RY..GL-.................D..L.. | ........... |
| iPS:43 | 21- | VH3\|3-33/D6\|6- | .......................... | D.....CV.. | .............. | ..............V. | .........R..................... | .RY..GL-.................D. | ........... |

| | | | | | | | | | |
|---|---|---|---|---|---|---|---|---|---|
| iPS:43 6386 | 21-225_212B11 | VH3\|3-33/D6\|6-6\|RF1/JH6 | ................................ | D......V.. | .............T | .................... | ............................... | .RY..GW-...............D..... | ........... |
| iPS:43 6412 | 21-225_214H9 | VH3\|3-33/D6\|6-6\|RF1/JH6 | ................................ | .......V.. | .............. | .................... | ...........................F.... | .RYT..W-...............D..... | ........... |
| iPS:43 6414 | 21-225_214G10 | VH3\|3-33/D6\|6-6\|RF1/JH6 | ................................ | D......V.. | .............T | .................... | ............................... | .RY..GW-...............D..... | ........... |
| iPS:43 6416 | 21-225_214G12 | VH3\|3-33/D6\|6-6\|RF1/JH6 | ................................ | D......V.. | .............T | .................... | ............................... | .RY..GW-...............D..... | ........... |
| iPS:43 6418 | 21-225_215E3 | VH3\|3-33/D6\|6-6\|RF1/JH6 | ................................ | D......VI. | .............T | .................... | ...................V........... | .RY..GW-...............D..... | ........... |
| iPS:43 6428 | 21-225_215E11 | VH3\|3-33/D6\|6-6\|RF1/JH6 | ................................ | D......V.. | .............T | .................... | ............................... | .RY..GW-...............D..... | ........... |
| iPS:43 6438 | 21-225_216E8 | VH3\|3-33/D6\|6-6\|RF1/JH6 | ................................ | D......V.. | .............T | .................... | ............................... | .RY..GW-...............D..... | ........... |
| iPS:43 6440 | 21-225_216H12 | VH3\|3-33/D6\|6-6\|RF1/JH6 | ................................ | D......V.. | .............T | .................... | ............................... | .RY..GW-...............D..... | ........... |
| iPS:43 6450 | 21-225_217E5 | VH3\|3-33/D6\|6-6\|RF1/JH6 | ................................ | D......V.. | .............T | .................... | ............................... | .RY..GW-...............D..... | ........... |
| iPS:43 6456 | 21-225_217G10 | VH3\|3-33/D6\|6-6\|RF1/JH6 | ................................ | D......V.. | .............T | .................... | ............................... | .RY..GW-...............D..... | ........... |
| iPS:43 6458 | 21-225_217H12 | VH3\|3-33/D6\|6-6\|RF1/JH6 | ................................ | D......V.. | .............T | .................... | ............................... | .RY..GW-...............D..... | ........... |
| iPS:43 | 21- | VH3\|3-33/D6\|6- | ........................ | D......V.. | .............T | ................ | ............................... | .RY..GW-...............D. | ........... |

| | | | | | | | | | |
|---|---|---|---|---|---|---|---|---|---|
| iPS:436480 | 21-225_220F8 | VH3\|3-33/D6\|6-6\|RF1/JH6 | ................................ | D......V.. | .............T | .................... | ................................ | .RY..GW-...............D..... | ........... |
| iPS:436534 | 21-225_224F1 | VH3\|3-33/D6\|6-6\|RF1/JH6 | ................................ | .......I.. | .............. | .................... | ................................ | .RY..NW-...............D..... | ........... |
| iPS:436540 | 21-225_224F3 | VH3\|3-33/D6\|6-6\|RF1/JH6 | ........................ .....S.. | .......... | .............. | .................... | ................................ | .RY...W-...............D..... | ........... |
| iPS:436564 | 21-225_225A1 | VH3\|3-33/D6\|6-6\|RF1/JH6 | ................................ | D......VI. | .............. | .................... | ................................ | .RY..GW-...............D..... | ........... |
| iPS:436596 | 21-225_226C6 | VH3\|3-33/D6\|6-6\|RF1/JH6 | ................................ | D......... | .............. | .................... | ................T............... | .RY...W-...............D..... | ........... |
| iPS:436620 | 21-225_226H11 | VH3\|3-33/D6\|6-6\|RF1/JH6 | ................................ | N.....C... | .............T | .................... | ................................ | .LY...W-...............D..L.. | ........... |
| iPS:436744 | 21-225_154F4 | VH3\|3-33/D6\|6-6\|RF1/JH6 | ................................ | .......... | .............. | .................... | ................................ | .D.YC.GTSC..........PYY....... | ........... |
| iPS:436946 | 21-225_183F4 | VH3\|3-33/D6\|6-6\|RF1/JH6 | ................................ | .......... | .............. | .................... | ................................ | .RTYC.GTTC..........PYY....LG. | ........... |
| iPS:437286 | 21-225_208F1 | VH3\|3-33/D6\|6-6\|RF1/JH6 | ..........................R..... | D......V.. | .............. | ..............V..... | .........R...................... | .RY..GL-...............D..... | ........... |
| iPS:437290 | 21-225_210G6 | VH3\|3-33/D6\|6-6\|RF1/JH6 | ................................ | D......V.. | .............. | ..............V..... | .........R...................... | .RY..GL-...............D..... | ........... |
| iPS:392634 | 21-225_17H3 | VH3\|3-33/D6\|6-6\|RF1/JH6 | ................................ | N.....CV.. | .............. | .................... | ................................ | .KY...W-...............D..... | ........... |
| iPS:39 | 21-225_20B2 | VH3\|3-33/D6\|6- | ........................ | N......VI. | .............. | ................ | ............................S... | .KY...W-...............D. | ........... |

| | | | | | | | | | |
|---|---|---|---|---|---|---|---|---|---|
| iPS:39 2836 | 21-225_22F4 | VH3\|3-33/D6\|6-6\|RF1/JH6 | ............<br>............<br>........ | D......... | ........<br>...... | ........<br>......V.<br>.... | .................<br>............... | .KY...W-<br>...............D.<br>.... | ......<br>..... |
| iPS:39 2846 | 21-225_24B6 | VH3\|3-33/D6\|6-6\|RF1/JH6 | ............<br>......P.....<br>.....I.. | N......V.. | ........<br>...... | ........<br>........<br>.... | .................<br>............... | .EY..GW-<br>...............D.<br>.... | ......<br>..... |
| iPS:39 2884 | 21-225_23A10 | VH3\|3-33/D6\|6-6\|RF1/JH6 | ............<br>............<br>........ | .......... | ........<br>...... | ........<br>........<br>.... | .................<br>............... | .RY..GW-<br>..............HD.<br>.... | ......<br>..... |
| iPS:39 2888 | 21-225_25A2 | VH3\|3-33/D6\|6-6\|RF1/JH6 | ............<br>............<br>........ | .......... | ........<br>...... | ........<br>........<br>.... | .................<br>............... | R....W--<br>...............-<br>SG.... | ......<br>..... |
| iPS:39 2914 | 21-225_25D12 | VH3\|3-33/D6\|6-6\|RF1/JH6 | ............<br>............<br>........ | ......D... | ........<br>...... | ........<br>........<br>.... | .................<br>............... | .RY...W-<br>...............D.<br>.... | ......<br>..... |
| iPS:39 2924 | 21-225_32H2 | VH3\|3-33/D6\|6-6\|RF1/JH6 | ............<br>............<br>........ | .......... | ........<br>...... | ........<br>........<br>.... | .............N...<br>........G...... | R....W--<br>...............-<br>TG.... | ......<br>..... |
| iPS:39 2938 | 21-225_29H4 | VH3\|3-33/D6\|6-6\|RF1/JH6 | ............<br>............<br>........ | ........I. | ........<br>...... | ........<br>........<br>.... | .................<br>............... | R....W--<br>...............-<br>SG.... | ......<br>..... |
| iPS:39 2974 | 21-225_26A11 | VH3\|3-33/D6\|6-6\|RF1/JH6 | ............<br>............<br>........ | N.....CV.. | ........<br>...... | ........<br>........<br>.... | .................<br>............... | .EY..GW-<br>...............D.<br>.... | ......<br>..... |
| iPS:39 3012 | 21-225_26G7 | VH3\|3-33/D6\|6-6\|RF1/JH6 | ............<br>............<br>........ | .......... | ........<br>...... | ........<br>........<br>.... | .........N.......<br>............... | R....W--<br>...............-<br>SG.... | ......<br>..... |
| iPS:39 3176 | 21-225_27E7 | VH3\|3-33/D6\|6-6\|RF1/JH6 | ............<br>............<br>........ | .......... | ........<br>...... | ........<br>........<br>.... | .................<br>............... | .D.YC.STSC........<br>..PYY....... | ......<br>..... |
| iPS:39 3864 | 21-225_4C5 | VH3\|3-33/D6\|6-6\|RF1/JH6 | ............<br>............<br>........ | ........VL. | ........<br>...... | ........<br>........<br>.... | .................<br>............... | .KYT..W-<br>...............D.<br>.... | ......<br>..... |
| iPS:39 | 21- | VH3\|3-33/D6\|6- | ............<br>............ | D......... | ........<br>...... | ........<br>........ | .................<br>............... | .KY...W-<br>...............D. | ......<br>..... |

| | | | | | | | | | |
|---|---|---|---|---|---|---|---|---|---|
| iPS:39 3908 | 21-225_10E9 | VH3\|3-33/D6\|6-6\|RF1/JH6 | ............ ............ ........ | N......VI. | ........ ...... | ........ ........ .... | ................. ............... | .KY...W- ...............D. .... | ...... ..... |
| iPS:39 3916 | 21-225_2G4 | VH3\|3-33/D6\|6-6\|RF1/JH6 | ............ ............ ........ | .......V.. | ........ ...... | ........ ........ .... | ................. ............... | .KY...W- ...............D. .... | ..H... ..... |
| iPS:39 3950 | 21-225_3H10 | VH3\|3-33/D6\|6-6\|RF1/JH6 | ............ ............ ........ | .......V.. | ........ ...... | ........ ........ .... | ................. ............... | .RY..GW- ...............D. .L.. | ...... ..... |
| iPS:39 3972 | 21-225_7C9 | VH3\|3-33/D6\|6-6\|RF1/JH6 | ............ ............ ....L... | N......V.. | ........ ...... | ........ ........ .... | ................. ............... | .KY...W- ...............D. .... | ...... ..... |
| iPS:39 3978 | 21-225_4C12 | VH3\|3-33/D6\|6-6\|RF1/JH6 | ............ ............ ........ | .......V.. | ........ ...... | ........ ........ .... | ................. ............... | .KY...W- ...............D. .... | ..H... ..... |
| iPS:39 3986 | 21-225_7G4 | VH3\|3-33/D6\|6-6\|RF1/JH6 | ............ ............ .......N | N......V.. | ........ ...... | ........ ........ .... | ................. T.............. | .KY..NW- ...............D. .... | ...... ..... |
| iPS:39 3996 | 21-225_15C11 | VH3\|3-33/D6\|6-6\|RF1/JH6 | ............ ............ ........ | D......V.. | ........ ...... | ........ ........ .... | ................. ............... | .KY...W- ...............D. .L.. | ...... ..... |
| iPS:39 4041 | 21-225_5E5 | VH3\|3-33/D6\|6-6\|RF1/JH6 | ............ ............ ........ | N......V.. | ........ ...... | ........ ........ .... | ................. .............T. | .VY..GW- ...............D. .... | ...... ..... |
| | | Germline | H_FR1 | H_CDR1 | H_FR2 | H_CDR2 | H_FR3 | H_CDR3 | H_FR4 |
| | VH3\|3-33/D2\|2-8\|RF3/JH4 | | QVQLVES- GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | DIVLMVY----------- ------AIYFDY | WGQGTL VTVSS |
| iPS:42 6118 | 21-225_7A10 | VH3\|3-33/D2\|2-8\|RF3/JH4 | ............ ............ .....N.. | .......... | ........ ....M. | ........ ........ .... | ................. H.............. | .ER.G-- .................- -I... | ...... ..... |
| iPS:39 3844 | 21-225_3G7 | VH3\|3-33/D2\|2-8\|RF3/JH4 | ............ ............ .....N.. | N......... | ........ ...... | ...H.... ......V. .... | ............V.... ............... | .ER.G-- .................- -I... | ...... ..... |

| | | | | | | | | | |
|---|---|---|---|---|---|---|---|---|---|
| iPS:39 3852 | 21-225_12A10 | VH3\|3-33/D2\|2-8\|RF3/JH4 | ............ ............ ........ | .......... | ........ ...... | ...H.... E.....T. .... | ................. ............... | .ER.G-- ................- -I... | ...... ..... |
| iPS:39 3868 | 21-225_9C11 | VH3\|3-33/D2\|2-8\|RF3/JH4 | ............ ............ ........ | .......... | ........ ...... | ...H.... ET...... .... | .............S... ............... | .ER.G-- ................- -I... | ...... ..... |
| iPS:39 3900 | 21-225_10E12 | VH3\|3-33/D2\|2-8\|RF3/JH4 | ............ ............ .....N.. | N......... | ....V... ...... | ...H.... ......V. .... | .............S... ...........C... | .ER.G-- ................- -I... | ...... ..... |
| iPS:39 3920 | 21-225_1H12 | VH3\|3-33/D2\|2-8\|RF3/JH4 | ............ ..S......... .....N.. | .......... | ........ ...... | I..H.... ......V. .... | ................. ............... | .ER.G-- ................- -I... | ...... ..... |
| iPS:39 3932 | 21-225_10F5 | VH3\|3-33/D2\|2-8\|RF3/JH4 | ............ ............ .....N.. | .......... | ........ .....S | I..H.... ......V. .... | ................. ............... | .ER.G-- ................- -I... | ...... ..... |
| | | Germline | H_FR1 | H_CDR1 | H_FR2 | H_CDR2 | H_FR3 | H_CDR3 | H_FR4 |
| | VH3\|3-33/D6\|6-6\|RF1/JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | EYSSS------------------SYFDY | WGQGTLVTVSS |
| iPS:42 6124 | 21-225_32D6 | VH3\|3-33/D6\|6-6\|RF1/JH4 | ............ ............ ........ | .......... | ........ .....T | ...H.... ...A.... .... | ................. ............... | .N............... .......Y.... | ...... ..... |
| iPS:39 2922 | 21-225_30G4 | VH3\|3-33/D6\|6-6\|RF1/JH4 | ............ ............ ........ | .......... | .......R ...... | ........ .TD...V. .... | ................. .......S....... | .N............... .......Y.... | ...... ..... |
| iPS:39 3002 | 21-225_30G1 | VH3\|3-33/D6\|6-6\|RF1/JH4 | ............ ............ ........ | Y......... | ........ ...... | ...H.... ......V. .... | ................. ............... | .N............... .......Y.... | ...... ..... |
| iPS:39 3066 | 21-225_34D3 | VH3\|3-33/D6\|6-6\|RF1/JH4 | ............ ............ ........ | Y......... | ........ ...... | ...H.... ......V. .... | ................. ............... | .N............... .......F.... | ...... ..... |
| iPS:39 3092 | 21-225_33C12 | VH3\|3-33/D6\|6-6\|RF1/JH4 | ............ ............ ........ | H......... | ........ ...... | ........ ........ .... | ................. ............... | .N............... .......Y.... | ...... ..... |

| | | | | | | | | | |
|---|---|---|---|---|---|---|---|---|---|
| iPS:39 3100 | 21-225_36B8 | VH3\|3-33/D6\|6-6\|RF1/JH4 | .............<br>.............<br>........ | N......... | ........<br>...... | ...H....<br>......G.<br>.... | ..................<br>.............. | .N................<br>.......Y.... | ......<br>..... |
| iPS:39 3122 | 21-225_33B2 | VH3\|3-33/D6\|6-6\|RF1/JH4 | .............<br>.............<br>........ | N......... | ........<br>...... | ...H....<br>........<br>.... | ..................<br>.............. | .N................<br>.......Y.... | ......<br>..... |
| iPS:39 3134 | 21-225_34C2 | VH3\|3-33/D6\|6-6\|RF1/JH4 | .............<br>.............<br>........ | N......V.. | ........<br>...... | L.......<br>........<br>.... | ..................<br>.............. | .N................<br>.......Y.... | ......<br>..... |
| iPS:39 3136 | 21-225_34D8 | VH3\|3-33/D6\|6-6\|RF1/JH4 | .............<br>.............<br>........ | N......... | ........<br>...... | ........<br>........<br>.... | ..................<br>.............. | .N................<br>.......Y.... | ......<br>..... |
| | | Germline | H_FR1 | H_CDR1 | H_FR2 | H_CDR2 | H_FR3 | H_CDR3 | H_FR4 |
| | VH3\|3-30.3/D6\|6-19\|RF2/JH6 | | QVQLVES-<br>GGGVVQPGRSLR<br>LSCAASG-FTFS | S-----YAMH | WVRQAPGK<br>GLEWVA | VISYD---<br>GSNKYYAD<br>SVKG | RFTISRDNSKNTLYLQM<br>NSLRAEDTAVYYCAR | GIAVAGYY----------<br>-----YYYGMDV | WGQGTT<br>VTVSS |
| iPS:45 1135 | 21-225_64A11 | VH3\|3-30.3/D6\|6-19\|RF2/JH6 | .............<br>.............<br>........ | N......G.. | ........<br>...... | ....V...<br>........<br>.... | ..................<br>.T............ | RG...P--<br>...............-<br>...... | ......<br>..... |
| | | Germline | H_FR1 | H_CDR1 | H_FR2 | H_CDR2 | H_FR3 | H_CDR3 | H_FR4 |
| | VH1\|1-08/D6\|6-19\|RF1/JH5 | | QVQLVQS-<br>GAEVKKPGASVK<br>VSCKASG-YTFT | S-----YDIN | WVRQATGQ<br>GLEWMG | WMNPN---<br>SGNTGYAQ<br>KFQG | RVTMTRNTSISTAYMEL<br>SSLRSEDTAVYYCAR | GYSSGW------------<br>------YNWFDP | WGQGTL<br>VTVSS |
| iPS:45 1137 | 21-225_74A7 | VH1\|1-08/D6\|6-19\|RF1/JH5 | .............<br>.............<br>........ | N......... | ........<br>...... | ........<br>........<br>.... | .........T...H...<br>..............V | SSGWN-<br>..................<br>--.... | ......<br>..... |
| iPS:43 4285 | 21-225_57A11 | VH1\|1-08/D6\|6-19\|RF1/JH5 | .............<br>.............<br>........ | N......... | ........<br>...... | ........<br>.V......<br>.... | ......D..........<br>..............I | SSGWN-<br>..................<br>--.... | ......<br>..... |
| iPS:43 4287 | 21-225_57F12 | VH1\|1-08/D6\|6-19\|RF1/JH5 | .............<br>.............<br>........ | N......... | ........<br>...... | ........<br>........<br>.... | ..................<br>..............I | SSGWY-<br>..................<br>--R... | ......<br>..... |
| iPS:43 4479 | 21-225_76H1 | VH1\|1-08/D6\|6-19\|RF1/JH5 | .............<br>.............<br>........ | N......... | ....VP..<br>...... | ..H.....<br>........<br>.... | ..................<br>..............I | SSGWY-<br>..................<br>--.... | ......<br>..... |

| | | | | | | | | | |
|---|---|---|---|---|---|---|---|---|---|
| iPS:43 4481 | 21-225_74B10 | VH1\|1-08/D6\|6-19\|RF1/JH5 | ............<br>............<br>....F... | N......... | ........<br>...... | ..H.....<br>.....F..<br>.... | .................<br>..............V | SSGWY-<br>...................<br>--.... | ......<br>..... |
| iPS:43 4483 | 21-225_74C12 | VH1\|1-08/D6\|6-19\|RF1/JH5 | ............<br>............<br>........ | N......... | ........<br>...... | ........<br>........<br>.... | .................<br>..............I | SSGWY-<br>...................<br>--.... | ......<br>..... |
| iPS:43 4493 | 21-225_76F3 | VH1\|1-08/D6\|6-19\|RF1/JH5 | ............<br>............<br>........ | N......... | ........<br>...... | ........<br>........<br>.... | .........T...H...<br>..............V | SSGWN-<br>...................<br>--.... | ......<br>..... |
| iPS:43 4509 | 21-225_76F5 | VH1\|1-08/D6\|6-19\|RF1/JH5 | ............<br>............<br>........ | N......... | ........<br>...... | ........<br>........<br>.... | .................<br>..............S | SSGWY-<br>...................<br>--.... | ......<br>..... |
| iPS:43 4513 | 21-225_76A6 | VH1\|1-08/D6\|6-19\|RF1/JH5 | ............<br>............<br>........ | N......... | ........<br>...... | ..H.....<br>N.......<br>.... | .................<br>N.............I | SSGWY-<br>...................<br>--.... | ......<br>....L |
| iPS:43 4515 | 21-225_74A5 | VH1\|1-08/D6\|6-19\|RF1/JH5 | ............<br>............<br>........ | N......... | ....VP..<br>...... | ..H.....<br>........<br>.... | .................<br>..............I | SSGWY-<br>...................<br>--.... | ......<br>..... |
| iPS:43 4525 | 21-225_76E8 | VH1\|1-08/D6\|6-19\|RF1/JH5 | ............<br>............<br>.......P | N......... | ........<br>...... | ........<br>........<br>.... | .................<br>..............V | SSGWH-<br>...................<br>--.... | ......<br>...A. |
| iPS:43 4529 | 21-225_76B9 | VH1\|1-08/D6\|6-19\|RF1/JH5 | ............<br>............<br>........ | N......... | ....VP..<br>...... | ..H.....<br>........<br>.... | .................<br>..............I | SSGWY-<br>...................<br>--.... | ......<br>..... |
| iPS:43 4575 | 21-225_77C7 | VH1\|1-08/D6\|6-19\|RF1/JH5 | ............<br>............<br>.......P | N......... | ........<br>...... | ........<br>........<br>.... | .................<br>..............V | SSGWH-<br>...................<br>--.... | ......<br>...A. |
| iPS:43 4583 | 21-225_74B6 | VH1\|1-08/D6\|6-19\|RF1/JH5 | ............<br>............<br>........ | N......... | ........<br>...... | ..H.....<br>N.......<br>.... | .................<br>N.............I | SSGWY-<br>...................<br>--.... | ......<br>....L |
| iPS:43 4587 | 21-225_74G3 | VH1\|1-08/D6\|6-19\|RF1/JH5 | ............<br>............<br>........ | N......... | ....VP..<br>...... | ..H.....<br>........<br>.... | .................<br>..............I | SSGWY-<br>...................<br>--.... | ......<br>..... |
| iPS:43 | 21- | VH1\|1-08/D6\|6- | ............<br>............ | N......... | ........<br>...... | ..H.....<br>........ | .................<br>..............I | SSGWY-<br>................... | ......<br>..... |

| | | | | | | | | | |
|---|---|---|---|---|---|---|---|---|---|
| iPS:434603 | 21-225_77D11 | VH1\|1-08/D6\|6-19\|RF1/JH5 | ................................ | N......... | ....VP........ | ..H................. | ................................I | SSGWY-.....................--.... | ........... |
| iPS:434613 | 21-225_77D12 | VH1\|1-08/D6\|6-19\|RF1/JH5 | ................................ | N......... | .............. | .................... | ..........N.....................I | SSGWY-.....................--.... | ........... |
| iPS:434617 | 21-225_74B8 | VH1\|1-08/D6\|6-19\|RF1/JH5 | ................................ | N......... | .............. | ..H................. | ...L............................V | SSGWY-.....................--.... | ........... |
| iPS:434619 | 21-225_78C1 | VH1\|1-08/D6\|6-19\|RF1/JH5 | ................................ | N......... | .............. | ..H................. | ................................I | SSGWY-.....................--.... | ........... |
| iPS:434639 | 21-225_74B7 | VH1\|1-08/D6\|6-19\|RF1/JH5 | ...............................P | N......... | .............. | .................... | ................................V | SSGWH-.....................--.... | .........A. |
| iPS:434653 | 21-225_74B5 | VH1\|1-08/D6\|6-19\|RF1/JH5 | ................................ | N......... | .............. | .................... | .........T...H..................V | SSGWN-.....................--.... | ........... |
| iPS:434655 | 21-225_78H12 | VH1\|1-08/D6\|6-19\|RF1/JH5 | ...............................P | N......... | .............. | .................... | ................................V | SSGWH-.....................--.... | .........A. |
| iPS:434675 | 21-225_79G6 | VH1\|1-08/D6\|6-19\|RF1/JH5 | ............................F... | N......... | .............. | ..H...........F..... | ................................V | SSGWY-.....................--.... | ........... |
| iPS:434689 | 21-225_79G10 | VH1\|1-08/D6\|6-19\|RF1/JH5 | ................................ | N......... | .............. | .................... | .........T...H..................V | SSGWN-.....................--.... | ........... |
| iPS:434705 | 21-225_80A2 | VH1\|1-08/D6\|6-19\|RF1/JH5 | ................................ | N......... | ....VP........ | ..H................. | ................................I | SSGWY-.....................--.... | ........... |
| iPS:434707 | 21-225_80D3 | VH1\|1-08/D6\|6-19\|RF1/JH5 | ................................ | N......... | .............. | ..H................. | ................................I | SSGWY-.....................--.... | ........... |
| iPS:43 | 21-225_80E9 | VH1\|1-08/D6\|6- | ........................ | N......... | .............. | ..H............. | ................................I | SSGWY-..................... | ........... |

| | | | | | | | | | |
|---|---|---|---|---|---|---|---|---|---|
| iPS:43 4747 | 21-225_80C12 | VH1\|1-08/D6\|6-19\|RF1/JH5 | ............<br>............<br>........ | N......... | ........<br>...... | ..H.....<br>N.......<br>.... | .................<br>N............I | SSGWY-<br>...................<br>--.... | ......<br>....L |
| iPS:43 4761 | 21-225_81E5 | VH1\|1-08/D6\|6-19\|RF1/JH5 | ............<br>............<br>........ | N......... | ........<br>...... | ........<br>........<br>.... | .........T...H...<br>.............V | SSGWN-<br>...................<br>--.... | ......<br>..... |
| iPS:43 4771 | 21-225_81F9 | VH1\|1-08/D6\|6-19\|RF1/JH5 | ............<br>............<br>........ | N......... | ........<br>...... | ..H.....<br>........<br>.... | .................<br>.............I | SSGWY-<br>...................<br>--.... | ......<br>..... |
| iPS:43 4793 | 21-225_82A5 | VH1\|1-08/D6\|6-19\|RF1/JH5 | ............<br>............<br>........ | N......... | ........<br>...... | ..H.....<br>N.......<br>.... | .................<br>N............I | SSGWY-<br>...................<br>--.... | ......<br>....L |
| iPS:43 4797 | 21-225_82G5 | VH1\|1-08/D6\|6-19\|RF1/JH5 | ............<br>............<br>........ | N......... | ....VP..<br>...... | ..H.....<br>........<br>.... | .................<br>.............I | SSGWY-<br>...................<br>--.... | ......<br>..... |
| iPS:43 4805 | 21-225_82D9 | VH1\|1-08/D6\|6-19\|RF1/JH5 | ............<br>............<br>........ | N......... | ....VP..<br>...... | ..H.....<br>........<br>.... | .................<br>.............I | SSGWY-<br>...................<br>--.... | ......<br>..... |
| iPS:43 4813 | 21-225_82C12 | VH1\|1-08/D6\|6-19\|RF1/JH5 | ............<br>............<br>........ | N......... | ........<br>...... | ..H.....<br>N.......<br>.... | .................<br>N............I | SSGWY-<br>...................<br>--.... | ......<br>....L |
| iPS:43 4825 | 21-225_83C2 | VH1\|1-08/D6\|6-19\|RF1/JH5 | ............<br>............<br>........ | N......... | ....VP..<br>...... | ..H.....<br>........<br>.... | .................<br>.............I | SSGWY-<br>...................<br>--.... | ......<br>..... |
| iPS:43 4827 | 21-225_83F3 | VH1\|1-08/D6\|6-19\|RF1/JH5 | ............<br>............<br>........ | N......... | ........<br>...... | ..H.....<br>........<br>.... | .................<br>.............I | SSGWY-<br>...................<br>--.... | ......<br>..... |
| iPS:43 4829 | 21-225_83G3 | VH1\|1-08/D6\|6-19\|RF1/JH5 | ............<br>............<br>........ | N......... | ........<br>...... | ..H.....<br>........<br>.... | .................<br>.............I | SSGWY-<br>...................<br>--.... | ......<br>..... |
| iPS:43 4833 | 21-225_83C5 | VH1\|1-08/D6\|6-19\|RF1/JH5 | ............<br>............<br>........ | N......... | ....VP..<br>...... | ..H.....<br>........<br>.... | .................<br>.............I | SSGWY-<br>...................<br>--.... | ......<br>..... |
| iPS:43 | 21-225_83G7 | VH1\|1-08/D6\|6- | ............<br>............ | N......... | ........<br>...... | ........<br>........ | .................<br>.............V | SSGWH-<br>................... | ......<br>...A. |

| | | | | | | | | | |
|---|---|---|---|---|---|---|---|---|---|
| iPS:43 4863 | 21-225_84G7 | VH1\|1-08/D6\|6-19\|RF1/J H5 | ............ ............ .......P | N......... | ........ ...... | ........ ........ ...D | ................. ..............V | SSGWH- .................. --.... | ...... ...A. |
| iPS:43 4877 | 21-225_85H2 | VH1\|1-08/D6\|6-19\|RF1/J H5 | ............ ............ ........ | N......... | ........ ...... | ..H..... ........ .... | ...L............. ..............V | SSGWY- .................. --.... | ...... ..... |
| iPS:43 4883 | 21-225_85B5 | VH1\|1-08/D6\|6-19\|RF1/J H5 | ............ ............ ........ | N......... | ....VP.. ...... | ..H..... ........ .... | ................. ..............I | SSGWY- .................. --.... | ...... ..... |
| iPS:43 4911 | 21-225_85D11 | VH1\|1-08/D6\|6-19\|RF1/J H5 | ............ ............ ........ | N......... | ....VP.. ...... | ..H..... ........ .... | ................. ..............I | SSGWY- .................. --.... | ...... ..... |
| iPS:43 4935 | 21-225_86E9 | VH1\|1-08/D6\|6-19\|RF1/J H5 | ............ ............ ........ | N......... | ........ ...... | ........ ........ .... | ......S..T...H... ..............V | SSGWS- .................. --.... | ...... ..... |
| iPS:43 4957 | 21-225_87A10 | VH1\|1-08/D6\|6-19\|RF1/J H5 | ............ ............ ........ | N......... | ........ ...... | ..H..... N....... .... | ................. N.............I | SSGWY- .................. --.... | ...... ....L |
| iPS:43 4971 | 21-225_88G2 | VH1\|1-08/D6\|6-19\|RF1/J H5 | ............ ............ ........ | N......... | ........ ...... | ..H..... ........ .... | ................. ..............I | SSGWY- .................. --.... | ...... ..... |
| iPS:43 5051 | 21-225_90D9 | VH1\|1-08/D6\|6-19\|RF1/J H5 | ............ ............ .......P | N......... | ........ ...... | ........ ........ .... | ................. ..............V | SSGWH- .................. --.... | ...... ...A. |
| iPS:43 5071 | 21-225_91F1 | VH1\|1-08/D6\|6-19\|RF1/J H5 | ............ ............ ........ | N......... | ........ ...... | ..H..... ........ .... | ................. ..............I | SSGWY- .................. --.... | ...... ..... |
| iPS:43 5087 | 21-225_91G8 | VH1\|1-08/D6\|6-19\|RF1/J H5 | ............ ............ ........ | N......... | ........ ...... | ..H..... ........ .... | ................. ..............I | SSGWY- .................. --.... | ...... ..... |
| iPS:43 5203 | 21-225_75A7 | VH1\|1-08/D6\|6-19\|RF1/J H5 | ............ ............ .......P | N......... | ........ ...... | ........ ........ ...D | ................. ..............V | SSGWH- .................. --.... | ...... ...A. |
| iPS:43 | 21- | VH1\|1-08/D6\|6- | ............ ............ | N......... | ........ ...... | ........ ........ | .........T...H... ..............V | SSGWK- .................. | ...... ..... |

| | | | | | | | | | |
|---|---|---|---|---|---|---|---|---|---|
| iPS:43 5227 | 21-225_95G4 | VH1\|1-08/D6\|6-19\|RF1/JH5 | ............ ............ ........ | N......... | ........ ...... | ........ ........ .... | .........T...H... ..............V | SSGWN- ................... --.... | ...... ..... |
| iPS:43 5245 | 21-225_95E12 | VH1\|1-08/D6\|6-19\|RF1/JH5 | ............ ............ ........ | N......... | ........ ...... | ........ ........ .... | ................. ..............I | SSGWY- ................... --.... | ...... ..... |
| iPS:43 5247 | 21-225_96G1 | VH1\|1-08/D6\|6-19\|RF1/JH5 | ............ ............ ........ | N......... | ........ ...... | ..H..... N....... .... | ................. N.............I | SSGWY- ................... --.... | ...... ....L |
| iPS:43 5249 | 21-225_96E2 | VH1\|1-08/D6\|6-19\|RF1/JH5 | ............ ............ ........ | N......... | ........ ...... | ..H..... ........ .... | ...L............. ..............V | SSGWY- ................... --.... | ...... ..... |
| iPS:43 5255 | 21-225_96D5 | VH1\|1-08/D6\|6-19\|RF1/JH5 | ............ ............ ........ | N......... | ........ ...... | ........ ........ .... | ......S..T...H... ..............V | SSGWS- ................... --.... | ...... ..... |
| iPS:43 5279 | 21-225_97H4 | VH1\|1-08/D6\|6-19\|RF1/JH5 | ............ ............ ........ | N......... | ........ ...... | ..H..... ........ .... | ................. ..............I | SSGWY- ................... --.... | ...... ..... |
| iPS:43 5327 | 21-225_147G6 | VH1\|1-08/D6\|6-19\|RF1/JH5 | ............ ............ ........ | N......... | ........ ...... | ..H..... ........ .... | ................. ..............Y | SSGWY- ................... --.... | ...... ..... |
| iPS:43 5437 | 21-225_152F4 | VH1\|1-08/D6\|6-19\|RF1/JH5 | ............ ............ ........ | N......... | ........ ...... | ..H..... ........ .... | ................. .....D........Y | SSGWY- ................... --.... | ...... ..... |
| iPS:43 5701 | 21-225_170F6 | VH1\|1-08/D6\|6-19\|RF1/JH5 | ............ ............ ........ | N......... | ........ ...... | ........ ........ ...D | ......H.......... ..............I | SSGWY- ................... --.... | ...... ..... |
| iPS:43 5737 | 21-225_174G5 | VH1\|1-08/D6\|6-19\|RF1/JH5 | ............ ............ ........ | N......... | ........ ...... | ..H..... ........ .... | ................. ..............V | SSGWY- ................... --.... | ...... ..... |
| iPS:43 6544 | 21-225_224H5 | VH1\|1-08/D6\|6-19\|RF1/JH5 | ............ ............ ........ | N......... | ........ ...... | ........ ........ .... | ................. ..............S | SSGWN- ................... --.... | ...... ..... |
| iPS:43 | 21- | VH1\|1-08/D6\|6- | ............ ............ | N......... | ........ ...... | ..H..... ..S..... | .L.........V..... N.............S | SSGWY- ................... | ...... ..... |

| | | | | | | | | | |
|---|---|---|---|---|---|---|---|---|---|
| iPS:436644 | 21-225_227G9 | VH1\|1-08/D6\|6-19\|RF1/JH5 | .............<br>...........R<br>........ | N......... | .......R<br>...... | ..Y.....<br>........<br>.... | .................<br>..............L | SSGWY-<br>..................<br>--.... | ......<br>..... |
| iPS:437322 | 21-225_75B1 | VH1\|1-08/D6\|6-19\|RF1/JH5 | .............<br>............<br>........ | N......... | ....VP..<br>...... | ..H.....<br>........<br>.... | .................<br>..............I | SSGWY-<br>..................<br>--.... | ......<br>..... |
| iPS:437361 | 21-225_74C1 | VH1\|1-08/D6\|6-19\|RF1/JH5 | .............<br>............<br>........ | N......... | ........<br>...... | ....D...<br>.....F..<br>.... | .................<br>..............V | SSGWY-<br>..................<br>--.... | ......<br>..... |
| iPS:437363 | 21-225_74C10 | VH1\|1-08/D6\|6-19\|RF1/JH5 | .............<br>............<br>........ | N......... | ........<br>...... | ........<br>...I....<br>.... | .................<br>..............I | SSGWY-<br>..................<br>--.... | ......<br>..... |
| iPS:437379 | 21-225_74H2 | VH1\|1-08/D6\|6-19\|RF1/JH5 | .............<br>............<br>....F... | N......... | ........<br>...... | ..H.....<br>.....F..<br>.... | .................<br>..............V | SSGWY-<br>..................<br>--.... | ......<br>..... |
| iPS:446094 | 21-225_77E1 | VH1\|1-08/D6\|6-19\|RF1/JH5 | .............<br>............<br>.......P | N......... | ........<br>...... | ........<br>........<br>.... | .................<br>..............V | SSGWH-<br>..................<br>--.... | ......<br>...A. |
| iPS:451129 | 21-225_94D2 | VH1\|1-08/D6\|6-19\|RF1/JH5 | .............<br>............<br>....F... | N......... | ........<br>...... | ..H.....<br>.....F..<br>.... | .................<br>..............V | SSGWY-<br>..................<br>--.... | ......<br>..... |
| iPS:451133 | 21-225_95H4 | VH1\|1-08/D6\|6-19\|RF1/JH5 | .............<br>............<br>........ | N......... | ........<br>...... | ........<br>........<br>.... | .........T...H...<br>..............V | SSGWN-<br>..................<br>--.... | ......<br>..... |
| iPS:398510 | 21-225_25A3 | VH1\|1-08/D6\|6-19\|RF1/JH5 | .............<br>............<br>.....I.. | N......... | ........<br>...... | ..H.....<br>........<br>.... | .....W.......N...<br>..............S | SSGWY-<br>..................<br>--.... | ......<br>..... |
| iPS:398516 | 21-225_26A9 | VH1\|1-08/D6\|6-19\|RF1/JH5 | .............<br>............<br>........ | N......... | ........<br>...... | ..H.....<br>.....C..<br>.... | .....W.M.........<br>..............S | SSGWY-<br>..................<br>--.... | ......<br>..... |
| | | Germline | H_FR1 | H_CDR1 | H_FR2 | H_CDR2 | H_FR3 | H_CDR3 | H_FR4 |
| | VH3\|3-30.3/D5\|5-18\|RF3/JH4 | | QVQLVES-<br>GGGVVQPGRSLR<br>LSCAASG-FTFS | S-----YAMH | WVRQAPGK<br>GLEWVA | VISYD---<br>GSNKYYAD<br>SVKG | RFTISRDNSKNTLYLQM<br>NSLRAEDTAVYYCAR | GYSYG------------<br>--------YYFDY | WGQGTL<br>VTVSS |

| | | | | | | | | | |
|---|---|---|---|---|---|---|---|---|---|
| iPS:45 1139 | 21-225_71A6 | VH3\|3-30.3/D5\|5-18\|RF3/JH4 | ............ ............ ........ | N......G.. | ........ ...... | ........ ...E.... .... | ................ ............... | DHR..V............ ......RGG... | ...... ..... |
| | | Germline | H_FR1 | H_CDR1 | H_FR2 | H_CDR2 | H_FR3 | H_CDR3 | H_FR4 |
| | VH1\|1-18/D6\|6-19\|RF2/JH5 | | QVQLVQS-GAEVKKPGASVKVSCKASG-YTFT | S-----YGIS | WVRQAPGQGLEWMG | WISAY---NGNTNYAQKLQG | RVTMTTDTSTSTAYMELRSLRSDDTAVYYCAR | GIAVAG-------------------NWFDP | WGQGTLVTVSS |
| iPS:45 1143 | 21-225_66H11 | VH1\|1-18/D6\|6-19\|RF2/JH5 | ............ ............ .......A | T......... | ........ ...... | ........ ........ .... | ................ ............... | .E...- .................. .-V... | ...... ..... |
| iPS:43 4361 | 21-225_65D5 | VH1\|1-18/D6\|6-19\|RF2/JH5 | ............ ............ .......P | .......... | ........ ...... | ........ S....... .... | ................ ...........F... | .E...- .................. .-V... | ...... ..... |
| | | Germline | H_FR1 | H_CDR1 | H_FR2 | H_CDR2 | H_FR3 | H_CDR3 | H_FR4 |
| | VH3\|3-33/D4\|4-23\|RF2/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DYGGNSYY--------------YYYGMDV | WGQGTTVTVSS |
| iPS:45 3445 | 21-225_148E10 | VH3\|3-33/D4\|4-23\|RF2/JH6 | ............ ............ ........ | .......... | ........ ...... | ...F.... ......V. ...D | ................ ............... | .RVEG.GTP......... ....Y....... | ...... ..... |
| iPS:43 6082 | 21-225_195D9 | VH3\|3-33/D4\|4-23\|RF2/JH6 | ............ ............ ........ | H......V.. | ........ ...... | ........ .T...... .... | ................ ............... | .WF.EGN- ...............- ...... | ...... ..... |
| iPS:43 6118 | 21-225_196A10 | VH3\|3-33/D4\|4-23\|RF2/JH6 | ............ ............ ........ | H......V.. | ........ ...... | ........ .T...... .... | ................ ............... | .WF.EGN- ...............- ...... | ...... ..... |
| iPS:43 6670 | 21-225_147D9 | VH3\|3-33/D4\|4-23\|RF2/JH6 | ............ ............ ........ | .......... | ........ ...... | D..F.... ......V. ...D | ................ ............... | .RVEG.GTP......... ....Y....... | ...... ..... |
| iPS:43 6720 | 21-225_151H6 | VH3\|3-33/D4\|4-23\|RF2/JH6 | ............ ............ ........ | D......... | ........ ...... | L....... ........ .... | ................ ............... | .DRSC.RTSC........ ..PYY....L.. | ...... .I... |
| iPS:43 6726 | 21-225_152G5 | VH3\|3-33/D4\|4-23\|RF2/JH6 | ............ ............ ........ | D......... | ........ ...... | L....... ........ .... | ................ ............... | .DRSC.RTSC........ ..PYY....L.. | ...... .I... |

| | | | | | | | | | |
|---|---|---|---|---|---|---|---|---|---|
| iPS:43 6732 | 21-225_152B12 | VH3\|3-33/D4\|4-23\|RF2/JH6 | ...V........<br>............<br>........ | D......... | ........<br>...... | ........<br>........<br>.... | .................<br>............... | .DRSC.STSC........<br>..PYY....L.. | ......<br>..... |
| iPS:43 6734 | 21-225_153A8 | VH3\|3-33/D4\|4-23\|RF2/JH6 | ....M.......<br>............<br>........ | D......... | ........<br>...... | L.......<br>........<br>.... | .................<br>............... | .DRSC.RTSC........<br>..PYY....L.. | ......<br>.I... |
| iPS:43 6736 | 21-225_153E8 | VH3\|3-33/D4\|4-23\|RF2/JH6 | ............<br>............<br>......L. | N......... | ........<br>...... | ...F....<br>......V.<br>...D | .................<br>............... | .RVEG.GTP.........<br>....Y....... | ......<br>..... |
| iPS:43 6756 | 21-225_146A10 | VH3\|3-33/D4\|4-23\|RF2/JH6 | ....E.......<br>............<br>........ | G......... | ........<br>....MT | L.R.....<br>..D.N...<br>.... | .................<br>............... | .RVFC.STSCL.......<br>..SYY....... | ......<br>..... |
| iPS:43 6766 | 21-225_158D10 | VH3\|3-33/D4\|4-23\|RF2/JH6 | ............<br>............<br>........ | .......... | ........<br>...... | ........<br>........<br>.... | .................<br>............... | .RVSC.STSC........<br>..PYY....... | ......<br>..... |
| iPS:43 6768 | 21-225_159H8 | VH3\|3-33/D4\|4-23\|RF2/JH6 | ............<br>............<br>........ | T......... | ........<br>...... | ........<br>........<br>.... | .................<br>............... | .RVSC.STSC........<br>..PYY....... | ......<br>..... |
| iPS:43 6770 | 21-225_160B12 | VH3\|3-33/D4\|4-23\|RF2/JH6 | ............<br>............<br>........ | .......... | ........<br>...... | ........<br>........<br>.... | .................<br>............... | .RVSC.STSC........<br>..PYY....... | ......<br>..... |
| iPS:43 6782 | 21-225_166G11 | VH3\|3-33/D4\|4-23\|RF2/JH6 | ............<br>............<br>.......N | G......... | ........<br>...... | ........<br>........<br>.... | .......T.....F...<br>...T........... | .DRYC.SPTCH.......<br>..PYY....L.. | ......<br>..... |
| iPS:43 6794 | 21-225_170F1 | VH3\|3-33/D4\|4-23\|RF2/JH6 | ............<br>............<br>........ | G........N | ........<br>...... | I.......<br>.N......<br>.... | .................<br>............... | .RVYC.STSCH.......<br>..PYY...A... | ......<br>..... |
| iPS:43 6836 | 21-225_52H1 | VH3\|3-33/D4\|4-23\|RF2/JH6 | ............<br>............<br>........ | G......... | ........<br>...... | ........<br>........<br>.... | .................<br>............... | .RVYC.SSSCS.......<br>..YYY....... | ......<br>..... |
| iPS:43 6922 | 21-225_78E9 | VH3\|3-33/D4\|4-23\|RF2/JH6 | ............<br>............<br>........ | .......... | ........<br>...... | ........<br>.N..S...<br>.... | .......I.........<br>............... | .RDYC.STSC........<br>..PYY....... | ......<br>..... |
| iPS:43 | 21-225_74B3 | VH3\|3-33/D4\|4- | ............<br>............ | R......... | ........<br>...... | .F......<br>....D... | .................<br>............... | .RDYC.STSC........<br>..PYY....... | ......<br>..... |

| | | | | | | | | | |
|---|---|---|---|---|---|---|---|---|---|
| iPS:43 6928 | 21-225_79E7 | VH3\|3-33/D4\|4-23\|RF2/JH6 | .............<br>.............<br>........ | .......... | ........<br>...... | ........<br>.N..S...<br>.... | .......I.........<br>............... | .RDYC.STSC........<br>..PYY....... | ......<br>..... |
| iPS:43 6932 | 21-225_92A4 | VH3\|3-33/D4\|4-23\|RF2/JH6 | .............<br>.............<br>........ | .......... | ........<br>...... | ........<br>.N..S...<br>.... | ....Y..I.........<br>............... | .RDYC.STSC........<br>..PYY....... | ......<br>..... |
| iPS:43 6936 | 21-225_97E6 | VH3\|3-33/D4\|4-23\|RF2/JH6 | .............<br>.............<br>........ | .......... | ........<br>...... | ........<br>.N..S...<br>.... | .......I.........<br>............... | .RDYC.STSC........<br>..PYY....... | ......<br>..... |
| iPS:43 7190 | 21-225_225A9 | VH3\|3-33/D4\|4-23\|RF2/JH6 | .............<br>....G........<br>........ | T......... | ...L....<br>...... | ........<br>........<br>.... | .......I.Q.......<br>............... | .NHYC.STSCS.......<br>..PYY..F.... | ......<br>..... |
| iPS:43 7254 | 21-225_149F2 | VH3\|3-33/D4\|4-23\|RF2/JH6 | ....G.A......<br>.............<br>........ | R......... | ........<br>...... | F.......<br>..EN....<br>.... | ......V..R.......<br>............... | .RVEG.GTP.........<br>....Y....... | ......<br>..... |
| iPS:43 7256 | 21-225_150F11 | VH3\|3-33/D4\|4-23\|RF2/JH6 | ....G.A......<br>.............<br>........ | R......... | ........<br>...... | F.......<br>..EN....<br>.... | ......V..R.......<br>............... | .RVEG.GTP.........<br>....Y....... | ......<br>..... |
| iPS:45 1110 | 21-225_74C9 | VH3\|3-33/D4\|4-23\|RF2/JH6 | .............<br>.............<br>........ | .......... | ........<br>...... | ........<br>.N..S...<br>.... | .......I.........<br>............... | .RDYC.STSC........<br>..PYY....... | ......<br>..... |
| iPS:39 2589 | 21-225_27H2 | VH3\|3-33/D4\|4-23\|RF2/JH6 | .............<br>.............<br>........ | G......... | ........<br>...... | ........<br>........<br>.... | .............F...<br>............... | .RVYC.STSCS.......<br>..PYY....... | ......<br>..... |
| iPS:39 3166 | 21-225_27G6 | VH3\|3-33/D4\|4-23\|RF2/JH6 | .............<br>.............<br>........ | G......... | ........<br>...... | I.......<br>..K..N..<br>.... | .................<br>............... | .RVYC.STSCS.......<br>..PYY....... | ......<br>..... |
| iPS:39 3198 | 21-225_28A11 | VH3\|3-33/D4\|4-23\|RF2/JH6 | .............<br>.............<br>........ | G.......L. | ........<br>...... | L.......<br>.N.T....<br>.... | .................<br>............... | .RVYC.STSCS.......<br>..PYY....... | ......<br>..... |
| iPS:39 3204 | 21-225_8C12 | VH3\|3-33/D4\|4-23\|RF2/JH6 | .............<br>.............<br>.......G | .......... | ........<br>...... | L.......<br>........<br>.... | .................<br>............... | .RVSC.SSSCY.......<br>..PYY....... | ......<br>..... |
| | | Germline | H_FR1 | H_CDR1 | H_FR2 | H_CDR2 | H_FR3 | H_CDR3 | H_FR4 |

| | | | | | | | | | |
|---|---|---|---|---|---|---|---|---|---|
| | VH1\|1-02/D4\|4-11\|RF2/JH4 | | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | DYSNY-------------------YFDY | WGQGTLVTVSS |
| iPS:453447 | 21-225_65F10 | VH1\|1-02/D4\|4-11\|RF2/JH4 | ............ ............ ........ | .......H.. | ........ ...... | ........ N...S... ...D | ..N............... ............... | .SRS- .................. ...SW.. | ...... ..... |
| iPS:434145 | 21-225_55B1 | VH1\|1-02/D4\|4-11\|RF2/JH4 | ............ ............ ........ | ........F. | ........ ...... | ..H..... NNA..... .... | ................. ..............K | .GRS- .................. ...S... | ...... ..... |
| iPS:434277 | 21-225_57A7 | VH1\|1-02/D4\|4-11\|RF2/JH4 | ............ ............ ........ | .......HI. | ........ D..... | ........ NN...... .... | ................. ............... | .GRS- .................. ...G... | ...... ..... |
| iPS:434389 | 21-225_66F11 | VH1\|1-02/D4\|4-11\|RF2/JH4 | ............ ............ ........ | .......H.. | ........ ...... | ........ N...H... ...D | W................ ............... | .SRS- .................. ...SW.. | ...... .S... |
| iPS:434423 | 21-225_70D1 | VH1\|1-02/D4\|4-11\|RF2/JH4 | ............ ............ ........ | .......H.. | ........ ...... | ........ .NA..... .... | ................. ............... | .SIS- .................. ...SW.. | ...... ..... |
| iPS:437234 | 21-225_64E2 | VH1\|1-02/D4\|4-11\|RF2/JH4 | ............ ............ ........ | .......... | ........ ...... | ........ NN...... .... | ................. ............... | .G.S- .................. ...G... | ...... ..... |
| | | Germline | H_FR1 | H_CDR1 | H_FR2 | H_CDR2 | H_FR3 | H_CDR3 | H_FR4 |
| | VH4\|4-59/D7\|7-27\|RF1/JH4 | | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAR | LTGY-------------------FDY | WGQGTLVTVSS |
| iPS:453449 | 21-225_208A2 | VH4\|4-59/D7\|7-27\|RF1/JH4 | ............ .........N.. .......R | .......... | .....A.. ...... | R..T.... ....D... .... | .I.M.............. ............... | GF.D.............. .........W.. | ...... ..... |
| iPS:435451 | 21-225_152D10 | VH4\|4-59/D7\|7-27\|RF1/JH4 | ............ ............ ........ | N......... | .....A.. ...... | R.DT.... ..I..... .... | ...M.............. T.............. | EG.LGA............ .......TF... | ...... ..... |
| iPS:435467 | 21-225_153B9 | VH4\|4-59/D7\|7-27\|RF1/JH4 | ............ ............ ........ | .......... | .....A.. ...... | R.DT.... ..I..... .... | ...M.............. T.............. | EG.VGA............ .......TY... | ...... ..... |
| iPS:43 | 21- | VH4\|4-59/D7\|7- | ............ ............ | ......HF.. | .....A.. ...... | R..T.... ..T...T. | ...M.............. ............... | .SSG.............. ........W... | ...... ..... |

| | | | | | | | | | |
|---|---|---|---|---|---|---|---|---|---|
| iPS:435665 | 21-225_169F2 | VH4\|4-59/D7\|7-27\|RF1/JH4 | ............<br>............<br>........ | .......... | .....A..<br>...... | R.DT....<br>..I.....<br>.... | ...M.I....S.I....<br>............... | EG.VGA...........<br>.......TY... | ......<br>..... |
| iPS:435671 | 21-225_169H5 | VH4\|4-59/D7\|7-27\|RF1/JH4 | ............<br>............<br>........ | .......... | .....A..<br>...... | R.DT....<br>..I.....<br>.... | ...M......S.I....<br>............... | EG.VGA...........<br>.......TY... | ......<br>..... |
| iPS:436354 | 21-225_210G10 | VH4\|4-59/D7\|7-27\|RF1/JH4 | ............<br>.........N..<br>.......R | .......... | .....A..<br>...... | R..T....<br>....D...<br>.... | .I.M............<br>............... | GF.D.............<br>.........W.. | ......<br>..... |
| | | Germline | H_FR1 | H_CDR1 | H_FR2 | H_CDR2 | H_FR3 | H_CDR3 | H_FR4 |
| | VH4\|4-34/D4\|4-17\|RF2/JH6 | | QVQLQQW-<br>GAGLLKPSETLS<br>LTCAVYG-GSFS | G-----YYWS | WIRQPPGK<br>GLEWIG | EINH----<br>SGSTNYNP<br>SLKS | RVTISVDTSKNQFSLKL<br>SSVTAADTAVYYCAR | DYGDYYY----------<br>-----YYYGMDV | WGQGTT<br>VTVSS |
| iPS:468810 | 21-225_74D5 | VH4\|4-34/D4\|4-17\|RF2/JH6 | ............<br>............<br>.N...P.. | ......C... | ........<br>...... | ...Y....<br>..R..F..<br>.... | ................<br>............... | ...G---<br>................--<br>--... | ......<br>..... |
| iPS:468832 | 21-225_76H10 | VH4\|4-34/D4\|4-17\|RF2/JH6 | ............<br>............<br>.N...P.. | ......C... | ........<br>...... | ...Y....<br>..R..F..<br>.... | ................<br>............... | ...G---<br>................--<br>--... | ......<br>..... |
| iPS:468834 | 21-225_94G10 | VH4\|4-34/D4\|4-17\|RF2/JH6 | ............<br>............<br>.N...P.. | ......C... | G.......<br>.R.... | ...Y....<br>..R..F..<br>.... | ................<br>............... | ...G---<br>................--<br>--... | ......<br>..... |
| iPS:468838 | 21-225_80E12 | VH4\|4-34/D4\|4-17\|RF2/JH6 | ............<br>............<br>.N...P.. | ......C... | ........<br>...... | ...Y....<br>..R..F..<br>.... | ................<br>............... | ...G---<br>................--<br>--... | ......<br>..... |
| iPS:468820 | 21-225_76E10 | VH4\|4-34/D4\|4-17\|RF2/JH6 | ............<br>............<br>.N...P.. | ......S... | ........<br>...... | ...Y....<br>..R..F..<br>.... | ................<br>............... | ...G---<br>................--<br>--... | ......<br>..... |
| iPS:434473 | 21-225_76D1 | VH4\|4-34/D4\|4-17\|RF2/JH6 | ............<br>............<br>..S..... | ......C... | ........<br>...... | ........<br>..R.....<br>.... | ................<br>............... | ...G---<br>................--<br>--... | ......<br>..... |
| iPS:434495 | 21-225_74B2 | VH4\|4-34/D4\|4-17\|RF2/JH6 | ............<br>.....P......<br>........ | ......P... | ........<br>...... | ........<br>........<br>..T. | ................<br>T......S....... | ...G---<br>................--<br>--L.. | ......<br>..... |

| | | | | | | | | | |
|---|---|---|---|---|---|---|---|---|---|
| iPS:43 4497 | 21-225_76A4 | VH4\|4-34/D4\|4-17\|RF2/JH6 | .............<br>.............<br>.H...... | ......C... | ........<br>...... | ........<br>........<br>.... | .................<br>.............. | ...G---<br>................--<br>--... | ......<br>..... |
| iPS:43 4501 | 21-225_76G4 | VH4\|4-34/D4\|4-17\|RF2/JH6 | .............<br>.............<br>.H...... | ......C... | ........<br>...... | ........<br>........<br>.... | .................<br>.............. | ...G---<br>................--<br>--... | ......<br>..... |
| iPS:43 4507 | 21-225_74C5 | VH4\|4-34/D4\|4-17\|RF2/JH6 | .............<br>.............<br>........ | ......C... | ........<br>...... | ........<br>..C..F..<br>.... | .................<br>.............. | ...G---<br>................--<br>--... | ......<br>..... |
| iPS:43 4523 | 21-225_75C3 | VH4\|4-34/D4\|4-17\|RF2/JH6 | ......G....P<br>.............<br>........ | ......C... | ........<br>...... | ...Y....<br>..R.....<br>.... | .................<br>.............. | ...G---<br>................--<br>--... | ......<br>..... |
| iPS:43 4533 | 21-225_85F7 | VH4\|4-34/D4\|4-17\|RF2/JH6 | .............<br>.....P......<br>........ | ......P... | ........<br>...... | ........<br>........<br>.... | .................<br>T............. | ...G---<br>................--<br>--L.. | ......<br>..... |
| iPS:43 4547 | 21-225_74H5 | VH4\|4-34/D4\|4-17\|RF2/JH6 | .............<br>.............<br>.N...P.. | ......C... | ........<br>...... | ........<br>..R..F..<br>.... | .................<br>.............. | ...G---<br>................--<br>--... | ......<br>..... |
| iPS:43 4559 | 21-225_74D11 | VH4\|4-34/D4\|4-17\|RF2/JH6 | .............<br>.............<br>.H...... | ......C... | ........<br>...... | ........<br>........<br>.... | .................<br>.............. | ...G---<br>................--<br>--... | ......<br>..... |
| iPS:43 4561 | 21-225_77G1 | VH4\|4-34/D4\|4-17\|RF2/JH6 | .............<br>.............<br>.H...... | ......C... | ........<br>...... | ........<br>........<br>.... | .................<br>.............. | ...G---<br>................--<br>--... | ......<br>..... |
| iPS:43 4565 | 21-225_75B10 | VH4\|4-34/D4\|4-17\|RF2/JH6 | .............<br>.........K.D<br>........ | .......... | ........<br>...... | ........<br>........<br>.... | .................<br>.............. | ...G---<br>................--<br>--L.. | ......<br>..... |
| iPS:43 4579 | 21-225_77F7 | VH4\|4-34/D4\|4-17\|RF2/JH6 | .............<br>.............<br>.H...... | ......C... | ........<br>...... | ........<br>........<br>.... | .................<br>.............. | ...G---<br>................--<br>--... | ......<br>..... |
| iPS:43 4581 | 21-225_74B12 | VH4\|4-34/D4\|4-17\|RF2/JH6 | .............<br>.............<br>.H...... | ......C... | ........<br>...... | ........<br>........<br>.... | .................<br>.............. | ...G---<br>................--<br>--... | ......<br>..... |
| iPS:43 | 21- | VH4\|4-34/D4\|4- | ......G....P<br>............. | ......C... | ........<br>...... | ...Y....<br>..R..... | .................<br>.............. | ...G---<br>................-- | ......<br>..... |

| | | | | | | | | | |
|---|---|---|---|---|---|---|---|---|---|
| iPS:43 4595 | 21-225_77A10 | VH4\|4-34/D4\|4-17\|RF2/JH6 | ......G....P<br>............<br>........ | ......C... | ........<br>...... | ...Y....<br>..R.....<br>.... | .................<br>............... | ...G---<br>................--<br>--... | ......<br>..... |
| iPS:43 4611 | 21-225_77C12 | VH4\|4-34/D4\|4-17\|RF2/JH6 | ............<br>............<br>.....A.. | ......S... | ....S...<br>...... | ...Y....<br>R.......<br>.... | ...............N.<br>............... | ...G---<br>................--<br>--... | ......<br>..... |
| iPS:43 4657 | 21-225_79G1 | VH4\|4-34/D4\|4-17\|RF2/JH6 | ............<br>............<br>.H...... | ......C... | ........<br>...... | ........<br>........<br>.... | .................<br>............... | ...G---<br>................--<br>--... | ......<br>..... |
| iPS:43 4663 | 21-225_79F3 | VH4\|4-34/D4\|4-17\|RF2/JH6 | ............<br>............<br>.H...... | ......C... | ........<br>...... | ........<br>........<br>.... | .................<br>............... | ...G---<br>................--<br>--... | ......<br>..... |
| iPS:43 4687 | 21-225_75A5 | VH4\|4-34/D4\|4-17\|RF2/JH6 | ............<br>............<br>.H...... | ......C... | ........<br>...... | ........<br>........<br>.... | .................<br>............... | ...G---<br>................--<br>--... | ......<br>..... |
| iPS:43 4691 | 21-225_75G7 | VH4\|4-34/D4\|4-17\|RF2/JH6 | ............<br>...........T<br>.....A.. | ......S... | ....S...<br>...... | ...Y....<br>R.......<br>.... | ...............N.<br>............... | ...G---<br>................--<br>--... | ......<br>..... |
| iPS:43 4693 | 21-225_79F11 | VH4\|4-34/D4\|4-17\|RF2/JH6 | ............<br>............<br>.H...... | ......C... | ........<br>...... | ........<br>........<br>.... | .................<br>............... | ...G---<br>................--<br>--... | ......<br>..... |
| iPS:43 4699 | 21-225_79G12 | VH4\|4-34/D4\|4-17\|RF2/JH6 | ............<br>............<br>.H...... | ......C... | ........<br>...... | ........<br>........<br>.... | .................<br>............... | ...G---<br>................--<br>--... | ......<br>..... |
| iPS:43 4701 | 21-225_80A1 | VH4\|4-34/D4\|4-17\|RF2/JH6 | ............<br>............<br>.H...... | ......C... | ........<br>...... | ........<br>........<br>.... | .................<br>............... | ...G---<br>................--<br>--... | ......<br>..... |
| iPS:43 4703 | 21-225_80C1 | VH4\|4-34/D4\|4-17\|RF2/JH6 | ............<br>............<br>.H...... | ......C... | ........<br>...... | ........<br>........<br>.... | .................<br>............... | ...G---<br>................--<br>--... | ......<br>..... |
| iPS:43 4709 | 21-225_80E3 | VH4\|4-34/D4\|4-17\|RF2/JH6 | ............<br>............<br>.H...... | ......C... | ........<br>...... | ........<br>........<br>.... | .................<br>............... | ...G---<br>................--<br>--... | ......<br>..... |
| iPS:43 | 21-225_80D5 | VH4\|4-34/D4\|4- | ............<br>.....P...... | ......P... | ........<br>...... | ........<br>........ | .................<br>T.....M...... | ...G---<br>................-- | ......<br>..... |

| | | | | | | | | | |
|---|---|---|---|---|---|---|---|---|---|
| iPS:43 4725 | 21-225_80H7 | VH4\|4-34/D4\|4-17\|RF2/JH6 | .............<br>.............<br>..V..... | ......S... | .........<br>...... | ...Q....<br>..R.....<br>.... | .................<br>............... | ...G---<br>.................--<br>--I.. | ......<br>..... |
| iPS:43 4743 | 21-225_74A4 | VH4\|4-34/D4\|4-17\|RF2/JH6 | .............<br>.............<br>.H...... | ......C... | .........<br>...... | ........<br>........<br>.... | .................<br>............... | ...G---<br>.................--<br>--... | ......<br>..... |
| iPS:43 4751 | 21-225_80H12 | VH4\|4-34/D4\|4-17\|RF2/JH6 | .............<br>.............<br>.H...... | ......C... | .........<br>...... | ........<br>........<br>.... | .................<br>............... | ...G---<br>.................--<br>--... | ......<br>..... |
| iPS:43 4759 | 21-225_81C5 | VH4\|4-34/D4\|4-17\|RF2/JH6 | .............<br>.............<br>.H...... | ......C... | .........<br>...... | ........<br>........<br>.... | .................<br>............... | ...G---<br>.................--<br>--... | ......<br>..... |
| iPS:43 4773 | 21-225_75D9 | VH4\|4-34/D4\|4-17\|RF2/JH6 | .............<br>.............<br>........ | ......P... | .........<br>...... | ...Y....<br>R.......<br>.... | ...............N.<br>............... | ...G---<br>.................--<br>--... | ......<br>..... |
| iPS:43 4777 | 21-225_81C11 | VH4\|4-34/D4\|4-17\|RF2/JH6 | .............<br>.............<br>.H...... | ......C... | .........<br>...... | ........<br>........<br>.... | .................<br>............... | ...G---<br>.................--<br>--... | ......<br>..... |
| iPS:43 4809 | 21-225_74F5 | VH4\|4-34/D4\|4-17\|RF2/JH6 | .............<br>.............<br>.H...... | ......C... | .........<br>...... | ........<br>........<br>.... | .................<br>............... | ...G---<br>.................--<br>--... | ......<br>..... |
| iPS:43 4821 | 21-225_83G1 | VH4\|4-34/D4\|4-17\|RF2/JH6 | .............<br>.............<br>.H...... | ......C... | .........<br>...... | ........<br>........<br>.... | .................<br>............... | ...G---<br>.................--<br>--... | ......<br>..... |
| iPS:43 4839 | 21-225_83B7 | VH4\|4-34/D4\|4-17\|RF2/JH6 | .............<br>.............<br>.H...... | ......C... | .........<br>...... | ........<br>........<br>.... | .................<br>............... | ...G---<br>.................--<br>--... | ......<br>..... |
| iPS:43 4869 | 21-225_84E12 | VH4\|4-34/D4\|4-17\|RF2/JH6 | .............<br>.............<br>........ | ......S... | .........<br>...... | ...Q....<br>..R.....<br>.... | .................<br>............... | ...G---<br>.................--<br>--I.. | ......<br>..... |
| iPS:43 4879 | 21-225_85A3 | VH4\|4-34/D4\|4-17\|RF2/JH6 | .............<br>.............<br>.H...... | ......C... | .........<br>...... | ........<br>........<br>.... | .................<br>............... | ...G---<br>.................--<br>--... | ......<br>..... |
| iPS:43 | 21-225_85B4 | VH4\|4-34/D4\|4- | .............<br>............. | ......C... | .........<br>...... | ........<br>........ | .................<br>............... | ...G---<br>.................-- | ......<br>..... |

| | | | | | | | | | |
|---|---|---|---|---|---|---|---|---|---|
| iPS:43 4887 | 21-225_85D6 | VH4\|4-34/D4\|4-17\|RF2/JH6 | ......G....P<br>............<br>........ | ......C... | ........<br>...... | ...Y....<br>..R.....<br>.... | .................<br>............... | ...G---<br>................--<br>--... | ......<br>..... |
| iPS:43 4895 | 21-225_74H7 | VH4\|4-34/D4\|4-17\|RF2/JH6 | ............<br>.....P......<br>........ | ......P... | ........<br>...... | ........<br>........<br>.... | .................<br>T.............. | ...G---<br>................--<br>--L.. | ......<br>..... |
| iPS:43 4899 | 21-225_85B9 | VH4\|4-34/D4\|4-17\|RF2/JH6 | ............<br>............<br>.N...P.. | ......C... | ........<br>...... | ........<br>..R..F..<br>.... | .................<br>............... | ...G---<br>................--<br>--... | ......<br>..... |
| iPS:43 4907 | 21-225_85G10 | VH4\|4-34/D4\|4-17\|RF2/JH6 | ......R.....<br>............<br>........ | ......C... | ........<br>...... | ........<br>..I.....<br>.... | .................<br>T.............. | ...G---<br>................--<br>--L.. | ......<br>..... |
| iPS:43 4913 | 21-225_86C1 | VH4\|4-34/D4\|4-17\|RF2/JH6 | ............<br>............<br>.H...... | ......C... | ........<br>...... | ........<br>........<br>.... | .................<br>............... | ...G---<br>................--<br>--... | ......<br>..... |
| iPS:43 4921 | 21-225_86E4 | VH4\|4-34/D4\|4-17\|RF2/JH6 | ............<br>............<br>.H...... | ......C... | ........<br>...... | ........<br>........<br>.... | .................<br>............... | ...G---<br>................--<br>--... | ......<br>..... |
| iPS:43 4939 | 21-225_86C11 | VH4\|4-34/D4\|4-17\|RF2/JH6 | ............<br>............<br>.H...... | ......C... | ........<br>...... | ........<br>........<br>.... | .................<br>............... | ...G---<br>................--<br>--... | ......<br>..... |
| iPS:43 4943 | 21-225_87H1 | VH4\|4-34/D4\|4-17\|RF2/JH6 | .....P......<br>............<br>........ | .......... | ........<br>...... | ........<br>..R.....<br>.... | ..........D......<br>............... | ...G---<br>................--<br>--L.. | ......<br>..... |
| iPS:43 4945 | 21-225_87E5 | VH4\|4-34/D4\|4-17\|RF2/JH6 | ............<br>............<br>.H...... | ......C... | ........<br>...... | ........<br>........<br>.... | .................<br>............... | ...G---<br>................--<br>--... | ......<br>..... |
| iPS:43 4955 | 21-225_87C9 | VH4\|4-34/D4\|4-17\|RF2/JH6 | ............<br>............<br>.H...... | ......C... | ........<br>...... | ........<br>........<br>.... | .................<br>............... | ...G---<br>................--<br>--... | ......<br>..... |
| iPS:43 4961 | 21-225_87A12 | VH4\|4-34/D4\|4-17\|RF2/JH6 | ............<br>............<br>.H...... | ......C... | ........<br>...... | ........<br>........<br>.... | .................<br>............... | ...G---<br>................--<br>--... | ......<br>..... |
| iPS:43 | 21-225_88H1 | VH4\|4-34/D4\|4- | ............<br>............ | ......C... | ........<br>...... | ........<br>........ | .................<br>............... | ...G---<br>................--<br> | ......<br>..... |

| | | | | | | | | | |
|---|---|---|---|---|---|---|---|---|---|
| iPS:43 4981 | 21-225_88E7 | VH4\|4-34/D4\|4-17\|RF2/JH6 | .............<br>.............<br>.H...... | ......C... | .........<br>...... | ........<br>........<br>.... | .................<br>............... | ...G---<br>................--<br>--... | ......<br>..... |
| iPS:43 4983 | 21-225_88F7 | VH4\|4-34/D4\|4-17\|RF2/JH6 | .............<br>.............<br>.H...... | ......C... | .........<br>...... | ........<br>........<br>.... | .................<br>............... | ...G---<br>................--<br>--... | ......<br>..... |
| iPS:43 4995 | 21-225_88G9 | VH4\|4-34/D4\|4-17\|RF2/JH6 | .............<br>.............<br>.H...... | ......C... | .........<br>...... | ........<br>........<br>.... | .................<br>............... | ...G---<br>................--<br>--... | ......<br>..... |
| iPS:43 4999 | 21-225_75A8 | VH4\|4-34/D4\|4-17\|RF2/JH6 | .............<br>.............<br>.H...... | ......C... | .........<br>...... | ........<br>........<br>.... | .................<br>............... | ...G---<br>................--<br>--... | ......<br>..... |
| iPS:43 5013 | 21-225_89D5 | VH4\|4-34/D4\|4-17\|RF2/JH6 | .............<br>.............<br>.H...... | ......C... | .........<br>...... | ........<br>........<br>.... | .................<br>............... | ...G---<br>................--<br>--... | ......<br>..... |
| iPS:43 5015 | 21-225_89H5 | VH4\|4-34/D4\|4-17\|RF2/JH6 | .............<br>.............<br>........ | ......C... | .........<br>...... | ...Y....<br>.....F..<br>.... | .....A...........<br>............... | ...G---<br>................--<br>--... | ......<br>..... |
| iPS:43 5025 | 21-225_89E10 | VH4\|4-34/D4\|4-17\|RF2/JH6 | .............<br>.............<br>.H...... | ......C... | .........<br>...... | ........<br>........<br>.... | .................<br>............... | ...G---<br>................--<br>--... | ......<br>..... |
| iPS:43 5029 | 21-225_89A11 | VH4\|4-34/D4\|4-17\|RF2/JH6 | .....P.......<br>.............<br>........ | .......... | .........<br>...... | ........<br>..R.S...<br>.... | .................<br>............... | ...G---<br>................--<br>--L.. | ......<br>..... |
| iPS:43 5039 | 21-225_90G4 | VH4\|4-34/D4\|4-17\|RF2/JH6 | .............<br>.............<br>.H...... | ......C... | .........<br>...... | ........<br>........<br>.... | .................<br>............... | ...G---<br>................--<br>--... | ......<br>..... |
| iPS:43 5041 | 21-225_90A5 | VH4\|4-34/D4\|4-17\|RF2/JH6 | .............<br>.............<br>.H...... | ......C... | .........<br>...... | ........<br>........<br>.... | .................<br>............... | ...G---<br>................--<br>--... | ......<br>..... |
| iPS:43 5043 | 21-225_90G5 | VH4\|4-34/D4\|4-17\|RF2/JH6 | .............<br>.............<br>.H...... | ......C... | .........<br>...... | ........<br>........<br>.... | .................<br>............... | ...G---<br>................--<br>--... | ......<br>..... |
| iPS:43 | 21- | VH4\|4-34/D4\|4- | .............<br>............. | ......C... | .........<br>...... | ........<br>........ | .................<br>............... | ...G---<br>................-- | ......<br>..... |

| | | | | | | | | | |
|---|---|---|---|---|---|---|---|---|---|
| iPS:43 5073 | 21-225_91B2 | VH4\|4-34/D4\|4-17\|RF2/JH6 | ............<br>............<br>.H...... | ......C... | .........<br>...... | ........<br>........<br>.... | .................<br>............... | ...G---<br>................--<br>--... | ......<br>..... |
| iPS:43 5075 | 21-225_91B3 | VH4\|4-34/D4\|4-17\|RF2/JH6 | ............<br>............<br>.H...... | ......C... | .........<br>...... | ........<br>........<br>.... | .................<br>............... | ...G---<br>................--<br>--... | ......<br>..... |
| iPS:43 5077 | 21-225_91F3 | VH4\|4-34/D4\|4-17\|RF2/JH6 | ............<br>............<br>.H...... | ......C... | .........<br>...... | ........<br>........<br>.... | .................<br>............... | ...G---<br>................--<br>--... | ......<br>..... |
| iPS:43 5079 | 21-225_91B4 | VH4\|4-34/D4\|4-17\|RF2/JH6 | ............<br>............<br>.H...... | ......C... | .........<br>...... | ........<br>........<br>.... | .................<br>............... | ...G---<br>................--<br>--... | ......<br>..... |
| iPS:43 5089 | 21-225_91E9 | VH4\|4-34/D4\|4-17\|RF2/JH6 | ............<br>............<br>.H...... | ......C... | .........<br>...... | ........<br>........<br>.... | .................<br>............... | ...G---<br>................--<br>--... | ......<br>..... |
| iPS:43 5097 | 21-225_92B1 | VH4\|4-34/D4\|4-17\|RF2/JH6 | ............<br>............<br>........ | ......S... | .........<br>...... | ...Y....<br>R.......<br>.... | ..A...........N.<br>T.............. | ...G---<br>................--<br>--L.. | ......<br>..... |
| iPS:43 5111 | 21-225_92D6 | VH4\|4-34/D4\|4-17\|RF2/JH6 | ............<br>............<br>.H...... | ......C... | .........<br>...... | ........<br>........<br>.... | .................<br>............... | ...G---<br>................--<br>--... | ......<br>..... |
| iPS:43 5115 | 21-225_77C5 | VH4\|4-34/D4\|4-17\|RF2/JH6 | ............<br>............<br>.H...... | ......C... | .........<br>...... | ........<br>........<br>.... | .................<br>............... | ...G---<br>................--<br>--... | ......<br>..... |
| iPS:43 5171 | 21-225_93C2 | VH4\|4-34/D4\|4-17\|RF2/JH6 | ............<br>............<br>.H...... | ......C... | .........<br>...... | ........<br>........<br>.... | .................<br>............... | ...G---<br>................--<br>--... | ......<br>..... |
| iPS:43 5177 | 21-225_93E4 | VH4\|4-34/D4\|4-17\|RF2/JH6 | ............<br>............<br>.H...... | ......C... | .........<br>...... | ........<br>........<br>.... | .................<br>............... | ...G---<br>................--<br>--... | ......<br>..... |
| iPS:43 5195 | 21-225_94D3 | VH4\|4-34/D4\|4-17\|RF2/JH6 | ......G....P<br>............<br>........ | ......C... | .........<br>...... | ...Y....<br>..R.....<br>.... | .................<br>............... | ...G---<br>................--<br>--... | ......<br>..... |
| iPS:43 | 21- | VH4\|4-34/D4\|4- | ............<br>............ | ......C... | .........<br>...... | ........<br>........ | .................<br>............... | ...G---<br>................-- | ......<br>..... |

| | | | | | | | | | |
|---|---|---|---|---|---|---|---|---|---|
| iPS:43 5219 | 21-225_95D2 | VH4\|4-34/D4\|4-17\|RF2/JH6 | ............ ............ .H...... | ......C... | ........ ...... | ........ ........ .... | ................. ............... | ...G--- ................-- --... | ...... ..... |
| iPS:43 5235 | 21-225_95F9 | VH4\|4-34/D4\|4-17\|RF2/JH6 | ............ ............ .H...... | ......C... | ........ ...... | ........ ........ .... | ................. ............... | ...G--- ................-- --... | ...... ..... |
| iPS:43 5237 | 21-225_95G9 | VH4\|4-34/D4\|4-17\|RF2/JH6 | ............ ............ .H...... | ......C... | ........ ...... | ........ ........ .... | ................. ............... | ...G--- ................-- --... | ...... ..... |
| iPS:43 5239 | 21-225_95H10 | VH4\|4-34/D4\|4-17\|RF2/JH6 | ............ ............ .H...... | ......C... | ........ ...... | ........ ........ .... | ................. ............... | ...G--- ................-- --... | ...... ..... |
| iPS:43 5273 | 21-225_97A2 | VH4\|4-34/D4\|4-17\|RF2/JH6 | ............ ............ .H...... | ......C... | ........ ...... | ........ ........ .... | ................. ............... | ...G--- ................-- --... | ...... ..... |
| iPS:43 5281 | 21-225_97E5 | VH4\|4-34/D4\|4-17\|RF2/JH6 | ............ ............ .H...... | ......C... | ........ ...... | ........ ........ .... | ................. ............... | ...G--- ................-- --... | ...... ..... |
| iPS:43 7324 | 21-225_75C2 | VH4\|4-34/D4\|4-17\|RF2/JH6 | ............ ............ .H...... | ......C... | ........ ...... | ........ ........ .... | ................. ............... | ...G--- ................-- --... | ...... ..... |
| iPS:43 7328 | 21-225_75D3 | VH4\|4-34/D4\|4-17\|RF2/JH6 | ............ ............ .H...... | ......C... | ........ ...... | ........ ........ .... | ................. ............... | ...G--- ................-- --... | ...... ..... |
| iPS:43 7332 | 21-225_75F3 | VH4\|4-34/D4\|4-17\|RF2/JH6 | ............ ............ .H...... | ......C... | ........ ...... | ........ ........ .... | ................. ............... | ...G--- ................-- --... | ...... ..... |
| iPS:43 7344 | 21-225_75G12 | VH4\|4-34/D4\|4-17\|RF2/JH6 | ............ ............ .H...... | ......C... | ........ ...... | ........ ........ .... | ................. ............... | ...G--- ................-- --... | ...... ..... |
| iPS:43 7350 | 21-225_74A3 | VH4\|4-34/D4\|4-17\|RF2/JH6 | ............ ............ .H...... | ......C... | ........ ...... | ........ ........ .... | ................. ............... | ...G--- ................-- --... | ...... ..... |
| iPS:43 | 21-225_74D6 | VH4\|4-34/D4\|4- | ............ ............ | ......C... | ........ ...... | ........ ........ | ................. ............... | ...G--- ................-- | ...... ..... |

| | | Germline | H_FR1 | H_CDR1 | H_FR2 | H_CDR2 | H_FR3 | H_CDR3 | H_FR4 |
|---|---|---|---|---|---|---|---|---|---|
| | VH3\|3-33/D4\|4-17\|RF2/JH6 | | QVQLVES-<br>GGGVVQPGRSLR<br>LSCAASG-FTFS | S------YGMH | WVRQAPGK<br>GLEWVA | VIWYD---<br>GSNKYYAD<br>SVKG | RFTISRDNSKNTLYLQM<br>NSLRAEDTAVYYCAR | DYGDYYY----------<br>------YYYGMDV | WGQGTT<br>VTVSS |
| iPS:468814 | 21-225_223D11 | VH3\|3-33/D4\|4-17\|RF2/JH6 | ............<br>............<br>........ | N........D | ........<br>...... | ........<br>...D....<br>.... | .............F...<br>............... | .R.IG.-<br>................--<br>ND... | ......<br>..... |
| iPS:434621 | 21-225_74D1 | VH3\|3-33/D4\|4-17\|RF2/JH6 | ............<br>............<br>........ | .......... | ........<br>...... | ........<br>.....H..<br>.... | .................<br>.........M..... | .E.FGEFD..........<br>....Y.N..... | ......<br>..... |
| iPS:434947 | 21-225_87B7 | VH3\|3-33/D4\|4-17\|RF2/JH6 | ............<br>............<br>........ | .......... | ........<br>...... | ........<br>....N...<br>.... | .................<br>............... | .F.VG.-<br>................--<br>..... | ......<br>..... |
| iPS:435819 | 21-225_190C11 | VH3\|3-33/D4\|4-17\|RF2/JH6 | ............<br>............<br>........ | .......... | ........<br>...... | ........<br>....N...<br>.... | .................<br>............... | .Q.VG.-<br>................--<br>D.L.. | ......<br>..... |
| iPS:435825 | 21-225_190G11 | VH3\|3-33/D4\|4-17\|RF2/JH6 | ............<br>............<br>........ | I......... | ........<br>...... | ........<br>....N...<br>.... | .................<br>............... | .Q.VG.-<br>................--<br>D.L.. | ......<br>..... |
| iPS:435837 | 21-225_198G3 | VH3\|3-33/D4\|4-17\|RF2/JH6 | ............<br>.......K....<br>........ | T......... | ........<br>...... | ........<br>.T..N...<br>.... | .............C...<br>............... | .Q.VG.-<br>................--<br>D.L.. | ......<br>..... |
| iPS:435845 | 21-225_191G1 | VH3\|3-33/D4\|4-17\|RF2/JH6 | ............<br>............<br>........ | .......... | ........<br>...... | ........<br>...EH...<br>.... | .................<br>............... | .R.VG.-<br>................--<br>..L.. | ......<br>..... |
| iPS:435859 | 21-225_190E6 | VH3\|3-33/D4\|4-17\|RF2/JH6 | ............<br>............<br>........ | .......... | ........<br>...... | ........<br>....N...<br>.... | .................<br>............... | .Q.VG.-<br>................--<br>D.L.. | ......<br>..... |
| iPS:435873 | 21-225_190G4 | VH3\|3-33/D4\|4-17\|RF2/JH6 | ............<br>............<br>........ | .......... | ........<br>...... | ........<br>....N...<br>.... | .................<br>.........M..... | .Q.VG.-<br>................--<br>D.L.. | .....S<br>..... |
| iPS:435933 | 21-225_190F8 | VH3\|3-33/D4\|4-17\|RF2/JH6 | ............<br>............<br>........ | T......... | ........<br>...... | ........<br>....N...<br>.... | .................<br>............... | .Q.VG.-<br>................--<br>D.L.. | ......<br>..... |

| | | | | | | | | | |
|---|---|---|---|---|---|---|---|---|---|
| iPS:43 5941 | 21-225_191E8 | VH3\|3-33/D4\|4-17\|RF2/JH6 | ............<br>............<br>........ | N......... | ........<br>...... | I..F....<br>...Q....<br>.... | ................L<br>............... | AH.V..-<br>................--<br>.A... | ......<br>..... |
| iPS:43 5945 | 21-225_191A10 | VH3\|3-33/D4\|4-17\|RF2/JH6 | ............<br>............<br>........ | .......... | ........<br>...... | ........<br>....N...<br>.... | .................<br>.........M..... | .Q.VG.-<br>................--<br>D.L.. | .....S<br>..... |
| iPS:43 5947 | 21-225_191E10 | VH3\|3-33/D4\|4-17\|RF2/JH6 | ............<br>............<br>........ | .......... | ........<br>...... | ........<br>....N...<br>.... | .................<br>.........M..... | .Q.VG.-<br>................--<br>D.L.. | .....S<br>..... |
| iPS:43 5957 | 21-225_191G12 | VH3\|3-33/D4\|4-17\|RF2/JH6 | ............<br>............<br>........ | .......... | ........<br>...... | ........<br>....N...<br>.... | .................<br>............... | .Q.VG.-<br>................--<br>D.L.. | ......<br>..... |
| iPS:43 5963 | 21-225_192D2 | VH3\|3-33/D4\|4-17\|RF2/JH6 | ............<br>............<br>........ | .......... | ........<br>...... | ........<br>....N...<br>.... | ...........M.....<br>......E........ | .R.VG.-<br>................--<br>..... | ......<br>..... |
| iPS:43 5971 | 21-225_192D3 | VH3\|3-33/D4\|4-17\|RF2/JH6 | ............<br>............<br>........ | .......... | ........<br>...... | ........<br>...EH...<br>.... | .................<br>............... | .R.VG.-<br>................--<br>..L.. | ......<br>..... |
| iPS:43 5979 | 21-225_192H4 | VH3\|3-33/D4\|4-17\|RF2/JH6 | ............<br>............<br>........ | .......... | ........<br>...... | ........<br>....N...<br>.... | .................<br>............... | .Q.VG.-<br>................--<br>..... | ......<br>..... |
| iPS:43 5987 | 21-225_192G6 | VH3\|3-33/D4\|4-17\|RF2/JH6 | ............<br>............<br>........ | .......... | ........<br>...... | .L......<br>.T..N...<br>.... | .................<br>............... | .Q.VG.-<br>................--<br>D.L.. | ......<br>..... |
| iPS:43 5993 | 21-225_192C8 | VH3\|3-33/D4\|4-17\|RF2/JH6 | ............<br>............<br>........ | .......... | ........<br>...... | ........<br>...EH...<br>.... | ..M..............<br>............... | .R.VG.-<br>................--<br>..... | ......<br>..... |
| iPS:43 5997 | 21-225_192G8 | VH3\|3-33/D4\|4-17\|RF2/JH6 | ............<br>............<br>........ | .......... | ........<br>...... | ........<br>....N...<br>.... | .................<br>............... | .Q.VG.-<br>................--<br>D.L.. | ......<br>..... |
| iPS:43 6005 | 21-225_192H10 | VH3\|3-33/D4\|4-17\|RF2/JH6 | ............<br>............<br>........ | .......... | ........<br>...... | ........<br>....N...<br>.... | .................<br>.........M..... | .Q.VG.-<br>................--<br>D.L.. | .....S<br>..... |
| iPS:43 | 21- | VH3\|3-33/D4\|4- | ............<br>............ | .......... | ........<br>...... | ........<br>....N... | .................<br>.........M..... | .Q.VG.-<br>................-- | .....S<br>..... |

| | | | | | | | | | |
|---|---|---|---|---|---|---|---|---|---|
| iPS:43 6045 | 21-225_193A10 | VH3\|3-33/D4\|4-17\|RF2/JH6 | .............<br>.............<br>........ | .......... | ........<br>...... | ........<br>...EH...<br>.... | .................<br>............... | .R.VG.–<br>.................––<br>..L.. | ......<br>..... |
| iPS:43 6076 | 21-225_194H11 | VH3\|3-33/D4\|4-17\|RF2/JH6 | .............<br>.............<br>........ | .......... | ........<br>...... | ........<br>...EH...<br>.... | .............S...<br>............... | .R.VG.–<br>.................––<br>..L.. | ......<br>..... |
| iPS:43 6086 | 21-225_191G10 | VH3\|3-33/D4\|4-17\|RF2/JH6 | .............<br>.............<br>........ | .......... | ........<br>...... | ........<br>....N...<br>.... | .................<br>............... | .Q.VG.–<br>.................––<br>D.L.. | ......<br>..... |
| iPS:43 6090 | 21-225_195A9 | VH3\|3-33/D4\|4-17\|RF2/JH6 | .............<br>.............<br>........ | .......... | ........<br>..Q... | ........<br>...EH...<br>.... | .................<br>............... | .R.VG.–<br>.................––<br>..L.. | ......<br>..... |
| iPS:43 6112 | 21-225_196C7 | VH3\|3-33/D4\|4-17\|RF2/JH6 | .............<br>.............<br>........ | .......... | ........<br>...... | ........<br>...EH...<br>.... | .................<br>............... | .R.VG.–<br>.................––<br>..L.. | ......<br>..... |
| iPS:43 6138 | 21-225_197F2 | VH3\|3-33/D4\|4-17\|RF2/JH6 | .............<br>.............<br>........ | .......... | ........<br>...... | ........<br>....N...<br>.... | .................<br>............... | .Q.VG.–<br>.................––<br>D.L.. | ......<br>..... |
| iPS:43 6152 | 21-225_197B6 | VH3\|3-33/D4\|4-17\|RF2/JH6 | .............<br>.............<br>........ | .......... | ........<br>...... | ........<br>....N...<br>.... | ...........M.....<br>.........M..... | .Q.VG.–<br>.................––<br>D.L.. | .....S<br>..... |
| iPS:43 6173 | 21-225_197G12 | VH3\|3-33/D4\|4-17\|RF2/JH6 | .............<br>.............<br>........ | .......... | ........<br>...... | ........<br>....N...<br>.... | .................<br>............... | .Q.VG.–<br>.................––<br>D.L.. | ......<br>..... |
| iPS:43 6189 | 21-225_198B6 | VH3\|3-33/D4\|4-17\|RF2/JH6 | .............<br>.............<br>........ | .......... | ........<br>...... | ........<br>....H...<br>.... | .................<br>............... | .Q.VG.–<br>.................––<br>..... | ......<br>..... |
| iPS:43 6201 | 21-225_199C5 | VH3\|3-33/D4\|4-17\|RF2/JH6 | .............<br>.............<br>........ | .......... | ........<br>...... | ........<br>....N...<br>.... | .................<br>............... | .Q.VG.–<br>.................––<br>..... | ......<br>..... |
| iPS:43 6203 | 21-225_199A6 | VH3\|3-33/D4\|4-17\|RF2/JH6 | .............<br>.............<br>........ | N......... | ........<br>...... | I..F....<br>...Q....<br>.... | ................L<br>............... | AH.V..–<br>.................––<br>.A... | ......<br>..... |
| iPS:43 | 21- | VH3\|3-33/D4\|4- | .............<br>...........T | .......... | ........<br>...... | ........<br>....N... | .................<br>............... | .R.VG.–<br>.................–– | ......<br>..... |

| | | | | | | | | | |
|---|---|---|---|---|---|---|---|---|---|
| iPS:43 6296 | 21-225_205F5 | VH3\|3-33/D4\|4-17\|RF2/JH6 | ................................ | R......... | .............. | ...........EN.V..... | .......T..KM.F.......TD......... | .M.IG.-................--..... | ........... |
| iPS:43 6324 | 21-225_207G6 | VH3\|3-33/D4\|4-17\|RF2/JH6 | ................................ | .......... | .............. | ............N..E.... | ................................ | .A.IG.-................--..I.. | ........... |
| iPS:43 6364 | 21-225_211A11 | VH3\|3-33/D4\|4-17\|RF2/JH6 | .....G.......................... | .......... | .............. | .L.F.......RN....... | ................................ | .R.VG.-................--..T.. | ........... |
| iPS:43 6372 | 21-225_211A8 | VH3\|3-33/D4\|4-17\|RF2/JH6 | ................................ | .......... | .............. | ...........EH....... | ..........K..................... | .H.VG.-................--..... | ........... |
| iPS:43 6376 | 21-225_212E6 | VH3\|3-33/D4\|4-17\|RF2/JH6 | ................................ | .......... | .............. | ............N.V..... | ................................ | ...VG.-................--..T.. | ........... |
| iPS:43 6378 | 21-225_212D7 | VH3\|3-33/D4\|4-17\|RF2/JH6 | ................................ | .......... | .............. | ............N....... | .............S.................. | ...VG.-................--..T.. | ........... |
| iPS:43 6380 | 21-225_212H9 | VH3\|3-33/D4\|4-17\|RF2/JH6 | ................................ | .......... | .............. | ...........EH....... | ..........K..................... | .H.VG.-................--..... | ........... |
| iPS:43 6384 | 21-225_212F10 | VH3\|3-33/D4\|4-17\|RF2/JH6 | ................................ | R......... | .............. | ............H....... | .....................G.......... | .R.VG.-................--N.... | ........... |
| iPS:43 6390 | 21-225_213D2 | VH3\|3-33/D4\|4-17\|RF2/JH6 | ................................ | .......... | .............. | ............N....... | .............S.................. | ...VG.-................--..T.. | ........... |
| iPS:43 6394 | 21-225_213C4 | VH3\|3-33/D4\|4-17\|RF2/JH6 | ........................T....... | .......... | .............. | ............H....... | ................................ | ...VG.-................--D.... | ........... |
| iPS:43 6398 | 21-225_213B8 | VH3\|3-33/D4\|4-17\|RF2/JH6 | ................................ | .......... | .............. | ............H....... | ................................ | ...VG.-................--..T.. | ........... |
| iPS:43 | 21- | VH3\|3-33/D4\|4- | .......................E | .......... | .............. | ............N.G. | ................................ | .R.VG.-................-- | ........... |

| | | | | | | | | | |
|---|---|---|---|---|---|---|---|---|---|
| iPS:436410 | 21-225_212E10 | VH3\|3-33/D4\|4-17\|RF2/JH6 | ............<br>............<br>........ | .......... | ........<br>...... | ........<br>........<br>.... | .............S...<br>............... | ...VG.-<br>...............--<br>..T.. | ......<br>..... |
| iPS:436420 | 21-225_215B5 | VH3\|3-33/D4\|4-17\|RF2/JH6 | ............<br>............<br>........ | .......... | ........<br>...... | ........<br>....N...<br>.... | .............S...<br>............... | ...VG.-<br>...............--<br>..T.. | ......<br>..... |
| iPS:436422 | 21-225_215D6 | VH3\|3-33/D4\|4-17\|RF2/JH6 | ............<br>............<br>........ | .......... | ........<br>...... | ........<br>....N...<br>.... | .................<br>............... | .C.VG.-<br>...............--<br>..T.. | ......<br>..... |
| iPS:436430 | 21-225_215A12 | VH3\|3-33/D4\|4-17\|RF2/JH6 | ............<br>............<br>....L... | .......... | ........<br>...... | ........<br>...EH...<br>.... | .................<br>............... | .R.VG.-<br>...............--<br>..... | ......<br>..... |
| iPS:436452 | 21-225_217G5 | VH3\|3-33/D4\|4-17\|RF2/JH6 | ............<br>............<br>........ | ......N... | ........<br>...... | ........<br>....N...<br>.... | .................<br>............... | ...VG.-<br>...............--<br>..L.. | ......<br>..... |
| iPS:436464 | 21-225_219H1 | VH3\|3-33/D4\|4-17\|RF2/JH6 | ............<br>............<br>........ | R......... | ........<br>...... | ........<br>....H...<br>.... | .................<br>....G.......... | .R.VG.-<br>...............--<br>N.... | ......<br>..... |
| iPS:451120 | 21-225_197D3 | VH3\|3-33/D4\|4-17\|RF2/JH6 | ............<br>............<br>.....I.. | ......H... | ........<br>...... | ........<br>...EH...<br>.... | .................<br>............... | .Q.VG.-<br>...............--<br>..... | ......<br>..... |
| | | Germline | **H_FR1** | **H_CDR1** | **H_FR2** | **H_CDR2** | **H_FR3** | **H_CDR3** | **H_FR4** |
| | VH3\|3-33/D4\|4-17\|RF2/JH4 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | DYGDY-------------<br>---------YFDY | WGQGTL<br>VTVSS |
| iPS:468822 | 21-225_147E10 | VH3\|3-33/D4\|4-17\|RF2/JH4 | ............<br>....K.......<br>........ | N.......L. | ........<br>...... | I.......<br>........<br>.... | .................<br>............... | .HY.FW...........<br>......SGH... | ......<br>..... |
| iPS:433965 | 21-225_46F2 | VH3\|3-33/D4\|4-17\|RF2/JH4 | ............<br>............<br>........ | .......... | ........<br>...... | I.......<br>......V.<br>.... | .................<br>............... | .RY.FW...........<br>......SG.... | ......<br>..... |
| iPS:434255 | 21-225_62E6 | VH3\|3-33/D4\|4-17\|RF2/JH4 | ............<br>............<br>........ | .......... | ........<br>...... | A.......<br>......G.<br>.... | .V........S.H...<br>............... | .Q.IVG...........<br>......ATW... | ......<br>..... |

| | | | | | | | | | |
|---|---|---|---|---|---|---|---|---|---|
| iPS:43 4269 | 21-225_57H3 | VH3\|3-33/D4\|4-17\|RF2/JH4 | .............<br>.............<br>........ | ........... | ........<br>...... | A.......<br>........<br>.... | .................<br>............... | ...IVG...........<br>......AT.... | ......<br>..... |
| iPS:43 4345 | 21-225_64H9 | VH3\|3-33/D4\|4-17\|RF2/JH4 | .............<br>.............<br>........ | T......... | ........<br>...... | I.......<br>........<br>.... | .................<br>............... | .TY.FW...........<br>......SG.LG. | ......<br>..... |
| iPS:43 4363 | 21-225_65A6 | VH3\|3-33/D4\|4-17\|RF2/JH4 | .............<br>.............<br>........ | N......... | ........<br>...... | ........<br>......G.<br>.... | .................<br>............... | .Q.IVG..........<br>......ATW... | ......<br>..... |
| iPS:43 4393 | 21-225_67C3 | VH3\|3-33/D4\|4-17\|RF2/JH4 | .............<br>.............<br>........ | ........... | ........<br>...... | A.......<br>......G.<br>.... | .V........S.H...<br>............... | .Q.IVG..........<br>......ATW... | ......<br>..... |
| iPS:43 4425 | 21-225_70A5 | VH3\|3-33/D4\|4-17\|RF2/JH4 | .............<br>.............<br>........ | ........... | ........<br>...... | ........<br>........<br>.... | ...........S.....<br>...S........... | .Q.IVG..........<br>......ATW... | ......<br>..... |
| iPS:43 5341 | 21-225_148B2 | VH3\|3-33/D4\|4-17\|RF2/JH4 | .............<br>.............<br>........ | ........... | ........<br>...... | I.......<br>..Y.....<br>.... | .................<br>...S........... | .HF.FW...........<br>......SGH... | ......<br>..... |
| iPS:43 5357 | 21-225_148G10 | VH3\|3-33/D4\|4-17\|RF2/JH4 | .............<br>.............<br>........ | ........... | ........<br>...... | I.......<br>........<br>.... | .................<br>............... | .RY.FW...........<br>......SGH... | ......<br>..... |
| iPS:43 5365 | 21-225_149F1 | VH3\|3-33/D4\|4-17\|RF2/JH4 | .............<br>.............<br>........ | ........... | ........<br>...... | I.......<br>..Y.....<br>.... | .................<br>...S........... | .HF.FW...........<br>......SGH... | ......<br>..... |
| iPS:43 5413 | 21-225_150B11 | VH3\|3-33/D4\|4-17\|RF2/JH4 | .............<br>.............<br>........ | ........... | ........<br>...... | I.......<br>........<br>.... | .................<br>............... | .RY.FW...........<br>......SGH... | ......<br>..... |
| iPS:43 5423 | 21-225_151G5 | VH3\|3-33/D4\|4-17\|RF2/JH4 | ....M.......<br>.............<br>.....I.. | ........... | ........<br>...... | I.......<br>........<br>.... | .................<br>............... | .RY.FW...........<br>......SGH... | ......<br>..... |
| iPS:43 5429 | 21-225_151A10 | VH3\|3-33/D4\|4-17\|RF2/JH4 | .............<br>.............<br>........ | N......... | ........<br>....L. | I.......<br>........<br>.... | .................<br>............... | .HY.FW...........<br>......SGH... | ......<br>..... |
| iPS:43 | 21- | VH3\|3-33/D4\|4- | ..........D.<br>............. | ........... | ........<br>...... | I.......<br>..S..... | ...............H.<br>............... | .RY.FW...........<br>......SGH... | ......<br>..... |

| | | | | | | | | | |
|---|---|---|---|---|---|---|---|---|---|
| iPS:435683 | 21-225_170A1 | VH3\|3-33/D4\|4-17\|RF2/JH4 | .............<br>.............<br>........ | .......... | ........<br>...... | I.......<br>..Y.....<br>.... | .................<br>............... | .AH.FW............<br>......SG...S | ......<br>..... |
| iPS:435755 | 21-225_176H4 | VH3\|3-33/D4\|4-17\|RF2/JH4 | .............<br>.............<br>........ | .......... | ........<br>...... | I.......<br>..Y.....<br>.... | .................<br>S.............. | .AH.FW............<br>......SG..A. | ....A.<br>..... |
| iPS:435795 | 21-225_181C2 | VH3\|3-33/D4\|4-17\|RF2/JH4 | .............<br>............V<br>........ | .......... | ........<br>.....T | I.......<br>..Y.....<br>.... | .................<br>............... | .HY.FW............<br>......SGH..F | ......<br>..... |
| iPS:435807 | 21-225_181C10 | VH3\|3-33/D4\|4-17\|RF2/JH4 | .............<br>.............<br>........ | .......... | ........<br>....M. | I.......<br>..Y.....<br>.... | .................<br>............... | .HY.FW............<br>......SGH... | ......<br>..... |
| iPS:435887 | 21-225_186F7 | VH3\|3-33/D4\|4-17\|RF2/JH4 | .............<br>.............<br>........ | T......... | ........<br>...... | I.......<br>..Y.....<br>.... | .................<br>............... | .HY.FW............<br>......SGH... | ......<br>..... |
| iPS:435901 | 21-225_189G2 | VH3\|3-33/D4\|4-17\|RF2/JH4 | .............<br>.............<br>........ | N......... | ........<br>...... | I.......<br>..Y.....<br>.... | .................<br>............... | .RF.FW............<br>......SG.S.. | ......<br>..... |
| iPS:436594 | 21-225_226A5 | VH3\|3-33/D4\|4-17\|RF2/JH4 | .............<br>.............<br>........ | N......... | ........<br>...... | I.......<br>.T....T.<br>.... | .................<br>............... | EGH.FW............<br>......SGF.C. | ......<br>..... |
| iPS:392814 | 21-225_22A1 | VH3\|3-33/D4\|4-17\|RF2/JH4 | .............<br>.............<br>........ | .......... | ........<br>...... | .M......<br>........<br>.... | .................<br>............... | .G.FL.............<br>.......EWL.. | ......<br>..... |
| iPS:393036 | 21-225_28G3 | VH3\|3-33/D4\|4-17\|RF2/JH4 | .............<br>.............<br>.....I.. | T......... | ........<br>...... | ........<br>........<br>.... | .................<br>............... | .RY.FW............<br>......SG.... | ......<br>..... |
| | | Germline | **H_FR1** | **H_CDR1** | **H_FR2** | **H_CDR2** | **H_FR3** | **H_CDR3** | **H_FR4** |
| | VH3\|3-33/D7\|7-27\|RF1/JH4 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | LTGY--------------<br>---------FDY | WGQGTL<br>VTVSS |
| iPS:468824 | 21-225_73G6 | VH3\|3-33/D7\|7-27\|RF1/JH4 | .............<br>.............<br>........ | .......... | ........<br>...... | ........<br>V.....G.<br>.... | .................<br>............... | EV.M..............<br>........TS.. | ......<br>..... |

| | | | | | | | | | |
|---|---|---|---|---|---|---|---|---|---|
| iPS:43 4169 | 21-225_50C4 | VH3\|3-33/D7\|7-27\|RF1/JH4 | ............<br>............<br>........ | D......... | ........<br>...... | ....E...<br>ET......<br>.... | .................<br>....G.......... | EV.F.............<br>........LN.. | ....I.<br>..... |
| iPS:43 5045 | 21-225_90H5 | VH3\|3-33/D7\|7-27\|RF1/JH4 | ............<br>............<br>........ | D......... | ....S...<br>...... | ....E...<br>...T....<br>.... | .................<br>.............V. | EM.W.............<br>........LD.. | ......<br>..... |
| iPS:43 5367 | 21-225_149G1 | VH3\|3-33/D7\|7-27\|RF1/JH4 | ............<br>............<br>........ | D......... | ........<br>...... | ....E...<br>........<br>.... | ...........M.....<br>............... | EI.F.............<br>........SE.. | ......<br>..... |
| iPS:43 5397 | 21-225_149F12 | VH3\|3-33/D7\|7-27\|RF1/JH4 | ............<br>............<br>........ | D......... | ........<br>...... | ....E...<br>EN......<br>.... | .............F...<br>............... | EI.F.............<br>........SE.. | ......<br>..... |
| iPS:43 5407 | 21-225_150E7 | VH3\|3-33/D7\|7-27\|RF1/JH4 | ............<br>............<br>........ | D......... | ........<br>...... | ....E...<br>EN......<br>.... | .............F...<br>............... | EI.F.............<br>........SE.. | ......<br>..... |
| iPS:43 5609 | 21-225_161F7 | VH3\|3-33/D7\|7-27\|RF1/JH4 | ............<br>............<br>........ | D.....F.L. | .......Q<br>...... | ...F....<br>........<br>.... | ....F............<br>............... | EI.W.............<br>........LS.. | ......<br>..... |
| iPS:43 5613 | 21-225_161D11 | VH3\|3-33/D7\|7-27\|RF1/JH4 | ............<br>............<br>........ | D.....F.L. | .......Q<br>...... | ...F....<br>........<br>.... | ....F............<br>............... | EI.W.............<br>........LS.. | ......<br>..... |
| iPS:43 5791 | 21-225_180H7 | VH3\|3-33/D7\|7-27\|RF1/JH4 | ............<br>............<br>........ | D......... | ........<br>...... | ........<br>EN..H...<br>.A.. | .................<br>....V.......... | EV.W.............<br>........SD.. | ......<br>..... |
| iPS:43 5805 | 21-225_181A8 | VH3\|3-33/D7\|7-27\|RF1/JH4 | ............<br>............<br>........ | D......... | ........<br>...... | ........<br>EN..H...<br>.A.. | .................<br>............... | EV.W.............<br>........SD.. | ......<br>.I... |
| iPS:43 5879 | 21-225_184H10 | VH3\|3-33/D7\|7-27\|RF1/JH4 | ............<br>...........V<br>........ | D......... | ........<br>...... | ........<br>ET..H.G.<br>.... | ..........D......<br>............... | EV.W.............<br>........HD.. | ......<br>..... |
| iPS:43 5881 | 21-225_184D11 | VH3\|3-33/D7\|7-27\|RF1/JH4 | ............<br>............<br>........ | D......... | ........<br>...... | ........<br>ET..H.G.<br>.... | ..........D......<br>............... | EV.W.............<br>........HD.. | ......<br>..... |
| iPS:43 | 21- | VH3\|3-33/D7\|7- | ............<br>............ | N......... | ........<br>...... | ........<br>EN....V. | .......D.........<br>.......S....... | E..F.............<br>........LS.. | ......<br>..... |

| | | | | | | | | | |
|---|---|---|---|---|---|---|---|---|---|
| iPS:436576 | 21-225_225B6 | VH3\|3-33/D7\|7-27\|RF1/JH4 | ..R.........<br>............<br>........ | D......... | ........<br>...... | ........<br>EN......<br>.... | .................<br>.............. | EV.F.............<br>........TE.. | ......<br>..... |
| iPS:436578 | 21-225_225D6 | VH3\|3-33/D7\|7-27\|RF1/JH4 | ............<br>............<br>........ | N......... | ........<br>...... | ........<br>EN......<br>.... | .........Q.......<br>T............. | EV.F.............<br>........TE.. | ......<br>..... |
| iPS:436582 | 21-225_225F8 | VH3\|3-33/D7\|7-27\|RF1/JH4 | ............<br>............<br>........ | .......... | ........<br>...... | ........<br>EN....V.<br>.... | .................<br>.............. | EV.F.............<br>........TE.. | ......<br>..... |
| iPS:436608 | 21-225_226A9 | VH3\|3-33/D7\|7-27\|RF1/JH4 | ............<br>............<br>........ | N......... | ........<br>...... | ....E...<br>E.....T.<br>.... | .............F...<br>.............. | EV.F.............<br>........TE.. | ......<br>..... |
| iPS:436630 | 21-225_227G3 | VH3\|3-33/D7\|7-27\|RF1/JH4 | ............<br>............<br>........ | .......... | ........<br>...... | ....V...<br>...Q....<br>.... | .................<br>S............. | EV.F.............<br>........TE.. | ......<br>..... |
| iPS:436634 | 21-225_227H5 | VH3\|3-33/D7\|7-27\|RF1/JH4 | ............<br>............<br>........ | N......... | ........<br>..D... | ....E...<br>E.......<br>.... | .................<br>.........M..... | EV.F.............<br>........TE.. | ......<br>..... |
| iPS:436650 | 21-225_227C12 | VH3\|3-33/D7\|7-27\|RF1/JH4 | ............<br>....K.......<br>........ | N......... | ........<br>...... | ....I...<br>...Q....<br>.... | .................<br>S............. | EV.F.............<br>........TE.. | ......<br>..... |
| iPS:437280 | 21-225_203C10 | VH3\|3-33/D7\|7-27\|RF1/JH4 | ............<br>............<br>........ | D......... | ........<br>...... | ........<br>.G.TH.T.<br>.... | .................<br>.............. | EV.W.............<br>........LD.. | ......<br>..... |
| iPS:392740 | 21-225_18H12 | VH3\|3-33/D7\|7-27\|RF1/JH4 | ............<br>............<br>........ | D......... | ........<br>....M. | ........<br>VT......<br>.... | .................<br>.............. | EV.W.............<br>........YE.. | ......<br>..... |
| iPS:392780 | 21-225_22B7 | VH3\|3-33/D7\|7-27\|RF1/JH4 | ............<br>............<br>........ | .......... | ........<br>...... | ....E...<br>EN.Q....<br>.... | .................<br>.............. | EV.F.............<br>........RS.. | ......<br>..... |
| iPS:392912 | 21-225_25A9 | VH3\|3-33/D7\|7-27\|RF1/JH4 | ............<br>............<br>........ | .......... | ........<br>...... | ........<br>VT....TG<br>.... | .................<br>.............. | EI.W.............<br>........LD.. | ......<br>..... |
| iPS:39 | 21-225_29D9 | VH3\|3-33/D7\|7- | ............<br>.......K...S | D.......I. | ........<br>...... | ........<br>E..N.... | .................<br>.............. | EI.W.............<br>........LD.. | .....Q<br>..... |

| | | | | | | | | | |
|---|---|---|---|---|---|---|---|---|---|
| iPS:39 2948 | 21-225_25G5 | VH3\|3-33/D7\|7-27\|RF1/J H4 | ............ ............ ........ | D.......I. | ........ ...... | ........ .N...... .... | .................. .N............. | EI.W.............. ........LD.. | ...... ..... |
| iPS:39 2978 | 21-225_28B8 | VH3\|3-33/D7\|7-27\|RF1/J H4 | ............ ............ ........ | D......... | ........ .....T | ........ AN...... .... | ........F...V.... ............... | EI.W.............. ........LD.. | ...... ..... |
| iPS:39 2998 | 21-225_28A9 | VH3\|3-33/D7\|7-27\|RF1/J H4 | ............ ............ ........ | ........I. | ........ ...... | ...F.... ........ .... | .............F... ....V.......... | EI.W.............. ........LD.. | ...... ..... |
| iPS:39 3038 | 21-225_29D8 | VH3\|3-33/D7\|7-27\|RF1/J H4 | ............ ............ .......R | D.......I. | ........ ...... | ...F.... .T...... .... | .................. .............G. | EI.W.............. ........LD.. | ...... ..... |
| iPS:39 3056 | 21-225_30F3 | VH3\|3-33/D7\|7-27\|RF1/J H4 | ............ ............ ........ | .......... | ........ ...... | ........ V....... .... | .................. ............... | EM.W.............. ........YD.. | ...... ..... |
| iPS:39 3074 | 21-225_33B1 | VH3\|3-33/D7\|7-27\|RF1/J H4 | ............ ............ ........ | D......... | ......A. ...... | ........ RN...... .... | .................. ............... | EM.W.............. ........YD.. | ...... ..... |
| iPS:39 3822 | 21-225_15B11 | VH3\|3-33/D7\|7-27\|RF1/J H4 | ............ ............ ........ | N......... | ........ ...... | ....E... E.....T. .... | .................. ....P.......... | EV.F.............. ........TE.. | ...... ..... |
| iPS:39 3856 | 21-225_14C2 | VH3\|3-33/D7\|7-27\|RF1/J H4 | ............ ............ ........ | D......... | ........ ...... | ........ E.....E. .... | .................. K.......G...... | EV.F.............. ........RS.. | ...... ..... |
| iPS:39 3874 | 21-225_4C8 | VH3\|3-33/D7\|7-27\|RF1/J H4 | ............ ............ ........ | N......... | ........ ...... | ....E... EN.Q.... .... | .................. ...T........SP. | EM.F.............. ........LS.. | ...... ..... |
| iPS:39 3984 | 21-225_4F12 | VH3\|3-33/D7\|7-27\|RF1/J H4 | ............ ............ ........ | .......... | ........ ...... | ........ VT..K... .... | .................. ...TP.N.GG.ENQ. | EK.G.............. ........L... | ...... ..... |
| iPS:39 4020 | 21-225_15H10 | VH3\|3-33/D7\|7-27\|RF1/J H4 | ............ ............ ........ | N......... | ........ ...... | ........ E.....E. .... | .................. .............V. | EV.F.............. ........LS.. | ....I. ..... |
| iPS:39 | 21-225_16H4 | VH3\|3-33/D7\|7- | ............ ............ | N......... | ........ ...... | ........ V....... | ...........M..... ............... | EM.W.............. ........TD.C | ...... ..... |

| | | Germline | H_FR1 | H_CDR1 | H_FR2 | H_CDR2 | H_FR3 | H_CDR3 | H_FR4 |
|---|---|---|---|---|---|---|---|---|---|
| | VH1\|1-08/D5\|5-24\|RF3/JH6 | | QVQLVQS-<br>GAEVKKPGASVK<br>VSCKASG-YTFT | S-----YDIN | WVRQATGQ<br>GLEWMG | WMNPN---<br>SGNTGYAQ<br>KFQG | RVTMTRNTSISTAYMEL<br>SSLRSEDTAVYYCAR | RDGYNYYY----------<br>-----YYYGMDV | WGQGTT<br>VTVSS |
| iPS:468818 | 21-225_190C8 | VH1\|1-08/D5\|5-24\|RF3/JH6 | ............<br>............<br>.......I | .......... | ........<br>...... | ....K...<br>R.......<br>.... | ......D.........<br>................ | G.P..WN-<br>...............-<br>S.A... | ....A.<br>..... |
| iPS:436023 | 21-225_193A5 | VH1\|1-08/D5\|5-24\|RF3/JH6 | ............<br>............<br>.......I | .......... | ........<br>...... | ....K...<br>R.......<br>.... | ......D.........<br>................ | G.P..WN-<br>...............-<br>S.A... | ....A.<br>..... |
| iPS:436132 | 21-225_196C12 | VH1\|1-08/D5\|5-24\|RF3/JH6 | ............<br>............<br>.......I | .......... | ........<br>...... | ....K...<br>R.......<br>.... | ......D.........<br>................ | G.P..WN-<br>...............-<br>S.A... | ....A.<br>..... |

| | | Germline | H_FR1 | H_CDR1 | H_FR2 | H_CDR2 | H_FR3 | H_CDR3 | H_FR4 |
|---|---|---|---|---|---|---|---|---|---|
| | VH3\|3-33/D2\|2-8\|RF3/JH5 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | DIVLMVY----------<br>-----AINWFDP | WGQGTL<br>VTVSS |
| iPS:468828 | 21-225_162A10 | VH3\|3-33/D2\|2-8\|RF3/JH5 | ............<br>............<br>........ | ......C... | ........<br>...... | A.......<br>........<br>.... | .............F...<br>............... | .KNI.G-<br>................-<br>DT...F | ......<br>..... |

| | | Germline | H_FR1 | H_CDR1 | H_FR2 | H_CDR2 | H_FR3 | H_CDR3 | H_FR4 |
|---|---|---|---|---|---|---|---|---|---|
| | VH1\|1-02/D5\|5-18\|RF3/JH4 | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | GYSYG------------<br>-------YYFDY | WGQGTL<br>VTVSS |
| iPS:468830 | 21-225_191G11 | VH1\|1-02/D5\|5-18\|RF3/JH4 | ............<br>............<br>........ | .......... | ........<br>...... | ........<br>.....F..<br>.... | ...L......N......<br>.W............. | .KN...............<br>.......S.... | ......<br>..... |
| iPS:436896 | 21-225_67F10 | VH1\|1-02/D5\|5-18\|RF3/JH4 | ............<br>............<br>........ | .......... | ........<br>...... | ........<br>......G.<br>.... | .................<br>............... | T.F..SGS..........<br>.....YYNG... | ......<br>..... |
| iPS:393218 | 21-225_14G3 | VH1\|1-02/D5\|5-18\|RF3/JH4 | ............<br>............<br>........ | .........Y | ........<br>...... | ........<br>........<br>.... | .................<br>............... | S.F..SGS..........<br>.....YYNE... | ......<br>..... |
| iPS:393565 | 21-225_34B11 | VH1\|1-02/D5\|5-18\|RF3/JH4 | ..K.........<br>............<br>........ | .......... | ........<br>...... | ........<br>........<br>.... | .................<br>............... | V.F..SGS..........<br>.....YYNE... | ......<br>..... |

| | | | | | | | | | |
|---|---|---|---|---|---|---|---|---|---|
| iPS:398470 | 21-225_14B7 | VH1\|1-02/D5\|5-18\|RF3/JH4 | .............<br>.............<br>........ | ........... | .........<br>...... | ........<br>......V.<br>.... | .............C...<br>...K.......F... | SFF..SGS..........<br>.....YYNE... | ......<br>..... |
| | | Germline | H_FR1 | H_CDR1 | H_FR2 | H_CDR2 | H_FR3 | H_CDR3 | H_FR4 |
| | VH3\|3-30.3/D1\|1-1\|RF1/JH6 | | QVQLVES-<br>GGGVVQPGRSLR<br>LSCAASG-FTFS | S-----YAMH | WVRQAPGK<br>GLEWVA | VISYD---<br>GSNKYYAD<br>SVKG | RFTISRDNSKNTLYLQM<br>NSLRAEDTAVYYCAR | GTTGTYY-----------<br>-----YYYGMDV | WGQGTT<br>VTVSS |
| iPS:468836 | 21-225_198E3 | VH3\|3-30.3/D1\|1-1\|RF1/JH6 | ............<br>............<br>........ | .......G.. | ........<br>...... | ........<br>.GY.N...<br>.... | .................<br>............... | ..H.Y.-<br>................--<br>..V.. | ......<br>..... |
| iPS:435831 | 21-225_190C12 | VH3\|3-30.3/D1\|1-1\|RF1/JH6 | ..........A.<br>............<br>........ | .......G.. | ........<br>...... | ........<br>.GY.N.V.<br>.... | .................<br>............... | ..H.Y.-<br>................--<br>..V.. | ......<br>..... |
| iPS:435857 | 21-225_191A4 | VH3\|3-30.3/D1\|1-1\|RF1/JH6 | ............<br>............<br>........ | .......G.. | ........<br>...... | ........<br>.GY.N...<br>.... | .................<br>............... | ..H.Y.-<br>................--<br>..V.. | ......<br>..... |
| iPS:435907 | 21-225_190G3 | VH3\|3-30.3/D1\|1-1\|RF1/JH6 | ............<br>............<br>........ | .......G.. | ........<br>...... | ........<br>.GY.N.V.<br>.... | .................<br>............... | ..H.Y.-<br>................--<br>..V.. | ......<br>..... |
| iPS:435919 | 21-225_190H5 | VH3\|3-30.3/D1\|1-1\|RF1/JH6 | ............<br>............<br>........ | .......G.. | ........<br>...... | ........<br>.GY.N...<br>.... | .................<br>............... | ..H.Y.-<br>................--<br>..V.. | ......<br>..... |
| iPS:435989 | 21-225_192F7 | VH3\|3-30.3/D1\|1-1\|RF1/JH6 | ............<br>............<br>........ | .......G.. | ........<br>.....T | ........<br>.GY.N...<br>.... | .................<br>............... | ..H.Y.-<br>................--<br>..V.. | ......<br>..... |
| iPS:436222 | 21-225_200C9 | VH3\|3-30.3/D1\|1-1\|RF1/JH6 | ............<br>............<br>........ | .......G.. | ........<br>...... | ........<br>.GY.N.I.<br>.... | .................<br>..........F.... | ..H.Y.-<br>................--<br>..V.. | ......<br>..... |
| | | Germline | H_FR1 | H_CDR1 | H_FR2 | H_CDR2 | H_FR3 | H_CDR3 | H_FR4 |
| | VH4\|4-30.1/D5\|5- | | QVQLQES-<br>GPGLVKPSQTLS | SG---GYYWS | WIRQHPGK<br>GLEWIG | YIYY----<br>SGSTYYNP | RVTISVDTSKNQFSLKL<br>SSVTAADTAVYYCAR | RDGYNYYY----------<br>-----YYYGMDV | WGQGTT<br>VTVSS |

| | | | | | | | | | |
|---|---|---|---|---|---|---|---|---|---|
| iPS:468840 | 21-225_200H9 | VH4\|4-30.1/D5\|5-24\|RF3/JH6 | .........................I......MR | ......D... | ............F. | ....................  | ...L............................ | M.YS..--...............-...... | .....S..... |
| iPS:436096 | 21-225_195E10 | VH4\|4-30.1/D5\|5-24\|RF3/JH6 | .................................. | .......... | .............. | .................... | ....................A........... | GGYNWN--...............--H.... | ........... |
| iPS:436120 | 21-225_196C10 | VH4\|4-30.1/D5\|5-24\|RF3/JH6 | ..........................MR | ......D... | ............F. | F................... | ...L............................ | M.YS..--...............-...... | ........... |
| iPS:436216 | 21-225_200B7 | VH4\|4-30.1/D5\|5-24\|RF3/JH6 | .................................. | .......... | .............. | ..F.........N.....R. | ................................ | AGYNWN--...............--N.... | ........... |
| | | Germline | H_FR1 | H_CDR1 | H_FR2 | H_CDR2 | H_FR3 | H_CDR3 | H_FR4 |
| | VH3\|3-21/D6\|6-6\|RF2/JH4 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | SIAAR-----------------YFDY | WGQGTLVTVSS |
| iPS:468844 | 21-225_48E10 | VH3\|3-21/D6\|6-6\|RF2/JH4 | .................................. | .......N.. | .............. | ...G................ | ................................ | .L---..................--.L | ........... |
| iPS:435537 | 21-225_157H12 | VH3\|3-21/D6\|6-6\|RF2/JH4 | ...W.............................. | .......... | .............. | ...G....G...N....... | ..........T.....V.G............. | .KF--..................--.S | ........... |
| iPS:435539 | 21-225_158G1 | VH3\|3-21/D6\|6-6\|RF2/JH4 | .................................. | .......G.. | ............I. | ...G....G........... | ...............................I | .SG--..................--WS | ........... |
| iPS:435583 | 21-225_160F2 | VH3\|3-21/D6\|6-6\|RF2/JH4 | .................................. | .......G.. | ............I. | ...G....G........... | ...............................I | .SG--..................--WS | ........... |
| | | Germline | H_FR1 | H_CDR1 | H_FR2 | H_CDR2 | H_FR3 | H_CDR3 | H_FR4 |
| | VH3\|3-21/D1\|1-1\|RF2/JH5 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | VQLER------------------NWFDP | WGQGTLVTVSS |

| | | | | | | | | | |
|---|---|---|---|---|---|---|---|---|---|
| iPS:46 8846 | 21-225_53B10 | VH3\|3-21/D1\|1-1\|RF2/JH5 | ............<br>............<br>........ | .......... | ........<br>...... | ...G....<br>......V.<br>.... | .................<br>............... | .NS--<br>..................<br>..--..S | ......<br>..... |
| iPS:43 4251 | 21-225_62G3 | VH3\|3-21/D1\|1-1\|RF2/JH5 | ............<br>............<br>........ | .......... | ........<br>...... | ........<br>........<br>.... | .................<br>............... | .NS--<br>..................<br>..--..S | ......<br>..... |
| iPS:43 4407 | 21-225_68G8 | VH3\|3-21/D1\|1-1\|RF2/JH5 | ............<br>............<br>........ | .......... | ........<br>...... | ...G....<br>........<br>..M. | .................<br>............... | .NS--<br>..................<br>..--..S | ......<br>..... |
| iPS:43 5575 | 21-225_159H11 | VH3\|3-21/D1\|1-1\|RF2/JH5 | ............<br>............<br>........ | .......T.. | ........<br>...... | ...G....<br>........<br>.... | .................<br>............... | .SW--<br>..................<br>..--A.C | ......<br>..... |
| | | Germline | H_FR1 | H_CDR1 | H_FR2 | H_CDR2 | H_FR3 | H_CDR3 | H_FR4 |
| | VH3\|3-23/D1\|1-1\|RF2/JH4 | | EVQLLES-<br>GGGLVQPGGSLR<br>LSCAASG-FTFS | S------YAMS | WVRQAPGK<br>GLEWVS | AISGS---<br>GGSTYYAD<br>SVKG | RFTISRDNSKNTLYLQM<br>NSLRAEDTAVYYCAK | VQLER-------------<br>--------YFDY | WGQGTL<br>VTVSS |
| iPS:46 8848 | 21-225_54B1 | VH3\|3-23/D1\|1-1\|RF2/JH4 | ............<br>............<br>........ | .......... | ........<br>...... | VL......<br>....F...<br>.... | ......E..........<br>S.............R | RGR.YSG...........<br>......YD.... | ......<br>..... |
| iPS:43 3993 | 21-225_47G7 | VH3\|3-23/D1\|1-1\|RF2/JH4 | .....D......<br>............<br>........ | .......... | ........<br>...... | ....R...<br>..N.F..E<br>..R. | .................<br>............... | IIR.Q.............<br>.......WA... | ......<br>..... |
| iPS:43 4007 | 21-225_48D7 | VH3\|3-23/D1\|1-1\|RF2/JH4 | ............<br>............<br>.......R | N.....S..N | ........<br>...... | ........<br>..T.F...<br>.... | ................I<br>............... | CGR.Q.............<br>........WL.. | ......<br>..... |
| iPS:43 4115 | 21-225_53E4 | VH3\|3-23/D1\|1-1\|RF2/JH4 | ............<br>............<br>........ | .......V.. | ........<br>...... | G.......<br>..R.....<br>.... | ..N..............<br>..............R | .A.--<br>..................<br>...-... | ......<br>..... |
| iPS:43 5679 | 21-225_169D10 | VH3\|3-23/D1\|1-1\|RF2/JH4 | ............<br>..S.........<br>........ | .......V.. | ........<br>...... | ........<br>.SRI....<br>.... | .................<br>.........L....R | .AF--<br>..................<br>...-... | ......<br>..... |
| iPS:43 5685 | 21-225_170E1 | VH3\|3-23/D1\|1-1\|RF2/JH4 | ............<br>............<br>........ | .......V.. | ........<br>...... | ........<br>.NRI....<br>.... | .................<br>.........L....R | .AF--<br>..................<br>...-... | ......<br>..... |

| | | | | | | | | | |
|---|---|---|---|---|---|---|---|---|---|
| **iPS:43 6632** | **21-225_227E4** | **VH3\|3-23/D1\|1-1\|RF2/JH4** | .G..........<br>...........T<br>........ | T.....F..T | .......R<br>...... | V...R...<br>...SF...<br>.... | ........T........<br>............... | D..W-<br>...................<br>...-... | ......<br>..... |
| | | **Germline** | **H_FR1** | **H_CDR1** | **H_FR2** | **H_CDR2** | **H_FR3** | **H_CDR3** | **H_FR4** |
| | **VH4\|4-39/D4\|4-11\|RF2/JH5** | | QLQLQES-<br>GPGLVKPSETLS<br>LTCTVSG-GSIS | SS---SYYWG | WIRQPPGK<br>GLEWIG | SIYY----<br>SGSTYYNP<br>SLKS | RVTISVDTSKNQFSLKL<br>SSVTAADTAVYYCAR | DYSNY-----------<br>-------NWFDP | WGQGTL<br>VTVSS |
| **iPS:46 8856** | **21-225_77C9** | **VH4\|4-39/D4\|4-11\|RF2/JH5** | ............<br>.T..........<br>........ | R......... | ........<br>...... | ........<br>...A.S..<br>.... | .................<br>.........LF.... | LD..W...........<br>.......-GL.Y | ......<br>..... |
| **iPS:43 4489** | **21-225_74E4** | **VH4\|4-39/D4\|4-11\|RF2/JH5** | ............<br>.........I..<br>........ | .....N.... | ........<br>...... | ........<br>..Y.S...<br>.... | .......S...H...R.<br>............... | LD..W...........<br>.......-GL.Y | ......<br>..... |
| **iPS:43 5251** | **21-225_96A3** | **VH4\|4-39/D4\|4-11\|RF2/JH5** | ............<br>.........I..<br>........ | .....N.... | ........<br>...... | ........<br>..Y.S...<br>.... | .......S...H...R.<br>............... | LD..W...........<br>.......-GL.Y | ......<br>..... |
| **iPS:43 7346** | **21-225_75H7** | **VH4\|4-39/D4\|4-11\|RF2/JH5** | ............<br>.T..........<br>........ | R......... | ........<br>...... | ........<br>...A.S..<br>.... | .................<br>.........LF.... | LD..W...........<br>.......-GL.Y | ......<br>..... |
| **iPS:39 3886** | **21-225_2G9** | **VH4\|4-39/D4\|4-11\|RF2/JH5** | ............<br>............<br>.......R | PN...-.... | ........<br>...... | ........<br>....S...<br>..N. | .................<br>N.............. | LS..W...........<br>.......-D..N | ......<br>..... |
| **iPS:39 3928** | **21-225_4E10** | **VH4\|4-39/D4\|4-11\|RF2/JH5** | ............<br>............<br>........ | R......... | ........<br>...... | .V......<br>..A.S...<br>.... | .................<br>N.......L...V. | LS..W...........<br>.......-D..Y | ......<br>F.... |
| | | **Germline** | **H_FR1** | **H_CDR1** | **H_FR2** | **H_CDR2** | **H_FR3** | **H_CDR3** | **H_FR4** |
| | **VH1\|1-02/D6\|6-6\|RF1/JH6** | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | EYSSSSYY---------<br>-----YYYGMDV | WGQGTT<br>VTVSS |
| **iPS:46 8862** | **21-225_178H8** | **VH1\|1-02/D6\|6-6\|RF1/JH6** | ............<br>RT..........<br>........ | D......... | ........<br>...... | ........<br>R.......<br>.... | .................<br>............... | .EDR.GW..........<br>............ | ......<br>..... |
| **iPS:45 1112** | **21-225_53D10** | **VH1\|1-02/D6\|6-6\|RF1/JH6** | ............<br>............<br>.....I.. | .........I. | ........<br>...... | ........<br>........<br>.... | .................<br>I.............. | .NE.LATRP.........<br>...FYD...... | ......<br>..... |
| | | **Germline** | **H_FR1** | **H_CDR1** | **H_FR2** | **H_CDR2** | **H_FR3** | **H_CDR3** | **H_FR4** |

| | VH2\|2-05/D6\|6-6\|RF2/JH4 | | QITLKES-GPTLVKPTQTLTLTCTFSG-FSLS | TS---GVGVG | WIRQPPGKALEWLA | LIYW----NDDKRYSPSLKS | RLTITKDTSKNQVVLTMTNMDPVDTATYYCAH | SIAAR-------------------YFDY | WGQGTLVTVSS |
|---|---|---|---|---|---|---|---|---|---|
| iPS:468864 | 21-225_60D6 | VH2\|2-05/D6\|6-6\|RF2/JH4 | ............<br>............<br>........ | .......... | ........<br>...... | ........<br>K..E....<br>.... | .................<br>............... | AV.V-<br>...................<br>...S... | ......<br>..... |
| iPS:436850 | 21-225_57D9 | VH2\|2-05/D6\|6-6\|RF2/JH4 | ..........M.<br>............<br>........ | .......... | ........<br>...... | ........<br>........<br>.... | .....E...........<br>............... | AV.V-<br>...................<br>...S... | ......<br>..... |
| iPS:436914 | 21-225_76B4 | VH2\|2-05/D6\|6-6\|RF2/JH4 | ............<br>............<br>........ | .G........ | ........<br>...... | ........<br>D.......<br>.... | ........P........<br>............... | L..V-<br>...................<br>...A... | ......<br>..... |
| iPS:436918 | 21-225_77A2 | VH2\|2-05/D6\|6-6\|RF2/JH4 | ..........S.<br>............<br>........ | .......... | ........<br>.....V | F.......<br>D.......<br>.... | .................<br>............... | L..V-<br>...................<br>...A... | ......<br>..... |
| iPS:436934 | 21-225_96B5 | VH2\|2-05/D6\|6-6\|RF2/JH4 | ............<br>............<br>........ | .G........ | ........<br>...... | ........<br>D.......<br>.... | ........P........<br>............... | L..V-<br>...................<br>...AC.. | ......<br>..... |
| iPS:437334 | 21-225_75F11 | VH2\|2-05/D6\|6-6\|RF2/JH4 | ............<br>............<br>........ | .G........ | ........<br>...... | ........<br>D.......<br>.... | ........P........<br>............... | L..V-<br>...................<br>...A... | ......<br>..... |
| iPS:437377 | 21-225_74G9 | VH2\|2-05/D6\|6-6\|RF2/JH4 | ............<br>............<br>........ | .G........ | ........<br>...... | ........<br>D.......<br>.... | ........P........<br>............... | L..V-<br>...................<br>...A... | ......<br>..... |
| iPS:392583 | 21-225_10B10 | VH2\|2-05/D6\|6-6\|RF2/JH4 | ............<br>............<br>........ | .G........ | ........<br>.....V | F.......<br>S.......<br>.... | ..S..............<br>..............R | IA.V-<br>...................<br>...A... | ......<br>..... |
| iPS:393184 | 21-225_15H11 | VH2\|2-05/D6\|6-6\|RF2/JH4 | .........L..<br>M...........<br>........ | .G........ | ........<br>...... | ........<br>H.......<br>..R. | .................<br>..............R | IV.V-<br>...................<br>...A... | ......<br>I.... |
| iPS:393212 | 21-225_30H6 | VH2\|2-05/D6\|6-6\|RF2/JH4 | ............<br>............<br>........ | .G........ | ........<br>...... | ........<br>H.......<br>.... | ..A.............I<br>............... | L..V-<br>...................<br>...A... | ......<br>..... |
| iPS:393222 | 21-225_17F5 | VH2\|2-05/D6\|6-6\|RF2/JH | ..........S.<br>............ | .......... | ........<br>...... | ........<br>D....... | .................<br>...........S... | L..V-<br>...................<br>A | ......<br>..... |

| | | | | | | | | | |
|---|---|---|---|---|---|---|---|---|---|
| iPS:39 3224 | 21-225_31C2 | VH2\|2-05/D6\|6-6\|RF2/JH4 | .............<br>.............<br>.......N | .G........ | ........<br>...... | ........<br>...E....<br>.... | .................<br>.....L...S..... | L..V-<br>..................<br>...S... | ....A.<br>..... |
| | | Germline | **H_FR1** | **H_CDR1** | **H_FR2** | **H_CDR2** | **H_FR3** | **H_CDR3** | **H_FR4** |
| | VH1\|1-02/D6\|6-19\|RF2/JH6 | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | GIAVAGYY----------<br>-----YYYGMDV | WGQGTT<br>VTVSS |
| iPS:46 8866 | 21-225_190C1 | VH1\|1-02/D6\|6-19\|RF2/JH6 | ............<br>............<br>........ | .......... | ........<br>...... | ....Y...<br>........<br>.... | .................<br>............... | DR....N..........<br>.....F...... | ......<br>..... |
| iPS:43 6972 | 21-225_190C7 | VH1\|1-02/D6\|6-19\|RF2/JH6 | ............<br>............<br>........ | .......... | ........<br>...... | ........<br>........<br>.... | .................<br>............... | DR....N..........<br>.....F...... | ......<br>..... |
| iPS:43 7020 | 21-225_193F11 | VH1\|1-02/D6\|6-19\|RF2/JH6 | ............<br>............<br>........ | .......... | ........<br>...... | ....Y...<br>........<br>.... | .................<br>............... | DR....N..........<br>.....F...... | ......<br>..... |
| iPS:43 7036 | 21-225_195H9 | VH1\|1-02/D6\|6-19\|RF2/JH6 | ............<br>............<br>........ | .......... | ........<br>...... | ....Y...<br>........<br>...D | ..........T......<br>............... | DR....N..........<br>.....F...... | ......<br>..... |
| iPS:43 7042 | 21-225_197E8 | VH1\|1-02/D6\|6-19\|RF2/JH6 | ....L.......<br>R......R....<br>........ | ........I. | ........<br>...... | ........<br>........<br>R... | .................<br>............... | E.....N..........<br>.....F....G. | ......<br>.A... |
| | | Germline | **H_FR1** | **H_CDR1** | **H_FR2** | **H_CDR2** | **H_FR3** | **H_CDR3** | **H_FR4** |
| | VH4\|4-39/D1\|1-1\|RF2/JH4 | | QLQLQES-<br>GPGLVKPSETLS<br>LTCTVSG-GSIS | SS---SYYWG | WIRQPPGK<br>GLEWIG | SIYY----<br>SGSTYYNP<br>SLKS | RVTISVDTSKNQFSLKL<br>SSVTAADTAVYYCAR | VQLER------------<br>--------YFDY | WGQGTL<br>VTVSS |
| iPS:46 8868 | 21-225_74A1 | VH4\|4-39/D1\|1-1\|RF2/JH4 | ............<br>............<br>........ | G......... | ........<br>...... | N.......<br>.....H..<br>.... | .................<br>T.........F.... | HD.LW............<br>.........SL.F | ....I.<br>..... |
| | | Germline | **H_FR1** | **H_CDR1** | **H_FR2** | **H_CDR2** | **H_FR3** | **H_CDR3** | **H_FR4** |
| | VH1\|1-02/D3\|3-22\|RF2/JH5 | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | YYYDSSGY----------<br>-----YYNWFDP | WGQGTL<br>VTVSS |
| iPS:47 2742 | 21-225_30D9_LC2 | VH1\|1-02/D3\|3-22\|RF2/JH5 | ..K.........<br>E...........<br>........ | ........L. | ........<br>...... | ........<br>........<br>.... | .................<br>............... | V..YG..S..........<br>.........E..N | ......<br>..... |

| | | | | | | | | | |
|---|---|---|---|---|---|---|---|---|---|
| **iPS:472741** | **21-225_30D9_LC1** | **VH1\|1-02/D3\|3-22\|RF2/JH5** | ..K.........<br>E...........<br>........ | ........L. | ........<br>...... | ........<br>........<br>.... | ..................<br>............... | V..YG..S..........<br>........E..N | ......<br>..... |
| **iPS:437040** | **21-225_196E7** | **VH1\|1-02/D3\|3-22\|RF2/JH5** | ............<br>............<br>V....... | .......N.. | ........<br>...... | ........<br>.......H<br>.... | ..................<br>............... | D...T..-<br>...............-<br>EG.... | ......<br>..... |
| **iPS:437050** | **21-225_197C11** | **VH1\|1-02/D3\|3-22\|RF2/JH5** | ............<br>............<br>V....... | .......N.. | ........<br>...... | ........<br>.......H<br>.... | ..................<br>............... | D......-<br>...............-<br>EG.... | ......<br>..... |
| **iPS:393214** | **21-225_33A1** | **VH1\|1-02/D3\|3-22\|RF2/JH5** | ............<br>............<br>.......S | .......... | ........<br>...... | ........<br>N...H...<br>.... | ..........R..S...<br>...........F... | G..YA..S..........<br>........DL.. | ......<br>..... |
| | | **Germline** | **H_FR1** | **H_CDR1** | **H_FR2** | **H_CDR2** | **H_FR3** | **H_CDR3** | **H_FR4** |
| | **VH1\|1-02/D3\|3-22\|RF2/JH4** | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | YYYDSSG-----------<br>-----YYYYFDY | WGQGTL<br>VTVSS |
| **iPS:472743** | **21-225_68G6** | **VH1\|1-02/D3\|3-22\|RF2/JH4** | ......F..G..<br>....S.......<br>........ | .......... | ........<br>...... | S.YR....<br>........<br>.... | .......K........K<br>..I............ | AF.YG..T..........<br>.......NE... | ......<br>..... |
| **iPS:436902** | **21-225_69B11** | **VH1\|1-02/D3\|3-22\|RF2/JH4** | ............<br>.......R....<br>........ | .......... | ........<br>...... | ........<br>......G.<br>...D | .............F...<br>.........A..... | T..YG..S..........<br>.......NG... | ......<br>..... |
| **iPS:436904** | **21-225_71D4** | **VH1\|1-02/D3\|3-22\|RF2/JH4** | ............<br>............<br>........ | .......C.. | ........<br>...... | ........<br>........<br>...V | .........V..V..D.<br>............... | A..YG..T..........<br>......HNE... | ....S.<br>..... |
| **iPS:436906** | **21-225_72B4** | **VH1\|1-02/D3\|3-22\|RF2/JH4** | ............<br>............<br>........ | .......... | ........<br>...... | ........<br>......G.<br>.... | ..................<br>............... | T..YG..S..........<br>.......NG... | ......<br>..... |
| **iPS:437034** | **21-225_195E9** | **VH1\|1-02/D3\|3-22\|RF2/JH4** | ...V........<br>............<br>........ | .......... | ........<br>...... | ........<br>..A.....<br>.... | ..................<br>N.............. | A..YG..T..........<br>.......NE... | ......<br>..... |
| **iPS:392598** | **21-225_18E10** | **VH1\|1-02/D3\|3-22\|RF2/JH4** | ............<br>............<br>........ | .......... | ........<br>...... | ........<br>........<br>.... | .......S..N......<br>............... | S..YG..S..........<br>.......NE... | ......<br>..... |

| | | | | | | | | | |
|---|---|---|---|---|---|---|---|---|---|
| iPS:39 3182 | 21-225_4B3 | VH1\|1-02/D3\|3-22\|RF2/JH4 | ............ ............ ........ | .......... | ........ ...... | ........ .....S.. .... | ................. ............... | S..YG..S......... .......NE... | ...... ..... |
| iPS:39 3200 | 21-225_35E1 | VH1\|1-02/D3\|3-22\|RF2/JH4 | ..K......... ............ ........ | .......... | ........ ...... | ....K... ........ .... | ............V...P ............... | V..HG..S......... .......NE... | ...... ..... |
| iPS:39 3206 | 21-225_13F6 | VH1\|1-02/D3\|3-22\|RF2/JH4 | ............ ............ ........ | .......... | ........ ...... | ........ ...A.... .... | ................. ...........F... | SF.YG..T......... .......NE... | ...... ..... |
| iPS:39 3208 | 21-225_16F3 | VH1\|1-02/D3\|3-22\|RF2/JH4 | ............ ............ ........ | ......H... | ........ ...... | ........ ........ .... | ................. ............... | S..YG..T......... .......NE... | ...... ..... |
| iPS:39 3210 | 21-225_17D3 | VH1\|1-02/D3\|3-22\|RF2/JH4 | ............ ............ ........ | .......... | ........ ...... | ........ ........ .... | ................. ............... | AN.YG..S......... .......ND... | ...... ..... |
| iPS:39 3226 | 21-225_33E6 | VH1\|1-02/D3\|3-22\|RF2/JH4 | ..K......... ............ ........ | .......... | ........ ...... | ........ ........ .... | ................. ............... | V..YG..S......... .......NE... | ...... ..... |
| iPS:39 3230 | 21-225_9G9 | VH1\|1-02/D3\|3-22\|RF2/JH4 | ............ ............ ........ | ........I. | ........ ...... | ........ ....D... .... | ................. ............... | S..YG..T......... .......NE... | ...... ..... |
| iPS:39 8490 | 21-225_21D12 | VH1\|1-02/D3\|3-22\|RF2/JH4 | ............ ............ ........ | D.......I. | ........ ...... | ........ .....N.. .... | ................. ...........S... | S..YG..T......... .......NE... | ...... ..... |
| iPS:42 3018 | 21-225_31D12_LC2 | VH1\|1-02/D3\|3-22\|RF2/JH4 | ..K......... ............ ........ | .......... | ........ ...... | ........ ......V. .... | ................. ............... | V..YG..S......... .......NE... | ...... ..... |
| iPS:42 3019 | 21-225_31D12_LC1 | VH1\|1-02/D3\|3-22\|RF2/JH4 | ..K......... ............ ........ | .......... | ........ ...... | ........ ......V. .... | ................. ............... | V..YG..S......... .......NE... | ...... ..... |
| | | Germline | H_FR1 | H_CDR1 | H_FR2 | H_CDR2 | H_FR3 | H_CDR3 | H_FR4 |
| | VH3\|3-33/D7\|7-27\|RF2/JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | *LGY-------------------FDY | WGQGTLVTVSS |

| | | | | | | | | | |
|---|---|---|---|---|---|---|---|---|---|
| iPS:39 2920 | 21-225_29G4 | VH3\|3-33/D7\|7-27\|RF2/JH4 | .............<br>..........T..<br>........ | D.......I. | .........<br>...... | ........<br>E.......<br>.M.. | ..................<br>................ | E..M.............<br>.........TG.. | ......<br>..... |
| iPS:43 3899 | 21-225_43C3 | VH3\|3-33/D7\|7-27\|RF2/JH4 | .............<br>.............<br>........ | ........I. | .........<br>...... | ........<br>EN......<br>.... | ..................<br>................ | E..F.............<br>.........SN.. | ......<br>..... |
| iPS:43 3921 | 21-225_44C3 | VH3\|3-33/D7\|7-27\|RF2/JH4 | .............<br>.............<br>........ | .......... | .........<br>...... | ...FE...<br>........<br>.... | ..................<br>S.............V. | E..F.............<br>.........ST.. | ......<br>..... |
| iPS:43 3933 | 21-225_44C8 | VH3\|3-33/D7\|7-27\|RF2/JH4 | .............<br>.............<br>.......N | N......... | .........<br>...... | ....E...<br>........<br>.... | ..................<br>..............V. | E..F.............<br>.........LS.. | ......<br>..... |
| iPS:43 3969 | 21-225_46F3 | VH3\|3-33/D7\|7-27\|RF2/JH4 | .............<br>.............<br>........ | D......... | .........<br>...... | ...FE...<br>........<br>.... | ...............V..<br>..............V. | E..F.............<br>.........SN.. | ......<br>..... |
| iPS:43 3975 | 21-225_46C6 | VH3\|3-33/D7\|7-27\|RF2/JH4 | .............<br>.............<br>........ | ........I. | .........<br>...... | ........<br>EN......<br>.... | ..................<br>................ | E..F.............<br>.........SN.. | ......<br>..... |
| iPS:43 3977 | 21-225_46D8 | VH3\|3-33/D7\|7-27\|RF2/JH4 | .............<br>.............<br>........ | D.......I. | .........<br>...... | ...FE...<br>........<br>.... | ...............V..<br>..............V. | E..F.............<br>.........SN.. | ......<br>..... |
| iPS:43 3983 | 21-225_47A1 | VH3\|3-33/D7\|7-27\|RF2/JH4 | .............<br>.............<br>........ | D......... | .........<br>...... | ........<br>DY..K...<br>.... | ........A........V<br>....V.........T. | E..M.............<br>.........L... | ......<br>..... |
| iPS:43 3997 | 21-225_48C1 | VH3\|3-33/D7\|7-27\|RF2/JH4 | .............<br>.............<br>........ | .......... | .........<br>...... | .V......<br>EI..K...<br>.... | .V................<br>.........M...... | E..W.............<br>.........EA.. | ......<br>..... |
| iPS:43 4009 | 21-225_48A9 | VH3\|3-33/D7\|7-27\|RF2/JH4 | .............<br>.............<br>........ | .......... | .........<br>...... | ....E...<br>ENK.....<br>.... | ..............F...<br>.........M.F.... | E.AW.............<br>.........YE.. | ......<br>..... |
| iPS:43 4013 | 21-225_48D12 | VH3\|3-33/D7\|7-27\|RF2/JH4 | .............<br>.............<br>........ | D......... | ........R<br>...... | ........<br>V.....V.<br>.... | ..................<br>................ | E..M.............<br>.........RS.. | ......<br>..... |
| iPS:43 | 21-225_49A1 | VH3\|3-33/D7\|7- | .............<br>............. | D......... | .........<br>...... | ........<br>ED....V. | ..................<br>................ | E..F.............<br>.........LS.. | ......<br>..... |

| | | | | | | | | | |
|---|---|---|---|---|---|---|---|---|---|
| iPS:43 4029 | 21-225_49C6 | VH3\|3-33/D7\|7-27\|RF2/JH4 | ............<br>............<br>........ | N......... | ........<br>...... | ...F....<br>V...K.V.<br>.... | .................<br>S.............. | D..M..............<br>.........IE.. | ......<br>..... |
| iPS:43 4057 | 21-225_51E4 | VH3\|3-33/D7\|7-27\|RF2/JH4 | ............<br>............<br>........ | .......... | ........<br>...... | ........<br>E.......<br>.... | .................<br>............... | E..F..............<br>.........LS.. | ......<br>..... |
| iPS:43 4071 | 21-225_51F9 | VH3\|3-33/D7\|7-27\|RF2/JH4 | ............<br>............<br>........ | D......... | ........<br>...... | ...F....<br>.N......<br>.... | .................<br>...S........... | E..F..............<br>.........LS.. | ......<br>..... |
| iPS:43 4075 | 21-225_51B11 | VH3\|3-33/D7\|7-27\|RF2/JH4 | ............<br>............<br>........ | D......... | ........<br>...... | ...FG...<br>.N....G.<br>.... | .................<br>...S........... | E..F..............<br>.........LS.. | ......<br>..... |
| iPS:43 4077 | 21-225_51F11 | VH3\|3-33/D7\|7-27\|RF2/JH4 | ............<br>............<br>........ | N.....F... | ........<br>...... | ....E...<br>E.......<br>.... | .................<br>............... | E..F..............<br>.........LS.F | ......<br>..... |
| iPS:43 4081 | 21-225_52B2 | VH3\|3-33/D7\|7-27\|RF2/JH4 | ............<br>............<br>........ | N......... | ........<br>...... | .T.F....<br>...QR...<br>.... | ......I.........<br>...S........... | D..M..............<br>.........IE.F | ......<br>..... |
| iPS:43 4091 | 21-225_52B9 | VH3\|3-33/D7\|7-27\|RF2/JH4 | ............<br>............<br>........ | D......... | ........<br>...... | ...F....<br>.N......<br>.... | .................<br>...S........... | E..F..............<br>.........LS.. | ......<br>..... |
| iPS:43 4105 | 21-225_53D2 | VH3\|3-33/D7\|7-27\|RF2/JH4 | ............<br>............<br>........ | N......... | ........<br>.....T | .V.D....<br>........<br>.... | .................<br>.............T. | G..F..............<br>.........TG.. | ....A.<br>..... |
| iPS:43 4119 | 21-225_53E6 | VH3\|3-33/D7\|7-27\|RF2/JH4 | ............<br>...........T<br>T....... | D.......I. | ........<br>...... | ........<br>E.....G.<br>.... | ..A..............<br>.............V. | E..M..............<br>.........TS.. | ......<br>..... |
| iPS:43 4129 | 21-225_53B12 | VH3\|3-33/D7\|7-27\|RF2/JH4 | ............<br>............<br>........ | N.....F... | ........<br>...... | .V......<br>.N.R....<br>.... | .................<br>H.............. | E..F..............<br>.........LS.F | ......<br>..... |
| iPS:43 4131 | 21-225_54D3 | VH3\|3-33/D7\|7-27\|RF2/JH4 | ............<br>............<br>........ | N......... | ........<br>...... | .T.F....<br>.N.N....<br>.... | .................<br>............... | E..F..............<br>.........LS.. | ......<br>..... |
| iPS:43 | 21-225_54C6 | VH3\|3-33/D7\|7- | ............<br>...........T | D.......I. | ........<br>..D... | ........<br>EN...... | ..A..............<br>.............V. | E..M..............<br>.........TS.. | ......<br>..... |

| | | | | | | | | | |
|---|---|---|---|---|---|---|---|---|---|
| iPS:43 4143 | 21-225_54G7 | VH3\|3-33/D7\|7-27\|RF2/JH4 | ............<br>............<br>........ | N......... | ........<br>...... | ....E...<br>E.....G.<br>.... | .................<br>............... | E..F.............<br>........LS.. | ......<br>..... |
| iPS:43 4155 | 21-225_55B3 | VH3\|3-33/D7\|7-27\|RF2/JH4 | ............<br>............<br>........ | .......... | ........<br>...... | ...F....<br>.N....E.<br>.... | .................<br>.............V. | E..F.............<br>........LS.. | ......<br>..... |
| iPS:43 4199 | 21-225_59F11 | VH3\|3-33/D7\|7-27\|RF2/JH4 | ............<br>............<br>........ | N......... | ........<br>...... | ........<br>E...H...<br>.... | ..........T......<br>S............S. | E..M.............<br>........NG.. | ......<br>..... |
| iPS:43 4207 | 21-225_60A3 | VH3\|3-33/D7\|7-27\|RF2/JH4 | ............<br>............<br>........ | .......... | ........<br>...... | ....E...<br>E.......<br>.... | .................<br>............... | E..M.............<br>........TG.. | ......<br>..... |
| iPS:43 4253 | 21-225_62E4 | VH3\|3-33/D7\|7-27\|RF2/JH4 | ............<br>............<br>........ | D......... | ........<br>...... | ........<br>R.......<br>.... | .................<br>....V......H... | E..F.............<br>........SS.. | ......<br>..... |
| iPS:43 4271 | 21-225_57A4 | VH3\|3-33/D7\|7-27\|RF2/JH4 | ............<br>...........V<br>........ | D......... | ........<br>...... | ....A...<br>......V.<br>.... | .................<br>............... | E..M.............<br>........RS.. | ......<br>..... |
| iPS:43 4293 | 21-225_58F5 | VH3\|3-33/D7\|7-27\|RF2/JH4 | ............<br>...........V<br>........ | D......... | ........<br>...... | ....A...<br>.....HV.<br>.... | .................<br>............... | E..M.............<br>........RS.. | ......<br>..... |
| iPS:43 4337 | 21-225_64E1 | VH3\|3-33/D7\|7-27\|RF2/JH4 | ............<br>...........V<br>........ | .......... | ........<br>...... | ...F....<br>ET....G.<br>.... | .................<br>............... | E..F.............<br>........SS.. | ......<br>..... |
| iPS:43 4357 | 21-225_65C1 | VH3\|3-33/D7\|7-27\|RF2/JH4 | ............<br>............<br>........ | D......... | ........<br>...... | ...FE...<br>....H.T.<br>.... | .................<br>............... | E..F.............<br>........SS.. | ......<br>..... |
| iPS:43 4375 | 21-225_66C7 | VH3\|3-33/D7\|7-27\|RF2/JH4 | ............<br>...........V<br>........ | D......... | ........<br>...... | ...FE...<br>..H...T.<br>.... | .................<br>............... | E..F.............<br>........SS.. | ......<br>..... |
| iPS:43 4411 | 21-225_68F11 | VH3\|3-33/D7\|7-27\|RF2/JH4 | ............<br>............<br>........ | D......... | ........<br>...... | ........<br>V.......<br>.... | .................<br>.............V. | E..M.............<br>........TS.C | ......<br>..... |
| iPS:43 | 21-225_71A2 | VH3\|3-33/D7\|7- | ............<br>............ | D......... | ........<br>...... | ........<br>E....... | .................<br>............... | E..W.............<br>........QD.. | ......<br>..... |

| | | | | | | | | | |
|---|---|---|---|---|---|---|---|---|---|
| iPS:43 4447 | 21-225_71B6 | VH3\|3-33/D7\|7-27\|RF2/J H4 | ............ ............ ........ | N......... | ........ ...... | ........ RT...... .... | ..............H... ............... | E..M.............. ........LS.. | ...... ..... |
| iPS:43 4453 | 21-225_71B11 | VH3\|3-33/D7\|7-27\|RF2/J H4 | ............ ............ ........ | D......... | ........ ...... | ........ RN....G. .... | ................. ............... | E..M.............. ........LS.. | ...... ..... |
| iPS:43 4457 | 21-225_72G12 | VH3\|3-33/D7\|7-27\|RF2/J H4 | ............ ............ ........ | .......... | ......DT ...... | ...F.... E....... .... | ................. ............... | E..F.............. ........SS.. | ...... ..... |
| iPS:43 5311 | 21-225_146H9 | VH3\|3-33/D7\|7-27\|RF2/J H4 | ............ ............ .....S.. | .......... | ........ ...... | ...F.... E...H.G. .... | ................. ............... | E..F.............. ........LS.. | ...... ..... |
| iPS:43 5511 | 21-225_157C3 | VH3\|3-33/D7\|7-27\|RF2/J H4 | ............ ............ ........ | T......... | ........ ...... | ........ VN...... .... | ................. ............... | E..F.............. ........LS.. | ...... ..... |
| iPS:43 5533 | 21-225_157H8 | VH3\|3-33/D7\|7-27\|RF2/J H4 | ............ ............ ........ | .......... | ........ ...... | ........ VN...... .... | ................. ............... | E..F.............. ........LS.. | ...... ..... |
| iPS:43 5551 | 21-225_158H6 | VH3\|3-33/D7\|7-27\|RF2/J H4 | ............ ............ V....... | .......... | ........ ...... | ........ VT...... .... | ................. ....D........V. | E..W.............. ........AE.. | ...... ..... |
| iPS:43 5569 | 21-225_159C5 | VH3\|3-33/D7\|7-27\|RF2/J H4 | ............ ............ ........ | ........I. | ........ ...... | .V...... VN...... .... | ................. ............... | E..F.............. ........LS.. | ...... ..... |
| iPS:43 6268 | 21-225_203B9 | VH3\|3-33/D7\|7-27\|RF2/J H4 | ............ ............ ......L. | ......F... | ........ ...... | ........ VN...... .... | ................. ....P.......... | E..F.............. ........LS.. | ....I. ..... |
| iPS:43 6328 | 21-225_207F12 | VH3\|3-33/D7\|7-27\|RF2/J H4 | ............ ............ ........ | N......... | ........ ...... | ........ RN....G. .... | ................. ............... | E..F.............. ........L... | ...... ..... |
| iPS:43 6556 | 21-225_224D10 | VH3\|3-33/D7\|7-27\|RF2/J H4 | ...........L ....K....... ........ | .......... | ........ ...... | ....E... ......V. ..R. | ..........S...... .............V. | E..F.............. ........QS.. | .....P ..... |
| iPS:39 | 21- | VH3\|3-33/D7\|7- | ............ ............ | D......... | ........ ...... | ........ .N....V. | ................. ............... | E.AW.............. ........YE.. | ...... ..... |

| | | | | | | | | | |
|---|---|---|---|---|---|---|---|---|---|
| iPS:39 2626 | 21-225_18A5 | VH3\|3-33/D7\|7-27\|RF2/JH4 | ............<br>..........YT<br>........ | D......... | ........<br>...... | ...F....<br>........<br>.... | .................<br>............... | D..W..............<br>........TEE. | ......<br>..... |
| iPS:39 2630 | 21-225_20E5 | VH3\|3-33/D7\|7-27\|RF2/JH4 | ............<br>............<br>........ | D......... | ........<br>...... | ....E...<br>EN.Q....<br>.... | .................<br>............... | E..F..............<br>........RS.. | ......<br>..... |
| iPS:39 2640 | 21-225_18A1 | VH3\|3-33/D7\|7-27\|RF2/JH4 | ............<br>............<br>V....... | .......... | ........<br>...... | ....E...<br>EN....V.<br>.... | ..........S......<br>.............V. | E..F..............<br>........QS.. | .....P<br>..... |
| iPS:39 2644 | 21-225_19E1 | VH3\|3-33/D7\|7-27\|RF2/JH4 | ............<br>............<br>........ | N......... | ........<br>...... | ....E...<br>EN.Q....<br>.... | .................<br>............... | E..F..............<br>........RS.. | ......<br>..... |
| iPS:39 2654 | 21-225_17A10 | VH3\|3-33/D7\|7-27\|RF2/JH4 | ............<br>............<br>........ | N......... | ......A.<br>...... | ....E...<br>EN.Q....<br>.... | .................<br>............... | E..F..............<br>........RS.. | ......<br>..... |
| iPS:39 2658 | 21-225_18E8 | VH3\|3-33/D7\|7-27\|RF2/JH4 | ............<br>............<br>........ | N......... | ........<br>...... | ....E...<br>EN.Q....<br>.... | ...V........F...<br>............... | E..F..............<br>........RS.. | ......<br>..... |
| iPS:39 2666 | 21-225_16F11 | VH3\|3-33/D7\|7-27\|RF2/JH4 | ...M........<br>...........E<br>........ | .......... | ........<br>...... | ....E...<br>EN....V.<br>.... | ..........S......<br>.............V. | E..F..............<br>........QS.. | .....P<br>..... |
| iPS:39 2674 | 21-225_18C2 | VH3\|3-33/D7\|7-27\|RF2/JH4 | ............<br>...........V<br>........ | D......... | ........<br>....M. | ........<br>VT......<br>.... | .................<br>...S........... | E..W..............<br>........YE.. | ......<br>..... |
| iPS:39 2680 | 21-225_20A7 | VH3\|3-33/D7\|7-27\|RF2/JH4 | ............<br>............<br>........ | N......... | ........<br>...... | ....E...<br>EN.Q....<br>.... | .................<br>.........I..... | E..F..............<br>........RS.. | ......<br>..... |
| iPS:39 2686 | 21-225_17C7 | VH3\|3-33/D7\|7-27\|RF2/JH4 | ............<br>....K.......<br>........ | D......... | ........<br>...... | ...F....<br>........<br>.... | .................<br>......G........ | D..W..............<br>........TEE. | ......<br>..... |
| iPS:39 2690 | 21-225_18F2 | VH3\|3-33/D7\|7-27\|RF2/JH4 | ............<br>............<br>........ | D......... | ........<br>...... | ...F....<br>......V.<br>.... | .................<br>....V.......... | D..W..............<br>........TEE. | ......<br>..... |
| iPS:39 | 21-225_17B5 | VH3\|3-33/D7\|7- | ............<br>............ | D......... | ........<br>...... | ........<br>E...H.I. | .................<br>.............V. | E..F..............<br>........R... | ......<br>..... |

| | | | | | | | | | |
|---|---|---|---|---|---|---|---|---|---|
| iPS:39 2732 | 21-225_17E5 | VH3\|3-33/D7\|7-27\|RF2/JH4 | ............ ............ ........ | D......... | ........ ...... | L....... VT....G. .... | .........Q......L ............... | E..W............. ........YE.. | ...... ..... |
| iPS:39 2744 | 21-225_20D5 | VH3\|3-33/D7\|7-27\|RF2/JH4 | ............ ............ ........ | .......... | ........ ...... | ....E... EN.Q.... .... | ................. .....D......... | E..F............. ........RS.. | ...... ..... |
| iPS:39 2758 | 21-225_21G11 | VH3\|3-33/D7\|7-27\|RF2/JH4 | ............ ............ ........ | D......... | ........ ....M. | ........ VT.E.... .... | ................. ............... | E..W............. ........YE.. | ...... ..... |
| iPS:39 2772 | 21-225_20E12 | VH3\|3-33/D7\|7-27\|RF2/JH4 | ............ ............ ........ | .......... | ........ ...... | .M...... E...H... .... | .........R....... ............... | E..F............. ........R... | ...... ..... |
| iPS:39 2790 | 21-225_20D10 | VH3\|3-33/D7\|7-27\|RF2/JH4 | ............ ............ ........ | D......... | ........ ...... | ...F.... ......V. .... | .......D......... ............... | D..W............. ........TEE. | ...... ..... |
| iPS:39 2796 | 21-225_22A4 | VH3\|3-33/D7\|7-27\|RF2/JH4 | ............ ............ ........ | D.......I. | ........ ...... | ...F.... ........ .... | .I............... ............... | D..W............. ........TEE. | ...... ..... |
| iPS:39 2810 | 21-225_20H12 | VH3\|3-33/D7\|7-27\|RF2/JH4 | ............ ............ ........ | N......... | ........ ...... | ........ EN....V. .... | ................. ............... | E..F............. ........RS.. | ...... ..... |
| iPS:39 2832 | 21-225_21H8 | VH3\|3-33/D7\|7-27\|RF2/JH4 | ............ ..S......... ........ | D......... | ........ ...... | ...F.... ........ .... | ................. ............... | D..W............. ........TEE. | ...... ..... |
| iPS:39 2854 | 21-225_21E5 | VH3\|3-33/D7\|7-27\|RF2/JH4 | ............ ...........S ........ | D......... | ........ ...... | ........ E....... .... | ................. .........M...T. | E..F............. ........RS.. | ...... ..... |
| iPS:39 2860 | 21-225_22H8 | VH3\|3-33/D7\|7-27\|RF2/JH4 | ............ ............ ........ | .......... | ........ ...... | ........ .N...... .... | ................. ............... | E.AW............. ........YE.. | ...... ..... |
| iPS:39 2866 | 21-225_23H11 | VH3\|3-33/D7\|7-27\|RF2/JH4 | .E.......... ............ ........ | .......... | ........ ...... | ........ EN....V. .... | ................. ............... | E..F............. ........RS.. | ...... ..... |
| iPS:39 | 21-225_21F7 | VH3\|3-33/D7\|7- | ............ ............ | D......... | ........ ...... | ...F.... .N....V. | ................. ............... | D..W............. ........TEE. | ...... ..... |

| | | | | | | | | | |
|---|---|---|---|---|---|---|---|---|---|
| iPS:39 2880 | 21-225_22F9 | VH3\|3-33/D7\|7-27\|RF2/JH4 | ...M........ ............ ........ | .......... | ........ ...... | ....E... EN..D.V. .... | ..........S...... ..............V. | E..F............. ........QS.. | .....P ..... |
| iPS:39 2894 | 21-225_21G2 | VH3\|3-33/D7\|7-27\|RF2/JH4 | ............ ............ ........ | D......... | ........ ....M. | ........ VT...... .... | ...............E. ............... | E..W............. ........YE.. | ...... ..... |
| iPS:39 2900 | 21-225_22F2 | VH3\|3-33/D7\|7-27\|RF2/JH4 | ............ ............ ........ | D......... | ........ ...... | ....E... ......V. ..R. | ..........S...... ..............V. | E..F............. ........QS.. | .....P ..... |
| iPS:39 2908 | 21-225_23F12 | VH3\|3-33/D7\|7-27\|RF2/JH4 | ............ ...........V ........ | .......... | ........ ...... | ........ ET...... .... | ................. .........I..... | E.AW............. ........YE.. | ....S. ..... |
| iPS:39 2918 | 21-225_28F5 | VH3\|3-33/D7\|7-27\|RF2/JH4 | ............ ............ ........ | .......... | ........ ...... | ........ EN...... .... | ................. ............... | E..W............. ........YD.. | ...... ..... |
| iPS:39 2934 | 21-225_27D5 | VH3\|3-33/D7\|7-27\|RF2/JH4 | ............ ............ ........ | .......... | ........ ...... | ........ E.....G. .... | ...........M..... ....G....L..... | E..F............. ........LS.. | ...... ..... |
| iPS:39 2958 | 21-225_28C7 | VH3\|3-33/D7\|7-27\|RF2/JH4 | ............ ............ ........ | .......... | ........ ...... | ........ E....... .... | ................. ............... | E..W............. ........YD.. | ...... ..... |
| iPS:39 2968 | 21-225_25B6 | VH3\|3-33/D7\|7-27\|RF2/JH4 | ............ ........V..T ......LR | N......... | ........ ...... | ....E... E.....TE .... | ................. ............... | E..F............. ........LS.. | ...... ..... |
| iPS:39 2972 | 21-225_26A2 | VH3\|3-33/D7\|7-27\|RF2/JH4 | ............ ............ ........ | .......... | ........ ...... | ....E... ......V. .... | ................. ............... | E..W............. ........YD.. | ...... ..... |
| iPS:39 2980 | 21-225_29H6 | VH3\|3-33/D7\|7-27\|RF2/JH4 | ............ ............ ........ | D......... | ........ .....T | ....N... EN...... .... | ................. ............... | E..M............. ........TG.S | ...... ..... |
| iPS:39 2988 | 21-225_25E6 | VH3\|3-33/D7\|7-27\|RF2/JH4 | ............ ............ ........ | D......... | ........ ...... | ........ EN...... .... | ................. .........L....T | E..M............. ........TG.S | ...... ..... |
| iPS:39 | 21- | VH3\|3-33/D7\|7- | ............ ............ | D......... | ........ ...... | ........ E....... | ................. ............... | E..M............. ........TG.. | ...... ..... |

| | | | | | | | | | |
|---|---|---|---|---|---|---|---|---|---|
| **iPS:39 3000** | **21-225_29D7** | **VH3\|3-33/D7\|7-27\|RF2/JH4** | ............<br>............<br>........ | .......... | ........<br>...... | ........<br>E.....G.<br>.... | .................<br>....G....L..... | E..F.............<br>........LS.. | ......<br>..... |
| **iPS:39 3018** | **21-225_29B8** | **VH3\|3-33/D7\|7-27\|RF2/JH4** | ............<br>............<br>........ | D......... | ........<br>...... | ........<br>EN......<br>.... | .................<br>............... | E..M.............<br>........TG.S | ......<br>..... |
| **iPS:39 3030** | **21-225_25H11** | **VH3\|3-33/D7\|7-27\|RF2/JH4** | ............<br>............<br>........ | D......... | ........<br>...... | ........<br>EN.E....<br>.... | .................<br>............... | E..M.............<br>........TG.S | ......<br>..... |
| **iPS:39 3034** | **21-225_27F2** | **VH3\|3-33/D7\|7-27\|RF2/JH4** | .E..........<br>............<br>.....I.. | D......... | ........<br>...... | ........<br>EN....V.<br>..R. | .................<br>............... | E..M.............<br>........TG.S | ......<br>..... |
| **iPS:39 3048** | **21-225_27C3** | **VH3\|3-33/D7\|7-27\|RF2/JH4** | ............<br>............<br>........ | D......... | ........<br>...... | ........<br>EN..S...<br>.... | .................<br>............... | E..M.............<br>........TG.S | ......<br>..... |
| **iPS:39 3054** | **21-225_29G8** | **VH3\|3-33/D7\|7-27\|RF2/JH4** | ............<br>............<br>........ | D......... | ........<br>...... | ........<br>ET......<br>.... | .................<br>............... | E..M.............<br>........TS.. | ......<br>..... |
| **iPS:39 3812** | **21-225_6A11** | **VH3\|3-33/D7\|7-27\|RF2/JH4** | ............<br>............<br>........ | D......... | ........<br>...... | ...F....<br>........<br>.... | .................<br>...........D... | D..W.............<br>........TEE. | ......<br>..... |
| **iPS:39 3818** | **21-225_6G12** | **VH3\|3-33/D7\|7-27\|RF2/JH4** | ............<br>............<br>........ | .......... | ........<br>...... | ........<br>R..N....<br>.... | .................<br>............... | E..F.............<br>........RS.. | ......<br>..... |
| **iPS:39 3820** | **21-225_8H7** | **VH3\|3-33/D7\|7-27\|RF2/JH4** | ............<br>...........P<br>........ | D.....F... | ........<br>...... | ....E...<br>EN.Q....<br>.... | .................<br>............... | E..F.............<br>........RS.. | ......<br>..... |
| **iPS:39 3826** | **21-225_10G5** | **VH3\|3-33/D7\|7-27\|RF2/JH4** | ............<br>............<br>........ | N......... | ........<br>...... | ....E...<br>EN.Q....<br>.... | .................<br>.............T. | E..F.............<br>........RS.. | ......<br>..... |
| **iPS:39 3828** | **21-225_10H12** | **VH3\|3-33/D7\|7-27\|RF2/JH4** | ............<br>............<br>........ | D......... | ........<br>...... | ....E...<br>DN.Q....<br>.... | .................<br>............... | E..F.............<br>........RS.. | ......<br>..... |
| **iPS:39** | **21-225_12A1** | **VH3\|3-33/D7\|7-** | ............<br>............ | .......... | ........<br>...... | ....E...<br>EN.Q.... | .................<br>............... | E..F.............<br>........RS.. | ......<br>..... |

| | | | | | | | | | |
|---|---|---|---|---|---|---|---|---|---|
| iPS:39 3838 | 21-225_6G2 | VH3\|3-33/D7\|7-27\|RF2/J H4 | L...........<br>....K.......<br>........ | D.......I. | ........<br>...... | ...F....<br>........<br>.... | .....S...........<br>............... | D..W.............<br>........TEE. | ......<br>..... |
| iPS:39 3854 | 21-225_7H11 | VH3\|3-33/D7\|7-27\|RF2/J H4 | ............<br>...........S<br>........ | D......... | ........<br>...... | ........<br>EN......<br>.... | .................<br>............... | E..F.............<br>........RS.. | ......<br>..... |
| iPS:39 3866 | 21-225_14E3 | VH3\|3-33/D7\|7-27\|RF2/J H4 | ............<br>............<br>........ | D.....F... | ........<br>...... | ....E...<br>EN.Q....<br>.... | .................<br>.........F..... | E..F.............<br>........RS.. | ......<br>..... |
| iPS:39 3876 | 21-225_9A1 | VH3\|3-33/D7\|7-27\|RF2/J H4 | ...V........<br>............<br>........ | D......... | ........<br>...... | ...F....<br>........<br>.... | .................<br>............... | D..W.............<br>........TEE. | .....P<br>..... |
| iPS:39 3882 | 21-225_15E3 | VH3\|3-33/D7\|7-27\|RF2/J H4 | ..........T.<br>............<br>........ | .......... | ........<br>...... | ....E...<br>EN..H...<br>.... | .................<br>............... | E..F.............<br>........LS.. | ......<br>..... |
| iPS:39 3884 | 21-225_16F4 | VH3\|3-33/D7\|7-27\|RF2/J H4 | ............<br>............<br>........ | N......... | ........<br>...... | ....E...<br>...Q..G.<br>.... | ............V....<br>H.............. | E..F.............<br>........LS.. | ......<br>..... |
| iPS:39 3912 | 21-225_16F6 | VH3\|3-33/D7\|7-27\|RF2/J H4 | ............<br>............<br>........ | N......... | R.......<br>...... | ....E...<br>...Q..G.<br>.... | ............V....<br>H.............. | E..F.............<br>........LS.. | ......<br>..... |
| iPS:39 3922 | 21-225_2B2 | VH3\|3-33/D7\|7-27\|RF2/J H4 | ............<br>............<br>........ | .......... | ........<br>...... | ....E...<br>EN....V.<br>.... | ..........S......<br>.............V. | E..F.............<br>........QS.. | .....P<br>..... |
| iPS:39 3934 | 21-225_13E6 | VH3\|3-33/D7\|7-27\|RF2/J H4 | ............<br>............<br>........ | N......... | ........<br>...... | ........<br>EN....I.<br>.... | .................<br>............... | E..F.............<br>........RS.. | ......<br>..... |
| iPS:39 3948 | 21-225_16A5 | VH3\|3-33/D7\|7-27\|RF2/J H4 | ............<br>............<br>........ | D......... | ........<br>...... | ...F....<br>......V.<br>.... | .................<br>S.............. | D..W.............<br>........TEE. | ......<br>..... |
| iPS:39 3960 | 21-225_7G2 | VH3\|3-33/D7\|7-27\|RF2/J H4 | ............<br>............<br>........ | D......... | ........<br>....M. | ........<br>VT......<br>.... | ................L<br>............... | E..W.............<br>........YE.. | ......<br>..... |
| iPS:39 | 21-225_7C4 | VH3\|3-33/D7\|7- | ............<br>....K....... | N......... | ........<br>...... | ....E...<br>EN.Q.... | .................<br>............... | E..F.............<br>........RS.. | ......<br>..... |

| | | | | | | | | | |
|---|---|---|---|---|---|---|---|---|---|
| iPS:39 3976 | 21-225_7E9 | VH3\|3-33/D7\|7-27\|RF2/JH4 | ............ ............ ........ | D......... | ........ ...... | ........ EN....V. .... | ................. ............... | E..F............... ........RS.. | ...... ..... |
| iPS:39 3994 | 21-225_8C9 | VH3\|3-33/D7\|7-27\|RF2/JH4 | ............ ............ ........ | .......... | ........ ...... | ........ EN....V. .... | ................. ............... | E..F............... ........RS.. | ...... ..... |
| iPS:39 3998 | 21-225_12B12 | VH3\|3-33/D7\|7-27\|RF2/JH4 | ............ ............ ........ | D......... | ........ ....M. | ........ VT...... .... | ................. ............... | E..W............... ........YE.. | ...... ..... |
| iPS:39 4024 | 21-225_16B7 | VH3\|3-33/D7\|7-27\|RF2/JH4 | ............ ............ ........ | .......... | ........ ...... | ........ E....... .... | ................. ............... | E..F............... ........LS.. | ...... ..... |
| iPS:39 4059 | 21-225_9E8 | VH3\|3-33/D7\|7-27\|RF2/JH4 | ............ ...........E ........ | .......... | ........ ...... | ....E... EN.Q.... .... | ................. ............... | E..F............... ........RS.. | ...... ..... |
| iPS:39 4067 | 21-225_12F2 | VH3\|3-33/D7\|7-27\|RF2/JH4 | ............ ............ ........ | D......... | ........ ...... | ...F.... .N....V. .... | .............F... .............V. | E.AW............... ........SE.. | ...... ..... |
| iPS:39 4089 | 21-225_12E6 | VH3\|3-33/D7\|7-27\|RF2/JH4 | ............ ............ ........ | D......... | ........ .....T | ........ E....... .... | .......D......... ............... | E.AW............... ........YE.. | ...... ..... |
| iPS:39 4097 | 21-225_16G7 | VH3\|3-33/D7\|7-27\|RF2/JH4 | ............ ............ .......T | D......... | ........ ...... | ........ EN.E.... .... | ......A.......... ............... | E.AW............... ........YE.. | ...... ..... |
| iPS:40 2219 | 21-225_1C12 | VH3\|3-33/D7\|7-27\|RF2/JH4 | ............ ............ ........ | N......... | ........ ...... | ........ EN....V. .... | ................. ............... | E..F............... ........RS.. | ...... ..... |
| | | Germline | H_FR1 | H_CDR1 | H_FR2 | H_CDR2 | H_FR3 | H_CDR3 | H_FR4 |
| | VH3\|3-21/D4\|4-11\|RF2/JH4 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | DYSNY---------------------YFDY | WGQGTLVTVSS |
| iPS:43 3895 | 21-225_43E1 | VH3\|3-21/D4\|4-11\|RF2/JH4 | ............ ............ ........ | .......... | ........ ...... | A..GN... .T...... .L.. | .............F..L ............... | .RG-- .................. ...--SE | ...... ..... |

| | | | | | | | | | |
|---|---|---|---|---|---|---|---|---|---|
| iPS:43 4103 | 21-225_53G1 | VH3\|3-21/D4\|4-11\|RF2/JH4 | ............<br>....E.......<br>........ | ........... | ........<br>...... | ...G....<br>........<br>.... | .................<br>............... | .RG--<br>...................<br>...--ST | ......<br>..... |
| iPS:43 4179 | 21-225_56F1 | VH3\|3-21/D4\|4-11\|RF2/JH4 | ............<br>..F.........<br>........ | ........... | ........<br>...... | ........<br>.T....G.<br>.... | .................<br>............... | .RG--<br>...................<br>...--SS | ......<br>..... |
| iPS:43 4263 | 21-225_56H7 | VH3\|3-21/D4\|4-11\|RF2/JH4 | ............<br>............<br>.....S.. | ........... | ........<br>...... | ........<br>.T....G.<br>.... | .................<br>............... | .RG--<br>...................<br>...--SS | ......<br>..... |
| iPS:43 5521 | 21-225_157H4 | VH3\|3-21/D4\|4-11\|RF2/JH4 | ............<br>............<br>........ | ........... | ........<br>...... | ........<br>.T......<br>.... | .................<br>............... | .RG--<br>...................<br>...--SI | ......<br>..... |
| iPS:43 5527 | 21-225_157G7 | VH3\|3-21/D4\|4-11\|RF2/JH4 | ............<br>........F...<br>........ | ........... | ........<br>...... | ...G....<br>.T......<br>.... | .................<br>............... | .RG--<br>...................<br>...--SS | ......<br>..... |
| iPS:43 5529 | 21-225_157H7 | VH3\|3-21/D4\|4-11\|RF2/JH4 | ............<br>............<br>........ | ........... | ........<br>...... | C..G....<br>........<br>.... | ...M.............<br>.............V. | .RG--<br>...................<br>...--G. | ......<br>..... |
| iPS:43 5547 | 21-225_158F5 | VH3\|3-21/D4\|4-11\|RF2/JH4 | ............<br>........F...<br>........ | ........... | ........<br>...... | ...G....<br>.T......<br>.... | .................<br>............... | .RG--<br>...................<br>...--SS | ......<br>..... |
| iPS:43 5549 | 21-225_158H5 | VH3\|3-21/D4\|4-11\|RF2/JH4 | ............<br>............<br>........ | ........... | ........<br>...... | ...G....<br>.T......<br>.... | .............H...<br>............... | .RG--<br>...................<br>...--SS | ......<br>..... |
| iPS:43 5553 | 21-225_158G8 | VH3\|3-21/D4\|4-11\|RF2/JH4 | ............<br>.T..........<br>........ | ........T.. | ........<br>...... | L..G....<br>........<br>.... | .................<br>............... | .RG--<br>...................<br>...--SL | ......<br>..... |
| iPS:43 5581 | 21-225_160H1 | VH3\|3-21/D4\|4-11\|RF2/JH4 | ............<br>............<br>........ | ........R.. | ........<br>...... | ........<br>TG.M....<br>.... | .................<br>...........F... | .K---<br>...................<br>...--.. | ......<br>..... |
| iPS:43 5593 | 21-225_160F4 | VH3\|3-21/D4\|4-11\|RF2/JH4 | ............<br>........F...<br>........ | ........... | ........<br>...... | ...G....<br>.T......<br>.... | .................<br>............... | .RG--<br>...................<br>...--SS | ......<br>..... |
| iPS:43 | 21- | VH3\|3-21/D4\|4- | ............<br>........F... | ........... | ........<br>...... | ...G....<br>.T...... | .................<br>............... | .RG--<br>................... | ......<br>..... |

| | | | | | | | | | |
|---|---|---|---|---|---|---|---|---|---|
| iPS:43 5621 | 21-225_162H3 | VH3\|3-21/D4\|4-11\|RF2/JH4 | ............<br>........F...<br>........ | .......... | R.......<br>...... | ...G....<br>.T......<br>.... | .................<br>............... | .RG--<br>...................<br>...--SS | ......<br>..... |
| iPS:43 5641 | 21-225_163F9 | VH3\|3-21/D4\|4-11\|RF2/JH4 | ............<br>.Q..........<br>........ | .......... | ........<br>...... | ...G....<br>.T......<br>.... | .................<br>............... | .RG--<br>...................<br>...--SL | ......<br>..... |
| iPS:43 5719 | 21-225_171A11 | VH3\|3-21/D4\|4-11\|RF2/JH4 | ............<br>............<br>........ | .......... | ........<br>...... | ...G....<br>........<br>.... | .................<br>............... | .RG--<br>...................<br>...--SS | ....I.<br>..... |
| iPS:43 6856 | 21-225_58C5 | VH3\|3-21/D4\|4-11\|RF2/JH4 | ............<br>............<br>.......N | .......... | ........<br>....I. | ........<br>.Y.L....<br>.... | .......S.........<br>............... | T..G-<br>...................<br>...S... | ......<br>..... |
| iPS:44 8904 | 21-225_65C12 | VH3\|3-21/D4\|4-11\|RF2/JH4 | ............<br>............<br>.......R | ......F.L. | ........<br>...... | ........<br>........<br>.... | .................<br>............... | .AY--<br>...................<br>...-SH. | ......<br>..... |
| iPS:47 2730 | 21-225_14B1_LC1 | VH3\|3-21/D4\|4-11\|RF2/JH4 | ............<br>............<br>........ | .......T.. | ........<br>...... | ...G....<br>...L..P.<br>.... | .................<br>............... | .RG--<br>...................<br>...--SS | ......<br>..... |
| iPS:47 2731 | 21-225_14B1_LC2 | VH3\|3-21/D4\|4-11\|RF2/JH4 | ............<br>............<br>........ | .......T.. | ........<br>...... | ...G....<br>...L..P.<br>.... | .................<br>............... | .RG--<br>...................<br>...--SS | ......<br>..... |
| iPS:39 2726 | 21-225_20B5 | VH3\|3-21/D4\|4-11\|RF2/JH4 | ......A.....<br>............<br>.......T | .......... | ........<br>...... | ...G....<br>........<br>.... | .................<br>............... | .RG--<br>...................<br>...--S. | ......<br>..... |
| iPS:39 2734 | 21-225_17D8 | VH3\|3-21/D4\|4-11\|RF2/JH4 | ............<br>............<br>........ | .......GL. | ........<br>...... | ...G....<br>G.H.S...<br>.... | ................L<br>............... | .RG--<br>...................<br>...--SG | ......<br>..... |
| iPS:39 2768 | 21-225_20B8 | VH3\|3-21/D4\|4-11\|RF2/JH4 | ............<br>............<br>........ | .......... | ........<br>...... | ...G....<br>G.H.....<br>.... | .................<br>............... | .RG--<br>...................<br>...--SG | ......<br>..... |
| iPS:39 2778 | 21-225_22H3 | VH3\|3-21/D4\|4-11\|RF2/JH4 | A...........<br>............<br>........ | .......... | ........<br>...... | ........<br>........<br>.... | .................<br>..........F.... | .RG--<br>...................<br>...--SL | ......<br>..... |
| iPS:39 | 21-225_20C8 | VH3\|3-21/D4\|4- | ............<br>............ | .......... | ........<br>...... | ...G....<br>........ | .................<br>............... | .RG--<br>................... | ......<br>..... |

| | | | | | | | | | |
|---|---|---|---|---|---|---|---|---|---|
| iPS:39 2792 | 21-225_20G12 | VH3\|3-21/D4\|4-11\|RF2/JH4 | ............<br>............<br>........ | .......... | ........<br>...... | ...G....<br>........<br>.L.. | .................<br>............... | .RG--<br>...................<br>...--S. | ......<br>..... |
| iPS:39 2844 | 21-225_23E11 | VH3\|3-21/D4\|4-11\|RF2/JH4 | ............<br>............<br>........ | .......T.. | ........<br>...... | ...G....<br>....W.V.<br>.... | .................<br>............... | .RG--<br>...................<br>...--SL | ......<br>..... |
| iPS:39 2848 | 21-225_20F9 | VH3\|3-21/D4\|4-11\|RF2/JH4 | ............<br>............<br>........ | .......... | ........<br>...... | ........<br>........<br>.... | .................<br>............... | .RG--<br>...................<br>...--SC | ......<br>..I.. |
| iPS:39 2850 | 21-225_20H10 | VH3\|3-21/D4\|4-11\|RF2/JH4 | ............<br>............<br>........ | T......... | ........<br>...... | ........<br>........<br>.... | .................<br>.........I..... | .RG--<br>...................<br>...--SL | ......<br>..... |
| iPS:39 3006 | 21-225_31G9 | VH3\|3-21/D4\|4-11\|RF2/JH4 | ............<br>............<br>........ | .......... | ........<br>...... | ........<br>........<br>.... | .................<br>............... | .RG--<br>...................<br>...--SS | ......<br>..... |
| iPS:39 3022 | 21-225_30H11 | VH3\|3-21/D4\|4-11\|RF2/JH4 | ............<br>............<br>........ | .......... | ........<br>...... | ...G....<br>........<br>.... | .................<br>............... | .RG--<br>...................<br>...--SL | ......<br>..... |
| iPS:39 3130 | 21-225_33C2 | VH3\|3-21/D4\|4-11\|RF2/JH4 | ............<br>............<br>........ | .......T.. | ........<br>..Q... | ...G....<br>........<br>.... | ..............F...<br>.........IF.... | .RG--<br>...................<br>...--GT | ......<br>..... |
| iPS:39 3906 | 21-225_13D3 | VH3\|3-21/D4\|4-11\|RF2/JH4 | ............<br>............<br>........ | .......T.. | ........<br>..D... | ...G....<br>........<br>.... | .................<br>............... | .RG--<br>...................<br>...--SG | ......<br>..... |
| iPS:39 3982 | 21-225_6C12 | VH3\|3-21/D4\|4-11\|RF2/JH4 | ............<br>............<br>........ | .......... | ........<br>...... | ........<br>........<br>.... | .................<br>............... | .RG--<br>...................<br>...--SL | ......<br>..... |
| iPS:39 8478 | 21-225_17C10 | VH3\|3-21/D4\|4-11\|RF2/JH4 | ............<br>............<br>........ | .......... | ........<br>...... | ...G....<br>...M....<br>.... | .................<br>.........L..... | .RG--<br>...................<br>...--SS | ......<br>..... |
| | | Germline | H_FR1 | H_CDR1 | H_FR2 | H_CDR2 | H_FR3 | H_CDR3 | H_FR4 |
| | VH3\|3-23/D6\|6-6\|RF1/JH4 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | EYSSS-----------<br>-------SYFDY | WGQGTL<br>VTVSS |

| | | | | | | | | | |
|---|---|---|---|---|---|---|---|---|---|
| iPS:43 3897 | 21-225_43C2 | VH3\|3-23/D6\|6-6\|RF1/JH4 | ............<br>............<br>........ | .......... | ........<br>...... | ....R...<br>.VN.FD..<br>.... | .................<br>............... | .R.G.............<br>.......–.... | ......<br>..... |
| iPS:43 3903 | 21-225_43H4 | VH3\|3-23/D6\|6-6\|RF1/JH4 | ............<br>............<br>........ | .......... | ........<br>...... | ....R...<br>.IN.FD..<br>.... | .................<br>............... | .R.G.............<br>.......–.... | ......<br>..... |
| iPS:43 3911 | 21-225_43E8 | VH3\|3-23/D6\|6-6\|RF1/JH4 | ............<br>............<br>........ | .......... | ........<br>...... | ....R...<br>.IN.FD..<br>.... | .................<br>............... | .R.G.............<br>.......–.... | ......<br>..... |
| iPS:43 3941 | 21-225_44D10 | VH3\|3-23/D6\|6-6\|RF1/JH4 | ............<br>............<br>........ | .......... | ........<br>...... | ....R...<br>.VN.FD..<br>.... | .................<br>............... | .R.G.............<br>.......–.... | ......<br>..... |
| iPS:43 3957 | 21-225_45F8 | VH3\|3-23/D6\|6-6\|RF1/JH4 | ............<br>............<br>........ | .......... | ........<br>...... | ....R...<br>.VN.FD..<br>.... | .................<br>............... | .R.G.............<br>.......–.... | ......<br>..... |
| iPS:43 3973 | 21-225_46A6 | VH3\|3-23/D6\|6-6\|RF1/JH4 | ............<br>............<br>........ | .......... | ........<br>...... | ....R...<br>.IN.FD..<br>.... | .................<br>............... | .R.G.............<br>.......–.... | ......<br>..... |
| iPS:43 5715 | 21-225_171A8 | VH3\|3-23/D6\|6-6\|RF1/JH4 | ............<br>............<br>.......R | ......S... | ........<br>...... | V.......<br>....F.T.<br>.... | .................<br>............... | SN..G...........<br>.......-W... | ......<br>..... |
| iPS:43 5739 | 21-225_174G7 | VH3\|3-23/D6\|6-6\|RF1/JH4 | ............<br>............<br>.......R | ......S... | ........<br>...... | V.......<br>....F.T.<br>.... | .................<br>............... | SN..G...........<br>.......-W... | ......<br>..... |
| iPS:43 5749 | 21-225_175C10 | VH3\|3-23/D6\|6-6\|RF1/JH4 | ............<br>............<br>........ | .......... | ........<br>...... | S...R...<br>....F...<br>.... | ...V.............<br>............... | SN..G...........<br>.......-W... | ......<br>..... |
| | | Germline | H_FR1 | H_CDR1 | H_FR2 | H_CDR2 | H_FR3 | H_CDR3 | H_FR4 |
| | VH3\|3-21/D7\|7-27\|RF1/JH4 | | EVQLVES-<br>GGGLVKPGGSLR<br>LSCAASG-FTFS | S-----YSMN | WVRQAPGK<br>GLEWVS | SISSS---<br>SSYIYYAD<br>SVKG | RFTISRDNAKNSLYLQM<br>NSLRAEDTAVYYCAR | LTGY-------------<br>---------FDY | WGQGTL<br>VTVSS |
| iPS:43 3901 | 21-225_43A4 | VH3\|3-21/D7\|7-27\|RF1/JH4 | ............<br>............<br>........ | ........T.. | ....V...<br>...... | ...G....<br>.T......<br>.... | .......D.Q.......<br>....G.......... | V.S–<br>..................<br>........ | ....A.<br>..... |

| | | | | | | | | | |
|---|---|---|---|---|---|---|---|---|---|
| iPS:43 3961 | 21-225_45D9 | VH3\|3-21/D7\|7-27\|RF1/JH4 | ............<br>............<br>........ | .......T.. | ....V...<br>...... | ...G....<br>.T......<br>.... | .......D.Q.......<br>....G.......... | V.S-<br>..................<br>........ | ....A.<br>..... |
| iPS:43 4135 | 21-225_54H3 | VH3\|3-21/D7\|7-27\|RF1/JH4 | ............<br>............<br>........ | .........I | ........<br>...... | ...GT...<br>........<br>.... | .................<br>..............G | M.T-<br>..................<br>.....-VI | ......<br>..... |
| iPS:43 4331 | 21-225_63H8 | VH3\|3-21/D7\|7-27\|RF1/JH4 | ............<br>............<br>........ | .......N.. | ........<br>...... | ...G....<br>.T.MN.T.<br>.... | .................<br>............... | .RN-<br>..................<br>........ | ......<br>..... |
| iPS:43 5421 | 21-225_151F1 | VH3\|3-21/D7\|7-27\|RF1/JH4 | ............<br>............<br>....Y..R | ......F... | ........<br>...... | ........<br>.Y......<br>.... | .................<br>............... | D.P-<br>..................<br>.....LV. | ......<br>..... |
| iPS:43 5653 | 21-225_166H12 | VH3\|3-21/D7\|7-27\|RF1/JH4 | ..........A.<br>............<br>........ | .........S | ........<br>..G... | ...G....<br>...S....<br>.... | .................<br>............... | ...-<br>..................<br>........ | ......<br>..... |
| iPS:43 6648 | 21-225_227F11 | VH3\|3-21/D7\|7-27\|RF1/JH4 | ............<br>............<br>V....... | T......... | ........<br>...... | ........<br>IN.M....<br>.... | .................<br>.......S....... | .G--<br>..................<br>.....-V. | ......<br>..... |
| iPS:39 2952 | 21-225_26G1 | VH3\|3-21/D7\|7-27\|RF1/JH4 | ............<br>............<br>........ | .......G.. | ........<br>...... | ...G....<br>........<br>.... | .................<br>............... | ..T-<br>..................<br>.......F | ......<br>..... |
| iPS:39 3082 | 21-225_34C11 | VH3\|3-21/D7\|7-27\|RF1/JH4 | ............<br>............<br>........ | .......T.S | ........<br>...... | ...G....<br>.N......<br>.... | .................<br>............... | ...-<br>..................<br>........ | ......<br>..... |
| iPS:39 4061 | 21-225_12D2 | VH3\|3-21/D7\|7-27\|RF1/JH4 | ............<br>............<br>........ | .......... | ........<br>...... | ........<br>........<br>.... | .................<br>............... | .G--<br>..................<br>.....-.. | ......<br>.A... |
| iPS:39 4071 | 21-225_10C7 | VH3\|3-21/D7\|7-27\|RF1/JH4 | ............<br>............<br>........ | .......... | ........<br>...... | ........<br>NN......<br>.... | .................<br>.......S....... | .G--<br>..................<br>.....-V. | ......<br>..... |
| iPS:39 8532 | 21-225_33B7 | VH3\|3-21/D7\|7-27\|RF1/JH4 | ............<br>............<br>........ | .......N.. | ........<br>...... | ...G....<br>........<br>.... | .................<br>............... | .N.-<br>..................<br>........ | ......<br>..... |
| iPS:40 | 21-225_2B1 | VH3\|3-21/D7\|7- | ............<br>............ | .......... | ........<br>...... | ........<br>........ | .................<br>............... | .G--<br>.................. | ......<br>..... |

| | | | | | | | | | |
|---|---|---|---|---|---|---|---|---|---|
| iPS:40 4090 | 21-225_8D8 | VH3\|3-21/D7\|7-27\|RF1/JH4 | .............<br>.............<br>........ | ........... | .........<br>...... | .........<br>.........<br>.... | ...................<br>................ | .G--<br>.....................<br>.....-N. | ......<br>..... |
| | | Germline | H_FR1 | H_CDR1 | H_FR2 | H_CDR2 | H_FR3 | H_CDR3 | H_FR4 |
| | VH3\|3-11/D4\|4-11\|RF2/JH4 | | QVQLVES-<br>GGGLVKPGGSLR<br>LSCAASG-FTFS | D-----YYMS | WIRQAPGK<br>GLEWVS | YISSS---<br>GSTIYYAD<br>SVKG | RFTISRDNAKNSLYLQM<br>NSLRAEDTAVYYCAR | DYSNY-----------<br>--------YFDY | WGQGTL<br>VTVSS |
| iPS:43 3905 | 21-225_43E5 | VH3\|3-11/D4\|4-11\|RF2/JH4 | .............<br>.............<br>........ | .........I | .........<br>...... | .........<br>.I.K....<br>.M.. | ...................<br>...............A | .T---<br>.....................<br>...--I. | ......<br>..... |
| iPS:43 3913 | 21-225_43H8 | VH3\|3-11/D4\|4-11\|RF2/JH4 | .............<br>.............<br>....I... | .........N | .........<br>...... | .........<br>.R..F...<br>.L.. | ...................<br>...............A | .T---<br>.....................<br>...--I. | ......<br>..... |
| iPS:43 3949 | 21-225_45H2 | VH3\|3-11/D4\|4-11\|RF2/JH4 | .............<br>.............<br>........ | .........N | .........<br>...... | .........<br>.I.K....<br>.... | ...................<br>...............A | .T---<br>.....................<br>...--I. | ......<br>..... |
| iPS:43 3981 | 21-225_46E9 | VH3\|3-11/D4\|4-11\|RF2/JH4 | .............<br>.............<br>........ | .........N | .........<br>...... | ..N.N...<br>.F......<br>.... | ...................<br>...............A | .T---<br>.....................<br>...--I. | ......<br>..... |
| iPS:43 3995 | 21-225_47H7 | VH3\|3-11/D4\|4-11\|RF2/JH4 | .............<br>.............<br>........ | .........I | .........<br>...... | ..N.N...<br>.F.K....<br>.... | ...................<br>...............A | .T---<br>.....................<br>...--V. | ......<br>..... |
| iPS:43 4039 | 21-225_43B1 | VH3\|3-11/D4\|4-11\|RF2/JH4 | .............<br>.............<br>........ | .........N | .........<br>...... | ..N.N...<br>.F......<br>.... | ...................<br>...............A | .T---<br>.....................<br>...--I. | .....R<br>..... |
| iPS:43 4275 | 21-225_57F4 | VH3\|3-11/D4\|4-11\|RF2/JH4 | .............<br>.............<br>.....I.. | .........N | .........<br>...... | .........<br>.........<br>.... | ...................<br>................ | .M---<br>.....................<br>...--IT | ......<br>..... |
| | | Germline | H_FR1 | H_CDR1 | H_FR2 | H_CDR2 | H_FR3 | H_CDR3 | H_FR4 |
| | VH3\|3-23/D7\|7-27\|RF1/JH3 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | LTGD------------<br>--------AFDI | WGQGTM<br>VTVSS |
| iPS:43 3915 | 21-225_43H9 | VH3\|3-23/D7\|7-27\|RF1/JH3 | .............<br>.............<br>........ | ........... | ........M<br>...... | .........<br>.SN.F...<br>.... | .................H.<br>............F... | R.PSD...............<br>........V... | ......<br>..... |

| | | | | | | | | | |
|---|---|---|---|---|---|---|---|---|---|
| iPS:43 3925 | 21-225_44F3 | VH3\|3-23/D7\|7-27\|RF1/JH3 | ..H.........<br>............<br>........ | ........... | ........<br>...... | IL..G...<br>.KT.....<br>.... | ..............F...<br>............... | R.PSD............<br>............ | ......<br>..... |
| iPS:43 3953 | 21-225_45H4 | VH3\|3-23/D7\|7-27\|RF1/JH3 | ............<br>............<br>........ | ........... | .......M<br>...... | ........<br>.SN.F...<br>.... | ...............H.<br>...........F... | R.PSD............<br>........V... | ......<br>..... |
| iPS:43 3959 | 21-225_45C9 | VH3\|3-23/D7\|7-27\|RF1/JH3 | ............<br>............<br>.......N | ........... | ........<br>...... | V...R...<br>..T.....<br>.... | .................<br>............... | R.PSD............<br>............ | ......<br>..... |
| iPS:43 5379 | 21-225_149B6 | VH3\|3-23/D7\|7-27\|RF1/JH3 | ............<br>............<br>........ | ........... | ........<br>...... | V.......<br>....F...<br>.... | ..............F...<br>............... | R.PED............<br>........V... | ......<br>..... |
| iPS:43 5787 | 21-225_180A3 | VH3\|3-23/D7\|7-27\|RF1/JH3 | ............<br>E...........<br>........ | ......F..N | ........<br>...... | V...R...<br>..N.F...<br>.... | ..............F...<br>...........F... | R...D............<br>........V..V | ......<br>..... |
| iPS:43 5809 | 21-225_182H5 | VH3\|3-23/D7\|7-27\|RF1/JH3 | ............<br>............<br>........ | ........... | ........<br>...... | V...R...<br>..T.F...<br>.... | .................<br>............... | R...D............<br>........V... | ......<br>..... |
| iPS:43 5889 | 21-225_186A11 | VH3\|3-23/D7\|7-27\|RF1/JH3 | ............<br>............<br>........ | ........... | ........<br>...... | V...R...<br>..T.F...<br>.... | .................<br>............... | R...D............<br>........V... | ......<br>..... |
| | | Germline | H_FR1 | H_CDR1 | H_FR2 | H_CDR2 | H_FR3 | H_CDR3 | H_FR4 |
| | VH4\|4-59/D6\|6-13\|RF2/JH4 | | QVQLQES-<br>GPGLVKPSETLS<br>LTCTVSG-GSIS | S------YYWS | WIRQPPGK<br>GLEWIG | YIYY----<br>SGSTNYNP<br>SLKS | RVTISVDTSKNQFSLKL<br>SSVTAADTAVYYCAR | GIAAA------------<br>-------GYFDY | WGQGTL<br>VTVSS |
| iPS:43 3931 | 21-225_44F6 | VH4\|4-59/D6\|6-13\|RF2/JH4 | ..H.........<br>............<br>........ | ........... | ........<br>...... | ........<br>..N.....<br>.... | .................<br>.............V. | .V.I-<br>.................<br>..--KN. | ....I.<br>..... |
| | | Germline | H_FR1 | H_CDR1 | H_FR2 | H_CDR2 | H_FR3 | H_CDR3 | H_FR4 |
| | VH3\|3-23/D4\|4-17\|RF2/JH6 | | EVQLLES-<br>GGGLVQPGGSLR<br>LSCAASG-FTFS | S------YAMS | WVRQAPGK<br>GLEWVS | AISGS---<br>GGSTYYAD<br>SVKG | RFTISRDNSKNTLYLQM<br>NSLRAEDTAVYYCAK | DYGDYYY----------<br>-----YYYGMDV | WGQGTT<br>VTVSS |
| iPS:43 3943 | 21-225_44E10 | VH3\|3-23/D4\|4-17\|RF2/JH6 | ............<br>..S.........<br>........ | .........N | ........<br>...... | GVV.....<br>..R.....<br>.... | ..I..............<br>............... | .R.QWL-<br>...............-<br>LG.... | ......<br>..... |

| | | | | | | | | | |
|---|---|---|---|---|---|---|---|---|---|
| iPS:433989 | 21-225_47C7 | VH3\|3-23/D4\|4-17\|RF2/JH6 | ............<br>............<br>........ | N......... | ........<br>...... | G.......<br>.SR.....<br>.... | .................<br>............... | .R.QWL-<br>................-<br>IG.... | ......<br>..... |
| iPS:434133 | 21-225_54G3 | VH3\|3-23/D4\|4-17\|RF2/JH6 | ............<br>............<br>........ | T......... | ........<br>...... | ........<br>.VN.F...<br>.... | .............F...<br>............... | LGK...-<br>................-<br>...... | ......<br>..... |
| iPS:434221 | 21-225_60A11 | VH3\|3-23/D4\|4-17\|RF2/JH6 | ............<br>............<br>........ | N........T | ........<br>...... | ........<br>..N.F...<br>..T. | .V...............<br>............... | LGK..H-<br>................-<br>...... | ......<br>..... |
| iPS:434257 | 21-225_62F7 | VH3\|3-23/D4\|4-17\|RF2/JH6 | ............<br>............<br>........ | .......V.. | ........<br>...... | G.......<br>.AK.....<br>.... | .................<br>..............E | LGI...-<br>................--<br>..... | ......<br>..... |
| iPS:434283 | 21-225_57F8 | VH3\|3-23/D4\|4-17\|RF2/JH6 | ............<br>...........V<br>V....... | N......... | ........<br>...... | .S......<br>..N.F...<br>..T. | .V...............<br>............... | LGK..H-<br>................-<br>...... | ......<br>..... |
| iPS:434385 | 21-225_66C10 | VH3\|3-23/D4\|4-17\|RF2/JH6 | ............<br>............<br>........ | .......V.. | ........<br>...... | G.......<br>.AR.....<br>.... | .................<br>..............E | LGI...-<br>................--<br>..... | ......<br>..... |
| iPS:435717 | 21-225_171A9 | VH3\|3-23/D4\|4-17\|RF2/JH6 | ............<br>A...........<br>........ | .........T | ........<br>...... | ........<br>..N.FN..<br>.... | .................<br>............... | LGI...-<br>................-<br>...... | ......<br>..... |
| iPS:436528 | 21-225_224B1 | VH3\|3-23/D4\|4-17\|RF2/JH6 | ...V........<br>............<br>........ | .......... | ........<br>...... | ........<br>..N.....<br>.... | ...............H.<br>..............G | EG.Y..-<br>................-<br>...V.. | ......<br>..... |
| iPS:393810 | 21-225_5A4 | VH3\|3-23/D4\|4-17\|RF2/JH6 | ...V........<br>............<br>....L... | ......S... | ........<br>...... | ....R...<br>..N.F.T.<br>.... | .................<br>............... | LGK...-<br>................-<br>...... | ......<br>..... |
| | | Germline | H_FR1 | H_CDR1 | H_FR2 | H_CDR2 | H_FR3 | H_CDR3 | H_FR4 |
| | VH3\|3-48/D5\|5-24\|RF3/JH6 | | EVQLVES-<br>GGGLVQPGGSLR<br>LSCAASG-FTFS | S-----YSMN | WVRQAPGK<br>GLEWVS | YISSS---<br>SSTIYYAD<br>SVKG | RFTISRDNAKNSLYLQM<br>NSLRDEDTAVYYCAR | RDGYNYYY----------<br>-----YYYGMDV | WGQGTT<br>VTVSS |
| iPS:433945 | 21-225_44C12 | VH3\|3-48/D5\|5-24\|RF3/JH6 | ............<br>............<br>........ | ........V. | ........<br>...... | ........<br>........<br>.... | .................<br>............... | SGYSYA............<br>............ | ......<br>..... |
| | | Germline | H_FR1 | H_CDR1 | H_FR2 | H_CDR2 | H_FR3 | H_CDR3 | H_FR4 |

| | | | | | | | | | |
|---|---|---|---|---|---|---|---|---|---|
| | VH3\|3-33/D6\|6-13\|RF2/JH4 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | GIAAA------------<br>-------GYFDY | WGQGTL<br>VTVSS |
| iPS:43 3947 | 21-225_44E12 | VH3\|3-33/D6\|6-13\|RF2/JH4 | ..H.A.......<br>...........E<br>......L. | ......DDT. | ....P...<br>...... | ...F....<br>EY......<br>.... | .................<br>.......A....... | DLI..A...........<br>........TG.. | ......<br>..... |
| iPS:43 3963 | 21-225_46B1 | VH3\|3-33/D6\|6-13\|RF2/JH4 | ..H.A.......<br>...........E<br>......L. | ......DDS. | ....P...<br>...... | ...F....<br>EYT.....<br>.... | .................<br>.N.....A....... | DLI..T...........<br>........TG.. | ......<br>..... |
| iPS:43 3987 | 21-225_47A5 | VH3\|3-33/D6\|6-13\|RF2/JH4 | ............<br>............<br>........ | D.....DDT. | ........<br>...... | ...F....<br>........<br>.... | .................<br>...S........... | DLI..A...........<br>........TV.. | ......<br>..... |
| iPS:43 6258 | 21-225_202F12 | VH3\|3-33/D6\|6-13\|RF2/JH4 | ............<br>...........E<br>........ | Y......... | ........<br>...... | I.......<br>....F...<br>.... | .......S.........<br>..............S | N....A...........<br>.......P.... | ......<br>..... |
| iPS:39 2646 | 21-225_20G2 | VH3\|3-33/D6\|6-13\|RF2/JH4 | ............<br>............<br>......L. | ......DD.. | ....E...<br>...... | ...F....<br>........<br>.... | ..IM.............<br>.....G......... | DLI..A...........<br>........TV.. | ......<br>..... |
| iPS:39 2750 | 21-225_20A10 | VH3\|3-33/D6\|6-13\|RF2/JH4 | ............<br>............<br>......L. | ......DD.. | ........<br>...... | ...F....<br>........<br>.... | ..I..............<br>.........M..... | DLI..A...........<br>........TV.. | ......<br>..... |
| | | Germline | **H_FR1** | **H_CDR1** | **H_FR2** | **H_CDR2** | **H_FR3** | **H_CDR3** | **H_FR4** |
| | VH3\|3-23/D6\|6-19\|RF2/JH3 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | GIAVAG-----------<br>-------DAFDI | WGQGTM<br>VTVSS |
| iPS:43 3999 | 21-225_48D1 | VH3\|3-23/D6\|6-19\|RF2/JH3 | ............<br>............<br>..R..... | .......... | ........<br>...... | V...R...<br>....F...<br>.... | .................<br>..............R | R................<br>......NE.... | ......<br>..... |
| iPS:43 4003 | 21-225_48C3 | VH3\|3-23/D6\|6-19\|RF2/JH3 | ............<br>............<br>..R..... | .......... | ........<br>...... | V...R...<br>....F...<br>.... | .................<br>..............R | R................<br>......NE.... | ......<br>..... |
| iPS:43 4037 | 21-225_49G12 | VH3\|3-23/D6\|6-19\|RF2/JH3 | ............<br>...........T<br>........ | .......... | ........<br>...... | V.......<br>..T.F...<br>.... | .L...............<br>..............R | R................<br>......N..... | ......<br>..... |
| iPS:43 4041 | 21-225_50H8 | VH3\|3-23/D6\|6-19\|RF2/J | ............<br>............ | .......... | ........<br>...... | V...R...<br>..T.F... | .................<br>..............R | R................<br>......NE.... | ......<br>..... |

| | | | | | | | | | |
|---|---|---|---|---|---|---|---|---|---|
| iPS:43 4045 | 21-225_50H10 | VH3\|3-23/D6\|6-19\|RF2/J H3 | .G..........<br>............<br>..R..... | .......... | ........<br>...... | V...R...<br>....F...<br>.... | .................<br>..............R | R................<br>......NE.... | ......<br>..... |
| iPS:43 4073 | 21-225_51H10 | VH3\|3-23/D6\|6-19\|RF2/J H3 | ............<br>...........T<br>........ | .......... | ........<br>...... | V.......<br>..T.F...<br>.... | .L...............<br>..............R | R................<br>......N..... | ......<br>..... |
| iPS:43 6212 | 21-225_200G1 | VH3\|3-23/D6\|6-19\|RF2/J H3 | ............<br>............<br>........ | .......... | ........<br>...... | ....R...<br>..N.F...<br>..R. | .................<br>..............R | R...D............<br>......Y....V | ......<br>..... |
| iPS:39 2652 | 21-225_17C6 | VH3\|3-23/D6\|6-19\|RF2/J H3 | ............<br>............<br>..E..... | .........N | ........<br>...... | V...R...<br>..N.F...<br>.... | .............F...<br>..............S | RL...............<br>......SE.... | ......<br>..... |
| iPS:39 2668 | 21-225_17B4 | VH3\|3-23/D6\|6-19\|RF2/J H3 | ............<br>............<br>..E..... | .........N | ........<br>...... | V...R...<br>..N.F...<br>.... | .................<br>..............S | RL...............<br>......SE.... | ......<br>..... |
| iPS:39 2696 | 21-225_20A4 | VH3\|3-23/D6\|6-19\|RF2/J H3 | ............<br>............<br>........ | .........T | ....G..M<br>...... | V.......<br>..Y..N..<br>.... | .................<br>....V.........S | R................<br>......SE.... | ......<br>..... |
| iPS:39 2702 | 21-225_17F7 | VH3\|3-23/D6\|6-19\|RF2/J H3 | ............<br>............<br>..E..... | .........N | ........<br>...... | I...R...<br>..N.F...<br>.... | .................<br>..............S | RL...............<br>......SE.... | ......<br>..... |
| iPS:39 2704 | 21-225_17F11 | VH3\|3-23/D6\|6-19\|RF2/J H3 | .A..........<br>E...........<br>........ | .......... | ........<br>...... | V...R...<br>..N..S..<br>.... | .................<br>..............S | RL...............<br>......SE..H. | ......<br>..... |
| iPS:39 2720 | 21-225_17A12 | VH3\|3-23/D6\|6-19\|RF2/J H3 | ............<br>I...........<br>..E..... | .......... | ....D...<br>...... | I...R...<br>..NAF...<br>.... | .................<br>..............S | R................<br>......SE.... | ......<br>..... |
| iPS:39 2722 | 21-225_18E12 | VH3\|3-23/D6\|6-19\|RF2/J H3 | ............<br>............<br>..E..... | .......... | .......T<br>...... | I...R...<br>..N.F...<br>.... | .................<br>..............S | RL...............<br>......SE.... | ......<br>..... |
| iPS:39 2760 | 21-225_22G3 | VH3\|3-23/D6\|6-19\|RF2/J H3 | ............<br>............<br>..E..... | .........N | ........<br>...... | I...R...<br>.VN.F...<br>.... | .................<br>..............S | RL...............<br>......SE.... | ......<br>..... |
| iPS:39 | 21- | VH3\|3-23/D6\|6- | ..........D.<br>...........T | .........N | ........<br>...... | V.......<br>..N.F... | .............F.H.<br>..............S | RM...............<br>......SE.... | ......<br>..... |

| | | | | | | | | | |
|---|---|---|---|---|---|---|---|---|---|
| iPS:39 2812 | 21-225_21F4 | VH3\|3-23/D6\|6-19\|RF2/J H3 | ............<br>............<br>..E..... | .........N | ........<br>...... | V...R...<br>..N.F...<br>.... | .................<br>..............S | RL...............<br>......SE.C.. | ......<br>..... |
| iPS:39 2816 | 21-225_22E4 | VH3\|3-23/D6\|6-19\|RF2/J H3 | ............<br>............<br>..E..... | .......... | ........<br>...... | I...R...<br>.TN.F...<br>.... | ......V..........<br>..............S | R................<br>......SE.... | ......<br>..... |
| iPS:39 2852 | 21-225_21A2 | VH3\|3-23/D6\|6-19\|RF2/J H3 | ............<br>............<br>..K..... | .........N | ........<br>....I. | I...R...<br>..N.F...<br>.... | .................<br>..............S | RL...............<br>......SE.... | ......<br>..... |
| iPS:39 2878 | 21-225_22C5 | VH3\|3-23/D6\|6-19\|RF2/J H3 | ............<br>..V.........<br>........ | .......... | .......M<br>...... | I.......<br>..Y.....<br>.... | .................<br>..............S | R................<br>......SE.... | ......<br>..... |
| iPS:39 2902 | 21-225_22D5 | VH3\|3-23/D6\|6-19\|RF2/J H3 | ............<br>............<br>..E..... | .......... | ........<br>...... | V...R...<br>..N.F...<br>.... | .................<br>..............S | R................<br>......SE.... | ......<br>..... |
| iPS:39 3824 | 21-225_10F12 | VH3\|3-23/D6\|6-19\|RF2/J H3 | ............<br>............<br>..E..... | .......... | ........<br>...... | I...R...<br>..N.F...<br>.... | .................<br>..............S | RV...............<br>......SE..A. | ......<br>..... |
| iPS:39 3848 | 21-225_4H2 | VH3\|3-23/D6\|6-19\|RF2/J H3 | ............<br>............<br>........ | G........N | .......M<br>...... | V..R....<br>..Y.....<br>.... | .................<br>..............S | RL...............<br>......SE.... | ......<br>..... |
| iPS:39 3862 | 21-225_5G2 | VH3\|3-23/D6\|6-19\|RF2/J H3 | ............<br>............<br>..E..... | .......... | ........<br>...... | V...R...<br>.VN.F...<br>.... | .................<br>..............S | R................<br>......SE.... | ......<br>..... |
| iPS:39 3888 | 21-225_3E3 | VH3\|3-23/D6\|6-19\|RF2/J H3 | ..........T.<br>............<br>..E..... | .......V.. | ........<br>...... | I...R...<br>..N.F...<br>.... | .................<br>.........L....S | RL...............<br>......SE.... | ......<br>..... |
| iPS:39 3898 | 21-225_5F7 | VH3\|3-23/D6\|6-19\|RF2/J H3 | ............<br>............<br>..E..... | .......... | ........<br>...... | I...R...<br>..N.F...<br>.... | .................<br>..............S | R................<br>......SE..A. | ......<br>..... |
| iPS:39 3980 | 21-225_6D3 | VH3\|3-23/D6\|6-19\|RF2/J H3 | ............<br>............<br>..E..... | .........N | ........<br>...... | V...R...<br>..N.F...<br>.... | .................<br>...........F..S | RL...............<br>......SE.... | ......<br>..... |
| iPS:39 | 21-225_8G6 | VH3\|3-23/D6\|6- | ............<br>...........V | .......V.N | ........<br>...... | V...R...<br>..N.F... | .................<br>..............S | RL...............<br>......SE.... | ......<br>.S... |

| | | | | | | | | | |
|---|---|---|---|---|---|---|---|---|---|
| iPS:39 4022 | 21-225_16H6 | VH3\|3-23/D6\|6-19\|RF2/JH3 | .............<br>.............<br>........ | .........N | .......M<br>...... | V..R....<br>..Y.....<br>.... | .................<br>..............S | RL...............<br>......SE.... | ......<br>..... |
| iPS:39 4043 | 21-225_3B1 | VH3\|3-23/D6\|6-19\|RF2/JH3 | .............<br>.............<br>..E..... | .........N | ........<br>...... | V...R...<br>.IN.F...<br>.... | .................<br>..............S | RL...............<br>......SE.... | ......<br>..... |
| iPS:39 4077 | 21-225_8E12 | VH3\|3-23/D6\|6-19\|RF2/JH3 | .............<br>.............<br>..E..... | .......... | ........<br>...... | I...R...<br>..N.F...<br>.... | .................<br>..............S | RM...............<br>......SE.... | ......<br>..... |
| iPS:39 4087 | 21-225_11A5 | VH3\|3-23/D6\|6-19\|RF2/JH3 | ..........D.<br>.............<br>..E..... | .......... | ........<br>....I. | V...R...<br>.VN.F...<br>.... | .................<br>..............S | R................<br>......SE.... | ......<br>..... |
| | | Germline | **H_FR1** | **H_CDR1** | **H_FR2** | **H_CDR2** | **H_FR3** | **H_CDR3** | **H_FR4** |
| | VH3\|3-11/D6\|6-6\|RF2/JH3 | | QVQLVES-<br>GGGLVKPGGSLR<br>LSCAASG-FTFS | D-----YYMS | WIRQAPGK<br>GLEWVS | YISSS---<br>GSTIYYAD<br>SVKG | RFTISRDNAKNSLYLQM<br>NSLRAEDTAVYYCAR | SIAAR------------<br>-------DAFDI | WGQGTM<br>VTVSS |
| iPS:43 4011 | 21-225_48B10 | VH3\|3-11/D6\|6-6\|RF2/JH3 | .............<br>.............<br>........ | .......F.T | .......Q<br>...... | ....A...<br>.GA.....<br>.... | .................<br>..............I | AV..P............<br>.......GV... | ......<br>..... |
| | | Germline | **H_FR1** | **H_CDR1** | **H_FR2** | **H_CDR2** | **H_FR3** | **H_CDR3** | **H_FR4** |
| | VH3\|3-11/D6\|6-6\|RF2/JH4 | | QVQLVES-<br>GGGLVKPGGSLR<br>LSCAASG-FTFS | D-----YYMS | WIRQAPGK<br>GLEWVS | YISSS---<br>GSTIYYAD<br>SVKG | RFTISRDNAKNSLYLQM<br>NSLRAEDTAVYYCAR | SIAAR------------<br>--------YFDY | WGQGTL<br>VTVSS |
| iPS:43 4015 | 21-225_48F12 | VH3\|3-11/D6\|6-6\|RF2/JH4 | .............<br>.............<br>........ | .......F.T | .......Q<br>...... | ....A...<br>.GA.....<br>.... | .............F...<br>..............I | AV..P............<br>.......GA..I | ......<br>..... |
| iPS:43 4017 | 21-225_48G12 | VH3\|3-11/D6\|6-6\|RF2/JH4 | .............<br>.............<br>........ | .......F.T | .......Q<br>...... | ....A...<br>.GA.....<br>.... | .............F...<br>..............I | AV..P............<br>.......GA..I | ......<br>..... |
| | | Germline | **H_FR1** | **H_CDR1** | **H_FR2** | **H_CDR2** | **H_FR3** | **H_CDR3** | **H_FR4** |
| | VH3\|3-23/D6\|6-13\|RF2/JH4 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | GIAAA------------<br>-------GYFDY | WGQGTL<br>VTVSS |
| iPS:43 4023 | 21-225_49F1 | VH3\|3-23/D6\|6-13\|RF2/JH4 | .............<br>.............<br>........ | .......... | ........<br>..D... | V.......<br>....F...<br>.... | ..........S......<br>..............S | A....G...........<br>......AH.... | ......<br>..... |

| | | | | | | | | | |
|---|---|---|---|---|---|---|---|---|---|
| iPS:43 6246 | 21-225_201G6 | VH3\|3-23/D6\|6-13\|RF2/JH4 | .............<br>............V<br>........ | .......... | ........<br>...... | T.......<br>.VR.....<br>.... | ..............F...<br>............... | .G.RSSG...........<br>......WFH... | ......<br>..... |
| iPS:43 6254 | 21-225_202C12 | VH3\|3-23/D6\|6-13\|RF2/JH4 | .............<br>.............<br>........ | .......... | ........<br>...... | T.......<br>.VR.....<br>.... | .S...........F...<br>............... | .G.RSSG...........<br>......WFH... | ......<br>..... |
| iPS:43 6304 | 21-225_201F3 | VH3\|3-23/D6\|6-13\|RF2/JH4 | .............<br>......Q.....<br>........ | .......... | ........<br>...... | T.......<br>.VR.....<br>.... | .........N...F...<br>............... | .G.RSSG...........<br>......WFH... | ......<br>..... |
| iPS:43 6334 | 21-225_208G3 | VH3\|3-23/D6\|6-13\|RF2/JH4 | .............<br>.............<br>........ | .......... | ........<br>...... | T.......<br>.VR.....<br>.... | .S...........F...<br>.......K....... | .G.RSSG...........<br>......WFH... | ......<br>..... |
| | | Germline | H_FR1 | H_CDR1 | H_FR2 | H_CDR2 | H_FR3 | H_CDR3 | H_FR4 |
| | VH3\|3-23/D6\|6-19\|RF2/JH5 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | GIAVAG-----------<br>-------NWFDP | WGQGTL<br>VTVSS |
| iPS:43 4027 | 21-225_49H5 | VH3\|3-23/D6\|6-19\|RF2/JH5 | .............<br>.............<br>........ | .........T | ....T...<br>...... | ........<br>..NSF...<br>.... | .........E.......<br>............... | AR................<br>......SH.... | ......<br>..... |
| iPS:43 4061 | 21-225_51C7 | VH3\|3-23/D6\|6-19\|RF2/JH5 | .............<br>.............<br>......I. | N........T | ....T...<br>...... | V..A....<br>..NSF...<br>.... | .........E..F....<br>............... | AR................<br>......SH.... | ......<br>..... |
| iPS:43 4167 | 21-225_50F3 | VH3\|3-23/D6\|6-19\|RF2/JH5 | .............<br>.............<br>.......R | T........T | ........<br>...... | ........<br>.VNSF...<br>.... | .........E.......<br>............... | AR................<br>......SH.... | ......<br>..... |
| iPS:43 4455 | 21-225_72F5 | VH3\|3-23/D6\|6-19\|RF2/JH5 | .............<br>.............<br>........ | .........I | ........<br>...... | T.......<br>..Y..S..<br>.... | .................<br>..............R | R...T.............<br>......TE.Y.. | ......<br>..... |
| iPS:43 7232 | 21-225_63E1 | VH3\|3-23/D6\|6-19\|RF2/JH5 | ...M........<br>G.S........T<br>.......T | T.....S... | ........<br>...... | ........<br>.AN.F...<br>.... | ...VT............<br>...T.........V. | V.................<br>......GHF... | ......<br>..... |
| | | Germline | H_FR1 | H_CDR1 | H_FR2 | H_CDR2 | H_FR3 | H_CDR3 | H_FR4 |
| | VH3\|3-21/D1\|1-1\|RF2/JH4 | | EVQLVES-<br>GGGLVKPGGSLR<br>LSCAASG-FTFS | S-----YSMN | WVRQAPGK<br>GLEWVS | SISSS---<br>SSYIYYAD<br>SVKG | RFTISRDNAKNSLYLQM<br>NSLRAEDTAVYYCAR | VQLER------------<br>--------YFDY | WGQGTL<br>VTVSS |

| | | | | | | | | | |
|---|---|---|---|---|---|---|---|---|---|
| iPS:43 4043 | 21-225_50G10 | VH3\|3-21/D1\|1-1\|RF2/JH4 | ............<br>............<br>........ | .......... | ........<br>...... | ...G....<br>........<br>.... | .................<br>............... | .AT--<br>...................<br>...-... | ......<br>..... |
| iPS:43 4085 | 21-225_52E3 | VH3\|3-21/D1\|1-1\|RF2/JH4 | ............<br>............<br>........ | .......K.. | ........<br>...... | ....G...<br>N.S.....<br>.... | .........E.......<br>............... | .SS--<br>...................<br>...-N.. | ......<br>..... |
| iPS:43 4101 | 21-225_52H12 | VH3\|3-21/D1\|1-1\|RF2/JH4 | ............<br>............<br>........ | .......... | ........<br>...... | ...G....<br>.T......<br>.... | ...V...........V<br>............... | .NS--<br>...................<br>...-... | ......<br>..... |
| iPS:43 4187 | 21-225_56A5 | VH3\|3-21/D1\|1-1\|RF2/JH4 | ............<br>............<br>........ | .......... | ........<br>...... | ...G....<br>........<br>.... | .................<br>............... | .AT--<br>...................<br>...-... | ......<br>..... |
| iPS:43 4265 | 21-225_57B2 | VH3\|3-21/D1\|1-1\|RF2/JH4 | ............<br>............<br>........ | .......... | ........<br>...... | ...G....<br>....N.T.<br>.... | .................<br>............... | .AG--<br>...................<br>...-... | ......<br>..... |
| iPS:43 4439 | 21-225_70E12 | VH3\|3-21/D1\|1-1\|RF2/JH4 | ............<br>............<br>........ | .......... | ........<br>...... | ...GN...<br>.T....T.<br>.... | .................<br>D..T........... | .AA--<br>...................<br>...-..C | ......<br>..... |
| iPS:43 5515 | 21-225_157E4 | VH3\|3-21/D1\|1-1\|RF2/JH4 | ............<br>............<br>........ | .......T.. | ........<br>...... | ...G....<br>........<br>.... | .................<br>...K........... | .AT--<br>...................<br>...-... | ......<br>..... |
| iPS:43 5535 | 21-225_157H10 | VH3\|3-21/D1\|1-1\|RF2/JH4 | ............<br>............<br>........ | .......T.. | ........<br>...... | ...G....<br>....N...<br>.... | .................<br>............... | .AH--<br>...................<br>...-... | ......<br>..... |
| iPS:43 7224 | 21-225_56H1 | VH3\|3-21/D1\|1-1\|RF2/JH4 | ............<br>............<br>........ | N......R.. | ....G...<br>....I. | ...G....<br>.TD.....<br>.... | .................<br>............... | .AS--<br>...................<br>...-... | ......<br>..... |
| iPS:39 2620 | 21-225_17H5 | VH3\|3-21/D1\|1-1\|RF2/JH4 | ............<br>............<br>........ | .......... | ........<br>...... | ........<br>.T......<br>.... | .................<br>............... | .AS--<br>...................<br>...-... | ......<br>..... |
| iPS:39 2632 | 21-225_16A11 | VH3\|3-21/D1\|1-1\|RF2/JH4 | ............<br>............<br>........ | .......... | ........<br>...... | ...G....<br>..L.....<br>.... | .................<br>............... | .AA--<br>...................<br>...-... | ......<br>..... |
| iPS:39 | 21-225_20H7 | VH3\|3-21/D1\|1- | ..H.........<br>............ | .......... | ........<br>...... | ...G....<br>..F..... | .................<br>............... | .AA--<br>................... | ......<br>..... |

| | | | | | | | | | |
|---|---|---|---|---|---|---|---|---|---|
| iPS:39 2782 | 21-225_22B12 | VH3\|3-21/D1\|1-1\|RF2/JH4 | ............. ............. ........ | ........... | ......... ...... | ...G.... ...T.... .... | .................. ................ | .AA-- .................... ...-L.S | ...... ..... |
| iPS:39 2916 | 21-225_27C5 | VH3\|3-21/D1\|1-1\|RF2/JH4 | ............. ............. ........ | ........... | ......... ...... | .T...... D....... .... | .................. ................ | .AS-- .................... ...-..C | ...... ..... |
| iPS:39 2976 | 21-225_27H12 | VH3\|3-21/D1\|1-1\|RF2/JH4 | ............. ............. ........ | ........L. | ......... ...... | ...G.... ..N...T. .... | ..............F... ................ | .AS-- .................... ...-... | ...... ..... |
| iPS:39 3120 | 21-225_35H8 | VH3\|3-21/D1\|1-1\|RF2/JH4 | ............. ............. ........ | ........... | ......... ...... | ...GT... G.F..... .... | ..........K.V.... ................ | .SG-- .................... ...-... | ...... ..... |
| iPS:39 3836 | 21-225_15A2 | VH3\|3-21/D1\|1-1\|RF2/JH4 | ............. ............. ........ | .......T.. | ......... ...... | ...G.... G....... .... | .................. .A.............. | .AS-- .................... ...-... | ...... ..... |
| iPS:39 3894 | 21-225_5E11 | VH3\|3-21/D1\|1-1\|RF2/JH4 | ............. ............. ........ | ........... | ......... ...... | ...G.... .T...... .... | .................. ..........F..... | .AS-- .................... ...-... | ...... ..... |
| iPS:39 3896 | 21-225_2A4 | VH3\|3-21/D1\|1-1\|RF2/JH4 | ............. ............. ........ | ........... | ......... ...... | ...G.... .T...... .... | .IA............... ................ | .AS-- .................... ...-... | ...... ..... |
| iPS:39 3914 | 21-225_16B8 | VH3\|3-21/D1\|1-1\|RF2/JH4 | ............. ............. ........ | ........... | ......... ....I. | ...G.... .T...... .... | .................. ................ | .AS-- .................... ...-... | ...... ..... |
| iPS:39 3944 | 21-225_14D6 | VH3\|3-21/D1\|1-1\|RF2/JH4 | ............. ............. ........ | .......T.. | ......... ...... | ...G.... G....... .... | ...M.............. ................ | .AA-- .................... ...-..S | ...... .S... |
| iPS:39 3952 | 21-225_1F1 | VH3\|3-21/D1\|1-1\|RF2/JH4 | ............. ............. ........ | .......N.. | ......... ...... | ........ ........ .... | .................. ................ | .N.-- .................... ...-... | ...... ..... |
| iPS:39 4033 | 21-225_5F4 | VH3\|3-21/D1\|1-1\|RF2/JH4 | ............. ............V ........ | ........... | ......... ...... | ...G.... ........ .... | ..............F... ................ | .NN-- .................... ...-... | ...... ..... |
| iPS:39 | 21-225_16H1 | VH3\|3-21/D1\|1- | ............. ............. | ........... | ......... ...... | ...G.... .T...... | .................. ................ | .AA-- .................... | ...... ..... |

| | | | | | | | | | |
|---|---|---|---|---|---|---|---|---|---|
| iPS:39 8482 | 21-225_17H6 | VH3\|3-21/D1\|1-1\|RF2/JH4 | .............<br>.............<br>........ | .......... | .......R<br>...... | ...G....<br>........<br>.... | ....F............<br>............... | .AS--<br>...................<br>...-... | ......<br>..... |
| iPS:39 8500 | 21-225_23A11 | VH3\|3-21/D1\|1-1\|RF2/JH4 | .............<br>.............<br>........ | .......... | .I......<br>...... | ...G....<br>.T......<br>.... | .IA..............<br>............... | .AS--<br>...................<br>...-... | ......<br>..... |
| iPS:39 8526 | 21-225_32B3 | VH3\|3-21/D1\|1-1\|RF2/JH4 | .............<br>.............<br>........ | .......... | ........<br>...... | ...G....<br>.T......<br>.... | .................<br>............... | .AG--<br>...................<br>...-... | ......<br>..... |
| iPS:40 2221 | 21-225_2C12 | VH3\|3-21/D1\|1-1\|RF2/JH4 | .............<br>.............<br>........ | .......... | ........<br>...... | ...G....<br>...M....<br>.... | ..........D......<br>............... | .N.--<br>...................<br>...-... | ......<br>..... |
| | | Germline | **H_FR1** | **H_CDR1** | **H_FR2** | **H_CDR2** | **H_FR3** | **H_CDR3** | **H_FR4** |
| | VH1\|1-02/D2\|2-15\|RF2/JH6 | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | GYCSGGSCYS--------<br>---YYYYYGMDV | WGQGTT<br>VTVSS |
| iPS:43 4047 | 21-225_50A12 | VH1\|1-02/D2\|2-15\|RF2/JH6 | .............<br>.............<br>........ | ......H.IN | ........<br>.P...A | .V......<br>.....S..<br>.... | ............V....<br>............... | .GQL..FN--<br>...........-<br>........ | ......<br>..... |
| iPS:43 4067 | 21-225_51H8 | VH1\|1-02/D2\|2-15\|RF2/JH6 | .............<br>.............<br>........ | ......H..N | ........<br>...... | .V......<br>...S.S..<br>Q... | ............V....<br>...S........... | .GQL..FN--<br>...........-<br>F....... | ......<br>..... |
| iPS:43 4197 | 21-225_56C7 | VH1\|1-02/D2\|2-15\|RF2/JH6 | .............<br>.............<br>........ | ......H.IN | ........<br>.....A | .V......<br>.....S..<br>.... | ............V....<br>............... | .GQL..FN--<br>...........-<br>........ | ......<br>..... |
| | | Germline | **H_FR1** | **H_CDR1** | **H_FR2** | **H_CDR2** | **H_FR3** | **H_CDR3** | **H_FR4** |
| | VH3\|3-21/D2\|2-15\|RF3/JH6 | | EVQLVES-<br>GGGLVKPGGSLR<br>LSCAASG-FTFS | S-----YSMN | WVRQAPGK<br>GLEWVS | SISSS---<br>SSYIYYAD<br>SVKG | RFTISRDNAKNSLYLQM<br>NSLRAEDTAVYYCAR | DIVVVVAAT---------<br>---YYYYYGMDV | WGQGTT<br>VTVSS |
| iPS:43 4049 | 21-225_50B12 | VH3\|3-21/D2\|2-15\|RF3/JH6 | .............<br>.............<br>........ | ......H... | ........<br>...... | ........<br>.N......<br>.... | .......Y.........<br>............... | .RSI...GP.........<br>...-WD...... | ......<br>..... |
| | | Germline | **H_FR1** | **H_CDR1** | **H_FR2** | **H_CDR2** | **H_FR3** | **H_CDR3** | **H_FR4** |
| | VH3\|3-23/D1\|1-20\|RF1/JH3 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | GITGT------------<br>-------DAFDI | WGQGTM<br>VTVSS |

| | | | | | | | | | |
|---|---|---|---|---|---|---|---|---|---|
| iPS:43 4055 | 21-225_51B4 | VH3\|3-23/D1\|1-20\|RF1/JH3 | ............<br>.......S....<br>.......R | .......V.. | ........<br>...... | ....R...<br>.SN.F.T.<br>.... | .................<br>............... | ....SH............<br>.......G.... | ......<br>..... |
| | | Germline | H_FR1 | H_CDR1 | H_FR2 | H_CDR2 | H_FR3 | H_CDR3 | H_FR4 |
| | VH3\|3-23/D7\|7-27\|RF1/JH4 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | LTGY------------<br>---------FDY | WGQGTL<br>VTVSS |
| iPS:43 4059 | 21-225_51C5 | VH3\|3-23/D7\|7-27\|RF1/JH4 | ............<br>............<br>........ | .......V.. | ....T...<br>...... | TM......<br>..R.....<br>..N. | ...V............<br>S.............R | V.A-<br>..................<br>........ | ......<br>..... |
| iPS:43 4213 | 21-225_60A4 | VH3\|3-23/D7\|7-27\|RF1/JH4 | ............<br>............<br>........ | .......V.. | ........<br>...... | S.......<br>..W.N...<br>.... | ...T............<br>..............R | ...-<br>..................<br>........ | ......<br>..... |
| iPS:43 4215 | 21-225_60F7 | VH3\|3-23/D7\|7-27\|RF1/JH4 | ............<br>............<br>........ | .......V.. | ........<br>...... | G.......<br>.NR.....<br>.... | .................<br>.......S.....GS | .G--<br>..................<br>.....-ID | ......<br>..... |
| iPS:43 4241 | 21-225_61E6 | VH3\|3-23/D7\|7-27\|RF1/JH4 | ............<br>............<br>.......R | .......... | ........<br>...... | .T......<br>.VN.F...<br>.... | .................<br>............... | .ELG.............<br>........I... | ......<br>..... |
| iPS:43 4281 | 21-225_57B8 | VH3\|3-23/D7\|7-27\|RF1/JH4 | ............<br>............<br>.......R | .......... | ........<br>...... | ........<br>..N.F...<br>.... | .................<br>............... | .ELG.............<br>........I... | ......<br>..... |
| iPS:43 4301 | 21-225_58F11 | VH3\|3-23/D7\|7-27\|RF1/JH4 | ............<br>............<br>........ | .......... | ........<br>...... | ........<br>..N.F...<br>.... | ......Y.........<br>............... | FF.MVG...........<br>......AGF... | ......<br>..... |
| iPS:43 5523 | 21-225_157G5 | VH3\|3-23/D7\|7-27\|RF1/JH4 | ............<br>............<br>........ | .......V.. | ........<br>..D... | .M......<br>..R.....<br>.... | .................<br>............... | Y.W-<br>..................<br>.....NG. | ......<br>..... |
| iPS:43 5659 | 21-225_167D12 | VH3\|3-23/D7\|7-27\|RF1/JH4 | ............<br>............<br>........ | .......V.. | ........<br>...... | .M......<br>..R.....<br>.... | .................<br>............... | Y.W-<br>..................<br>.....NG. | ......<br>..... |
| iPS:43 5765 | 21-225_177D3 | VH3\|3-23/D7\|7-27\|RF1/JH4 | ............<br>............<br>........ | .......V.N | ........<br>...... | GM......<br>..R.....<br>...D | ..............S...<br>..............R | V.F-<br>..................<br>........ | ......<br>..... |

| | | | | | | | | | |
|---|---|---|---|---|---|---|---|---|---|
| iPS:43 7216 | 21-225_51D5 | VH3\|3-23/D7\|7-27\|RF1/JH4 | .............<br>.............<br>........ | .......V.. | ....T...<br>...... | TM......<br>..R.....<br>..N. | ...V.............<br>S.............R | V.A-<br>...................<br>........ | ......<br>..... |
| | | Germline | H_FR1 | H_CDR1 | H_FR2 | H_CDR2 | H_FR3 | H_CDR3 | H_FR4 |
| | VH3\|3-21/D5\|5-24\|RF2/JH4 | | EVQLVES-<br>GGGLVKPGGSLR<br>LSCAASG-FTFS | S-----YSMN | WVRQAPGK<br>GLEWVS | SISSS---<br>SSYIYYAD<br>SVKG | RFTISRDNAKNSLYLQM<br>NSLRAEDTAVYYCAR | *RWLQ------------<br>-------LYFDY | WGQGTL<br>VTVSS |
| iPS:43 4063 | 21-225_51G7 | VH3\|3-21/D5\|5-24\|RF2/JH4 | .............<br>.............<br>........ | .......... | ........<br>...... | ........<br>........<br>.... | .................<br>............... | A.L--<br>...................<br>..---.. | ......<br>..... |
| | | Germline | H_FR1 | H_CDR1 | H_FR2 | H_CDR2 | H_FR3 | H_CDR3 | H_FR4 |
| | VH3\|3-23/D7\|7-27\|RF3/JH4 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | NWGY-------------<br>---------FDY | WGQGTL<br>VTVSS |
| iPS:43 4083 | 21-225_52H2 | VH3\|3-23/D7\|7-27\|RF3/JH4 | .............<br>.............<br>........ | R.....N... | .......M<br>...... | ....R...<br>..N.F...<br>.... | ...V........F...<br>............... | .GREQ............<br>........WL.. | ......<br>..... |
| | | Germline | H_FR1 | H_CDR1 | H_FR2 | H_CDR2 | H_FR3 | H_CDR3 | H_FR4 |
| | VH3\|3-30.3/D6\|6-6\|RF2/JH6 | | QVQLVES-<br>GGGVVQPGRSLR<br>LSCAASG-FTFS | S-----YAMH | WVRQAPGK<br>GLEWVA | VISYD---<br>GSNKYYAD<br>SVKG | RFTISRDNSKNTLYLQM<br>NSLRAEDTAVYYCAR | SIAARYY----------<br>-----YYYGMDV | WGQGTT<br>VTVSS |
| iPS:43 4087 | 21-225_52F6 | VH3\|3-30.3/D6\|6-6\|RF2/JH6 | .............<br>.............<br>........ | .......G.. | ........<br>...... | I...G...<br>........<br>.... | .................<br>.....D......... | RS...P-<br>................-<br>G..... | ......<br>..... |
| iPS:43 4111 | 21-225_53H2 | VH3\|3-30.3/D6\|6-6\|RF2/JH6 | .............<br>.............<br>........ | .......G.. | ........<br>.P.... | ....G...<br>.....H..<br>.... | .................<br>............... | RG...P-<br>................-<br>G..... | ......<br>..... |
| iPS:43 4121 | 21-225_53F6 | VH3\|3-30.3/D6\|6-6\|RF2/JH6 | .............<br>.............<br>........ | .......G.. | ........<br>...... | ....G...<br>.....D..<br>.... | .................<br>T.............. | RR...P-<br>................-<br>G..... | ......<br>..... |
| iPS:43 4163 | 21-225_50H1 | VH3\|3-30.3/D6\|6-6\|RF2/JH6 | .............<br>.............<br>........ | .......G.. | ........<br>.....S | I...G...<br>.....D..<br>.... | .................<br>T.............. | RR...P-<br>................-<br>G..... | ....I.<br>..... |

| | | | | | | | | | |
|---|---|---|---|---|---|---|---|---|---|
| iPS:39 4035 | 21-225_5G9 | VH3\|3-30.3/D6\|6-6\|RF2/JH6 | .................................. | N......G.. | .............. | I...A.............. | ...............................V. | R.T..L-..................-...... | ........... |
| | | Germline | H_FR1 | H_CDR1 | H_FR2 | H_CDR2 | H_FR3 | H_CDR3 | H_FR4 |
| | VH3\|3-33/D2\|2-8\|RF1/JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | RILY*WC-----------------MLYYFDY | WGQGTLVTVSS |
| iPS:43 4095 | 21-225_52F10 | VH3\|3-33/D2\|2-8\|RF1/JH4 | .................................. | F......... | .............. | ...D................ | .............F...M.............. | DS..SS-..................-SWL... | ........... |
| | | Germline | H_FR1 | H_CDR1 | H_FR2 | H_CDR2 | H_FR3 | H_CDR3 | H_FR4 |
| | VH3\|3-23/D4\|4-23\|RF3/JH4 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | TTVVT--------------------YFDY | WGQGTLVTVSS |
| iPS:43 4107 | 21-225_53E2 | VH3\|3-23/D4\|4-23\|RF3/JH4 | ............I...................R | .......... | .............. | ..........N.F....... | ..................T...D......... | KV.D.A...................MAL.. | ........... |
| iPS:43 4181 | 21-225_56B2 | VH3\|3-23/D4\|4-23\|RF3/JH4 | ............I...................R | .......... | .............. | ..........N.F....... | ..................T...D......... | KV.D.A...................MAL.. | ........... |
| | | Germline | H_FR1 | H_CDR1 | H_FR2 | H_CDR2 | H_FR3 | H_CDR3 | H_FR4 |
| | VH3\|3-23/D1\|1-26\|RF1/JH3 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GIVGAT-----------------DAFDI | WGQGTMVTVSS |
| iPS:43 4117 | 21-225_53C6 | VH3\|3-23/D1\|1-26\|RF1/JH3 | .................................. | .........N | .............. | ..........A......... | ................................ | PL...H.....................E. | ........... |
| iPS:39 2984 | 21-225_30E11 | VH3\|3-23/D1\|1-26\|RF1/JH3 | ..........DM...................... | I......... | .............. | V..........SF....... | .............................T. | DR.K.H..................G... | ........... |
| iPS:39 3114 | 21-225_33G12 | VH3\|3-23/D1\|1-26\|RF1/JH3 | ..........DM...................... | .......... | .............. | V..........SF....... | ................................ | DR.R.H..................G... | ........... |
| | | Germline | H_FR1 | H_CDR1 | H_FR2 | H_CDR2 | H_FR3 | H_CDR3 | H_FR4 |
| | VH3\|3-33/D5\|5-24\|RF2/JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | *RWLQ-------------------LYFDY | WGQGTLVTVSS |

| | | | | | | | | | |
|---|---|---|---|---|---|---|---|---|---|
| iPS:43 4127 | 21-225_53H8 | VH3\|3-33/D5\|5-24\|RF2/JH4 | ............<br>............<br>........ | .......... | ........<br>...... | ........<br>........<br>.... | .................<br>............... | A.IG-<br>..................<br>..-...S | ......<br>..... |
| | | Germline | H_FR1 | H_CDR1 | H_FR2 | H_CDR2 | H_FR3 | H_CDR3 | H_FR4 |
| | VH3\|3-23/D4\|4-17\|RF2/JH4 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | DYGDY-----------<br>--------YFDY | WGQGTL<br>VTVSS |
| iPS:43 4147 | 21-225_55E1 | VH3\|3-23/D4\|4-17\|RF2/JH4 | ............<br>............<br>........ | .......... | ........<br>...... | ....R...<br>.S..F...<br>.... | .................<br>..........F.... | .H.IVG..........<br>......TI.... | ......<br>..... |
| iPS:43 5303 | 21-225_146A6 | VH3\|3-23/D4\|4-17\|RF2/JH4 | ............<br>............<br>.......N | .........N | ........<br>...... | ........<br>..N.F...<br>.... | .................<br>............... | KDN..VW..........<br>.....GSP.... | ......<br>..... |
| iPS:43 5335 | 21-225_147D10 | VH3\|3-23/D4\|4-17\|RF2/JH4 | ............<br>............<br>........ | .......... | ........<br>...... | ....R...<br>..N.F...<br>.... | .................<br>............... | KDY..VW..........<br>.....GSP.... | ......<br>..... |
| iPS:43 5339 | 21-225_147D12 | VH3\|3-23/D4\|4-17\|RF2/JH4 | ............<br>..S.........<br>........ | .......... | ........<br>...... | ....R...<br>..N.F...<br>.... | .................<br>............... | KDY..VW..........<br>.....GSP.... | ......<br>..... |
| iPS:43 5343 | 21-225_148E2 | VH3\|3-23/D4\|4-17\|RF2/JH4 | ............<br>............<br>........ | .......... | ........<br>...... | ....R...<br>..N.F...<br>.... | .................<br>............... | KDY..VW..........<br>.....GSP.... | ......<br>..... |
| iPS:43 5381 | 21-225_149C6 | VH3\|3-23/D4\|4-17\|RF2/JH4 | ............<br>............<br>........ | .......... | ........<br>...... | ........<br>..N.F...<br>.... | ...............H.<br>............... | KDY..VW..........<br>.....GSP.... | ......<br>..... |
| iPS:43 5391 | 21-225_149F8 | VH3\|3-23/D4\|4-17\|RF2/JH4 | ............<br>............<br>........ | .......... | ........<br>...... | ....R...<br>..N.F...<br>.... | .................<br>............... | KDY..VW..........<br>.....GSP.... | ......<br>..... |
| iPS:43 5395 | 21-225_149D11 | VH3\|3-23/D4\|4-17\|RF2/JH4 | ............<br>............<br>........ | .......... | ........<br>...... | ....R...<br>..N.F...<br>.... | ..............R..<br>............... | KDY..VW..........<br>.....GSP.... | ......<br>..... |
| iPS:43 5403 | 21-225_150C5 | VH3\|3-23/D4\|4-17\|RF2/JH4 | ............<br>............<br>........ | .......... | ........<br>...... | ........<br>..N.F...<br>.... | .................<br>............... | KDY..VW..........<br>.....GSP.... | ......<br>..... |

| | | | | | | | | | |
|---|---|---|---|---|---|---|---|---|---|
| iPS:43 5447 | 21-225_152H7 | VH3\|3-23/D4\|4-17\|RF2/JH4 | ............<br>............<br>.......N | .........N | ........<br>...... | ........<br>..N.F...<br>.... | .................<br>............... | KDN..VW...........<br>.....GSP.... | ......<br>..... |
| iPS:43 5453 | 21-225_152G10 | VH3\|3-23/D4\|4-17\|RF2/JH4 | ............<br>............<br>........ | .......... | ........<br>...... | ....R...<br>..N.F...<br>.... | .................<br>............... | KDY..VW...........<br>.....GSP.... | ......<br>..... |
| iPS:43 5483 | 21-225_155A4 | VH3\|3-23/D4\|4-17\|RF2/JH4 | ............<br>............<br>.......N | .......... | ........<br>...... | ........<br>..N.F...<br>.... | .................<br>............... | KDY..VW...........<br>.....GSP.... | ......<br>..... |
| iPS:43 5485 | 21-225_155B4 | VH3\|3-23/D4\|4-17\|RF2/JH4 | ............<br>............<br>........ | .......... | ........<br>...... | ........<br>..N.F...<br>.... | .................<br>............... | KDY..VW...........<br>.....GSP.... | ......<br>..... |
| iPS:43 5777 | 21-225_178F7 | VH3\|3-23/D4\|4-17\|RF2/JH4 | ..H.........<br>..T.........<br>........ | .........T | ........<br>...... | V.......<br>..N.F...<br>.... | .................<br>..............R | R...-<br>..................<br>....... | ......<br>..... |
| iPS:43 5783 | 21-225_179G1 | VH3\|3-23/D4\|4-17\|RF2/JH4 | ..H.........<br>..T.........<br>........ | .........T | ........<br>...... | V...F...<br>..N.F...<br>.... | .................<br>..............R | R...-<br>..................<br>....... | ......<br>..... |
| iPS:43 5833 | 21-225_190D12 | VH3\|3-23/D4\|4-17\|RF2/JH4 | ............<br>............<br>........ | .......... | ........<br>....D. | ..I.N...<br>..R.....<br>.... | .................<br>............... | .M.R.S............<br>......YGF... | ......<br>..... |
| iPS:43 6156 | 21-225_197C8 | VH3\|3-23/D4\|4-17\|RF2/JH4 | ............<br>............<br>........ | ......S..T | ........<br>...... | ..I.N...<br>..RA....<br>.... | .................<br>....T.......... | .R.YSRIA..........<br>.....VAGT... | ......<br>..... |
| | | Germline | **H_FR1** | **H_CDR1** | **H_FR2** | **H_CDR2** | **H_FR3** | **H_CDR3** | **H_FR4** |
| | VH3\|3-21/D1\|1-1\|RF1/JH4 | | EVQLVES-<br>GGGLVKPGGSLR<br>LSCAASG-FTFS | S-----YSMN | WVRQAPGK<br>GLEWVS | SISSS---<br>SSYIYYAD<br>SVKG | RFTISRDNAKNSLYLQM<br>NSLRAEDTAVYYCAR | GTTGT-------------<br>--------YFDY | WGQGTL<br>VTVSS |
| iPS:43 4157 | 21-225_55D4 | VH3\|3-21/D1\|1-1\|RF1/JH4 | ............<br>............<br>.......N | ........R.. | ........<br>...... | ........<br>.NH.D...<br>.... | .................<br>.........L..... | ..---<br>..................<br>...--.. | ......<br>.S... |
| iPS:43 4243 | 21-225_62C1 | VH3\|3-21/D1\|1-1\|RF1/JH4 | ............<br>............<br>........ | .......... | ........<br>...... | ........<br>........<br>.... | .................<br>..............I | FG---<br>..................<br>...--VD | ......<br>..... |

| | | | | | | | | | |
|---|---|---|---|---|---|---|---|---|---|
| iPS:43 5505 | 21-225_157C1 | VH3\|3-21/D1\|1-1\|RF1/JH4 | ............<br>............<br>........ | .......R.. | ........<br>...... | .M.N....<br>..S.....<br>.... | ..................<br>.............. | QAA--<br>..................<br>...-Q.. | ......<br>..... |
| iPS:39 2966 | 21-225_32G3 | VH3\|3-21/D1\|1-1\|RF1/JH4 | ............<br>............<br>........ | .......... | ........<br>...... | ...G....<br>........<br>.... | .................I<br>.............. | .NIA-<br>..................<br>...-R.. | ......<br>..... |
| | | Germline | H_FR1 | H_CDR1 | H_FR2 | H_CDR2 | H_FR3 | H_CDR3 | H_FR4 |
| | VH3\|3-33/D7\|7-27\|RF3/JH4 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | NWGY--------------<br>---------FDY | WGQGTL<br>VTVSS |
| iPS:43 4159 | 21-225_55B8 | VH3\|3-33/D7\|7-27\|RF3/JH4 | ............<br>............<br>........ | D......... | ........<br>...... | ........<br>EN......<br>.... | ..................<br>............T. | E.F-<br>..................<br>.....-.. | ......<br>..... |
| iPS:39 3026 | 21-225_32B6 | VH3\|3-33/D7\|7-27\|RF3/JH4 | ............<br>............<br>........ | D......... | ......D.<br>...... | ........<br>ENT.....<br>.... | ..................<br>.............. | E..-<br>..................<br>.....-.. | ......<br>..... |
| | | Germline | H_FR1 | H_CDR1 | H_FR2 | H_CDR2 | H_FR3 | H_CDR3 | H_FR4 |
| | VH3\|3-33/D6\|6-6\|RF3/JH4 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | V*QLV-------------<br>--------YFDY | WGQGTL<br>VTVSS |
| iPS:43 4165 | 21-225_50F2 | VH3\|3-33/D6\|6-6\|RF3/JH4 | ............<br>............<br>..A..... | .......... | ....T...<br>...... | ...H....<br>........<br>.... | ..................<br>.............. | DE..G.............<br>........T... | ......<br>..... |
| iPS:43 4191 | 21-225_56B6 | VH3\|3-33/D6\|6-6\|RF3/JH4 | ............<br>............<br>........ | .......... | ........<br>...... | ...H....<br>......V.<br>.... | ..................<br>.............. | DE..G.............<br>........T... | ......<br>..... |
| | | Germline | H_FR1 | H_CDR1 | H_FR2 | H_CDR2 | H_FR3 | H_CDR3 | H_FR4 |
| | VH1\|1-46/D4\|4-17\|RF2/JH6 | | QVQLVQS-<br>GAEVKKPGASVK<br>VSCKASG-YTFT | S-----YYMH | WVRQAPGQ<br>GLEWMG | IINPS---<br>GGSTSYAQ<br>KFQG | RVTMTRDTSTSTVYMEL<br>SSLRSEDTAVYYCAR | DYGDYYY-----------<br>-----YYYGMDV | WGQGTT<br>VTVSS |
| iPS:43 4171 | 21-225_50G4 | VH1\|1-46/D4\|4-17\|RF2/JH6 | ............<br>.E..........<br>........ | ........I. | ........<br>...... | V.......<br>N.R.....<br>.... | ..................<br>.............. | .R..G.............<br>.....F...... | ......<br>..... |
| | | Germline | H_FR1 | H_CDR1 | H_FR2 | H_CDR2 | H_FR3 | H_CDR3 | H_FR4 |
| | VH3\|3-30.3/D4\|4-17\|RF2/JH3 | | QVQLVES-<br>GGGVVQPGRSLR<br>LSCAASG-FTFS | S-----YAMH | WVRQAPGK<br>GLEWVA | VISYD---<br>GSNKYYAD<br>SVKG | RFTISRDNSKNTLYLQM<br>NSLRAEDTAVYYCAR | DYGDY-------------<br>--------DAFDI | WGQGTM<br>VTVSS |

| | | | | | | | | | |
|---|---|---|---|---|---|---|---|---|---|
| iPS:434175 | 21-225_55A11 | VH3\|3-30.3/D4\|4-17\|RF2/JH3 | .........................................  | ........G.. | ....T........... | ....V.....T.......R. | ..................T.......... | GR.R.SDY............GH..... | ........... |
| | | Germline | H_FR1 | H_CDR1 | H_FR2 | H_CDR2 | H_FR3 | H_CDR3 | H_FR4 |
| | VH1\|1-02/D1\|1-1\|RF1/JH2 | | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | GTTGTY------------------WYFDL | WGRGTLVTVSS |
| iPS:434193 | 21-225_56C6 | VH1\|1-02/D1\|1-1\|RF1/JH2 | ................................ | D......H.. | .............. | ........R.....V..... | ..A..N.............G............. | DG.S--...................-S..Y | ........... |
| | | Germline | H_FR1 | H_CDR1 | H_FR2 | H_CDR2 | H_FR3 | H_CDR3 | H_FR4 |
| | VH1\|1-02/D5\|5-24\|RF1/JH4 | | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | VEMAT-------------------IYFDY | WGQGTLVTVSS |
| iPS:434195 | 21-225_56F6 | VH1\|1-02/D5\|5-24\|RF1/JH4 | ........................F... | D......... | .............. | ................R... | ...............................T. | EGATRP..................TG... | ........... |
| | | Germline | H_FR1 | H_CDR1 | H_FR2 | H_CDR2 | H_FR3 | H_CDR3 | H_FR4 |
| | VH3\|3-33/D1\|1-1\|RF2/JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | VQLER-------------------YFDY | WGQGTLVTVSS |
| iPS:434203 | 21-225_60E2 | VH3\|3-33/D1\|1-1\|RF2/JH4 | ...........................N | D......... | .............. | I.......EN.......... | .................S.............. | DV.D-...................P... | ........... |
| iPS:434229 | 21-225_61H1 | VH3\|3-33/D1\|1-1\|RF2/JH4 | ...........................N | D......... | .............. | I.......E........... | ................................ | DV.D-...................P... | ........... |
| | | Germline | H_FR1 | H_CDR1 | H_FR2 | H_CDR2 | H_FR3 | H_CDR3 | H_FR4 |
| | VH1\|1-02/D5\|5-18\|RF3/JH6 | | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | GYSYGYYY---------------YYYGMDV | WGQGTTVTVSS |
| iPS:434209 | 21-225_60C3 | VH1\|1-02/D5\|5-18\|RF3/JH6 | .............................S.. | ......H.I. | ...........Y.. | ..............V..... | ..........................I...S. | .GLL.ATN..............Y....... | ........... |
| iPS:434315 | 21-225_59G7 | VH1\|1-02/D5\|5-18\|RF3/JH6 | .............................S.. | ......H.I. | ...........Y.. | ..............V..... | ..........................I...S. | .GLL.ATN..............Y....... | .........T. |

| | | | | | | | | | |
|---|---|---|---|---|---|---|---|---|---|
| iPS:43 4319 | 21-225_59B9 | VH1\|1-02/D5\|5-18\|RF3/JH6 | .........P..<br>............<br>.....I.. | ......N.I. | ........<br>...Y.. | ........<br>......V.<br>.... | .............N...<br>TS..........S. | .GLL.AT..........<br>....Y....... | ......<br>..... |
| iPS:44 3003 | 21-225_43F11_LC2 | VH1\|1-02/D5\|5-18\|RF3/JH6 | ............<br>............<br>........ | ........I. | ........<br>...... | ........<br>........<br>.... | G....L...N....D.<br>............... | .GN.F.--<br>...............-<br>NHV... | .....P<br>..... |
| iPS:44 3005 | 21-225_43F11_LC1 | VH1\|1-02/D5\|5-18\|RF3/JH6 | ............<br>............<br>........ | ........I. | ........<br>...... | ........<br>........<br>.... | G....L...N....D.<br>............... | .GN.F.--<br>...............-<br>NHV... | .....P<br>..... |
| | | Germline | H_FR1 | H_CDR1 | H_FR2 | H_CDR2 | H_FR3 | H_CDR3 | H_FR4 |
| | VH4\|4-30.4/D5\|5-12\|RF3/JH4 | | QVQLQES-<br>GPGLVKPSQTLS<br>LTCTVSG-GSIS | SG---DYYWS | WIRQPPGK<br>GLEWIG | YIYY----<br>SGSTYYNP<br>SLKS | RVTISVDTSKNQFSLKL<br>SSVTAADTAVYYCAR | GYSGYD-----------<br>-------YYFDY | WGQGTL<br>VTVSS |
| iPS:43 4217 | 21-225_60E8 | VH4\|4-30.4/D5\|5-12\|RF3/JH4 | ............<br>.R..A.......<br>........ | RS...S...G | ........<br>...... | S.......<br>...AS...<br>.... | .........E.....R.<br>............... | LD..W-<br>..................<br>.-S... | ......<br>..... |
| | | Germline | H_FR1 | H_CDR1 | H_FR2 | H_CDR2 | H_FR3 | H_CDR3 | H_FR4 |
| | VH3\|3-23/D3\|3-22\|RF3/JH4 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | ITMIVVV----------<br>------ITYFDY | WGQGTL<br>VTVSS |
| iPS:43 4219 | 21-225_60E9 | VH3\|3-23/D3\|3-22\|RF3/JH4 | ............<br>............<br>........ | .......... | ........<br>...... | ........<br>..N.F...<br>.... | .............F...<br>............... | FFG..G-<br>.................A<br>G.... | ......<br>..... |
| iPS:43 4289 | 21-225_57H12 | VH3\|3-23/D3\|3-22\|RF3/JH4 | ............<br>............<br>....L... | .......... | ....D...<br>...... | ........<br>..N.F.G.<br>.... | ..........K......<br>............... | FFG..G-<br>.................A<br>G.... | ......<br>..... |
| iPS:43 4297 | 21-225_58A10 | VH3\|3-23/D3\|3-22\|RF3/JH4 | ............<br>............<br>.......R | .......... | ........<br>...... | ........<br>..N.F...<br>.... | .............F...<br>............... | FFG..G-<br>.................A<br>G.... | ......<br>..... |
| iPS:39 2996 | 21-225_28B1 | VH3\|3-23/D3\|3-22\|RF3/JH4 | ............<br>............<br>........ | .......... | ........<br>...... | ........<br>..N.F...<br>.... | .................<br>............... | LGR.A.T..........<br>......GP.... | ......<br>..... |
| | | Germline | H_FR1 | H_CDR1 | H_FR2 | H_CDR2 | H_FR3 | H_CDR3 | H_FR4 |
| | VH4\|4-59/D4\|4-11\|RF3/JH4 | | QVQLQES-<br>GPGLVKPSETLS<br>LTCTVSG-GSIS | S-----YYWS | WIRQPPGK<br>GLEWIG | YIYY----<br>SGSTNYNP<br>SLKS | RVTISVDTSKNQFSLKL<br>SSVTAADTAVYYCAR | TTVT-------------<br>--------YFDY | WGQGTL<br>VTVSS |

| | | | | | | | | | |
|---|---|---|---|---|---|---|---|---|---|
| iPS:43 4225 | 21-225_60E12 | VH4\|4-59/D4\|4-11\|RF3/JH4 | ............. ............. ........ | .......F.. | .....A.. ...... | R..T.... R....... .... | ...M............. ............... | EGK.GG........... ......VS.... | ...... ..... |
| iPS:43 4227 | 21-225_61A1 | VH4\|4-59/D4\|4-11\|RF3/JH4 | ............. ............. ........ | ......HF.. | .....A.. ...... | R..I.... R....... .... | ...M............. ............... | EGK.GG........... ......VS.... | ...... ..... |
| iPS:43 4267 | 21-225_57F2 | VH4\|4-59/D4\|4-11\|RF3/JH4 | ............. ............. ........ | .......... | .....A.. ...... | R..T.... R....... .... | ...M.I........... ............... | EGK.GG........... ......VS.... | ...... ..... |
| | | Germline | H_FR1 | H_CDR1 | H_FR2 | H_CDR2 | H_FR3 | H_CDR3 | H_FR4 |
| | VH3\|3-23/D5\|5-18\|RF3/JH3 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GYSYGY------------------DAFDI | WGQGTMVTVSS |
| iPS:43 4239 | 21-225_58F1 | VH3\|3-23/D5\|5-18\|RF3/JH3 | ............. ............. ........ | .......... | ........ ...... | ...TG... ..N..... .... | ................. ............... | RGV..D........... ......F..... | ...... ..... |
| iPS:43 4309 | 21-225_59B5 | VH3\|3-23/D5\|5-18\|RF3/JH3 | ............. ............. ........ | .........N | ........ ...... | ........ ..N.F... .... | ................. ............... | RGV..D........... ......YE.... | ...... ..... |
| | | Germline | H_FR1 | H_CDR1 | H_FR2 | H_CDR2 | H_FR3 | H_CDR3 | H_FR4 |
| | VH3\|3-33/D5\|5-18\|RF1/JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | VDIAM-------------------VYFDY | WGQGTLVTVSS |
| iPS:43 4245 | 21-225_62H1 | VH3\|3-33/D5\|5-18\|RF1/JH4 | ............. ............. ........ | .......... | .......Q ....M. | I....... ........ .... | ................I ............... | E.PRT............ .......SCS.. | ...... ..... |
| iPS:43 4323 | 21-225_62H8 | VH3\|3-33/D5\|5-18\|RF1/JH4 | ............. ............. ........ | T......... | ........ ...... | ...H.... ..D...V. .... | ................. ............... | E.PRT............ .......SCS.. | ...... ..... |
| iPS:43 4379 | 21-225_66A9 | VH3\|3-33/D5\|5-18\|RF1/JH4 | ............. ............. ........ | .......... | ........ ...... | ...H.... ..D..... .... | ................. ............... | E.PRT............ .......SCS.. | ...... ..... |
| | | Germline | H_FR1 | H_CDR1 | H_FR2 | H_CDR2 | H_FR3 | H_CDR3 | H_FR4 |
| | VH3\|3-33/D1\|1-1\|RF3/JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YNWND--------------------YFDY | WGQGTLVTVSS |

| | | | | | | | | | |
|---|---|---|---|---|---|---|---|---|---|
| iPS:43 4247 | 21-225_62D2 | VH3\|3-33/D1\|1-1\|RF3/JH4 | ..Y.........<br>............<br>........ | .......... | ........<br>...... | ........<br>.....S..<br>.... | ..............D..<br>............... | D.G.W............<br>.......N.L.. | ......<br>..... |
| iPS:43 6838 | 21-225_52H4 | VH3\|3-33/D1\|1-1\|RF3/JH4 | ............<br>............<br>........ | H.....F... | ........<br>..K... | ........<br>........<br>.... | .................<br>............... | GD..Y............<br>.......EG... | ......<br>..... |
| iPS:43 6948 | 21-225_183F5 | VH3\|3-33/D1\|1-1\|RF3/JH4 | ............<br>............<br>........ | .........L | ........<br>.....T | ........<br>..G.....<br>.... | .................<br>............... | E.FW-<br>.................<br>...SG.. | ......<br>..... |
| | | Germline | **H_FR1** | **H_CDR1** | **H_FR2** | **H_CDR2** | **H_FR3** | **H_CDR3** | **H_FR4** |
| | VH4\|4-39/D5\|5-12\|RF3/JH4 | | QLQLQES-<br>GPGLVKPSETLS<br>LTCTVSG-GSIS | SS---SYYWG | WIRQPPGK<br>GLEWIG | SIYY----<br>SGSTYYNP<br>SLKS | RVTISVDTSKNQFSLKL<br>SSVTAADTAVYYCAR | GYSGYD-----------<br>-------YYFDY | WGQGTL<br>VTVSS |
| iPS:43 4249 | 21-225_62E2 | VH4\|4-39/D5\|5-12\|RF3/JH4 | ............<br>...........I<br>........ | R......... | ........<br>...... | ........<br>..IAS...<br>.... | .................<br>N....T......... | LS..W-<br>.................<br>.-S... | ......<br>..... |
| iPS:43 4353 | 21-225_64B12 | VH4\|4-39/D5\|5-12\|RF3/JH4 | ............<br>............<br>....V... | R......... | ........<br>...... | ........<br>....S...<br>.... | .................<br>............... | LD..W-<br>.................<br>.-S... | ......<br>..... |
| iPS:39 4073 | 21-225_15C9 | VH4\|4-39/D5\|5-12\|RF3/JH4 | ............<br>............<br>........ | R......... | ........<br>...... | N.......<br>.....N..<br>.... | .................<br>............... | QG..W-<br>.................<br>.-EV.. | ......<br>..... |
| | | Germline | **H_FR1** | **H_CDR1** | **H_FR2** | **H_CDR2** | **H_FR3** | **H_CDR3** | **H_FR4** |
| | VH1\|1-02/D7\|7-27\|RF1/JH4 | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | LTGY-------------<br>---------FDY | WGQGTL<br>VTVSS |
| iPS:43 4259 | 21-225_62G7 | VH1\|1-02/D7\|7-27\|RF1/JH4 | ............<br>............<br>........ | .......... | ........<br>...... | ..K.K...<br>.....Q..<br>.... | .................<br>.S............. | AP.IAAAG.........<br>.....TWGY... | ......<br>..... |
| iPS:43 4347 | 21-225_64H10 | VH1\|1-02/D7\|7-27\|RF1/JH4 | ............<br>............<br>........ | .......... | ........<br>...... | ..K.....<br>.....Q..<br>.... | .................<br>.G............. | AP.TAATG.........<br>.....TWGY... | ......<br>..... |
| iPS:43 4359 | 21-225_65G3 | VH1\|1-02/D7\|7-27\|RF1/JH4 | ............<br>............<br>........ | ........I. | ........<br>...... | ........<br>.....S..<br>.... | .................<br>............... | AP.KAAAG.........<br>.....TWGY... | ......<br>..... |

| | | | | | | | | | |
|---|---|---|---|---|---|---|---|---|---|
| iPS:43 4369 | 21-225_66B1 | VH1\|1-02/D7\|7-27\|RF1/JH4 | ............<br>............<br>........ | .......... | ........<br>...... | ........<br>.....N..<br>.... | ......A..........<br>............... | AP.TAAAG.........<br>.....TWGY... | ......<br>..... |
| iPS:43 4373 | 21-225_66A7 | VH1\|1-02/D7\|7-27\|RF1/JH4 | ............<br>............<br>........ | .......... | ........<br>...... | ..K.....<br>.....Q..<br>.... | ............G....<br>............... | AP.TVAAG.........<br>.....TWGY... | ......<br>..... |
| iPS:43 4397 | 21-225_67H4 | VH1\|1-02/D7\|7-27\|RF1/JH4 | ............<br>............<br>........ | .......... | ........<br>...... | ..K.....<br>.....Q..<br>.... | .................<br>.G............. | AP.TAATG.........<br>.....TWGY... | ......<br>..... |
| iPS:43 4427 | 21-225_70D6 | VH1\|1-02/D7\|7-27\|RF1/JH4 | ............<br>............<br>.....I.. | .......... | ........<br>...... | ....K...<br>.....S..<br>.... | ..S......G......<br>RG......E..... | AP.KAAAG.........<br>.....TWGF... | ......<br>..... |
| iPS:43 4435 | 21-225_70G9 | VH1\|1-02/D7\|7-27\|RF1/JH4 | ............<br>............<br>........ | .......... | ........<br>...... | ..K.....<br>.....Q..<br>.... | .................<br>.S............. | AP.IAAAG.........<br>.....TWGY... | ......<br>..... |
| iPS:43 4437 | 21-225_70A12 | VH1\|1-02/D7\|7-27\|RF1/JH4 | ............<br>............<br>........ | .......... | ........<br>...... | ..K.....<br>.....Q..<br>.... | .................<br>.G............. | AP.TAATG.........<br>.....TWGY... | ......<br>..... |
| iPS:43 4451 | 21-225_71B7 | VH1\|1-02/D7\|7-27\|RF1/JH4 | ............<br>............<br>.....I.. | .......... | ........<br>...... | ........<br>.....S..<br>.... | ..........G......<br>.G............. | AP.KAAAG.........<br>.....TWGF... | ......<br>..... |
| iPS:43 4459 | 21-225_71A7 | VH1\|1-02/D7\|7-27\|RF1/JH4 | ............<br>............<br>........ | .......... | ........<br>...... | ....K...<br>......V.<br>.... | .................<br>.S............. | AP.TAPAG.........<br>.....SWGY... | ......<br>..... |
| iPS:43 4461 | 21-225_73A3 | VH1\|1-02/D7\|7-27\|RF1/JH4 | ............<br>............<br>........ | .......... | ........<br>..D... | ....K...<br>.....HV.<br>.... | ..A..............<br>.S............. | AP.TAAAG.........<br>.....SWGC... | ......<br>..... |
| | | Germline | H_FR1 | H_CDR1 | H_FR2 | H_CDR2 | H_FR3 | H_CDR3 | H_FR4 |
| | VH3\|3-23/D4\|4-11\|RF3/JH4 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | TTVT-------------<br>--------YFDY | WGQGTL<br>VTVSS |
| iPS:43 4261 | 21-225_56F7 | VH3\|3-23/D4\|4-11\|RF3/JH4 | ............<br>............<br>........ | .......VLN | ........<br>...... | .M......<br>..R.....<br>.... | .................<br>...........F..M | ..H-<br>.................<br>....-... | ......<br>..... |

| | | | | | | | | | |
|---|---|---|---|---|---|---|---|---|---|
| iPS:43 5461 | 21-225_153A1 | VH3\|3-23/D4\|4-11\|RF3/JH4 | ............. ............. .....S.. | .......V.. | ....G... ...... | ........ .DR..... .... | .................. ..............R | .AT- .................. ....-K.. | ...... ..... |
| iPS:43 5509 | 21-225_157H1 | VH3\|3-23/D4\|4-11\|RF3/JH4 | ............. ............. ........ | .........R | .I...... ..Q... | D....... ..T..... .... | .................. ............... | .Y-- .................. ....---L | ...... ..... |
| iPS:43 5747 | 21-225_175C4 | VH3\|3-23/D4\|4-11\|RF3/JH4 | ............. ............. ..A..... | .......V.. | ........ ...... | ........ .DR..... .... | .......D.NT...... ..............R | .AG- .................. ....-... | ...... ..... |
| iPS:39 2784 | 21-225_23C7 | VH3\|3-23/D4\|4-11\|RF3/JH4 | ............. ............. ........ | .......V.. | ........ ...... | .M...... ......V. .... | .................. ..............R | .G.- .................. ....-... | ...... .I... |
| iPS:39 2802 | 21-225_23E7 | VH3\|3-23/D4\|4-11\|RF3/JH4 | ............. ...........T ........ | .........N | ........ ...... | ........ ..F..... .... | .........R....... ..............R | .SG- .................. ....-... | ...... ..... |
| iPS:39 2962 | 21-225_30A1 | VH3\|3-23/D4\|4-11\|RF3/JH4 | ............. ............. ........ | .......V.N | ........ ...... | ........ ........ .... | ......N.......... ..............R | .G.- .................. ....-... | ...... ..... |
| iPS:39 3090 | 21-225_33A5 | VH3\|3-23/D4\|4-11\|RF3/JH4 | ............. ............. ........ | .......VIN | ........ ...... | ........ .V...... .... | .................. ..............R | .SL- .................. ....-... | ...... ..... |
| | | Germline | H_FR1 | H_CDR1 | H_FR2 | H_CDR2 | H_FR3 | H_CDR3 | H_FR4 |
| | VH3\|3-23/D1\|1-1\|RF3/JH4 | | EVQLLES-GGGLVQPGGSLRLSCAASG-FTFS | S------YAMS | WVRQAPGKGLEWVS | AISGS---GGSTYYADSVKG | RFTISRDNSKNTLYLQMNSLRAEDTAVYYCAK | YNWND-------------------YFDY | WGQGTLVTVSS |
| iPS:43 4273 | 21-225_57E4 | VH3\|3-23/D1\|1-1\|RF3/JH4 | ............. ............. ........ | .......... | ........ ...... | V....... ....F... .... | ..........T...... ............... | RD................. ........V... | ...... ..... |
| | | Germline | H_FR1 | H_CDR1 | H_FR2 | H_CDR2 | H_FR3 | H_CDR3 | H_FR4 |
| | VH3\|3-23/D3\|3-3\|RF3/JH4 | | EVQLLES-GGGLVQPGGSLRLSCAASG-FTFS | S------YAMS | WVRQAPGKGLEWVS | AISGS---GGSTYYADSVKG | RFTISRDNSKNTLYLQMNSLRAEDTAVYYCAK | ITIFGVV-----------------IIYFDY | WGQGTLVTVSS |
| iPS:43 4279 | 21-225_57F7 | VH3\|3-23/D3\|3-3\|RF3/JH4 | ............. ............. ........ | .......... | ........ ...... | ........ ..N.F... .... | .................. ............... | FFGVVG- .................V GC... | ...... ..... |

| | | | | | | | | | |
|---|---|---|---|---|---|---|---|---|---|
| iPS:43 7228 | 21-225_60C11 | VH3\|3-23/D3\|3-3\|RF3/JH4 | ............<br>............<br>.....P.. | .......... | ........<br>...... | ........<br>..N.F...<br>.... | .................<br>............... | FFGVVG-<br>.................V<br>GC... | ......<br>..... |
| | | Germline | H_FR1 | H_CDR1 | H_FR2 | H_CDR2 | H_FR3 | H_CDR3 | H_FR4 |
| | VH3\|3-23/D3\|3-22\|RF3/JH1 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | ITMIVVVI----------<br>-----TAEYFQH | WGQGTL<br>VTVSS |
| iPS:43 4291 | 21-225_58A4 | VH3\|3-23/D3\|3-22\|RF3/JH1 | ............<br>............<br>....L... | .......... | ....D...<br>...... | ........<br>..N.F.G.<br>.... | ..........K......<br>............... | FFG..G--<br>...............-<br>.GF.DS | ......<br>..... |
| | | Germline | H_FR1 | H_CDR1 | H_FR2 | H_CDR2 | H_FR3 | H_CDR3 | H_FR4 |
| | VH3\|3-33/D2\|2-15\|RF3/JH4 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | DIVVVVA-----------<br>------ATYFDY | WGQGTL<br>VTVSS |
| iPS:43 4299 | 21-225_58D11 | VH3\|3-33/D2\|2-15\|RF3/JH4 | ............<br>............<br>........ | D......DI. | ....S...<br>...... | ........<br>..K.....<br>.... | .................<br>.........L..... | .R.T---<br>.................-<br>--... | ......<br>..... |
| iPS:43 4871 | 21-225_85H1 | VH3\|3-33/D2\|2-15\|RF3/JH4 | ............<br>............<br>........ | .........I. | ........<br>...... | ........<br>........<br>.... | .................<br>............... | .PFI.G-<br>..................<br>..... | ......<br>..... |
| iPS:43 5109 | 21-225_92H5 | VH3\|3-33/D2\|2-15\|RF3/JH4 | ............<br>............<br>........ | .......... | ........<br>...... | ........<br>........<br>.... | .................<br>............... | .PFI.G-<br>..................<br>..... | ......<br>..... |
| iPS:43 6434 | 21-225_216B10 | VH3\|3-33/D2\|2-15\|RF3/JH4 | ............<br>............<br>........ | .......... | ........<br>...... | A.......<br>........<br>.... | ..........R......<br>............... | .PNI.G-<br>..................<br>.W... | ......<br>..... |
| | | Germline | H_FR1 | H_CDR1 | H_FR2 | H_CDR2 | H_FR3 | H_CDR3 | H_FR4 |
| | VH1\|1-02/D4\|4-17\|RF2/JH4 | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | DYGDY-------------<br>---------YFDY | WGQGTL<br>VTVSS |
| iPS:43 4307 | 21-225_59B2 | VH1\|1-02/D4\|4-17\|RF2/JH4 | ............<br>............<br>.......A | .........I. | ........<br>....L. | ........<br>........<br>.... | .................<br>....F.......... | .P.P-<br>..................<br>...-... | ......<br>..... |
| | | Germline | H_FR1 | H_CDR1 | H_FR2 | H_CDR2 | H_FR3 | H_CDR3 | H_FR4 |
| | VH3\|3-33/D3\|3-22\|RF3/JH6 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | ITMIVVVIT---------<br>----YYYYYGMDV | WGQGTT<br>VTVSS |

| | | | | | | | | | |
|---|---|---|---|---|---|---|---|---|---|
| iPS:43 4311 | 21-225_59H5 | VH3\|3-33/D3\|3-22\|RF3/JH6 | ............<br>............<br>........ | .......... | ........<br>...... | ........<br>........<br>.... | .................<br>............... | ERG.A.G--<br>............---<br>...... | ......<br>..... |
| | | Germline | H_FR1 | H_CDR1 | H_FR2 | H_CDR2 | H_FR3 | H_CDR3 | H_FR4 |
| | VH4\|4-39/D1\|1-26\|RF3/JH4 | | QLQLQES-<br>GPGLVKPSETLS<br>LTCTVSG-GSIS | SS---SYYWG | WIRQPPGK<br>GLEWIG | SIYY----<br>SGSTYYNP<br>SLKS | RVTISVDTSKNQFSLKL<br>SSVTAADTAVYYCAR | YSGSY-----------<br>-------YYFDY | WGQGTL<br>VTVSS |
| iPS:43 4313 | 21-225_59E6 | VH4\|4-39/D1\|1-26\|RF3/JH4 | ............<br>............<br>........ | R......... | ........<br>...... | N.......<br>........<br>.... | .................<br>.........L..... | H.S.W............<br>.......-SL.. | ......<br>..... |
| iPS:43 4413 | 21-225_68D12 | VH4\|4-39/D1\|1-26\|RF3/JH4 | ............<br>............<br>........ | R......... | ........<br>...... | N.......<br>......I.<br>.... | .................<br>T.............. | H.T.W............<br>.......-SI.. | ......<br>..... |
| iPS:39 2628 | 21-225_20C2 | VH4\|4-39/D1\|1-26\|RF3/JH4 | ............<br>............<br>........ | R......... | ........<br>...... | N.......<br>..TA.C.S<br>.... | ..I..............<br>.....T......... | H.S.W............<br>.......-SL.N | ......<br>..... |
| iPS:39 2642 | 21-225_18C6 | VH4\|4-39/D1\|1-26\|RF3/JH4 | ............<br>............<br>........ | R......... | ........<br>...... | N.......<br>..Y.....<br>.... | ..I..............<br>.........L..... | H.S.W............<br>.......-SL.D | ......<br>..... |
| iPS:39 2706 | 21-225_18A3 | VH4\|4-39/D1\|1-26\|RF3/JH4 | ............<br>..F.........<br>........ | R......... | ........<br>...... | N.......<br>..Y...T.<br>.... | .................<br>............... | H.T.W............<br>.......-SL.. | ......<br>..... |
| iPS:39 2800 | 21-225_22D12 | VH4\|4-39/D1\|1-26\|RF3/JH4 | ............<br>............<br>........ | R......... | ........<br>...... | N.......<br>..T.S...<br>.... | .........R.......<br>..........F.... | L.S.W............<br>.......-SV.. | ......<br>..... |
| iPS:39 2820 | 21-225_23D1 | VH4\|4-39/D1\|1-26\|RF3/JH4 | ............<br>............<br>........ | R......... | ........<br>...... | ........<br>...AQ...<br>.... | ................T.<br>.........L..... | L.S.W............<br>.......-S... | ......<br>..... |
| iPS:39 2824 | 21-225_24E5 | VH4\|4-39/D1\|1-26\|RF3/JH4 | ............<br>............<br>.....A.. | R......... | ........<br>...... | ........<br>...AN...<br>.... | .................<br>............... | L.S.W............<br>.......-SI.N | ......<br>..... |
| iPS:39 2834 | 21-225_22C1 | VH4\|4-39/D1\|1-26\|RF3/JH4 | ............<br>............<br>.......N | R......... | ........<br>...... | N.......<br>..A....S<br>.... | .........T.......<br>............... | H...W............<br>.......-SL.. | ......<br>..... |

| | | | | | | | | | |
|---|---|---|---|---|---|---|---|---|---|
| iPS:39 2870 | 21-225_20G9 | VH4\|4-39/D1\|1-26\|RF3/JH4 | ............<br>............<br>........ | R......... | ........<br>...... | ........<br>...AS...<br>.... | .........R.......<br>.........A..... | L.S.W............<br>.......-S... | ......<br>..... |
| iPS:39 2896 | 21-225_21G7 | VH4\|4-39/D1\|1-26\|RF3/JH4 | ............<br>..F.........<br>........ | R......... | ........<br>.Q.... | N.......<br>..YS....<br>.... | .................<br>............... | H.T.W............<br>.......-SL.. | ......<br>..... |
| iPS:39 2904 | 21-225_22G9 | VH4\|4-39/D1\|1-26\|RF3/JH4 | ............<br>............<br>.....A.. | G....N.... | ........<br>E..... | N.......<br>........<br>.... | .................<br>R....E......... | H.S.W............<br>.......-SL.. | ......<br>..... |
| iPS:39 3094 | 21-225_34C4 | VH4\|4-39/D1\|1-26\|RF3/JH4 | ............<br>............<br>........ | R......... | ....S...<br>...... | ........<br>....A...<br>.... | ............V....<br>............... | L.S.W............<br>.......-S... | ......<br>..... |
| iPS:39 3806 | 21-225_6A6 | VH4\|4-39/D1\|1-26\|RF3/JH4 | ............<br>............<br>........ | R......... | ........<br>...... | N.......<br>..IP....<br>.... | ..N..............<br>N.............. | H.S.W............<br>.......-SL.. | ......<br>..... |
| iPS:39 3814 | 21-225_7F4 | VH4\|4-39/D1\|1-26\|RF3/JH4 | ............<br>............<br>........ | R......... | ........<br>...... | N.......<br>..A.....<br>.... | .........T.......<br>............... | H...W............<br>.......-SL.. | ......<br>..... |
| iPS:39 3816 | 21-225_6D4 | VH4\|4-39/D1\|1-26\|RF3/JH4 | ............<br>............<br>........ | R.....S... | ........<br>...... | N.......<br>...A..I.<br>.... | ......A........N.<br>T.............. | H.S.W............<br>.......-SL.C | ......<br>..... |
| iPS:39 3880 | 21-225_15A1 | VH4\|4-39/D1\|1-26\|RF3/JH4 | ..H.........<br>............<br>........ | R......... | ........<br>...... | ........<br>...AQ...<br>.... | ........T......T.<br>............... | L.S.W............<br>.......-S... | ......<br>..... |
| iPS:39 4002 | 21-225_15G7 | VH4\|4-39/D1\|1-26\|RF3/JH4 | ............<br>............<br>........ | R.....S... | ........<br>...... | ........<br>..Y...T.<br>.... | ...............R.<br>.........S..... | L.S.W............<br>.......-S..F | ......<br>..... |
| iPS:39 4053 | 21-225_11F10 | VH4\|4-39/D1\|1-26\|RF3/JH4 | ..H.........<br>............<br>....A... | R......... | ........<br>...... | ........<br>...AQ...<br>.... | ...............T.<br>............... | L.S.W............<br>.......-S... | ......<br>..... |
| iPS:39 4057 | 21-225_15H1 | VH4\|4-39/D1\|1-26\|RF3/JH4 | ............<br>..F.........<br>........ | R......... | ........<br>...... | N.......<br>..YP....<br>.... | .................<br>............... | H.T.W............<br>.......-SL.. | ......<br>..... |
| iPS:39 | 21-225_16A1 | VH4\|4-39/D1\|1- | ............<br>.........I.. | R......... | ........<br>...... | ........<br>...A.H.. | .G...............<br>.........A..... | L.S.W............<br>.......-S... | ......<br>..... |

| | | | | | | | | | |
|---|---|---|---|---|---|---|---|---|---|
| iPS:394075 | 21-225_8D12 | VH4\|4-39/D1\|1-26\|RF3/JH4 | ............<br>..F.........<br>........ | R......... | ........<br>...... | N.......<br>..YP....<br>.... | .....I...........<br>................ | H.T.W.............<br>.......-SL.. | ......<br>..... |
| | | Germline | H_FR1 | H_CDR1 | H_FR2 | H_CDR2 | H_FR3 | H_CDR3 | H_FR4 |
| | VH3\|3-48/D7\|7-27\|RF3/JH4 | | EVQLVES-<br>GGGLVQPGGSLR<br>LSCAASG-FTFS | S-----YSMN | WVRQAPGK<br>GLEWVS | YISSS---<br>SSTIYYAD<br>SVKG | RFTISRDNAKNSLYLQM<br>NSLRDEDTAVYYCAR | NWGY-------------<br>---------FDY | WGQGTL<br>VTVSS |
| iPS:434317 | 21-225_59E8 | VH3\|3-48/D7\|7-27\|RF3/JH4 | ............<br>............<br>........ | .......... | ........<br>..G... | ........<br>.G......<br>.... | .................<br>................ | E..MAV...........<br>......AGP... | ......<br>..... |
| | | Germline | H_FR1 | H_CDR1 | H_FR2 | H_CDR2 | H_FR3 | H_CDR3 | H_FR4 |
| | VH3\|3-33/D1\|1-26\|RF3/JH4 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | YSGSY------------<br>-------YYFDY | WGQGTL<br>VTVSS |
| iPS:434327 | 21-225_63G6 | VH3\|3-33/D1\|1-26\|RF3/JH4 | ............<br>............<br>........ | .......... | ........<br>.....T | ........<br>........<br>.... | .................<br>................ | D.L.GI...........<br>......AAA... | ......<br>..... |
| iPS:437084 | 21-225_206B5 | VH3\|3-33/D1\|1-26\|RF3/JH4 | ............<br>............<br>........ | .......... | ........<br>...... | ........<br>........<br>.... | .................<br>...........N... | EG...............<br>.......-HL.. | ....I.<br>..... |
| iPS:437088 | 21-225_209H10 | VH3\|3-33/D1\|1-26\|RF3/JH4 | ............<br>............<br>........ | .......... | ........<br>...... | ........<br>........<br>.... | .................<br>...........N... | EG...............<br>.......-HL.. | ....I.<br>..... |
| iPS:392684 | 21-225_17F4 | VH3\|3-33/D1\|1-26\|RF3/JH4 | ............<br>............<br>........ | N........N | ........<br>...... | ........<br>.N..H...<br>.... | .................<br>..............S | SGSY-<br>.................<br>..-F... | ......<br>..... |
| | | Germline | H_FR1 | H_CDR1 | H_FR2 | H_CDR2 | H_FR3 | H_CDR3 | H_FR4 |
| | VH1\|1-02/D6\|6-19\|RF2/JH4 | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | GIAVA------------<br>-------GYFDY | WGQGTL<br>VTVSS |
| iPS:434333 | 21-225_63C9 | VH1\|1-02/D6\|6-19\|RF2/JH4 | ............<br>............<br>........ | .......... | ........<br>...... | ........<br>.....F..<br>.... | .......A..N......<br>RS.I........... | APG..AAG.........<br>.....SW..... | ......<br>..... |
| | | Germline | H_FR1 | H_CDR1 | H_FR2 | H_CDR2 | H_FR3 | H_CDR3 | H_FR4 |
| | VH3\|3-33/D4\|4-11\|RF2/JH4 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | DYSNY------------<br>---------YFDY | WGQGTL<br>VTVSS |

| | | | | | | | | | |
|---|---|---|---|---|---|---|---|---|---|
| iPS:43 4335 | 21-225_63C10 | VH3\|3-33/D4\|4-11\|RF2/JH4 | ............... ............... ........ | ........... | ........ ..D... | ........ ........ .... | ................. ............... | .DPRS............ .......SAG.. | ...... ..... |
| iPS:43 4429 | 21-225_70H6 | VH3\|3-33/D4\|4-11\|RF2/JH4 | ............... ............... ........ | ........... | ....T... ..D... | ...H.... ........ .... | ................. ............... | .DPRS............ .......SAG.. | ...... ..... |
| iPS:43 4569 | 21-225_77H5 | VH3\|3-33/D4\|4-11\|RF2/JH4 | ............... ............... ........ | N.......... | ........ ...... | ........ .R...... .... | ................. .........L..... | .R.ILG........... ......ATF... | ...... ..... |
| iPS:43 4629 | 21-225_74C3 | VH3\|3-33/D4\|4-11\|RF2/JH4 | ............... ............... ........ | ......F... | .A...... ...... | A....... .....C.. .... | .............S... .......S....... | .R.ILG........... ......AAF... | ...... ..... |
| iPS:43 5793 | 21-225_180F8 | VH3\|3-33/D4\|4-11\|RF2/JH4 | ............... ............... ........ | ........... | ........ .....S | ........ ..D...E. .... | ................. ............... | .HPRW............ .......S.G.. | ...... ..... |
| iPS:43 6382 | 21-225_212C10 | VH3\|3-33/D4\|4-11\|RF2/JH4 | ............... ............... ........ | ........... | ........ .....T | A....... ..H...T. .... | ................. ............... | .R.IVG........... ......AT.... | ...... ..... |
| iPS:39 2660 | 21-225_19B3 | VH3\|3-33/D4\|4-11\|RF2/JH4 | ............... ............... ........ | .......D.. | ........ ...... | ........ ..D..... .... | .............F... ............... | .RAYS............ .......SSS.. | ...... ..... |
| iPS:39 3904 | 21-225_8H11 | VH3\|3-33/D4\|4-11\|RF2/JH4 | ............... ............... ........ | T......N.. | ........ ...... | ........ ..DR.S.. .... | ................. ............... | .RAYS............ .......SSS.F | ...... ..... |
| | | Germline | H_FR1 | H_CDR1 | H_FR2 | H_CDR2 | H_FR3 | H_CDR3 | H_FR4 |
| | VH4\|4-59/D4\|4-11\|RF2/JH4 | | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAR | DYSNY------------------YFDY | WGQGTLVTVSS |
| iPS:43 4341 | 21-225_64F7 | VH4\|4-59/D4\|4-11\|RF2/JH4 | ............... ............... ........ | .......F.. | .....A.. ...... | R..T.... ..IS.... .... | ...M............. ............... | FS.G- ................... ...F... | ...... ..... |
| | | Germline | H_FR1 | H_CDR1 | H_FR2 | H_CDR2 | H_FR3 | H_CDR3 | H_FR4 |
| | VH3\|3-33/D7\|7-27\|RF2/JH1 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | *LGAE------------------YFQH | WGQGTLVTVSS |

| | | | | | | | | | |
|---|---|---|---|---|---|---|---|---|---|
| iPS:43 4351 | 21-225_64A12 | VH3\|3-33/D7\|7-27\|RF2/JH1 | ................................ | .......... | .............. | ........E.....V..... | ................................ | E..F-......................LSD. | ........... |
| iPS:39 2700 | 21-225_16E12 | VH3\|3-33/D7\|7-27\|RF2/JH1 | ...........L.................... | N......... | .............. | ....E.........V...R. | ...........S...................V. | E..F-......................QSD. | .....P..... |
| iPS:39 2710 | 21-225_19A10 | VH3\|3-33/D7\|7-27\|RF2/JH1 | ................................ | .......... | .............. | ........E........... | ................................ | E.AW-.......................EDS | ........... |
| iPS:39 4093 | 21-225_9D12 | VH3\|3-33/D7\|7-27\|RF2/JH1 | ................................ | D......... | .............. | .........N.N........ | ................................ | E.AW-.......................EDF | ........... |
| | | Germline | H_FR1 | H_CDR1 | H_FR2 | H_CDR2 | H_FR3 | H_CDR3 | H_FR4 |
| | VH3\|3-23/D3\|3-22\|RF2/JH3 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | YYYDSSGY----------------YYDAFDI | WGQGTMVTVSS |
| iPS:43 4355 | 21-225_64G12 | VH3\|3-23/D3\|3-22\|RF2/JH3 | ................................ | ......N... | .............. | V................... | .........................L..... | RN..D---...............---.... | ........... |
| | | Germline | H_FR1 | H_CDR1 | H_FR2 | H_CDR2 | H_FR3 | H_CDR3 | H_FR4 |
| | VH3\|3-21/D4\|4-11\|RF3/JH4 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | TTVT--------------------YFDY | WGQGTLVTVSS |
| iPS:43 4367 | 21-225_65H11 | VH3\|3-21/D4\|4-11\|RF3/JH4 | ................................ | .......R.. | .............. | ........N.S......... | ...T..............................TS | .S--...................--GS | ........... |
| iPS:43 7220 | 21-225_55H6 | VH3\|3-21/D4\|4-11\|RF3/JH4 | ..H............................. | .......... | .............. | ...G.....T.......... | ................................ | .G.-.......................-... | ........... |
| iPS:40 2237 | 21-225_23D11 | VH3\|3-21/D4\|4-11\|RF3/JH4 | ................................ | .......NI. | .............. | ...GN....G.......... | ................................ | .NL-.......................-... | ........... |
| | | Germline | H_FR1 | H_CDR1 | H_FR2 | H_CDR2 | H_FR3 | H_CDR3 | H_FR4 |
| | VH3\|3-21/D4\|4-11\|RF3/JH3 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | TTVTD----------------------AFDI | WGQGTMVTVSS |

| | | | | | | | | | |
|---|---|---|---|---|---|---|---|---|---|
| iPS:43 4383 | 21-225_66F9 | VH3\|3-21/D4\|4-11\|RF3/JH3 | ............ ............ ........ | .......... | ........ ...... | ...GT... ........ .... | ................. .G.........F... | .NA-- .................. ...-... | ...... ..... |
| iPS:43 4449 | 21-225_71H6 | VH3\|3-21/D4\|4-11\|RF3/JH3 | ............ ............ ........ | .......... | ........ ...... | ...GT... ........ .... | ................. .G.........F..K | .NA-- .................. ...-... | ...... ..... |
| | | Germline | H_FR1 | H_CDR1 | H_FR2 | H_CDR2 | H_FR3 | H_CDR3 | H_FR4 |
| | VH3\|3-33/D6\|6-6\|RF2/JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | SIAAR-------------------YFDY | WGQGTLVTVSS |
| iPS:43 4399 | 21-225_67B7 | VH3\|3-33/D6\|6-6\|RF2/JH4 | ............ ............ ........ | N......... | ........ ...... | ..L..... ..K..... .... | ................. ............... | ..PE- .................. ...-... | ...... ..... |
| iPS:43 4463 | 21-225_73A6 | VH3\|3-33/D6\|6-6\|RF2/JH4 | ............ ............ ........ | Y......... | ........ ...... | ..L..... ..K....A .... | ................. ............... | ..PD- .................. ...-... | ...... ..... |
| | | Germline | H_FR1 | H_CDR1 | H_FR2 | H_CDR2 | H_FR3 | H_CDR3 | H_FR4 |
| | VH3\|3-21/D1\|1-26\|RF3/JH4 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | YSGSY-----------------YYFDY | WGQGTLVTVSS |
| iPS:43 4405 | 21-225_68E6 | VH3\|3-21/D1\|1-26\|RF3/JH4 | ............ ............ ......L. | ......FG.. | ........ ...... | Y..R.... ..H..... .... | ................. ..............V | S...- .................. ..-P... | ...... ..... |
| iPS:43 5595 | 21-225_160H4 | VH3\|3-21/D1\|1-26\|RF3/JH4 | ............ ..S......... ........ | .......R.. | ........ ...... | ...G.... ....D... .... | ................. ............... | K.W-- .................. ..--... | ...... ..... |
| iPS:43 5635 | 21-225_163F1 | VH3\|3-21/D1\|1-26\|RF3/JH4 | .....D...... ............ ........ | .......... | ........ ...... | ...G.... G....... .... | ...T............. ...S.D.......TL | ..S-- .................. ..--SH. | ...... ..... |
| | | Germline | H_FR1 | H_CDR1 | H_FR2 | H_CDR2 | H_FR3 | H_CDR3 | H_FR4 |
| | VH3\|3-30.3/D2\|2-15\|RF3/JH4 | | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | DIVVVVA-----------------ATYFDY | WGQGTLVTVSS |
| iPS:43 4417 | 21-225_69C8 | VH3\|3-30.3/D2\|2-15\|RF3/JH4 | ............ ............ ........ | N......... | ........ ...... | ..W..... ..D..... .... | ................. I.............. | ..PSN-- .................- SAG.. | ...... ..... |

| | | | | | | | | | |
|---|---|---|---|---|---|---|---|---|---|
| | VH3\|3-33/D3\|3-10\|RF1/JH4 | | QVQLVES- GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | VLLWFGE----------- -----LL*YFDY | WGQGTL VTVSS |
| iPS:43 4433 | 21-225_70E8 | VH3\|3-33/D3\|3-10\|RF1/J H4 | ............ ............ ........ | .........L | ........ ...... | I....... E....... .... | ................. ............... | D..D--- ................-- -PR.. | ...... ..... |
| | | Germline | H_FR1 | H_CDR1 | H_FR2 | H_CDR2 | H_FR3 | H_CDR3 | H_FR4 |
| | VH3\|3-23/D6\|6-13\|RF1/JH4 | | EVQLLES- GGGLVQPGGSLR LSCAASG-FTFS | S-----YAMS | WVRQAPGK GLEWVS | AISGS--- GGSTYYAD SVKG | RFTISRDNSKNTLYLQM NSLRAEDTAVYYCAK | GYSSSW----------- -------YYFDY | WGQGTL VTVSS |
| iPS:43 4467 | 21-225_73H8 | VH3\|3-23/D6\|6-13\|RF1/J H4 | ...........W ..S......... ........ | ......N... | ........ ...... | D..R.... ..T.F... .... | ................. ............... | WD....Y.......... .....DVTP... | ...... ..... |
| | | Germline | H_FR1 | H_CDR1 | H_FR2 | H_CDR2 | H_FR3 | H_CDR3 | H_FR4 |
| | VH4\|4-34/D4\|4-17\|RF2/JH4 | | QVQLQQW- GAGLLKPSETLS LTCAVYG-GSFS | G-----YYWS | WIRQPPGK GLEWIG | EINH---- SGSTNYNP SLKS | RVTISVDTSKNQFSLKL SSVTAADTAVYYCAR | DYGDY------------ --------YFDY | WGQGTL VTVSS |
| iPS:43 4471 | 21-225_75G3 | VH4\|4-34/D4\|4-17\|RF2/J H4 | ............ ............ ......L. | ......S... | ........ ...... | ...L.... ........ .... | ..........S....T. R............. | ...G- ................. ...-L.. | ...... ..... |
| iPS:43 4517 | 21-225_76A7 | VH4\|4-34/D4\|4-17\|RF2/J H4 | ............ ............ ........ | ......C... | ........ ...... | ........ ..R..... .... | .........E....... ............... | ...G- ................. ...-L.. | ....A. ..... |
| iPS:43 4519 | 21-225_74C7 | VH4\|4-34/D4\|4-17\|RF2/J H4 | ............ ............ ......L. | ......S... | ........ ...... | ...L.... ........ .... | ..........S....T. R............. | ...G- ................. ...-L.. | ...... ..... |
| iPS:43 4571 | 21-225_74D2 | VH4\|4-34/D4\|4-17\|RF2/J H4 | ............ ............ ........ | ......C... | ........ ...... | ........ ..R..... .... | .........E....... ............... | ...G- ................. ...-L.. | ...... ..... |
| iPS:43 4637 | 21-225_78E7 | VH4\|4-34/D4\|4-17\|RF2/J H4 | ............ ............ ......L. | ......S... | ........ ...... | ...L.... ........ .... | ..........S....T. R............. | ...G- ................. ...-L.. | ...... ..... |
| iPS:43 4717 | 21-225_80A6 | VH4\|4-34/D4\|4-17\|RF2/J H4 | ............ ............ ........ | ......C... | ........ ...... | ........ ..R..... .... | .........EK...... ............... | ...G- ................. ...-L.. | ...... ..... |
| iPS:43 | 21- | VH4\|4-34/D4\|4- | ............ ............ | ......C... | ........ ...... | ........ ........ | ................. ............... | ...G- ................. | ...... ..... |

| | | | | | | | | | |
|---|---|---|---|---|---|---|---|---|---|
| iPS:43 4835 | 21-225_83B6 | VH4\|4-34/D4\|4-17\|RF2/J H4 | ............<br>............<br>........ | ......C... | ........<br>...... | ........<br>..R.....<br>.... | .........E.......<br>............... | ...G-<br>...................<br>...-L.. | ......<br>..... |
| iPS:43 4849 | 21-225_83C10 | VH4\|4-34/D4\|4-17\|RF2/J H4 | ............<br>...........T<br>........ | .......... | ........<br>...... | ........<br>.....F..<br>.... | .................<br>..........F.... | ...G-<br>...................<br>...-L.. | ......<br>..... |
| iPS:43 4891 | 21-225_85G6 | VH4\|4-34/D4\|4-17\|RF2/J H4 | ............<br>............<br>........ | D.....C... | ........<br>...... | ........<br>..R.....<br>.... | .........E.......<br>............... | ...G-<br>...................<br>...-L.. | ......<br>..... |
| iPS:43 5183 | 21-225_93E9 | VH4\|4-34/D4\|4-17\|RF2/J H4 | ............<br>............<br>........ | ......C... | ........<br>...... | ........<br>..R.....<br>.... | .........E.K.....<br>............... | ...G-<br>...................<br>...-L.. | ......<br>..... |
| iPS:43 5331 | 21-225_147G8 | VH4\|4-34/D4\|4-17\|RF2/J H4 | ............<br>...........G<br>........ | A......... | ........<br>...... | ........<br>......K.<br>.... | .................<br>............... | ...V-<br>...................<br>...-... | ......<br>..... |
| iPS:43 5995 | 21-225_192F8 | VH4\|4-34/D4\|4-17\|RF2/J H4 | ............<br>............<br>........ | ......C... | ........<br>...... | ...Q....<br>..RS....<br>.... | .........T.......<br>R.............. | ...V-<br>...................<br>...-... | ......<br>..... |
| iPS:43 6027 | 21-225_193E6 | VH4\|4-34/D4\|4-17\|RF2/J H4 | ............<br>............<br>.F...... | ......P... | ........<br>...... | .S......<br>..R.....<br>.... | ...............R.<br>............... | ...G-<br>...................<br>...-L.. | ......<br>..... |
| iPS:43 6080 | 21-225_195B1 | VH4\|4-34/D4\|4-17\|RF2/J H4 | ............<br>............<br>.S.....R | Y.....F.. | ....S...<br>....F. | ........<br>..R.....<br>.... | .................<br>R.............. | ...A-<br>...................<br>...-..I | ......<br>..... |
| iPS:43 6232 | 21-225_201E1 | VH4\|4-34/D4\|4-17\|RF2/J H4 | ............<br>............<br>.FD..... | P......... | ........<br>...... | .V......<br>........<br>.... | .................<br>............... | ...G-<br>...................<br>...-L.. | ....A.<br>..... |
| iPS:43 6238 | 21-225_201B2 | VH4\|4-34/D4\|4-17\|RF2/J H4 | ............<br>............<br>.F....I. | V........T | ........<br>...... | ........<br>........<br>.... | .................<br>............... | ...V-<br>...................<br>...-... | ......<br>..... |
| iPS:43 6256 | 21-225_202D9 | VH4\|4-34/D4\|4-17\|RF2/J H4 | ............<br>............<br>..D..... | P......... | ........<br>...... | ........<br>........<br>.... | .................<br>............... | ...G-<br>...................<br>...-L.. | ......<br>..... |
| iPS:43 | 21- | VH4\|4-34/D4\|4- | ............<br>............ | V......... | ........<br>...... | .S.Q....<br>..R.T... | ...............N.<br>I.............. | ...V-<br>................... | ......<br>..... |

| | | | | | | | | | |
|---|---|---|---|---|---|---|---|---|---|
| iPS:43 6310 | 21-225_202D11 | VH4\|4-34/D4\|4-17\|RF2/JH4 | ......R.....<br>............<br>A....... | ......P... | ........<br>...... | ........<br>........<br>.... | ..................<br>................ | ...V-<br>....................<br>...-L.. | ......<br>..... |
| iPS:43 6336 | 21-225_208B5 | VH4\|4-34/D4\|4-17\|RF2/JH4 | ............<br>............<br>.F....I. | V........T | ........<br>...... | ........<br>........<br>.... | ..................<br>................ | ...V-<br>....................<br>...-... | ......<br>..... |
| iPS:43 6340 | 21-225_208A9 | VH4\|4-34/D4\|4-17\|RF2/JH4 | ............<br>.....P......<br>........ | V.....S... | ........<br>...... | ........<br>..RA....<br>.... | .....I............<br>................ | ...V-<br>....................<br>...-L.. | ......<br>..... |
| iPS:43 7340 | 21-225_75G9 | VH4\|4-34/D4\|4-17\|RF2/JH4 | ............<br>............<br>........ | ......C... | ........<br>...... | ........<br>..R.....<br>.... | .........E........<br>................ | ...G-<br>....................<br>...-L.. | ......<br>..... |
| iPS:45 1122 | 21-225_200A1 | VH4\|4-34/D4\|4-17\|RF2/JH4 | ............<br>............<br>........ | V......... | ........<br>...... | ........<br>........<br>.... | .....L............<br>................ | ...V-<br>....................<br>...-... | ......<br>..... |
| | | Germline | H_FR1 | H_CDR1 | H_FR2 | H_CDR2 | H_FR3 | H_CDR3 | H_FR4 |
| | VH3\|3-33/D2\|2-15\|RF3/JH1 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | DIVVVVAA----------<br>-----TAEYFQH | WGQGTL<br>VTVSS |
| iPS:43 4485 | 21-225_76D2 | VH3\|3-33/D2\|2-15\|RF3/JH1 | ............<br>............<br>........ | .......... | ........<br>...... | ........<br>........<br>.... | .............F...<br>..............S | .RNI.G--<br>..............-<br>.T..ES | ......<br>..... |
| iPS:43 4537 | 21-225_74E11 | VH3\|3-33/D2\|2-15\|RF3/JH1 | ............<br>............<br>........ | .......... | ........<br>...... | ........<br>........<br>.... | .............F...<br>..............S | .RNI.G--<br>..............-<br>.T..ES | ......<br>..... |
| iPS:43 4673 | 21-225_74E3 | VH3\|3-33/D2\|2-15\|RF3/JH1 | ............<br>...........T<br>........ | .......... | ........<br>...... | ........<br>........<br>.... | .............F...<br>..............S | .RNI.G--<br>..............-<br>.T..ES | ......<br>..... |
| iPS:43 5221 | 21-225_95G2 | VH3\|3-33/D2\|2-15\|RF3/JH1 | ............<br>...........T<br>........ | .......... | ........<br>...... | ........<br>........<br>.... | .............F...<br>..............S | .RNI.G--<br>..............-<br>.T..ES | ......<br>..... |
| | | Germline | H_FR1 | H_CDR1 | H_FR2 | H_CDR2 | H_FR3 | H_CDR3 | H_FR4 |
| | VH4\|4-39/D7\|7-27\|RF1/JH5 | | QLQLQES-<br>GPGLVKPSETLS<br>LTCTVSG-GSIS | SS---SYYWG | WIRQPPGK<br>GLEWIG | SIYY----<br>SGSTYYNP<br>SLKS | RVTISVDTSKNQFSLKL<br>SSVTAADTAVYYCAR | LTGN-------------<br>--------WFDP | WGQGTL<br>VTVSS |

| | | | | | | | | | |
|---|---|---|---|---|---|---|---|---|---|
| iPS:43 4503 | 21-225_74D7 | VH4\|4-39/D7\|7-27\|RF1/JH5 | ............<br>...........S<br>.......F | R......... | ........<br>...... | G.......<br>....S...<br>.... | .........E.......<br>............... | .RP.W............<br>........D..Y | ......<br>..... |
| | | Germline | H_FR1 | H_CDR1 | H_FR2 | H_CDR2 | H_FR3 | H_CDR3 | H_FR4 |
| | VH3\|3-15/D1\|1-1\|RF2/JH4 | | EVQLVES-<br>GGGLVKPGGSLR<br>LSCAASG-FTFS | N-----AWMS | WVRQAPGK<br>GLEWVG | RIKSKT-<br>DGGTTDYA<br>APVKG | RFTISRDDSKNTLYLQM<br>NSLKTEDTAVYYCTT | VQLER------------<br>--------YFDY | WGQGTL<br>VTVSS |
| iPS:43 4531 | 21-225_76C9 | VH3\|3-15/D1\|1-1\|RF2/JH4 | ............<br>............<br>.....S.. | .........N | ........<br>...... | ...N.A..<br>.....F..<br>.... | ..........H......<br>............... | .GPT-<br>.................<br>...-T.. | ......<br>..... |
| iPS:43 4633 | 21-225_74G8 | VH3\|3-15/D1\|1-1\|RF2/JH4 | ............<br>............<br>........ | .........N | ........<br>...... | ...N.A..<br>........<br>.... | .................<br>............... | .GAT-<br>.................<br>...-T.. | ......<br>..... |
| iPS:43 4671 | 21-225_74F4 | VH3\|3-15/D1\|1-1\|RF2/JH4 | ............<br>............<br>........ | .........N | ........<br>...... | ...N.I..<br>........<br>.... | .................<br>............... | .GAT-<br>.................<br>...-T.. | ......<br>..... |
| iPS:43 7383 | 21-225_74H8 | VH3\|3-15/D1\|1-1\|RF2/JH4 | ..H.........<br>............<br>........ | .........N | ........<br>...... | ........<br>........<br>.... | .................<br>............... | .GAT-<br>.................<br>...-T.. | ......<br>..... |
| | | Germline | H_FR1 | H_CDR1 | H_FR2 | H_CDR2 | H_FR3 | H_CDR3 | H_FR4 |
| | VH3\|3-33/D5\|5-18\|RF3/JH4 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | GYSYG------------<br>-------YYFDY | WGQGTL<br>VTVSS |
| iPS:43 4535 | 21-225_74C8 | VH3\|3-33/D5\|5-18\|RF3/JH4 | ............<br>............<br>........ | .......... | ........<br>...... | ........<br>....N...<br>.... | .................<br>............... | DG...Y...........<br>.......DGL.. | ......<br>..... |
| iPS:43 4573 | 21-225_77E6 | VH3\|3-33/D5\|5-18\|RF3/JH4 | ............<br>..S.........<br>........ | .......... | ........<br>...... | ........<br>...QN...<br>.... | ..............F...<br>............... | DG...Y...........<br>.......DGL.. | ......<br>..... |
| iPS:43 4615 | 21-225_76C5 | VH3\|3-33/D5\|5-18\|RF3/JH4 | ............<br>............<br>........ | .......... | ........<br>...... | ........<br>....N...<br>.... | .................<br>............... | DG...Y...........<br>.......DGL.. | ......<br>..... |
| iPS:43 4669 | 21-225_79F4 | VH3\|3-33/D5\|5-18\|RF3/JH4 | ............<br>..S.........<br>........ | N......... | ........<br>...... | ........<br>...QN...<br>.... | ..............F...<br>............... | DG...Y...........<br>.......DGL.. | ......<br>..... |

| | | | | | | | | | |
|---|---|---|---|---|---|---|---|---|---|
| iPS:43 4737 | 21-225_74G6 | VH3\|3-33/D5\|5-18\|RF3/JH4 | .............<br>.............<br>........ | .......... | ........<br>...... | ........<br>....N...<br>.... | ..................<br>............... | DG...Y...........<br>.......DGL.. | ......<br>..... |
| iPS:43 4741 | 21-225_80C11 | VH3\|3-33/D5\|5-18\|RF3/JH4 | .............<br>.............<br>........ | .......... | ........<br>...... | ........<br>....N...<br>.... | ..............S...<br>............... | DG...Y...........<br>.......DGL.. | ......<br>..... |
| iPS:43 4867 | 21-225_79A12 | VH3\|3-33/D5\|5-18\|RF3/JH4 | .............<br>.............<br>........ | .......... | ........<br>...... | ........<br>....N...<br>.... | ..................<br>............... | DG...Y...........<br>.......DGL.. | ......<br>.A... |
| | | Germline | H_FR1 | H_CDR1 | H_FR2 | H_CDR2 | H_FR3 | H_CDR3 | H_FR4 |
| | VH4\|4-34/D3\|3-10\|RF2/JH6 | | QVQLQQW-<br>GAGLLKPSETLS<br>LTCAVYG-GSFS | G-----YYWS | WIRQPPGK<br>GLEWIG | EINH----<br>SGSTNYNP<br>SLKS | RVTISVDTSKNQFSLKL<br>SSVTAADTAVYYCAR | YYYGSGSYYN--------<br>---YYYYYGMDV | WGQGTT<br>VTVSS |
| iPS:43 4539 | 21-225_74A2 | VH4\|4-34/D3\|3-10\|RF2/JH6 | ...V........<br>............<br>.......T | D......... | ........<br>...... | ........<br>..D.....<br>.... | ..................<br>............... | EFPY....--<br>...........-<br>L....... | ......<br>..... |
| iPS:43 7248 | 21-225_97H3 | VH4\|4-34/D3\|3-10\|RF2/JH6 | ...V........<br>............<br>.......T | D......... | ........<br>...... | ........<br>..D.....<br>.... | ..................<br>............... | EFPY....--<br>...........-<br>L....... | ......<br>..... |
| iPS:43 7320 | 21-225_75A1 | VH4\|4-34/D3\|3-10\|RF2/JH6 | ...V........<br>..H.........<br>.......T | D......... | ........<br>...... | ........<br>..D.....<br>.... | ..................<br>............... | EFPY....--<br>...........-<br>L....... | ......<br>..... |
| | | Germline | H_FR1 | H_CDR1 | H_FR2 | H_CDR2 | H_FR3 | H_CDR3 | H_FR4 |
| | VH3\|3-13/D3\|3-9\|RF1/JH6 | | EVQLVES-<br>GGGLVQPGGSLR<br>LSCAASG-FTFS | S-----YDMH | WVRQATGK<br>GLEWVS | AIGT----<br>AGDTYYPG<br>SVKG | RFTISRENAKNSLYLQM<br>NSLRAGDTAVYYCAR | VLRYFDWLL*--------<br>---YYYYYGMDV | WGQGTT<br>VTVSS |
| iPS:43 4563 | 21-225_75D8 | VH3\|3-13/D3\|3-9\|RF1/JH6 | .............<br>.............<br>........ | N......... | ........<br>...... | ........<br>........<br>.... | ..................<br>............... | ..D.G.S.G-<br>...........-<br>........ | ......<br>..... |
| iPS:43 5009 | 21-225_89G4 | VH3\|3-13/D3\|3-9\|RF1/JH6 | .............<br>.............<br>........ | .......... | ........<br>...... | ........<br>........<br>.... | ..................<br>...........F... | A.D.G.S.G-<br>...........-<br>........ | ......<br>..... |
| iPS:43 5059 | 21-225_90C11 | VH3\|3-13/D3\|3-9\|RF1/JH6 | .............<br>.............<br>........ | N......... | ........<br>...... | ........<br>........<br>.... | ..................<br>............... | ..D.G.S.G-<br>...........-<br>........ | ......<br>..... |

| | | | | | | | | | |
|---|---|---|---|---|---|---|---|---|---|
| iPS:435103 | 21-225_92B2 | VH3\|3-13/D3\|3-9\|RF1/JH6 | ................................ | N......... | .............. | .................... | ..............................F... | A.D.G.S.G-...........-........ | ........... |
| iPS:437371 | 21-225_74D8 | VH3\|3-13/D3\|3-9\|RF1/JH6 | ................................ | N......... | .............. | .................... | ................................ | ..D.G.S.G-...........-........ | ........... |
| | | Germline | H_FR1 | H_CDR1 | H_FR2 | H_CDR2 | H_FR3 | H_CDR3 | H_FR4 |
| | VH1\|1-08/D1\|1-26\|RF3/JH4 | | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | YSGSY-------------------YYFDY | WGQGTLVTVSS |
| iPS:434711 | 21-225_80H3 | VH1\|1-08/D1\|1-26\|RF3/JH4 | ............................L. | N......... | .............. | .................... | ..............................GS | T..WN.............-F... | ........... |
| iPS:434901 | 21-225_85H9 | VH1\|1-08/D1\|1-26\|RF3/JH4 | ............................L. | N......... | .............. | .................... | .............................RGS | T..WN.............-F... | ........... |
| iPS:435167 | 21-225_92F12 | VH1\|1-08/D1\|1-26\|RF3/JH4 | ............................L. | N......... | .............. | .................... | .............................RGS | T..GK.............-F... | ........... |
| iPS:435215 | 21-225_94E12 | VH1\|1-08/D1\|1-26\|RF3/JH4 | ............................L. | N......... | .............. | .................... | .............................RGS | T..WK.............-F... | ........... |
| iPS:437356 | 21-225_74B1 | VH1\|1-08/D1\|1-26\|RF3/JH4 | ............................L. | N......... | .............. | .................... | ..............................GS | T..WN.............-F... | ........... |
| | | Germline | H_FR1 | H_CDR1 | H_FR2 | H_CDR2 | H_FR3 | H_CDR3 | H_FR4 |
| | VH1\|1-08/D1\|1-1\|RF1/JH6 | | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | GTTGTYY----------------YYYGMDV | WGQGTTVTVSS |
| iPS:434815 | 21-225_74A11 | VH1\|1-08/D1\|1-1\|RF1/JH6 | ................................ | .......... | .............. | .................... | .....W...K....................... | .FYD.LTGS.............G...V... | ........... |
| iPS:435253 | 21-225_96A4 | VH1\|1-08/D1\|1-1\|RF1/JH6 | ................................ | .......... | .......H...... | .................... | .....W...K....................... | .FYD.LTGS.............G...V... | ........... |
| | | Germline | H_FR1 | H_CDR1 | H_FR2 | H_CDR2 | H_FR3 | H_CDR3 | H_FR4 |

| | | | | | | | | | |
|---|---|---|---|---|---|---|---|---|---|
| | VH1\|1-08/D3\|3-22\|RF2/JH6 | | QVQLVQS-<br>GAEVKKPGASVK<br>VSCKASG-YIFT | S-----YDIN | WVRQATGQ<br>GLEWMG | WMNPN---<br>SGNTGYAQ<br>KFQG | RVTMTRNTSISTAYMEL<br>SSLRSEDTAVYYCAR | YYYDSSGYYY--------<br>---YYYYYGMDV | WGQGTT<br>VTVSS |
| iPS:43 4977 | 21-225_88A5 | VH1\|1-08/D3\|3-22\|RF2/JH6 | ............<br>............<br>........ | .......... | ........<br>...... | ........<br>........<br>.... | .....W....R......<br>................ | GF..FLTG.S........<br>..PT....D... | ......<br>..... |
| iPS:43 5259 | 21-225_96C6 | VH1\|1-08/D3\|3-22\|RF2/JH6 | ............<br>............<br>........ | .......... | ........<br>...... | ........<br>.R......<br>.... | .....W...........<br>................ | GG..VLPGN-<br>...........-<br>N...D... | ......<br>..... |
| | | Germline | H_FR1 | H_CDR1 | H_FR2 | H_CDR2 | H_FR3 | H_CDR3 | H_FR4 |
| | VH3\|3-33/D2\|2-15\|RF3/JH3 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | DIVVVVA-----------<br>-----ATDAFDI | WGQGTM<br>VTVSS |
| iPS:43 5291 | 21-225_146E1 | VH3\|3-33/D2\|2-15\|RF3/JH3 | ............<br>...........E<br>....I... | .......... | ........<br>...... | I.......<br>........<br>.... | .............F...<br>................ | .RL.GAT..........<br>.....-A..... | ......<br>..... |
| iPS:43 6360 | 21-225_210H11 | VH3\|3-33/D2\|2-15\|RF3/JH3 | ............<br>............<br>........ | ......H... | ........<br>...... | .T......<br>..D.....<br>.... | .................<br>................ | .RL.GAT..........<br>.....-...... | ......<br>..... |
| iPS:43 6370 | 21-225_211A6 | VH3\|3-33/D2\|2-15\|RF3/JH3 | ............<br>............<br>........ | .......... | ........<br>...... | ........<br>....N...<br>.... | .................<br>.........F.... | .RT.GY-<br>.................--<br>.G... | ......<br>..... |
| iPS:43 6392 | 21-225_213B3 | VH3\|3-33/D2\|2-15\|RF3/JH3 | ............<br>............<br>........ | .......... | ........<br>...... | ........<br>....N...<br>.... | .................<br>................ | .RT.GY-<br>.................--<br>.G... | ......<br>..... |
| iPS:43 6406 | 21-225_214E4 | VH3\|3-33/D2\|2-15\|RF3/JH3 | ............<br>............<br>........ | .......... | ........<br>...... | ........<br>...EN...<br>.... | .I...............<br>................ | .RT.GY-<br>.................--<br>.GC.. | ....A.<br>..... |
| iPS:43 7326 | 21-225_75C10 | VH3\|3-33/D2\|2-15\|RF3/JH3 | ............<br>............<br>........ | .......... | ........<br>...... | ........<br>..D.....<br>.... | .................<br>................ | .RL.GAT..........<br>.....-V..... | ......<br>..... |
| | | Germline | H_FR1 | H_CDR1 | H_FR2 | H_CDR2 | H_FR3 | H_CDR3 | H_FR4 |
| | VH4\|4-39/D7\|7-27\|RF1/JH4 | | QLQLQES-<br>GPGLVKPSETLS<br>LTCTVSG-GSIS | SS---SYYWG | WIRQPPGK<br>GLEWIG | SIYY----<br>SGSTYYNP<br>SLKS | RVTISVDTSKNQFSLKL<br>SSVTAADTAVYYCAR | LTGY-------------<br>---------FDY | WGQGTL<br>VTVSS |
| iPS:43 | 21- | VH4\|4-39/D7\|7- | ............<br>............ | R......... | ........<br>...... | ........<br>....S... | .................<br>................ | .DLLW............<br>........S... | ......<br>..... |

| | | | | | | | | | |
|---|---|---|---|---|---|---|---|---|---|
| iPS:43 5361 | 21-225_148E11 | VH4\|4-39/D7\|7-27\|RF1/JH4 | ............<br>............<br>....V... | R......... | ........<br>...... | ........<br>....S...<br>.... | .................<br>..........F.... | .DPQW.............<br>........S... | ....I.<br>..... |
| iPS:43 5449 | 21-225_152H9 | VH4\|4-39/D7\|7-27\|RF1/JH4 | ............<br>............<br>........ | R......... | ........<br>...... | ........<br>...AS...<br>.... | .................<br>..........F.... | .DLQW.............<br>........S..F | ......<br>..... |
| iPS:43 5499 | 21-225_156G1 | VH4\|4-39/D7\|7-27\|RF1/JH4 | ............<br>............<br>........ | R......... | ........<br>...... | ........<br>...AS...<br>.... | .................<br>..........F.... | .DLQW.............<br>........S..F | ......<br>..... |
| iPS:43 5587 | 21-225_160H3 | VH4\|4-39/D7\|7-27\|RF1/JH4 | ............<br>............<br>........ | R......... | ........<br>...... | ........<br>....S...<br>..E. | .................<br>..........F.... | .SQRW.............<br>........D... | ......<br>..... |
| iPS:40 3868 | 21-225_19D11 | VH4\|4-39/D7\|7-27\|RF1/JH4 | ............<br>............<br>........ | R......... | ........<br>..D... | ........<br>...AN...<br>.... | .................<br>.......A....... | .DRGW.............<br>........S... | ......<br>..... |
| | | Germline | H_FR1 | H_CDR1 | H_FR2 | H_CDR2 | H_FR3 | H_CDR3 | H_FR4 |
| | VH3\|3-23/D4\|4-17\|RF2/JH5 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | DYGDY------------<br>-------NWFDP | WGQGTL<br>VTVSS |
| iPS:43 5295 | 21-225_146H1 | VH3\|3-23/D4\|4-17\|RF2/JH5 | ............<br>............<br>........ | .......... | ........<br>...... | ....R...<br>..N.F...<br>.... | .................<br>............... | RVT..GG..........<br>......ND.... | ......<br>..... |
| iPS:43 5307 | 21-225_146E9 | VH3\|3-23/D4\|4-17\|RF2/JH5 | ............<br>............<br>........ | .......... | ........<br>...... | ....R...<br>..N.F...<br>.... | ..........K......<br>............... | RVT..GG..........<br>......ND.... | ......<br>..... |
| iPS:43 5347 | 21-225_148C4 | VH3\|3-23/D4\|4-17\|RF2/JH5 | ............<br>............<br>........ | .........N | ........<br>...... | ........<br>..N.F...<br>.... | .................<br>............... | RVT..GG..........<br>......ND.... | ......<br>..... |
| iPS:43 5355 | 21-225_148H9 | VH3\|3-23/D4\|4-17\|RF2/JH5 | ............<br>............<br>........ | .......... | ........<br>...... | ....R...<br>..N.F...<br>.... | .................<br>............... | RVT..GG..........<br>......ND.... | ......<br>..... |
| iPS:43 5371 | 21-225_149A3 | VH3\|3-23/D4\|4-17\|RF2/JH5 | ............<br>............<br>........ | N........T | ........<br>...... | ....R...<br>..N.F...<br>.... | .................<br>.............T. | RVT..GG..........<br>......ND.... | ......<br>..... |

| | | | | | | | | | |
|---|---|---|---|---|---|---|---|---|---|
| iPS:43 5415 | 21-225_150C11 | VH3\|3-23/D4\|4-17\|RF2/JH5 | ............<br>............<br>........ | .........N | ........<br>...... | ........<br>..N.F...<br>.... | ..............N..<br>S.............. | RVT..GG...........<br>......ND.... | ......<br>..... |
| iPS:43 5419 | 21-225_150C12 | VH3\|3-23/D4\|4-17\|RF2/JH5 | ............<br>............<br>........ | .......... | ........<br>...... | ....R...<br>..N.F...<br>.... | .................<br>............... | RVT..GG...........<br>......ND.... | ......<br>..... |
| iPS:43 5425 | 21-225_151B12 | VH3\|3-23/D4\|4-17\|RF2/JH5 | ............<br>............<br>........ | .........N | ...H....<br>...... | ........<br>.KN.F...<br>.... | .................<br>............... | RVT..GG...........<br>......ND.... | ......<br>..... |
| iPS:43 5431 | 21-225_152D2 | VH3\|3-23/D4\|4-17\|RF2/JH5 | ............<br>............<br>........ | .......... | ........<br>...... | ....R...<br>..N.F...<br>.... | ..........K......<br>............... | RVT..GG...........<br>......ND.... | ......<br>..... |
| iPS:43 5439 | 21-225_152G4 | VH3\|3-23/D4\|4-17\|RF2/JH5 | ............<br>............<br>........ | .......... | ........<br>...... | ....R...<br>..N.F...<br>.... | .................<br>............... | RVT..GG...........<br>......ND.... | ......<br>..... |
| iPS:43 5455 | 21-225_152B11 | VH3\|3-23/D4\|4-17\|RF2/JH5 | ............<br>............<br>........ | .........N | ........<br>...... | ....R...<br>..N.F...<br>.... | .................<br>............... | RVT..GG...........<br>......ND.... | ......<br>..... |
| iPS:43 5487 | 21-225_155C4 | VH3\|3-23/D4\|4-17\|RF2/JH5 | ............<br>............<br>........ | .......... | ........<br>...... | ....R...<br>..N.F...<br>.... | .................<br>............... | RVT..GG...........<br>......ND.... | ......<br>..... |
| iPS:43 5503 | 21-225_156E4 | VH3\|3-23/D4\|4-17\|RF2/JH5 | ............<br>............<br>........ | .......... | ........<br>...... | ....R...<br>..N.F...<br>.... | .................<br>............... | RVT..GG...........<br>......ND.... | ......<br>..... |
| | | Germline | H_FR1 | H_CDR1 | H_FR2 | H_CDR2 | H_FR3 | H_CDR3 | H_FR4 |
| | VH3\|3-30/D3\|3-22\|RF3/JH6 | | QVQLVES-<br>GGGVVQPGRSLR<br>LSCAASG-FTFS | S-----YGMH | WVRQAPGK<br>GLEWVA | VISYD---<br>GSNKYYAD<br>SVKG | RFTISRDNSKNTLYLQM<br>NSLRAEDTAVYYCAK | ITMIVVVIT---------<br>---YYYYYGMDV | WGQGTT<br>VTVSS |
| iPS:43 5297 | 21-225_146B3 | VH3\|3-30/D3\|3-22\|RF3/JH6 | ............<br>............<br>........ | .......... | ........<br>...... | ..W.....<br>..Y.....<br>.... | ..............D..<br>..............V. | MGIE.A.D-<br>............--<br>....... | ......<br>..... |
| | | Germline | H_FR1 | H_CDR1 | H_FR2 | H_CDR2 | H_FR3 | H_CDR3 | H_FR4 |
| | VH4\|4-30.1/D1\|1-1\|RF1/JH3 | | QVQLQES-<br>GPGLVKPSQTLS<br>LTCTVSG-GSIS | SG---GYYWS | WIRQHPGK<br>GLEWIG | YIYY----<br>SGSTYYNP<br>SLKS | RVTISVDTSKNQFSLKL<br>SSVTAADTAVYYCAR | GTTGT-----------<br>-------DAFDI | WGQGTM<br>VTVSS |

| | | | | | | | | | |
|---|---|---|---|---|---|---|---|---|---|
| iPS:43 5301 | 21-225_146G4 | VH4\|4-30.1/D1\|1-1\|RF1/JH3 | ............ .........N.. ........ | NS........ | ........ ...... | .S...... ........ .... | .I.......N....... T.............. | .KYNWN........... .......H.... | ...... ..... |
| | | Germline | H_FR1 | H_CDR1 | H_FR2 | H_CDR2 | H_FR3 | H_CDR3 | H_FR4 |
| | VH3\|3-21/D6\|6-13\|RF1/JH4 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | GYSSSW------------------YYFDY | WGQGTLVTVSS |
| iPS:43 5313 | 21-225_146G11 | VH3\|3-21/D6\|6-13\|RF1/JH4 | ............ ............ ........ | .......... | ........ ...... | ...G.... G..T.... .... | ................ .............. | .S...- ................. .-G... | ...... ..... |
| iPS:39 3808 | 21-225_1A2 | VH3\|3-21/D6\|6-13\|RF1/JH4 | ............ ............ ........ | .......T.. | ........ ...... | ...G.... G....... .... | ................ .............. | .S...- ................. .-G... | ...... ..... |
| iPS:39 3958 | 21-225_5H2 | VH3\|3-21/D6\|6-13\|RF1/JH4 | ............ ............ ........ | .......T.. | ........ ...... | ...G.... ........ .... | ......A......... .............. | .S...- ................. .-G... | ...... ..... |
| | | Germline | H_FR1 | H_CDR1 | H_FR2 | H_CDR2 | H_FR3 | H_CDR3 | H_FR4 |
| | VH4\|4-30.4/D5\|5-18\|RF3/JH6 | | QVQLQES-GPGLVKPSQTLSLTCTVSG-GSIS | SG---DYYWS | WIRQPPGKGLEWIG | YIYY----SGSTYYNPSLKS | RVTISVDTSKNQFSLKLSSVTAADTAVYYCAR | GYSYGYYY----------------YYYGMDV | WGQGTTVTVSS |
| iPS:43 5317 | 21-225_147D2 | VH4\|4-30.4/D5\|5-18\|RF3/JH6 | ..L........ ...........A .....P.. | .........N | ....R... ...... | F....... T....... .... | ..S..E...E.....N. .............. | .GA.YS-- ...............- ...... | ...... ..... |
| | | Germline | H_FR1 | H_CDR1 | H_FR2 | H_CDR2 | H_FR3 | H_CDR3 | H_FR4 |
| | VH4\|4-30.1/D5\|5-24\|RF3/JH3 | | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | RDGYNY------------------DAFDI | WGQGTMVTVSS |
| iPS:43 5319 | 21-225_147E3 | VH4\|4-30.1/D5\|5-24\|RF3/JH3 | ............ ............ .......T | NS......Y. | ........ ...... | ........ ..G..... .... | .I.......N....... .............. | GGYNWN........... .......H...F | ...... ..... |
| iPS:43 5383 | 21-225_149D7 | VH4\|4-30.1/D5\|5-24\|RF3/JH3 | ............ .........N.. ........ | NS........ | ........ ...... | .S...... ........ .... | .I.......N....... .............. | GGYNWN........... .......H.... | ...... .I... |

| | | | | | | | | | |
|---|---|---|---|---|---|---|---|---|---|
| **iPS:43 5443** | **21-225_152E7** | **VH4\|4-30.1/D5\|5-24\|RF3/JH3** | ............<br>.........N..<br>........ | NS........ | ........<br>...... | .S......<br>........<br>.... | .I.......N.....N.<br>................ | GGYNWN...........<br>.......H.... | ......<br>..... |
| **iPS:43 5465** | **21-225_153A6** | **VH4\|4-30.1/D5\|5-24\|RF3/JH3** | ............<br>.........N..<br>........ | NS........ | ........<br>...... | .S......<br>........<br>.... | .I.......N.....N.<br>................ | GGYNWN...........<br>.......H.... | ......<br>..... |
| **iPS:44 2568** | **21-225_149D8** | **VH4\|4-30.1/D5\|5-24\|RF3/JH3** | ............<br>.........N..<br>........ | NS........ | ........<br>...... | .S......<br>........<br>.... | .I.......N.......<br>................ | GGYNWN...........<br>.......H.... | ......<br>..... |
| | | **Germline** | **H_FR1** | **H_CDR1** | **H_FR2** | **H_CDR2** | **H_FR3** | **H_CDR3** | **H_FR4** |
| | **VH3\|3-48/D4\|4-11\|RF2/JH4** | | EVQLVES-<br>GGGLVQPGGSLR<br>LSCAASG-FTFS | S-----YSMN | WVRQAPGK<br>GLEWVS | YISSS---<br>SSTIYYAD<br>SVKG | RFTISRDNAKNSLYLQM<br>NSLRDEDTAVYYCAR | DYSNY------------<br>--------YFDY | WGQGTL<br>VTVSS |
| **iPS:43 5333** | **21-225_147E9** | **VH3\|3-48/D4\|4-11\|RF2/JH4** | ............<br>............<br>........ | .......... | ........<br>...... | S..GR...<br>NT......<br>.... | ................<br>.............S. | .RG--<br>..................<br>...--SC | ......<br>..... |
| **iPS:43 5637** | **21-225_163E2** | **VH3\|3-48/D4\|4-11\|RF2/JH4** | ............<br>............<br>........ | .......... | ........<br>...... | ST.G....<br>.TY.....<br>.... | ...........LV....<br>....P.......... | .RG--<br>..................<br>...--SL | ......<br>..... |
| | | **Germline** | **H_FR1** | **H_CDR1** | **H_FR2** | **H_CDR2** | **H_FR3** | **H_CDR3** | **H_FR4** |
| | **VH1\|1-02/D2\|2-15\|RF3/JH4** | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | DIVVVVA----------<br>------ATYFDY | WGQGTL<br>VTVSS |
| **iPS:43 5351** | **21-225_148B6** | **VH1\|1-02/D2\|2-15\|RF3/JH4** | ............<br>............<br>........ | .......... | ........<br>...... | ..H.....<br>N.......<br>T... | ............V....<br>................ | .P...P-<br>..................<br>AP... | ......<br>..... |
| | | **Germline** | **H_FR1** | **H_CDR1** | **H_FR2** | **H_CDR2** | **H_FR3** | **H_CDR3** | **H_FR4** |
| | **VH4\|4-30.1/D5\|5-12\|RF3/JH6** | | QVQLQES-<br>GPGLVKPSQTLS<br>LTCTVSG-GSIS | SG---GYYWS | WIRQHPGK<br>GLEWIG | YIYY----<br>SGSTYYNP<br>SLKS | RVTISVDTSKNQFSLKL<br>SSVTAADTAVYYCAR | GYSGYDYY---------<br>----YYYYGMDV | WGQGTT<br>VTVSS |
| **iPS:43 5363** | **21-225_148F12** | **VH4\|4-30.1/D5\|5-12\|RF3/JH6** | ............<br>............<br>........ | N........N | ........<br>...... | ........<br>........<br>.... | QI........D....R.<br>................ | YSTYDY--<br>..............--<br>...... | ......<br>..... |

| | | | | | | | | | |
|---|---|---|---|---|---|---|---|---|---|
| iPS:43 5377 | 21-225_149G5 | VH4\|4-30.1/D5\|5-12\|RF3/JH6 | ..................................... | N........N | .............. | ..................... | ..........D....R................ | YSTYDY--...............--...... | ........... |
| | | Germline | H_FR1 | H_CDR1 | H_FR2 | H_CDR2 | H_FR3 | H_CDR3 | H_FR4 |
| | VH3\|3-48/D6\|6-6\|RF2/JH4 | | EVQLVES-GGGLVQPGGSLRLSCAASG-FTFS | S-----YSMN | WVRQAPGKGLEWVS | YISSS---SSTIYYADSVKG | RFTISRDNAKNSLYLQMNSLRDEDTAVYYCAR | SIAAR-----------------YFDY | WGQGTLVTVSS |
| iPS:43 5409 | 21-225_150G8 | VH3\|3-48/D6\|6-6\|RF2/JH4 | ..................................... | T........T | ..........D... | ...R................ | ..S.....................L..... | .AFS-.....................P... | ........... |
| | | Germline | H_FR1 | H_CDR1 | H_FR2 | H_CDR2 | H_FR3 | H_CDR3 | H_FR4 |
| | VH3\|3-30.3/D5\|5-18\|RF2/JH5 | | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | WIQLWL--------------NWFDP | WGQGTLVTVSS |
| iPS:43 5427 | 21-225_151C9 | VH3\|3-30.3/D5\|5-18\|RF2/JH5 | ..................................... | .......G.. | .............. | ..................... | ............................... | GVL..FGE..........LEDD.... | ........... |
| | | Germline | H_FR1 | H_CDR1 | H_FR2 | H_CDR2 | H_FR3 | H_CDR3 | H_FR4 |
| | VH3\|3-33/D3\|3-22\|RF2/JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YYYDSSG---------------YYYFDY | WGQGTLVTVSS |
| iPS:43 5441 | 21-225_152F6 | VH3\|3-33/D3\|3-22\|RF2/JH4 | ..................................... | .......... | .............. | I.........Y........ | ............................... | EG..FW-................-SG.LG. | ........... |
| iPS:43 5457 | 21-225_152C11 | VH3\|3-33/D3\|3-22\|RF2/JH4 | ...............................R | N......... | .............. | I.................. | ............................... | EA..FW-................-SG.... | ....I...... |
| iPS:43 5463 | 21-225_153D2 | VH3\|3-33/D3\|3-22\|RF2/JH4 | ..................................... | .......... | .............. | I.........Y........ | ............V................... | EG..FW-................-SG.LG. | ........... |
| iPS:43 5531 | 21-225_157G8 | VH3\|3-33/D3\|3-22\|RF2/JH4 | ..................................... | N......... | .............. | I.........Y........ | ...V........................... | EG..FW-................-SGF..S | ........... |
| iPS:43 5577 | 21-225_160B1 | VH3\|3-33/D3\|3-22\|RF2/J | ..................................... | .......... | .............. | ..........Y........ | ............................... | EA..FW-................-SG.Y.. | ........... |

| | | | | | | | | | |
|---|---|---|---|---|---|---|---|---|---|
| iPS:43 5723 | 21-225_172B7 | VH3\|3-33/D3\|3-22\|RF2/JH4 | ................................. | N......... | .............. | I...........V..... | ................................ | EA..FW-.................-SG.W.. | ........... |
| iPS:43 5731 | 21-225_173A11 | VH3\|3-33/D3\|3-22\|RF2/JH4 | ................................. | .......... | .............. | ................... | ................................ | EA..FW-.................-SGF..S | ........... |
| iPS:43 5781 | 21-225_178G10 | VH3\|3-33/D3\|3-22\|RF2/JH4 | ................................. | T......... | .............. | ................... | ........F....................... | ER..FW-.................-SGH... | ........... |
| iPS:43 5899 | 21-225_188G11 | VH3\|3-33/D3\|3-22\|RF2/JH4 | ................................. | .......... | .............T | I.........Y........ | .............F.................. | ER..FW-.................-SGH... | ........... |
| iPS:43 6602 | 21-225_226E7 | VH3\|3-33/D3\|3-22\|RF2/JH4 | ................................. | T......... | .............. | I.................. | ........F........H....D......... | EN..FW-.................-SG.YG. | ........... |
| iPS:39 2930 | 21-225_25H9 | VH3\|3-33/D3\|3-22\|RF2/JH4 | ..........................P.N | N......... | .............S | I.........Y........ | ................................ | EG..FW-.................-SGF..S | ........... |
| | | Germline | H_FR1 | H_CDR1 | H_FR2 | H_CDR2 | H_FR3 | H_CDR3 | H_FR4 |
| | VH3\|3-23/D1\|1-26\|RF2/JH4 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | V*WEL--------------------LYFDY | WGQGTLVTVSS |
| iPS:43 5479 | 21-225_154E9 | VH3\|3-23/D1\|1-26\|RF2/JH4 | ..K.............................. | .......... | .............. | ....R.....N.F....... | .............F.................. | RGFRFLE..........WLGG... | ........... |
| | | Germline | H_FR1 | H_CDR1 | H_FR2 | H_CDR2 | H_FR3 | H_CDR3 | H_FR4 |
| | VH3\|3-23/D5\|5-18\|RF3/JH4 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GYSYG--------------------YYFDY | WGQGTLVTVSS |
| iPS:43 5497 | 21-225_155H9 | VH3\|3-23/D5\|5-18\|RF3/JH4 | ................................. | .........N | .............. | T...R....LG......... | ...............L................ | DHD..DY..........NI.... | ........... |
| | | Germline | H_FR1 | H_CDR1 | H_FR2 | H_CDR2 | H_FR3 | H_CDR3 | H_FR4 |
| | VH3\|3-23/D6\|6-19\|RF1/JH3 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GYSSGW------------------YDAFDI | WGQGTMVTVSS |

| | | | | | | | | | |
|---|---|---|---|---|---|---|---|---|---|
| iPS:43 5513 | 21-225_157F3 | VH3\|3-23/D6\|6-19\|RF1/JH3 | ............<br>............<br>..E..... | T......... | ........<br>...... | V.......<br>........<br>.... | .................<br>............... | RS.GWY...........<br>......E..L.. | ......<br>..... |
| iPS:39 2766 | 21-225_23H4 | VH3\|3-23/D6\|6-19\|RF1/JH3 | ............<br>............<br>........ | .........N | ........<br>...... | V.......<br>..T..F..<br>.... | .................<br>............... | RN...............<br>......H.V... | .....K<br>..... |
| iPS:39 2808 | 21-225_20F8 | VH3\|3-23/D6\|6-19\|RF1/JH3 | ............<br>............<br>.......R | .......... | .I.....R<br>....S. | V.......<br>........<br>.... | .................<br>S.............. | R.N..............<br>......H.V... | ......<br>..... |
| | | Germline | H_FR1 | H_CDR1 | H_FR2 | H_CDR2 | H_FR3 | H_CDR3 | H_FR4 |
| | VH4\|4-39/D2\|2-21\|RF3/JH4 | | QLQLQES-<br>GPGLVKPSETLS<br>LTCTVSG-GSIS | SS---SYYWG | WIRQPPGK<br>GLEWIG | SIYY----<br>SGSTYYNP<br>SLKS | RVTISVDTSKNQFSLKL<br>SSVTAADTAVYYCAR | HIVVVT-----------<br>------AIYFDY | WGQGTL<br>VTVSS |
| iPS:43 5525 | 21-225_157E7 | VH4\|4-39/D2\|2-21\|RF3/JH4 | ..H.........<br>............<br>........ | .G........ | ........<br>...... | ........<br>........<br>.... | ...............N.<br>............... | .K.AG-<br>.................<br>--P... | ......<br>..... |
| | | Germline | H_FR1 | H_CDR1 | H_FR2 | H_CDR2 | H_FR3 | H_CDR3 | H_FR4 |
| | VH3\|3-33/D3\|3-10\|RF2/JH6 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | YYYGSGSYYN-------<br>---YYYYYGMDV | WGQGTT<br>VTVSS |
| iPS:43 5543 | 21-225_158D4 | VH3\|3-33/D3\|3-10\|RF2/JH6 | ............<br>............<br>........ | D......V.. | ........<br>.....S | ........<br>........<br>.... | .................<br>............... | EP.T..W---<br>...........--<br>.D.... | ......<br>..... |
| iPS:43 5571 | 21-225_159C8 | VH3\|3-33/D3\|3-10\|RF2/JH6 | ............<br>............<br>........ | D......V.Q | ........<br>...... | ........<br>........<br>.... | .................<br>............... | EP.N..W---<br>...........--<br>.D.... | ......<br>..... |
| iPS:43 5591 | 21-225_160C4 | VH3\|3-33/D3\|3-10\|RF2/JH6 | ............<br>............<br>........ | D......V.Q | ........<br>...... | ........<br>........<br>.... | .................<br>............... | EP.N..W---<br>...........--<br>.D.... | ......<br>..... |
| iPS:43 5615 | 21-225_161G12 | VH3\|3-33/D3\|3-10\|RF2/JH6 | ............<br>............<br>........ | D......V.Q | ........<br>...... | ........<br>........<br>.... | .................<br>............... | EP.N..W---<br>...........--<br>.D.... | ......<br>..... |
| iPS:43 6604 | 21-225_226F7 | VH3\|3-33/D3\|3-10\|RF2/JH6 | ............<br>............<br>........ | .......... | ........<br>...... | I.......<br>........<br>.... | .................<br>............... | ER.N..W---<br>...........--<br>.D..L.. | ......<br>..... |

| | | | | | | | | | |
|---|---|---|---|---|---|---|---|---|---|
| iPS:451114 | 21-225_159A3 | VH3\|3-33/D3\|3-10\|RF2/JH6 | .................................. | D......V.Q | ............... | ..................... | ........I.................... | EP.N..W---...........--.D..... | ........... |
| iPS:472732 | 21-225_2B10_LC1 | VH3\|3-33/D3\|3-10\|RF2/JH6 | .................................. | .......V.. | ............... | ...................V | ...V.........S................... | ER.T..W---...........--.D..... | ........... |
| iPS:472733 | 21-225_2B10_LC2 | VH3\|3-33/D3\|3-10\|RF2/JH6 | .................................. | .......V.. | ............... | ...................V | ...V.........S................... | ER.T..W---...........--.D..... | ........... |
| iPS:392872 | 21-225_20B11 | VH3\|3-33/D3\|3-10\|RF2/JH6 | .................................. | .......V.. | ............... | ...................V | ...V.........S................... | ER.T..W---...........--.D..... | ........... |
| iPS:393966 | 21-225_7F8 | VH3\|3-33/D3\|3-10\|RF2/JH6 | .................................. | ......CV.. | ............... | ..................... | .........N....................... | ER.T..W---...........--HD..... | ........... |
| | | Germline | H_FR1 | H_CDR1 | H_FR2 | H_CDR2 | H_FR3 | H_CDR3 | H_FR4 |
| | VH3\|3-23/D1\|1-1\|RF1/JH6 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GTTGTYY-----------------YYYGMDV | WGQGTTVTVSS |
| iPS:435559 | 21-225_158H12 | VH3\|3-23/D1\|1-1\|RF1/JH6 | ....V............................. | .......V.. | ............... | ..........R.D....... | ................................. | .GW----.................----NHD | ........... |
| | | Germline | H_FR1 | H_CDR1 | H_FR2 | H_CDR2 | H_FR3 | H_CDR3 | H_FR4 |
| | VH3\|3-21/D1\|1-1\|RF1/JH6 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | GTTGTYY-----------------YYYGMDV | WGQGTTVTVSS |
| iPS:435561 | 21-225_159F1 | VH3\|3-21/D1\|1-1\|RF1/JH6 | .................................. | .......R.. | ............... | ...G....GN..D....... | ................................. | .W-----.................-----.. | ........... |
| | | Germline | H_FR1 | H_CDR1 | H_FR2 | H_CDR2 | H_FR3 | H_CDR3 | H_FR4 |
| | VH1\|1-08/D1\|1-1\|RF1/JH4 | | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | GTTGT-------------------YFDY | WGQGTLVTVSS |
| iPS:435563 | 21-225_159H2 | VH1\|1-08/D1\|1-1\|RF1/JH4 | .................................. | .......... | ............... | ..............V..... | ................................. | KK.--...................--...-G.. | ........... |

| | | | | | | | | | |
|---|---|---|---|---|---|---|---|---|---|
| iPS:39 2718 | 21-225_17B8 | VH1\|1-08/D1\|1-1\|RF1/JH4 | ............<br>............<br>........ | .......A.. | ........<br>...... | ........<br>T.......<br>.... | .................<br>...........F.T. | KAG--<br>...................<br>...-... | ......<br>..... |
| iPS:39 3064 | 21-225_33A9 | VH1\|1-08/D1\|1-1\|RF1/JH4 | ............<br>............<br>........ | .......... | ........<br>...... | ........<br>........<br>.... | .................<br>...G........... | KKA--<br>...................<br>...-N.. | ......<br>..... |
| iPS:39 3148 | 21-225_35E5 | VH1\|1-08/D1\|1-1\|RF1/JH4 | ............<br>............<br>........ | .......... | ........<br>...... | ........<br>........<br>.... | .................<br>............... | KKS--<br>...................<br>...-N.. | ......<br>..... |
| iPS:39 8530 | 21-225_32G4 | VH1\|1-08/D1\|1-1\|RF1/JH4 | ............<br>............<br>........ | .......... | ........<br>...... | ........<br>........<br>.... | .................<br>............... | KKA--<br>...................<br>...-N.. | ......<br>..... |
| | | Germline | H_FR1 | H_CDR1 | H_FR2 | H_CDR2 | H_FR3 | H_CDR3 | H_FR4 |
| | VH3\|3-30.3/D1\|1-26\|RF3/JH6 | | QVQLVES-<br>GGGVVQPGRSLR<br>LSCAASG-FTFS | S-----YAMH | WVRQAPGK<br>GLEWVA | VISYD---<br>GSNKYYAD<br>SVKG | RFTISRDNSKNTLYLQM<br>NSLRAEDTAVYYCAR | YSGSYYYY----------<br>-----YYYGMDV | WGQGTT<br>VTVSS |
| iPS:43 5565 | 21-225_159C4 | VH3\|3-30.3/D1\|1-26\|RF3/JH6 | ............<br>............<br>........ | N.....G.. | ........<br>...... | ....S...<br>.N......<br>.... | ................L<br>.......M....... | R.S.WG--<br>...............-<br>G..... | ..H...<br>..... |
| iPS:39 3892 | 21-225_6G7 | VH3\|3-30.3/D1\|1-26\|RF3/JH6 | ............<br>............<br>........ | .......G.. | ....T...<br>...... | I...V...<br>.K......<br>.... | .................<br>............... | RGN..G--<br>...............-<br>G..... | ......<br>..... |
| | | Germline | H_FR1 | H_CDR1 | H_FR2 | H_CDR2 | H_FR3 | H_CDR3 | H_FR4 |
| | VH3\|3-23/D2\|2-21\|RF3/JH4 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | HIVVVT-----------<br>------AIYFDY | WGQGTL<br>VTVSS |
| iPS:43 5579 | 21-225_160G1 | VH3\|3-23/D2\|2-21\|RF3/JH4 | ............<br>............<br>........ | .......V.. | ........<br>...... | .M......<br>..H.....<br>.... | ............V....<br>.............V. | .G----<br>...................<br>----YS | ......<br>..... |
| iPS:43 5585 | 21-225_160G3 | VH3\|3-23/D2\|2-21\|RF3/JH4 | ............<br>............<br>........ | .......V.. | ....T...<br>...... | .M......<br>..H.....<br>.... | .................<br>.........I...V. | .G----<br>...................<br>----YS | ......<br>..... |
| | | Germline | H_FR1 | H_CDR1 | H_FR2 | H_CDR2 | H_FR3 | H_CDR3 | H_FR4 |
| | VH4\|4-39/D1\|1- | | QLQLQES-<br>GPGLVKPSETLS | SS---SYYWG | WIRQPPGK<br>GLEWIG | SIYY----<br>SGSTYYNP | RVTISVDTSKNQFSLKL<br>SSVTAADTAVYYCAR | YNWND------------<br>-------NWFDP | WGQGTL<br>VTVSS |

| | | | | | | | | | |
|---|---|---|---|---|---|---|---|---|---|
| iPS:43 5599 | 21-225_160B10 | VH4\|4-39/D1\|1-1\|RF3/JH5 | ............. ............. ........ | R......... | ....Y... ...... | N....... ...A.HI. .... | .................. N............V. | HDP.W............. .......-GV.Y | ...... ..... |
| | | Germline | H_FR1 | H_CDR1 | H_FR2 | H_CDR2 | H_FR3 | H_CDR3 | H_FR4 |
| | VH3\|3-33/D1\|1-1\|RF2/JH6 | | QVQLVES- GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | VQLERYY---------- -----YYYGMDV | WGQGTT VTVSS |
| iPS:43 5601 | 21-225_160G10 | VH3\|3-33/D1\|1-1\|RF2/JH6 | ............. ............. ........ | ......F... | ........ ...... | ........ ..Y..... .... | .................. ................ | .GI.VAGD.......... .......F..E. | ...... ..... |
| iPS:43 5655 | 21-225_167E2 | VH3\|3-33/D1\|1-1\|RF2/JH6 | ............. ............. ........ | ......F... | ........ ...... | ........ .TY..... .... | .................. ................ | .GI.VAGD.......... ..........E. | ...... ..... |
| iPS:43 5657 | 21-225_167H10 | VH3\|3-33/D1\|1-1\|RF2/JH6 | ............. ......Q..... ........ | ......F... | ........ ...... | ........ ..Y..H.. .... | .................. ................ | .GI.VAGD.......... ..........E. | ...... ..... |
| | | Germline | H_FR1 | H_CDR1 | H_FR2 | H_CDR2 | H_FR3 | H_CDR3 | H_FR4 |
| | VH3\|3-53/D7\|7-27\|RF3/JH4 | | EVQLVET- GGGLIQPGGSLR LSCAASG-FTVS | S-----NYMS | WVRQAPGK GLEWVS | VIYS---- GGSTYYAD SVKG | RFTISRDNSKNTLYLQM NSLRAEDTAVYYCAR | NWGY------------- ---------FDY | WGQGTL VTVSS |
| iPS:43 5605 | 21-225_161A4 | VH3\|3-53/D7\|7-27\|RF3/JH4 | ......S..... ............. ........ | .......... | ........ ...... | ...T.... .....N.. .... | .................. ................ | ...MA............. .......GP... | ...... ..... |
| | | Germline | H_FR1 | H_CDR1 | H_FR2 | H_CDR2 | H_FR3 | H_CDR3 | H_FR4 |
| | VH3\|3-30.3/D6\|6-6\|RF1/JH6 | | QVQLVES- GGGVVQPGRSLR LSCAASG-FTFS | S-----YAMH | WVRQAPGK GLEWVA | VISYD--- GSNKYYAD SVKG | RFTISRDNSKNTLYLQM NSLRAEDTAVYYCAR | EYSSSSYY--------- -----YYYGMDV | WGQGTT VTVSS |
| iPS:43 5607 | 21-225_161G4 | VH3\|3-30.3/D6\|6-6\|RF1/JH6 | ............. ............. ........ | .......G.. | ........ ...... | ....G... .....H.. .... | .................. .............V. | RS...G-- ...............- G..... | ...... ..... |
| iPS:39 3020 | 21-225_30E2 | VH3\|3-30.3/D6\|6-6\|RF1/JH6 | ............. ............. ........ | .......G.. | ........ ...... | ....G... ....F..V .... | ..N............... .............V. | RGY..G-- ...............- G..... | ...... ..... |

| | | | | | | | | | |
|---|---|---|---|---|---|---|---|---|---|
| iPS:39 3062 | 21-225_33H3 | VH3\|3-30.3/D6\|6-6\|RF1/JH6 | ............ ............ ........ | .......G.. | ...R.... ...... | I...G... ...NF... .... | ................. ............... | RGY..G-- ...............- G..... | ...... ..... |
| iPS:39 3138 | 21-225_35E3 | VH3\|3-30.3/D6\|6-6\|RF1/JH6 | ............ ............ ........ | .......G.. | ........ ...... | ....G... ........ .... | ................. .............V. | RGY..G-- ...............- G..... | ...... ..... |
| | | Germline | H_FR1 | H_CDR1 | H_FR2 | H_CDR2 | H_FR3 | H_CDR3 | H_FR4 |
| | VH3\|3-30.3/D6\|6-13\|RF2/JH6 | | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | GIAAAGYY----------------YYYGMDV | WGQGTTVTVSS |
| iPS:43 5611 | 21-225_161F10 | VH3\|3-30.3/D6\|6-13\|RF2/JH6 | ............ ............ ........ | .......G.. | ........ ...... | I...S... .R.DF... .... | ................. ............... | R.....-- ...............- H..... | ...... ..... |
| | | Germline | H_FR1 | H_CDR1 | H_FR2 | H_CDR2 | H_FR3 | H_CDR3 | H_FR4 |
| | VH3\|3-33/D5\|5-18\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | VDTAMVYY----------------YYYGMDV | WGQGTTVTVSS |
| iPS:43 5629 | 21-225_162H6 | VH3\|3-33/D5\|5-18\|RF1/JH6 | ............ ............ .......N | N......... | ........ ...... | ........ ........ .... | ................. ............... | KGI.A.GD.......... ............ | ...... ..... |
| | | Germline | H_FR1 | H_CDR1 | H_FR2 | H_CDR2 | H_FR3 | H_CDR3 | H_FR4 |
| | VH3\|3-21/D7\|7-27\|RF1/JH6 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | LTGYYY-----------------YYGMDV | WGQGTTVTVSS |
| iPS:43 5639 | 21-225_163G6 | VH3\|3-21/D7\|7-27\|RF1/JH6 | ............ ............ ........ | .........S | ........ ...... | ...G.... .A...... .... | ................. ............... | .S.--- .................. ---.. | ...... ..... |
| | | Germline | H_FR1 | H_CDR1 | H_FR2 | H_CDR2 | H_FR3 | H_CDR3 | H_FR4 |
| | VH3\|3-21/D5\|5-24\|RF2/JH6 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | *RWLQLYY----------------YYYGMDV | WGQGTTVTVSS |
| iPS:43 5643 | 21-225_163G10 | VH3\|3-21/D5\|5-24\|RF2/JH6 | ............ ............ ........ | .......... | ........ ...... | ...G.... .T...... .... | ................. ............... | A.M----- ...............--- --.. | ...... ..... |
| | | Germline | H_FR1 | H_CDR1 | H_FR2 | H_CDR2 | H_FR3 | H_CDR3 | H_FR4 |

| | VH3\|3-33/D3\|3-9\|RF2/JH4 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YYDILTG-----------------YYNYFDY | WGQGTLVTVSS |
|---|---|---|---|---|---|---|---|---|---|
| iPS:435663 | 21-225_169B1 | VH3\|3-33/D3\|3-9\|RF2/JH4 | ............ ............ ........ | .......... | ........ ...... | ........ ........ ..R. | ................. ............... | DPLRGYN........... ......-DPVM.. | ...... ..... |
| iPS:435669 | 21-225_169F9 | VH3\|3-33/D3\|3-9\|RF2/JH4 | ............ ............ ........ | T......... | ........ .....S | I....... .T...... .... | ................L ............... | DPLRGYN........... ......-DPVM.. | ...... ..... |
| iPS:435693 | 21-225_170G4 | VH3\|3-33/D3\|3-9\|RF2/JH4 | ............ ...........T ........ | T......... | ........ .....S | I....... .T...... .... | .............F..L .........M..... | DPLRGYN........... ......-DPVM.. | ...... ..... |
| iPS:435695 | 21-225_170D5 | VH3\|3-33/D3\|3-9\|RF2/JH4 | ............ ............ ........ | .......... | ........ ...... | I....... .T...... .... | ................L ............... | DPLRGYN........... ......-DPVM.. | ...... ..... |
| iPS:435697 | 21-225_170G5 | VH3\|3-33/D3\|3-9\|RF2/JH4 | ............ ....G....... ........ | T......... | ........ .....T | I....... .T...... .... | ..........S...... ...........F... | DPLRGYN........... ......-DPVM.. | ...... ..... |
| iPS:435703 | 21-225_170D11 | VH3\|3-33/D3\|3-9\|RF2/JH4 | ............ ...........V ........ | .......... | ........ ...... | I....... .T...... .... | ................L ............... | DPLRGYN........... ......-DPVM.. | ...... ..... |
| iPS:435705 | 21-225_171C3 | VH3\|3-33/D3\|3-9\|RF2/JH4 | ............ ....G....... ........ | T......... | ........ .....T | I....... .T...... .... | ................. ............... | DPLRGYN........... ......-DPVM.. | ...... ..... |
| iPS:435709 | 21-225_171A4 | VH3\|3-33/D3\|3-9\|RF2/JH4 | ............ ....G....... ........ | T......... | ........ ...... | I....... ........ .... | ................. ............... | DPLRGYN........... ......-DPVM.. | ...... ..... |
| iPS:435721 | 21-225_172B3 | VH3\|3-33/D3\|3-9\|RF2/JH4 | ............ .....P...... ........ | T......... | ........ ...... | ........ ........ .... | ................. ............... | DPLRGYN........... ......-DPVM.. | ...... ..... |
| iPS:435725 | 21-225_172G8 | VH3\|3-33/D3\|3-9\|RF2/JH4 | ...M........ ............ ........ | T......... | ........ ....M. | I....... .T...... .... | ................L ............... | DPLRGYN........... ......-DPVM.. | ...... ..... |
| iPS:435735 | 21-225_173H12 | VH3\|3-33/D3\|3-9\|RF2/JH | ............ ............ | .......... | ........ ...... | I....... .T...... | ................L ............... | DPLRGYN........... ......-DPVM.. | ...... ..... |

| | | | | | | | | | |
|---|---|---|---|---|---|---|---|---|---|
| iPS:43 5743 | 21-225_175G1 | VH3\|3-33/D3\|3-9\|RF2/JH4 | ............<br>....G.......<br>........ | T......... | ........<br>...... | ........<br>........<br>.... | ..............V..<br>............... | DPLRGYN..........<br>.....-DPVM.. | ......<br>..... |
| iPS:43 5761 | 21-225_176B11 | VH3\|3-33/D3\|3-9\|RF2/JH4 | ............<br>............<br>........ | T......... | ........<br>.....S | I.......<br>.T......<br>.... | .............F..L<br>............... | DPLRGYN..........<br>.....-DPVL.. | ......<br>..... |
| iPS:43 5779 | 21-225_178B10 | VH3\|3-33/D3\|3-9\|RF2/JH4 | ............<br>...........V<br>......S. | T......... | ........<br>....M. | I.......<br>.T......<br>.... | ................L<br>............... | DPLRGYN..........<br>.....-DPVM.. | ......<br>..... |
| | | Germline | H_FR1 | H_CDR1 | H_FR2 | H_CDR2 | H_FR3 | H_CDR3 | H_FR4 |
| | VH3\|3-48/D2\|2-15\|RF3/JH6 | | EVQLVES-<br>GGGLVQPGGSLR<br>LSCAASG-FTFS | S-----YSMN | WVRQAPGK<br>GLEWVS | YISSS---<br>SSTIYYAD<br>SVKG | RFTISRDNAKNSLYLQM<br>NSLRDEDTAVYYCAR | DIVVVVAAT---------<br>---YYYYYGMDV | WGQGTT<br>VTVSS |
| iPS:43 5667 | 21-225_169E3 | VH3\|3-48/D2\|2-15\|RF3/JH6 | ............<br>............<br>........ | G.....H... | ........<br>.....A | ...L....<br>G...K...<br>.... | .........RD......<br>............... | RGIT..R--<br>...........--<br>NED.L.. | ......<br>..... |
| iPS:43 5673 | 21-225_169E6 | VH3\|3-48/D2\|2-15\|RF3/JH6 | ............<br>............<br>........ | G.....H... | ........<br>.....A | ...I....<br>....K...<br>.... | .........RD......<br>............... | RGIT..R--<br>...........--<br>NED.L.. | ......<br>..... |
| iPS:43 5759 | 21-225_176E6 | VH3\|3-48/D2\|2-15\|RF3/JH6 | ............<br>............<br>........ | G.....H... | ........<br>.....A | ...I....<br>G...K...<br>.... | ..I......RD......<br>............... | RGIT..R--<br>...........--<br>NED.L.. | ......<br>..... |
| | | Germline | H_FR1 | H_CDR1 | H_FR2 | H_CDR2 | H_FR3 | H_CDR3 | H_FR4 |
| | VH4\|4-59/D3\|3-9\|RF1/JH6 | | QVQLQES-<br>GPGLVKPSETLS<br>LTCTVSG-GSIS | S-----YYWS | WIRQPPGK<br>GLEWIG | YIYY----<br>SGSTNYNP<br>SLKS | RVTISVDTSKNQFSLKL<br>SSVTAADTAVYYCAR | VLRYFDWLL*--------<br>---YYYYYGMDV | WGQGTT<br>VTVSS |
| iPS:43 5675 | 21-225_169D7 | VH4\|4-59/D3\|3-9\|RF1/JH6 | ............<br>............<br>........ | .........T | .....A..<br>...... | R..T....<br>........<br>.... | ...M.............<br>...........F..K | .G..Y-----<br>...........----<br>..... | ......<br>..... |
| iPS:43 5687 | 21-225_170H1 | VH4\|4-59/D3\|3-9\|RF1/JH6 | ............<br>............<br>........ | .......... | .....A..<br>...... | R..T....<br>........<br>.... | ...M.............<br>............... | .G..Y-----<br>...........----<br>..... | ......<br>..... |
| | | Germline | H_FR1 | H_CDR1 | H_FR2 | H_CDR2 | H_FR3 | H_CDR3 | H_FR4 |
| | VH3\|3-23/D4\|4-23\|RF2/JH4 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | DYGGN------------<br>-------SYFDY | WGQGTL<br>VTVSS |

| | | | | | | | | | |
|---|---|---|---|---|---|---|---|---|---|
| iPS:43 5711 | 21-225_171G4 | VH3\|3-23/D4\|4-23\|RF2/JH4 | ..........D.<br>............<br>........ | ......C..T | ........<br>...... | ....R...<br>..T.F...<br>..R. | .............F...<br>............... | .LI.GA............<br>.......T.... | ......<br>..... |
| iPS:43 5875 | 21-225_190B9 | VH3\|3-23/D4\|4-23\|RF2/JH4 | ............<br>............<br>........ | T......... | ........<br>...... | ...R....<br>..N.H...<br>.... | .................<br>...........S... | .GF.GS............<br>............ | ......<br>..... |
| iPS:43 5909 | 21-225_190H3 | VH3\|3-23/D4\|4-23\|RF2/JH4 | ............<br>............<br>........ | .......... | ........<br>...... | ....R...<br>..N.....<br>.... | .................<br>............... | .GF.GS............<br>............ | ......<br>..... |
| iPS:43 6013 | 21-225_193F2 | VH3\|3-23/D4\|4-23\|RF2/JH4 | ............<br>............<br>........ | T.....F... | ........<br>...... | ...R....<br>..N.H...<br>.... | .................<br>...........S... | .GF.GS............<br>............ | ......<br>..... |
| iPS:43 6100 | 21-225_195G12 | VH3\|3-23/D4\|4-23\|RF2/JH4 | ............<br>............<br>........ | T......... | ........<br>...... | ...R....<br>..N.H...<br>.... | .................<br>...........S... | .GF.GS............<br>............ | ......<br>..... |
| | | Germline | H_FR1 | H_CDR1 | H_FR2 | H_CDR2 | H_FR3 | H_CDR3 | H_FR4 |
| | VH3\|3-30.3/D1\|1-7\|RF2/JH6 | | QVQLVES-<br>GGGVVQPGRSLR<br>LSCAASG-FTFS | S-----YAMH | WVRQAPGK<br>GLEWVA | VISYD---<br>GSNKYYAD<br>SVKG | RFTISRDNSKNTLYLQM<br>NSLRAEDTAVYYCAR | V*LELYY-----------<br>-----YYYGMDV | WGQGTT<br>VTVSS |
| iPS:43 5713 | 21-225_171D7 | VH3\|3-30.3/D1\|1-7\|RF2/JH6 | ........R...<br>............<br>........ | .......G.. | ........<br>...... | ........<br>.N.RH...<br>..Q. | .............S...<br>...G........... | DRHR.D-<br>.................-<br>..AL.. | ......<br>..... |
| | | Germline | H_FR1 | H_CDR1 | H_FR2 | H_CDR2 | H_FR3 | H_CDR3 | H_FR4 |
| | VH3\|3-23/D5\|5-24\|RF3/JH3 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | RDGYNY-----------<br>-------DAFDI | WGQGTM<br>VTVSS |
| iPS:43 5729 | 21-225_173E7 | VH3\|3-23/D5\|5-24\|RF3/JH3 | ...........S<br>............<br>........ | .......... | ........<br>...... | F.......<br>..N.F...<br>.... | .................<br>...Q.........T. | ..T..G............<br>......W..... | ......<br>..... |
| iPS:43 5753 | 21-225_175G10 | VH3\|3-23/D5\|5-24\|RF3/JH3 | ............<br>............<br>........ | .......... | ....T...<br>...... | I.......<br>..N.....<br>.... | .................<br>............... | ..TW.G............<br>......W..... | ......<br>....L |
| iPS:39 3024 | 21-225_31H9 | VH3\|3-23/D5\|5-24\|RF3/JH3 | ............<br>............<br>........ | ......C..N | ........<br>...... | ........<br>...SF...<br>.... | .................<br>............... | .TP.D-<br>..................<br>.-V... | ......<br>..... |

| | | | | | | | | | |
|---|---|---|---|---|---|---|---|---|---|
| iPS:39 8474 | 21-225_17B10 | VH3\|3-23/D5\|5-24\|RF3/JH3 | .............<br>.............<br>.......R | ........... | ........<br>...... | V.......<br>..N..F..<br>.... | ..................<br>D.............. | .GIPEA............<br>............. | ......<br>..... |
| | | Germline | H_FR1 | H_CDR1 | H_FR2 | H_CDR2 | H_FR3 | H_CDR3 | H_FR4 |
| | VH1\|1-02/D1\|1-1\|RF1/JH3 | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | GTTGT------------<br>-------DAFDI | WGQGTM<br>VTVSS |
| iPS:43 5745 | 21-225_175G3 | VH1\|1-02/D1\|1-1\|RF1/JH3 | ...V........<br>............<br>........ | .......F.. | ........<br>...... | ..K.K...<br>.....C..<br>R... | ..........T......<br>.............V. | .G.TVTT..........<br>......WGV..Y | ......<br>..... |
| iPS:43 7270 | 21-225_178H4 | VH1\|1-02/D1\|1-1\|RF1/JH3 | ............<br>............<br>........ | .......F.. | ........<br>...... | ..K.K...<br>.....C..<br>.... | .................<br>.............V. | .G.TVTT..........<br>......WGV..Y | ......<br>..... |
| | | Germline | H_FR1 | H_CDR1 | H_FR2 | H_CDR2 | H_FR3 | H_CDR3 | H_FR4 |
| | VH3\|3-23/D3\|3-22\|RF2/JH4 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | YYYDSSG----------<br>-----YYYYFDY | WGQGTL<br>VTVSS |
| iPS:43 5769 | 21-225_177B6 | VH3\|3-23/D3\|3-22\|RF2/JH4 | ............<br>......R....E<br>........ | ........... | ........<br>...... | V.......<br>.SN...V.<br>.... | .............N...<br>.......S.....T. | G................<br>.........P..F | ......<br>..... |
| | | Germline | H_FR1 | H_CDR1 | H_FR2 | H_CDR2 | H_FR3 | H_CDR3 | H_FR4 |
| | VH3\|3-33/D3\|3-22\|RF2/JH1 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | YYYDSSGY---------<br>----YYAEYFQH | WGQGTL<br>VTVSS |
| iPS:43 5771 | 21-225_177B11 | VH3\|3-33/D3\|3-22\|RF2/JH1 | ............<br>.........T..<br>........ | ........... | ........<br>...... | I.......<br>..Y...T.<br>.... | .................<br>................ | ET..FW--<br>..............--<br>SG..VF | ......<br>..... |
| | | Germline | H_FR1 | H_CDR1 | H_FR2 | H_CDR2 | H_FR3 | H_CDR3 | H_FR4 |
| | VH3\|3-23/D5\|5-24\|RF3/JH4 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | RDGYN------------<br>-------YYFDY | WGQGTL<br>VTVSS |
| iPS:43 5775 | 21-225_178A5 | VH3\|3-23/D5\|5-24\|RF3/JH4 | ..H.........<br>..T.........<br>........ | ..........T | ........<br>...... | V.......<br>..N.F...<br>.... | .................<br>..............R | ...D-<br>.................<br>..-.... | ......<br>..... |
| iPS:43 7214 | 21-225_48B12 | VH3\|3-23/D5\|5-24\|RF3/JH4 | ............<br>............<br>........ | ..........N | ........<br>...... | ....R...<br>..N.F...<br>.... | .................<br>................ | .ET..WN..........<br>......YEG... | ......<br>..... |

| | | | | | | | | | |
|---|---|---|---|---|---|---|---|---|---|
| iPS:39 3028 | 21-225_25D7 | VH3\|3-23/D5\|5-24\|RF3/JH4 | ............ ............ ........ | .......... | ....T..Q ...... | ....R... ..T.F... .... | ................. ............... | DGYGGN........... .......SF... | ...... ..... |
| | | Germline | **H_FR1** | **H_CDR1** | **H_FR2** | **H_CDR2** | **H_FR3** | **H_CDR3** | **H_FR4** |
| | VH3\|3-33/D4\|4-11\|RF3/JH6 | | QVQLVES- GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | TTVTYYY---------- ------YYGMDV | WGQGTT VTVSS |
| iPS:43 5789 | 21-225_180C4 | VH3\|3-33/D4\|4-11\|RF3/JH6 | ............ ............ ........ | A......... | ........ .....T | I....... ..Y..... .... | ..A.............. ............... | .G.DPWD.......... .....Y.N.... | ...... ..... |
| | | Germline | **H_FR1** | **H_CDR1** | **H_FR2** | **H_CDR2** | **H_FR3** | **H_CDR3** | **H_FR4** |
| | VH3\|3-23/D2\|2-8\|RF1/JH3 | | EVQLLES- GGGLVQPGGSLR LSCAASG-FTFS | S-----YAMS | WVRQAPGK GLEWVS | AISGS--- GGSTYYAD SVKG | RFTISRDNSKNTLYLQM NSLRAEDTAVYYCAK | RILY*WCM--------- -----LYDAFDI | WGQGTM VTVSS |
| iPS:43 5799 | 21-225_181G3 | VH3\|3-23/D2\|2-8\|RF1/JH3 | ............ ............ ........ | .......... | ........ ...... | V....... ..N.F.G. .... | ................. .........I..... | .ET.D.G- ..............- S..... | ...... ..... |
| | | Germline | **H_FR1** | **H_CDR1** | **H_FR2** | **H_CDR2** | **H_FR3** | **H_CDR3** | **H_FR4** |
| | VH3\|3-30.3/D5\|5-18\|RF2/JH6 | | QVQLVES- GGGVVQPGRSLR LSCAASG-FTFS | S-----YAMH | WVRQAPGK GLEWVA | VISYD--- GSNKYYAD SVKG | RFTISRDNSKNTLYLQM NSLRAEDTAVYYCAR | WIQLWLYY--------- -----YYYGMDV | WGQGTT VTVSS |
| iPS:43 5811 | 21-225_183H6 | VH3\|3-30.3/D5\|5-18\|RF2/JH6 | ............ ............ ........ | .......G.. | ........ ...... | I...A... ..T.F... .... | ................. .............V. | RPPQ..V- ..............EG. .... | ...... ..... |
| iPS:43 6754 | 21-225_155G3 | VH3\|3-30.3/D5\|5-18\|RF2/JH6 | ............ ...........T ........ | .......G.. | ........ ...... | ........ ........ .... | ................. ............... | DTER..P- ...............S. .... | ...... ..... |
| iPS:44 8908 | 21-225_50G9 | VH3\|3-30.3/D5\|5-18\|RF2/JH6 | ............ ............ .....I.. | .......G.. | ........ ...... | ...Q.... .IIR.... .... | ................. ............... | DVKQ..V- ..............RT. .... | ...... ..... |
| | | Germline | **H_FR1** | **H_CDR1** | **H_FR2** | **H_CDR2** | **H_FR3** | **H_CDR3** | **H_FR4** |
| | VH3\|3-30.3/D6\|6-19\|RF1/JH5 | | QVQLVES- GGGVVQPGRSLR LSCAASG-FTFS | S-----YAMH | WVRQAPGK GLEWVA | VISYD--- GSNKYYAD SVKG | RFTISRDNSKNTLYLQM NSLRAEDTAVYYCAR | GYSSGW----------- ------YNWFDP | WGQGTL VTVSS |

| | | | | | | | | | |
|---|---|---|---|---|---|---|---|---|---|
| iPS:43 5813 | 21-225_183A12 | VH3\|3-30.3/D6\|6-19\|RF1/JH5 | ............<br>............<br>........ | ........G.. | ........<br>...... | ...SA...<br>........<br>.... | .................<br>............... | R.................<br>.......-D.... | ......<br>..... |
| | | Germline | H_FR1 | H_CDR1 | H_FR2 | H_CDR2 | H_FR3 | H_CDR3 | H_FR4 |
| | VH3\|3-21/D3\|3-10\|RF3/JH4 | | EVQLVES-<br>GGGLVKPGGSLR<br>LSCAASG-FTFS | S-----YSMN | WVRQAPGK<br>GLEWVS | SISSS---<br>SSYIYYAD<br>SVKG | RFTISRDNAKNSLYLQM<br>NSLRAEDTAVYYCAR | ITMVRGV-----------<br>------IIYFDY | WGQGTL<br>VTVSS |
| iPS:43 5815 | 21-225_190G10 | VH3\|3-21/D3\|3-10\|RF3/JH4 | ............<br>............<br>.......R | D......... | ........<br>...... | ....G...<br>.G..H...<br>.... | .................<br>............... | A..A---<br>.................-<br>--L.. | ......<br>..... |
| iPS:43 5865 | 21-225_191A5 | VH3\|3-21/D3\|3-10\|RF3/JH4 | .I.V........<br>............<br>.......R | D......... | ........<br>...... | ....G...<br>.G......<br>.... | .................<br>............... | A..A---<br>.................-<br>--L.. | ....A.<br>..... |
| iPS:43 6047 | 21-225_193B10 | VH3\|3-21/D3\|3-10\|RF3/JH4 | ............<br>............<br>.......R | D......... | ........<br>...... | ....A...<br>GG......<br>.L.. | .................<br>............... | A..A---<br>.................-<br>--L.. | ......<br>..... |
| iPS:43 6122 | 21-225_196G10 | VH3\|3-21/D3\|3-10\|RF3/JH4 | ............<br>............<br>.......R | D......... | ........<br>...... | ....G...<br>.G..H...<br>.... | .................<br>............... | A..A---<br>.................-<br>--L.. | ......<br>..... |
| | | Germline | H_FR1 | H_CDR1 | H_FR2 | H_CDR2 | H_FR3 | H_CDR3 | H_FR4 |
| | VH4\|4-59/D4\|4-17\|RF2/JH6 | | QVQLQES-<br>GPGLVKPSETLS<br>LTCTVSG-GSIS | S-----YYWS | WIRQPPGK<br>GLEWIG | YIYY----<br>SGSTNYNP<br>SLKS | RVTISVDTSKNQFSLKL<br>SSVTAADTAVYYCAR | DYGDYYY-----------<br>-----YYYGMDV | WGQGTT<br>VTVSS |
| iPS:43 5817 | 21-225_190B11 | VH4\|4-59/D4\|4-17\|RF2/JH6 | ............<br>............<br>.......N | N......... | .....A..<br>...... | R..T....<br>........<br>.... | ...M.............<br>..........H.... | .R.Y.G-<br>................-<br>...... | ......<br>..... |
| iPS:43 5917 | 21-225_190D5 | VH4\|4-59/D4\|4-17\|RF2/JH6 | ............<br>............<br>.......N | N......... | .....A..<br>...... | R..A....<br>........<br>.... | ...M.I...........<br>............... | .R.Y.G-<br>................-<br>...... | ......<br>..I.. |
| iPS:43 6056 | 21-225_194C3 | VH4\|4-59/D4\|4-17\|RF2/JH6 | ............<br>............<br>.......N | N......... | .....A..<br>...... | R..A....<br>........<br>.... | ...M.I...........<br>..........H.... | .R.Y.G-<br>................-<br>...... | ......<br>..I.. |
| iPS:43 6220 | 21-225_200F8 | VH4\|4-59/D4\|4-17\|RF2/JH6 | ............<br>............<br>.......N | N......... | .....A..<br>...... | R..T....<br>........<br>.... | ...M.............<br>............... | .R.Y.G-<br>................-<br>...... | ......<br>..... |

| | | | | | | | | |
|---|---|---|---|---|---|---|---|---|
| | VH3\|3-30/D4\|4-17\|RF2/JH6 | | QVQLVES-GGGVVQPGRSLRLSCAASG-FTFS | S-----YGMH | WVRQAPGKGLEWVA | VISYD---GSNKYYADSVKG | RFTISRDNSKNTLYLQMNSLRAEDTAVYYCAK | DYGDYYY-----------------YYYGMDV | WGQGTTVTVSS |
| iPS:435821 | 21-225_190E11 | VH3\|3-30/D4\|4-17\|RF2/JH6 | ............<br>...........T<br>........ | N......... | ........<br>...... | I.WF....<br>........<br>.... | .........N.......<br>............... | AQ.V..-<br>................--<br>.V... | ......<br>..... |
| | | Germline | H_FR1 | H_CDR1 | H_FR2 | H_CDR2 | H_FR3 | H_CDR3 | H_FR4 |
| | VH3\|3-23/D5\|5-12\|RF3/JH5 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GYSGYD-----------------YNWFDP | WGQGTLVTVSS |
| iPS:435823 | 21-225_190F11 | VH3\|3-23/D5\|5-12\|RF3/JH5 | .....D......<br>G...........<br>.......D | .........N | ........<br>...... | T...T...<br>.RR.....<br>.... | .................<br>............... | EEDY..S..........<br>.....SGPG... | ......<br>..... |
| iPS:435867 | 21-225_191E5 | VH3\|3-23/D5\|5-12\|RF3/JH5 | .....D......<br>G...........<br>.......D | .........N | ........<br>...... | T...T...<br>.RR.....<br>.... | .................<br>............... | EEDY..S..........<br>.....SGPG... | ......<br>..... |
| iPS:435929 | 21-225_190D9 | VH3\|3-23/D5\|5-12\|RF3/JH5 | .....D......<br>G...........<br>.......D | .......... | ........<br>...... | T...T...<br>.RR.....<br>.... | .................<br>............... | EEDY..S..........<br>.....SGPG... | ......<br>..... |
| iPS:435935 | 21-225_190H8 | VH3\|3-23/D5\|5-12\|RF3/JH5 | G....D......<br>G...........<br>.......D | .......... | ........<br>...... | T...T...<br>.RR.....<br>.... | .................<br>............... | EEDY..S..........<br>.....SGPG... | ......<br>..... |
| | | Germline | H_FR1 | H_CDR1 | H_FR2 | H_CDR2 | H_FR3 | H_CDR3 | H_FR4 |
| | VH4\|4-39/D3\|3-9\|RF1/JH4 | | QLQLQES-GPGLVKPSETLSLTCTVSG-GSIS | SS---SYYWG | WIRQPPGKGLEWIG | SIYY----SGSTYYNPSLKS | RVTISVDTSKNQFSLKLSSVTAADTAVYYCAR | VLRYFDW-----------------LL*YFDY | WGQGTLVTVSS |
| iPS:435827 | 21-225_190H11 | VH4\|4-39/D3\|3-9\|RF1/JH4 | ............<br>............<br>.......R | .-...-.H.S | .....A..<br>...... | L..T....<br>.R..I...<br>.... | ...L.............<br>............... | LRYNWN-<br>................-<br>FP.... | ......<br>..... |
| iPS:435853 | 21-225_191E3 | VH4\|4-39/D3\|3-9\|RF1/JH4 | ............<br>............<br>.......R | .-...-.H.S | .....A..<br>...... | L..T....<br>.R..N...<br>.... | ...M.............<br>N.............. | LRYNWN-<br>................-<br>FP.... | ......<br>..... |
| iPS:435871 | 21-225_191E6 | VH4\|4-39/D3\|3-9\|RF1/JH4 | ............<br>............<br>.......R | .-...-.H.S | .....A..<br>...... | L..T....<br>.R..N...<br>.... | ...M.............<br>............... | LRYNWN-<br>................-<br>FP...F | ......<br>..... |
| iPS:43 | 21- | VH4\|4-39/D3\|3- | ............<br>............ | .-...-.H.S | .....A..<br>...... | H..T....<br>.R..N... | .................<br>.....T......... | LRYNWN-<br>................- | ......<br>..... |

| | | | | | | | | | |
|---|---|---|---|---|---|---|---|---|---|
| iPS:43 5999 | 21-225_192F9 | VH4\|4-39/D3\|3-9\|RF1/JH4 | ............ ............ .......R | .-...-.H.S | .....A.. ...... | L..T.... .R..N... .... | ...M............ N............. | LRYNWN- ................- FP.... | ...... ..... |
| iPS:43 6060 | 21-225_194F4 | VH4\|4-39/D3\|3-9\|RF1/JH4 | ............ ............ ........ | .-...-.H.S | .....A.. ...... | L..T.... .R..N... .... | ...M...R..S...... .............. | LRYNWN- ................- FP.... | ...... ..... |
| iPS:43 6193 | 21-225_198A10 | VH4\|4-39/D3\|3-9\|RF1/JH4 | ............ ............ .......R | .-...-.H.S | .....A.. ...... | H..T.... .R..N... .... | ................. .....T......... | LRYNWN- ................- FP.... | ...... ..... |
| | | Germline | H_FR1 | H_CDR1 | H_FR2 | H_CDR2 | H_FR3 | H_CDR3 | H_FR4 |
| | VH4\|4-30.1/D5\|5-24\|RF3/JH2 | | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | RDGYNY-----------------YWYFDL | WGRGTLVTVSS |
| iPS:43 5829 | 21-225_190B12 | VH4\|4-30.1/D5\|5-24\|RF3/JH2 | ............ ............ ........ | .........N | ........ ...... | ........ ........ .... | .............F... N............. | SGYNWD........... ......-AGV.P | ...... ..... |
| | | Germline | H_FR1 | H_CDR1 | H_FR2 | H_CDR2 | H_FR3 | H_CDR3 | H_FR4 |
| | VH3\|3-33/D4\|4-11\|RF2/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DYSNYYY-----------YYYGMDV | WGQGTTVTVSS |
| iPS:43 5835 | 21-225_190F12 | VH3\|3-33/D4\|4-11\|RF2/JH6 | ............ ............ .......R | N......... | ........ ...... | ...F.... ...D.... .... | .............S... .............. | .R.VG.- ................-- D.L.. | ...... ..... |
| iPS:43 5861 | 21-225_190A5 | VH3\|3-33/D4\|4-11\|RF2/JH6 | ............ G........... ........ | .......... | ........ ...... | ........ ....N... .... | ................. .............. | .F.VG.- ................-- D.... | ...... ..... |
| iPS:43 5937 | 21-225_190H9 | VH3\|3-33/D4\|4-11\|RF2/JH6 | ............ ............ .......R | N......... | ........ ...... | ...F.... ...D.... .... | .............S... .............. | .R.VG.- ................-- D.L.. | ...... ..... |
| iPS:43 5977 | 21-225_192E4 | VH3\|3-33/D4\|4-11\|RF2/JH6 | ............ ............ ........ | .......... | ........ ...... | ........ ....N.V. ..R. | ................. .............. | .R.VG.- ................-- D.... | ...... ..... |
| iPS:43 6001 | 21-225_192C10 | VH3\|3-33/D4\|4-11\|RF2/JH6 | ............ ............ S......R | N......... | ........ ..K... | ...F.... ...D.... .... | .............S... .............. | .R.VG.- ................-- D.L.. | ...... ..... |

| | | | | | | | | | |
|---|---|---|---|---|---|---|---|---|---|
| iPS:436066 | 21-225_194B7 | VH3\|3-33/D4\|4-11\|RF2/JH6 | ............<br>............<br>........ | .......... | ........<br>...... | ........<br>....N...<br>.... | .................<br>............... | .R.KG.-<br>................--<br>D.... | ......<br>..... |
| iPS:436078 | 21-225_194H12 | VH3\|3-33/D4\|4-11\|RF2/JH6 | ............<br>............<br>.......R | N......... | ........<br>...... | ...F....<br>...D....<br>.... | .............S...<br>............... | .R.VG.-<br>................--<br>D.L.. | ......<br>..... |
| iPS:436140 | 21-225_197G3 | VH3\|3-33/D4\|4-11\|RF2/JH6 | ............<br>...........V<br>........ | ......H... | ........<br>...... | ........<br>....N...<br>.... | .................<br>............... | .P.VG.-<br>................--<br>D.... | ......<br>..... |
| iPS:436167 | 21-225_197E11 | VH3\|3-33/D4\|4-11\|RF2/JH6 | ............<br>............<br>.......R | N......... | ........<br>...... | ...F....<br>...D....<br>.... | .............S...<br>............... | .R.VG.-<br>................--<br>D.L.. | ......<br>..... |
| iPS:436292 | 21-225_205H3 | VH3\|3-33/D4\|4-11\|RF2/JH6 | ............<br>............<br>........ | .......... | ........<br>...... | ........<br>........<br>.... | .................<br>............... | .R.VG.-<br>................--<br>D.T.. | ......<br>..... |
| iPS:436802 | 21-225_171E12 | VH3\|3-33/D4\|4-11\|RF2/JH6 | ............<br>............<br>........ | .......... | ........<br>...... | ...N....<br>.G...NG.<br>.... | .................<br>............... | .RTY.SGSGSP.......<br>..PYY....... | ......<br>..... |
| iPS:436816 | 21-225_179H5 | VH3\|3-33/D4\|4-11\|RF2/JH6 | ............<br>............<br>........ | .......... | ........<br>...... | ........<br>...E....<br>.... | .................<br>............... | .IR...-<br>................--<br>..L.. | ......<br>..... |
| iPS:436960 | 21-225_198D2 | VH3\|3-33/D4\|4-11\|RF2/JH6 | ............<br>............<br>.......R | .......... | ........<br>...... | ..I.....<br>..Y.....<br>.... | .................<br>............... | T..G---<br>................--<br>-.... | ......<br>..... |
| iPS:436974 | 21-225_190H7 | VH3\|3-33/D4\|4-11\|RF2/JH6 | ............<br>............<br>.....N.R | .......... | ........<br>...... | ..I.....<br>..Y.....<br>.... | .................<br>............... | T..G---<br>................--<br>-.... | ......<br>..... |
| iPS:436982 | 21-225_190D10 | VH3\|3-33/D4\|4-11\|RF2/JH6 | ............<br>............<br>.......R | .......... | ........<br>...... | ..I.....<br>..Y.....<br>.... | .................<br>............... | T..G---<br>................--<br>-.... | ......<br>..... |
| iPS:437274 | 21-225_196D4 | VH3\|3-33/D4\|4-11\|RF2/JH6 | ...V........<br>............<br>........ | .......... | ........<br>...... | ........<br>...RN...<br>.... | .................<br>....V.......... | .R.KG.-<br>................--<br>D.... | ......<br>..... |
| iPS:39 | 21-225_20F6 | VH3\|3-33/D4\|4- | ............<br>...........V | .......... | ........<br>....G. | ...H....<br>........ | ........A........<br>............... | .L.MG--<br>................-- | ......<br>..... |

| | | | | | | | | | |
|---|---|---|---|---|---|---|---|---|---|
| iPS:39 2738 | 21-225_18G4 | VH3\|3-33/D4\|4-11\|RF2/JH6 | ............<br>............<br>........ | ........... | ........<br>...... | I.......<br>........<br>.... | .................<br>............... | .L.MG--<br>...............--<br>-.... | ......<br>..... |
| iPS:39 2798 | 21-225_22C7 | VH3\|3-33/D4\|4-11\|RF2/JH6 | ............<br>............<br>........ | ........... | ........<br>....G. | ...H....<br>........<br>.... | ........A........<br>............... | .L.MG--<br>...............--<br>-.... | ......<br>..... |
| iPS:39 2956 | 21-225_27A11 | VH3\|3-33/D4\|4-11\|RF2/JH6 | ............<br>............<br>........ | ........... | ........<br>...... | ........<br>........<br>.... | .................<br>...........H... | .S.P.--<br>...............--<br>-.... | ......<br>..... |
| iPS:39 2994 | 21-225_26G11 | VH3\|3-33/D4\|4-11\|RF2/JH6 | ............<br>............<br>........ | ........... | ........<br>...... | ........<br>........<br>.... | .................<br>............... | R...SW-<br>...............-<br>SG.... | ......<br>..... |
| iPS:39 3014 | 21-225_26D12 | VH3\|3-33/D4\|4-11\|RF2/JH6 | ............<br>............<br>........ | ........... | ........<br>...... | ........<br>...E....<br>.... | .................<br>.....A......... | .S.P.--<br>...............--<br>-.... | ......<br>..... |
| iPS:39 3152 | 21-225_25B3 | VH3\|3-33/D4\|4-11\|RF2/JH6 | ............<br>............<br>........ | ........... | ........<br>...... | ........<br>........<br>.... | .................<br>............... | .S.P.--<br>...............--<br>-.... | ......<br>..... |
| iPS:39 3840 | 21-225_3F8 | VH3\|3-33/D4\|4-11\|RF2/JH6 | ............<br>............<br>........ | ........... | ........<br>....G. | ...H....<br>........<br>.... | ........A........<br>............... | .L.MG--<br>...............--<br>-.... | ......<br>..... |
| iPS:39 3930 | 21-225_7E11 | VH3\|3-33/D4\|4-11\|RF2/JH6 | ............<br>............<br>........ | ......F... | ........<br>...... | I..H....<br>........<br>.... | .........N.......<br>S.............. | .L.MG--<br>...............--<br>-.... | ......<br>..... |
| iPS:39 3964 | 21-225_6G1 | VH3\|3-33/D4\|4-11\|RF2/JH6 | ............<br>............<br>.....I.. | ........... | ........<br>...... | I.......<br>........<br>.... | .................<br>.........M..... | .L.MG--<br>...............--<br>-.... | ......<br>..... |
| iPS:39 4012 | 21-225_15A3 | VH3\|3-33/D4\|4-11\|RF2/JH6 | ............<br>............<br>........ | ........I. | ........<br>...... | ...H....<br>........<br>.... | .................<br>............... | .L.MG--<br>...............--<br>-.... | ......<br>..... |
| iPS:39 4016 | 21-225_13D4 | VH3\|3-33/D4\|4-11\|RF2/JH6 | ............<br>............<br>........ | ........... | ........<br>...... | ...H....<br>........<br>.... | .................<br>............... | .L.MG--<br>...............--<br>-.... | ......<br>..... |
| iPS:39 | 21-225_16F6 | VH3\|3-33/D4\|4- | ............<br>............ | ........... | ........<br>...... | ...H....<br>......V. | ..............N...<br>............... | .L.MG--<br>...............-- | ......<br>..... |

| | | Germline | H_FR1 | H_CDR1 | H_FR2 | H_CDR2 | H_FR3 | H_CDR3 | H_FR4 |
|---|---|---|---|---|---|---|---|---|---|
| | VH4\|4-59/D3\|3-9\|RF1/JH4 | | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAR | VLRYFDW-----------------LL*YFDY | WGQGTLVTVSS |
| iPS:435839 | 21-225_191B1 | VH4\|4-59/D3\|3-9\|RF1/JH4 | ............ ............ ........ | .......H.. | .....A.. ...... | H..T.... ....K... .... | ...M............ ............... | LRYNWN- ................- FPF... | ...... ..... |
| iPS:436158 | 21-225_197G8 | VH4\|4-59/D3\|3-9\|RF1/JH4 | ..H......... ............ ........ | A......S.. | .....A.. ...... | RLSP.... G....F.. .... | ...M..........R. ............... | LRYNWN- ................- FP.... | ....A. ..... |
| | | Germline | H_FR1 | H_CDR1 | H_FR2 | H_CDR2 | H_FR3 | H_CDR3 | H_FR4 |
| | VH4\|4-30.1/D3\|3-22\|RF2/JH6 | | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | YYYDSSGYYY-----------YYYYYGMDV | WGQGTTVTVSS |
| iPS:435843 | 21-225_191F1 | VH4\|4-30.1/D3\|3-22\|RF2/JH6 | ............ ............ .......N | .........N | ........ ...... | ..F..... ........ .... | ................. ............... | GD..G..S.- ...........- H....... | ...... ..... |
| iPS:435847 | 21-225_191A3 | VH4\|4-30.1/D3\|3-22\|RF2/JH6 | ............ ............ ........ | .....D...N | ........ ...... | ..F..... ........ .... | ................. ............... | GD..G..S.- ...........- H....... | ...... ..... |
| iPS:435851 | 21-225_191D3 | VH4\|4-30.1/D3\|3-22\|RF2/JH6 | ....K....... ............ .......N | .....D...N | ........ ..D... | ..F..... ........ .... | ................. ............... | GD..G..S.- ...........- H....... | ...... ..... |
| iPS:435905 | 21-225_190A3 | VH4\|4-30.1/D3\|3-22\|RF2/JH6 | ............ ............ .......N | .........N | ........ ...... | F.F..... ........ .... | ................. N............. | GD..G..S.- ...........- H....... | ...... ..... |
| iPS:435911 | 21-225_190B4 | VH4\|4-30.1/D3\|3-22\|RF2/JH6 | ............ ............ ........ | .....D...N | ........ ...... | ..F..... ........ .... | ................. ............... | GD..G..S.- ...........- H....... | ...... ..... |
| iPS:435913 | 21-225_190A7 | VH4\|4-30.1/D3\|3-22\|RF2/JH6 | ............ .N.......... ........ | .....V.... | ........ ...... | N....... .....N.. .... | .II.............. ............... | GD..G..S.- ...........- H....L.. | ...... ..... |

| | | | | | | | | | |
|---|---|---|---|---|---|---|---|---|---|
| iPS:43 5939 | 21-225_191H7 | VH4\|4-30.1/D3\|3-22\|RF2/JH6 | ............ ............ .......N | .....D...N | ........ ...... | ..F..... ........ .... | ................. ............... | GD..G..S.– ...........– H....... | ...... ..... |
| iPS:43 5967 | 21-225_192B3 | VH4\|4-30.1/D3\|3-22\|RF2/JH6 | ............ ............ ........ | .....D...N | ........ ...... | F.F..... ........ .... | ................. ....V.......... | GD..G..S.– ...........– HH...... | ...... ..... |
| iPS:43 5973 | 21-225_192H3 | VH4\|4-30.1/D3\|3-22\|RF2/JH6 | ............ ............ ........ | .V...S.... | ....R... ...... | NL...... ........ ..R. | .A............... .............T. | GD..G..S.– ...........– H..H.... | ...... ..... |
| iPS:43 6007 | 21-225_192G12 | VH4\|4-30.1/D3\|3-22\|RF2/JH6 | ............ ............ ........ | .....V.H.. | ........ ...... | N.H..... .....N.. .... | ................. ............... | GD..G..S.– ...........– H....... | ...... ..... |
| iPS:43 6009 | 21-225_193A1 | VH4\|4-30.1/D3\|3-22\|RF2/JH6 | ............ ............ ........ | .....V.... | ........ ...... | N....... .....N.. .... | .L...A........... ............... | GD..G..S.– ...........– H....... | ...... ..... |
| iPS:43 6011 | 21-225_193B1 | VH4\|4-30.1/D3\|3-22\|RF2/JH6 | ............ ............ ........ | .....V.... | ........ ...... | N....... .....N.. .... | ................. ............... | GD..G..S.– ...........– H....... | ...... ..... |
| iPS:43 6017 | 21-225_193F3 | VH4\|4-30.1/D3\|3-22\|RF2/JH6 | ............ ............ .......N | .....D...N | ........ ...... | ..F..... ........ .... | ................. ............... | GD..G..S.– ...........– H....... | ...... ..... |
| iPS:43 6029 | 21-225_193H6 | VH4\|4-30.1/D3\|3-22\|RF2/JH6 | ............ ............ .......N | .....D...N | ........ ...... | ..F..... ........ .... | ................. ............... | GD..G..S.– ...........– H....... | ...... ..... |
| iPS:43 6035 | 21-225_193C8 | VH4\|4-30.1/D3\|3-22\|RF2/JH6 | ............ ............ ........ | .....D...N | ........ ...... | ..F..... ........ .... | ................. ............... | GD..G..S.– ...........– H....... | ...... ..... |

| | | | | | | | | | |
|---|---|---|---|---|---|---|---|---|---|
| iPS:43 6037 | 21-225_193D8 | VH4\|4-30.1/D3\|3-22\|RF2/JH6 | ............<br>............<br>.......N | .........N | ........<br>...... | F.F.....<br>........<br>.... | ..S..............<br>............... | GD..G..S.–<br>...........–<br>H....... | ......<br>..... |
| iPS:43 6041 | 21-225_193G8 | VH4\|4-30.1/D3\|3-22\|RF2/JH6 | ......K.....<br>............<br>......V. | .....V.... | ........<br>...... | N.......<br>.....N..<br>.... | .................<br>N.............. | GD..G..S.–<br>...........–<br>HF...L.. | ..H...<br>..... |
| iPS:43 6062 | 21-225_194E5 | VH4\|4-30.1/D3\|3-22\|RF2/JH6 | ............<br>............<br>.......N | .....D...N | ...H....<br>...... | ..F.....<br>........<br>.... | .................<br>............... | GD..G..S.–<br>...........–<br>H....... | ......<br>..... |
| iPS:43 6064 | 21-225_194E6 | VH4\|4-30.1/D3\|3-22\|RF2/JH6 | ............<br>............<br>....D..N | .....D...N | ........<br>...... | F.F.....<br>........<br>.... | .....I..........<br>...NV.......... | GD..G..S.–<br>...........–<br>H....... | ......<br>..... |
| iPS:43 6134 | 21-225_196H12 | VH4\|4-30.1/D3\|3-22\|RF2/JH6 | ............<br>.N..........<br>........ | .....V.... | ........<br>...... | N.......<br>.....N..<br>.... | .II..............<br>............... | GD..G..S.–<br>...........–<br>H....L.. | ......<br>..... |
| iPS:43 6146 | 21-225_197F4 | VH4\|4-30.1/D3\|3-22\|RF2/JH6 | ............<br>............<br>........ | .....D...N | ........<br>...... | ..F.....<br>........<br>.... | .I...............<br>............... | GD..G..S.–<br>...........–<br>H....L.. | ......<br>..... |
| iPS:43 6177 | 21-225_198B1 | VH4\|4-30.1/D3\|3-22\|RF2/JH6 | ....K.......<br>............<br>.......N | .....D...N | .F......<br>...... | ..FH....<br>........<br>.... | ...V............<br>............... | GD..G..S.–<br>...........–<br>H....... | ..R...<br>..... |
| iPS:43 6179 | 21-225_198E1 | VH4\|4-30.1/D3\|3-22\|RF2/JH6 | ............<br>............<br>.......N | .........N | ........<br>...... | F.F.....<br>........<br>.... | .LS..............<br>............... | GD..G..S.–<br>...........–<br>H....... | ......<br>..... |
| iPS:43 6197 | 21-225_199C2 | VH4\|4-30.1/D3\|3-22\|RF2/JH6 | ............<br>............<br>........ | .....D...N | .L....E.<br>...... | ..F.....<br>........<br>.... | .........MT......<br>T.............. | GD..G..S.–<br>...........–<br>H....... | ......<br>..... |

| | | | | | | | | | |
|---|---|---|---|---|---|---|---|---|---|
| iPS:436207 | 21-225_199C7 | VH4\|4-30.1/D3\|3-22\|RF2/JH6 | ............ ............ .......N | .........N | ........ ...... | F.F..... ........ .... | ..S............. .............. | GD..G..S.- ...........- H....... | ...... ..... |
| iPS:436226 | 21-225_200F10 | VH4\|4-30.1/D3\|3-22\|RF2/JH6 | ............ ............ ........ | .....D...N | .L....E. ...... | ..F..... ........ .... | .I.......MT...... T............. | GD..G..S.- ...........- H....... | ...... ..... |
| | | Germline | H_FR1 | H_CDR1 | H_FR2 | H_CDR2 | H_FR3 | H_CDR3 | H_FR4 |
| | VH4\|4-30.4/D3\|3-22\|RF2/JH6 | | QVQLQES-GPGLVKPSQTLSLTCTVSG-GSIS | SG---DYYWS | WIRQPPGKGLEWIG | YIYY----SGSTYYNPSLKS | RVTISVDTSKNQFSLKLSSVTAADTAVYYCAR | YYYDSSGYYY---------YYYYYGMDV | WGQGTTVTVSS |
| iPS:435849 | 21-225_191C3 | VH4\|4-30.4/D3\|3-22\|RF2/JH6 | ............ ............ ........ | .........N | ....L... ...... | ..F..... ........ .... | .L.............. N............. | GD..G..S.- ...........- HF...... | ...... ..... |
| iPS:436015 | 21-225_193D3 | VH4\|4-30.4/D3\|3-22\|RF2/JH6 | ............ ............ ........ | .........N | ....L... ...... | ..F..... ........ .... | .L.............. .............T. | GD..G..S.- ...........- HF...... | ...... ..... |
| iPS:436049 | 21-225_193B12 | VH4\|4-30.4/D3\|3-22\|RF2/JH6 | ............ ............ ........ | .A.......N | ....L... ...... | ..F..... ........ .... | .L.............. .............. | GD..G..S.- ...........- HF...... | ...... ..... |
| iPS:436088 | 21-225_195C8 | VH4\|4-30.4/D3\|3-22\|RF2/JH6 | ............ ............ ........ | .........N | ....L... ...... | ..F..... ........ .... | .L.............. .............. | GD..G..S.- ...........- HF...... | ...... ..... |
| iPS:436195 | 21-225_198G10 | VH4\|4-30.4/D3\|3-22\|RF2/JH6 | ............ ............ ........ | .........N | ....L... ...... | ..F..... ........ .... | .L.............. .............. | GD..G..S.- ...........- HF...... | ...... ..... |
| | | Germline | H_FR1 | H_CDR1 | H_FR2 | H_CDR2 | H_FR3 | H_CDR3 | H_FR4 |
| | VH4\|4-30.1/D5\|5-24\|RF3/JH5 | | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | RDGYNY------------------NWFDP | WGQGTLVTVSS |

| | | | | | | | | | |
|---|---|---|---|---|---|---|---|---|---|
| iPS:43 5863 | 21-225_191H4 | VH4\|4-30.1/D5\|5-24\|RF3/JH5 | ............<br>............<br>........ | .........N | ........<br>...... | ..F.....<br>........<br>..R. | .L...I...........<br>T............G. | SGYNWD...........<br>........GV.. | ......<br>..... |
| iPS:43 5943 | 21-225_191C9 | VH4\|4-30.1/D5\|5-24\|RF3/JH5 | ............<br>............<br>........ | .........N | ........<br>..D... | ........<br>........<br>.... | .................<br>N............. | SGYNWD...........<br>.......AGV.. | ......<br>..... |
| iPS:43 6094 | 21-225_195B10 | VH4\|4-30.1/D5\|5-24\|RF3/JH5 | ............<br>............<br>........ | NS........ | ........<br>...... | .M......<br>........<br>.... | .............Y...<br>.A...........K | GGYNWN...........<br>........G..C | ......<br>..... |
| | | Germline | **H_FR1** | **H_CDR1** | **H_FR2** | **H_CDR2** | **H_FR3** | **H_CDR3** | **H_FR4** |
| | VH3\|3-33/D2\|2-15\|RF3/JH6 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | DIVVVVAAT---------<br>---YYYYYGMDV | WGQGTT<br>VTVSS |
| iPS:43 5869 | 21-225_190B1 | VH3\|3-33/D2\|2-15\|RF3/JH6 | ............<br>............<br>T....... | .......... | ........<br>...... | ........<br>....H...<br>.... | .................<br>.............. | .RT.GY---<br>...........----<br>S.... | ......<br>..... |
| iPS:43 6260 | 21-225_203H1 | VH3\|3-33/D2\|2-15\|RF3/JH6 | ............<br>............<br>........ | .......... | ........<br>...... | ........<br>....N...<br>.... | .................<br>.............. | .RT.GY---<br>...........----<br>N.... | ......<br>..... |
| iPS:43 6490 | 21-225_221F6 | VH3\|3-33/D2\|2-15\|RF3/JH6 | ............<br>............<br>........ | ......N... | ........<br>...... | ........<br>...EN...<br>.... | .................<br>.............. | .RT.GY---<br>...........----<br>N.... | ......<br>..... |
| iPS:43 6502 | 21-225_222A11 | VH3\|3-33/D2\|2-15\|RF3/JH6 | ............<br>............<br>........ | .......... | ........<br>...... | ........<br>....N...<br>.... | .................<br>.............. | .RD.GY---<br>...........----<br>N.... | ......<br>..... |
| iPS:43 6514 | 21-225_222D10 | VH3\|3-33/D2\|2-15\|RF3/JH6 | ............<br>............<br>........ | .......... | ........<br>...... | ........<br>....N...<br>.... | .................<br>.............. | .RD.GY---<br>...........----<br>N.... | ......<br>..... |
| iPS:43 6522 | 21-225_223H10 | VH3\|3-33/D2\|2-15\|RF3/JH6 | ............<br>............<br>........ | .......... | ........<br>...... | ........<br>....N...<br>.... | .......H.........<br>.............. | .RD.GY---<br>...........----<br>N.... | ......<br>..... |
| | | Germline | **H_FR1** | **H_CDR1** | **H_FR2** | **H_CDR2** | **H_FR3** | **H_CDR3** | **H_FR4** |
| | VH3\|3-21/D1\|1- | | EVQLVES- | S-----YSMN | WVRQAPGK | SISSS--- | RFTISRDNAKNSLYLQM | YNWND------------ | WGQGTL |

| | | | | | | | | | |
|---|---|---|---|---|---|---|---|---|---|
| iPS:435883 | 21-225_185A1 | VH3\|3-21/D1\|1-1\|RF3/JH5 | ...............................N | .......... | .............. | ........G........... | .................H.............. | S.L--...................--..C | .....P..... |
| | | Germline | H_FR1 | H_CDR1 | H_FR2 | H_CDR2 | H_FR3 | H_CDR3 | H_FR4 |
| | VH3\|3-23/D1\|1-1\|RF1/JH3 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GTTGT--------------------DAFDI | WGQGTMVTVSS |
| iPS:435895 | 21-225_188E8 | VH3\|3-23/D1\|1-1\|RF1/JH3 | ................................ | ......S..N | .............. | V.........Y......... | ............................F... | RN.DD.................-.... | ........... |
| | | Germline | H_FR1 | H_CDR1 | H_FR2 | H_CDR2 | H_FR3 | H_CDR3 | H_FR4 |
| | VH3\|3-11/D7\|7-27\|RF3/JH4 | | QVQLVES-GGGLVKPGGSLRLSCAASG-FTFS | D-----YYMS | WIRQAPGKGLEWVS | YISSS---GSIIYYADSVKG | RFTISRDNAKNSLYLQMNSLRAEDTAVYYCAR | NWGY-----------------------FDY | WGQGTLVTVSS |
| iPS:435903 | 21-225_190E2 | VH3\|3-11/D7\|7-27\|RF3/JH4 | ................................ | .......... | ............L. | .........T.VF....... | ................................ | E.VG.......................A.. | ........... |
| iPS:435923 | 21-225_190H6 | VH3\|3-11/D7\|7-27\|RF3/JH4 | ................................ | .......... | ............L. | .........T.VF....... | ................................ | E.VG.......................A.. | ........... |
| iPS:435953 | 21-225_191B12 | VH3\|3-11/D7\|7-27\|RF3/JH4 | ................................ | .......... | .............. | .........T.VF....... | ................................ | E.VG.......................A.. | ........... |
| iPS:436098 | 21-225_195G11 | VH3\|3-11/D7\|7-27\|RF3/JH4 | ................................ | .......... | ............L. | .........T.VF....... | ................................ | E.VG.......................A.. | ........... |
| iPS:436102 | 21-225_196B1 | VH3\|3-11/D7\|7-27\|RF3/JH4 | ................................ | .......... | ............I. | .........I.M........ | ................................ | E.VG.......................A.. | ........... |
| iPS:436104 | 21-225_196C1 | VH3\|3-11/D7\|7-27\|RF3/JH4 | ................................ | .......... | ............L. | .........T.VF....... | ................................ | E.VG.......................A.. | ........... |
| | | Germline | H_FR1 | H_CDR1 | H_FR2 | H_CDR2 | H_FR3 | H_CDR3 | H_FR4 |
| | VH3\|3-23/D6\|6-19\|RF2/JH4 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GIAVA--------------------GYFDY | WGQGTLVTVSS |

| | | | | | | | | | |
|---|---|---|---|---|---|---|---|---|---|
| iPS:43 5965 | 21-225_192H2 | VH3\|3-23/D6\|6-19\|RF2/JH4 | ..........D.<br>I...........<br>........ | ......S... | ........<br>...... | ........<br>..N.F...<br>.... | .................<br>............... | L...VG...........<br>......SH.... | ......<br>..... |
| iPS:43 6160 | 21-225_197C9 | VH3\|3-23/D6\|6-19\|RF2/JH4 | ............<br>A...........<br>.......R | .......... | ........<br>...... | V...R...<br>..N.....<br>.... | .................<br>............... | .....G...........<br>......SH.... | ......<br>..... |
| iPS:39 2954 | 21-225_26A10 | VH3\|3-23/D6\|6-19\|RF2/JH4 | ............<br>............<br>........ | .......... | ........<br>...... | V.......<br>.VN.F...<br>.... | ...............L.<br>............... | K....G...........<br>......TH.... | ......<br>..... |
| | | Germline | H_FR1 | H_CDR1 | H_FR2 | H_CDR2 | H_FR3 | H_CDR3 | H_FR4 |
| | VH4\|4-30.1/D5\|5-24\|RF3/JH4 | | QVQLQES-<br>GPGLVKPSQTLS<br>LTCTVSG-GSIS | SG---GYYWS | WIRQHPGK<br>GLEWIG | YIYY----<br>SGSTYYNP<br>SLKS | RVTISVDTSKNQFSLKL<br>SSVTAADTAVYYCAR | RDGYN------------<br>-------YYFDY | WGQGTL<br>VTVSS |
| iPS:43 5983 | 21-225_192E5 | VH4\|4-30.1/D5\|5-24\|RF3/JH4 | ............<br>............<br>....D..N | N......... | ........<br>...... | ..F.....<br>........<br>.... | .................<br>N.........F... | AGYNWD...........<br>.......NG... | ......<br>..... |
| iPS:43 6043 | 21-225_193G9 | VH4\|4-30.1/D5\|5-24\|RF3/JH4 | ............<br>............<br>....D..N | N......... | ........<br>...... | ..F.....<br>........<br>.... | .................<br>N.........F... | AGYNWD...........<br>.......NG... | ......<br>..... |
| iPS:43 6084 | 21-225_195F2 | VH4\|4-30.1/D5\|5-24\|RF3/JH4 | ............<br>............<br>....D... | .......... | ........<br>...... | .S......<br>....N...<br>.... | .......M.........<br>....D.......... | GGYNWN...........<br>.......NG... | ....A.<br>..... |
| iPS:43 7138 | 21-225_214D8 | VH4\|4-30.1/D5\|5-24\|RF3/JH4 | ............<br>............<br>........ | TA...F.... | ........<br>...... | ...F....<br>........<br>.... | ...............N.<br>............... | AR..HY...........<br>.......SI... | ......<br>..... |
| | | Germline | H_FR1 | H_CDR1 | H_FR2 | H_CDR2 | H_FR3 | H_CDR3 | H_FR4 |
| | VH3\|3-23/D3\|3-16\|RF1/JH3 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | VL*LRLGEL--------<br>----SLYDAFDI | WGQGTM<br>VTVSS |
| iPS:43 6003 | 21-225_192G10 | VH3\|3-23/D3\|3-16\|RF1/JH3 | ............<br>...........S<br>........ | .......... | ........<br>...... | ....R...<br>....F...<br>.... | .................<br>..............R | R.A.DG---<br>..............--<br>...... | ......<br>..... |
| | | Germline | H_FR1 | H_CDR1 | H_FR2 | H_CDR2 | H_FR3 | H_CDR3 | H_FR4 |

| | | | | | | | | | |
|---|---|---|---|---|---|---|---|---|---|
| | VH3\|3-23/D7\|7-27\|RF2/JH4 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | *LGY-------------<br>---------FDY | WGQGTL<br>VTVSS |
| iPS:43 6019 | 21-225_193C4 | VH3\|3-23/D7\|7-27\|RF2/JH4 | ............<br>A...........<br>........ | .........N | ........<br>...... | ..I.N...<br>..R.....<br>.... | ..S..........F...<br>............... | D..RYS...........<br>......YGF... | ......<br>..... |
| | | Germline | H_FR1 | H_CDR1 | H_FR2 | H_CDR2 | H_FR3 | H_CDR3 | H_FR4 |
| | VH4\|4-30.1/D1\|1-1\|RF1/JH6 | | QVQLQES-<br>GPGLVKPSQTLS<br>LTCTVSG-GSIS | SG---GYYWS | WIRQHPGK<br>GLEWIG | YIYY----<br>SGSTYYNP<br>SLKS | RVTISVDTSKNQFSLKL<br>SSVTAADTAVYYCAR | GTTGTYY-----------<br>-----YYYGMDV | WGQGTT<br>VTVSS |
| iPS:43 6025 | 21-225_193B5 | VH4\|4-30.1/D1\|1-1\|RF1/JH6 | ............<br>............<br>........ | .......... | ........<br>...... | ........<br>........<br>.... | .................<br>...A........... | .EYNWN-<br>................--<br>H.... | ......<br>..... |
| | | Germline | H_FR1 | H_CDR1 | H_FR2 | H_CDR2 | H_FR3 | H_CDR3 | H_FR4 |
| | VH4\|4-34/D4\|4-11\|RF2/JH4 | | QVQLQQW-<br>GAGLLKPSETLS<br>LTCAVYG-GSFS | G-----YYWS | WIRQPPGK<br>GLEWIG | EINH----<br>SGSTNYNP<br>SLKS | RVTISVDTSKNQFSLKL<br>SSVTAADTAVYYCAR | DYSNY------------<br>--------YFDY | WGQGTL<br>VTVSS |
| iPS:43 6033 | 21-225_193E7 | VH4\|4-34/D4\|4-11\|RF2/JH4 | ............<br>............<br>........ | .......F.T | ........<br>...... | ........<br>........<br>.... | .................<br>............... | ..G--<br>.................<br>...-A.. | ......<br>.A..A |
| iPS:43 6199 | 21-225_199E3 | VH4\|4-34/D4\|4-11\|RF2/JH4 | ............<br>............<br>........ | .......F.T | ........<br>...... | ........<br>........<br>.... | .................<br>............... | ..G--<br>.................<br>...-A.. | ......<br>.A..A |
| iPS:43 6228 | 21-225_200F12 | VH4\|4-34/D4\|4-11\|RF2/JH4 | ............<br>............<br>........ | .......F.T | ........<br>...... | ..S.....<br>........<br>.... | ................V<br>............... | ..G--<br>.................<br>...-A.. | ......<br>..... |
| iPS:43 6230 | 21-225_201A1 | VH4\|4-34/D4\|4-11\|RF2/JH4 | ............<br>............<br>.T...... | .......F.T | ........<br>...... | ..S.....<br>..R.....<br>.... | .....G...........<br>............... | ..G--<br>.................<br>...-A.. | ......<br>..... |
| iPS:43 6242 | 21-225_201A10 | VH4\|4-34/D4\|4-11\|RF2/JH4 | ............<br>............<br>........ | .......F.T | ........<br>...... | ..S.....<br>........<br>.... | ................V<br>N.............. | ..G--<br>.................<br>...-A.. | ......<br>..... |
| iPS:43 6286 | 21-225_204H8 | VH4\|4-34/D4\|4-11\|RF2/JH4 | ............<br>.......F....<br>........ | .......F.T | .V......<br>...... | ..S.....<br>....S...<br>.... | .......K.........<br>............... | ..G--<br>.................<br>...-A.. | ......<br>..... |

| | | | | | | | | | |
|---|---|---|---|---|---|---|---|---|---|
| iPS:43 6308 | 21-225_205H8 | VH4\|4-34/D4\|4-11\|RF2/JH4 | ............<br>............<br>........ | .......F.. | ........<br>...... | ..S.....<br>..R.....<br>.... | ................V<br>............... | ..G--<br>..................<br>...-A.. | ......<br>..... |
| | | Germline | H_FR1 | H_CDR1 | H_FR2 | H_CDR2 | H_FR3 | H_CDR3 | H_FR4 |
| | VH3\|3-30.3/D1\|1-1\|RF1/JH4 | | QVQLVES-<br>GGGVVQPGRSLR<br>LSCAASG-FTFS | S-----YAMH | WVRQAPGK<br>GLEWVA | VISYD---<br>GSNKYYAD<br>SVKG | RFTISRDNSKNTLYLQM<br>NSLRAEDTAVYYCAR | GTTGT------------<br>--------YFDY | WGQGTL<br>VTVSS |
| iPS:43 6051 | 21-225_193G12 | VH3\|3-30.3/D1\|1-1\|RF1/JH4 | ............<br>...........G<br>........ | .......... | ........<br>...... | ..W.....<br>.T....G.<br>.... | .............N...<br>............... | DF.I.G...........<br>......AT.... | ......<br>..... |
| | | Germline | H_FR1 | H_CDR1 | H_FR2 | H_CDR2 | H_FR3 | H_CDR3 | H_FR4 |
| | VH3\|3-33/D7\|7-27\|RF3/JH6 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | NWGYYY-----------<br>------YYGMDV | WGQGTT<br>VTVSS |
| iPS:43 6054 | 21-225_194C1 | VH3\|3-33/D7\|7-27\|RF3/JH6 | ............<br>............<br>........ | .......... | ........<br>...... | ........<br>...EH...<br>.... | .................<br>............... | .R.VG............<br>......-..L.. | ......<br>..... |
| | | Germline | H_FR1 | H_CDR1 | H_FR2 | H_CDR2 | H_FR3 | H_CDR3 | H_FR4 |
| | VH1\|1-02/D5\|5-18\|RF3/JH1 | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | GYSYGY-----------<br>------AEYFQH | WGQGTL<br>VTVSS |
| iPS:43 6058 | 21-225_194A4 | VH1\|1-02/D5\|5-18\|RF3/JH1 | .........T..<br>......L.....<br>........ | V.......LN | ........<br>...... | ........<br>........<br>.... | .................<br>............... | ..DI--<br>..................<br>---LTG | ......<br>..... |
| | | Germline | H_FR1 | H_CDR1 | H_FR2 | H_CDR2 | H_FR3 | H_CDR3 | H_FR4 |
| | VH1\|1-02/D1\|1-26\|RF3/JH6 | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | YSGSYYYY---------<br>-----YYYGMDV | WGQGTT<br>VTVSS |
| iPS:43 6068 | 21-225_194F7 | VH1\|1-02/D1\|1-26\|RF3/JH6 | ............<br>............<br>........ | D.......I. | ........<br>...... | ........<br>N.......<br>.... | .................<br>............... | EPLG..GSG........<br>...SYGA..... | ......<br>..... |
| | | Germline | H_FR1 | H_CDR1 | H_FR2 | H_CDR2 | H_FR3 | H_CDR3 | H_FR4 |
| | VH4\|4-34/D4\|4-17\|RF2/JH3 | | QVQLQQW-<br>GAGLLKPSETLS<br>LTCAVYG-GSFS | G-----YYWS | WIRQPPGK<br>GLEWIG | EINH----<br>SGSTNYNP<br>SLKS | RVTISVDTSKNQFSLKL<br>SSVTAADTAVYYCAR | DYGDY------------<br>-------DAFDI | WGQGTM<br>VTVSS |
| iPS:43 6072 | 21-225_194C10 | VH4\|4-34/D4\|4-17\|RF2/J | ............<br>............<br>.S.....R | Y......... | ........<br>....F. | ........<br>........<br>.... | .....I..........<br>R............. | ...A-<br>..................<br>..--... | ......<br>..... |

| | | | | | | | | | |
|---|---|---|---|---|---|---|---|---|---|
| iPS:43 6506 | 21-225_222C7 | VH4\|4-34/D4\|4-17\|RF2/JH3 | ............ ............ ........ | .......R... | ........ ...... | ........ ...A.... .... | ................. ............... | ...A- ................... ..--L.F | ...... ..... |
| | | Germline | H_FR1 | H_CDR1 | H_FR2 | H_CDR2 | H_FR3 | H_CDR3 | H_FR4 |
| | VH3\|3-33/D5\|5-24\|RF2/JH6 | | QVQLVES- GGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | *RWLQLYY---------- -----YYYGMDV | WGQGTT VTVSS |
| iPS:43 6092 | 21-225_195B9 | VH3\|3-33/D5\|5-24\|RF2/JH6 | ............ ............ ........ | .......... | ........ ...... | ........ ........ .... | ................. ............... | EWLQFR.- ...............- ...... | ...... ..... |
| iPS:43 6164 | 21-225_197G10 | VH3\|3-33/D5\|5-24\|RF2/JH6 | ............ ...........V ........ | .......... | ........ ...... | .T...... ........ .... | ................. ............... | EWLQFR.- ...............- ...I.. | ...... ..... |
| iPS:43 6191 | 21-225_198B9 | VH3\|3-33/D5\|5-24\|RF2/JH6 | ............ ............ ........ | N......... | ........ ...... | I..F.... ......V. .... | .............F... S.............. | EWLQFR.- ...............- ...... | ...... ..... |
| iPS:43 6205 | 21-225_199A7 | VH3\|3-33/D5\|5-24\|RF2/JH6 | ............ ............ ........ | N......... | ........ ...... | I..F.... ........ .... | .............F... S.............. | EWLQFR.- ...............- ...... | ...... ..... |
| iPS:43 6214 | 21-225_200F6 | VH3\|3-33/D5\|5-24\|RF2/JH6 | ............ ............ ........ | .......... | ........ ...... | ........ ........ .... | ................. .............T. | EWLQFR.- ...............- ...... | ...... ..... |
| | | Germline | H_FR1 | H_CDR1 | H_FR2 | H_CDR2 | H_FR3 | H_CDR3 | H_FR4 |
| | VH3\|3-33/D6\|6-19\|RF3/JH6 | | QVQLVES- GGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | V*QWLVYY---------- -----YYYGMDV | WGQGTT VTVSS |
| iPS:43 6106 | 21-225_196F2 | VH3\|3-33/D6\|6-19\|RF3/JH6 | ............ ............ ........ | ......F... | ........ ...... | ..LN.... ....KC.. .... | .C............... ............... | GQ....-- ...............-- N.V.. | ...... ..... |
| | | Germline | H_FR1 | H_CDR1 | H_FR2 | H_CDR2 | H_FR3 | H_CDR3 | H_FR4 |
| | VH3\|3-23/D6\|6-6\|RF3/JH6 | | EVQLLES- GGGLVQPGGSLR LSCAASG-FTFS | S-----YAMS | WVRQAPGK GLEWVS | AISGS--- GGSTYYAD SVKG | RFTISRDNSKNTLYLQM NSLRAEDTAVYYCAK | V*QLVYY---------- -----YYYGMDV | WGQGTT VTVSS |
| iPS:43 6110 | 21-225_196F4 | VH3\|3-23/D6\|6-6\|RF3/JH6 | ............ ........F... ........ | ......C..T | ........ ...... | ........ ........ .... | ................. ............... | .GG.TGSY......... ............ | ...... ..... |
| | | Germline | H_FR1 | H_CDR1 | H_FR2 | H_CDR2 | H_FR3 | H_CDR3 | H_FR4 |

| | | | | | | | | | |
|---|---|---|---|---|---|---|---|---|---|
| | VH1\|1-08/D2\|2-21\|RF1/JH5 | | QVQLVQS-<br>GAEVKKPGASVK<br>VSCKASG-YTFT | S-----YDIN | WVRQATGQ<br>GLEWMG | WMNPN---<br>SGNTGYAQ<br>KFQG | RVTMTRNTSISTAYMEL<br>SSLRSEDTAVYYCAR | SILWW*L-----------<br>-----LFNWFDP | WGQGTL<br>VTVSS |
| iPS:43 6114 | 21-225_196G8 | VH1\|1-08/D2\|2-21\|RF1/JH5 | ............<br>............<br>........ | N......... | .M......<br>...... | ..HL....<br>.......P<br>.... | ......D......F...<br>..............Y | .GG.Y--<br>................--<br>-V... | ......<br>..... |
| iPS:43 6218 | 21-225_200G7 | VH1\|1-08/D2\|2-21\|RF1/JH5 | ............<br>............<br>........ | N......... | .M......<br>...... | ..HL....<br>.......P<br>.... | ......D......F...<br>..............Y | .GG.Y--<br>................--<br>-V... | ......<br>..... |
| | | Germline | H_FR1 | H_CDR1 | H_FR2 | H_CDR2 | H_FR3 | H_CDR3 | H_FR4 |
| | VH1\|1-02/D3\|3-10\|RF3/JH6 | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | ITMVRGVII---------<br>---YYYYYGMDV | WGQGTT<br>VTVSS |
| iPS:43 6116 | 21-225_196B9 | VH1\|1-02/D3\|3-10\|RF3/JH6 | ............<br>......M.....<br>........ | .......... | ........<br>...... | ........<br>.....F..<br>..R. | ................<br>...S........... | GGVRGVPN-<br>............--<br>...V... | ......<br>..... |
| | | Germline | H_FR1 | H_CDR1 | H_FR2 | H_CDR2 | H_FR3 | H_CDR3 | H_FR4 |
| | VH4\|4-30.1/D5\|5-18\|RF3/JH6 | | QVQLQES-<br>GPGLVKPSQTLS<br>LTCTVSG-GSIS | SG---GYYWS | WIRQHPGK<br>GLEWIG | YIYY----<br>SGSTYYNP<br>SLKS | RVTISVDTSKNQFSLKL<br>SSVTAADTAVYYCAR | GYSYGYYY----------<br>-----YYYGMDV | WGQGTT<br>VTVSS |
| iPS:43 6181 | 21-225_198C2 | VH4\|4-30.1/D5\|5-18\|RF3/JH6 | ............<br>............<br>........ | .......... | ........<br>...... | N.......<br>........<br>.... | ................<br>............... | .DY..SGSY.........<br>....HN...L.. | ......<br>..... |
| iPS:43 6210 | 21-225_199G11 | VH4\|4-30.1/D5\|5-18\|RF3/JH6 | ............<br>............<br>........ | .......... | ........<br>...... | N.......<br>........<br>.... | ................<br>............... | .DY..SGSY.........<br>....HN...L.. | ......<br>..... |
| | | Germline | H_FR1 | H_CDR1 | H_FR2 | H_CDR2 | H_FR3 | H_CDR3 | H_FR4 |
| | VH1\|1-18/D3\|3-3\|RF2/JH6 | | QVQLVQS-<br>GAEVKKPGASVK<br>VSCKASG-YTFT | S-----YGIS | WVRQAPGQ<br>GLEWMG | WISAY---<br>NGNTNYAQ<br>KLQG | RVTMTTDTSTSTAYMEL<br>RSLRSDDTAVYYCAR | YYDFWSGYYT--------<br>---YYYYYGMDV | WGQGTT<br>VTVSS |
| iPS:43 6234 | 21-225_51E3 | VH1\|1-18/D3\|3-3\|RF2/JH6 | ............<br>............<br>.......N | .......... | ...L....<br>...... | ........<br>....KN..<br>RF.. | ................<br>............... | HDFWSGY---<br>...........---<br>.K.... | ......<br>..... |
| iPS:43 6830 | 21-225_51F4 | VH1\|1-18/D3\|3-3\|RF2/JH6 | ............<br>............<br>.......N | .......... | ........<br>...... | ........<br>....K...<br>.... | ................<br>............... | HDFWSGY---<br>...........---<br>.K.... | ......<br>..... |

| | | | | | | | | | |
|---|---|---|---|---|---|---|---|---|---|
| iPS:43 6834 | 21-225_52F1 | VH1\|1-18/D3\|3-3\|RF2/JH6 | ............<br>............<br>.......N | .........V. | ........<br>...... | ........<br>...RK...<br>.... | ..S...............<br>............... | HDFWSGY---<br>...........---<br>.K.... | ......<br>..... |
| iPS:43 6842 | 21-225_54E9 | VH1\|1-18/D3\|3-3\|RF2/JH6 | ............<br>............<br>.......N | .......... | ...L....<br>...... | ........<br>....KN..<br>.... | .................<br>............... | HDFWSGY---<br>...........---<br>.K.... | ......<br>..... |
| iPS:43 6844 | 21-225_56G1 | VH1\|1-18/D3\|3-3\|RF2/JH6 | ............<br>............<br>.......N | .......... | ........<br>...... | ........<br>....K...<br>.F.. | .................<br>............... | HDFWSGY---<br>...........---<br>.K.... | ......<br>..... |
| iPS:43 6846 | 21-225_56E3 | VH1\|1-18/D3\|3-3\|RF2/JH6 | ............<br>............<br>.......N | .........F. | ........<br>...... | ........<br>....KE..<br>.F.. | .................<br>....A.......... | HDFWSGY---<br>...........---<br>.K.... | ......<br>..... |
| iPS:45 1104 | 21-225_49C5 | VH1\|1-18/D3\|3-3\|RF2/JH6 | ............<br>............<br>.......N | .......... | ........<br>...... | ........<br>....K...<br>.... | .................<br>............... | HDFWSGY---<br>...........---<br>.K.... | ......<br>..... |
| iPS:45 1106 | 21-225_49D10 | VH1\|1-18/D3\|3-3\|RF2/JH6 | ............<br>............<br>.......N | .......... | ...L....<br>.F.... | ........<br>....KN..<br>.... | .................<br>............... | HDFWSGY---<br>...........---<br>.K.... | ......<br>..... |
| iPS:45 1108 | 21-225_53E8 | VH1\|1-18/D3\|3-3\|RF2/JH6 | ............<br>............<br>.......N | .......... | ........<br>...... | ........<br>....KF..<br>.... | .................<br>............... | HDFWSGY---<br>...........---<br>.K.... | ......<br>..... |
| | | Germline | H_FR1 | H_CDR1 | H_FR2 | H_CDR2 | H_FR3 | H_CDR3 | H_FR4 |
| | VH6\|6-01/D3\|3-9\|RF1/JH6 | | QVQLQQS-<br>GPGLVKPSQTLS<br>LTCAISG-DSVS | SN---SAAWN | WIRQSPSR<br>GLEWLG | RTYYR--<br>SKWYNDYA<br>VSVKS | RITINPDTSKNQFSLQL<br>NSVTPEDTAVYYCAR | VLRYFDWLL*--------<br>---YYYYYGMDV | WGQGTT<br>VTVSS |
| iPS:43 6236 | 21-225_201F7 | VH6\|6-01/D3\|3-9\|RF1/JH6 | ............<br>............<br>........ | .......... | ........<br>...... | ........<br>....Y.E.<br>..R. | .................<br>...........F... | DQ..Y-----<br>...........-----<br>.... | ......<br>..... |
| iPS:43 6250 | 21-225_201A4 | VH6\|6-01/D3\|3-9\|RF1/JH6 | ............<br>............<br>........ | .......... | ........<br>...... | ........<br>....Y.E.<br>.... | .................<br>...........F... | DQ..Y-----<br>...........-----<br>.... | ......<br>..... |
| iPS:43 6252 | 21-225_202A8 | VH6\|6-01/D3\|3-9\|RF1/JH6 | ............<br>............<br>........ | .......... | ........<br>...... | ........<br>....E...<br>..R. | .................<br>.........L...T. | DQ..Y-----<br>...........-----<br>.... | .....P<br>..... |

| | | | | | | | | | |
|---|---|---|---|---|---|---|---|---|---|
| iPS:43 6278 | 21-225_201F2 | VH6\|6-01/D3\|3-9\|RF1/JH6 | .............<br>.............<br>........ | .......... | ........<br>...... | ........<br>....Y.E.<br>..R. | .V...............<br>...........F... | DQ..Y-----<br>...........-----<br>.... | ......<br>..... |
| iPS:43 6294 | 21-225_205G4 | VH6\|6-01/D3\|3-9\|RF1/JH6 | .............<br>.............<br>........ | .......... | ........<br>...... | ........<br>....Y.E.<br>..R. | .................<br>...........F... | DQ..Y-----<br>...........-----<br>.... | ......<br>..... |
| iPS:43 6356 | 21-225_210H10 | VH6\|6-01/D3\|3-9\|RF1/JH6 | .............<br>.............<br>........ | .......... | ........<br>...... | ........<br>....Y.P.<br>..R. | ...............L.<br>............... | DQ..Y-----<br>...........-----<br>.... | ......<br>..... |
| | | Germline | H_FR1 | H_CDR1 | H_FR2 | H_CDR2 | H_FR3 | H_CDR3 | H_FR4 |
| | VH1\|1-02/D4\|4-23\|RF2/JH4 | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | DYGGN------------<br>-------SYFDY | WGQGTL<br>VTVSS |
| iPS:43 6240 | 21-225_201E8 | VH1\|1-02/D4\|4-23\|RF2/JH4 | .............<br>.............<br>........ | .......... | ........<br>...... | ..D.....<br>......P.<br>.... | .................<br>..........F...K | .Q.Y.W...........<br>.......NS... | ......<br>..... |
| iPS:43 6314 | 21-225_206G4 | VH1\|1-02/D4\|4-23\|RF2/JH4 | .............<br>.............<br>........ | ........I. | ........<br>...... | ..D.....<br>........<br>.... | .I...............<br>..........F...K | .Q.Y.W...........<br>.......NS... | ......<br>..... |
| | | Germline | H_FR1 | H_CDR1 | H_FR2 | H_CDR2 | H_FR3 | H_CDR3 | H_FR4 |
| | VH1\|1-02/D5\|5-18\|RF3/JH5 | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | GYSYGY-----------<br>-------NWFDP | WGQGTL<br>VTVSS |
| iPS:43 6244 | 21-225_201H10 | VH1\|1-02/D5\|5-18\|RF3/JH5 | .............<br>.............<br>........ | ........I. | ........<br>.....A | ........<br>........<br>.... | ..........T.T....<br>............... | .................<br>............ | ......<br>..... |
| iPS:43 6262 | 21-225_203E3 | VH1\|1-02/D5\|5-18\|RF3/JH5 | .............<br>.............<br>........ | ........I. | ........<br>.....A | ........<br>........<br>.... | ..........T......<br>............... | .................<br>............ | ......<br>..... |
| iPS:43 6276 | 21-225_204H4 | VH1\|1-02/D5\|5-18\|RF3/JH5 | .............<br>.............<br>........ | ........I. | ........<br>.....A | ........<br>........<br>.... | ..........T......<br>............... | .................<br>............ | ......<br>..... |
| iPS:43 6312 | 21-225_206A4 | VH1\|1-02/D5\|5-18\|RF3/JH5 | .............<br>.............<br>........ | ........I. | ........<br>.....A | ........<br>........<br>.... | ..........T......<br>............... | .................<br>............ | ......<br>..... |

| | | | | | | | | | |
|---|---|---|---|---|---|---|---|---|---|
| iPS:436316 | 21-225_206A5 | VH1\|1-02/D5\|5-18\|RF3/JH5 | ............<br>............<br>........ | .........I. | ........<br>.....A | ........<br>........<br>.... | ..........T......<br>............... | ..................<br>............ | ......<br>..... |
| iPS:436338 | 21-225_208E8 | VH1\|1-02/D5\|5-18\|RF3/JH5 | ............<br>............<br>........ | .........I. | ........<br>.....A | ........<br>........<br>.... | ..........T......<br>............... | ..................<br>............ | ......<br>..... |
| iPS:436344 | 21-225_208B11 | VH1\|1-02/D5\|5-18\|RF3/JH5 | ............<br>............<br>........ | .........I. | ........<br>.....A | ........<br>........<br>.... | ..........T......<br>............... | ..................<br>............ | ......<br>..... |
| iPS:436358 | 21-225_210D11 | VH1\|1-02/D5\|5-18\|RF3/JH5 | ............<br>............<br>........ | .........I. | ........<br>.....A | ........<br>........<br>.... | ..........T......<br>............... | ..................<br>............ | ......<br>..... |
| iPS:436408 | 21-225_214H8 | VH1\|1-02/D5\|5-18\|RF3/JH5 | ............<br>............<br>........ | ......H.I. | ........<br>...... | ...S....<br>........<br>.... | .................<br>..............K | DGR.S.G..........<br>......YD.... | ......<br>..... |
| iPS:436424 | 21-225_215H6 | VH1\|1-02/D5\|5-18\|RF3/JH5 | ............<br>............<br>........ | ......H.I. | ........<br>...... | ...S....<br>.......E<br>.... | .................<br>..............K | DGR.S.G..........<br>......HD.... | ......<br>..... |
| iPS:437092 | 21-225_210B12 | VH1\|1-02/D5\|5-18\|RF3/JH5 | ............<br>........I...<br>....F... | D.......N | ........<br>...... | ........<br>........<br>.... | .................<br>............... | ..DS--<br>..................<br>.--.A. | ......<br>..... |
| iPS:437134 | 21-225_213A7 | VH1\|1-02/D5\|5-18\|RF3/JH5 | ............<br>........I..R<br>....F... | D.......N | ........<br>...... | ....K...<br>N.......<br>.... | .........L.R.....<br>..............K | ..DS--<br>..................<br>.--.A. | ......<br>..... |
| iPS:437194 | 21-225_226B2 | VH1\|1-02/D5\|5-18\|RF3/JH5 | ............<br>............<br>........ | .......F.. | ........<br>...... | ........<br>..D.....<br>.... | .........LN......<br>.........I..... | .TY..SGS.........<br>.....YF.EL.S | ......<br>..... |
| iPS:437196 | 21-225_226B7 | VH1\|1-02/D5\|5-18\|RF3/JH5 | ............<br>............<br>........ | .......... | ........<br>...... | ........<br>........<br>...D | ..............H...<br>............... | ..Y..SGS.........<br>.....YY....S | ......<br>..... |
| iPS:437200 | 21-225_226A10 | VH1\|1-02/D5\|5-18\|RF3/JH5 | ............<br>............<br>........ | .......F.. | ........<br>...... | ........<br>..D.....<br>.... | .........L.......<br>.........I..... | .TY..SGS.........<br>.....YF.EL.S | ......<br>..... |
| iPS:39 | 21- | VH1\|1-02/D5\|5- | ............<br>............ | .......... | ........<br>...... | ........<br>.D...... | .................<br>N............. | .FY..SGS.........<br>.....YY.DL.. | ......<br>..... |

| | | | | | | | | | |
|---|---|---|---|---|---|---|---|---|---|
| iPS:39 3178 | 21-225_34D7 | VH1\|1-02/D5\|5-18\|RF3/JH5 | ............. ............. ........ | ........... | ......... ...... | ......... ......... .... | .................. ................ | ..Y..SGS.......... .....YY.DL.. | ...... ..... |
| iPS:39 8480 | 21-225_17G4 | VH1\|1-02/D5\|5-18\|RF3/JH5 | ............. ............. T....... | D......... | ......... ...... | ......... ......E. .... | .................. ...............S | .................. ............ | ...... ..... |
| iPS:39 8486 | 21-225_19A1 | VH1\|1-02/D5\|5-18\|RF3/JH5 | ............. ............. ........ | ........... | ......... ...... | ......... ......... .... | .................. ...............S | .................. ............ | ...... ..... |
| | | Germline | H_FR1 | H_CDR1 | H_FR2 | H_CDR2 | H_FR3 | H_CDR3 | H_FR4 |
| | VH1\|1-08/D6\|6-13\|RF1/JH6 | | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | GYSSSWYY-----------YYYYGMDV | WGQGTTVTVSS |
| iPS:43 6248 | 21-225_202A3 | VH1\|1-08/D6\|6-13\|RF1/JH6 | ....E........ ............. ........ | ........... | ......... ....L. | ....K... R....... .... | .............H... ................ | .RY.RED.......... ........D... | ...... ..... |
| | | Germline | H_FR1 | H_CDR1 | H_FR2 | H_CDR2 | H_FR3 | H_CDR3 | H_FR4 |
| | VH3\|3-11/D4\|4-17\|RF2/JH6 | | QVQLVES-GGGLVKPGGSLRLSCAASG-FTFS | D-----YYMS | WIRQAPGKGLEWVS | YISSS---GSTIYYADSVKG | RFTISRDNAKNSLYLQMNSLRAEDTAVYYCAR | DYGDYYY-----------YYYGMDV | WGQGTTVTVSS |
| iPS:43 6270 | 21-225_203F10 | VH3\|3-11/D4\|4-17\|RF2/JH6 | ............. ............. ........ | ........... | ......... .....L | ...G.... .T.T.... .... | .................. ................ | .R.G--- .................-- --L.. | ...... ..... |
| | | Germline | H_FR1 | H_CDR1 | H_FR2 | H_CDR2 | H_FR3 | H_CDR3 | H_FR4 |
| | VH3\|3-23/D1\|1-20\|RF1/JH6 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GITGTYY-----------YYYGMDV | WGQGTTVTVSS |
| iPS:43 6280 | 21-225_204D6 | VH3\|3-23/D1\|1-20\|RF1/JH6 | ...V......... ............. ........ | T......... | ......... ...... | ......... ......... .... | .................. D............... | ..S..GSY.......... .........V.. | ...... ..... |
| | | Germline | H_FR1 | H_CDR1 | H_FR2 | H_CDR2 | H_FR3 | H_CDR3 | H_FR4 |
| | VH3\|3-33/D7\|7-27\|RF2/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | *LGYYY------------YYGMDV | WGQGTTVTVSS |
| iPS:43 6284 | 21-225_204G8 | VH3\|3-33/D7\|7-27\|RF2/JH6 | ..........D. ............. ........ | ........... | .G...... ...... | ......... ...EN.VA .... | ....F............ ................ | D..IG............ ......-...... | ...... ..... |

| | | | | | | | | | |
|---|---|---|---|---|---|---|---|---|---|
| iPS:436968 | 21-225_190B10 | VH3\|3-33/D7\|7-27\|RF2/JH6 | ............................V........ | .......... | .............. | ...N......K..HV..... | ..........................L..... | D.DKRNFPY.............YY...... | ........... |
| iPS:437006 | 21-225_192G2 | VH3\|3-33/D7\|7-27\|RF2/JH6 | ................................ | .......... | .............T | ...N............... | ..........................L..... | D.DKRNFPY.............YY...... | ........... |
| iPS:437024 | 21-225_194F11 | VH3\|3-33/D7\|7-27\|RF2/JH6 | ................................ | .......... | .............. | ...N......K..HV..... | ..........................L..... | D.DKRNFPY.............YY...... | ........... |
| iPS:437028 | 21-225_194G12 | VH3\|3-33/D7\|7-27\|RF2/JH6 | ................................ | .......... | .............T | ...N............... | ..........................L..... | D.DKRNFPY.............YY...... | ........... |
| | | Germline | H_FR1 | H_CDR1 | H_FR2 | H_CDR2 | H_FR3 | H_CDR3 | H_FR4 |
| | VH4\|4-34/D6\|6-19\|RF3/JH3 | | QVQLQQW-GAGLLKPSETLSLTCAVYG-GSFS | G-----YYWS | WIRQPPGKGLEWIG | EINH---SGSTNYNPSLKS | RVTISVDTSKNQFSLKLSSVTAADTAVYYCAR | V*QWLV-------------------DAFDI | WGQGTMVTVSS |
| iPS:436290 | 21-225_205G3 | VH4\|4-34/D6\|6-19\|RF3/JH3 | .........................F...... | ......H... | .............. | .MY.....F.N......... | ...M......K........................ | .G...-...................-.... | ........... |
| | | Germline | H_FR1 | H_CDR1 | H_FR2 | H_CDR2 | H_FR3 | H_CDR3 | H_FR4 |
| | VH3\|3-30.3/D4\|4-17\|RF2/JH1 | | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | DYGDYA-------------------EYFQH | WGQGTLVTVSS |
| iPS:436306 | 21-225_201H4 | VH3\|3-30.3/D4\|4-17\|RF2/JH1 | ................................ | .......... | .............. | A.W..........N...... | ..............M.................. | .V.TVG.................AT..DC | ..P........ |
| | | Germline | H_FR1 | H_CDR1 | H_FR2 | H_CDR2 | H_FR3 | H_CDR3 | H_FR4 |
| | VH1\|1-18/D1\|1-1\|RF1/JH6 | | QVQLVQS-GAEVKKPGASVKVSCKASG-YTFT | S-----YGIS | WVRQAPGQGLEWMG | WISAY---NGNTNYAQKLQG | RVTMTTDTSTSTAYMELRSLRSDDTAVYYCAR | GTTGTYY-----------YYYGMDV | WGQGTTVTVSS |
| iPS:436362 | 21-225_210C12 | VH1\|1-18/D1\|1-1\|RF1/JH6 | ............T................... | N.....N... | .............. | ..N.....H......F.. | ................................ | DP.V.H...................... | ........... |
| iPS:436374 | 21-225_211C10 | VH1\|1-18/D1\|1-1\|RF1/JH6 | ................................ | R.....H... | ...L.......... | ........L......F.. | ............G.................... | DP.V.H...................... | ........... |

| | VH3\|3-33/D5\|5-18\|RF3/JH6 | | QVQLVES-GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | GYSYGYYY---------- ------YYYGMDV | WGQGTT VTVSS |
|---|---|---|---|---|---|---|---|---|---|
| iPS:436366 | 21-225_211A3 | VH3\|3-33/D5\|5-18\|RF3/JH6 | ............ ............ V...I... | .......... | .A...... ...... | ........ ....N.E. .... | ................. ............... | DG....-- ...............-- D.... | ...... ..... |
| iPS:436388 | 21-225_212H11 | VH3\|3-33/D5\|5-18\|RF3/JH6 | ............ ............ V...I... | .......... | .A...... ...... | ........ ....N.E. .... | ................. ............... | DG....-- ...............-- D.... | ...... ..... |
| iPS:436396 | 21-225_213E5 | VH3\|3-33/D5\|5-18\|RF3/JH6 | ............ ............ V...I... | .......... | .A...... ...... | ........ ....N.E. .... | ................. ............... | DG....-- ...............-- D.... | ...... ..... |
| iPS:436454 | 21-225_217B10 | VH3\|3-33/D5\|5-18\|RF3/JH6 | ............ ............ V...I... | .......... | .A...... ...... | ........ ....N.E. .... | ................. ............... | DG....-- ...............-- D.... | ...... ..... |
| iPS:436668 | 21-225_147B9 | VH3\|3-33/D5\|5-18\|RF3/JH6 | ............ ............ ........ | .......... | ........ ...... | ........ ........ .... | ................. ............... | DRD..DPPY......... ....Y....... | ...... ..... |
| iPS:436688 | 21-225_148C8 | VH3\|3-33/D5\|5-18\|RF3/JH6 | ............ ............ ........ | .......... | ........ ...... | ........ ........ .... | ................. ............... | DRD..DPPY......... ....Y....... | ...... ..... |
| iPS:436706 | 21-225_149A11 | VH3\|3-33/D5\|5-18\|RF3/JH6 | ............ ............ ........ | .......... | ........ ...... | ........ ........ .... | ................. ............... | DRD..DPPY......... ....Y....... | ...... ..... |
| iPS:436760 | 21-225_155E10 | VH3\|3-33/D5\|5-18\|RF3/JH6 | ............ ............ ........ | .......... | ........ ...... | ........ ........ .... | ................. ............... | DRD..DPPY......... ....Y....... | ...... ..... |
| iPS:436966 | 21-225_190C3 | VH3\|3-33/D5\|5-18\|RF3/JH6 | ............ ............ ........ | .......... | ........ ...... | ........ ........ .... | ................. ..............N | W.Y.Y--- ...............-- ..... | ...... ..... |
| iPS:436976 | 21-225_190D8 | VH3\|3-33/D5\|5-18\|RF3/JH6 | ..........D. ...........E ........ | ........L. | ........ ...... | ........ ........ .... | ................. ..............N | W.Y.Y--- ...............-- ..... | ...... ..... |
| iPS:437168 | 21-225_218G4 | VH3\|3-33/D5\|5-18\|RF3/J | ............ ............ | ........L. | ........ ...... | ........ ........ | ................. ....V.........N | W.Y.Y--- ...............-- | ...... ..... |

| | | Germline | H_FR1 | H_CDR1 | H_FR2 | H_CDR2 | H_FR3 | H_CDR3 | H_FR4 |
|---|---|---|---|---|---|---|---|---|---|
| | VH3\|3-33/D5\|5-24\|RF3/JH5 | | QVQLVES- GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | RDGYNY----------- -------NWFDP | WGQGTL VTVSS |
| iPS:43 6368 | 21-225_211G3 | VH3\|3-33/D5\|5-24\|RF3/JH5 | ............ ............ ........ | N......... | ........ ...... | ...H.... ........ .... | ................. ............... | L.YS............. .......G.... | ...... ..... |
| iPS:43 6426 | 21-225_215C7 | VH3\|3-33/D5\|5-24\|RF3/JH5 | ............ ............ ........ | Y......... | ........ ...... | ...H.... ........ .... | ................. ............... | L.YS............. .......G.... | ...... ..... |
| iPS:43 6432 | 21-225_215H12 | VH3\|3-33/D5\|5-24\|RF3/JH5 | ............ ............ ........ | N......... | ........ ...... | ...H.... ........ .... | ................. ............... | L.YS............. .......G.... | ...... ..... |
| | | Germline | H_FR1 | H_CDR1 | H_FR2 | H_CDR2 | H_FR3 | H_CDR3 | H_FR4 |
| | VH1\|1-18/D4\|4-11\|RF2/JH6 | | QVQLVQS- GAEVKKPGASVK VSCKASG-YTFT | S-----YGIS | WVRQAPGQ GLEWMG | WISAY--- NGNTNYAQ KLQG | RVTMTTDTSTSTAYMEL RSLRSDDTAVYYCAR | DYSNYYY---------- -----YYYGMDV | WGQGTT VTVSS |
| iPS:43 6402 | 21-225_213H12 | VH1\|1-18/D4\|4-11\|RF2/JH6 | ............ ............ ....F... | .........N | ........ ...... | ...VH... ....D... .F.. | ................. ............... | ..YY--- ...............-- -.... | .....K ..... |
| iPS:43 6500 | 21-225_222H3 | VH1\|1-18/D4\|4-11\|RF2/JH6 | ............ ............ ....F... | .........N | ........ ...... | ...V.... ........ .... | ................. ............... | ..YY--- ...............-- -.F.. | ...... ..... |
| iPS:43 6520 | 21-225_223G10 | VH1\|1-18/D4\|4-11\|RF2/JH6 | ............ ............ ....F... | .........N | ........ ...... | ...V.... S....... .... | ................. ............... | ..YY--- ...............-- -.... | ...... ..... |
| | | Germline | H_FR1 | H_CDR1 | H_FR2 | H_CDR2 | H_FR3 | H_CDR3 | H_FR4 |
| | VH3\|3-48/D2\|2-21\|RF1/JH4 | | EVQLVES- GGGLVQPGGSLR LSCAASG-FTFS | S-----YSMN | WVRQAPGK GLEWVS | YISSS--- SSTIYYAD SVKG | RFTISRDNAKNSLYLQM NSLRDEDTAVYYCAR | SILWW*L---------- ------LFYFDY | WGQGTL VTVSS |
| iPS:43 6436 | 21-225_216F10 | VH3\|3-48/D2\|2-21\|RF1/JH4 | ............ ............ .....S.R | .......... | ........ ...... | ..TG.... ........ .... | ................. ............... | .G.A--- ................- -VE.. | ...... ..... |
| | | Germline | H_FR1 | H_CDR1 | H_FR2 | H_CDR2 | H_FR3 | H_CDR3 | H_FR4 |
| | VH3\|3-48/D6\|6-6\|RF1/JH4 | | EVQLVES- GGGLVQPGGSLR LSCAASG-FTFS | S-----YSMN | WVRQAPGK GLEWVS | YISSS--- SSTIYYAD SVKG | RFTISRDNAKNSLYLQM NSLRDEDTAVYYCAR | EYSSS------------ -------SYFDY | WGQGTL VTVSS |

| | | | | | | | | | |
|---|---|---|---|---|---|---|---|---|---|
| iPS:43 6448 | 21-225_217A3 | VH3\|3-48/D6\|6-6\|RF1/JH4 | ............<br>............<br>........ | ........N.. | ........<br>...... | ........<br>RNI.....<br>.... | ......E......S...<br>D.............. | DG.Y.SG...........<br>.....WYWG... | ......<br>..... |
| | | Germline | H_FR1 | H_CDR1 | H_FR2 | H_CDR2 | H_FR3 | H_CDR3 | H_FR4 |
| | VH4\|4-59/D6\|6-19\|RF3/JH6 | | QVQLQES-<br>GPGLVKPSETLS<br>LTCTVSG-GSIS | S-----YYWS | WIRQPPGK<br>GLEWIG | YIYY----<br>SGSTNYNP<br>SLKS | RVTISVDTSKNQFSLKL<br>SSVTAADTAVYYCAR | V*QWLVYY----------<br>-----YYYGMDV | WGQGTT<br>VTVSS |
| iPS:43 6472 | 21-225_220E1 | VH4\|4-59/D6\|6-19\|RF3/JH6 | ............<br>............<br>........ | T......... | ........<br>...... | ........<br>..T.....<br>.... | .................<br>............... | DQ....RGR.........<br>...DN....... | ......<br>..... |
| | | Germline | H_FR1 | H_CDR1 | H_FR2 | H_CDR2 | H_FR3 | H_CDR3 | H_FR4 |
| | VH3\|3-23/D2\|2-15\|RF3/JH4 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | DIVVVVA-----------<br>------ATYFDY | WGQGTL<br>VTVSS |
| iPS:43 6504 | 21-225_222H4 | VH3\|3-23/D2\|2-15\|RF3/JH4 | ............<br>............<br>........ | N.....F... | ........<br>...... | S.V.....<br>..R.....<br>.... | .................<br>............... | .PYR.AV...........<br>.......GA... | ......<br>I.... |
| iPS:43 6510 | 21-225_222H8 | VH3\|3-23/D2\|2-15\|RF3/JH4 | ............<br>............<br>........ | N.....F... | ........<br>...... | S.V.....<br>..R.....<br>.... | .................<br>............... | .PYR.AV...........<br>.......GA... | ......<br>I.... |
| | | Germline | H_FR1 | H_CDR1 | H_FR2 | H_CDR2 | H_FR3 | H_CDR3 | H_FR4 |
| | VH3\|3-23/D3\|3-22\|RF2/JH1 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | YYYDSSGY----------<br>----YYAEYFQH | WGQGTL<br>VTVSS |
| iPS:43 6526 | 21-225_224A1 | VH3\|3-23/D3\|3-22\|RF2/JH1 | ...V........<br>............<br>........ | .......... | ........<br>...... | ....R...<br>........<br>A... | .................<br>............... | GS.....-<br>..............-<br>.YH.LD. | ......<br>..... |
| | | Germline | H_FR1 | H_CDR1 | H_FR2 | H_CDR2 | H_FR3 | H_CDR3 | H_FR4 |
| | VH1\|1-02/D4\|4-23\|RF2/JH6 | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | DYGGNSYY----------<br>-----YYYGMDV | WGQGTT<br>VTVSS |
| iPS:43 6536 | 21-225_224G1 | VH1\|1-02/D4\|4-23\|RF2/JH6 | ............<br>............<br>........ | .........I. | ........<br>...... | ....Y...<br>..D.....<br>.... | .................<br>....F.....F.... | .W..Y.S-<br>..................<br>.... | ......<br>..... |
| iPS:43 6548 | 21-225_224A7 | VH1\|1-02/D4\|4-23\|RF2/JH6 | ............<br>............<br>........ | .........I. | ........<br>...... | ....Y...<br>..D.....<br>.... | ..........T......<br>....F.....F.... | .W..Y.S-<br>..................<br>.... | ......<br>..... |

| | | | | | | | | | |
|---|---|---|---|---|---|---|---|---|---|
| iPS:43 6558 | 21-225_224C11 | VH1\|1-02/D4\|4-23\|RF2/JH6 | ............ ............ ........ | ........I. | ........ ...... | ....Y... ..D..... .... | ................. R...F.....F.... | .W..Y.S- .................F .... | ...... ..... |
| iPS:43 6562 | 21-225_224H11 | VH1\|1-02/D4\|4-23\|RF2/JH6 | ............ ............ ........ | ........I. | ....T... ...... | ....Y... ..D..... .... | ................. R...F.....F.... | .W..Y.S- .................. .... | ...... ..... |
| iPS:43 6572 | 21-225_225G4 | VH1\|1-02/D4\|4-23\|RF2/JH6 | ............ ............ ........ | ........I. | ........ ...... | ....Y... ..D..... .... | ................. R...F.....F.... | .W..Y.S- .................. .... | ...... ..... |
| iPS:43 6606 | 21-225_226G8 | VH1\|1-02/D4\|4-23\|RF2/JH6 | ............ ............ ........ | ........I. | ........ ...... | ....Y... ..D.K... .... | ................. ....F.....F.... | .W..Y.S- .................. .... | ...... ..... |
| iPS:43 6610 | 21-225_226F9 | VH1\|1-02/D4\|4-23\|RF2/JH6 | ............ ............ ........ | ........I. | ........ ...... | ....Y... ..D.K... .... | ......A.......... ....F.....F.... | .W..Y.S- .................. .... | ...... ..... |
| iPS:43 6612 | 21-225_226H9 | VH1\|1-02/D4\|4-23\|RF2/JH6 | ............ ............ ........ | ........I. | ........ ...... | ....Y... ..D..S.. .... | ................. ....F.....F.... | .W..Y.S- .................. .... | ...... ..... |
| iPS:43 6614 | 21-225_226F10 | VH1\|1-02/D4\|4-23\|RF2/JH6 | ..........G.. ..LR........ ........ | ........I. | ........ ...... | ....Y... ..D..... .... | .........V....... ....L.....F.... | .W..Y.S- .................. .... | .....P ..... |
| iPS:43 6618 | 21-225_226E11 | VH1\|1-02/D4\|4-23\|RF2/JH6 | ............ ............ ........ | ........I. | ........ ...... | ....Y... ..D..... .... | ................. ....F.....F.... | .W..Y.S- .................. .... | ...... ..... |
| iPS:43 6624 | 21-225_226H12 | VH1\|1-02/D4\|4-23\|RF2/JH6 | ............ ............ ........ | ........I. | ........ ...... | ....Y... ..D..... .... | ................. R...F.....F.... | .W..Y.S- .................. .... | ...... ..... |
| iPS:43 6626 | 21-225_227C1 | VH1\|1-02/D4\|4-23\|RF2/JH6 | ............ ............ ........ | ........T. | ........ ...... | ....Y... ........ .... | ................. ....F.......... | .W..Y.S- .................. .... | ...... ..... |
| iPS:43 6628 | 21-225_227F2 | VH1\|1-02/D4\|4-23\|RF2/JH6 | ..H......... ............ ........ | ........I. | ........ ...... | ....Y... ..D.K... .... | ................. ....F.....F.... | .W..Y.S- .................. .... | ...... ..... |
| iPS:43 | 21- | VH1\|1-02/D4\|4- | ............ ............ | ........I. | ........ ...... | ....Y... ..D..... | .........V....... ....F.....F.... | .W..Y.S- .................. | ...... ..... |

<table>
<tr><td></td><td></td><td>Germline</td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH4|4-39/D4|4-17|RF1/JH4</td><td></td><td>QLQLQES-<br>GPGLVKPSETLS<br>LTCTVSG-GSIS</td><td>SS---SYYWG</td><td>WIRQPPGK<br>GLEWIG</td><td>SIYY----<br>SGSTYYNP<br>SLKS</td><td>RVTISVDTSKNQFSLKL<br>SSVTAADTAVYYCAR</td><td>*LR*L-------------<br>---------YFDY</td><td>WGQGTL<br>VTVSS</td></tr>
<tr><td>iPS:436538</td><td>21-225_224C3</td><td>VH4|4-39/D4|4-17|RF1/JH4</td><td>............<br>..S.........<br>........</td><td>R.........</td><td>........<br>......</td><td>N.......<br>........<br>....</td><td>.................<br>...............</td><td>QG.DW............<br>........GV..</td><td>G.....<br>.....</td></tr>
<tr><td></td><td></td><td>Germline</td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH3|3-23/D4|4-11|RF2/JH5</td><td></td><td>EVQLLES-<br>GGGLVQPGGSLR<br>LSCAASG-FTFS</td><td>S-----YAMS</td><td>WVRQAPGK<br>GLEWVS</td><td>AISGS---<br>GGSTYYAD<br>SVKG</td><td>RFTISRDNSKNTLYLQM<br>NSLRAEDTAVYYCAK</td><td>DYSNY-------------<br>-------NWFDP</td><td>WGQGTL<br>VTVSS</td></tr>
<tr><td>iPS:436546</td><td>21-225_224D6</td><td>VH3|3-23/D4|4-11|RF2/JH5</td><td>...V........<br>............<br>....S...</td><td>......D...</td><td>........<br>......</td><td>........<br>.DN.F...<br>....</td><td>.................<br>...............</td><td>V..A.D............<br>......SH....</td><td>......<br>.....</td></tr>
<tr><td></td><td></td><td>Germline</td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH1|1-46/D6|6-6|RF2/JH6</td><td></td><td>QVQLVQS-<br>GAEVKKPGASVK<br>VSCKASG-YTFT</td><td>S-----YYMH</td><td>WVRQAPGQ<br>GLEWMG</td><td>IINPS---<br>GGSTSYAQ<br>KFQG</td><td>RVTMTRDTSTSTVYMEL<br>SSLRSEDTAVYYCAR</td><td>SIAARYY-----------<br>-----YYYGMDV</td><td>WGQGTT<br>VTVSS</td></tr>
<tr><td>iPS:436568</td><td>21-225_225B3</td><td>VH1|1-46/D6|6-6|RF2/JH6</td><td>............<br>............<br>........</td><td>..........</td><td>........<br>......</td><td>........<br>........<br>....</td><td>.................<br>.....A.........</td><td>DL...S.Y..........<br>.......F....</td><td>....A.<br>.....</td></tr>
<tr><td></td><td></td><td>Germline</td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH4|4-30.1/D4|4-17|RF2/JH6</td><td></td><td>QVQLQES-<br>GPGLVKPSQTLS<br>LTCTVSG-GSIS</td><td>SG---GYYWS</td><td>WIRQHPGK<br>GLEWIG</td><td>YIYY----<br>SGSTYYNP<br>SLKS</td><td>RVTISVDTSKNQFSLKL<br>SSVTAADTAVYYCAR</td><td>DYGDYYY-----------<br>-----YYYGMDV</td><td>WGQGTT<br>VTVSS</td></tr>
<tr><td>iPS:436580</td><td>21-225_225E7</td><td>VH4|4-30.1/D4|4-17|RF2/JH6</td><td>............<br>............<br>....N...</td><td>.....H....</td><td>........<br>......</td><td>F.......<br>T.......<br>....</td><td>.................<br>...............</td><td>EA...G-<br>...............-<br>......</td><td>......<br>.....</td></tr>
<tr><td>iPS:436926</td><td>21-225_78D10</td><td>VH4|4-30.1/D4|4-17|RF2/JH6</td><td>............<br>............<br>........</td><td>..........</td><td>........<br>......</td><td>........<br>I..V....<br>....</td><td>.................<br>...............</td><td>.AP.F--<br>...............--<br>-....</td><td>......<br>.....</td></tr>
<tr><td></td><td></td><td>Germline</td><td>H_FR1</td><td>H_CDR1</td><td>H_FR2</td><td>H_CDR2</td><td>H_FR3</td><td>H_CDR3</td><td>H_FR4</td></tr>
<tr><td></td><td>VH3|3-33/D4|4-17|RF2/JH1</td><td></td><td>QVQLVES-<br>GGGVVQPGRSLR<br>LSCAASG-FTFS</td><td>S-----YGMH</td><td>WVRQAPGK<br>GLEWVA</td><td>VIWYD---<br>GSNKYYAD<br>SVKG</td><td>RFTISRDNSKNTLYLQM<br>NSLRAEDTAVYYCAR</td><td>DYGDYA------------<br>-------EYFQH</td><td>WGQGTL<br>VTVSS</td></tr>
<tr><td>iPS:43</td><td>21-</td><td>VH3|3-33/D4|4-</td><td>............<br>............</td><td>T.........</td><td>........<br>......</td><td>I.......<br>.GY.....</td><td>.............F...<br>...............</td><td>.HY.FW............<br>......SG.LT.</td><td>......<br>.....</td></tr>
</table>

| | | Germline | H_FR1 | H_CDR1 | H_FR2 | H_CDR2 | H_FR3 | H_CDR3 | H_FR4 |
|---|---|---|---|---|---|---|---|---|---|
| | VH3\|3-23/D7\|7-27\|RF1/JH6 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | LTGYYY-----------<br>-------YYGMDV | WGQGTT<br>VTVSS |
| iPS:43 6652 | 21-225_146B11 | VH3\|3-23/D7\|7-27\|RF1/JH6 | ............<br>............<br>........ | .......... | ........<br>...... | V...G...<br>.S......<br>.... | .................<br>............... | WR.NPT............<br>......D..... | ......<br>..... |
| iPS:43 6654 | 21-225_146C11 | VH3\|3-23/D7\|7-27\|RF1/JH6 | ............<br>............<br>....I... | .......... | ........<br>...... | V...G...<br>.S......<br>.... | .................<br>............... | WR.NPT............<br>......D..... | ......<br>..... |
| iPS:43 6658 | 21-225_146A2 | VH3\|3-23/D7\|7-27\|RF1/JH6 | ............<br>............<br>........ | .......... | ........<br>...... | V...G...<br>.S......<br>.... | .............H...<br>............... | WR.NPT............<br>......D..... | ......<br>..... |
| iPS:43 6664 | 21-225_147E7 | VH3\|3-23/D7\|7-27\|RF1/JH6 | ............<br>............<br>........ | .......... | ........<br>...... | V...G...<br>.S......<br>.... | ...L.............<br>.........F.... | WR.NPT............<br>......D..... | ......<br>..... |
| iPS:43 6676 | 21-225_147E11 | VH3\|3-23/D7\|7-27\|RF1/JH6 | ............<br>............<br>........ | N......V.. | ........<br>...... | V...G...<br>.S......<br>.... | .................<br>............... | WR.NPT............<br>......D..... | ......<br>..... |
| iPS:43 6678 | 21-225_147B12 | VH3\|3-23/D7\|7-27\|RF1/JH6 | ............<br>............<br>........ | .......... | ........<br>...... | V...G...<br>.S......<br>.... | .................<br>............... | WR.NPT............<br>......D..... | ......<br>..... |
| iPS:43 6686 | 21-225_148G6 | VH3\|3-23/D7\|7-27\|RF1/JH6 | ............<br>............<br>........ | .......... | ........<br>...... | V...G...<br>.S......<br>.... | ...........M.H...<br>............... | WR.NPT............<br>......D..... | ......<br>..... |
| iPS:43 6694 | 21-225_148G11 | VH3\|3-23/D7\|7-27\|RF1/JH6 | ............<br>............<br>........ | .......P.. | ........<br>...... | V...G...<br>.S.A....<br>.... | .................<br>............... | WR.NPT............<br>......D..... | ......<br>..... |
| iPS:43 6700 | 21-225_149C7 | VH3\|3-23/D7\|7-27\|RF1/JH6 | ............<br>............<br>........ | .......... | ........<br>...... | V...G...<br>.S......<br>.... | .................<br>............... | WR.NPT............<br>......D..... | ......<br>..... |
| iPS:43 6704 | 21-225_149C10 | VH3\|3-23/D7\|7-27\|RF1/JH6 | ............<br>........F...<br>........ | ......H... | ........<br>...... | V...G...<br>.S......<br>.... | .................<br>............... | WR.NPT............<br>......D..... | ......<br>..... |

| | | | | | | | | | |
|---|---|---|---|---|---|---|---|---|---|
| iPS:43 6710 | 21-225_150F6 | VH3\|3-23/D7\|7-27\|RF1/JH6 | ............<br>............<br>........ | .......... | ........<br>...... | V...G...<br>.S......<br>.... | .................<br>............... | WR.NPT...........<br>......D..... | ......<br>..... |
| iPS:43 6714 | 21-225_150H11 | VH3\|3-23/D7\|7-27\|RF1/JH6 | ............<br>............<br>........ | T......... | ........<br>...... | I...G...<br>.S......<br>.... | .................<br>............... | WR.NPT...........<br>......D..... | ......<br>..... |
| iPS:43 6718 | 21-225_151H5 | VH3\|3-23/D7\|7-27\|RF1/JH6 | ............<br>............<br>........ | .......... | ........<br>...... | V...G...<br>.S......<br>.... | .................<br>............... | WR.NPT...........<br>......D..... | ......<br>..... |
| iPS:43 6722 | 21-225_151H7 | VH3\|3-23/D7\|7-27\|RF1/JH6 | ............<br>............<br>........ | .......... | ........<br>...... | V...G...<br>.S......<br>.... | .................<br>............... | WR.NPT...........<br>......D..... | ......<br>..... |
| iPS:43 6724 | 21-225_151B9 | VH3\|3-23/D7\|7-27\|RF1/JH6 | ............<br>............<br>........ | .......... | ........<br>...... | V...G...<br>.S......<br>.... | .................<br>............... | WR.NPT...........<br>......D..... | ......<br>..... |
| iPS:43 6728 | 21-225_152G6 | VH3\|3-23/D7\|7-27\|RF1/JH6 | ............<br>............<br>........ | .......... | ........<br>...... | V...G...<br>.S......<br>.... | .................<br>............... | WR.NPT...........<br>......D..... | ......<br>..... |
| iPS:43 6730 | 21-225_152D7 | VH3\|3-23/D7\|7-27\|RF1/JH6 | ............<br>............<br>........ | .......... | ........<br>...... | V...G...<br>.S......<br>.... | .................<br>............... | WR.NPT...........<br>......DS.... | ......<br>..... |
| iPS:43 6742 | 21-225_154C4 | VH3\|3-23/D7\|7-27\|RF1/JH6 | ............<br>I...........<br>........ | .......... | ........<br>...... | V...G...<br>.S......<br>.... | .................<br>............... | WR.NPT...........<br>......D..... | ......<br>..... |
| iPS:43 6746 | 21-225_154E10 | VH3\|3-23/D7\|7-27\|RF1/JH6 | ............<br>............<br>........ | .......... | ........<br>...... | V...G...<br>.S......<br>.... | .................<br>............... | WR.NPT...........<br>......D..... | ......<br>..... |
| iPS:43 6758 | 21-225_155C10 | VH3\|3-23/D7\|7-27\|RF1/JH6 | ............<br>............<br>........ | .......... | ........<br>...... | V...G...<br>.S......<br>.... | .................<br>............... | WR.NPT...........<br>......D..... | ......<br>..... |
| iPS:43 6938 | 21-225_146A3 | VH3\|3-23/D7\|7-27\|RF1/JH6 | ............<br>............<br>........ | .......... | ........<br>...... | V...G...<br>.TT.....<br>.... | .................<br>............... | WR.NPT...........<br>......D..... | ......<br>..... |
| iPS:43 | 21- | VH3\|3-23/D7\|7- | ............<br>............ | .......... | ........<br>...... | V...G...<br>.S...... | .................<br>............... | WR.NPT...........<br>......D..... | ......<br>..... |

| | | | | | | | | | |
|---|---|---|---|---|---|---|---|---|---|
| iPS:43 7252 | 21-225_148H11 | VH3\|3-23/D7\|7-27\|RF1/JH6 | ............<br>............<br>........ | .......... | ........<br>...... | V...G...<br>.S......<br>.... | .................<br>............... | WR.NPT............<br>......D..... | ......<br>..... |
| iPS:43 7282 | 21-225_207C9 | VH3\|3-23/D7\|7-27\|RF1/JH6 | ............<br>............<br>........ | .......... | ........<br>...... | ........<br>........<br>.... | .................<br>............... | AG.TTGSY..........<br>.....Y.N.... | ......<br>..... |
| | | Germline | H_FR1 | H_CDR1 | H_FR2 | H_CDR2 | H_FR3 | H_CDR3 | H_FR4 |
| | VH3\|3-48/D4\|4-11\|RF2/JH6 | | EVQLVES-<br>GGGLVQPGGSLR<br>LSCAASG-FTFS | S-----YSMN | WVRQAPGK<br>GLEWVS | YISSS---<br>SSTIYYAD<br>SVKG | RFTISRDNAKNSLYLQM<br>NSLRDEDTAVYYCAR | DYSNYYY-----------<br>-----YYYGMDV | WGQGTT<br>VTVSS |
| iPS:43 6660 | 21-225_146D8 | VH3\|3-48/D4\|4-11\|RF2/JH6 | ............<br>............<br>........ | N......N.. | ........<br>...... | ...R....<br>.N.K..V.<br>.... | .................<br>............... | .R.GS.GY..........<br>....F....L.. | ......<br>..... |
| iPS:43 6682 | 21-225_146A8 | VH3\|3-48/D4\|4-11\|RF2/JH6 | ..K.........<br>....E......V<br>........ | N......N.. | ........<br>...... | ...R....<br>.N.K....<br>..R. | .................<br>............... | .R.GS.GY..........<br>....F....L.. | ......<br>..... |
| iPS:43 6684 | 21-225_146B6 | VH3\|3-48/D4\|4-11\|RF2/JH6 | ............<br>............<br>........ | .......N.. | ........<br>...... | ...R....<br>.N.KH...<br>.... | .................<br>D.............. | .R.GS.GY..........<br>....F....L.. | ......<br>..... |
| iPS:43 6696 | 21-225_149A1 | VH3\|3-48/D4\|4-11\|RF2/JH6 | ............<br>............<br>........ | .......N.. | ........<br>...... | ...R....<br>.N.KH...<br>.... | .................<br>............... | .R.GS.GY..........<br>....F....L.. | ......<br>..... |
| iPS:43 6712 | 21-225_150F9 | VH3\|3-48/D4\|4-11\|RF2/JH6 | .M..........<br>............<br>........ | .......N.. | ....V...<br>...... | ...R....<br>.N.KH...<br>.... | .................<br>............... | .R.GS.GY..........<br>....F....L.. | ......<br>..... |
| iPS:43 6762 | 21-225_156H2 | VH3\|3-48/D4\|4-11\|RF2/JH6 | ............<br>I...........<br>........ | N......N.. | ........<br>...... | ...R....<br>.N.K....<br>.... | .................<br>............... | .R.GS.GY..........<br>....F....... | ......<br>..... |
| iPS:43 6820 | 21-225_179D10 | VH3\|3-48/D4\|4-11\|RF2/JH6 | ............<br>........F...<br>........ | .......... | ........<br>.....A | ........<br>G..T....<br>..Q. | .................<br>...........R... | .SRKGF-<br>................-<br>...L.. | ....I.<br>..... |
| iPS:43 7262 | 21-225_170E4 | VH3\|3-48/D4\|4-11\|RF2/JH6 | ...........S<br>............<br>........ | .......... | ........<br>...... | ........<br>G..K....<br>..E. | .............D...<br>...........R... | .SRKGF-<br>................-<br>...L.. | ......<br>..... |
| | | Germline | H_FR1 | H_CDR1 | H_FR2 | H_CDR2 | H_FR3 | H_CDR3 | H_FR4 |

| | | | | | | | | | |
|---|---|---|---|---|---|---|---|---|---|
| | VH1\|1-08/D5\|5-12\|RF1/JH6 | | QVQLVQS- GAEVKKPGASVK VSCKASG-YIFT | S-----YDIN | WVRQATGQ GLEWMG | WMNPN--- SGNTGYAQ KFQG | RVTMTRNTSISTAYMEL SSLRSEDTAVYYCAR | VDIVATIY---------- ----YYYYGMDV | WGQGTT VTVSS |
| iPS:43 6662 | 21-225_147D7 | VH1\|1-08/D5\|5-12\|RF1/JH6 | ............ ............ ........ | .......... | ........ ...... | ........ ........ .... | ................. R.............. | A...LVPAAI........ ..PYN..FA... | ...... ..... |
| | | Germline | H_FR1 | H_CDR1 | H_FR2 | H_CDR2 | H_FR3 | H_CDR3 | H_FR4 |
| | VH1\|1-02/D3\|3-3\|RF2/JH6 | | QVQLVQS- GAEVKKPGASVK VSCKASG-YTFT | G-----YYMH | WVRQAPGQ GLEWMG | WINPN--- SGGTNYAQ KFQG | RVTMTRDTSISTAYMEL SRLRSDDTAVYYCAR | YYDFWSGYYT-------- ---YYYYYGMDV | WGQGTT VTVSS |
| iPS:43 6666 | 21-225_147B8 | VH1\|1-02/D3\|3-3\|RF2/JH6 | ............ ............ ........ | D.......L. | ........ ...... | ........ ..D..... .... | ................. ............... | DR.SG..S.P........ ............ | ...... ..... |
| | | Germline | H_FR1 | H_CDR1 | H_FR2 | H_CDR2 | H_FR3 | H_CDR3 | H_FR4 |
| | VH3\|3-33/D3\|3-3\|RF2/JH6 | | QVQLVES- GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | YYDFWSGYYT-------- ---YYYYYGMDV | WGQGTT VTVSS |
| iPS:43 6672 | 21-225_147F9 | VH3\|3-33/D3\|3-3\|RF2/JH6 | ............ ............ ........ | T......... | ........ ...... | ....G... ..D.D... .... | .......I......... ............... | DR.YC..GTC........ ..P......... | ...... ..... |
| iPS:43 6674 | 21-225_147G9 | VH3\|3-33/D3\|3-3\|RF2/JH6 | ............ ............ ........ | T......... | ........ ...... | ....G... ..D.D... .... | .......I......... ............... | DR.YC..GSC........ ..P......... | ...... ..... |
| iPS:43 6690 | 21-225_148A9 | VH3\|3-33/D3\|3-3\|RF2/JH6 | ............ ............ ........ | T......... | ........ ...... | ....G... ..D.D... .... | .......I......... ............... | DR.YC..GTC........ ..P......... | ...... ..... |
| iPS:43 6708 | 21-225_150D3 | VH3\|3-33/D3\|3-3\|RF2/JH6 | ............ ............ ........ | T......... | ........ ...... | ....G... ....D... .... | ................. ............... | DR.YC..GTC........ ..P......... | ...... ..... |
| iPS:43 6716 | 21-225_151F3 | VH3\|3-33/D3\|3-3\|RF2/JH6 | ............ ............ ........ | T......... | ........ ...... | ....G... ...TD... .... | .......I......... ............... | DR.YC..TSC........ ..P......... | ...... ..... |
| iPS:43 6738 | 21-225_153D9 | VH3\|3-33/D3\|3-3\|RF2/JH6 | ............ ............ ......V. | T......... | ........ ...... | ....G... ....D... .... | .......I......... ............... | DR.YC..GSC........ ..P......... | ...... ..... |
| iPS:43 | 21- | VH3\|3-33/D3\|3- | ............ ...........T | T......... | ........ ...... | .V..G... .N..D... | .......I......... ............... | DR.YC..GSC........ ..P........ | ...... ..... |

| | | | | | | | | | |
|---|---|---|---|---|---|---|---|---|---|
| iPS:43 6748 | 21-225_154D11 | VH3\|3-33/D3\|3-3\|RF2/JH6 | ............<br>............<br>.....NV. | T......... | ........<br>...... | ....G...<br>....D...<br>.... | .......S.........<br>............... | DR.YC..GSC........<br>..P......... | ......<br>..... |
| iPS:43 6764 | 21-225_158E9 | VH3\|3-33/D3\|3-3\|RF2/JH6 | ............<br>............<br>........ | .......... | ........<br>...... | ........<br>..S.....<br>.... | .................<br>............... | DRV.C..TSC........<br>..P......... | ......<br>..... |
| iPS:43 6774 | 21-225_161E10 | VH3\|3-33/D3\|3-3\|RF2/JH6 | ............<br>............<br>........ | .......... | ........<br>...... | ........<br>......V.<br>.... | .................<br>............... | DRV.C..TSC........<br>..P......... | ......<br>..... |
| iPS:43 6916 | 21-225_74A9 | VH3\|3-33/D3\|3-3\|RF2/JH6 | ............<br>............<br>........ | .......... | ........<br>...... | ........<br>.N..S...<br>.... | .......I.....F...<br>.....D......... | DR.YC..TSC........<br>..P......... | ......<br>..... |
| iPS:43 6940 | 21-225_146B8 | VH3\|3-33/D3\|3-3\|RF2/JH6 | ............<br>............<br>........ | T......... | ........<br>...... | .V..G...<br>.ND.DF..<br>..T. | .......I.........<br>............... | DR.YC..GSC........<br>..P......... | ......<br>..... |
| iPS:43 7258 | 21-225_153F9 | VH3\|3-33/D3\|3-3\|RF2/JH6 | ..H.........<br>............<br>......I. | T......... | ....G...<br>...... | ....G...<br>..DTD...<br>..R. | .......I.........<br>............... | DR.YC..GNC........<br>..P......... | ......<br>..... |
| | | Germline | **H_FR1** | **H_CDR1** | **H_FR2** | **H_CDR2** | **H_FR3** | **H_CDR3** | **H_FR4** |
| | VH4\|4-30.1/D7\|7-27\|RF3/JH5 | | QVQLQES-<br>GPGLVKPSQTLS<br>LTCTVSG-GSIS | SG---GYYWS | WIRQHPGK<br>GLEWIG | YIYY----<br>SGSTYYNP<br>SLKS | RVTISVDTSKNQFSLKL<br>SSVTAADTAVYYCAR | NWGN-------------<br>--------WFDP | WGQGTL<br>VTVSS |
| iPS:43 6680 | 21-225_147H12 | VH4\|4-30.1/D7\|7-27\|RF3/JH5 | ............<br>.N.........A<br>........ | .....Y.H.. | ........<br>...... | ........<br>........<br>.... | .................<br>T.............. | D..GYDS..........<br>......SG.... | ......<br>..... |
| iPS:43 6750 | 21-225_154G12 | VH4\|4-30.1/D7\|7-27\|RF3/JH5 | ............<br>.N.........G<br>........ | .....Y.... | ........<br>...... | ........<br>........<br>.... | ..S..L..P........<br>T.............. | D..GYDS..........<br>......SG.... | ......<br>..... |
| | | Germline | **H_FR1** | **H_CDR1** | **H_FR2** | **H_CDR2** | **H_FR3** | **H_CDR3** | **H_FR4** |
| | VH3\|3-07/D6\|6-13\|RF1/JH4 | | EVQLVES-<br>GGGLVQPGGSLR<br>LSCAASG-FTFS | S-----YWMS | WVRQAPGK<br>GLEWVA | NIKQD---<br>GSEKYYVD<br>SVKG | RFTISRDNAKNSLYLQM<br>NSLRAEDTAVYYCAR | GYSSSW-----------<br>-------YYFDY | WGQGTL<br>VTVSS |
| iPS:43 6698 | 21-225_149B5 | VH3\|3-07/D6\|6-13\|RF1/JH4 | ............<br>............<br>........ | G.......N | ........<br>...... | ........<br>........<br>.... | .................<br>............... | .MY..G...........<br>......W.V... | ......<br>..... |

| | | | | | | | | | |
|---|---|---|---|---|---|---|---|---|---|
| | VH3\|3-21/D6\|6-19\|RF2/JH5 | | EVQLVES- GGGLVKPGGSLR LSCAASG-FTFS | S-----YSMN | WVRQAPGK GLEWVS | SISSS--- SSYIYYAD SVKG | RFTISRDNAKNSLYLQM NSLRAEDTAVYYCAR | GIAVAG----------- --------NWFDP | WGQGTL VTVSS |
| iPS:436702 | 21-225_149E8 | VH3\|3-21/D6\|6-19\|RF2/JH5 | ............ ............ ........ | .......... | ...R.... ...... | A...T... G....... .... | ................. ............... | TAVAGT........... .......G.... | ...... ..... |
| | | Germline | H_FR1 | H_CDR1 | H_FR2 | H_CDR2 | H_FR3 | H_CDR3 | H_FR4 |
| | VH5\|5-51/D3\|3-22\|RF2/JH6 | | EVQLVQS- GAEVKKPGESLK ISCKGSG-YSFT | S-----YWIG | WVRQMPGK GLEWMG | IIYPG--- DSDTRYSP SFQG | QVTISADKSISTAYLQW SSLKASDTAMYYCAR | YYYDSSGYYY-------- ---YYYYYGMDV | WGQGTT VTVSS |
| iPS:436752 | 21-225_155H1 | VH5\|5-51/D3\|3-22\|RF2/JH6 | ............ ............ ........ | .......... | ........ ...... | L....... A....... .... | ................. ............... | QAIA.R.R-- ..........- ........ | ...... ..... |
| | | Germline | H_FR1 | H_CDR1 | H_FR2 | H_CDR2 | H_FR3 | H_CDR3 | H_FR4 |
| | VH3\|3-33/D6\|6-19\|RF1/JH4 | | QVQLVES- GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | GYSSGW----------- --------YYFDY | WGQGTL VTVSS |
| iPS:436772 | 21-225_161H3 | VH3\|3-33/D6\|6-19\|RF1/JH4 | ............ ............ ........ | .......... | ........ ...... | ........ ........ .... | ................. ............... | VGY..G........... ......W.I... | ...... ..... |
| | | Germline | H_FR1 | H_CDR1 | H_FR2 | H_CDR2 | H_FR3 | H_CDR3 | H_FR4 |
| | VH4\|4-30.1/D2\|2-2\|RF2/JH6 | | QVQLQES- GPGLVKPSQTLS LTCTVSG-GSIS | SG---GYYWS | WIRQHPGK GLEWIG | YIYY---- SGSTYYNP SLKS | RVTISVDTSKNQFSLKL SSVTAADTAVYYCAR | GYCSSTSCYT-------- ---YYYYYGMDV | WGQGTT VTVSS |
| iPS:436776 | 21-225_161F12 | VH4\|4-30.1/D2\|2-2\|RF2/JH6 | ............ ............ ........ | .......... | ........ ...... | ........ ...P.... .... | .I...I.......... N............. | SN...AN.......... ..VGF....L.. | ..R... ..... |
| iPS:436780 | 21-225_165H3 | VH4\|4-30.1/D2\|2-2\|RF2/JH6 | ............ ............ ........ | .......... | ........ ...... | ........ ...P.... .... | .I...I.......... N............. | SN...AN.......... ..VGF....... | ...... ..... |
| | | Germline | H_FR1 | H_CDR1 | H_FR2 | H_CDR2 | H_FR3 | H_CDR3 | H_FR4 |
| | VH3\|3-33/D5\|5-24\|RF3/JH6 | | QVQLVES- GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | RDGYNYYY---------- -----YYYGMDV | WGQGTT VTVSS |
| iPS:436784 | 21-225_169C1 | VH3\|3-33/D5\|5-24\|RF3/JH6 | ............ ............ ......L. | ......N... | ........ ...... | ........ ........ .... | .......T........ ............... | DQYNRNDGPP........ ..AYY....L.. | ...... ..... |

| | | | | | | | | | |
|---|---|---|---|---|---|---|---|---|---|
| iPS:43 6786 | 21-225_169A6 | VH3\|3-33/D5\|5-24\|RF3/JH6 | ............<br>............<br>......L. | .......N... | ........<br>...... | ........<br>........<br>.... | ..............S...<br>................ | DQYNRNDGPP........<br>..AYY....... | ......<br>..... |
| iPS:43 6796 | 21-225_170A5 | VH3\|3-33/D5\|5-24\|RF3/JH6 | ............<br>............<br>....L... | N.....C... | ........<br>...... | I.......<br>........<br>.... | ..............D...<br>................ | DQYNRNDGPP........<br>..AYY....L.. | ......<br>..... |
| iPS:43 6812 | 21-225_175C6 | VH3\|3-33/D5\|5-24\|RF3/JH6 | ............<br>............<br>....L... | N.....C... | ........<br>...... | I.......<br>........<br>.... | ...V........D...<br>................ | DQYNRNDGPP........<br>..AYY....L.. | ......<br>..... |
| | | Germline | H_FR1 | H_CDR1 | H_FR2 | H_CDR2 | H_FR3 | H_CDR3 | H_FR4 |
| | VH3\|3-48/D5\|5-18\|RF1/JH6 | | EVQLVES-<br>GGGLVQPGGSLR<br>LSCAASG-FTFS | S-----YSMN | WVRQAPGK<br>GLEWVS | YISSS---<br>SSTIYYAD<br>SVKG | RFTISRDNAKNSLYLQM<br>NSLRDEDTAVYYCAR | VDTAMVYY----------<br>-----YYYGMDV | WGQGTT<br>VTVSS |
| iPS:43 6788 | 21-225_169B7 | VH3\|3-48/D5\|5-18\|RF1/JH6 | ............<br>............<br>........ | ........L. | ........<br>...... | ..G.....<br>G.I.F...<br>.... | .................<br>................ | G...G.T-<br>...................<br>.... | ......<br>..... |
| iPS:43 6798 | 21-225_171F5 | VH3\|3-48/D5\|5-18\|RF1/JH6 | ............<br>............<br>........ | ........L. | ........<br>...... | ..G.....<br>G.I.F...<br>.... | .................<br>................ | G...G.T-<br>...................<br>.... | ......<br>..... |
| iPS:43 6864 | 21-225_58G11 | VH3\|3-48/D5\|5-18\|RF1/JH6 | ............<br>............<br>........ | .......... | ........<br>....I. | ...T....<br>....F...<br>.... | .......S.........<br>................ | G.....L-<br>...................<br>.... | ......<br>..... |
| iPS:43 6866 | 21-225_59F2 | VH3\|3-48/D5\|5-18\|RF1/JH6 | ............<br>...........G<br>........ | .......... | ........<br>...... | ...G....<br>.NI...T.<br>.... | .................<br>................ | A..P..L-<br>.................F.<br>.... | ......<br>..... |
| iPS:43 6872 | 21-225_60D2 | VH3\|3-48/D5\|5-18\|RF1/JH6 | ............<br>...........G<br>........ | .......... | ........<br>...... | ...E....<br>.NI...T.<br>.... | .........M.......<br>................ | A..P..L-<br>.................F.<br>.... | ......<br>..... |
| | | Germline | H_FR1 | H_CDR1 | H_FR2 | H_CDR2 | H_FR3 | H_CDR3 | H_FR4 |
| | VH3\|3-33/D5\|5-24\|RF1/JH6 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | VEMATIYY----------<br>-----YYYGMDV | WGQGTT<br>VTVSS |
| iPS:43 6790 | 21-225_169G11 | VH3\|3-33/D5\|5-24\|RF1/JH6 | ............<br>............<br>........ | .......... | ........<br>...... | I.......<br>........<br>.... | .................<br>........G...... | EGATYYHGSGS.......<br>.YYPATN..... | ......<br>..... |
| | | Germline | H_FR1 | H_CDR1 | H_FR2 | H_CDR2 | H_FR3 | H_CDR3 | H_FR4 |

| | | | | | | | | | |
|---|---|---|---|---|---|---|---|---|---|
| | VH3\|3-33/D3\|3-10\|RF1/JH6 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | VLLWFGELL*--------<br>---YYYYYGMDV | WGQGTT<br>VTVSS |
| iPS:43 6792 | 21-225_169D12 | VH3\|3-33/D3\|3-10\|RF1/JH6 | ............<br>............<br>........ | .......... | ........<br>...... | ........<br>........<br>.... | .................<br>..............S | P.YDM.L---<br>...........---<br>..D... | ......<br>..... |
| | | Germline | H_FR1 | H_CDR1 | H_FR2 | H_CDR2 | H_FR3 | H_CDR3 | H_FR4 |
| | VH1\|1-46/D7\|7-27\|RF3/JH4 | | QVQLVQS-<br>GAEVKKPGASVK<br>VSCKASG-YTFT | S-----YYMH | WVRQAPGQ<br>GLEWMG | IINPS---<br>GGSTSYAQ<br>KFQG | RVTMTRDTSTSTVYMEL<br>SSLRSEDTAVYYCAR | NWGY------------<br>---------FDY | WGQGTL<br>VTVSS |
| iPS:43 6800 | 21-225_171D12 | VH1\|1-46/D7\|7-27\|RF3/JH4 | ............<br>............<br>.......N | .........Y | ........<br>...... | ........<br>....N...<br>.... | ...........L....<br>N............S | G.E-<br>..................<br>.....LN. | ......<br>..... |
| iPS:43 6804 | 21-225_172C3 | VH1\|1-46/D7\|7-27\|RF3/JH4 | ............<br>............<br>.......R | .........Y | ........<br>....V. | T.......<br>....N...<br>.... | ...........L....<br>N............S | G.E-<br>..................<br>.....LN. | ......<br>..... |
| iPS:43 6806 | 21-225_172B12 | VH1\|1-46/D7\|7-27\|RF3/JH4 | ............<br>.......T....<br>.......R | .........Y | ........<br>....V. | T.......<br>....D...<br>.... | ...........L....<br>N............S | G.E-<br>..................<br>.....LN. | ......<br>..... |
| | | Germline | H_FR1 | H_CDR1 | H_FR2 | H_CDR2 | H_FR3 | H_CDR3 | H_FR4 |
| | VH3\|3-30.3/D4\|4-23\|RF1/JH6 | | QVQLVES-<br>GGGVVQPGRSLR<br>LSCAASG-FTFS | S-----YAMH | WVRQAPGK<br>GLEWVA | VISYD---<br>GSNKYYAD<br>SVKG | RFTISRDNSKNTLYLQM<br>NSLRAEDTAVYYCAR | *LRW*LYY----------<br>-----YYYGMDV | WGQGTT<br>VTVSS |
| iPS:43 6808 | 21-225_173F8 | VH3\|3-30.3/D4\|4-23\|RF1/JH6 | ............<br>............<br>........ | .......G.. | ........<br>...... | ........<br>..P..C..<br>.... | .............F...<br>............... | DE.QW.P-<br>...............AP.<br>.... | ......<br>..... |
| | | Germline | H_FR1 | H_CDR1 | H_FR2 | H_CDR2 | H_FR3 | H_CDR3 | H_FR4 |
| | VH6\|6-01/D6\|6-6\|RF2/JH6 | | QVQLQQS-<br>GPGLVKPSQTLS<br>LTCAISG-DSVS | SN---SAAWN | WIRQSPSR<br>GLEWLG | RTYYR--<br>SKWYNDYA<br>VSVKS | RITINPDTSKNQFSLQL<br>NSVTPEDTAVYYCAR | SIAARYY-----------<br>-----YYYGMDV | WGQGTT<br>VTVSS |
| iPS:43 6810 | 21-225_175F4 | VH6\|6-01/D6\|6-6\|RF2/JH6 | ............<br>............<br>........ | .......... | ........<br>...... | ........<br>....A.P.<br>.ME. | ..S.............<br>............... | DK..GRND.........<br>....F....... | ......<br>..... |
| iPS:43 6814 | 21-225_178H10 | VH6\|6-01/D6\|6-6\|RF2/JH6 | ............<br>............<br>........ | .......... | ........<br>...... | ........<br>...SA.P.<br>.ME. | .VS.............<br>............... | DK..GRND.........<br>....F....... | ......<br>..... |
| | | Germline | H_FR1 | H_CDR1 | H_FR2 | H_CDR2 | H_FR3 | H_CDR3 | H_FR4 |

| | VH3\|3-33/D1\|1-1\|RF3/JH6 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | YNWNDYY----------<br>------YYYGMDV | WGQGTT<br>VTVSS |
|---|---|---|---|---|---|---|---|---|---|
| iPS:436818 | 21-225_179C7 | VH3\|3-33/D1\|1-1\|RF3/JH6 | .A..........<br>............<br>........ | N.....S... | ....G...<br>...... | I.Y.....<br>..Y..N..<br>.... | .................<br>............... | DRHY.FHVP.........<br>...YY....... | ......<br>..... |
| iPS:437094 | 21-225_210D12 | VH3\|3-33/D1\|1-1\|RF3/JH6 | ............<br>............<br>........ | H......... | ........<br>...... | ........<br>........<br>.... | .................<br>............... | GD..P--<br>................--<br>E.L.. | ......<br>..... |
| iPS:437096 | 21-225_210E12 | VH3\|3-33/D1\|1-1\|RF3/JH6 | ..H.........<br>............<br>........ | N......... | ........<br>...... | ........<br>........<br>.... | .................<br>............... | GD..P--<br>................--<br>E.... | ......<br>.I... |
| iPS:437098 | 21-225_211C1 | VH3\|3-33/D1\|1-1\|RF3/JH6 | ............<br>............<br>........ | H......... | ........<br>...... | ........<br>........<br>.... | .................<br>.T.......I..... | GD..P--<br>................--<br>E.L.. | ......<br>..... |
| iPS:437104 | 21-225_211G5 | VH3\|3-33/D1\|1-1\|RF3/JH6 | ............<br>............<br>........ | H......... | ........<br>...... | ........<br>........<br>.... | .................<br>............... | GD..P--<br>................--<br>E.L.. | ......<br>..... |
| iPS:437112 | 21-225_212C2 | VH3\|3-33/D1\|1-1\|RF3/JH6 | ............<br>............<br>........ | H......... | ........<br>...... | ........<br>........<br>.... | .................<br>............... | GD..P--<br>................--<br>E.... | .....S<br>..... |
| iPS:437114 | 21-225_212A4 | VH3\|3-33/D1\|1-1\|RF3/JH6 | ..H.........<br>..A.........<br>........ | N......... | ........<br>...... | ........<br>........<br>.... | .................<br>............... | GD..P--<br>................--<br>E.... | ......<br>.I... |
| iPS:437116 | 21-225_212F6 | VH3\|3-33/D1\|1-1\|RF3/JH6 | ............<br>............<br>........ | H......... | ........<br>...... | ........<br>........<br>.... | .................<br>............... | GD..P--<br>................--<br>E.L.. | ......<br>..... |
| iPS:437118 | 21-225_212G7 | VH3\|3-33/D1\|1-1\|RF3/JH6 | ............<br>............<br>........ | H......... | ........<br>...... | ........<br>.....C..<br>.... | .........T.......<br>............... | GD..P--<br>................--<br>E.L.. | ......<br>..... |
| iPS:437128 | 21-225_213G3 | VH3\|3-33/D1\|1-1\|RF3/JH6 | ............<br>............<br>........ | H......... | ........<br>...... | ........<br>........<br>.... | .............H...<br>............... | GD..P--<br>................--<br>E.... | .....S<br>..... |
| iPS:437130 | 21-225_213D5 | VH3\|3-33/D1\|1-1\|RF3/JH | ............<br>............ | H......... | ........<br>...... | ........<br>........ | .............F...<br>....V.......... | GD..P--<br>................--<br>E | ......<br>..... |

| | | | | | | | | | |
|---|---|---|---|---|---|---|---|---|---|
| iPS:437146 | 21-225_215D3 | VH3\|3-33/D1\|1-1\|RF3/JH6 | .T..........<br>............<br>........ | H......... | ........<br>...... | ........<br>...E....<br>.... | .................<br>............... | GD..P--<br>................--<br>E.... | ......<br>..... |
| iPS:437150 | 21-225_216A3 | VH3\|3-33/D1\|1-1\|RF3/JH6 | ............<br>............<br>........ | H......... | ........<br>...... | ........<br>........<br>.... | .................<br>............... | GD..P--<br>................--<br>E.L.. | ......<br>..... |
| iPS:437162 | 21-225_217B2 | VH3\|3-33/D1\|1-1\|RF3/JH6 | ..H.........<br>............<br>........ | N......... | ........<br>...... | ........<br>........<br>.... | .................<br>............... | GD..P--<br>................--<br>E.... | ......<br>.I... |
| iPS:437172 | 21-225_219A7 | VH3\|3-33/D1\|1-1\|RF3/JH6 | ............<br>...........P<br>........ | H......... | ........<br>...... | ........<br>........<br>.... | ...........M.....<br>............... | GD..P--<br>................--<br>E.... | ......<br>..... |
| iPS:437182 | 21-225_221H2 | VH3\|3-33/D1\|1-1\|RF3/JH6 | ............<br>............<br>........ | H......... | ........<br>...... | ........<br>........<br>.... | .............H...<br>............... | GD..P--<br>................--<br>E.... | ......<br>..... |
| iPS:437184 | 21-225_221G4 | VH3\|3-33/D1\|1-1\|RF3/JH6 | ............<br>............<br>........ | H......... | ........<br>...... | ........<br>........<br>.... | .............H...<br>............... | GD..P--<br>................--<br>E.... | .....S<br>..... |
| iPS:438664 | 21-225_216G1 | VH3\|3-33/D1\|1-1\|RF3/JH6 | ..H.........<br>............<br>........ | N......... | ........<br>...... | ........<br>........<br>.... | .................<br>............... | GD..P--<br>................--<br>E.... | ......<br>.I... |
| | | Germline | **H_FR1** | **H_CDR1** | **H_FR2** | **H_CDR2** | **H_FR3** | **H_CDR3** | **H_FR4** |
| | VH3\|3-33/D1\|1-1\|RF1/JH4 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | GTTGT-------------<br>---------YFDY | WGQGTL<br>VTVSS |
| iPS:436822 | 21-225_180D4 | VH3\|3-33/D1\|1-1\|RF1/JH4 | ............<br>............<br>........ | N.....F... | ........<br>...... | I.......<br>..D.....<br>.... | .................<br>.T........F.... | .GPPFST...........<br>.....VTM.... | ......<br>..... |
| iPS:436828 | 21-225_181H1 | VH3\|3-33/D1\|1-1\|RF1/JH4 | ............<br>............<br>........ | N......... | ........<br>...... | I.......<br>..D.....<br>.... | .I...............<br>.T........F.... | .GPPFST...........<br>.....VTM.... | ......<br>..... |
| iPS:436950 | 21-225_184G4 | VH3\|3-33/D1\|1-1\|RF1/JH4 | ............<br>............<br>V....... | .......... | ........<br>...... | I.......<br>........<br>.... | .................<br>............... | .GPPFST...........<br>.....VTM.... | ......<br>..... |

| | | | | | | | | | |
|---|---|---|---|---|---|---|---|---|---|
| iPS:436952 | 21-225_185D2 | VH3\|3-33/D1\|1-1\|RF1/JH4 | ................................ | N......... | .............. | I.........D......... | ..................T.........F... | .GPPFST...........VTM.... | ........... |
| | | Germline | H_FR1 | H_CDR1 | H_FR2 | H_CDR2 | H_FR3 | H_CDR3 | H_FR4 |
| | VH2\|2-05/D6\|6-6\|RF2/JH3 | | QITLKES-GPTLVKPTQTLTLTCTFSG-FSLS | TS---GVGVG | WIRQPPGKALEWLA | LIYW----NDDKRYSPSLKS | RLTITKDTSKNQVVLTMTNMDPVDTATYYCAH | SIAAR-------------------DAFDI | WGQGTMVTVSS |
| iPS:436824 | 21-225_180C5 | VH2\|2-05/D6\|6-6\|RF2/JH3 | ................................ | .......... | .............G | F.S................. | S..............I................ | KA..V.................-.... | ........... |
| iPS:436956 | 21-225_186H6 | VH2\|2-05/D6\|6-6\|RF2/JH3 | ................................ | .......... | .............G | F.S................. | S............................... | KA..V.................-.... | ........... |
| | | Germline | H_FR1 | H_CDR1 | H_FR2 | H_CDR2 | H_FR3 | H_CDR3 | H_FR4 |
| | VH1\|1-08/D5\|5-18\|RF3/JH6 | | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | GYSYGYYY-----------YYYGMDV | WGQGTTVTVSS |
| iPS:436826 | 21-225_180G5 | VH1\|1-08/D5\|5-18\|RF3/JH6 | ................................ | .......... | .............. | .................... | ................................ | .FY.YGSGSHV.........PYH....L.. | ........... |
| | | Germline | H_FR1 | H_CDR1 | H_FR2 | H_CDR2 | H_FR3 | H_CDR3 | H_FR4 |
| | VH6\|6-01/D5\|5-24\|RF3/JH2 | | QVQLQQS-GPGLVKPSQTLSLTCAISG-DSVS | SN---SAAWN | WIRQSPSRGLEWLG | RTYYR---SKWYNDYAVSVKS | RITINPDTSKNQFSLQLNSVTPEDTAVYYCAR | RDGYNY-------------YWYFDL | WGRGTLVTVSS |
| iPS:436832 | 21-225_51D8 | VH6\|6-01/D5\|5-24\|RF3/JH2 | ................................ | .......... | .............. | .................... | ...F..........R................. | DRYNWNY................P...... | ........... |
| | | Germline | H_FR1 | H_CDR1 | H_FR2 | H_CDR2 | H_FR3 | H_CDR3 | H_FR4 |
| | VH1\|1-02/D5\|5-24\|RF3/JH5 | | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | RDGYNY-------------NWFDP | WGQGTLVTVSS |
| iPS:436840 | 21-225_53E9 | VH1\|1-02/D5\|5-24\|RF3/JH5 | ........................N... | .......... | .............. | ..I.......D......... | ................................ | DGYSSGW..................F..... | ........... |
| | | Germline | H_FR1 | H_CDR1 | H_FR2 | H_CDR2 | H_FR3 | H_CDR3 | H_FR4 |
| | VH2\|2-05/D7\|7-27\|RF1/JH4 | | QITLKES-GPTLVKPTQTLTLTCTFSG-FSLS | TS---GVGVG | WIRQPPGKALEWLA | LIYW----NDDKRYSPSLKS | RLTITKDTSKNQVVLTMTNMDPVDTATYYCAH | LTGY---------------------FDY | WGQGTLVTVSS |

| | | | | | | | | | |
|---|---|---|---|---|---|---|---|---|---|
| iPS:43 6848 | 21-225_57F1 | VH2\|2-05/D7\|7-27\|RF1/JH4 | .............<br>............<br>........ | .......... | ........<br>...... | ........<br>HE......<br>.... | .....E.......D...<br>...A.......... | V..I...............<br>........AAP. | ......<br>..... |
| iPS:43 6852 | 21-225_57H11 | VH2\|2-05/D7\|7-27\|RF1/JH4 | .............<br>............<br>.......T | .......... | ........<br>...... | ........<br>HE.R....<br>.... | .....E.......D...<br>...A.......... | V..I...............<br>........AAP. | ......<br>..... |
| iPS:43 6870 | 21-225_60B1 | VH2\|2-05/D7\|7-27\|RF1/JH4 | .............<br>............<br>........ | .......... | ........<br>...... | ........<br>HE......<br>.... | .....E.......D...<br>...A.......... | V.YI...............<br>........AAP. | ......<br>..... |
| iPS:43 6876 | 21-225_61F5 | VH2\|2-05/D7\|7-27\|RF1/JH4 | .............<br>............<br>........ | ......L... | ........<br>...... | ...S....<br>HE......<br>.... | .....E.......D...<br>...A.......... | V..I...............<br>........AAP. | ......<br>..... |
| iPS:39 2593 | 21-225_3E10 | VH2\|2-05/D7\|7-27\|RF1/JH4 | .............<br>............<br>.......N | .G........ | ........<br>...... | ........<br>.....H..<br>.... | .................<br>.H.A.......... | .IEV...............<br>........A... | ......<br>..... |
| | | Germline | **H_FR1** | **H_CDR1** | **H_FR2** | **H_CDR2** | **H_FR3** | **H_CDR3** | **H_FR4** |
| | VH2\|2-05/D6\|6-6\|RF2/JH1 | | QITLKES-<br>GPTLVKPTQTLT<br>LTCTFSG-FSLS | TS---GVGVG | WIRQPPGK<br>ALEWLA | LIYW----<br>NDDKRYSP<br>SLKS | RLTITKDTSKNQVVLTM<br>TNMDPVDTATYYCAH | SIAARA-----------<br>-------EYFQH | WGQGTL<br>VTVSS |
| iPS:43 6854 | 21-225_58C1 | VH2\|2-05/D6\|6-6\|RF2/JH1 | .............<br>............<br>........ | .......... | ........<br>...... | ........<br>D.......<br>.... | .....E...........<br>.............. | L..V--<br>...................<br>.-A.DS | ......<br>..... |
| iPS:43 6874 | 21-225_60A12 | VH2\|2-05/D6\|6-6\|RF2/JH1 | .............<br>............<br>........ | .......... | ........<br>...... | ........<br>D.......<br>.... | .....E...........<br>.............. | L..V--<br>...................<br>.-A.DS | ......<br>..... |
| iPS:43 6954 | 21-225_185G7 | VH2\|2-05/D6\|6-6\|RF2/JH1 | .............<br>............<br>.......T | .G........ | ........<br>...... | ........<br>...E....<br>.... | .................<br>.............. | I..V--<br>...................<br>.-A... | ......<br>..... |
| iPS:39 3188 | 21-225_34B9 | VH2\|2-05/D6\|6-6\|RF2/JH1 | ....R.......<br>............<br>........ | .......... | ........<br>...... | ........<br>........<br>.... | .................<br>.............. | L..V--<br>...................<br>.-T.DS | ....S.<br>..... |
| | | Germline | **H_FR1** | **H_CDR1** | **H_FR2** | **H_CDR2** | **H_FR3** | **H_CDR3** | **H_FR4** |
| | VH3\|3-30.3/D3\|3-10\|RF2/JH6 | | QVQLVES-<br>GGGVVQPGRSLR<br>LSCAASG-FTFS | S-----YAMH | WVRQAPGK<br>GLEWVA | VISYD---<br>GSNKYYAD<br>SVKG | RFTISRDNSKNTLYLQM<br>NSLRAEDTAVYYCAR | YYYGSGSYYN-------<br>---YYYYYGMDV | WGQGTT<br>VTVSS |

| | | | | | | | | | |
|---|---|---|---|---|---|---|---|---|---|
| iPS:43 6858 | 21-225_58E7 | VH3\|3-30.3/D3\|3-10\|RF2/JH6 | ............<br>............<br>........ | F......G.. | ........<br>...... | .T......<br>..D.....<br>.... | ..S..............<br>S........M..... | DD.....P--<br>...........--<br>L...... | ......<br>..... |
| | | Germline | H_FR1 | H_CDR1 | H_FR2 | H_CDR2 | H_FR3 | H_CDR3 | H_FR4 |
| | VH3\|3-07/D5\|5-12\|RF3/JH6 | | EVQLVES-<br>GGGLVQPGGSLR<br>LSCAASG-FTFS | S-----YWMS | WVRQAPGK<br>GLEWVA | NIKQD---<br>GSEKYYVD<br>SVKG | RFTISRDNAKNSLYLQM<br>NSLRAEDTAVYYCAR | GYSGYDYY----------<br>----YYYYGMDV | WGQGTT<br>VTVSS |
| iPS:43 6860 | 21-225_58F7 | VH3\|3-07/D5\|5-12\|RF3/JH6 | ............<br>............<br>........ | ......F... | ........<br>...... | H.......<br>........<br>.... | .................<br>............... | .DLP.SSG..........<br>....-....... | ......<br>..... |
| | | Germline | H_FR1 | H_CDR1 | H_FR2 | H_CDR2 | H_FR3 | H_CDR3 | H_FR4 |
| | VH3\|3-30.3/D4\|4-17\|RF2/JH6 | | QVQLVES-<br>GGGVVQPGRSLR<br>LSCAASG-FTFS | S-----YAMH | WVRQAPGK<br>GLEWVA | VISYD---<br>GSNKYYAD<br>SVKG | RFTISRDNSKNTLYLQM<br>NSLRAEDTAVYYCAR | DYGDYYY-----------<br>-----YYYGMDV | WGQGTT<br>VTVSS |
| iPS:43 6862 | 21-225_58F8 | VH3\|3-30.3/D4\|4-17\|RF2/JH6 | ............<br>............<br>........ | .......G.. | ........<br>...... | ........<br>........<br>.... | .................<br>............... | .E.RG.GGYER.......<br>.GYYY....... | ......<br>..... |
| | | Germline | H_FR1 | H_CDR1 | H_FR2 | H_CDR2 | H_FR3 | H_CDR3 | H_FR4 |
| | VH3\|3-33/D3\|3-16\|RF2/JH6 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | YYDYVWGSYRY-------<br>--TYYYYYGMDV | WGQGTT<br>VTVSS |
| iPS:43 6868 | 21-225_59B11 | VH3\|3-33/D3\|3-16\|RF2/JH6 | ............<br>............<br>........ | ........V. | ........<br>...... | A.......<br>........<br>.... | ...............L.<br>............... | DR..CSS.SC-<br>.........P........<br>. | ......<br>..... |
| | | Germline | H_FR1 | H_CDR1 | H_FR2 | H_CDR2 | H_FR3 | H_CDR3 | H_FR4 |
| | VH2\|2-05/D1\|1-1\|RF1/JH3 | | QITLKES-<br>GPTLVKPTQTLT<br>LTCTFSG-FSLS | TS---GVGVG | WIRQPPGK<br>ALEWLA | LIYW----<br>NDDKRYSP<br>SLKS | RLTITKDTSKNQVVLTM<br>TNMDPVDTATYYCAH | GTTGT------------<br>-------DAFDI | WGQGTM<br>VTVSS |
| iPS:43 6878 | 21-225_62E3 | VH2\|2-05/D1\|1-1\|RF1/JH3 | ............<br>............<br>........ | .......... | ........<br>...... | ..N.....<br>........<br>.... | .F...R....D......<br>............... | KA.WV.............<br>.......-.... | ......<br>..... |
| iPS:43 6880 | 21-225_62E8 | VH2\|2-05/D1\|1-1\|RF1/JH3 | ............<br>............<br>........ | .......... | ........<br>...... | ..N.....<br>........<br>.... | .F...R....D......<br>............... | KA.WV.............<br>.......-.... | ......<br>..... |
| iPS:43 6882 | 21-225_62D10 | VH2\|2-05/D1\|1-1\|RF1/JH | ............<br>..S......... | .......... | ........<br>...... | ..N.....<br>........ | .F...R....D......<br>............... | KA.WV.............<br>.......-.... | ......<br>..... |

| | | | | | | | | | |
|---|---|---|---|---|---|---|---|---|---|
| iPS:436884 | 21-225_62A12 | VH2\|2-05/D1\|1-1\|RF1/JH3 | ............<br>............<br>........ | .......... | ........<br>...... | ..N.....<br>........<br>.... | .F...R....D......<br>.....L......... | K..WV............<br>.......-.... | ......<br>..... |
| iPS:436886 | 21-225_62B12 | VH2\|2-05/D1\|1-1\|RF1/JH3 | ............<br>............<br>.......N | .......... | ........<br>...... | ..N.....<br>........<br>.... | .F...R....D......<br>............... | KA.WV............<br>.......-.... | ......<br>..... |
| iPS:436894 | 21-225_66G9 | VH2\|2-05/D1\|1-1\|RF1/JH3 | ............<br>............<br>........ | .......... | ........<br>...... | ..N.....<br>.....F..<br>.... | .F...R....D......<br>............... | KA.WV............<br>.......-.... | ......<br>..... |
| iPS:436908 | 21-225_72D5 | VH2\|2-05/D1\|1-1\|RF1/JH3 | ............<br>............<br>........ | .......... | ........<br>...... | ..N.....<br>........<br>.... | .F...R....D...F..<br>........G...... | KA.WV............<br>.......-.... | ......<br>..... |
| iPS:436912 | 21-225_73C4 | VH2\|2-05/D1\|1-1\|RF1/JH3 | ............<br>............<br>........ | .......... | ........<br>...... | ..N.....<br>........<br>.... | .F...R....D......<br>............... | K..WV............<br>.......-.... | ......<br>..... |
| | | Germline | H_FR1 | H_CDR1 | H_FR2 | H_CDR2 | H_FR3 | H_CDR3 | H_FR4 |
| | VH3\|3-48/D4\|4-11\|RF2/JH3 | | EVQLVES-<br>GGGLVQPGGSLR<br>LSCAASG-FTFS | S-----YSMN | WVRQAPGK<br>GLEWVS | YISSS---<br>SSTIYYAD<br>SVKG | RFTISRDNAKNSLYLQM<br>NSLRDEDTAVYYCAR | DYSNY-----------<br>-------DAFDI | WGQGTM<br>VTVSS |
| iPS:436888 | 21-225_63G7 | VH3\|3-48/D4\|4-11\|RF2/JH3 | ............<br>............<br>........ | .......... | ........<br>...... | ........<br>T......A<br>.... | .................<br>............... | .HRY.DSSG........<br>....YYS..... | ......<br>..... |
| iPS:436890 | 21-225_63A10 | VH3\|3-48/D4\|4-11\|RF2/JH3 | ............<br>............<br>........ | .......... | ........<br>...... | ........<br>T......A<br>.... | .................<br>............... | .HRY.DSSG........<br>....YYS..... | ......<br>..... |
| | | Germline | H_FR1 | H_CDR1 | H_FR2 | H_CDR2 | H_FR3 | H_CDR3 | H_FR4 |
| | VH1\|1-02/D3\|3-22\|RF2/JH3 | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | YYYDSSGY---------<br>-----YYDAFDI | WGQGTM<br>VTVSS |
| iPS:436892 | 21-225_65E9 | VH1\|1-02/D3\|3-22\|RF2/JH3 | ............<br>............<br>........ | .......... | ........<br>...... | ........<br>........<br>.... | .................<br>............... | A..YG..S.........<br>.......NE..M | ......<br>..... |
| iPS:436900 | 21-225_69B9 | VH1\|1-02/D3\|3-22\|RF2/JH3 | ...M.....D..<br>............<br>........ | .......H.. | ........<br>...... | ........<br>........<br>.... | .................<br>..M............ | A..YG..S.........<br>.......NES.M | ......<br>..... |
| | | Germline | H_FR1 | H_CDR1 | H_FR2 | H_CDR2 | H_FR3 | H_CDR3 | H_FR4 |

| | | | | | | | | | |
|---|---|---|---|---|---|---|---|---|---|
| | VH6\|6-01/D4\|4-17\|RF2/JH6 | | QVQLQQS-<br>GPGLVKPSQTLS<br>LTCAISG-DSVS | SN---SAAWN | WIRQSPSR<br>GLEWLG | RIYYR--<br>SKWYNDYA<br>VSVKS | RITINPDTSKNQFSLQL<br>NSVTPEDTAVYYCAR | DYGDYYY-----------<br>------YYYGMDV | WGQGTT<br>VTVSS |
| iPS:436898 | 21-225_68D8 | VH6\|6-01/D4\|4-17\|RF2/JH6 | ............<br>............<br>........ | .......... | ........<br>...... | ........<br>EC......<br>..Q. | ..............H.<br>...........F... | .R.HRG-<br>................-<br>F..... | ......<br>..... |
| | | Germline | H_FR1 | H_CDR1 | H_FR2 | H_CDR2 | H_FR3 | H_CDR3 | H_FR4 |
| | VH3\|3-33/D7\|7-27\|RF1/JH3 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | LTGD-------------<br>---------AFDI | WGQGTM<br>VTVSS |
| iPS:436910 | 21-225_73G1 | VH3\|3-33/D7\|7-27\|RF1/JH3 | ............<br>............<br>GT...... | Y......... | ........<br>...... | .TT.....<br>........<br>.... | ...S............<br>...........H... | E..TW............<br>............ | ......<br>....L |
| | | Germline | H_FR1 | H_CDR1 | H_FR2 | H_CDR2 | H_FR3 | H_CDR3 | H_FR4 |
| | VH4\|4-30.1/D2\|2-15\|RF3/JH4 | | QVQLQES-<br>GPGLVKPSQTLS<br>LTCTVSG-GSIS | SG---GYYWS | WIRQHPGK<br>GLEWIG | YIYY----<br>SGSTYYNP<br>SLKS | RVTISVDTSKNQFSLKL<br>SSVTAADTAVYYCAR | DIVVVVA-----------<br>-------ATYFDY | WGQGTL<br>VTVSS |
| iPS:436920 | 21-225_74E5 | VH4\|4-30.1/D2\|2-15\|RF3/JH4 | ............<br>............<br>.......R | .......... | ........<br>...... | ........<br>........<br>..R. | .AS..............<br>............... | .SP.A--<br>.................-<br>-GT.. | ......<br>..... |
| iPS:437012 | 21-225_192G7 | VH4\|4-30.1/D2\|2-15\|RF3/JH4 | ............<br>............<br>........ | .......... | ........<br>...... | ........<br>R.......<br>.... | ................<br>N.............. | .SP.T--<br>.................-<br>-G... | ....I.<br>..... |
| | | Germline | H_FR1 | H_CDR1 | H_FR2 | H_CDR2 | H_FR3 | H_CDR3 | H_FR4 |
| | VH1\|1-08/D5\|5-12\|RF3/JH6 | | QVQLVQS-<br>GAEVKKPGASVK<br>VSCKASG-YTFT | S-----YDIN | WVRQATGQ<br>GLEWMG | WMNPN---<br>SGNTGYAQ<br>KFQG | RVTMTRNTSISTAYMEL<br>SSLRSEDTAVYYCAR | GYSGYDYY----------<br>----YYYYGMDV | WGQGTT<br>VTVSS |
| iPS:436942 | 21-225_146H8 | VH1\|1-08/D5\|5-12\|RF3/JH6 | ............<br>............<br>.......I | .......... | ........<br>...... | ........<br>........<br>.... | .........K.......<br>............... | .DYY..SSGHQ.......<br>..PY........ | ......<br>..... |
| | | Germline | H_FR1 | H_CDR1 | H_FR2 | H_CDR2 | H_FR3 | H_CDR3 | H_FR4 |
| | VH2\|2-05/D6\|6-6\|RF3/JH4 | | QITLKES-<br>GPTLVKPTQTLT<br>LTCTFSG-FSLS | TS---GVGVG | WIRQPPGK<br>ALEWLA | LIYW----<br>NDDKRYSP<br>SLKS | RLTITKDTSKNQVVLTM<br>TNMDPVDTATYYCAH | V*QLV-------------<br>---------YFDY | WGQGTL<br>VTVSS |
| iPS:436944 | 21-225_182D12 | VH2\|2-05/D6\|6-6\|RF3/JH4 | ............<br>.....P......<br>........ | .T........ | ........<br>.....G | ILF.....<br>...E....<br>.... | .................<br>............... | KS...............<br>............ | ......<br>..... |

| | | | | | | | | | |
|---|---|---|---|---|---|---|---|---|---|
| | VH4\|4-30.1/D2\|2-8\|RF3/JH4 | | QVQLQES-<br>GPGLVKPSQTLS<br>LTCTVSG-GSIS | SG---GYYWS | WIRQHPGK<br>GLEWIG | YIYY----<br>SGSTYYNP<br>SLKS | RVTISVDTSKNQFSLKL<br>SSVTAADTAVYYCAR | DIVLMVY----------<br>------AIYFDY | WGQGTL<br>VTVSS |
| iPS:43 6958 | 21-225_190D1 | VH4\|4-30.1/D2\|2-8\|RF3/JH4 | ............<br>............<br>........ | ........... | ........<br>...... | ........<br>........<br>.... | .................<br>............... | .SP.R--<br>..................-<br>-G... | ......<br>..... |
| | | Germline | H_FR1 | H_CDR1 | H_FR2 | H_CDR2 | H_FR3 | H_CDR3 | H_FR4 |
| | VH6\|6-01/D4\|4-17\|RF2/JH4 | | QVQLQQS-<br>GPGLVKPSQTLS<br>LTCAISG-DSVS | SN---SAAWN | WIRQSPSR<br>GLEWLG | RTYYR---<br>SKWYNDYA<br>VSVKS | RITINPDTSKNQFSLQL<br>NSVTPEDTAVYYCAR | DYGDY------------<br>---------YFDY | WGQGTL<br>VTVSS |
| iPS:43 6962 | 21-225_190H1 | VH6\|6-01/D4\|4-17\|RF2/JH4 | ............<br>...........D<br>........ | RK.....T.. | ........<br>...... | K.......<br>........<br>.... | .................<br>............... | .P.G-<br>..................<br>...L... | ......<br>..... |
| iPS:43 6978 | 21-225_190G9 | VH6\|6-01/D4\|4-17\|RF2/JH4 | ............<br>............<br>........ | RK.....T.. | ........<br>...... | ........<br>........<br>.... | ..I..............<br>............... | .P.G-<br>..................<br>...L... | ......<br>..... |
| iPS:43 7070 | 21-225_201G11 | VH6\|6-01/D4\|4-17\|RF2/JH4 | ............<br>............<br>........ | RI...NPT.. | ........<br>...... | ........<br>...HV...<br>.... | .................<br>............... | .P.G-<br>..................<br>...L... | ......<br>..... |
| iPS:43 7076 | 21-225_203G6 | VH6\|6-01/D4\|4-17\|RF2/JH4 | ............<br>............<br>........ | RT...NPT.. | ........<br>...... | ........<br>...HV..L<br>.... | ....T............<br>............... | .P.G-<br>..................<br>...L... | ......<br>..... |
| | | Germline | H_FR1 | H_CDR1 | H_FR2 | H_CDR2 | H_FR3 | H_CDR3 | H_FR4 |
| | VH3\|3-33/D1\|1-7\|RF3/JH6 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | YNWNYYY----------<br>-----YYYGMDV | WGQGTT<br>VTVSS |
| iPS:43 6964 | 21-225_190B3 | VH3\|3-33/D1\|1-7\|RF3/JH6 | ............<br>............<br>.......N | N.......I. | ........<br>...... | ...F....<br>.D......<br>.... | .................<br>............... | D....GDH.........<br>....Y..F.... | ......<br>..... |
| iPS:43 6970 | 21-225_190B8 | VH3\|3-33/D1\|1-7\|RF3/JH6 | ............<br>............<br>........ | .......... | ........<br>...... | ...F....<br>.......T.<br>.... | .................<br>............... | D....GDY.........<br>....Y....... | ......<br>..... |
| iPS:43 6980 | 21-225_190C10 | VH3\|3-33/D1\|1-7\|RF3/JH6 | ............<br>............<br>......L. | N......... | ........<br>...... | ...FG...<br>.D......<br>..R. | .................<br>..........F.... | D....GDH.........<br>....Y....... | ......<br>..... |

| | | | | | | | | | |
|---|---|---|---|---|---|---|---|---|---|
| iPS:43 6992 | 21-225_191B8 | VH3\|3-33/D1\|1-7\|RF3/JH6 | ............ ............ ......L. | .......... | ........ ...... | ...F.... ........ .... | ................. ............... | D....GDH.......... ....Y....... | ...... ..... |
| iPS:43 6994 | 21-225_191A9 | VH3\|3-33/D1\|1-7\|RF3/JH6 | ............ ............ ......L. | N......... | ........ ...... | ...FG... .D...... .... | ................. ..........F.... | D....GDH.......... ....Y....... | ...... ..... |
| | | Germline | H_FR1 | H_CDR1 | H_FR2 | H_CDR2 | H_FR3 | H_CDR3 | H_FR4 |
| | VH4\|4-30.1/D4\|4-11\|RF2/JH6 | | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | DYSNYYY-----------------YYYGMDV | WGQGTTVTVSS |
| iPS:43 6984 | 21-225_190F10 | VH4\|4-30.1/D4\|4-11\|RF2/JH6 | ............ ............ .......R | ......D... | .......E ...... | ........ ..I..... .... | ................. ............... | .S.SR-- ................-- -.... | ...... ..... |
| iPS:43 6988 | 21-225_191A2 | VH4\|4-30.1/D4\|4-11\|RF2/JH6 | ............ ............ .......R | ......D... | .......E ...... | ........ ..I..... .... | ................. ............... | .S.SR-- ................-- -.... | ...... ..... |
| iPS:43 7014 | 21-225_192H8 | VH4\|4-30.1/D4\|4-11\|RF2/JH6 | ...........V ............ ........ | ......D... | ........ ...... | ........ ..P..... .... | .L.M.A........... ............... | .S.L.-- ................-- -.... | ...... ..... |
| iPS:43 7022 | 21-225_194G5 | VH4\|4-30.1/D4\|4-11\|RF2/JH6 | ............ .........I.. .......R | ......D... | ........ ...... | ........ ........ .... | ................. ............... | .H.L.-- ................-- -.... | ...... ..... |
| iPS:43 7026 | 21-225_194D12 | VH4\|4-30.1/D4\|4-11\|RF2/JH6 | ............ ............ ........ | ......D... | ........ ...... | ........ ........ .... | ................. ............... | .GARH-- ................-- -.... | ...... ..... |
| iPS:43 7056 | 21-225_198B8 | VH4\|4-30.1/D4\|4-11\|RF2/JH6 | ............ ............ .......R | ......D... | .......E ...... | ........ ..I..... .... | ................. ............... | .S.SR-- ................-- -.... | ...... ..... |
| iPS:43 7124 | 21-225_212H12 | VH4\|4-30.1/D4\|4-11\|RF2/J | ............ ............ .......R | ......D... | ........ ...... | ........ ........ .... | .....G........... ............... | .S.S.-- ................-- -.... | ...... ..... |

| | | | | | | | | | |
|---|---|---|---|---|---|---|---|---|---|
| iPS:437136 | 21-225_214H3 | VH4\|4-30.1/D4\|4-11\|RF2/JH6 | ............ ............ .......R | ......D... | ........ ...... | ........ ........ .... | .....G.......... .............. | .S.S.-- ................-- -.... | ...... ..... |
| | | Germline | H_FR1 | H_CDR1 | H_FR2 | H_CDR2 | H_FR3 | H_CDR3 | H_FR4 |
| | VH4\|4-59/D1\|1-26\|RF1/JH4 | | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAR | GIVGA-------------------TYFDY | WGQGTLVTVSS |
| iPS:436986 | 21-225_191A1 | VH4\|4-59/D1\|1-26\|RF1/JH4 | ............ ............ .......R | .........I | ........ ...... | ........ ....K... .... | ................ .............. | KG..T............ .......IH... | ...... ..... |
| iPS:437064 | 21-225_200G8 | VH4\|4-59/D1\|1-26\|RF1/JH4 | ............ ............ .......R | .......... | ........ ...... | ........ ....K... .... | ................ .............. | KG..T............ .......IH... | ...... ..... |
| | | Germline | H_FR1 | H_CDR1 | H_FR2 | H_CDR2 | H_FR3 | H_CDR3 | H_FR4 |
| | VH3\|3-30/D6\|6-6\|RF1/JH4 | | QVQLVES-GGGVVQPGRSLRLSCAASG-FTFS | S-----YGMH | WVRQAPGKGLEWVA | VISYD---GSNKYYADSVKG | RFTISRDNSKNTLYLQMNSLRAEDTAVYYCAK | EYSSS-------------------SYFDY | WGQGTLVTVSS |
| iPS:436996 | 21-225_191B9 | VH3\|3-30/D6\|6-6\|RF1/JH4 | ............ ............ ........ | F.....H... | ........ ...... | ..W..... ..K..... .... | .............H... .............. | .GY..GF.......... ......YRG..N | ...... ..... |
| iPS:437054 | 21-225_194G3 | VH3\|3-30/D6\|6-6\|RF1/JH4 | ............ ............ ........ | F.....H... | ........ ...... | ..W..... ..K..... .... | .............H... .............. | .GF..GF.......... ......YRG..N | ...... ..... |
| | | Germline | H_FR1 | H_CDR1 | H_FR2 | H_CDR2 | H_FR3 | H_CDR3 | H_FR4 |
| | VH3\|3-33/D1\|1-26\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | GIVGATYY-----------------YYYGMDV | WGQGTTVTVSS |
| iPS:437000 | 21-225_191G9 | VH3\|3-33/D1\|1-26\|RF1/JH6 | ............ ............ ........ | T......... | ........ .....T | L..F.... ........ .... | ................ .............. | DR..G.SPP........ ...YY....... | ...... ..... |
| iPS:393192 | 21-225_12B1 | VH3\|3-33/D1\|1-26\|RF1/JH6 | ............ ............ ........ | .......... | ........ ...... | ...N.... ........ .... | ................ .............. | DR.A.AGTP........ ....Y....... | ...... ..... |
| | | Germline | H_FR1 | H_CDR1 | H_FR2 | H_CDR2 | H_FR3 | H_CDR3 | H_FR4 |
| | VH1\|1-02/D3\|3-10\|RF2/JH5 | | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | YYYGSGSY-----------------YNNWFDP | WGQGTLVTVSS |

| | | | | | | | | | |
|---|---|---|---|---|---|---|---|---|---|
| iPS:43 7002 | 21-225_191H9 | VH1\|1-02/D3\|3-10\|RF2/JH5 | ............ ............ V....... | ........N.. | ........ ...... | ........ .......H .... | ................. ............... | DF.D..G- ................- EG.... | ...... ..... |
| | | Germline | H_FR1 | H_CDR1 | H_FR2 | H_CDR2 | H_FR3 | H_CDR3 | H_FR4 |
| | VH4\|4-30.4/D2\|2-8\|RF3/JH6 | | QVQLQES-GPGLVKPSQTLSLTCTVSG-GSIS | SG---DYYWS | WIRQPPGKGLEWIG | YIYY----SGSTYYNPSLKS | RVTISVDTSKNQFSLKLSSVTAADTAVYYCAR | DIVLMVYAI-----------YYYYYGMDV | WGQGTTVTVSS |
| iPS:43 7008 | 21-225_192E3 | VH4\|4-30.4/D2\|2-8\|RF3/JH6 | ............ ............ ........ | .....G.... | ........ ...... | ........ T....... .... | ................. ............... | .DP.Y---- ............----- .... | ...... ..... |
| iPS:43 7048 | 21-225_197B11 | VH4\|4-30.4/D2\|2-8\|RF3/JH6 | ............ ............ ........ | .....G.... | ........ ...... | ........ T....... .... | ................. ............... | .DP.Y---- ............----- .... | ...... ..... |
| | | Germline | H_FR1 | H_CDR1 | H_FR2 | H_CDR2 | H_FR3 | H_CDR3 | H_FR4 |
| | VH4\|4-59/D7\|7-27\|RF3/JH4 | | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAR | NWGY-------------------FDY | WGQGTLVTVSS |
| iPS:43 7010 | 21-225_192G3 | VH4\|4-59/D7\|7-27\|RF3/JH4 | ............ ............ ........ | N......... | .....A.. ...... | R..S.... ........ .... | ...M............. N.............K | G.E- ................. .....LN. | ...... ..... |
| iPS:43 7016 | 21-225_193A6 | VH4\|4-59/D7\|7-27\|RF3/JH4 | ............ ............ ........ | .......... | ........ ...... | ........ ........ .... | ................. ..............G | G.E- ................. .....LN. | ...... ..... |
| | | Germline | H_FR1 | H_CDR1 | H_FR2 | H_CDR2 | H_FR3 | H_CDR3 | H_FR4 |
| | VH3\|3-15/D4\|4-17\|RF2/JH4 | | EVQLVES-GGGLVKPGGSLRLSCAASG-FTFS | N-----AWMS | WVRQAPGKGLEWVG | RIKSKT-DGGTTDYAAPVKG | RFTISRDDSKNTLYLQMNSLKTEDTAVYYCTT | DYGDY-----------------YFDY | WGQGTLVTVSS |
| iPS:43 7018 | 21-225_193H5 | VH3\|3-15/D4\|4-17\|RF2/JH4 | ............ .M.....S.... ........ | .......Y.T | ........ ...... | ........ ........ .... | ................. ............... | .P.G- ................. ...I... | ...... ..... |
| iPS:43 7144 | 21-225_215B3 | VH3\|3-15/D4\|4-17\|RF2/JH4 | ............ ............ ........ | .........H | ........ ...... | .......N ........ .... | ................. ............... | .P.G- ................. ...I... | ...... ..... |
| | | Germline | H_FR1 | H_CDR1 | H_FR2 | H_CDR2 | H_FR3 | H_CDR3 | H_FR4 |
| | VH3\|3-11/D3\|3- | | QVQLVES-GGGLVKPGGSLR | D-----YYMS | WIRQAPGKGLEWVS | YISSS---GSTIYYAD | RFTISRDNAKNSLYLQMNSLRAEDTAVYYCAR | VLRYFDW-----------LL*YFDY | WGQGTLVTVSS |

| | | | | | | | | | |
|---|---|---|---|---|---|---|---|---|---|
| iPS:43 7030 | 21-225_195E3 | VH3\|3-11/D3\|3-9\|RF1/JH4 | ............. ............. ........ | .......... | ........ ...... | ..T..... .N...... .... | ................. ............... | DS...-- ................-- DW... | ...... ..... |
| | | Germline | H_FR1 | H_CDR1 | H_FR2 | H_CDR2 | H_FR3 | H_CDR3 | H_FR4 |
| | VH4\|4-59/D7\|7-27\|RF3/JH1 | | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAR | NWGAE------------------YFQH | WGQGTLVTVSS |
| iPS:43 7032 | 21-225_195H6 | VH4\|4-59/D7\|7-27\|RF3/JH1 | ............. ............. .......R | N......... | .....A.. ...... | R..S.... ........ .... | ..SM............. .............T. | G.E-- ................... ...-LNN | ...... ..... |
| | | Germline | H_FR1 | H_CDR1 | H_FR2 | H_CDR2 | H_FR3 | H_CDR3 | H_FR4 |
| | VH4\|4-59/D6\|6-6\|RF1/JH6 | | QVQLQES-GPGLVKPSETLSLTCTVSG-GSIS | S-----YYWS | WIRQPPGKGLEWIG | YIYY----SGSTNYNPSLKS | RVTISVDTSKNQFSLKLSSVTAADTAVYYCAR | EYSSSSYY---------------YYYGMDV | WGQGTTVTVSS |
| iPS:43 7044 | 21-225_197F9 | VH4\|4-59/D6\|6-6\|RF1/JH6 | ............. ............. .......R | I......... | ........ ...... | .V...... ....T... .... | ................. N...........V. | .RG..HRW.......... ....GD...... | ..R... ..... |
| iPS:43 7060 | 21-225_199C3 | VH4\|4-59/D6\|6-6\|RF1/JH6 | ............. ............. .......R | I......... | ....T... ...... | ........ ....T... .... | ................. N...........V. | .RG..HRW.......... ....GD...... | ..R... ..... |
| | | Germline | H_FR1 | H_CDR1 | H_FR2 | H_CDR2 | H_FR3 | H_CDR3 | H_FR4 |
| | VH3\|3-30/D6\|6-6\|RF1/JH1 | | QVQLVES-GGGVVQPGRSLRLSCAASG-FTFS | S-----YGMH | WVRQAPGKGLEWVA | VISYD---GSNKYYADSVKG | RFTISRDNSKNTLYLQMNSLRAEDTAVYYCAK | EYSSSS-----------------AEYFQH | WGQGTLVTVSS |
| iPS:43 7058 | 21-225_199F3 | VH3\|3-30/D6\|6-6\|RF1/JH1 | ............. ............. ........ | F......... | ........ ...... | ..W..... ..S..... .... | ...V............. ............... | .GY..GF.......... ......YRG.AN | ...... ..... |
| | | Germline | H_FR1 | H_CDR1 | H_FR2 | H_CDR2 | H_FR3 | H_CDR3 | H_FR4 |
| | VH4\|4-30.1/D6\|6-6\|RF2/JH6 | | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | SIAARYY-----------------YYYGMDV | WGQGTTVTVSS |
| iPS:43 7062 | 21-225_200H1 | VH4\|4-30.1/D6\|6-6\|RF2/JH6 | ............. ............. ........ | ......D... | ........ ...... | ........ ........ .... | ................. N............. | DG..L-- ................-- -.... | ...... ..... |
| iPS:43 7066 | 21-225_200G9 | VH4\|4-30.1/D6\|6-6\|RF2/JH | ............. ............. ........ | ......D... | ........ ...... | ........ ........ .... | ................. N............. | DG..L-- ................-- -.... | ...... ..... |

| | | | | | | | | | |
|---|---|---|---|---|---|---|---|---|---|
| iPS:437068 | 21-225_200A11 | VH4\|4-30.1/D6\|6-6\|RF2/JH6 | .................................. | ......D... | ............... | R............ | ................................. | DA..H--...................----.... | ........... |
| iPS:437140 | 21-225_214E12 | VH4\|4-30.1/D6\|6-6\|RF2/JH6 | .................................. | ......D... | ............... | ....P....... | ................................. | DG..E--...................----.... | ........... |
| iPS:437158 | 21-225_216H11 | VH4\|4-30.1/D6\|6-6\|RF2/JH6 | .................................. | ......D... | ............... | ....P....... | ................................. | DG..E--...................---.L.. | ........... |
| | | Germline | H_FR1 | H_CDR1 | H_FR2 | H_CDR2 | H_FR3 | H_CDR3 | H_FR4 |
| | VH3\|3-33/D1\|1-26\|RF3/JH3 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | YSGSYY-------------------DAFDI | WGQGTMVTVSS |
| iPS:437074 | 21-225_203B2 | VH3\|3-33/D1\|1-26\|RF3/JH3 | .................................. | N......... | ............... | I..F.......E....... | .........MS............................. | E....-....................-.LY. | ........... |
| iPS:437082 | 21-225_205E12 | VH3\|3-33/D1\|1-26\|RF3/JH3 | .................................. | N......... | ............... | I..F.......E....... | .........MS............................. | E....-....................-.LY. | ........... |
| | | Germline | H_FR1 | H_CDR1 | H_FR2 | H_CDR2 | H_FR3 | H_CDR3 | H_FR4 |
| | VH3\|3-48/D4\|4-17\|RF2/JH4 | | EVQLVES-GGGLVQPGGSLRLSCAASG-FTFS | S-----YSMN | WVRQAPGKGLEWVS | YISSS---SSTIYYADSVKG | RFTISRDNAKNSLYLQMNSLRDEDTAVYYCAR | DYGDY--------------------YFDY | WGQGTLVTVSS |
| iPS:437086 | 21-225_209A8 | VH3\|3-48/D4\|4-17\|RF2/JH4 | .........................R | .......... | .............L | ..........IKK....... | ..............................V. | .D.S...................... | ........... |
| | | Germline | H_FR1 | H_CDR1 | H_FR2 | H_CDR2 | H_FR3 | H_CDR3 | H_FR4 |
| | VH4\|4-30.1/D2\|2-8\|RF3/JH6 | | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | DIVLMVYAI----------YYYYYGMDV | WGQGTTVTVSS |
| iPS:437090 | 21-225_210F11 | VH4\|4-30.1/D2\|2-8\|RF3/JH6 | .................................. | ......S... | ............... | ........I.T........ | .........T.H.......................... | .EP.T----............-----.... | ........... |

| | | | | | | | | | |
|---|---|---|---|---|---|---|---|---|---|
| iPS:43 7106 | 21-225_211H7 | VH4\|4-30.1/D2\|2-8\|RF3/JH6 | .............<br>.............<br>......... | ........... | .T......<br>...... | ........<br>V.......<br>.... | .................<br>............... | .GP.S----<br>............-----<br>.... | ......<br>..... |
| | | Germline | **H_FR1** | **H_CDR1** | **H_FR2** | **H_CDR2** | **H_FR3** | **H_CDR3** | **H_FR4** |
| | VH3\|3-30.3/D4\|4-17\|RF2/JH5 | | QVQLVES-<br>GGGVVQPGRSLR<br>LSCAASG-FTFS | S-----YAMH | WVRQAPGK<br>GLEWVA | VISYD---<br>GSNKYYAD<br>SVKG | RFTISRDNSKNTLYLQM<br>NSLRAEDTAVYYCAR | DYGDY------------<br>-------NWFDP | WGQGTL<br>VTVSS |
| iPS:43 7100 | 21-225_211H2 | VH3\|3-30.3/D4\|4-17\|RF2/JH5 | .............<br>.............<br>......... | ........... | ........<br>...... | ..W.....<br>........<br>.... | ....A............<br>............... | .P.S..............<br>.......-G... | ......<br>..... |
| | | Germline | **H_FR1** | **H_CDR1** | **H_FR2** | **H_CDR2** | **H_FR3** | **H_CDR3** | **H_FR4** |
| | VH3\|3-33/D5\|5-12\|RF3/JH6 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | GYSGYDYY---------<br>----YYYYGMDV | WGQGTT<br>VTVSS |
| iPS:43 7102 | 21-225_211E5 | VH3\|3-33/D5\|5-12\|RF3/JH6 | .............<br>.............<br>.......R | N.......... | ........<br>.....S | I..F....<br>..DQ....<br>.... | .................<br>............... | .L.V.Y--<br>..............---<br>...G. | ......<br>..... |
| iPS:43 7164 | 21-225_217C6 | VH3\|3-33/D5\|5-12\|RF3/JH6 | .............<br>.............<br>......... | N.......... | ........<br>....M. | I..F....<br>..DE....<br>.... | ............M....<br>............... | .L.V.Y--<br>..............---<br>..... | ......<br>..... |
| iPS:43 7166 | 21-225_217G11 | VH3\|3-33/D5\|5-12\|RF3/JH6 | .............<br>.............<br>.......R | N.......... | ........<br>.....S | I..F....<br>..DQ....<br>.... | .................<br>............... | .L.V.Y--<br>..............---<br>..... | ......<br>..... |
| iPS:43 7170 | 21-225_218E5 | VH3\|3-33/D5\|5-12\|RF3/JH6 | .............<br>.............<br>......... | N.......... | ........<br>....M. | I..F....<br>..DE....<br>.... | .................<br>............... | .L.V.Y--<br>..............---<br>..... | ......<br>..... |
| | | Germline | **H_FR1** | **H_CDR1** | **H_FR2** | **H_CDR2** | **H_FR3** | **H_CDR3** | **H_FR4** |
| | VH4\|4-30.1/D6\|6-19\|RF2/JH6 | | QVQLQES-<br>GPGLVKPSQTLS<br>LTCTVSG-GSIS | SG---GYYWS | WIRQHPGK<br>GLEWIG | YIYY----<br>SGSTYYNP<br>SLKS | RVTISVDTSKNQFSLKL<br>SSVTAADTAVYYCAR | GIAVAGYY---------<br>-----YYYGMDV | WGQGTT<br>VTVSS |
| iPS:43 7108 | 21-225_211C9 | VH4\|4-30.1/D6\|6-19\|RF2/JH6 | .............<br>.............<br>.......R | ......D... | ........<br>...... | ........<br>........<br>.... | ....LL...........<br>....V.......... | DS..Y---<br>...............---<br>N... | ......<br>..... |

| | | | | | | | | | |
|---|---|---|---|---|---|---|---|---|---|
| iPS:437110 | 21-225_211E9 | VH4\|4-30.1/D6\|6-19\|RF2/JH6 | ............ ............ ........ | ......D... | ........ ...... | ........ T..N.... .... | ...............N. I............. | DS..Y--- ...............--- .... | ...... ..... |
| iPS:437120 | 21-225_212A9 | VH4\|4-30.1/D6\|6-19\|RF2/JH6 | ............ ...........S .......R | ......D... | ........ ...... | .M...... ........ .... | ................. ....V.......... | DS..Y--- ...............--- .... | ...... ..... |
| iPS:437132 | 21-225_213F5 | VH4\|4-30.1/D6\|6-19\|RF2/JH6 | ............ ............ .......R | ......D... | ........ ...... | ........ ........ .... | .....L........... ....V.......... | DS..Y--- ...............--- N... | ...... ..... |
| iPS:437142 | 21-225_215A3 | VH4\|4-30.1/D6\|6-19\|RF2/JH6 | ............ ............ ........ | ......D... | ........ ...... | ........ T..N.... .... | ................V I............. | DS..Y--- ...............--- .... | ...... ..... |
| iPS:437148 | 21-225_215H3 | VH4\|4-30.1/D6\|6-19\|RF2/JH6 | ............ ...........S .......R | ......D... | ........ ...... | .M...... ........ .... | ................. ....V.......... | DS..Y--- ...............--- .... | ...... ..... |
| iPS:437154 | 21-225_216A7 | VH4\|4-30.1/D6\|6-19\|RF2/JH6 | ............ ...........S .......R | ......D... | ........ ...... | .M...... ........ .... | ................. ....V.......... | DS..Y--- ...............--- .... | ...... ..... |
| | | Germline | H_FR1 | H_CDR1 | H_FR2 | H_CDR2 | H_FR3 | H_CDR3 | H_FR4 |
| | VH3\|3-30.3/D7\|7-27\|RF2/JH4 | | QVQLVES- GGGVVQPGRSLR LSCAASG-FTFS | S-----YAMH | WVRQAPGK GLEWVA | VISYD--- GSNKYYAD SVKG | RFTISRDNSKNTLYLQM NSLRAEDTAVYYCAR | *LGY------------- ----------FDY | WGQGTL VTVSS |
| iPS:437160 | 21-225_216B12 | VH3\|3-30.3/D7\|7-27\|RF2/JH4 | ............ ............ ........ | .......... | ........ ...... | ..W..... ........ .... | ................. ............... | D..LG............ ........YF... | ...... ..... |
| | | Germline | H_FR1 | H_CDR1 | H_FR2 | H_CDR2 | H_FR3 | H_CDR3 | H_FR4 |
| | VH6\|6-01/D2\|2-21\|RF2/JH5 | | QVQLQQS- GPGLVKPSQTLS LTCAISG-DSVS | SN---SAAWN | WIRQSPSR GLEWLG | RTYYR-- SKWYNDYA VSVKS | RITINPDTSKNQFSLQL NSVTPEDTAVYYCAR | AYCGGDC---------- -----YSNWFDP | WGQGTL VTVSS |
| iPS:437186 | 21-225_224H2 | VH6\|6-01/D2\|2-21\|RF2/J | ............ ...........D | .......... | ........ ...... | ........ ........ | .V............... ............... | EGGL.Y.SST........ ...SC.GG.... | ...... ..... |

| | | Germline | H_FR1 | H_CDR1 | H_FR2 | H_CDR2 | H_FR3 | H_CDR3 | H_FR4 |
|---|---|---|---|---|---|---|---|---|---|
| | VH1\|1-02/D4\|4-17\|RF2/JH6 | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | DYGDYYY----------<br>-----YYYGMDV | WGQGTT<br>VTVSS |
| iPS:43 7188 | 21-225_224B11 | VH1\|1-02/D4\|4-17\|RF2/JH6 | ..H.........<br>............<br>........ | ........I. | ........<br>...... | ....K...<br>N.......<br>.... | .......A....T....<br>............... | GAF..F...........<br>.....-..A... | ..H...<br>..... |
| iPS:43 7198 | 21-225_226F8 | VH1\|1-02/D4\|4-17\|RF2/JH6 | ............<br>............<br>........ | ........I. | ........<br>...... | ........<br>.......R<br>.... | .................<br>............... | GAF..............<br>.....-..AL.. | ......<br>..... |
| iPS:43 7202 | 21-225_227D3 | VH1\|1-02/D4\|4-17\|RF2/JH6 | ............<br>............<br>........ | .......... | ........<br>...R.. | ....K...<br>.....F..<br>.... | .................<br>............... | GAF..F...........<br>.....-...... | ......<br>..... |
| iPS:43 7208 | 21-225_227C10 | VH1\|1-02/D4\|4-17\|RF2/JH6 | ............<br>............<br>........ | .......... | ........<br>...R.. | ....K...<br>........<br>.... | .................<br>............... | GAF..F...........<br>.....-...... | ......<br>..... |
| | | Germline | H_FR1 | H_CDR1 | H_FR2 | H_CDR2 | H_FR3 | H_CDR3 | H_FR4 |
| | VH3\|3-33/D2\|2-2\|RF2/JH6 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | GYCSSTSCYT-------<br>---YYYYYGMDV | WGQGTT<br>VTVSS |
| iPS:43 7192 | 21-225_225E9 | VH3\|3-33/D2\|2-2\|RF2/JH6 | ............<br>...........E<br>.....I.. | .......... | ........<br>...... | .M......<br>.G..D...<br>.... | .................<br>............... | DREYC..TSC.......<br>..P......... | ......<br>..... |
| | | Germline | H_FR1 | H_CDR1 | H_FR2 | H_CDR2 | H_FR3 | H_CDR3 | H_FR4 |
| | VH3\|3-23/D2\|2-21\|RF2/JH6 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | AYCGGDCYS--------<br>---YYYYYGMDV | WGQGTT<br>VTVSS |
| iPS:43 7204 | 21-225_227E5 | VH3\|3-23/D2\|2-21\|RF2/JH6 | ............<br>...E........<br>........ | .......... | ........<br>...... | ........<br>........<br>.... | .............C...<br>............... | E........P.......<br>............ | ......<br>..... |
| iPS:39 3196 | 21-225_16G8 | VH3\|3-23/D2\|2-21\|RF2/JH6 | ............<br>...E........<br>........ | .......... | ........<br>...... | G.......<br>........<br>.... | .............C.H.<br>............... | E........P.......<br>............ | ......<br>..... |
| iPS:39 3202 | 21-225_6B4 | VH3\|3-23/D2\|2-21\|RF2/JH6 | ............<br>...E........<br>........ | .......... | ........<br>...... | ........<br>.S......<br>.... | .............C...<br>............... | E........P.......<br>............ | ......<br>..... |

| | | | | | | | | | |
|---|---|---|---|---|---|---|---|---|---|
| iPS:39 3345 | 21-225_5G7 | VH3\|3-23/D2\|2-21\|RF2/JH6 | ............<br>...E........<br>........ | .......... | ........<br>...... | ........<br>........<br>.... | .............C...<br>............... | E........P.........<br>............. | ......<br>..... |
| | | Germline | H_FR1 | H_CDR1 | H_FR2 | H_CDR2 | H_FR3 | H_CDR3 | H_FR4 |
| | VH2\|2-05/D6\|6-13\|RF3/JH4 | | QITLKES-<br>GPTLVKPTQTLT<br>LTCTFSG-FSLS | TS---GVGVG | WIRQPPGK<br>ALEWLA | LIYW----<br>NDDKRYSP<br>SLKS | RLTITKDTSKNQVVLTM<br>TNMDPVDTATYYCAH | V*QQL------------<br>-------VYFDY | WGQGTL<br>VTVSS |
| iPS:43 7210 | 21-225_227E12 | VH2\|2-05/D6\|6-13\|RF3/JH4 | ............<br>............<br>........ | .......... | ........<br>...C.S | ........<br>....V...<br>.... | ......Y..........<br>............... | RG...............<br>.......-AL.. | ......<br>..... |
| iPS:39 2587 | 21-225_18G5 | VH2\|2-05/D6\|6-13\|RF3/JH4 | ............<br>............<br>........ | .......... | ........<br>...C.S | ........<br>....V...<br>.... | ......Y..........<br>............... | RG...............<br>.......-AL.. | ......<br>..... |
| iPS:39 8504 | 21-225_23D7 | VH2\|2-05/D6\|6-13\|RF3/JH4 | ............<br>............<br>........ | .......... | ........<br>...C.S | ........<br>.N..V...<br>.... | ......Y..........<br>S.............. | RG...............<br>.......-AL.. | ......<br>..... |
| | | Germline | H_FR1 | H_CDR1 | H_FR2 | H_CDR2 | H_FR3 | H_CDR3 | H_FR4 |
| | VH3\|3-21/D4\|4-11\|RF2/JH6 | | EVQLVES-<br>GGGLVKPGGSLR<br>LSCAASG-FTFS | S-----YSMN | WVRQAPGK<br>GLEWVS | SISSS---<br>SSYIYYAD<br>SVKG | RFTISRDNAKNSLYLQM<br>NSLRAEDTAVYYCAR | DYSNYYY----------<br>-----YYYGMDV | WGQGTT<br>VTVSS |
| iPS:43 7226 | 21-225_57C2 | VH3\|3-21/D4\|4-11\|RF2/JH6 | ............<br>............<br>........ | ......FG.. | ........<br>...... | ........<br>TG...N..<br>.... | .................<br>............... | T..G---<br>................--<br>-SL.. | ......<br>..... |
| | | Germline | H_FR1 | H_CDR1 | H_FR2 | H_CDR2 | H_FR3 | H_CDR3 | H_FR4 |
| | VH3\|3-21/D5\|5-18\|RF3/JH5 | | EVQLVES-<br>GGGLVKPGGSLR<br>LSCAASG-FTFS | S-----YSMN | WVRQAPGK<br>GLEWVS | SISSS---<br>SSYIYYAD<br>SVKG | RFTISRDNAKNSLYLQM<br>NSLRAEDTAVYYCAR | GYSYGY-----------<br>-------NWFDP | WGQGTL<br>VTVSS |
| iPS:43 7230 | 21-225_62H10 | VH3\|3-21/D5\|5-18\|RF3/JH5 | ............<br>............<br>........ | .......... | ........<br>...... | ........<br>........<br>.... | .................<br>............... | .G.R--<br>.................<br>.-G... | ......<br>..... |
| iPS:44 8906 | 21-225_72G9 | VH3\|3-21/D5\|5-18\|RF3/JH5 | ............<br>............<br>........ | .......... | ........<br>...... | ...G....<br>........<br>.... | .................<br>...........F... | .G.R--<br>.................<br>.-G... | ......<br>..... |
| | | Germline | H_FR1 | H_CDR1 | H_FR2 | H_CDR2 | H_FR3 | H_CDR3 | H_FR4 |
| | VH1\|1-02/D1\|1-26\|RF1/JH4 | | QVQLVQS-<br>GAEVKKPGASVK<br>VSCKASG-YTFT | G-----YYMH | WVRQAPGQ<br>GLEWMG | WINPN---<br>SGGTNYAQ<br>KFQG | RVTMTRDTSISTAYMEL<br>SRLRSDDTAVYYCAR | GIVGA------------<br>-------TYFDY | WGQGTL<br>VTVSS |

| | | | | | | | | | |
|---|---|---|---|---|---|---|---|---|---|
| iPS:437260 | 21-225_170D1 | VH1\|1-02/D1\|1-26\|RF1/JH4 | ................................ | ........F.. | .............. | ..K.K.........C...... | .........S...........T............ | .GATVTT................WGV... | ........... |
| | | Germline | H_FR1 | H_CDR1 | H_FR2 | H_CDR2 | H_FR3 | H_CDR3 | H_FR4 |
| | VH3\|3-33/D2\|2-21\|RF2/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | AYCGGDCYS-----------YYYYYGMDV | WGQGTTVTVSS |
| iPS:437268 | 21-225_177D2 | VH3\|3-33/D2\|2-21\|RF2/JH6 | ................................ | .........D | .............. | I..F................ | ................................ | ........FP.........HLH...... | ........... |
| | | Germline | H_FR1 | H_CDR1 | H_FR2 | H_CDR2 | H_FR3 | H_CDR3 | H_FR4 |
| | VH4\|4-30.1/D4\|4-17\|RF2/JH4 | | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | DYGDY-------------------YFDY | WGQGTLVTVSS |
| iPS:437294 | 21-225_216D5 | VH4\|4-30.1/D4\|4-17\|RF2/JH4 | ................................ | .......... | .............. | .................... | ................N.........F... | .SP.R.................G... | ........... |
| | | Germline | H_FR1 | H_CDR1 | H_FR2 | H_CDR2 | H_FR3 | H_CDR3 | H_FR4 |
| | VH3\|3-30.3/D5\|5-24\|RF3/JH6 | | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | RDGYNYYY---------------YYYGMDV | WGQGTTVTVSS |
| iPS:437302 | 21-225_225B11 | VH3\|3-30.3/D5\|5-24\|RF3/JH6 | ................................ | .......G.. | .............T | I...S............... | ................................ | .GYSYG--...............-G..... | ........... |
| iPS:451102 | 21-225_45F6 | VH3\|3-30.3/D5\|5-24\|RF3/JH6 | ................................ | Y......GL. | .............. | .................... | .................N........F.... | E.R.CSGTSC..........PYY....... | ........... |
| iPS:392868 | 21-225_24D6 | VH3\|3-30.3/D5\|5-24\|RF3/JH6 | ................................ | .......G.. | ....V......... | I...A.......S....... | ..............................V. | .GYSYG--...............-G..... | ....A...... |
| iPS:393910 | 21-225_15F10 | VH3\|3-30.3/D5\|5-24\|RF3/JH6 | ................................ | .......G.. | .............S | ....G......N........ | ..............................V. | .GYSYG--...............-G..... | ........... |

| | | | | | | | | | |
|---|---|---|---|---|---|---|---|---|---|
| iPS:39 4000 | 21-225_11A2 | VH3\|3-30.3/D5\|5-24\|RF3/JH6 | ............<br>............<br>........ | .......G.. | ........<br>...... | ....G...<br>....DS..<br>.... | ..I..............<br>.............V. | .GYSYG--<br>...............-<br>G..... | ......<br>..... |
| iPS:39 4004 | 21-225_13A1 | VH3\|3-30.3/D5\|5-24\|RF3/JH6 | ............<br>............<br>........ | .......G.. | ........<br>...... | ....A...<br>.T.Q....<br>.... | .................<br>.............V. | .GYSYG--<br>...............-<br>G..... | ......<br>..... |
| iPS:39 4006 | 21-225_15C2 | VH3\|3-30.3/D5\|5-24\|RF3/JH6 | ............<br>............<br>........ | .......G.. | ........<br>.....S | I...G...<br>.R.NH...<br>.... | .................<br>.............V. | .GYSYG--<br>...............-<br>G..... | ......<br>..... |
| iPS:39 4029 | 21-225_1B12 | VH3\|3-30.3/D5\|5-24\|RF3/JH6 | ............<br>............<br>........ | .......G.. | ........<br>...... | I...A...<br>....S...<br>.... | ...........M.....<br>.............V. | .GYSYG--<br>...............-<br>G..... | ....A.<br>..... |
| iPS:39 4047 | 21-225_5E6 | VH3\|3-30.3/D5\|5-24\|RF3/JH6 | ............<br>............<br>........ | .......G.. | ........<br>...... | I...V...<br>.N......<br>.... | .................<br>....V.......... | .GYSYG--<br>...............-<br>G..... | ......<br>..... |
| iPS:39 4081 | 21-225_16B3 | VH3\|3-30.3/D5\|5-24\|RF3/JH6 | ............<br>...........V<br>........ | .......G.. | ........<br>...... | ....A...<br>.I..S...<br>.... | .........N.......<br>.............V. | .GYSYG--<br>...............-<br>G..... | ....A.<br>..... |
| | | Germline | H_FR1 | H_CDR1 | H_FR2 | H_CDR2 | H_FR3 | H_CDR3 | H_FR4 |
| | VH4\|4-61/D3\|3-9\|RF1/JH4 | | QVQLQES-GPGLVKPSETLSLTCTVSG-GSVS | SG---SYYWS | WIRQPPGKGLEWIG | YIYY----SGSTNYNPSLKS | RVTISVDTSKNQFSLKLSSVTAADTAVYYCAR | VLRYFDW-----------------LL*YFDY | WGQGTLVTVSS |
| iPS:45 1118 | 21-225_191C8 | VH4\|4-61/D3\|3-9\|RF1/JH4 | ............<br>............<br>..S..... | .....G.... | ........<br>...... | ........<br>..T.I...<br>.... | .................<br>T...V.......... | DTFC..G..........<br>.....CGYF..S | ......<br>..... |
| | | Germline | H_FR1 | H_CDR1 | H_FR2 | H_CDR2 | H_FR3 | H_CDR3 | H_FR4 |
| | VH2\|2-05/D4\|4-11\|RF3/JH4 | | QITLKES-GPTLVKPTQTLTLTCTFSG-FSLS | TS---GVGVG | WIRQPPGKALEWLA | LIYW----NDDKRYSPSLKS | RLTITKDTSKNQVVLTMTNMDPVDTATYYCAH | TTVI--------------------YFDY | WGQGTLVTVSS |
| iPS:39 2573 | 21-225_15G2 | VH2\|2-05/D4\|4-11\|RF3/JH4 | ............<br>............<br>........ | .......... | ........<br>...... | ........<br>........<br>.... | .................<br>..............D | .G.SC............<br>.......C..H. | ......<br>..... |

| | VH1\|1-02/D2\|2-2\|RF2/JH6 | | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | GYCSSTSCYT---------YYYYYGMDV | WGQGTTVTVSS |
|---|---|---|---|---|---|---|---|---|---|
| iPS:39 2585 | 21-225_14H11 | VH1\|1-02/D2\|2-2\|RF2/JH6 | ............ ...........Q G....... | ......H..C | ........ ...... | ........ ........ .... | .................. ..............A | .....S...L........ ...QPG...... | ...... ..... |
| iPS:39 3186 | 21-225_27D9 | VH1\|1-02/D2\|2-2\|RF2/JH6 | ............ ............ ........ | .......... | ........ ...... | ........ ....K... .... | .................. N............. | ER..T....L........ ..GITG...... | ...... ..... |
| iPS:39 3234 | 21-225_26C10 | VH1\|1-02/D2\|2-2\|RF2/JH6 | ............ ............ ........ | ........V. | ........ ...... | ........ ........ .... | .................. .............. | ER..T....L........ ..GITG...... | ...... ..... |
| | | Germline | H_FR1 | H_CDR1 | H_FR2 | H_CDR2 | H_FR3 | H_CDR3 | H_FR4 |
| | VH3\|3-23/D5\|5-12\|RF3/JH6 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GYSGYDYY-----------YYYYGMDV | WGQGTTVTVSS |
| iPS:39 2596 | 21-225_12D8 | VH3\|3-23/D5\|5-12\|RF3/JH6 | ............ ............ ........ | .......V.. | ........ ...... | T..VG... ........ .... | ..........T...... .............. | WGR..S.E.......... ....-....... | ...... ..... |
| iPS:39 2942 | 21-225_30E9 | VH3\|3-23/D5\|5-12\|RF3/JH6 | ...V........ ............ ........ | ......C..N | ........ ...... | ....R... ....F... .... | .................. .............. | .ELLE..- ..............- ....... | ...... ..... |
| iPS:39 2944 | 21-225_31H5 | VH3\|3-23/D5\|5-12\|RF3/JH6 | ............ ............ ........ | .......... | ........ ...... | ....R... ...IFH.. .... | .................. .............V. | .ELLE..- ..............- .F..... | ...... ..... |
| iPS:39 2964 | 21-225_31A8 | VH3\|3-23/D5\|5-12\|RF3/JH6 | ............ ............ ........ | .......... | ........ ...... | ....R... ....FH.. .... | .................. ....D........V. | .ELLE..- ..............- .F..... | ...... ..... |
| iPS:39 2982 | 21-225_30D1 | VH3\|3-23/D5\|5-12\|RF3/JH6 | ...V........ ............ ........ | .......... | ........ ...... | ....R... ....FH.. .... | .................. .............V. | .ELLE..- ..............- .F..L.. | ...... ..... |
| iPS:39 2986 | 21-225_31B8 | VH3\|3-23/D5\|5-12\|RF3/JH6 | ............ ............ ........ | .......... | ........ ...... | ....R... ....FH.. .... | ................L .....D.......V. | .ELLE..- ..............- .F..... | ...... ..... |
| iPS:39 | 21- | VH3\|3-23/D5\|5- | ............ ............ | .......... | ........ ...... | ....R... ....FN.. | ..........T...... .............V. | .ELLE..- ..............- | ...... ..... |

| | | | | | | | | | |
|---|---|---|---|---|---|---|---|---|---|
| iPS:39 3040 | 21-225_30E3 | VH3\|3-23/D5\|5-12\|RF3/JH6 | ...V........<br>............<br>........ | .........N | ........<br>...... | ....R...<br>....F...<br>.E.. | .................<br>.........L..... | .ELLE..-<br>..............-<br>....... | ......<br>..... |
| iPS:39 3058 | 21-225_31H3 | VH3\|3-23/D5\|5-12\|RF3/JH6 | ...V........<br>............<br>........ | .........N | ........<br>...... | ....R...<br>..N.F...<br>.... | .................<br>............... | .ELLE..-<br>..............-<br>....... | ......<br>..... |
| iPS:39 3060 | 21-225_32G12 | VH3\|3-23/D5\|5-12\|RF3/JH6 | ............<br>L...........<br>.......N | .......... | ........<br>...... | S...R...<br>....FH..<br>.... | .........R.......<br>.............V. | .ELLE..-<br>..............-<br>.F..... | ......<br>..... |
| iPS:39 3068 | 21-225_34G9 | VH3\|3-23/D5\|5-12\|RF3/JH6 | ....V.......<br>............<br>........ | .......... | ........<br>...... | ....R...<br>....FH..<br>.... | .................<br>.............V. | .ELLE..-<br>..............-<br>.F..... | ......<br>..... |
| iPS:39 3072 | 21-225_36C5 | VH3\|3-23/D5\|5-12\|RF3/JH6 | ....V.......<br>............<br>........ | .........N | ........<br>...... | ....R...<br>....FH..<br>.... | .................<br>.............V. | .ELLE..-<br>..............-<br>.F..... | ......<br>..... |
| iPS:39 3076 | 21-225_33A4 | VH3\|3-23/D5\|5-12\|RF3/JH6 | ............<br>..S........E<br>.....I.. | .........N | ....V...<br>...... | ...RR...<br>....F...<br>.... | .................<br>...........F... | .ELLE..-<br>..............-<br>S...I.. | ......<br>..... |
| iPS:39 3102 | 21-225_33F1 | VH3\|3-23/D5\|5-12\|RF3/JH6 | ............<br>L...........<br>........ | .......... | ........<br>...... | S...R...<br>....FH..<br>.... | .................<br>.............V. | .ELLE..-<br>..............-<br>.F..... | ......<br>..... |
| iPS:39 3104 | 21-225_33A7 | VH3\|3-23/D5\|5-12\|RF3/JH6 | ............<br>............<br>........ | ......C... | ........<br>...... | ....R...<br>....FH..<br>.... | .................<br>.............V. | .ELLE..-<br>..............-<br>.F..... | ......<br>..... |
| iPS:39 3106 | 21-225_34A6 | VH3\|3-23/D5\|5-12\|RF3/JH6 | ............<br>............<br>.......N | N........N | ........<br>...... | ...RR...<br>....F...<br>.... | .................<br>............... | .ELLE..-<br>..............-<br>..FA... | ......<br>..... |
| iPS:39 3110 | 21-225_35B7 | VH3\|3-23/D5\|5-12\|RF3/JH6 | ............<br>............<br>........ | .......... | ........<br>...... | ....R...<br>....FH..<br>.... | .................<br>.............V. | .ELLE..-<br>..............-<br>.F..... | ....A.<br>..... |
| iPS:39 3118 | 21-225_34H11 | VH3\|3-23/D5\|5-12\|RF3/JH6 | ............<br>............<br>........ | .........N | .......E<br>...... | ....R...<br>....F...<br>.... | .............F...<br>............... | .ELLE..-<br>..............-<br>....... | ......<br>..... |
| iPS:39 | 21-225_33G7 | VH3\|3-23/D5\|5- | ............<br>............ | N......... | ........<br>...... | ...RR...<br>....F... | Q................<br>............... | .ELLE..-<br>..............- | ......<br>..... |

| | | | | | | | | | |
|---|---|---|---|---|---|---|---|---|---|
| iPS:39 3126 | 21-225_35D1 | VH3\|3-23/D5\|5-12\|RF3/JH6 | ............<br>............<br>........ | .......... | ........<br>...... | ....R...<br>....FH..<br>.... | .........N.......<br>.............V. | .ELLE..–<br>..............–<br>.F..... | ....A.<br>..... |
| iPS:39 3128 | 21-225_35F11 | VH3\|3-23/D5\|5-12\|RF3/JH6 | ............<br>............<br>........ | .......... | ........<br>...... | ....R...<br>....FH..<br>.M.. | .................<br>.............V. | .ELLE..–<br>..............–<br>.F..... | ......<br>..... |
| iPS:39 3146 | 21-225_34G8 | VH3\|3-23/D5\|5-12\|RF3/JH6 | ............<br>............<br>........ | .......... | ........<br>...... | ....R...<br>....FH..<br>.... | ................I<br>.............V. | .ELLE..–<br>..............–<br>.F..... | ......<br>..... |
| iPS:39 3150 | 21-225_36A5 | VH3\|3-23/D5\|5-12\|RF3/JH6 | ............<br>............<br>.......N | N........N | ........<br>...... | ...RR...<br>..N.F...<br>.... | .................<br>............... | .ELLE..–<br>..............–<br>...A... | ......<br>..... |
| iPS:39 3180 | 21-225_4G12 | VH3\|3-23/D5\|5-12\|RF3/JH6 | ............<br>............<br>........ | .......... | ........<br>...... | TL..R...<br>........<br>.... | .S...............<br>S.............. | WGR..S.E..........<br>....–....... | ......<br>..... |
| iPS:39 3232 | 21-225_17F12 | VH3\|3-23/D5\|5-12\|RF3/JH6 | ............<br>............<br>........ | .......... | ........<br>...... | ....G...<br>........<br>.... | .V...............<br>............... | WGR..N.E..........<br>....–....... | ......<br>..... |
| iPS:39 8494 | 21-225_21H4 | VH3\|3-23/D5\|5-12\|RF3/JH6 | ............<br>............<br>........ | .......... | ........<br>...... | .L..R...<br>........<br>.... | .................<br>............... | WGR..S.E..........<br>....–....... | ......<br>..... |
| iPS:39 8508 | 21-225_24B1 | VH3\|3-23/D5\|5-12\|RF3/JH6 | ............<br>............<br>........ | .......... | ........<br>...... | ....R...<br>........<br>.... | .................<br>............... | WGR..S.E..........<br>....–....... | ......<br>..... |
| iPS:39 8528 | 21-225_32G1 | VH3\|3-23/D5\|5-12\|RF3/JH6 | ............<br>A...........<br>........ | .......... | ........<br>...... | ....R...<br>....FH..<br>.... | .................<br>.............V. | .ELLE..–<br>..............–<br>.F..... | ......<br>..... |
| iPS:39 8534 | 21-225_33B8 | VH3\|3-23/D5\|5-12\|RF3/JH6 | ............<br>............<br>........ | .......... | ........<br>...... | ....R...<br>....FH..<br>.... | .................<br>.............V. | .ELLE..–<br>..............–<br>.F..... | ......<br>..... |
| iPS:39 8540 | 21-225_35A6 | VH3\|3-23/D5\|5-12\|RF3/JH6 | ............<br>............<br>........ | .......... | ........<br>...... | T...R...<br>....FH..<br>.... | .................<br>.............V. | .ELLE..–<br>..............–<br>.F..... | ......<br>..... |
| | | Germline | H_FR1 | H_CDR1 | H_FR2 | H_CDR2 | H_FR3 | H_CDR3 | H_FR4 |

| | VH4\|4-39/D4\|4-17\|RF2/JH4 | | QLQLQES-<br>GPGLVKPSETLS<br>LTCTVSG-GSIS | SS---SYYWG | WIRQPPGK<br>GLEWIG | SIYY----<br>SGSTYYNP<br>SLKS | RVTISVDTSKNQFSLKL<br>SSVTAADTAVYYCAR | DYGDY------------<br>---------YFDY | WGQGTL<br>VTVSS |
|---|---|---|---|---|---|---|---|---|---|
| iPS:39 2622 | 21-225_17H8 | VH4\|4-39/D4\|4-17\|RF2/JH4 | ............<br>............<br>........ | R......... | .......R<br>...... | N.......<br>G.N.....<br>.... | .................<br>.............. | HGK.W............<br>........GL.. | ......<br>..... |
| iPS:39 2638 | 21-225_17F9 | VH4\|4-39/D4\|4-17\|RF2/JH4 | ............<br>............<br>........ | R......... | ........<br>...... | N.......<br>........<br>.... | .......S.......N.<br>N.........S... | HGK.W............<br>........GL.. | ......<br>..... |
| iPS:39 2656 | 21-225_1F2 | VH4\|4-39/D4\|4-17\|RF2/JH4 | ............<br>............<br>........ | R......... | ........<br>...... | N.......<br>...A.N..<br>...G | .................<br>N...........G. | HGK.W............<br>........GL.. | ......<br>..... |
| iPS:39 2794 | 21-225_21H3 | VH4\|4-39/D4\|4-17\|RF2/JH4 | ............<br>............<br>........ | R......... | ........<br>...... | N.......<br>.....D..<br>.... | .................<br>.............G. | HGK.W............<br>........GL.. | ......<br>..... |
| iPS:39 2822 | 21-225_23C8 | VH4\|4-39/D4\|4-17\|RF2/JH4 | ............<br>............<br>........ | R......... | ........<br>...... | N.......<br>..T..N..<br>.... | ...........H.....<br>.............G. | HGK.W............<br>........GL.. | ......<br>..... |
| iPS:39 2838 | 21-225_22G8 | VH4\|4-39/D4\|4-17\|RF2/JH4 | ............<br>............<br>.......N | R......... | ........<br>..D... | N.......<br>........<br>.V.. | .F...............<br>.............. | HGK.W............<br>........GL.. | ......<br>..... |
| iPS:39 2858 | 21-225_22H4 | VH4\|4-39/D4\|4-17\|RF2/JH4 | ............<br>............<br>........ | R......... | ........<br>...... | N.......<br>.....H..<br>.... | .........M.......<br>T.........F.G. | HGK.W............<br>........GL.. | ......<br>..... |
| iPS:39 2882 | 21-225_23A3 | VH4\|4-39/D4\|4-17\|RF2/JH4 | ............<br>............<br>........ | R......... | .......Q<br>...... | N.......<br>.....N..<br>.... | ..S............N.<br>.............G. | HGK.W............<br>........GL.F | ......<br>..... |
| iPS:39 3804 | 21-225_5H7 | VH4\|4-39/D4\|4-17\|RF2/JH4 | ............<br>............<br>.......N | R......... | ........<br>...... | N.......<br>..T.....<br>.... | ...............N.<br>...........S... | HGK.W............<br>........GL.. | ......<br>..... |
| iPS:39 3832 | 21-225_14B2 | VH4\|4-39/D4\|4-17\|RF2/JH4 | ............<br>............<br>.......N | R......... | ........<br>...... | N.......<br>..T.....<br>.... | ...............N.<br>...........S... | HGK.W............<br>........GL.. | ....A.<br>..... |
| iPS:39 4037 | 21-225_4F4 | VH4\|4-39/D4\|4-17\|RF2/J | ............<br>.R.......... | R......... | ........<br>...... | N.......<br>.....D.. | .................<br>.............G. | HGK.W............<br>........GL.. | ......<br>..... |

| | | | | | | | | | |
|---|---|---|---|---|---|---|---|---|---|
| iPS:39 4045 | 21-225_4H4 | VH4\|4-39/D4\|4-17\|RF2/JH4 | ............<br>............<br>........ | R......... | .......M<br>...... | N.......<br>..N..N..<br>.... | .................<br>N............G. | HGK.W.............<br>........GL.. | ......<br>.I... |
| iPS:39 4079 | 21-225_11F5 | VH4\|4-39/D4\|4-17\|RF2/JH4 | ............<br>............<br>........ | R......... | ........<br>...... | N.......<br>......T..<br>.... | ...........H.....<br>.............G. | HGK.W.............<br>........GL.N | ......<br>..... |
| | | Germline | **H_FR1** | **H_CDR1** | **H_FR2** | **H_CDR2** | **H_FR3** | **H_CDR3** | **H_FR4** |
| | VH3\|3-21/D4\|4-11\|RF2/JH3 | | EVQLVES-<br>GGGLVKPGGSLR<br>LSCAASG-FTFS | S-----YSMN | WVRQAPGK<br>GLEWVS | SISSS---<br>SSYIYYAD<br>SVKG | RFTISRDNAKNSLYLQM<br>NSLRAEDTAVYYCAR | DYSNY-------------<br>-------DAFDI | WGQGTM<br>VTVSS |
| iPS:39 2624 | 21-225_17H12 | VH3\|3-21/D4\|4-11\|RF2/JH3 | ............<br>............<br>........ | .......T.. | ........<br>...... | ...G....<br>........<br>.... | .................<br>............... | .RG--<br>..................<br>..---S. | ......<br>..... |
| iPS:39 3946 | 21-225_16A4 | VH3\|3-21/D4\|4-11\|RF2/JH3 | ............<br>............<br>........ | .......... | ........<br>...... | ...G....<br>.T.K....<br>.... | .................<br>....T.......... | .RG--<br>..................<br>..---SY | .....Q<br>..... |
| iPS:39 4008 | 21-225_15H8 | VH3\|3-21/D4\|4-11\|RF2/JH3 | ............<br>............<br>........ | .......V.. | ........<br>...... | ...G....<br>.T...C..<br>.I.. | .................<br>.....D......... | .RG--<br>..................<br>..---S. | ......<br>..... |
| | | Germline | **H_FR1** | **H_CDR1** | **H_FR2** | **H_CDR2** | **H_FR3** | **H_CDR3** | **H_FR4** |
| | VH6\|6-01/D6\|6-19\|RF1/JH6 | | QVQLQQS-<br>GPGLVKPSQTLS<br>LTCAISG-DSVS | SN---SAAWN | WIRQSPSR<br>GLEWLG | RTYYR--<br>SKWYNDYA<br>VSVKS | RITINPDTSKNQFSLQL<br>NSVTPEDTAVYYCAR | GYSSGWYY----------<br>----YYYYGMDV | WGQGTT<br>VTVSS |
| iPS:39 2636 | 21-225_17A6 | VH6\|6-01/D6\|6-19\|RF1/JH6 | ............<br>............<br>........ | R....T...S | ........<br>...... | ........<br>........<br>.... | .V...............<br>............... | VS.GWSHH.........<br>............ | ......<br>..... |
| | | Germline | **H_FR1** | **H_CDR1** | **H_FR2** | **H_CDR2** | **H_FR3** | **H_CDR3** | **H_FR4** |
| | VH6\|6-01/D2\|2-15\|RF2/JH6 | | QVQLQQS-<br>GPGLVKPSQTLS<br>LTCAISG-DSVS | SN---SAAWN | WIRQSPSR<br>GLEWLG | RTYYR--<br>SKWYNDYA<br>VSVKS | RITINPDTSKNQFSLQL<br>NSVTPEDTAVYYCAR | GYCSGGSCYS--------<br>---YYYYYGMDV | WGQGTT<br>VTVSS |
| iPS:39 2648 | 21-225_16D11 | VH6\|6-01/D2\|2-15\|RF2/JH6 | ............<br>............<br>........ | R....T...S | ........<br>...... | ........<br>........<br>.... | .................<br>............... | VNSGWSHH--<br>...........-<br>........ | ......<br>..... |
| | | Germline | **H_FR1** | **H_CDR1** | **H_FR2** | **H_CDR2** | **H_FR3** | **H_CDR3** | **H_FR4** |
| | VH3\|3-11/D1\|1-1\|RF3/JH2 | | QVQLVES-<br>GGGLVKPGGSLR<br>LSCAASG-FTFS | D-----YYMS | WIRQAPGK<br>GLEWVS | YISSS---<br>GSTIYYAD<br>SVKG | RFTISRDNAKNSLYLQM<br>NSLRAEDTAVYYCAR | YNWNDY------------<br>-------WYFDL | WGRGTL<br>VTVSS |

| | | | | | | | | | |
|---|---|---|---|---|---|---|---|---|---|
| iPS:392650 | 21-225_17A4 | VH3\|3-11/D1\|1-1\|RF3/JH2 | ............ ............ ........ | ........... | ........ ...... | H....... ........ .... | ................. ............... | .RN.R- .................. .G.... | ...... ..... |
| iPS:392728 | 21-225_20F7 | VH3\|3-11/D1\|1-1\|RF3/JH2 | ............ ............ ........ | ........... | ........ ...... | H....... ........ .... | ........GE....... ............... | .RN.R- .................. .G.... | ....S. ..... |
| | | Germline | **H_FR1** | **H_CDR1** | **H_FR2** | **H_CDR2** | **H_FR3** | **H_CDR3** | **H_FR4** |
| | VH4\|4-59/D1\|1-26\|RF3/JH4 | | QVQLQES- GPGLVKPSETLS LTCTVSG-GSIS | S-----YYWS | WIRQPPGK GLEWIG | YIYY---- SGSTNYNP SLKS | RVTISVDTSKNQFSLKL SSVTAADTAVYYCAR | YSGSY------------ -------YYFDY | WGQGTL VTVSS |
| iPS:392676 | 21-225_19F3 | VH4\|4-59/D1\|1-26\|RF3/JH4 | ............ ............ .....A.. | GS...S...G | ........ Q..... | N....... ....Y... .F.. | ................. .....E......... | H.S.W............ .......-SL.. | ...... ..... |
| | | Germline | **H_FR1** | **H_CDR1** | **H_FR2** | **H_CDR2** | **H_FR3** | **H_CDR3** | **H_FR4** |
| | VH3\|3-23/D6\|6-13\|RF2/JH2 | | EVQLLES- GGGLVQPGGSLR LSCAASG-FTFS | S-----YAMS | WVRQAPGK GLEWVS | AISGS--- GGSTYYAD SVKG | RFTISRDNSKNTLYLQM NSLRAEDTAVYYCAK | GIAAAG----------- ------YWYFDL | WGRGTL VTVSS |
| iPS:392678 | 21-225_20F3 | VH3\|3-23/D6\|6-13\|RF2/JH2 | ............ ............ ........ | ..........N | ........ ...... | V....... ........ .... | ................. ............... | R................ ......TE.... | ...... ..... |
| | | Germline | **H_FR1** | **H_CDR1** | **H_FR2** | **H_CDR2** | **H_FR3** | **H_CDR3** | **H_FR4** |
| | VH3\|3-21/D5\|5-24\|RF3/JH4 | | EVQLVES- GGGLVKPGGSLR LSCAASG-FTFS | S-----YSMN | WVRQAPGK GLEWVS | SISSS--- SSYIYYAD SVKG | RFTISRDNAKNSLYLQM NSLRAEDTAVYYCAR | RDGYN------------ -------YYFDY | WGQGTL VTVSS |
| iPS:392682 | 21-225_16A12 | VH3\|3-21/D5\|5-24\|RF3/JH4 | ............ ............ ........ | .......R.. | .F...... ...... | ...G.... .TD..... .... | .........E....... ............... | ..--- .................. ..----F | ...... ..... |
| | | Germline | **H_FR1** | **H_CDR1** | **H_FR2** | **H_CDR2** | **H_FR3** | **H_CDR3** | **H_FR4** |
| | VH3\|3-48/D7\|7-27\|RF1/JH4 | | EVQLVES- GGGLVQPGGSLR LSCAASG-FTFS | S-----YSMN | WVRQAPGK GLEWVS | YISSS--- SSTIYYAD SVKG | RFTISRDNAKNSLYLQM NSLRDEDTAVYYCAR | LTGY------------- ---------FDY | WGQGTL VTVSS |
| iPS:392692 | 21-225_18G10 | VH3\|3-48/D7\|7-27\|RF1/JH4 | ............ .........L.. ....I... | T......... | ........ ...... | ...R.... ....D... .... | ................. ............... | GG.S............. ........P... | ...... ..... |
| iPS:392708 | 21-225_18D11 | VH3\|3-48/D7\|7-27\|RF1/JH4 | ............ ............ .......R | ........... | ........ ....L. | ........ .G...... .... | .........R...N... ............... | GG.S............. ........P... | ....I. ..... |

| | | | | | | | | | |
|---|---|---|---|---|---|---|---|---|---|
| iPS:39 3992 | 21-225_14H8 | VH3\|3-48/D7\|7-27\|RF1/JH4 | ............. ............. ........ | .......N... | ......... ...... | ......... ......... .... | ....A............ ................ | GG.S............... ........P... | ...... ..... |
| iPS:39 4055 | 21-225_9C8 | VH3\|3-48/D7\|7-27\|RF1/JH4 | ............. ............V V....... | .......Q... | ......... ...... | ...I- ......... ........ | ................. ................ | GG.S............... ........P..S | ...... ..... |
| | | Germline | H_FR1 | H_CDR1 | H_FR2 | H_CDR2 | H_FR3 | H_CDR3 | H_FR4 |
| | VH3\|3-30.3/D4\|4-11\|RF2/JH4 | | QVQLVES- GGGVVQPGRSLR LSCAASG-FTFS | S-----YAMH | WVRQAPGK GLEWVA | VISYD--- GSNKYYAD SVKG | RFTISRDNSKNTLYLQM NSLRAEDTAVYYCAR | DYSNY------------- --------YFDY | WGQGTL VTVSS |
| iPS:39 2694 | 21-225_19A5 | VH3\|3-30.3/D4\|4-11\|RF2/JH4 | ............. ............. ........ | ........... | ......... ...... | ..WF.... ..D..... .... | ................. ................ | .RAYS.............. .......SSS.. | ...... ..... |
| | | Germline | H_FR1 | H_CDR1 | H_FR2 | H_CDR2 | H_FR3 | H_CDR3 | H_FR4 |
| | VH3\|3-23/D1\|1-1\|RF1/JH4 | | EVQLLES- GGGLVQPGGSLR LSCAASG-FTFS | S-----YAMS | WVRQAPGK GLEWVS | AISGS--- GGSTYYAD SVKG | RFTISRDNSKNTLYLQM NSLRAEDTAVYYCAK | GTTGT------------- --------YFDY | WGQGTL VTVSS |
| iPS:39 2714 | 21-225_16G12 | VH3\|3-23/D1\|1-1\|RF1/JH4 | ............. ............. .....S.. | ..........T | ......... ...... | T...R... ..H..... ..R. | ..A....S......... ................ | QD--- .................. ...---C | ...... ..... |
| iPS:39 2890 | 21-225_20H9 | VH3\|3-23/D1\|1-1\|RF1/JH4 | ............. ............. ........ | ........... | ......... ...... | ......... ..Y..... .... | .........E....... S............. | .GS-- .................. ...-LF. | ...... ..... |
| iPS:39 2892 | 21-225_20C11 | VH3\|3-23/D1\|1-1\|RF1/JH4 | ............. ............. .....S.. | ........... | ......... ...... | T...R... ..H..... .... | ..A....S......... ................ | QD--- .................. ...---C | ...... ..... |
| iPS:39 3968 | 21-225_5A5 | VH3\|3-23/D1\|1-1\|RF1/JH4 | ....W........ ............. ........ | ..........N | ......... ...... | ......... ..Y..... .... | ................. S............. | .GS-- .................. ...-LF. | ...... ..... |
| | | Germline | H_FR1 | H_CDR1 | H_FR2 | H_CDR2 | H_FR3 | H_CDR3 | H_FR4 |
| | VH3\|3-23/D6\|6-6\|RF1/JH3 | | EVQLLES- GGGLVQPGGSLR LSCAASG-FTFS | S-----YAMS | WVRQAPGK GLEWVS | AISGS--- GGSTYYAD SVKG | RFTISRDNSKNTLYLQM NSLRAEDTAVYYCAK | EYSSSS------------ -------DAFDI | WGQGTM VTVSS |
| iPS:39 2730 | 21-225_17A1 | VH3\|3-23/D6\|6-6\|RF1/JH3 | ............. ............. ........ | ..........N | ......... ...... | V....... .SN..... .... | ................. ................ | R.T.DW............ ......H..... | ...... ..... |

| | | | | | | | | | |
|---|---|---|---|---|---|---|---|---|---|
| iPS:39 2736 | 21-225_17B12 | VH3\|3-23/D6\|6-6\|RF1/JH3 | ............<br>...........V<br>........ | .........N | ........<br>...... | V.......<br>........<br>.... | .................<br>.T............. | R.T.DW...........<br>......H..... | ......<br>..... |
| iPS:39 2770 | 21-225_20C10 | VH3\|3-23/D6\|6-6\|RF1/JH3 | ............<br>............<br>........ | .........N | ........<br>...... | V.......<br>..T.....<br>.... | .................<br>.T............. | R.T.DW...........<br>......H..... | ......<br>..... |
| iPS:39 3878 | 21-225_7G12 | VH3\|3-23/D6\|6-6\|RF1/JH3 | ............<br>............<br>........ | .........N | ........<br>...... | V.......<br>........<br>.... | .................<br>.T............. | R.T.DW...........<br>......H..... | ......<br>..... |
| | | Germline | **H_FR1** | **H_CDR1** | **H_FR2** | **H_CDR2** | **H_FR3** | **H_CDR3** | **H_FR4** |
| | VH3\|3-21/D7\|7-27\|RF3/JH5 | | EVQLVES-<br>GGGLVKPGGSLR<br>LSCAASG-FTFS | S-----YSMN | WVRQAPGK<br>GLEWVS | SISSS---<br>SSYIYYAD<br>SVKG | RFTISRDNAKNSLYLQM<br>NSLRAEDTAVYYCAR | NWGN------------<br>--------WFDP | WGQGTL<br>VTVSS |
| iPS:39 2748 | 21-225_20A8 | VH3\|3-21/D7\|7-27\|RF3/JH5 | ............<br>............<br>........ | ........V. | ...R....<br>...... | ........<br>..FL....<br>.... | ............V....<br>............... | ..--<br>..................<br>....--.Y | ......<br>..... |
| | | Germline | **H_FR1** | **H_CDR1** | **H_FR2** | **H_CDR2** | **H_FR3** | **H_CDR3** | **H_FR4** |
| | VH3\|3-30.3/D1\|1-1\|RF1/JH5 | | QVQLVES-<br>GGGVVQPGRSLR<br>LSCAASG-FTFS | S-----YAMH | WVRQAPGK<br>GLEWVA | VISYD---<br>GSNKYYAD<br>SVKG | RFTISRDNSKNTLYLQM<br>NSLRAEDTAVYYCAR | GTTGT-----------<br>-------NWFDP | WGQGTL<br>VTVSS |
| iPS:39 2754 | 21-225_21D3 | VH3\|3-30.3/D1\|1-1\|RF1/JH5 | ............<br>...........V<br>........ | .......G.. | ........<br>.....T | ........<br>........<br>.... | .................<br>............... | .VWF-<br>..................<br>..--G.L | ......<br>..... |
| iPS:39 2818 | 21-225_22D8 | VH3\|3-30.3/D1\|1-1\|RF1/JH5 | ............<br>...........V<br>.......R | .......G.. | ........<br>.....T | I.......<br>........<br>.... | .................<br>...........F... | .VWF-<br>..................<br>..--G.F | ......<br>..... |
| | | Germline | **H_FR1** | **H_CDR1** | **H_FR2** | **H_CDR2** | **H_FR3** | **H_CDR3** | **H_FR4** |
| | VH3\|3-23/D6\|6-19\|RF2/JH1 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | GIAVAG----------<br>------AEYFQH | WGQGTL<br>VTVSS |
| iPS:39 2762 | 21-225_22G5 | VH3\|3-23/D6\|6-19\|RF2/JH1 | ............<br>............<br>........ | .........N | .......M<br>...... | V..R....<br>..Y.....<br>.... | .................<br>..............S | RL...............<br>......S.A.DI | ......<br>..... |
| iPS:39 4051 | 21-225_9E5 | VH3\|3-23/D6\|6-19\|RF2/J | ............<br>............<br>........ | N........N | ........<br>...... | ....G...<br>..N.F...<br>.... | .................<br>.G............S | R................<br>......S.A.AI | ......<br>..... |

| | | Germline | H_FR1 | H_CDR1 | H_FR2 | H_CDR2 | H_FR3 | H_CDR3 | H_FR4 |
|---|---|---|---|---|---|---|---|---|---|
| | VH4\|4-39/D1\|1-26\|RF3/JH1 | | QLQLQES- GPGLVKPSETLS LTCTVSG-GSIS | SS---SYYWG | WIRQPPGK GLEWIG | SIYY---- SGSTYYNP SLKS | RVTISVDTSKNQFSLKL SSVTAADTAVYYCAR | YSGSYY----------- ------AEYFQH | WGQGTL VTVSS |
| iPS:392774 | 21-225_21F3 | VH4\|4-39/D1\|1-26\|RF3/JH1 | ............ ...A........ ........ | R......... | ........ V..... | ........ ........ .... | ................. .........E....S | L.S.W- .................. --D... | ...... ..... |
| iPS:393962 | 21-225_7H7 | VH4\|4-39/D1\|1-26\|RF3/JH1 | ............ ............ ........ | R......... | ........ ...... | N....... ......I. .... | ................. ............... | H.T.W- .................. --SLD. | ...... ..... |
| iPS:394049 | 21-225_13H5 | VH4\|4-39/D1\|1-26\|RF3/JH1 | ............ ...A........ ........ | R......... | ........ ...... | ........ ........ .... | ................. ..............S | L.S.W- .................. --D... | ...... ..... |
| | | Germline | H_FR1 | H_CDR1 | H_FR2 | H_CDR2 | H_FR3 | H_CDR3 | H_FR4 |
| | VH3\|3-21/D2\|2-21\|RF2/JH4 | | EVQLVES- GGGLVKPGGSLR LSCAASG-FTFS | S-----YSMN | WVRQAPGK GLEWVS | SISSS--- SSYIYYAD SVKG | RFTISRDNAKNSLYLQM NSLRAEDTAVYYCAR | AYCGGDC---------- ------YSYFDY | WGQGTL VTVSS |
| iPS:392776 | 21-225_21A12 | VH3\|3-21/D2\|2-21\|RF2/JH4 | ............ ............ .......N | .......... | ........ ...... | ...G.... ........ .... | ................. .........L..... | .AG---- .................- --... | ...... ..... |
| | | Germline | H_FR1 | H_CDR1 | H_FR2 | H_CDR2 | H_FR3 | H_CDR3 | H_FR4 |
| | VH3\|3-33/D6\|6-19\|RF2/JH6 | | QVQLVES- GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | GIAVAGYY--------- -----YYYGMDV | WGQGTT VTVSS |
| iPS:392806 | 21-225_24H3 | VH3\|3-33/D6\|6-19\|RF2/JH6 | ............ ...........G ........ | .......... | ........ ...... | ........ ........ .... | ................. ............... | VAVAG--- ...............--- .... | ...... ..... |
| | | Germline | H_FR1 | H_CDR1 | H_FR2 | H_CDR2 | H_FR3 | H_CDR3 | H_FR4 |
| | VH3\|3-48/D7\|7-27\|RF2/JH4 | | EVQLVES- GGGLVQPGGSLR LSCAASG-FTFS | S-----YSMN | WVRQAPGK GLEWVS | YISSS--- SSTIYYAD SVKG | RFTISRDNAKNSLYLQM NSLRDEDTAVYYCAR | *LGY------------- ---------FDY | WGQGTL VTVSS |
| iPS:392826 | 21-225_20B9 | VH3\|3-48/D7\|7-27\|RF2/JH4 | ............ ............ ........ | .......... | ........ ...... | ........ ........ .... | ................. ............... | S.WS............. ........P... | ...... ..... |
| | | Germline | H_FR1 | H_CDR1 | H_FR2 | H_CDR2 | H_FR3 | H_CDR3 | H_FR4 |
| | VH4\|4-59/D1\|1-1\|RF1/JH5 | | QVQLQES- GPGLVKPSETLS LTCTVSG-GSIS | S-----YYWS | WIRQPPGK GLEWIG | YIYY---- SGSTNYNP SLKS | RVTISVDTSKNQFSLKL SSVTAADTAVYYCAR | GTTGT------------ -------NWFDP | WGQGTL VTVSS |

| | | | | | | | | | |
|---|---|---|---|---|---|---|---|---|---|
| iPS:39 2830 | 21-225_21A5 | VH4\|4-59/D1\|1-1\|RF1/JH5 | ............. ............. ........ | .......F.. | .....A.. ...... | R..T.... ..I..... .... | ...M............. .........I..... | .P.SG............. .......-.... | ...... ..... |
| | | Germline | H_FR1 | H_CDR1 | H_FR2 | H_CDR2 | H_FR3 | H_CDR3 | H_FR4 |
| | VH3\|3-23/D6\|6-6\|RF2/JH4 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | SIAAR------------------------YFDY | WGQGTLVTVSS |
| iPS:39 2840 | 21-225_23G1 | VH3\|3-23/D6\|6-6\|RF2/JH4 | ............. ............. ........ | .......... | .......R ...... | V....... ..T..NT. .... | ................. ..............R | .SL-- .................. ...-... | ...... ..... |
| iPS:39 4018 | 21-225_15B1 | VH3\|3-23/D6\|6-6\|RF2/JH4 | ............. ............. T....... | .......V.. | ........ ...... | G....... ....NN.. .... | ................V ..............R | .SL-- .................. ...-... | ...... ..... |
| iPS:39 4026 | 21-225_16C7 | VH3\|3-23/D6\|6-6\|RF2/JH4 | ............. ............. ........ | .......V.T | ........ ...... | T....... ..W..... .... | ................. .........E....R | .SL-- .................. ...-... | ...... ..... |
| | | Germline | H_FR1 | H_CDR1 | H_FR2 | H_CDR2 | H_FR3 | H_CDR3 | H_FR4 |
| | VH3\|3-23/D6\|6-6\|RF2/JH5 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | SIAAR-----------------------NWFDP | WGQGTLVTVSS |
| iPS:39 2842 | 21-225_23G8 | VH3\|3-23/D6\|6-6\|RF2/JH5 | ............. ............. ........ | .......... | ........ ...... | ........ ........ .... | ................. ..............V | .SG-- .................. ..--WFA | ...... ..... |
| | | Germline | H_FR1 | H_CDR1 | H_FR2 | H_CDR2 | H_FR3 | H_CDR3 | H_FR4 |
| | VH3\|3-23/D1\|1-1\|RF2/JH2 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | VQLERY-----------------------WYFDL | WGRGTLVTVSS |
| iPS:39 2856 | 21-225_22A2 | VH3\|3-23/D1\|1-1\|RF2/JH2 | ............. ............. ........ | .......... | ........ ...... | G....... ..N.P... .... | .......I......... ............... | .VG--- .................. .--AVH | ...... ..... |
| | | Germline | H_FR1 | H_CDR1 | H_FR2 | H_CDR2 | H_FR3 | H_CDR3 | H_FR4 |
| | VH4\|4-30.1/D5\|5-18\|RF1/JH6 | | QVQLQES-GPGLVKPSQTLSLTCTVSG-GSIS | SG---GYYWS | WIRQHPGKGLEWIG | YIYY----SGSTYYNPSLKS | RVTISVDTSKNQFSLKLSSVTAADTAVYYCAR | VDTAMVYY-----------------YYYGMDV | WGQGTTVTVSS |
| iPS:39 2864 | 21-225_23B9 | VH4\|4-30.1/D5\|5-18\|RF1/JH6 | .....A...... ............. ..D..... | .......... | ........ ...... | ........ ........ .... | ................. ............... | E.G.FG-- ...............- ...... | ...... ..... |

| | | | | | | | | | |
|---|---|---|---|---|---|---|---|---|---|
| | VH3\|3-23/D2\|2-2\|RF2/JH3 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | GYCSSTSC----------------YTDAFDI | WGQGTMVTVSS |
| iPS:392874 | 21-225_21D2 | VH3\|3-23/D2\|2-2\|RF2/JH3 | ..K.....................................N | N......... | .............. | VL........F....... | ................S....G.....F..R | YCS.ARC-...............PY..... | ........... |
| iPS:393940 | 21-225_16B2 | VH3\|3-23/D2\|2-2\|RF2/JH3 | ................................ | .........T | ........P.... | V........F....... | ................S.........F..R | YCS.TRC-...............PY..... | ........... |
| iPS:393956 | 21-225_4D7 | VH3\|3-23/D2\|2-2\|RF2/JH3 | ..K............................. | .......... | .............. | VL........F....... | ................S....G.....F..R | YCS.ARC-...............PY..... | ........... |
| | | | | | | | | | |
| iPS:398476 | 21-225_17C1 | VH3\|3-23/D2\|2-2\|RF2/JH3 | ............E.................. | .......... | .............. | V........T.F....... | ................S.........F..R | YCS.TRC-...............PY..... | ........... |
| | | Germline | H_FR1 | H_CDR1 | H_FR2 | H_CDR2 | H_FR3 | H_CDR3 | H_FR4 |
| | VH3\|3-15/D1\|1-1\|RF3/JH4 | | EVQLVES-GGGLVKPGGSLRLSCAASG-FTFS | N-----AWMS | WVRQAPGKGLEWVG | RIKSKT-DGGTTDYAAPVKG | RFTISRDDSKNTLYLQMNSLKTEDTAVYYCTT | YNWND----------------YFDY | WGQGTLVTVSS |
| iPS:392898 | 21-225_21H10 | VH3\|3-15/D1\|1-1\|RF3/JH4 | ................................ | .........N | .............. | .................... | ................................ | EG..-...................-T.. | ........... |
| iPS:393802 | 21-225_3D12 | VH3\|3-15/D1\|1-1\|RF3/JH4 | ............................V... | T........N | .............. | ...N.I.......V..... | ................................ | EG..-...................-T.. | ........... |
| | | Germline | H_FR1 | H_CDR1 | H_FR2 | H_CDR2 | H_FR3 | H_CDR3 | H_FR4 |
| | VH3\|3-48/D4\|4-11\|RF3/JH4 | | EVQLVES-GGGLVQPGGSLRLSCAASG-FTFS | S-----YSMN | WVRQAPGKGLEWVS | YISSS---SSTIYYADSVKG | RFTISRDNAKNSLYLQMNSLRDEDTAVYYCAR | TTVT----------------YFDY | WGQGTLVTVSS |
| iPS:392950 | 21-225_25C10 | VH3\|3-48/D4\|4-11\|RF3/JH4 | ................................ | .......R.. | .............. | S................... | ................................ | .AG-...................-... | ........... |
| | | Germline | H_FR1 | H_CDR1 | H_FR2 | H_CDR2 | H_FR3 | H_CDR3 | H_FR4 |
| | VH3\|3-23/D1\|1-26\|RF3/JH4 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | YSGSY---------------YYFDY | WGQGTLVTVSS |

| | | | | | | | | | |
|---|---|---|---|---|---|---|---|---|---|
| iPS:39 3010 | 21-225_25E11 | VH3\|3-23/D1\|1-26\|RF3/JH4 | ............ ............ ........ | .......... | ........ ...... | V...G... ........ .... | ................. ............... | RGY.GYE........... .....DLL...C | ...... ..... |
| | | Germline | H_FR1 | H_CDR1 | H_FR2 | H_CDR2 | H_FR3 | H_CDR3 | H_FR4 |
| | VH3\|3-23/D3\|3-3\|RF3/JH3 | | EVQLLES- GGGLVQPGGSLR LSCAASG-FTFS | S-----YAMS | WVRQAPGK GLEWVS | AISGS--- GGSTYYAD SVKG | RFTISRDNSKNTLYLQM NSLRAEDTAVYYCAK | ITIFGVV----------- -----IIDAFDI | WGQGTM VTVSS |
| iPS:39 3016 | 21-225_28F11 | VH3\|3-23/D3\|3-3\|RF3/JH3 | ............ ............ .....I.. | .......... | ........ ...... | VT...... ..T.F... .... | ................. ............... | R.Q.D-- ................-- -D... | ...... ..... |
| | | Germline | H_FR1 | H_CDR1 | H_FR2 | H_CDR2 | H_FR3 | H_CDR3 | H_FR4 |
| | VH3\|3-33/D3\|3-22\|RF2/JH6 | | QVQLVES- GGGVVQPGRSLR LSCAASG-FTFS | S-----YGMH | WVRQAPGK GLEWVA | VIWYD--- GSNKYYAD SVKG | RFTISRDNSKNTLYLQM NSLRAEDTAVYYCAR | YYYDSSGYYY-------- ---YYYYYGMDV | WGQGTT VTVSS |
| iPS:39 3032 | 21-225_26F8 | VH3\|3-33/D3\|3-22\|RF2/JH6 | ............ ............ ........ | G......... | ........ ...... | I....... ........ .... | ................. ............... | ER..FW---- ...........--- SGC... | ...... ..... |
| | | Germline | H_FR1 | H_CDR1 | H_FR2 | H_CDR2 | H_FR3 | H_CDR3 | H_FR4 |
| | VH1\|1-02/D4\|4-11\|RF2/JH6 | | QVQLVQS- GAEVKKPGASVK VSCKASG-YTFT | G-----YYMH | WVRQAPGQ GLEWMG | WINPN--- SGGTNYAQ KFQG | RVTMTRDTSISTAYMEL SRLRSDDTAVYYCAR | DYSNYYY----------- -----YYYGMDV | WGQGTT VTVSS |
| iPS:39 3042 | 21-225_31F1 | VH1\|1-02/D4\|4-11\|RF2/JH6 | ............ ............ ........ | D......... | ........ ...... | ........ ........ .... | ..........M...... ............... | .S..FSNW.......... ....YD...... | ...... ..... |
| iPS:39 3108 | 21-225_34G11 | VH1\|1-02/D4\|4-11\|RF2/JH6 | ............ ............ ........ | .......... | ........ ...... | ........ ........ .... | ................. ............... | .I..FSSW.......... ....YD..A... | ...... ..... |
| | | Germline | H_FR1 | H_CDR1 | H_FR2 | H_CDR2 | H_FR3 | H_CDR3 | H_FR4 |
| | VH1\|1-18/D5\|5-12\|RF3/JH4 | | QVQLVQS- GAEVKKPGASVK VSCKASG-YTFT | S-----YGIS | WVRQAPGQ GLEWMG | WISAY--- NGNTNYAQ KLQG | RVTMTTDTSTSTAYMEL RSLRSDDTAVYYCAR | GYSGYD------------ -------YYFDY | WGQGTL VTVSS |
| iPS:39 3044 | 21-225_25B8 | VH1\|1-18/D5\|5-12\|RF3/JH4 | ............ ............ ........ | .......... | ........ ...... | ........ ....T... ..R. | ...............D. ............... | TAA..S........... ......SSW... | ...... ..... |
| iPS:39 3050 | 21-225_28C5 | VH1\|1-18/D5\|5-12\|RF3/JH4 | ............ ............ ..D..... | .......... | ........ ...... | ........ ....T... ..R. | ...............D. ............... | TAA..S........... ......SSW... | ...... ..... |
| | | Germline | H_FR1 | H_CDR1 | H_FR2 | H_CDR2 | H_FR3 | H_CDR3 | H_FR4 |

| | | | | | | | | | |
|---|---|---|---|---|---|---|---|---|---|
| | VH3\|3-33/D6\|6-6\|RF1/JH3 | | QVQLVES-<br>GGGVVQPGRSLR<br>LSCAASG-FTFS | S-----YGMH | WVRQAPGK<br>GLEWVA | VIWYD---<br>GSNKYYAD<br>SVKG | RFTISRDNSKNTLYLQM<br>NSLRAEDTAVYYCAR | EYSSSS-----------<br>-------DAFDI | WGQGTM<br>VTVSS |
| iPS:39 3046 | 21-225_25A12 | VH3\|3-33/D6\|6-6\|RF1/JH3 | ............<br>............<br>........ | N.....CV.. | ........<br>...... | ........<br>........<br>.... | .................<br>............... | .EY..GW..........<br>.....YDYGM.V | ......<br>..... |
| | | Germline | H_FR1 | H_CDR1 | H_FR2 | H_CDR2 | H_FR3 | H_CDR3 | H_FR4 |
| | VH3\|3-21/D4\|4-11\|RF3/JH6 | | EVQLVES-<br>GGGLVKPGGSLR<br>LSCAASG-FTFS | S-----YSMN | WVRQAPGK<br>GLEWVS | SISSS---<br>SSYIYYAD<br>SVKG | RFTISRDNAKNSLYLQM<br>NSLRAEDTAVYYCAR | TTVTYYY----------<br>------YYGMDV | WGQGTT<br>VTVSS |
| iPS:39 3078 | 21-225_33H11 | VH3\|3-21/D4\|4-11\|RF3/JH6 | ............<br>............<br>........ | .......... | ........<br>...... | ...G....<br>........<br>.... | .................<br>S.............. | .NG----<br>.................-<br>--... | ......<br>..... |
| iPS:39 3142 | 21-225_33A3 | VH3\|3-21/D4\|4-11\|RF3/JH6 | ............<br>............<br>........ | .......G.. | ........<br>...... | ...G....<br>.T......<br>.... | .................<br>............... | .NG----<br>.................-<br>--... | ......<br>..... |
| | | Germline | H_FR1 | H_CDR1 | H_FR2 | H_CDR2 | H_FR3 | H_CDR3 | H_FR4 |
| | VH3\|3-23/D6\|6-19\|RF2/JH6 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | GIAVAGYY---------<br>-----YYYGMDV | WGQGTT<br>VTVSS |
| iPS:39 3096 | 21-225_34D11 | VH3\|3-23/D6\|6-19\|RF2/JH6 | ....S.......<br>............<br>........ | .........N | ........<br>...... | ....R...<br>....FH..<br>.... | .................<br>.............V. | .EL.ED.-<br>................F.<br>.... | ......<br>..... |
| | | Germline | H_FR1 | H_CDR1 | H_FR2 | H_CDR2 | H_FR3 | H_CDR3 | H_FR4 |
| | VH3\|3-30.3/D4\|4-23\|RF2/JH3 | | QVQLVES-<br>GGGVVQPGRSLR<br>LSCAASG-FTFS | S-----YAMH | WVRQAPGK<br>GLEWVA | VISYD---<br>GSNKYYAD<br>SVKG | RFTISRDNSKNTLYLQM<br>NSLRAEDTAVYYCAR | DYGGNS-----------<br>-------DAFDI | WGQGTM<br>VTVSS |
| iPS:39 3172 | 21-225_3B12 | VH3\|3-30.3/D4\|4-23\|RF2/JH3 | ..H.........<br>............<br>........ | Y......G.. | ........<br>...... | ........<br>........<br>.... | .............H...<br>............... | .RR.GYG..........<br>.....VP..... | ......<br>..... |
| | | Germline | H_FR1 | H_CDR1 | H_FR2 | H_CDR2 | H_FR3 | H_CDR3 | H_FR4 |
| | VH3\|3-30.3/D4\|4-23\|RF2/JH6 | | QVQLVES-<br>GGGVVQPGRSLR<br>LSCAASG-FTFS | S-----YAMH | WVRQAPGK<br>GLEWVA | VISYD---<br>GSNKYYAD<br>SVKG | RFTISRDNSKNTLYLQM<br>NSLRAEDTAVYYCAR | DYGGNSYY---------<br>-----YYYGMDV | WGQGTT<br>VTVSS |
| iPS:39 3174 | 21-225_15D8 | VH3\|3-30.3/D4\|4-23\|RF2/J | ............<br>..T.........<br>........ | G......G.. | ........<br>.....S | ........<br>........<br>.... | .................<br>............... | .RVYC.STSCV.......<br>..PYYD...... | ......<br>..... |

| | | | | | | | | | |
|---|---|---|---|---|---|---|---|---|---|
| | VH3\|3-15/D7\|7-27\|RF1/JH6 | | EVQLVES-<br>GGGLVKPGGSLR<br>LSCAASG-FTFS | N-----AWMS | WVRQAPGK<br>GLEWVG | RIKSKT-<br>DGGTTDYA<br>APVKG | RFTISRDDSKNTLYLQM<br>NSLKTEDTAVYYCTT | LTGYYY-----------<br>------YYGMDV | WGQGTT<br>VTVSS |
| iPS:39 3194 | 21-225_16D2 | VH3\|3-15/D7\|7-27\|RF1/JH6 | ............<br>............<br>........ | .........N | ........<br>...... | ........<br>........<br>.... | ................<br>H............. | D..PIAARLA........<br>...YYY..A... | ......<br>..... |
| iPS:39 8488 | 21-225_19F6 | VH3\|3-15/D7\|7-27\|RF1/JH6 | ............<br>............<br>........ | .........N | ........<br>...... | ........<br>........<br>.... | ................<br>.............. | D..PIAARLA........<br>...YYY..A... | ..H...<br>..... |
| iPS:39 8544 | 21-225_7C8 | VH3\|3-15/D7\|7-27\|RF1/JH6 | ............<br>............<br>........ | .......R.N | ...L....<br>...... | ........<br>........<br>.... | .......E.E.......<br>........G....S. | D..PIAARLA........<br>...YYY..A... | ......<br>..... |
| iPS:40 2231 | 21-225_6D9 | VH3\|3-15/D7\|7-27\|RF1/JH6 | ............<br>............<br>........ | .........N | ...L....<br>...... | ........<br>........<br>.... | .........E.......<br>.............S. | D..PIAARLA........<br>...YYY..A... | ......<br>..... |
| | | Germline | H_FR1 | H_CDR1 | H_FR2 | H_CDR2 | H_FR3 | H_CDR3 | H_FR4 |
| | VH3\|3-23/D4\|4-11\|RF2/JH4 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | DYSNY------------<br>--------YFDY | WGQGTL<br>VTVSS |
| iPS:39 3870 | 21-225_7B1 | VH3\|3-23/D4\|4-11\|RF2/JH4 | ............<br>............<br>........ | .......D.. | ........<br>...... | T.......<br>..I.....<br>.... | ..........K......<br>...........F..R | .RG--<br>..................<br>...--SV | ......<br>..... |
| | | Germline | H_FR1 | H_CDR1 | H_FR2 | H_CDR2 | H_FR3 | H_CDR3 | H_FR4 |
| | VH4\|4-39/D4\|4-17\|RF2/JH1 | | QLQLQES-<br>GPGLVKPSETLS<br>LTCTVSG-GSIS | SS---SYYWG | WIRQPPGK<br>GLEWIG | SIYY----<br>SGSTYYNP<br>SLKS | RVTISVDTSKNQFSLKL<br>SSVTAADTAVYYCAR | DYGDYA-----------<br>-------EYFQH | WGQGTL<br>VTVSS |
| iPS:39 3872 | 21-225_2A11 | VH4\|4-39/D4\|4-17\|RF2/JH1 | ............<br>............<br>........ | R......... | ........<br>...... | N.......<br>........<br>.V.. | ................<br>.T............. | HGK.W-<br>..................<br>.-GLED | ......<br>..... |
| | | Germline | H_FR1 | H_CDR1 | H_FR2 | H_CDR2 | H_FR3 | H_CDR3 | H_FR4 |
| | VH4\|4-59/D6\|6-6\|RF1/JH4 | | QVQLQES-<br>GPGLVKPSETLS<br>LTCTVSG-GSIS | S-----YYWS | WIRQPPGK<br>GLEWIG | YIYY----<br>SGSTNYNP<br>SLKS | RVTISVDTSKNQFSLKL<br>SSVTAADTAVYYCAR | EYSSS------------<br>-------SYFDY | WGQGTL<br>VTVSS |
| iPS:39 3890 | 21-225_4B1 | VH4\|4-59/D6\|6-6\|RF1/JH4 | ............<br>...........S<br>....D... | .......S.. | .....A..<br>...... | R..T....<br>......I.<br>.... | .I.M......K......<br>............... | DLK..G...........<br>......CLF... | ......<br>..... |
| | | Germline | H_FR1 | H_CDR1 | H_FR2 | H_CDR2 | H_FR3 | H_CDR3 | H_FR4 |

| | | | | | | | | | |
|---|---|---|---|---|---|---|---|---|---|
| | VH3\|3-33/D4\|4-17\|RF1/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | *LR*LYY-----------------YYYGMDV | WGQGTTVTVSS |
| iPS:393926 | 21-225_4G4 | VH3\|3-33/D4\|4-17\|RF1/JH6 | ............................... | .......... | .........G. | ...H............ | ........A....................... | D..MG--................--..... | ........... |
| | | Germline | H_FR1 | H_CDR1 | H_FR2 | H_CDR2 | H_FR3 | H_CDR3 | H_FR4 |
| | VH3\|3-23/D6\|6-6\|RF2/JH2 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | SIAARY-------------WYFDL | WGRGTLVTVSS |
| iPS:393936 | 21-225_14A11 | VH3\|3-23/D6\|6-6\|RF2/JH2 | ............................... | .........N | .............. | V...R............ | ............................... | R...GM............E.... | ........... |
| | | Germline | H_FR1 | H_CDR1 | H_FR2 | H_CDR2 | H_FR3 | H_CDR3 | H_FR4 |
| | VH3\|3-21/D1\|1-1\|RF3/JH4 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | YNWND---------------YFDY | WGQGTLVTVSS |
| iPS:393988 | 21-225_7F10 | VH3\|3-21/D1\|1-1\|RF3/JH4 | ............................... | .......... | .............. | .................... | ....T.......................... | A.L--..................-... | ........... |
| | | Germline | H_FR1 | H_CDR1 | H_FR2 | H_CDR2 | H_FR3 | H_CDR3 | H_FR4 |
| | VH4\|4-39/D6\|6-6\|RF1/JH4 | | QLQLQES-GPGLVKPSETLSLTCTVSG-GSIS | SS---SYYWG | WIRQPPGKGLEWIG | SIYY----SGSTYYNPSLKS | RVTISVDTSKNQFSLKLSSVTAADTAVYYCAR | EYSSS--------------SYFDY | WGQGTLVTVSS |
| iPS:393990 | 21-225_11G7 | VH4\|4-39/D6\|6-6\|RF1/JH4 | .........................F.. | R....T.... | .............. | ............S.S..... | ............................... | LN..W.................-S... | ........... |
| | | Germline | H_FR1 | H_CDR1 | H_FR2 | H_CDR2 | H_FR3 | H_CDR3 | H_FR4 |
| | VH3\|3-30.3/D2\|2-15\|RF3/JH6 | | QVQLVES-GGGVVQPGRSLRLSCAASG-FTFS | S-----YAMH | WVRQAPGKGLEWVA | VISYD---GSNKYYADSVKG | RFTISRDNSKNTLYLQMNSLRAEDTAVYYCAR | DIVVVVAAT--------------YYYYYGMDV | WGQGTTVTVSS |
| iPS:394010 | 21-225_12G5 | VH3\|3-30.3/D2\|2-15\|RF3/JH6 | ............................... | N......GIY | .............. | .................... | ............................... | .RGA.A.Y-............--....I.. | ........... |
| | | Germline | H_FR1 | H_CDR1 | H_FR2 | H_CDR2 | H_FR3 | H_CDR3 | H_FR4 |
| | VH1\|1-08/D2\|2-21\|RF1/JH4 | | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | SILWW*L-----------------LFYFDY | WGQGTLVTVSS |

| | | | | | | | | | |
|---|---|---|---|---|---|---|---|---|---|
| iPS:39 4065 | 21-225_11E2 | VH1\|1-08/D2\|2-21\|RF1/JH4 | ...V........<br>............<br>........ | N......... | ........<br>...... | ...T....<br>........<br>.... | ...............D.<br>..............Y | .HG.F--<br>..................-<br>-L... | ....I.<br>..... |
| iPS:39 8506 | 21-225_23G12 | VH1\|1-08/D2\|2-21\|RF1/JH4 | ....L.......<br>............<br>........ | N......... | ........<br>...... | ..Y.....<br>........<br>.... | .....M...........<br>..............I | .GG.Y--<br>..................-<br>-.... | ......<br>..... |
| iPS:39 8512 | 21-225_25E12 | VH1\|1-08/D2\|2-21\|RF1/JH4 | ............<br>............<br>........ | N......... | ........<br>...... | ........<br>........<br>.... | ..............L..<br>..............G | .NG.Y--<br>..................-<br>-.... | ......<br>..... |
| | | Germline | **H_FR1** | **H_CDR1** | **H_FR2** | **H_CDR2** | **H_FR3** | **H_CDR3** | **H_FR4** |
| | VH3\|3-33/D7\|7-27\|RF2/JH3 | | QVQLVES-<br>GGGVVQPGRSLR<br>LSCAASG-FTFS | S------YGMH | WVRQAPGK<br>GLEWVA | VIWYD---<br>GSNKYYAD<br>SVKG | RFTISRDNSKNTLYLQM<br>NSLRAEDTAVYYCAR | *LGD-------------<br>--------AFDI | WGQGTM<br>VTVSS |
| iPS:39 4091 | 21-225_13H3 | VH3\|3-33/D7\|7-27\|RF2/JH3 | ...........L<br>............<br>........ | .......... | ........<br>...... | ....E...<br>E.....V.<br>..R. | ..........S......<br>.............V. | E..F..............<br>........QS.F | .....P<br>..... |
| | | Germline | **H_FR1** | **H_CDR1** | **H_FR2** | **H_CDR2** | **H_FR3** | **H_CDR3** | **H_FR4** |
| | VH3\|3-23/D4\|4-23\|RF2/JH6 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | DYGGNSYY----------<br>-----YYYGMDV | WGQGTT<br>VTVSS |
| iPS:39 8472 | 21-225_16E4 | VH3\|3-23/D4\|4-23\|RF2/JH6 | ............<br>I...........<br>........ | .......V.. | ........<br>...... | T..VG...<br>..T.....<br>.... | .................<br>............... | WGR....E..........<br>............ | ......<br>..... |
| | | Germline | **H_FR1** | **H_CDR1** | **H_FR2** | **H_CDR2** | **H_FR3** | **H_CDR3** | **H_FR4** |
| | VH2\|2-05/D6\|6-19\|RF2/JH4 | | QITLKES-<br>GPTLVKPTQTLT<br>LTCTFSG-FSLS | TS---GVGVG | WIRQPPGK<br>ALEWLA | LIYW----<br>NDDKRYSP<br>SLKS | RLTITKDTSKNQVVLTM<br>TNMDPVDTATYYCAH | GIAVA-------------<br>-------GYFDY | WGQGTL<br>VTVSS |
| iPS:39 8498 | 21-225_22E6 | VH2\|2-05/D6\|6-19\|RF2/JH4 | ............<br>............<br>........ | .G........ | ........<br>...... | ........<br>........<br>.... | .....R...........<br>............... | T...R.............<br>.......-G... | ......<br>..... |
| | | Germline | **H_FR1** | **H_CDR1** | **H_FR2** | **H_CDR2** | **H_FR3** | **H_CDR3** | **H_FR4** |
| | VH1\|1-08/D5\|5-24\|RF2/JH4 | | QVQLVQS-<br>GAEVKKPGASVK<br>VSCKASG-YTFT | S-----YDIN | WVRQATGQ<br>GLEWMG | WMNPN---<br>SGNTGYAQ<br>KFQG | RVTMTRNTSISTAYMEL<br>SSLRSEDTAVYYCAR | *RWLQ-------------<br>-------LYFDY | WGQGTL<br>VTVSS |
| iPS:39 8536 | 21-225_33D12 | VH1\|1-08/D5\|5-24\|RF2/JH4 | ............<br>............<br>........ | .........S | ...L....<br>...... | ........<br>........<br>.... | .................<br>............... | K.A--<br>..................<br>..--N.. | ......<br>..... |
| | | Germline | **H_FR1** | **H_CDR1** | **H_FR2** | **H_CDR2** | **H_FR3** | **H_CDR3** | **H_FR4** |

| | | | | | | | | | |
|---|---|---|---|---|---|---|---|---|---|
| | VH2\|2-05/D6\|6-6\|RF1/JH4 | | QITLKES-GPTLVKPTQTLTLTCTFSG-FSLS | TS---GVGVG | WIRQPPGKALEWLA | LIYW----NDDKRYSPSLKS | RLTITKDTSKNQVVLTMTNMDPVDTATYYCAH | EYSSS------------------SYFDY | WGQGTLVTVSS |
| iPS:398546 | 21-225_9H10 | VH2\|2-05/D6\|6-6\|RF1/JH4 | ........................ .......T | .......... | .............. | ........S.......  .... | .................. ...A.......... | TG..C............ .......C.... | ...... ..... |
| | | Germline | H_FR1 | H_CDR1 | H_FR2 | H_CDR2 | H_FR3 | H_CDR3 | H_FR4 |
| | VH3\|3-21/D1\|1-1\|RF2/JH6 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | VQLERYY-----------------YYYGMDV | WGQGTTVTVSS |
| iPS:402229 | 21-225_16H9 | VH3\|3-21/D1\|1-1\|RF2/JH6 | ............ ............ ........ | .......... | ........ ...... | ...G.... ........ .... | ................ ............... | .NG---- ................-- --... | ...... ..... |
| | | Germline | H_FR1 | H_CDR1 | H_FR2 | H_CDR2 | H_FR3 | H_CDR3 | H_FR4 |
| | VH3\|3-21/D4\|4-11\|RF3/JH5 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | TTVTN-------------------WFDP | WGQGTLVTVSS |
| iPS:402233 | 21-225_16D10 | VH3\|3-21/D4\|4-11\|RF3/JH5 | ............ ............ ........ | T......NL. | ........ ...... | ...GG... AGH...S. .... | ................ ............... | .NG-- .................. ...-..F | ...... ..... |
| | | Germline | H_FR1 | H_CDR1 | H_FR2 | H_CDR2 | H_FR3 | H_CDR3 | H_FR4 |
| | VH3\|3-21/D1\|1-1\|RF1/JH3 | | EVQLVES-GGGLVKPGGSLRLSCAASG-FTFS | S-----YSMN | WVRQAPGKGLEWVS | SISSS---SSYIYYADSVKG | RFTISRDNAKNSLYLQMNSLRAEDTAVYYCAR | GTTGT-------------------DAFDI | WGQGTMVTVSS |
| iPS:402235 | 21-225_20F10 | VH3\|3-21/D1\|1-1\|RF1/JH3 | ............ ............ ........ | .......... | ........ ...... | ...T- ....TF.. ....... | ................ ............... | KAG-- .................. ..--L.. | ...... ..... |
| | | Germline | H_FR1 | H_CDR1 | H_FR2 | H_CDR2 | H_FR3 | H_CDR3 | H_FR4 |
| | VH3\|3-23/D5\|5-12\|RF1/JH3 | | EVQLLES-GGGLVQPGGSLRLSCAASG-FTFS | S-----YAMS | WVRQAPGKGLEWVS | AISGS---GGSTYYADSVKG | RFTISRDNSKNTLYLQMNSLRAEDTAVYYCAK | VDIVAT------------------IDAFDI | WGQGTMVTVSS |
| iPS:403870 | 21-225_23G4 | VH3\|3-23/D5\|5-12\|RF1/JH3 | ............ ............ ........ | .......... | ........ ...... | V...R... ........ .... | ................ ............... | RG..GA........... ......TE.... | ...... ..... |
| | | Germline | H_FR1 | H_CDR1 | H_FR2 | H_CDR2 | H_FR3 | H_CDR3 | H_FR4 |
| | VH4\|4-39/D4\|4-11\|RF1/JH4 | | QLQLQES-GPGLVKPSETLSLTCTVSG-GSIS | SS---SYYWG | WIRQPPGKGLEWIG | SIYY----SGSTYYNPSLKS | RVTISVDTSKNQFSLKLSSVTAADTAVYYCAR | *LQ*L-------------------YFDY | WGQGTLVTVSS |

| | | | | | | | | | |
|---|---|---|---|---|---|---|---|---|---|
| iPS:403872 | 21-225_8F11 | VH4\|4-39/D4\|4-11\|RF1/JH4 | ............<br>.Q..........<br>....V... | RT........ | .L......<br>...... | N.......<br>...A.N..<br>.... | .................<br>.............G. | HG.DW............<br>........GL.. | ......<br>..... |
| | | Germline | H_FR1 | H_CDR1 | H_FR2 | H_CDR2 | H_FR3 | H_CDR3 | H_FR4 |
| | VH1\|1-08/D3\|3-9\|RF2/JH6 | | QVQLVQS-<br>GAEVKKPGASVK<br>VSCKASG-YTFT | S-----YDIN | WVRQATGQ<br>GLEWMG | WMNPN---<br>SGNTGYAQ<br>KFQG | RVTMTRNTSISTAYMEL<br>SSLRSEDTAVYYCAR | -YYDILTGYYN------<br>----YYYYYGMDV | WGQGTT<br>VTVSS |
| iPS:437240 | 21-225_84H12 | VH1\|1-08/D3\|3-9\|RF2/JH6 | ............<br>............<br>........ | .......... | ........<br>...... | .L..H...<br>........<br>.... | .I...W....R......<br>............... | GF.......S-<br>..........PT....D.<br>.. | ......<br>..... |
| iPS:434577 | 21-225_75C11 | VH1\|1-08/D3\|3-9\|RF2/JH6 | ............<br>............<br>........ | .......... | ........<br>...... | .L..H...<br>........<br>.... | .I...W....R......<br>............... | GF.......S-<br>..........PT....D.<br>.. | ......<br>..... |
| iPS:434553 | 21-225_76H12 | VH1\|1-08/D3\|3-9\|RF2/JH6 | ............<br>............<br>........ | .......... | ........<br>...... | .L..H...<br>........<br>.... | .I...W....R......<br>............... | GF.......S-<br>..........PT....D.<br>.. | ......<br>..... |
| iPS:434927 | 21-225_86E5 | VH1\|1-08/D3\|3-9\|RF2/JH6 | ............<br>............<br>........ | .......... | ........<br>...... | ........<br>........<br>.... | .....W....R......<br>............... | GF.......S-<br>..........PT....D.<br>.. | ......<br>..... |
| | | Germline | H_FR1 | H_CDR1 | H_FR2 | H_CDR2 | H_FR3 | H_CDR3 | H_FR4 |
| | VH3\|3-23/D6\|6-19\|RF2/JH4 | | EVQLLES-<br>GGGLVQPGGSLR<br>LSCAASG-FTFS | S-----YAMS | WVRQAPGK<br>GLEWVS | AISGS---<br>GGSTYYAD<br>SVKG | RFTISRDNSKNTLYLQM<br>NSLRAEDTAVYYCAK | -GIAVA-----------<br>--------GYFDY | WGQGTL<br>VTVSS |
| iPS:435477 | 21-225_154E8 | VH3\|3-23/D6\|6-19\|RF2/JH4 | ............<br>............<br>........ | .......... | .......R<br>...... | ........<br>..N.F...<br>.... | .................<br>..............I | H.....GT.........<br>......GAH.... | ......<br>A.... |
| iPS:435385 | 21-225_149G7 | VH3\|3-23/D6\|6-19\|RF2/JH4 | ............<br>............<br>........ | .......... | ........<br>...... | ........<br>..N.F...<br>.... | .................<br>..............I | H.....GT.........<br>......GAH.... | ......<br>A.... |

**Figure 53 (Table 6)**
**Standard IgG Antibody Variable Region Protein Alignment**
**CDRs defined by Kabat**
**Alignment numbering defined by Amgen Reference (AHo)**

| KAPPA_ VARIABLE | | Germline | K_FR1 | K_CDR1 | K_FR2 | K_CDR2 | K_FR3 | K_CDR3 | K_FR4 |
|---|---|---|---|---|---|---|---|---|---|
| | VK4\|B3/J K1 | | DIVMTQSPDSLAVSLG ERATINC | KSS-- QSVLYSSNNKNY LA | WYQQKPGQPPK LLIY | W-------- ASTRES | GVPDRFSGSGSG-- TDFTLTISSLQAEDVAVY YC | QQYYS------------ -----------TPWT | FGQGTK VEIK |
| iPS:39 2928 | 21- 225_25A4 | VK4\|B3/J K1 | DIVMTQFPDSLAVSLG ERATIKC | KSS-- QSVLYSSHNNNY LA | WYQQKPGQPPK LLLY | W-------- ASTRES | GVPDRFSGSGSG-- TEFTLTISSLEAEDVAVY YC | QQYYS------------ -----------TPPT | FGQGTK VEIK |
| iPS:42 4419 | 21- 225_25A4 .001 | VK4\|B3/J K1 | DIVMTQFPDSLAVSLG ERATIKC | KSS-- QSVLYSSHNNNY LA | WYQQKPGQPPK LLLY | W-------- ASTRES | GVPDRFSGSGSG-- TEFTLTISSLEAEDVAVY YC | QQYYS------------ -----------TPPT | FGQGTK VEIK |
| iPS:44 1468 | 21- 225_25A4 .001.001 | VK4\|B3/J K1 | DIVMTQFPDSLAVSLG ERATIKC | KSS-- QSVLYSSHNNNY LA | WYQQKPGQPPK LLLY | W-------- ASTRES | GVPDRFSGSGSG-- TEFTLTISSLEAEDVAVY YC | QQYYS------------ -----------TPPT | FGQGTK VEIK |
| iPS:44 1475 | 21- 225_25A4 .001.002 | VK4\|B3/J K1 | DIVMTQFPDSLAVSLG ERATIKC | KSS-- QSVLYSSHNNNY LA | WYQQKPGQPPK LLLY | W-------- ASTRES | GVPDRFSGSGSG-- TEFTLTISSLEAEDVAVY YC | QQYYS------------ -----------TPPT | FGQGTK VEIK |
| iPS:44 1482 | 21- 225_25A4 .001.003 | VK4\|B3/J K1 | DIVMTQFPDSLAVSLG ERATIKC | KSS-- QSVLYSSHNNNY LA | WYQQKPGQPPK LLLY | W-------- ASTRES | GVPDRFSGSGSG-- TEFTLTISSLEAEDVAVY YC | QQYYS------------ -----------TPPT | FGQGTK VEIK |
| iPS:44 1489 | 21- 225_25A4 .001.004 | VK4\|B3/J K1 | DIVMTQFPDSLAVSLG ERATIKC | KSS-- QSVLYSSHNNNY LA | WYQQKPGQPPK LLLY | W-------- ASTRES | GVPDRFSGSGSG-- TEFTLTISSLEAEDVAVY YC | QQYYS------------ -----------TPPT | FGQGTK VEIK |
| iPS:44 1496 | 21- 225_25A4 .001.005 | VK4\|B3/J K1 | DIVMTQSPDSLAVSLG ERATIKC | KSS-- QSVLYSSHNNNY LA | WYQQKPGQPPK LLLY | W-------- ASTRES | GVPDRFSGSGSG-- TEFTLTISSLQAEDVAVY YC | QQYYS------------ -----------TPPT | FGQGTK VEIK |
| iPS:44 1505 | 21- 225_25A4 .001.006 | VK4\|B3/J K1 | DIVMTQSPDSLAVSLG ERATIKC | KSS-- QSVLYSSHNNNY LA | WYQQKPGQPPK LLLY | W-------- ASTRES | GVPDRFSGSGSG-- TEFTLTISSLQAEDVAVY YC | QQYYS------------ -----------TPPT | FGQGTK VEIK |
| iPS:44 1512 | 21- 225_25A4 .001.007 | VK4\|B3/J K1 | DIVMTQSPDSLAVSLG ERATIKC | KSS-- QSVLYSSHNNNY LA | WYQQKPGQPPK LLLY | W-------- ASTRES | GVPDRFSGSGSG-- TEFTLTISSLQAEDVAVY YC | QQYYS------------ -----------TPPT | FGQGTK VEIK |
| iPS:44 1519 | 21- 225_25A4 .001.008 | VK4\|B3/J K1 | DIVMTQSPDSLAVSLG ERATIKC | KSS-- QSVLYSSHNNNY LA | WYQQKPGQPPK LLLY | W-------- ASTRES | GVPDRFSGSGSG-- TEFTLTISSLQAEDVAVY YC | QQYYS------------ -----------TPPT | FGQGTK VEIK |
| iPS:44 1554 | 21- 225_25A4 .001.013 | VK4\|B3/J K1 | DIVMTQFPDSLAVSLG ERATIKC | KSS-- QSVLYSSHNNNY LA | WYQQKPGQPPK LLLY | W-------- ASTRES | GVPDRFSGSGSG-- TEFTLTISSLQAEDVAVY YC | QQYYS------------ -----------TPPT | FGQGTK VEIK |
| iPS:44 | 21- | VK4\|B3/J | DIVMTQSPDSLAVSLG | KSS-- | WYQQKPGQPPK | W-------- | GVPDRFSGSGSG-- | QQYYS------------ | FGQGTK |

| | | | | | | | | | |
|---|---|---|---|---|---|---|---|---|---|
| iPS:44 1604 | 21-225_25A4.001.020 | VK4\|B3/JK1 | DIVMTQSPDSLAVSLG ERATINC | KSS-- QSVLYSSHNNNY LA | WYQQKPGQPPK LLLY | W-------- ASTRES | GVPDRFSGSGSG-- TEFTLTISSLQAEDVAVY YC | QQYYS----------- -----------TPPT | FGQGTK VEIK |
| iPS:44 1613 | 21-225_25A4.001.021 | VK4\|B3/JK1 | DIVMTQSPDSLAVSLG ERATINC | KSS-- QSVLYSSHNNNY LA | WYQQKPGQPPK LLLY | W-------- ASTRES | GVPDRFSGSGSG-- TEFTLTISSLQAEDVAVY YC | QQYYS----------- -----------TPPT | FGQGTK VEIK |
| iPS:44 3006 | 21-225_25A4.001.029 | VK4\|B3/JK1 | DIVMTQFPDSLAVSLG ERATIKC | KSS-- QSVLYSSHNNNY LA | WYQQKPGQPPK LLLY | W-------- ASTRES | GVPDRFSGSGSG-- TEFTLTISSLQAEDVAVY YC | QQYYS----------- -----------TPPT | FGQGTK VEIK |
| iPS:44 1962 | 21-225_4A2.001.023 | VK4\|B3/JK1 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYS----------- -----------TPVT | FGQGTK VEIK |
| iPS:44 1999 | 21-225_4A2.001.028 | VK4\|B3/JK1 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYS----------- -----------TPVT | FGQGTK VEIK |
| iPS:44 2006 | 21-225_4A2.001.029 | VK4\|B3/JK1 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYS----------- -----------TPVT | FGQGTK VEIK |
| iPS:44 2020 | 21-225_4A2.001.031 | VK4\|B3/JK1 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYN----------- -----------TPVT | FGPGTK VEIK |
| | | Germline | K_FR1 | K_CDR1 | K_FR2 | K_CDR2 | K_FR3 | K_CDR3 | K_FR4 |
| | VK1\|L5/JK3 | | DIQMTQSPSSVSASVG DRVTITC | RAS--QGIS--- ---SWLA | WYQQKPGKAPK LLIY | A-------- ASSLQS | GVPSRFSGSGSG-- TDFTLTISSLQPEDFATY YC | QQANS----------- -----------FPFT | FGPGTK VDIK |
| iPS:39 3954 | 21-225_4H6 | VK1\|L5/JK3 | DIQMTQSPSSVSASVG DRVTITC | RAS--QGIS--- ---RWLA | WYQQKPGKAPK LLIY | G-------- ASSLQS | GVPSRFSGSGSG-- TDFTLTISSLQPEDFAIY YC | QQANS----------- -----------FPFT | FGPGTK VDIK |
| iPS:44 2050 | 21-225_4H6.004 | VK1\|L5/JK3 | DIQMTQSPSSVSASVG DRVTITC | RAS--QGIS--- ---RWLA | WYQQKPGKAPK LLIY | G-------- ASSLQS | GVPSRFSGSGSG-- TDFTLTISSLQPEDFAIY YC | QQANS----------- -----------FPFT | FGPGTK VDIK |
| iPS:44 2059 | 21-225_4H6.005 | VK1\|L5/JK3 | DIQMTQSPSSVSASVG DRVTITC | RAS--QGIS--- ---RWLA | WYQQKPGKAPK LLIY | G-------- ASSLQS | GVPSRFSGSGSG-- TDFTLTISSLQPEDFAIY YC | QQANS----------- -----------FPFT | FGQGTK VDIK |
| iPS:44 2065 | 21-225_4H6.006 | VK1\|L5/JK3 | DIQMTQSPSSVSASVG DRVTITC | RAS--QGIS--- ---RWLA | WYQQKPGKAPK LLIY | G-------- ASSLQS | GVPSRFSGSGSG-- TDFTLTISSLQPEDFATY YC | QQANS----------- -----------FPFT | FGQGTK VDIK |
| iPS:44 2071 | 21-225_4H6.007 | VK1\|L5/JK3 | DIQMTQSPSSVSASVG DRVTITC | RAS--QGIS--- ---RWLA | WYQQKPGKAPK LLIY | G-------- ASSLQS | GVPSRFSGSGSG-- TDFTLTISSLQPEDFAIY YC | QQANS----------- -----------FPFT | FGPGTK VDIK |
| iPS:44 2078 | 21-225_4H6.008 | VK1\|L5/JK3 | DIQMTQSPSSVSASVG DRVTITC | RAS--QGIS--- ---RWLA | WYQQKPGKAPK LLIY | G-------- ASSLQS | GVPSRFSGSGSG-- TDFTLTISSLQPEDFAIY YC | QQANS----------- -----------FPFT | FGPGTK VDIK |
| iPS:44 2085 | 21-225_4H6.009 | VK1\|L5/JK3 | DIQMTQSPSSVSASVG DRVTITC | RAS--QGIS--- ---RWLA | WYQQKPGKAPK LLIY | G-------- ASSLQS | GVPSRFSGSGSG-- TDFTLTISSLQPEDFAIY YC | QQANS----------- -----------FPFT | FGQGTK VDIK |

| | | | | | | | | | |
|---|---|---|---|---|---|---|---|---|---|
| iPS:44 2089 | 21-225_4H6.010 | VK1\|L5/JK3 | DIQMTQSPSSVSASVG DRVTITC | RAS--QGIS--- ---RWLA | WYQQKPGKAPK LLIY | G-------- ASSLQS | GVPSRFSGSGSG-- TDFTLTISSLQPEDFAIY YC | QQANS----------- -----------FPFT | FGQGTK VDIK |
| iPS:44 2093 | 21-225_4H6.011 | VK1\|L5/JK3 | DIQMTQSPSSVSASVG DRVTITC | RAS--QGIS--- ---RWLA | WYQQKPGKAPK LLIY | G-------- ASSLQS | GVPSRFSGSGSG-- TDFTLTISSLQPEDFATY YC | QQANS----------- -----------FPFT | FGQGTK VDIK |
| iPS:44 3016 | 21-225_4H6.014 | VK1\|L5/JK3 | DIQMTQSPSSVSASVG DRVTITC | RAS--QGIS--- ---RWLA | WYQQKPGKAPK LLIY | G-------- ASSLQS | GVPSRFSGSGSG-- TDFTLTISSLQPEDFATY YC | QQANS----------- -----------FPFT | FGPGTK VDIK |
| | | Germline | K_FR1 | K_CDR1 | K_FR2 | K_CDR2 | K_FR3 | K_CDR3 | K_FR4 |
| | VK4\|B3/JK3 | | DIVMTQSPDSLAVSLG ERATINC | KSS-- QSVLYSSNNKNY LA | WYQQKPGQPPK LLIY | W-------- ASTRES | GVPDRFSGSGSG-- TDFTLTISSLQAEDVAVY YC | QQYYS----------- -----------TPFT | FGPGTK VDIK |
| iPS:41 2232 | 21-225_4A2 | VK4\|B3/JK3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYN----------- -----------TPVT | FGPGTK VGIK |
| iPS:42 2894 | 21-225_4A2.001 | VK4\|B3/JK3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYN----------- -----------TPVT | FGPGTK VGIK |
| iPS:44 1841 | 21-225_4A2.001.001 | VK4\|B3/JK3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYS----------- -----------TPVT | FGPGTK VGIK |
| iPS:44 1847 | 21-225_4A2.001.002 | VK4\|B3/JK3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYN----------- -----------APVT | FGPGTK VGIK |
| iPS:44 1853 | 21-225_4A2.001.003 | VK4\|B3/JK3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYQ----------- -----------TPVT | FGPGTK VGIK |
| iPS:44 1859 | 21-225_4A2.001.004 | VK4\|B3/JK3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYN----------- -----------TPVT | FGPGTK VGIK |
| iPS:44 1866 | 21-225_4A2.001.005 | VK4\|B3/JK3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYN----------- -----------TPVT | FGPGTK VGIK |
| iPS:44 1873 | 21-225_4A2.001.006 | VK4\|B3/JK3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYN----------- -----------TPVT | FGPGTK VGIK |
| iPS:44 1880 | 21-225_4A2.001.007 | VK4\|B3/JK3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYS----------- -----------TPVT | FGPGTK VGIK |
| iPS:44 1884 | 21-225_4A2.001.008 | VK4\|B3/JK3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYS----------- -----------TPVT | FGPGTK VGIK |
| iPS:44 1888 | 21-225_4A2.001.009 | VK4\|B3/JK3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYN----------- -----------APVT | FGPGTK VGIK |

| | | | | | | | | | |
|---|---|---|---|---|---|---|---|---|---|
| iPS:44 1892 | 21-225_4A2. 001.010 | VK4\|B3/J K3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYQ------------ -----------TPVT | FGPGTK VGIK |
| iPS:44 1896 | 21-225_4A2. 001.011 | VK4\|B3/J K3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYQ------------ -----------TPVT | FGPGTK VGIK |
| iPS:44 1900 | 21-225_4A2. 001.012 | VK4\|B3/J K3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYQ------------ -----------TPVT | FGPGTK VGIK |
| iPS:44 1955 | 21-225_4A2. 001.022 | VK4\|B3/J K3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYS------------ -----------TPVT | FGPGTK VGIK |
| iPS:44 1971 | 21-225_4A2. 001.024 | VK4\|B3/J K3 | DIVMTQSPDSLAVSLG ERATINC | KSS-- QSILHSSNNNNY LA | WFQQKPGQPPK LLLY | W-------- ASTRES | GVPDRFSGSGSG-- TDFTLTISSLQPEDVAVY YC | QQYYS------------ -----------TPVT | FGPGTK VGIK |
| | | **Germline** | **K_FR1** | **K_CDR1** | **K_FR2** | **K_CDR2** | **K_FR3** | **K_CDR3** | **K_FR4** |
| | VK1\|A30/ JK1 | | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHNS------------ -----------YPWT | FGQGTK VEIK |
| iPS:39 4041 | 21-225_5E5 | VK1\|A30/ JK1 | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| iPS:44 2115 | 21-225_5E5. 003 | VK1\|A30/ JK1 | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| iPS:44 2122 | 21-225_5E5. 004 | VK1\|A30/ JK1 | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| iPS:44 2129 | 21-225_5E5. 005 | VK1\|A30/ JK1 | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| iPS:44 2136 | 21-225_5E5. 006 | VK1\|A30/ JK1 | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| iPS:44 2171 | 21-225_5E5. 011 | VK1\|A30/ JK1 | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| iPS:44 2178 | 21-225_5E5. 012 | VK1\|A30/ JK1 | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| iPS:44 2199 | 21-225_5E5. 015 | VK1\|A30/ JK1 | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |
| iPS:44 2206 | 21-225_5E5. 016 | VK1\|A30/ JK1 | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS------------ -----------YPRT | FGQGTK VEVK |

| | | | | | | | | | |
|---|---|---|---|---|---|---|---|---|---|
| **iPS:44 2213** | **21-225_5E5.017** | **VK1\|A30/ JK1** | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS----------- -----------YPRT | FGQGTK VEVK |
| **iPS:44 2220** | **21-225_5E5.018** | **VK1\|A30/ JK1** | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS----------- -----------YPRT | FGQGTK VEVK |
| **iPS:44 2227** | **21-225_5E5.019** | **VK1\|A30/ JK1** | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS----------- -----------YPRT | FGQGTK VEVK |
| **iPS:44 2255** | **21-225_5E5.023** | **VK1\|A30/ JK1** | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS----------- -----------YPRT | FGQGTK VEVK |
| **iPS:44 2262** | **21-225_5E5.024** | **VK1\|A30/ JK1** | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS----------- -----------YPRT | FGQGTK VEVK |
| **iPS:44 2269** | **21-225_5E5.025** | **VK1\|A30/ JK1** | DIQMTQSPSSLSASVG DRVTITC | RAS--QGIR--- ---NDLG | WYQQKPGKAPK RLIY | A-------- ASSLQS | GVPSRFSGSGSG-- TEFTLTISSLQPEDFATY YC | LQHYS----------- -----------YPRT | FGQGTK VEVK |
| | | **Germline** | **K_FR1** | **K_CDR1** | **K_FR2** | **K_CDR2** | **K_FR3** | **K_CDR3** | **K_FR4** |
| | **VK1\|O12/ JK4** | | DIQMTQSPSSLSASVG DRVTITC | RAS--QSIS--- ---SYLN | WYQQKPGKAPK LLIY | A-------- ASSLQS | GVPSRFSGSGSG-- TDFTLTISSLQPEDFATY YC | QQSYS----------- -----------TPLT | FGGGTK VEIK |
| **iPS:39 3930** | **21-225_7E11** | **VK1\|O12/ JK4** | DIQMTQSPSSLSASVG DRVTIAC | RAS--QNII--- ---SYLN | WYQQKPGKAPK FLIY | T-------- ASSLQS | GVPSRFSGSGSG-- TDFTLTISSLQPEDFAIY YC | QQTYS----------- -----------TPLT | FGGGTK VEIK |
| **iPS:42 4460** | **21-225_7E11.001** | **VK1\|O12/ JK4** | DIQMTQSPSSLSASVG DRVTIAC | RAS--QNII--- ---SYLN | WYQQKPGKAPK FLIY | T-------- ASSLQS | GVPSRFSGSGSG-- TDFTLTISSLQPEDFAIY YC | QQTYS----------- -----------TPLT | FGGGTK VEIK |
| **iPS:44 2311** | **21-225_7E11.001.001** | **VK1\|O12/ JK4** | DIQMTQSPSSLSASVG DRVTITC | RAS--QNII--- ---SYLN | WYQQKPGKAPK FLIY | T-------- ASSLQS | GVPSRFSGSGSG-- TDFTLTISSLQPEDFAIY YC | QQTYS----------- -----------TPLT | FGGGTK VEIK |
| **iPS:44 2317** | **21-225_7E11.001.002** | **VK1\|O12/ JK4** | DIQMTQSPSSLSASVG DRVTITC | RAS--QNII--- ---SYLN | WYQQKPGKAPK LLIY | T-------- ASSLQS | GVPSRFSGSGSG-- TDFTLTISSLQPEDFATY YC | QQTYS----------- -----------TPLT | FGGGTK VEIK |
| **iPS:44 2323** | **21-225_7E11.001.003** | **VK1\|O12/ JK4** | DIQMTQSPSSLSASVG DRVTIAC | RAS--QNII--- ---SYLN | WYQQKPGKAPK FLIY | T-------- ASSLQS | GVPSRFSGSGSG-- TDFTLTISSLQPEDFAIY YC | QQTYS----------- -----------TPLT | FGGGTK VEIK |
| **iPS:44 2330** | **21-225_7E11.001.004** | **VK1\|O12/ JK4** | DIQMTQSPSSLSASVG DRVTIAC | RAS--QNII--- ---SYLN | WYQQKPGKAPK FLIY | T-------- ASSLQS | GVPSRFSGSGSG-- TDFTLTISSLQPEDFAIY YC | QQTYS----------- -----------TPLT | FGGGTK VEIK |
| **iPS:44 2337** | **21-225_7E11.001.005** | **VK1\|O12/ JK4** | DIQMTQSPSSLSASVG DRVTIAC | RAS--QNII--- ---SYLN | WYQQKPGKAPK FLIY | T-------- ASSLQS | GVPSRFSGSGSG-- TDFTLTISSLQPEDFAIY YC | QQTYS----------- -----------TPLT | FGGGTK VEIK |
| **iPS:44 2344** | **21-225_7E11.001.006** | **VK1\|O12/ JK4** | DIQMTQSPSSLSASVG DRVTIAC | RAS--QNII--- ---SYLN | WYQQKPGKAPK FLIY | T-------- ASSLQS | GVPSRFSGSGSG-- TDFTLTISSLQPEDFAIY YC | QQTYS----------- -----------TPLT | FGGGTK VEIK |

| | | | | | | | | | |
|---|---|---|---|---|---|---|---|---|---|
| **iPS:44<br>2351** | **21-<br>225_7E11<br>.001.007** | **VK1\|O12/<br>JK4** | DIQMTQSPSSLSASVG<br>DRVTIAC | RAS--QNII---<br>---SYLN | WYQQKPGKAPK<br>FLIY | T--------<br>ASSLQS | GVPSRFSGSGSG--<br>TDFTLTISSLQPEDFAIY<br>YC | QQTYS------------<br>-----------TPLT | FGGGTK<br>VEIK |
| **iPS:44<br>2358** | **21-<br>225_7E11<br>.001.008** | **VK1\|O12/<br>JK4** | DIQMTQSPSSLSASVG<br>DRVTIAC | RAS--QNII---<br>---SYLN | WYQQKPGKAPK<br>FLIY | T--------<br>ASSLQS | GVPSRFSGSGSG--<br>TDFTLTISSLQPEDFAIY<br>YC | QQTYS------------<br>-----------TPLT | FGGGTK<br>VEIK |
| **iPS:44<br>2365** | **21-<br>225_7E11<br>.001.009** | **VK1\|O12/<br>JK4** | DIQMTQSPSSLSASVG<br>DRVTIAC | RAS--QNII---<br>---SYLN | WYQQKPGKAPK<br>FLIY | T--------<br>ASSLQS | GVPSRFSGSGSG--<br>TDFTLTISSLQPEDFAIY<br>YC | QQTYS------------<br>-----------TPLT | FGGGTK<br>VEIK |
| **iPS:44<br>2372** | **21-<br>225_7E11<br>.001.010** | **VK1\|O12/<br>JK4** | DIQMTQSPSSLSASVG<br>DRVTIAC | RAS--QNII---<br>---SYLN | WYQQKPGKAPK<br>FLIY | T--------<br>ASSLQS | GVPSRFSGSGSG--<br>TDFTLTISSLQPEDFAIY<br>YC | QQTYS------------<br>-----------TPLT | FGGGTK<br>VEIK |
| **iPS:44<br>2379** | **21-<br>225_7E11<br>.001.011** | **VK1\|O12/<br>JK4** | DIQMTQSPSSLSASVG<br>DRVTIAC | RAS--QNII---<br>---SYLN | WYQQKPGKAPK<br>FLIY | T--------<br>ASSLQS | GVPSRFSGSGSG--<br>TDFTLTISSLQPEDFAIY<br>YC | QQTYS------------<br>-----------TPLT | FGGGTK<br>VEIK |
| **iPS:44<br>2386** | **21-<br>225_7E11<br>.001.012** | **VK1\|O12/<br>JK4** | DIQMTQSPSSLSASVG<br>DRVTITC | RAS--QNII---<br>---SYLN | WYQQKPGKAPK<br>FLIY | T--------<br>ASSLQS | GVPSRFSGSGSG--<br>TDFTLTISSLQPEDFAIY<br>YC | QQTYS------------<br>-----------TPLT | FGGGTK<br>VEIK |
| **iPS:44<br>2390** | **21-<br>225_7E11<br>.001.013** | **VK1\|O12/<br>JK4** | DIQMTQSPSSLSASVG<br>DRVTITC | RAS--QNII---<br>---SYLN | WYQQKPGKAPK<br>FLIY | T--------<br>ASSLQS | GVPSRFSGSGSG--<br>TDFTLTISSLQPEDFAIY<br>YC | QQTYS------------<br>-----------TPLT | FGGGTK<br>VEIK |
| **iPS:44<br>2394** | **21-<br>225_7E11<br>.001.014** | **VK1\|O12/<br>JK4** | DIQMTQSPSSLSASVG<br>DRVTITC | RAS--QNII---<br>---SYLN | WYQQKPGKAPK<br>FLIY | T--------<br>ASSLQS | GVPSRFSGSGSG--<br>TDFTLTISSLQPEDFAIY<br>YC | QQTYS------------<br>-----------TPLT | FGGGTK<br>VEIK |
| **iPS:44<br>2398** | **21-<br>225_7E11<br>.001.015** | **VK1\|O12/<br>JK4** | DIQMTQSPSSLSASVG<br>DRVTITC | RAS--QNII---<br>---SYLN | WYQQKPGKAPK<br>LLIY | T--------<br>ASSLQS | GVPSRFSGSGSG--<br>TDFTLTISSLQPEDFATY<br>YC | QQTYS------------<br>-----------TPLT | FGGGTK<br>VEIK |
| **iPS:44<br>2402** | **21-<br>225_7E11<br>.001.016** | **VK1\|O12/<br>JK4** | DIQMTQSPSSLSASVG<br>DRVTITC | RAS--QNII---<br>---SYLN | WYQQKPGKAPK<br>LLIY | T--------<br>ASSLQS | GVPSRFSGSGSG--<br>TDFTLTISSLQPEDFATY<br>YC | QQTYS------------<br>-----------TPLT | FGGGTK<br>VEIK |
| **iPS:44<br>2406** | **21-<br>225_7E11<br>.001.017** | **VK1\|O12/<br>JK4** | DIQMTQSPSSLSASVG<br>DRVTITC | RAS--QNII---<br>---SYLN | WYQQKPGKAPK<br>LLIY | T--------<br>ASSLQS | GVPSRFSGSGSG--<br>TDFTLTISSLQPEDFATY<br>YC | QQTYS------------<br>-----------TPLT | FGGGTK<br>VEIK |
| **iPS:44<br>2410** | **21-<br>225_7E11<br>.001.018** | **VK1\|O12/<br>JK4** | DIQMTQSPSSLSASVG<br>DRVTITC | RAS--QNII---<br>---SYLN | WYQQKPGKAPK<br>FLIY | T--------<br>ASSLQS | GVPSRFSGSGSG--<br>TDFTLTISSLQPEDFAIY<br>YC | QQTYS------------<br>-----------TPLT | FGGGTK<br>VEIK |
| **iPS:44<br>2417** | **21-<br>225_7E11<br>.001.019** | **VK1\|O12/<br>JK4** | DIQMTQSPSSLSASVG<br>DRVTIAC | RAS--QNII---<br>---SYLN | WYQQKPGKAPK<br>FLIY | T--------<br>ASSLQS | GVPSRFSGSGSG--<br>TDFTLTISSLQPEDFAIY<br>YC | QQTYS------------<br>-----------TPLT | FGGGTK<br>VEIK |
| **iPS:44<br>2431** | **21-<br>225_7E11<br>.001.021** | **VK1\|O12/<br>JK4** | DIQMTQSPSSLSASVG<br>DRVTIAC | RAS--QNII---<br>---SYLN | WYQQKPGKAPK<br>FLIY | T--------<br>ASSLQS | GVPSRFSGSGSG--<br>TDFTLTISSLQPEDFAIY<br>YC | QQTYS------------<br>-----------TPLT | FGGGTK<br>VEIK |
| **iPS:44<br>2438** | **21-<br>225_7E11<br>.001.022** | **VK1\|O12/<br>JK4** | DIQMTQSPSSLSASVG<br>DRVTITC | RAS--QNII---<br>---SYLN | WYQQKPGKAPK<br>FLIY | T--------<br>ASSLQS | GVPSRFSGSGSG--<br>TDFTLTISSLQPEDFAIY<br>YC | QQTYS------------<br>-----------TPLT | FGGGTK<br>VEIK |

| | | | | | | | | | |
|---|---|---|---|---|---|---|---|---|---|
| iPS:44 3027 | 21-225_7E11.001.023 | VK1\|O12/JK4 | DIQMTQSPSSLSASVG DRVTIAC | RAS--QNII------SYLN | WYQQKPGKAPK FLIY | T-------- ASSLQS | GVPSRFSGSGSG-- TDFTLTISSLQPEDFAIY YC | QQTYS----------------------TPLT | FGGGTK VEIK |
| HEAVY_VARIABLE | | | | | | | | | |
| | | Germline | H_FR1 | H_CDR1 | H_FR2 | H_CDR2 | H_FR3 | H_CDR3 | H_FR4 |
| | VH1\|1-08/D6\|6-19\|RF1/JH4 | | QVQLVQS-GAEVKKPGASVKVSCKASG-YTFT | S-----YDIN | WVRQATGQGLE WMG | WMNPN---SGNTGYAQKFQG | RVTMTRNTSISTAYMELS SLRSEDTAVYYCAR | GYSSGW-------------------YYFDY | WGQGTL VTVSS |
| iPS:39 2928 | 21-225_25A4 | VH1\|1-08/D6\|6-19\|RF1/JH4 | QVLLVQS-GAEVKRPGASVKVSCKASG-YTFT | N-----YDIN | WVRQATGQGLE WMG | WMYPN---SGNTGYAQKFQG | RVTMTRNTSISTAYMELS SLRSEDTAVYYCAS | SSGWY---------------------YFDY | WGQGTL VTVSS |
| iPS:42 4419 | 21-225_25A4.001 | VH1\|1-08/D6\|6-19\|RF1/JH4 | QVLLVQS-GAEVKRPGASVKVSCKASG-YTFT | N-----YDIN | WVRQATGQGLE WMG | WMYPN---SGNTGYAQKFQG | RVTMTRDTSISTAYMELS SLRSEDTAVYYCAS | SSGWY---------------------YFDY | WGQGTL VTVSS |
| iPS:44 1468 | 21-225_25A4.001.001 | VH1\|1-08/D6\|6-19\|RF1/JH4 | QVLLVQS-GAEVKRPGASVKVSCKASG-YTFT | N-----YDIN | WVRQATGQGLE WMG | WMYPN---SGSTGYAQKFQG | RVTMTRDTSISTAYMELS SLRSEDTAVYYCAS | SSGWY---------------------YFDY | WGQGTL VTVSS |
| iPS:44 1475 | 21-225_25A4.001.002 | VH1\|1-08/D6\|6-19\|RF1/JH4 | QVLLVQS-GAEVKRPGASVKVSCKASG-YTFT | N-----YDIN | WVRQATGQGLE WMG | WMYPN---SGNAGYAQKFQG | RVTMTRDTSISTAYMELS SLRSEDTAVYYCAS | SSGWY---------------------YFDY | WGQGTL VTVSS |
| iPS:44 1482 | 21-225_25A4.001.003 | VH1\|1-08/D6\|6-19\|RF1/JH4 | QVLLVQS-GAEVKRPGASVKVSCKASG-YTFT | N-----YDIN | WVRQATGQGLE WMG | WMYPN---SGNVGYAQKFQG | RVTMTRDTSISTAYMELS SLRSEDTAVYYCAS | SSGWY---------------------YFDY | WGQGTL VTVSS |
| iPS:44 1489 | 21-225_25A4.001.004 | VH1\|1-08/D6\|6-19\|RF1/JH4 | QVLLVQS-GAEVKRPGASVKVSCKASG-YTFT | N-----YDIN | WVRQATGQGLE WMG | WMYPN---SGQTGYAQKFQG | RVTMTRDTSISTAYMELS SLRSEDTAVYYCAS | SSGWY---------------------YFDY | WGQGTL VTVSS |
| iPS:44 1496 | 21-225_25A4.001.005 | VH1\|1-08/D6\|6-19\|RF1/JH4 | QVQLVQS-GAEVKRPGASVKVSCKASG-YTFT | N-----YDIN | WVRQATGQGLE WMG | WMYPN---SGSTGYAQKFQG | RVTMTRDTSISTAYMELS SLRSEDTAVYYCAS | SSGWY---------------------YFDY | WGQGTL VTVSS |
| iPS:44 1505 | 21-225_25A4.001.006 | VH1\|1-08/D6\|6-19\|RF1/JH4 | QVQLVQS-GAEVKRPGASVKVSCKASG-YTFT | N-----YDIN | WVRQATGQGLE WMG | WMYPN---SGNAGYAQKFQG | RVTMTRDTSISTAYMELS SLRSEDTAVYYCAS | SSGWY---------------------YFDY | WGQGTL VTVSS |
| iPS:44 1512 | 21-225_25A4.001.007 | VH1\|1-08/D6\|6-19\|RF1/JH4 | QVQLVQS-GAEVKRPGASVKVSCKASG-YTFT | N-----YDIN | WVRQATGQGLE WMG | WMYPN---SGNVGYAQKFQG | RVTMTRDTSISTAYMELS SLRSEDTAVYYCAS | SSGWY---------------------YFDY | WGQGTL VTVSS |

| | | | | | | | | | |
|---|---|---|---|---|---|---|---|---|---|
| **iPS:44 1519** | **21-225_25A4 .001.008** | **VH1\|1-08/D6\|6-19\|RF1/J H4** | QVQLVQS-GAEVKRPGASVKVSCK ASG-YTFT | N-----YDIN | WVRQATGQGLE WMG | WMYPN---SGQTGYAQK FQG | RVTMTRDTSISTAYMELS SLRSEDTAVYYCAS | SSGWY------------ ---------YFDY | WGQGTL VTVSS |
| **iPS:44 1554** | **21-225_25A4 .001.013** | **VH1\|1-08/D6\|6-19\|RF1/J H4** | QVLLVQS-GAEVKRPGASVKVSCK ASG-YTFT | N-----YDIN | WVRQATGQGLE WMG | WMYPN---SGSTGYAQK FQG | RVTMTRDTSISTAYMELS SLRSEDTAVYYCAS | SSGWY------------ ---------YFDY | WGQGTL VTVSS |
| **iPS:44 1595** | **21-225_25A4 .001.019** | **VH1\|1-08/D6\|6-19\|RF1/J H4** | QVQLVQS-GAEVKRPGASVKVSCK ASG-YTFT | N-----YDIN | WVRQAPGQGLE WMG | WMYPN---SGSTGYAQK FQG | RVTMTRDTSISTAYMELS SLRSEDTAVYYCAS | SSGWY------------ ---------YFDY | WGQGTL VTVSS |
| **iPS:44 1604** | **21-225_25A4 .001.020** | **VH1\|1-08/D6\|6-19\|RF1/J H4** | QVLLVQS-GAEVKRPGASVKVSCK ASG-YTFT | N-----YDIN | WVRQAPGQGLE WMG | WMYPN---SGQTGYAQK FQG | RVTMTRDTSISTAYMELS SLRSEDTAVYYCAS | SSGWY------------ ---------YFDY | WGQGTL VTVSS |
| **iPS:44 1613** | **21-225_25A4 .001.021** | **VH1\|1-08/D6\|6-19\|RF1/J H4** | QVQLVQS-GAEVKRPGASVKVSCK ASG-YTFT | N-----YDIN | WVRQATGQGLE WMG | WMYPN---SGNAGYAQK FQG | RVTMTRDTSISTAYMELS SLRSEDTAVYYCAS | SSGWY------------ ---------YFDY | WGQGTL VTVSS |
| **iPS:44 3006** | **21-225_25A4 .001.029** | **VH1\|1-08/D6\|6-19\|RF1/J H4** | QVLLVQS-GAEVKRPGASVKVSCK ASG-YTFT | N-----YDIN | WVRQATGQGLE WMG | WMYPN---SGQTGYAQK FQG | RVTMTRDTSISTAYMELS SLRSEDTAVYYCAS | SSGWY------------ ---------YFDY | WGQGTL VTVSS |
| **iPS:41 2232** | **21-225_4A2** | **VH1\|1-08/D6\|6-19\|RF1/J H4** | QVQLVQS-GTEVKKPGASVKVSCK ASG-YTFT | N-----YDIN | WVRQATGQGLE WMG | WMHPN---SGNTGYAQK FQG | RVTLTRNTSISTAYMELS SLRSEDTAVYYCAS | SSGWY------------ ---------YFDY | WGQGTL VTVSS |
| **iPS:42 2894** | **21-225_4A2. 001** | **VH1\|1-08/D6\|6-19\|RF1/J H4** | QVQLVQS-GTEVKKPGASVKVSCK ASG-YTFT | N-----YDIN | WVRQATGQGLE WMG | WMHPN---SGNTGYAQK FQG | RVTLTRDTSISTAYMELS SLRSEDTAVYYCAS | SSGWY------------ ---------YFDY | WGQGTL VTVSS |
| **iPS:44 1841** | **21-225_4A2. 001.001** | **VH1\|1-08/D6\|6-19\|RF1/J H4** | QVQLVQS-GTEVKKPGASVKVSCK ASG-YTFT | N-----YDIN | WVRQATGQGLE WMG | WMHPN---SGNTGYAQK FQG | RVTLTRDTSISTAYMELS SLRSEDTAVYYCAS | SSGWY------------ ---------YFDY | WGQGTL VTVSS |
| **iPS:44 1847** | **21-225_4A2. 001.002** | **VH1\|1-08/D6\|6-19\|RF1/J H4** | QVQLVQS-GTEVKKPGASVKVSCK ASG-YTFT | N-----YDIN | WVRQATGQGLE WMG | WMHPN---SGNTGYAQK FQG | RVTLTRDTSISTAYMELS SLRSEDTAVYYCAS | SSGWY------------ ---------YFDY | WGQGTL VTVSS |
| **iPS:44 1853** | **21-225_4A2. 001.003** | **VH1\|1-08/D6\|6-19\|RF1/J H4** | QVQLVQS-GTEVKKPGASVKVSCK ASG-YTFT | N-----YDIN | WVRQATGQGLE WMG | WMHPN---SGNTGYAQK FQG | RVTLTRDTSISTAYMELS SLRSEDTAVYYCAS | SSGWY------------ ---------YFDY | WGQGTL VTVSS |
| **iPS:44 1859** | **21-225_4A2.** | **VH1\|1-08/D6\|6-** | QVQLVQS-GTEVKKPGASVKVSCK | N-----YDIN | WVRQATGQGLE WMG | WMHPN---SGNAGYAQK | RVTLTRDTSISTAYMELS SLRSEDTAVYYCAS | SSGWY------------ ---------YFDY | WGQGTL VTVSS |

| | | | | | | | | | |
|---|---|---|---|---|---|---|---|---|---|
| **iPS:441866** | **21-225_4A2.001.005** | **VH1\|1-08/D6\|6-19\|RF1/JH4** | QVQLVQS-GTEVKKPGASVKVSCKASG-YTFT | N-----YDIN | WVRQATGQGLEWMG | WMHPN---SGSTGYAQKFQG | RVTLTRDTSISTAYMELSSLRSEDTAVYYCAS | SSGWY---------------------YFDY | WGQGTLVTVSS |
| **iPS:441873** | **21-225_4A2.001.006** | **VH1\|1-08/D6\|6-19\|RF1/JH4** | QVQLVQS-GTEVKKPGASVKVSCKASG-YTFT | N-----YDIN | WVRQATGQGLEWMG | WMHPN---SGQTGYAQKFQG | RVTLTRDTSISTAYMELSSLRSEDTAVYYCAS | SSGWY---------------------YFDY | WGQGTLVTVSS |
| **iPS:441880** | **21-225_4A2.001.007** | **VH1\|1-08/D6\|6-19\|RF1/JH4** | QVQLVQS-GTEVKKPGASVKVSCKASG-YTFT | N-----YDIN | WVRQATGQGLEWMG | WMHPN---SGQTGYAQKFQG | RVTLTRDTSISTAYMELSSLRSEDTAVYYCAS | SSGWY---------------------YFDY | WGQGTLVTVSS |
| **iPS:441884** | **21-225_4A2.001.008** | **VH1\|1-08/D6\|6-19\|RF1/JH4** | QVQLVQS-GTEVKKPGASVKVSCKASG-YTFT | N-----YDIN | WVRQATGQGLEWMG | WMHPN---SGNAGYAQKFQG | RVTLTRDTSISTAYMELSSLRSEDTAVYYCAS | SSGWY---------------------YFDY | WGQGTLVTVSS |
| **iPS:441888** | **21-225_4A2.001.009** | **VH1\|1-08/D6\|6-19\|RF1/JH4** | QVQLVQS-GTEVKKPGASVKVSCKASG-YTFT | N-----YDIN | WVRQATGQGLEWMG | WMHPN---SGQTGYAQKFQG | RVTLTRDTSISTAYMELSSLRSEDTAVYYCAS | SSGWY---------------------YFDY | WGQGTLVTVSS |
| **iPS:441892** | **21-225_4A2.001.010** | **VH1\|1-08/D6\|6-19\|RF1/JH4** | QVQLVQS-GTEVKKPGASVKVSCKASG-YTFT | N-----YDIN | WVRQATGQGLEWMG | WMHPN---SGNAGYAQKFQG | RVTLTRDTSISTAYMELSSLRSEDTAVYYCAS | SSGWY---------------------YFDY | WGQGTLVTVSS |
| **iPS:441896** | **21-225_4A2.001.011** | **VH1\|1-08/D6\|6-19\|RF1/JH4** | QVQLVQS-GTEVKKPGASVKVSCKASG-YTFT | N-----YDIN | WVRQATGQGLEWMG | WMHPN---SGQTGYAQKFQG | RVTLTRDTSISTAYMELSSLRSEDTAVYYCAS | SSGWY---------------------YFDY | WGQGTLVTVSS |
| **iPS:441900** | **21-225_4A2.001.012** | **VH1\|1-08/D6\|6-19\|RF1/JH4** | QVQLVQS-GTEVKKPGASVKVSCKASG-YTFT | N-----YDIN | WVRQATGQGLEWMG | WMHPN---SGSTGYAQKFQG | RVTLTRDTSISTAYMELSSLRSEDTAVYYCAS | SSGWY---------------------YFDY | WGQGTLVTVSS |
| **iPS:441955** | **21-225_4A2.001.022** | **VH1\|1-08/D6\|6-19\|RF1/JH4** | QVQLVQS-GAEVKKPGASVKVSCKASG-YTFT | N-----YDIN | WVRQATGQGLEWMG | WMHPN---SGNTGYAQKFQG | RVTLTRDTSISTAYMELSSLRSEDTAVYYCAS | SSGWY---------------------YFDY | WGQGTLVTVSS |
| **iPS:441962** | **21-225_4A2.001.023** | **VH1\|1-08/D6\|6-19\|RF1/JH4** | QVQLVQS-GAEVKKPGASVKVSCKASG-YTFT | N-----YDIN | WVRQAPGQGLEWMG | WMHPN---SGNTGYAQKFQG | RVTLTRDTSISTAYMELSSLRSEDTAVYYCAS | SSGWY---------------------YFDY | WGQGTLVTVSS |
| **iPS:441971** | **21-225_4A2.001.024** | **VH1\|1-08/D6\|6-19\|RF1/JH4** | QVQLVQS-GAEVKKPGASVKVSCKASG-YTFT | N-----YDIN | WVRQATGQGLEWMG | WMHPN---SGQTGYAQKFQG | RVTLTRDTSISTAYMELSSLRSEDTAVYYCAS | SSGWY---------------------YFDY | WGQGTLVTVSS |
| **iPS:441999** | **21-225_4A2.** | **VH1\|1-08/D6\|6-** | QVQLVQS-GAEVKKPGASVKVSCK | N-----YDIN | WVRQAPGQGLEWMG | WMHPN---SGQTGYAQK | RVTLTRDTSISTAYMELSSLRSEDTAVYYCAS | SSGWY---------------------YFDY | WGQGTLVTVSS |

| | | | | | | | | | |
|---|---|---|---|---|---|---|---|---|---|
| iPS:44 2006 | 21-225_4A2.001.029 | VH1\|1-08/D6\|6-19\|RF1/JH4 | QVQLVQS-GAEVKKPGASVKVSCKASG-YTFT | N-----YDIN | WVRQAPGQGLEWMG | WMHPN---SGSTGYAQKFQG | RVTLTRDTSISTAYMELSSLRSEDTAVYYCAS | SSGWY------------------YFDY | WGQGTLVTVSS |
| iPS:44 2020 | 21-225_4A2.001.031 | VH1\|1-08/D6\|6-19\|RF1/JH4 | QVQLVQS-GGEVKKPGASVKVSCKASG-YTFT | N-----YDIN | WVRQAEGQGLEWMG | WMHPN---SGNEGYAQKFQG | RVTLTRDTSISTAYMELSSLRSEDTAVYYCAS | SSGWY------------------YFDY | WGQGTLVTVSS |
| | | Germline | H_FR1 | H_CDR1 | H_FR2 | H_CDR2 | H_FR3 | H_CDR3 | H_FR4 |
| | VH1\|1-02/D1\|1-1\|RF1/JH4 | | QVQLVQS-GAEVKKPGASVKVSCKASG-YTFT | G-----YYMH | WVRQAPGQGLEWMG | WINPN---SGGTNYAQKFQG | RVTMTRDTSISTAYMELSRLRSDDTAVYYCAR | GTTGT------------------YFDY | WGQGTLVTVSS |
| iPS:39 3954 | 21-225_4H6 | VH1\|1-02/D1\|1-1\|RF1/JH4 | QVQLVQS-GAEVKKPGASVKVSCKASG-YTFT | D-----YYLH | WVRQAPGQGLEWMG | WIHPN---SGGTNYAQKFQG | RVTMTRDTSISTAYMGLSSLRSDDTAVYYCAR | DGTS-------------------SFDY | WGQGTLVTVSS |
| iPS:44 2050 | 21-225_4H6.004 | VH1\|1-02/D1\|1-1\|RF1/JH4 | QVQLVQS-GAEVKKPGASVKVSCKASG-YTFT | D-----YYLH | WVRQAPGQGLEWMG | WIHPN---SGGTNYAQKFQG | RVTMTRDTSISTAYMELSSLRSDDTAVYYCAR | DGTS-------------------SFDY | WGQGTLVTVSS |
| iPS:44 2059 | 21-225_4H6.005 | VH1\|1-02/D1\|1-1\|RF1/JH4 | QVQLVQS-GAEVKKPGASVKVSCKASG-YTFT | D-----YYLH | WVRQAPGQGLEWMG | WIHPN---SGGTNYAQKFQG | RVTMTRDTSISTAYMELSSLRSDDTAVYYCAR | DGTS-------------------SFDY | WGQGTLVTVSS |
| iPS:44 2065 | 21-225_4H6.006 | VH1\|1-02/D1\|1-1\|RF1/JH4 | QVQLVQS-GAEVKKPGASVKVSCKASG-YTFT | D-----YYLH | WVRQAPGQGLEWMG | WIHPN---SGGTNYAQKFQG | RVTMTRDTSISTAYMELSSLRSDDTAVYYCAR | DGTS-------------------SFDY | WGQGTLVTVSS |
| iPS:44 2071 | 21-225_4H6.007 | VH1\|1-02/D1\|1-1\|RF1/JH4 | QVQLVQS-GAEVKKPGASVKVSCKASG-YTFT | D-----YYLH | WVRQAPGQGLEWMG | WIHPN---SGGTNYAQKFQG | RVTMTRDTSISTAYMELSSLRSDDTAVYYCAR | DATS-------------------SFDY | WGQGTLVTVSS |
| iPS:44 2078 | 21-225_4H6.008 | VH1\|1-02/D1\|1-1\|RF1/JH4 | QVQLVQS-GAEVKKPGASVKVSCKASG-YTFT | D-----YYLH | WVRQAPGQGLEWMG | WIHPN---SGGTNYAQKFQG | RVTMTRDTSISTAYMELSSLRSDDTAVYYCAR | SGTS-------------------SFDY | WGQGTLVTVSS |
| iPS:44 2085 | 21-225_4H6.009 | VH1\|1-02/D1\|1-1\|RF1/JH4 | QVQLVQS-GAEVKKPGASVKVSCKASG-YTFT | D-----YYLH | WVRQAPGQGLEWMG | WIHPN---SGGTNYAQKFQG | RVTMTRDTSISTAYMELSSLRSDDTAVYYCAR | SGTS-------------------SFDY | WGQGTLVTVSS |
| iPS:44 2089 | 21-225_4H6.010 | VH1\|1-02/D1\|1-1\|RF1/JH4 | QVQLVQS-GAEVKKPGASVKVSCKASG-YTFT | D-----YYLH | WVRQAPGQGLEWMG | WIHPN---SGGTNYAQKFQG | RVTMTRDTSISTAYMELSSLRSDDTAVYYCAR | DATS-------------------SFDY | WGQGTLVTVSS |

| | | | | | | | | | |
|---|---|---|---|---|---|---|---|---|---|
| **iPS:44 2093** | **21-225_4H6. 011** | **VH1\|1-02/D1\|1-1\|RF1/JH 4** | QVQLVQS- GAEVKKPGASVKVSCK ASG-YTFT | D-----YYLH | WVRQAPGQGLE WMG | WIHPN--- SGGTNYAQK FQG | RVTMTRDTSISTAYMELS SLRSDDTAVYYCAR | DATS------------- ---------SFDY | WGQGTL VTVSS |
| **iPS:44 3016** | **21-225_4H6. 014** | **VH1\|1-02/D1\|1-1\|RF1/JH 4** | QVQLVQS- GAEVKKPGASVKVSCK ASG-YTFT | D-----YYLH | WVRQAPGQGLE WMG | WIHPN--- SGGTNYAQK FQG | RVTMTRDTSISTAYMGLS SLRSDDTAVYYCAR | DATS------------- ---------SFDY | WGQGTL VTVSS |
| | | **Germline** | **H_FR1** | **H_CDR1** | **H_FR2** | **H_CDR2** | **H_FR3** | **H_CDR3** | **H_FR4** |
| | **VH3\|3-33/D6\|6-6\|RF1/JH 6** | | QVQLVES- GGGVVQPGRSLRLSCA ASG-FTFS | S-----YGMH | WVRQAPGKGLE WVA | VIWYD--- GSNKYYADS VKG | RFTISRDNSKNTLYLQMN SLRAEDTAVYYCAR | EYSSSSYY---------- ------YYYGMDV | WGQGTT VTVSS |
| **iPS:39 4041** | **21-225_5E5** | **VH3\|3-33/D6\|6-6\|RF1/JH 6** | QVQLVES- GGGVVQPGRSLRLSCA ASG-FTFS | N-----YVMH | WVRQAPGKGLE WVA | VIWYD--- GSNKYYADS VKG | RFTISRDNSKNTLYLQMN SLRAEDTAVYYCTR | EVYSSGW---------- ------YDYGMDV | WGQGTT VTVSS |
| **iPS:44 2115** | **21-225_5E5. 003** | **VH3\|3-33/D6\|6-6\|RF1/JH 6** | QVQLVES- GGGVVQPGRSLRLSCA ASG-FTFS | N-----YVMH | WVRQAPGKGLE WVA | VIWYD--- ASNKYYADS VKG | RFTISRDNSKNTLYLQMN SLRAEDTAVYYCTR | EVYSSGW---------- ------YDYGMDV | WGQGTT VTVSS |
| **iPS:44 2122** | **21-225_5E5. 004** | **VH3\|3-33/D6\|6-6\|RF1/JH 6** | QVQLVES- GGGVVQPGRSLRLSCA ASG-FTFS | N-----YVMH | WVRQAPGKGLE WVA | VIWYD--- GSNKYYAES VKG | RFTISRDNSKNTLYLQMN SLRAEDTAVYYCTR | EVYSSGW---------- ------YDYGMDV | WGQGTT VTVSS |
| **iPS:44 2129** | **21-225_5E5. 005** | **VH3\|3-33/D6\|6-6\|RF1/JH 6** | QVQLVES- GGGVVQPGRSLRLSCA ASG-FTFS | N-----YVMH | WVRQAPGKGLE WVA | VIWYD--- GSNKYYAGS VKG | RFTISRDNSKNTLYLQMN SLRAEDTAVYYCTR | EVYSSGW---------- ------YDYGMDV | WGQGTT VTVSS |
| **iPS:44 2136** | **21-225_5E5. 006** | **VH3\|3-33/D6\|6-6\|RF1/JH 6** | QVQLVES- GGGVVQPGRSLRLSCA ASG-FTFS | N-----YVMH | WVRQAPGKGLE WVA | VIWYD--- GSNKYYADA VKG | RFTISRDNSKNTLYLQMN SLRAEDTAVYYCTR | EVYSSGW---------- ------YDYGMDV | WGQGTT VTVSS |
| **iPS:44 2171** | **21-225_5E5. 011** | **VH3\|3-33/D6\|6-6\|RF1/JH 6** | QVQLVES- GGGVVQPGRSLRLSCA ASG-FTFS | N-----YVMH | WVRQAPGKGLE WVA | VIWYD--- ASNKYYAES VKG | RFTISRDNSKNTLYLQMN SLRAEDTAVYYCTR | EVYSSGW---------- ------YDYGMDV | WGQGTT VTVSS |
| **iPS:44 2178** | **21-225_5E5. 012** | **VH3\|3-33/D6\|6-6\|RF1/JH 6** | QVQLVES- GGGVVQPGRSLRLSCA ASG-FTFS | N-----YVMH | WVRQAPGKGLE WVA | VIWYD--- ASNKYYADA VKG | RFTISRDNSKNTLYLQMN SLRAEDTAVYYCTR | EVYSSGW---------- ------YDYGMDV | WGQGTT VTVSS |
| **iPS:44 2199** | **21-225_5E5. 015** | **VH3\|3-33/D6\|6-6\|RF1/JH 6** | QVQLVES- GGGVVQPGRSLRLSCA ASG-FTFS | N-----YVMH | WVRQAPGKGLE WVA | VIWYD--- ASNKYYADS VKG | RFTISRDNSKNTLYLQMN SLRAEDTAVYYCTR | EVYSSGY---------- ------YDYGMDV | WGQGTT VTVSS |

| | | | | | | | | | |
|---|---|---|---|---|---|---|---|---|---|
| iPS:44 2206 | 21-225_5E5.016 | VH3\|3-33/D6\|6-6\|RF1/JH6 | QVQLVES-GGGVVQPGRSLRLSCAASG-FTFS | N-----YVMH | WVRQAPGKGLEWVA | VIWYD---ASNKYYADSVKG | RFTISRDNSKNTLYLQMNSLRAEDTAVYYCTR | EVYSSGF----------------YDYGMDV | WGQGTTVTVSS |
| iPS:44 2213 | 21-225_5E5.017 | VH3\|3-33/D6\|6-6\|RF1/JH6 | QVQLVES-GGGVVQPGRSLRLSCAASG-FTFS | N-----YVMH | WVRQAPGKGLEWVA | VIWYD---GSNKYYAESVKG | RFTISRDNSKNTLYLQMNSLRAEDTAVYYCTR | EVYSSGY----------------YDYGMDV | WGQGTTVTVSS |
| iPS:44 2220 | 21-225_5E5.018 | VH3\|3-33/D6\|6-6\|RF1/JH6 | QVQLVES-GGGVVQPGRSLRLSCAASG-FTFS | N-----YVMH | WVRQAPGKGLEWVA | VIWYD---GSNKYYADSVKG | RFTISRDNSKNTLYLQMNSLRAEDTAVYYCTR | EVYSSGY----------------YDYGMDV | WGQGTTVTVSS |
| iPS:44 2227 | 21-225_5E5.019 | VH3\|3-33/D6\|6-6\|RF1/JH6 | QVQLVES-GGGVVQPGRSLRLSCAASG-FTFS | N-----YVMH | WVRQAPGKGLEWVA | VIWYD---GSNKYYADSVKG | RFTISRDNSKNTLYLQMNSLRAEDTAVYYCTR | EVYSSGF----------------YDYGMDV | WGQGTTVTVSS |
| iPS:44 2255 | 21-225_5E5.023 | VH3\|3-33/D6\|6-6\|RF1/JH6 | QVQLVES-GGGVVQPGRSLRLSCAASG-FTFS | N-----YVMH | WVRQAPGKGLEWVA | VIWYD---ASNKYYAESVKG | RFTISRDNSKNTLYLQMNSLRAEDTAVYYCTR | EVYSSGY----------------YDYGMDV | WGQGTTVTVSS |
| iPS:44 2262 | 21-225_5E5.024 | VH3\|3-33/D6\|6-6\|RF1/JH6 | QVQLVES-GGGVVQPGRSLRLSCAASG-FTFS | N-----YVMH | WVRQAPGKGLEWVA | VIWYD---ASNKYYADAVKG | RFTISRDNSKNTLYLQMNSLRAEDTAVYYCTR | EVYSSGY----------------YDYGMDV | WGQGTTVTVSS |
| | | Germline | H_FR1 | H_CDR1 | H_FR2 | H_CDR2 | H_FR3 | H_CDR3 | H_FR4 |
| | VH3\|3-07/D6\|6-6\|RF1/JH6 | | EVQLVES-GGGLVQPGGSLRLSCAASG-FTFS | S-----YWMS | WVRQAPGKGLEWVA | NIKQD---GSEKYYVDSVKG | RFTISRDNAKNSLYLQMNSLRAEDTAVYYCAR | EYSSSSYY---------------YYYGMDV | WGQGTTVTVSS |
| iPS:44 2269 | 21-225_5E5.025 | VH3\|3-07/D6\|6-6\|RF1/JH6 | EVQLVES-GGGVVQPGGSLRLSCAASG-FTFS | N-----YVMH | WVRQAPGKGLEWVA | VIWYD---GSNKYYAESVKG | RFTISRDNAKNTLYLQMNSLRAEDTAVYYCTR | EVYSSGW----------------YDYGMDV | WGQGTTVTVSS |
| | | Germline | H_FR1 | H_CDR1 | H_FR2 | H_CDR2 | H_FR3 | H_CDR3 | H_FR4 |
| | VH3\|3-33/D4\|4-11\|RF2/JH6 | | QVQLVES-GGGVVQPGRSLRLSCAASG-FTFS | S-----YGMH | WVRQAPGKGLEWVA | VIWYD---GSNKYYADSVKG | RFTISRDNSKNTLYLQMNSLRAEDTAVYYCAR | DYSNYYY----------------YYYGMDV | WGQGTTVTVSS |
| iPS:39 3930 | 21-225_7E11 | VH3\|3-33/D4\|4-11\|RF2/JH6 | QVQLVES-GGGVVQPGRSLRLSCAASG-FTFS | S-----FGMH | WVRQAPGKGLEWVA | IIWHD---GSNKYYADSVKG | RFTISRDNSNNTLYLQMSSLRAEDTAVYYCAR | DLSMG---------------------GMDV | WGQGTTVTVSS |
| iPS:42 4460 | 21-225_7E11.001 | VH3\|3-33/D4\|4-11\|RF2/JH6 | QVQLVES-GGGVVQPGRSLRLSCAASG-FTFS | S-----FGMH | WVRQAPGKGLEWVA | IIWHD---GSNKYYADSVKG | RFTISRDNSKNTLYLQMSSLRAEDTAVYYCAR | DLSMG---------------------GMDV | WGQGTTVTVSS |

| | | | | | | | | | |
|---|---|---|---|---|---|---|---|---|---|
| **iPS:44 2311** | **21-225_7E11 .001.001** | **VH3\|3-33/D4\|4-11\|RF2/JH6** | QVQLVES-GGGVVQPGRSLRLSCA ASG-FTFS | S-----FGMH | WVRQAPGKGLE WVA | IIWHD---GSNKYYADS VKG | RFTISRDNSKNTLYLQMS SLRAEDTAVYYCAR | DLSMG------------ ---------GMDV | WGQGTT VTVSS |
| **iPS:44 2317** | **21-225_7E11 .001.002** | **VH3\|3-33/D4\|4-11\|RF2/JH6** | QVQLVES-GGGVVQPGRSLRLSCA ASG-FTFS | S-----FGMH | WVRQAPGKGLE WVA | IIWHD---GSNKYYADS VKG | RFTISRDNSKNTLYLQMS SLRAEDTAVYYCAR | DLSMG------------ ---------GMDV | WGQGTT VTVSS |
| **iPS:44 2323** | **21-225_7E11 .001.003** | **VH3\|3-33/D4\|4-11\|RF2/JH6** | QVQLVES-GGGVVQPGRSLRLSCA ASG-FTFS | S-----FGMH | WVRQAPGKGLE WVA | IIWHS---GSNKYYADS VKG | RFTISRDNSKNTLYLQMS SLRAEDTAVYYCAR | DLSMG------------ ---------GMDV | WGQGTT VTVSS |
| **iPS:44 2330** | **21-225_7E11 .001.004** | **VH3\|3-33/D4\|4-11\|RF2/JH6** | QVQLVES-GGGVVQPGRSLRLSCA ASG-FTFS | S-----FGMH | WVRQAPGKGLE WVA | IIWHE---GSNKYYADS VKG | RFTISRDNSKNTLYLQMS SLRAEDTAVYYCAR | DLSMG------------ ---------GMDV | WGQGTT VTVSS |
| **iPS:44 2337** | **21-225_7E11 .001.005** | **VH3\|3-33/D4\|4-11\|RF2/JH6** | QVQLVES-GGGVVQPGRSLRLSCA ASG-FTFS | S-----FGMH | WVRQAPGKGLE WVA | IIWHD---ASNKYYADS VKG | RFTISRDNSKNTLYLQMS SLRAEDTAVYYCAR | DLSMG------------ ---------GMDV | WGQGTT VTVSS |
| **iPS:44 2344** | **21-225_7E11 .001.006** | **VH3\|3-33/D4\|4-11\|RF2/JH6** | QVQLVES-GGGVVQPGRSLRLSCA ASG-FTFS | S-----FGMH | WVRQAPGKGLE WVA | IIWHD---GSNKYYAES VKG | RFTISRDNSKNTLYLQMS SLRAEDTAVYYCAR | DLSMG------------ ---------GMDV | WGQGTT VTVSS |
| **iPS:44 2351** | **21-225_7E11 .001.007** | **VH3\|3-33/D4\|4-11\|RF2/JH6** | QVQLVES-GGGVVQPGRSLRLSCA ASG-FTFS | S-----FGMH | WVRQAPGKGLE WVA | IIWHD---GSNKYYADA VKG | RFTISRDNSKNTLYLQMS SLRAEDTAVYYCAR | DLSMG------------ ---------GMDV | WGQGTT VTVSS |
| **iPS:44 2358** | **21-225_7E11 .001.008** | **VH3\|3-33/D4\|4-11\|RF2/JH6** | QVQLVES-GGGVVQPGRSLRLSCA ASG-FTFS | S-----FGMH | WVRQAPGKGLE WVA | IIWHE---GSNKYYADA VKG | RFTISRDNSKNTLYLQMS SLRAEDTAVYYCAR | DLSMG------------ ---------GMDV | WGQGTT VTVSS |
| **iPS:44 2365** | **21-225_7E11 .001.009** | **VH3\|3-33/D4\|4-11\|RF2/JH6** | QVQLVES-GGGVVQPGRSLRLSCA ASG-FTFS | S-----FGMH | WVRQAPGKGLE WVA | IIWHE---GSNKYYAES VKG | RFTISRDNSKNTLYLQMS SLRAEDTAVYYCAR | DLSMG------------ ---------GMDV | WGQGTT VTVSS |
| **iPS:44 2372** | **21-225_7E11 .001.010** | **VH3\|3-33/D4\|4-11\|RF2/JH6** | QVQLVES-GGGVVQPGRSLRLSCA ASG-FTFS | S-----FGMH | WVRQAPGKGLE WVA | IIWHS---GSNKYYAES VKG | RFTISRDNSKNTLYLQMS SLRAEDTAVYYCAR | DLSMG------------ ---------GMDV | WGQGTT VTVSS |
| **iPS:44 2379** | **21-225_7E11 .001.011** | **VH3\|3-33/D4\|4-11\|RF2/JH6** | QVQLVES-GGGVVQPGRSLRLSCA ASG-FTFS | S-----FGMH | WVRQAPGKGLE WVA | IIWHS---GSNKYYADA VKG | RFTISRDNSKNTLYLQMS SLRAEDTAVYYCAR | DLSMG------------ ---------GMDV | WGQGTT VTVSS |
| **iPS:44 2386** | **21-225_7E11** | **VH3\|3-33/D4\|4-** | QVQLVES-GGGVVQPGRSLRLSCA | S-----FGMH | WVRQAPGKGLE WVA | IIWHS---GSNKYYADS | RFTISRDNSKNTLYLQMS SLRAEDTAVYYCAR | DLSMG------------ ---------GMDV | WGQGTT VTVSS |

| | | | | | | | | | |
|---|---|---|---|---|---|---|---|---|---|
| **iPS:442390** | **21-225_7E11.001.013** | **VH3\|3-33/D4\|4-11\|RF2/JH6** | QVQLVES-GGGVVQPGRSLRLSCAASG-FTFS | S-----FGMH | WVRQAPGKGLEWVA | IIWHD---GSNKYYAESVKG | RFTISRDNSKNTLYLQMSSLRAEDTAVYYCAR | DLSMG------------ ---------GMDV | WGQGTTVTVSS |
| **iPS:442394** | **21-225_7E11.001.014** | **VH3\|3-33/D4\|4-11\|RF2/JH6** | QVQLVES-GGGVVQPGRSLRLSCAASG-FTFS | S-----FGMH | WVRQAPGKGLEWVA | IIWHS---GSNKYYAESVKG | RFTISRDNSKNTLYLQMSSLRAEDTAVYYCAR | DLSMG------------ ---------GMDV | WGQGTTVTVSS |
| **iPS:442398** | **21-225_7E11.001.015** | **VH3\|3-33/D4\|4-11\|RF2/JH6** | QVQLVES-GGGVVQPGRSLRLSCAASG-FTFS | S-----FGMH | WVRQAPGKGLEWVA | IIWHS---GSNKYYADSVKG | RFTISRDNSKNTLYLQMSSLRAEDTAVYYCAR | DLSMG------------ ---------GMDV | WGQGTTVTVSS |
| **iPS:442402** | **21-225_7E11.001.016** | **VH3\|3-33/D4\|4-11\|RF2/JH6** | QVQLVES-GGGVVQPGRSLRLSCAASG-FTFS | S-----FGMH | WVRQAPGKGLEWVA | IIWHS---GSNKYYAESVKG | RFTISRDNSKNTLYLQMSSLRAEDTAVYYCAR | DLSMG------------ ---------GMDV | WGQGTTVTVSS |
| **iPS:442406** | **21-225_7E11.001.017** | **VH3\|3-33/D4\|4-11\|RF2/JH6** | QVQLVES-GGGVVQPGRSLRLSCAASG-FTFS | S-----FGMH | WVRQAPGKGLEWVA | IIWHE---GSNKYYADAVKG | RFTISRDNSKNTLYLQMSSLRAEDTAVYYCAR | DLSMG------------ ---------GMDV | WGQGTTVTVSS |
| **iPS:442410** | **21-225_7E11.001.018** | **VH3\|3-33/D4\|4-11\|RF2/JH6** | QVQLVES-GGGVVQPGRSLRLSCAASG-FTFS | S-----FGMH | WVRQAPGKGLEWVA | IIWHD---ASNKYYAESVKG | RFTISRDNSKNTLYLQMSSLRAEDTAVYYCAR | DLSMG------------ ---------GMDV | WGQGTTVTVSS |
| **iPS:443027** | **21-225_7E11.001.023** | **VH3\|3-33/D4\|4-11\|RF2/JH6** | QVQLVES-GGGVVQPGRSLRLSCAASG-FTFS | S-----FGMH | WVRQAPGKGLEWVA | IIWHD---ASNKYYADAVKG | RFTISRDNSKNTLYLQMSSLRAEDTAVYYCAR | DLSMG------------ ---------GMDV | WGQGTTVTVSS |
| | | **Germline** | **H_FR1** | **H_CDR1** | **H_FR2** | **H_CDR2** | **H_FR3** | **H_CDR3** | **H_FR4** |
| | **VH3\|3-07/D4\|4-11\|RF2/JH6** | | EVQLVES-GGGLVQPGGSLRLSCAASG-FTFS | S-----YWMS | WVRQAPGKGLEWVA | NIKQD---GSEKYYVDSVKG | RFTISRDNAKNSLYLQMNSLRAEDTAVYYCAR | DYSNYYY---------- ------YYYGMDV | WGQGTTVTVSS |
| **iPS:442417** | **21-225_7E11.001.019** | **VH3\|3-07/D4\|4-11\|RF2/JH6** | EVQLVES-GGGVVQPGGSLRLSCAASG-FTFS | S-----FGMH | WVRQAPGKGLEWVA | IIWHD---GSNKYYAESVKG | RFTISRDNAKNTLYLQMSSLRAEDTAVYYCAR | DLSMG------------ ---------GMDV | WGQGTTVTVSS |
| **iPS:442431** | **21-225_7E11.001.021** | **VH3\|3-07/D4\|4-11\|RF2/JH6** | EVQLVES-GGGVVQPGGSLRLSCAASG-FTFS | S-----FGMH | WVRQAPGKGLEWVA | IIWHS---GSNKYYAESVKG | RFTISRDNAKNTLYLQMSSLRAEDTAVYYCAR | DLSMG------------ ---------GMDV | WGQGTTVTVSS |
| **iPS:442438** | **21-225_7E11.001.022** | **VH3\|3-07/D4\|4-11\|RF2/JH6** | EVQLVES-GGGVVQPGGSLRLSCAASG-FTFS | S-----FGMH | WVRQAPGKGLEWVA | IIWHE---GSNKYYAESVKG | RFTISRDNAKNTLYLQMSSLRAEDTAVYYCAR | DLSMG------------ ---------GMDV | WGQGTTVTVSS |

Figure 54 (Table 7)
Standard IgG Antibody Variable Region Consensus Protein Alignment
CDRs defined by Kabat
Alignment numbering defined by Amgen Reference (AHo)

| KAPPA_ VARIABLE | | Germline | K_FR1 | K_CDR1 | K_FR2 | K_CDR2 | K_FR3 | K_CDR3 | K_FR4 |
|---|---|---|---|---|---|---|---|---|---|
| | VK4\|B3/J K1 | | DIVMTQSPDSLAV SLGERATINC | KSS-- QSVLYSSNNKNYLA | WYQQKPGQPPK LLIY | W-------- ASTRES | GVPDRFSGSGSG-- TDFTLTISSLQAEDVAV YYC | QQYYS------------ ----------TPWT | FGQGTKV EIK |
| iPS:39 2928 | 21- 225_25A 4 | VK4\|B3/J K1 | ......F...... ........K. | ............H. N.... | ........... ..L. | ......... ..... | ...............E. .......E......... | ................... ............P. | ....... ... |
| iPS:42 4419 | 21- 225_25A 4.001 | VK4\|B3/J K1 | ......F...... ........K. | ............H. N.... | ........... ..L. | ......... ..... | ...............E. .......E......... | ................... ............P. | ....... ... |
| iPS:44 1468 | 21- 225_25A 4.001.001 | VK4\|B3/J K1 | ......F...... ........K. | ............H. N.... | ........... ..L. | ......... ..... | ...............E. .......E......... | ................... ............P. | ....... ... |
| iPS:44 1475 | 21- 225_25A 4.001.002 | VK4\|B3/J K1 | ......F...... ........K. | ............H. N.... | ........... ..L. | ......... ..... | ...............E. .......E......... | ................... ............P. | ....... ... |
| iPS:44 1482 | 21- 225_25A 4.001.003 | VK4\|B3/J K1 | ......F...... ........K. | ............H. N.... | ........... ..L. | ......... ..... | ...............E. .......E......... | ................... ............P. | ....... ... |
| iPS:44 1489 | 21- 225_25A 4.001.004 | VK4\|B3/J K1 | ......F...... ........K. | ............H. N.... | ........... ..L. | ......... ..... | ...............E. .......E......... | ................... ............P. | ....... ... |
| iPS:44 1496 | 21- 225_25A 4.001.005 | VK4\|B3/J K1 | ............. ........K. | ............H. N.... | ........... ..L. | ......... ..... | ...............E. ................. | ................... ............P. | ....... ... |
| iPS:44 1505 | 21- 225_25A 4.001.006 | VK4\|B3/J K1 | ............. ........K. | ............H. N.... | ........... ..L. | ......... ..... | ...............E. ................. | ................... ............P. | ....... ... |
| iPS:44 1512 | 21- 225_25A 4.001.007 | VK4\|B3/J K1 | ............. ........K. | ............H. N.... | ........... ..L. | ......... ..... | ...............E. ................. | ................... ............P. | ....... ... |
| iPS:44 1519 | 21- 225_25A 4.001.008 | VK4\|B3/J K1 | ............. ........K. | ............H. N.... | ........... ..L. | ......... ..... | ...............E. ................. | ................... ............P. | ....... ... |
| iPS:44 1554 | 21- 225_25A 4.001.013 | VK4\|B3/J K1 | ......F...... ........K. | ............H. N.... | ........... ..L. | ......... ..... | ...............E. ................. | ................... ............P. | ....... ... |
| iPS:44 1595 | 21- 225_25A 4.001.010 | VK4\|B3/J K1 | ............. .......... | ............H. N.... | ........... ..L. | ......... ..... | ...............E. .......E......... | ................... ............P. | ....... ... |

| | | | | | | | | | |
|---|---|---|---|---|---|---|---|---|---|
| iPS:44 1604 | 21-225_25A 4.001.020 | VK4\|B3/J K1 | .............<br>.......... | ............H.<br>N.... | ...........<br>..L. | .........<br>.... | ...............E.<br>................ | .................<br>............P. | .......<br>... |
| iPS:44 1613 | 21-225_25A 4.001.021 | VK4\|B3/J K1 | .............<br>.......... | ............H.<br>N.... | ...........<br>..L. | .........<br>.... | ...............E.<br>................ | .................<br>............P. | .......<br>... |
| iPS:44 3006 | 21-225_25A 4.001.029 | VK4\|B3/J K1 | ......F......<br>........K. | ............H.<br>N.... | ...........<br>..L. | .........<br>.... | ...............E.<br>................ | .................<br>............P. | .......<br>... |
| iPS:44 1962 | 21-225_4A2. 001.023 | VK4\|B3/J K1 | .............<br>.......... | .......I.H....<br>N.... | .F.........<br>..L. | .........<br>.... | ................<br>........P....... | .................<br>............V. | .......<br>... |
| iPS:44 1999 | 21-225_4A2. 001.028 | VK4\|B3/J K1 | .............<br>.......... | .......I.H....<br>N.... | .F.........<br>..L. | .........<br>.... | ................<br>........P....... | .................<br>............V. | .......<br>... |
| iPS:44 2006 | 21-225_4A2. 001.029 | VK4\|B3/J K1 | .............<br>.......... | .......I.H....<br>N.... | .F.........<br>..L. | .........<br>.... | ................<br>........P....... | .................<br>............V. | .......<br>... |
| iPS:44 2020 | 21-225_4A2. 001.031 | VK4\|B3/J K1 | .............<br>.......... | .......I.H....<br>N.... | .F.........<br>..L. | .........<br>.... | ................<br>........P....... | ....N............<br>............V. | ..P....<br>... |
| | | Germline | K_FR1 | K_CDR1 | K_FR2 | K_CDR2 | K_FR3 | K_CDR3 | K_FR4 |
| | VK1\|L5/J K3 | | DIQMTQSPSSVSA<br>SVGDRVTITC | RAS--QGIS-----<br>-SWLA | WYQQKPGKAPK<br>LLIY | A--------<br>ASSLQS | GVPSRFSGSGSG--<br>TDFTLTISSLQPEDFAT<br>YYC | QQANS------------<br>----------FPFT | FGPGTKV<br>DIK |
| iPS:39 3954 | 21-225_4H6 | VK1\|L5/J K3 | .............<br>.......... | ..............<br>.R... | ...........<br>.... | G........<br>..... | ................<br>.............I... | .................<br>............. | .......<br>... |
| iPS:44 2050 | 21-225_4H6. 004 | VK1\|L5/J K3 | .............<br>.......... | ..............<br>.R... | ...........<br>.... | G........<br>..... | ................<br>.............I... | .................<br>............. | .......<br>... |
| iPS:44 2059 | 21-225_4H6. 005 | VK1\|L5/J K3 | .............<br>.......... | ..............<br>.R... | ...........<br>.... | G........<br>..... | ................<br>.............I... | .................<br>............. | ..Q....<br>... |
| iPS:44 2065 | 21-225_4H6. 006 | VK1\|L5/J K3 | .............<br>.......... | ..............<br>.R... | ...........<br>.... | G........<br>..... | ................<br>................ | .................<br>............. | ..Q....<br>... |
| iPS:44 2071 | 21-225_4H6. 007 | VK1\|L5/J K3 | .............<br>.......... | ..............<br>.R... | ...........<br>.... | G........<br>..... | ................<br>.............I... | .................<br>............. | .......<br>... |
| iPS:44 2078 | 21-225_4H6. 008 | VK1\|L5/J K3 | .............<br>.......... | ..............<br>.R... | ...........<br>.... | G........<br>..... | ................<br>.............I... | .................<br>............. | .......<br>... |
| iPS:44 2085 | 21-225_4H6. 009 | VK1\|L5/J K3 | .............<br>.......... | ..............<br>.R... | ...........<br>.... | G........<br>..... | ................<br>.............I... | .................<br>............. | ..Q....<br>... |

| | | | | | | | | | |
|---|---|---|---|---|---|---|---|---|---|
| iPS:44<br>2089 | 21-<br>225_4H6.<br>010 | VK1\|L5/J<br>K3 | .............<br>.......... | ...............<br>.R... | ...........<br>.... | G........<br>..... | .................<br>..............I... | ..................<br>.............. | ..Q....<br>... |
| iPS:44<br>2093 | 21-<br>225_4H6.<br>011 | VK1\|L5/J<br>K3 | .............<br>.......... | ...............<br>.R... | ...........<br>.... | G........<br>..... | .................<br>................. | ..................<br>.............. | ..Q....<br>... |
| iPS:44<br>3016 | 21-<br>225_4H6.<br>014 | VK1\|L5/J<br>K3 | .............<br>.......... | ...............<br>.R... | ...........<br>.... | G........<br>..... | .................<br>................. | ..................<br>.............. | .......<br>... |
| | | Germline | K_FR1 | K_CDR1 | K_FR2 | K_CDR2 | K_FR3 | K_CDR3 | K_FR4 |
| | VK4\|B3/J<br>K3 | | DIVMTQSPDSLAV<br>SLGERATINC | KSS--<br>QSVLYSSNNKNYLA | WYQQKPGQPPK<br>LLIY | W--------<br>ASTRES | GVPDRFSGSGSG--<br>TDFTLTISSLQAEDVAV<br>YYC | QQYYS------------<br>----------TPFT | FGPGTKV<br>DIK |
| iPS:41<br>2232 | 21-<br>225_4A2 | VK4\|B3/J<br>K3 | .............<br>.......... | .......I.H....<br>N.... | .F.........<br>..L. | ..........<br>..... | .................<br>........P........ | ....N............<br>...........V. | .......<br>G.. |
| iPS:42<br>2894 | 21-<br>225_4A2.<br>001 | VK4\|B3/J<br>K3 | .............<br>.......... | .......I.H....<br>N.... | .F.........<br>..L. | ..........<br>..... | .................<br>........P........ | ....N............<br>...........V. | .......<br>G.. |
| iPS:44<br>1841 | 21-<br>225_4A2.<br>001.001 | VK4\|B3/J<br>K3 | .............<br>.......... | .......I.H....<br>N.... | .F.........<br>..L. | ..........<br>..... | .................<br>........P........ | .................<br>...........V. | .......<br>G.. |
| iPS:44<br>1847 | 21-<br>225_4A2.<br>001.002 | VK4\|B3/J<br>K3 | .............<br>.......... | .......I.H....<br>N.... | .F.........<br>..L. | ..........<br>..... | .................<br>........P........ | ....N............<br>.........A.V. | .......<br>G.. |
| iPS:44<br>1853 | 21-<br>225_4A2.<br>001.003 | VK4\|B3/J<br>K3 | .............<br>.......... | .......I.H....<br>N.... | .F.........<br>..L. | ..........<br>..... | .................<br>........P........ | ....Q............<br>...........V. | .......<br>G.. |
| iPS:44<br>1859 | 21-<br>225_4A2.<br>001.004 | VK4\|B3/J<br>K3 | .............<br>.......... | .......I.H....<br>N.... | .F.........<br>..L. | ..........<br>..... | .................<br>........P........ | ....N............<br>...........V. | .......<br>G.. |
| iPS:44<br>1866 | 21-<br>225_4A2.<br>001.005 | VK4\|B3/J<br>K3 | .............<br>.......... | .......I.H....<br>N.... | .F.........<br>..L. | ..........<br>..... | .................<br>........P........ | ....N............<br>...........V. | .......<br>G.. |
| iPS:44<br>1873 | 21-<br>225_4A2.<br>001.006 | VK4\|B3/J<br>K3 | .............<br>.......... | .......I.H....<br>N.... | .F.........<br>..L. | ..........<br>..... | .................<br>........P........ | ....N............<br>...........V. | .......<br>G.. |
| iPS:44<br>1880 | 21-<br>225_4A2.<br>001.007 | VK4\|B3/J<br>K3 | .............<br>.......... | .......I.H....<br>N.... | .F.........<br>..L. | ..........<br>..... | .................<br>........P........ | .................<br>...........V. | .......<br>G.. |
| iPS:44<br>1884 | 21-<br>225_4A2.<br>001.008 | VK4\|B3/J<br>K3 | .............<br>.......... | .......I.H....<br>N.... | .F.........<br>..L. | ..........<br>..... | .................<br>........P........ | .................<br>...........V. | .......<br>G.. |
| iPS:44<br>1888 | 21-<br>225_4A2.<br>001.009 | VK4\|B3/J<br>K3 | .............<br>.......... | .......I.H....<br>N.... | .F.........<br>..L. | ..........<br>..... | .................<br>........P........ | ....N............<br>.........A.V. | .......<br>G.. |

| | | | | | | | | | |
|---|---|---|---|---|---|---|---|---|---|
| iPS:44 1892 | 21-225_4A2.001.010 | VK4\|B3/JK3 | .............<br>.......... | .......I.H....<br>N.... | .F.........<br>..L. | ..........<br>..... | ................<br>........P....... | ....Q............<br>...........V. | .......<br>G.. |
| iPS:44 1896 | 21-225_4A2.001.011 | VK4\|B3/JK3 | .............<br>.......... | .......I.H....<br>N.... | .F.........<br>..L. | ..........<br>..... | ................<br>........P....... | ....Q............<br>...........V. | .......<br>G.. |
| iPS:44 1900 | 21-225_4A2.001.012 | VK4\|B3/JK3 | .............<br>.......... | .......I.H....<br>N.... | .F.........<br>..L. | ..........<br>..... | ................<br>........P....... | ....Q............<br>...........V. | .......<br>G.. |
| iPS:44 1955 | 21-225_4A2.001.022 | VK4\|B3/JK3 | .............<br>.......... | .......I.H....<br>N.... | .F.........<br>..L. | ..........<br>..... | ................<br>........P....... | .................<br>...........V. | .......<br>G.. |
| iPS:44 1971 | 21-225_4A2.001.024 | VK4\|B3/JK3 | .............<br>.......... | .......I.H....<br>N.... | .F.........<br>..L. | ..........<br>..... | ................<br>........P....... | .................<br>...........V. | .......<br>G.. |
| | | Germline | K_FR1 | K_CDR1 | K_FR2 | K_CDR2 | K_FR3 | K_CDR3 | K_FR4 |
| | VK1\|A30/JK1 | | DIQMTQSPSSLSA<br>SVGDRVTITC | RAS--QGIR-----<br>-NDLG | WYQQKPGKAPK<br>RLIY | A--------<br>ASSLQS | GVPSRFSGSGSG--<br>TEFTLTISSLQPEDFAT<br>YYC | LQHNS------------<br>----------YPWT | FGQGTKV<br>EIK |
| iPS:39 4041 | 21-225_5E5 | VK1\|A30/JK1 | .............<br>.......... | ..............<br>..... | ...........<br>.... | ..........<br>..... | ................<br>................ | ...Y.............<br>...........R. | .......<br>.V. |
| iPS:44 2115 | 21-225_5E5.003 | VK1\|A30/JK1 | .............<br>.......... | ..............<br>..... | ...........<br>.... | ..........<br>..... | ................<br>................ | ...Y.............<br>...........R. | .......<br>.V. |
| iPS:44 2122 | 21-225_5E5.004 | VK1\|A30/JK1 | .............<br>.......... | ..............<br>..... | ...........<br>.... | ..........<br>..... | ................<br>................ | ...Y.............<br>...........R. | .......<br>.V. |
| iPS:44 2129 | 21-225_5E5.005 | VK1\|A30/JK1 | .............<br>.......... | ..............<br>..... | ...........<br>.... | ..........<br>..... | ................<br>................ | ...Y.............<br>...........R. | .......<br>.V. |
| iPS:44 2136 | 21-225_5E5.006 | VK1\|A30/JK1 | .............<br>.......... | ..............<br>..... | ...........<br>.... | ..........<br>..... | ................<br>................ | ...Y.............<br>...........R. | .......<br>.V. |
| iPS:44 2171 | 21-225_5E5.011 | VK1\|A30/JK1 | .............<br>.......... | ..............<br>..... | ...........<br>.... | ..........<br>..... | ................<br>................ | ...Y.............<br>...........R. | .......<br>.V. |
| iPS:44 2178 | 21-225_5E5.012 | VK1\|A30/JK1 | .............<br>.......... | ..............<br>..... | ...........<br>.... | ..........<br>..... | ................<br>................ | ...Y.............<br>...........R. | .......<br>.V. |
| iPS:44 2199 | 21-225_5E5.015 | VK1\|A30/JK1 | .............<br>.......... | ..............<br>..... | ...........<br>.... | ..........<br>..... | ................<br>................ | ...Y.............<br>...........R. | .......<br>.V. |
| iPS:44 2206 | 21-225_5E5.016 | VK1\|A30/JK1 | .............<br>.......... | ..............<br>..... | ...........<br>.... | ..........<br>..... | ................<br>................ | ...Y.............<br>...........R. | .......<br>.V. |

| | | | | | | | | | |
|---|---|---|---|---|---|---|---|---|---|
| iPS:44 2213 | 21-225_5E5.017 | VK1\|A30/JK1 | ............. .......... | ............. ..... | ........... .... | .......... ..... | ................. ................. | ...Y............ ...........R. | ....... .V. |
| iPS:44 2220 | 21-225_5E5.018 | VK1\|A30/JK1 | ............. .......... | ............. ..... | ........... .... | .......... ..... | ................. ................. | ...Y............ ...........R. | ....... .V. |
| iPS:44 2227 | 21-225_5E5.019 | VK1\|A30/JK1 | ............. .......... | ............. ..... | ........... .... | .......... ..... | ................. ................. | ...Y............ ...........R. | ....... .V. |
| iPS:44 2255 | 21-225_5E5.023 | VK1\|A30/JK1 | ............. .......... | ............. ..... | ........... .... | .......... ..... | ................. ................. | ...Y............ ...........R. | ....... .V. |
| iPS:44 2262 | 21-225_5E5.024 | VK1\|A30/JK1 | ............. .......... | ............. ..... | ........... .... | .......... ..... | ................. ................. | ...Y............ ...........R. | ....... .V. |
| iPS:44 2269 | 21-225_5E5.025 | VK1\|A30/JK1 | ............. .......... | ............. ..... | ........... .... | .......... ..... | ................. ................. | ...Y............ ...........R. | ....... .V. |
| | | Germline | K_FR1 | K_CDR1 | K_FR2 | K_CDR2 | K_FR3 | K_CDR3 | K_FR4 |
| | VK1\|O12/JK4 | | DIQMTQSPSSLSA SVGDRVTITC | RAS--QSIS----- -SYLN | WYQQKPGKAPK LLIY | A-------- ASSLQS | GVPSRFSGSGSG-- TDFTLTISSLQPEDFAT YYC | QQSYS------------ ---------TPLT | FGGGTKV EIK |
| iPS:39 3930 | 21-225_7E11 | VK1\|O12/JK4 | ............. ........A. | ......N.I..... ..... | ........... F... | T......... ..... | ................. .............I... | ..T.............. ............. | ....... ... |
| iPS:42 4460 | 21-225_7E11.001 | VK1\|O12/JK4 | ............. ........A. | ......N.I..... ..... | ........... F... | T......... ..... | ................. .............I... | ..T.............. ............. | ....... ... |
| iPS:44 2311 | 21-225_7E11.001.001 | VK1\|O12/JK4 | ............. .......... | ......N.I..... ..... | ........... F... | T......... ..... | ................. .............I... | ..T.............. ............. | ....... ... |
| iPS:44 2317 | 21-225_7E11.001.002 | VK1\|O12/JK4 | ............. .......... | ......N.I..... ..... | ........... .... | T......... ..... | ................. ................. | ..T.............. ............. | ....... ... |
| iPS:44 2323 | 21-225_7E11.001.003 | VK1\|O12/JK4 | ............. ........A. | ......N.I..... ..... | ........... F... | T......... ..... | ................. .............I... | ..T.............. ............. | ....... ... |
| iPS:44 2330 | 21-225_7E11.001.004 | VK1\|O12/JK4 | ............. ........A. | ......N.I..... ..... | ........... F... | T......... ..... | ................. .............I... | ..T.............. ............. | ....... ... |
| iPS:44 2337 | 21-225_7E11.001.005 | VK1\|O12/JK4 | ............. ........A. | ......N.I..... ..... | ........... F... | T......... ..... | ................. .............I... | ..T.............. ............. | ....... ... |
| iPS:44 2344 | 21-225_7E11.001.006 | VK1\|O12/JK4 | ............. ........A. | ......N.I..... ..... | ........... F... | T......... ..... | ................. .............I... | ..T.............. ............. | ....... ... |

| | | | | | | | | | |
|---|---|---|---|---|---|---|---|---|---|
| iPS:44 2351 | 21-225_7E1 1.001.007 | VK1\|O12/ JK4 | ............. ........A. | ......N.I..... ..... | ........... F... | T........ ..... | ................. .............I... | ..T.............. .............. | ....... ... |
| iPS:44 2358 | 21-225_7E1 1.001.008 | VK1\|O12/ JK4 | ............. ........A. | ......N.I..... ..... | ........... F... | T........ ..... | ................. .............I... | ..T.............. .............. | ....... ... |
| iPS:44 2365 | 21-225_7E1 1.001.009 | VK1\|O12/ JK4 | ............. ........A. | ......N.I..... ..... | ........... F... | T........ ..... | ................. .............I... | ..T.............. .............. | ....... ... |
| iPS:44 2372 | 21-225_7E1 1.001.010 | VK1\|O12/ JK4 | ............. ........A. | ......N.I..... ..... | ........... F... | T........ ..... | ................. .............I... | ..T.............. .............. | ....... ... |
| iPS:44 2379 | 21-225_7E1 1.001.011 | VK1\|O12/ JK4 | ............. ........A. | ......N.I..... ..... | ........... F... | T........ ..... | ................. .............I... | ..T.............. .............. | ....... ... |
| iPS:44 2386 | 21-225_7E1 1.001.012 | VK1\|O12/ JK4 | ............. .......... | ......N.I..... ..... | ........... F... | T........ ..... | ................. .............I... | ..T.............. .............. | ....... ... |
| iPS:44 2390 | 21-225_7E1 1.001.013 | VK1\|O12/ JK4 | ............. .......... | ......N.I..... ..... | ........... F... | T........ ..... | ................. .............I... | ..T.............. .............. | ....... ... |
| iPS:44 2394 | 21-225_7E1 1.001.014 | VK1\|O12/ JK4 | ............. .......... | ......N.I..... ..... | ........... F... | T........ ..... | ................. .............I... | ..T.............. .............. | ....... ... |
| iPS:44 2398 | 21-225_7E1 1.001.015 | VK1\|O12/ JK4 | ............. .......... | ......N.I..... ..... | ........... .... | T........ ..... | ................. ................. | ..T.............. .............. | ....... ... |
| iPS:44 2402 | 21-225_7E1 1.001.016 | VK1\|O12/ JK4 | ............. .......... | ......N.I..... ..... | ........... .... | T........ ..... | ................. ................. | ..T.............. .............. | ....... ... |
| iPS:44 2406 | 21-225_7E1 1.001.017 | VK1\|O12/ JK4 | ............. .......... | ......N.I..... ..... | ........... .... | T........ ..... | ................. ................. | ..T.............. .............. | ....... ... |
| iPS:44 2410 | 21-225_7E1 1.001.018 | VK1\|O12/ JK4 | ............. .......... | ......N.I..... ..... | ........... F... | T........ ..... | ................. .............I... | ..T.............. .............. | ....... ... |
| iPS:44 2417 | 21-225_7E1 1.001.019 | VK1\|O12/ JK4 | ............. ........A. | ......N.I..... ..... | ........... F... | T........ ..... | ................. .............I... | ..T.............. .............. | ....... ... |
| iPS:44 2431 | 21-225_7E1 1.001.021 | VK1\|O12/ JK4 | ............. ........A. | ......N.I..... ..... | ........... F... | T........ ..... | ................. .............I... | ..T.............. .............. | ....... ... |
| iPS:44 2438 | 21-225_7E1 1.001.022 | VK1\|O12/ JK4 | ............. .......... | ......N.I..... ..... | ........... F... | T........ ..... | ................. .............I... | ..T.............. .............. | ....... ... |

| | | | | | | | | | |
|---|---|---|---|---|---|---|---|---|---|
| iPS:44 3027 | 21-225_7E1 1.001.023 | VK1\|O12/ JK4 | ............. ........A. | ......N.I..... ..... | ........... F... | T......... ..... | ................. .............I... | ..T............... ............... | ....... ... |
| HEAVY_VARIABLE | | | | | | | | | |
| | | Germline | H_FR1 | H_CDR1 | H_FR2 | H_CDR2 | H_FR3 | H_CDR3 | H_FR4 |
| | VH1\|1-08/D6\|6-19\|RF1/J H4 | | QVQLVQS-GAEVKKPGASVKVSCKASG-YTFT | S-----YDIN | WVRQATGQGLEWMG | WMNPN---SGNTGYAQKFQG | RVTMTRNTSISTAYMELSSLRSEDTAVYYCAR | GYSSGW-----------------YYFDY | WGQGTLVTVSS |
| iPS:39 2928 | 21-225_25A 4 | VH1\|1-08/D6\|6-19\|RF1/J H4 | ..L.......... R............ ...... | N......... | ........... ... | ..Y....... .......... | ................. ..............S | SSGWY- .................. .-.... | ....... .... |
| iPS:42 4419 | 21-225_25A 4.001 | VH1\|1-08/D6\|6-19\|RF1/J H4 | ..L.......... R............ ...... | N......... | ........... ... | ..Y....... .......... | ......D.......... ..............S | SSGWY- .................. .-.... | ....... .... |
| iPS:44 1468 | 21-225_25A 4.001.001 | VH1\|1-08/D6\|6-19\|RF1/J H4 | ..L.......... R............ ...... | N......... | ........... ... | ..Y....... S......... | ......D.......... ..............S | SSGWY- .................. .-.... | ....... .... |
| iPS:44 1475 | 21-225_25A 4.001.002 | VH1\|1-08/D6\|6-19\|RF1/J H4 | ..L.......... R............ ...... | N......... | ........... ... | ..Y....... .A........ | ......D.......... ..............S | SSGWY- .................. .-.... | ....... .... |
| iPS:44 1482 | 21-225_25A 4.001.003 | VH1\|1-08/D6\|6-19\|RF1/J H4 | ..L.......... R............ ...... | N......... | ........... ... | ..Y....... .V........ | ......D.......... ..............S | SSGWY- .................. .-.... | ....... .... |
| iPS:44 1489 | 21-225_25A 4.001.004 | VH1\|1-08/D6\|6-19\|RF1/J H4 | ..L.......... R............ ...... | N......... | ........... ... | ..Y....... Q......... | ......D.......... ..............S | SSGWY- .................. .-.... | ....... .... |
| iPS:44 1496 | 21-225_25A 4.001.005 | VH1\|1-08/D6\|6-19\|RF1/J H4 | ............. R............ ...... | N......... | ........... ... | ..Y....... S......... | ......D.......... ..............S | SSGWY- .................. .-.... | ....... .... |
| iPS:44 1505 | 21-225_25A 4.001.006 | VH1\|1-08/D6\|6-19\|RF1/J H4 | ............. R............ ...... | N......... | ........... ... | ..Y....... .A........ | ......D.......... ..............S | SSGWY- .................. .-.... | ....... .... |
| iPS:44 1512 | 21-225_25A 4.001.007 | VH1\|1-08/D6\|6-19\|RF1/J H4 | ............. R............ ...... | N......... | ........... ... | ..Y....... .V........ | ......D.......... ..............S | SSGWY- .................. .-.... | ....... .... |

| | | | | | | | | | |
|---|---|---|---|---|---|---|---|---|---|
| iPS:441519 | 21-225_25A4.001.008 | VH1\|1-08/D6\|6-19\|RF1/JH4 | .............R............ ...... | N......... | .............. | ..Y.......Q......... | ......D.........................S | SSGWY-...................-.... | ........... |
| iPS:441554 | 21-225_25A4.001.013 | VH1\|1-08/D6\|6-19\|RF1/JH4 | ..L..........R........................ | N......... | .............. | ..Y.......S......... | ......D.........................S | SSGWY-...................-.... | ........... |
| iPS:441595 | 21-225_25A4.001.019 | VH1\|1-08/D6\|6-19\|RF1/JH4 | .............R........................ | N......... | .....P........ | ..Y.......S......... | ......D.........................S | SSGWY-...................-.... | ........... |
| iPS:441604 | 21-225_25A4.001.020 | VH1\|1-08/D6\|6-19\|RF1/JH4 | ..L..........R........................ | N......... | .....P........ | ..Y.......Q......... | ......D.........................S | SSGWY-...................-.... | ........... |
| iPS:441613 | 21-225_25A4.001.021 | VH1\|1-08/D6\|6-19\|RF1/JH4 | .............R........................ | N......... | .............. | ..Y........A........ | ......D.........................S | SSGWY-...................-.... | ........... |
| iPS:443006 | 21-225_25A4.001.029 | VH1\|1-08/D6\|6-19\|RF1/JH4 | ..L..........R........................ | N......... | .............. | ..Y.......Q......... | ......D.........................S | SSGWY-...................-.... | ........... |
| iPS:412232 | 21-225_4A2 | VH1\|1-08/D6\|6-19\|RF1/JH4 | .........T.......................... | N......... | .............. | ..H................. | ...L............................S | SSGWY-...................-.... | ........... |
| iPS:422894 | 21-225_4A2.001 | VH1\|1-08/D6\|6-19\|RF1/JH4 | .........T.......................... | N......... | .............. | ..H................. | ...L..D.........................S | SSGWY-...................-.... | ........... |
| iPS:441841 | 21-225_4A2.001.001 | VH1\|1-08/D6\|6-19\|RF1/JH4 | .........T.......................... | N......... | .............. | ..H................. | ...L..D.........................S | SSGWY-...................-.... | ........... |
| iPS:441847 | 21-225_4A2.001.002 | VH1\|1-08/D6\|6-19\|RF1/JH4 | .........T.......................... | N......... | .............. | ..H................. | ...L..D.........................S | SSGWY-...................-.... | ........... |
| iPS:441853 | 21-225_4A2.001.003 | VH1\|1-08/D6\|6-19\|RF1/JH4 | .........T.......................... | N......... | .............. | ..H................. | ...L..D.........................S | SSGWY-...................-.... | ........... |
| iPS:441859 | 21-225_4A2.001.004 | VH1\|1-08/D6\|6-19\|RF1/J | .........T.......................... | N......... | .............. | ..H.......A......... | ...L..D.........................S | SSGWY-...................-.... | ........... |

| | | | | | | | | | |
|---|---|---|---|---|---|---|---|---|---|
| iPS:44 1866 | 21-225_4A2.001.005 | VH1\|1-08/D6\|6-19\|RF1/JH4 | .........T...<br>.............<br>...... | N......... | ...........<br>... | ..H.......<br>S......... | ...L..D..........<br>..............S | SSGWY-<br>.................<br>.-.... | .......<br>.... |
| iPS:44 1873 | 21-225_4A2.001.006 | VH1\|1-08/D6\|6-19\|RF1/JH4 | .........T...<br>.............<br>...... | N......... | ...........<br>... | ..H.......<br>Q......... | ...L..D..........<br>..............S | SSGWY-<br>.................<br>.-.... | .......<br>.... |
| iPS:44 1880 | 21-225_4A2.001.007 | VH1\|1-08/D6\|6-19\|RF1/JH4 | .........T...<br>.............<br>...... | N......... | ...........<br>... | ..H.......<br>Q......... | ...L..D..........<br>..............S | SSGWY-<br>.................<br>.-.... | .......<br>.... |
| iPS:44 1884 | 21-225_4A2.001.008 | VH1\|1-08/D6\|6-19\|RF1/JH4 | .........T...<br>.............<br>...... | N......... | ...........<br>... | ..H.......<br>.A........ | ...L..D..........<br>..............S | SSGWY-<br>.................<br>.-.... | .......<br>.... |
| iPS:44 1888 | 21-225_4A2.001.009 | VH1\|1-08/D6\|6-19\|RF1/JH4 | .........T...<br>.............<br>...... | N......... | ...........<br>... | ..H.......<br>Q......... | ...L..D..........<br>..............S | SSGWY-<br>.................<br>.-.... | .......<br>.... |
| iPS:44 1892 | 21-225_4A2.001.010 | VH1\|1-08/D6\|6-19\|RF1/JH4 | .........T...<br>.............<br>...... | N......... | ...........<br>... | ..H.......<br>.A........ | ...L..D..........<br>..............S | SSGWY-<br>.................<br>.-.... | .......<br>.... |
| iPS:44 1896 | 21-225_4A2.001.011 | VH1\|1-08/D6\|6-19\|RF1/JH4 | .........T...<br>.............<br>...... | N......... | ...........<br>... | ..H.......<br>Q......... | ...L..D..........<br>..............S | SSGWY-<br>.................<br>.-.... | .......<br>.... |
| iPS:44 1900 | 21-225_4A2.001.012 | VH1\|1-08/D6\|6-19\|RF1/JH4 | .........T...<br>.............<br>...... | N......... | ...........<br>... | ..H.......<br>S......... | ...L..D..........<br>..............S | SSGWY-<br>.................<br>.-.... | .......<br>.... |
| iPS:44 1955 | 21-225_4A2.001.022 | VH1\|1-08/D6\|6-19\|RF1/JH4 | .............<br>.............<br>...... | N......... | ...........<br>... | ..H.......<br>.......... | ...L..D..........<br>..............S | SSGWY-<br>.................<br>.-.... | .......<br>.... |
| iPS:44 1962 | 21-225_4A2.001.023 | VH1\|1-08/D6\|6-19\|RF1/JH4 | .............<br>.............<br>...... | N......... | .....P.....<br>... | ..H.......<br>.......... | ...L..D..........<br>..............S | SSGWY-<br>.................<br>.-.... | .......<br>.... |
| iPS:44 1971 | 21-225_4A2.001.024 | VH1\|1-08/D6\|6-19\|RF1/JH4 | .............<br>.............<br>...... | N......... | ...........<br>... | ..H.......<br>Q......... | ...L..D..........<br>..............S | SSGWY-<br>.................<br>.-.... | .......<br>.... |
| iPS:44 1999 | 21-225_4A2.001.028 | VH1\|1-08/D6\|6-19\|RF1/J | .............<br>.............<br>...... | N......... | .....P.....<br>... | ..H.......<br>Q......... | ...L..D..........<br>..............S | SSGWY-<br>.................<br>.-.... | .......<br>.... |

| | | | | | | | | | |
|---|---|---|---|---|---|---|---|---|---|
| iPS:44 2006 | 21-225_4A2.001.029 | VH1\|1-08/D6\|6-19\|RF1/JH4 | .............<br>.............<br>...... | N......... | .....P.....<br>... | ..H.......<br>S......... | ...L..D..........<br>..............S | SSGWY-<br>..................<br>.-.... | .......<br>.... |
| iPS:44 2020 | 21-225_4A2.001.031 | VH1\|1-08/D6\|6-19\|RF1/JH4 | .........G...<br>.............<br>...... | N......... | .....E.....<br>... | ..H.......<br>.E........ | ...L..D..........<br>..............S | SSGWY-<br>..................<br>.-.... | .......<br>.... |
| | | Germline | H_FR1 | H_CDR1 | H_FR2 | H_CDR2 | H_FR3 | H_CDR3 | H_FR4 |
| | VH1\|1-02/D1\|1-1\|RF1/JH4 | | QVQLVQS-<br>GAEVKKPGASVKV<br>SCKASG-YTFT | G-----YYMH | WVRQAPGQGLE<br>WMG | WINPN---<br>SGGTNYAQKF<br>QG | RVTMTRDTSISTAYMEL<br>SRLRSDDTAVYYCAR | GTTGT------------<br>--------YFDY | WGQGTLV<br>TVSS |
| iPS:39 3954 | 21-225_4H6 | VH1\|1-02/D1\|1-1\|RF1/JH4 | .............<br>.............<br>...... | D.......L. | ...........<br>... | ..H.......<br>.......... | ...............G.<br>.S............ | DG.S-<br>..................<br>...S... | .......<br>.... |
| iPS:44 2050 | 21-225_4H6.004 | VH1\|1-02/D1\|1-1\|RF1/JH4 | .............<br>.............<br>...... | D.......L. | ...........<br>... | ..H.......<br>.......... | .................<br>.S............ | DG.S-<br>..................<br>...S... | .......<br>.... |
| iPS:44 2059 | 21-225_4H6.005 | VH1\|1-02/D1\|1-1\|RF1/JH4 | .............<br>.............<br>...... | D.......L. | ...........<br>... | ..H.......<br>.......... | .................<br>.S............ | DG.S-<br>..................<br>...S... | .......<br>.... |
| iPS:44 2065 | 21-225_4H6.006 | VH1\|1-02/D1\|1-1\|RF1/JH4 | .............<br>.............<br>...... | D.......L. | ...........<br>... | ..H.......<br>.......... | .................<br>.S............ | DG.S-<br>..................<br>...S... | .......<br>.... |
| iPS:44 2071 | 21-225_4H6.007 | VH1\|1-02/D1\|1-1\|RF1/JH4 | .............<br>.............<br>...... | D.......L. | ...........<br>... | ..H.......<br>.......... | .................<br>.S............ | DA.S-<br>..................<br>...S... | .......<br>.... |
| iPS:44 2078 | 21-225_4H6.008 | VH1\|1-02/D1\|1-1\|RF1/JH4 | .............<br>.............<br>...... | D.......L. | ...........<br>... | ..H.......<br>.......... | .................<br>.S............ | SG.S-<br>..................<br>...S... | .......<br>.... |
| iPS:44 2085 | 21-225_4H6.009 | VH1\|1-02/D1\|1-1\|RF1/JH4 | .............<br>.............<br>...... | D.......L. | ...........<br>... | ..H.......<br>.......... | .................<br>.S............ | SG.S-<br>..................<br>...S... | .......<br>.... |
| iPS:44 2089 | 21-225_4H6.010 | VH1\|1-02/D1\|1-1\|RF1/JH4 | .............<br>.............<br>...... | D.......L. | ...........<br>... | ..H.......<br>.......... | .................<br>.S............ | DA.S-<br>..................<br>...S... | .......<br>.... |

| | | | | | | | | | |
|---|---|---|---|---|---|---|---|---|---|
| iPS:44 2093 | 21-225_4H6. 011 | VH1\|1-02/D1\|1-1\|RF1/JH 4 | .............<br>.............<br>...... | D.......L. | ...........<br>... | ..H.......<br>.......... | .................<br>.S............. | DA.S-<br>..................<br>...S... | .......<br>.... |
| iPS:44 3016 | 21-225_4H6. 014 | VH1\|1-02/D1\|1-1\|RF1/JH 4 | .............<br>.............<br>...... | D.......L. | ...........<br>... | ..H.......<br>.......... | ...............G.<br>.S............. | DA.S-<br>..................<br>...S... | .......<br>.... |
| | | Germline | H_FR1 | H_CDR1 | H_FR2 | H_CDR2 | H_FR3 | H_CDR3 | H_FR4 |
| | VH3\|3-33/D6\|6-6\|RF1/JH 6 | | QVQLVES-<br>GGGVVQPGRSLRL<br>SCAASG-FTFS | S-----YGMH | WVRQAPGKGLE<br>WVA | VIWYD---<br>GSNKYYADSV<br>KG | RFTISRDNSKNTLYLQM<br>NSLRAEDTAVYYCAR | EYSSSSYY----------<br>-----YYYGMDV | WGQGTTV<br>TVSS |
| iPS:39 4041 | 21-225_5E5 | VH3\|3-33/D6\|6-6\|RF1/JH 6 | .............<br>.............<br>...... | N......V.. | ...........<br>... | ..........<br>.......... | .................<br>.............T. | .VY..GW-<br>...............D.<br>.... | .......<br>.... |
| iPS:44 2115 | 21-225_5E5. 003 | VH3\|3-33/D6\|6-6\|RF1/JH 6 | .............<br>.............<br>...... | N......V.. | ...........<br>... | ........A.<br>.......... | .................<br>.............T. | .VY..GW-<br>...............D.<br>.... | .......<br>.... |
| iPS:44 2122 | 21-225_5E5. 004 | VH3\|3-33/D6\|6-6\|RF1/JH 6 | .............<br>.............<br>...... | N......V.. | ...........<br>... | ..........<br>.....E.... | .................<br>.............T. | .VY..GW-<br>...............D.<br>.... | .......<br>.... |
| iPS:44 2129 | 21-225_5E5. 005 | VH3\|3-33/D6\|6-6\|RF1/JH 6 | .............<br>.............<br>...... | N......V.. | ...........<br>... | ..........<br>.....G.... | .................<br>.............T. | .VY..GW-<br>...............D.<br>.... | .......<br>.... |
| iPS:44 2136 | 21-225_5E5. 006 | VH3\|3-33/D6\|6-6\|RF1/JH 6 | .............<br>.............<br>...... | N......V.. | ...........<br>... | ..........<br>......A... | .................<br>.............T. | .VY..GW-<br>...............D.<br>.... | .......<br>.... |
| iPS:44 2171 | 21-225_5E5. 011 | VH3\|3-33/D6\|6-6\|RF1/JH 6 | .............<br>.............<br>...... | N......V.. | ...........<br>... | ........A.<br>.....E.... | .................<br>.............T. | .VY..GW-<br>...............D.<br>.... | .......<br>.... |
| iPS:44 2178 | 21-225_5E5. 012 | VH3\|3-33/D6\|6-6\|RF1/JH 6 | .............<br>.............<br>...... | N......V.. | ...........<br>... | ........A.<br>......A... | .................<br>.............T. | .VY..GW-<br>...............D.<br>.... | .......<br>.... |
| iPS:44 2199 | 21-225_5E5. 015 | VH3\|3-33/D6\|6-6\|RF1/JH 6 | .............<br>.............<br>...... | N......V.. | ...........<br>... | ........A.<br>.......... | .................<br>.............T. | .VY..G.-<br>...............D.<br>.... | .......<br>.... |

| | | | | | | | | | |
|---|---|---|---|---|---|---|---|---|---|
| iPS:44<br>2206 | 21-<br>225_5E5.<br>016 | VH3\|3-<br>33/D6\|6-<br>6\|RF1/JH<br>6 | .............<br>.............<br>...... | N......V.. | ...........<br>... | ........A.<br>.......... | .................<br>.............T. | .VY..GF-<br>...............D.<br>.... | .......<br>.... |
| iPS:44<br>2213 | 21-<br>225_5E5.<br>017 | VH3\|3-<br>33/D6\|6-<br>6\|RF1/JH<br>6 | .............<br>.............<br>...... | N......V.. | ...........<br>... | ..........<br>.....E.... | .................<br>.............T. | .VY..G.-<br>...............D.<br>.... | .......<br>.... |
| iPS:44<br>2220 | 21-<br>225_5E5.<br>018 | VH3\|3-<br>33/D6\|6-<br>6\|RF1/JH<br>6 | .............<br>.............<br>...... | N......V.. | ...........<br>... | ..........<br>.......... | .................<br>.............T. | .VY..G.-<br>...............D.<br>.... | .......<br>.... |
| iPS:44<br>2227 | 21-<br>225_5E5.<br>019 | VH3\|3-<br>33/D6\|6-<br>6\|RF1/JH<br>6 | .............<br>.............<br>...... | N......V.. | ...........<br>... | ..........<br>.......... | .................<br>.............T. | .VY..GF-<br>...............D.<br>.... | .......<br>.... |
| iPS:44<br>2255 | 21-<br>225_5E5.<br>023 | VH3\|3-<br>33/D6\|6-<br>6\|RF1/JH<br>6 | .............<br>.............<br>...... | N......V.. | ...........<br>... | ........A.<br>.....E.... | .................<br>.............T. | .VY..G.-<br>...............D.<br>.... | .......<br>.... |
| iPS:44<br>2262 | 21-<br>225_5E5.<br>024 | VH3\|3-<br>33/D6\|6-<br>6\|RF1/JH<br>6 | .............<br>.............<br>...... | N......V.. | ...........<br>... | ........A.<br>......A... | .................<br>.............T. | .VY..G.-<br>...............D.<br>.... | .......<br>.... |
| | | Germline | H_FR1 | H_CDR1 | H_FR2 | H_CDR2 | H_FR3 | H_CDR3 | H_FR4 |
| | VH3\|3-<br>07/D6\|6-<br>6\|RF1/JH<br>6 | | EVQLVES-<br>GGGLVQPGGSLRL<br>SCAASG-FTFS | S-----YWMS | WVRQAPGKGLE<br>WVA | NIKQD---<br>GSEKYYVDSV<br>KG | RFTISRDNAKNSLYLQM<br>NSLRAEDTAVYYCAR | EYSSSSYY----------<br>-----YYYGMDV | WGQGTTV<br>TVSS |
| iPS:44<br>2269 | 21-<br>225_5E5.<br>025 | VH3\|3-<br>07/D6\|6-<br>6\|RF1/JH<br>6 | ...........V.<br>.............<br>...... | N......V.H | ...........<br>... | V.WY......<br>N...AE.... | ...........T.....<br>.............T. | .VY..GW-<br>...............D.<br>.... | .......<br>.... |
| | | Germline | H_FR1 | H_CDR1 | H_FR2 | H_CDR2 | H_FR3 | H_CDR3 | H_FR4 |
| | VH3\|3-<br>33/D4\|4-<br>11\|RF2/J<br>H6 | | QVQLVES-<br>GGGVVQPGRSLRL<br>SCAASG-FTFS | S-----YGMH | WVRQAPGKGLE<br>WVA | VIWYD---<br>GSNKYYADSV<br>KG | RFTISRDNSKNTLYLQM<br>NSLRAEDTAVYYCAR | DYSNYYY----------<br>-----YYYGMDV | WGQGTTV<br>TVSS |
| iPS:39<br>3930 | 21-<br>225_7E1<br>1 | VH3\|3-<br>33/D4\|4-<br>11\|RF2/J<br>H6 | .............<br>.............<br>...... | ......F... | ...........<br>... | I..H......<br>.......... | ..........N......<br>S.............. | .L.MG--<br>................--<br>-.... | .......<br>.... |
| iPS:42<br>4460 | 21-<br>225_7E1<br>1.001 | VH3\|3-<br>33/D4\|4-<br>11\|RF2/J<br>H6 | .............<br>.............<br>...... | ......F... | ...........<br>... | I..H......<br>.......... | .................<br>S.............. | .L.MG--<br>................--<br>-.... | .......<br>.... |

| | | | | | | | | | |
|---|---|---|---|---|---|---|---|---|---|
| iPS:44 2311 | 21-225_7E1 1.001.001 | VH3\|3-33/D4\|4-11\|RF2/J H6 | ................................ | ......F... | .............. | I..H................ | .................S............. | .L.MG--...............---.... | ........... |
| iPS:44 2317 | 21-225_7E1 1.001.002 | VH3\|3-33/D4\|4-11\|RF2/J H6 | ................................ | ......F... | .............. | I..H................ | .................S............. | .L.MG--...............---.... | ........... |
| iPS:44 2323 | 21-225_7E1 1.001.003 | VH3\|3-33/D4\|4-11\|RF2/J H6 | ................................ | ......F... | .............. | I..HS............... | .................S............. | .L.MG--...............---.... | ........... |
| iPS:44 2330 | 21-225_7E1 1.001.004 | VH3\|3-33/D4\|4-11\|RF2/J H6 | ................................ | ......F... | .............. | I..HE............... | .................S............. | .L.MG--...............---.... | ........... |
| iPS:44 2337 | 21-225_7E1 1.001.005 | VH3\|3-33/D4\|4-11\|RF2/J H6 | ................................ | ......F... | .............. | I..H....A........... | .................S............. | .L.MG--...............---.... | ........... |
| iPS:44 2344 | 21-225_7E1 1.001.006 | VH3\|3-33/D4\|4-11\|RF2/J H6 | ................................ | ......F... | .............. | I..H..........E.... | .................S............. | .L.MG--...............---.... | ........... |
| iPS:44 2351 | 21-225_7E1 1.001.007 | VH3\|3-33/D4\|4-11\|RF2/J H6 | ................................ | ......F... | .............. | I..H...........A... | .................S............. | .L.MG--...............---.... | ........... |
| iPS:44 2358 | 21-225_7E1 1.001.008 | VH3\|3-33/D4\|4-11\|RF2/J H6 | ................................ | ......F... | .............. | I..HE...........A... | .................S............. | .L.MG--...............---.... | ........... |
| iPS:44 2365 | 21-225_7E1 1.001.009 | VH3\|3-33/D4\|4-11\|RF2/J H6 | ................................ | ......F... | .............. | I..HE..........E.... | .................S............. | .L.MG--...............---.... | ........... |
| iPS:44 2372 | 21-225_7E1 1.001.010 | VH3\|3-33/D4\|4-11\|RF2/J H6 | ................................ | ......F... | .............. | I..HS..........E.... | .................S............. | .L.MG--...............---.... | ........... |
| iPS:44 2379 | 21-225_7E1 1.001.011 | VH3\|3-33/D4\|4-11\|RF2/J H6 | ................................ | ......F... | .............. | I..HS...........A... | .................S............. | .L.MG--...............---.... | ........... |
| iPS:44 2386 | 21-225_7E1 1.001.012 | VH3\|3-33/D4\|4-11\|RF2/J | ................................ | ......F... | .............. | I..HS............... | .................S............. | .L.MG--...............---.... | ........... |

| | | | | | | | | | |
|---|---|---|---|---|---|---|---|---|---|
| iPS:44 2390 | 21-225_7E1 1.001.013 | VH3\|3-33/D4\|4-11\|RF2/JH6 | ................................ | ......F... | .............. | I..H...........E.... | .................S.............. | .L.MG--................--—.... | ........... |
| iPS:44 2394 | 21-225_7E1 1.001.014 | VH3\|3-33/D4\|4-11\|RF2/JH6 | ................................ | ......F... | .............. | I..HS..........E.... | .................S.............. | .L.MG--................--—.... | ........... |
| iPS:44 2398 | 21-225_7E1 1.001.015 | VH3\|3-33/D4\|4-11\|RF2/JH6 | ................................ | ......F... | .............. | I..HS............... | .................S.............. | .L.MG--................--—.... | ........... |
| iPS:44 2402 | 21-225_7E1 1.001.016 | VH3\|3-33/D4\|4-11\|RF2/JH6 | ................................ | ......F... | .............. | I..HS..........E.... | .................S.............. | .L.MG--................--—.... | ........... |
| iPS:44 2406 | 21-225_7E1 1.001.017 | VH3\|3-33/D4\|4-11\|RF2/JH6 | ................................ | ......F... | .............. | I..HE...........A... | .................S.............. | .L.MG--................--—.... | ........... |
| iPS:44 2410 | 21-225_7E1 1.001.018 | VH3\|3-33/D4\|4-11\|RF2/JH6 | ................................ | ......F... | .............. | I..H....A......E.... | .................S.............. | .L.MG--................--—.... | ........... |
| iPS:44 3027 | 21-225_7E1 1.001.023 | VH3\|3-33/D4\|4-11\|RF2/JH6 | ................................ | ......F... | .............. | I..H....A.......A... | .................S.............. | .L.MG--................--—.... | ........... |
| | | Germline | H_FR1 | H_CDR1 | H_FR2 | H_CDR2 | H_FR3 | H_CDR3 | H_FR4 |
| | VH3\|3-07/D4\|4-11\|RF2/JH6 | | EVQLVES-GGGLVQPGGSLRLSCAASG-FTFS | S-----YWMS | WVRQAPGKGLEWVA | NIKQD---GSEKYYVDSVKG | RFTISRDNAKNSLYLQMNSLRAEDTAVYYCAR | DYSNYYY-----------------YYYGMDV | WGQGTTVTVSS |
| iPS:44 2417 | 21-225_7E1 1.001.019 | VH3\|3-07/D4\|4-11\|RF2/JH6 | ...........V.................... | ......FG.H | .............. | I.WH......N...AE.... | ...........T.....S.............. | .L.MG--................--—.... | ........... |
| iPS:44 2431 | 21-225_7E1 1.001.021 | VH3\|3-07/D4\|4-11\|RF2/JH6 | ...........V.................... | ......FG.H | .............. | I.WHS.....N...AE.... | ...........T.....S.............. | .L.MG--................--—.... | ........... |
| iPS:44 2438 | 21-225_7E1 1.001.022 | VH3\|3-07/D4\|4-11\|RF2/JH6 | ...........V.................... | ......FG.H | .............. | I.WHE.....N...AE.... | ...........T.....S.............. | .L.MG--................--—.... | ........... |

**FIGURE 55**

Table 19A

**VARIALBLE HEAVY CHAIN CONSENSUS SEQUENCES**

| NAME (ORIGINAL AND PATENT) | Patent SEQ ID NO.: | SEQUENCE |
|---|---|---|
| VH-Consensus 1 (Table 21) (generated from 13 heavy chain sequences) | SEQ ID NO: 50352 | QVQLVQSGAEVKKPGASVKVSCKASGYTFTDYYMHWVRQAPGQGLEWMGWINPNNGGTNYAQKFQGRVTMTRDTSISTAYMELSRLRSDDTAVYYCARDGTGSFDYWGQGTLVTVSS |
| VH-Consensus 2 (Table 22) (generated from 11 heavy chain sequences) | SEQ ID NO: 50353 | QVQLVQSGAEVKKPGASVKVSCKASGYTFTGYYMHWVRQAPGQGLEWMGWINPNSGGTNYAQKFQGRVTMTRDTSISTAYMELSRLRSDDTAVYYCARSYYYGSGSYYNEFDYWGQGTLVTVSS |
| VH-Consensus 3 (Table 23) (generated from 15 heavy chain sequences) | SEQ ID NO: 50354 | QVQLVQSGAEVKKPGASVKVSCKASGYTFTNYDINWVRQATGQGLEWMGWMHPNSGNTGYAQKFQGRVTMTRNTSISTAYMELSSLRSEDTAVYYCASSSGWYYFDYWGQGTLVTVSS |
| VH-Consensus 4 (Table 24) (generated from 23 heavy chain sequences) | SEQ ID NO: 50355 | EVQLVESGGGLVKPGGSLRLSCAASGFTFSSYSMNWVRQAPGKGLEWVSSISGSSSYIYYADSVKGRFTISRDNAKNSLYLQMNSLRAEDTAVYYCARVASFDYWGQGTLVTVSS |
| VH-Consensus 5 | SEQ ID NO: 50356 | EVQLVESGGGLVKPGGSLRLSCAASGFTFSSYSMNWVRQAPGKGLEWVSSISGSSSYIYYADSVKGRFTISRDNAKNSLYLQMNSLRAEDTAVYYCARDRGSLWGQGTLVTVSS |
| VH-Consensus 6 (Table 26) (generated from 11 heavy chain sequences) | SEQ ID NO: 50357 | EVQLLESGGGLVQPGGSLRLSCAASGFTFSSYVMNWVRQAPGKGLEWVSAISGSGGSTYYADSVKGRFTISRDNSKNTLYLQMNSLRAEDTAVYYCARTGVFDYWGQGTLVTVSS |
| VH-Consensus 7 (Table 27) (generated from 30 heavy chain sequences) | SEQ ID NO: 50358 | EVQLLESGGGLVQPGGSLRLSCAASGFTFSSYAMSWVRQAPGKGLEWVSAISGRGGSTFHADSVKGRFTISRDNSKNTLYLQMNSLRAEDTAVYYCVKGELLEDYYFYGMDVWGQGTTVTVSS |
| VH-Consensus 8 (Table 28) (generated from 25 heavy chain sequences) | SEQ ID NO: 50359 | EVQLLESGGGLVQPGGSLRLSCAASEFTFSSYAMXWVRQAPGKGLEWVSVISGRGGNTFYADSVKGRFTISRDNSKNTLYLQMNSLRAEDTAVYYCASRLAVAGSEAFDIWGQGTMVTVSS |
| VH-Consensus 9 (Table 29) (generated from 14 heavy chain sequences) | SEQ ID NO: 50360 | QVQLVESGGGVVQPGRSLRLSCAASGFTFSSYGMHWVRQAPGKGLEWVAVIWXDGSNKYYADSVKGRFTISRDNSKNTLYLQMNSLRAEDTAVYYCARDLSMGGMDVWGQGTTVTVSS |

FIGURE 55

| | | |
|---|---|---|
| VH-Consensus 10 (Table 30) (generated from 22 heavy chain sequences) | SEQ ID NO: 50361 | QVQLVESGGGVVQPGRSLRLSCAASGFTFSSYVMHWVRQAPGKGLEWVAVIWYDGSNKYYADSVKGRFTISRDNSKNTLYLQMNSLRAEDTAVYYCAREKYSSSWYDYGMDVWGQGTTVTVSS |
| | | |
| VH-Consensus 11 (Table 31) (generated from 16 heavy chain sequences) | SEQ ID NO: 50362 | QVQLVESGGGVVQPGRSLRLSCAASGFTFSDYGMHWVRQAPGKGLEWVAVIWYDESNKYYADSVKGRFTISRDNSKNTLYLQMNSLRAEDTAVYYCAREIGWLDDYWGQGTLVTVSS |
| | | |
| VH-Consensus 12 (Table 32) (generated from 71 heavy chain sequences) | SEQ ID NO: 50363 | QVQLVESGGGVVQPGRSLRLSCAASGFTFSDYGMHWVRQAPGKGLEWVAVIWYDENNKYYADSVKGRFTISRDNSKNTLYLQMNSLRAEDTAVYYCARELGFRSDYWGQGTLVTVSS |
| | | |
| VH-Consensus 13 (Table 33) (generated from 21 heavy chain sequences) | SEQ ID NO: 50364 | QLQLQESGPGLVKPSETLSLTCTVSGGSISRSSYYWGWIRQPPGKGLEWIGNIYYSGSTYYNPSLKSRVTISVDTSKNQFSLKLSSVTAADTAVYYCARHSSSWSLDYWGQGTLVTVSS |
| | | |
| VH-Consensus 14 (Table 34) (generated from 13 heavy chain sequences) | SEQ ID NO: 50365 | QLQLQESGPGLVKPSETLSLTCTVSGGSISRSSYYWGWIRQPPGKGLEWIGNIYYSGSTYYNPSLKSRVTISVDTSKNQFSLKLSSVTAADTAVYYCGRHGKDWGLDYWGQGTLVTVSS |
| | | |
| VH-Consensus 15 (Table 49) ) (generated from 149 heavy chain sequences) | SEQ ID NO: 50266 | QVQLVQS-GAEVKKPGASVKVSCKASG-YTFTN-----YDINWVRQATGQGLEWMGWMHPN---SGNTGYAQKFQGRVTMTRNTSISTAYMELSSLRSEDTAVYYCASSSGWY--------------------YFDYWGQGTLVTVSS |
| | | |
| VH-Consensus 16 (Table 50) ) (generated from 128 heavy chain sequences) | SEQ ID NO: 50267 | QVQLVES-GGGVVQPGRSLRLSCAASG-FTFSS-----YVMHWVRQAPGKGLEWVAVIWYD---GSNKYYADSVKGRFTISRDNSKNTLYLQMNSLRAEDTAVYYCARERYSSGW---------------YDYGMDVWGQGTTVTVSS |
| | | |
| VH-Consensus 17 (Table 51) ) (generated from 117 heavy chain sequences) | SEQ ID NO: 50268 | QVQLVES-GGGVVQPGRSLRLSCAASG-FTFSD-----YGMHWVRQAPGKGLEWVAVIWYD---ENNKYYADSVKGRFTISRDNSKNTLYLQMNSLRAEDTAVYYCARELGF---------------------SDYWGQGTLVTVSS |
| | | |
| VH-Consensus 18 (Table 52) ) (generated from 91 heavy chain sequences) | SEQ ID NO: 50269 | QVQLQQW-GAGLLKPSETLSLTCAVHG-GSFSG-----CYWSWIRQPPGKGLEWIGEINH----SGSTNYNPSLKSRVTISVDTSKNQFSLKLSSVTAADTAVYYCARDYGG----------------------MDVWGQGTTVTVSS |
| | | |

FIGURE 55

| | | |
|---|---|---|
| VH-Consensus 19 (Table 53) (generated from 74 heavy chain sequences) | SEQ ID NO: 50270 | QVQLVQS-GAEVKKPGASVKVSCKASG-YTFTN-----YDINWVRQATGQGLEWMGWMHPN---SGNTGYAQKFQGRVTMTRNTSISTAYMELSSLRSEDTAVYYCAISSGWY--------------------WFDPWGQGTLVTVSS |
| | | |
| VH-Consensus 20 (Table 54) (generated from 53 heavy chain sequences) | SEQ ID NO: 50271 | QVQLVES-GGGVVQPGRSLRLSCAASG-FTFSS-----YGMHWVRQAPGKGLEWVAVIWYD---GSNKNYADSVKGRFTISRDNSKNTLYLQMNSLRAEDTAVYYCARDQGVGY------------------YGLDVWGQGTTVTVSS |
| | | |
| VH-Consensus 21 (Table 55) (generated from 52 heavy chain sequences) | SEQ ID NO: 50272 | QVQLVQS-GAEVKKPGASVKVSCKASG-YTFTG-----YYMHWVRQAPGQGLEWMGWINPN---SGGTNYAQKFQGRVTMTRDTSISTAYMELSRLRSDDTAVYYCARDGTS---------------------SFDYWGQGTLVTVSS |
| | | |
| VH-Consensus 22 (Table 56) (generated from 49 heavy chain sequences) | SEQ ID NO: 50273 | QVQLVES-GGGVVQPGRSLRLSCAASG-FTFSS-----YGMHWVRQAPGKGLEWVAVIWHD---GSNKYYADSVKGRFTISRDNSKNTLYLQMNSLRAEDTAVYYCARDLSMG--------------------GMDVWGQGTTVTVSS |
| | | |
| VH-Consensus 23 (Table 57) (generated from 37 heavy chain sequences) | SEQ ID NO: 50274 | QVQLVES-GGGVVQPGRSLRLSCAASG-FTFSD-----YGMHWVRQAPGKGLEWVAVIWYD---ESNKYYADSVKGRFTISRDNSKNTLYLQMNSLRAEDTAVYYCAREVGW---------------------LDDYWGQGTLVTVSS |
| | | |
| VH-Consensus 24 (Table 58) (generated from 35 heavy chain sequences) | SEQ ID NO: 50275 | EVQLVES-GGGLVKPGGSLRLSCAASG-FTFSS-----YSMNWVRQAPGKGLEWVSSISGS---SSYIYYADSVKGRFTISRDNAKNSLYLQMNSLRAEDTAVYYCARDRG------------------------SSWGQGTLVTVSS |
| | | |
| VH-Consensus 25 (Table 59) (generated from 32 heavy chain sequences) | SEQ ID NO: 50276 | EVQLLES-GGGLVQPGGSLRLSCAASE-FTFSS-----YAMSWVRQAPGKGLEWVSVISGR---GGNTFYADSVKGRFTISRDNSKNTLYLQMNSLRAEDTAVYYCASRIAVAG-----------------SEAFDIWGQGTMVTVSS |
| | | |
| VH-Consensus 26 (Table 60) (generated from 30 heavy chain sequences) | SEQ ID NO: 50277 | EVQLLES-GGGLVQPGGSLRLSCAASG-FTFSS-----YAMSWVRQAPGKGLEWVSAISGR---GGSTFHADSVKGRFTISRDNSKNTLYLQMNSLRAEDTAVYYCVKGELLEDY---------------YFYGMDVWGQGTTVTVSS |
| | | |
| VH-Consensus 27 | SEQ ID NO: | QVQLVES-GGGVVQPGRSLRLSCAASG-FTFSS----- |

**FIGURE 55**

| | | |
|---|---|---|
| from 29 heavy chain sequences) | | GSNKYYADSVKGRFTISRDNSKNTLYLQMNSLRAED TAVYYCARDRVYCSSTSC---------- PYYYYYGMDVWGQGTTVTVSS |
| | | |
| VH-Consensus 28 (Table 62) (generated from 28 heavy chain sequences) | SEQ ID NO: 50279 | EVQLVES-GGGLVKPGGSLRLSCAASG-FTFSS----- YSMNWVRQAPGKGLEWVSSISGS--- SSYIYYADSVKGRFTISRDNAKNSLYLQMNSLRAED TAVYYCARVAS----------------------- FDYWGQGTLVTVSS |
| | | |
| VH-Consensus 29 (Table 63) (generated from 26 heavy chain sequences) | SEQ ID NO: 50280 | QVQLQES-GPGLVKPSQTLSLTCTVSG-GSISSG--- DYYWNWIRQHPGKGLEWIGYIFY---- SGSTYYNPSLKSRVTISVDTSKNQFSLKLSSVTAADT AVYYCARGDYDGSGSY------------ HYYYGMDVWGQGTTVTVSS |
| | | |
| VH-Consensus 30 (Table 64) (generated from 24 heavy chain sequences) | SEQ ID NO: 50281 | EVQLLES-GGGLVQPGGSLRLSCAASG-FTFSS----- YAMSWVRQAPGKGLEWVSVISGG--- GSSTYYADSVKGRFTISRDNSKNTLYLQMNSLRAED TAVYYCAKWRGNPT----------------- DYGMDVWGQGTTVTVSS |
| | | |
| VH-Consensus 31 (Table 65) (generated from 24 heavy chain sequences) | SEQ ID NO: 50282 | QVQLVES-GGGVVQPGRSLRLSCAASG-FTFSS----- YGMHWVRQAPGKGLEWVAIIWYD--- GSNKYYADSVKGRFTISRDNSKNTLYLQMNSLRAED TAVYYCARDHYDFW----------------- SGHFDYWGQGTLVTVSS |
| | | |
| VH-Consensus 32 (Table 66) (generated from 24 heavy chain sequences) | SEQ ID NO: 50283 | QVQLQQW-GAGLLKPSETLSLTCAVYG-GSFSG----- YYWSWIRQPPGKGLEWIGEINH---- SGRTNYNPSLKSRVTISVDTSKNQFSLKLSSVTAADT AVYYCARDYGG---------------------- LDYWGQGTLVTVSS |
| | | |
| VH-Consensus 33 (Table 67) (generated from 22 heavy chain sequences) | SEQ ID NO: 50284 | QLQLQES-GPGLVKPSETLSLTCTVSG-GSISRS--- SYYWGWIRQPPGKGLEWIGNIYY---- SGSTYYNPSLKSRVTISVDTSKNQFSLKLSSVTAADT AVYYCARHSSSW-------------------- SLDYWGQGTLVTVSS |
| | | |
| VH-Consensus 34 (Table 68) (generated from 19 heavy chain sequences) | SEQ ID NO: 50285 | QVQLVQS-GAEVKKPGASVKVSCKASG-YTFTG----- YYIHWVRQAPGQGLEWMGWINPN--- SGGTNYAQKFQGRVTMTRDTSISTAYMELSRLRSDD TAVYYCARGYSYGY------------------ NWFDPWGQGTLVTVSS |
| | | |
| VH-Consensus 35 (Table 69) (generated from 18 heavy chain | SEQ ID NO: 50286 | QVQLVES-GGGVVQPGRSLRLSCAASG-FTFSH----- YGMHWVRQAPGKGLEWVAVIWYD--- GSNKYYADSVKGRFTISRDNSKNTLYLQMNSLRAED |

FIGURE 55

| | | |
|---|---|---|
| | | TAVYYCARGDWNP--------------------<br>EGMDVWGQGTTVTVSS |
| | | |
| VH-Consensus 36 (Table 70) (generated from 17 heavy chain sequences) | SEQ ID NO: 50287 | EVQLLES-GGGLVQPGGSLRLSCAASG-FTFSS-----<br>YAMSWVRQAPGKGLEWVSAISGS---<br>GGNTFYADSVKGRFTISRDNSKNTLYLQMNSLRAED<br>TAVYYCAKKDYDYVW----------------<br>GSPYFDYWGQGTLVTVSS |
| | | |
| VH-Consensus 37 (Table 71) (generated from 16 heavy chain sequences) | SEQ ID NO: 50288 | QVQLVQS-GAEVKKPGASVKVSCKASG-YTFTG-----<br>YYMHWVRQAPGQGLEWMGWINPN---<br>SGGTNYAQKFQGRVTMTRDTSISTAYMELSRLRSDD<br>TAVYYCARSYYYGSGS---------------<br>YYNEFDYWGQGTLVTVSS |
| | | |
| VH-Consensus 38 (Table 72) (generated from 14 heavy chain sequences) | SEQ ID NO: 50289 | QVQLVQS-GAEVKKPGASVKVSCKASG-YTFTG-----<br>YYIHWVRQAPGQGLEWMGWINPY---<br>SGDTNYAQKFQGRVTMTRDTSISTAYMELSRLRFDD<br>TAVFYCARDWGGYSS----------------<br>YYYGMDVWGQGTTVTVSS |
| | | |
| VH-Consensus 39 (Table 73) (generated from 14 heavy chain sequences) | SEQ ID NO: 50290 | EVQLVES-GGGLVKPGGSLRLSCAASG-FTFSS-----<br>YSMNWVRQAPGKGLEWVSSISGS---<br>SSYIYYADSVKGRFTISRDNAKNSLYLQMNSLRAED<br>TAVYYCARLT-------------------------<br>FDYWGQGTLVTVSS |
| | | |
| VH-Consensus 40 (Table 74) (generated from 14 heavy chain sequences) | SEQ ID NO: 50291 | QVQLVES-GGGVVQPGRSLRLSCAASG-FTFST-----<br>YGMHWVRQAPGKGLEWVAIIWYD---<br>GTNKYYADSVKGRFTISRDNSKNTLYLQLNSLRAED<br>TAVYYCARDPLRGYN----------------<br>DPVMDYWGQGTLVTVSS |
| | | |
| VH-Consensus 41 (Table 75) (generated from 13 heavy chain sequences) | SEQ ID NO: 50292 | EVQLLES-GGGLVQPGGSLRLSCAASG-FTFSS-----<br>YAMSWVRQAPGKGLEWVSAISGR---<br>GGNTFYADSVKGRFTISRDNSKNTLYLQMNSLRAED<br>TAVYYCAKRVTDYGG----------------<br>NDWFDPWGQGTLVTVSS |
| | | |
| VH-Consensus 42 (Table 76) (generated from 13 heavy chain sequences) | SEQ ID NO: 50293 | QVQLVES-GGGVVQPGRSLRLSCAASG-FTFST-----<br>YGMHWVRQAPGKGLEWVAVIWYG---<br>GSNKDYADSVKGRFTISRDISKNTLYLQMNSLRAED<br>TAVYYCARDRDYCSGGSC----------<br>PYYYYYGMDVWGQGTTVTVSS |
| | | |
| VH-Consensus 43 (Table 77) (generated from 13 heavy chain sequences) | SEQ ID NO: 50294 | QLQLQES-GPGLVKPSETLSLTCTVSG-GSISRS---<br>SYYWGWIRQPPGKGLEWIGNIYY----<br>SGSTYYNPSLKSRVTISVDTSKNQFSLKLSSVTAADT<br>AVYYCGRHGKDW--------------------- |

FIGURE 55

| | | |
|---|---|---|
| VH-Consensus 44 (Table 78) (generated from 12 heavy chain sequences) | SEQ ID NO: 50295 | QVQLVQS-GAEVKKPGASVKVSCKASG-YTFTG-----<br>YYMHWVRQAPGQGLEWMGWIKPN---<br>SGGTNQAQKFQGRVTMTRDTSISTAYMELSGLRSDD<br>TAVYYCARAPGTAAAG--------------<br>TWGYFDYWGQGTLVTVSS |
| VH-Consensus 45 (Table 79) (generated from 12 heavy chain sequences) | SEQ ID NO: 50296 | QITLKES-GPTLVKPTQTLTLTCTFSG-FSLSTG---<br>GVGVGWIRQPPGKALEWLALIYW----<br>DDDKRYSPSLKSRLTITKDTSKNQVVLTMTNMDPVD<br>TATYYCAHLIAV---------------------<br>AFDYWGQGTLVTVSS |
| VH-Consensus 46 (Table 80) (generated from 11 heavy chain sequences) | SEQ ID NO: 50297 | QVQLVES-GGGVVQPGRSLRLSCAASG-FTFSS-----<br>YGMHWVRQAPGKGLEWVAIIWYD---<br>GSYKYYADSVKGRFTISRDNSKNTLYLQMNSLRAED<br>TAVYYCAREAYDFW-----------------<br>SGYFDYWGQGTLVTVSS |
| VH-Consensus 47 (Table 81) (generated from 11 heavy chain sequences) | SEQ ID NO: 50298 | QVQLVES-GGGVVQPGRSLRLSCAASG-FTFSS-----<br>YGMHWVRQAPGKGLEWVAVIWYD---<br>GSNKYYADSVKGRFTISRDNSKNTLYLQMNSLRAED<br>TAVYYCARDRDYGD------------------<br>YGMDVWGQGTTVTVSS |
| VH-Consensus 48 (Table 82) (generated from 10 heavy chain sequences) | SEQ ID NO: 50299 | EVQLLES-GGGLVQPGGSLRLSCAASG-FTFSS-----<br>YAMSWVRQAPGKGLEWVSAISGS---<br>GGNTFYADSVKGRFTISRDNSKNTLYLQMNSLRAED<br>TAVYYCAKLGKDYY-----------------<br>YYGMDVWGQGTTVTVSS |
| VH-Consensus 49 (Table 83) (generated from 10 heavy chain sequences) | SEQ ID NO: 50300 | EVQLLES-GGGLVQPGGSLRLSCAASG-FTFSS-----<br>YVMSWVRQAPGKGLEWVSAMSGS---<br>GGRTYYADSVKGRFTISRDNSKNTLYLQMNSLRAED<br>TAVYYCAKLTA-----------------------<br>FDYWGQGTLVTVSS |
| VH-Consensus 50 (Table 84) (generated from 10 heavy chain sequences) | SEQ ID NO: 50301 | QVQLVES-GGGVVQPGRSLRLSCAASG-FTFSS-----<br>YGMHWVRQAPGKGLEWVAIISYA---<br>GSNKYYADSVKGRFTISRDNSKNTLYLQMNSLRAED<br>TAVYYCVRRGYSYG-----------------<br>GYGMDVWGQGTTVTVSS |
| VH-Consensus 51 (Table 85) (generated from 10 heavy chain sequences) | SEQ ID NO: 50302 | QVQLVES-GGGVVQPGRSLRLSCAASG-FTFSD-----<br>YVMHWVRQAPGKGLEWVAVIWYD---<br>GSNKYYADSVKGRFTISRDNSKNTLYLQMNSLRAED<br>TAVYYCAREPYTSGW---------------<br>YDYGMDVWGQGTTVTVSS |

**FIGURE 55**

| | | |
|---|---|---|
| VH-Consensus 52 (Table 86) (generated from 9 heavy chain sequences) | SEQ ID NO: 50303 | QVQLVQS-GAEVKKPGASVKVSCKASG-YTFNS-----YGISWVRQAPGQGLEWMGWISAY---NGNTKYAQKLQGRVTMTTDTSTSTAYMELRSLRSDDTAVYYCARHDFWSGY----------------YKGMDVWGQGTTVTVSS |
| VH-Consensus 53 (Table 87) (generated from 9 heavy chain sequences) | SEQ ID NO: 50304 | EVQLLES-GGGLVQPGGSLRLSCAASG-FTFSS-----YAMSWVRQAPGKGLEWVSAISGR---GGNTFDADSVKGRFTISRDNSKNTLYLQMNSLRAEDTAVYYCAKERSGS--------------------YFDYWGQGTLVTVSS |
| VH-Consensus 54 (Table 88) (generated from 9 heavy chain sequences) | SEQ ID NO: 50305 | QVQLVES-GGGVVQPGRSLRLSCAASG-FTFSN-----YGMHWVRQAPGKGLEWVAVIWHD---GSNKYYADSVKGRFTISRDNSKNTLYLQMNSLRAEDTAVYYCARENSSS-------------------YYFDYWGQGTLVTVSS |
| VH-Consensus 55 (Table 89) (generated from 8 heavy chain sequences) | SEQ ID NO: 50306 | QITLKES-GPTLVKPTQTLTLTCTFSG-FSLSTS---GVGVGWIRQPPGKALEWLALINW----NDDKRYSPSLKSRFTITRDTSKDQVVLTMTNMDPVDTATYYCAHKATWV--------------------AFDIWGQGTMVTVSS |
| VH-Consensus 56 (Table 90) (generated from 8 heavy chain sequences) | SEQ ID NO: 50307 | EVQLLES-GGGLVQPGGSLRLSCAASG-FTFSS-----YVMNWVRQAPGKGLEWVSAISGS---GGRTYYADSVKGRFTISRDNSKNTLYLQMNSLRAEDTAVYYCARTAT----------------------FDYWGQGTLVTVSS |
| VH-Consensus 57 (Table 91) (generated from 8 heavy chain sequences) | SEQ ID NO: 50308 | EVQLLES-GGGLVQPGGSLRLSCAASG-FTFSS-----YAMSWVRQAPGKGLEWVSVISGR---GGTTFYADSVKGRFTISRDNSKNTLYLQMNSLRAEDTAVYYCAKRTPSD--------------------VFDIWGQGTMVTVSS |
| VH-Consensus 58 (Table 92) (generated from 8 heavy chain sequences) | SEQ ID NO: 50309 | QVQLVES-GGGVVQPGRSLRLSCAASG-FTFSS-----YGMHWVRQAPGKGLEWVAVIWYD---GSNKYYADSVKGRFTISRDNSKNTLYLQMNSLRAEDTAVYYCARDRPRS------------------SAFDYWGQGTLVTVSS |
| VH-Consensus 59 (Table 93) (generated from 8 heavy chain sequences) | SEQ ID NO: 50310 | EVQLVES-GGGLVQPGGSLRLSCAASG-FTFSS-----YNMNWVRQAPGKGLEWVSYISRS---SNTKYYADSVKGRFTISRDNAKNSLYLQMNSLRDEDTAVYYCARDRSGSYGY-------------FYYYGLDVWGQGTTVTVSS |
| VH-Consensus 60 | SEQ ID NO: | QVQLQES-GPGLVKPSQTLSLTCTVSG-GSIRSG--- |

**FIGURE 55**

| | | |
|---|---|---|
| from 8 heavy chain sequences) | | SGSTYYNPSLKSRVTISVDTSKNQFSLKLSSVTAADT AVYYCARDSSSY-------------------- GMDVWGQGTTVTVSS |

**FIGURE 55**

Table 19B

**VARIABLE HEAVY CDR CONSENSUS SEQUENCES I**

| NAME (ORIGINAL AND PATENT) | Patent SEQ ID NO: | CDR | Sequence |
|---|---|---|---|
| VH-CONSENSUS-1 TABLE 21 | 50381 | VH1 | DYYMH |
| | 50382 | VH2 | WINPNNGGTNYAQKFQG |
| | 50383 | VH3 | DGTGSFDY |
| | | | |
| VH-CONSENSUS-2 TABLE 22 | 50384 | VH1 | GYYMH |
| | 50385 | VH2 | WINPNSGGTNYAQKFQG |
| | 50386 | VH3 | SYYYGSGSYYNEFDY |
| | | | |
| VH-CONSENSUS-3 TABLE 23 | 50387 | VH1 | NYDIN |
| | 50388 | VH2 | WMHPNSGNTGYAQKFQG |
| | 50389 | VH3 | SSGWYYFDY |
| | | | |
| VH-CONSENSUS-4 TABLE 24 | 50390 | VH1 | SYSMN |
| | 50391 | VH2 | SISGSSSYIYYADSVKG |
| | 50392 | VH3 | VASFDY |
| | | | |
| VH-CONSENSUS-5 TABLE 25 | 50393 | VH1 | SYSMN |
| | 50394 | VH2 | SISGSSSYIYYADSVKG |
| | 50395 | VH3 | DRGSL |
| | | | |
| VH-CONSENSUS-6 TABLE 26 | 50396 | VH1 | SYVMN |
| | 50397 | VH2 | AISGSGGSTYYADSVKG |
| | 50398 | VH3 | TGVFDY |
| | | | |
| VH-CONSENSUS-7 TABLE 27 | 50399 | VH1 | SYAMS |
| | 50400 | VH2 | AISGRGGSTFHADSVKG |
| | 50401 | VH3 | GELLEDYYFYGMDV |
| | | | |
| VH-CONSENSUS-8 TABLE 28 | 50402 | VH1 | SYAMX |
| | 50403 | VH2 | VISGRGGNTFYADSVKG |
| | 50404 | VH3 | RLAVAGSEAFDI |
| | | | |
| VH-CONSENSUS-9 TABLE 29 | 50405 | VH1 | SYGMH |
| | 50406 | VH2 | VIWXDGSNKYYADSVKG |
| | 50407 | VH3 | DLSMGGMDV |
| | | | |
| VH-CONSENSUS-10 TABLE 30 | 50408 | VH1 | SYVMH |
| | 50409 | VH2 | VIWYDGSNKYYADSVKG |
| | 50410 | VH3 | EKYSSSWYDYGMDV |
| | | | |
| VH-CONSENSUS-11 TABLE 31 | 50411 | VH1 | DYGMH |
| | 50412 | VH2 | VIWYDESNKYYADSVKG |

FIGURE 55

| | | | |
|---|---|---|---|
| | | | |
| VH-CONSENSUS-12 | 50414 | VH1 | DYGMH |
| TABLE 32 | 50415 | VH2 | VIWYDENNKYYADSVKG |
| | 50416 | VH3 | ELGFRSDY |
| | | | |
| VH-CONSENSUS-13 | 50417 | VH1 | RSSYYWG |
| TABLE 33 | 50418 | VH2 | NIYYSGSTYYNPSLKS |
| | 50419 | VH3 | HSSSWSLDY |
| | | | |
| VH-CONSENSUS-14 | 50420 | VH1 | RSSYYWG |
| TABLE 34 | 50421 | VH2 | NIYYSGSTYYNPSLKS |
| | 50422 | VH3 | HGKDWGLDY |
| | | | |
| VH-CONSENSUS-15 | 50468 | VH1 | NYDIN |
| TABLE 49 | 50469 | VH2 | WMHPNSGNTGYAQKFQG |
| | 50470 | VH3 | SSGWYYFDY |
| | | | |
| VH-CONSENSUS-16 | 50471 | VH1 | SYVMH |
| TABLE 50 | 50472 | VH2 | VIWYDGSNKYYADSVKG |
| | 50473 | VH3 | ERYSSGWYDYGMDV |
| | | | |
| VH-CONSENSUS-17 | 50474 | VH1 | DYGMH |
| TABLE 51 | 50475 | VH2 | VIWYDENNKYYADSVKG |
| | 50476 | VH3 | ELGFSDY |
| | | | |
| VH-CONSENSUS-18 | 50477 | VH1 | GCYWS |
| TABLE 52 | 50478 | VH2 | EINHSGSTNYNPSLKS |
| | 50479 | VH3 | DYGGMDV |
| | | | |
| VH-CONSENSUS-19 | 50480 | VH1 | NYDIN |
| TABLE 53 | 50481 | VH2 | WMHPNSGNTGYAQKFQG |
| | 50482 | VH3 | SSGWYWFDP |
| | | | |
| VH-CONSENSUS-20 | 50483 | VH1 | SYGMH |
| TABLE 54 | 50484 | VH2 | VIWYDGSNKNYADSVKG |
| | 50485 | VH3 | DQGVGYYGLDV |
| | | | |
| VH-CONSENSUS-21 | 50486 | VH1 | GYYMH |
| TABLE 55 | 50487 | VH2 | WINPNSGGTNYAQKFQG |
| | 50488 | VH3 | DGTSSFDY |
| | | | |
| VH-CONSENSUS-22 | 50489 | VH1 | SYGMH |
| TABLE 56 | 50490 | VH2 | VIWHDGSNKYYADSVKG |
| | 50491 | VH3 | DLSMGGMDV |
| | | | |
| VH-CONSENSUS-23 | 50492 | VH1 | DYGMH |
| TABLE 57 | 50493 | VH2 | VIWYDESNKYYADSVKG |

**FIGURE 55**

| | | | |
|---|---|---|---|
| VH-CONSENSUS-24 TABLE 58 | 50495 | VH1 | SYSMN |
| | 50496 | VH2 | SISGSSSYIYYADSVKG |
| | 50497 | VH3 | DRGSS |
| VH-CONSENSUS-25 TABLE 59 | 50498 | VH1 | SYAMS |
| | 50499 | VH2 | VISGRGGNTFYADSVKG |
| | 50500 | VH3 | RIAVAGSEAFDI |
| VH-CONSENSUS-26 TABLE 60 | 50501 | VH1 | SYAMS |
| | 50502 | VH2 | AISGRGGSTFHADSVKG |
| | 50503 | VH3 | GELLEDYYFYGMDV |
| VH-CONSENSUS-27 TABLE 61 | 50504 | VH1 | SYGMH |
| | 50505 | VH2 | VIWYDGSNKYYADSVKG |
| | 50506 | VH3 | DRVYCSSTSCPYYYYYGMDV |
| VH-CONSENSUS-28 TABLE 62 | 50507 | VH1 | SYSMN |
| | 50508 | VH2 | SISGSSSYIYYADSVKG |
| | 50509 | VH3 | VASFDY |
| VH-CONSENSUS-29 TABLE 63 | 50510 | VH1 | SGDYYWN |
| | 50511 | VH2 | YIFYSGSTYYNPSLKS |
| | 50512 | VH3 | GDYDGSGSYHYYYGMDV |
| VH-CONSENSUS-30 TABLE 64 | 50513 | VH1 | SYAMS |
| | 50514 | VH2 | VISGGGSSTYYADSVKG |
| | 50515 | VH3 | WRGNPTDYGMD |
| VH-CONSENSUS-31 TABLE 65 | 50516 | VH1 | SYGMH |
| | 50517 | VH2 | IIWYDGSNKYYADSVKG |
| | 50518 | VH3 | DHYDFWSGHFDY |
| VH-CONSENSUS-32 TABLE 66 | 50519 | VH1 | GYYWS |
| | 50520 | VH2 | EINHSGRTNYNPSLKS |
| | 50521 | VH3 | DYGGLDY |
| VH-CONSENSUS-33 TABLE 67 | 50522 | VH1 | RSSYYWG |
| | 50523 | VH2 | NIYYSGSTYYNPSLKS |
| | 50524 | VH3 | HSSSWSLDY |
| VH-CONSENSUS-34 TABLE 68 | 50525 | VH1 | GYYIH |
| | 50526 | VH2 | WINPNSGGTNYAQKFQG |
| | 50527 | VH3 | GYSYGYNWFDP |
| VH-CONSENSUS-35 TABLE 69 | 50528 | VH1 | HYGMH |
| | 50529 | VH2 | VIWYDGSNKYYADSVKG |

**FIGURE 55**

| | | | |
|---|---|---|---|
| VH-CONSENSUS-36 TABLE 70 | 50531 | VH1 | SYAMS |
| | 50532 | VH2 | AISGSGGNTFYADSVKG |
| | 50533 | VH3 | KDYDYVWGSPYFDY |
| VH-CONSENSUS-37 TABLE 71 | 50534 | VH1 | GYYMH |
| | 50535 | VH2 | WINPNSGGTNYAQKFQG |
| | 50536 | VH3 | SYYYGSGSYYNEFDY |
| VH-CONSENSUS-38 TABLE 72 | 50537 | VH1 | GYYIH |
| | 50538 | VH2 | WINPYSGDTNYAQKFQG |
| | 50539 | VH3 | DWGGYSSYYYGMDV |
| VH-CONSENSUS-39 TABLE 73 | 50540 | VH1 | SYSMN |
| | 50541 | VH2 | SISGSSSYIYYADSVKG |
| | 50542 | VH3 | LTFDY |
| VH-CONSENSUS-40 TABLE 74 | 50543 | VH1 | TYGMH |
| | 50544 | VH2 | IIWYDGTNKYYADSVKG |
| | 50545 | VH3 | DPLRGYNDPVMDY |
| VH-CONSENSUS-41 TABLE 75 | 50546 | VH1 | SYAMS |
| | 50547 | VH2 | AISGRGGNTFYADSVKG |
| | 50548 | VH3 | RVTDYGGNDWFDP |
| VH-CONSENSUS-42 TABLE 76 | 50549 | VH1 | TYGMH |
| | 50550 | VH2 | VIWYGGSNKDYADSVKG |
| | 50551 | VH3 | DRDYCSGGSCPYYYYYGMDV |
| VH-CONSENSUS-43 TABLE 77 | 50552 | VH1 | RSSYYWG |
| | 50553 | VH2 | NIYYSGSTYYNPSLKS |
| | 50554 | VH3 | HGKDWGLDY |
| VH-CONSENSUS-44 TABLE 78 | 50555 | VH1 | GYYMH |
| | 50556 | VH2 | WIKPNSGGTNQAQKFQG |
| | 50557 | VH3 | APGTAAAGTWGYFDY |
| VH-CONSENSUS-45 TABLE 79 | 50558 | VH1 | TGGVGVG |
| | 50559 | VH2 | LIYWDDDKRYSPSLKS |
| | 50560 | VH3 | LIAVAFDY |
| VH-CONSENSUS-46 TABLE 80 | 50561 | VH1 | SYGMH |
| | 50562 | VH2 | IIWYDGSYKYYADSVKG |
| | 50563 | VH3 | EAYDFWSGYFDY |
| VH-CONSENSUS-47 TABLE 81 | 50564 | VH1 | SYGMH |
| | 50565 | VH2 | VIWYDGSNKYYADSVKG |
| | 50566 | VH3 | DRDYGDYGMDV |

FIGURE 55

| | | | |
|---|---|---|---|
| VH-CONSENSUS-48<br>TABLE 82 | 50567 | VH1 | SYAMS |
| | 50568 | VH2 | AISGSGGNTFYADSVKG |
| | 50569 | VH3 | LGKDYYYYGMDV |
| | | | |
| VH-CONSENSUS-49<br>TABLE 83 | 50570 | VH1 | SYVMS |
| | 50571 | VH2 | AMSGSGGRTYYADSVKG |
| | 50572 | VH3 | LTAFDY |
| | | | |
| VH-CONSENSUS-50<br>TABLE 84 | 50573 | VH1 | SYGMH |
| | 50574 | VH2 | IISYAGSNKYYADSVKG |
| | 50575 | VH3 | RGYSYGGYGMDV |
| | | | |
| VH-CONSENSUS-51<br>TABLE 85 | 50576 | VH1 | DYVMH |
| | 50577 | VH2 | VIWYDGSNKYYADSVKG |
| | 50578 | VH3 | EPYTSGWYDYGMDV |
| | | | |
| VH-CONSENSUS-52<br>TABLE 86 | 50579 | VH1 | SYGIS |
| | 50580 | VH2 | WISAYNGNTKYAQKLQG |
| | 50581 | VH3 | HDFWSGYYKGMDV |
| | | | |
| VH-CONSENSUS-53<br>TABLE 87 | 50582 | VH1 | SYAMS |
| | 50583 | VH2 | AISGRGG NTFDADSVKG |
| | 50584 | VH3 | ERSGSYFDY |
| | | | |
| VH-CONSENSUS-54<br>TABLE 88 | 50585 | VH1 | NYGMH |
| | 50586 | VH2 | VIWHDGSNKYYADSVKG |
| | 50587 | VH3 | ENSSSYYFDY |
| | | | |
| VH-CONSENSUS-55<br>TABLE 89 | 50588 | VH1 | TSGVGVG |
| | 50589 | VH2 | LINWNDDKRYSPSLKS |
| | 50590 | VH3 | KATWVAFDI |
| | | | |
| VH-CONSENSUS-56<br>TABLE 90 | 50591 | VH1 | SYVMN |
| | 50592 | VH2 | AISGSGGRTYYADSVKG |
| | 50593 | VH3 | TATFDY |
| | | | |
| VH-CONSENSUS-57<br>TABLE 91 | 50594 | VH1 | SYAMS |
| | 50595 | VH2 | VISGRGGTTFYADSVKG |
| | 50596 | VH3 | KRTPSDVFDI |
| | | | |
| VH-CONSENSUS-58<br>TABLE 92 | 50597 | VH1 | SYGMH |
| | 50598 | VH2 | VIWYDGSNKYYADSVKG |
| | 50599 | VH3 | DRPRSSAFDY |
| | | | |
| VH-CONSENSUS-59<br>TABLE 93 | 50600 | VH1 | SYNMN |
| | 50601 | VH2 | YISRSSNTKYYADSVKG |
| | 50602 | VH3 | DRSGSYGYFYYYGLDV |

**FIGURE 55**

| | | | |
|---|---|---|---|
| TABLE 94 | 50604 | VH2 | YIYYSGSTYYNPSLKS |
| | 50605 | VH3 | DSSSYGMDV |
| | | | |

**FIGURE 55**

Table 19C

**VARIABLE HEAVY CDR CONSENSUS SEQUENCES II**

| NAME (ORIGINAL AND PATENT) | Patent SEQ ID NO: | CDR | Sequence |
|---|---|---|---|
| VH-CONSENSUS-15 TABLE 49 | SEQ ID NO: 50001 | VH1 | X1 Tyr Asp X2 Asn, wherein X1 = N or H or a conservative substitution thereof, X2 = I or V or L or a conservative substitution thereof. |
| | SEQ ID NO: 50002 | VH2 | X1 X2 X3 Pro X4 Ser X5 X6 X7 X8 X9 X10 X11 X12 Phe X13 X14 wherein X1 = W or R or a conservative substitution thereof, X2 = M or V or L or a conservative substitution thereof, X3 = H or N or Y or T or a conservative substitution thereof, X4 = N or D or H or a conservative substitution thereof, X5 = G or H or a conservative substitution thereof, X6 = N or S or Q or A or D or K or T or a conservative substitution thereof, X7 = T or A or V or E or a conservative substitution thereof, X8 = G or D or a conservative substitution thereof, X9 = Y or F or C or a conservative substitution thereof, X10 = A or P or a conservative substitution thereof, X11 = Q or K or a conservative substitution thereof, X12 = K or R or N or a conservative substitution thereof, X13 = Q or R or a conservative substitution thereof, X14 = G or V or a conservative substitution thereof. |
| | SEQ ID NO: 50003 | VH3 | Ser Ser Gly Trp X1 X2 Phe Asp X3, wherein X1 = Y or E or N or T or a conservative substitution thereof, X2 = Y or F or K or I or R or V or L or M or W or H or S or a conservative substitution thereof, X3 = Y or F or S or N or a conservative substitution thereof. |
| | | | |
| VH-CONSENSUS-16 TABLE 50 | SEQ ID NO: 50004 | VH1 | X1 X2 X3 X4 X5, wherein X1 = S or D or N or I or a conservative substitution thereof, X2 = Y or C or F or D or S or a conservative substitution thereof, X3 = V or G or I or L or a conservative substitution thereof, X4 = M or I or L or a conservative substitution thereof, X5 = H or D or a conservative substitution thereof. |
| | SEQ ID NO: 50005 | VH2 | X1 Ile X2 Tyr Asp X3 X4 X5 Lys X6 X7 X8 X9 X10 X11 Lys Gly, wherein X1 = V or L or A or a conservative substitution thereof, X2 = W or F or a conservative substitution thereof, X3 = G or A or a conservative substitution thereof, X4 = S or R or N or a conservative substitution thereof, X5 = N or Y or G or S or a conservative substitution thereof, X6 = Y or H or a conservative substitution thereof, X7 = Y or H or N or a conservative substitution thereof, X8 = A or V or E or G or T or a conservative substitution thereof, X9 = D or E or G or a conservative substitution thereof, X10 = S or A or a conservative substitution thereof, X11 = V or M or a |

FIGURE 55

| | | | |
|---|---|---|---|
| | SEQ ID NO: 50006 | VH3 | X1 X2 X3 X4 X5 X6 X7 X8 X9 X10 X11 X12 X13 X14 X15 X16 Gly X17 X18 Val, wherein X1 = E or R or V or a conservative substitution thereof, X2 = R or Y or K or V or E or P or D or L or F or M or N or Q or T or a conservative substitution thereof, X3 = Y or S or T or V or a conservative substitution thereof, X4 = S or R or Y or P or T or G or a conservative substitution thereof, X5 = S or C or Y or a conservative substitution thereof, X6 = G or W or S or N or a conservative substitution thereof, X7 = W or Absent or L or Y or G or F or S or a conservative substitution thereof, X8 = Absent or Y or a conservative substitution thereof, X9 = Absent or A or G or T or a conservative substitution thereof, X10 = Absent or C or a conservative substitution thereof, X11 = Absent or P or a conservative substitution thereof, X12 = Absent or Y or L or a conservative substitution thereof, X13 = Absent or Y or D or a conservative substitution thereof, X14 = Y or Absent or F or H or a conservative substitution thereof, X15 = D or S or G or T or V or Y or A or F or M or a conservative substitution thereof, X16 = Y or G or F or a conservative substitution thereof, X17 = M or L or a conservative substitution thereof, X18 = D or G or a conservative substitution thereof. |
| | | | |
| VH-CONSENSUS-17 TABLE 51 | SEQ ID NO: 50007 | VH1 | X1 X2 Gly X3 His, wherein X1 = D or S or N or T or a conservative substitution thereof, X2 = Y or F or a conservative substitution thereof, X3 = M or I or a conservative substitution thereof. |
| | SEQ ID NO: 50008 | VH2 | X1 X2 Trp X3 X4 X5 X6 X7 X8 X9 X10 X11 X12 Ser X13 X14 Gly, wherein X1 = V or L or a conservative substitution thereof, X2 = I or V or T or M or a conservative substitution thereof, X3 = Y or F or D or a conservative substitution thereof, X4 = D or E or A or G or N or a conservative substitution thereof, X5 = E or G or V or R or D or a conservative substitution thereof, X6 = N or S or T or D or I or Y or a conservative substitution thereof, X7 = N or H or K or a conservative substitution thereof, X8 = K or Q or E or N or R or a conservative substitution thereof, X9 = Y or H or K or D or R or S or a conservative substitution thereof, X10 = Y or H or a conservative substitution thereof, X11 = A or V or G or T or I or E or a conservative substitution thereof, X12 = D or E or a conservative substitution thereof, X13 = V or M or a conservative substitution thereof, X14 = K or R or a conservative substitution thereof. |
| | SEQ ID NO: 50009 | VH3 | X1 Leu X2 X3 X4 X5 X6 X7, wherein X1 = E or D or G or a conservative substitution thereof, X2 = G or A or a conservative substitution thereof, X3 = F or W or M or a conservative substitution thereof, X4 = L or R or T or Y or |

FIGURE 55

| | | | |
|---|---|---|---|
| | | | thereof, X5 = S or E or G or D or F or N or A or T or a conservative substitution thereof, X6 = D or E or a conservative substitution thereof, X7 = Y or S or F or C or a conservative substitution thereof. |
| | | | |
| VH-CONSENSUS-18 TABLE 52 | SEQ ID NO: 50010 | VH1 | Gly X1 Tyr Trp Ser, wherein X1 = C or S or P or Y or a conservative substitution thereof. |
| | SEQ ID NO: 50011 | VH2 | Glu Ile Asn X1 X2 Gly X3 Thr X4 X5 Asn Pro Ser Leu X6 Ser, wherein X1 = H or Y or Q or a conservative substitution thereof, X2 = S or R or a conservative substitution thereof, X3 = S or R or C or I or a conservative substitution thereof, X4 = N or S or a conservative substitution thereof, X5 = Y or F or a conservative substitution thereof, X6 = K or T or a conservative substitution thereof. |
| | SEQ ID NO: 50012 | VH3 | Asp Tyr Gly Gly X1 Asp Val, wherein X1 = M or L or I or a conservative substitution thereof. |
| | | | |
| VH-CONSENSUS-19 TABLE 53 | SEQ ID NO: 50013 | VH1 | Asn Tyr Asp Ile Asn. |
| | SEQ ID NO: 50014 | VH2 | Trp Met X1 Pro X2 X3 X4 X5 X6 Gly X7 Ala Gln Lys Phe Gln X8, wherein X1 = H or N or Y or a conservative substitution thereof, X2 = N or D or a conservative substitution thereof, X3 = S or N or a conservative substitution thereof, X4 = G or V or a conservative substitution thereof, X5 = N or S or a conservative substitution thereof, X6 = T or I or a conservative substitution thereof, X7 = Y or F or C or a conservative substitution thereof, X8 = G or D or a conservative substitution thereof. |
| | SEQ ID NO: 50015 | VH3 | Ser Ser Gly Trp X1 X2 Phe Asp Pro, wherein X1 = Y or H or N or S or K or a conservative substitution thereof, X2 = W or R or a conservative substitution thereof. |
| | | | |
| VH-CONSENSUS-20 TABLE 54 | SEQ ID NO: 50016 | VH1 | X1 X2 Gly Met X3, wherein X1 = S or N or R or T or I or a conservative substitution thereof, X2 = Y or H or N or a conservative substitution thereof, X3 = H or D or a conservative substitution thereof. |
| | SEQ ID NO: 50017 | VH2 | X1 X2 Trp X3 Asp Gly X4 Asn X5 X6 X7 X8 X9 Ser Val Lys Gly, wherein X1 = V or I or a conservative substitution thereof, X2 = I or L or a conservative substitution thereof, X3 = Y or F or a conservative substitution thereof, X4 = S or T or a conservative substitution thereof, X5 = K or E or Q or D or R or a conservative substitution thereof, X6 = N or H or Y or a conservative substitution thereof, X7 = Y or H or a conservative substitution thereof, X8 = A or V or G |

FIGURE 55

| | | | |
|---|---|---|---|
| | | | or a conservative substitution thereof, X9 = D or E or a conservative substitution thereof. |
| | SEQ ID NO: 50018 | VH3 | X1 X2 Gly X3 X4 X5 X6 X7 X8 X9 X10 X11 X12 X13 Asp Val, wherein X1 = D or A or a conservative substitution thereof, X2 = Q or R or Y or H or A or C or E or F or M or a conservative substitution thereof, X3 = V or I or F or a conservative substitution thereof, X4 = G or Y or a conservative substitution thereof, X5 = Y or E or a conservative substitution thereof, X6 = Absent or F or a conservative substitution thereof, X7 = Absent or D or a conservative substitution thereof, X8 = Absent or Y or a conservative substitution thereof, X9 = Absent or Y or a conservative substitution thereof, X10 = Absent or N or a conservative substitution thereof, X11 = Y or D or N or a conservative substitution thereof, X12 = G or A or D or a conservative substitution thereof, X13 = L or M or T or I or a conservative substitution thereof. |
| | | | |
| VH-CONSENSUS-21 TABLE 55 | SEQ ID NO: 50019 | VH1 | X1 X2 X3 X4 X5, wherein X1 = G or D or S or A or a conservative substitution thereof, X2 = Y or D or a conservative substitution thereof, X3 = Y or H or N or F or a conservative substitution thereof, X4 = M or L or I or a conservative substitution thereof, X5 = H or Q or a conservative substitution thereof. |
| | SEQ ID NO: 50020 | VH2 | Trp X1 X2 Pro X3 X4 X5 X6 X7 X8 X9 X10 Gln X11 Phe Gln X12, wherein X1 = I or V or a conservative substitution thereof, X2 = N or H or K or S or a conservative substitution thereof, X3 = N or K or a conservative substitution thereof, X4 = S or N or R or T or a conservative substitution thereof, X5 = G or N or D or a conservative substitution thereof, X6 = G or A or a conservative substitution thereof, X7 = T or S or a conservative substitution thereof, X8 = N or H or Q or I or a conservative substitution thereof, X9 = Y or S or F or a conservative substitution thereof, X10 = A or T or a conservative substitution thereof, X11 = K or R or N or E or S or a conservative substitution thereof, X12 = G or D or a conservative substitution thereof. |
| | SEQ ID NO: 50021 | VH3 | X1 X2 X3 X4 X5 X6 X7 X8 X9 X10 X11 X12 X13, wherein X1 = D or K or G or S or E or a conservative substitution thereof, X2 = G or F or A or K or V or a conservative substitution thereof, X3 = T or Absent or P or a conservative substitution thereof, X4 = S or G or Absent or T or a conservative substitution thereof, X5 = Absent or V or S or a conservative substitution thereof, X6 = Absent or A or a conservative substitution thereof, X7 = Absent or T or a conservative substitution thereof, X8 = Absent or W or a conservative substitution thereof, X9 = Absent or G or a conservative substitution thereof, X10 = S or Absent or V |

FIGURE 55

| | | | |
|---|---|---|---|
| | | | Absent or L or Y or a conservative substitution thereof, X12 = D or G or K or a conservative substitution thereof, X13 = Y or D or F or a conservative substitution thereof. |
| | | | |
| VH-CONSENSUS-22 TABLE 56 | SEQ ID NO: 50022 | VH1 | X1 X2 Gly X3 His, wherein X1 = S or N or a conservative substitution thereof, X2 = Y or F or H or a conservative substitution thereof, X3 = M or I or a conservative substitution thereof. |
| | SEQ ID NO: 50023 | VH2 | X1 Ile X2 X3 X4 X5 X6 X7 X8 X9 X10 X11 X12 X13 Val X14 Gly, wherein X1 = V or I or a conservative substitution thereof, X2 = W or I or a conservative substitution thereof, X3 = H or Y or F or N or a conservative substitution thereof, X4 = D or S or E or a conservative substitution thereof, X5 = G or A or a conservative substitution thereof, X6 = S or G or a conservative substitution thereof, X7 = N or Y or a conservative substitution thereof, X8 = K or D or E or R or a conservative substitution thereof, X9 = Y or N or a conservative substitution thereof, X10 = Y or N or a conservative substitution thereof, X11 = A or V or G or a conservative substitution thereof, X12 = D or E or a conservative substitution thereof, X13 = S or A or a conservative substitution thereof, X14 = K or R or a conservative substitution thereof. |
| | SEQ ID NO: 50024 | VH3 | X1 X2 X3 X4 X5 X6 X7 X8 X9 X10 X11 X12 X13 X14 X15 X16 X17 Gly X18 Asp Val, wherein X1 = D or T or R or a conservative substitution thereof, X2 = L or R or Y or S or F or I or P or a conservative substitution thereof, X3 = S or R or T or a conservative substitution thereof, X4 = M or V or G or P or K or N or Y or a conservative substitution thereof, X5 = G or Y or Absent or S or a conservative substitution thereof, X6 = Absent or Y or S or W or a conservative substitution thereof, X7 = Absent or G or a conservative substitution thereof, X8 = Absent or S or a conservative substitution thereof, X9 = Absent or G or a conservative substitution thereof, X10 = Absent or S or a conservative substitution thereof, X11 = Absent or P or a conservative substitution thereof, X12 = Absent or P or a conservative substitution thereof, X13 = Absent or Y or a conservative substitution thereof, X14 = Absent or Y or a conservative substitution thereof, X15 = Absent or Y or a conservative substitution thereof, X16 = Absent or S or Y or a conservative substitution thereof, X17 = Absent or D or Y or G or a conservative substitution thereof, X18 = M or L or T or a conservative substitution thereof. |
| | | | |
| VH-CONSENSUS-23 TABLE 57 | SEQ ID NO: 50025 | VH1 | X1 X2 Gly X3 His, wherein X1 = D or N or S or a conservative substitution thereof, X2 = Y or F or a conservative substitution thereof, X3 = M or I or L or a conservative substitution thereof. |

**FIGURE 55**

| | | | |
|---|---|---|---|
| | SEQ ID NO: 50026 | VH2 | Val Ile Trp X1 X2 X3 X4 Asn X5 X6 Tyr X7 X8 Ser X9 Lys Gly, wherein X1 = Y or F or a conservative substitution thereof, X2 = D or E or I or V or a conservative substitution thereof, X3 = E or G or V or A or R or a conservative substitution thereof, X4 = S or N or T or G or a conservative substitution thereof, X5 = K or Q or T or N or a conservative substitution thereof, X6 = Y or H or K or a conservative substitution thereof, X7 = A or T or G or E or V or a conservative substitution thereof, X8 = D or G or a conservative substitution thereof, X9 = V or A or a conservative substitution thereof. |
| | SEQ ID NO: 50027 | VH3 | Glu X1 Gly X2 X3 X4 Asp X5, wherein X1 = V or I or M or K or T or a conservative substitution thereof, X2 = W or F or G or M or a conservative substitution thereof, X3 = L or T or S or Y or H or R or a conservative substitution thereof, X4 = D or E or S or F or N or a conservative substitution thereof, X5 = Y or C or a conservative substitution thereof. |
| | | | |
| VH-CONSENSUS-24 TABLE 58 | SEQ ID NO: 50028 | VH1 | X1 X2 X3 X4 Asn, wherein X1 = S or T or a conservative substitution thereof, X2 = Y or F or a conservative substitution thereof, X3 = S or T or G or R or a conservative substitution thereof, X4 = M or L or a conservative substitution thereof. |
| | SEQ ID NO: 50029 | VH2 | X1 Ile Ser X2 Ser X3 X4 X5 X6 X7 Tyr X8 Asp Ser X9 Lys X10, wherein X1 = S or A or C or L or a conservative substitution thereof, X2 = G or S or a conservative substitution thereof, X3 = S or G or T or a conservative substitution thereof, X4 = S or T or G or Y or a conservative substitution thereof, X5 = Y or H or a conservative substitution thereof, X6 = I or L or M or a conservative substitution thereof, X7 = Y or S or W or a conservative substitution thereof, X8 = A or G or P or V or a conservative substitution thereof, X9 = V or L or a conservative substitution thereof, X10 = G or A or a conservative substitution thereof. |
| | SEQ ID NO: 50030 | VH3 | X1 Arg X2 X3 X4 X5 X6 X7, wherein X1 = D or T or a conservative substitution thereof, X2 = G or Absent or S or Y or a conservative substitution thereof, X3 = Absent or G or a conservative substitution thereof, X4 = Absent or S or a conservative substitution thereof, X5 = Absent or F or S or a conservative substitution thereof, X6 = S or D or G or H or a conservative substitution thereof, X7 = S or L or Y or G or T or C or E or I or a conservative substitution thereof. |
| | | | |
| VH-CONSENSUS-25 TABLE 59 | SEQ ID NO: 50031 | VH1 | X1 Tyr X2 Met X3, wherein X1 = S or G or a conservative substitution thereof, X2 = A or V or a conservative substitution thereof, X3 = S or N or T or a conservative substitution thereof. |

**FIGURE 55**

| | | | |
|---|---|---|---|
| | SEQ ID NO: 50032 | VH2 | X1 Ile Ser X2 X3 Gly X4 X5 X6 X7 X8 Ala Asp Ser Val X9 Gly, wherein X1 = V or I or A or a conservative substitution thereof, X2 = G or R or a conservative substitution thereof, X3 = R or S or a conservative substitution thereof, X4 = G or V or I or T or a conservative substitution thereof, X5 = N or Y or S or T or a conservative substitution thereof, X6 = T or A or a conservative substitution thereof, X7 = F or Y or a conservative substitution thereof, X8 = Y or N or S or a conservative substitution thereof, X9 = K or R or a conservative substitution thereof. |
| | SEQ ID NO: 50033 | VH3 | Arg X1 Ala Val X2 Gly X3 X4 Ala X5 X6 X7, wherein X1 = I or L or M or V or a conservative substitution thereof, X2 = A or D or a conservative substitution thereof, X3 = S or N or Y or a conservative substitution thereof, X4 = E or D or a conservative substitution thereof, X5 = F or C or a conservative substitution thereof, X6 = D or A or H or a conservative substitution thereof, X7 = I or V or a conservative substitution thereof. |
| | | | |
| VH-CONSENSUS-26 TABLE 60 | SEQ ID NO: 50034 | VH1 | X1 X2 X3 Met X4, wherein X1 = S or N or a conservative substitution thereof, X2 = Y or C or a conservative substitution thereof, X3 = A or V or a conservative substitution thereof, X4 = S or N or a conservative substitution thereof. |
| | SEQ ID NO: 50035 | VH2 | X1 X2 Ser X3 X4 Gly Gly X5 X6 X7 X8 Ala Asp Ser X9 Lys Gly, wherein X1 = A or T or S or a conservative substitution thereof, X2 = I or L or a conservative substitution thereof, X3 = G or R or V or a conservative substitution thereof, X4 = R or G or a conservative substitution thereof, X5 = S or N or a conservative substitution thereof, X6 = T or I or a conservative substitution thereof, X7 = F or Y or a conservative substitution thereof, X8 = H or Y or N or a conservative substitution thereof, X9 = V or E or M or a conservative substitution thereof. |
| | SEQ ID NO: 50036 | VH3 | X1 X2 Leu Leu X3 X4 Tyr X5 X6 X7 X8 X9 X10 Asp Val, wherein X1 = G or W or a conservative substitution thereof, X2 = E or G or a conservative substitution thereof, X3 = E or Y or a conservative substitution thereof, X4 = D or S or N or a conservative substitution thereof, X5 = Absent or E or a conservative substitution thereof, X6 = Y or S or a conservative substitution thereof, X7 = F or Y or a conservative substitution thereof, X8 = Y or F or a conservative substitution thereof, X9 = G or A or a conservative substitution thereof, X10 = M or I or L or a conservative substitution thereof. |
| | | | |

**FIGURE 55**

| | | | |
|---|---|---|---|
| VH-CONSENSUS-27 TABLE 61 | SEQ ID NO: 50037 | VH1 | X1 Tyr X2 X3 X4, wherein X1 = S or G or D or R or H or T or N or a conservative substitution thereof, X2 = G or V or a conservative substitution thereof, X3 = M or L or a conservative substitution thereof, X4 = H or N or a conservative substitution thereof. |
| | SEQ ID NO: 50038 | VH2 | X1 X2 X3 X4 Asp Gly X5 X6 X7 X8 X9 X10 Asp Ser Val Lys X11, wherein X1 = V or L or F or I or D or a conservative substitution thereof, X2 = I or F or a conservative substitution thereof, X3 = W or R or a conservative substitution thereof, X4 = Y or F or a conservative substitution thereof, X5 = S or N or T or a conservative substitution thereof, X6 = N or E or D or K or a conservative substitution thereof, X7 = K or N or T or a conservative substitution thereof, X8 = Y or S or D or N or a conservative substitution thereof, X9 = Y or N or a conservative substitution thereof, X10 = A or V or a conservative substitution thereof, X11 = G or D or a conservative substitution thereof. |
| | SEQ ID NO: 50039 | VH3 | Asp X1 X2 X3 X4 X5 X6 X7 X8 X9 X10 X11 X12 X13 X14 X15 Tyr X16 X17 Asp Val, wherein X1 = R or D or W or N or a conservative substitution thereof, X2 = V or D or R or F or H or a conservative substitution thereof, X3 = Y or S or E or G or F or a conservative substitution thereof, X4 = C or G or E or a conservative substitution thereof, X5 = S or G or a conservative substitution thereof, X6 = S or G or R or N or a conservative substitution thereof, X7 = T or Absent or S or P or a conservative substitution thereof, X8 = S or P or Absent or T or a conservative substitution thereof, X9 = C or Absent or a conservative substitution thereof, X10 = Absent or S or H or L or Y or a conservative substitution thereof, X11 = P or Absent or S or Y or a conservative substitution thereof, X12 = Y or Absent or a conservative substitution thereof, X13 = Y or Absent or a conservative substitution thereof, X14 = Y or Absent or a conservative substitution thereof, X15 = Y or F or a conservative substitution thereof, X16 = G or A or a conservative substitution thereof, X17 = M or L or a conservative substitution thereof. |
| | | | |
| VH-CONSENSUS-28 TABLE 62 | SEQ ID NO: 50040 | VH1 | X1 Tyr X2 X3 Asn, wherein X1 = S or N or a conservative substitution thereof, X2 = S or T or K or N or R or a conservative substitution thereof, X3 = M or L or a conservative substitution thereof. |
| | SEQ ID NO: 50041 | VH2 | Ser X1 Ser X2 X3 X4 X5 X6 X7 X8 Tyr X9 Asp Ser Val Lys Gly, wherein X1 = I or T or a conservative substitution thereof, X2 = G or S or a conservative substitution thereof, X3 = S or G or N or T or a conservative substitution thereof, X4 = S or G or D or N or a conservative substitution thereof, X5 = S or T or a conservative |

FIGURE 55

| | | | |
|---|---|---|---|
| | | | conservative substitution thereof, X7 = I or M or T or a conservative substitution thereof, X8 = Y or N or a conservative substitution thereof, X9 = A or T or a conservative substitution thereof. |
| | SEQ ID NO: 50042 | VH3 | Val X1 X2 X3 Asp X4, wherein X1 = A or N or S or a conservative substitution thereof, X2 = S or A or G or T or L or H or N or a conservative substitution thereof, X3 = F or L or N or a conservative substitution thereof, X4 = Y or C or S or a conservative substitution thereof. |
| | | | |
| VH-CONSENSUS-29 TABLE 63 | SEQ ID NO: 50230 | VH1 | Ser X1 X2 Tyr X3 Trp X4, wherein X1 = G or V or a conservative substitution thereof, X2 = D or V or G or S or a conservative substitution thereof, X3 = Y or H or a conservative substitution thereof, X4 = N or S or a conservative substitution thereof. |
| | SEQ ID NO: 50231 | VH2 | X1 X2 X3 X4 Ser Gly Ser Thr Tyr X5 Asn Pro Ser Leu X6 Ser, wherein X1 = Y or N or F or a conservative substitution thereof, X2 = I or L or a conservative substitution thereof, X3 = F or Y or H or a conservative substitution thereof, X4 = Y or H or a conservative substitution thereof, X5 = Y or N or a conservative substitution thereof, X6 = K or R or a conservative substitution thereof. |
| | SEQ ID NO: 50232 | VH3 | Gly Asp Tyr Asp Gly Ser Gly Ser Tyr His X1 Tyr X2 Gly X3 Asp Val, wherein X1 = Y or F or H or a conservative substitution thereof, X2 = Y or H or a conservative substitution thereof, X3 = M or L or a conservative substitution thereof. |
| | | | |
| VH-CONSENSUS-30 TABLE 64 | SEQ ID NO: 50043 | VH1 | X1 X2 X3 Met Ser, wherein X1 = S or N or T or a conservative substitution thereof, X2 = Y or H or a conservative substitution thereof, X3 = A or P or V or a conservative substitution thereof. |
| | SEQ ID NO: 50044 | VH2 | X1 Ile Ser Gly X2 Gly X3 X4 X5 Tyr Tyr Ala Asp Ser Val Lys Gly, wherein X1 = V or A or I or a conservative substitution thereof, X2 = G or S or a conservative substitution thereof, X3 = S or G or T or a conservative substitution thereof, X4 = S or T or a conservative substitution thereof, X5 = T or A or a conservative substitution thereof. |
| | SEQ ID NO: 50045 | VH3 | X1 X2 Gly X3 X4 X5 X6 X7 X8 X9 X10 Gly Met Asp, wherein X1 = W or A or a conservative substitution thereof, X2 = R or G or a conservative substitution thereof, X3 = N or T or a conservative substitution thereof, X4 = P or T or a conservative substitution thereof, X5 = T or G or a conservative substitution thereof, X6 = Absent or S or a conservative substitution thereof, X7 = Absent or Y or a conservative substitution thereof, X8 = Absent or Y or a conservative substitution thereof, X9 = D or Y or a |

FIGURE 55

| | | | |
|---|---|---|---|
| | | | conservative substitution thereof, X10 = Y or N or S or a conservative substitution thereof. |
| | | | |
| VH-CONSENSUS-31 TABLE 65 | SEQ ID NO: 50253 | VH1 | X1 Tyr Gly X2 His, wherein X1 = S or N or T or a conservative substitution thereof, X2 = M or L or a conservative substitution thereof. |
| | SEQ ID NO: 50254 | VH2 | X1 X2 Trp Tyr Asp Gly X3 X4 Lys Tyr Tyr X5 Asp Ser Val Lys Gly, wherein X1 = I or V or A or a conservative substitution thereof, X2 = I or M or a conservative substitution thereof, X3 = S or T or a conservative substitution thereof, X4 = N or Y or S or a conservative substitution thereof, X5 = A or G or T or V or a conservative substitution thereof. |
| | SEQ ID NO: 50255 | VH3 | X1 X2 X3 X4 X5 X6 X7 X8 X9 X10 X11 X12, wherein X1 = D or E or a conservative substitution thereof, X2 = H or R or Q or A or G or T or Y or a conservative substitution thereof, X3 = Y or G or F or H or a conservative substitution thereof, X4 = D or I or F or a conservative substitution thereof, X5 = F or V or L or a conservative substitution thereof, X6 = W or G or Absent or a conservative substitution thereof, X7 = S or A or Absent or a conservative substitution thereof, X8 = G or T or E or a conservative substitution thereof, X9 = H or Y or W or F or a conservative substitution thereof, X10 = F or L or S or a conservative substitution thereof, X11 = D or A or C or G or a conservative substitution thereof, X12 = Y or F or S or a conservative substitution thereof. |
| | | | |
| VH-CONSENSUS-32 TABLE 66 | SEQ ID NO: 50233 | VH1 | X1 X2 X3 Trp Ser () wherein X1 = G or V or P or A or D or Y or a conservative substitution thereof, X2 = Y or C or S or P or a conservative substitution thereof, X3 = Y or F or a conservative substitution thereof. |
| | SEQ ID NO: 50234 | VH2 | Glu X1 Asn X2 Ser Gly X3 X4 X5 X6 Asn Pro Ser Leu Lys Ser, wherein X1 = I or S or V or a conservative substitution thereof, X2 = H or I or Q or a conservative substitution thereof, X3 = R or S or a conservative substitution thereof, X4 = T or A or S or a conservative substitution thereof, X5 = N or T or a conservative substitution thereof, X6 = Y or F or a conservative substitution thereof. |
| | SEQ ID NO: 50235 | VH3 | Asp Tyr Gly Gly Leu Asp Tyr. |
| | | | |
| VH-CONSENSUS-33 TABLE 67 | SEQ ID NO: 50046 | VH1 | X1 Ser X2 X3 Tyr Trp Gly, wherein X1 = R or G or a conservative substitution thereof, X2 = S or N or a conservative substitution thereof, X3 = Y or S or a conservative substitution thereof. |
| | SEQ ID NO: | VH2 | X1 Ile Tyr Tyr Ser Gly X2 X3 X4 X5 X6 Pro Ser Leu Lys Ser, wherein X1 = N or S or a conservative substitution |

FIGURE 55

| | | | |
|---|---|---|---|
| | | | substitution thereof, X3 = T or A or P or S or a conservative substitution thereof, X4 = Y or Q or S or A or N or a conservative substitution thereof, X5 = Y or C or H or a conservative substitution thereof, X6 = N or I or T or a conservative substitution thereof. |
| | SEQ ID NO: 50048 | VH3 | X1 Ser X2 Ser Trp Ser X3 Asp X4, wherein X1 = H or L or a conservative substitution thereof, X2 = S or T or G or a conservative substitution thereof, X3 = L or F or I or V or a conservative substitution thereof, X4 = Y or N or C or D or F or a conservative substitution thereof. |
| | | | |
| VH-CONSENSUS-34 TABLE 68 | SEQ ID NO: 50049 | VH1 | X1 X2 X3 X4 X5, wherein X1 = G or D or a conservative substitution thereof, X2 = Y or H or a conservative substitution thereof, X3 = Y or F or a conservative substitution thereof, X4 = I or M or a conservative substitution thereof, X5 = H or N or a conservative substitution thereof. |
| | SEQ ID NO: 50050 | VH2 | Trp Ile Asn X1 X2 X3 X4 X5 Thr Asn Tyr X6 X7 Lys Phe Gln X8, wherein X1 = P or S or a conservative substitution thereof, X2 = N or K or a conservative substitution thereof, X3 = S or N or a conservative substitution thereof, X4 = G or D or a conservative substitution thereof, X5 = G or D or a conservative substitution thereof, X6 = A or E or a conservative substitution thereof, X7 = Q or E or a conservative substitution thereof, X8 = G or D or a conservative substitution thereof. |
| | SEQ ID NO: 50051 | VH3 | X1 X2 X3 X4 X5 X6 X7 X8 X9 X10 X11 X12 X13 X14 X15, wherein X1 = G or D or a conservative substitution thereof, X2 = Y or G or T or F or a conservative substitution thereof, X3 = S or Y or D or R or a conservative substitution thereof, X4 = Y or S or a conservative substitution thereof, X5 = G or Absent or S or a conservative substitution thereof, X6 = Y or S or Absent or a conservative substitution thereof, X7 = Absent or G or a conservative substitution thereof, X8 = Absent or S or a conservative substitution thereof, X9 = Absent or Y or a conservative substitution thereof, X10 = Absent or Y or F or H or a conservative substitution thereof, X11 = N or Absent or D or a conservative substitution thereof, X12 = W or Absent or D or E or a conservative substitution thereof, X13 = F or L or a conservative substitution thereof, X14 = D or A or a conservative substitution thereof, X15 = P or S or a conservative substitution thereof. |
| | | | |
| VH-CONSENSUS-35 TABLE 69 | SEQ ID NO: 50052 | VH1 | X1 X2 Gly Met His, wherein X1 = H or N or a conservative substitution thereof, X2 = Y or S or a conservative substitution thereof. |
| | SEQ ID NO: | VH2 | X1 Ile X2 Tyr Asp Gly Ser X3 X4 X5 Tyr Ala Asp Ser Val Lys Gly, wherein X1 = V or I or a conservative substitution |

FIGURE 55

| | | | |
|---|---|---|---|
| | | | X3 = N or Y or a conservative substitution thereof, X4 = K or E or a conservative substitution thereof, X5 = Y or C or N or a conservative substitution thereof. |
| | SEQ ID NO: 50054 | VH3 | X1 X2 X3 X4 X5 X6 X7 X8 X9 X10 X11 X12 X13 X14 Gly X15 Asp Val, wherein X1 = G or D or a conservative substitution thereof, X2 = D or R or a conservative substitution thereof, X3 = W or H or a conservative substitution thereof, X4 = N or Y or a conservative substitution thereof, X5 = P or D or a conservative substitution thereof, X6 = Absent or F or a conservative substitution thereof, X7 = Absent or H or a conservative substitution thereof, X8 = Absent or V or a conservative substitution thereof, X9 = Absent or P or a conservative substitution thereof, X10 = Absent or Y or a conservative substitution thereof, X11 = Absent or Y or a conservative substitution thereof, X12 = Absent or Y or a conservative substitution thereof, X13 = Absent or Y or a conservative substitution thereof, X14 = E or Y or a conservative substitution thereof, X15 = M or L or a conservative substitution thereof. |
| | | | |
| VH-CONSENSUS-36 TABLE 70 | SEQ ID NO: 50055 | VH1 | Ser X1 Ala Met X2, wherein X1 = Y or S or a conservative substitution thereof, X2 = S or T or N or a conservative substitution thereof. |
| | SEQ ID NO: 50056 | VH2 | X1 Ile X2 Gly X3 Gly X4 X5 X6 X7 Tyr Ala Asp Ser Val Lys Gly, wherein X1 = A or V or a conservative substitution thereof, X2 = S or I or a conservative substitution thereof, X3 = S or R or N or F or a conservative substitution thereof, X4 = G or S or a conservative substitution thereof, X5 = N or R or S or a conservative substitution thereof, X6 = T or A or a conservative substitution thereof, X7 = F or Y or a conservative substitution thereof. |
| | SEQ ID NO: 50057 | VH3 | X1 X2 X3 X4 X5 X6 X7 X8 X9 X10 X11 X12 Phe Asp Tyr, wherein X1 = K or D or R or a conservative substitution thereof, X2 = D or Y or H or M or R or a conservative substitution thereof, X3 = Y or G or N or a conservative substitution thereof, X4 = D or I or R or Y or a conservative substitution thereof, X5 = Y or Absent or S or V or a conservative substitution thereof, X6 = V or Absent or G or R or S or a conservative substitution thereof, X7 = W or Absent or I or a conservative substitution thereof, X8 = Absent or A or a conservative substitution thereof, X9 = G or Absent or V or a conservative substitution thereof, X10 = S or Absent or A or T or Y or a conservative substitution thereof, X11 = P or Absent or G or I or a conservative substitution thereof, X12 = Y or F or T or a conservative substitution thereof. |
| | | | |

FIGURE 55

| | | | |
|---|---|---|---|
| VH-CONSENSUS-37 TABLE 71 | SEQ ID NO: 50058 | VH1 | X1 X2 X3 X4 His, wherein X1 = G or D or a conservative substitution thereof, X2 = Y or H or a conservative substitution thereof, X3 = Y or C or a conservative substitution thereof, X4 = M or I or a conservative substitution thereof. |
| | SEQ ID NO: 50059 | VH2 | X1 Ile X2 X3 X4 Ser Gly X5 X6 X7 X8 X9 Gln Lys Phe Gln X10, wherein X1 = W or S or a conservative substitution thereof, X2 = N or Y or a conservative substitution thereof, X3 = P or R or a conservative substitution thereof, X4 = N or K or a conservative substitution thereof, X5 = G or A or a conservative substitution thereof, X6 = T or A or a conservative substitution thereof, X7 = N or D or a conservative substitution thereof, X8 = Y or N or S or a conservative substitution thereof, X9 = A or G or V or a conservative substitution thereof, X10 = G or D or V or a conservative substitution thereof. |
| | SEQ ID NO: 50060 | VH3 | X1 X2 Tyr X3 Gly Ser Gly X4 Tyr X5 Asn X6 Phe Asp Tyr, wherein X1 = S or A or V or T or a conservative substitution thereof, X2 = Y or F or N or a conservative substitution thereof, X3 = Y or H or a conservative substitution thereof, X4 = S or T or a conservative substitution thereof, X5 = Y or H or a conservative substitution thereof, X6 = E or G or D or a conservative substitution thereof. |
| | | | |
| | | | |
| VH-CONSENSUS-38 TABLE 72 | SEQ ID NO: 50061 | VH1 | Gly Tyr Tyr X1 His, wherein X1 = I or T or a conservative substitution thereof. |
| | SEQ ID NO: 50062 | VH2 | Trp Ile Asn Pro Tyr Ser Gly X1 Thr X2 X3 Ala Gln Lys Phe Gln Gly, wherein X1 = D or G or a conservative substitution thereof, X2 = N or K or a conservative substitution thereof, X3 = Y or S or a conservative substitution thereof. |
| | SEQ ID NO: 50236 | VH3 | Asp Trp Gly Gly Tyr Ser Ser Tyr Tyr X1 Gly Met Asp Val, wherein X1 = Y or F or a conservative substitution thereof. |
| | | | |
| VH-CONSENSUS-39 TABLE 73 | SEQ ID NO: 50063 | VH1 | X1 X2 X3 Met X4, wherein X1 = S or T or a conservative substitution thereof, X2 = Y or F or a conservative substitution thereof, X3 = S or T or N or G or a conservative substitution thereof, X4 = N or S or I or a conservative substitution thereof. |
| | SEQ ID NO: 50064 | VH2 | Ser Ile Ser X1 X2 X3 X4 Tyr X5 X6 Tyr X7 Asp Ser Val Lys Gly, wherein X1 = G or S or a conservative substitution thereof, X2 = S or T or a conservative substitution thereof, X3 = S or I or N or a conservative substitution thereof, X4 = |

**FIGURE 55**

| | | | |
|---|---|---|---|
| | | | = I or M or S or a conservative substitution thereof, X6 = Y or N or a conservative substitution thereof, X7 = A or T or a conservative substitution thereof. |
| | SEQ ID NO: 50065 | VH3 | Leu Thr Phe Asp Tyr. |
| | | | |
| VH-CONSENSUS-40 TABLE 74 | SEQ ID NO: 50066 | VH1 | X1 Tyr Gly Met His, wherein X1 = T or S or a conservative substitution thereof. |
| | SEQ ID NO: 50067 | VH2 | X1 Ile Trp Tyr Asp Gly X2 Asn Lys Tyr Tyr Ala Asp Ser Val X3 Gly, wherein X1 = I or V or a conservative substitution thereof, X2 = T or S or a conservative substitution thereof, X3 = K or R or a conservative substitution thereof. |
| | SEQ ID NO: 50068 | VH3 | Asp Pro Leu Arg Gly Tyr Asn Asp Pro Val X1 Asp Tyr, wherein X1 = M or L or a conservative substitution thereof. |
| | | | |
| VH-CONSENSUS-41 TABLE 75 | SEQ ID NO: 50069 | VH1 | X1 Tyr Ala Met X2, wherein X1 = S or N or a conservative substitution thereof, X2 = S or N or T or a conservative substitution thereof. |
| | SEQ ID NO: 50070 | VH2 | Ala Ile Ser Gly X1 Gly X2 Asn Thr Phe Tyr Ala Asp Ser Val Lys Gly, wherein X1 = R or S or a conservative substitution thereof, X2 = G or K or a conservative substitution thereof. |
| | SEQ ID NO: 50071 | VH3 | Arg Val Thr Asp Tyr Gly Gly Asn Asp Trp Phe Asp Pro. |
| | | | |
| VH-CONSENSUS-42 TABLE 76 | SEQ ID NO: 50072 | VH1 | X1 Tyr Gly Met His, wherein X1 = T or S or a conservative substitution thereof. |
| | SEQ ID NO: 50073 | VH2 | Val X1 Trp Tyr X2 Gly X3 X4 X5 X6 X7 X8 Asp Ser Val X9 Gly, wherein X1 = I or V or a conservative substitution thereof, X2 = G or D or a conservative substitution thereof, X3 = S or N or a conservative substitution thereof, X4 = N or D or S or a conservative substitution thereof, X5 = K or T or a conservative substitution thereof, X6 = D or Y or S or a conservative substitution thereof, X7 = Y or F or a conservative substitution thereof, X8 = A or V or a conservative substitution thereof, X9 = K or R or T or a conservative substitution thereof. |
| | SEQ ID NO: 50074 | VH3 | Asp Arg X1 X2 Cys Ser Gly X3 X4 Cys Pro Tyr Tyr Tyr Tyr Tyr Gly Met Asp Val, wherein X1 = D or V or a conservative substitution thereof, X2 = Y or F or a conservative substitution thereof, X3 = G or T or a conservative substitution thereof, X4 = S or T or N or a conservative substitution thereof. |

**FIGURE 55**

| | | | |
|---|---|---|---|
| VH-CONSENSUS-43 TABLE 77 | SEQ ID NO: 50075 | VH1 | Arg Ser Ser Tyr Tyr Trp Gly. |
| | SEQ ID NO: 50076 | VH2 | Asn Ile Tyr Tyr X1 Gly X2 X3 Tyr X4 Asn Pro Ser X5 Lys X6, wherein X1 = S or G or a conservative substitution thereof, X2 = S or T or N or a conservative substitution thereof, X3 = T or A or a conservative substitution thereof, X4 = Y or N or D or H or T or a conservative substitution thereof, X5 = L or V or a conservative substitution thereof, X6 = S or G or a conservative substitution thereof. |
| | SEQ ID NO: 50077 | VH3 | His Gly Lys Asp Trp Gly Leu Asp X1, wherein X1 = Y or F or N or a conservative substitution thereof. |
| | | | |
| VH-CONSENSUS-44 TABLE 78 | SEQ ID NO: 50078 | VH1 | Gly Tyr Tyr X1 His, wherein X1 = M or I or a conservative substitution thereof. |
| | SEQ ID NO: 50079 | VH2 | Trp Ile X1 Pro X2 Ser Gly Gly Thr Asn X3 X4 Gln Lys Phe Gln Gly, wherein X1 = K or N or a conservative substitution thereof, X2 = N or K or a conservative substitution thereof, X3 = Q or S or H or N or Y or a conservative substitution thereof, X4 = A or V or a conservative substitution thereof. |
| | SEQ ID NO: 50080 | VH3 | Ala Pro Gly X1 X2 X3 X4 Gly X5 Trp Gly X6 Phe Asp Tyr, wherein X1 = T or K or I or a conservative substitution thereof, X2 = A or V or a conservative substitution thereof, X3 = A or P or a conservative substitution thereof, X4 = A or T or a conservative substitution thereof, X5 = T or S or a conservative substitution thereof, X6 = Y or F or C or a conservative substitution thereof. |
| | | | |
| VH-CONSENSUS-45 TABLE 79 | SEQ ID NO: 50081 | VH1 | Thr X1 Gly Val Gly Val Gly, wherein X1 = G or S or a conservative substitution thereof. |
| | SEQ ID NO: 50082 | VH2 | X1 Ile Tyr Trp X2 Asp Asp X3 Arg Tyr Ser Pro Ser Leu X4 Ser, wherein X1 = L or F or a conservative substitution thereof, X2 = D or H or N or K or S or a conservative substitution thereof, X3 = K or E or a conservative substitution thereof, X4 = K or R or a conservative substitution thereof. |
| | SEQ ID NO: 50083 | VH3 | X1 X2 Ala Val X3 X4 Asp Tyr, wherein X1 = L or I or A or a conservative substitution thereof, X2 = I or V or A or a conservative substitution thereof, X3 = A or S or a conservative substitution thereof, X4 = F or C or a conservative substitution thereof. |
| | | | |
| VH-CONSENSUS-46 TABLE 80 | SEQ ID NO: | VH1 | X1 Tyr Gly Met His () wherein X1 = S or N or T or a conservative substitution thereof. |

**FIGURE 55**

| | | | |
|---|---|---|---|
| | SEQ ID NO: 50085 | VH2 | X1 Ile Trp Tyr Asp Gly Ser X2 Lys Tyr Tyr X3 Asp Ser Val Lys Gly, wherein X1 = I or V or a conservative substitution thereof, X2 = Y or N or a conservative substitution thereof, X3 = A or V or a conservative substitution thereof. |
| | SEQ ID NO: 50086 | VH3 | Glu X1 Tyr Asp Phe Trp Ser Gly X2 X3 X4 X5, wherein X1 = A or G or R or N or a conservative substitution thereof, X2 = Y or F or H or a conservative substitution thereof, X3 = F or L or Y or W or a conservative substitution thereof, X4 = D or G or a conservative substitution thereof, X5 = Y or S or a conservative substitution thereof. |
| | | | |
| VH-CONSENSUS-47 TABLE 81 | SEQ ID NO: 50087 | VH1 | Ser Tyr Gly X1 His, wherein X1 = M or L or a conservative substitution thereof. |
| | SEQ ID NO: 50088 | VH2 | Val Ile Trp Tyr Asp Gly Ser Asn Lys X1 Tyr X2 Asp Ser Val Lys Gly, wherein X1 = Y or N or a conservative substitution thereof, X2 = A or E or a conservative substitution thereof. |
| | SEQ ID NO: 50089 | VH3 | X1 X2 X3 Tyr X4 X5 X6 X7 X8 X9 X10 X11 X12 Gly Met Asp Val, wherein X1 = D or W or a conservative substitution thereof, X2 = R or G or Y or a conservative substitution thereof, X3 = D or S or Y or a conservative substitution thereof, X4 = G or Y or a conservative substitution thereof, X5 = D or Y or a conservative substitution thereof, X6 = Absent or P or a conservative substitution thereof, X7 = Absent or P or a conservative substitution thereof, X8 = Absent or Y or a conservative substitution thereof, X9 = Absent or Y or a conservative substitution thereof, X10 = Absent or Y or a conservative substitution thereof, X11 = Absent or Y or a conservative substitution thereof, X12 = Y or D or a conservative substitution thereof. |
| | | | |
| VH-CONSENSUS-48 TABLE 82 | | VH1 | X1 X2 X3 Met X4 (SEQ ID NO: 50090) wherein X1 = S or N or T or a conservative substitution thereof, X2 = Y or S or a conservative substitution thereof, X3 = A or V or a conservative substitution thereof, X4 = S or T or N or a conservative substitution thereof. |
| | | VH2 | X1 X2 X3 Gly X4 Gly X5 X6 Thr X7 X8 X9 Asp Ser Val X10 Gly (SEQ ID NO: 50091) wherein X1 = A or G or a conservative substitution thereof, X2 = I or S or V or a conservative substitution thereof, X3 = S or V or a conservative substitution thereof, X4 = S or R or a conservative substitution thereof, X5 = G or A or S or V or a conservative substitution thereof, X6 = N or R or K or a conservative substitution thereof, X7 = F or Y or a |

**FIGURE 55**

| | | | |
|---|---|---|---|
| | | | conservative substitution thereof, X9 = A or T or a conservative substitution thereof, X10 = K or T or a conservative substitution thereof. |
| | | VH3 | X1 X2 X3 X4 X5 X6 X7 X8 Gly X9 Asp Val (SEQ ID NO: 50092) wherein X1 = L or D or E or a conservative substitution thereof, X2 = G or R or a conservative substitution thereof, X3 = K or G or I or a conservative substitution thereof, X4 = D or Q or Y or a conservative substitution thereof, X5 = Y or W or a conservative substitution thereof, X6 = Y or H or L or a conservative substitution thereof, X7 = Y or Absent or I or L or a conservative substitution thereof, X8 = Y or G or a conservative substitution thereof, X9 = M or V or a conservative substitution thereof. |
| | | | |
| VH-CONSENSUS-49 TABLE 83 | | VH1 | Ser Tyr X1 Met X2 (SEQ ID NO: 50093) wherein X1 = V or A or a conservative substitution thereof, X2 = S or N or a conservative substitution thereof. |
| | | VH2 | X1 X2 Ser Gly Ser Gly X3 X4 Thr X5 Tyr Ala Asp Ser Val X6 X7 (SEQ ID NO: 50094) wherein X1 = A or G or T or S or a conservative substitution thereof, X2 = M or I or T or a conservative substitution thereof, X3 = G or N or V or a conservative substitution thereof, X4 = R or N or W or a conservative substitution thereof, X5 = Y or F or N or a conservative substitution thereof, X6 = K or N or a conservative substitution thereof, X7 = G or D or a conservative substitution thereof. |
| | | VH3 | X1 X2 X3 X4 X5 X6 X7 X8 X9 X10 X11 X12 (SEQ ID NO: 50095) wherein X1 = L or V or Y or F or a conservative substitution thereof, X2 = T or E or F or G or a conservative substitution thereof, X3 = A or G or L or W or Absent or F or a conservative substitution thereof, X4 = Absent or G or M or a conservative substitution thereof, X5 = Absent or V or a conservative substitution thereof, X6 = Absent or G or a conservative substitution thereof, X7 = Absent or A or a conservative substitution thereof, X8 = Absent or G or a conservative substitution thereof, X9 = Absent or I or F or a conservative substitution thereof, X10 = F or N or Absent or a conservative substitution thereof, X11 = D or G or I or a conservative substitution thereof, X12 = Y or D or a conservative substitution thereof. |
| | | | |
| VH-CONSENSUS-50 TABLE 84 | | VH1 | X1 Tyr Gly X2 His (SEQ ID NO: 50096) wherein X1 = S or Y or a conservative substitution thereof, X2 = M or L or a conservative substitution thereof. |
| | | VH2 | X1 Ile Ser Tyr X2 Gly X3 Asn X4 X5 X6 Ala Asp Ser Val Lys Gly (SEQ ID NO: 50097) wherein X1 = I or V or a conservative substitution thereof, X2 = A or G or D or S or V or a conservative substitution thereof, X3 = S or I or N or |

**FIGURE 55**

| | | | |
|---|---|---|---|
| | | | or Q or a conservative substitution thereof, X5 = Y or S or D or H or a conservative substitution thereof, X6 = Y or S or a conservative substitution thereof. |
| | | VH3 | X1 X2 X3 X4 X5 X6 X7 X8 X9 X10 X11 X12 X13 X14 X15 Tyr Gly Met Asp Val (SEQ ID NO: 50098) wherein X1 = R or E or a conservative substitution thereof, X2 = G or D or a conservative substitution thereof, X3 = Y or R or a conservative substitution thereof, X4 = S or Y or a conservative substitution thereof, X5 = Y or C or a conservative substitution thereof, X6 = G or S or a conservative substitution thereof, X7 = Absent or G or a conservative substitution thereof, X8 = Absent or T or a conservative substitution thereof, X9 = Absent or S or a conservative substitution thereof, X10 = Absent or C or a conservative substitution thereof, X11 = Absent or P or a conservative substitution thereof, X12 = Absent or Y or a conservative substitution thereof, X13 = Absent or Y or a conservative substitution thereof, X14 = Absent or Y or a conservative substitution thereof, X15 = G or Y or a conservative substitution thereof. |
| | | | |
| VH-CONSENSUS-51 TABLE 85 | | VH1 | X1 X2 X3 Met X4 (SEQ ID NO: 50099) wherein X1 = D or S or a conservative substitution thereof, X2 = Y or C or a conservative substitution thereof, X3 = V or G or a conservative substitution thereof, X4 = H or Q or a conservative substitution thereof. |
| | | VH2 | X1 Ile Trp Tyr Asp Gly Ser Asn Lys Tyr Tyr Ala Asp Ser Val Lys X2 (SEQ ID NO: 50100) wherein X1 = V or I or a conservative substitution thereof, X2 = G or V or a conservative substitution thereof. |
| | | VH3 | Glu X1 Tyr X2 Ser Gly Trp X3 Asp Tyr Gly X4 Asp Val (SEQ ID NO: 50101) wherein X1 = P or R or a conservative substitution thereof, X2 = T or N or a conservative substitution thereof, X3 = Y or H or a conservative substitution thereof, X4 = M or L or a conservative substitution thereof. |
| | | | |
| VH-CONSENSUS-52 TABLE 86 | | VH1 | Ser Tyr Gly X1 Ser (SEQ ID NO: 50102) wherein X4 = I or F or V or a conservative substitution thereof. |
| | | VH2 | Trp Ile Ser Ala Tyr Asn Gly Asn X1 Lys X2 Ala Gln X3 X4 Gln Gly (SEQ ID NO: 50103) wherein X1 = T or R or a conservative substitution thereof, X2 = Y or N or E or F or a conservative substitution thereof, X3 = K or R or a conservative substitution thereof, X4 = L or F or a conservative substitution thereof. |
| | | VH3 | His Asp Phe Trp Ser Gly Tyr Tyr Lys Gly Met Asp Val (SEQ ID NO: 50227). |
| | | | |

**FIGURE 55**

| | | | |
|---|---|---|---|
| VH-CONSENSUS-53 TABLE 87 | | VH1 | X1 Ser X2 Ala Met Ser (SEQ ID NO: 50104) wherein X1 = S or R or a conservative substitution thereof, X2 = Y or S or a conservative substitution thereof. |
| | | VH2 | X1 Ile Ser Gly X2 Gly X3 X4 Thr Phe X5 X6 Asp Ser Val Lys Gly (SEQ ID NO: 50105) wherein X1 = A or V or S or a conservative substitution thereof, X2 = R or S or a conservative substitution thereof, X3 = G or I or V or a conservative substitution thereof, X4 = N or S or a conservative substitution thereof, X5 = D or Y or a conservative substitution thereof, X6 = A or T or a conservative substitution thereof. |
| | | VH3 | X1 X2 Ser X3 X4 X5 Phe Asp Tyr (SEQ ID NO: 50106) wherein X1 = E or S or a conservative substitution thereof, X2 = R or N or a conservative substitution thereof, X3 = G or S or a conservative substitution thereof, X4 = S or G or a conservative substitution thereof, X5 = Y or W or a conservative substitution thereof. |
| | | | |
| VH-CONSENSUS-54 TABLE 88 | | VH1 | X1 Tyr X2 Met His (SEQ ID NO: 50107) wherein X1 = S or N or Y or H or a conservative substitution thereof, X2 = V or G or a conservative substitution thereof. |
| | | VH2 | X1 Ile Trp X2 Asp Gly X3 X4 X5 Tyr Tyr X6 Asp Ser Val Lys Gly (SEQ ID NO: 50108) wherein X1 = V or L or a conservative substitution thereof, X2 = H or Y or a conservative substitution thereof, X3 = S or T or a conservative substitution thereof, X4 = N or D or a conservative substitution thereof, X5 = K or A or a conservative substitution thereof, X6 = A or V or G or a conservative substitution thereof. |
| | | VH3 | Glu Asn Ser Ser Ser X1 Tyr Phe Asp Tyr Trp Gly Gln Gly Thr Leu Val Thr Val Ser Ser (SEQ ID NO: 50109) wherein X1 = Y or F or a conservative substitution thereof. |
| | | | |
| VH-CONSENSUS-55 TABLE 89 | | VH1 | Thr Ser Gly Val Gly Val Gly (SEQ ID NO: 50110). |
| | | VH2 | Leu Ile Asn Trp Asn Asp Asp Lys Arg X1 Ser Pro Ser Leu Lys Ser (SEQ ID NO: 50111) wherein X1 = Y or F or a conservative substitution thereof. |
| | | VH3 | Lys X1 Thr Trp Val Ala Phe Asp Ile (SEQ ID NO: 50112) wherein X1 = A or T or a conservative substitution thereof. |
| | | | |
| VH-CONSENSUS-56 TABLE 90 | | VH1 | Ser Tyr X1 X2 X3 (SEQ ID NO: 50113) wherein X1 = V or A or a conservative substitution thereof, X2 = M or I or L or a conservative substitution thereof, X3 = N or S or R or a conservative substitution thereof. |
| | | VH2 | X1 X2 Ser Gly Ser Gly X3 X4 Thr Tyr Tyr X5 Asp Ser Val Lys Gly (SEQ ID NO: 50114) wherein X1 = A or D or a conservative substitution thereof, X2 = I or M or a conservative substitution thereof, X3 = G or D or V or a conservative substitution thereof, X4 = R or S or F or T or a |

FIGURE 55

| | | | |
|---|---|---|---|
| | | | conservative substitution thereof, X5 = A or V or a conservative substitution thereof. |
| | | VH3 | Thr X1 X2 X3 X4 X5 (SEQ ID NO: 50115) wherein X1 = A or G or S or T or Y or a conservative substitution thereof, X2 = T or V or Absent or H or L or G or a conservative substitution thereof, X3 = F or Absent or K or a conservative substitution thereof, X4 = D or Absent or a conservative substitution thereof, X5 = Y or L or a conservative substitution thereof. |
| | | | |
| VH-CONSENSUS-57 TABLE 91 | | VH1 | Ser X1 Ala Met X2 (SEQ ID NO: 50116) wherein X1 = Y or F or a conservative substitution thereof, X2 = S or N or a conservative substitution thereof. |
| | | VH2 | X1 X2 Ser Gly X3 Gly X4 X5 Thr X6 Tyr Ala Asp Ser Val Lys Gly (SEQ ID NO: 50117) wherein X1 = V or A or I or a conservative substitution thereof, X2 = I or L or a conservative substitution thereof, X3 = R or S or G or a conservative substitution thereof, X4 = G or S or K or a conservative substitution thereof, X5 = T or N or S or a conservative substitution thereof, X6 = F or Y or a conservative substitution thereof. |
| | | VH3 | Lys Arg Thr X1 X2 Asp X3 Phe Asp X4 (SEQ ID NO: 50118) wherein X1 = P or G or a conservative substitution thereof, X2 = S or D or E or a conservative substitution thereof, X3 = V or A or a conservative substitution thereof, X4 = I or V or a conservative substitution thereof. |
| | | | |
| VH-CONSENSUS-58 TABLE 92 | | VH1 | X1 X2 X3 Met His (SEQ ID NO: 50237) wherein X1 = S or N or T or a conservative substitution thereof, X2 = Y or F or a conservative substitution thereof, X3 = G or D or N or a conservative substitution thereof. |
| | | VH2 | X1 Ile Trp X2 Asp Gly X3 X4 X5 Tyr X6 X7 Asp Ser Val Lys Gly (SEQ ID NO: 50238) wherein X1 = V or A or a conservative substitution thereof, X2 = Y or H or a conservative substitution thereof, X3 = S or R or a conservative substitution thereof, X4 = N or D or H or a conservative substitution thereof, X5 = K or R or a conservative substitution thereof, X6 = Y or C or S or a conservative substitution thereof, X7 = A or E or T or a conservative substitution thereof. |
| | | VH3 | Asp X1 X2 X3 X4 X5 X6 X7 X8 X9 Asp X10 (SEQ ID NO: 50239) wherein X1 = R or D or H or a conservative substitution thereof, X2 = P or S or A or a conservative substitution thereof, X3 = I or R or Y or a conservative substitution thereof, X4 = S or L or V or W or a conservative substitution thereof, X5 = Absent or G or a conservative substitution thereof, X6 = Absent or A or a conservative substitution thereof, X7 = S or T or A or a conservative substitution thereof, X8 = A or F or S or Y or a |

**FIGURE 55**

| | | | |
|---|---|---|---|
| | | | conservative substitution thereof, X10 = Y or F or a conservative substitution thereof. |
| | | | |
| VH-CONSENSUS-59 TABLE 93 | | VH1 | X1 Tyr X2 Met Asn (SEQ ID NO: 50240) weherein X1 = S or N or a conservative substitution thereof, X2 = N or S or a conservative substitution thereof. |
| | | VH2 | Tyr Ile Ser X1 Ser X2 X3 Thr X4 X5 Tyr X6 Asp Ser Val X7 Gly (SEQ ID NO: 50241) wherein X1 = R or S or a conservative substitution thereof, X2 = S or G or a conservative substitution thereof, X3 = N or S or a conservative substitution thereof, X4 = K or T or a conservative substitution thereof, X5 = Y or H or a conservative substitution thereof, X6 = A or V or a conservative substitution thereof, X7 = K or R or E or Q or a conservative substitution thereof. |
| | | VH3 | Asp X1 X2 X3 X4 X5 X6 X7 X8 X9 Tyr Tyr Gly X10 Asp Val (SEQ ID NO: 50242) wherein X1 = R or S or a conservative substitution thereof, X2 = S or R or a conservative substitution thereof, X3 = G or K or a conservative substitution thereof, X4 = S or G or a conservative substitution thereof, X5 = Y or F or a conservative substitution thereof, X6 = G or Absent or a conservative substitution thereof, X7 = Y or Absent or a conservative substitution thereof, X8 = F or Absent or a conservative substitution thereof, X9 = Y or Absent or a conservative substitution thereof, X10 = L or M or a conservative substitution thereof. |
| | | | |
| VH-CONSENSUS-60 TABLE 94 | | VH1 | Ser Gly Gly Asp Tyr Trp Ser (SEQ ID NO: 50119). |
| | | VH2 | Tyr Ile Tyr Tyr Ser Gly X1 Thr Tyr Tyr Asn Pro Ser Leu Lys Ser (SEQ ID NO: 50120) wherein X1 = S or I or P or a conservative substitution thereof. |
| | | VH3 | Asp X1 X2 X3 X4 Gly Met Asp Val (SEQ ID NO: 50121) wherein X1 = S or G or H or a conservative substitution thereof, X2 = S or A or a conservative substitution thereof, X3 = S or L or R or a conservative substitution thereof, X4 = Y or R or H or a conservative substitution thereof. |
| | | | |

**FIGURE 55**

Table 20A

**VARIABLE LIGHT CDR CONSENSUS SEQUENCES I**

| NAME (ORIGINAL AND PATENT) | Patent SEQ ID NO.: | SEQUENCE |
|---|---|---|
| VL-Consensus-1 (Table 35) (generated from 13 light chain sequences) | SEQ ID NO: 50366 | DIQMTQSPSSVSASVGDRVTITCRASQGISRWLAWYQQKPGKAPKLLIYAASSLQSGVPSRFSGSGSGTDFTLTISSLQPEDFATYYCQQANSFPFTFGPGTKVDIKR |
| | | |
| VL-Consensus-2 (Table 36) (generated from 11 light chain sequences) | SEQ ID NO: 50367 | SYELTQPPSVSVSPGQTASITCSGDKLGDKYAYWYQQKPGQSPVLVIYQDRKRPSGIPERFSGSNSGNTATLTISGTQAMDEADYYCQAWDNSTVVFGGGTKLTVLGG |
| | | |
| VL-Consensus-3 (Table 37) (generated from 15 light chain sequences) | SEQ ID NO: 50368 | DIVMTQSPDSLAVSLGERATINCKSSQSVLYSSNNNNYLAWYQQKPGQPPKLLIYWASTRESGVPDRFSGSGSGTDFTLTISSLQAEDVAVYYCQQYYSTPPTFGQGTKVEIKR |
| | | |
| VL-Consensus-4 (Table 38) (generated from 23 light chain sequences) | SEQ ID NO: 50369 | DIQMTQSPSSLSASVGDRVTITCRASQGISNYLAWFQQKPGKAPKSLIYAASSLQSGVPSKFSGSGSGTDFTLTISSLQPEDFATYYCQQYNSYPFTFGPGTKVDIKR |
| | | |
| VL-Consensus 5 (Table 39) (generated from 17 light chain sequences) | SEQ ID NO: 50370 | DIQMTQSPSSLSASVGDRVTITCRASQGIRNNLGWYQQKPGKAPKRLIYAASSLQSGVPSRFSGSGSGTEFTLTISSLQPEDFATYYCLQYNSYPFTFGQGTKVEIKR |
| | | |
| VL-Consensus 6 (Table 40) (generated from 11 light chain sequences) | SEQ ID NO: 50371 | DIQMTQSPSSLSASVGDRVTITCRASQGISNYLAWFQQKPGKAPKSLISAASSLQSGVPSKFSGSGSGTDFTLTISSLQPEDFATYYCQQYNSYPFTFGPGTKVDIKR |
| | | |
| VL-Consensus 7 (kappa) (Table 41) (generated from 26 light chain sequences) | SEQ ID NO: 50372 | DIQMTQSPSSLSASVGDRVTITCRASQDIRSDLGWYQQKPGKAPKRLIYAASSLQSGVPSRFSGSGSGTEFTLTISSLQPEDFATYYCLQHTIYPPTFGGGTKVEIKR |
| | | |
| VL-Consensus-7 (lambda) (Table 41) (generated from 26 light chain sequences) | SEQ ID NO: 50373 | QSXLTQPXSXSGSPGQSITISCTGTSSDVGXXNXVSWYQQHPGKAPKLMIYEVSNRPSGVXXRFSGSKSGNTASLTISGLQXEDEADYYCSSYTXSXTVVFGGGTKLTVLG |
| | | |
| VL-Consensus-8 (Table 42) (generated from 25 light chain | SEQ ID NO: 50374 | DIQMTQSPSSLSASVGDRVTITCRASQSISSYLNWYQQKPGKAPKLLIYAASSLQSGVPSRFSGSGSGTDFTLTISSLQPEDFATYYCQQSYRTPLFTFGPGTKVDIKR |

**FIGURE 55**

| | | |
|---|---|---|
| VL-Consensus-9 (Table 43) (generated from 14 light chain sequences) | SEQ ID NO: 50375 | DIQMTQSPSSLSASVGDRVTITCRASQSIISYLNWYQQKPGKAPKLLIYTASSLQSGVPSRFSGSGSGTDFTLTISSLQPEDFATYYCQQTYSTPLTFGGGTKVEIKR |
| | | |
| VL-Consensus-10 (Table 44) (generated from 22 light chain sequences) | SEQ ID NO: 50376 | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPRTFGQGTKVEIKR |
| | | |
| VL-Consensus 11 (Table 45) (generated from 16 light chain sequences) | SEQ ID NO: 50377 | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPFTFGXGTKVEIKR |
| | | |
| VL-Consensus 12 (Table 46) (generated from 71 light chain sequences) | SEQ ID NO: 50378 | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPLTFGGGTKVEIKR |
| | | |
| VL-Consensus 13 (Table 47) (generated from 21 light chain sequences) | SEQ ID NO: 50379 | DIQMTQSPSSLSASVGDRVTITCRASQGIRNDLGWYQQKPGKAPKRLIYAASSLQSGVPSRFSGSGSGTEFTLTISSLQPEDFATYYCLQHSSYPLTFGGGTKVEIKR |
| | | |
| VL-Consensus 14 (Table 48) (generated from 13 light chain sequences) | SEQ ID NO: 50380 | DIQMTQSPSSLSASVGDRVTITCRASQGIRNDLGWYQQKPGKAPKRLIYAASSLQSGVPSRFSGSGSGTEFTLTISSLQPEDFATYYCLQHSSYPLTFGGGTKVEIKR |
| | | |
| VL-Consensus 15 (Table 95) (generated from 209 light chain sequences) | SEQ ID NO: 50312 | DIQMTQSPSSLSASVGDRVTITCRASQGIRDLGWYQQKPGKAPKRLIYAASSLQSGVPSRFSGSGSGTEFTLTISSLQPEDFATYYCLQHNSYPLTFGGGTKVEIKR |
| | | |
| VL-Consensus 16 (Table 96) (generated from 174 light chain sequences) | SEQ ID NO: 50313 | SYELTQPPSVSVSPGQTASITCSGDKLGDKYACWYQQKPGQSPVLVIYQDRKRPSGIPERFSGSNSGNTATLTISGTQAMDEADYYCQAWDSSTVVFGGGTKLTVLG |
| | | |
| VL-Consensus 17 (Table 97) (generated from 162 light chain sequences) | SEQ ID NO: 50314 | EIVLTQSPGTLSLSPGERATLSCRASQSVYSSYLAWYQQKPGQAPRLLIYGASSRATGIPDRFSGSGSGTDFTLTISRLEPEDFAVYYCQQYDNSPWTFGQGTKVEIKR |
| | | |
| VL-Consensus 18 (Table 98) (generated from 147 light chain | SEQ ID NO: 50315 | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPRTFGQGTKVEIKR |

**FIGURE 55**

| | | |
|---|---|---|
| VL-Consensus 19 (Table 99) (generated from 132 light chain sequences) | SEQ ID NO: 50316 | DIVMTQSPDSLAVSLGERATINCKSSQSVLHSSNNNNYLAWYQQKPGQPPKLLIYWASTRESGVPDRFSGSGSGTDFTLTISSLQAEDVAVYYCQQYYSTPPTFGQGTKVEIKR |
| VL-Consensus 20 (Table 100) (generated from 109 light chain sequences) | SEQ ID NO: 50317 | DIQMTQSPSSLSASVGDRVTITCRASQGIRNDLGWYQQKPGKAPKRLIYAASSLQSGVPSRFSGSGSGEFTLTISSLQPEDFATYYCLQHNSYPFTFGPGTKVDIKR |
| VL-Consensus 21 (Table 101) (generated from 92 light chain sequences) | SEQ ID NO: 50318 | DIQMTQSPSSLSASVGDRVTITCRASQGISNYLAWFQQKPGKAPKSLIYAASSLQSGVPSKFSGSGSGTDFTLTISSLQPEDFATYYCQQYSTYPLTFGGGTKVEIKR |
| VL-Consensus 22 (Table 102) (generated from 89 light chain sequences) | SEQ ID NO: 50319 | DIQMTQSPSSLSASVGDRVTITCRASQNIISYLNWYQQKPGKAPKLLIYTASSLQSGVPSRFSGSGSGTDFTLTISSLQPEDFATYYCQQSYSTPLTFGGGTKVEIKR |
| VL-Consensus 23 (Table 103) (generated from 86 light chain sequences) | SEQ ID NO: 50320 | DIQMTQSPSSLSASVGDRVTITCRASQGISNYLAWFQQKPGKAPKSLIYAASSLQSGVPSKFSGSGSGTDFTLTISSLQPEDFATYYCQQYNSYPFTFGPGTKVDIKR |
| VL-Consensus 24 (Table 104) (generated from 81 light chain sequences) | SEQ ID NO: 50321 | SYELTQPPSVSVSPGQTASITCSGDKLGDKYACWYQQKPGQSPVLVIYQDRKRPSGIPERFSGSNSGNTATLTISGTQAMDEADYYCQAWDSSTVVFGGGTKLTVLG |
| VL-Consensus 25 (Table 105) (generated from 65 light chain sequences) | SEQ ID NO: 50322 | DIVMTQTPLSLSVTPGQPASISCKSSQSLLHGD-GKTYLYWYLQKPGQPPQLLIYEVSNRFSGVPDRFSGSGSGTDFTLKISRVEAEDVGVYYCMQSIQLPWTFGQGTKVEIKR |
| VL-Consensus 26 (Table 106) (generated from 58 light chain sequences) | SEQ ID NO: 50323 | DIQMTQSPSSLSASVGDRVTITCRASQSISSYLNWYQQKPGKAPKLLIYAASSLQSGVPSRFSGSGSGTDFTLTISSLQPEDFATYYCQQSYSPPFTFGPGTKVDIKR |
| VL-Consensus 27 (Table 107) (generated from 47 light chain sequences) | SEQ ID NO: 50324 | DIVMTQSPDSLAVSLGERATINCKSSQSVLYSSNNNNYLAWYQQKPGQPPKLLIYWASTRESGVPDRFSGSGSGTDFTLTISSLQAEDVAVYYCQQYYSTPCSFGQGTKLEIKR |
| VL-Consensus 28 (Table 108) (generated from 42 light chain | SEQ ID NO: 50325 | DIQMTQSPSSVSASVGDRVTITCRASQGISNWLAWYQQKPGKAPKLLIYAASSLQSGVPSRFSGSGSGTDFTLTISSLQPEDFATYYCQQAFPWTFGQGTKVEIKR |

FIGURE 55

| | | |
|---|---|---|
| VL-Consensus 29 (Table 109) (generated from 37 light chain sequences) | SEQ ID NO: 50326 | DIVMTQSPDSLAVSLGERATINCKSSQSVLHSSNNNNYLAWYQQKPGQPPKLLIYWASTRESGVPDRFSGSGSGTDFTLTISSLQAEDVAVYYCQQYYSTPVTFGPGTKVDIKR |
| | | |
| VL-Consensus 30 (Table 110) (generated from 31 light chain sequences) | SEQ ID NO: 50327 | QTVVTQEPSLTVSPGGTVTLTCASSTGAVTSG-YYPNWFQQKPGQAPRALIYSTSNKHSWTPARFSGSLLGGKAALTLSGVQPEDEAEYYCLLYYGGAQLVFGGGTKLTVLG |
| | | |
| VL-Consensus 31 (Table 111) (generated from 25 light chain sequences) | SEQ ID NO: 50328 | EIVMTQSPATLSVSPGERATLSCRASQSVNSNLAWYQQKPGQAPRLLIYGASTRATGIPARFSGSGSGTEFTLTISSLQSEDFAVYYCQQYNDWPCSFGQGTKLEIKR |
| | | |
| VL-Consensus 32 (Table 112) (generated from 24 light chain sequences) | SEQ ID NO: 50329 | EFMLTQSPGTLSLSPGERATLSCRASQSVSSSYLVWYQQKPGQAPRLLIYGASTRATGIPDRFSGSGSGTDFTLTISRLEPEYFAVYYCQQYGCSPLTFGGGTKVEITR |
| | | |
| VL-Consensus-33 (Table 113) (generated from 21 light chain sequences) | SEQ ID NO: 50330 | DIVMTQTPLSLSVTPGQPASISCKSSQSLLHSEGKTYLYWYLQKPGQPPQLLIYEVSNRFSGVPDRFSGSGSGTDFTLKISRVEAEDVGVYYCMQSIQLPLTFGGGTKVEIKR |
| | | |
| VL-Consensus-34 (Table 114) (generated from 18 light chain sequences) | SEQ ID NO: 50331 | QSALTQPASVSGSPGQSITISCTGTSSDVGGYNYVSWYQQHPGKAPKLMIYEVSNRPSGVSNRFSGSKSGNTASLTISGLQAEDEADYYCNSYTRSITWVFGGGTKLTVLG |
| | | |
| VL-Consensus 35 (Table 115) (generated from 17 light chain sequences) | SEQ ID NO: 50332 | DIQMTQSPSSLSASVGDRVTITCRASQSISNYLNWYQQKPGKAPKLLIYAASSLQSGVPSRFSGSGSGTDFTLTISSLQPEDFATYYCQQSYSTPTWTFGQGTKVEIKR |
| | | |
| VL-Consensus 36 (Table 116) (generated from 16 light chain sequences) | SEQ ID NO: 50333 | DIQMTQSPSSVSASVGDRVTITCRASQGISSWLAWYQQKPGKAPKLLIYAASSLQSGVPSRFSGSGSGTDFTLTISSLQPEDFATYYCQQINSFPLTFGGGTKVEIKR |
| | | |
| VL-Consensus 37 (Table 117) (generated from 16 light chain sequences) | SEQ ID NO: 50334 | DIQMTQSPSSLSASVGDRVTITCQASQDINNYLNWYQQKPGKAPKLLIYDASNLETGVPSRFSGSGSGTDFTFTISSLQPEDIATYYCQQYDNLPITFGQGTRLEIKR |
| | | |
| VL-Consensus 38 (Table 118) (generated from 16 light chain | SEQ ID NO: 50335 | QSVLTQPPSVSAAPGQKVTISCSGSSSNIGNNYVSWYQQLPGTAPKLLIYDNNKRPSGIPDRFSGSKSGTSATLGITGLQTGDEADYYCGTWDSSLSVGVFGGGTKLTV |

FIGURE 55

| | | |
|---|---|---|
| VL-Consensus 39 (Table 119) (generated from 14 light chain sequences) | SEQ ID NO: 50336 | DIVMTQSPLSLPVTPGEPASISCRSSQSLLHSNGYNYLDWYLQKPGQSPQLLIYLGSNRASGVPDRFSGSGSGTDFTLKISRVEAEDVGVYYCMQALHPPLTFGGGTKVEIKR |
| VL-Consensus 40 (Table 120) (generated from 13 light chain sequences) | SEQ ID NO: 50337 | QSVLTQPPSASGTPGQRVTISCSGSSSNIGSNTVNWYQQLPGTAPKLLIYSNNQRPSGVPDRFSGSKSGTSASLAISGLQSEDEADYYCAAWDDSLNGVVFGGGTKLTVLG |
| VL-Consensus 41 (Table 121) (generated from 11 light chain sequences) | SEQ ID NO: 50338 | QSVLTQPPSASGTPGQRVTISCSGSSSNIGSNIVTWYQQLPGTAPKLLIYSNDQRPSGVPDRFSGSKSGTSASLAISGLQSEDEADYYCAAWDDSLNGWVFGGGTTLTVLG |
| VL-Consensus 42 (Table 122) (generated from 10 light chain sequences) | SEQ ID NO: 50339 | DIQMTQSPSSLSASVGDRVTITCRASQGIRNDLGWYQQKPGKAPKRLIYAASSLQSGVPSRFSGSGSGTEFTLTISSLQPEDFATYYCLQHNSYPITFGQGTRLEIKR |
| VL-Consensus 43 (Table 123) (generated from 10 light chain sequences) | SEQ ID NO: 50340 | DIQMTQSPSSLSASVGDRVTITCRASQSISSYLNWYQQKPGKAPKLLIYAASSLQSGVPSRFSGSGSGTDFTLTISSLQPEDFATYYCQQSYSIPITFGQGTRLEIKR |
| VL-Consensus 44 (Table 124) (generated from 10 light chain sequences) | SEQ ID NO: 50341 | DIVMTQTPLSLSVTPGQPASISCKSSQSLLHSEGKTYLYWYLQKPGQPPQLLIYEVSNRFSGVPDRFSGSGSGTDFTLKISRVEAEDVGVYYCMQSIQLPITFGQGTRLEIKR |
| VL-Consensus 45 (Table 125) (generated from 10 light chain sequences) | SEQ ID NO: 50342 | EIVLTQSPGTLSLFPGERATLSCRASQSVISSYLAWYQQKPGQAPRLLIFGVSSRATGIPDRFSGSGSGTDFTLTISRLEPEDFAVYYCQQYGRSPFNFGPGTKVDIKR |
| VL-Consensus 46 (Table 126) (generated from 10 light chain sequences) | SEQ ID NO: 50343 | EIVMTQSPATLSVSPGERATLSCRASQSVSSNLAWYQQKPGQAPRLLIYGASTRATGIPARFSGSGSGTEFTLTISSLQSEDFAVYYCQQYNDWPWTFGQGTKVEIKR |
| VL-Consensus 47 (Table 127) (generated from 10 light chain sequences) | SEQ ID NO: 50344 | EIVLTQSPDFQSVTPKEKVTITCRASQSIGSSLHWYQQKPDQSPKLLIKYASQSFSGVPSRFSGSGSGTDFTLTINSLEAEDAATYYCHQSSSLPWTFGQGTKVEIKR |
| VL-Consensus 48 (Table 128) (generated from 9 light chain | SEQ ID NO: 50345 | DIQMTQSPSSLSASVGDRVTITCRASQGIRNDLGWYQQKPGKAPKRLIYAASSLQSGVPSRFSGSGSGTEFTLTISSLQPEDFATYYCLQHYSYPRSFGQGTKLEIKR |

**FIGURE 55**

| | | |
|---|---|---|
| VL-Consensus 49 (Table 129) (generated from 9 light chain sequences) | SEQ ID NO: 50346 | DIQMTQSPSSLSASVGDRVTITCRASQGISNYLAWFQQKPGKAPKSLIYAASSLQSGVPSKFSGSGSGTDFTLTISSLQPEDFATYYCQQYLSYPITFGQGTRLEIKR |
| | | |
| VL-Consensus 50 (Table 130) (generated from 9 light sequences) | SEQ ID NO: 50347 | EIVLTQSPDFQSVTPKEKVTITCRASQSIGSSLHWYQQKPDQSPKLLIKYASQSFSGVPSRFSGSGSGTDFTLTINSLEAEDAATYYCHQSRRLPLTFGGGTKVEIKR |
| | | |
| VL-Consensus 51 (Table 131) (generated from 9 light chain sequences) | SEQ ID NO: 50348 | SSELTQDPAVSVALGQTVRITCQGDSLRPYYASWYQQKPGQAPVLVIYGKNNRPSGIPDRFSGSSSGNTASLTITGAQAEDEADYYCNSRDSSGNHLVVFGGGTKLTVLG |
| | | |
| VL-Consensus 52 (Table 132) (generated from 8 light chain sequences) | SEQ ID NO: 50349 | DVVMTQSPLSLPVTLGQPASISCRSSQSLVYSDGNTYLNWFQQRPGQSPRRLIYKVSNWDSGVPDRFSGSGSGTDFTLKISRVEAEDVGVYYCMQGTHWPLTFGGGTKVEIKR |
| | | |
| VL-Consensus 53 (Table 133) (generated from 8 light chain sequences) | SEQ ID NO: 50350 | EIVMTQSPATLSVSPGERATLSCRASQSVSRNLAWYQQKPGQAPRLLIYGASTRATGIPARFSGSGSGTEFTLTISSLQSEDFAVYYCQQYNNWPLTFGGGTKVEIKR |
| | | |
| VL-Consensus 54 (Table 134) (generated from 8 light chain sequences) | SEQ ID NO: 50351 | SYELTQPLSVSVALGQTARITCGGNNIGRKNVHWYQQKPGQAPVLVIYRDSDRPSGIPERFSGSNSGNTATLTISRAQAGDEADYYCQVWDSSTVVFGGGTKLTVLG |
| | | |

**FIGURE 55**

Table 20B

VARIABLE LIGHT CDR CONSENSUS SEQUENCES

| Name (original and patent) | Patent SEQ ID NO: | CDR | Sequence |
|---|---|---|---|
| VL-Consensus-1 (Table 35) | 50423 | VL1 | RASQGISRWLA |
| | 50424 | VL2 | AASSLQS |
| | 50425 | VL3 | QQANSFPFT |
| VL-Consensus-2 (Table 36) | 50426 | VL1 | SGDKLGDKYAY |
| | 50427 | VL2 | QDRKRPS |
| | 50428 | VL3 | QAWDNSTVV |
| VL-Consensus-3 (Table 37) | 50429 | VL1 | KSSQSVLYSSNNNNYLA |
| | 50430 | VL2 | WASTRES |
| | 50431 | VL3 | QQYYSTPPT |
| VL-Consensus-4 (Table 38) | 50432 | VL1 | RASQGISNYLA |
| | 50433 | VL2 | AASSLQS |
| | 50434 | VL3 | QQYNSYPFT |
| VL-Consensus-5 (Table 39) | 50435 | VL1 | RASQGIRNNLG |
| | 50436 | VL2 | AASSLQS |
| | 50437 | VL3 | LQYNSYPFT |
| VL-Consensus-6 (Table 40) | 50438 | VL1 | RASQGISNYLA |
| | 50439 | VL2 | AASSLQS |
| | 50440 | VL3 | QQYNSYPFT |
| VL-Consensus-7κ (Table 41) | 50441 | VL1 | RASQDIRSDLG |
| | 50442 | VL2 | AASSLQS |
| | 50443 | VL3 | LQHTIYPPT |
| VL-Consensus-7λ (Table 41) | 50444 | VL1 | TGTSSDVGXXNXVS |
| | 50445 | VL2 | EVSNRPS |
| | 50446 | VL3 | SSYTXSXTVV |
| VL-Consensus-8 (Table 42) | 50447 | VL1 | RASQSISSYLN |
| | 50448 | VL2 | AASSLQS |
| | 50449 | VL3 | QQSYRTPLFT |
| VL-Consensus-9 (Table 43) | 50450 | VL1 | RASQSIISYLN |
| | 50451 | VL2 | TASSLQS |
| | 50452 | VL3 | QQTYSTPLT |

FIGURE 55

| | | | |
|---|---|---|---|
| VL-Consensus-10 (Table 44) | 50453 | VL1 | RASQGIRNDLG |
| | 50454 | VL2 | AASSLQS |
| | 50455 | VL3 | LQHYSYPRT |
| VL-Consensus-11 (Table 45) | 50456 | VL1 | RASQGIRNDLG |
| | 50457 | VL2 | AASSLQS |
| | 50458 | VL3 | LQHNSYPFT |
| VL-Consensus-12 (Table 46) | 50459 | VL1 | RASQGIRNDLG |
| | 50460 | VL2 | AASSLQS |
| | 50461 | VL3 | LQHNSYPLT |
| VL-Consensus-13 (Table 47) | 50462 | VL1 | RASQGIRNDLG |
| | 50463 | VL2 | AASSLQS |
| | 50464 | VL3 | LQHSSYPLT |
| VL-Consensus-14 (Table 48) | 50465 | VL1 | RASQGIRNDLG |
| | 50466 | VL2 | AASSVQS |
| | 50467 | VL3 | LQHNSYPLT |
| VL-Consensus-15 (Table 95) | 50606 | VL1 | RASQGIRNDLG |
| | 50607 | VL2 | AASSLQS |
| | 50608 | VL3 | LQHNSYPLT |
| VL-Consensus-16 (Table 96) | 50609 | VL1 | SGDKLGDKYAC |
| | 50610 | VL2 | AASSLQS |
| | 50611 | VL3 | QAWDSSTVV |
| VL-Consensus-17 (Table 97) | 50612 | VL1 | RASQSVYSSYLA |
| | 50613 | VL2 | GASSRAT |
| | 50614 | VL3 | QQYDNSPWT |
| VL-Consensus-18 (Table 98) | 50615 | VL1 | RASQGIRNDLG |
| | 50616 | VL2 | AASSLQS |
| | 50617 | VL3 | LQHYSYPRT |
| VL-Consensus-19 (Table 99) | 50618 | VL1 | KSSQSVLHSSNNNNYLA |
| | 50619 | VL2 | WASTRES |
| | 50620 | VL3 | QQYYSTPPT |
| VL-Consensus-20 (Table 100) | 50621 | VL1 | RASQGIRNDLG |
| | 50622 | VL2 | AASSLQS |
| | 50623 | VL3 | LQHNSYPFT |
| VL-Consensus-21 (Table 101) | 50624 | VL1 | RASQGISNYLA |
| | 50625 | VL2 | AASSLQS |
| | 50626 | VL3 | QQYSTYPLT |

**FIGURE 55**

| | | | |
|---|---|---|---|
| VL-Consensus-22 (Table 102) | 50627 | VL1 | RASQNIISYLN |
| | 50628 | VL2 | TASSLQS |
| | 50629 | VL3 | QQSYSTPLT |
| | | | |
| VL-Consensus-23 (Table 103) | 50630 | VL1 | RASQGISNYLA |
| | 50631 | VL2 | AASSLQS |
| | 50632 | VL3 | QQYNSYPFT |
| | | | |
| VL-Consensus-24 (Table 104) | 50633 | VL1 | RASQGISRWLA |
| | 50634 | VL2 | AASSLQS |
| | 50635 | VL3 | QQANSFPFT |
| | | | |
| VL-Consensus-25 (Table 105) | 50636 | VL1 | KSSQSLLHGDGKTYLY |
| | 50637 | VL2 | EVSNRFS |
| | 50638 | VL3 | MQSIQLPWT |
| | | | |
| VL-Consensus-26 (Table 106) | 50639 | VL1 | RASQSISSYLN |
| | 50640 | VL2 | AASSLQS |
| | 50641 | VL3 | QQSYSPPFT |
| | | | |
| VL-Consensus-27 (Table 107) | 50642 | VL1 | KSSQSVLYSSNNNNYLA |
| | 50643 | VL2 | WASTRES |
| | 50644 | VL3 | QQYYSTPCS |
| | | | |
| VL-Consensus-28 (Table 108) | 50645 | VL1 | RASQGISNWLA |
| | 50646 | VL2 | AASSLQS |
| | 50647 | VL3 | QQANSFPWT |
| | | | |
| VL-Consensus-29 (Table 109) | 50648 | VL1 | KSSQSVLHSSNNNNYLA |
| | 50649 | VL2 | WASTRES |
| | 50650 | VL3 | QQYYSTPVT |
| VL-Consensus-30 (Table 110) | 50651 | VL1 | ASSTGAVTSGYYPN |
| | 50652 | VL2 | STSNKHS |
| | 50653 | VL3 | LLYYGGAQLV |
| | | | |
| VL-Consensus-31 (Table 111) | 50654 | VL1 | RASQSVNSNLA |
| | 50655 | VL2 | GASTRAT |
| | 50656 | VL3 | QQYNDWPCS |

**FIGURE 55**

| | | | |
|---|---|---|---|
| VL-Consensus-32 (Table 112) | 50657 | VL1 | RASQSVSSSYLV |
| | 50658 | VL2 | GASTRAT |
| | 50659 | VL3 | QQYGCSPLT |
| VL-Consensus-33 (Table 113) | 50660 | VL1 | KSSQSLLHSEGKTYLY |
| | 50661 | VL2 | EVSNRFS |
| | 50662 | VL3 | MQSIQLPLT |
| VL-Consensus-34 (Table 114) | 50663 | VL1 | TGTSSDVGGYNYVS |
| | 50664 | VL2 | EVSNRPS |
| | 50665 | VL3 | NSYTRSITWV |
| VL-Consensus-35 (Table 115) | 50666 | VL1 | RASQSISNYLN |
| | 50667 | VL2 | AASSLQS |
| | 50668 | VL3 | QQSYSTPTWT |
| VL-Consensus-36 (Table 116) | 50669 | VL1 | RASQGISSWLA |
| | 50670 | VL2 | AASSLQS |
| | 50671 | VL3 | QQ NSFPLT |
| VL-Consensus-37 (Table 117) | 50672 | VL1 | QASQDINNYLN |
| | 50673 | VL2 | DASNLET |
| | 50674 | VL3 | QQYDNLPIT |
| VL-Consensus-38 (Table 118) | 50675 | VL1 | SGSSSNIGNNYVS |
| | 50676 | VL2 | DNNKRP |
| | 50677 | VL3 | GTWDSSLSVGV |
| VL-Consensus-39 (Table 119) | 50678 | VL1 | RSSQSLLHSNGYNYLD |
| | 50679 | VL2 | LGSNRAS |
| | 50680 | VL3 | MQALPLT |
| VL-Consensus-40 (Table 120) | 50681 | VL1 | SGSSSNIGSNTVN |
| | 50682 | VL2 | SNNQRPS |
| | 50683 | VL3 | AAWDDSLNGVV |
| VL-Consensus-41 (Table 121) | 50684 | VL1 | SGSSSNIGSNIVT |
| | 50685 | VL2 | SNDQRPS |

FIGURE 55

| | | | |
|---|---|---|---|
| VL-Consensus-42 (Table 122) | 50687 | VL1 | RASQGIRNDLG |
| | 50688 | VL2 | AASSLQS |
| | 50689 | VL3 | LQHNSYPIT |
| VL-Consensus-43 (Table 123) | 50690 | VL1 | RASQSISSYLN |
| | 50691 | VL2 | AASSLQS |
| | 50692 | VL3 | QQSYSIPIT |
| VL-Consensus-44 (Table 124) | 50693 | VL1 | KSSQSLLHSEGKTYLY |
| | 50694 | VL2 | EVSNRFS |
| | 50695 | VL3 | MQSIQ PIT |
| VL-Consensus-45 (Table 125) | 50696 | VL1 | RASQSVISSYLA |
| | 50697 | VL2 | GVSSRAT |
| | 50698 | VL3 | QQYGRSPFN |
| VL-Consensus-46 (Table 126) | 50699 | VL1 | RASQSVSSNLA |
| | 50700 | VL2 | GASTRAT |
| | 50701 | VL3 | QQYNDWPWT |
| VL-Consensus-47 (Table 127) | 50702 | VL1 | RASQSIGSSLH |
| | 50703 | VL2 | YASQSFS |
| | 50704 | VL3 | HQSSSLPWT |
| VL-Consensus-48 (Table 128) | 50705 | VL1 | RASQGIRNDLG |
| | 50706 | VL2 | AASSLQS |
| | 50707 | VL3 | LQHYSYPRS |
| VL-Consensus-49 (Table 129) | 50708 | VL1 | RASQGISNYLA |
| | 50709 | VL2 | AASSLQS |
| | 50710 | VL3 | QQYLSYPIT |
| VL-Consensus-50 (Table 130) | 50711 | VL1 | RASQSIGSSLH |
| | 50712 | VL2 | YASQSFS |
| | 50713 | VL3 | HQSRRLPLT |
| VL-Consensus-51 (Table 131) | 50714 | VL1 | QGDSLRPYYAS |

**FIGURE 55**

| | | | |
|---|---|---|---|
| | 50716 | VL3 | NSRDSSGNHLVV |
| | | | |
| VL-Consensus-52 (Table 132) | 50717 | VL1 | RSSQSLVYSDGNTYLN |
| | 50718 | VL2 | KVSNWDS |
| | 50719 | VL3 | MQGTHWPLT |
| | | | |
| VL-Consensus-53 (Table 133) | 50720 | VL1 | RASQSVSRNLA |
| | 50721 | VL2 | GASTRAT |
| | 50722 | VL3 | QQYNNWPLT |
| | | | |
| VL-Consensus-54 (Table 134) | 50723 | VL1 | GGNNIGRKNVH |
| | 50724 | VL2 | RDSRPS |
| | 50725 | VL3 | QVWDSSTVV |

FIGURE 55

Table 20C

**VARIABLE LIGHT CDR CONSENSUS SEQUENCES II**

| NAME (ORIGINAL AND PATENT) | Patent SEQ ID NO: | CDR | Sequence |
|---|---|---|---|
| VL-CONSENSUS-15 TABLE 95 | SEQ ID NO: 50122 | VL1 | X1 X2 Ser X3 X4 X5 X6 X7 X8 X9 X10, wherein X1 = R or L or a conservative substitution thereof, X2 = A or T or a conservative substitution thereof, X3 = Q or R or a conservative substitution thereof, X4 = G or D or A or V or a conservative substitution thereof, X5 = I or M or V or a conservative substitution thereof, X6 = R or E or K or N or S or a conservative substitution thereof, X7 = N or S or D or T or K or I or a conservative substitution thereof, X8 = D or N or A or a conservative substitution thereof, X9 = L or F or V or a conservative substitution thereof, X10 = G or D or N or a conservative substitution thereof. |
| | SEQ ID NO: 50123 | VL2 | X1 X2 X3 X4 X5 X6 X7, wherein X1 = A or T or S or V or G or D or R or a conservative substitution thereof, X2 = A or T or V or E or a conservative substitution thereof, X3 = S or F or C or Y or a conservative substitution thereof, X4 = S or N or T or F or R or I or a conservative substitution thereof, X5 = L or V or F or S or a conservative substitution thereof, X6 = Q or H or E or a conservative substitution thereof, X7 = S or R or N or T or G or a conservative substitution thereof. |
| | SEQ ID NO: 50124 | VL3 | X1 X2 X3 X4 X5 X6 X7 X8 X9, wherein X1 = L or V or I or a conservative substitution thereof, X2 = Q or H or a conservative substitution thereof, X3 = H or D or Y or N or R or a conservative substitution thereof, X4 = N or S or Y or T or D or K or A or I or E or H or P or R or a conservative substitution thereof, X5 = S or I or N or R or T or D or A or L or V or a conservative substitution thereof, X6 = Y or F or H or S or a conservative substitution thereof, X7 = P or A or M or a conservative substitution thereof, X8 = L or P or F or N or V or a conservative substitution thereof, X9 = T or K or I or a conservative substitution thereof. |
| | | | |
| VL-CONSENSUS-16 TABLE 96 | SEQ ID NO: 50125 | VL1 | Ser Gly X1 X2 X3 Gly X4 X5 X6 X7 X8, wherein X1 = D or N or E or Y or S or a conservative substitution thereof, X2 = K or R or N or E or T or a conservative substitution thereof, X3 = L or M or S or a conservative substitution thereof, X4 = D or N or E or G or Y or T or H or V or a conservative substitution thereof, X5 = K or R or a conservative substitution thereof, X6 = Y or F or S or a conservative substitution thereof, X7 = A or V or T or D or S or a conservative substitution thereof, X8 = C or S or Y or W or H or a conservative substitution |

**FIGURE 55**

| | | | |
|---|---|---|---|
| | SEQ ID NO: 50126 | VL2 | X1 X2 X3 X4 Arg X5 X6, wherein X1 = Q or E or K or a conservative substitution thereof, X2 = D or N or a conservative substitution thereof, X3 = R or S or N or K or Y or M or T or G or I or a conservative substitution thereof, X4 = K or R or Q or a conservative substitution |
| | SEQ ID NO: 50228 | VL3 | X1 X2 X3 X4 X5 X6 X7 X8 X9 X10, wherein X1 = Q or L or K or a conservative substitution thereof, X2 = A or T or a conservative substitution thereof, X3 = W or R or a conservative substitution thereof, X4 = D or H or V or N or G or a conservative substitution thereof, X5 = S or N or Absent or I or R or K or T or a conservative substitution thereof, X6 = Absent or S or N or R or a conservative substitution thereof, X7 = S or T or N or R or G or F or I or V or Y or a conservative substitution thereof, X8 = T or S or Y or A or F or P or N or K or I or R or a conservative substitution thereof, X9 = V or A or T or M or L or G or a conservative substitution thereof, X10 = V or I or L or A or M. |
| | | | |
| VL-CONSENSUS-17 TABLE 97 | SEQ ID NO: 50127 | VL1 | X1 X2 X3 X4 X5 X6 X7 X8 X9 X10 Leu X11, wherein X1 = R or W or a conservative substitution thereof, X2 = A or T or a conservative substitution thereof, X3 = S or G or R or a conservative substitution thereof, X4 = Q or P or a conservative substitution thereof, X5 = S or N or I or R or a conservative substitution thereof, X6 = V or I or F or a conservative substitution thereof, X7 = Y or R or S or N or D or F or H or G or W or a conservative substitution thereof, X8 = S or T or N or G or L or R or a conservative substitution thereof, X9 = S or N or G or R or D or A or Y or a conservative substitution thereof, X10 = Y or F or H or a conservative substitution thereof, X11 = A or V or S or a conservative substitution thereof. |
| | SEQ ID NO: 50128 | VL2 | X1 X2 X3 X4 Arg X5 X6, wherein X1 = G or D or V or a conservative substitution thereof, X2 = A or T or P or V or a conservative substitution thereof, X3 = S or F or Y or A or T or a conservative substitution thereof, X4 = S or R or N or A or a conservative substitution thereof, X5 = A or S or T or a conservative substitution thereof, X6 = T or P or S or A or a conservative substitution thereof. |
| | SEQ ID NO: 50129 | VL3 | X1 X2 X3 X4 X5 X6 X7 X8 X9 X10, wherein X1 = Q or H or a conservative substitution thereof, X2 = Q or H or a conservative substitution thereof, X3 = Y or S or a conservative substitution thereof, X4 = D or E or G or H or a conservative substitution thereof, X5 = N or S or Absent or R or T or I or D or G or a conservative substitution thereof, X6 = Absent or S or a conservative substitution thereof, X7 = S or P or N or R or a |

**FIGURE 55**

| | | | |
|---|---|---|---|
| | | | conservative substitution thereof, X9 = W or R or a conservative substitution thereof, X10 = T or A or a conservative substitution thereof. |
| | | | |
| VL-CONSENSUS-18 TABLE 98 | SEQ ID NO: 50130 | VL1 | Arg X1 X2 X3 X4 Ile X5 X6 X7 Leu X8, wherein X1 = A or T or a conservative substitution thereof, X2 = S or T or a conservative substitution thereof, X3 = Q or R or a conservative substitution thereof, X4 = G or D or A or N or V or a conservative substitution thereof, X5 = R or G or a conservative substitution thereof, X6 = N or K or D or H or S or G or T or a conservative substitution thereof, X7 = D or I or Y or a conservative substitution thereof, X8 = G or N or a conservative substitution thereof. |
| | SEQ ID NO: 50131 | VL2 | X1 X2 X3 X4 X5 X6 X7, wherein X1 = A or T or I or V or P or G or R or S or a conservative substitution thereof, X2 = A or T or S or a conservative substitution thereof, X3 = S or A or F or P or Y or a conservative substitution thereof, X4 = S or N or R or G or T or a conservative substitution thereof, X5 = L or C or F or S or a conservative substitution thereof, X6 = Q or H or E or a conservative substitution thereof, X7 = S or N or G or I or a conservative substitution thereof. |
| | SEQ ID NO: 50132 | VL3 | X1 X2 X3 X4 X5 X6 X7 X8 X9 Thr, wherein X1 = L or V or I or H or a conservative substitution thereof, X2 = Q or M or H or L or V or a conservative substitution thereof, X3 = H or Q or Y or L or S or a conservative substitution thereof, X4 = Y or N or H or S or T or a conservative substitution thereof, X5 = S or N or T or R or D or F or G or a conservative substitution thereof, X6 = Absent or Y or F or a conservative substitution thereof, X7 = Y or F or P or C or N or T or a conservative substitution thereof, X8 = P or L or a conservative substitution thereof, X9 = R or W or F or L or a conservative substitution thereof. |
| | | | |
| VL-CONSENSUS-19 TABLE 99 | SEQ ID NO: 50133 | VL1 | X1 X2 X3 Gln Ser X4 Leu X5 X6 X7 X8 X9 X10 X11 X12 X13 X14, wherein X1 = K or R or M or a conservative substitution thereof, X2 = S or G or R or a conservative substitution thereof, X3 = S or T or N or I or a conservative substitution thereof, X4 = V or I or A or a conservative substitution thereof, X5 = H or Y or F or S or K or D or L or M or a conservative substitution thereof, X6 = S or T or R or N or I or D or a conservative substitution thereof, X7 = S or F or P or a conservative substitution thereof, X8 = N or H or a conservative substitution thereof, X9 = N or K or S or D or H or a conservative substitution thereof, X10 = N or Y or K or H or R or A or F or W or a conservative |

**FIGURE 55**

| | | | |
|---|---|---|---|
| | | | conservative substitution thereof, X12 = Y or S or a conservative substitution thereof, X13 = L or F or a conservative substitution thereof, X14 = A or T or V or G or a conservative substitution thereof. |
| | SEQ ID NO: 50229 | VL2 | Trp X1 X2 X3 X4 X5 X6 wherein X1 = A or T or S or a conservative substitution thereof, X2 = S or F or a conservative substitution thereof, X3 = T or I or K or S or a conservative substitution thereof, X4 = R or W or L or a conservative substitution thereof, X5 = E or K or A or D or R or a conservative substitution thereof, X6 = S or T or a conservative substitution thereof. |
| | SEQ ID NO: 50134 | VL3 | X1 X2 X3 X4 X5 X6 Pro X7 X8, wherein X1 = Q or H or L or a conservative substitution thereof, X2 = Q or H or a conservative substitution thereof, X3 = Y or F or a conservative substitution thereof, X4 = Y or F or H or N or L or S or a conservative substitution thereof, X5 = S or N or R or D or I or T or C or E or K or a conservative substitution thereof, X6 = T or S or I or V or A or Y or a conservative substitution thereof, X7 = P or W or L or V or C or G or R or S or a conservative substitution thereof, X8 = T or K or S or a conservative substitution thereof. |
| | | | |
| VL-CONSENSUS-20 TABLE 100 | SEQ ID NO: 50135 | VL1 | Arg X1 Ser Gln X2 X3 X4 X5 X6 X7 X8, wherein X1 = A or T or a conservative substitution thereof, X2 = G or D or V or a conservative substitution thereof, X3 = I or M or a conservative substitution thereof, X4 = R or S or a conservative substitution thereof, X5 = N or K or S or D or T or a conservative substitution thereof, X6 = D or N or H or Y or V or A or I or L or a conservative substitution thereof, X7 = L or F or a conservative substitution thereof, X8 = G or D or a conservative substitution thereof. |
| | SEQ ID NO: 50136 | VL2 | X1 X2 X3 X4 X5 X6 X7, wherein X1 = A or P or T or G or I or R or V or a conservative substitution thereof, X2 = A or V or a conservative substitution thereof, X3 = S or F or T or a conservative substitution thereof, X4 = S or N or T or D or a conservative substitution thereof, X5 = L or V or a conservative substitution thereof, X6 = Q or L or a conservative substitution thereof, X7 = S or N or T or G or R or a conservative substitution thereof. |
| | SEQ ID NO: 50137 | VL3 | X1 X2 X3 X4 X5 X6 X7 X8 X9, wherein X1 = L or I or a conservative substitution thereof, X2 = Q or H or L or a conservative substitution thereof, X3 = H or Y or D or L or a conservative substitution thereof, X4 = N or Y or T or H or G or I or a conservative substitution thereof, X5 = S or R or D or T or G or N or a conservative substitution thereof, X6 = Y or F or H or a |

**FIGURE 55**

| | | | |
|---|---|---|---|
| | | | conservative substitution thereof, X8 = F or L or a conservative substitution thereof, X9 = T or K or a conservative substitution thereof. |
| | | | |
| VL-CONSENSUS-21 TABLE 101 | SEQ ID NO: 50138 | VL1 | Arg X1 X2 Gln X3 Ile X4 X5 X6 Leu X7, wherein X1 = A or T or a conservative substitution thereof, X2 = S or N or a conservative substitution thereof, X3 = G or D or A or V or S or a conservative substitution thereof, X4 = S or G or N or R or A or F or a conservative substitution thereof, X5 = N or K or R or H or S or T or I or a conservative substitution thereof, X6 = Y or H or C or D or a conservative substitution thereof, X7 = A or D or V or I or N or a conservative substitution thereof. |
| | SEQ ID NO: 50139 | VL2 | X1 X2 X3 X4 Leu X5 X6, wherein X1 = A or K or S or T or V or D or G or a conservative substitution thereof, X2 = A or T or V or a conservative substitution thereof, X3 = S or P or a conservative substitution thereof, X4 = S or N or R or a conservative substitution thereof, X5 = Q or L or E or H or a conservative substitution thereof, X6 = S or G or N or T or a conservative substitution thereof. |
| | SEQ ID NO: 50140 | VL3 | X1 X2 X3 X4 X5 X6 Pro X7 X8, wherein X1 = Q or L or H or a conservative substitution thereof, X2 = Q or H or R or Y or a conservative substitution thereof, X3 = Y or S or C or T or a conservative substitution thereof, X4 = S or L or N or M or D or H or V or I or Y or a conservative substitution thereof, X5 = T or N or S or K or a conservative substitution thereof, X6 = Y or F or I or S or a conservative substitution thereof, X7 = L or V or F or N or a conservative substitution thereof, X8 = T or I or Q or S or a conservative substitution thereof. |
| | | | |
| VL-CONSENSUS-22 TABLE 102 | SEQ ID NO: 50141 | VL1 | Arg X1 X2 X3 X4 X5 X6 X7 X8 Leu X9, wherein X1 = A or T or a conservative substitution thereof, X2 = S or T or a conservative substitution thereof, X3 = Q or H or R or a conservative substitution thereof, X4 = N or S or R or T or I or a conservative substitution thereof, X5 = I or V or F or a conservative substitution thereof, X6 = I or S or N or Y or F or R or H or L or K or T or a conservative substitution thereof, X7 = S or N or D or R or K or T or a conservative substitution thereof, X8 = Y or F or a conservative substitution thereof, X9 = N or H or a conservative substitution thereof. |
| | SEQ ID NO: 50142 | VL2 | X1 X2 Ser X3 X4 X5 X6, wherein X1 = T or A or V or G or I or S or a conservative substitution thereof, X2 = A or T or a conservative substitution thereof, X3 = S or N or R or T or a conservative substitution thereof, X4 = L or F or S or a conservative substitution thereof, X5 = Q or H or E or P or a conservative substitution thereof, |

**FIGURE 55**

| | | | |
|---|---|---|---|
| | | | X6 = S or G or T or N or R or a conservative substitution thereof. |
| | SEQ ID NO: 50143 | VL3 | Gln X1 X2 X3 X4 X5 X6 X7 Thr, wherein X1 = Q or L or a conservative substitution thereof, X2 = S or T or N or P or a conservative substitution thereof, X3 = Y or H or C or D or F or N or a conservative substitution thereof, X4 = S or Absent or I or N or F or G or T or a conservative substitution thereof, X5 = T or S or P or F or N or I or L or a conservative substitution thereof, X6 = P or T or I or A or S or a conservative substitution thereof, X7 = L or P or Y or F or V or a conservative substitution thereof. |
| | | | |
| VL-CONSENSUS-23 TABLE 103 | SEQ ID NO: 50144 | VL1 | X1 X2 X3 X4 X5 X6 X7 X8 X9 Leu X10, wherein X1 = R or P or a conservative substitution thereof, X2 = A or T or a conservative substitution thereof, X3 = S or N or a conservative substitution thereof, X4 = Q or R or a conservative substitution thereof, X5 = G or D or V or A or a conservative substitution thereof, X6 = I or V or a conservative substitution thereof, X7 = S or N or G or R or T or K or a conservative substitution thereof, X8 = N or K or Y or H or I or T or a conservative substitution thereof, X9 = Y or H or a conservative substitution thereof, X10 = A or V or S or a conservative substitution thereof. |
| | SEQ ID NO: 50145 | VL2 | X1 X2 X3 X4 X5 X6 X7, wherein X1 = A or V or G or T or a conservative substitution thereof, X2 = A or S or V or a conservative substitution thereof, X3 = S or F or a conservative substitution thereof, X4 = S or G or N or T or a conservative substitution thereof, X5 = L or V or a conservative substitution thereof, X6 = Q or R or H or L or E or a conservative substitution thereof, X7 = S or G or T or a conservative substitution thereof. |
| | SEQ ID NO: 50146 | VL3 | X1 X2 X3 X4 X5 X6 Pro X7 X8, wherein X1 = Q or H or L or P or R or a conservative substitution thereof, X2 = Q or R or K or H or L or a conservative substitution thereof, X3 = Y or F or a conservative substitution thereof, X4 = N or H or D or Y or S or K or L or M or Q or a conservative substitution thereof, X5 = S or T or G or N or C or D or a conservative substitution thereof, X6 = Y or F or H or a conservative substitution thereof, X7 = F or V or L or I or a conservative substitution thereof, X8 = T or K or a conservative substitution thereof. |
| | | | |
| VL-CONSENSUS-24 TABLE 104 | SEQ ID NO: 50147 | VL1 | Arg X1 X2 X3 X4 X5 X6 X7 X8 X9 X10, wherein X1 = A or V or E or G or a conservative substitution thereof, X2 = S or G or a conservative substitution thereof, X3 = Q or R or a conservative substitution |

**FIGURE 55**

| | | | |
|---|---|---|---|
| | | | conservative substitution thereof, X5 = I or V or F or a conservative substitution thereof, X6 = S or N or T or R or I or a conservative substitution thereof, X7 = R or S or N or K or T or D or I or G or a conservative substitution thereof, X8 = W or Y or a conservative substitution thereof, X9 = L or I or a conservative substitution thereof, 10 = A or T or V or a conservative substitution thereof. |
| | SEQ ID NO: 50148 | VL2 | X1 X2 X3 X4 X5 X6 X7, wherein X1 = A or G or T or D or V or a conservative substitution thereof, X2 = A or T or V or a conservative substitution thereof, X3 = S or Y or a conservative substitution thereof, X4 = S or R or N or T or G or I or a conservative substitution thereof, X5 = L or F or a conservative substitution thereof, X6 = Q or E or a conservative substitution thereof, X7 = S or G or N or D or a conservative substitution thereof. |
| | SEQ ID NO: 50149 | VL3 | X1 Gln X2 X3 X4 X5 Pro X6 Thr, wherein X1 = Q or H or a conservative substitution thereof, X2 = A or T or G or S or V or D or a conservative substitution thereof, X3 = N or D or K or S or a conservative substitution thereof, X4 = S or I or a conservative substitution thereof, X5 = F or L or I or V or a conservative substitution thereof, X6 = F or I or a conservative substitution thereof. |
| | | | |
| VL-CONSENSUS-25 TABLE 105 | SEQ ID NO: 50150 | VL1 | X1 Ser X2 X3 X4 Leu X5 X6 X7 X8 Gly X9 Thr X10 X11 X12, wherein X1 = K or M or R or T or a conservative substitution thereof, X2 = S or G or T or a conservative substitution thereof, X3 = Q or K or a conservative substitution thereof, X4 = S or R or N or T or a conservative substitution thereof, X5 = L or R or V or a conservative substitution thereof, X6 = H or Y or a conservative substitution thereof, X7 = G or S or a conservative substitution thereof, X8 = D or E or G or a conservative substitution thereof, X9 = K or R or a conservative substitution thereof, X10 = Y or F or a conservative substitution thereof, X11 = L or F or a conservative substitution thereof, X12 = Y or F or T or C or S or a conservative substitution thereof. |
| | SEQ ID NO: 50151 | VL2 | X1 X2 Ser X3 Arg X4 X5, wherein X1 = E or A or a conservative substitution thereof, X2 = V or I or L or T or a conservative substitution thereof, X3 = N or K or H or I or S or a conservative substitution thereof, X4 = F or L or a conservative substitution thereof, X5 = S or A or T or C or P or a conservative substitution thereof. |
| | SEQ ID NO: 50152 | VL3 | X1 Gln X2 X3 X4 X5 Pro X6 Thr, wherein X1 = M or K or a conservative substitution thereof, X2 = S or T or a conservative substitution thereof, X3 = I or T or F or L or a conservative substitution thereof, X4 = Q or H or |

**FIGURE 55**

| | | | |
|---|---|---|---|
| | | | F or a conservative substitution thereof, X6 = W or R or a conservative substitution thereof. |
| | | | |
| VL-CONSENSUS-26 TABLE 106 | SEQ ID NO: 50153 | VL1 | Arg X1 X2 X3 X4 X5 X6 X7 X8 X9 X10, wherein X1 = A or S or T or a conservative substitution thereof, X2 = S or G or I or a conservative substitution thereof, X3 = Q or H or R or a conservative substitution thereof, X4 = S or N or T or a conservative substitution thereof, X5 = I or F or a conservative substitution thereof, X6 = S or I or F or N or R or Y or T or A or G or L or a conservative substitution thereof, X7 = S or N or T or H or K or a conservative substitution thereof, X8 = Y or F or H or a conservative substitution thereof, X9 = L or V or a conservative substitution thereof, X10 = N or I or M or H or Y or a conservative substitution thereof. |
| | SEQ ID NO: 50154 | VL2 | X1 X2 X3 X4 Leu X5 X6, wherein X1 = A or G or T or V or I or a conservative substitution thereof, X2 = A or T or V or S or a conservative substitution thereof, X3 = S or F or a conservative substitution thereof, X4 = S or N or T or V or a conservative substitution thereof, X5 = Q or H or a conservative substitution thereof, X6 = S or N or G or H or I or T or a conservative substitution thereof. |
| | SEQ ID NO: 50155 | VL3 | Gln Gln X1 X2 X3 X4 X5 X6 Phe X7, wherein X1 = S or T or Y or a conservative substitution thereof, X2 = Y or N or F or H or a conservative substitution thereof, X3 = S or R or N or F or I or a conservative substitution thereof, X4 = Absent or A or S or a conservative substitution thereof, X5 = P or T or I or F or A or L or V or a conservative substitution thereof, X6 = P or L or F or S or a conservative substitution thereof, X7 = T or A or S or a conservative substitution thereof. |
| | | | |
| VL-CONSENSUS-27 TABLE 107 | SEQ ID NO: 50156 | VL1 | X1 Ser X2 Gln X3 X4 Leu X5 X6 Ser X7 X8 X9 X10 X11 Leu X12, wherein X1 = K or R or T or a conservative substitution thereof, X2 = S or I or a conservative substitution thereof, X3 = S or N or a conservative substitution thereof, X4 = V or I or a conservative substitution thereof, X5 = Y or H or S or F or a conservative substitution thereof, X6 = S or N or I or R or H or a conservative substitution thereof, X7 = N or H or a conservative substitution thereof, X8 = N or S or D or a conservative substitution thereof, X9 = N or Y or K or H or A or M or Q or a conservative substitution thereof, X10 = N or K or a conservative substitution thereof, X11 = Y or F or a conservative substitution thereof, X12 = A or T or D or a conservative substitution thereof. |
| | SEQ ID NO: | VL2 | Trp X1 X2 X3 Arg X4 X5, wherein X1 = A or T or G |

**FIGURE 55**

| | | | |
|---|---|---|---|
| | | | or a conservative substitution thereof, X3 = T or I or a conservative substitution thereof, X4 = E or K or D or a conservative substitution thereof, X5 = S or F or a conservative substitution thereof. |
| | SEQ ID NO: 50158 | VL3 | X1 X2 X3 X4 X5 X6 X7 X8 X9, wherein X1 = Q or H or a conservative substitution thereof, X2 = Q or H or a conservative substitution thereof, X3 = Y or S or a conservative substitution thereof, X4 = Y or F or K or N or a conservative substitution thereof, X5 = S or T or I or Absent or N or a conservative substitution thereof, X6 = T or S or I or R or A or G or N or a conservative substitution thereof, X7 = P or S or a conservative substitution thereof, X8 = C or Y or G or L or P or a conservative substitution thereof, X9 = S or K or N or a conservative substitution thereof. |
| | | | |
| VL-CONSENSUS-28 TABLE 108 | SEQ ID NO: 50159 | VL1 | Arg Ala X1 Gln X2 X3 X4 X5 X6 Leu Ala, wherein X1 = S or N or a conservative substitution thereof, X2 = G or D or F or N or V or a conservative substitution thereof, X3 = I or L or V or a conservative substitution thereof, X4 = S or N or T or I or F or G or a conservative substitution thereof, X5 = N or S or D or T or R or a conservative substitution thereof, X6 = W or C or a conservative substitution thereof. |
| | SEQ ID NO: 50160 | VL2 | X1 X2 X3 X4 Leu Gln X5, wherein X1 = A or G or D or T or S or a conservative substitution thereof, X2 = A or V or P or T or a conservative substitution thereof, X3 = S or F or a conservative substitution thereof, X4 = S or N or a conservative substitution thereof, X5 = S or G or N or a conservative substitution thereof. |
| | SEQ ID NO: 50161 | VL3 | X1 Gln X2 X3 Ser X4 Pro X5 Thr, wherein X1 = Q or L or a conservative substitution thereof, X2 = A or T or S or G or V or Y or a conservative substitution thereof, X3 = N or D or H or Y or a conservative substitution thereof, X4 = F or L or a conservative substitution thereof, X5 = W or R or P or a conservative substitution thereof. |
| | | | |
| VL-CONSENSUS-29 TABLE 109 | SEQ ID NO: 50162 | VL1 | Lys Ser X1 Gln X2 X3 X4 X5 X6 Ser X7 X8 X9 X10 Tyr Leu X11, wherein X1 = S or N or a conservative substitution thereof, X2 = S or R or N or a conservative substitution thereof, X3 = V or I or L or a conservative substitution thereof, X4 = L or F or a conservative substitution thereof, X5 = H or F or Y or K or S or a conservative substitution thereof, X6 = S or N or H or a conservative substitution thereof, X7 = N or H or a conservative substitution thereof, X8 = N or S or a conservative substitution thereof, X9 = N or K or Y or H or a conservative substitution thereof, X10 = N or R |

FIGURE 55

| | | | |
|---|---|---|---|
| | | | or S or a conservative substitution thereof, X11 = A or T or V or a conservative substitution thereof. |
| | SEQ ID NO: 50163 | VL2 | Trp Ala Ser X1 X2 X3 Ser, wherein X1 = T or A or I or S or a conservative substitution thereof, X2 = R or L or a conservative substitution thereof, X3 = E or K or D or a conservative substitution thereof. |
| | SEQ ID NO: 50164 | VL3 | X1 Gln X2 X3 X4 X5 Pro X6 Thr, wherein X1 = Q or H or a conservative substitution thereof, X2 = Y or S or a conservative substitution thereof, X3 = Y or C or F or S or H or a conservative substitution thereof, X4 = S or N or Q or D or T or a conservative substitution thereof, X5 = T or L or I or A or F or S or a conservative substitution thereof, X6 = V or F or P or a conservative substitution thereof. |
| | | | |
| VL-CONSENSUS-30 TABLE 110 | SEQ ID NO: 50165 | VL1 | X1 X2 X3 Thr X4 X5 Val Thr Ser X6 X7 X8 Pro X9, wherein X1 = A or V or G or a conservative substitution thereof, X2 = S or F or L or a conservative substitution thereof, X3 = S or N or a conservative substitution thereof, X4 = G or E or a conservative substitution thereof, X5 = A or S or T or a conservative substitution thereof, X6 = G or A or a conservative substitution thereof, X7 = Y or S or N or F or a conservative substitution thereof, X8 = Y or F or a conservative substitution thereof, X9 = N or S or Q or a conservative substitution thereof. |
| | SEQ ID NO: 50166 | VL2 | X1 Thr X2 Asn X3 His Ser, wherein X1 = S or H or N or a conservative substitution thereof, X2 = S or N or D or I or T or a conservative substitution thereof, X3 = K or R or a conservative substitution thereof. |
| | SEQ ID NO: 50167 | VL3 | X1 X2 X3 X4 X5 Gly X6 X7 X8 X9, wherein X1 = L or M or a conservative substitution thereof, X2 = L or I or F or a conservative substitution thereof, X3 = Y or F or a conservative substitution thereof, X4 = Y or C or F or S or a conservative substitution thereof, X5 = G or D or a conservative substitution thereof, X6 = A or V or a conservative substitution thereof, X7 = Q or H or a conservative substitution thereof, X8 = L or V or M or a conservative substitution thereof, X9 = V or A or I or G or M or a conservative substitution thereof. |
| | | | |
| VL-CONSENSUS-31 TABLE 111 | SEQ ID NO: 50168 | VL1 | Arg X1 Ser X2 X3 X4 X5 X6 X7 Leu Ala, wherein X1 = A or S or a conservative substitution thereof, X2 = Q or L or M or V or a conservative substitution thereof, X3 = S or N or D or R or T or a conservative substitution thereof, X4 = V or I or a conservative substitution thereof, X5 = N or K or S or V or A or I or L or a conservative substitution thereof, X6 = S or N or T or a conservative substitution thereof, X7 = N or S or |

**FIGURE 55**

| | | | |
|---|---|---|---|
| | SEQ ID NO: 50169 | VL2 | X1 X2 Ser X3 Arg Ala Thr, wherein X1 = G or I or F or V or a conservative substitution thereof, X2 = A or T or a conservative substitution thereof, X3 = T or I or a conservative substitution thereof. |
| | SEQ ID NO: 50170 | VL3 | Gln X1 X2 X3 X4 X5 X6 X7 Cys Ser, wherein X1 = Q or E or a conservative substitution thereof, X2 = Y or F or a conservative substitution thereof, X3 = N or Y or D or a conservative substitution thereof, X4 = D or N or a conservative substitution thereof, X5 = Absent or W or a conservative substitution thereof, X6 = W or P or a conservative substitution thereof, X7 = P or L or M or a conservative substitution thereof. |
| | | | |
| VL-CONSENSUS-32 TABLE 112 | SEQ ID NO: 50171 | VL1 | Arg X1 Ser X2 X3 X4 X5 X6 X7 X8 Leu X9, wherein X1 = A or S or a conservative substitution thereof, X2 = Q or E or a conservative substitution thereof, X3 = S or R or a conservative substitution thereof, X4 = V or I or a conservative substitution thereof, X5 = S or T or a conservative substitution thereof, X6 = S or T or a conservative substitution thereof, X7 = S or N or a conservative substitution thereof, X8 = Y or A or a conservative substitution thereof, X9 = V or S or a conservative substitution thereof. |
| | SEQ ID NO: 50172 | VL2 | Gly Ala Ser X1 Arg Ala X2, wherein X1 = T or S or a conservative substitution thereof, X2 = T or S or I or a conservative substitution thereof. |
| | SEQ ID NO: 50173 | VL3 | Gln Gln Tyr X1 X2 Ser X3 Leu Thr wherein X1 = G or V or a conservative substitution thereof, X2 = C or N or S or a conservative substitution thereof, X3 = P or L or a conservative substitution thereof. |
| | | | |
| VL-CONSENSUS-33 TABLE 113 | SEQ ID NO: 50174 | VL1 | Lys Ser Ser Gln X1 Leu X2 X3 X4 X5 Gly X6 Thr X7 Leu X8, wherein X1 = S or T or a conservative substitution thereof, X2 = L or Q or a conservative substitution thereof, X3 = H or R or a conservative substitution thereof, X4 = S or G or a conservative substitution thereof, X5 = E or D or a conservative substitution thereof, X6 = K or R or a conservative substitution thereof, X7 = Y or H or F or a conservative substitution thereof, X8 = Y or N or a conservative substitution thereof. |
| | SEQ ID NO: 50175 | VL2 | Glu X1 Ser X2 Arg X3 Ser, wherein X1 = V or I or a conservative substitution thereof, X2 = N or Y or H or a conservative substitution thereof, X3 = F or I or V or L or a conservative substitution thereof. |
| | SEQ ID NO: 50176 | VL3 | X1 X2 X3 X4 X5 X6 X7 X8 X9, wherein X1 = M or F or a conservative substitution thereof, X2 = Q or H or a conservative substitution thereof, X3 = S or G or N or a conservative substitution thereof, X4 = I or K or T or a |

FIGURE 55

| | | | |
|---|---|---|---|
| | | | K or H or a conservative substitution thereof, X6 = L or Q or Y or F or H or a conservative substitution thereof, X7 = P or L or V or a conservative substitution thereof, X8 = L or F or P or a conservative substitution thereof, X9 = T or P or S or a conservative substitution thereof. |
| | | | |
| VL-CONSENSUS-34 TABLE 114 | SEQ ID NO: 50177 | VL1 | Thr Gly Thr Ser Ser Asp X1 Gly X2 Tyr Asn X3 Val Ser, wherein X1 = V or I or a conservative substitution thereof, X2 = G or S or a conservative substitution thereof, X3 = Y or F or a conservative substitution thereof. |
| | SEQ ID NO: 50178 | VL2 | Glu Val X1 Asn Arg Pro Ser, wherein X1 = S or R or a conservative substitution thereof. |
| | SEQ ID NO: 50179 | VL3 | X1 Ser Tyr X2 X3 X4 X5 Thr Trp Val, wherein X1 = N or G or C or S or a conservative substitution thereof, X2 = T or V or K or a conservative substitution thereof, X3 = R or S or K or a conservative substitution thereof, X4 = S or G or N or R or a conservative substitution thereof, X5 = I or S or Y or a conservative substitution thereof. |
| | | | |
| VL-CONSENSUS-35 TABLE 115 | SEQ ID NO: 50180 | VL1 | Arg X1 Ser X2 X3 Ile X4 X5 X6 Leu Asn, wherein X1 = A or S or a conservative substitution thereof, X2 = Q or H or R or a conservative substitution thereof, X3 = S or N or T or H or a conservative substitution thereof, X4 = S or N or G or T or a conservative substitution thereof, X5 = N or S or R or a conservative substitution thereof, X6 = Y or F or a conservative substitution thereof. |
| | SEQ ID NO: 50181 | VL2 | X1 X2 X3 X4 Leu X5 X6, wherein X1 = A or T or S or V or a conservative substitution thereof, X2 = A or T or E or V or a conservative substitution thereof, X3 = S or L or a conservative substitution thereof, X4 = S or N or a conservative substitution thereof, X5 = Q or H or a conservative substitution thereof, X6 = S or I or a conservative substitution thereof. |
| | SEQ ID NO: 50182 | VL3 | Gln Gln X1 Tyr X2 X3 X4 X5 Trp Thr, wherein X1 = S or G or T or a conservative substitution thereof, X2 = S or T or N or R or a conservative substitution thereof, X3 = T or S or Absent or a conservative substitution thereof, X4 = P or I or a conservative substitution thereof, X5 = T or P or Q or L or a conservative substitution thereof. |
| | | | |
| VL-CONSENSUS-36 TABLE 116 | SEQ ID NO: 50183 | VL1 | Arg Ala Ser Gln X1 Ile Ser X2 X3 Leu Ala, wherein X11 = G or D or a conservative substitution thereof, X2 = S or N or I or K or a conservative substitution thereof, X3 = W or Y or a conservative substitution thereof. |
| | SEQ ID NO: | VL2 | Ala Ala Ser Ser Leu Gln X1, wherein X1 = S or G or a |

**FIGURE 55**

| | | | |
|---|---|---|---|
| | SEQ ID NO: 50185 | VL3 | Gln Gln X1 X2 Ser Phe Pro Leu Thr, wherein X1 = I or T or V or A or G or a conservative substitution thereof, X2 = N or K or a conservative substitution thereof. |
| | | | |
| VL-CONSENSUS-37 TABLE 117 | SEQ ID NO: 50186 | VL1 | Gln Ala X1 Gln X2 Ile X3 X4 X5 Leu Asn, wherein X1 = S or N or a conservative substitution thereof, X2 = D or Y or a conservative substitution thereof, X3 = N or S or T or F or Y or a conservative substitution thereof, X4 = N or D or a conservative substitution thereof, X5 = Y or F or a conservative substitution thereof. |
| | SEQ ID NO: 50187 | VL2 | Asp X1 Ser X2 Leu Glu Thr wherein X1 = A or G or a conservative substitution thereof, X2 = N or T or D or S or a conservative substitution thereof. |
| | SEQ ID NO: 50188 | VL3 | Gln Gln X1 X2 X3 X4 X5 Ile Thr, wherein X1 = Y or F or a conservative substitution thereof, X2 = D or E or a conservative substitution thereof, X3 = N or Absent or I or a conservative substitution thereof, X4 = L or N or V or a conservative substitution thereof, X5 = P or L or a conservative substitution thereof. |
| | | | |
| VL-CONSENSUS-38 TABLE 118 | SEQ ID NO: 50189 | VL1 | Ser Gly Ser Ser Ser Asn X1 Gly X2 X3 X4 X5 Ser, wherein X1 = I or L or a conservative substitution thereof, X2 = N or S or a conservative substitution thereof, X3 = N or H or K or a conservative substitution thereof, X4 = Y or F or a conservative substitution thereof, X5 = V or L or a conservative substitution thereof. |
| | SEQ ID NO: 50190 | VL2 | Asp X1 X2 Lys Arg Pro Ser, wherein X1 = N or S or a conservative substitution thereof, X2 = N or Y or S or a conservative substitution thereof. |
| | SEQ ID NO: 50191 | VL3 | Gly X1 Trp Asp X2 X3 Leu X4 X5 X6 Val, wherein X1 = T or A or I or a conservative substitution thereof, X2 = S or G or I or R or a conservative substitution thereof, X3 = S or R or a conservative substitution thereof, X4 = S or N or a conservative substitution thereof, X5 = V or A or T or a conservative substitution thereof, X6 = G or V or M or a conservative substitution thereof. |
| | | | |
| VL-CONSENSUS-39 TABLE 119 | SEQ ID NO: 50243 | VL1 | Arg X1 Ser Gln Ser Leu X2 X3 X4 X5 X6 X7 Asn X8 Leu Asp, wherein X1 = S or Y or a conservative substitution thereof, X2 = L or V or a conservative substitution thereof, X3 = H or Y or a conservative substitution thereof, X4 = S or N or H or a conservative substitution thereof, X5 = N or S or a conservative substitution thereof, X6 = G or K or R or a conservative substitution thereof, X7 = Y or H or N or a conservative substitution thereof, X8 = Y or H or S or a conservative substitution thereof. |

**FIGURE 55**

| | | | |
|---|---|---|---|
| | SEQ ID NO: 50244 | VL2 | X1 Gly Ser X2 Arg Ala Ser, wherein X1 = L or V or a conservative substitution thereof, X2 = N or H or a conservative substitution thereof. |
| | SEQ ID NO: 50245 | VL3 | Met Gln X1 Leu X2 X3 X4 X5 X6 Thr, wherein X1 = A or P or T or V or a conservative substitution thereof, X2 = H or Q or Absent or a conservative substitution thereof, X3 = Absent or T or a conservative substitution thereof, X4 = P or Q or T or I or a conservative substitution thereof, X5 = P or T or a conservative substitution thereof, X6 = L or P or F or a conservative substitution thereof. |
| | | | |
| VL-CONSENSUS-40 TABLE 120 | SEQ ID NO: 50192 | VL1 | Ser Gly X1 X2 Ser X3 Ile Gly X4 X5 X6 X7 X8, wherein X1 = S or T or a conservative substitution thereof, X2 = S or N or a conservative substitution thereof, X3 = N or Y or a conservative substitution thereof, X4 = S or N or a conservative substitution thereof, X5 = N or Y or a conservative substitution thereof, X6 = T or A or S or a conservative substitution thereof, X7 = V or I or a conservative substitution thereof, X8 = N or D or S or a conservative substitution thereof. |
| | SEQ ID NO: 50193 | VL2 | X1 X2 X3 X4 Arg Pro Ser, wherein X1 = S or I or a conservative substitution thereof, X2 = N or S or a conservative substitution thereof, X3 = N or D or S or a conservative substitution thereof, X4 = Q or H or a conservative substitution thereof. |
| | SEQ ID NO: 50194 | VL3 | X1 Ala Trp Asp Asp X2 X3 X4 X5 X6 X7 X8, wherein X1 = A or E or a conservative substitution thereof, X2 = S or Absent or a conservative substitution thereof, X3 = Absent or L or a conservative substitution thereof, X4 = L or N or M or S or a conservative substitution thereof, X5 = N or G or K or L or a conservative substitution thereof, X6 = G or H or N or a conservative substitution thereof, X7 = V or P or G or a conservative substitution thereof, X8 = V or P or a conservative substitution thereof. |
| | | | |
| VL-CONSENSUS-41 TABLE 121 | SEQ ID NO: 50195 | VL1 | Ser Gly Ser X1 Ser Asn Ile Gly X2 X3 X4 Val X5, wherein X1 = S or N or C or a conservative substitution thereof, X2 = S or N or a conservative substitution thereof, X3 = N or H or a conservative substitution thereof, X4 = I or T or a conservative substitution thereof, X5 = T or N or a conservative substitution thereof. |
| | SEQ ID NO: 50196 | VL2 | X1 Asn X2 Gln Arg Pro Ser, wherein X1 = S or G or N or V or a conservative substitution thereof, X2 = D or K or N or Y or a conservative substitution thereof. |
| | SEQ ID NO: | VL3 | X1 X2 Trp Asp Asp Ser Leu X3 X4 Trp Val, wherein |

**FIGURE 55**

| | | | |
|---|---|---|---|
| | | | A or T or V or a conservative substitution thereof, X3 = N or I or S or a conservative substitution thereof, X4 = G or D or V or a conservative substitution thereof. |
| | | | |
| VL-CONSENSUS-42 TABLE 122 | SEQ ID NO: 50198 | VL1 | Arg X1 Ser Gln X2 X3 Arg X4 Asp Leu Gly, wherein X1 = A or T or a conservative substitution thereof, X2 = G or D or R or a conservative substitution thereof, X3 = I or V or a conservative substitution thereof, X4 = N or S or a conservative substitution thereof. |
| | SEQ ID NO: 50199 | VL2 | X1 Ala Ser X2 Leu X3 Ser, wherein X1 = A or D or I or T or a conservative substitution thereof, X2 = S or N or a conservative substitution thereof, X3 = Q or E or F or L or a conservative substitution thereof. |
| | SEQ ID NO: 50200 | VL3 | X1 X2 X3 X4 X5 X6 X7 Pro X8 Thr wherein, X1 = L or I or a conservative substitution thereof, X2 = Q or H or a conservative substitution thereof, X3 = H or Y or a conservative substitution thereof, X4 = N or H or S or a conservative substitution thereof, X5 = S or N or a conservative substitution thereof, X6 = Absent or Y or a conservative substitution thereof, X7 = Y or L or F or P or a conservative substitution thereof, X8 = I or P or L or a conservative substitution thereof. |
| | | | |
| VL-CONSENSUS-43 TABLE 123 | SEQ ID NO: 50201 | VL1 | Arg X1 Ser Gln X2 X3 X4 X5 Tyr X6 X7, wherein X1 = A or T or a conservative substitution thereof, X2 = S or N or Y or a conservative substitution thereof, X3 = I or S or F or a conservative substitution thereof, X4 = S or F or N or R or T or a conservative substitution thereof, X5 = S or D or G or R or a conservative substitution thereof, X6 = L or S or a conservative substitution thereof, X7 = N or S or a conservative substitution thereof. |
| | SEQ ID NO: 50202 | VL2 | X1 X2 X3 X4 X5 X6 X7, wherein X1 = A or D or G or S or a conservative substitution thereof, X2 = A or T or a conservative substitution thereof, X3 = S or Y or a conservative substitution thereof, X4 = S or T or a conservative substitution thereof, X5 = L or F or a conservative substitution thereof, X6 = Q or E or K or a conservative substitution thereof, X7 = S or T or a conservative substitution thereof. |
| | SEQ ID NO: 50203 | VL3 | X1 X2 X3 X4 X5 X6 X7 X8 X9, wherein X1 = Q or H or a conservative substitution thereof, X2 = Q or E or a conservative substitution thereof, X3 = S or T or a conservative substitution thereof, X4 = Y or F or a conservative substitution thereof, X5 = S or G or N or a conservative substitution thereof, X6 = I or T or L or N or S or a conservative substitution thereof, X7 = P or S or R or T or a conservative substitution thereof, X8 = I or F or P or a conservative substitution thereof, X9 = T |

**FIGURE 55**

| | | | |
|---|---|---|---|
| VL-CONSENSUS-44 TABLE 124 | SEQ ID NO: 50204 | VL1 | X1 X2 X3 Gln X4 X5 X6 His X7 X8 Gly X9 Thr Tyr Leu Tyr, wherein X1 = K or R or a conservative substitution thereof, X2 = S or T or a conservative substitution thereof, X3 = S or N or a conservative substitution thereof, X4 = S or I or a conservative substitution thereof, X5 = L or F or a conservative substitution thereof, X6 = L or V or a conservative substitution thereof, X7 = S or N or R or a conservative substitution thereof, X8 = E or D or a conservative substitution thereof, X9 = K or R or a conservative substitution thereof. |
| | SEQ ID NO: 50205 | VL2 | Glu X1 Ser X2 Arg X3 Ser, wherein X1 = V or L or a conservative substitution thereof, X2 = N or K or H or a conservative substitution thereof, X3 = F or L or V or a conservative substitution thereof. |
| | SEQ ID NO: 50246 | VL3 | X1 Gln Ser X2 X3 X4 X5 Ile X6, wherein X1 = M or I or L or a conservative substitution thereof, X2 = I or M or a conservative substitution thereof, X3 = Q or Absent or L or a conservative substitution thereof, X4 = L or Y or I or Q or a conservative substitution thereof, X5 = P or L or a conservative substitution thereof, X6 = T or I or a conservative substitution thereof. |
| | | | |
| VL-CONSENSUS-45 TABLE 125 | SEQ ID NO: 50206 | VL1 | Arg Ala Ser X1 X2 X3 X4 X5 X6 X7 Leu Ala, wherein X1 = Q or R or a conservative substitution thereof, X2 = S or G or N or a conservative substitution thereof, X3 = V or I or a conservative substitution thereof, X4 = I or S or G or a conservative substitution thereof, X5 = S or N or a conservative substitution thereof, X6 = S or I or N or a conservative substitution thereof, X7 = Y or F or a conservative substitution thereof. |
| | SEQ ID NO: 50207 | VL2 | Gly X1 Ser X2 X3 Ala Thr, wherein X1 = V or A or T or a conservative substitution thereof, X2 = S or N or T or a conservative substitution thereof, X3 = R or W or a conservative substitution thereof. |
| | SEQ ID NO: 50208 | VL3 | X1 X2 X3 X4 X5 Ser X6 X7 Asn, wherein X1 = Q or H or a conservative substitution thereof, X2 = Q or H or a conservative substitution thereof, X3 = Y or N or a conservative substitution thereof, X4 = G or D or a conservative substitution thereof, X5 = R or Absent or N or Y or a conservative substitution thereof, X6 = P or L or M or a conservative substitution thereof, X7 = N or T or a conservative substitution thereof. |
| | | | |
| VL-CONSENSUS-46 TABLE 126 | SEQ ID NO: 50209 | VL1 | Arg X1 Ser Gln X2 X3 X4 X5 X6 X7 Ala, wherein X1 = A or S or a conservative substitution thereof, X2 = S or D or T or a conservative substitution thereof, X3 = V or I or a conservative substitution thereof, X4 = S or N |

**FIGURE 55**

| | | | |
|---|---|---|---|
| | | | or I or T or a conservative substitution thereof, X6 = N or Y or a conservative substitution thereof, X7 = L or I or a conservative substitution thereof. |
| | SEQ ID NO: 50210 | VL2 | Gly Ala Ser Thr Arg Ala X1, wherein X1 = T or S or a conservative substitution thereof. |
| | SEQ ID NO: 50211 | VL3 | Gln X1 X2 X3 X4 X5 X6 X7 X8 X9, wherein X1 = Q or E or a conservative substitution thereof, X2 = Y or S or a conservative substitution thereof, X3 = N or D or F or H or a conservative substitution thereof, X4 = D or N or Absent or T or a conservative substitution thereof, X5 = Absent or W or a conservative substitution thereof, X6 = W or P or C or N or a conservative substitution thereof, X7 = P or L or W or a conservative substitution thereof, X8 = W or L or C or P or R or a conservative substitution thereof, X9 = T or P or S or a conservative substitution thereof. |
| | | | |
| VL-CONSENSUS-47 TABLE 127 | SEQ ID NO: 50256 | VL1 | Arg Ala Ser Gln X1 Ile Gly X2 X3 Leu His, wherein X1 = S or N or a conservative substitution thereof, X2 = S or N or a conservative substitution thereof, X3 = S or N or T or a conservative substitution thereof. |
| | SEQ ID NO: 50257 | VL2 | X1 Ala Ser Gln Ser Phe Ser, wherein X1 = Y or S or a conservative substitution thereof. |
| | SEQ ID NO: 50258 | VL3 | X1 Gln Ser X2 Ser X3 Pro X4 Thr, wherein X1 = H or Q or a conservative substitution thereof, X2 = S or G or R or a conservative substitution thereof, X3 = L or F or a conservative substitution thereof, X4 = W or R or Q or a conservative substitution thereof. |
| | | | |
| VL-CONSENSUS-48 TABLE 128 | SEQ ID NO: 50247 | VL1 | Arg Ala Ser Gln X1 Ile X2 X3 Asp Leu Gly, wherein X1 = G or A or a conservative substitution thereof, X2 = R or G or a conservative substitution thereof, X3 = N or D or a conservative substitution thereof. |
| | SEQ ID NO: 50248 | VL2 | Ala X1 Ser Ser Leu Gln Ser, wherein X1 = A or T or a conservative substitution thereof. |
| | SEQ ID NO: 50249 | VL3 | Leu Gln His X1 X2 X3 Pro X4 Ser, wherein X1 = Y or N or a conservative substitution thereof, X2 = S or N or a conservative substitution thereof, X3 = Y or F or a conservative substitution thereof, X4 = R or Y or a conservative substitution thereof. |
| | | | |
| VL-CONSENSUS-49 TABLE 129 | SEQ ID NO: 50212 | VL1 | X1 Ala Ser Gln X2 Ile X3 X4 X5 Leu X6, wherein X1 = R or Q or a conservative substitution thereof, X2 = G or D or a conservative substitution thereof, X3 = S or N or a conservative substitution thereof, X4 = N or K or a conservative substitution thereof, X5 = Y or F or a conservative substitution thereof, X6 = A or N or V or a conservative substitution thereof. |

**FIGURE 55**

| | | | |
|---|---|---|---|
| | SEQ ID NO: 50213 | VL2 | X1 Ala X2 X3 Leu X4 X5, wherein X1 = A or D or G or T or a conservative substitution thereof, X2 = S or T or a conservative substitution thereof, X3 = S or R or N or a conservative substitution thereof, X4 = Q or H or L or V or a conservative substitution thereof, X5 = S or T or a conservative substitution thereof. |
| | SEQ ID NO: 50214 | VL3 | X1 X2 Tyr X3 X4 X5 Pro X6 Thr, wherein X1 = Q or H or L or a conservative substitution thereof, X2 = Q or H or L or a conservative substitution thereof, X3 = L or H or D or K or N or Y or a conservative substitution thereof, X4 = S or N or H or T or a conservative substitution thereof, X5 = Y or L or a conservative substitution thereof, X6 = I or L or a conservative substitution thereof. |
| | | | |
| VL-CONSENSUS-50 TABLE 130 | SEQ ID NO: 50215 | VL1 | X1 Ala X2 Gln Ser X3 Gly Ser Ser Leu His, wherein X1 = S or N or a conservative substitution thereof, X2 = S or N or a conservative substitution thereof, X3 = S or R or a conservative substitution thereof. |
| | SEQ ID NO: 50216 | VL2 | Tyr Ala Ser Gln Ser X1 Ser, wherein X1 = F or L or a conservative substitution thereof. |
| | SEQ ID NO: 50217 | VL3 | His Gln X1 X2 X3 Leu Pro Leu Thr, wherein X1 = S or T or a conservative substitution thereof, X2 = R or G or S or a conservative substitution thereof, X3 = R or S or T or a conservative substitution thereof. |
| | | | |
| VL-CONSENSUS-51 TABLE 131 | SEQ ID NO: 50218 | VL1 | Gln Gly Asp X1 Leu Arg X2 Tyr Tyr X3 X4, wherein X1 = S or T or K or a conservative substitution thereof, X2 = P or N or S or T or a conservative substitution thereof, X3 = A or V or a conservative substitution thereof, X4 = S or N or a conservative substitution thereof. |
| | SEQ ID NO: 50219 | VL2 | X1 Lys Asn X2 Arg Pro Ser, wherein X1 = G or A or T or a conservative substitution thereof, X2 = N or S or a conservative substitution thereof. |
| | SEQ ID NO: 50220 | VL3 | Asn Ser Arg Asp Ser X1 X2 X3 X4 X5 X6 X7, wherein X1 = S or C or a conservative substitution thereof, X2 = G or Absent or a conservative substitution thereof, X3 = N or G or a conservative substitution thereof, X4 = H or N or S or a conservative substitution thereof, X5 = L or H or a conservative substitution thereof, X6 = V or L or a conservative substitution thereof, X7 = V or L or a conservative substitution thereof. |
| | | | |
| VL-CONSENSUS-52 TABLE 132 | (SEQ ID NO: 50221) | VL1 | Arg Ser X1 Gln Ser Leu Val Tyr Ser Asp Gly Asn Thr X2 Leu Asn wherein X1 = S or G or a conservative substitution thereof, X2 = Y or S or a conservative substitution thereof. |
| | SEQ ID NO: | VL2 | X1 Val Ser X2 Trp Asp X3, wherein X1 = K or E or a |

**FIGURE 55**

| | | | |
|---|---|---|---|
| | | | conservative substitution thereof, X3 = S or Y or a conservative substitution thereof. |
| | SEQ ID NO: 50223 | VL3 | Met Gln Gly X1 X2 X3 X4 X5 Thr, wherein X1 = T or I or a conservative substitution thereof, X2 = H or Absent or a conservative substitution thereof, X3 = W or H or a conservative substitution thereof, X4 = P or L or S or W or a conservative substitution thereof, X5 = L or P or a conservative substitution thereof. |
| | | | |
| VL-CONSENSUS-53 TABLE 133 | SEQ ID NO: 50224 | VL1 | Arg X1 Ser Gln Ser X2 X3 X4 X5 X6 Ala, wherein X1 = A or P or T or a conservative substitution thereof, X2 = V or F or a conservative substitution thereof, X3 = S or R or W or a conservative substitution thereof, X4 = R or I or S or a conservative substitution thereof, X5 = N or D or S or a conservative substitution thereof, X6 = L or V or a conservative substitution thereof. |
| | SEQ ID NO: 50225 | VL2 | X1 Ala X2 X3 Arg Ala Thr, wherein X1 = G or D or a conservative substitution thereof, X2 = S or A or a conservative substitution thereof, X3 = T or I or A or a conservative substitution thereof. |
| | SEQ ID NO: 50226 | VL3 | Gln Gln Tyr X1 X2 X3 X4 Pro Leu Thr, wherein X1 = N or Y or a conservative substitution thereof, X2 = N or T or Y or a conservative substitution thereof, X3 = Absent or W or a conservative substitution thereof, X4 = W or P or a conservative substitution thereof. |
| | | | |
| VL-CONSENSUS-54 TABLE 134 | SEQ ID NO: 50250 | VL1 | Gly Gly X1 Asn Ile X2 X3 X4 X5 Val His, wherein X1 = N or D or a conservative substitution thereof, X2 = G or R or a conservative substitution thereof, X3 = R or S or a conservative substitution thereof, X4 = K or R or a conservative substitution thereof, X5 = N or A or a conservative substitution thereof. |
| | SEQ ID NO: 50251 | VL2 | X1 Asp X2 X3 Arg X4 Ser, wherein X1 = R or S or a conservative substitution thereof, X2 = S or R or a conservative substitution thereof, X3 = D or N or Y or a conservative substitution thereof, X4 = P or S or a conservative substitution thereof. |
| | SEQ ID NO: 50252 | VL3 | Gln X1 Trp Asp Ser X2 X3 X4 X5 X6 Val, wherein X1 = V or D or a conservative substitution thereof, X2 = Absent or S or a conservative substitution thereof, X3 = Absent or S or a conservative substitution thereof, X4 = S or D or G or a conservative substitution thereof, X5 = T or H or a conservative substitution thereof, X6 = V or A or G or a conservative substitution thereof. |

FIGURE 56

Table 21

| Reference # | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|
| | | | | | | | | | | | | | | | | H_ |
| CONSENSUS | Q | V | Q | L | V | Q | S | - | G | A | E | V | K | K | P | G |
| 21-225_30A11_HC | . | . | . | . | . | . | . | . | . | . | . | . | . | . | . | . |
| 21-225_34F3_HC | . | . | . | . | . | . | . | . | . | . | . | . | . | . | . | . |
| 21-225_35C6_HC | . | . | . | . | . | . | . | . | . | T | . | . | . | . | . | . |
| 21-225_36H5_HC | . | . | . | . | . | . | . | . | . | . | . | . | . | . | . | . |
| 21-225_35G6_HC | . | . | . | . | . | . | . | . | . | . | D | . | . | . | . | . |
| 21-225_33G1_HC | . | . | . | . | A | . | . | . | . | . | . | . | . | . | . | . |
| 21-225_34G7_HC | . | . | . | . | . | . | . | . | . | . | . | . | . | . | . | . |
| 21-225_33H7_HC | . | . | . | . | . | . | . | . | . | . | D | . | . | . | . | . |
| 21-225_35H12_HC | . | . | . | . | . | . | . | . | . | . | . | . | . | . | . | . |
| 21-225_4H6_HC | . | . | . | . | . | . | . | . | . | . | . | . | . | . | . | . |
| 21-225_18D4_HC | . | . | . | . | . | . | . | . | . | . | . | . | . | . | . | . |
| 21-225_23B11_HC | . | . | . | . | . | . | . | . | . | . | . | . | . | . | . | . |
| 21-225_31C4_HC | . | . | . | . | . | . | . | . | . | T | . | . | . | . | . | . |

| Reference # | 17 | 18 | 19 | 20 | 21 | 22 | 23 | 24 | 25 | 26 | 27 | 28 | 29 | 30 | 31 | 32 |
|---|---|---|---|---|---|---|---|---|---|---|---|---|---|---|---|---|
| | R1 | | | | | | | | | | | | | | | |
| CONSENSUS | A | S | V | K | V | S | C | K | A | S | G | - | Y | T | F | T |
| 21-225_30A11_HC | . | . | . | . | . | . | . | . | . | . | . | . | . | . | . | . |
| 21-225_34F3_HC | . | . | . | . | . | . | . | . | . | . | . | . | . | . | . | . |
| 21-225_35C6_HC | . | . | . | . | . | . | . | . | . | . | . | . | . | . | . | . |
| 21-225_36H5_HC | . | . | M | . | . | . | . | . | . | . | . | . | . | . | . | . |
| 21-225_35G6_HC | . | . | . | . | . | . | . | . | . | . | . | . | . | . | . | . |
| 21-225_33G1_HC | . | . | . | . | . | . | . | . | . | . | . | . | . | . | . | . |
| 21-225_34G7_HC | . | . | . | . | . | . | . | R | . | . | . | . | . | . | . | . |
| 21-225_33H7_HC | . | . | . | . | . | . | . | . | . | . | . | . | . | . | . | . |
| 21-225_35H12_HC | . | . | . | . | . | . | . | . | . | . | . | . | . | . | . | . |
| 21-225_4H6_HC | . | . | . | . | . | . | . | . | . | . | . | . | . | . | . | . |
| 21-225_18D4_HC | . | . | . | . | . | . | . | . | . | . | . | . | . | . | . | . |
| 21-225_23B11_HC | . | . | . | . | . | . | . | . | . | . | . | . | . | . | . | . |
| 21-225_31C4_HC | . | . | . | . | . | . | . | . | . | . | . | . | . | . | . | . |

| Reference # | 33 | 34 | 35 | 36 | 37 | 38 | 39 | 40 | 41 | 42 | 43 | 44 | 45 | 46 | 47 | 48 |
|---|---|---|---|---|---|---|---|---|---|---|---|---|---|---|---|---|
| | H_CDR1 | | | | | | | | | | | | | | | |
| CONSENSUS | D | - | - | - | - | - | Y | Y | M | H | W | V | R | Q | A | P |
| 21-225_30A11_HC | . | . | . | . | . | . | . | H | . | . | . | . | . | . | . | . |
| 21-225_34F3_HC | G | . | . | . | . | . | . | H | . | . | . | . | . | . | . | . |
| 21-225_35C6_HC | G | . | . | . | . | . | D | . | . | . | . | . | . | . | . | . |
| 21-225_36H5_HC | . | . | . | . | . | . | . | H | . | . | . | . | . | . | . | . |
| 21-225_35G6_HC | . | . | . | . | . | . | . | H | I | . | . | . | . | . | . | . |
| 21-225_33G1_HC | G | . | . | . | . | . | . | . | . | . | . | . | . | . | . | . |
| 21-225_34G7_HC | . | . | . | . | . | . | . | H | I | . | . | . | . | . | . | . |
| 21-225_33H7_HC | . | . | . | . | . | . | . | H | I | . | . | . | . | . | . | . |
| 21-225_35H12_HC | . | . | . | . | . | . | . | . | I | . | . | . | . | . | . | . |
| 21-225_4H6_HC | . | . | . | . | . | . | . | . | L | . | . | . | . | . | . | . |
| 21-225_18D4_HC | G | . | . | . | . | . | . | . | L | . | . | . | . | . | . | . |
| 21-225_23B11_HC | G | . | . | . | . | . | . | . | L | . | . | . | . | . | . | . |

FIGURE 56

| Reference # | 49 | 50 | 51 | 52 | 53 | 54 | 55 | 56 | 57 | 58 | 59 | 60 | 61 | 62 | 63 | 64 |
|---|---|---|---|---|---|---|---|---|---|---|---|---|---|---|---|---|
| | H_FR2 | | | | | | | | | | | | | | | |
| CONSENSUS | G | Q | G | L | E | W | M | G | W | I | N | P | N | - | - | - |
| 21-225_30A11_HC | . | . | . | . | . | . | . | . | . | . | . | . | . | . | . | . |
| 21-225_34F3_HC | . | . | . | . | D | . | . | . | . | . | . | . | . | . | . | . |
| 21-225_35C6_HC | . | . | . | . | . | . | . | . | . | . | S | . | K | . | . | . |
| 21-225_36H5_HC | . | . | . | . | . | . | . | . | . | . | . | . | . | . | . | . |
| 21-225_35G6_HC | . | . | . | . | . | . | . | . | . | . | . | . | . | . | . | . |
| 21-225_33G1_HC | . | . | . | . | . | . | . | . | . | . | S | . | . | . | . | . |
| 21-225_34G7_HC | . | . | . | . | . | . | . | . | . | . | . | . | . | . | . | . |
| 21-225_33H7_HC | . | . | . | . | . | . | . | . | . | . | . | . | . | . | . | . |
| 21-225_35H12_HC | . | . | . | . | . | . | . | . | . | . | . | . | . | . | . | . |
| 21-225_4H6_HC | . | . | . | . | . | . | . | . | . | . | H | . | . | . | . | . |
| 21-225_18D4_HC | . | . | . | . | . | . | L | . | . | . | . | . | . | . | . | . |
| 21-225_23B11_HC | . | . | . | . | . | . | . | . | . | . | . | . | . | . | . | . |
| 21-225_31C4_HC | . | . | . | . | . | . | . | . | . | . | S | . | K | . | . | . |

| Reference # | 65 | 66 | 67 | 68 | 69 | 70 | 71 | 72 | 73 | 74 | 75 | 76 | 77 | 78 | 79 | 80 |
|---|---|---|---|---|---|---|---|---|---|---|---|---|---|---|---|---|
| | | H_CDR2 | | | | | | | | | | | | | | |
| CONSENSUS | N | G | G | T | N | Y | A | Q | K | F | Q | G | R | V | T | M |
| 21-225_30A11_HC | [illegible] | . | . | . | . | . | . | . | . | . | . | D | . | . | . | . |
| 21-225_34F3_HC | S | . | . | . | . | . | . | . | . | . | . | . | . | . | . | . |
| 21-225_35C6_HC | . | . | . | . | . | . | . | . | . | . | . | . | . | . | . | . |
| 21-225_36H5_HC | [illegible] | . | . | . | . | . | . | . | . | . | . | D | . | . | . | . |
| 21-225_35G8_HC | . | . | . | . | H | . | . | . | [illegible] | . | . | . | . | . | . | . |
| 21-225_33G1_HC | . | . | . | . | . | . | . | . | . | . | . | . | . | . | . | . |
| 21-225_34G7_HC | . | . | . | . | H | . | . | . | . | . | . | . | . | . | . | . |
| 21-225_33H7_HC | . | . | . | . | H | . | . | . | E | . | . | . | . | . | . | . |
| 21-225_35H12_HC | [illegible] | . | . | . | . | . | . | . | . | . | . | . | . | . | . | . |
| 21-225_4H6_HC | S | . | . | . | . | . | . | . | . | . | . | . | . | . | . | . |
| 21-225_18D4_HC | S | N | . | . | I | S | . | . | . | . | . | . | . | . | . | . |
| 21-225_23B11_HC | . | N | . | . | . | . | . | . | . | . | . | . | . | . | . | . |
| 21-225_31C4_HC | . | . | . | . | . | . | . | . | . | . | . | . | . | . | . | . |

| Reference # | 81 | 82 | 83 | 84 | 85 | 86 | 87 | 88 | 89 | 90 | 91 | 92 | 93 | 94 | 95 | 96 |
|---|---|---|---|---|---|---|---|---|---|---|---|---|---|---|---|---|
| | | | | | | | | | | | | H_FR3 | | | | |
| CONSENSUS | T | R | D | T | S | I | S | T | A | Y | M | E | L | S | R | L |
| 21-225_30A11_HC | . | . | . | . | . | . | . | . | . | . | . | . | . | . | . | . |
| 21-225_34F3_HC | . | . | . | . | . | . | . | . | . | . | . | . | . | . | . | . |
| 21-225_35C6_HC | . | . | . | . | . | . | . | . | . | . | . | . | . | N | . | . |
| 21-225_36H5_HC | . | . | . | . | . | . | . | . | . | . | . | . | . | . | . | . |
| 21-225_35G6_HC | . | . | . | . | . | . | . | . | . | . | . | . | . | . | S | . |
| 21-225_33G1_HC | . | . | . | . | . | . | . | . | . | . | . | . | . | . | . | . |
| 21-225_34G7_HC | . | . | . | . | . | . | . | . | . | . | . | . | . | . | . | . |
| 21-225_33H7_HC | . | . | . | . | . | . | . | . | . | . | . | . | . | . | S | . |
| 21-225_35H12_HC | . | . | . | . | . | . | . | . | . | . | . | . | . | . | . | . |
| 21-225_4H6_HC | . | . | . | . | . | . | . | . | . | . | . | G | . | . | S | . |
| 21-225_18D4_HC | . | . | . | . | . | . | . | . | . | . | . | . | . | . | . | . |
| 21-225_23B11_HC | | | | | | | | | | | | | | | | |

FIGURE 56

| Reference # | 97 | 98 | 99 | 100 | 101 | 102 | 103 | 104 | 105 | 106 | 107 | 108 | 109 | 110 | 111 | 112 |
|---|---|---|---|---|---|---|---|---|---|---|---|---|---|---|---|---|
| CONSENSUS | R | S | D | D | T | A | V | Y | Y | C | A | R | D | G | T | G |
| 21-225_30A11_HC | . | . | . | . | . | . | . | . | F | . | . | . | . | . | . | . |
| 21-225_34F3_HC | . | . | . | . | . | . | . | . | . | . | . | . | . | . | . | S |
| 21-225_35C6_HC | . | . | . | . | . | . | . | . | . | . | . | . | . | . | . | . |
| 21-225_36H5_HC | . | . | . | . | . | . | . | . | F | . | . | . | . | . | . | . |
| 21-225_35G6_HC | . | . | . | . | . | . | . | . | . | . | . | . | . | . | . | . |
| 21-225_33G1_HC | . | . | . | . | . | . | . | . | . | . | . | . | . | . | . | . |
| 21-225_34G7_HC | . | . | . | . | . | . | . | . | . | . | . | . | . | . | . | . |
| 21-225_33H7_HC | . | . | . | . | . | . | . | . | H | . | . | . | . | . | . | . |
| 21-225_35H12_HC | . | . | . | . | . | . | . | . | . | . | . | . | . | . | . | . |
| 21-225_4H6_HC | . | . | . | . | . | . | . | . | . | . | . | . | . | . | . | S |
| 21-225_18D4_HC | I | . | . | . | . | . | . | . | . | . | . | . | . | . | . | S |
| 21-225_23B11_HC | . | . | . | . | . | . | . | . | . | . | . | . | . | . | . | S |
| 21-225_31C4_HC | . | . | . | . | . | . | . | . | . | . | . | . | . | . | . | . |

H_CDR3

| Reference # | 113 | 114 | 115 | 116 | 117 | 118 | 119 | 120 | 121 | 122 | 123 | 124 | 125 | 126 | 127 | 128 |
|---|---|---|---|---|---|---|---|---|---|---|---|---|---|---|---|---|
| CONSENSUS | - | - | - | - | - | - | - | - | - | - | - | - | - | - | - | - |
| 21-225_30A11_HC | . | . | . | . | . | . | . | . | . | . | . | . | . | . | . | . |
| 21-225_34F3_HC | . | . | . | . | . | . | . | . | . | . | . | . | . | . | . | . |
| 21-225_35C6_HC | . | . | . | . | . | . | . | . | . | . | . | . | . | . | . | . |
| 21-225_36H5_HC | . | . | . | . | . | . | . | . | . | . | . | . | . | . | . | . |
| 21-225_35G6_HC | . | . | . | . | . | . | . | . | . | . | . | . | . | . | . | . |
| 21-225_33G1_HC | . | . | . | . | . | . | . | . | . | . | . | . | . | . | . | . |
| 21-225_34G7_HC | . | . | . | . | . | . | . | . | . | . | . | . | . | . | . | . |
| 21-225_33H7_HC | . | . | . | . | . | . | . | . | . | . | . | . | . | . | . | . |
| 21-225_35H12_HC | . | . | . | . | . | . | . | . | . | . | . | . | . | . | . | . |
| 21-225_4H6_HC | . | . | . | . | . | . | . | . | . | . | . | . | . | . | . | . |
| 21-225_18D4_HC | . | . | . | . | . | . | . | . | . | . | . | . | . | . | . | . |
| 21-225_23B11_HC | . | . | . | . | . | . | . | . | . | . | . | . | . | . | . | . |
| 21-225_31C4_HC | . | . | . | . | . | . | . | . | . | . | . | . | . | . | . | . |

H_FR

| Reference # | 129 | 130 | 131 | 132 | 133 | 134 | 135 | 136 | 137 | 138 | 139 | 140 | 141 | 142 | 143 | 144 |
|---|---|---|---|---|---|---|---|---|---|---|---|---|---|---|---|---|
| CONSENSUS | - | - | - | - | - | - | S | F | D | Y | W | G | Q | G | T | L |
| 21-225_30A11_HC | . | . | . | . | . | . | . | . | . | . | . | . | . | . | . | . |
| 21-225_34F3_HC | . | . | . | . | . | . | . | . | . | . | . | . | . | . | . | . |
| 21-225_35C6_HC | . | . | . | . | . | . | . | . | . | . | . | . | . | . | . | . |
| 21-225_36H5_HC | . | . | . | . | . | . | . | . | . | . | . | . | . | . | . | . |
| 21-225_35G6_HC | . | . | . | . | . | . | . | . | . | . | . | . | . | . | . | . |
| 21-225_33G1_HC | . | . | . | . | . | . | . | . | . | . | . | . | . | . | . | . |
| 21-225_34G7_HC | . | . | . | . | . | . | . | . | . | . | . | . | . | . | N | . |
| 21-225_33H7_HC | . | . | . | . | . | . | . | . | . | . | . | . | . | . | . | . |
| 21-225_35H12_HC | . | . | . | . | . | . | . | . | . | . | . | . | . | . | . | . |
| 21-225_4H6_HC | . | . | . | . | . | . | . | . | . | . | . | . | . | . | . | . |
| 21-225_18D4_HC | . | . | . | . | . | . | . | L | . | . | . | . | . | . | . | . |
| 21-225_23B11_HC | . | . | . | . | . | . | . | . | . | . | . | . | . | . | . | . |

FIGURE 56

| Reference # | 145 | 146 | 147 | 148 | 149 |
|---|---|---|---|---|---|
| CONSENSUS | V | T | V | S | S |
| 21-225_30A11_HC | . | . | . | . | . |
| 21-225_34F3_HC | . | . | . | . | . |
| 21-225_35C6_HC | . | . | . | . | . |
| 21-225_36H5_HC | . | . | . | . | . |
| 21-225_35G6_HC | . | . | . | . | . |
| 21-225_33G1_HC | . | . | . | . | . |
| 21-225_34G7_HC | . | . | . | . | . |
| 21-225_33H7_HC | . | . | . | . | . |
| 21-225_35H12_HC | . | . | . | . | . |
| 21-225_4H6_HC | . | . | . | . | . |
| 21-225_18D4_HC | . | . | . | . | . |
| 21-225_23B11_HC | . | . | . | . | . |
| 21-225_31C4_HC | . | . | . | . | . |

FIGURE 56

Table 22

| Reference # | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|
| | | | | | | | | | | | | | | | | H |
| CONSENSUS | Q | V | Q | L | V | Q | S | - | G | A | E | V | K | K | P | G |
| 21-225_31D12_HC | . | . | K | . | . | . | . | . | . | . | . | . | . | . | . | . |
| 21-225_18E10_HC | . | . | . | . | . | . | . | . | . | . | . | . | . | . | . | . |
| 21-225_4B3_HC | . | . | . | . | . | . | . | . | . | . | . | . | . | . | . | . |
| 21-225_35E1_HC | . | . | K | . | . | . | . | . | . | . | . | . | . | . | . | . |
| 21-225_13F6_HC | . | . | . | . | . | . | . | . | . | . | . | . | . | . | . | . |
| 21-225_16F3_HC | . | . | . | . | . | . | . | . | . | . | . | . | . | . | . | . |
| 21-225_17D3_HC | . | . | . | . | . | . | . | . | . | . | . | . | . | . | . | . |
| 21-225_33E6_HC | . | . | K | . | . | . | . | . | . | . | . | . | . | . | . | . |
| 21-225_9G9_HC | . | . | . | . | . | . | . | . | . | . | . | . | . | . | . | . |
| 21-225_19D1_HC | . | . | . | . | . | . | . | . | . | . | . | . | . | . | . | . |
| 21-225_21D12_HC | . | . | . | . | . | . | . | . | . | . | . | . | . | . | . | . |

| Reference # | 17 | 18 | 19 | 20 | 21 | 22 | 23 | 24 | 25 | 26 | 27 | 28 | 29 | 30 | 31 | 32 |
|---|---|---|---|---|---|---|---|---|---|---|---|---|---|---|---|---|
| | R1 | | | | | | | | | | | | | | | |
| CONSENSUS | A | S | V | K | V | S | C | K | A | S | G | - | Y | T | F | T |
| 21-225_31D12_HC | . | . | . | . | . | . | . | . | . | . | . | . | . | . | . | . |
| 21-225_18E10_HC | . | . | . | . | . | . | . | . | . | . | . | . | . | . | . | . |
| 21-225_4B3_HC | . | . | . | . | . | . | . | . | . | . | . | . | . | . | . | . |
| 21-225_35E1_HC | . | . | . | . | . | . | . | . | . | . | . | . | . | . | . | . |
| 21-225_13F6_HC | . | . | . | . | . | . | . | . | . | . | . | . | . | . | . | . |
| 21-225_16F3_HC | . | . | . | . | . | . | . | . | . | . | . | . | . | . | . | . |
| 21-225_17D3_HC | . | . | . | . | . | . | . | . | . | . | . | . | . | . | . | . |
| 21-225_33E6_HC | . | . | . | . | . | . | . | . | . | . | . | . | . | . | . | . |
| 21-225_9G9_HC | . | . | . | . | . | . | . | . | . | . | . | . | . | . | . | . |
| 21-225_19D1_HC | . | . | . | . | . | . | . | . | . | . | . | . | . | . | . | . |
| 21-225_21D12_HC | . | . | . | . | . | . | . | . | . | . | . | . | . | . | . | . |

| Reference # | 33 | 34 | 35 | 36 | 37 | 38 | 39 | 40 | 41 | 42 | 43 | 44 | 45 | 46 | 47 | 48 |
|---|---|---|---|---|---|---|---|---|---|---|---|---|---|---|---|---|
| | | | | | H_CDR1 | | | | | | | | | | | |
| CONSENSUS | G | - | - | - | - | - | Y | Y | M | H | W | V | R | Q | A | P |
| 21-225_31D12_HC | . | . | . | . | . | . | . | . | . | . | . | . | . | . | . | . |
| 21-225_18E10_HC | . | . | . | . | . | . | . | . | . | . | . | . | . | . | . | . |
| 21-225_4B3_HC | . | . | . | . | . | . | . | . | . | . | . | . | . | . | . | . |
| 21-225_35E1_HC | . | . | . | . | . | . | . | . | . | . | . | . | . | . | . | . |
| 21-225_13F6_HC | . | . | . | . | . | . | . | . | . | . | . | . | . | . | . | . |
| 21-225_16F3_HC | . | . | . | . | . | . | . | . | . | . | . | . | . | . | . | . |
| 21-225_17D3_HC | . | . | . | . | . | . | . | . | . | . | . | . | . | . | . | . |
| 21-225_33E6_HC | . | . | . | . | . | . | . | . | . | . | . | . | . | . | . | . |
| 21-225_9G9_HC | . | . | . | . | . | . | . | . | I | . | . | . | . | . | . | . |
| 21-225_19D1_HC | . | . | . | . | . | . | . | . | . | . | . | . | . | . | . | . |
| 21-225_21D12_HC | . | | | | | | | | . | | | | | | | |

FIGURE 56

| Reference # | 49 | 50 | 51 | 52 | 53 | 54 | 55 | 56 | 57 | 58 | 59 | 60 | 61 | 62 | 63 | 64 |
|---|---|---|---|---|---|---|---|---|---|---|---|---|---|---|---|---|
| | H_FR2 | | | | | | | | | | | | | | | |
| CONSENSUS | G | Q | G | L | E | W | M | G | W | I | N | P | N | - | - | - |
| 21-225_31D12_HC | . | . | . | . | . | . | . | . | . | . | . | . | . | . | . | . |
| 21-225_18E10_HC | . | . | . | . | . | . | . | . | . | . | . | . | . | . | . | . |
| 21-225_4B3_HC | . | . | . | . | . | . | . | . | . | . | . | . | . | . | . | . |
| 21-225_35E1_HC | . | . | . | . | . | . | . | . | . | . | . | . | K | . | . | . |
| 21-225_13F6_HC | . | . | . | . | . | . | . | . | . | . | . | . | . | . | . | . |
| 21-225_16F3_HC | . | . | . | . | . | . | . | . | . | . | . | . | . | . | . | . |
| 21-225_17D3_HC | . | . | . | . | . | . | . | . | . | . | . | . | . | . | . | . |
| 21-225_33E6_HC | . | . | . | . | . | . | . | . | . | . | . | . | . | . | . | . |
| 21-225_9G9_HC | . | . | . | . | . | . | . | . | . | . | . | . | . | . | . | . |
| 21-225_19D1_HC | . | . | . | . | . | . | . | . | . | . | . | . | . | . | . | . |
| 21-225_21D12_HC | . | . | . | . | . | . | . | . | . | . | . | . | . | . | . | . |

| Reference # | 65 | 66 | 67 | 68 | 69 | 70 | 71 | 72 | 73 | 74 | 75 | 76 | 77 | 78 | 79 | 80 |
|---|---|---|---|---|---|---|---|---|---|---|---|---|---|---|---|---|
| | | H_CDR2 | | | | | | | | | | | | | | |
| CONSENSUS | S | G | G | T | N | Y | A | Q | K | F | Q | G | R | V | T | M |
| 21-225_31D12_HC | . | . | . | . | . | . | V | . | . | . | . | . | . | . | . | . |
| 21-225_18E10_HC | . | . | . | . | . | . | . | . | . | . | . | . | . | . | . | . |
| 21-225_4B3_HC | . | . | . | . | . | S | . | . | . | . | . | . | . | . | . | . |
| 21-225_35E1_HC | . | . | . | . | . | . | . | . | . | . | . | . | . | . | . | . |
| 21-225_13F6_HC | . | . | . | A | . | . | . | . | . | . | . | . | . | . | . | . |
| 21-225_16F3_HC | . | . | . | . | . | . | . | . | . | . | . | . | . | . | . | . |
| 21-225_17D3_HC | . | . | . | . | . | . | . | . | . | . | . | . | . | . | . | . |
| 21-225_33E6_HC | . | . | . | . | . | . | . | . | . | . | . | . | . | . | . | . |
| 21-225_9G9_HC | . | . | . | . | D | . | . | . | . | . | . | . | . | . | . | . |
| 21-225_19D1_HC | . | . | . | . | . | . | . | . | . | . | L | . | . | . | . | . |
| 21-225_21D12_HC | . | . | . | . | . | N | . | . | . | . | . | . | . | . | . | . |


| Reference # | 65 | 66 | 67 | 68 | 69 | 70 | 71 | 72 | 73 | 74 | 75 | 76 | 77 | 78 | 79 | 80 |
|---|---|---|---|---|---|---|---|---|---|---|---|---|---|---|---|---|
| | | H_CDR2 | | | | | | | | | | | | | | |
| CONSENSUS | S | G | G | T | N | Y | A | Q | K | F | Q | G | R | V | T | M |
| 21-225_31D12_HC | . | . | . | . | . | . | V | . | . | . | . | . | . | . | . | . |
| 21-225_18E10_HC | . | . | . | . | . | . | . | . | . | . | . | . | . | . | . | . |
| 21-225_4B3_HC | . | . | . | . | . | S | . | . | . | . | . | . | . | . | . | . |
| 21-225_35E1_HC | . | . | . | . | . | . | . | . | . | . | . | . | . | . | . | . |
| 21-225_13F6_HC | . | . | . | A | . | . | . | . | . | . | . | . | . | . | . | . |
| 21-225_16F3_HC | . | . | . | . | . | . | . | . | . | . | . | . | . | . | . | . |
| 21-225_17D3_HC | . | . | . | . | . | . | . | . | . | . | . | . | . | . | . | . |
| 21-225_33E6_HC | . | . | . | . | . | . | . | . | . | . | . | . | . | . | . | . |
| 21-225_9G9_HC | . | . | . | . | D | . | . | . | . | . | . | . | . | . | . | . |
| 21-225_19D1_HC | . | . | . | . | . | . | . | . | . | . | L | . | . | . | . | . |
| 21-225_21D12_HC | . | . | . | . | . | N | . | . | . | . | . | . | . | . | . | . |

FIGURE 56

| Reference # | 97 | 98 | 99 | 100 | 101 | 102 | 103 | 104 | 105 | 106 | 107 | 108 | 109 | 110 | 111 | 112 |
|---|---|---|---|---|---|---|---|---|---|---|---|---|---|---|---|---|
| CONSENSUS | R | S | D | D | T | A | V | Y | Y | C | A | R | S | Y | Y | Y |
| 21-225_31D12_HC | . | . | . | . | . | . | . | . | . | . | . | . | V | . | . | . |
| 21-225_18E10_HC | . | . | . | . | . | . | . | . | . | . | . | . | . | . | . | . |
| 21-225_4B3_HC | . | . | . | . | . | . | . | . | . | . | . | . | . | . | . | . |
| 21-225_35E1_HC | . | . | . | . | . | . | . | . | . | . | . | . | V | . | . | H |
| 21-225_13F6_HC | . | . | . | . | . | . | . | . | F | . | . | . | . | F | . | . |
| 21-225_16F3_HC | . | . | . | . | . | . | . | . | . | . | . | . | . | . | . | . |
| 21-225_17D3_HC | . | . | . | . | . | . | . | . | . | . | . | . | A | N | . | . |
| 21-225_33E6_HC | . | . | . | . | . | . | . | . | . | . | . | . | V | . | . | . |
| 21-225_9G9_HC | . | . | . | . | . | . | . | . | . | . | . | . | . | . | . | . |
| 21-225_19D1_HC | . | . | . | . | . | . | . | . | . | . | . | . | . | . | . | . |
| 21-225_21D12_HC | . | . | . | . | . | . | . | . | S | . | . | . | . | . | . | . |

| Reference # | 113 | 114 | 115 | 116 | 117 | 118 | 119 | 120 | 121 | 122 | 123 | 124 | 125 | 126 | 127 | 128 |
|---|---|---|---|---|---|---|---|---|---|---|---|---|---|---|---|---|
| | | | | | | | | | | H_CDR3 | | | | | | |
| CONSENSUS | G | S | G | S | - | - | - | - | - | - | - | - | - | - | - | - |
| 21-225_31D12_HC | . | . | . | . | . | . | . | . | . | . | . | . | . | . | . | . |
| 21-225_18E10_HC | . | . | . | . | . | . | . | . | . | . | . | . | . | . | . | . |
| 21-225_4B3_HC | . | . | . | . | . | . | . | . | . | . | . | . | . | . | . | . |
| 21-225_35E1_HC | . | . | . | . | . | . | . | . | . | . | . | . | . | . | . | . |
| 21-225_13F6_HC | . | . | . | T | . | . | . | . | . | . | . | . | . | . | . | . |
| 21-225_16F3_HC | . | . | . | T | . | . | . | . | . | . | . | . | . | . | . | . |
| 21-225_17D3_HC | . | . | . | . | . | . | . | . | . | . | . | . | . | . | . | . |
| 21-225_33E6_HC | . | . | . | . | . | . | . | . | . | . | . | . | . | . | . | . |
| 21-225_9G9_HC | . | . | . | T | . | . | . | . | . | . | . | . | . | . | . | . |
| 21-225_19D1_HC | . | . | . | . | . | . | . | . | . | . | . | . | . | . | . | . |
| 21-225_21D12_HC | . | . | . | T | . | . | . | . | . | . | . | . | . | . | . | . |

| Reference # | 129 | 130 | 131 | 132 | 133 | 134 | 135 | 136 | 137 | 138 | 139 | 140 | 141 | 142 | 143 | 144 |
|---|---|---|---|---|---|---|---|---|---|---|---|---|---|---|---|---|
| | | | | | | | | | | | | | | | | H_FR |
| CONSENSUS | - | - | - | Y | Y | N | E | F | D | Y | W | G | Q | G | T | L |
| 21-225_31D12_HC | . | . | . | . | . | . | . | . | . | . | . | . | . | . | . | . |
| 21-225_18E10_HC | . | . | . | . | . | . | . | . | . | . | . | . | . | . | . | . |
| 21-225_4B3_HC | . | . | . | . | . | . | . | . | . | . | . | . | . | . | . | . |
| 21-225_35E1_HC | . | . | . | . | . | . | . | . | . | . | . | . | . | . | . | . |
| 21-225_13F6_HC | . | . | . | . | . | . | . | . | . | . | . | . | . | . | . | . |
| 21-225_16F3_HC | . | . | . | . | . | . | . | . | . | . | . | . | . | . | . | . |
| 21-225_17D3_HC | . | . | . | . | . | . | D | . | . | . | . | . | . | . | . | . |
| 21-225_33E6_HC | . | . | . | . | . | . | . | . | . | . | . | . | . | . | . | . |
| 21-225_9G9_HC | . | . | . | . | . | . | . | . | . | . | . | . | . | . | . | . |
| 21-225_19D1_HC | . | . | . | . | . | . | . | . | . | . | . | . | . | . | . | . |
| 21-225_21D12_HC | . | . | . | . | . | . | . | . | . | . | . | . | . | . | . | . |

FIGURE 56

| Reference # | 145 | 146 | 147 | 148 | 149 |
|---|---|---|---|---|---|
| CONSENSUS | V | T | V | S | S |
| 21-225_31D12_HC | . | . | . | . | . |
| 21-225_18E10_HC | . | . | . | . | . |
| 21-225_4B3_HC | . | . | . | . | . |
| 21-225_35E1_HC | . | . | . | . | . |
| 21-225_13F6_HC | . | . | . | . | . |
| 21-225_16F3_HC | . | . | . | . | . |
| 21-225_17D3_HC | . | . | . | . | . |
| 21-225_33E6_HC | . | . | . | . | . |
| 21-225_9G9_HC | . | . | . | . | . |
| 21-225_19D1_HC | . | . | . | . | . |
| 21-225_21D12_HC | . | . | . | . | . |

**FIGURE 56**

**Table 23**

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | | | | | H_FR1 | |
| CONSENSUS | Q | V | Q | L | V | Q | S | - | G | A | E | V | K | K | P | G | A |
| 21-225_24E1_HC | . | . | . | . | . | . | . | . | . | . | . | . | . | . | . | . | . |
| 21-225_23A12_HC | . | . | . | . | . | . | . | . | . | . | . | . | . | . | . | . | . |
| 21-225_25A4_HC | . | . | L | . | . | . | . | . | . | . | . | . | . | R | . | . | . |
| 21-225_28B6_HC | . | . | L | . | . | . | . | . | . | . | . | . | . | R | . | . | . |
| 21-225_29E6_HC | . | . | L | . | . | . | . | . | . | . | . | . | . | R | . | . | . |
| 21-225_26C4_HC | . | . | . | . | . | . | . | . | . | . | . | . | . | . | . | . | . |
| 21-225_33D1_HC | . | . | . | . | . | . | . | . | . | . | . | . | . | . | . | . | . |
| 21-225_34D2_HC | . | . | . | . | . | . | . | . | . | . | . | . | . | . | . | . | . |
| 21-225_29H8_HC | . | . | . | . | . | . | . | . | . | . | . | . | Q | . | . | . | . |
| 21-225_11E5_HC | . | . | . | . | . | . | . | . | . | P | . | . | . | . | . | . | . |
| 21-225_8B11_HC | . | . | . | . | . | . | . | . | . | . | . | . | . | . | . | . | . |
| 21-225_22D2_HC | . | . | . | . | . | . | . | . | . | . | . | . | . | . | . | . | . |
| 21-225_32A1_HC | . | . | . | . | . | . | . | . | . | . | . | . | . | . | . | . | . |
| 21-225_32A5_HC | . | . | . | . | . | . | . | . | . | . | . | . | . | . | . | . | . |
| 21-225_34H7_HC | . | . | . | . | . | . | . | . | . | . | . | . | . | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | S | V | K | V | S | C | K | A | S | G | - | Y | T | F | T | N | - |
| 21-225_24E1_HC | . | . | . | . | . | . | . | . | . | . | . | . | . | . | . | . | . |
| 21-225_23A12_HC | . | . | . | . | . | . | . | . | . | . | . | . | P | . | . | . | . |
| 21-225_25A4_HC | . | . | . | . | . | . | . | . | . | . | . | . | . | . | . | . | . |
| 21-225_28B6_HC | . | . | . | . | . | . | . | . | . | . | . | . | . | . | . | . | . |
| 21-225_29E6_HC | . | . | . | . | . | . | . | . | . | . | . | . | . | . | . | . | . |
| 21-225_26C4_HC | . | . | . | . | . | . | . | . | . | . | . | . | . | . | . | . | . |
| 21-225_33D1_HC | . | . | . | . | . | . | . | . | . | . | . | . | . | . | . | . | . |
| 21-225_34D2_HC | . | . | . | . | . | . | . | . | . | . | . | . | . | . | . | . | . |
| 21-225_29H8_HC | . | . | . | . | . | . | . | . | . | . | . | . | . | . | . | . | . |
| 21-225_11E5_HC | . | . | . | . | . | . | . | . | . | . | . | . | . | . | . | . | . |
| 21-225_8B11_HC | . | . | . | . | . | . | . | . | . | . | . | . | . | . | . | . | . |
| 21-225_22D2_HC | . | . | . | . | . | . | . | . | . | . | . | . | . | . | I | . | . |
| 21-225_32A1_HC | . | . | . | . | . | . | . | . | . | . | . | . | . | . | . | . | . |
| 21-225_32A5_HC | . | . | . | . | . | . | . | . | . | . | . | . | . | . | . | . | . |
| 21-225_34H7_HC | . | . | . | . | . | . | . | . | . | . | . | . | . | . | . | . | . |

**FIGURE 56**

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | H_CDR1 | | | | | | | | H_FR2 | | | | | | | | |
| CONSENSUS | - | - | - | - | Y | D | I | N | W | V | R | Q | A | T | G | Q | G |
| 21-225_24E1_HC | . | . | . | . | . | . | . | . | . | . | . | . | . | . | . | . | . |
| 21-225_23A12_HC | . | . | . | . | . | . | . | . | . | . | . | . | . | . | . | . | . |
| 21-225_25A4_HC | . | . | . | . | . | . | . | . | . | . | . | . | . | . | . | . | . |
| 21-225_28B6_HC | . | . | . | . | . | . | . | . | . | . | . | . | . | . | . | . | . |
| 21-225_29E6_HC | . | . | . | . | . | . | . | . | . | . | . | . | . | . | . | . | . |
| 21-225_26C4_HC | . | . | . | . | . | . | . | . | . | . | . | . | . | S | . | . | . |
| 21-225_33D1_HC | . | . | . | . | . | . | . | . | . | . | . | . | . | . | . | . | . |
| 21-225_34D2_HC | . | . | . | . | . | . | . | . | . | . | . | . | . | . | . | . | . |
| 21-225_29H8_HC | . | . | . | . | . | . | . | . | . | . | . | . | . | . | . | . | . |
| 21-225_11E5_HC | . | . | . | . | . | . | . | . | . | . | . | . | . | . | . | . | . |
| 21-225_8B11_HC | . | . | . | . | . | . | . | . | . | . | . | . | . | A | . | . | . |
| 21-225_22D2_HC | . | . | . | . | . | . | . | . | . | . | . | . | . | . | . | . | . |
| 21-225_32A1_HC | . | . | . | . | . | . | . | . | . | . | . | . | . | . | . | . | . |
| 21-225_32A5_HC | . | . | . | . | . | . | . | . | . | . | . | . | . | . | . | . | . |
| 21-225_34H7_HC | . | . | . | . | . | . | . | . | . | . | . | . | . | . | . | . | . |

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | H_CDR2 | | | | | | | | | | | |
| CONSENSUS | L | E | W | M | G | W | M | H | P | N | - | - | - | S | G | N | T |
| 21-225_24E1_HC | . | . | . | . | . | . | . | . | . | . | . | . | . | . | . | . | . |
| 21-225_23A12_HC | . | . | . | . | . | . | . | . | . | . | . | . | . | . | . | . | . |
| 21-225_25A4_HC | . | . | . | . | . | . | . | Y | . | . | . | . | . | . | . | . | . |
| 21-225_28B6_HC | . | . | . | . | . | . | . | . | . | D | . | . | . | . | . | . | . |
| 21-225_29E6_HC | . | . | . | . | . | . | . | . | . | . | . | . | . | . | . | . | . |
| 21-225_26C4_HC | . | . | . | . | . | . | . | N | . | . | . | . | . | . | . | . | . |
| 21-225_33D1_HC | . | . | . | . | . | . | . | . | . | . | . | . | . | . | . | . | . |
| 21-225_34D2_HC | . | . | . | V | . | . | L | . | . | . | . | . | . | . | . | T | . |
| 21-225_29H8_HC | . | . | . | . | . | . | . | N | . | . | . | . | . | . | . | . | . |
| 21-225_11E5_HC | . | . | . | . | . | . | . | . | . | . | . | . | . | . | . | A | . |
| 21-225_8B11_HC | . | . | . | . | . | . | . | . | . | . | . | . | . | . | . | . | . |
| 21-225_22D2_HC | . | . | . | . | . | . | . | . | . | D | . | . | . | . | . | . | . |
| 21-225_32A1_HC | . | . | . | . | . | . | . | N | . | . | . | . | . | . | . | . | . |
| 21-225_32A5_HC | . | . | . | . | . | . | . | . | . | . | . | . | . | . | . | . | . |
| 21-225_34H7_HC | . | . | . | . | . | . | . | N | . | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | G | Y | A | Q | K | F | Q | G | R | V | T | M | T | R | N | T | S |
| 21-225_24E1_HC | . | . | . | . | R | . | . | . | . | . | . | . | . | . | . | . | . |
| 21-225_23A12_HC | . | . | . | . | . | . | . | . | . | . | . | . | . | . | . | . | . |
| 21-225_25A4_HC | . | . | . | . | . | . | . | . | . | . | . | . | . | . | . | . | . |
| 21-225_28B6_HC | . | . | . | . | . | . | . | . | . | . | . | . | . | . | . | . | . |
| 21-225_29E6_HC | . | . | . | . | . | . | . | . | . | . | . | . | . | . | . | . | . |
| 21-225_26C4_HC | . | . | . | . | [illegible] | . | . | . | . | . | . | . | . | . | . | . | . |
| 21-225_33D1_HC | . | F | . | . | . | . | R | . | . | . | . | . | . | . | . | . | . |
| 21-225_34D2_HC | . | F | . | . | . | . | R | . | . | . | . | . | . | . | . | . | . |
| 21-225_29H8_HC | . | . | . | . | R | . | . | . | . | . | . | . | . | . | . | . | . |
| 21-225_11E5_HC | . | . | . | . | [illegible] | . | . | . | . | . | . | . | . | . | . | . | . |
| 21-225_8B11_HC | . | . | . | . | . | . | . | . | . | . | . | . | . | . | . | . | . |
| 21-225_22D2_HC | . | . | . | . | . | . | . | . | . | . | . | . | . | . | . | . | . |
| 21-225_32A1_HC | . | . | . | . | . | . | . | . | . | . | . | . | . | . | . | . | . |
| 21-225_32A5_HC | . | F | . | . | . | . | R | . | . | . | . | . | . | . | . | . | . |
| 21-225_34H7_HC | . | . | . | . | . | . | . | . | . | . | . | . | . | . | . | . | . |

H_FR3

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| CONSENSUS | I | S | T | A | Y | M | E | L | S | S | L | R | S | E | D | T | A |
| 21-225_24E1_HC | . | . | . | . | . | . | . | . | . | . | . | . | . | . | . | . | . |
| 21-225_23A12_HC | . | N | . | . | . | . | . | . | . | . | . | . | . | . | . | . | . |
| 21-225_25A4_HC | . | . | . | . | . | . | . | . | . | . | . | . | . | . | . | . | . |
| 21-225_28B6_HC | . | . | . | . | . | . | . | . | . | . | . | . | . | . | . | . | . |
| 21-225_29E6_HC | . | . | . | . | . | . | . | . | . | . | . | . | . | . | . | . | . |
| 21-225_26C4_HC | . | N | . | . | . | . | . | . | . | . | . | . | . | . | . | . | . |
| 21-225_33D1_HC | . | . | . | . | . | . | . | . | . | . | . | . | . | . | . | . | . |
| 21-225_34D2_HC | . | . | . | . | . | L | . | . | . | . | . | . | . | . | . | . | . |
| 21-225_29H8_HC | . | . | A | . | . | . | . | . | . | . | . | . | . | . | . | . | . |
| 21-225_11E5_HC | . | . | . | . | . | . | . | . | . | . | . | . | . | . | . | . | . |
| 21-225_8B11_HC | R | . | . | . | . | . | . | . | . | . | . | . | . | . | . | . | . |
| 21-225_22D2_HC | . | . | . | . | . | . | . | . | . | . | . | . | . | . | . | . | . |
| 21-225_32A1_HC | . | . | . | . | . | . | . | . | . | . | . | . | . | . | . | . | . |
| 21-225_32A5_HC | . | . | . | . | . | . | . | . | . | . | . | . | . | . | . | . | . |
| 21-225_34H7_HC | . | . | . | . | . | . | . | . | N | . | . | . | . | . | . | . | . |

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | A | S | S | S | G | W | Y | - | - | - | - | - | - |
| 21-225_24E1_HC | . | . | . | . | . | T | . | . | . | . | [illegible] | . | . | . | . | . | . |
| 21-225_23A12_HC | . | . | . | . | . | G | . | . | . | . | . | . | . | . | . | . | . |
| 21-225_25A4_HC | . | . | . | . | . | . | . | . | . | . | . | . | . | . | . | . | . |
| 21-225_28B6_HC | . | . | . | . | . | . | . | . | . | . | . | . | . | . | . | . | . |
| 21-225_29E6_HC | . | . | . | . | . | . | . | . | . | . | . | . | . | . | . | . | . |
| 21-225_26C4_HC | . | . | . | . | . | . | . | . | . | . | . | . | . | . | . | . | . |
| 21-225_33D1_HC | . | . | . | . | . | . | . | . | . | . | . | . | . | . | . | . | . |
| 21-225_34D2_HC | . | . | . | . | . | . | . | . | . | . | . | . | . | . | . | . | . |
| 21-225_29H8_HC | . | . | . | . | . | . | . | . | . | . | . | . | . | . | . | . | . |
| 21-225_11E5_HC | . | . | . | . | . | T | . | . | . | . | [illegible] | . | . | . | . | . | . |
| 21-225_8B11_HC | . | . | . | . | . | Y | . | . | . | . | . | . | . | . | . | . | . |
| 21-225_22D2_HC | . | . | . | . | . | Y | . | . | . | . | . | . | . | . | . | . | . |
| 21-225_32A1_HC | . | . | . | . | . | . | . | . | . | . | . | . | . | . | . | . | . |
| 21-225_32A5_HC | . | . | . | . | S | . | . | . | . | . | . | . | . | . | . | . | . |
| 21-225_34H7_HC | . | . | . | . | . | . | . | . | . | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | | H_CDR3 | | | | | | | | | | | | | |
| CONSENSUS | - | - | - | - | - | - | - | - | - | - | - | - | - | - | - | Y | F |
| 21-225_24E1_HC | . | . | . | . | . | . | . | . | . | . | . | . | . | . | . | V | . |
| 21-225_23A12_HC | . | . | . | . | . | . | . | . | . | . | . | . | . | . | . | . | . |
| 21-225_25A4_HC | . | . | . | . | . | . | . | . | . | . | . | . | . | . | . | . | . |
| 21-225_28B6_HC | . | . | . | . | . | . | . | . | . | . | . | . | . | . | . | . | . |
| 21-225_29E6_HC | . | . | . | . | . | . | . | . | . | . | . | . | . | . | . | . | . |
| 21-225_26C4_HC | . | . | . | . | . | . | . | . | . | . | . | . | . | . | . | . | . |
| 21-225_33D1_HC | . | . | . | . | . | . | . | . | . | . | . | . | . | . | . | [illegible] | . |
| 21-225_34D2_HC | . | . | . | . | . | . | . | . | . | . | . | . | . | . | . | [illegible] | . |
| 21-225_29H8_HC | . | . | . | . | . | . | . | . | . | . | . | . | . | . | . | . | . |
| 21-225_11E5_HC | . | . | . | . | . | . | . | . | . | . | . | . | . | . | . | V | . |
| 21-225_8B11_HC | . | . | . | . | . | . | . | . | . | . | . | . | . | . | . | [illegible] | . |
| 21-225_22D2_HC | . | . | . | . | . | . | . | . | . | . | . | . | . | . | . | . | . |
| 21-225_32A1_HC | . | . | . | . | . | . | . | . | . | . | . | . | . | . | . | [illegible] | . |
| 21-225_32A5_HC | . | . | . | . | . | . | . | . | . | . | . | . | . | . | . | [illegible] | . |
| 21-225_34H7_HC | . | . | . | . | . | . | . | . | . | . | . | . | . | . | . | [illegible] | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | H_FR4 | | | | | | | | | | |
| CONSENSUS | D | Y | W | G | Q | G | T | L | V | T | V | S | S |
| 21-225_24E1_HC | . | . | . | . | . | . | . | . | . | . | . | . | . |
| 21-225_23A12_HC | . | . | . | . | . | . | . | . | . | . | . | . | . |
| 21-225_25A4_HC | . | . | . | . | . | . | . | . | . | . | . | . | . |
| 21-225_28B6_HC | . | . | . | . | . | . | . | . | . | . | . | . | . |
| 21-225_29E6_HC | . | . | . | . | . | . | . | . | . | . | . | . | . |
| 21-225_26C4_HC | . | . | . | . | . | . | . | . | . | . | . | . | . |
| 21-225_33D1_HC | . | . | . | . | . | . | . | . | . | . | . | . | . |
| 21-225_34D2_HC | . | . | . | . | . | . | . | . | . | . | . | . | . |
| 21-225_29H8_HC | . | . | . | . | . | . | . | . | . | . | . | . | . |
| 21-225_11E5_HC | . | . | . | . | . | . | . | . | . | . | . | . | . |
| 21-225_8B11_HC | . | . | . | . | . | . | . | . | . | . | . | . | . |
| 21-225_22D2_HC | . | . | . | . | . | . | . | . | . | . | . | . | . |
| 21-225_32A1_HC | . | . | . | . | . | . | . | . | . | . | . | . | . |
| 21-225_32A5_HC | . | . | . | . | . | . | . | . | . | . | . | . | . |
| 21-225_34H7_HC | . | . | . | . | . | . | . | . | . | . | . | . | . |

**FIGURE 56**

**Table 24**

| Reference # | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|
| | | | | | | | | | | | | | | | | H |
| CONSENSUS | E | V | Q | L | V | E | S | - | G | G | G | L | V | K | P | G |
| 21-225_17H5_HC | . | . | . | . | . | . | . | . | . | . | . | . | . | . | . | . |
| 21-225_16A11_HC | . | . | . | . | . | . | . | . | . | . | . | . | . | . | . | . |
| 21-225_20H7_HC | . | . | H | . | . | . | . | . | . | . | . | . | . | . | . | . |
| 21-225_20D12_HC | . | . | . | . | . | . | . | . | . | . | . | . | . | . | . | . |
| 21-225_22B12_HC | . | . | . | . | . | . | . | . | . | . | . | . | . | . | . | . |
| 21-225_27C5_HC | . | . | . | . | . | . | . | . | . | . | . | . | . | . | . | . |
| 21-225_27H12_HC | . | . | . | . | . | . | . | . | . | . | . | . | . | . | . | . |
| 21-225_35H8_HC | . | . | . | . | . | . | . | . | . | . | . | . | . | . | . | . |
| 21-225_15F8_HC | . | . | . | . | . | . | . | . | . | . | . | . | . | . | . | . |
| 21-225_20D6_HC | . | . | . | . | . | . | . | . | . | . | . | . | . | . | . | . |
| 21-225_15A2_HC | . | . | . | . | . | . | . | . | . | . | . | . | . | . | . | . |
| 21-225_5E11_HC | . | . | . | . | . | . | . | . | . | . | . | . | . | . | . | . |
| 21-225_2A4_HC | . | . | . | . | . | . | . | . | . | . | . | . | . | . | . | . |
| 21-225_16B8_HC | . | . | . | . | . | . | . | . | . | . | . | . | . | . | . | . |
| 21-225_14D6_HC | . | . | . | . | . | . | . | . | . | . | . | . | . | . | . | . |
| 21-225_1F1_HC | . | . | . | . | . | . | . | . | . | . | . | . | . | . | . | . |
| 21-225_5F4_HC | . | . | . | . | . | . | . | . | . | . | . | . | . | . | . | . |
| 21-225_16H1_HC | . | . | . | . | . | . | . | . | . | . | . | . | . | . | . | . |
| 21-225_17H6_HC | . | . | . | . | . | . | . | . | . | . | . | . | . | . | . | . |
| 21-225_21F12_HC | . | . | . | . | . | . | . | . | . | . | . | . | . | . | . | . |
| 21-225_23A11_HC | . | . | . | . | . | . | . | . | . | . | . | . | . | . | . | . |
| 21-225_32B3_HC | . | . | . | . | . | . | . | . | . | . | . | . | . | . | . | . |
| 21-225_2C12_HC | . | . | . | . | . | . | . | . | . | . | . | . | . | . | . | . |

FIGURE 56

| Reference # | 17 | 18 | 19 | 20 | 21 | 22 | 23 | 24 | 25 | 26 | 27 | 28 | 29 | 30 | 31 | 32 |
|---|---|---|---|---|---|---|---|---|---|---|---|---|---|---|---|---|
| | R1 | | | | | | | | | | | | | | | |
| CONSENSUS | G | S | L | R | L | S | C | A | A | S | G | - | F | T | F | S |
| 21-225_17H5_HC | . | . | . | . | . | . | . | . | . | . | . | . | . | . | . | . |
| 21-225_16A11_HC | . | . | . | . | . | . | . | . | . | . | . | . | . | . | . | . |
| 21-225_20H7_HC | . | . | . | . | . | . | . | . | . | . | . | . | . | . | . | . |
| 21-225_20D12_HC | . | . | . | . | . | . | . | . | . | . | . | . | . | . | . | . |
| 21-225_22B12_HC | . | . | . | . | . | . | . | . | . | . | . | . | . | . | . | . |
| 21-225_27C5_HC | . | . | . | . | . | . | . | . | . | . | . | . | . | . | . | . |
| 21-225_27H12_HC | . | . | . | . | . | . | . | . | . | . | . | . | . | . | . | . |
| 21-225_35H8_HC | . | . | . | . | . | . | . | . | . | . | . | . | . | . | . | . |
| 21-225_15F8_HC | . | . | . | . | . | . | . | . | . | . | . | . | . | . | . | . |
| 21-225_20D6_HC | . | . | . | . | . | . | . | . | . | . | . | . | . | . | . | . |
| 21-225_15A2_HC | . | . | . | . | . | . | . | . | . | . | . | . | . | . | . | . |
| 21-225_5E11_HC | . | . | . | . | . | . | . | . | . | . | . | . | . | . | . | . |
| 21-225_2A4_HC | . | . | . | . | . | . | . | . | . | . | . | . | . | . | . | . |
| 21-225_16B8_HC | . | . | . | . | . | . | . | . | . | . | . | . | . | . | . | . |
| 21-225_14D6_HC | . | . | . | . | . | . | . | . | . | . | . | . | . | . | . | . |
| 21-225_1F1_HC | . | . | . | . | . | . | . | . | . | . | . | . | . | . | . | . |
| 21-225_5F4_HC | . | . | . | . | . | . | . | V | . | . | . | . | . | . | . | . |
| 21-225_16H1_HC | . | . | . | . | . | . | . | . | . | . | . | . | . | . | . | . |
| 21-225_17H6_HC | . | . | . | . | . | . | . | . | . | . | . | . | . | . | . | . |
| 21-225_21F12_HC | . | . | . | . | . | . | . | . | . | . | . | . | . | . | . | . |
| 21-225_23A11_HC | . | . | . | . | . | . | . | . | . | . | . | . | . | . | . | . |
| 21-225_32B3_HC | . | . | . | . | . | . | . | . | . | . | . | . | . | . | . | . |
| 21-225_2C12_HC | . | . | . | . | . | . | . | . | . | . | . | . | . | . | . | . |

FIGURE 56

| Reference # | 33 | 34 | 35 | 36 | 37 | 38 | 39 | 40 | 41 | 42 | 43 | 44 | 45 | 46 | 47 | 48 |
|---|---|---|---|---|---|---|---|---|---|---|---|---|---|---|---|---|
| | H_CDR1 | | | | | | | | | | | | | | | |
| CONSENSUS | S | - | - | - | - | - | Y | S | M | N | W | V | R | Q | A | P |
| 21-225_17H5_HC | . | . | . | . | . | . | . | . | . | . | . | . | . | . | . | . |
| 21-225_16A11_HC | . | . | . | . | . | . | . | . | . | . | . | . | . | . | . | . |
| 21-225_20H7_HC | . | . | . | . | . | . | . | . | . | . | . | . | . | . | . | . |
| 21-225_20D12_HC | . | . | . | . | . | . | . | . | . | . | . | . | . | . | . | . |
| 21-225_22B12_HC | . | . | . | . | . | . | . | . | . | . | . | . | . | . | . | . |
| 21-225_27C5_HC | . | . | . | . | . | . | . | . | . | . | . | . | . | . | . | . |
| 21-225_27H12_HC | . | . | . | . | . | . | . | . | L | . | . | . | . | . | . | . |
| 21-225_35H8_HC | . | . | . | . | . | . | . | . | . | . | . | . | . | . | . | . |
| 21-225_15F8_HC | . | . | . | . | . | . | . | . | . | . | . | . | . | . | . | . |
| 21-225_20D6_HC | . | . | . | . | . | . | . | R | . | . | . | . | . | . | . | . |
| 21-225_15A2_HC | . | . | . | . | . | . | . | T | . | . | . | . | . | . | . | . |
| 21-225_5E11_HC | . | . | . | . | . | . | . | . | . | . | . | . | . | . | . | . |
| 21-225_2A4_HC | . | . | . | . | . | . | . | . | . | . | . | . | . | . | . | . |
| 21-225_16B8_HC | . | . | . | . | . | . | . | . | . | . | . | . | . | . | . | . |
| 21-225_14D6_HC | . | . | . | . | . | . | . | T | . | . | . | . | . | . | . | . |
| 21-225_1F1_HC | . | . | . | . | . | . | . | N | . | . | . | . | . | . | . | . |
| 21-225_5F4_HC | . | . | . | . | . | . | . | . | . | . | . | . | . | . | . | . |
| 21-225_16H1_HC | . | . | . | . | . | . | . | . | . | . | . | . | . | . | . | . |
| 21-225_17H6_HC | . | . | . | . | . | . | . | . | . | . | . | . | . | . | . | . |
| 21-225_21F12_HC | . | . | . | . | . | . | . | R | . | . | . | . | . | . | . | . |
| 21-225_23A11_HC | . | . | . | . | . | . | . | . | . | . | . | I | . | . | . | . |
| 21-225_32B3_HC | . | . | . | . | . | . | . | . | . | . | . | . | . | . | . | . |
| 21-225_2C12_HC | . | . | . | . | . | . | . | . | . | . | . | . | . | . | . | . |

FIGURE 56

| Reference # | 49 | 50 | 51 | 52 | 53 | 54 | 55 | 56 | 57 | 58 | 59 | 60 | 61 | 62 | 63 | 64 |
|---|---|---|---|---|---|---|---|---|---|---|---|---|---|---|---|---|
| | H_FR2 | | | | | | | | | | | | | | | |
| CONSENSUS | G | K | G | L | E | W | V | S | S | I | S | G | S | - | - | - |
| 21-225_17H5_HC | . | . | . | . | . | . | . | . | . | . | . | S | . | . | . | . |
| 21-225_16A11_HC | . | . | . | . | . | . | . | . | . | . | . | . | . | . | . | . |
| 21-225_20H7_HC | . | . | . | . | . | . | . | . | . | . | . | . | . | . | . | . |
| 21-225_20D12_HC | . | . | . | . | . | . | . | . | . | . | . | . | . | . | . | . |
| 21-225_22B12_HC | . | . | . | . | . | . | . | . | . | . | . | . | . | . | . | . |
| 21-225_27C5_HC | . | . | . | . | . | . | . | . | . | T | . | S | . | . | . | . |
| 21-225_27H12_HC | . | . | . | . | . | . | . | . | . | . | . | . | . | . | . | . |
| 21-225_35H8_HC | . | . | . | . | . | . | . | . | . | . | . | . | T | . | . | . |
| 21-225_15F8_HC | . | . | . | . | . | . | . | . | . | . | . | . | . | . | . | . |
| 21-225_20D6_HC | . | . | . | . | . | . | . | . | . | M | . | S | . | . | . | . |
| 21-225_15A2_HC | . | . | . | . | . | . | . | . | . | . | . | . | . | . | . | . |
| 21-225_5E11_HC | . | . | . | . | . | . | . | . | . | . | . | . | . | . | . | . |
| 21-225_2A4_HC | . | . | . | . | . | . | . | . | . | . | . | . | . | . | . | . |
| 21-225_16B8_HC | . | . | . | . | . | . | I | . | . | . | . | . | . | . | . | . |
| 21-225_14D6_HC | . | . | . | . | . | . | . | . | . | . | . | . | . | . | . | . |
| 21-225_1F1_HC | . | . | . | . | . | . | . | . | . | . | . | S | . | . | . | . |
| 21-225_5F4_HC | . | . | . | . | . | . | . | . | . | . | . | . | . | . | . | . |
| 21-225_16H1_HC | . | . | . | . | . | . | . | . | . | . | . | . | . | . | . | . |
| 21-225_17H6_HC | . | R | . | . | . | . | . | . | . | . | . | . | . | . | . | . |
| 21-225_21F12_HC | . | . | . | . | . | . | . | . | . | . | N | S | Y | . | . | . |
| 21-225_23A11_HC | . | . | . | . | . | . | . | . | . | . | . | . | . | . | . | . |
| 21-225_32B3_HC | . | . | . | . | . | . | . | . | . | . | . | . | . | . | . | . |
| 21-225_2C12_HC | . | . | . | . | . | . | . | . | . | . | . | . | . | . | . | . |

FIGURE 56

| Reference # | 65 | 66 | 67 | 68 | 69 | 70 | 71 | 72 | 73 | 74 | 75 | 76 | 77 | 78 | 79 | 80 |
|---|---|---|---|---|---|---|---|---|---|---|---|---|---|---|---|---|
| | H_CDR2 | | | | | | | | | | | | | | | |
| CONSENSUS | S | S | Y | I | Y | Y | A | D | S | V | K | G | R | F | T | I |
| 21-225_17H5_HC | . | T | . | . | . | . | . | . | . | . | . | . | . | . | . | . |
| 21-225_16A11_HC | . | . | L | . | . | . | . | . | . | . | . | . | . | . | . | . |
| 21-225_20H7_HC | . | . | F | . | . | . | . | . | . | . | . | . | . | . | . | . |
| 21-225_20D12_HC | . | . | . | K | . | . | . | . | . | . | . | . | . | . | . | . |
| 21-225_22B12_HC | . | . | . | T | . | . | . | . | . | . | . | . | . | . | . | . |
| 21-225_27C5_HC | D | . | . | . | . | . | . | . | . | . | . | . | . | . | . | . |
| 21-225_27H12_HC | . | . | N | . | . | . | T | . | . | . | . | . | . | . | . | . |
| 21-225_35H8_HC | G | . | F | . | . | . | . | . | . | . | . | . | . | . | . | . |
| 21-225_15F8_HC | . | . | . | . | . | . | . | . | . | . | . | . | . | . | . | . |
| 21-225_20D6_HC | . | . | N | K | . | . | . | . | . | . | . | . | . | . | . | . |
| 21-225_15A2_HC | G | . | . | . | . | . | . | . | . | . | . | . | . | . | . | . |
| 21-225_5E11_HC | . | T | . | . | . | . | . | . | . | . | . | . | . | . | . | . |
| 21-225_2A4_HC | . | T | . | . | . | . | . | . | . | . | . | . | . | I | A | . |
| 21-225_16B8_HC | . | T | . | . | . | . | . | . | . | . | . | . | . | . | . | . |
| 21-225_14D6_HC | G | . | . | . | . | . | . | . | . | . | . | . | . | . | . | M |
| 21-225_1F1_HC | . | . | . | . | . | . | . | . | . | . | . | . | . | . | . | . |
| 21-225_5F4_HC | . | . | . | . | . | . | . | . | . | . | . | . | . | . | . | . |
| 21-225_16H1_HC | . | T | . | . | . | . | . | . | . | . | . | . | . | . | . | . |
| 21-225_17H6_HC | . | . | . | . | . | . | . | . | . | . | . | . | . | . | . | . |
| 21-225_21F12_HC | . | . | . | . | . | . | . | . | . | . | . | . | . | . | . | . |
| 21-225_23A11_HC | . | T | . | . | . | . | . | . | . | . | . | . | . | I | A | . |
| 21-225_32B3_HC | . | T | . | . | . | . | . | . | . | . | . | . | . | . | . | . |
| 21-225_2C12_HC | . | . | . | M | . | . | . | . | . | . | . | . | . | . | . | . |

FIGURE 56

| Reference # | 81 | 82 | 83 | 84 | 85 | 86 | 87 | 88 | 89 | 90 | 91 | 92 | 93 | 94 | 95 | 96 |
|---|---|---|---|---|---|---|---|---|---|---|---|---|---|---|---|---|
| | H_FR3 | | | | | | | | | | | | | | | |
| CONSENSUS | S | R | D | N | A | K | N | S | L | Y | L | Q | M | N | S | L |
| 21-225_17H5_HC | . | . | . | . | . | . | . | . | . | . | . | . | . | . | . | . |
| 21-225_16A11_HC | . | . | . | . | . | . | . | . | . | . | . | . | . | . | . | . |
| 21-225_20H7_HC | . | . | . | . | . | . | . | . | . | . | . | . | . | . | . | . |
| 21-225_20D12_HC | . | . | . | . | . | . | . | . | . | L | . | . | . | . | . | . |
| 21-225_22B12_HC | . | . | . | . | . | . | . | . | . | . | . | . | . | . | . | . |
| 21-225_27C5_HC | . | . | . | . | . | . | . | . | . | . | . | . | . | . | . | . |
| 21-225_27H12_HC | . | . | . | . | . | . | . | . | . | F | . | . | . | . | . | . |
| 21-225_35H8_HC | . | . | . | . | . | . | K | . | V | . | . | . | . | . | . | . |
| 21-225_15F8_HC | F | . | . | . | . | . | . | . | . | . | . | . | . | . | . | . |
| 21-225_20D6_HC | . | . | . | . | . | . | . | . | . | . | . | . | . | . | . | . |
| 21-225_15A2_HC | . | . | . | . | . | . | . | . | . | . | . | . | . | . | A | . |
| 21-225_5E11_HC | . | . | . | . | . | . | . | . | . | . | . | . | . | . | . | . |
| 21-225_2A4_HC | . | . | . | . | . | . | . | . | . | . | . | . | . | . | . | . |
| 21-225_16B8_HC | . | . | . | . | . | . | . | . | . | . | . | . | . | . | . | . |
| 21-225_14D6_HC | . | . | . | . | . | . | . | . | . | . | . | . | . | . | . | . |
| 21-225_1F1_HC | . | . | . | . | . | . | . | . | . | . | . | . | . | . | . | . |
| 21-225_5F4_HC | . | . | . | . | . | . | . | . | . | F | . | . | . | . | . | . |
| 21-225_16H1_HC | . | . | . | . | . | . | . | . | . | . | . | . | . | . | . | . |
| 21-225_17H6_HC | F | . | . | . | . | . | . | . | . | . | . | . | . | . | . | . |
| 21-225_21F12_HC | . | . | . | . | . | . | . | . | . | . | . | . | . | . | . | . |
| 21-225_23A11_HC | . | . | . | . | . | . | . | . | . | . | . | . | . | . | . | . |
| 21-225_32B3_HC | . | . | . | . | . | . | . | . | . | . | . | . | . | . | . | . |
| 21-225_2C12_HC | . | . | . | . | . | . | D | . | . | . | . | . | . | . | . | . |

FIGURE 56

| Reference # | 97 | 98 | 99 | 100 | 101 | 102 | 103 | 104 | 105 | 106 | 107 | 108 | 109 | 110 | 111 | 112 |
|---|---|---|---|---|---|---|---|---|---|---|---|---|---|---|---|---|
| CONSENSUS | R | A | E | D | T | A | V | Y | Y | C | A | R | V | A | S | - |
| 21-225_17H5_HC | . | . | . | . | . | . | . | . | . | . | . | . | . | . | . | . |
| 21-225_16A11_HC | . | . | . | . | . | . | . | . | . | . | . | . | . | . | A | . |
| 21-225_20H7_HC | . | . | . | . | . | . | . | . | . | . | . | . | . | . | A | . |
| 21-225_20D12_HC | . | . | . | . | . | . | . | . | . | . | . | . | . | N | N | . |
| 21-225_22B12_HC | . | . | . | . | . | . | . | . | . | . | . | . | . | . | A | . |
| 21-225_27C5_HC | . | . | . | . | . | . | . | . | . | . | . | . | . | . | . | . |
| 21-225_27H12_HC | . | . | . | . | . | . | . | . | . | . | . | . | . | . | . | . |
| 21-225_35H8_HC | . | . | . | . | . | . | . | . | . | . | . | . | . | S | G | . |
| 21-225_15F8_HC | . | . | . | . | . | . | . | . | . | . | . | . | . | . | . | . |
| 21-225_20D6_HC | . | . | . | . | . | . | . | . | . | . | . | . | . | . | A | . |
| 21-225_15A2_HC | . | . | . | . | . | . | . | . | . | . | . | . | . | . | . | . |
| 21-225_5E11_HC | . | . | . | . | . | . | . | F | . | . | . | . | . | . | . | . |
| 21-225_2A4_HC | . | . | . | . | . | . | . | . | . | . | . | . | . | . | . | . |
| 21-225_16B8_HC | . | . | . | . | . | . | . | . | . | . | . | . | . | . | . | . |
| 21-225_14D6_HC | . | . | . | . | . | . | . | . | . | . | . | . | . | . | A | . |
| 21-225_1F1_HC | . | . | . | . | . | . | . | . | . | . | . | . | . | N | L | . |
| 21-225_5F4_HC | . | . | . | . | . | . | . | . | . | . | . | . | . | N | N | . |
| 21-225_16H1_HC | . | . | . | . | . | . | . | . | . | . | . | . | . | . | A | . |
| 21-225_17H6_HC | . | . | . | . | . | . | . | . | . | . | . | . | . | . | . | . |
| 21-225_21F12_HC | . | . | . | . | . | . | . | . | . | . | . | . | . | . | . | . |
| 21-225_23A11_HC | . | . | . | . | . | . | . | . | . | . | . | . | . | . | . | . |
| 21-225_32B3_HC | . | . | . | . | . | . | . | . | . | . | . | . | . | . | G | . |
| 21-225_2C12_HC | . | . | . | . | . | . | . | . | . | . | . | . | . | N | L | . |

FIGURE 56

| Reference # | 113 | 114 | 115 | 116 | 117 | 118 | 119 | 120 | 121 | 122 | 123 | 124 | 125 | 126 | 127 | 128 |
|---|---|---|---|---|---|---|---|---|---|---|---|---|---|---|---|---|
| | | | | | | | | | | | H_CDR3 | | | | | |
| CONSENSUS | - | - | - | - | - | - | - | - | - | - | - | - | - | - | - | - |
| 21-225_17H5_HC | . | . | . | . | . | . | . | . | . | . | . | . | . | . | . | . |
| 21-225_16A11_HC | . | . | . | . | . | . | . | . | . | . | . | . | . | . | . | . |
| 21-225_20H7_HC | . | . | . | . | . | . | . | . | . | . | . | . | . | . | . | . |
| 21-225_20D12_HC | . | . | . | . | . | . | . | . | . | . | . | . | . | . | . | . |
| 21-225_22B12_HC | . | . | . | . | . | . | . | . | . | . | . | . | . | . | . | . |
| 21-225_27C5_HC | . | . | . | . | . | . | . | . | . | . | . | . | . | . | . | . |
| 21-225_27H12_HC | . | . | . | . | . | . | . | . | . | . | . | . | . | . | . | . |
| 21-225_35H8_HC | . | . | . | . | . | . | . | . | . | . | . | . | . | . | . | . |
| 21-225_15F8_HC | . | . | . | . | . | . | . | . | . | . | . | . | . | . | . | . |
| 21-225_20D6_HC | . | . | . | . | . | . | . | . | . | . | . | . | . | . | . | . |
| 21-225_15A2_HC | . | . | . | . | . | . | . | . | . | . | . | . | . | . | . | . |
| 21-225_5E11_HC | . | . | . | . | . | . | . | . | . | . | . | . | . | . | . | . |
| 21-225_2A4_HC | . | . | . | . | . | . | . | . | . | . | . | . | . | . | . | . |
| 21-225_16B8_HC | . | . | . | . | . | . | . | . | . | . | . | . | . | . | . | . |
| 21-225_14D6_HC | . | . | . | . | . | . | . | . | . | . | . | . | . | . | . | . |
| 21-225_1F1_HC | . | . | . | . | . | . | . | . | . | . | . | . | . | . | . | . |
| 21-225_5F4_HC | . | . | . | . | . | . | . | . | . | . | . | . | . | . | . | . |
| 21-225_16H1_HC | . | . | . | . | . | . | . | . | . | . | . | . | . | . | . | . |
| 21-225_17H6_HC | . | . | . | . | . | . | . | . | . | . | . | . | . | . | . | . |
| 21-225_21F12_HC | . | . | . | . | . | . | . | . | . | . | . | . | . | . | . | . |
| 21-225_23A11_HC | . | . | . | . | . | . | . | . | . | . | . | . | . | . | . | . |
| 21-225_32B3_HC | . | . | . | . | . | . | . | . | . | . | . | . | . | . | . | . |
| 21-225_2C12_HC | . | . | . | . | . | . | . | . | . | . | . | . | . | . | . | . |

FIGURE 56

| Reference # | 129 | 130 | 131 | 132 | 133 | 134 | 135 | 136 | 137 | 138 | 139 | 140 | 141 | 142 | 143 | 144 |
|---|---|---|---|---|---|---|---|---|---|---|---|---|---|---|---|---|
| | | | | | | | | | | | | | | | | H_FR |
| CONSENSUS | - | - | - | - | - | - | - | F | D | Y | W | G | Q | G | T | L |
| 21-225_17H5_HC | . | . | . | . | . | . | . | . | . | . | . | . | . | . | . | . |
| 21-225_16A11_HC | . | . | . | . | . | . | . | . | . | . | . | . | . | . | . | . |
| 21-225_20H7_HC | . | . | . | . | . | . | . | L | . | . | . | . | . | . | . | . |
| 21-225_20D12_HC | . | . | . | . | . | . | . | . | . | . | . | . | . | . | . | . |
| 21-225_22B12_HC | . | . | . | . | . | . | . | L | . | S | . | . | . | . | . | . |
| 21-225_27C5_HC | . | . | . | . | . | . | . | . | . | C | . | . | . | . | . | . |
| 21-225_27H12_HC | . | . | . | . | . | . | . | . | . | . | . | . | . | . | . | . |
| 21-225_35H8_HC | . | . | . | . | . | . | . | . | . | . | . | . | . | . | . | . |
| 21-225_15F8_HC | . | . | . | . | . | . | . | . | . | . | . | . | . | . | . | . |
| 21-225_20D6_HC | . | . | . | . | . | . | . | L | . | . | . | . | . | . | . | . |
| 21-225_15A2_HC | . | . | . | . | . | . | . | . | . | . | . | . | . | . | . | . |
| 21-225_5E11_HC | . | . | . | . | . | . | . | . | . | . | . | . | . | . | . | . |
| 21-225_2A4_HC | . | . | . | . | . | . | . | . | . | . | . | . | . | . | . | . |
| 21-225_16B8_HC | . | . | . | . | . | . | . | . | . | . | . | . | . | . | . | . |
| 21-225_14D6_HC | . | . | . | . | . | . | . | . | . | S | . | . | . | . | . | . |
| 21-225_1F1_HC | . | . | . | . | . | . | . | . | . | . | . | . | . | . | . | . |
| 21-225_5F4_HC | . | . | . | . | . | . | . | . | . | . | . | . | . | . | . | . |
| 21-225_16H1_HC | . | . | . | . | . | . | . | . | . | . | . | . | . | . | . | . |
| 21-225_17H6_HC | . | . | . | . | . | . | . | . | . | . | . | . | . | . | . | . |
| 21-225_21F12_HC | . | . | . | . | . | . | . | . | . | . | . | . | . | . | . | . |
| 21-225_23A11_HC | . | . | . | . | . | . | . | . | . | . | . | . | . | . | . | . |
| 21-225_32B3_HC | . | . | . | . | . | . | . | . | . | . | . | . | . | . | . | . |
| 21-225_2C12_HC | . | . | . | . | . | . | . | . | . | . | . | . | . | . | . | . |

FIGURE 56

| Reference # | 129 | 130 | 131 | 132 | 133 | 134 | 135 | 136 | 137 | 138 | 139 | 140 | 141 | 142 | 143 | 144 |
|---|---|---|---|---|---|---|---|---|---|---|---|---|---|---|---|---|
| | | | | | | | | | | | | | | | | H FR |
| CONSENSUS | - | - | - | - | - | - | - | F | D | Y | W | G | Q | G | T | L |
| 21-225_17H5_HC | . | . | . | . | . | . | . | . | . | . | . | . | . | . | . | . |
| 21-225_16A11_HC | . | . | . | . | . | . | . | . | . | . | . | . | . | . | . | . |
| 21-225_20H7_HC | . | . | . | . | . | . | . | L | . | . | . | . | . | . | . | . |
| 21-225_20D12_HC | . | . | . | . | . | . | . | . | . | . | . | . | . | . | . | . |
| 21-225_22B12_HC | . | . | . | . | . | . | . | L | . | S | . | . | . | . | . | . |
| 21-225_27C5_HC | . | . | . | . | . | . | . | . | . | C | . | . | . | . | . | . |
| 21-225_27H12_HC | . | . | . | . | . | . | . | . | . | . | . | . | . | . | . | . |
| 21-225_35H8_HC | . | . | . | . | . | . | . | . | . | . | . | . | . | . | . | . |
| 21-225_15F8_HC | . | . | . | . | . | . | . | . | . | . | . | . | . | . | . | . |
| 21-225_20D6_HC | . | . | . | . | . | . | . | L | . | . | . | . | . | . | . | . |
| 21-225_15A2_HC | . | . | . | . | . | . | . | . | . | . | . | . | . | . | . | . |
| 21-225_5E11_HC | . | . | . | . | . | . | . | . | . | . | . | . | . | . | . | . |
| 21-225_2A4_HC | . | . | . | . | . | . | . | . | . | . | . | . | . | . | . | . |
| 21-225_16B8_HC | . | . | . | . | . | . | . | . | . | . | . | . | . | . | . | . |
| 21-225_14D6_HC | . | . | . | . | . | . | . | . | . | S | . | . | . | . | . | . |
| 21-225_1F1_HC | . | . | . | . | . | . | . | . | . | . | . | . | . | . | . | . |
| 21-225_5F4_HC | . | . | . | . | . | . | . | . | . | . | . | . | . | . | . | . |
| 21-225_16H1_HC | . | . | . | . | . | . | . | . | . | . | . | . | . | . | . | . |
| 21-225_17H6_HC | . | . | . | . | . | . | . | . | . | . | . | . | . | . | . | . |
| 21-225_21F12_HC | . | . | . | . | . | . | . | . | . | . | . | . | . | . | . | . |
| 21-225_23A11_HC | . | . | . | . | . | . | . | . | . | . | . | . | . | . | . | . |
| 21-225_32B3_HC | . | . | . | . | . | . | . | . | . | . | . | . | . | . | . | . |
| 21-225_2C12_HC | . | . | . | . | . | . | . | . | . | . | . | . | . | . | . | . |

FIGURE 56

| Reference # | 145 | 146 | 147 | 148 | 149 |
|---|---|---|---|---|---|
| CONSENSUS | V | T | V | S | S |
| 21-225_17H5_HC | . | . | . | . | . |
| 21-225_16A11_HC | . | . | . | . | . |
| 21-225_20H7_HC | . | . | . | . | . |
| 21-225_20D12_HC | . | . | . | . | . |
| 21-225_22B12_HC | . | . | . | . | . |
| 21-225_27C5_HC | . | . | . | . | . |
| 21-225_27H12_HC | . | . | . | . | . |
| 21-225_35H8_HC | . | . | . | . | . |
| 21-225_15F8_HC | . | . | . | . | . |
| 21-225_20D6_HC | . | . | . | . | . |
| 21-225_15A2_HC | . | . | . | . | . |
| 21-225_5E11_HC | . | . | . | . | . |
| 21-225_2A4_HC | . | . | . | . | . |
| 21-225_16B8_HC | . | . | . | . | . |
| 21-225_14D6_HC | . | S | . | . | . |
| 21-225_1F1_HC | . | . | . | . | . |
| 21-225_5F4_HC | . | . | . | . | . |
| 21-225_16H1_HC | . | . | . | . | . |
| 21-225_17H6_HC | . | . | . | . | . |
| 21-225_21F12_HC | . | . | . | . | . |
| 21-225_23A11_HC | . | . | . | . | . |
| 21-225_32B3_HC | . | . | . | . | . |
| 21-225_2C12_HC | . | . | . | . | . |

FIGURE 56

Table 25

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | K_FR1 | | | | | | | | | | | | | | | | |
| CONSENSUS | D | I | Q | M | T | Q | S | P | S | S | L | S | A | S | V | G | D |
| 21-225_2C12_LC | . | . | . | . | . | . | . | . | . | . | . | . | . | . | . | . | . |
| 21-225_17H5_LC | . | . | . | . | . | . | . | . | . | . | . | . | . | . | . | . | . |
| 21-225_16A11_LC | . | . | . | . | . | . | . | . | . | . | . | . | . | . | . | . | . |
| 21-225_20H7_LC | . | . | . | . | . | . | . | . | . | . | . | . | . | . | . | . | . |
| 21-225_20D12_LC | . | . | . | . | . | . | . | . | . | . | . | . | . | . | . | . | . |
| 21-225_22B12_LC | . | . | . | . | . | . | . | . | . | . | . | . | . | . | . | . | . |
| 21-225_27C5_LC | . | . | . | . | . | . | . | . | . | . | V | . | . | . | . | . | . |
| 21-225_27H12_LC | . | . | . | . | . | . | . | . | . | . | . | . | . | . | . | . | . |
| 21-225_35H8_LC | . | . | . | . | . | . | . | . | . | . | . | . | . | . | . | . | . |
| 21-225_15F8_LC | . | . | . | . | . | . | . | . | . | . | . | F | . | . | . | . | . |
| 21-225_20D6_LC | . | . | . | . | . | . | . | . | . | . | . | . | . | . | . | . | . |
| 21-225_15A2_LC | . | . | . | . | . | . | . | . | . | . | . | F | . | F | I | . | . |
| 21-225_5E11_LC | V | . | . | . | . | . | . | . | . | . | . | . | . | . | . | . | . |
| 21-225_2A4_LC | . | . | . | . | . | . | . | . | . | . | . | F | . | . | . | . | . |
| 21-225_16B8_LC | . | . | . | . | . | . | . | . | . | . | . | . | . | . | . | . | . |
| 21-225_14D6_LC | . | . | . | . | . | . | . | . | . | . | . | . | . | . | . | . | . |
| 21-225_1F1_LC | . | . | . | . | . | . | . | . | . | . | . | . | . | . | . | . | . |
| 21-225_5F4_LC | . | . | . | . | . | . | . | . | . | . | . | . | . | . | . | . | . |
| 21-225_16H1_LC | . | . | . | . | . | . | . | . | . | . | . | . | . | . | . | . | . |
| 21-225_17H6_LC | . | . | . | . | . | . | . | . | . | . | . | . | . | . | . | . | . |
| 21-225_21F12_LC | . | . | . | . | . | . | . | . | . | . | . | . | . | . | . | . | . |
| 21-225_23A11_LC | . | . | . | . | . | . | . | . | . | . | . | . | T | . | . | . | . |
| 21-225_32B3_LC | . | . | . | . | . | . | . | . | . | . | . | . | . | . | I | . | . |

FIGURE 56

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | K_CDR1 | | |
| CONSENSUS | R | V | T | I | T | C | R | A | S | - | - | Q | G | I | S | - | - |
| 21-225_2C12_LC | . | . | . | . | . | . | . | . | . | . | . | . | . | . | . | . | . |
| 21-225_17H5_LC | . | . | . | . | . | . | . | . | . | . | . | . | . | . | . | . | . |
| 21-225_16A11_LC | S | . | . | . | S | . | . | . | . | . | . | . | D | . | R | . | . |
| 21-225_20H7_LC | . | . | . | . | . | . | . | T | . | . | . | . | . | . | N | . | . |
| 21-225_20D12_LC | . | . | . | . | . | . | . | . | . | . | . | . | D | . | R | . | . |
| 21-225_22B12_LC | . | . | . | . | I | . | . | . | . | . | . | . | D | . | . | . | . |
| 21-225_27C5_LC | . | . | . | . | . | . | . | . | . | . | . | . | . | . | . | . | . |
| 21-225_27H12_LC | . | . | . | . | . | . | . | . | . | . | . | . | . | . | . | . | . |
| 21-225_35H8_LC | . | . | . | . | . | . | . | . | . | . | . | . | A | . | . | . | . |
| 21-225_15F8_LC | . | . | . | . | . | . | . | . | . | . | . | R | D | . | . | . | . |
| 21-225_20D6_LC | . | . | . | . | . | . | . | . | . | . | . | . | . | . | . | . | . |
| 21-225_15A2_LC | . | . | . | . | . | . | . | . | . | . | . | . | . | . | . | . | . |
| 21-225_5E11_LC | . | . | . | . | . | . | . | . | . | . | . | . | . | . | . | . | . |
| 21-225_2A4_LC | . | . | . | . | . | . | . | . | . | . | . | . | . | . | . | . | . |
| 21-225_16B8_LC | . | . | . | . | . | . | . | . | . | . | . | . | . | . | N | . | . |
| 21-225_14D6_LC | S | . | . | . | . | . | . | . | . | . | . | . | D | . | R | . | . |
| 21-225_1F1_LC | . | . | . | . | . | . | . | . | . | . | . | . | . | . | N | . | . |
| 21-225_5F4_LC | . | . | . | . | . | . | . | . | . | . | . | . | . | . | R | . | . |
| 21-225_16H1_LC | . | . | . | . | . | . | . | . | . | . | . | . | . | . | R | . | . |
| 21-225_17H6_LC | . | . | . | . | . | . | . | . | . | . | . | R | D | . | . | . | . |
| 21-225_21F12_LC | . | . | . | . | . | . | . | . | . | . | . | . | . | . | R | . | . |
| 21-225_23A11_LC | . | . | . | . | . | . | . | . | . | . | . | . | D | . | . | . | . |
| 21-225_32B3_LC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | | K_FR2 | |
| CONSENSUS | - | - | - | - | N | Y | L | A | W | F | Q | Q | K | P | G | K | A |
| 21-225_2C12_LC | . | . | . | . | . | . | . | . | . | . | . | . | . | S | . | . | . |
| 21-225_17H5_LC | . | . | . | . | . | . | . | . | . | . | . | . | . | . | . | . | . |
| 21-225_16A11_LC | . | . | . | . | . | H | . | G | . | Y | . | H | N | . | . | . | . |
| 21-225_20H7_LC | . | . | . | . | . | . | . | V | . | . | . | . | . | . | . | . | . |
| 21-225_20D12_LC | . | . | . | . | . | H | . | G | . | Y | . | . | . | . | . | . | . |
| 21-225_22B12_LC | . | . | . | . | . | . | . | . | . | . | . | E | . | . | . | . | . |
| 21-225_27C5_LC | . | . | . | . | S | W | . | . | . | Y | . | . | . | . | . | . | . |
| 21-225_27H12_LC | . | . | . | . | . | . | . | . | . | . | . | . | . | . | . | . | . |
| 21-225_35H8_LC | . | . | . | . | . | . | . | . | . | . | . | . | . | . | . | . | . |
| 21-225_15F8_LC | . | . | . | . | . | . | . | . | . | . | . | . | . | . | . | . | . |
| 21-225_20D6_LC | . | . | . | . | . | . | . | . | . | . | . | . | . | . | . | . | . |
| 21-225_15A2_LC | . | . | . | . | . | . | . | . | . | . | . | . | . | . | . | . | . |
| 21-225_5E11_LC | . | . | . | . | . | . | . | . | . | . | . | . | . | . | . | . | . |
| 21-225_2A4_LC | . | . | . | . | . | . | . | . | . | . | . | . | . | . | . | . | . |
| 21-225_16B8_LC | . | . | . | . | . | . | . | . | . | . | . | . | . | . | . | . | . |
| 21-225_14D6_LC | . | . | . | . | . | H | . | G | . | Y | . | H | . | . | . | . | . |
| 21-225_1F1_LC | . | . | . | . | . | . | . | . | . | . | . | . | . | S | . | . | . |
| 21-225_5F4_LC | . | . | . | . | . | H | . | G | . | Y | . | . | . | . | . | . | . |
| 21-225_16H1_LC | . | . | . | . | D | I | . | G | . | Y | . | . | . | . | . | . | . |
| 21-225_17H6_LC | . | . | . | . | . | . | . | . | . | . | . | . | . | . | . | . | . |
| 21-225_21F12_LC | . | . | . | . | . | F | . | . | . | . | . | . | . | . | . | . | . |
| 21-225_23A11_LC | . | . | . | . | . | . | . | . | . | . | . | . | . | . | . | . | . |
| 21-225_32B3_LC | . | . | . | . | . | . | . | . | . | . | . | . | . | . | . | . | . |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | K_CDR2 | | | |
| CONSENSUS | P | K | S | L | I | Y | A | - | - | - | - | - | - | - | - | A | S |
| 21-225_2C12_LC | . | . | . | . | . | S | . | . | . | . | . | . | . | . | . | . | T |
| 21-225_17H5_LC | . | . | . | . | . | N | . | . | . | . | . | . | . | . | . | . | . |
| 21-225_16A11_LC | . | . | R | . | . | . | . | . | . | . | . | . | . | . | . | . | . |
| 21-225_20H7_LC | . | . | R | . | . | . | . | . | . | . | . | . | . | . | . | . | . |
| 21-225_20D12_LC | . | . | R | . | . | . | . | . | . | . | . | . | . | . | . | . | . |
| 21-225_22B12_LC | H | . | . | . | . | . | G | . | . | . | . | . | . | . | . | . | . |
| 21-225_27C5_LC | . | . | F | . | . | . | G | . | . | . | . | . | . | . | . | . | . |
| 21-225_27H12_LC | . | . | . | . | . | N | G | . | . | . | . | . | . | . | . | . | . |
| 21-225_35H8_LC | . | . | . | . | . | . | G | . | . | . | . | . | . | . | . | . | . |
| 21-225_15F8_LC | . | . | . | . | . | C | S | . | . | . | . | . | . | . | . | T | . |
| 21-225_20D6_LC | . | . | . | . | . | S | . | . | . | . | . | . | . | . | . | . | . |
| 21-225_15A2_LC | . | . | . | . | . | F | . | . | . | . | . | . | . | . | . | . | . |
| 21-225_5E11_LC | . | . | . | . | . | N | . | . | . | . | . | . | . | . | . | . | . |
| 21-225_2A4_LC | . | . | R | . | . | . | T | . | . | . | . | . | . | . | . | . | . |
| 21-225_16B8_LC | L | . | . | . | . | N | . | . | . | . | . | . | . | . | . | . | . |
| 21-225_14D6_LC | . | . | R | . | . | . | . | . | . | . | . | . | . | . | . | . | . |
| 21-225_1F1_LC | . | . | . | . | . | S | V | . | . | . | . | . | . | . | . | . | . |
| 21-225_5F4_LC | . | . | R | . | . | . | . | . | . | . | . | . | . | . | . | . | . |
| 21-225_16H1_LC | . | . | R | . | . | . | . | . | . | . | . | . | . | . | . | . | . |
| 21-225_17H6_LC | . | . | . | . | . | S | T | . | . | . | . | . | . | . | . | . | . |
| 21-225_21F12_LC | . | . | . | . | . | S | . | . | . | . | . | . | . | . | . | . | . |
| 21-225_23A11_LC | . | . | R | . | . | . | . | . | . | . | . | . | . | . | . | . | . |
| 21-225_32B3_LC | . | R | . | . | . | . | . | . | . | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | K | F | S | G | S | G | S | G | - |
| 21-225_2C12_LC | . | . | . | . | . | . | . | . | Q | . | . | . | . | . | . | . | . |
| 21-225_17H5_LC | . | . | . | . | . | . | . | . | . | . | . | . | . | . | . | . | . |
| 21-225_16A11_LC | . | . | . | . | . | . | . | . | R | . | . | . | . | . | . | . | . |
| 21-225_20H7_LC | . | . | . | . | . | . | . | . | . | . | . | . | . | . | . | . | . |
| 21-225_20D12_LC | . | . | . | . | . | . | . | . | R | . | . | . | . | . | . | . | . |
| 21-225_22B12_LC | . | . | R | . | . | . | . | . | . | . | . | . | . | . | . | . | . |
| 21-225_27C5_LC | . | . | . | . | . | . | . | . | R | . | . | A | . | . | . | . | . |
| 21-225_27H12_LC | . | . | . | . | . | . | . | . | . | . | . | . | . | . | . | . | . |
| 21-225_35H8_LC | G | . | . | . | . | . | . | . | . | . | . | . | . | . | . | . | . |
| 21-225_15F8_LC | . | . | . | . | . | . | . | . | . | . | . | . | . | . | F | . | . |
| 21-225_20D6_LC | N | . | . | . | . | . | . | . | . | . | . | . | . | . | . | . | . |
| 21-225_15A2_LC | . | . | . | . | . | . | . | . | . | . | . | . | . | . | F | . | . |
| 21-225_5E11_LC | . | V | . | . | . | . | . | . | . | . | . | . | N | . | . | . | . |
| 21-225_2A4_LC | . | . | . | . | . | . | . | . | . | . | . | . | . | . | F | . | . |
| 21-225_16B8_LC | . | V | . | . | . | . | . | . | . | . | . | . | . | . | . | . | . |
| 21-225_14D6_LC | . | . | . | . | . | . | . | . | R | . | . | . | . | . | . | . | . |
| 21-225_1F1_LC | . | . | . | T | . | . | . | . | R | . | . | . | . | . | . | . | . |
| 21-225_5F4_LC | . | . | . | . | . | . | . | . | R | . | . | . | . | . | . | . | . |
| 21-225_16H1_LC | . | . | . | N | . | . | . | . | R | . | . | . | . | . | . | . | . |
| 21-225_17H6_LC | . | . | . | . | . | . | . | . | . | . | . | . | . | . | . | . | . |
| 21-225_21F12_LC | . | . | . | T | . | . | . | . | . | . | . | . | . | . | . | . | . |
| 21-225_23A11_LC | T | . | . | . | . | . | . | . | . | . | . | . | . | . | . | . | . |
| 21-225_32B3_LC | . | . | . | . | . | . | . | . | T | . | . | . | . | . | . | . | . |

FIGURE 56

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | K_FR3 | | | | | | | | | | | | | | | | |
| CONSENSUS | - | T | D | F | T | L | T | I | S | S | L | Q | P | E | D | F | A |
| 21-225_2C12_LC | . | . | . | . | . | . | . | . | . | . | . | . | . | . | . | . | . |
| 21-225_17H5_LC | . | . | . | . | . | . | . | . | . | . | . | . | . | . | . | . | . |
| 21-225_16A11_LC | . | . | S | . | . | . | . | . | . | . | . | . | . | . | . | . | . |
| 21-225_20H7_LC | . | . | . | . | . | . | . | . | . | . | . | . | . | . | . | . | . |
| 21-225_20D12_LC | . | . | S | . | . | . | . | . | . | . | . | . | . | . | . | . | . |
| 21-225_22B12_LC | . | . | . | . | N | . | . | . | . | . | . | . | . | . | . | L | . |
| 21-225_27C5_LC | . | . | S | . | . | . | . | . | . | . | . | . | . | . | . | . | . |
| 21-225_27H12_LC | . | . | . | . | . | . | . | . | . | . | . | . | . | . | . | . | . |
| 21-225_35H8_LC | . | . | . | . | . | . | . | . | . | . | . | . | . | . | . | . | . |
| 21-225_15F8_LC | . | . | . | . | . | . | . | . | . | . | . | . | . | . | . | . | . |
| 21-225_20D6_LC | . | . | . | . | . | . | . | . | . | . | . | . | . | . | . | . | . |
| 21-225_15A2_LC | . | . | . | . | . | F | P | . | . | . | . | . | . | . | . | . | . |
| 21-225_5E11_LC | . | . | . | . | . | . | . | . | . | . | . | . | . | . | . | . | . |
| 21-225_2A4_LC | . | . | . | . | . | . | . | . | . | . | . | . | . | . | . | . | . |
| 21-225_16B8_LC | . | . | . | . | . | . | . | . | . | . | . | . | . | . | . | . | . |
| 21-225_14D6_LC | . | . | S | . | . | . | . | . | . | . | . | . | . | . | . | . | . |
| 21-225_1F1_LC | . | . | . | . | . | . | . | . | . | . | . | . | . | . | . | . | . |
| 21-225_5F4_LC | . | . | S | . | . | . | . | V | . | . | . | . | . | . | . | . | . |
| 21-225_16H1_LC | . | . | S | . | . | . | . | . | . | . | . | . | . | . | . | . | . |
| 21-225_17H6_LC | . | . | . | . | . | . | . | . | . | . | . | . | . | . | . | . | . |
| 21-225_21F12_LC | . | . | . | . | . | . | . | . | . | . | . | . | . | . | . | . | . |
| 21-225_23A11_LC | . | . | . | . | . | . | . | . | . | . | . | . | . | . | . | . | . |
| 21-225_32B3_LC | . | . | . | . | . | . | . | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | Q | Q | Y | N | S | - | - | - | - | - | - | - | - |
| 21-225_2C12_LC | . | . | . | . | . | . | . | Y | . | . | . | . | . | . | . | . | . |
| 21-225_17H5_LC | . | . | . | . | . | . | . | H | . | . | . | . | . | . | . | . | . |
| 21-225_16A11_LC | . | . | . | . | L | . | . | . | . | . | . | . | . | . | . | . | . |
| 21-225_20H7_LC | . | . | . | . | . | . | . | Y | . | . | . | . | . | . | . | . | . |
| 21-225_20D12_LC | . | . | . | . | L | . | . | . | . | . | . | . | . | . | . | . | . |
| 21-225_22B12_LC | . | . | . | . | . | . | . | H | . | . | . | . | . | . | . | . | . |
| 21-225_27C5_LC | . | . | C | . | . | . | . | D | . | . | . | . | . | . | . | . | . |
| 21-225_27H12_LC | . | . | . | . | . | . | . | Y | . | . | . | . | . | . | . | . | . |
| 21-225_35H8_LC | . | . | . | . | . | . | . | . | . | . | . | . | . | . | . | . | . |
| 21-225_15F8_LC | A | . | . | . | . | . | . | H | . | . | . | . | . | . | . | . | . |
| 21-225_20D6_LC | . | . | F | . | . | . | . | H | . | . | . | . | . | . | . | . | . |
| 21-225_15A2_LC | N | . | . | . | . | . | . | Y | . | . | . | . | . | . | . | . | . |
| 21-225_5E11_LC | . | . | . | . | H | . | . | H | . | . | . | . | . | . | . | . | . |
| 21-225_2A4_LC | . | . | . | . | . | . | . | . | . | . | . | . | . | . | . | . | . |
| 21-225_16B8_LC | . | . | . | . | H | . | . | H | . | . | . | . | . | . | . | . | . |
| 21-225_14D6_LC | . | . | . | . | L | . | . | . | . | . | . | . | . | . | . | . | . |
| 21-225_1F1_LC | . | . | . | . | . | . | . | . | . | . | . | . | . | . | . | . | . |
| 21-225_5F4_LC | . | . | . | . | L | . | . | . | R | . | . | . | . | . | . | . | . |
| 21-225_16H1_LC | . | . | . | . | L | . | . | H | . | . | . | . | . | . | . | . | . |
| 21-225_17H6_LC | I | . | . | . | . | . | . | H | . | . | . | . | . | . | . | . | . |
| 21-225_21F12_LC | . | . | . | . | . | . | . | . | . | . | . | . | . | . | . | . | . |
| 21-225_23A11_LC | . | . | . | . | . | . | . | . | . | . | . | . | . | . | . | . | . |
| 21-225_32B3_LC | . | . | . | . | . | . | . | . | . | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | K_CDR3 | | | | | | | | | | | | | | | | |
| CONSENSUS | - | - | - | - | - | - | - | - | - | - | - | - | - | - | - | Y | P |
| 21-225_2C12_LC | . | . | . | . | . | . | . | . | . | . | . | . | . | . | . | . | . |
| 21-225_17H5_LC | . | . | . | . | . | . | . | . | . | . | . | . | . | . | . | . | . |
| 21-225_16A11_LC | . | . | . | . | . | . | . | . | . | . | . | . | . | . | . | . | . |
| 21-225_20H7_LC | . | . | . | . | . | . | . | . | . | . | . | . | . | . | . | . | . |
| 21-225_20D12_LC | . | . | . | . | . | . | . | . | . | . | . | . | . | . | . | . | . |
| 21-225_22B12_LC | . | . | . | . | . | . | . | . | . | . | . | . | . | . | . | . | . |
| 21-225_27C5_LC | . | . | . | . | . | . | . | . | . | . | . | . | . | . | . | [illegible] | . |
| 21-225_27H12_LC | . | . | . | . | . | . | . | . | . | . | . | . | . | . | . | . | . |
| 21-225_35H8_LC | . | . | . | . | . | . | . | . | . | . | . | . | . | . | . | . | . |
| 21-225_15F8_LC | . | . | . | . | . | . | . | . | . | . | . | . | . | . | . | . | . |
| 21-225_20D6_LC | . | . | . | . | . | . | . | . | . | . | . | . | . | . | . | [illegible] | . |
| 21-225_15A2_LC | . | . | . | . | . | . | . | . | . | . | . | . | . | . | . | . | . |
| 21-225_5E11_LC | . | . | . | . | . | . | . | . | . | . | . | . | . | . | . | . | . |
| 21-225_2A4_LC | . | . | . | . | . | . | . | . | . | . | . | . | . | . | . | . | . |
| 21-225_16B8_LC | . | . | . | . | . | . | . | . | . | . | . | . | . | . | . | . | . |
| 21-225_14D6_LC | . | . | . | . | . | . | . | . | . | . | . | . | . | . | . | . | . |
| 21-225_1F1_LC | . | . | . | . | . | . | . | . | . | . | . | . | . | . | . | . | . |
| 21-225_5F4_LC | . | . | . | . | . | . | . | . | . | . | . | . | . | . | . | . | . |
| 21-225_16H1_LC | . | . | . | . | . | . | . | . | . | . | . | . | . | . | . | . | . |
| 21-225_17H6_LC | . | . | . | . | . | . | . | . | . | . | . | . | . | . | . | . | . |
| 21-225_21F12_LC | . | . | . | . | . | . | . | . | . | . | . | . | . | . | . | [illegible] | . |
| 21-225_23A11_LC | . | . | . | . | . | . | . | . | . | . | . | . | . | . | . | . | . |
| 21-225_32B3_LC | . | . | . | . | . | . | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | K_FR4 | | | | | | | | | | | | |
| CONSENSUS | F | T | F | G | P | G | T | K | V | D | I | K | R |
| 21-225_2C12_LC | I | . | . | . | Q | . | . | R | L | E | . | . | . |
| 21-225_17H5_LC | . | . | . | . | . | . | . | . | . | . | . | . | . |
| 21-225_16A11_LC | . | . | . | . | . | . | . | . | . | . | . | . | . |
| 21-225_20H7_LC | . | . | . | . | . | . | . | . | M | . | F | . | . |
| 21-225_20D12_LC | . | . | . | . | . | . | . | . | . | . | . | . | . |
| 21-225_22B12_LC | . | . | . | . | . | . | . | . | . | . | F | . | . |
| 21-225_27C5_LC | S | . | . | . | Q | . | . | . | . | E | . | . | . |
| 21-225_27H12_LC | . | . | . | . | . | . | . | . | . | N | . | N | . |
| 21-225_35H8_LC | . | . | . | . | . | . | . | . | . | . | F | . | . |
| 21-225_15F8_LC | . | . | . | . | S | . | . | . | . | . | . | Q | . |
| 21-225_20D6_LC | . | . | . | . | . | . | . | . | . | . | . | . | . |
| 21-225_15A2_LC | . | . | . | . | . | . | . | Q | . | . | V | . | . |
| 21-225_5E11_LC | . | . | . | . | . | . | . | . | . | . | . | . | . |
| 21-225_2A4_LC | . | . | . | . | . | . | . | . | . | . | . | . | . |
| 21-225_16B8_LC | . | . | . | . | . | . | . | . | . | . | . | V | . |
| 21-225_14D6_LC | . | . | . | . | . | . | . | . | . | . | . | N | . |
| 21-225_1F1_LC | L | . | . | . | Q | . | . | . | . | E | . | . | . |
| 21-225_5F4_LC | . | . | . | . | . | . | . | . | . | . | . | . | . |
| 21-225_16H1_LC | . | . | . | . | . | . | . | . | . | . | V | . | . |
| 21-225_17H6_LC | . | . | . | . | . | . | . | . | . | . | . | Q | . |
| 21-225_21F12_LC | . | . | . | . | . | . | . | . | . | . | . | . | . |
| 21-225_23A11_LC | . | . | . | . | . | . | . | . | . | . | L | . | . |
| 21-225_32B3_LC | . | . | . | . | . | . | . | . | . | . | . | . | . |

**FIGURE 56**

**Table 26**

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | | | | | H_FR1 | |
| CONSENSUS | E | V | Q | L | L | E | S | - | G | G | G | L | V | Q | P | G | G |
| 21-225_23C7_HC | . | . | . | . | . | . | . | . | . | . | . | . | . | . | . | . | . |
| 21-225_23E7_HC | . | . | . | . | . | . | . | . | . | . | . | . | . | . | . | . | . |
| 21-225_30A1_HC | . | . | . | . | . | . | . | . | . | . | . | . | . | . | . | . | . |
| 21-225_25D10_HC | . | . | . | . | . | . | . | . | . | . | . | . | . | . | . | . | . |
| 21-225_33A5_HC | . | . | . | . | . | . | . | . | . | . | . | . | . | . | . | . | . |
| 21-225_26C2_HC | . | . | . | . | . | . | . | . | . | . | . | . | . | . | . | . | . |
| 21-225_26D9_HC | . | . | . | . | . | . | . | . | . | . | . | . | . | . | . | . | . |
| 21-225_31G1_HC | A | . | . | . | . | . | . | . | . | . | D | . | . | . | . | . | . |
| 21-225_10C5_HC | . | . | . | . | . | . | . | . | . | . | . | . | . | . | . | . | . |
| 21-225_18F7_HC | . | . | . | . | . | . | . | . | . | . | . | . | E | . | . | . | . |
| 21-225_20G11_HC | . | . | . | . | . | . | . | . | . | . | . | . | E | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | S | L | R | L | S | C | A | A | S | G | - | F | T | F | S | S | - |
| 21-225_23C7_HC | . | . | . | . | . | . | . | . | . | . | . | . | . | . | . | . | . |
| 21-225_23E7_HC | . | . | . | . | . | . | T | . | . | . | . | . | . | . | . | . | . |
| 21-225_30A1_HC | . | . | . | . | . | . | . | . | . | . | . | . | . | . | . | . | . |
| 21-225_25D10_HC | . | . | . | . | . | . | . | . | . | . | . | . | . | . | . | . | . |
| 21-225_33A5_HC | . | . | . | . | . | . | . | . | . | . | . | . | . | . | . | . | . |
| 21-225_26C2_HC | . | . | . | . | . | . | . | . | . | . | . | . | . | . | . | . | . |
| 21-225_26D9_HC | . | . | . | . | . | . | . | . | . | . | . | . | . | . | . | . | . |
| 21-225_31G1_HC | . | . | . | . | . | . | . | . | . | . | . | . | . | . | . | . | . |
| 21-225_10C5_HC | . | . | . | . | . | . | . | . | . | . | . | . | . | . | T | . | . |
| 21-225_18F7_HC | . | . | . | . | . | . | . | . | . | . | . | . | . | . | R | N | . |
| 21-225_20G11_HC | . | . | . | . | . | . | . | . | . | . | . | . | . | . | R | . | . |

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | H_CDR1 | | | | | | | | | | | | H_FR2 | | |
| CONSENSUS | - | - | - | - | Y | V | M | N | W | V | R | Q | A | P | G | K | G |
| 21-225_23C7_HC | . | . | . | . | . | . | . | S | . | . | . | . | . | . | . | . | . |
| 21-225_23E7_HC | . | . | . | . | . | A | . | . | . | . | . | . | . | . | . | . | . |
| 21-225_30A1_HC | . | . | . | . | . | . | . | . | . | . | . | . | . | . | . | . | . |
| 21-225_25D10_HC | . | . | . | . | . | . | . | . | . | . | . | . | . | . | . | . | . |
| 21-225_33A5_HC | . | . | . | . | . | . | I | . | . | . | . | . | . | . | . | . | . |
| 21-225_26C2_HC | . | . | . | . | . | . | . | . | . | . | . | . | . | . | . | . | . |
| 21-225_26D9_HC | . | . | . | . | . | A | . | . | . | . | . | . | . | . | . | . | . |
| 21-225_31G1_HC | . | . | . | . | . | . | . | . | . | . | . | . | . | . | . | . | . |
| 21-225_10C5_HC | . | . | . | . | . | . | . | . | . | . | . | . | . | . | . | . | . |
| 21-225_18F7_HC | . | . | . | . | . | . | . | . | . | . | . | . | . | . | . | . | . |
| 21-225_20G11_HC | . | . | . | . | . | . | . | . | . | . | . | . | . | . | . | . | . |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | H_CDR2 | | |
| CONSENSUS | L | E | W | V | S | A | I | S | G | S | - | - | - | G | G | S | T |
| 21-225_23C7_HC | . | . | . | . | . | . | M | . | . | . | . | . | . | . | . | . | . |
| 21-225_23E7_HC | . | . | . | . | . | . | . | . | . | . | . | . | . | . | . | F | . |
| 21-225_30A1_HC | . | . | . | . | . | . | . | . | . | . | . | . | . | . | . | . | . |
| 21-225_25D10_HC | . | . | . | . | . | V | . | . | . | . | . | . | . | . | . | . | . |
| 21-225_33A5_HC | . | . | . | . | . | . | . | . | . | . | . | . | . | . | V | . | . |
| 21-225_26C2_HC | . | . | . | . | . | . | . | . | . | . | . | . | . | . | . | . | . |
| 21-225_26D9_HC | . | . | . | . | . | . | . | . | . | . | . | . | . | . | . | G | . |
| 21-225_31G1_HC | . | . | . | . | . | . | . | . | . | . | . | . | . | . | . | N | . |
| 21-225_10C5_HC | . | . | . | . | . | . | . | . | . | . | . | . | . | . | . | [illegible] | . |
| 21-225_18F7_HC | . | . | . | . | . | G | . | . | . | . | . | . | . | . | . | [illegible] | . |
| 21-225_20G11_HC | . | . | . | . | . | G | . | . | . | . | . | . | . | . | . | . | . |

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | A | D | S | V | K | G | R | F | T | I | S | R | D | N | S |
| 21-225_23C7_HC | . | . | V | . | . | . | . | . | . | . | . | . | . | . | . | . | . |
| 21-225_23E7_HC | . | . | . | . | . | . | . | . | . | . | . | . | . | . | . | . | . |
| 21-225_30A1_HC | . | . | . | . | . | . | . | . | . | . | . | . | . | . | N | . | . |
| 21-225_25D10_HC | D | . | . | . | . | . | . | . | . | . | . | . | . | . | . | . | . |
| 21-225_33A5_HC | . | . | . | . | . | . | . | . | . | . | . | . | . | . | . | . | . |
| 21-225_26C2_HC | . | . | . | . | . | . | . | . | . | . | . | . | . | . | . | . | . |
| 21-225_26D9_HC | . | . | . | . | . | . | . | . | . | . | . | . | . | . | . | . | . |
| 21-225_31G1_HC | . | . | . | . | . | . | . | . | . | . | . | . | . | . | . | . | . |
| 21-225_10C5_HC | N | . | . | . | . | . | . | . | . | . | . | . | . | . | . | . | . |
| 21-225_18F7_HC | . | . | . | . | . | . | . | D | . | . | . | . | . | . | . | . | . |
| 21-225_20G11_HC | . | . | . | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | | | | H_FR3 | | | | | | | | | | |
| CONSENSUS | K | N | T | L | Y | L | Q | M | N | S | L | R | A | E | D | T | A |
| 21-225_23C7_HC | . | . | . | . | . | . | . | . | . | . | . | . | . | . | . | . | . |
| 21-225_23E7_HC | R | . | . | . | . | . | . | . | . | . | . | . | . | . | . | . | . |
| 21-225_30A1_HC | . | . | . | . | . | . | . | . | . | . | . | . | . | . | . | . | . |
| 21-225_25D10_HC | . | . | . | . | . | . | . | . | . | . | . | . | . | . | . | . | . |
| 21-225_33A5_HC | . | . | . | . | . | . | . | . | . | . | . | . | . | . | . | . | . |
| 21-225_26C2_HC | . | . | . | . | . | . | . | . | . | . | . | . | . | . | . | . | . |
| 21-225_26D9_HC | . | . | . | . | . | . | . | . | . | . | . | . | . | . | . | . | . |
| 21-225_31G1_HC | . | . | . | . | F | . | . | . | . | . | . | . | . | . | . | . | . |
| 21-225_10C5_HC | . | . | . | . | . | . | . | . | . | . | . | . | . | . | . | . | . |
| 21-225_18F7_HC | . | . | . | . | . | . | . | . | . | . | . | . | . | . | . | . | . |
| 21-225_20G11_HC | . | . | . | . | . | . | . | . | . | . | . | . | . | . | . | . | . |

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | A | R | T | G | V | - | - | - | - | - | - | - | - |
| 21-225_23C7_HC | . | . | . | . | . | . | . | . | . | . | . | . | . | . | . | . | . |
| 21-225_23E7_HC | . | . | . | . | . | . | . | S | G | . | . | . | . | . | . | . | . |
| 21-225_30A1_HC | . | . | . | . | . | . | . | . | . | . | . | . | . | . | . | . | . |
| 21-225_25D10_HC | . | . | . | . | . | . | . | . | . | . | . | . | . | . | . | . | . |
| 21-225_33A5_HC | . | . | . | . | . | . | . | S | L | . | . | . | . | . | . | . | . |
| 21-225_26C2_HC | . | F | . | . | . | . | . | . | . | . | . | . | . | . | . | . | . |
| 21-225_26D9_HC | . | . | . | . | . | . | . | . | G | . | . | . | . | . | . | . | . |
| 21-225_31G1_HC | . | . | . | . | . | . | . | S | L | . | . | . | . | . | . | . | . |
| 21-225_10C5_HC | . | . | . | . | . | . | . | . | . | . | . | . | . | . | . | . | . |
| 21-225_18F7_HC | . | . | . | . | . | . | . | S | L | . | . | . | . | . | . | . | . |
| 21-225_20G11_HC | . | . | . | . | . | . | . | S | L | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | H_CDR3 | | | | | | | | | | | | | | | | |
| CONSENSUS | - | - | - | - | - | - | - | - | - | - | - | - | - | - | - | - | F |
| 21-225_23C7_HC | . | . | . | . | . | . | . | . | . | . | . | . | . | . | . | . | . |
| 21-225_23E7_HC | . | . | . | . | . | . | . | . | . | . | . | . | . | . | . | . | . |
| 21-225_30A1_HC | . | . | . | . | . | . | . | . | . | . | . | . | . | . | . | . | . |
| 21-225_25D10_HC | . | . | . | . | . | . | . | . | . | . | . | . | . | . | . | . | . |
| 21-225_33A5_HC | . | . | . | . | . | . | . | . | . | . | . | . | . | . | . | . | . |
| 21-225_26C2_HC | . | . | . | . | . | . | . | . | . | . | . | . | . | . | . | . | . |
| 21-225_26D9_HC | . | . | . | . | . | . | . | . | . | . | . | . | . | . | . | . | . |
| 21-225_31G1_HC | . | . | . | . | . | . | . | . | . | . | . | . | . | . | . | . | . |
| 21-225_10C5_HC | . | . | . | . | . | . | . | . | . | . | . | . | . | . | . | . | . |
| 21-225_18F7_HC | . | . | . | . | . | . | . | . | . | . | . | . | . | . | . | . | . |
| 21-225_20G11_HC | . | . | . | . | . | . | . | . | . | . | . | . | . | . | . | . | . |

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | H_FR4 | | | | | | | | | | |
| CONSENSUS | D | Y | W | G | Q | G | T | L | V | T | V | S | S |
| 21-225_23C7_HC | . | . | . | . | . | . | . | . | . | I | . | . | . |
| 21-225_23E7_HC | . | . | . | . | . | . | . | . | . | . | . | . | . |
| 21-225_30A1_HC | . | . | . | . | . | . | . | . | . | . | . | . | . |
| 21-225_25D10_HC | . | . | . | . | . | . | . | . | . | . | . | . | . |
| 21-225_33A5_HC | . | . | . | . | . | . | . | . | . | . | . | . | . |
| 21-225_26C2_HC | . | . | . | . | . | . | . | . | . | . | . | . | . |
| 21-225_26D9_HC | . | . | . | . | . | . | . | . | . | . | . | . | . |
| 21-225_31G1_HC | . | . | . | . | . | . | . | . | . | . | . | . | . |
| 21-225_10C5_HC | . | . | . | . | . | . | . | . | . | . | . | . | . |
| 21-225_18F7_HC | . | S | . | . | . | . | . | . | . | . | . | . | . |
| 21-225_20G11_HC | . | S | . | . | . | . | . | . | . | . | . | . | . |

FIGURE 56

Table 27

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | H_FR1 | | | | | | | | | | | | | | | | |
| CONSENSUS | E | V | Q | L | L | E | S | - | G | G | G | L | V | Q | P | G | G |
| 21-225_12D8_HC | . | . | . | . | . | . | . | . | . | . | . | . | . | . | . | . | . |
| 21-225_30E9_HC | . | . | . | V | . | . | . | . | . | . | . | . | . | . | . | . | . |
| 21-225_31H5_HC | . | . | . | . | . | . | . | . | . | . | . | . | . | . | . | . | . |
| 21-225_31A8_HC | . | . | . | . | . | . | . | . | . | . | . | . | . | . | . | . | . |
| 21-225_30D1_HC | . | . | . | V | . | . | . | . | . | . | . | . | . | . | . | . | . |
| 21-225_31B8_HC | . | . | . | . | . | . | . | . | . | . | . | . | . | . | . | . | . |
| 21-225_30G11_HC | . | . | . | . | . | . | . | . | . | . | . | . | . | . | . | . | . |
| 21-225_30E3_HC | . | . | . | V | . | . | . | . | . | . | . | . | . | . | . | . | . |
| 21-225_31H3_HC | . | . | . | V | . | . | . | . | . | . | . | . | . | . | . | . | . |
| 21-225_32G12_HC | . | . | . | . | . | . | . | . | . | . | . | . | L | . | . | . | . |
| 21-225_34G9_HC | . | . | . | . | V | . | . | . | . | . | . | . | . | . | . | . | . |
| 21-225_36C5_HC | . | . | . | . | V | . | . | . | . | . | . | . | . | . | . | . | . |
| 21-225_33A4_HC | . | . | . | . | . | . | . | . | . | . | . | . | . | . | S | . | . |
| 21-225_33F1_HC | . | . | . | . | . | . | . | . | . | . | . | . | L | . | . | . | . |
| 21-225_33A7_HC | . | . | . | . | . | . | . | . | . | . | . | . | . | . | . | . | . |
| 21-225_34A6_HC | . | . | . | . | . | . | . | . | . | . | . | . | . | . | . | . | . |
| 21-225_35B7_HC | . | . | . | . | . | . | . | . | . | . | . | . | . | . | . | . | . |
| 21-225_34H11_HC | . | . | . | . | . | . | . | . | . | . | . | . | . | . | . | . | . |
| 21-225_33G7_HC | . | . | . | . | . | . | . | . | . | . | . | . | . | . | . | . | . |
| 21-225_35D1_HC | . | . | . | . | . | . | . | . | . | . | . | . | . | . | . | . | . |
| 21-225_35F11_HC | . | . | . | . | . | . | . | . | . | . | . | . | . | . | . | . | . |
| 21-225_34G8_HC | . | . | . | . | . | . | . | . | . | . | . | . | . | . | . | . | . |
| 21-225_36A5_HC | . | . | . | . | . | . | . | . | . | . | . | . | . | . | . | . | . |
| 21-225_4G12_HC | . | . | . | . | . | . | . | . | . | . | . | . | . | . | . | . | . |
| 21-225_17F12_HC | . | . | . | . | . | . | . | . | . | . | . | . | . | . | . | . | . |
| 21-225_21H4_HC | . | . | . | . | . | . | . | . | . | . | . | . | . | . | . | . | . |
| 21-225_24B1_HC | . | . | . | . | . | . | . | . | . | . | . | . | . | . | . | . | . |
| 21-225_32G1_HC | . | . | . | . | . | . | . | . | . | . | . | . | A | . | . | . | . |
| 21-225_33B8_HC | . | . | . | . | . | . | . | . | . | . | . | . | . | . | . | . | . |
| 21-225_35A6_HC | . | . | . | . | . | . | . | . | . | . | . | . | . | . | . | . | . |

FIGURE 56

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | S | L | R | L | S | C | A | A | S | G | - | F | T | F | S | S | - |
| 21-225_12D8_HC | . | . | . | . | . | . | . | . | . | . | . | . | . | . | . | . | . |
| 21-225_30E9_HC | . | . | . | . | . | . | . | . | . | . | . | . | . | . | . | . | . |
| 21-225_31H5_HC | . | . | . | . | . | . | . | . | . | . | . | . | . | . | . | . | . |
| 21-225_31A8_HC | . | . | . | . | . | . | . | . | . | . | . | . | . | . | . | . | . |
| 21-225_30D1_HC | . | . | . | . | . | . | . | . | . | . | . | . | . | . | . | . | . |
| 21-225_31B8_HC | . | . | . | . | . | . | . | . | . | . | . | . | . | . | . | . | . |
| 21-225_30G11_HC | . | . | . | . | . | . | . | . | . | . | . | . | . | . | . | . | . |
| 21-225_30E3_HC | . | . | . | . | . | . | . | . | . | . | . | . | . | . | . | . | . |
| 21-225_31H3_HC | . | . | . | . | . | . | . | . | . | . | . | . | . | . | . | . | . |
| 21-225_32G12_HC | . | . | . | . | . | . | . | . | . | . | . | . | . | . | N | . | . |
| 21-225_34G9_HC | . | . | . | . | . | . | . | . | . | . | . | . | . | . | . | . | . |
| 21-225_36C5_HC | . | . | . | . | . | . | . | . | . | . | . | . | . | . | . | . | . |
| 21-225_33A4_HC | . | . | . | . | . | . | E | . | . | . | . | . | I | . | . | . | . |
| 21-225_33F1_HC | . | . | . | . | . | . | . | . | . | . | . | . | . | . | . | . | . |
| 21-225_33A7_HC | . | . | . | . | . | . | . | . | . | . | . | . | . | . | . | . | . |
| 21-225_34A6_HC | . | . | . | . | . | . | . | . | . | . | . | . | . | . | N | N | . |
| 21-225_35B7_HC | . | . | . | . | . | . | . | . | . | . | . | . | . | . | . | . | . |
| 21-225_34H11_HC | . | . | . | . | . | . | . | . | . | . | . | . | . | . | . | . | . |
| 21-225_33G7_HC | . | . | . | . | . | . | . | . | . | . | . | . | . | . | . | N | . |
| 21-225_35D1_HC | . | . | . | . | . | . | . | . | . | . | . | . | . | . | . | . | . |
| 21-225_35F11_HC | . | . | . | . | . | . | . | . | . | . | . | . | . | . | . | . | . |
| 21-225_34G8_HC | . | . | . | . | . | . | . | . | . | . | . | . | . | . | . | . | . |
| 21-225_36A5_HC | . | . | . | . | . | . | . | . | . | . | . | . | . | . | N | N | . |
| 21-225_4G12_HC | . | . | . | . | . | . | . | . | . | . | . | . | . | . | . | . | . |
| 21-225_17F12_HC | . | . | . | . | . | . | . | . | . | . | . | . | . | . | . | . | . |
| 21-225_21H4_HC | . | . | . | . | . | . | . | . | . | . | . | . | . | . | . | . | . |
| 21-225_24B1_HC | . | . | . | . | . | . | . | . | . | . | . | . | . | . | . | . | . |
| 21-225_32G1_HC | . | . | . | . | . | . | . | . | . | . | . | . | . | . | . | . | . |
| 21-225_33B8_HC | . | . | . | . | . | . | . | . | . | . | . | . | . | . | . | . | . |
| 21-225_35A6_HC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | H_CDR1 | | | | | | | | H_FR2 | | | | | | | | |
| CONSENSUS | - | - | - | - | Y | A | M | S | W | V | R | Q | A | P | G | K | G |
| 21-225_12D8_HC | . | . | . | . | . | V | . | . | . | . | . | . | . | . | . | . | . |
| 21-225_30E9_HC | . | . | . | . | C | . | . | N | . | . | . | . | . | . | . | . | . |
| 21-225_31H5_HC | . | . | . | . | . | . | . | . | . | . | . | . | . | . | . | . | . |
| 21-225_31A8_HC | . | . | . | . | . | . | . | . | . | . | . | . | . | . | . | . | . |
| 21-225_30D1_HC | . | . | . | . | . | . | . | . | . | . | . | . | . | . | . | . | . |
| 21-225_31B8_HC | . | . | . | . | . | . | . | . | . | . | . | . | . | . | . | . | . |
| 21-225_30G11_HC | . | . | . | . | . | . | . | . | . | . | . | . | . | . | . | . | . |
| 21-225_30E3_HC | . | . | . | . | . | . | . | N | . | . | . | . | . | . | . | . | . |
| 21-225_31H3_HC | . | . | . | . | . | . | . | N | . | . | . | . | . | . | . | . | . |
| 21-225_32G12_HC | . | . | . | . | . | . | . | . | . | . | . | . | . | . | . | . | . |
| 21-225_34G9_HC | . | . | . | . | . | . | . | . | . | . | . | . | . | . | . | . | . |
| 21-225_36C5_HC | . | . | . | . | . | . | . | N | . | . | . | . | . | . | . | . | . |
| 21-225_33A4_HC | . | . | . | . | . | . | . | N | . | . | . | . | V | . | . | . | . |
| 21-225_33F1_HC | . | . | . | . | . | . | . | . | . | . | . | . | . | . | . | . | . |
| 21-225_33A7_HC | . | . | . | . | C | . | . | . | . | . | . | . | . | . | . | . | . |
| 21-225_34A6_HC | . | . | . | . | . | . | . | N | . | . | . | . | . | . | . | . | . |
| 21-225_35B7_HC | . | . | . | . | . | . | . | . | . | . | . | . | . | . | . | . | . |
| 21-225_34H11_HC | . | . | . | . | . | . | . | N | . | . | . | . | . | . | . | E | . |
| 21-225_33G7_HC | . | . | . | . | . | . | . | . | . | . | . | . | . | . | . | . | . |
| 21-225_35D1_HC | . | . | . | . | . | . | . | . | . | . | . | . | . | . | . | . | . |
| 21-225_35F11_HC | . | . | . | . | . | . | . | . | . | . | . | . | . | . | . | . | . |
| 21-225_34G8_HC | . | . | . | . | . | . | . | . | . | . | . | . | . | . | . | . | . |
| 21-225_36A5_HC | . | . | . | . | . | . | . | N | . | . | . | . | . | . | . | . | . |
| 21-225_4G12_HC | . | . | . | . | . | . | . | . | . | . | . | . | . | . | . | . | . |
| 21-225_17F12_HC | . | . | . | . | . | . | . | . | . | . | . | . | . | . | . | . | . |
| 21-225_21H4_HC | . | . | . | . | . | . | . | . | . | . | . | . | . | . | . | . | . |
| 21-225_24B1_HC | . | . | . | . | . | . | . | . | . | . | . | . | . | . | . | . | . |
| 21-225_32G1_HC | . | . | . | . | . | . | . | . | . | . | . | . | . | . | . | . | . |
| 21-225_33B8_HC | . | . | . | . | . | . | . | . | . | . | . | . | . | . | . | . | . |
| 21-225_35A6_HC | . | . | . | . | . | . | . | . | . | . | . | . | . | . | . | . | . |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | H_CDR2 | | |
| CONSENSUS | L | E | W | V | S | A | I | S | G | R | - | - | - | G | G | S | T |
| 21-225_12D8_HC | . | . | . | . | . | T | . | . | V | G | . | . | . | . | . | . | . |
| 21-225_30E9_HC | . | . | . | . | . | . | . | . | . | . | . | . | . | . | . | . | . |
| 21-225_31H5_HC | . | . | . | . | . | . | . | . | . | . | . | . | . | . | . | . | I |
| 21-225_31A8_HC | . | . | . | . | . | . | . | . | . | . | . | . | . | . | . | . | . |
| 21-225_30D1_HC | . | . | . | . | . | . | . | . | . | . | . | . | . | . | . | . | . |
| 21-225_31B8_HC | . | . | . | . | . | . | . | . | . | . | . | . | . | . | . | . | . |
| 21-225_30G11_HC | . | . | . | . | . | . | . | . | . | . | . | . | . | . | . | . | . |
| 21-225_30E3_HC | . | . | . | . | . | . | . | . | . | . | . | . | . | . | . | . | . |
| 21-225_31H3_HC | . | . | . | . | . | . | . | . | . | . | . | . | . | . | . | N | . |
| 21-225_32G12_HC | . | . | . | . | . | S | . | . | . | . | . | . | . | . | . | . | . |
| 21-225_34G9_HC | . | . | . | . | . | . | . | . | . | . | . | . | . | . | . | . | . |
| 21-225_36C5_HC | . | . | . | . | . | . | . | . | . | . | . | . | . | . | . | . | . |
| 21-225_33A4_HC | . | . | . | . | . | . | . | . | R | . | . | . | . | . | . | . | . |
| 21-225_33F1_HC | . | . | . | . | . | S | . | . | . | . | . | . | . | . | . | . | . |
| 21-225_33A7_HC | . | . | . | . | . | . | . | . | . | . | . | . | . | . | . | . | . |
| 21-225_34A6_HC | . | . | . | . | . | . | . | . | R | . | . | . | . | . | . | . | . |
| 21-225_35B7_HC | . | . | . | . | . | . | . | . | . | . | . | . | . | . | . | . | . |
| 21-225_34H11_HC | . | . | . | . | . | . | . | . | . | . | . | . | . | . | . | . | . |
| 21-225_33G7_HC | . | . | . | . | . | . | . | . | R | . | . | . | . | . | . | . | . |
| 21-225_35D1_HC | . | . | . | . | . | . | . | . | . | . | . | . | . | . | . | . | . |
| 21-225_35F11_HC | . | . | . | . | . | . | . | . | . | . | . | . | . | . | . | . | . |
| 21-225_34G8_HC | . | . | . | . | . | . | . | . | . | . | . | . | . | . | . | . | . |
| 21-225_36A5_HC | . | . | . | . | . | . | . | . | R | . | . | . | . | . | . | N | . |
| 21-225_4G12_HC | . | . | . | . | . | T | L | . | . | . | . | . | . | . | . | . | . |
| 21-225_17F12_HC | . | . | . | . | . | . | . | . | . | G | . | . | . | . | . | . | . |
| 21-225_21H4_HC | . | . | . | . | . | . | L | . | . | . | . | . | . | . | . | . | . |
| 21-225_24B1_HC | . | . | . | . | . | . | . | . | . | . | . | . | . | . | . | . | . |
| 21-225_32G1_HC | . | . | . | . | . | . | . | . | . | . | . | . | . | . | . | . | . |
| 21-225_33B8_HC | . | . | . | . | . | . | . | . | . | . | . | . | . | . | . | . | . |
| 21-225_35A6_HC | . | . | . | . | . | T | . | . | . | . | . | . | . | . | . | . | . |

**FIGURE 56**

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | F | H | A | D | S | V | K | G | R | F | T | I | S | R | D | N | S |
| 21-225_12D8_HC | Y | Y | . | . | . | . | . | . | . | . | . | . | . | . | . | . | . |
| 21-225_30E9_HC | . | Y | . | . | . | . | . | . | . | . | . | . | . | . | . | . | . |
| 21-225_31H5_HC | . | . | . | . | . | . | . | . | . | . | . | . | . | . | . | . | . |
| 21-225_31A8_HC | . | . | . | . | . | . | . | . | . | . | . | . | . | . | . | . | . |
| 21-225_30D1_HC | . | . | . | . | . | . | . | . | . | . | . | . | . | . | . | . | . |
| 21-225_31B8_HC | . | . | . | . | . | . | . | . | . | . | . | . | . | . | . | . | . |
| 21-225_30G11_HC | . | N | . | . | . | . | . | . | . | . | . | . | . | . | . | . | . |
| 21-225_30E3_HC | . | Y | . | . | . | E | . | . | . | . | . | . | . | . | . | . | . |
| 21-225_31H3_HC | . | Y | . | . | . | . | . | . | . | . | . | . | . | . | . | . | . |
| 21-225_32G12_HC | . | . | . | . | . | . | . | . | . | . | . | . | . | . | . | . | . |
| 21-225_34G9_HC | . | . | . | . | . | . | . | . | . | . | . | . | . | . | . | . | . |
| 21-225_36C5_HC | . | . | . | . | . | . | . | . | . | . | . | . | . | . | . | . | . |
| 21-225_33A4_HC | . | Y | . | . | . | . | . | . | . | . | . | . | . | . | . | . | . |
| 21-225_33F1_HC | . | . | . | . | . | . | . | . | . | . | . | . | . | . | . | . | . |
| 21-225_33A7_HC | . | . | . | . | . | . | . | . | . | . | . | . | . | . | . | . | . |
| 21-225_34A6_HC | . | Y | . | . | . | . | . | . | . | . | . | . | . | . | . | . | . |
| 21-225_35B7_HC | . | . | . | . | . | . | . | . | . | . | . | . | . | . | . | . | . |
| 21-225_34H11_HC | . | Y | . | . | . | . | . | . | . | . | . | . | . | . | . | . | . |
| 21-225_33G7_HC | . | Y | . | . | . | . | . | . | Q | . | . | . | . | . | . | . | . |
| 21-225_35D1_HC | . | . | . | . | . | . | . | . | . | . | . | . | . | . | . | . | . |
| 21-225_35F11_HC | . | . | . | . | . | M | . | . | . | . | . | . | . | . | . | . | . |
| 21-225_34G8_HC | . | . | . | . | . | . | . | . | . | . | . | . | . | . | . | . | . |
| 21-225_36A5_HC | . | Y | . | . | . | . | . | . | . | . | . | . | . | . | . | . | . |
| 21-225_4G12_HC | Y | Y | . | . | . | . | . | . | . | S | . | . | . | . | . | . | . |
| 21-225_17F12_HC | Y | Y | . | . | . | . | . | . | . | V | . | . | . | . | . | . | . |
| 21-225_21H4_HC | Y | Y | . | . | . | . | . | . | . | . | . | . | . | . | . | . | . |
| 21-225_24B1_HC | Y | Y | . | . | . | . | . | . | . | . | . | . | . | . | . | . | . |
| 21-225_32G1_HC | . | . | . | . | . | . | . | . | . | . | . | . | . | . | . | . | . |
| 21-225_33B8_HC | . | . | . | . | . | . | . | . | . | . | . | . | . | . | . | . | . |
| 21-225_35A6_HC | . | . | . | . | . | . | . | . | . | . | . | . | . | . | . | . | . |

FIGURE 56

H_FR3

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| CONSENSUS | K | N | T | L | Y | L | Q | M | N | S | L | R | A | E | D | T | A |
| 21-225_12D8_HC | . | T | . | . | . | . | . | . | . | . | . | . | . | . | . | . | . |
| 21-225_30E9_HC | . | . | . | . | . | . | . | . | . | . | . | . | . | . | . | . | . |
| 21-225_31H5_HC | . | . | . | . | . | . | . | . | . | . | . | . | . | . | . | . | . |
| 21-225_31A8_HC | . | . | . | . | . | . | . | . | . | . | . | . | D | . | . | . | . |
| 21-225_30D1_HC | . | . | . | . | . | . | . | . | . | . | . | . | . | . | . | . | . |
| 21-225_31B8_HC | . | . | . | . | . | . | . | L | . | . | . | . | . | D | . | . | . |
| 21-225_30G11_HC | . | T | . | . | . | . | . | . | . | . | . | . | . | . | . | . | . |
| 21-225_30E3_HC | . | . | . | . | . | . | . | . | . | . | . | . | . | . | . | . | . |
| 21-225_31H3_HC | . | . | . | . | . | . | . | . | . | . | . | . | . | . | . | . | . |
| 21-225_32G12_HC | R | . | . | . | . | . | . | . | . | . | . | . | . | . | . | . | . |
| 21-225_34G9_HC | . | . | . | . | . | . | . | . | . | . | . | . | . | . | . | . | . |
| 21-225_36C5_HC | . | . | . | . | . | . | . | . | . | . | . | . | . | . | . | . | . |
| 21-225_33A4_HC | . | . | . | . | . | . | . | . | . | . | . | . | . | . | . | . | . |
| 21-225_33F1_HC | . | . | . | . | . | . | . | . | . | . | . | . | . | . | . | . | . |
| 21-225_33A7_HC | . | . | . | . | . | . | . | . | . | . | . | . | . | . | . | . | . |
| 21-225_34A6_HC | . | . | . | . | . | . | . | . | . | . | . | . | . | . | . | . | . |
| 21-225_35B7_HC | . | . | . | . | . | . | . | . | . | . | . | . | . | . | . | . | . |
| 21-225_34H11_HC | . | . | . | . | F | . | . | . | . | . | . | . | . | . | . | . | . |
| 21-225_33G7_HC | . | . | . | . | . | . | . | . | . | . | . | . | . | . | . | . | . |
| 21-225_35D1_HC | N | . | . | . | . | . | . | . | . | . | . | . | . | . | . | . | . |
| 21-225_35F11_HC | . | . | . | . | . | . | . | . | . | . | . | . | . | . | . | . | . |
| 21-225_34G8_HC | . | . | . | . | . | . | . | I | . | . | . | . | . | . | . | . | . |
| 21-225_36A5_HC | . | . | . | . | . | . | . | . | . | . | . | . | . | . | . | . | . |
| 21-225_4G12_HC | . | . | . | . | . | . | . | . | S | . | . | . | . | . | . | . | . |
| 21-225_17F12_HC | . | . | . | . | . | . | . | . | . | . | . | . | . | . | . | . | . |
| 21-225_21H4_HC | . | . | . | . | . | . | . | . | . | . | . | . | . | . | . | . | . |
| 21-225_24B1_HC | . | . | . | . | . | . | . | . | . | . | . | . | . | . | . | . | . |
| 21-225_32G1_HC | . | . | . | . | . | . | . | . | . | . | . | . | . | . | . | . | . |
| 21-225_33B8_HC | . | . | . | . | . | . | . | . | . | . | . | . | . | . | . | . | . |
| 21-225_35A6_HC | . | . | . | . | . | . | . | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | V | K | G | E | L | L | E | D | Y | - | - | - | - |
| 21-225_12D8_HC | . | . | . | . | A | . | W | G | R | G | Y | S | . | E | . | . | . |
| 21-225_30E9_HC | . | . | . | . | A | . | . | . | . | . | . | . | . | . | . | . | . |
| 21-225_31H5_HC | . | . | . | . | . | . | . | . | . | . | . | . | . | . | . | . | . |
| 21-225_31A8_HC | . | . | . | . | . | . | . | . | . | . | . | . | . | . | . | . | . |
| 21-225_30D1_HC | . | . | . | . | . | . | . | . | . | . | . | . | . | . | . | . | . |
| 21-225_31B8_HC | . | . | . | . | . | . | . | . | . | . | . | . | . | . | . | . | . |
| 21-225_30G11_HC | . | . | . | . | . | . | . | . | . | . | . | . | . | . | . | . | . |
| 21-225_30E3_HC | L | . | . | . | A | . | . | . | . | . | . | . | . | . | . | . | . |
| 21-225_31H3_HC | . | . | . | . | A | . | . | . | . | . | . | . | . | . | . | . | . |
| 21-225_32G12_HC | . | . | . | . | . | . | . | . | . | . | . | . | . | . | . | . | . |
| 21-225_34G9_HC | . | . | . | . | . | . | . | . | . | . | . | . | . | . | . | . | . |
| 21-225_36C5_HC | . | . | . | . | . | . | . | . | . | . | . | . | . | . | . | . | . |
| 21-225_33A4_HC | . | . | F | . | A | . | . | . | . | . | . | . | . | . | . | . | . |
| 21-225_33F1_HC | . | . | . | . | . | . | . | . | . | . | . | . | . | . | . | . | . |
| 21-225_33A7_HC | . | . | . | . | . | . | . | . | . | . | . | . | . | . | . | . | . |
| 21-225_34A6_HC | . | . | . | . | A | . | . | . | . | . | . | . | . | . | . | . | . |
| 21-225_35B7_HC | . | . | . | . | . | . | . | . | . | . | . | . | . | . | . | . | . |
| 21-225_34H11_HC | . | . | . | . | A | . | . | . | . | . | . | . | . | . | . | . | . |
| 21-225_33G7_HC | . | . | . | . | A | . | . | . | . | . | . | . | . | . | . | . | . |
| 21-225_35D1_HC | . | . | . | . | . | . | . | . | . | . | . | . | . | . | . | . | . |
| 21-225_35F11_HC | . | . | . | . | . | . | . | . | . | . | . | . | . | . | . | . | . |
| 21-225_34G8_HC | . | . | . | . | . | . | . | . | . | . | . | . | . | . | . | . | . |
| 21-225_36A5_HC | . | . | . | . | A | . | . | . | . | . | . | . | . | . | . | . | . |
| 21-225_4G12_HC | . | . | . | . | A | . | W | G | R | G | Y | S | . | E | . | . | . |
| 21-225_17F12_HC | . | . | . | . | A | . | W | G | R | G | Y | N | . | E | . | . | . |
| 21-225_21H4_HC | . | . | . | . | A | . | W | G | R | G | Y | S | . | E | . | . | . |
| 21-225_24B1_HC | . | . | . | . | A | . | W | G | R | G | Y | S | . | E | . | . | . |
| 21-225_32G1_HC | . | . | . | . | . | . | . | . | . | . | . | . | . | . | . | . | . |
| 21-225_33B8_HC | . | . | . | . | . | . | . | . | . | . | . | . | . | . | . | . | . |
| 21-225_35A6_HC | . | . | . | . | . | . | . | . | . | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | H_CDR3 | | | | | | | | | | | | | | | | |
| CONSENSUS | - | - | - | - | - | - | - | - | - | - | - | - | Y | F | Y | G | M |
| 21-225_12D8_HC | . | . | . | . | . | . | . | . | . | . | . | . | . | Y | . | . | . |
| 21-225_30E9_HC | . | . | . | . | . | . | . | . | . | . | . | . | . | Y | . | . | . |
| 21-225_31H5_HC | . | . | . | . | . | . | . | . | . | . | . | . | . | . | . | . | . |
| 21-225_31A8_HC | . | . | . | . | . | . | . | . | . | . | . | . | . | . | . | . | . |
| 21-225_30D1_HC | . | . | . | . | . | . | . | . | . | . | . | . | . | . | . | . | L |
| 21-225_31B8_HC | . | . | . | . | . | . | . | . | . | . | . | . | . | . | . | . | . |
| 21-225_30G11_HC | . | . | . | . | . | . | . | . | . | . | . | . | . | . | . | . | . |
| 21-225_30E3_HC | . | . | . | . | . | . | . | . | . | . | . | . | . | Y | . | . | . |
| 21-225_31H3_HC | . | . | . | . | . | . | . | . | . | . | . | . | . | Y | . | . | . |
| 21-225_32G12_HC | . | . | . | . | . | . | . | . | . | . | . | . | . | . | . | . | . |
| 21-225_34G9_HC | . | . | . | . | . | . | . | . | . | . | . | . | . | . | . | . | . |
| 21-225_36C5_HC | . | . | . | . | . | . | . | . | . | . | . | . | . | . | . | . | . |
| 21-225_33A4_HC | . | . | . | . | . | . | . | . | . | . | . | . | S | Y | . | . | I |
| 21-225_33F1_HC | . | . | . | . | . | . | . | . | . | . | . | . | . | . | . | . | . |
| 21-225_33A7_HC | . | . | . | . | . | . | . | . | . | . | . | . | . | . | . | . | . |
| 21-225_34A6_HC | . | . | . | . | . | . | . | . | . | . | . | . | . | Y | F | A | . |
| 21-225_35B7_HC | . | . | . | . | . | . | . | . | . | . | . | . | . | . | . | . | . |
| 21-225_34H11_HC | . | . | . | . | . | . | . | . | . | . | . | . | . | Y | . | . | . |
| 21-225_33G7_HC | . | . | . | . | . | . | . | . | . | . | . | . | S | Y | . | . | . |
| 21-225_35D1_HC | . | . | . | . | . | . | . | . | . | . | . | . | . | . | . | . | . |
| 21-225_35F11_HC | . | . | . | . | . | . | . | . | . | . | . | . | . | . | . | . | . |
| 21-225_34G8_HC | . | . | . | . | . | . | . | . | . | . | . | . | . | . | . | . | . |
| 21-225_36A5_HC | . | . | . | . | . | . | . | . | . | . | . | . | . | Y | . | A | . |
| 21-225_4G12_HC | . | . | . | . | . | . | . | . | . | . | . | . | . | Y | . | . | . |
| 21-225_17F12_HC | . | . | . | . | . | . | . | . | . | . | . | . | . | Y | . | . | . |
| 21-225_21H4_HC | . | . | . | . | . | . | . | . | . | . | . | . | . | Y | . | . | . |
| 21-225_24B1_HC | . | . | . | . | . | . | . | . | . | . | . | . | . | Y | . | . | . |
| 21-225_32G1_HC | . | . | . | . | . | . | . | . | . | . | . | . | . | . | . | . | . |
| 21-225_33B8_HC | . | . | . | . | . | . | . | . | . | . | . | . | . | . | . | . | . |
| 21-225_35A6_HC | . | . | . | . | . | . | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | H_FR4 | | | | | | | | | | |
| CONSENSUS | D | V | W | G | Q | G | T | T | V | T | V | S | S |
| 21-225_12D8_HC | . | . | . | . | . | . | . | . | . | . | . | . | . |
| 21-225_30E9_HC | . | . | . | . | . | . | . | . | . | . | . | . | . |
| 21-225_31H5_HC | . | . | . | . | . | . | . | . | . | . | . | . | . |
| 21-225_31A8_HC | . | . | . | . | . | . | . | . | . | . | . | . | . |
| 21-225_30D1_HC | . | . | . | . | . | . | . | . | . | . | . | . | . |
| 21-225_31B8_HC | . | . | . | . | . | . | . | . | . | . | . | . | . |
| 21-225_30G11_HC | . | . | . | . | . | . | . | . | . | . | . | . | . |
| 21-225_30E3_HC | . | . | . | . | . | . | . | . | . | . | . | . | . |
| 21-225_31H3_HC | . | . | . | . | . | . | . | . | . | . | . | . | . |
| 21-225_32G12_HC | . | . | . | . | . | . | . | . | . | . | . | . | . |
| 21-225_34G9_HC | . | . | . | . | . | . | . | . | . | . | . | . | . |
| 21-225_36C5_HC | . | . | . | . | . | . | . | . | . | . | . | . | . |
| 21-225_33A4_HC | . | . | . | . | . | . | . | . | . | . | . | . | . |
| 21-225_33F1_HC | . | . | . | . | . | . | . | . | . | . | . | . | . |
| 21-225_33A7_HC | . | . | . | . | . | . | . | . | . | . | . | . | . |
| 21-225_34A6_HC | . | . | . | . | . | . | . | . | . | . | . | . | . |
| 21-225_35B7_HC | . | . | . | . | . | . | A | . | . | . | . | . | . |
| 21-225_34H11_HC | . | . | . | . | . | . | . | . | . | . | . | . | . |
| 21-225_33G7_HC | . | . | . | . | . | . | . | . | . | . | . | . | . |
| 21-225_35D1_HC | . | . | . | . | . | . | A | . | . | . | . | . | . |
| 21-225_35F11_HC | . | . | . | . | . | . | . | . | . | . | . | . | . |
| 21-225_34G8_HC | . | . | . | . | . | . | . | . | . | . | . | . | . |
| 21-225_36A5_HC | . | . | . | . | . | . | . | . | . | . | . | . | . |
| 21-225_4G12_HC | . | . | . | . | . | . | . | . | . | . | . | . | . |
| 21-225_17F12_HC | . | . | . | . | . | . | . | . | . | . | . | . | . |
| 21-225_21H4_HC | . | . | . | . | . | . | . | . | . | . | . | . | . |
| 21-225_24B1_HC | . | . | . | . | . | . | . | . | . | . | . | . | . |
| 21-225_32G1_HC | . | . | . | . | . | . | . | . | . | . | . | . | . |
| 21-225_33B8_HC | . | . | . | . | . | . | . | . | . | . | . | . | . |
| 21-225_35A6_HC | . | . | . | . | . | . | . | . | . | . | . | . | . |

FIGURE 56

Table 28

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | H_FR1 | | | | | | | | | | | | | | | | |
| CONSENSUS | E | V | Q | L | L | E | S | - | G | G | G | L | V | Q | P | G | G |
| 21-225_17C6_HC | . | . | . | . | . | . | . | . | . | . | . | . | . | . | . | . | . |
| 21-225_17B4_HC | . | . | . | . | . | . | . | . | . | . | . | . | . | . | . | . | . |
| 21-225_20A4_HC | . | . | . | . | . | . | . | . | . | . | . | . | . | . | . | . | . |
| 21-225_17F7_HC | . | . | . | . | . | . | . | . | . | . | . | . | . | . | . | . | . |
| 21-225_17F11_HC | . | A | . | . | . | . | . | . | . | . | . | . | E | . | . | . | . |
| 21-225_17A12_HC | . | . | . | . | . | . | . | . | . | . | . | . | I | . | . | . | . |
| 21-225_18E12_HC | . | . | . | . | . | . | . | . | . | . | . | . | . | . | . | . | . |
| 21-225_22G3_HC | . | . | . | . | . | . | . | . | . | . | . | . | . | . | . | . | . |
| 21-225_22G10_HC | . | . | . | . | . | . | . | . | . | . | D | . | . | . | . | . | . |
| 21-225_21F4_HC | . | . | . | . | . | . | . | . | . | . | . | . | . | . | . | . | . |
| 21-225_22E4_HC | . | . | . | . | . | . | . | . | . | . | . | . | . | . | . | . | . |
| 21-225_21A2_HC | . | . | . | . | . | . | . | . | . | . | . | . | . | . | . | . | . |
| 21-225_22C5_HC | . | . | . | . | . | . | . | . | . | . | . | . | . | . | V | . | . |
| 21-225_22D5_HC | . | . | . | . | . | . | . | . | . | . | . | . | . | . | . | . | . |
| 21-225_10F12_HC | . | . | . | . | . | . | . | . | . | . | . | . | . | . | . | . | . |
| 21-225_4H2_HC | . | . | . | . | . | . | . | . | . | . | . | . | . | . | . | . | . |
| 21-225_5G2_HC | . | . | . | . | . | . | . | . | . | . | . | . | . | . | . | . | . |
| 21-225_3E3_HC | . | . | . | . | . | . | . | . | . | . | T | . | . | . | . | . | . |
| 21-225_5F7_HC | . | . | . | . | . | . | . | . | . | . | . | . | . | . | . | . | . |
| 21-225_6D3_HC | . | . | . | . | . | . | . | . | . | . | . | . | . | . | . | . | . |
| 21-225_8G6_HC | . | . | . | . | . | . | . | . | . | . | . | . | . | . | . | . | . |
| 21-225_16H6_HC | . | . | . | . | . | . | . | . | . | . | . | . | . | . | . | . | . |
| 21-225_3B1_HC | . | . | . | . | . | . | . | . | . | . | . | . | . | . | . | . | . |
| 21-225_8E12_HC | . | . | . | . | . | . | . | . | . | . | . | . | . | . | . | . | . |
| 21-225_11A5_HC | . | . | . | . | . | . | . | . | . | . | D | . | . | . | . | . | . |

**FIGURE 56**

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | S | L | R | L | S | C | A | A | S | E | - | F | T | F | S | S | - |
| 21-225_17C8_HC | . | . | . | . | . | . | . | . | . | . | . | . | . | . | . | . | . |
| 21-225_17B4_HC | . | . | . | . | . | . | . | . | . | . | . | . | . | . | . | . | . |
| 21-225_20A4_HC | . | . | . | . | . | . | . | . | . | G | . | . | . | . | . | . | . |
| 21-225_17F7_HC | . | . | . | . | . | . | . | . | . | . | . | . | . | . | . | . | . |
| 21-225_17F11_HC | . | . | . | . | . | . | . | . | . | G | . | . | . | . | . | . | . |
| 21-225_17A12_HC | . | . | . | . | . | . | . | . | . | . | . | . | . | . | . | . | . |
| 21-225_18E12_HC | . | . | . | . | . | . | . | . | . | . | . | . | . | . | . | . | . |
| 21-225_22G3_HC | . | . | . | . | . | . | . | . | . | . | . | . | . | . | . | . | . |
| 21-225_22G10_HC | . | . | . | . | . | . | T | . | . | G | . | . | . | . | . | . | . |
| 21-225_21F4_HC | . | . | . | . | . | . | . | . | . | . | . | . | . | . | . | . | . |
| 21-225_22E4_HC | . | . | . | . | . | . | . | . | . | . | . | . | . | . | . | . | . |
| 21-225_21A2_HC | . | . | . | . | . | . | . | . | . | K | . | . | . | . | . | . | . |
| 21-225_22C5_HC | . | . | . | . | . | . | . | . | . | G | . | . | . | . | . | . | . |
| 21-225_22D5_HC | . | . | . | . | . | . | . | . | . | . | . | . | . | . | . | . | . |
| 21-225_10F12_HC | . | . | . | . | . | . | . | . | . | . | . | . | . | . | . | . | . |
| 21-225_4H2_HC | . | . | . | . | . | . | . | . | . | G | . | . | . | . | . | G | . |
| 21-225_5G2_HC | . | . | . | . | . | . | . | . | . | . | . | . | . | . | . | . | . |
| 21-225_3E3_HC | . | . | . | . | . | . | . | . | . | . | . | . | . | . | . | . | . |
| 21-225_5F7_HC | . | . | . | . | . | . | . | . | . | . | . | . | . | . | . | . | . |
| 21-225_6D3_HC | . | . | . | . | . | . | . | . | . | . | . | . | . | . | . | . | . |
| 21-225_8G6_HC | . | . | . | . | . | . | V | . | . | . | . | . | . | . | . | . | . |
| 21-225_16H6_HC | . | . | . | . | . | . | . | . | . | G | . | . | . | . | . | . | . |
| 21-225_3B1_HC | . | . | . | . | . | . | . | . | . | . | . | . | . | . | . | . | . |
| 21-225_8E12_HC | . | . | . | . | . | . | . | . | . | . | . | . | . | . | . | . | . |
| 21-225_11A5_HC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | H_CDR1 | | | | | | | | H_FR2 | | | | | | | | |
| CONSENSUS | - | - | - | - | Y | A | M | | W | V | R | Q | A | P | G | K | G |
| 21-225_17C6_HC | . | . | . | . | . | . | . | N | . | . | . | . | . | . | . | . | . |
| 21-225_17B4_HC | . | . | . | . | . | . | . | N | . | . | . | . | . | . | . | . | . |
| 21-225_20A4_HC | . | . | . | . | . | . | . | T | . | . | . | . | G | . | . | M | . |
| 21-225_17F7_HC | . | . | . | . | . | . | . | N | . | . | . | . | . | . | . | . | . |
| 21-225_17F11_HC | . | . | . | . | . | . | . | S | . | . | . | . | . | . | . | . | . |
| 21-225_17A12_HC | . | . | . | . | . | . | . | S | . | . | . | . | D | . | . | . | . |
| 21-225_18E12_HC | . | . | . | . | . | . | . | S | . | . | . | . | . | . | . | T | . |
| 21-225_22G3_HC | . | . | . | . | . | . | . | N | . | . | . | . | . | . | . | . | . |
| 21-225_22G10_HC | . | . | . | . | . | . | . | N | . | . | . | . | . | . | . | . | . |
| 21-225_21F4_HC | . | . | . | . | . | . | . | N | . | . | . | . | . | . | . | . | . |
| 21-225_22E4_HC | . | . | . | . | . | . | . | S | . | . | . | . | . | . | . | . | . |
| 21-225_21A2_HC | . | . | . | . | . | . | . | N | . | . | . | . | . | . | . | . | . |
| 21-225_22C5_HC | . | . | . | . | . | . | . | S | . | . | . | . | . | . | . | M | . |
| 21-225_22D5_HC | . | . | . | . | . | . | . | S | . | . | . | . | . | . | . | . | . |
| 21-225_10F12_HC | . | . | . | . | . | . | . | S | . | . | . | . | . | . | . | . | . |
| 21-225_4H2_HC | . | . | . | . | . | . | . | N | . | . | . | . | . | . | . | M | . |
| 21-225_5G2_HC | . | . | . | . | . | . | . | S | . | . | . | . | . | . | . | . | . |
| 21-225_3E3_HC | . | . | . | . | . | V | . | S | . | . | . | . | . | . | . | . | . |
| 21-225_5F7_HC | . | . | . | . | . | . | . | S | . | . | . | . | . | . | . | . | . |
| 21-225_6D3_HC | . | . | . | . | . | . | . | N | . | . | . | . | . | . | . | . | . |
| 21-225_8G6_HC | . | . | . | . | . | V | . | N | . | . | . | . | . | . | . | . | . |
| 21-225_16H6_HC | . | . | . | . | . | . | . | N | . | . | . | . | . | . | . | M | . |
| 21-225_3B1_HC | . | . | . | . | . | . | . | N | . | . | . | . | . | . | . | . | . |
| 21-225_8E12_HC | . | . | . | . | . | . | . | S | . | . | . | . | . | . | . | . | . |
| 21-225_11A5_HC | . | . | . | . | . | . | . | S | . | . | . | . | . | . | . | . | . |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | H_CDR2 | | |
| CONSENSUS | L | E | W | V | S | V | I | S | G | R | - | - | - | G | G | N | T |
| 21-225_17C6_HC | . | . | . | . | . | . | . | . | . | . | . | . | . | . | . | . | . |
| 21-225_17B4_HC | . | . | . | . | . | . | . | . | . | . | . | . | . | . | . | . | . |
| 21-225_20A4_HC | . | . | . | . | . | . | . | . | . | S | . | . | . | . | . | Y | . |
| 21-225_17F7_HC | . | . | . | . | . | I | . | . | . | . | . | . | . | . | . | . | . |
| 21-225_17F11_HC | . | . | . | . | . | . | . | . | . | . | . | . | . | . | . | . | . |
| 21-225_17A12_HC | . | . | . | . | . | I | . | . | . | . | . | . | . | . | . | . | A |
| 21-225_18E12_HC | . | . | . | . | . | I | . | . | . | . | . | . | . | . | . | . | . |
| 21-225_22G3_HC | . | . | . | . | . | I | . | . | . | . | . | . | . | . | V | . | . |
| 21-225_22G10_HC | . | . | . | . | . | . | . | . | . | S | . | . | . | . | . | . | . |
| 21-225_21F4_HC | . | . | . | . | . | . | . | . | . | . | . | . | . | . | . | . | . |
| 21-225_22E4_HC | . | . | . | . | . | I | . | . | . | . | . | . | . | . | T | . | . |
| 21-225_21A2_HC | . | . | . | I | . | I | . | . | . | . | . | . | . | . | . | . | . |
| 21-225_22C5_HC | . | . | . | . | . | I | . | . | . | S | . | . | . | . | . | Y | . |
| 21-225_22D5_HC | . | . | . | . | . | . | . | . | . | . | . | . | . | . | . | . | . |
| 21-225_10F12_HC | . | . | . | . | . | I | . | . | . | . | . | . | . | . | . | . | . |
| 21-225_4H2_HC | . | . | . | . | . | . | . | . | . | S | . | . | . | . | . | Y | . |
| 21-225_5G2_HC | . | . | . | . | . | . | . | . | . | . | . | . | . | . | V | . | . |
| 21-225_3E3_HC | . | . | . | . | . | I | . | . | . | . | . | . | . | . | . | . | . |
| 21-225_5F7_HC | . | . | . | . | . | I | . | . | . | . | . | . | . | . | . | . | . |
| 21-225_6D3_HC | . | . | . | . | . | . | . | . | . | . | . | . | . | . | . | . | . |
| 21-225_8G6_HC | . | . | . | . | . | . | . | . | . | . | . | . | . | . | . | . | . |
| 21-225_16H6_HC | . | . | . | . | . | . | . | . | . | S | . | . | . | . | . | Y | . |
| 21-225_3B1_HC | . | . | . | . | . | . | . | . | . | . | . | . | . | . | I | . | . |
| 21-225_8E12_HC | . | . | . | . | . | I | . | . | . | . | . | . | . | . | . | . | . |
| 21-225_11A5_HC | . | . | . | I | . | . | . | . | . | . | . | . | . | . | V | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | F | Y | A | D | S | V | K | G | R | F | T | I | S | R | D | N | S |
| 21-225_17C6_HC | . | . | . | . | . | . | . | . | . | . | . | . | . | . | . | . | . |
| 21-225_17B4_HC | . | . | . | . | . | . | . | . | . | . | . | . | . | . | . | . | . |
| 21-225_20A4_HC | Y | N | . | . | . | . | . | . | . | . | . | . | . | . | . | . | . |
| 21-225_17F7_HC | . | . | . | . | . | . | . | . | . | . | . | . | . | . | . | . | . |
| 21-225_17F11_HC | Y | S | . | . | . | . | . | . | . | . | . | . | . | . | . | . | . |
| 21-225_17A12_HC | . | . | . | . | . | . | . | . | . | . | . | . | . | . | . | . | . |
| 21-225_18E12_HC | . | . | . | . | . | . | . | . | . | . | . | . | . | . | . | . | . |
| 21-225_22G3_HC | . | . | . | . | . | . | . | . | . | . | . | . | . | . | . | . | . |
| 21-225_22G10_HC | . | . | . | . | . | . | . | . | . | . | . | . | . | . | . | . | . |
| 21-225_21F4_HC | . | . | . | . | . | . | . | . | . | . | . | . | . | . | . | . | . |
| 21-225_22E4_HC | . | . | . | . | . | . | . | . | . | . | . | . | . | . | V | . | . |
| 21-225_21A2_HC | . | . | . | . | . | . | . | . | . | . | . | . | . | . | . | . | . |
| 21-225_22C5_HC | Y | . | . | . | . | . | . | . | . | . | . | . | . | . | . | . | . |
| 21-225_22D5_HC | . | . | . | . | . | . | . | . | . | . | . | . | . | . | . | . | . |
| 21-225_10F12_HC | . | . | . | . | . | . | . | . | . | . | . | . | . | . | . | . | . |
| 21-225_4H2_HC | Y | . | . | . | . | . | . | . | . | . | . | . | . | . | . | . | . |
| 21-225_5G2_HC | . | . | . | . | . | . | . | . | . | . | . | . | . | . | . | . | . |
| 21-225_3E3_HC | . | . | . | . | . | . | . | . | . | . | . | . | . | . | . | . | . |
| 21-225_5F7_HC | . | . | . | . | . | . | . | . | . | . | . | . | . | . | . | . | . |
| 21-225_6D3_HC | . | . | . | . | . | . | . | . | . | . | . | . | . | . | . | . | . |
| 21-225_8G6_HC | . | . | . | . | . | . | . | . | . | . | . | . | . | . | . | . | . |
| 21-225_16H6_HC | Y | . | . | . | . | . | . | . | . | . | . | . | . | . | . | . | . |
| 21-225_3B1_HC | . | . | . | . | . | . | . | . | . | . | . | . | . | . | . | . | . |
| 21-225_8E12_HC | . | . | . | . | . | . | . | . | . | . | . | . | . | . | . | . | . |
| 21-225_11A5_HC | . | . | . | . | . | . | . | . | . | . | . | . | . | . | . | . | . |

FIGURE 56

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | H_FR3 | | | | | | | | | | | | | | | | |
| CONSENSUS | K | N | T | L | Y | L | Q | M | N | S | L | R | A | E | D | T | A |
| 21-225_17C6_HC | . | . | . | . | F | . | . | . | . | . | . | . | . | . | . | . | . |
| 21-225_17B4_HC | . | . | . | . | . | . | . | . | . | . | . | . | . | . | . | . | . |
| 21-225_20A4_HC | . | . | . | . | . | . | . | . | . | . | . | . | V | . | . | . | . |
| 21-225_17F7_HC | . | . | . | . | . | . | . | . | . | . | . | . | . | . | . | . | . |
| 21-225_17F11_HC | . | . | . | . | . | . | . | . | . | . | . | . | . | . | . | . | . |
| 21-225_17A12_HC | . | . | . | . | . | . | . | . | . | . | . | . | . | . | . | . | . |
| 21-225_18E12_HC | . | . | . | . | . | . | . | . | . | . | . | . | . | . | . | . | . |
| 21-225_22G3_HC | . | . | . | . | . | . | . | . | . | . | . | . | . | . | . | . | . |
| 21-225_22G10_HC | . | . | . | . | F | . | H | . | . | . | . | . | . | . | . | . | . |
| 21-225_21F4_HC | . | . | . | . | . | . | . | . | . | . | . | . | . | . | . | . | . |
| 21-225_22E4_HC | . | . | . | . | . | . | . | . | . | . | . | . | . | . | . | . | . |
| 21-225_21A2_HC | . | . | . | . | . | . | . | . | . | . | . | . | . | . | . | . | . |
| 21-225_22C5_HC | . | . | . | . | . | . | . | . | . | . | . | . | . | . | . | . | . |
| 21-225_22D5_HC | . | . | . | . | . | . | . | . | . | . | . | . | . | . | . | . | . |
| 21-225_10F12_HC | . | . | . | . | . | . | . | . | . | . | . | . | . | . | . | . | . |
| 21-225_4H2_HC | . | . | . | . | . | . | . | . | . | . | . | . | . | . | . | . | . |
| 21-225_5G2_HC | . | . | . | . | . | . | . | . | . | . | . | . | . | . | . | . | . |
| 21-225_3E3_HC | . | . | . | . | . | . | . | . | . | . | . | . | . | . | . | . | . |
| 21-225_5F7_HC | . | . | . | . | . | . | . | . | . | . | . | . | . | . | . | . | . |
| 21-225_6D3_HC | . | . | . | . | . | . | . | . | . | . | . | . | . | . | . | . | . |
| 21-225_8G6_HC | . | . | . | . | . | . | . | . | . | . | . | . | . | . | . | . | . |
| 21-225_16H6_HC | . | . | . | . | . | . | . | . | . | . | . | . | . | . | . | . | . |
| 21-225_3B1_HC | . | . | . | . | . | . | . | . | . | . | . | . | . | . | . | . | . |
| 21-225_8E12_HC | . | . | . | . | . | . | . | . | . | . | . | . | . | . | . | . | . |
| 21-225_11A5_HC | . | . | . | . | . | . | . | . | . | . | . | . | . | . | . | . | . |

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | A | S | R | L | A | V | A | G | - | - | - | - | - |
| 21-225_17C6_HC | . | . | . | . | . | . | . | . | . | . | . | . | . | . | . | . | . |
| 21-225_17B4_HC | . | . | . | . | . | . | . | . | . | . | . | . | . | . | . | . | . |
| 21-225_20A4_HC | . | . | . | . | . | . | . | I | . | . | . | . | . | . | . | . | . |
| 21-225_17F7_HC | . | . | . | . | . | . | . | . | . | . | . | . | . | . | . | . | . |
| 21-225_17F11_HC | . | . | . | . | . | . | . | . | . | . | . | . | . | . | . | . | . |
| 21-225_17A12_HC | . | . | . | . | . | . | . | I | . | . | . | . | . | . | . | . | . |
| 21-225_18E12_HC | . | . | . | . | . | . | . | . | . | . | . | . | . | . | . | . | . |
| 21-225_22G3_HC | . | . | . | . | . | . | . | . | . | . | . | . | . | . | . | . | . |
| 21-225_22G10_HC | . | . | . | . | . | . | . | M | . | . | . | . | . | . | . | . | . |
| 21-225_21F4_HC | . | . | . | . | . | . | . | . | . | . | . | . | . | . | . | . | . |
| 21-225_22E4_HC | . | . | . | . | . | . | . | I | . | . | . | . | . | . | . | . | . |
| 21-225_21A2_HC | . | . | . | . | . | . | . | . | . | . | . | . | . | . | . | . | . |
| 21-225_22C5_HC | . | . | . | . | . | . | . | I | . | . | . | . | . | . | . | . | . |
| 21-225_22D5_HC | . | . | . | . | . | . | . | I | . | . | . | . | . | . | . | . | . |
| 21-225_10F12_HC | . | . | . | . | . | . | . | V | . | . | . | . | . | . | . | . | . |
| 21-225_4H2_HC | . | . | . | . | . | . | . | . | . | . | . | . | . | . | . | . | . |
| 21-225_5G2_HC | . | . | . | . | . | . | . | I | . | . | . | . | . | . | . | . | . |
| 21-225_3E3_HC | L | . | . | . | . | . | . | . | . | . | . | . | . | . | . | . | . |
| 21-225_5F7_HC | . | . | . | . | . | . | . | I | . | . | . | . | . | . | . | . | . |
| 21-225_6D3_HC | . | . | F | . | . | . | . | . | . | . | . | . | . | . | . | . | . |
| 21-225_8G6_HC | . | . | . | . | . | . | . | . | . | . | . | . | . | . | . | . | . |
| 21-225_16H6_HC | . | . | . | . | . | . | . | . | . | . | . | . | . | . | . | . | . |
| 21-225_3B1_HC | . | . | . | . | . | . | . | . | . | . | . | . | . | . | . | . | . |
| 21-225_8E12_HC | . | . | . | . | . | . | . | M | . | . | . | . | . | . | . | . | . |
| 21-225_11A5_HC | . | . | . | . | . | . | . | I | . | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | H_CDR3 | | | | | | | | | | | | | | | | |
| CONSENSUS | - | - | - | - | - | - | - | - | - | - | - | - | - | S | E | A | F |
| 21-225_17C6_HC | . | . | . | . | . | . | . | . | . | . | . | . | . | . | . | . | . |
| 21-225_17B4_HC | . | . | . | . | . | . | . | . | . | . | . | . | . | . | . | . | . |
| 21-225_20A4_HC | . | . | . | . | . | . | . | . | . | . | . | . | . | . | . | . | . |
| 21-225_17F7_HC | . | . | . | . | . | . | . | . | . | . | . | . | . | . | . | . | . |
| 21-225_17F11_HC | . | . | . | . | . | . | . | . | . | . | . | . | . | . | . | . | . |
| 21-225_17A12_HC | . | . | . | . | . | . | . | . | . | . | . | . | . | . | . | . | . |
| 21-225_18E12_HC | . | . | . | . | . | . | . | . | . | . | . | . | . | . | . | . | . |
| 21-225_22G3_HC | . | . | . | . | . | . | . | . | . | . | . | . | . | . | . | . | . |
| 21-225_22G10_HC | . | . | . | . | . | . | . | . | . | . | . | . | . | . | . | . | . |
| 21-225_21F4_HC | . | . | . | . | . | . | . | . | . | . | . | . | . | . | . | . | C |
| 21-225_22E4_HC | . | . | . | . | . | . | . | . | . | . | . | . | . | . | . | . | . |
| 21-225_21A2_HC | . | . | . | . | . | . | . | . | . | . | . | . | . | . | . | . | . |
| 21-225_22C5_HC | . | . | . | . | . | . | . | . | . | . | . | . | . | . | . | . | . |
| 21-225_22D5_HC | . | . | . | . | . | . | . | . | . | . | . | . | . | . | . | . | . |
| 21-225_10F12_HC | . | . | . | . | . | . | . | . | . | . | . | . | . | . | . | . | . |
| 21-225_4H2_HC | . | . | . | . | . | . | . | . | . | . | . | . | . | . | . | . | . |
| 21-225_5G2_HC | . | . | . | . | . | . | . | . | . | . | . | . | . | . | . | . | . |
| 21-225_3E3_HC | . | . | . | . | . | . | . | . | . | . | . | . | . | . | . | . | . |
| 21-225_5F7_HC | . | . | . | . | . | . | . | . | . | . | . | . | . | . | . | . | . |
| 21-225_6D3_HC | . | . | . | . | . | . | . | . | . | . | . | . | . | . | . | . | . |
| 21-225_8G6_HC | . | . | . | . | . | . | . | . | . | . | . | . | . | . | . | . | . |
| 21-225_16H6_HC | . | . | . | . | . | . | . | . | . | . | . | . | . | . | . | . | . |
| 21-225_3B1_HC | . | . | . | . | . | . | . | . | . | . | . | . | . | . | . | . | . |
| 21-225_8E12_HC | . | . | . | . | . | . | . | . | . | . | . | . | . | . | . | . | . |
| 21-225_11A5_HC | . | . | . | . | . | . | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | H_FR4 | | | | | | | | | | |
| CONSENSUS | D | I | W | G | Q | G | T | M | V | T | V | S | S |
| 21-225_17C6_HC | . | . | . | . | . | . | . | . | . | . | . | . | . |
| 21-225_17B4_HC | . | . | . | . | . | . | . | . | . | . | . | . | . |
| 21-225_20A4_HC | . | . | . | . | . | . | . | . | . | . | . | . | . |
| 21-225_17F7_HC | . | . | . | . | . | . | . | . | . | . | . | . | . |
| 21-225_17F11_HC | H | . | . | . | . | . | . | . | . | . | . | . | . |
| 21-225_17A12_HC | . | . | . | . | . | . | . | . | . | . | . | . | . |
| 21-225_18E12_HC | . | . | . | . | . | . | . | . | . | . | . | . | . |
| 21-225_22G3_HC | . | . | . | . | . | . | . | . | . | . | . | . | . |
| 21-225_22G10_HC | . | . | . | . | . | . | . | . | . | . | . | . | . |
| 21-225_21F4_HC | . | . | . | . | . | . | . | . | . | . | . | . | . |
| 21-225_22E4_HC | . | . | . | . | . | . | . | . | . | . | . | . | . |
| 21-225_21A2_HC | . | . | . | . | . | . | . | . | . | . | . | . | . |
| 21-225_22C5_HC | . | . | . | . | . | . | . | . | . | . | . | . | . |
| 21-225_22D5_HC | . | . | . | . | . | . | . | . | . | . | . | . | . |
| 21-225_10F12_HC | A | . | . | . | . | . | . | . | . | . | . | . | . |
| 21-225_4H2_HC | . | . | . | . | . | . | . | . | . | . | . | . | . |
| 21-225_5G2_HC | . | . | . | . | . | . | . | . | . | . | . | . | . |
| 21-225_3E3_HC | . | . | . | . | . | . | . | . | . | . | . | . | . |
| 21-225_5F7_HC | A | . | . | . | . | . | . | . | . | . | . | . | . |
| 21-225_6D3_HC | . | . | . | . | . | . | . | . | . | . | . | . | . |
| 21-225_8G6_HC | . | . | . | . | . | . | . | . | . | S | . | . | . |
| 21-225_16H6_HC | . | . | . | . | . | . | . | . | . | . | . | . | . |
| 21-225_3B1_HC | . | . | . | . | . | . | . | . | . | . | . | . | . |
| 21-225_8E12_HC | . | . | . | . | . | . | . | . | . | . | . | . | . |
| 21-225_11A5_HC | . | . | . | . | . | . | . | . | . | . | . | . | . |

FIGURE 56

Table 29

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | H_FR1 | | | | | | | | | | | | | | | | |
| CONSENSUS | Q | V | Q | L | V | E | S | - | G | G | G | V | V | Q | P | G | R |
| 21-225_20F6_HC | . | . | . | . | . | . | . | . | . | . | . | . | . | . | . | . | . |
| 21-225_18G4_HC | . | . | . | . | . | . | . | . | . | . | . | . | . | . | . | . | . |
| 21-225_22C7_HC | . | . | . | . | . | . | . | . | . | . | . | . | . | . | . | . | . |
| 21-225_27A11_HC | . | . | . | . | . | . | . | . | . | . | . | . | . | . | . | . | . |
| 21-225_26G11_HC | . | . | . | . | . | . | . | . | . | . | . | . | . | . | . | . | . |
| 21-225_26D12_HC | . | . | . | . | . | . | . | . | . | . | . | . | . | . | . | . | . |
| 21-225_25B3_HC | . | . | . | . | . | . | . | . | . | . | . | . | . | . | . | . | . |
| 21-225_25F3_HC | . | . | . | . | . | . | . | . | . | . | . | . | . | . | . | . | . |
| 21-225_3F8_HC | . | . | . | . | . | . | . | . | . | . | . | . | . | . | . | . | . |
| 21-225_7E11_HC | . | . | . | . | . | . | . | . | . | . | . | . | . | . | . | . | . |
| 21-225_6G1_HC | . | . | . | . | . | . | . | . | . | . | . | . | . | . | . | . | . |
| 21-225_15A3_HC | . | . | . | . | . | . | . | . | . | . | . | . | . | . | . | . | . |
| 21-225_13D4_HC | . | . | . | . | . | . | . | . | . | . | . | . | . | . | . | . | . |
| 21-225_16E6_HC | . | . | . | . | . | . | . | . | . | . | . | . | . | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | S | L | R | L | S | C | A | A | S | G | - | F | T | F | S | S | - |
| 21-225_20F6_HC | . | . | . | . | . | . | V | . | . | . | . | . | . | . | . | . | . |
| 21-225_18G4_HC | . | . | . | . | . | . | . | . | . | . | . | . | . | . | . | . | . |
| 21-225_22C7_HC | . | . | . | . | . | . | . | . | . | . | . | . | . | . | . | . | . |
| 21-225_27A11_HC | . | . | . | . | . | . | . | . | . | . | . | . | . | . | . | . | . |
| 21-225_26G11_HC | . | . | . | . | . | . | . | . | . | . | . | . | . | . | . | . | . |
| 21-225_26D12_HC | . | . | . | . | . | . | . | . | . | . | . | . | . | . | . | . | . |
| 21-225_25B3_HC | . | . | . | . | . | . | . | . | . | . | . | . | . | . | . | . | . |
| 21-225_25F3_HC | . | . | . | . | . | . | . | . | . | . | . | . | . | . | . | . | . |
| 21-225_3F8_HC | . | . | . | . | . | . | . | . | . | . | . | . | . | . | . | . | . |
| 21-225_7E11_HC | . | . | . | . | . | . | . | . | . | . | . | . | . | . | . | . | . |
| 21-225_6G1_HC | . | . | . | . | . | . | . | . | . | . | . | . | I | . | . | . | . |
| 21-225_15A3_HC | . | . | . | . | . | . | . | . | . | . | . | . | . | . | . | . | . |
| 21-225_13D4_HC | . | . | . | . | . | . | . | . | . | . | . | . | . | . | . | . | . |
| 21-225_16E6_HC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | H_CDR1 | | | | | | | | | | | | | | H_FR2 | | |
| CONSENSUS | - | - | - | - | Y | G | M | H | W | V | R | Q | A | P | G | K | G |
| 21-225_20F6_HC | . | . | . | . | . | . | . | . | . | . | . | . | . | . | . | . | . |
| 21-225_18G4_HC | . | . | . | . | . | . | . | . | . | . | . | . | . | . | . | . | . |
| 21-225_22C7_HC | . | . | . | . | . | . | . | . | . | . | . | . | . | . | . | . | . |
| 21-225_27A11_HC | . | . | . | . | . | . | . | . | . | . | . | . | . | . | . | . | . |
| 21-225_26G11_HC | . | . | . | . | . | . | . | . | . | . | . | . | . | . | . | . | . |
| 21-225_26D12_HC | . | . | . | . | . | . | . | . | . | . | . | . | . | . | . | . | . |
| 21-225_25B3_HC | . | . | . | . | . | . | . | . | . | . | . | . | . | . | . | . | . |
| 21-225_25F3_HC | . | . | . | . | . | . | . | . | . | . | . | . | . | . | . | . | . |
| 21-225_3F8_HC | . | . | . | . | . | . | . | . | . | . | . | . | . | . | . | . | . |
| 21-225_7E11_HC | . | . | . | . | [illegible] | . | . | . | . | . | . | . | . | . | . | . | . |
| 21-225_6G1_HC | . | . | . | . | . | . | . | . | . | . | . | . | . | . | . | . | . |
| 21-225_15A3_HC | . | . | . | . | . | . | [illegible] | . | . | . | . | . | . | . | . | . | . |
| 21-225_13D4_HC | . | . | . | . | . | . | . | . | . | . | . | . | . | . | . | . | . |
| 21-225_16E6_HC | . | . | . | . | . | . | . | . | . | . | . | . | . | . | . | . | . |

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | H_CDR2 | | |
| CONSENSUS | L | E | W | V | A | V | I | W | | D | - | - | - | G | S | N | K |
| 21-225_20F6_HC | . | . | . | G | . | . | . | . | [illegible] | . | . | . | . | . | . | . | . |
| 21-225_18G4_HC | . | . | . | . | . | [illegible] | . | . | Y | . | . | . | . | . | . | . | . |
| 21-225_22C7_HC | . | . | . | G | . | . | . | . | [illegible] | . | . | . | . | . | . | . | . |
| 21-225_27A11_HC | . | . | . | . | . | . | . | . | Y | . | . | . | . | . | . | . | . |
| 21-225_26G11_HC | . | . | . | . | . | . | . | . | Y | . | . | . | . | . | . | . | . |
| 21-225_26D12_HC | . | . | . | . | . | . | . | . | Y | . | . | . | . | . | . | . | E |
| 21-225_25B3_HC | . | . | . | . | . | . | . | . | Y | . | . | . | . | . | . | . | . |
| 21-225_25F3_HC | . | . | . | . | . | . | . | . | Y | . | . | . | . | . | . | . | . |
| 21-225_3F8_HC | . | . | . | G | . | . | . | . | [illegible] | . | . | . | . | . | . | . | . |
| 21-225_7E11_HC | . | . | . | . | . | [illegible] | . | . | [illegible] | . | . | . | . | . | . | . | . |
| 21-225_6G1_HC | . | . | . | . | . | [illegible] | . | . | Y | . | . | . | . | . | . | . | . |
| 21-225_15A3_HC | . | . | . | . | . | . | . | . | [illegible] | . | . | . | . | . | . | . | . |
| 21-225_13D4_HC | . | . | . | . | . | . | . | . | [illegible] | . | . | . | . | . | . | . | . |
| 21-225_16E6_HC | . | . | . | . | . | . | . | . | [illegible] | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | A | D | S | V | K | G | R | F | T | I | S | R | D | N | S |
| 21-225_20F6_HC | . | . | . | . | . | . | . | . | . | . | . | . | . | . | . | . | A |
| 21-225_18G4_HC | . | . | . | . | . | . | . | . | . | . | . | . | . | . | . | . | . |
| 21-225_22C7_HC | . | . | . | . | . | . | . | . | . | . | . | . | . | . | . | . | A |
| 21-225_27A11_HC | . | . | . | . | . | . | . | . | . | . | . | . | . | . | . | . | . |
| 21-225_26G11_HC | . | . | . | . | . | . | . | . | . | . | . | . | . | . | . | . | . |
| 21-225_26D12_HC | . | . | . | . | . | . | . | . | . | . | . | . | . | . | . | . | . |
| 21-225_25B3_HC | . | . | . | . | . | . | . | . | . | . | . | . | . | . | . | . | . |
| 21-225_25F3_HC | . | . | . | . | . | . | . | . | . | . | . | . | . | . | . | . | . |
| 21-225_3F8_HC | . | . | . | . | . | . | . | . | . | . | . | . | . | . | . | . | A |
| 21-225_7E11_HC | . | . | . | . | . | . | . | . | . | . | . | . | . | . | . | . | . |
| 21-225_6G1_HC | . | . | . | . | . | . | . | . | . | . | . | . | . | . | . | . | . |
| 21-225_15A3_HC | . | . | . | . | . | . | . | . | . | . | . | . | . | . | . | . | . |
| 21-225_13D4_HC | . | . | . | . | . | . | . | . | . | . | . | . | . | . | . | . | . |
| 21-225_16E6_HC | . | . | V | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | | | | H_FR3 | | | | | | | | | | |
| CONSENSUS | K | N | T | L | Y | L | Q | M | N | S | L | R | A | E | D | T | A |
| 21-225_20F6_HC | . | . | . | . | . | . | . | . | . | . | . | . | . | . | . | . | . |
| 21-225_18G4_HC | . | . | . | . | . | . | . | . | . | . | . | . | . | . | . | . | . |
| 21-225_22C7_HC | . | . | . | . | . | . | . | . | . | . | . | . | . | . | . | . | . |
| 21-225_27A11_HC | . | . | . | . | . | . | . | . | . | . | . | . | . | . | . | . | . |
| 21-225_26G11_HC | . | . | . | . | . | . | . | . | . | . | . | . | . | . | . | . | . |
| 21-225_26D12_HC | . | . | . | . | . | . | . | . | . | . | . | . | . | A | . | . | . |
| 21-225_25B3_HC | . | . | . | . | . | . | . | . | . | . | . | . | . | . | . | . | . |
| 21-225_25F3_HC | . | . | . | . | . | . | . | . | . | . | . | . | . | . | . | . | . |
| 21-225_3F8_HC | . | . | . | . | . | . | . | . | . | . | . | . | . | . | . | . | . |
| 21-225_7E11_HC | N | . | . | . | . | . | . | . | S | . | . | . | . | . | . | . | . |
| 21-225_6G1_HC | . | . | . | . | . | . | . | . | . | . | . | . | . | . | . | . | . |
| 21-225_15A3_HC | . | . | . | . | . | . | . | . | . | . | . | . | . | . | . | . | . |
| 21-225_13D4_HC | . | . | . | . | . | . | . | . | . | . | . | . | . | . | . | . | . |
| 21-225_16E6_HC | . | . | . | . | N | . | . | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | A | R | D | L | S | M | G | - | - | - | - | - | - |
| 21-225_20F6_HC | . | . | . | . | . | . | . | . | . | . | . | . | . | . | . | . | . |
| 21-225_18G4_HC | . | . | . | . | . | . | . | . | . | . | . | . | . | . | . | . | . |
| 21-225_22C7_HC | . | . | . | . | . | . | . | . | . | . | . | . | . | . | . | . | . |
| 21-225_27A11_HC | . | . | H | . | . | . | . | S | . | P | Y | . | . | . | . | . | . |
| 21-225_26G11_HC | . | . | . | . | . | . | . | Y | . | N | S | W | . | . | . | . | . |
| 21-225_26D12_HC | . | . | . | . | . | . | . | S | . | P | Y | . | . | . | . | . | . |
| 21-225_25B3_HC | . | . | . | . | . | . | . | S | . | P | Y | . | . | . | . | . | . |
| 21-225_25F3_HC | . | . | . | . | . | . | . | S | . | P | Y | . | . | . | . | . | . |
| 21-225_3F8_HC | . | . | . | . | . | . | . | . | . | . | . | . | . | . | . | . | . |
| 21-225_7E11_HC | . | . | . | . | . | . | . | . | . | . | . | . | . | . | . | . | . |
| 21-225_6G1_HC | M | . | . | . | . | . | . | . | . | . | . | . | . | . | . | . | . |
| 21-225_15A3_HC | . | . | . | . | . | . | . | . | . | . | . | . | . | . | . | . | . |
| 21-225_13D4_HC | . | . | . | . | . | . | . | . | . | . | . | . | . | . | . | . | . |
| 21-225 16E6 HC | . | . | . | . | . | . | . | . | . | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | | H_CDR3 | | | | | | | | | | | | | |
| CONSENSUS | - | - | - | - | - | - | - | - | - | - | - | - | - | - | - | G | M |
| 21-225_20F6_HC | . | . | . | . | . | . | . | . | . | . | . | . | . | . | . | . | . |
| 21-225_18G4_HC | . | . | . | . | . | . | . | . | . | . | . | . | . | . | . | . | . |
| 21-225_22C7_HC | . | . | . | . | . | . | . | . | . | . | . | . | . | . | . | . | . |
| 21-225_27A11_HC | . | . | . | . | . | . | . | . | . | . | . | . | . | . | . | . | . |
| 21-225_26G11_HC | . | . | . | . | . | . | . | . | . | . | . | . | . | S | G | . | . |
| 21-225_26D12_HC | . | . | . | . | . | . | . | . | . | . | . | . | . | . | . | . | . |
| 21-225_25B3_HC | . | . | . | . | . | . | . | . | . | . | . | . | . | . | . | . | . |
| 21-225_25F3_HC | . | . | . | . | . | . | . | . | . | . | . | . | . | . | . | . | . |
| 21-225_3F8_HC | . | . | . | . | . | . | . | . | . | . | . | . | . | . | . | . | . |
| 21-225_7E11_HC | . | . | . | . | . | . | . | . | . | . | . | . | . | . | . | . | . |
| 21-225_6G1_HC | . | . | . | . | . | . | . | . | . | . | . | . | . | . | . | . | . |
| 21-225_15A3_HC | . | . | . | . | . | . | . | . | . | . | . | . | . | . | . | . | . |
| 21-225_13D4_HC | . | . | . | . | . | . | . | . | . | . | . | . | . | . | . | . | . |
| 21-225 16E6 HC | . | . | . | . | . | . | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | H_FR4 | | | | | | | | | | |
| CONSENSUS | D | V | W | G | Q | G | T | T | V | T | V | S | S |
| 21-225_20F6_HC | . | . | . | . | . | . | . | . | . | . | . | . | . |
| 21-225_18G4_HC | . | . | . | . | . | . | . | . | . | . | . | . | . |
| 21-225_22C7_HC | . | . | . | . | . | . | . | . | . | . | . | . | . |
| 21-225_27A11_HC | . | . | . | . | . | . | . | . | . | . | . | . | . |
| 21-225_26G11_HC | . | . | . | . | . | . | . | . | . | . | . | . | . |
| 21-225_26D12_HC | . | . | . | . | . | . | . | . | . | . | . | . | . |
| 21-225_25B3_HC | . | . | . | . | . | . | . | . | . | . | . | . | . |
| 21-225_25F3_HC | . | . | . | . | . | . | . | . | . | . | . | . | . |
| 21-225_3F8_HC | . | . | . | . | . | . | . | . | . | . | . | . | . |
| 21-225_7E11_HC | . | . | . | . | . | . | . | . | . | . | . | . | . |
| 21-225_6G1_HC | . | . | . | . | . | . | . | . | . | . | . | . | . |
| 21-225_15A3_HC | . | . | . | . | . | . | . | . | . | . | . | . | . |
| 21-225_13D4_HC | . | . | . | . | . | . | . | . | . | . | . | . | . |
| 21-225_16E6_HC | . | . | . | . | . | . | . | . | . | . | . | . | . |

FIGURE 56

Table 30

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | H_FR1 | | | | | | | | | | | | | | | | |
| CONSENSUS | Q | V | Q | L | V | E | S | - | G | G | G | V | V | Q | P | G | R |
| 21-225_17H3_HC | . | . | . | . | . | . | . | . | . | . | . | . | . | . | . | . | . |
| 21-225_20B2_HC | . | . | . | . | . | . | . | . | . | . | . | . | . | . | . | . | . |
| 21-225_22F4_HC | . | . | . | . | . | . | . | . | . | . | . | . | . | . | . | . | . |
| 21-225_24B6_HC | . | . | . | . | . | . | . | . | . | . | . | . | . | . | . | . | . |
| 21-225_23A10_HC | . | . | . | . | . | . | . | . | . | . | . | . | . | . | . | . | . |
| 21-225_25A2_HC | . | . | . | . | . | . | . | . | . | . | . | . | . | . | . | . | . |
| 21-225_25D12_HC | . | . | . | . | . | . | . | . | . | . | . | . | . | . | . | . | . |
| 21-225_32H2_HC | . | . | . | . | . | . | . | . | . | . | . | . | . | . | . | . | . |
| 21-225_29H4_HC | . | . | . | . | . | . | . | . | . | . | . | . | . | . | . | . | . |
| 21-225_26A11_HC | . | . | . | . | . | . | . | . | . | . | . | . | . | . | . | . | . |
| 21-225_26G7_HC | . | . | . | . | . | . | . | . | . | . | . | . | . | . | . | . | . |
| 21-225_27E7_HC | . | . | . | . | . | . | . | . | . | . | . | . | . | . | . | . | . |
| 21-225_4C5_HC | . | . | . | . | . | . | . | . | . | . | . | . | . | . | . | . | . |
| 21-225_14E10_HC | . | . | . | . | . | . | . | . | . | . | . | . | . | . | . | . | . |
| 21-225_10E9_HC | . | . | . | . | . | . | . | . | . | . | . | . | . | . | . | . | . |
| 21-225_2G4_HC | . | . | . | . | . | . | . | . | . | . | . | . | . | . | . | . | . |
| 21-225_3H10_HC | . | . | . | . | . | . | . | . | . | . | . | . | . | . | . | . | . |
| 21-225_7C9_HC | . | . | . | . | . | . | . | . | . | . | . | . | . | . | . | . | . |
| 21-225_4C12_HC | . | . | . | . | . | . | . | . | . | . | . | . | . | . | . | . | . |
| 21-225_7G4_HC | . | . | . | . | . | . | . | . | . | . | . | . | . | . | . | . | . |
| 21-225_15C11_HC | . | . | . | . | . | . | . | . | . | . | . | . | . | . | . | . | . |
| 21-225_5E5_HC | . | . | . | . | . | . | . | . | . | . | . | . | . | . | . | . | . |

FIGURE 56

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | S | L | R | L | S | C | A | A | S | G | - | F | T | F | S | S | - |
| 21-225_17H3_HC | . | . | . | . | . | . | . | . | . | . | . | . | . | . | . | N | . |
| 21-225_20B2_HC | . | . | . | . | . | . | . | . | . | . | . | . | . | . | . | N | . |
| 21-225_22F4_HC | . | . | . | . | . | . | . | . | . | . | . | . | . | . | . | D | . |
| 21-225_24B6_HC | . | P | . | . | . | . | . | . | . | . | . | . | I | . | . | N | . |
| 21-225_23A10_HC | . | . | . | . | . | . | . | . | . | . | . | . | . | . | . | . | . |
| 21-225_25A2_HC | . | . | . | . | . | . | . | . | . | . | . | . | . | . | . | . | . |
| 21-225_25D12_HC | . | . | . | . | . | . | . | . | . | . | . | . | . | . | . | . | . |
| 21-225_32H2_HC | . | . | . | . | . | . | . | . | . | . | . | . | . | . | . | . | . |
| 21-225_29H4_HC | . | . | . | . | . | . | . | . | . | . | . | . | . | . | . | . | . |
| 21-225_26A11_HC | . | . | . | . | . | . | . | . | . | . | . | . | . | . | . | N | . |
| 21-225_26G7_HC | . | . | . | . | . | . | . | . | . | . | . | . | . | . | . | . | . |
| 21-225_27E7_HC | . | . | . | . | . | . | . | . | . | . | . | . | . | . | . | . | . |
| 21-225_4C5_HC | . | . | . | . | . | . | . | . | . | . | . | . | . | . | . | . | . |
| 21-225_14E10_HC | . | . | . | . | . | . | . | . | . | . | . | . | . | . | . | D | . |
| 21-225_10E9_HC | . | . | . | . | . | . | . | . | . | . | . | . | . | . | . | N | . |
| 21-225_2G4_HC | . | . | . | . | . | . | . | . | . | . | . | . | . | . | . | . | . |
| 21-225_3H10_HC | . | . | . | . | . | . | . | . | . | . | . | . | . | . | . | . | . |
| 21-225_7C9_HC | . | . | . | . | . | . | . | . | . | . | . | L | . | . | . | N | . |
| 21-225_4C12_HC | . | . | . | . | . | . | . | . | . | . | . | . | . | . | . | . | . |
| 21-225_7G4_HC | . | . | . | . | . | . | . | . | . | . | . | . | . | . | N | N | . |
| 21-225_15C11_HC | . | . | . | . | . | . | . | . | . | . | . | . | . | . | . | D | . |
| 21-225_5E5_HC | . | . | . | . | . | . | . | . | . | . | . | . | . | . | . | N | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | H_CDR1 | | | | | | | | H_FR2 | | | | | | | | |
| CONSENSUS | - | - | - | - | Y | V | M | H | W | V | R | Q | A | P | G | K | G |
| 21-225_17H3_HC | . | . | . | . | C | . | . | . | . | . | . | . | . | . | . | . | . |
| 21-225_20B2_HC | . | . | . | . | . | . | I | . | . | . | . | . | . | . | . | . | . |
| 21-225_22F4_HC | . | . | . | . | . | G | . | . | . | . | . | . | . | . | . | . | . |
| 21-225_24B6_HC | . | . | . | . | . | . | . | . | . | . | . | . | . | . | . | . | . |
| 21-225_23A10_HC | . | . | . | . | . | G | . | . | . | . | . | . | . | . | . | . | . |
| 21-225_25A2_HC | . | . | . | . | . | G | . | . | . | . | . | . | . | . | . | . | . |
| 21-225_25D12_HC | . | . | . | . | D | G | . | . | . | . | . | . | . | . | . | . | . |
| 21-225_32H2_HC | . | . | . | . | . | G | . | . | . | . | . | . | . | . | . | . | . |
| 21-225_29H4_HC | . | . | . | . | . | G | I | . | . | . | . | . | . | . | . | . | . |
| 21-225_26A11_HC | . | . | . | . | C | . | . | . | . | . | . | . | . | . | . | . | . |
| 21-225_26G7_HC | . | . | . | . | . | G | . | . | . | . | . | . | . | . | . | . | . |
| 21-225_27E7_HC | . | . | . | . | . | G | . | . | . | . | . | . | . | . | . | . | . |
| 21-225_4C5_HC | . | . | . | . | . | . | L | . | . | . | . | . | . | . | . | . | . |
| 21-225_14E10_HC | . | . | . | . | . | G | . | . | . | . | . | . | . | . | . | . | . |
| 21-225_10E9_HC | . | . | . | . | . | . | I | . | . | . | . | . | . | . | . | . | . |
| 21-225_2G4_HC | . | . | . | . | . | . | . | . | . | . | . | . | . | . | . | . | . |
| 21-225_3H10_HC | . | . | . | . | . | . | . | . | . | . | . | . | . | . | . | . | . |
| 21-225_7C9_HC | . | . | . | . | . | . | . | . | . | . | . | . | . | . | . | . | . |
| 21-225_4C12_HC | . | . | . | . | . | . | . | . | . | . | . | . | . | . | . | . | . |
| 21-225_7G4_HC | . | . | . | . | . | . | . | . | . | . | . | . | . | . | . | . | . |
| 21-225_16C11_HC | . | . | . | . | . | . | . | . | . | . | . | . | . | . | . | . | . |
| 21-225_5E5_HC | . | . | . | . | . | . | . | . | . | . | . | . | . | . | . | . | . |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | H_CDR2 | | |
| CONSENSUS | L | E | W | V | A | V | I | W | Y | D | - | - | - | G | S | N | K |
| 21-225_17H3_HC | . | . | . | . | . | . | . | . | . | . | . | . | . | . | . | . | . |
| 21-225_20B2_HC | . | . | . | . | . | . | . | . | . | . | . | . | . | . | . | . | . |
| 21-225_22F4_HC | . | . | . | . | . | . | . | . | . | . | . | . | . | . | . | . | . |
| 21-225_24B6_HC | . | . | . | . | . | . | . | . | . | . | . | . | . | . | . | . | . |
| 21-225_23A10_HC | . | . | . | . | . | . | . | . | . | . | . | . | . | . | . | . | . |
| 21-225_25A2_HC | . | . | . | . | . | . | . | . | . | . | . | . | . | . | . | . | . |
| 21-225_25D12_HC | . | . | . | . | . | . | . | . | . | . | . | . | . | . | . | . | . |
| 21-225_32H2_HC | . | . | . | . | . | . | . | . | . | . | . | . | . | . | . | . | . |
| 21-225_29H4_HC | . | . | . | . | . | . | . | . | . | . | . | . | . | . | . | . | . |
| 21-225_26A11_HC | . | . | . | . | . | . | . | . | . | . | . | . | . | . | . | . | . |
| 21-225_26G7_HC | . | . | . | . | . | . | . | . | . | . | . | . | . | . | . | . | . |
| 21-225_27E7_HC | . | . | . | . | . | . | . | . | . | . | . | . | . | . | . | . | . |
| 21-225_4C5_HC | . | . | . | . | . | . | . | . | . | . | . | . | . | . | . | . | . |
| 21-225_14E10_HC | . | . | . | . | . | . | . | . | . | . | . | . | . | . | . | . | . |
| 21-225_10E9_HC | . | . | . | . | . | . | . | . | . | . | . | . | . | . | . | . | . |
| 21-225_2G4_HC | . | . | . | . | . | . | . | . | . | . | . | . | . | . | . | . | . |
| 21-225_3H10_HC | . | . | . | . | . | . | . | . | . | . | . | . | . | . | . | . | . |
| 21-225_7C9_HC | . | . | . | . | . | . | . | . | . | . | . | . | . | . | . | . | . |
| 21-225_4C12_HC | . | . | . | . | . | . | . | . | . | . | . | . | . | . | . | . | . |
| 21-225_7G4_HC | . | . | . | . | . | . | . | . | . | . | . | . | . | . | . | . | . |
| 21-225_15C11_HC | . | . | . | . | . | . | . | . | . | . | . | . | . | . | . | . | . |
| 21-225_5E5_HC | . | . | . | . | . | . | . | . | . | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | A | D | S | V | K | G | R | F | T | I | S | R | D | N | S |
| 21-225_17H3_HC | . | . | . | . | . | . | . | . | . | . | . | . | . | . | . | . | . |
| 21-225_20B2_HC | . | . | . | . | . | . | . | . | . | . | . | . | . | . | . | . | . |
| 21-225_22F4_HC | . | . | V | . | . | . | . | . | . | . | . | . | . | . | . | . | . |
| 21-225_24B6_HC | . | . | . | . | . | . | . | . | . | . | . | . | . | . | . | . | . |
| 21-225_23A10_HC | . | . | . | . | . | . | . | . | . | . | . | . | . | . | . | . | . |
| 21-225_25A2_HC | . | . | . | . | . | . | . | . | . | . | . | . | . | . | . | . | . |
| 21-225_25D12_HC | . | . | . | . | . | . | . | . | . | . | . | . | . | . | . | . | . |
| 21-225_32H2_HC | . | . | . | . | . | . | . | . | . | . | . | . | . | . | . | . | . |
| 21-225_29H4_HC | . | . | . | . | . | . | . | . | . | . | . | . | . | . | . | . | . |
| 21-225_26A11_HC | . | . | . | . | . | . | . | . | . | . | . | . | . | . | . | . | . |
| 21-225_26G7_HC | . | . | . | . | . | . | . | . | . | . | . | . | . | . | . | . | . |
| 21-225_27E7_HC | . | . | . | . | . | . | . | . | . | . | . | . | . | . | . | . | . |
| 21-225_4C5_HC | . | . | . | . | . | . | . | . | . | . | . | . | . | . | . | . | . |
| 21-225_14E10_HC | . | . | . | . | . | . | . | . | . | . | . | . | . | . | . | . | . |
| 21-225_10E9_HC | . | . | . | . | . | . | . | . | . | . | . | . | . | . | . | . | . |
| 21-225_2G4_HC | . | . | . | . | . | . | . | . | . | . | . | . | . | . | . | . | . |
| 21-225_3H10_HC | . | . | . | . | . | . | . | . | . | . | . | . | . | . | . | . | . |
| 21-225_7C9_HC | . | . | . | . | . | . | . | . | . | . | . | . | . | . | . | . | . |
| 21-225_4C12_HC | . | . | . | . | . | . | . | . | . | . | . | . | . | . | . | . | . |
| 21-225_7G4_HC | . | . | . | . | . | . | . | . | . | . | . | . | . | . | . | . | . |
| 21-225_15C11_HC | . | . | . | . | . | . | . | . | . | . | . | . | . | . | . | . | . |
| 21-225_5E5_HC | . | . | . | . | . | . | . | . | . | . | . | . | . | . | . | . | . |

FIGURE 56

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | H_FR3 | | | | | | | | | | | | | | | | |
| CONSENSUS | K | N | T | L | Y | L | Q | M | N | S | L | R | A | E | D | T | A |
| 21-225_17H3_HC | . | . | . | . | . | . | . | . | . | . | . | . | . | . | . | . | . |
| 21-225_20B2_HC | . | . | . | . | . | . | . | . | . | . | . | . | . | . | . | . | . |
| 21-225_22F4_HC | . | . | . | . | . | . | . | . | . | . | . | . | . | . | . | . | . |
| 21-225_24B6_HC | . | . | . | . | . | . | . | . | . | . | . | . | . | . | . | . | . |
| 21-225_23A10_HC | . | . | . | . | . | . | . | . | . | . | . | . | . | . | . | . | . |
| 21-225_25A2_HC | . | . | . | . | . | . | . | . | . | . | . | . | . | . | . | . | . |
| 21-225_25D12_HC | . | . | . | . | . | . | . | . | . | . | . | . | . | . | . | . | . |
| 21-225_32H2_HC | . | . | . | . | N | . | . | . | . | . | . | . | . | . | . | . | G |
| 21-225_29H4_HC | . | . | . | . | . | . | . | . | . | . | . | . | . | . | . | . | . |
| 21-225_26A11_HC | . | . | . | . | . | . | . | . | . | . | . | . | . | . | . | . | . |
| 21-225_26G7_HC | N | . | . | . | . | . | . | . | . | . | . | . | . | . | . | . | . |
| 21-225_27E7_HC | . | . | . | . | . | . | . | . | . | . | . | . | . | . | . | . | . |
| 21-225_4C5_HC | . | . | . | . | . | . | . | . | . | . | . | . | . | . | . | . | . |
| 21-225_14E10_HC | . | . | . | . | . | . | . | . | . | . | . | . | . | . | . | . | . |
| 21-225_10E9_HC | . | . | . | . | . | . | . | . | . | . | . | . | . | . | . | . | . |
| 21-225_2G4_HC | . | . | . | . | . | . | . | . | . | . | . | . | . | . | . | . | . |
| 21-225_3H10_HC | . | . | . | . | . | . | . | . | . | . | . | . | . | . | . | . | . |
| 21-225_7C9_HC | . | . | . | . | . | . | . | . | . | . | . | . | . | . | . | . | . |
| 21-225_4C12_HC | . | . | . | . | . | . | . | . | . | . | . | . | . | . | . | . | . |
| 21-225_7G4_HC | . | . | . | . | . | . | . | . | T | . | . | . | . | . | . | . | . |
| 21-225_15C11_HC | . | . | . | . | . | . | . | . | . | . | . | . | . | . | . | . | . |
| 21-225_5E5_HC | . | . | . | . | . | . | . | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | A | R | E | K | Y | S | S | S | W | - | - | - | - |
| 21-225_17H3_HC | . | . | . | . | . | . | . | . | . | . | . | . | . | . | . | . | . |
| 21-225_20B2_HC | . | . | S | . | . | . | . | . | . | . | . | . | . | . | . | . | . |
| 21-225_22F4_HC | . | . | . | . | . | . | . | . | . | . | . | . | . | . | . | . | . |
| 21-225_24B6_HC | . | . | . | . | . | . | . | E | . | . | . | G | . | . | . | . | . |
| 21-225_23A10_HC | . | . | . | . | . | . | . | R | . | . | . | G | . | . | . | . | . |
| 21-225_25A2_HC | . | . | . | . | . | . | K | Y | S | . | . | W | - | . | . | . | . |
| 21-225_25D12_HC | . | . | . | . | . | . | . | R | . | . | . | . | . | . | . | . | . |
| 21-225_32H2_HC | . | . | . | . | . | . | K | Y | S | . | . | W | - | . | . | . | . |
| 21-225_29H4_HC | . | . | . | . | . | . | K | Y | S | . | . | W | - | . | . | . | . |
| 21-225_26A11_HC | . | . | . | . | . | . | . | E | . | . | . | G | . | . | . | . | . |
| 21-225_26G7_HC | . | . | . | . | . | . | K | Y | S | . | . | W | - | . | . | . | . |
| 21-225_27E7_HC | . | . | . | . | . | . | . | D | S | Y | C | . | S | T | S | C | . |
| 21-225_4C5_HC | . | . | . | . | . | . | . | . | . | T | . | . | . | . | . | . | . |
| 21-225_14E10_HC | . | . | . | . | . | . | . | . | . | . | . | . | . | . | . | . | . |
| 21-225_10E9_HC | . | . | . | . | . | . | . | . | . | . | . | . | . | . | . | . | . |
| 21-225_2G4_HC | . | . | . | . | . | . | . | . | . | . | . | . | . | . | . | . | . |
| 21-225_3H10_HC | . | . | . | . | . | . | . | R | . | . | . | G | . | . | . | . | . |
| 21-225_7C9_HC | . | . | . | . | . | . | . | . | . | . | . | . | . | . | . | . | . |
| 21-225_4C12_HC | . | . | . | . | . | . | . | . | . | . | . | . | . | . | . | . | . |
| 21-225_7G4_HC | . | . | . | . | . | . | . | . | . | . | . | N | . | . | . | . | . |
| 21-225_15C11_HC | . | . | . | . | . | . | . | . | . | . | . | . | . | . | . | . | . |
| 21-225_5E5_HC | . | . | . | . | T | . | . | V | . | . | . | G | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | H_CDR3 | | | | | | | | | | | | | | | | |
| CONSENSUS | - | - | - | - | - | - | - | - | - | - | - | - | Y | D | Y | G | M |
| 21-225_17H3_HC | . | . | . | . | . | . | . | . | . | . | . | . | . | . | . | . | . |
| 21-225_20B2_HC | . | . | . | . | . | . | . | . | . | . | . | . | . | . | . | . | . |
| 21-225_22F4_HC | . | . | . | . | . | . | . | . | . | . | . | . | . | . | . | . | . |
| 21-225_24B6_HC | . | . | . | . | . | . | . | . | . | . | . | . | . | . | . | . | . |
| 21-225_23A10_HC | . | . | . | . | . | . | . | . | . | . | . | . | [illegible] | . | . | . | . |
| 21-225_25A2_HC | . | . | . | . | . | . | . | . | . | . | . | . | - | S | G | . | . |
| 21-225_25D12_HC | . | . | . | . | . | . | . | . | . | . | . | . | . | . | . | . | . |
| 21-225_32H2_HC | . | . | . | . | . | . | . | . | . | . | . | . | - | T | G | . | . |
| 21-225_29H4_HC | . | . | . | . | . | . | . | . | . | . | . | . | - | S | G | . | . |
| 21-225_26A11_HC | . | . | . | . | . | . | . | . | . | . | . | . | . | . | . | . | . |
| 21-225_26G7_HC | . | . | . | . | . | . | . | . | . | . | . | . | - | S | G | . | . |
| 21-225_27E7_HC | . | . | . | . | . | . | . | . | . | P | Y | Y | . | Y | . | . | . |
| 21-225_4C5_HC | . | . | . | . | . | . | . | . | . | . | . | . | . | . | . | . | . |
| 21-225_14E10_HC | . | . | . | . | . | . | . | . | . | . | . | . | . | . | . | . | . |
| 21-225_10E9_HC | . | . | . | . | . | . | . | . | . | . | . | . | . | . | . | . | . |
| 21-225_2G4_HC | . | . | . | . | . | . | . | . | . | . | . | . | . | . | . | . | . |
| 21-225_3H10_HC | . | . | . | . | . | . | . | . | . | . | . | . | . | . | . | . | L |
| 21-225_7C9_HC | . | . | . | . | . | . | . | . | . | . | . | . | . | . | . | . | . |
| 21-225_4C12_HC | . | . | . | . | . | . | . | . | . | . | . | . | . | . | . | . | . |
| 21-225_7G4_HC | . | . | . | . | . | . | . | . | . | . | . | . | . | . | . | . | . |
| 21-225_15C11_HC | . | . | . | . | . | . | . | . | . | . | . | . | . | . | . | . | L |
| 21-225_5E5_HC | . | . | . | . | . | . | . | . | . | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | | H_CDR3 | | | | | | | | | | | | | |
| CONSENSUS | - | - | - | - | - | - | - | - | - | - | - | - | Y | D | Y | G | M |
| 21-225_17H3_HC | . | . | . | . | . | . | . | . | . | . | . | . | . | . | . | . | . |
| 21-225_20B2_HC | . | . | . | . | . | . | . | . | . | . | . | . | . | . | . | . | . |
| 21-225_22F4_HC | . | . | . | . | . | . | . | . | . | . | . | . | . | . | . | . | . |
| 21-225_24B6_HC | . | . | . | . | . | . | . | . | . | . | . | . | . | . | . | . | . |
| 21-225_23A10_HC | . | . | . | . | . | . | . | . | . | . | . | . | [illegible] | . | . | . | . |
| 21-225_25A2_HC | . | . | . | . | . | . | . | . | . | . | . | . | - | S | G | . | . |
| 21-225_25D12_HC | . | . | . | . | . | . | . | . | . | . | . | . | . | . | . | . | . |
| 21-225_32H2_HC | . | . | . | . | . | . | . | . | . | . | . | . | - | T | G | . | . |
| 21-225_29H4_HC | . | . | . | . | . | . | . | . | . | . | . | . | - | S | G | . | . |
| 21-225_26A11_HC | . | . | . | . | . | . | . | . | . | . | . | . | . | . | . | . | . |
| 21-225_26G7_HC | . | . | . | . | . | . | . | . | . | . | . | . | - | S | G | . | . |
| 21-225_27E7_HC | . | . | . | . | . | . | . | . | . | P | Y | Y | . | Y | . | . | . |
| 21-225_4C5_HC | . | . | . | . | . | . | . | . | . | . | . | . | . | . | . | . | . |
| 21-225_14E10_HC | . | . | . | . | . | . | . | . | . | . | . | . | . | . | . | . | . |
| 21-225_10E9_HC | . | . | . | . | . | . | . | . | . | . | . | . | . | . | . | . | . |
| 21-225_2G4_HC | . | . | . | . | . | . | . | . | . | . | . | . | . | . | . | . | . |
| 21-225_3H10_HC | . | . | . | . | . | . | . | . | . | . | . | . | . | . | . | . | L |
| 21-225_7C9_HC | . | . | . | . | . | . | . | . | . | . | . | . | . | . | . | . | . |
| 21-225_4C12_HC | . | . | . | . | . | . | . | . | . | . | . | . | . | . | . | . | . |
| 21-225_7G4_HC | . | . | . | . | . | . | . | . | . | . | . | . | . | . | . | . | . |
| 21-225_15C11_HC | . | . | . | . | . | . | . | . | . | . | . | . | . | . | . | . | L |
| 21-225_5E5_HC | . | . | . | . | . | . | . | . | . | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | H_CDR3 | | | | | | | | | | | | | | | | |
| CONSENSUS | - | - | - | - | - | - | - | - | - | - | - | - | Y | D | Y | G | M |
| 21-225_17H3_HC | . | . | . | . | . | . | . | . | . | . | . | . | . | . | . | . | . |
| 21-225_20B2_HC | . | . | . | . | . | . | . | . | . | . | . | . | . | . | . | . | . |
| 21-225_22F4_HC | . | . | . | . | . | . | . | . | . | . | . | . | . | . | . | . | . |
| 21-225_24B6_HC | . | . | . | . | . | . | . | . | . | . | . | . | . | . | . | . | . |
| 21-225_23A10_HC | . | . | . | . | . | . | . | . | . | . | . | . | [illegible] | . | . | . | . |
| 21-225_25A2_HC | . | . | . | . | . | . | . | . | . | . | . | . | - | S | G | . | . |
| 21-225_25D12_HC | . | . | . | . | . | . | . | . | . | . | . | . | . | . | . | . | . |
| 21-225_32H2_HC | . | . | . | . | . | . | . | . | . | . | . | . | - | T | G | . | . |
| 21-225_29H4_HC | . | . | . | . | . | . | . | . | . | . | . | . | - | S | G | . | . |
| 21-225_26A11_HC | . | . | . | . | . | . | . | . | . | . | . | . | . | . | . | . | . |
| 21-225_26G7_HC | . | . | . | . | . | . | . | . | . | . | . | . | - | S | G | . | . |
| 21-225_27E7_HC | . | . | . | . | . | . | . | . | . | P | Y | Y | . | Y | . | . | . |
| 21-225_4C5_HC | . | . | . | . | . | . | . | . | . | . | . | . | . | . | . | . | . |
| 21-225_14E10_HC | . | . | . | . | . | . | . | . | . | . | . | . | . | . | . | . | . |
| 21-225_10E9_HC | . | . | . | . | . | . | . | . | . | . | . | . | . | . | . | . | . |
| 21-225_2G4_HC | . | . | . | . | . | . | . | . | . | . | . | . | . | . | . | . | . |
| 21-225_3H10_HC | . | . | . | . | . | . | . | . | . | . | . | . | . | . | . | . | L |
| 21-225_7C9_HC | . | . | . | . | . | . | . | . | . | . | . | . | . | . | . | . | . |
| 21-225_4C12_HC | . | . | . | . | . | . | . | . | . | . | . | . | . | . | . | . | . |
| 21-225_7G4_HC | . | . | . | . | . | . | . | . | . | . | . | . | . | . | . | . | . |
| 21-225_15C11_HC | . | . | . | . | . | . | . | . | . | . | . | . | . | . | . | . | L |
| 21-225_5E5_HC | . | . | . | . | . | . | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | H_FR4 | | | | | | | | | | |
| CONSENSUS | D | V | W | G | Q | G | T | T | V | T | V | S | S |
| 21-225_17H3_HC | . | . | . | . | . | . | . | . | . | . | . | . | . |
| 21-225_20B2_HC | . | . | . | . | . | . | . | . | . | . | . | . | . |
| 21-225_22F4_HC | . | . | . | . | . | . | . | . | . | . | . | . | . |
| 21-225_24B6_HC | . | . | . | . | . | . | . | . | . | . | . | . | . |
| 21-225_23A10_HC | . | . | . | . | . | . | . | . | . | . | . | . | . |
| 21-225_25A2_HC | . | . | . | . | . | . | . | . | . | . | . | . | . |
| 21-225_25D12_HC | . | . | . | . | . | . | . | . | . | . | . | . | . |
| 21-225_32H2_HC | . | . | . | . | . | . | . | . | . | . | . | . | . |
| 21-225_29H4_HC | . | . | . | . | . | . | . | . | . | . | . | . | . |
| 21-225_26A11_HC | . | . | . | . | . | . | . | . | . | . | . | . | . |
| 21-225_26G7_HC | . | . | . | . | . | . | . | . | . | . | . | . | . |
| 21-225_27E7_HC | . | . | . | . | . | . | . | . | . | . | . | . | . |
| 21-225_4C5_HC | . | . | . | . | . | . | . | . | . | . | . | . | . |
| 21-225_14E10_HC | . | . | . | . | . | . | . | . | . | . | . | . | . |
| 21-225_10E9_HC | . | . | . | . | . | . | . | . | . | . | . | . | . |
| 21-225_2G4_HC | . | . | . | . | H | . | . | . | . | . | . | . | . |
| 21-225_3H10_HC | . | . | . | . | . | . | . | . | . | . | . | . | . |
| 21-225_7C9_HC | . | . | . | . | . | . | . | . | . | . | . | . | . |
| 21-225_4C12_HC | . | . | . | . | H | . | . | . | . | . | . | . | . |
| 21-225_7G4_HC | . | . | . | . | . | . | . | . | . | . | . | . | . |
| 21-225_15C11_HC | . | . | . | . | . | . | . | . | . | . | . | . | . |
| 21-225_5E5_HC | . | . | . | . | . | . | . | . | . | . | . | . | . |

**FIGURE 56**

**Table 31**

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | | | | | H_FR1 | |
| CONSENSUS | Q | V | Q | L | V | E | S | - | G | G | G | V | V | Q | P | G | R |
| 21-225_18H12_HC | . | . | . | . | . | . | . | . | . | . | . | . | . | . | . | . | . |
| 21-225_22B7_HC | . | . | . | . | . | . | . | . | . | . | . | . | . | . | . | . | . |
| 21-225_25A9_HC | . | . | . | . | . | . | . | . | . | . | . | . | . | . | . | . | . |
| 21-225_29D9_HC | . | . | . | . | . | . | . | . | . | . | . | . | . | . | . | . | . |
| 21-225_25G5_HC | . | . | . | . | . | . | . | . | . | . | . | . | . | . | . | . | . |
| 21-225_28B8_HC | . | . | . | . | . | . | . | . | . | . | . | . | . | . | . | . | . |
| 21-225_28A9_HC | . | . | . | . | . | . | . | . | . | . | . | . | . | . | . | . | . |
| 21-225_29D8_HC | . | . | . | . | . | . | . | . | . | . | . | . | . | . | . | . | . |
| 21-225_30F3_HC | . | . | . | . | . | . | . | . | . | . | . | . | . | . | . | . | . |
| 21-225_33B1_HC | . | . | . | . | . | . | . | . | . | . | . | . | . | . | . | . | . |
| 21-225_15B11_HC | . | . | . | . | . | . | . | . | . | . | . | . | . | . | . | . | . |
| 21-225_14C2_HC | . | . | . | . | . | . | . | . | . | . | . | . | . | . | . | . | . |
| 21-225_4C8_HC | . | . | . | . | . | . | . | . | . | . | . | . | . | . | . | . | . |
| 21-225_4F12_HC | . | . | . | . | . | . | . | . | . | . | . | . | . | . | . | . | . |
| 21-225_15H10_HC | . | . | . | . | . | . | . | . | . | . | . | . | . | . | . | . | . |
| 21-225_16H4_HC | . | . | . | . | . | . | . | . | . | . | . | . | . | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | S | L | R | L | S | C | A | A | S | G | - | F | T | F | S | D | - |
| 21-225_18H12_HC | . | . | . | . | . | . | . | . | . | . | . | . | . | . | . | . | . |
| 21-225_22B7_HC | . | . | . | . | . | . | . | . | . | . | . | . | . | . | . | S | . |
| 21-225_25A9_HC | . | . | . | . | . | . | . | . | . | . | . | . | . | . | . | S | . |
| 21-225_29D9_HC | . | . | K | . | . | . | S | . | . | . | . | . | . | . | . | . | . |
| 21-225_25G5_HC | . | . | . | . | . | . | . | . | . | . | . | . | . | . | . | . | . |
| 21-225_28B8_HC | . | . | . | . | . | . | . | . | . | . | . | . | . | . | . | . | . |
| 21-225_28A9_HC | . | . | . | . | . | . | . | . | . | . | . | . | . | . | . | S | . |
| 21-225_29D8_HC | . | . | . | . | . | . | . | . | . | . | . | . | . | . | R | . | . |
| 21-225_30F3_HC | . | . | . | . | . | . | . | . | . | . | . | . | . | . | . | S | . |
| 21-225_33B1_HC | . | . | . | . | . | . | . | . | . | . | . | . | . | . | . | . | . |
| 21-225_15B11_HC | . | . | . | . | . | . | . | . | . | . | . | . | . | . | . | N | . |
| 21-225_14C2_HC | . | . | . | . | . | . | . | . | . | . | . | . | . | . | . | . | . |
| 21-225_4C8_HC | . | . | . | . | . | . | . | . | . | . | . | . | . | . | . | N | . |
| 21-225_4F12_HC | . | . | . | . | . | . | . | . | . | . | . | . | . | . | . | S | . |
| 21-225_15H10_HC | . | . | . | . | . | . | . | . | . | . | . | . | . | . | . | N | . |
| 21-225_16H4_HC | . | . | . | . | . | . | . | . | . | . | . | . | . | . | . | N | . |

**FIGURE 56**

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | H_CDR1 | | | | | | | | H_FR2 | | | | | | | | |
| CONSENSUS | - | - | - | - | Y | G | M | H | W | V | R | Q | A | P | G | K | G |
| 21-225_18H12_HC | . | . | . | . | . | . | . | . | . | . | . | . | . | . | . | . | . |
| 21-225_22B7_HC | . | . | . | . | . | . | . | . | . | . | . | . | . | . | . | . | . |
| 21-225_25A9_HC | . | . | . | . | . | . | . | . | . | . | . | . | . | . | . | . | . |
| 21-225_29D9_HC | . | . | . | . | . | . | I | . | . | . | . | . | . | . | . | . | . |
| 21-225_25G5_HC | . | . | . | . | . | . | I | . | . | . | . | . | . | . | . | . | . |
| 21-225_28B8_HC | . | . | . | . | . | . | . | . | . | . | . | . | . | . | . | . | . |
| 21-225_28A9_HC | . | . | . | . | . | . | I | . | . | . | . | . | . | . | . | . | . |
| 21-225_29D8_HC | . | . | . | . | . | . | I | . | . | . | . | . | . | . | . | . | . |
| 21-225_30F3_HC | . | . | . | . | . | . | . | . | . | . | . | . | . | . | . | . | . |
| 21-225_33B1_HC | . | . | . | . | . | . | . | . | . | . | . | . | . | . | A | . | . |
| 21-225_15B11_HC | . | . | . | . | . | . | . | . | . | . | . | . | . | . | . | . | . |
| 21-225_14C2_HC | . | . | . | . | . | . | . | . | . | . | . | . | . | . | . | . | . |
| 21-225_4C8_HC | . | . | . | . | . | . | . | . | . | . | . | . | . | . | . | . | . |
| 21-225_4F12_HC | . | . | . | . | . | . | . | . | . | . | . | . | . | . | . | . | . |
| 21-225_15H10_HC | . | . | . | . | . | . | . | . | . | . | . | . | . | . | . | . | . |
| 21-225_16H4_HC | . | . | . | . | . | . | . | . | . | . | . | . | . | . | . | . | . |

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | H_CDR2 | | | | | | | | | | | |
| CONSENSUS | L | E | W | V | A | V | I | W | Y | D | - | - | - | E | S | N | K |
| 21-225_18H12_HC | . | . | . | M | . | . | . | . | . | . | . | . | . | V | T | . | . |
| 21-225_22B7_HC | . | . | . | . | . | . | . | . | . | E | . | . | . | . | N | . | Q |
| 21-225_25A9_HC | . | . | . | . | . | . | . | . | . | . | . | . | . | V | T | . | . |
| 21-225_29D9_HC | . | . | . | . | . | . | . | . | . | . | . | . | . | . | . | . | N |
| 21-225_25G5_HC | . | . | . | . | . | . | . | . | . | . | . | . | . | G | N | . | . |
| 21-225_28B8_HC | . | . | . | . | T | . | . | . | . | . | . | . | . | A | N | . | . |
| 21-225_28A9_HC | . | . | . | . | . | . | . | . | F | . | . | . | . | G | . | . | . |
| 21-225_29D8_HC | . | . | . | . | . | . | . | . | F | . | . | . | . | G | T | . | . |
| 21-225_30F3_HC | . | . | . | . | . | . | . | . | . | . | . | . | . | V | . | . | . |
| 21-225_33B1_HC | . | . | . | . | . | . | . | . | . | . | . | . | . | R | N | . | . |
| 21-225_15B11_HC | . | . | . | . | . | . | . | . | . | E | . | . | . | . | . | . | . |
| 21-225_14C2_HC | . | . | . | . | . | . | . | . | . | . | . | . | . | . | . | . | . |
| 21-225_4C8_HC | . | . | . | . | . | . | . | . | . | E | . | . | . | . | N | . | Q |
| 21-225_4F12_HC | . | . | . | . | . | . | . | . | . | . | . | . | . | V | T | . | . |
| 21-225_15H10_HC | . | . | . | . | . | . | . | . | . | . | . | . | . | . | . | . | . |
| 21-225_16H4_HC | . | . | . | . | . | . | . | . | . | . | . | . | . | V | . | . | . |

**FIGURE 56**

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | A | D | S | V | K | G | R | F | T | I | S | R | D | N | S |
| 21-225_18H12_HC | . | . | . | . | . | . | . | . | . | . | . | . | . | . | . | . | . |
| 21-225_22B7_HC | . | . | . | . | . | . | . | . | . | . | . | . | . | . | . | . | . |
| 21-225_25A9_HC | . | . | T | G | . | . | . | . | . | . | . | . | . | . | . | . | . |
| 21-225_29D9_HC | . | . | . | . | . | . | . | . | . | . | . | . | . | . | . | . | . |
| 21-225_25G5_HC | . | . | . | . | . | . | . | . | . | . | . | . | . | . | . | . | . |
| 21-225_28B8_HC | . | . | . | . | . | . | . | . | . | . | . | . | . | . | . | . | F |
| 21-225_28A9_HC | . | . | . | . | . | . | . | . | . | . | . | . | . | . | . | . | . |
| 21-225_29D8_HC | . | . | . | . | . | . | . | . | . | . | . | . | . | . | . | . | . |
| 21-225_30F3_HC | . | . | . | . | . | . | . | . | . | . | . | . | . | . | . | . | . |
| 21-225_33B1_HC | . | . | . | . | . | . | . | . | . | . | . | . | . | . | . | . | . |
| 21-225_15B11_HC | . | . | T | . | . | . | . | . | . | . | . | . | . | . | . | . | . |
| 21-225_14C2_HC | . | . | S | . | . | . | . | . | . | . | . | . | . | . | . | . | . |
| 21-225_4C8_HC | . | . | . | . | . | . | . | . | . | . | . | . | . | . | . | . | . |
| 21-225_4F12_HC | K | . | . | . | . | . | . | . | . | . | . | . | . | . | . | . | . |
| 21-225_15H10_HC | . | . | S | . | . | . | . | . | . | . | . | . | . | . | . | . | . |
| 21-225_16H4_HC | . | . | . | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | | | | H_FR3 | | | | | | | | | | |
| CONSENSUS | K | N | T | L | Y | L | Q | M | N | S | L | R | A | E | D | T | A |
| 21-225_18H12_HC | . | . | . | . | . | . | . | . | . | . | . | . | . | . | . | . | . |
| 21-225_22B7_HC | . | . | . | . | . | . | . | . | . | . | . | . | . | . | . | . | . |
| 21-225_25A9_HC | . | . | . | . | . | . | . | . | . | . | . | . | . | . | . | . | . |
| 21-225_29D9_HC | . | . | . | . | . | . | . | . | . | . | . | . | . | . | . | . | . |
| 21-225_25G5_HC | . | . | . | . | . | . | . | . | . | N | . | . | . | . | . | . | . |
| 21-225_28B8_HC | . | . | . | V | . | . | . | . | . | . | . | . | . | . | . | . | . |
| 21-225_28A9_HC | . | . | . | . | F | . | . | . | . | . | . | . | V | . | . | . | . |
| 21-225_29D8_HC | . | . | . | . | . | . | . | . | . | . | . | . | . | . | . | . | . |
| 21-225_30F3_HC | . | . | . | . | . | . | . | . | . | . | . | . | . | . | . | . | . |
| 21-225_33B1_HC | . | . | . | . | . | . | . | . | . | . | . | . | . | . | . | . | . |
| 21-225_15B11_HC | . | . | . | . | . | . | . | . | . | . | . | . | P | . | . | . | . |
| 21-225_14C2_HC | . | . | . | . | . | . | . | . | K | . | . | . | . | . | . | . | G |
| 21-225_4C8_HC | . | . | . | . | . | . | . | . | . | . | . | T | . | . | . | . | . |
| 21-225_4F12_HC | . | . | . | . | . | . | . | . | . | . | . | T | P | . | N | . | G |
| 21-225_15H10_HC | . | . | . | . | . | . | . | . | . | . | . | . | . | . | . | . | . |
| 21-225_16H4_HC | . | . | M | . | . | . | . | . | . | . | . | . | . | . | . | . | . |

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | A | R | E | I | G | W | - | - | - | - | - | - | - |
| 21-225_18H12_HC | . | . | . | . | . | . | . | V | . | . | . | . | . | . | . | . | . |
| 21-225_22B7_HC | . | . | . | . | . | . | . | V | . | F | . | . | . | . | . | . | . |
| 21-225_25A9_HC | . | . | . | . | . | . | . | . | . | . | . | . | . | . | . | . | . |
| 21-225_29D9_HC | . | . | . | . | . | . | . | . | . | . | . | . | . | . | . | . | . |
| 21-225_25G5_HC | . | . | . | . | . | . | . | . | . | . | . | . | . | . | . | . | . |
| 21-225_28B8_HC | . | . | . | . | . | . | . | . | . | . | . | . | . | . | . | . | . |
| 21-225_28A9_HC | . | . | . | . | . | . | . | . | . | . | . | . | . | . | . | . | . |
| 21-225_29D8_HC | . | . | . | . | G | . | . | . | . | . | . | . | . | . | . | . | . |
| 21-225_30F3_HC | . | . | . | . | . | . | . | M | . | . | . | . | . | . | . | . | . |
| 21-225_33B1_HC | . | . | . | . | . | . | . | M | . | . | . | . | . | . | . | . | . |
| 21-225_15B11_HC | . | . | . | . | . | . | . | V | . | F | . | . | . | . | . | . | . |
| 21-225_14C2_HC | . | . | . | . | . | . | . | V | . | F | . | . | . | . | . | . | . |
| 21-225_4C8_HC | . | . | . | S | P | . | . | M | . | F | . | . | . | . | . | . | . |
| 21-225_4F12_HC | G | . | E | N | Q | . | . | K | . | G | . | . | . | . | . | . | . |
| 21-225_15H10_HC | . | . | . | . | V | . | . | V | . | F | . | . | . | . | . | . | . |
| 21-225_16H4_HC | . | . | . | . | . | . | . | M | . | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | | H_CDR3 | | | | | | | | | | | | | |
| CONSENSUS | - | - | - | - | - | - | - | - | - | - | - | - | - | - | - | L | D |
| 21-225_18H12_HC | . | . | . | . | . | . | . | . | . | . | . | . | . | . | . | Y | E |
| 21-225_22B7_HC | . | . | . | . | . | . | . | . | . | . | . | . | . | . | . | . | S |
| 21-225_25A9_HC | . | . | . | . | . | . | . | . | . | . | . | . | . | . | . | . | . |
| 21-225_29D9_HC | . | . | . | . | . | . | . | . | . | . | . | . | . | . | . | . | . |
| 21-225_25G5_HC | . | . | . | . | . | . | . | . | . | . | . | . | . | . | . | . | . |
| 21-225_28B8_HC | . | . | . | . | . | . | . | . | . | . | . | . | . | . | . | . | . |
| 21-225_28A9_HC | . | . | . | . | . | . | . | . | . | . | . | . | . | . | . | . | . |
| 21-225_29D8_HC | . | . | . | . | . | . | . | . | . | . | . | . | . | . | . | . | . |
| 21-225_30F3_HC | . | . | . | . | . | . | . | . | . | . | . | . | . | . | . | Y | . |
| 21-225_33B1_HC | . | . | . | . | . | . | . | . | . | . | . | . | . | . | . | Y | . |
| 21-225_15B11_HC | . | . | . | . | . | . | . | . | . | . | . | . | . | . | . | T | E |
| 21-225_14C2_HC | . | . | . | . | . | . | . | . | . | . | . | . | . | . | . | . | S |
| 21-225_4C8_HC | . | . | . | . | . | . | . | . | . | . | . | . | . | . | . | . | S |
| 21-225_4F12_HC | . | . | . | . | . | . | . | . | . | . | . | . | . | . | . | . | F |
| 21-225_15H10_HC | . | . | . | . | . | . | . | . | . | . | . | . | . | . | . | . | S |
| 21-225_16H4_HC | . | . | . | . | . | . | . | . | . | . | . | . | . | . | . | T | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | H_FR4 | | | | | | | | | | |
| CONSENSUS | D | Y | W | G | Q | G | T | L | V | T | V | S | S |
| 21-225_18H12_HC | . | . | . | . | . | . | . | . | . | . | . | . | . |
| 21-225_22B7_HC | . | . | . | . | . | . | . | . | . | . | . | . | . |
| 21-225_25A9_HC | . | . | . | . | . | . | . | . | . | . | . | . | . |
| 21-225_29D9_HC | . | . | . | . | . | . | . | Q | . | . | . | . | . |
| 21-225_25G5_HC | . | . | . | . | . | . | . | . | . | . | . | . | . |
| 21-225_28B8_HC | . | . | . | . | . | . | . | . | . | . | . | . | . |
| 21-225_28A9_HC | . | . | . | . | . | . | . | . | . | . | . | . | . |
| 21-225_29D8_HC | . | . | . | . | . | . | . | . | . | . | . | . | . |
| 21-225_30F3_HC | . | . | . | . | . | . | . | . | . | . | . | . | . |
| 21-225_33B1_HC | . | . | . | . | . | . | . | . | . | . | . | . | . |
| 21-225_15B11_HC | . | . | . | . | . | . | . | . | . | . | . | . | . |
| 21-225_14C2_HC | . | . | . | . | . | . | . | . | . | . | . | . | . |
| 21-225_4C8_HC | . | . | . | . | . | . | . | . | . | . | . | . | . |
| 21-225_4F12_HC | . | . | . | . | . | . | . | . | . | . | . | . | . |
| 21-225_15H10_HC | . | . | . | . | . | . | I | . | . | . | . | . | . |
| 21-225_16H4_HC | . | C | . | . | . | . | . | . | . | . | . | . | . |

FIGURE 56

Table 32

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | | | | | H_FR1 | |
| CONSENSUS | Q | V | Q | L | V | E | S | - | G | G | G | V | V | Q | P | G | R |
| 21-225_16F10_HC | . | . | . | . | . | . | . | . | . | . | . | . | . | . | . | . | . |
| 21-225_18A5_HC | . | . | . | . | . | . | . | . | . | . | . | . | . | . | . | . | . |
| 21-225_20E5_HC | . | . | . | . | . | . | . | . | . | . | . | . | . | . | . | . | . |
| 21-225_18A1_HC | . | . | . | . | . | . | . | . | . | . | . | . | . | . | . | . | . |
| 21-225_19E1_HC | . | . | . | . | . | . | . | . | . | . | . | . | . | . | . | . | . |
| 21-225_17A10_HC | . | . | . | . | . | . | . | . | . | . | . | . | . | . | . | . | . |
| 21-225_18E8_HC | . | . | . | . | . | . | . | . | . | . | . | . | . | . | . | . | . |
| 21-225_16F11_HC | . | . | . | M | . | . | . | . | . | . | . | . | . | . | . | . | . |
| 21-225_18C2_HC | . | . | . | . | . | . | . | . | . | . | . | . | . | . | . | . | . |
| 21-225_20A7_HC | . | . | . | . | . | . | . | . | . | . | . | . | . | . | . | . | . |
| 21-225_17C7_HC | . | . | . | . | . | . | . | . | . | . | . | . | . | . | . | . | K |
| 21-225_18F2_HC | . | . | . | . | . | . | . | . | . | . | . | . | . | . | . | . | . |
| 21-225_17B5_HC | . | . | . | . | . | . | . | . | . | . | . | . | . | . | . | . | . |
| 21-225_17E5_HC | . | . | . | . | . | . | . | . | . | . | . | . | . | . | . | . | . |
| 21-225_20D5_HC | . | . | . | . | . | . | . | . | . | . | . | . | . | . | . | . | . |
| 21-225_21G11_HC | . | . | . | . | . | . | . | . | . | . | . | . | . | . | . | . | . |
| 21-225_20E12_HC | . | . | . | . | . | . | . | . | . | . | . | . | . | . | . | . | . |
| 21-225_20D10_HC | . | . | . | . | . | . | . | . | . | . | . | . | . | . | . | . | . |
| 21-225_22A4_HC | . | . | . | . | . | . | . | . | . | . | . | . | . | . | . | . | . |
| 21-225_20H12_HC | . | . | . | . | . | . | . | . | . | . | . | . | . | . | . | . | . |
| 21-225_21H8_HC | . | . | . | . | . | . | . | . | . | . | . | . | . | . | S | . | . |
| 21-225_21E5_HC | . | . | . | . | . | . | . | . | . | . | . | . | . | . | . | . | . |
| 21-225_22H8_HC | . | . | . | . | . | . | . | . | . | . | . | . | . | . | . | . | . |
| 21-225_23H11_HC | . | S | . | . | . | . | . | . | . | . | . | . | . | . | . | . | . |
| 21-225_21F7_HC | . | . | . | . | . | . | . | . | . | . | . | . | . | . | . | . | . |
| 21-225_22F9_HC | . | . | . | M | . | . | . | . | . | . | . | . | . | . | . | . | . |
| 21-225_21G2_HC | . | . | . | . | . | . | . | . | . | . | . | . | . | . | . | . | . |
| 21-225_22F2_HC | . | . | . | . | . | . | . | . | . | . | . | . | . | . | . | . | . |
| 21-225_23F12_HC | . | . | . | . | . | . | . | . | . | . | . | . | . | . | . | . | . |
| 21-225_28F5_HC | . | . | . | . | . | . | . | . | . | . | . | . | . | . | . | . | . |
| 21-225_29G4_HC | . | . | . | . | . | . | . | . | . | . | . | . | . | . | . | . | . |
| 21-225_27D5_HC | . | . | . | . | . | . | . | . | . | . | . | . | . | . | . | . | . |
| 21-225_28C7_HC | . | . | . | . | . | . | . | . | . | . | . | . | . | . | . | . | . |
| 21-225_25B8_HC | . | . | . | . | . | . | . | . | . | . | . | . | . | . | . | . | . |
| 21-225_26A2_HC | . | . | . | . | . | . | . | . | . | . | . | . | . | . | . | . | . |
| 21-225_29H6_HC | . | . | . | . | . | . | . | . | . | . | . | . | . | . | . | . | . |
| 21-225_25E6_HC | . | . | . | . | . | . | . | . | . | . | . | . | . | . | . | . | . |
| 21-225_25H10_HC | . | . | . | . | . | . | . | . | . | . | . | . | . | . | . | . | . |
| 21-225_29D7_HC | . | . | . | . | . | . | . | . | . | . | . | . | . | . | . | . | . |
| 21-225_29B8_HC | . | . | . | . | . | . | . | . | . | . | . | . | . | . | . | . | . |

FIGURE 56

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | H_FR1 | | | | | | | | | | | | | | | | |
| CONSENSUS | Q | V | Q | L | V | E | S | - | G | G | G | V | V | Q | P | G | R |
| 21-225_25H11_HC | . | . | . | . | . | . | . | . | . | . | . | . | . | . | . | . | . |
| 21-225_27F2_HC | . | E | . | . | . | . | . | . | . | . | . | . | . | . | . | . | . |
| 21-225_27C3_HC | . | . | . | . | . | . | . | . | . | . | . | . | . | . | . | . | . |
| 21-225_29G8_HC | . | . | . | . | . | . | . | . | . | . | . | . | . | . | . | . | . |
| 21-225_6A11_HC | . | . | . | . | . | . | . | . | . | . | . | . | . | . | . | . | . |
| 21-225_6G12_HC | . | . | . | . | . | . | . | . | . | . | . | . | . | . | . | . | . |
| 21-225_8H7_HC | . | . | . | . | . | . | . | . | . | . | . | . | . | . | . | . | . |
| 21-225_10G5_HC | . | . | . | . | . | . | . | . | . | . | . | . | . | . | . | . | . |
| 21-225_10H12_HC | . | . | . | . | . | . | . | . | . | . | . | . | . | . | . | . | . |
| 21-225_12A1_HC | . | . | . | . | . | . | . | . | . | . | . | . | . | . | . | . | . |
| 21-225_6G2_HC | L | . | . | . | . | . | . | . | . | . | . | . | . | . | . | . | K |
| 21-225_7H11_HC | . | . | . | . | . | . | . | . | . | . | . | . | . | . | . | . | . |
| 21-225_14E3_HC | . | . | . | . | . | . | . | . | . | . | . | . | . | . | . | . | . |
| 21-225_9A1_HC | . | . | . | V | . | . | . | . | . | . | . | . | . | . | . | . | . |
| 21-225_15E3_HC | . | . | . | . | . | . | . | . | . | . | T | . | . | . | . | . | . |
| 21-225_16F4_HC | . | . | . | . | . | . | . | . | . | . | . | . | . | . | . | . | . |
| 21-225_16F6_HC | . | . | . | . | . | . | . | . | . | . | . | . | . | . | . | . | . |
| 21-225_2B2_HC | . | . | . | . | . | . | . | . | . | . | . | . | . | . | . | . | . |
| 21-225_13E6_HC | . | . | . | . | . | . | . | . | . | . | . | . | . | . | . | . | . |
| 21-225_16A5_HC | . | . | . | . | . | . | . | . | . | . | . | . | . | . | . | . | . |
| 21-225_7G2_HC | . | . | . | . | . | . | . | . | . | . | . | . | . | . | . | . | . |
| 21-225_7C4_HC | . | . | . | . | . | . | . | . | . | . | . | . | . | . | . | . | K |
| 21-225_7E9_HC | . | . | . | . | . | . | . | . | . | . | . | . | . | . | . | . | . |
| 21-225_8C9_HC | . | . | . | . | . | . | . | . | . | . | . | . | . | . | . | . | . |
| 21-225_12B12_HC | . | . | . | . | . | . | . | . | . | . | . | . | . | . | . | . | . |
| 21-225_16B7_HC | . | . | . | . | . | . | . | . | . | . | . | . | . | . | . | . | . |
| 21-225_9E8_HC | . | . | . | . | . | . | . | . | . | . | . | . | . | . | . | . | . |
| 21-225_12F2_HC | . | . | . | . | . | . | . | . | . | . | . | . | . | . | . | . | . |
| 21-225_12E6_HC | . | . | . | . | . | . | . | . | . | . | . | . | . | . | . | . | . |
| 21-225_16G7_HC | . | . | . | . | . | . | . | . | . | . | . | . | . | . | . | . | . |
| 21-225_1C12_HC | . | . | . | . | . | . | . | . | . | . | . | . | . | . | . | . | . |

FIGURE 56

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | S | L | R | L | S | C | A | A | S | G | - | F | T | F | S | D | - |
| 21-225_16F10_HC | . | . | . | . | . | . | . | . | . | . | . | . | . | . | . | . | . |
| 21-225_18A5_HC | . | . | . | . | . | Y | T | . | . | . | . | . | . | . | . | . | . |
| 21-225_20E5_HC | . | . | . | . | . | . | . | . | . | . | . | . | . | . | . | . | . |
| 21-225_18A1_HC | . | . | . | . | . | . | . | V | . | . | . | . | . | . | . | S | . |
| 21-225_19E1_HC | . | . | . | . | . | . | . | . | . | . | . | . | . | . | . | N | . |
| 21-225_17A10_HC | . | . | . | . | . | . | . | . | . | . | . | . | . | . | . | N | . |
| 21-225_18E8_HC | . | . | . | . | . | . | . | . | . | . | . | . | . | . | . | N | . |
| 21-225_16F11_HC | . | . | . | . | . | . | S | . | . | . | . | . | . | . | . | S | . |
| 21-225_18C2_HC | . | . | . | . | . | . | V | . | . | . | . | . | . | . | . | . | . |
| 21-225_20A7_HC | . | . | . | . | . | . | . | . | . | . | . | . | . | . | . | N | . |
| 21-225_17C7_HC | . | . | . | . | . | . | . | . | . | . | . | . | . | . | . | . | . |
| 21-225_18F2_HC | . | . | . | . | . | . | . | . | . | . | . | . | . | . | . | . | . |
| 21-225_17B5_HC | . | . | . | . | . | . | . | . | . | . | . | . | . | . | . | . | . |
| 21-225_17E5_HC | . | . | . | . | . | . | . | . | . | . | . | . | . | . | . | . | . |
| 21-225_20D5_HC | . | . | . | . | . | . | . | . | . | . | . | . | . | . | . | S | . |
| 21-225_21G11_HC | . | . | . | . | . | . | . | . | . | . | . | . | . | . | . | . | . |
| 21-225_20E12_HC | . | . | . | . | . | . | . | . | . | . | . | . | . | . | . | S | . |
| 21-225_20D10_HC | . | . | . | . | . | . | . | . | . | . | . | . | . | . | . | . | . |
| 21-225_22A4_HC | . | . | . | . | . | . | . | . | . | . | . | . | . | . | . | . | . |
| 21-225_20H12_HC | . | . | . | . | . | . | . | . | . | . | . | . | . | . | . | N | . |
| 21-225_21H8_HC | . | . | . | . | . | . | . | . | . | . | . | . | . | . | . | . | . |
| 21-225_21E5_HC | . | . | . | . | . | . | S | . | . | . | . | . | . | . | . | . | . |
| 21-225_22H8_HC | . | . | . | . | . | . | . | . | . | . | . | . | . | . | . | S | . |
| 21-225_23H11_HC | . | . | . | . | . | . | . | . | . | . | . | . | . | . | . | S | . |
| 21-225_21F7_HC | . | . | . | . | . | . | . | . | . | . | . | . | . | . | . | . | . |
| 21-225_22F9_HC | . | . | . | . | . | . | . | . | . | . | . | . | . | . | . | S | . |
| 21-225_21G2_HC | . | . | . | . | . | . | . | . | . | . | . | . | . | . | . | . | . |
| 21-225_22F2_HC | . | . | . | . | . | . | . | . | . | . | . | . | . | . | . | . | . |
| 21-225_23F12_HC | . | . | . | . | . | . | V | . | . | . | . | . | . | . | . | S | . |
| 21-225_28F5_HC | . | . | . | . | . | . | . | . | . | . | . | . | . | . | . | S | . |
| 21-225_29G4_HC | . | . | . | . | T | . | . | . | . | . | . | . | . | . | . | . | . |
| 21-225_27D5_HC | . | . | . | . | . | . | . | . | . | . | . | . | . | . | . | S | . |
| 21-225_28C7_HC | . | . | . | . | . | . | . | . | . | . | . | . | . | . | . | S | . |
| 21-225_25B6_HC | . | . | . | V | . | . | T | . | . | . | . | . | . | L | N | N | . |
| 21-225_26A2_HC | . | . | . | . | . | . | . | . | . | . | . | . | . | . | . | S | . |
| 21-225_29H6_HC | . | . | . | . | . | . | . | . | . | . | . | . | . | . | . | . | . |
| 21-225_25E6_HC | . | . | . | . | . | . | . | . | . | . | . | . | . | . | . | . | . |
| 21-225_25H10_HC | . | . | . | . | . | . | . | . | . | . | . | . | . | . | . | . | . |
| 21-225_29D7_HC | . | . | . | . | . | . | . | . | . | . | . | . | . | . | . | S | . |
| 21-225_29B8_HC | . | . | . | . | . | . | . | . | . | . | . | . | . | . | . | . | . |

FIGURE 56

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | S | L | R | L | S | C | A | A | S | G | - | F | T | F | S | D | - |
| 21-225_25H11_HC | . | . | . | . | . | . | . | . | . | . | . | . | . | . | . | . | . |
| 21-225_27F2_HC | . | . | . | . | . | . | . | . | . | . | . | . | I | . | . | . | . |
| 21-225_27C3_HC | . | . | . | . | . | . | . | . | . | . | . | . | . | . | . | . | . |
| 21-225_29G8_HC | . | . | . | . | . | . | . | . | . | . | . | . | . | . | . | . | . |
| 21-225_6A11_HC | . | . | . | . | . | . | . | . | . | . | . | . | . | . | . | . | . |
| 21-225_6G12_HC | . | . | . | . | . | . | . | . | . | . | . | . | . | . | . | S | . |
| 21-225_8H7_HC | . | . | . | . | . | . | P | . | . | . | . | . | . | . | . | . | . |
| 21-225_10G5_HC | . | . | . | . | . | . | . | . | . | . | . | . | . | . | . | N | . |
| 21-225_10H12_HC | . | . | . | . | . | . | . | . | . | . | . | . | . | . | . | . | . |
| 21-225_12A1_HC | . | . | . | . | . | . | . | . | . | . | . | . | . | . | . | S | . |
| 21-225_6G2_HC | . | . | . | . | . | . | . | . | . | . | . | . | . | . | . | . | . |
| 21-225_7H11_HC | . | . | . | . | . | . | S | . | . | . | . | . | . | . | . | . | . |
| 21-225_14E3_HC | . | . | . | . | . | . | . | . | . | . | . | . | . | . | . | . | . |
| 21-225_9A1_HC | . | . | . | . | . | . | . | . | . | . | . | . | . | . | . | . | . |
| 21-225_15E3_HC | . | . | . | . | . | . | . | . | . | . | . | . | . | . | . | S | . |
| 21-225_16F4_HC | . | . | . | . | . | . | . | . | . | . | . | . | . | . | . | N | . |
| 21-225_16F6_HC | . | . | . | . | . | . | . | . | . | . | . | . | . | . | . | N | . |
| 21-225_2B2_HC | . | . | . | . | . | . | . | . | . | . | . | . | . | . | . | S | . |
| 21-225_13E6_HC | . | . | . | . | . | . | . | . | . | . | . | . | . | . | . | N | . |
| 21-225_16A5_HC | . | . | . | . | . | . | . | . | . | . | . | . | . | . | . | . | . |
| 21-225_7G2_HC | . | . | . | . | . | . | . | . | . | . | . | . | . | . | . | . | . |
| 21-225_7C4_HC | . | . | . | . | . | . | . | . | . | . | . | . | . | . | . | N | . |
| 21-225_7E9_HC | . | . | . | . | . | . | . | . | . | . | . | . | . | . | . | . | . |
| 21-225_8C9_HC | . | . | . | . | . | . | . | . | . | . | . | . | . | . | . | S | . |
| 21-225_12B12_HC | . | . | . | . | . | . | . | . | . | . | . | . | . | . | . | . | . |
| 21-225_16B7_HC | . | . | . | . | . | . | . | . | . | . | . | . | . | . | . | S | . |
| 21-225_9E8_HC | . | . | . | . | . | . | E | . | . | . | . | . | . | . | . | S | . |
| 21-225_12F2_HC | . | . | . | . | . | . | . | . | . | . | . | . | . | . | . | . | . |
| 21-225_12E6_HC | . | . | . | . | . | . | . | . | . | . | . | . | . | . | . | . | . |
| 21-225_16G7_HC | . | . | . | . | . | . | . | . | . | . | . | . | . | . | T | . | . |
| 21-225_1C12_HC | . | . | . | . | . | . | . | . | . | . | . | . | . | . | . | N | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | H_CDR1 | | | | | | | | H_FR2 | | | | | | | | |
| CONSENSUS | - | - | - | - | Y | G | M | H | W | V | R | Q | A | P | G | K | G |
| 21-225_16F10_HC | . | . | . | . | . | . | . | . | . | . | . | . | . | . | . | . | . |
| 21-225_18A5_HC | . | . | . | . | . | . | . | . | . | . | . | . | . | . | . | . | . |
| 21-225_20E5_HC | . | . | . | . | . | . | . | . | . | . | . | . | . | . | . | . | . |
| 21-225_18A1_HC | . | . | . | . | . | . | . | . | . | . | . | . | . | . | . | . | . |
| 21-225_19E1_HC | . | . | . | . | . | . | . | . | . | . | . | . | . | . | . | . | . |
| 21-225_17A10_HC | . | . | . | . | . | . | . | . | . | . | . | . | . | . | A | . | . |
| 21-225_18E8_HC | . | . | . | . | . | . | . | . | . | . | . | . | . | . | . | . | . |
| 21-225_16F11_HC | . | . | . | . | . | . | . | . | . | . | . | . | . | . | . | . | . |
| 21-225_18C2_HC | . | . | . | . | . | . | . | . | . | . | . | . | . | . | . | . | . |
| 21-225_20A7_HC | . | . | . | . | . | . | . | . | . | . | . | . | . | . | . | . | . |
| 21-225_17C7_HC | . | . | . | . | . | . | . | . | . | . | . | . | . | . | . | . | . |
| 21-225_18F2_HC | . | . | . | . | . | . | . | . | . | . | . | . | . | . | . | . | . |
| 21-225_17B5_HC | . | . | . | . | . | . | . | . | . | . | . | . | . | . | . | . | . |
| 21-225_17E5_HC | . | . | . | . | . | . | . | . | . | . | . | . | . | . | . | . | . |
| 21-225_20D5_HC | . | . | . | . | . | . | . | . | . | . | . | . | . | . | . | . | . |
| 21-225_21G11_HC | . | . | . | . | . | . | . | . | . | . | . | . | . | . | . | . | . |
| 21-225_20E12_HC | . | . | . | . | . | . | . | . | . | . | . | . | . | . | . | . | . |
| 21-225_20D10_HC | . | . | . | . | . | . | . | . | . | . | . | . | . | . | . | . | . |
| 21-225_22A4_HC | . | . | . | . | . | . | I | . | . | . | . | . | . | . | . | . | . |
| 21-225_20H12_HC | . | . | . | . | . | . | . | . | . | . | . | . | . | . | . | . | . |
| 21-225_21H8_HC | . | . | . | . | . | . | . | . | . | . | . | . | . | . | . | . | . |
| 21-225_21E5_HC | . | . | . | . | . | . | . | . | . | . | . | . | . | . | . | . | . |
| 21-225_22H8_HC | . | . | . | . | . | . | . | . | . | . | . | . | . | . | . | . | . |
| 21-225_23H11_HC | . | . | . | . | . | . | . | . | . | . | . | . | . | . | . | . | . |
| 21-225_21F7_HC | . | . | . | . | . | . | . | . | . | . | . | . | . | . | . | . | . |
| 21-225_22F9_HC | . | . | . | . | . | . | . | . | . | . | . | . | . | . | . | . | . |
| 21-225_21G2_HC | . | . | . | . | . | . | . | . | . | . | . | . | . | . | . | . | . |
| 21-225_22F2_HC | . | . | . | . | . | . | . | . | . | . | . | . | . | . | . | . | . |
| 21-225_23F12_HC | . | . | . | . | . | . | . | . | . | . | . | . | . | . | . | . | . |
| 21-225_28F5_HC | . | . | . | . | . | . | . | . | . | . | . | . | . | . | . | . | . |
| 21-225_29G4_HC | . | . | . | . | . | . | I | . | . | . | . | . | . | . | . | . | . |
| 21-225_27D5_HC | . | . | . | . | . | . | . | . | . | . | . | . | . | . | . | . | . |
| 21-225_28C7_HC | . | . | . | . | . | . | . | . | . | . | . | . | . | . | . | . | . |
| 21-225_25B6_HC | . | . | . | . | . | . | . | . | . | . | . | . | . | . | . | . | . |
| 21-225_26A2_HC | . | . | . | . | . | . | . | . | . | . | . | . | . | . | . | . | . |
| 21-225_29H6_HC | . | . | . | . | . | . | . | . | . | . | . | . | . | . | . | . | . |
| 21-225_25E6_HC | . | . | . | . | . | . | . | . | . | . | . | . | . | . | . | . | . |
| 21-225_25H10_HC | . | . | . | . | . | . | . | . | . | . | . | . | . | . | . | . | . |
| 21-225_29D7_HC | . | . | . | . | . | . | . | . | . | . | . | . | . | . | . | . | . |
| 21-225_29B8_HC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | H_CDR1 | | | | | | | | H_FR2 | | | | | | | | |
| CONSENSUS | - | - | - | - | Y | G | M | H | W | V | R | Q | A | P | G | K | G |
| 21-225_25H11_HC | . | . | . | . | . | . | . | . | . | . | . | . | . | . | . | . | . |
| 21-225_27F2_HC | . | . | . | . | . | . | . | . | . | . | . | . | . | . | . | . | . |
| 21-225_27C3_HC | . | . | . | . | . | . | . | . | . | . | . | . | . | . | . | . | . |
| 21-225_29G8_HC | . | . | . | . | . | . | . | . | . | . | . | . | . | . | . | . | . |
| 21-225_6A11_HC | . | . | . | . | . | . | . | . | . | . | . | . | . | . | . | . | . |
| 21-225_6G12_HC | . | . | . | . | . | . | . | . | . | . | . | . | . | . | . | . | . |
| 21-225_8H7_HC | . | . | . | . | [illegible] | . | . | . | . | . | . | . | . | . | . | . | . |
| 21-225_10G5_HC | . | . | . | . | . | . | . | . | . | . | . | . | . | . | . | . | . |
| 21-225_10H12_HC | . | . | . | . | . | . | . | . | . | . | . | . | . | . | . | . | . |
| 21-225_12A1_HC | . | . | . | . | . | . | . | . | . | . | . | . | . | . | . | . | . |
| 21-225_6G2_HC | . | . | . | . | . | . | [illegible] | . | . | . | . | . | . | . | . | . | . |
| 21-225_7H11_HC | . | . | . | . | . | . | . | . | . | . | . | . | . | . | . | . | . |
| 21-225_14E3_HC | . | . | . | . | [illegible] | . | . | . | . | . | . | . | . | . | . | . | . |
| 21-225_9A1_HC | . | . | . | . | . | . | . | . | . | . | . | . | . | . | . | . | . |
| 21-225_15E3_HC | . | . | . | . | . | . | . | . | . | . | . | . | . | . | . | . | . |
| 21-225_16F4_HC | . | . | . | . | . | . | . | . | . | . | . | . | . | . | . | . | . |
| 21-225_16F6_HC | . | . | . | . | . | . | . | . | [illegible] | . | . | . | . | . | . | . | . |
| 21-225_2B2_HC | . | . | . | . | . | . | . | . | . | . | . | . | . | . | . | . | . |
| 21-225_13E6_HC | . | . | . | . | . | . | . | . | . | . | . | . | . | . | . | . | . |
| 21-225_16A5_HC | . | . | . | . | . | . | . | . | . | . | . | . | . | . | . | . | . |
| 21-225_7G2_HC | . | . | . | . | . | . | . | . | . | . | . | . | . | . | . | . | . |
| 21-225_7C4_HC | . | . | . | . | . | . | . | . | . | . | . | . | . | . | . | . | . |
| 21-225_7E9_HC | . | . | . | . | . | . | . | . | . | . | . | . | . | . | . | . | . |
| 21-225_8C9_HC | . | . | . | . | . | . | . | . | . | . | . | . | . | . | . | . | . |
| 21-225_12B12_HC | . | . | . | . | . | . | . | . | . | . | . | . | . | . | . | . | . |
| 21-225_16B7_HC | . | . | . | . | . | . | . | . | . | . | . | . | . | . | . | . | . |
| 21-225_9E8_HC | . | . | . | . | . | . | . | . | . | . | . | . | . | . | . | . | . |
| 21-225_12F2_HC | . | . | . | . | . | . | . | . | . | . | . | . | . | . | . | . | . |
| 21-225_12E6_HC | . | . | . | . | . | . | . | . | . | . | . | . | . | . | . | . | . |
| 21-225_16G7_HC | . | . | . | . | . | . | . | . | . | . | . | . | . | . | . | . | . |
| 21-225_1C12_HC | . | . | . | . | . | . | . | . | . | . | . | . | . | . | . | . | . |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | H_CDR2 | | |
| CONSENSUS | L | E | W | V | A | V | I | W | Y | D | - | - | - | E | N | N | K |
| 21-225_16F10_HC | . | . | . | . | . | . | . | . | . | . | . | . | . | G | . | . | . |
| 21-225_18A5_HC | . | . | . | . | . | . | . | . | F | . | . | . | . | G | S | . | . |
| 21-225_20E5_HC | . | . | . | . | . | . | . | . | . | E | . | . | . | . | . | . | Q |
| 21-225_18A1_HC | . | . | . | . | . | . | . | . | . | E | . | . | . | . | . | . | . |
| 21-225_19E1_HC | . | . | . | . | . | . | . | . | . | E | . | . | . | . | . | . | Q |
| 21-225_17A10_HC | . | . | . | . | . | . | . | . | . | E | . | . | . | . | . | . | Q |
| 21-225_18E8_HC | . | . | . | . | . | . | . | . | . | E | . | . | . | . | . | . | Q |
| 21-225_16F11_HC | . | . | . | . | . | . | . | . | . | E | . | . | . | . | . | . | . |
| 21-225_18C2_HC | . | . | . | M | . | . | . | . | . | . | . | . | . | V | T | . | . |
| 21-225_20A7_HC | . | . | . | . | . | . | . | . | . | E | . | . | . | . | . | . | Q |
| 21-225_17C7_HC | . | . | . | . | . | . | . | . | F | . | . | . | . | G | S | . | . |
| 21-225_18F2_HC | . | . | . | . | . | . | . | . | F | . | . | . | . | G | S | . | . |
| 21-225_17B5_HC | . | . | . | . | . | . | . | . | . | . | . | . | . | . | S | . | . |
| 21-225_17E5_HC | . | . | . | . | . | L | . | . | . | . | . | . | . | V | T | . | . |
| 21-225_20D5_HC | . | . | . | . | . | . | . | . | . | E | . | . | . | . | . | . | Q |
| 21-225_21G11_HC | . | . | . | M | . | . | . | . | . | . | . | . | . | V | T | . | E |
| 21-225_20E12_HC | . | . | . | . | . | . | M | . | . | . | . | . | . | . | S | . | . |
| 21-225_20D10_HC | . | . | . | . | . | . | . | . | F | . | . | . | . | G | S | . | . |
| 21-225_22A4_HC | . | . | . | . | . | . | . | . | F | . | . | . | . | G | S | . | . |
| 21-225_20H12_HC | . | . | . | . | . | . | . | . | . | . | . | . | . | . | . | . | . |
| 21-225_21H8_HC | . | . | . | . | . | . | . | . | F | . | . | . | . | G | S | . | . |
| 21-225_21E5_HC | . | . | . | . | . | . | . | . | . | . | . | . | . | . | S | . | . |
| 21-225_22H8_HC | . | . | . | . | . | . | . | . | . | . | . | . | . | G | . | . | . |
| 21-225_23H11_HC | . | . | . | . | . | . | . | . | . | . | . | . | . | . | . | . | . |
| 21-225_21F7_HC | . | . | . | . | . | . | . | . | F | . | . | . | . | G | . | . | . |
| 21-225_22F9_HC | . | . | . | . | . | . | . | . | . | E | . | . | . | . | . | . | . |
| 21-225_21G2_HC | . | . | . | M | . | . | . | . | . | . | . | . | . | V | T | . | . |
| 21-225_22F2_HC | . | . | . | . | . | . | . | . | . | E | . | . | . | G | S | . | . |
| 21-225_23F12_HC | . | . | . | . | . | . | . | . | . | . | . | . | . | . | T | . | . |
| 21-225_28F5_HC | . | . | . | . | . | . | . | . | . | . | . | . | . | . | . | . | . |
| 21-225_29G4_HC | . | . | . | . | . | . | . | . | . | . | . | . | . | . | S | . | . |
| 21-225_27D5_HC | . | . | . | . | . | . | . | . | . | . | . | . | . | . | S | . | . |
| 21-225_28C7_HC | . | . | . | . | . | . | . | . | . | . | . | . | . | . | S | . | . |
| 21-225_25B6_HC | . | . | . | . | . | . | . | . | . | E | . | . | . | . | S | . | . |
| 21-225_26A2_HC | . | . | . | . | . | . | . | . | . | E | . | . | . | G | S | . | . |
| 21-225_29H6_HC | . | . | . | . | T | . | . | . | . | N | . | . | . | . | . | . | . |
| 21-225_25E6_HC | . | . | . | . | . | . | . | . | . | . | . | . | . | . | . | . | . |
| 21-225_25H10_HC | . | . | . | . | . | . | . | . | . | . | . | . | . | . | S | . | . |
| 21-225_29D7_HC | . | . | . | . | . | . | . | . | . | . | . | . | . | . | S | . | . |
| 21-225_29B8_HC | . | . | . | . | . | . | . | . | . | . | . | . | . | . | . | . | . |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | H_CDR2 | | |
| CONSENSUS | L | E | W | V | A | V | I | W | Y | D | - | - | - | E | N | N | K |
| 21-225_25H11_HC | . | . | . | . | . | . | . | . | . | . | . | . | . | . | . | . | E |
| 21-225_27F2_HC | . | . | . | . | . | . | . | . | . | . | . | . | . | . | . | . | . |
| 21-225_27C3_HC | . | . | . | . | . | . | . | . | . | . | . | . | . | . | . | . | . |
| 21-225_29G8_HC | . | . | . | . | . | . | . | . | . | . | . | . | . | . | T | . | . |
| 21-225_6A11_HC | . | . | . | . | . | . | . | . | F | . | . | . | . | G | S | . | . |
| 21-225_6G12_HC | . | . | . | . | . | . | . | . | . | . | . | . | . | K | S | . | N |
| 21-225_8H7_HC | . | . | . | . | . | . | . | . | . | E | . | . | . | . | . | . | Q |
| 21-225_10G5_HC | . | . | . | . | . | . | . | . | . | E | . | . | . | . | . | . | Q |
| 21-225_10H12_HC | . | . | . | . | . | . | . | . | . | E | . | . | . | D | . | . | Q |
| 21-225_12A1_HC | . | . | . | . | . | . | . | . | . | E | . | . | . | . | . | . | Q |
| 21-225_6G2_HC | . | . | . | . | . | . | . | . | F | . | . | . | . | G | S | . | . |
| 21-225_7H11_HC | . | . | . | . | . | . | . | . | . | . | . | . | . | . | . | . | . |
| 21-225_14E3_HC | . | . | . | . | . | . | . | . | . | E | . | . | . | . | . | . | Q |
| 21-225_9A1_HC | . | . | . | . | . | . | . | . | F | . | . | . | . | G | S | . | . |
| 21-225_15E3_HC | . | . | . | . | . | . | . | . | . | E | . | . | . | . | . | . | . |
| 21-225_16F4_HC | . | . | . | . | . | . | . | . | . | E | . | . | . | G | S | . | Q |
| 21-225_16F6_HC | . | . | . | . | . | . | . | . | . | E | . | . | . | G | S | . | Q |
| 21-225_2B2_HC | . | . | . | . | . | . | . | . | . | E | . | . | . | . | . | . | . |
| 21-225_13E6_HC | . | . | . | . | . | . | . | . | . | . | . | . | . | . | . | . | . |
| 21-225_16A5_HC | . | . | . | . | . | . | . | . | F | . | . | . | . | G | S | . | . |
| 21-225_7G2_HC | . | . | . | M | . | . | . | . | . | . | . | . | . | V | T | . | . |
| 21-225_7C4_HC | . | . | . | . | . | . | . | . | . | E | . | . | . | . | . | . | Q |
| 21-225_7E9_HC | . | . | . | . | . | . | . | . | . | . | . | . | . | . | . | . | . |
| 21-225_8C9_HC | . | . | . | . | . | . | . | . | . | . | . | . | . | . | . | . | . |
| 21-225_12B12_HC | . | . | . | M | . | . | . | . | . | . | . | . | . | V | T | . | . |
| 21-225_16B7_HC | . | . | . | . | . | . | . | . | . | . | . | . | . | . | S | . | . |
| 21-225_9E8_HC | . | . | . | . | . | . | . | . | . | E | . | . | . | . | . | . | Q |
| 21-225_12F2_HC | . | . | . | . | . | . | . | . | F | . | . | . | . | G | . | . | . |
| 21-225_12E6_HC | . | . | . | . | T | . | . | . | . | . | . | . | . | . | S | . | . |
| 21-225_16G7_HC | . | . | . | . | . | . | . | . | . | . | . | . | . | . | . | . | E |
| 21-225_1C12_HC | . | . | . | . | . | . | . | . | . | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | A | D | S | V | K | G | R | F | T | I | S | R | D | N | S |
| 21-225_16F10_HC | . | . | V | . | . | . | . | . | . | . | . | . | . | . | . | . | . |
| 21-225_18A5_HC | . | . | . | . | . | . | . | . | . | . | . | . | . | . | . | . | . |
| 21-225_20E5_HC | . | . | . | . | . | . | . | . | . | . | . | . | . | . | . | . | . |
| 21-225_18A1_HC | . | . | V | . | . | . | . | . | . | . | . | . | . | . | . | . | . |
| 21-225_19E1_HC | . | . | . | . | . | . | . | . | . | . | . | . | . | . | . | . | . |
| 21-225_17A10_HC | . | . | . | . | . | . | . | . | . | . | . | . | . | . | . | . | . |
| 21-225_18E8_HC | . | . | . | . | . | . | . | . | . | . | . | V | . | . | . | . | . |
| 21-225_16F11_HC | . | . | V | . | . | . | . | . | . | . | . | . | . | . | . | . | . |
| 21-225_18C2_HC | . | . | . | . | . | . | . | . | . | . | . | . | . | . | . | . | . |
| 21-225_20A7_HC | . | . | . | . | . | . | . | . | . | . | . | . | . | . | . | . | . |
| 21-225_17C7_HC | . | . | . | . | . | . | . | . | . | . | . | . | . | . | . | . | . |
| 21-225_18F2_HC | . | . | V | . | . | . | . | . | . | . | . | . | . | . | . | . | . |
| 21-225_17B5_HC | N | . | I | . | . | . | . | . | . | . | . | . | . | . | . | . | . |
| 21-225_17E5_HC | . | . | G | . | . | . | . | . | . | . | . | . | . | . | . | . | . |
| 21-225_20D5_HC | . | . | . | . | . | . | . | . | . | . | . | . | . | . | . | . | . |
| 21-225_21G11_HC | . | . | . | . | . | . | . | . | . | . | . | . | . | . | . | . | . |
| 21-225_20E12_HC | N | . | . | . | . | . | . | . | . | . | . | . | . | . | . | . | . |
| 21-225_20D10_HC | . | . | V | . | . | . | . | . | . | . | . | . | . | . | . | D | . |
| 21-225_22A4_HC | . | . | . | . | . | . | . | . | . | I | . | . | . | . | . | . | . |
| 21-225_20H12_HC | . | . | V | . | . | . | . | . | . | . | . | . | . | . | . | . | . |
| 21-225_21H8_HC | . | . | . | . | . | . | . | . | . | . | . | . | . | . | . | . | . |
| 21-225_21E5_HC | . | . | . | . | . | . | . | . | . | . | . | . | . | . | . | . | . |
| 21-225_22H8_HC | . | . | . | . | . | . | . | . | . | . | . | . | . | . | . | . | . |
| 21-225_23H11_HC | . | . | V | . | . | . | . | . | . | . | . | . | . | . | . | . | . |
| 21-225_21F7_HC | . | . | V | . | . | . | . | . | . | . | . | . | . | . | . | . | . |
| 21-225_22F9_HC | D | . | V | . | . | . | . | . | . | . | . | . | . | . | . | . | . |
| 21-225_21G2_HC | . | . | . | . | . | . | . | . | . | . | . | . | . | . | . | . | . |
| 21-225_22F2_HC | . | . | V | . | . | . | R | . | . | . | . | . | . | . | . | . | . |
| 21-225_23F12_HC | . | . | . | . | . | . | . | . | . | . | . | . | . | . | . | . | . |
| 21-225_28F5_HC | . | . | . | . | . | . | . | . | . | . | . | . | . | . | . | . | . |
| 21-225_29G4_HC | . | . | . | . | . | M | . | . | . | . | . | . | . | . | . | . | . |
| 21-225_27D5_HC | . | . | G | . | . | . | . | . | . | . | . | . | . | . | . | . | . |
| 21-225_28C7_HC | . | . | . | . | . | . | . | . | . | . | . | . | . | . | . | . | . |
| 21-225_25B6_HC | . | . | T | E | . | . | . | . | . | . | . | . | . | . | . | . | . |
| 21-225_26A2_HC | . | . | V | . | . | . | . | . | . | . | . | . | . | . | . | . | . |
| 21-225_29H6_HC | . | . | . | . | . | . | . | . | . | . | . | . | . | . | . | . | . |
| 21-225_25E6_HC | . | . | . | . | . | . | . | . | . | . | . | . | . | . | . | . | . |
| 21-225_25H10_HC | . | . | . | . | . | M | . | . | . | . | . | . | . | . | . | . | . |
| 21-225_29D7_HC | . | . | G | . | . | . | . | . | . | . | . | . | . | . | . | . | . |
| 21-225_29B8_HC | . | . | . | . | . | . | . | . | . | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | A | D | S | V | K | G | R | F | T | I | S | R | D | N | S |
| 21-225_16F10_HC | . | . | V | . | . | . | . | . | . | . | . | . | . | . | . | . | . |
| 21-225_18A5_HC | . | . | . | . | . | . | . | . | . | . | . | . | . | . | . | . | . |
| 21-225_20E5_HC | . | . | . | . | . | . | . | . | . | . | . | . | . | . | . | . | . |
| 21-225_18A1_HC | . | . | V | . | . | . | . | . | . | . | . | . | . | . | . | . | . |
| 21-225_19E1_HC | . | . | . | . | . | . | . | . | . | . | . | . | . | . | . | . | . |
| 21-225_17A10_HC | . | . | . | . | . | . | . | . | . | . | . | . | . | . | . | . | . |
| 21-225_18E8_HC | . | . | . | . | . | . | . | . | . | . | . | V | . | . | . | . | . |
| 21-225_16F11_HC | . | . | V | . | . | . | . | . | . | . | . | . | . | . | . | . | . |
| 21-225_18C2_HC | . | . | . | . | . | . | . | . | . | . | . | . | . | . | . | . | . |
| 21-225_20A7_HC | . | . | . | . | . | . | . | . | . | . | . | . | . | . | . | . | . |
| 21-225_17C7_HC | . | . | . | . | . | . | . | . | . | . | . | . | . | . | . | . | . |
| 21-225_18F2_HC | . | . | V | . | . | . | . | . | . | . | . | . | . | . | . | . | . |
| 21-225_17B5_HC | H | . | I | . | . | . | . | . | . | . | . | . | . | . | . | . | . |
| 21-225_17E5_HC | . | . | S | . | . | . | . | . | . | . | . | . | . | . | . | . | . |
| 21-225_20D5_HC | . | . | . | . | . | . | . | . | . | . | . | . | . | . | . | . | . |
| 21-225_21G11_HC | . | . | . | . | . | . | . | . | . | . | . | . | . | . | . | . | . |
| 21-225_20E12_HC | H | . | . | . | . | . | . | . | . | . | . | . | . | . | . | . | . |
| 21-225_20D10_HC | . | . | V | . | . | . | . | . | . | . | . | . | . | . | . | D | . |
| 21-225_22A4_HC | . | . | . | . | . | . | . | . | . | I | . | . | . | . | . | . | . |
| 21-225_20H12_HC | . | . | V | . | . | . | . | . | . | . | . | . | . | . | . | . | . |
| 21-225_21H8_HC | . | . | . | . | . | . | . | . | . | . | . | . | . | . | . | . | . |
| 21-225_21E5_HC | . | . | . | . | . | . | . | . | . | . | . | . | . | . | . | . | . |
| 21-225_22H8_HC | . | . | . | . | . | . | . | . | . | . | . | . | . | . | . | . | . |
| 21-225_23H11_HC | . | . | V | . | . | . | . | . | . | . | . | . | . | . | . | . | . |
| 21-225_21F7_HC | . | . | V | . | . | . | . | . | . | . | . | . | . | . | . | . | . |
| 21-225_22F9_HC | D | . | V | . | . | . | . | . | . | . | . | . | . | . | . | . | . |
| 21-225_21G2_HC | . | . | . | . | . | . | . | . | . | . | . | . | . | . | . | . | . |
| 21-225_22F2_HC | . | . | V | . | . | . | R | . | . | . | . | . | . | . | . | . | . |
| 21-225_23F12_HC | . | . | . | . | . | . | . | . | . | . | . | . | . | . | . | . | . |
| 21-225_28F5_HC | . | . | . | . | . | . | . | . | . | . | . | . | . | . | . | . | . |
| 21-225_29G4_HC | . | . | . | . | . | M | . | . | . | . | . | . | . | . | . | . | . |
| 21-225_27D5_HC | . | . | S | . | . | . | . | . | . | . | . | . | . | . | . | . | . |
| 21-225_28C7_HC | . | . | . | . | . | . | . | . | . | . | . | . | . | . | . | . | . |
| 21-225_25B6_HC | . | . | T | E | . | . | . | . | . | . | . | . | . | . | . | . | . |
| 21-225_26A2_HC | . | . | V | . | . | . | . | . | . | . | . | . | . | . | . | . | . |
| 21-225_29H6_HC | . | . | . | . | . | . | . | . | . | . | . | . | . | . | . | . | . |
| 21-225_25E6_HC | . | . | . | . | . | . | . | . | . | . | . | . | . | . | . | . | . |
| 21-225_25H10_HC | . | . | . | . | . | M | . | . | . | . | . | . | . | . | . | . | . |
| 21-225_29D7_HC | . | . | S | . | . | . | . | . | . | . | . | . | . | . | . | . | . |
| 21-225_29B8_HC | . | . | . | . | . | . | . | . | . | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | A | D | S | V | K | G | R | F | T | I | S | R | D | N | S |
| 21-225_25H11_HC | . | . | . | . | . | . | . | . | . | . | . | . | . | . | . | . | . |
| 21-225_27F2_HC | . | . | V | . | . | . | R | . | . | . | . | . | . | . | . | . | . |
| 21-225_27C3_HC | S | . | . | . | . | . | . | . | . | . | . | . | . | . | . | . | . |
| 21-225_29G8_HC | . | . | . | . | . | . | . | . | . | . | . | . | . | . | . | . | . |
| 21-225_6A11_HC | . | . | . | . | . | . | . | . | . | . | . | . | . | . | . | . | . |
| 21-225_6G12_HC | . | . | . | . | . | . | . | . | . | . | . | . | . | . | . | . | . |
| 21-225_8H7_HC | . | . | . | . | . | . | . | . | . | . | . | . | . | . | . | . | . |
| 21-225_10G5_HC | . | . | . | . | . | . | . | . | . | . | . | . | . | . | . | . | . |
| 21-225_10H12_HC | . | . | . | . | . | . | . | . | . | . | . | . | . | . | . | . | . |
| 21-225_12A1_HC | . | . | . | . | . | . | . | . | . | . | . | . | . | . | . | . | . |
| 21-225_6G2_HC | . | . | . | . | . | . | . | . | . | . | . | . | . | S | . | . | . |
| 21-225_7H11_HC | . | . | . | . | . | . | . | . | . | . | . | . | . | . | . | . | . |
| 21-225_14E3_HC | . | . | . | . | . | . | . | . | . | . | . | . | . | . | . | . | . |
| 21-225_9A1_HC | . | . | . | . | . | . | . | . | . | . | . | . | . | . | . | . | . |
| 21-225_15E3_HC | H | . | . | . | . | . | . | . | . | . | . | . | . | . | . | . | . |
| 21-225_16F4_HC | . | . | G | . | . | . | . | . | . | . | . | . | . | . | . | . | . |
| 21-225_16F6_HC | . | . | G | . | . | . | . | . | . | . | . | . | . | . | . | . | . |
| 21-225_2B2_HC | . | . | V | . | . | . | . | . | . | . | . | . | . | . | . | . | . |
| 21-225_13E6_HC | . | . | I | . | . | . | . | . | . | . | . | . | . | . | . | . | . |
| 21-225_16A5_HC | . | . | V | . | . | . | . | . | . | . | . | . | . | . | . | . | . |
| 21-225_7G2_HC | . | . | . | . | . | . | . | . | . | . | . | . | . | . | . | . | . |
| 21-225_7C4_HC | . | . | . | . | . | . | . | . | . | . | . | . | . | . | . | . | . |
| 21-225_7E9_HC | . | . | V | . | . | . | . | . | . | . | . | . | . | . | . | . | . |
| 21-225_8C9_HC | . | . | V | . | . | . | . | . | . | . | . | . | . | . | . | . | . |
| 21-225_12B12_HC | . | . | . | . | . | . | . | . | . | . | . | . | . | . | . | . | . |
| 21-225_16B7_HC | . | . | . | . | . | . | . | . | . | . | . | . | . | . | . | . | . |
| 21-225_9E8_HC | . | . | . | . | . | . | . | . | . | . | . | . | . | . | . | . | . |
| 21-225_12F2_HC | . | . | V | . | . | . | . | . | . | . | . | . | . | . | . | . | . |
| 21-225_12E6_HC | . | . | . | . | . | . | . | . | . | . | . | . | . | . | . | D | . |
| 21-225_16G7_HC | . | . | . | . | . | . | . | . | . | . | . | . | . | . | A | . | . |
| 21-225_1C12_HC | . | . | V | . | . | . | . | . | . | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | A | D | S | V | K | G | R | F | T | I | S | R | D | N | S |
| 21-225_25H11_HC | . | . | . | . | . | . | . | . | . | . | . | . | . | . | . | . | . |
| 21-225_27F2_HC | . | . | V | . | . | . | R | . | . | . | . | . | . | . | . | . | . |
| 21-225_27C3_HC | S | . | . | . | . | . | . | . | . | . | . | . | . | . | . | . | . |
| 21-225_29G8_HC | . | . | . | . | . | . | . | . | . | . | . | . | . | . | . | . | . |
| 21-225_6A11_HC | . | . | . | . | . | . | . | . | . | . | . | . | . | . | . | . | . |
| 21-225_6G12_HC | . | . | . | . | . | . | . | . | . | . | . | . | . | . | . | . | . |
| 21-225_8H7_HC | . | . | . | . | . | . | . | . | . | . | . | . | . | . | . | . | . |
| 21-225_10G5_HC | . | . | . | . | . | . | . | . | . | . | . | . | . | . | . | . | . |
| 21-225_10H12_HC | . | . | . | . | . | . | . | . | . | . | . | . | . | . | . | . | . |
| 21-225_12A1_HC | . | . | . | . | . | . | . | . | . | . | . | . | . | . | . | . | . |
| 21-225_6G2_HC | . | . | . | . | . | . | . | . | . | . | . | . | . | S | . | . | . |
| 21-225_7H11_HC | . | . | . | . | . | . | . | . | . | . | . | . | . | . | . | . | . |
| 21-225_14E3_HC | . | . | . | . | . | . | . | . | . | . | . | . | . | . | . | . | . |
| 21-225_9A1_HC | . | . | . | . | . | . | . | . | . | . | . | . | . | . | . | . | . |
| 21-225_15E3_HC | H | . | . | . | . | . | . | . | . | . | . | . | . | . | . | . | . |
| 21-225_16F4_HC | . | . | G | . | . | . | . | . | . | . | . | . | . | . | . | . | . |
| 21-225_16F6_HC | . | . | G | . | . | . | . | . | . | . | . | . | . | . | . | . | . |
| 21-225_2B2_HC | . | . | V | . | . | . | . | . | . | . | . | . | . | . | . | . | . |
| 21-225_13E6_HC | . | . | I | . | . | . | . | . | . | . | . | . | . | . | . | . | . |
| 21-225_16A5_HC | . | . | V | . | . | . | . | . | . | . | . | . | . | . | . | . | . |
| 21-225_7G2_HC | . | . | . | . | . | . | . | . | . | . | . | . | . | . | . | . | . |
| 21-225_7C4_HC | . | . | . | . | . | . | . | . | . | . | . | . | . | . | . | . | . |
| 21-225_7E9_HC | . | . | V | . | . | . | . | . | . | . | . | . | . | . | . | . | . |
| 21-225_8C9_HC | . | . | V | . | . | . | . | . | . | . | . | . | . | . | . | . | . |
| 21-225_12B12_HC | . | . | . | . | . | . | . | . | . | . | . | . | . | . | . | . | . |
| 21-225_16B7_HC | . | . | . | . | . | . | . | . | . | . | . | . | . | . | . | . | . |
| 21-225_9E8_HC | . | . | . | . | . | . | . | . | . | . | . | . | . | . | . | . | . |
| 21-225_12F2_HC | . | . | V | . | . | . | . | . | . | . | . | . | . | . | . | . | . |
| 21-225_12E6_HC | . | . | . | . | . | . | . | . | . | . | . | . | . | . | . | D | . |
| 21-225_16G7_HC | . | . | . | . | . | . | . | . | . | . | . | . | . | . | A | . | . |
| 21-225_1C12_HC | . | . | V | . | . | . | . | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | A | R | E | L | G | F | - | - | - | - | - | - | - |
| 21-225_16F10_HC | . | . | . | . | . | . | . | . | A | W | . | . | . | . | . | . | . |
| 21-225_18A5_HC | . | . | . | . | . | . | D | . | . | W | . | . | . | . | . | . | . |
| 21-225_20E5_HC | . | . | . | . | . | . | . | . | . | . | . | . | . | . | . | . | . |
| 21-225_18A1_HC | . | . | . | . | V | . | . | . | . | . | . | . | . | . | . | . | . |
| 21-225_19E1_HC | . | . | . | . | . | . | . | . | . | . | . | . | . | . | . | . | . |
| 21-225_17A10_HC | . | . | . | . | . | . | . | . | . | . | . | . | . | . | . | . | . |
| 21-225_18E8_HC | . | . | . | . | . | . | . | . | . | . | . | . | . | . | . | . | . |
| 21-225_16F11_HC | . | . | . | . | V | . | . | . | . | . | . | . | . | . | . | . | . |
| 21-225_18C2_HC | . | . | . | . | . | . | . | . | . | W | . | . | . | . | . | . | . |
| 21-225_20A7_HC | I | . | . | . | . | . | . | . | . | . | . | . | . | . | . | . | . |
| 21-225_17C7_HC | . | . | . | . | . | . | D | . | . | W | . | . | . | . | . | . | . |
| 21-225_18F2_HC | . | . | . | . | . | . | D | . | . | W | . | . | . | . | . | . | . |
| 21-225_17B5_HC | . | . | . | . | V | . | . | . | . | . | . | . | . | . | . | . | . |
| 21-225_17E5_HC | . | . | . | . | . | . | . | . | . | W | . | . | . | . | . | . | . |
| 21-225_20D5_HC | . | . | . | . | . | . | . | . | . | . | . | . | . | . | . | . | . |
| 21-225_21G11_HC | . | . | . | . | . | . | . | . | . | W | . | . | . | . | . | . | . |
| 21-225_20E12_HC | . | . | . | . | . | . | . | . | . | . | . | . | . | . | . | . | . |
| 21-225_20D10_HC | . | . | . | . | . | . | D | . | . | W | . | . | . | . | . | . | . |
| 21-225_22A4_HC | . | . | . | . | . | . | D | . | . | W | . | . | . | . | . | . | . |
| 21-225_20H12_HC | . | . | . | . | . | . | . | . | . | . | . | . | . | . | . | . | . |
| 21-225_21H8_HC | . | . | . | . | . | . | D | . | . | W | . | . | . | . | . | . | . |
| 21-225_21E5_HC | M | . | . | . | T | . | . | . | . | . | . | . | . | . | . | . | . |
| 21-225_22H8_HC | . | . | . | . | . | . | . | . | A | W | . | . | . | . | . | . | . |
| 21-225_23H11_HC | . | . | . | . | . | . | . | . | . | . | . | . | . | . | . | . | . |
| 21-225_21F7_HC | . | . | . | . | . | . | D | . | . | W | . | . | . | . | . | . | . |
| 21-225_22F9_HC | . | . | . | . | V | . | . | . | . | . | . | . | . | . | . | . | . |
| 21-225_21G2_HC | . | . | . | . | . | . | . | . | . | W | . | . | . | . | . | . | . |
| 21-225_22F2_HC | . | . | . | . | V | . | . | . | . | . | . | . | . | . | . | . | . |
| 21-225_23F12_HC | I | . | . | . | . | . | . | . | A | W | . | . | . | . | . | . | . |
| 21-225_28F5_HC | . | . | . | . | . | . | . | . | . | W | . | . | . | . | . | . | . |
| 21-225_29G4_HC | . | . | . | . | . | . | . | . | . | M | . | . | . | . | . | . | . |
| 21-225_27D5_HC | L | . | . | . | . | . | . | . | . | . | . | . | . | . | . | . | . |
| 21-225_28C7_HC | . | . | . | . | . | . | . | . | . | W | . | . | . | . | . | . | . |
| 21-225_25B6_HC | . | . | . | . | . | . | . | . | . | . | . | . | . | . | . | . | . |
| 21-225_26A2_HC | . | . | . | . | . | . | . | . | . | W | . | . | . | . | . | . | . |
| 21-225_29H6_HC | . | . | . | . | . | . | . | . | . | M | . | . | . | . | . | . | . |
| 21-225_25E6_HC | L | . | . | . | . | T | . | . | . | M | . | . | . | . | . | . | . |
| 21-225_25H10_HC | . | . | . | . | . | . | . | . | . | M | . | . | . | . | . | . | . |
| 21-225_29D7_HC | L | . | . | . | . | . | . | . | . | . | . | . | . | . | . | . | . |
| 21-225_29B8_HC | . | . | . | . | . | . | . | . | . | M | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | A | R | E | L | G | F | - | - | - | - | - | - | - |
| 21-225_25H11_HC | . | . | . | . | . | . | . | . | . | M | . | . | . | . | . | . | . |
| 21-225_27F2_HC | . | . | . | . | . | . | . | . | . | M | . | . | . | . | . | . | . |
| 21-225_27C3_HC | . | . | . | . | . | . | . | . | . | M | . | . | . | . | . | . | . |
| 21-225_29G8_HC | . | . | . | . | . | . | . | . | . | M | . | . | . | . | . | . | . |
| 21-225_6A11_HC | . | . | D | . | . | . | D | . | . | W | . | . | . | . | . | . | . |
| 21-225_6G12_HC | . | . | . | . | . | . | . | . | . | . | . | . | . | . | . | . | . |
| 21-225_8H7_HC | . | . | . | . | . | . | . | . | . | . | . | . | . | . | . | . | . |
| 21-225_10G5_HC | . | . | . | . | T | . | . | . | . | . | . | . | . | . | . | . | . |
| 21-225_10H12_HC | . | . | . | . | . | . | . | . | . | . | . | . | . | . | . | . | . |
| 21-225_12A1_HC | . | . | . | . | . | . | . | . | . | . | . | . | . | . | . | . | . |
| 21-225_6G2_HC | . | . | . | . | . | . | D | . | . | W | . | . | . | . | . | . | . |
| 21-225_7H11_HC | . | . | . | . | . | . | . | . | . | . | . | . | . | . | . | . | . |
| 21-225_14E3_HC | F | . | . | . | . | . | . | . | . | . | . | . | . | . | . | . | . |
| 21-225_9A1_HC | . | . | . | . | . | . | D | . | . | W | . | . | . | . | . | . | . |
| 21-225_15E3_HC | . | . | . | . | . | . | . | . | . | . | . | . | . | . | . | . | . |
| 21-225_16F4_HC | . | . | . | . | . | . | . | . | . | . | . | . | . | . | . | . | . |
| 21-225_16F6_HC | . | . | . | . | . | . | . | . | . | . | . | . | . | . | . | . | . |
| 21-225_2B2_HC | . | . | . | . | V | . | . | . | . | . | . | . | . | . | . | . | . |
| 21-225_13E6_HC | . | . | . | . | . | . | . | . | . | . | . | . | . | . | . | . | . |
| 21-225_16A5_HC | . | . | . | . | . | . | D | . | . | W | . | . | . | . | . | . | . |
| 21-225_7G2_HC | . | . | . | . | . | . | . | . | . | W | . | . | . | . | . | . | . |
| 21-225_7C4_HC | . | . | . | . | . | . | . | . | . | . | . | . | . | . | . | . | . |
| 21-225_7E9_HC | . | . | . | . | . | . | . | . | . | . | . | . | . | . | . | . | . |
| 21-225_8C9_HC | . | . | . | . | . | . | . | . | . | . | . | . | . | . | . | . | . |
| 21-225_12B12_HC | . | . | . | . | . | . | . | . | . | W | . | . | . | . | . | . | . |
| 21-225_16B7_HC | . | . | . | . | . | . | . | . | . | . | . | . | . | . | . | . | . |
| 21-225_9E8_HC | . | . | . | . | . | . | . | . | . | . | . | . | . | . | . | . | . |
| 21-225_12F2_HC | . | . | . | . | V | . | . | . | A | W | . | . | . | . | . | . | . |
| 21-225_12E6_HC | . | . | . | . | . | . | . | . | A | W | . | . | . | . | . | . | . |
| 21-225_16G7_HC | . | . | . | . | . | . | . | . | A | W | . | . | . | . | . | . | . |
| 21-225_1C12_HC | . | . | . | . | . | . | . | . | . | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | H_CDR3 | | | | | | | | | | | | | | | | |
| CONSENSUS | - | - | - | - | - | - | - | - | - | - | - | - | - | - | - | R | S |
| 21-225_16F10_HC | . | . | . | . | . | . | . | . | . | . | . | . | . | . | . | Y | E |
| 21-225_18A5_HC | . | . | . | . | . | . | . | . | . | . | . | . | . | . | . | T | E |
| 21-225_20E5_HC | . | . | . | . | . | . | . | . | . | . | . | . | . | . | . | . | . |
| 21-225_18A1_HC | . | . | . | . | . | . | . | . | . | . | . | . | . | . | . | Q | . |
| 21-225_19E1_HC | . | . | . | . | . | . | . | . | . | . | . | . | . | . | . | . | . |
| 21-225_17A10_HC | . | . | . | . | . | . | . | . | . | . | . | . | . | . | . | . | . |
| 21-225_18E8_HC | . | . | . | . | . | . | . | . | . | . | . | . | . | . | . | . | . |
| 21-225_16F11_HC | . | . | . | . | . | . | . | . | . | . | . | . | . | . | . | Q | . |
| 21-225_18C2_HC | . | . | . | . | . | . | . | . | . | . | . | . | . | . | . | Y | E |
| 21-225_20A7_HC | . | . | . | . | . | . | . | . | . | . | . | . | . | . | . | . | . |
| 21-225_17C7_HC | . | . | . | . | . | . | . | . | . | . | . | . | . | . | . | T | E |
| 21-225_18F2_HC | . | . | . | . | . | . | . | . | . | . | . | . | . | . | . | T | E |
| 21-225_17B5_HC | . | . | . | . | . | . | . | . | . | . | . | . | . | . | . | . | F |
| 21-225_17E5_HC | . | . | . | . | . | . | . | . | . | . | . | . | . | . | . | Y | E |
| 21-225_20D5_HC | . | . | . | . | . | . | . | . | . | . | . | . | . | . | . | . | . |
| 21-225_21G11_HC | . | . | . | . | . | . | . | . | . | . | . | . | . | . | . | Y | E |
| 21-225_20E12_HC | . | . | . | . | . | . | . | . | . | . | . | . | . | . | . | . | F |
| 21-225_20D10_HC | . | . | . | . | . | . | . | . | . | . | . | . | . | . | . | T | E |
| 21-225_22A4_HC | . | . | . | . | . | . | . | . | . | . | . | . | . | . | . | T | E |
| 21-225_20H12_HC | . | . | . | . | . | . | . | . | . | . | . | . | . | . | . | . | . |
| 21-225_21H8_HC | . | . | . | . | . | . | . | . | . | . | . | . | . | . | . | T | E |
| 21-225_21E5_HC | . | . | . | . | . | . | . | . | . | . | . | . | . | . | . | . | . |
| 21-225_22H8_HC | . | . | . | . | . | . | . | . | . | . | . | . | . | . | . | Y | E |
| 21-225_23H11_HC | . | . | . | . | . | . | . | . | . | . | . | . | . | . | . | . | . |
| 21-225_21F7_HC | . | . | . | . | . | . | . | . | . | . | . | . | . | . | . | T | E |
| 21-225_22F9_HC | . | . | . | . | . | . | . | . | . | . | . | . | . | . | . | Q | . |
| 21-225_21G2_HC | . | . | . | . | . | . | . | . | . | . | . | . | . | . | . | Y | E |
| 21-225_22F2_HC | . | . | . | . | . | . | . | . | . | . | . | . | . | . | . | Q | . |
| 21-225_23F12_HC | . | . | . | . | . | . | . | . | . | . | . | . | . | . | . | Y | E |
| 21-225_28F5_HC | . | . | . | . | . | . | . | . | . | . | . | . | . | . | . | Y | D |
| 21-225_29G4_HC | . | . | . | . | . | . | . | . | . | . | . | . | . | . | . | T | G |
| 21-225_27D5_HC | . | . | . | . | . | . | . | . | . | . | . | . | . | . | . | L | . |
| 21-225_28C7_HC | . | . | . | . | . | . | . | . | . | . | . | . | . | . | . | Y | D |
| 21-225_25B6_HC | . | . | . | . | . | . | . | . | . | . | . | . | . | . | . | L | . |
| 21-225_26A2_HC | . | . | . | . | . | . | . | . | . | . | . | . | . | . | . | Y | D |
| 21-225_29H6_HC | . | . | . | . | . | . | . | . | . | . | . | . | . | . | . | T | G |
| 21-225_25E6_HC | . | . | . | . | . | . | . | . | . | . | . | . | . | . | . | T | G |
| 21-225_25H10_HC | . | . | . | . | . | . | . | . | . | . | . | . | . | . | . | T | G |
| 21-225_29D7_HC | . | . | . | . | . | . | . | . | . | . | . | . | . | . | . | L | . |
| 21-225_29B8_HC | . | . | . | . | . | . | . | . | . | . | . | . | . | . | . | T | G |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | H_CDR3 | | | | | | | | | | | | | | | | |
| CONSENSUS | - | - | - | - | - | - | - | - | - | - | - | - | - | - | - | R | S |
| 21-225_25H11_HC | . | . | . | . | . | . | . | . | . | . | . | . | . | . | . | T | G |
| 21-225_27F2_HC | . | . | . | . | . | . | . | . | . | . | . | . | . | . | . | T | G |
| 21-225_27C3_HC | . | . | . | . | . | . | . | . | . | . | . | . | . | . | . | T | G |
| 21-225_29G8_HC | . | . | . | . | . | . | . | . | . | . | . | . | . | . | . | T | . |
| 21-225_6A11_HC | . | . | . | . | . | . | . | . | . | . | . | . | . | . | . | T | E |
| 21-225_6G12_HC | . | . | . | . | . | . | . | . | . | . | . | . | . | . | . | . | . |
| 21-225_8H7_HC | . | . | . | . | . | . | . | . | . | . | . | . | . | . | . | . | . |
| 21-225_10G5_HC | . | . | . | . | . | . | . | . | . | . | . | . | . | . | . | . | . |
| 21-225_10H12_HC | . | . | . | . | . | . | . | . | . | . | . | . | . | . | . | . | . |
| 21-225_12A1_HC | . | . | . | . | . | . | . | . | . | . | . | . | . | . | . | . | . |
| 21-225_6G2_HC | . | . | . | . | . | . | . | . | . | . | . | . | . | . | . | T | E |
| 21-225_7H11_HC | . | . | . | . | . | . | . | . | . | . | . | . | . | . | . | . | . |
| 21-225_14E3_HC | . | . | . | . | . | . | . | . | . | . | . | . | . | . | . | . | . |
| 21-225_9A1_HC | . | . | . | . | . | . | . | . | . | . | . | . | . | . | . | T | E |
| 21-225_15E3_HC | . | . | . | . | . | . | . | . | . | . | . | . | . | . | . | L | . |
| 21-225_16F4_HC | . | . | . | . | . | . | . | . | . | . | . | . | . | . | . | L | . |
| 21-225_16F6_HC | . | . | . | . | . | . | . | . | . | . | . | . | . | . | . | L | . |
| 21-225_2B2_HC | . | . | . | . | . | . | . | . | . | . | . | . | . | . | . | Q | . |
| 21-225_13E6_HC | . | . | . | . | . | . | . | . | . | . | . | . | . | . | . | . | . |
| 21-225_16A5_HC | . | . | . | . | . | . | . | . | . | . | . | . | . | . | . | T | E |
| 21-225_7G2_HC | . | . | . | . | . | . | . | . | . | . | . | . | . | . | . | Y | E |
| 21-225_7C4_HC | . | . | . | . | . | . | . | . | . | . | . | . | . | . | . | . | . |
| 21-225_7E9_HC | . | . | . | . | . | . | . | . | . | . | . | . | . | . | . | . | . |
| 21-225_8C9_HC | . | . | . | . | . | . | . | . | . | . | . | . | . | . | . | . | . |
| 21-225_12B12_HC | . | . | . | . | . | . | . | . | . | . | . | . | . | . | . | Y | E |
| 21-225_16B7_HC | . | . | . | . | . | . | . | . | . | . | . | . | . | . | . | L | . |
| 21-225_9E8_HC | . | . | . | . | . | . | . | . | . | . | . | . | . | . | . | . | . |
| 21-225_12F2_HC | . | . | . | . | . | . | . | . | . | . | . | . | . | . | . | S | E |
| 21-225_12E6_HC | . | . | . | . | . | . | . | . | . | . | . | . | . | . | . | Y | E |
| 21-225_16G7_HC | . | . | . | . | . | . | . | . | . | . | . | . | . | . | . | Y | E |
| 21-225_1C12_HC | . | . | . | . | . | . | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | H_FR4 | | | | | | | | | | |
| CONSENSUS | D | Y | W | G | Q | G | T | L | V | T | V | S | S |
| 21-225_16F10_HC | . | . | . | . | . | . | . | . | . | . | . | . | . |
| 21-225_18A5_HC | E | . | . | . | . | . | . | . | . | . | . | . | . |
| 21-225_20E5_HC | . | . | . | . | . | . | . | . | . | . | . | . | . |
| 21-225_18A1_HC | . | . | . | . | . | . | . | P | . | . | . | . | . |
| 21-225_19E1_HC | . | . | . | . | . | . | . | . | . | . | . | . | . |
| 21-225_17A10_HC | . | . | . | . | . | . | . | . | . | . | . | . | . |
| 21-225_18E8_HC | . | . | . | . | . | . | . | . | . | . | . | . | . |
| 21-225_16F11_HC | . | . | . | . | . | . | . | P | . | . | . | . | . |
| 21-225_18C2_HC | . | . | . | . | . | . | . | . | . | . | . | . | . |
| 21-225_20A7_HC | . | . | . | . | . | . | . | . | . | . | . | . | . |
| 21-225_17C7_HC | E | . | . | . | . | . | . | . | . | . | . | . | . |
| 21-225_18F2_HC | E | . | . | . | . | . | . | . | . | . | . | . | . |
| 21-225_17B5_HC | . | . | . | . | . | . | . | . | . | . | . | . | . |
| 21-225_17E5_HC | . | . | . | . | . | . | . | . | . | . | . | . | . |
| 21-225_20D5_HC | . | . | . | . | . | . | . | . | . | . | . | . | . |
| 21-225_21G11_HC | . | . | . | . | . | . | . | . | . | . | . | . | . |
| 21-225_20E12_HC | . | . | . | . | . | . | . | . | . | . | . | . | . |
| 21-225_20D10_HC | E | . | . | . | . | . | . | . | . | . | . | . | . |
| 21-225_22A4_HC | E | . | . | . | . | . | . | . | . | . | . | . | . |
| 21-225_20H12_HC | . | . | . | . | . | . | . | . | . | . | . | . | . |
| 21-225_21H8_HC | E | . | . | . | . | . | . | . | . | . | . | . | . |
| 21-225_21E5_HC | . | . | . | . | . | . | . | . | . | . | . | . | . |
| 21-225_22H8_HC | . | . | . | . | . | . | . | . | . | . | . | . | . |
| 21-225_23H11_HC | . | . | . | . | . | . | . | . | . | . | . | . | . |
| 21-225_21F7_HC | E | . | . | . | . | . | . | . | . | . | . | . | . |
| 21-225_22F9_HC | . | . | . | . | . | . | . | P | . | . | . | . | . |
| 21-225_21G2_HC | . | . | . | . | . | . | . | . | . | . | . | . | . |
| 21-225_22F2_HC | . | . | . | . | . | . | . | P | . | . | . | . | . |
| 21-225_23F12_HC | . | . | . | . | . | . | S | . | . | . | . | . | . |
| 21-225_28F5_HC | . | . | . | . | . | . | . | . | . | . | . | . | . |
| 21-225_29G4_HC | . | . | . | . | . | . | . | . | . | . | . | . | . |
| 21-225_27D5_HC | . | . | . | . | . | . | . | . | . | . | . | . | . |
| 21-225_28C7_HC | . | . | . | . | . | . | . | . | . | . | . | . | . |
| 21-225_25B6_HC | . | . | . | . | . | . | . | . | . | . | . | . | . |
| 21-225_26A2_HC | . | . | . | . | . | . | . | . | . | . | . | . | . |
| 21-225_29H6_HC | . | S | . | . | . | . | . | . | . | . | . | . | . |
| 21-225_25E6_HC | . | S | . | . | . | . | . | . | . | . | . | . | . |
| 21-225_25H10_HC | . | . | . | . | . | . | . | . | . | . | . | . | . |
| 21-225_29D7_HC | . | . | . | . | . | . | . | . | . | . | . | . | . |
| 21-225_29B8_HC | . | S | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | H_FR4 | | | | | | | | | | | |
| CONSENSUS | D | Y | W | G | Q | G | T | L | V | T | V | S | S |
| 21-225_25H11_HC | . | S | . | . | . | . | . | . | . | . | . | . | . |
| 21-225_27F2_HC | . | S | . | . | . | . | . | . | . | . | . | . | . |
| 21-225_27C3_HC | . | S | . | . | . | . | . | . | . | . | . | . | . |
| 21-225_29G8_HC | . | . | . | . | . | . | . | . | . | . | . | . | . |
| 21-225_6A11_HC | [illegible] | . | . | . | . | . | . | . | . | . | . | . | . |
| 21-225_6G12_HC | . | . | . | . | . | . | . | . | . | . | . | . | . |
| 21-225_8H7_HC | . | . | . | . | . | . | . | . | . | . | . | . | . |
| 21-225_10G5_HC | . | . | . | . | . | . | . | . | . | . | . | . | . |
| 21-225_10H12_HC | . | . | . | . | . | . | . | . | . | . | . | . | . |
| 21-225_12A1_HC | . | . | . | . | . | . | . | . | . | . | . | . | . |
| 21-225_6G2_HC | [illegible] | . | . | . | . | . | . | . | . | . | . | . | . |
| 21-225_7H11_HC | . | . | . | . | . | . | . | . | . | . | . | . | . |
| 21-225_14E3_HC | . | . | . | . | . | . | . | . | . | . | . | . | . |
| 21-225_9A1_HC | [illegible] | . | . | . | . | . | . | [illegible] | . | . | . | . | . |
| 21-225_15E3_HC | . | . | . | . | . | . | . | . | . | . | . | . | . |
| 21-225_16F4_HC | . | . | . | . | . | . | . | . | . | . | . | . | . |
| 21-225_16F6_HC | . | . | . | . | . | . | . | . | . | . | . | . | . |
| 21-225_2B2_HC | . | . | . | . | . | . | . | [illegible] | . | . | . | . | . |
| 21-225_13E6_HC | . | . | . | . | . | . | . | . | . | . | . | . | . |
| 21-225_16A5_HC | [illegible] | . | . | . | . | . | . | . | . | . | . | . | . |
| 21-225_7G2_HC | . | . | . | . | . | . | . | . | . | . | . | . | . |
| 21-225_7C4_HC | . | . | . | . | . | . | . | . | . | . | . | . | . |
| 21-225_7E9_HC | . | . | . | . | . | . | . | . | . | . | . | . | . |
| 21-225_8C9_HC | . | . | . | . | . | . | . | . | . | . | . | . | . |
| 21-225_12B12_HC | . | . | . | . | . | . | . | . | . | . | . | . | . |
| 21-225_16B7_HC | . | . | . | . | . | . | . | . | . | . | . | . | . |
| 21-225_9E8_HC | . | . | . | . | . | . | . | . | . | . | . | . | . |
| 21-225_12F2_HC | . | . | . | . | . | . | . | . | . | . | . | . | . |
| 21-225_12E6_HC | . | . | . | . | . | . | . | . | . | . | . | . | . |
| 21-225_16G7_HC | . | . | . | . | . | . | . | . | . | . | . | . | . |
| 21-225_1C12_HC | . | . | . | . | . | . | . | . | . | . | . | . | . |

FIGURE 56

Table 33

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | H_FR1 | | | | | | | | | | | | | | | | |
| CONSENSUS | Q | L | Q | L | Q | E | S | - | G | P | G | L | V | K | P | S | E |
| 21-225_20C2_HC | . | . | . | . | . | . | . | . | . | . | . | . | . | . | . | . | . |
| 21-225_18C6_HC | . | . | . | . | . | . | . | . | . | . | . | . | . | . | . | . | . |
| 21-225_18A3_HC | . | . | . | . | . | . | . | . | . | . | . | . | . | . | F | . | . |
| 21-225_22D12_HC | . | . | . | . | . | . | . | . | . | . | . | . | . | . | . | . | . |
| 21-225_23D1_HC | . | . | . | . | . | . | . | . | . | . | . | . | . | . | . | . | . |
| 21-225_24E5_HC | . | . | . | . | . | . | . | . | . | . | . | . | . | . | . | . | . |
| 21-225_22C1_HC | . | . | . | . | . | . | . | . | . | . | . | . | . | . | . | . | . |
| 21-225_20G9_HC | . | . | . | . | . | . | . | . | . | . | . | . | . | . | . | . | . |
| 21-225_21G7_HC | . | . | . | . | . | . | . | . | . | . | . | . | . | . | F | . | . |
| 21-225_22G9_HC | . | . | . | . | . | . | . | . | . | . | . | . | . | . | . | . | . |
| 21-225_34C4_HC | . | . | . | . | . | . | . | . | . | . | . | . | . | . | . | . | . |
| 21-225_2F7_HC | . | . | . | . | . | . | . | . | . | . | . | . | . | . | . | . | . |
| 21-225_3G4_HC | . | . | . | . | . | . | . | . | . | . | . | . | . | . | . | . | . |
| 21-225_7F4_HC | . | . | . | . | . | . | . | . | . | . | . | . | . | . | . | . | . |
| 21-225_6D4_HC | . | . | . | . | . | . | . | . | . | . | . | . | . | . | . | . | . |
| 21-225_15A1_HC | . | . | H | . | . | . | . | . | . | . | . | . | . | . | . | . | . |
| 21-225_15G7_HC | . | . | . | . | . | . | . | . | . | . | . | . | . | . | . | . | . |
| 21-225_11F10_HC | . | . | H | . | . | . | . | . | . | . | . | . | . | . | . | . | . |
| 21-225_15H1_HC | . | . | . | . | . | . | . | . | . | . | . | . | . | . | F | . | . |
| 21-225_16A1_HC | . | . | . | . | . | . | . | . | . | . | . | . | . | . | . | . | . |
| 21-225_8D12_HC | . | . | . | . | . | . | . | . | . | . | . | . | . | . | F | . | . |

FIGURE 56

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | T | L | S | L | T | C | T | V | S | G | - | G | S | I | S | R | S |
| 21-225_20C2_HC | . | . | . | . | . | . | . | . | . | . | . | . | . | . | . | . | . |
| 21-225_18C6_HC | . | . | . | . | . | . | . | . | . | . | . | . | . | . | . | . | . |
| 21-225_18A3_HC | . | . | . | . | . | . | . | . | . | . | . | . | . | . | . | . | . |
| 21-225_22D12_HC | . | . | . | . | . | . | . | . | . | . | . | . | . | . | . | . | . |
| 21-225_23D1_HC | . | . | . | . | . | . | . | . | . | . | . | . | . | . | . | . | . |
| 21-225_24E5_HC | . | . | . | . | . | . | . | . | . | . | . | . | A | . | . | . | . |
| 21-225_22C1_HC | . | . | . | . | . | . | . | . | . | . | . | . | . | . | N | . | . |
| 21-225_20G9_HC | . | . | . | . | . | . | . | . | . | . | . | . | . | . | . | . | . |
| 21-225_21G7_HC | . | . | . | . | . | . | . | . | . | . | . | . | . | . | . | . | . |
| 21-225_22G9_HC | . | . | . | . | . | . | . | . | . | . | . | . | A | . | . | G | . |
| 21-225_34C4_HC | . | . | . | . | . | . | . | . | . | . | . | . | . | . | . | . | . |
| 21-225_2F7_HC | . | . | . | . | . | . | . | . | . | . | . | . | A | . | . | G | . |
| 21-225_3G4_HC | . | . | . | . | . | . | . | . | . | . | . | . | . | . | . | . | . |
| 21-225_7F4_HC | . | . | . | . | . | . | . | . | . | . | . | . | . | . | . | . | . |
| 21-225_6D4_HC | . | . | . | . | . | . | . | . | . | . | . | . | . | . | . | . | . |
| 21-225_15A1_HC | . | . | . | . | . | . | . | . | . | . | . | . | . | . | . | . | . |
| 21-225_15G7_HC | . | . | . | . | . | . | . | . | . | . | . | . | . | . | . | . | . |
| 21-225_11F10_HC | . | . | . | . | . | . | . | . | . | . | . | A | . | . | . | . | . |
| 21-225_15H1_HC | . | . | . | . | . | . | . | . | . | . | . | . | . | . | . | . | . |
| 21-225_16A1_HC | . | . | . | . | I | . | . | . | . | . | . | . | . | . | D | . | . |
| 21-225_8D12_HC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | H_CDR1 | | | | | | | | H_FR2 | | | | | | | | |
| CONSENSUS | - | - | - | S | Y | Y | W | G | W | I | R | Q | P | P | G | K | G |
| 21-225_20C2_HC | . | . | . | . | . | . | . | . | . | . | . | . | . | . | . | . | . |
| 21-225_18C6_HC | . | . | . | . | . | . | . | . | . | . | . | . | . | . | . | . | . |
| 21-225_18A3_HC | . | . | . | . | . | . | . | . | . | . | . | . | . | . | . | . | . |
| 21-225_22D12_HC | . | . | . | . | . | . | . | . | . | . | . | . | . | . | . | . | . |
| 21-225_23D1_HC | . | . | . | . | . | . | . | . | . | . | . | . | . | . | . | . | . |
| 21-225_24E5_HC | . | . | . | . | . | . | . | . | . | . | . | . | . | . | . | . | . |
| 21-225_22C1_HC | . | . | . | . | . | . | . | . | . | . | . | . | . | . | . | . | . |
| 21-225_20G9_HC | . | . | . | . | . | . | . | . | . | . | . | . | . | . | . | . | . |
| 21-225_21G7_HC | . | . | . | . | . | . | . | . | . | . | . | . | . | . | . | . | . |
| 21-225_22G9_HC | . | . | . | N | . | . | . | . | . | . | . | . | . | . | . | . | E |
| 21-225_34C4_HC | . | . | . | . | . | . | . | . | . | . | . | . | S | . | . | . | . |
| 21-225_2F7_HC | . | . | . | . | . | . | . | . | . | . | . | . | . | . | . | . | E |
| 21-225_3G4_HC | . | . | . | . | . | . | . | . | . | . | . | . | . | . | . | . | . |
| 21-225_7F4_HC | . | . | . | . | . | . | . | . | . | . | . | . | . | . | . | . | . |
| 21-225_6D4_HC | . | . | . | . | S | . | . | . | . | . | . | . | . | . | . | . | . |
| 21-225_15A1_HC | . | . | . | . | . | . | . | . | . | . | . | . | . | . | . | . | . |
| 21-225_15G7_HC | . | . | . | . | S | . | . | . | . | . | . | . | . | . | . | . | . |
| 21-225_11F10_HC | . | . | . | . | . | . | . | . | . | . | . | . | . | . | . | . | . |
| 21-225_15H1_HC | . | . | . | . | . | . | . | . | . | . | . | . | . | . | . | . | . |
| 21-225_16A1_HC | . | . | . | . | . | . | . | . | . | . | . | . | . | . | . | . | . |
| 21-225_8D12_HC | . | . | . | . | . | . | . | . | . | . | . | . | . | . | . | . | . |

**FIGURE 56**

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | | | H_CDR2 | |
| CONSENSUS | L | E | W | I | G | N | I | Y | Y | - | - | - | - | S | G | S | T |
| 21-225_20C2_HC | . | . | . | . | . | . | . | . | . | . | . | . | . | . | . | T | A |
| 21-225_18C6_HC | . | . | . | . | . | . | . | . | . | . | . | . | . | . | . | Y | . |
| 21-225_18A3_HC | . | . | . | . | . | . | . | . | . | . | . | . | . | . | . | Y | . |
| 21-225_22D12_HC | . | . | . | . | . | . | . | . | . | . | . | . | . | . | . | T | . |
| 21-225_23D1_HC | . | . | . | . | . | S | . | . | . | . | . | . | . | . | . | . | A |
| 21-225_24E5_HC | . | . | . | . | . | S | . | . | . | . | . | . | . | . | . | . | A |
| 21-225_22C1_HC | . | . | . | . | . | . | . | . | . | . | . | . | . | . | . | A | . |
| 21-225_20G9_HC | . | . | . | . | . | S | . | . | . | . | . | . | . | . | . | . | A |
| 21-225_21G7_HC | Q | . | . | . | . | . | . | . | . | . | . | . | . | . | . | Y | S |
| 21-225_22G9_HC | . | . | . | . | . | . | . | . | . | . | . | . | . | . | . | . | . |
| 21-225_34C4_HC | . | . | . | . | . | S | . | . | . | . | . | . | . | . | . | . | . |
| 21-225_2F7_HC | . | . | . | . | . | . | . | . | . | . | . | . | . | . | . | . | . |
| 21-225_3G4_HC | . | . | . | . | . | . | . | . | . | . | . | . | . | . | . | I | P |
| 21-225_7F4_HC | . | . | . | . | . | . | . | . | . | . | . | . | . | . | . | A | . |
| 21-225_6D4_HC | . | . | . | . | . | . | . | . | . | . | . | . | . | . | . | . | A |
| 21-225_15A1_HC | . | . | . | . | . | S | . | . | . | . | . | . | . | . | . | . | A |
| 21-225_15G7_HC | . | . | . | . | . | S | . | . | . | . | . | . | . | . | . | Y | . |
| 21-225_11F10_HC | . | . | . | . | . | S | . | . | . | . | . | . | . | . | . | . | A |
| 21-225_15H1_HC | . | . | . | . | . | . | . | . | . | . | . | . | . | . | . | Y | P |
| 21-225_16A1_HC | . | . | . | . | . | S | . | . | . | . | . | . | . | . | . | . | A |
| 21-225_8D12_HC | . | . | . | . | . | . | . | . | . | . | . | . | . | . | . | Y | P |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | N | P | S | L | K | S | R | V | T | I | S | V | D | T | S |
| 21-225_20C2_HC | . | C | . | S | . | . | . | . | . | . | I | . | . | . | . | . | . |
| 21-225_18C6_HC | . | . | . | . | . | . | . | . | . | . | I | . | . | . | . | . | . |
| 21-225_18A3_HC | . | . | T | . | . | . | . | . | . | . | . | . | . | . | . | . | . |
| 21-225_22D12_HC | S | . | . | . | . | . | . | . | . | . | . | . | . | . | . | . | . |
| 21-225_23D1_HC | Q | . | . | . | . | . | . | . | . | . | . | . | . | . | . | . | . |
| 21-225_24E5_HC | N | . | . | . | . | . | . | . | . | . | . | . | . | . | . | . | . |
| 21-225_22C1_HC | . | . | . | S | . | . | . | . | . | . | . | . | . | . | . | . | . |
| 21-225_20G9_HC | S | . | . | . | . | . | . | . | . | . | . | . | . | . | . | . | . |
| 21-225_21G7_HC | . | . | . | . | . | . | . | . | . | . | . | . | . | . | . | . | . |
| 21-225_22G9_HC | . | . | . | . | . | . | . | . | . | . | . | . | . | . | . | . | . |
| 21-225_34C4_HC | A | . | . | . | . | . | . | . | . | . | . | . | . | . | . | . | . |
| 21-225_2F7_HC | . | . | . | . | . | . | . | . | . | . | . | . | . | . | . | . | . |
| 21-225_3G4_HC | . | . | . | . | . | . | . | . | . | . | N | . | . | . | . | . | . |
| 21-225_7F4_HC | . | . | . | . | . | . | . | . | . | . | . | . | . | . | . | . | . |
| 21-225_6D4_HC | . | . | I | . | . | . | . | . | . | . | . | . | . | . | A | . | . |
| 21-225_15A1_HC | Q | . | . | . | . | . | . | . | . | . | . | . | . | . | . | . | T |
| 21-225_15G7_HC | . | . | T | . | . | . | . | . | . | . | . | . | . | . | . | . | . |
| 21-225_11F10_HC | Q | . | . | . | . | . | . | . | . | . | . | . | . | . | . | . | . |
| 21-225_15H1_HC | . | . | . | . | . | . | . | . | . | . | . | . | . | . | . | . | . |
| 21-225_16A1_HC | . | H | . | . | . | . | . | . | . | G | . | . | . | . | . | . | . |
| 21-225_8D12_HC | . | . | . | . | . | . | . | . | . | . | . | . | . | I | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | N | P | S | L | K | S | R | V | T | I | S | V | D | T | S |
| 21-225_20C2_HC | . | C | . | S | . | . | . | . | . | . | I | . | . | . | . | . | . |
| 21-225_18C6_HC | . | . | . | . | . | . | . | . | . | . | I | . | . | . | . | . | . |
| 21-225_18A3_HC | . | . | T | . | . | . | . | . | . | . | . | . | . | . | . | . | . |
| 21-225_22D12_HC | S | . | . | . | . | . | . | . | . | . | . | . | . | . | . | . | . |
| 21-225_23D1_HC | Q | . | . | . | . | . | . | . | . | . | . | . | . | . | . | . | . |
| 21-225_24E5_HC | N | . | . | . | . | . | . | . | . | . | . | . | . | . | . | . | . |
| 21-225_22C1_HC | . | . | . | S | . | . | . | . | . | . | . | . | . | . | . | . | . |
| 21-225_20G9_HC | S | . | . | . | . | . | . | . | . | . | . | . | . | . | . | . | . |
| 21-225_21G7_HC | . | . | . | . | . | . | . | . | . | . | . | . | . | . | . | . | . |
| 21-225_22G9_HC | . | . | . | . | . | . | . | . | . | . | . | . | . | . | . | . | . |
| 21-225_34C4_HC | A | . | . | . | . | . | . | . | . | . | . | . | . | . | . | . | . |
| 21-225_2F7_HC | . | . | . | . | . | . | . | . | . | . | . | . | . | . | . | . | . |
| 21-225_3G4_HC | . | . | . | . | . | . | . | . | . | . | N | . | . | . | . | . | . |
| 21-225_7F4_HC | . | . | . | . | . | . | . | . | . | . | . | . | . | . | . | . | . |
| 21-225_6D4_HC | . | . | I | . | . | . | . | . | . | . | . | . | . | . | A | . | . |
| 21-225_15A1_HC | Q | . | . | . | . | . | . | . | . | . | . | . | . | . | . | . | T |
| 21-225_15G7_HC | . | . | T | . | . | . | . | . | . | . | . | . | . | . | . | . | . |
| 21-225_11F10_HC | Q | . | . | . | . | . | . | . | . | . | . | . | . | . | . | . | . |
| 21-225_15H1_HC | . | . | . | . | . | . | . | . | . | . | . | . | . | . | . | . | . |
| 21-225_16A1_HC | . | H | . | . | . | . | . | . | . | G | . | . | . | . | . | . | . |
| 21-225_8D12_HC | . | . | . | . | . | . | . | . | . | . | . | . | . | I | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | A | R | H | S | S | S | W | - | - | - | - | - | - |
| 21-225_20C2_HC | . | . | . | . | . | . | . | . | . | . | . | . | . | . | . | . | . |
| 21-225_18C6_HC | L | . | . | . | . | . | . | . | . | . | . | . | . | . | . | . | . |
| 21-225_18A3_HC | . | . | . | . | . | . | . | . | T | . | . | . | . | . | . | . | . |
| 21-225_22D12_HC | . | F | . | . | . | . | L | . | . | . | . | . | . | . | . | . | . |
| 21-225_23D1_HC | L | . | . | . | . | . | L | . | . | . | . | . | . | . | . | . | . |
| 21-225_24E5_HC | . | . | . | . | . | . | L | . | . | . | . | . | . | . | . | . | . |
| 21-225_22C1_HC | . | . | . | . | . | . | . | . | G | . | . | . | . | . | . | . | . |
| 21-225_20G9_HC | A | . | . | . | . | . | L | . | . | . | . | . | . | . | . | . | . |
| 21-225_21G7_HC | . | . | . | . | . | . | . | . | T | . | . | . | . | . | . | . | . |
| 21-225_22G9_HC | . | . | . | . | . | . | . | . | . | . | . | . | . | . | . | . | . |
| 21-225_34C4_HC | . | . | . | . | . | . | L | . | . | . | . | . | . | . | . | . | . |
| 21-225_2F7_HC | . | . | . | . | . | . | . | . | T | . | . | . | . | . | . | . | . |
| 21-225_3G4_HC | . | . | . | . | . | . | . | . | . | . | . | . | . | . | . | . | . |
| 21-225_7F4_HC | . | . | . | . | . | . | . | . | G | . | . | . | . | . | . | . | . |
| 21-225_6D4_HC | . | . | . | . | . | . | . | . | . | . | . | . | . | . | . | . | . |
| 21-225_15A1_HC | . | . | . | . | . | . | L | . | . | . | . | . | . | . | . | . | . |
| 21-225_15G7_HC | S | . | . | . | . | . | L | . | . | . | . | . | . | . | . | . | . |
| 21-225_11F10_HC | . | . | . | . | . | . | L | . | . | . | . | . | . | . | . | . | . |
| 21-225_15H1_HC | . | . | . | . | . | . | . | . | T | . | . | . | . | . | . | . | . |
| 21-225_16A1_HC | A | . | . | . | . | . | L | . | . | . | . | . | . | . | . | . | . |
| 21-225_8D12_HC | . | . | . | . | . | . | . | . | T | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | H_CDR3 | | | | | | | | | | | | | | | | |
| CONSENSUS | - | - | - | - | - | - | - | - | - | - | - | - | - | - | - | S | L |
| 21-225_20C2_HC | . | . | . | . | . | . | . | . | . | . | . | . | . | . | . | . | . |
| 21-225_18C6_HC | . | . | . | . | . | . | . | . | . | . | . | . | . | . | . | . | . |
| 21-225_18A3_HC | . | . | . | . | . | . | . | . | . | . | . | . | . | . | . | . | . |
| 21-225_22D12_HC | . | . | . | . | . | . | . | . | . | . | . | . | . | . | . | . | [illegible] |
| 21-225_23D1_HC | . | . | . | . | . | . | . | . | . | . | . | . | . | . | . | . | [illegible] |
| 21-225_24E5_HC | . | . | . | . | . | . | . | . | . | . | . | . | . | . | . | . | [illegible] |
| 21-225_22C1_HC | . | . | . | . | . | . | . | . | . | . | . | . | . | . | . | . | . |
| 21-225_20G9_HC | . | . | . | . | . | . | . | . | . | . | . | . | . | . | . | . | [illegible] |
| 21-225_21G7_HC | . | . | . | . | . | . | . | . | . | . | . | . | . | . | . | . | . |
| 21-225_22G9_HC | . | . | . | . | . | . | . | . | . | . | . | . | . | . | . | . | . |
| 21-225_34C4_HC | . | . | . | . | . | . | . | . | . | . | . | . | . | . | . | . | [illegible] |
| 21-225_2F7_HC | . | . | . | . | . | . | . | . | . | . | . | . | . | . | . | . | |
| 21-225_3G4_HC | . | . | . | . | . | . | . | . | . | . | . | . | . | . | . | . | . |
| 21-225_7F4_HC | . | . | . | . | . | . | . | . | . | . | . | . | . | . | . | . | . |
| 21-225_6D4_HC | . | . | . | . | . | . | . | . | . | . | . | . | . | . | . | . | . |
| 21-225_15A1_HC | . | . | . | . | . | . | . | . | . | . | . | . | . | . | . | . | [illegible] |
| 21-225_15G7_HC | . | . | . | . | . | . | . | . | . | . | . | . | . | . | . | . | [illegible] |
| 21-225_11F10_HC | . | . | . | . | . | . | . | . | . | . | . | . | . | . | . | . | [illegible] |
| 21-225_15H1_HC | . | . | . | . | . | . | . | . | . | . | . | . | . | . | . | . | . |
| 21-225_16A1_HC | . | . | . | . | . | . | . | . | . | . | . | . | . | . | . | . | [illegible] |
| 21-225_8D12_HC | . | . | . | . | . | . | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | H_FR4 | | | | | | | | | | |
| CONSENSUS | D | Y | W | G | Q | G | T | L | V | T | V | S | S |
| 21-225_20C2_HC | . | N | . | . | . | . | . | . | . | . | . | . | . |
| 21-225_18C6_HC | . | D | . | . | . | . | . | . | . | . | . | . | . |
| 21-225_18A3_HC | . | . | . | . | . | . | . | . | . | . | . | . | . |
| 21-225_22D12_HC | . | . | . | . | . | . | . | . | . | . | . | . | . |
| 21-225_23D1_HC | . | . | . | . | . | . | . | . | . | . | . | . | . |
| 21-225_24E5_HC | . | N | . | . | . | . | . | . | . | . | . | . | . |
| 21-225_22C1_HC | . | . | . | . | . | . | . | . | . | . | . | . | . |
| 21-225_20G9_HC | . | . | . | . | . | . | . | . | . | . | . | . | . |
| 21-225_21G7_HC | . | . | . | . | . | . | . | . | . | . | . | . | . |
| 21-225_22G9_HC | . | . | . | . | . | . | . | . | . | . | . | . | . |
| 21-225_34C4_HC | . | . | . | . | . | . | . | . | . | . | . | . | . |
| 21-225_2F7_HC | . | . | . | . | . | . | . | . | . | . | . | . | . |
| 21-225_3G4_HC | . | . | . | . | . | . | . | . | . | . | . | . | . |
| 21-225_7F4_HC | . | . | . | . | . | . | . | . | . | . | . | . | . |
| 21-225_6D4_HC | . | C | . | . | . | . | . | . | . | . | . | . | . |
| 21-225_15A1_HC | . | . | . | . | . | . | . | . | . | . | . | . | . |
| 21-225_15G7_HC | . | F | . | . | . | . | . | . | . | . | . | . | . |
| 21-225_11F10_HC | . | . | . | . | . | . | . | . | . | . | . | . | . |
| 21-225_15H1_HC | . | . | . | . | . | . | . | . | . | . | . | . | . |
| 21-225_16A1_HC | . | . | . | . | . | . | . | . | . | . | . | . | . |
| 21-225_8D12_HC | . | . | . | . | . | . | . | . | . | . | . | . | . |

**FIGURE 56**

**Table 34**

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | | | | | H_FR1 | |
| CONSENSUS | Q | L | Q | L | Q | E | S | - | G | P | G | L | V | K | P | S | E |
| 21-225_17H8_HC | . | . | . | . | . | . | . | . | . | . | . | . | . | . | . | . | . |
| 21-225_17F9_HC | . | . | . | . | . | . | . | . | . | . | . | . | . | . | . | . | . |
| 21-225_1F2_HC | . | . | . | . | . | . | . | . | . | . | . | . | . | . | . | . | . |
| 21-225_21H3_HC | . | . | . | . | . | . | . | . | . | . | . | . | . | . | . | . | . |
| 21-225_23C8_HC | . | . | . | . | . | . | . | . | . | . | . | . | . | . | . | . | . |
| 21-225_22G8_HC | . | . | . | . | . | . | . | . | . | . | . | . | . | . | . | . | . |
| 21-225_22H4_HC | . | . | . | . | . | . | . | . | . | . | . | . | . | . | . | . | . |
| 21-225_23A3_HC | . | . | . | . | . | . | . | . | . | . | . | . | . | . | . | . | . |
| 21-225_5H7_HC | . | . | . | . | . | . | . | . | . | . | . | . | . | . | . | . | . |
| 21-225_14B2_HC | . | . | . | . | . | . | . | . | . | . | . | . | . | . | . | . | . |
| 21-225_4F4_HC | . | . | . | . | . | . | . | . | . | . | . | . | . | R | . | . | . |
| 21-225_4H4_HC | . | . | . | . | . | . | . | . | . | . | . | . | . | . | . | . | . |
| 21-225_11F5_HC | . | . | . | . | . | . | . | . | . | . | . | . | . | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| CONSENSUS | T | L | S | L | T | C | T | V | S | G | - | G | S | I | S | R | S |
| 21-225_17H8_HC | . | . | . | . | . | . | . | . | . | . | . | . | . | . | . | . | . |
| 21-225_17F9_HC | . | . | . | . | . | . | . | . | . | . | . | . | . | . | . | . | . |
| 21-225_1F2_HC | . | . | . | . | . | . | . | . | . | . | . | . | . | . | . | . | . |
| 21-225_21H3_HC | . | . | . | . | . | . | . | . | . | . | . | . | . | . | . | . | . |
| 21-225_23C8_HC | . | . | . | . | . | . | . | . | . | . | . | . | . | . | . | . | . |
| 21-225_22G8_HC | . | . | . | . | . | . | . | . | . | . | . | . | . | . | N | . | . |
| 21-225_22H4_HC | . | . | . | . | . | . | . | . | . | . | . | . | . | . | . | . | . |
| 21-225_23A3_HC | . | . | . | . | . | . | . | . | . | . | . | . | . | . | . | . | . |
| 21-225_5H7_HC | . | . | . | . | . | . | . | . | . | . | . | . | . | . | N | . | . |
| 21-225_14B2_HC | . | . | . | . | . | . | . | . | . | . | . | . | . | . | N | . | . |
| 21-225_4F4_HC | . | . | . | . | . | . | . | . | . | . | . | . | . | . | . | . | . |
| 21-225_4H4_HC | . | . | . | . | . | . | . | . | . | . | . | . | . | . | . | . | . |
| 21-225_11F5_HC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | H_CDR1 | | | | | | | | H_FR2 | | | | | | | | |
| CONSENSUS | - | - | - | S | Y | Y | W | G | W | I | R | Q | P | P | G | K | G |
| 21-225_17H8_HC | . | . | . | . | . | . | . | . | . | . | . | . | . | . | . | R | . |
| 21-225_17F9_HC | . | . | . | . | . | . | . | . | . | . | . | . | . | . | . | . | . |
| 21-225_1F2_HC | . | . | . | . | . | . | . | . | . | . | . | . | . | . | . | . | . |
| 21-225_21H3_HC | . | . | . | . | . | . | . | . | . | . | . | . | . | . | . | . | . |
| 21-225_23C8_HC | . | . | . | . | . | . | . | . | . | . | . | . | . | . | . | . | . |
| 21-225_22G8_HC | . | . | . | . | . | . | . | . | . | . | . | . | . | . | . | . | . |
| 21-225_22H4_HC | . | . | . | . | . | . | . | . | . | . | . | . | . | . | . | . | . |
| 21-225_23A3_HC | . | . | . | . | . | . | . | . | . | . | . | . | . | . | . | Q | . |
| 21-225_5H7_HC | . | . | . | . | . | . | . | . | . | . | . | . | . | . | . | . | . |
| 21-225_14B2_HC | . | . | . | . | . | . | . | . | . | . | . | . | . | . | . | . | . |
| 21-225_4F4_HC | . | . | . | . | . | . | . | . | . | . | . | . | . | . | . | . | . |
| 21-225_4H4_HC | . | . | . | . | . | . | . | . | . | . | . | . | . | . | . | M | . |
| 21-225_11F5_HC | . | . | . | . | . | . | . | . | . | . | . | . | . | . | . | . | . |

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | H_CDR2 | | | | | | | | | | | |
| CONSENSUS | L | E | W | I | G | N | I | Y | Y | - | - | - | - | S | G | S | T |
| 21-225_17H8_HC | . | . | . | . | . | . | . | . | . | . | . | . | . | G | . | N | . |
| 21-225_17F9_HC | . | . | . | . | . | . | . | . | . | . | . | . | . | . | . | . | . |
| 21-225_1F2_HC | . | . | . | . | . | . | . | . | . | . | . | . | . | . | . | . | A |
| 21-225_21H3_HC | . | . | . | . | . | . | . | . | . | . | . | . | . | . | . | . | . |
| 21-225_23C8_HC | . | . | . | . | . | . | . | . | . | . | . | . | . | . | . | T | . |
| 21-225_22G8_HC | . | D | . | . | . | . | . | . | . | . | . | . | . | . | . | . | . |
| 21-225_22H4_HC | . | . | . | . | . | . | . | . | . | . | . | . | . | . | . | . | . |
| 21-225_23A3_HC | . | . | . | . | . | . | . | . | . | . | . | . | . | . | . | . | . |
| 21-225_5H7_HC | . | . | . | . | . | . | . | . | . | . | . | . | . | . | . | T | . |
| 21-225_14B2_HC | . | . | . | . | . | . | . | . | . | . | . | . | . | . | . | T | . |
| 21-225_4F4_HC | . | . | . | . | . | . | . | . | . | . | . | . | . | . | . | . | . |
| 21-225_4H4_HC | . | . | . | . | . | . | . | . | . | . | . | . | . | . | . | N | . |
| 21-225_11F5_HC | . | . | . | . | . | . | . | . | . | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | Y | Y | N | P | S | L | K | S | R | V | T | I | S | V | D | T | S |
| 21-225_17H8_HC | . | . | . | . | . | . | . | . | . | . | . | . | . | . | . | . | . |
| 21-225_17F9_HC | . | . | . | . | . | . | . | . | . | . | . | . | . | . | . | S | . |
| 21-225_1F2_HC | . | N | . | . | . | . | . | G | . | . | . | . | . | . | . | . | . |
| 21-225_21H3_HC | . | D | . | . | . | . | . | . | . | . | . | . | . | . | . | . | . |
| 21-225_23C8_HC | . | N | . | . | . | . | . | . | . | . | . | . | . | . | . | . | . |
| 21-225_22G8_HC | . | . | . | . | . | V | . | . | . | F | . | . | . | . | . | . | . |
| 21-225_22H4_HC | . | H | . | . | . | . | . | . | . | . | . | - | . | . | . | . | . |
| 21-225_23A3_HC | . | N | . | . | . | . | . | . | . | . | S | . | . | . | . | . | . |
| 21-225_5H7_HC | . | . | . | . | . | . | . | . | . | . | . | . | . | . | . | . | . |
| 21-225_14B2_HC | . | . | . | . | . | . | . | . | . | . | . | . | . | . | . | . | . |
| 21-225_4F4_HC | . | D | . | . | . | . | . | . | . | . | . | . | . | . | . | . | . |
| 21-225_4H4_HC | . | N | . | . | . | . | . | . | . | . | . | . | . | . | . | . | . |
| 21-225_11F5_HC | . | T | . | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | H_FR3 | | | | | | | | | | | | | | | | |
| CONSENSUS | K | N | Q | F | S | L | K | L | S | S | V | T | A | A | D | T | A |
| 21-225_17H8_HC | . | . | . | . | . | . | . | . | . | . | . | . | . | . | . | . | . |
| 21-225_17F9_HC | . | . | . | . | . | . | N | . | N | . | . | . | . | . | . | . | . |
| 21-225_1F2_HC | . | . | . | . | . | . | . | . | N | . | . | . | . | . | . | . | . |
| 21-225_21H3_HC | . | . | . | . | . | . | . | . | . | . | . | . | . | . | . | . | . |
| 21-225_23C8_HC | . | . | H | . | . | . | . | . | . | . | . | . | . | . | . | . | . |
| 21-225_22G8_HC | . | . | . | . | . | . | . | . | . | . | . | . | . | . | . | . | . |
| 21-225_22H4_HC | M | . | . | . | . | . | . | . | T | . | . | . | . | . | . | . | . |
| 21-225_23A3_HC | . | . | . | . | . | . | N | . | . | . | . | . | . | . | . | . | . |
| 21-225_5H7_HC | . | . | . | . | . | . | N | . | . | . | . | . | . | . | . | . | . |
| 21-225_14B2_HC | . | . | . | . | . | . | N | . | . | . | . | . | . | . | . | . | . |
| 21-225_4F4_HC | . | . | . | . | . | . | . | . | . | . | . | . | . | . | . | . | . |
| 21-225_4H4_HC | . | . | . | . | . | . | . | . | N | . | . | . | . | . | . | . | . |
| 21-225_11F5_HC | . | . | H | . | . | . | . | . | . | . | . | . | . | . | . | . | . |

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | G | R | H | G | K | D | W | - | - | - | - | - | - |
| 21-225_17H8_HC | . | . | . | . | A | . | . | . | . | . | . | . | . | . | . | . | . |
| 21-225_17F9_HC | . | . | S | . | A | . | . | . | . | . | . | . | . | . | . | . | . |
| 21-225_1F2_HC | . | . | . | . | . | . | . | . | . | . | . | . | . | . | . | . | . |
| 21-225_21H3_HC | . | . | . | . | . | . | . | . | . | . | . | . | . | . | . | . | . |
| 21-225_23C8_HC | . | . | . | . | . | . | . | . | . | . | . | . | . | . | . | . | . |
| 21-225_22G8_HC | . | . | . | . | A | . | . | . | . | . | . | . | . | . | . | . | . |
| 21-225_22H4_HC | . | . | F | . | . | . | . | . | . | . | . | . | . | . | . | . | . |
| 21-225_23A3_HC | . | . | . | . | . | . | . | . | . | . | . | . | . | . | . | . | . |
| 21-225_5H7_HC | . | . | S | . | A | . | . | . | . | . | . | . | . | . | . | . | . |
| 21-225_14B2_HC | . | . | S | . | A | . | . | . | . | . | . | . | . | . | . | . | . |
| 21-225_4F4_HC | . | . | . | . | . | . | . | . | . | . | . | . | . | . | . | . | . |
| 21-225_4H4_HC | . | . | . | . | . | . | . | . | . | . | . | . | . | . | . | . | . |
| 21-225_11F5_HC | . | . | . | . | . | . | . | . | . | . | . | . | . | . | . | . | . |

H_CDR3

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| CONSENSUS | - | - | - | - | - | - | - | - | - | - | - | - | - | - | - | G | L |
| 21-225_17H8_HC | . | . | . | . | . | . | . | . | . | . | . | . | . | . | . | . | . |
| 21-225_17F9_HC | . | . | . | . | . | . | . | . | . | . | . | . | . | . | . | . | . |
| 21-225_1F2_HC | . | . | . | . | . | . | . | . | . | . | . | . | . | . | . | . | . |
| 21-225_21H3_HC | . | . | . | . | . | . | . | . | . | . | . | . | . | . | . | . | . |
| 21-225_23C8_HC | . | . | . | . | . | . | . | . | . | . | . | . | . | . | . | . | . |
| 21-225_22G8_HC | . | . | . | . | . | . | . | . | . | . | . | . | . | . | . | . | . |
| 21-225_22H4_HC | . | . | . | . | . | . | . | . | . | . | . | . | . | . | . | . | . |
| 21-225_23A3_HC | . | . | . | . | . | . | . | . | . | . | . | . | . | . | . | . | . |
| 21-225_5H7_HC | . | . | . | . | . | . | . | . | . | . | . | . | . | . | . | . | . |
| 21-225_14B2_HC | . | . | . | . | . | . | . | . | . | . | . | . | . | . | . | . | . |
| 21-225_4F4_HC | . | . | . | . | . | . | . | . | . | . | . | . | . | . | . | . | . |
| 21-225_4H4_HC | . | . | . | . | . | . | . | . | . | . | . | . | . | . | . | . | . |
| 21-225_11F5_HC | . | . | . | . | . | . | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | H_FR4 | | | | | | | | | | |
| CONSENSUS | D | Y | W | G | Q | G | T | L | V | T | V | S | S |
| 21-225_17H8_HC | . | . | . | . | . | . | . | . | . | . | . | . | . |
| 21-225_17F9_HC | . | . | . | . | . | . | . | . | . | . | . | . | . |
| 21-225_1F2_HC | . | . | . | . | . | . | . | . | . | . | . | . | . |
| 21-225_21H3_HC | . | . | . | . | . | . | . | . | . | . | . | . | . |
| 21-225_23C8_HC | . | . | . | . | . | . | . | . | . | . | . | . | . |
| 21-225_22G8_HC | . | . | . | . | . | . | . | . | . | . | . | . | . |
| 21-225_22H4_HC | . | . | . | . | . | . | . | . | . | . | . | . | . |
| 21-225_23A3_HC | . | F | . | . | . | . | . | . | . | . | . | . | . |
| 21-225_5H7_HC | . | . | . | . | . | . | . | . | . | . | . | . | . |
| 21-225_14B2_HC | . | . | . | . | . | . | A | . | . | . | . | . | . |
| 21-225_4F4_HC | . | . | . | . | . | . | . | . | . | . | . | . | . |
| 21-225_4H4_HC | . | . | . | . | . | . | . | . | . | I | . | . | . |
| 21-225_11F5_HC | . | N | . | . | . | . | . | . | . | . | . | . | . |

FIGURE 56

Table 35

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | K_FR1 | | | | | | | | | | | | | | | | |
| CONSENSUS | D | I | Q | M | T | Q | S | P | S | S | V | S | A | S | V | G | D |
| 21-225_30A11_LC | . | . | . | . | . | . | . | . | . | . | . | . | . | . | . | . | . |
| 21-225_34F3_LC | . | . | . | . | . | . | . | . | . | . | . | . | . | T | . | . | . |
| 21-225_35C6_LC | . | . | . | . | . | . | . | . | . | . | . | . | . | . | . | . | . |
| 21-225_36H5_LC | . | . | . | . | . | . | . | . | . | . | . | . | . | . | . | . | . |
| 21-225_35G6_LC | . | . | . | . | . | . | . | . | . | . | . | . | . | . | . | . | . |
| 21-225_33G1_LC | . | . | . | . | . | . | . | . | . | . | . | . | V | . | . | . | . |
| 21-225_34G7_LC | . | . | . | . | . | . | . | . | . | . | . | . | . | . | . | . | . |
| 21-225_33H7_LC | . | . | . | . | . | . | . | . | . | . | . | . | . | . | . | . | . |
| 21-225_35H12_LC | . | . | . | . | . | . | . | . | . | . | . | . | . | . | . | . | . |
| 21-225_4H6_LC | . | . | . | . | . | . | . | . | . | . | . | . | . | . | . | . | . |
| 21-225_18D4_LC | . | . | . | . | . | . | . | . | . | . | L | . | . | . | . | . | . |
| 21-225_23B11_LC | . | . | . | . | . | . | . | . | . | . | . | . | . | . | . | . | . |
| 21-225_31C4_LC | . | . | . | . | . | . | . | . | . | . | . | . | . | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | K_CDR1 | | | | | | | | | | |
| CONSENSUS | R | V | T | I | T | C | R | A | S | - | - | Q | G | I | S | - | - |
| 21-225_30A11_LC | . | . | . | . | . | . | . | . | . | . | . | . | . | . | . | . | . |
| 21-225_34F3_LC | . | . | . | S | . | . | . | . | . | . | . | . | . | . | . | . | . |
| 21-225_35C6_LC | . | . | . | . | . | . | . | . | . | . | . | . | . | . | . | . | . |
| 21-225_36H5_LC | . | . | . | . | . | . | . | . | . | . | . | . | . | . | . | . | . |
| 21-225_35G6_LC | . | L | . | . | . | . | . | . | . | . | . | . | . | . | . | . | . |
| 21-225_33G1_LC | . | . | . | . | . | . | . | . | . | . | . | . | . | . | . | . | . |
| 21-225_34G7_LC | . | . | . | . | . | . | . | . | . | . | . | . | L | . | . | . | . |
| 21-225_33H7_LC | . | . | . | . | . | . | . | . | . | . | . | . | . | . | . | . | . |
| 21-225_35H12_LC | . | . | . | . | . | . | . | . | . | . | . | . | . | . | . | . | . |
| 21-225_4H6_LC | . | . | . | . | . | . | . | . | . | . | . | . | . | . | . | . | . |
| 21-225_18D4_LC | . | . | . | . | . | . | . | . | . | . | . | . | . | . | . | . | . |
| 21-225_23B11_LC | . | . | . | . | . | . | . | . | . | . | . | . | . | . | T | . | . |
| 21-225_31C4_LC | . | . | . | . | . | . | . | . | . | . | . | . | . | . | . | . | . |

FIGURE 56

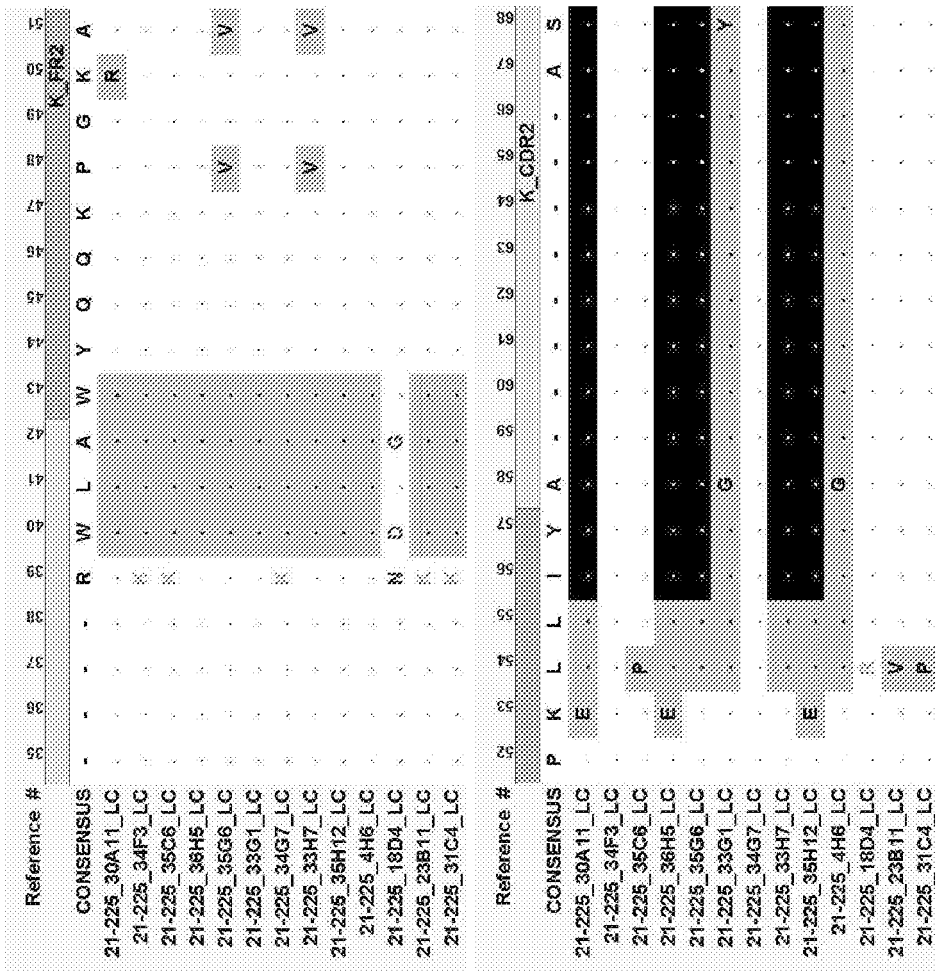

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | R | F | S | G | S | G | S | G | - |
| 21-225_30A11_LC | R | . | . | . | . | I | . | . | . | . | . | . | . | . | . | . | . |
| 21-225_34F3_LC | . | . | . | . | . | . | . | . | . | . | . | . | . | . | . | . | . |
| 21-225_35C6_LC | . | . | . | . | . | . | . | T | . | . | . | . | . | . | . | . | . |
| 21-225_36H5_LC | R | . | . | . | . | I | . | . | . | . | . | . | . | . | . | . | . |
| 21-225_35G6_LC | R | . | . | . | . | . | . | . | . | . | . | . | . | . | . | . | . |
| 21-225_33G1_LC | . | . | . | . | . | . | . | . | . | . | . | . | . | . | . | . | . |
| 21-225_34G7_LC | . | . | . | . | . | . | . | L | . | . | . | . | . | . | . | . | . |
| 21-225_33H7_LC | R | . | . | . | . | . | . | . | . | . | . | . | . | . | . | . | . |
| 21-225_35H12_LC | R | . | . | . | . | I | . | . | . | . | . | . | . | . | . | . | . |
| 21-225_4H6_LC | . | . | . | . | . | . | . | . | . | . | . | . | . | . | . | . | . |
| 21-225_18D4_LC | . | . | E | . | . | . | . | . | . | . | . | . | . | . | . | . | . |
| 21-225_23B11_LC | . | . | . | . | R | . | . | . | . | . | . | . | . | R | . | . | . |
| 21-225_31C4_LC | . | . | . | . | . | . | . | T | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | K_FR3 | | | | | | | | | | | | | |
| CONSENSUS | - | T | D | F | T | L | T | I | S | S | L | Q | P | E | D | F | A |
| 21-225_30A11_LC | . | . | . | . | . | . | . | . | . | . | . | . | . | . | . | . | . |
| 21-225_34F3_LC | . | . | . | . | . | . | . | . | . | . | . | . | . | . | . | S | . |
| 21-225_35C6_LC | . | . | . | . | . | . | . | . | . | . | . | . | . | . | . | . | . |
| 21-225_36H5_LC | . | . | . | . | . | . | . | . | . | . | . | . | . | . | . | . | . |
| 21-225_35G6_LC | . | . | A | . | . | . | . | . | G | . | . | . | . | . | . | . | . |
| 21-225_33G1_LC | . | . | . | . | . | . | . | . | . | . | . | . | . | . | . | . | . |
| 21-225_34G7_LC | . | . | . | . | . | . | . | . | . | . | . | . | . | . | . | . | . |
| 21-225_33H7_LC | . | . | . | . | . | . | . | . | G | . | . | . | . | . | . | . | . |
| 21-225_35H12_LC | . | . | . | . | . | . | . | . | . | . | . | . | . | . | . | . | . |
| 21-225_4H6_LC | . | . | . | . | . | . | . | . | . | . | . | . | . | . | . | . | . |
| 21-225_18D4_LC | . | . | E | . | . | . | . | V | . | . | . | . | . | . | . | . | . |
| 21-225_23B11_LC | . | . | . | . | . | . | . | . | . | . | . | . | . | . | . | . | . |
| 21-225_31C4_LC | . | . | . | . | . | . | . | . | . | . | . | . | . | . | . | . | . |

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | Q | Q | A | N | S | - | - | - | - | - | - | - | - |
| 21-225_30A11_LC | . | . | . | . | . | . | . | . | . | . | . | . | . | . | . | . | . |
| 21-225_34F3_LC | . | . | . | . | . | . | . | . | . | . | . | . | . | . | . | . | . |
| 21-225_35C6_LC | I | . | . | . | . | . | . | . | . | . | . | . | . | . | . | . | . |
| 21-225_36H5_LC | . | . | . | . | . | . | . | . | . | . | . | . | . | . | . | . | . |
| 21-225_35G6_LC | . | . | . | . | . | . | . | . | . | . | . | . | . | . | . | . | . |
| 21-225_33G1_LC | . | . | . | . | . | . | . | . | . | . | . | . | . | . | . | . | . |
| 21-225_34G7_LC | . | . | . | . | . | . | . | . | . | . | . | . | . | . | . | . | . |
| 21-225_33H7_LC | . | . | . | . | . | . | . | . | I | . | . | . | . | . | . | . | . |
| 21-225_35H12_LC | . | . | . | . | . | . | . | . | . | . | . | . | . | . | . | . | . |
| 21-225_4H6_LC | I | . | . | . | . | . | . | . | . | . | . | . | . | . | . | . | . |
| 21-225_18D4_LC | . | . | . | . | L | H | H | . | N | . | . | . | . | . | . | . | . |
| 21-225_23B11_LC | . | . | . | . | . | . | . | . | . | . | . | . | . | . | . | . | . |
| 21-225_31C4_LC | I | . | . | . | . | . | . | . | . | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | F | P |
| 21-225_30A11_LC | . | . | . | . | . | . | . | . | . | . | . | . | . | . | . | . | . |
| 21-225_34F3_LC | . | . | . | . | . | . | . | . | . | . | . | . | . | . | . | . | . |
| 21-225_35C6_LC | . | . | . | . | . | . | . | . | . | . | . | . | . | . | . | . | . |
| 21-225_36H5_LC | . | . | . | . | . | . | . | . | . | . | . | . | . | . | . | . | . |
| 21-225_35G6_LC | . | . | . | . | . | . | . | . | . | . | . | . | . | . | . | . | . |
| 21-225_33G1_LC | . | . | . | . | . | . | . | . | . | . | . | . | . | . | . | . | . |
| 21-225_34G7_LC | . | . | . | . | . | . | . | . | . | . | . | . | . | . | . | . | . |
| 21-225_33H7_LC | . | . | . | . | . | . | . | . | . | . | . | . | . | . | . | . | . |
| 21-225_35H12_LC | . | . | . | . | . | . | . | . | . | . | . | . | . | . | . | . | . |
| 21-225_4H6_LC | . | . | . | . | . | . | . | . | . | . | . | . | . | . | . | . | . |
| 21-225_18D4_LC | . | . | . | . | . | . | . | . | . | . | . | . | . | . | Y | L | . |
| 21-225_23B11_LC | . | . | . | . | . | . | . | . | . | . | . | . | . | . | . | . | . |
| 21-225_31C4_LC | . | . | . | . | . | . | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | K_FR4 | | | | | | | | | | |
| CONSENSUS | F | T | F | G | P | G | T | K | V | D | I | K | R |
| 21-225_30A11_LC | . | . | . | . | . | . | . | . | . | . | N | . | . |
| 21-225_34F3_LC | . | . | . | . | . | . | . | . | . | . | . | . | . |
| 21-225_35C6_LC | . | . | . | . | . | . | . | . | . | . | . | . | . |
| 21-225_36H5_LC | . | . | . | . | . | . | . | . | . | . | . | . | . |
| 21-225_35G6_LC | . | . | . | . | . | . | . | . | . | . | L | . | . |
| 21-225_33G1_LC | . | . | . | . | . | . | . | . | . | . | . | . | . |
| 21-225_34G7_LC | . | . | . | . | . | . | . | . | . | . | . | . | . |
| 21-225_33H7_LC | . | . | . | . | . | . | . | . | . | . | L | . | . |
| 21-225_35H12_LC | . | . | . | . | . | . | . | . | . | . | . | . | . |
| 21-225_4H6_LC | . | . | . | . | . | . | . | . | . | . | | | G |
| 21-225_18D4_LC | I | . | . | . | Q | . | . | R | L | E | . | . | . |
| 21-225_23B11_LC | . | . | . | . | . | . | . | . | . | . | . | . | . |
| 21-225_31C4_LC | . | . | . | . | . | . | . | . | . | . | . | . | . |

FIGURE 56

Table 36

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| Linear # | 1 | 2 | 3 | 4 | 5 | 6 | 7 | 8 | 9 | 10 | 11 | 12 | 13 | 14 | 15 | 16 | 17 |
| | K_FR1 | | | | | | | | | | | | | | | | |
| 21-225_31D12_LC | D | V | V | M | T | Q | S | P | L | S | L | P | V | T | L | G | Q |
| CONSENSUS | S | Y | E | L | T | Q | P | - | P | S | V | S | V | S | P | G | Q |
| 21-225_18E10_LC | . | . | . | . | . | . | . | . | . | . | . | . | . | . | . | . | . |
| 21-225_19D1_LC | . | . | . | . | . | . | . | . | . | . | . | . | . | . | . | . | . |
| 21-225_4B3_LC | . | . | . | . | . | . | . | . | . | . | . | . | . | . | . | R | . |
| 21-225_21D12_LC | . | . | . | . | . | . | . | . | . | . | . | . | . | . | . | . | . |
| 21-225_13F6_LC | . | . | . | . | . | . | . | . | . | . | . | . | . | . | . | . | . |
| 21-225_31D12_LC | . | . | . | . | . | . | . | . | . | . | . | . | . | . | . | . | . |
| 21-225_33E6_LC | . | . | . | . | . | . | . | . | . | . | . | . | . | . | . | . | . |
| 21-225_35E1_LC | . | . | . | . | . | . | . | . | . | . | . | . | . | . | . | . | . |
| 21-225_17D3_LC | . | . | . | . | . | . | S | . | . | . | . | . | . | . | . | . | . |
| 21-225_16F3_LC | . | . | V | . | . | . | . | . | . | . | . | . | . | A | . | . | . |
| 21-225_9G9_LC | Q | S | V | . | . | . | . | . | . | . | A | . | G | T | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| Linear # | 18 | 19 | 20 | 21 | 22 | 23 | 24 | 25 | 26 | 26.1 | 26.2 | 27 | 28 | 29 | 30 | 31 | 32 |
| | | | | | | | K_CDR1 | | | | | | | | | | |
| 21-225_31D12_LC | P | A | S | I | S | C | R | S | S | . | . | Q | S | L | I | Y | S |
| CONSENSUS | T | A | S | I | T | C | S | G | D | - | - | - | K | L | G | D | - |
| 21-225_18E10_LC | . | . | . | . | . | . | . | . | . | . | . | . | R | . | . | . | . |
| 21-225_19D1_LC | . | . | . | . | . | . | . | . | . | . | . | . | . | . | . | N | . |
| 21-225_4B3_LC | . | V | . | . | . | . | . | . | . | . | . | . | . | . | . | . | . |
| 21-225_21D12_LC | . | . | . | . | . | . | . | . | . | . | . | . | . | . | . | N | . |
| 21-225_13F6_LC | . | . | . | . | . | . | . | . | . | . | . | . | . | . | . | . | . |
| 21-225_31D12_LC | . | . | . | . | . | . | . | . | . | . | . | . | . | . | . | . | . |
| 21-225_33E6_LC | . | . | . | . | . | . | . | . | . | . | . | . | . | . | . | . | . |
| 21-225_35E1_LC | . | . | . | . | . | . | . | . | . | . | . | . | . | . | . | S | . |
| 21-225_17D3_LC | . | . | . | . | . | . | . | . | . | . | . | . | . | . | . | . | . |
| 21-225_16F3_LC | . | . | S | . | . | . | G | . | N | . | . | . | N | I | . | S | . |
| 21-225_9G9_LC | R | V | N | M | S | . | . | . | T | N | . | S | N | I | . | S | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| Linear # | 33 | 33.1 | 34 | 35 | 36 | 37 | 38 | 39 | 40 | 41 | 42 | 43 | 44 | 45 | 46 | 47 | 48 |
| | | | | | | | | | | | | | | | | K_FR2 | |
| 21-225_31D12_LC | Q | - | G | N | T | F | L | N | W | F | Q | Q | R | P | G | Q | S |
| CONSENSUS | - | - | - | - | K | Y | A | Y | W | Y | Q | Q | K | P | G | Q | S |
| 21-225_18E10_LC | . | . | . | . | . | . | . | W | . | . | . | . | . | . | . | . | . |
| 21-225_19D1_LC | . | . | . | . | . | . | . | . | . | . | . | . | . | . | . | . | . |
| 21-225_4B3_LC | . | . | . | . | . | . | . | C | . | . | . | . | . | . | . | . | . |
| 21-225_21D12_LC | . | . | . | . | . | . | . | . | . | . | . | . | . | . | . | . | . |
| 21-225_13F6_LC | . | . | . | . | . | . | . | C | . | . | . | . | . | . | . | . | . |
| 21-225_31D12_LC | . | . | . | . | . | . | . | . | . | F | . | . | . | . | . | . | . |
| 21-225_33E6_LC | . | . | . | . | . | . | . | . | . | F | . | . | . | . | . | . | . |
| 21-225_35E1_LC | . | . | . | . | . | . | . | . | . | F | . | . | . | . | . | . | . |
| 21-225_17D3_LC | . | . | . | . | . | . | V | . | . | . | . | . | . | . | . | . | . |
| 21-225_16F3_LC | . | . | . | . | . | S | V | H | . | . | . | . | . | . | . | . | A |
| 21-225_9G9_LC | . | . | . | . | Y | T | V | N | . | . | . | . | L | . | . | T | A |

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| Linear # | 49 | 50 | 51 | 52 | 53 | 54 | 55 | 55.1 | 55.2 | 55.3 | 55.4 | 55.5 | 55.6 | 55.7 | 55.8 | 56 | 57 |
| | | | | | | | | | | | | | K_CDR2 | | | | |
| 21-225_31D12_LC | P | R | R | L | I | Y | K | - | - | - | - | - | - | - | - | V | G |
| CONSENSUS | P | V | L | V | I | Y | Q | - | - | - | - | - | - | - | - | D | R |
| 21-225_18E10_LC | . | . | . | . | . | . | . | . | . | . | . | . | . | . | . | . | . |
| 21-225_19D1_LC | . | . | . | . | . | . | . | . | . | . | . | . | . | . | . | . | . |
| 21-225_4B3_LC | . | . | . | . | . | . | . | . | . | . | . | . | . | . | . | . | . |
| 21-225_21D12_LC | . | . | . | . | . | . | . | . | . | . | . | . | . | . | . | . | . |
| 21-225_13F6_LC | . | . | . | . | . | . | . | . | . | . | . | . | . | . | . | . | S |
| 21-225_31D12_LC | . | . | I | . | . | . | . | . | . | . | . | . | . | . | . | . | . |
| 21-225_33E6_LC | . | . | I | . | . | . | . | . | . | . | . | . | . | . | . | . | . |
| 21-225_35E1_LC | . | . | I | . | . | . | . | . | . | . | . | . | . | . | . | . | . |
| 21-225_17D3_LC | . | . | V | . | . | . | . | . | . | . | . | . | . | . | . | . | . |
| 21-225_16F3_LC | . | . | | | V | | D | . | . | . | . | . | . | . | . | . | T |
| 21-225_9G9_LC | . | K | | L | . | . | I | . | . | . | . | . | . | . | . | N | N |

**FIGURE 56**

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| Linear # | 58 | 59 | 60 | 61 | 62 | 63 | 64 | 65 | 66 | 67 | 68 | 69 | 70 | 71 | 72 | 73 | 73.1 |
| 21-225_31D12_LC | N | W | D | S | G | V | P | D | R | F | S | G | S | G | S | G | - |
| CONSENSUS | K | R | P | S | G | I | P | E | R | F | S | G | S | N | S | G | - |
| 21-225_18E10_LC | . | . | . | . | . | . | . | . | . | . | . | . | . | . | . | . | . |
| 21-225_19D1_LC | . | . | . | . | . | . | . | . | . | . | . | . | . | . | . | . | . |
| 21-225_4B3_LC | . | . | . | . | . | . | . | . | . | . | . | . | . | . | . | . | . |
| 21-225_21D12_LC | . | . | . | . | . | . | . | . | . | . | . | . | . | . | . | . | . |
| 21-225_13F6_LC | . | . | . | . | . | . | . | . | . | . | . | . | . | . | . | . | . |
| 21-225_31D12_LC | . | . | . | . | . | . | . | . | . | . | . | . | . | . | . | . | . |
| 21-225_33E6_LC | . | . | . | . | . | . | . | . | . | . | . | . | . | . | . | . | . |
| 21-225_35E1_LC | . | . | . | . | . | . | . | . | . | . | . | . | . | . | . | . | . |
| 21-225_17D3_LC | . | . | . | . | . | . | . | . | . | . | . | . | . | . | . | . | . |
| 21-225_16F3_LC | D | . | . | . | . | . | . | . | . | . | . | . | . | . | . | . | . |
| 21-225_9G9_LC | Q | . | . | . | . | V | . | D | . | . | . | . | . | K | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| Linear # | 73.2 | 74 | 75 | 76 | 77 | 78 | 79 | 80 | 81 | 82 | 83 | 84 | 85 | 86 | 87 | 88 | 89 |
| | | | | K_FR3 | | | | | | | | | | | | | |
| 21-225_31D12_LC | - | T | D | F | T | L | K | I | S | R | L | E | A | E | D | V | G |
| CONSENSUS | - | N | T | A | T | L | T | I | S | G | T | Q | A | M | D | E | A |
| 21-225_18E10_LC | . | . | . | . | . | . | . | . | . | . | . | . | . | . | . | . | . |
| 21-225_19D1_LC | . | . | . | . | . | . | . | . | . | . | . | . | . | . | . | . | . |
| 21-225_4B3_LC | . | . | . | . | . | . | . | . | . | . | . | . | . | . | . | . | . |
| 21-225_21D12_LC | . | . | . | . | . | . | . | . | . | . | . | . | . | . | . | . | . |
| 21-225_13F6_LC | . | . | . | . | . | . | . | . | . | . | . | . | . | . | . | . | . |
| 21-225_31D12_LC | . | . | . | . | . | . | . | . | . | . | . | . | . | . | . | . | . |
| 21-225_33E6_LC | . | . | . | . | . | . | . | . | . | . | . | . | . | . | . | . | . |
| 21-225_35E1_LC | . | . | . | . | . | . | . | . | . | . | . | . | . | . | . | . | . |
| 21-225_17D3_LC | . | . | . | . | . | . | . | . | . | . | . | . | . | . | . | . | . |
| 21-225_16F3_LC | . | . | . | . | . | . | . | . | . | R | V | E | . | G | . | . | . |
| 21-225_9G9_LC | . | T | S | . | S | . | A | . | . | . | L | . | S | E | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| Linear # | 90 | 91 | 92 | 93 | 94 | 95 | 96 | 97 | 98 | 98.1 | 98.2 | 98.3 | 98.4 | 98.5 | 98.6 | 98.7 | 98.8 |
| 21-225_31D12_LC | I | Y | Y | C | M | Q | G | T | N | . | . | . | . | . | . | . | . |
| CONSENSUS | D | Y | Y | C | Q | A | W | D | N | - | - | - | - | - | - | - | - |
| 21-225_18E10_LC | . | . | . | . | . | . | . | . | S | . | . | . | . | . | . | . | . |
| 21-225_19D1_LC | . | . | . | . | . | . | . | . | S | . | . | . | . | . | . | . | . |
| 21-225_4B3_LC | . | . | . | . | . | . | . | . | . | . | . | . | . | . | . | . | . |
| 21-225_21D12_LC | . | F | . | . | . | . | . | . | . | . | . | . | . | . | . | . | . |
| 21-225_13F6_LC | . | . | . | . | . | . | . | G | . | . | . | . | . | . | . | . | . |
| 21-225_31D12_LC | . | . | . | . | . | . | . | . | . | . | . | . | . | . | . | . | . |
| 21-225_33E6_LC | . | . | . | . | . | . | . | . | . | . | . | . | . | . | . | . | . |
| 21-225_35E1_LC | . | . | . | . | . | . | . | . | . | . | . | . | . | . | . | . | . |
| 21-225_17D3_LC | . | . | . | . | . | . | . | . | S | . | . | . | . | . | . | . | . |
| 21-225_16F3_LC | . | . | . | . | . | V | . | . | S | S | . | . | . | . | . | . | . |
| 21-225_9G9_LC | . | . | . | . | A | . | . | . | D | S | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| Linear # | 98.9 | 98.10 | 98.11 | 98.12 | 98.13 | 98.14 | 98.15 | 98.16 | 98.17 | 98.18 | 98.19 | 98.20 | 98.21 | 98.22 | 98.23 | 99 | 100 |
| | | K_CDR3 | | | | | | | | | | | | | | | |
| 21-225_31D12_LC | . | . | . | . | . | . | . | . | . | . | . | . | . | . | . | W | P |
| CONSENSUS | - | - | - | - | - | - | - | - | - | - | - | - | - | - | - | S | T |
| 21-225_18E10_LC | . | . | . | . | . | . | . | . | . | . | . | . | . | . | . | . | . |
| 21-225_19D1_LC | . | . | . | . | . | . | . | . | . | . | . | . | . | . | . | . | S |
| 21-225_4B3_LC | . | . | . | . | . | . | . | . | . | . | . | . | . | . | . | N | . |
| 21-225_21D12_LC | . | . | . | . | . | . | . | . | . | . | . | . | . | . | . | . | . |
| 21-225_13F6_LC | . | . | . | . | . | . | . | . | . | . | . | . | . | . | S | T | A |
| 21-225_31D12_LC | . | . | . | . | . | . | . | . | . | . | . | . | . | . | . | . | . |
| 21-225_33E6_LC | . | . | . | . | . | . | . | . | . | . | . | . | . | . | . | . | . |
| 21-225_35E1_LC | . | . | . | . | . | . | . | . | . | . | . | . | . | . | . | . | . |
| 21-225_17D3_LC | . | . | . | . | . | . | . | . | . | . | . | . | . | . | . | I | . |
| 21-225_16F3_LC | . | . | . | . | . | . | . | . | . | . | . | . | . | . | S | D | H |
| 21-225_9G9_LC | . | . | . | . | . | . | . | . | . | . | . | . | . | L | N | G | H |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| Linear # | 101 | 102 | 103 | 104 | 105 | 106 | 107 | 108 | 109 | 110 | 111 | 112 | 113 |
| | | | K_FR4 | | | | | | | | | | |
| 21-225_31D12_LC | L | T | F | G | G | G | T | R | L | E | I | K | R |
| CONSENSUS | V | V | F | G | G | G | T | K | L | T | V | L | G |
| 21-225_18E10_LC | . | . | . | . | . | . | . | . | . | . | . | . | . |
| 21-225_19D1_LC | . | . | . | . | . | . | . | . | . | . | . | . | . |
| 21-225_4B3_LC | . | I | . | . | . | . | . | . | . | . | . | . | . |
| 21-225_21D12_LC | . | . | . | . | . | . | . | R | . | . | . | . | . |
| 21-225_13F6_LC | . | . | . | . | . | . | . | . | . | . | . | . | . |
| 21-225_31D12_LC | A | . | . | . | . | . | . | . | . | . | . | . | . |
| 21-225_33E6_LC | A | . | . | . | . | . | . | . | . | . | . | . | . |
| 21-225_35E1_LC | A | . | . | . | . | . | . | . | . | . | . | . | . |
| 21-225_17D3_LC | A | . | . | . | . | . | . | . | . | . | . | R | . |
| 21-225_16F3_LC | . | . | . | . | . | . | . | . | . | . | . | . | . |
| 21-225_9G9_LC | . | . | . | . | . | R | . | . | . | . | . | . | . |

FIGURE 56

Table 37

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | K_FR1 | | | | | | | | | | | | | | | | |
| CONSENSUS | D | I | V | M | T | Q | S | P | D | S | L | A | V | S | L | G | E |
| 21-225_34H7_LC | . | . | . | . | . | . | . | . | . | . | . | . | . | . | . | . | . |
| 21-225_24E1_LC | . | . | . | . | . | . | . | . | . | . | . | . | . | . | . | . | . |
| 21-225_23A12_LC | . | . | . | . | . | . | . | . | . | . | . | . | . | . | . | . | . |
| 21-225_25A4_LC | . | . | . | . | . | . | P | . | . | . | . | . | . | . | . | . | . |
| 21-225_28B6_LC | . | V | . | . | . | . | . | . | L | . | . | P | . | T | . | . | Q |
| 21-225_29E6_LC | . | . | . | . | . | . | P | . | . | . | . | . | . | . | . | . | . |
| 21-225_26C4_LC | . | . | . | . | . | . | . | . | . | . | . | . | . | . | . | . | . |
| 21-225_33D1_LC | . | . | . | . | . | . | . | . | . | . | . | S | . | . | . | . | . |
| 21-225_34D2_LC | . | . | . | . | . | . | T | . | L | . | . | S | . | T | P | . | Q |
| 21-225_29H8_LC | . | . | . | . | . | . | . | . | . | . | . | . | . | . | . | . | . |
| 21-225_11E5_LC | . | . | . | . | . | . | . | . | . | . | . | . | . | . | . | . | . |
| 21-225_8B11_LC | . | . | . | . | . | . | . | . | . | . | . | . | . | . | . | . | . |
| 21-225_22D2_LC | . | . | . | . | . | . | . | . | . | . | . | . | . | . | . | . | . |
| 21-225_32A1_LC | . | . | . | . | . | . | . | . | . | . | . | . | . | . | . | . | . |
| 21-225_32A5_LC | . | . | . | . | . | . | . | . | . | . | . | . | . | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | K_CDR1 | | | | | | | | | | |
| CONSENSUS | R | A | T | I | N | C | K | S | S | - | - | Q | S | V | L | Y | S |
| 21-225_34H7_LC | . | . | . | . | . | . | . | . | . | . | . | . | . | . | . | . | . |
| 21-225_24E1_LC | . | . | . | . | . | . | . | . | . | . | . | . | . | . | . | . | T |
| 21-225_23A12_LC | . | . | . | . | . | . | . | . | . | . | . | . | . | . | . | . | . |
| 21-225_25A4_LC | . | . | . | . | K | . | . | . | . | . | . | . | . | . | . | . | . |
| 21-225_28B6_LC | P | . | S | . | S | . | R | . | . | . | . | . | . | L | V | . | . |
| 21-225_29E6_LC | . | . | . | . | . | . | . | . | . | . | . | . | . | . | . | . | . |
| 21-225_26C4_LC | . | . | . | . | . | . | . | . | . | . | . | . | . | . | . | . | R |
| 21-225_33D1_LC | . | . | . | . | . | . | . | . | I | . | . | . | . | . | . | . | R |
| 21-225_34D2_LC | P | . | S | . | S | . | . | . | . | . | . | . | . | L | . | H | . |
| 21-225_29H8_LC | . | . | . | . | . | . | R | . | . | . | . | . | T | I | . | H | . |
| 21-225_11E5_LC | . | . | . | L | . | . | . | . | . | . | . | . | . | . | . | . | . |
| 21-225_8B11_LC | . | . | . | . | . | . | . | . | . | . | . | . | N | . | . | . | N |
| 21-225_22D2_LC | . | . | . | . | T | . | . | . | . | . | . | . | . | . | . | H | . |
| 21-225_32A1_LC | . | . | . | . | . | . | . | . | . | . | . | . | . | . | . | . | . |
| 21-225_32A5_LC | . | . | . | . | . | . | . | . | . | . | . | . | . | . | . | H | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | | K_FR2 | |
| CONSENSUS | S | N | N | N | N | Y | L | A | W | Y | Q | Q | K | P | G | Q | P |
| 21-225_34H7_LC | . | . | . | Y | . | . | . | . | . | . | . | L | . | . | . | . | . |
| 21-225_24E1_LC | . | . | . | . | . | . | . | T | . | . | . | . | . | . | . | . | [illegible] |
| 21-225_23A12_LC | . | . | . | . | . | . | . | . | . | . | . | . | . | . | . | . | . |
| 21-225_25A4_LC | . | [illegible] | . | . | . | . | . | . | . | . | . | . | . | . | . | . | . |
| 21-225_28B6_LC | D | - | G | D | T | . | . | N | . | F | . | . | [illegible] | . | . | . | S |
| 21-225_29E6_LC | . | [illegible] | . | . | Y | . | . | T | . | . | . | . | . | . | . | . | . |
| 21-225_26C4_LC | . | . | . | Y | . | . | . | . | . | . | . | [illegible] | . | . | . | . | . |
| 21-225_33D1_LC | . | . | . | [illegible] | . | . | . | T | . | . | . | . | . | . | . | . | . |
| 21-225_34D2_LC | D | - | G | [illegible] | T | . | . | Y | . | . | L | . | . | . | . | . | . |
| 21-225_29H8_LC | . | . | . | Y | . | . | . | . | . | . | . | . | . | . | . | . | . |
| 21-225_11E5_LC | . | . | . | . | . | . | . | T | . | . | . | . | . | . | . | . | [illegible] |
| 21-225_8B11_LC | . | . | . | . | . | . | . | . | . | . | . | . | . | . | . | . | . |
| 21-225_22D2_LC | . | . | . | . | . | . | . | . | . | . | . | . | . | . | . | . | . |
| 21-225_32A1_LC | . | . | . | Y | . | . | . | . | . | . | . | L | . | . | . | . | . |
| 21-225_32A5_LC | . | . | . | [illegible] | . | . | . | . | . | . | . | . | . | . | . | . | . |

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | K_CDR2 | | | |
| CONSENSUS | P | K | L | L | I | Y | W | - | - | - | - | - | - | - | - | A | S |
| 21-225_34H7_LC | . | . | . | . | . | . | . | . | . | . | . | . | . | . | . | . | . |
| 21-225_24E1_LC | . | N | . | . | . | . | . | . | . | . | . | . | . | . | . | . | . |
| 21-225_23A12_LC | . | . | . | . | . | . | . | . | . | . | . | . | . | . | . | T | . |
| 21-225_25A4_LC | . | . | . | . | L | . | . | . | . | . | . | . | . | . | . | . | . |
| 21-225_28B6_LC | . | [illegible] | [illegible] | Q | . | . | [illegible] | . | . | . | . | . | . | . | . | V | . |
| 21-225_29E6_LC | [illegible] | . | . | . | L | . | . | . | . | . | . | . | . | . | . | . | . |
| 21-225_26C4_LC | . | . | . | . | . | . | . | . | . | . | . | . | . | . | . | . | . |
| 21-225_33D1_LC | [illegible] | . | . | F | . | . | . | . | . | . | . | . | . | . | . | . | . |
| 21-225_34D2_LC | . | Q | . | . | . | . | S | . | . | . | . | . | . | . | . | V | . |
| 21-225_29H8_LC | . | . | . | . | . | . | . | . | . | . | . | . | . | . | . | . | . |
| 21-225_11E5_LC | . | . | . | . | . | . | . | . | . | . | . | . | . | . | . | . | . |
| 21-225_8B11_LC | . | . | . | . | . | . | . | . | . | . | . | . | . | . | . | . | . |
| 21-225_22D2_LC | . | . | . | . | . | . | . | . | . | . | . | . | . | . | . | . | . |
| 21-225_32A1_LC | . | . | . | . | . | . | . | . | . | . | . | . | . | . | . | . | . |
| 21-225_32A5_LC | . | . | . | . | . | . | . | . | . | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | T | R | E | S | G | V | P | D | R | F | S | G | S | G | S | G | - |
| 21-225_34H7_LC | . | . | K | . | . | . | . | . | . | . | . | . | . | . | . | . | . |
| 21-225_24E1_LC | . | . | . | . | . | . | . | . | . | . | . | . | . | . | . | . | . |
| 21-225_23A12_LC | . | . | . | . | . | . | . | . | . | . | . | . | . | . | . | . | . |
| 21-225_25A4_LC | . | . | . | . | . | . | . | . | . | . | . | . | . | . | . | . | . |
| 21-225_28B6_LC | N | W | D | . | . | . | . | . | . | . | . | . | . | . | . | . | . |
| 21-225_29E6_LC | S | . | . | . | . | . | . | . | . | . | . | . | . | . | . | . | . |
| 21-225_26C4_LC | . | . | . | . | . | . | . | . | . | . | . | . | . | . | . | . | . |
| 21-225_33D1_LC | . | . | . | . | . | . | L | . | . | . | . | . | . | . | C | . | . |
| 21-225_34D2_LC | N | . | F | . | . | . | . | . | . | . | . | . | . | . | . | . | . |
| 21-225_29H8_LC | . | . | . | . | . | . | . | . | . | . | . | . | . | . | . | . | . |
| 21-225_11E5_LC | . | . | . | . | . | . | . | . | . | . | . | . | . | . | . | . | . |
| 21-225_8B11_LC | . | . | K | . | . | . | . | . | . | . | . | . | . | . | . | . | . |
| 21-225_22D2_LC | . | . | K | . | . | . | . | . | . | . | . | . | . | . | . | . | . |
| 21-225_32A1_LC | . | . | K | . | . | . | . | . | . | . | . | . | . | . | . | . | . |
| 21-225_32A5_LC | . | . | . | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | K_FR3 | | | | | | | | | | | | | | | | |
| CONSENSUS | - | T | D | F | T | L | T | I | S | S | L | Q | A | E | D | V | A |
| 21-225_34H7_LC | . | . | . | . | . | . | . | . | . | . | . | . | T | . | . | . | . |
| 21-225_24E1_LC | . | . | . | . | . | . | . | . | . | . | . | . | . | . | . | . | . |
| 21-225_23A12_LC | . | . | . | . | . | . | . | . | . | . | . | . | . | . | . | . | . |
| 21-225_25A4_LC | . | . | E | . | . | . | . | . | . | . | . | E | . | . | . | . | . |
| 21-225_28B6_LC | . | . | . | . | . | . | N | . | . | R | V | E | . | . | . | . | G |
| 21-225_29E6_LC | . | . | E | . | . | . | . | . | . | . | . | . | . | . | . | . | . |
| 21-225_26C4_LC | . | . | . | . | . | . | . | . | . | . | . | . | . | . | . | . | . |
| 21-225_33D1_LC | . | . | . | . | . | . | . | . | I | . | . | . | . | . | . | . | . |
| 21-225_34D2_LC | . | . | . | . | . | . | K | . | . | R | V | E | . | . | . | . | G |
| 21-225_29H8_LC | . | . | . | . | . | . | . | . | . | . | . | . | . | . | . | . | . |
| 21-225_11E5_LC | . | . | N | . | . | . | . | . | . | . | . | . | . | . | . | . | . |
| 21-225_8B11_LC | . | . | . | . | . | . | . | . | . | . | . | . | . | . | . | . | . |
| 21-225_22D2_LC | . | . | . | . | . | . | . | . | . | . | . | . | . | . | . | . | . |
| 21-225_32A1_LC | . | . | . | . | . | . | . | . | . | . | . | . | T | . | . | . | . |
| 21-225_32A5_LC | . | . | . | . | . | . | A | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | V | Y | Y | C | Q | Q | Y | Y | S | - | - | - | - | - | - | - | - |
| 21-225_34H7_LC | L | . | . | . | . | . | . | . | T | . | . | . | . | . | . | . | . |
| 21-225_24E1_LC | . | . | . | . | . | . | F | . | . | . | . | . | . | . | . | . | . |
| 21-225_23A12_LC | . | . | . | . | . | . | . | . | G | . | . | . | . | . | . | . | . |
| 21-225_25A4_LC | . | . | . | . | . | . | . | . | . | . | . | . | . | . | . | . | . |
| 21-225_28B6_LC | I | . | F | . | M | H | G | T | - | . | . | . | . | . | . | . | . |
| 21-225_29E6_LC | . | . | . | . | . | . | . | . | . | . | . | . | . | . | . | . | . |
| 21-225_26C4_LC | . | . | . | . | . | . | . | . | . | . | . | . | . | . | . | . | . |
| 21-225_33D1_LC | L | . | . | . | . | . | . | . | . | . | . | . | . | . | . | . | . |
| 21-225_34D2_LC | . | . | . | . | M | . | S | H | - | . | . | . | . | . | . | . | . |
| 21-225_29H8_LC | . | . | . | . | . | . | . | . | G | . | . | . | . | . | . | . | . |
| 21-225_11E5_LC | . | . | . | . | . | . | . | . | . | . | . | . | . | . | . | . | . |
| 21-225_8B11_LC | . | . | . | . | . | . | . | . | T | . | . | . | . | . | . | . | . |
| 21-225_22D2_LC | . | . | F | . | . | . | . | . | . | . | . | . | . | . | . | . | . |
| 21-225_32A1_LC | L | . | . | . | . | . | . | . | T | . | . | . | . | . | . | . | . |
| 21-225_32A5_LC | L | . | H | . | . | . | . | . | . | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | T | P |
| 21-225_34H7_LC | . | . | . | . | . | . | . | . | . | . | . | . | . | . | . | S | . |
| 21-225_24E1_LC | . | . | . | . | . | . | . | . | . | . | . | . | . | . | . | . | . |
| 21-225_23A12_LC | . | . | . | . | . | . | . | . | . | . | . | . | . | . | . | . | . |
| 21-225_25A4_LC | . | . | . | . | . | . | . | . | . | . | . | . | . | . | . | . | . |
| 21-225_28B6_LC | . | . | . | . | . | . | . | . | . | . | . | . | . | . | . | H | W |
| 21-225_29E6_LC | . | . | . | . | . | . | . | . | . | . | . | . | . | . | . | . | . |
| 21-225_26C4_LC | . | . | . | . | . | . | . | . | . | . | . | . | . | . | . | . | . |
| 21-225_33D1_LC | . | . | . | . | . | . | . | . | . | . | . | . | . | . | . | S | . |
| 21-225_34D2_LC | . | . | . | . | . | . | . | . | . | . | . | . | . | . | . | Q | L |
| 21-225_29H8_LC | . | . | . | . | . | . | . | . | . | . | . | . | . | . | . | . | . |
| 21-225_11E5_LC | . | . | . | . | . | . | . | . | . | . | . | . | . | . | . | . | . |
| 21-225_8B11_LC | . | . | . | . | . | . | . | . | . | . | . | . | . | . | . | . | . |
| 21-225_22D2_LC | . | . | . | . | . | . | . | . | . | . | . | . | . | . | . | . | . |
| 21-225_32A1_LC | . | . | . | . | . | . | . | . | . | . | . | . | . | . | . | S | . |
| 21-225_32A5_LC | . | . | . | . | . | . | . | . | . | . | . | . | . | . | . | S | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | K_FR4 | | | | | | | | | | |
| CONSENSUS | P | T | F | G | Q | G | T | K | V | E | I | K | R |
| 21-225_34H7_LC | C | S | . | . | . | . | . | . | L | . | . | . | . |
| 21-225_24E1_LC | . | . | . | . | . | . | . | . | . | . | . | . | . |
| 21-225_23A12_LC | . | . | . | . | . | . | . | . | . | . | . | . | . |
| 21-225_25A4_LC | . | . | . | . | . | . | . | . | . | . | . | . | . |
| 21-225_28B6_LC | L | L | . | . | P | . | . | . | . | D | . | . | . |
| 21-225_29E6_LC | . | . | . | . | . | . | . | . | . | . | . | . | . |
| 21-225_26C4_LC | . | . | . | . | . | . | . | . | . | . | F | . | . |
| 21-225_33D1_LC | C | S | . | . | . | . | . | . | L | . | . | . | . |
| 21-225_34D2_LC | . | P | . | . | G | . | . | . | . | . | . | R | . |
| 21-225_29H8_LC | . | . | . | . | . | . | . | . | . | . | . | . | . |
| 21-225_11E5_LC | . | . | . | . | . | . | . | . | . | . | . | . | . |
| 21-225_8B11_LC | C | S | . | . | . | . | . | . | L | . | . | . | . |
| 21-225_22D2_LC | C | S | . | . | . | . | . | . | L | . | . | . | . |
| 21-225_32A1_LC | C | S | . | . | . | . | . | . | L | . | . | . | . |
| 21-225_32A5_LC | C | S | . | . | . | . | . | G | L | . | . | . | . |

FIGURE 56

Table 38

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | K_FR1 | | | | | | | | | | | | | | | | |
| CONSENSUS | D | I | Q | M | T | Q | S | P | S | S | L | S | A | S | V | G | D |
| 21-225_2C12_LC | . | . | . | . | . | . | . | . | . | . | . | . | . | . | . | . | . |
| 21-225_17H5_LC | . | . | . | . | . | . | . | . | . | . | . | . | . | . | . | . | . |
| 21-225_16A11_LC | . | . | . | . | . | . | . | . | . | . | . | . | . | . | . | . | . |
| 21-225_20H7_LC | . | . | . | . | . | . | . | . | . | . | . | . | . | . | . | . | . |
| 21-225_20D12_LC | . | . | . | . | . | . | . | . | . | . | . | . | . | . | . | . | . |
| 21-225_22B12_LC | . | . | . | . | . | . | . | . | . | . | . | . | . | . | . | . | . |
| 21-225_27C5_LC | . | . | . | . | . | . | . | . | . | . | V | . | . | . | . | . | . |
| 21-225_27H12_LC | . | . | . | . | . | . | . | . | . | . | . | . | . | . | . | . | . |
| 21-225_35H8_LC | . | . | . | . | . | . | . | . | . | . | . | . | . | . | . | . | . |
| 21-225_15F8_LC | . | . | . | . | . | . | . | . | . | . | . | F | . | . | . | . | . |
| 21-225_20D6_LC | . | . | . | . | . | . | . | . | . | . | . | . | . | . | . | . | . |
| 21-225_15A2_LC | . | . | . | . | . | . | . | . | . | . | . | F | . | F | I | . | . |
| 21-225_5E11_LC | V | . | . | . | . | . | . | . | . | . | . | . | . | . | . | . | . |
| 21-225_2A4_LC | . | . | . | . | . | . | . | . | . | . | . | F | . | . | . | . | . |
| 21-225_16B8_LC | . | . | . | . | . | . | . | . | . | . | . | . | . | . | . | . | . |
| 21-225_14D6_LC | . | . | . | . | . | . | . | . | . | . | . | . | . | . | . | . | . |
| 21-225_1F1_LC | . | . | . | . | . | . | . | . | . | . | . | . | . | . | . | . | . |
| 21-225_5F4_LC | . | . | . | . | . | . | . | . | . | . | . | . | . | . | . | . | . |
| 21-225_18H1_LC | . | . | . | . | . | . | . | . | . | . | . | . | . | . | . | . | . |
| 21-225_17H6_LC | . | . | . | . | . | . | . | . | . | . | . | . | . | . | . | . | . |
| 21-225_21F12_LC | . | . | . | . | . | . | . | . | . | . | . | . | . | . | . | . | . |
| 21-225_23A11_LC | . | . | . | . | . | . | . | . | . | . | . | . | T | . | . | . | . |
| 21-225_32B3_LC | . | . | . | . | . | . | . | . | . | . | . | . | . | . | I | . | . |

FIGURE 56

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | K_CDR1 | | |
| CONSENSUS | R | V | T | I | T | C | R | A | S | - | - | Q | G | I | S | - | - |
| 21-225_2C12_LC | . | . | . | . | . | . | . | . | . | . | . | . | . | . | . | . | . |
| 21-225_17H5_LC | . | . | . | . | . | . | . | . | . | . | . | . | . | . | . | . | . |
| 21-225_16A11_LC | S | . | . | . | S | . | . | . | . | . | . | . | D | . | R | . | . |
| 21-225_20H7_LC | . | . | . | . | . | . | . | T | . | . | . | . | . | . | N | . | . |
| 21-225_20D12_LC | . | . | . | . | . | . | . | . | . | . | . | . | D | . | R | . | . |
| 21-225_22B12_LC | . | . | . | . | I | . | . | . | . | . | . | . | D | . | . | . | . |
| 21-225_27C5_LC | . | . | . | . | . | . | . | . | . | . | . | . | . | . | . | . | . |
| 21-225_27H12_LC | . | . | . | . | . | . | . | . | . | . | . | . | . | . | . | . | . |
| 21-225_35H8_LC | . | . | . | . | . | . | . | . | . | . | . | . | A | . | . | . | . |
| 21-225_15F8_LC | . | . | . | . | . | . | . | . | . | . | . | R | D | . | . | . | . |
| 21-225_20D6_LC | . | . | . | . | . | . | . | . | . | . | . | . | . | . | . | . | . |
| 21-225_15A2_LC | . | . | . | . | . | . | . | . | . | . | . | . | . | . | . | . | . |
| 21-225_5E11_LC | . | . | . | . | . | . | . | . | . | . | . | . | . | . | . | . | . |
| 21-225_2A4_LC | . | . | . | . | . | . | . | . | . | . | . | . | . | . | . | . | . |
| 21-225_16B8_LC | . | . | . | . | . | . | . | . | . | . | . | . | . | . | N | . | . |
| 21-225_14D6_LC | S | . | . | . | . | . | . | . | . | . | . | . | D | . | R | . | . |
| 21-225_1F1_LC | . | . | . | . | . | . | . | . | . | . | . | . | . | . | N | . | . |
| 21-225_5F4_LC | . | . | . | . | . | . | . | . | . | . | . | . | . | . | R | . | . |
| 21-225_16H1_LC | . | . | . | . | . | . | . | . | . | . | . | . | . | . | R | . | . |
| 21-225_17H6_LC | . | . | . | . | . | . | . | . | . | . | . | R | D | . | . | . | . |
| 21-225_21F12_LC | . | . | . | . | . | . | . | . | . | . | . | . | . | . | R | . | . |
| 21-225_23A11_LC | . | . | . | . | . | . | . | . | . | . | . | . | D | . | . | . | . |
| 21-225_32B3_LC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | | K_FR2 | |
| CONSENSUS | - | - | - | - | N | Y | L | A | W | F | Q | Q | K | P | G | K | A |
| 21-225_2C12_LC | . | . | . | . | . | . | . | . | . | . | . | . | . | S | . | . | . |
| 21-225_17H5_LC | . | . | . | . | . | . | . | . | . | . | . | . | . | . | . | . | . |
| 21-225_16A11_LC | . | . | . | . | . | H | . | G | . | Y | . | H | N | . | . | . | . |
| 21-225_20H7_LC | . | . | . | . | . | . | . | V | . | . | . | . | . | . | . | . | . |
| 21-225_20D12_LC | . | . | . | . | . | H | . | G | . | Y | . | . | . | . | . | . | . |
| 21-225_22B12_LC | . | . | . | . | . | . | . | . | . | . | . | E | . | . | . | . | . |
| 21-225_27C5_LC | . | . | . | . | S | W | . | . | . | Y | . | . | . | . | . | . | . |
| 21-225_27H12_LC | . | . | . | . | . | . | . | . | . | . | . | . | . | . | . | . | . |
| 21-225_35H8_LC | . | . | . | . | . | . | . | . | . | . | . | . | . | . | . | . | . |
| 21-225_15F8_LC | . | . | . | . | . | . | . | . | . | . | . | . | . | . | . | . | . |
| 21-225_20D6_LC | . | . | . | . | . | . | . | . | . | . | . | . | . | . | . | . | . |
| 21-225_15A2_LC | . | . | . | . | . | . | . | . | . | . | . | . | . | . | . | . | . |
| 21-225_5E11_LC | . | . | . | . | . | . | . | . | . | . | . | . | . | . | . | . | . |
| 21-225_2A4_LC | . | . | . | . | . | . | . | . | . | . | . | . | . | . | . | . | . |
| 21-225_16B8_LC | . | . | . | . | . | . | . | . | . | . | . | . | . | . | . | . | . |
| 21-225_14D6_LC | . | . | . | . | . | H | . | G | . | Y | . | H | . | . | . | . | . |
| 21-225_1F1_LC | . | . | . | . | . | . | . | . | . | . | . | . | . | S | . | . | . |
| 21-225_5F4_LC | . | . | . | . | . | H | . | G | . | Y | . | . | . | . | . | . | . |
| 21-225_16H1_LC | . | . | . | . | D | I | . | G | . | Y | . | . | . | . | . | . | . |
| 21-225_17H6_LC | . | . | . | . | . | . | . | . | . | . | . | . | . | . | . | . | . |
| 21-225_21F12_LC | . | . | . | . | . | F | . | . | . | . | . | . | . | . | . | . | . |
| 21-225_23A11_LC | . | . | . | . | . | . | . | . | . | . | . | . | . | . | . | . | . |
| 21-225_32B3_LC | . | . | . | . | . | . | . | . | . | . | . | . | . | . | . | . | . |

**FIGURE 56**

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | K_CDR2 | | | |
| CONSENSUS | P | K | S | L | I | Y | A | - | - | - | - | - | - | - | - | A | S |
| 21-225_2C12_LC | . | . | . | . | . | S | . | . | . | . | . | . | . | . | . | . | T |
| 21-225_17H5_LC | . | . | . | . | . | N | . | . | . | . | . | . | . | . | . | . | . |
| 21-225_16A11_LC | . | . | R | . | . | . | . | . | . | . | . | . | . | . | . | . | . |
| 21-225_20H7_LC | . | . | R | . | . | . | . | . | . | . | . | . | . | . | . | . | . |
| 21-225_20D12_LC | . | . | R | . | . | . | . | . | . | . | . | . | . | . | . | . | . |
| 21-225_22B12_LC | H | . | . | . | . | . | G | . | . | . | . | . | . | . | . | . | . |
| 21-225_27C5_LC | . | . | F | . | . | . | G | . | . | . | . | . | . | . | . | . | . |
| 21-225_27H12_LC | . | . | . | . | . | N | G | . | . | . | . | . | . | . | . | . | . |
| 21-225_35H8_LC | . | . | . | . | . | . | G | . | . | . | . | . | . | . | . | . | . |
| 21-225_15F8_LC | . | . | . | . | . | C | S | . | . | . | . | . | . | . | . | T | . |
| 21-225_20D6_LC | . | . | . | . | . | S | . | . | . | . | . | . | . | . | . | . | . |
| 21-225_15A2_LC | . | . | . | . | . | F | . | . | . | . | . | . | . | . | . | . | . |
| 21-225_5E11_LC | . | . | . | . | . | N | . | . | . | . | . | . | . | . | . | . | . |
| 21-225_2A4_LC | . | . | R | . | . | . | T | . | . | . | . | . | . | . | . | . | . |
| 21-225_16B8_LC | L | . | . | . | . | N | . | . | . | . | . | . | . | . | . | . | . |
| 21-225_14D6_LC | . | . | R | . | . | . | . | . | . | . | . | . | . | . | . | . | . |
| 21-225_1F1_LC | . | . | . | . | . | S | V | . | . | . | . | . | . | . | . | . | . |
| 21-225_5F4_LC | . | . | R | . | . | . | . | . | . | . | . | . | . | . | . | . | . |
| 21-225_16H1_LC | . | . | R | . | . | . | . | . | . | . | . | . | . | . | . | . | . |
| 21-225_17H6_LC | . | . | . | . | . | S | T | . | . | . | . | . | . | . | . | . | . |
| 21-225_21F12_LC | . | . | . | . | . | S | . | . | . | . | . | . | . | . | . | . | . |
| 21-225_23A11_LC | . | . | R | . | . | . | . | . | . | . | . | . | . | . | . | . | . |
| 21-225_32B3_LC | . | R | . | . | . | . | . | . | . | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | K | F | S | G | S | G | S | G | - |
| 21-225_2C12_LC | . | . | . | . | . | . | . | . | Q | . | . | . | . | . | . | . | . |
| 21-225_17H5_LC | . | . | . | . | . | . | . | . | . | . | . | . | . | . | . | . | . |
| 21-225_16A11_LC | . | . | . | . | . | . | . | . | R | . | . | . | . | . | . | . | . |
| 21-225_20H7_LC | . | . | . | . | . | . | . | . | . | . | . | . | . | . | . | . | . |
| 21-225_20D12_LC | . | . | . | . | . | . | . | . | R | . | . | . | . | . | . | . | . |
| 21-225_22B12_LC | . | . | R | . | . | . | . | . | . | . | . | . | . | . | . | . | . |
| 21-225_27C5_LC | . | . | . | . | . | . | . | . | R | . | . | A | . | . | . | . | . |
| 21-225_27H12_LC | . | . | . | . | . | . | . | . | . | . | . | . | . | . | . | . | . |
| 21-225_35H8_LC | G | . | . | . | . | . | . | . | . | . | . | . | . | . | . | . | . |
| 21-225_15F8_LC | . | . | . | . | . | . | . | . | . | . | . | . | . | . | F | . | . |
| 21-225_20D6_LC | N | . | . | . | . | . | . | . | . | . | . | . | . | . | . | . | . |
| 21-225_15A2_LC | . | . | . | . | . | . | . | . | . | . | . | . | . | . | F | . | . |
| 21-225_5E11_LC | . | V | . | . | . | . | . | . | . | . | . | . | N | . | . | . | . |
| 21-225_2A4_LC | . | . | . | . | . | . | . | . | . | . | . | . | . | . | F | . | . |
| 21-225_16B8_LC | . | V | . | . | . | . | . | . | . | . | . | . | . | . | . | . | . |
| 21-225_14D6_LC | . | . | . | . | . | . | . | . | R | . | . | . | . | . | . | . | . |
| 21-225_1F1_LC | . | . | . | T | . | . | . | . | R | . | . | . | . | . | . | . | . |
| 21-225_5F4_LC | . | . | . | . | . | . | . | . | R | . | . | . | . | . | . | . | . |
| 21-225_16H1_LC | . | . | . | N | . | . | . | . | R | . | . | . | . | . | . | . | . |
| 21-225_17H6_LC | . | . | . | . | . | . | . | . | . | . | . | . | . | . | . | . | . |
| 21-225_21F12_LC | . | . | . | T | . | . | . | . | . | . | . | . | . | . | . | . | . |
| 21-225_23A11_LC | T | . | . | . | . | . | . | . | . | . | . | . | . | . | . | . | . |
| 21-225_32B3_LC | . | . | . | . | . | . | . | . | T | . | . | . | . | . | . | . | . |

FIGURE 56

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | K_FR3 | | | | | | | | | | | | | | | | |
| CONSENSUS | - | T | D | F | T | L | T | I | S | S | L | Q | P | E | D | F | A |
| 21-225_2C12_LC | . | . | . | . | . | . | . | . | . | . | . | . | . | . | . | . | . |
| 21-225_17H5_LC | . | . | . | . | . | . | . | . | . | . | . | . | . | . | . | . | . |
| 21-225_16A11_LC | . | . | S | . | . | . | . | . | . | . | . | . | . | . | . | . | . |
| 21-225_20H7_LC | . | . | . | . | . | . | . | . | . | . | . | . | . | . | . | . | . |
| 21-225_20D12_LC | . | . | S | . | . | . | . | . | . | . | . | . | . | . | . | . | . |
| 21-225_22B12_LC | . | . | . | . | N | . | . | . | . | . | . | . | . | . | . | L | . |
| 21-225_27C5_LC | . | . | S | . | . | . | . | . | . | . | . | . | . | . | . | . | . |
| 21-225_27H12_LC | . | . | . | . | . | . | . | . | . | . | . | . | . | . | . | . | . |
| 21-225_35H8_LC | . | . | . | . | . | . | . | . | . | . | . | . | . | . | . | . | . |
| 21-225_15F8_LC | . | . | . | . | . | . | . | . | . | . | . | . | . | . | . | . | . |
| 21-225_20D6_LC | . | . | . | . | . | . | . | . | . | . | . | . | . | . | . | . | . |
| 21-225_15A2_LC | . | . | . | . | . | F | P | . | . | . | . | . | . | . | . | . | . |
| 21-225_5E11_LC | . | . | . | . | . | . | . | . | . | . | . | . | . | . | . | . | . |
| 21-225_2A4_LC | . | . | . | . | . | . | . | . | . | . | . | . | . | . | . | . | . |
| 21-225_16B8_LC | . | . | . | . | . | . | . | . | . | . | . | . | . | . | . | . | . |
| 21-225_14D6_LC | . | . | S | . | . | . | . | . | . | . | . | . | . | . | . | . | . |
| 21-225_1F1_LC | . | . | . | . | . | . | . | . | . | . | . | . | . | . | . | . | . |
| 21-225_5F4_LC | . | . | S | . | . | . | . | V | . | . | . | . | . | . | . | . | . |
| 21-225_16H1_LC | . | . | S | . | . | . | . | . | . | . | . | . | . | . | . | . | . |
| 21-225_17H6_LC | . | . | . | . | . | . | . | . | . | . | . | . | . | . | . | . | . |
| 21-225_21F12_LC | . | . | . | . | . | . | . | . | . | . | . | . | . | . | . | . | . |
| 21-225_23A11_LC | . | . | . | . | . | . | . | . | . | . | . | . | . | . | . | . | . |
| 21-225_32B3_LC | . | . | . | . | . | . | . | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | Q | Q | Y | N | S | - | - | - | - | - | - | - | - |
| 21-225_2C12_LC | . | . | . | . | . | . | . | Y | . | . | . | . | . | . | . | . | . |
| 21-225_17H5_LC | . | . | . | . | . | . | . | H | . | . | . | . | . | . | . | . | . |
| 21-225_16A11_LC | . | . | . | . | L | . | . | . | . | . | . | . | . | . | . | . | . |
| 21-225_20H7_LC | . | . | . | . | . | . | . | Y | . | . | . | . | . | . | . | . | . |
| 21-225_20D12_LC | . | . | . | . | L | . | . | . | . | . | . | . | . | . | . | . | . |
| 21-225_22B12_LC | . | . | . | . | . | . | . | H | . | . | . | . | . | . | . | . | . |
| 21-225_27C5_LC | . | . | C | . | . | . | . | D | . | . | . | . | . | . | . | . | . |
| 21-225_27H12_LC | . | . | . | . | . | . | . | Y | . | . | . | . | . | . | . | . | . |
| 21-225_35H8_LC | . | . | . | . | . | . | . | . | . | . | . | . | . | . | . | . | . |
| 21-225_15F8_LC | A | . | . | . | . | . | . | H | . | . | . | . | . | . | . | . | . |
| 21-225_20D6_LC | . | . | F | . | . | . | . | H | . | . | . | . | . | . | . | . | . |
| 21-225_15A2_LC | N | . | . | . | . | . | . | Y | . | . | . | . | . | . | . | . | . |
| 21-225_5E11_LC | . | . | . | . | H | . | . | H | . | . | . | . | . | . | . | . | . |
| 21-225_2A4_LC | . | . | . | . | . | . | . | . | . | . | . | . | . | . | . | . | . |
| 21-225_16B8_LC | . | . | . | . | H | . | . | H | . | . | . | . | . | . | . | . | . |
| 21-225_14D6_LC | . | . | . | . | L | . | . | . | . | . | . | . | . | . | . | . | . |
| 21-225_1F1_LC | . | . | . | . | . | . | . | . | . | . | . | . | . | . | . | . | . |
| 21-225_5F4_LC | . | . | . | . | L | . | . | . | G | . | . | . | . | . | . | . | . |
| 21-225_16H1_LC | . | . | . | . | L | . | . | H | . | . | . | . | . | . | . | . | . |
| 21-225_17H6_LC | I | . | . | . | . | . | . | H | . | . | . | . | . | . | . | . | . |
| 21-225_21F12_LC | . | . | . | . | . | . | . | . | . | . | . | . | . | . | . | . | . |
| 21-225_23A11_LC | . | . | . | . | . | . | . | . | . | . | . | . | . | . | . | . | . |
| 21-225_32B3_LC | . | . | . | . | . | . | . | . | . | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_2C12_LC | . | . | . | . | . | . | . | . | . | . | . | . | . | . | . | . | . |
| 21-225_17H5_LC | . | . | . | . | . | . | . | . | . | . | . | . | . | . | . | . | . |
| 21-225_16A11_LC | . | . | . | . | . | . | . | . | . | . | . | . | . | . | . | . | . |
| 21-225_20H7_LC | . | . | . | . | . | . | . | . | . | . | . | . | . | . | . | . | . |
| 21-225_20D12_LC | . | . | . | . | . | . | . | . | . | . | . | . | . | . | . | . | . |
| 21-225_22B12_LC | . | . | . | . | . | . | . | . | . | . | . | . | . | . | . | . | . |
| 21-225_27C5_LC | . | . | . | . | . | . | . | . | . | . | . | . | . | . | . | [illegible] | . |
| 21-225_27H12_LC | . | . | . | . | . | . | . | . | . | . | . | . | . | . | . | . | . |
| 21-225_35H8_LC | . | . | . | . | . | . | . | . | . | . | . | . | . | . | . | . | . |
| 21-225_15F8_LC | . | . | . | . | . | . | . | . | . | . | . | . | . | . | . | . | . |
| 21-225_20D6_LC | . | . | . | . | . | . | . | . | . | . | . | . | . | . | . | [illegible] | . |
| 21-225_15A2_LC | . | . | . | . | . | . | . | . | . | . | . | . | . | . | . | . | . |
| 21-225_5E11_LC | . | . | . | . | . | . | . | . | . | . | . | . | . | . | . | . | . |
| 21-225_2A4_LC | . | . | . | . | . | . | . | . | . | . | . | . | . | . | . | . | . |
| 21-225_16B8_LC | . | . | . | . | . | . | . | . | . | . | . | . | . | . | . | . | . |
| 21-225_14D6_LC | . | . | . | . | . | . | . | . | . | . | . | . | . | . | . | . | . |
| 21-225_1F1_LC | . | . | . | . | . | . | . | . | . | . | . | . | . | . | . | . | . |
| 21-225_5F4_LC | . | . | . | . | . | . | . | . | . | . | . | . | . | . | . | . | . |
| 21-225_16H1_LC | . | . | . | . | . | . | . | . | . | . | . | . | . | . | . | . | . |
| 21-225_17H6_LC | . | . | . | . | . | . | . | . | . | . | . | . | . | . | . | . | . |
| 21-225_21F12_LC | . | . | . | . | . | . | . | . | . | . | . | . | . | . | . | [illegible] | . |
| 21-225_23A11_LC | . | . | . | . | . | . | . | . | . | . | . | . | . | . | . | . | . |
| 21-225_32B3_LC | . | . | . | . | . | . | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | K_FR4 | | | | | | | | | | | | |
| CONSENSUS | F | T | F | G | P | G | T | K | V | D | I | K | R |
| 21-225_2C12_LC | I | . | . | . | Q | . | . | [illegible] | L | E | . | . | . |
| 21-225_17H5_LC | . | . | . | . | . | . | . | . | . | . | . | . | . |
| 21-225_16A11_LC | . | . | . | . | . | . | . | . | . | . | . | . | . |
| 21-225_20H7_LC | . | . | . | . | . | . | . | . | M | . | F | . | . |
| 21-225_20D12_LC | . | . | . | . | . | . | . | . | . | . | . | . | . |
| 21-225_22B12_LC | . | . | . | . | . | . | . | . | . | . | F | . | . |
| 21-225_27C5_LC | [illegible] | . | . | . | Q | . | . | . | . | E | . | . | . |
| 21-225_27H12_LC | . | . | . | . | . | . | . | . | . | N | . | N | . |
| 21-225_35H8_LC | . | . | . | . | . | . | . | . | . | . | F | . | . |
| 21-225_15F8_LC | . | . | . | . | [illegible] | . | . | . | . | . | . | Q | . |
| 21-225_20D6_LC | . | . | . | . | . | . | . | . | . | . | . | . | . |
| 21-225_15A2_LC | . | . | . | . | . | . | . | Q | . | . | V | . | . |
| 21-225_5E11_LC | . | . | . | . | . | . | . | . | . | . | . | . | . |
| 21-225_2A4_LC | . | . | . | . | . | . | . | . | . | . | . | . | . |
| 21-225_16B8_LC | . | . | . | . | . | . | . | . | . | . | . | V | . |
| 21-225_14D6_LC | . | . | . | . | . | . | . | . | . | . | . | N | . |
| 21-225_1F1_LC | L | . | . | . | Q | . | . | . | . | E | . | . | . |
| 21-225_5F4_LC | . | . | . | . | . | . | . | . | . | . | . | . | . |
| 21-225_16H1_LC | . | . | . | . | . | . | . | . | . | . | V | . | . |
| 21-225_17H6_LC | . | . | . | . | . | . | . | . | . | . | . | Q | . |
| 21-225_21F12_LC | . | . | . | . | . | . | . | . | . | . | . | . | . |
| 21-225_23A11_LC | . | . | . | . | . | . | . | . | . | . | L | . | . |
| 21-225_32B3_LC | . | . | . | . | . | . | . | . | . | . | . | . | . |

FIGURE 56

Table 39

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | K_FR1 | | | | | | | | | | | | | | | | |
| CONSENSUS | D | I | Q | M | T | Q | S | P | S | S | L | S | A | S | V | G | D |
| 21-225_17C10_LC | . | . | . | . | . | . | . | . | . | . | Q | . | . | . | . | . | . |
| 21-225_20B5_LC | . | . | . | . | . | . | . | . | . | . | . | . | . | . | . | . | . |
| 21-225_17D8_LC | E | . | V | . | . | . | . | . | . | T | . | . | V | . | P | . | E |
| 21-225_20B8_LC | E | . | V | . | . | . | . | . | . | T | . | . | V | . | P | . | E |
| 21-225_22H3_LC | . | . | . | . | . | . | . | . | . | . | . | . | . | . | . | . | . |
| 21-225_20C8_LC | . | . | . | . | . | . | . | . | . | . | . | . | . | . | . | . | . |
| 21-225_20G12_LC | . | . | . | . | . | . | . | . | . | . | . | . | . | . | . | . | . |
| 21-225_23E11_LC | . | . | . | . | . | . | . | . | . | . | . | . | . | . | . | . | . |
| 21-225_20F9_LC | . | . | . | . | . | . | . | . | . | . | . | . | . | . | . | . | . |
| 21-225_20H10_LC | G | . | . | . | . | . | . | . | . | . | . | . | . | . | . | . | . |
| 21-225_31G9_LC | . | . | . | . | . | . | . | . | . | . | . | . | . | . | I | . | . |
| 21-225_30H11_LC | . | . | . | . | . | . | . | . | . | . | . | . | . | . | . | . | . |
| 21-225_33C2_LC | . | . | . | . | . | . | . | . | . | . | . | . | . | . | . | . | . |
| 21-225_14B1_LC | . | . | . | . | . | . | . | . | . | Y | . | . | . | . | . | . | . |
| 21-225_26B11_LC | . | . | . | . | . | . | . | . | . | . | . | . | . | . | . | . | . |
| 21-225_13D3_LC | E | . | V | . | . | . | . | . | A | T | . | . | V | . | P | . | E |
| 21-225_6C12_LC | . | . | . | . | . | . | . | . | . | . | . | . | . | . | . | . | . |

| Linear # | 1 | 2 | 3 | 4 | 5 | 6 | 7 | 7.1 | 8 | 9 | 10 | 11 | 12 | 13 | 14 | 15 | 16 |
|---|---|---|---|---|---|---|---|---|---|---|---|---|---|---|---|---|---|
| | L_FR1 | | | | | | | | | | | | | | | | |
| 21-225_14B1_LC | S | Y | E | L | T | Q | P | - | P | S | V | S | V | S | P | G | Q |

FIGURE 56

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | K_CDR1 | | |
| CONSENSUS | R | V | T | I | T | C | R | A | S | - | - | Q | G | I | R | - | - |
| 21-225_17C10_LC | . | . | . | . | . | . | . | . | . | . | . | . | . | . | S | . | . |
| 21-225_20B5_LC | . | . | . | . | . | . | . | . | . | . | . | . | . | . | N | . | . |
| 21-225_17D8_LC | . | A | . | L | S | . | . | . | . | . | . | . | S | V | S | . | . |
| 21-225_20B8_LC | . | . | . | L | S | . | . | . | . | . | . | . | S | V | S | . | . |
| 21-225_22H3_LC | . | . | . | . | . | . | . | . | . | . | . | . | D | . | . | . | . |
| 21-225_20C8_LC | . | . | . | . | . | . | . | . | . | . | . | . | . | . | . | . | . |
| 21-225_20G12_LC | . | . | . | . | . | . | . | . | . | . | . | . | . | . | S | . | . |
| 21-225_23E11_LC | . | I | . | . | . | . | . | . | . | . | . | . | . | . | . | . | . |
| 21-225_20F9_LC | . | I | . | . | . | . | . | . | . | . | . | . | . | . | S | . | . |
| 21-225_20H10_LC | . | . | . | . | . | . | . | . | . | . | . | . | . | . | K | . | . |
| 21-225_31G9_LC | . | . | . | . | . | . | . | . | . | . | . | . | . | . | . | . | . |
| 21-225_30H11_LC | . | . | . | . | . | . | . | . | . | . | . | . | . | . | . | . | . |
| 21-225_33C2_LC | . | . | . | . | . | . | . | . | . | . | . | . | . | . | . | . | . |
| 21-225_14B1_LC | . | . | . | . | . | . | . | . | . | . | . | . | D | . | . | . | . |
| 21-225_26B11_LC | . | . | . | . | . | . | . | . | . | . | . | . | D | . | . | . | . |
| 21-225_13D3_LC | S | A | . | L | S | . | . | . | . | . | . | . | T | V | S | . | . |
| 21-225_6C12_LC | . | G | . | . | . | . | . | . | . | . | . | . | . | . | . | . | . |

| Linear # | 17 | 18 | 19 | 20 | 21 | 22 | 23 | 24 | 25 | 25.1 | 25.2 | 25.3 | 26 | 27 | 28 | 29 | 29.1 |
|---|---|---|---|---|---|---|---|---|---|---|---|---|---|---|---|---|---|
| | | | | | | | | | | | | | | | L_CDR1 | | |
| 21-225_14B1_LC | Y | A | S | I | Y | C | S | G | D | . | . | . | K | L | G | D | . |

**FIGURE 56**

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | | K_FR2 | |
| CONSENSUS | - | - | - | - | N | N | L | G | W | Y | Q | Q | K | P | G | K | A |
| 21-225_17C10_LC | . | . | . | . | . | Y | . | A | . | . | . | . | . | . | . | R | V |
| 21-225_20B5_LC | . | . | . | . | . | Y | . | A | . | . | . | . | . | . | . | . | I |
| 21-225_17D8_LC | . | . | . | . | S | . | . | A | . | F | . | . | . | . | . | Q | . |
| 21-225_20B8_LC | . | . | . | . | S | . | . | A | . | F | . | . | . | . | . | Q | . |
| 21-225_22H3_LC | . | . | . | . | . | . | . | . | . | . | . | . | . | . | . | . | . |
| 21-225_20C8_LC | . | . | . | . | . | D | . | . | . | . | . | K | . | . | . | . | . |
| 21-225_20G12_LC | . | . | . | . | . | Y | . | A | . | . | . | . | . | . | . | . | V |
| 21-225_23E11_LC | . | . | . | . | . | D | . | . | . | . | . | . | . | . | . | . | . |
| 21-225_20F9_LC | . | . | . | . | . | Y | . | A | . | F | . | . | . | . | . | . | . |
| 21-225_20H10_LC | . | . | . | . | . | . | . | . | . | . | . | . | . | . | . | . | G |
| 21-225_31G9_LC | . | . | . | . | . | D | . | . | . | . | . | . | . | . | . | . | . |
| 21-225_30H11_LC | . | . | . | . | . | D | . | . | . | . | . | . | . | . | . | . | . |
| 21-225_33C2_LC | . | . | . | . | . | D | . | . | . | . | . | . | . | . | V | . | . |
| 21-225_14B1_LC | . | . | . | . | D | . | . | . | . | . | . | . | . | . | . | . | . |
| 21-225_26B11_LC | . | . | . | . | D | D | . | . | . | . | . | . | . | . | . | . | . |
| 21-225_13D3_LC | . | . | . | . | S | . | . | A | . | F | . | . | . | . | . | Q | . |
| 21-225_6C12_LC | . | . | . | . | S | . | . | . | . | . | . | . | . | . | . | . | . |

| Linear # | 29.2 | 29.3 | 29.4 | 29.5 | 30 | 31 | 32 | 33 | 34 | 35 | 36 | 37 | 38 | 39 | 40 | 41 | 42 |
|---|---|---|---|---|---|---|---|---|---|---|---|---|---|---|---|---|---|
| | | | | | | | | | | | | | | | | L_FR2 | |
| 21-225_14B1_LC | . | . | . | . | K | Y | A | Y | W | Y | Q | Q | K | P | G | Q | S |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | K_CDR2 | | | | | | | | | | |
| CONSENSUS | P | K | R | L | I | Y | A | - | - | - | - | - | - | - | - | A | S |
| 21-225_17C10_LC | . | . | L | . | . | . | . | . | . | . | . | . | . | . | . | . | . |
| 21-225_20B5_LC | . | . | L | . | . | . | . | . | . | . | . | . | . | . | . | . | . |
| 21-225_17D8_LC | . | R | L | . | . | N | G | . | . | . | . | . | . | . | . | . | . |
| 21-225_20B8_LC | . | R | L | . | . | N | G | . | . | . | . | . | . | . | . | . | . |
| 21-225_22H3_LC | . | . | . | . | . | . | P | . | . | . | . | . | . | . | . | . | . |
| 21-225_20C8_LC | . | . | . | . | . | N | . | . | . | . | . | . | . | . | . | . | . |
| 21-225_20G12_LC | . | . | V | . | . | . | T | . | . | . | . | . | . | . | . | . | . |
| 21-225_23E11_LC | . | . | . | . | . | . | P | . | . | . | . | . | . | . | . | . | . |
| 21-225_20F9_LC | . | . | S | . | . | S | . | . | . | . | . | . | . | . | . | . | . |
| 21-225_20H10_LC | . | . | G | . | . | . | . | . | . | . | . | . | . | . | . | . | . |
| 21-225_31G9_LC | . | . | . | . | . | . | P | . | . | . | . | . | . | . | . | . | . |
| 21-225_30H11_LC | . | . | . | . | V | . | P | . | . | . | . | . | . | . | . | . | . |
| 21-225_33C2_LC | . | . | . | . | . | . | . | . | . | . | . | . | . | . | . | . | P |
| 21-225_14B1_LC | . | . | . | . | . | . | T | . | . | . | . | . | . | . | . | . | Y |
| 21-225_26B11_LC | . | R | . | . | . | . | P | . | . | . | . | . | . | . | . | . | . |
| 21-225_13D3_LC | . | R | L | . | . | N | G | . | . | . | . | . | . | . | . | . | . |
| 21-225_6C12_LC | . | . | . | . | . | . | . | . | . | . | . | . | . | . | . | . | . |

| Linear # | 43 | 44 | 45 | 46 | 47 | 48 | 49 | 49.1 | 49.2 | 49.3 | 49.4 | 49.5 | 49.6 | 49.7 | 49.8 | 50 | 51 |
|---|---|---|---|---|---|---|---|---|---|---|---|---|---|---|---|---|---|
| | | | | | | | L_CDR2 | | | | | | | | | | |
| 21-225_14B1_LC | P | V | L | V | I | Y | G | - | - | - | - | - | - | - | - | D | R |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | R | F | S | G | S | G | S | G | - |
| 21-225_17C10_LC | T | . | . | . | . | . | . | . | . | . | . | . | . | . | . | . | . |
| 21-225_20B5_LC | T | . | . | . | . | . | . | . | . | . | . | . | . | . | . | . | . |
| 21-225_17D8_LC | T | R | A | . | . | I | . | A | . | . | . | . | . | . | . | . | . |
| 21-225_20B8_LC | T | R | A | . | . | I | . | A | . | . | . | . | . | . | . | . | . |
| 21-225_22H3_LC | . | . | . | T | . | . | . | . | . | . | . | . | . | . | . | . | . |
| 21-225_20C8_LC | . | . | . | . | . | . | . | . | . | . | . | . | . | . | . | . | . |
| 21-225_20G12_LC | T | . | . | . | . | . | . | . | . | . | . | . | . | . | . | . | . |
| 21-225_23E11_LC | . | . | . | . | . | . | . | . | . | . | . | . | . | . | . | . | . |
| 21-225_20F9_LC | . | . | . | . | . | . | . | . | K | . | . | . | . | . | . | . | . |
| 21-225_20H10_LC | . | . | . | . | . | . | . | . | . | . | . | . | . | . | . | . | . |
| 21-225_31G9_LC | . | . | . | . | . | . | . | . | . | . | . | . | . | . | . | . | . |
| 21-225_30H11_LC | . | . | . | . | . | . | . | . | . | . | . | . | . | . | . | . | . |
| 21-225_33C2_LC | . | . | . | . | . | . | . | . | . | . | . | . | . | . | . | . | . |
| 21-225_14B1_LC | . | . | . | . | . | . | . | . | . | . | . | . | . | . | . | . | . |
| 21-225_26B11_LC | . | . | . | . | . | . | . | . | . | . | . | . | . | . | . | . | . |
| 21-225_13D3_LC | T | R | A | T | . | I | . | A | . | . | . | . | . | . | . | . | . |
| 21-225_6C12_LC | . | . | E | . | . | . | . | . | . | . | . | . | . | . | . | . | . |

| Linear # | 52 | 53 | 54 | 55 | 56 | 57 | 58 | 59 | 60 | 61 | 62 | 63 | 64 | 65 | 66 | 67 | 67.1 |
|---|---|---|---|---|---|---|---|---|---|---|---|---|---|---|---|---|---|
| 21-225_14B1_LC | K | R | P | S | G | I | P | E | R | F | S | G | S | R | S | G | . |

FIGURE 56

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | K_FR3 | | | | | | | | | | | | | | | | |
| CONSENSUS | - | T | E | F | T | L | T | I | S | S | L | Q | P | E | D | F | A |
| 21-225_17C10_LC | . | . | D | . | . | . | . | . | . | . | . | . | . | D | . | V | . |
| 21-225_20B5_LC | . | . | D | . | . | . | . | . | . | . | . | . | . | . | . | V | . |
| 21-225_17D8_LC | . | . | . | . | . | . | . | . | . | . | . | . | S | . | . | . | . |
| 21-225_20B8_LC | . | . | . | . | . | . | . | . | . | . | . | . | S | . | . | . | . |
| 21-225_22H3_LC | . | . | . | . | . | . | . | . | . | . | . | . | . | . | . | . | . |
| 21-225_20C8_LC | . | . | . | . | . | . | . | . | . | . | . | . | . | . | . | . | . |
| 21-225_20G12_LC | . | . | D | . | . | . | . | V | . | . | . | . | . | . | . | V | . |
| 21-225_23E11_LC | . | . | . | . | . | . | . | . | . | . | . | . | . | . | . | . | . |
| 21-225_20F9_LC | . | . | D | . | . | . | . | . | . | . | . | . | . | . | . | . | . |
| 21-225_20H10_LC | . | . | . | . | . | . | . | . | . | . | . | . | . | . | . | . | . |
| 21-225_31G9_LC | . | . | . | . | . | . | . | . | . | . | . | . | . | . | . | . | . |
| 21-225_30H11_LC | . | . | . | . | . | . | . | . | . | . | . | . | . | . | . | . | . |
| 21-225_33C2_LC | . | . | . | . | . | . | . | . | . | . | . | . | . | . | N | . | . |
| 21-225_14B1_LC | . | . | . | . | . | . | . | . | . | . | . | . | . | . | . | . | . |
| 21-225_26B11_LC | . | . | . | . | . | . | . | . | . | . | . | . | . | . | . | . | . |
| 21-225_13D3_LC | . | . | . | . | . | . | . | . | . | . | . | . | S | . | . | . | . |
| 21-225_6C12_LC | . | . | . | . | . | . | . | . | . | . | . | . | . | . | . | . | . |

| Linear # | 67.2 | 68 | 69 | 70 | 71 | 72 | 73 | 74 | 75 | 76 | 77 | 78 | 79 | 80 | 81 | 82 | 83 |
|---|---|---|---|---|---|---|---|---|---|---|---|---|---|---|---|---|---|
| | L_FR3 | | | | | | | | | | | | | | | | |
| 21-225_14B1_LC | - | N | T | A | T | L | T | I | S | S | T | Q | A | M | D | E | A |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | L | Q | Y | N | S | - | - | - | - | - | - | - | - |
| 21-225_17C10_LC | . | . | . | . | Q | K | . | . | . | . | . | . | . | . | . | . | . |
| 21-225_20B5_LC | . | . | F | . | Q | K | . | . | . | . | . | . | . | . | . | . | . |
| 21-225_17D8_LC | V | . | . | . | Q | . | . | . | N | . | . | . | . | . | . | . | . |
| 21-225_20B8_LC | V | . | . | . | Q | . | . | . | N | . | . | . | . | . | . | . | . |
| 21-225_22H3_LC | . | . | F | . | . | . | D | . | . | . | . | . | . | . | . | . | . |
| 21-225_20C8_LC | . | . | . | . | . | . | D | . | . | . | . | . | . | . | . | . | . |
| 21-225_20G12_LC | . | . | . | . | Q | K | . | . | . | . | . | . | . | . | . | . | . |
| 21-225_23E11_LC | . | . | . | . | . | . | H | . | . | . | . | . | . | . | . | . | . |
| 21-225_20F9_LC | . | . | F | . | Q | . | . | H | . | . | . | . | . | . | . | . | . |
| 21-225_20H10_LC | . | . | . | . | . | . | H | . | . | . | . | . | . | . | . | . | . |
| 21-225_31G9_LC | . | . | . | . | . | . | D | . | . | . | . | . | . | . | . | . | . |
| 21-225_30H11_LC | . | . | . | . | . | . | D | . | . | . | . | . | . | . | . | . | . |
| 21-225_33C2_LC | . | . | . | . | . | . | H | . | . | . | . | . | . | . | . | . | . |
| 21-225_14B1_LC | . | . | . | . | . | . | H | Y | N | . | . | . | . | . | . | . | . |
| 21-225_26B11_LC | . | . | . | . | . | . | D | . | . | . | . | . | . | . | . | . | . |
| 21-225_13D3_LC | V | . | F | . | Q | . | . | H | D | . | . | . | . | . | . | . | . |
| 21-225_6C12_LC | . | . | F | . | . | . | D | . | . | . | . | . | . | . | . | . | . |

| Linear # | 84 | 85 | 86 | 87 | 88 | 89 | 90 | 91 | 92 | 92.1 | 92.2 | 92.3 | 92.4 | 92.5 | 92.6 | 92.7 | 92.8 |
|---|---|---|---|---|---|---|---|---|---|---|---|---|---|---|---|---|---|
| 21-225_14B1_LC | D | Y | Y | C | Q | A | W | D | N | - | - | - | - | - | - | - | - |

**FIGURE 56**

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_17C10_LC | . | . | . | . | . | . | . | . | . | . | . | . | . | . | A | P | . |
| 21-225_20B5_LC | . | . | . | . | . | . | . | . | . | . | . | . | . | . | A | P | . |
| 21-225_17D8_LC | . | . | . | . | . | . | . | . | . | . | . | . | . | . | . | W | . |
| 21-225_20B8_LC | . | . | . | . | . | . | . | . | . | . | . | . | . | . | . | G | . |
| 21-225_22H3_LC | . | . | . | . | . | . | . | . | . | . | . | . | . | . | . | . | . |
| 21-225_20C8_LC | . | . | . | . | . | . | . | . | . | . | . | . | . | . | . | . | . |
| 21-225_20G12_LC | . | . | . | . | . | . | . | . | . | . | . | . | . | . | A | P | . |
| 21-225_23E11_LC | . | . | . | . | . | . | . | . | . | . | . | . | . | . | . | . | . |
| 21-225_20F9_LC | . | . | . | . | . | . | . | . | . | . | . | . | . | . | . | . | . |
| 21-225_20H10_LC | . | . | . | . | . | . | . | . | . | . | . | . | . | . | . | . | . |
| 21-225_31G9_LC | . | . | . | . | . | . | . | . | . | . | . | . | . | . | . | . | . |
| 21-225_30H11_LC | . | . | . | . | . | . | . | . | . | . | . | . | . | . | . | H | . |
| 21-225_33C2_LC | . | . | . | . | . | . | . | . | . | . | . | . | . | . | . | . | . |
| 21-225_14B1_LC | . | . | . | . | . | . | . | . | . | . | . | . | . | . | . | . | . |
| 21-225_26B11_LC | . | . | . | . | . | . | . | . | . | . | . | . | . | . | . | . | . |
| 21-225_13D3_LC | . | . | . | . | . | . | . | . | . | . | . | . | . | . | . | W | . |
| 21-225_6C12_LC | . | . | . | . | . | . | . | . | . | . | . | . | . | . | . | . | . |

| Linear # | 92.9 | 92.10 | 92.11 | 92.12 | 92.13 | 92.14 | 92.15 | 92.16 | 92.17 | 92.18 | 92.19 | 92.20 | 92.21 | 92.22 | 92.23 | 93 | 94 |
|---|---|---|---|---|---|---|---|---|---|---|---|---|---|---|---|---|---|
| | | | L_CDR3 | | | | | | | | | | | | | | |
| 21-225_14B1_LC | . | . | . | . | . | . | . | . | . | . | . | . | . | . | . | S | Y |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | K_FR4 | | | | | | | | | | |
| CONSENSUS | F | T | F | G | Q | G | T | K | V | E | I | K | R |
| 21-225_17C10_LC | L | . | . | . | . | . | . | R | L | . | . | . | . |
| 21-225_20B5_LC | I | . | . | . | . | . | . | R | L | . | . | . | . |
| 21-225_17D8_LC | L | . | . | . | . | . | . | . | . | . | . | . | . |
| 21-225_20B8_LC | L | . | . | . | . | . | . | . | . | . | . | . | . |
| 21-225_22H3_LC | . | . | . | . | P | . | . | . | . | D | . | . | . |
| 21-225_20C8_LC | . | . | . | . | G | . | . | . | . | . | . | T | . |
| 21-225_20G12_LC | I | . | . | . | . | . | . | R | L | . | . | . | . |
| 21-225_23E11_LC | W | . | . | . | . | . | . | . | . | . | . | . | . |
| 21-225_20F9_LC | W | . | . | . | . | . | . | . | . | . | . | . | . |
| 21-225_20H10_LC | L | . | . | . | G | . | . | . | . | . | . | . | . |
| 21-225_31G9_LC | . | . | . | . | P | . | . | . | . | D | . | . | . |
| 21-225_30H11_LC | . | . | . | . | P | . | . | . | . | D | . | T | . |
| 21-225_33C2_LC | W | . | . | . | . | . | . | . | . | . | . | . | . |
| 21-225_14B1_LC | L | . | . | . | G | . | . | . | . | . | . | . | . |
| 21-225_26B11_LC | . | . | . | . | P | . | . | . | . | D | . | . | . |
| 21-225_13D3_LC | P | . | . | . | . | . | . | . | . | . | . | . | . |
| 21-225_6C12_LC | . | . | . | . | G | . | . | . | . | . | . | . | . |

| Linear # | 95 | 96 | 97 | 98 | 99 | 100 | 101 | 102 | 103 | 104 | 105 | 106 | 107 |
|---|---|---|---|---|---|---|---|---|---|---|---|---|---|
| | | | L_FR4 | | | | | | | | | | |
| 21-225_14B1_LC | V | V | F | G | G | G | T | K | L | T | V | L | G |

**FIGURE 56**

**Table 40**

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | K_FR1 | | | | | | | | | | | | | | | | |
| CONSENSUS | D | I | Q | M | T | Q | S | P | S | S | L | S | A | S | V | G | D |
| 21-225_23C7_LC | . | . | . | . | . | . | . | . | . | . | . | . | . | . | . | . | . |
| 21-225_23E7_LC | . | . | . | . | . | . | . | . | . | . | . | . | . | . | . | . | . |
| 21-225_30A1_LC | . | . | . | . | . | . | . | . | . | . | . | . | . | . | . | . | . |
| 21-225_25D10_LC | . | . | . | . | . | . | . | . | . | . | . | . | . | . | . | . | . |
| 21-225_33A5_LC | . | . | . | . | . | . | . | . | . | . | . | . | . | . | . | . | . |
| 21-225_26C2_LC | G | . | . | . | . | . | . | . | . | . | . | . | . | . | . | . | . |
| 21-225_26D9_LC | . | . | . | . | . | . | . | . | . | . | . | . | . | . | . | . | . |
| 21-225_31G1_LC | . | . | . | . | . | . | . | . | . | . | . | . | . | . | . | . | . |
| 21-225_10C5_LC | . | . | . | . | . | . | . | . | . | . | . | . | . | . | . | . | . |
| 21-225_18F7_LC | . | . | . | . | . | . | . | . | . | . | . | . | . | . | E | . | . |
| 21-225_20G11_LC | . | . | . | . | . | . | . | . | . | . | . | . | . | . | E | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | K_CDR1 | | | | | | | | | | |
| CONSENSUS | R | V | T | I | T | C | R | A | S | - | - | Q | G | I | S | - | - |
| 21-225_23C7_LC | . | . | . | . | . | . | . | . | . | . | . | . | . | . | G | . | . |
| 21-225_23E7_LC | . | . | . | . | . | . | . | . | . | . | . | . | . | . | . | . | . |
| 21-225_30A1_LC | . | . | . | . | . | . | . | . | . | . | . | . | . | . | . | . | . |
| 21-225_25D10_LC | . | . | . | . | . | . | . | . | . | . | . | . | . | . | . | . | . |
| 21-225_33A5_LC | . | . | . | . | . | . | . | . | . | . | . | . | . | . | . | . | . |
| 21-225_26C2_LC | . | . | . | . | . | . | . | . | . | . | . | . | . | . | . | . | . |
| 21-225_26D9_LC | . | . | . | . | . | . | . | . | . | . | . | . | . | . | N | . | . |
| 21-225_31G1_LC | . | . | . | . | . | . | . | . | . | . | . | . | . | . | N | . | . |
| 21-225_10C5_LC | . | . | . | . | . | . | . | . | . | . | . | . | . | . | . | . | . |
| 21-225_18F7_LC | . | . | . | . | . | . | . | . | . | . | . | . | D | . | . | . | . |
| 21-225_20G11_LC | . | . | . | . | . | . | . | . | . | . | . | . | D | . | . | . | . |

| Reference # | 35 | 36 | 37 | 38 | 38 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | K_FR2 | | | | | | | | |
| CONSENSUS | - | - | - | - | N | Y | L | A | W | F | Q | Q | K | P | G | K | A |
| 21-225_23C7_LC | . | . | . | . | I | . | . | . | . | . | . | . | . | . | . | . | . |
| 21-225_23E7_LC | . | . | . | . | . | . | . | . | . | . | . | . | I | . | . | . | . |
| 21-225_30A1_LC | . | . | . | . | . | . | . | . | . | . | . | . | . | . | . | . | . |
| 21-225_25D10_LC | . | . | . | . | . | . | . | . | . | . | . | . | R | . | . | . | . |
| 21-225_33A5_LC | . | . | . | . | . | . | . | . | . | . | . | . | . | . | . | . | . |
| 21-225_26C2_LC | . | . | . | . | . | . | . | . | . | . | . | . | . | . | . | . | . |
| 21-225_26D9_LC | . | . | . | . | . | . | . | . | . | . | . | . | . | . | . | . | . |
| 21-225_31G1_LC | . | . | . | . | . | . | . | . | . | . | . | . | . | . | . | . | . |
| 21-225_10C5_LC | . | . | . | . | . | . | . | . | . | . | . | . | R | S | . | . | . |
| 21-225_18F7_LC | . | . | . | . | . | . | . | . | . | . | . | . | . | . | . | . | . |
| 21-225_20G11_LC | . | . | . | . | . | . | . | . | . | . | . | . | . | . | . | . | . |

**FIGURE 56**

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | K_CDR2 | | | |
| CONSENSUS | P | K | S | L | I | S | A | - | - | - | - | - | - | - | - | A | S |
| 21-225_23C7_LC | . | . | . | . | . | . | . | . | . | . | . | . | . | . | . | . | . |
| 21-225_23E7_LC | . | . | . | . | . | Y | . | . | . | . | . | . | . | . | . | . | . |
| 21-225_30A1_LC | . | . | . | . | . | . | . | . | . | . | . | . | . | . | . | . | . |
| 21-225_25D10_LC | . | . | . | . | . | . | . | . | . | . | . | . | . | . | . | . | . |
| 21-225_33A5_LC | . | . | . | . | . | . | . | . | . | . | . | . | . | . | . | . | . |
| 21-225_26C2_LC | . | . | . | . | . | . | . | . | . | . | . | . | . | . | . | . | . |
| 21-225_26D9_LC | . | . | . | . | . | Y | . | . | . | . | . | . | . | . | . | . | . |
| 21-225_31G1_LC | . | . | . | . | . | . | S | . | . | . | . | . | . | . | . | . | . |
| 21-225_10C5_LC | . | R | . | . | . | . | . | . | . | . | . | . | . | . | . | . | . |
| 21-225_18F7_LC | . | . | . | . | . | . | . | . | . | . | . | . | . | . | . | . | . |
| 21-225_20G11_LC | . | . | . | . | . | . | . | . | . | . | . | . | . | . | . | . | . |

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | K | F | S | G | S | G | S | G | - |
| 21-225_23C7_LC | . | . | . | . | . | . | . | . | . | . | . | . | . | . | . | . | . |
| 21-225_23E7_LC | . | . | . | . | . | . | . | . | . | . | . | . | . | . | . | . | . |
| 21-225_30A1_LC | . | . | . | T | . | . | . | . | . | . | . | . | . | . | . | . | . |
| 21-225_25D10_LC | . | . | . | . | . | . | . | . | . | . | . | . | . | . | . | . | . |
| 21-225_33A5_LC | . | . | . | . | . | . | . | . | . | . | . | . | . | . | . | . | . |
| 21-225_26C2_LC | . | . | . | . | . | . | . | . | . | . | . | . | . | . | . | . | . |
| 21-225_26D9_LC | . | . | . | . | . | . | . | . | . | . | . | . | . | . | . | . | . |
| 21-225_31G1_LC | . | . | . | . | . | . | . | . | . | . | . | . | . | . | . | . | . |
| 21-225_10C5_LC | F | . | . | . | . | . | . | . | . | . | G | . | . | . | . | . | . |
| 21-225_18F7_LC | . | . | . | . | . | . | . | . | . | . | . | . | . | . | . | . | . |
| 21-225_20G11_LC | . | . | . | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | K_FR3 | | | | | | | | | | | | | |
| CONSENSUS | - | T | D | F | T | L | T | I | S | S | L | Q | P | E | D | F | A |
| 21-225_23C7_LC | . | . | E | . | . | . | . | . | . | . | . | . | . | . | . | . | . |
| 21-225_23E7_LC | . | . | . | . | . | . | . | . | . | . | . | . | . | . | . | . | . |
| 21-225_30A1_LC | . | . | . | . | . | . | . | . | . | . | . | . | . | . | . | . | . |
| 21-225_25D10_LC | . | . | . | . | . | . | S | . | . | . | . | . | . | . | . | . | . |
| 21-225_33A5_LC | . | . | . | . | . | . | . | . | . | . | . | . | . | . | . | . | . |
| 21-225_26C2_LC | . | . | . | . | . | . | . | . | . | . | . | . | . | . | . | . | . |
| 21-225_26D9_LC | . | . | . | . | . | . | . | . | . | . | . | . | . | . | . | . | . |
| 21-225_31G1_LC | . | . | . | . | . | . | . | . | . | . | . | . | . | . | . | . | . |
| 21-225_10C5_LC | . | . | . | . | . | . | . | . | . | . | . | . | A | . | . | . | . |
| 21-225_18F7_LC | . | . | . | . | . | . | . | . | . | . | . | . | . | . | . | . | . |
| 21-225_20G11_LC | . | . | . | . | . | . | . | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | Q | Q | Y | N | S | - | - | - | - | - | - | - | - |
| 21-225_23C7_LC | . | . | . | . | . | . | . | . | . | . | . | . | . | . | . | . | . |
| 21-225_23E7_LC | . | . | . | . | . | . | [illegible] | Y | . | . | . | . | . | . | . | . | . |
| 21-225_30A1_LC | . | . | . | . | . | . | . | . | . | . | . | . | . | . | . | . | . |
| 21-225_25D10_LC | . | . | . | . | . | . | . | H | T | . | . | . | . | . | . | . | . |
| 21-225_33A5_LC | . | . | . | . | . | . | . | . | . | . | . | . | . | . | . | . | . |
| 21-225_26C2_LC | . | . | . | . | . | . | . | H | . | . | . | . | . | . | . | . | . |
| 21-225_26D9_LC | . | . | . | . | . | . | [illegible] | . | . | . | . | . | . | . | . | . | . |
| 21-225_31G1_LC | . | . | F | . | . | . | . | . | . | . | . | . | . | . | . | . | . |
| 21-225_10C5_LC | . | . | . | . | . | . | . | Y | . | . | . | . | . | . | . | . | . |
| 21-225_18F7_LC | . | . | . | . | . | . | . | H | . | . | . | . | . | . | . | . | . |
| 21-225_20G11_LC | . | . | . | . | . | . | . | H | . | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_23C7_LC | . | . | . | . | . | . | . | . | . | . | . | . | . | . | . | . | . |
| 21-225_23E7_LC | . | . | . | . | . | . | . | . | . | . | . | . | . | . | . | . | . |
| 21-225_30A1_LC | . | . | . | . | . | . | . | . | . | . | . | . | . | . | . | . | . |
| 21-225_25D10_LC | . | . | . | . | . | . | . | . | . | . | . | . | . | . | . | . | . |
| 21-225_33A5_LC | . | . | . | . | . | . | . | . | . | . | . | . | . | . | . | . | . |
| 21-225_26C2_LC | . | . | . | . | . | . | . | . | . | . | . | . | . | . | . | . | . |
| 21-225_26D9_LC | . | . | . | . | . | . | . | . | . | . | . | . | . | . | . | . | . |
| 21-225_31G1_LC | . | . | . | . | . | . | . | . | . | . | . | . | . | . | . | . | . |
| 21-225_10C5_LC | . | . | . | . | . | . | . | . | . | . | . | . | . | . | . | . | . |
| 21-225_18F7_LC | . | . | . | . | . | . | . | . | . | . | . | . | . | . | . | . | . |
| 21-225_20G11_LC | . | . | . | . | . | . | . | . | . | . | . | . | . | . | . | . | . |

| Reference # | 137 | 138 | 139 | 140 | 141 | 142 | 143 | 144 | 145 | 146 | 147 | 148 | 149 | Reference # |
|---|---|---|---|---|---|---|---|---|---|---|---|---|---|---|
| | | | | | | | | K_FR4 | | | | | | |
| CONSENSUS | F | T | F | G | P | G | T | K | V | D | I | K | R | |
| 21-225_23C7_LC | . | . | . | . | . | . | . | . | . | . | . | . | . | |
| 21-225_23E7_LC | . | . | . | . | . | . | . | . | . | . | . | N | . | |
| 21-225_30A1_LC | . | . | . | . | . | . | . | . | . | . | . | . | . | |
| 21-225_25D10_LC | . | . | . | . | . | . | . | . | . | . | . | . | . | |
| 21-225_33A5_LC | . | . | . | . | . | . | . | N | . | . | . | . | . | |
| 21-225_26C2_LC | . | . | . | . | . | . | . | . | . | . | . | . | . | |
| 21-225_26D9_LC | . | . | . | . | . | . | . | . | . | . | . | . | . | |
| 21-225_31G1_LC | . | . | . | . | . | . | . | . | . | . | . | . | . | |
| 21-225_10C5_LC | . | . | . | . | . | . | . | . | . | . | . | R | . | |
| 21-225_18F7_LC | . | . | . | . | . | . | . | . | . | . | . | . | . | |

FIGURE 56

Table 41

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | K_FR1 | | | | | | | | | | | | | | | | |
| CONSENSUS | D | I | Q | M | T | Q | S | P | S | S | L | S | A | S | V | G | D |
| 21-225_35A6_LC | . | . | . | . | . | . | . | . | . | . | . | . | . | . | . | . | . |
| 21-225_30E9_LC | . | . | . | . | . | . | . | . | . | . | . | . | . | . | . | . | . |
| 21-225_31H5_LC | . | . | . | . | . | . | . | . | . | . | . | . | . | . | . | . | . |
| 21-225_31A8_LC | . | . | . | . | . | . | . | . | . | . | . | . | . | . | . | . | . |
| 21-225_30D1_LC | . | . | . | . | . | . | . | . | . | . | . | . | . | . | . | . | . |
| 21-225_31B8_LC | . | . | . | . | I | . | . | . | . | . | . | . | . | . | . | . | . |
| 21-225_30G11_LC | . | . | . | . | . | . | . | . | . | . | . | . | . | . | . | . | . |
| 21-225_30E3_LC | . | . | . | . | . | . | . | . | . | . | . | . | . | . | . | . | . |
| 21-225_31H3_LC | . | . | . | . | . | . | . | . | . | . | . | . | . | . | . | . | . |
| 21-225_32G12_LC | . | . | . | . | . | . | . | . | . | . | . | . | . | . | . | . | . |
| 21-225_34G9_LC | . | . | . | . | . | . | . | . | . | . | . | . | . | . | . | . | . |
| 21-225_36C5_LC | . | . | . | . | . | . | . | . | . | . | . | . | . | . | . | . | . |
| 21-225_33A4_LC | . | . | . | . | . | . | . | . | . | . | . | . | . | . | . | . | . |
| 21-225_33F1_LC | . | . | . | . | . | . | . | . | . | . | . | . | . | . | . | . | . |
| 21-225_33A7_LC | . | . | . | . | . | . | . | . | . | . | . | . | . | . | . | . | . |
| 21-225_34A6_LC | . | . | . | . | . | . | . | . | . | . | . | . | . | . | . | . | . |
| 21-225_35B7_LC | . | . | . | . | . | . | . | . | . | . | . | . | . | . | . | . | . |
| 21-225_34H11_LC | . | . | . | . | . | . | . | . | . | . | . | . | . | . | . | . | . |
| 21-225_33G7_LC | . | . | . | . | . | . | . | . | . | . | . | . | . | . | . | . | . |
| 21-225_35D1_LC | . | . | . | . | . | . | . | . | . | . | . | . | . | . | . | . | . |
| 21-225_35F11_LC | . | . | . | . | . | . | . | . | . | . | . | . | . | . | . | . | . |
| 21-225_34G8_LC | . | . | . | . | . | . | . | . | . | . | . | . | . | . | . | . | . |
| 21-225_36A5_LC | . | . | . | . | . | . | . | . | . | . | . | . | . | . | . | . | . |
| 21-225_24B1_LC | . | V | V | . | . | . | . | . | L | . | . | P | V | T | L | . | Q |
| 21-225_32G1_LC | . | . | . | . | . | . | . | . | . | . | . | . | . | . | . | . | . |
| 21-225_33B8_LC | . | . | . | . | . | . | . | . | . | . | . | . | . | . | . | . | . |

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | L_FR1 | | | | | | | | | | | | | | | | |
| CONSENSUS | Q | S | | L | T | Q | P | - | | S | | S | G | S | P | G | Q |
| 21-225_4G12_LC | . | . | V | . | . | . | . | . | P | . | A | . | . | T | . | . | . |
| 21-225_17F12_LC | . | . | A | . | . | . | . | . | A | . | V | . | . | . | . | . | . |
| 21-225_21H4_LC | . | . | A | . | . | . | . | . | A | . | V | . | . | . | . | . | . |
| 21-225_12D8_LC | L | P | V | . | . | . | . | . | P | . | A | . | A | L | L | . | A |

FIGURE 56

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | | K_CDR1 | |
| CONSENSUS | R | V | T | I | T | C | R | A | S | - | - | Q | D | I | R | - | - |
| 21-225_35A6_LC | . | . | . | . | . | . | . | . | . | . | . | . | . | . | . | . | . |
| 21-225_30E9_LC | . | . | . | . | . | . | . | . | . | . | . | . | . | . | . | . | . |
| 21-225_31H5_LC | . | F | . | . | S | F | . | . | . | . | . | . | . | . | . | . | . |
| 21-225_31A8_LC | . | . | . | . | . | . | . | . | . | . | . | . | . | . | . | . | . |
| 21-225_30D1_LC | . | . | . | . | . | . | . | . | . | . | . | . | . | . | . | . | . |
| 21-225_31B8_LC | . | . | . | . | . | . | . | . | . | . | . | . | . | . | . | . | . |
| 21-225_30G11_LC | . | . | . | . | . | . | . | . | . | . | . | . | . | . | . | . | . |
| 21-225_30E3_LC | . | . | . | . | . | . | . | . | . | . | . | . | . | . | . | . | . |
| 21-225_31H3_LC | . | . | . | . | . | . | . | . | . | . | . | . | . | . | . | . | . |
| 21-225_32G12_LC | . | . | . | . | . | . | . | . | . | . | . | . | . | . | . | . | . |
| 21-225_34G9_LC | . | . | . | . | . | . | . | . | . | . | . | . | . | . | . | . | . |
| 21-225_36C5_LC | . | . | . | . | . | . | . | . | . | . | . | . | . | . | . | . | . |
| 21-225_33A4_LC | . | . | . | . | . | . | . | . | . | . | . | . | . | . | . | . | . |
| 21-225_33F1_LC | . | . | . | . | . | . | . | T | . | . | . | . | . | . | . | . | . |
| 21-225_33A7_LC | . | . | . | . | . | . | . | . | . | . | . | . | . | . | . | . | . |
| 21-225_34A6_LC | . | . | . | . | . | . | . | T | . | . | . | . | . | . | . | . | . |
| 21-225_35B7_LC | . | I | . | . | . | . | . | . | . | . | . | . | . | . | . | . | . |
| 21-225_34H11_LC | . | . | . | . | . | . | . | . | . | . | . | . | . | . | . | . | . |
| 21-225_33G7_LC | . | . | . | . | . | . | . | . | . | . | . | . | G | . | . | . | . |
| 21-225_35D1_LC | . | . | . | . | . | . | . | . | . | . | . | . | . | . | . | . | . |
| 21-225_35F11_LC | . | . | . | . | . | . | . | . | . | . | . | . | . | . | . | . | . |
| 21-225_34G8_LC | . | . | . | . | . | . | . | . | . | . | . | . | . | . | . | . | . |
| 21-225_36A5_LC | . | . | . | . | . | . | . | T | . | . | . | . | . | . | . | . | . |
| 21-225_24B1_LC | P | A | S | . | S | . | . | S | . | . | . | . | S | L | V | Y | S |
| 21-225_32G1_LC | . | . | . | . | . | . | . | . | . | . | . | . | . | M | . | . | . |
| 21-225_33B8_LC | . | . | . | . | . | . | . | . | . | . | . | . | . | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | | L_CDR1 | |
| CONSENSUS | S | I | T | I | S | C | T | G | T | S | - | S | D | V | G | | |
| 21-225_4G12_LC | [illegible] | V | N | M | . | . | S | . | . | N | . | . | N | I | . | S | - |
| 21-225_17F12_LC | . | . | . | . | . | . | . | . | A | . | . | . | . | . | . | D | Y |
| 21-225_21H4_LC | . | . | . | . | . | . | . | . | . | . | . | . | . | . | . | G | Y |
| 21-225_12D8_LC | . | . | K | L | T | . | . | L | S | . | . | E | H | S | T | Y | - |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | K_FR2 | | | | | | | | |
| CONSENSUS | - | - | - | - | S | D | L | G | W | Y | Q | Q | K | P | G | K | A |
| 21-225_36A6_LC | . | . | . | . | . | . | . | . | . | . | . | . | . | . | . | . | . |
| 21-225_30E9_LC | . | . | . | . | D | . | . | . | . | . | . | . | . | . | . | . | . |
| 21-225_31H5_LC | . | . | . | . | . | . | . | . | . | . | . | . | . | . | . | . | . |
| 21-225_31A8_LC | . | . | . | . | . | . | . | . | . | . | . | . | . | . | . | . | . |
| 21-225_30D1_LC | . | . | . | . | . | . | . | . | . | . | . | . | . | . | . | . | . |
| 21-225_31B8_LC | . | . | . | . | . | . | . | . | . | . | . | . | . | . | . | . | . |
| 21-225_30G11_LC | . | . | . | . | . | . | . | . | . | . | . | . | . | . | . | . | . |
| 21-225_30E3_LC | . | . | . | . | D | . | . | . | . | . | . | . | . | . | . | . | . |
| 21-225_31H3_LC | . | . | . | . | D | . | . | . | . | . | . | . | . | . | . | . | . |
| 21-225_32G12_LC | . | . | . | . | . | . | . | . | . | . | . | . | R | . | . | . | . |
| 21-225_34G9_LC | . | . | . | . | . | . | . | . | . | . | . | . | . | . | . | . | . |
| 21-225_36C5_LC | . | . | . | . | . | . | . | . | . | . | . | . | . | . | . | . | . |
| 21-225_33A4_LC | . | . | . | . | N | . | V | . | . | . | . | . | . | . | . | . | . |
| 21-225_33F1_LC | . | . | . | . | . | . | . | . | . | . | . | . | . | . | . | . | . |
| 21-225_33A7_LC | . | . | . | . | . | . | . | . | . | . | . | . | . | . | . | . | . |
| 21-225_34A6_LC | . | . | . | . | N | . | . | . | . | . | . | . | . | . | . | . | . |
| 21-225_35B7_LC | . | . | . | . | . | . | . | . | . | . | . | . | . | . | . | . | . |
| 21-225_34H11_LC | . | . | . | . | N | . | . | . | . | . | . | . | . | . | . | . | . |
| 21-225_33G7_LC | . | . | . | . | N | . | . | . | . | . | . | . | . | . | . | . | . |
| 21-225_35D1_LC | . | . | . | . | . | . | . | . | . | . | . | . | . | . | . | . | . |
| 21-225_35F11_LC | . | . | . | . | . | . | . | . | . | . | . | . | . | . | . | . | . |
| 21-225_34G8_LC | . | . | . | . | . | . | . | . | . | . | . | . | . | . | . | . | . |
| 21-225_36A5_LC | . | . | . | . | N | . | . | . | . | . | . | . | . | . | . | . | . |
| 21-225_24B1_LC | D | . | G | N | T | Y | . | N | . | F | . | . | R | . | . | Q | S |
| 21-225_32G1_LC | . | . | . | . | . | . | . | . | . | . | . | . | . | . | . | . | . |
| 21-225_33B8_LC | . | . | . | . | . | . | . | . | . | . | . | . | . | . | . | . | . |

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | L_FR2 | | | | | | | | |
| CONSENSUS | - | - | - | - | N | | V | S | W | Y | Q | Q | H | P | G | K | A |
| 21-225_4G12_LC | . | . | . | . | Y | T | . | N | . | . | . | . | L | . | . | T | . |
| 21-225_17F12_LC | . | . | . | . | . | S | . | . | . | . | . | . | . | . | . | . | . |
| 21-225_21H4_LC | . | . | . | . | . | S | . | . | . | . | . | . | . | . | D | . | . |
| 21-225_12D8_LC | . | . | . | . | - | T | I | E | . | . | . | . | R | . | . | R | S |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | K_CDR2 | | | | |
| CONSENSUS | P | K | R | L | I | Y | A | - | - | - | - | - | - | - | - | A | S |
| 21-225_35A6_LC | . | . | . | . | . | . | . | . | . | . | . | . | . | . | . | T | . |
| 21-225_30E9_LC | . | R | . | . | . | . | G | . | . | . | . | . | . | . | . | . | F |
| 21-225_31H5_LC | H | . | . | I | . | . | . | . | . | . | . | . | . | . | . | . | . |
| 21-225_31A8_LC | . | . | . | . | . | . | . | . | . | . | . | . | . | . | . | V | . |
| 21-225_30D1_LC | . | E | . | . | . | . | . | . | . | . | . | . | . | . | . | . | . |
| 21-225_31B8_LC | . | . | . | . | . | . | G | . | . | . | . | . | . | . | . | . | . |
| 21-225_30G11_LC | . | . | . | . | . | . | . | . | . | . | . | . | . | . | . | . | . |
| 21-225_30E3_LC | . | R | . | . | . | . | . | . | . | . | . | . | . | . | . | . | F |
| 21-225_31H3_LC | . | R | . | . | . | . | . | . | . | . | . | . | . | . | . | . | F |
| 21-225_32G12_LC | . | . | . | . | . | . | . | . | . | . | . | . | . | . | . | . | . |
| 21-225_34G9_LC | . | . | . | . | . | . | . | . | . | . | . | . | . | . | . | . | . |
| 21-225_36C5_LC | . | . | . | . | . | . | . | . | . | . | . | . | . | . | . | . | . |
| 21-225_33A4_LC | . | E | . | . | . | . | . | . | . | . | . | . | . | . | . | . | . |
| 21-225_33F1_LC | . | . | . | . | . | . | . | . | . | . | . | . | . | . | . | . | . |
| 21-225_33A7_LC | . | . | . | . | . | . | V | . | . | . | . | . | . | . | . | . | . |
| 21-225_34A6_LC | . | . | . | . | . | F | . | . | . | . | . | . | . | . | . | . | . |
| 21-225_35B7_LC | . | E | . | . | . | . | . | . | . | . | . | . | . | . | . | . | . |
| 21-225_34H11_LC | . | . | . | . | . | . | . | . | . | . | . | . | . | . | . | T | . |
| 21-225_33G7_LC | . | . | . | . | . | . | . | . | . | . | . | . | . | . | . | . | . |
| 21-225_35D1_LC | . | . | . | . | . | . | . | . | . | . | . | . | . | . | . | . | . |
| 21-225_35F11_LC | . | . | . | . | . | . | . | . | . | . | . | . | . | . | . | . | . |
| 21-225_34G8_LC | . | . | . | . | . | . | . | . | . | . | . | . | . | . | . | . | . |
| 21-225_36A5_LC | . | . | . | . | . | F | . | . | . | . | . | . | . | . | . | . | . |
| 21-225_24B1_LC | . | R | . | . | . | . | K | . | . | . | . | . | . | . | . | V | . |
| 21-225_32G1_LC | . | . | . | . | . | . | . | . | . | . | . | . | . | . | . | . | . |
| 21-225_33B8_LC | . | . | . | . | . | . | . | . | . | . | . | . | . | . | . | . | . |

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | L_CDR2 | | | | |
| CONSENSUS | P | K | L | M | I | Y | E | - | - | - | - | - | - | - | - | V | S |
| 21-225_4G12_LC | . | . | . | L | . | . | I | . | . | . | . | . | . | . | . | N | N |
| 21-225_17F12_LC | . | . | . | . | . | . | . | . | . | . | . | . | . | . | . | . | . |
| 21-225_21H4_LC | . | . | . | . | . | . | . | . | . | . | . | . | . | . | . | . | . |
| 21-225_12D8_LC | . | Q | Y | I | M | K | V | K | S | . | . | . | . | D | G | S | H |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | R | F | S | G | S | G | S | G | - |
| 21-225_35A6_LC | . | . | . | . | . | . | . | . | . | . | . | . | . | . | . | . | . |
| 21-225_30E9_LC | . | . | . | . | . | . | . | . | . | . | . | . | . | . | . | . | . |
| 21-225_31H5_LC | . | . | . | . | . | . | . | . | . | . | . | V | . | . | . | . | . |
| 21-225_31A8_LC | . | . | . | . | . | . | . | . | . | . | . | . | . | . | . | . | . |
| 21-225_30D1_LC | . | . | . | . | . | . | . | . | . | . | . | . | . | . | . | . | . |
| 21-225_31B8_LC | . | . | . | . | . | . | . | . | . | . | . | . | . | . | . | . | . |
| 21-225_30G11_LC | . | . | . | . | . | . | . | . | . | . | . | . | . | . | . | . | . |
| 21-225_30E3_LC | . | . | . | . | . | . | . | . | . | . | . | . | . | . | . | . | . |
| 21-225_31H3_LC | . | . | . | . | . | . | . | . | . | . | . | . | . | . | . | . | . |
| 21-225_32G12_LC | . | . | . | . | . | . | . | . | . | . | . | . | . | . | . | . | . |
| 21-225_34G9_LC | . | . | . | . | . | . | . | . | . | . | T | . | . | . | . | . | . |
| 21-225_36C5_LC | . | . | . | . | . | . | . | . | . | . | T | . | . | . | . | . | . |
| 21-225_33A4_LC | . | . | . | P | . | . | . | . | . | . | . | . | . | . | . | . | . |
| 21-225_33F1_LC | . | . | . | . | . | . | . | . | . | . | . | . | . | . | . | . | . |
| 21-225_33A7_LC | . | . | . | . | . | . | . | . | . | . | . | . | . | . | . | . | . |
| 21-225_34A6_LC | . | . | . | . | . | . | . | . | . | . | . | . | . | . | . | . | . |
| 21-225_35B7_LC | . | . | . | . | . | . | . | . | . | . | . | . | . | . | . | . | . |
| 21-225_34H11_LC | . | . | . | . | . | . | . | . | . | . | . | . | . | . | . | . | . |
| 21-225_33G7_LC | . | . | . | . | . | . | . | . | . | . | . | . | . | . | . | . | . |
| 21-225_35D1_LC | . | . | . | . | . | . | . | . | . | . | . | . | . | . | . | . | . |
| 21-225_35F11_LC | . | . | . | . | . | . | . | . | . | . | . | . | . | . | . | . | . |
| 21-225_34G8_LC | . | . | . | . | . | . | . | . | . | . | . | . | . | . | . | . | . |
| 21-225_36A5_LC | . | . | . | . | . | . | . | . | . | . | . | . | . | . | . | . | . |
| 21-225_24B1_LC | N | W | D | . | . | . | . | D | . | . | . | . | . | . | . | . | . |
| 21-225_32G1_LC | . | . | . | . | . | . | . | . | . | . | . | . | . | . | . | . | . |
| 21-225_33B8_LC | . | . | . | . | . | . | . | . | . | . | . | . | . | . | . | . | . |

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | N | R | P | S | G | V | | | R | F | S | G | S | K | S | G | - |
| 21-225_4G12_LC | Q | . | . | . | . | . | P | D | . | . | . | . | . | . | . | . | . |
| 21-225_17F12_LC | . | . | . | . | . | . | S | N | . | . | . | . | . | . | . | . | . |
| 21-225_21H4_LC | . | . | . | . | . | . | S | N | . | . | . | . | . | . | . | . | . |
| 21-225_12D8_LC | S | K | G | D | . | I | P | D | . | . | M | . | . | S | . | . | . |

**FIGURE 56**

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | K_FR3 | | | | | | | | | | | | | | | | |
| CONSENSUS | - | T | E | F | T | L | T | I | S | S | L | Q | P | E | D | F | A |
| 21-225_35A6_LC | . | . | . | . | . | . | . | . | . | . | . | . | . | . | . | . | . |
| 21-225_30E9_LC | . | . | . | . | . | . | . | . | . | . | . | . | . | . | . | . | . |
| 21-225_31H5_LC | . | . | . | . | . | F | . | . | . | . | M | . | . | D | . | . | S |
| 21-225_31A8_LC | . | . | . | . | . | . | . | . | . | . | . | . | . | . | . | . | . |
| 21-225_30D1_LC | . | . | . | . | . | . | . | . | . | . | . | . | . | . | . | . | T |
| 21-225_31B8_LC | . | . | . | . | . | . | . | . | . | . | . | . | . | . | . | . | . |
| 21-225_30G11_LC | . | . | . | . | . | . | . | . | G | . | . | . | . | . | . | . | . |
| 21-225_30E3_LC | . | . | . | . | . | . | . | . | . | . | . | . | . | . | . | . | . |
| 21-225_31H3_LC | . | . | . | . | . | . | . | . | . | . | . | . | . | . | . | . | . |
| 21-225_32G12_LC | . | . | . | . | . | . | . | . | . | . | . | . | . | . | . | . | . |
| 21-225_34G9_LC | . | . | . | . | . | . | . | . | . | . | . | . | . | . | A | . | . |
| 21-225_36C5_LC | . | . | . | . | . | . | . | . | . | . | V | . | . | . | . | . | . |
| 21-225_33A4_LC | . | . | . | . | . | . | . | . | . | . | . | . | . | . | . | . | . |
| 21-225_33F1_LC | . | . | . | . | . | . | . | . | . | . | . | . | . | . | . | . | . |
| 21-225_33A7_LC | . | . | . | . | . | . | . | . | . | . | . | . | . | . | . | . | . |
| 21-225_34A6_LC | . | . | . | . | . | . | . | V | . | . | . | . | . | . | . | . | . |
| 21-225_35B7_LC | . | . | . | . | . | . | . | . | . | . | . | . | . | . | . | . | . |
| 21-225_34H11_LC | . | . | . | . | . | . | . | . | . | . | . | . | . | . | . | . | . |
| 21-225_33G7_LC | . | . | . | . | . | . | . | . | . | . | . | . | . | . | . | . | . |
| 21-225_35D1_LC | . | . | . | . | . | . | . | . | . | . | . | . | . | . | . | . | . |
| 21-225_35F11_LC | . | . | . | . | . | . | . | . | . | . | . | . | . | . | . | . | . |
| 21-225_34G8_LC | . | . | . | . | . | . | . | . | . | . | . | . | . | . | . | . | . |
| 21-225_36A5_LC | . | . | . | . | . | . | . | . | . | . | . | . | . | . | . | . | . |
| 21-225_24B1_LC | . | . | D | . | . | . | K | . | . | W | V | E | A | . | . | V | G |
| 21-225_32G1_LC | . | . | . | . | . | . | . | . | . | . | . | . | . | . | . | . | . |
| 21-225_33B8_LC | . | . | . | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | L_FR3 | | | | | | | | | | | | | | | | |
| CONSENSUS | - | N | T | A | S | L | T | I | S | G | L | Q | | E | D | E | A |
| 21-225_4G12_LC | . | T | S | . | . | . | A | . | . | . | . | . | S | . | . | . | . |
| 21-225_17F12_LC | . | . | . | . | . | . | . | . | . | . | . | . | A | . | . | . | . |
| 21-225_21H4_LC | . | . | . | . | . | . | . | . | . | . | . | . | A | . | . | . | . |
| 21-225_12D8_LC | . | A | D | R | Y | . | . | F | . | N | . | . | S | D | . | . | D |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | L | Q | H | T | I | - | - | - | - | - | - | - | - |
| 21-225_35A6_LC | . | . | . | . | . | . | . | . | . | . | . | . | . | . | . | . | . |
| 21-225_30E9_LC | . | . | . | . | . | . | . | . | S | . | . | . | . | . | . | . | . |
| 21-225_31H5_LC | N | . | . | . | I | . | . | I | . | . | . | . | . | . | . | . | . |
| 21-225_31A8_LC | . | . | . | . | . | . | . | . | . | . | . | . | . | . | . | . | . |
| 21-225_30D1_LC | . | . | . | . | . | . | . | . | . | . | . | . | . | . | . | . | . |
| 21-225_31B8_LC | . | . | . | . | . | . | . | I | . | . | . | . | . | . | . | . | . |
| 21-225_30G11_LC | . | . | F | . | . | . | . | . | . | . | . | . | . | . | . | . | . |
| 21-225_30E3_LC | . | . | . | . | . | . | . | . | S | . | . | . | . | . | . | . | . |
| 21-225_31H3_LC | . | . | . | . | . | . | . | . | S | . | . | . | . | . | . | . | . |
| 21-225_32G12_LC | . | . | . | . | . | . | . | . | . | . | . | . | . | . | . | . | . |
| 21-225_34G9_LC | I | . | . | . | . | . | . | . | . | . | . | . | . | . | . | . | . |
| 21-225_36C5_LC | . | . | . | . | . | H | . | P | . | . | . | . | . | . | . | . | . |
| 21-225_33A4_LC | R | . | . | . | . | . | . | Y | S | . | . | . | . | . | . | . | . |
| 21-225_33F1_LC | I | . | . | . | . | . | . | . | . | . | . | . | . | . | . | . | . |
| 21-225_33A7_LC | A | . | . | . | . | . | . | . | . | . | . | . | . | . | . | . | . |
| 21-225_34A6_LC | . | . | . | . | . | . | . | N | S | . | . | . | . | . | . | . | . |
| 21-225_35B7_LC | . | . | . | . | . | . | . | . | . | . | . | . | . | . | . | . | . |
| 21-225_34H11_LC | . | . | . | . | . | . | . | N | S | . | . | . | . | . | . | . | . |
| 21-225_33G7_LC | . | . | . | . | . | . | . | Y | S | . | . | . | . | . | . | . | . |
| 21-225_35D1_LC | . | . | . | . | . | . | . | . | . | . | . | . | . | . | . | . | . |
| 21-225_35F11_LC | . | . | . | . | . | . | . | . | V | . | . | . | . | . | . | . | . |
| 21-225_34G8_LC | . | . | . | . | . | . | . | . | . | . | . | . | . | . | . | . | . |
| 21-225_36A5_LC | . | . | . | . | . | H | . | N | S | . | . | . | . | . | . | . | . |
| 21-225_24B1_LC | V | C | . | . | M | . | G | A | H | . | . | . | . | . | . | . | . |
| 21-225_32G1_LC | . | . | . | . | . | . | . | . | . | . | . | . | . | . | . | . | . |
| 21-225_33B8_LC | . | . | F | . | . | . | . | . | . | . | . | . | . | . | . | . | . |

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | D | Y | Y | C | S | S | Y | T | | - | - | - | - | - | - | - | - |
| 21-225_4G12_LC | . | . | . | . | A | A | W | Q | D | S | . | . | . | . | . | . | . |
| 21-225_17F12_LC | . | . | . | . | . | . | . | . | S | . | . | . | . | . | . | . | . |
| 21-225_21H4_LC | . | . | . | . | . | . | . | . | R | . | . | . | . | . | . | . | . |
| 21-225_12D8_LC | E | . | H | . | G | E | S | N | T | I | D | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_35A6_LC | . | . | . | . | . | . | . | . | . | . | . | . | . | . | . | . | . |
| 21-225_30E9_LC | . | . | . | . | . | . | . | . | . | . | . | . | . | . | . | . | . |
| 21-225_31H5_LC | . | . | . | . | . | . | . | . | . | . | . | . | . | . | . | . | . |
| 21-225_31A8_LC | . | . | . | . | . | . | . | . | . | . | . | . | . | . | . | . | . |
| 21-225_30D1_LC | . | . | . | . | . | . | . | . | . | . | . | . | . | . | . | . | . |
| 21-225_31B8_LC | . | . | . | . | . | . | . | . | . | . | . | . | . | . | . | . | . |
| 21-225_30G11_LC | . | . | . | . | . | . | . | . | . | . | . | . | . | . | . | . | . |
| 21-225_30E3_LC | . | . | . | . | . | . | . | . | . | . | . | . | . | . | . | . | . |
| 21-225_31H3_LC | . | . | . | . | . | . | . | . | . | . | . | . | . | . | . | . | . |
| 21-225_32G12_LC | . | . | . | . | . | . | . | . | . | . | . | . | . | . | . | . | . |
| 21-225_34G9_LC | . | . | . | . | . | . | . | . | . | . | . | . | . | . | . | . | . |
| 21-225_36C5_LC | . | . | . | . | . | . | . | . | . | . | . | . | . | . | . | . | . |
| 21-225_33A4_LC | . | . | . | . | . | . | . | . | . | . | . | . | . | . | . | . | . |
| 21-225_33F1_LC | . | . | . | . | . | . | . | . | . | . | . | . | . | . | . | . | . |
| 21-225_33A7_LC | . | . | . | . | . | . | . | . | . | . | . | . | . | . | . | . | . |
| 21-225_34A6_LC | . | . | . | . | . | . | . | . | . | . | . | . | . | . | . | . | . |
| 21-225_35B7_LC | . | . | . | . | . | . | . | . | . | . | . | . | . | . | . | . | . |
| 21-225_34H11_LC | . | . | . | . | . | . | . | . | . | . | . | . | . | . | . | . | . |
| 21-225_33G7_LC | . | . | . | . | . | . | . | . | . | . | . | . | . | . | . | . | . |
| 21-225_35D1_LC | . | . | . | . | . | . | . | . | . | . | . | . | . | . | . | . | . |
| 21-225_35F11_LC | . | . | . | . | . | . | . | . | . | . | . | . | . | . | . | . | . |
| 21-225_34G8_LC | . | . | . | . | . | . | . | . | . | . | . | . | . | . | . | . | . |
| 21-225_36A5_LC | . | . | . | . | . | . | . | . | . | . | . | . | . | . | . | . | . |
| 21-225_24B1_LC | . | . | . | . | . | . | . | . | . | . | . | . | . | | W | F | . |
| 21-225_32G1_LC | . | . | . | . | . | . | . | . | . | . | . | . | . | . | . | S | |
| 21-225_33B8_LC | . | . | . | . | . | . | . | . | . | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | L_CDR3 | | | | | | | | | | | | | | |
| CONSENSUS | - | - | - | - | - | - | - | - | - | - | - | - | - | - | S | | T |
| 21-225_4G12_LC | . | . | . | . | . | . | . | . | . | . | . | . | . | L | N | G | H |
| 21-225_17F12_LC | . | . | . | . | . | . | . | . | . | . | . | . | . | . | . | I | . |
| 21-225_21H4_LC | . | . | . | . | . | . | . | . | . | . | . | . | . | . | . | S | . |
| 21-225_12D8_LC | . | . | . | . | . | . | . | . | . | . | . | . | . | G | Q | V | G |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | K_FR4 | | | | | | | | | | |
| CONSENSUS | P | T | F | G | G | G | T | K | V | E | I | K | R |
| 21-225_35A6_LC | . | . | . | . | . | . | . | . | . | . | . | . | . |
| 21-225_30E9_LC | . | . | . | . | . | . | . | . | . | . | . | . | . |
| 21-225_31H5_LC | . | . | . | . | . | . | . | . | . | . | . | . | . |
| 21-225_31A8_LC | . | . | . | . | . | . | . | . | . | . | . | . | . |
| 21-225_30D1_LC | . | . | . | . | . | . | . | . | . | . | . | . | . |
| 21-225_31B8_LC | . | . | . | . | . | . | . | . | . | . | . | . | . |
| 21-225_30G11_LC | . | . | . | . | . | . | . | . | . | . | . | . | . |
| 21-225_30E3_LC | . | . | . | . | . | . | . | . | . | . | . | . | . |
| 21-225_31H3_LC | . | . | . | . | . | . | . | . | . | . | . | . | . |
| 21-225_32G12_LC | . | . | . | . | . | . | . | . | . | . | . | . | . |
| 21-225_34G9_LC | . | . | . | . | . | . | . | . | . | W | . | . | . |
| 21-225_36C5_LC | . | . | . | . | . | . | . | . | . | W | . | . | . |
| 21-225_33A4_LC | . | . | . | . | . | . | . | . | . | . | . | . | . |
| 21-225_33F1_LC | . | . | . | . | . | . | . | . | . | . | . | . | . |
| 21-225_33A7_LC | . | . | . | . | . | . | . | . | . | . | . | . | . |
| 21-225_34A6_LC | . | . | . | . | . | . | . | . | . | . | . | . | . |
| 21-225_35B7_LC | . | . | . | . | . | . | . | . | . | . | . | . | . |
| 21-225_34H11_LC | . | . | . | . | . | . | . | . | . | . | . | . | . |
| 21-225_33G7_LC | . | . | . | . | . | . | . | . | . | . | . | . | . |
| 21-225_35D1_LC | . | . | . | . | . | . | . | . | . | . | . | . | . |
| 21-225_35F11_LC | . | . | . | . | . | . | . | . | . | . | . | . | . |
| 21-225_34G8_LC | . | . | . | . | . | . | . | . | . | . | . | . | . |
| 21-225_36A5_LC | . | K | . | . | . | . | I | . | . | . | . | T | G |
| 21-225_24B1_LC | I | . | . | . | Q | . | . | K | L | . | . | . | . |
| 21-225_32G1_LC | . | . | . | . | . | . | . | . | . | . | . | . | . |
| 21-225_33B8_LC | . | . | . | . | . | . | . | . | . | . | . | . | . |

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | L_FR4 | | | | | | | | | | |
| CONSENSUS | V | V | F | G | G | G | T | K | L | T | V | L | G |
| 21-225_4G12_LC | . | . | . | . | . | R | . | . | . | . | . | . | . |
| 21-225_17F12_LC | . | . | . | . | . | . | . | . | . | . | . | . | . |
| 21-225_21H4_LC | . | . | . | . | . | . | . | . | . | . | . | . | . |
| 21-225_12D8_LC | . | . | . | . | . | . | . | . | . | . | . | . | . |

FIGURE 56

Table 42

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | K_FR1 | | | | | | | | | | | | | | | | |
| CONSENSUS | D | I | Q | M | T | Q | S | P | S | S | L | S | A | S | V | G | D |
| 21-225_11A5_LC | . | . | . | . | . | . | . | . | . | . | . | . | . | . | . | . | . |
| 21-225_17C6_LC | . | . | . | . | . | . | . | . | . | . | . | . | . | . | . | . | . |
| 21-225_17B4_LC | . | . | . | . | . | . | . | . | . | . | . | . | . | . | . | . | . |
| 21-225_20A4_LC | . | . | . | . | . | . | . | . | A | . | . | . | . | . | . | . | . |
| 21-225_17F7_LC | . | . | . | . | . | . | . | . | . | . | . | . | . | . | . | . | . |
| 21-225_17F11_LC | . | . | . | . | . | . | . | . | . | . | . | . | . | . | I | . | . |
| 21-225_17A12_LC | . | . | . | . | . | . | . | . | . | . | . | . | . | . | . | . | . |
| 21-225_18E12_LC | . | . | . | . | . | . | . | . | . | . | . | . | . | . | . | . | . |
| 21-225_22G3_LC | . | . | . | . | . | . | . | . | . | . | . | . | . | . | . | . | . |
| 21-225_22G10_LC | . | . | . | . | . | . | . | . | . | . | . | . | . | . | . | . | . |
| 21-225_21F4_LC | . | . | . | . | . | . | . | . | . | . | . | . | . | . | . | . | . |
| 21-225_22E4_LC | . | . | . | . | . | . | . | . | . | . | . | . | . | . | . | . | . |
| 21-225_21A2_LC | . | . | . | . | . | . | . | . | . | . | . | . | . | . | . | . | . |
| 21-225_22C5_LC | . | . | . | . | . | . | . | . | A | . | . | . | . | . | . | . | . |
| 21-225_22D5_LC | . | . | . | . | . | . | . | . | . | . | . | . | . | . | . | . | . |
| 21-225_10F12_LC | . | . | . | . | . | . | . | . | . | . | . | . | . | . | . | . | . |
| 21-225_4H2_LC | . | . | . | . | . | L | . | . | . | . | . | . | . | . | . | . | . |
| 21-225_5G2_LC | . | . | . | . | . | . | . | . | . | . | P | . | . | . | . | . | . |
| 21-225_3E3_LC | . | . | . | . | . | . | . | . | . | . | P | . | . | . | . | . | . |
| 21-225_5F7_LC | . | . | . | . | . | . | . | . | . | . | P | . | . | . | . | . | . |
| 21-225_6D3_LC | . | . | . | . | . | . | . | . | . | . | . | . | . | . | . | . | . |
| 21-225_8G6_LC | . | . | . | . | . | . | . | . | . | . | . | . | . | . | . | . | . |
| 21-225_16H6_LC | . | . | . | . | . | . | . | . | . | . | . | . | . | . | . | . | . |
| 21-225_3B1_LC | . | . | . | . | . | . | . | . | . | . | . | . | . | . | . | . | . |
| 21-225_8E12_LC | . | . | . | . | . | . | . | . | . | . | . | . | . | . | . | . | . |

**FIGURE 56**

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | | K_CDR1 | |
| CONSENSUS | R | V | T | I | T | C | R | A | S | - | - | Q | S | I | S | - | - |
| 21-225_11A5_LC | . | . | . | . | . | . | . | . | . | . | . | . | N | . | Y | . | . |
| 21-225_17C6_LC | . | . | . | . | . | . | . | . | . | . | . | . | . | . | N | . | . |
| 21-225_17B4_LC | . | . | . | . | . | . | . | . | . | . | . | . | N | . | . | . | . |
| 21-225_20A4_LC | . | . | . | . | . | . | . | . | . | . | . | . | . | . | I | . | . |
| 21-225_17F7_LC | . | . | . | . | . | . | . | . | . | . | . | . | T | . | . | . | . |
| 21-225_17F11_LC | . | . | S | . | . | . | . | . | . | . | . | [illegible] | T | . | N | . | . |
| 21-225_17A12_LC | . | . | . | . | . | . | . | . | . | . | . | . | . | . | . | . | . |
| 21-225_18E12_LC | . | . | . | . | . | . | . | . | . | . | . | . | . | . | . | . | . |
| 21-225_22G3_LC | . | . | . | . | . | . | . | . | . | . | . | . | . | . | . | . | . |
| 21-225_22G10_LC | . | . | . | . | . | . | . | . | . | . | . | . | . | . | F | . | . |
| 21-225_21F4_LC | . | . | . | . | . | . | . | . | . | . | . | . | N | . | G | . | . |
| 21-225_22E4_LC | . | . | . | . | . | . | . | . | . | . | . | . | N | . | . | . | . |
| 21-225_21A2_LC | . | . | . | . | . | . | . | . | . | . | . | . | . | . | . | . | . |
| 21-225_22C5_LC | . | . | . | . | . | . | . | . | . | . | . | . | N | . | . | . | . |
| 21-225_22D5_LC | . | . | . | . | . | . | . | . | . | . | . | . | N | . | F | . | . |
| 21-225_10F12_LC | . | . | . | . | . | . | . | . | . | . | . | . | N | . | . | . | . |
| 21-225_4H2_LC | . | . | . | . | . | . | . | . | I | . | . | . | N | . | . | . | . |
| 21-225_5G2_LC | . | . | . | . | . | F | . | . | . | . | . | . | N | . | I | . | . |
| 21-225_3E3_LC | . | . | . | . | . | F | . | . | . | . | . | . | . | . | [illegible] | . | . |
| 21-225_5F7_LC | . | . | . | . | . | F | . | . | . | . | . | . | T | . | . | . | . |
| 21-225_6D3_LC | . | . | . | . | . | . | . | T | . | . | . | . | . | . | . | . | . |
| 21-225_8G6_LC | . | . | . | . | . | . | . | . | . | . | . | . | . | . | . | . | . |
| 21-225_16H6_LC | . | . | . | . | . | . | . | . | . | . | . | . | N | . | . | . | . |
| 21-225_3B1_LC | . | . | . | . | . | . | . | . | . | . | . | . | . | . | N | . | . |
| 21-225_8E12_LC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | | K_FR2 | |
| CONSENSUS | - | - | - | - | S | Y | L | N | W | Y | Q | Q | K | P | G | K | A |
| 21-225_11A5_LC | . | . | . | . | . | . | . | . | . | . | . | . | . | . | . | . | . |
| 21-225_17C6_LC | . | . | . | . | Y | . | . | . | . | . | . | . | . | . | . | . | . |
| 21-225_17B4_LC | . | . | . | . | . | . | . | . | . | . | . | . | . | . | . | . | . |
| 21-225_20A4_LC | . | . | . | . | N | . | . | . | . | . | . | . | [illegible] | . | . | . | S |
| 21-225_17F7_LC | . | . | . | . | . | F | . | . | . | . | . | . | . | . | . | . | . |
| 21-225_17F11_LC | . | . | . | . | N | . | . | . | . | . | . | . | . | . | . | . | . |
| 21-225_17A12_LC | . | . | . | . | . | . | . | . | . | . | [illegible] | . | . | . | . | . | . |
| 21-225_18E12_LC | . | . | . | . | . | . | . | . | . | . | . | . | . | . | . | . | . |
| 21-225_22G3_LC | . | . | . | . | N | . | . | . | . | . | . | . | . | . | . | . | . |
| 21-225_22G10_LC | . | . | . | . | . | . | . | . | . | . | . | . | . | . | . | . | . |
| 21-225_21F4_LC | . | . | . | . | . | . | . | . | . | . | . | . | . | . | . | . | . |
| 21-225_22E4_LC | . | . | . | . | . | . | . | . | . | . | . | . | . | . | . | . | . |
| 21-225_21A2_LC | . | . | . | . | . | . | . | . | . | . | . | . | . | . | . | . | . |
| 21-225_22C5_LC | . | . | . | . | . | . | . | . | . | . | . | . | . | . | . | . | . |
| 21-225_22D5_LC | . | . | . | . | . | . | . | . | . | . | [illegible] | . | . | . | . | . | . |
| 21-225_10F12_LC | . | . | . | . | . | . | . | . | . | . | . | . | . | . | . | . | . |
| 21-225_4H2_LC | . | . | . | . | . | . | . | . | . | . | . | . | . | . | . | . | . |
| 21-225_5G2_LC | . | . | . | . | . | . | . | . | . | . | . | . | . | . | . | . | . |
| 21-225_3E3_LC | . | . | . | . | . | . | . | . | . | . | . | . | . | . | . | . | . |
| 21-225_5F7_LC | . | . | . | . | . | . | . | . | . | . | . | . | . | . | . | . | . |
| 21-225_6D3_LC | . | . | . | . | Y | . | . | . | . | . | . | . | . | . | . | . | . |
| 21-225_8G6_LC | . | . | . | . | . | . | . | . | . | . | . | . | . | . | . | . | . |
| 21-225_16H6_LC | . | . | . | . | . | . | . | . | . | . | . | . | . | . | . | . | . |
| 21-225_3B1_LC | . | . | . | . | N | . | . | . | . | . | . | . | . | . | . | . | . |
| 21-225_8E12_LC | . | . | . | . | N | . | . | . | . | . | . | . | . | . | . | . | . |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | K_CDR2 | | | |
| CONSENSUS | P | K | L | L | I | Y | A | - | - | - | - | - | - | - | - | A | S |
| 21-225_11A5_LC | . | . | . | . | . | . | . | . | . | . | . | . | . | . | . | . | . |
| 21-225_17C6_LC | . | . | . | . | . | . | . | . | . | . | . | . | . | . | . | . | . |
| 21-225_17B4_LC | . | . | . | . | . | F | G | . | . | . | . | . | . | . | . | . | . |
| 21-225_20A4_LC | . | . | . | . | . | . | . | . | . | . | . | . | . | . | . | . | . |
| 21-225_17F7_LC | . | . | . | . | . | . | . | . | . | . | . | . | . | . | . | . | . |
| 21-225_17F11_LC | . | . | . | . | . | F | . | . | . | . | . | . | . | . | . | T | . |
| 21-225_17A12_LC | . | . | . | . | . | . | . | . | . | . | . | . | . | . | . | . | . |
| 21-225_18E12_LC | . | T | . | . | . | . | . | . | . | . | . | . | . | . | . | . | . |
| 21-225_22G3_LC | . | . | . | . | . | F | . | . | . | . | . | . | . | . | . | . | . |
| 21-225_22G10_LC | . | . | . | . | . | . | . | . | . | . | . | . | . | . | . | . | . |
| 21-225_21F4_LC | . | . | . | . | . | F | . | . | . | . | . | . | . | . | . | . | . |
| 21-225_22E4_LC | . | . | . | . | . | . | . | . | . | . | . | . | . | . | . | . | . |
| 21-225_21A2_LC | . | . | . | . | . | . | . | . | . | . | . | . | . | . | . | . | . |
| 21-225_22C5_LC | . | . | . | . | . | . | . | . | . | . | . | . | . | . | . | . | . |
| 21-225_22D5_LC | . | . | . | . | . | . | . | . | . | . | . | . | . | . | . | . | . |
| 21-225_10F12_LC | . | . | . | . | . | . | . | . | . | . | . | . | . | . | . | . | . |
| 21-225_4H2_LC | . | . | . | V | . | . | . | . | . | . | . | . | . | . | . | . | . |
| 21-225_5G2_LC | R | . | . | V | . | . | G | . | . | . | . | . | . | . | . | . | . |
| 21-225_3E3_LC | N | . | . | V | . | . | G | . | . | . | . | . | . | . | . | T | . |
| 21-225_5F7_LC | . | . | . | . | . | S | . | . | . | . | . | . | . | . | . | . | . |
| 21-225_6D3_LC | . | . | . | . | . | F | . | . | . | . | . | . | . | . | . | . | . |
| 21-225_8G6_LC | . | . | . | . | . | F | . | . | . | . | . | . | . | . | . | . | . |
| 21-225_16H6_LC | . | . | . | . | . | . | . | . | . | . | . | . | . | . | . | . | . |
| 21-225_3B1_LC | . | . | . | . | . | . | . | . | . | . | . | . | . | . | . | T | . |
| 21-225_8E12_LC | . | . | . | . | . | . | . | . | . | . | . | . | . | . | . | . | . |

**FIGURE 56**

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | R | F | S | G | S | G | S | G | - |
| 21-225_11A5_LC | . | . | . | . | . | . | . | . | . | . | . | . | . | . | . | . | . |
| 21-225_17C6_LC | . | . | . | . | . | . | . | . | . | . | . | . | . | . | . | . | . |
| 21-225_17B4_LC | . | . | . | T | . | . | . | . | . | . | . | . | . | . | . | . | . |
| 21-225_20A4_LC | . | . | [illegible] | . | . | . | . | . | . | . | . | . | [illegible] | . | . | . | . |
| 21-225_17F7_LC | . | . | . | . | . | . | . | . | . | . | . | . | . | . | . | . | . |
| 21-225_17F11_LC | . | . | . | . | . | . | . | . | . | . | . | . | . | . | . | . | . |
| 21-225_17A12_LC | . | . | . | . | . | . | . | . | . | . | . | . | . | . | . | . | . |
| 21-225_18E12_LC | . | . | . | . | . | . | . | . | . | . | . | . | . | . | . | . | . |
| 21-225_22G3_LC | . | . | . | . | . | . | . | . | . | . | . | . | . | . | . | . | . |
| 21-225_22G10_LC | . | . | . | . | . | . | . | . | . | . | . | . | . | . | . | . | . |
| 21-225_21F4_LC | . | . | . | . | . | . | . | . | . | . | . | . | . | . | . | . | . |
| 21-225_22E4_LC | . | . | . | . | . | . | . | . | . | . | . | . | . | . | . | . | . |
| 21-225_21A2_LC | . | . | . | . | . | . | . | . | . | . | . | . | . | . | . | . | . |
| 21-225_22C5_LC | V | . | . | [illegible] | . | I | . | . | . | . | . | . | [illegible] | . | . | . | . |
| 21-225_22D5_LC | . | . | . | . | . | . | . | . | . | . | . | . | . | . | . | . | . |
| 21-225_10F12_LC | . | . | . | . | . | . | . | . | . | . | . | . | . | . | . | . | . |
| 21-225_4H2_LC | . | . | . | . | . | . | . | . | . | . | . | . | [illegible] | . | . | . | . |
| 21-225_5G2_LC | . | . | . | . | . | . | . | . | . | . | . | . | . | . | . | . | . |
| 21-225_3E3_LC | . | . | . | . | . | . | . | . | . | . | . | . | . | . | . | . | . |
| 21-225_5F7_LC | . | . | . | . | . | . | . | . | . | . | . | . | . | . | . | . | . |
| 21-225_6D3_LC | . | . | . | . | . | . | . | . | . | . | . | . | . | . | . | . | . |
| 21-225_8G6_LC | . | . | . | . | . | . | . | . | . | . | . | . | . | . | . | . | . |
| 21-225_16H6_LC | . | . | . | . | . | . | . | . | . | . | . | . | [illegible] | . | . | . | . |
| 21-225_3B1_LC | . | . | . | N | . | . | . | . | . | . | . | . | . | . | . | . | . |
| 21-225_8E12_LC | . | . | . | . | . | . | . | . | . | . | . | . | . | . | . | . | . |

FIGURE 56

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | K_FR3 | | | | | | | | | | | | | | | | |
| CONSENSUS | - | T | D | F | T | L | T | I | S | S | L | Q | P | E | D | F | A |
| 21-225_11A5_LC | . | . | . | . | . | . | . | . | . | . | . | . | . | . | . | . | . |
| 21-225_17C6_LC | . | . | . | . | . | . | . | . | . | . | . | . | . | . | . | . | . |
| 21-225_17B4_LC | . | . | . | . | . | . | . | . | N | . | . | . | . | . | . | . | . |
| 21-225_20A4_LC | . | . | . | . | . | . | . | . | . | . | . | . | . | . | . | . | . |
| 21-225_17F7_LC | . | . | . | . | . | . | . | . | . | G | . | . | . | . | . | . | . |
| 21-225_17F11_LC | . | . | . | . | . | . | . | . | . | . | . | . | . | . | . | . | . |
| 21-225_17A12_LC | . | . | . | . | . | . | . | . | . | N | . | . | . | . | . | . | . |
| 21-225_18E12_LC | . | . | . | . | . | . | . | . | N | . | . | . | . | . | . | . | . |
| 21-225_22G3_LC | . | . | . | . | . | . | . | . | . | G | . | . | . | . | . | . | . |
| 21-225_22G10_LC | . | . | . | . | . | . | . | . | . | . | . | . | . | . | . | . | . |
| 21-225_21F4_LC | . | . | . | . | . | . | . | . | . | . | . | . | . | . | . | . | . |
| 21-225_22E4_LC | . | . | . | . | . | . | . | . | . | . | . | . | . | . | . | . | . |
| 21-225_21A2_LC | . | . | . | . | . | . | . | . | . | . | . | . | . | . | . | . | . |
| 21-225_22C5_LC | . | . | . | . | . | . | I | . | . | . | . | . | . | . | . | . | . |
| 21-225_22D5_LC | . | . | . | . | . | . | . | . | . | . | . | . | . | . | . | . | . |
| 21-225_10F12_LC | . | . | . | . | . | . | . | . | . | . | . | . | . | . | . | . | . |
| 21-225_4H2_LC | . | . | . | . | . | . | . | . | G | G | V | . | R | . | . | . | . |
| 21-225_5G2_LC | . | . | . | . | . | . | N | . | R | . | . | . | . | . | . | . | . |
| 21-225_3E3_LC | . | . | . | . | . | . | . | . | . | . | . | . | . | . | . | . | . |
| 21-225_5F7_LC | . | . | . | . | . | . | . | . | . | . | . | . | . | . | . | . | . |
| 21-225_6D3_LC | . | . | . | . | . | . | . | . | . | . | . | . | . | . | . | . | . |
| 21-225_8G6_LC | . | . | . | . | . | . | . | . | . | . | . | . | . | . | . | . | . |
| 21-225_16H6_LC | . | . | . | . | . | . | . | . | . | . | . | . | . | . | . | . | . |
| 21-225_3B1_LC | . | . | . | . | . | . | . | . | . | . | . | . | . | . | . | . | . |
| 21-225_8E12_LC | . | . | . | . | . | . | . | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | Q | Q | S | Y | R | - | - | - | - | - | - | - | - |
| 21-225_11A5_LC | . | . | . | . | . | . | . | . | N | . | . | . | . | . | . | . | . |
| 21-225_17C6_LC | . | . | F | . | . | . | . | . | . | . | . | . | . | . | . | . | . |
| 21-225_17B4_LC | . | . | F | . | . | . | . | . | . | . | . | . | . | . | . | . | . |
| 21-225_20A4_LC | . | . | F | . | . | . | . | . | . | . | . | . | . | . | . | . | . |
| 21-225_17F7_LC | . | . | F | . | . | . | . | . | . | . | . | . | . | . | . | . | . |
| 21-225_17F11_LC | . | . | . | . | . | . | T | . | S | . | . | . | . | . | . | . | . |
| 21-225_17A12_LC | . | . | . | . | . | . | . | . | N | . | . | . | . | . | . | . | . |
| 21-225_18E12_LC | . | . | F | . | . | . | . | . | . | . | . | . | . | . | . | . | . |
| 21-225_22G3_LC | . | . | F | . | . | . | . | F | . | . | . | . | . | . | . | . | . |
| 21-225_22G10_LC | . | . | F | . | . | . | . | F | . | . | . | . | . | . | . | . | . |
| 21-225_21F4_LC | . | F | F | . | . | . | . | . | . | . | . | . | . | . | . | . | . |
| 21-225_22E4_LC | . | . | . | . | . | . | . | . | N | . | . | . | . | . | . | . | . |
| 21-225_21A2_LC | . | . | F | . | . | . | . | . | . | . | . | . | . | . | . | . | . |
| 21-225_22C5_LC | . | . | . | . | . | . | . | . | . | . | . | . | . | . | . | . | . |
| 21-225_22D5_LC | . | . | . | . | . | . | . | . | S | . | . | . | . | . | . | . | . |
| 21-225_10F12_LC | . | . | . | . | . | . | . | . | N | . | . | . | . | . | . | . | . |
| 21-225_4H2_LC | . | . | . | . | . | . | . | . | . | . | . | . | . | . | . | . | . |
| 21-225_5G2_LC | . | . | . | . | . | . | . | . | S | . | . | . | . | . | . | . | . |
| 21-225_3E3_LC | . | . | . | . | . | . | . | . | S | . | . | . | . | . | . | . | . |
| 21-225_5F7_LC | . | . | . | . | . | . | . | . | N | . | . | . | . | . | . | . | . |
| 21-225_6D3_LC | . | . | F | . | . | . | . | . | . | . | . | . | . | . | . | . | . |
| 21-225_8G6_LC | . | . | F | . | . | . | . | . | . | . | . | . | . | . | . | . | . |
| 21-225_16H6_LC | . | . | . | . | . | . | . | . | . | . | . | . | . | . | . | . | . |
| 21-225_3B1_LC | . | . | . | . | . | . | . | . | S | . | . | . | . | . | . | . | . |
| 21-225_8E12_LC | . | . | F | . | . | . | . | . | . | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | K_CDR3 | | | | | | | | | | | | | | | | |
| CONSENSUS | - | - | - | - | - | - | - | - | - | - | - | - | - | - | T | P | L |
| 21-225_11A5_LC | . | . | . | . | . | . | . | . | . | . | . | . | . | . | . | . | . |
| 21-225_17C6_LC | . | . | . | . | . | . | . | . | . | . | . | . | . | . | . | . | [illegible] |
| 21-225_17B4_LC | . | . | . | . | . | . | . | . | . | . | . | . | . | . | . | . | [illegible] |
| 21-225_20A4_LC | . | . | . | . | . | . | . | . | . | . | . | . | . | . | . | . | . |
| 21-225_17F7_LC | . | . | . | . | . | . | . | . | . | . | . | . | . | . | . | . | [illegible] |
| 21-225_17F11_LC | . | . | . | . | . | . | . | . | . | . | . | . | . | . | . | . | . |
| 21-225_17A12_LC | . | . | . | . | . | . | . | . | . | . | . | . | . | . | . | . | . |
| 21-225_18E12_LC | . | . | . | . | . | . | . | . | . | . | . | . | . | . | . | . | [illegible] |
| 21-225_22G3_LC | . | . | . | . | . | . | . | . | . | . | . | . | . | . | . | . | [illegible] |
| 21-225_22G10_LC | . | . | . | . | . | . | . | . | . | . | . | . | . | . | . | . | . |
| 21-225_21F4_LC | . | . | . | . | . | . | . | . | . | . | . | . | . | . | . | . | [illegible] |
| 21-225_22E4_LC | . | . | . | . | . | . | . | . | . | . | . | . | . | . | . | . | . |
| 21-225_21A2_LC | . | . | . | . | . | . | . | . | . | . | . | . | . | . | . | . | [illegible] |
| 21-225_22C5_LC | . | . | . | . | . | . | . | . | . | . | . | . | . | . | . | . | . |
| 21-225_22D5_LC | . | . | . | . | . | . | . | . | . | . | . | . | . | . | . | . | . |
| 21-225_10F12_LC | . | . | . | . | . | . | . | . | . | . | . | . | . | . | . | . | [illegible] |
| 21-225_4H2_LC | . | . | . | . | . | . | . | . | . | . | . | . | . | . | . | . | . |
| 21-225_5G2_LC | . | . | . | . | . | . | . | . | . | . | . | . | . | . | . | . | . |
| 21-225_3E3_LC | . | . | . | . | . | . | . | . | . | . | . | . | . | . | . | . | . |
| 21-225_5F7_LC | . | . | . | . | . | . | . | . | . | . | . | . | . | . | . | . | . |
| 21-225_6D3_LC | . | . | . | . | . | . | . | . | . | . | . | . | . | . | . | . | [illegible] |
| 21-225_8G6_LC | . | . | . | . | . | . | . | . | . | . | . | . | . | . | . | . | [illegible] |
| 21-225_16H6_LC | . | . | . | . | . | . | . | . | . | . | . | . | . | . | . | . | . |
| 21-225_3B1_LC | . | . | . | . | . | . | . | . | . | . | . | . | . | . | . | . | . |
| 21-225_8E12_LC | . | . | . | . | . | . | . | . | . | . | . | . | . | . | . | . | [illegible] |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | K_FR4 | | | | | | | | | | |
| CONSENSUS | F | T | F | G | P | G | T | K | V | D | I | K | R |
| 21-225_11A5_LC | . | . | . | . | . | . | . | . | . | . | . | . | . |
| 21-225_17C6_LC | . | . | . | . | . | . | . | . | . | . | . | . | . |
| 21-225_17B4_LC | . | . | . | . | . | . | . | . | . | . | . | . | . |
| 21-225_20A4_LC | . | . | . | . | . | . | . | . | . | . | F | . | . |
| 21-225_17F7_LC | . | . | . | . | . | . | . | . | . | . | . | . | . |
| 21-225_17F11_LC | . | A | . | . | . | . | . | . | . | . | . | . | . |
| 21-225_17A12_LC | . | . | . | . | . | . | . | . | . | . | . | . | . |
| 21-225_18E12_LC | . | . | . | . | . | . | . | . | . | . | . | . | . |
| 21-225_22G3_LC | . | . | . | . | . | . | . | . | . | . | . | . | . |
| 21-225_22G10_LC | . | . | . | . | . | . | . | . | . | . | F | . | . |
| 21-225_21F4_LC | . | . | . | . | . | . | . | . | . | . | F | . | . |
| 21-225_22E4_LC | . | . | . | . | . | . | . | . | . | . | . | . | . |
| 21-225_21A2_LC | . | . | . | . | . | . | . | . | . | . | . | . | . |
| 21-225_22C5_LC | . | . | . | . | . | . | . | . | . | . | F | . | . |
| 21-225_22D5_LC | . | . | . | . | . | . | . | . | . | . | . | . | . |
| 21-225_10F12_LC | . | . | . | . | . | . | . | . | . | . | . | . | . |
| 21-225_4H2_LC | . | . | . | . | . | . | . | . | . | . | . | . | . |
| 21-225_5G2_LC | . | . | . | . | . | . | . | . | . | . | . | . | . |
| 21-225_3E3_LC | . | . | . | . | . | . | . | . | . | . | . | . | . |
| 21-225_5F7_LC | . | . | . | . | . | . | . | . | . | V | . | . | . |
| 21-225_6D3_LC | . | . | . | . | . | . | . | . | . | . | . | . | . |
| 21-225_8G6_LC | . | . | . | . | . | . | . | . | . | . | . | . | . |
| 21-225_16H6_LC | . | . | . | . | . | . | . | . | . | . | F | . | . |
| 21-225_3B1_LC | . | . | . | . | . | . | . | . | . | . | . | N | . |
| 21-225_8E12_LC | . | . | . | . | . | . | . | . | . | . | . | . | . |

FIGURE 56

Table 43

| Reference # | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|
| | K_FR1 | | | | | | | | | | | | | | | |
| CONSENSUS | D | I | Q | M | T | Q | S | P | S | S | L | S | A | S | V | G |
| 21-225_20F6_LC | . | . | . | . | . | . | . | . | . | . | . | . | . | . | . | . |
| 21-225_18G4_LC | . | . | H | . | . | . | . | . | . | . | . | . | . | . | . | . |
| 21-225_22C7_LC | . | . | . | . | . | . | . | . | . | . | . | . | . | . | . | . |
| 21-225_27A11_LC | . | . | . | . | . | . | . | . | . | . | V | . | . | . | . | . |
| 21-225_26G11_LC | . | . | V | . | . | . | T | . | L | . | . | . | V | T | P | . |
| 21-225_26D12_LC | . | . | . | . | . | . | . | . | . | . | V | . | . | . | . | . |
| 21-225_25B3_LC | . | . | . | . | . | . | . | . | . | . | V | . | . | . | . | . |
| 21-225_25F3_LC | . | . | . | . | . | . | . | . | . | . | V | . | . | . | . | . |
| 21-225_3F8_LC | . | F | . | . | . | . | . | . | . | . | . | . | . | . | . | . |
| 21-225_7E11_LC | . | . | . | . | . | . | . | . | . | . | . | . | . | . | . | . |
| 21-225_6G1_LC | . | . | . | . | . | . | . | . | . | . | . | . | . | . | . | . |
| 21-225_15A3_LC | . | . | . | . | . | . | . | . | . | . | . | . | . | . | . | . |
| 21-225_13D4_LC | . | L | . | . | . | . | . | . | . | . | . | . | . | . | . | . |
| 21-225_16E6_LC | . | . | . | . | . | . | . | . | . | . | . | . | . | . | . | . |

| Reference # | 17 | 18 | 19 | 20 | 21 | 22 | 23 | 24 | 25 | 26 | 27 | 28 | 29 | 30 | 31 | 32 |
|---|---|---|---|---|---|---|---|---|---|---|---|---|---|---|---|---|
| | | | | | | | | | | | | | | | | K |
| CONSENSUS | D | R | V | T | I | T | C | R | A | S | - | - | Q | S | I | I |
| 21-225_20F6_LC | . | . | . | . | . | . | . | . | . | . | . | . | . | . | . | . |
| 21-225_18G4_LC | . | . | . | . | . | . | . | . | . | . | . | . | . | . | . | . |
| 21-225_22C7_LC | . | . | . | . | . | . | . | . | . | . | . | . | . | N | . | . |
| 21-225_27A11_LC | . | . | . | . | . | . | . | . | . | . | . | . | . | G | . | S |
| 21-225_26G11_LC | Q | P | A | S | . | S | . | K | S | . | . | . | . | T | L | L |
| 21-225_26D12_LC | . | . | . | . | . | . | . | . | . | . | . | . | . | G | . | S |
| 21-225_25B3_LC | . | . | . | . | . | . | . | . | . | . | . | . | . | G | . | S |
| 21-225_25F3_LC | . | . | . | . | . | . | . | . | . | . | . | . | . | G | . | S |
| 21-225_3F8_LC | . | . | . | . | . | . | . | . | . | . | . | . | . | . | . | L |
| 21-225_7E11_LC | . | . | . | . | . | A | . | . | . | . | . | . | . | N | . | . |
| 21-225_6G1_LC | . | . | . | . | . | . | . | . | T | . | . | . | . | N | . | . |
| 21-225_15A3_LC | . | . | . | . | . | . | . | . | . | . | . | . | . | . | | |
| 21-225_13D4_LC | . | . | . | . | . | . | . | . | . | . | . | . | . | . | . | F |
| 21-225_16E6_LC | . | . | . | . | . | . | . | . | . | . | . | . | . | . | . | . |

FIGURE 56

| Reference # | 33 | 34 | 35 | 36 | 37 | 38 | 39 | 40 | 41 | 42 | 43 | 44 | 45 | 46 | 47 | 48 |
|---|---|---|---|---|---|---|---|---|---|---|---|---|---|---|---|---|
| | CDR1 | | | | | | | | | | | | | | | |
| CONSENSUS | - | - | - | - | - | - | S | Y | L | N | W | Y | Q | Q | K | P |
| 21-225_20F6_LC | . | . | . | . | . | . | T | . | . | . | . | . | . | . | . | . |
| 21-225_18G4_LC | . | . | . | . | . | . | . | . | . | . | . | . | . | . | . | . |
| 21-225_22C7_LC | . | . | . | . | . | . | . | . | . | . | . | . | . | . | . | . |
| 21-225_27A11_LC | . | . | . | . | . | . | . | W | . | A | . | . | . | . | . | . |
| 21-225_26G11_LC | H | G | E | . | G | K | T | . | . | Y | . | . | L | . | . | . |
| 21-225_26D12_LC | . | . | . | . | . | . | . | W | . | A | . | . | . | . | . | . |
| 21-225_25B3_LC | . | . | . | . | . | . | . | W | . | A | . | . | . | . | . | . |
| 21-225_25F3_LC | . | . | . | . | . | . | . | W | . | A | . | . | . | . | . | . |
| 21-225_3F8_LC | . | . | . | . | . | . | . | . | . | . | . | . | R | . | . | . |
| 21-225_7E11_LC | . | . | . | . | . | . | . | . | . | . | . | . | . | . | . | . |
| 21-225_6G1_LC | . | . | . | . | . | . | . | . | . | . | . | . | . | . | . | . |
| 21-225_15A3_LC | . | . | . | . | . | . | . | . | . | . | . | . | L | . | . | . |
| 21-225_13D4_LC | . | . | . | . | . | . | . | . | . | . | . | . | . | . | . | . |
| 21-225_16E6_LC | . | . | . | . | . | . | . | . | . | . | . | . | . | . | . | . |

| Reference # | 49 | 50 | 51 | 52 | 53 | 54 | 55 | 56 | 57 | 58 | 59 | 60 | 61 | 62 | 63 | 64 |
|---|---|---|---|---|---|---|---|---|---|---|---|---|---|---|---|---|
| | K_FR2 | | | | | | | | | | | | | | | K |
| CONSENSUS | G | K | A | P | K | L | L | I | Y | T | - | - | - | - | - | - |
| 21-225_20F6_LC | . | . | . | . | . | V | . | . | H | . | . | . | . | . | . | . |
| 21-225_18G4_LC | . | . | . | . | . | V | . | . | . | . | . | . | . | . | . | . |
| 21-225_22C7_LC | E | . | . | . | . | . | . | . | H | I | . | . | . | . | . | . |
| 21-225_27A11_LC | . | . | . | . | . | . | . | . | . | G | . | . | . | . | . | . |
| 21-225_26G11_LC | . | Q | P | . | H | . | . | . | . | E | . | . | . | . | . | . |
| 21-225_26D12_LC | . | . | . | . | . | . | . | . | . | G | . | . | . | . | . | . |
| 21-225_25B3_LC | . | . | . | . | . | . | . | . | . | G | . | . | . | . | . | . |
| 21-225_25F3_LC | . | . | . | . | . | . | . | . | . | G | . | . | . | . | . | . |
| 21-225_3F8_LC | . | R | . | . | Q | V | . | . | H | . | . | . | . | . | . | . |
| 21-225_7E11_LC | . | . | . | . | . | F | . | . | . | . | . | . | . | . | . | . |
| 21-225_6G1_LC | . | . | . | . | . | V | . | . | . | . | . | . | . | . | . | . |
| 21-225_15A3_LC | . | . | . | . | . | F | . | . | . | . | . | . | . | . | . | . |
| 21-225_13D4_LC | . | . | . | . | . | . | . | . | C | . | . | . | . | . | . | . |
| 21-225_16E6_LC | . | . | . | . | . | F | . | . | . | . | . | . | . | . | . | . |

FIGURE 56

| Reference # | 65 | 66 | 67 | 68 | 69 | 70 | 71 | 72 | 73 | 74 | 75 | 76 | 77 | 78 | 79 | 80 |
|---|---|---|---|---|---|---|---|---|---|---|---|---|---|---|---|---|
| | CDR2 | | | | | | | | | | | | | | | |
| CONSENSUS | - | - | A | S | S | L | Q | S | G | V | P | S | R | F | S | G |
| 21-225_20F6_LC | . | . | . | . | . | . | . | . | . | . | . | . | . | . | . | . |
| 21-225_18G4_LC | . | . | . | . | . | . | . | T | . | . | . | . | G | . | . | . |
| 21-225_22C7_LC | . | . | . | . | . | . | . | . | . | . | . | . | . | . | . | . |
| 21-225_27A11_LC | . | . | . | . | . | . | . | . | . | . | . | . | . | . | . | A |
| 21-225_26G11_LC | . | . | V | . | N | R | F | . | . | . | . | D | . | . | . | . |
| 21-225_26D12_LC | . | . | . | . | . | . | . | . | . | . | . | . | . | . | . | A |
| 21-225_25B3_LC | . | . | . | . | . | . | . | . | . | . | . | . | . | . | . | A |
| 21-225_25F3_LC | . | . | . | . | . | . | . | . | . | . | . | . | . | . | . | A |
| 21-225_3F8_LC | . | . | T | . | . | . | . | . | . | . | . | . | . | . | . | . |
| 21-225_7E11_LC | . | . | . | . | . | . | . | . | . | . | . | . | . | . | . | . |
| 21-225_6G1_LC | . | . | . | . | N | . | . | T | . | . | . | . | G | . | . | . |
| 21-225_15A3_LC | . | . | . | . | . | . | . | . | . | . | . | . | . | . | . | . |
| 21-225_13D4_LC | . | . | . | . | . | . | . | N | . | . | . | . | . | . | . | . |
| 21-225_16E6_LC | . | . | T | . | . | . | . | . | . | . | . | . | . | . | . | . |

| Reference # | 81 | 82 | 83 | 84 | 85 | 86 | 87 | 88 | 89 | 90 | 91 | 92 | 93 | 94 | 95 | 96 |
|---|---|---|---|---|---|---|---|---|---|---|---|---|---|---|---|---|
| | | | | | | | | | K_FR3 | | | | | | | |
| CONSENSUS | S | G | S | G | - | - | T | D | F | T | L | T | I | S | S | L |
| 21-225_20F6_LC | . | . | . | . | . | . | . | . | . | . | . | . | . | . | . | . |
| 21-225_18G4_LC | . | . | . | . | . | . | . | . | . | . | . | . | . | . | . | . |
| 21-225_22C7_LC | . | . | . | . | . | . | . | . | . | . | . | . | . | . | . | . |
| 21-225_27A11_LC | . | . | . | . | . | . | . | . | . | . | . | . | . | . | . | . |
| 21-225_26G11_LC | . | . | . | . | . | . | . | . | . | . | . | K | . | . | R | V |
| 21-225_26D12_LC | . | . | . | . | . | . | . | . | . | . | . | . | . | . | . | . |
| 21-225_25B3_LC | . | . | . | . | . | . | . | . | . | . | . | . | . | . | . | . |
| 21-225_25F3_LC | . | . | . | . | . | . | . | E | . | . | . | . | . | . | . | . |
| 21-225_3F8_LC | . | . | . | . | . | . | . | V | . | . | . | . | . | . | . | . |
| 21-225_7E11_LC | . | . | . | . | . | . | . | . | . | . | . | . | . | . | . | . |
| 21-225_6G1_LC | . | . | . | . | . | . | . | . | . | . | . | . | . | . | . | . |
| 21-225_15A3_LC | . | . | . | . | . | . | . | . | . | . | . | . | . | . | . | . |
| 21-225_13D4_LC | . | . | . | . | . | . | . | . | . | . | . | . | . | . | . | . |
| 21-225_16E6_LC | . | . | . | . | . | . | . | . | . | . | . | . | . | . | . | . |

FIGURE 56

| Reference # | 97 | 98 | 99 | 100 | 101 | 102 | 103 | 104 | 105 | 106 | 107 | 108 | 109 | 110 | 111 | 112 |
|---|---|---|---|---|---|---|---|---|---|---|---|---|---|---|---|---|
| CONSENSUS | Q | P | E | D | F | A | T | Y | Y | C | Q | Q | T | Y | S | - |
| 21-225_20F6_LC | . | . | . | . | . | . | . | . | . | . | . | . | . | . | . | . |
| 21-225_18G4_LC | . | . | . | . | . | . | . | . | . | . | . | . | . | . | . | . |
| 21-225_22C7_LC | . | . | . | . | . | . | . | . | . | . | . | . | . | . | . | . |
| 21-225_27A11_LC | . | . | . | . | . | . | . | . | C | . | . | . | S | D | . | . |
| 21-225_26G11_LC | E | A | . | . | V | G | I | . | . | . | M | . | S | I | K | . |
| 21-225_26D12_LC | . | . | . | . | . | . | . | . | C | . | . | . | S | D | . | . |
| 21-225_25B3_LC | . | . | . | . | . | . | . | . | C | . | . | . | S | D | . | . |
| 21-225_25F3_LC | . | . | . | . | . | . | . | . | C | . | . | . | S | D | . | . |
| 21-225_3F8_LC | . | . | . | . | . | . | . | . | . | . | . | . | . | . | . | . |
| 21-225_7E11_LC | . | . | . | . | . | . | I | . | . | . | . | . | . | . | . | . |
| 21-225_6G1_LC | . | . | . | . | . | . | . | . | . | . | . | . | S | H | . | . |
| 21-225_15A3_LC | . | . | . | . | . | . | . | . | . | . | . | . | . | . | . | . |
| 21-225_13D4_LC | . | . | . | . | . | . | . | . | . | . | . | . | . | . | . | . |
| 21-225_16E6_LC | . | . | . | . | . | . | . | . | . | . | . | . | . | . | . | . |

| Reference # | 113 | 114 | 115 | 116 | 117 | 118 | 119 | 120 | 121 | 122 | 123 | 124 | 125 | 126 | 127 | 128 |
|---|---|---|---|---|---|---|---|---|---|---|---|---|---|---|---|---|
| | K_CDR3 | | | | | | | | | | | | | | | |
| CONSENSUS | - | - | - | - | - | - | - | - | - | - | - | - | - | - | - | - |
| 21-225_20F6_LC | . | . | . | . | . | . | . | . | . | . | . | . | . | . | . | . |
| 21-225_18G4_LC | . | . | . | . | . | . | . | . | . | . | . | . | . | . | . | . |
| 21-225_22C7_LC | . | . | . | . | . | . | . | . | . | . | . | . | . | . | . | . |
| 21-225_27A11_LC | . | . | . | . | . | . | . | . | . | . | . | . | . | . | . | . |
| 21-225_26G11_LC | . | . | . | . | . | . | . | . | . | . | . | . | . | . | . | . |
| 21-225_26D12_LC | . | . | . | . | . | . | . | . | . | . | . | . | . | . | . | . |
| 21-225_25B3_LC | . | . | . | . | . | . | . | . | . | . | . | . | . | . | . | . |
| 21-225_25F3_LC | . | . | . | . | . | . | . | . | . | . | . | . | . | . | . | . |
| 21-225_3F8_LC | . | . | . | . | . | . | . | . | . | . | . | . | . | . | . | . |
| 21-225_7E11_LC | . | . | . | . | . | . | . | . | . | . | . | . | . | . | . | . |
| 21-225_6G1_LC | . | . | . | . | . | . | . | . | . | . | . | . | . | . | . | . |
| 21-225_15A3_LC | . | . | . | . | . | . | . | . | . | . | . | . | . | . | . | . |
| 21-225_13D4_LC | . | . | . | . | . | . | . | . | . | . | . | . | . | . | . | . |
| 21-225_16E6_LC | . | . | . | . | . | . | . | . | . | . | . | . | . | . | . | . |

FIGURE 56

| Reference # | 129 | 130 | 131 | 132 | 133 | 134 | 135 | 136 | 137 | 138 | 139 | 140 | 141 | 142 | 143 | 144 |
|---|---|---|---|---|---|---|---|---|---|---|---|---|---|---|---|---|
| | | | | | | | | | | | | | | | | K_FR |
| CONSENSUS | - | - | - | - | - | - | T | P | L | T | F | G | G | G | T | K |
| 21-225_20F6_LC | . | . | . | . | . | . | P | . | . | . | . | . | . | . | . | . |
| 21-225_18G4_LC | . | . | . | . | . | . | P | . | . | . | . | . | . | . | . | . |
| 21-225_22C7_LC | . | . | . | . | . | . | . | . | . | . | . | . | . | . | . | . |
| 21-225_27A11_LC | . | . | . | . | . | . | P | . | [illegible] | . | . | . | Q | . | . | . |
| 21-225_26G11_LC | . | . | . | . | . | . | L | . | . | . | . | . | . | . | . | . |
| 21-225_26D12_LC | . | . | . | . | . | . | P | . | [illegible] | . | . | . | Q | . | . | . |
| 21-225_25B3_LC | . | . | . | . | . | . | P | . | [illegible] | . | . | . | Q | . | . | . |
| 21-225_25F3_LC | . | . | . | . | . | . | P | . | [illegible] | . | . | . | Q | . | . | . |
| 21-225_3F8_LC | . | . | . | . | . | . | . | . | . | . | . | . | . | . | . | R |
| 21-225_7E11_LC | . | . | . | . | . | . | . | . | . | . | . | . | . | . | . | . |
| 21-225_6G1_LC | . | . | . | . | . | . | P | . | . | . | . | . | . | . | . | . |
| 21-225_15A3_LC | . | . | . | . | . | . | . | . | . | . | . | . | . | . | . | . |
| 21-225_13D4_LC | . | . | . | . | . | . | L | . | . | . | . | . | . | . | . | . |
| 21-225_16E6_LC | . | . | . | . | . | . | . | . | . | . | . | . | . | . | . | . |

| Reference # | 145 | 146 | 147 | 148 | 149 |
|---|---|---|---|---|---|
| CONSENSUS | V | E | I | K | R |
| 21-225_20F6_LC | . | . | . | . | . |
| 21-225_18G4_LC | . | . | . | . | . |
| 21-225_22C7_LC | . | . | . | . | . |
| 21-225_27A11_LC | . | . | . | . | . |
| 21-225_26G11_LC | . | . | . | . | . |
| 21-225_26D12_LC | . | . | . | . | . |
| 21-225_25B3_LC | . | . | . | . | . |
| 21-225_25F3_LC | . | . | . | . | . |
| 21-225_3F8_LC | . | . | . | N | . |
| 21-225_7E11_LC | . | . | . | . | . |
| 21-225_6G1_LC | . | . | . | . | . |
| 21-225_15A3_LC | . | . | . | . | . |
| 21-225_13D4_LC | . | . | . | . | . |
| 21-225_16E6_LC | . | . | . | . | . |

**FIGURE 56**

**Table 44**

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | K_FR1 | | | | | | | | | | | | | | | | |
| CONSENSUS | D | I | Q | M | T | Q | S | P | S | S | L | S | A | S | V | G | D |
| 21-225_5E5_LC | . | . | . | . | . | . | . | . | . | . | . | . | . | . | . | . | . |
| 21-225_17H3_LC | . | . | . | . | . | . | . | . | . | . | . | . | . | . | . | . | . |
| 21-225_20B2_LC | . | . | . | . | . | . | . | . | . | . | . | . | . | . | . | . | . |
| 21-225_22F4_LC | . | . | . | . | . | . | . | . | . | . | . | . | . | . | . | . | . |
| 21-225_24B6_LC | . | . | . | . | . | . | . | . | . | . | . | . | . | . | . | . | . |
| 21-225_23A10_LC | . | . | . | . | . | . | . | . | . | . | . | . | . | . | . | . | . |
| 21-225_25A2_LC | . | . | V | . | N | . | T | . | L | . | . | . | V | T | P | . | Q |
| 21-225_25D12_LC | . | . | . | . | . | . | . | . | . | . | . | . | . | . | . | . | . |
| 21-225_32H2_LC | . | . | V | . | . | . | T | . | L | . | . | . | V | T | P | . | Q |
| 21-225_29H4_LC | . | . | V | . | . | . | T | . | L | . | . | . | V | T | P | . | Q |
| 21-225_26A11_LC | . | . | . | . | . | . | . | . | I | . | . | . | . | . | . | . | . |
| 21-225_26G7_LC | . | . | L | . | . | . | T | . | L | . | . | . | V | T | P | . | Q |
| 21-225_4C5_LC | . | . | . | . | . | . | . | . | . | . | . | . | . | . | . | . | H |
| 21-225_14E10_LC | . | . | . | . | . | . | . | . | . | . | . | . | . | . | . | . | . |
| 21-225_10E9_LC | . | . | . | . | . | . | . | . | . | . | . | . | . | . | . | . | . |
| 21-225_2G4_LC | . | . | . | . | . | . | . | . | . | . | . | . | . | . | . | . | . |
| 21-225_7C9_LC | . | . | . | . | . | . | . | . | . | . | . | . | . | . | G | . | . |
| 21-225_4C12_LC | . | . | . | . | . | . | . | . | . | . | . | . | . | . | . | . | . |
| 21-225_7G4_LC | . | . | . | . | . | . | . | . | . | . | . | . | . | . | . | . | . |
| 21-225_15C11_LC | . | . | . | . | . | . | . | . | . | . | . | . | . | . | . | . | . |

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | L_FR1 | | | | | | | | | | | | | | | | |
| CONSENSUS | S | Y | E | L | | Q | P | - | P | S | V | S | V | S | P | G | Q |
| 21-225_27E7_LC | . | . | . | . | T | . | . | . | . | . | . | . | . | . | . | . | . |
| 21-225_3H10_LC | . | . | . | . | S | . | . | . | . | . | . | . | . | . | . | . | . |

FIGURE 56

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | | K_CDR1 | |
| CONSENSUS | R | V | T | I | T | C | R | A | S | - | - | Q | G | I | R | - | - |
| 21-225_5E5_LC | . | . | . | . | . | . | . | . | . | . | . | . | . | . | . | . | . |
| 21-225_17H3_LC | . | . | . | F | . | . | . | . | . | . | . | . | . | . | . | . | . |
| 21-225_20B2_LC | . | . | N | . | . | . | . | . | . | . | . | . | D | . | . | . | . |
| 21-225_22F4_LC | . | . | . | . | . | . | . | . | . | . | . | . | V | . | . | . | . |
| 21-225_24B6_LC | . | . | . | . | . | . | . | . | . | . | . | . | D | . | . | . | . |
| 21-225_23A10_LC | . | . | . | . | . | . | . | . | . | . | . | . | . | . | . | . | . |
| 21-225_25A2_LC | P | A | S | . | S | . | K | S | . | . | . | . | S | L | L | H | S |
| 21-225_25D12_LC | . | . | . | . | . | . | . | T | . | . | . | . | . | . | . | . | . |
| 21-225_32H2_LC | P | A | S | . | S | . | . | S | . | . | . | . | S | L | L | H | S |
| 21-225_29H4_LC | P | A | S | F | S | . | K | S | . | . | . | . | S | L | L | H | S |
| 21-225_26A11_LC | . | . | . | . | . | . | . | . | . | . | . | . | A | . | . | . | . |
| 21-225_26G7_LC | P | A | S | . | S | . | K | S | . | . | . | . | S | L | L | H | S |
| 21-225_4C5_LC | . | . | H | L | . | . | . | . | . | . | . | R | . | . | . | . | . |
| 21-225_14E10_LC | . | . | . | . | . | . | . | T | . | . | . | . | . | . | . | . | . |
| 21-225_10E9_LC | . | . | . | F | . | . | . | . | . | . | . | . | D | . | . | . | . |
| 21-225_2G4_LC | . | . | . | . | . | . | . | . | . | . | . | . | . | . | . | . | . |
| 21-225_7C9_LC | . | . | . | . | . | . | . | . | . | . | . | . | . | . | . | . | . |
| 21-225_4C12_LC | . | . | . | . | . | . | . | . | . | . | . | . | . | . | . | . | . |
| 21-225_7G4_LC | . | . | . | . | . | . | . | . | . | . | . | . | . | . | . | . | . |
| 21-225_15C11_LC | . | . | . | . | . | . | . | . | . | . | . | . | . | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | | L_CDR1 | |
| CONSENSUS | T | A | S | I | T | C | S | G | D | - | - | - | K | L | G | | - |
| 21-225_27E7_LC | . | . | . | . | . | . | . | . | . | . | . | . | . | . | . | N | . |
| 21-225_3H10_LC | . | . | . | . | . | . | . | . | . | . | . | . | . | . | . | E | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | | K_FR2 | |
| CONSENSUS | - | - | - | - | N | D | L | G | W | Y | Q | Q | K | P | G | K | A |
| 21-225_5E5_LC | . | . | . | . | . | . | . | . | . | . | . | . | . | . | . | . | . |
| 21-225_17H3_LC | . | . | . | . | S | . | . | . | . | . | . | . | . | . | . | . | . |
| 21-225_20B2_LC | . | . | . | . | . | . | . | . | . | . | . | . | . | . | . | . | . |
| 21-225_22F4_LC | . | . | . | . | D | . | . | . | . | . | . | . | . | . | . | . | . |
| 21-225_24B6_LC | . | . | . | . | . | . | . | . | . | . | . | . | . | . | . | . | . |
| 21-225_23A10_LC | . | . | . | . | . | . | . | . | . | . | . | . | . | . | . | . | . |
| 21-225_25A2_LC | E | . | G | K | T | Y | . | Y | . | . | L | . | . | . | . | Q | P |
| 21-225_25D12_LC | . | . | . | . | . | . | . | . | . | . | . | . | . | . | . | . | . |
| 21-225_32H2_LC | D | . | G | R | T | Y | . | Y | . | . | L | . | . | . | . | Q | P |
| 21-225_29H4_LC | D | . | G | K | T | Y | . | Y | . | . | L | . | . | . | . | Q | P |
| 21-225_26A11_LC | . | . | . | . | . | . | . | . | . | . | . | . | R | . | . | . | . |
| 21-225_26G7_LC | E | . | G | K | T | Y | . | Y | . | . | L | . | . | . | . | Q | P |
| 21-225_4C5_LC | . | . | . | . | G | . | . | . | . | . | R | . | . | . | . | . | . |
| 21-225_14E10_LC | . | . | . | . | . | . | . | . | . | . | . | H | . | . | . | Q | . |
| 21-225_10E9_LC | . | . | . | . | S | . | . | . | . | . | . | . | . | . | . | . | . |
| 21-225_2G4_LC | . | . | . | . | . | . | . | . | . | . | . | . | . | . | . | . | . |
| 21-225_7C9_LC | . | . | . | . | . | . | . | . | . | . | . | . | . | . | . | . | . |
| 21-225_4C12_LC | . | . | . | . | . | . | . | . | . | . | . | . | . | . | . | . | . |
| 21-225_7G4_LC | . | . | . | . | . | . | . | . | . | . | . | . | R | . | . | . | . |
| 21-225_15C11_LC | . | . | . | . | . | . | . | . | . | . | . | . | . | . | . | . | V |

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | | L_FR2 | |
| CONSENSUS | - | - | - | - | K | Y | A | C | W | Y | Q | Q | K | P | G | Q | S |
| 21-225_27E7_LC | . | . | . | . | . | . | . | . | . | . | . | . | . | . | . | . | . |
| 21-225_3H10_LC | . | . | . | . | . | . | . | . | . | . | . | . | . | . | . | . | . |

**FIGURE 56**

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | K_CDR2 | | | | | | | | | | |
| CONSENSUS | P | K | R | L | I | Y | A | - | - | - | - | - | - | - | - | A | S |
| 21-225_5E5_LC | . | . | . | . | . | . | . | . | . | . | . | . | . | . | . | . | . |
| 21-225_17H3_LC | . | . | . | . | . | . | . | . | . | . | . | . | . | . | . | . | . |
| 21-225_20B2_LC | . | . | . | . | . | . | . | . | . | . | . | . | . | . | . | . | . |
| 21-225_22F4_LC | . | . | . | . | . | . | . | . | . | . | . | . | . | . | . | . | . |
| 21-225_24B6_LC | . | . | . | . | . | . | . | . | . | . | . | . | . | . | . | . | . |
| 21-225_23A10_LC | . | . | . | . | . | . | . | . | . | . | . | . | . | . | . | . | . |
| 21-225_25A2_LC | . | Q | L | . | . | . | E | . | . | . | . | . | . | . | . | I | . |
| 21-225_25D12_LC | . | . | . | . | . | . | . | . | . | . | . | . | . | . | . | . | . |
| 21-225_32H2_LC | . | Q | L | . | . | . | E | . | . | . | . | . | . | . | . | L | . |
| 21-225_29H4_LC | . | Q | L | . | . | F | E | . | . | . | . | . | . | . | . | V | . |
| 21-225_26A11_LC | . | . | . | . | . | . | . | . | . | . | . | . | . | . | . | . | . |
| 21-225_26G7_LC | . | Q | F | . | . | . | E | . | . | . | . | . | . | . | . | V | . |
| 21-225_4C5_LC | . | . | . | . | . | . | . | . | . | . | . | . | . | . | . | . | . |
| 21-225_14E10_LC | . | . | . | . | . | . | . | . | . | . | . | . | . | . | . | . | . |
| 21-225_10E9_LC | . | T | . | . | . | F | . | . | . | . | . | . | . | . | . | . | . |
| 21-225_2G4_LC | . | T | . | . | . | . | . | . | . | . | . | . | . | . | . | . | . |
| 21-225_7C9_LC | . | . | . | . | . | . | . | . | . | . | . | . | . | . | . | . | . |
| 21-225_4C12_LC | . | T | . | . | . | . | . | . | . | . | . | . | . | . | . | . | . |
| 21-225_7G4_LC | . | . | . | . | . | . | . | . | . | . | . | . | . | . | . | . | . |
| 21-225_15C11_LC | . | . | . | . | . | . | . | . | . | . | . | . | . | . | . | . | . |

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | L_CDR2 | | | | | | | | | | |
| CONSENSUS | P | V | | V | I | Y | Q | - | - | - | - | - | - | - | - | D | |
| 21-225_27E7_LC | . | . | E | . | . | . | . | . | . | . | . | . | . | . | . | . | S |
| 21-225_3H10_LC | . | . | L | . | . | . | . | . | . | . | . | . | . | . | . | . | R |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | R | F | S | G | S | G | S | G | - |
| 21-225_5E5_LC | . | . | . | . | . | . | . | . | . | . | . | . | . | . | . | . | . |
| 21-225_17H3_LC | . | . | . | . | . | . | . | . | . | . | . | . | . | . | . | . | . |
| 21-225_20B2_LC | . | . | . | . | . | . | . | . | . | . | . | . | . | . | . | . | . |
| 21-225_22F4_LC | . | . | . | . | . | . | . | . | . | . | . | . | . | R | . | . | . |
| 21-225_24B6_LC | . | . | . | . | . | . | . | . | . | . | . | . | . | . | . | . | . |
| 21-225_23A10_LC | . | . | . | . | . | . | . | . | . | . | . | . | . | . | . | . | . |
| 21-225_25A2_LC | N | R | F | . | . | . | . | A | . | L | . | . | . | . | . | . | . |
| 21-225_25D12_LC | . | . | . | . | . | . | . | . | . | . | . | . | . | . | . | . | . |
| 21-225_32H2_LC | N | R | F | . | . | . | . | D | . | . | . | . | . | . | . | . | . |
| 21-225_29H4_LC | H | R | F | . | . | L | . | D | . | . | . | . | . | . | . | . | . |
| 21-225_26A11_LC | . | . | . | . | . | . | . | . | . | . | . | . | . | . | . | . | . |
| 21-225_26G7_LC | H | R | L | . | . | . | . | D | . | . | . | . | . | . | . | . | . |
| 21-225_4C5_LC | N | . | . | . | . | . | . | . | . | . | . | . | . | . | Y | . | . |
| 21-225_14E10_LC | . | . | . | . | . | . | . | . | . | . | . | . | . | . | . | . | . |
| 21-225_10E9_LC | . | . | . | . | . | . | . | . | . | . | . | . | . | . | . | . | . |
| 21-225_2G4_LC | . | . | . | . | . | . | . | . | . | . | . | . | . | . | . | . | . |
| 21-225_7C9_LC | . | . | . | . | . | . | . | . | . | . | . | . | . | . | . | . | . |
| 21-225_4C12_LC | . | . | . | . | . | . | . | . | . | . | . | . | . | . | . | . | . |
| 21-225_7G4_LC | . | . | . | . | . | . | . | . | . | . | . | . | . | . | . | . | . |
| 21-225_15C11_LC | . | . | . | . | . | . | . | . | . | . | . | . | . | . | . | . | . |

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | K | R | P | | G | I | P | E | R | F | S | G | S | N | S | G | - |
| 21-225_27E7_LC | . | . | . | L | . | . | . | . | . | . | . | . | . | . | . | . | . |
| 21-225_3H10_LC | . | . | . | S | . | . | . | . | . | . | . | . | . | . | . | . | . |

# FIGURE 56

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | K_FR3 | | | | | | | | | | | | | | | | |
| CONSENSUS | - | T | E | F | T | L | T | I | S | S | L | Q | P | E | D | F | A |
| 21-225_5E5_LC | . | . | . | . | . | . | . | . | . | . | . | . | . | . | . | . | . |
| 21-225_17H3_LC | . | . | . | . | . | . | . | . | . | . | . | . | . | . | . | . | . |
| 21-225_20B2_LC | . | . | . | . | . | . | . | . | . | . | . | . | . | . | . | . | . |
| 21-225_22F4_LC | . | . | D | . | . | . | . | . | . | . | . | . | . | . | . | . | . |
| 21-225_24B6_LC | . | . | . | . | . | . | . | . | . | . | . | . | T | . | . | . | . |
| 21-225_23A10_LC | . | . | . | . | . | . | . | . | G | . | . | . | . | . | . | . | . |
| 21-225_25A2_LC | . | . | D | . | . | . | K | . | . | R | V | E | A | . | . | V | G |
| 21-225_25D12_LC | . | . | . | . | . | . | . | . | . | . | . | . | . | . | . | . | . |
| 21-225_32H2_LC | . | . | D | . | . | . | K | . | . | R | V | E | A | . | . | V | G |
| 21-225_29H4_LC | . | . | D | . | . | . | K | . | . | R | V | E | A | . | . | V | G |
| 21-225_26A11_LC | . | . | . | . | I | . | . | . | . | . | . | . | . | . | . | . | . |
| 21-225_26G7_LC | . | . | D | . | . | . | K | . | . | R | V | E | A | . | . | V | G |
| 21-225_4C5_LC | . | . | . | . | . | . | . | . | G | . | . | . | . | . | . | . | . |
| 21-225_14E10_LC | . | . | . | . | . | . | . | . | . | . | . | . | . | . | . | . | T |
| 21-225_10E9_LC | . | . | . | . | . | . | . | . | N | . | . | . | . | . | . | . | . |
| 21-225_2G4_LC | . | . | . | . | . | . | . | . | . | . | . | . | . | . | . | L | . |
| 21-225_7C9_LC | . | . | . | . | . | . | . | . | . | . | . | . | . | . | . | . | . |
| 21-225_4C12_LC | . | . | . | . | . | . | . | . | . | . | . | . | . | . | . | L | . |
| 21-225_7G4_LC | . | . | . | . | . | . | . | . | . | . | . | . | . | . | . | S | . |
| 21-225_15C11_LC | . | . | . | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | L_FR3 | | | | | | | | | | | | | | | | |
| CONSENSUS | - | N | | A | T | L | T | I | S | G | T | Q | A | M | D | E | A |
| 21-225_27E7_LC | . | . | T | . | . | . | . | . | . | . | . | . | . | . | . | . | . |
| 21-225_3H10_LC | . | . | K | . | . | . | . | . | . | . | . | . | . | . | . | . | . |

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | L | Q | H | Y | S | - | - | - | - | - | - | - | - |
| 21-225_5E5_LC | . | . | . | . | . | . | . | . | . | . | . | . | . | . | . | . | . |
| 21-225_17H3_LC | . | . | . | . | . | . | Q | . | . | . | . | . | . | . | . | . | . |
| 21-225_20B2_LC | . | . | . | . | . | . | . | . | N | . | . | . | . | . | . | . | . |
| 21-225_22F4_LC | . | . | . | . | . | H | . | . | . | . | . | . | . | . | . | . | . |
| 21-225_24B6_LC | . | . | . | . | . | . | . | . | . | . | . | . | . | . | . | . | . |
| 21-225_23A10_LC | . | . | . | . | . | . | . | . | . | . | . | . | . | . | . | . | . |
| 21-225_25A2_LC | V | . | . | . | M | . | S | T | Q | . | . | . | . | . | . | . | . |
| 21-225_25D12_LC | . | . | . | . | . | . | . | . | . | . | . | . | . | . | . | . | . |
| 21-225_32H2_LC | I | . | S | . | . | . | S | I | Q | . | . | . | . | . | . | . | . |
| 21-225_29H4_LC | V | . | . | . | M | . | S | I | Q | . | . | . | . | . | . | . | . |
| 21-225_26A11_LC | . | . | . | . | . | . | . | . | N | . | . | . | . | . | . | . | . |
| 21-225_26G7_LC | V | . | . | . | M | . | S | I | Q | . | . | . | . | . | . | . | . |
| 21-225_4C5_LC | . | . | . | . | . | . | . | . | . | . | . | . | . | . | . | . | . |
| 21-225_14E10_LC | A | . | . | . | . | . | . | . | . | . | . | . | . | . | . | . | . |
| 21-225_10E9_LC | . | . | . | . | . | . | . | . | . | . | . | . | . | . | . | . | . |
| 21-225_2G4_LC | . | . | . | . | . | . | . | . | . | . | . | . | . | . | . | . | . |
| 21-225_7C9_LC | . | . | . | . | . | . | L | . | . | . | . | . | . | . | . | . | . |
| 21-225_4C12_LC | . | . | . | . | . | . | . | . | . | . | . | . | . | . | . | . | . |
| 21-225_7G4_LC | . | . | . | . | . | H | Q | . | . | . | . | . | . | . | . | . | . |
| 21-225_15C11_LC | . | . | . | . | . | L | . | . | . | . | . | . | . | . | . | . | . |

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | D | Y | Y | C | Q | A | W | | | - | - | - | - | - | - | - | - |
| 21-225_27E7_LC | . | . | . | . | . | . | . | D | S | . | . | . | . | . | . | . | . |
| 21-225_3H10_LC | . | . | . | . | . | . | . | V | N | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_5E5_LC | . | . | . | . | . | . | . | . | . | . | . | . | . | . | . | . | . |
| 21-225_17H3_LC | . | . | . | . | . | . | . | . | . | . | . | . | . | . | . | . | . |
| 21-225_20B2_LC | . | . | . | . | . | . | . | . | . | . | . | . | . | . | . | . | . |
| 21-225_22F4_LC | . | . | . | . | . | . | . | . | . | . | . | . | . | . | . | . | . |
| 21-225_24B6_LC | . | . | . | . | . | . | . | . | . | . | . | . | . | . | . | . | . |
| 21-225_23A10_LC | . | . | . | . | . | . | . | . | . | . | . | . | . | . | . | . | . |
| 21-225_25A2_LC | . | . | . | . | . | . | . | . | . | . | . | . | . | . | . | F | . |
| 21-225_25D12_LC | . | . | . | . | . | . | . | . | . | . | . | . | . | . | . | F | . |
| 21-225_32H2_LC | . | . | . | . | . | . | . | . | . | . | . | . | . | . | . | . | . |
| 21-225_29H4_LC | . | . | . | . | . | . | . | . | . | . | . | . | . | . | . | H | . |
| 21-225_26A11_LC | . | . | . | . | . | . | . | . | . | . | . | . | . | . | . | . | . |
| 21-225_26G7_LC | . | . | . | . | . | . | . | . | . | . | . | . | . | . | . | L | . |
| 21-225_4C5_LC | . | . | . | . | . | . | . | . | . | . | . | . | . | . | . | . | . |
| 21-225_14E10_LC | . | . | . | . | . | . | . | . | . | . | . | . | . | . | . | . | . |
| 21-225_10E9_LC | . | . | . | . | . | . | . | . | . | . | . | . | . | . | . | . | . |
| 21-225_2G4_LC | . | . | . | . | . | . | . | . | . | . | . | . | . | . | . | F | . |
| 21-225_7C9_LC | . | . | . | . | . | . | . | . | . | . | . | . | . | . | . | . | . |
| 21-225_4C12_LC | . | . | . | . | . | . | . | . | . | . | . | . | . | . | . | F | . |
| 21-225_7G4_LC | . | . | . | . | . | . | . | . | . | . | . | . | . | . | . | . | . |
| 21-225_15C11_LC | . | . | . | . | . | . | . | . | . | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | L_CDR3 | | | | | | | | | | | | | | |
| CONSENSUS | - | - | - | - | - | - | - | - | - | - | - | - | - | - | - | | T |
| 21-225_27E7_LC | . | . | . | . | . | . | . | . | . | . | . | . | . | . | . | S | . |
| 21-225_3H10_LC | . | . | . | . | . | . | . | . | . | . | . | . | . | . | . | N | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | K_FR4 | | | | | | | | | | |
| CONSENSUS | R | T | F | G | Q | G | T | K | V | E | I | K | R |
| 21-225_5E5_LC | . | . | . | . | . | . | . | . | . | . | V | . | . |
| 21-225_17H3_LC | . | . | . | . | . | . | . | . | . | . | . | . | . |
| 21-225_20B2_LC | . | A | . | . | . | . | . | . | . | D | . | . | . |
| 21-225_22F4_LC | . | . | . | . | . | . | . | . | . | . | . | . | . |
| 21-225_24B6_LC | . | . | . | . | . | . | . | . | . | . | V | . | . |
| 21-225_23A10_LC | . | . | . | . | L | . | . | . | . | . | . | . | . |
| 21-225_25A2_LC | L | . | . | . | G | . | . | . | . | . | . | . | . |
| 21-225_25D12_LC | . | . | . | . | . | . | . | . | . | . | . | . | . |
| 21-225_32H2_LC | I | . | . | . | . | . | . | R | L | . | . | . | . |
| 21-225_29H4_LC | F | . | . | . | G | . | . | R | . | . | . | . | . |
| 21-225_26A11_LC | . | S | . | . | . | . | . | . | L | . | . | R | . |
| 21-225_26G7_LC | L | . | . | . | G | . | . | . | . | . | . | . | . |
| 21-225_4C5_LC | . | . | . | . | . | . | . | . | . | . | . | . | . |
| 21-225_14E10_LC | . | . | . | . | . | . | . | . | . | . | . | . | . |
| 21-225_10E9_LC | . | . | . | . | . | . | . | . | . | . | . | . | . |
| 21-225_2G4_LC | . | . | . | . | R | . | . | . | . | . | . | . | . |
| 21-225_7C9_LC | . | . | . | . | . | . | . | . | . | D | . | . | . |
| 21-225_4C12_LC | . | . | . | . | . | . | . | . | . | . | . | . | . |
| 21-225_7G4_LC | . | . | . | . | . | . | . | . | . | . | . | . | . |
| 21-225_15C11_LC | . | . | . | . | R | . | . | . | . | . | . | . | . |

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | L_FR4 | | | | | | | | | | |
| CONSENSUS | | | F | G | G | G | T | K | L | T | V | L | G |
| 21-225_27E7_LC | V | V | . | . | . | . | . | . | . | . | . | . | . |
| 21-225_3H10_LC | M | I | . | . | . | . | . | . | . | . | . | . | . |

FIGURE 56

Table 45

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | | | | | | | | | | | | K_FR1 | | | | | |
| CONSENSUS | D | I | Q | M | T | Q | S | P | S | S | L | S | A | S | V | G | D |
| 21-225_18H12_LC | . | . | . | . | . | . | . | . | . | . | . | . | . | . | . | . | . |
| 21-225_22B7_LC | . | . | . | . | . | . | . | . | . | . | . | . | . | . | . | . | . |
| 21-225_25A9_LC | . | . | . | . | . | . | . | . | . | . | . | . | . | . | . | . | . |
| 21-225_29D9_LC | . | . | . | . | . | . | . | . | . | . | . | . | . | . | . | . | . |
| 21-225_25G5_LC | . | . | . | . | . | . | . | . | . | . | . | . | . | . | . | . | . |
| 21-225_28B8_LC | . | . | . | . | . | . | . | . | . | . | . | . | . | . | . | . | . |
| 21-225_28A9_LC | . | . | . | . | . | . | . | . | . | . | . | . | . | . | . | . | . |
| 21-225_29D8_LC | . | . | . | . | . | . | . | . | . | . | . | . | . | . | . | . | . |
| 21-225_30F3_LC | . | . | . | . | . | . | . | . | . | . | . | . | . | . | . | . | . |
| 21-225_33B1_LC | . | . | . | . | . | . | . | . | . | . | . | . | . | . | . | . | . |
| 21-225_15B11_LC | . | . | . | . | . | . | . | . | . | . | . | . | . | . | . | . | . |
| 21-225_14C2_LC | . | . | . | . | I | . | . | . | . | . | . | F | . | C | . | . | . |
| 21-225_4C8_LC | . | . | . | . | I | . | . | . | . | F | . | F | . | C | . | . | . |
| 21-225_4F12_LC | . | . | . | . | . | . | . | . | . | . | . | . | . | . | . | . | . |
| 21-225_15H10_LC | . | . | . | . | . | . | . | . | . | . | . | . | . | . | . | . | . |
| 21-225_16H4_LC | . | . | . | . | . | . | . | . | . | . | . | . | . | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | | K_CDR1 | |
| CONSENSUS | R | V | T | I | T | C | R | A | S | - | - | Q | G | I | R | - | - |
| 21-225_18H12_LC | . | . | . | . | . | . | . | . | . | . | . | . | . | . | . | . | . |
| 21-225_22B7_LC | . | . | . | . | . | . | . | . | . | . | . | . | . | . | . | . | . |
| 21-225_25A9_LC | . | . | . | . | . | . | . | . | . | . | . | . | . | . | . | . | . |
| 21-225_29D9_LC | . | . | . | . | . | . | . | . | . | . | . | . | . | . | . | . | . |
| 21-225_25G5_LC | . | . | . | . | . | . | . | . | . | . | . | . | D | . | . | . | . |
| 21-225_28B8_LC | . | . | . | . | . | . | . | . | . | . | . | . | . | . | . | . | . |
| 21-225_28A9_LC | . | . | . | . | . | . | . | . | . | . | . | . | . | . | . | . | . |
| 21-225_29D8_LC | . | . | . | . | . | . | . | . | . | . | . | . | . | . | . | . | . |
| 21-225_30F3_LC | . | . | . | . | . | . | . | . | . | . | . | . | . | . | . | . | . |
| 21-225_33B1_LC | . | . | . | . | . | . | . | T | . | . | . | . | . | . | . | . | . |
| 21-225_15B11_LC | . | . | . | . | . | . | . | . | . | . | . | . | . | . | . | . | . |
| 21-225_14C2_LC | . | . | I | . | . | . | . | . | . | . | . | . | . | . | . | . | . |
| 21-225_4C8_LC | . | . | . | . | . | . | . | . | . | . | . | . | . | . | . | . | . |
| 21-225_4F12_LC | . | . | . | . | . | . | . | . | . | . | . | . | . | . | . | . | . |
| 21-225_15H10_LC | . | . | . | . | . | . | . | . | . | . | . | . | . | . | . | . | . |
| 21-225_16H4_LC | . | . | . | . | . | . | . | . | . | . | . | . | . | . | . | . | . |

**FIGURE 56**

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | | K_FR2 | |
| CONSENSUS | - | - | - | - | N | D | L | G | W | Y | Q | Q | K | P | G | K | A |
| 21-225_18H12_LC | . | . | . | . | . | . | . | . | . | . | . | . | . | . | . | . | . |
| 21-225_22B7_LC | . | . | . | . | . | . | . | . | . | . | . | . | . | . | . | . | . |
| 21-225_25A9_LC | . | . | . | . | . | . | . | . | . | . | . | . | . | . | . | . | . |
| 21-225_29D9_LC | . | . | . | . | . | . | . | . | . | F | . | . | . | . | . | . | . |
| 21-225_25G5_LC | . | . | . | . | . | . | . | . | . | . | . | . | . | . | . | . | . |
| 21-225_28B8_LC | . | . | . | . | . | . | . | . | . | . | . | H | . | . | . | . | . |
| 21-225_28A9_LC | . | . | . | . | . | . | . | . | . | . | . | . | . | . | . | . | . |
| 21-225_29D8_LC | . | . | . | . | . | . | . | . | . | . | . | . | . | . | . | . | . |
| 21-225_30F3_LC | . | . | . | . | . | . | . | . | . | . | . | . | . | . | . | . | . |
| 21-225_33B1_LC | . | . | . | . | . | . | . | . | . | F | . | . | . | . | . | . | . |
| 21-225_15B11_LC | . | . | . | . | . | . | . | . | . | . | . | . | . | . | . | . | . |
| 21-225_14C2_LC | . | . | . | . | . | . | . | D | . | . | . | . | . | . | . | . | . |
| 21-225_4C8_LC | . | . | . | . | . | . | . | . | . | . | . | . | . | . | . | . | . |
| 21-225_4F12_LC | . | . | . | . | . | . | . | . | . | . | . | . | . | . | . | . | . |
| 21-225_15H10_LC | . | . | . | . | . | . | . | . | . | . | . | . | . | . | . | . | . |
| 21-225_16H4_LC | . | . | . | . | . | . | . | . | . | . | . | . | . | . | . | . | . |

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | K_CDR2 | | | |
| CONSENSUS | P | K | R | L | I | Y | A | - | - | - | - | - | - | - | - | A | S |
| 21-225_18H12_LC | . | . | . | . | . | . | T | . | . | . | . | . | . | . | . | . | . |
| 21-225_22B7_LC | . | . | . | . | . | . | T | . | . | . | . | . | . | . | . | . | . |
| 21-225_25A9_LC | . | . | . | . | . | . | . | . | . | . | . | . | . | . | . | . | . |
| 21-225_29D9_LC | . | . | . | . | . | . | . | . | . | . | . | . | . | . | . | . | . |
| 21-225_25G5_LC | . | . | . | . | . | . | . | . | . | . | . | . | . | . | . | . | . |
| 21-225_28B8_LC | . | . | . | . | . | . | . | . | . | . | . | . | . | . | . | . | . |
| 21-225_28A9_LC | . | . | . | . | . | . | . | . | . | . | . | . | . | . | . | . | . |
| 21-225_29D8_LC | . | . | . | . | . | . | T | . | . | . | . | . | . | . | . | . | . |
| 21-225_30F3_LC | . | . | . | . | . | . | T | . | . | . | . | . | . | . | . | . | . |
| 21-225_33B1_LC | . | . | . | . | . | . | T | . | . | . | . | . | . | . | . | . | . |
| 21-225_15B11_LC | . | . | . | . | . | . | . | . | . | . | . | . | . | . | . | . | . |
| 21-225_14C2_LC | . | . | . | . | . | . | . | . | . | . | . | . | . | . | . | . | . |
| 21-225_4C8_LC | . | . | . | . | . | . | . | . | . | . | . | . | . | . | . | . | . |
| 21-225_4F12_LC | . | . | . | . | . | . | . | . | . | . | . | . | . | . | . | . | . |
| 21-225_15H10_LC | . | . | . | . | . | . | . | . | . | . | . | . | . | . | . | . | . |
| 21-225_16H4_LC | . | . | . | . | . | . | T | . | . | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | R | F | S | G | S | G | S | G | - |
| 21-225_18H12_LC | N | . | . | . | . | . | . | . | . | . | . | . | . | . | . | . | . |
| 21-225_22B7_LC | . | . | . | . | . | . | . | . | . | . | . | . | . | . | . | . | . |
| 21-225_25A9_LC | . | . | . | . | . | . | . | . | . | . | . | . | . | . | . | . | . |
| 21-225_29D9_LC | . | . | . | . | . | . | . | . | . | . | . | . | . | . | . | . | . |
| 21-225_25G5_LC | . | . | . | . | . | . | . | . | . | . | . | . | . | . | . | . | . |
| 21-225_28B8_LC | . | . | . | . | . | . | . | . | . | . | . | . | . | . | . | . | . |
| 21-225_28A9_LC | . | . | . | N | . | . | . | . | . | . | . | . | . | . | . | . | . |
| 21-225_29D8_LC | . | . | . | . | . | . | . | . | . | . | . | . | . | . | . | . | . |
| 21-225_30F3_LC | . | . | . | . | . | . | . | . | . | . | . | . | . | . | . | . | . |
| 21-225_33B1_LC | . | . | . | . | . | . | . | . | . | . | . | . | . | . | . | . | . |
| 21-225_15B11_LC | . | . | . | . | . | . | . | . | . | . | . | . | . | . | . | . | . |
| 21-225_14C2_LC | . | . | . | . | . | . | . | . | . | . | . | . | . | . | . | . | . |
| 21-225_4C8_LC | . | . | . | . | . | . | . | . | . | . | . | . | . | V | . | . | . |
| 21-225_4F12_LC | . | . | . | . | . | . | . | . | . | . | . | . | G | . | . | . | . |
| 21-225_15H10_LC | . | . | . | . | . | . | . | . | . | . | . | . | . | . | . | . | . |
| 21-225_16H4_LC | . | . | . | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | | | | K_FR3 | | | | | | | | | | | | | |
| CONSENSUS | - | T | E | F | T | L | T | I | S | S | L | Q | P | E | D | F | A |
| 21-225_18H12_LC | . | . | . | . | . | . | . | . | . | . | . | . | . | . | . | . | . |
| 21-225_22B7_LC | . | . | . | . | . | . | . | . | . | . | . | . | . | . | . | . | . |
| 21-225_25A9_LC | . | . | . | . | . | . | . | . | . | . | . | . | . | . | . | . | . |
| 21-225_29D9_LC | . | . | . | . | . | . | . | . | . | . | . | . | . | . | . | . | . |
| 21-225_25G5_LC | . | . | . | . | . | . | . | V | . | . | . | . | . | . | . | . | . |
| 21-225_28B8_LC | . | . | . | . | . | . | . | . | . | . | . | . | . | . | . | . | . |
| 21-225_28A9_LC | . | . | . | . | . | . | . | . | . | . | . | . | . | . | . | . | . |
| 21-225_29D8_LC | . | . | . | . | . | . | . | . | . | . | . | . | . | . | . | . | . |
| 21-225_30F3_LC | . | . | D | . | . | . | . | . | . | . | . | . | . | . | . | . | . |
| 21-225_33B1_LC | . | . | . | . | . | . | . | . | . | . | . | . | . | . | . | . | . |
| 21-225_15B11_LC | . | . | . | . | . | . | . | . | . | . | . | . | . | . | . | . | . |
| 21-225_14C2_LC | . | . | . | . | . | . | . | . | . | . | V | . | R | . | . | . | . |
| 21-225_4C8_LC | . | . | . | . | . | . | . | . | . | . | . | . | . | . | . | . | . |
| 21-225_4F12_LC | . | . | I | . | . | . | . | . | . | . | . | . | . | . | . | . | . |
| 21-225_15H10_LC | . | . | . | . | . | . | . | . | . | . | . | . | . | . | . | . | . |
| 21-225_16H4_LC | . | . | . | . | . | . | . | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | L | Q | H | N | S | - | - | - | - | - | - | - | - |
| 21-225_18H12_LC | . | . | . | . | . | . | . | . | N | . | . | . | . | . | . | . | . |
| 21-225_22B7_LC | . | . | . | . | . | . | . | . | T | . | . | . | . | . | . | . | . |
| 21-225_25A9_LC | . | . | . | . | . | . | . | . | . | . | . | . | . | . | . | . | . |
| 21-225_29D9_LC | . | . | . | . | . | . | . | . | T | . | . | . | . | . | . | . | . |
| 21-225_25G5_LC | . | . | . | . | . | . | . | . | . | . | . | . | . | . | . | . | . |
| 21-225_28B8_LC | . | . | . | . | . | . | . | . | . | . | . | . | . | . | . | . | . |
| 21-225_28A9_LC | . | . | . | . | . | . | . | . | R | . | . | . | . | . | . | . | . |
| 21-225_29D8_LC | . | . | . | . | . | . | . | . | . | . | . | . | . | . | . | . | . |
| 21-225_30F3_LC | S | . | . | . | . | . | . | . | . | . | . | . | . | . | . | . | . |
| 21-225_33B1_LC | . | . | . | . | . | . | . | . | . | . | . | . | . | . | . | . | . |
| 21-225_15B11_LC | . | . | . | . | . | . | . | . | . | . | . | . | . | . | . | . | . |
| 21-225_14C2_LC | . | . | . | . | V | . | . | . | . | . | . | . | . | . | . | . | . |
| 21-225_4C8_LC | . | . | . | . | . | . | . | . | . | . | . | . | . | . | . | . | . |
| 21-225_4F12_LC | . | . | . | . | . | . | . | . | . | . | . | . | . | . | . | . | . |
| 21-225_15H10_LC | S | . | . | . | . | . | . | . | . | . | . | . | . | . | . | . | . |
| 21-225_16H4_LC | . | . | . | . | . | . | . | . | . | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_18H12_LC | . | . | . | . | . | . | . | . | . | . | . | . | . | . | . | . | . |
| 21-225_22B7_LC | . | . | . | . | . | . | . | . | . | . | . | . | . | . | . | . | . |
| 21-225_25A9_LC | . | . | . | . | . | . | . | . | . | . | . | . | . | . | . | . | . |
| 21-225_29D9_LC | . | . | . | . | . | . | . | . | . | . | . | . | . | . | . | . | . |
| 21-225_25G5_LC | . | . | . | . | . | . | . | . | . | . | . | . | . | . | . | . | . |
| 21-225_28B8_LC | . | . | . | . | . | . | . | . | . | . | . | . | . | . | . | . | . |
| 21-225_28A9_LC | . | . | . | . | . | . | . | . | . | . | . | . | . | . | . | . | . |
| 21-225_29D8_LC | . | . | . | . | . | . | . | . | . | . | . | . | . | . | . | . | . |
| 21-225_30F3_LC | . | . | . | . | . | . | . | . | . | . | . | . | . | . | . | . | . |
| 21-225_33B1_LC | . | . | . | . | . | . | . | . | . | . | . | . | . | . | . | . | . |
| 21-225_15B11_LC | . | . | . | . | . | . | . | . | . | . | . | . | . | . | . | . | . |
| 21-225_14C2_LC | . | . | . | . | . | . | . | . | . | . | . | . | . | . | . | . | . |
| 21-225_4C8_LC | . | . | . | . | . | . | . | . | . | . | . | . | . | . | . | . | . |
| 21-225_4F12_LC | . | . | . | . | . | . | . | . | . | . | . | . | . | . | . | . | S |
| 21-225_15H10_LC | . | . | . | . | . | . | . | . | . | . | . | . | . | . | . | . | . |
| 21-225_16H4_LC | . | . | . | . | . | . | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | K_FR4 | | | | | | | | | | |
| CONSENSUS | F | T | F | G | | G | T | K | V | E | I | K | R |
| 21-225_18H12_LC | W | . | . | . | L | . | . | . | . | V | . | . | . |
| 21-225_22B7_LC | L | . | . | . | Q | . | . | . | . | . | . | . | . |
| 21-225_25A9_LC | . | . | . | . | P | . | . | . | . | D | . | . | . |
| 21-225_29D9_LC | . | . | . | . | P | . | . | . | . | D | F | . | . |
| 21-225_25G5_LC | . | . | . | . | P | . | . | . | . | D | . | . | . |
| 21-225_28B8_LC | . | . | . | . | P | . | . | . | . | D | . | N | . |
| 21-225_28A9_LC | . | . | . | . | P | . | . | . | . | D | . | . | . |
| 21-225_29D8_LC | . | . | . | . | P | . | . | . | . | D | . | . | . |
| 21-225_30F3_LC | . | . | . | . | Q | . | . | . | . | . | . | . | . |
| 21-225_33B1_LC | L | . | . | . | Q | . | . | . | . | . | V | . | . |
| 21-225_15B11_LC | . | . | . | . | P | . | . | . | . | D | . | . | . |
| 21-225_14C2_LC | L | . | . | . | Q | . | . | . | . | . | . | R | . |
| 21-225_4C8_LC | L | . | . | . | Q | . | . | . | . | . | . | . | . |
| 21-225_4F12_LC | L | . | . | . | Q | . | . | . | . | . | . | . | . |
| 21-225_15H10_LC | L | . | . | . | Q | . | . | . | . | . | . | N | . |
| 21-225_16H4_LC | W | . | . | . | Q | . | . | . | . | . | . | . | . |

FIGURE 56

Table 46

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | K_FR1 | | | | | | | | | | | | | | | | |
| CONSENSUS | D | I | Q | M | T | Q | S | P | S | S | L | S | A | S | V | G | D |
| 21-225_16F10_LC | . | . | V | . | . | . | T | . | L | . | . | . | V | I | P | . | Q |
| 21-225_18A5_LC | . | . | . | . | . | . | . | . | . | . | . | . | . | . | . | . | . |
| 21-225_20E5_LC | . | . | . | . | . | . | . | . | . | . | . | . | . | . | . | . | . |
| 21-225_18A1_LC | . | . | . | . | . | . | . | . | . | . | . | . | . | . | . | . | . |
| 21-225_19E1_LC | . | . | . | . | . | . | . | . | . | . | . | . | . | . | . | . | . |
| 21-225_17A10_LC | . | . | . | . | . | . | . | . | . | . | . | . | . | . | . | . | . |
| 21-225_18E8_LC | . | . | . | . | . | . | A | . | . | . | . | . | . | . | . | . | . |
| 21-225_16F11_LC | . | . | . | . | . | . | . | . | . | . | . | . | . | . | . | . | . |
| 21-225_18C2_LC | . | . | . | . | . | . | . | . | . | . | . | . | . | . | . | . | . |
| 21-225_20A7_LC | . | . | . | . | . | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B5_LC | . | . | . | . | . | . | . | . | . | . | . | . | . | . | . | . | . |
| 21-225_17E5_LC | . | . | . | . | . | . | . | . | . | . | . | . | . | . | . | . | . |
| 21-225_20D5_LC | . | . | . | . | . | . | . | . | . | . | . | . | . | . | . | . | . |
| 21-225_21G11_LC | . | . | . | . | . | . | . | . | . | . | . | . | . | . | . | . | . |
| 21-225_20E12_LC | . | . | . | . | . | . | . | . | . | . | . | . | . | . | . | . | . |
| 21-225_20D10_LC | . | . | . | . | . | . | . | . | . | . | . | . | . | P | . | . | . |
| 21-225_22A4_LC | . | . | . | . | . | . | . | . | . | . | . | . | . | . | . | . | . |
| 21-225_20H12_LC | . | . | . | . | . | . | . | . | . | . | . | . | . | . | . | . | . |
| 21-225_21H8_LC | . | . | . | . | . | . | . | . | . | . | . | . | . | . | . | . | . |
| 21-225_21E5_LC | . | . | . | . | . | . | . | . | . | . | . | . | . | . | . | . | . |
| 21-225_22H8_LC | . | . | V | . | . | . | T | . | L | . | . | . | V | T | P | . | Q |
| 21-225_23H11_LC | . | . | . | . | . | . | . | . | . | . | . | . | . | . | . | . | . |
| 21-225_21F7_LC | . | . | . | . | . | . | . | . | . | . | . | . | . | . | . | . | . |
| 21-225_22F9_LC | . | . | . | . | . | . | . | . | . | . | . | . | . | . | . | . | . |
| 21-225_21G2_LC | . | . | . | . | . | . | . | . | . | . | . | . | . | . | . | . | . |
| 21-225_22F2_LC | . | . | . | . | . | . | . | . | . | . | . | . | . | . | . | . | . |
| 21-225_23F12_LC | . | . | . | . | . | . | . | . | . | . | . | . | . | . | . | . | . |
| 21-225_28F5_LC | . | . | . | . | . | . | . | . | . | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_28C7_LC | . | . | . | . | . | . | . | . | . | . | . | . | . | . | . | . | . |
| 21-225_25B6_LC | . | . | . | . | . | . | . | . | . | . | . | . | . | . | . | . | . |
| 21-225_26A2_LC | . | . | . | . | . | . | . | . | . | . | . | . | . | . | . | . | . |
| 21-225_29H6_LC | . | . | . | . | . | . | . | . | . | . | . | . | . | . | . | . | . |
| 21-225_25E6_LC | . | . | . | . | . | . | . | . | . | . | . | . | . | . | . | . | . |
| 21-225_25H10_LC | . | . | . | . | . | . | . | . | . | . | . | . | . | . | . | . | . |
| 21-225_29D7_LC | . | . | . | . | . | . | . | . | . | . | . | . | . | . | . | . | . |
| 21-225_29B8_LC | . | . | . | . | . | . | . | . | . | . | . | . | . | . | . | . | . |

**FIGURE 56**

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | K_FR1 | | | | | | | | | | | | | | | | |
| CONSENSUS | D | I | Q | M | T | Q | S | P | S | S | L | S | A | S | V | G | D |
| 21-225_25H11_LC | . | . | . | . | . | . | . | . | . | . | . | . | . | . | . | . | . |
| 21-225_27F2_LC | . | . | . | . | . | . | . | . | . | . | . | . | . | . | . | . | . |
| 21-225_27C3_LC | . | . | . | . | . | . | . | . | . | . | . | . | . | . | . | . | . |
| 21-225_29G8_LC | . | . | . | . | . | . | . | . | . | . | . | . | . | . | . | . | . |
| 21-225_6A11_LC | . | . | . | . | . | . | . | . | . | . | . | . | . | . | . | . | . |
| 21-225_6G12_LC | . | . | . | . | . | . | . | . | . | . | . | . | . | . | . | . | . |
| 21-225_8H7_LC | . | . | . | . | . | . | . | . | . | . | . | . | . | . | . | . | . |
| 21-225_10G5_LC | . | . | . | . | . | . | . | . | . | . | . | . | . | . | . | . | . |
| 21-225_10H12_LC | . | . | . | . | . | . | . | . | . | . | . | . | . | . | . | . | . |
| 21-225_12A1_LC | . | . | . | . | . | . | . | . | . | . | . | . | . | . | . | . | . |
| 21-225_6G2_LC | . | . | . | . | . | . | . | . | . | . | [illegible] | . | . | F | . | . | . |
| 21-225_7H11_LC | . | . | . | . | . | . | . | . | . | . | A | . | . | . | . | . | V |
| 21-225_14E3_LC | . | . | . | . | . | . | . | . | . | . | . | . | . | . | . | . | . |
| 21-225_9A1_LC | . | . | . | . | . | . | . | . | . | . | [illegible] | . | . | F | . | . | . |
| 21-225_15E3_LC | . | . | . | . | . | . | . | . | . | . | . | . | . | . | . | . | . |
| 21-225_16F4_LC | . | . | . | . | I | . | . | . | . | . | P | . | . | . | . | . | . |
| 21-225_16F6_LC | . | . | . | . | . | . | . | . | . | . | . | . | . | . | . | . | . |
| 21-225_2B2_LC | . | . | . | . | . | . | . | . | . | . | . | . | . | . | . | . | . |
| 21-225_13E6_LC | . | . | . | V | . | . | . | . | . | . | . | . | . | . | . | . | . |
| 21-225_16A5_LC | . | . | . | . | . | . | . | . | . | . | . | . | . | . | . | . | . |
| 21-225_7G2_LC | . | . | . | . | . | . | . | . | . | . | . | . | . | . | . | . | . |
| 21-225_7C4_LC | . | . | . | . | . | . | . | . | . | . | . | F | . | . | . | . | . |
| 21-225_7E9_LC | . | . | . | . | . | . | . | . | . | . | . | F | . | . | . | . | . |
| 21-225_8C9_LC | . | . | . | . | . | . | . | . | . | . | . | . | . | . | I | . | . |
| 21-225_12B12_LC | . | . | . | . | . | . | . | . | . | . | . | . | . | . | . | . | . |
| 21-225_16B7_LC | . | . | . | . | . | . | . | . | . | . | . | . | . | . | . | . | . |
| 21-225_9E8_LC | . | . | . | . | . | . | . | . | . | . | . | F | . | . | . | . | . |
| 21-225_12F2_LC | . | . | . | . | . | . | . | . | . | . | . | . | . | . | . | . | . |
| 21-225_12E6_LC | . | . | . | . | . | . | . | . | . | . | . | . | . | . | . | . | . |
| 21-225_16G7_LC | . | . | . | . | . | . | . | . | . | . | . | . | . | . | . | . | . |
| 21-225_1C12_LC | . | . | . | . | . | . | . | . | . | . | . | . | . | . | . | . | . |

FIGURE 56

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | | K_CDR1 | |
| CONSENSUS | R | V | T | I | T | C | R | A | S | - | - | Q | G | I | R | - | - |
| 21-225_16F10_LC | P | A | S | . | S | . | K | S | . | . | . | . | S | L | L | H | S |
| 21-225_18A5_LC | . | . | . | . | . | . | . | . | . | . | . | . | . | . | . | . | . |
| 21-225_20E5_LC | . | . | . | . | . | . | . | . | . | . | . | . | . | . | . | . | . |
| 21-225_18A1_LC | . | . | . | . | . | . | . | . | . | . | . | . | . | . | . | . | . |
| 21-225_19E1_LC | . | . | . | . | . | . | . | . | . | . | . | . | . | . | . | . | . |
| 21-225_17A10_LC | . | . | . | . | . | . | . | . | . | . | . | . | . | . | . | . | . |
| 21-225_18E8_LC | . | . | . | . | . | . | . | . | . | . | . | . | . | . | . | . | . |
| 21-225_16F11_LC | . | . | . | . | . | . | . | . | . | . | . | . | . | . | . | . | . |
| 21-225_18C2_LC | . | . | . | . | . | . | . | . | . | . | . | . | . | . | . | . | . |
| 21-225_20A7_LC | . | . | . | . | . | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B5_LC | . | . | . | . | . | . | . | . | . | . | . | . | . | . | . | . | . |
| 21-225_17E5_LC | . | . | . | . | . | . | . | . | . | . | . | . | . | . | . | . | . |
| 21-225_20D5_LC | . | . | . | . | . | . | . | . | . | . | . | . | . | . | . | . | . |
| 21-225_21G11_LC | . | . | . | . | . | . | . | . | . | . | . | . | . | . | . | . | . |
| 21-225_20E12_LC | . | . | . | . | . | . | . | . | . | . | . | . | . | . | . | . | . |
| 21-225_20D10_LC | . | . | . | . | . | . | . | . | . | . | . | . | . | . | . | . | . |
| 21-225_22A4_LC | . | . | . | . | . | . | . | . | . | . | . | . | . | . | . | . | . |
| 21-225_20H12_LC | . | . | . | . | . | . | . | . | . | . | . | . | . | . | . | . | . |
| 21-225_21H8_LC | . | . | . | . | . | . | . | . | . | . | . | . | . | . | . | . | . |
| 21-225_21E5_LC | . | . | . | . | . | . | . | . | . | . | . | . | . | . | . | . | . |
| 21-225_22H8_LC | P | A | S | . | S | . | K | S | . | . | . | . | S | L | L | H | S |
| 21-225_23H11_LC | . | . | . | . | . | . | . | . | . | . | . | . | . | . | . | . | . |
| 21-225_21F7_LC | . | . | . | . | . | . | . | . | . | . | . | . | D | . | . | . | . |
| 21-225_22F9_LC | . | . | . | . | . | . | . | . | . | . | . | . | . | . | . | . | . |
| 21-225_21G2_LC | . | . | . | . | . | . | . | . | . | . | . | . | . | . | . | . | . |
| 21-225_22F2_LC | . | . | . | . | . | . | . | . | . | . | . | . | . | . | . | . | . |
| 21-225_23F12_LC | . | . | . | . | . | . | . | . | . | . | . | . | . | . | . | . | . |
| 21-225_28F5_LC | . | . | . | . | . | . | . | . | . | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_28C7_LC | . | . | . | . | . | . | . | . | . | . | . | . | . | . | . | . | . |
| 21-225_25B6_LC | . | . | . | . | . | . | . | . | . | . | . | . | . | . | . | . | . |
| 21-225_26A2_LC | . | . | . | . | . | . | . | . | . | . | . | . | . | . | . | . | . |
| 21-225_29H6_LC | . | . | . | . | . | . | . | . | . | . | . | . | . | . | . | . | . |
| 21-225_25E6_LC | . | . | . | . | . | . | . | . | . | . | . | . | . | . | . | . | . |
| 21-225_25H10_LC | . | . | . | . | . | . | . | . | . | . | . | K | . | . | . | . | . |
| 21-225_29D7_LC | . | . | . | . | . | . | . | . | . | . | . | . | . | . | . | . | . |
| 21-225_29B8_LC | . | . | . | . | . | . | . | . | . | . | . | . | . | . | . | . | . |

FIGURE 56

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | K_CDR1 | | | | | | | | | | |
| CONSENSUS | R | V | T | I | T | C | R | A | S | - | - | Q | G | I | R | - | - |
| 21-225_25H11_LC | . | . | . | . | . | . | . | . | . | . | . | . | . | . | . | . | . |
| 21-225_27F2_LC | . | . | . | . | . | . | . | . | . | . | . | . | . | . | . | . | . |
| 21-225_27C3_LC | . | . | . | . | . | . | . | . | . | . | . | . | . | . | . | . | . |
| 21-225_29G8_LC | . | . | . | . | . | . | . | . | . | . | . | . | . | . | . | . | . |
| 21-225_6A11_LC | . | . | . | . | . | . | . | . | . | . | . | . | . | . | . | . | . |
| 21-225_6G12_LC | . | . | . | . | . | . | . | . | . | . | . | . | . | . | . | . | . |
| 21-225_8H7_LC | . | . | . | . | . | . | . | . | . | . | . | . | . | . | . | . | . |
| 21-225_10G5_LC | . | . | . | . | . | . | . | . | . | . | . | . | . | . | . | . | . |
| 21-225_10H12_LC | . | . | . | . | . | . | . | . | . | . | . | . | . | . | . | . | . |
| 21-225_12A1_LC | . | . | . | . | . | . | . | . | . | . | . | . | . | . | . | . | . |
| 21-225_6G2_LC | . | . | . | . | . | Y | . | . | . | . | . | . | . | . | . | . | . |
| 21-225_7H11_LC | . | . | . | . | I | . | L | . | . | . | . | . | . | . | . | . | . |
| 21-225_14E3_LC | . | . | . | . | . | . | . | . | . | . | . | . | . | . | . | . | . |
| 21-225_9A1_LC | . | . | . | . | . | . | . | . | . | . | . | . | . | . | . | . | . |
| 21-225_15E3_LC | . | . | . | . | . | . | . | . | . | . | . | . | . | . | . | . | . |
| 21-225_16F4_LC | . | . | . | . | . | . | . | . | . | . | . | . | . | . | . | . | . |
| 21-225_16F6_LC | . | . | . | . | . | . | Q | . | N | . | . | . | D | . | T | . | . |
| 21-225_2B2_LC | . | . | . | . | . | . | . | . | . | . | . | . | . | . | . | . | . |
| 21-225_13E6_LC | . | . | . | . | . | S | . | . | . | . | . | . | . | . | . | . | . |
| 21-225_16A5_LC | . | . | . | . | . | . | . | . | . | . | . | . | . | . | . | . | . |
| 21-225_7G2_LC | . | . | . | . | . | . | . | . | . | . | . | . | . | . | . | . | . |
| 21-225_7C4_LC | . | . | . | . | . | . | . | . | . | . | . | . | . | . | . | . | . |
| 21-225_7E9_LC | . | . | . | . | . | . | . | . | . | . | . | . | . | . | . | . | . |
| 21-225_8C9_LC | . | . | . | . | . | . | . | . | . | . | . | . | A | . | . | . | . |
| 21-225_12B12_LC | . | . | . | . | . | . | . | . | . | . | . | . | . | . | . | . | . |
| 21-225_16B7_LC | . | . | . | . | . | . | . | . | . | . | . | . | . | . | . | . | . |
| 21-225_9E8_LC | . | . | . | . | . | . | . | . | . | . | . | . | . | . | . | . | . |
| 21-225_12F2_LC | . | . | . | . | . | . | . | . | . | . | . | . | . | . | . | . | . |
| 21-225_12E6_LC | . | . | . | . | . | . | . | . | . | . | . | . | . | . | . | . | . |
| 21-225_16G7_LC | . | . | . | . | . | . | . | . | . | . | . | . | . | . | . | . | . |
| 21-225_1C12_LC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | | K_FR2 | |
| CONSENSUS | - | - | - | - | N | D | L | G | W | Y | Q | Q | K | P | G | K | A |
| 21-225_16F10_LC | D | . | G | K | T | H | . | N | . | . | L | . | . | . | . | Q | P |
| 21-225_18A5_LC | . | . | . | . | . | . | . | . | . | . | . | . | . | . | . | . | . |
| 21-225_20E5_LC | . | . | . | . | . | . | . | . | . | . | . | . | . | . | . | . | . |
| 21-225_18A1_LC | . | . | . | . | . | . | . | . | . | . | . | . | . | . | . | . | . |
| 21-225_19E1_LC | . | . | . | . | . | . | . | . | . | . | . | . | . | . | . | . | . |
| 21-225_17A10_LC | . | . | . | . | . | . | . | . | . | . | . | . | . | . | . | . | . |
| 21-225_18E8_LC | . | . | . | . | . | . | . | . | . | . | . | . | . | . | . | . | . |
| 21-225_16F11_LC | . | . | . | . | . | . | . | . | . | . | . | . | . | . | . | . | . |
| 21-225_18C2_LC | . | . | . | . | . | . | . | . | . | . | . | . | . | . | . | . | . |
| 21-225_20A7_LC | . | . | . | . | . | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B5_LC | . | . | . | . | . | . | . | . | . | . | . | . | . | . | . | . | . |
| 21-225_17E5_LC | . | . | . | . | . | . | . | . | . | . | . | . | . | . | . | . | . |
| 21-225_20D5_LC | . | . | . | . | . | . | . | . | . | . | . | . | . | . | . | . | . |
| 21-225_21G11_LC | . | . | . | . | . | . | . | . | . | . | . | . | . | . | . | . | . |
| 21-225_20E12_LC | . | . | . | . | . | . | . | . | . | . | . | . | . | . | . | . | . |
| 21-225_20D10_LC | . | . | . | . | . | . | . | . | . | . | . | . | . | . | . | . | . |
| 21-225_22A4_LC | . | . | . | . | . | . | . | . | . | . | . | . | . | . | . | . | . |
| 21-225_20H12_LC | . | . | . | . | . | . | . | D | . | . | . | . | . | . | . | . | . |
| 21-225_21H8_LC | . | . | . | . | . | . | . | . | . | . | . | . | . | . | . | . | . |
| 21-225_21E5_LC | . | . | . | . | . | . | . | . | . | . | . | . | . | . | . | . | . |
| 21-225_22H8_LC | D | . | G | K | T | P | . | Y | . | . | L | . | . | . | . | H | P |
| 21-225_23H11_LC | . | . | . | . | . | . | . | D | . | . | . | . | . | . | . | . | . |
| 21-225_21F7_LC | . | . | . | . | . | . | . | . | . | . | . | . | . | . | . | . | . |
| 21-225_22F9_LC | . | . | . | . | . | . | . | . | . | . | . | . | . | . | . | . | . |
| 21-225_21G2_LC | . | . | . | . | . | . | . | . | . | . | . | . | . | . | . | . | . |
| 21-225_22F2_LC | . | . | . | . | . | . | . | . | . | . | . | . | . | . | . | . | . |
| 21-225_23F12_LC | . | . | . | . | . | . | . | . | . | . | . | . | . | . | . | . | . |
| 21-225_28F5_LC | . | . | . | . | . | . | . | . | . | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_28C7_LC | . | . | . | . | . | . | . | . | . | . | . | . | . | . | . | . | . |
| 21-225_25B6_LC | . | . | . | . | . | . | . | . | . | . | . | . | . | . | . | . | . |
| 21-225_26A2_LC | . | . | . | . | S | . | . | . | . | . | . | . | . | . | . | . | . |
| 21-225_29H6_LC | . | . | . | . | . | . | . | . | . | . | . | . | . | S | . | . | T |
| 21-225_25E6_LC | . | . | . | . | . | . | . | . | . | . | . | H | . | . | . | . | . |
| 21-225_25H10_LC | . | . | . | . | . | . | . | . | . | . | . | . | . | . | . | . | . |
| 21-225_29D7_LC | . | . | . | . | . | . | . | . | . | . | . | . | . | . | . | . | . |
| 21-225_29B8_LC | . | . | . | . | . | . | . | . | . | . | . | . | . | . | . | . | . |

**FIGURE 56**

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | K_FR2 | | | | | | | | |
| CONSENSUS | - | - | - | - | N | D | L | G | W | Y | Q | Q | K | P | G | K | A |
| 21-225_25H11_LC | . | . | . | . | T | . | . | . | . | . | . | . | . | . | . | . | . |
| 21-225_27F2_LC | . | . | . | . | . | . | . | . | . | . | . | . | . | . | . | . | . |
| 21-225_27C3_LC | . | . | . | . | . | . | . | . | . | . | . | . | . | . | . | . | . |
| 21-225_29G8_LC | . | . | . | . | . | . | . | . | . | . | . | L | . | . | . | . | . |
| 21-225_6A11_LC | . | . | . | . | . | . | . | . | . | . | . | . | . | . | . | . | . |
| 21-225_6G12_LC | . | . | . | . | . | . | . | . | . | . | . | . | . | . | . | . | . |
| 21-225_8H7_LC | . | . | . | . | . | . | . | . | . | . | . | . | . | . | . | . | . |
| 21-225_10G5_LC | . | . | . | . | . | . | . | . | . | . | . | . | . | . | . | . | . |
| 21-225_10H12_LC | . | . | . | . | . | . | . | . | . | . | . | . | . | . | . | . | . |
| 21-225_12A1_LC | . | . | . | . | . | . | . | . | . | . | . | . | . | . | . | . | . |
| 21-225_6G2_LC | . | . | . | . | . | . | . | . | . | . | . | . | . | . | . | . | . |
| 21-225_7H11_LC | . | . | . | . | . | . | . | . | . | . | . | . | . | . | . | . | . |
| 21-225_14E3_LC | . | . | . | . | . | . | . | . | . | . | . | . | . | . | . | . | . |
| 21-225_9A1_LC | . | . | . | . | . | . | . | . | . | . | . | . | . | . | . | . | . |
| 21-225_15E3_LC | . | . | . | . | . | . | . | . | . | . | . | . | . | . | . | . | . |
| 21-225_16F4_LC | . | . | . | . | . | . | . | . | . | . | . | . | . | S | . | . | . |
| 21-225_16F6_LC | . | . | . | . | . | F | . | N | . | . | . | L | . | . | . | . | . |
| 21-225_2B2_LC | . | . | . | . | . | . | . | . | . | . | . | . | . | . | . | Q | . |
| 21-225_13E6_LC | . | . | . | . | . | . | . | . | . | . | . | . | . | . | . | . | . |
| 21-225_16A5_LC | . | . | . | . | . | . | . | . | C | . | . | . | . | . | . | . | . |
| 21-225_7G2_LC | . | . | . | . | . | . | . | . | . | . | . | . | . | . | . | . | . |
| 21-225_7C4_LC | . | . | . | . | . | . | . | . | . | . | . | . | . | . | . | . | . |
| 21-225_7E9_LC | . | . | . | . | . | . | . | . | . | . | E | . | . | . | . | . | . |
| 21-225_8C9_LC | . | . | . | . | . | . | . | D | . | . | . | . | . | . | . | . | . |
| 21-225_12B12_LC | . | . | . | . | . | . | . | . | . | . | . | . | . | . | . | . | . |
| 21-225_16B7_LC | . | . | . | . | . | . | . | . | . | . | . | . | . | . | . | . | . |
| 21-225_9E8_LC | . | . | . | . | . | . | . | . | . | . | . | . | . | . | . | . | . |
| 21-225_12F2_LC | . | . | . | . | . | . | . | . | . | . | . | . | . | . | . | . | . |
| 21-225_12E6_LC | . | . | . | . | S | . | . | . | . | . | . | . | . | . | . | . | . |
| 21-225_16G7_LC | . | . | . | . | . | . | . | . | . | . | . | . | . | . | . | . | . |
| 21-225_1C12_LC | . | . | . | . | . | . | . | . | . | . | . | . | . | . | . | . | . |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | K_CDR2 | | | | | | | | | | |
| CONSENSUS | P | K | R | L | I | Y | A | - | - | - | - | - | - | - | - | A | S |
| 21-225_16F10_LC | . | Q | L | . | . | . | E | . | . | . | . | . | . | . | . | V | . |
| 21-225_18A5_LC | . | . | . | . | . | . | . | . | . | . | . | . | . | . | . | . | . |
| 21-225_20E5_LC | . | . | . | . | . | . | . | . | . | . | . | . | . | . | . | . | . |
| 21-225_18A1_LC | . | . | . | . | . | . | . | . | . | . | . | . | . | . | . | . | . |
| 21-225_19E1_LC | . | . | . | . | . | . | . | . | . | . | . | . | . | . | . | . | . |
| 21-225_17A10_LC | . | . | . | . | . | . | . | . | . | . | . | . | . | . | . | . | . |
| 21-225_18E8_LC | . | . | . | . | . | . | . | . | . | . | . | . | . | . | . | . | . |
| 21-225_16F11_LC | . | . | . | . | . | . | . | . | . | . | . | . | . | . | . | . | . |
| 21-225_18C2_LC | . | . | . | . | . | . | T | . | . | . | . | . | . | . | . | . | . |
| 21-225_20A7_LC | . | . | . | . | . | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B5_LC | . | . | . | . | . | . | . | . | . | . | . | . | . | . | . | . | . |
| 21-225_17E5_LC | . | . | . | . | . | . | T | . | . | . | . | . | . | . | . | . | . |
| 21-225_20D5_LC | . | . | . | . | . | . | . | . | . | . | . | . | . | . | . | . | . |
| 21-225_21G11_LC | . | . | . | . | . | . | T | . | . | . | . | . | . | . | . | . | . |
| 21-225_20E12_LC | . | . | . | . | . | F | . | . | . | . | . | . | . | . | . | . | . |
| 21-225_20D10_LC | [illegible] | . | . | . | . | . | V | . | . | . | . | . | . | . | . | . | Y |
| 21-225_22A4_LC | . | . | . | . | . | . | . | . | . | . | . | . | . | . | . | . | . |
| 21-225_20H12_LC | . | . | . | . | . | . | . | . | . | . | . | . | . | . | . | . | . |
| 21-225_21H8_LC | . | . | . | . | . | . | . | . | . | . | . | . | . | . | . | . | . |
| 21-225_21E5_LC | . | . | . | . | . | . | . | . | . | . | . | . | . | . | . | T | . |
| 21-225_22H8_LC | . | Q | L | . | . | . | E | . | . | . | . | . | . | . | . | V | . |
| 21-225_23H11_LC | . | . | . | . | . | . | . | . | . | . | . | . | . | . | . | . | . |
| 21-225_21F7_LC | . | . | . | . | . | . | . | . | . | . | . | . | . | . | . | . | . |
| 21-225_22F9_LC | . | . | . | . | . | . | . | . | . | . | . | . | . | . | . | . | . |
| 21-225_21G2_LC | . | . | . | . | . | . | T | . | . | . | . | . | . | . | . | S | . |
| 21-225_22F2_LC | . | . | . | . | . | . | . | . | . | . | . | . | . | . | . | . | . |
| 21-225_23F12_LC | . | . | . | . | . | . | V | . | . | . | . | . | . | . | . | . | . |
| 21-225_28F5_LC | . | . | . | . | . | . | T | . | . | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_28C7_LC | . | . | . | . | . | . | I | . | . | . | . | . | . | . | . | . | . |
| 21-225_25B6_LC | . | . | . | . | . | . | [illegible] | . | . | . | . | . | . | . | . | . | . |
| 21-225_26A2_LC | . | . | . | . | . | . | T | . | . | . | . | . | . | . | . | . | . |
| 21-225_29H6_LC | . | . | . | . | . | . | . | . | . | . | . | . | . | . | . | . | . |
| 21-225_25E6_LC | . | . | . | . | . | . | . | . | . | . | . | . | . | . | . | . | . |
| 21-225_25H10_LC | . | . | . | . | . | . | . | . | . | . | . | . | . | . | . | . | F |
| 21-225_29D7_LC | . | . | . | . | . | . | . | . | . | . | . | . | . | . | . | . | . |
| 21-225_29B8_LC | . | . | . | . | . | . | . | . | . | . | . | . | . | . | . | . | . |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | K_CDR2 | | | | | | | | | | |
| CONSENSUS | P | K | R | L | I | Y | A | - | - | - | - | - | - | - | - | A | S |
| 21-225_25H11_LC | . | . | . | . | . | . | . | . | . | . | . | . | . | . | . | . | . |
| 21-225_27F2_LC | . | . | . | . | . | . | V | . | . | . | . | . | . | . | . | . | . |
| 21-225_27C3_LC | . | . | . | . | . | . | . | . | . | . | . | . | . | . | . | . | . |
| 21-225_29G8_LC | . | . | . | . | . | . | . | . | . | . | . | . | . | . | . | . | . |
| 21-225_6A11_LC | . | . | . | . | . | . | . | . | . | . | . | . | . | . | . | . | . |
| 21-225_6G12_LC | . | . | . | . | . | . | . | . | . | . | . | . | . | . | . | . | . |
| 21-225_8H7_LC | . | . | . | . | . | . | . | . | . | . | . | . | . | . | . | . | . |
| 21-225_10G5_LC | . | . | . | . | . | . | . | . | . | . | . | . | . | . | . | . | . |
| 21-225_10H12_LC | . | . | . | . | . | . | G | . | . | . | . | . | . | . | . | . | . |
| 21-225_12A1_LC | . | . | . | . | . | . | . | . | . | . | . | . | . | . | . | . | . |
| 21-225_6G2_LC | . | Q | . | . | . | . | . | . | . | . | . | . | . | . | . | . | . |
| 21-225_7H11_LC | . | . | . | I | . | . | V | . | . | . | . | . | . | . | . | . | G |
| 21-225_14E3_LC | . | . | . | I | . | . | S | . | . | . | . | . | . | . | . | . | . |
| 21-225_9A1_LC | [illegible] | . | . | . | . | . | T | . | . | . | . | . | . | . | . | . | . |
| 21-225_15E3_LC | . | . | . | . | . | . | . | . | . | . | . | . | . | . | . | . | . |
| 21-225_16F4_LC | . | . | . | . | . | . | V | . | . | . | . | . | . | . | . | . | . |
| 21-225_16F6_LC | . | N | L | . | . | S | D | . | . | . | . | . | . | . | . | . | . |
| 21-225_2B2_LC | . | . | . | . | . | . | . | . | . | . | . | . | . | . | . | . | . |
| 21-225_13E6_LC | . | . | . | . | . | . | . | . | . | . | . | . | . | . | . | . | . |
| 21-225_16A5_LC | . | . | . | . | . | . | . | . | . | . | . | . | . | . | . | . | . |
| 21-225_7G2_LC | . | . | . | . | . | . | T | . | . | . | . | . | . | . | . | . | . |
| 21-225_7C4_LC | [illegible] | . | . | . | . | . | . | . | . | . | . | . | . | . | . | . | . |
| 21-225_7E9_LC | . | . | . | . | . | . | T | . | . | . | . | . | . | . | . | . | . |
| 21-225_8C9_LC | . | . | . | . | . | . | . | . | . | . | . | . | . | . | . | . | . |
| 21-225_12B12_LC | . | . | . | . | . | . | T | . | . | . | . | . | . | . | . | . | . |
| 21-225_16B7_LC | . | . | . | . | . | . | . | . | . | . | . | . | . | . | . | . | . |
| 21-225_9E8_LC | . | . | . | . | . | . | . | . | . | . | . | . | . | . | . | . | . |
| 21-225_12F2_LC | . | . | . | . | . | . | . | . | . | . | . | . | . | . | . | . | . |
| 21-225_12E6_LC | . | . | . | . | . | . | . | . | . | . | . | . | . | . | . | . | . |
| 21-225_16G7_LC | . | . | . | . | . | . | . | . | . | . | . | . | . | . | . | . | . |
| 21-225_1C12_LC | . | . | . | . | . | . | . | . | . | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | R | F | S | G | S | G | S | G | - |
| 21-225_16F10_LC | Y | R | F | . | . | . | . | D | . | . | . | . | . | . | . | . | . |
| 21-225_18A5_LC | . | . | . | . | . | . | . | . | . | . | . | . | . | . | . | . | . |
| 21-225_20E5_LC | . | . | . | . | . | . | . | . | . | . | . | . | . | . | . | . | . |
| 21-225_18A1_LC | . | . | . | . | . | . | . | . | . | . | . | . | . | . | . | . | . |
| 21-225_19E1_LC | N | . | . | . | . | . | . | . | . | . | . | . | . | . | . | . | . |
| 21-225_17A10_LC | . | . | . | . | . | . | . | . | . | . | . | . | . | . | . | . | . |
| 21-225_18E8_LC | . | . | . | . | . | . | . | . | . | . | . | . | . | . | . | . | . |
| 21-225_16F11_LC | . | V | . | . | . | . | . | . | . | . | . | . | . | . | . | . | . |
| 21-225_18C2_LC | . | . | . | . | . | . | . | . | . | . | . | . | . | . | F | . | . |
| 21-225_20A7_LC | . | . | . | . | . | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B5_LC | N | . | . | . | . | . | . | . | . | . | . | . | . | . | . | . | . |
| 21-225_17E5_LC | . | . | . | . | . | . | . | . | . | . | . | . | . | . | . | . | . |
| 21-225_20D5_LC | . | . | . | . | . | . | . | . | . | . | . | . | . | . | . | . | . |
| 21-225_21G11_LC | . | . | . | . | . | . | . | . | . | . | . | . | . | . | . | . | . |
| 21-225_20E12_LC | . | . | . | . | . | . | . | . | . | . | . | . | . | . | . | . | . |
| 21-225_20D10_LC | . | . | . | . | . | . | . | . | . | . | . | . | . | . | Y | . | . |
| 21-225_22A4_LC | . | . | . | . | . | . | . | . | . | . | . | . | . | . | . | . | . |
| 21-225_20H12_LC | . | . | . | . | . | . | . | . | . | . | . | . | . | . | . | . | . |
| 21-225_21H8_LC | . | . | . | . | . | . | . | . | . | . | R | . | . | . | . | . | . |
| 21-225_21E5_LC | . | . | . | . | . | . | . | . | . | . | . | . | . | . | . | . | . |
| 21-225_22H8_LC | N | R | F | . | . | . | . | D | . | . | . | . | . | . | . | . | . |
| 21-225_23H11_LC | . | . | . | . | . | . | . | . | . | . | . | . | . | . | . | . | . |
| 21-225_21F7_LC | N | F | . | . | . | . | . | . | . | . | . | . | . | . | . | . | . |
| 21-225_22F9_LC | . | . | . | . | . | . | . | . | . | . | . | . | . | . | . | . | . |
| 21-225_21G2_LC | . | . | . | . | . | . | . | . | . | . | . | . | . | . | . | . | . |
| 21-225_22F2_LC | . | . | . | . | . | . | . | . | . | . | . | . | . | . | . | . | . |
| 21-225_23F12_LC | . | . | . | . | . | . | . | . | . | . | . | . | . | . | . | . | . |
| 21-225_28F5_LC | . | . | . | . | . | . | . | . | . | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_28C7_LC | . | . | . | . | . | . | . | . | . | . | . | . | . | . | . | . | . |
| 21-225_25B6_LC | . | . | . | . | . | . | . | . | . | . | . | . | . | . | . | . | . |
| 21-225_26A2_LC | . | . | . | . | . | . | . | . | . | . | . | . | . | . | . | . | . |
| 21-225_29H6_LC | . | . | . | . | . | . | . | . | . | . | . | . | . | . | . | . | . |
| 21-225_25E6_LC | . | . | . | . | . | . | . | . | . | . | R | . | . | . | . | . | . |
| 21-225_25H10_LC | . | . | . | . | . | . | . | . | . | . | . | . | . | . | . | . | . |
| 21-225_29D7_LC | . | . | . | . | . | . | . | . | . | . | . | . | . | . | . | . | . |
| 21-225_29B8_LC | . | . | . | . | . | . | . | . | . | . | . | . | . | R | . | . | . |

**FIGURE 56**

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | R | F | S | G | S | G | S | G | - |
| 21-225_25H11_LC | . | . | . | . | . | . | . | . | . | . | . | . | . | . | . | . | . |
| 21-225_27F2_LC | . | . | . | . | . | . | . | . | . | . | . | . | . | . | . | . | . |
| 21-225_27C3_LC | . | . | . | . | . | . | . | . | . | . | . | . | . | . | . | . | . |
| 21-225_29G8_LC | . | . | . | . | . | . | . | . | . | . | . | . | . | . | . | . | . |
| 21-225_6A11_LC | . | . | . | . | . | . | . | . | . | . | . | . | . | . | . | . | . |
| 21-225_6G12_LC | T | . | . | . | . | . | . | . | . | . | . | . | . | . | . | . | . |
| 21-225_8H7_LC | . | . | . | . | . | . | . | . | . | . | . | . | . | . | . | . | . |
| 21-225_10G5_LC | . | . | . | . | . | . | . | . | . | . | . | . | . | . | . | . | . |
| 21-225_10H12_LC | . | . | . | . | . | . | . | . | . | . | . | . | . | . | . | . | . |
| 21-225_12A1_LC | . | . | . | . | . | . | . | . | . | . | . | . | . | . | . | . | . |
| 21-225_6G2_LC | . | . | . | . | . | . | . | . | . | . | . | . | . | . | Y | . | . |
| 21-225_7H11_LC | . | F | . | . | . | . | . | . | . | . | . | . | . | . | Y | . | . |
| 21-225_14E3_LC | . | . | . | . | . | . | . | . | . | . | I | . | . | . | C | . | . |
| 21-225_9A1_LC | . | . | . | . | . | . | . | . | . | . | . | . | . | . | Y | . | . |
| 21-225_15E3_LC | . | S | . | . | . | . | . | . | . | . | . | . | . | R | . | . | . |
| 21-225_16F4_LC | . | . | . | . | . | . | . | . | . | . | . | . | . | . | . | . | . |
| 21-225_16F6_LC | N | . | E | T | . | . | . | . | . | . | . | . | . | . | . | . | . |
| 21-225_2B2_LC | . | . | . | . | . | . | . | . | . | . | . | . | . | . | . | . | . |
| 21-225_13E6_LC | . | . | . | . | . | . | . | . | . | . | . | . | . | . | . | . | . |
| 21-225_16A5_LC | . | . | . | . | . | . | . | . | . | . | . | . | N | . | Y | . | . |
| 21-225_7G2_LC | . | . | . | . | . | . | . | . | . | . | . | . | . | . | . | . | . |
| 21-225_7C4_LC | . | . | . | . | . | . | . | . | . | . | . | . | . | . | . | . | . |
| 21-225_7E9_LC | . | . | . | . | . | . | . | . | . | . | . | . | . | . | . | . | . |
| 21-225_8C9_LC | . | . | . | . | . | . | . | . | . | . | . | . | . | . | . | . | . |
| 21-225_12B12_LC | . | . | . | . | . | . | . | . | . | . | . | . | . | . | . | . | . |
| 21-225_16B7_LC | . | . | . | . | . | . | . | . | . | . | . | . | . | . | . | . | . |
| 21-225_9E8_LC | . | . | . | . | . | . | . | . | . | . | . | . | . | . | . | . | . |
| 21-225_12F2_LC | . | . | . | . | . | . | . | . | . | . | . | . | . | . | . | . | . |
| 21-225_12E6_LC | . | . | . | . | . | . | . | . | . | . | . | . | . | . | . | . | . |
| 21-225_16G7_LC | . | . | . | . | . | . | . | . | . | . | . | . | . | . | . | . | . |
| 21-225_1C12_LC | . | . | . | . | . | . | . | . | . | . | . | . | . | . | . | . | . |

FIGURE 56

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | K_FR3 | | | | | | | | | | | | | | | | |
| CONSENSUS | - | T | E | F | T | L | T | I | S | S | L | Q | P | E | D | F | A |
| 21-225_16F10_LC | . | . | V | . | . | . | S | . | . | R | V | E | A | A | . | V | G |
| 21-225_18A5_LC | . | . | . | . | . | . | . | . | . | . | . | . | . | . | . | . | . |
| 21-225_20E5_LC | . | . | . | . | . | . | . | . | . | . | . | . | . | . | . | . | . |
| 21-225_18A1_LC | . | . | . | . | . | . | . | . | . | . | . | . | . | . | . | . | . |
| 21-225_19E1_LC | . | . | . | . | . | . | . | . | . | . | . | . | . | . | . | . | . |
| 21-225_17A10_LC | . | . | . | . | . | . | . | . | . | . | . | . | . | . | . | . | . |
| 21-225_18E8_LC | . | . | . | . | . | . | . | . | . | . | . | . | . | . | . | . | . |
| 21-225_16F11_LC | . | . | . | . | . | . | . | . | . | . | . | . | . | . | . | . | . |
| 21-225_18C2_LC | . | . | . | . | . | . | . | . | . | . | . | . | . | . | . | . | . |
| 21-225_20A7_LC | . | . | . | . | S | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B5_LC | . | . | . | . | S | P | . | . | . | . | . | . | . | . | . | . | . |
| 21-225_17E5_LC | . | . | . | . | . | . | . | . | . | . | . | . | . | . | . | . | . |
| 21-225_20D5_LC | . | . | . | . | . | . | . | . | . | . | . | . | . | . | . | . | . |
| 21-225_21G11_LC | . | . | . | . | . | . | . | . | . | . | . | . | . | . | . | . | . |
| 21-225_20E12_LC | . | . | . | . | . | . | . | . | . | . | . | . | . | . | . | . | . |
| 21-225_20D10_LC | . | . | . | . | . | . | . | . | . | . | . | . | . | . | . | . | . |
| 21-225_22A4_LC | . | . | . | . | . | . | . | . | . | . | . | . | . | . | . | . | . |
| 21-225_20H12_LC | . | . | . | . | . | . | . | . | . | . | . | . | . | . | . | . | . |
| 21-225_21H8_LC | . | . | . | . | . | . | . | . | . | . | . | . | . | . | . | . | . |
| 21-225_21E5_LC | . | . | . | . | . | . | . | . | . | . | . | . | . | . | . | . | . |
| 21-225_22H8_LC | . | . | D | . | . | . | K | . | . | R | V | E | A | . | . | V | G |
| 21-225_23H11_LC | . | . | . | . | . | . | . | . | . | . | . | . | . | . | . | . | . |
| 21-225_21F7_LC | . | . | . | . | . | . | . | . | . | . | . | . | . | . | . | . | . |
| 21-225_22F9_LC | . | . | . | . | . | . | . | . | . | . | . | . | . | . | . | . | . |
| 21-225_21G2_LC | . | . | . | . | . | . | . | . | . | . | . | . | . | . | . | . | . |
| 21-225_22F2_LC | . | . | . | . | . | . | . | . | . | . | . | . | . | . | . | . | . |
| 21-225_23F12_LC | . | . | . | . | . | . | . | . | . | . | . | . | . | . | . | . | . |
| 21-225_28F5_LC | . | . | . | . | . | . | . | . | . | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_28C7_LC | . | . | . | . | . | . | . | . | . | . | . | . | . | . | . | . | . |
| 21-225_25B6_LC | . | . | . | . | . | . | . | . | . | . | . | . | . | . | . | . | . |
| 21-225_26A2_LC | . | . | . | . | . | . | . | P | . | . | . | . | . | . | . | . | . |
| 21-225_29H6_LC | . | . | . | . | . | . | . | . | . | . | . | . | . | . | . | . | . |
| 21-225_25E6_LC | . | . | . | . | . | . | . | . | N | . | . | . | . | . | . | . | . |
| 21-225_25H10_LC | . | . | . | . | . | . | . | . | . | . | . | . | . | . | . | . | . |
| 21-225_29D7_LC | . | . | . | . | . | . | . | . | . | . | . | . | . | . | . | . | . |
| 21-225_29B8_LC | . | . | . | . | . | . | . | . | . | . | . | . | . | . | . | . | . |

FIGURE 56

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | K_FR3 | | | | | | | | | | | | | | | | |
| CONSENSUS | - | T | E | F | T | L | T | I | S | S | L | Q | P | E | D | F | A |
| 21-225_16F10_LC | . | . | V | . | . | . | S | . | . | R | V | E | A | A | . | V | G |
| 21-225_18A5_LC | . | . | . | . | . | . | . | . | . | . | . | . | . | . | . | . | . |
| 21-225_20E5_LC | . | . | . | . | . | . | . | . | . | . | . | . | . | . | . | . | . |
| 21-225_18A1_LC | . | . | . | . | . | . | . | . | . | . | . | . | . | . | . | . | . |
| 21-225_19E1_LC | . | . | . | . | . | . | . | . | . | . | . | . | . | . | . | . | . |
| 21-225_17A10_LC | . | . | . | . | . | . | . | . | . | . | . | . | . | . | . | . | . |
| 21-225_18E8_LC | . | . | . | . | . | . | . | . | . | . | . | . | . | . | . | . | . |
| 21-225_16F11_LC | . | . | . | . | . | . | . | . | . | . | . | . | . | . | . | . | . |
| 21-225_18C2_LC | . | . | . | . | . | . | . | . | . | . | . | . | . | . | . | . | . |
| 21-225_20A7_LC | . | . | . | . | S | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B5_LC | . | . | . | . | S | F | . | . | . | . | . | . | . | . | . | . | . |
| 21-225_17E5_LC | . | . | . | . | . | . | . | . | . | . | . | . | . | . | . | . | . |
| 21-225_20D5_LC | . | . | . | . | . | . | . | . | . | . | . | . | . | . | . | . | . |
| 21-225_21G11_LC | . | . | . | . | . | . | . | . | . | . | . | . | . | . | . | . | . |
| 21-225_20E12_LC | . | . | . | . | . | . | . | . | . | . | . | . | . | . | . | . | . |
| 21-225_20D10_LC | . | . | . | . | . | . | . | . | . | . | . | . | . | . | . | . | . |
| 21-225_22A4_LC | . | . | . | . | . | . | . | . | . | . | . | . | . | . | . | . | . |
| 21-225_20H12_LC | . | . | . | . | . | . | . | . | . | . | . | . | . | . | . | . | . |
| 21-225_21H8_LC | . | . | . | . | . | . | . | . | . | . | . | . | . | . | . | . | . |
| 21-225_21E5_LC | . | . | . | . | . | . | . | . | . | . | . | . | . | . | . | . | . |
| 21-225_22H8_LC | . | . | D | . | . | . | K | . | . | R | V | E | A | . | . | V | G |
| 21-225_23H11_LC | . | . | . | . | . | . | . | . | . | . | . | . | . | . | . | . | . |
| 21-225_21F7_LC | . | . | . | . | . | . | . | . | . | . | . | . | . | . | . | . | . |
| 21-225_22F9_LC | . | . | . | . | . | . | . | . | . | . | . | . | . | . | . | . | . |
| 21-225_21G2_LC | . | . | . | . | . | . | . | . | . | . | . | . | . | . | . | . | . |
| 21-225_22F2_LC | . | . | . | . | . | . | . | . | . | . | . | . | . | . | . | . | . |
| 21-225_23F12_LC | . | . | . | . | . | . | . | . | . | . | . | . | . | . | . | . | . |
| 21-225_28F5_LC | . | . | . | . | . | . | . | . | . | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_28C7_LC | . | . | . | . | . | . | . | . | . | . | . | . | . | . | . | . | . |
| 21-225_25B6_LC | . | . | . | . | . | . | . | . | . | . | . | . | . | . | . | . | . |
| 21-225_26A2_LC | . | . | . | . | . | . | . | F | . | . | . | . | . | . | . | . | . |
| 21-225_29H6_LC | . | . | . | . | . | . | . | . | . | . | . | . | . | . | . | . | . |
| 21-225_25E6_LC | . | . | . | . | . | . | . | . | N | . | . | . | . | . | . | . | . |
| 21-225_25H10_LC | . | . | . | . | . | . | . | . | . | . | . | . | . | . | . | . | . |
| 21-225_29D7_LC | . | . | . | . | . | . | . | . | . | . | . | . | . | . | . | . | . |
| 21-225_29B8_LC | . | . | . | . | . | . | . | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | L | Q | H | N | S | - | - | - | - | - | - | - | - |
| 21-225_16F10_LC | V | . | . | . | M | . | S | I | Q | . | . | . | . | . | . | . | . |
| 21-225_18A5_LC | . | . | . | . | . | . | . | . | . | . | . | . | . | . | . | . | . |
| 21-225_20E5_LC | . | . | . | . | . | . | . | . | . | . | . | . | . | . | . | . | . |
| 21-225_18A1_LC | . | . | . | . | . | . | . | . | . | . | . | . | . | . | . | . | . |
| 21-225_19E1_LC | . | . | . | . | . | . | . | . | . | . | . | . | . | . | . | . | . |
| 21-225_17A10_LC | . | . | . | . | . | . | . | . | . | . | . | . | . | . | . | . | . |
| 21-225_18E8_LC | . | . | . | . | . | . | . | . | . | . | . | . | . | . | . | . | . |
| 21-225_16F11_LC | . | . | . | . | . | . | . | . | . | . | . | . | . | . | . | . | . |
| 21-225_18C2_LC | . | . | . | . | . | . | . | . | . | . | . | . | . | . | . | . | . |
| 21-225_20A7_LC | . | . | . | . | . | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B5_LC | . | . | . | . | . | . | . | . | . | . | . | . | . | . | . | . | . |
| 21-225_17E5_LC | . | . | . | . | . | . | . | . | . | . | . | . | . | . | . | . | . |
| 21-225_20D5_LC | . | . | . | . | . | . | . | . | . | . | . | . | . | . | . | . | . |
| 21-225_21G11_LC | . | . | . | . | . | . | . | . | N | . | . | . | . | . | . | . | . |
| 21-225_20E12_LC | . | . | . | . | . | . | . | . | . | . | . | . | . | . | . | . | . |
| 21-225_20D10_LC | . | . | . | . | I | . | Q | . | . | . | . | . | . | . | . | . | . |
| 21-225_22A4_LC | . | . | . | . | . | . | . | . | . | . | . | . | . | . | . | . | . |
| 21-225_20H12_LC | . | . | . | . | . | . | . | . | . | . | . | . | . | . | . | . | . |
| 21-225_21H8_LC | . | . | . | . | . | . | . | . | . | . | . | . | . | . | . | . | . |
| 21-225_21E5_LC | . | . | . | . | . | . | . | . | . | . | . | . | . | . | . | . | . |
| 21-225_22H8_LC | I | . | . | . | M | . | S | I | Q | . | . | . | . | . | . | . | . |
| 21-225_23H11_LC | . | . | . | . | . | . | . | . | [illegible] | . | . | . | . | . | . | . | . |
| 21-225_21F7_LC | . | . | . | . | . | . | . | . | N | . | . | . | . | . | . | . | . |
| 21-225_22F9_LC | . | . | . | . | . | . | . | . | . | . | . | . | . | . | . | . | . |
| 21-225_21G2_LC | . | . | . | . | . | . | . | . | . | . | . | . | . | . | . | . | . |
| 21-225_22F2_LC | . | . | . | . | . | . | . | . | . | . | . | . | . | . | . | . | . |
| 21-225_23F12_LC | S | . | . | . | . | . | . | . | . | . | . | . | . | . | . | . | . |
| 21-225_28F5_LC | . | . | . | . | . | . | . | . | T | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_28C7_LC | . | . | . | . | . | . | . | . | T | . | . | . | . | . | . | . | . |
| 21-225_25B6_LC | . | . | . | . | . | . | . | . | . | . | . | . | . | . | . | . | . |
| 21-225_26A2_LC | . | . | . | . | . | . | . | . | [illegible] | . | . | . | . | . | . | . | . |
| 21-225_29H6_LC | . | . | . | . | . | . | . | . | . | . | . | . | . | . | . | . | . |
| 21-225_25E6_LC | . | . | . | . | . | . | . | . | . | . | . | . | . | . | . | . | . |
| 21-225_25H10_LC | . | . | . | . | . | . | . | . | . | . | . | . | . | . | . | . | . |
| 21-225_29D7_LC | . | . | . | . | . | . | . | . | . | . | . | . | . | . | . | . | . |
| 21-225_29B8_LC | . | . | . | . | . | . | . | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | L | Q | H | N | S | - | - | - | - | - | - | - | - |
| 21-225_25H11_LC | . | . | . | . | . | . | . | . | . | . | . | . | . | . | . | . | . |
| 21-225_27F2_LC | . | . | . | . | . | . | . | . | . | . | . | . | . | . | . | . | . |
| 21-225_27G3_LC | . | . | . | . | . | . | . | . | R | . | . | . | . | . | . | . | . |
| 21-225_29G8_LC | . | . | N | . | . | . | . | . | . | . | . | . | . | . | . | . | . |
| 21-225_6A11_LC | . | . | . | . | . | . | . | . | . | . | . | . | . | . | . | . | . |
| 21-225_6G12_LC | . | . | . | . | . | . | . | . | . | . | . | . | . | . | . | . | . |
| 21-225_8H7_LC | . | . | . | . | . | . | . | . | . | . | . | . | . | . | . | . | . |
| 21-225_10G5_LC | . | . | . | . | . | . | . | . | . | . | . | . | . | . | . | . | . |
| 21-225_10H12_LC | . | . | . | . | . | . | . | . | . | . | . | . | . | . | . | . | . |
| 21-225_12A1_LC | . | . | . | . | . | . | . | . | . | . | . | . | . | . | . | . | . |
| 21-225_6G2_LC | I | . | . | . | I | . | . | . | . | . | . | . | . | . | . | . | . |
| 21-225_7H11_LC | . | . | . | . | . | . | . | . | L | . | . | . | . | . | . | . | . |
| 21-225_14E3_LC | A | . | . | S | V | . | . | Y | . | . | . | . | . | . | . | . | . |
| 21-225_9A1_LC | . | . | . | . | I | . | . | . | . | . | . | . | . | . | . | . | . |
| 21-225_15E3_LC | A | . | . | . | . | . | . | H | . | . | . | . | . | . | . | . | . |
| 21-225_16F4_LC | . | . | . | . | I | . | . | . | . | . | . | . | . | . | . | . | . |
| 21-225_16F6_LC | . | . | . | . | Q | . | Y | D | N | . | . | . | . | . | . | . | . |
| 21-225_2B2_LC | . | . | H | . | . | . | . | . | . | . | . | . | . | . | . | . | . |
| 21-225_13E6_LC | . | . | . | . | V | . | . | . | . | . | . | . | . | . | . | . | . |
| 21-225_16A5_LC | . | . | . | . | . | . | . | . | . | . | . | . | . | . | . | . | . |
| 21-225_7G2_LC | . | . | . | . | . | . | . | . | . | . | . | . | . | . | . | . | . |
| 21-225_7C4_LC | . | . | . | . | . | . | . | . | . | . | . | . | . | . | . | . | . |
| 21-225_7E9_LC | . | . | . | . | . | . | . | . | . | . | . | . | . | . | . | . | . |
| 21-225_8C9_LC | . | . | . | . | . | . | . | . | . | . | . | . | . | . | . | . | . |
| 21-225_12B12_LC | . | . | . | . | . | . | . | . | . | . | . | . | . | . | . | . | . |
| 21-225_16B7_LC | . | . | . | . | . | . | . | . | . | . | . | . | . | . | . | . | . |
| 21-225_9E8_LC | . | . | . | . | . | . | . | . | . | . | . | . | . | . | . | . | . |
| 21-225_12F2_LC | . | . | . | . | . | . | . | . | . | . | . | . | . | . | . | . | . |
| 21-225_12E6_LC | . | . | . | . | . | . | . | . | . | . | . | . | . | . | . | . | . |
| 21-225_16G7_LC | . | . | . | . | . | . | . | . | . | . | . | . | . | . | . | . | . |
| 21-225_1C12_LC | . | . | . | . | . | . | . | . | . | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_16F10_LC | . | . | . | . | . | . | . | . | . | . | . | . | . | . | . | L | . |
| 21-225_18A5_LC | . | . | . | . | . | . | . | . | . | . | . | . | . | . | . | . | . |
| 21-225_20E5_LC | . | . | . | . | . | . | . | . | . | . | . | . | . | . | . | . | . |
| 21-225_18A1_LC | . | . | . | . | . | . | . | . | . | . | . | . | . | . | . | . | . |
| 21-225_19E1_LC | . | . | . | . | . | . | . | . | . | . | . | . | . | . | . | W | . |
| 21-225_17A10_LC | . | . | . | . | . | . | . | . | . | . | . | . | . | . | . | . | . |
| 21-225_18E8_LC | . | . | . | . | . | . | . | . | . | . | . | . | . | . | . | . | . |
| 21-225_16F11_LC | . | . | . | . | . | . | . | . | . | . | . | . | . | . | . | . | . |
| 21-225_18C2_LC | . | . | . | . | . | . | . | . | . | . | . | . | . | . | . | . | . |
| 21-225_20A7_LC | . | . | . | . | . | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B5_LC | . | . | . | . | . | . | . | . | . | . | . | . | . | . | . | . | . |
| 21-225_17E5_LC | . | . | . | . | . | . | . | . | . | . | . | . | . | . | . | . | . |
| 21-225_20D5_LC | . | . | . | . | . | . | . | . | . | . | . | . | . | . | . | . | . |
| 21-225_21G11_LC | . | . | . | . | . | . | . | . | . | . | . | . | . | . | . | . | . |
| 21-225_20E12_LC | . | . | . | . | . | . | . | . | . | . | . | . | . | . | . | . | . |
| 21-225_20D10_LC | . | . | . | . | . | . | . | . | . | . | . | . | . | . | . | . | . |
| 21-225_22A4_LC | . | . | . | . | . | . | . | . | . | . | . | . | . | . | . | . | . |
| 21-225_20H12_LC | . | . | . | . | . | . | . | . | . | . | . | . | . | . | . | . | . |
| 21-225_21H8_LC | . | . | . | . | . | . | . | . | . | . | . | . | . | . | . | . | . |
| 21-225_21E5_LC | . | . | . | . | . | . | . | . | . | . | . | . | . | . | . | . | . |
| 21-225_22H8_LC | . | . | . | . | . | . | . | . | . | . | . | . | . | . | . | L | . |
| 21-225_23H11_LC | . | . | . | . | . | . | . | . | . | . | . | . | . | . | . | . | . |
| 21-225_21F7_LC | . | . | . | . | . | . | . | . | . | . | . | . | . | . | . | . | . |
| 21-225_22F9_LC | . | . | . | . | . | . | . | . | . | . | . | . | . | . | . | . | . |
| 21-225_21G2_LC | . | . | . | . | . | . | . | . | . | . | . | . | . | . | . | . | . |
| 21-225_22F2_LC | . | . | . | . | . | . | . | . | . | . | . | . | . | . | . | . | . |
| 21-225_23F12_LC | . | . | . | . | . | . | . | . | . | . | . | . | . | . | . | . | . |
| 21-225_28F5_LC | . | . | . | . | . | . | . | . | . | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_28C7_LC | . | . | . | . | . | . | . | . | . | . | . | . | . | . | . | . | . |
| 21-225_25B6_LC | . | . | . | . | . | . | . | . | . | . | . | . | . | . | . | . | . |
| 21-225_26A2_LC | . | . | . | . | . | . | . | . | . | . | . | . | . | . | . | . | . |
| 21-225_29H6_LC | . | . | . | . | . | . | . | . | . | . | . | . | . | . | . | . | . |
| 21-225_25E6_LC | . | . | . | . | . | . | . | . | . | . | . | . | . | . | . | . | . |
| 21-225_25H10_LC | . | . | . | . | . | . | . | . | . | . | . | . | . | . | . | . | . |
| 21-225_29D7_LC | . | . | . | . | . | . | . | . | . | . | . | . | . | . | . | . | . |
| 21-225_29B8_LC | . | . | . | . | . | . | . | . | . | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_16F10_LC | . | . | . | . | . | . | . | . | . | . | . | . | . | . | . | [illegible] | . |
| 21-225_18A5_LC | . | . | . | . | . | . | . | . | . | . | . | . | . | . | . | . | . |
| 21-225_20E5_LC | . | . | . | . | . | . | . | . | . | . | . | . | . | . | . | . | . |
| 21-225_18A1_LC | . | . | . | . | . | . | . | . | . | . | . | . | . | . | . | . | . |
| 21-225_19E1_LC | . | . | . | . | . | . | . | . | . | . | . | . | . | . | . | [illegible] | . |
| 21-225_17A10_LC | . | . | . | . | . | . | . | . | . | . | . | . | . | . | . | . | . |
| 21-225_18E8_LC | . | . | . | . | . | . | . | . | . | . | . | . | . | . | . | . | . |
| 21-225_16F11_LC | . | . | . | . | . | . | . | . | . | . | . | . | . | . | . | . | . |
| 21-225_18C2_LC | . | . | . | . | . | . | . | . | . | . | . | . | . | . | . | . | . |
| 21-225_20A7_LC | . | . | . | . | . | . | . | . | . | . | . | . | . | . | . | . | . |
| 21-225_17C7_LC | . | . | . | . | . | . | . | . | . | . | . | . | . | . | . | . | . |
| 21-225_18F2_LC | . | . | . | . | . | . | . | . | . | . | . | . | . | . | . | . | . |
| 21-225_17B5_LC | . | . | . | . | . | . | . | . | . | . | . | . | . | . | . | . | . |
| 21-225_17E5_LC | . | . | . | . | . | . | . | . | . | . | . | . | . | . | . | . | . |
| 21-225_20D5_LC | . | . | . | . | . | . | . | . | . | . | . | . | . | . | . | . | . |
| 21-225_21G11_LC | . | . | . | . | . | . | . | . | . | . | . | . | . | . | . | . | . |
| 21-225_20E12_LC | . | . | . | . | . | . | . | . | . | . | . | . | . | . | . | . | . |
| 21-225_20D10_LC | . | . | . | . | . | . | . | . | . | . | . | . | . | . | . | . | . |
| 21-225_22A4_LC | . | . | . | . | . | . | . | . | . | . | . | . | . | . | . | . | . |
| 21-225_20H12_LC | . | . | . | . | . | . | . | . | . | . | . | . | . | . | . | . | . |
| 21-225_21H8_LC | . | . | . | . | . | . | . | . | . | . | . | . | . | . | . | . | . |
| 21-225_21E5_LC | . | . | . | . | . | . | . | . | . | . | . | . | . | . | . | . | . |
| 21-225_22H8_LC | . | . | . | . | . | . | . | . | . | . | . | . | . | . | . | [illegible] | . |
| 21-225_23H11_LC | . | . | . | . | . | . | . | . | . | . | . | . | . | . | . | . | . |
| 21-225_21F7_LC | . | . | . | . | . | . | . | . | . | . | . | . | . | . | . | . | . |
| 21-225_22F9_LC | . | . | . | . | . | . | . | . | . | . | . | . | . | . | . | . | . |
| 21-225_21G2_LC | . | . | . | . | . | . | . | . | . | . | . | . | . | . | . | . | . |
| 21-225_22F2_LC | . | . | . | . | . | . | . | . | . | . | . | . | . | . | . | . | . |
| 21-225_23F12_LC | . | . | . | . | . | . | . | . | . | . | . | . | . | . | . | . | . |
| 21-225_28F5_LC | . | . | . | . | . | . | . | . | . | . | . | . | . | . | . | . | . |
| 21-225_29G4_LC | . | . | . | . | . | . | . | . | . | . | . | . | . | . | . | . | . |
| 21-225_27D5_LC | . | . | . | . | . | . | . | . | . | . | . | . | . | . | . | . | . |
| 21-225_28C7_LC | . | . | . | . | . | . | . | . | . | . | . | . | . | . | . | . | . |
| 21-225_25B6_LC | . | . | . | . | . | . | . | . | . | . | . | . | . | . | . | . | . |
| 21-225_26A2_LC | . | . | . | . | . | . | . | . | . | . | . | . | . | . | . | . | . |
| 21-225_29H6_LC | . | . | . | . | . | . | . | . | . | . | . | . | . | . | . | . | . |
| 21-225_25E6_LC | . | . | . | . | . | . | . | . | . | . | . | . | . | . | . | . | . |
| 21-225_25H10_LC | . | . | . | . | . | . | . | . | . | . | . | . | . | . | . | . | . |
| 21-225_29D7_LC | . | . | . | . | . | . | . | . | . | . | . | . | . | . | . | . | . |
| 21-225_29B8_LC | . | . | . | . | . | . | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | K_FR4 | | | | | | | | | | |
| CONSENSUS | L | T | F | G | G | G | T | K | V | E | I | K | R |
| 21-225_16F10_LC | . | . | . | . | . | . | . | . | . | . | . | . | . |
| 21-225_18A5_LC | W | . | . | . | Q | . | . | . | . | . | . | . | . |
| 21-225_20E5_LC | . | . | . | . | . | . | . | . | . | . | . | . | . |
| 21-225_18A1_LC | . | . | . | . | . | . | . | . | . | . | . | . | . |
| 21-225_19E1_LC | . | . | . | . | . | . | . | . | . | . | . | . | . |
| 21-225_17A10_LC | . | . | . | . | . | . | . | . | . | . | . | . | . |
| 21-225_18E8_LC | . | . | . | . | . | . | . | . | . | . | . | . | . |
| 21-225_16F11_LC | . | . | . | . | . | . | . | . | . | . | . | . | . |
| 21-225_18C2_LC | W | . | . | . | L | . | . | . | . | V | . | . | . |
| 21-225_20A7_LC | . | . | . | . | . | . | . | . | . | . | . | . | . |
| 21-225_17C7_LC | W | . | . | . | Q | . | . | . | . | . | . | . | . |
| 21-225_18F2_LC | W | . | . | . | Q | . | . | . | . | . | . | . | . |
| 21-225_17B5_LC | . | . | . | . | . | . | . | . | . | . | . | . | . |
| 21-225_17E5_LC | . | . | . | . | P | . | . | . | . | V | . | . | . |
| 21-225_20D5_LC | F | . | . | . | . | . | . | . | . | . | . | . | . |
| 21-225_21G11_LC | W | . | . | . | L | . | . | . | . | V | . | . | . |
| 21-225_20E12_LC | F | . | . | . | . | . | . | . | . | . | . | . | . |
| 21-225_20D10_LC | W | . | . | . | Q | . | . | . | . | . | . | . | . |
| 21-225_22A4_LC | W | . | . | . | Q | . | . | . | . | . | . | . | . |
| 21-225_20H12_LC | . | . | . | . | . | . | . | . | . | . | . | . | . |
| 21-225_21H8_LC | W | . | . | . | Q | . | . | . | . | . | . | . | . |
| 21-225_21E5_LC | . | . | . | . | . | . | . | . | . | . | . | . | . |
| 21-225_22H8_LC | . | S | . | . | . | . | . | . | . | . | . | N | . |
| 21-225_23H11_LC | . | . | . | . | . | . | . | . | . | . | . | . | . |
| 21-225_21F7_LC | W | . | . | . | Q | . | . | . | . | . | . | . | . |
| 21-225_22F9_LC | . | . | . | . | . | . | . | . | . | . | . | . | . |
| 21-225_21G2_LC | W | . | . | . | L | . | . | . | . | V | . | . | . |
| 21-225_22F2_LC | . | . | . | . | . | . | . | . | . | . | . | . | . |
| 21-225_23F12_LC | W | . | . | . | Q | . | . | . | . | . | . | . | . |
| 21-225_28F5_LC | W | . | . | . | Q | . | . | . | . | . | V | . | . |
| 21-225_29G4_LC | . | . | . | . | . | . | . | . | . | . | . | T | . |
| 21-225_27D5_LC | F | . | . | . | P | . | . | . | . | D | . | . | . |
| 21-225_28C7_LC | W | . | . | . | Q | . | . | . | . | . | . | . | . |
| 21-225_25B6_LC | F | . | . | . | P | . | . | . | . | D | . | . | . |
| 21-225_26A2_LC | W | . | . | . | Q | . | . | . | . | . | . | . | . |
| 21-225_29H6_LC | . | . | . | . | . | . | . | . | . | . | . | . | . |
| 21-225_25E6_LC | . | . | . | . | . | . | . | . | . | . | . | . | . |
| 21-225_25H10_LC | . | . | . | . | . | . | . | . | . | . | . | . | . |
| 21-225_29D7_LC | F | . | . | . | P | . | . | . | . | D | . | . | . |
| 21-225_29B8_LC | . | . | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | K_FR4 | | | | | | | | | | |
| CONSENSUS | L | T | F | G | G | G | T | K | V | E | I | K | R |
| 21-225_25H11_LC | . | . | . | . | . | . | . | . | . | . | . | . | . |
| 21-225_27F2_LC | . | . | . | . | . | . | . | . | . | . | . | . | . |
| 21-225_27C3_LC | . | . | . | . | . | . | . | . | . | . | . | . | . |
| 21-225_29G8_LC | . | . | . | . | . | . | . | . | . | . | . | . | . |
| 21-225_6A11_LC | W | . | . | . | Q | . | . | . | . | . | . | . | . |
| 21-225_6G12_LC | . | . | . | . | . | . | . | . | . | . | . | . | . |
| 21-225_8H7_LC | F | . | . | . | . | . | . | . | . | . | . | . | . |
| 21-225_10G5_LC | . | . | . | . | . | . | . | . | . | . | . | . | . |
| 21-225_10H12_LC | . | . | . | . | . | . | . | . | . | . | . | . | . |
| 21-225_12A1_LC | . | . | . | . | . | . | . | . | . | . | . | . | . |
| 21-225_6G2_LC | W | . | . | . | Q | . | . | . | . | . | . | T | . |
| 21-225_7H11_LC | . | . | . | . | . | . | . | . | . | . | . | . | . |
| 21-225_14E3_LC | F | . | . | . | . | . | . | . | . | . | . | . | . |
| 21-225_9A1_LC | W | . | . | . | Q | . | . | . | . | . | . | . | . |
| 21-225_15E3_LC | . | . | . | . | . | . | . | E | . | . | . | Y | . |
| 21-225_16F4_LC | F | . | . | . | . | . | . | . | . | . | . | . | . |
| 21-225_16F6_LC | I | . | . | . | Q | . | . | R | L | . | . | . | . |
| 21-225_2B2_LC | . | . | . | . | . | . | . | . | . | . | . | . | . |
| 21-225_13E6_LC | . | . | . | . | . | . | . | . | . | A | . | . | . |
| 21-225_16A5_LC | W | . | . | . | Q | . | . | . | . | . | . | . | . |
| 21-225_7G2_LC | W | . | . | . | L | . | . | . | . | V | . | . | . |
| 21-225_7C4_LC | . | . | . | . | . | . | . | . | . | . | . | . | . |
| 21-225_7E9_LC | . | . | . | . | . | . | . | . | . | . | . | . | . |
| 21-225_8C9_LC | . | . | . | . | . | . | . | . | . | . | . | . | . |
| 21-225_12B12_LC | W | . | . | . | L | . | . | . | . | V | . | . | . |
| 21-225_16B7_LC | . | . | . | . | . | . | . | . | . | . | F | N | . |
| 21-225_9E8_LC | . | . | . | . | . | . | . | . | . | . | . | . | . |
| 21-225_12F2_LC | W | . | . | . | Q | . | . | . | . | . | . | . | . |
| 21-225_12E6_LC | W | . | . | . | Q | . | . | . | . | . | . | . | . |
| 21-225_16G7_LC | W | . | . | . | Q | . | . | . | . | . | . | . | . |
| 21-225_1C12_LC | . | . | . | . | . | . | . | . | . | A | . | . | . |

FIGURE 56

Table 47

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | K_FR1 | | | | | | | | | | | | | | | | |
| CONSENSUS | D | I | Q | M | T | Q | S | P | S | S | L | S | A | S | V | G | D |
| 21-225_8D12_LC | . | . | . | . | . | . | . | . | . | . | . | . | . | . | . | . | . |
| 21-225_20C2_LC | . | . | . | . | . | . | . | . | . | . | . | . | . | . | . | Q | . |
| 21-225_18C6_LC | . | . | . | . | . | . | . | . | . | . | . | . | . | . | . | . | . |
| 21-225_18A3_LC | . | . | . | . | . | . | . | . | . | . | . | . | . | . | . | . | . |
| 21-225_22D12_LC | . | . | . | . | . | . | . | . | . | . | . | . | . | . | . | . | . |
| 21-225_23D1_LC | . | . | . | . | . | . | . | . | . | . | . | . | . | . | . | . | . |
| 21-225_24E5_LC | . | . | . | . | . | . | . | . | . | . | . | . | . | . | . | . | . |
| 21-225_22C1_LC | . | . | . | . | . | . | . | . | . | . | . | . | . | . | . | . | . |
| 21-225_20G9_LC | . | . | . | . | . | . | . | . | . | . | . | . | . | . | . | . | . |
| 21-225_21G7_LC | . | . | . | . | . | . | . | . | . | . | . | . | . | . | . | . | . |
| 21-225_22G9_LC | . | . | . | . | . | . | . | . | . | . | . | . | . | . | . | . | . |
| 21-225_34C4_LC | . | . | . | . | . | . | . | . | . | . | . | . | . | . | . | . | . |
| 21-225_2F7_LC | . | . | . | . | . | . | . | . | . | . | . | . | . | . | . | . | . |
| 21-225_3G4_LC | . | . | . | . | . | . | . | . | . | . | . | . | . | . | . | . | . |
| 21-225_7F4_LC | . | . | . | . | . | . | . | . | . | . | . | . | . | . | . | . | . |
| 21-225_6D4_LC | . | . | . | . | . | . | . | . | . | . | . | . | . | . | . | . | . |
| 21-225_15A1_LC | . | . | . | . | . | . | . | . | . | . | . | . | . | . | . | . | . |
| 21-225_15G7_LC | . | . | . | . | . | . | . | . | . | . | . | . | . | . | . | . | . |
| 21-225_11F10_LC | . | . | . | . | . | . | . | . | . | . | . | . | . | . | . | . | . |
| 21-225_15H1_LC | . | . | . | . | . | . | . | . | . | . | . | . | . | . | . | . | . |
| 21-225_16A1_LC | . | . | . | . | . | . | . | . | . | . | . | . | . | . | . | . | . |

FIGURE 56

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | | | | | | | | | | K_CDR1 | |
| CONSENSUS | R | V | T | I | T | C | R | A | S | - | - | Q | G | I | R | - | - |
| 21-225_8D12_LC | . | . | . | . | . | . | . | . | . | . | . | . | . | . | . | . | . |
| 21-225_20C2_LC | . | . | . | . | . | . | . | . | . | . | . | . | . | . | . | . | . |
| 21-225_18C6_LC | . | . | . | . | . | . | . | T | . | . | . | . | . | . | . | . | . |
| 21-225_18A3_LC | . | . | . | . | . | . | . | . | . | . | . | . | . | . | . | . | . |
| 21-225_22D12_LC | . | . | . | . | . | . | . | . | . | . | . | . | . | . | . | . | . |
| 21-225_23D1_LC | . | . | . | . | . | . | . | . | . | . | . | . | . | . | . | . | . |
| 21-225_24E5_LC | . | . | . | . | . | . | . | . | . | . | . | . | . | . | . | . | . |
| 21-225_22C1_LC | . | . | . | . | . | . | . | T | . | . | . | . | . | . | . | . | . |
| 21-225_20G9_LC | . | . | . | . | . | . | . | . | . | . | . | . | . | . | . | . | . |
| 21-225_21G7_LC | . | . | . | . | . | . | . | . | . | . | . | . | . | V | . | . | . |
| 21-225_22G9_LC | . | . | . | . | . | . | . | . | . | . | . | . | . | . | . | . | . |
| 21-225_34C4_LC | . | . | . | . | . | . | . | . | . | . | . | . | . | . | . | . | . |
| 21-225_2F7_LC | . | . | . | . | . | . | . | . | . | . | . | . | . | . | . | . | . |
| 21-225_3G4_LC | . | . | . | . | . | . | . | . | . | . | . | . | . | . | . | . | . |
| 21-225_7F4_LC | . | . | . | . | . | . | . | T | . | . | . | . | . | . | . | . | . |
| 21-225_6D4_LC | . | . | . | . | . | . | . | . | . | . | . | . | A | . | . | . | . |
| 21-225_15A1_LC | . | . | . | . | . | . | . | . | . | . | . | . | . | . | . | . | . |
| 21-225_15G7_LC | . | . | . | . | . | . | . | . | . | . | . | . | . | . | . | . | . |
| 21-225_11F10_LC | . | . | . | . | . | . | . | . | . | . | . | . | . | . | . | . | . |
| 21-225_15H1_LC | . | . | . | . | . | . | . | . | . | . | . | . | . | . | . | . | . |
| 21-225_16A1_LC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | | K_FR2 | |
| CONSENSUS | - | - | - | - | N | D | L | G | W | Y | Q | Q | K | P | G | K | A |
| 21-225_8D12_LC | . | . | . | . | . | . | . | . | . | . | . | . | . | . | . | . | . |
| 21-225_20C2_LC | . | . | . | . | . | . | . | . | . | . | . | . | . | . | . | . | . |
| 21-225_18C6_LC | . | . | . | . | . | . | . | . | . | . | . | . | . | . | . | . | . |
| 21-225_18A3_LC | . | . | . | . | . | . | . | . | . | . | . | . | . | . | . | . | . |
| 21-225_22D12_LC | . | . | . | . | . | . | . | . | . | . | . | . | . | . | . | . | . |
| 21-225_23D1_LC | . | . | . | . | . | . | . | . | . | . | . | . | . | . | . | . | . |
| 21-225_24E5_LC | . | . | . | . | . | . | . | . | . | . | . | . | . | . | . | . | . |
| 21-225_22C1_LC | . | . | . | . | . | . | . | . | . | . | . | . | . | . | . | . | . |
| 21-225_20G9_LC | . | . | . | . | . | . | . | . | . | . | . | . | . | . | . | . | . |
| 21-225_21G7_LC | . | . | . | . | . | . | . | . | . | . | . | . | . | . | . | . | . |
| 21-225_22G9_LC | . | . | . | . | . | . | . | . | . | . | . | . | . | . | . | . | . |
| 21-225_34C4_LC | . | . | . | . | . | . | . | . | . | . | . | . | . | . | . | . | . |
| 21-225_2F7_LC | . | . | . | . | . | . | . | . | . | . | . | . | . | . | . | . | . |
| 21-225_3G4_LC | . | . | . | . | . | . | . | . | . | . | . | . | . | . | . | . | . |
| 21-225_7F4_LC | . | . | . | . | . | . | . | . | . | . | . | . | . | . | . | . | . |
| 21-225_6D4_LC | . | . | . | . | . | . | . | . | . | . | . | . | . | . | . | . | . |
| 21-225_15A1_LC | . | . | . | . | . | . | . | . | . | . | . | . | . | . | . | . | . |
| 21-225_15G7_LC | . | . | . | . | . | . | . | . | . | . | . | . | . | . | . | . | . |
| 21-225_11F10_LC | . | . | . | . | . | . | . | . | . | . | . | . | . | . | . | . | . |
| 21-225_15H1_LC | . | . | . | . | . | . | . | . | . | . | . | . | . | . | . | . | . |
| 21-225_16A1_LC | . | . | . | . | . | . | . | . | . | . | . | . | . | . | . | . | . |

FIGURE 56

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | K_CDR2 | | | | | | | | | | |
| CONSENSUS | P | K | R | L | I | Y | A | - | - | - | - | - | - | - | - | A | S |
| 21-225_8D12_LC | . | . | . | . | . | . | . | . | . | . | . | . | . | . | . | . | . |
| 21-225_20C2_LC | . | . | . | . | . | F | . | . | . | . | . | . | . | . | . | . | . |
| 21-225_18C6_LC | . | . | . | . | . | . | . | . | . | . | . | . | . | . | . | . | . |
| 21-225_18A3_LC | . | . | . | . | . | . | V | . | . | . | . | . | . | . | . | . | . |
| 21-225_22D12_LC | . | . | . | . | . | . | . | . | . | . | . | . | . | . | . | . | . |
| 21-225_23D1_LC | . | . | . | . | . | . | . | . | . | . | . | . | . | . | . | . | . |
| 21-225_24E5_LC | . | . | . | . | . | . | . | . | . | . | . | . | . | . | . | . | . |
| 21-225_22C1_LC | . | . | . | . | . | . | . | . | . | . | . | . | . | . | . | . | . |
| 21-225_20G9_LC | . | . | . | . | . | . | . | . | . | . | . | . | . | . | . | . | . |
| 21-225_21G7_LC | . | Q | . | . | . | . | . | . | . | . | . | . | . | . | . | . | . |
| 21-225_22G9_LC | . | . | . | . | . | . | . | . | . | . | . | . | . | . | . | . | . |
| 21-225_34C4_LC | . | . | . | . | . | . | T | . | . | . | . | . | . | . | . | . | . |
| 21-225_2F7_LC | . | . | . | . | . | . | . | . | . | . | . | . | . | . | . | . | . |
| 21-225_3G4_LC | . | . | . | . | . | . | . | . | . | . | . | . | . | . | . | . | . |
| 21-225_7F4_LC | . | . | . | . | . | . | . | . | . | . | . | . | . | . | . | . | . |
| 21-225_6D4_LC | . | . | . | . | . | . | . | . | . | . | . | . | . | . | . | . | . |
| 21-225_15A1_LC | . | . | . | . | . | . | . | . | . | . | . | . | . | . | . | . | . |
| 21-225_15G7_LC | . | . | . | . | . | . | . | . | . | . | . | . | . | . | . | . | . |
| 21-225_11F10_LC | . | . | . | . | . | . | . | . | . | . | . | . | . | . | . | . | . |
| 21-225_15H1_LC | . | . | . | . | . | . | T | . | . | . | . | . | . | . | . | . | . |
| 21-225_16A1_LC | . | . | . | . | . | . | . | . | . | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | L | Q | S | G | V | P | S | R | F | S | G | S | G | S | G | - |
| 21-225_8D12_LC | . | . | . | . | . | . | . | . | . | . | N | . | . | . | . | . | . |
| 21-225_20C2_LC | . | . | . | . | . | . | . | . | . | . | . | . | . | . | . | . | . |
| 21-225_18C6_LC | . | . | . | . | . | . | . | . | . | . | . | . | . | . | . | . | . |
| 21-225_18A3_LC | . | . | . | . | . | . | . | . | . | . | N | . | . | . | . | . | . |
| 21-225_22D12_LC | . | . | . | . | . | . | . | . | . | . | . | . | . | . | . | . | . |
| 21-225_23D1_LC | . | . | . | . | . | . | . | . | . | . | . | . | . | . | . | . | . |
| 21-225_24E5_LC | . | . | . | . | . | . | . | . | . | . | . | . | . | . | . | . | . |
| 21-225_22C1_LC | . | . | . | . | . | . | . | . | . | . | . | . | . | . | . | . | . |
| 21-225_20G9_LC | . | . | . | . | . | . | . | . | . | . | . | . | . | . | . | . | . |
| 21-225_21G7_LC | . | . | . | . | . | . | . | . | . | . | N | . | . | . | . | . | . |
| 21-225_22G9_LC | . | . | . | . | . | . | . | . | . | . | . | . | . | . | . | . | . |
| 21-225_34C4_LC | N | . | . | . | . | . | . | . | . | . | . | . | . | . | . | . | . |
| 21-225_2F7_LC | . | . | . | . | . | . | . | . | . | . | . | . | . | . | . | . | . |
| 21-225_3G4_LC | . | . | . | . | . | . | . | . | . | . | . | . | . | . | . | . | . |
| 21-225_7F4_LC | N | . | . | . | . | . | . | . | . | . | . | . | . | . | . | . | . |
| 21-225_6D4_LC | . | . | . | . | . | . | . | . | . | . | . | . | . | . | . | . | . |
| 21-225_15A1_LC | . | . | . | . | . | . | . | . | . | . | . | . | . | . | . | . | . |
| 21-225_15G7_LC | . | . | . | . | . | I | . | . | . | . | . | . | . | . | [illegible] | . | . |
| 21-225_11F10_LC | . | . | . | . | . | . | . | . | . | . | . | . | . | . | . | . | . |
| 21-225_15H1_LC | . | . | . | . | . | . | . | . | . | . | N | . | . | . | . | . | . |
| 21-225_16A1_LC | . | . | . | . | . | . | . | . | . | . | . | . | . | . | . | . | . |

**FIGURE 56**

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | K_FR3 | | | | | | | | | | | | | | | | |
| CONSENSUS | - | T | E | F | T | L | T | I | S | S | L | Q | P | E | D | F | A |
| 21-225_8D12_LC | . | . | . | . | . | . | . | . | . | . | . | . | . | . | . | . | . |
| 21-225_20C2_LC | . | . | . | . | . | . | . | . | . | . | . | . | . | . | . | . | . |
| 21-225_18C6_LC | . | . | . | . | . | . | . | . | . | . | . | . | . | . | . | . | . |
| 21-225_18A3_LC | . | . | . | . | . | . | . | . | . | . | . | . | . | . | . | . | . |
| 21-225_22D12_LC | . | . | D | . | . | . | . | . | . | . | . | . | . | . | . | . | . |
| 21-225_23D1_LC | . | . | . | . | . | . | . | V | . | . | . | . | . | . | . | . | . |
| 21-225_24E5_LC | . | . | . | . | . | . | . | . | . | . | . | . | . | . | . | . | . |
| 21-225_22C1_LC | . | . | . | . | . | . | . | . | . | . | . | . | . | . | . | . | . |
| 21-225_20G9_LC | . | . | . | . | . | . | . | . | . | . | . | . | . | . | . | . | . |
| 21-225_21G7_LC | . | . | . | . | . | . | . | . | . | . | . | . | . | . | . | . | . |
| 21-225_22G9_LC | . | . | . | . | . | . | . | . | . | . | . | . | . | . | . | . | . |
| 21-225_34C4_LC | . | . | . | . | . | . | . | . | . | . | . | . | . | . | . | . | . |
| 21-225_2F7_LC | . | . | . | . | . | . | . | . | . | . | . | . | . | . | . | . | . |
| 21-225_3G4_LC | . | . | . | . | . | . | . | . | . | . | . | . | . | . | . | . | . |
| 21-225_7F4_LC | . | . | . | . | . | . | . | . | . | . | . | | . | . | . | . | . |
| 21-225_6D4_LC | . | . | . | . | . | . | . | . | . | . | . | | . | . | . | . | . |
| 21-225_15A1_LC | . | . | . | . | . | . | . | V | . | . | . | . | . | . | . | . | . |
| 21-225_15G7_LC | . | . | . | . | . | . | . | . | . | . | . | . | . | . | . | . | . |
| 21-225_11F10_LC | . | . | . | . | . | . | . | V | . | . | . | . | . | . | . | . | . |
| 21-225_15H1_LC | . | . | . | . | . | . | . | . | . | . | . | . | . | . | . | . | . |
| 21-225_16A1_LC | . | . | . | . | . | . | . | . | . | . | . | . | . | . | . | . | . |

**FIGURE 56**

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | T | Y | Y | C | L | Q | H | S | S | - | - | - | - | - | - | - | - |
| 21-225_8D12_LC | . | . | . | . | . | . | . | . | . | . | . | . | . | . | . | . | . |
| 21-225_20C2_LC | . | . | . | . | . | . | . | A | . | . | . | . | . | . | . | . | . |
| 21-225_18C6_LC | . | . | . | . | . | . | . | . | . | . | . | . | . | . | . | . | . |
| 21-225_18A3_LC | . | . | . | . | . | . | . | . | . | . | . | . | . | . | . | . | . |
| 21-225_22D12_LC | . | . | . | . | . | . | . | . | T | . | . | . | . | . | . | . | . |
| 21-225_23D1_LC | . | . | . | . | . | . | . | . | . | . | . | . | . | . | . | . | . |
| 21-225_24E5_LC | . | . | . | . | . | . | . | . | N | . | . | . | . | . | . | . | . |
| 21-225_22C1_LC | . | . | . | . | . | . | . | . | T | . | . | . | . | . | . | . | . |
| 21-225_20G9_LC | . | . | . | . | . | . | . | . | T | . | . | . | . | . | . | . | . |
| 21-225_21G7_LC | . | . | . | . | . | . | . | . | . | . | . | . | . | . | . | . | . |
| 21-225_22G9_LC | . | . | . | . | . | . | . | A | . | . | . | . | . | . | . | . | . |
| 21-225_34C4_LC | . | . | . | . | . | . | . | . | . | . | . | . | . | . | . | . | . |
| 21-225_2F7_LC | . | . | . | . | . | . | . | A | . | . | . | . | . | . | . | . | . |
| 21-225_3G4_LC | . | . | . | . | . | . | . | . | . | . | . | . | . | . | . | . | . |
| 21-225_7F4_LC | . | . | . | . | . | . | . | . | A | . | . | . | . | . | . | . | . |
| 21-225_6D4_LC | . | . | . | . | . | . | . | . | . | . | . | . | . | . | . | . | . |
| 21-225_15A1_LC | S | . | . | . | . | H | N | . | . | . | . | . | . | . | . | . | . |
| 21-225_15G7_LC | . | . | . | . | . | . | . | . | N | . | . | . | . | . | . | . | . |
| 21-225_11F10_LC | . | . | . | . | . | . | . | . | . | . | . | . | . | . | . | . | . |
| 21-225_15H1_LC | . | . | . | . | . | . | . | . | . | . | . | . | . | . | . | . | . |
| 21-225_16A1_LC | . | . | . | . | . | H | . | . | N | . | . | . | . | . | . | . | . |

FIGURE 56

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_8D12_LC | . | . | . | . | . | . | . | . | . | . | . | . | . | . | . | . | . |
| 21-225_20C2_LC | . | . | . | . | . | . | . | . | . | . | . | . | . | . | . | . | . |
| 21-225_18C6_LC | . | . | . | . | . | . | . | . | . | . | . | . | . | . | . | . | . |
| 21-225_18A3_LC | . | . | . | . | . | . | . | . | . | . | . | . | . | . | . | . | . |
| 21-225_22D12_LC | . | . | . | . | . | . | . | . | . | . | . | . | . | . | . | . | . |
| 21-225_23D1_LC | . | . | . | . | . | . | . | . | . | . | . | . | . | . | . | . | . |
| 21-225_24E5_LC | . | . | . | . | . | . | . | . | . | . | . | . | . | . | . | . | . |
| 21-225_22C1_LC | . | . | . | . | . | . | . | . | . | . | . | . | . | . | . | . | . |
| 21-225_20G9_LC | . | . | . | . | . | . | . | . | . | . | . | . | . | . | . | . | . |
| 21-225_21G7_LC | . | . | . | . | . | . | . | . | . | . | . | . | . | . | . | . | . |
| 21-225_22G9_LC | . | . | . | . | . | . | . | . | . | . | . | . | . | . | . | . | . |
| 21-225_34C4_LC | . | . | . | . | . | . | . | . | . | . | . | . | . | . | . | . | . |
| 21-225_2F7_LC | . | . | . | . | . | . | . | . | . | . | . | . | . | . | . | . | . |
| 21-225_3G4_LC | . | . | . | . | . | . | . | . | . | . | . | . | . | . | . | . | . |
| 21-225_7F4_LC | . | . | . | . | . | . | . | . | . | . | . | . | . | . | . | . | . |
| 21-225_6D4_LC | . | . | . | . | . | . | . | . | . | . | . | . | . | . | . | . | . |
| 21-225_15A1_LC | . | . | . | . | . | . | . | . | . | . | . | . | . | . | . | . | . |
| 21-225_15G7_LC | . | . | . | . | . | . | . | . | . | . | . | . | . | . | . | . | . |
| 21-225_11F10_LC | . | . | . | . | . | . | . | . | . | . | . | . | . | . | . | . | . |
| 21-225_15H1_LC | . | . | . | . | . | . | . | . | . | . | . | . | . | . | . | . | . |
| 21-225_16A1_LC | . | . | . | . | . | . | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | K_FR4 | | | | | | | | | | |
| CONSENSUS | L | T | F | G | G | G | T | K | V | E | I | K | R |
| 21-225_8D12_LC | . | . | . | . | . | . | . | . | . | . | . | . | . |
| 21-225_20C2_LC | . | . | . | . | . | . | . | . | . | . | . | E | . |
| 21-225_18C6_LC | . | . | . | . | . | . | . | . | . | . | . | . | . |
| 21-225_18A3_LC | . | . | . | . | . | . | . | . | . | . | . | . | . |
| 21-225_22D12_LC | . | . | . | . | . | . | . | . | . | . | . | . | . |
| 21-225_23D1_LC | . | . | . | . | . | . | . | . | . | . | . | . | . |
| 21-225_24E5_LC | . | . | . | . | . | . | . | . | . | . | . | . | . |
| 21-225_22C1_LC | . | . | . | . | . | . | . | . | . | . | . | . | . |
| 21-225_20G9_LC | . | . | . | . | . | . | . | . | . | . | . | . | . |
| 21-225_21G7_LC | . | . | . | . | . | . | . | . | . | . | . | . | . |
| 21-225_22G9_LC | . | . | . | . | . | . | . | . | . | . | . | . | . |
| 21-225_34C4_LC | I | . | . | . | Q | . | . | [illegible] | L | . | . | . | . |
| 21-225_2F7_LC | . | . | . | . | . | . | . | . | . | . | . | . | . |
| 21-225_3G4_LC | . | . | . | . | . | . | . | . | . | . | . | . | . |
| 21-225_7F4_LC | . | . | . | . | . | . | . | . | . | . | . | . | . |
| 21-225_6D4_LC | . | . | . | . | . | . | . | . | . | . | . | . | . |
| 21-225_15A1_LC | V | K | . | . | . | . | I | . | . | . | . | T | G |
| 21-225_15G7_LC | . | . | . | . | . | . | . | . | . | . | . | . | . |
| 21-225_11F10_LC | . | . | . | . | . | . | . | . | . | . | . | . | . |
| 21-225_15H1_LC | . | . | . | . | . | . | . | . | . | . | . | . | . |
| 21-225_16A1_LC | . | . | . | . | . | . | . | . | . | . | . | E | . |

FIGURE 56

Table 48

| Reference # | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| | K_FR1 | | | | | | | | | | | | | | | | |
| CONSENSUS | D | I | Q | M | T | Q | S | P | S | S | L | S | A | S | V | G | D |
| 21-225_11F5_LC | . | . | . | . | . | . | . | . | . | . | . | . | . | . | . | . | . |
| 21-225_17H8_LC | . | . | . | . | . | . | . | . | . | . | . | . | . | . | . | . | . |
| 21-225_17F9_LC | . | . | . | . | . | . | . | . | . | . | . | . | . | . | . | . | . |
| 21-225_1F2_LC | . | . | . | . | . | . | . | . | . | . | . | . | . | . | . | . | . |
| 21-225_21H3_LC | . | . | . | . | . | . | . | . | . | . | . | . | . | . | . | . | . |
| 21-225_23C8_LC | . | . | . | . | . | . | . | . | . | . | R | . | . | . | . | . | . |
| 21-225_22G8_LC | . | . | . | . | I | . | . | . | . | . | . | F | . | . | . | . | . |
| 21-225_22H4_LC | . | . | . | . | . | . | . | . | . | . | . | . | . | . | . | . | . |
| 21-225_23A3_LC | . | . | . | . | . | . | . | . | . | . | . | . | . | . | . | . | . |
| 21-225_5H7_LC | . | . | . | . | . | . | . | . | . | . | . | . | . | . | . | . | . |
| 21-225_14B2_LC | . | . | . | . | . | . | . | . | . | . | . | . | . | . | . | . | . |
| 21-225_4F4_LC | . | . | . | . | . | . | . | . | . | . | . | . | . | . | . | . | . |
| 21-225_4H4_LC | . | . | . | . | . | . | . | . | . | . | . | . | . | . | . | . | . |

| Reference # | 18 | 19 | 20 | 21 | 22 | 23 | 24 | 25 | 26 | 27 | 28 | 29 | 30 | 31 | 32 | 33 | 34 |
|---|---|---|---|---|---|---|---|---|---|---|---|---|---|---|---|---|---|
| | | | | | | | K_CDR1 | | | | | | | | | | |
| CONSENSUS | R | V | T | I | T | C | R | A | S | - | - | Q | G | I | R | - | - |
| 21-225_11F5_LC | . | . | . | . | . | . | . | . | . | . | . | . | . | . | . | . | . |
| 21-225_17H8_LC | . | . | . | . | . | . | . | . | . | . | . | . | . | . | . | . | . |
| 21-225_17F9_LC | . | . | . | . | . | . | . | . | . | . | . | . | V | . | . | . | . |
| 21-225_1F2_LC | . | . | . | . | . | . | . | . | . | . | . | . | . | . | . | . | . |
| 21-225_21H3_LC | . | . | . | . | . | . | . | . | . | . | . | . | . | . | . | . | . |
| 21-225_23C8_LC | . | . | . | . | . | . | . | . | . | . | . | . | D | . | . | . | . |
| 21-225_22G8_LC | . | . | . | . | . | . | . | T | . | . | . | . | . | . | . | . | . |
| 21-225_22H4_LC | . | . | . | . | . | . | . | . | . | . | . | . | . | . | . | . | . |
| 21-225_23A3_LC | . | . | . | . | . | . | . | . | . | . | . | . | . | . | . | . | . |
| 21-225_5H7_LC | . | . | . | . | . | . | . | . | . | . | . | . | . | . | . | . | . |
| 21-225_14B2_LC | . | . | . | . | . | . | . | . | . | . | . | . | . | . | . | . | . |
| 21-225_4F4_LC | . | . | . | . | . | . | . | . | . | . | . | . | . | . | . | . | . |
| 21-225_4H4_LC | . | . | . | . | . | . | . | . | . | . | . | . | . | . | . | . | . |

FIGURE 56

| Reference # | 35 | 36 | 37 | 38 | 39 | 40 | 41 | 42 | 43 | 44 | 45 | 46 | 47 | 48 | 49 | 50 | 51 |
|---|---|---|---|---|---|---|---|---|---|---|---|---|---|---|---|---|---|
| | | | | | | | | | | | | | | | | K_FR2 | |
| CONSENSUS | - | - | - | - | N | D | L | G | W | Y | Q | Q | K | P | G | K | A |
| 21-225_11F5_LC | . | . | . | . | . | . | . | . | . | . | . | . | . | . | . | . | . |
| 21-225_17H8_LC | . | . | . | . | . | . | . | . | . | . | . | . | . | . | . | . | . |
| 21-225_17F9_LC | . | . | . | . | . | . | . | . | . | . | . | . | . | . | . | . | . |
| 21-225_1F2_LC | . | . | . | . | . | . | . | . | . | . | . | . | . | . | . | . | . |
| 21-225_21H3_LC | . | . | . | . | . | . | . | . | . | . | . | . | . | . | . | . | . |
| 21-225_23C8_LC | . | . | . | . | . | . | . | . | . | . | . | . | . | . | . | R | . |
| 21-225_22G8_LC | . | . | . | . | . | . | . | . | . | . | . | . | . | . | . | . | . |
| 21-225_22H4_LC | . | . | . | . | . | . | . | . | . | . | . | . | . | . | . | . | . |
| 21-225_23A3_LC | . | . | . | . | . | . | . | . | . | . | . | . | . | . | . | . | . |
| 21-225_5H7_LC | . | . | . | . | . | . | . | . | . | . | . | . | . | . | . | . | . |
| 21-225_14B2_LC | . | . | . | . | . | . | . | . | . | . | . | . | . | . | . | . | . |
| 21-225_4F4_LC | . | . | . | . | . | . | . | . | . | . | . | . | . | . | . | . | . |
| 21-225_4H4_LC | . | . | . | . | . | . | . | . | . | . | . | . | . | . | . | . | . |

| Reference # | 52 | 53 | 54 | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|---|---|
| | | | | | | | | | | | | | | K_CDR2 | | | |
| CONSENSUS | P | K | R | L | I | Y | A | - | - | - | - | - | - | - | - | A | S |
| 21-225_11F5_LC | . | . | . | . | . | . | . | . | . | . | . | . | . | . | . | . | . |
| 21-225_17H8_LC | . | . | . | . | . | . | G | . | . | . | . | . | . | . | . | . | . |
| 21-225_17F9_LC | . | . | . | . | . | . | . | . | . | . | . | . | . | . | . | V | . |
| 21-225_1F2_LC | . | . | . | . | . | . | . | . | . | . | . | . | . | . | . | . | . |
| 21-225_21H3_LC | . | . | . | . | . | . | . | . | . | . | . | . | . | . | . | . | . |
| 21-225_23C8_LC | . | . | . | . | . | N | G | . | . | . | . | . | . | . | . | . | . |
| 21-225_22G8_LC | . | . | . | . | . | . | . | . | . | . | . | . | . | . | . | . | . |
| 21-225_22H4_LC | . | . | . | . | . | . | . | . | . | . | . | . | . | . | . | . | . |
| 21-225_23A3_LC | . | . | . | . | . | . | . | . | . | . | . | . | . | . | . | . | . |
| 21-225_5H7_LC | . | . | . | . | . | . | V | . | . | . | . | . | . | . | . | T | . |
| 21-225_14B2_LC | . | . | . | . | . | . | V | . | . | . | . | . | . | . | . | T | . |
| 21-225_4F4_LC | . | . | . | . | . | . | . | . | . | . | . | . | . | . | . | . | . |
| 21-225_4H4_LC | . | . | . | . | . | . | . | . | . | . | . | . | . | . | . | . | . |

FIGURE 56

| Reference # | 69 | 70 | 71 | 72 | 73 | 74 | 75 | 76 | 77 | 78 | 79 | 80 | 81 | 82 | 83 | 84 | 85 |
|---|---|---|---|---|---|---|---|---|---|---|---|---|---|---|---|---|---|
| CONSENSUS | S | V | Q | S | G | V | P | S | R | F | S | G | S | G | S | G | - |
| 21-225_11F5_LC | . | . | . | . | . | . | . | . | . | . | . | . | . | . | . | . | . |
| 21-225_17H8_LC | . | L | . | . | . | . | . | . | . | . | . | . | . | . | . | . | . |
| 21-225_17F9_LC | . | L | . | . | . | . | . | . | . | . | . | . | . | . | . | . | . |
| 21-225_1F2_LC | . | . | . | . | . | . | . | . | . | . | . | . | . | . | . | . | . |
| 21-225_21H3_LC | . | . | . | T | . | . | . | . | . | . | . | . | . | . | . | . | . |
| 21-225_23C8_LC | . | . | . | . | . | . | . | . | . | . | . | . | . | . | . | . | . |
| 21-225_22G8_LC | . | L | . | . | . | . | . | . | K | . | . | . | . | . | . | . | . |
| 21-225_22H4_LC | . | . | . | . | . | . | . | . | . | . | . | . | . | . | . | . | . |
| 21-225_23A3_LC | . | . | . | . | . | . | . | . | . | . | . | . | . | . | . | . | . |
| 21-225_5H7_LC | . | L | . | G | . | . | . | . | . | . | . | . | . | . | . | . | . |
| 21-225_14B2_LC | . | L | . | G | . | . | . | . | . | . | . | . | . | . | . | . | . |
| 21-225_4F4_LC | . | . | . | T | . | . | . | . | . | . | . | . | . | . | . | . | . |
| 21-225_4H4_LC | . | . | . | . | . | . | . | . | . | . | . | . | . | . | . | . | . |

| Reference # | 86 | 87 | 88 | 89 | 90 | 91 | 92 | 93 | 94 | 95 | 96 | 97 | 98 | 99 | 100 | 101 | 102 |
|---|---|---|---|---|---|---|---|---|---|---|---|---|---|---|---|---|---|
| | K_FR3 | | | | | | | | | | | | | | | | |
| CONSENSUS | - | T | E | F | T | L | T | I | S | S | L | Q | P | E | D | F | A |
| 21-225_11F5_LC | . | . | . | . | . | . | . | . | . | . | . | . | . | . | . | . | . |
| 21-225_17H8_LC | . | . | . | . | . | . | . | . | . | . | . | . | . | . | . | . | T |
| 21-225_17F9_LC | . | . | . | . | . | . | . | . | N | . | . | . | . | . | . | . | . |
| 21-225_1F2_LC | . | . | . | . | . | . | . | . | . | . | . | . | . | . | . | . | . |
| 21-225_21H3_LC | . | . | . | . | . | . | . | . | . | . | . | . | A | . | . | L | . |
| 21-225_23C8_LC | . | . | . | . | . | . | . | . | . | . | . | . | . | . | . | . | V |
| 21-225_22G8_LC | . | . | . | . | . | . | S | . | . | . | . | . | . | . | . | . | . |
| 21-225_22H4_LC | . | . | . | . | . | . | . | . | . | . | . | . | . | . | . | . | . |
| 21-225_23A3_LC | . | . | . | . | . | . | . | . | . | . | . | . | . | . | . | . | . |
| 21-225_5H7_LC | . | . | . | . | . | . | . | . | . | . | . | . | . | . | . | . | . |
| 21-225_14B2_LC | . | . | . | . | . | . | . | . | . | . | . | . | . | . | . | . | . |
| 21-225_4F4_LC | . | . | . | . | . | . | . | . | . | . | . | . | . | . | . | . | . |
| 21-225_4H4_LC | . | . | . | . | . | . | . | . | . | . | . | . | . | . | . | . | . |

FIGURE 56

| Reference # | 103 | 104 | 105 | 106 | 107 | 108 | 109 | 110 | 111 | 112 | 113 | 114 | 115 | 116 | 117 | 118 | 119 |
|---|---|---|---|---|---|---|---|---|---|---|---|---|---|---|---|---|---|
| CONSENSUS | I | Y | Y | C | L | Q | H | N | S | - | - | - | - | - | - | - | - |
| 21-225_11F5_LC | . | . | . | . | . | . | . | . | . | . | . | . | . | . | . | . | . |
| 21-225_17H8_LC | T | . | . | . | . | . | . | . | . | . | . | . | . | . | . | . | . |
| 21-225_17F9_LC | T | . | . | . | . | . | . | . | T | . | . | . | . | . | . | . | . |
| 21-225_1F2_LC | . | . | . | . | . | . | . | . | . | . | . | . | . | . | . | . | . |
| 21-225_21H3_LC | . | . | . | . | . | . | . | . | . | . | . | . | . | . | . | . | . |
| 21-225_23C8_LC | . | . | . | . | . | . | . | . | . | . | . | . | . | . | . | . | . |
| 21-225_22G8_LC | T | . | . | . | . | . | . | . | . | . | . | . | . | . | . | . | . |
| 21-225_22H4_LC | L | . | . | . | . | . | . | . | . | . | . | . | . | . | . | . | . |
| 21-225_23A3_LC | . | . | F | . | . | . | . | . | . | . | . | . | . | . | . | . | . |
| 21-225_5H7_LC | T | . | . | . | . | . | . | . | . | . | . | . | . | . | . | . | . |
| 21-225_14B2_LC | T | . | . | . | . | . | . | . | . | . | . | . | . | . | . | . | . |
| 21-225_4F4_LC | . | . | . | . | . | . | . | . | . | . | . | . | . | . | . | . | . |
| 21-225_4H4_LC | . | . | . | . | . | . | . | . | . | . | . | . | . | . | . | . | . |

| Reference # | 120 | 121 | 122 | 123 | 124 | 125 | 126 | 127 | 128 | 129 | 130 | 131 | 132 | 133 | 134 | 135 | 136 |
|---|---|---|---|---|---|---|---|---|---|---|---|---|---|---|---|---|---|
| | | | K_CDR3 | | | | | | | | | | | | | | |
| CONSENSUS | - | - | - | - | - | - | - | - | - | - | - | - | - | - | - | Y | P |
| 21-225_11F5_LC | . | . | . | . | . | . | . | . | . | . | . | . | . | . | . | . | . |
| 21-225_17H8_LC | . | . | . | . | . | . | . | . | . | . | . | . | . | . | . | . | . |
| 21-225_17F9_LC | . | . | . | . | . | . | . | . | . | . | . | . | . | . | . | . | . |
| 21-225_1F2_LC | . | . | . | . | . | . | . | . | . | . | . | . | . | . | . | . | . |
| 21-225_21H3_LC | . | . | . | . | . | . | . | . | . | . | . | . | . | . | . | . | . |
| 21-225_23C8_LC | . | . | . | . | . | . | . | . | . | . | . | . | . | . | . | . | . |
| 21-225_22G8_LC | . | . | . | . | . | . | . | . | . | . | . | . | . | . | . | . | . |
| 21-225_22H4_LC | . | . | . | . | . | . | . | . | . | . | . | . | . | . | . | . | . |
| 21-225_23A3_LC | . | . | . | . | . | . | . | . | . | . | . | . | . | . | . | . | . |
| 21-225_5H7_LC | . | . | . | . | . | . | . | . | . | . | . | . | . | . | . | . | . |
| 21-225_14B2_LC | . | . | . | . | . | . | . | . | . | . | . | . | . | . | . | . | . |
| 21-225_4F4_LC | . | . | . | . | . | . | . | . | . | . | . | . | . | . | . | . | . |
| 21-225_4H4_LC | . | . | . | . | . | . | . | . | . | . | . | . | . | . | . | . | . |

FIGURE 56

| Reference # | 137 | 138 | 139 | 140 | 141 | 142 | 143 | 144 | 145 | 146 | 147 | 148 | 149 |
|---|---|---|---|---|---|---|---|---|---|---|---|---|---|
| | | | K_FR4 | | | | | | | | | | |
| CONSENSUS | L | T | F | G | G | G | T | K | V | E | I | K | R |
| 21-225_11F5_LC | . | . | . | . | . | . | . | . | . | . | . | . | . |
| 21-225_17H8_LC | . | . | . | . | . | . | . | . | . | . | . | . | . |
| 21-225_17F9_LC | . | . | . | . | . | . | . | . | . | . | F | . | . |
| 21-225_1F2_LC | . | . | . | . | . | . | . | . | . | . | . | . | . |
| 21-225_21H3_LC | . | . | . | . | . | . | . | . | . | . | . | . | . |
| 21-225_23C8_LC | . | . | . | . | . | . | . | . | . | . | . | . | . |
| 21-225_22G8_LC | . | . | . | . | . | . | . | . | . | . | . | . | . |
| 21-225_22H4_LC | . | . | . | . | . | . | . | . | . | . | . | . | . |
| 21-225_23A3_LC | . | . | . | . | . | . | . | . | . | . | . | . | . |
| 21-225_5H7_LC | . | . | . | . | . | . | . | . | . | . | . | . | . |
| 21-225_14B2_LC | . | . | . | . | . | . | . | . | . | . | . | . | . |
| 21-225_4F4_LC | . | . | . | . | . | . | . | . | . | . | . | . | . |
| 21-225_4H4_LC | . | . | . | . | . | . | . | . | . | . | . | . | . |

**FIGURE 57**

Table 49: Consensus 15-VH1|1-08/D6|6-19|RF1/JH4 (SEQ ID NO: 50266):

QVQLVQS-GAEVKKPGASVKVSCKASG-YTFTN-----YDINWVRQATGQGLEWMGWMHPN---SGNTGYAQKFQGRVTMTRNTSISTAYMELSSLRSEDTAVYYCASSSGWY---------------------YFDYWGQGTLVTVSS wherein:

N at position 33 can be substituted with H

I at position 41 can be substituted with V or L

W at position 57 can be substituted with R

M at position 58 can be substituted with V or L

H at position 59 can substituted with N, Y or T

N at position 61 can be substituted with D or H

G at position 66 can be substituted with H

N at position 67 can be substituted with S, Q, A, D, K or T

T at position 68 can be substituted with A, V or E

G at position 69 can be substituted with D

Y at position 70 can be substituted with F or C

A at position 71 can be substituted with P

Q at position 72 can be substituted with K

K at position 73 can be substituted with R or N

Q at position 75 can be substituted with R

G at position 76 can be substituted with V

Y at position 113 can be substituted with E, N or T

**FIGURE 57**

Y at position 135 can be substituted with F, K, I, R, V, L, M, W, H or S

Y at position 138 can be substituted with F, S or N

Xaa Tyr Asp Xaa Asn (SEQ ID NO: 50001)

Xaa Xaa Xaa Pro Xaa Ser Xaa Xaa Xaa Xaa Xaa Xaa Xaa Xaa Phe Xaa Xaa (SEQ ID NO: 50002)

Ser Ser Gly Trp Xaa Xaa Phe Asp Xaa (SEQ ID NO: 50003)

Asn Tyr Asp Ile Asn (SEQ ID NO: 50468)

Trp Met His Pro Asn Ser Gly Asn Thr Gly Tyr Ala Gln Lys Phe Gln Gly (SEQ ID NO: 50469)

Ser Ser Gly Trp Tyr Tyr Phe Asp Tyr (SEQ ID NO: 50470)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | V | Q | L | V | Q | S | - | G | A | E | V | K | K | P | G | A | S | V | K | V | S | C | K | A |
| 21-225_11E5_HC | . | . | . | . | . | . | . | . | . | P | . | . | . | . | . | . | . | . | . | . | . | . | . | . | . |
| 21-225_12F11_HC | . | . | . | . | . | . | . | . | . | . | . | . | . | . | . | . | . | . | . | . | . | . | . | . | . |
| 21-225_12F12_HC | . | . | . | . | L | . | . | . | . | . | . | . | . | . | . | . | . | . | . | . | . | . | . | . | . |
| 21-225_146C9_HC | . | . | . | . | . | . | . | . | . | . | . | . | . | . | . | . | . | . | . | . | . | . | . | . | . |
| 21-225_146D4_HC | . | . | . | . | . | . | . | . | . | . | . | . | . | . | . | . | . | . | . | . | . | . | . | . | . |
| 21-225_146F9_HC | . | . | . | . | . | . | . | . | . | . | . | . | . | . | . | . | . | . | . | . | . | . | . | . | . |
| 21-225_147D5_HC | . | . | . | . | . | . | . | . | . | . | . | . | . | . | . | . | . | . | . | . | . | . | . | . | . |
| 21-225_147E4_HC | . | . | . | . | . | . | . | . | . | . | . | . | . | . | . | . | . | . | . | . | . | . | . | . | . |
| 21-225_148F8_HC | . | . | . | . | . | . | . | . | . | . | . | . | . | . | . | . | . | . | . | . | . | . | . | . | . |
| 21-225_148G3_HC | . | . | . | . | . | . | . | . | . | . | . | . | . | . | . | . | . | . | . | . | . | . | . | . | . |
| 21-225_149A2_HC | . | . | . | . | . | . | . | . | . | . | . | . | . | . | . | . | . | . | . | . | . | . | . | . | . |
| 21-225_149E3_HC | . | . | . | . | . | . | . | . | . | . | . | . | . | . | . | . | . | . | . | . | . | . | . | . | . |
| 21-225_149H4_HC | . | . | . | . | . | . | . | . | . | . | . | . | . | . | . | . | . | . | . | . | . | . | . | . | . |
| 21-225_150B7_HC | . | . | . | . | . | . | . | . | . | . | . | . | . | . | . | . | . | . | . | T | . | . | . | . | . |
| 21-225_150D2_HC | . | . | . | . | . | . | . | . | . | . | . | . | . | . | . | . | . | . | . | . | . | . | . | . | . |
| 21-225_152E12_HC | . | . | . | . | . | . | . | . | . | . | . | . | . | . | . | . | . | . | . | . | . | . | . | . | . |
| 21-225_152E3_HC | . | . | . | . | . | . | . | . | . | . | . | . | . | . | . | . | . | . | . | . | . | . | . | . | . |
| 21-225_152H3_HC | . | . | . | . | . | . | . | . | . | . | . | . | . | . | . | . | . | . | . | . | . | . | . | . | . |
| 21-225_153F11_HC | . | . | . | . | . | . | . | . | . | . | . | . | . | E | . | . | . | . | . | . | . | . | . | . | . |
| 21-225_154A11_HC | . | . | . | . | . | . | . | . | . | . | . | . | . | . | . | . | . | . | . | . | . | . | . | . | . |
| 21-225_154H6_HC | . | . | . | . | . | . | . | . | . | . | . | . | . | . | . | . | . | . | . | . | . | . | . | . | . |
| 21-225_155B6_HC | . | . | . | . | . | . | . | . | . | . | . | . | . | . | . | . | . | . | . | . | . | . | . | . | . |
| 21-225_155E5_HC | . | . | . | . | . | . | . | . | . | . | . | . | . | . | . | . | . | . | . | . | . | . | . | . | . |
| 21-225_156H1_HC | . | . | . | . | . | . | . | . | . | . | . | . | . | . | . | . | . | . | . | . | . | . | . | . | . |
| 21-225_158B12_HC | . | . | . | . | . | . | . | . | . | . | . | . | R | . | . | . | . | . | . | . | . | . | . | . | . |
| 21-225_160A4_HC | . | . | . | . | . | . | . | . | . | T | . | . | . | . | . | . | . | . | . | . | . | . | . | . | . |
| 21-225_160A7_HC | . | . | . | . | . | . | . | . | . | . | . | . | . | . | . | . | . | . | . | . | . | . | . | . | . |
| 21-225_162D5_HC | . | . | . | . | . | . | . | . | . | S | . | . | . | . | . | . | . | . | . | . | . | . | . | . | . |
| 21-225_162F6_HC | . | . | . | . | . | . | . | . | . | . | . | . | R | . | . | . | . | . | . | . | . | . | . | . | . |

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | H_FR1 | | | | | | | | | | | | | | | | | | | | | | | | |
| CONSENSUS | Q | V | Q | L | V | Q | S | - | G | A | E | V | K | K | P | G | A | S | V | K | V | S | C | K | A |
| 21-225_164A4_HC | . | . | . | . | . | . | . | . | . | . | . | . | . | . | . | . | . | . | . | . | . | . | . | . | . |
| 21-225_164A7_HC | . | . | . | . | . | . | . | . | . | . | . | . | . | . | . | . | . | . | . | . | . | . | . | . | . |
| 21-225_164B11_HC | . | . | . | . | . | . | . | . | . | . | . | . | . | . | . | . | . | . | . | . | . | . | . | . | . |
| 21-225_165H2_HC | . | . | . | . | . | . | . | . | . | . | . | . | . | . | . | . | . | . | . | . | . | . | . | . | . |
| 21-225_172E11_HC | . | . | . | . | . | . | . | . | . | . | . | . | . | . | . | . | . | . | . | M | . | . | . | . | . |
| 21-225_175D10_HC | . | . | . | . | . | . | . | . | . | . | . | . | . | . | . | . | . | . | . | . | . | . | . | . | . |
| 21-225_177B12_HC | . | . | . | . | . | . | . | . | . | . | . | . | . | . | . | . | . | . | . | . | . | . | . | . | . |
| 21-225_181E5_HC | . | . | . | . | . | . | . | . | . | . | . | . | . | . | . | . | . | . | . | . | . | . | . | . | . |
| 21-225_190D7_HC | . | . | . | . | . | . | . | . | . | . | . | . | . | . | . | . | . | . | . | . | . | . | . | . | . |
| 21-225_190H4_HC | . | . | . | . | . | . | . | . | . | . | . | . | . | . | . | . | . | . | . | . | . | . | . | . | . |
| 21-225_191D8_HC | . | . | . | . | . | . | . | . | . | . | . | . | . | . | . | . | . | . | . | . | . | . | . | . | . |
| 21-225_191G3_HC | . | . | . | . | . | . | . | . | . | . | . | . | . | . | . | . | . | . | . | . | . | . | . | . | . |
| 21-225_193G4_HC | . | . | . | . | . | . | . | . | . | . | . | . | . | . | . | . | . | . | . | . | . | . | . | . | . |
| 21-225_197C6_HC | . | . | . | . | . | . | . | . | . | . | . | . | . | . | . | . | . | . | . | . | . | . | . | . | . |
| 21-225_197H4_HC | . | . | . | . | . | . | . | . | . | . | . | . | . | . | . | . | . | . | . | . | . | . | . | . | . |
| 21-225_201F5_HC | . | . | . | . | . | . | . | . | . | . | A | . | . | . | . | . | . | . | . | . | . | . | . | . | . |
| 21-225_224C10_HC | . | . | . | . | . | . | . | . | . | . | . | . | . | . | . | . | . | . | . | . | . | . | . | . | . |
| 21-225_224D8_HC | . | . | . | . | . | . | . | . | . | . | . | . | . | . | . | . | . | . | . | . | . | . | . | . | . |
| 21-225_224F11_HC | . | . | . | . | . | . | . | . | . | . | . | . | . | . | . | . | . | . | . | . | . | . | . | . | . |
| 21-225_225B9_HC | . | . | . | . | . | . | . | . | . | T | . | . | R | . | . | . | . | . | . | . | . | . | . | . | . |
| 21-225_225F11_HC | . | . | . | . | . | . | . | . | . | . | . | . | . | . | . | . | . | . | . | . | . | . | . | . | . |
| 21-225_225F12_HC | . | . | . | . | . | . | . | . | . | . | . | . | . | . | . | . | . | . | . | . | . | . | . | . | . |
| 21-225_225F5_HC | . | . | . | . | . | . | . | . | . | . | . | . | . | . | . | R | . | . | . | . | . | . | . | . | . |
| 21-225_225H12_HC | . | . | . | . | . | . | . | . | . | . | . | . | . | . | . | . | . | . | . | . | . | . | . | . | . |
| 21-225_226A12_HC | . | . | . | . | . | . | . | . | . | . | . | . | . | . | . | . | . | . | . | . | . | . | . | . | . |
| 21-225_226D11_HC | . | . | . | . | . | . | . | . | . | . | . | . | . | . | . | . | . | . | . | . | . | . | . | R | S |
| 21-225_226D6_HC | . | . | . | . | . | . | . | . | . | . | . | . | . | . | . | . | . | . | . | . | . | . | . | . | . |
| 21-225_226F6_HC | . | . | . | . | . | . | . | . | . | . | . | . | . | . | . | . | . | . | . | . | . | . | . | . | . |
| 21-225_227C7_HC | . | . | . | . | . | . | . | . | . | . | . | . | . | . | . | R | . | . | . | . | . | . | . | . | . |

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | H_FR1 | | | | | | | | | | | | | | | | | | | | | | | | |
| CONSENSUS | Q | V | Q | L | V | Q | S | - | G | A | E | V | K | K | P | G | A | S | V | K | V | S | C | K | A |
| 21-225_227D11_HC | . | . | . | . | . | . | . | . | . | . | . | . | . | . | . | . | . | . | . | . | . | . | . | . | . |
| 21-225_227E6_HC | . | . | . | . | . | . | . | . | . | . | . | . | . | . | . | . | . | . | . | . | . | . | . | . | . |
| 21-225_22D2_HC | . | . | . | . | . | . | . | . | . | . | . | . | . | . | . | . | . | . | . | . | . | . | . | . | . |
| 21-225_23A12_HC | . | . | . | . | . | . | . | . | . | . | . | . | . | . | . | . | . | . | . | . | . | . | . | . | . |
| 21-225_24E1_HC | . | . | . | . | . | . | . | . | . | . | . | . | . | . | . | . | . | . | . | . | . | . | . | . | . |
| 21-225_25A4_HC | . | . | L | . | . | . | . | . | . | . | . | . | . | R | . | . | . | . | . | . | . | . | . | . | . |
| 21-225_25A4.001_HC | . | . | L | . | . | . | . | . | . | . | . | . | . | R | . | . | . | . | . | . | . | . | . | . | . |
| 21-225_25A4.001.001_HC | . | . | L | . | . | . | . | . | . | . | . | . | . | R | . | . | . | . | . | . | . | . | . | . | . |
| 21-225_25A4.001.002_HC | . | . | L | . | . | . | . | . | . | . | . | . | . | R | . | . | . | . | . | . | . | . | . | . | . |
| 21-225_25A4.001.003_HC | . | . | L | . | . | . | . | . | . | . | . | . | . | R | . | . | . | . | . | . | . | . | . | . | . |
| 21-225_25A4.001.004_HC | . | . | L | . | . | . | . | . | . | . | . | . | . | R | . | . | . | . | . | . | . | . | . | . | . |
| 21-225_25A4.001.005_HC | . | . | . | . | . | . | . | . | . | . | . | . | . | R | . | . | . | . | . | . | . | . | . | . | . |
| 21-225_25A4.001.006_HC | . | . | . | . | . | . | . | . | . | . | . | . | . | R | . | . | . | . | . | . | . | . | . | . | . |
| 21-225_25A4.001.007_HC | . | . | . | . | . | . | . | . | . | . | . | . | . | R | . | . | . | . | . | . | . | . | . | . | . |
| 21-225_25A4.001.008_HC | . | . | . | . | . | . | . | . | . | . | . | . | . | R | . | . | . | . | . | . | . | . | . | . | . |
| 21-225_25A4.001.013_HC | . | . | L | . | . | . | . | . | . | . | . | . | . | R | . | . | . | . | . | . | . | . | . | . | . |
| 21-225_25A4.001.019_HC | . | . | . | . | . | . | . | . | . | . | . | . | . | R | . | . | . | . | . | . | . | . | . | . | . |
| 21-225_25A4.001.020_HC | . | . | L | . | . | . | . | . | . | . | . | . | . | R | . | . | . | . | . | . | . | . | . | . | . |
| 21-225_25A4.001.021_HC | . | . | . | . | . | . | . | . | . | . | . | . | . | R | . | . | . | . | . | . | . | . | . | . | . |
| 21-225_25A4.001.029_HC | . | . | L | . | . | . | . | . | . | . | . | . | . | R | . | . | . | . | . | . | . | . | . | . | . |
| 21-225_26C4_HC | . | . | . | . | . | . | . | . | . | . | . | . | . | . | . | . | . | . | . | . | . | . | . | . | . |
| 21-225_28B6_HC | . | . | L | . | . | . | . | . | . | . | . | . | . | R | . | . | . | . | . | . | . | . | . | . | . |
| 21-225_29E6_HC | . | . | L | . | . | . | . | . | . | . | . | . | . | R | . | . | . | . | . | . | . | . | . | . | . |
| 21-225_29H8_HC | . | . | . | . | . | . | . | . | . | . | . | . | Q | . | . | . | . | . | . | . | . | . | . | . | . |
| 21-225_32A1_HC | . | . | . | . | . | . | . | . | . | . | . | . | . | . | . | . | . | . | . | . | . | . | . | . | . |
| 21-225_32A5_HC | . | . | . | . | . | . | . | . | . | . | . | . | . | . | . | . | . | . | . | . | . | . | . | . | . |
| 21-225_33D1_HC | . | . | . | . | . | . | . | . | . | . | . | . | . | . | . | . | . | . | . | . | . | . | . | . | . |
| 21-225_34D2_HC | . | . | . | . | . | . | . | . | . | . | . | . | . | . | . | . | . | . | . | . | . | . | . | . | . |
| 21-225_34H7_HC | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | H_FR1 | | | | | | | | | | | | | | | | | | | | | | | | |
| CONSENSUS | Q | V | Q | L | V | Q | S | - | G | A | E | V | K | K | P | G | A | S | V | K | V | S | C | K | A |
| 21-225_6G6_HC | . | . | . | . | . | . | . | . | . | . | . | . | R | . | . | . | . | . | . | . | . | . | . | . | . |
| 21-225_70E7_HC | . | . | . | . | . | . | . | . | . | . | . | . | . | . | . | . | . | . | . | . | . | . | . | . | . |
| 21-225_71F3_HC | . | . | . | . | . | . | . | . | . | . | . | . | . | . | . | . | . | . | . | . | . | . | . | . | . |
| 21-225_71G3_HC | . | . | . | . | . | . | . | . | . | . | . | . | . | . | . | . | . | . | . | . | . | . | . | . | . |
| 21-225_72C4_HC | . | . | . | . | . | . | . | . | . | . | . | . | . | . | . | . | . | . | . | . | . | . | . | . | . |
| 21-225_74A6_HC | . | . | . | . | . | . | . | . | . | . | . | . | . | . | . | . | . | . | . | . | . | . | . | . | . |
| 21-225_74A8_HC | . | . | . | . | . | . | . | . | . | . | . | . | . | . | . | . | . | . | . | . | . | . | . | . | . |
| 21-225_74B11_HC | . | . | . | . | . | . | . | . | . | . | . | . | . | . | . | . | . | . | . | R | . | . | . | . | . |
| 21-225_74F6_HC | . | . | . | . | . | . | . | . | . | . | . | . | . | . | . | . | . | . | . | R | . | . | . | . | . |
| 21-225_74F9_HC | . | . | . | . | . | . | . | . | . | . | . | . | . | . | . | . | . | . | . | . | . | . | . | . | . |
| 21-225_74G4_HC | . | . | . | . | . | . | . | . | . | . | . | . | . | . | . | . | . | . | . | . | . | . | . | . | . |
| 21-225_75A10_HC | . | . | . | . | . | . | . | . | . | . | . | . | . | . | . | . | . | . | . | . | . | . | . | . | . |
| 21-225_75A6_HC | . | . | . | . | . | . | . | . | . | . | . | . | . | . | . | . | . | . | . | . | . | . | . | . | . |
| 21-225_75C4_HC | . | . | . | . | . | . | . | . | . | . | . | . | . | . | . | . | . | . | . | . | . | . | . | . | . |
| 21-225_75F9_HC | . | . | . | . | . | . | . | . | . | . | . | . | . | . | . | . | . | . | . | . | . | . | . | . | . |
| 21-225_76E11_HC | . | . | . | . | . | . | . | . | . | . | . | . | . | . | . | . | . | . | . | . | . | . | . | . | . |
| 21-225_76G2_HC | . | . | . | . | . | . | . | . | . | . | . | . | . | . | . | . | . | . | . | . | . | . | . | . | . |
| 21-225_78E11_HC | . | . | . | . | . | . | . | . | . | . | . | . | . | . | . | . | . | . | . | . | . | . | . | . | . |
| 21-225_78E6_HC | . | . | . | . | . | . | . | . | . | . | . | . | . | . | . | . | . | . | . | . | . | . | . | . | . |
| 21-225_79E9_HC | . | . | . | . | . | . | . | . | . | . | . | . | . | . | . | . | . | . | . | . | . | . | . | . | . |
| 21-225_79F12_HC | . | . | . | . | . | . | . | . | . | . | . | . | . | . | . | . | . | . | . | . | . | . | . | . | . |
| 21-225_79G7_HC | . | . | . | . | . | . | . | . | . | . | . | . | . | . | . | . | . | . | . | . | . | . | . | . | . |
| 21-225_80B12_HC | . | . | . | . | . | . | . | . | . | . | . | . | . | . | . | . | . | . | . | . | . | . | . | . | T |
| 21-225_85C11_HC | . | . | . | . | . | . | . | . | . | . | . | . | . | . | . | . | . | . | . | . | . | . | . | . | . |
| 21-225_87E10_HC | . | . | . | . | . | . | . | . | . | . | . | . | . | . | . | . | . | . | . | . | . | . | . | . | . |
| 21-225_88A1_HC | . | . | . | . | . | . | . | . | . | . | . | . | . | . | . | . | . | . | . | . | . | . | . | . | . |
| 21-225_88B4_HC | . | . | . | . | . | . | . | . | . | . | . | . | . | . | . | . | . | . | . | . | . | . | . | . | . |
| 21-225_88C10_HC | . | . | . | . | . | . | . | . | . | . | . | . | . | . | . | . | . | . | . | R | . | . | . | . | . |
| 21-225_8B11_HC | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | H_FR1 | | | | | | | | | | | | | | | | | | | | | | | | |
| CONSENSUS | Q | V | Q | L | V | Q | S | - | G | A | E | V | K | K | P | G | A | S | V | K | V | S | C | K | A |
| 21-225_92E6_HC | . | . | . | . | . | . | . | . | . | . | . | . | . | . | . | . | . | . | . | | . | . | . | . | . |
| 21-225_94D8_HC | . | . | . | . | . | . | . | . | . | . | . | . | . | . | . | . | . | . | . | . | . | . | . | . | T |
| 21-225_96D10_HC | . | . | . | . | . | . | . | . | . | . | . | . | . | . | . | . | . | . | . | | . | . | . | . | . |
| 21-225_96H5_HC | . | . | . | . | . | . | . | . | . | . | . | . | . | . | . | . | . | . | . | . | . | . | . | . | . |

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | H_FR1 | | | | | | | | | | | | | | | | | | | | | | | | |
| CONSENSUS | Q | V | Q | L | V | Q | S | - | G | A | E | V | K | K | P | G | A | S | V | K | V | S | C | K | A |
| 21-225_43D8_HC | . | . | . | . | . | . | . | . | . | . | . | . | . | . | . | . | . | . | . | . | . | . | . | . | . |
| 21-225_4A2_HC | . | . | . | . | . | . | . | . | . | T | . | . | . | . | . | . | . | . | . | . | . | . | . | . | . |
| 21-225_4A2.001_HC | . | . | . | . | . | . | . | . | . | T | . | . | . | . | . | . | . | . | . | . | . | . | . | . | . |
| 21-225_4A2.001.001_HC | . | . | . | . | . | . | . | . | . | T | . | . | . | . | . | . | . | . | . | . | . | . | . | . | . |
| 21-225_4A2.001.002_HC | . | . | . | . | . | . | . | . | . | T | . | . | . | . | . | . | . | . | . | . | . | . | . | . | . |
| 21-225_4A2.001.003_HC | . | . | . | . | . | . | . | . | . | T | . | . | . | . | . | . | . | . | . | . | . | . | . | . | . |
| 21-225_4A2.001.004_HC | . | . | . | . | . | . | . | . | . | T | . | . | . | . | . | . | . | . | . | . | . | . | . | . | . |
| 21-225_4A2.001.005_HC | . | . | . | . | . | . | . | . | . | T | . | . | . | . | . | . | . | . | . | . | . | . | . | . | . |
| 21-225_4A2.001.006_HC | . | . | . | . | . | . | . | . | . | T | . | . | . | . | . | . | . | . | . | . | . | . | . | . | . |
| 21-225_4A2.001.007_HC | . | . | . | . | . | . | . | . | . | T | . | . | . | . | . | . | . | . | . | . | . | . | . | . | . |
| 21-225_4A2.001.008_HC | . | . | . | . | . | . | . | . | . | T | . | . | . | . | . | . | . | . | . | . | . | . | . | . | . |
| 21-225_4A2.001.009_HC | . | . | . | . | . | . | . | . | . | T | . | . | . | . | . | . | . | . | . | . | . | . | . | . | . |
| 21-225_4A2.001.010_HC | . | . | . | . | . | . | . | . | . | T | . | . | . | . | . | . | . | . | . | . | . | . | . | . | . |
| 21-225_4A2.001.011_HC | . | . | . | . | . | . | . | . | . | T | . | . | . | . | . | . | . | . | . | . | . | . | . | . | . |
| 21-225_4A2.001.012_HC | . | . | . | . | . | . | . | . | . | T | . | . | . | . | . | . | . | . | . | . | . | . | . | . | . |
| 21-225_4A2.001.022_HC | . | . | . | . | . | . | . | . | . | . | . | . | . | . | . | . | . | . | . | . | . | . | . | . | . |
| 21-225_4A2.001.023_HC | . | . | . | . | . | . | . | . | . | . | . | . | . | . | . | . | . | . | . | . | . | . | . | . | . |
| 21-225_4A2.001.024_HC | . | . | . | . | . | . | . | . | . | . | . | . | . | . | . | . | . | . | . | . | . | . | . | . | . |
| 21-225_4A2.001.028_HC | . | . | . | . | . | . | . | . | . | . | . | . | . | . | . | . | . | . | . | . | . | . | . | . | . |
| 21-225_4A2.001.029_HC | . | . | . | . | . | . | . | . | . | . | . | . | . | . | . | . | . | . | . | . | . | . | . | . | . |
| 21-225_4A2.001.031_HC | . | . | . | . | . | . | . | . | . | G | . | . | . | . | . | . | . | . | . | . | . | . | . | . | . |
| 21-225_56A1_HC | . | . | . | . | . | . | . | . | . | . | . | . | . | . | . | . | . | . | . | . | . | . | . | . | . |
| 21-225_58B9_HC | . | . | . | . | . | . | . | . | . | . | . | . | . | . | . | . | . | . | . | . | . | . | . | . | . |
| 21-225_59E1_HC | . | . | . | . | . | . | . | . | . | . | . | . | . | . | . | . | . | . | . | . | . | . | . | . | . |
| 21-225_59F10_HC | . | . | . | . | . | . | . | . | . | . | . | . | . | . | . | . | . | . | . | . | . | . | . | . | . |
| 21-225_60F3_HC | . | . | L | . | . | . | . | . | . | . | . | . | . | . | . | . | . | . | . | . | . | . | . | . | . |
| 21-225_61B5_HC | . | . | . | . | . | . | . | . | . | . | . | . | . | . | . | . | . | . | . | . | . | . | . | . | . |
| 21-225_61E3_HC | . | . | . | . | . | . | . | . | . | . | . | . | . | . | . | . | . | . | . | . | . | . | . | . | . |
| 21-225_63F4_HC | . | . | . | . | . | . | . | . | . | . | . | . | . | . | . | . | . | . | . | . | . | . | . | . | . |

FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | | | H_CDR1 | | | | | | | | | | | | H_FR2 | |
| CONSENSUS | S | G | - | Y | T | F | T | N | - | - | - | - | - | Y | D | I | N | W | V | R | Q | A | T | G | Q |
| 21-225_11E5_HC | . | . | . | . | . | . | . | . | . | . | . | . | . | . | . | . | . | . | . | . | . | . | . | . | . |
| 21-225_12F11_HC | . | . | . | . | . | . | . | . | . | . | . | . | . | . | . | . | . | . | . | . | . | . | . | . | . |
| 21-225_12F12_HC | . | . | . | . | . | . | . | . | . | . | . | . | . | . | . | . | . | . | . | . | . | . | . | . | . |
| 21-225_146C9_HC | . | . | . | . | . | . | . | . | . | . | . | . | . | . | . | . | . | . | . | . | . | . | . | . | . |
| 21-225_146D4_HC | . | . | . | . | . | . | . | . | . | . | . | . | . | . | . | . | . | . | . | . | . | . | . | . | . |
| 21-225_146F9_HC | . | . | . | . | . | . | . | . | . | . | . | . | . | . | . | . | . | . | . | . | . | . | . | . | . |
| 21-225_147D5_HC | . | . | . | . | . | . | . | . | . | . | . | . | . | . | . | . | . | . | . | . | . | . | . | . | . |
| 21-225_147E4_HC | . | . | . | . | . | . | . | . | . | . | . | . | . | . | . | . | . | . | . | . | . | . | . | . | . |
| 21-225_148F8_HC | . | . | . | . | . | . | . | . | . | . | . | . | . | . | . | . | . | . | . | . | . | . | . | . | . |
| 21-225_148G3_HC | . | . | . | . | . | . | . | . | . | . | . | . | . | . | . | . | . | . | . | . | . | . | S | . | . |
| 21-225_149A2_HC | . | . | . | . | . | . | . | . | . | . | . | . | . | . | . | . | . | . | . | . | . | . | . | . | . |
| 21-225_149E3_HC | . | . | . | . | . | . | . | . | . | . | . | . | . | . | . | . | . | . | . | . | . | . | . | . | . |
| 21-225_149H4_HC | . | . | . | . | . | . | . | . | . | . | . | . | . | . | . | . | . | . | . | . | . | . | . | . | . |
| 21-225_150B7_HC | . | . | . | F | P | . | . | . | . | . | . | . | . | . | . | . | . | . | . | . | . | . | . | . | . |
| 21-225_150D2_HC | . | . | . | . | . | . | . | . | . | . | . | . | . | . | . | . | . | . | . | . | . | . | . | . | . |
| 21-225_152E12_HC | . | . | . | . | . | . | . | . | . | . | . | . | . | . | . | . | . | . | . | . | . | . | . | . | . |
| 21-225_152E3_HC | . | . | . | . | . | . | . | . | . | . | . | . | . | . | . | . | . | . | . | . | . | . | . | . | . |
| 21-225_152H3_HC | . | . | . | . | . | . | P | . | . | . | . | . | . | . | . | . | . | . | . | . | . | . | . | . | . |
| 21-225_153F11_HC | . | . | . | . | . | . | . | . | . | . | . | . | . | . | . | . | . | . | . | . | . | . | . | . | . |
| 21-225_154A11_HC | . | . | . | . | . | . | . | . | . | . | . | . | . | . | . | . | . | . | . | . | . | . | . | . | . |
| 21-225_154H6_HC | . | . | . | . | . | . | . | . | . | . | . | . | . | . | . | . | . | . | . | . | . | . | . | . | . |
| 21-225_155B6_HC | . | . | . | . | . | . | . | . | . | . | . | . | . | . | . | . | . | . | . | . | . | . | . | . | . |
| 21-225_155E5_HC | . | . | . | . | . | . | . | . | . | . | . | . | . | . | . | . | . | . | . | . | . | . | . | . | . |
| 21-225_156H1_HC | . | . | . | . | . | . | . | . | . | . | . | . | . | . | . | . | . | . | . | . | . | . | . | . | . |
| 21-225_158B12_HC | . | . | . | . | . | . | . | . | . | . | . | . | . | . | . | . | . | . | . | . | . | . | . | . | . |
| 21-225_160A4_HC | . | . | . | . | . | . | . | . | . | . | . | . | . | . | . | . | . | . | . | . | . | . | . | . | . |
| 21-225_160A7_HC | . | . | . | . | . | . | . | . | . | . | . | . | . | . | . | . | . | . | . | . | . | . | . | . | . |
| 21-225_162D5_HC | . | . | . | . | . | . | . | . | . | . | . | . | . | . | . | . | . | . | . | . | . | . | . | . | . |
| 21-225_162F6_HC | . | . | . | . | . | . | . | . | . | . | . | . | . | . | . | . | . | . | . | . | . | . | . | . | . |

# FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | H_CDR1 | | | | | | | | | | H_FR2 | | | | | | | |
| CONSENSUS | S | G | - | Y | T | F | T | N | - | - | - | - | - | Y | D | I | N | W | V | R | Q | A | T | G | Q |
| 21-225_164A4_HC | . | . | . | F | . | . | P | . | . | . | . | . | . | . | . | . | . | . | . | . | . | . | . | . | . |
| 21-225_164A7_HC | . | . | . | . | . | . | . | . | . | . | . | . | . | . | . | V | . | . | . | . | . | . | . | . | . |
| 21-225_164B11_HC | . | . | . | . | . | . | . | . | . | . | . | . | . | . | . | . | . | . | . | . | . | . | . | . | . |
| 21-225_165H2_HC | . | . | . | . | . | . | . | H | . | . | . | . | . | . | . | . | . | . | . | . | . | . | . | . | . |
| 21-225_172E11_HC | . | . | . | . | . | . | . | . | . | . | . | . | . | . | . | . | . | . | . | . | . | . | . | . | . |
| 21-225_175D10_HC | . | . | . | . | . | . | . | . | . | . | . | . | . | . | . | L | . | . | . | . | . | . | . | . | . |
| 21-225_177B12_HC | . | . | . | . | . | . | . | . | . | . | . | . | . | . | . | . | . | . | . | . | . | . | . | . | . |
| 21-225_181E5_HC | . | . | . | . | . | . | . | . | . | . | . | . | . | . | . | . | . | . | . | . | . | . | . | . | . |
| 21-225_190D7_HC | . | . | . | . | . | . | . | . | . | . | . | . | . | . | . | . | . | . | . | . | . | . | . | . | . |
| 21-225_190H4_HC | . | . | . | N | . | . | . | . | . | . | . | . | . | . | . | . | . | . | . | . | . | . | . | . | . |
| 21-225_191D8_HC | . | . | . | . | . | . | . | . | . | . | . | . | . | . | . | . | . | . | . | . | . | . | . | . | . |
| 21-225_191G3_HC | . | . | . | . | . | . | . | . | . | . | . | . | . | . | . | . | . | . | . | . | . | . | . | . | . |
| 21-225_193G4_HC | . | . | . | . | . | . | . | . | . | . | . | . | . | . | . | . | . | . | . | . | . | . | . | . | . |
| 21-225_197C6_HC | . | . | . | . | . | . | . | . | . | . | . | . | . | . | . | . | . | . | . | . | . | . | . | . | . |
| 21-225_197H4_HC | . | . | . | . | . | . | . | . | . | . | . | . | . | . | . | . | . | . | . | . | . | . | . | . | . |
| 21-225_201F5_HC | . | . | . | . | . | . | . | . | . | . | . | . | . | . | . | . | . | . | . | . | . | . | . | . | . |
| 21-225_224C10_HC | . | . | . | S | . | . | . | . | . | . | . | . | . | . | . | . | . | . | . | . | . | . | . | . | . |
| 21-225_224D8_HC | . | . | . | . | . | . | . | . | . | . | . | . | . | . | . | . | . | . | . | . | . | . | . | . | . |
| 21-225_224F11_HC | . | . | . | . | . | . | . | . | . | . | . | . | . | . | . | . | . | . | . | . | . | . | . | . | . |
| 21-225_225B9_HC | . | . | . | . | . | . | . | . | . | . | . | . | . | . | . | . | . | . | . | . | . | . | . | . | . |
| 21-225_225F11_HC | . | . | . | . | . | . | . | . | . | . | . | . | . | . | . | . | . | . | . | . | . | . | . | . | . |
| 21-225_225F12_HC | . | . | . | . | . | . | . | H | . | . | . | . | . | . | . | . | . | . | . | . | . | . | . | . | . |
| 21-225_225F5_HC | . | . | . | H | . | . | . | . | . | . | . | . | . | . | . | . | . | . | . | . | . | . | . | . | . |
| 21-225_225H12_HC | . | . | . | . | . | . | . | . | . | . | . | . | . | . | . | . | . | . | . | . | . | . | . | . | . |
| 21-225_226A12_HC | . | . | . | . | . | . | . | . | . | . | . | . | . | . | . | . | . | . | . | . | . | . | . | . | . |
| 21-225_226D11_HC | . | . | . | . | . | . | . | . | . | . | . | . | . | . | . | . | . | . | . | . | . | . | . | . | . |
| 21-225_226D6_HC | . | . | . | . | . | . | . | . | . | . | . | . | . | . | . | . | . | . | . | . | . | . | . | . | . |
| 21-225_226F6_HC | . | . | . | . | . | . | . | . | . | . | . | . | . | . | . | . | . | . | . | . | . | . | . | . | . |
| 21-225_227C7_HC | . | . | . | H | . | . | . | . | . | . | . | . | . | . | . | . | . | . | . | . | . | . | . | . | . |

FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | | | H_CDR1 | | | | | | | | | | | | H_FR2 | |
| CONSENSUS | S | G | - | Y | T | F | T | N | - | - | - | - | - | Y | D | I | N | W | V | R | Q | A | T | G | Q |
| 21-225_227D11_HC | . | . | . | . | . | . | . | . | . | . | . | . | . | . | . | . | . | . | . | . | . | . | . | . | . |
| 21-225_227E6_HC | . | . | . | . | . | . | . | . | . | . | . | . | . | . | . | . | . | . | . | . | . | . | . | . | . |
| 21-225_22D2_HC | . | . | . | . | . | . | I | . | . | . | . | . | . | . | . | . | . | . | . | . | . | . | . | . | . |
| 21-225_23A12_HC | . | . | . | . | P | . | . | . | . | . | . | . | . | . | . | . | . | . | . | . | . | . | . | . | . |
| 21-225_24E1_HC | . | . | . | . | . | . | . | . | . | . | . | . | . | . | . | . | . | . | . | . | . | . | . | . | . |
| 21-225_25A4_HC | . | . | . | . | . | . | . | . | . | . | . | . | . | . | . | . | . | . | . | . | . | . | . | . | . |
| 21-225_25A4.001_HC | . | . | . | . | . | . | . | . | . | . | . | . | . | . | . | . | . | . | . | . | . | . | . | . | . |
| 21-225_25A4.001.001_HC | . | . | . | . | . | . | . | . | . | . | . | . | . | . | . | . | . | . | . | . | . | . | . | . | . |
| 21-225_25A4.001.002_HC | . | . | . | . | . | . | . | . | . | . | . | . | . | . | . | . | . | . | . | . | . | . | . | . | . |
| 21-225_25A4.001.003_HC | . | . | . | . | . | . | . | . | . | . | . | . | . | . | . | . | . | . | . | . | . | . | . | . | . |
| 21-225_25A4.001.004_HC | . | . | . | . | . | . | . | . | . | . | . | . | . | . | . | . | . | . | . | . | . | . | . | . | . |
| 21-225_25A4.001.005_HC | . | . | . | . | . | . | . | . | . | . | . | . | . | . | . | . | . | . | . | . | . | . | . | . | . |
| 21-225_25A4.001.006_HC | . | . | . | . | . | . | . | . | . | . | . | . | . | . | . | . | . | . | . | . | . | . | . | . | . |
| 21-225_25A4.001.007_HC | . | . | . | . | . | . | . | . | . | . | . | . | . | . | . | . | . | . | . | . | . | . | . | . | . |
| 21-225_25A4.001.008_HC | . | . | . | . | . | . | . | . | . | . | . | . | . | . | . | . | . | . | . | . | . | . | . | . | . |
| 21-225_25A4.001.013_HC | . | . | . | . | . | . | . | . | . | . | . | . | . | . | . | . | . | . | . | . | . | . | . | . | . |
| 21-225_25A4.001.019_HC | . | . | . | . | . | . | . | . | . | . | . | . | . | . | . | . | . | . | . | . | . | . | P | . | . |
| 21-225_25A4.001.020_HC | . | . | . | . | . | . | . | . | . | . | . | . | . | . | . | . | . | . | . | . | . | . | P | . | . |
| 21-225_25A4.001.021_HC | . | . | . | . | . | . | . | . | . | . | . | . | . | . | . | . | . | . | . | . | . | . | . | . | . |
| 21-225_25A4.001.029_HC | . | . | . | . | . | . | . | . | . | . | . | . | . | . | . | . | . | . | . | . | . | . | . | . | . |
| 21-225_26C4_HC | . | . | . | . | . | . | . | . | . | . | . | . | . | . | . | . | . | . | . | . | . | . | S | . | . |
| 21-225_28B6_HC | . | . | . | . | . | . | . | . | . | . | . | . | . | . | . | . | . | . | . | . | . | . | . | . | . |
| 21-225_29E6_HC | . | . | . | . | . | . | . | . | . | . | . | . | . | . | . | . | . | . | . | . | . | . | . | . | . |
| 21-225_29H8_HC | . | . | . | . | . | . | . | . | . | . | . | . | . | . | . | . | . | . | . | . | . | . | . | . | . |
| 21-225_32A1_HC | . | . | . | . | . | . | . | . | . | . | . | . | . | . | . | . | . | . | . | . | . | . | . | . | . |
| 21-225_32A5_HC | . | . | . | . | . | . | . | . | . | . | . | . | . | . | . | . | . | . | . | . | . | . | . | . | . |
| 21-225_33D1_HC | . | . | . | . | . | . | . | . | . | . | . | . | . | . | . | . | . | . | . | . | . | . | . | . | . |
| 21-225_34D2_HC | . | . | . | . | . | . | . | . | . | . | . | . | . | . | . | . | . | . | . | . | . | . | . | . | . |
| 21-225_34H7_HC | . | . | . | . | . | . | . | . | . | . | . | . | . | . | . | . | . | . | . | . | . | . | . | . | . |

**FIGURE 57**

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | | | H_CDR1 | | | | | | | | | | | | | H_FR2 |
| CONSENSUS | S | G | - | Y | T | F | T | N | - | - | - | - | - | Y | D | I | N | W | V | R | Q | A | T | G | Q |
| 21-225_43D8_HC | . | . | . | . | . | . | . | . | . | . | . | . | . | . | . | . | . | . | . | . | . | . | . | . | . |
| 21-225_4A2_HC | . | . | . | . | . | . | . | . | . | . | . | . | . | . | . | . | . | . | . | . | . | . | . | . | . |
| 21-225_4A2.001_HC | . | . | . | . | . | . | . | . | . | . | . | . | . | . | . | . | . | . | . | . | . | . | . | . | . |
| 21-225_4A2.001.001_HC | . | . | . | . | . | . | . | . | . | . | . | . | . | . | . | . | . | . | . | . | . | . | . | . | . |
| 21-225_4A2.001.002_HC | . | . | . | . | . | . | . | . | . | . | . | . | . | . | . | . | . | . | . | . | . | . | . | . | . |
| 21-225_4A2.001.003_HC | . | . | . | . | . | . | . | . | . | . | . | . | . | . | . | . | . | . | . | . | . | . | . | . | . |
| 21-225_4A2.001.004_HC | . | . | . | . | . | . | . | . | . | . | . | . | . | . | . | . | . | . | . | . | . | . | . | . | . |
| 21-225_4A2.001.005_HC | . | . | . | . | . | . | . | . | . | . | . | . | . | . | . | . | . | . | . | . | . | . | . | . | . |
| 21-225_4A2.001.006_HC | . | . | . | . | . | . | . | . | . | . | . | . | . | . | . | . | . | . | . | . | . | . | . | . | . |
| 21-225_4A2.001.007_HC | . | . | . | . | . | . | . | . | . | . | . | . | . | . | . | . | . | . | . | . | . | . | . | . | . |
| 21-225_4A2.001.008_HC | . | . | . | . | . | . | . | . | . | . | . | . | . | . | . | . | . | . | . | . | . | . | . | . | . |
| 21-225_4A2.001.009_HC | . | . | . | . | . | . | . | . | . | . | . | . | . | . | . | . | . | . | . | . | . | . | . | . | . |
| 21-225_4A2.001.010_HC | . | . | . | . | . | . | . | . | . | . | . | . | . | . | . | . | . | . | . | . | . | . | . | . | . |
| 21-225_4A2.001.011_HC | . | . | . | . | . | . | . | . | . | . | . | . | . | . | . | . | . | . | . | . | . | . | . | . | . |
| 21-225_4A2.001.012_HC | . | . | . | . | . | . | . | . | . | . | . | . | . | . | . | . | . | . | . | . | . | . | . | . | . |
| 21-225_4A2.001.022_HC | . | . | . | . | . | . | . | . | . | . | . | . | . | . | . | . | . | . | . | . | . | . | . | . | . |
| 21-225_4A2.001.023_HC | . | . | . | . | . | . | . | . | . | . | . | . | . | . | . | . | . | . | . | . | . | . | P | . | . |
| 21-225_4A2.001.024_HC | . | . | . | . | . | . | . | . | . | . | . | . | . | . | . | . | . | . | . | . | . | . | . | . | . |
| 21-225_4A2.001.028_HC | . | . | . | . | . | . | . | . | . | . | . | . | . | . | . | . | . | . | . | . | . | . | P | . | . |
| 21-225_4A2.001.029_HC | . | . | . | . | . | . | . | . | . | . | . | . | . | . | . | . | . | . | . | . | . | . | P | . | . |
| 21-225_4A2.001.031_HC | . | . | . | . | . | . | . | . | . | . | . | . | . | . | . | . | . | . | . | . | . | . | E | . | . |
| 21-225_56A1_HC | . | . | . | . | . | . | . | . | . | . | . | . | . | . | . | . | . | . | . | . | . | . | . | . | . |
| 21-225_58B9_HC | . | . | . | . | . | . | . | . | . | . | . | . | . | . | . | . | . | . | . | . | . | . | . | . | . |
| 21-225_59E1_HC | . | . | . | . | . | . | . | . | . | . | . | . | . | . | . | . | . | . | . | . | . | . | . | . | . |
| 21-225_59F10_HC | . | . | . | . | . | . | . | . | . | . | . | . | . | . | . | . | . | . | . | . | . | . | . | . | . |
| 21-225_60F3_HC | . | . | . | . | . | . | . | . | . | . | . | . | . | . | . | . | . | . | . | . | . | . | . | . | . |
| 21-225_61B5_HC | . | . | . | . | . | . | . | . | . | . | . | . | . | . | . | . | . | . | . | . | . | . | . | . | . |
| 21-225_61E3_HC | . | . | . | . | . | . | . | . | . | . | . | . | . | . | . | . | . | . | . | . | . | . | . | . | . |
| 21-225_63F4_HC | . | . | . | . | . | . | . | . | . | . | . | . | . | . | . | V | . | . | . | . | . | . | . | . | . |

# FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | | | H_CDR1 | | | | | | | | | | | | H_FR2 | |
| CONSENSUS | S | G | - | Y | T | F | T | N | - | - | - | - | - | Y | D | I | N | W | V | R | Q | A | T | G | Q |
| 21-225_6G6_HC | . | . | . | . | . | . | . | . | . | . | . | . | . | . | . | . | . | . | . | . | . | . | . | . | . |
| 21-225_70E7_HC | . | . | . | . | . | . | . | . | . | . | . | . | . | . | . | . | . | . | . | . | . | . | . | . | . |
| 21-225_71F3_HC | . | . | . | . | . | . | . | . | . | . | . | . | . | . | . | V | . | . | . | . | . | . | . | . | . |
| 21-225_71G3_HC | . | . | . | . | . | . | . | . | . | . | . | . | . | . | . | . | . | . | . | . | . | . | . | . | . |
| 21-225_72C4_HC | . | . | . | . | . | . | . | . | . | . | . | . | . | . | . | . | . | . | . | . | . | V | . | . | . |
| 21-225_74A6_HC | . | . | . | . | . | . | . | . | . | . | . | . | . | . | . | . | . | . | . | . | . | . | . | . | . |
| 21-225_74A8_HC | . | . | . | . | . | . | . | . | . | . | . | . | . | . | . | . | . | . | . | . | . | . | S | . | . |
| 21-225_74B11_HC | . | . | . | . | . | . | . | . | . | . | . | . | . | . | . | . | . | . | . | . | . | . | . | . | . |
| 21-225_74F6_HC | . | . | . | . | . | . | . | . | . | . | . | . | . | . | . | . | . | . | . | . | . | . | . | . | . |
| 21-225_74F9_HC | . | . | . | . | . | . | . | . | . | . | . | . | . | . | . | . | . | . | . | . | . | . | . | . | . |
| 21-225_74G4_HC | . | . | . | . | . | . | . | . | . | . | . | . | . | . | . | V | . | . | . | . | . | . | . | . | . |
| 21-225_75A10_HC | . | . | . | . | . | . | . | . | . | . | . | . | . | . | . | . | . | . | . | . | . | . | . | . | . |
| 21-225_75A6_HC | . | . | . | . | . | . | . | . | . | . | . | . | . | . | . | . | . | . | . | . | . | . | . | . | . |
| 21-225_75C4_HC | . | . | . | . | . | . | . | . | . | . | . | . | . | . | . | . | . | . | . | . | . | . | . | . | . |
| 21-225_75F9_HC | . | . | . | . | . | . | . | . | . | . | . | . | . | . | . | . | . | . | . | . | . | . | . | . | . |
| 21-225_76E11_HC | . | . | . | . | . | . | . | . | . | . | . | . | . | . | . | . | . | . | . | . | . | . | . | . | . |
| 21-225_76G2_HC | . | . | . | . | . | . | . | . | . | . | . | . | . | . | . | . | . | . | . | . | . | . | . | . | . |
| 21-225_78E11_HC | . | . | . | . | . | . | . | . | . | . | . | . | . | . | . | . | . | . | . | . | . | . | . | . | . |
| 21-225_78E6_HC | . | . | . | . | . | . | . | . | . | . | . | . | . | . | . | . | . | . | . | . | . | . | S | . | . |
| 21-225_79E9_HC | . | . | . | . | . | . | . | . | . | . | . | . | . | . | . | . | . | . | . | . | . | . | . | . | . |
| 21-225_79F12_HC | . | . | . | . | . | . | . | . | . | . | . | . | . | . | . | . | . | . | . | . | . | . | . | . | . |
| 21-225_79G7_HC | . | . | . | . | . | . | . | . | . | . | . | . | . | . | . | . | . | . | . | . | . | . | S | . | . |
| 21-225_80B12_HC | . | . | . | . | . | . | . | . | . | . | . | . | . | . | . | . | . | . | . | . | . | . | . | . | . |
| 21-225_85C11_HC | . | . | . | . | . | . | . | . | . | . | . | . | . | . | . | . | . | . | . | . | . | . | S | . | . |
| 21-225_87E10_HC | . | . | . | . | . | . | . | . | . | . | . | . | . | . | . | . | . | . | . | . | . | . | . | . | . |
| 21-225_88A1_HC | . | . | . | . | . | . | . | . | . | . | . | . | . | . | . | . | . | . | . | . | . | . | . | . | . |
| 21-225_88B4_HC | . | . | . | . | . | . | . | . | . | . | . | . | . | . | . | . | . | . | . | . | . | . | . | . | . |
| 21-225_88C10_HC | . | . | . | . | . | . | S | . | . | . | . | . | . | . | . | . | . | . | . | . | . | . | . | . | . |
| 21-225_8B11_HC | . | . | . | . | . | . | . | . | . | . | . | . | . | . | . | . | . | . | . | . | . | . | A | . | . |

FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | H_CDR1 | | | | | | | | | | H_FR2 | | | | | | | |
| CONSENSUS | S | G | - | Y | T | F | T | N | - | - | - | - | - | Y | D | I | N | W | V | R | Q | A | T | G | Q |
| 21-225_92E6_HC | . | . | . | . | . | . | . | . | . | . | . | . | . | . | . | . | . | . | . | . | . | . | . | . | . |
| 21-225_94D8_HC | . | . | . | . | . | . | . | . | . | . | . | . | . | . | . | . | . | . | . | . | . | . | . | . | . |
| 21-225_96D10_HC | . | . | . | . | . | . | . | . | . | . | . | . | . | . | . | . | . | . | . | . | . | . | . | . | . |
| 21-225_96H5_HC | . | . | . | . | . | . | . | . | . | . | . | . | . | . | . | . | . | . | . | . | . | . | S | . | . |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | H_CDR2 | | | | | | | | | | | | | | | | | | |
| CONSENSUS | G | L | E | W | M | G | W | M | H | P | N | - | - | - | S | G | N | T | G | Y | A | Q | K | F | Q |
| 21-225_11E5_HC | . | . | . | . | . | . | . | . | . | . | . | . | . | . | . | . | A | . | . | . | . | . | R | . | . |
| 21-225_12F11_HC | . | P | . | . | . | . | . | . | . | . | . | . | . | . | . | . | . | . | . | . | . | K | . | . | . |
| 21-225_12F12_HC | . | . | . | . | . | . | . | . | Y | . | . | . | . | . | . | . | . | . | . | . | . | . | . | . | . |
| 21-225_146C9_HC | . | . | . | . | . | . | . | . | . | . | . | . | . | . | . | . | . | . | . | . | . | . | . | . | . |
| 21-225_146D4_HC | . | . | . | . | . | . | . | V | . | . | . | . | . | . | . | . | . | . | . | . | . | . | . | . | . |
| 21-225_146F9_HC | . | . | . | . | . | . | . | . | . | . | . | . | . | . | . | . | . | . | . | . | . | . | . | . | . |
| 21-225_147D5_HC | . | . | . | . | . | . | . | V | . | . | . | . | . | . | . | . | . | . | . | . | . | . | . | . | . |
| 21-225_147E4_HC | . | . | . | . | . | . | . | . | N | . | . | . | . | . | . | . | . | . | . | . | . | . | . | . | . |
| 21-225_148F8_HC | . | . | . | . | . | . | . | . | N | . | . | . | . | . | . | . | . | . | . | . | . | . | . | . | . |
| 21-225_148G3_HC | . | . | . | . | . | . | . | . | . | . | . | . | . | . | . | . | . | . | . | . | . | . | N | . | . |
| 21-225_149A2_HC | . | . | . | . | . | . | . | V | . | . | . | . | . | . | . | . | . | . | . | . | . | . | . | . | . |
| 21-225_149E3_HC | . | . | . | . | . | . | . | . | . | . | . | . | . | . | . | . | . | . | . | . | . | . | . | . | . |
| 21-225_149H4_HC | . | . | . | . | . | . | . | . | . | . | . | . | . | . | . | . | . | . | D | . | . | . | . | . | . |
| 21-225_150B7_HC | . | . | . | . | . | . | . | . | . | . | . | . | . | . | . | . | . | . | . | . | . | . | . | . | . |
| 21-225_150D2_HC | . | . | . | . | . | . | . | . | . | . | . | . | . | . | . | . | . | . | . | . | . | . | . | . | . |
| 21-225_152E12_HC | . | . | . | . | . | . | . | . | N | . | . | . | . | . | . | . | . | . | . | . | . | . | . | . | . |
| 21-225_152E3_HC | . | . | . | . | . | . | . | . | . | . | . | . | . | . | . | . | . | . | . | . | . | . | . | . | . |
| 21-225_152H3_HC | . | . | . | . | . | . | . | . | . | . | . | . | . | . | . | . | . | . | . | . | . | . | . | . | . |
| 21-225_153F11_HC | . | . | . | . | . | . | . | . | . | . | . | . | . | . | . | . | . | . | . | . | . | . | . | . | . |
| 21-225_154A11_HC | . | . | . | . | . | . | . | . | N | . | . | . | . | . | . | . | . | . | . | . | . | . | . | . | . |
| 21-225_154H6_HC | . | . | . | . | . | . | . | . | N | . | . | . | . | . | . | . | . | . | . | . | . | . | . | . | . |
| 21-225_155B6_HC | . | . | . | . | . | . | . | . | N | . | . | . | . | . | . | . | . | . | . | . | . | . | . | . | . |
| 21-225_155E5_HC | . | . | . | . | . | . | . | . | . | . | . | . | . | . | . | . | S | . | . | . | . | . | R | . | . |
| 21-225_156H1_HC | . | . | . | . | . | . | . | V | . | . | . | . | . | . | . | . | . | . | . | . | . | . | . | . | . |
| 21-225_158B12_HC | . | . | . | . | . | . | . | . | . | . | . | . | . | . | . | . | . | . | . | . | . | . | . | . | . |
| 21-225_160A4_HC | . | P | . | . | . | . | . | . | . | . | . | . | . | . | . | . | . | . | . | . | P | . | . | . | . |
| 21-225_160A7_HC | . | . | . | . | . | . | . | . | . | . | H | . | . | . | . | . | . | . | . | . | . | . | . | . | . |
| 21-225_162D5_HC | . | . | . | . | . | . | . | . | . | . | . | . | . | . | . | . | . | . | . | . | . | . | . | . | . |
| 21-225_162F6_HC | . | . | . | . | . | . | . | . | . | . | . | . | . | . | . | . | . | . | . | . | . | . | . | . | . |

# FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | H_CDR2 | | | | | | | | | | | | | | | | | | |
| CONSENSUS | G | L | E | W | M | G | W | M | H | P | N | - | - | - | S | G | N | T | G | Y | A | Q | K | F | Q |
| 21-225_164A4_HC | . | . | . | . | . | . | . | . | . | . | . | . | . | . | . | . | . | . | . | . | . | . | . | . | . |
| 21-225_164A7_HC | . | . | . | . | . | . | . | . | . | . | . | . | . | . | . | . | . | . | . | . | . | . | . | . | . |
| 21-225_164B11_HC | . | . | . | . | . | . | . | . | T | . | . | . | . | . | . | . | . | . | . | . | . | . | . | . | . |
| 21-225_165H2_HC | . | . | . | . | V | . | . | . | . | . | . | . | . | . | . | H | K | . | . | . | . | . | . | . | . |
| 21-225_172E11_HC | . | . | . | . | . | . | . | . | . | . | . | . | . | . | . | . | . | . | . | . | . | . | . | . | . |
| 21-225_175D10_HC | . | . | . | . | . | . | . | . | . | . | . | . | . | . | . | . | . | . | . | . | . | . | . | . | . |
| 21-225_177B12_HC | . | . | . | . | . | . | . | . | . | . | . | . | . | . | . | . | . | . | . | . | . | . | . | . | . |
| 21-225_181E5_HC | . | . | . | . | . | . | . | . | N | . | . | . | . | . | . | . | . | . | . | . | . | . | . | . | . |
| 21-225_190D7_HC | . | . | . | . | . | . | . | . | N | . | . | . | . | . | . | . | . | . | . | . | . | . | . | . | . |
| 21-225_190H4_HC | . | . | . | . | . | . | . | . | N | . | . | . | . | . | . | . | . | . | . | . | . | . | . | . | . |
| 21-225_191D8_HC | . | . | . | . | . | . | . | . | . | . | . | . | . | . | . | . | . | . | . | . | . | . | . | . | . |
| 21-225_191G3_HC | . | . | . | . | . | . | R | . | N | . | . | . | . | . | . | . | . | . | . | . | . | . | . | . | . |
| 21-225_193G4_HC | . | . | . | . | . | . | . | . | N | . | . | . | . | . | . | . | . | . | . | . | . | . | . | . | . |
| 21-225_197C6_HC | . | . | . | . | . | . | . | . | . | . | . | . | . | . | . | . | . | . | . | . | . | . | . | . | . |
| 21-225_197H4_HC | . | . | . | . | . | . | . | . | . | . | . | . | . | . | . | . | . | . | . | . | . | . | . | . | . |
| 21-225_201F5_HC | . | . | . | . | . | . | . | . | . | . | . | . | . | . | . | . | . | . | . | . | . | . | . | . | . |
| 21-225_224C10_HC | . | . | . | . | . | . | . | . | . | . | . | . | . | . | . | . | . | . | . | . | . | . | . | . | . |
| 21-225_224D8_HC | . | . | . | . | . | . | . | L | Y | . | . | . | . | . | . | . | . | . | . | . | . | . | . | . | . |
| 21-225_224F11_HC | . | . | . | . | . | . | . | . | N | . | . | . | . | . | . | . | . | . | . | . | . | . | . | . | . |
| 21-225_225B9_HC | R | . | . | . | . | . | . | . | . | . | . | . | . | . | . | . | . | . | . | . | . | . | . | . | R |
| 21-225_225F11_HC | . | . | . | . | . | . | . | . | . | . | . | . | . | . | . | . | . | . | . | . | . | . | . | . | . |
| 21-225_225F12_HC | . | . | . | . | . | . | . | . | . | . | . | . | . | . | . | . | . | . | . | . | . | . | . | . | . |
| 21-225_225F5_HC | . | . | . | . | . | . | . | . | . | . | . | . | . | . | . | . | . | . | . | F | . | . | . | . | . |
| 21-225_225H12_HC | . | . | . | . | . | . | . | . | . | . | . | . | . | . | . | . | . | . | . | . | . | . | . | . | . |
| 21-225_226A12_HC | . | . | . | . | . | . | . | . | . | . | . | . | . | . | . | . | . | . | . | . | . | . | . | . | . |
| 21-225_226D11_HC | . | . | . | . | . | . | . | . | . | . | . | . | . | . | . | . | . | . | . | . | . | . | . | . | . |
| 21-225_226D6_HC | . | . | . | . | . | . | . | . | . | . | . | . | . | . | . | . | . | . | . | . | . | . | . | . | . |
| 21-225_226F6_HC | . | . | . | . | . | . | . | . | . | . | . | . | . | . | . | . | . | . | . | . | . | . | . | . | . |
| 21-225_227C7_HC | . | . | . | . | . | . | . | . | . | . | . | . | . | . | . | . | . | . | . | . | . | . | . | . | . |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | H_CDR2 | | | | | | | | | | | | | | | | | | |
| CONSENSUS | G | L | E | W | M | G | W | M | H | P | N | - | - | - | S | G | N | T | G | Y | A | Q | K | F | Q |
| 21-225_227D11_HC | . | . | . | . | . | . | . | . | . | . | . | . | . | . | . | . | . | . | . | . | . | . | . | . | . |
| 21-225_227E6_HC | . | . | . | . | . | . | . | . | . | . | . | . | . | . | . | . | . | . | . | . | . | . | . | . | . |
| 21-225_22D2_HC | . | . | . | . | . | . | . | . | . | . | D | . | . | . | . | . | . | . | . | . | . | . | . | . | . |
| 21-225_23A12_HC | . | . | . | . | . | . | . | . | . | . | . | . | . | . | . | . | . | . | . | . | . | . | . | . | . |
| 21-225_24E1_HC | . | . | . | . | . | . | . | . | . | . | . | . | . | . | . | . | . | . | . | . | . | . | R | . | . |
| 21-225_25A4_HC | . | . | . | . | . | . | . | . | Y | . | . | . | . | . | . | . | . | . | . | . | . | . | . | . | . |
| 21-225_25A4.001_HC | . | . | . | . | . | . | . | . | Y | . | . | . | . | . | . | . | . | . | . | . | . | . | . | . | . |
| 21-225_25A4.001.001_HC | . | . | . | . | . | . | . | . | Y | . | . | . | . | . | . | . | S | . | . | . | . | . | . | . | . |
| 21-225_25A4.001.002_HC | . | . | . | . | . | . | . | . | Y | . | . | . | . | . | . | . | . | A | . | . | . | . | . | . | . |
| 21-225_25A4.001.003_HC | . | . | . | . | . | . | . | . | Y | . | . | . | . | . | . | . | . | V | . | . | . | . | . | . | . |
| 21-225_25A4.001.004_HC | . | . | . | . | . | . | . | . | Y | . | . | . | . | . | . | . | Q | . | . | . | . | . | . | . | . |
| 21-225_25A4.001.005_HC | . | . | . | . | . | . | . | . | Y | . | . | . | . | . | . | . | S | . | . | . | . | . | . | . | . |
| 21-225_25A4.001.006_HC | . | . | . | . | . | . | . | . | Y | . | . | . | . | . | . | . | . | A | . | . | . | . | . | . | . |
| 21-225_25A4.001.007_HC | . | . | . | . | . | . | . | . | Y | . | . | . | . | . | . | . | . | V | . | . | . | . | . | . | . |
| 21-225_25A4.001.008_HC | . | . | . | . | . | . | . | . | Y | . | . | . | . | . | . | . | Q | . | . | . | . | . | . | . | . |
| 21-225_25A4.001.013_HC | . | . | . | . | . | . | . | . | Y | . | . | . | . | . | . | . | S | . | . | . | . | . | . | . | . |
| 21-225_25A4.001.019_HC | . | . | . | . | . | . | . | . | Y | . | . | . | . | . | . | . | S | . | . | . | . | . | . | . | . |
| 21-225_25A4.001.020_HC | . | . | . | . | . | . | . | . | Y | . | . | . | . | . | . | . | Q | . | . | . | . | . | . | . | . |
| 21-225_25A4.001.021_HC | . | . | . | . | . | . | . | . | Y | . | . | . | . | . | . | . | . | A | . | . | . | . | . | . | . |
| 21-225_25A4.001.029_HC | . | . | . | . | . | . | . | . | Y | . | . | . | . | . | . | . | Q | . | . | . | . | . | . | . | . |
| 21-225_26C4_HC | . | . | . | . | . | . | . | . | N | . | . | . | . | . | . | . | . | . | . | . | . | . | R | . | . |
| 21-225_28B6_HC | . | . | . | . | . | . | . | . | . | . | D | . | . | . | . | . | . | . | . | . | . | . | . | . | . |
| 21-225_29E6_HC | . | . | . | . | . | . | . | . | . | . | . | . | . | . | . | . | . | . | . | . | . | . | . | . | . |
| 21-225_29H8_HC | . | . | . | . | . | . | . | . | N | . | . | . | . | . | . | . | . | . | . | . | . | . | R | . | . |
| 21-225_32A1_HC | . | . | . | . | . | . | . | . | N | . | . | . | . | . | . | . | . | . | . | . | . | . | . | . | . |
| 21-225_32A5_HC | . | . | . | . | . | . | . | . | . | . | . | . | . | . | . | . | . | . | . | F | . | . | . | . | R |
| 21-225_33D1_HC | . | . | . | . | . | . | . | . | . | . | . | . | . | . | . | . | . | . | . | F | . | . | . | . | R |
| 21-225_34D2_HC | . | . | . | . | V | . | . | L | . | . | . | . | . | . | . | . | T | . | . | F | . | . | . | . | R |
| 21-225_34H7_HC | . | . | . | . | . | . | . | . | N | . | . | . | . | . | . | . | . | . | . | . | . | . | . | . | . |

**FIGURE 57**

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | H_CDR2 | | | | | | | | | | | | | | | | | | |
| CONSENSUS | G | L | E | W | M | G | W | M | H | P | N | - | - | - | S | G | N | T | G | Y | A | Q | K | F | Q |
| 21-225_43D8_HC | . | . | . | . | . | . | . | . | . | . | . | . | . | . | . | . | . | . | . | . | . | . | . | . | . |
| 21-225_4A2_HC | . | . | . | . | . | . | . | . | . | . | . | . | . | . | . | . | . | . | . | . | . | . | . | . | . |
| 21-225_4A2.001_HC | . | . | . | . | . | . | . | . | . | . | . | . | . | . | . | . | . | . | . | . | . | . | . | . | . |
| 21-225_4A2.001.001_HC | . | . | . | . | . | . | . | . | . | . | . | . | . | . | . | . | . | . | . | . | . | . | . | . | . |
| 21-225_4A2.001.002_HC | . | . | . | . | . | . | . | . | . | . | . | . | . | . | . | . | . | . | . | . | . | . | . | . | . |
| 21-225_4A2.001.003_HC | . | . | . | . | . | . | . | . | . | . | . | . | . | . | . | . | . | . | . | . | . | . | . | . | . |
| 21-225_4A2.001.004_HC | . | . | . | . | . | . | . | . | . | . | . | . | . | . | . | . | . | A | . | . | . | . | . | . | . |
| 21-225_4A2.001.005_HC | . | . | . | . | . | . | . | . | . | . | . | . | . | . | . | . | S | . | . | . | . | . | . | . | . |
| 21-225_4A2.001.006_HC | . | . | . | . | . | . | . | . | . | . | . | . | . | . | . | . | Q | . | . | . | . | . | . | . | . |
| 21-225_4A2.001.007_HC | . | . | . | . | . | . | . | . | . | . | . | . | . | . | . | . | Q | . | . | . | . | . | . | . | . |
| 21-225_4A2.001.008_HC | . | . | . | . | . | . | . | . | . | . | . | . | . | . | . | . | . | A | . | . | . | . | . | . | . |
| 21-225_4A2.001.009_HC | . | . | . | . | . | . | . | . | . | . | . | . | . | . | . | . | Q | . | . | . | . | . | . | . | . |
| 21-225_4A2.001.010_HC | . | . | . | . | . | . | . | . | . | . | . | . | . | . | . | . | . | A | . | . | . | . | . | . | . |
| 21-225_4A2.001.011_HC | . | . | . | . | . | . | . | . | . | . | . | . | . | . | . | . | Q | . | . | . | . | . | . | . | . |
| 21-225_4A2.001.012_HC | . | . | . | . | . | . | . | . | . | . | . | . | . | . | . | . | S | . | . | . | . | . | . | . | . |
| 21-225_4A2.001.022_HC | . | . | . | . | . | . | . | . | . | . | . | . | . | . | . | . | . | . | . | . | . | . | . | . | . |
| 21-225_4A2.001.023_HC | . | . | . | . | . | . | . | . | . | . | . | . | . | . | . | . | . | . | . | . | . | . | . | . | . |
| 21-225_4A2.001.024_HC | . | . | . | . | . | . | . | . | . | . | . | . | . | . | . | . | Q | . | . | . | . | . | . | . | . |
| 21-225_4A2.001.028_HC | . | . | . | . | . | . | . | . | . | . | . | . | . | . | . | . | Q | . | . | . | . | . | . | . | . |
| 21-225_4A2.001.029_HC | . | . | . | . | . | . | . | . | . | . | . | . | . | . | . | . | S | . | . | . | . | . | . | . | . |
| 21-225_4A2.001.031_HC | . | . | . | . | . | . | . | . | . | . | . | . | . | . | . | . | . | E | . | . | . | . | . | . | . |
| 21-225_56A1_HC | . | . | . | . | L | . | . | . | . | . | . | . | . | . | . | . | . | . | . | . | . | . | . | . | . |
| 21-225_58B9_HC | . | . | . | . | . | . | . | . | N | . | . | . | . | . | . | . | S | . | . | . | . | . | . | . | . |
| 21-225_59E1_HC | . | . | . | . | . | . | . | . | T | . | . | . | . | . | . | . | . | . | . | . | . | . | . | . | . |
| 21-225_59F10_HC | . | . | . | . | . | . | . | . | N | . | . | . | . | . | . | . | . | . | . | . | . | . | . | . | . |
| 21-225_60F3_HC | . | . | . | . | . | . | . | . | . | . | . | . | . | . | . | . | . | . | . | . | . | . | . | . | . |
| 21-225_61B5_HC | . | . | . | . | . | . | . | . | . | . | . | . | . | . | . | . | S | . | . | . | . | . | . | . | . |
| 21-225_61E3_HC | . | . | . | . | . | . | . | . | T | . | . | . | . | . | . | . | . | . | . | . | . | . | . | . | . |
| 21-225_63F4_HC | . | . | . | . | . | . | . | . | . | . | . | . | . | . | . | . | . | . | . | . | . | . | . | . | R |

# FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | H_CDR2 | | | | | | | | | | | | | | | | | | |
| CONSENSUS | G | L | E | W | M | G | W | M | H | P | N | - | - | - | S | G | N | T | G | Y | A | Q | K | F | Q |
| 21-225_6G6_HC | . | P | . | . | . | . | . | . | . | . | . | . | . | . | . | . | . | . | . | . | . | K | . | . | . |
| 21-225_70E7_HC | . | . | . | . | . | . | . | . | . | . | . | . | . | . | . | . | . | . | . | . | . | . | . | . | . |
| 21-225_71F3_HC | . | . | . | . | . | . | . | . | . | . | . | . | . | . | . | . | . | . | . | . | . | . | . | . | . |
| 21-225_71G3_HC | . | . | . | . | . | . | . | . | . | . | . | . | . | . | . | . | S | . | . | . | . | . | . | . | . |
| 21-225_72C4_HC | . | . | . | . | . | . | . | . | . | . | . | . | . | . | . | . | . | . | . | . | . | . | . | . | . |
| 21-225_74A6_HC | . | . | . | . | . | . | . | . | . | . | . | . | . | . | . | . | . | . | . | . | . | . | . | . | R |
| 21-225_74A8_HC | . | . | . | . | . | . | . | . | . | . | . | . | . | . | . | . | . | . | . | . | . | . | . | . | . |
| 21-225_74B11_HC | . | . | . | . | . | . | . | . | . | . | . | . | . | . | . | . | . | . | . | . | . | . | . | . | . |
| 21-225_74F6_HC | . | . | . | . | . | . | . | . | . | . | . | . | . | . | . | . | . | . | . | . | . | . | . | . | . |
| 21-225_74F9_HC | . | . | . | . | . | . | . | . | N | . | . | . | . | . | . | . | . | . | . | C | . | . | . | . | . |
| 21-225_74G4_HC | . | . | . | . | . | . | . | . | . | . | . | . | . | . | . | . | . | . | . | . | . | . | . | . | . |
| 21-225_75A10_HC | . | . | . | . | . | . | . | . | N | . | . | . | . | . | . | . | . | . | . | . | . | . | . | . | . |
| 21-225_75A6_HC | . | . | . | . | . | . | . | . | N | . | . | . | . | . | . | . | . | . | . | . | . | . | . | . | . |
| 21-225_75C4_HC | . | . | . | . | . | . | . | . | . | . | . | . | . | . | . | . | . | . | . | . | . | . | . | . | . |
| 21-225_75F9_HC | . | . | . | . | . | . | . | . | . | . | . | . | . | . | . | . | . | . | . | . | . | . | . | . | . |
| 21-225_76E11_HC | . | . | . | . | . | . | . | . | N | . | . | . | . | . | . | . | . | . | . | . | . | . | . | . | . |
| 21-225_76G2_HC | . | . | . | . | . | . | . | . | N | . | . | . | . | . | . | . | . | . | . | . | . | . | . | . | . |
| 21-225_78E11_HC | . | . | . | . | . | . | . | . | N | . | . | . | . | . | . | . | . | . | . | C | . | . | . | . | . |
| 21-225_78E6_HC | . | . | . | . | . | . | . | . | . | . | . | . | . | . | . | . | . | . | . | . | . | . | . | . | . |
| 21-225_79E9_HC | . | . | . | . | . | . | . | . | . | . | . | . | . | . | . | . | . | . | . | . | . | . | . | . | . |
| 21-225_79F12_HC | . | . | . | . | . | . | . | . | N | . | . | . | . | . | . | . | . | . | . | . | . | . | . | . | . |
| 21-225_79G7_HC | . | . | . | . | . | . | . | . | . | . | . | . | . | . | . | . | . | . | . | . | . | . | . | . | . |
| 21-225_80B12_HC | . | . | . | . | . | . | . | . | N | . | . | . | . | . | . | . | . | . | . | . | . | . | . | . | . |
| 21-225_85C11_HC | . | . | . | . | . | . | . | . | . | . | . | . | . | . | . | . | . | . | . | . | . | . | . | . | . |
| 21-225_87E10_HC | . | . | . | . | . | . | . | . | N | . | . | . | . | . | . | . | . | . | . | . | . | . | . | . | . |
| 21-225_88A1_HC | . | . | . | . | . | . | . | . | N | . | . | . | . | . | . | . | . | . | . | . | . | . | . | . | . |
| 21-225_88B4_HC | . | . | . | . | . | . | . | . | N | . | . | . | . | . | . | . | D | . | . | . | . | . | . | . | . |
| 21-225_88C10_HC | . | . | . | . | . | . | . | . | T | . | . | . | . | . | . | . | . | . | . | . | . | . | . | . | . |
| 21-225_8B11_HC | . | . | . | . | . | . | . | . | . | . | . | . | . | . | . | . | . | . | . | . | . | . | . | . | . |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | H_CDR2 | | | | | | | | | | | | | | | | | | |
| CONSENSUS | G | L | E | W | M | G | W | M | H | P | N | - | - | - | S | G | N | T | G | Y | A | Q | K | F | Q |
| 21-225_92E6_HC | . | . | . | . | . | . | . | . | . | . | . | . | . | . | . | . | . | . | . | . | . | . | . | . | . |
| 21-225_94D8_HC | . | . | . | . | . | . | . | . | N | . | . | . | . | . | . | . | . | . | . | . | . | . | . | . | . |
| 21-225_96D10_HC | . | . | . | . | . | . | . | . | . | . | . | . | . | . | . | . | . | . | . | . | . | . | . | . | . |
| 21-225_96H5_HC | . | . | . | . | . | . | . | . | . | . | . | . | . | . | . | . | . | . | . | . | . | . | . | . | . |

FIGURE 57

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | H_FR3 | | | | | | | | | | | | | | | | | | | | | | | |
| CONSENSUS | G | R | V | T | M | T | R | N | T | S | I | S | T | A | Y | M | E | L | S | S | L | R | S | E | D |
| 21-225_11E5_HC | . | . | . | . | . | . | . | . | . | . | . | . | . | . | . | . | . | . | . | . | . | . | . | . | . |
| 21-225_12F11_HC | . | . | . | . | . | . | . | . | . | . | . | . | A | . | . | . | V | . | . | . | . | . | . | . | . |
| 21-225_12F12_HC | . | . | . | . | . | . | M | . | . | . | . | . | . | . | . | . | . | . | . | . | . | . | . | . | . |
| 21-225_146C9_HC | . | . | . | . | . | . | W | . | . | . | . | . | . | . | . | . | A | . | . | . | . | . | . | . | . |
| 21-225_146D4_HC | . | . | . | . | . | . | . | . | . | . | . | . | . | . | . | . | . | . | . | . | . | . | . | . | . |
| 21-225_146F9_HC | . | . | . | . | . | . | . | . | . | . | . | . | . | . | . | . | . | . | . | . | . | . | . | . | . |
| 21-225_147D5_HC | . | . | . | . | . | . | . | . | . | . | . | . | . | . | . | . | . | . | . | . | . | . | . | G | . |
| 21-225_147E4_HC | . | . | . | . | . | . | . | . | . | . | . | . | . | . | . | . | . | . | . | . | . | . | . | . | . |
| 21-225_148F8_HC | . | . | . | . | . | . | . | . | . | . | . | . | . | . | . | . | . | . | . | . | . | . | . | . | . |
| 21-225_148G3_HC | . | . | . | . | . | . | . | . | . | . | . | . | . | . | . | . | . | . | . | . | . | . | . | . | . |
| 21-225_149A2_HC | . | . | . | . | . | . | . | . | . | . | . | . | . | . | . | . | . | . | . | . | . | . | . | . | . |
| 21-225_149E3_HC | . | . | . | . | . | . | . | . | . | . | . | . | . | . | . | . | . | . | . | . | . | T | . | . | . |
| 21-225_149H4_HC | . | . | . | . | . | . | . | . | . | . | . | T | . | V | . | . | . | . | . | . | . | . | . | . | . |
| 21-225_150B7_HC | . | . | . | . | . | . | . | . | . | . | . | . | . | . | . | L | . | . | . | . | . | . | . | . | . |
| 21-225_150D2_HC | . | . | . | . | . | . | . | . | . | . | . | . | . | . | . | . | . | . | . | . | . | . | . | . | . |
| 21-225_152E12_HC | . | . | . | . | . | . | . | . | . | . | . | . | . | . | . | . | . | . | . | . | . | . | . | . | . |
| 21-225_152E3_HC | . | . | . | . | . | . | . | . | . | . | . | . | . | . | . | . | . | . | . | . | . | . | . | . | . |
| 21-225_152H3_HC | . | . | . | . | . | . | . | . | . | . | . | . | . | . | . | . | . | . | . | . | . | . | . | . | . |
| 21-225_153F11_HC | . | . | . | . | . | . | . | . | . | . | . | N | . | . | . | . | . | . | . | . | . | . | . | . | . |
| 21-225_154A11_HC | . | . | . | . | . | . | . | . | . | . | . | . | . | . | . | . | . | . | . | . | . | . | . | . | . |
| 21-225_154H6_HC | . | . | . | . | . | . | . | . | . | . | . | . | . | . | . | . | . | . | . | . | . | . | . | . | . |
| 21-225_155B6_HC | . | . | . | . | . | . | . | . | . | . | . | . | . | . | . | . | . | . | . | . | . | . | . | . | . |
| 21-225_155E5_HC | . | . | . | . | . | . | . | . | . | . | . | . | . | . | . | . | . | . | . | . | . | . | . | . | . |
| 21-225_156H1_HC | . | . | . | . | . | . | . | . | . | . | . | . | . | . | . | . | . | . | . | . | . | . | . | . | . |
| 21-225_158B12_HC | . | . | F | . | . | . | . | . | . | . | . | . | . | . | . | . | . | . | . | . | . | . | . | . | . |
| 21-225_160A4_HC | . | . | . | . | . | . | . | . | . | . | . | . | . | . | . | . | . | . | . | . | . | . | . | . | . |
| 21-225_160A7_HC | . | . | . | . | . | . | . | . | . | . | . | N | . | . | . | . | . | . | . | . | . | . | . | . | . |
| 21-225_162D5_HC | . | . | . | . | . | . | . | . | . | . | . | D | . | . | . | . | . | . | . | . | . | S | . | . | . |
| 21-225_162F6_HC | . | . | F | . | . | . | . | . | . | . | . | . | . | . | . | . | . | . | . | . | . | . | . | . | . |

FIGURE 57

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | H_FR3 | | | | | | | | | | | | | | | | | | | | | | | |
| CONSENSUS | G | R | V | T | M | T | R | N | T | S | I | S | T | A | Y | M | E | L | S | S | L | R | S | E | D |
| 21-225_164A4_HC | . | . | . | . | . | . | . | . | . | . | . | . | . | . | . | . | . | . | . | . | . | . | . | . | . |
| 21-225_164A7_HC | . | . | . | . | . | . | . | . | . | . | T | . | . | . | . | . | . | . | . | . | . | . | . | . | . |
| 21-225_164B11_HC | . | . | . | . | . | . | . | . | . | . | M | . | . | . | . | . | . | . | . | . | . | . | . | . | . |
| 21-225_165H2_HC | . | . | . | . | . | . | . | . | . | . | N | . | . | . | . | . | D | . | . | . | . | . | . | . | . |
| 21-225_172E11_HC | . | . | . | . | . | . | . | . | . | . | . | . | . | . | . | . | . | . | . | . | . | . | . | . | . |
| 21-225_175D10_HC | . | . | . | . | . | . | . | . | . | . | . | . | . | V | . | . | . | . | . | . | . | . | . | . | . |
| 21-225_177B12_HC | . | . | . | . | . | . | . | . | . | . | . | . | . | . | . | . | . | . | . | . | . | . | . | . | . |
| 21-225_181E5_HC | . | . | . | . | . | . | . | . | . | . | . | . | . | . | . | . | . | . | . | . | . | . | . | . | . |
| 21-225_190D7_HC | . | . | . | . | . | . | . | . | . | . | . | . | . | . | . | . | . | . | . | . | . | . | . | . | . |
| 21-225_190H4_HC | . | . | . | . | . | . | . | . | . | . | . | . | . | . | . | . | . | . | . | . | . | . | . | . | . |
| 21-225_191D8_HC | . | . | . | . | . | . | . | . | . | . | . | . | . | . | . | . | . | . | . | . | . | . | . | . | . |
| 21-225_191G3_HC | . | . | . | . | . | . | . | . | . | . | . | . | . | . | . | . | . | . | . | . | . | . | . | . | . |
| 21-225_193G4_HC | . | . | . | . | . | . | . | . | . | . | . | R | . | . | . | . | . | . | N | . | . | . | . | . | . |
| 21-225_197C6_HC | . | . | . | . | . | . | . | . | . | . | . | . | . | . | . | . | . | . | . | . | . | . | . | . | . |
| 21-225_197H4_HC | . | . | . | . | . | . | . | . | . | . | . | . | . | . | . | . | . | . | . | . | . | . | . | . | . |
| 21-225_201F5_HC | . | . | . | . | . | . | . | . | . | . | . | . | . | . | . | . | . | . | . | . | . | . | . | . | . |
| 21-225_224C10_HC | . | . | . | . | . | . | . | . | . | . | . | . | . | . | . | . | . | . | . | . | . | . | . | . | . |
| 21-225_224D8_HC | . | . | . | . | . | . | . | . | . | . | . | . | . | . | . | . | . | . | . | . | . | . | . | . | . |
| 21-225_224F11_HC | . | . | . | . | . | . | . | . | . | . | . | . | . | . | . | . | . | . | . | . | . | . | . | . | . |
| 21-225_225B9_HC | . | . | . | . | . | . | . | . | . | . | . | N | . | . | . | . | . | . | N | . | . | . | . | . | . |
| 21-225_225F11_HC | . | . | . | . | . | . | . | . | . | . | . | N | . | . | . | . | . | . | . | . | . | . | . | . | . |
| 21-225_225F12_HC | . | . | . | . | . | A | . | . | . | . | . | . | . | . | . | . | . | . | . | . | . | . | . | . | . |
| 21-225_225F5_HC | . | . | . | . | . | . | . | . | . | . | . | . | . | . | . | . | . | . | . | . | . | . | . | . | . |
| 21-225_225H12_HC | . | . | . | . | . | . | . | . | . | . | . | . | . | . | . | . | . | . | . | . | . | . | . | . | . |
| 21-225_226A12_HC | . | . | . | . | . | . | . | . | . | . | . | . | . | . | . | . | . | . | . | . | . | . | . | . | . |
| 21-225_226D11_HC | . | . | . | . | . | . | . | . | . | . | . | . | . | . | . | . | . | . | . | . | . | . | . | . | . |
| 21-225_226D6_HC | . | . | . | . | . | . | . | . | . | . | . | . | . | . | . | . | . | . | . | . | . | . | . | . | . |
| 21-225_226F6_HC | . | . | . | . | . | . | . | . | . | . | . | N | . | . | . | . | . | . | . | . | . | . | . | . | . |
| 21-225_227C7_HC | . | . | . | . | . | . | . | . | . | . | . | . | . | . | . | . | . | . | . | . | . | . | . | . | . |

# FIGURE 57

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | H_FR3 | | | | | | | | | | | | | | | | | | | | | | | |
| CONSENSUS | G | R | V | T | M | T | R | N | T | S | I | S | T | A | Y | M | E | L | S | S | L | R | S | E | D |
| 21-225_227D11_HC | . | . | . | . | . | . | . | . | . | . | . | . | . | . | . | . | . | . | . | . | . | . | . | . | . |
| 21-225_227E6_HC | . | . | . | . | . | . | . | . | . | . | . | . | . | . | . | . | . | . | . | . | . | . | . | . | . |
| 21-225_22D2_HC | . | . | . | . | . | . | . | . | . | . | . | . | . | . | . | . | . | . | . | . | . | . | . | . | . |
| 21-225_23A12_HC | . | . | . | . | . | . | . | . | . | . | . | N | . | . | . | . | . | . | . | . | . | . | . | . | . |
| 21-225_24E1_HC | . | . | . | . | . | . | . | . | . | . | . | . | . | . | . | . | . | . | . | . | . | . | . | . | . |
| 21-225_25A4_HC | . | . | . | . | . | . | . | . | . | . | . | . | . | . | . | . | . | . | . | . | . | . | . | . | . |
| 21-225_25A4.001_HC | . | . | . | . | . | . | . | D | . | . | . | . | . | . | . | . | . | . | . | . | . | . | . | . | . |
| 21-225_25A4.001.001_HC | . | . | . | . | . | . | . | D | . | . | . | . | . | . | . | . | . | . | . | . | . | . | . | . | . |
| 21-225_25A4.001.002_HC | . | . | . | . | . | . | . | D | . | . | . | . | . | . | . | . | . | . | . | . | . | . | . | . | . |
| 21-225_25A4.001.003_HC | . | . | . | . | . | . | . | D | . | . | . | . | . | . | . | . | . | . | . | . | . | . | . | . | . |
| 21-225_25A4.001.004_HC | . | . | . | . | . | . | . | D | . | . | . | . | . | . | . | . | . | . | . | . | . | . | . | . | . |
| 21-225_25A4.001.005_HC | . | . | . | . | . | . | . | D | . | . | . | . | . | . | . | . | . | . | . | . | . | . | . | . | . |
| 21-225_25A4.001.006_HC | . | . | . | . | . | . | . | D | . | . | . | . | . | . | . | . | . | . | . | . | . | . | . | . | . |
| 21-225_25A4.001.007_HC | . | . | . | . | . | . | . | D | . | . | . | . | . | . | . | . | . | . | . | . | . | . | . | . | . |
| 21-225_25A4.001.008_HC | . | . | . | . | . | . | . | D | . | . | . | . | . | . | . | . | . | . | . | . | . | . | . | . | . |
| 21-225_25A4.001.013_HC | . | . | . | . | . | . | . | D | . | . | . | . | . | . | . | . | . | . | . | . | . | . | . | . | . |
| 21-225_25A4.001.019_HC | . | . | . | . | . | . | . | D | . | . | . | . | . | . | . | . | . | . | . | . | . | . | . | . | . |
| 21-225_25A4.001.020_HC | . | . | . | . | . | . | . | D | . | . | . | . | . | . | . | . | . | . | . | . | . | . | . | . | . |
| 21-225_25A4.001.021_HC | . | . | . | . | . | . | . | D | . | . | . | . | . | . | . | . | . | . | . | . | . | . | . | . | . |
| 21-225_25A4.001.029_HC | . | . | . | . | . | . | . | D | . | . | . | . | . | . | . | . | . | . | . | . | . | . | . | . | . |
| 21-225_26C4_HC | . | . | . | . | . | . | . | . | . | . | . | N | . | . | . | . | . | . | . | . | . | . | . | . | . |
| 21-225_28B6_HC | . | . | . | . | . | . | . | . | . | . | . | . | . | . | . | . | . | . | . | . | . | . | . | . | . |
| 21-225_29E6_HC | . | . | . | . | . | . | . | . | . | . | . | . | . | . | . | . | . | . | . | . | . | . | . | . | . |
| 21-225_29H8_HC | . | . | . | . | . | . | . | . | . | . | . | . | A | . | . | . | . | . | . | . | . | . | . | . | . |
| 21-225_32A1_HC | . | . | . | . | . | . | . | . | . | . | . | . | . | . | . | . | . | . | . | . | . | . | . | . | . |
| 21-225_32A5_HC | . | . | . | . | . | . | . | . | . | . | . | . | . | . | . | . | . | . | . | . | . | . | . | . | . |
| 21-225_33D1_HC | . | . | . | . | . | . | . | . | . | . | . | . | . | . | . | . | . | . | . | . | . | . | . | . | . |
| 21-225_34D2_HC | . | . | . | . | . | . | . | . | . | . | . | . | . | . | . | L | . | . | . | . | . | . | . | . | . |
| 21-225_34H7_HC | . | . | . | . | . | . | . | . | . | . | . | . | . | . | . | . | . | . | N | . | . | . | . | . | . |

**FIGURE 57**

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | H_FR3 | | | | | | | | | | | | | | | | | | | | | | | |
| CONSENSUS | G | R | V | T | M | T | R | N | T | S | I | S | T | A | Y | M | E | L | S | S | L | R | S | E | D |
| 21-225_43D8_HC | . | . | . | . | . | . | . | . | . | . | . | . | . | . | . | . | . | . | . | . | . | T | . | . | . |
| 21-225_4A2_HC | . | . | . | . | L | . | . | . | . | . | . | . | . | . | . | . | . | . | . | . | . | . | . | . | . |
| 21-225_4A2.001_HC | . | . | . | . | L | . | . | D | . | . | . | . | . | . | . | . | . | . | . | . | . | . | . | . | . |
| 21-225_4A2.001.001_HC | . | . | . | . | L | . | . | D | . | . | . | . | . | . | . | . | . | . | . | . | . | . | . | . | . |
| 21-225_4A2.001.002_HC | . | . | . | . | L | . | . | D | . | . | . | . | . | . | . | . | . | . | . | . | . | . | . | . | . |
| 21-225_4A2.001.003_HC | . | . | . | . | L | . | . | D | . | . | . | . | . | . | . | . | . | . | . | . | . | . | . | . | . |
| 21-225_4A2.001.004_HC | . | . | . | . | L | . | . | D | . | . | . | . | . | . | . | . | . | . | . | . | . | . | . | . | . |
| 21-225_4A2.001.005_HC | . | . | . | . | L | . | . | D | . | . | . | . | . | . | . | . | . | . | . | . | . | . | . | . | . |
| 21-225_4A2.001.006_HC | . | . | . | . | L | . | . | D | . | . | . | . | . | . | . | . | . | . | . | . | . | . | . | . | . |
| 21-225_4A2.001.007_HC | . | . | . | . | L | . | . | D | . | . | . | . | . | . | . | . | . | . | . | . | . | . | . | . | . |
| 21-225_4A2.001.008_HC | . | . | . | . | L | . | . | D | . | . | . | . | . | . | . | . | . | . | . | . | . | . | . | . | . |
| 21-225_4A2.001.009_HC | . | . | . | . | L | . | . | D | . | . | . | . | . | . | . | . | . | . | . | . | . | . | . | . | . |
| 21-225_4A2.001.010_HC | . | . | . | . | L | . | . | D | . | . | . | . | . | . | . | . | . | . | . | . | . | . | . | . | . |
| 21-225_4A2.001.011_HC | . | . | . | . | L | . | . | D | . | . | . | . | . | . | . | . | . | . | . | . | . | . | . | . | . |
| 21-225_4A2.001.012_HC | . | . | . | . | L | . | . | D | . | . | . | . | . | . | . | . | . | . | . | . | . | . | . | . | . |
| 21-225_4A2.001.022_HC | . | . | . | . | L | . | . | D | . | . | . | . | . | . | . | . | . | . | . | . | . | . | . | . | . |
| 21-225_4A2.001.023_HC | . | . | . | . | L | . | . | D | . | . | . | . | . | . | . | . | . | . | . | . | . | . | . | . | . |
| 21-225_4A2.001.024_HC | . | . | . | . | L | . | . | D | . | . | . | . | . | . | . | . | . | . | . | . | . | . | . | . | . |
| 21-225_4A2.001.028_HC | . | . | . | . | L | . | . | D | . | . | . | . | . | . | . | . | . | . | . | . | . | . | . | . | . |
| 21-225_4A2.001.029_HC | . | . | . | . | L | . | . | D | . | . | . | . | . | . | . | . | . | . | . | . | . | . | . | . | . |
| 21-225_4A2.001.031_HC | . | . | . | . | L | . | . | D | . | . | . | . | . | . | . | . | . | . | . | . | . | . | . | . | . |
| 21-225_56A1_HC | . | . | . | . | . | . | . | . | . | . | . | . | . | . | . | . | . | . | . | . | . | . | . | . | . |
| 21-225_58B9_HC | . | . | . | . | . | . | . | . | . | . | . | . | . | . | . | . | . | . | . | . | . | . | . | . | . |
| 21-225_59E1_HC | . | . | . | . | . | . | . | T | . | . | . | . | . | . | . | . | . | . | . | . | . | . | . | . | . |
| 21-225_59F10_HC | . | . | . | . | . | . | . | . | . | . | . | . | . | . | . | . | . | . | . | . | . | . | . | . | . |
| 21-225_60F3_HC | . | . | . | . | . | . | . | . | . | . | . | . | . | . | . | . | . | . | . | . | . | . | . | . | . |
| 21-225_61B5_HC | . | . | . | . | . | . | . | . | . | . | . | . | . | . | . | . | . | . | . | . | . | . | . | . | . |
| 21-225_61E3_HC | . | . | . | . | . | . | . | . | . | . | . | . | . | . | . | . | . | . | . | . | . | K | . | . | . |
| 21-225_63F4_HC | . | . | . | . | . | . | . | . | . | . | L | . | . | V | . | . | . | . | . | . | . | . | . | . | . |

# FIGURE 57

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | H_FR3 | | | | | | | | | | | | | | | | | | | | | | | |
| CONSENSUS | G | R | V | T | M | T | R | N | T | S | I | S | T | A | Y | M | E | L | S | S | L | R | S | E | D |
| 21-225_6G6_HC | . | . | . | . | . | . | . | . | . | . | . | . | A | . | . | . | V | . | . | . | . | . | . | . | . |
| 21-225_70E7_HC | . | . | . | . | . | . | . | . | . | . | . | . | . | . | . | . | . | . | . | . | . | . | . | . | . |
| 21-225_71F3_HC | . | . | . | . | . | . | . | . | . | . | . | . | . | . | . | . | . | . | . | . | . | . | . | . | . |
| 21-225_71G3_HC | . | . | . | . | . | . | . | D | . | . | V | . | . | . | . | . | . | . | . | . | . | . | . | . | . |
| 21-225_72C4_HC | . | . | . | . | . | . | . | . | . | . | . | . | . | . | . | . | . | . | N | . | . | . | . | . | . |
| 21-225_74A6_HC | . | . | . | . | . | . | W | . | . | . | . | . | . | . | . | . | . | . | . | . | . | . | . | . | . |
| 21-225_74A8_HC | . | . | . | . | . | . | . | . | . | . | . | . | . | . | . | . | . | . | . | . | . | . | . | . | . |
| 21-225_74B11_HC | . | . | . | . | . | . | . | . | . | . | . | . | . | . | . | . | . | . | . | . | . | . | . | . | . |
| 21-225_74F6_HC | . | . | . | . | . | . | . | . | . | . | M | . | . | . | . | . | . | . | . | . | . | . | . | . | . |
| 21-225_74F9_HC | . | . | . | . | . | . | . | . | . | . | . | . | . | . | . | . | . | . | . | . | . | . | . | . | . |
| 21-225_74G4_HC | . | . | . | . | . | . | . | . | . | . | . | R | . | . | . | . | . | . | . | . | . | . | . | . | . |
| 21-225_75A10_HC | . | . | . | . | . | . | . | . | . | . | . | . | . | . | . | . | . | . | . | . | . | . | . | . | . |
| 21-225_75A6_HC | . | . | . | . | . | . | . | . | . | . | . | . | . | . | . | . | . | . | . | . | . | . | . | . | . |
| 21-225_75C4_HC | . | . | . | . | . | . | . | . | . | . | . | . | . | . | . | . | . | . | . | . | . | . | . | . | . |
| 21-225_75F9_HC | . | . | . | . | . | . | . | . | . | . | . | . | . | . | . | . | . | . | . | . | . | . | . | . | . |
| 21-225_76E11_HC | . | . | L | . | . | . | . | . | . | . | . | . | . | . | . | . | . | . | . | . | . | . | . | . | . |
| 21-225_76G2_HC | . | . | . | . | . | . | . | . | . | . | . | . | . | . | . | . | . | . | . | . | . | . | . | . | . |
| 21-225_78E11_HC | . | . | . | . | . | . | . | . | . | . | . | . | . | . | . | . | . | . | . | . | . | . | . | . | . |
| 21-225_78E6_HC | . | . | . | . | . | . | . | . | . | . | . | . | . | . | . | . | . | . | . | . | . | . | . | . | . |
| 21-225_79E9_HC | . | . | . | . | . | . | . | . | . | . | . | . | . | . | . | . | . | . | . | . | . | . | . | . | . |
| 21-225_79F12_HC | . | . | . | . | . | . | . | . | . | . | . | . | . | . | . | . | . | . | . | . | . | . | . | . | . |
| 21-225_79G7_HC | . | . | . | . | . | . | . | . | . | . | . | . | . | . | . | . | . | . | . | . | . | . | . | . | . |
| 21-225_80B12_HC | V | . | . | . | . | . | . | . | . | . | . | . | . | . | . | . | . | . | . | . | . | . | . | . | . |
| 21-225_85C11_HC | . | . | . | . | . | . | . | . | . | . | . | . | . | . | . | . | . | . | . | . | . | . | . | . | . |
| 21-225_87E10_HC | . | . | . | . | . | . | . | . | . | . | . | . | . | . | . | . | . | . | . | . | . | . | . | . | . |
| 21-225_88A1_HC | . | . | . | . | . | . | . | . | . | . | . | . | . | . | . | . | . | . | . | . | . | . | . | . | . |
| 21-225_88B4_HC | . | S | . | . | . | . | . | . | . | . | . | T | . | . | . | . | . | . | . | . | . | . | . | . | . |
| 21-225_88C10_HC | . | . | . | . | . | . | . | . | . | . | . | . | . | . | . | . | . | . | . | . | . | . | . | . | . |
| 21-225_8B11_HC | . | . | . | . | . | . | . | . | . | . | R | . | . | . | . | . | . | . | . | . | . | . | . | . | . |

**FIGURE 57**

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | H_FR3 | | | | | | | | | | | | | | | | | | | | | | | |
| CONSENSUS | G | R | V | T | M | T | R | N | T | S | I | S | T | A | Y | M | E | L | S | S | L | R | S | E | D |
| 21-225_92E6_HC | . | . | . | . | . | . | . | . | . | . | M | . | . | . | . | . | . | . | . | . | . | . | . | . | . |
| 21-225_94D8_HC | V | . | . | . | . | . | . | . | . | . | . | . | . | . | . | . | . | V | . | . | . | . | . | . | . |
| 21-225_96D10_HC | . | . | . | . | . | . | . | . | . | . | M | . | . | . | . | . | . | . | . | . | . | . | . | . | . |
| 21-225_96H5_HC | . | . | . | . | . | . | . | . | . | . | . | . | . | . | . | . | . | . | . | . | . | . | . | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | S | S | S | G | W | Y | - | - | - | - | - | - | - | - | - | - | - | - |
| 21-225_11E5_HC | . | . | . | . | . | . | . | T | . | . | . | . | E | . | . | . | . | . | . | . | . | . | . | . | . |
| 21-225_12F11_HC | . | . | . | . | . | . | . | L | . | . | . | . | . | . | . | . | . | . | . | . | . | . | . | . | . |
| 21-225_12F12_HC | . | . | . | . | . | . | . | M | . | . | . | . | . | . | . | . | . | . | . | . | . | . | . | . | . |
| 21-225_146C9_HC | . | . | . | . | . | . | . | Y | . | . | . | . | . | . | . | . | . | . | . | . | . | . | . | . | . |
| 21-225_146D4_HC | . | . | . | . | . | . | . | G | . | . | . | . | . | . | . | . | . | . | . | . | . | . | . | . | . |
| 21-225_146F9_HC | . | . | . | . | . | . | . | . | . | . | . | . | . | . | . | . | . | . | . | . | . | . | . | . | . |
| 21-225_147D5_HC | . | . | . | . | . | . | . | G | . | . | . | . | . | . | . | . | . | . | . | . | . | . | . | . | . |
| 21-225_147E4_HC | . | . | . | . | . | . | . | . | . | . | . | . | . | . | . | . | . | . | . | . | . | . | . | . | . |
| 21-225_148F8_HC | . | . | . | . | . | . | . | . | . | . | . | . | . | . | . | . | . | . | . | . | . | . | . | . | . |
| 21-225_148G3_HC | . | . | . | . | . | . | . | . | . | . | . | . | . | . | . | . | . | . | . | . | . | . | . | . | . |
| 21-225_149A2_HC | . | . | . | . | . | . | . | G | . | . | . | . | . | . | . | . | . | . | . | . | . | . | . | . | . |
| 21-225_149E3_HC | . | . | . | . | . | . | . | Y | . | . | . | . | . | . | . | . | . | . | . | . | . | . | . | . | . |
| 21-225_149H4_HC | . | . | . | . | . | . | T | F | . | . | . | . | . | . | . | . | . | . | . | . | . | . | . | . | . |
| 21-225_150B7_HC | . | . | . | . | . | . | . | . | . | . | . | . | . | . | . | . | . | . | . | . | . | . | . | . | . |
| 21-225_150D2_HC | . | . | . | . | . | . | . | Y | . | . | . | . | . | . | . | . | . | . | . | . | . | . | . | . | . |
| 21-225_152E12_HC | . | . | . | . | . | . | . | Y | . | . | . | . | . | . | . | . | . | . | . | . | . | . | . | . | . |
| 21-225_152E3_HC | . | . | . | . | . | . | . | . | . | . | . | . | . | . | . | . | . | . | . | . | . | . | . | . | . |
| 21-225_152H3_HC | . | . | . | . | . | . | . | Y | . | . | . | . | . | . | . | . | . | . | . | . | . | . | . | . | . |
| 21-225_153F11_HC | . | . | . | . | . | . | . | Y | . | . | . | . | . | . | . | . | . | . | . | . | . | . | . | . | . |
| 21-225_154A11_HC | . | . | . | . | . | . | . | . | . | . | . | . | . | . | . | . | . | . | . | . | . | . | . | . | . |
| 21-225_154H6_HC | . | . | . | . | . | . | . | Y | . | . | . | . | . | . | . | . | . | . | . | . | . | . | . | . | . |
| 21-225_155B6_HC | . | . | . | . | . | . | . | Y | . | . | . | . | . | . | . | . | . | . | . | . | . | . | . | . | . |
| 21-225_155E5_HC | . | . | . | . | . | . | . | F | . | . | . | . | . | . | . | . | . | . | . | . | . | . | . | . | . |
| 21-225_156H1_HC | . | . | . | . | . | . | . | G | . | . | . | . | . | . | . | . | . | . | . | . | . | . | . | . | . |
| 21-225_158B12_HC | . | . | . | . | . | . | . | Y | . | . | . | . | . | . | . | . | . | . | . | . | . | . | . | . | . |
| 21-225_160A4_HC | . | . | . | . | . | . | . | Y | . | . | . | . | . | . | . | . | . | . | . | . | . | . | . | . | . |
| 21-225_160A7_HC | . | . | . | . | . | . | . | H | . | . | . | . | . | . | . | . | . | . | . | . | . | . | . | . | . |
| 21-225_162D5_HC | . | . | . | . | F | . | . | F | . | . | . | . | . | . | . | . | . | . | . | . | . | . | . | . | . |
| 21-225_162F6_HC | . | . | . | . | . | . | . | Y | . | . | . | . | . | . | . | . | . | . | . | . | . | . | . | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | S | S | S | G | W | Y | - | - | - | - | - | - | - | - | - | - | - | - |
| 21-225_164A4_HC | . | . | . | . | . | . | . | . | . | . | . | . | . | . | . | . | . | . | . | . | . | . | . | . | . |
| 21-225_164A7_HC | S | . | . | . | . | . | . | . | . | . | . | . | . | . | . | . | . | . | . | . | . | . | . | . | . |
| 21-225_164B11_HC | S | . | . | . | . | . | S | Y | . | . | . | . | . | . | . | . | . | . | . | . | . | . | . | . | . |
| 21-225_165H2_HC | . | . | . | . | . | . | . | Y | . | . | . | . | . | . | . | . | . | . | . | . | . | . | . | . | . |
| 21-225_172E11_HC | . | . | . | . | . | . | . | Y | . | . | . | . | . | . | . | . | . | . | . | . | . | . | . | . | . |
| 21-225_175D10_HC | . | . | . | . | . | . | . | Y | . | . | . | . | . | . | . | . | . | . | . | . | . | . | . | . | . |
| 21-225_177B12_HC | . | . | . | . | . | . | . | Y | . | . | . | . | . | . | . | . | . | . | . | . | . | . | . | . | . |
| 21-225_181E5_HC | . | . | . | . | . | . | . | . | . | . | . | . | . | . | . | . | . | . | . | . | . | . | . | . | . |
| 21-225_190D7_HC | . | . | . | . | . | . | . | . | . | . | . | . | . | . | . | . | . | . | . | . | . | . | . | . | . |
| 21-225_190H4_HC | . | . | . | . | . | . | . | H | . | . | . | . | . | . | . | . | . | . | . | . | . | . | . | . | . |
| 21-225_191D8_HC | . | . | . | . | . | . | . | H | . | . | . | . | . | . | . | . | . | . | . | . | . | . | . | . | . |
| 21-225_191G3_HC | . | . | . | . | . | . | . | H | . | . | . | . | . | . | . | . | . | . | . | . | . | . | . | . | . |
| 21-225_193G4_HC | . | . | . | . | . | . | . | . | . | . | . | . | . | . | . | . | . | . | . | . | . | . | . | . | . |
| 21-225_197C6_HC | . | . | . | . | . | . | . | H | . | . | . | . | . | . | . | . | . | . | . | . | . | . | . | . | . |
| 21-225_197H4_HC | . | . | . | . | . | . | . | H | . | . | . | . | . | . | . | . | . | . | . | . | . | . | . | . | . |
| 21-225_201F5_HC | . | . | . | . | . | . | . | Y | . | . | . | . | . | . | . | . | . | . | . | . | . | . | . | . | . |
| 21-225_224C10_HC | . | . | . | . | . | . | . | Y | . | . | . | . | . | . | . | . | . | . | . | . | . | . | . | . | . |
| 21-225_224D8_HC | . | . | . | . | . | . | . | Y | . | . | . | . | . | . | . | . | . | . | . | . | . | . | . | . | . |
| 21-225_224F11_HC | . | . | . | . | . | . | . | Y | . | . | . | . | . | . | . | . | . | . | . | . | . | . | . | . | . |
| 21-225_225B9_HC | . | . | . | . | . | . | . | Y | . | . | . | . | T | . | . | . | . | . | . | . | . | . | . | . | . |
| 21-225_225F11_HC | . | . | . | . | . | . | . | Y | . | . | . | . | . | . | . | . | . | . | . | . | . | . | . | . | . |
| 21-225_225F12_HC | . | . | . | . | . | . | . | Y | . | . | . | . | . | . | . | . | . | . | . | . | . | . | . | . | . |
| 21-225_225F5_HC | . | . | . | . | . | . | . | Y | . | . | . | . | . | . | . | . | . | . | . | . | . | . | . | . | . |
| 21-225_225H12_HC | . | . | . | . | . | . | . | Y | . | . | . | . | . | . | . | . | . | . | . | . | . | . | . | . | . |
| 21-225_226A12_HC | . | . | . | . | . | . | . | Y | . | . | . | . | . | . | . | . | . | . | . | . | . | . | . | . | . |
| 21-225_226D11_HC | . | . | . | . | . | . | . | Y | . | . | . | . | . | . | . | . | . | . | . | . | . | . | . | . | . |
| 21-225_226D6_HC | . | . | . | . | . | . | . | Y | . | . | . | . | . | . | . | . | . | . | . | . | . | . | . | . | . |
| 21-225_226F6_HC | . | . | . | . | . | . | . | Y | . | . | . | . | . | . | . | . | . | . | . | . | . | . | . | . | . |
| 21-225_227C7_HC | . | . | . | . | . | . | . | Y | . | . | . | . | . | . | . | . | . | . | . | . | . | . | . | . | . |

# FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | H_CDR3 | | | | | | | | | | | | | | | | |
| CONSENSUS | T | A | V | Y | Y | C | A | S | S | S | G | W | Y | - | - | - | - | - | - | - | - | - | - | - | - |
| 21-225_227D11_HC | . | . | . | . | . | . | . | Y | . | . | . | . | . | . | . | . | . | . | . | . | . | . | . | . | . |
| 21-225_227E6_HC | . | . | . | . | . | . | . | Y | . | . | . | . | . | . | . | . | . | . | . | . | . | . | . | . | . |
| 21-225_22D2_HC | . | . | . | . | . | . | . | Y | . | . | . | . | . | . | . | . | . | . | . | . | . | . | . | . | . |
| 21-225_23A12_HC | . | . | . | . | . | . | . | G | . | . | . | . | . | . | . | . | . | . | . | . | . | . | . | . | . |
| 21-225_24E1_HC | . | . | . | . | . | . | . | T | . | . | . | . | E | . | . | . | . | . | . | . | . | . | . | . | . |
| 21-225_25A4_HC | . | . | . | . | . | . | . | . | . | . | . | . | . | . | . | . | . | . | . | . | . | . | . | . | . |
| 21-225_25A4.001_HC | . | . | . | . | . | . | . | . | . | . | . | . | . | . | . | . | . | . | . | . | . | . | . | . | . |
| 21-225_25A4.001.001_HC | . | . | . | . | . | . | . | . | . | . | . | . | . | . | . | . | . | . | . | . | . | . | . | . | . |
| 21-225_25A4.001.002_HC | . | . | . | . | . | . | . | . | . | . | . | . | . | . | . | . | . | . | . | . | . | . | . | . | . |
| 21-225_25A4.001.003_HC | . | . | . | . | . | . | . | . | . | . | . | . | . | . | . | . | . | . | . | . | . | . | . | . | . |
| 21-225_25A4.001.004_HC | . | . | . | . | . | . | . | . | . | . | . | . | . | . | . | . | . | . | . | . | . | . | . | . | . |
| 21-225_25A4.001.005_HC | . | . | . | . | . | . | . | . | . | . | . | . | . | . | . | . | . | . | . | . | . | . | . | . | . |
| 21-225_25A4.001.006_HC | . | . | . | . | . | . | . | . | . | . | . | . | . | . | . | . | . | . | . | . | . | . | . | . | . |
| 21-225_25A4.001.007_HC | . | . | . | . | . | . | . | . | . | . | . | . | . | . | . | . | . | . | . | . | . | . | . | . | . |
| 21-225_25A4.001.008_HC | . | . | . | . | . | . | . | . | . | . | . | . | . | . | . | . | . | . | . | . | . | . | . | . | . |
| 21-225_25A4.001.013_HC | . | . | . | . | . | . | . | . | . | . | . | . | . | . | . | . | . | . | . | . | . | . | . | . | . |
| 21-225_25A4.001.019_HC | . | . | . | . | . | . | . | . | . | . | . | . | . | . | . | . | . | . | . | . | . | . | . | . | . |
| 21-225_25A4.001.020_HC | . | . | . | . | . | . | . | . | . | . | . | . | . | . | . | . | . | . | . | . | . | . | . | . | . |
| 21-225_25A4.001.021_HC | . | . | . | . | . | . | . | . | . | . | . | . | . | . | . | . | . | . | . | . | . | . | . | . | . |
| 21-225_25A4.001.029_HC | . | . | . | . | . | . | . | . | . | . | . | . | . | . | . | . | . | . | . | . | . | . | . | . | . |
| 21-225_26C4_HC | . | . | . | . | . | . | . | . | . | . | . | . | . | . | . | . | . | . | . | . | . | . | . | . | . |
| 21-225_28B6_HC | . | . | . | . | . | . | . | . | . | . | . | . | . | . | . | . | . | . | . | . | . | . | . | . | . |
| 21-225_29E6_HC | . | . | . | . | . | . | . | . | . | . | . | . | . | . | . | . | . | . | . | . | . | . | . | . | . |
| 21-225_29H8_HC | . | . | . | . | . | . | . | . | . | . | . | . | . | . | . | . | . | . | . | . | . | . | . | . | . |
| 21-225_32A1_HC | . | . | . | . | . | . | . | . | . | . | . | . | . | . | . | . | . | . | . | . | . | . | . | . | . |
| 21-225_32A5_HC | . | . | . | . | . | . | S | . | . | . | . | . | . | . | . | . | . | . | . | . | . | . | . | . | . |
| 21-225_33D1_HC | . | . | . | . | . | . | . | . | . | . | . | . | . | . | . | . | . | . | . | . | . | . | . | . | . |
| 21-225_34D2_HC | . | . | . | . | . | . | . | . | . | . | . | . | . | . | . | . | . | . | . | . | . | . | . | . | . |
| 21-225_34H7_HC | . | . | . | . | . | . | . | . | . | . | . | . | . | . | . | . | . | . | . | . | . | . | . | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | S | S | S | G | W | Y | - | - | - | - | - | - | - | - | - | - | - | - |
| 21-225_6G6_HC | . | . | . | . | . | . | . | L | . | . | . | . | . | . | . | . | . | . | . | . | . | . | . | . | . |
| 21-225_70E7_HC | . | . | . | . | . | . | . | Y | . | . | . | . | . | . | . | . | . | . | . | . | . | . | . | . | . |
| 21-225_71F3_HC | . | . | . | . | . | . | . | Y | . | . | . | . | . | . | . | . | . | . | . | . | . | . | . | . | . |
| 21-225_71G3_HC | . | . | . | . | . | . | . | Y | . | . | . | . | . | . | . | . | . | . | . | . | . | . | . | . | . |
| 21-225_72C4_HC | . | . | . | . | . | . | S | H | . | . | . | . | . | . | . | . | . | . | . | . | . | . | . | . | . |
| 21-225_74A6_HC | . | . | . | . | . | . | . | . | . | . | . | . | . | . | . | . | . | . | . | . | . | . | . | . | . |
| 21-225_74A8_HC | S | . | . | . | . | . | . | Y | . | . | . | . | . | . | . | . | . | . | . | . | . | . | . | . | . |
| 21-225_74B11_HC | . | . | . | . | . | . | . | Y | . | . | . | . | . | . | . | . | . | . | . | . | . | . | . | . | . |
| 21-225_74F6_HC | . | . | . | . | . | . | . | H | . | . | . | . | . | . | . | . | . | . | . | . | . | . | . | . | . |
| 21-225_74F9_HC | . | . | . | . | . | . | . | . | . | . | . | . | N | . | . | . | . | . | . | . | . | . | . | . | . |
| 21-225_74G4_HC | . | . | . | . | . | . | . | . | . | . | . | . | . | . | . | . | . | . | . | . | . | . | . | . | . |
| 21-225_75A10_HC | . | . | . | . | . | . | . | Y | . | . | . | . | . | . | . | . | . | . | . | . | . | . | . | . | . |
| 21-225_75A6_HC | . | . | . | . | . | . | . | Y | . | . | . | . | . | . | . | . | . | . | . | . | . | . | . | . | . |
| 21-225_75C4_HC | . | . | . | . | . | . | . | . | . | . | . | . | . | . | . | . | . | . | . | . | . | . | . | . | . |
| 21-225_75F9_HC | . | . | . | . | . | . | . | Y | . | . | . | . | . | . | . | . | . | . | . | . | . | . | . | . | . |
| 21-225_76E11_HC | . | . | . | F | . | . | . | Y | . | . | . | . | . | . | . | . | . | . | . | . | . | . | . | . | . |
| 21-225_76G2_HC | . | . | . | . | . | . | . | G | . | . | . | . | . | . | . | . | . | . | . | . | . | . | . | . | . |
| 21-225_78E11_HC | . | . | . | . | . | . | . | . | . | . | . | . | N | . | . | . | . | . | . | . | . | . | . | . | . |
| 21-225_78E6_HC | S | . | . | . | . | . | . | Y | . | . | . | . | . | . | . | . | . | . | . | . | . | . | . | . | . |
| 21-225_79E9_HC | . | . | . | . | . | . | . | . | . | . | . | . | . | . | . | . | . | . | . | . | . | . | . | . | . |
| 21-225_79F12_HC | . | . | . | . | . | . | . | . | . | . | . | . | . | . | . | . | . | . | . | . | . | . | . | . | . |
| 21-225_79G7_HC | S | . | . | . | . | . | . | Y | . | . | . | . | . | . | . | . | . | . | . | . | . | . | . | . | . |
| 21-225_80B12_HC | . | . | . | . | . | . | . | Y | . | . | . | . | . | . | . | . | . | . | . | . | . | . | . | . | . |
| 21-225_85C11_HC | S | . | . | . | . | . | . | Y | . | . | . | . | . | . | . | . | . | . | . | . | . | . | . | . | . |
| 21-225_87E10_HC | . | . | . | . | . | . | . | Y | . | . | . | . | . | . | . | . | . | . | . | . | . | . | . | . | . |
| 21-225_88A1_HC | . | . | . | . | . | . | . | Y | . | . | . | . | . | . | . | . | . | . | . | . | . | . | . | . | . |
| 21-225_88B4_HC | . | . | . | . | . | . | S | Y | . | . | . | . | . | . | . | . | . | . | . | . | . | . | . | . | . |
| 21-225_88C10_HC | . | . | . | . | . | . | . | . | . | . | . | . | . | . | . | . | . | . | . | . | . | . | . | . | . |
| 21-225_8B11_HC | . | . | . | . | . | . | . | Y | . | . | . | . | . | . | . | . | . | . | . | . | . | . | . | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | H_CDR3 | | | | | | | | | | | | | | | | |
| CONSENSUS | T | A | V | Y | Y | C | A | S | S | S | G | W | Y | - | - | - | - | - | - | - | - | - | - | - | - |
| 21-225_92E6_HC | . | . | . | . | . | . | . | H | . | . | . | . | . | . | . | . | . | . | . | . | . | . | . | . | . |
| 21-225_94D8_HC | . | . | . | . | . | . | . | Y | . | . | . | . | . | . | . | . | . | . | . | . | . | . | . | . | . |
| 21-225_96D10_HC | . | . | . | . | . | . | . | H | . | . | . | . | . | . | . | . | . | . | . | . | . | . | . | . | . |
| 21-225_96H5_HC | S | . | . | . | . | . | . | Y | . | . | . | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | | | | | | H_FR4 | | | | | |
| CONSENSUS | - | - | - | - | - | - | - | - | - | Y | F | D | Y | W | G | Q | G | T | L | V | T | V | S | S |
| 21-225_11E5_HC | . | . | . | . | . | . | . | . | . | V | . | . | . | . | . | . | . | . | . | . | . | . | . | . |
| 21-225_12F11_HC | . | . | . | . | . | . | . | . | . | . | . | . | . | . | . | . | . | . | . | . | . | . | . | . |
| 21-225_12F12_HC | . | . | . | . | . | . | . | . | . | . | . | . | F | . | . | . | . | . | . | . | . | . | . | . |
| 21-225_146C9_HC | . | . | . | . | . | . | . | . | . | S | . | . | . | . | . | . | . | . | . | . | . | . | . | . |
| 21-225_146D4_HC | . | . | . | . | . | . | . | . | . | . | . | . | . | . | . | . | . | . | . | . | . | . | . | . |
| 21-225_146F9_HC | . | . | . | . | . | . | . | . | . | F | . | . | . | . | . | . | . | . | . | . | . | . | . | . |
| 21-225_147D5_HC | . | . | . | . | . | . | . | . | . | . | . | . | . | . | . | . | . | . | . | . | . | . | . | . |
| 21-225_147E4_HC | . | . | . | . | . | . | . | . | . | F | . | . | . | . | . | . | . | . | . | . | . | . | . | . |
| 21-225_148F8_HC | . | . | . | . | . | . | . | . | . | . | . | . | . | . | . | . | . | . | . | . | . | . | . | . |
| 21-225_148G3_HC | . | . | . | . | . | . | . | . | . | F | . | . | . | . | . | . | . | . | . | . | . | . | . | . |
| 21-225_149A2_HC | . | . | . | . | . | . | . | . | . | . | . | . | . | . | . | . | . | . | . | . | . | . | . | . |
| 21-225_149E3_HC | . | . | . | . | . | . | . | . | . | W | . | . | . | . | . | . | . | . | . | . | . | . | . | . |
| 21-225_149H4_HC | . | . | . | . | . | . | . | . | . | . | . | . | . | . | . | . | . | . | . | . | . | . | . | . |
| 21-225_150B7_HC | . | . | . | . | . | . | . | . | . | F | . | . | . | . | . | . | . | . | . | . | . | . | . | . |
| 21-225_150D2_HC | . | . | . | . | . | . | . | . | . | . | . | . | . | . | . | . | . | . | . | . | . | . | . | . |
| 21-225_152E12_HC | . | . | . | . | . | . | . | . | . | . | . | . | . | . | . | . | . | . | . | . | . | . | . | . |
| 21-225_152E3_HC | . | . | . | . | . | . | . | . | . | F | . | . | . | . | . | . | . | . | . | . | . | . | . | . |
| 21-225_152H3_HC | . | . | . | . | . | . | . | . | . | W | . | . | . | . | . | . | . | . | . | . | . | . | . | . |
| 21-225_153F11_HC | . | . | . | . | . | . | . | . | . | F | . | . | N | . | . | . | . | . | . | . | . | . | . | . |
| 21-225_154A11_HC | . | . | . | . | . | . | . | . | . | . | . | . | . | . | . | . | . | . | . | . | . | . | . | . |
| 21-225_154H6_HC | . | . | . | . | . | . | . | . | . | I | . | . | . | . | . | . | . | . | . | . | . | . | . | . |
| 21-225_155B6_HC | . | . | . | . | . | . | . | . | . | . | . | . | . | . | . | . | . | . | . | . | . | . | . | . |
| 21-225_155E5_HC | . | . | . | . | . | . | . | . | . | . | . | . | . | . | . | . | . | . | . | . | . | . | . | . |
| 21-225_156H1_HC | . | . | . | . | . | . | . | . | . | . | . | . | . | . | . | . | . | . | . | . | . | . | . | . |
| 21-225_158B12_HC | . | . | . | . | . | . | . | . | . | R | . | . | . | . | . | . | . | . | . | . | . | . | . | . |
| 21-225_160A4_HC | . | . | . | . | . | . | . | . | . | I | . | . | . | . | . | . | . | . | . | . | . | . | . | . |
| 21-225_160A7_HC | . | . | . | . | . | . | . | . | . | . | . | . | . | . | . | . | . | . | . | . | . | . | . | . |
| 21-225_162D5_HC | . | . | . | . | . | . | . | . | . | F | . | . | . | . | . | . | . | . | . | . | . | . | . | . |
| 21-225_162F6_HC | . | . | . | . | . | . | . | . | . | R | . | . | . | . | . | . | . | . | . | . | . | . | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | S | S | S | G | W | Y | - | - | - | - | - | - | - | - | - | - | - | - |
| 21-225_43D8_HC | . | . | . | . | . | . | . | H | . | . | . | . | T | . | . | . | . | . | . | . | . | . | . | . | . |
| 21-225_4A2_HC | . | . | . | . | . | . | . | . | . | . | . | . | . | . | . | . | . | . | . | . | . | . | . | . | . |
| 21-225_4A2.001_HC | . | . | . | . | . | . | . | . | . | . | . | . | . | . | . | . | . | . | . | . | . | . | . | . | . |
| 21-225_4A2.001.001_HC | . | . | . | . | . | . | . | . | . | . | . | . | . | . | . | . | . | . | . | . | . | . | . | . | . |
| 21-225_4A2.001.002_HC | . | . | . | . | . | . | . | . | . | . | . | . | . | . | . | . | . | . | . | . | . | . | . | . | . |
| 21-225_4A2.001.003_HC | . | . | . | . | . | . | . | . | . | . | . | . | . | . | . | . | . | . | . | . | . | . | . | . | . |
| 21-225_4A2.001.004_HC | . | . | . | . | . | . | . | . | . | . | . | . | . | . | . | . | . | . | . | . | . | . | . | . | . |
| 21-225_4A2.001.005_HC | . | . | . | . | . | . | . | . | . | . | . | . | . | . | . | . | . | . | . | . | . | . | . | . | . |
| 21-225_4A2.001.006_HC | . | . | . | . | . | . | . | . | . | . | . | . | . | . | . | . | . | . | . | . | . | . | . | . | . |
| 21-225_4A2.001.007_HC | . | . | . | . | . | . | . | . | . | . | . | . | . | . | . | . | . | . | . | . | . | . | . | . | . |
| 21-225_4A2.001.008_HC | . | . | . | . | . | . | . | . | . | . | . | . | . | . | . | . | . | . | . | . | . | . | . | . | . |
| 21-225_4A2.001.009_HC | . | . | . | . | . | . | . | . | . | . | . | . | . | . | . | . | . | . | . | . | . | . | . | . | . |
| 21-225_4A2.001.010_HC | . | . | . | . | . | . | . | . | . | . | . | . | . | . | . | . | . | . | . | . | . | . | . | . | . |
| 21-225_4A2.001.011_HC | . | . | . | . | . | . | . | . | . | . | . | . | . | . | . | . | . | . | . | . | . | . | . | . | . |
| 21-225_4A2.001.012_HC | . | . | . | . | . | . | . | . | . | . | . | . | . | . | . | . | . | . | . | . | . | . | . | . | . |
| 21-225_4A2.001.022_HC | . | . | . | . | . | . | . | . | . | . | . | . | . | . | . | . | . | . | . | . | . | . | . | . | . |
| 21-225_4A2.001.023_HC | . | . | . | . | . | . | . | . | . | . | . | . | . | . | . | . | . | . | . | . | . | . | . | . | . |
| 21-225_4A2.001.024_HC | . | . | . | . | . | . | . | . | . | . | . | . | . | . | . | . | . | . | . | . | . | . | . | . | . |
| 21-225_4A2.001.028_HC | . | . | . | . | . | . | . | . | . | . | . | . | . | . | . | . | . | . | . | . | . | . | . | . | . |
| 21-225_4A2.001.029_HC | . | . | . | . | . | . | . | . | . | . | . | . | . | . | . | . | . | . | . | . | . | . | . | . | . |
| 21-225_4A2.001.031_HC | . | . | . | . | . | . | . | . | . | . | . | . | . | . | . | . | . | . | . | . | . | . | . | . | . |
| 21-225_56A1_HC | . | . | . | . | . | . | . | Y | . | . | . | . | . | . | . | . | . | . | . | . | . | . | . | . | . |
| 21-225_58B9_HC | . | . | . | . | . | . | . | Y | . | . | . | . | . | . | . | . | . | . | . | . | . | . | . | . | . |
| 21-225_59E1_HC | . | . | . | . | . | . | . | F | . | . | . | . | . | . | . | . | . | . | . | . | . | . | . | . | . |
| 21-225_59F10_HC | . | . | . | . | N | . | . | V | . | . | . | . | . | . | . | . | . | . | . | . | . | . | . | . | . |
| 21-225_60F3_HC | . | . | . | . | . | . | . | Y | . | . | . | . | . | . | . | . | . | . | . | . | . | . | . | . | . |
| 21-225_61B5_HC | . | . | . | . | . | . | . | Y | . | . | . | . | . | . | . | . | . | . | . | . | . | . | . | . | . |
| 21-225_61E3_HC | . | . | . | . | . | . | . | Y | . | . | . | . | . | . | . | . | . | . | . | . | . | . | . | . | . |
| 21-225_63F4_HC | . | . | . | . | . | . | . | Y | . | . | . | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | H_FR4 | | | | | | | | | | |
| CONSENSUS | - | - | - | - | - | - | - | - | - | Y | F | D | Y | W | G | Q | G | T | L | V | T | V | S | S |
| 21-225_11E5_HC | . | . | . | . | . | . | . | . | . | V | . | . | . | . | . | . | . | . | . | . | . | . | . | . |
| 21-225_12F11_HC | . | . | . | . | . | . | . | . | . | . | . | . | . | . | . | . | . | . | . | . | . | . | . | . |
| 21-225_12F12_HC | . | . | . | . | . | . | . | . | . | . | . | . | F | . | . | . | . | . | . | . | . | . | . | . |
| 21-225_146C9_HC | . | . | . | . | . | . | . | . | . | S | . | . | . | . | . | . | . | . | . | . | . | . | . | . |
| 21-225_146D4_HC | . | . | . | . | . | . | . | . | . | . | . | . | . | . | . | . | . | . | . | . | . | . | . | . |
| 21-225_146F9_HC | . | . | . | . | . | . | . | . | . | F | . | . | . | . | . | . | . | . | . | . | . | . | . | . |
| 21-225_147D5_HC | . | . | . | . | . | . | . | . | . | . | . | . | . | . | . | . | . | . | . | . | . | . | . | . |
| 21-225_147E4_HC | . | . | . | . | . | . | . | . | . | F | . | . | . | . | . | . | . | . | . | . | . | . | . | . |
| 21-225_148F8_HC | . | . | . | . | . | . | . | . | . | . | . | . | . | . | . | . | . | . | . | . | . | . | . | . |
| 21-225_148G3_HC | . | . | . | . | . | . | . | . | . | F | . | . | . | . | . | . | . | . | . | . | . | . | . | . |
| 21-225_149A2_HC | . | . | . | . | . | . | . | . | . | . | . | . | . | . | . | . | . | . | . | . | . | . | . | . |
| 21-225_149E3_HC | . | . | . | . | . | . | . | . | . | W | . | . | . | . | . | . | . | . | . | . | . | . | . | . |
| 21-225_149H4_HC | . | . | . | . | . | . | . | . | . | . | . | . | . | . | . | . | . | . | . | . | . | . | . | . |
| 21-225_150B7_HC | . | . | . | . | . | . | . | . | . | F | . | . | . | . | . | . | . | . | . | . | . | . | . | . |
| 21-225_150D2_HC | . | . | . | . | . | . | . | . | . | . | . | . | . | . | . | . | . | . | . | . | . | . | . | . |
| 21-225_152E12_HC | . | . | . | . | . | . | . | . | . | . | . | . | . | . | . | . | . | . | . | . | . | . | . | . |
| 21-225_152E3_HC | . | . | . | . | . | . | . | . | . | F | . | . | . | . | . | . | . | . | . | . | . | . | . | . |
| 21-225_152H3_HC | . | . | . | . | . | . | . | . | . | W | . | . | . | . | . | . | . | . | . | . | . | . | . | . |
| 21-225_153F11_HC | . | . | . | . | . | . | . | . | . | F | . | . | N | . | . | . | . | . | . | . | . | . | . | . |
| 21-225_154A11_HC | . | . | . | . | . | . | . | . | . | . | . | . | . | . | . | . | . | . | . | . | . | . | . | . |
| 21-225_154H6_HC | . | . | . | . | . | . | . | . | . | I | . | . | . | . | . | . | . | . | . | . | . | . | . | . |
| 21-225_155B6_HC | . | . | . | . | . | . | . | . | . | . | . | . | . | . | . | . | . | . | . | . | . | . | . | . |
| 21-225_155E5_HC | . | . | . | . | . | . | . | . | . | . | . | . | . | . | . | . | . | . | . | . | . | . | . | . |
| 21-225_156H1_HC | . | . | . | . | . | . | . | . | . | . | . | . | . | . | . | . | . | . | . | . | . | . | . | . |
| 21-225_158B12_HC | . | . | . | . | . | . | . | . | . | R | . | . | . | . | . | . | . | . | . | . | . | . | . | . |
| 21-225_160A4_HC | . | . | . | . | . | . | . | . | . | I | . | . | . | . | . | . | . | . | . | . | . | . | . | . |
| 21-225_160A7_HC | . | . | . | . | . | . | . | . | . | . | . | . | . | . | . | . | . | . | . | . | . | . | . | . |
| 21-225_162D5_HC | . | . | . | . | . | . | . | . | . | F | . | . | . | . | . | . | . | . | . | . | . | . | . | . |
| 21-225_162F6_HC | . | . | . | . | . | . | . | . | . | R | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | H_FR4 | | | | | | | | | | |
| CONSENSUS | - | - | - | - | - | - | - | - | - | Y | F | D | Y | W | G | Q | G | T | L | V | T | V | S | S |
| 21-225_164A4_HC | . | . | . | . | . | . | . | . | . | F | . | . | . | . | . | . | . | . | . | . | . | . | . | . |
| 21-225_164A7_HC | . | . | . | . | . | . | . | . | . | L | . | . | . | . | . | . | . | . | . | . | . | . | . | . |
| 21-225_164B11_HC | . | . | . | . | . | . | . | . | . | M | . | . | . | . | . | . | . | . | . | . | . | . | . | . |
| 21-225_165H2_HC | . | . | . | . | . | . | . | . | . | M | . | . | . | . | . | . | . | . | . | . | . | . | . | . |
| 21-225_172E11_HC | . | . | . | . | . | . | . | . | . | R | . | . | . | . | . | . | . | . | . | . | . | . | . | . |
| 21-225_175D10_HC | . | . | . | . | . | . | . | . | . | . | . | . | . | . | . | . | . | . | . | . | . | . | . | . |
| 21-225_177B12_HC | . | . | . | . | . | . | . | . | . | . | . | . | F | . | . | . | . | . | . | . | . | . | . | . |
| 21-225_181E5_HC | . | . | . | . | . | . | . | . | . | I | . | . | . | . | . | . | . | . | . | . | . | . | . | . |
| 21-225_190D7_HC | . | . | . | . | . | . | . | . | . | F | . | . | . | . | . | . | . | . | . | . | . | . | . | . |
| 21-225_190H4_HC | . | . | . | . | . | . | . | . | . | I | . | . | . | . | . | . | . | . | . | . | . | . | . | . |
| 21-225_191D8_HC | . | . | . | . | . | . | . | . | . | . | . | . | . | . | . | . | . | . | . | . | . | . | . | . |
| 21-225_191G3_HC | . | . | . | . | . | . | . | . | . | I | . | . | . | . | . | . | . | . | . | . | . | . | . | . |
| 21-225_193G4_HC | . | . | . | . | . | . | . | . | . | F | . | . | . | . | . | . | . | . | . | . | . | . | . | . |
| 21-225_197C6_HC | . | . | . | . | . | . | . | . | . | . | . | . | . | . | . | . | . | . | . | . | . | . | . | . |
| 21-225_197H4_HC | . | . | . | . | . | . | . | . | . | . | . | . | . | . | . | . | . | . | . | . | . | . | . | . |
| 21-225_201F5_HC | . | . | . | . | . | . | . | . | . | . | . | . | . | . | . | . | . | . | . | . | . | . | . | . |
| 21-225_224C10_HC | . | . | . | . | . | . | . | . | . | K | . | . | . | . | . | . | . | . | . | . | . | . | . | . |
| 21-225_224D8_HC | . | . | . | . | . | . | . | . | . | . | . | . | . | . | . | . | . | . | . | . | I | . | . | . |
| 21-225_224F11_HC | . | . | . | . | . | . | . | . | . | K | . | . | . | . | . | . | . | . | . | . | . | . | . | . |
| 21-225_225B9_HC | . | . | . | . | . | . | . | . | . | L | . | . | . | . | . | . | . | . | . | . | . | . | . | . |
| 21-225_225F11_HC | . | . | . | . | . | . | . | . | . | R | . | . | . | . | . | . | . | . | . | . | . | . | . | . |
| 21-225_225F12_HC | . | . | . | . | . | . | . | . | . | K | . | . | . | . | . | . | . | . | . | . | . | . | . | . |
| 21-225_225F5_HC | . | . | . | . | . | . | . | . | . | R | . | . | . | . | . | . | . | . | . | . | . | . | . | . |
| 21-225_225H12_HC | . | . | . | . | . | . | . | . | . | K | . | . | . | . | . | . | . | . | . | . | . | . | . | . |
| 21-225_226A12_HC | . | . | . | . | . | . | . | . | . | . | . | . | . | . | . | . | . | . | . | . | . | . | . | . |
| 21-225_226D11_HC | . | . | . | . | . | . | . | . | . | . | . | . | . | . | . | . | . | . | . | . | . | . | . | . |
| 21-225_226D6_HC | . | . | . | . | . | . | . | . | . | K | . | . | . | . | . | . | . | . | . | . | . | . | . | . |
| 21-225_226F6_HC | . | . | . | . | . | . | . | . | . | R | . | . | . | . | . | . | . | . | . | . | . | . | . | . |
| 21-225_227C7_HC | . | . | . | . | . | . | . | . | . | R | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | H_FR4 | | | | | | | | | | |
| CONSENSUS | - | - | - | - | - | - | - | - | - | Y | F | D | Y | W | G | Q | G | T | L | V | T | V | S | S |
| 21-225_227D11_HC | . | . | . | . | . | . | . | . | . | . | . | . | . | . | . | . | . | . | . | . | . | . | . | . |
| 21-225_227E6_HC | . | . | . | . | . | . | . | . | . | K | . | . | . | . | . | . | . | . | . | . | . | . | . | . |
| 21-225_22D2_HC | . | . | . | . | . | . | . | . | . | . | . | . | . | . | . | . | . | . | . | . | . | . | . | . |
| 21-225_23A12_HC | . | . | . | . | . | . | . | . | . | . | . | . | . | . | . | . | . | . | . | . | . | . | . | . |
| 21-225_24E1_HC | . | . | . | . | . | . | . | . | . | V | . | . | . | . | . | . | . | . | . | . | . | . | . | . |
| 21-225_25A4_HC | . | . | . | . | . | . | . | . | . | . | . | . | . | . | . | . | . | . | . | . | . | . | . | . |
| 21-225_25A4.001_HC | . | . | . | . | . | . | . | . | . | . | . | . | . | . | . | . | . | . | . | . | . | . | . | . |
| 21-225_25A4.001.001_HC | . | . | . | . | . | . | . | . | . | . | . | . | . | . | . | . | . | . | . | . | . | . | . | . |
| 21-225_25A4.001.002_HC | . | . | . | . | . | . | . | . | . | . | . | . | . | . | . | . | . | . | . | . | . | . | . | . |
| 21-225_25A4.001.003_HC | . | . | . | . | . | . | . | . | . | . | . | . | . | . | . | . | . | . | . | . | . | . | . | . |
| 21-225_25A4.001.004_HC | . | . | . | . | . | . | . | . | . | . | . | . | . | . | . | . | . | . | . | . | . | . | . | . |
| 21-225_25A4.001.005_HC | . | . | . | . | . | . | . | . | . | . | . | . | . | . | . | . | . | . | . | . | . | . | . | . |
| 21-225_25A4.001.006_HC | . | . | . | . | . | . | . | . | . | . | . | . | . | . | . | . | . | . | . | . | . | . | . | . |
| 21-225_25A4.001.007_HC | . | . | . | . | . | . | . | . | . | . | . | . | . | . | . | . | . | . | . | . | . | . | . | . |
| 21-225_25A4.001.008_HC | . | . | . | . | . | . | . | . | . | . | . | . | . | . | . | . | . | . | . | . | . | . | . | . |
| 21-225_25A4.001.013_HC | . | . | . | . | . | . | . | . | . | . | . | . | . | . | . | . | . | . | . | . | . | . | . | . |
| 21-225_25A4.001.019_HC | . | . | . | . | . | . | . | . | . | . | . | . | . | . | . | . | . | . | . | . | . | . | . | . |
| 21-225_25A4.001.020_HC | . | . | . | . | . | . | . | . | . | . | . | . | . | . | . | . | . | . | . | . | . | . | . | . |
| 21-225_25A4.001.021_HC | . | . | . | . | . | . | . | . | . | . | . | . | . | . | . | . | . | . | . | . | . | . | . | . |
| 21-225_25A4.001.029_HC | . | . | . | . | . | . | . | . | . | . | . | . | . | . | . | . | . | . | . | . | . | . | . | . |
| 21-225_26C4_HC | . | . | . | . | . | . | . | . | . | . | . | . | . | . | . | . | . | . | . | . | . | . | . | . |
| 21-225_28B6_HC | . | . | . | . | . | . | . | . | . | . | . | . | . | . | . | . | . | . | . | . | . | . | . | . |
| 21-225_29E6_HC | . | . | . | . | . | . | . | . | . | . | . | . | . | . | . | . | . | . | . | . | . | . | . | . |
| 21-225_29H8_HC | . | . | . | . | . | . | . | . | . | . | . | . | . | . | . | . | . | . | . | . | . | . | . | . |
| 21-225_32A1_HC | . | . | . | . | . | . | . | . | . | F | . | . | . | . | . | . | . | . | . | . | . | . | . | . |
| 21-225_32A5_HC | . | . | . | . | . | . | . | . | . | F | . | . | . | . | . | . | . | . | . | . | . | . | . | . |
| 21-225_33D1_HC | . | . | . | . | . | . | . | . | . | F | . | . | . | . | . | . | . | . | . | . | . | . | . | . |
| 21-225_34D2_HC | . | . | . | . | . | . | . | . | . | F | . | . | . | . | . | . | . | . | . | . | . | . | . | . |
| 21-225_34H7_HC | . | . | . | . | . | . | . | . | . | F | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | H_FR4 | | | | | | | | | | |
| CONSENSUS | - | - | - | - | - | - | - | - | - | Y | F | D | Y | W | G | Q | G | T | L | V | T | V | S | S |
| 21-225_43D8_HC | . | . | . | . | . | . | . | . | . | L | . | . | . | . | . | . | . | . | . | . | . | . | . | A |
| 21-225_4A2_HC | . | . | . | . | . | . | . | . | . | . | . | . | . | . | . | . | . | . | . | . | . | . | . | . |
| 21-225_4A2.001_HC | . | . | . | . | . | . | . | . | . | . | . | . | . | . | . | . | . | . | . | . | . | . | . | . |
| 21-225_4A2.001.001_HC | . | . | . | . | . | . | . | . | . | . | . | . | . | . | . | . | . | . | . | . | . | . | . | . |
| 21-225_4A2.001.002_HC | . | . | . | . | . | . | . | . | . | . | . | . | . | . | . | . | . | . | . | . | . | . | . | . |
| 21-225_4A2.001.003_HC | . | . | . | . | . | . | . | . | . | . | . | . | . | . | . | . | . | . | . | . | . | . | . | . |
| 21-225_4A2.001.004_HC | . | . | . | . | . | . | . | . | . | . | . | . | . | . | . | . | . | . | . | . | . | . | . | . |
| 21-225_4A2.001.005_HC | . | . | . | . | . | . | . | . | . | . | . | . | . | . | . | . | . | . | . | . | . | . | . | . |
| 21-225_4A2.001.006_HC | . | . | . | . | . | . | . | . | . | . | . | . | . | . | . | . | . | . | . | . | . | . | . | . |
| 21-225_4A2.001.007_HC | . | . | . | . | . | . | . | . | . | . | . | . | . | . | . | . | . | . | . | . | . | . | . | . |
| 21-225_4A2.001.008_HC | . | . | . | . | . | . | . | . | . | . | . | . | . | . | . | . | . | . | . | . | . | . | . | . |
| 21-225_4A2.001.009_HC | . | . | . | . | . | . | . | . | . | . | . | . | . | . | . | . | . | . | . | . | . | . | . | . |
| 21-225_4A2.001.010_HC | . | . | . | . | . | . | . | . | . | . | . | . | . | . | . | . | . | . | . | . | . | . | . | . |
| 21-225_4A2.001.011_HC | . | . | . | . | . | . | . | . | . | . | . | . | . | . | . | . | . | . | . | . | . | . | . | . |
| 21-225_4A2.001.012_HC | . | . | . | . | . | . | . | . | . | . | . | . | . | . | . | . | . | . | . | . | . | . | . | . |
| 21-225_4A2.001.022_HC | . | . | . | . | . | . | . | . | . | . | . | . | . | . | . | . | . | . | . | . | . | . | . | . |
| 21-225_4A2.001.023_HC | . | . | . | . | . | . | . | . | . | . | . | . | . | . | . | . | . | . | . | . | . | . | . | . |
| 21-225_4A2.001.024_HC | . | . | . | . | . | . | . | . | . | . | . | . | . | . | . | . | . | . | . | . | . | . | . | . |
| 21-225_4A2.001.028_HC | . | . | . | . | . | . | . | . | . | . | . | . | . | . | . | . | . | . | . | . | . | . | . | . |
| 21-225_4A2.001.029_HC | . | . | . | . | . | . | . | . | . | . | . | . | . | . | . | . | . | . | . | . | . | . | . | . |
| 21-225_4A2.001.031_HC | . | . | . | . | . | . | . | . | . | . | . | . | . | . | . | . | . | . | . | . | . | . | . | . |
| 21-225_56A1_HC | . | . | . | . | . | . | . | . | . | V | . | . | . | . | . | . | . | . | . | . | . | . | . | . |
| 21-225_58B9_HC | . | . | . | . | . | . | . | . | . | . | . | . | . | . | . | . | . | . | . | . | . | . | . | . |
| 21-225_59E1_HC | . | . | . | . | . | . | . | . | . | F | . | . | . | . | . | . | . | . | . | . | . | . | . | . |
| 21-225_59F10_HC | . | . | . | . | . | . | . | . | . | . | . | . | . | . | . | . | . | . | . | . | . | . | . | . |
| 21-225_60F3_HC | . | . | . | . | . | . | . | . | . | F | . | . | . | . | . | . | . | . | . | . | . | . | . | . |
| 21-225_61B5_HC | . | . | . | . | . | . | . | . | . | . | . | . | . | . | . | . | . | . | . | . | . | . | . | . |
| 21-225_61E3_HC | . | . | . | . | . | . | . | . | . | R | . | . | . | . | . | . | . | . | . | . | . | . | . | . |
| 21-225_63F4_HC | . | . | . | . | . | . | . | . | . | V | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | H_FR4 | | | | | | | | | | |
| CONSENSUS | - | - | - | - | - | - | - | - | - | Y | F | D | Y | W | G | Q | G | T | L | V | T | V | S | S |
| 21-225_6G6_HC | . | . | . | . | . | . | . | . | . | . | . | . | . | . | . | . | . | . | . | . | . | . | . | . |
| 21-225_70E7_HC | . | . | . | . | . | . | . | . | . | V | . | . | . | . | . | . | . | . | . | . | . | . | . | . |
| 21-225_71F3_HC | . | . | . | . | . | . | . | . | . | V | . | . | S | . | . | . | . | . | . | . | . | . | . | . |
| 21-225_71G3_HC | . | . | . | . | . | . | . | . | . | . | . | . | . | . | . | . | . | . | . | . | . | . | . | . |
| 21-225_72C4_HC | . | . | . | . | . | . | . | . | . | L | . | . | . | . | . | . | . | . | . | . | . | . | . | . |
| 21-225_74A6_HC | . | . | . | . | . | . | . | . | . | . | . | . | . | . | . | . | . | . | . | . | . | . | . | . |
| 21-225_74A8_HC | . | . | . | . | . | . | . | . | . | K | . | . | . | . | S | . | . | . | . | . | . | . | . | . |
| 21-225_74B11_HC | . | . | . | . | . | . | . | . | . | . | . | . | . | . | . | . | . | . | . | . | . | . | . | . |
| 21-225_74F6_HC | . | . | . | . | . | . | . | . | . | F | . | . | . | . | . | . | . | . | . | . | . | . | . | . |
| 21-225_74F9_HC | . | . | . | . | . | . | . | . | . | F | . | . | . | . | . | . | . | . | . | . | . | . | . | . |
| 21-225_74G4_HC | . | . | . | . | . | . | . | . | . | H | . | . | . | . | . | . | . | . | . | . | . | . | . | . |
| 21-225_75A10_HC | . | . | . | . | . | . | . | . | . | . | . | . | . | . | . | . | . | . | . | . | . | . | . | . |
| 21-225_75A6_HC | . | . | . | . | . | . | . | . | . | I | . | . | . | . | . | . | . | . | . | . | . | . | . | . |
| 21-225_75C4_HC | . | . | . | . | . | . | . | . | . | . | . | . | . | . | . | . | . | . | . | . | . | . | . | . |
| 21-225_75F9_HC | . | . | . | . | . | . | . | . | . | I | . | . | . | . | . | . | . | . | . | . | . | . | . | . |
| 21-225_76E11_HC | . | . | . | . | . | . | . | . | . | . | . | . | . | . | . | . | . | . | . | . | . | . | . | . |
| 21-225_76G2_HC | . | . | . | . | . | . | . | . | . | M | . | . | . | . | . | . | . | . | . | . | . | . | . | . |
| 21-225_78E11_HC | . | . | . | . | . | . | . | . | . | F | . | . | . | . | . | . | . | . | . | . | . | . | . | . |
| 21-225_78E6_HC | . | . | . | . | . | . | . | . | . | K | . | . | . | . | S | . | . | . | . | . | . | . | . | . |
| 21-225_79E9_HC | . | . | . | . | . | . | . | . | . | . | . | . | . | . | . | . | . | . | . | . | . | . | . | . |
| 21-225_79F12_HC | . | . | . | . | . | . | . | . | . | F | . | . | . | . | . | . | . | . | . | . | . | L | . | . |
| 21-225_79G7_HC | . | . | . | . | . | . | . | . | . | K | . | . | . | . | S | . | . | . | . | . | . | . | . | . |
| 21-225_80B12_HC | . | . | . | . | . | . | . | . | . | I | . | . | . | . | . | . | . | . | . | . | . | . | . | . |
| 21-225_85C11_HC | . | . | . | . | . | . | . | . | . | K | . | . | . | . | S | . | . | . | . | . | . | . | . | . |
| 21-225_87E10_HC | . | . | . | . | . | . | . | . | . | F | . | . | . | . | . | . | . | . | . | . | . | . | . | . |
| 21-225_88A1_HC | . | . | . | . | . | . | . | . | . | . | . | . | . | . | . | . | . | . | . | . | . | . | . | . |
| 21-225_88B4_HC | . | . | . | . | . | . | . | . | . | . | . | . | . | . | . | . | . | . | . | . | . | . | . | . |
| 21-225_88C10_HC | . | . | . | . | . | . | . | . | . | . | . | . | S | . | . | . | . | . | . | . | . | . | . | L |
| 21-225_8B11_HC | . | . | . | . | . | . | . | . | . | F | . | . | . | . | . | . | . | . | . | . | . | . | . | . |

**FIGURE 57**

Table 50. Consensus 16- VH3|3-33/D6|6-6|RF1/JH6 (SEQ ID NO: 50267):

QVQLVES-GGGVVQPGRSLRLSCAASG-FTFSS-----YVMHWVRQAPGKGLEWVAVIWYD---GSNKYYADSVKGRFTISRDNSKNTLYLQMNSLRAEDTAVYYCARERYSSGW----------------YDYGMDVWGQGTTVTVSS wherein:

S at position 33 can be substituted with D, N or I

Y at position 39 can be substituted with C, F, D or S

V at position 40 can be substituted with G, I or L

M at position 41 can be substituted with I or L

H at position 42 can be substituted with D

V at position 57 can be substituted with L or A

W at position 59 can be substituted with F

G at position 65 can be substituted with A

S at position 66 can be substituted with R or N

N at position 67 can be substituted with Y, G or S

Y at position 69 can be substituted with H

Y at position 70 can be substituted with H or N

A at position 71 can be substituted with V, E, G or T

D at position 72 can be substituted with E or G

S at position 73 can be substituted with A

V at position 74 can be substituted with M

E at position 109 can be substituted with R or V

**FIGURE 57**

R at position 110 can be substituted with Y, K, V, E, P, D, L, F, M, N, Q or T

Y at position 111 can be substituted with S, T or V

S at position 112 can be substituted with R, Y, P, T or G

S at position 113 can be substituted with C or Y

G at position 114 can be substituted with W, S or N

W at position 115 can be substituted with null (-), L, Y, G, F or S

null (-) at position 116 can be substituted with Y

null (-) at position 117 can be substituted with A, G or T

null (-) at position 118 can be substituted with C

null (-) at position 129 can be substituted with P

null (-) at position 130 can be substituted with Y or L

null (-) at position 131 can be substituted with Y or D

Y at position 132 can be substituted with null (-), F or H

D at position 133 can be substituted with S, G, T, V, Y, A, F or M

Y at position 134 can be substituted with G or F

M at position 136 can be substituted with L

D at position 137 can be substituted with G

Xaa Xaa Xaa Xaa Xaa (SEQ ID NO: 50004)

Xaa Ile Xaa Tyr Asp Xaa Xaa Xaa Lys Xaa Xaa Xaa Xaa Xaa Xaa Lys Gly (SEQ ID NO: 50005)

**FIGURE 57**

Xaa Xaa Xaa Xaa Xaa Xaa Xaa Xaa Xaa Xaa Xaa Xaa Xaa Xaa Xaa Xaa Gly Xaa Xaa Val (SEQ ID NO: 50006)

Ser Tyr Val Met His (SEQ ID NO: 50471)

Val Ile Trp Tyr Asp Gly Ser Asn Lys Tyr Tyr Ala Asp Ser Val Lys Gly (SEQ ID NO: 50472)

Glu Arg Tyr Ser Ser Gly Trp Tyr Asp Tyr Gly Met Asp Val (SEQ ID NO: 50473)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | H_FR1 | | | | | | | | | | | | | | | | | | | | | | | | |
| CONSENSUS | Q | V | Q | L | V | E | S | - | G | G | G | V | V | Q | P | G | R | S | L | R | L | S | C | A | A |
| 21-225_10E9_HC | . | . | . | . | . | . | . | . | . | . | . | . | . | . | . | . | . | . | . | . | . | . | . | . | . |
| 21-225_147A8_HC | . | . | . | . | . | . | . | . | . | . | . | . | . | . | . | . | . | . | . | . | . | . | . | . | . |
| 21-225_147B2_HC | . | . | . | . | . | . | . | . | . | . | . | . | . | . | . | . | . | . | . | . | . | . | . | . | . |
| 21-225_147H5_HC | . | . | . | . | . | . | . | . | . | . | . | . | A | . | . | . | . | . | . | . | . | . | . | . | . |
| 21-225_148C9_HC | . | . | . | . | . | . | . | . | . | . | . | . | A | . | . | . | . | . | . | . | . | . | . | . | . |
| 21-225_148F5_HC | . | . | . | . | . | . | . | . | . | . | . | . | . | . | . | . | . | . | . | . | . | . | . | . | . |
| 21-225_148H10_HC | . | . | . | . | . | . | . | . | . | . | . | . | . | . | . | . | . | . | . | . | . | . | . | . | . |
| 21-225_149D10_HC | . | . | . | . | . | . | . | . | . | . | . | . | A | . | . | . | . | . | . | . | . | . | . | . | . |
| 21-225_14E10_HC | . | . | . | . | . | . | . | . | . | . | . | . | . | . | . | . | . | . | . | . | . | . | . | . | . |
| 21-225_150D11_HC | . | . | . | . | . | . | . | . | . | . | . | . | . | . | . | . | . | . | . | . | . | . | . | . | . |
| 21-225_150E2_HC | . | . | . | . | . | . | . | . | . | . | . | . | . | . | . | . | . | . | . | . | . | . | . | . | . |
| 21-225_152F7_HC | . | . | . | . | . | . | . | . | . | . | . | . | . | . | . | . | . | . | . | . | . | . | . | . | . |
| 21-225_153G9_HC | . | . | . | . | . | . | . | . | . | . | . | . | . | . | . | . | . | . | . | . | . | . | . | . | . |
| 21-225_154F4_HC | . | . | . | . | . | . | . | . | . | . | . | . | . | . | . | . | . | . | . | . | . | . | . | . | . |
| 21-225_159D8_HC | . | . | . | . | . | . | . | . | . | . | . | . | . | . | . | . | . | . | . | . | . | . | . | . | . |
| 21-225_15C11_HC | . | . | . | . | . | . | . | . | . | . | . | . | . | . | . | . | . | . | . | . | . | . | . | . | . |
| 21-225_169D11_HC | . | . | . | . | . | . | . | . | . | . | . | . | . | . | . | . | . | . | . | . | . | . | . | . | . |
| 21-225_170F3_HC | . | . | . | . | . | . | . | . | . | . | . | . | . | . | . | . | . | . | . | . | . | . | . | . | . |
| 21-225_173C11_HC | . | . | . | . | . | . | . | . | . | . | . | . | . | . | . | . | . | . | . | . | . | . | . | . | . |
| 21-225_174G10_HC | . | . | . | . | . | . | . | . | . | . | . | . | . | . | . | . | . | . | . | . | . | . | . | . | . |
| 21-225_176H12_HC | . | . | . | . | . | . | . | . | . | . | . | . | . | . | . | . | . | . | . | . | . | . | . | . | . |
| 21-225_177B4_HC | . | . | . | . | . | . | . | . | . | . | . | . | . | . | . | . | . | . | . | . | . | . | . | . | . |
| 21-225_179C2_HC | . | . | . | . | . | . | . | . | . | . | . | . | . | . | . | . | . | . | . | . | . | . | . | . | . |
| 21-225_17H3_HC | . | . | . | . | . | . | . | . | . | . | . | . | . | . | . | . | . | . | . | . | . | . | . | . | . |
| 21-225_183F4_HC | . | . | . | . | . | . | . | . | . | . | . | . | . | . | . | . | . | . | . | . | . | . | . | . | . |
| 21-225_190D6_HC | . | . | . | . | . | . | . | . | . | . | . | . | . | . | . | . | . | . | . | . | . | . | . | . | . |
| 21-225_192A2_HC | . | . | . | . | . | . | . | . | . | . | . | . | . | . | . | . | . | . | . | . | . | . | . | . | . |
| 21-225_192F6_HC | . | . | . | . | . | . | . | . | . | . | . | . | . | . | . | . | . | . | . | . | . | . | . | . | . |
| 21-225_193F8_HC | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | | | | | | | | | | | | | | | | H_FR1 | | | | | | | | | |
| CONSENSUS | Q | V | Q | L | V | E | S | - | G | G | G | V | V | Q | P | G | R | S | L | R | L | S | C | A | A |
| 21-225_194F10_HC | . | . | . | . | . | . | . | . | . | . | . | . | . | . | . | . | . | . | . | . | . | . | . | . | . |
| 21-225_201C5_HC | . | . | . | . | . | . | . | . | . | . | . | . | . | . | . | . | . | . | . | . | . | . | . | . | . |
| 21-225_203F7_HC | . | . | . | . | . | . | . | . | . | . | . | . | . | . | . | . | . | . | . | . | . | . | . | . | . |
| 21-225_204H3_HC | . | . | . | . | . | . | . | . | . | . | . | . | G | . | . | . | . | . | . | . | . | . | . | T | . |
| 21-225_208B2_HC | . | . | . | . | . | . | . | . | . | . | . | . | . | . | . | . | . | . | . | . | . | . | . | . | . |
| 21-225_208F1_HC | . | . | . | . | . | . | . | . | . | . | . | . | . | . | . | . | . | . | . | . | . | . | . | . | . |
| 21-225_20B2_HC | . | . | . | . | . | . | . | . | . | . | . | . | . | . | . | . | . | . | . | . | . | . | . | . | . |
| 21-225_210G5_HC | . | . | . | . | . | . | . | . | . | . | . | . | . | . | . | . | . | . | . | . | . | . | . | . | . |
| 21-225_210G6_HC | . | . | . | . | . | . | . | . | . | . | . | . | . | . | . | . | . | . | . | . | . | . | . | . | . |
| 21-225_212B11_HC | . | . | . | . | . | . | . | . | . | . | . | . | . | . | . | . | . | . | . | . | . | . | . | . | . |
| 21-225_214G10_HC | . | . | . | . | . | . | . | . | . | . | . | . | . | . | . | . | . | . | . | . | . | . | . | . | . |
| 21-225_214G12_HC | . | . | . | . | . | . | . | . | . | . | . | . | . | . | . | . | . | . | . | . | . | . | . | . | . |
| 21-225_214H9_HC | . | . | . | . | . | . | . | . | . | . | . | . | . | . | . | . | . | . | . | . | . | . | . | . | . |
| 21-225_215E11_HC | . | . | . | . | . | . | . | . | . | . | . | . | . | . | . | . | . | . | . | . | . | . | . | . | . |
| 21-225_215E3_HC | . | . | . | . | . | . | . | . | . | . | . | . | . | . | . | . | . | . | . | . | . | . | . | . | . |
| 21-225_216E8_HC | . | . | . | . | . | . | . | . | . | . | . | . | . | . | . | . | . | . | . | . | . | . | . | . | . |
| 21-225_216H12_HC | . | . | . | . | . | . | . | . | . | . | . | . | . | . | . | . | . | . | . | . | . | . | . | . | . |
| 21-225_217E5_HC | . | . | . | . | . | . | . | . | . | . | . | . | . | . | . | . | . | . | . | . | . | . | . | . | . |
| 21-225_217G10_HC | . | . | . | . | . | . | . | . | . | . | . | . | . | . | . | . | . | . | . | . | . | . | . | . | . |
| 21-225_217H12_HC | . | . | . | . | . | . | . | . | . | . | . | . | . | . | . | . | . | . | . | . | . | . | . | . | . |
| 21-225_218C4_HC | . | . | . | . | . | . | . | . | . | . | . | . | . | . | . | . | . | . | . | . | . | . | . | . | . |
| 21-225_220F8_HC | . | . | . | . | . | . | . | . | . | . | . | . | . | . | . | . | . | . | . | . | . | . | . | . | . |
| 21-225_224E7_HC | . | . | . | . | . | . | . | . | . | . | . | . | . | . | . | . | . | . | . | S | . | . | . | . | . |
| 21-225_224F1_HC | . | . | . | . | . | . | . | . | . | . | . | . | . | . | . | . | . | . | . | . | . | . | . | . | . |
| 21-225_224F3_HC | . | . | . | . | . | . | . | . | . | . | . | . | . | . | . | . | . | . | . | . | . | . | . | . | . |
| 21-225_225A1_HC | . | . | . | . | . | . | . | . | . | . | . | . | . | . | . | . | . | . | . | . | . | . | . | . | . |
| 21-225_226C6_HC | . | . | . | . | . | . | . | . | . | . | . | . | . | . | . | . | . | . | . | . | . | . | . | . | . |
| 21-225_226H11_HC | . | . | . | . | . | . | . | . | . | . | . | . | . | . | . | . | . | . | . | . | . | . | . | . | . |
| 21-225_22F4_HC | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | H_FR1 | | | | | | | | | | | | | | | | | | | | | | | | |
| CONSENSUS | Q | V | Q | L | V | E | S | - | G | G | G | V | V | Q | P | G | R | S | L | R | L | S | C | A | A |
| 21-225_23A10_HC | . | . | . | . | . | . | . | . | . | . | . | . | . | . | . | . | . | . | . | . | . | . | . | . | . |
| 21-225_24B6_HC | . | . | . | . | . | . | . | . | . | . | . | . | . | . | . | . | . | . | P | . | . | . | . | . | . |
| 21-225_25A2_HC | . | . | . | . | . | . | . | . | . | . | . | . | . | . | . | . | . | . | . | . | . | . | . | . | . |
| 21-225_25D12_HC | . | . | . | . | . | . | . | . | . | . | . | . | . | . | . | . | . | . | . | . | . | . | . | . | . |
| 21-225_26A11_HC | . | . | . | . | . | . | . | . | . | . | . | . | . | . | . | . | . | . | . | . | . | . | . | . | . |
| 21-225_26G7_HC | . | . | . | . | . | . | . | . | . | . | . | . | . | . | . | . | . | . | . | . | . | . | . | . | . |
| 21-225_27E7_HC | . | . | . | . | . | . | . | . | . | . | . | . | . | . | . | . | . | . | . | . | . | . | . | . | . |
| 21-225_28H2_HC | . | . | . | . | . | . | . | . | . | . | . | . | . | . | . | . | . | . | . | . | . | . | . | . | . |
| 21-225_29E2_HC | . | . | . | . | . | . | . | . | . | . | . | . | . | . | . | . | . | . | . | . | . | . | . | . | . |
| 21-225_29H4_HC | . | . | . | . | . | . | . | . | . | . | . | . | . | . | . | . | . | . | . | . | . | . | . | . | . |
| 21-225_2G4_HC | . | . | . | . | . | . | . | . | . | . | . | . | . | . | . | . | . | . | . | . | . | . | . | . | . |
| 21-225_32H2_HC | . | . | . | . | . | . | . | . | . | . | . | . | . | . | . | . | . | . | . | . | . | . | . | . | . |
| 21-225_3H10_HC | . | . | . | . | . | . | . | . | . | . | . | . | . | . | . | . | . | . | . | . | . | . | . | . | . |
| 21-225_43E11_HC | . | . | . | . | . | . | . | . | . | . | . | . | . | . | . | . | . | . | . | . | . | . | . | . | . |
| 21-225_44B10_HC | . | . | . | . | . | . | . | . | . | . | . | . | . | . | . | . | . | . | . | . | . | . | . | V | . |
| 21-225_44B3_HC | . | . | . | . | . | . | . | . | . | . | . | . | . | . | . | . | . | . | . | . | . | . | . | T | . |
| 21-225_44C10_HC | . | . | . | . | . | . | . | . | . | . | . | . | . | . | . | . | . | . | . | . | . | . | . | . | . |
| 21-225_44D3_HC | . | . | . | . | . | . | . | . | . | . | . | . | . | . | . | . | . | . | . | . | . | . | . | . | . |
| 21-225_44D5_HC | . | . | . | . | . | . | . | . | . | . | . | . | . | . | . | . | . | . | . | . | . | . | . | . | . |
| 21-225_44F9_HC | . | . | . | . | . | . | . | . | . | . | . | . | . | . | . | . | . | . | . | . | . | . | . | . | . |
| 21-225_45B4_HC | . | . | . | . | . | . | . | . | . | . | . | . | . | . | . | . | . | . | . | . | . | . | . | . | . |
| 21-225_45B8_HC | . | . | . | . | . | . | . | . | . | . | . | . | . | . | . | . | . | . | . | . | . | . | . | . | . |
| 21-225_46B9_HC | . | . | . | . | . | . | . | . | . | . | . | . | . | . | . | . | . | . | . | . | . | . | . | S | . |
| 21-225_46C3_HC | . | . | . | . | . | . | . | . | . | . | . | . | . | . | . | . | . | . | . | . | . | . | . | . | . |
| 21-225_46D4_HC | . | . | . | . | . | . | . | . | . | . | . | . | . | . | . | . | . | . | . | . | . | . | . | . | . |
| 21-225_47C1_HC | . | . | . | . | . | . | . | . | . | . | . | . | . | . | . | . | . | . | . | . | . | . | . | . | . |
| 21-225_47E7_HC | . | . | . | . | . | . | . | . | . | . | . | . | . | . | . | . | . | . | . | . | . | . | . | . | . |
| 21-225_48F2_HC | . | . | . | . | . | . | . | . | . | . | . | . | . | . | . | . | . | . | . | . | . | . | . | . | . |
| 21-225_48H4_HC | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | H_FR1 | | | | | | | | | | | | | | | | | | | | | | | | |
| CONSENSUS | Q | V | Q | L | V | E | S | - | G | G | G | V | V | Q | P | G | R | S | L | R | L | S | C | A | A |
| 21-225_49C1_HC | . | . | . | . | . | . | . | . | . | . | . | . | . | . | . | . | . | . | . | . | . | . | . | T | . |
| 21-225_49E7_HC | . | . | . | . | . | . | . | . | . | . | . | . | . | . | . | . | . | . | . | . | . | . | . | . | . |
| 21-225_49F9_HC | . | . | . | . | . | . | . | . | . | . | . | . | . | . | . | . | . | . | . | . | . | . | . | . | . |
| 21-225_49G3_HC | . | . | . | . | . | . | . | . | . | . | . | . | . | . | . | . | . | . | . | . | . | . | . | . | . |
| 21-225_4C12_HC | . | . | . | . | . | . | . | . | . | . | . | . | . | . | . | . | . | . | . | . | . | . | . | . | . |
| 21-225_4C5_HC | . | . | . | . | . | . | . | . | . | . | . | . | . | . | . | . | . | . | . | . | . | . | . | . | . |
| 21-225_50H4_HC | . | . | . | . | . | . | . | . | . | . | . | . | . | . | . | . | . | . | . | . | . | . | . | . | . |
| 21-225_51E1_HC | . | . | . | . | . | . | . | . | . | . | . | . | . | . | . | . | . | . | . | . | . | . | . | . | . |
| 21-225_52D10_HC | . | . | . | . | . | . | . | . | . | . | . | . | . | . | . | . | . | . | . | . | . | . | . | . | . |
| 21-225_52G8_HC | . | . | . | . | . | . | . | . | . | . | . | . | . | . | . | . | . | . | . | . | . | . | . | . | . |
| 21-225_54D4_HC | . | . | . | . | . | . | . | . | . | . | . | . | . | . | . | . | . | . | . | . | . | . | . | . | . |
| 21-225_55C2_HC | . | . | . | . | . | . | . | . | . | . | . | . | . | . | . | . | . | . | . | . | . | . | . | . | . |
| 21-225_55F9_HC | . | . | . | . | . | . | . | . | . | . | . | . | . | . | . | . | . | . | . | . | . | . | . | . | . |
| 21-225_55H1_HC | . | . | . | . | . | . | . | . | . | . | . | . | . | . | . | . | . | . | . | . | . | . | . | . | . |
| 21-225_58H11_HC | . | . | . | . | . | . | . | . | . | . | . | . | . | . | . | . | . | . | . | . | . | . | . | . | . |
| 21-225_59A12_HC | . | . | . | . | . | . | . | . | . | . | . | . | . | . | . | . | . | . | . | . | . | . | . | . | . |
| 21-225_5E5_HC | . | . | . | . | . | . | . | . | . | . | . | . | . | . | . | . | . | . | . | . | . | . | . | . | . |
| 21-225_5E5.003_HC | . | . | . | . | . | . | . | . | . | . | . | . | . | . | . | . | . | . | . | . | . | . | . | . | . |
| 21-225_5E5.004_HC | . | . | . | . | . | . | . | . | . | . | . | . | . | . | . | . | . | . | . | . | . | . | . | . | . |
| 21-225_5E5.005_HC | . | . | . | . | . | . | . | . | . | . | . | . | . | . | . | . | . | . | . | . | . | . | . | . | . |
| 21-225_5E5.006_HC | . | . | . | . | . | . | . | . | . | . | . | . | . | . | . | . | . | . | . | . | . | . | . | . | . |
| 21-225_5E5.011_HC | . | . | . | . | . | . | . | . | . | . | . | . | . | . | . | . | . | . | . | . | . | . | . | . | . |
| 21-225_5E5.012_HC | . | . | . | . | . | . | . | . | . | . | . | . | . | . | . | . | . | . | . | . | . | . | . | . | . |
| 21-225_5E5.015_HC | . | . | . | . | . | . | . | . | . | . | . | . | . | . | . | . | . | . | . | . | . | . | . | . | . |
| 21-225_5E5.016_HC | . | . | . | . | . | . | . | . | . | . | . | . | . | . | . | . | . | . | . | . | . | . | . | . | . |
| 21-225_5E5.017_HC | . | . | . | . | . | . | . | . | . | . | . | . | . | . | . | . | . | . | . | . | . | . | . | . | . |
| 21-225_5E5.018_HC | . | . | . | . | . | . | . | . | . | . | . | . | . | . | . | . | . | . | . | . | . | . | . | . | . |
| 21-225_5E5.019_HC | . | . | . | . | . | . | . | . | . | . | . | . | . | . | . | . | . | . | . | . | . | . | . | . | . |
| 21-225_5E5.023_HC | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | H_FR1 | | | | | | | | | | | | | | | | | | | | | | | | |
| CONSENSUS | Q | V | Q | L | V | E | S | - | G | G | G | V | V | Q | P | G | R | S | L | R | L | S | C | A | A |
| 21-225_5E5.024_HC | . | . | . | . | . | . | . | . | . | . | . | . | . | . | . | . | . | . | . | . | . | . | . | . | . |
| 21-225_60C12_HC | . | . | . | . | . | . | . | . | . | . | . | . | . | . | . | . | . | . | . | . | . | . | . | . | . |
| 21-225_60G2_HC | . | . | . | . | . | . | . | . | . | . | . | . | . | . | . | . | . | . | . | . | . | . | . | . | . |
| 21-225_61B3_HC | . | . | . | . | . | . | . | . | . | . | . | . | . | . | . | . | . | . | . | . | . | . | . | . | . |
| 21-225_61F2_HC | . | . | . | . | . | . | . | . | . | . | . | . | . | . | . | . | . | . | . | . | . | . | . | . | . |
| 21-225_64A4_HC | . | . | . | . | . | . | . | . | . | . | . | . | . | . | . | . | . | . | . | . | . | . | . | . | . |
| 21-225_64C8_HC | . | . | . | . | . | . | . | . | . | . | . | . | . | . | . | . | . | . | . | . | . | . | . | . | . |
| 21-225_66D11_HC | . | . | . | . | . | . | . | . | . | . | . | . | . | . | . | . | . | . | . | . | . | . | . | [illegible] | . |
| 21-225_73C9_HC | . | . | . | . | . | . | . | . | . | . | . | . | . | . | . | . | . | . | . | . | . | . | . | . | . |
| 21-225_7C9_HC | . | . | . | . | . | . | . | . | . | . | . | . | . | . | . | . | . | . | . | . | . | . | . | . | . |
| 21-225_7G4_HC | . | . | . | . | . | . | . | . | . | . | . | . | . | . | . | . | . | . | . | . | . | . | . | . | . |
| 21-225_94F3_HC | . | . | . | . | . | . | . | . | . | . | . | . | . | . | . | . | . | . | . | . | . | . | . | . | . |

FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | H_CDR1 | | | | | | | | | | H_FR2 | | | | | | | |
| CONSENSUS | S | G | - | F | T | F | S | S | - | - | - | - | - | Y | V | M | H | W | V | R | Q | A | P | G | K |
| 21-225_10E9_HC | . | . | . | . | . | . | . | N | . | . | . | . | . | . | . | I | . | . | . | . | . | . | . | . | . |
| 21-225_147A8_HC | . | . | . | . | . | . | . | . | . | . | . | . | . | . | G | . | . | . | . | . | . | . | . | . | . |
| 21-225_147B2_HC | . | . | . | . | . | . | . | . | . | . | . | . | . | . | G | . | . | . | . | . | . | . | . | . | . |
| 21-225_147H5_HC | . | . | . | . | . | . | . | D | . | . | . | . | . | . | G | . | . | . | . | . | . | . | . | . | . |
| 21-225_148C9_HC | . | . | . | . | . | . | . | D | . | . | . | . | . | . | G | . | . | . | . | . | . | . | . | . | . |
| 21-225_148F5_HC | . | . | . | . | . | . | . | . | . | . | . | . | . | . | G | . | . | . | . | . | . | . | . | . | . |
| 21-225_148H10_HC | . | . | . | . | . | . | . | . | . | . | . | . | . | . | G | . | . | . | . | . | . | . | . | . | . |
| 21-225_149D10_HC | . | . | . | . | . | . | . | D | . | . | . | . | . | . | G | . | . | . | . | . | . | . | . | . | . |
| 21-225_14E10_HC | . | . | . | . | . | . | . | D | . | . | . | . | . | . | G | . | . | . | . | . | . | . | . | . | . |
| 21-225_150D11_HC | . | . | . | . | . | . | . | . | . | . | . | . | . | . | G | . | . | . | . | . | . | . | . | . | . |
| 21-225_150E2_HC | . | . | . | . | . | . | . | . | . | . | . | . | . | . | . | . | . | . | . | . | . | . | . | . | . |
| 21-225_152F7_HC | . | . | . | . | . | . | . | . | . | . | . | . | . | . | I | . | . | . | . | . | . | . | . | . | . |
| 21-225_153G9_HC | . | . | . | . | . | . | . | . | . | . | . | . | . | . | G | . | . | . | . | . | . | . | . | . | . |
| 21-225_154F4_HC | . | . | . | . | . | . | . | . | . | . | . | . | . | . | G | . | . | . | . | . | . | . | . | . | . |
| 21-225_159D8_HC | . | . | . | . | . | . | . | N | . | . | . | . | . | . | . | . | . | C | . | . | . | . | . | . | . |
| 21-225_15C11_HC | . | . | . | . | . | . | . | D | . | . | . | . | . | . | . | . | . | . | . | . | . | . | . | . | . |
| 21-225_169D11_HC | . | . | . | . | . | . | . | D | . | . | . | . | . | . | . | . | . | . | . | . | . | . | . | . | . |
| 21-225_170F3_HC | . | . | . | . | S | . | . | D | . | . | . | . | . | . | . | . | . | . | . | . | . | . | . | . | . |
| 21-225_173C11_HC | . | . | . | . | . | . | . | . | . | . | . | . | . | . | G | I | . | . | . | . | . | . | . | . | . |
| 21-225_174G10_HC | . | . | . | . | . | . | . | D | . | . | . | . | . | . | . | . | . | . | . | . | . | . | . | . | . |
| 21-225_176H12_HC | . | . | . | . | . | . | . | D | . | . | . | . | . | . | . | . | . | . | . | . | . | . | . | . | . |
| 21-225_177B4_HC | . | . | . | . | . | . | . | D | . | . | . | . | . | . | . | . | . | . | . | . | . | . | . | . | . |
| 21-225_179C2_HC | . | . | . | . | . | . | . | . | . | . | . | . | . | . | G | . | . | . | . | . | . | . | . | . | . |
| 21-225_17H3_HC | . | . | . | . | . | . | . | N | . | . | . | . | . | C | . | . | . | . | . | . | . | . | . | . | . |
| 21-225_183F4_HC | . | . | . | . | . | . | . | . | . | . | . | . | . | . | G | . | . | . | . | . | . | . | . | . | . |
| 21-225_190D6_HC | . | . | . | . | . | . | . | . | . | . | . | . | . | F | I | . | . | . | . | . | . | . | . | . | R |
| 21-225_192A2_HC | . | . | . | . | . | . | . | . | . | . | . | . | . | . | G | . | . | . | . | . | . | . | . | . | . |
| 21-225_192F6_HC | . | . | . | . | . | . | . | . | . | . | . | . | . | F | I | . | . | . | . | . | . | . | . | . | R |
| 21-225_193F8_HC | . | . | . | . | . | . | . | I | . | . | . | . | . | . | G | . | D | . | . | . | . | . | . | . | . |

FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | H_CDR1 | | | | | | | | | | H_FR2 | | | | | | | |
| CONSENSUS | S | G | - | F | T | F | S | S | - | - | - | - | - | Y | V | M | H | W | V | R | Q | A | P | G | K |
| 21-225_194F10_HC | . | . | . | . | . | . | . | . | . | . | . | . | . | F | I | . | . | . | . | . | . | . | . | . | R |
| 21-225_201C5_HC | . | . | . | . | . | . | . | D | . | . | . | . | . | . | . | . | . | . | . | . | . | . | . | . | . |
| 21-225_203F7_HC | . | . | . | . | . | . | . | D | . | . | . | . | . | . | . | . | . | . | . | . | . | . | . | . | . |
| 21-225_204H3_HC | . | . | . | . | . | . | . | . | . | . | . | . | . | . | . | . | . | . | . | . | . | . | . | . | . |
| 21-225_208B2_HC | . | . | . | . | . | . | . | D | . | . | . | . | . | C | . | . | . | . | . | . | . | . | . | . | . |
| 21-225_208F1_HC | . | R | . | . | . | . | . | D | . | . | . | . | . | . | . | . | . | . | . | . | . | . | . | . | . |
| 21-225_20B2_HC | . | . | . | . | . | . | . | N | . | . | . | . | . | . | . | I | . | . | . | . | . | . | . | . | . |
| 21-225_210G5_HC | . | . | . | . | . | . | . | D | . | . | . | . | . | C | . | . | . | . | . | . | . | . | . | . | . |
| 21-225_210G6_HC | . | . | . | . | . | . | . | D | . | . | . | . | . | . | . | . | . | . | . | . | . | . | . | . | . |
| 21-225_212B11_HC | . | . | . | . | . | . | . | D | . | . | . | . | . | . | . | . | . | . | . | . | . | . | . | . | . |
| 21-225_214G10_HC | . | . | . | . | . | . | . | D | . | . | . | . | . | . | . | . | . | . | . | . | . | . | . | . | . |
| 21-225_214G12_HC | . | . | . | . | . | . | . | D | . | . | . | . | . | . | . | . | . | . | . | . | . | . | . | . | . |
| 21-225_214H9_HC | . | . | . | . | . | . | . | . | . | . | . | . | . | . | . | . | . | . | . | . | . | . | . | . | . |
| 21-225_215E11_HC | . | . | . | . | . | . | . | D | . | . | . | . | . | . | . | . | . | . | . | . | . | . | . | . | . |
| 21-225_215E3_HC | . | . | . | . | . | . | . | D | . | . | . | . | . | . | . | I | . | . | . | . | . | . | . | . | . |
| 21-225_216E8_HC | . | . | . | . | . | . | . | D | . | . | . | . | . | . | . | . | . | . | . | . | . | . | . | . | . |
| 21-225_216H12_HC | . | . | . | . | . | . | . | D | . | . | . | . | . | . | . | . | . | . | . | . | . | . | . | . | . |
| 21-225_217E5_HC | . | . | . | . | . | . | . | D | . | . | . | . | . | . | . | . | . | . | . | . | . | . | . | . | . |
| 21-225_217G10_HC | . | . | . | . | . | . | . | D | . | . | . | . | . | . | . | . | . | . | . | . | . | . | . | . | . |
| 21-225_217H12_HC | . | . | . | . | . | . | . | D | . | . | . | . | . | . | . | . | . | . | . | . | . | . | . | . | . |
| 21-225_218C4_HC | . | . | . | . | . | . | . | D | . | . | . | . | . | . | . | . | . | . | . | . | . | . | . | . | . |
| 21-225_220F8_HC | . | . | . | . | . | . | . | D | . | . | . | . | . | . | . | . | . | . | . | . | . | . | . | . | . |
| 21-225_224E7_HC | . | . | . | . | . | . | . | . | . | . | . | . | . | . | . | . | . | . | . | . | . | . | . | . | . |
| 21-225_224F1_HC | . | . | . | . | . | . | . | . | . | . | . | . | . | . | I | . | . | . | . | . | . | . | . | . | . |
| 21-225_224F3_HC | . | . | . | . | S | . | . | . | . | . | . | . | . | . | G | . | . | . | . | . | . | . | . | . | . |
| 21-225_225A1_HC | . | . | . | . | . | . | . | D | . | . | . | . | . | . | . | I | . | . | . | . | . | . | . | . | . |
| 21-225_226C6_HC | . | . | . | . | . | . | . | D | . | . | . | . | . | . | G | . | . | . | . | . | . | . | . | . | . |
| 21-225_226H11_HC | . | . | . | . | . | . | . | N | . | . | . | . | . | C | G | . | . | . | . | . | . | . | . | . | . |
| 21-225_22F4_HC | . | . | . | . | . | . | . | D | . | . | . | . | . | . | G | . | . | . | . | . | . | . | . | . | . |

# FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | H_CDR1 | | | | | | | | | | H_FR2 | | | | | | | |
| CONSENSUS | S | G | - | F | T | F | S | S | - | - | - | - | - | Y | V | M | H | W | V | R | Q | A | P | G | K |
| 21-225_23A10_HC | . | . | . | . | . | . | . | . | . | . | . | . | . | . | G | . | . | . | . | . | . | . | . | . | . |
| 21-225_24B6_HC | . | . | . | . | I | . | . | N | . | . | . | . | . | . | . | . | . | . | . | . | . | . | . | . | . |
| 21-225_25A2_HC | . | . | . | . | . | . | . | . | . | . | . | . | . | . | G | . | . | . | . | . | . | . | . | . | . |
| 21-225_25D12_HC | . | . | . | . | . | . | . | . | . | . | . | . | . | D | G | . | . | . | . | . | . | . | . | . | . |
| 21-225_26A11_HC | . | . | . | . | . | . | . | N | . | . | . | . | . | C | . | . | . | . | . | . | . | . | . | . | . |
| 21-225_26G7_HC | . | . | . | . | . | . | . | . | . | . | . | . | . | . | G | . | . | . | . | . | . | . | . | . | . |
| 21-225_27E7_HC | . | . | . | . | . | . | . | . | . | . | . | . | . | . | G | . | . | . | . | . | . | . | . | . | . |
| 21-225_28H2_HC | . | . | . | . | . | . | . | N | . | . | . | . | . | . | . | . | . | . | . | . | . | . | . | . | . |
| 21-225_29E2_HC | . | . | . | . | . | . | . | N | . | . | . | . | . | C | . | . | . | . | . | . | . | . | . | . | . |
| 21-225_29H4_HC | . | . | . | . | . | . | . | . | . | . | . | . | . | . | G | I | . | . | . | . | . | . | . | . | . |
| 21-225_2G4_HC | . | . | . | . | . | . | . | . | . | . | . | . | . | . | . | . | . | . | . | . | . | . | . | . | . |
| 21-225_32H2_HC | . | . | . | . | . | . | . | . | . | . | . | . | . | . | G | . | . | . | . | . | . | . | . | . | . |
| 21-225_3H10_HC | . | . | . | . | . | . | . | . | . | . | . | . | . | . | . | . | . | . | . | . | . | . | . | . | . |
| 21-225_43E11_HC | . | . | . | . | S | . | . | D | . | . | . | . | . | . | G | . | . | . | . | . | . | . | . | . | . |
| 21-225_44B10_HC | . | . | . | . | . | . | . | . | . | . | . | . | . | . | G | . | . | . | . | . | . | . | . | . | . |
| 21-225_44B3_HC | . | . | . | . | . | . | . | D | . | . | . | . | . | . | G | . | . | . | . | . | . | . | . | . | . |
| 21-225_44C10_HC | . | . | . | . | . | . | . | D | . | . | . | . | . | C | . | . | . | . | . | . | . | . | . | . | . |
| 21-225_44D3_HC | . | . | . | . | . | . | . | . | . | . | . | . | . | . | . | . | . | . | . | . | . | . | . | . | . |
| 21-225_44D5_HC | . | . | . | . | . | . | . | . | . | . | . | . | . | . | . | . | . | . | . | . | . | . | . | . | . |
| 21-225_44F9_HC | . | . | . | . | . | . | . | . | . | . | . | . | . | . | . | . | . | . | . | . | . | . | . | . | . |
| 21-225_45B4_HC | . | . | . | . | . | . | . | D | . | . | . | . | . | C | . | . | . | . | . | . | . | . | . | . | . |
| 21-225_45B8_HC | . | . | . | . | . | . | . | D | . | . | . | . | . | C | . | . | . | . | . | . | . | . | . | . | . |
| 21-225_46B9_HC | . | . | . | . | . | . | . | . | . | . | . | . | . | . | G | . | . | . | . | . | . | . | . | . | . |
| 21-225_46C3_HC | . | . | . | . | . | . | . | . | . | . | . | . | . | . | . | . | . | . | . | . | . | . | . | . | . |
| 21-225_46D4_HC | . | . | . | . | . | . | . | . | . | . | . | . | . | . | . | . | . | . | . | . | . | . | . | . | . |
| 21-225_47C1_HC | . | . | . | . | . | . | . | D | . | . | . | . | . | . | G | . | . | . | . | . | . | T | . | . | . |
| 21-225_47E7_HC | . | . | . | . | . | . | . | I | . | . | . | . | . | . | G | . | . | . | . | . | . | . | . | . | . |
| 21-225_48F2_HC | . | . | . | . | . | . | . | N | . | . | . | . | . | . | . | . | . | . | . | . | . | . | . | . | . |
| 21-225_48H4_HC | . | . | . | . | . | . | . | D | . | . | . | . | . | S | L | . | . | . | . | . | . | . | . | . | . |

# FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | H_CDR1 | | | | | | | | | | H_FR2 | | | | | | | |
| CONSENSUS | S | G | - | F | T | F | S | S | - | - | - | - | - | Y | V | M | H | W | V | R | Q | A | P | G | K |
| 21-225_49C1_HC | . | . | . | . | . | . | . | . | . | . | . | . | . | . | G | . | . | . | . | . | . | . | . | . | . |
| 21-225_49E7_HC | . | . | . | . | . | . | . | . | . | . | . | . | . | . | G | . | . | . | . | . | . | . | . | . | . |
| 21-225_49F9_HC | . | . | . | . | . | . | . | . | . | . | . | . | . | . | G | . | . | . | . | . | . | . | . | . | . |
| 21-225_49G3_HC | . | . | . | . | . | . | . | . | . | . | . | . | . | . | G | . | . | . | . | . | . | . | . | . | . |
| 21-225_4C12_HC | . | . | . | . | . | . | . | . | . | . | . | . | . | . | . | . | . | . | . | . | . | . | . | . | . |
| 21-225_4C5_HC | . | . | . | . | . | . | . | . | . | . | . | . | . | . | . | L | . | . | . | . | . | . | . | . | . |
| 21-225_50H4_HC | . | . | . | . | . | . | . | . | . | . | . | . | . | . | . | . | . | . | . | . | . | . | . | . | . |
| 21-225_51E1_HC | . | . | . | . | . | . | . | . | . | . | . | . | . | . | G | . | . | . | . | . | . | . | . | . | . |
| 21-225_52D10_HC | . | . | . | . | . | . | . | . | . | . | . | . | . | . | G | . | . | . | . | . | . | . | . | . | . |
| 21-225_52G8_HC | . | . | . | . | . | . | . | . | . | . | . | . | . | . | G | . | . | . | . | . | . | . | . | . | . |
| 21-225_54D4_HC | . | . | . | . | . | . | . | . | . | . | . | . | . | . | G | . | . | . | . | . | . | . | . | . | . |
| 21-225_55C2_HC | . | . | . | . | . | . | . | . | . | . | . | . | . | . | G | . | . | . | . | . | . | . | . | . | . |
| 21-225_55F9_HC | . | . | . | . | . | . | . | . | . | . | . | . | . | . | G | . | . | . | . | . | . | . | . | . | . |
| 21-225_55H1_HC | . | . | . | . | . | . | . | . | . | . | . | . | . | . | G | . | . | . | . | . | . | . | . | . | . |
| 21-225_58H11_HC | . | . | . | . | . | . | . | . | . | . | . | . | . | . | G | . | . | . | . | . | . | . | . | . | . |
| 21-225_59A12_HC | . | . | . | . | . | . | . | . | . | . | . | . | . | . | G | . | . | . | . | . | . | . | . | . | . |
| 21-225_5E5_HC | . | . | . | . | . | . | . | N | . | . | . | . | . | . | . | . | . | . | . | . | . | . | . | . | . |
| 21-225_5E5.003_HC | . | . | . | . | . | . | . | N | . | . | . | . | . | . | . | . | . | . | . | . | . | . | . | . | . |
| 21-225_5E5.004_HC | . | . | . | . | . | . | . | N | . | . | . | . | . | . | . | . | . | . | . | . | . | . | . | . | . |
| 21-225_5E5.005_HC | . | . | . | . | . | . | . | N | . | . | . | . | . | . | . | . | . | . | . | . | . | . | . | . | . |
| 21-225_5E5.006_HC | . | . | . | . | . | . | . | N | . | . | . | . | . | . | . | . | . | . | . | . | . | . | . | . | . |
| 21-225_5E5.011_HC | . | . | . | . | . | . | . | N | . | . | . | . | . | . | . | . | . | . | . | . | . | . | . | . | . |
| 21-225_5E5.012_HC | . | . | . | . | . | . | . | N | . | . | . | . | . | . | . | . | . | . | . | . | . | . | . | . | . |
| 21-225_5E5.015_HC | . | . | . | . | . | . | . | N | . | . | . | . | . | . | . | . | . | . | . | . | . | . | . | . | . |
| 21-225_5E5.016_HC | . | . | . | . | . | . | . | N | . | . | . | . | . | . | . | . | . | . | . | . | . | . | . | . | . |
| 21-225_5E5.017_HC | . | . | . | . | . | . | . | N | . | . | . | . | . | . | . | . | . | . | . | . | . | . | . | . | . |
| 21-225_5E5.018_HC | . | . | . | . | . | . | . | N | . | . | . | . | . | . | . | . | . | . | . | . | . | . | . | . | . |
| 21-225_5E5.019_HC | . | . | . | . | . | . | . | N | . | . | . | . | . | . | . | . | . | . | . | . | . | . | . | . | . |
| 21-225_5E5.023_HC | . | . | . | . | . | . | . | N | . | . | . | . | . | . | . | . | . | . | . | . | . | . | . | . | . |

**FIGURE 57**

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | H_CDR1 | | | | | | | | | | H_FR2 | | | | | | | |
| CONSENSUS | S | G | - | F | T | F | S | S | - | - | - | - | - | Y | V | M | H | W | V | R | Q | A | P | G | K |
| 21-225_5E5.024_HC | . | . | . | . | . | . | . | N | . | . | . | . | . | . | . | . | . | . | . | . | . | . | . | . | . |
| 21-225_60C12_HC | . | . | . | . | . | . | . | . | . | . | . | . | . | . | G | . | . | . | . | . | . | . | . | . | . |
| 21-225_60G2_HC | . | . | . | . | . | . | . | . | . | . | . | . | . | . | G | . | . | . | . | . | . | . | . | . | . |
| 21-225_61B3_HC | . | . | . | . | . | . | . | . | . | . | . | . | . | . | G | . | . | . | . | . | . | . | . | . | . |
| 21-225_61F2_HC | . | . | . | . | . | . | . | . | . | . | . | . | . | . | G | . | . | . | . | . | . | . | . | . | . |
| 21-225_64A4_HC | . | . | . | . | . | . | . | D | . | . | . | . | . | . | . | . | . | . | . | . | . | . | . | . | . |
| 21-225_64C8_HC | . | . | . | . | . | . | . | D | . | . | . | . | . | . | . | . | . | . | . | . | . | . | . | . | . |
| 21-225_66D11_HC | . | . | . | . | . | . | . | . | . | . | . | . | . | . | G | . | . | . | . | . | . | . | . | . | . |
| 21-225_73C9_HC | . | . | . | . | . | . | . | N | . | . | . | . | . | . | . | . | . | . | . | . | . | . | . | . | . |
| 21-225_7C9_HC | . | . | . | L | . | . | . | N | . | . | . | . | . | . | . | . | . | . | . | . | . | . | . | . | . |
| 21-225_7G4_HC | . | . | . | . | . | . | N | N | . | . | . | . | . | . | . | . | . | . | . | . | . | . | . | . | . |
| 21-225_94F3_HC | . | . | . | . | . | . | . | N | . | . | . | . | . | D | I | . | . | . | . | . | . | . | . | . | . |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | H_CDR2 | | | | | | | | | | | | | | | | | | |
| CONSENSUS | G | L | E | W | V | A | V | I | W | Y | D | - | - | - | G | S | N | K | Y | Y | A | D | S | V | K |
| 21-225_10E9_HC | . | . | . | . | . | . | . | . | . | . | . | . | . | . | . | . | . | . | . | . | . | . | . | . | . |
| 21-225_147A8_HC | . | . | . | . | . | . | . | . | . | . | . | . | . | . | . | . | . | . | . | . | . | . | . | . | . |
| 21-225_147B2_HC | . | . | . | . | . | . | . | . | . | . | . | . | . | . | . | . | . | . | . | . | . | . | . | . | . |
| 21-225_147H5_HC | . | . | . | . | . | . | . | . | . | . | . | . | . | . | . | . | . | . | . | . | . | . | . | . | . |
| 21-225_148C9_HC | . | . | . | . | . | . | . | . | . | . | . | . | . | . | . | . | . | . | . | . | . | . | . | . | . |
| 21-225_148F5_HC | . | . | . | . | . | . | . | . | . | . | . | . | . | . | . | . | . | . | . | . | . | . | . | . | . |
| 21-225_148H10_HC | . | . | . | . | . | . | . | . | . | . | . | . | . | . | . | . | . | . | . | . | G | . | . | . | . |
| 21-225_149D10_HC | . | . | . | . | . | . | . | . | . | . | . | . | . | . | . | . | . | . | . | . | . | . | . | . | . |
| 21-225_14E10_HC | . | . | . | . | . | . | . | . | . | . | . | . | . | . | . | . | . | . | . | . | . | . | . | . | . |
| 21-225_150D11_HC | . | . | . | . | . | . | . | . | F | . | . | . | . | . | . | . | . | . | H | . | . | . | . | . | . |
| 21-225_150E2_HC | . | . | . | . | . | . | . | . | . | . | . | . | . | . | . | . | . | . | . | . | . | . | . | . | . |
| 21-225_152F7_HC | . | . | . | . | . | . | . | . | . | . | . | . | . | . | . | . | . | . | . | . | . | . | . | . | . |
| 21-225_153G9_HC | . | . | . | . | . | . | L | . | F | . | . | . | . | . | . | . | . | . | . | . | . | . | . | . | . |
| 21-225_154F4_HC | . | . | . | . | . | . | . | . | . | . | . | . | . | . | . | . | . | . | . | . | . | . | . | . | . |
| 21-225_159D8_HC | . | . | . | . | . | . | . | . | . | . | . | . | . | . | . | . | . | . | . | . | . | . | . | . | . |
| 21-225_15C11_HC | . | . | . | . | . | . | . | . | . | . | . | . | . | . | . | . | . | . | . | . | . | . | . | . | . |
| 21-225_169D11_HC | . | . | . | . | . | . | . | . | . | . | . | . | . | . | . | . | . | . | . | . | . | . | . | . | . |
| 21-225_170F3_HC | . | . | . | . | . | . | . | . | . | . | . | . | . | . | . | . | . | . | . | . | E | . | . | . | . |
| 21-225_173C11_HC | . | . | . | . | . | . | L | . | F | . | . | . | . | . | . | . | . | . | . | . | . | . | . | . | . |
| 21-225_174G10_HC | . | . | . | . | . | . | . | . | . | . | . | . | . | . | . | . | . | . | . | . | . | . | . | . | . |
| 21-225_176H12_HC | . | . | . | . | . | . | . | . | . | . | . | . | . | . | . | . | . | . | . | . | . | . | . | . | . |
| 21-225_177B4_HC | . | . | . | . | . | . | . | . | . | . | . | . | . | . | . | . | . | . | . | . | . | . | . | . | . |
| 21-225_179C2_HC | . | . | . | . | . | . | L | . | F | . | . | . | . | . | . | . | . | . | . | . | . | . | . | . | . |
| 21-225_17H3_HC | . | . | . | . | . | . | . | . | . | . | . | . | . | . | . | . | . | . | . | . | . | . | . | . | . |
| 21-225_183F4_HC | . | . | . | . | . | . | . | . | . | . | . | . | . | . | . | . | . | . | . | . | . | . | . | . | . |
| 21-225_190D6_HC | . | . | . | . | . | . | . | . | . | . | . | . | . | . | . | . | . | . | . | . | . | . | . | . | . |
| 21-225_192A2_HC | . | . | . | . | L | . | . | . | . | . | . | . | . | . | . | . | Y | . | . | . | . | . | . | . | . |
| 21-225_192F6_HC | . | . | . | . | . | . | . | . | . | . | . | . | . | . | . | . | . | . | . | . | V | . | . | . | . |
| 21-225_193F8_HC | . | . | . | . | . | . | . | . | . | . | . | . | . | . | . | . | Y | . | . | . | . | . | . | . | . |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | | | | | | | | | | H_CDR2 | | | | | | | | | |
| CONSENSUS | G | L | E | W | V | A | V | I | W | Y | D | - | - | - | G | S | N | K | Y | Y | A | D | S | V | K |
| 21-225_194F10_HC | . | . | . | . | . | . | . | . | . | . | . | . | . | . | . | . | . | . | . | . | . | . | . | . | . |
| 21-225_201C5_HC | . | . | . | . | . | . | . | . | . | . | . | . | . | . | . | . | . | . | . | . | V | . | . | . | . |
| 21-225_203F7_HC | . | . | . | . | . | . | . | . | . | . | . | . | . | . | . | . | . | . | . | . | V | . | . | . | . |
| 21-225_204H3_HC | . | . | . | . | . | . | . | . | . | . | . | . | . | . | . | . | . | . | . | N | . | . | . | . | . |
| 21-225_208B2_HC | . | . | . | . | . | . | . | . | . | . | . | . | . | . | . | . | . | . | . | . | V | . | . | . | . |
| 21-225_208F1_HC | . | . | . | . | . | . | . | . | . | . | . | . | . | . | . | . | . | . | . | . | V | . | . | . | . |
| 21-225_20B2_HC | . | . | . | . | . | . | . | . | . | . | . | . | . | . | . | . | . | . | . | . | . | . | . | . | . |
| 21-225_210G5_HC | . | . | . | . | . | . | . | . | . | . | . | . | . | . | . | . | . | . | . | . | V | . | . | . | . |
| 21-225_210G6_HC | . | . | . | . | . | . | . | . | . | . | . | . | . | . | . | . | . | . | . | . | V | . | . | . | . |
| 21-225_212B11_HC | . | . | . | . | . | T | . | . | . | . | . | . | . | . | . | . | . | . | . | . | . | . | . | . | . |
| 21-225_214G10_HC | . | . | . | . | . | T | . | . | . | . | . | . | . | . | . | . | . | . | . | . | . | . | . | . | . |
| 21-225_214G12_HC | . | . | . | . | . | T | . | . | . | . | . | . | . | . | . | . | . | . | . | . | . | . | . | . | . |
| 21-225_214H9_HC | . | . | . | . | . | . | . | . | . | . | . | . | . | . | . | . | . | . | . | . | . | . | . | . | . |
| 21-225_215E11_HC | . | . | . | . | . | T | . | . | . | . | . | . | . | . | . | . | . | . | . | . | . | . | . | . | . |
| 21-225_215E3_HC | . | . | . | . | . | T | . | . | . | . | . | . | . | . | . | . | . | . | . | . | . | . | . | . | . |
| 21-225_216E8_HC | . | . | . | . | . | T | . | . | . | . | . | . | . | . | . | . | . | . | . | . | . | . | . | . | . |
| 21-225_216H12_HC | . | . | . | . | . | T | . | . | . | . | . | . | . | . | . | . | . | . | . | . | . | . | . | . | . |
| 21-225_217E5_HC | . | . | . | . | . | T | . | . | . | . | . | . | . | . | . | . | . | . | . | . | . | . | . | . | . |
| 21-225_217G10_HC | . | . | . | . | . | T | . | . | . | . | . | . | . | . | . | . | . | . | . | . | . | . | . | . | . |
| 21-225_217H12_HC | . | . | . | . | . | T | . | . | . | . | . | . | . | . | . | . | . | . | . | . | . | . | . | . | . |
| 21-225_218C4_HC | . | . | . | . | . | T | . | . | . | . | . | . | . | . | . | . | . | . | . | . | . | . | . | . | . |
| 21-225_220F8_HC | . | . | . | . | . | T | . | . | . | . | . | . | . | . | . | . | . | . | . | . | . | . | . | . | . |
| 21-225_224E7_HC | . | . | . | . | . | . | . | . | . | . | . | . | . | . | . | . | . | . | . | . | . | . | . | . | . |
| 21-225_224F1_HC | . | . | . | . | . | . | . | . | . | . | . | . | . | . | . | . | . | . | . | . | . | . | . | . | . |
| 21-225_224F3_HC | . | . | . | . | . | . | . | . | . | . | . | . | . | . | . | . | . | . | . | . | . | . | . | . | . |
| 21-225_225A1_HC | . | . | . | . | . | . | . | . | . | . | . | . | . | . | . | . | . | . | . | . | . | . | . | . | . |
| 21-225_226C6_HC | . | . | . | . | . | . | . | . | . | . | . | . | . | . | . | . | . | . | . | . | . | . | . | . | . |
| 21-225_226H11_HC | . | . | . | . | . | T | . | . | . | . | . | . | . | . | . | . | . | . | . | . | . | . | . | . | . |
| 21-225_22F4_HC | . | . | . | . | . | . | . | . | . | . | . | . | . | . | . | . | . | . | . | . | V | . | . | . | . |

# FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | H_CDR2 | | | | | | | | | | | | | | | | | | |
| CONSENSUS | G | L | E | W | V | A | V | I | W | Y | D | - | - | - | G | S | N | K | Y | Y | A | D | S | V | K |
| 21-225_23A10_HC | . | . | . | . | . | . | . | . | . | . | . | . | . | . | . | . | . | . | . | . | . | . | . | . | . |
| 21-225_24B6_HC | . | . | . | . | . | . | . | . | . | . | . | . | . | . | . | . | . | . | . | . | . | . | . | . | . |
| 21-225_25A2_HC | . | . | . | . | . | . | . | . | . | . | . | . | . | . | . | . | . | . | . | . | . | . | . | . | . |
| 21-225_25D12_HC | . | . | . | . | . | . | . | . | . | . | . | . | . | . | . | . | . | . | . | . | . | . | . | . | . |
| 21-225_26A11_HC | . | . | . | . | . | . | . | . | . | . | . | . | . | . | . | . | . | . | . | . | . | . | . | . | . |
| 21-225_26G7_HC | . | . | . | . | . | . | . | . | . | . | . | . | . | . | . | . | . | . | . | . | . | . | . | . | . |
| 21-225_27E7_HC | . | . | . | . | . | . | . | . | . | . | . | . | . | . | . | . | . | . | . | . | . | . | . | . | . |
| 21-225_28H2_HC | . | . | . | . | . | . | . | . | . | . | . | . | . | . | . | . | . | . | . | . | . | . | . | . | . |
| 21-225_29E2_HC | . | . | . | . | . | . | . | . | . | . | . | . | . | . | . | . | . | . | . | . | . | . | . | . | . |
| 21-225_29H4_HC | . | . | . | . | . | . | . | . | . | . | . | . | . | . | . | . | . | . | . | . | . | . | . | . | . |
| 21-225_2G4_HC | . | . | . | . | . | . | . | . | . | . | . | . | . | . | . | . | . | . | . | . | . | . | . | . | . |
| 21-225_32H2_HC | . | . | . | . | . | . | . | . | . | . | . | . | . | . | . | . | . | . | . | . | . | . | . | . | . |
| 21-225_3H10_HC | . | . | . | . | . | . | . | . | . | . | . | . | . | . | . | . | . | . | . | . | . | . | . | . | . |
| 21-225_43E11_HC | . | . | . | . | . | . | . | . | . | . | . | . | . | . | . | . | . | . | . | . | . | . | . | . | . |
| 21-225_44B10_HC | . | . | . | . | . | . | . | . | . | . | . | . | . | . | . | . | . | . | . | . | . | . | . | . | . |
| 21-225_44B3_HC | . | . | . | . | . | . | . | . | . | . | . | . | . | . | . | . | . | . | . | . | . | . | . | . | . |
| 21-225_44C10_HC | . | . | . | . | . | . | . | . | . | . | . | . | . | . | . | . | . | . | . | . | . | . | . | . | . |
| 21-225_44D3_HC | . | . | . | . | . | . | . | . | . | . | . | . | . | . | . | . | . | . | . | . | . | . | . | . | . |
| 21-225_44D5_HC | . | . | . | . | . | . | . | . | . | . | . | . | . | . | . | . | . | . | . | . | . | . | . | . | . |
| 21-225_44F9_HC | . | . | . | . | . | . | . | . | . | . | . | . | . | . | . | . | . | . | . | . | . | . | . | . | . |
| 21-225_45B4_HC | . | . | . | . | . | . | . | . | . | . | . | . | . | . | . | . | . | . | . | . | . | . | . | . | . |
| 21-225_45B8_HC | . | . | . | . | . | . | . | . | . | . | . | . | . | . | . | . | . | . | . | . | . | . | . | . | . |
| 21-225_46B9_HC | . | . | . | . | . | . | . | . | . | . | . | . | . | . | . | R | . | . | . | . | . | . | . | . | . |
| 21-225_46C3_HC | . | . | . | . | . | . | . | . | . | . | . | . | . | . | . | . | . | . | . | . | . | . | . | . | . |
| 21-225_46D4_HC | . | . | . | . | . | . | . | . | . | . | . | . | . | . | . | . | . | . | . | . | . | . | . | . | . |
| 21-225_47C1_HC | . | . | . | . | . | . | . | . | . | . | . | . | . | . | . | . | . | . | . | . | . | . | . | . | . |
| 21-225_47E7_HC | . | . | . | . | . | . | . | . | . | . | . | . | . | . | . | . | . | . | . | . | . | . | . | . | . |
| 21-225_48F2_HC | . | . | . | . | . | . | . | . | . | . | . | . | . | . | . | . | . | . | . | . | . | . | . | . | . |
| 21-225_48H4_HC | . | . | . | . | . | . | . | . | . | . | . | . | . | . | . | . | . | . | . | . | T | . | . | . | . |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | H_CDR2 | | | | | | | | | | | | | | | | | | |
| CONSENSUS | G | L | E | W | V | A | V | I | W | Y | D | - | - | - | G | S | N | K | Y | Y | A | D | S | V | K |
| 21-225_49C1_HC | . | . | . | . | . | . | . | . | . | . | . | . | . | . | . | . | . | . | . | . | . | . | . | . | . |
| 21-225_49E7_HC | . | . | . | . | . | . | . | . | . | . | . | . | . | . | . | . | . | . | . | . | . | . | . | . | . |
| 21-225_49F9_HC | . | . | . | . | . | . | L | . | . | . | . | . | . | . | . | R | . | . | . | . | . | . | . | . | . |
| 21-225_49G3_HC | . | . | . | . | . | . | . | . | . | . | . | . | . | . | . | . | . | . | . | . | . | . | . | . | . |
| 21-225_4C12_HC | . | . | . | . | . | . | . | . | . | . | . | . | . | . | . | . | . | . | . | . | . | . | . | . | . |
| 21-225_4C5_HC | . | . | . | . | . | . | . | . | . | . | . | . | . | . | . | . | . | . | . | . | . | . | . | . | . |
| 21-225_50H4_HC | . | . | . | . | . | . | A | . | . | . | . | . | . | . | . | . | . | . | . | . | . | . | . | . | . |
| 21-225_51E1_HC | . | . | . | . | . | . | . | . | . | . | . | . | . | . | . | . | S | . | . | . | . | . | . | . | . |
| 21-225_52D10_HC | . | . | . | . | . | . | L | . | . | . | . | . | . | . | . | . | . | . | . | H | . | . | . | . | . |
| 21-225_52G8_HC | . | . | . | . | . | . | . | . | . | . | . | . | . | . | . | . | . | . | . | . | . | . | . | . | . |
| 21-225_54D4_HC | . | . | . | . | . | . | . | . | . | . | . | . | . | . | . | . | . | . | . | . | . | . | . | . | . |
| 21-225_55C2_HC | . | . | . | . | . | . | L | . | . | . | . | . | . | . | . | . | . | . | . | H | . | . | . | . | . |
| 21-225_55F9_HC | . | . | . | . | . | . | . | . | . | . | . | . | . | . | . | N | G | . | . | . | . | . | . | . | . |
| 21-225_55H1_HC | . | . | . | . | . | . | . | . | . | . | . | . | . | . | . | . | . | . | . | . | . | . | . | . | . |
| 21-225_58H11_HC | . | . | . | . | . | . | . | . | . | . | . | . | . | . | . | . | . | . | . | H | . | . | . | . | . |
| 21-225_59A12_HC | . | . | . | . | . | . | . | . | . | . | . | . | . | . | . | . | . | . | . | . | . | . | . | . | . |
| 21-225_5E5_HC | . | . | . | . | . | . | . | . | . | . | . | . | . | . | . | . | . | . | . | . | . | . | . | . | . |
| 21-225_5E5.003_HC | . | . | . | . | . | . | . | . | . | . | . | . | . | . | A | . | . | . | . | . | . | . | . | . | . |
| 21-225_5E5.004_HC | . | . | . | . | . | . | . | . | . | . | . | . | . | . | . | . | . | . | . | . | . | E | . | . | . |
| 21-225_5E5.005_HC | . | . | . | . | . | . | . | . | . | . | . | . | . | . | . | . | . | . | . | . | . | G | . | . | . |
| 21-225_5E5.006_HC | . | . | . | . | . | . | . | . | . | . | . | . | . | . | . | . | . | . | . | . | . | . | A | . | . |
| 21-225_5E5.011_HC | . | . | . | . | . | . | . | . | . | . | . | . | . | . | A | . | . | . | . | . | . | E | . | . | . |
| 21-225_5E5.012_HC | . | . | . | . | . | . | . | . | . | . | . | . | . | . | A | . | . | . | . | . | . | . | A | . | . |
| 21-225_5E5.015_HC | . | . | . | . | . | . | . | . | . | . | . | . | . | . | A | . | . | . | . | . | . | . | . | . | . |
| 21-225_5E5.016_HC | . | . | . | . | . | . | . | . | . | . | . | . | . | . | A | . | . | . | . | . | . | . | . | . | . |
| 21-225_5E5.017_HC | . | . | . | . | . | . | . | . | . | . | . | . | . | . | . | . | . | . | . | . | . | E | . | . | . |
| 21-225_5E5.018_HC | . | . | . | . | . | . | . | . | . | . | . | . | . | . | . | . | . | . | . | . | . | . | . | . | . |
| 21-225_5E5.019_HC | . | . | . | . | . | . | . | . | . | . | . | . | . | . | . | . | . | . | . | . | . | . | . | . | . |
| 21-225_5E5.023_HC | . | . | . | . | . | . | . | . | . | . | . | . | . | . | A | . | . | . | . | . | . | E | . | . | . |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | H_CDR2 | | | | | | | | | | | | | | | | | | |
| CONSENSUS | G | L | E | W | V | A | V | I | W | Y | D | - | - | - | G | S | N | K | Y | Y | A | D | S | V | K |
| 21-225_5E5.024_HC | . | . | . | . | . | . | . | . | . | . | . | . | . | . | A | . | . | . | . | . | . | . | A | . | . |
| 21-225_60C12_HC | . | . | . | . | . | . | . | . | . | . | . | . | . | . | . | . | . | . | . | . | V | . | . | . | . |
| 21-225_60G2_HC | . | . | . | . | . | . | . | . | . | . | . | . | . | . | . | . | . | . | . | . | . | . | . | . | . |
| 21-225_61B3_HC | . | . | . | . | . | . | . | . | . | . | . | . | . | . | . | . | . | . | . | . | . | . | . | . | . |
| 21-225_61F2_HC | . | . | . | . | . | . | . | . | . | . | . | . | . | . | . | . | . | . | . | . | . | . | . | . | . |
| 21-225_64A4_HC | . | . | . | . | . | . | . | . | . | . | . | . | . | . | . | . | . | . | . | . | . | . | . | . | . |
| 21-225_64C8_HC | . | . | . | . | . | . | . | . | . | . | . | . | . | . | . | . | . | . | . | . | . | . | . | M | . |
| 21-225_66D11_HC | . | . | . | . | . | . | . | . | . | . | . | . | . | . | . | . | . | . | . | . | . | . | . | . | . |
| 21-225_73C9_HC | . | . | . | . | . | . | . | . | . | . | . | . | . | . | . | . | . | . | . | . | . | . | . | . | . |
| 21-225_7C9_HC | . | . | . | . | . | . | . | . | . | . | . | . | . | . | . | . | . | . | . | . | . | . | . | . | . |
| 21-225_7G4_HC | . | . | . | . | . | . | . | . | . | . | . | . | . | . | . | . | . | . | . | . | . | . | . | . | . |
| 21-225_94F3_HC | . | . | . | . | . | . | . | . | . | . | . | . | . | . | . | . | . | . | . | . | . | . | . | . | . |

FIGURE 57

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | H_FR3 | | | | | | | | | | | | | | | | | | | | | | | |
| CONSENSUS | G | R | F | T | I | S | R | D | N | S | K | N | T | L | Y | L | Q | M | N | S | L | R | A | E | D |
| 21-225_10E9_HC | . | . | . | . | . | . | . | . | . | . | . | . | . | . | . | . | . | . | . | . | . | . | . | . | . |
| 21-225_147A8_HC | . | . | . | . | . | . | . | . | . | . | . | . | . | . | . | . | . | . | . | . | . | . | . | . | . |
| 21-225_147B2_HC | . | . | . | . | . | . | . | . | . | . | . | . | . | . | . | . | . | . | . | . | . | . | . | . | . |
| 21-225_147H5_HC | . | . | L | . | . | . | . | . | . | . | . | . | . | . | . | . | . | . | . | . | . | S | . | . | . |
| 21-225_148C9_HC | . | . | L | . | . | . | . | . | . | . | . | . | . | . | . | . | . | . | . | . | . | . | . | . | . |
| 21-225_148F5_HC | . | . | . | . | . | . | . | . | . | . | . | . | . | . | . | . | . | . | . | . | . | . | . | . | . |
| 21-225_148H10_HC | . | . | . | . | . | . | . | . | . | . | . | . | . | . | . | . | . | . | . | . | . | . | . | . | . |
| 21-225_149D10_HC | . | . | L | . | . | . | . | . | . | . | . | . | . | . | . | . | . | . | . | . | . | . | . | . | . |
| 21-225_14E10_HC | . | . | . | . | . | . | . | . | . | . | . | . | . | . | . | . | . | . | . | . | . | . | . | . | . |
| 21-225_150D11_HC | . | . | . | . | . | . | . | . | . | . | . | . | . | . | . | . | . | . | . | . | . | . | . | . | . |
| 21-225_150E2_HC | . | . | . | . | . | . | . | . | . | . | . | . | . | . | . | . | . | . | . | . | . | . | . | . | . |
| 21-225_152F7_HC | . | . | . | . | . | . | . | . | . | . | . | . | . | . | . | . | . | . | . | . | . | . | . | . | . |
| 21-225_153G9_HC | . | . | . | . | . | . | . | . | . | . | . | . | . | . | . | . | . | I | . | . | . | . | . | . | . |
| 21-225_154F4_HC | . | . | . | . | . | . | . | . | . | . | . | . | . | . | . | . | . | . | . | . | . | . | . | . | . |
| 21-225_159D8_HC | . | . | . | . | . | . | . | . | . | . | . | . | . | . | . | . | . | . | . | . | . | . | . | . | . |
| 21-225_15C11_HC | . | . | . | . | . | . | . | . | . | . | . | . | . | . | . | . | . | . | . | . | . | . | . | . | . |
| 21-225_169D11_HC | . | . | . | . | . | . | . | . | . | . | . | . | . | . | . | . | . | L | . | . | . | . | . | . | . |
| 21-225_170F3_HC | . | . | . | . | . | . | . | . | . | . | . | . | . | . | . | . | . | . | . | . | . | . | . | . | . |
| 21-225_173C11_HC | . | . | . | . | . | . | . | . | . | . | . | . | . | . | . | . | H | . | S | . | . | . | . | . | . |
| 21-225_174G10_HC | . | . | . | . | . | . | . | . | . | . | . | . | . | . | . | . | . | L | . | . | . | . | . | . | . |
| 21-225_176H12_HC | . | . | . | . | . | . | . | . | . | . | . | . | . | . | . | . | . | . | . | . | . | . | . | . | . |
| 21-225_177B4_HC | . | . | . | I | V | . | . | . | . | . | . | . | . | . | . | . | . | . | . | . | . | . | . | . | . |
| 21-225_179C2_HC | . | . | . | . | . | . | . | . | . | . | . | . | . | . | F | . | . | . | . | . | . | . | . | . | . |
| 21-225_17H3_HC | . | . | . | . | . | . | . | . | . | . | . | . | . | . | . | . | . | . | . | . | . | . | . | . | . |
| 21-225_183F4_HC | . | . | . | . | . | . | . | . | . | . | . | . | . | . | . | . | . | . | . | . | . | . | . | . | . |
| 21-225_190D6_HC | . | . | . | . | . | . | . | . | . | . | . | . | . | . | . | . | . | . | . | . | . | . | . | . | . |
| 21-225_192A2_HC | . | . | . | . | . | . | . | . | . | . | . | . | . | . | . | . | . | . | . | . | . | . | . | . | . |
| 21-225_192F6_HC | . | . | . | . | . | . | . | . | . | . | . | . | . | . | . | . | . | . | . | . | . | . | . | . | . |
| 21-225_193F8_HC | . | . | . | . | . | . | . | . | . | . | . | . | . | . | . | . | . | . | . | . | . | . | . | . | . |

# FIGURE 57

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | H_FR3 | | | | | | | | | | | | | | | | | | | | | | | |
| CONSENSUS | G | R | F | T | I | S | R | D | N | S | K | N | T | L | Y | L | Q | M | N | S | L | R | A | E | D |
| 21-225_194F10_HC | . | . | . | . | . | . | . | . | . | . | . | . | . | . | . | . | E | . | . | . | . | . | V | . | . |
| 21-225_201C5_HC | . | . | . | . | . | . | . | . | . | . | R | . | . | . | . | . | . | . | . | . | . | . | . | . | . |
| 21-225_203F7_HC | . | . | . | . | . | . | . | . | . | . | R | . | . | . | . | . | . | . | . | . | . | . | . | . | . |
| 21-225_204H3_HC | . | . | . | . | . | . | . | . | . | . | . | . | . | . | . | . | . | . | . | . | . | . | . | . | . |
| 21-225_208B2_HC | . | . | . | . | . | . | . | . | . | . | R | . | . | . | . | . | . | . | . | . | . | . | . | . | . |
| 21-225_208F1_HC | . | . | . | . | . | . | . | . | . | . | R | . | . | . | . | . | . | . | . | . | . | . | . | . | . |
| 21-225_20B2_HC | . | . | . | . | . | . | . | . | . | . | . | . | . | . | . | . | . | . | . | . | . | . | . | . | . |
| 21-225_210G5_HC | . | . | . | . | . | . | . | . | . | . | R | . | . | . | . | . | . | . | . | . | . | . | . | . | . |
| 21-225_210G6_HC | . | . | . | . | . | . | . | . | . | . | R | . | . | . | . | . | . | . | . | . | . | . | . | . | . |
| 21-225_212B11_HC | . | . | . | . | . | . | . | . | . | . | . | . | . | . | . | . | . | . | . | . | . | . | . | . | . |
| 21-225_214G10_HC | . | . | . | . | . | . | . | . | . | . | . | . | . | . | . | . | . | . | . | . | . | . | . | . | . |
| 21-225_214G12_HC | . | . | . | . | . | . | . | . | . | . | . | . | . | . | . | . | . | . | . | . | . | . | . | . | . |
| 21-225_214H9_HC | . | . | . | . | . | . | . | . | . | . | . | . | . | . | . | . | . | . | . | . | . | . | . | . | . |
| 21-225_215E11_HC | . | . | . | . | . | . | . | . | . | . | . | . | . | . | . | . | . | . | . | . | . | . | . | . | . |
| 21-225_215E3_HC | . | . | . | . | . | . | . | . | . | . | . | . | . | . | . | . | . | . | . | . | V | . | . | . | . |
| 21-225_216E8_HC | . | . | . | . | . | . | . | . | . | . | . | . | . | . | . | . | . | . | . | . | . | . | . | . | . |
| 21-225_216H12_HC | . | . | . | . | . | . | . | . | . | . | . | . | . | . | . | . | . | . | . | . | . | . | . | . | . |
| 21-225_217E5_HC | . | . | . | . | . | . | . | . | . | . | . | . | . | . | . | . | . | . | . | . | . | . | . | . | . |
| 21-225_217G10_HC | . | . | . | . | . | . | . | . | . | . | . | . | . | . | . | . | . | . | . | . | . | . | . | . | . |
| 21-225_217H12_HC | . | . | . | . | . | . | . | . | . | . | . | . | . | . | . | . | . | . | . | . | . | . | . | . | . |
| 21-225_218C4_HC | . | . | . | . | . | . | . | . | . | . | . | . | . | . | . | . | . | . | . | . | . | . | . | . | . |
| 21-225_220F8_HC | . | . | . | . | . | . | . | . | . | . | . | . | . | . | . | . | . | . | . | . | . | . | . | . | . |
| 21-225_224E7_HC | . | . | . | . | . | . | . | . | . | . | . | . | . | . | . | . | . | . | . | . | . | . | . | . | . |
| 21-225_224F1_HC | . | . | . | . | . | . | . | . | . | . | . | . | . | . | . | . | . | . | . | . | . | . | . | . | . |
| 21-225_224F3_HC | . | . | . | . | . | . | . | . | . | . | . | . | . | . | . | . | . | . | . | . | . | . | . | . | . |
| 21-225_225A1_HC | . | . | . | . | . | . | . | . | . | . | . | . | . | . | . | . | . | . | . | . | . | . | . | . | . |
| 21-225_226C6_HC | . | . | . | . | . | . | . | . | . | . | . | . | . | . | . | . | . | T | . | . | . | . | . | . | . |
| 21-225_226H11_HC | . | . | . | . | . | . | . | . | . | . | . | . | . | . | . | . | . | . | . | . | . | . | . | . | . |
| 21-225_22F4_HC | . | . | . | . | . | . | . | . | . | . | . | . | . | . | . | . | . | . | . | . | . | . | . | . | . |

**FIGURE 57**

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | H_FR3 | | | | | | | | | | | | | | | | | | | | | | | |
| CONSENSUS | G | R | F | T | I | S | R | D | N | S | K | N | T | L | Y | L | Q | M | N | S | L | R | A | E | D |
| 21-225_23A10_HC | . | . | . | . | . | . | . | . | . | . | . | . | . | . | . | . | . | . | . | . | . | . | . | . | . |
| 21-225_24B6_HC | . | . | . | . | . | . | . | . | . | . | . | . | . | . | . | . | . | . | . | . | . | . | . | . | . |
| 21-225_25A2_HC | . | . | . | . | . | . | . | . | . | . | . | . | . | . | . | . | . | . | . | . | . | . | . | . | . |
| 21-225_25D12_HC | . | . | . | . | . | . | . | . | . | . | . | . | . | . | . | . | . | . | . | . | . | . | . | . | . |
| 21-225_26A11_HC | . | . | . | . | . | . | . | . | . | . | . | . | . | . | . | . | . | . | . | . | . | . | . | . | . |
| 21-225_26G7_HC | . | . | . | . | . | . | . | . | . | . | N | . | . | . | . | . | . | . | . | . | . | . | . | . | . |
| 21-225_27E7_HC | . | . | . | . | . | . | . | . | . | . | . | . | . | . | . | . | . | . | . | . | . | . | . | . | . |
| 21-225_28H2_HC | . | . | . | . | . | . | . | . | . | . | . | . | . | . | . | . | . | . | . | . | . | . | . | V | . |
| 21-225_29E2_HC | . | . | I | . | . | . | . | . | . | . | . | . | . | . | . | . | . | . | . | . | . | . | . | . | . |
| 21-225_29H4_HC | . | . | . | . | . | . | . | . | . | . | . | . | . | . | . | . | . | . | . | . | . | . | . | . | . |
| 21-225_2G4_HC | . | . | . | . | . | . | . | . | . | . | . | . | . | . | . | . | . | . | . | . | . | . | . | . | . |
| 21-225_32H2_HC | . | . | . | . | . | . | . | . | . | . | . | . | . | . | N | . | . | . | . | . | . | . | . | . | . |
| 21-225_3H10_HC | . | . | . | . | . | . | . | . | . | . | . | . | . | . | . | . | . | . | . | . | . | . | . | . | . |
| 21-225_43E11_HC | . | . | L | . | . | . | . | . | . | . | . | . | . | . | . | . | . | . | . | . | . | . | . | . | . |
| 21-225_44B10_HC | . | . | . | . | . | . | . | . | . | . | . | . | . | . | . | . | . | . | . | . | . | . | . | . | . |
| 21-225_44B3_HC | . | . | . | . | . | . | . | . | . | . | . | . | . | . | . | . | . | . | . | . | . | . | . | . | . |
| 21-225_44C10_HC | . | . | . | . | . | . | . | . | . | . | . | . | . | . | . | . | . | . | . | . | . | . | . | . | . |
| 21-225_44D3_HC | . | . | . | . | . | . | . | . | . | . | . | . | . | . | . | . | . | . | . | . | . | . | . | . | . |
| 21-225_44D5_HC | . | . | . | . | . | . | . | . | . | . | . | . | . | . | . | . | . | . | . | . | . | . | . | . | . |
| 21-225_44F9_HC | . | . | . | . | . | . | . | . | . | . | . | . | . | . | . | . | . | . | . | . | . | . | . | . | . |
| 21-225_45B4_HC | . | . | . | . | . | . | . | . | . | . | . | . | . | . | . | . | . | . | . | . | . | . | . | . | . |
| 21-225_45B8_HC | . | . | . | . | . | . | . | . | . | . | . | . | . | . | . | . | . | . | . | . | . | . | . | . | . |
| 21-225_46B9_HC | . | . | I | . | . | . | . | . | . | . | . | . | . | . | . | . | . | . | . | . | . | . | . | . | . |
| 21-225_46C3_HC | . | . | . | . | . | . | . | . | . | . | . | . | . | . | . | . | . | . | . | . | . | . | . | . | . |
| 21-225_46D4_HC | . | . | . | . | . | . | . | . | . | . | . | . | . | . | . | . | . | . | . | . | . | . | . | . | . |
| 21-225_47C1_HC | . | . | . | . | . | . | . | . | . | . | . | . | . | . | F | . | . | . | . | . | . | . | D | . | . |
| 21-225_47E7_HC | . | . | . | . | . | . | . | . | . | . | . | . | . | . | . | . | . | . | . | . | . | . | . | . | . |
| 21-225_48F2_HC | . | . | . | . | . | . | . | . | . | . | . | . | . | . | . | . | . | . | . | . | . | . | . | . | . |
| 21-225_48H4_HC | . | . | . | . | . | . | . | . | . | . | . | . | . | . | . | . | . | . | . | . | . | . | . | . | . |

FIGURE 57

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | H_FR3 | | | | | | | | | | | | | | | | | | | | | | | |
| CONSENSUS | G | R | F | T | I | S | R | D | N | S | K | N | T | L | Y | L | Q | M | N | S | L | R | A | E | D |
| 21-225_49C1_HC | . | . | . | . | . | . | . | . | . | . | . | . | . | . | . | . | . | . | . | . | . | . | . | . | . |
| 21-225_49E7_HC | . | . | . | . | . | . | . | . | . | . | . | . | . | . | . | . | . | . | . | . | . | . | . | . | . |
| 21-225_49F9_HC | . | . | . | . | . | . | . | . | . | P | . | . | . | . | . | . | . | . | . | . | . | . | . | . | . |
| 21-225_49G3_HC | . | . | . | . | . | . | . | . | . | . | . | . | . | . | . | . | . | . | . | . | . | . | . | . | . |
| 21-225_4C12_HC | . | . | . | . | . | . | . | . | . | . | . | . | . | . | . | . | . | . | . | . | . | . | . | . | . |
| 21-225_4C5_HC | . | . | . | . | . | . | . | . | . | . | . | . | . | . | . | . | . | . | . | . | . | . | . | . | . |
| 21-225_50H4_HC | . | . | . | . | . | . | . | . | . | . | . | . | . | . | D | . | . | . | . | . | . | . | . | . | . |
| 21-225_51E1_HC | . | . | . | . | . | . | . | . | . | . | . | . | . | . | . | . | . | . | . | . | . | . | . | . | . |
| 21-225_52D10_HC | . | . | . | . | . | . | . | . | . | . | . | . | . | . | . | . | . | . | . | . | . | . | . | . | . |
| 21-225_52G8_HC | . | . | . | . | . | . | . | . | . | . | . | . | . | . | . | . | . | . | . | . | . | . | . | . | . |
| 21-225_54D4_HC | . | . | . | . | . | . | . | . | . | . | . | . | . | . | . | . | . | . | . | . | . | . | . | . | . |
| 21-225_55C2_HC | . | . | . | . | . | . | . | . | . | . | . | . | . | . | . | . | . | . | . | . | . | . | . | . | . |
| 21-225_55F9_HC | . | . | . | . | . | . | . | . | . | . | . | . | . | . | . | . | . | . | . | . | . | . | . | . | . |
| 21-225_55H1_HC | . | . | . | . | . | . | . | . | . | . | . | . | . | . | . | . | . | . | . | . | . | . | . | . | . |
| 21-225_58H11_HC | . | . | . | . | . | . | . | . | . | . | . | . | . | . | . | . | . | . | . | . | . | . | . | . | . |
| 21-225_59A12_HC | . | . | . | . | . | . | . | . | . | . | . | . | . | . | . | . | . | . | . | . | . | . | . | . | . |
| 21-225_5E5_HC | . | . | . | . | . | . | . | . | . | . | . | . | . | . | . | . | . | . | . | . | . | . | . | . | . |
| 21-225_5E5.003_HC | . | . | . | . | . | . | . | . | . | . | . | . | . | . | . | . | . | . | . | . | . | . | . | . | . |
| 21-225_5E5.004_HC | . | . | . | . | . | . | . | . | . | . | . | . | . | . | . | . | . | . | . | . | . | . | . | . | . |
| 21-225_5E5.005_HC | . | . | . | . | . | . | . | . | . | . | . | . | . | . | . | . | . | . | . | . | . | . | . | . | . |
| 21-225_5E5.006_HC | . | . | . | . | . | . | . | . | . | . | . | . | . | . | . | . | . | . | . | . | . | . | . | . | . |
| 21-225_5E5.011_HC | . | . | . | . | . | . | . | . | . | . | . | . | . | . | . | . | . | . | . | . | . | . | . | . | . |
| 21-225_5E5.012_HC | . | . | . | . | . | . | . | . | . | . | . | . | . | . | . | . | . | . | . | . | . | . | . | . | . |
| 21-225_5E5.015_HC | . | . | . | . | . | . | . | . | . | . | . | . | . | . | . | . | . | . | . | . | . | . | . | . | . |
| 21-225_5E5.016_HC | . | . | . | . | . | . | . | . | . | . | . | . | . | . | . | . | . | . | . | . | . | . | . | . | . |
| 21-225_5E5.017_HC | . | . | . | . | . | . | . | . | . | . | . | . | . | . | . | . | . | . | . | . | . | . | . | . | . |
| 21-225_5E5.018_HC | . | . | . | . | . | . | . | . | . | . | . | . | . | . | . | . | . | . | . | . | . | . | . | . | . |
| 21-225_5E5.019_HC | . | . | . | . | . | . | . | . | . | . | . | . | . | . | . | . | . | . | . | . | . | . | . | . | . |
| 21-225_5E5.023_HC | . | . | . | . | . | . | . | . | . | . | . | . | . | . | . | . | . | . | . | . | . | . | . | . | . |

FIGURE 57

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | H_FR3 | | | | | | | | | | | | | | | | | | | | | | | |
| CONSENSUS | G | R | F | T | I | S | R | D | N | S | K | N | T | L | Y | L | Q | M | N | S | L | R | A | E | D |
| 21-225_5E5.024_HC | . | . | . | . | . | . | . | . | . | . | . | . | . | . | . | . | . | . | . | . | . | . | . | . | . |
| 21-225_60C12_HC | . | . | . | . | . | . | . | . | . | . | . | . | . | . | . | . | . | . | . | . | . | . | . | . | . |
| 21-225_60G2_HC | . | . | . | . | . | . | . | . | . | . | . | . | . | . | . | . | . | . | . | . | . | . | . | . | . |
| 21-225_61B3_HC | . | . | . | . | . | . | . | . | . | . | . | . | . | . | . | . | . | . | . | . | . | . | . | . | . |
| 21-225_61F2_HC | . | . | . | . | . | . | . | . | . | . | . | . | . | . | . | . | . | . | . | . | . | . | . | . | . |
| 21-225_64A4_HC | . | . | . | . | . | . | . | . | . | . | Q | . | . | . | . | . | . | . | . | . | . | . | . | . | . |
| 21-225_64C8_HC | . | . | . | . | . | . | . | . | . | . | . | . | . | . | . | . | . | . | . | . | . | . | . | . | . |
| 21-225_66D11_HC | . | . | . | . | . | . | . | . | . | . | . | . | . | . | S | . | . | . | . | . | . | . | . | . | . |
| 21-225_73C9_HC | . | . | . | . | . | . | . | . | . | . | . | . | . | . | . | . | . | . | . | . | . | . | . | . | . |
| 21-225_7C9_HC | . | . | . | . | . | . | . | . | . | . | . | . | . | . | . | . | . | . | . | . | . | . | . | . | . |
| 21-225_7G4_HC | . | . | . | . | . | . | . | . | . | . | . | . | . | . | . | . | . | . | T | . | . | . | . | . | . |
| 21-225_94F3_HC | . | . | . | . | . | . | . | . | . | . | . | . | . | . | . | . | . | I | . | . | . | . | . | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | R | E | R | Y | S | S | G | W | - | - | - | - | - | - | - | - | - | - |
| 21-225_10E9_HC | . | . | . | . | . | . | . | . | . | K | . | . | . | S | . | . | . | . | . | . | . | . | . | . | . |
| 21-225_147A8_HC | . | . | . | . | . | . | . | . | R | Y | S | . | . | W | - | . | . | . | . | . | . | . | . | . | . |
| 21-225_147B2_HC | . | . | . | . | . | . | . | . | R | Y | S | . | . | W | - | . | . | . | . | . | . | . | . | . | . |
| 21-225_147H5_HC | . | . | . | . | . | . | . | . | . | . | . | . | . | . | . | . | . | . | . | . | . | . | . | . | . |
| 21-225_148C9_HC | . | . | . | . | . | . | . | . | . | . | . | . | . | . | . | . | . | . | . | . | . | . | . | . | . |
| 21-225_148F5_HC | . | . | . | . | . | . | . | . | R | Y | S | . | . | W | - | . | . | . | . | . | . | . | . | . | . |
| 21-225_148H10_HC | . | . | . | . | . | . | . | . | R | Y | S | . | . | W | - | . | . | . | . | . | . | . | . | . | . |
| 21-225_149D10_HC | . | . | . | . | . | . | . | . | . | . | . | . | . | . | . | . | . | . | . | . | . | . | . | . | . |
| 21-225_14E10_HC | . | . | . | . | . | . | . | . | . | K | . | . | . | S | . | . | . | . | . | . | . | . | . | . | . |
| 21-225_150D11_HC | S | . | . | . | . | . | T | . | R | F | S | . | . | W | - | . | . | . | . | . | . | . | . | . | . |
| 21-225_150E2_HC | . | . | . | . | . | . | . | . | . | E | . | . | . | S | . | . | . | . | . | . | . | . | . | . | . |
| 21-225_152F7_HC | . | . | . | . | . | . | . | . | . | E | . | . | . | S | . | . | . | . | . | . | . | . | . | . | . |
| 21-225_153G9_HC | . | . | . | . | F | . | . | . | R | Y | S | R | . | W | - | . | . | . | . | . | . | . | . | . | . |
| 21-225_154F4_HC | . | . | . | . | . | . | . | . | . | D | S | Y | C | S | G | T | S | C | . | . | . | . | . | . | . |
| 21-225_159D8_HC | . | . | . | . | . | . | . | . | . | P | . | . | . | . | . | . | . | . | . | . | . | . | . | . | . |
| 21-225_15C11_HC | . | . | . | . | . | . | . | . | . | K | . | . | . | S | . | . | . | . | . | . | . | . | . | . | . |
| 21-225_169D11_HC | . | . | . | . | . | . | . | . | . | . | . | . | . | . | . | . | . | . | . | . | . | . | . | . | . |
| 21-225_170F3_HC | . | . | . | . | . | . | . | . | . | T | . | . | . | S | . | . | . | . | . | . | . | . | . | . | . |
| 21-225_173C11_HC | . | . | . | . | . | . | . | . | R | Y | S | . | . | W | - | . | . | . | . | . | . | . | . | . | . |
| 21-225_174G10_HC | . | . | . | . | . | . | . | . | . | . | . | . | . | . | . | . | . | . | . | . | . | . | . | . | . |
| 21-225_176H12_HC | . | . | . | . | . | . | . | . | . | K | . | . | . | N | . | . | . | . | . | . | . | . | . | . | . |
| 21-225_177B4_HC | . | . | . | . | . | . | . | . | . | K | . | . | . | S | . | . | . | . | . | . | . | . | . | . | . |
| 21-225_179C2_HC | . | . | . | . | . | . | . | . | R | Y | S | G | . | W | - | . | . | . | . | . | . | . | . | . | . |
| 21-225_17H3_HC | . | . | . | . | . | . | . | . | . | K | . | . | . | S | . | . | . | . | . | . | . | . | . | . | . |
| 21-225_183F4_HC | . | . | . | . | . | . | . | . | . | . | T | Y | C | S | G | T | T | C | . | . | . | . | . | . | . |
| 21-225_190D6_HC | . | . | . | . | . | . | . | . | . | E | . | . | . | . | . | . | . | . | . | . | . | . | . | . | . |
| 21-225_192A2_HC | . | . | . | . | . | . | . | . | . | D | S | P | Y | S | G | Y | A | . | . | . | . | . | . | . | . |
| 21-225_192F6_HC | . | . | . | . | . | . | . | . | . | E | . | . | . | . | . | . | . | . | . | . | . | . | . | . | . |
| 21-225_193F8_HC | . | . | . | . | . | . | V | . | . | D | S | P | Y | S | G | Y | G | . | . | . | . | . | . | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | R | E | R | Y | S | S | G | W | - | - | - | - | - | - | - | - | - | - |
| 21-225_194F10_HC | . | . | . | . | . | . | . | . | . | E | . | . | . | . | . | . | . | . | . | . | . | . | . | . | . |
| 21-225_201C5_HC | . | . | . | . | . | . | . | . | . | . | . | . | . | . | L | . | . | . | . | . | . | . | . | . | . |
| 21-225_203F7_HC | . | . | . | . | . | . | . | . | . | . | . | . | . | . | L | . | . | . | . | . | . | . | . | . | . |
| 21-225_204H3_HC | . | . | . | . | . | . | . | . | . | P | . | . | . | S | . | . | . | . | . | . | . | . | . | . | . |
| 21-225_208B2_HC | . | . | . | . | . | . | . | . | . | . | . | . | . | . | L | . | . | . | . | . | . | . | . | . | . |
| 21-225_208F1_HC | . | . | . | . | . | . | . | . | . | . | . | . | . | . | L | . | . | . | . | . | . | . | . | . | . |
| 21-225_20B2_HC | . | . | . | . | S | . | . | . | . | K | . | . | . | S | . | . | . | . | . | . | . | . | . | . | . |
| 21-225_210G5_HC | . | . | . | . | . | . | . | . | . | . | . | . | . | . | L | . | . | . | . | . | . | . | . | . | . |
| 21-225_210G6_HC | . | . | . | . | . | . | . | . | . | . | . | . | . | . | L | . | . | . | . | . | . | . | . | . | . |
| 21-225_212B11_HC | . | . | . | . | . | . | . | . | . | . | . | . | . | . | . | . | . | . | . | . | . | . | . | . | . |
| 21-225_214G10_HC | . | . | . | . | . | . | . | . | . | . | . | . | . | . | . | . | . | . | . | . | . | . | . | . | . |
| 21-225_214G12_HC | . | . | . | . | . | . | . | . | . | . | . | . | . | . | . | . | . | . | . | . | . | . | . | . | . |
| 21-225_214H9_HC | . | . | . | F | . | . | . | . | . | . | . | T | . | S | . | . | . | . | . | . | . | . | . | . | . |
| 21-225_215E11_HC | . | . | . | . | . | . | . | . | . | . | . | . | . | . | . | . | . | . | . | . | . | . | . | . | . |
| 21-225_215E3_HC | . | . | . | . | . | . | . | . | . | . | . | . | . | . | . | . | . | . | . | . | . | . | . | . | . |
| 21-225_216E8_HC | . | . | . | . | . | . | . | . | . | . | . | . | . | . | . | . | . | . | . | . | . | . | . | . | . |
| 21-225_216H12_HC | . | . | . | . | . | . | . | . | . | . | . | . | . | . | . | . | . | . | . | . | . | . | . | . | . |
| 21-225_217E5_HC | . | . | . | . | . | . | . | . | . | . | . | . | . | . | . | . | . | . | . | . | . | . | . | . | . |
| 21-225_217G10_HC | . | . | . | . | . | . | . | . | . | . | . | . | . | . | . | . | . | . | . | . | . | . | . | . | . |
| 21-225_217H12_HC | . | . | . | . | . | . | . | . | . | . | . | . | . | . | . | . | . | . | . | . | . | . | . | . | . |
| 21-225_218C4_HC | . | . | . | . | . | . | . | . | . | . | . | . | . | . | . | . | . | . | . | . | . | . | . | . | . |
| 21-225_220F8_HC | . | . | . | . | . | . | . | . | . | . | . | . | . | . | . | . | . | . | . | . | . | . | . | . | . |
| 21-225_224E7_HC | . | . | . | . | . | . | . | . | . | Q | . | . | . | S | . | . | . | . | . | . | . | . | . | . | . |
| 21-225_224F1_HC | . | . | . | . | . | . | . | . | . | . | . | . | . | N | . | . | . | . | . | . | . | . | . | . | . |
| 21-225_224F3_HC | . | . | . | . | . | . | . | . | . | . | . | . | . | S | . | . | . | . | . | . | . | . | . | . | . |
| 21-225_225A1_HC | . | . | . | . | . | . | . | . | . | . | . | . | . | . | . | . | . | . | . | . | . | . | . | . | . |
| 21-225_226C6_HC | . | . | . | . | . | . | . | . | . | . | . | . | . | S | . | . | . | . | . | . | . | . | . | . | . |
| 21-225_226H11_HC | . | . | . | . | . | . | . | . | . | L | . | . | . | S | . | . | . | . | . | . | . | . | . | . | . |
| 21-225_22F4_HC | . | . | . | . | . | . | . | . | . | K | . | . | . | S | . | . | . | . | . | . | . | . | . | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | H_CDR3 | | | | | | | | | | | | | | | | |
| CONSENSUS | T | A | V | Y | Y | C | A | R | E | R | Y | S | S | G | W | - | - | - | - | - | - | - | - | - | - |
| 21-225_23A10_HC | . | . | . | . | . | . | . | . | . | . | . | . | . | . | . | . | . | . | . | . | . | . | . | . | . |
| 21-225_24B6_HC | . | . | . | . | . | . | . | . | . | E | . | . | . | . | . | . | . | . | . | . | . | . | . | . | . |
| 21-225_25A2_HC | . | . | . | . | . | . | . | . | R | Y | S | . | . | W | - | . | . | . | . | . | . | . | . | . | . |
| 21-225_25D12_HC | . | . | . | . | . | . | . | . | . | . | . | . | . | S | . | . | . | . | . | . | . | . | . | . | . |
| 21-225_26A11_HC | . | . | . | . | . | . | . | . | . | E | . | . | . | . | . | . | . | . | . | . | . | . | . | . | . |
| 21-225_26G7_HC | . | . | . | . | . | . | . | . | R | Y | S | . | . | W | - | . | . | . | . | . | . | . | . | . | . |
| 21-225_27E7_HC | . | . | . | . | . | . | . | . | . | D | S | Y | C | S | S | T | S | C | . | . | . | . | . | . | . |
| 21-225_28H2_HC | . | . | . | . | . | . | . | . | . | E | . | . | . | . | . | . | . | . | . | . | . | . | . | . | . |
| 21-225_29E2_HC | . | . | . | . | . | . | . | . | . | E | . | . | . | . | . | . | . | . | . | . | . | . | . | . | . |
| 21-225_29H4_HC | . | . | . | . | . | . | . | . | R | Y | S | . | . | W | - | . | . | . | . | . | . | . | . | . | . |
| 21-225_2G4_HC | . | . | . | . | . | . | . | . | . | K | . | . | . | S | . | . | . | . | . | . | . | . | . | . | . |
| 21-225_32H2_HC | . | G | . | . | . | . | . | . | R | Y | S | . | . | W | - | . | . | . | . | . | . | . | . | . | . |
| 21-225_3H10_HC | . | . | . | . | . | . | . | . | . | . | . | . | . | . | . | . | . | . | . | . | . | . | . | . | . |
| 21-225_43E11_HC | . | . | . | . | . | . | . | . | R | Y | V | R | . | W | - | . | . | . | . | . | . | . | . | . | . |
| 21-225_44B10_HC | . | . | . | . | . | . | . | . | R | Y | S | . | . | W | - | . | . | . | . | . | . | . | . | . | . |
| 21-225_44B3_HC | . | . | . | . | . | . | . | . | . | . | . | . | . | . | . | . | . | . | . | . | . | . | . | . | . |
| 21-225_44C10_HC | . | . | . | . | . | . | . | . | . | . | . | . | . | . | L | . | . | . | . | . | . | . | . | . | . |
| 21-225_44D3_HC | . | . | . | . | . | . | . | . | . | . | . | . | . | . | L | . | . | . | . | . | . | . | . | . | . |
| 21-225_44D5_HC | . | . | . | . | . | . | . | . | V | P | . | . | . | S | . | . | . | . | . | . | . | . | . | . | . |
| 21-225_44F9_HC | . | . | . | . | . | . | . | . | . | P | . | . | . | S | . | . | . | . | . | . | . | . | . | . | . |
| 21-225_45B4_HC | . | . | . | . | . | . | . | . | . | . | . | . | . | . | L | . | . | . | . | . | . | . | . | . | . |
| 21-225_45B8_HC | . | . | . | . | . | . | . | . | . | . | . | . | . | . | L | . | . | . | . | . | . | . | . | . | . |
| 21-225_46B9_HC | . | . | . | . | . | . | . | . | R | Y | S | . | . | W | - | . | . | . | . | . | . | . | . | . | . |
| 21-225_46C3_HC | . | . | . | . | . | . | . | . | . | . | . | . | . | . | L | . | . | . | . | . | . | . | . | . | . |
| 21-225_46D4_HC | . | . | . | . | . | . | . | . | V | P | . | . | . | S | . | . | . | . | . | . | . | . | . | . | . |
| 21-225_47C1_HC | . | . | . | . | . | . | . | . | R | Y | S | R | . | W | - | . | . | . | . | . | . | . | . | . | . |
| 21-225_47E7_HC | . | . | . | . | . | . | . | . | R | Y | S | R | . | W | - | . | . | . | . | . | . | . | . | . | . |
| 21-225_48F2_HC | . | . | . | . | . | . | . | . | . | . | . | . | . | S | . | . | . | . | . | . | . | . | . | . | . |
| 21-225_48H4_HC | . | . | . | . | . | . | . | . | . | N | . | . | . | . | . | . | . | . | . | . | . | . | . | . | . |

## FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | R | E | R | Y | S | S | G | W | - | - | - | - | - | - | - | - | - | - |
| 21-225_49C1_HC | . | . | . | . | . | . | . | . | R | Y | S | . | . | W | - | . | . | . | . | . | . | . | . | . | . |
| 21-225_49E7_HC | . | . | L | . | . | . | . | . | R | Y | S | . | . | W | - | . | . | . | . | . | . | . | . | . | . |
| 21-225_49F9_HC | . | . | . | . | H | . | . | . | R | Y | S | . | . | W | - | . | . | . | . | . | . | . | . | . | . |
| 21-225_49G3_HC | . | . | . | . | . | . | . | . | R | Y | S | . | . | W | - | . | . | . | . | . | . | . | . | . | . |
| 21-225_4C12_HC | . | . | . | . | . | . | . | . | . | K | . | . | . | S | . | . | . | . | . | . | . | . | . | . | . |
| 21-225_4C5_HC | . | . | . | . | . | . | . | . | . | K | . | T | . | S | . | . | . | . | . | . | . | . | . | . | . |
| 21-225_50H4_HC | . | . | . | . | . | . | . | . | . | L | . | . | . | N | . | . | . | . | . | . | . | . | . | . | . |
| 21-225_51E1_HC | . | . | . | . | . | . | . | . | R | Y | S | . | . | W | - | . | . | . | . | . | . | . | . | . | . |
| 21-225_52D10_HC | . | . | . | . | . | . | . | . | R | Y | S | . | . | W | - | . | . | . | . | . | . | . | . | . | . |
| 21-225_52G8_HC | . | . | L | . | . | . | . | . | R | Y | S | . | . | W | - | . | . | . | . | . | . | . | . | . | . |
| 21-225_54D4_HC | . | . | . | . | . | . | . | . | R | Y | S | . | . | W | - | . | . | . | . | . | . | . | . | . | . |
| 21-225_55C2_HC | . | . | . | . | . | . | . | . | R | Y | S | . | . | W | - | . | . | . | . | . | . | . | . | . | . |
| 21-225_55F9_HC | . | . | . | . | . | . | . | . | R | Y | S | . | . | W | - | . | . | . | . | . | . | . | . | . | . |
| 21-225_55H1_HC | . | . | . | . | . | . | . | . | R | Y | S | . | . | W | - | . | . | . | . | . | . | . | . | . | . |
| 21-225_58H11_HC | . | . | . | . | . | . | . | . | R | Y | S | . | . | W | - | . | . | . | . | . | . | . | . | . | . |
| 21-225_59A12_HC | . | . | . | . | . | . | . | . | R | Y | S | . | . | W | - | . | . | . | . | . | . | . | . | . | . |
| 21-225_5E5_HC | . | . | . | . | . | . | T | . | . | V | . | . | . | . | . | . | . | . | . | . | . | . | . | . | . |
| 21-225_5E5.003_HC | . | . | . | . | . | . | T | . | . | V | . | . | . | . | . | . | . | . | . | . | . | . | . | . | . |
| 21-225_5E5.004_HC | . | . | . | . | . | . | T | . | . | V | . | . | . | . | . | . | . | . | . | . | . | . | . | . | . |
| 21-225_5E5.005_HC | . | . | . | . | . | . | T | . | . | V | . | . | . | . | . | . | . | . | . | . | . | . | . | . | . |
| 21-225_5E5.006_HC | . | . | . | . | . | . | T | . | . | V | . | . | . | . | . | . | . | . | . | . | . | . | . | . | . |
| 21-225_5E5.011_HC | . | . | . | . | . | . | T | . | . | V | . | . | . | . | . | . | . | . | . | . | . | . | . | . | . |
| 21-225_5E5.012_HC | . | . | . | . | . | . | T | . | . | V | . | . | . | . | . | . | . | . | . | . | . | . | . | . | . |
| 21-225_5E5.015_HC | . | . | . | . | . | . | T | . | . | V | . | . | . | . | Y | . | . | . | . | . | . | . | . | . | . |
| 21-225_5E5.016_HC | . | . | . | . | . | . | T | . | . | V | . | . | . | . | F | . | . | . | . | . | . | . | . | . | . |
| 21-225_5E5.017_HC | . | . | . | . | . | . | T | . | . | V | . | . | . | . | Y | . | . | . | . | . | . | . | . | . | . |
| 21-225_5E5.018_HC | . | . | . | . | . | . | T | . | . | V | . | . | . | . | Y | . | . | . | . | . | . | . | . | . | . |
| 21-225_5E5.019_HC | . | . | . | . | . | . | T | . | . | V | . | . | . | . | F | . | . | . | . | . | . | . | . | . | . |
| 21-225_5E5.023_HC | . | . | . | . | . | . | T | . | . | V | . | . | . | . | Y | . | . | . | . | . | . | . | . | . | . |

**FIGURE 57**

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | R | E | R | Y | S | S | G | W | - | - | - | - | - | - | - | - | - | - |
| 21-225_5E5.024_HC | . | . | . | . | . | . | T | . | . | V | . | . | . | . | Y | . | . | . | . | . | . | . | . | . | . |
| 21-225_60C12_HC | . | . | . | . | . | . | . | . | R | Y | S | R | . | W | - | . | . | . | . | . | . | . | . | . | . |
| 21-225_60G2_HC | . | . | . | . | . | . | . | . | R | Y | S | R | . | W | - | . | . | . | . | . | . | . | . | . | . |
| 21-225_61B3_HC | . | . | . | . | . | . | . | . | R | Y | S | R | . | W | - | . | . | . | . | . | . | . | . | . | . |
| 21-225_61F2_HC | . | . | . | . | . | . | . | . | . | . | . | . | . | . | . | . | . | . | . | . | . | . | . | . | . |
| 21-225_64A4_HC | . | . | . | . | . | . | . | . | . | . | . | . | . | S | . | . | . | . | . | . | . | . | . | . | . |
| 21-225_64C8_HC | . | . | . | . | . | . | . | . | . | . | . | . | . | . | . | . | . | . | . | . | . | . | . | . | . |
| 21-225_66D11_HC | . | . | L | . | . | . | . | . | . | M | . | . | . | N | . | . | . | . | . | . | . | . | . | . | . |
| 21-225_73C9_HC | . | . | . | . | . | . | . | . | . | . | . | . | . | S | . | . | . | . | . | . | . | . | . | . | . |
| 21-225_7C9_HC | . | . | . | . | . | . | . | . | . | K | . | . | . | S | . | . | . | . | . | . | . | . | . | . | . |
| 21-225_7G4_HC | . | . | . | . | . | . | . | . | . | K | . | . | . | N | . | . | . | . | . | . | . | . | . | . | . |
| 21-225_94F3_HC | . | . | . | . | F | . | . | . | . | K | . | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | H_FR4 | | | | | | | | | | |
| CONSENSUS | - | - | - | - | - | - | Y | D | Y | G | M | D | V | W | G | Q | G | T | T | V | T | V | S | S |
| 21-225_10E9_HC | . | . | . | . | . | . | . | . | . | . | . | . | . | . | . | . | . | . | . | . | . | . | . | . |
| 21-225_147A8_HC | . | . | . | . | . | . | - | T | G | . | . | . | . | . | . | . | . | . | . | . | . | . | . | . |
| 21-225_147B2_HC | . | . | . | . | . | . | - | S | G | . | . | . | . | . | . | . | . | . | . | . | . | . | . | . |
| 21-225_147H5_HC | . | . | . | . | . | . | . | . | . | . | L | . | . | . | . | . | . | . | . | . | . | . | . | . |
| 21-225_148C9_HC | . | . | . | . | . | . | . | . | . | . | L | . | . | . | . | . | . | . | . | . | . | . | . | . |
| 21-225_148F5_HC | . | . | . | . | . | . | - | S | G | . | . | . | . | . | . | . | . | . | . | . | . | . | . | . |
| 21-225_148H10_HC | . | . | . | . | . | . | - | S | G | . | . | . | . | . | . | . | . | . | . | . | . | . | . | . |
| 21-225_149D10_HC | . | . | . | . | . | . | . | . | . | . | . | . | . | . | . | . | . | . | . | . | . | . | . | . |
| 21-225_14E10_HC | . | . | . | . | . | . | . | . | . | . | . | . | . | . | . | . | . | . | . | . | . | . | . | . |
| 21-225_150D11_HC | . | . | . | . | . | . | - | S | G | . | . | . | . | . | . | . | . | . | . | . | . | . | . | . |
| 21-225_150E2_HC | . | . | . | . | . | . | . | G | . | . | . | . | . | . | . | . | . | . | . | . | . | . | . | . |
| 21-225_152F7_HC | . | . | . | . | . | . | . | G | . | . | . | . | . | . | . | . | . | . | . | . | . | . | . | . |
| 21-225_153G9_HC | . | . | . | . | . | . | - | A | G | . | . | . | . | . | . | . | . | . | A | . | . | . | . | . |
| 21-225_154F4_HC | . | . | . | P | Y | Y | . | Y | . | . | . | . | . | . | . | . | . | . | . | . | . | . | . | . |
| 21-225_159D8_HC | . | . | . | . | . | . | . | . | . | . | . | . | . | . | . | . | . | . | . | . | . | . | . | . |
| 21-225_15C11_HC | . | . | . | . | . | . | . | . | . | . | L | . | . | . | . | . | . | . | . | . | . | . | . | . |
| 21-225_169D11_HC | . | . | . | . | . | . | . | . | . | . | . | . | . | . | . | . | . | . | . | . | . | . | . | . |
| 21-225_170F3_HC | . | . | . | . | . | . | . | . | . | . | . | . | . | . | . | . | . | . | . | . | . | . | . | . |
| 21-225_173C11_HC | . | . | . | . | . | . | - | S | G | . | . | . | . | . | . | . | . | . | . | . | . | . | . | . |
| 21-225_174G10_HC | . | . | . | . | . | . | . | . | . | . | . | . | . | . | . | . | . | . | . | . | . | . | . | . |
| 21-225_176H12_HC | . | . | . | . | . | . | . | . | . | . | . | . | . | . | . | . | . | . | . | . | . | . | . | . |
| 21-225_177B4_HC | . | . | . | . | . | . | . | . | . | . | L | . | . | . | . | . | . | . | . | . | . | . | . | . |
| 21-225_179C2_HC | . | . | . | . | . | . | - | S | G | . | . | . | . | . | . | . | . | . | . | . | . | . | . | . |
| 21-225_17H3_HC | . | . | . | . | . | . | . | . | . | . | . | . | . | . | . | . | . | . | . | . | . | . | . | . |
| 21-225_183F4_HC | . | . | . | P | Y | Y | . | Y | . | . | L | G | . | . | . | . | . | . | . | . | . | . | . | . |
| 21-225_190D6_HC | . | . | . | . | . | . | F | G | . | . | . | . | . | . | . | . | . | . | . | . | . | . | . | . |
| 21-225_192A2_HC | . | . | . | . | L | D | . | F | . | . | . | . | . | . | . | . | . | . | . | . | . | . | . | . |
| 21-225_192F6_HC | . | . | . | . | . | . | F | G | . | . | . | . | . | . | . | . | . | . | . | . | . | . | . | . |
| 21-225_193F8_HC | . | . | . | . | L | D | . | Y | . | . | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | | | | | | H_FR4 | | | | | |
| CONSENSUS | - | - | - | - | - | - | Y | D | Y | G | M | D | V | W | G | Q | G | T | T | V | T | V | S | S |
| 21-225_194F10_HC | . | . | . | . | . | . | F | G | . | . | . | . | . | . | . | . | . | . | . | . | . | . | . | . |
| 21-225_201C5_HC | . | . | . | . | . | . | . | . | . | . | . | . | . | . | . | . | . | . | . | . | . | . | . | . |
| 21-225_203F7_HC | . | . | . | . | . | . | . | . | . | . | . | . | . | . | . | . | . | . | . | . | . | . | . | . |
| 21-225_204H3_HC | . | . | . | . | . | . | . | . | . | . | . | . | . | S | . | . | . | . | . | . | . | . | . | . |
| 21-225_208B2_HC | . | . | . | . | . | . | . | . | . | . | L | . | . | . | . | . | . | . | . | . | . | . | . | . |
| 21-225_208F1_HC | . | . | . | . | . | . | . | . | . | . | . | . | . | . | . | . | . | . | . | . | . | . | . | . |
| 21-225_20B2_HC | . | . | . | . | . | . | . | . | . | . | . | . | . | . | . | . | . | . | . | . | . | . | . | . |
| 21-225_210G5_HC | . | . | . | . | . | . | . | . | . | . | L | . | . | . | . | . | . | . | . | . | . | . | . | . |
| 21-225_210G6_HC | . | . | . | . | . | . | . | . | . | . | . | . | . | . | . | . | . | . | . | . | . | . | . | . |
| 21-225_212B11_HC | . | . | . | . | . | . | . | . | . | . | . | . | . | . | . | . | . | . | . | . | . | . | . | . |
| 21-225_214G10_HC | . | . | . | . | . | . | . | . | . | . | . | . | . | . | . | . | . | . | . | . | . | . | . | . |
| 21-225_214G12_HC | . | . | . | . | . | . | . | . | . | . | . | . | . | . | . | . | . | . | . | . | . | . | . | . |
| 21-225_214H9_HC | . | . | . | . | . | . | . | . | . | . | . | . | . | . | . | . | . | . | . | . | . | . | . | . |
| 21-225_215E11_HC | . | . | . | . | . | . | . | . | . | . | . | . | . | . | . | . | . | . | . | . | . | . | . | . |
| 21-225_215E3_HC | . | . | . | . | . | . | . | . | . | . | . | . | . | . | . | . | . | . | . | . | . | . | . | . |
| 21-225_216E8_HC | . | . | . | . | . | . | . | . | . | . | . | . | . | . | . | . | . | . | . | . | . | . | . | . |
| 21-225_216H12_HC | . | . | . | . | . | . | . | . | . | . | . | . | . | . | . | . | . | . | . | . | . | . | . | . |
| 21-225_217E5_HC | . | . | . | . | . | . | . | . | . | . | . | . | . | . | . | . | . | . | . | . | . | . | . | . |
| 21-225_217G10_HC | . | . | . | . | . | . | . | . | . | . | . | . | . | . | . | . | . | . | . | . | . | . | . | . |
| 21-225_217H12_HC | . | . | . | . | . | . | . | . | . | . | . | . | . | . | . | . | . | . | . | . | . | . | . | . |
| 21-225_218C4_HC | . | . | . | . | . | . | . | . | . | . | . | . | . | . | . | . | . | . | . | . | . | . | . | . |
| 21-225_220F8_HC | . | . | . | . | . | . | . | . | . | . | . | . | . | . | . | . | . | . | . | . | . | . | . | . |
| 21-225_224E7_HC | . | . | . | . | . | . | . | . | F | . | L | . | . | . | . | . | . | . | . | . | . | . | . | . |
| 21-225_224F1_HC | . | . | . | . | . | . | . | . | . | . | . | . | . | . | . | . | . | . | . | . | . | . | . | . |
| 21-225_224F3_HC | . | . | . | . | . | . | . | . | . | . | . | . | . | . | . | . | . | . | . | . | . | . | . | . |
| 21-225_225A1_HC | . | . | . | . | . | . | . | . | . | . | . | . | . | . | . | . | . | . | . | . | . | . | . | . |
| 21-225_226C6_HC | . | . | . | . | . | . | . | . | . | . | . | . | . | . | . | . | . | . | . | . | . | . | . | . |
| 21-225_226H11_HC | . | . | . | . | . | . | . | . | . | . | L | . | . | . | . | . | . | . | . | . | . | . | . | . |
| 21-225_22F4_HC | . | . | . | . | . | . | . | . | . | . | . | . | . | . | . | . | . | . | . | . | . | . | . | . |

# FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | | | | | | H_FR4 | | | | | |
| CONSENSUS | - | - | - | - | - | - | Y | D | Y | G | M | D | V | W | G | Q | G | T | T | V | T | V | S | S |
| 21-225_23A10_HC | . | . | . | . | . | . | H | . | . | . | . | . | . | . | . | . | . | . | . | . | . | . | . | . |
| 21-225_24B6_HC | . | . | . | . | . | . | . | . | . | . | . | . | . | . | . | . | . | . | . | . | . | . | . | . |
| 21-225_25A2_HC | . | . | . | . | . | . | - | S | G | . | . | . | . | . | . | . | . | . | . | . | . | . | . | . |
| 21-225_25D12_HC | . | . | . | . | . | . | . | . | . | . | . | . | . | . | . | . | . | . | . | . | . | . | . | . |
| 21-225_26A11_HC | . | . | . | . | . | . | . | . | . | . | . | . | . | . | . | . | . | . | . | . | . | . | . | . |
| 21-225_26G7_HC | . | . | . | . | . | . | - | S | G | . | . | . | . | . | . | . | . | . | . | . | . | . | . | . |
| 21-225_27E7_HC | . | . | . | P | Y | Y | . | Y | . | . | . | . | . | . | . | . | . | . | . | . | . | . | . | . |
| 21-225_28H2_HC | . | . | . | . | . | . | . | . | . | . | . | . | . | . | . | . | . | . | . | . | . | . | . | . |
| 21-225_29E2_HC | . | . | . | . | . | . | . | . | . | . | . | . | . | . | . | . | . | . | . | . | . | . | . | . |
| 21-225_29H4_HC | . | . | . | . | . | . | - | S | G | . | . | . | . | . | . | . | . | . | . | . | . | . | . | . |
| 21-225_2G4_HC | . | . | . | . | . | . | . | . | . | . | . | . | . | . | . | H | . | . | . | . | . | . | . | . |
| 21-225_32H2_HC | . | . | . | . | . | . | - | T | G | . | . | . | . | . | . | . | . | . | . | . | . | . | . | . |
| 21-225_3H10_HC | . | . | . | . | . | . | . | . | . | . | L | . | . | . | . | . | . | . | . | . | . | . | . | . |
| 21-225_43E11_HC | . | . | . | . | . | . | - | V | G | . | . | . | . | . | . | . | . | . | . | . | . | . | . | . |
| 21-225_44B10_HC | . | . | . | . | . | . | - | V | G | . | . | . | . | . | . | . | . | . | . | . | . | . | . | . |
| 21-225_44B3_HC | . | . | . | . | . | . | . | . | . | . | . | . | . | . | . | . | . | . | . | . | . | . | . | . |
| 21-225_44C10_HC | . | . | . | . | . | . | . | . | . | . | . | . | . | . | . | . | . | . | . | . | . | . | . | . |
| 21-225_44D3_HC | . | . | . | . | . | . | . | . | . | . | . | . | . | . | . | . | . | . | . | . | . | . | . | . |
| 21-225_44D5_HC | . | . | . | . | . | . | . | . | . | . | . | . | . | . | . | . | . | . | . | . | . | . | . | . |
| 21-225_44F9_HC | . | . | . | . | . | . | . | . | . | . | . | . | . | G | . | . | . | . | . | . | . | . | . | . |
| 21-225_45B4_HC | . | . | . | . | . | . | . | . | . | . | . | . | . | . | . | . | . | . | . | . | . | . | . | . |
| 21-225_45B8_HC | . | . | . | . | . | . | . | . | . | . | . | . | . | . | . | . | . | . | . | . | . | . | . | . |
| 21-225_46B9_HC | . | . | . | . | . | . | - | M | G | . | . | . | . | . | . | . | . | . | . | . | . | . | . | . |
| 21-225_46C3_HC | . | . | . | . | . | . | . | . | . | . | . | . | . | . | . | . | . | . | . | . | . | . | . | . |
| 21-225_46D4_HC | . | . | . | . | . | . | . | . | . | . | . | . | . | . | . | . | . | . | . | . | . | . | . | . |
| 21-225_47C1_HC | . | . | . | . | . | . | - | V | G | . | . | . | . | . | . | . | . | . | . | . | . | . | . | . |
| 21-225_47E7_HC | . | . | . | . | . | . | - | V | G | . | . | . | . | . | . | . | . | . | . | . | . | . | . | . |
| 21-225_48F2_HC | . | . | . | . | . | . | . | . | . | . | L | . | . | . | . | . | . | . | . | . | . | . | . | . |
| 21-225_48H4_HC | . | . | . | . | . | . | . | G | . | . | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | | | | | | H_FR4 | | | | | |
| CONSENSUS | - | - | - | - | - | - | Y | D | Y | G | M | D | V | W | G | Q | G | T | T | V | T | V | S | S |
| 21-225_49C1_HC | . | . | . | . | . | . | - | S | G | . | . | . | . | . | . | . | . | . | . | . | . | . | . | . |
| 21-225_49E7_HC | . | . | . | . | . | . | - | S | G | . | . | . | . | . | . | . | . | . | . | . | . | . | . | . |
| 21-225_49F9_HC | . | . | . | . | . | . | - | S | G | . | . | . | . | . | . | . | . | . | . | . | . | . | . | . |
| 21-225_49G3_HC | . | . | . | . | . | . | - | S | G | . | . | . | . | . | . | . | . | . | . | . | . | . | . | . |
| 21-225_4C12_HC | . | . | . | . | . | . | . | . | . | . | . | . | . | . | . | H | . | . | . | . | . | . | . | . |
| 21-225_4C5_HC | . | . | . | . | . | . | . | . | . | . | . | . | . | . | . | . | . | . | . | . | . | . | . | . |
| 21-225_50H4_HC | . | . | . | . | . | . | . | . | . | . | . | . | . | . | . | . | . | . | . | . | . | . | . | . |
| 21-225_51E1_HC | . | . | . | . | . | . | - | S | G | . | . | . | . | . | . | . | . | . | . | . | . | . | . | . |
| 21-225_52D10_HC | . | . | . | . | . | . | - | S | G | . | . | . | . | . | . | . | . | . | . | . | . | . | . | . |
| 21-225_52G8_HC | . | . | . | . | . | . | - | S | G | . | . | . | . | . | . | . | . | . | . | . | . | . | . | . |
| 21-225_54D4_HC | . | . | . | . | . | . | - | S | G | . | . | . | . | . | . | . | . | . | . | . | . | . | . | . |
| 21-225_55C2_HC | . | . | . | . | . | . | - | S | G | . | . | . | . | . | . | . | . | . | . | . | . | . | . | . |
| 21-225_55F9_HC | . | . | . | . | . | . | - | S | G | . | . | . | . | . | . | . | . | . | . | . | . | . | . | . |
| 21-225_55H1_HC | . | . | . | . | . | . | - | S | G | . | . | . | . | . | . | . | . | . | . | . | . | . | . | . |
| 21-225_58H11_HC | . | . | . | . | . | . | - | . | G | . | . | . | . | . | . | . | . | . | . | . | . | . | . | . |
| 21-225_59A12_HC | . | . | . | . | . | . | - | . | G | . | . | . | . | . | . | . | . | . | . | . | . | . | . | . |
| 21-225_5E5_HC | . | . | . | . | . | . | . | . | . | . | . | . | . | . | . | . | . | . | . | . | . | . | . | . |
| 21-225_5E5.003_HC | . | . | . | . | . | . | . | . | . | . | . | . | . | . | . | . | . | . | . | . | . | . | . | . |
| 21-225_5E5.004_HC | . | . | . | . | . | . | . | . | . | . | . | . | . | . | . | . | . | . | . | . | . | . | . | . |
| 21-225_5E5.005_HC | . | . | . | . | . | . | . | . | . | . | . | . | . | . | . | . | . | . | . | . | . | . | . | . |
| 21-225_5E5.006_HC | . | . | . | . | . | . | . | . | . | . | . | . | . | . | . | . | . | . | . | . | . | . | . | . |
| 21-225_5E5.011_HC | . | . | . | . | . | . | . | . | . | . | . | . | . | . | . | . | . | . | . | . | . | . | . | . |
| 21-225_5E5.012_HC | . | . | . | . | . | . | . | . | . | . | . | . | . | . | . | . | . | . | . | . | . | . | . | . |
| 21-225_5E5.015_HC | . | . | . | . | . | . | . | . | . | . | . | . | . | . | . | . | . | . | . | . | . | . | . | . |
| 21-225_5E5.016_HC | . | . | . | . | . | . | . | . | . | . | . | . | . | . | . | . | . | . | . | . | . | . | . | . |
| 21-225_5E5.017_HC | . | . | . | . | . | . | . | . | . | . | . | . | . | . | . | . | . | . | . | . | . | . | . | . |
| 21-225_5E5.018_HC | . | . | . | . | . | . | . | . | . | . | . | . | . | . | . | . | . | . | . | . | . | . | . | . |
| 21-225_5E5.019_HC | . | . | . | . | . | . | . | . | . | . | . | . | . | . | . | . | . | . | . | . | . | . | . | . |
| 21-225_5E5.023_HC | . | . | . | . | . | . | . | . | . | . | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | H_FR4 | | | | | | | | | | |
| CONSENSUS | - | - | - | - | - | - | Y | D | Y | G | M | D | V | W | G | Q | G | T | T | V | T | V | S | S |
| 21-225_5E5.024_HC | . | . | . | . | . | . | . | . | . | . | . | . | . | . | . | . | . | . | . | . | . | . | . | . |
| 21-225_60C12_HC | . | . | . | . | . | . | - | T | G | . | . | . | . | . | . | . | . | . | . | . | . | . | . | . |
| 21-225_60G2_HC | . | . | . | . | . | . | - | T | G | . | . | . | . | . | . | . | . | . | . | . | . | . | . | . |
| 21-225_61B3_HC | . | . | . | . | . | . | - | A | G | . | . | . | . | . | . | . | . | . | . | . | . | . | . | . |
| 21-225_61F2_HC | . | . | . | . | . | . | . | . | . | . | . | . | . | . | . | . | . | . | . | . | . | . | . | . |
| 21-225_64A4_HC | . | . | . | . | . | . | . | . | . | . | . | . | . | . | . | . | . | . | . | . | . | . | . | . |
| 21-225_64C8_HC | . | . | . | . | . | . | . | . | . | . | . | . | . | . | . | . | . | . | . | . | . | . | . | . |
| 21-225_66D11_HC | . | . | . | . | . | . | . | . | . | . | L | . | . | . | . | . | . | . | . | . | . | . | . | . |
| 21-225_73C9_HC | . | . | . | . | . | . | F | . | . | . | . | . | . | . | . | . | . | . | . | . | . | . | . | . |
| 21-225_7C9_HC | . | . | . | . | . | . | . | . | . | . | . | . | . | . | . | . | . | . | . | . | . | . | . | . |
| 21-225_7G4_HC | . | . | . | . | . | . | . | . | . | . | . | . | . | . | . | . | . | . | . | . | . | . | . | . |
| 21-225_94F3_HC | . | . | . | . | . | . | . | . | . | . | . | . | . | . | . | . | . | . | . | . | . | . | . | . |

**FIGURE 57**

Table 51. Consensus 17- VH3|3-33/D7|7-27|RF2/JH4 (SEQ ID NO: 50268):

QVQLVES-GGGVVQPGRSLRLSCAASG-FTFSD-----YGMHWVRQAPGKGLEWVAVIWYD---ENNKYYADSVKGRFTISRDNSKNTLYLQMNSLRAEDTAVYYCARELGF---------------------- SDYWGQGTLVTVSS wherein:

D at position 33 can be substituted with S, N or T

Y at position 39 can be substituted with F

M at position 41 can be substituted with I

V at position 57 can be substituted with L

I at position 58 can be substituted with V, T or M

Y at position 60 can be substituted with F or D

D at position 61 can be substituted with E, A, G or N

E at position 65 can be substituted with G, V, R or D

N at position 66 can be substituted with S, T, D, I or Y

N at position 67 can be substituted with H or K

K at position 68 can be substituted with Q, E, N or R

Y at position 69 can be substituted with H, K, D, R or S

Y at position 70 can be substituted with H

A at position 71 can be substituted with V, G, T, I or E

D at position 72 can be substituted with E

V at position 74 can be substituted with M

K at position 75 can be substituted with R

**FIGURE 57**

E at position 109 can be substituted with D or G

G at position 111 can be substituted with A

F at position 112 can be substituted with W or M

L at position 135 can be substituted with R, T, Y, S, Q, I, A, E or N

S at position 136 can be substituted with E, G, D, F, N, A or T

D at position 137 can be substituted with E

Y at position 138 can be substituted with S, F or C

Xaa Xaa Gly Xaa His (SEQ ID NO: 50007)

Xaa Xaa Trp Xaa Xaa Xaa Xaa Xaa Xaa Xaa Xaa Xaa Xaa Ser Xaa Xaa Gly (SEQ ID NO: 50008)

Xaa Leu Xaa Xaa Xaa Xaa Xaa Xaa (SEQ ID NO: 50009)

Asp Tyr Gly Met His (SEQ ID NO: 50474)

Val Ile Trp Tyr Asp Glu Asn Asn Lys Tyr Tyr Ala Asp Ser Val Lys Gly (SEQ ID NO: 50475)

Glu Leu Gly Phe Ser Asp Tyr (SEQ ID NO: 50476)

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | E | S | - | G | G | G | V | V | Q | P | G | R | S | L | R | L | S | C | A | A | S |
| 21-225_10G5_HC | . | . | . | . | . | . | . | . | . | . | . | . | . | . | . | . | . | . | . | . | . | . | . | . | . | . |
| 21-225_10H12_HC | . | . | . | . | . | . | . | . | . | . | . | . | . | . | . | . | . | . | . | . | . | . | . | . | . | . |
| 21-225_12A1_HC | . | . | . | . | . | . | . | . | . | . | . | . | . | . | . | . | . | . | . | . | . | . | . | . | . | . |
| 21-225_12B12_HC | . | . | . | . | . | . | . | . | . | . | . | . | . | . | . | . | . | . | . | . | . | . | . | . | . | . |
| 21-225_12E6_HC | . | . | . | . | . | . | . | . | . | . | . | . | . | . | . | . | . | . | . | . | . | . | . | . | . | . |
| 21-225_12F2_HC | . | . | . | . | . | . | . | . | . | . | . | . | . | . | . | . | . | . | . | . | . | . | . | . | . | . |
| 21-225_13E6_HC | . | . | . | . | . | . | . | . | . | . | . | . | . | . | . | . | . | . | . | . | . | . | . | . | . | . |
| 21-225_146H9_HC | . | . | . | . | . | . | . | . | . | . | . | . | . | . | . | . | . | . | . | . | . | . | . | . | . | . |
| 21-225_14E3_HC | . | . | . | . | . | . | . | . | . | . | . | . | . | . | . | . | . | . | . | . | . | . | . | . | . | . |
| 21-225_157C3_HC | . | . | . | . | . | . | . | . | . | . | . | . | . | . | . | . | . | . | . | . | . | . | . | . | . | . |
| 21-225_157H8_HC | . | . | . | . | . | . | . | . | . | . | . | . | . | . | . | . | . | . | . | . | . | . | . | . | . | . |
| 21-225_158H6_HC | . | . | . | . | . | . | . | . | . | . | . | . | . | . | . | . | . | . | . | . | . | . | . | . | V | . |
| 21-225_159C5_HC | . | . | . | . | . | . | . | . | . | . | . | . | . | . | . | . | . | . | . | . | . | . | . | . | . | . |
| 21-225_15E3_HC | . | . | . | . | . | . | . | . | . | . | T | . | . | . | . | . | . | . | . | . | . | . | . | . | . | . |
| 21-225_16A5_HC | . | . | . | . | . | . | . | . | . | . | . | . | . | . | . | . | . | . | . | . | . | . | . | . | . | . |
| 21-225_16B7_HC | . | . | . | . | . | . | . | . | . | . | . | . | . | . | . | . | . | . | . | . | . | . | . | . | . | . |
| 21-225_16F10_HC | . | . | . | . | . | . | . | . | . | . | . | . | . | . | . | . | . | . | . | . | . | . | . | . | . | . |
| 21-225_16F11_HC | . | . | . | M | . | . | . | . | . | . | . | . | . | . | . | . | . | . | . | . | . | . | . | E | . | . |
| 21-225_16F4_HC | . | . | . | . | . | . | . | . | . | . | . | . | . | . | . | . | . | . | . | . | . | . | . | . | . | . |
| 21-225_16F6_HC | . | . | . | . | . | . | . | . | . | . | . | . | . | . | . | . | . | . | . | . | . | . | . | . | . | . |
| 21-225_16G7_HC | . | . | . | . | . | . | . | . | . | . | . | . | . | . | . | . | . | . | . | . | . | . | . | . | . | . |
| 21-225_17A10_HC | . | . | . | . | . | . | . | . | . | . | . | . | . | . | . | . | . | . | . | . | . | . | . | . | . | . |
| 21-225_17B5_HC | . | . | . | . | . | . | . | . | . | . | . | . | . | . | . | . | . | . | . | . | . | . | . | . | . | . |
| 21-225_17C7_HC | . | . | . | . | . | . | . | . | . | . | . | . | . | . | . | . | K | . | . | . | . | . | . | . | . | . |
| 21-225_17E5_HC | . | . | . | . | . | . | . | . | . | . | . | . | . | . | . | . | . | . | . | . | . | . | . | . | . | . |
| 21-225_18A1_HC | . | . | . | . | . | . | . | . | . | . | . | . | . | . | . | . | . | . | . | . | . | . | . | . | V | . |
| 21-225_18A5_HC | . | . | . | . | . | . | . | . | . | . | . | . | . | . | . | . | . | . | . | . | . | . | Y | T | . | . |
| 21-225_18C2_HC | . | . | . | . | . | . | . | . | . | . | . | . | . | . | . | . | . | . | . | . | . | . | . | V | . | . |
| 21-225_18E8_HC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | E | S | - | G | G | G | V | V | Q | P | G | R | S | L | R | L | S | C | A | A | S |
| 21-225_18F2_HC | . | . | . | . | . | . | . | . | . | . | . | . | . | . | . | . | . | . | . | . | . | . | . | . | . | . |
| 21-225_19E1_HC | . | . | . | . | . | . | . | . | . | . | . | . | . | . | . | . | . | . | . | . | . | . | . | . | . | . |
| 21-225_1C12_HC | . | . | . | . | . | . | . | . | . | . | . | . | . | . | . | . | . | . | . | . | . | . | . | . | . | . |
| 21-225_203B9_HC | . | . | . | . | . | . | . | . | . | . | . | . | . | . | . | . | . | . | . | . | . | . | . | . | . | . |
| 21-225_207F12_HC | . | . | . | . | . | . | . | . | . | . | . | . | . | . | . | . | . | . | . | . | . | . | . | . | . | . |
| 21-225_20A7_HC | . | . | . | . | . | . | . | . | . | . | . | . | . | . | . | . | . | . | . | . | . | . | . | . | . | . |
| 21-225_20D10_HC | . | . | . | . | . | . | . | . | . | . | . | . | . | . | . | . | . | . | . | . | . | . | . | . | . | . |
| 21-225_20D5_HC | . | . | . | . | . | . | . | . | . | . | . | . | . | . | . | . | . | . | . | . | . | . | . | . | . | . |
| 21-225_20E12_HC | . | . | . | . | . | . | . | . | . | . | . | . | . | . | . | . | . | . | . | . | . | . | . | . | . | . |
| 21-225_20E5_HC | . | . | . | . | . | . | . | . | . | . | . | . | . | . | . | . | . | . | . | . | . | . | . | . | . | . |
| 21-225_20H12_HC | . | . | . | . | . | . | . | . | . | . | . | . | . | . | . | . | . | . | . | . | . | . | . | . | . | . |
| 21-225_21E5_HC | . | . | . | . | . | . | . | . | . | . | . | . | . | . | . | . | . | . | . | . | . | . | . | S | . | . |
| 21-225_21F7_HC | . | . | . | . | . | . | . | . | . | . | . | . | . | . | . | . | . | . | . | . | . | . | . | . | . | . |
| 21-225_21G11_HC | . | . | . | . | . | . | . | . | . | . | . | . | . | . | . | . | . | . | . | . | . | . | . | . | . | . |
| 21-225_21G2_HC | . | . | . | . | . | . | . | . | . | . | . | . | . | . | . | . | . | . | . | . | . | . | . | . | . | . |
| 21-225_21H8_HC | . | . | . | . | . | . | . | . | . | . | . | . | . | . | S | . | . | . | . | . | . | . | . | . | . | . |
| 21-225_224D10_HC | . | . | . | . | . | . | . | . | . | . | . | L | . | . | . | . | K | . | . | . | . | . | . | . | . | . |
| 21-225_22A4_HC | . | . | . | . | . | . | . | . | . | . | . | . | . | . | . | . | . | . | . | . | . | . | . | . | . | . |
| 21-225_22F2_HC | . | . | . | . | . | . | . | . | . | . | . | . | . | . | . | . | . | . | . | . | . | . | . | . | . | . |
| 21-225_22F9_HC | . | . | . | M | . | . | . | . | . | . | . | . | . | . | . | . | . | . | . | . | . | . | . | . | . | . |
| 21-225_22H8_HC | . | . | . | . | . | . | . | . | . | . | . | . | . | . | . | . | . | . | . | . | . | . | . | . | . | . |
| 21-225_23F12_HC | . | . | . | . | . | . | . | . | . | . | . | . | . | . | . | . | . | . | . | . | . | . | . | V | . | . |
| 21-225_23H11_HC | . | E | . | . | . | . | . | . | . | . | . | . | . | . | . | . | . | . | . | . | . | . | . | . | . | . |
| 21-225_25B6_HC | . | . | . | . | . | . | . | . | . | . | . | . | . | . | . | . | . | . | . | . | V | . | . | T | . | . |
| 21-225_25E6_HC | . | . | . | . | . | . | . | . | . | . | . | . | . | . | . | . | . | . | . | . | . | . | . | . | . | . |
| 21-225_25H10_HC | . | . | . | . | . | . | . | . | . | . | . | . | . | . | . | . | . | . | . | . | . | . | . | . | . | . |
| 21-225_25H11_HC | . | . | . | . | . | . | . | . | . | . | . | . | . | . | . | . | . | . | . | . | . | . | . | . | . | . |
| 21-225_26A2_HC | . | . | . | . | . | . | . | . | . | . | . | . | . | . | . | . | . | . | . | . | . | . | . | . | . | . |
| 21-225_27C3_HC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | E | S | - | G | G | G | V | V | Q | P | G | R | S | L | R | L | S | C | A | A | S |
| 21-225_27D5_HC | . | . | . | . | . | . | . | . | . | . | . | . | . | . | . | . | . | . | . | . | . | . | . | . | . | . |
| 21-225_27F2_HC | . | [illegible] | . | . | . | . | . | . | . | . | . | . | . | . | . | . | . | . | . | . | . | . | . | . | . | . |
| 21-225_28C7_HC | . | . | . | . | . | . | . | . | . | . | . | . | . | . | . | . | . | . | . | . | . | . | . | . | . | . |
| 21-225_28F5_HC | . | . | . | . | . | . | . | . | . | . | . | . | . | . | . | . | . | . | . | . | . | . | . | . | . | . |
| 21-225_29B8_HC | . | . | . | . | . | . | . | . | . | . | . | . | . | . | . | . | . | . | . | . | . | . | . | . | . | . |
| 21-225_29D7_HC | . | . | . | . | . | . | . | . | . | . | . | . | . | . | . | . | . | . | . | . | . | . | . | . | . | . |
| 21-225_29G4_HC | . | . | . | . | . | . | . | . | . | . | . | . | . | . | . | . | . | . | . | . | . | T | . | . | . | . |
| 21-225_29G8_HC | . | . | . | . | . | . | . | . | . | . | . | . | . | . | . | . | . | . | . | . | . | . | . | . | . | . |
| 21-225_29H6_HC | . | . | . | . | . | . | . | . | . | . | . | . | . | . | . | . | . | . | . | . | . | . | . | . | . | . |
| 21-225_2B2_HC | . | . | . | . | . | . | . | . | . | . | . | . | . | . | . | . | . | . | . | . | . | . | . | . | . | . |
| 21-225_43C3_HC | . | . | . | . | . | . | . | . | . | . | . | . | . | . | . | . | . | . | . | . | . | . | . | . | . | . |
| 21-225_44C3_HC | . | . | . | . | . | . | . | . | . | . | . | . | . | . | . | . | . | . | . | . | . | . | . | . | . | . |
| 21-225_44C8_HC | . | . | . | . | . | . | . | . | . | . | . | . | . | . | . | . | . | . | . | . | . | . | . | . | . | . |
| 21-225_46C6_HC | . | . | . | . | . | . | . | . | . | . | . | . | . | . | . | . | . | . | . | . | . | . | . | . | . | . |
| 21-225_46D8_HC | . | . | . | . | . | . | . | . | . | . | . | . | . | . | . | . | . | . | . | . | . | . | . | . | . | . |
| 21-225_46F3_HC | . | . | . | . | . | . | . | . | . | . | . | . | . | . | . | . | . | . | . | . | . | . | . | . | . | . |
| 21-225_47A1_HC | . | . | . | . | . | . | . | . | . | . | . | . | . | . | . | . | . | . | . | . | . | . | . | . | . | . |
| 21-225_48A9_HC | . | . | . | . | . | . | . | . | . | . | . | . | . | . | . | . | . | . | . | . | . | . | . | . | . | . |
| 21-225_48C1_HC | . | . | . | . | . | . | . | . | . | . | . | . | . | . | . | . | . | . | . | . | . | . | . | . | . | . |
| 21-225_48D12_HC | . | . | . | . | . | . | . | . | . | . | . | . | . | . | . | . | . | . | . | . | . | . | . | . | . | . |
| 21-225_49A1_HC | . | . | . | . | . | . | . | . | . | . | . | . | . | . | . | . | . | . | . | . | . | . | . | . | . | . |
| 21-225_49C6_HC | . | . | . | . | . | . | . | . | . | . | . | . | . | . | . | . | . | . | . | . | . | . | . | . | . | . |
| 21-225_51B11_HC | . | . | . | . | . | . | . | . | . | . | . | . | . | . | . | . | . | . | . | . | . | . | . | . | . | . |
| 21-225_51E4_HC | . | . | . | . | . | . | . | . | . | . | . | . | . | . | . | . | . | . | . | . | . | . | . | . | . | . |
| 21-225_51F11_HC | . | . | . | . | . | . | . | . | . | . | . | . | . | . | . | . | . | . | . | . | . | . | . | . | . | . |
| 21-225_51F9_HC | . | . | . | . | . | . | . | . | . | . | . | . | . | . | . | . | . | . | . | . | . | . | . | . | . | . |
| 21-225_52B2_HC | . | . | . | . | . | . | . | . | . | . | . | . | . | . | . | . | . | . | . | . | . | . | . | . | . | . |
| 21-225_52B9_HC | . | . | . | . | . | . | . | . | . | . | . | . | . | . | . | . | . | . | . | . | . | . | . | . | . | . |
| 21-225_53B12_HC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 |
|---|---|---|---|---|---|---|---|---|---|---|---|---|---|
| | H_FR1 | | | | | | | | | | | | |
| CONSENSUS | Q | V | Q | L | V | E | S | - | G | G | G | V | V |
| 21-225_53D2_HC | . | . | . | . | . | . | . | . | . | . | . | . | . |
| 21-225_53E6_HC | . | . | . | . | . | . | . | . | . | . | . | . | . |
| 21-225_54C6_HC | . | . | . | . | . | . | . | . | . | . | . | . | . |
| 21-225_54D3_HC | . | . | . | . | . | . | . | . | . | . | . | . | . |
| 21-225_54G7_HC | . | . | . | . | . | . | . | . | . | . | . | . | . |
| 21-225_55B3_HC | . | . | . | . | . | . | . | . | . | . | . | . | . |
| 21-225_57A4_HC | . | . | . | . | . | . | . | . | . | . | . | . | . |
| 21-225_58F5_HC | . | . | . | . | . | . | . | . | . | . | . | . | . |
| 21-225_59F11_HC | . | . | . | . | . | . | . | . | . | . | . | . | . |
| 21-225_60A3_HC | . | . | . | . | . | . | . | . | . | . | . | . | . |
| 21-225_62E4_HC | . | . | . | . | . | . | . | . | . | . | . | . | . |
| 21-225_64E1_HC | . | . | . | . | . | . | . | . | . | . | . | . | . |
| 21-225_65C1_HC | . | . | . | . | . | . | . | . | . | . | . | . | . |
| 21-225_66C7_HC | . | . | . | . | . | . | . | . | . | . | . | . | . |
| 21-225_68F11_HC | . | . | . | . | . | . | . | . | . | . | . | . | . |
| 21-225_6A11_HC | . | . | . | . | . | . | . | . | . | . | . | . | . |
| 21-225_6G12_HC | . | . | . | . | . | . | . | . | . | . | . | . | . |
| 21-225_6G2_HC | L | . | . | . | . | . | . | . | . | . | . | . | . |
| 21-225_71A2_HC | . | . | . | . | . | . | . | . | . | . | . | . | . |
| 21-225_71B11_HC | . | . | . | . | . | . | . | . | . | . | . | . | . |
| 21-225_71B6_HC | . | . | . | . | . | . | . | . | . | . | . | . | . |
| 21-225_72G12_HC | . | . | . | . | . | . | . | . | . | . | . | . | . |
| 21-225_7C4_HC | . | . | . | . | . | . | . | . | . | . | . | . | . |
| 21-225_7E9_HC | . | . | . | . | . | . | . | . | . | . | . | . | . |
| 21-225_7G2_HC | . | . | . | . | . | . | . | . | . | . | . | . | . |
| 21-225_7H11_HC | . | . | . | . | . | . | . | . | . | . | . | . | . |
| 21-225_8C9_HC | . | . | . | . | . | . | . | . | . | . | . | . | . |
| 21-225_8H7_HC | . | . | . | . | . | . | . | . | . | . | . | . | . |
| 21-225_9A1_HC | . | . | . | V | . | . | . | . | . | . | . | . | . |

| Reference # | 14 | 15 | 16 | 17 | 18 | 19 | 20 | 21 | 22 | 23 | 24 | 25 | 26 |
|---|---|---|---|---|---|---|---|---|---|---|---|---|---|
| | | | | | | | | | | | | | |
| CONSENSUS | Q | P | G | R | S | L | R | L | S | C | A | A | S |
| 21-225_53D2_HC | . | . | . | . | . | . | . | . | . | . | . | . | . |
| 21-225_53E6_HC | . | . | . | . | . | . | . | . | . | . | T | T | . |
| 21-225_54C6_HC | . | . | . | . | . | . | . | . | . | . | T | T | . |
| 21-225_54D3_HC | . | . | . | . | . | . | . | . | . | . | . | . | . |
| 21-225_54G7_HC | . | . | . | . | . | . | . | . | . | . | . | . | . |
| 21-225_55B3_HC | . | . | . | . | . | . | . | . | . | . | . | . | . |
| 21-225_57A4_HC | . | . | . | . | . | . | . | . | . | . | V | . | . |
| 21-225_58F5_HC | . | . | . | . | . | . | . | . | . | . | V | . | . |
| 21-225_59F11_HC | . | . | . | . | . | . | . | . | . | . | . | . | . |
| 21-225_60A3_HC | . | . | . | . | . | . | . | . | . | . | . | . | . |
| 21-225_62E4_HC | . | . | . | . | . | . | . | . | . | . | . | . | . |
| 21-225_64E1_HC | . | . | . | . | . | . | . | . | . | . | V | . | . |
| 21-225_65C1_HC | . | . | . | . | . | . | . | . | . | . | . | . | . |
| 21-225_66C7_HC | . | . | . | . | . | . | . | . | . | . | V | . | . |
| 21-225_68F11_HC | . | . | . | . | . | . | . | . | . | . | . | . | . |
| 21-225_6A11_HC | . | . | . | . | . | . | . | . | . | . | . | . | . |
| 21-225_6G12_HC | . | . | . | . | . | . | . | . | . | . | . | . | . |
| 21-225_6G2_HC | . | . | . | K | . | . | . | . | . | . | . | . | . |
| 21-225_71A2_HC | . | . | . | . | . | . | . | . | . | . | . | T | . |
| 21-225_71B11_HC | . | . | . | . | . | . | . | . | . | . | . | . | . |
| 21-225_71B6_HC | . | . | . | . | . | . | . | . | . | . | . | . | . |
| 21-225_72G12_HC | . | . | . | . | . | . | . | . | . | . | . | . | . |
| 21-225_7C4_HC | . | . | . | K | . | . | . | . | . | . | . | . | . |
| 21-225_7E9_HC | . | . | . | . | . | . | . | . | . | . | . | . | . |
| 21-225_7G2_HC | . | . | . | . | . | . | . | . | . | . | . | . | . |
| 21-225_7H11_HC | . | . | . | . | . | . | . | . | . | . | S | . | . |
| 21-225_8C9_HC | . | . | . | . | . | . | . | . | . | . | . | . | . |
| 21-225_8H7_HC | . | . | . | . | . | . | . | . | . | . | P | . | . |
| 21-225_9A1_HC | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | E | S | - | G | G | G | V | V | Q | P | G | R | S | L | R | L | S | C | A | A | S |
| 21-225_9E8_HC | . | . | . | . | . | . | . | . | . | . | . | . | . | . | . | . | . | . | . | . | . | . | . | [illegible] | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | D | - | - | - | - | - | Y | G | M | H | W | V | R | Q | A | P | G | K | G | L |
| 21-225_10G5_HC | . | . | . | . | . | . | N | . | . | . | . | . | . | . | . | . | . | . | . | . | . | . | . | . | . | . |
| 21-225_10H12_HC | . | . | . | . | . | . | . | . | . | . | . | . | . | . | . | . | . | . | . | . | . | . | . | . | . | . |
| 21-225_12A1_HC | . | . | . | . | . | . | S | . | . | . | . | . | . | . | . | . | . | . | . | . | . | . | . | . | . | . |
| 21-225_12B12_HC | . | . | . | . | . | . | . | . | . | . | . | . | . | . | . | . | . | . | . | . | . | . | . | . | . | . |
| 21-225_12E6_HC | . | . | . | . | . | . | . | . | . | . | . | . | . | . | . | . | . | . | . | . | . | . | . | . | . | . |
| 21-225_12F2_HC | . | . | . | . | . | . | . | . | . | . | . | . | . | . | . | . | . | . | . | . | . | . | . | . | . | . |
| 21-225_13E6_HC | . | . | . | . | . | . | N | . | . | . | . | . | . | . | . | . | . | . | . | . | . | . | . | . | . | . |
| 21-225_146H9_HC | . | . | . | S | . | . | S | . | . | . | . | . | . | . | . | . | . | . | . | . | . | . | . | . | . | . |
| 21-225_14E3_HC | . | . | . | . | . | . | . | . | . | . | . | . | F | . | . | . | . | . | . | . | . | . | . | . | . | . |
| 21-225_157C3_HC | . | . | . | . | . | . | T | . | . | . | . | . | . | . | . | . | . | . | . | . | . | . | . | . | . | . |
| 21-225_157H8_HC | . | . | . | . | . | . | S | . | . | . | . | . | . | . | . | . | . | . | . | . | . | . | . | . | . | . |
| 21-225_158H6_HC | . | . | . | . | . | . | S | . | . | . | . | . | . | . | . | . | . | . | . | . | . | . | . | . | . | . |
| 21-225_159C5_HC | . | . | . | . | . | . | S | . | . | . | . | . | . | . | I | . | . | . | . | . | . | . | . | . | . | . |
| 21-225_15E3_HC | . | . | . | . | . | . | S | . | . | . | . | . | . | . | . | . | . | . | . | . | . | . | . | . | . | . |
| 21-225_16A5_HC | . | . | . | . | . | . | . | . | . | . | . | . | . | . | . | . | . | . | . | . | . | . | . | . | . | . |
| 21-225_16B7_HC | . | . | . | . | . | . | S | . | . | . | . | . | . | . | . | . | . | . | . | . | . | . | . | . | . | . |
| 21-225_16F10_HC | . | . | . | . | . | . | . | . | . | . | . | . | . | . | . | . | . | . | . | . | . | . | . | . | . | . |
| 21-225_16F11_HC | . | . | . | . | . | . | S | . | . | . | . | . | . | . | . | . | . | . | . | . | . | . | . | . | . | . |
| 21-225_16F4_HC | . | . | . | . | . | . | N | . | . | . | . | . | . | . | . | . | . | . | . | . | . | . | . | . | . | . |
| 21-225_16F6_HC | . | . | . | . | . | . | N | . | . | . | . | . | . | . | . | . | R | . | . | . | . | . | . | . | . | . |
| 21-225_16G7_HC | . | . | . | . | . | T | . | . | . | . | . | . | . | . | . | . | . | . | . | . | . | . | . | . | . | . |
| 21-225_17A10_HC | . | . | . | . | . | . | N | . | . | . | . | . | . | . | . | . | . | . | . | . | . | . | A | . | . | . |
| 21-225_17B5_HC | . | . | . | . | . | . | . | . | . | . | . | . | . | . | . | . | . | . | . | . | . | . | . | . | . | . |
| 21-225_17C7_HC | . | . | . | . | . | . | . | . | . | . | . | . | . | . | . | . | . | . | . | . | . | . | . | . | . | . |
| 21-225_17E5_HC | . | . | . | . | . | . | . | . | . | . | . | . | . | . | . | . | . | . | . | . | . | . | . | . | . | . |
| 21-225_18A1_HC | . | . | . | . | . | . | S | . | . | . | . | . | . | . | . | . | . | . | . | . | . | . | . | . | . | . |
| 21-225_18A5_HC | . | . | . | . | . | . | . | . | . | . | . | . | . | . | . | . | . | . | . | . | . | . | . | . | . | . |
| 21-225_18C2_HC | . | . | . | . | . | . | . | . | . | . | . | . | . | . | . | . | . | . | . | . | . | . | . | . | . | . |
| 21-225_18E8_HC | . | . | . | . | . | . | N | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | D | - | - | - | - | - | Y | G | M | H | W | V | R | Q | A | P | G | K | G | L |
| 21-225_18F2_HC | . | . | . | . | . | . | . | . | . | . | . | . | . | . | . | . | . | . | . | . | . | . | . | . | . | . |
| 21-225_19E1_HC | . | . | . | . | . | . | N | . | . | . | . | . | . | . | . | . | . | . | . | . | . | . | . | . | . | . |
| 21-225_1C12_HC | . | . | . | . | . | . | N | . | . | . | . | . | . | . | . | . | . | . | . | . | . | . | . | . | . | . |
| 21-225_203B9_HC | . | . | . | . | L | . | S | . | . | . | . | . | F | . | . | . | . | . | . | . | . | . | . | . | . | . |
| 21-225_207F12_HC | . | . | . | . | . | . | N | . | . | . | . | . | . | . | . | . | . | . | . | . | . | . | . | . | . | . |
| 21-225_20A7_HC | . | . | . | . | . | . | N | . | . | . | . | . | . | . | . | . | . | . | . | . | . | . | . | . | . | . |
| 21-225_20D10_HC | . | . | . | . | . | . | . | . | . | . | . | . | . | . | . | . | . | . | . | . | . | . | . | . | . | . |
| 21-225_20D5_HC | . | . | . | . | . | . | S | . | . | . | . | . | . | . | . | . | . | . | . | . | . | . | . | . | . | . |
| 21-225_20E12_HC | . | . | . | . | . | . | S | . | . | . | . | . | . | . | . | . | . | . | . | . | . | . | . | . | . | . |
| 21-225_20E5_HC | . | . | . | . | . | . | . | . | . | . | . | . | . | . | . | . | . | . | . | . | . | . | . | . | . | . |
| 21-225_20H12_HC | . | . | . | . | . | . | N | . | . | . | . | . | . | . | . | . | . | . | . | . | . | . | . | . | . | . |
| 21-225_21E5_HC | . | . | . | . | . | . | . | . | . | . | . | . | . | . | . | . | . | . | . | . | . | . | . | . | . | . |
| 21-225_21F7_HC | . | . | . | . | . | . | . | . | . | . | . | . | . | . | . | . | . | . | . | . | . | . | . | . | . | . |
| 21-225_21G11_HC | . | . | . | . | . | . | . | . | . | . | . | . | . | . | . | . | . | . | . | . | . | . | . | . | . | . |
| 21-225_21G2_HC | . | . | . | . | . | . | . | . | . | . | . | . | . | . | . | . | . | . | . | . | . | . | . | . | . | . |
| 21-225_21H8_HC | . | . | . | . | . | . | . | . | . | . | . | . | . | . | . | . | . | . | . | . | . | . | . | . | . | . |
| 21-225_224D10_HC | . | . | . | . | . | . | S | . | . | . | . | . | . | . | . | . | . | . | . | . | . | . | . | . | . | . |
| 21-225_22A4_HC | . | . | . | . | . | . | . | . | . | . | . | . | . | . | I | . | . | . | . | . | . | . | . | . | . | . |
| 21-225_22F2_HC | . | . | . | . | . | . | . | . | . | . | . | . | . | . | . | . | . | . | . | . | . | . | . | . | . | . |
| 21-225_22F9_HC | . | . | . | . | . | . | S | . | . | . | . | . | . | . | . | . | . | . | . | . | . | . | . | . | . | . |
| 21-225_22H8_HC | . | . | . | . | . | . | S | . | . | . | . | . | . | . | . | . | . | . | . | . | . | . | . | . | . | . |
| 21-225_23F12_HC | . | . | . | . | . | . | S | . | . | . | . | . | . | . | . | . | . | . | . | . | . | . | . | . | . | . |
| 21-225_23H11_HC | . | . | . | . | . | . | S | . | . | . | . | . | . | . | . | . | . | . | . | . | . | . | . | . | . | . |
| 21-225_25B6_HC | . | . | . | . | L | R | N | . | . | . | . | . | . | . | . | . | . | . | . | . | . | . | . | . | . | . |
| 21-225_25E6_HC | . | . | . | . | . | . | . | . | . | . | . | . | . | . | . | . | . | . | . | . | . | . | . | . | . | . |
| 21-225_25H10_HC | . | . | . | . | . | . | . | . | . | . | . | . | . | . | . | . | . | . | . | . | . | . | . | . | . | . |
| 21-225_25H11_HC | . | . | . | . | . | . | . | . | . | . | . | . | . | . | . | . | . | . | . | . | . | . | . | . | . | . |
| 21-225_26A2_HC | . | . | . | . | . | . | S | . | . | . | . | . | . | . | . | . | . | . | . | . | . | . | . | . | . | . |
| 21-225_27C3_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | D | - | - | - | - | - | Y | G | M | H | W | V | R | Q | A | P | G | K | G | L |
| 21-225_27D5_HC | . | . | . | . | . | . | S | . | . | . | . | . | . | . | . | . | . | . | . | . | . | . | . | . | . | . |
| 21-225_27F2_HC | . | . | . | I | . | . | . | . | . | . | . | . | . | . | . | . | . | . | . | . | . | . | . | . | . | . |
| 21-225_28C7_HC | . | . | . | . | . | . | S | . | . | . | . | . | . | . | . | . | . | . | . | . | . | . | . | . | . | . |
| 21-225_28F5_HC | . | . | . | . | . | . | S | . | . | . | . | . | . | . | . | . | . | . | . | . | . | . | . | . | . | . |
| 21-225_29B8_HC | . | . | . | . | . | . | . | . | . | . | . | . | . | . | . | . | . | . | . | . | . | . | . | . | . | . |
| 21-225_29D7_HC | . | . | . | . | . | . | S | . | . | . | . | . | . | . | . | . | . | . | . | . | . | . | . | . | . | . |
| 21-225_29G4_HC | . | . | . | . | . | . | . | . | . | . | . | . | . | . | I | . | . | . | . | . | . | . | . | . | . | . |
| 21-225_29G8_HC | . | . | . | . | . | . | . | . | . | . | . | . | . | . | . | . | . | . | . | . | . | . | . | . | . | . |
| 21-225_29H6_HC | . | . | . | . | . | . | . | . | . | . | . | . | . | . | . | . | . | . | . | . | . | . | . | . | . | . |
| 21-225_2B2_HC | . | . | . | . | . | . | S | . | . | . | . | . | . | . | . | . | . | . | . | . | . | . | . | . | . | . |
| 21-225_43C3_HC | . | . | . | . | . | . | S | . | . | . | . | . | . | . | I | . | . | . | . | . | . | . | . | . | . | . |
| 21-225_44C3_HC | . | . | . | . | . | . | S | . | . | . | . | . | . | . | . | . | . | . | . | . | . | . | . | . | . | . |
| 21-225_44C8_HC | . | . | . | . | . | N | N | . | . | . | . | . | . | . | . | . | . | . | . | . | . | . | . | . | . | . |
| 21-225_46C6_HC | . | . | . | . | . | . | S | . | . | . | . | . | . | . | I | . | . | . | . | . | . | . | . | . | . | . |
| 21-225_46D8_HC | . | . | . | . | . | . | . | . | . | . | . | . | . | . | I | . | . | . | . | . | . | . | . | . | . | . |
| 21-225_46F3_HC | . | . | . | . | . | . | . | . | . | . | . | . | . | . | . | . | . | . | . | . | . | . | . | . | . | . |
| 21-225_47A1_HC | . | . | . | . | . | . | . | . | . | . | . | . | . | . | . | . | . | . | . | . | . | . | . | . | . | . |
| 21-225_48A9_HC | . | . | . | . | . | . | S | . | . | . | . | . | . | . | . | . | . | . | . | . | . | . | . | . | . | . |
| 21-225_48C1_HC | . | . | . | . | . | . | S | . | . | . | . | . | . | . | . | . | . | . | . | . | . | . | . | . | . | . |
| 21-225_48D12_HC | . | . | . | . | . | . | . | . | . | . | . | . | . | . | . | . | . | . | . | . | . | . | . | R | . | . |
| 21-225_49A1_HC | . | . | . | . | . | . | . | . | . | . | . | . | . | . | . | . | . | . | . | . | . | . | . | . | . | . |
| 21-225_49C6_HC | . | . | . | . | . | . | N | . | . | . | . | . | . | . | . | . | . | . | . | . | . | . | . | . | . | . |
| 21-225_51B11_HC | . | . | . | . | . | . | . | . | . | . | . | . | . | . | . | . | . | . | . | . | . | . | . | . | . | . |
| 21-225_51E4_HC | . | . | . | . | . | . | S | . | . | . | . | . | . | . | . | . | . | . | . | . | . | . | . | . | . | . |
| 21-225_51F11_HC | . | . | . | . | . | . | N | . | . | . | . | . | F | . | . | . | . | . | . | . | . | . | . | . | . | . |
| 21-225_51F9_HC | . | . | . | . | . | . | . | . | . | . | . | . | . | . | . | . | . | . | . | . | . | . | . | . | . | . |
| 21-225_52B2_HC | . | . | . | . | . | . | N | . | . | . | . | . | . | . | . | . | . | . | . | . | . | . | . | . | . | . |
| 21-225_52B9_HC | . | . | . | . | . | . | . | . | . | . | . | . | . | . | . | . | . | . | . | . | . | . | . | . | . | . |
| 21-225_53B12_HC | . | . | . | . | . | . | N | . | . | . | . | . | F | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | D | - | - | - | - | - | Y | G | M | H | W | V | R | Q | A | P | G | K | G | L |
| 21-225_53D2_HC | . | . | . | . | . | . | N | . | . | . | . | . | . | . | . | . | . | . | . | . | . | . | . | . | . | . |
| 21-225_53E6_HC | . | . | . | . | . | . | . | . | . | . | . | . | . | . | I | . | . | . | . | . | . | . | . | . | . | . |
| 21-225_54C6_HC | . | . | . | . | . | . | . | . | . | . | . | . | . | . | I | . | . | . | . | . | . | . | . | . | . | . |
| 21-225_54D3_HC | . | . | . | . | . | . | N | . | . | . | . | . | . | . | . | . | . | . | . | . | . | . | . | . | . | . |
| 21-225_54G7_HC | . | . | . | . | . | . | N | . | . | . | . | . | . | . | . | . | . | . | . | . | . | . | . | . | . | . |
| 21-225_55B3_HC | . | . | . | . | . | . | S | . | . | . | . | . | . | . | . | . | . | . | . | . | . | . | . | . | . | . |
| 21-225_57A4_HC | . | . | . | . | . | . | . | . | . | . | . | . | . | . | . | . | . | . | . | . | . | . | . | . | . | . |
| 21-225_58F5_HC | . | . | . | . | . | . | . | . | . | . | . | . | . | . | . | . | . | . | . | . | . | . | . | . | . | . |
| 21-225_59F11_HC | . | . | . | . | . | . | N | . | . | . | . | . | . | . | . | . | . | . | . | . | . | . | . | . | . | . |
| 21-225_60A3_HC | . | . | . | . | . | . | S | . | . | . | . | . | . | . | . | . | . | . | . | . | . | . | . | . | . | . |
| 21-225_62E4_HC | . | . | . | . | . | . | . | . | . | . | . | . | . | . | . | . | . | . | . | . | . | . | . | . | . | . |
| 21-225_64E1_HC | . | . | . | . | . | . | S | . | . | . | . | . | . | . | . | . | . | . | . | . | . | . | . | . | . | . |
| 21-225_65C1_HC | . | . | . | . | . | . | . | . | . | . | . | . | . | . | . | . | . | . | . | . | . | . | . | . | . | . |
| 21-225_66C7_HC | . | . | . | . | . | . | . | . | . | . | . | . | . | . | . | . | . | . | . | . | . | . | . | . | . | . |
| 21-225_68F11_HC | . | . | . | . | . | . | . | . | . | . | . | . | . | . | . | . | . | . | . | . | . | . | . | . | . | . |
| 21-225_6A11_HC | . | . | . | . | . | . | . | . | . | . | . | . | . | . | . | . | . | . | . | . | . | . | . | . | . | . |
| 21-225_6G12_HC | . | . | . | . | . | . | S | . | . | . | . | . | . | . | . | . | . | . | . | . | . | . | . | . | . | . |
| 21-225_6G2_HC | . | . | . | . | . | . | . | . | . | . | . | . | . | . | I | . | . | . | . | . | . | . | . | . | . | . |
| 21-225_71A2_HC | . | . | . | . | . | . | . | . | . | . | . | . | . | . | . | . | . | . | . | . | . | . | . | . | . | . |
| 21-225_71B11_HC | . | . | . | . | . | . | . | . | . | . | . | . | . | . | . | . | . | . | . | . | . | . | . | . | . | . |
| 21-225_71B6_HC | . | . | . | . | . | . | N | . | . | . | . | . | . | . | . | . | . | . | . | . | . | . | . | . | . | . |
| 21-225_72G12_HC | . | . | . | . | . | . | S | . | . | . | . | . | . | . | . | . | . | . | . | . | . | . | D | T | . | . |
| 21-225_7C4_HC | . | . | . | . | . | . | N | . | . | . | . | . | . | . | . | . | . | . | . | . | . | . | . | . | . | . |
| 21-225_7E9_HC | . | . | . | . | . | . | . | . | . | . | . | . | . | . | . | . | . | . | . | . | . | . | . | . | . | . |
| 21-225_7G2_HC | . | . | . | . | . | . | . | . | . | . | . | . | . | . | . | . | . | . | . | . | . | . | . | . | . | . |
| 21-225_7H11_HC | . | . | . | . | . | . | . | . | . | . | . | . | . | . | . | . | . | . | . | . | . | . | . | . | . | . |
| 21-225_8C9_HC | . | . | . | . | . | . | S | . | . | . | . | . | . | . | . | . | . | . | . | . | . | . | . | . | . | . |
| 21-225_8H7_HC | . | . | . | . | . | . | . | . | . | . | . | . | F | . | . | . | . | . | . | . | . | . | . | . | . | . |
| 21-225_9A1_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | D | - | - | - | - | - | Y | G | M | H | W | V | R | Q | A | P | G | K | G | L |
| 21-225_9E8_HC | . | . | . | . | . | . | S | . | . | . | . | . | . | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | A | V | I | W | Y | D | - | - | - | E | N | N | K | Y | Y | A | D | S | V | K | G | R | F |
| 21-225_10G5_HC | . | . | . | . | . | . | . | . | E | . | . | . | . | . | . | Q | . | . | . | . | . | . | . | . | . | . |
| 21-225_10H12_HC | . | . | . | . | . | . | . | . | E | . | . | . | D | . | . | Q | . | . | . | . | . | . | . | . | . | . |
| 21-225_12A1_HC | . | . | . | . | . | . | . | . | E | . | . | . | . | . | . | Q | . | . | . | . | . | . | . | . | . | . |
| 21-225_12B12_HC | . | . | M | . | . | . | . | . | . | . | . | . | V | T | . | . | . | . | . | . | . | . | . | . | . | . |
| 21-225_12E6_HC | . | . | . | T | . | . | . | . | . | . | . | . | . | S | . | . | . | . | . | . | . | . | . | . | . | . |
| 21-225_12F2_HC | . | . | . | . | . | . | . | F | . | . | . | . | G | . | . | . | . | . | V | . | . | . | . | . | . | . |
| 21-225_13E6_HC | . | . | . | . | . | . | . | . | . | . | . | . | . | . | . | . | . | . | I | . | . | . | . | . | . | . |
| 21-225_146H9_HC | . | . | . | . | . | . | . | F | . | . | . | . | . | S | . | . | H | . | G | . | . | . | . | . | . | . |
| 21-225_14E3_HC | . | . | . | . | . | . | . | . | E | . | . | . | . | . | . | Q | . | . | . | . | . | . | . | . | . | . |
| 21-225_157C3_HC | . | . | . | . | . | . | . | . | . | . | . | . | V | . | . | . | . | . | . | . | . | . | . | . | . | . |
| 21-225_157H8_HC | . | . | . | . | . | . | . | . | . | . | . | . | V | . | . | . | . | . | . | . | . | . | . | . | . | . |
| 21-225_158H6_HC | . | . | . | . | . | . | . | . | . | . | . | . | V | T | . | . | . | . | . | . | . | . | . | . | . | . |
| 21-225_159C5_HC | . | . | . | . | . | V | . | . | . | . | . | . | V | . | . | . | . | . | . | . | . | . | . | . | . | . |
| 21-225_15E3_HC | . | . | . | . | . | . | . | . | E | . | . | . | . | . | . | . | H | . | . | . | . | . | . | . | . | . |
| 21-225_16A5_HC | . | . | . | . | . | . | . | F | . | . | . | . | G | S | . | . | . | . | V | . | . | . | . | . | . | . |
| 21-225_16B7_HC | . | . | . | . | . | . | . | . | . | . | . | . | . | S | . | . | . | . | . | . | . | . | . | . | . | . |
| 21-225_16F10_HC | . | . | . | . | . | . | . | . | . | . | . | . | G | . | . | . | . | . | V | . | . | . | . | . | . | . |
| 21-225_16F11_HC | . | . | . | . | . | . | . | . | E | . | . | . | . | . | . | . | . | . | V | . | . | . | . | . | . | . |
| 21-225_16F4_HC | . | . | . | . | . | . | . | . | E | . | . | . | G | S | . | Q | . | . | G | . | . | . | . | . | . | . |
| 21-225_16F6_HC | . | . | . | . | . | . | . | . | E | . | . | . | G | S | . | Q | . | . | G | . | . | . | . | . | . | . |
| 21-225_16G7_HC | . | . | . | . | . | . | . | . | . | . | . | . | . | . | . | E | . | . | . | . | . | . | . | . | . | . |
| 21-225_17A10_HC | . | . | . | . | . | . | . | . | E | . | . | . | . | . | . | Q | . | . | . | . | . | . | . | . | . | . |
| 21-225_17B5_HC | . | . | . | . | . | . | . | . | . | . | . | . | . | S | . | . | H | . | I | . | . | . | . | . | . | . |
| 21-225_17C7_HC | . | . | . | . | . | . | . | F | . | . | . | . | G | S | . | . | . | . | . | . | . | . | . | . | . | . |
| 21-225_17E5_HC | . | . | . | . | L | . | . | . | . | . | . | . | V | T | . | . | . | . | G | . | . | . | . | . | . | . |
| 21-225_18A1_HC | . | . | . | . | . | . | . | . | E | . | . | . | . | . | . | . | . | . | V | . | . | . | . | . | . | . |
| 21-225_18A5_HC | . | . | . | . | . | . | . | F | . | . | . | . | G | S | . | . | . | . | . | . | . | . | . | . | . | . |
| 21-225_18C2_HC | . | . | M | . | . | . | . | . | . | . | . | . | V | T | . | . | . | . | . | . | . | . | . | . | . | . |
| 21-225_18E8_HC | . | . | . | . | . | . | . | . | E | . | . | . | . | . | . | Q | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | A | V | I | W | Y | D | - | - | - | E | N | N | K | Y | Y | A | D | S | V | K | G | R | F |
| 21-225_18F2_HC | . | . | . | . | . | . | . | F | . | . | . | . | G | S | . | . | . | . | V | . | . | . | . | . | . | . |
| 21-225_19E1_HC | . | . | . | . | . | . | . | . | E | . | . | . | . | . | . | Q | . | . | . | . | . | . | . | . | . | . |
| 21-225_1C12_HC | . | . | . | . | . | . | . | . | . | . | . | . | . | . | . | . | . | . | V | . | . | . | . | . | . | . |
| 21-225_203B9_HC | . | . | . | . | . | . | . | . | . | . | . | . | V | . | . | . | . | . | . | . | . | . | . | . | . | . |
| 21-225_207F12_HC | . | . | . | . | . | . | . | . | . | . | . | . | R | . | . | . | . | . | G | . | . | . | . | . | . | . |
| 21-225_20A7_HC | . | . | . | . | . | . | . | . | E | . | . | . | . | . | . | Q | . | . | . | . | . | . | . | . | . | . |
| 21-225_20D10_HC | . | . | . | . | . | . | . | F | . | . | . | . | G | S | . | . | . | . | V | . | . | . | . | . | . | . |
| 21-225_20D5_HC | . | . | . | . | . | . | . | . | E | . | . | . | . | . | . | Q | . | . | . | . | . | . | . | . | . | . |
| 21-225_20E12_HC | . | . | . | . | . | M | . | . | . | . | . | . | . | S | . | . | H | . | . | . | . | . | . | . | . | . |
| 21-225_20E5_HC | . | . | . | . | . | . | . | . | E | . | . | . | . | . | . | Q | . | . | . | . | . | . | . | . | . | . |
| 21-225_20H12_HC | . | . | . | . | . | . | . | . | . | . | . | . | . | . | . | . | . | . | V | . | . | . | . | . | . | . |
| 21-225_21E5_HC | . | . | . | . | . | . | . | . | . | . | . | . | . | S | . | . | . | . | . | . | . | . | . | . | . | . |
| 21-225_21F7_HC | . | . | . | . | . | . | . | F | . | . | . | . | G | . | . | . | . | . | V | . | . | . | . | . | . | . |
| 21-225_21G11_HC | . | . | M | . | . | . | . | . | . | . | . | . | V | T | . | E | . | . | . | . | . | . | . | . | . | . |
| 21-225_21G2_HC | . | . | M | . | . | . | . | . | . | . | . | . | V | T | . | . | . | . | . | . | . | . | . | . | . | . |
| 21-225_21H8_HC | . | . | . | . | . | . | . | F | . | . | . | . | G | S | . | . | . | . | . | . | . | . | . | . | . | . |
| 21-225_224D10_HC | . | . | . | . | . | . | . | . | E | . | . | . | G | S | . | . | . | . | V | . | . | . | R | . | . | . |
| 21-225_22A4_HC | . | . | . | . | . | . | . | F | . | . | . | . | G | S | . | . | . | . | . | . | . | . | . | . | . | I |
| 21-225_22F2_HC | . | . | . | . | . | . | . | . | E | . | . | . | G | S | . | . | . | . | V | . | . | . | R | . | . | . |
| 21-225_22F9_HC | . | . | . | . | . | . | . | . | E | . | . | . | . | . | . | . | D | . | V | . | . | . | . | . | . | . |
| 21-225_22H8_HC | . | . | . | . | . | . | . | . | . | . | . | . | G | . | . | . | . | . | . | . | . | . | . | . | . | . |
| 21-225_23F12_HC | . | . | . | . | . | . | . | . | . | . | . | . | . | T | . | . | . | . | . | . | . | . | . | . | . | . |
| 21-225_23H11_HC | . | . | . | . | . | . | . | . | . | . | . | . | . | . | . | . | . | . | V | . | . | . | . | . | . | . |
| 21-225_25B6_HC | . | . | . | . | . | . | . | . | E | . | . | . | . | S | . | . | . | . | T | E | . | . | . | . | . | . |
| 21-225_25E6_HC | . | . | . | . | . | . | . | . | . | . | . | . | . | . | . | . | . | . | . | . | . | . | . | . | . | . |
| 21-225_25H10_HC | . | . | . | . | . | . | . | . | . | . | . | . | . | S | . | . | . | . | . | . | . | M | . | . | . | . |
| 21-225_25H11_HC | . | . | . | . | . | . | . | . | . | . | . | . | . | . | . | E | . | . | . | . | . | . | . | . | . | . |
| 21-225_26A2_HC | . | . | . | . | . | . | . | . | E | . | . | . | G | S | . | . | . | . | V | . | . | . | . | . | . | . |
| 21-225_27C3_HC | . | . | . | . | . | . | . | . | . | . | . | . | . | . | . | . | S | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | A | V | I | W | Y | D | - | - | - | E | N | N | K | Y | Y | A | D | S | V | K | G | R | F |
| 21-225_27D5_HC | . | . | . | . | . | . | . | . | . | . | . | . | . | S | . | . | . | . | G | . | . | . | . | . | . | . |
| 21-225_27F2_HC | . | . | . | . | . | . | . | . | . | . | . | . | . | . | . | . | . | . | V | . | . | . | R | . | . | . |
| 21-225_28C7_HC | . | . | . | . | . | . | . | . | . | . | . | . | . | S | . | . | . | . | . | . | . | . | . | . | . | . |
| 21-225_28F5_HC | . | . | . | . | . | . | . | . | . | . | . | . | . | . | . | . | . | . | . | . | . | . | . | . | . | . |
| 21-225_29B8_HC | . | . | . | . | . | . | . | . | . | . | . | . | . | . | . | . | . | . | . | . | . | . | . | . | . | . |
| 21-225_29D7_HC | . | . | . | . | . | . | . | . | . | . | . | . | . | S | . | . | . | . | G | . | . | . | . | . | . | . |
| 21-225_29G4_HC | . | . | . | . | . | . | . | . | . | . | . | . | . | S | . | . | . | . | . | . | . | M | . | . | . | . |
| 21-225_29G8_HC | . | . | . | . | . | . | . | . | . | . | . | . | . | T | . | . | . | . | . | . | . | . | . | . | . | . |
| 21-225_29H6_HC | . | . | . | T | . | . | . | . | N | . | . | . | . | . | . | . | . | . | . | . | . | . | . | . | . | . |
| 21-225_2B2_HC | . | . | . | . | . | . | . | . | E | . | . | . | . | . | . | . | . | . | V | . | . | . | . | . | . | . |
| 21-225_43C3_HC | . | . | . | . | . | . | . | . | . | . | . | . | . | . | . | . | . | . | . | . | . | . | . | . | . | . |
| 21-225_44C3_HC | . | . | . | . | . | . | . | F | E | . | . | . | G | S | . | . | . | . | . | . | . | . | . | . | . | . |
| 21-225_44C8_HC | . | . | . | . | . | . | . | . | E | . | . | . | G | S | . | . | . | . | . | . | . | . | . | . | . | . |
| 21-225_46C6_HC | . | . | . | . | . | . | . | . | . | . | . | . | . | . | . | . | . | . | . | . | . | . | . | . | . | . |
| 21-225_46D8_HC | . | . | . | . | . | . | . | F | E | . | . | . | G | S | . | . | . | . | . | . | . | . | . | . | . | . |
| 21-225_46F3_HC | . | . | . | . | . | . | . | F | E | . | . | . | G | S | . | . | . | . | . | . | . | . | . | . | . | . |
| 21-225_47A1_HC | . | . | . | . | . | . | . | . | . | . | . | . | D | Y | . | . | K | . | . | . | . | . | . | . | . | . |
| 21-225_48A9_HC | . | . | . | . | . | . | . | . | E | . | . | . | . | . | K | . | . | . | . | . | . | . | . | . | . | . |
| 21-225_48C1_HC | . | . | . | . | . | V | . | . | . | . | . | . | . | I | . | . | K | . | . | . | . | . | . | . | . | V |
| 21-225_48D12_HC | . | . | . | . | . | . | . | . | . | . | . | . | V | S | . | . | . | . | V | . | . | . | . | . | . | . |
| 21-225_49A1_HC | . | . | . | . | . | . | . | . | . | . | . | . | . | D | . | . | . | . | V | . | . | . | . | . | . | . |
| 21-225_49C6_HC | . | . | . | . | . | . | . | F | . | . | . | . | V | S | . | . | K | . | V | . | . | . | . | . | . | . |
| 21-225_51B11_HC | . | . | . | . | . | . | . | F | G | . | . | . | G | . | . | . | . | . | G | . | . | . | . | . | . | . |
| 21-225_51E4_HC | . | . | . | . | . | . | . | . | . | . | . | . | . | S | . | . | . | . | . | . | . | . | . | . | . | . |
| 21-225_51F11_HC | . | . | . | . | . | . | . | . | E | . | . | . | . | S | . | . | . | . | . | . | . | . | . | . | . | . |
| 21-225_51F9_HC | . | . | . | . | . | . | . | F | . | . | . | . | G | . | . | . | . | . | . | . | . | . | . | . | . | . |
| 21-225_52B2_HC | . | . | . | . | . | T | . | F | . | . | . | . | G | S | . | Q | R | . | . | . | . | . | . | . | . | . |
| 21-225_52B9_HC | . | . | . | . | . | . | . | F | . | . | . | . | G | . | . | . | . | . | . | . | . | . | . | . | . | . |
| 21-225_53B12_HC | . | . | . | . | . | V | . | . | . | . | . | . | G | . | . | R | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | A | V | I | W | Y | D | - | - | - | E | N | N | K | Y | Y | A | D | S | V | K | G | R | F |
| 21-225_53D2_HC | . | . | . | T | . | V | . | D | . | . | . | . | G | S | . | . | . | . | . | . | . | . | . | . | . | . |
| 21-225_53E6_HC | . | . | . | . | . | . | . | . | . | . | . | . | . | S | . | . | . | . | G | . | . | . | . | . | . | . |
| 21-225_54C6_HC | D | . | . | . | . | . | . | . | . | . | . | . | . | . | . | . | . | . | . | . | . | . | . | . | . | . |
| 21-225_54D3_HC | . | . | . | . | . | T | . | F | . | . | . | . | G | . | . | N | . | . | . | . | . | . | . | . | . | . |
| 21-225_54G7_HC | . | . | . | . | . | . | . | . | E | . | . | . | . | S | . | . | . | . | G | . | . | . | . | . | . | . |
| 21-225_55B3_HC | . | . | . | . | . | . | . | F | . | . | . | . | G | . | . | . | . | . | E | . | . | . | . | . | . | . |
| 21-225_57A4_HC | . | . | . | . | . | . | . | . | A | . | . | . | G | S | . | . | . | . | V | . | . | . | . | . | . | . |
| 21-225_58F5_HC | . | . | . | . | . | . | . | . | A | . | . | . | G | S | . | . | . | H | V | . | . | . | . | . | . | . |
| 21-225_59F11_HC | . | . | . | . | . | . | . | . | . | . | . | . | . | S | . | . | H | . | . | . | . | . | . | . | . | . |
| 21-225_60A3_HC | . | . | . | . | . | . | . | . | E | . | . | . | . | S | . | . | . | . | . | . | . | . | . | . | . | . |
| 21-225_62E4_HC | . | . | . | . | . | . | . | . | . | . | . | . | R | S | . | . | . | . | . | . | . | . | . | . | . | . |
| 21-225_64E1_HC | . | . | . | . | . | . | . | F | . | . | . | . | . | T | . | . | . | . | G | . | . | . | . | . | . | . |
| 21-225_65C1_HC | . | . | . | . | . | . | . | F | E | . | . | . | G | S | . | . | H | . | T | . | . | . | . | . | . | . |
| 21-225_66C7_HC | . | . | . | . | . | . | . | F | E | . | . | . | G | S | H | . | . | . | T | . | . | . | . | . | . | . |
| 21-225_68F11_HC | . | . | . | . | . | . | . | . | . | . | . | . | V | S | . | . | . | . | . | . | . | . | . | . | . | . |
| 21-225_6A11_HC | . | . | . | . | . | . | . | F | . | . | . | . | G | S | . | . | . | . | . | . | . | . | . | . | . | . |
| 21-225_6G12_HC | . | . | . | . | . | . | . | . | . | . | . | . | R | S | . | N | . | . | . | . | . | . | . | . | . | . |
| 21-225_6G2_HC | . | . | . | . | . | . | . | F | . | . | . | . | G | S | . | . | . | . | . | . | . | . | . | . | . | . |
| 21-225_71A2_HC | . | . | . | . | . | . | . | . | . | . | . | . | . | S | . | . | . | . | . | . | . | . | . | . | . | . |
| 21-225_71B11_HC | . | . | . | . | . | . | . | . | . | . | . | . | R | . | . | . | . | . | G | . | . | . | . | . | . | . |
| 21-225_71B6_HC | . | . | . | . | . | . | . | . | . | . | . | . | R | T | . | . | . | . | . | . | . | . | . | . | . | . |
| 21-225_72G12_HC | . | . | . | . | . | . | . | F | . | . | . | . | . | S | . | . | . | . | . | . | . | . | . | . | . | . |
| 21-225_7C4_HC | . | . | . | . | . | . | . | . | E | . | . | . | . | . | . | Q | . | . | . | . | . | . | . | . | . | . |
| 21-225_7E9_HC | . | . | . | . | . | . | . | . | . | . | . | . | . | . | . | . | . | . | V | . | . | . | . | . | . | . |
| 21-225_7G2_HC | . | . | M | . | . | . | . | . | . | . | . | . | V | T | . | . | . | . | . | . | . | . | . | . | . | . |
| 21-225_7H11_HC | . | . | . | . | . | . | . | . | . | . | . | . | . | . | . | . | . | . | . | . | . | . | . | . | . | . |
| 21-225_8C9_HC | . | . | . | . | . | . | . | . | . | . | . | . | . | . | . | . | . | . | V | . | . | . | . | . | . | . |
| 21-225_8H7_HC | . | . | . | . | . | . | . | . | E | . | . | . | . | . | . | Q | . | . | . | . | . | . | . | . | . | . |
| 21-225_9A1_HC | . | . | . | . | . | . | . | F | . | . | . | . | G | S | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | A | V | I | W | Y | D | - | - | - | E | N | N | K | Y | Y | A | D | S | V | K | G | R | F |
| 21-225_9E8_HC | . | . | . | . | . | . | . | . | E | . | . | . | . | . | . | Q | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_10G5_HC | . | . | . | . | . | . | . | . | . | . | . | . | . | . | . | . | . | . | . | . | . | . | . | . | . | . |
| 21-225_10H12_HC | . | . | . | . | . | . | . | . | . | . | . | . | . | . | . | . | . | . | . | . | . | . | . | . | . | . |
| 21-225_12A1_HC | . | . | . | . | . | . | . | . | . | . | . | . | . | . | . | . | . | . | . | . | . | . | . | . | . | . |
| 21-225_12B12_HC | . | . | . | . | . | . | . | . | . | . | . | . | . | . | . | . | . | . | . | . | . | . | . | . | . | . |
| 21-225_12E6_HC | . | . | . | . | . | D | . | . | . | . | . | . | . | . | . | . | . | . | . | . | . | . | . | . | . | . |
| 21-225_12F2_HC | . | . | . | . | . | . | . | . | . | . | . | F | . | . | . | . | . | . | . | . | . | . | . | . | . | . |
| 21-225_13E6_HC | . | . | . | . | . | . | . | . | . | . | . | . | . | . | . | . | . | . | . | . | . | . | . | . | . | . |
| 21-225_146H9_HC | . | . | . | . | . | . | . | . | . | . | . | . | . | . | . | . | . | . | . | . | . | . | . | . | . | . |
| 21-225_14E3_HC | . | . | . | . | . | . | . | . | . | . | . | . | . | . | . | . | . | . | . | . | . | . | . | . | F | . |
| 21-225_157C3_HC | . | . | . | . | . | . | . | . | . | . | . | . | . | . | . | . | . | . | . | . | . | . | . | . | . | . |
| 21-225_157H8_HC | . | . | . | . | . | . | . | . | . | . | . | . | . | . | . | . | . | . | . | . | . | . | . | . | . | . |
| 21-225_158H6_HC | . | . | . | . | . | . | . | . | . | . | . | . | . | . | . | . | . | . | . | D | . | . | . | . | . | . |
| 21-225_159C5_HC | . | . | . | . | . | . | . | . | . | . | . | . | . | . | . | . | . | . | . | . | . | . | . | . | . | . |
| 21-225_15E3_HC | . | . | . | . | . | . | . | . | . | . | . | . | . | . | . | . | . | . | . | . | . | . | . | . | . | . |
| 21-225_16A5_HC | . | . | . | . | . | . | . | . | . | . | . | . | . | . | . | S | . | . | . | . | . | . | . | . | . | . |
| 21-225_16B7_HC | . | . | . | . | . | . | . | . | . | . | . | . | . | . | . | . | . | . | . | . | . | . | . | . | . | . |
| 21-225_16F10_HC | . | . | . | . | . | . | . | . | . | . | . | . | . | . | . | . | . | . | . | . | . | . | . | . | . | . |
| 21-225_16F11_HC | . | . | . | . | . | . | . | . | S | . | . | . | . | . | . | . | . | . | . | . | . | . | . | . | . | . |
| 21-225_16F4_HC | . | . | . | . | . | . | . | . | . | . | V | . | . | . | . | H | . | . | . | . | . | . | . | . | . | . |
| 21-225_16F6_HC | . | . | . | . | . | . | . | . | . | . | V | . | . | . | . | H | . | . | . | . | . | . | . | . | . | . |
| 21-225_16G7_HC | . | . | . | . | A | . | . | . | . | . | . | . | . | . | . | . | . | . | . | . | . | . | . | . | . | . |
| 21-225_17A10_HC | . | . | . | . | . | . | . | . | . | . | . | . | . | . | . | . | . | . | . | . | . | . | . | . | . | . |
| 21-225_17B5_HC | . | . | . | . | . | . | . | . | . | . | . | . | . | . | . | . | . | . | . | . | . | . | . | . | . | . |
| 21-225_17C7_HC | . | . | . | . | . | . | . | . | . | . | . | . | . | . | . | . | . | . | . | . | . | G | . | . | . | . |
| 21-225_17E5_HC | . | . | . | . | . | . | . | Q | . | . | . | . | . | . | L | . | . | . | . | . | . | . | . | . | . | . |
| 21-225_18A1_HC | . | . | . | . | . | . | . | . | S | . | . | . | . | . | . | . | . | . | . | . | . | . | . | . | . | . |
| 21-225_18A5_HC | . | . | . | . | . | . | . | . | . | . | . | . | . | . | . | . | . | . | . | . | . | . | . | . | . | . |
| 21-225_18C2_HC | . | . | . | . | . | . | . | . | . | . | . | . | . | . | . | . | . | . | S | . | . | . | . | . | . | . |
| 21-225_18E8_HC | . | V | . | . | . | . | . | . | . | . | . | F | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_18F2_HC | . | . | . | . | . | . | . | . | . | . | . | . | . | . | . | . | . | . | . | V | . | . | . | . | . | . |
| 21-225_19E1_HC | . | . | . | . | . | . | . | . | . | . | . | . | . | . | . | . | . | . | . | . | . | . | . | . | . | . |
| 21-225_1C12_HC | . | . | . | . | . | . | . | . | . | . | . | . | . | . | . | . | . | . | . | . | . | . | . | . | . | . |
| 21-225_203B9_HC | . | . | . | . | . | . | . | . | . | . | . | . | . | . | . | . | . | . | . | P | . | . | . | . | . | . |
| 21-225_207F12_HC | . | . | . | . | . | . | . | . | . | . | . | . | . | . | . | . | . | . | . | . | . | . | . | . | . | . |
| 21-225_20A7_HC | . | . | . | . | . | . | . | . | . | . | . | . | . | . | . | . | . | . | . | . | . | . | . | . | I | . |
| 21-225_20D10_HC | . | . | . | . | . | D | . | . | . | . | . | . | . | . | . | . | . | . | . | . | . | . | . | . | . | . |
| 21-225_20D5_HC | . | . | . | . | . | . | . | . | . | . | . | . | . | . | . | . | . | . | . | . | D | . | . | . | . | . |
| 21-225_20E12_HC | . | . | . | . | . | . | . | R | . | . | . | . | . | . | . | . | . | . | . | . | . | . | . | . | . | . |
| 21-225_20E5_HC | . | . | . | . | . | . | . | . | . | . | . | . | . | . | . | . | . | . | . | . | . | . | . | . | . | . |
| 21-225_20H12_HC | . | . | . | . | . | . | . | . | . | . | . | . | . | . | . | . | . | . | . | . | . | . | . | . | . | . |
| 21-225_21E5_HC | . | . | . | . | . | . | . | . | . | . | . | . | . | . | . | . | . | . | . | . | . | . | . | . | M | . |
| 21-225_21F7_HC | . | . | . | . | . | . | . | . | . | . | . | . | . | . | . | . | . | . | . | . | . | . | . | . | . | . |
| 21-225_21G11_HC | . | . | . | . | . | . | . | . | . | . | . | . | . | . | . | . | . | . | . | . | . | . | . | . | . | . |
| 21-225_21G2_HC | . | . | . | . | . | . | . | . | . | . | . | . | . | E | . | . | . | . | . | . | . | . | . | . | . | . |
| 21-225_21H8_HC | . | . | . | . | . | . | . | . | . | . | . | . | . | . | . | . | . | . | . | . | . | . | . | . | . | . |
| 21-225_224D10_HC | . | . | . | . | . | . | . | . | S | . | . | . | . | . | . | . | . | . | . | . | . | . | . | . | . | . |
| 21-225_22A4_HC | . | . | . | . | . | . | . | . | . | . | . | . | . | . | . | . | . | . | . | . | . | . | . | . | . | . |
| 21-225_22F2_HC | . | . | . | . | . | . | . | . | S | . | . | . | . | . | . | . | . | . | . | . | . | . | . | . | . | . |
| 21-225_22F9_HC | . | . | . | . | . | . | . | . | S | . | . | . | . | . | . | . | . | . | . | . | . | . | . | . | . | . |
| 21-225_22H8_HC | . | . | . | . | . | . | . | . | . | . | . | . | . | . | . | . | . | . | . | . | . | . | . | . | . | . |
| 21-225_23F12_HC | . | . | . | . | . | . | . | . | . | . | . | . | . | . | . | . | . | . | . | . | . | . | . | . | I | . |
| 21-225_23H11_HC | . | . | . | . | . | . | . | . | . | . | . | . | . | . | . | . | . | . | . | . | . | . | . | . | . | . |
| 21-225_25B6_HC | . | . | . | . | . | . | . | . | . | . | . | . | . | . | . | . | . | . | . | . | . | . | . | . | . | . |
| 21-225_25E6_HC | . | . | . | . | . | . | . | . | . | . | . | . | . | . | . | . | . | . | . | . | . | . | . | . | L | . |
| 21-225_25H10_HC | . | . | . | . | . | . | . | . | . | . | . | . | . | . | . | . | . | . | . | . | . | . | . | . | . | . |
| 21-225_25H11_HC | . | . | . | . | . | . | . | . | . | . | . | . | . | . | . | . | . | . | . | . | . | . | . | . | . | . |
| 21-225_26A2_HC | . | . | . | . | . | . | . | . | . | . | . | . | . | . | . | . | . | . | . | . | . | . | . | . | . | . |
| 21-225_27C3_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_27D5_HC | . | . | . | . | . | . | . | . | . | M | . | . | . | . | . | . | . | . | . | G | . | . | . | . | L | . |
| 21-225_27F2_HC | . | . | . | . | . | . | . | . | . | . | . | . | . | . | . | . | . | . | . | . | . | . | . | . | . | . |
| 21-225_28C7_HC | . | . | . | . | . | . | . | . | . | . | . | . | . | . | . | . | . | . | . | . | . | . | . | . | . | . |
| 21-225_28F5_HC | . | . | . | . | . | . | . | . | . | . | . | . | . | . | . | . | . | . | . | . | . | . | . | . | . | . |
| 21-225_29B8_HC | . | . | . | . | . | . | . | . | . | . | . | . | . | . | . | . | . | . | . | . | . | . | . | . | . | . |
| 21-225_29D7_HC | . | . | . | . | . | . | . | . | . | . | . | . | . | . | . | . | . | . | . | G | . | . | . | . | L | . |
| 21-225_29G4_HC | . | . | . | . | . | . | . | . | . | . | . | . | . | . | . | . | . | . | . | . | . | . | . | . | . | . |
| 21-225_29G8_HC | . | . | . | . | . | . | . | . | . | . | . | . | . | . | . | . | . | . | . | . | . | . | . | . | . | . |
| 21-225_29H6_HC | . | . | . | . | . | . | . | . | . | . | . | . | . | . | . | . | . | . | . | . | . | . | . | . | . | . |
| 21-225_2B2_HC | . | . | . | . | . | . | . | . | S | . | . | . | . | . | . | . | . | . | . | . | . | . | . | . | . | . |
| 21-225_43C3_HC | . | . | . | . | . | . | . | . | . | . | . | . | . | . | . | . | . | . | . | . | . | . | . | . | . | . |
| 21-225_44C3_HC | . | . | . | . | . | . | . | . | . | . | . | . | . | . | . | S | . | . | . | . | . | . | . | . | . | . |
| 21-225_44C8_HC | . | . | . | . | . | . | . | . | . | . | . | . | . | . | . | . | . | . | . | . | . | . | . | . | . | . |
| 21-225_46C6_HC | . | . | . | . | . | . | . | . | . | . | . | . | . | . | . | . | . | . | . | . | . | . | . | . | . | . |
| 21-225_46D8_HC | . | . | . | . | . | . | . | . | . | . | . | . | V | . | . | . | . | . | . | . | . | . | . | . | . | . |
| 21-225_46F3_HC | . | . | . | . | . | . | . | . | . | . | . | . | V | . | . | . | . | . | . | . | . | . | . | . | . | . |
| 21-225_47A1_HC | . | . | . | . | . | . | A | . | . | . | . | . | . | . | V | . | . | . | . | V | . | . | . | . | . | . |
| 21-225_48A9_HC | . | . | . | . | . | . | . | . | . | . | . | F | . | . | . | . | . | . | . | . | . | . | . | . | M | . |
| 21-225_48C1_HC | . | . | . | . | . | . | . | . | . | . | . | . | . | . | . | . | . | . | . | . | . | . | . | . | M | . |
| 21-225_48D12_HC | . | . | . | . | . | . | . | . | . | . | . | . | . | . | . | . | . | . | . | . | . | . | . | . | . | . |
| 21-225_49A1_HC | . | . | . | . | . | . | . | . | . | . | . | . | . | . | . | . | . | . | . | . | . | . | . | . | . | . |
| 21-225_49C6_HC | . | . | . | . | . | . | . | . | . | . | . | . | . | . | . | S | . | . | . | . | . | . | . | . | . | . |
| 21-225_51B11_HC | . | . | . | . | . | . | . | . | . | . | . | . | . | . | . | . | . | . | S | . | . | . | . | . | . | . |
| 21-225_51E4_HC | . | . | . | . | . | . | . | . | . | . | . | . | . | . | . | . | . | . | . | . | . | . | . | . | . | . |
| 21-225_51F11_HC | . | . | . | . | . | . | . | . | . | . | . | . | . | . | . | . | . | . | . | . | . | . | . | . | . | . |
| 21-225_51F9_HC | . | . | . | . | . | . | . | . | . | . | . | . | . | . | . | . | . | . | S | . | . | . | . | . | . | . |
| 21-225_52B2_HC | . | . | . | . | . | I | . | . | . | . | . | . | . | . | . | . | . | . | S | . | . | . | . | . | . | . |
| 21-225_52B9_HC | . | . | . | . | . | . | . | . | . | . | . | . | . | . | . | . | . | . | S | . | . | . | . | . | . | . |
| 21-225_53B12_HC | . | . | . | . | . | . | . | . | . | . | . | . | . | . | . | H | . | . | . | . | . | . | . | . | . | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_53D2_HC | . | . | . | . | . | . | . | . | . | . | . | . | . | . | . | . | . | . | . | . | . | . | . | . | . | . |
| 21-225_53E6_HC | A | . | . | . | . | . | . | . | . | . | . | . | . | . | . | . | . | . | . | . | . | . | . | . | . | . |
| 21-225_54C6_HC | A | . | . | . | . | . | . | . | . | . | . | . | . | . | . | . | . | . | . | . | . | . | . | . | . | . |
| 21-225_54D3_HC | . | . | . | . | . | . | . | . | . | . | . | . | . | . | . | . | . | . | . | . | . | . | . | . | . | . |
| 21-225_54G7_HC | . | . | . | . | . | . | . | . | . | . | . | . | . | . | . | . | . | . | . | . | . | . | . | . | . | . |
| 21-225_55B3_HC | . | . | . | . | . | . | . | . | . | . | . | . | . | . | . | . | . | . | . | . | . | . | . | . | . | . |
| 21-225_57A4_HC | . | . | . | . | . | . | . | . | . | . | . | . | . | . | . | . | . | . | . | . | . | . | . | . | . | . |
| 21-225_58F5_HC | . | . | . | . | . | . | . | . | . | . | . | . | . | . | . | . | . | . | . | . | . | . | . | . | . | . |
| 21-225_59F11_HC | . | . | . | . | . | . | . | . | T | . | . | . | . | . | . | S | . | . | . | . | . | . | . | . | . | . |
| 21-225_60A3_HC | . | . | . | . | . | . | . | . | . | . | . | . | . | . | . | . | . | . | . | . | . | . | . | . | . | . |
| 21-225_62E4_HC | . | . | . | . | . | . | . | . | . | . | . | . | . | . | . | . | . | . | . | V | . | . | . | . | . | . |
| 21-225_64E1_HC | . | . | . | . | . | . | . | . | . | . | . | . | . | . | . | . | . | . | . | . | . | . | . | . | . | . |
| 21-225_65C1_HC | . | . | . | . | . | . | . | . | . | . | . | . | . | . | . | . | . | . | . | . | . | . | . | . | . | . |
| 21-225_66C7_HC | . | . | . | . | . | . | . | . | . | . | . | . | . | . | . | . | . | . | . | . | . | . | . | . | . | . |
| 21-225_68F11_HC | . | . | . | . | . | . | . | . | . | . | . | . | . | . | . | . | . | . | . | . | . | . | . | . | . | . |
| 21-225_6A11_HC | . | . | . | . | . | . | . | . | . | . | . | . | . | . | . | . | . | . | . | . | . | . | . | . | . | . |
| 21-225_6G12_HC | . | . | . | . | . | . | . | . | . | . | . | . | . | . | . | . | . | . | . | . | . | . | . | . | . | . |
| 21-225_6G2_HC | . | . | . | S | . | . | . | . | . | . | . | . | . | . | . | . | . | . | . | . | . | . | . | . | . | . |
| 21-225_71A2_HC | . | . | . | . | . | . | . | . | . | . | . | . | . | . | . | . | . | . | . | . | . | . | . | . | . | . |
| 21-225_71B11_HC | . | . | . | . | . | . | . | . | . | . | . | . | . | . | . | . | . | . | . | . | . | . | . | . | . | . |
| 21-225_71B6_HC | . | . | . | . | . | . | . | . | . | . | . | H | . | . | . | . | . | . | . | . | . | . | . | . | . | . |
| 21-225_72G12_HC | . | . | . | . | . | . | . | . | . | . | . | . | . | . | . | . | . | . | . | . | . | . | . | . | . | . |
| 21-225_7C4_HC | . | . | . | . | . | . | . | . | . | . | . | . | . | . | . | . | . | . | . | . | . | . | . | . | . | . |
| 21-225_7E9_HC | . | . | . | . | . | . | . | . | . | . | . | . | . | . | . | . | . | . | . | . | . | . | . | . | . | . |
| 21-225_7G2_HC | . | . | . | . | . | . | . | . | . | . | . | . | . | . | L | . | . | . | . | . | . | . | . | . | . | . |
| 21-225_7H11_HC | . | . | . | . | . | . | . | . | . | . | . | . | . | . | . | . | . | . | . | . | . | . | . | . | . | . |
| 21-225_8C9_HC | . | . | . | . | . | . | . | . | . | . | . | . | . | . | . | . | . | . | . | . | . | . | . | . | . | . |
| 21-225_8H7_HC | . | . | . | . | . | . | . | . | . | . | . | . | . | . | . | . | . | . | . | . | . | . | . | . | . | . |
| 21-225_9A1_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_9E8_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | E | L | G | F | - | - | - | - | - | - | - | - | - | - | - | - | - | - | - | - | - | - |
| 21-225_10G5_HC | . | . | T | . | . | . | . | . | . | . | . | . | . | . | . | . | . | . | . | . | . | . | . | . | . | . |
| 21-225_10H12_HC | . | . | . | . | . | . | . | . | . | . | . | . | . | . | . | . | . | . | . | . | . | . | . | . | . | . |
| 21-225_12A1_HC | . | . | . | . | . | . | . | . | . | . | . | . | . | . | . | . | . | . | . | . | . | . | . | . | . | . |
| 21-225_12B12_HC | . | . | . | . | . | . | . | W | . | . | . | . | . | . | . | . | . | . | . | . | . | . | . | . | . | . |
| 21-225_12E6_HC | . | . | . | . | . | . | A | W | . | . | . | . | . | . | . | . | . | . | . | . | . | . | . | . | . | . |
| 21-225_12F2_HC | . | . | V | . | . | . | A | W | . | . | . | . | . | . | . | . | . | . | . | . | . | . | . | . | . | . |
| 21-225_13E6_HC | . | . | . | . | . | . | . | . | . | . | . | . | . | . | . | . | . | . | . | . | . | . | . | . | . | . |
| 21-225_146H9_HC | . | . | . | . | . | . | . | . | . | . | . | . | . | . | . | . | . | . | . | . | . | . | . | . | . | . |
| 21-225_14E3_HC | . | . | . | . | . | . | . | . | . | . | . | . | . | . | . | . | . | . | . | . | . | . | . | . | . | . |
| 21-225_157C3_HC | . | . | . | . | . | . | . | . | . | . | . | . | . | . | . | . | . | . | . | . | . | . | . | . | . | . |
| 21-225_157H8_HC | . | . | . | . | . | . | . | . | . | . | . | . | . | . | . | . | . | . | . | . | . | . | . | . | . | . |
| 21-225_158H6_HC | . | . | V | . | . | . | . | W | . | . | . | . | . | . | . | . | . | . | . | . | . | . | . | . | . | . |
| 21-225_159C5_HC | . | . | . | . | . | . | . | . | . | . | . | . | . | . | . | . | . | . | . | . | . | . | . | . | . | . |
| 21-225_15E3_HC | . | . | . | . | . | . | . | . | . | . | . | . | . | . | . | . | . | . | . | . | . | . | . | . | . | . |
| 21-225_16A5_HC | . | . | . | . | D | . | . | W | . | . | . | . | . | . | . | . | . | . | . | . | . | . | . | . | . | . |
| 21-225_16B7_HC | . | . | . | . | . | . | . | . | . | . | . | . | . | . | . | . | . | . | . | . | . | . | . | . | . | . |
| 21-225_16F10_HC | . | . | . | . | . | . | A | W | . | . | . | . | . | . | . | . | . | . | . | . | . | . | . | . | . | . |
| 21-225_16F11_HC | . | . | V | . | . | . | . | . | . | . | . | . | . | . | . | . | . | . | . | . | . | . | . | . | . | . |
| 21-225_16F4_HC | . | . | . | . | . | . | . | . | . | . | . | . | . | . | . | . | . | . | . | . | . | . | . | . | . | . |
| 21-225_16F6_HC | . | . | . | . | . | . | . | . | . | . | . | . | . | . | . | . | . | . | . | . | . | . | . | . | . | . |
| 21-225_16G7_HC | . | . | . | . | . | . | A | W | . | . | . | . | . | . | . | . | . | . | . | . | . | . | . | . | . | . |
| 21-225_17A10_HC | . | . | . | . | . | . | . | . | . | . | . | . | . | . | . | . | . | . | . | . | . | . | . | . | . | . |
| 21-225_17B5_HC | . | . | V | . | . | . | . | . | . | . | . | . | . | . | . | . | . | . | . | . | . | . | . | . | . | . |
| 21-225_17C7_HC | . | . | . | . | D | . | . | W | . | . | . | . | . | . | . | . | . | . | . | . | . | . | . | . | . | . |
| 21-225_17E5_HC | . | . | . | . | . | . | . | W | . | . | . | . | . | . | . | . | . | . | . | . | . | . | . | . | . | . |
| 21-225_18A1_HC | . | . | V | . | . | . | . | . | . | . | . | . | . | . | . | . | . | . | . | . | . | . | . | . | . | . |
| 21-225_18A5_HC | . | . | . | . | D | . | . | W | . | . | . | . | . | . | . | . | . | . | . | . | . | . | . | . | . | . |
| 21-225_18C2_HC | . | . | . | . | . | . | . | W | . | . | . | . | . | . | . | . | . | . | . | . | . | . | . | . | . | . |
| 21-225_18E8_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | E | L | G | F | - | - | - | - | - | - | - | - | - | - | - | - | - | - | - | - | - | - |
| 21-225_18F2_HC | . | . | . | . | D | . | . | W | . | . | . | . | . | . | . | . | . | . | . | . | . | . | . | . | . | . |
| 21-225_19E1_HC | . | . | . | . | . | . | . | . | . | . | . | . | . | . | . | . | . | . | . | . | . | . | . | . | . | . |
| 21-225_1C12_HC | . | . | . | . | . | . | . | . | . | . | . | . | . | . | . | . | . | . | . | . | . | . | . | . | . | . |
| 21-225_203B9_HC | . | . | . | . | . | . | . | . | . | . | . | . | . | . | . | . | . | . | . | . | . | . | . | . | . | . |
| 21-225_207F12_HC | . | . | . | . | . | . | . | . | . | . | . | . | . | . | . | . | . | . | . | . | . | . | . | . | . | . |
| 21-225_20A7_HC | . | . | . | . | . | . | . | . | . | . | . | . | . | . | . | . | . | . | . | . | . | . | . | . | . | . |
| 21-225_20D10_HC | . | . | . | . | D | . | . | W | . | . | . | . | . | . | . | . | . | . | . | . | . | . | . | . | . | . |
| 21-225_20D5_HC | . | . | . | . | . | . | . | . | . | . | . | . | . | . | . | . | . | . | . | . | . | . | . | . | . | . |
| 21-225_20E12_HC | . | . | . | . | . | . | . | . | . | . | . | . | . | . | . | . | . | . | . | . | . | . | . | . | . | . |
| 21-225_20E5_HC | . | . | . | . | . | . | . | . | . | . | . | . | . | . | . | . | . | . | . | . | . | . | . | . | . | . |
| 21-225_20H12_HC | . | . | . | . | . | . | . | . | . | . | . | . | . | . | . | . | . | . | . | . | . | . | . | . | . | . |
| 21-225_21E5_HC | . | . | T | . | . | . | . | . | . | . | . | . | . | . | . | . | . | . | . | . | . | . | . | . | . | . |
| 21-225_21F7_HC | . | . | . | . | D | . | . | W | . | . | . | . | . | . | . | . | . | . | . | . | . | . | . | . | . | . |
| 21-225_21G11_HC | . | . | . | . | . | . | . | W | . | . | . | . | . | . | . | . | . | . | . | . | . | . | . | . | . | . |
| 21-225_21G2_HC | . | . | . | . | . | . | . | W | . | . | . | . | . | . | . | . | . | . | . | . | . | . | . | . | . | . |
| 21-225_21H8_HC | . | . | . | . | D | . | . | W | . | . | . | . | . | . | . | . | . | . | . | . | . | . | . | . | . | . |
| 21-225_224D10_HC | . | . | V | . | . | . | . | . | . | . | . | . | . | . | . | . | . | . | . | . | . | . | . | . | . | . |
| 21-225_22A4_HC | . | . | . | . | D | . | . | W | . | . | . | . | . | . | . | . | . | . | . | . | . | . | . | . | . | . |
| 21-225_22F2_HC | . | . | V | . | . | . | . | . | . | . | . | . | . | . | . | . | . | . | . | . | . | . | . | . | . | . |
| 21-225_22F9_HC | . | . | V | . | . | . | . | . | . | . | . | . | . | . | . | . | . | . | . | . | . | . | . | . | . | . |
| 21-225_22H8_HC | . | . | . | . | . | . | A | W | . | . | . | . | . | . | . | . | . | . | . | . | . | . | . | . | . | . |
| 21-225_23F12_HC | . | . | . | . | . | . | A | W | . | . | . | . | . | . | . | . | . | . | . | . | . | . | . | . | . | . |
| 21-225_23H11_HC | . | . | . | . | . | . | . | . | . | . | . | . | . | . | . | . | . | . | . | . | . | . | . | . | . | . |
| 21-225_25B6_HC | . | . | . | . | . | . | . | . | . | . | . | . | . | . | . | . | . | . | . | . | . | . | . | . | . | . |
| 21-225_25E6_HC | . | . | . | T | . | . | . | M | . | . | . | . | . | . | . | . | . | . | . | . | . | . | . | . | . | . |
| 21-225_25H10_HC | . | . | . | . | . | . | . | M | . | . | . | . | . | . | . | . | . | . | . | . | . | . | . | . | . | . |
| 21-225_25H11_HC | . | . | . | . | . | . | . | M | . | . | . | . | . | . | . | . | . | . | . | . | . | . | . | . | . | . |
| 21-225_26A2_HC | . | . | . | . | . | . | . | W | . | . | . | . | . | . | . | . | . | . | . | . | . | . | . | . | . | . |
| 21-225_27C3_HC | . | . | . | . | . | . | . | M | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | E | L | G | F | - | - | - | - | - | - | - | - | - | - | - | - | - | - | - | - | - | - |
| 21-225_27D5_HC | . | . | . | . | . | . | . | . | . | . | . | . | . | . | . | . | . | . | . | . | . | . | . | . | . | . |
| 21-225_27F2_HC | . | . | . | . | . | . | . | M | . | . | . | . | . | . | . | . | . | . | . | . | . | . | . | . | . | . |
| 21-225_28C7_HC | . | . | . | . | . | . | . | W | . | . | . | . | . | . | . | . | . | . | . | . | . | . | . | . | . | . |
| 21-225_28F5_HC | . | . | . | . | . | . | . | W | . | . | . | . | . | . | . | . | . | . | . | . | . | . | . | . | . | . |
| 21-225_29B8_HC | . | . | . | . | . | . | . | M | . | . | . | . | . | . | . | . | . | . | . | . | . | . | . | . | . | . |
| 21-225_29D7_HC | . | . | . | . | . | . | . | . | . | . | . | . | . | . | . | . | . | . | . | . | . | . | . | . | . | . |
| 21-225_29G4_HC | . | . | . | . | . | . | . | M | . | . | . | . | . | . | . | . | . | . | . | . | . | . | . | . | . | . |
| 21-225_29G8_HC | . | . | . | . | . | . | . | M | . | . | . | . | . | . | . | . | . | . | . | . | . | . | . | . | . | . |
| 21-225_29H6_HC | . | . | . | . | . | . | . | M | . | . | . | . | . | . | . | . | . | . | . | . | . | . | . | . | . | . |
| 21-225_2B2_HC | . | . | V | . | . | . | . | . | . | . | . | . | . | . | . | . | . | . | . | . | . | . | . | . | . | . |
| 21-225_43C3_HC | . | . | . | . | . | . | . | . | . | . | . | . | . | . | . | . | . | . | . | . | . | . | . | . | . | . |
| 21-225_44C3_HC | . | . | V | . | . | . | . | . | . | . | . | . | . | . | . | . | . | . | . | . | . | . | . | . | . | . |
| 21-225_44C8_HC | . | . | V | . | . | . | . | . | . | . | . | . | . | . | . | . | . | . | . | . | . | . | . | . | . | . |
| 21-225_46C6_HC | . | . | . | . | . | . | . | . | . | . | . | . | . | . | . | . | . | . | . | . | . | . | . | . | . | . |
| 21-225_46D8_HC | . | . | V | . | . | . | . | . | . | . | . | . | . | . | . | . | . | . | . | . | . | . | . | . | . | . |
| 21-225_46F3_HC | . | . | V | . | . | . | . | . | . | . | . | . | . | . | . | . | . | . | . | . | . | . | . | . | . | . |
| 21-225_47A1_HC | . | . | . | T | . | . | . | M | . | . | . | . | . | . | . | . | . | . | . | . | . | . | . | . | . | . |
| 21-225_48A9_HC | F | . | . | . | . | . | A | W | . | . | . | . | . | . | . | . | . | . | . | . | . | . | . | . | . | . |
| 21-225_48C1_HC | . | . | . | . | . | . | . | W | . | . | . | . | . | . | . | . | . | . | . | . | . | . | . | . | . | . |
| 21-225_48D12_HC | . | . | . | . | . | . | . | M | . | . | . | . | . | . | . | . | . | . | . | . | . | . | . | . | . | . |
| 21-225_49A1_HC | . | . | . | . | . | . | . | . | . | . | . | . | . | . | . | . | . | . | . | . | . | . | . | . | . | . |
| 21-225_49C6_HC | . | . | . | . | D | . | . | M | . | . | . | . | . | . | . | . | . | . | . | . | . | . | . | . | . | . |
| 21-225_51B11_HC | . | . | . | . | . | . | . | . | . | . | . | . | . | . | . | . | . | . | . | . | . | . | . | . | . | . |
| 21-225_51E4_HC | . | . | . | . | . | . | . | . | . | . | . | . | . | . | . | . | . | . | . | . | . | . | . | . | . | . |
| 21-225_51F11_HC | . | . | . | . | . | . | . | . | . | . | . | . | . | . | . | . | . | . | . | . | . | . | . | . | . | . |
| 21-225_51F9_HC | . | . | . | . | . | . | . | . | . | . | . | . | . | . | . | . | . | . | . | . | . | . | . | . | . | . |
| 21-225_52B2_HC | . | . | . | . | D | . | . | M | . | . | . | . | . | . | . | . | . | . | . | . | . | . | . | . | . | . |
| 21-225_52B9_HC | . | . | . | . | . | . | . | . | . | . | . | . | . | . | . | . | . | . | . | . | . | . | . | . | . | . |
| 21-225_53B12_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | E | L | G | F | - | - | - | - | - | - | - | - | - | - | - | - | - | - | - | - | - | - |
| 21-225_9E8_HC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | | S | D | Y | W | G | Q | G | T | L | V | T | V | S | S |
| 21-225_10G5_HC | . | . | . | . | R | . | . | . | . | . | . | . | . | . | . | . | . | . | . |
| 21-225_10H12_HC | . | . | . | . | R | . | . | . | . | . | . | . | . | . | . | . | . | . | . |
| 21-225_12A1_HC | . | . | . | . | R | . | . | . | . | . | . | . | . | . | . | . | . | . | . |
| 21-225_12B12_HC | . | . | . | . | Y | E | . | . | . | . | . | . | . | . | . | . | . | . | . |
| 21-225_12E6_HC | . | . | . | . | Y | E | . | . | . | . | . | . | . | . | . | . | . | . | . |
| 21-225_12F2_HC | . | . | . | . | S | E | . | . | . | . | . | . | . | . | . | . | . | . | . |
| 21-225_13E6_HC | . | . | . | . | R | . | . | . | . | . | . | . | . | . | . | . | . | . | . |
| 21-225_146H9_HC | . | . | . | . | L | . | . | . | . | . | . | . | . | . | . | . | . | . | . |
| 21-225_14E3_HC | . | . | . | . | R | . | . | . | . | . | . | . | . | . | . | . | . | . | . |
| 21-225_157C3_HC | . | . | . | . | L | . | . | . | . | . | . | . | . | . | . | . | . | . | . |
| 21-225_157H8_HC | . | . | . | . | L | . | . | . | . | . | . | . | . | . | . | . | . | . | . |
| 21-225_158H6_HC | . | . | . | . | A | E | . | . | . | . | . | . | . | . | . | . | . | . | . |
| 21-225_159C5_HC | . | . | . | . | L | . | . | . | . | . | . | . | . | . | . | . | . | . | . |
| 21-225_15E3_HC | . | . | . | . | L | . | . | . | . | . | . | . | . | . | . | . | . | . | . |
| 21-225_16A5_HC | . | . | . | . | T | E | E | . | . | . | . | . | . | . | . | . | . | . | . |
| 21-225_16B7_HC | . | . | . | . | L | . | . | . | . | . | . | . | . | . | . | . | . | . | . |
| 21-225_16F10_HC | . | . | . | . | Y | E | . | . | . | . | . | . | . | . | . | . | . | . | . |
| 21-225_16F11_HC | . | . | . | . | Q | . | . | . | . | . | . | . | . | P | . | . | . | . | . |
| 21-225_16F4_HC | . | . | . | . | L | . | . | . | . | . | . | . | . | . | . | . | . | . | . |
| 21-225_16F6_HC | . | . | . | . | L | . | . | . | . | . | . | . | . | . | . | . | . | . | . |
| 21-225_16G7_HC | . | . | . | . | Y | E | . | . | . | . | . | . | . | . | . | . | . | . | . |
| 21-225_17A10_HC | . | . | . | . | R | . | . | . | . | . | . | . | . | . | . | . | . | . | . |
| 21-225_17B5_HC | . | . | . | . | R | P | . | . | . | . | . | . | . | . | . | . | . | . | . |
| 21-225_17C7_HC | . | . | . | . | T | E | E | . | . | . | . | . | . | . | . | . | . | . | . |
| 21-225_17E5_HC | . | . | . | . | Y | E | . | . | . | . | . | . | . | . | . | . | . | . | . |
| 21-225_18A1_HC | . | . | . | . | Q | . | . | . | . | . | . | . | . | P | . | . | . | . | . |
| 21-225_18A5_HC | . | . | . | . | T | E | E | . | . | . | . | . | . | . | . | . | . | . | . |
| 21-225_18C2_HC | . | . | . | . | Y | E | . | . | . | . | . | . | . | . | . | . | . | . | . |
| 21-225_18E8_HC | . | . | . | . | R | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | | S | D | Y | W | G | Q | G | T | L | V | T | V | S | S |
| 21-225_18F2_HC | . | . | . | . | T | E | E | . | . | . | . | . | . | . | . | . | . | . | . |
| 21-225_19E1_HC | . | . | . | . | R | . | . | . | . | . | . | . | . | . | . | . | . | . | . |
| 21-225_1C12_HC | . | . | . | . | R | . | . | . | . | . | . | . | . | . | . | . | . | . | . |
| 21-225_203B9_HC | . | . | . | . | L | . | . | . | . | . | . | . | I | . | . | . | . | . | . |
| 21-225_207F12_HC | . | . | . | . | L | F | . | . | . | . | . | . | . | . | . | . | . | . | . |
| 21-225_20A7_HC | . | . | . | . | R | . | . | . | . | . | . | . | . | . | . | . | . | . | . |
| 21-225_20D10_HC | . | . | . | . | T | E | E | . | . | . | . | . | . | . | . | . | . | . | . |
| 21-225_20D5_HC | . | . | . | . | R | . | . | . | . | . | . | . | . | . | . | . | . | . | . |
| 21-225_20E12_HC | . | . | . | . | R | F | . | . | . | . | . | . | . | . | . | . | . | . | . |
| 21-225_20E5_HC | . | . | . | . | R | . | . | . | . | . | . | . | . | . | . | . | . | . | . |
| 21-225_20H12_HC | . | . | . | . | R | . | . | . | . | . | . | . | . | . | . | . | . | . | . |
| 21-225_21E5_HC | . | . | . | . | R | . | . | . | . | . | . | . | . | . | . | . | . | . | . |
| 21-225_21F7_HC | . | . | . | . | T | E | E | . | . | . | . | . | . | . | . | . | . | . | . |
| 21-225_21G11_HC | . | . | . | . | Y | E | . | . | . | . | . | . | . | . | . | . | . | . | . |
| 21-225_21G2_HC | . | . | . | . | Y | E | . | . | . | . | . | . | . | . | . | . | . | . | . |
| 21-225_21H8_HC | . | . | . | . | T | E | E | . | . | . | . | . | . | . | . | . | . | . | . |
| 21-225_224D10_HC | . | . | . | . | Q | . | . | . | . | . | . | . | . | P | . | . | . | . | . |
| 21-225_22A4_HC | . | . | . | . | T | E | E | . | . | . | . | . | . | . | . | . | . | . | . |
| 21-225_22F2_HC | . | . | . | . | Q | . | . | . | . | . | . | . | . | P | . | . | . | . | . |
| 21-225_22F9_HC | . | . | . | . | Q | . | . | . | . | . | . | . | . | P | . | . | . | . | . |
| 21-225_22H8_HC | . | . | . | . | Y | E | . | . | . | . | . | . | . | . | . | . | . | . | . |
| 21-225_23F12_HC | . | . | . | . | Y | E | . | . | . | . | . | . | S | . | . | . | . | . | . |
| 21-225_23H11_HC | . | . | . | . | R | . | . | . | . | . | . | . | . | . | . | . | . | . | . |
| 21-225_25B6_HC | . | . | . | . | L | . | . | . | . | . | . | . | . | . | . | . | . | . | . |
| 21-225_25E6_HC | . | . | . | . | T | G | . | S | . | . | . | . | . | . | . | . | . | . | . |
| 21-225_25H10_HC | . | . | . | . | T | G | . | . | . | . | . | . | . | . | . | . | . | . | . |
| 21-225_25H11_HC | . | . | . | . | T | G | . | S | . | . | . | . | . | . | . | . | . | . | . |
| 21-225_26A2_HC | . | . | . | . | Y | D | . | . | . | . | . | . | . | . | . | . | . | . | . |
| 21-225_27C3_HC | . | . | . | . | T | G | . | S | . | . | . | . | . | . | . | . | . | . | . |

# FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | | S | D | Y | W | G | Q | G | T | L | V | T | V | S | S |
| 21-225_27D5_HC | . | . | . | . | L | . | . | . | . | . | . | . | . | . | . | . | . | . | . |
| 21-225_27F2_HC | . | . | . | . | T | G | . | S | . | . | . | . | . | . | . | . | . | . | . |
| 21-225_28C7_HC | . | . | . | . | Y | D | . | . | . | . | . | . | . | . | . | . | . | . | . |
| 21-225_28F5_HC | . | . | . | . | Y | D | . | . | . | . | . | . | . | . | . | . | . | . | . |
| 21-225_29B8_HC | . | . | . | . | T | G | . | S | . | . | . | . | . | . | . | . | . | . | . |
| 21-225_29D7_HC | . | . | . | . | L | . | . | . | . | . | . | . | . | . | . | . | . | . | . |
| 21-225_29G4_HC | . | . | . | . | T | G | . | . | . | . | . | . | . | . | . | . | . | . | . |
| 21-225_29G8_HC | . | . | . | . | T | . | . | . | . | . | . | . | . | . | . | . | . | . | . |
| 21-225_29H6_HC | . | . | . | . | T | G | . | S | . | . | . | . | . | . | . | . | . | . | . |
| 21-225_2B2_HC | . | . | . | . | Q | . | . | . | . | . | . | . | . | P | . | . | . | . | . |
| 21-225_43C3_HC | . | . | . | . | S | N | . | . | . | . | . | . | . | . | . | . | . | . | . |
| 21-225_44C3_HC | . | . | . | . | S | T | . | . | . | . | . | . | . | . | . | . | . | . | . |
| 21-225_44C8_HC | . | . | . | . | L | . | . | . | . | . | . | . | . | . | . | . | . | . | . |
| 21-225_46C6_HC | . | . | . | . | S | N | . | . | . | . | . | . | . | . | . | . | . | . | . |
| 21-225_46D8_HC | . | . | . | . | S | N | . | . | . | . | . | . | . | . | . | . | . | . | . |
| 21-225_46F3_HC | . | . | . | . | S | N | . | . | . | . | . | . | . | . | . | . | . | . | . |
| 21-225_47A1_HC | . | . | . | . | L | F | . | . | . | . | . | . | . | . | . | . | . | . | . |
| 21-225_48A9_HC | . | . | . | . | Y | E | . | . | . | . | . | . | . | . | . | . | . | . | . |
| 21-225_48C1_HC | . | . | . | . | E | A | . | . | . | . | . | . | . | . | . | . | . | . | . |
| 21-225_48D12_HC | . | . | . | . | R | . | . | . | . | . | . | . | . | . | . | . | . | . | . |
| 21-225_49A1_HC | . | . | . | . | L | . | . | . | . | . | . | . | . | . | . | . | . | . | . |
| 21-225_49C6_HC | . | . | . | . | I | E | . | . | . | . | . | . | . | . | . | . | . | . | . |
| 21-225_51B11_HC | . | . | . | . | L | . | . | . | . | . | . | . | . | . | . | . | . | . | . |
| 21-225_51E4_HC | . | . | . | . | L | . | . | . | . | . | . | . | . | . | . | . | . | . | . |
| 21-225_51F11_HC | . | . | . | . | L | . | . | F | . | . | . | . | . | . | . | . | . | . | . |
| 21-225_51F9_HC | . | . | . | . | L | . | . | . | . | . | . | . | . | . | . | . | . | . | . |
| 21-225_52B2_HC | . | . | . | . | I | E | . | F | . | . | . | . | . | . | . | . | . | . | . |
| 21-225_52B9_HC | . | . | . | . | L | . | . | . | . | . | . | . | . | . | . | . | . | . | . |
| 21-225_53B12_HC | . | . | . | . | L | . | . | F | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | | S | D | Y | W | G | Q | G | T | L | V | T | V | S | S |
| 21-225_53D2_HC | . | . | . | . | T | G | . | . | . | . | . | . | A | . | . | . | . | . | . |
| 21-225_53E6_HC | . | . | . | . | T | . | . | . | . | . | . | . | . | . | . | . | . | . | . |
| 21-225_54C6_HC | . | . | . | . | T | . | . | . | . | . | . | . | . | . | . | . | . | . | . |
| 21-225_54D3_HC | . | . | . | . | L | . | . | . | . | . | . | . | . | . | . | . | . | . | . |
| 21-225_54G7_HC | . | . | . | . | L | . | . | . | . | . | . | . | . | . | . | . | . | . | . |
| 21-225_55B3_HC | . | . | . | . | L | . | . | . | . | . | . | . | . | . | . | . | . | . | . |
| 21-225_57A4_HC | . | . | . | . | R | . | . | . | . | . | . | . | . | . | . | . | . | . | . |
| 21-225_58F5_HC | . | . | . | . | R | . | . | . | . | . | . | . | . | . | . | . | . | . | . |
| 21-225_59F11_HC | . | . | . | . | N | G | . | . | . | . | . | . | . | . | . | . | . | . | . |
| 21-225_60A3_HC | . | . | . | . | T | G | . | . | . | . | . | . | . | . | . | . | . | . | . |
| 21-225_62E4_HC | . | . | . | . | S | . | . | . | . | . | . | . | . | . | . | . | . | . | . |
| 21-225_64E1_HC | . | . | . | . | S | . | . | . | . | . | . | . | . | . | . | . | . | . | . |
| 21-225_65C1_HC | . | . | . | . | S | . | . | . | . | . | . | . | . | . | . | . | . | . | . |
| 21-225_66C7_HC | . | . | . | . | S | . | . | . | . | . | . | . | . | . | . | . | . | . | . |
| 21-225_68F11_HC | . | . | . | . | T | . | . | C | . | . | . | . | . | . | . | . | . | . | . |
| 21-225_6A11_HC | . | . | . | . | T | E | E | . | . | . | . | . | . | . | . | . | . | . | . |
| 21-225_6G12_HC | . | . | . | . | R | . | . | . | . | . | . | . | . | . | . | . | . | . | . |
| 21-225_6G2_HC | . | . | . | . | T | E | E | . | . | . | . | . | . | . | . | . | . | . | . |
| 21-225_71A2_HC | . | . | . | . | Q | D | . | . | . | . | . | . | . | . | . | . | . | . | . |
| 21-225_71B11_HC | . | . | . | . | L | . | . | . | . | . | . | . | . | . | . | . | . | . | . |
| 21-225_71B6_HC | . | . | . | . | L | . | . | . | . | . | . | . | . | . | . | . | . | . | . |
| 21-225_72G12_HC | . | . | . | . | S | . | . | . | . | . | . | . | . | . | . | . | . | . | . |
| 21-225_7C4_HC | . | . | . | . | R | . | . | . | . | . | . | . | . | . | . | . | . | . | . |
| 21-225_7E9_HC | . | . | . | . | R | . | . | . | . | . | . | . | . | . | . | . | . | . | . |
| 21-225_7G2_HC | . | . | . | . | Y | E | . | . | . | . | . | . | . | . | . | . | . | . | . |
| 21-225_7H11_HC | . | . | . | . | R | . | . | . | . | . | . | . | . | . | . | . | . | . | . |
| 21-225_8C9_HC | . | . | . | . | R | . | . | . | . | . | . | . | . | . | . | . | . | . | . |
| 21-225_8H7_HC | . | . | . | . | R | . | . | . | . | . | . | . | . | . | . | . | . | . | . |
| 21-225_9A1_HC | . | . | . | . | T | E | E | . | . | . | . | . | . | P | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | | S | D | Y | W | G | Q | G | T | L | V | T | V | S | S |
| 21-225_9E8_HC | . | . | . | . | R | . | . | . | . | . | . | . | . | . | . | . | . | . | . |

**FIGURE 57**

Table 52. Consensus 18- VH4|4-34/D4|4-17|RF2/JH6 (SEQ ID NO: 50269):

QVQLQQW-GAGLLKPSETLSLTCAVHG-GSFSG-----CYWSWIRQPPGKGLEWIGEINH----SGSTNYNPSLKSRVTISVDTSKNQFSLKLSSVTAADTAVYYCARDYGG----------------------MDVWGQGTTVTVSS wherein:

C at position 39 can be substituted with S, P or Y

H at position 60 can be substituted with Y or Q

S at position 65 can be substituted with R

S at position 67 can be substituted with R, C, or I

N at position 69 can be substituted with S

Y at position 70 can be substituted with F

K at position 75 can be substituted with T

M at position 136 can be substituted with L or I

Gly Xaa Tyr Trp Ser (SEQ ID NO: 50010)

Glu Ile Asn Xaa Xaa Gly Xaa Thr Xaa Xaa Asn Pro Ser Leu Xaa Ser (SEQ ID NO: 50011)

Asp Tyr Gly Gly Xaa Asp Val (SEQ ID NO: 50012)

Gly Cys Tyr Trp Ser (SEQ ID NO: 50477)

Glu Ile Asn His Ser Gly Ser Thr Asn Tyr Asn Pro Ser Leu Lys Ser (SEQ ID NO: 50478)

Asp Tyr Gly Gly Met Asp Val (SEQ ID NO: 50479)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | Q | Q | W | - | G | A | G | L | L | K | P | S | E | T | L | S | L | T | C | A | V | H |
| 21-225_74A3_HC | . | . | . | . | . | . | . | . | . | . | . | . | . | . | . | . | . | . | . | . | . | . | . | . | . | . |
| 21-225_74A4_HC | . | . | . | . | . | . | . | . | . | . | . | . | . | . | . | . | . | . | . | . | . | . | . | . | . | . |
| 21-225_74B12_HC | . | . | . | . | . | . | . | . | . | . | . | . | . | . | . | . | . | . | . | . | . | . | . | . | . | . |
| 21-225_74B2_HC | . | . | . | . | . | . | . | . | . | . | . | . | . | . | . | . | . | P | . | . | . | . | . | . | . | Y |
| 21-225_74C5_HC | . | . | . | . | . | . | . | . | . | . | . | . | . | . | . | . | . | . | . | . | . | . | . | . | . | Y |
| 21-225_74D11_HC | . | . | . | . | . | . | . | . | . | . | . | . | . | . | . | . | . | . | . | . | . | . | . | . | . | . |
| 21-225_74D5_HC | . | . | . | . | . | . | . | . | . | . | . | . | . | . | . | . | . | . | . | . | . | . | . | . | . | N |
| 21-225_74D6_HC | . | . | . | . | . | . | . | . | . | . | . | . | . | . | . | . | . | . | . | . | . | . | . | . | . | . |
| 21-225_74F5_HC | . | . | . | . | . | . | . | . | . | . | . | . | . | . | . | . | . | . | . | . | . | . | . | . | . | . |
| 21-225_74H5_HC | . | . | . | . | . | . | . | . | . | . | . | . | . | . | . | . | . | . | . | . | . | . | . | . | . | N |
| 21-225_74H7_HC | . | . | . | . | . | . | . | . | . | . | . | . | . | . | . | . | . | P | . | . | . | . | . | . | . | Y |
| 21-225_75A12_HC | . | . | . | . | . | . | G | . | . | . | . | P | . | . | . | . | . | . | . | . | . | . | . | . | . | Y |
| 21-225_75A5_HC | . | . | . | . | . | . | . | . | . | . | . | . | . | . | . | . | . | . | . | . | . | . | . | . | . | . |
| 21-225_75A8_HC | . | . | . | . | . | . | . | . | . | . | . | . | . | . | . | . | . | . | . | . | . | . | . | . | . | . |
| 21-225_75B10_HC | . | . | . | . | . | . | . | . | . | . | . | . | . | . | . | . | . | . | . | . | . | K | . | D | . | Y |
| 21-225_75C2_HC | . | . | . | . | . | . | . | . | . | . | . | . | . | . | . | . | . | . | . | . | . | . | . | . | . | . |
| 21-225_75C3_HC | . | . | . | . | . | . | G | . | . | . | . | P | . | . | . | . | . | . | . | . | . | . | . | . | . | Y |
| 21-225_75D3_HC | . | . | . | . | . | . | . | . | . | . | . | . | . | . | . | . | . | . | . | . | . | . | . | . | . | . |
| 21-225_75D9_HC | . | . | . | . | . | . | . | . | . | . | . | . | . | . | . | . | . | . | . | . | . | . | . | . | . | Y |
| 21-225_75F3_HC | . | . | . | . | . | . | . | . | . | . | . | . | . | . | . | . | . | . | . | . | . | . | . | . | . | . |
| 21-225_75G12_HC | . | . | . | . | . | . | . | . | . | . | . | . | . | . | . | . | . | . | . | . | . | . | . | . | . | . |
| 21-225_75G7_HC | . | . | . | . | . | . | . | . | . | . | . | . | . | . | . | . | . | . | . | . | . | . | . | T | . | Y |
| 21-225_75H8_HC | . | . | . | . | . | . | . | . | . | . | . | . | . | . | . | . | . | P | . | . | . | . | . | . | . | Y |
| 21-225_76A4_HC | . | . | . | . | . | . | . | . | . | . | . | . | . | . | . | . | . | . | . | . | . | . | . | . | . | . |
| 21-225_76D1_HC | . | . | . | . | . | . | . | . | . | . | . | . | . | . | . | . | . | . | . | . | . | . | . | . | . | Y |
| 21-225_76E10_HC | . | . | . | . | . | . | . | . | . | . | . | . | . | . | . | . | . | . | . | . | . | . | . | . | . | N |
| 21-225_76G4_HC | . | . | . | . | . | . | . | . | . | . | . | . | . | . | . | . | . | . | . | . | . | . | . | . | . | . |
| 21-225_76H10_HC | . | . | . | . | . | . | . | . | . | . | . | . | . | . | . | . | . | . | . | . | . | . | . | . | . | N |
| 21-225_77A10_HC | . | . | . | . | . | . | G | . | . | . | . | P | . | . | . | . | . | . | . | . | . | . | . | . | . | Y |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | Q | Q | W | - | G | A | G | L | L | K | P | S | E | T | L | S | L | T | C | A | V | H |
| 21-225_77C12_HC | . | . | . | . | . | . | . | . | . | . | . | . | . | . | . | . | . | . | . | . | . | . | . | . | . | Y |
| 21-225_77C5_HC | . | . | . | . | . | . | . | . | . | . | . | . | . | . | . | . | . | . | . | . | . | . | . | . | . | . |
| 21-225_77F7_HC | . | . | . | . | . | . | . | . | . | . | . | . | . | . | . | . | . | . | . | . | . | . | . | . | . | . |
| 21-225_77G1_HC | . | . | . | . | . | . | . | . | . | . | . | . | . | . | . | . | . | . | . | . | . | . | . | . | . | . |
| 21-225_79F11_HC | . | . | . | . | . | . | . | . | . | . | . | . | . | . | . | . | . | . | . | . | . | . | . | . | . | . |
| 21-225_79F3_HC | . | . | . | . | . | . | . | . | . | . | . | . | . | . | . | . | . | . | . | . | . | . | . | . | . | . |
| 21-225_79G1_HC | . | . | . | . | . | . | . | . | . | . | . | . | . | . | . | . | . | . | . | . | . | . | . | . | . | . |
| 21-225_79G12_HC | . | . | . | . | . | . | . | . | . | . | . | . | . | . | . | . | . | . | . | . | . | . | . | . | . | . |
| 21-225_80A1_HC | . | . | . | . | . | . | . | . | . | . | . | . | . | . | . | . | . | . | . | . | . | . | . | . | . | . |
| 21-225_80C1_HC | . | . | . | . | . | . | . | . | . | . | . | . | . | . | . | . | . | . | . | . | . | . | . | . | . | . |
| 21-225_80D5_HC | . | . | . | . | . | . | . | . | . | . | . | . | . | . | . | . | . | P | . | . | . | . | . | . | . | Y |
| 21-225_80E12_HC | . | . | . | . | . | . | . | . | . | . | . | . | . | . | . | . | . | . | . | . | . | . | . | . | . | N |
| 21-225_80E3_HC | . | . | . | . | . | . | . | . | . | . | . | . | . | . | . | . | . | . | . | . | . | . | . | . | . | . |
| 21-225_80H12_HC | . | . | . | . | . | . | . | . | . | . | . | . | . | . | . | . | . | . | . | . | . | . | . | . | . | . |
| 21-225_80H7_HC | . | . | . | . | . | . | . | . | . | . | . | . | . | . | . | . | . | . | . | . | . | . | . | . | . | Y |
| 21-225_81C11_HC | . | . | . | . | . | . | . | . | . | . | . | . | . | . | . | . | . | . | . | . | . | . | . | . | . | . |
| 21-225_81C5_HC | . | . | . | . | . | . | . | . | . | . | . | . | . | . | . | . | . | . | . | . | . | . | . | . | . | . |
| 21-225_83B7_HC | . | . | . | . | . | . | . | . | . | . | . | . | . | . | . | . | . | . | . | . | . | . | . | . | . | . |
| 21-225_83G1_HC | . | . | . | . | . | . | . | . | . | . | . | . | . | . | . | . | . | . | . | . | . | . | . | . | . | . |
| 21-225_84E12_HC | . | . | . | . | . | . | . | . | . | . | . | . | . | . | . | . | . | . | . | . | . | . | . | . | . | Y |
| 21-225_85A3_HC | . | . | . | . | . | . | . | . | . | . | . | . | . | . | . | . | . | . | . | . | . | . | . | . | . | . |
| 21-225_85B4_HC | . | . | . | . | . | . | . | . | . | . | . | . | . | . | . | . | . | . | . | . | . | . | . | . | . | . |
| 21-225_85B9_HC | . | . | . | . | . | . | . | . | . | . | . | . | . | . | . | . | . | . | . | . | . | . | . | . | . | N |
| 21-225_85D6_HC | . | . | . | . | . | . | G | . | . | . | . | P | . | . | . | . | . | . | . | . | . | . | . | . | . | Y |
| 21-225_85G10_HC | . | . | . | . | . | . | R | . | . | . | . | . | . | . | . | . | . | . | . | . | . | . | . | . | . | Y |
| 21-225_86C1_HC | . | . | . | . | . | . | . | . | . | . | . | . | . | . | . | . | . | . | . | . | . | . | . | . | . | . |
| 21-225_86C11_HC | . | . | . | . | . | . | . | . | . | . | . | . | . | . | . | . | . | . | . | . | . | . | . | . | . | . |
| 21-225_86E4_HC | . | . | . | . | . | . | . | . | . | . | . | . | . | . | . | . | . | . | . | . | . | . | . | . | . | . |
| 21-225_87A12_HC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | Q | Q | W | - | G | A | G | L | L | K | P | S | E | T | L | S | L | T | C | A | V | H |
| 21-225_87C9_HC | . | . | . | . | . | . | . | . | . | . | . | . | . | . | . | . | . | . | . | . | . | . | . | . | . | . |
| 21-225_87E5_HC | . | . | . | . | . | . | . | . | . | . | . | . | . | . | . | . | . | . | . | . | . | . | . | . | . | . |
| 21-225_87H1_HC | . | . | . | . | . | P | . | . | . | . | . | . | . | . | . | . | . | . | . | . | . | . | . | . | . | Y |
| 21-225_88E7_HC | . | . | . | . | . | . | . | . | . | . | . | . | . | . | . | . | . | . | . | . | . | . | . | . | . | . |
| 21-225_88F7_HC | . | . | . | . | . | . | . | . | . | . | . | . | . | . | . | . | . | . | . | . | . | . | . | . | . | . |
| 21-225_88G9_HC | . | . | . | . | . | . | . | . | . | . | . | . | . | . | . | . | . | . | . | . | . | . | . | . | . | . |
| 21-225_88H1_HC | . | . | . | . | . | . | . | . | . | . | . | . | . | . | . | . | . | . | . | . | . | . | . | . | . | . |
| 21-225_89A11_HC | . | . | . | . | . | P | . | . | . | . | . | . | . | . | . | . | . | . | . | . | . | . | . | . | . | Y |
| 21-225_89D5_HC | . | . | . | . | . | . | . | . | . | . | . | . | . | . | . | . | . | . | . | . | . | . | . | . | . | . |
| 21-225_89E10_HC | . | . | . | . | . | . | . | . | . | . | . | . | . | . | . | . | . | . | . | . | . | . | . | . | . | . |
| 21-225_89H5_HC | . | . | . | . | . | . | . | . | . | . | . | . | . | . | . | . | . | . | . | . | . | . | . | . | . | Y |
| 21-225_90A5_HC | . | . | . | . | . | . | . | . | . | . | . | . | . | . | . | . | . | . | . | . | . | . | . | . | . | . |
| 21-225_90F10_HC | . | . | . | . | . | . | . | . | . | . | . | . | . | . | . | . | . | . | . | . | . | . | . | . | . | . |
| 21-225_90G4_HC | . | . | . | . | . | . | . | . | . | . | . | . | . | . | . | . | . | . | . | . | . | . | . | . | . | . |
| 21-225_90G5_HC | . | . | . | . | . | . | . | . | . | . | . | . | . | . | . | . | . | . | . | . | . | . | . | . | . | . |
| 21-225_91B2_HC | . | . | . | . | . | . | . | . | . | . | . | . | . | . | . | . | . | . | . | . | . | . | . | . | . | . |
| 21-225_91B3_HC | . | . | . | . | . | . | . | . | . | . | . | . | . | . | . | . | . | . | . | . | . | . | . | . | . | . |
| 21-225_91B4_HC | . | . | . | . | . | . | . | . | . | . | . | . | . | . | . | . | . | . | . | . | . | . | . | . | . | . |
| 21-225_91E9_HC | . | . | . | . | . | . | . | . | . | . | . | . | . | . | . | . | . | . | . | . | . | . | . | . | . | . |
| 21-225_91F3_HC | . | . | . | . | . | . | . | . | . | . | . | . | . | . | . | . | . | . | . | . | . | . | . | . | . | . |
| 21-225_92B1_HC | . | . | . | . | . | . | . | . | . | . | . | . | . | . | . | . | . | . | . | . | . | . | . | . | . | Y |
| 21-225_92D6_HC | . | . | . | . | . | . | . | . | . | . | . | . | . | . | . | . | . | . | . | . | . | . | . | . | . | . |
| 21-225_93C2_HC | . | . | . | . | . | . | . | . | . | . | . | . | . | . | . | . | . | . | . | . | . | . | . | . | . | . |
| 21-225_93E4_HC | . | . | . | . | . | . | . | . | . | . | . | . | . | . | . | . | . | . | . | . | . | . | . | . | . | . |
| 21-225_94D3_HC | . | . | . | . | . | . | G | . | . | . | . | P | . | . | . | . | . | . | . | . | . | . | . | . | . | Y |
| 21-225_94F12_HC | . | . | . | . | . | . | . | . | . | . | . | . | . | . | . | . | . | . | . | . | . | . | . | . | . | . |
| 21-225_94G10_HC | . | . | . | . | . | . | . | . | . | . | . | . | . | . | . | . | . | . | . | . | . | . | . | . | . | N |
| 21-225_95D2_HC | . | . | . | . | . | . | . | . | . | . | . | . | . | . | . | . | . | . | . | . | . | . | . | . | . | . |
| 21-225_95F9_HC | . | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | Q | Q | W | - | G | A | G | L | L | K | P | S | E | T | L | S | L | T | C | A | V | H |
| 21-225_95G9_HC | . | . | . | . | . | . | . | . | . | . | . | . | . | . | . | . | . | . | . | . | . | . | . | . | . | . |
| 21-225_95H10_HC | . | . | . | . | . | . | . | . | . | . | . | . | . | . | . | . | . | . | . | . | . | . | . | . | . | . |
| 21-225_97A2_HC | . | . | . | . | . | . | . | . | . | . | . | . | . | . | . | . | . | . | . | . | . | . | . | . | . | . |
| 21-225_97E5_HC | . | . | . | . | . | . | . | . | . | . | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | G | S | F | S | G | - | - | - | - | - | C | Y | W | S | W | I | R | Q | P | P | G | K | G | L |
| 21-225_74A3_HC | . | . | . | . | . | . | . | . | . | . | . | . | . | . | . | . | . | . | . | . | . | . | . | . | . | . |
| 21-225_74A4_HC | . | . | . | . | . | . | . | . | . | . | . | . | . | . | . | . | . | . | . | . | . | . | . | . | . | . |
| 21-225_74B12_HC | . | . | . | . | . | . | . | . | . | . | . | . | . | . | . | . | . | . | . | . | . | . | . | . | . | . |
| 21-225_74B2_HC | . | . | . | . | . | . | . | . | . | . | . | . | P | . | . | . | . | . | . | . | . | . | . | . | . | . |
| 21-225_74C5_HC | . | . | . | . | . | . | . | . | . | . | . | . | . | . | . | . | . | . | . | . | . | . | . | . | . | . |
| 21-225_74D11_HC | . | . | . | . | . | . | . | . | . | . | . | . | . | . | . | . | . | . | . | . | . | . | . | . | . | . |
| 21-225_74D5_HC | . | . | . | P | . | . | . | . | . | . | . | . | . | . | . | . | . | . | . | . | . | . | . | . | . | . |
| 21-225_74D6_HC | . | . | . | . | . | . | . | . | . | . | . | . | . | . | . | . | . | . | . | . | . | . | . | . | . | . |
| 21-225_74F5_HC | . | . | . | . | . | . | . | . | . | . | . | . | . | . | . | . | . | . | . | . | . | . | . | . | . | . |
| 21-225_74H5_HC | . | . | . | P | . | . | . | . | . | . | . | . | . | . | . | . | . | . | . | . | . | . | . | . | . | . |
| 21-225_74H7_HC | . | . | . | . | . | . | . | . | . | . | . | . | P | . | . | . | . | . | . | . | . | . | . | . | . | . |
| 21-225_75A12_HC | . | . | . | . | . | . | . | . | . | . | . | . | . | . | . | . | . | . | . | . | . | . | . | . | . | . |
| 21-225_75A5_HC | . | . | . | . | . | . | . | . | . | . | . | . | . | . | . | . | . | . | . | . | . | . | . | . | . | . |
| 21-225_75A8_HC | . | . | . | . | . | . | . | . | . | . | . | . | . | . | . | . | . | . | . | . | . | . | . | . | . | . |
| 21-225_75B10_HC | . | . | . | . | . | . | . | . | . | . | . | . | Y | . | . | . | . | . | . | . | . | . | . | . | . | . |
| 21-225_75C2_HC | . | . | . | . | . | . | . | . | . | . | . | . | . | . | . | . | . | . | . | . | . | . | . | . | . | . |
| 21-225_75C3_HC | . | . | . | . | . | . | . | . | . | . | . | . | . | . | . | . | . | . | . | . | . | . | . | . | . | . |
| 21-225_75D3_HC | . | . | . | . | . | . | . | . | . | . | . | . | . | . | . | . | . | . | . | . | . | . | . | . | . | . |
| 21-225_75D9_HC | . | . | . | . | . | . | . | . | . | . | . | . | P | . | . | . | . | . | . | . | . | . | . | . | . | . |
| 21-225_75F3_HC | . | . | . | . | . | . | . | . | . | . | . | . | . | . | . | . | . | . | . | . | . | . | . | . | . | . |
| 21-225_75G12_HC | . | . | . | . | . | . | . | . | . | . | . | . | . | . | . | . | . | . | . | . | . | . | . | . | . | . |
| 21-225_75G7_HC | . | . | . | A | . | . | . | . | . | . | . | . | S | . | . | . | . | . | . | . | S | . | . | . | . | . |
| 21-225_75H8_HC | . | . | . | . | . | . | . | . | . | . | . | . | P | . | . | . | . | . | . | . | . | . | . | . | . | . |
| 21-225_76A4_HC | . | . | . | . | . | . | . | . | . | . | . | . | . | . | . | . | . | . | . | . | . | . | . | . | . | . |
| 21-225_76D1_HC | S | . | . | . | . | . | . | . | . | . | . | . | . | . | . | . | . | . | . | . | . | . | . | . | . | . |
| 21-225_76E10_HC | . | . | . | P | . | . | . | . | . | . | . | . | S | . | . | . | . | . | . | . | . | . | . | . | . | . |
| 21-225_76G4_HC | . | . | . | . | . | . | . | . | . | . | . | . | . | . | . | . | . | . | . | . | . | . | . | . | . | . |
| 21-225_76H10_HC | . | . | . | P | . | . | . | . | . | . | . | . | . | . | . | . | . | . | . | . | . | . | . | . | . | . |
| 21-225_77A10_HC | . | . | . | . | . | . | . | . | . | . | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | G | S | F | S | G | - | - | - | - | - | C | Y | W | S | W | I | R | Q | P | P | G | K | G | L |
| 21-225_77C12_HC | . | . | . | A | . | . | . | . | . | . | . | . | S | . | . | . | . | . | . | . | S | . | . | . | . | . |
| 21-225_77C5_HC | . | . | . | . | . | . | . | . | . | . | . | . | . | . | . | . | . | . | . | . | . | . | . | . | . | . |
| 21-225_77F7_HC | . | . | . | . | . | . | . | . | . | . | . | . | . | . | . | . | . | . | . | . | . | . | . | . | . | . |
| 21-225_77G1_HC | . | . | . | . | . | . | . | . | . | . | . | . | . | . | . | . | . | . | . | . | . | . | . | . | . | . |
| 21-225_79F11_HC | . | . | . | . | . | . | . | . | . | . | . | . | . | . | . | . | . | . | . | . | . | . | . | . | . | . |
| 21-225_79F3_HC | . | . | . | . | . | . | . | . | . | . | . | . | . | . | . | . | . | . | . | . | . | . | . | . | . | . |
| 21-225_79G1_HC | . | . | . | . | . | . | . | . | . | . | . | . | . | . | . | . | . | . | . | . | . | . | . | . | . | . |
| 21-225_79G12_HC | . | . | . | . | . | . | . | . | . | . | . | . | . | . | . | . | . | . | . | . | . | . | . | . | . | . |
| 21-225_80A1_HC | . | . | . | . | . | . | . | . | . | . | . | . | . | . | . | . | . | . | . | . | . | . | . | . | . | . |
| 21-225_80C1_HC | . | . | . | . | . | . | . | . | . | . | . | . | . | . | . | . | . | . | . | . | . | . | . | . | . | . |
| 21-225_80D5_HC | . | . | . | . | . | . | . | . | . | . | . | . | P | . | . | . | . | . | . | . | . | . | . | . | . | . |
| 21-225_80E12_HC | . | . | . | P | . | . | . | . | . | . | . | . | . | . | . | . | . | . | . | . | . | . | . | . | . | . |
| 21-225_80E3_HC | . | . | . | . | . | . | . | . | . | . | . | . | . | . | . | . | . | . | . | . | . | . | . | . | . | . |
| 21-225_80H12_HC | . | . | . | . | . | . | . | . | . | . | . | . | . | . | . | . | . | . | . | . | . | . | . | . | . | . |
| 21-225_80H7_HC | V | . | . | . | . | . | . | . | . | . | . | . | S | . | . | . | . | . | . | . | . | . | . | . | . | . |
| 21-225_81C11_HC | . | . | . | . | . | . | . | . | . | . | . | . | . | . | . | . | . | . | . | . | . | . | . | . | . | . |
| 21-225_81C5_HC | . | . | . | . | . | . | . | . | . | . | . | . | . | . | . | . | . | . | . | . | . | . | . | . | . | . |
| 21-225_83B7_HC | . | . | . | . | . | . | . | . | . | . | . | . | . | . | . | . | . | . | . | . | . | . | . | . | . | . |
| 21-225_83G1_HC | . | . | . | . | . | . | . | . | . | . | . | . | . | . | . | . | . | . | . | . | . | . | . | . | . | . |
| 21-225_84E12_HC | . | . | . | . | . | . | . | . | . | . | . | . | S | . | . | . | . | . | . | . | . | . | . | . | . | . |
| 21-225_85A3_HC | . | . | . | . | . | . | . | . | . | . | . | . | . | . | . | . | . | . | . | . | . | . | . | . | . | . |
| 21-225_85B4_HC | . | . | . | . | . | . | . | . | . | . | . | . | . | . | . | . | . | . | . | . | . | . | . | . | . | . |
| 21-225_85B9_HC | . | . | . | P | . | . | . | . | . | . | . | . | . | . | . | . | . | . | . | . | . | . | . | . | . | . |
| 21-225_85D6_HC | . | . | . | . | . | . | . | . | . | . | . | . | . | . | . | . | . | . | . | . | . | . | . | . | . | . |
| 21-225_85G10_HC | . | . | . | . | . | . | . | . | . | . | . | . | . | . | . | . | . | . | . | . | . | . | . | . | . | . |
| 21-225_86C1_HC | . | . | . | . | . | . | . | . | . | . | . | . | . | . | . | . | . | . | . | . | . | . | . | . | . | . |
| 21-225_86C11_HC | . | . | . | . | . | . | . | . | . | . | . | . | . | . | . | . | . | . | . | . | . | . | . | . | . | . |
| 21-225_86E4_HC | . | . | . | . | . | . | . | . | . | . | . | . | . | . | . | . | . | . | . | . | . | . | . | . | . | . |
| 21-225_87A12_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | G | S | F | S | G | - | - | - | - | - | C | Y | W | S | W | I | R | Q | P | P | G | K | G | L |
| 21-225_87C9_HC | . | . | . | . | . | . | . | . | . | . | . | . | . | . | . | . | . | . | . | . | . | . | . | . | . | . |
| 21-225_87E5_HC | . | . | . | . | . | . | . | . | . | . | . | . | . | . | . | . | . | . | . | . | . | . | . | . | . | . |
| 21-225_87H1_HC | . | . | . | . | . | . | . | . | . | . | . | . | Y | . | . | . | . | . | . | . | . | . | . | . | . | . |
| 21-225_88E7_HC | . | . | . | . | . | . | . | . | . | . | . | . | . | . | . | . | . | . | . | . | . | . | . | . | . | . |
| 21-225_88F7_HC | . | . | . | . | . | . | . | . | . | . | . | . | . | . | . | . | . | . | . | . | . | . | . | . | . | . |
| 21-225_88G9_HC | . | . | . | . | . | . | . | . | . | . | . | . | . | . | . | . | . | . | . | . | . | . | . | . | . | . |
| 21-225_88H1_HC | . | . | . | . | . | . | . | . | . | . | . | . | . | . | . | . | . | . | . | . | . | . | . | . | . | . |
| 21-225_89A11_HC | . | . | . | . | . | . | . | . | . | . | . | . | Y | . | . | . | . | . | . | . | . | . | . | . | . | . |
| 21-225_89D5_HC | . | . | . | . | . | . | . | . | . | . | . | . | . | . | . | . | . | . | . | . | . | . | . | . | . | . |
| 21-225_89E10_HC | . | . | . | . | . | . | . | . | . | . | . | . | . | . | . | . | . | . | . | . | . | . | . | . | . | . |
| 21-225_89H5_HC | . | . | . | . | . | . | . | . | . | . | . | . | . | . | . | . | . | . | . | . | . | . | . | . | . | . |
| 21-225_90A5_HC | . | . | . | . | . | . | . | . | . | . | . | . | . | . | . | . | . | . | . | . | . | . | . | . | . | . |
| 21-225_90F10_HC | . | . | . | . | . | . | . | . | . | . | . | . | . | . | . | . | . | . | . | . | . | . | . | . | . | . |
| 21-225_90G4_HC | . | . | . | . | . | . | . | . | . | . | . | . | . | . | . | . | . | . | . | . | . | . | . | . | . | . |
| 21-225_90G5_HC | . | . | . | . | . | . | . | . | . | . | . | . | . | . | . | . | . | . | . | . | . | . | . | . | . | . |
| 21-225_91B2_HC | . | . | . | . | . | . | . | . | . | . | . | . | . | . | . | . | . | . | . | . | . | . | . | . | . | . |
| 21-225_91B3_HC | . | . | . | . | . | . | . | . | . | . | . | . | . | . | . | . | . | . | . | . | . | . | . | . | . | . |
| 21-225_91B4_HC | . | . | . | . | . | . | . | . | . | . | . | . | . | . | . | . | . | . | . | . | . | . | . | . | . | . |
| 21-225_91E9_HC | . | . | . | . | . | . | . | . | . | . | . | . | . | . | . | . | . | . | . | . | . | . | . | . | . | . |
| 21-225_91F3_HC | . | . | . | . | . | . | . | . | . | . | . | . | . | . | . | . | . | . | . | . | . | . | . | . | . | . |
| 21-225_92B1_HC | . | . | . | . | . | . | . | . | . | . | . | . | S | . | . | . | . | . | . | . | . | . | . | . | . | . |
| 21-225_92D6_HC | . | . | . | . | . | . | . | . | . | . | . | . | . | . | . | . | . | . | . | . | . | . | . | . | . | . |
| 21-225_93C2_HC | . | . | . | . | . | . | . | . | . | . | . | . | . | . | . | . | . | . | . | . | . | . | . | . | . | . |
| 21-225_93E4_HC | . | . | . | . | . | . | . | . | . | . | . | . | . | . | . | . | . | . | . | . | . | . | . | . | . | . |
| 21-225_94D3_HC | . | . | . | . | . | . | . | . | . | . | . | . | . | . | . | . | . | . | . | . | . | . | . | . | . | . |
| 21-225_94F12_HC | . | . | . | . | . | . | . | . | . | . | . | . | . | . | . | . | . | . | . | . | . | . | . | . | . | . |
| 21-225_94G10_HC | . | . | . | P | . | . | . | . | . | . | . | . | . | . | . | . | G | . | . | . | . | . | . | . | . | R |
| 21-225_95D2_HC | . | . | . | . | . | . | . | . | . | . | . | . | . | . | . | . | . | . | . | . | . | . | . | . | . | . |
| 21-225_95F9_HC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | G | S | F | S | G | - | - | - | - | - | C | Y | W | S | W | I | R | Q | P | P | G | K | G | L |
| 21-225_95G9_HC | . | . | . | . | . | . | . | . | . | . | . | . | . | . | . | . | . | . | . | . | . | . | . | . | . | . |
| 21-225_95H10_HC | . | . | . | . | . | . | . | . | . | . | . | . | . | . | . | . | . | . | . | . | . | . | . | . | . | . |
| 21-225_97A2_HC | . | . | . | . | . | . | . | . | . | . | . | . | . | . | . | . | . | . | . | . | . | . | . | . | . | . |
| 21-225_97E5_HC | . | . | . | . | . | . | . | . | . | . | . | . | . | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | I | G | E | I | N | H | - | - | - | - | S | G | S | T | N | Y | N | P | S | L | K | S | R | V |
| 21-225_74A3_HC | . | . | . | . | . | . | . | . | . | . | . | . | . | . | . | . | . | . | . | . | . | . | . | . | . | . |
| 21-225_74A4_HC | . | . | . | . | . | . | . | . | . | . | . | . | . | . | . | . | . | . | . | . | . | . | . | . | . | . |
| 21-225_74B12_HC | . | . | . | . | . | . | . | . | . | . | . | . | . | . | . | . | . | . | . | . | . | . | . | . | . | . |
| 21-225_74B2_HC | . | . | . | . | . | . | . | . | . | . | . | . | . | . | . | . | . | . | . | . | . | . | T | . | . | . |
| 21-225_74C5_HC | . | . | . | . | . | . | . | . | . | . | . | . | . | . | G | . | . | F | . | . | . | . | . | . | . | . |
| 21-225_74D11_HC | . | . | . | . | . | . | . | . | . | . | . | . | . | . | . | . | . | . | . | . | . | . | . | . | . | . |
| 21-225_74D5_HC | . | . | . | . | . | . | . | Y | . | . | . | . | . | . | R | . | . | F | . | . | . | . | . | . | . | . |
| 21-225_74D6_HC | . | . | . | . | . | . | . | . | . | . | . | . | . | . | . | . | . | . | . | . | . | . | . | . | . | . |
| 21-225_74F5_HC | . | . | . | . | . | . | . | . | . | . | . | . | . | . | . | . | . | . | . | . | . | . | . | . | . | . |
| 21-225_74H5_HC | . | . | . | . | . | . | . | . | . | . | . | . | . | . | R | . | . | F | . | . | . | . | . | . | . | . |
| 21-225_74H7_HC | . | . | . | . | . | . | . | . | . | . | . | . | . | . | . | . | . | . | . | . | . | . | . | . | . | . |
| 21-225_75A12_HC | . | . | . | . | . | . | . | Y | . | . | . | . | . | . | R | . | . | . | . | . | . | . | . | . | . | . |
| 21-225_75A5_HC | . | . | . | . | . | . | . | . | . | . | . | . | . | . | . | . | . | . | . | . | . | . | . | . | . | . |
| 21-225_75A8_HC | . | . | . | . | . | . | . | . | . | . | . | . | . | . | . | . | . | . | . | . | . | . | . | . | . | . |
| 21-225_75B10_HC | . | . | . | . | . | . | . | . | . | . | . | . | . | . | . | . | . | . | . | . | . | . | . | . | . | . |
| 21-225_75C2_HC | . | . | . | . | . | . | . | . | . | . | . | . | . | . | . | . | . | . | . | . | . | . | . | . | . | . |
| 21-225_75C3_HC | . | . | . | . | . | . | . | Y | . | . | . | . | . | . | R | . | . | . | . | . | . | . | . | . | . | . |
| 21-225_75D3_HC | . | . | . | . | . | . | . | . | . | . | . | . | . | . | . | . | . | . | . | . | . | . | . | . | . | . |
| 21-225_75D9_HC | . | . | . | . | . | . | . | Y | . | . | . | . | R | . | . | . | . | . | . | . | . | . | . | . | . | . |
| 21-225_75F3_HC | . | . | . | . | . | . | . | . | . | . | . | . | . | . | . | . | . | . | . | . | . | . | . | . | . | . |
| 21-225_75G12_HC | . | . | . | . | . | . | . | . | . | . | . | . | . | . | . | . | . | . | . | . | . | . | . | . | . | . |
| 21-225_75G7_HC | . | . | . | . | . | . | . | Y | . | . | . | . | R | . | . | . | . | . | . | . | . | . | . | . | . | . |
| 21-225_75H8_HC | . | . | . | . | . | . | . | . | . | . | . | . | . | . | . | . | . | . | . | . | . | . | . | . | . | . |
| 21-225_76A4_HC | . | . | . | . | . | . | . | . | . | . | . | . | . | . | . | . | . | . | . | . | . | . | . | . | . | . |
| 21-225_76D1_HC | . | . | . | . | . | . | . | . | . | . | . | . | . | . | R | . | . | . | . | . | . | . | . | . | . | . |
| 21-225_76E10_HC | . | . | . | . | . | . | . | Y | . | . | . | . | . | . | R | . | . | F | . | . | . | . | . | . | . | . |
| 21-225_76G4_HC | . | . | . | . | . | . | . | . | . | . | . | . | . | . | . | . | . | . | . | . | . | . | . | . | . | . |
| 21-225_76H10_HC | . | . | . | . | . | . | . | Y | . | . | . | . | . | . | R | . | . | F | . | . | . | . | . | . | . | . |
| 21-225_77A10_HC | . | . | . | . | . | . | . | Y | . | . | . | . | . | . | R | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | I | G | E | I | N | H | - | - | - | - | S | G | S | T | N | Y | N | P | S | L | K | S | R | V |
| 21-225_77C12_HC | . | . | . | . | . | . | . | Y | . | . | . | . | R | . | . | . | . | . | . | . | . | . | . | . | . | . |
| 21-225_77C5_HC | . | . | . | . | . | . | . | . | . | . | . | . | . | . | . | . | . | . | . | . | . | . | . | . | . | . |
| 21-225_77F7_HC | . | . | . | . | . | . | . | . | . | . | . | . | . | . | . | . | . | . | . | . | . | . | . | . | . | . |
| 21-225_77G1_HC | . | . | . | . | . | . | . | . | . | . | . | . | . | . | . | . | . | . | . | . | . | . | . | . | . | . |
| 21-225_79F11_HC | . | . | . | . | . | . | . | . | . | . | . | . | . | . | . | . | . | . | . | . | . | . | . | . | . | . |
| 21-225_79F3_HC | . | . | . | . | . | . | . | . | . | . | . | . | . | . | . | . | . | . | . | . | . | . | . | . | . | . |
| 21-225_79G1_HC | . | . | . | . | . | . | . | . | . | . | . | . | . | . | . | . | . | . | . | . | . | . | . | . | . | . |
| 21-225_79G12_HC | . | . | . | . | . | . | . | . | . | . | . | . | . | . | . | . | . | . | . | . | . | . | . | . | . | . |
| 21-225_80A1_HC | . | . | . | . | . | . | . | . | . | . | . | . | . | . | . | . | . | . | . | . | . | . | . | . | . | . |
| 21-225_80C1_HC | . | . | . | . | . | . | . | . | . | . | . | . | . | . | . | . | . | . | . | . | . | . | . | . | . | . |
| 21-225_80D5_HC | . | . | . | . | . | . | . | . | . | . | . | . | . | . | . | . | . | . | . | . | . | . | . | . | . | . |
| 21-225_80E12_HC | . | . | . | . | . | . | . | Y | . | . | . | . | . | . | R | . | . | F | . | . | . | . | . | . | . | . |
| 21-225_80E3_HC | . | . | . | . | . | . | . | . | . | . | . | . | . | . | . | . | . | . | . | . | . | . | . | . | . | . |
| 21-225_80H12_HC | . | . | . | . | . | . | . | . | . | . | . | . | . | . | . | . | . | . | . | . | . | . | . | . | . | . |
| 21-225_80H7_HC | . | . | . | . | . | . | . | Q | . | . | . | . | . | . | R | . | . | . | . | . | . | . | . | . | . | . |
| 21-225_81C11_HC | . | . | . | . | . | . | . | . | . | . | . | . | . | . | . | . | . | . | . | . | . | . | . | . | . | . |
| 21-225_81C5_HC | . | . | . | . | . | . | . | . | . | . | . | . | . | . | . | . | . | . | . | . | . | . | . | . | . | . |
| 21-225_83B7_HC | . | . | . | . | . | . | . | . | . | . | . | . | . | . | . | . | . | . | . | . | . | . | . | . | . | . |
| 21-225_83G1_HC | . | . | . | . | . | . | . | . | . | . | . | . | . | . | . | . | . | . | . | . | . | . | . | . | . | . |
| 21-225_84E12_HC | . | . | . | . | . | . | . | Q | . | . | . | . | . | . | R | . | . | . | . | . | . | . | . | . | . | . |
| 21-225_85A3_HC | . | . | . | . | . | . | . | . | . | . | . | . | . | . | . | . | . | . | . | . | . | . | . | . | . | . |
| 21-225_85B4_HC | . | . | . | . | . | . | . | . | . | . | . | . | . | . | . | . | . | . | . | . | . | . | . | . | . | . |
| 21-225_85B9_HC | . | . | . | . | . | . | . | . | . | . | . | . | . | . | R | . | . | F | . | . | . | . | . | . | . | . |
| 21-225_85D6_HC | . | . | . | . | . | . | . | Y | . | . | . | . | . | . | R | . | . | . | . | . | . | . | . | . | . | . |
| 21-225_85G10_HC | . | . | . | . | . | . | . | . | . | . | . | . | . | . | I | . | . | . | . | . | . | . | . | . | . | . |
| 21-225_86C1_HC | . | . | . | . | . | . | . | . | . | . | . | . | . | . | . | . | . | . | . | . | . | . | . | . | . | . |
| 21-225_86C11_HC | . | . | . | . | . | . | . | . | . | . | . | . | . | . | . | . | . | . | . | . | . | . | . | . | . | . |
| 21-225_86E4_HC | . | . | . | . | . | . | . | . | . | . | . | . | . | . | . | . | . | . | . | . | . | . | . | . | . | . |
| 21-225_87A12_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | I | G | E | I | N | H | - | - | - | - | S | G | S | T | N | Y | N | P | S | L | K | S | R | V |
| 21-225_87C9_HC | . | . | . | . | . | . | . | . | . | . | . | . | . | . | . | . | . | . | . | . | . | . | . | . | . | . |
| 21-225_87E5_HC | . | . | . | . | . | . | . | . | . | . | . | . | . | . | . | . | . | . | . | . | . | . | . | . | . | . |
| 21-225_87H1_HC | . | . | . | . | . | . | . | . | . | . | . | . | . | . | [illegible] | . | . | . | . | . | . | . | . | . | . | . |
| 21-225_88E7_HC | . | . | . | . | . | . | . | . | . | . | . | . | . | . | . | . | . | . | . | . | . | . | . | . | . | . |
| 21-225_88F7_HC | . | . | . | . | . | . | . | . | . | . | . | . | . | . | . | . | . | . | . | . | . | . | . | . | . | . |
| 21-225_88G9_HC | . | . | . | . | . | . | . | . | . | . | . | . | . | . | . | . | . | . | . | . | . | . | . | . | . | . |
| 21-225_88H1_HC | . | . | . | . | . | . | . | . | . | . | . | . | . | . | . | . | . | . | . | . | . | . | . | . | . | . |
| 21-225_89A11_HC | . | . | . | . | . | . | . | . | . | . | . | . | . | . | [illegible] | . | S | . | . | . | . | . | . | . | . | . |
| 21-225_89D5_HC | . | . | . | . | . | . | . | . | . | . | . | . | . | . | . | . | . | . | . | . | . | . | . | . | . | . |
| 21-225_89E10_HC | . | . | . | . | . | . | . | . | . | . | . | . | . | . | . | . | . | . | . | . | . | . | . | . | . | . |
| 21-225_89H5_HC | . | . | . | . | . | . | . | Y | . | . | . | . | . | . | . | . | . | F | . | . | . | . | . | . | . | . |
| 21-225_90A5_HC | . | . | . | . | . | . | . | . | . | . | . | . | . | . | . | . | . | . | . | . | . | . | . | . | . | . |
| 21-225_90F10_HC | . | . | . | . | . | . | . | . | . | . | . | . | . | . | . | . | . | . | . | . | . | . | . | . | . | . |
| 21-225_90G4_HC | . | . | . | . | . | . | . | . | . | . | . | . | . | . | . | . | . | . | . | . | . | . | . | . | . | . |
| 21-225_90G5_HC | . | . | . | . | . | . | . | . | . | . | . | . | . | . | . | . | . | . | . | . | . | . | . | . | . | . |
| 21-225_91B2_HC | . | . | . | . | . | . | . | . | . | . | . | . | . | . | . | . | . | . | . | . | . | . | . | . | . | . |
| 21-225_91B3_HC | . | . | . | . | . | . | . | . | . | . | . | . | . | . | . | . | . | . | . | . | . | . | . | . | . | . |
| 21-225_91B4_HC | . | . | . | . | . | . | . | . | . | . | . | . | . | . | . | . | . | . | . | . | . | . | . | . | . | . |
| 21-225_91E9_HC | . | . | . | . | . | . | . | . | . | . | . | . | . | . | . | . | . | . | . | . | . | . | . | . | . | . |
| 21-225_91F3_HC | . | . | . | . | . | . | . | . | . | . | . | . | . | . | . | . | . | . | . | . | . | . | . | . | . | . |
| 21-225_92B1_HC | . | . | . | . | . | . | . | Y | . | . | . | . | [illegible] | . | . | . | . | . | . | . | . | . | . | . | . | . |
| 21-225_92D6_HC | . | . | . | . | . | . | . | . | . | . | . | . | . | . | . | . | . | . | . | . | . | . | . | . | . | . |
| 21-225_93C2_HC | . | . | . | . | . | . | . | . | . | . | . | . | . | . | . | . | . | . | . | . | . | . | . | . | . | . |
| 21-225_93E4_HC | . | . | . | . | . | . | . | . | . | . | . | . | . | . | . | . | . | . | . | . | . | . | . | . | . | . |
| 21-225_94D3_HC | . | . | . | . | . | . | . | Y | . | . | . | . | . | . | [illegible] | . | . | . | . | . | . | . | . | . | . | . |
| 21-225_94F12_HC | . | . | . | . | . | . | . | . | . | . | . | . | . | . | . | . | . | . | . | . | . | . | . | . | . | . |
| 21-225_94G10_HC | . | . | . | . | . | . | . | Y | . | . | . | . | . | . | [illegible] | . | . | F | . | . | . | . | . | . | . | . |
| 21-225_95D2_HC | . | . | . | . | . | . | . | . | . | . | . | . | . | . | . | . | . | . | . | . | . | . | . | . | . | . |
| 21-225_95F9_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | I | G | E | I | N | H | - | - | - | - | S | G | S | T | N | Y | N | P | S | L | K | S | R | V |
| 21-225_95G9_HC | . | . | . | . | . | . | . | . | . | . | . | . | . | . | . | . | . | . | . | . | . | . | . | . | . | . |
| 21-225_95H10_HC | . | . | . | . | . | . | . | . | . | . | . | . | . | . | . | . | . | . | . | . | . | . | . | . | . | . |
| 21-225_97A2_HC | . | . | . | . | . | . | . | . | . | . | . | . | . | . | . | . | . | . | . | . | . | . | . | . | . | . |
| 21-225_97E5_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | V | D | T | S | K | N | Q | F | S | L | K | L | S | S | V | T | A | A | D | T | A | V | Y |
| 21-225_74A3_HC | . | . | . | . | . | . | . | . | . | . | . | . | . | . | . | . | . | . | . | . | . | . | . | . | . | . |
| 21-225_74A4_HC | . | . | . | . | . | . | . | . | . | . | . | . | . | . | . | . | . | . | . | . | . | . | . | . | . | . |
| 21-225_74B12_HC | . | . | . | . | . | . | . | . | . | . | . | . | . | . | . | . | . | . | . | . | . | . | . | . | . | . |
| 21-225_74B2_HC | . | . | . | . | . | . | . | . | . | . | . | . | . | . | . | T | . | . | . | . | . | . | S | . | . | . |
| 21-225_74C5_HC | . | . | . | . | . | . | . | . | . | . | . | . | . | . | . | . | . | . | . | . | . | . | . | . | . | . |
| 21-225_74D11_HC | . | . | . | . | . | . | . | . | . | . | . | . | . | . | . | . | . | . | . | . | . | . | . | . | . | . |
| 21-225_74D5_HC | . | . | . | . | . | . | . | . | . | . | . | . | . | . | . | . | . | . | . | . | . | . | . | . | . | . |
| 21-225_74D6_HC | . | . | . | . | . | . | . | . | . | . | . | . | . | . | . | . | . | . | . | . | . | . | . | . | . | . |
| 21-225_74F5_HC | . | . | . | . | . | . | . | . | . | . | . | . | . | . | . | . | . | . | . | . | . | . | . | . | . | . |
| 21-225_74H5_HC | . | . | . | . | . | . | . | . | . | . | . | . | . | . | . | . | . | . | . | . | . | . | . | . | . | . |
| 21-225_74H7_HC | . | . | . | . | . | . | . | . | . | . | . | . | . | . | . | T | . | . | . | . | . | . | . | . | . | . |
| 21-225_75A12_HC | . | . | . | . | . | . | . | . | . | . | . | . | . | . | . | . | . | . | . | . | . | . | . | . | . | . |
| 21-225_75A5_HC | . | . | . | . | . | . | . | . | . | . | . | . | . | . | . | . | . | . | . | . | . | . | . | . | . | . |
| 21-225_75A8_HC | . | . | . | . | . | . | . | . | . | . | . | . | . | . | . | . | . | . | . | . | . | . | . | . | . | . |
| 21-225_75B10_HC | . | . | . | . | . | . | . | . | . | . | . | . | . | . | . | . | . | . | . | . | . | . | . | . | . | . |
| 21-225_75C2_HC | . | . | . | . | . | . | . | . | . | . | . | . | . | . | . | . | . | . | . | . | . | . | . | . | . | . |
| 21-225_75C3_HC | . | . | . | . | . | . | . | . | . | . | . | . | . | . | . | . | . | . | . | . | . | . | . | . | . | . |
| 21-225_75D3_HC | . | . | . | . | . | . | . | . | . | . | . | . | . | . | . | . | . | . | . | . | . | . | . | . | . | . |
| 21-225_75D9_HC | . | . | . | . | . | . | . | . | . | . | . | . | . | N | . | . | . | . | . | . | . | . | . | . | . | . |
| 21-225_75F3_HC | . | . | . | . | . | . | . | . | . | . | . | . | . | . | . | . | . | . | . | . | . | . | . | . | . | . |
| 21-225_75G12_HC | . | . | . | . | . | . | . | . | . | . | . | . | . | . | . | . | . | . | . | . | . | . | . | . | . | . |
| 21-225_75G7_HC | . | . | . | . | . | . | . | . | . | . | . | . | . | N | . | . | . | . | . | . | . | . | . | . | . | . |
| 21-225_75H8_HC | . | . | . | . | . | . | . | . | . | . | . | . | . | . | . | T | . | . | . | . | . | . | . | . | . | . |
| 21-225_76A4_HC | . | . | . | . | . | . | . | . | . | . | . | . | . | . | . | . | . | . | . | . | . | . | . | . | . | . |
| 21-225_76D1_HC | . | . | . | . | . | . | . | . | . | . | . | . | . | . | . | . | . | . | . | . | . | . | . | . | . | . |
| 21-225_76E10_HC | . | . | . | . | . | . | . | . | . | . | . | . | . | . | . | . | . | . | . | . | . | . | . | . | . | . |
| 21-225_76G4_HC | . | . | . | . | . | . | . | . | . | . | . | . | . | . | . | . | . | . | . | . | . | . | . | . | . | . |
| 21-225_76H10_HC | . | . | . | . | . | . | . | . | . | . | . | . | . | . | . | . | . | . | . | . | . | . | . | . | . | . |
| 21-225_77A10_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | V | D | T | S | K | N | Q | F | S | L | K | L | S | S | V | T | A | A | D | T | A | V | Y |
| 21-225_77C12_HC | . | . | . | . | . | . | . | . | . | . | . | . | . | N | . | . | . | . | . | . | . | . | . | . | . | . |
| 21-225_77C5_HC | . | . | . | . | . | . | . | . | . | . | . | . | . | . | . | . | . | . | . | . | . | . | . | . | . | . |
| 21-225_77F7_HC | . | . | . | . | . | . | . | . | . | . | . | . | . | . | . | . | . | . | . | . | . | . | . | . | . | . |
| 21-225_77G1_HC | . | . | . | . | . | . | . | . | . | . | . | . | . | . | . | . | . | . | . | . | . | . | . | . | . | . |
| 21-225_79F11_HC | . | . | . | . | . | . | . | . | . | . | . | . | . | . | . | . | . | . | . | . | . | . | . | . | . | . |
| 21-225_79F3_HC | . | . | . | . | . | . | . | . | . | . | . | . | . | . | . | . | . | . | . | . | . | . | . | . | . | . |
| 21-225_79G1_HC | . | . | . | . | . | . | . | . | . | . | . | . | . | . | . | . | . | . | . | . | . | . | . | . | . | . |
| 21-225_79G12_HC | . | . | . | . | . | . | . | . | . | . | . | . | . | . | . | . | . | . | . | . | . | . | . | . | . | . |
| 21-225_80A1_HC | . | . | . | . | . | . | . | . | . | . | . | . | . | . | . | . | . | . | . | . | . | . | . | . | . | . |
| 21-225_80C1_HC | . | . | . | . | . | . | . | . | . | . | . | . | . | . | . | . | . | . | . | . | . | . | . | . | . | . |
| 21-225_80D5_HC | . | . | . | . | . | . | . | . | . | . | . | . | . | . | . | T | . | . | . | . | . | . | M | . | . | . |
| 21-225_80E12_HC | . | . | . | . | . | . | . | . | . | . | . | . | . | . | . | . | . | . | . | . | . | . | . | . | . | . |
| 21-225_80E3_HC | . | . | . | . | . | . | . | . | . | . | . | . | . | . | . | . | . | . | . | . | . | . | . | . | . | . |
| 21-225_80H12_HC | . | . | . | . | . | . | . | . | . | . | . | . | . | . | . | . | . | . | . | . | . | . | . | . | . | . |
| 21-225_80H7_HC | . | . | . | . | . | . | . | . | . | . | . | . | . | . | . | . | . | . | . | . | . | . | . | . | . | . |
| 21-225_81C11_HC | . | . | . | . | . | . | . | . | . | . | . | . | . | . | . | . | . | . | . | . | . | . | . | . | . | . |
| 21-225_81C5_HC | . | . | . | . | . | . | . | . | . | . | . | . | . | . | . | . | . | . | . | . | . | . | . | . | . | . |
| 21-225_83B7_HC | . | . | . | . | . | . | . | . | . | . | . | . | . | . | . | . | . | . | . | . | . | . | . | . | . | . |
| 21-225_83G1_HC | . | . | . | . | . | . | . | . | . | . | . | . | . | . | . | . | . | . | . | . | . | . | . | . | . | . |
| 21-225_84E12_HC | . | . | . | . | . | . | . | . | . | . | . | . | . | . | . | . | . | . | . | . | . | . | . | . | . | . |
| 21-225_85A3_HC | . | . | . | . | . | . | . | . | . | . | . | . | . | . | . | . | . | . | . | . | . | . | . | . | . | . |
| 21-225_85B4_HC | . | . | . | . | . | . | . | . | . | . | . | . | . | . | . | . | . | . | . | . | . | . | . | . | . | . |
| 21-225_85B9_HC | . | . | . | . | . | . | . | . | . | . | . | . | . | . | . | . | . | . | . | . | . | . | . | . | . | . |
| 21-225_85D6_HC | . | . | . | . | . | . | . | . | . | . | . | . | . | . | . | . | . | . | . | . | . | . | . | . | . | . |
| 21-225_85G10_HC | . | . | . | . | . | . | . | . | . | . | . | . | . | . | . | T | . | . | . | . | . | . | . | . | . | . |
| 21-225_86C1_HC | . | . | . | . | . | . | . | . | . | . | . | . | . | . | . | . | . | . | . | . | . | . | . | . | . | . |
| 21-225_86C11_HC | . | . | . | . | . | . | . | . | . | . | . | . | . | . | . | . | . | . | . | . | . | . | . | . | . | . |
| 21-225_86E4_HC | . | . | . | . | . | . | . | . | . | . | . | . | . | . | . | . | . | . | . | . | . | . | . | . | . | . |
| 21-225_87A12_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | V | D | T | S | K | N | Q | F | S | L | K | L | S | S | V | T | A | A | D | T | A | V | Y |
| 21-225_87C9_HC | . | . | . | . | . | . | . | . | . | . | . | . | . | . | . | . | . | . | . | . | . | . | . | . | . | . |
| 21-225_87E5_HC | . | . | . | . | . | . | . | . | . | . | . | . | . | . | . | . | . | . | . | . | . | . | . | . | . | . |
| 21-225_87H1_HC | . | . | . | . | . | . | . | . | D | . | . | . | . | . | . | . | . | . | . | . | . | . | . | . | . | . |
| 21-225_88E7_HC | . | . | . | . | . | . | . | . | . | . | . | . | . | . | . | . | . | . | . | . | . | . | . | . | . | . |
| 21-225_88F7_HC | . | . | . | . | . | . | . | . | . | . | . | . | . | . | . | . | . | . | . | . | . | . | . | . | . | . |
| 21-225_88G9_HC | . | . | . | . | . | . | . | . | . | . | . | . | . | . | . | . | . | . | . | . | . | . | . | . | . | . |
| 21-225_88H1_HC | . | . | . | . | . | . | . | . | . | . | . | . | . | . | . | . | . | . | . | . | . | . | . | . | . | . |
| 21-225_89A11_HC | . | . | . | . | . | . | . | . | . | . | . | . | . | . | . | . | . | . | . | . | . | . | . | . | . | . |
| 21-225_89D5_HC | . | . | . | . | . | . | . | . | . | . | . | . | . | . | . | . | . | . | . | . | . | . | . | . | . | . |
| 21-225_89E10_HC | . | . | . | . | . | . | . | . | . | . | . | . | . | . | . | . | . | . | . | . | . | . | . | . | . | . |
| 21-225_89H5_HC | . | . | . | A | . | . | . | . | . | . | . | . | . | . | . | . | . | . | . | . | . | . | . | . | . | . |
| 21-225_90A5_HC | . | . | . | . | . | . | . | . | . | . | . | . | . | . | . | . | . | . | . | . | . | . | . | . | . | . |
| 21-225_90F10_HC | . | . | . | . | . | . | . | . | . | . | . | . | . | . | . | . | . | . | . | . | . | . | . | . | . | . |
| 21-225_90G4_HC | . | . | . | . | . | . | . | . | . | . | . | . | . | . | . | . | . | . | . | . | . | . | . | . | . | . |
| 21-225_90G5_HC | . | . | . | . | . | . | . | . | . | . | . | . | . | . | . | . | . | . | . | . | . | . | . | . | . | . |
| 21-225_91B2_HC | . | . | . | . | . | . | . | . | . | . | . | . | . | . | . | . | . | . | . | . | . | . | . | . | . | . |
| 21-225_91B3_HC | . | . | . | . | . | . | . | . | . | . | . | . | . | . | . | . | . | . | . | . | . | . | . | . | . | . |
| 21-225_91B4_HC | . | . | . | . | . | . | . | . | . | . | . | . | . | . | . | . | . | . | . | . | . | . | . | . | . | . |
| 21-225_91E9_HC | . | . | . | . | . | . | . | . | . | . | . | . | . | . | . | . | . | . | . | . | . | . | . | . | . | . |
| 21-225_91F3_HC | . | . | . | . | . | . | . | . | . | . | . | . | . | . | . | . | . | . | . | . | . | . | . | . | . | . |
| 21-225_92B1_HC | A | . | . | . | . | . | . | . | . | . | . | . | . | N | . | T | . | . | . | . | . | . | . | . | . | . |
| 21-225_92D6_HC | . | . | . | . | . | . | . | . | . | . | . | . | . | . | . | . | . | . | . | . | . | . | . | . | . | . |
| 21-225_93C2_HC | . | . | . | . | . | . | . | . | . | . | . | . | . | . | . | . | . | . | . | . | . | . | . | . | . | . |
| 21-225_93E4_HC | . | . | . | . | . | . | . | . | . | . | . | . | . | . | . | . | . | . | . | . | . | . | . | . | . | . |
| 21-225_94D3_HC | . | . | . | . | . | . | . | . | . | . | . | . | . | . | . | . | . | . | . | . | . | . | . | . | . | . |
| 21-225_94F12_HC | . | . | . | . | . | . | . | . | . | . | . | . | . | . | . | . | . | . | . | . | . | . | . | . | . | . |
| 21-225_94G10_HC | . | . | . | . | . | . | . | . | . | . | . | . | . | . | . | . | . | . | . | . | . | . | . | . | . | . |
| 21-225_95D2_HC | . | . | . | . | . | . | . | . | . | . | . | . | . | . | . | . | . | . | . | . | . | . | . | . | . | . |
| 21-225_95F9_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | V | D | T | S | K | N | Q | F | S | L | K | L | S | S | V | T | A | A | D | T | A | V | Y |
| 21-225_95G9_HC | . | . | . | . | . | . | . | . | . | . | . | . | . | . | . | . | . | . | . | . | . | . | . | . | . | . |
| 21-225_95H10_HC | . | . | . | . | . | . | . | . | . | . | . | . | . | . | . | . | . | . | . | . | . | . | . | . | . | . |
| 21-225_97A2_HC | . | . | . | . | . | . | . | . | . | . | . | . | . | . | . | . | . | . | . | . | . | . | . | . | . | . |
| 21-225_97E5_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | D | Y | G | G | - | - | - | - | - | - | - | - | - | - | - | - | - | - | - | - | - | - |
| 21-225_77C12_HC | . | . | . | . | . | . | . | . | . | . | . | . | . | . | . | . | . | . | . | . | . | . | . | . | . | . |
| 21-225_77C5_HC | . | . | . | . | . | . | . | . | . | . | . | . | . | . | . | . | . | . | . | . | . | . | . | . | . | . |
| 21-225_77F7_HC | . | . | . | . | . | . | . | . | . | . | . | . | . | . | . | . | . | . | . | . | . | . | . | . | . | . |
| 21-225_77G1_HC | . | . | . | . | . | . | . | . | . | . | . | . | . | . | . | . | . | . | . | . | . | . | . | . | . | . |
| 21-225_79F11_HC | . | . | . | . | . | . | . | . | . | . | . | . | . | . | . | . | . | . | . | . | . | . | . | . | . | . |
| 21-225_79F3_HC | . | . | . | . | . | . | . | . | . | . | . | . | . | . | . | . | . | . | . | . | . | . | . | . | . | . |
| 21-225_79G1_HC | . | . | . | . | . | . | . | . | . | . | . | . | . | . | . | . | . | . | . | . | . | . | . | . | . | . |
| 21-225_79G12_HC | . | . | . | . | . | . | . | . | . | . | . | . | . | . | . | . | . | . | . | . | . | . | . | . | . | . |
| 21-225_80A1_HC | . | . | . | . | . | . | . | . | . | . | . | . | . | . | . | . | . | . | . | . | . | . | . | . | . | . |
| 21-225_80C1_HC | . | . | . | . | . | . | . | . | . | . | . | . | . | . | . | . | . | . | . | . | . | . | . | . | . | . |
| 21-225_80D5_HC | . | . | . | . | . | . | . | . | . | . | . | . | . | . | . | . | . | . | . | . | . | . | . | . | . | . |
| 21-225_80E12_HC | . | . | . | . | . | . | . | . | . | . | . | . | . | . | . | . | . | . | . | . | . | . | . | . | . | . |
| 21-225_80E3_HC | . | . | . | . | . | . | . | . | . | . | . | . | . | . | . | . | . | . | . | . | . | . | . | . | . | . |
| 21-225_80H12_HC | . | . | . | . | . | . | . | . | . | . | . | . | . | . | . | . | . | . | . | . | . | . | . | . | . | . |
| 21-225_80H7_HC | . | . | . | . | . | . | . | . | . | . | . | . | . | . | . | . | . | . | . | . | . | . | . | . | . | . |
| 21-225_81C11_HC | . | . | . | . | . | . | . | . | . | . | . | . | . | . | . | . | . | . | . | . | . | . | . | . | . | . |
| 21-225_81C5_HC | . | . | . | . | . | . | . | . | . | . | . | . | . | . | . | . | . | . | . | . | . | . | . | . | . | . |
| 21-225_83B7_HC | . | . | . | . | . | . | . | . | . | . | . | . | . | . | . | . | . | . | . | . | . | . | . | . | . | . |
| 21-225_83G1_HC | . | . | . | . | . | . | . | . | . | . | . | . | . | . | . | . | . | . | . | . | . | . | . | . | . | . |
| 21-225_84E12_HC | . | . | . | . | . | . | . | . | . | . | . | . | . | . | . | . | . | . | . | . | . | . | . | . | . | . |
| 21-225_85A3_HC | . | . | . | . | . | . | . | . | . | . | . | . | . | . | . | . | . | . | . | . | . | . | . | . | . | . |
| 21-225_85B4_HC | . | . | . | . | . | . | . | . | . | . | . | . | . | . | . | . | . | . | . | . | . | . | . | . | . | . |
| 21-225_85B9_HC | . | . | . | . | . | . | . | . | . | . | . | . | . | . | . | . | . | . | . | . | . | . | . | . | . | . |
| 21-225_85D6_HC | . | . | . | . | . | . | . | . | . | . | . | . | . | . | . | . | . | . | . | . | . | . | . | . | . | . |
| 21-225_85G10_HC | . | . | . | . | . | . | . | . | . | . | . | . | . | . | . | . | . | . | . | . | . | . | . | . | . | . |
| 21-225_86C1_HC | . | . | . | . | . | . | . | . | . | . | . | . | . | . | . | . | . | . | . | . | . | . | . | . | . | . |
| 21-225_86C11_HC | . | . | . | . | . | . | . | . | . | . | . | . | . | . | . | . | . | . | . | . | . | . | . | . | . | . |
| 21-225_86E4_HC | . | . | . | . | . | . | . | . | . | . | . | . | . | . | . | . | . | . | . | . | . | . | . | . | . | . |
| 21-225_87A12_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | D | Y | G | G | - | - | - | - | - | - | - | - | - | - | - | - | - | - | - | - | - | - |
| 21-225_87C9_HC | . | . | . | . | . | . | . | . | . | . | . | . | . | . | . | . | . | . | . | . | . | . | . | . | . | . |
| 21-225_87E5_HC | . | . | . | . | . | . | . | . | . | . | . | . | . | . | . | . | . | . | . | . | . | . | . | . | . | . |
| 21-225_87H1_HC | . | . | . | . | . | . | . | . | . | . | . | . | . | . | . | . | . | . | . | . | . | . | . | . | . | . |
| 21-225_88E7_HC | . | . | . | . | . | . | . | . | . | . | . | . | . | . | . | . | . | . | . | . | . | . | . | . | . | . |
| 21-225_88F7_HC | . | . | . | . | . | . | . | . | . | . | . | . | . | . | . | . | . | . | . | . | . | . | . | . | . | . |
| 21-225_88G9_HC | . | . | . | . | . | . | . | . | . | . | . | . | . | . | . | . | . | . | . | . | . | . | . | . | . | . |
| 21-225_88H1_HC | . | . | . | . | . | . | . | . | . | . | . | . | . | . | . | . | . | . | . | . | . | . | . | . | . | . |
| 21-225_89A11_HC | . | . | . | . | . | . | . | . | . | . | . | . | . | . | . | . | . | . | . | . | . | . | . | . | . | . |
| 21-225_89D5_HC | . | . | . | . | . | . | . | . | . | . | . | . | . | . | . | . | . | . | . | . | . | . | . | . | . | . |
| 21-225_89E10_HC | . | . | . | . | . | . | . | . | . | . | . | . | . | . | . | . | . | . | . | . | . | . | . | . | . | . |
| 21-225_89H5_HC | . | . | . | . | . | . | . | . | . | . | . | . | . | . | . | . | . | . | . | . | . | . | . | . | . | . |
| 21-225_90A5_HC | . | . | . | . | . | . | . | . | . | . | . | . | . | . | . | . | . | . | . | . | . | . | . | . | . | . |
| 21-225_90F10_HC | . | . | . | . | . | . | . | . | . | . | . | . | . | . | . | . | . | . | . | . | . | . | . | . | . | . |
| 21-225_90G4_HC | . | . | . | . | . | . | . | . | . | . | . | . | . | . | . | . | . | . | . | . | . | . | . | . | . | . |
| 21-225_90G5_HC | . | . | . | . | . | . | . | . | . | . | . | . | . | . | . | . | . | . | . | . | . | . | . | . | . | . |
| 21-225_91B2_HC | . | . | . | . | . | . | . | . | . | . | . | . | . | . | . | . | . | . | . | . | . | . | . | . | . | . |
| 21-225_91B3_HC | . | . | . | . | . | . | . | . | . | . | . | . | . | . | . | . | . | . | . | . | . | . | . | . | . | . |
| 21-225_91B4_HC | . | . | . | . | . | . | . | . | . | . | . | . | . | . | . | . | . | . | . | . | . | . | . | . | . | . |
| 21-225_91E9_HC | . | . | . | . | . | . | . | . | . | . | . | . | . | . | . | . | . | . | . | . | . | . | . | . | . | . |
| 21-225_91F3_HC | . | . | . | . | . | . | . | . | . | . | . | . | . | . | . | . | . | . | . | . | . | . | . | . | . | . |
| 21-225_92B1_HC | . | . | . | . | . | . | . | . | . | . | . | . | . | . | . | . | . | . | . | . | . | . | . | . | . | . |
| 21-225_92D6_HC | . | . | . | . | . | . | . | . | . | . | . | . | . | . | . | . | . | . | . | . | . | . | . | . | . | . |
| 21-225_93C2_HC | . | . | . | . | . | . | . | . | . | . | . | . | . | . | . | . | . | . | . | . | . | . | . | . | . | . |
| 21-225_93E4_HC | . | . | . | . | . | . | . | . | . | . | . | . | . | . | . | . | . | . | . | . | . | . | . | . | . | . |
| 21-225_94D3_HC | . | . | . | . | . | . | . | . | . | . | . | . | . | . | . | . | . | . | . | . | . | . | . | . | . | . |
| 21-225_94F12_HC | . | . | . | . | . | . | . | . | . | . | . | . | . | . | . | . | . | . | . | . | . | . | . | . | . | . |
| 21-225_94G10_HC | . | . | . | . | . | . | . | . | . | . | . | . | . | . | . | . | . | . | . | . | . | . | . | . | . | . |
| 21-225_95D2_HC | . | . | . | . | . | . | . | . | . | . | . | . | . | . | . | . | . | . | . | . | . | . | . | . | . | . |
| 21-225_95F9_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | D | Y | G | G | - | - | - | - | - | - | - | - | - | - | - | - | - | - | - | - | - | - |
| 21-225_74A3_HC | . | . | . | . | . | . | . | . | . | . | . | . | . | . | . | . | . | . | . | . | . | . | . | . | . | . |
| 21-225_74A4_HC | . | . | . | . | . | . | . | . | . | . | . | . | . | . | . | . | . | . | . | . | . | . | . | . | . | . |
| 21-225_74B12_HC | . | . | . | . | . | . | . | . | . | . | . | . | . | . | . | . | . | . | . | . | . | . | . | . | . | . |
| 21-225_74B2_HC | . | . | . | . | . | . | . | . | . | . | . | . | . | . | . | . | . | . | . | . | . | . | . | . | . | . |
| 21-225_74C5_HC | . | . | . | . | . | . | . | . | . | . | . | . | . | . | . | . | . | . | . | . | . | . | . | . | . | . |
| 21-225_74D11_HC | . | . | . | . | . | . | . | . | . | . | . | . | . | . | . | . | . | . | . | . | . | . | . | . | . | . |
| 21-225_74D5_HC | . | . | . | . | . | . | . | . | . | . | . | . | . | . | . | . | . | . | . | . | . | . | . | . | . | . |
| 21-225_74D6_HC | . | . | . | . | . | . | . | . | . | . | . | . | . | . | . | . | . | . | . | . | . | . | . | . | . | . |
| 21-225_74F5_HC | . | . | . | . | . | . | . | . | . | . | . | . | . | . | . | . | . | . | . | . | . | . | . | . | . | . |
| 21-225_74H5_HC | . | . | . | . | . | . | . | . | . | . | . | . | . | . | . | . | . | . | . | . | . | . | . | . | . | . |
| 21-225_74H7_HC | . | . | . | . | . | . | . | . | . | . | . | . | . | . | . | . | . | . | . | . | . | . | . | . | . | . |
| 21-225_75A12_HC | . | . | . | . | . | . | . | . | . | . | . | . | . | . | . | . | . | . | . | . | . | . | . | . | . | . |
| 21-225_75A5_HC | . | . | . | . | . | . | . | . | . | . | . | . | . | . | . | . | . | . | . | . | . | . | . | . | . | . |
| 21-225_75A8_HC | . | . | . | . | . | . | . | . | . | . | . | . | . | . | . | . | . | . | . | . | . | . | . | . | . | . |
| 21-225_75B10_HC | . | . | . | . | . | . | . | . | . | . | . | . | . | . | . | . | . | . | . | . | . | . | . | . | . | . |
| 21-225_75C2_HC | . | . | . | . | . | . | . | . | . | . | . | . | . | . | . | . | . | . | . | . | . | . | . | . | . | . |
| 21-225_75C3_HC | . | . | . | . | . | . | . | . | . | . | . | . | . | . | . | . | . | . | . | . | . | . | . | . | . | . |
| 21-225_75D3_HC | . | . | . | . | . | . | . | . | . | . | . | . | . | . | . | . | . | . | . | . | . | . | . | . | . | . |
| 21-225_75D9_HC | . | . | . | . | . | . | . | . | . | . | . | . | . | . | . | . | . | . | . | . | . | . | . | . | . | . |
| 21-225_75F3_HC | . | . | . | . | . | . | . | . | . | . | . | . | . | . | . | . | . | . | . | . | . | . | . | . | . | . |
| 21-225_75G12_HC | . | . | . | . | . | . | . | . | . | . | . | . | . | . | . | . | . | . | . | . | . | . | . | . | . | . |
| 21-225_75G7_HC | . | . | . | . | . | . | . | . | . | . | . | . | . | . | . | . | . | . | . | . | . | . | . | . | . | . |
| 21-225_75H8_HC | . | . | . | . | . | . | . | . | . | . | . | . | . | . | . | . | . | . | . | . | . | . | . | . | . | . |
| 21-225_76A4_HC | . | . | . | . | . | . | . | . | . | . | . | . | . | . | . | . | . | . | . | . | . | . | . | . | . | . |
| 21-225_76D1_HC | . | . | . | . | . | . | . | . | . | . | . | . | . | . | . | . | . | . | . | . | . | . | . | . | . | . |
| 21-225_76E10_HC | . | . | . | . | . | . | . | . | . | . | . | . | . | . | . | . | . | . | . | . | . | . | . | . | . | . |
| 21-225_76G4_HC | . | . | . | . | . | . | . | . | . | . | . | . | . | . | . | . | . | . | . | . | . | . | . | . | . | . |
| 21-225_76H10_HC | . | . | . | . | . | . | . | . | . | . | . | . | . | . | . | . | . | . | . | . | . | . | . | . | . | . |
| 21-225_77A10_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | D | Y | G | G | - | - | - | - | - | - | - | - | - | - | - | - | - | - | - | - | - | - |
| 21-225_95G9_HC | . | . | . | . | . | . | . | . | . | . | . | . | . | . | . | . | . | . | . | . | . | . | . | . | . | . |
| 21-225_95H10_HC | . | . | . | . | . | . | . | . | . | . | . | . | . | . | . | . | . | . | . | . | . | . | . | . | . | . |
| 21-225_97A2_HC | . | . | . | . | . | . | . | . | . | . | . | . | . | . | . | . | . | . | . | . | . | . | . | . | . | . |
| 21-225_97E5_HC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | - | M | D | V | W | G | Q | G | T | T | V | T | V | S | S |
| 21-225_74A3_HC | . | . | . | . | . | . | . | . | . | . | . | . | . | . | . | . | . | . | . |
| 21-225_74A4_HC | . | . | . | . | . | . | . | . | . | . | . | . | . | . | . | . | . | . | . |
| 21-225_74B12_HC | . | . | . | . | . | . | . | . | . | . | . | . | . | . | . | . | . | . | . |
| 21-225_74B2_HC | . | . | . | . | . | L | . | . | . | . | . | . | . | . | . | . | . | . | . |
| 21-225_74C5_HC | . | . | . | . | . | . | . | . | . | . | . | . | . | . | . | . | . | . | . |
| 21-225_74D11_HC | . | . | . | . | . | . | . | . | . | . | . | . | . | . | . | . | . | . | . |
| 21-225_74D5_HC | . | . | . | . | . | . | . | . | . | . | . | . | . | . | . | . | . | . | . |
| 21-225_74D6_HC | . | . | . | . | . | . | . | . | . | . | . | . | . | . | . | . | . | . | . |
| 21-225_74F5_HC | . | . | . | . | . | . | . | . | . | . | . | . | . | . | . | . | . | . | . |
| 21-225_74H5_HC | . | . | . | . | . | . | . | . | . | . | . | . | . | . | . | . | . | . | . |
| 21-225_74H7_HC | . | . | . | . | . | L | . | . | . | . | . | . | . | . | . | . | . | . | . |
| 21-225_75A12_HC | . | . | . | . | . | . | . | . | . | . | . | . | . | . | . | . | . | . | . |
| 21-225_75A5_HC | . | . | . | . | . | . | . | . | . | . | . | . | . | . | . | . | . | . | . |
| 21-225_75A8_HC | . | . | . | . | . | . | . | . | . | . | . | . | . | . | . | . | . | . | . |
| 21-225_75B10_HC | . | . | . | . | . | L | . | . | . | . | . | . | . | . | . | . | . | . | . |
| 21-225_75C2_HC | . | . | . | . | . | . | . | . | . | . | . | . | . | . | . | . | . | . | . |
| 21-225_75C3_HC | . | . | . | . | . | . | . | . | . | . | . | . | . | . | . | . | . | . | . |
| 21-225_75D3_HC | . | . | . | . | . | . | . | . | . | . | . | . | . | . | . | . | . | . | . |
| 21-225_75D9_HC | . | . | . | . | . | . | . | . | . | . | . | . | . | . | . | . | . | . | . |
| 21-225_75F3_HC | . | . | . | . | . | . | . | . | . | . | . | . | . | . | . | . | . | . | . |
| 21-225_75G12_HC | . | . | . | . | . | . | . | . | . | . | . | . | . | . | . | . | . | . | . |
| 21-225_75G7_HC | . | . | . | . | . | . | . | . | . | . | . | . | . | . | . | . | . | . | . |
| 21-225_75H8_HC | . | . | . | . | . | L | . | . | . | . | . | . | . | . | . | . | . | . | . |
| 21-225_76A4_HC | . | . | . | . | . | . | . | . | . | . | . | . | . | . | . | . | . | . | . |
| 21-225_76D1_HC | . | . | . | . | . | . | . | . | . | . | . | . | . | . | . | . | . | . | . |
| 21-225_76E10_HC | . | . | . | . | . | . | . | . | . | . | . | . | . | . | . | . | . | . | . |
| 21-225_76G4_HC | . | . | . | . | . | . | . | . | . | . | . | . | . | . | . | . | . | . | . |
| 21-225_76H10_HC | . | . | . | . | . | . | . | . | . | . | . | . | . | . | . | . | . | . | . |
| 21-225_77A10_HC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | - | M | D | V | W | G | Q | G | T | T | V | T | V | S | S |
| 21-225_77C12_HC | . | . | . | . | . | . | . | . | . | . | . | . | . | . | . | . | . | . | . |
| 21-225_77C5_HC | . | . | . | . | . | . | . | . | . | . | . | . | . | . | . | . | . | . | . |
| 21-225_77F7_HC | . | . | . | . | . | . | . | . | . | . | . | . | . | . | . | . | . | . | . |
| 21-225_77G1_HC | . | . | . | . | . | . | . | . | . | . | . | . | . | . | . | . | . | . | . |
| 21-225_79F11_HC | . | . | . | . | . | . | . | . | . | . | . | . | . | . | . | . | . | . | . |
| 21-225_79F3_HC | . | . | . | . | . | . | . | . | . | . | . | . | . | . | . | . | . | . | . |
| 21-225_79G1_HC | . | . | . | . | . | . | . | . | . | . | . | . | . | . | . | . | . | . | . |
| 21-225_79G12_HC | . | . | . | . | . | . | . | . | . | . | . | . | . | . | . | . | . | . | . |
| 21-225_80A1_HC | . | . | . | . | . | . | . | . | . | . | . | . | . | . | . | . | . | . | . |
| 21-225_80C1_HC | . | . | . | . | . | . | . | . | . | . | . | . | . | . | . | . | . | . | . |
| 21-225_80D5_HC | . | . | . | . | . | L | . | . | . | . | . | . | . | . | . | . | . | . | . |
| 21-225_80E12_HC | . | . | . | . | . | . | . | . | . | . | . | . | . | . | . | . | . | . | . |
| 21-225_80E3_HC | . | . | . | . | . | . | . | . | . | . | . | . | . | . | . | . | . | . | . |
| 21-225_80H12_HC | . | . | . | . | . | . | . | . | . | . | . | . | . | . | . | . | . | . | . |
| 21-225_80H7_HC | . | . | . | . | . | I | . | . | . | . | . | . | . | . | . | . | . | . | . |
| 21-225_81C11_HC | . | . | . | . | . | . | . | . | . | . | . | . | . | . | . | . | . | . | . |
| 21-225_81C5_HC | . | . | . | . | . | . | . | . | . | . | . | . | . | . | . | . | . | . | . |
| 21-225_83B7_HC | . | . | . | . | . | . | . | . | . | . | . | . | . | . | . | . | . | . | . |
| 21-225_83G1_HC | . | . | . | . | . | . | . | . | . | . | . | . | . | . | . | . | . | . | . |
| 21-225_84E12_HC | . | . | . | . | . | I | . | . | . | . | . | . | . | . | . | . | . | . | . |
| 21-225_85A3_HC | . | . | . | . | . | . | . | . | . | . | . | . | . | . | . | . | . | . | . |
| 21-225_85B4_HC | . | . | . | . | . | . | . | . | . | . | . | . | . | . | . | . | . | . | . |
| 21-225_85B9_HC | . | . | . | . | . | . | . | . | . | . | . | . | . | . | . | . | . | . | . |
| 21-225_85D6_HC | . | . | . | . | . | . | . | . | . | . | . | . | . | . | . | . | . | . | . |
| 21-225_85G10_HC | . | . | . | . | . | L | . | . | . | . | . | . | . | . | . | . | . | . | . |
| 21-225_86C1_HC | . | . | . | . | . | . | . | . | . | . | . | . | . | . | . | . | . | . | . |
| 21-225_86C11_HC | . | . | . | . | . | . | . | . | . | . | . | . | . | . | . | . | . | . | . |
| 21-225_86E4_HC | . | . | . | . | . | . | . | . | . | . | . | . | . | . | . | . | . | . | . |
| 21-225_87A12_HC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | - | M | D | V | W | G | Q | G | T | T | V | T | V | S | S |
| 21-225_87C9_HC | . | . | . | . | . | . | . | . | . | . | . | . | . | . | . | . | . | . | . |
| 21-225_87E5_HC | . | . | . | . | . | . | . | . | . | . | . | . | . | . | . | . | . | . | . |
| 21-225_87H1_HC | . | . | . | . | . | L | . | . | . | . | . | . | . | . | . | . | . | . | . |
| 21-225_88E7_HC | . | . | . | . | . | . | . | . | . | . | . | . | . | . | . | . | . | . | . |
| 21-225_88F7_HC | . | . | . | . | . | . | . | . | . | . | . | . | . | . | . | . | . | . | . |
| 21-225_88G9_HC | . | . | . | . | . | . | . | . | . | . | . | . | . | . | . | . | . | . | . |
| 21-225_88H1_HC | . | . | . | . | . | . | . | . | . | . | . | . | . | . | . | . | . | . | . |
| 21-225_89A11_HC | . | . | . | . | . | L | . | . | . | . | . | . | . | . | . | . | . | . | . |
| 21-225_89D5_HC | . | . | . | . | . | . | . | . | . | . | . | . | . | . | . | . | . | . | . |
| 21-225_89E10_HC | . | . | . | . | . | . | . | . | . | . | . | . | . | . | . | . | . | . | . |
| 21-225_89H5_HC | . | . | . | . | . | . | . | . | . | . | . | . | . | . | . | . | . | . | . |
| 21-225_90A5_HC | . | . | . | . | . | . | . | . | . | . | . | . | . | . | . | . | . | . | . |
| 21-225_90F10_HC | . | . | . | . | . | . | . | . | . | . | . | . | . | . | . | . | . | . | . |
| 21-225_90G4_HC | . | . | . | . | . | . | . | . | . | . | . | . | . | . | . | . | . | . | . |
| 21-225_90G5_HC | . | . | . | . | . | . | . | . | . | . | . | . | . | . | . | . | . | . | . |
| 21-225_91B2_HC | . | . | . | . | . | . | . | . | . | . | . | . | . | . | . | . | . | . | . |
| 21-225_91B3_HC | . | . | . | . | . | . | . | . | . | . | . | . | . | . | . | . | . | . | . |
| 21-225_91B4_HC | . | . | . | . | . | . | . | . | . | . | . | . | . | . | . | . | . | . | . |
| 21-225_91E9_HC | . | . | . | . | . | . | . | . | . | . | . | . | . | . | . | . | . | . | . |
| 21-225_91F3_HC | . | . | . | . | . | . | . | . | . | . | . | . | . | . | . | . | . | . | . |
| 21-225_92B1_HC | . | . | . | . | . | L | . | . | . | . | . | . | . | . | . | . | . | . | . |
| 21-225_92D6_HC | . | . | . | . | . | . | . | . | . | . | . | . | . | . | . | . | . | . | . |
| 21-225_93C2_HC | . | . | . | . | . | . | . | . | . | . | . | . | . | . | . | . | . | . | . |
| 21-225_93E4_HC | . | . | . | . | . | . | . | . | . | . | . | . | . | . | . | . | . | . | . |
| 21-225_94D3_HC | . | . | . | . | . | . | . | . | . | . | . | . | . | . | . | . | . | . | . |
| 21-225_94F12_HC | . | . | . | . | . | . | . | . | . | . | . | . | . | . | . | . | . | . | . |
| 21-225_94G10_HC | . | . | . | . | . | . | . | . | . | . | . | . | . | . | . | . | . | . | . |
| 21-225_95D2_HC | . | . | . | . | . | . | . | . | . | . | . | . | . | . | . | . | . | . | . |
| 21-225_95F9_HC | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | - | M | D | V | W | G | Q | G | T | T | V | T | V | S | S |
| 21-225_95G9_HC | . | . | . | . | . | . | . | . | . | . | . | . | . | . | . | . | . | . | . |
| 21-225_95H10_HC | . | . | . | . | . | . | . | . | . | . | . | . | . | . | . | . | . | . | . |
| 21-225_97A2_HC | . | . | . | . | . | . | . | . | . | . | . | . | . | . | . | . | . | . | . |
| 21-225_97E5_HC | . | . | . | . | . | . | . | . | . | . | . | . | . | . | . | . | . | . | . |

Table 53. Consensus 19-VH1|1-08/D6|6-19|RF1/JH5 (SEQ ID NO: 50270):

QVQLVQS-GAEVKKPGASVKVSCKASG-YTFTN-----YDINWVRQATGQGLEWMGWMHPN---SGNTGYAQKFQGRVTMTRNTSISTAYMELSSLRSEDTAVYYCAISSGWY--------------------WFDPWGQGTLVTVSS wherein:

H at position 59 can be substituted with N, or Y

N at position 61 can be substituted with D

S at position 65 can be substituted with N

G at position 66 can be substituted with V

N at position 67 can be substituted with S

T at position 68 can be substituted with I

Y at position 70 can be substituted with F, or C

G at position 76 can be substituted with D

Y at position 113 can be substituted with H, N, S, or K

**FIGURE 57**

W at position 135 can be substituted with R

Asn Tyr Asp Ile Asn (SEQ ID NO: 50013)

Trp Met Xaa Pro Xaa Xaa Xaa Xaa Xaa Gly Xaa Ala Gln Lys Phe Gln Xaa (SEQ ID NO: 50014)

Ser Ser Gly Trp Xaa Xaa Phe Asp Pro (SEQ ID NO: 50015)

Asn Tyr Asp Ile Asn (SEQ ID NO: 50480)

Trp Met His Pro Asn Ser Gly Asn Thr Gly Tyr Ala Gln Lys Phe Gln Gly (SEQ ID NO: 50481)

Ser Ser Gly Trp Tyr Trp Phe Asp Pro (SEQ ID NO: 50482)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | Q | S | - | G | A | E | V | K | K | P | G | A | S | V | K | V | S | C | K | A | S |
| 21-225_147G6_HC | . | . | . | . | . | . | . | . | . | . | . | . | . | . | . | . | . | . | . | . | . | . | . | . | . | . |
| 21-225_152F4_HC | . | . | . | . | . | . | . | . | . | . | . | . | . | . | . | . | . | . | . | . | . | . | . | . | . | . |
| 21-225_170F6_HC | . | . | . | . | . | . | . | . | . | . | . | . | . | . | . | . | . | . | . | . | . | . | . | . | . | . |
| 21-225_174G5_HC | . | . | . | . | . | . | . | . | . | . | . | . | . | . | . | . | . | . | . | . | . | . | . | . | . | . |
| 21-225_224H5_HC | . | . | . | . | . | . | . | . | . | . | . | . | . | . | . | . | . | . | . | . | . | . | . | . | . | . |
| 21-225_225F4_HC | . | . | . | . | . | . | . | . | . | . | . | . | . | . | . | . | . | . | . | . | . | . | . | . | . | . |
| 21-225_227G9_HC | . | . | . | . | . | . | . | . | . | . | . | . | . | . | . | . | . | . | . | . | . | . | . | | . | . |
| 21-225_25A3_HC | . | . | . | . | . | . | . | . | . | . | . | . | . | . | . | . | . | . | . | . | . | . | . | . | . | . |
| 21-225_26A9_HC | . | . | . | . | . | . | . | . | . | . | . | . | . | . | . | . | . | . | . | . | . | . | . | . | . | . |
| 21-225_57A11_HC | . | . | . | . | . | . | . | . | . | . | . | . | . | . | . | . | . | . | . | . | . | . | . | . | . | . |
| 21-225_57F12_HC | . | . | . | . | . | . | . | . | . | . | . | . | . | . | . | . | . | . | . | . | . | . | . | . | . | . |
| 21-225_74A5_HC | . | . | . | . | . | . | . | . | . | . | . | . | . | . | . | . | . | . | . | . | . | . | . | . | . | . |
| 21-225_74A7_HC | . | . | . | . | . | . | . | . | . | . | . | . | . | . | . | . | . | . | . | . | . | . | . | . | . | . |
| 21-225_74B10_HC | . | . | . | . | . | . | . | . | . | . | . | . | . | . | . | . | . | . | . | . | . | . | . | . | . | . |
| 21-225_74B5_HC | . | . | . | . | . | . | . | . | . | . | . | . | . | . | . | . | . | . | . | . | . | . | . | . | . | . |
| 21-225_74B6_HC | . | . | . | . | . | . | . | . | . | . | . | . | . | . | . | . | . | . | . | . | . | . | . | . | . | . |
| 21-225_74B7_HC | . | . | . | . | . | . | . | . | . | . | . | . | . | . | . | . | . | . | . | . | . | . | . | . | . | . |
| 21-225_74B8_HC | . | . | . | . | . | . | . | . | . | . | . | . | . | . | . | . | . | . | . | . | . | . | . | . | . | . |
| 21-225_74C1_HC | . | . | . | . | . | . | . | . | . | . | . | . | . | . | . | . | . | . | . | . | . | . | . | . | . | . |
| 21-225_74C10_HC | . | . | . | . | . | . | . | . | . | . | . | . | . | . | . | . | . | . | . | . | . | . | . | . | . | . |
| 21-225_74C12_HC | . | . | . | . | . | . | . | . | . | . | . | . | . | . | . | . | . | . | . | . | . | . | . | . | . | . |
| 21-225_74G3_HC | . | . | . | . | . | . | . | . | . | . | . | . | . | . | . | . | . | . | . | . | . | . | . | . | . | . |
| 21-225_74H2_HC | . | . | . | . | . | . | . | . | . | . | . | . | . | . | . | . | . | . | . | . | . | . | . | . | . | . |
| 21-225_75A7_HC | . | . | . | . | . | . | . | . | . | . | . | . | . | . | . | . | . | . | . | . | . | . | . | . | . | . |
| 21-225_75B1_HC | . | . | . | . | . | . | . | . | . | . | . | . | . | . | . | . | . | . | . | . | . | . | . | . | . | . |
| 21-225_76A6_HC | . | . | . | . | . | . | . | . | . | . | . | . | . | . | . | . | . | . | . | . | . | . | . | . | . | . |
| 21-225_76B9_HC | . | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | Q | S | - | G | A | E | V | K | K | P | G | A | S | V | K | V | S | C | K | A | S |
| 21-225_76E8_HC | . | . | . | . | . | . | . | . | . | . | . | . | . | . | . | . | . | . | . | . | . | . | . | . | . | . |
| 21-225_76F3_HC | . | . | . | . | . | . | . | . | . | . | . | . | . | . | . | . | . | . | . | . | . | . | . | . | . | . |
| 21-225_76F5_HC | . | . | . | . | . | . | . | . | . | . | . | . | . | . | . | . | . | . | . | . | . | . | . | . | . | . |
| 21-225_76H1_HC | . | . | . | . | . | . | . | . | . | . | . | . | . | . | . | . | . | . | . | . | . | . | . | . | . | . |
| 21-225_77C10_HC | . | . | . | . | . | . | . | . | . | . | . | . | . | . | . | . | . | . | . | . | . | . | . | . | . | . |
| 21-225_77C7_HC | . | . | . | . | . | . | . | . | . | . | . | . | . | . | . | . | . | . | . | . | . | . | . | . | . | . |
| 21-225_77D11_HC | . | . | . | . | . | . | . | . | . | . | . | . | . | . | . | . | . | . | . | . | . | . | . | . | . | . |
| 21-225_77D12_HC | . | . | . | . | . | . | . | . | . | . | . | . | . | . | . | . | . | . | . | . | . | . | . | . | . | . |
| 21-225_77E1_HC | . | . | . | . | . | . | . | . | . | . | . | . | . | . | . | . | . | . | . | . | . | . | . | . | . | . |
| 21-225_78C1_HC | . | . | . | . | . | . | . | . | . | . | . | . | . | . | . | . | . | . | . | . | . | . | . | . | . | . |
| 21-225_78H12_HC | . | . | . | . | . | . | . | . | . | . | . | . | . | . | . | . | . | . | . | . | . | . | . | . | . | . |
| 21-225_79G10_HC | . | . | . | . | . | . | . | . | . | . | . | . | . | . | . | . | . | . | . | . | . | . | . | . | . | . |
| 21-225_79G6_HC | . | . | . | . | . | . | . | . | . | . | . | . | . | . | . | . | . | . | . | . | . | . | . | . | . | . |
| 21-225_80A2_HC | . | . | . | . | . | . | . | . | . | . | . | . | . | . | . | . | . | . | . | . | . | . | . | . | . | . |
| 21-225_80C12_HC | . | . | . | . | . | . | . | . | . | . | . | . | . | . | . | . | . | . | . | . | . | . | . | . | . | . |
| 21-225_80D3_HC | . | . | . | . | . | . | . | . | . | . | . | . | . | . | . | . | . | . | . | . | . | . | . | . | . | . |
| 21-225_80E9_HC | . | . | . | . | . | . | . | . | . | . | . | . | . | . | . | . | . | . | . | . | . | . | . | . | . | . |
| 21-225_81E5_HC | . | . | . | . | . | . | . | . | . | . | . | . | . | . | . | . | . | . | . | . | . | . | . | . | . | . |
| 21-225_81F9_HC | . | . | . | . | . | . | . | . | . | . | . | . | . | . | . | . | . | . | . | . | . | . | . | . | . | . |
| 21-225_82A5_HC | . | . | . | . | . | . | . | . | . | . | . | . | . | . | . | . | . | . | . | . | . | . | . | . | . | . |
| 21-225_82C12_HC | . | . | . | . | . | . | . | . | . | . | . | . | . | . | . | . | . | . | . | . | . | . | . | . | . | . |
| 21-225_82D9_HC | . | . | . | . | . | . | . | . | . | . | . | . | . | . | . | . | . | . | . | . | . | . | . | . | . | . |
| 21-225_82G5_HC | . | . | . | . | . | . | . | . | . | . | . | . | . | . | . | . | . | . | . | . | . | . | . | . | . | . |
| 21-225_83C2_HC | . | . | . | . | . | . | . | . | . | . | . | . | . | . | . | . | . | . | . | . | . | . | . | . | . | . |
| 21-225_83C5_HC | . | . | . | . | . | . | . | . | . | . | . | . | . | . | . | . | . | . | . | . | . | . | . | . | . | . |
| 21-225_83F3_HC | . | . | . | . | . | . | . | . | . | . | . | . | . | . | . | . | . | . | . | . | . | . | . | . | . | . |
| 21-225_83G3_HC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | Q | S | - | G | A | E | V | K | K | P | G | A | S | V | K | V | S | C | K | A | S |
| 21-225_83G7_HC | . | . | . | . | . | . | . | . | . | . | . | . | . | . | . | . | . | . | . | . | . | . | . | . | . | . |
| 21-225_84G7_HC | . | . | . | . | . | . | . | . | . | . | . | . | . | . | . | . | . | . | . | . | . | . | . | . | . | . |
| 21-225_85B5_HC | . | . | . | . | . | . | . | . | . | . | . | . | . | . | . | . | . | . | . | . | . | . | . | . | . | . |
| 21-225_85D11_HC | . | . | . | . | . | . | . | . | . | . | . | . | . | . | . | . | . | . | . | . | . | . | . | . | . | . |
| 21-225_85H2_HC | . | . | . | . | . | . | . | . | . | . | . | . | . | . | . | . | . | . | . | . | . | . | . | . | . | . |
| 21-225_86E9_HC | . | . | . | . | . | . | . | . | . | . | . | . | . | . | . | . | . | . | . | . | . | . | . | . | . | . |
| 21-225_87A10_HC | . | . | . | . | . | . | . | . | . | . | . | . | . | . | . | . | . | . | . | . | . | . | . | . | . | . |
| 21-225_88G2_HC | . | . | . | . | . | . | . | . | . | . | . | . | . | . | . | . | . | . | . | . | . | . | . | . | . | . |
| 21-225_90D9_HC | . | . | . | . | . | . | . | . | . | . | . | . | . | . | . | . | . | . | . | . | . | . | . | . | . | . |
| 21-225_91F1_HC | . | . | . | . | . | . | . | . | . | . | . | . | . | . | . | . | . | . | . | . | . | . | . | . | . | . |
| 21-225_91G8_HC | . | . | . | . | . | . | . | . | . | . | . | . | . | . | . | . | . | . | . | . | . | . | . | . | . | . |
| 21-225_94D2_HC | . | . | . | . | . | . | . | . | . | . | . | . | . | . | . | . | . | . | . | . | . | . | . | . | . | . |
| 21-225_94E11_HC | . | . | . | . | . | . | . | . | . | . | . | . | . | . | . | . | . | . | . | . | . | . | . | . | . | . |
| 21-225_95E12_HC | . | . | . | . | . | . | . | . | . | . | . | . | . | . | . | . | . | . | . | . | . | . | . | . | . | . |
| 21-225_95G4_HC | . | . | . | . | . | . | . | . | . | . | . | . | . | . | . | . | . | . | . | . | . | . | . | . | . | . |
| 21-225_95H4_HC | . | . | . | . | . | . | . | . | . | . | . | . | . | . | . | . | . | . | . | . | . | . | . | . | . | . |
| 21-225_96D5_HC | . | . | . | . | . | . | . | . | . | . | . | . | . | . | . | . | . | . | . | . | . | . | . | . | . | . |
| 21-225_96E2_HC | . | . | . | . | . | . | . | . | . | . | . | . | . | . | . | . | . | . | . | . | . | . | . | . | . | . |
| 21-225_96G1_HC | . | . | . | . | . | . | . | . | . | . | . | . | . | . | . | . | . | . | . | . | . | . | . | . | . | . |
| 21-225_97H4_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | Y | T | F | T | N | - | - | - | - | - | Y | D | I | N | W | V | R | Q | A | T | G | Q | G | L |
| 21-225_147G6_HC | . | . | . | . | . | . | . | . | . | . | . | . | . | . | . | . | . | . | . | . | . | . | . | . | . | . |
| 21-225_152F4_HC | . | . | . | . | . | . | . | . | . | . | . | . | . | . | . | . | . | . | . | . | . | . | . | . | . | . |
| 21-225_170F6_HC | . | . | . | . | . | . | . | . | . | . | . | . | . | . | . | . | . | . | . | . | . | . | . | . | . | . |
| 21-225_174G5_HC | . | . | . | . | . | . | . | . | . | . | . | . | . | . | . | . | . | . | . | . | . | . | . | . | . | . |
| 21-225_224H5_HC | . | . | . | . | . | . | . | . | . | . | . | . | . | . | . | . | . | . | . | . | . | . | . | . | . | . |
| 21-225_225F4_HC | . | . | . | . | . | . | . | . | . | . | . | . | . | . | . | . | . | . | . | . | . | . | . | . | . | . |
| 21-225_227G9_HC | . | . | . | . | . | . | . | . | . | . | . | . | . | . | . | . | . | . | . | . | . | . | . | R | . | . |
| 21-225_25A3_HC | . | . | . | I | . | . | . | . | . | . | . | . | . | . | . | . | . | . | . | . | . | . | . | . | . | . |
| 21-225_26A9_HC | . | . | . | . | . | . | . | . | . | . | . | . | . | . | . | . | . | . | . | . | . | . | . | . | . | . |
| 21-225_57A11_HC | . | . | . | . | . | . | . | . | . | . | . | . | . | . | . | . | . | . | . | . | . | . | . | . | . | . |
| 21-225_57F12_HC | . | . | . | . | . | . | . | . | . | . | . | . | . | . | . | . | . | . | . | . | . | . | . | . | . | . |
| 21-225_74A5_HC | . | . | . | . | . | . | . | . | . | . | . | . | . | . | . | . | . | . | . | . | V | P | . | . | . | . |
| 21-225_74A7_HC | . | . | . | . | . | . | . | . | . | . | . | . | . | . | . | . | . | . | . | . | . | . | . | . | . | . |
| 21-225_74B10_HC | . | . | F | . | . | . | . | . | . | . | . | . | . | . | . | . | . | . | . | . | . | . | . | . | . | . |
| 21-225_74B5_HC | . | . | . | . | . | . | . | . | . | . | . | . | . | . | . | . | . | . | . | . | . | . | . | . | . | . |
| 21-225_74B6_HC | . | . | . | . | . | . | . | . | . | . | . | . | . | . | . | . | . | . | . | . | . | . | . | . | . | . |
| 21-225_74B7_HC | . | . | . | . | . | P | . | . | . | . | . | . | . | . | . | . | . | . | . | . | . | . | . | . | . | . |
| 21-225_74B8_HC | . | . | . | . | . | . | . | . | . | . | . | . | . | . | . | . | . | . | . | . | . | . | . | . | . | . |
| 21-225_74C1_HC | . | . | . | . | . | . | . | . | . | . | . | . | . | . | . | . | . | . | . | . | . | . | . | . | . | . |
| 21-225_74C10_HC | . | . | . | . | . | . | . | . | . | . | . | . | . | . | . | . | . | . | . | . | . | . | . | . | . | . |
| 21-225_74C12_HC | . | . | . | . | . | . | . | . | . | . | . | . | . | . | . | . | . | . | . | . | . | . | . | . | . | . |
| 21-225_74G3_HC | . | . | . | . | . | . | . | . | . | . | . | . | . | . | . | . | . | . | . | . | V | P | . | . | . | . |
| 21-225_74H2_HC | . | . | F | . | . | . | . | . | . | . | . | . | . | . | . | . | . | . | . | . | . | . | . | . | . | . |
| 21-225_75A7_HC | . | . | . | . | . | P | . | . | . | . | . | . | . | . | . | . | . | . | . | . | . | . | . | . | . | . |
| 21-225_75B1_HC | . | . | . | . | . | . | . | . | . | . | . | . | . | . | . | . | . | . | . | . | V | P | . | . | . | . |
| 21-225_76A6_HC | . | . | . | . | . | . | . | . | . | . | . | . | . | . | . | . | . | . | . | . | . | . | . | . | . | . |
| 21-225_76B9_HC | . | . | . | . | . | . | . | . | . | . | . | . | . | . | . | . | . | . | . | . | V | P | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | Y | T | F | T | N | - | - | - | - | - | Y | D | I | N | W | V | R | Q | A | T | G | Q | G | L |
| 21-225_76E8_HC | . | . | . | . | . | P | . | . | . | . | . | . | . | . | . | . | . | . | . | . | . | . | . | . | . | . |
| 21-225_76F3_HC | . | . | . | . | . | . | . | . | . | . | . | . | . | . | . | . | . | . | . | . | . | . | . | . | . | . |
| 21-225_76F5_HC | . | . | . | . | . | . | . | . | . | . | . | . | . | . | . | . | . | . | . | . | . | . | . | . | . | . |
| 21-225_76H1_HC | . | . | . | . | . | . | . | . | . | . | . | . | . | . | . | . | . | . | . | . | V | P | . | . | . | . |
| 21-225_77C10_HC | . | . | . | . | . | . | . | . | . | . | . | . | . | . | . | . | . | . | . | . | . | . | . | . | . | . |
| 21-225_77C7_HC | . | . | . | . | . | P | . | . | . | . | . | . | . | . | . | . | . | . | . | . | . | . | . | . | . | . |
| 21-225_77D11_HC | . | . | . | . | . | . | . | . | . | . | . | . | . | . | . | . | . | . | . | . | V | P | . | . | . | . |
| 21-225_77D12_HC | . | . | . | . | . | . | . | . | . | . | . | . | . | . | . | . | . | . | . | . | . | . | . | . | . | . |
| 21-225_77E1_HC | . | . | . | . | . | P | . | . | . | . | . | . | . | . | . | . | . | . | . | . | . | . | . | . | . | . |
| 21-225_78C1_HC | . | . | . | . | . | . | . | . | . | . | . | . | . | . | . | . | . | . | . | . | . | . | . | . | . | . |
| 21-225_78H12_HC | . | . | . | . | . | P | . | . | . | . | . | . | . | . | . | . | . | . | . | . | . | . | . | . | . | . |
| 21-225_79G10_HC | . | . | . | . | . | . | . | . | . | . | . | . | . | . | . | . | . | . | . | . | . | . | . | . | . | . |
| 21-225_79G6_HC | . | . | P | . | . | . | . | . | . | . | . | . | . | . | . | . | . | . | . | . | . | . | . | . | . | . |
| 21-225_80A2_HC | . | . | . | . | . | . | . | . | . | . | . | . | . | . | . | . | . | . | . | . | V | P | . | . | . | . |
| 21-225_80C12_HC | . | . | . | . | . | . | . | . | . | . | . | . | . | . | . | . | . | . | . | . | . | . | . | . | . | . |
| 21-225_80D3_HC | . | . | . | . | . | . | . | . | . | . | . | . | . | . | . | . | . | . | . | . | . | . | . | . | . | . |
| 21-225_80E9_HC | . | . | . | . | . | . | . | . | . | . | . | . | . | . | . | . | . | . | . | . | . | . | . | . | . | . |
| 21-225_81E5_HC | . | . | . | . | . | . | . | . | . | . | . | . | . | . | . | . | . | . | . | . | . | . | . | . | . | . |
| 21-225_81F9_HC | . | . | . | . | . | . | . | . | . | . | . | . | . | . | . | . | . | . | . | . | . | . | . | . | . | . |
| 21-225_82A5_HC | . | . | . | . | . | . | . | . | . | . | . | . | . | . | . | . | . | . | . | . | . | . | . | . | . | . |
| 21-225_82C12_HC | . | . | . | . | . | . | . | . | . | . | . | . | . | . | . | . | . | . | . | . | . | . | . | . | . | . |
| 21-225_82D9_HC | . | . | . | . | . | . | . | . | . | . | . | . | . | . | . | . | . | . | . | . | V | P | . | . | . | . |
| 21-225_82G5_HC | . | . | . | . | . | . | . | . | . | . | . | . | . | . | . | . | . | . | . | . | V | P | . | . | . | . |
| 21-225_83C2_HC | . | . | . | . | . | . | . | . | . | . | . | . | . | . | . | . | . | . | . | . | V | P | . | . | . | . |
| 21-225_83C5_HC | . | . | . | . | . | . | . | . | . | . | . | . | . | . | . | . | . | . | . | . | V | P | . | . | . | . |
| 21-225_83F3_HC | . | . | . | . | . | . | . | . | . | . | . | . | . | . | . | . | . | . | . | . | . | . | . | . | . | . |
| 21-225_83G3_HC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | Y | T | F | T | N | - | - | - | - | - | Y | D | I | N | W | V | R | Q | A | T | G | Q | G | L |
| 21-225_83G7_HC | . | . | . | . | . | P | . | . | . | . | . | . | . | . | . | . | . | . | . | . | . | . | . | . | . | . |
| 21-225_84G7_HC | . | . | . | . | . | P | . | . | . | . | . | . | . | . | . | . | . | . | . | . | . | . | . | . | . | . |
| 21-225_85B5_HC | . | . | . | . | . | . | . | . | . | . | . | . | . | . | . | . | . | . | . | . | V | P | . | . | . | . |
| 21-225_85D11_HC | . | . | . | . | . | . | . | . | . | . | . | . | . | . | . | . | . | . | . | . | V | P | . | . | . | . |
| 21-225_85H2_HC | . | . | . | . | . | . | . | . | . | . | . | . | . | . | . | . | . | . | . | . | . | . | . | . | . | . |
| 21-225_86E9_HC | . | . | . | . | . | . | . | . | . | . | . | . | . | . | . | . | . | . | . | . | . | . | . | . | . | . |
| 21-225_87A10_HC | . | . | . | . | . | . | . | . | . | . | . | . | . | . | . | . | . | . | . | . | . | . | . | . | . | . |
| 21-225_88G2_HC | . | . | . | . | . | . | . | . | . | . | . | . | . | . | . | . | . | . | . | . | . | . | . | . | . | . |
| 21-225_90D9_HC | . | . | . | . | . | P | . | . | . | . | . | . | . | . | . | . | . | . | . | . | . | . | . | . | . | . |
| 21-225_91F1_HC | . | . | . | . | . | . | . | . | . | . | . | . | . | . | . | . | . | . | . | . | . | . | . | . | . | . |
| 21-225_91G8_HC | . | . | . | . | . | . | . | . | . | . | . | . | . | . | . | . | . | . | . | . | . | . | . | . | . | . |
| 21-225_94D2_HC | . | . | F | . | . | . | . | . | . | . | . | . | . | . | . | . | . | . | . | . | . | . | . | . | . | . |
| 21-225_94E11_HC | . | . | . | . | . | . | . | . | . | . | . | . | . | . | . | . | . | . | . | . | . | . | . | . | . | . |
| 21-225_95E12_HC | . | . | . | . | . | . | . | . | . | . | . | . | . | . | . | . | . | . | . | . | . | . | . | . | . | . |
| 21-225_95G4_HC | . | . | . | . | . | . | . | . | . | . | . | . | . | . | . | . | . | . | . | . | . | . | . | . | . | . |
| 21-225_95H4_HC | . | . | . | . | . | . | . | . | . | . | . | . | . | . | . | . | . | . | . | . | . | . | . | . | . | . |
| 21-225_96D5_HC | . | . | . | . | . | . | . | . | . | . | . | . | . | . | . | . | . | . | . | . | . | . | . | . | . | . |
| 21-225_96E2_HC | . | . | . | . | . | . | . | . | . | . | . | . | . | . | . | . | . | . | . | . | . | . | . | . | . | . |
| 21-225_96G1_HC | . | . | . | . | . | . | . | . | . | . | . | . | . | . | . | . | . | . | . | . | . | . | . | . | . | . |
| 21-225_97H4_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | M | G | W | M | H | P | N | - | - | - | S | G | N | T | G | Y | A | Q | K | F | Q | G | R | V |
| 21-225_147G6_HC | . | . | . | . | . | . | . | . | . | . | . | . | . | . | . | . | . | . | . | . | . | . | . | . | . | . |
| 21-225_152F4_HC | . | . | . | . | . | . | . | . | . | . | . | . | . | . | . | . | . | . | . | . | . | . | . | . | . | . |
| 21-225_170F6_HC | . | . | . | . | . | . | N | . | . | . | . | . | . | . | . | . | . | . | . | . | . | . | . | D | . | . |
| 21-225_174G5_HC | . | . | . | . | . | . | . | . | . | . | . | . | . | . | . | . | . | . | . | . | . | . | . | . | . | . |
| 21-225_224H5_HC | . | . | . | . | . | . | N | . | . | . | . | . | . | . | . | . | . | . | . | . | . | . | . | . | . | . |
| 21-225_225F4_HC | . | . | . | . | . | . | . | . | . | . | . | . | . | . | S | . | . | . | . | . | . | . | . | . | . | L |
| 21-225_227G9_HC | . | . | . | . | . | . | Y | . | . | . | . | . | . | . | . | . | . | . | . | . | . | . | . | . | . | . |
| 21-225_25A3_HC | . | . | . | . | . | . | . | . | . | . | . | . | . | . | . | . | . | . | . | . | . | . | . | . | . | . |
| 21-225_26A9_HC | . | . | . | . | . | . | . | . | . | . | . | . | . | . | . | . | . | C | . | . | . | . | . | . | . | . |
| 21-225_57A11_HC | . | . | . | . | . | . | N | . | . | . | . | . | . | V | . | . | . | . | . | . | . | . | . | . | . | . |
| 21-225_57F12_HC | . | . | . | . | . | . | N | . | . | . | . | . | . | . | . | . | . | . | . | . | . | . | . | . | . | . |
| 21-225_74A5_HC | . | . | . | . | . | . | . | . | . | . | . | . | . | . | . | . | . | . | . | . | . | . | . | . | . | . |
| 21-225_74A7_HC | . | . | . | . | . | . | N | . | . | . | . | . | . | . | . | . | . | . | . | . | . | . | . | . | . | . |
| 21-225_74B10_HC | . | . | . | . | . | . | . | . | . | . | . | . | . | . | . | . | . | F | . | . | . | . | . | . | . | . |
| 21-225_74B5_HC | . | . | . | . | . | . | N | . | . | . | . | . | . | . | . | . | . | . | . | . | . | . | . | . | . | . |
| 21-225_74B6_HC | . | . | . | . | . | . | . | . | . | . | . | . | N | . | . | . | . | . | . | . | . | . | . | . | . | . |
| 21-225_74B7_HC | . | . | . | . | . | . | N | . | . | . | . | . | . | . | . | . | . | . | . | . | . | . | . | . | . | . |
| 21-225_74B8_HC | . | . | . | . | . | . | . | . | . | . | . | . | . | . | . | . | . | . | . | . | . | . | . | . | . | . |
| 21-225_74C1_HC | . | . | . | . | . | . | N | . | D | . | . | . | . | . | . | . | . | F | . | . | . | . | . | . | . | . |
| 21-225_74C10_HC | . | . | . | . | . | . | N | . | . | . | . | . | . | . | . | I | . | . | . | . | . | . | . | . | . | . |
| 21-225_74C12_HC | . | . | . | . | . | . | N | . | . | . | . | . | . | . | . | . | . | . | . | . | . | . | . | . | . | . |
| 21-225_74G3_HC | . | . | . | . | . | . | . | . | . | . | . | . | . | . | . | . | . | . | . | . | . | . | . | . | . | . |
| 21-225_74H2_HC | . | . | . | . | . | . | . | . | . | . | . | . | . | . | . | . | . | F | . | . | . | . | . | . | . | . |
| 21-225_75A7_HC | . | . | . | . | . | . | N | . | . | . | . | . | . | . | . | . | . | . | . | . | . | . | . | D | . | . |
| 21-225_75B1_HC | . | . | . | . | . | . | . | . | . | . | . | . | . | . | . | . | . | . | . | . | . | . | . | . | . | . |
| 21-225_76A6_HC | . | . | . | . | . | . | . | . | . | . | . | . | N | . | . | . | . | . | . | . | . | . | . | . | . | . |
| 21-225_76B9_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | M | G | W | M | H | P | N | - | - | - | S | G | N | T | G | Y | A | Q | K | F | Q | G | R | V |
| 21-225_76E8_HC | . | . | . | . | . | . | N | . | . | . | . | . | . | . | . | . | . | . | . | . | . | . | . | . | . | . |
| 21-225_76F3_HC | . | . | . | . | . | . | N | . | . | . | . | . | . | . | . | . | . | . | . | . | . | . | . | . | . | . |
| 21-225_76F5_HC | . | . | . | . | . | . | N | . | . | . | . | . | . | . | . | . | . | . | . | . | . | . | . | . | . | . |
| 21-225_76H1_HC | . | . | . | . | . | . | . | . | . | . | . | . | . | . | . | . | . | . | . | . | . | . | . | . | . | . |
| 21-225_77C10_HC | . | . | . | . | . | . | . | . | . | . | . | . | . | . | . | . | . | . | . | . | . | . | . | . | . | . |
| 21-225_77C7_HC | . | . | . | . | . | . | N | . | . | . | . | . | . | . | . | . | . | . | . | . | . | . | . | . | . | . |
| 21-225_77D11_HC | . | . | . | . | . | . | . | . | . | . | . | . | . | . | . | . | . | . | . | . | . | . | . | . | . | . |
| 21-225_77D12_HC | . | . | . | . | . | . | N | . | . | . | . | . | . | . | . | . | . | . | . | . | . | . | . | . | . | . |
| 21-225_77E1_HC | . | . | . | . | . | . | N | . | . | . | . | . | . | . | . | . | . | . | . | . | . | . | . | . | . | . |
| 21-225_78C1_HC | . | . | . | . | . | . | . | . | . | . | . | . | . | . | . | . | . | . | . | . | . | . | . | . | . | . |
| 21-225_78H12_HC | . | . | . | . | . | . | N | . | . | . | . | . | . | . | . | . | . | . | . | . | . | . | . | . | . | . |
| 21-225_79G10_HC | . | . | . | . | . | . | N | . | . | . | . | . | . | . | . | . | . | . | . | . | . | . | . | . | . | . |
| 21-225_79G6_HC | . | . | . | . | . | . | . | . | . | . | . | . | . | . | . | . | . | F | . | . | . | . | . | . | . | . |
| 21-225_80A2_HC | . | . | . | . | . | . | . | . | . | . | . | . | . | . | . | . | . | . | . | . | . | . | . | . | . | . |
| 21-225_80C12_HC | . | . | . | . | . | . | . | . | . | . | . | . | N | . | . | . | . | . | . | . | . | . | . | . | . | . |
| 21-225_80D3_HC | . | . | . | . | . | . | . | . | . | . | . | . | . | . | . | . | . | . | . | . | . | . | . | . | . | . |
| 21-225_80E9_HC | . | . | . | . | . | . | . | . | . | . | . | . | . | . | . | . | . | . | . | . | . | . | . | . | . | . |
| 21-225_81E5_HC | . | . | . | . | . | . | N | . | . | . | . | . | . | . | . | . | . | . | . | . | . | . | . | . | . | . |
| 21-225_81F9_HC | . | . | . | . | . | . | . | . | . | . | . | . | . | . | . | . | . | . | . | . | . | . | . | . | . | . |
| 21-225_82A5_HC | . | . | . | . | . | . | . | . | . | . | . | . | N | . | . | . | . | . | . | . | . | . | . | . | . | . |
| 21-225_82C12_HC | . | . | . | . | . | . | . | . | . | . | . | . | N | . | . | . | . | . | . | . | . | . | . | . | . | . |
| 21-225_82D9_HC | . | . | . | . | . | . | . | . | . | . | . | . | . | . | . | . | . | . | . | . | . | . | . | . | . | . |
| 21-225_82G5_HC | . | . | . | . | . | . | . | . | . | . | . | . | . | . | . | . | . | . | . | . | . | . | . | . | . | . |
| 21-225_83C2_HC | . | . | . | . | . | . | . | . | . | . | . | . | . | . | . | . | . | . | . | . | . | . | . | . | . | . |
| 21-225_83C5_HC | . | . | . | . | . | . | . | . | . | . | . | . | . | . | . | . | . | . | . | . | . | . | . | . | . | . |
| 21-225_83F3_HC | . | . | . | . | . | . | . | . | . | . | . | . | . | . | . | . | . | . | . | . | . | . | . | . | . | . |
| 21-225_83G3_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | M | G | W | M | H | P | N | - | - | - | S | G | N | T | G | Y | A | Q | K | F | Q | G | R | V |
| 21-225_83G7_HC | . | . | . | . | . | . | N | . | . | . | . | . | . | . | . | . | . | . | . | . | . | . | . | . | . | . |
| 21-225_84G7_HC | . | . | . | . | . | . | N | . | . | . | . | . | . | . | . | . | . | . | . | . | . | . | . | D | . | . |
| 21-225_85B5_HC | . | . | . | . | . | . | . | . | . | . | . | . | . | . | . | . | . | . | . | . | . | . | . | . | . | . |
| 21-225_85D11_HC | . | . | . | . | . | . | . | . | . | . | . | . | . | . | . | . | . | . | . | . | . | . | . | . | . | . |
| 21-225_85H2_HC | . | . | . | . | . | . | . | . | . | . | . | . | . | . | . | . | . | . | . | . | . | . | . | . | . | . |
| 21-225_86E9_HC | . | . | . | . | . | . | N | . | . | . | . | . | . | . | . | . | . | . | . | . | . | . | . | . | . | . |
| 21-225_87A10_HC | . | . | . | . | . | . | . | . | . | . | . | . | N | . | . | . | . | . | . | . | . | . | . | . | . | . |
| 21-225_88G2_HC | . | . | . | . | . | . | . | . | . | . | . | . | . | . | . | . | . | . | . | . | . | . | . | . | . | . |
| 21-225_90D9_HC | . | . | . | . | . | . | N | . | . | . | . | . | . | . | . | . | . | . | . | . | . | . | . | . | . | . |
| 21-225_91F1_HC | . | . | . | . | . | . | . | . | . | . | . | . | . | . | . | . | . | . | . | . | . | . | . | . | . | . |
| 21-225_91G8_HC | . | . | . | . | . | . | . | . | . | . | . | . | . | . | . | . | . | . | . | . | . | . | . | . | . | . |
| 21-225_94D2_HC | . | . | . | . | . | . | . | . | . | . | . | . | . | . | . | . | . | F | . | . | . | . | . | . | . | . |
| 21-225_94E11_HC | . | . | . | . | . | . | N | . | . | . | . | . | . | . | . | . | . | . | . | . | . | . | . | . | . | . |
| 21-225_95E12_HC | . | . | . | . | . | . | N | . | . | . | . | . | . | . | . | . | . | . | . | . | . | . | . | . | . | . |
| 21-225_95G4_HC | . | . | . | . | . | . | N | . | . | . | . | . | . | . | . | . | . | . | . | . | . | . | . | . | . | . |
| 21-225_95H4_HC | . | . | . | . | . | . | N | . | . | . | . | . | . | . | . | . | . | . | . | . | . | . | . | . | . | . |
| 21-225_96D5_HC | . | . | . | . | . | . | N | . | . | . | . | . | . | . | . | . | . | . | . | . | . | . | . | . | . | . |
| 21-225_96E2_HC | . | . | . | . | . | . | . | . | . | . | . | . | . | . | . | . | . | . | . | . | . | . | . | . | . | . |
| 21-225_96G1_HC | . | . | . | . | . | . | . | . | . | . | . | . | N | . | . | . | . | . | . | . | . | . | . | . | . | . |
| 21-225_97H4_HC | . | . | . | . | . | . | . | . | . | . | . | . | . | . | . | . | . | . | . | . | . | . | . | . | . | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | M | T | R | N | T | S | I | S | T | A | Y | M | E | L | S | S | L | R | S | E | D | T | A | V | Y |
| 21-225_147G6_HC | . | . | . | . | . | . | . | . | . | . | . | . | . | . | . | . | . | . | . | . | . | . | . | . | . | . |
| 21-225_152F4_HC | . | . | . | . | . | . | . | . | . | . | . | . | . | . | . | . | . | . | . | . | D | . | . | . | . | . |
| 21-225_170F6_HC | . | . | . | . | H | . | . | . | . | . | . | . | . | . | . | . | . | . | . | . | . | . | . | . | . | . |
| 21-225_174G5_HC | . | . | . | . | . | . | . | . | . | . | . | . | . | . | . | . | . | . | . | . | . | . | . | . | . | . |
| 21-225_224H5_HC | . | . | . | . | . | . | . | . | . | . | . | . | . | . | . | . | . | . | . | . | . | . | . | . | . | . |
| 21-225_225F4_HC | . | . | . | . | . | . | . | . | . | . | V | . | . | . | . | N | . | . | . | . | . | . | . | . | . | . |
| 21-225_227G9_HC | . | . | . | . | . | . | . | . | . | . | . | . | . | . | . | . | . | . | . | . | . | . | . | . | . | . |
| 21-225_25A3_HC | . | . | . | W | . | . | . | . | . | . | . | N | . | . | . | . | . | . | . | . | . | . | . | . | . | . |
| 21-225_26A9_HC | . | . | . | W | . | M | . | . | . | . | . | . | . | . | . | . | . | . | . | . | . | . | . | . | . | . |
| 21-225_57A11_HC | . | . | . | . | D | . | . | . | . | . | . | . | . | . | . | . | . | . | . | . | . | . | . | . | . | . |
| 21-225_57F12_HC | . | . | . | . | . | . | . | . | . | . | . | . | . | . | . | . | . | . | . | . | . | . | . | . | . | . |
| 21-225_74A5_HC | . | . | . | . | . | . | . | . | . | . | . | . | . | . | . | . | . | . | . | . | . | . | . | . | . | . |
| 21-225_74A7_HC | . | . | . | . | . | . | . | T | . | . | . | H | . | . | . | . | . | . | . | . | . | . | . | . | . | . |
| 21-225_74B10_HC | . | . | . | . | . | . | . | . | . | . | . | . | . | . | . | . | . | . | . | . | . | . | . | . | . | . |
| 21-225_74B5_HC | . | . | . | . | . | . | . | T | . | . | . | H | . | . | . | . | . | . | . | . | . | . | . | . | . | . |
| 21-225_74B6_HC | . | . | . | . | . | . | . | . | . | . | . | . | . | . | . | N | . | . | . | . | . | . | . | . | . | . |
| 21-225_74B7_HC | . | . | . | . | . | . | . | . | . | . | . | . | . | . | . | . | . | . | . | . | . | . | . | . | . | . |
| 21-225_74B8_HC | . | L | . | . | . | . | . | . | . | . | . | . | . | . | . | . | . | . | . | . | . | . | . | . | . | . |
| 21-225_74C1_HC | . | . | . | . | . | . | . | . | . | . | . | . | . | . | . | . | . | . | . | . | . | . | . | . | . | . |
| 21-225_74C10_HC | . | . | . | . | . | . | . | . | . | . | . | . | . | . | . | . | . | . | . | . | . | . | . | . | . | . |
| 21-225_74C12_HC | . | . | . | . | . | . | . | . | . | . | . | . | . | . | . | . | . | . | . | . | . | . | . | . | . | . |
| 21-225_74G3_HC | . | . | . | . | . | . | . | . | . | . | . | . | . | . | . | . | . | . | . | . | . | . | . | . | . | . |
| 21-225_74H2_HC | . | . | . | . | . | . | . | . | . | . | . | . | . | . | . | . | . | . | . | . | . | . | . | . | . | . |
| 21-225_75A7_HC | . | . | . | . | . | . | . | . | . | . | . | . | . | . | . | . | . | . | . | . | . | . | . | . | . | . |
| 21-225_75B1_HC | . | . | . | . | . | . | . | . | . | . | . | . | . | . | . | . | . | . | . | . | . | . | . | . | . | . |
| 21-225_76A6_HC | . | . | . | . | . | . | . | . | . | . | . | . | . | . | . | N | . | . | . | . | . | . | . | . | . | . |
| 21-225_76B9_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | M | T | R | N | T | S | I | S | T | A | Y | M | E | L | S | S | L | R | S | E | D | T | A | V | Y |
| 21-225_76E8_HC | . | . | . | . | . | . | . | . | . | . | . | . | . | . | . | . | . | . | . | . | . | . | . | . | . | . |
| 21-225_76F3_HC | . | . | . | . | . | . | . | T | . | . | . | H | . | . | . | . | . | . | . | . | . | . | . | . | . | . |
| 21-225_76F5_HC | . | . | . | . | . | . | . | . | . | . | . | . | . | . | . | . | . | . | . | . | . | . | . | . | . | . |
| 21-225_76H1_HC | . | . | . | . | . | . | . | . | . | . | . | . | . | . | . | . | . | . | . | . | . | . | . | . | . | . |
| 21-225_77C10_HC | . | . | . | . | . | . | . | . | . | . | . | . | . | . | . | . | . | . | . | . | . | . | . | . | . | . |
| 21-225_77C7_HC | . | . | . | . | . | . | . | . | . | . | . | . | . | . | . | . | . | . | . | . | . | . | . | . | . | . |
| 21-225_77D11_HC | . | . | . | . | . | . | . | . | . | . | . | . | . | . | . | . | . | . | . | . | . | . | . | . | . | . |
| 21-225_77D12_HC | . | . | . | . | . | . | . | . | N | . | . | . | . | . | . | . | . | . | . | . | . | . | . | . | . | . |
| 21-225_77E1_HC | . | . | . | . | . | . | . | . | . | . | . | . | . | . | . | . | . | . | . | . | . | . | . | . | . | . |
| 21-225_78C1_HC | . | . | . | . | . | . | . | . | . | . | . | . | . | . | . | . | . | . | . | . | . | . | . | . | . | . |
| 21-225_78H12_HC | . | . | . | . | . | . | . | . | . | . | . | . | . | . | . | . | . | . | . | . | . | . | . | . | . | . |
| 21-225_79G10_HC | . | . | . | . | . | . | . | T | . | . | . | H | . | . | . | . | . | . | . | . | . | . | . | . | . | . |
| 21-225_79G6_HC | . | . | . | . | . | . | . | . | . | . | . | . | . | . | . | . | . | . | . | . | . | . | . | . | . | . |
| 21-225_80A2_HC | . | . | . | . | . | . | . | . | . | . | . | . | . | . | . | . | . | . | . | . | . | . | . | . | . | . |
| 21-225_80C12_HC | . | . | . | . | . | . | . | . | . | . | . | . | . | . | . | N | . | . | . | . | . | . | . | . | . | . |
| 21-225_80D3_HC | . | . | . | . | . | . | . | . | . | . | . | . | . | . | . | . | . | . | . | . | . | . | . | . | . | . |
| 21-225_80E9_HC | . | . | . | . | . | . | . | . | . | . | . | . | . | . | . | . | . | . | . | . | . | . | . | . | . | . |
| 21-225_81E5_HC | . | . | . | . | . | . | . | T | . | . | . | H | . | . | . | . | . | . | . | . | . | . | . | . | . | . |
| 21-225_81F9_HC | . | . | . | . | . | . | . | . | . | . | . | . | . | . | . | . | . | . | . | . | . | . | . | . | . | . |
| 21-225_82A5_HC | . | . | . | . | . | . | . | . | . | . | . | . | . | . | . | N | . | . | . | . | . | . | . | . | . | . |
| 21-225_82C12_HC | . | . | . | . | . | . | . | . | . | . | . | . | . | . | . | N | . | . | . | . | . | . | . | . | . | . |
| 21-225_82D9_HC | . | . | . | . | . | . | . | . | . | . | . | . | . | . | . | . | . | . | . | . | . | . | . | . | . | . |
| 21-225_82G5_HC | . | . | . | . | . | . | . | . | . | . | . | . | . | . | . | . | . | . | . | . | . | . | . | . | . | . |
| 21-225_83C2_HC | . | . | . | . | . | . | . | . | . | . | . | . | . | . | . | . | . | . | . | . | . | . | . | . | . | . |
| 21-225_83C5_HC | . | . | . | . | . | . | . | . | . | . | . | . | . | . | . | . | . | . | . | . | . | . | . | . | . | . |
| 21-225_83F3_HC | . | . | . | . | . | . | . | . | . | . | . | . | . | . | . | . | . | . | . | . | . | . | . | . | . | . |
| 21-225_83G3_HC | . | . | . | . | . | . | . | . | . | . | . | . | . | . | . | . | . | . | . | . | . | . | . | . | . | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | M | T | R | N | T | S | I | S | T | A | Y | M | E | L | S | S | L | R | S | E | D | T | A | V | Y |
| 21-225_83G7_HC | . | . | . | . | . | . | . | . | . | . | . | . | . | . | . | . | . | . | . | . | . | . | . | . | . | . |
| 21-225_84G7_HC | . | . | . | . | . | . | . | . | . | . | . | . | . | . | . | . | . | . | . | . | . | . | . | . | . | . |
| 21-225_85B5_HC | . | . | . | . | . | . | . | . | . | . | . | . | . | . | . | . | . | . | . | . | . | . | . | . | . | . |
| 21-225_85D11_HC | . | . | . | . | . | . | . | . | . | . | . | . | . | . | . | . | . | . | . | . | . | . | . | . | . | . |
| 21-225_85H2_HC | . | L | . | . | . | . | . | . | . | . | . | . | . | . | . | . | . | . | . | . | . | . | . | . | . | . |
| 21-225_86E9_HC | . | . | . | . | S | . | . | T | . | . | . | H | . | . | . | . | . | . | . | . | . | . | . | . | . | . |
| 21-225_87A10_HC | . | . | . | . | . | . | . | . | . | . | . | . | . | . | . | N | . | . | . | . | . | . | . | . | . | . |
| 21-225_88G2_HC | . | . | . | . | . | . | . | . | . | . | . | . | . | . | . | . | . | . | . | . | . | . | . | . | . | . |
| 21-225_90D9_HC | . | . | . | . | . | . | . | . | . | . | . | . | . | . | . | . | . | . | . | . | . | . | . | . | . | . |
| 21-225_91F1_HC | . | . | . | . | . | . | . | . | . | . | . | . | . | . | . | . | . | . | . | . | . | . | . | . | . | . |
| 21-225_91G8_HC | . | . | . | . | . | . | . | . | . | . | . | . | . | . | . | . | . | . | . | . | . | . | . | . | . | . |
| 21-225_94D2_HC | . | . | . | . | . | . | . | . | . | . | . | . | . | . | . | . | . | . | . | . | . | . | . | . | . | . |
| 21-225_94E11_HC | . | . | . | . | . | . | . | T | . | . | . | H | . | . | . | . | . | . | . | . | . | . | . | . | . | . |
| 21-225_95E12_HC | . | . | . | . | . | . | . | . | . | . | . | . | . | . | . | . | . | . | . | . | . | . | . | . | . | . |
| 21-225_95G4_HC | . | . | . | . | . | . | . | T | . | . | . | H | . | . | . | . | . | . | . | . | . | . | . | . | . | . |
| 21-225_95H4_HC | . | . | . | . | . | . | . | T | . | . | . | H | . | . | . | . | . | . | . | . | . | . | . | . | . | . |
| 21-225_96D5_HC | . | . | . | . | S | . | . | T | . | . | . | H | . | . | . | . | . | . | . | . | . | . | . | . | . | . |
| 21-225_96E2_HC | . | L | . | . | . | . | . | . | . | . | . | . | . | . | . | . | . | . | . | . | . | . | . | . | . | . |
| 21-225_96G1_HC | . | . | . | . | . | . | . | . | . | . | . | . | . | . | . | N | . | . | . | . | . | . | . | . | . | . |
| 21-225_97H4_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | I | S | S | G | W | Y | - | - | - | - | - | - | - | - | - | - | - | - | - | - | - | - | - |
| 21-225_147G6_HC | . | . | . | Y | . | . | . | . | . | . | . | . | . | . | . | . | . | . | . | . | . | . | . | . | . | . |
| 21-225_152F4_HC | . | . | . | Y | . | . | . | . | . | . | . | . | . | . | . | . | . | . | . | . | . | . | . | . | . | . |
| 21-225_170F6_HC | . | . | . | . | . | . | . | . | . | . | . | . | . | . | . | . | . | . | . | . | . | . | . | . | . | . |
| 21-225_174G5_HC | . | . | . | V | . | . | . | . | . | . | . | . | . | . | . | . | . | . | . | . | . | . | . | . | . | . |
| 21-225_224H5_HC | . | . | . | S | . | . | . | . | N | . | . | . | . | . | . | . | . | . | . | . | . | . | . | . | . | . |
| 21-225_225F4_HC | . | . | . | S | . | . | . | . | . | . | . | . | . | . | . | . | . | . | . | . | . | . | . | . | . | . |
| 21-225_227G9_HC | . | . | . | L | . | . | . | . | . | . | . | . | . | . | . | . | . | . | . | . | . | . | . | . | . | . |
| 21-225_25A3_HC | . | . | . | S | . | . | . | . | . | . | . | . | . | . | . | . | . | . | . | . | . | . | . | . | . | . |
| 21-225_26A9_HC | . | . | . | S | . | . | . | . | . | . | . | . | . | . | . | . | . | . | . | . | . | . | . | . | . | . |
| 21-225_57A11_HC | . | . | . | . | . | . | . | . | N | . | . | . | . | . | . | . | . | . | . | . | . | . | . | . | . | . |
| 21-225_57F12_HC | . | . | . | . | . | . | . | . | . | . | . | . | . | . | . | . | . | . | . | . | . | . | . | . | . | . |
| 21-225_74A5_HC | . | . | . | . | . | . | . | . | . | . | . | . | . | . | . | . | . | . | . | . | . | . | . | . | . | . |
| 21-225_74A7_HC | . | . | . | V | . | . | . | . | N | . | . | . | . | . | . | . | . | . | . | . | . | . | . | . | . | . |
| 21-225_74B10_HC | . | . | . | V | . | . | . | . | . | . | . | . | . | . | . | . | . | . | . | . | . | . | . | . | . | . |
| 21-225_74B5_HC | . | . | . | V | . | . | . | . | N | . | . | . | . | . | . | . | . | . | . | . | . | . | . | . | . | . |
| 21-225_74B6_HC | . | . | . | . | . | . | . | . | . | . | . | . | . | . | . | . | . | . | . | . | . | . | . | . | . | . |
| 21-225_74B7_HC | . | . | . | V | . | . | . | . | H | . | . | . | . | . | . | . | . | . | . | . | . | . | . | . | . | . |
| 21-225_74B8_HC | . | . | . | V | . | . | . | . | . | . | . | . | . | . | . | . | . | . | . | . | . | . | . | . | . | . |
| 21-225_74C1_HC | . | . | . | V | . | . | . | . | . | . | . | . | . | . | . | . | . | . | . | . | . | . | . | . | . | . |
| 21-225_74C10_HC | . | . | . | . | . | . | . | . | . | . | . | . | . | . | . | . | . | . | . | . | . | . | . | . | . | . |
| 21-225_74C12_HC | . | . | . | . | . | . | . | . | . | . | . | . | . | . | . | . | . | . | . | . | . | . | . | . | . | . |
| 21-225_74G3_HC | . | . | . | . | . | . | . | . | . | . | . | . | . | . | . | . | . | . | . | . | . | . | . | . | . | . |
| 21-225_74H2_HC | . | . | . | V | . | . | . | . | . | . | . | . | . | . | . | . | . | . | . | . | . | . | . | . | . | . |
| 21-225_75A7_HC | . | . | . | V | . | . | . | . | H | . | . | . | . | . | . | . | . | . | . | . | . | . | . | . | . | . |
| 21-225_75B1_HC | . | . | . | . | . | . | . | . | . | . | . | . | . | . | . | . | . | . | . | . | . | . | . | . | . | . |
| 21-225_76A6_HC | . | . | . | . | . | . | . | . | . | . | . | . | . | . | . | . | . | . | . | . | . | . | . | . | . | . |
| 21-225_76B9_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | I | S | S | G | W | Y | - | - | - | - | - | - | - | - | - | - | - | - | - | - | - | - | - |
| 21-225_76E8_HC | . | . | . | V | . | . | . | . | H | . | . | . | . | . | . | . | . | . | . | . | . | . | . | . | . | . |
| 21-225_76F3_HC | . | . | . | V | . | . | . | . | N | . | . | . | . | . | . | . | . | . | . | . | . | . | . | . | . | . |
| 21-225_76F5_HC | . | . | . | S | . | . | . | . | . | . | . | . | . | . | . | . | . | . | . | . | . | . | . | . | . | . |
| 21-225_76H1_HC | . | . | . | . | . | . | . | . | . | . | . | . | . | . | . | . | . | . | . | . | . | . | . | . | . | . |
| 21-225_77C10_HC | . | . | . | . | . | . | . | . | . | . | . | . | . | . | . | . | . | . | . | . | . | . | . | . | . | . |
| 21-225_77C7_HC | . | . | . | V | . | . | . | . | H | . | . | . | . | . | . | . | . | . | . | . | . | . | . | . | . | . |
| 21-225_77D11_HC | . | . | . | . | . | . | . | . | . | . | . | . | . | . | . | . | . | . | . | . | . | . | . | . | . | . |
| 21-225_77D12_HC | . | . | . | . | . | . | . | . | . | . | . | . | . | . | . | . | . | . | . | . | . | . | . | . | . | . |
| 21-225_77E1_HC | . | . | . | V | . | . | . | . | H | . | . | . | . | . | . | . | . | . | . | . | . | . | . | . | . | . |
| 21-225_78C1_HC | . | . | . | . | . | . | . | . | . | . | . | . | . | . | . | . | . | . | . | . | . | . | . | . | . | . |
| 21-225_78H12_HC | . | . | . | V | . | . | . | . | H | . | . | . | . | . | . | . | . | . | . | . | . | . | . | . | . | . |
| 21-225_79G10_HC | . | . | . | V | . | . | . | . | N | . | . | . | . | . | . | . | . | . | . | . | . | . | . | . | . | . |
| 21-225_79G6_HC | . | . | . | V | . | . | . | . | . | . | . | . | . | . | . | . | . | . | . | . | . | . | . | . | . | . |
| 21-225_80A2_HC | . | . | . | . | . | . | . | . | . | . | . | . | . | . | . | . | . | . | . | . | . | . | . | . | . | . |
| 21-225_80C12_HC | . | . | . | . | . | . | . | . | . | . | . | . | . | . | . | . | . | . | . | . | . | . | . | . | . | . |
| 21-225_80D3_HC | . | . | . | . | . | . | . | . | . | . | . | . | . | . | . | . | . | . | . | . | . | . | . | . | . | . |
| 21-225_80E9_HC | . | . | . | . | . | . | . | . | . | . | . | . | . | . | . | . | . | . | . | . | . | . | . | . | . | . |
| 21-225_81E5_HC | . | . | . | V | . | . | . | . | N | . | . | . | . | . | . | . | . | . | . | . | . | . | . | . | . | . |
| 21-225_81F9_HC | . | . | . | . | . | . | . | . | . | . | . | . | . | . | . | . | . | . | . | . | . | . | . | . | . | . |
| 21-225_82A5_HC | . | . | . | . | . | . | . | . | . | . | . | . | . | . | . | . | . | . | . | . | . | . | . | . | . | . |
| 21-225_82C12_HC | . | . | . | . | . | . | . | . | . | . | . | . | . | . | . | . | . | . | . | . | . | . | . | . | . | . |
| 21-225_82D9_HC | . | . | . | . | . | . | . | . | . | . | . | . | . | . | . | . | . | . | . | . | . | . | . | . | . | . |
| 21-225_82G5_HC | . | . | . | . | . | . | . | . | . | . | . | . | . | . | . | . | . | . | . | . | . | . | . | . | . | . |
| 21-225_83C2_HC | . | . | . | . | . | . | . | . | . | . | . | . | . | . | . | . | . | . | . | . | . | . | . | . | . | . |
| 21-225_83C5_HC | . | . | . | . | . | . | . | . | . | . | . | . | . | . | . | . | . | . | . | . | . | . | . | . | . | . |
| 21-225_83F3_HC | . | . | . | . | . | . | . | . | . | . | . | . | . | . | . | . | . | . | . | . | . | . | . | . | . | . |
| 21-225_83G3_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | I | S | S | G | W | Y | - | - | - | - | - | - | - | - | - | - | - | - | - | - | - | - | - |
| 21-225_83G7_HC | . | . | . | V | . | . | . | . | H | . | . | . | . | . | . | . | . | . | . | . | . | . | . | . | . | . |
| 21-225_84G7_HC | . | . | . | V | . | . | . | . | H | . | . | . | . | . | . | . | . | . | . | . | . | . | . | . | . | . |
| 21-225_85B5_HC | . | . | . | . | . | . | . | . | . | . | . | . | . | . | . | . | . | . | . | . | . | . | . | . | . | . |
| 21-225_85D11_HC | . | . | . | . | . | . | . | . | . | . | . | . | . | . | . | . | . | . | . | . | . | . | . | . | . | . |
| 21-225_85H2_HC | . | . | . | V | . | . | . | . | . | . | . | . | . | . | . | . | . | . | . | . | . | . | . | . | . | . |
| 21-225_86E9_HC | . | . | . | V | . | . | . | . | S | . | . | . | . | . | . | . | . | . | . | . | . | . | . | . | . | . |
| 21-225_87A10_HC | . | . | . | . | . | . | . | . | . | . | . | . | . | . | . | . | . | . | . | . | . | . | . | . | . | . |
| 21-225_88G2_HC | . | . | . | . | . | . | . | . | . | . | . | . | . | . | . | . | . | . | . | . | . | . | . | . | . | . |
| 21-225_90D9_HC | . | . | . | V | . | . | . | . | H | . | . | . | . | . | . | . | . | . | . | . | . | . | . | . | . | . |
| 21-225_91F1_HC | . | . | . | . | . | . | . | . | . | . | . | . | . | . | . | . | . | . | . | . | . | . | . | . | . | . |
| 21-225_91G8_HC | . | . | . | . | . | . | . | . | . | . | . | . | . | . | . | . | . | . | . | . | . | . | . | . | . | . |
| 21-225_94D2_HC | . | . | . | V | . | . | . | . | . | . | . | . | . | . | . | . | . | . | . | . | . | . | . | . | . | . |
| 21-225_94E11_HC | . | . | . | V | . | . | . | . | K | . | . | . | . | . | . | . | . | . | . | . | . | . | . | . | . | . |
| 21-225_95E12_HC | . | . | . | . | . | . | . | . | . | . | . | . | . | . | . | . | . | . | . | . | . | . | . | . | . | . |
| 21-225_95G4_HC | . | . | . | V | . | . | . | . | N | . | . | . | . | . | . | . | . | . | . | . | . | . | . | . | . | . |
| 21-225_95H4_HC | . | . | . | V | . | . | . | . | N | . | . | . | . | . | . | . | . | . | . | . | . | . | . | . | . | . |
| 21-225_96D5_HC | . | . | . | V | . | . | . | . | S | . | . | . | . | . | . | . | . | . | . | . | . | . | . | . | . | . |
| 21-225_96E2_HC | . | . | . | V | . | . | . | . | . | . | . | . | . | . | . | . | . | . | . | . | . | . | . | . | . | . |
| 21-225_96G1_HC | . | . | . | . | . | . | . | . | . | . | . | . | . | . | . | . | . | . | . | . | . | . | . | . | . | . |
| 21-225_97H4_HC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | W | F | D | P | W | G | Q | G | T | L | V | T | V | S | S |
| 21-225_147G6_HC | . | . | . | . | . | . | . | . | . | . | . | . | . | . | . | . | . | . | . |
| 21-225_152F4_HC | . | . | . | . | . | . | . | . | . | . | . | . | . | . | . | . | . | . | . |
| 21-225_170F6_HC | . | . | . | . | . | . | . | . | . | . | . | . | . | . | . | . | . | . | . |
| 21-225_174G5_HC | . | . | . | . | . | . | . | . | . | . | . | . | . | . | . | . | . | . | . |
| 21-225_224H5_HC | . | . | . | . | . | . | . | . | . | . | . | . | . | . | . | . | . | . | . |
| 21-225_225F4_HC | . | . | . | . | . | . | . | . | . | . | . | . | . | . | . | . | . | . | . |
| 21-225_227G9_HC | . | . | . | . | . | . | . | . | . | . | . | . | . | . | . | . | . | . | . |
| 21-225_25A3_HC | . | . | . | . | . | . | . | . | . | . | . | . | . | . | . | . | . | . | . |
| 21-225_26A9_HC | . | . | . | . | . | . | . | . | . | . | . | . | . | . | . | . | . | . | . |
| 21-225_57A11_HC | . | . | . | . | . | . | . | . | . | . | . | . | . | . | . | . | . | . | . |
| 21-225_57F12_HC | . | . | . | . | R | . | . | . | . | . | . | . | . | . | . | . | . | . | . |
| 21-225_74A5_HC | . | . | . | . | . | . | . | . | . | . | . | . | . | . | . | . | . | . | . |
| 21-225_74A7_HC | . | . | . | . | . | . | . | . | . | . | . | . | . | . | . | . | . | . | . |
| 21-225_74B10_HC | . | . | . | . | . | . | . | . | . | . | . | . | . | . | . | . | . | . | . |
| 21-225_74B5_HC | . | . | . | . | . | . | . | . | . | . | . | . | . | . | . | . | . | . | . |
| 21-225_74B6_HC | . | . | . | . | . | . | . | . | . | . | . | . | . | . | . | . | . | . | L |
| 21-225_74B7_HC | . | . | . | . | . | . | . | . | . | . | . | . | . | . | . | . | . | A | . |
| 21-225_74B8_HC | . | . | . | . | . | . | . | . | . | . | . | . | . | . | . | . | . | . | . |
| 21-225_74C1_HC | . | . | . | . | . | . | . | . | . | . | . | . | . | . | . | . | . | . | . |
| 21-225_74C10_HC | . | . | . | . | . | . | . | . | . | . | . | . | . | . | . | . | . | . | . |
| 21-225_74C12_HC | . | . | . | . | . | . | . | . | . | . | . | . | . | . | . | . | . | . | . |
| 21-225_74G3_HC | . | . | . | . | . | . | . | . | . | . | . | . | . | . | . | . | . | . | . |
| 21-225_74H2_HC | . | . | . | . | . | . | . | . | . | . | . | . | . | . | . | . | . | . | . |
| 21-225_75A7_HC | . | . | . | . | . | . | . | . | . | . | . | . | . | . | . | . | . | A | . |
| 21-225_75B1_HC | . | . | . | . | . | . | . | . | . | . | . | . | . | . | . | . | . | . | . |
| 21-225_76A6_HC | . | . | . | . | . | . | . | . | . | . | . | . | . | . | . | . | . | . | L |
| 21-225_76B9_HC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | W | F | D | P | W | G | Q | G | T | L | V | T | V | S | S |
| 21-225_76E8_HC | . | . | . | . | . | . | . | . | . | . | . | . | . | . | . | . | . | A | . |
| 21-225_76F3_HC | . | . | . | . | . | . | . | . | . | . | . | . | . | . | . | . | . | . | . |
| 21-225_76F5_HC | . | . | . | . | . | . | . | . | . | . | . | . | . | . | . | . | . | . | . |
| 21-225_76H1_HC | . | . | . | . | . | . | . | . | . | . | . | . | . | . | . | . | . | . | . |
| 21-225_77C10_HC | . | . | . | . | . | . | . | . | . | . | . | . | . | . | . | . | . | . | . |
| 21-225_77C7_HC | . | . | . | . | . | . | . | . | . | . | . | . | . | . | . | . | . | A | . |
| 21-225_77D11_HC | . | . | . | . | . | . | . | . | . | . | . | . | . | . | . | . | . | . | . |
| 21-225_77D12_HC | . | . | . | . | . | . | . | . | . | . | . | . | . | . | . | . | . | . | . |
| 21-225_77E1_HC | . | . | . | . | . | . | . | . | . | . | . | . | . | . | . | . | . | A | . |
| 21-225_78C1_HC | . | . | . | . | . | . | . | . | . | . | . | . | . | . | . | . | . | . | . |
| 21-225_78H12_HC | . | . | . | . | . | . | . | . | . | . | . | . | . | . | . | . | . | A | . |
| 21-225_79G10_HC | . | . | . | . | . | . | . | . | . | . | . | . | . | . | . | . | . | . | . |
| 21-225_79G6_HC | . | . | . | . | . | . | . | . | . | . | . | . | . | . | . | . | . | . | . |
| 21-225_80A2_HC | . | . | . | . | . | . | . | . | . | . | . | . | . | . | . | . | . | . | . |
| 21-225_80C12_HC | . | . | . | . | . | . | . | . | . | . | . | . | . | . | . | . | . | . | L |
| 21-225_80D3_HC | . | . | . | . | . | . | . | . | . | . | . | . | . | . | . | . | . | . | . |
| 21-225_80E9_HC | . | . | . | . | . | . | . | . | . | . | . | . | . | . | . | . | . | . | . |
| 21-225_81E5_HC | . | . | . | . | . | . | . | . | . | . | . | . | . | . | . | . | . | . | . |
| 21-225_81F9_HC | . | . | . | . | . | . | . | . | . | . | . | . | . | . | . | . | . | . | . |
| 21-225_82A5_HC | . | . | . | . | . | . | . | . | . | . | . | . | . | . | . | . | . | . | L |
| 21-225_82C12_HC | . | . | . | . | . | . | . | . | . | . | . | . | . | . | . | . | . | . | L |
| 21-225_82D9_HC | . | . | . | . | . | . | . | . | . | . | . | . | . | . | . | . | . | . | . |
| 21-225_82G5_HC | . | . | . | . | . | . | . | . | . | . | . | . | . | . | . | . | . | . | . |
| 21-225_83C2_HC | . | . | . | . | . | . | . | . | . | . | . | . | . | . | . | . | . | . | . |
| 21-225_83C5_HC | . | . | . | . | . | . | . | . | . | . | . | . | . | . | . | . | . | . | . |
| 21-225_83F3_HC | . | . | . | . | . | . | . | . | . | . | . | . | . | . | . | . | . | . | . |
| 21-225_83G3_HC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | W | F | D | P | W | G | Q | G | T | L | V | T | V | S | S |
| 21-225_83G7_HC | . | . | . | . | . | . | . | . | . | . | . | . | . | . | . | . | . | A | . |
| 21-225_84G7_HC | . | . | . | . | . | . | . | . | . | . | . | . | . | . | . | . | . | A | . |
| 21-225_85B5_HC | . | . | . | . | . | . | . | . | . | . | . | . | . | . | . | . | . | . | . |
| 21-225_85D11_HC | . | . | . | . | . | . | . | . | . | . | . | . | . | . | . | . | . | . | . |
| 21-225_85H2_HC | . | . | . | . | . | . | . | . | . | . | . | . | . | . | . | . | . | . | . |
| 21-225_86E9_HC | . | . | . | . | . | . | . | . | . | . | . | . | . | . | . | . | . | . | . |
| 21-225_87A10_HC | . | . | . | . | . | . | . | . | . | . | . | . | . | . | . | . | . | . | L |
| 21-225_88G2_HC | . | . | . | . | . | . | . | . | . | . | . | . | . | . | . | . | . | . | . |
| 21-225_90D9_HC | . | . | . | . | . | . | . | . | . | . | . | . | . | . | . | . | . | A | . |
| 21-225_91F1_HC | . | . | . | . | . | . | . | . | . | . | . | . | . | . | . | . | . | . | . |
| 21-225_91G8_HC | . | . | . | . | . | . | . | . | . | . | . | . | . | . | . | . | . | . | . |
| 21-225_94D2_HC | . | . | . | . | . | . | . | . | . | . | . | . | . | . | . | . | . | . | . |
| 21-225_94E11_HC | . | . | . | . | . | . | . | . | . | . | . | . | . | . | . | . | . | . | . |
| 21-225_95E12_HC | . | . | . | . | . | . | . | . | . | . | . | . | . | . | . | . | . | . | . |
| 21-225_95G4_HC | . | . | . | . | . | . | . | . | . | . | . | . | . | . | . | . | . | . | . |
| 21-225_95H4_HC | . | . | . | . | . | . | . | . | . | . | . | . | . | . | . | . | . | . | . |
| 21-225_96D5_HC | . | . | . | . | . | . | . | . | . | . | . | . | . | . | . | . | . | . | . |
| 21-225_96E2_HC | . | . | . | . | . | . | . | . | . | . | . | . | . | . | . | . | . | . | . |
| 21-225_96G1_HC | . | . | . | . | . | . | . | . | . | . | . | . | . | . | . | . | . | . | L |
| 21-225_97H4_HC | . | . | . | . | . | . | . | . | . | . | . | . | . | . | . | . | . | . | . |

FIGURE 57

| Reference # | 139 | 140 | 141 | 142 | 143 | 144 | 145 | 146 | 147 | 148 | 149 |
|---|---|---|---|---|---|---|---|---|---|---|---|
| | H_FR4 | | | | | | | | | | |
| CONSENSUS | W | G | Q | G | T | L | V | T | V | S | S |
| 21-225_147G6_HC | . | . | . | . | . | . | . | . | . | . | . |
| 21-225_152F4_HC | . | . | . | . | . | . | . | . | . | . | . |
| 21-225_170F6_HC | . | . | . | . | . | . | . | . | . | . | . |
| 21-225_174G5_HC | . | . | . | . | . | . | . | . | . | . | . |
| 21-225_224H5_HC | . | . | . | . | . | . | . | . | . | . | . |
| 21-225_225F4_HC | . | . | . | . | . | . | . | . | . | . | . |
| 21-225_227G9_HC | . | . | . | . | . | . | . | . | . | . | . |
| 21-225_25A3_HC | . | . | . | . | . | . | . | . | . | . | . |
| 21-225_26A9_HC | . | . | . | . | . | . | . | . | . | . | . |
| 21-225_57A11_HC | . | . | . | . | . | . | . | . | . | . | . |
| 21-225_57F12_HC | . | . | . | . | . | . | . | . | . | . | . |
| 21-225_74A5_HC | . | . | . | . | . | . | . | . | . | . | . |
| 21-225_74A7_HC | . | . | . | . | . | . | . | . | . | . | . |
| 21-225_74B10_HC | . | . | . | . | . | . | . | . | . | . | . |
| 21-225_74B5_HC | . | . | . | . | . | . | . | . | . | . | . |
| 21-225_74B6_HC | . | . | . | . | . | . | . | . | . | . | L |
| 21-225_74B7_HC | . | . | . | . | . | . | . | . | . | A | . |
| 21-225_74B8_HC | . | . | . | . | . | . | . | . | . | . | . |
| 21-225_74C1_HC | . | . | . | . | . | . | . | . | . | . | . |
| 21-225_74C10_HC | . | . | . | . | . | . | . | . | . | . | . |
| 21-225_74C12_HC | . | . | . | . | . | . | . | . | . | . | . |
| 21-225_74G3_HC | . | . | . | . | . | . | . | . | . | . | . |
| 21-225_74H2_HC | . | . | . | . | . | . | . | . | . | . | . |
| 21-225_75A7_HC | . | . | . | . | . | . | . | . | . | A | . |
| 21-225_75B1_HC | . | . | . | . | . | . | . | . | . | . | . |
| 21-225_76A6_HC | . | . | . | . | . | . | . | . | . | . | L |
| 21-225_76B9_HC | . | . | . | . | . | . | . | . | . | . | . |

FIGURE 57

| Reference # | 139 | 140 | 141 | 142 | 143 | 144 | 145 | 146 | 147 | 148 | 149 |
|---|---|---|---|---|---|---|---|---|---|---|---|
| | H_FR4 | | | | | | | | | | |
| CONSENSUS | W | G | Q | G | T | L | V | T | V | S | S |
| 21-225_76E8_HC | . | . | . | . | . | . | . | . | . | A | . |
| 21-225_76F3_HC | . | . | . | . | . | . | . | . | . | . | . |
| 21-225_76F5_HC | . | . | . | . | . | . | . | . | . | . | . |
| 21-225_76H1_HC | . | . | . | . | . | . | . | . | . | . | . |
| 21-225_77C10_HC | . | . | . | . | . | . | . | . | . | . | . |
| 21-225_77C7_HC | . | . | . | . | . | . | . | . | . | A | . |
| 21-225_77D11_HC | . | . | . | . | . | . | . | . | . | . | . |
| 21-225_77D12_HC | . | . | . | . | . | . | . | . | . | . | . |
| 21-225_77E1_HC | . | . | . | . | . | . | . | . | . | A | . |
| 21-225_78C1_HC | . | . | . | . | . | . | . | . | . | . | . |
| 21-225_78H12_HC | . | . | . | . | . | . | . | . | . | A | . |
| 21-225_79G10_HC | . | . | . | . | . | . | . | . | . | . | . |
| 21-225_79G6_HC | . | . | . | . | . | . | . | . | . | . | . |
| 21-225_80A2_HC | . | . | . | . | . | . | . | . | . | . | . |
| 21-225_80C12_HC | . | . | . | . | . | . | . | . | . | . | L |
| 21-225_80D3_HC | . | . | . | . | . | . | . | . | . | . | . |
| 21-225_80E9_HC | . | . | . | . | . | . | . | . | . | . | . |
| 21-225_81E5_HC | . | . | . | . | . | . | . | . | . | . | . |
| 21-225_81F9_HC | . | . | . | . | . | . | . | . | . | . | . |
| 21-225_82A5_HC | . | . | . | . | . | . | . | . | . | . | L |
| 21-225_82C12_HC | . | . | . | . | . | . | . | . | . | . | L |
| 21-225_82D9_HC | . | . | . | . | . | . | . | . | . | . | . |
| 21-225_82G5_HC | . | . | . | . | . | . | . | . | . | . | . |
| 21-225_83C2_HC | . | . | . | . | . | . | . | . | . | . | . |
| 21-225_83C5_HC | . | . | . | . | . | . | . | . | . | . | . |
| 21-225_83F3_HC | . | . | . | . | . | . | . | . | . | . | . |
| 21-225_83G3_HC | . | . | . | . | . | . | . | . | . | . | . |

**FIGURE 57**

| Reference # | 139 | 140 | 141 | 142 | 143 | 144 | 145 | 146 | 147 | 148 | 149 |
|---|---|---|---|---|---|---|---|---|---|---|---|
| | H_FR4 | | | | | | | | | | |
| CONSENSUS | W | G | Q | G | T | L | V | T | V | S | S |
| 21-225_83G7_HC | . | . | . | . | . | . | . | . | . | A | . |
| 21-225_84G7_HC | . | . | . | . | . | . | . | . | . | A | . |
| 21-225_85B5_HC | . | . | . | . | . | . | . | . | . | . | . |
| 21-225_85D11_HC | . | . | . | . | . | . | . | . | . | . | . |
| 21-225_85H2_HC | . | . | . | . | . | . | . | . | . | . | . |
| 21-225_86E9_HC | . | . | . | . | . | . | . | . | . | . | . |
| 21-225_87A10_HC | . | . | . | . | . | . | . | . | . | . | L |
| 21-225_88G2_HC | . | . | . | . | . | . | . | . | . | . | . |
| 21-225_90D9_HC | . | . | . | . | . | . | . | . | . | A | . |
| 21-225_91F1_HC | . | . | . | . | . | . | . | . | . | . | . |
| 21-225_91G8_HC | . | . | . | . | . | . | . | . | . | . | . |
| 21-225_94D2_HC | . | . | . | . | . | . | . | . | . | . | . |
| 21-225_94E11_HC | . | . | . | . | . | . | . | . | . | . | . |
| 21-225_95E12_HC | . | . | . | . | . | . | . | . | . | . | . |
| 21-225_95G4_HC | . | . | . | . | . | . | . | . | . | . | . |
| 21-225_95H4_HC | . | . | . | . | . | . | . | . | . | . | . |
| 21-225_96D5_HC | . | . | . | . | . | . | . | . | . | . | . |
| 21-225_96E2_HC | . | . | . | . | . | . | . | . | . | . | . |
| 21-225_96G1_HC | . | . | . | . | . | . | . | . | . | . | L |
| 21-225_97H4_HC | . | . | . | . | . | . | . | . | . | . | . |

**FIGURE 57**

Table 54. Consensus 20- VH3|3-33/D4|4-17|RF2/JH6 (SEQ ID NO: 50271):

QVQLVES-GGGVVQPGRSLRLSCAASG-FTFSS-----YGMHWVRQAPGKGLEWVAVIWYD---GSNKNYADSVKGRFTISRDNSKNTLYLQMNSLRAEDTAVYYCARDQGVGY--------------------YGLDVWGQGTTVTVSS wherein:

S at position 33 can be substituted with N, R, T or I

Y at position 39 can be substituted with H or N

H at position 42 can be substituted with D

V at position 57 can be substituted with I

I at position 58 can be substituted with L

Y at position 60 can be substituted with F

S at position 66 can be substituted with T

K at position 68 can be substituted with E, Q, D or R

N at position 69 can be substituted with H or Y

Y at position 70 can be substituted with H

A at position 71 can be substituted with V or G

D at position 72 can be substituted with E

D at position 109 can be substituted with A

Q at position 110 can be substituted with R, Y, H, A, C, E, F or M

V at position 112 can be substituted with I or F

G at position 113 can be substituted with Y

**FIGURE 57**

Y at position 114 can be substituted with E

null (-) at position 115 can be substituted with F

null (-) at position 116 can be substituted with D

null (-) at position 131 can be substituted with Y

null (-) at position 132 can be substituted with Y

null (-) at position 133 can be substituted with N

Y at position 134 can be substituted with D, or N

G at position 135 can be substituted with A or D

L at position 136 can be substituted with M, Tor I

Xaa Xaa Gly Met Xaa (SEQ ID NO: 50016)

Xaa Xaa Trp Xaa Asp Gly Xaa Asn Xaa Xaa Xaa Xaa Xaa Ser Val Lys Gly (SEQ ID NO: 50017)

Xaa Xaa Gly Xaa Xaa Xaa Xaa Xaa Xaa Xaa Xaa Xaa Xaa Xaa Asp Val (SEQ ID NO: 50018)

Ser Tyr Gly Met His (SEQ ID NO: 50483)

Val Ile Trp Tyr Asp Gly Ser Asn Lys Asn Tyr Ala Asp Ser Val Lys Gly (SEQ ID NO: 50484)

Asp Gln Gly Val Gly Tyr Tyr Gly Leu Asp Val (SEQ ID NO: 50485)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | E | S | - | G | G | G | V | V | Q | P | G | R | S | L | R | L | S | C | A | A | S |
| 21-225_190C11_HC | . | . | . | . | . | . | . | . | . | . | . | . | . | . | . | . | . | . | . | . | . | . | . | . | . | . |
| 21-225_190E10_HC | . | . | . | . | . | . | . | . | . | . | . | . | . | . | . | . | . | . | . | K | . | . | . | . | . | . |
| 21-225_190E6_HC | . | . | . | . | . | . | . | . | . | . | . | . | . | . | . | . | . | . | . | . | . | . | . | . | . | . |
| 21-225_190F8_HC | . | . | . | . | . | . | . | . | . | . | . | . | . | . | . | . | . | . | . | . | . | . | . | . | . | . |
| 21-225_190G11_HC | . | . | . | . | . | . | . | . | . | . | . | . | . | . | . | . | . | . | . | . | . | . | . | . | . | . |
| 21-225_190G4_HC | . | . | . | . | . | . | . | . | . | . | . | . | . | . | . | . | . | . | . | . | . | . | . | . | . | . |
| 21-225_191A10_HC | . | . | . | . | . | . | . | . | . | . | . | . | . | . | . | . | . | . | . | . | . | . | . | . | . | . |
| 21-225_191E10_HC | . | . | . | . | . | . | . | . | . | . | . | . | . | . | . | . | . | . | . | . | . | . | . | . | . | . |
| 21-225_191E8_HC | . | . | . | . | . | . | . | . | . | . | . | . | . | . | . | . | . | . | . | . | . | . | . | . | . | . |
| 21-225_191G1_HC | . | . | . | . | . | . | . | . | . | . | . | . | . | . | . | . | . | . | . | . | . | . | . | . | . | . |
| 21-225_191G10_HC | . | . | . | . | . | . | . | . | . | . | . | . | . | . | . | . | . | . | . | . | . | . | . | . | . | . |
| 21-225_191G12_HC | . | . | . | . | . | . | . | . | . | . | . | . | . | . | . | . | . | . | . | . | . | . | . | . | . | . |
| 21-225_192C8_HC | . | . | . | . | . | . | . | . | . | . | . | . | . | . | . | . | . | . | . | . | . | . | . | . | . | . |
| 21-225_192D2_HC | . | . | . | . | . | . | . | . | . | . | . | . | . | . | . | . | . | . | . | . | . | . | . | . | . | . |
| 21-225_192D3_HC | . | . | . | . | . | . | . | . | . | . | . | . | . | . | . | . | . | . | . | . | . | . | . | . | . | . |
| 21-225_192G6_HC | . | . | . | . | . | . | . | . | . | . | . | . | . | . | . | . | . | . | . | . | . | . | . | . | . | . |
| 21-225_192G8_HC | . | . | . | . | . | . | . | . | . | . | . | . | . | . | . | . | . | . | . | . | . | . | . | . | . | . |
| 21-225_192H10_HC | . | . | . | . | . | . | . | . | . | . | . | . | . | . | . | . | . | . | . | . | . | . | . | . | . | . |
| 21-225_192H4_HC | . | . | . | . | . | . | . | . | . | . | . | . | . | . | . | . | . | . | . | . | . | . | . | . | . | . |
| 21-225_193A10_HC | . | . | . | . | . | . | . | . | . | . | . | . | . | . | . | . | . | . | . | . | . | . | . | . | . | . |
| 21-225_193C7_HC | . | . | . | . | . | . | . | . | . | . | . | . | . | . | . | . | . | . | . | . | . | . | . | . | . | . |
| 21-225_194H11_HC | . | . | . | . | . | . | . | . | . | . | . | . | . | . | . | . | . | . | . | . | . | . | . | . | . | . |
| 21-225_195A9_HC | . | . | . | . | . | . | . | . | . | . | . | . | . | . | . | . | . | . | . | . | . | . | . | . | . | . |
| 21-225_196C7_HC | . | . | . | . | . | . | . | . | . | . | . | . | . | . | . | . | . | . | . | . | . | . | . | . | . | . |
| 21-225_197B6_HC | . | . | . | . | . | . | . | . | . | . | . | . | . | . | . | . | . | . | . | . | . | . | . | . | . | . |
| 21-225_197D3_HC | . | . | . | . | . | . | . | . | . | . | . | . | . | . | . | . | . | . | . | . | . | . | . | . | . | . |
| 21-225_197F2_HC | . | . | . | . | . | . | . | . | . | . | . | . | . | . | . | . | . | . | . | . | . | . | . | . | . | . |
| 21-225_197G12_HC | . | . | . | . | . | . | . | . | . | . | . | . | . | . | . | . | . | . | . | . | . | . | . | . | . | . |
| 21-225_198B6_HC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | E | S | - | G | G | G | V | V | Q | P | G | R | S | L | R | L | S | C | A | A | S |
| 21-225_199A6_HC | . | . | . | . | . | . | . | . | . | . | . | . | . | . | . | . | . | . | . | . | . | . | . | . | . | . |
| 21-225_199C5_HC | . | . | . | . | . | . | . | . | . | . | . | . | . | . | . | . | . | . | . | . | . | . | . | . | . | . |
| 21-225_204G6_HC | . | . | . | . | . | . | . | . | . | . | . | . | . | . | . | . | . | . | . | . | . | . | . | T | . | . |
| 21-225_205F5_HC | . | . | . | . | . | . | . | . | . | . | . | . | . | . | . | . | . | . | . | . | . | . | . | . | . | . |
| 21-225_207G6_HC | . | . | . | . | . | . | . | . | . | . | . | . | . | . | . | . | . | . | . | . | . | . | . | . | . | . |
| 21-225_211A11_HC | . | . | . | . | . | G | . | . | . | . | . | . | . | . | . | . | . | . | . | . | . | . | . | . | . | . |
| 21-225_211A8_HC | . | . | . | . | . | . | . | . | . | . | . | . | . | . | . | . | . | . | . | . | . | . | . | . | . | . |
| 21-225_212D7_HC | . | . | . | . | . | . | . | . | . | . | . | . | . | . | . | . | . | . | . | . | . | . | . | . | . | . |
| 21-225_212E10_HC | . | . | . | . | . | . | . | . | . | . | . | . | . | . | . | . | . | . | . | . | . | . | . | . | . | . |
| 21-225_212E6_HC | . | . | . | . | . | . | . | . | . | . | . | . | . | . | . | . | . | . | . | . | . | . | . | . | . | . |
| 21-225_212F10_HC | . | . | . | . | . | . | . | . | . | . | . | . | . | . | . | . | . | . | . | . | . | . | . | . | . | . |
| 21-225_212H9_HC | . | . | . | . | . | . | . | . | . | . | . | . | . | . | . | . | . | . | . | . | . | . | . | . | . | . |
| 21-225_213B8_HC | . | . | . | . | . | . | . | . | . | . | . | . | . | . | . | . | . | . | . | . | . | . | . | . | . | . |
| 21-225_213C4_HC | . | . | . | . | . | . | . | . | . | . | . | . | . | . | . | . | . | . | . | . | . | . | . | . | T | . |
| 21-225_213D2_HC | . | . | . | . | . | . | . | . | . | . | . | . | . | . | . | . | . | . | . | . | . | . | . | . | . | . |
| 21-225_214C3_HC | . | . | . | . | . | . | . | . | . | . | . | . | . | . | . | . | . | . | . | . | . | . | . | E | . | . |
| 21-225_215A12_HC | . | . | . | . | . | . | . | . | . | . | . | . | . | . | . | . | . | . | . | . | . | . | . | . | . | . |
| 21-225_215B5_HC | . | . | . | . | . | . | . | . | . | . | . | . | . | . | . | . | . | . | . | . | . | . | . | . | . | . |
| 21-225_215D6_HC | . | . | . | . | . | . | . | . | . | . | . | . | . | . | . | . | . | . | . | . | . | . | . | . | . | . |
| 21-225_217G5_HC | . | . | . | . | . | . | . | . | . | . | . | . | . | . | . | . | . | . | . | . | . | . | . | . | . | . |
| 21-225_219H1_HC | . | . | . | . | . | . | . | . | . | . | . | . | . | . | . | . | . | . | . | . | . | . | . | . | . | . |
| 21-225_223D11_HC | . | . | . | . | . | . | . | . | . | . | . | . | . | . | . | . | . | . | . | . | . | . | . | . | . | . |
| 21-225_74D1_HC | . | . | . | . | . | . | . | . | . | . | . | . | . | . | . | . | . | . | . | . | . | . | . | . | . | . |
| 21-225_87B7_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | G | M | H | W | V | R | Q | A | P | G | K | G | L |
| 21-225_190C11_HC | . | . | . | . | . | . | . | . | . | . | . | . | . | . | . | . | . | . | . | . | . | . | . | . | . | . |
| 21-225_190E10_HC | . | . | . | . | . | . | T | . | . | . | . | . | . | . | . | . | . | . | . | . | . | . | . | . | . | . |
| 21-225_190E6_HC | . | . | . | . | . | . | . | . | . | . | . | . | . | . | . | . | . | . | . | . | . | . | . | . | . | . |
| 21-225_190F8_HC | . | . | . | . | . | . | T | . | . | . | . | . | . | . | . | . | . | . | . | . | . | . | . | . | . | . |
| 21-225_190G11_HC | . | . | . | . | . | . | I | . | . | . | . | . | . | . | . | . | . | . | . | . | . | . | . | . | . | . |
| 21-225_190G4_HC | . | . | . | . | . | . | . | . | . | . | . | . | . | . | . | . | . | . | . | . | . | . | . | . | . | . |
| 21-225_191A10_HC | . | . | . | . | . | . | . | . | . | . | . | . | . | . | . | . | . | . | . | . | . | . | . | . | . | . |
| 21-225_191E10_HC | . | . | . | . | . | . | . | . | . | . | . | . | . | . | . | . | . | . | . | . | . | . | . | . | . | . |
| 21-225_191E8_HC | . | . | . | . | . | . | N | . | . | . | . | . | . | . | . | . | . | . | . | . | . | . | . | . | . | . |
| 21-225_191G1_HC | . | . | . | . | . | . | . | . | . | . | . | . | . | . | . | . | . | . | . | . | . | . | . | . | . | . |
| 21-225_191G10_HC | . | . | . | . | . | . | . | . | . | . | . | . | . | . | . | . | . | . | . | . | . | . | . | . | . | . |
| 21-225_191G12_HC | . | . | . | . | . | . | . | . | . | . | . | . | . | . | . | . | . | . | . | . | . | . | . | . | . | . |
| 21-225_192C8_HC | . | . | . | . | . | . | . | . | . | . | . | . | . | . | . | . | . | . | . | . | . | . | . | . | . | . |
| 21-225_192D2_HC | . | . | . | . | . | . | . | . | . | . | . | . | . | . | . | . | . | . | . | . | . | . | . | . | . | . |
| 21-225_192D3_HC | . | . | . | . | . | . | . | . | . | . | . | . | . | . | . | . | . | . | . | . | . | . | . | . | . | . |
| 21-225_192G6_HC | . | . | . | . | . | . | . | . | . | . | . | . | . | . | . | . | . | . | . | . | . | . | . | . | . | . |
| 21-225_192G8_HC | . | . | . | . | . | . | . | . | . | . | . | . | . | . | . | . | . | . | . | . | . | . | . | . | . | . |
| 21-225_192H10_HC | . | . | . | . | . | . | . | . | . | . | . | . | . | . | . | . | . | . | . | . | . | . | . | . | . | . |
| 21-225_192H4_HC | . | . | . | . | . | . | . | . | . | . | . | . | . | . | . | . | . | . | . | . | . | . | . | . | . | . |
| 21-225_193A10_HC | . | . | . | . | . | . | . | . | . | . | . | . | . | . | . | . | . | . | . | . | . | . | . | . | . | . |
| 21-225_193C7_HC | . | . | . | . | . | . | . | . | . | . | . | . | . | . | . | . | . | . | . | . | . | . | . | . | . | . |
| 21-225_194H11_HC | . | . | . | . | . | . | . | . | . | . | . | . | . | . | . | . | . | . | . | . | . | . | . | . | . | . |
| 21-225_195A9_HC | . | . | . | . | . | . | . | . | . | . | . | . | . | . | . | . | . | . | . | . | . | . | . | . | . | . |
| 21-225_196C7_HC | . | . | . | . | . | . | . | . | . | . | . | . | . | . | . | . | . | . | . | . | . | . | . | . | . | . |
| 21-225_197B6_HC | . | . | . | . | . | . | . | . | . | . | . | . | . | . | . | . | . | . | . | . | . | . | . | . | . | . |
| 21-225_197D3_HC | . | . | . | I | . | . | . | . | . | . | . | . | H | . | . | . | . | . | . | . | . | . | . | . | . | . |
| 21-225_197F2_HC | . | . | . | . | . | . | . | . | . | . | . | . | . | . | . | . | . | . | . | . | . | . | . | . | . | . |
| 21-225_197G12_HC | . | . | . | . | . | . | . | . | . | . | . | . | . | . | . | . | . | . | . | . | . | . | . | . | . | . |
| 21-225_198B6_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | G | M | H | W | V | R | Q | A | P | G | K | G | L |
| 21-225_199A6_HC | . | . | . | . | . | . | N | . | . | . | . | . | . | . | . | . | . | . | . | . | . | . | . | . | . | . |
| 21-225_199C5_HC | . | . | . | . | . | . | . | . | . | . | . | . | . | . | . | . | . | . | . | . | . | . | . | . | . | . |
| 21-225_204G6_HC | . | . | . | . | . | . | . | . | . | . | . | . | . | . | . | . | . | . | . | . | . | . | . | . | . | . |
| 21-225_205F5_HC | . | . | . | . | . | . | R | . | . | . | . | . | . | . | . | . | . | . | . | . | . | . | . | . | . | . |
| 21-225_207G6_HC | . | . | . | . | . | . | . | . | . | . | . | . | . | . | . | . | . | . | . | . | . | . | . | . | . | . |
| 21-225_211A11_HC | . | . | . | . | . | . | . | . | . | . | . | . | . | . | . | . | . | . | . | . | . | . | . | . | . | . |
| 21-225_211A8_HC | . | . | . | . | . | . | . | . | . | . | . | . | . | . | . | . | . | . | . | . | . | . | . | . | . | . |
| 21-225_212D7_HC | . | . | . | . | . | . | . | . | . | . | . | . | . | . | . | . | . | . | . | . | . | . | . | . | . | . |
| 21-225_212E10_HC | . | . | . | . | . | . | . | . | . | . | . | . | . | . | . | . | . | . | . | . | . | . | . | . | . | . |
| 21-225_212E6_HC | . | . | . | . | . | . | . | . | . | . | . | . | . | . | . | . | . | . | . | . | . | . | . | . | . | . |
| 21-225_212F10_HC | . | . | . | . | . | . | R | . | . | . | . | . | . | . | . | . | . | . | . | . | . | . | . | . | . | . |
| 21-225_212H9_HC | . | . | . | . | . | . | . | . | . | . | . | . | . | . | . | . | . | . | . | . | . | . | . | . | . | . |
| 21-225_213B8_HC | . | . | . | . | . | . | . | . | . | . | . | . | . | . | . | . | . | . | . | . | . | . | . | . | . | . |
| 21-225_213C4_HC | . | . | . | . | . | . | . | . | . | . | . | . | . | . | . | . | . | . | . | . | . | . | . | . | . | . |
| 21-225_213D2_HC | . | . | . | . | . | . | . | . | . | . | . | . | . | . | . | . | . | . | . | . | . | . | . | . | . | . |
| 21-225_214C3_HC | . | . | . | . | . | . | . | . | . | . | . | . | . | . | . | . | . | . | . | . | . | . | . | . | . | . |
| 21-225_215A12_HC | . | . | L | . | . | . | . | . | . | . | . | . | . | . | . | . | . | . | . | . | . | . | . | . | . | . |
| 21-225_215B5_HC | . | . | . | . | . | . | . | . | . | . | . | . | . | . | . | . | . | . | . | . | . | . | . | . | . | . |
| 21-225_215D6_HC | . | . | . | . | . | . | . | . | . | . | . | . | . | . | . | . | . | . | . | . | . | . | . | . | . | . |
| 21-225_217G5_HC | . | . | . | . | . | . | . | . | . | . | . | . | N | . | . | . | . | . | . | . | . | . | . | . | . | . |
| 21-225_219H1_HC | . | . | . | . | . | . | R | . | . | . | . | . | . | . | . | . | . | . | . | . | . | . | . | . | . | . |
| 21-225_223D11_HC | . | . | . | . | . | . | N | . | . | . | . | . | . | . | . | D | . | . | . | . | . | . | . | . | . | . |
| 21-225_74D1_HC | . | . | . | . | . | . | . | . | . | . | . | . | . | . | . | . | . | . | . | . | . | . | . | . | . | . |
| 21-225_87B7_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | A | V | I | W | Y | D | - | - | - | G | S | N | K | N | Y | A | D | S | V | K | G | R | F |
| 21-225_190C11_HC | . | . | . | . | . | . | . | . | . | . | . | . | . | . | . | . | . | . | . | . | . | . | . | . | . | . |
| 21-225_190E10_HC | . | . | . | . | . | . | . | . | . | . | . | . | . | T | . | . | . | . | . | . | . | . | . | . | . | . |
| 21-225_190E6_HC | . | . | . | . | . | . | . | . | . | . | . | . | . | . | . | . | . | . | . | . | . | . | . | . | . | . |
| 21-225_190F8_HC | . | . | . | . | . | . | . | . | . | . | . | . | . | . | . | . | . | . | . | . | . | . | . | . | . | . |
| 21-225_190G11_HC | . | . | . | . | . | . | . | . | . | . | . | . | . | . | . | . | . | . | . | . | . | . | . | . | . | . |
| 21-225_190G4_HC | . | . | . | . | . | . | . | . | . | . | . | . | . | . | . | . | . | . | . | . | . | . | . | . | . | . |
| 21-225_191A10_HC | . | . | . | . | . | . | . | . | . | . | . | . | . | . | . | . | . | . | . | . | . | . | . | . | . | . |
| 21-225_191E10_HC | . | . | . | . | . | . | . | . | . | . | . | . | . | . | . | . | . | . | . | . | . | . | . | . | . | . |
| 21-225_191E8_HC | . | . | . | . | I | . | . | F | . | . | . | . | . | . | . | Q | Y | . | . | . | . | . | . | . | . | . |
| 21-225_191G1_HC | . | . | . | . | . | . | . | . | . | . | . | . | . | . | . | E | H | . | . | . | . | . | . | . | . | . |
| 21-225_191G10_HC | . | . | . | . | . | . | . | . | . | . | . | . | . | . | . | . | . | . | . | . | . | . | . | . | . | . |
| 21-225_191G12_HC | . | . | . | . | . | . | . | . | . | . | . | . | . | . | . | . | . | . | . | . | . | . | . | . | . | . |
| 21-225_192C8_HC | . | . | . | . | . | . | . | . | . | . | . | . | . | . | . | E | H | . | . | . | . | . | . | . | . | . |
| 21-225_192D2_HC | . | . | . | . | . | . | . | . | . | . | . | . | . | . | . | . | . | . | . | . | . | . | . | . | . | . |
| 21-225_192D3_HC | . | . | . | . | . | . | . | . | . | . | . | . | . | . | . | E | H | . | . | . | . | . | . | . | . | . |
| 21-225_192G6_HC | . | . | . | . | . | L | . | . | . | . | . | . | . | T | . | . | . | . | . | . | . | . | . | . | . | . |
| 21-225_192G8_HC | . | . | . | . | . | . | . | . | . | . | . | . | . | . | . | . | . | . | . | . | . | . | . | . | . | . |
| 21-225_192H10_HC | . | . | . | . | . | . | . | . | . | . | . | . | . | . | . | . | . | . | . | . | . | . | . | . | . | . |
| 21-225_192H4_HC | . | . | . | . | . | . | . | . | . | . | . | . | . | . | . | . | . | . | . | . | . | . | . | . | . | . |
| 21-225_193A10_HC | . | . | . | . | . | . | . | . | . | . | . | . | . | . | . | E | H | . | . | . | . | . | . | . | . | . |
| 21-225_193C7_HC | . | . | . | . | . | . | . | . | . | . | . | . | . | . | . | . | . | . | . | . | . | . | . | . | . | . |
| 21-225_194H11_HC | . | . | . | . | . | . | . | . | . | . | . | . | . | . | . | E | H | . | . | . | . | . | . | . | . | . |
| 21-225_195A9_HC | Q | . | . | . | . | . | . | . | . | . | . | . | . | . | . | E | H | . | . | . | . | . | . | . | . | . |
| 21-225_196C7_HC | . | . | . | . | . | . | . | . | . | . | . | . | . | . | . | E | H | . | . | . | . | . | . | . | . | . |
| 21-225_197B6_HC | . | . | . | . | . | . | . | . | . | . | . | . | . | . | . | . | . | . | . | . | . | . | . | . | . | . |
| 21-225_197D3_HC | . | . | . | . | . | . | . | . | . | . | . | . | . | . | . | E | H | . | . | . | . | . | . | . | . | . |
| 21-225_197F2_HC | . | . | . | . | . | . | . | . | . | . | . | . | . | . | . | . | . | . | . | . | . | . | . | . | . | . |
| 21-225_197G12_HC | . | . | . | . | . | . | . | . | . | . | . | . | . | . | . | . | . | . | . | . | . | . | . | . | . | . |
| 21-225_198B6_HC | . | . | . | . | . | . | . | . | . | . | . | . | . | . | . | . | H | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | A | V | I | W | Y | D | - | - | - | G | S | N | K | N | Y | A | D | S | V | K | G | R | F |
| 21-225_199A6_HC | . | . | . | . | I | . | . | F | . | . | . | . | . | . | . | Q | Y | . | . | . | . | . | . | . | . | . |
| 21-225_199C5_HC | . | . | . | . | . | . | . | . | . | . | . | . | . | . | . | . | . | . | . | . | . | . | . | . | . | . |
| 21-225_204G6_HC | . | . | . | . | . | . | . | . | . | . | . | . | . | . | . | . | . | . | . | . | . | . | . | . | . | . |
| 21-225_205F5_HC | . | . | . | . | . | . | . | . | . | . | . | . | . | . | . | E | . | . | V | . | . | . | . | . | . | . |
| 21-225_207G6_HC | . | . | . | . | . | . | . | . | . | . | . | . | . | . | . | . | . | . | . | E | . | . | . | . | . | . |
| 21-225_211A11_HC | . | . | . | . | . | L | . | F | . | . | . | . | . | . | . | R | . | . | . | . | . | . | . | . | . | . |
| 21-225_211A8_HC | . | . | . | . | . | . | . | . | . | . | . | . | . | . | . | E | H | . | . | . | . | . | . | . | . | . |
| 21-225_212D7_HC | . | . | . | . | . | . | . | . | . | . | . | . | . | . | . | . | . | . | . | . | . | . | . | . | . | . |
| 21-225_212E10_HC | . | . | . | . | . | . | . | . | . | . | . | . | . | . | . | . | Y | . | . | . | . | . | . | . | . | . |
| 21-225_212E6_HC | . | . | . | . | . | . | . | . | . | . | . | . | . | . | . | . | . | . | V | . | . | . | . | . | . | . |
| 21-225_212F10_HC | . | . | . | . | . | . | . | . | . | . | . | . | . | . | . | . | H | . | . | . | . | . | . | . | . | . |
| 21-225_212H9_HC | . | . | . | . | . | . | . | . | . | . | . | . | . | . | . | E | H | . | . | . | . | . | . | . | . | . |
| 21-225_213B8_HC | . | . | . | . | . | . | . | . | . | . | . | . | . | . | . | . | H | . | . | . | . | . | . | . | . | . |
| 21-225_213C4_HC | . | . | . | . | . | . | . | . | . | . | . | . | . | . | . | . | H | . | . | . | . | . | . | . | . | . |
| 21-225_213D2_HC | . | . | . | . | . | . | . | . | . | . | . | . | . | . | . | . | . | . | . | . | . | . | . | . | . | . |
| 21-225_214C3_HC | . | . | . | . | . | . | . | . | . | . | . | . | . | . | . | . | . | . | G | . | . | . | . | . | . | . |
| 21-225_215A12_HC | . | . | . | . | . | . | . | . | . | . | . | . | . | . | . | E | H | . | . | . | . | . | . | . | . | . |
| 21-225_215B5_HC | . | . | . | . | . | . | . | . | . | . | . | . | . | . | . | . | . | . | . | . | . | . | . | . | . | . |
| 21-225_215D6_HC | . | . | . | . | . | . | . | . | . | . | . | . | . | . | . | . | . | . | . | . | . | . | . | . | . | . |
| 21-225_217G5_HC | . | . | . | . | . | . | . | . | . | . | . | . | . | . | . | . | . | . | . | . | . | . | . | . | . | . |
| 21-225_219H1_HC | . | . | . | . | . | . | . | . | . | . | . | . | . | . | . | . | H | . | . | . | . | . | . | . | . | . |
| 21-225_223D11_HC | . | . | . | . | . | . | . | . | . | . | . | . | . | . | . | D | Y | . | . | . | . | . | . | . | . | . |
| 21-225_74D1_HC | . | . | . | . | . | . | . | . | . | . | . | . | . | . | . | . | Y | H | . | . | . | . | . | . | . | . |
| 21-225_87B7_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_190C11_HC | . | . | . | . | . | . | . | . | . | . | . | . | . | . | . | . | . | . | . | . | . | . | . | . | . | . |
| 21-225_190E10_HC | . | . | . | . | . | . | . | . | . | . | . | C | . | . | . | . | . | . | . | . | . | . | . | . | . | . |
| 21-225_190E6_HC | . | . | . | . | . | . | . | . | . | . | . | . | . | . | . | . | . | . | . | . | . | . | . | . | . | . |
| 21-225_190F8_HC | . | . | . | . | . | . | . | . | . | . | . | . | . | . | . | . | . | . | . | . | . | . | . | . | . | . |
| 21-225_190G11_HC | . | . | . | . | . | . | . | . | . | . | . | . | . | . | . | . | . | . | . | . | . | . | . | . | . | . |
| 21-225_190G4_HC | . | . | . | . | . | . | . | . | . | . | . | . | . | . | . | . | . | . | . | . | . | . | . | . | M | . |
| 21-225_191A10_HC | . | . | . | . | . | . | . | . | . | . | . | . | . | . | . | . | . | . | . | . | . | . | . | . | M | . |
| 21-225_191E10_HC | . | . | . | . | . | . | . | . | . | . | . | . | . | . | . | . | . | . | . | . | . | . | . | . | M | . |
| 21-225_191E8_HC | . | . | . | . | . | . | . | . | . | . | . | . | . | . | L | . | . | . | . | . | . | . | . | . | . | . |
| 21-225_191G1_HC | . | . | . | . | . | . | . | . | . | . | . | . | . | . | . | . | . | . | . | . | . | . | . | . | . | . |
| 21-225_191G10_HC | . | . | . | . | . | . | . | . | . | . | . | . | . | . | . | . | . | . | . | . | . | . | . | . | . | . |
| 21-225_191G12_HC | . | . | . | . | . | . | . | . | . | . | . | . | . | . | . | . | . | . | . | . | . | . | . | . | . | . |
| 21-225_192C8_HC | M | . | . | . | . | . | . | . | . | . | . | . | . | . | . | . | . | . | . | . | . | . | . | . | . | . |
| 21-225_192D2_HC | . | . | . | . | . | . | . | . | . | M | . | . | . | . | . | . | . | . | . | . | . | E | . | . | . | . |
| 21-225_192D3_HC | . | . | . | . | . | . | . | . | . | . | . | . | . | . | . | . | . | . | . | . | . | . | . | . | . | . |
| 21-225_192G6_HC | . | . | . | . | . | . | . | . | . | . | . | . | . | . | . | . | . | . | . | . | . | . | . | . | . | . |
| 21-225_192G8_HC | . | . | . | . | . | . | . | . | . | . | . | . | . | . | . | . | . | . | . | . | . | . | . | . | . | . |
| 21-225_192H10_HC | . | . | . | . | . | . | . | . | . | . | . | . | . | . | . | . | . | . | . | . | . | . | . | . | M | . |
| 21-225_192H4_HC | . | . | . | . | . | . | . | . | . | . | . | . | . | . | . | . | . | . | . | . | . | . | . | . | . | . |
| 21-225_193A10_HC | . | . | . | . | . | . | . | . | . | . | . | . | . | . | . | . | . | . | . | . | . | . | . | . | . | . |
| 21-225_193C7_HC | . | . | . | . | . | . | . | . | . | . | . | . | . | . | . | . | . | . | . | . | . | . | . | . | M | . |
| 21-225_194H11_HC | . | . | . | . | . | . | . | . | . | . | . | S | . | . | . | . | . | . | . | . | . | . | . | . | . | . |
| 21-225_195A9_HC | . | . | . | . | . | . | . | . | . | . | . | . | . | . | . | . | . | . | . | . | . | . | . | . | . | . |
| 21-225_196C7_HC | . | . | . | . | . | . | . | . | . | . | . | . | . | . | . | . | . | . | . | . | . | . | . | . | . | . |
| 21-225_197B6_HC | . | . | . | . | . | . | . | . | . | M | . | . | . | . | . | . | . | . | . | . | . | . | . | . | M | . |
| 21-225_197D3_HC | . | . | . | . | . | . | . | . | . | . | . | . | . | . | . | . | . | . | . | . | . | . | . | . | . | . |
| 21-225_197F2_HC | . | . | . | . | . | . | . | . | . | . | . | . | . | . | . | . | . | . | . | . | . | . | . | . | . | . |
| 21-225_197G12_HC | . | . | . | . | . | . | . | . | . | . | . | . | . | . | . | . | . | . | . | . | . | . | . | . | . | . |
| 21-225_198B6_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_199A6_HC | . | . | . | . | . | . | . | . | . | . | . | . | . | . | L | . | . | . | . | . | . | . | . | . | . | . |
| 21-225_199C5_HC | . | . | . | . | . | . | . | . | . | . | . | . | . | . | . | . | . | . | . | . | . | . | . | . | . | . |
| 21-225_204G6_HC | . | . | . | . | . | . | . | . | . | . | . | . | . | . | . | . | . | . | . | . | . | . | . | . | . | . |
| 21-225_205F5_HC | . | . | . | . | . | T | . | . | K | M | . | F | . | . | . | . | . | . | . | T | D | . | . | . | . | . |
| 21-225_207G6_HC | . | . | . | . | . | . | . | . | . | . | . | . | . | . | . | . | . | . | . | . | . | . | . | . | . | . |
| 21-225_211A11_HC | . | . | . | . | . | . | . | . | . | . | . | . | . | . | . | . | . | . | . | . | . | . | . | . | . | . |
| 21-225_211A8_HC | . | . | . | . | . | . | . | . | K | . | . | . | . | . | . | . | . | . | . | . | . | . | . | . | . | . |
| 21-225_212D7_HC | . | . | . | . | . | . | . | . | . | . | . | S | . | . | . | . | . | . | . | . | . | . | . | . | . | . |
| 21-225_212E10_HC | . | . | . | . | . | . | . | . | . | . | . | S | . | . | . | . | . | . | . | . | . | . | . | . | . | . |
| 21-225_212E6_HC | . | . | . | . | . | . | . | . | . | . | . | . | . | . | . | . | . | . | . | . | . | . | . | . | . | . |
| 21-225_212F10_HC | . | . | . | . | . | . | . | . | . | . | . | . | . | . | . | . | . | . | . | G | . | . | . | . | . | . |
| 21-225_212H9_HC | . | . | . | . | . | . | . | . | K | . | . | . | . | . | . | . | . | . | . | . | . | . | . | . | . | . |
| 21-225_213B8_HC | . | . | . | . | . | . | . | . | . | . | . | . | . | . | . | . | . | . | . | . | . | . | . | . | . | . |
| 21-225_213C4_HC | . | . | . | . | . | . | . | . | . | . | . | . | . | . | . | . | . | . | . | . | . | . | . | . | . | . |
| 21-225_213D2_HC | . | . | . | . | . | . | . | . | . | . | . | S | . | . | . | . | . | . | . | . | . | . | . | . | . | . |
| 21-225_214C3_HC | . | . | . | . | . | . | . | . | . | . | . | . | . | . | . | . | . | . | . | . | . | . | . | . | . | . |
| 21-225_215A12_HC | . | . | . | . | . | . | . | . | . | . | . | . | . | . | . | . | . | . | . | . | . | . | . | . | . | . |
| 21-225_215B5_HC | . | . | . | . | . | . | . | . | . | . | . | S | . | . | . | . | . | . | . | . | . | . | . | . | . | . |
| 21-225_215D6_HC | . | . | . | . | . | . | . | . | . | . | . | . | . | . | . | . | . | . | . | . | . | . | . | . | . | . |
| 21-225_217G5_HC | . | . | . | . | . | . | . | . | . | . | . | . | . | . | . | . | . | . | . | . | . | . | . | . | . | . |
| 21-225_219H1_HC | . | . | . | . | . | . | . | . | . | . | . | . | . | . | . | . | . | . | . | G | . | . | . | . | . | . |
| 21-225_223D11_HC | . | . | . | . | . | . | . | . | . | . | . | F | . | . | . | . | . | . | . | . | . | . | . | . | . | . |
| 21-225_74D1_HC | . | . | . | . | . | . | . | . | . | . | . | . | . | . | . | . | . | . | . | . | . | . | . | . | M | . |
| 21-225_87B7_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | Q | G | V | G | Y | - | - | - | - | - | - | - | - | - | - | - | - | - | - | - | - |
| 21-225_190C11_HC | . | . | . | . | . | . | . | . | . | . | . | . | . | . | . | . | . | . | . | . | . | . | . | . | . | . |
| 21-225_190E10_HC | . | . | . | . | . | . | . | . | . | . | . | . | . | . | . | . | . | . | . | . | . | . | . | . | . | . |
| 21-225_190E6_HC | . | . | . | . | . | . | . | . | . | . | . | . | . | . | . | . | . | . | . | . | . | . | . | . | . | . |
| 21-225_190F8_HC | . | . | . | . | . | . | . | . | . | . | . | . | . | . | . | . | . | . | . | . | . | . | . | . | . | . |
| 21-225_190G11_HC | . | . | . | . | . | . | . | . | . | . | . | . | . | . | . | . | . | . | . | . | . | . | . | . | . | . |
| 21-225_190G4_HC | . | . | . | . | . | . | . | . | . | . | . | . | . | . | . | . | . | . | . | . | . | . | . | . | . | . |
| 21-225_191A10_HC | . | . | . | . | . | . | . | . | . | . | . | . | . | . | . | . | . | . | . | . | . | . | . | . | . | . |
| 21-225_191E10_HC | . | . | . | . | . | . | . | . | . | . | . | . | . | . | . | . | . | . | . | . | . | . | . | . | . | . |
| 21-225_191E8_HC | . | . | . | . | A | H | . | . | Y | . | . | . | . | . | . | . | . | . | . | . | . | . | . | . | . | . |
| 21-225_191G1_HC | . | . | . | . | . | R | . | . | . | . | . | . | . | . | . | . | . | . | . | . | . | . | . | . | . | . |
| 21-225_191G10_HC | . | . | . | . | . | . | . | . | . | . | . | . | . | . | . | . | . | . | . | . | . | . | . | . | . | . |
| 21-225_191G12_HC | . | . | . | . | . | . | . | . | . | . | . | . | . | . | . | . | . | . | . | . | . | . | . | . | . | . |
| 21-225_192C8_HC | . | . | . | . | . | R | . | . | . | . | . | . | . | . | . | . | . | . | . | . | . | . | . | . | . | . |
| 21-225_192D2_HC | . | . | . | . | . | R | . | . | . | . | . | . | . | . | . | . | . | . | . | . | . | . | . | . | . | . |
| 21-225_192D3_HC | . | . | . | . | . | R | . | . | . | . | . | . | . | . | . | . | . | . | . | . | . | . | . | . | . | . |
| 21-225_192G6_HC | . | . | . | . | . | . | . | . | . | . | . | . | . | . | . | . | . | . | . | . | . | . | . | . | . | . |
| 21-225_192G8_HC | . | . | . | . | . | . | . | . | . | . | . | . | . | . | . | . | . | . | . | . | . | . | . | . | . | . |
| 21-225_192H10_HC | . | . | . | . | . | . | . | . | . | . | . | . | . | . | . | . | . | . | . | . | . | . | . | . | . | . |
| 21-225_192H4_HC | . | . | . | . | . | . | . | . | . | . | . | . | . | . | . | . | . | . | . | . | . | . | . | . | . | . |
| 21-225_193A10_HC | . | . | . | . | . | R | . | . | . | . | . | . | . | . | . | . | . | . | . | . | . | . | . | . | . | . |
| 21-225_193C7_HC | . | . | . | . | . | . | . | . | . | . | . | . | . | . | . | . | . | . | . | . | . | . | . | . | . | . |
| 21-225_194H11_HC | . | . | . | . | . | R | . | . | . | . | . | . | . | . | . | . | . | . | . | . | . | . | . | . | . | . |
| 21-225_195A9_HC | . | . | . | . | . | R | . | . | . | . | . | . | . | . | . | . | . | . | . | . | . | . | . | . | . | . |
| 21-225_196C7_HC | . | . | . | . | . | R | . | . | . | . | . | . | . | . | . | . | . | . | . | . | . | . | . | . | . | . |
| 21-225_197B6_HC | . | . | . | . | . | . | . | . | . | . | . | . | . | . | . | . | . | . | . | . | . | . | . | . | . | . |
| 21-225_197D3_HC | . | . | . | . | . | . | . | . | . | . | . | . | . | . | . | . | . | . | . | . | . | . | . | . | . | . |
| 21-225_197F2_HC | . | . | . | . | . | . | . | . | . | . | . | . | . | . | . | . | . | . | . | . | . | . | . | . | . | . |
| 21-225_197G12_HC | . | . | . | . | . | . | . | . | . | . | . | . | . | . | . | . | . | . | . | . | . | . | . | . | . | . |
| 21-225_198B6_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | D | Q | G | V | G | Y | - | - | - | - | - | - | - | - | - | - | - | - | - | - | - | - |
| 21-225_199A6_HC | . | . | . | . | A | H | . | . | Y | . | . | . | . | . | . | . | . | . | . | . | . | . | . | . | . | . |
| 21-225_199C5_HC | . | . | . | . | . | . | . | . | . | . | . | . | . | . | . | . | . | . | . | . | . | . | . | . | . | . |
| 21-225_204G6_HC | . | . | . | . | . | R | . | . | . | . | . | . | . | . | . | . | . | . | . | . | . | . | . | . | . | . |
| 21-225_205F5_HC | . | . | . | . | . | M | . | I | . | . | . | . | . | . | . | . | . | . | . | . | . | . | . | . | . | . |
| 21-225_207G6_HC | . | . | . | . | . | A | . | I | . | . | . | . | . | . | . | . | . | . | . | . | . | . | . | . | . | . |
| 21-225_211A11_HC | . | . | . | . | . | R | . | . | . | . | . | . | . | . | . | . | . | . | . | . | . | . | . | . | . | . |
| 21-225_211A8_HC | . | . | . | . | . | H | . | . | . | . | . | . | . | . | . | . | . | . | . | . | . | . | . | . | . | . |
| 21-225_212D7_HC | . | . | . | . | . | Y | . | . | . | . | . | . | . | . | . | . | . | . | . | . | . | . | . | . | . | . |
| 21-225_212E10_HC | . | . | . | . | . | Y | . | . | . | . | . | . | . | . | . | . | . | . | . | . | . | . | . | . | . | . |
| 21-225_212E6_HC | . | . | . | . | . | Y | . | . | . | . | . | . | . | . | . | . | . | . | . | . | . | . | . | . | . | . |
| 21-225_212F10_HC | . | . | . | . | . | R | . | . | . | . | . | . | . | . | . | . | . | . | . | . | . | . | . | . | . | . |
| 21-225_212H9_HC | . | . | . | . | . | H | . | . | . | . | . | . | . | . | . | . | . | . | . | . | . | . | . | . | . | . |
| 21-225_213B8_HC | . | . | . | . | . | Y | . | . | . | . | . | . | . | . | . | . | . | . | . | . | . | . | . | . | . | . |
| 21-225_213C4_HC | . | . | . | . | . | Y | . | . | . | . | . | . | . | . | . | . | . | . | . | . | . | . | . | . | . | . |
| 21-225_213D2_HC | . | . | . | . | . | Y | . | . | . | . | . | . | . | . | . | . | . | . | . | . | . | . | . | . | . | . |
| 21-225_214C3_HC | . | . | . | . | . | R | . | . | . | . | . | . | . | . | . | . | . | . | . | . | . | . | . | . | . | . |
| 21-225_215A12_HC | . | . | . | . | . | R | . | . | . | . | . | . | . | . | . | . | . | . | . | . | . | . | . | . | . | . |
| 21-225_215B5_HC | . | . | . | . | . | Y | . | . | . | . | . | . | . | . | . | . | . | . | . | . | . | . | . | . | . | . |
| 21-225_215D6_HC | . | . | . | . | . | C | . | . | . | . | . | . | . | . | . | . | . | . | . | . | . | . | . | . | . | . |
| 21-225_217G5_HC | . | . | . | . | . | Y | . | . | . | . | . | . | . | . | . | . | . | . | . | . | . | . | . | . | . | . |
| 21-225_219H1_HC | . | . | . | . | . | R | . | . | . | . | . | . | . | . | . | . | . | . | . | . | . | . | . | . | . | . |
| 21-225_223D11_HC | . | . | . | . | . | R | . | I | . | . | . | . | . | . | . | . | . | . | . | . | . | . | . | . | . | . |
| 21-225_74D1_HC | . | . | . | . | . | E | . | F | . | E | F | D | . | . | . | . | . | . | . | . | . | . | . | . | . | . |
| 21-225_87B7_HC | . | . | . | . | . | F | . | . | . | . | . | . | . | . | . | . | . | . | . | . | . | . | . | . | . | . |

# FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | - | Y | G | L | D | V | W | G | Q | G | T | T | V | T | V | S | S |
| 21-225_190C11_HC | . | . | . | D | . | . | . | . | . | . | . | . | . | . | . | . | . | . | . |
| 21-225_190E10_HC | . | . | . | D | . | . | . | . | . | . | . | . | . | . | . | . | . | . | . |
| 21-225_190E6_HC | . | . | . | D | . | . | . | . | . | . | . | . | . | . | . | . | . | . | . |
| 21-225_190F8_HC | . | . | . | D | . | . | . | . | . | . | . | . | . | . | . | . | . | . | . |
| 21-225_190G11_HC | . | . | . | D | . | . | . | . | . | . | . | . | . | . | . | . | . | . | . |
| 21-225_190G4_HC | . | . | . | D | . | . | . | . | . | . | . | . | . | S | . | . | . | . | . |
| 21-225_191A10_HC | . | . | . | D | . | . | . | . | . | . | . | . | . | S | . | . | . | . | . |
| 21-225_191E10_HC | . | . | . | D | . | . | . | . | . | . | . | . | . | S | . | . | . | . | . |
| 21-225_191E8_HC | . | . | . | . | A | M | . | . | . | . | . | . | . | . | . | . | . | . | . |
| 21-225_191G1_HC | . | . | . | . | . | . | . | . | . | . | . | . | . | . | . | . | . | . | . |
| 21-225_191G10_HC | . | . | . | D | . | . | . | . | . | . | . | . | . | . | . | . | . | . | . |
| 21-225_191G12_HC | . | . | . | D | . | . | . | . | . | . | . | . | . | . | . | . | . | . | . |
| 21-225_192C8_HC | . | . | . | . | . | M | . | . | . | . | . | . | . | . | . | . | . | . | . |
| 21-225_192D2_HC | . | . | . | . | . | M | . | . | . | . | . | . | . | . | . | . | . | . | . |
| 21-225_192D3_HC | . | . | . | . | . | . | . | . | . | . | . | . | . | . | . | . | . | . | . |
| 21-225_192G6_HC | . | . | . | D | . | . | . | . | . | . | . | . | . | . | . | . | . | . | . |
| 21-225_192G8_HC | . | . | . | D | . | . | . | . | . | . | . | . | . | . | . | . | . | . | . |
| 21-225_192H10_HC | . | . | . | D | . | . | . | . | . | . | . | . | . | S | . | . | . | . | . |
| 21-225_192H4_HC | . | . | . | . | . | M | . | . | . | . | . | . | . | . | . | . | . | . | . |
| 21-225_193A10_HC | . | . | . | . | . | . | . | . | . | . | . | . | . | . | . | . | . | . | . |
| 21-225_193C7_HC | . | . | . | D | . | . | . | . | . | . | . | . | . | S | . | . | . | . | . |
| 21-225_194H11_HC | . | . | . | . | . | . | . | . | . | . | . | . | . | . | . | . | . | . | . |
| 21-225_195A9_HC | . | . | . | . | . | . | . | . | . | . | . | . | . | . | . | . | . | . | . |
| 21-225_196C7_HC | . | . | . | . | . | . | . | . | . | . | . | . | . | . | . | . | . | . | . |
| 21-225_197B6_HC | . | . | . | D | . | . | . | . | . | . | . | . | . | S | . | . | . | . | . |
| 21-225_197D3_HC | . | . | . | . | . | M | . | . | . | . | . | . | . | . | . | . | . | . | . |
| 21-225_197F2_HC | . | . | . | D | . | . | . | . | . | . | . | . | . | . | . | . | . | . | . |
| 21-225_197G12_HC | . | . | . | D | . | . | . | . | . | . | . | . | . | . | . | . | . | . | . |
| 21-225_198B6_HC | . | . | . | . | . | M | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | Y | G | L | D | V | W | G | Q | G | T | T | V | T | V | S | S |
| 21-225_199A6_HC | . | . | . | . | A | M | . | . | . | . | . | . | . | . | . | . | . | . | . |
| 21-225_199C5_HC | . | . | . | . | . | M | . | . | . | . | . | . | . | . | . | . | . | . | . |
| 21-225_204G6_HC | . | . | . | D | . | M | . | . | . | . | . | . | . | . | . | . | . | . | . |
| 21-225_205F5_HC | . | . | . | . | . | M | . | . | . | . | . | . | . | . | . | . | . | . | . |
| 21-225_207G6_HC | . | . | . | . | . | I | . | . | . | . | . | . | . | . | . | . | . | . | . |
| 21-225_211A11_HC | . | . | . | . | . | T | . | . | . | . | . | . | . | . | . | . | . | . | . |
| 21-225_211A8_HC | . | . | . | . | . | M | . | . | . | . | . | . | . | . | . | . | . | . | . |
| 21-225_212D7_HC | . | . | . | . | . | T | . | . | . | . | . | . | . | . | . | . | . | . | . |
| 21-225_212E10_HC | . | . | . | . | . | T | . | . | . | . | . | . | . | . | . | . | . | . | . |
| 21-225_212E6_HC | . | . | . | . | . | T | . | . | . | . | . | . | . | . | . | . | . | . | . |
| 21-225_212F10_HC | . | . | . | N | . | M | . | . | . | . | . | . | . | . | . | . | . | . | . |
| 21-225_212H9_HC | . | . | . | . | . | M | . | . | . | . | . | . | . | . | . | . | . | . | . |
| 21-225_213B8_HC | . | . | . | . | . | T | . | . | . | . | . | . | . | . | . | . | . | . | . |
| 21-225_213C4_HC | . | . | . | D | . | M | . | . | . | . | . | . | . | . | . | . | . | . | . |
| 21-225_213D2_HC | . | . | . | . | . | T | . | . | . | . | . | . | . | . | . | . | . | . | . |
| 21-225_214C3_HC | . | . | . | D | . | M | . | . | . | . | . | . | . | . | . | . | . | . | . |
| 21-225_215A12_HC | . | . | . | . | . | M | . | . | . | . | . | . | . | . | . | . | . | . | . |
| 21-225_215B5_HC | . | . | . | . | . | T | . | . | . | . | . | . | . | . | . | . | . | . | . |
| 21-225_215D6_HC | . | . | . | . | . | T | . | . | . | . | . | . | . | . | . | . | . | . | . |
| 21-225_217G5_HC | . | . | . | . | . | . | . | . | . | . | . | . | . | . | . | . | . | . | . |
| 21-225_219H1_HC | . | . | . | N | . | M | . | . | . | . | . | . | . | . | . | . | . | . | . |
| 21-225_223D11_HC | . | . | . | N | D | M | . | . | . | . | . | . | . | . | . | . | . | . | . |
| 21-225_74D1_HC | Y | Y | N | . | . | M | . | . | . | . | . | . | . | . | . | . | . | . | . |
| 21-225_87B7_HC | . | . | . | . | . | M | . | . | . | . | . | . | . | . | . | . | . | . | . |

**FIGURE 57**

Table 55. Consensus 21- VH1|1-02/D1|1-1|RF1/JH4 (SEQ ID NO: 50272):

QVQLVQS-GAEVKKPGASVKVSCKASG-YTFTG-----YYMHWVRQAPGQGLEWMGWINPN---SGGTNYAQKFQGRVTMTRDTSISTAYMELSRLRSDDTAVYYCARDGTS----------------------SFDYWGQGTLVTVSS wherein:

G at position 33 can be substituted with D, S or A

Y at position 39 can be substituted with D

Y at position 40 can be substituted with H, N or F

M at position 41 can be substituted with L or I

H at position 42 can be substituted with Q

I at position 58 can be substituted with V

N at position 59 can be substituted with H, K or S

N at position 61 can be substituted with K

S at position 65 can be substituted with N, R or T

G at position 66 can be substituted with N or D

G at position 67 can be substituted with A

T at position 68 can be substituted with S

N at position 69 can be substituted with H, Q or I

Y at position 70 can be substituted with S or F

A at position 71 can be substituted with T

K at position 73 can be substituted with R, N, E or S

G at position 76 can be substituted with D

**FIGURE 57**

D at position 109 can be substituted with K, G, S or E

G at position 110 can be substituted with F, A, K or V

T at position 111 can be substituted with null (-)or P

S at position 112 can be substituted with G, null (-, or T

null (-) at position 113 can be substituted with V or S

null (-) at position 114 can be substituted with A

null (-) at position 115 can be substituted with T

null (-) at position 133 can be substituted with W

null (-) at position 134 can be substituted with G

S at position 135 can be substituted with null (-), V or Y

F at position 136 can be substituted with null (-), L or Y

D at position 137 can be substituted with G or K

Y at position 138 can be substituted with D or F

Xaa Xaa Xaa Xaa Xaa (SEQ ID NO: 50019)

Trp Xaa Xaa Pro Xaa Xaa Xaa Xaa Xaa Xaa Xaa Xaa Gln Xaa Phe Gln Xaa (SEQ ID NO: 50020)

Xaa Xaa Xaa Xaa Xaa Xaa Xaa Xaa Xaa Xaa Xaa Xaa Xaa (SEQ ID NO: 50021)

Gly Tyr Tyr Met His (SEQ ID NO: 50486)

Trp Ile Asn Pro Asn Ser Gly Gly Thr Asn Tyr Ala Gln Lys Phe Gln Gly (SEQ ID NO: 50487)

Asp Gly Thr Ser Ser Phe Asp Tyr (SEQ ID NO: 50488)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 |
|---|---|---|---|---|---|---|---|---|---|---|---|---|---|---|---|---|---|---|---|---|---|---|---|---|---|---|---|---|
| | H_FR1 | | | | | | | | | | | | | | | | | | | | | | | | | | | |
| CONSENSUS | Q | V | Q | L | V | Q | S | - | G | A | E | V | K | K | P | G | A | S | V | K | V | S | C | K | A | S | G | - |
| 21-225_10G6_HC | . | . | . | . | . | . | . | . | . | . | . | . | . | . | . | . | . | . | . | . | . | . | . | . | . | . | . | . |
| 21-225_12E9_HC | . | . | . | . | . | . | . | . | . | . | . | . | . | . | . | . | . | . | . | . | . | . | . | . | . | . | . | . |
| 21-225_169C10_HC | . | . | . | . | . | . | . | . | . | . | . | . | . | . | . | . | . | . | . | R | . | . | . | . | . | . | . | . |
| 21-225_170D6_HC | . | . | . | . | . | . | . | . | . | . | . | . | . | . | . | . | . | . | . | R | . | . | . | . | . | . | . | . |
| 21-225_171H12_HC | . | . | . | . | . | . | . | . | . | . | . | . | . | . | . | . | . | . | . | R | . | . | . | . | . | . | . | . |
| 21-225_177A5_HC | . | . | . | . | . | . | . | . | . | . | . | . | . | . | . | . | . | . | . | R | . | . | . | . | . | . | . | . |
| 21-225_181G2_HC | . | . | . | . | . | . | . | . | . | . | . | . | . | T | . | . | . | . | . | . | . | . | . | R | . | . | . | . |
| 21-225_184E7_HC | . | . | . | . | . | . | . | . | . | . | . | . | . | T | . | . | . | . | . | . | . | . | . | R | . | . | . | . |
| 21-225_185E10_HC | . | . | . | . | . | . | . | . | . | . | . | . | . | T | . | . | . | . | . | . | . | . | . | R | . | . | . | . |
| 21-225_188B9_HC | . | . | . | . | . | . | . | . | . | . | . | . | . | T | . | . | . | . | . | . | . | . | . | R | . | . | . | . |
| 21-225_188H5_HC | . | . | . | . | . | . | . | . | . | . | . | . | . | T | . | . | . | . | . | . | . | . | . | R | . | . | . | . |
| 21-225_18D4_HC | . | . | . | . | . | . | . | . | . | . | . | . | . | . | . | . | . | . | . | . | . | . | . | . | . | . | . | . |
| 21-225_213H7_HC | . | . | . | . | . | . | . | . | . | . | . | . | . | . | . | . | . | . | . | . | . | . | . | . | . | . | . | . |
| 21-225_221A6_HC | . | . | . | . | . | . | . | . | . | . | . | . | . | . | . | . | . | . | . | . | . | . | . | . | T | . | . | . |
| 21-225_221F12_HC | . | . | . | . | . | . | . | . | . | . | . | . | . | . | . | . | . | . | . | . | . | . | . | . | T | . | . | . |
| 21-225_222C12_HC | . | . | . | . | . | . | . | . | . | . | . | . | . | . | . | . | . | . | . | . | . | . | . | . | T | . | . | . |
| 21-225_222E1_HC | . | . | . | . | . | . | . | . | . | . | . | . | . | . | . | . | . | . | . | . | . | . | . | . | T | . | . | . |
| 21-225_222F7_HC | . | . | . | . | . | . | . | . | . | . | . | . | . | . | . | . | . | . | . | . | . | . | . | . | T | . | . | . |
| 21-225_23B11_HC | . | . | . | . | . | . | . | . | . | . | . | . | . | . | . | . | . | . | . | . | . | . | . | . | . | . | . | . |
| 21-225_30A11_HC | . | . | . | . | . | . | . | . | . | . | . | . | . | . | . | . | . | . | . | . | . | . | . | . | . | . | . | . |
| 21-225_31C4_HC | . | . | . | . | . | . | . | . | . | T | . | . | . | . | . | . | . | . | . | . | . | . | . | . | . | . | . | . |
| 21-225_33G1_HC | . | . | . | . | A | . | . | . | . | . | . | . | . | . | . | . | . | . | . | . | . | . | . | . | . | . | . | . |
| 21-225_33H7_HC | . | . | . | . | . | . | . | . | . | . | D | . | . | . | . | . | . | . | . | . | . | . | . | . | . | . | . | . |
| 21-225_34F3_HC | . | . | . | . | . | . | . | . | . | . | . | . | . | . | . | . | . | . | . | . | . | . | . | . | . | . | . | . |
| 21-225_34G7_HC | . | . | . | . | . | . | . | . | . | . | . | . | . | . | . | . | . | . | . | . | . | . | . | R | . | . | . | . |
| 21-225_35C6_HC | . | . | . | . | . | . | . | . | . | T | . | . | . | . | . | . | . | . | . | . | . | . | . | . | . | . | . | . |
| 21-225_35G6_HC | . | . | . | . | . | . | . | . | . | . | D | . | . | . | . | . | . | . | . | . | . | . | . | . | . | . | . | . |
| 21-225_35H12_HC | . | . | . | . | . | . | . | . | . | . | . | . | . | . | . | . | . | . | . | . | . | . | . | . | . | . | . | . |

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 |
|---|---|---|---|---|---|---|---|---|---|---|---|---|---|---|---|---|---|---|---|---|---|---|---|---|---|---|---|---|
| | H_FR1 | | | | | | | | | | | | | | | | | | | | | | | | | | | |
| CONSENSUS | Q | V | Q | L | V | Q | S | - | G | A | E | V | K | K | P | G | A | S | V | K | V | S | C | K | A | S | G | - |
| 21-225_36H5_HC | . | . | . | . | . | . | . | . | . | . | . | . | . | . | . | . | . | . | . | . | . | . | . | . | . | . | . | . |
| 21-225_49F10_HC | . | . | . | . | . | . | . | . | . | . | . | . | . | . | . | . | . | . | . | . | . | . | . | . | . | . | . | . |
| 21-225_4H6_HC | . | . | . | . | . | . | . | . | . | . | . | . | . | . | . | . | . | . | . | . | . | . | . | . | . | . | . | . |
| 21-225_4H6.004_HC | . | . | . | . | . | . | . | . | . | . | . | . | . | . | . | . | . | . | . | . | . | . | . | . | . | . | . | . |
| 21-225_4H6.005_HC | . | . | . | . | . | . | . | . | . | . | . | . | . | . | . | . | . | . | . | . | . | . | . | . | . | . | . | . |
| 21-225_4H6.006_HC | . | . | . | . | . | . | . | . | . | . | . | . | . | . | . | . | . | . | . | . | . | . | . | . | . | . | . | . |
| 21-225_4H6.007_HC | . | . | . | . | . | . | . | . | . | . | . | . | . | . | . | . | . | . | . | . | . | . | . | . | . | . | . | . |
| 21-225_4H6.008_HC | . | . | . | . | . | . | . | . | . | . | . | . | . | . | . | . | . | . | . | . | . | . | . | . | . | . | . | . |
| 21-225_4H6.009_HC | . | . | . | . | . | . | . | . | . | . | . | . | . | . | . | . | . | . | . | . | . | . | . | . | . | . | . | . |
| 21-225_4H6.010_HC | . | . | . | . | . | . | . | . | . | . | . | . | . | . | . | . | . | . | . | . | . | . | . | . | . | . | . | . |
| 21-225_4H6.011_HC | . | . | . | . | . | . | . | . | . | . | . | . | . | . | . | . | . | . | . | . | . | . | . | . | . | . | . | . |
| 21-225_4H6.014_HC | . | . | . | . | . | . | . | . | . | . | . | . | . | . | . | . | . | . | . | . | . | . | . | . | . | . | . | . |
| 21-225_50D4_HC | . | . | . | . | . | . | . | . | . | . | . | . | . | . | . | . | . | . | . | . | . | . | . | . | . | . | . | . |
| 21-225_51E9_HC | . | . | . | . | . | . | . | . | . | . | . | . | . | . | . | . | . | . | . | . | . | . | . | . | . | . | . | . |
| 21-225_52B1_HC | . | . | . | . | . | . | . | . | . | . | . | . | . | . | . | . | . | . | . | . | . | . | . | . | . | . | . | . |
| 21-225_52G11_HC | . | . | . | . | . | . | . | . | . | . | . | . | . | . | . | . | . | . | . | . | . | . | . | . | . | . | . | . |
| 21-225_52H10_HC | . | . | . | . | . | . | . | . | . | . | . | . | . | . | . | . | . | . | . | . | . | . | . | . | . | . | . | . |
| 21-225_53F2_HC | . | . | . | . | . | . | . | . | . | . | . | . | . | . | . | . | . | . | . | . | . | . | . | . | . | . | . | . |
| 21-225_53F7_HC | . | . | . | . | . | . | . | . | . | . | . | . | . | . | . | . | . | . | . | . | . | . | . | . | . | . | . | . |
| 21-225_56E5_HC | . | . | . | . | . | . | . | . | . | . | . | . | . | . | . | . | . | . | . | . | . | . | . | Q | . | . | . | . |
| 21-225_7C3_LC1_HC | . | . | . | . | . | . | . | . | . | . | . | . | . | . | . | . | . | . | . | . | . | . | . | . | . | . | . | . |
| 21-225_7C3_LC2_HC | . | . | . | . | . | . | . | . | . | . | . | . | . | . | . | . | . | . | . | . | . | . | . | . | . | . | . | . |
| 21-225_9F12_LC1_HC | . | . | . | . | . | . | . | . | . | . | . | . | . | . | . | . | . | . | . | . | . | . | . | . | . | . | . | . |
| 21-225_9F12_LC2_HC | . | . | . | . | . | . | . | . | . | . | . | . | . | . | . | . | . | . | . | . | . | . | . | . | . | . | . | . |

FIGURE 57

| Reference # | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 |
|---|---|---|---|---|---|---|---|---|---|---|---|---|---|---|---|---|---|---|---|---|---|---|---|---|---|---|---|---|
| | | | | | H_CDR1 | | | | | | | | | | H_FR2 | | | | | | | | | | | | | |
| CONSENSUS | Y | T | F | T | G | - | - | - | - | - | Y | Y | M | H | W | V | R | Q | A | P | G | Q | G | L | E | W | M | G |
| 21-225_10G6_HC | . | . | . | . | A | . | . | . | . | . | . | H | . | . | . | . | . | . | . | . | . | . | . | . | . | . | . | . |
| 21-225_12E9_HC | . | . | . | . | D | . | . | . | . | . | . | . | L | . | . | . | . | . | . | . | . | . | . | . | . | . | . | . |
| 21-225_169C10_HC | . | . | . | . | . | . | . | . | . | . | . | F | . | . | . | . | . | . | . | . | . | . | . | . | . | . | . | . |
| 21-225_170D6_HC | . | . | . | . | . | . | . | . | . | . | . | F | I | . | . | . | . | . | . | . | . | . | . | . | . | . | . | . |
| 21-225_171H12_HC | . | . | . | . | . | . | . | . | . | . | . | F | . | . | . | . | . | . | . | . | . | . | . | . | . | . | . | . |
| 21-225_177A5_HC | . | . | . | . | . | . | . | . | . | . | . | F | . | . | . | . | . | . | . | . | . | . | . | . | . | . | . | . |
| 21-225_181G2_HC | . | . | . | . | S | . | . | . | . | . | . | N | . | . | . | . | . | . | V | . | . | . | . | . | . | . | . | . |
| 21-225_184E7_HC | . | . | . | . | S | . | . | . | . | . | . | N | . | . | . | . | . | . | V | . | . | . | . | . | . | . | . | . |
| 21-225_185E10_HC | . | . | . | . | S | . | . | . | . | . | . | N | . | . | . | . | . | . | V | . | . | . | . | . | . | . | . | . |
| 21-225_188B9_HC | . | . | . | . | S | . | . | . | . | . | . | N | . | . | . | . | . | . | V | . | . | . | . | . | . | . | . | . |
| 21-225_188H5_HC | . | . | . | . | S | . | . | . | . | . | . | N | . | . | . | . | . | . | V | . | . | . | . | . | . | . | . | . |
| 21-225_18D4_HC | . | . | . | . | . | . | . | . | . | . | . | . | L | . | . | . | . | . | . | . | . | . | . | . | . | . | L | . |
| 21-225_213H7_HC | . | . | . | . | . | . | . | . | . | . | . | H | . | . | . | . | . | . | . | . | . | . | . | . | . | . | . | . |
| 21-225_221A6_HC | . | . | . | . | . | . | . | . | . | . | . | . | . | . | . | . | . | . | . | . | . | . | . | . | . | . | . | . |
| 21-225_221F12_HC | . | . | . | . | . | . | . | . | . | . | . | . | . | . | . | . | . | . | . | . | . | . | . | . | . | . | . | . |
| 21-225_222C12_HC | . | . | . | . | . | . | . | . | . | . | . | . | . | . | . | . | . | . | . | . | . | . | . | . | . | . | . | . |
| 21-225_222E1_HC | . | . | . | . | . | . | . | . | . | . | . | . | . | . | . | . | . | . | . | . | . | . | . | . | . | . | . | . |
| 21-225_222F7_HC | . | . | . | . | . | . | . | . | . | . | . | . | . | . | . | . | . | . | . | . | . | . | . | . | . | . | . | . |
| 21-225_23B11_HC | . | . | . | . | . | . | . | . | . | . | . | . | L | . | . | . | . | . | . | . | . | . | . | . | . | . | . | . |
| 21-225_30A11_HC | . | . | . | . | D | . | . | . | . | . | . | H | . | . | . | . | . | . | . | . | . | . | . | . | . | . | . | . |
| 21-225_31C4_HC | . | . | . | . | . | . | . | . | . | . | D | . | . | . | . | . | . | . | . | . | . | . | . | . | . | . | . | . |
| 21-225_33G1_HC | . | . | . | . | . | . | . | . | . | . | . | . | . | . | . | . | . | . | . | . | . | . | . | . | . | . | . | . |
| 21-225_33H7_HC | . | . | . | . | D | . | . | . | . | . | . | H | I | . | . | . | . | . | . | . | . | . | . | . | . | . | . | . |
| 21-225_34F3_HC | . | . | . | . | . | . | . | . | . | . | . | H | . | . | . | . | . | . | . | . | . | . | . | . | D | . | . | . |
| 21-225_34G7_HC | . | . | . | . | D | . | . | . | . | . | . | H | I | . | . | . | . | . | . | . | . | . | . | . | . | . | . | . |
| 21-225_35C6_HC | . | . | . | . | . | . | . | . | . | . | D | . | . | . | . | . | . | . | . | . | . | . | . | . | . | . | . | . |
| 21-225_35G6_HC | . | . | . | . | D | . | . | . | . | . | . | H | I | . | . | . | . | . | . | . | . | . | . | . | . | . | . | . |
| 21-225_35H12_HC | . | . | . | . | D | . | . | . | . | . | . | . | I | . | . | . | . | . | . | . | . | . | . | . | . | . | . | . |

FIGURE 57

| Reference # | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 |
|---|---|---|---|---|---|---|---|---|---|---|---|---|---|---|---|---|---|---|---|---|---|---|---|---|---|---|---|---|
| | | | | | H_CDR1 | | | | | | | | | | H_FR2 | | | | | | | | | | | | | |
| CONSENSUS | Y | T | F | T | G | - | - | - | - | - | Y | Y | M | H | W | V | R | Q | A | P | G | Q | G | L | E | W | M | G |
| 21-225_36H5_HC | . | . | . | . | D | . | . | . | . | . | . | H | . | . | . | . | . | . | . | . | . | . | . | . | . | . | . | . |
| 21-225_49F10_HC | . | . | . | . | . | . | . | . | . | . | . | H | . | . | . | . | . | . | . | . | . | . | . | . | . | . | . | . |
| 21-225_4H6_HC | . | . | . | . | D | . | . | . | . | . | . | . | L | . | . | . | . | . | . | . | . | . | . | . | . | . | . | . |
| 21-225_4H6.004_HC | . | . | . | . | D | . | . | . | . | . | . | . | L | . | . | . | . | . | . | . | . | . | . | . | . | . | . | . |
| 21-225_4H6.005_HC | . | . | . | . | D | . | . | . | . | . | . | . | L | . | . | . | . | . | . | . | . | . | . | . | . | . | . | . |
| 21-225_4H6.006_HC | . | . | . | . | D | . | . | . | . | . | . | . | L | . | . | . | . | . | . | . | . | . | . | . | . | . | . | . |
| 21-225_4H6.007_HC | . | . | . | . | D | . | . | . | . | . | . | . | L | . | . | . | . | . | . | . | . | . | . | . | . | . | . | . |
| 21-225_4H6.008_HC | . | . | . | . | D | . | . | . | . | . | . | . | L | . | . | . | . | . | . | . | . | . | . | . | . | . | . | . |
| 21-225_4H6.009_HC | . | . | . | . | D | . | . | . | . | . | . | . | L | . | . | . | . | . | . | . | . | . | . | . | . | . | . | . |
| 21-225_4H6.010_HC | . | . | . | . | D | . | . | . | . | . | . | . | L | . | . | . | . | . | . | . | . | . | . | . | . | . | . | . |
| 21-225_4H6.011_HC | . | . | . | . | D | . | . | . | . | . | . | . | L | . | . | . | . | . | . | . | . | . | . | . | . | . | . | . |
| 21-225_4H6.014_HC | . | . | . | . | D | . | . | . | . | . | . | . | L | . | . | . | . | . | . | . | . | . | . | . | . | . | . | . |
| 21-225_50D4_HC | . | . | . | . | . | . | . | . | . | . | . | H | . | . | . | . | . | . | . | . | . | . | . | . | . | . | . | . |
| 21-225_51E9_HC | . | . | . | . | . | . | . | . | . | . | . | H | I | . | . | . | . | . | . | . | . | . | . | . | . | . | . | . |
| 21-225_52B1_HC | . | . | . | . | . | . | . | . | . | . | . | H | . | Q | . | . | . | . | . | . | . | . | . | . | . | . | . | . |
| 21-225_52G11_HC | . | . | . | . | . | . | . | . | . | . | . | . | L | . | . | . | . | . | . | . | . | . | . | . | . | . | . | . |
| 21-225_52H10_HC | . | . | . | . | . | . | . | . | . | . | . | H | . | Q | . | . | . | . | . | . | . | . | . | . | . | . | . | . |
| 21-225_53F2_HC | . | . | . | . | . | . | . | . | . | . | . | . | L | . | . | . | . | . | . | . | . | . | . | . | . | . | . | . |
| 21-225_53F7_HC | . | . | . | . | . | . | . | . | . | . | . | H | . | . | . | . | . | . | . | . | . | . | . | . | . | . | . | . |
| 21-225_56E5_HC | . | . | . | . | . | . | . | . | . | . | . | H | . | . | . | . | . | . | . | . | . | . | . | . | . | . | . | . |
| 21-225_7C3_LC1_HC | . | . | . | N | D | . | . | . | . | . | . | . | L | . | . | . | . | . | . | . | . | . | . | . | . | . | . | . |
| 21-225_7C3_LC2_HC | . | . | . | N | D | . | . | . | . | . | . | . | L | . | . | . | . | . | . | . | . | . | . | . | . | . | . | . |
| 21-225_9F12_LC1_HC | . | . | . | A | D | . | . | . | . | . | . | . | L | . | . | . | . | . | . | . | . | . | . | . | . | . | . | . |
| 21-225_9F12_LC2_HC | . | . | . | A | D | . | . | . | . | . | . | . | L | . | . | . | . | . | . | . | . | . | . | . | . | . | . | . |

FIGURE 57

| Reference # | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 | 81 | 82 | 83 | 84 |
|---|---|---|---|---|---|---|---|---|---|---|---|---|---|---|---|---|---|---|---|---|---|---|---|---|---|---|---|---|
| | H_CDR2 | | | | | | | | | | | | | | | | | | | | | | | | | | | |
| CONSENSUS | W | I | N | P | N | - | - | - | S | G | G | T | N | Y | A | Q | K | F | Q | G | R | V | T | M | T | R | D | T |
| 21-225_10G6_HC | . | . | . | . | . | . | . | . | N | N | . | . | . | . | . | . | . | . | . | . | . | . | . | . | . | . | . | . |
| 21-225_12E9_HC | . | V | H | . | . | . | . | . | . | . | . | . | . | F | . | . | . | . | . | D | . | . | . | . | . | . | . | . |
| 21-225_169C10_HC | . | . | K | . | K | . | . | . | . | . | . | . | . | S | . | . | R | . | . | . | . | . | . | . | . | . | . | . |
| 21-225_170D6_HC | . | . | K | . | . | . | . | . | . | . | . | . | . | S | . | . | R | . | . | . | . | . | . | . | . | . | . | . |
| 21-225_171H12_HC | . | . | K | . | K | . | . | . | . | . | . | . | . | S | . | . | R | . | . | . | . | . | . | . | . | . | . | . |
| 21-225_177A5_HC | . | . | K | . | K | . | . | . | . | . | . | . | . | S | . | . | R | . | . | . | . | . | . | . | . | . | . | . |
| 21-225_181G2_HC | . | . | . | . | . | . | . | . | N | . | . | S | . | . | T | . | . | . | . | . | . | . | . | . | . | . | . | . |
| 21-225_184E7_HC | . | . | . | . | . | . | . | . | N | . | . | S | . | . | T | . | . | . | . | . | . | . | . | . | . | . | . | . |
| 21-225_185E10_HC | . | . | . | . | . | . | . | . | N | . | . | S | . | . | T | . | . | . | . | . | . | . | . | . | . | . | . | . |
| 21-225_188B9_HC | . | . | . | . | . | . | . | . | . | . | . | S | . | . | T | . | . | . | . | . | . | . | . | . | . | . | . | . |
| 21-225_188H5_HC | . | . | . | . | . | . | . | . | . | . | . | S | . | . | T | . | . | . | . | . | . | I | . | . | . | . | . | . |
| 21-225_18D4_HC | . | . | . | . | . | . | . | . | . | N | . | . | I | S | . | . | . | . | . | . | . | . | . | . | . | . | . | . |
| 21-225_213H7_HC | . | . | . | . | K | . | . | . | . | D | . | . | . | . | . | . | . | . | . | . | . | . | . | . | . | . | . | . |
| 21-225_221A6_HC | . | . | H | . | . | . | . | . | . | . | . | . | . | . | . | . | . | . | . | . | . | . | . | L | . | . | . | . |
| 21-225_221F12_HC | . | . | H | . | . | . | . | . | . | . | . | . | . | . | . | . | . | . | . | . | . | . | . | L | . | . | . | . |
| 21-225_222C12_HC | . | . | H | . | . | . | . | . | . | . | . | . | . | . | . | . | . | . | . | . | . | . | . | L | . | . | . | . |
| 21-225_222E1_HC | . | . | H | . | . | . | . | . | . | . | . | . | . | . | . | . | . | . | . | . | . | . | . | L | . | . | . | . |
| 21-225_222F7_HC | . | . | H | . | . | . | . | . | . | . | . | . | . | . | . | . | . | . | . | . | . | . | . | L | . | . | . | . |
| 21-225_23B11_HC | . | . | . | . | . | . | . | . | N | N | . | . | . | . | . | . | . | . | . | . | . | . | . | . | . | . | . | . |
| 21-225_30A11_HC | . | . | . | . | . | . | . | . | K | . | . | . | . | . | . | . | . | . | . | D | . | . | . | . | . | . | . | . |
| 21-225_31C4_HC | . | . | S | . | K | . | . | . | N | . | . | . | . | . | . | . | . | . | . | . | . | . | . | . | . | . | . | . |
| 21-225_33G1_HC | . | . | S | . | . | . | . | . | N | . | . | . | . | . | . | . | . | . | . | . | . | . | . | . | . | . | . | . |
| 21-225_33H7_HC | . | . | . | . | . | . | . | . | N | . | . | . | H | . | . | . | E | . | . | . | . | . | . | . | . | . | . | . |
| 21-225_34F3_HC | . | . | . | . | . | . | . | . | . | . | . | . | . | . | . | . | . | . | . | . | . | . | . | . | . | . | . | . |
| 21-225_34G7_HC | . | . | . | . | . | . | . | . | N | . | . | . | H | . | . | . | . | . | . | . | . | . | . | . | . | . | . | . |
| 21-225_35C6_HC | . | . | S | . | K | . | . | . | N | . | . | . | . | . | . | . | . | . | . | . | . | . | . | . | . | . | . | . |
| 21-225_35G6_HC | . | . | . | . | . | . | . | . | N | . | . | . | H | . | . | . | E | . | . | . | . | . | . | . | . | . | . | . |
| 21-225_35H12_HC | . | . | . | . | . | . | . | . | K | . | . | . | . | . | . | . | . | . | . | . | . | . | . | . | . | . | . | . |

**FIGURE 57**

| Reference # | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 | 81 | 82 | 83 | 84 |
|---|---|---|---|---|---|---|---|---|---|---|---|---|---|---|---|---|---|---|---|---|---|---|---|---|---|---|---|---|
| | | | | | | | | | | H_CDR2 | | | | | | | | | | | | | | | | | | |
| CONSENSUS | W | I | N | P | N | - | - | - | S | G | G | T | N | Y | A | Q | K | F | Q | G | R | V | T | M | T | R | D | T |
| 21-225_36H5_HC | . | . | . | . | . | . | . | . | [illegible] | . | . | . | . | . | . | . | . | . | . | D | . | . | . | . | . | . | . | . |
| 21-225_49F10_HC | . | . | . | . | . | . | . | . | N | N | A | . | . | . | . | . | N | . | . | . | . | . | . | L | . | . | . | . |
| 21-225_4H6_HC | . | . | [illegible] | . | . | . | . | . | . | . | . | . | . | . | . | . | . | . | . | . | . | . | . | . | . | . | . | . |
| 21-225_4H6.004_HC | . | . | [illegible] | . | . | . | . | . | . | . | . | . | . | . | . | . | . | . | . | . | . | . | . | . | . | . | . | . |
| 21-225_4H6.005_HC | . | . | [illegible] | . | . | . | . | . | . | . | . | . | . | . | . | . | . | . | . | . | . | . | . | . | . | . | . | . |
| 21-225_4H6.006_HC | . | . | [illegible] | . | . | . | . | . | . | . | . | . | . | . | . | . | . | . | . | . | . | . | . | . | . | . | . | . |
| 21-225_4H6.007_HC | . | . | [illegible] | . | . | . | . | . | . | . | . | . | . | . | . | . | . | . | . | . | . | . | . | . | . | . | . | . |
| 21-225_4H6.008_HC | . | . | [illegible] | . | . | . | . | . | . | . | . | . | . | . | . | . | . | . | . | . | . | . | . | . | . | . | . | . |
| 21-225_4H6.009_HC | . | . | [illegible] | . | . | . | . | . | . | . | . | . | . | . | . | . | . | . | . | . | . | . | . | . | . | . | . | . |
| 21-225_4H6.010_HC | . | . | [illegible] | . | . | . | . | . | . | . | . | . | . | . | . | . | . | . | . | . | . | . | . | . | . | . | . | . |
| 21-225_4H6.011_HC | . | . | [illegible] | . | . | . | . | . | . | . | . | . | . | . | . | . | . | . | . | . | . | . | . | . | . | . | . | . |
| 21-225_4H6.014_HC | . | . | [illegible] | . | . | . | . | . | . | . | . | . | . | . | . | . | . | . | . | . | . | . | . | . | . | . | . | . |
| 21-225_50D4_HC | . | . | . | . | . | . | . | . | N | N | A | . | . | . | . | . | S | . | . | . | . | . | . | L | . | . | . | . |
| 21-225_51E9_HC | . | . | . | . | . | . | . | . | T | N | . | . | Q | . | . | . | . | . | . | . | . | . | . | . | . | . | . | . |
| 21-225_52B1_HC | . | . | . | . | . | . | . | . | . | . | A | . | . | . | . | . | N | . | . | . | . | . | . | . | . | . | . | . |
| 21-225_52G11_HC | . | . | . | . | . | . | . | . | [illegible] | N | . | . | . | . | . | . | . | . | . | . | . | . | . | . | . | . | . | . |
| 21-225_52H10_HC | . | . | . | . | . | . | . | . | N | . | . | . | Q | . | . | . | . | . | . | . | . | . | . | . | . | . | . | . |
| 21-225_53F2_HC | . | . | . | . | . | . | . | . | [illegible] | N | . | . | . | . | . | . | N | . | . | . | . | . | . | . | . | . | . | . |
| 21-225_53F7_HC | . | . | . | . | . | . | . | . | N | N | . | . | . | . | . | . | . | . | . | . | . | . | . | . | . | . | . | . |
| 21-225_56E5_HC | . | . | . | . | . | . | . | . | N | N | A | . | . | . | . | . | . | . | . | . | . | . | . | . | . | . | . | . |
| 21-225_7C3_LC1_HC | . | . | [illegible] | . | . | . | . | . | . | . | . | . | . | . | . | . | . | . | . | . | . | . | . | . | . | . | . | . |
| 21-225_7C3_LC2_HC | . | . | [illegible] | . | . | . | . | . | . | . | . | . | . | . | . | . | . | . | . | . | . | . | . | . | . | . | . | . |
| 21-225_9F12_LC1_HC | . | . | [illegible] | . | . | . | . | . | . | . | . | . | . | F | . | . | . | . | . | . | . | . | . | . | . | . | . | . |
| 21-225_9F12_LC2_HC | . | . | [illegible] | . | . | . | . | . | . | . | . | . | . | F | . | . | . | . | . | . | . | . | . | . | . | . | . | . |

FIGURE 57

| Reference # | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 | 109 | 110 | 111 | 112 |
|---|---|---|---|---|---|---|---|---|---|---|---|---|---|---|---|---|---|---|---|---|---|---|---|---|---|---|---|---|
| | H_FR3 | | | | | | | | | | | | | | | | | | | | | | | | | | | |
| CONSENSUS | S | I | S | T | A | Y | M | E | L | S | R | L | R | S | D | D | T | A | V | Y | Y | C | A | R | D | G | T | S |
| 21-225_10G6_HC | . | . | . | . | . | . | . | . | . | . | . | . | . | . | . | . | . | . | . | . | . | . | G | . | . | V | . | . |
| 21-225_12E9_HC | . | . | . | . | . | . | . | . | . | . | S | . | . | . | . | . | . | . | I | . | S | . | . | . | . | . | . | . |
| 21-225_169C10_HC | . | . | N | . | . | . | . | . | . | N | . | . | . | . | . | . | . | . | . | . | . | . | . | . | G | . | . | T |
| 21-225_170D6_HC | . | . | N | . | . | . | . | . | . | N | . | . | . | . | . | . | . | . | . | . | . | . | . | . | G | . | . | T |
| 21-225_171H12_HC | . | . | N | . | . | . | . | . | . | N | . | . | . | . | . | . | . | . | . | . | . | . | . | . | G | . | . | T |
| 21-225_177A5_HC | . | . | N | . | . | . | . | . | . | N | W | . | . | . | . | . | . | . | . | . | . | . | . | . | G | . | . | T |
| 21-225_181G2_HC | . | . | . | . | . | . | . | . | . | . | . | . | . | . | . | . | . | . | . | . | . | . | . | . | K | F | - | - |
| 21-225_184E7_HC | . | . | . | . | . | . | . | . | . | . | . | . | . | . | . | . | . | . | . | . | . | . | . | . | K | F | - | - |
| 21-225_185E10_HC | . | . | . | . | . | . | . | . | . | . | . | . | . | . | . | . | . | . | . | . | . | . | . | . | K | F | - | - |
| 21-225_188B9_HC | . | . | . | . | . | . | . | . | . | . | . | . | . | . | . | . | . | . | . | . | . | . | . | . | K | F | - | - |
| 21-225_188H5_HC | . | . | . | . | . | . | . | . | . | . | . | . | . | . | . | . | . | . | . | . | . | . | . | . | K | F | - | - |
| 21-225_18D4_HC | . | . | . | . | . | . | . | . | . | . | . | . | I | . | . | . | . | . | . | . | . | . | . | . | . | . | . | . |
| 21-225_213H7_HC | . | . | . | . | . | . | . | . | . | . | . | . | . | . | . | . | . | . | . | . | . | . | . | . | E | K | P | G |
| 21-225_221A6_HC | . | . | . | . | . | . | . | D | . | . | . | . | . | . | . | . | . | . | . | . | . | . | . | . | . | . | . | . |
| 21-225_221F12_HC | . | . | . | . | . | . | . | D | . | . | . | . | . | . | . | . | . | . | . | . | . | . | . | . | . | . | . | . |
| 21-225_222C12_HC | . | . | . | . | . | . | . | D | . | . | . | . | . | . | . | . | . | . | . | . | . | . | . | . | . | . | . | . |
| 21-225_222E1_HC | . | . | . | . | . | . | . | D | . | . | . | . | . | . | . | . | . | . | . | . | . | . | . | . | . | . | . | . |
| 21-225_222F7_HC | . | . | . | . | . | . | . | D | . | . | . | . | . | . | . | . | . | . | . | . | . | . | . | . | . | . | . | . |
| 21-225_23B11_HC | . | . | . | . | . | . | . | . | . | . | . | . | . | . | . | . | . | . | . | . | . | . | . | . | . | . | . | . |
| 21-225_30A11_HC | . | . | . | . | . | . | . | . | . | . | . | . | . | . | . | . | . | . | . | . | F | . | . | . | . | . | . | G |
| 21-225_31C4_HC | . | . | . | . | V | . | . | . | . | N | . | . | . | . | . | . | . | . | . | . | . | . | . | . | . | . | . | G |
| 21-225_33G1_HC | . | . | . | . | . | . | . | . | . | . | . | . | . | . | . | . | . | . | . | . | . | . | . | . | . | . | . | G |
| 21-225_33H7_HC | . | . | . | . | . | . | . | . | . | . | S | . | . | . | . | . | . | . | . | . | H | . | . | . | . | . | . | G |
| 21-225_34F3_HC | . | . | . | . | . | . | . | . | . | . | . | . | . | . | . | . | . | . | . | . | . | . | . | . | . | . | . | . |
| 21-225_34G7_HC | . | . | . | . | . | . | . | . | . | . | . | . | . | . | . | . | . | . | . | . | . | . | . | . | . | . | . | G |
| 21-225_35C6_HC | . | . | . | . | . | . | . | . | . | N | . | . | . | . | . | . | . | . | . | . | . | . | . | . | . | . | . | G |
| 21-225_35G6_HC | . | . | . | . | . | . | . | . | . | . | S | . | . | . | . | . | . | . | . | . | . | . | . | . | . | . | . | G |
| 21-225_35H12_HC | . | . | . | . | . | . | . | . | . | . | . | . | . | . | . | . | . | . | . | . | . | . | . | . | . | . | . | G |

FIGURE 57

| Reference # | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 | 109 | 110 | 111 | 112 |
|---|---|---|---|---|---|---|---|---|---|---|---|---|---|---|---|---|---|---|---|---|---|---|---|---|---|---|---|---|
| | H_FR3 | | | | | | | | | | | | | | | | | | | | | | | | | | | |
| CONSENSUS | S | I | S | T | A | Y | M | E | L | S | R | L | R | S | D | D | T | A | V | Y | Y | C | A | R | D | G | T | S |
| 21-225_36H5_HC | . | . | . | . | . | . | . | . | . | . | . | . | . | . | . | . | . | . | . | . | F | . | . | . | . | . | . | G |
| 21-225_49F10_HC | . | . | . | . | . | . | . | . | . | . | . | . | . | . | . | . | . | . | . | . | . | . | . | . | . | . | . | . |
| 21-225_4H6_HC | . | . | . | . | . | . | . | G | . | . | S | . | . | . | . | . | . | . | . | . | . | . | . | . | . | . | . | . |
| 21-225_4H6.004_HC | . | . | . | . | . | . | . | . | . | . | S | . | . | . | . | . | . | . | . | . | . | . | . | . | . | . | . | . |
| 21-225_4H6.005_HC | . | . | . | . | . | . | . | . | . | . | S | . | . | . | . | . | . | . | . | . | . | . | . | . | . | . | . | . |
| 21-225_4H6.006_HC | . | . | . | . | . | . | . | . | . | . | S | . | . | . | . | . | . | . | . | . | . | . | . | . | . | . | . | . |
| 21-225_4H6.007_HC | . | . | . | . | . | . | . | . | . | . | S | . | . | . | . | . | . | . | . | . | . | . | . | . | . | A | . | . |
| 21-225_4H6.008_HC | . | . | . | . | . | . | . | . | . | . | S | . | . | . | . | . | . | . | . | . | . | . | . | . | S | . | . | . |
| 21-225_4H6.009_HC | . | . | . | . | . | . | . | . | . | . | S | . | . | . | . | . | . | . | . | . | . | . | . | . | S | . | . | . |
| 21-225_4H6.010_HC | . | . | . | . | . | . | . | . | . | . | S | . | . | . | . | . | . | . | . | . | . | . | . | . | . | A | . | . |
| 21-225_4H6.011_HC | . | . | . | . | . | . | . | . | . | . | S | . | . | . | . | . | . | . | . | . | . | . | . | . | . | A | . | . |
| 21-225_4H6.014_HC | . | . | . | . | . | . | . | G | . | . | S | . | . | . | . | . | . | . | . | . | . | . | . | . | . | A | . | . |
| 21-225_50D4_HC | . | . | . | . | . | . | . | . | . | . | . | . | . | . | . | . | . | . | . | . | . | . | . | . | . | . | . | . |
| 21-225_51E9_HC | . | . | . | . | . | . | . | . | . | . | . | . | . | . | . | . | . | . | . | . | . | . | . | . | . | . | . | . |
| 21-225_52B1_HC | . | . | . | . | . | . | L | D | . | . | . | . | . | . | . | . | . | . | . | . | . | . | . | . | . | . | . | . |
| 21-225_52G11_HC | . | . | . | . | . | F | . | . | . | . | . | . | . | . | . | . | . | . | . | . | . | . | . | . | . | . | . | . |
| 21-225_52H10_HC | . | . | . | . | . | . | . | . | . | . | . | . | . | . | . | . | . | . | . | . | . | . | . | . | . | . | . | . |
| 21-225_53F2_HC | . | . | . | . | . | . | . | . | . | . | . | . | K | . | . | . | . | . | . | . | . | . | . | . | . | . | . | . |
| 21-225_53F7_HC | . | . | . | . | . | . | . | . | . | N | . | . | T | . | . | . | . | . | . | . | . | . | . | . | . | . | . | . |
| 21-225_56E5_HC | . | . | . | . | . | . | . | . | . | R | . | . | . | . | . | . | . | . | . | . | H | . | . | . | . | . | . | . |
| 21-225_7C3_LC1_HC | . | . | . | . | . | . | . | . | . | . | S | . | . | . | . | . | . | . | . | . | S | . | . | . | . | . | . | . |
| 21-225_7C3_LC2_HC | . | . | . | . | . | . | . | . | . | . | S | . | . | . | . | . | . | . | . | . | S | . | . | . | . | . | . | . |
| 21-225_9F12_LC1_HC | . | . | . | . | . | . | L | . | . | . | S | . | . | . | . | . | . | . | F | . | . | . | . | . | . | . | . | . |
| 21-225_9F12_LC2_HC | . | . | . | . | . | . | L | . | . | . | S | . | . | . | . | . | . | . | F | . | . | . | . | . | . | . | . | . |

FIGURE 57

| Reference # | 113 | 114 | 115 | 116 | 117 | 118 | 119 | 120 | 121 | 122 | 123 | 124 | 125 | 126 | 127 | 128 | 129 | 130 | 131 | 132 | 133 | 134 | 135 | 136 | 137 | 138 | 139 | 140 |
|---|---|---|---|---|---|---|---|---|---|---|---|---|---|---|---|---|---|---|---|---|---|---|---|---|---|---|---|---|
| | H_CDR3 | | | | | | | | | | | | | | | | | | | | | | | | | | | |
| CONSENSUS | - | - | - | - | - | - | - | - | - | - | - | - | - | - | - | - | - | - | - | - | - | - | S | F | D | Y | W | G |
| 21-225_10G6_HC | . | . | . | . | . | . | . | . | . | . | . | . | . | . | . | . | . | . | . | . | . | . | . | . | . | . | . | . |
| 21-225_12E9_HC | . | . | . | . | . | . | . | . | . | . | . | . | . | . | . | . | . | . | . | . | . | . | . | . | . | . | . | . |
| 21-225_169C10_HC | V | A | T | . | . | . | . | . | . | . | . | . | . | . | . | . | . | . | . | . | W | G | V | . | . | . | . | . |
| 21-225_170D6_HC | V | A | T | . | . | . | . | . | . | . | . | . | . | . | . | . | . | . | . | . | W | G | V | . | . | . | . | . |
| 21-225_171H12_HC | V | A | T | . | . | . | . | . | . | . | . | . | . | . | . | . | . | . | . | . | W | G | V | . | . | . | . | . |
| 21-225_177A5_HC | V | A | T | . | . | . | . | . | . | . | . | . | . | . | . | . | . | . | . | . | W | G | V | . | . | . | . | . |
| 21-225_181G2_HC | . | . | . | . | . | . | . | . | . | . | . | . | . | . | . | . | . | . | . | . | . | . | - | - | G | D | . | . |
| 21-225_184E7_HC | . | . | . | . | . | . | . | . | . | . | . | . | . | . | . | . | . | . | . | . | . | . | - | - | G | D | . | . |
| 21-225_185E10_HC | . | . | . | . | . | . | . | . | . | . | . | . | . | . | . | . | . | . | . | . | . | . | - | - | G | D | . | . |
| 21-225_188B9_HC | . | . | . | . | . | . | . | . | . | . | . | . | . | . | . | . | . | . | . | . | . | . | - | - | G | D | . | . |
| 21-225_188H5_HC | . | . | . | . | . | . | . | . | . | . | . | . | . | . | . | . | . | . | . | . | . | . | - | - | G | D | . | . |
| 21-225_18D4_HC | . | . | . | . | . | . | . | . | . | . | . | . | . | . | . | . | . | . | . | . | . | . | . | L | . | . | . | . |
| 21-225_213H7_HC | S | . | . | . | . | . | . | . | . | . | . | . | . | . | . | . | . | . | . | . | . | . | Y | Y | K | . | . | . |
| 21-225_221A6_HC | . | . | . | . | . | . | . | . | . | . | . | . | . | . | . | . | . | . | . | . | . | . | . | . | . | . | . | . |
| 21-225_221F12_HC | . | . | . | . | . | . | . | . | . | . | . | . | . | . | . | . | . | . | . | . | . | . | . | . | . | . | . | . |
| 21-225_222C12_HC | . | . | . | . | . | . | . | . | . | . | . | . | . | . | . | . | . | . | . | . | . | . | . | . | . | . | . | . |
| 21-225_222E1_HC | . | . | . | . | . | . | . | . | . | . | . | . | . | . | . | . | . | . | . | . | . | . | . | . | . | . | . | . |
| 21-225_222F7_HC | . | . | . | . | . | . | . | . | . | . | . | . | . | . | . | . | . | . | . | . | . | . | . | . | . | . | . | . |
| 21-225_23B11_HC | . | . | . | . | . | . | . | . | . | . | . | . | . | . | . | . | . | . | . | . | . | . | . | . | . | . | . | . |
| 21-225_30A11_HC | . | . | . | . | . | . | . | . | . | . | . | . | . | . | . | . | . | . | . | . | . | . | . | . | . | . | . | . |
| 21-225_31C4_HC | . | . | . | . | . | . | . | . | . | . | . | . | . | . | . | . | . | . | . | . | . | . | . | . | . | . | . | . |
| 21-225_33G1_HC | . | . | . | . | . | . | . | . | . | . | . | . | . | . | . | . | . | . | . | . | . | . | . | . | . | . | . | . |
| 21-225_33H7_HC | . | . | . | . | . | . | . | . | . | . | . | . | . | . | . | . | . | . | . | . | . | . | . | . | . | . | . | . |
| 21-225_34F3_HC | . | . | . | . | . | . | . | . | . | . | . | . | . | . | . | . | . | . | . | . | . | . | . | . | . | . | . | . |
| 21-225_34G7_HC | . | . | . | . | . | . | . | . | . | . | . | . | . | . | . | . | . | . | . | . | . | . | . | . | . | . | . | . |
| 21-225_35C6_HC | . | . | . | . | . | . | . | . | . | . | . | . | . | . | . | . | . | . | . | . | . | . | . | . | . | . | . | . |
| 21-225_35G6_HC | . | . | . | . | . | . | . | . | . | . | . | . | . | . | . | . | . | . | . | . | . | . | . | . | . | . | . | . |
| 21-225_35H12_HC | . | . | . | . | . | . | . | . | . | . | . | . | . | . | . | . | . | . | . | . | . | . | . | . | . | . | . | . |

**FIGURE 57**

| Reference # | 113 | 114 | 115 | 116 | 117 | 118 | 119 | 120 | 121 | 122 | 123 | 124 | 125 | 126 | 127 | 128 | 129 | 130 | 131 | 132 | 133 | 134 | 135 | 136 | 137 | 138 | 139 | 140 |
|---|---|---|---|---|---|---|---|---|---|---|---|---|---|---|---|---|---|---|---|---|---|---|---|---|---|---|---|---|
| | H_CDR3 | | | | | | | | | | | | | | | | | | | | | | | | | | | |
| CONSENSUS | - | - | - | - | - | - | - | - | - | - | - | - | - | - | - | - | - | - | - | - | - | - | S | F | D | Y | W | G |
| 21-225_36H5_HC | . | . | . | . | . | . | . | . | . | . | . | . | . | . | . | . | . | . | . | . | . | . | . | . | . | . | . | . |
| 21-225_49F10_HC | . | . | . | . | . | . | . | . | . | . | . | . | . | . | . | . | . | . | . | . | . | . | . | . | . | . | . | . |
| 21-225_4H6_HC | . | . | . | . | . | . | . | . | . | . | . | . | . | . | . | . | . | . | . | . | . | . | . | . | . | . | . | . |
| 21-225_4H6.004_HC | . | . | . | . | . | . | . | . | . | . | . | . | . | . | . | . | . | . | . | . | . | . | . | . | . | . | . | . |
| 21-225_4H6.005_HC | . | . | . | . | . | . | . | . | . | . | . | . | . | . | . | . | . | . | . | . | . | . | . | . | . | . | . | . |
| 21-225_4H6.006_HC | . | . | . | . | . | . | . | . | . | . | . | . | . | . | . | . | . | . | . | . | . | . | . | . | . | . | . | . |
| 21-225_4H6.007_HC | . | . | . | . | . | . | . | . | . | . | . | . | . | . | . | . | . | . | . | . | . | . | . | . | . | . | . | . |
| 21-225_4H6.008_HC | . | . | . | . | . | . | . | . | . | . | . | . | . | . | . | . | . | . | . | . | . | . | . | . | . | . | . | . |
| 21-225_4H6.009_HC | . | . | . | . | . | . | . | . | . | . | . | . | . | . | . | . | . | . | . | . | . | . | . | . | . | . | . | . |
| 21-225_4H6.010_HC | . | . | . | . | . | . | . | . | . | . | . | . | . | . | . | . | . | . | . | . | . | . | . | . | . | . | . | . |
| 21-225_4H6.011_HC | . | . | . | . | . | . | . | . | . | . | . | . | . | . | . | . | . | . | . | . | . | . | . | . | . | . | . | . |
| 21-225_4H6.014_HC | . | . | . | . | . | . | . | . | . | . | . | . | . | . | . | . | . | . | . | . | . | . | . | . | . | . | . | . |
| 21-225_50D4_HC | . | . | . | . | . | . | . | . | . | . | . | . | . | . | . | . | . | . | . | . | . | . | . | . | . | . | . | . |
| 21-225_51E9_HC | . | . | . | . | . | . | . | . | . | . | . | . | . | . | . | . | . | . | . | . | . | . | . | . | . | . | . | . |
| 21-225_52B1_HC | . | . | . | . | . | . | . | . | . | . | . | . | . | . | . | . | . | . | . | . | . | . | . | . | . | . | . | . |
| 21-225_52G11_HC | . | . | . | . | . | . | . | . | . | . | . | . | . | . | . | . | . | . | . | . | . | . | . | . | . | . | . | . |
| 21-225_52H10_HC | . | . | . | . | . | . | . | . | . | . | . | . | . | . | . | . | . | . | . | . | . | . | . | . | . | . | . | . |
| 21-225_53F2_HC | . | . | . | . | . | . | . | . | . | . | . | . | . | . | . | . | . | . | . | . | . | . | . | . | . | . | . | . |
| 21-225_53F7_HC | . | . | . | . | . | . | . | . | . | . | . | . | . | . | . | . | . | . | . | . | . | . | . | . | . | . | . | . |
| 21-225_56E5_HC | . | . | . | . | . | . | . | . | . | . | . | . | . | . | . | . | . | . | . | . | . | . | . | . | . | . | . | . |
| 21-225_7C3_LC1_HC | . | . | . | . | . | . | . | . | . | . | . | . | . | . | . | . | . | . | . | . | . | . | . | . | . | . | . | . |
| 21-225_7C3_LC2_HC | . | . | . | . | . | . | . | . | . | . | . | . | . | . | . | . | . | . | . | . | . | . | . | . | . | . | . | . |
| 21-225_9F12_LC1_HC | . | . | . | . | . | . | . | . | . | . | . | . | . | . | . | . | . | . | . | . | . | . | . | . | . | . | . | . |
| 21-225_9F12_LC2_HC | . | . | . | . | . | . | . | . | . | . | . | . | . | . | . | . | . | . | . | . | . | . | . | . | . | . | . | . |

FIGURE 57

| Reference # | 141 | 142 | 143 | 144 | 145 | 146 | 147 | 148 | 149 |
|---|---|---|---|---|---|---|---|---|---|
| | H_FR4 | | | | | | | | |
| CONSENSUS | Q | G | T | L | V | T | V | S | S |
| 21-225_10G6_HC | . | . | . | . | . | . | . | . | . |
| 21-225_12E9_HC | . | . | . | . | . | . | . | . | . |
| 21-225_169C10_HC | . | . | . | . | . | . | . | . | . |
| 21-225_170D6_HC | . | . | . | . | . | . | . | . | . |
| 21-225_171H12_HC | . | . | . | . | . | . | . | . | . |
| 21-225_177A5_HC | . | . | . | . | . | . | . | . | . |
| 21-225_181G2_HC | . | . | . | . | . | . | . | . | . |
| 21-225_184E7_HC | . | . | . | . | . | . | . | . | . |
| 21-225_185E10_HC | . | . | . | . | . | . | . | . | . |
| 21-225_188B9_HC | . | . | . | . | . | . | . | . | . |
| 21-225_188H5_HC | . | . | . | . | . | . | . | . | . |
| 21-225_18D4_HC | . | . | . | . | . | . | . | . | . |
| 21-225_213H7_HC | . | . | . | . | . | . | . | . | . |
| 21-225_221A6_HC | . | . | . | . | . | . | . | . | . |
| 21-225_221F12_HC | . | . | . | . | . | . | . | . | . |
| 21-225_222C12_HC | . | . | . | . | . | . | . | . | . |
| 21-225_222E1_HC | . | . | . | . | . | . | . | . | . |
| 21-225_222F7_HC | . | . | . | . | . | . | . | . | . |
| 21-225_23B11_HC | . | . | . | . | . | . | . | . | . |
| 21-225_30A11_HC | . | . | . | . | . | . | . | . | . |
| 21-225_31C4_HC | . | . | . | . | . | . | . | . | . |
| 21-225_33G1_HC | . | . | . | . | . | . | . | . | . |
| 21-225_33H7_HC | . | . | . | . | . | . | . | . | . |
| 21-225_34F3_HC | . | . | . | . | . | . | . | . | . |
| 21-225_34G7_HC | . | . | N | . | . | . | . | . | . |
| 21-225_35C6_HC | . | . | . | . | . | . | . | . | . |
| 21-225_35G6_HC | . | . | . | . | . | . | . | . | . |
| 21-225_35H12_HC | . | . | . | . | . | . | . | . | . |

| Reference # | 141 | 142 | 143 | 144 | 145 | 146 | 147 | 148 | 149 |
|---|---|---|---|---|---|---|---|---|---|
| | H_FR4 | | | | | | | | |
| CONSENSUS | Q | G | T | L | V | T | V | S | S |
| 21-225_36H5_HC | . | . | . | . | . | . | . | . | . |
| 21-225_49F10_HC | . | . | . | . | . | . | . | . | . |
| 21-225_4H6_HC | . | . | . | . | . | . | . | . | . |
| 21-225_4H6.004_HC | . | . | . | . | . | . | . | . | . |
| 21-225_4H6.005_HC | . | . | . | . | . | . | . | . | . |
| 21-225_4H6.006_HC | . | . | . | . | . | . | . | . | . |
| 21-225_4H6.007_HC | . | . | . | . | . | . | . | . | . |
| 21-225_4H6.008_HC | . | . | . | . | . | . | . | . | . |
| 21-225_4H6.009_HC | . | . | . | . | . | . | . | . | . |
| 21-225_4H6.010_HC | . | . | . | . | . | . | . | . | . |
| 21-225_4H6.011_HC | . | . | . | . | . | . | . | . | . |
| 21-225_4H6.014_HC | . | . | . | . | . | . | . | . | . |
| 21-225_50D4_HC | . | . | . | . | . | . | . | . | . |
| 21-225_51E9_HC | . | . | . | . | . | . | . | . | . |
| 21-225_52B1_HC | . | . | . | . | . | . | . | . | . |
| 21-225_52G11_HC | . | . | . | . | . | . | . | . | . |
| 21-225_52H10_HC | . | . | . | . | . | . | . | . | . |
| 21-225_53F2_HC | . | . | . | . | . | . | . | . | . |
| 21-225_53F7_HC | . | . | . | . | . | . | . | . | . |
| 21-225_56E5_HC | . | . | . | . | . | . | . | . | . |
| 21-225_7C3_LC1_HC | . | . | . | . | . | . | . | . | . |
| 21-225_7C3_LC2_HC | . | . | . | . | . | . | . | . | . |
| 21-225_9F12_LC1_HC | . | . | . | . | . | . | . | . | . |
| 21-225_9F12_LC2_HC | . | . | . | . | . | . | . | . | . |

**FIGURE 57**

Table 56. Consensus 22- VH3|3-33/D4|4-11|RF2/JH6 (SEQ ID NO: 50273):

QVQLVES-GGGVVQPGRSLRLSCAASG-FTFSS-----YGMHWVRQAPGKGLEWVAVIWHD---GSNKYYADSVKGRFTISRDNSKNTLYLQMNSLRAEDTAVYYCARDLSMG--------------------GMDVWGQGTTVTVSS wherein:

S at position 33 can be substituted with N

Y at position 39 can be substituted with F or H

M at position 41 can be substituted with I

V at position 57 can be substituted with I

W at position 59 can be substituted with I

H at position 60 can be substituted with Y, F or N

D at position 61 can be substituted with S or E

G at position 65 can be substituted with A

S at position 66 can be substituted with G

N at position 67 can be substituted with Y

K at position 68 can be substituted with D, E or R

Y at position 69 can be substituted with N

Y at position 70 can be substituted with N

A at position 71 can be substituted with V or G

D at position 72 can be substituted with E

S at position 73 can be substituted with A

K at position 75 can be substituted with R

**FIGURE 57**

D at position 109 can be substituted with T or R

L at position 110 can be substituted with R, Y, S, F, I or P

Sat position 111 can be substituted with R or T

M at position 112 can be substituted with V, G, P, K, N or Y

G at position 113 can be substituted with Y, null (-) or S

null (-) at position 114 can be substituted with Y, S or W

null (-) at position 115 can be substituted with G

null (-) at position 116 can be substituted with S

null (-) at position 117 can be substituted with G

null (-) at position 118 can be substituted with S

null (-) at position 119 can be substituted with P

null (-) at position 129 can be substituted with P

null (-) at position 130 can be substituted with Y

null (-) at position 131 can be substituted with Y

null (-) at position 132 can be substituted with Y

null (-) at position 133 can be substituted with S or Y

null (-) at position 134 can be substituted with D, Y or G

M at position 136 can be substituted with L or T

Xaa Xaa Gly Xaa His (SEQ ID NO: 50022)

**FIGURE 57**

Xaa Ile Xaa Xaa Xaa Xaa Xaa Xaa Xaa Xaa Xaa Xaa Xaa Xaa Val Xaa Gly (SEQ ID NO: 50023)

Xaa Xaa Xaa Xaa Xaa Xaa Xaa Xaa Xaa Xaa Xaa Xaa Xaa Xaa Xaa Xaa Xaa Gly Xaa Asp Val (SEQ ID NO: 50024)

Ser Tyr Gly Met His (SEQ ID NO: 50489)

Val Ile Trp His Asp Gly Ser Asn Lys Tyr Tyr Ala Asp Ser Val Lys Gly (SEQ ID NO: 50490)

Asp Leu Ser Met Gly Gly Met Asp Val (SEQ ID NO: 50491)

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | H_FR1 | | | | | | | | | | | | | | | | | | | | | | | | |
| CONSENSUS | Q | V | Q | L | V | E | S | - | G | G | G | V | V | Q | P | G | R | S | L | R | L | S | C | A | A |
| 21-225_13D4_HC | . | . | . | . | . | . | . | . | . | . | . | . | . | . | . | . | . | . | . | . | . | . | . | . | . |
| 21-225_15A3_HC | . | . | . | . | . | . | . | . | . | . | . | . | . | . | . | . | . | . | . | . | . | . | . | . | . |
| 21-225_16E6_HC | . | . | . | . | . | . | . | . | . | . | . | . | . | . | . | . | . | . | . | . | . | . | . | . | . |
| 21-225_171E12_HC | . | . | . | . | . | . | . | . | . | . | . | . | . | . | . | . | . | . | . | . | . | . | . | . | . |
| 21-225_179H5_HC | . | . | . | . | . | . | . | . | . | . | . | . | . | . | . | . | . | . | . | . | . | . | . | . | . |
| 21-225_18G4_HC | . | . | . | . | . | . | . | . | . | . | . | . | . | . | . | . | . | . | . | . | . | . | . | . | . |
| 21-225_190A5_HC | . | . | . | . | . | . | . | . | . | . | . | . | G | . | . | . | . | . | . | . | . | . | . | . | . |
| 21-225_190D10_HC | . | . | . | . | . | . | . | . | . | . | . | . | . | . | . | . | . | . | . | . | . | . | . | . | . |
| 21-225_190F12_HC | . | . | . | . | . | . | . | . | . | . | . | . | . | . | . | . | . | . | . | . | . | . | . | . | . |
| 21-225_190H7_HC | . | . | . | . | . | . | . | . | . | . | . | . | . | . | . | . | . | . | . | . | . | . | . | . | . |
| 21-225_190H9_HC | . | . | . | . | . | . | . | . | . | . | . | . | . | . | . | . | . | . | . | . | . | . | . | . | . |
| 21-225_192C10_HC | . | . | . | . | . | . | . | . | . | . | . | . | . | . | . | . | . | . | . | . | . | . | . | . | S |
| 21-225_192E4_HC | . | . | . | . | . | . | . | . | . | . | . | . | . | . | . | . | . | . | . | . | . | . | . | . | . |
| 21-225_192H6_HC | . | . | . | . | . | . | . | . | . | . | . | . | . | . | . | . | . | . | . | . | . | . | . | V | . |
| 21-225_194B7_HC | . | . | . | . | . | . | . | . | . | . | . | . | . | . | . | . | . | . | . | . | . | . | . | . | . |
| 21-225_194H12_HC | . | . | . | . | . | . | . | . | . | . | . | . | . | . | . | . | . | . | . | . | . | . | . | . | . |
| 21-225_196D4_HC | . | . | . | V | . | . | . | . | . | . | . | . | . | . | . | . | . | . | . | . | . | . | . | . | . |
| 21-225_197E11_HC | . | . | . | . | . | . | . | . | . | . | . | . | . | . | . | . | . | . | . | . | . | . | . | . | . |
| 21-225_198D2_HC | . | . | . | . | . | . | . | . | . | . | . | . | . | . | . | . | . | . | . | . | . | . | . | . | . |
| 21-225_205H3_HC | . | . | . | . | . | . | . | . | . | . | . | . | . | . | . | . | . | . | . | . | . | . | . | . | . |
| 21-225_20F6_HC | . | . | . | . | . | . | . | . | . | . | . | . | . | . | . | . | . | . | . | . | . | . | . | V | . |
| 21-225_22C7_HC | . | . | . | . | . | . | . | . | . | . | . | . | . | . | . | . | . | . | . | . | . | . | . | . | . |
| 21-225_25B3_HC | . | . | . | . | . | . | . | . | . | . | . | . | . | . | . | . | . | . | . | . | . | . | . | . | . |
| 21-225_26D12_HC | . | . | . | . | . | . | . | . | . | . | . | . | . | . | . | . | . | . | . | . | . | . | . | . | . |
| 21-225_26G11_HC | . | . | . | . | . | . | . | . | . | . | . | . | . | . | . | . | . | . | . | . | . | . | . | . | . |
| 21-225_27A11_HC | . | . | . | . | . | . | . | . | . | . | . | . | . | . | . | . | . | . | . | . | . | . | . | . | . |
| 21-225_3F8_HC | . | . | . | . | . | . | . | . | . | . | . | . | . | . | . | . | . | . | . | . | . | . | . | . | . |
| 21-225_6G1_HC | . | . | . | . | . | . | . | . | . | . | . | . | . | . | . | . | . | . | . | . | . | . | . | . | . |
| 21-225_7E11_HC | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | H_FR1 | | | | | | | | | | | | | | | | | | | | | | | | |
| CONSENSUS | Q | V | Q | L | V | E | S | - | G | G | G | V | V | Q | P | G | R | S | L | R | L | S | C | A | A |
| 21-225_7E11.001_HC | . | . | . | . | . | . | . | . | . | . | . | . | . | . | . | . | . | . | . | . | . | . | . | . | . |
| 21-225_7E11.001.001_HC | . | . | . | . | . | . | . | . | . | . | . | . | . | . | . | . | . | . | . | . | . | . | . | . | . |
| 21-225_7E11.001.002_HC | . | . | . | . | . | . | . | . | . | . | . | . | . | . | . | . | . | . | . | . | . | . | . | . | . |
| 21-225_7E11.001.003_HC | . | . | . | . | . | . | . | . | . | . | . | . | . | . | . | . | . | . | . | . | . | . | . | . | . |
| 21-225_7E11.001.004_HC | . | . | . | . | . | . | . | . | . | . | . | . | . | . | . | . | . | . | . | . | . | . | . | . | . |
| 21-225_7E11.001.005_HC | . | . | . | . | . | . | . | . | . | . | . | . | . | . | . | . | . | . | . | . | . | . | . | . | . |
| 21-225_7E11.001.006_HC | . | . | . | . | . | . | . | . | . | . | . | . | . | . | . | . | . | . | . | . | . | . | . | . | . |
| 21-225_7E11.001.007_HC | . | . | . | . | . | . | . | . | . | . | . | . | . | . | . | . | . | . | . | . | . | . | . | . | . |
| 21-225_7E11.001.008_HC | . | . | . | . | . | . | . | . | . | . | . | . | . | . | . | . | . | . | . | . | . | . | . | . | . |
| 21-225_7E11.001.009_HC | . | . | . | . | . | . | . | . | . | . | . | . | . | . | . | . | . | . | . | . | . | . | . | . | . |
| 21-225_7E11.001.010_HC | . | . | . | . | . | . | . | . | . | . | . | . | . | . | . | . | . | . | . | . | . | . | . | . | . |
| 21-225_7E11.001.011_HC | . | . | . | . | . | . | . | . | . | . | . | . | . | . | . | . | . | . | . | . | . | . | . | . | . |
| 21-225_7E11.001.012_HC | . | . | . | . | . | . | . | . | . | . | . | . | . | . | . | . | . | . | . | . | . | . | . | . | . |
| 21-225_7E11.001.013_HC | . | . | . | . | . | . | . | . | . | . | . | . | . | . | . | . | . | . | . | . | . | . | . | . | . |
| 21-225_7E11.001.014_HC | . | . | . | . | . | . | . | . | . | . | . | . | . | . | . | . | . | . | . | . | . | . | . | . | . |
| 21-225_7E11.001.015_HC | . | . | . | . | . | . | . | . | . | . | . | . | . | . | . | . | . | . | . | . | . | . | . | . | . |
| 21-225_7E11.001.016_HC | . | . | . | . | . | . | . | . | . | . | . | . | . | . | . | . | . | . | . | . | . | . | . | . | . |
| 21-225_7E11.001.017_HC | . | . | . | . | . | . | . | . | . | . | . | . | . | . | . | . | . | . | . | . | . | . | . | . | . |
| 21-225_7E11.001.018_HC | . | . | . | . | . | . | . | . | . | . | . | . | . | . | . | . | . | . | . | . | . | . | . | . | . |
| 21-225_7E11.001.023_HC | . | . | . | . | . | . | . | . | . | . | . | . | . | . | . | . | . | . | . | . | . | . | . | . | . |

# FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | | | H_CDR1 | | | | | | | | | | | | H_FR2 | |
| CONSENSUS | S | G | - | F | T | F | S | S | - | - | - | - | - | Y | G | M | H | W | V | R | Q | A | P | G | K |
| 21-225_13D4_HC | . | . | . | . | . | . | . | . | . | . | . | . | . | . | . | . | . | . | . | . | . | . | . | . | . |
| 21-225_15A3_HC | . | . | . | . | . | . | . | . | . | . | . | . | . | . | . | I | . | . | . | . | . | . | . | . | . |
| 21-225_16E6_HC | . | . | . | . | . | . | . | . | . | . | . | . | . | . | . | . | . | . | . | . | . | . | . | . | . |
| 21-225_171E12_HC | . | . | . | . | . | . | . | . | . | . | . | . | . | . | . | . | . | . | . | . | . | . | . | . | . |
| 21-225_179H5_HC | . | . | . | . | . | . | . | . | . | . | . | . | . | . | . | . | . | . | . | . | . | . | . | . | . |
| 21-225_18G4_HC | . | . | . | . | . | . | . | . | . | . | . | . | . | . | . | . | . | . | . | . | . | . | . | . | . |
| 21-225_190A5_HC | . | . | . | . | . | . | . | . | . | . | . | . | . | . | . | . | . | . | . | . | . | . | . | . | . |
| 21-225_190D10_HC | . | . | . | . | . | . | R | . | . | . | . | . | . | . | . | . | . | . | . | . | . | . | . | . | . |
| 21-225_190F12_HC | . | . | . | . | . | . | R | N | . | . | . | . | . | . | . | . | . | . | . | . | . | . | . | . | . |
| 21-225_190H7_HC | . | . | . | . | N | . | R | . | . | . | . | . | . | . | . | . | . | . | . | . | . | . | . | . | . |
| 21-225_190H9_HC | . | . | . | . | . | . | R | N | . | . | . | . | . | . | . | . | . | . | . | . | . | . | . | . | . |
| 21-225_192C10_HC | . | . | . | . | . | . | R | N | . | . | . | . | . | . | . | . | . | . | . | . | . | . | . | . | . |
| 21-225_192E4_HC | . | . | . | . | . | . | . | . | . | . | . | . | . | . | . | . | . | . | . | . | . | . | . | . | . |
| 21-225_192H6_HC | . | . | . | . | . | . | . | . | . | . | . | . | . | H | . | . | . | . | . | . | . | . | . | . | . |
| 21-225_194B7_HC | . | . | . | . | . | . | . | . | . | . | . | . | . | . | . | . | . | . | . | . | . | . | . | . | . |
| 21-225_194H12_HC | . | . | . | . | . | . | R | N | . | . | . | . | . | . | . | . | . | . | . | . | . | . | . | . | . |
| 21-225_196D4_HC | . | . | . | . | . | . | . | . | . | . | . | . | . | . | . | . | . | . | . | . | . | . | . | . | . |
| 21-225_197E11_HC | . | . | . | . | . | . | R | N | . | . | . | . | . | . | . | . | . | . | . | . | . | . | . | . | . |
| 21-225_198D2_HC | . | . | . | . | . | . | R | . | . | . | . | . | . | . | . | . | . | . | . | . | . | . | . | . | . |
| 21-225_205H3_HC | . | . | . | . | . | . | . | . | . | . | . | . | . | . | . | . | . | . | . | . | . | . | . | . | . |
| 21-225_20F6_HC | . | . | . | . | . | . | . | . | . | . | . | . | . | . | . | . | . | . | . | . | . | . | . | . | . |
| 21-225_22C7_HC | . | . | . | . | . | . | . | . | . | . | . | . | . | . | . | . | . | . | . | . | . | . | . | . | . |
| 21-225_25B3_HC | . | . | . | . | . | . | . | . | . | . | . | . | . | . | . | . | . | . | . | . | . | . | . | . | . |
| 21-225_26D12_HC | . | . | . | . | . | . | . | . | . | . | . | . | . | . | . | . | . | . | . | . | . | . | . | . | . |
| 21-225_26G11_HC | . | . | . | . | . | . | . | . | . | . | . | . | . | . | . | . | . | . | . | . | . | . | . | . | . |
| 21-225_27A11_HC | . | . | . | . | . | . | . | . | . | . | . | . | . | . | . | . | . | . | . | . | . | . | . | . | . |
| 21-225_3F8_HC | . | . | . | . | . | . | . | . | . | . | . | . | . | . | . | . | . | . | . | . | . | . | . | . | . |
| 21-225_6G1_HC | . | . | . | . | I | . | . | . | . | . | . | . | . | . | . | . | . | . | . | . | . | . | . | . | . |
| 21-225_7E11_HC | . | . | . | . | . | . | . | . | . | . | . | . | . | F | . | . | . | . | . | . | . | . | . | . | . |

FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | H_CDR1 | | | | | | | | | | H_FR2 | | | | | | | |
| CONSENSUS | S | G | - | F | T | F | S | S | - | - | - | - | - | Y | G | M | H | W | V | R | Q | A | P | G | K |
| 21-225_7E11.001_HC | . | . | . | . | . | . | . | . | . | . | . | . | . | F | . | . | . | . | . | . | . | . | . | . | . |
| 21-225_7E11.001.001_HC | . | . | . | . | . | . | . | . | . | . | . | . | . | F | . | . | . | . | . | . | . | . | . | . | . |
| 21-225_7E11.001.002_HC | . | . | . | . | . | . | . | . | . | . | . | . | . | F | . | . | . | . | . | . | . | . | . | . | . |
| 21-225_7E11.001.003_HC | . | . | . | . | . | . | . | . | . | . | . | . | . | F | . | . | . | . | . | . | . | . | . | . | . |
| 21-225_7E11.001.004_HC | . | . | . | . | . | . | . | . | . | . | . | . | . | F | . | . | . | . | . | . | . | . | . | . | . |
| 21-225_7E11.001.005_HC | . | . | . | . | . | . | . | . | . | . | . | . | . | F | . | . | . | . | . | . | . | . | . | . | . |
| 21-225_7E11.001.006_HC | . | . | . | . | . | . | . | . | . | . | . | . | . | F | . | . | . | . | . | . | . | . | . | . | . |
| 21-225_7E11.001.007_HC | . | . | . | . | . | . | . | . | . | . | . | . | . | F | . | . | . | . | . | . | . | . | . | . | . |
| 21-225_7E11.001.008_HC | . | . | . | . | . | . | . | . | . | . | . | . | . | F | . | . | . | . | . | . | . | . | . | . | . |
| 21-225_7E11.001.009_HC | . | . | . | . | . | . | . | . | . | . | . | . | . | F | . | . | . | . | . | . | . | . | . | . | . |
| 21-225_7E11.001.010_HC | . | . | . | . | . | . | . | . | . | . | . | . | . | F | . | . | . | . | . | . | . | . | . | . | . |
| 21-225_7E11.001.011_HC | . | . | . | . | . | . | . | . | . | . | . | . | . | F | . | . | . | . | . | . | . | . | . | . | . |
| 21-225_7E11.001.012_HC | . | . | . | . | . | . | . | . | . | . | . | . | . | F | . | . | . | . | . | . | . | . | . | . | . |
| 21-225_7E11.001.013_HC | . | . | . | . | . | . | . | . | . | . | . | . | . | F | . | . | . | . | . | . | . | . | . | . | . |
| 21-225_7E11.001.014_HC | . | . | . | . | . | . | . | . | . | . | . | . | . | F | . | . | . | . | . | . | . | . | . | . | . |
| 21-225_7E11.001.015_HC | . | . | . | . | . | . | . | . | . | . | . | . | . | F | . | . | . | . | . | . | . | . | . | . | . |
| 21-225_7E11.001.016_HC | . | . | . | . | . | . | . | . | . | . | . | . | . | F | . | . | . | . | . | . | . | . | . | . | . |
| 21-225_7E11.001.017_HC | . | . | . | . | . | . | . | . | . | . | . | . | . | F | . | . | . | . | . | . | . | . | . | . | . |
| 21-225_7E11.001.018_HC | . | . | . | . | . | . | . | . | . | . | . | . | . | F | . | . | . | . | . | . | . | . | . | . | . |
| 21-225_7E11.001.023_HC | . | . | . | . | . | . | . | . | . | . | . | . | . | F | . | . | . | . | . | . | . | . | . | . | . |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | H_CDR2 | | | | | | | | | | | | | | | | | | |
| CONSENSUS | G | L | E | W | V | A | V | I | W | H | D | - | - | - | G | S | N | K | Y | Y | A | D | S | V | K |
| 21-225_13D4_HC | . | . | . | . | . | . | . | . | . | . | . | . | . | . | . | . | . | . | . | . | . | . | . | . | . |
| 21-225_15A3_HC | . | . | . | . | . | . | . | . | . | . | . | . | . | . | . | . | . | . | . | . | . | . | . | . | . |
| 21-225_16E6_HC | . | . | . | . | . | . | . | . | . | . | . | . | . | . | . | . | . | . | . | . | V | . | . | . | . |
| 21-225_171E12_HC | . | . | . | . | . | . | . | . | . | N | . | . | . | . | . | G | . | . | . | N | G | . | . | . | . |
| 21-225_179H5_HC | . | . | . | . | . | . | . | . | . | Y | . | . | . | . | . | . | . | E | . | . | . | . | . | . | . |
| 21-225_18G4_HC | . | . | . | . | . | . | I | . | . | Y | . | . | . | . | . | . | . | . | . | . | . | . | . | . | . |
| 21-225_190A5_HC | . | . | . | . | . | . | . | . | . | Y | . | . | . | . | . | . | . | . | N | . | . | . | . | . | . |
| 21-225_190D10_HC | . | . | . | . | . | . | . | . | I | Y | . | . | . | . | . | . | Y | . | . | . | . | . | . | . | . |
| 21-225_190F12_HC | . | . | . | . | . | . | . | . | . | F | . | . | . | . | . | . | . | D | . | . | . | . | . | . | . |
| 21-225_190H7_HC | . | . | . | . | . | . | . | . | I | Y | . | . | . | . | . | . | Y | . | . | . | . | . | . | . | . |
| 21-225_190H9_HC | . | . | . | . | . | . | . | . | . | F | . | . | . | . | . | . | . | D | . | . | . | . | . | . | . |
| 21-225_192C10_HC | . | . | K | . | . | . | . | . | . | F | . | . | . | . | . | . | . | D | . | . | . | . | . | . | . |
| 21-225_192E4_HC | . | . | . | . | . | . | . | . | . | Y | . | . | . | . | . | . | . | . | N | . | V | . | . | . | R |
| 21-225_192H6_HC | . | . | . | . | . | . | . | . | . | Y | . | . | . | . | . | . | . | . | N | . | . | . | . | . | . |
| 21-225_194B7_HC | . | . | . | . | . | . | . | . | . | Y | . | . | . | . | . | . | . | . | N | . | . | . | . | . | . |
| 21-225_194H12_HC | . | . | . | . | . | . | . | . | . | F | . | . | . | . | . | . | . | D | . | . | . | . | . | . | . |
| 21-225_196D4_HC | . | . | . | . | . | . | . | . | . | Y | . | . | . | . | . | . | . | R | N | . | . | . | . | . | . |
| 21-225_197E11_HC | . | . | . | . | . | . | . | . | . | F | . | . | . | . | . | . | . | D | . | . | . | . | . | . | . |
| 21-225_198D2_HC | . | . | . | . | . | . | . | . | I | Y | . | . | . | . | . | . | Y | . | . | . | . | . | . | . | . |
| 21-225_205H3_HC | . | . | . | . | . | . | . | . | . | Y | . | . | . | . | . | . | . | . | . | . | . | . | . | . | . |
| 21-225_20F6_HC | . | . | . | . | G | . | . | . | . | . | . | . | . | . | . | . | . | . | . | . | . | . | . | . | . |
| 21-225_22C7_HC | . | . | . | . | G | . | . | . | . | . | . | . | . | . | . | . | . | . | . | . | . | . | . | . | . |
| 21-225_25B3_HC | . | . | . | . | . | . | . | . | . | Y | . | . | . | . | . | . | . | . | . | . | . | . | . | . | . |
| 21-225_26D12_HC | . | . | . | . | . | . | . | . | . | Y | . | . | . | . | . | . | . | E | . | . | . | . | . | . | . |
| 21-225_26G11_HC | . | . | . | . | . | . | . | . | . | Y | . | . | . | . | . | . | . | . | . | . | . | . | . | . | . |
| 21-225_27A11_HC | . | . | . | . | . | . | . | . | . | Y | . | . | . | . | . | . | . | . | . | . | . | . | . | . | . |
| 21-225_3F8_HC | . | . | . | . | G | . | . | . | . | . | . | . | . | . | . | . | . | . | . | . | . | . | . | . | . |
| 21-225_6G1_HC | . | . | . | . | . | . | I | . | . | Y | . | . | . | . | . | . | . | . | . | . | . | . | . | . | . |
| 21-225_7E11_HC | . | . | . | . | . | . | I | . | . | . | . | . | . | . | . | . | . | . | . | . | . | . | . | . | . |

# FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | H_CDR2 | | | | | | | | | | | | | | | | | | |
| CONSENSUS | G | L | E | W | V | A | V | I | W | H | D | - | - | - | G | S | N | K | Y | Y | A | D | S | V | K |
| 21-225_7E11.001_HC | . | . | . | . | . | . | I | . | . | . | . | . | . | . | . | . | . | . | . | . | . | . | . | . | . |
| 21-225_7E11.001.001_HC | . | . | . | . | . | . | I | . | . | . | . | . | . | . | . | . | . | . | . | . | . | . | . | . | . |
| 21-225_7E11.001.002_HC | . | . | . | . | . | . | I | . | . | . | . | . | . | . | . | . | . | . | . | . | . | . | . | . | . |
| 21-225_7E11.001.003_HC | . | . | . | . | . | . | I | . | . | . | S | . | . | . | . | . | . | . | . | . | . | . | . | . | . |
| 21-225_7E11.001.004_HC | . | . | . | . | . | . | I | . | . | . | E | . | . | . | . | . | . | . | . | . | . | . | . | . | . |
| 21-225_7E11.001.005_HC | . | . | . | . | . | . | I | . | . | . | . | . | . | . | A | . | . | . | . | . | . | . | . | . | . |
| 21-225_7E11.001.006_HC | . | . | . | . | . | . | I | . | . | . | . | . | . | . | . | . | . | . | . | . | . | E | . | . | . |
| 21-225_7E11.001.007_HC | . | . | . | . | . | . | I | . | . | . | . | . | . | . | . | . | . | . | . | . | . | . | A | . | . |
| 21-225_7E11.001.008_HC | . | . | . | . | . | . | I | . | . | . | E | . | . | . | . | . | . | . | . | . | . | . | A | . | . |
| 21-225_7E11.001.009_HC | . | . | . | . | . | . | I | . | . | . | E | . | . | . | . | . | . | . | . | . | . | E | . | . | . |
| 21-225_7E11.001.010_HC | . | . | . | . | . | . | I | . | . | . | S | . | . | . | . | . | . | . | . | . | . | E | . | . | . |
| 21-225_7E11.001.011_HC | . | . | . | . | . | . | I | . | . | . | S | . | . | . | . | . | . | . | . | . | . | . | A | . | . |
| 21-225_7E11.001.012_HC | . | . | . | . | . | . | I | . | . | . | S | . | . | . | . | . | . | . | . | . | . | . | . | . | . |
| 21-225_7E11.001.013_HC | . | . | . | . | . | . | I | . | . | . | . | . | . | . | . | . | . | . | . | . | . | E | . | . | . |
| 21-225_7E11.001.014_HC | . | . | . | . | . | . | I | . | . | . | S | . | . | . | . | . | . | . | . | . | . | E | . | . | . |
| 21-225_7E11.001.015_HC | . | . | . | . | . | . | I | . | . | . | S | . | . | . | . | . | . | . | . | . | . | . | . | . | . |
| 21-225_7E11.001.016_HC | . | . | . | . | . | . | I | . | . | . | S | . | . | . | . | . | . | . | . | . | . | E | . | . | . |
| 21-225_7E11.001.017_HC | . | . | . | . | . | . | I | . | . | . | E | . | . | . | . | . | . | . | . | . | . | . | A | . | . |
| 21-225_7E11.001.018_HC | . | . | . | . | . | . | I | . | . | . | . | . | . | . | A | . | . | . | . | . | . | E | . | . | . |
| 21-225_7E11.001.023_HC | . | . | . | . | . | . | I | . | . | . | . | . | . | . | A | . | . | . | . | . | . | . | A | . | . |

FIGURE 57

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | H_FR3 | | | | | | | | | | | | | | | | | | | | | | | |
| CONSENSUS | G | R | F | T | I | S | R | D | N | S | K | N | T | L | Y | L | Q | M | N | S | L | R | A | E | D |
| 21-225_13D4_HC | . | . | . | . | . | . | . | . | . | . | . | . | . | . | . | . | . | . | . | . | . | . | . | . | . |
| 21-225_15A3_HC | . | . | . | . | . | . | . | . | . | . | . | . | . | . | . | . | . | . | . | . | . | . | . | . | . |
| 21-225_16E6_HC | . | . | . | . | . | . | . | . | . | . | . | . | . | . | N | . | . | . | . | . | . | . | . | . | . |
| 21-225_171E12_HC | . | . | . | . | . | . | . | . | . | . | . | . | . | . | . | . | . | . | . | . | . | . | . | . | . |
| 21-225_179H5_HC | . | . | . | . | . | . | . | . | . | . | . | . | . | . | . | . | . | . | . | . | . | . | . | . | . |
| 21-225_18G4_HC | . | . | . | . | . | . | . | . | . | . | . | . | . | . | . | . | . | . | . | . | . | . | . | . | . |
| 21-225_190A5_HC | . | . | . | . | . | . | . | . | . | . | . | . | . | . | . | . | . | . | . | . | . | . | . | . | . |
| 21-225_190D10_HC | . | . | . | . | . | . | . | . | . | . | . | . | . | . | . | . | . | . | . | . | . | . | . | . | . |
| 21-225_190F12_HC | . | . | . | . | . | . | . | . | . | . | . | . | . | . | S | . | . | . | . | . | . | . | . | . | . |
| 21-225_190H7_HC | . | . | . | . | . | . | . | . | . | . | . | . | . | . | . | . | . | . | . | . | . | . | . | . | . |
| 21-225_190H9_HC | . | . | . | . | . | . | . | . | . | . | . | . | . | . | S | . | . | . | . | . | . | . | . | . | . |
| 21-225_192C10_HC | . | . | . | . | . | . | . | . | . | . | . | . | . | . | S | . | . | . | . | . | . | . | . | . | . |
| 21-225_192E4_HC | . | . | . | . | . | . | . | . | . | . | . | . | . | . | . | . | . | . | . | . | . | . | . | . | . |
| 21-225_192H6_HC | . | . | . | . | . | . | . | . | . | . | . | . | . | . | . | . | . | . | . | . | . | . | . | . | . |
| 21-225_194B7_HC | . | . | . | . | . | . | . | . | . | . | . | . | . | . | . | . | . | . | . | . | . | . | . | . | . |
| 21-225_194H12_HC | . | . | . | . | . | . | . | . | . | . | . | . | . | . | S | . | . | . | . | . | . | . | . | . | . |
| 21-225_196D4_HC | . | . | . | . | . | . | . | . | . | . | . | . | . | . | . | . | . | . | . | . | . | . | V | . | . |
| 21-225_197E11_HC | . | . | . | . | . | . | . | . | . | . | . | . | . | . | S | . | . | . | . | . | . | . | . | . | . |
| 21-225_198D2_HC | . | . | . | . | . | . | . | . | . | . | . | . | . | . | . | . | . | . | . | . | . | . | . | . | . |
| 21-225_205H3_HC | . | . | . | . | . | . | . | . | . | . | . | . | . | . | . | . | . | . | . | . | . | . | . | . | . |
| 21-225_20F6_HC | . | . | . | . | . | . | . | . | . | A | . | . | . | . | . | . | . | . | . | . | . | . | . | . | . |
| 21-225_22C7_HC | . | . | . | . | . | . | . | . | . | A | . | . | . | . | . | . | . | . | . | . | . | . | . | . | . |
| 21-225_25B3_HC | . | . | . | . | . | . | . | . | . | . | . | . | . | . | . | . | . | . | . | . | . | . | . | . | . |
| 21-225_26D12_HC | . | . | . | . | . | . | . | . | . | . | . | . | . | . | . | . | . | . | . | . | . | . | . | A | . |
| 21-225_26G11_HC | . | . | . | . | . | . | . | . | . | . | . | . | . | . | . | . | . | . | . | . | . | . | . | . | . |
| 21-225_27A11_HC | . | . | . | . | . | . | . | . | . | . | . | . | . | . | . | . | . | . | . | . | . | . | . | . | . |
| 21-225_3F8_HC | . | . | . | . | . | . | . | . | . | A | . | . | . | . | . | . | . | . | . | . | . | . | . | . | . |
| 21-225_6G1_HC | . | . | . | . | . | . | . | . | . | . | . | . | . | . | . | . | . | . | . | . | . | . | . | . | . |
| 21-225_7E11_HC | . | . | . | . | . | . | . | . | . | . | N | . | . | . | . | . | . | . | S | . | . | . | . | . | . |

**FIGURE 57**

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | H_FR3 | | | | | | | | | | | | | | | | | | | | | | | |
| CONSENSUS | G | R | F | T | I | S | R | D | N | S | K | N | T | L | Y | L | Q | M | N | S | L | R | A | E | D |
| 21-225_7E11.001_HC | . | . | . | . | . | . | . | . | . | . | . | . | . | . | . | . | . | . | S | . | . | . | . | . | . |
| 21-225_7E11.001.001_HC | . | . | . | . | . | . | . | . | . | . | . | . | . | . | . | . | . | . | S | . | . | . | . | . | . |
| 21-225_7E11.001.002_HC | . | . | . | . | . | . | . | . | . | . | . | . | . | . | . | . | . | . | S | . | . | . | . | . | . |
| 21-225_7E11.001.003_HC | . | . | . | . | . | . | . | . | . | . | . | . | . | . | . | . | . | . | S | . | . | . | . | . | . |
| 21-225_7E11.001.004_HC | . | . | . | . | . | . | . | . | . | . | . | . | . | . | . | . | . | . | S | . | . | . | . | . | . |
| 21-225_7E11.001.005_HC | . | . | . | . | . | . | . | . | . | . | . | . | . | . | . | . | . | . | S | . | . | . | . | . | . |
| 21-225_7E11.001.006_HC | . | . | . | . | . | . | . | . | . | . | . | . | . | . | . | . | . | . | S | . | . | . | . | . | . |
| 21-225_7E11.001.007_HC | . | . | . | . | . | . | . | . | . | . | . | . | . | . | . | . | . | . | S | . | . | . | . | . | . |
| 21-225_7E11.001.008_HC | . | . | . | . | . | . | . | . | . | . | . | . | . | . | . | . | . | . | S | . | . | . | . | . | . |
| 21-225_7E11.001.009_HC | . | . | . | . | . | . | . | . | . | . | . | . | . | . | . | . | . | . | S | . | . | . | . | . | . |
| 21-225_7E11.001.010_HC | . | . | . | . | . | . | . | . | . | . | . | . | . | . | . | . | . | . | S | . | . | . | . | . | . |
| 21-225_7E11.001.011_HC | . | . | . | . | . | . | . | . | . | . | . | . | . | . | . | . | . | . | S | . | . | . | . | . | . |
| 21-225_7E11.001.012_HC | . | . | . | . | . | . | . | . | . | . | . | . | . | . | . | . | . | . | S | . | . | . | . | . | . |
| 21-225_7E11.001.013_HC | . | . | . | . | . | . | . | . | . | . | . | . | . | . | . | . | . | . | S | . | . | . | . | . | . |
| 21-225_7E11.001.014_HC | . | . | . | . | . | . | . | . | . | . | . | . | . | . | . | . | . | . | S | . | . | . | . | . | . |
| 21-225_7E11.001.015_HC | . | . | . | . | . | . | . | . | . | . | . | . | . | . | . | . | . | . | S | . | . | . | . | . | . |
| 21-225_7E11.001.016_HC | . | . | . | . | . | . | . | . | . | . | . | . | . | . | . | . | . | . | S | . | . | . | . | . | . |
| 21-225_7E11.001.017_HC | . | . | . | . | . | . | . | . | . | . | . | . | . | . | . | . | . | . | S | . | . | . | . | . | . |
| 21-225_7E11.001.018_HC | . | . | . | . | . | . | . | . | . | . | . | . | . | . | . | . | . | . | S | . | . | . | . | . | . |
| 21-225_7E11.001.023_HC | . | . | . | . | . | . | . | . | . | . | . | . | . | . | . | . | . | . | S | . | . | . | . | . | . |

**FIGURE 57**

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | R | D | L | S | M | G | - | - | - | - | - | - | - | - | - | - | - | - |
| 21-225_13D4_HC | . | . | . | . | . | . | . | . | . | . | . | . | . | . | . | . | . | . | . | . | . | . | . | . | . |
| 21-225_15A3_HC | . | . | . | . | . | . | . | . | . | . | . | . | . | . | . | . | . | . | . | . | . | . | . | . | . |
| 21-225_16E6_HC | . | . | . | . | . | . | . | . | . | . | . | . | . | . | . | . | . | . | . | . | . | . | . | . | . |
| 21-225_171E12_HC | . | . | . | . | . | . | . | . | . | R | T | Y | Y | S | G | S | G | S | P | . | . | . | . | . | . |
| 21-225_179H5_HC | . | . | . | . | . | . | . | . | . | I | R | N | Y | Y | . | . | . | . | . | . | . | . | . | . | . |
| 21-225_18G4_HC | . | . | . | . | . | . | . | . | . | . | . | . | . | . | . | . | . | . | . | . | . | . | . | . | . |
| 21-225_190A5_HC | . | . | . | . | . | . | . | . | . | P | . | V | . | Y | . | . | . | . | . | . | . | . | . | . | . |
| 21-225_190D10_HC | . | . | . | . | . | . | . | . | T | Y | . | G | - | . | . | . | . | . | . | . | . | . | . | . | . |
| 21-225_190F12_HC | . | . | . | . | . | . | . | . | . | R | . | V | . | Y | . | . | . | . | . | . | . | . | . | . | . |
| 21-225_190H7_HC | . | . | . | . | . | . | . | . | T | Y | . | G | - | . | . | . | . | . | . | . | . | . | . | . | . |
| 21-225_190H9_HC | . | . | . | . | . | . | . | . | . | R | . | V | . | Y | . | . | . | . | . | . | . | . | . | . | . |
| 21-225_192C10_HC | . | . | . | . | . | . | . | . | . | R | . | V | . | Y | . | . | . | . | . | . | . | . | . | . | . |
| 21-225_192E4_HC | . | . | . | . | . | . | . | . | . | R | . | V | . | Y | . | . | . | . | . | . | . | . | . | . | . |
| 21-225_192H6_HC | . | . | . | . | . | . | . | . | . | P | . | V | . | Y | . | . | . | . | . | . | . | . | . | . | . |
| 21-225_194B7_HC | . | . | . | . | . | . | . | . | . | R | . | K | . | Y | . | . | . | . | . | . | . | . | . | . | . |
| 21-225_194H12_HC | . | . | . | . | . | . | . | . | . | R | . | V | . | Y | . | . | . | . | . | . | . | . | . | . | . |
| 21-225_196D4_HC | . | . | . | . | . | . | . | . | . | R | . | K | . | Y | . | . | . | . | . | . | . | . | . | . | . |
| 21-225_197E11_HC | . | . | . | . | . | . | . | . | . | R | . | V | . | Y | . | . | . | . | . | . | . | . | . | . | . |
| 21-225_198D2_HC | . | . | . | . | . | . | . | . | T | Y | . | G | - | . | . | . | . | . | . | . | . | . | . | . | . |
| 21-225_205H3_HC | . | . | . | . | . | . | . | . | . | R | . | V | . | Y | . | . | . | . | . | . | . | . | . | . | . |
| 21-225_20F6_HC | . | . | . | . | . | . | . | . | . | . | . | . | . | . | . | . | . | . | . | . | . | . | . | . | . |
| 21-225_22C7_HC | . | . | . | . | . | . | . | . | . | . | . | . | . | . | . | . | . | . | . | . | . | . | . | . | . |
| 21-225_25B3_HC | . | . | . | . | . | . | . | . | . | S | . | P | Y | . | . | . | . | . | . | . | . | . | . | . | . |
| 21-225_26D12_HC | . | . | . | . | . | . | . | . | . | S | . | P | Y | . | . | . | . | . | . | . | . | . | . | . | . |
| 21-225_26G11_HC | . | . | . | . | . | . | . | . | R | Y | . | N | S | W | . | . | . | . | . | . | . | . | . | . | . |
| 21-225_27A11_HC | . | . | . | . | H | . | . | . | . | S | . | P | Y | . | . | . | . | . | . | . | . | . | . | . | . |
| 21-225_3F8_HC | . | . | . | . | . | . | . | . | . | . | . | . | . | . | . | . | . | . | . | . | . | . | . | . | . |
| 21-225_6G1_HC | . | . | M | . | . | . | . | . | . | . | . | . | . | . | . | . | . | . | . | . | . | . | . | . | . |
| 21-225_7E11_HC | . | . | . | . | . | . | . | . | . | . | . | . | . | . | . | . | . | . | . | . | . | . | . | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | R | D | L | S | M | G | - | - | - | - | - | - | - | - | - | - | - | - |
| 21-225_7E11.001_HC | . | . | . | . | . | . | . | . | . | . | . | . | . | . | . | . | . | . | . | . | . | . | . | . | . |
| 21-225_7E11.001.001_HC | . | . | . | . | . | . | . | . | . | . | . | . | . | . | . | . | . | . | . | . | . | . | . | . | . |
| 21-225_7E11.001.002_HC | . | . | . | . | . | . | . | . | . | . | . | . | . | . | . | . | . | . | . | . | . | . | . | . | . |
| 21-225_7E11.001.003_HC | . | . | . | . | . | . | . | . | . | . | . | . | . | . | . | . | . | . | . | . | . | . | . | . | . |
| 21-225_7E11.001.004_HC | . | . | . | . | . | . | . | . | . | . | . | . | . | . | . | . | . | . | . | . | . | . | . | . | . |
| 21-225_7E11.001.005_HC | . | . | . | . | . | . | . | . | . | . | . | . | . | . | . | . | . | . | . | . | . | . | . | . | . |
| 21-225_7E11.001.006_HC | . | . | . | . | . | . | . | . | . | . | . | . | . | . | . | . | . | . | . | . | . | . | . | . | . |
| 21-225_7E11.001.007_HC | . | . | . | . | . | . | . | . | . | . | . | . | . | . | . | . | . | . | . | . | . | . | . | . | . |
| 21-225_7E11.001.008_HC | . | . | . | . | . | . | . | . | . | . | . | . | . | . | . | . | . | . | . | . | . | . | . | . | . |
| 21-225_7E11.001.009_HC | . | . | . | . | . | . | . | . | . | . | . | . | . | . | . | . | . | . | . | . | . | . | . | . | . |
| 21-225_7E11.001.010_HC | . | . | . | . | . | . | . | . | . | . | . | . | . | . | . | . | . | . | . | . | . | . | . | . | . |
| 21-225_7E11.001.011_HC | . | . | . | . | . | . | . | . | . | . | . | . | . | . | . | . | . | . | . | . | . | . | . | . | . |
| 21-225_7E11.001.012_HC | . | . | . | . | . | . | . | . | . | . | . | . | . | . | . | . | . | . | . | . | . | . | . | . | . |
| 21-225_7E11.001.013_HC | . | . | . | . | . | . | . | . | . | . | . | . | . | . | . | . | . | . | . | . | . | . | . | . | . |
| 21-225_7E11.001.014_HC | . | . | . | . | . | . | . | . | . | . | . | . | . | . | . | . | . | . | . | . | . | . | . | . | . |
| 21-225_7E11.001.015_HC | . | . | . | . | . | . | . | . | . | . | . | . | . | . | . | . | . | . | . | . | . | . | . | . | . |
| 21-225_7E11.001.016_HC | . | . | . | . | . | . | . | . | . | . | . | . | . | . | . | . | . | . | . | . | . | . | . | . | . |
| 21-225_7E11.001.017_HC | . | . | . | . | . | . | . | . | . | . | . | . | . | . | . | . | . | . | . | . | . | . | . | . | . |
| 21-225_7E11.001.018_HC | . | . | . | . | . | . | . | . | . | . | . | . | . | . | . | . | . | . | . | . | . | . | . | . | . |
| 21-225_7E11.001.023_HC | . | . | . | . | . | . | . | . | . | . | . | . | . | . | . | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | | | | | | H_FR4 | | | | | |
| CONSENSUS | - | - | - | - | - | - | - | - | - | G | M | D | V | W | G | Q | G | T | T | V | T | V | S | S |
| 21-225_13D4_HC | . | . | . | . | . | . | . | . | . | . | . | . | . | . | . | . | . | . | . | . | . | . | . | . |
| 21-225_15A3_HC | . | . | . | . | . | . | . | . | . | . | . | . | . | . | . | . | . | . | . | . | . | . | . | . |
| 21-225_16E6_HC | . | . | . | . | . | . | . | . | . | . | . | . | . | . | . | . | . | . | . | . | . | . | . | . |
| 21-225_171E12_HC | . | . | . | P | Y | Y | Y | Y | Y | . | . | . | . | . | . | . | . | . | . | . | . | . | . | . |
| 21-225_179H5_HC | . | . | . | . | . | . | . | . | Y | . | L | . | . | . | . | . | . | . | . | . | . | . | . | . |
| 21-225_18G4_HC | . | . | . | . | . | . | . | . | . | . | . | . | . | . | . | . | . | . | . | . | . | . | . | . |
| 21-225_190A5_HC | . | . | . | . | . | . | . | . | D | . | . | . | . | . | . | . | . | . | . | . | . | . | . | . |
| 21-225_190D10_HC | . | . | . | . | . | . | . | . | . | . | . | . | . | . | . | . | . | . | . | . | . | . | . | . |
| 21-225_190F12_HC | . | . | . | . | . | . | . | . | D | . | L | . | . | . | . | . | . | . | . | . | . | . | . | . |
| 21-225_190H7_HC | . | . | . | . | . | . | . | . | . | . | . | . | . | . | . | . | . | . | . | . | . | . | . | . |
| 21-225_190H9_HC | . | . | . | . | . | . | . | . | D | . | L | . | . | . | . | . | . | . | . | . | . | . | . | . |
| 21-225_192C10_HC | . | . | . | . | . | . | . | . | D | . | L | . | . | . | . | . | . | . | . | . | . | . | . | . |
| 21-225_192E4_HC | . | . | . | . | . | . | . | . | D | . | . | . | . | . | . | . | . | . | . | . | . | . | . | . |
| 21-225_192H6_HC | . | . | . | . | . | . | . | . | D | . | . | . | . | . | . | . | . | . | . | . | . | . | . | . |
| 21-225_194B7_HC | . | . | . | . | . | . | . | . | D | . | . | . | . | . | . | . | . | . | . | . | . | . | . | . |
| 21-225_194H12_HC | . | . | . | . | . | . | . | . | D | . | L | . | . | . | . | . | . | . | . | . | . | . | . | . |
| 21-225_196D4_HC | . | . | . | . | . | . | . | . | D | . | . | . | . | . | . | . | . | . | . | . | . | . | . | . |
| 21-225_197E11_HC | . | . | . | . | . | . | . | . | D | . | L | . | . | . | . | . | . | . | . | . | . | . | . | . |
| 21-225_198D2_HC | . | . | . | . | . | . | . | . | . | . | . | . | . | . | . | . | . | . | . | . | . | . | . | . |
| 21-225_205H3_HC | . | . | . | . | . | . | . | . | D | . | T | . | . | . | . | . | . | . | . | . | . | . | . | . |
| 21-225_20F6_HC | . | . | . | . | . | . | . | . | . | . | . | . | . | . | . | . | . | . | . | . | . | . | . | . |
| 21-225_22C7_HC | . | . | . | . | . | . | . | . | . | . | . | . | . | . | . | . | . | . | . | . | . | . | . | . |
| 21-225_25B3_HC | . | . | . | . | . | . | . | . | . | . | . | . | . | . | . | . | . | . | . | . | . | . | . | . |
| 21-225_26D12_HC | . | . | . | . | . | . | . | . | . | . | . | . | . | . | . | . | . | . | . | . | . | . | . | . |
| 21-225_26G11_HC | . | . | . | . | . | . | . | S | G | . | . | . | . | . | . | . | . | . | . | . | . | . | . | . |
| 21-225_27A11_HC | . | . | . | . | . | . | . | . | . | . | . | . | . | . | . | . | . | . | . | . | . | . | . | . |
| 21-225_3F8_HC | . | . | . | . | . | . | . | . | . | . | . | . | . | . | . | . | . | . | . | . | . | . | . | . |
| 21-225_6G1_HC | . | . | . | . | . | . | . | . | . | . | . | . | . | . | . | . | . | . | . | . | . | . | . | . |
| 21-225_7E11_HC | . | . | . | . | . | . | . | . | . | . | . | . | . | . | . | . | . | . | . | . | . | . | . | . |

**FIGURE 57**

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | H_FR4 | | | | | | | | | | |
| CONSENSUS | - | - | - | - | - | - | - | - | - | G | M | D | V | W | G | Q | G | T | T | V | T | V | S | S |
| 21-225_7E11.001_HC | . | . | . | . | . | . | . | . | . | . | . | . | . | . | . | . | . | . | . | . | . | . | . | . |
| 21-225_7E11.001.001_HC | . | . | . | . | . | . | . | . | . | . | . | . | . | . | . | . | . | . | . | . | . | . | . | . |
| 21-225_7E11.001.002_HC | . | . | . | . | . | . | . | . | . | . | . | . | . | . | . | . | . | . | . | . | . | . | . | . |
| 21-225_7E11.001.003_HC | . | . | . | . | . | . | . | . | . | . | . | . | . | . | . | . | . | . | . | . | . | . | . | . |
| 21-225_7E11.001.004_HC | . | . | . | . | . | . | . | . | . | . | . | . | . | . | . | . | . | . | . | . | . | . | . | . |
| 21-225_7E11.001.005_HC | . | . | . | . | . | . | . | . | . | . | . | . | . | . | . | . | . | . | . | . | . | . | . | . |
| 21-225_7E11.001.006_HC | . | . | . | . | . | . | . | . | . | . | . | . | . | . | . | . | . | . | . | . | . | . | . | . |
| 21-225_7E11.001.007_HC | . | . | . | . | . | . | . | . | . | . | . | . | . | . | . | . | . | . | . | . | . | . | . | . |
| 21-225_7E11.001.008_HC | . | . | . | . | . | . | . | . | . | . | . | . | . | . | . | . | . | . | . | . | . | . | . | . |
| 21-225_7E11.001.009_HC | . | . | . | . | . | . | . | . | . | . | . | . | . | . | . | . | . | . | . | . | . | . | . | . |
| 21-225_7E11.001.010_HC | . | . | . | . | . | . | . | . | . | . | . | . | . | . | . | . | . | . | . | . | . | . | . | . |
| 21-225_7E11.001.011_HC | . | . | . | . | . | . | . | . | . | . | . | . | . | . | . | . | . | . | . | . | . | . | . | . |
| 21-225_7E11.001.012_HC | . | . | . | . | . | . | . | . | . | . | . | . | . | . | . | . | . | . | . | . | . | . | . | . |
| 21-225_7E11.001.013_HC | . | . | . | . | . | . | . | . | . | . | . | . | . | . | . | . | . | . | . | . | . | . | . | . |
| 21-225_7E11.001.014_HC | . | . | . | . | . | . | . | . | . | . | . | . | . | . | . | . | . | . | . | . | . | . | . | . |
| 21-225_7E11.001.015_HC | . | . | . | . | . | . | . | . | . | . | . | . | . | . | . | . | . | . | . | . | . | . | . | . |
| 21-225_7E11.001.016_HC | . | . | . | . | . | . | . | . | . | . | . | . | . | . | . | . | . | . | . | . | . | . | . | . |
| 21-225_7E11.001.017_HC | . | . | . | . | . | . | . | . | . | . | . | . | . | . | . | . | . | . | . | . | . | . | . | . |
| 21-225_7E11.001.018_HC | . | . | . | . | . | . | . | . | . | . | . | . | . | . | . | . | . | . | . | . | . | . | . | . |
| 21-225_7E11.001.023_HC | . | . | . | . | . | . | . | . | . | . | . | . | . | . | . | . | . | . | . | . | . | . | . | . |

Table 57. Consensus 23- VH3|3-33/D7|7-27|RF1/JH4 (SEQ ID NO: 50274):

QVQLVES-GGGVVQPGRSLRLSCAASG-FTFSD-----YGMHWVRQAPGKGLEWVAVIWYD---ESNKYYADSVKGRFTISRDNSKNTLYLQMNSLRAEDTAVYYCAREVGW---------------------LDDYWGQGTLVTVSS wherein:

**FIGURE 57**

D at position 33 can be substituted with N or S

Y at position 39 can be substituted with F

M at position 41 can be substituted with I or L

Y at position 60 can be substituted with F

D at position 61 can be substituted with E, I or V

E at position 65 can be substituted with G, V, A or R

S at position 66 can be substituted with N, T or G

K at position 68 can be substituted with Q, T or N

Y at position 69 can be substituted with H or K

A at position 71 can be substituted with T, G, E or V

D at position 72 can be substituted with G

V at position 74 can be substituted with A

V at position 110 can be substituted with I, M, K or T

W at position 112 can be substituted with F, G or M

L at position 135 can be substituted with T, S, Y, H or R

D at position 136 can be substituted with E, S, F or N

Y at position 138 can be substituted with C

Xaa Xaa Gly Xaa His (SEQ ID NO: 50025)

Val Ile Trp Xaa Xaa Xaa Xaa Asn Xaa Xaa Tyr Xaa Xaa Ser Xaa Lys Gly (SEQ ID NO: 50026)

**FIGURE 57**

Glu Xaa Gly Xaa Xaa Xaa Asp Xaa (SEQ ID NO: 50027)

Asp Tyr Gly Met His (SEQ ID NO: 50492)

Val Ile Trp Tyr Asp Glu Ser Asn Lys Tyr Tyr Ala Asp Ser Val Lys Gly (SEQ ID NO: 50493)

Glu Val Gly Trp Leu Asp Asp Tyr (SEQ ID NO: 50494)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | E | S | - | G | G | G | V | V | Q | P | G | R | S | L | R | L | S | C | A | A | S |
| 21-225_149F12_HC | . | . | . | . | . | . | . | . | . | . | . | . | . | . | . | . | . | . | . | . | . | . | . | . | . | . |
| 21-225_149G1_HC | . | . | . | . | . | . | . | . | . | . | . | . | . | . | . | . | . | . | . | . | . | . | . | . | . | . |
| 21-225_14C2_HC | . | . | . | . | . | . | . | . | . | . | . | . | . | . | . | . | . | . | . | . | . | . | . | . | . | . |
| 21-225_150E7_HC | . | . | . | . | . | . | . | . | . | . | . | . | . | . | . | . | . | . | . | . | . | . | . | . | . | . |
| 21-225_15B11_HC | . | . | . | . | . | . | . | . | . | . | . | . | . | . | . | . | . | . | . | . | . | . | . | . | . | . |
| 21-225_15H10_HC | . | . | . | . | . | . | . | . | . | . | . | . | . | . | . | . | . | . | . | . | . | . | . | . | . | . |
| 21-225_161D11_HC | . | . | . | . | . | . | . | . | . | . | . | . | . | . | . | . | . | . | . | . | . | . | . | . | . | . |
| 21-225_161F7_HC | . | . | . | . | . | . | . | . | . | . | . | . | . | . | . | . | . | . | . | . | . | . | . | . | . | . |
| 21-225_16H4_HC | . | . | . | . | . | . | . | . | . | . | . | . | . | . | . | . | . | . | . | . | . | . | . | . | . | . |
| 21-225_180H7_HC | . | . | . | . | . | . | . | . | . | . | . | . | . | . | . | . | . | . | . | . | . | . | . | . | . | . |
| 21-225_181A8_HC | . | . | . | . | . | . | . | . | . | . | . | . | . | . | . | . | . | . | . | . | . | . | . | . | . | . |
| 21-225_184D11_HC | . | . | . | . | . | . | . | . | . | . | . | . | . | . | . | . | . | . | . | . | . | . | . | . | . | . |
| 21-225_184H10_HC | . | . | . | . | . | . | . | . | . | . | . | . | . | . | . | . | . | . | . | . | . | . | . | V | . | . |
| 21-225_18H12_HC | . | . | . | . | . | . | . | . | . | . | . | . | . | . | . | . | . | . | . | . | . | . | . | . | . | . |
| 21-225_203C10_HC | . | . | . | . | . | . | . | . | . | . | . | . | . | . | . | . | . | . | . | . | . | . | . | . | . | . |
| 21-225_210E4_HC | . | . | . | . | . | . | . | . | . | . | . | . | . | . | . | . | . | . | . | . | . | . | . | . | . | . |
| 21-225_225B6_HC | . | . | R | . | . | . | . | . | . | . | . | . | . | . | . | . | . | . | . | . | . | . | . | . | . | . |
| 21-225_225D6_HC | . | . | . | . | . | . | . | . | . | . | . | . | . | . | . | . | . | . | . | . | . | . | . | . | . | . |
| 21-225_225F8_HC | . | . | . | . | . | . | . | . | . | . | . | . | . | . | . | . | . | . | . | . | . | . | . | . | . | . |
| 21-225_226A9_HC | . | . | . | . | . | . | . | . | . | . | . | . | . | . | . | . | . | . | . | . | . | . | . | . | . | . |
| 21-225_227C12_HC | . | . | . | . | . | . | . | . | . | . | . | . | . | . | . | . | K | . | . | . | . | . | . | . | . | . |
| 21-225_227G3_HC | . | . | . | . | . | . | . | . | . | . | . | . | . | . | . | . | . | . | . | . | . | . | . | . | . | . |
| 21-225_227H5_HC | . | . | . | . | . | . | . | . | . | . | . | . | . | . | . | . | . | . | . | . | . | . | . | . | . | . |
| 21-225_22B7_HC | . | . | . | . | . | . | . | . | . | . | . | . | . | . | . | . | . | . | . | . | . | . | . | . | . | . |
| 21-225_25A9_HC | . | . | . | . | . | . | . | . | . | . | . | . | . | . | . | . | . | . | . | . | . | . | . | . | . | . |
| 21-225_25G5_HC | . | . | . | . | . | . | . | . | . | . | . | . | . | . | . | . | . | . | . | . | . | . | . | . | . | . |
| 21-225_28A9_HC | . | . | . | . | . | . | . | . | . | . | . | . | . | . | . | . | . | . | . | . | . | . | . | . | . | . |
| 21-225_28B8_HC | . | . | . | . | . | . | . | . | . | . | . | . | . | . | . | . | . | . | . | . | . | . | . | . | . | . |
| 21-225_29D8_HC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | E | S | - | G | G | G | V | V | Q | P | G | R | S | L | R | L | S | C | A | A | S |
| 21-225_29D9_HC | . | . | . | . | . | . | . | . | . | . | . | . | . | . | . | . | . | . | . | K | . | . | . | S | . | . |
| 21-225_30F3_HC | . | . | . | . | . | . | . | . | . | . | . | . | . | . | . | . | . | . | . | . | . | . | . | . | . | . |
| 21-225_33B1_HC | . | . | . | . | . | . | . | . | . | . | . | . | . | . | . | . | . | . | . | . | . | . | . | . | . | . |
| 21-225_4C8_HC | . | . | . | . | . | . | . | . | . | . | . | . | . | . | . | . | . | . | . | . | . | . | . | . | . | . |
| 21-225_4F12_HC | . | . | . | . | . | . | . | . | . | . | . | . | . | . | . | . | . | . | . | . | . | . | . | . | . | . |
| 21-225_50C4_HC | . | . | . | . | . | . | . | . | . | . | . | . | . | . | . | . | . | . | . | . | . | . | . | . | . | . |
| 21-225_73G6_HC | . | . | . | . | . | . | . | . | . | . | . | . | . | . | . | . | . | . | . | . | . | . | . | . | . | . |
| 21-225_90H5_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | D | - | - | - | - | - | Y | G | M | H | W | V | R | Q | A | P | G | K | G | L |
| 21-225_149F12_HC | . | . | . | . | . | . | . | . | . | . | . | . | . | . | . | . | . | . | . | . | . | . | . | . | . | . |
| 21-225_149G1_HC | . | . | . | . | . | . | . | . | . | . | . | . | . | . | . | . | . | . | . | . | . | . | . | . | . | . |
| 21-225_14C2_HC | . | . | . | . | . | . | . | . | . | . | . | . | . | . | . | . | . | . | . | . | . | . | . | . | . | . |
| 21-225_150E7_HC | . | . | . | . | . | . | . | . | . | . | . | . | . | . | . | . | . | . | . | . | . | . | . | . | . | . |
| 21-225_15B11_HC | . | . | . | . | . | . | N | . | . | . | . | . | . | . | . | . | . | . | . | . | . | . | . | . | . | . |
| 21-225_15H10_HC | . | . | . | . | . | . | N | . | . | . | . | . | . | . | . | . | . | . | . | . | . | . | . | . | . | . |
| 21-225_161D11_HC | . | . | . | . | . | . | . | . | . | . | . | . | F | . | L | . | . | . | . | . | . | . | . | Q | . | . |
| 21-225_161F7_HC | . | . | . | . | . | . | . | . | . | . | . | . | F | . | L | . | . | . | . | . | . | . | . | Q | . | . |
| 21-225_16H4_HC | . | . | . | . | . | . | N | . | . | . | . | . | . | . | . | . | . | . | . | . | . | . | . | . | . | . |
| 21-225_180H7_HC | . | . | . | . | . | . | . | . | . | . | . | . | . | . | . | . | . | . | . | . | . | . | . | . | . | . |
| 21-225_181A8_HC | . | . | . | . | . | . | . | . | . | . | . | . | . | . | . | . | . | . | . | . | . | . | . | . | . | . |
| 21-225_184D11_HC | . | . | . | . | . | . | . | . | . | . | . | . | . | . | . | . | . | . | . | . | . | . | . | . | . | . |
| 21-225_184H10_HC | . | . | . | . | . | . | . | . | . | . | . | . | . | . | . | . | . | . | . | . | . | . | . | . | . | . |
| 21-225_18H12_HC | . | . | . | . | . | . | . | . | . | . | . | . | . | . | . | . | . | . | . | . | . | . | . | . | . | . |
| 21-225_203C10_HC | . | . | . | . | . | . | . | . | . | . | . | . | . | . | . | . | . | . | . | . | . | . | . | . | . | . |
| 21-225_210E4_HC | . | . | . | . | L | I | N | . | . | . | . | . | . | . | . | . | . | . | . | . | . | . | . | . | . | . |
| 21-225_225B6_HC | . | . | . | . | . | . | . | . | . | . | . | . | . | . | . | . | . | . | . | . | . | . | . | . | . | . |
| 21-225_225D6_HC | . | . | . | . | . | . | N | . | . | . | . | . | . | . | . | . | . | . | . | . | . | . | . | . | . | . |
| 21-225_225F8_HC | . | . | . | . | . | . | S | . | . | . | . | . | . | . | . | . | . | . | . | . | . | . | . | . | . | . |
| 21-225_226A9_HC | . | . | . | . | . | . | N | . | . | . | . | . | . | . | . | . | . | . | . | . | . | . | . | . | . | . |
| 21-225_227C12_HC | . | . | . | . | . | . | N | . | . | . | . | . | . | . | . | . | . | . | . | . | . | . | . | . | . | . |
| 21-225_227G3_HC | . | . | . | . | . | . | S | . | . | . | . | . | . | . | . | . | . | . | . | . | . | . | . | . | . | . |
| 21-225_227H5_HC | . | . | . | . | . | . | N | . | . | . | . | . | . | . | . | . | . | . | . | . | . | . | . | . | . | . |
| 21-225_22B7_HC | . | . | . | . | . | . | S | . | . | . | . | . | . | . | . | . | . | . | . | . | . | . | . | . | . | . |
| 21-225_25A9_HC | . | . | . | . | . | . | S | . | . | . | . | . | . | . | . | . | . | . | . | . | . | . | . | . | . | . |
| 21-225_25G5_HC | . | . | . | . | . | . | . | . | . | . | . | . | . | . | I | . | . | . | . | . | . | . | . | . | . | . |
| 21-225_28A9_HC | . | . | . | . | . | . | S | . | . | . | . | . | . | . | I | . | . | . | . | . | . | . | . | . | . | . |
| 21-225_28B8_HC | . | . | . | . | . | . | . | . | . | . | . | . | . | . | . | . | . | . | . | . | . | . | . | . | . | . |
| 21-225_29D8_HC | . | . | . | . | . | R | . | . | . | . | . | . | . | . | I | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | D | - | - | - | - | - | Y | G | M | H | W | V | R | Q | A | P | G | K | G | L |
| 21-225_29D9_HC | . | . | . | . | . | . | . | . | . | . | . | . | . | . | I | . | . | . | . | . | . | . | . | . | . | . |
| 21-225_30F3_HC | . | . | . | . | . | . | S | . | . | . | . | . | . | . | . | . | . | . | . | . | . | . | . | . | . | . |
| 21-225_33B1_HC | . | . | . | . | . | . | . | . | . | . | . | . | . | . | . | . | . | . | . | . | . | . | A | . | . | . |
| 21-225_4C8_HC | . | . | . | . | . | . | N | . | . | . | . | . | . | . | . | . | . | . | . | . | . | . | . | . | . | . |
| 21-225_4F12_HC | . | . | . | . | . | . | S | . | . | . | . | . | . | . | . | . | . | . | . | . | . | . | . | . | . | . |
| 21-225_50C4_HC | . | . | . | . | . | . | . | . | . | . | . | . | . | . | . | . | . | . | . | . | . | . | . | . | . | . |
| 21-225_73G6_HC | . | . | . | . | . | . | S | . | . | . | . | . | . | . | . | . | . | . | . | . | . | . | . | . | . | . |
| 21-225_90H5_HC | . | . | . | . | . | . | . | . | . | . | . | . | . | . | . | . | . | . | . | . | S | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | A | V | I | W | Y | D | - | - | - | E | S | N | K | Y | Y | A | D | S | V | K | G | R | F |
| 21-225_149F12_HC | . | . | . | . | . | . | . | . | E | . | . | . | . | N | . | . | . | . | . | . | . | . | . | . | . | . |
| 21-225_149G1_HC | . | . | . | . | . | . | . | . | E | . | . | . | G | . | . | . | . | . | . | . | . | . | . | . | . | . |
| 21-225_14C2_HC | . | . | . | . | . | . | . | . | . | . | . | . | . | . | . | . | . | . | E | . | . | . | . | . | . | . |
| 21-225_150E7_HC | . | . | . | . | . | . | . | . | E | . | . | . | . | N | . | . | . | . | . | . | . | . | . | . | . | . |
| 21-225_15B11_HC | . | . | . | . | . | . | . | . | E | . | . | . | . | . | . | . | . | . | T | . | . | . | . | . | . | . |
| 21-225_15H10_HC | . | . | . | . | . | . | . | . | . | . | . | . | . | . | . | . | . | . | E | . | . | . | . | . | . | . |
| 21-225_161D11_HC | . | . | . | . | . | . | . | F | . | . | . | . | G | . | . | . | . | . | . | . | . | . | . | . | . | . |
| 21-225_161F7_HC | . | . | . | . | . | . | . | F | . | . | . | . | G | . | . | . | . | . | . | . | . | . | . | . | . | . |
| 21-225_16H4_HC | . | . | . | . | . | . | . | . | . | . | . | . | V | . | . | . | . | . | . | . | . | . | . | . | . | . |
| 21-225_180H7_HC | . | . | . | . | . | . | . | . | . | . | . | . | . | N | . | . | H | . | . | . | . | A | . | . | . | . |
| 21-225_181A8_HC | . | . | . | . | . | . | . | . | . | . | . | . | . | N | . | . | H | . | . | . | . | A | . | . | . | . |
| 21-225_184D11_HC | . | . | . | . | . | . | . | . | . | . | . | . | . | T | . | . | H | . | G | . | . | . | . | . | . | . |
| 21-225_184H10_HC | . | . | . | . | . | . | . | . | . | . | . | . | . | T | . | . | H | . | G | . | . | . | . | . | . | . |
| 21-225_18H12_HC | . | . | M | . | . | . | . | . | . | . | . | . | V | T | . | . | . | . | . | . | . | . | . | . | . | . |
| 21-225_203C10_HC | . | . | . | . | . | . | . | . | . | . | . | . | G | G | . | T | H | . | T | . | . | . | . | . | . | . |
| 21-225_210E4_HC | . | . | . | . | . | . | . | . | . | . | . | . | . | N | . | . | . | . | V | . | . | . | . | . | . | . |
| 21-225_225B6_HC | . | . | . | . | . | . | . | . | . | . | . | . | . | N | . | . | . | . | . | . | . | . | . | . | . | . |
| 21-225_225D6_HC | . | . | . | . | . | . | . | . | . | . | . | . | . | N | . | . | . | . | . | . | . | . | . | . | . | . |
| 21-225_225F8_HC | . | . | . | . | . | . | . | . | . | . | . | . | . | N | . | . | . | . | V | . | . | . | . | . | . | . |
| 21-225_226A9_HC | . | . | . | . | . | . | . | . | E | . | . | . | . | . | . | . | . | . | T | . | . | . | . | . | . | . |
| 21-225_227C12_HC | . | . | . | . | . | . | . | . | I | . | . | . | G | . | . | Q | . | . | . | . | . | . | . | . | . | . |
| 21-225_227G3_HC | . | . | . | . | . | . | . | . | V | . | . | . | G | . | . | Q | . | . | . | . | . | . | . | . | . | . |
| 21-225_227H5_HC | D | . | . | . | . | . | . | . | E | . | . | . | . | . | . | . | . | . | . | . | . | . | . | . | . | . |
| 21-225_22B7_HC | . | . | . | . | . | . | . | . | E | . | . | . | . | N | . | Q | . | . | . | . | . | . | . | . | . | . |
| 21-225_25A9_HC | . | . | . | . | . | . | . | . | . | . | . | . | V | T | . | . | . | . | T | G | . | . | . | . | . | . |
| 21-225_25G5_HC | . | . | . | . | . | . | . | . | . | . | . | . | G | N | . | . | . | . | . | . | . | . | . | . | . | . |
| 21-225_28A9_HC | . | . | . | . | . | . | . | F | . | . | . | . | G | . | . | . | . | . | . | . | . | . | . | . | . | . |
| 21-225_28B8_HC | . | . | . | T | . | . | . | . | . | . | . | . | A | N | . | . | . | . | . | . | . | . | . | . | . | . |
| 21-225_29D8_HC | . | . | . | . | . | . | . | F | . | . | . | . | G | T | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | A | V | I | W | Y | D | - | - | - | E | S | N | K | Y | Y | A | D | S | V | K | G | R | F |
| 21-225_29D9_HC | . | . | . | . | . | . | . | . | . | . | . | . | . | . | . | N | . | . | . | . | . | . | . | . | . | . |
| 21-225_30F3_HC | . | . | . | . | . | . | . | . | . | . | . | . | V | . | . | . | . | . | . | . | . | . | . | . | . | . |
| 21-225_33B1_HC | . | . | . | . | . | . | . | . | . | . | . | . | R | N | . | . | . | . | . | . | . | . | . | . | . | . |
| 21-225_4C8_HC | . | . | . | . | . | . | . | . | E | . | . | . | . | N | . | Q | . | . | . | . | . | . | . | . | . | . |
| 21-225_4F12_HC | . | . | . | . | . | . | . | . | . | . | . | . | V | T | . | . | K | . | . | . | . | . | . | . | . | . |
| 21-225_50C4_HC | . | . | . | . | . | . | . | . | E | . | . | . | . | T | . | . | . | . | . | . | . | . | . | . | . | . |
| 21-225_73G6_HC | . | . | . | . | . | . | . | . | . | . | . | . | V | . | . | . | . | . | G | . | . | . | . | . | . | . |
| 21-225_90H5_HC | . | . | . | . | . | . | . | . | E | . | . | . | G | . | . | T | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_149F12_HC | . | . | . | . | . | . | . | . | . | . | . | F | . | . | . | . | . | . | . | . | . | . | . | . | . | . |
| 21-225_149G1_HC | . | . | . | . | . | . | . | . | . | M | . | . | . | . | . | . | . | . | . | . | . | . | . | . | . | . |
| 21-225_14C2_HC | . | . | . | . | . | . | . | . | . | . | . | . | . | . | . | K | . | . | . | . | . | . | . | Q | . | . |
| 21-225_150E7_HC | . | . | . | . | . | . | . | . | . | . | . | F | . | . | . | . | . | . | . | . | . | . | . | . | . | . |
| 21-225_15B11_HC | . | . | . | . | . | . | . | . | . | . | . | . | . | . | . | . | . | . | . | P | . | . | . | . | . | . |
| 21-225_15H10_HC | . | . | . | . | . | . | . | . | . | . | . | . | . | . | . | . | . | . | . | . | . | . | . | . | . | . |
| 21-225_161D11_HC | . | . | F | . | . | . | . | . | . | . | . | . | . | . | . | . | . | . | . | . | . | . | . | . | . | . |
| 21-225_161F7_HC | . | . | F | . | . | . | . | . | . | . | . | . | . | . | . | . | . | . | . | . | . | . | . | . | . | . |
| 21-225_16H4_HC | . | . | . | . | . | . | . | . | . | M | . | . | . | . | . | . | . | . | . | . | . | . | . | . | . | . |
| 21-225_180H7_HC | . | . | . | . | . | . | . | . | . | . | . | . | . | . | . | . | . | . | . | V | . | . | . | . | . | . |
| 21-225_181A8_HC | . | . | . | . | . | . | . | . | . | . | . | . | . | . | . | . | . | . | . | . | . | . | . | . | . | . |
| 21-225_184D11_HC | . | . | . | . | . | . | . | . | D | . | . | . | . | . | . | . | . | . | . | . | . | . | . | . | . | . |
| 21-225_184H10_HC | . | . | . | . | . | . | . | . | D | . | . | . | . | . | . | . | . | . | . | . | . | . | . | . | . | . |
| 21-225_18H12_HC | . | . | . | . | . | . | . | . | . | . | . | . | . | . | . | . | . | . | . | . | . | . | . | . | . | . |
| 21-225_203C10_HC | . | . | . | . | . | . | . | . | . | . | . | . | . | . | . | . | . | . | . | . | . | . | . | . | . | . |
| 21-225_210E4_HC | . | . | . | . | . | D | . | . | . | . | . | . | . | . | . | . | . | . | . | . | . | . | S | . | . | . |
| 21-225_225B6_HC | . | . | . | . | . | . | . | . | . | . | . | . | . | . | . | . | . | . | . | . | . | . | . | . | . | . |
| 21-225_225D6_HC | . | . | . | . | . | . | . | Q | . | . | . | . | . | . | . | T | . | . | . | . | . | . | . | . | . | . |
| 21-225_225F8_HC | . | . | . | . | . | . | . | . | . | . | . | . | . | . | . | . | . | . | . | . | . | . | . | . | . | . |
| 21-225_226A9_HC | . | . | . | . | . | . | . | . | . | . | . | F | . | . | . | . | . | . | . | . | . | . | . | . | . | . |
| 21-225_227C12_HC | . | . | . | . | . | . | . | . | . | . | . | . | . | . | . | S | . | . | . | . | . | . | . | . | . | . |
| 21-225_227G3_HC | . | . | . | . | . | . | . | . | . | . | . | . | . | . | . | S | . | . | . | . | . | . | . | . | . | . |
| 21-225_227H5_HC | . | . | . | . | . | . | . | . | . | . | . | . | . | . | . | . | . | . | . | . | . | . | . | . | M | . |
| 21-225_22B7_HC | . | . | . | . | . | . | . | . | . | . | . | . | . | . | . | . | . | . | . | . | . | . | . | . | . | . |
| 21-225_25A9_HC | . | . | . | . | . | . | . | . | . | . | . | . | . | . | . | . | . | . | . | . | . | . | . | . | . | . |
| 21-225_25G5_HC | . | . | . | . | . | . | . | . | . | . | . | . | . | . | . | . | N | . | . | . | . | . | . | . | . | . |
| 21-225_28A9_HC | . | . | . | . | . | . | . | . | . | . | . | F | . | . | . | . | . | . | . | V | . | . | . | . | . | . |
| 21-225_28B8_HC | . | . | . | . | . | . | F | . | . | . | V | . | . | . | . | . | . | . | . | . | . | . | . | . | . | . |
| 21-225_29D8_HC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_29D9_HC | . | . | . | . | . | . | . | . | . | . | . | . | . | . | . | . | . | . | . | . | . | . | . | . | . | . |
| 21-225_30F3_HC | . | . | . | . | . | . | . | . | . | . | . | . | . | . | . | . | . | . | . | . | . | . | . | . | . | . |
| 21-225_33B1_HC | . | . | . | . | . | . | . | . | . | . | . | . | . | . | . | . | . | . | . | . | . | . | . | . | . | . |
| 21-225_4C8_HC | . | . | . | . | . | . | . | . | . | . | . | . | . | . | . | . | . | . | T | . | . | . | . | . | . | . |
| 21-225_4F12_HC | . | . | . | . | . | . | . | . | . | . | . | . | . | . | . | . | . | . | T | P | . | N | . | G | G | . |
| 21-225_50C4_HC | . | . | . | . | . | . | . | . | . | . | . | . | . | . | . | . | . | . | . | G | . | . | . | . | . | . |
| 21-225_73G6_HC | . | . | . | . | . | . | . | . | . | . | . | . | . | . | . | . | . | . | . | . | . | . | . | . | . | . |
| 21-225_90H5_HC | . | . | . | . | . | . | . | . | . | . | . | . | . | . | . | . | . | . | . | . | . | . | . | . | . | . |

# FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | E | V | G | W | - | - | - | - | - | - | - | - | - | - | - | - | - | - | - | - | - | - |
| 21-225_149F12_HC | . | . | . | . | . | I | . | F | . | . | . | . | . | . | . | . | . | . | . | . | . | . | . | . | . | . |
| 21-225_149G1_HC | . | . | . | . | . | I | . | F | . | . | . | . | . | . | . | . | . | . | . | . | . | . | . | . | . | . |
| 21-225_14C2_HC | . | . | . | . | . | . | . | F | . | . | . | . | . | . | . | . | . | . | . | . | . | . | . | . | . | . |
| 21-225_150E7_HC | . | . | . | . | . | I | . | F | . | . | . | . | . | . | . | . | . | . | . | . | . | . | . | . | . | . |
| 21-225_15B11_HC | . | . | . | . | . | . | . | F | . | . | . | . | . | . | . | . | . | . | . | . | . | . | . | . | . | . |
| 21-225_15H10_HC | . | . | V | . | . | . | . | F | . | . | . | . | . | . | . | . | . | . | . | . | . | . | . | . | . | . |
| 21-225_161D11_HC | . | . | . | . | . | I | . | . | . | . | . | . | . | . | . | . | . | . | . | . | . | . | . | . | . | . |
| 21-225_161F7_HC | . | . | . | . | . | I | . | . | . | . | . | . | . | . | . | . | . | . | . | . | . | . | . | . | . | . |
| 21-225_16H4_HC | . | . | . | . | . | M | . | . | . | . | . | . | . | . | . | . | . | . | . | . | . | . | . | . | . | . |
| 21-225_180H7_HC | . | . | . | . | . | . | . | . | . | . | . | . | . | . | . | . | . | . | . | . | . | . | . | . | . | . |
| 21-225_181A8_HC | . | . | . | . | . | . | . | . | . | . | . | . | . | . | . | . | . | . | . | . | . | . | . | . | . | . |
| 21-225_184D11_HC | . | . | . | . | . | . | . | . | . | . | . | . | . | . | . | . | . | . | . | . | . | . | . | . | . | . |
| 21-225_184H10_HC | . | . | . | . | . | . | . | . | . | . | . | . | . | . | . | . | . | . | . | . | . | . | . | . | . | . |
| 21-225_18H12_HC | . | . | . | . | . | . | . | . | . | . | . | . | . | . | . | . | . | . | . | . | . | . | . | . | . | . |
| 21-225_203C10_HC | . | . | . | . | . | . | . | . | . | . | . | . | . | . | . | . | . | . | . | . | . | . | . | . | . | . |
| 21-225_210E4_HC | . | . | . | . | . | T | . | F | . | . | . | . | . | . | . | . | . | . | . | . | . | . | . | . | . | . |
| 21-225_225B6_HC | . | . | . | . | . | . | . | F | . | . | . | . | . | . | . | . | . | . | . | . | . | . | . | . | . | . |
| 21-225_225D6_HC | . | . | . | . | . | . | . | F | . | . | . | . | . | . | . | . | . | . | . | . | . | . | . | . | . | . |
| 21-225_225F8_HC | . | . | . | . | . | . | . | F | . | . | . | . | . | . | . | . | . | . | . | . | . | . | . | . | . | . |
| 21-225_226A9_HC | . | . | . | . | . | . | . | F | . | . | . | . | . | . | . | . | . | . | . | . | . | . | . | . | . | . |
| 21-225_227C12_HC | . | . | . | . | . | . | . | F | . | . | . | . | . | . | . | . | . | . | . | . | . | . | . | . | . | . |
| 21-225_227G3_HC | . | . | . | . | . | . | . | F | . | . | . | . | . | . | . | . | . | . | . | . | . | . | . | . | . | . |
| 21-225_227H5_HC | . | . | . | . | . | . | . | F | . | . | . | . | . | . | . | . | . | . | . | . | . | . | . | . | . | . |
| 21-225_22B7_HC | . | . | . | . | . | . | . | F | . | . | . | . | . | . | . | . | . | . | . | . | . | . | . | . | . | . |
| 21-225_25A9_HC | . | . | . | . | . | I | . | . | . | . | . | . | . | . | . | . | . | . | . | . | . | . | . | . | . | . |
| 21-225_25G5_HC | . | . | . | . | . | I | . | . | . | . | . | . | . | . | . | . | . | . | . | . | . | . | . | . | . | . |
| 21-225_28A9_HC | . | . | . | . | . | I | . | . | . | . | . | . | . | . | . | . | . | . | . | . | . | . | . | . | . | . |
| 21-225_28B8_HC | . | . | . | . | . | I | . | . | . | . | . | . | . | . | . | . | . | . | . | . | . | . | . | . | . | . |
| 21-225_29D8_HC | . | . | G | . | . | I | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | E | V | G | W | - | - | - | - | - | - | - | - | - | - | - | - | - | - | - | - | - | - |
| 21-225_29D9_HC | . | . | . | . | . | I | . | . | . | . | . | . | . | . | . | . | . | . | . | . | . | . | . | . | . | . |
| 21-225_30F3_HC | . | . | . | . | . | M | . | . | . | . | . | . | . | . | . | . | . | . | . | . | . | . | . | . | . | . |
| 21-225_33B1_HC | . | . | . | . | . | M | . | . | . | . | . | . | . | . | . | . | . | . | . | . | . | . | . | . | . | . |
| 21-225_4C8_HC | . | S | P | . | . | M | . | F | . | . | . | . | . | . | . | . | . | . | . | . | . | . | . | . | . | . |
| 21-225_4F12_HC | E | N | Q | . | . | K | . | G | . | . | . | . | . | . | . | . | . | . | . | . | . | . | . | . | . | . |
| 21-225_50C4_HC | . | . | . | . | . | . | . | F | . | . | . | . | . | . | . | . | . | . | . | . | . | . | . | . | . | . |
| 21-225_73G6_HC | . | . | . | . | . | . | . | M | . | . | . | . | . | . | . | . | . | . | . | . | . | . | . | . | . | . |
| 21-225_90H5_HC | . | . | V | . | . | M | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | L | D | D | Y | W | G | Q | G | T | L | V | T | V | S | S |
| 21-225_149F12_HC | . | . | . | . | S | E | . | . | . | . | . | . | . | . | . | . | . | . | . |
| 21-225_149G1_HC | . | . | . | . | S | E | . | . | . | . | . | . | . | . | . | . | . | . | . |
| 21-225_14C2_HC | . | . | . | . | R | S | . | . | . | . | . | . | . | . | . | . | . | . | . |
| 21-225_150E7_HC | . | . | . | . | S | E | . | . | . | . | . | . | . | . | . | . | . | . | . |
| 21-225_15B11_HC | . | . | . | . | T | E | . | . | . | . | . | . | . | . | . | . | . | . | . |
| 21-225_15H10_HC | . | . | . | . | . | S | . | . | . | . | . | . | I | . | . | . | . | . | . |
| 21-225_161D11_HC | . | . | . | . | . | S | . | . | . | . | . | . | . | . | . | . | . | . | . |
| 21-225_161F7_HC | . | . | . | . | . | S | . | . | . | . | . | . | . | . | . | . | . | . | . |
| 21-225_16H4_HC | . | . | . | . | T | . | . | C | . | . | . | . | . | . | . | . | . | . | . |
| 21-225_180H7_HC | . | . | . | . | S | . | . | . | . | . | . | . | . | . | . | . | . | . | . |
| 21-225_181A8_HC | . | . | . | . | S | . | . | . | . | . | . | . | . | . | . | I | . | . | . |
| 21-225_184D11_HC | . | . | . | . | H | . | . | . | . | . | . | . | . | . | . | . | . | . | . |
| 21-225_184H10_HC | . | . | . | . | N | . | . | . | . | . | . | . | . | . | . | . | . | . | . |
| 21-225_18H12_HC | . | . | . | . | Y | E | . | . | . | . | . | . | . | . | . | . | . | . | . |
| 21-225_203C10_HC | . | . | . | . | . | . | . | . | . | . | . | . | . | . | . | . | . | . | . |
| 21-225_210E4_HC | . | . | . | . | . | S | . | . | . | . | . | . | . | . | . | . | . | . | . |
| 21-225_225B6_HC | . | . | . | . | T | E | . | . | . | . | . | . | . | . | . | . | . | . | . |
| 21-225_225D6_HC | . | . | . | . | T | E | . | . | . | . | . | . | . | . | . | . | . | . | . |
| 21-225_225F8_HC | . | . | . | . | T | E | . | . | . | . | . | . | . | . | . | . | . | . | . |
| 21-225_226A9_HC | . | . | . | . | T | E | . | . | . | . | . | . | . | . | . | . | . | . | . |
| 21-225_227C12_HC | . | . | . | . | T | E | . | . | . | . | . | . | . | . | . | . | . | . | . |
| 21-225_227G3_HC | . | . | . | . | T | E | . | . | . | . | . | . | . | . | . | . | . | . | . |
| 21-225_227H5_HC | . | . | . | . | T | E | . | . | . | . | . | . | . | . | . | . | . | . | . |
| 21-225_22B7_HC | . | . | . | . | R | S | . | . | . | . | . | . | . | . | . | . | . | . | . |
| 21-225_25A9_HC | . | . | . | . | . | . | . | . | . | . | . | . | . | . | . | . | . | . | . |
| 21-225_25G5_HC | . | . | . | . | . | . | . | . | . | . | . | . | . | . | . | . | . | . | . |
| 21-225_28A9_HC | . | . | . | . | . | . | . | . | . | . | . | . | . | . | . | . | . | . | . |
| 21-225_28B8_HC | . | . | . | . | . | . | . | . | . | . | . | . | . | . | . | . | . | . | . |
| 21-225_29D8_HC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | L | D | D | Y | W | G | Q | G | T | L | V | T | V | S | S |
| 21-225_29D9_HC | . | . | . | . | . | . | . | . | . | . | . | . | . | Q | . | . | . | . | . |
| 21-225_30F3_HC | . | . | . | . | Y | . | . | . | . | . | . | . | . | . | . | . | . | . | . |
| 21-225_33B1_HC | . | . | . | . | Y | . | . | . | . | . | . | . | . | . | . | . | . | . | . |
| 21-225_4C8_HC | . | . | . | . | . | S | . | . | . | . | . | . | . | . | . | . | . | . | . |
| 21-225_4F12_HC | . | . | . | . | . | F | . | . | . | . | . | . | . | . | . | . | . | . | . |
| 21-225_50C4_HC | . | . | . | . | . | N | . | . | . | . | . | . | I | . | . | . | . | . | . |
| 21-225_73G6_HC | . | . | . | . | T | S | . | . | . | . | . | . | . | . | . | . | . | . | . |
| 21-225_90H5_HC | . | . | . | . | . | . | . | . | . | . | . | . | . | . | . | . | . | . | . |

**FIGURE 57**

Table 58. Consensus 24-VH3|3-21/D4|4-11|RF2/JH4 (SEQ ID NO: 50275)

EVQLVES-GGGLVKPGGSLRLSCAASG-FTFSS-----YSMNWVRQAPGKGLEWVSSISGS---SSYIYYADSVKGRFTISRDNAKNSLYLQMNSLRAEDTAVYYCARDRG------------------------SSWGQGTLVTVSS wherein:

S at position 33 can be substituted with T

Y at position 39 can be substituted with F

S at position 40 can be substituted with T, G or R

M at position 41 can be substituted with L

S at position 57 can be substituted with A, C or L

G at position 60 can be substituted with S

S at position 65 can be substituted with G or T

S at position 66 can be substituted with T, G or Y

Y at position 67 can be substituted with H

I at position 68 can be substituted with L or M

Y at position 69 can be substituted with S or W

A at position 71 can be substituted with G, P or V

V at position 74 can be substituted with L

G at position 76 can be substituted with A

D at position 109 can be substituted with T

G at position 111 can be substituted with null (-), S or Y

null (-) at position 112 can be substituted with G

**FIGURE 57**

null (-) at position 135 can be substituted with S

null (-) at position 136 can be substituted with F or S

S at position 137 can be substituted with D, G or H

S at position 138 can be substituted with L, Y, G, T, C, E or I

Xaa Xaa Xaa Xaa Asn (SEQ ID NO: 50028)

Xaa Ile Ser Xaa Ser Xaa Xaa Xaa Xaa Xaa Tyr Xaa Asp Ser Xaa Lys Xaa (SEQ ID NO: 50029)

Xaa Arg Xaa Xaa Xaa Xaa Xaa Xaa (SEQ ID NO: 50030)

Ser Tyr Ser Met Asn (SEQ ID NO: 50495)

Ser Ile Ser Gly Ser Ser Ser Tyr Ile Tyr Tyr Ala Asp Ser Val Lys Gly (SEQ ID NO: 50496)

Asp Arg Gly Ser Ser (SEQ ID NO: 50497)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | E | V | Q | L | V | E | S | - | G | G | G | L | V | K | P | G | G | S | L | R | L | S | C | A | A | S |
| 21-225_13D3_HC | . | . | . | . | . | . | . | . | . | . | . | . | . | . | . | . | . | . | . | . | . | . | . | . | . | . |
| 1-225_14B1_LC1_HC | . | . | . | . | . | . | . | . | . | . | . | . | . | . | . | . | . | . | . | . | . | . | . | . | . | . |
| 1-225_14B1_LC2_HC | . | . | . | . | . | . | . | . | . | . | . | . | . | . | . | . | . | . | . | . | . | . | . | . | . | . |
| 21-225_157G7_HC | . | . | . | . | . | . | . | . | . | . | . | . | . | . | . | . | . | . | . | . | F | . | . | . | . | . |
| 21-225_157H4_HC | . | . | . | . | . | . | . | . | . | . | . | . | . | . | . | . | . | . | . | . | . | . | . | . | . | . |
| 21-225_157H7_HC | . | . | . | . | . | . | . | . | . | . | . | . | . | . | . | . | . | . | . | . | . | . | . | . | . | . |
| 21-225_158F5_HC | . | . | . | . | . | . | . | . | . | . | . | . | . | . | . | . | . | . | . | . | F | . | . | . | . | . |
| 21-225_158G8_HC | . | . | . | . | . | . | . | . | . | . | . | . | . | T | . | . | . | . | . | . | . | . | . | . | . | . |
| 21-225_158H5_HC | . | . | . | . | . | . | . | . | . | . | . | . | . | . | . | . | . | . | . | . | . | . | . | . | . | . |
| 21-225_160F4_HC | . | . | . | . | . | . | . | . | . | . | . | . | . | . | . | . | . | . | . | . | F | . | . | . | . | . |
| 21-225_160H1_HC | . | . | . | . | . | . | . | . | . | . | . | . | . | . | . | . | . | . | . | . | . | . | . | . | . | . |
| 21-225_162F2_HC | . | . | . | . | . | . | . | . | . | . | . | . | . | . | . | . | . | . | . | . | F | . | . | . | . | . |
| 21-225_162H3_HC | . | . | . | . | . | . | . | . | . | . | . | . | . | . | . | . | . | . | . | . | F | . | . | . | . | . |
| 21-225_163F9_HC | . | . | . | . | . | . | . | . | . | . | . | . | . | Q | . | . | . | . | . | . | . | . | . | . | . | . |
| 21-225_171A11_HC | . | . | . | . | . | . | . | . | . | . | . | . | . | . | . | . | . | . | . | . | . | . | . | . | . | . |
| 21-225_17C10_HC | . | . | . | . | . | . | . | . | . | . | . | . | . | . | . | . | . | . | . | . | . | . | . | . | . | . |
| 21-225_17D8_HC | . | . | . | . | . | . | . | . | . | . | . | . | . | . | . | . | . | . | . | . | . | . | . | . | . | . |
| 21-225_20B5_HC | . | . | . | . | . | . | A | . | . | . | . | . | . | . | . | . | . | . | . | . | . | . | . | . | . | . |
| 21-225_20B8_HC | . | . | . | . | . | . | . | . | . | . | . | . | . | . | . | . | . | . | . | . | . | . | . | . | . | . |
| 21-225_20C8_HC | . | . | . | . | . | . | . | . | . | . | . | . | . | . | . | . | . | . | . | . | . | . | . | . | . | . |
| 21-225_20F9_HC | . | . | . | . | . | . | . | . | . | . | . | . | . | . | . | . | . | . | . | . | . | . | . | . | . | . |
| 21-225_20G12_HC | . | . | . | . | . | . | . | . | . | . | . | . | . | . | . | . | . | . | . | . | . | . | . | . | . | . |
| 21-225_20H10_HC | . | . | . | . | . | . | . | . | . | . | . | . | . | . | . | . | . | . | . | . | . | . | . | . | . | . |
| 21-225_22H3_HC | A | . | . | . | . | . | . | . | . | . | . | . | . | . | . | . | . | . | . | . | . | . | . | . | . | . |
| 21-225_23E11_HC | . | . | . | . | . | . | . | . | . | . | . | . | . | . | . | . | . | . | . | . | . | . | . | . | . | . |
| 21-225_30H11_HC | . | . | . | . | . | . | . | . | . | . | . | . | . | . | . | . | . | . | . | . | . | . | . | . | . | . |
| 21-225_31G9_HC | . | . | . | . | . | . | . | . | . | . | . | . | . | . | . | . | . | . | . | . | . | . | . | . | . | . |
| 21-225_33C2_HC | . | . | . | . | . | . | . | . | . | . | . | . | . | . | . | . | . | . | . | . | . | . | . | . | . | . |
| 21-225_43E1_HC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | E | V | Q | L | V | E | S | - | G | G | G | L | V | K | P | G | G | S | L | R | L | S | C | A | A | S |
| 21-225_53G1_HC | . | . | . | . | . | . | . | . | . | . | . | . | . | . | . | . | [illegible] | . | . | . | . | . | . | . | . | . |
| 21-225_56F1_HC | . | . | . | . | . | . | . | . | . | . | . | . | . | . | [illegible] | . | . | . | . | . | . | . | . | . | . | . |
| 21-225_56H7_HC | . | . | . | . | . | . | . | . | . | . | . | . | . | . | . | . | . | . | . | . | . | . | . | . | . | . |
| 21-225_58C5_HC | . | . | . | . | . | . | . | . | . | . | . | . | . | . | . | . | . | . | . | . | . | . | . | . | . | . |
| 21-225_65C12_HC | . | . | . | . | . | . | . | . | . | . | . | . | . | . | . | . | . | . | . | . | . | . | . | . | . | . |
| 21-225_6C12_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | S | M | N | W | V | R | Q | A | P | G | K | G | L |
| 21-225_13D3_HC | . | . | . | . | . | . | . | . | . | . | . | . | . | T | . | . | . | . | . | . | . | . | . | . | . | . |
| 1-225_14B1_LC1_HC | . | . | . | . | . | . | . | . | . | . | . | . | . | T | . | . | . | . | . | . | . | . | . | . | . | . |
| 1-225_14B1_LC2_HC | . | . | . | . | . | . | . | . | . | . | . | . | . | T | . | . | . | . | . | . | . | . | . | . | . | . |
| 21-225_157G7_HC | . | . | . | . | . | . | . | . | . | . | . | . | . | . | . | . | . | . | . | . | . | . | . | . | . | . |
| 21-225_157H4_HC | . | . | . | . | . | . | . | . | . | . | . | . | . | . | . | . | . | . | . | . | . | . | . | . | . | . |
| 21-225_157H7_HC | . | . | . | . | . | . | . | . | . | . | . | . | . | . | . | . | . | . | . | . | . | . | . | . | . | . |
| 21-225_158F5_HC | . | . | . | . | . | . | . | . | . | . | . | . | . | . | . | . | . | . | . | . | . | . | . | . | . | . |
| 21-225_158G8_HC | . | . | . | . | . | . | . | . | . | . | . | . | . | T | . | . | . | . | . | . | . | . | . | . | . | . |
| 21-225_158H5_HC | . | . | . | . | . | . | . | . | . | . | . | . | . | . | . | . | . | . | . | . | . | . | . | . | . | . |
| 21-225_160F4_HC | . | . | . | . | . | . | . | . | . | . | . | . | . | . | . | . | . | . | . | . | . | . | . | . | . | . |
| 21-225_160H1_HC | . | . | . | . | . | . | . | . | . | . | . | . | . | R | . | . | . | . | . | . | . | . | . | . | . | . |
| 21-225_162F2_HC | . | . | . | . | . | . | . | . | . | . | . | . | . | . | . | . | . | . | . | . | . | . | . | . | . | . |
| 21-225_162H3_HC | . | . | . | . | . | . | . | . | . | . | . | . | . | . | . | . | R | . | . | . | . | . | . | . | . | . |
| 21-225_163F9_HC | . | . | . | . | . | . | . | . | . | . | . | . | . | . | . | . | . | . | . | . | . | . | . | . | . | . |
| 21-225_171A11_HC | . | . | . | . | . | . | . | . | . | . | . | . | . | . | . | . | . | . | . | . | . | . | . | . | . | . |
| 21-225_17C10_HC | . | . | . | . | . | . | . | . | . | . | . | . | . | . | . | . | . | . | . | . | . | . | . | . | . | . |
| 21-225_17D8_HC | . | . | . | . | . | . | . | . | . | . | . | . | . | G | L | . | . | . | . | . | . | . | . | . | . | . |
| 21-225_20B5_HC | . | . | . | . | . | T | . | . | . | . | . | . | . | . | . | . | . | . | . | . | . | . | . | . | . | . |
| 21-225_20B8_HC | . | . | . | . | . | . | . | . | . | . | . | . | . | . | . | . | . | . | . | . | . | . | . | . | . | . |
| 21-225_20C8_HC | . | . | . | . | . | . | . | . | . | . | . | . | . | . | . | . | . | . | . | . | . | . | . | . | . | . |
| 21-225_20F9_HC | . | . | . | . | . | . | . | . | . | . | . | . | . | . | . | . | . | . | . | . | . | . | . | . | . | . |
| 21-225_20G12_HC | . | . | . | . | . | . | . | . | . | . | . | . | . | . | . | . | . | . | . | . | . | . | . | . | . | . |
| 21-225_20H10_HC | . | . | . | . | . | . | T | . | . | . | . | . | . | . | . | . | . | . | . | . | . | . | . | . | . | . |
| 21-225_22H3_HC | . | . | . | . | . | . | . | . | . | . | . | . | . | . | . | . | . | . | . | . | . | . | . | . | . | . |
| 21-225_23E11_HC | . | . | . | . | . | . | . | . | . | . | . | . | . | T | . | . | . | . | . | . | . | . | . | . | . | . |
| 21-225_30H11_HC | . | . | . | . | . | . | . | . | . | . | . | . | . | . | . | . | . | . | . | . | . | . | . | . | . | . |
| 21-225_31G9_HC | . | . | . | . | . | . | . | . | . | . | . | . | . | . | . | . | . | . | . | . | . | . | . | . | . | . |
| 21-225_33C2_HC | . | . | . | . | . | . | . | . | . | . | . | . | . | T | . | . | . | . | . | . | . | . | . | . | . | . |
| 21-225_43E1_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | S | M | N | W | V | R | Q | A | P | G | K | G | L |
| 21-225_53G1_HC | . | . | . | . | . | . | . | . | . | . | . | . | . | . | . | . | . | . | . | . | . | . | . | . | . | . |
| 21-225_56F1_HC | . | . | . | . | . | . | . | . | . | . | . | . | . | . | . | . | . | . | . | . | . | . | . | . | . | . |
| 21-225_56H7_HC | . | . | . | S | . | . | . | . | . | . | . | . | . | . | . | . | . | . | . | . | . | . | . | . | . | . |
| 21-225_58C5_HC | . | . | . | . | . | N | . | . | . | . | . | . | . | . | . | . | . | . | . | . | . | . | . | . | . | . |
| 21-225_65C12_HC | . | . | . | . | . | [illegible] | . | . | . | . | . | . | F | . | L | . | . | . | . | . | . | . | . | . | . | . |
| 21-225_6C12_HC | . | . | . | . | . | . | . | . | . | . | . | . | . | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | S | S | I | S | G | S | - | - | - | S | S | Y | I | Y | Y | A | D | S | V | K | G | R | F |
| 21-225_13D3_HC | D | . | . | . | . | . | . | . | . | . | . | . | . | . | . | . | . | . | . | . | . | . | . | . | . | . |
| 1-225_14B1_LC1_HC | . | . | . | . | . | . | . | . | . | . | . | . | . | . | . | L | . | . | P | . | . | . | . | . | . | . |
| 1-225_14B1_LC2_HC | . | . | . | . | . | . | . | . | . | . | . | . | . | . | . | L | . | . | P | . | . | . | . | . | . | . |
| 21-225_157G7_HC | . | . | . | . | . | . | . | . | . | . | . | . | . | T | . | . | . | . | . | . | . | . | . | . | . | . |
| 21-225_157H4_HC | . | . | . | . | . | . | . | S | . | . | . | . | . | T | . | . | . | . | . | . | . | . | . | . | . | . |
| 21-225_157H7_HC | . | . | . | . | C | . | . | . | . | . | . | . | . | . | . | . | . | . | . | . | . | . | . | . | . | . |
| 21-225_158F5_HC | . | . | . | . | . | . | . | . | . | . | . | . | . | T | . | . | . | . | . | . | . | . | . | . | . | . |
| 21-225_158G8_HC | . | . | . | . | L | . | . | . | . | . | . | . | . | . | . | . | . | . | . | . | . | . | . | . | . | . |
| 21-225_158H5_HC | . | . | . | . | . | . | . | . | . | . | . | . | . | T | . | . | . | . | . | . | . | . | . | . | . | . |
| 21-225_160F4_HC | . | . | . | . | . | . | . | . | . | . | . | . | . | T | . | . | . | . | . | . | . | . | . | . | . | . |
| 21-225_160H1_HC | . | . | . | . | . | . | . | S | . | . | . | . | T | G | . | M | . | . | . | . | . | . | . | . | . | . |
| 21-225_162F2_HC | . | . | . | . | . | . | . | . | . | . | . | . | . | T | . | . | . | . | . | . | . | . | . | . | . | . |
| 21-225_162H3_HC | . | . | . | . | . | . | . | . | . | . | . | . | . | T | . | . | . | . | . | . | . | . | . | . | . | . |
| 21-225_163F9_HC | . | . | . | . | . | . | . | . | . | . | . | . | . | T | . | . | . | . | . | . | . | . | . | . | . | . |
| 21-225_171A11_HC | . | . | . | . | . | . | . | . | . | . | . | . | . | . | . | . | . | . | . | . | . | . | . | . | . | . |
| 21-225_17C10_HC | . | . | . | . | . | . | . | . | . | . | . | . | . | . | . | M | . | . | . | . | . | . | . | . | . | . |
| 21-225_17D8_HC | . | . | . | . | . | . | . | . | . | . | . | . | G | . | H | . | S | . | . | . | . | . | . | . | . | . |
| 21-225_20B5_HC | . | . | . | . | . | . | . | . | . | . | . | . | . | . | . | . | . | . | . | . | . | . | . | . | . | . |
| 21-225_20B8_HC | . | . | . | . | . | . | . | . | . | . | . | . | G | . | H | . | . | . | . | . | . | . | . | . | . | . |
| 21-225_20C8_HC | . | . | . | . | . | . | . | . | . | . | . | . | . | . | . | . | . | . | . | . | . | . | . | A | . | . |
| 21-225_20F9_HC | . | . | . | . | . | . | . | S | . | . | . | . | . | . | . | . | . | . | . | . | . | . | . | . | . | . |
| 21-225_20G12_HC | . | . | . | . | . | . | . | . | . | . | . | . | . | . | . | . | . | . | . | . | . | L | . | . | . | . |
| 21-225_20H10_HC | . | . | . | . | . | . | . | S | . | . | . | . | . | . | . | . | . | . | . | . | . | . | . | . | . | . |
| 21-225_22H3_HC | . | . | . | . | . | . | . | S | . | . | . | . | . | . | . | . | . | . | . | . | . | . | . | . | . | . |
| 21-225_23E11_HC | . | . | . | . | . | . | . | . | . | . | . | . | . | . | . | . | W | . | V | . | . | . | . | . | . | . |
| 21-225_30H11_HC | . | . | . | . | . | . | . | . | . | . | . | . | . | . | . | . | . | . | . | . | . | . | . | . | . | . |
| 21-225_31G9_HC | . | . | . | . | . | . | . | S | . | . | . | . | . | . | . | . | . | . | . | . | . | . | . | . | . | . |
| 21-225_33C2_HC | Q | . | . | . | . | . | . | . | . | . | . | . | . | . | . | . | . | . | . | . | . | . | . | . | . | . |
| 21-225_43E1_HC | . | . | . | . | A | . | . | . | N | . | . | . | . | T | . | . | . | . | . | . | . | L | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | S | S | I | S | G | S | - | - | - | S | S | Y | I | Y | Y | A | D | S | V | K | G | R | F |
| 21-225_53G1_HC | . | . | . | . | . | . | . | . | . | . | . | . | . | . | . | . | . | . | . | . | . | . | . | . | . | . |
| 21-225_56F1_HC | . | . | . | . | . | . | . | S | . | . | . | . | . | T | . | . | . | . | G | . | . | . | . | . | . | . |
| 21-225_56H7_HC | . | . | . | . | . | . | . | S | . | . | . | . | . | T | . | . | . | . | G | . | . | . | . | . | . | . |
| 21-225_58C5_HC | . | . | I | . | . | . | . | S | . | . | . | . | . | Y | . | L | . | . | . | . | . | . | . | . | . | . |
| 21-225_65C12_HC | . | . | . | . | . | . | . | S | . | . | . | . | . | . | . | . | . | . | . | . | . | . | . | . | . | . |
| 21-225_6C12_HC | . | . | . | . | . | . | . | S | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | A | K | N | S | L | Y | L | Q | M | N | S | L | R | A | E | D | T | A | V | Y |
| 21-225_13D3_HC | . | . | . | . | . | . | . | . | . | . | . | . | . | . | . | . | . | . | . | . | . | . | . | . | . | . |
| 1-225_14B1_LC1_HC | . | . | . | . | . | . | . | . | . | . | . | . | . | . | . | . | . | . | . | . | . | . | . | . | . | . |
| 1-225_14B1_LC2_HC | . | . | . | . | . | . | . | . | . | . | . | . | . | . | . | . | . | . | . | . | . | . | . | . | . | . |
| 21-225_157G7_HC | . | . | . | . | . | . | . | . | . | . | . | . | . | . | . | . | . | . | . | . | . | . | . | . | . | . |
| 21-225_157H4_HC | . | . | . | . | . | . | . | . | . | . | . | . | . | . | . | . | . | . | . | . | . | . | . | . | . | . |
| 21-225_157H7_HC | . | M | . | . | . | . | . | . | . | . | . | . | . | . | . | . | . | . | . | . | . | . | . | . | . | . |
| 21-225_158F5_HC | . | . | . | . | . | . | . | . | . | . | . | . | . | . | . | . | . | . | . | . | . | . | . | . | . | . |
| 21-225_158G8_HC | . | . | . | . | . | . | . | . | . | . | . | . | . | . | . | . | . | . | . | . | . | . | . | . | . | . |
| 21-225_158H5_HC | . | . | . | . | . | . | . | . | . | . | . | H | . | . | . | . | . | . | . | . | . | . | . | . | . | . |
| 21-225_160F4_HC | . | . | . | . | . | . | . | . | . | . | . | . | . | . | . | . | . | . | . | . | . | . | . | . | . | . |
| 21-225_160H1_HC | . | . | . | . | . | . | . | . | . | . | . | . | . | . | . | . | . | . | . | . | . | . | . | . | . | . |
| 21-225_162F2_HC | . | . | . | . | . | . | . | . | . | . | . | . | . | . | . | . | . | . | . | . | . | . | . | . | . | . |
| 21-225_162H3_HC | . | . | . | . | . | . | . | . | . | . | . | . | . | . | . | . | . | . | . | . | . | . | . | . | . | . |
| 21-225_163F9_HC | . | . | . | . | . | . | . | . | . | . | . | . | . | . | . | . | . | . | . | . | . | . | . | . | . | . |
| 21-225_171A11_HC | . | . | . | . | . | . | . | . | . | . | . | . | . | . | . | . | . | . | . | . | . | . | . | . | . | . |
| 21-225_17C10_HC | . | . | . | . | . | . | . | . | . | . | . | . | . | . | . | . | . | . | . | . | . | . | . | . | L | . |
| 21-225_17D8_HC | . | . | . | . | . | . | . | . | . | . | . | . | . | . | L | . | . | . | . | . | . | . | . | . | . | . |
| 21-225_20B5_HC | . | . | . | . | . | . | . | . | . | . | . | . | . | . | . | . | . | . | . | . | . | . | . | . | . | . |
| 21-225_20B8_HC | . | . | . | . | . | . | . | . | . | . | . | . | . | . | . | . | . | . | . | . | . | . | . | . | . | . |
| 21-225_20C8_HC | . | . | . | . | . | . | . | . | . | . | . | . | . | . | . | . | . | . | . | . | . | . | . | . | . | . |
| 21-225_20F9_HC | . | . | . | . | . | . | . | . | . | . | . | . | . | . | . | . | . | . | . | . | . | . | . | . | . | . |
| 21-225_20G12_HC | . | . | . | . | . | . | . | . | . | . | . | . | . | . | . | . | . | . | . | . | . | . | . | . | . | . |
| 21-225_20H10_HC | . | . | . | . | . | . | . | . | . | . | . | . | . | . | . | . | . | . | . | . | . | . | . | . | I | . |
| 21-225_22H3_HC | . | . | . | . | . | . | . | . | . | . | . | . | . | . | . | . | . | . | . | . | . | . | . | . | . | F |
| 21-225_23E11_HC | . | . | . | . | . | . | . | . | . | . | . | . | . | . | . | . | . | . | . | . | . | . | . | . | . | . |
| 21-225_30H11_HC | . | . | . | . | . | . | . | . | . | . | . | . | . | . | . | . | . | . | . | . | . | . | . | . | . | . |
| 21-225_31G9_HC | . | . | . | . | . | . | . | . | . | . | . | . | . | . | . | . | . | . | . | . | . | . | . | . | . | . |
| 21-225_33C2_HC | . | . | . | . | . | . | . | . | . | . | . | F | . | . | . | . | . | . | . | . | . | . | . | . | I | F |
| 21-225_43E1_HC | . | . | . | . | . | . | . | . | . | . | . | F | . | . | L | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | A | K | N | S | L | Y | L | Q | M | N | S | L | R | A | E | D | T | A | V | Y |
| 21-225_53G1_HC | . | . | . | . | . | . | . | . | . | . | . | . | . | . | . | . | . | . | . | . | . | . | . | . | . | . |
| 21-225_56F1_HC | . | . | . | . | . | . | . | . | . | . | . | . | . | . | . | . | . | . | . | . | . | . | . | . | . | . |
| 21-225_56H7_HC | . | . | . | . | . | . | . | . | . | . | . | . | . | . | . | . | . | . | . | . | . | . | . | . | . | . |
| 21-225_58C5_HC | . | . | . | . | . | S | . | . | . | . | . | . | . | . | . | . | . | . | . | . | . | . | . | . | . | . |
| 21-225_65C12_HC | . | . | . | . | . | . | . | . | . | . | . | . | . | . | . | . | . | . | . | . | . | . | . | . | . | . |
| 21-225_6C12_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | R | G | - | - | - | - | - | - | - | - | - | - | - | - | - | - | - | - | - | - | - |
| 21-225_13D3_HC | . | . | . | . | . | . | . | . | . | . | . | . | . | . | . | . | . | . | . | . | . | . | . | . | . | . |
| 1-225_14B1_LC1_HC | . | . | . | . | . | . | . | . | . | . | . | . | . | . | . | . | . | . | . | . | . | . | . | . | . | . |
| 1-225_14B1_LC2_HC | . | . | . | . | . | . | . | . | . | . | . | . | . | . | . | . | . | . | . | . | . | . | . | . | . | . |
| 21-225_157G7_HC | . | . | . | . | . | . | . | . | . | . | . | . | . | . | . | . | . | . | . | . | . | . | . | . | . | . |
| 21-225_157H4_HC | . | . | . | . | . | . | . | . | . | . | . | . | . | . | . | . | . | . | . | . | . | . | . | . | . | . |
| 21-225_157H7_HC | . | . | V | . | . | . | . | . | . | . | . | . | . | . | . | . | . | . | . | . | . | . | . | . | . | . |
| 21-225_158F5_HC | . | . | . | . | . | . | . | . | . | . | . | . | . | . | . | . | . | . | . | . | . | . | . | . | . | . |
| 21-225_158G8_HC | . | . | . | . | . | . | . | . | . | . | . | . | . | . | . | . | . | . | . | . | . | . | . | . | . | . |
| 21-225_158H5_HC | . | . | . | . | . | . | . | . | . | . | . | . | . | . | . | . | . | . | . | . | . | . | . | . | . | . |
| 21-225_160F4_HC | . | . | . | . | . | . | . | . | . | . | . | . | . | . | . | . | . | . | . | . | . | . | . | . | . | . |
| 21-225_160H1_HC | F | . | . | . | . | K | - | . | . | . | . | . | . | . | . | . | . | . | . | . | . | . | . | . | . | . |
| 21-225_162F2_HC | . | . | . | . | . | . | . | . | . | . | . | . | . | . | . | . | . | . | . | . | . | . | . | . | . | . |
| 21-225_162H3_HC | . | . | . | . | . | . | . | . | . | . | . | . | . | . | . | . | . | . | . | . | . | . | . | . | . | . |
| 21-225_163F9_HC | . | . | . | . | . | . | . | . | . | . | . | . | . | . | . | . | . | . | . | . | . | . | . | . | . | . |
| 21-225_171A11_HC | . | . | . | . | . | . | . | . | . | . | . | . | . | . | . | . | . | . | . | . | . | . | . | . | . | . |
| 21-225_17C10_HC | . | . | . | . | . | . | . | . | . | . | . | . | . | . | . | . | . | . | . | . | . | . | . | . | . | . |
| 21-225_17D8_HC | . | . | . | . | . | . | . | . | . | . | . | . | . | . | . | . | . | . | . | . | . | . | . | . | . | . |
| 21-225_20B5_HC | . | . | . | . | . | . | . | . | . | . | . | . | . | . | . | . | . | . | . | . | . | . | . | . | . | . |
| 21-225_20B8_HC | . | . | . | . | . | . | . | . | . | . | . | . | . | . | . | . | . | . | . | . | . | . | . | . | . | . |
| 21-225_20C8_HC | . | . | . | . | . | . | . | . | . | . | . | . | . | . | . | . | . | . | . | . | . | . | . | . | . | . |
| 21-225_20F9_HC | . | . | . | . | . | . | . | . | . | . | . | . | . | . | . | . | . | . | . | . | . | . | . | . | . | . |
| 21-225_20G12_HC | . | . | . | . | . | . | . | . | . | . | . | . | . | . | . | . | . | . | . | . | . | . | . | . | . | . |
| 21-225_20H10_HC | . | . | . | . | . | . | . | . | . | . | . | . | . | . | . | . | . | . | . | . | . | . | . | . | . | . |
| 21-225_22H3_HC | . | . | . | . | . | . | . | . | . | . | . | . | . | . | . | . | . | . | . | . | . | . | . | . | . | . |
| 21-225_23E11_HC | . | . | . | . | . | . | . | . | . | . | . | . | . | . | . | . | . | . | . | . | . | . | . | . | . | . |
| 21-225_30H11_HC | . | . | . | . | . | . | . | . | . | . | . | . | . | . | . | . | . | . | . | . | . | . | . | . | . | . |
| 21-225_31G9_HC | . | . | . | . | . | . | . | . | . | . | . | . | . | . | . | . | . | . | . | . | . | . | . | . | . | . |
| 21-225_33C2_HC | . | . | . | . | . | . | . | . | . | . | . | . | . | . | . | . | . | . | . | . | . | . | . | . | . | . |
| 21-225_43E1_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | D | R | G | - | - | - | - | - | - | - | - | - | - | - | - | - | - | - | - | - | - | - |
| 21-225_53G1_HC | . | . | . | . | . | . | . | . | . | . | . | . | . | . | . | . | . | . | . | . | . | . | . | . | . | . |
| 21-225_56F1_HC | . | . | . | . | . | . | . | . | . | . | . | . | . | . | . | . | . | . | . | . | . | . | . | . | . | . |
| 21-225_56H7_HC | . | . | . | . | . | . | . | . | . | . | . | . | . | . | . | . | . | . | . | . | . | . | . | . | . | . |
| 21-225_58C5_HC | . | . | . | . | T | Y | S | G | . | . | . | . | . | . | . | . | . | . | . | . | . | . | . | . | . | . |
| 21-225_65C12_HC | . | . | . | . | . | A | Y | . | . | . | . | . | . | . | . | . | . | . | . | . | . | . | . | . | . | . |
| 21-225_6C12_HC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | - | - | S | S | W | G | Q | G | T | L | V | T | V | S | S |
| 21-225_13D3_HC | . | . | . | . | . | . | . | G | . | . | . | . | . | . | . | . | . | . | . |
| 1-225_14B1_LC1_HC | . | . | . | . | . | . | . | . | . | . | . | . | . | . | . | . | . | . | . |
| 1-225_14B1_LC2_HC | . | . | . | . | . | . | . | . | . | . | . | . | . | . | . | . | . | . | . |
| 21-225_157G7_HC | . | . | . | . | . | . | . | . | . | . | . | . | . | . | . | . | . | . | . |
| 21-225_157H4_HC | . | . | . | . | . | . | . | I | . | . | . | . | . | . | . | . | . | . | . |
| 21-225_157H7_HC | . | . | . | . | . | . | G | Y | . | . | . | . | . | . | . | . | . | . | . |
| 21-225_158F5_HC | . | . | . | . | . | . | . | . | . | . | . | . | . | . | . | . | . | . | . |
| 21-225_158G8_HC | . | . | . | . | . | . | . | L | . | . | . | . | . | . | . | . | . | . | . |
| 21-225_158H5_HC | . | . | . | . | . | . | . | . | . | . | . | . | . | . | . | . | . | . | . |
| 21-225_160F4_HC | . | . | . | . | . | . | . | . | . | . | . | . | . | . | . | . | . | . | . |
| 21-225_160H1_HC | . | . | . | . | . | . | D | Y | . | . | . | . | . | . | . | . | . | . | . |
| 21-225_162F2_HC | . | . | . | . | . | . | . | . | . | . | . | . | . | . | . | . | . | . | . |
| 21-225_162H3_HC | . | . | . | . | . | . | . | . | . | . | . | . | . | . | . | . | . | . | . |
| 21-225_163F9_HC | . | . | . | . | . | . | . | L | . | . | . | . | . | . | . | . | . | . | . |
| 21-225_171A11_HC | . | . | . | . | . | . | . | . | . | . | . | . | I | . | . | . | . | . | . |
| 21-225_17C10_HC | . | . | . | . | . | . | . | . | . | . | . | . | . | . | . | . | . | . | . |
| 21-225_17D8_HC | . | . | . | . | . | . | . | G | . | . | . | . | . | . | . | . | . | . | . |
| 21-225_20B5_HC | . | . | . | . | . | . | . | Y | . | . | . | . | . | . | . | . | . | . | . |
| 21-225_20B8_HC | . | . | . | . | . | . | . | G | . | . | . | . | . | . | . | . | . | . | . |
| 21-225_20C8_HC | . | . | . | . | . | . | . | L | . | . | . | . | . | . | . | . | . | . | . |
| 21-225_20F9_HC | . | . | . | . | . | . | . | C | . | . | . | . | . | . | . | . | I | . | . |
| 21-225_20G12_HC | . | . | . | . | . | . | . | Y | . | . | . | . | . | . | . | . | . | . | . |
| 21-225_20H10_HC | . | . | . | . | . | . | . | L | . | . | . | . | . | . | . | . | . | . | . |
| 21-225_22H3_HC | . | . | . | . | . | . | . | L | . | . | . | . | . | . | . | . | . | . | . |
| 21-225_23E11_HC | . | . | . | . | . | . | . | L | . | . | . | . | . | . | . | . | . | . | . |
| 21-225_30H11_HC | . | . | . | . | . | . | . | L | . | . | . | . | . | . | . | . | . | . | . |
| 21-225_31G9_HC | . | . | . | . | . | . | . | . | . | . | . | . | . | . | . | . | . | . | . |
| 21-225_33C2_HC | . | . | . | . | . | . | G | T | . | . | . | . | . | . | . | . | . | . | . |
| 21-225_43E1_HC | . | . | . | . | . | . | . | E | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | - | - | - | - | S | S | W | G | Q | G | T | L | V | T | V | S | S |
| 21-225_53G1_HC | . | . | . | . | . | . | . | T | . | . | . | . | . | . | . | . | . | . | . |
| 21-225_56F1_HC | . | . | . | . | . | . | . | . | . | . | . | . | . | . | . | . | . | . | . |
| 21-225_56H7_HC | . | . | . | . | . | . | . | . | . | . | . | . | . | . | . | . | . | . | . |
| 21-225_58C5_HC | . | . | . | . | S | F | D | Y | . | . | . | . | . | . | . | . | . | . | . |
| 21-225_65C12_HC | . | . | . | . | . | S | H | Y | . | . | . | . | . | . | . | . | . | . | . |
| 21-225_6C12_HC | . | . | . | . | . | . | . | L | . | . | . | . | . | . | . | . | . | . | . |

**FIGURE 57**

Table 59. Consensus 25-VH3|3-23/D6|6-19|RF2/JH3 (SEQ ID NO: 50276)

EVQLLES-GGGLVQPGGSLRLSCAASE-FTFSS-----YAMSWVRQAPGKGLEWVSVISGR---GGNTFYADSVKGRFTISRDNSKNTLYLQMNSLRAEDTAVYYCASRIAVAG------------------SEAFDIWGQGTMVTVSS wherein:

S at position 33 can be substituted with G

A at position 40 can be substituted with V

S at position 42 can be substituted with N or T

V at position 57 can be substituted with I or A

G at position 60 can be substituted with R

R at position 61 can be substituted with S

G at position 66 can be substituted with V, I or T

N at position 67 can be substituted with Y, S or T

T at position 68 can be substituted with A

F at position 69 can be substituted with Y

Y at position 70 can be substituted with N or S

K at position 75 can be substituted with R

I at position 110 can be substituted with L, M or V

A at position 113 can be substituted with D

S at position 133 can be substituted with N or Y

E at position 134 can be substituted with D

F at position 136 can be substituted with C

**FIGURE 57**

D at position 137 can be substituted with A or H

I at position 138 can be substituted with V

Xaa Tyr Xaa Met Xaa (SEQ ID NO: 50031)

Xaa Ile Ser Xaa Xaa Gly Xaa Xaa Xaa Xaa Xaa Ala Asp Ser Val Xaa Gly (SEQ ID NO: 50032)

Arg Xaa Ala Val Xaa Gly Xaa Xaa Ala Xaa Xaa Xaa (SEQ ID NO: 50033)

Ser Tyr Ala Met Ser (SEQ ID NO: 50498)

Val Ile Ser Gly Arg Gly Gly Asn Thr Phe Tyr Ala Asp Ser Val Lys Gly (SEQ ID NO: 50499)

Arg Ile Ala Val Ala Gly Ser Glu Ala Phe Asp Ile (SEQ ID NO: 50500)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | E | V | Q | L | L | E | S | - | G | G | G | L | V | Q | P | G | G | S | L | R | L | S | C | A | A | S |
| 21-225_10F12_HC | . | . | . | . | . | . | . | . | . | . | . | . | . | . | . | . | . | . | . | . | . | . | . | . | . | . |
| 21-225_11A5_HC | . | . | . | . | . | . | . | . | . | . | D | . | . | . | . | . | . | . | . | . | . | . | . | . | . | . |
| 21-225_16H6_HC | . | . | . | . | . | . | . | . | . | . | . | . | . | . | . | . | . | . | . | . | . | . | . | . | . | . |
| 21-225_17A12_HC | . | . | . | . | . | . | . | . | . | . | . | . | I | . | . | . | . | . | . | . | . | . | . | . | . | . |
| 21-225_17B4_HC | . | . | . | . | . | . | . | . | . | . | . | . | . | . | . | . | . | . | . | . | . | . | . | . | . | . |
| 21-225_17C6_HC | . | . | . | . | . | . | . | . | . | . | . | . | . | . | . | . | . | . | . | . | . | . | . | . | . | . |
| 21-225_17F11_HC | . | A | . | . | . | . | . | . | . | . | . | . | E | . | . | . | . | . | . | . | . | . | . | . | . | . |
| 21-225_17F7_HC | . | . | . | . | . | . | . | . | . | . | . | . | . | . | . | . | . | . | . | . | . | . | . | . | . | . |
| 21-225_18E12_HC | . | . | . | . | . | . | . | . | . | . | . | . | . | . | . | . | . | . | . | . | . | . | . | . | . | . |
| 21-225_200G1_HC | . | . | . | . | . | . | . | . | . | . | . | . | . | . | . | . | . | . | . | . | . | . | . | . | . | . |
| 21-225_20A4_HC | . | . | . | . | . | . | . | . | . | . | . | . | . | . | . | . | . | . | . | . | . | . | . | . | . | . |
| 21-225_21A2_HC | . | . | . | . | . | . | . | . | . | . | . | . | . | . | . | . | . | . | . | . | . | . | . | . | . | . |
| 21-225_21F4_HC | . | . | . | . | . | . | . | . | . | . | . | . | . | . | . | . | . | . | . | . | . | . | . | . | . | . |
| 21-225_22C5_HC | . | . | . | . | . | . | . | . | . | . | . | . | . | . | V | . | . | . | . | . | . | . | . | . | . | . |
| 21-225_22D5_HC | . | . | . | . | . | . | . | . | . | . | . | . | . | . | . | . | . | . | . | . | . | . | . | . | . | . |
| 21-225_22E4_HC | . | . | . | . | . | . | . | . | . | . | . | . | . | . | . | . | . | . | . | . | . | . | . | . | . | . |
| 21-225_22G10_HC | . | . | . | . | . | . | . | . | . | . | D | . | . | . | . | . | . | . | . | . | . | . | . | T | . | . |
| 21-225_22G3_HC | . | . | . | . | . | . | . | . | . | . | . | . | . | . | . | . | . | . | . | . | . | . | . | . | . | . |
| 21-225_3B1_HC | . | . | . | . | . | . | . | . | . | . | . | . | . | . | . | . | . | . | . | . | . | . | . | . | . | . |
| 21-225_3E3_HC | . | . | . | . | . | . | . | . | . | . | T | . | . | . | . | . | . | . | . | . | . | . | . | . | . | . |
| 21-225_48C3_HC | . | . | . | . | . | . | . | . | . | . | . | . | . | . | . | . | . | . | . | . | . | . | . | . | . | . |
| 21-225_48D1_HC | . | . | . | . | . | . | . | . | . | . | . | . | . | . | . | . | . | . | . | . | . | . | . | . | . | . |
| 21-225_49G12_HC | . | . | . | . | . | . | . | . | . | . | . | . | . | . | . | . | . | . | . | . | . | . | . | T | . | . |
| 21-225_4H2_HC | . | . | . | . | . | . | . | . | . | . | . | . | . | . | . | . | . | . | . | . | . | . | . | . | . | . |
| 21-225_50H10_HC | . | G | . | . | . | . | . | . | . | . | . | . | . | . | . | . | . | . | . | . | . | . | . | . | . | . |
| 21-225_50H8_HC | . | . | . | . | . | . | . | . | . | . | . | . | . | . | . | . | . | . | . | . | . | . | . | . | . | . |
| 21-225_51H10_HC | . | . | . | . | . | . | . | . | . | . | . | . | . | . | . | . | . | . | . | . | . | . | . | T | . | . |
| 21-225_5F7_HC | . | . | . | . | . | . | . | . | . | . | . | . | . | . | . | . | . | . | . | . | . | . | . | . | . | . |
| 21-225_5G2_HC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | E | V | Q | L | L | E | S | - | G | G | G | L | V | Q | P | G | G | S | L | R | L | S | C | A | A | S |
| 21-225_6D3_HC | . | . | . | . | . | . | . | . | . | . | . | . | . | . | . | . | . | . | . | . | . | . | . | . | . | . |
| 21-225_8E12_HC | . | . | . | . | . | . | . | . | . | . | . | . | . | . | . | . | . | . | . | . | . | . | . | . | . | . |
| 21-225_8G6_HC | . | . | . | . | . | . | . | . | . | . | . | . | . | . | . | . | . | . | . | . | . | . | . | V | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | E | - | F | T | F | S | S | - | - | - | - | - | Y | A | M | S | W | V | R | Q | A | P | G | K | G | L |
| 21-225_10F12_HC | . | . | . | . | . | . | . | . | . | . | . | . | . | . | . | . | . | . | . | . | . | . | . | . | . | . |
| 21-225_11A5_HC | . | . | . | . | . | . | . | . | . | . | . | . | . | . | . | . | . | . | . | . | . | . | . | . | . | . |
| 21-225_16H6_HC | G | . | . | . | . | . | . | . | . | . | . | . | . | . | . | N | . | . | . | . | . | . | . | M | . | . |
| 21-225_17A12_HC | . | . | . | . | . | . | . | . | . | . | . | . | . | . | . | . | . | . | . | . | D | . | . | . | . | . |
| 21-225_17B4_HC | . | . | . | . | . | . | . | . | . | . | . | . | . | . | . | N | . | . | . | . | . | . | . | . | . | . |
| 21-225_17C6_HC | . | . | . | . | . | . | . | . | . | . | . | . | . | . | . | N | . | . | . | . | . | . | . | . | . | . |
| 21-225_17F11_HC | G | . | . | . | . | . | . | . | . | . | . | . | . | . | . | . | . | . | . | . | . | . | . | . | . | . |
| 21-225_17F7_HC | . | . | . | . | . | . | . | . | . | . | . | . | . | . | . | N | . | . | . | . | . | . | . | . | . | . |
| 21-225_18E12_HC | . | . | . | . | . | . | . | . | . | . | . | . | . | . | . | . | . | . | . | . | . | . | . | T | . | . |
| 21-225_200G1_HC | G | . | . | . | . | . | . | . | . | . | . | . | . | . | . | . | . | . | . | . | . | . | . | . | . | . |
| 21-225_20A4_HC | G | . | . | . | . | . | . | . | . | . | . | . | . | . | . | T | . | . | . | . | G | . | . | M | . | . |
| 21-225_21A2_HC | K | . | . | . | . | . | . | . | . | . | . | . | . | . | . | N | . | . | . | . | . | . | . | . | . | . |
| 21-225_21F4_HC | . | . | . | . | . | . | . | . | . | . | . | . | . | . | . | N | . | . | . | . | . | . | . | . | . | . |
| 21-225_22C5_HC | G | . | . | . | . | . | . | . | . | . | . | . | . | . | . | . | . | . | . | . | . | . | . | M | . | . |
| 21-225_22D5_HC | . | . | . | . | . | . | . | . | . | . | . | . | . | . | . | . | . | . | . | . | . | . | . | . | . | . |
| 21-225_22E4_HC | . | . | . | . | . | . | . | . | . | . | . | . | . | . | . | . | . | . | . | . | . | . | . | . | . | . |
| 21-225_22G10_HC | G | . | . | . | . | . | . | . | . | . | . | . | . | . | . | N | . | . | . | . | . | . | . | . | . | . |
| 21-225_22G3_HC | . | . | . | . | . | . | . | . | . | . | . | . | . | . | . | N | . | . | . | . | . | . | . | . | . | . |
| 21-225_3B1_HC | . | . | . | . | . | . | . | . | . | . | . | . | . | . | . | N | . | . | . | . | . | . | . | . | . | . |
| 21-225_3E3_HC | . | . | . | . | . | . | . | . | . | . | . | . | . | V | . | . | . | . | . | . | . | . | . | . | . | . |
| 21-225_48C3_HC | R | . | . | . | . | . | . | . | . | . | . | . | . | . | . | . | . | . | . | . | . | . | . | . | . | . |
| 21-225_48D1_HC | R | . | . | . | . | . | . | . | . | . | . | . | . | . | . | . | . | . | . | . | . | . | . | . | . | . |
| 21-225_49G12_HC | G | . | . | . | . | . | . | . | . | . | . | . | . | . | . | . | . | . | . | . | . | . | . | . | . | . |
| 21-225_4H2_HC | G | . | . | . | . | . | G | . | . | . | . | . | . | . | . | N | . | . | . | . | . | . | . | M | . | . |
| 21-225_50H10_HC | R | . | . | . | . | . | . | . | . | . | . | . | . | . | . | . | . | . | . | . | . | . | . | . | . | . |
| 21-225_50H8_HC | R | . | . | . | . | . | . | . | . | . | . | . | . | . | . | . | . | . | . | . | . | . | . | . | . | . |
| 21-225_51H10_HC | G | . | . | . | . | . | . | . | . | . | . | . | . | . | . | . | . | . | . | . | . | . | . | . | . | . |
| 21-225_5F7_HC | . | . | . | . | . | . | . | . | . | . | . | . | . | . | . | . | . | . | . | . | . | . | . | . | . | . |
| 21-225_5G2_HC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | E | - | F | T | F | S | S | - | - | - | - | - | Y | A | M | S | W | V | R | Q | A | P | G | K | G | L |
| 21-225_6D3_HC | . | . | . | . | . | . | . | . | . | . | . | . | . | . | . | N | . | . | . | . | . | . | . | . | . | . |
| 21-225_8E12_HC | . | . | . | . | . | . | . | . | . | . | . | . | . | . | . | . | . | . | . | . | . | . | . | . | . | . |
| 21-225_8G6_HC | . | . | . | . | . | . | . | . | . | . | . | . | . | V | . | N | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | S | V | I | S | G | R | - | - | - | G | G | N | T | F | Y | A | D | S | V | K | G | R | F |
| 21-225_10F12_HC | . | . | . | . | I | . | . | . | . | . | . | . | . | . | . | . | . | . | . | . | . | . | . | . | . | . |
| 21-225_11A5_HC | . | . | I | . | . | . | . | . | . | . | . | . | . | V | . | . | . | . | . | . | . | . | . | . | . | . |
| 21-225_16H6_HC | . | . | . | . | . | . | . | R | S | . | . | . | . | . | Y | . | Y | . | . | . | . | . | . | . | . | . |
| 21-225_17A12_HC | . | . | . | . | I | . | . | . | . | . | . | . | . | . | . | A | . | . | . | . | . | . | . | . | . | . |
| 21-225_17B4_HC | . | . | . | . | . | . | . | . | . | . | . | . | . | . | . | . | . | . | . | . | . | . | . | . | . | . |
| 21-225_17C6_HC | . | . | . | . | . | . | . | . | . | . | . | . | . | . | . | . | . | . | . | . | . | . | . | . | . | . |
| 21-225_17F11_HC | . | . | . | . | . | . | . | . | . | . | . | . | . | . | . | . | Y | S | . | . | . | . | . | . | . | . |
| 21-225_17F7_HC | . | . | . | . | I | . | . | . | . | . | . | . | . | . | . | . | . | . | . | . | . | . | . | . | . | . |
| 21-225_18E12_HC | . | . | . | . | I | . | . | . | . | . | . | . | . | . | . | . | . | . | . | . | . | . | . | . | . | . |
| 21-225_200G1_HC | . | . | . | . | A | . | . | . | . | . | . | . | . | . | . | . | . | . | . | . | . | . | R | . | . | . |
| 21-225_20A4_HC | . | . | . | . | . | . | . | . | S | . | . | . | . | . | Y | . | Y | N | . | . | . | . | . | . | . | . |
| 21-225_21A2_HC | . | . | I | . | I | . | . | . | . | . | . | . | . | . | . | . | . | . | . | . | . | . | . | . | . | . |
| 21-225_21F4_HC | . | . | . | . | . | . | . | . | . | . | . | . | . | . | . | . | . | . | . | . | . | . | . | . | . | . |
| 21-225_22C5_HC | . | . | . | . | I | . | . | . | S | . | . | . | . | . | Y | . | Y | . | . | . | . | . | . | . | . | . |
| 21-225_22D5_HC | . | . | . | . | . | . | . | . | . | . | . | . | . | . | . | . | . | . | . | . | . | . | . | . | . | . |
| 21-225_22E4_HC | . | . | . | . | I | . | . | . | . | . | . | . | . | T | . | . | . | . | . | . | . | . | . | . | . | . |
| 21-225_22G10_HC | . | . | . | . | . | . | . | . | S | . | . | . | . | . | . | . | . | . | . | . | . | . | . | . | . | . |
| 21-225_22G3_HC | . | . | . | . | I | . | . | . | . | . | . | . | . | V | . | . | . | . | . | . | . | . | . | . | . | . |
| 21-225_3B1_HC | . | . | . | . | . | . | . | . | . | . | . | . | . | I | . | . | . | . | . | . | . | . | . | . | . | . |
| 21-225_3E3_HC | . | . | . | . | I | . | . | . | . | . | . | . | . | . | . | . | . | . | . | . | . | . | . | . | . | . |
| 21-225_48C3_HC | . | . | . | . | . | . | . | . | . | . | . | . | . | . | S | . | . | . | . | . | . | . | . | . | . | . |
| 21-225_48D1_HC | . | . | . | . | . | . | . | . | . | . | . | . | . | . | S | . | . | . | . | . | . | . | . | . | . | . |
| 21-225_49G12_HC | . | . | . | . | . | . | . | . | S | . | . | . | . | . | T | . | . | . | . | . | . | . | . | . | . | L |
| 21-225_4H2_HC | . | . | . | . | . | . | . | R | S | . | . | . | . | . | Y | . | Y | . | . | . | . | . | . | . | . | . |
| 21-225_50H10_HC | . | . | . | . | . | . | . | . | . | . | . | . | . | . | S | . | . | . | . | . | . | . | . | . | . | . |
| 21-225_50H8_HC | . | . | . | . | . | . | . | . | . | . | . | . | . | . | T | . | . | . | . | . | . | . | . | . | . | . |
| 21-225_51H10_HC | . | . | . | . | . | . | . | . | S | . | . | . | . | . | T | . | . | . | . | . | . | . | . | . | . | L |
| 21-225_5F7_HC | . | . | . | . | I | . | . | . | . | . | . | . | . | . | . | . | . | . | . | . | . | . | . | . | . | . |
| 21-225_5G2_HC | . | . | . | . | . | . | . | . | . | . | . | . | . | V | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | S | V | I | S | G | R | - | - | - | G | G | N | T | F | Y | A | D | S | V | K | G | R | F |
| 21-225_6D3_HC | . | . | . | . | . | . | . | . | . | . | . | . | . | . | . | . | . | . | . | . | . | . | . | . | . | . |
| 21-225_8E12_HC | . | . | . | . | I | . | . | . | . | . | . | . | . | . | . | . | . | . | . | . | . | . | . | . | . | . |
| 21-225_8G6_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_10F12_HC | . | . | . | . | . | . | . | . | . | . | . | . | . | . | . | . | . | . | . | . | . | . | . | . | . | . |
| 21-225_11A5_HC | . | . | . | . | . | . | . | . | . | . | . | . | . | . | . | . | . | . | . | . | . | . | . | . | . | . |
| 21-225_16H6_HC | . | . | . | . | . | . | . | . | . | . | . | . | . | . | . | . | . | . | . | . | . | . | . | . | . | . |
| 21-225_17A12_HC | . | . | . | . | . | . | . | . | . | . | . | . | . | . | . | . | . | . | . | . | . | . | . | . | . | . |
| 21-225_17B4_HC | . | . | . | . | . | . | . | . | . | . | . | . | . | . | . | . | . | . | . | . | . | . | . | . | . | . |
| 21-225_17C6_HC | . | . | . | . | . | . | . | . | . | . | . | F | . | . | . | . | . | . | . | . | . | . | . | . | . | . |
| 21-225_17F11_HC | . | . | . | . | . | . | . | . | . | . | . | . | . | . | . | . | . | . | . | . | . | . | . | . | . | . |
| 21-225_17F7_HC | . | . | . | . | . | . | . | . | . | . | . | . | . | . | . | . | . | . | . | . | . | . | . | . | . | . |
| 21-225_18E12_HC | . | . | . | . | . | . | . | . | . | . | . | . | . | . | . | . | . | . | . | . | . | . | . | . | . | . |
| 21-225_200G1_HC | . | . | . | . | . | . | . | . | . | . | . | . | . | . | . | . | . | . | . | . | . | . | . | . | . | . |
| 21-225_20A4_HC | . | . | . | . | . | . | . | . | . | . | . | . | . | . | . | . | . | . | . | V | . | . | . | . | . | . |
| 21-225_21A2_HC | . | . | . | . | . | . | . | . | . | . | . | . | . | . | . | . | . | . | . | . | . | . | . | . | . | . |
| 21-225_21F4_HC | . | . | . | . | . | . | . | . | . | . | . | . | . | . | . | . | . | . | . | . | . | . | . | . | . | . |
| 21-225_22C5_HC | . | . | . | . | . | . | . | . | . | . | . | . | . | . | . | . | . | . | . | . | . | . | . | . | . | . |
| 21-225_22D5_HC | . | . | . | . | . | . | . | . | . | . | . | . | . | . | . | . | . | . | . | . | . | . | . | . | . | . |
| 21-225_22E4_HC | . | . | . | . | V | . | . | . | . | . | . | . | . | . | . | . | . | . | . | . | . | . | . | . | . | . |
| 21-225_22G10_HC | . | . | . | . | . | . | . | . | . | . | . | F | . | H | . | . | . | . | . | . | . | . | . | . | . | . |
| 21-225_22G3_HC | . | . | . | . | . | . | . | . | . | . | . | . | . | . | . | . | . | . | . | . | . | . | . | . | . | . |
| 21-225_3B1_HC | . | . | . | . | . | . | . | . | . | . | . | . | . | . | . | . | . | . | . | . | . | . | . | . | . | . |
| 21-225_3E3_HC | . | . | . | . | . | . | . | . | . | . | . | . | . | . | . | . | . | . | . | . | . | . | . | . | L | . |
| 21-225_48C3_HC | . | . | . | . | . | . | . | . | . | . | . | . | . | . | . | . | . | . | . | . | . | . | . | . | . | . |
| 21-225_48D1_HC | . | . | . | . | . | . | . | . | . | . | . | . | . | . | . | . | . | . | . | . | . | . | . | . | . | . |
| 21-225_49G12_HC | . | . | . | . | . | . | . | . | . | . | . | . | . | . | . | . | . | . | . | . | . | . | . | . | . | . |
| 21-225_4H2_HC | . | . | . | . | . | . | . | . | . | . | . | . | . | . | . | . | . | . | . | . | . | . | . | . | . | . |
| 21-225_50H10_HC | . | . | . | . | . | . | . | . | . | . | . | . | . | . | . | . | . | . | . | . | . | . | . | . | . | . |
| 21-225_50H8_HC | . | . | . | . | . | . | . | . | . | . | . | . | . | . | . | . | . | . | . | . | . | . | . | . | . | . |
| 21-225_51H10_HC | . | . | . | . | . | . | . | . | . | . | . | . | . | . | . | . | . | . | . | . | . | . | . | . | . | . |
| 21-225_5F7_HC | . | . | . | . | . | . | . | . | . | . | . | . | . | . | . | . | . | . | . | . | . | . | . | . | . | . |
| 21-225_5G2_HC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_6D3_HC | . | . | . | . | . | . | . | . | . | . | . | . | . | . | . | . | . | . | . | . | . | . | . | . | . | . |
| 21-225_8E12_HC | . | . | . | . | . | . | . | . | . | . | . | . | . | . | . | . | . | . | . | . | . | . | . | . | . | . |
| 21-225_8G6_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | S | R | I | A | V | A | G | - | - | - | - | - | - | - | - | - | - | - | - | - | - | - | - |
| 21-225_10F12_HC | . | . | . | . | . | V | . | . | . | . | . | . | . | . | . | . | . | . | . | . | . | . | . | . | . | . |
| 21-225_11A5_HC | . | . | . | . | . | . | . | . | . | . | . | . | . | . | . | . | . | . | . | . | . | . | . | . | . | . |
| 21-225_16H6_HC | . | . | . | . | . | L | . | . | . | . | . | . | . | . | . | . | . | . | . | . | . | . | . | . | . | . |
| 21-225_17A12_HC | . | . | . | . | . | . | . | . | . | . | . | . | . | . | . | . | . | . | . | . | . | . | . | . | . | . |
| 21-225_17B4_HC | . | . | . | . | . | L | . | . | . | . | . | . | . | . | . | . | . | . | . | . | . | . | . | . | . | . |
| 21-225_17C6_HC | . | . | . | . | . | L | . | . | . | . | . | . | . | . | . | . | . | . | . | . | . | . | . | . | . | . |
| 21-225_17F11_HC | . | . | . | . | . | L | . | . | . | . | . | . | . | . | . | . | . | . | . | . | . | . | . | . | . | . |
| 21-225_17F7_HC | . | . | . | . | . | L | . | . | . | . | . | . | . | . | . | . | . | . | . | . | . | . | . | . | . | . |
| 21-225_18E12_HC | . | . | . | . | . | L | . | . | . | . | . | . | . | . | . | . | . | . | . | . | . | . | . | . | . | . |
| 21-225_200G1_HC | . | . | . | R | . | . | . | . | D | . | . | . | . | . | . | . | . | . | . | . | . | . | . | . | . | . |
| 21-225_20A4_HC | . | . | . | . | . | . | . | . | . | . | . | . | . | . | . | . | . | . | . | . | . | . | . | . | . | . |
| 21-225_21A2_HC | . | . | . | . | . | L | . | . | . | . | . | . | . | . | . | . | . | . | . | . | . | . | . | . | . | . |
| 21-225_21F4_HC | . | . | . | . | . | L | . | . | . | . | . | . | . | . | . | . | . | . | . | . | . | . | . | . | . | . |
| 21-225_22C5_HC | . | . | . | . | . | . | . | . | . | . | . | . | . | . | . | . | . | . | . | . | . | . | . | . | . | . |
| 21-225_22D5_HC | . | . | . | . | . | . | . | . | . | . | . | . | . | . | . | . | . | . | . | . | . | . | . | . | . | . |
| 21-225_22E4_HC | . | . | . | . | . | . | . | . | . | . | . | . | . | . | . | . | . | . | . | . | . | . | . | . | . | . |
| 21-225_22G10_HC | . | . | . | . | . | M | . | . | . | . | . | . | . | . | . | . | . | . | . | . | . | . | . | . | . | . |
| 21-225_22G3_HC | . | . | . | . | . | L | . | . | . | . | . | . | . | . | . | . | . | . | . | . | . | . | . | . | . | . |
| 21-225_3B1_HC | . | . | . | . | . | L | . | . | . | . | . | . | . | . | . | . | . | . | . | . | . | . | . | . | . | . |
| 21-225_3E3_HC | . | . | . | . | . | L | . | . | . | . | . | . | . | . | . | . | . | . | . | . | . | . | . | . | . | . |
| 21-225_48C3_HC | . | . | . | R | . | . | . | . | . | . | . | . | . | . | . | . | . | . | . | . | . | . | . | . | . | . |
| 21-225_48D1_HC | . | . | . | R | . | . | . | . | . | . | . | . | . | . | . | . | . | . | . | . | . | . | . | . | . | . |
| 21-225_49G12_HC | . | . | . | R | . | . | . | . | . | . | . | . | . | . | . | . | . | . | . | . | . | . | . | . | . | . |
| 21-225_4H2_HC | . | . | . | . | . | L | . | . | . | . | . | . | . | . | . | . | . | . | . | . | . | . | . | . | . | . |
| 21-225_50H10_HC | . | . | . | R | . | . | . | . | . | . | . | . | . | . | . | . | . | . | . | . | . | . | . | . | . | . |
| 21-225_50H8_HC | . | . | . | R | . | . | . | . | . | . | . | . | . | . | . | . | . | . | . | . | . | . | . | . | . | . |
| 21-225_51H10_HC | . | . | . | R | . | . | . | . | . | . | . | . | . | . | . | . | . | . | . | . | . | . | . | . | . | . |
| 21-225_5F7_HC | . | . | . | . | . | . | . | . | . | . | . | . | . | . | . | . | . | . | . | . | . | . | . | . | . | . |
| 21-225_5G2_HC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | S | R | I | A | V | A | G | - | - | - | - | - | - | - | - | - | - | - | - | - | - | - | - |
| 21-225_6D3_HC | F | . | . | . | . | L | . | . | . | . | . | . | . | . | . | . | . | . | . | . | . | . | . | . | . | . |
| 21-225_8E12_HC | . | . | . | . | . | M | . | . | . | . | . | . | . | . | . | . | . | . | . | . | . | . | . | . | . | . |
| 21-225_8G6_HC | . | . | . | . | . | L | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | S | E | A | F | D | I | W | G | Q | G | T | M | V | T | V | S | S |
| 21-225_10F12_HC | . | . | . | . | . | . | A | . | . | . | . | . | . | . | . | . | . | . | . |
| 21-225_11A5_HC | . | . | . | . | . | . | . | . | . | . | . | . | . | . | . | . | . | . | . |
| 21-225_16H6_HC | . | . | . | . | . | . | . | . | . | . | . | . | . | . | . | . | . | . | . |
| 21-225_17A12_HC | . | . | . | . | . | . | . | . | . | . | . | . | . | . | . | . | . | . | . |
| 21-225_17B4_HC | . | . | . | . | . | . | . | . | . | . | . | . | . | . | . | . | . | . | . |
| 21-225_17C6_HC | . | . | . | . | . | . | . | . | . | . | . | . | . | . | . | . | . | . | . |
| 21-225_17F11_HC | . | . | . | . | . | . | H | . | . | . | . | . | . | . | . | . | . | . | . |
| 21-225_17F7_HC | . | . | . | . | . | . | . | . | . | . | . | . | . | . | . | . | . | . | . |
| 21-225_18E12_HC | . | . | . | . | . | . | . | . | . | . | . | . | . | . | . | . | . | . | . |
| 21-225_200G1_HC | . | . | Y | D | . | . | . | V | . | . | . | . | . | . | . | . | . | . | . |
| 21-225_20A4_HC | . | . | . | . | . | . | . | . | . | . | . | . | . | . | . | . | . | . | . |
| 21-225_21A2_HC | . | . | . | . | . | . | . | . | . | . | . | . | . | . | . | . | . | . | . |
| 21-225_21F4_HC | . | . | . | . | . | C | . | . | . | . | . | . | . | . | . | . | . | . | . |
| 21-225_22C5_HC | . | . | . | . | . | . | . | . | . | . | . | . | . | . | . | . | . | . | . |
| 21-225_22D5_HC | . | . | . | . | . | . | . | . | . | . | . | . | . | . | . | . | . | . | . |
| 21-225_22E4_HC | . | . | . | . | . | . | . | . | . | . | . | . | . | . | . | . | . | . | . |
| 21-225_22G10_HC | . | . | . | . | . | . | . | . | . | . | . | . | . | . | . | . | . | . | . |
| 21-225_22G3_HC | . | . | . | . | . | . | . | . | . | . | . | . | . | . | . | . | . | . | . |
| 21-225_3B1_HC | . | . | . | . | . | . | . | . | . | . | . | . | . | . | . | . | . | . | . |
| 21-225_3E3_HC | . | . | . | . | . | . | . | . | . | . | . | . | . | . | . | . | . | . | . |
| 21-225_48C3_HC | . | . | N | . | . | . | . | . | . | . | . | . | . | . | . | . | . | . | . |
| 21-225_48D1_HC | . | . | N | . | . | . | . | . | . | . | . | . | . | . | . | . | . | . | . |
| 21-225_49G12_HC | . | . | N | D | . | . | . | . | . | . | . | . | . | . | . | . | . | . | . |
| 21-225_4H2_HC | . | . | . | . | . | . | . | . | . | . | . | . | . | . | . | . | . | . | . |
| 21-225_50H10_HC | . | . | N | . | . | . | . | . | . | . | . | . | . | . | . | . | . | . | . |
| 21-225_50H8_HC | . | . | N | . | . | . | . | . | . | . | . | . | . | . | . | . | . | . | . |
| 21-225_51H10_HC | . | . | N | D | . | . | . | . | . | . | . | . | . | . | . | . | . | . | . |
| 21-225_5F7_HC | . | . | . | . | . | . | A | . | . | . | . | . | . | . | . | . | . | . | . |
| 21-225_5G2_HC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | S | E | A | F | D | I | W | G | Q | G | T | M | V | T | V | S | S |
| 21-225_6D3_HC | . | . | . | . | . | . | . | . | . | . | . | . | . | . | . | . | . | . | . |
| 21-225_8E12_HC | . | . | . | . | . | . | . | . | . | . | . | . | . | . | . | . | . | . | . |
| 21-225_8G6_HC | . | . | . | . | . | . | . | . | . | . | . | . | . | . | . | S | . | . | . |

**FIGURE 57**

Table 60. Consensus 26- VH3|3-23/D5|5-12|RF3/JH6 (SEQ ID NO: 50277):

EVQLLES-GGGLVQPGGSLRLSCAASG-FTFSS-----YAMSWVRQAPGKGLEWVSAISGR---GGSTFHADSVKGRFTISRDNSKNTLYLQMNSLRAEDTAVYYCVKGELLEDY----------------YFYGMDVWGQGTTVTVSS wherein:

S at position 33 can be substituted with N

Y at position 39 can be substituted with C

A at position 40 can be substituted with V

S at position 42 can be substituted with N

A at position 57 can be substituted with T or S

I at position 58 can be substituted with L

G at position 60 can be substituted with R or V

R at position 61 can be substituted with G

S at position 67 can be substituted with N

T at position 68 can be substituted with I

F at position 69 can be substituted with Y

H at position 70 can be substituted with Y or N

V at position 74 can be substituted with E or M

G at position 109 can be substituted with W

E at position 110 can be substituted with G

E at position 113 can be substituted with Y

D at position 114 can be substituted with S or N

**FIGURE 57**

null (-) at position 116 can be substituted with E

Y at position 132 can be substituted with S

F at position 133 can be substituted with Y

Y at position 134 can be substituted with F

G at position 135 can be substituted with A

M at position 136 can be substituted with I or L

Xaa Xaa Xaa Met Xaa (SEQ ID NO: 50034)

Xaa Xaa Ser Xaa Xaa Gly Gly Xaa Xaa Xaa Xaa Ala Asp Ser Xaa Lys Gly (SEQ ID NO: 50035)

Xaa Xaa Leu Leu Xaa Xaa Tyr Xaa Xaa Xaa Xaa Xaa Xaa Asp Val (SEQ ID NO: 50036)

Ser Tyr Ala Met Ser (SEQ ID NO: 50501)

Ala Ile Ser Gly Arg Gly Gly Ser Thr Phe His Ala Asp Ser Val Lys Gly (SEQ ID NO: 50502)

Gly Glu Leu Leu Glu Asp Tyr Tyr Phe Tyr Gly Met Asp Val (SEQ ID NO: 50503)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | E | V | Q | L | L | E | S | - | G | G | G | L | V | Q | P | G | G | S | L | R | L | S | C | A | A | S |
| 21-225_12D8_HC | . | . | . | . | . | . | . | . | . | . | . | . | . | . | . | . | . | . | . | . | . | . | . | . | . | . |
| 21-225_17F12_HC | . | . | . | . | . | . | . | . | . | . | . | . | . | . | . | . | . | . | . | . | . | . | . | . | . | . |
| 21-225_21H4_HC | . | . | . | . | . | . | . | . | . | . | . | . | . | . | . | . | . | . | . | . | . | . | . | . | . | . |
| 21-225_24B1_HC | . | . | . | . | . | . | . | . | . | . | . | . | . | . | . | . | . | . | . | . | . | . | . | . | . | . |
| 21-225_30D1_HC | . | . | . | V | . | . | . | . | . | . | . | . | . | . | . | . | . | . | . | . | . | . | . | . | . | . |
| 21-225_30E3_HC | . | . | . | V | . | . | . | . | . | . | . | . | . | . | . | . | . | . | . | . | . | . | . | . | . | . |
| 21-225_30E9_HC | . | . | . | V | . | . | . | . | . | . | . | . | . | . | . | . | . | . | . | . | . | . | . | . | . | . |
| 21-225_30G11_HC | . | . | . | . | . | . | . | . | . | . | . | . | . | . | . | . | . | . | . | . | . | . | . | . | . | . |
| 21-225_31A8_HC | . | . | . | . | . | . | . | . | . | . | . | . | . | . | . | . | . | . | . | . | . | . | . | . | . | . |
| 21-225_31B8_HC | . | . | . | . | . | . | . | . | . | . | . | . | . | . | . | . | . | . | . | . | . | . | . | . | . | . |
| 21-225_31H3_HC | . | . | . | V | . | . | . | . | . | . | . | . | . | . | . | . | . | . | . | . | . | . | . | . | . | . |
| 21-225_31H5_HC | . | . | . | . | . | . | . | . | . | . | . | . | . | . | . | . | . | . | . | . | . | . | . | . | . | . |
| 21-225_32G1_HC | . | . | . | . | . | . | . | . | . | . | . | . | A | . | . | . | . | . | . | . | . | . | . | . | . | . |
| 21-225_32G12_HC | . | . | . | . | . | . | . | . | . | . | . | . | L | . | . | . | . | . | . | . | . | . | . | . | . | . |
| 21-225_33A4_HC | . | . | . | . | . | . | . | . | . | . | . | . | . | . | S | . | . | . | . | . | . | . | . | E | . | . |
| 21-225_33A7_HC | . | . | . | . | . | . | . | . | . | . | . | . | . | . | . | . | . | . | . | . | . | . | . | . | . | . |
| 21-225_33B8_HC | . | . | . | . | . | . | . | . | . | . | . | . | . | . | . | . | . | . | . | . | . | . | . | . | . | . |
| 21-225_33F1_HC | . | . | . | . | . | . | . | . | . | . | . | . | L | . | . | . | . | . | . | . | . | . | . | . | . | . |
| 21-225_33G7_HC | . | . | . | . | . | . | . | . | . | . | . | . | . | . | . | . | . | . | . | . | . | . | . | . | . | . |
| 21-225_34A6_HC | . | . | . | . | . | . | . | . | . | . | . | . | . | . | . | . | . | . | . | . | . | . | . | . | . | . |
| 21-225_34G8_HC | . | . | . | . | . | . | . | . | . | . | . | . | . | . | . | . | . | . | . | . | . | . | . | . | . | . |
| 21-225_34G9_HC | . | . | . | . | V | . | . | . | . | . | . | . | . | . | . | . | . | . | . | . | . | . | . | . | . | . |
| 21-225_34H11_HC | . | . | . | . | . | . | . | . | . | . | . | . | . | . | . | . | . | . | . | . | . | . | . | . | . | . |
| 21-225_35A6_HC | . | . | . | . | . | . | . | . | . | . | . | . | . | . | . | . | . | . | . | . | . | . | . | . | . | . |
| 21-225_35B7_HC | . | . | . | . | . | . | . | . | . | . | . | . | . | . | . | . | . | . | . | . | . | . | . | . | . | . |
| 21-225_35D1_HC | . | . | . | . | . | . | . | . | . | . | . | . | . | . | . | . | . | . | . | . | . | . | . | . | . | . |
| 21-225_35F11_HC | . | . | . | . | . | . | . | . | . | . | . | . | . | . | . | . | . | . | . | . | . | . | . | . | . | . |
| 21-225_36A5_HC | . | . | . | . | . | . | . | . | . | . | . | . | . | . | . | . | . | . | . | . | . | . | . | . | . | . |
| 21-225_36C5_HC | . | . | . | . | V | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | E | V | Q | L | L | E | S | - | G | G | G | L | V | Q | P | G | G | S | L | R | L | S | C | A | A | S |
| 21-225_4G12_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | A | M | S | W | V | R | Q | A | P | G | K | G | L |
| 21-225_12D8_HC | . | . | . | . | . | . | . | . | . | . | . | . | . | V | . | . | . | . | . | . | . | . | . | . | . | . |
| 21-225_17F12_HC | . | . | . | . | . | . | . | . | . | . | . | . | . | . | . | . | . | . | . | . | . | . | . | . | . | . |
| 21-225_21H4_HC | . | . | . | . | . | . | . | . | . | . | . | . | . | . | . | . | . | . | . | . | . | . | . | . | . | . |
| 21-225_24B1_HC | . | . | . | . | . | . | . | . | . | . | . | . | . | . | . | . | . | . | . | . | . | . | . | . | . | . |
| 21-225_30D1_HC | . | . | . | . | . | . | . | . | . | . | . | . | . | . | . | . | . | . | . | . | . | . | . | . | . | . |
| 21-225_30E3_HC | . | . | . | . | . | . | . | . | . | . | . | . | . | . | . | N | . | . | . | . | . | . | . | . | . | . |
| 21-225_30E9_HC | . | . | . | . | . | . | . | . | . | . | . | . | C | . | . | N | . | . | . | . | . | . | . | . | . | . |
| 21-225_30G11_HC | . | . | . | . | . | . | . | . | . | . | . | . | . | . | . | . | . | . | . | . | . | . | . | . | . | . |
| 21-225_31A8_HC | . | . | . | . | . | . | . | . | . | . | . | . | . | . | . | . | . | . | . | . | . | . | . | . | . | . |
| 21-225_31B8_HC | . | . | . | . | . | . | . | . | . | . | . | . | . | . | . | . | . | . | . | . | . | . | . | . | . | . |
| 21-225_31H3_HC | . | . | . | . | . | . | . | . | . | . | . | . | . | . | . | N | . | . | . | . | . | . | . | . | . | . |
| 21-225_31H5_HC | . | . | . | . | . | . | . | . | . | . | . | . | . | . | . | . | . | . | . | . | . | . | . | . | . | . |
| 21-225_32G1_HC | . | . | . | . | . | . | . | . | . | . | . | . | . | . | . | . | . | . | . | . | . | . | . | . | . | . |
| 21-225_32G12_HC | . | . | . | . | . | N | . | . | . | . | . | . | . | . | . | . | . | . | . | . | . | . | . | . | . | . |
| 21-225_33A4_HC | . | . | . | I | . | . | . | . | . | . | . | . | . | . | . | N | . | . | . | . | V | . | . | . | . | . |
| 21-225_33A7_HC | . | . | . | . | . | . | . | . | . | . | . | . | C | . | . | . | . | . | . | . | . | . | . | . | . | . |
| 21-225_33B8_HC | . | . | . | . | . | . | . | . | . | . | . | . | . | . | . | . | . | . | . | . | . | . | . | . | . | . |
| 21-225_33F1_HC | . | . | . | . | . | . | . | . | . | . | . | . | . | . | . | . | . | . | . | . | . | . | . | . | . | . |
| 21-225_33G7_HC | . | . | . | . | . | . | N | . | . | . | . | . | . | . | . | . | . | . | . | . | . | . | . | . | . | . |
| 21-225_34A6_HC | . | . | . | . | . | N | N | . | . | . | . | . | . | . | . | N | . | . | . | . | . | . | . | . | . | . |
| 21-225_34G8_HC | . | . | . | . | . | . | . | . | . | . | . | . | . | . | . | . | . | . | . | . | . | . | . | . | . | . |
| 21-225_34G9_HC | . | . | . | . | . | . | . | . | . | . | . | . | . | . | . | . | . | . | . | . | . | . | . | . | . | . |
| 21-225_34H11_HC | . | . | . | . | . | . | . | . | . | . | . | . | . | . | . | N | . | . | . | . | . | . | . | E | . | . |
| 21-225_35A6_HC | . | . | . | . | . | . | . | . | . | . | . | . | . | . | . | . | . | . | . | . | . | . | . | . | . | . |
| 21-225_35B7_HC | . | . | . | . | . | . | . | . | . | . | . | . | . | . | . | . | . | . | . | . | . | . | . | . | . | . |
| 21-225_35D1_HC | . | . | . | . | . | . | . | . | . | . | . | . | . | . | . | . | . | . | . | . | . | . | . | . | . | . |
| 21-225_35F11_HC | . | . | . | . | . | . | . | . | . | . | . | . | . | . | . | . | . | . | . | . | . | . | . | . | . | . |
| 21-225_36A5_HC | . | . | . | . | . | N | N | . | . | . | . | . | . | . | . | N | . | . | . | . | . | . | . | . | . | . |
| 21-225_36C5_HC | . | . | . | . | . | . | . | . | . | . | . | . | . | . | . | N | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | A | M | S | W | V | R | Q | A | P | G | K | G | L |
| 21-225_4G12_HC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | S | A | I | S | G | R | - | - | - | G | G | S | T | F | H | A | D | S | V | K | G | R | F |
| 21-225_12D8_HC | . | . | . | . | T | . | . | V | G | . | . | . | . | . | . | . | Y | Y | . | . | . | . | . | . | . | . |
| 21-225_17F12_HC | . | . | . | . | . | . | . | . | G | . | . | . | . | . | . | . | Y | Y | . | . | . | . | . | . | . | V |
| 21-225_21H4_HC | . | . | . | . | . | L | . | . | . | . | . | . | . | . | . | . | Y | Y | . | . | . | . | . | . | . | . |
| 21-225_24B1_HC | . | . | . | . | . | . | . | . | . | . | . | . | . | . | . | . | Y | Y | . | . | . | . | . | . | . | . |
| 21-225_30D1_HC | . | . | . | . | . | . | . | . | . | . | . | . | . | . | . | . | . | . | . | . | . | . | . | . | . | . |
| 21-225_30E3_HC | . | . | . | . | . | . | . | . | . | . | . | . | . | . | . | . | . | Y | . | . | . | E | . | . | . | . |
| 21-225_30E9_HC | . | . | . | . | . | . | . | . | . | . | . | . | . | . | . | . | . | Y | . | . | . | . | . | . | . | . |
| 21-225_30G11_HC | . | . | . | . | . | . | . | . | . | . | . | . | . | . | . | . | . | N | . | . | . | . | . | . | . | . |
| 21-225_31A8_HC | . | . | . | . | . | . | . | . | . | . | . | . | . | . | . | . | . | . | . | . | . | . | . | . | . | . |
| 21-225_31B8_HC | . | . | . | . | . | . | . | . | . | . | . | . | . | . | . | . | . | . | . | . | . | . | . | . | . | . |
| 21-225_31H3_HC | . | . | . | . | . | . | . | . | . | . | . | . | . | . | N | . | . | Y | . | . | . | . | . | . | . | . |
| 21-225_31H5_HC | . | . | . | . | . | . | . | . | . | . | . | . | . | . | . | I | . | . | . | . | . | . | . | . | . | . |
| 21-225_32G1_HC | . | . | . | . | . | . | . | . | . | . | . | . | . | . | . | . | . | . | . | . | . | . | . | . | . | . |
| 21-225_32G12_HC | . | . | . | . | S | . | . | . | . | . | . | . | . | . | . | . | . | . | . | . | . | . | . | . | . | . |
| 21-225_33A4_HC | . | . | . | . | . | . | . | R | . | . | . | . | . | . | . | . | . | Y | . | . | . | . | . | . | . | . |
| 21-225_33A7_HC | . | . | . | . | . | . | . | . | . | . | . | . | . | . | . | . | . | . | . | . | . | . | . | . | . | . |
| 21-225_33B8_HC | . | . | . | . | . | . | . | . | . | . | . | . | . | . | . | . | . | . | . | . | . | . | . | . | . | . |
| 21-225_33F1_HC | . | . | . | . | S | . | . | . | . | . | . | . | . | . | . | . | . | . | . | . | . | . | . | . | . | . |
| 21-225_33G7_HC | . | . | . | . | . | . | . | R | . | . | . | . | . | . | . | . | . | Y | . | . | . | . | . | . | Q | . |
| 21-225_34A6_HC | . | . | . | . | . | . | . | R | . | . | . | . | . | . | . | . | . | Y | . | . | . | . | . | . | . | . |
| 21-225_34G8_HC | . | . | . | . | . | . | . | . | . | . | . | . | . | . | . | . | . | . | . | . | . | . | . | . | . | . |
| 21-225_34G9_HC | . | . | . | . | . | . | . | . | . | . | . | . | . | . | . | . | . | . | . | . | . | . | . | . | . | . |
| 21-225_34H11_HC | . | . | . | . | . | . | . | . | . | . | . | . | . | . | . | . | . | Y | . | . | . | . | . | . | . | . |
| 21-225_35A6_HC | . | . | . | . | T | . | . | . | . | . | . | . | . | . | . | . | . | . | . | . | . | . | . | . | . | . |
| 21-225_35B7_HC | . | . | . | . | . | . | . | . | . | . | . | . | . | . | . | . | . | . | . | . | . | . | . | . | . | . |
| 21-225_35D1_HC | . | . | . | . | . | . | . | . | . | . | . | . | . | . | . | . | . | . | . | . | . | . | . | . | . | . |
| 21-225_35F11_HC | . | . | . | . | . | . | . | . | . | . | . | . | . | . | . | . | . | . | . | . | . | M | . | . | . | . |
| 21-225_36A5_HC | . | . | . | . | . | . | . | R | . | . | . | . | . | . | N | . | . | Y | . | . | . | . | . | . | . | . |
| 21-225_36C5_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | S | A | I | S | G | R | - | - | - | G | G | S | T | F | H | A | D | S | V | K | G | R | F |
| 21-225_4G12_HC | . | . | . | . | T | M | . | . | . | . | . | . | . | . | . | . | Y | Y | . | . | . | . | . | . | . | S |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_12D8_HC | . | . | . | . | . | . | . | . | T | . | . | . | . | . | . | . | . | . | . | . | . | . | . | . | . | . |
| 21-225_17F12_HC | . | . | . | . | . | . | . | . | . | . | . | . | . | . | . | . | . | . | . | . | . | . | . | . | . | . |
| 21-225_21H4_HC | . | . | . | . | . | . | . | . | . | . | . | . | . | . | . | . | . | . | . | . | . | . | . | . | . | . |
| 21-225_24B1_HC | . | . | . | . | . | . | . | . | . | . | . | . | . | . | . | . | . | . | . | . | . | . | . | . | . | . |
| 21-225_30D1_HC | . | . | . | . | . | . | . | . | . | . | . | . | . | . | . | . | . | . | . | . | . | . | . | . | . | . |
| 21-225_30E3_HC | . | . | . | . | . | . | . | . | . | . | . | . | . | . | . | . | . | . | . | . | . | . | . | . | L | . |
| 21-225_30E9_HC | . | . | . | . | . | . | . | . | . | . | . | . | . | . | . | . | . | . | . | . | . | . | . | . | . | . |
| 21-225_30G11_HC | . | . | . | . | . | . | . | . | T | . | . | . | . | . | . | . | . | . | . | . | . | . | . | . | . | . |
| 21-225_31A8_HC | . | . | . | . | . | . | . | . | . | . | . | . | . | . | . | . | . | . | . | D | . | . | . | . | . | . |
| 21-225_31B8_HC | . | . | . | . | . | . | . | . | . | . | . | . | . | . | L | . | . | . | . | . | D | . | . | . | . | . |
| 21-225_31H3_HC | . | . | . | . | . | . | . | . | . | . | . | . | . | . | . | . | . | . | . | . | . | . | . | . | . | . |
| 21-225_31H5_HC | . | . | . | . | . | . | . | . | . | . | . | . | . | . | . | . | . | . | . | . | . | . | . | . | . | . |
| 21-225_32G1_HC | . | . | . | . | . | . | . | . | . | . | . | . | . | . | . | . | . | . | . | . | . | . | . | . | . | . |
| 21-225_32G12_HC | . | . | . | . | . | . | . | [illegible] | . | . | . | . | . | . | . | . | . | . | . | . | . | . | . | . | . | . |
| 21-225_33A4_HC | . | . | . | . | . | . | . | . | . | . | . | . | . | . | . | . | . | . | . | . | . | . | . | . | . | . |
| 21-225_33A7_HC | . | . | . | . | . | . | . | . | . | . | . | . | . | . | . | . | . | . | . | . | . | . | . | . | . | . |
| 21-225_33B8_HC | . | . | . | . | . | . | . | . | . | . | . | . | . | . | . | . | . | . | . | . | . | . | . | . | . | . |
| 21-225_33F1_HC | . | . | . | . | . | . | . | . | . | . | . | . | . | . | . | . | . | . | . | . | . | . | . | . | . | . |
| 21-225_33G7_HC | . | . | . | . | . | . | . | . | . | . | . | . | . | . | . | . | . | . | . | . | . | . | . | . | . | . |
| 21-225_34A6_HC | . | . | . | . | . | . | . | . | . | . | . | . | . | . | . | . | . | . | . | . | . | . | . | . | . | . |
| 21-225_34G8_HC | . | . | . | . | . | . | . | . | . | . | . | . | . | . | I | . | . | . | . | . | . | . | . | . | . | . |
| 21-225_34G9_HC | . | . | . | . | . | . | . | . | . | . | . | . | . | . | . | . | . | . | . | . | . | . | . | . | . | . |
| 21-225_34H11_HC | . | . | . | . | . | . | . | . | . | . | . | F | . | . | . | . | . | . | . | . | . | . | . | . | . | . |
| 21-225_35A6_HC | . | . | . | . | . | . | . | . | . | . | . | . | . | . | . | . | . | . | . | . | . | . | . | . | . | . |
| 21-225_35B7_HC | . | . | . | . | . | . | . | . | . | . | . | . | . | . | . | . | . | . | . | . | . | . | . | . | . | . |
| 21-225_35D1_HC | . | . | . | . | . | . | . | N | . | . | . | . | . | . | . | . | . | . | . | . | . | . | . | . | . | . |
| 21-225_35F11_HC | . | . | . | . | . | . | . | . | . | . | . | . | . | . | . | . | . | . | . | . | . | . | . | . | . | . |
| 21-225_36A5_HC | . | . | . | . | . | . | . | . | . | . | . | . | . | . | . | . | . | . | . | . | . | . | . | . | . | . |
| 21-225_36C5_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_4G12_HC | . | . | . | . | . | . | . | . | . | . | . | . | . | . | . | S | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | V | K | G | E | L | L | E | D | Y | - | - | - | - | - | - | - | - | - | - | - | - | - | - | - |
| 21-225_12D8_HC | . | . | A | . | W | G | R | G | Y | S | . | E | . | . | . | . | . | . | . | . | . | . | . | . | . | . |
| 21-225_17F12_HC | . | . | A | . | W | G | R | G | Y | N | . | E | . | . | . | . | . | . | . | . | . | . | . | . | . | . |
| 21-225_21H4_HC | . | . | A | . | W | G | R | G | Y | S | . | E | . | . | . | . | . | . | . | . | . | . | . | . | . | . |
| 21-225_24B1_HC | . | . | A | . | W | G | R | G | Y | S | . | E | . | . | . | . | . | . | . | . | . | . | . | . | . | . |
| 21-225_30D1_HC | . | . | . | . | . | . | . | . | . | . | . | . | . | . | . | . | . | . | . | . | . | . | . | . | . | . |
| 21-225_30E3_HC | . | . | A | . | . | . | . | . | . | . | . | . | . | . | . | . | . | . | . | . | . | . | . | . | . | . |
| 21-225_30E9_HC | . | . | A | . | . | . | . | . | . | . | . | . | . | . | . | . | . | . | . | . | . | . | . | . | . | . |
| 21-225_30G11_HC | . | . | . | . | . | . | . | . | . | . | . | . | . | . | . | . | . | . | . | . | . | . | . | . | . | . |
| 21-225_31A8_HC | . | . | . | . | . | . | . | . | . | . | . | . | . | . | . | . | . | . | . | . | . | . | . | . | . | . |
| 21-225_31B8_HC | . | . | . | . | . | . | . | . | . | . | . | . | . | . | . | . | . | . | . | . | . | . | . | . | . | . |
| 21-225_31H3_HC | . | . | A | . | . | . | . | . | . | . | . | . | . | . | . | . | . | . | . | . | . | . | . | . | . | . |
| 21-225_31H5_HC | . | . | . | . | . | . | . | . | . | . | . | . | . | . | . | . | . | . | . | . | . | . | . | . | . | . |
| 21-225_32G1_HC | . | . | . | . | . | . | . | . | . | . | . | . | . | . | . | . | . | . | . | . | . | . | . | . | . | . |
| 21-225_32G12_HC | . | . | . | . | . | . | . | . | . | . | . | . | . | . | . | . | . | . | . | . | . | . | . | . | . | . |
| 21-225_33A4_HC | F | . | A | . | . | . | . | . | . | . | . | . | . | . | . | . | . | . | . | . | . | . | . | . | . | . |
| 21-225_33A7_HC | . | . | . | . | . | . | . | . | . | . | . | . | . | . | . | . | . | . | . | . | . | . | . | . | . | . |
| 21-225_33B8_HC | . | . | . | . | . | . | . | . | . | . | . | . | . | . | . | . | . | . | . | . | . | . | . | . | . | . |
| 21-225_33F1_HC | . | . | . | . | . | . | . | . | . | . | . | . | . | . | . | . | . | . | . | . | . | . | . | . | . | . |
| 21-225_33G7_HC | . | . | A | . | . | . | . | . | . | . | . | . | . | . | . | . | . | . | . | . | . | . | . | . | . | . |
| 21-225_34A6_HC | . | . | A | . | . | . | . | . | . | . | . | . | . | . | . | . | . | . | . | . | . | . | . | . | . | . |
| 21-225_34G8_HC | . | . | . | . | . | . | . | . | . | . | . | . | . | . | . | . | . | . | . | . | . | . | . | . | . | . |
| 21-225_34G9_HC | . | . | . | . | . | . | . | . | . | . | . | . | . | . | . | . | . | . | . | . | . | . | . | . | . | . |
| 21-225_34H11_HC | . | . | A | . | . | . | . | . | . | . | . | . | . | . | . | . | . | . | . | . | . | . | . | . | . | . |
| 21-225_35A6_HC | . | . | . | . | . | . | . | . | . | . | . | . | . | . | . | . | . | . | . | . | . | . | . | . | . | . |
| 21-225_35B7_HC | . | . | . | . | . | . | . | . | . | . | . | . | . | . | . | . | . | . | . | . | . | . | . | . | . | . |
| 21-225_35D1_HC | . | . | . | . | . | . | . | . | . | . | . | . | . | . | . | . | . | . | . | . | . | . | . | . | . | . |
| 21-225_35F11_HC | . | . | . | . | . | . | . | . | . | . | . | . | . | . | . | . | . | . | . | . | . | . | . | . | . | . |
| 21-225_36A5_HC | . | . | A | . | . | . | . | . | . | . | . | . | . | . | . | . | . | . | . | . | . | . | . | . | . | . |
| 21-225_36C5_HC | . | . | . | . | . | . | . | . | . | . | . | . | . | . | . | . | . | . | . | . | . | . | . | . | . | . |

# FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | V | K | G | E | L | L | E | D | Y | - | - | - | - | - | - | - | - | - | - | - | - | - | - | - |
| 21-225_4G12_HC | . | . | A | . | W | G | R | G | Y | S | . | E | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | Y | F | Y | G | M | D | V | W | G | Q | G | T | T | V | T | V | S | S |
| 21-225_12D8_HC | . | . | Y | . | . | . | . | . | . | . | . | . | . | . | . | . | . | . | . |
| 21-225_17F12_HC | . | . | Y | . | . | . | . | . | . | . | . | . | . | . | . | . | . | . | . |
| 21-225_21H4_HC | . | . | Y | . | . | . | . | . | . | . | . | . | . | . | . | . | . | . | . |
| 21-225_24B1_HC | . | . | Y | . | . | . | . | . | . | . | . | . | . | . | . | . | . | . | . |
| 21-225_30D1_HC | . | . | . | . | . | L | . | . | . | . | . | . | . | . | . | . | . | . | . |
| 21-225_30E3_HC | . | . | Y | . | . | . | . | . | . | . | . | . | . | . | . | . | . | . | . |
| 21-225_30E9_HC | . | . | Y | . | . | . | . | . | . | . | . | . | . | . | . | . | . | . | . |
| 21-225_30G11_HC | . | . | . | . | . | . | . | . | . | . | . | . | . | . | . | . | . | . | . |
| 21-225_31A8_HC | . | . | . | . | . | . | . | . | . | . | . | . | . | . | . | . | . | . | . |
| 21-225_31B8_HC | . | . | . | . | . | . | . | . | . | . | . | . | . | . | . | . | . | . | . |
| 21-225_31H3_HC | . | . | Y | . | . | . | . | . | . | . | . | . | . | . | . | . | . | . | . |
| 21-225_31H5_HC | . | . | . | . | . | . | . | . | . | . | . | . | . | . | . | . | . | . | . |
| 21-225_32G1_HC | . | . | . | . | . | . | . | . | . | . | . | . | . | . | . | . | . | . | . |
| 21-225_32G12_HC | . | . | . | . | . | . | . | . | . | . | . | . | . | . | . | . | . | . | . |
| 21-225_33A4_HC | . | S | Y | . | . | I | . | . | . | . | . | . | . | . | . | . | . | . | . |
| 21-225_33A7_HC | . | . | . | . | . | . | . | . | . | . | . | . | . | . | . | . | . | . | . |
| 21-225_33B8_HC | . | . | . | . | . | . | . | . | . | . | . | . | . | . | . | . | . | . | . |
| 21-225_33F1_HC | . | . | . | . | . | . | . | . | . | . | . | . | . | . | . | . | . | . | . |
| 21-225_33G7_HC | . | S | Y | . | . | . | . | . | . | . | . | . | . | . | . | . | . | . | . |
| 21-225_34A6_HC | . | . | Y | F | A | . | . | . | . | . | . | . | . | . | . | . | . | . | . |
| 21-225_34G8_HC | . | . | . | . | . | . | . | . | . | . | . | . | . | . | . | . | . | . | . |
| 21-225_34G9_HC | . | . | . | . | . | . | . | . | . | . | . | . | . | . | . | . | . | . | . |
| 21-225_34H11_HC | . | . | Y | . | . | . | . | . | . | . | . | . | . | . | . | . | . | . | . |
| 21-225_35A6_HC | . | . | . | . | . | . | . | . | . | . | . | . | . | . | . | . | . | . | . |
| 21-225_35B7_HC | . | . | . | . | . | . | . | . | . | . | . | . | A | . | . | . | . | . | . |
| 21-225_35D1_HC | . | . | . | . | . | . | . | . | . | . | . | . | A | . | . | . | . | . | . |
| 21-225_35F11_HC | . | . | . | . | . | . | . | . | . | . | . | . | . | . | . | . | . | . | . |
| 21-225_36A5_HC | . | . | Y | . | A | . | . | . | . | . | . | . | . | . | . | . | . | . | . |
| 21-225_36C5_HC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | Y | F | Y | G | M | D | V | W | G | Q | G | T | T | V | T | V | S | S |
| 21-225_4G12_HC | . | . | Y | . | . | . | . | . | . | . | . | . | . | . | . | . | . | . | . |

**FIGURE 57**

Table 61. Consensus 27- VH3|3-33/D4|4-23|RF2/JH6 (SEQ ID NO: 50278):

QVQLVES-GGGVVQPGRSLRLSCAASG-FTFSS-----YGMHWVRQAPGKGLEWVAVIWYD---GSNKYYADSVKGRFTISRDNSKNTLYLQMNSLRAEDTAVYYCARDRVYCSSTSC----------PYYYYYGMDVWGQGTTVTVSS wherein:

S in position 33 can be substituted with G, D, R, H, T or N

G in position 40 can be substituted with V

M in position 41 can be substituted with L

H in position 42 can be substituted with N

V in position 57 can be substituted with L, F, I or D

I in position 58 can be substituted with F

W in position 59 can be substituted with R

Y in position 60 can be substituted with F

S in position 66 can be substituted with N or T

N in position 67 can be substituted with E, D or K

K in position 68 can be substituted with N or T

Y in position 69 can be substituted with S, D or N

Y in position 70 can be substituted with N

A in position 71 can be substituted with V

G in position 76 can be substituted with D

R in position 110 can be substituted with D, W or N

V in position 111 can be substituted with D, R, F or H

**FIGURE 57**

Y in position 112 can be substituted with S, E, G or F

C in position 113 can be substituted with G or E

S in position 114 can be substituted with G

S in position 115 can be substituted with G, R or N

T in position 116 can be substituted with null (-), S or P

S in position 117 can be substituted with P, null (-) or T

C in position 118 can be substituted with null (-)

null (-) in position 119 can be substituted with S, H, L or Y

P in position 129 can be substituted with null (-), S or Y

Y in position 130 can be substituted with null (-)

Y in position 131 can be substituted with null (-)

Y in position 132 can be substituted with null (-)

Y in position 134 can be substituted with F

G in position 135 can be substituted with A

M in position 136 can be substituted with L

Xaa Tyr Xaa Xaa Xaa (SEQ ID NO: 50037)

Xaa Xaa Xaa Xaa Asp Gly Xaa Xaa Xaa Xaa Xaa Xaa Asp Ser Val Lys Xaa (SEQ ID NO: 50038)

Asp Xaa Xaa Xaa Xaa Xaa Xaa Xaa Xaa Xaa Xaa Xaa Xaa Xaa Xaa Xaa Tyr Xaa Xaa Asp Val (SEQ ID NO: 50039)

Ser Tyr Gly Met His (SEQ ID NO: 50504)

**FIGURE 57**

Val Ile Trp Tyr Asp Gly Ser Asn Lys Tyr Tyr Ala Asp Ser Val Lys Gly (SEQ ID NO: 50505)

Asp Arg Val Tyr Cys Ser Ser Thr Ser Cys Pro Tyr Tyr Tyr Tyr Tyr Gly Met Asp Val (SEQ ID NO: 50506)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | E | S | - | G | G | G | V | V | Q | P | G | R | S | L | R | L | S | C | A | A | S |
| 21-225_146A10_HC | . | . | . | . | E | . | . | . | . | . | . | . | . | . | . | . | . | . | . | . | . | . | . | . | . | . |
| 21-225_147D9_HC | . | . | . | . | . | . | . | . | . | . | . | . | . | . | . | . | . | . | . | . | . | . | . | . | . | . |
| 21-225_148E10_HC | . | . | . | . | . | . | . | . | . | . | . | . | . | . | . | . | . | . | . | . | . | . | . | . | . | . |
| 21-225_149F2_HC | . | . | . | . | G | . | A | . | . | . | . | . | . | . | . | . | . | . | . | . | . | . | . | . | . | . |
| 21-225_150F11_HC | . | . | . | . | G | . | A | . | . | . | . | . | . | . | . | . | . | . | . | . | . | . | . | . | . | . |
| 21-225_151H6_HC | . | . | . | . | . | . | . | . | . | . | . | . | . | . | . | . | . | . | . | . | . | . | . | . | . | . |
| 21-225_152B12_HC | . | . | . | V | . | . | . | . | . | . | . | . | . | . | . | . | . | . | . | . | . | . | . | . | . | . |
| 21-225_152G5_HC | . | . | . | . | . | . | . | . | . | . | . | . | . | . | . | . | . | . | . | . | . | . | . | . | . | . |
| 21-225_153A8_HC | . | . | . | . | M | . | . | . | . | . | . | . | . | . | . | . | . | . | . | . | . | . | . | . | . | . |
| 21-225_153E8_HC | . | . | . | . | . | . | . | . | . | . | . | . | . | . | . | . | . | . | . | . | . | . | . | . | . | . |
| 21-225_158D10_HC | . | . | . | . | . | . | . | . | . | . | . | . | . | . | . | . | . | . | . | . | . | . | . | . | . | . |
| 21-225_159H8_HC | . | . | . | . | . | . | . | . | . | . | . | . | . | . | . | . | . | . | . | . | . | . | . | . | . | . |
| 21-225_160B12_HC | . | . | . | . | . | . | . | . | . | . | . | . | . | . | . | . | . | . | . | . | . | . | . | . | . | . |
| 21-225_166G11_HC | . | . | . | . | . | . | . | . | . | . | . | . | . | . | . | . | . | . | . | . | . | . | . | . | . | . |
| 21-225_170F1_HC | . | . | . | . | . | . | . | . | . | . | . | . | . | . | . | . | . | . | . | . | . | . | . | . | . | . |
| 21-225_195D9_HC | . | . | . | . | . | . | . | . | . | . | . | . | . | . | . | . | . | . | . | . | . | . | . | . | . | . |
| 21-225_196A10_HC | . | . | . | . | . | . | . | . | . | . | . | . | . | . | . | . | . | . | . | . | . | . | . | . | . | . |
| 21-225_225A9_HC | . | . | . | . | . | . | . | . | . | . | . | . | . | . | . | . | G | . | . | . | . | . | . | . | . | . |
| 21-225_27G6_HC | . | . | . | . | . | . | . | . | . | . | . | . | . | . | . | . | . | . | . | . | . | . | . | . | . | . |
| 21-225_27H2_HC | . | . | . | . | . | . | . | . | . | . | . | . | . | . | . | . | . | . | . | . | . | . | . | . | . | . |
| 21-225_28A11_HC | . | . | . | . | . | . | . | . | . | . | . | . | . | . | . | . | . | . | . | . | . | . | . | . | . | . |
| 21-225_52H1_HC | . | . | . | . | . | . | . | . | . | . | . | . | . | . | . | . | . | . | . | . | . | . | . | . | . | . |
| 21-225_74B3_HC | . | . | . | . | . | . | . | . | . | . | . | . | . | . | . | . | . | . | . | . | . | . | . | . | . | . |
| 21-225_74C9_HC | . | . | . | . | . | . | . | . | . | . | . | . | . | . | . | . | . | . | . | . | . | . | . | . | . | . |
| 21-225_78E9_HC | . | . | . | . | . | . | . | . | . | . | . | . | . | . | . | . | . | . | . | . | . | . | . | . | . | . |
| 21-225_79E7_HC | . | . | . | . | . | . | . | . | . | . | . | . | . | . | . | . | . | . | . | . | . | . | . | . | . | . |
| 21-225_8C12_HC | . | . | . | . | . | . | . | . | . | . | . | . | . | . | . | . | . | . | . | . | . | . | . | . | . | . |
| 21-225_92A4_HC | . | . | . | . | . | . | . | . | . | . | . | . | . | . | . | . | . | . | . | . | . | . | . | . | . | . |
| 21-225_97E6_HC | . | . | . | . | . | . | . | . | . | . | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | G | M | H | W | V | R | Q | A | P | G | K | G | L |
| 21-225_146A10_HC | . | . | . | . | . | . | G | . | . | . | . | . | . | . | . | . | . | . | . | . | . | . | . | . | . | . |
| 21-225_147D9_HC | . | . | . | . | . | . | . | . | . | . | . | . | . | . | . | . | . | . | . | . | . | . | . | . | . | . |
| 21-225_148E10_HC | . | . | . | . | . | . | . | . | . | . | . | . | . | . | . | . | . | . | . | . | . | . | . | . | . | . |
| 21-225_149F2_HC | . | . | . | . | . | . | R | . | . | . | . | . | . | . | . | . | . | . | . | . | . | . | . | . | . | . |
| 21-225_150F11_HC | . | . | . | . | . | . | R | . | . | . | . | . | . | . | . | . | . | . | . | . | . | . | . | . | . | . |
| 21-225_151H6_HC | . | . | . | . | . | . | D | . | . | . | . | . | . | . | . | . | . | . | . | . | . | . | . | . | . | . |
| 21-225_152B12_HC | . | . | . | . | . | . | D | . | . | . | . | . | . | . | . | . | . | . | . | . | . | . | . | . | . | . |
| 21-225_152G5_HC | . | . | . | . | . | . | D | . | . | . | . | . | . | . | . | . | . | . | . | . | . | . | . | . | . | . |
| 21-225_153A8_HC | . | . | . | . | . | . | D | . | . | . | . | . | . | . | . | . | . | . | . | . | . | . | . | . | . | . |
| 21-225_153E8_HC | . | . | . | . | L | . | N | . | . | . | . | . | . | . | . | . | . | . | . | . | . | . | . | . | . | . |
| 21-225_158D10_HC | . | . | . | . | . | . | . | . | . | . | . | . | . | . | . | . | . | . | . | . | . | . | . | . | . | . |
| 21-225_159H8_HC | . | . | . | . | . | . | T | . | . | . | . | . | . | . | . | . | . | . | . | . | . | . | . | . | . | . |
| 21-225_160B12_HC | . | . | . | . | . | . | . | . | . | . | . | . | . | . | . | . | . | . | . | . | . | . | . | . | . | . |
| 21-225_166G11_HC | . | . | . | . | . | N | G | . | . | . | . | . | . | . | . | . | . | . | . | . | . | . | . | . | . | . |
| 21-225_170F1_HC | . | . | . | . | . | . | G | . | . | . | . | . | . | . | . | N | . | . | . | . | . | . | . | . | . | . |
| 21-225_195D9_HC | . | . | . | . | . | . | H | . | . | . | . | . | . | V | . | . | . | . | . | . | . | . | . | . | . | . |
| 21-225_196A10_HC | . | . | . | . | . | . | H | . | . | . | . | . | . | V | . | . | . | . | . | . | . | . | . | . | . | . |
| 21-225_225A9_HC | . | . | . | . | . | . | T | . | . | . | . | . | . | . | . | . | . | . | . | L | . | . | . | . | . | . |
| 21-225_27G6_HC | . | . | . | . | . | . | G | . | . | . | . | . | . | . | . | . | . | . | . | . | . | . | . | . | . | . |
| 21-225_27H2_HC | . | . | . | . | . | . | G | . | . | . | . | . | . | . | . | . | . | . | . | . | . | . | . | . | . | . |
| 21-225_28A11_HC | . | . | . | . | . | . | G | . | . | . | . | . | . | . | L | . | . | . | . | . | . | . | . | . | . | . |
| 21-225_52H1_HC | . | . | . | . | . | . | G | . | . | . | . | . | . | . | . | . | . | . | . | . | . | . | . | . | . | . |
| 21-225_74B3_HC | . | . | . | . | . | . | R | . | . | . | . | . | . | . | . | . | . | . | . | . | . | . | . | . | . | . |
| 21-225_74C9_HC | . | . | . | . | . | . | . | . | . | . | . | . | . | . | . | . | . | . | . | . | . | . | . | . | . | . |
| 21-225_78E9_HC | . | . | . | . | . | . | . | . | . | . | . | . | . | . | . | . | . | . | . | . | . | . | . | . | . | . |
| 21-225_79E7_HC | . | . | . | . | . | . | . | . | . | . | . | . | . | . | . | . | . | . | . | . | . | . | . | . | . | . |
| 21-225_8C12_HC | . | . | . | . | . | G | . | . | . | . | . | . | . | . | . | . | . | . | . | . | . | . | . | . | . | . |
| 21-225_92A4_HC | . | . | . | . | . | . | . | . | . | . | . | . | . | . | . | . | . | . | . | . | . | . | . | . | . | . |
| 21-225_97E6_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | A | V | I | W | Y | D | - | - | - | G | S | N | K | Y | Y | A | D | S | V | K | G | R | F |
| 21-225_146A10_HC | . | . | M | T | L | . | R | . | . | . | . | . | . | . | D | . | N | . | . | . | . | . | . | . | . | . |
| 21-225_147D9_HC | . | . | . | . | D | . | . | F | . | . | . | . | . | . | . | . | . | . | V | . | . | . | . | D | . | . |
| 21-225_148E10_HC | . | . | . | . | . | . | . | F | . | . | . | . | . | . | . | . | . | . | V | . | . | . | . | D | . | . |
| 21-225_149F2_HC | . | . | . | . | F | . | . | . | . | . | . | . | . | . | E | N | . | . | . | . | . | . | . | . | . | . |
| 21-225_150F11_HC | . | . | . | . | F | . | . | . | . | . | . | . | . | . | E | N | . | . | . | . | . | . | . | . | . | . |
| 21-225_151H6_HC | . | . | . | . | L | . | . | . | . | . | . | . | . | . | . | . | . | . | . | . | . | . | . | . | . | . |
| 21-225_152B12_HC | . | . | . | . | . | . | . | . | . | . | . | . | . | . | . | . | . | . | . | . | . | . | . | . | . | . |
| 21-225_152G5_HC | . | . | . | . | L | . | . | . | . | . | . | . | . | . | . | . | . | . | . | . | . | . | . | . | . | . |
| 21-225_153A8_HC | . | . | . | . | L | . | . | . | . | . | . | . | . | . | . | . | . | . | . | . | . | . | . | . | . | . |
| 21-225_153E8_HC | . | . | . | . | . | . | . | F | . | . | . | . | . | . | . | . | . | . | V | . | . | . | . | D | . | . |
| 21-225_158D10_HC | . | . | . | . | . | . | . | . | . | . | . | . | . | . | . | . | . | . | . | . | . | . | . | . | . | . |
| 21-225_159H8_HC | . | . | . | . | . | . | . | . | . | . | . | . | . | . | . | . | . | . | . | . | . | . | . | . | . | . |
| 21-225_160B12_HC | . | . | . | . | . | . | . | . | . | . | . | . | . | . | . | . | . | . | . | . | . | . | . | . | . | . |
| 21-225_166G11_HC | . | . | . | . | . | . | . | . | . | . | . | . | . | . | . | . | . | . | . | . | . | . | . | . | . | . |
| 21-225_170F1_HC | . | . | . | . | I | . | . | . | . | . | . | . | . | N | . | . | . | . | . | . | . | . | . | . | . | . |
| 21-225_195D9_HC | . | . | . | . | . | . | . | . | . | . | . | . | . | T | . | . | . | . | . | . | . | . | . | . | . | . |
| 21-225_196A10_HC | . | . | . | . | . | . | . | . | . | . | . | . | . | T | . | . | . | . | . | . | . | . | . | . | . | . |
| 21-225_225A9_HC | . | . | . | . | . | . | . | . | . | . | . | . | . | . | . | . | . | . | . | . | . | . | . | . | . | . |
| 21-225_27G6_HC | . | . | . | . | I | . | . | . | . | . | . | . | . | . | K | . | . | N | . | . | . | . | . | . | . | . |
| 21-225_27H2_HC | . | . | . | . | . | . | . | . | . | . | . | . | . | . | . | . | . | . | . | . | . | . | . | . | . | . |
| 21-225_28A11_HC | . | . | . | . | L | . | . | . | . | . | . | . | . | N | . | T | . | . | . | . | . | . | . | . | . | . |
| 21-225_52H1_HC | . | . | . | . | . | . | . | . | . | . | . | . | . | . | . | . | . | . | . | . | . | . | . | . | . | . |
| 21-225_74B3_HC | . | . | . | . | . | F | . | . | . | . | . | . | . | . | . | . | D | . | . | . | . | . | . | . | . | . |
| 21-225_74C9_HC | . | . | . | . | . | . | . | . | . | . | . | . | . | N | . | . | S | . | . | . | . | . | . | . | . | . |
| 21-225_78E9_HC | . | . | . | . | . | . | . | . | . | . | . | . | . | N | . | . | S | . | . | . | . | . | . | . | . | . |
| 21-225_79E7_HC | . | . | . | . | . | . | . | . | . | . | . | . | . | N | . | . | S | . | . | . | . | . | . | . | . | . |
| 21-225_8C12_HC | . | . | . | . | L | . | . | . | . | . | . | . | . | . | . | . | . | . | . | . | . | . | . | . | . | . |
| 21-225_92A4_HC | . | . | . | . | . | . | . | . | . | . | . | . | . | N | . | . | S | . | . | . | . | . | . | . | . | . |
| 21-225_97E6_HC | . | . | . | . | . | . | . | . | . | . | . | . | . | N | . | . | S | . | . | . | . | . | . | . | . | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_146A10_HC | . | . | . | . | . | . | . | . | . | . | . | . | . | . | . | . | . | . | . | . | . | . | . | . | . | . |
| 21-225_147D9_HC | . | . | . | . | . | . | . | . | . | . | . | . | . | . | . | . | . | . | . | . | . | . | . | . | . | . |
| 21-225_148E10_HC | . | . | . | . | . | . | . | . | . | . | . | . | . | . | . | . | . | . | . | . | . | . | . | . | . | . |
| 21-225_149F2_HC | . | . | . | . | V | . | . | R | . | . | . | . | . | . | . | . | . | . | . | . | . | . | . | . | . | . |
| 21-225_150F11_HC | . | . | . | . | V | . | . | R | . | . | . | . | . | . | . | . | . | . | . | . | . | . | . | . | . | . |
| 21-225_151H6_HC | . | . | . | . | . | . | . | . | . | . | . | . | . | . | . | . | . | . | . | . | . | . | . | . | . | . |
| 21-225_152B12_HC | . | . | . | . | . | . | . | . | . | . | . | . | . | . | . | . | . | . | . | . | . | . | . | . | . | . |
| 21-225_152G5_HC | . | . | . | . | . | . | . | . | . | . | . | . | . | . | . | . | . | . | . | . | . | . | . | . | . | . |
| 21-225_153A8_HC | . | . | . | . | . | . | . | . | . | . | . | . | . | . | . | . | . | . | . | . | . | . | . | . | . | . |
| 21-225_153E8_HC | . | . | . | . | . | . | . | . | . | . | . | . | . | . | . | . | . | . | . | . | . | . | . | . | . | . |
| 21-225_158D10_HC | . | . | . | . | . | . | . | . | . | . | . | . | . | . | . | . | . | . | . | . | . | . | . | . | . | . |
| 21-225_159H8_HC | . | . | . | . | . | . | . | . | . | . | . | . | . | . | . | . | . | . | . | . | . | . | . | . | . | . |
| 21-225_160B12_HC | . | . | . | . | . | . | . | . | . | . | . | . | . | . | . | . | . | . | . | . | . | . | . | . | . | . |
| 21-225_166G11_HC | . | . | . | . | . | T | . | . | . | . | . | F | . | . | . | . | . | . | T | . | . | . | . | . | . | . |
| 21-225_170F1_HC | . | . | . | . | . | . | . | . | . | . | . | . | . | . | . | . | . | . | . | . | . | . | . | . | . | . |
| 21-225_195D9_HC | . | . | . | . | . | . | . | . | . | . | . | . | . | . | . | . | . | . | . | . | . | . | . | . | . | . |
| 21-225_196A10_HC | . | . | . | . | . | . | . | . | . | . | . | . | . | . | . | . | . | . | . | . | . | . | . | . | . | . |
| 21-225_225A9_HC | . | . | . | . | . | I | . | Q | . | . | . | . | . | . | . | . | . | . | . | . | . | . | . | . | . | . |
| 21-225_27G6_HC | . | . | . | . | . | . | . | . | . | . | . | . | . | . | . | . | . | . | . | . | . | . | . | . | . | . |
| 21-225_27H2_HC | . | . | . | . | . | . | . | . | . | . | . | F | . | . | . | . | . | . | . | . | . | . | . | . | . | . |
| 21-225_28A11_HC | . | . | . | . | . | . | . | . | . | . | . | . | . | . | . | . | . | . | . | . | . | . | . | . | . | . |
| 21-225_52H1_HC | . | . | . | . | . | . | . | . | . | . | . | . | . | . | . | . | . | . | . | . | . | . | . | . | . | . |
| 21-225_74B3_HC | . | . | . | . | . | . | . | . | . | . | . | . | . | . | . | . | . | . | . | . | . | . | . | . | . | . |
| 21-225_74C9_HC | . | . | . | . | . | I | . | . | . | . | . | . | . | . | . | . | . | . | . | . | . | . | . | . | . | . |
| 21-225_78E9_HC | . | . | . | . | . | I | . | . | . | . | . | . | . | . | . | . | . | . | . | . | . | . | . | . | . | . |
| 21-225_79E7_HC | . | . | . | . | . | I | . | . | . | . | . | . | . | . | . | . | . | . | . | . | . | . | . | . | . | . |
| 21-225_8C12_HC | . | . | . | . | . | . | . | . | . | . | . | . | . | . | . | . | . | . | . | . | . | . | . | . | . | . |
| 21-225_92A4_HC | . | . | Y | . | . | I | . | . | . | . | . | . | . | . | . | . | . | . | . | . | . | . | . | . | . | . |
| 21-225_97E6_HC | . | . | . | . | . | I | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | D | R | V | Y | C | S | S | T | S | C | - | - | - | - | - | - | - | - | - | - | P | Y |
| 21-225_146A10_HC | . | . | . | . | . | . | . | F | . | . | . | . | . | . | L | . | . | . | . | . | . | . | . | . | S | . |
| 21-225_147D9_HC | . | . | . | . | . | . | . | E | G | . | G | . | P | - | . | . | . | . | . | . | . | . | . | . | - | - |
| 21-225_148E10_HC | . | . | . | . | . | . | . | E | G | . | G | . | P | - | . | . | . | . | . | . | . | . | . | . | - | - |
| 21-225_149F2_HC | . | . | . | . | . | . | . | E | G | . | G | . | P | - | . | . | . | . | . | . | . | . | . | . | - | - |
| 21-225_150F11_HC | . | . | . | . | . | . | . | E | G | . | G | . | P | - | . | . | . | . | . | . | . | . | . | . | - | - |
| 21-225_151H6_HC | . | . | . | . | . | D | R | S | . | . | R | . | . | . | . | . | . | . | . | . | . | . | . | . | . | . |
| 21-225_152B12_HC | . | . | . | . | . | D | R | S | . | . | . | . | . | . | . | . | . | . | . | . | . | . | . | . | . | . |
| 21-225_152G5_HC | . | . | . | . | . | D | R | S | . | . | R | . | . | . | . | . | . | . | . | . | . | . | . | . | . | . |
| 21-225_153A8_HC | . | . | . | . | . | D | R | S | . | . | R | . | . | . | . | . | . | . | . | . | . | . | . | . | . | . |
| 21-225_153E8_HC | . | . | . | . | . | . | . | E | G | . | G | . | P | - | . | . | . | . | . | . | . | . | . | . | - | - |
| 21-225_158D10_HC | . | . | . | . | . | . | . | S | . | . | . | . | . | . | . | . | . | . | . | . | . | . | . | . | . | . |
| 21-225_159H8_HC | . | . | . | . | . | . | . | S | . | . | . | . | . | . | . | . | . | . | . | . | . | . | . | . | . | . |
| 21-225_160B12_HC | . | . | . | . | . | . | . | S | . | . | . | . | . | . | . | . | . | . | . | . | . | . | . | . | . | . |
| 21-225_166G11_HC | . | . | . | . | . | D | R | . | . | . | . | P | T | . | H | . | . | . | . | . | . | . | . | . | . | . |
| 21-225_170F1_HC | . | . | . | . | . | . | . | . | . | . | . | . | . | . | H | . | . | . | . | . | . | . | . | . | . | . |
| 21-225_195D9_HC | . | . | . | . | . | W | F | G | E | G | N | - | - | - | . | . | . | . | . | . | . | . | . | . | - | - |
| 21-225_196A10_HC | . | . | . | . | . | W | F | G | E | G | N | - | - | - | . | . | . | . | . | . | . | . | . | . | - | - |
| 21-225_225A9_HC | . | . | . | . | . | N | H | . | . | . | . | . | . | . | S | . | . | . | . | . | . | . | . | . | . | . |
| 21-225_27G6_HC | . | . | . | . | . | . | . | . | . | . | . | . | . | . | S | . | . | . | . | . | . | . | . | . | . | . |
| 21-225_27H2_HC | . | . | . | . | . | . | . | . | . | . | . | . | . | . | S | . | . | . | . | . | . | . | . | . | . | . |
| 21-225_28A11_HC | . | . | . | . | . | . | . | . | . | . | . | . | . | . | S | . | . | . | . | . | . | . | . | . | . | . |
| 21-225_52H1_HC | . | . | . | . | . | . | . | . | . | . | . | S | . | . | S | . | . | . | . | . | . | . | . | . | Y | . |
| 21-225_74B3_HC | . | . | . | . | . | . | D | . | . | . | . | . | . | . | . | . | . | . | . | . | . | . | . | . | . | . |
| 21-225_74C9_HC | . | . | . | . | . | . | D | . | . | . | . | . | . | . | . | . | . | . | . | . | . | . | . | . | . | . |
| 21-225_78E9_HC | . | . | . | . | . | . | D | . | . | . | . | . | . | . | . | . | . | . | . | . | . | . | . | . | . | . |
| 21-225_79E7_HC | . | . | . | . | . | . | D | . | . | . | . | . | . | . | . | . | . | . | . | . | . | . | . | . | . | . |
| 21-225_8C12_HC | . | . | . | . | . | . | . | S | . | . | . | S | . | . | Y | . | . | . | . | . | . | . | . | . | . | . |
| 21-225_92A4_HC | . | . | . | . | . | . | D | . | . | . | . | . | . | . | . | . | . | . | . | . | . | . | . | . | . | . |
| 21-225_97E6_HC | . | . | . | . | . | . | D | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | Y | Y | Y | Y | G | M | D | V | W | G | Q | G | T | T | V | T | V | S | S |
| 21-225_146A10_HC | . | . | . | . | . | . | . | . | . | . | . | . | . | . | . | . | . | . | . |
| 21-225_147D9_HC | . | . | . | . | . | . | . | . | . | . | . | . | . | . | . | . | . | . | . |
| 21-225_148E10_HC | . | . | . | . | . | . | . | . | . | . | . | . | . | . | . | . | . | . | . |
| 21-225_149F2_HC | . | . | . | . | . | . | . | . | . | . | . | . | . | . | . | . | . | . | . |
| 21-225_150F11_HC | . | . | . | . | . | . | . | . | . | . | . | . | . | . | . | . | . | . | . |
| 21-225_151H6_HC | . | . | . | . | . | L | . | . | . | . | . | . | . | . | . | I | . | . | . |
| 21-225_152B12_HC | . | . | . | . | . | L | . | . | . | . | . | . | . | . | . | . | . | . | . |
| 21-225_152G5_HC | . | . | . | . | . | L | . | . | . | . | . | . | . | . | . | I | . | . | . |
| 21-225_153A8_HC | . | . | . | . | . | L | . | . | . | . | . | . | . | . | . | I | . | . | . |
| 21-225_153E8_HC | . | . | . | . | . | . | . | . | . | . | . | . | . | . | . | . | . | . | . |
| 21-225_158D10_HC | . | . | . | . | . | . | . | . | . | . | . | . | . | . | . | . | . | . | . |
| 21-225_159H8_HC | . | . | . | . | . | . | . | . | . | . | . | . | . | . | . | . | . | . | . |
| 21-225_160B12_HC | . | . | . | . | . | . | . | . | . | . | . | . | . | . | . | . | . | . | . |
| 21-225_166G11_HC | . | . | . | . | . | L | . | . | . | . | . | . | . | . | . | . | . | . | . |
| 21-225_170F1_HC | . | . | . | . | A | . | . | . | . | . | . | . | . | . | . | . | . | . | . |
| 21-225_195D9_HC | - | - | . | . | . | . | . | . | . | . | . | . | . | . | . | . | . | . | . |
| 21-225_196A10_HC | - | - | . | . | . | . | . | . | . | . | . | . | . | . | . | . | . | . | . |
| 21-225_225A9_HC | . | . | . | F | . | . | . | . | . | . | . | . | . | . | . | . | . | . | . |
| 21-225_27G6_HC | . | . | . | . | . | . | . | . | . | . | . | . | . | . | . | . | . | . | . |
| 21-225_27H2_HC | . | . | . | . | . | . | . | . | . | . | . | . | . | . | . | . | . | . | . |
| 21-225_28A11_HC | . | . | . | . | . | . | . | . | . | . | . | . | . | . | . | . | . | . | . |
| 21-225_52H1_HC | . | . | . | . | . | . | . | . | . | . | . | . | . | . | . | . | . | . | . |
| 21-225_74B3_HC | . | . | . | . | . | . | . | . | . | . | . | . | . | . | . | . | . | . | . |
| 21-225_74C9_HC | . | . | . | . | . | . | . | . | . | . | . | . | . | . | . | . | . | . | . |
| 21-225_78E9_HC | . | . | . | . | . | . | . | . | . | . | . | . | . | . | . | . | . | . | . |
| 21-225_79E7_HC | . | . | . | . | . | . | . | . | . | . | . | . | . | . | . | . | . | . | . |
| 21-225_8C12_HC | . | . | . | . | . | . | . | . | . | . | . | . | . | . | . | . | . | . | . |
| 21-225_92A4_HC | . | . | . | . | . | . | . | . | . | . | . | . | . | . | . | . | . | . | . |
| 21-225_97E6_HC | . | . | . | . | . | . | . | . | . | . | . | . | . | . | . | . | . | . | . |

## FIGURE 57

Table 62. Consensus 28- VH3|3-21/D1|1-1|RF2/JH4 (SEQ ID NO: 50279):

EVQLVES-GGGLVKPGGSLRLSCAASG-FTFSS-----YSMNWVRQAPGKGLEWVSSISGS---SSYIYYADSVKGRFTISRDNAKNSLYLQMNSLRAEDTAVYYCARVAS-------------------------FDYWGQGTLVTVSS wherein:

S at position 33 can be substituted with N

S at position 40 can be substituted with T, K, N or R

M at position 41 can be substituted with L

I at position 58 can be substituted with T

G at position 60 can be substituted with S

S at position 61 can be substituted with G, N or T

S at position 65 can be substituted with G, D or N

S at position 66 can be substituted with T

Y at position 67 can be substituted with F, D, L, N or S

I at position 68 can be substituted with M or T

Y at position 69 can be substituted with N

A at position 71 can be substituted with T

A at position 110 can be substituted with N or S

S at position 111 can be substituted with A, G, T, L, H or N

F at position 136 can be substituted with L or N

Y at position 138 can be substituted with C or S

Xaa Tyr Xaa Xaa Asn (SEQ ID NO: 50040)

**FIGURE 57**

Ser Xaa Ser Xaa Xaa Xaa Xaa Xaa Xaa Xaa Tyr Xaa Asp Ser Val Lys Gly (SEQ ID NO: 50041)

Val Xaa Xaa Xaa Asp Xaa (SEQ ID NO: 50042)

Ser Tyr Ser Met Asn (SEQ ID NO: 50507)

Ser Ile Ser Gly Ser Ser Ser Tyr Ile Tyr Tyr Ala Asp Ser Val Lys Gly (SEQ ID NO: 50508)

Val Ala Ser Phe Asp Tyr (SEQ ID NO: 50509)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | E | V | Q | L | V | E | S | - | G | G | G | L | V | K | P | G | G | S | L | R | L | S | C | A | A | S |
| 21-225_14D6_HC | . | . | . | . | . | . | . | . | . | . | . | . | . | . | . | . | . | . | . | . | . | . | . | . | . | . |
| 21-225_157E4_HC | . | . | . | . | . | . | . | . | . | . | . | . | . | . | . | . | . | . | . | . | . | . | . | . | . | . |
| 21-225_157H10_HC | . | . | . | . | . | . | . | . | . | . | . | . | . | . | . | . | . | . | . | . | . | . | . | . | . | . |
| 21-225_15A2_HC | . | . | . | . | . | . | . | . | . | . | . | . | . | . | . | . | . | . | . | . | . | . | . | . | . | . |
| 21-225_16A11_HC | . | . | . | . | . | . | . | . | . | . | . | . | . | . | . | . | . | . | . | . | . | . | . | . | . | . |
| 21-225_16B8_HC | . | . | . | . | . | . | . | . | . | . | . | . | . | . | . | . | . | . | . | . | . | . | . | . | . | . |
| 21-225_16H1_HC | . | . | . | . | . | . | . | . | . | . | . | . | . | . | . | . | . | . | . | . | . | . | . | . | . | . |
| 21-225_17H5_HC | . | . | . | . | . | . | . | . | . | . | . | . | . | . | . | . | . | . | . | . | . | . | . | . | . | . |
| 21-225_17H6_HC | . | . | . | . | . | . | . | . | . | . | . | . | . | . | . | . | . | . | . | . | . | . | . | . | . | . |
| 21-225_1F1_HC | . | . | . | . | . | . | . | . | . | . | . | . | . | . | . | . | . | . | . | . | . | . | . | . | . | . |
| 21-225_20H7_HC | . | . | H | . | . | . | . | . | . | . | . | . | . | . | . | . | . | . | . | . | . | . | . | . | . | . |
| 21-225_22B12_HC | . | . | . | . | . | . | . | . | . | . | . | . | . | . | . | . | . | . | . | . | . | . | . | . | . | . |
| 21-225_23A11_HC | . | . | . | . | . | . | . | . | . | . | . | . | . | . | . | . | . | . | . | . | . | . | . | . | . | . |
| 21-225_27C5_HC | . | . | . | . | . | . | . | . | . | . | . | . | . | . | . | . | . | . | . | . | . | . | . | . | . | . |
| 21-225_27H12_HC | . | . | . | . | . | . | . | . | . | . | . | . | . | . | . | . | . | . | . | . | . | . | . | . | . | . |
| 21-225_2A4_HC | . | . | . | . | . | . | . | . | . | . | . | . | . | . | . | . | . | . | . | . | . | . | . | . | . | . |
| 21-225_2C12_HC | . | . | . | . | . | . | . | . | . | . | . | . | . | . | . | . | . | . | . | . | . | . | . | . | . | . |
| 21-225_32B3_HC | . | . | . | . | . | . | . | . | . | . | . | . | . | . | . | . | . | . | . | . | . | . | . | . | . | . |
| 21-225_35H8_HC | . | . | . | . | . | . | . | . | . | . | . | . | . | . | . | . | . | . | . | . | . | . | . | . | . | . |
| 21-225_50G10_HC | . | . | . | . | . | . | . | . | . | . | . | . | . | . | . | . | . | . | . | . | . | . | . | . | . | . |
| 21-225_52E3_HC | . | . | . | . | . | . | . | . | . | . | . | . | . | . | . | . | . | . | . | . | . | . | . | . | . | . |
| 21-225_52H12_HC | . | . | . | . | . | . | . | . | . | . | . | . | . | . | . | . | . | . | . | . | . | . | . | . | . | . |
| 21-225_56A5_HC | . | . | . | . | . | . | . | . | . | . | . | . | . | . | . | . | . | . | . | . | . | . | . | . | . | . |
| 21-225_56H1_HC | . | . | . | . | . | . | . | . | . | . | . | . | . | . | . | . | . | . | . | . | . | . | . | . | . | . |
| 21-225_57B2_HC | . | . | . | . | . | . | . | . | . | . | . | . | . | . | . | . | . | . | . | . | . | . | . | . | . | . |
| 21-225_5E11_HC | . | . | . | . | . | . | . | . | . | . | . | . | . | . | . | . | . | . | . | . | . | . | . | . | . | . |
| 21-225_5F4_HC | . | . | . | . | . | . | . | . | . | . | . | . | . | . | . | . | . | . | . | . | . | . | . | V | . | . |
| 21-225_70E12_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | S | M | N | W | V | R | Q | A | P | G | K | G | L |
| 21-225_14D6_HC | . | . | . | . | . | . | . | . | . | . | . | . | . | T | . | . | . | . | . | . | . | . | . | . | . | . |
| 21-225_157E4_HC | . | . | . | . | . | . | . | . | . | . | . | . | . | T | . | . | . | . | . | . | . | . | . | . | . | . |
| 21-225_157H10_HC | . | . | . | . | . | . | . | . | . | . | . | . | . | T | . | . | . | . | . | . | . | . | . | . | . | . |
| 21-225_15A2_HC | . | . | . | . | . | . | . | . | . | . | . | . | . | T | . | . | . | . | . | . | . | . | . | . | . | . |
| 21-225_16A11_HC | . | . | . | . | . | . | . | . | . | . | . | . | . | . | . | . | . | . | . | . | . | . | . | . | . | . |
| 21-225_16B8_HC | . | . | . | . | . | . | . | . | . | . | . | . | . | . | . | . | . | . | . | . | . | . | . | . | . | . |
| 21-225_16H1_HC | . | . | . | . | . | . | . | . | . | . | . | . | . | . | . | . | . | . | . | . | . | . | . | . | . | . |
| 21-225_17H5_HC | . | . | . | . | . | . | . | . | . | . | . | . | . | . | . | . | . | . | . | . | . | . | . | . | . | . |
| 21-225_17H6_HC | . | . | . | . | . | . | . | . | . | . | . | . | . | . | . | . | . | . | . | . | . | . | . | R | . | . |
| 21-225_1F1_HC | . | . | . | . | . | . | . | . | . | . | . | . | . | N | . | . | . | . | . | . | . | . | . | . | . | . |
| 21-225_20H7_HC | . | . | . | . | . | . | . | . | . | . | . | . | . | . | . | . | . | . | . | . | . | . | . | . | . | . |
| 21-225_22B12_HC | . | . | . | . | . | . | . | . | . | . | . | . | . | . | . | . | . | . | . | . | . | . | . | . | . | . |
| 21-225_23A11_HC | . | . | . | . | . | . | . | . | . | . | . | . | . | . | . | . | . | I | . | . | . | . | . | . | . | . |
| 21-225_27C5_HC | . | . | . | . | . | . | . | . | . | . | . | . | . | . | . | . | . | . | . | . | . | . | . | . | . | . |
| 21-225_27H12_HC | . | . | . | . | . | . | . | . | . | . | . | . | . | . | L | . | . | . | . | . | . | . | . | . | . | . |
| 21-225_2A4_HC | . | . | . | . | . | . | . | . | . | . | . | . | . | . | . | . | . | . | . | . | . | . | . | . | . | . |
| 21-225_2C12_HC | . | . | . | . | . | . | . | . | . | . | . | . | . | . | . | . | . | . | . | . | . | . | . | . | . | . |
| 21-225_32B3_HC | . | . | . | . | . | . | . | . | . | . | . | . | . | . | . | . | . | . | . | . | . | . | . | . | . | . |
| 21-225_35H8_HC | . | . | . | . | . | . | . | . | . | . | . | . | . | . | . | . | . | . | . | . | . | . | . | . | . | . |
| 21-225_50G10_HC | . | . | . | . | . | . | . | . | . | . | . | . | . | . | . | . | . | . | . | . | . | . | . | . | . | . |
| 21-225_52E3_HC | . | . | . | . | . | . | . | . | . | . | . | . | . | K | . | . | . | . | . | . | . | . | . | . | . | . |
| 21-225_52H12_HC | . | . | . | . | . | . | . | . | . | . | . | . | . | . | . | . | . | . | . | . | . | . | . | . | . | . |
| 21-225_56A5_HC | . | . | . | . | . | . | . | . | . | . | . | . | . | . | . | . | . | . | . | . | . | . | . | . | . | . |
| 21-225_56H1_HC | . | . | . | . | . | . | N | . | . | . | . | . | . | R | . | . | . | . | . | . | G | . | . | . | . | . |
| 21-225_57B2_HC | . | . | . | . | . | . | . | . | . | . | . | . | . | . | . | . | . | . | . | . | . | . | . | . | . | . |
| 21-225_5E11_HC | . | . | . | . | . | . | . | . | . | . | . | . | . | . | . | . | . | . | . | . | . | . | . | . | . | . |
| 21-225_5F4_HC | . | . | . | . | . | . | . | . | . | . | . | . | . | . | . | . | . | . | . | . | . | . | . | . | . | . |
| 21-225_70E12_HC | . | . | . | . | . | . | . | . | . | . | . | . | . | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | S | S | I | S | G | S | - | - | - | S | S | Y | I | Y | Y | A | D | S | V | K | G | R | F |
| 21-225_14D6_HC | . | . | . | . | . | . | . | . | . | . | . | . | G | . | . | . | . | . | . | . | . | . | . | . | . | . |
| 21-225_157E4_HC | . | . | . | . | . | . | . | . | . | . | . | . | . | . | . | . | . | . | . | . | . | . | . | . | . | . |
| 21-225_157H10_HC | . | . | . | . | . | . | . | . | . | . | . | . | . | . | . | . | N | . | . | . | . | . | . | . | . | . |
| 21-225_15A2_HC | . | . | . | . | . | . | . | . | . | . | . | . | G | . | . | . | . | . | . | . | . | . | . | . | . | . |
| 21-225_16A11_HC | . | . | . | . | . | . | . | . | . | . | . | . | . | . | L | . | . | . | . | . | . | . | . | . | . | . |
| 21-225_16B8_HC | . | . | I | . | . | . | . | . | . | . | . | . | . | T | . | . | . | . | . | . | . | . | . | . | . | . |
| 21-225_16H1_HC | . | . | . | . | . | . | . | . | . | . | . | . | . | T | . | . | . | . | . | . | . | . | . | . | . | . |
| 21-225_17H5_HC | . | . | . | . | . | . | . | S | . | . | . | . | . | T | . | . | . | . | . | . | . | . | . | . | . | . |
| 21-225_17H6_HC | . | . | . | . | . | . | . | . | . | . | . | . | . | . | . | . | . | . | . | . | . | . | . | . | . | . |
| 21-225_1F1_HC | . | . | . | . | . | . | . | S | . | . | . | . | . | . | . | . | . | . | . | . | . | . | . | . | . | . |
| 21-225_20H7_HC | . | . | . | . | . | . | . | . | . | . | . | . | . | . | F | . | . | . | . | . | . | . | . | . | . | . |
| 21-225_22B12_HC | . | . | . | . | . | . | . | . | . | . | . | . | . | . | . | T | . | . | . | . | . | . | . | . | . | . |
| 21-225_23A11_HC | . | . | . | . | . | . | . | . | . | . | . | . | . | T | . | . | . | . | . | . | . | . | . | . | . | I |
| 21-225_27C5_HC | . | . | . | . | . | T | . | S | . | . | . | . | D | . | . | . | . | . | . | . | . | . | . | . | . | . |
| 21-225_27H12_HC | . | . | . | . | . | . | . | . | . | . | . | . | . | . | N | . | . | . | T | . | . | . | . | . | . | . |
| 21-225_2A4_HC | . | . | . | . | . | . | . | . | . | . | . | . | . | T | . | . | . | . | . | . | . | . | . | . | . | I |
| 21-225_2C12_HC | . | . | . | . | . | . | . | . | . | . | . | . | . | . | . | M | . | . | . | . | . | . | . | . | . | . |
| 21-225_32B3_HC | . | . | . | . | . | . | . | . | . | . | . | . | . | T | . | . | . | . | . | . | . | . | . | . | . | . |
| 21-225_35H8_HC | . | . | . | . | . | . | . | . | T | . | . | . | G | . | F | . | . | . | . | . | . | . | . | . | . | . |
| 21-225_50G10_HC | . | . | . | . | . | . | . | . | . | . | . | . | . | . | . | . | . | . | . | . | . | . | . | . | . | . |
| 21-225_52E3_HC | . | . | . | . | . | . | . | S | G | . | . | . | N | . | S | . | . | . | . | . | . | . | . | . | . | . |
| 21-225_52H12_HC | . | . | . | . | . | . | . | . | . | . | . | . | . | T | . | . | . | . | . | . | . | . | . | . | . | . |
| 21-225_56A5_HC | . | . | . | . | . | . | . | . | . | . | . | . | . | . | . | . | . | . | . | . | . | . | . | . | . | . |
| 21-225_56H1_HC | . | . | I | . | . | . | . | . | . | . | . | . | . | T | D | . | . | . | . | . | . | . | . | . | . | . |
| 21-225_57B2_HC | . | . | . | . | . | . | . | . | . | . | . | . | . | . | . | . | N | . | T | . | . | . | . | . | . | . |
| 21-225_5E11_HC | . | . | . | . | . | . | . | . | . | . | . | . | . | T | . | . | . | . | . | . | . | . | . | . | . | . |
| 21-225_5F4_HC | . | . | . | . | . | . | . | . | . | . | . | . | . | . | . | . | . | . | . | . | . | . | . | . | . | . |
| 21-225_70E12_HC | . | . | . | . | . | . | . | . | N | . | . | . | . | T | . | . | . | . | T | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | A | K | N | S | L | Y | L | Q | M | N | S | L | R | A | E | D | T | A | V | Y |
| 21-225_14D6_HC | . | M | . | . | . | . | . | . | . | . | . | . | . | . | . | . | . | . | . | . | . | . | . | . | . | . |
| 21-225_157E4_HC | . | . | . | . | . | . | . | . | . | . | . | . | . | . | . | . | . | . | K | . | . | . | . | . | . | . |
| 21-225_157H10_HC | . | . | . | . | . | . | . | . | . | . | . | . | . | . | . | . | . | . | . | . | . | . | . | . | . | . |
| 21-225_15A2_HC | . | . | . | . | . | . | . | . | . | . | . | . | . | . | . | . | A | . | . | . | . | . | . | . | . | . |
| 21-225_16A11_HC | . | . | . | . | . | . | . | . | . | . | . | . | . | . | . | . | . | . | . | . | . | . | . | . | . | . |
| 21-225_16B8_HC | . | . | . | . | . | . | . | . | . | . | . | . | . | . | . | . | . | . | . | . | . | . | . | . | . | . |
| 21-225_16H1_HC | . | . | . | . | . | . | . | . | . | . | . | . | . | . | . | . | . | . | . | . | . | . | . | . | . | . |
| 21-225_17H5_HC | . | . | . | . | . | . | . | . | . | . | . | . | . | . | . | . | . | . | . | . | . | . | . | . | . | . |
| 21-225_17H6_HC | . | . | F | . | . | . | . | . | . | . | . | . | . | . | . | . | . | . | . | . | . | . | . | . | . | . |
| 21-225_1F1_HC | . | . | . | . | . | . | . | . | . | . | . | . | . | . | . | . | . | . | . | . | . | . | . | . | . | . |
| 21-225_20H7_HC | . | . | . | . | . | . | . | . | . | . | . | . | . | . | . | . | . | . | . | . | . | . | . | . | . | . |
| 21-225_22B12_HC | . | . | . | . | . | . | . | . | . | . | . | . | . | . | . | . | . | . | . | . | . | . | . | . | . | . |
| 21-225_23A11_HC | A | . | . | . | . | . | . | . | . | . | . | . | . | . | . | . | . | . | . | . | . | . | . | . | . | . |
| 21-225_27C5_HC | . | . | . | . | . | . | . | . | . | . | . | . | . | . | . | . | . | . | . | . | . | . | . | . | . | . |
| 21-225_27H12_HC | . | . | . | . | . | . | . | . | . | . | . | F | . | . | . | . | . | . | . | . | . | . | . | . | . | . |
| 21-225_2A4_HC | A | . | . | . | . | . | . | . | . | . | . | . | . | . | . | . | . | . | . | . | . | . | . | . | . | . |
| 21-225_2C12_HC | . | . | . | . | . | . | . | . | D | . | . | . | . | . | . | . | . | . | . | . | . | . | . | . | . | . |
| 21-225_32B3_HC | . | . | . | . | . | . | . | . | . | . | . | . | . | . | . | . | . | . | . | . | . | . | . | . | . | . |
| 21-225_35H8_HC | . | . | . | . | . | . | . | . | K | . | V | . | . | . | . | . | . | . | . | . | . | . | . | . | . | . |
| 21-225_50G10_HC | . | . | . | . | . | . | . | . | . | . | . | . | . | . | . | . | . | . | . | . | . | . | . | . | . | . |
| 21-225_52E3_HC | . | . | . | . | . | . | . | E | . | . | . | . | . | . | . | . | . | . | . | . | . | . | . | . | . | . |
| 21-225_52H12_HC | . | V | . | . | . | . | . | . | . | . | . | . | . | . | V | . | . | . | . | . | . | . | . | . | . | . |
| 21-225_56A5_HC | . | . | . | . | . | . | . | . | . | . | . | . | . | . | . | . | . | . | . | . | . | . | . | . | . | . |
| 21-225_56H1_HC | . | . | . | . | . | . | . | . | . | . | . | . | . | . | . | . | . | . | . | . | . | . | . | . | . | . |
| 21-225_57B2_HC | . | . | . | . | . | . | . | . | . | . | . | . | . | . | . | . | . | . | . | . | . | . | . | . | . | . |
| 21-225_5E11_HC | . | . | . | . | . | . | . | . | . | . | . | . | . | . | . | . | . | . | . | . | . | . | . | . | . | F |
| 21-225_5F4_HC | . | . | . | . | . | . | . | . | . | . | . | F | . | . | . | . | . | . | . | . | . | . | . | . | . | . |
| 21-225_70E12_HC | . | . | . | . | . | . | . | . | . | . | . | . | . | . | . | D | . | . | T | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | H_CDR3 | | | | | | | | | | | | | | | | | | | |
| CONSENSUS | Y | C | A | R | V | A | S | - | - | - | - | - | - | - | - | - | - | - | - | - | - | - | - | - | - | - |
| 21-225_14D6_HC | . | . | . | . | . | . | A | . | . | . | . | . | . | . | . | . | . | . | . | . | . | . | . | . | . | . |
| 21-225_157E4_HC | . | . | . | . | . | . | T | . | . | . | . | . | . | . | . | . | . | . | . | . | . | . | . | . | . | . |
| 21-225_157H10_HC | . | . | . | . | . | . | H | . | . | . | . | . | . | . | . | . | . | . | . | . | . | . | . | . | . | . |
| 21-225_15A2_HC | . | . | . | . | . | . | . | . | . | . | . | . | . | . | . | . | . | . | . | . | . | . | . | . | . | . |
| 21-225_16A11_HC | . | . | . | . | . | . | A | . | . | . | . | . | . | . | . | . | . | . | . | . | . | . | . | . | . | . |
| 21-225_16B8_HC | . | . | . | . | . | . | . | . | . | . | . | . | . | . | . | . | . | . | . | . | . | . | . | . | . | . |
| 21-225_16H1_HC | . | . | . | . | . | . | A | . | . | . | . | . | . | . | . | . | . | . | . | . | . | . | . | . | . | . |
| 21-225_17H5_HC | . | . | . | . | . | . | . | . | . | . | . | . | . | . | . | . | . | . | . | . | . | . | . | . | . | . |
| 21-225_17H6_HC | . | . | . | . | . | . | . | . | . | . | . | . | . | . | . | . | . | . | . | . | . | . | . | . | . | . |
| 21-225_1F1_HC | . | . | . | . | . | N | L | . | . | . | . | . | . | . | . | . | . | . | . | . | . | . | . | . | . | . |
| 21-225_20H7_HC | . | . | . | . | . | . | A | . | . | . | . | . | . | . | . | . | . | . | . | . | . | . | . | . | . | . |
| 21-225_22B12_HC | . | . | . | . | . | . | A | . | . | . | . | . | . | . | . | . | . | . | . | . | . | . | . | . | . | . |
| 21-225_23A11_HC | . | . | . | . | . | . | . | . | . | . | . | . | . | . | . | . | . | . | . | . | . | . | . | . | . | . |
| 21-225_27C5_HC | . | . | . | . | . | . | . | . | . | . | . | . | . | . | . | . | . | . | . | . | . | . | . | . | . | . |
| 21-225_27H12_HC | . | . | . | . | . | . | . | . | . | . | . | . | . | . | . | . | . | . | . | . | . | . | . | . | . | . |
| 21-225_2A4_HC | . | . | . | . | . | . | . | . | . | . | . | . | . | . | . | . | . | . | . | . | . | . | . | . | . | . |
| 21-225_2C12_HC | . | . | . | . | . | N | L | . | . | . | . | . | . | . | . | . | . | . | . | . | . | . | . | . | . | . |
| 21-225_32B3_HC | . | . | . | . | . | . | G | . | . | . | . | . | . | . | . | . | . | . | . | . | . | . | . | . | . | . |
| 21-225_35H8_HC | . | . | . | . | . | S | G | . | . | . | . | . | . | . | . | . | . | . | . | . | . | . | . | . | . | . |
| 21-225_50G10_HC | . | . | . | . | . | . | T | . | . | . | . | . | . | . | . | . | . | . | . | . | . | . | . | . | . | . |
| 21-225_52E3_HC | . | . | . | . | . | S | . | . | . | . | . | . | . | . | . | . | . | . | . | . | . | . | . | . | . | . |
| 21-225_52H12_HC | . | . | . | . | . | N | . | . | . | . | . | . | . | . | . | . | . | . | . | . | . | . | . | . | . | . |
| 21-225_56A5_HC | . | . | . | . | . | . | T | . | . | . | . | . | . | . | . | . | . | . | . | . | . | . | . | . | . | . |
| 21-225_56H1_HC | . | . | . | . | . | . | . | . | . | . | . | . | . | . | . | . | . | . | . | . | . | . | . | . | . | . |
| 21-225_57B2_HC | . | . | . | . | . | . | G | . | . | . | . | . | . | . | . | . | . | . | . | . | . | . | . | . | . | . |
| 21-225_5E11_HC | . | . | . | . | . | . | . | . | . | . | . | . | . | . | . | . | . | . | . | . | . | . | . | . | . | . |
| 21-225_5F4_HC | . | . | . | . | . | N | N | . | . | . | . | . | . | . | . | . | . | . | . | . | . | . | . | . | . | . |
| 21-225_70E12_HC | . | . | . | . | . | . | A | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | - | F | D | Y | W | G | Q | G | T | L | V | T | V | S | S |
| 21-225_14D6_HC | . | . | . | . | . | . | . | S | . | . | . | . | . | . | . | S | . | . | . |
| 21-225_157E4_HC | . | . | . | . | . | . | . | . | . | . | . | . | . | . | . | . | . | . | . |
| 21-225_157H10_HC | . | . | . | . | . | . | . | . | . | . | . | . | . | . | . | . | . | . | . |
| 21-225_15A2_HC | . | . | . | . | . | . | . | . | . | . | . | . | . | . | . | . | . | . | . |
| 21-225_16A11_HC | . | . | . | . | . | . | . | . | . | . | . | . | . | . | . | . | . | . | . |
| 21-225_16B8_HC | . | . | . | . | . | . | . | . | . | . | . | . | . | . | . | . | . | . | . |
| 21-225_16H1_HC | . | . | . | . | . | . | . | . | . | . | . | . | . | . | . | . | . | . | . |
| 21-225_17H5_HC | . | . | . | . | . | . | . | . | . | . | . | . | . | . | . | . | . | . | . |
| 21-225_17H6_HC | . | . | . | . | . | . | . | . | . | . | . | . | . | . | . | . | . | . | . |
| 21-225_1F1_HC | . | . | . | . | . | . | . | . | . | . | . | . | . | . | . | . | . | . | . |
| 21-225_20H7_HC | . | . | . | . | . | L | . | . | . | . | . | . | . | . | . | . | . | . | . |
| 21-225_22B12_HC | . | . | . | . | . | L | . | S | . | . | . | . | . | . | . | . | . | . | . |
| 21-225_23A11_HC | . | . | . | . | . | . | . | . | . | . | . | . | . | . | . | . | . | . | . |
| 21-225_27C5_HC | . | . | . | . | . | . | . | C | . | . | . | . | . | . | . | . | . | . | . |
| 21-225_27H12_HC | . | . | . | . | . | . | . | . | . | . | . | . | . | . | . | . | . | . | . |
| 21-225_2A4_HC | . | . | . | . | . | . | . | . | . | . | . | . | . | . | . | . | . | . | . |
| 21-225_2C12_HC | . | . | . | . | . | . | . | . | . | . | . | . | . | . | . | . | . | . | . |
| 21-225_32B3_HC | . | . | . | . | . | . | . | . | . | . | . | . | . | . | . | . | . | . | . |
| 21-225_35H8_HC | . | . | . | . | . | . | . | . | . | . | . | . | . | . | . | . | . | . | . |
| 21-225_50G10_HC | . | . | . | . | . | . | . | . | . | . | . | . | . | . | . | . | . | . | . |
| 21-225_52E3_HC | . | . | . | . | . | N | . | . | . | . | . | . | . | . | . | . | . | . | . |
| 21-225_52H12_HC | . | . | . | . | . | . | . | . | . | . | . | . | . | . | . | . | . | . | . |
| 21-225_56A5_HC | . | . | . | . | . | . | . | . | . | . | . | . | . | . | . | . | . | . | . |
| 21-225_56H1_HC | . | . | . | . | . | . | . | . | . | . | . | . | . | . | . | . | . | . | . |
| 21-225_57B2_HC | . | . | . | . | . | . | . | . | . | . | . | . | . | . | . | . | . | . | . |
| 21-225_5E11_HC | . | . | . | . | . | . | . | . | . | . | . | . | . | . | . | . | . | . | . |
| 21-225_5F4_HC | . | . | . | . | . | . | . | . | . | . | . | . | . | . | . | . | . | . | . |
| 21-225_70E12_HC | . | . | . | . | . | . | . | C | . | . | . | . | . | . | . | . | . | . | . |

**FIGURE 57**

Table 63. Consensus 29-VH4|4-30.1/D3|3-22|RF2/JH6 (SEQ ID NO: 50280):

QVQLQES-GPGLVKPSQTLSLTCTVSG-GSISSG---DYYWNWIRQHPGKGLEWIGYIFY----SGSTYYNPSLKSRVTISVDTSKNQFSLKLSSVTAADTAVYYCARGDYDGSGSY------------HYYYGMDVWGQGTTVTVSS wherein:

G at position 34 can be substituted with V

D at position 38 can be substituted with V, G or S

Y at position 40 can be substituted with H

N at position 42 can be substituted with S

Y at position 57 can be substituted with N or F

I at position 58 can be substituted with L

F at position 59 can be substituted with Y or H

Y at position 60 can be substituted with H

Y at position 70 can be substituted with N

K at position 75 can be substituted with R

Y at position 132 can be substituted with F or H

Y at position 134 can be substituted with H

M at position 136 can be substituted with L

Ser Xaa Xaa Tyr Xaa Trp Xaa (SEQ ID NO: 50230)

Xaa Xaa Xaa Xaa Ser Gly Ser Thr Tyr Xaa Asn Pro Ser Leu Xaa Ser (SEQ ID NO: 50231)

Gly Asp Tyr Asp Gly Ser Gly Ser Tyr His Xaa Tyr Xaa Gly Xaa Asp Val (SEQ ID NO: 50232)

Ser Gly Asp Tyr Tyr Trp Asn (SEQ ID NO: 50510)

**FIGURE 57**

Tyr Ile Phe Tyr Ser Gly Ser Thr Tyr Tyr Asn Pro Ser Leu Lys Ser (SEQ ID NO: 50511)

Gly Asp Tyr Asp Gly Ser Gly Ser Tyr His Tyr Tyr Tyr Gly Met Asp Val (SEQ ID NO: 50512)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 |
|---|---|---|---|---|---|---|---|---|---|---|---|---|---|---|---|---|---|---|---|---|---|---|---|---|---|---|---|
| | H_FR1 | | | | | | | | | | | | | | | | | | | | | | | | | | |
| CONSENSUS | Q | V | Q | L | Q | E | S | - | G | P | G | L | V | K | P | S | Q | T | L | S | L | T | C | T | V | S | G |
| 21-225_190A3_HC | . | . | . | . | . | . | . | . | . | . | . | . | . | . | . | . | . | . | . | . | . | . | . | . | . | . | . |
| 21-225_190A7_HC | . | . | . | . | . | . | . | . | . | . | . | . | . | N | . | . | . | . | . | . | . | . | . | . | . | . | . |
| 21-225_190B4_HC | . | . | . | . | . | . | . | . | . | . | . | . | . | . | . | . | . | . | . | . | . | . | . | . | . | . | . |
| 21-225_191A3_HC | . | . | . | . | . | . | . | . | . | . | . | . | . | . | . | . | . | . | . | . | . | . | . | . | . | . | . |
| 21-225_191D3_HC | . | . | . | . | K | . | . | . | . | . | . | . | . | . | . | . | . | . | . | . | . | . | . | . | . | . | . |
| 21-225_191F1_HC | . | . | . | . | . | . | . | . | . | . | . | . | . | . | . | . | . | . | . | . | . | . | . | . | . | . | . |
| 21-225_191H7_HC | . | . | . | . | . | . | . | . | . | . | . | . | . | . | . | . | . | . | . | . | . | . | . | . | . | . | . |
| 21-225_192B3_HC | . | . | . | . | . | . | . | . | . | . | . | . | . | . | . | . | . | . | . | . | . | . | . | . | . | . | . |
| 21-225_192G12_HC | . | . | . | . | . | . | . | . | . | . | . | . | . | . | . | . | . | . | . | . | . | . | . | . | . | . | . |
| 21-225_192H3_HC | . | . | . | . | . | . | . | . | . | . | . | . | . | . | . | . | . | . | . | . | . | . | . | . | . | . | . |
| 21-225_193A1_HC | . | . | . | . | . | . | . | . | . | . | . | . | . | . | . | . | . | . | . | . | . | . | . | . | . | . | . |
| 21-225_193B1_HC | . | . | . | . | . | . | . | . | . | . | . | . | . | . | . | . | . | . | . | . | . | . | . | . | . | . | . |
| 21-225_193C8_HC | . | . | . | . | . | . | . | . | . | . | . | . | . | . | . | . | . | . | . | . | . | . | . | . | . | . | . |
| 21-225_193D8_HC | . | . | . | . | . | . | . | . | . | . | . | . | . | . | . | . | . | . | . | . | . | . | . | . | . | . | . |
| 21-225_193F3_HC | . | . | . | . | . | . | . | . | . | . | . | . | . | . | . | . | . | . | . | . | . | . | . | . | . | . | . |
| 21-225_193G8_HC | . | . | . | . | . | . | K | . | . | . | . | . | . | . | . | . | . | . | . | . | . | . | . | . | . | . | . |
| 21-225_193H6_HC | . | . | . | . | . | . | . | . | . | . | . | . | . | . | . | . | . | . | . | . | . | . | . | . | . | . | . |
| 21-225_194E5_HC | . | . | . | . | . | . | . | . | . | . | . | . | . | . | . | . | . | . | . | . | . | . | . | . | . | . | . |
| 21-225_194E6_HC | . | . | . | . | . | . | . | . | . | . | . | . | . | . | . | . | . | . | . | . | . | . | . | . | . | . | . |
| 21-225_196H12_HC | . | . | . | . | . | . | . | . | . | . | . | . | . | N | . | . | . | . | . | . | . | . | . | . | . | . | . |
| 21-225_197F4_HC | . | . | . | . | . | . | . | . | . | . | . | . | . | . | . | . | . | . | . | . | . | . | . | . | . | . | . |
| 21-225_198B1_HC | . | . | . | . | K | . | . | . | . | . | . | . | . | . | . | . | . | . | . | . | . | . | . | . | . | . | . |
| 21-225_198E1_HC | . | . | . | . | . | . | . | . | . | . | . | . | . | . | . | . | . | . | . | . | . | . | . | . | . | . | . |
| 21-225_199C2_HC | . | . | . | . | . | . | . | . | . | . | . | . | . | . | . | . | . | . | . | . | . | . | . | . | . | . | . |
| 21-225_199C7_HC | . | . | . | . | . | . | . | . | . | . | . | . | . | . | . | . | . | . | . | . | . | . | . | . | . | . | . |
| 21-225_200F10_HC | . | . | . | . | . | . | . | . | . | . | . | . | . | . | . | . | . | . | . | . | . | . | . | . | . | . | . |

FIGURE 57

| Reference # | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 |
|---|---|---|---|---|---|---|---|---|---|---|---|---|---|---|---|---|---|---|---|---|---|---|---|---|---|---|---|
| | | | | | | H_CDR1 | | | | | | | | | | H_FR2 | | | | | | | | | | | |
| CONSENSUS | - | G | S | I | S | S | G | - | - | - | D | Y | Y | W | N | W | I | R | Q | H | P | G | K | G | L | E | W |
| 21-225_190A3_HC | . | . | . | . | N | . | . | . | . | . | G | . | . | . | . | . | . | . | . | . | . | . | . | . | . | . | . |
| 21-225_190A7_HC | . | . | . | . | . | . | . | . | . | . | V | . | . | . | S | . | . | . | . | . | . | . | . | . | . | . | . |
| 21-225_190B4_HC | . | . | . | . | . | . | . | . | . | . | . | . | . | . | . | . | . | . | . | . | . | . | . | . | . | . | . |
| 21-225_191A3_HC | . | . | . | . | . | . | . | . | . | . | . | . | . | . | . | . | . | . | . | . | . | . | . | . | . | . | . |
| 21-225_191D3_HC | . | . | . | . | N | . | . | . | . | . | . | . | . | . | . | . | . | . | . | . | . | . | . | . | . | D | . |
| 21-225_191F1_HC | . | . | . | . | N | . | . | . | . | . | G | . | . | . | . | . | . | . | . | . | . | . | . | . | . | . | . |
| 21-225_191H7_HC | . | . | . | . | N | . | . | . | . | . | . | . | . | . | . | . | . | . | . | . | . | . | . | . | . | . | . |
| 21-225_192B3_HC | . | . | . | . | . | . | . | . | . | . | . | . | . | . | . | . | . | . | . | . | . | . | . | . | . | . | . |
| 21-225_192G12_HC | . | . | . | . | . | . | . | . | . | . | V | . | H | . | S | . | . | . | . | . | . | . | . | . | . | . | . |
| 21-225_192H3_HC | . | . | . | . | . | . | V | . | . | . | S | . | . | . | S | . | . | . | . | R | . | . | . | . | . | . | . |
| 21-225_193A1_HC | . | . | . | . | . | . | . | . | . | . | V | . | . | . | S | . | . | . | . | . | . | . | . | . | . | . | . |
| 21-225_193B1_HC | . | . | . | . | . | . | . | . | . | . | V | . | . | . | S | . | . | . | . | . | . | . | . | . | . | . | . |
| 21-225_193C8_HC | . | . | . | . | . | . | . | . | . | . | . | . | . | . | . | . | . | . | . | . | . | . | . | . | . | . | . |
| 21-225_193D8_HC | . | . | . | . | N | . | . | . | . | . | G | . | . | . | . | . | . | . | . | . | . | . | . | . | . | . | . |
| 21-225_193F3_HC | . | . | . | . | N | . | . | . | . | . | . | . | . | . | . | . | . | . | . | . | . | . | . | . | . | . | . |
| 21-225_193G8_HC | . | . | . | V | . | . | . | . | . | . | V | . | . | . | S | . | . | . | . | . | . | . | . | . | . | . | . |
| 21-225_193H6_HC | . | . | . | . | N | . | . | . | . | . | . | . | . | . | . | . | . | . | . | . | . | . | . | . | . | . | . |
| 21-225_194E5_HC | . | . | . | . | N | . | . | . | . | . | . | . | . | . | . | . | . | . | H | . | . | . | . | . | . | . | . |
| 21-225_194E6_HC | . | D | . | . | N | . | . | . | . | . | . | . | . | . | . | . | . | . | . | . | . | . | . | . | . | . | . |
| 21-225_196H12_HC | . | . | . | . | . | . | . | . | . | . | V | . | . | . | S | . | . | . | . | . | . | . | . | . | . | . | . |
| 21-225_197F4_HC | . | . | . | . | . | . | . | . | . | . | . | . | . | . | . | . | . | . | . | . | . | . | . | . | . | . | . |
| 21-225_198B1_HC | . | . | . | . | N | . | . | . | . | . | . | . | . | . | . | . | F | . | . | . | . | . | . | . | . | . | . |
| 21-225_198E1_HC | . | . | . | . | N | . | . | . | . | . | G | . | . | . | . | . | . | . | . | . | . | . | . | . | . | . | . |
| 21-225_199C2_HC | . | . | . | . | . | . | . | . | . | . | . | . | . | . | . | . | L | . | . | . | . | E | . | . | . | . | . |
| 21-225_199C7_HC | . | . | . | . | N | . | . | . | . | . | G | . | . | . | . | . | . | . | . | . | . | . | . | . | . | . | . |
| 21-225_200F10_HC | . | . | . | . | . | . | . | . | . | . | . | . | . | . | . | . | L | . | . | . | . | E | . | . | . | . | . |

FIGURE 57

| Reference # | 55 | 56 | 57 | 58 | 59 | 60 | 61 | 62 | 63 | 64 | 65 | 67 | 68 | 69 | 70 | 71 | 72 | 73 | 74 | 75 | 76 | 77 | 78 | 79 | 80 | 81 | 82 | 83 |
|---|---|---|---|---|---|---|---|---|---|---|---|---|---|---|---|---|---|---|---|---|---|---|---|---|---|---|---|---|
| | | | H_CDR2 | | | | | | | | | | | | | | | | | | | | | | | | | |
| CONSENSUS | I | G | Y | I | F | Y | - | - | - | - | S | S | T | Y | Y | N | P | S | L | K | S | R | V | T | I | S | V | D |
| 21-225_190A3_HC | . | . | F | . | . | . | . | . | . | . | . | . | . | . | . | . | . | . | . | . | . | . | . | . | . | . | . | . |
| 21-225_190A7_HC | . | . | N | . | Y | . | . | . | . | . | . | . | . | . | N | . | . | . | . | . | . | . | I | I | . | . | . | . |
| 21-225_190B4_HC | . | . | . | . | . | . | . | . | . | . | . | . | . | . | . | . | . | . | . | . | . | . | . | . | . | . | . | . |
| 21-225_191A3_HC | . | . | . | . | . | . | . | . | . | . | . | . | . | . | . | . | . | . | . | . | . | . | . | . | . | . | . | . |
| 21-225_191D3_HC | . | . | . | . | . | . | . | . | . | . | . | . | . | . | . | . | . | . | . | . | . | . | . | . | . | . | . | . |
| 21-225_191F1_HC | . | . | . | . | . | . | . | . | . | . | . | . | . | . | . | . | . | . | . | . | . | . | . | . | . | . | . | . |
| 21-225_191H7_HC | . | . | . | . | . | . | . | . | . | . | . | . | . | . | . | . | . | . | . | . | . | . | . | . | . | . | . | . |
| 21-225_192B3_HC | . | . | F | . | . | . | . | . | . | . | . | . | . | . | . | . | . | . | . | . | . | . | . | . | . | . | . | . |
| 21-225_192G12_HC | . | . | N | . | H | . | . | . | . | . | . | . | . | . | N | . | . | . | . | . | . | . | . | . | . | . | . | . |
| 21-225_192H3_HC | . | . | N | L | Y | . | . | . | . | . | . | . | . | . | . | . | . | . | . | R | . | . | A | . | . | . | . | . |
| 21-225_193A1_HC | . | . | N | . | Y | . | . | . | . | . | . | . | . | . | N | . | . | . | . | . | . | . | L | . | . | . | A | . |
| 21-225_193B1_HC | . | . | N | . | Y | . | . | . | . | . | . | . | . | . | N | . | . | . | . | . | . | . | . | . | . | . | . | . |
| 21-225_193C8_HC | . | . | . | . | . | . | . | . | . | . | . | . | . | . | . | . | . | . | . | . | . | . | . | . | . | . | . | . |
| 21-225_193D8_HC | . | . | F | . | . | . | . | . | . | . | . | . | . | . | . | . | . | . | . | . | . | . | . | S | . | . | . | . |
| 21-225_193F3_HC | . | . | . | . | . | . | . | . | . | . | . | . | . | . | . | . | . | . | . | . | . | . | . | . | . | . | . | . |
| 21-225_193G8_HC | . | . | N | . | Y | . | . | . | . | . | . | . | . | . | N | . | . | . | . | . | . | . | . | . | . | . | . | . |
| 21-225_193H6_HC | . | . | . | . | . | . | . | . | . | . | . | . | . | . | . | . | . | . | . | . | . | . | . | . | . | . | . | . |
| 21-225_194E5_HC | . | . | . | . | . | . | . | . | . | . | . | . | . | . | . | . | . | . | . | . | . | . | . | . | . | . | . | . |
| 21-225_194E6_HC | . | . | F | . | . | . | . | . | . | . | . | . | . | . | . | . | . | . | . | . | . | . | . | . | . | . | I | . |
| 21-225_196H12_HC | . | . | N | . | Y | . | . | . | . | . | . | . | . | . | N | . | . | . | . | . | . | . | I | I | . | . | . | . |
| 21-225_197F4_HC | . | . | . | . | . | . | . | . | . | . | . | . | . | . | . | . | . | . | . | . | . | . | I | . | . | . | . | . |
| 21-225_198B1_HC | . | . | . | . | . | H | . | . | . | . | . | . | . | . | . | . | . | . | . | . | . | . | . | . | V | . | . | . |
| 21-225_198E1_HC | . | . | F | . | . | . | . | . | . | . | . | . | . | . | . | . | . | . | . | . | . | . | L | S | . | . | . | . |
| 21-225_199C2_HC | . | . | . | . | . | . | . | . | . | . | . | . | . | . | . | . | . | . | . | . | . | . | . | . | . | . | . | . |
| 21-225_199C7_HC | . | . | F | . | . | . | . | . | . | . | . | . | . | . | . | . | . | . | . | . | . | . | . | S | . | . | . | . |
| 21-225_200F10_HC | . | . | . | . | . | . | . | . | . | . | . | . | . | . | . | . | . | . | . | . | . | . | I | . | . | . | . | . |

# FIGURE 57

| Reference # | 84 | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 | 109 | 110 |
|---|---|---|---|---|---|---|---|---|---|---|---|---|---|---|---|---|---|---|---|---|---|---|---|---|---|---|---|
| | H_FR3 | | | | | | | | | | | | | | | | | | | | | | | | | | |
| CONSENSUS | T | S | K | N | Q | F | S | L | K | L | S | S | V | T | A | A | D | T | A | V | Y | Y | C | A | R | G | D |
| 21-225_190A3_HC | . | . | . | . | . | . | . | . | . | . | N | . | . | . | . | . | . | . | . | . | . | . | . | . | . | . | . |
| 21-225_190A7_HC | . | . | . | . | . | . | . | . | . | . | . | . | . | . | . | . | . | . | . | . | . | . | . | . | . | . | . |
| 21-225_190B4_HC | . | . | . | . | . | . | . | . | . | . | . | . | . | . | . | . | . | . | . | . | . | . | . | . | . | . | . |
| 21-225_191A3_HC | . | . | . | . | . | . | . | . | . | . | . | . | . | . | . | . | . | . | . | . | . | . | . | . | . | . | . |
| 21-225_191D3_HC | . | . | . | . | . | . | . | . | . | . | . | . | . | . | . | . | . | . | . | . | . | . | . | . | . | . | . |
| 21-225_191F1_HC | . | . | . | . | . | . | . | . | . | . | . | . | . | . | . | . | . | . | . | . | . | . | . | . | . | . | . |
| 21-225_191H7_HC | . | . | . | . | . | . | . | . | . | . | . | . | . | . | . | . | . | . | . | . | . | . | . | . | . | . | . |
| 21-225_192B3_HC | . | . | . | . | . | . | . | . | . | . | . | . | . | . | V | . | . | . | . | . | . | . | . | . | . | . | . |
| 21-225_192G12_HC | . | . | . | . | . | . | . | . | . | . | . | . | . | . | . | . | . | . | . | . | . | . | . | . | . | . | . |
| 21-225_192H3_HC | . | . | . | . | . | . | . | . | . | . | . | . | . | . | . | . | . | . | . | . | . | . | . | T | . | . | . |
| 21-225_193A1_HC | . | . | . | . | . | . | . | . | . | . | . | . | . | . | . | . | . | . | . | . | . | . | . | . | . | . | . |
| 21-225_193B1_HC | . | . | . | . | . | . | . | . | . | . | . | . | . | . | . | . | . | . | . | . | . | . | . | . | . | . | . |
| 21-225_193C8_HC | . | . | . | . | . | . | . | . | . | . | . | . | . | . | . | . | . | . | . | . | . | . | . | . | . | . | . |
| 21-225_193D8_HC | . | . | . | . | . | . | . | . | . | . | . | . | . | . | . | . | . | . | . | . | . | . | . | . | . | . | . |
| 21-225_193F3_HC | . | . | . | . | . | . | . | . | . | . | . | . | . | . | . | . | . | . | . | . | . | . | . | . | . | . | . |
| 21-225_193G8_HC | . | . | . | . | . | . | . | . | . | . | N | . | . | . | . | . | . | . | . | . | . | . | . | . | . | . | . |
| 21-225_193H6_HC | . | . | . | . | . | . | . | . | . | . | . | . | . | . | . | . | . | . | . | . | . | . | . | . | . | . | . |
| 21-225_194E5_HC | . | . | . | . | . | . | . | . | . | . | . | . | . | . | . | . | . | . | . | . | . | . | . | . | . | . | . |
| 21-225_194E6_HC | . | . | . | . | . | . | . | . | . | . | . | . | . | N | V | . | . | . | . | . | . | . | . | . | . | . | . |
| 21-225_196H12_HC | . | . | . | . | . | . | . | . | . | . | . | . | . | . | . | . | . | . | . | . | . | . | . | . | . | . | . |
| 21-225_197F4_HC | . | . | . | . | . | . | . | . | . | . | . | . | . | . | . | . | . | . | . | . | . | . | . | . | . | . | . |
| 21-225_198B1_HC | . | . | . | . | . | . | . | . | . | . | . | . | . | . | . | . | . | . | . | . | . | . | . | . | . | . | . |
| 21-225_198E1_HC | . | . | . | . | . | . | . | . | . | . | . | . | . | . | . | . | . | . | . | . | . | . | . | . | . | . | . |
| 21-225_199C2_HC | . | . | M | T | . | . | . | . | . | . | T | . | . | . | . | . | . | . | . | . | . | . | . | . | . | . | . |
| 21-225_199C7_HC | . | . | . | . | . | . | . | . | . | . | . | . | . | . | . | . | . | . | . | . | . | . | . | . | . | . | . |
| 21-225_200F10_HC | . | . | M | T | . | . | . | . | . | . | T | . | . | . | . | . | . | . | . | . | . | . | . | . | . | . | . |

## FIGURE 57

| Reference # | 111 | 112 | 113 | 114 | 115 | 116 | 117 | 118 | 119 | 120 | 121 | 122 | 123 | 124 | 125 | 126 | 127 | 128 | 129 | 130 | 133 | 134 | 135 | 136 | 137 | 138 | 139 | 140 |
|---|---|---|---|---|---|---|---|---|---|---|---|---|---|---|---|---|---|---|---|---|---|---|---|---|---|---|---|---|
| | H_CDR3 | | | | | | | | | | | | | | | | | | | | | | | | | | | |
| CONSENSUS | Y | D | G | S | G | S | Y | - | - | - | - | - | - | - | - | - | - | - | - | - | Y | Y | G | M | D | V | W | G |
| 21-225_190A3_HC | . | . | . | . | . | . | . | . | . | . | . | . | . | . | . | . | . | . | . | . | . | . | . | . | . | . | . | . |
| 21-225_190A7_HC | . | . | . | . | . | . | . | . | . | . | . | . | . | . | . | . | . | . | . | . | . | . | . | L | . | . | . | . |
| 21-225_190B4_HC | . | . | . | . | . | . | . | . | . | . | . | . | . | . | . | . | . | . | . | . | . | . | . | . | . | . | . | . |
| 21-225_191A3_HC | . | . | . | . | . | . | . | . | . | . | . | . | . | . | . | . | . | . | . | . | . | . | . | . | . | . | . | . |
| 21-225_191D3_HC | . | . | . | . | . | . | . | . | . | . | . | . | . | . | . | . | . | . | . | . | . | . | . | . | . | . | . | . |
| 21-225_191F1_HC | . | . | . | . | . | . | . | . | . | . | . | . | . | . | . | . | . | . | . | . | . | . | . | . | . | . | . | . |
| 21-225_191H7_HC | . | . | . | . | . | . | . | . | . | . | . | . | . | . | . | . | . | . | . | . | . | . | . | . | . | . | . | . |
| 21-225_192B3_HC | . | . | . | . | . | . | . | . | . | . | . | . | . | . | . | . | . | . | . | . | . | . | . | . | . | . | . | . |
| 21-225_192G12_HC | . | . | . | . | . | . | . | . | . | . | . | . | . | . | . | . | . | . | . | . | . | . | . | . | . | . | . | . |
| 21-225_192H3_HC | . | . | . | . | . | . | . | . | . | . | . | . | . | . | . | . | . | . | . | . | . | N | . | . | . | . | . | . |
| 21-225_193A1_HC | . | . | . | . | . | . | . | . | . | . | . | . | . | . | . | . | . | . | . | . | . | . | . | . | . | . | . | . |
| 21-225_193B1_HC | . | . | . | . | . | . | . | . | . | . | . | . | . | . | . | . | . | . | . | . | . | . | . | . | . | . | . | . |
| 21-225_193C8_HC | . | . | . | . | . | . | . | . | . | . | . | . | . | . | . | . | . | . | . | . | . | . | . | . | . | . | . | . |
| 21-225_193D8_HC | . | . | . | . | . | . | . | . | . | . | . | . | . | . | . | . | . | . | . | . | . | . | . | . | . | . | . | . |
| 21-225_193F3_HC | . | . | . | . | . | . | . | . | . | . | . | . | . | . | . | . | . | . | . | . | . | . | . | . | . | . | . | . |
| 21-225_193G8_HC | . | . | . | . | . | . | . | . | . | . | . | . | . | . | . | . | . | . | . | . | . | . | . | L | . | . | . | . |
| 21-225_193H6_HC | . | . | . | . | . | . | . | . | . | . | . | . | . | . | . | . | . | . | . | . | . | . | . | . | . | . | . | . |
| 21-225_194E5_HC | . | . | . | . | . | . | . | . | . | . | . | . | . | . | . | . | . | . | . | . | . | . | . | . | . | . | . | . |
| 21-225_194E6_HC | . | . | . | . | . | . | . | . | . | . | . | . | . | . | . | . | . | . | . | . | . | . | . | . | . | . | . | . |
| 21-225_196H12_HC | . | . | . | . | . | . | . | . | . | . | . | . | . | . | . | . | . | . | . | . | . | . | . | L | . | . | . | . |
| 21-225_197F4_HC | . | . | . | . | . | . | . | . | . | . | . | . | . | . | . | . | . | . | . | . | . | . | . | L | . | . | . | . |
| 21-225_198B1_HC | . | . | . | . | . | . | . | . | . | . | . | . | . | . | . | . | . | . | . | . | . | . | . | . | . | . | . | . |
| 21-225_198E1_HC | . | . | . | . | . | . | . | . | . | . | . | . | . | . | . | . | . | . | . | . | . | . | . | . | . | . | . | . |
| 21-225_199C2_HC | . | . | . | . | . | . | . | . | . | . | . | . | . | . | . | . | . | . | . | . | . | . | . | . | . | . | . | . |
| 21-225_199C7_HC | . | . | . | . | . | . | . | . | . | . | . | . | . | . | . | . | . | . | . | . | . | . | . | . | . | . | . | . |
| 21-225_200F10_HC | . | . | . | . | . | . | . | . | . | . | . | . | . | . | . | . | . | . | . | . | . | . | . | . | . | . | . | . |

FIGURE 57

| Reference # | 141 | 142 | 143 | 144 | 145 | 146 | 147 | 148 | 149 |
|---|---|---|---|---|---|---|---|---|---|
| | H_FR4 | | | | | | | | |
| CONSENSUS | Q | G | T | T | V | T | V | S | S |
| 21-225_190A3_HC | . | . | . | . | . | . | . | . | . |
| 21-225_190A7_HC | . | . | . | . | . | . | . | . | . |
| 21-225_190B4_HC | . | . | . | . | . | . | . | . | . |
| 21-225_191A3_HC | . | . | . | . | . | . | . | . | . |
| 21-225_191D3_HC | . | . | . | . | . | . | . | . | . |
| 21-225_191F1_HC | . | . | . | . | . | . | . | . | . |
| 21-225_191H7_HC | . | . | . | . | . | . | . | . | . |
| 21-225_192B3_HC | . | . | . | . | . | . | . | . | . |
| 21-225_192G12_HC | . | . | . | . | . | . | . | . | . |
| 21-225_192H3_HC | . | . | . | . | . | . | . | . | . |
| 21-225_193A1_HC | . | . | . | . | . | . | . | . | . |
| 21-225_193B1_HC | . | . | . | . | . | . | . | . | . |
| 21-225_193C8_HC | . | . | . | . | . | . | . | . | . |
| 21-225_193D8_HC | . | . | . | . | . | . | . | . | . |
| 21-225_193F3_HC | . | . | . | . | . | . | . | . | . |
| 21-225_193G8_HC | H | . | . | . | . | . | . | . | . |
| 21-225_193H6_HC | . | . | . | . | . | . | . | . | . |
| 21-225_194E5_HC | . | . | . | . | . | . | . | . | . |
| 21-225_194E6_HC | . | . | . | . | . | . | . | . | . |
| 21-225_196H12_HC | . | . | . | . | . | . | . | . | . |
| 21-225_197F4_HC | . | . | . | . | . | . | . | . | . |
| 21-225_198B1_HC | R | . | . | . | . | . | . | . | . |
| 21-225_198E1_HC | . | . | . | . | . | . | . | . | . |
| 21-225_199C2_HC | . | . | . | . | . | . | . | . | . |
| 21-225_199C7_HC | . | . | . | . | . | . | . | . | . |
| 21-225_200F10_HC | . | . | . | . | . | . | . | . | . |

**FIGURE 57**

Table 64. Consensus 30- VH3|3-23/D7|7-27|RF1/JH6 (SEQ ID NO: 50281):

EVQLLES-GGGLVQPGGSLRLSCAASG-FTFSS-----YAMSWVRQAPGKGLEWVSVISGG---GSSTYYADSVKGRFTISRDNSKNTLYLQMNSLRAEDTAVYYCAKWRGNPT------------------DYGMDVWGQGTTVTVSS wherein:

S at position 33 can be substituted with N or T

Y at position 39 can be substituted with H

A at position 40 can be substituted with P or V

V at position 57 can be substituted with A or I

G at position 61 can be substituted with S

S at position 66 can be substituted with G or T

S at position 67 can be substituted with T

T at position 68 can be substituted with A

W at position 109 can be substituted with A

R at position 110 can be substituted with G

N at position 112 can be substituted with T

P at position 113 can be substituted with T

T at position 114 can be substituted with G

null (-) at position 115 can be substituted with S

null (-) at position 116 can be substituted with Y

null (-) at position 132 can be substituted with Y

D at position 133 can be substituted with Y

**FIGURE 57**

Y at position 134 can be substituted with N or S

Xaa Xaa Xaa Met Ser (SEQ ID NO: 50043)

Xaa Ile Ser Gly Xaa Gly Xaa Xaa Xaa Tyr Tyr Ala Asp Ser Val Lys Gly (SEQ ID NO: 50044)

Xaa Xaa Gly Xaa Xaa Xaa Xaa Xaa Xaa Xaa Xaa Gly Met Asp (SEQ ID NO: 50045)

Ser Tyr Ala Met Ser (SEQ ID NO: 50513)

Val Ile Ser Gly Gly Gly Ser Ser Thr Tyr Tyr Ala Asp Ser Val Lys Gly (SEQ ID NO: 50514)

Trp Arg Gly Asn Pro Thr Asp Tyr Gly Met Asp (SEQ ID NO: 50515)

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | E | V | Q | L | L | E | S | - | G | G | G | L | V | Q | P | G | G | S | L | R | L | S | C | A | A | S |
| 21-225_146A2_HC | . | . | . | . | . | . | . | . | . | . | . | . | . | . | . | . | . | . | . | . | . | . | . | . | . | . |
| 21-225_146A3_HC | . | . | . | . | . | . | . | . | . | . | . | . | . | . | . | . | . | . | . | . | . | . | . | . | . | . |
| 21-225_146B11_HC | . | . | . | . | . | . | . | . | . | . | . | . | . | . | . | . | . | . | . | . | . | . | . | . | . | . |
| 21-225_146C11_HC | . | . | . | . | . | . | . | . | . | . | . | . | . | . | . | . | . | . | . | . | . | . | . | . | . | . |
| 21-225_147B12_HC | . | . | . | . | . | . | . | . | . | . | . | . | . | . | . | . | . | . | . | . | . | . | . | . | . | . |
| 21-225_147E11_HC | . | . | . | . | . | . | . | . | . | . | . | . | . | . | . | . | . | . | . | . | . | . | . | . | . | . |
| 21-225_147E7_HC | . | . | . | . | . | . | . | . | . | . | . | . | . | . | . | . | . | . | . | . | . | . | . | . | . | . |
| 21-225_148C6_HC | . | . | . | . | . | . | . | . | . | . | . | . | . | . | . | . | . | . | . | . | . | . | . | . | . | . |
| 21-225_148G11_HC | . | . | . | . | . | . | . | . | . | . | . | . | . | . | . | . | . | . | . | . | . | . | . | . | . | . |
| 21-225_148G6_HC | . | . | . | . | . | . | . | . | . | . | . | . | . | . | . | . | . | . | . | . | . | . | . | . | . | . |
| 21-225_148H11_HC | . | . | . | . | . | . | . | . | . | . | . | . | . | . | . | . | . | . | . | . | . | . | . | . | . | . |
| 21-225_149C10_HC | . | . | . | . | . | . | . | . | . | . | . | . | . | . | . | . | . | . | . | . | [illegible] | . | . | . | . | . |
| 21-225_149C7_HC | . | . | . | . | . | . | . | . | . | . | . | . | . | . | . | . | . | . | . | . | . | . | . | . | . | . |
| 21-225_150F6_HC | . | . | . | . | . | . | . | . | . | . | . | . | . | . | . | . | . | . | . | . | . | . | . | . | . | . |
| 21-225_150H11_HC | . | . | . | . | . | . | . | . | . | . | . | . | . | . | . | . | . | . | . | . | . | . | . | . | . | . |
| 21-225_151B9_HC | . | . | . | . | . | . | . | . | . | . | . | . | . | . | . | . | . | . | . | . | . | . | . | . | . | . |
| 21-225_151H5_HC | . | . | . | . | . | . | . | . | . | . | . | . | . | . | . | . | . | . | . | . | . | . | . | . | . | . |
| 21-225_151H7_HC | . | . | . | . | . | . | . | . | . | . | . | . | . | . | . | . | . | . | . | . | . | . | . | . | . | . |
| 21-225_152D7_HC | . | . | . | . | . | . | . | . | . | . | . | . | . | . | . | . | . | . | . | . | . | . | . | . | . | . |
| 21-225_152G6_HC | . | . | . | . | . | . | . | . | . | . | . | . | . | . | . | . | . | . | . | . | . | . | . | . | . | . |
| 21-225_154C4_HC | . | . | . | . | . | . | . | . | . | . | . | . | [illegible] | . | . | . | . | . | . | . | . | . | . | . | . | . |
| 21-225_154E10_HC | . | . | . | . | . | . | . | . | . | . | . | . | . | . | . | . | . | . | . | . | . | . | . | . | . | . |
| 21-225_155C10_HC | . | . | . | . | . | . | . | . | . | . | . | . | . | . | . | . | . | . | . | . | . | . | . | . | . | . |
| 21-225_207C9_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | | H_CDR1 | | | | | | | | | | | | H_FR2 | | | |
| CONSENSUS | G | - | F | T | F | S | S | - | - | - | - | - | Y | A | M | S | W | V | R | Q | A | P | G | K | G | L |
| 21-225_146A2_HC | . | . | . | . | . | . | . | . | . | . | . | . | . | . | . | . | . | . | . | . | . | . | . | . | . | . |
| 21-225_146A3_HC | . | . | . | . | . | . | . | . | . | . | . | . | . | . | . | . | . | . | . | . | . | . | . | . | . | . |
| 21-225_146B11_HC | . | . | . | . | . | . | . | . | . | . | . | . | . | . | . | . | . | . | . | . | . | . | . | . | . | . |
| 21-225_146C11_HC | . | . | I | . | . | . | . | . | . | . | . | . | . | . | . | . | . | . | . | . | . | . | . | . | . | . |
| 21-225_147B12_HC | . | . | . | . | . | . | . | . | . | . | . | . | . | . | . | . | . | . | . | . | . | . | . | . | . | . |
| 21-225_147E11_HC | . | . | . | . | . | . | N | . | . | . | . | . | . | V | . | . | . | . | . | . | . | . | . | . | . | . |
| 21-225_147E7_HC | . | . | . | . | . | . | . | . | . | . | . | . | . | . | . | . | . | . | . | . | . | . | . | . | . | . |
| 21-225_148C6_HC | . | . | . | . | . | . | . | . | . | . | . | . | . | . | . | . | . | . | . | . | . | . | . | . | . | . |
| 21-225_148G11_HC | . | . | . | . | . | . | . | . | . | . | . | . | . | P | . | . | . | . | . | . | . | . | . | . | . | . |
| 21-225_148G6_HC | . | . | . | . | . | . | . | . | . | . | . | . | . | . | . | . | . | . | . | . | . | . | . | . | . | . |
| 21-225_148H11_HC | . | . | . | . | . | . | . | . | . | . | . | . | . | . | . | . | . | . | . | . | . | . | . | . | . | . |
| 21-225_149C10_HC | . | . | . | . | . | . | . | . | . | . | . | . | H | . | . | . | . | . | . | . | . | . | . | . | . | . |
| 21-225_149C7_HC | . | . | . | . | . | . | . | . | . | . | . | . | . | . | . | . | . | . | . | . | . | . | . | . | . | . |
| 21-225_150F6_HC | . | . | . | . | . | . | . | . | . | . | . | . | . | . | . | . | . | . | . | . | . | . | . | . | . | . |
| 21-225_150H11_HC | . | . | . | . | . | . | T | . | . | . | . | . | . | . | . | . | . | . | . | . | . | . | . | . | . | . |
| 21-225_151B9_HC | . | . | . | . | . | . | . | . | . | . | . | . | . | . | . | . | . | . | . | . | . | . | . | . | . | . |
| 21-225_151H5_HC | . | . | . | . | . | . | . | . | . | . | . | . | . | . | . | . | . | . | . | . | . | . | . | . | . | . |
| 21-225_151H7_HC | . | . | . | . | . | . | . | . | . | . | . | . | . | . | . | . | . | . | . | . | . | . | . | . | . | . |
| 21-225_152D7_HC | . | . | . | . | . | . | . | . | . | . | . | . | . | . | . | . | . | . | . | . | . | . | . | . | . | . |
| 21-225_152G6_HC | . | . | . | . | . | . | . | . | . | . | . | . | . | . | . | . | . | . | . | . | . | . | . | . | . | . |
| 21-225_154C4_HC | . | . | . | . | . | . | . | . | . | . | . | . | . | . | . | . | . | . | . | . | . | . | . | . | . | . |
| 21-225_154E10_HC | . | . | . | . | . | . | . | . | . | . | . | . | . | . | . | . | . | . | . | . | . | . | . | . | . | . |
| 21-225_155C10_HC | . | . | . | . | . | . | . | . | . | . | . | . | . | . | . | . | . | . | . | . | . | . | . | . | . | . |
| 21-225_207C9_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | S | V | I | S | G | G | - | - | - | G | S | S | T | Y | Y | A | D | S | V | K | G | R | F |
| 21-225_146A2_HC | . | . | . | . | . | . | . | . | . | . | . | . | . | . | . | . | . | . | . | . | . | . | . | . | . | . |
| 21-225_146A3_HC | . | . | . | . | . | . | . | . | . | . | . | . | . | T | T | . | . | . | . | . | . | . | . | . | . | . |
| 21-225_146B11_HC | . | . | . | . | . | . | . | . | . | . | . | . | . | . | . | . | . | . | . | . | . | . | . | . | . | . |
| 21-225_146C11_HC | . | . | . | . | . | . | . | . | . | . | . | . | . | . | . | . | . | . | . | . | . | . | . | . | . | . |
| 21-225_147B12_HC | . | . | . | . | . | . | . | . | . | . | . | . | . | . | . | . | . | . | . | . | . | . | . | . | . | . |
| 21-225_147E11_HC | . | . | . | . | . | . | . | . | . | . | . | . | . | . | . | . | . | . | . | . | . | . | . | . | . | . |
| 21-225_147E7_HC | . | . | . | . | . | . | . | . | . | . | . | . | . | . | . | . | . | . | . | . | . | . | . | . | . | . |
| 21-225_148C6_HC | . | . | . | . | . | . | . | . | . | . | . | . | . | . | . | . | . | . | . | . | . | . | . | . | . | . |
| 21-225_148G11_HC | . | . | . | . | . | . | . | . | . | . | . | . | . | . | . | A | . | . | . | . | . | . | . | . | . | . |
| 21-225_148G6_HC | . | . | . | . | . | . | . | . | . | . | . | . | . | . | . | . | . | . | . | . | . | . | . | . | . | . |
| 21-225_148H11_HC | . | . | . | . | . | . | . | . | . | . | . | . | . | . | . | . | . | . | . | . | . | . | . | . | . | . |
| 21-225_149C10_HC | . | . | . | . | . | . | . | . | . | . | . | . | . | . | . | . | . | . | . | . | . | . | . | . | . | . |
| 21-225_149C7_HC | . | . | . | . | . | . | . | . | . | . | . | . | . | . | . | . | . | . | . | . | . | . | . | . | . | . |
| 21-225_150F6_HC | . | . | . | . | . | . | . | . | . | . | . | . | . | . | . | . | . | . | . | . | . | . | . | . | . | . |
| 21-225_150H11_HC | . | . | . | . | I | . | . | . | . | . | . | . | . | . | . | . | . | . | . | . | . | . | . | . | . | . |
| 21-225_151B9_HC | . | . | . | . | . | . | . | . | . | . | . | . | . | . | . | . | . | . | . | . | . | . | . | . | . | . |
| 21-225_151H5_HC | . | . | . | . | . | . | . | . | . | . | . | . | . | . | . | . | . | . | . | . | . | . | . | . | . | . |
| 21-225_151H7_HC | . | . | . | . | . | . | . | . | . | . | . | . | . | . | . | . | . | . | . | . | . | . | . | . | . | . |
| 21-225_152D7_HC | . | . | . | . | . | . | . | . | . | . | . | . | . | . | . | . | . | . | . | . | . | . | . | . | . | . |
| 21-225_152G6_HC | . | . | . | . | . | . | . | . | . | . | . | . | . | . | . | . | . | . | . | . | . | . | . | . | . | . |
| 21-225_154C4_HC | . | . | . | . | . | . | . | . | . | . | . | . | . | . | . | . | . | . | . | . | . | . | . | . | . | . |
| 21-225_154E10_HC | . | . | . | . | . | . | . | . | . | . | . | . | . | . | . | . | . | . | . | . | . | . | . | . | . | . |
| 21-225_155C10_HC | . | . | . | . | . | . | . | . | . | . | . | . | . | . | . | . | . | . | . | . | . | . | . | . | . | . |
| 21-225_207C9_HC | . | . | . | . | A | . | . | . | S | . | . | . | . | G | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_146A2_HC | . | . | . | . | . | . | . | . | . | . | . | H | . | . | . | . | . | . | . | . | . | . | . | . | . | . |
| 21-225_146A3_HC | . | . | . | . | . | . | . | . | . | . | . | . | . | . | . | . | . | . | . | . | . | . | . | . | . | . |
| 21-225_146B11_HC | . | . | . | . | . | . | . | . | . | . | . | . | . | . | . | . | . | . | . | . | . | . | . | . | . | . |
| 21-225_146C11_HC | . | . | . | . | . | . | . | . | . | . | . | . | . | . | . | . | . | . | . | . | . | . | . | . | . | . |
| 21-225_147B12_HC | . | . | . | . | . | . | . | . | . | . | . | . | . | . | . | . | . | . | . | . | . | . | . | . | . | . |
| 21-225_147E11_HC | . | . | . | . | . | . | . | . | . | . | . | . | . | . | . | . | . | . | . | . | . | . | . | . | . | . |
| 21-225_147E7_HC | . | L | . | . | . | . | . | . | . | . | . | . | . | . | . | . | . | . | . | . | . | . | . | . | . | F |
| 21-225_148C6_HC | . | . | . | . | . | . | . | . | . | . | . | . | . | . | . | . | . | . | . | . | . | . | . | . | . | . |
| 21-225_148G11_HC | . | . | . | . | . | . | . | . | . | . | . | . | . | . | . | . | . | . | . | . | . | . | . | . | . | . |
| 21-225_148G6_HC | . | . | . | . | . | . | . | . | . | M | . | H | . | . | . | . | . | . | . | . | . | . | . | . | . | . |
| 21-225_148H11_HC | . | . | . | . | . | . | . | . | . | . | . | . | . | . | . | . | . | . | . | . | . | . | . | . | . | . |
| 21-225_149C10_HC | . | . | . | . | . | . | . | . | . | . | . | . | . | . | . | . | . | . | . | . | . | . | . | . | . | . |
| 21-225_149C7_HC | . | . | . | . | . | . | . | . | . | . | . | . | . | . | . | . | . | . | . | . | . | . | . | . | . | . |
| 21-225_150F6_HC | . | . | . | . | . | . | . | . | . | . | . | . | . | . | . | . | . | . | . | . | . | . | . | . | . | . |
| 21-225_150H11_HC | . | . | . | . | . | . | . | . | . | . | . | . | . | . | . | . | . | . | . | . | . | . | . | . | . | . |
| 21-225_151B9_HC | . | . | . | . | . | . | . | . | . | . | . | . | . | . | . | . | . | . | . | . | . | . | . | . | . | . |
| 21-225_151H5_HC | . | . | . | . | . | . | . | . | . | . | . | . | . | . | . | . | . | . | . | . | . | . | . | . | . | . |
| 21-225_151H7_HC | . | . | . | . | . | . | . | . | . | . | . | . | . | . | . | . | . | . | . | . | . | . | . | . | . | . |
| 21-225_152D7_HC | . | . | . | . | . | . | . | . | . | . | . | . | . | . | . | . | . | . | . | . | . | . | . | . | . | . |
| 21-225_152G6_HC | . | . | . | . | . | . | . | . | . | . | . | . | . | . | . | . | . | . | . | . | . | . | . | . | . | . |
| 21-225_154C4_HC | . | . | . | . | . | . | . | . | . | . | . | . | . | . | . | . | . | . | . | . | . | . | . | . | . | . |
| 21-225_154E10_HC | . | . | . | . | . | . | . | . | . | . | . | . | . | . | . | . | . | . | . | . | . | . | . | . | . | . |
| 21-225_155C10_HC | . | . | . | . | . | . | . | . | . | . | . | . | . | . | . | . | . | . | . | . | . | . | . | . | . | . |
| 21-225_207C9_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | K | W | R | G | N | P | T | - | - | - | - | - | - | - | - | - | - | - | - | - | - | - | - |
| 21-225_146A2_HC | . | . | . | . | . | . | . | . | . | . | . | . | . | . | . | . | . | . | . | . | . | . | . | . | . | . |
| 21-225_146A3_HC | . | . | . | . | . | . | . | . | . | . | . | . | . | . | . | . | . | . | . | . | . | . | . | . | . | . |
| 21-225_146B11_HC | . | . | . | . | . | . | . | . | . | . | . | . | . | . | . | . | . | . | . | . | . | . | . | . | . | . |
| 21-225_146C11_HC | . | . | . | . | . | . | . | . | . | . | . | . | . | . | . | . | . | . | . | . | . | . | . | . | . | . |
| 21-225_147B12_HC | . | . | . | . | . | . | . | . | . | . | . | . | . | . | . | . | . | . | . | . | . | . | . | . | . | . |
| 21-225_147E11_HC | . | . | . | . | . | . | . | . | . | . | . | . | . | . | . | . | . | . | . | . | . | . | . | . | . | . |
| 21-225_147E7_HC | . | . | . | . | . | . | . | . | . | . | . | . | . | . | . | . | . | . | . | . | . | . | . | . | . | . |
| 21-225_148C6_HC | . | . | . | . | . | . | . | . | . | . | . | . | . | . | . | . | . | . | . | . | . | . | . | . | . | . |
| 21-225_148G11_HC | . | . | . | . | . | . | . | . | . | . | . | . | . | . | . | . | . | . | . | . | . | . | . | . | . | . |
| 21-225_148G6_HC | . | . | . | . | . | . | . | . | . | . | . | . | . | . | . | . | . | . | . | . | . | . | . | . | . | . |
| 21-225_148H11_HC | . | . | . | . | . | . | . | . | . | . | . | . | . | . | . | . | . | . | . | . | . | . | . | . | . | . |
| 21-225_149C10_HC | . | . | . | . | . | . | . | . | . | . | . | . | . | . | . | . | . | . | . | . | . | . | . | . | . | . |
| 21-225_149C7_HC | . | . | . | . | . | . | . | . | . | . | . | . | . | . | . | . | . | . | . | . | . | . | . | . | . | . |
| 21-225_150F6_HC | . | . | . | . | . | . | . | . | . | . | . | . | . | . | . | . | . | . | . | . | . | . | . | . | . | . |
| 21-225_150H11_HC | . | . | . | . | . | . | . | . | . | . | . | . | . | . | . | . | . | . | . | . | . | . | . | . | . | . |
| 21-225_151B9_HC | . | . | . | . | . | . | . | . | . | . | . | . | . | . | . | . | . | . | . | . | . | . | . | . | . | . |
| 21-225_151H5_HC | . | . | . | . | . | . | . | . | . | . | . | . | . | . | . | . | . | . | . | . | . | . | . | . | . | . |
| 21-225_151H7_HC | . | . | . | . | . | . | . | . | . | . | . | . | . | . | . | . | . | . | . | . | . | . | . | . | . | . |
| 21-225_152D7_HC | . | . | . | . | . | . | . | . | . | . | . | . | . | . | . | . | . | . | . | . | . | . | . | . | . | . |
| 21-225_152G6_HC | . | . | . | . | . | . | . | . | . | . | . | . | . | . | . | . | . | . | . | . | . | . | . | . | . | . |
| 21-225_154C4_HC | . | . | . | . | . | . | . | . | . | . | . | . | . | . | . | . | . | . | . | . | . | . | . | . | . | . |
| 21-225_154E10_HC | . | . | . | . | . | . | . | . | . | . | . | . | . | . | . | . | . | . | . | . | . | . | . | . | . | . |
| 21-225_155C10_HC | . | . | . | . | . | . | . | . | . | . | . | . | . | . | . | . | . | . | . | . | . | . | . | . | . | . |
| 21-225_207C9_HC | . | . | . | . | A | G | . | T | T | G | S | Y | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | D | Y | G | M | D | V | W | G | Q | G | T | T | V | T | V | S | S |
| 21-225_146A2_HC | . | . | . | . | . | . | . | . | . | . | . | . | . | . | . | . | . | . | . |
| 21-225_146A3_HC | . | . | . | . | . | . | . | . | . | . | . | . | . | . | . | . | . | . | . |
| 21-225_146B11_HC | . | . | . | . | . | . | . | . | . | . | . | . | . | . | . | . | . | . | . |
| 21-225_146C11_HC | . | . | . | . | . | . | . | . | . | . | . | . | . | . | . | . | . | . | . |
| 21-225_147B12_HC | . | . | . | . | . | . | . | . | . | . | . | . | . | . | . | . | . | . | . |
| 21-225_147E11_HC | . | . | . | . | . | . | . | . | . | . | . | . | . | . | . | . | . | . | . |
| 21-225_147E7_HC | . | . | . | . | . | . | . | . | . | . | . | . | . | . | . | . | . | . | . |
| 21-225_148C6_HC | . | . | . | . | . | . | . | . | . | . | . | . | . | . | . | . | . | . | . |
| 21-225_148G11_HC | . | . | . | . | . | . | . | . | . | . | . | . | . | . | . | . | . | . | . |
| 21-225_148G6_HC | . | . | . | . | . | . | . | . | . | . | . | . | . | . | . | . | . | . | . |
| 21-225_148H11_HC | . | . | . | . | . | . | . | . | . | . | . | . | . | . | . | . | . | . | . |
| 21-225_149C10_HC | . | . | . | . | . | . | . | . | . | . | . | . | . | . | . | . | . | . | . |
| 21-225_149C7_HC | . | . | . | . | . | . | . | . | . | . | . | . | . | . | . | . | . | . | . |
| 21-225_150F6_HC | . | . | . | . | . | . | . | . | . | . | . | . | . | . | . | . | . | . | . |
| 21-225_150H11_HC | . | . | . | . | . | . | . | . | . | . | . | . | . | . | . | . | . | . | . |
| 21-225_151B9_HC | . | . | . | . | . | . | . | . | . | . | . | . | . | . | . | . | . | . | . |
| 21-225_151H5_HC | . | . | . | . | . | . | . | . | . | . | . | . | . | . | . | . | . | . | . |
| 21-225_151H7_HC | . | . | . | . | . | . | . | . | . | . | . | . | . | . | . | . | . | . | . |
| 21-225_152D7_HC | . | . | . | S | . | . | . | . | . | . | . | . | . | . | . | . | . | . | . |
| 21-225_152G6_HC | . | . | . | . | . | . | . | . | . | . | . | . | . | . | . | . | . | . | . |
| 21-225_154C4_HC | . | . | . | . | . | . | . | . | . | . | . | . | . | . | . | . | . | . | . |
| 21-225_154E10_HC | . | . | . | . | . | . | . | . | . | . | . | . | . | . | . | . | . | . | . |
| 21-225_155C10_HC | . | . | . | . | . | . | . | . | . | . | . | . | . | . | . | . | . | . | . |
| 21-225_207C9_HC | . | Y | Y | N | . | . | . | . | . | . | . | . | . | . | . | . | . | . | . |

**FIGURE 57**

Table 65. Consensus 31-VH3|3-33/D4|4-17|RF2/JH4 (SEQ ID NO: 50282):

QVQLVES-GGGVVQPGRSLRLSCAASG-FTFSS-----YGMHWVRQAPGKGLEWVAIIWYD---GSNKYYADSVKGRFTISRDNSKNTLYLQMNSLRAEDTAVYYCARDHYDFW------------------SGHFDYWGQGTLVTVSS wherein:

S at position 33 can be substituted with N or T

M at position 41 can be substituted with L

I at position 57 can be substituted with V or A

I at position 58 can be substituted with M

S at position 66 can be substituted with T

N at position 67 can be substituted with Y or S

A at position 71 can be substituted with G, T or V

D at position 109 can be substituted with E

H at position 110 can be substituted with R, Q, A, G, T or Y

Y at position 111 can be substituted with G, F or H

D at position 112 can be substituted with I or F

F at position 113 can be substituted with V or L

W at position 114 can be substituted with G or null (-)

S at position 133 can be substituted with A or null (-)

G at position 134 can be substituted with T or E

H at position 135 can be substituted with Y, W or F

F at position 136 can be substituted with L or S

**FIGURE 57**

D at position 137 can be substituted with A, C or G

Y at position 138 can be substituted with F or S

Xaa Tyr Gly Xaa His (SEQ ID NO: 50253)

Xaa Xaa Trp Tyr Asp Gly Xaa Xaa Lys Tyr Tyr Xaa Asp Ser Val Lys Gly (SEQ ID NO: 50254)

Xaa Xaa Xaa Xaa Xaa Xaa Xaa Xaa Xaa Xaa Xaa Xaa (SEQ ID NO: 50255)

Ser Tyr Gly Met His (SEQ ID NO: 50516)

Ile Ile Trp Tyr Asp Gly Ser Asn Lys Tyr Tyr Ala Asp Ser Val Lys Gly (SEQ ID NO: 50517)

Asp His Tyr Asp Phe Trp Ser Gly His Phe Asp Tyr (SEQ ID NO: 50518)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | E | S | - | G | G | G | V | V | Q | P | G | R | S | L | R | L | S | C | A | A | S |
| 21-225_147E10_HC | . | . | . | . | . | . | . | . | . | . | . | . | . | . | . | . | K | . | . | . | . | . | . | . | . | . |
| 21-225_148B2_HC | . | . | . | . | . | . | . | . | . | . | . | . | . | . | . | . | . | . | . | . | . | . | . | . | . | . |
| 21-225_148G10_HC | . | . | . | . | . | . | . | . | . | . | . | . | . | . | . | . | . | . | . | . | . | . | . | . | . | . |
| 21-225_149F1_HC | . | . | . | . | . | . | . | . | . | . | . | . | . | . | . | . | . | . | . | . | . | . | . | . | . | . |
| 21-225_150B11_HC | . | . | . | . | . | . | . | . | . | . | . | . | . | . | . | . | . | . | . | . | . | . | . | . | . | . |
| 21-225_151A10_HC | . | . | . | . | . | . | . | . | . | . | . | . | . | . | . | . | . | . | . | . | . | . | . | . | . | . |
| 21-225_151G5_HC | . | . | . | . | M | . | . | . | . | . | . | . | . | . | . | . | . | . | . | . | . | . | . | . | . | . |
| 21-225_155A5_HC | . | . | . | . | . | . | . | . | . | . | D | . | . | . | . | . | . | . | . | . | . | . | . | . | . | . |
| 21-225_170A1_HC | . | . | . | . | . | . | . | . | . | . | . | . | . | . | . | . | . | . | . | . | . | . | . | . | . | . |
| 21-225_176H4_HC | . | . | . | . | . | . | . | . | . | . | . | . | . | . | . | . | . | . | . | . | . | . | . | . | . | . |
| 21-225_181C10_HC | . | . | . | . | . | . | . | . | . | . | . | . | . | . | . | . | . | . | . | . | . | . | . | . | . | . |
| 21-225_181C2_HC | . | . | . | . | . | . | . | . | . | . | . | . | . | . | . | . | . | . | . | . | . | . | . | V | . | . |
| 21-225_186F7_HC | . | . | . | . | . | . | . | . | . | . | . | . | . | . | . | . | . | . | . | . | . | . | . | . | . | . |
| 21-225_189G2_HC | . | . | . | . | . | . | . | . | . | . | . | . | . | . | . | . | . | . | . | . | . | . | . | . | . | . |
| 21-225_226A5_HC | . | . | . | . | . | . | . | . | . | . | . | . | . | . | . | . | . | . | . | . | . | . | . | . | . | . |
| 21-225_22A1_HC | . | . | . | . | . | . | . | . | . | . | . | . | . | . | . | . | . | . | . | . | . | . | . | . | . | . |
| 21-225_28G3_HC | . | . | . | . | . | . | . | . | . | . | . | . | . | . | . | . | . | . | . | . | . | . | . | . | . | . |
| 21-225_46F2_HC | . | . | . | . | . | . | . | . | . | . | . | . | . | . | . | . | . | . | . | . | . | . | . | . | . | . |
| 21-225_57H3_HC | . | . | . | . | . | . | . | . | . | . | . | . | . | . | . | . | . | . | . | . | . | . | . | . | . | . |
| 21-225_62E6_HC | . | . | . | . | . | . | . | . | . | . | . | . | . | . | . | . | . | . | . | . | . | . | . | . | . | . |
| 21-225_64H9_HC | . | . | . | . | . | . | . | . | . | . | . | . | . | . | . | . | . | . | . | . | . | . | . | . | . | . |
| 21-225_65A6_HC | . | . | . | . | . | . | . | . | . | . | . | . | . | . | . | . | . | . | . | . | . | . | . | . | . | . |
| 21-225_67C3_HC | . | . | . | . | . | . | . | . | . | . | . | . | . | . | . | . | . | . | . | . | . | . | . | . | . | . |
| 21-225_70A5_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | G | M | H | W | V | R | Q | A | P | G | K | G | L |
| 21-225_147E10_HC | . | . | . | . | . | . | N | . | . | . | . | . | . | . | L | . | . | . | . | . | . | . | . | . | . | . |
| 21-225_148B2_HC | . | . | . | . | . | . | . | . | . | . | . | . | . | . | . | . | . | . | . | . | . | . | . | . | . | . |
| 21-225_148G10_HC | . | . | . | . | . | . | . | . | . | . | . | . | . | . | . | . | . | . | . | . | . | . | . | . | . | . |
| 21-225_149F1_HC | . | . | . | . | . | . | . | . | . | . | . | . | . | . | . | . | . | . | . | . | . | . | . | . | . | . |
| 21-225_150B11_HC | . | . | . | . | . | . | . | . | . | . | . | . | . | . | . | . | . | . | . | . | . | . | . | . | . | . |
| 21-225_151A10_HC | . | . | . | . | . | . | N | . | . | . | . | . | . | . | . | . | . | . | . | . | . | . | . | . | . | . |
| 21-225_151G5_HC | . | . | . | I | . | . | . | . | . | . | . | . | . | . | . | . | . | . | . | . | . | . | . | . | . | . |
| 21-225_155A5_HC | . | . | . | . | . | . | . | . | . | . | . | . | . | . | . | . | . | . | . | . | . | . | . | . | . | . |
| 21-225_170A1_HC | . | . | . | . | . | . | . | . | . | . | . | . | . | . | . | . | . | . | . | . | . | . | . | . | . | . |
| 21-225_176H4_HC | . | . | . | . | . | . | . | . | . | . | . | . | . | . | . | . | . | . | . | . | . | . | . | . | . | . |
| 21-225_181C10_HC | . | . | . | . | . | . | . | . | . | . | . | . | . | . | . | . | . | . | . | . | . | . | . | . | . | . |
| 21-225_181C2_HC | . | . | . | . | . | . | . | . | . | . | . | . | . | . | . | . | . | . | . | . | . | . | . | . | . | . |
| 21-225_186F7_HC | . | . | . | . | . | . | T | . | . | . | . | . | . | . | . | . | . | . | . | . | . | . | . | . | . | . |
| 21-225_189G2_HC | . | . | . | . | . | . | N | . | . | . | . | . | . | . | . | . | . | . | . | . | . | . | . | . | . | . |
| 21-225_226A5_HC | . | . | . | . | . | . | N | . | . | . | . | . | . | . | . | . | . | . | . | . | . | . | . | . | . | . |
| 21-225_22A1_HC | . | . | . | . | . | . | . | . | . | . | . | . | . | . | . | . | . | . | . | . | . | . | . | . | . | . |
| 21-225_28G3_HC | . | . | . | I | . | . | T | . | . | . | . | . | . | . | . | . | . | . | . | . | . | . | . | . | . | . |
| 21-225_46F2_HC | . | . | . | . | . | . | . | . | . | . | . | . | . | . | . | . | . | . | . | . | . | . | . | . | . | . |
| 21-225_57H3_HC | . | . | . | . | . | . | . | . | . | . | . | . | . | . | . | . | . | . | . | . | . | . | . | . | . | . |
| 21-225_62E6_HC | . | . | . | . | . | . | . | . | . | . | . | . | . | . | . | . | . | . | . | . | . | . | . | . | . | . |
| 21-225_64H9_HC | . | . | . | . | . | . | T | . | . | . | . | . | . | . | . | . | . | . | . | . | . | . | . | . | . | . |
| 21-225_65A6_HC | . | . | . | . | . | . | N | . | . | . | . | . | . | . | . | . | . | . | . | . | . | . | . | . | . | . |
| 21-225_67C3_HC | . | . | . | . | . | . | . | . | . | . | . | . | . | . | . | . | . | . | . | . | . | . | . | . | . | . |
| 21-225_70A5_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | A | I | I | W | Y | D | - | - | - | G | S | N | K | Y | Y | A | D | S | V | K | G | R | F |
| 21-225_147E10_HC | . | . | . | . | . | . | . | . | . | . | . | . | . | . | . | . | . | . | . | . | . | . | . | . | . | . |
| 21-225_148B2_HC | . | . | . | . | . | . | . | . | . | . | . | . | . | . | Y | . | . | . | . | . | . | . | . | . | . | . |
| 21-225_148G10_HC | . | . | . | . | . | . | . | . | . | . | . | . | . | . | . | . | . | . | . | . | . | . | . | . | . | . |
| 21-225_149F1_HC | . | . | . | . | . | . | . | . | . | . | . | . | . | . | Y | . | . | . | . | . | . | . | . | . | . | . |
| 21-225_150B11_HC | . | . | . | . | . | . | . | . | . | . | . | . | . | . | . | . | . | . | . | . | . | . | . | . | . | . |
| 21-225_151A10_HC | . | . | L | . | . | . | . | . | . | . | . | . | . | . | . | . | . | . | . | . | . | . | . | . | . | . |
| 21-225_151G5_HC | . | . | . | . | . | . | . | . | . | . | . | . | . | . | . | . | . | . | . | . | . | . | . | . | . | . |
| 21-225_155A5_HC | . | . | . | . | . | . | . | . | . | . | . | . | . | . | S | . | . | . | . | . | . | . | . | . | . | . |
| 21-225_170A1_HC | . | . | . | . | . | . | . | . | . | . | . | . | . | . | Y | . | . | . | . | . | . | . | . | . | . | . |
| 21-225_176H4_HC | . | . | . | . | . | . | . | . | . | . | . | . | . | . | Y | . | . | . | . | . | . | . | . | . | . | . |
| 21-225_181C10_HC | . | . | M | . | . | . | . | . | . | . | . | . | . | . | Y | . | . | . | . | . | . | . | . | . | . | . |
| 21-225_181C2_HC | . | . | . | T | . | . | . | . | . | . | . | . | . | . | Y | . | . | . | . | . | . | . | . | . | . | . |
| 21-225_186F7_HC | . | . | . | . | . | . | . | . | . | . | . | . | . | . | Y | . | . | . | . | . | . | . | . | . | . | . |
| 21-225_189G2_HC | . | . | . | . | . | . | . | . | . | . | . | . | . | . | Y | . | . | . | . | . | . | . | . | . | . | . |
| 21-225_226A5_HC | . | . | . | . | . | . | . | . | . | . | . | . | . | T | . | . | . | . | T | . | . | . | . | . | . | . |
| 21-225_22A1_HC | . | . | . | . | V | M | . | . | . | . | . | . | . | . | . | . | . | . | . | . | . | . | . | . | . | . |
| 21-225_28G3_HC | . | . | . | . | V | . | . | . | . | . | . | . | . | . | . | . | . | . | . | . | . | . | . | . | . | . |
| 21-225_46F2_HC | . | . | . | . | . | . | . | . | . | . | . | . | . | . | . | . | . | . | V | . | . | . | . | . | . | . |
| 21-225_57H3_HC | . | . | . | . | A | . | . | . | . | . | . | . | . | . | . | . | . | . | . | . | . | . | . | . | . | . |
| 21-225_62E6_HC | . | . | . | . | A | . | . | . | . | . | . | . | . | . | . | . | . | . | G | . | . | . | . | . | . | V |
| 21-225_64H9_HC | . | . | . | . | . | . | . | . | . | . | . | . | . | . | . | . | . | . | . | . | . | . | . | . | . | . |
| 21-225_65A6_HC | . | . | . | . | V | . | . | . | . | . | . | . | . | . | . | . | . | . | G | . | . | . | . | . | . | . |
| 21-225_67C3_HC | . | . | . | . | A | . | . | . | . | . | . | . | . | . | . | . | . | . | G | . | . | . | . | . | . | V |
| 21-225_70A5_HC | . | . | . | . | V | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_147E10_HC | . | . | . | . | . | . | . | . | . | . | . | . | . | . | . | . | . | . | . | . | . | . | . | . | . | . |
| 21-225_148B2_HC | . | . | . | . | . | . | . | . | . | . | . | . | . | . | . | . | . | . | S | . | . | . | . | . | . | . |
| 21-225_148G10_HC | . | . | . | . | . | . | . | . | . | . | . | . | . | . | . | . | . | . | . | . | . | . | . | . | . | . |
| 21-225_149F1_HC | . | . | . | . | . | . | . | . | . | . | . | . | . | . | . | . | . | . | S | . | . | . | . | . | . | . |
| 21-225_150B11_HC | . | . | . | . | . | . | . | . | . | . | . | . | . | . | . | . | . | . | . | . | . | . | . | . | . | . |
| 21-225_151A10_HC | . | . | . | . | . | . | . | . | . | . | . | . | . | . | . | . | . | . | . | . | . | . | . | . | . | . |
| 21-225_151G5_HC | . | . | . | . | . | . | . | . | . | . | . | . | . | . | . | . | . | . | . | . | . | . | . | . | . | . |
| 21-225_155A5_HC | . | . | . | . | . | . | . | . | . | . | . | . | . | H | . | . | . | . | . | . | . | . | . | . | . | . |
| 21-225_170A1_HC | . | . | . | . | . | . | . | . | . | . | . | . | . | . | . | . | . | . | . | . | . | . | . | . | . | . |
| 21-225_176H4_HC | . | . | . | . | . | . | . | . | . | . | . | . | . | . | . | S | . | . | . | . | . | . | . | . | . | . |
| 21-225_181C10_HC | . | . | . | . | . | . | . | . | . | . | . | . | . | . | . | . | . | . | . | . | . | . | . | . | . | . |
| 21-225_181C2_HC | . | . | . | . | . | . | . | . | . | . | . | . | . | . | . | . | . | . | . | . | . | . | . | . | . | . |
| 21-225_186F7_HC | . | . | . | . | . | . | . | . | . | . | . | . | . | . | . | . | . | . | . | . | . | . | . | . | . | . |
| 21-225_189G2_HC | . | . | . | . | . | . | . | . | . | . | . | . | . | . | . | . | . | . | . | . | . | . | . | . | . | . |
| 21-225_226A5_HC | . | . | . | . | . | . | . | . | . | . | . | . | . | . | . | . | . | . | . | . | . | . | . | . | . | . |
| 21-225_22A1_HC | . | . | . | . | . | . | . | . | . | . | . | . | . | . | . | . | . | . | . | . | . | . | . | . | . | . |
| 21-225_28G3_HC | . | . | . | . | . | . | . | . | . | . | . | . | . | . | . | . | . | . | . | . | . | . | . | . | . | . |
| 21-225_46F2_HC | . | . | . | . | . | . | . | . | . | . | . | . | . | . | . | . | . | . | . | . | . | . | . | . | . | . |
| 21-225_57H3_HC | . | . | . | . | . | . | . | . | . | . | . | . | . | . | . | . | . | . | . | . | . | . | . | . | . | . |
| 21-225_62E6_HC | . | . | . | . | . | . | . | . | . | S | . | H | . | . | . | . | . | . | . | . | . | . | . | . | . | . |
| 21-225_64H9_HC | . | . | . | . | . | . | . | . | . | . | . | . | . | . | . | . | . | . | . | . | . | . | . | . | . | . |
| 21-225_65A6_HC | . | . | . | . | . | . | . | . | . | . | . | . | . | . | . | . | . | . | . | . | . | . | . | . | . | . |
| 21-225_67C3_HC | . | . | . | . | . | . | . | . | . | S | . | H | . | . | . | . | . | . | . | . | . | . | . | . | . | . |
| 21-225_70A5_HC | . | . | . | . | . | . | . | . | . | S | . | . | . | . | . | . | . | . | S | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | D | | Y | D | F | W | - | - | - | - | - | - | - | - | - | - | - | - | - | - | - | - |
| 21-225_147E10_HC | . | . | . | . | . | H | . | . | . | . | . | . | . | . | . | . | . | . | . | . | . | . | . | . | . | . |
| 21-225_148B2_HC | . | . | . | . | . | H | F | . | . | . | . | . | . | . | . | . | . | . | . | . | . | . | . | . | . | . |
| 21-225_148G10_HC | . | . | . | . | . | R | . | . | . | . | . | . | . | . | . | . | . | . | . | . | . | . | . | . | . | . |
| 21-225_149F1_HC | . | . | . | . | . | H | F | . | . | . | . | . | . | . | . | . | . | . | . | . | . | . | . | . | . | . |
| 21-225_150B11_HC | . | . | . | . | . | R | . | . | . | . | . | . | . | . | . | . | . | . | . | . | . | . | . | . | . | . |
| 21-225_151A10_HC | . | . | . | . | . | H | . | . | . | . | . | . | . | . | . | . | . | . | . | . | . | . | . | . | . | . |
| 21-225_151G5_HC | . | . | . | . | . | R | . | . | . | . | . | . | . | . | . | . | . | . | . | . | . | . | . | . | . | . |
| 21-225_155A5_HC | . | . | . | . | . | R | . | . | . | . | . | . | . | . | . | . | . | . | . | . | . | . | . | . | . | . |
| 21-225_170A1_HC | . | . | . | . | . | A | H | . | . | . | . | . | . | . | . | . | . | . | . | . | . | . | . | . | . | . |
| 21-225_176H4_HC | . | . | . | . | . | A | H | . | . | . | . | . | . | . | . | . | . | . | . | . | . | . | . | . | . | . |
| 21-225_181C10_HC | . | . | . | . | . | H | . | . | . | . | . | . | . | . | . | . | . | . | . | . | . | . | . | . | . | . |
| 21-225_181C2_HC | . | . | . | . | . | H | . | . | . | . | . | . | . | . | . | . | . | . | . | . | . | . | . | . | . | . |
| 21-225_186F7_HC | . | . | . | . | . | H | . | . | . | . | . | . | . | . | . | . | . | . | . | . | . | . | . | . | . | . |
| 21-225_189G2_HC | . | . | . | . | . | R | F | . | . | . | . | . | . | . | . | . | . | . | . | . | . | . | . | . | . | . |
| 21-225_226A5_HC | . | . | . | . | E | G | H | . | . | . | . | . | . | . | . | . | . | . | . | . | . | . | . | . | . | . |
| 21-225_22A1_HC | . | . | . | . | . | G | G | F | L | - | . | . | . | . | . | . | . | . | . | . | . | . | . | . | . | . |
| 21-225_28G3_HC | . | . | . | . | . | R | . | . | . | . | . | . | . | . | . | . | . | . | . | . | . | . | . | . | . | . |
| 21-225_46F2_HC | . | . | . | . | . | R | . | . | . | . | . | . | . | . | . | . | . | . | . | . | . | . | . | . | . | . |
| 21-225_57H3_HC | . | . | . | . | . | Y | G | I | V | G | . | . | . | . | . | . | . | . | . | . | . | . | . | . | . | . |
| 21-225_62E6_HC | . | . | . | . | . | Q | G | I | V | G | . | . | . | . | . | . | . | . | . | . | . | . | . | . | . | . |
| 21-225_64H9_HC | . | . | . | . | . | T | . | . | . | . | . | . | . | . | . | . | . | . | . | . | . | . | . | . | . | . |
| 21-225_65A6_HC | . | . | . | . | . | Q | G | I | V | G | . | . | . | . | . | . | . | . | . | . | . | . | . | . | . | . |
| 21-225_67C3_HC | . | . | . | . | . | Q | G | I | V | G | . | . | . | . | . | . | . | . | . | . | . | . | . | . | . | . |
| 21-225_70A5_HC | . | . | . | . | . | Q | G | I | V | G | . | . | . | . | . | . | . | . | . | . | . | . | . | . | . | . |

# FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | S | G | H | F | D | Y | W | G | Q | G | T | L | V | T | V | S | S |
| 21-225_147E10_HC | . | . | . | . | . | . | . | . | . | . | . | . | . | . | . | . | . | . | . |
| 21-225_148B2_HC | . | . | . | . | . | . | . | . | . | . | . | . | . | . | . | . | . | . | . |
| 21-225_148G10_HC | . | . | . | . | . | . | . | . | . | . | . | . | . | . | . | . | . | . | . |
| 21-225_149F1_HC | . | . | . | . | . | . | . | . | . | . | . | . | . | . | . | . | . | . | . |
| 21-225_150B11_HC | . | . | . | . | . | . | . | . | . | . | . | . | . | . | . | . | . | . | . |
| 21-225_151A10_HC | . | . | . | . | . | . | . | . | . | . | . | . | . | . | . | . | . | . | . |
| 21-225_151G5_HC | . | . | . | . | . | . | . | . | . | . | . | . | . | . | . | . | . | . | . |
| 21-225_155A5_HC | . | . | . | . | . | . | . | . | . | . | . | . | . | . | . | . | . | . | . |
| 21-225_170A1_HC | . | . | . | . | Y | . | . | S | . | . | . | . | . | . | . | . | . | . | . |
| 21-225_176H4_HC | . | . | . | . | Y | . | A | . | . | . | . | . | A | . | . | . | . | . | . |
| 21-225_181C10_HC | . | . | . | . | . | . | . | . | . | . | . | . | . | . | . | . | . | . | . |
| 21-225_181C2_HC | . | . | . | . | . | . | . | F | . | . | . | . | . | . | . | . | . | . | . |
| 21-225_186F7_HC | . | . | . | . | . | . | . | . | . | . | . | . | . | . | . | . | . | . | . |
| 21-225_189G2_HC | . | . | . | . | Y | S | . | . | . | . | . | . | . | . | . | . | . | . | . |
| 21-225_226A5_HC | . | . | . | . | F | . | C | . | . | . | . | . | . | . | . | . | . | . | . |
| 21-225_22A1_HC | . | . | - | E | W | L | . | . | . | . | . | . | . | . | . | . | . | . | . |
| 21-225_28G3_HC | . | . | . | . | Y | . | . | . | . | . | . | . | . | . | . | . | . | . | . |
| 21-225_46F2_HC | . | . | . | . | Y | . | . | . | . | . | . | . | . | . | . | . | . | . | . |
| 21-225_57H3_HC | . | . | A | T | Y | . | . | . | . | . | . | . | . | . | . | . | . | . | . |
| 21-225_62E6_HC | . | . | A | T | W | . | . | . | . | . | . | . | . | . | . | . | . | . | . |
| 21-225_64H9_HC | . | . | . | . | Y | L | G | . | . | . | . | . | . | . | . | . | . | . | . |
| 21-225_65A6_HC | . | . | A | T | W | . | . | . | . | . | . | . | . | . | . | . | . | . | . |
| 21-225_67C3_HC | . | . | A | T | W | . | . | . | . | . | . | . | . | . | . | . | . | . | . |
| 21-225_70A5_HC | . | . | A | T | W | . | . | . | . | . | . | . | . | . | . | . | . | . | . |

**FIGURE 57**

Table 66. Consensus 32 -VH4|4-34/D4|4-17|RF2/JH4 (SEQ ID NO: 50283):

QVQLQQW-GAGLLKPSETLSLTCAVYG-GSFSG-----YYWSWIRQPPGKGLEWIGEINH----SGRTNYNPSLKSRVTISVDTSKNQFSLKLSSVTAADTAVYYCARDYGG---------------------LDYWGQGTLVTVSS wherein:

G at position 33 can be substituted with V, P, A, D or Y

Y at position 39 can be substituted with C, S or P

Y at position 40 can be substituted with F

I at position 58 can be substituted with S or V

H at position 60 can be substituted with I or Q

R at position 67 can be substituted with S

T at position 68 can be substituted with A or S

N at position 69 can be substituted with T

Y at position 70 can be substituted with F

Xaa Xaa Xaa Trp Ser (SEQ ID NO: 50233)

Glu Xaa Asn Xaa Ser Gly Xaa Xaa Xaa Xaa Asn Pro Ser Leu Lys Ser (SEQ ID NO: 50234)

Asp Tyr Gly Gly Leu Asp Tyr (SEQ ID NO: 50235)

Gly Tyr Tyr Trp Ser (SEQ ID NO: 50519)

Glu Ile Asn His Ser Gly Arg Thr Asn Tyr Asn Pro Ser Leu Lys Ser (SEQ ID NO: 50520)

Asp Tyr Gly Gly Leu Asp Tyr (SEQ ID NO: 50521)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | Q | Q | W | - | G | A | G | L | L | K | P | S | E | T | L | S | L | T | C | A | V | Y |
| 21-225_147G8_HC | . | . | . | . | . | . | . | . | . | . | . | . | . | . | . | . | . | . | . | . | . | . | . | G | . | . |
| 21-225_192F8_HC | . | . | . | . | . | . | . | . | . | . | . | . | . | . | . | . | . | . | . | . | . | . | . | . | . | . |
| 21-225_193E6_HC | . | . | . | . | . | . | . | . | . | . | . | . | . | . | . | . | . | . | . | . | . | . | . | . | . | F |
| 21-225_195B1_HC | . | . | . | . | . | . | . | . | . | . | . | . | . | . | . | . | . | . | . | . | . | . | . | . | . | S |
| 21-225_200A1_HC | . | . | . | . | . | . | . | . | . | . | . | . | . | . | . | . | . | . | . | . | . | . | . | . | . | . |
| 21-225_201B2_HC | . | . | . | . | . | . | . | . | . | . | . | . | . | . | . | . | . | . | . | . | . | . | . | . | . | F |
| 21-225_201E1_HC | . | . | . | . | . | . | . | . | . | . | . | . | . | . | . | . | . | . | . | . | . | . | . | . | . | F |
| 21-225_202D11_HC | . | . | . | . | . | . | R | . | . | . | . | . | . | . | . | . | . | . | . | . | . | . | . | . | A | . |
| 21-225_202D9_HC | . | . | . | . | . | . | . | . | . | . | . | . | . | . | . | . | . | . | . | . | . | . | . | . | . | . |
| 21-225_205G7_HC | . | . | . | . | . | . | . | . | . | . | . | . | . | . | . | . | . | . | . | . | . | . | . | . | . | . |
| 21-225_208A9_HC | . | . | . | . | . | . | . | . | . | . | . | . | . | . | . | . | . | P | . | . | . | . | . | . | . | . |
| 21-225_208B5_HC | . | . | . | . | . | . | . | . | . | . | . | . | . | . | . | . | . | . | . | . | . | . | . | . | . | F |
| 21-225_74C7_HC | . | . | . | . | . | . | . | . | . | . | . | . | . | . | . | . | . | . | . | . | . | . | . | . | . | . |
| 21-225_74D2_HC | . | . | . | . | . | . | . | . | . | . | . | . | . | . | . | . | . | . | . | . | . | . | . | . | . | . |
| 21-225_75G3_HC | . | . | . | . | . | . | . | . | . | . | . | . | . | . | . | . | . | . | . | . | . | . | . | . | . | . |
| 21-225_75G9_HC | . | . | . | . | . | . | . | . | . | . | . | . | . | . | . | . | . | . | . | . | . | . | . | . | . | . |
| 21-225_76A7_HC | . | . | . | . | . | . | . | . | . | . | . | . | . | . | . | . | . | . | . | . | . | . | . | . | . | . |
| 21-225_78E7_HC | . | . | . | . | . | . | . | . | . | . | . | . | . | . | . | . | . | . | . | . | . | . | . | . | . | . |
| 21-225_80A6_HC | . | . | . | . | . | . | . | . | . | . | . | . | . | . | . | . | . | . | . | . | . | . | . | . | . | . |
| 21-225_80B10_HC | . | . | . | . | . | . | . | . | . | . | . | . | . | . | . | . | . | . | . | . | . | . | . | . | . | . |
| 21-225_83B6_HC | . | . | . | . | . | . | . | . | . | . | . | . | . | . | . | . | . | . | . | . | . | . | . | . | . | . |
| 21-225_83C10_HC | . | . | . | . | . | . | . | . | . | . | . | . | . | . | . | . | . | . | . | . | . | . | . | T | . | . |
| 21-225_85G6_HC | . | . | . | . | . | . | . | . | . | . | . | . | . | . | . | . | . | . | . | . | . | . | . | . | . | . |
| 21-225_93E9_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | G | S | F | S | G | - | - | - | - | - | | Y | W | S | W | I | R | Q | P | P | G | K | G | L |
| 21-225_147G8_HC | . | . | . | . | . | . | A | . | . | . | . | . | Y | . | . | . | . | . | . | . | . | . | . | . | . | . |
| 21-225_192F8_HC | . | . | . | . | . | . | . | . | . | . | . | . | C | . | . | . | . | . | . | . | . | . | . | . | . | . |
| 21-225_193E6_HC | . | . | . | . | . | . | . | . | . | . | . | . | P | . | . | . | . | . | . | . | . | . | . | . | . | . |
| 21-225_195B1_HC | . | . | . | . | . | R | Y | . | . | . | . | . | Y | F | . | . | . | . | . | . | S | . | . | . | . | . |
| 21-225_200A1_HC | . | . | . | . | . | . | V | . | . | . | . | . | Y | . | . | . | . | . | . | . | . | . | . | . | . | . |
| 21-225_201B2_HC | . | . | . | . | I | . | V | . | . | . | . | . | Y | . | . | T | . | . | . | . | . | . | . | . | . | . |
| 21-225_201E1_HC | D | . | . | . | . | . | P | . | . | . | . | . | Y | . | . | . | . | . | . | . | . | . | . | . | . | . |
| 21-225_202D11_HC | . | . | . | . | . | . | . | . | . | . | . | . | P | . | . | . | . | . | . | . | . | . | . | . | . | . |
| 21-225_202D9_HC | D | . | . | . | . | . | P | . | . | . | . | . | Y | . | . | . | . | . | . | . | . | . | . | . | . | . |
| 21-225_205G7_HC | . | . | . | . | . | . | V | . | . | . | . | . | Y | . | . | . | . | . | . | . | . | . | . | . | . | . |
| 21-225_208A9_HC | . | . | . | . | . | . | V | . | . | . | . | . | S | . | . | . | . | . | . | . | . | . | . | . | . | . |
| 21-225_208B5_HC | . | . | . | . | I | . | V | . | . | . | . | . | Y | . | . | T | . | . | . | . | . | . | . | . | . | . |
| 21-225_74C7_HC | . | . | . | . | L | . | . | . | . | . | . | . | S | . | . | . | . | . | . | . | . | . | . | . | . | . |
| 21-225_74D2_HC | . | . | . | . | . | . | . | . | . | . | . | . | C | . | . | . | . | . | . | . | . | . | . | . | . | . |
| 21-225_75G3_HC | . | . | . | . | L | . | . | . | . | . | . | . | S | . | . | . | . | . | . | . | . | . | . | . | . | . |
| 21-225_75G9_HC | . | . | . | . | . | . | . | . | . | . | . | . | C | . | . | . | . | . | . | . | . | . | . | . | . | . |
| 21-225_76A7_HC | . | . | . | . | . | . | . | . | . | . | . | . | C | . | . | . | . | . | . | . | . | . | . | . | . | . |
| 21-225_78E7_HC | . | . | . | . | L | . | . | . | . | . | . | . | S | . | . | . | . | . | . | . | . | . | . | . | . | . |
| 21-225_80A6_HC | . | . | . | . | . | . | . | . | . | . | . | . | C | . | . | . | . | . | . | . | . | . | . | . | . | . |
| 21-225_80B10_HC | . | . | . | . | . | . | . | . | . | . | . | . | C | . | . | . | . | . | . | . | . | . | . | . | . | . |
| 21-225_83B6_HC | . | . | . | . | . | . | . | . | . | . | . | . | C | . | . | . | . | . | . | . | . | . | . | . | . | . |
| 21-225_83C10_HC | . | . | . | . | . | . | . | . | . | . | . | . | Y | . | . | . | . | . | . | . | . | . | . | . | . | . |
| 21-225_85G6_HC | . | . | . | . | . | . | D | . | . | . | . | . | C | . | . | . | . | . | . | . | . | . | . | . | . | . |
| 21-225_93E9_HC | . | . | . | . | . | . | . | . | . | . | . | . | C | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | I | G | E | I | N | H | - | - | - | - | S | G | | T | N | Y | N | P | S | L | K | S | R | V |
| 21-225_147G8_HC | . | . | . | . | . | . | . | . | . | . | . | . | . | . | S | . | . | . | K | . | . | . | . | . | . | . |
| 21-225_192F8_HC | . | . | . | . | . | . | . | Q | . | . | . | . | . | . | R | S | . | . | . | . | . | . | . | . | . | . |
| 21-225_193E6_HC | . | . | . | . | . | S | . | . | . | . | . | . | . | . | R | . | . | . | . | . | . | . | . | . | . | . |
| 21-225_195B1_HC | . | . | F | . | . | . | . | . | . | . | . | . | . | . | R | . | . | . | . | . | . | . | . | . | . | . |
| 21-225_200A1_HC | . | . | . | . | . | . | . | . | . | . | . | . | . | . | S | . | . | . | . | . | . | . | . | . | . | . |
| 21-225_201B2_HC | . | . | . | . | . | . | . | . | . | . | . | . | . | . | S | . | . | . | . | . | . | . | . | . | . | . |
| 21-225_201E1_HC | . | . | . | . | . | V | . | . | . | . | . | . | . | . | S | . | . | . | . | . | . | . | . | . | . | . |
| 21-225_202D11_HC | . | . | . | . | . | . | . | . | . | . | . | . | . | . | S | . | . | . | . | . | . | . | . | . | . | . |
| 21-225_202D9_HC | . | . | . | . | . | . | . | . | . | . | . | . | . | . | S | . | . | . | . | . | . | . | . | . | . | . |
| 21-225_205G7_HC | . | . | . | . | . | S | . | Q | . | . | . | . | . | . | R | . | T | . | . | . | . | . | . | . | . | . |
| 21-225_208A9_HC | . | . | . | . | . | . | . | . | . | . | . | . | . | . | R | A | . | . | . | . | . | . | . | . | . | . |
| 21-225_208B5_HC | . | . | . | . | . | . | . | . | . | . | . | . | . | . | S | . | . | . | . | . | . | . | . | . | . | . |
| 21-225_74C7_HC | . | . | . | . | . | . | . | L | . | . | . | . | . | . | S | . | . | . | . | . | . | . | . | . | . | . |
| 21-225_74D2_HC | . | . | . | . | . | . | . | . | . | . | . | . | . | . | R | . | . | . | . | . | . | . | . | . | . | . |
| 21-225_75G3_HC | . | . | . | . | . | . | . | L | . | . | . | . | . | . | S | . | . | . | . | . | . | . | . | . | . | . |
| 21-225_75G9_HC | . | . | . | . | . | . | . | . | . | . | . | . | . | . | R | . | . | . | . | . | . | . | . | . | . | . |
| 21-225_76A7_HC | . | . | . | . | . | . | . | . | . | . | . | . | . | . | R | . | . | . | . | . | . | . | . | . | . | . |
| 21-225_78E7_HC | . | . | . | . | . | . | . | L | . | . | . | . | . | . | S | . | . | . | . | . | . | . | . | . | . | . |
| 21-225_80A6_HC | . | . | . | . | . | . | . | . | . | . | . | . | . | . | R | . | . | . | . | . | . | . | . | . | . | . |
| 21-225_80B10_HC | . | . | . | . | . | . | . | . | . | . | . | . | . | . | S | . | . | . | . | . | . | . | . | . | . | . |
| 21-225_83B6_HC | . | . | . | . | . | . | . | . | . | . | . | . | . | . | R | . | . | . | . | . | . | . | . | . | . | . |
| 21-225_83C10_HC | . | . | . | . | . | . | . | . | . | . | . | . | . | . | S | . | . | F | . | . | . | . | . | . | . | . |
| 21-225_85G6_HC | . | . | . | . | . | . | . | . | . | . | . | . | . | . | R | . | . | . | . | . | . | . | . | . | . | . |
| 21-225_93E9_HC | . | . | . | . | . | . | . | . | . | . | . | . | . | . | R | . | . | . | . | . | . | . | . | . | . | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | V | D | T | S | K | N | Q | F | S | L | K | L | S | S | V | T | A | A | D | T | A | V | Y |
| 21-225_147G8_HC | . | . | . | . | . | . | . | . | . | . | . | . | . | . | . | . | . | . | . | . | . | . | . | . | . | . |
| 21-225_192F8_HC | . | . | . | . | . | . | . | T | . | . | . | . | . | . | . | R | . | . | . | . | . | . | . | . | . | . |
| 21-225_193E6_HC | . | . | . | . | . | . | . | . | . | . | . | . | . | R | . | . | . | . | . | . | . | . | . | . | . | . |
| 21-225_195B1_HC | . | . | . | . | . | . | . | . | . | . | . | . | . | . | . | R | . | . | . | . | . | . | . | . | . | . |
| 21-225_200A1_HC | . | . | . | L | . | . | . | . | . | . | . | . | . | . | . | . | . | . | . | . | . | . | . | . | . | . |
| 21-225_201B2_HC | . | . | . | . | . | . | . | . | . | . | . | . | . | . | . | . | . | . | . | . | . | . | . | . | . | . |
| 21-225_201E1_HC | . | . | . | . | . | . | . | . | . | . | . | . | . | . | . | . | . | . | . | . | . | . | . | . | . | . |
| 21-225_202D11_HC | . | . | . | . | . | . | . | . | . | . | . | . | . | . | . | . | . | . | . | . | . | . | . | . | . | . |
| 21-225_202D9_HC | . | . | . | . | . | . | . | . | . | . | . | . | . | . | . | . | . | . | . | . | . | . | . | . | . | . |
| 21-225_205G7_HC | . | . | . | . | . | . | . | . | . | . | . | . | . | N | . | I | . | . | . | . | . | . | . | . | . | . |
| 21-225_208A9_HC | . | . | . | I | . | . | . | . | . | . | . | . | . | . | . | . | . | . | . | . | . | . | . | . | . | . |
| 21-225_208B5_HC | . | . | . | . | . | . | . | . | . | . | . | . | . | . | . | . | . | . | . | . | . | . | . | . | . | . |
| 21-225_74C7_HC | . | . | . | . | . | . | . | . | S | . | . | . | . | T | . | R | . | . | . | . | . | . | . | . | . | . |
| 21-225_74D2_HC | . | . | . | . | . | . | . | E | . | . | . | . | . | . | . | . | . | . | . | . | . | . | . | . | . | . |
| 21-225_75G3_HC | . | . | . | . | . | . | . | . | S | . | . | . | . | T | . | R | . | . | . | . | . | . | . | . | . | . |
| 21-225_75G9_HC | . | . | . | . | . | . | . | E | . | . | . | . | . | . | . | . | . | . | . | . | . | . | . | . | . | . |
| 21-225_76A7_HC | . | . | . | . | . | . | . | E | . | . | . | . | . | . | . | . | . | . | . | . | . | . | . | . | . | . |
| 21-225_78E7_HC | . | . | . | . | . | . | . | . | S | . | . | . | . | T | . | R | . | . | . | . | . | . | . | . | . | . |
| 21-225_80A6_HC | . | . | . | . | . | . | . | E | K | . | . | . | . | . | . | . | . | . | . | . | . | . | . | . | . | . |
| 21-225_80B10_HC | . | . | . | . | . | . | . | . | . | . | . | . | . | . | . | . | . | . | . | . | . | . | . | . | . | . |
| 21-225_83B6_HC | . | . | . | . | . | . | . | E | . | . | . | . | . | . | . | . | . | . | . | . | . | . | . | . | . | . |
| 21-225_83C10_HC | . | . | . | . | . | . | . | . | . | . | . | . | . | . | . | . | . | . | . | . | . | . | . | . | . | F |
| 21-225_85G6_HC | . | . | . | . | . | . | . | E | . | . | . | . | . | . | . | . | . | . | . | . | . | . | . | . | . | . |
| 21-225_93E9_HC | . | . | . | . | . | . | . | E | . | K | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | D | Y | G | G | - | - | - | - | - | - | - | - | - | - | - | - | - | - | - | - | - | - |
| 21-225_147G8_HC | . | . | . | . | . | . | . | V | . | . | . | . | . | . | . | . | . | . | . | . | . | . | . | . | . | . |
| 21-225_192F8_HC | . | . | . | . | . | . | . | V | . | . | . | . | . | . | . | . | . | . | . | . | . | . | . | . | . | . |
| 21-225_193E6_HC | . | . | . | . | . | . | . | . | . | . | . | . | . | . | . | . | . | . | . | . | . | . | . | . | . | . |
| 21-225_195B1_HC | . | . | . | . | . | . | . | A | . | . | . | . | . | . | . | . | . | . | . | . | . | . | . | . | . | . |
| 21-225_200A1_HC | . | . | . | . | . | . | . | V | . | . | . | . | . | . | . | . | . | . | . | . | . | . | . | . | . | . |
| 21-225_201B2_HC | . | . | . | . | . | . | . | V | . | . | . | . | . | . | . | . | . | . | . | . | . | . | . | . | . | . |
| 21-225_201E1_HC | . | . | . | . | . | . | . | . | . | . | . | . | . | . | . | . | . | . | . | . | . | . | . | . | . | . |
| 21-225_202D11_HC | . | . | . | . | . | . | . | V | . | . | . | . | . | . | . | . | . | . | . | . | . | . | . | . | . | . |
| 21-225_202D9_HC | . | . | . | . | . | . | . | . | . | . | . | . | . | . | . | . | . | . | . | . | . | . | . | . | . | . |
| 21-225_205G7_HC | . | . | . | . | . | . | . | V | . | . | . | . | . | . | . | . | . | . | . | . | . | . | . | . | . | . |
| 21-225_208A9_HC | . | . | . | . | . | . | . | V | . | . | . | . | . | . | . | . | . | . | . | . | . | . | . | . | . | . |
| 21-225_208B5_HC | . | . | . | . | . | . | . | V | . | . | . | . | . | . | . | . | . | . | . | . | . | . | . | . | . | . |
| 21-225_74C7_HC | . | . | . | . | . | . | . | . | . | . | . | . | . | . | . | . | . | . | . | . | . | . | . | . | . | . |
| 21-225_74D2_HC | . | . | . | . | . | . | . | . | . | . | . | . | . | . | . | . | . | . | . | . | . | . | . | . | . | . |
| 21-225_75G3_HC | . | . | . | . | . | . | . | . | . | . | . | . | . | . | . | . | . | . | . | . | . | . | . | . | . | . |
| 21-225_75G9_HC | . | . | . | . | . | . | . | . | . | . | . | . | . | . | . | . | . | . | . | . | . | . | . | . | . | . |
| 21-225_76A7_HC | . | . | . | . | . | . | . | . | . | . | . | . | . | . | . | . | . | . | . | . | . | . | . | . | . | . |
| 21-225_78E7_HC | . | . | . | . | . | . | . | . | . | . | . | . | . | . | . | . | . | . | . | . | . | . | . | . | . | . |
| 21-225_80A6_HC | . | . | . | . | . | . | . | . | . | . | . | . | . | . | . | . | . | . | . | . | . | . | . | . | . | . |
| 21-225_80B10_HC | . | . | . | . | . | . | . | . | . | . | . | . | . | . | . | . | . | . | . | . | . | . | . | . | . | . |
| 21-225_83B6_HC | . | . | . | . | . | . | . | . | . | . | . | . | . | . | . | . | . | . | . | . | . | . | . | . | . | . |
| 21-225_83C10_HC | . | . | . | . | . | . | . | . | . | . | . | . | . | . | . | . | . | . | . | . | . | . | . | . | . | . |
| 21-225_85G6_HC | . | . | . | . | . | . | . | . | . | . | . | . | . | . | . | . | . | . | . | . | . | . | . | . | . | . |
| 21-225_93E9_HC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | - | L | D | Y | W | G | Q | G | T | L | V | T | V | S | S |
| 21-225_147G8_HC | . | . | . | . | . | F | . | . | . | . | . | . | . | . | . | . | . | . | . |
| 21-225_192F8_HC | . | . | . | . | . | F | . | . | . | . | . | . | . | . | . | . | . | . | . |
| 21-225_193E6_HC | . | . | . | . | . | . | . | . | . | . | . | . | . | . | . | . | . | . | . |
| 21-225_195B1_HC | . | . | . | . | . | F | . | I | . | . | . | . | . | . | . | . | . | . | . |
| 21-225_200A1_HC | . | . | . | . | . | F | . | . | . | . | . | . | . | . | . | . | . | . | . |
| 21-225_201B2_HC | . | . | . | . | . | F | . | . | . | . | . | . | . | . | . | . | . | . | . |
| 21-225_201E1_HC | . | . | . | . | . | . | . | . | . | . | . | . | A | . | . | . | . | . | . |
| 21-225_202D11_HC | . | . | . | . | . | . | . | . | . | . | . | . | . | . | . | . | . | . | . |
| 21-225_202D9_HC | . | . | . | . | . | . | . | . | . | . | . | . | . | . | . | . | . | . | . |
| 21-225_205G7_HC | . | . | . | . | . | F | . | . | . | . | . | . | . | . | . | . | . | . | . |
| 21-225_208A9_HC | . | . | . | . | . | . | . | . | . | . | . | . | . | . | . | . | . | . | . |
| 21-225_208B5_HC | . | . | . | . | . | F | . | . | . | . | . | . | . | . | . | . | . | . | . |
| 21-225_74C7_HC | . | . | . | . | . | . | . | . | . | . | . | . | . | . | . | . | . | . | . |
| 21-225_74D2_HC | . | . | . | . | . | . | . | . | . | . | . | . | . | . | . | . | . | . | . |
| 21-225_75G3_HC | . | . | . | . | . | . | . | . | . | . | . | . | . | . | . | . | . | . | . |
| 21-225_75G9_HC | . | . | . | . | . | . | . | . | . | . | . | . | . | . | . | . | . | . | . |
| 21-225_76A7_HC | . | . | . | . | . | . | . | . | . | . | . | . | A | . | . | . | . | . | . |
| 21-225_78E7_HC | . | . | . | . | . | . | . | . | . | . | . | . | . | . | . | . | . | . | . |
| 21-225_80A6_HC | . | . | . | . | . | . | . | . | . | . | . | . | . | . | . | . | . | . | . |
| 21-225_80B10_HC | . | . | . | . | . | . | . | . | . | . | . | . | . | . | . | . | . | . | . |
| 21-225_83B6_HC | . | . | . | . | . | . | . | . | . | . | . | . | . | . | . | . | . | . | . |
| 21-225_83C10_HC | . | . | . | . | . | . | . | . | . | . | . | . | . | . | . | . | . | . | . |
| 21-225_85G6_HC | . | . | . | . | . | . | . | . | . | . | . | . | . | . | . | . | . | . | . |
| 21-225_93E9_HC | . | . | . | . | . | . | . | . | . | . | . | . | . | . | . | . | . | . | . |

**FIGURE 57**

Table 67. Consensus 33-VH4|4-39/D1|1-26|RF3/JH4 (SEQ ID NO: 50284):

QLQLQES-GPGLVKPSETLSLTCTVSG-GSISRS---SYYWGWIRQPPGKGLEWIGNIYY----SGSTYYNPSLKSRVTISVDTSKNQFSLKLSSVTAADTAVYYCARHSSSW--------------------SLDYWGQGTLVTVSS wherein:

R in position 33 can be substituted with G

S in position 38 can be substituted with N

Y in position 39 can be substituted with S

N in position 57 can be substituted with S

S in position 67 can be substituted with Y, A, T or I

T in position 68 can be substituted with A, P or S

Y in position 69 can be substituted with Q, S, A or N

Y in position 70 can be substituted with C or H

N in position 71 can be substituted with I or T

H in position 109 can be substituted with L

S in position 111 can be substituted with T or G

L in position 136 can be substituted with F, I or V

Y in position 138 can be substituted with N, C, D or F

Xaa Ser Xaa Xaa Tyr Trp Gly (SEQ ID NO: 50046)

Xaa Ile Tyr Tyr Ser Gly Xaa Xaa Xaa Xaa Xaa Pro Ser Leu Lys Ser (SEQ ID NO: 50047)

Xaa Ser Xaa Ser Trp Ser Xaa Asp Xaa (SEQ ID NO: 50048)

Arg Ser Ser Tyr Tyr Trp Gly (SEQ ID NO: 50522)

**FIGURE 57**

Asn Ile Tyr Tyr Ser Gly Ser Thr Tyr Tyr Asn Pro Ser Leu Lys Ser (SEQ ID NO: 50523)

His Ser Ser Ser Trp Ser Leu Asp Tyr (SEQ ID NO: 50524)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | H_FR1 | | | | | | | | | | | | | | | | | | | | | | | | |
| CONSENSUS | Q | L | Q | L | Q | E | S | - | G | P | G | L | V | K | P | S | E | T | L | S | L | T | C | T | V |
| 21-225_11F10_HC | . | . | H | . | . | . | . | . | . | . | . | . | . | . | . | . | . | . | . | . | . | . | . | . | . |
| 21-225_15A1_HC | . | . | H | . | . | . | . | . | . | . | . | . | . | . | . | . | . | . | . | . | . | . | . | . | . |
| 21-225_15G7_HC | . | . | . | . | . | . | . | . | . | . | . | . | . | . | . | . | . | . | . | . | . | . | . | . | . |
| 21-225_15H1_HC | . | . | . | . | . | . | . | . | . | . | . | . | . | . | F | . | . | . | . | . | . | . | . | . | . |
| 21-225_16A1_HC | . | . | . | . | . | . | . | . | . | . | . | . | . | . | . | . | . | . | . | . | . | I | . | . | . |
| 21-225_18A3_HC | . | . | . | . | . | . | . | . | . | . | . | . | . | . | F | . | . | . | . | . | . | . | . | . | . |
| 21-225_18C6_HC | . | . | . | . | . | . | . | . | . | . | . | . | . | . | . | . | . | . | . | . | . | . | . | . | . |
| 21-225_20C2_HC | . | . | . | . | . | . | . | . | . | . | . | . | . | . | . | . | . | . | . | . | . | . | . | . | . |
| 21-225_20G9_HC | . | . | . | . | . | . | . | . | . | . | . | . | . | . | . | . | . | . | . | . | . | . | . | . | . |
| 21-225_21G7_HC | . | . | . | . | . | . | . | . | . | . | . | . | . | . | F | . | . | . | . | . | . | . | . | . | . |
| 21-225_22C1_HC | . | . | . | . | . | . | . | . | . | . | . | . | . | . | . | . | . | . | . | . | . | . | . | . | . |
| 21-225_22D12_HC | . | . | . | . | . | . | . | . | . | . | . | . | . | . | . | . | . | . | . | . | . | . | . | . | . |
| 21-225_22G9_HC | . | . | . | . | . | . | . | . | . | . | . | . | . | . | . | . | . | . | . | . | . | . | . | . | . |
| 21-225_23D1_HC | . | . | . | . | . | . | . | . | . | . | . | . | . | . | . | . | . | . | . | . | . | . | . | . | . |
| 21-225_24E5_HC | . | . | . | . | . | . | . | . | . | . | . | . | . | . | . | . | . | . | . | . | . | . | . | . | . |
| 21-225_34C4_HC | . | . | . | . | . | . | . | . | . | . | . | . | . | . | . | . | . | . | . | . | . | . | . | . | . |
| 21-225_59E6_HC | . | . | . | . | . | . | . | . | . | . | . | . | . | . | . | . | . | . | . | . | . | . | . | . | . |
| 21-225_68D12_HC | . | . | . | . | . | . | . | . | . | . | . | . | . | . | . | . | . | . | . | . | . | . | . | . | . |
| 21-225_6A6_HC | . | . | . | . | . | . | . | . | . | . | . | . | . | . | . | . | . | . | . | . | . | . | . | . | . |
| 21-225_6D4_HC | . | . | . | . | . | . | . | . | . | . | . | . | . | . | . | . | . | . | . | . | . | . | . | . | . |
| 21-225_7F4_HC | . | . | . | . | . | . | . | . | . | . | . | . | . | . | . | . | . | . | . | . | . | . | . | . | . |
| 21-225_8D12_HC | . | . | . | . | . | . | . | . | . | . | . | . | . | . | F | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | G | S | I | S | R | S | - | - | - | S | Y | Y | W | G | W | I | R | Q | P | P | G | K | G | L |
| 21-225_11F10_HC | . | . | A | . | . | . | . | . | . | . | . | . | . | . | . | . | . | . | . | . | . | . | . | . | . | . |
| 21-225_15A1_HC | . | . | . | . | . | . | . | . | . | . | . | . | . | . | . | . | . | . | . | . | . | . | . | . | . | . |
| 21-225_15G7_HC | . | . | . | . | . | . | . | . | . | . | . | . | S | . | . | . | . | . | . | . | . | . | . | . | . | . |
| 21-225_15H1_HC | . | . | . | . | . | . | . | . | . | . | . | . | . | . | . | . | . | . | . | . | . | . | . | . | . | . |
| 21-225_16A1_HC | . | . | . | . | . | D | . | . | . | . | . | . | . | . | . | . | . | . | . | . | . | . | . | . | . | . |
| 21-225_18A3_HC | . | . | . | . | . | . | . | . | . | . | . | . | . | . | . | . | . | . | . | . | . | . | . | . | . | . |
| 21-225_18C6_HC | . | . | . | . | . | . | . | . | . | . | . | . | . | . | . | . | . | . | . | . | . | . | . | . | . | . |
| 21-225_20C2_HC | . | . | . | . | . | . | . | . | . | . | . | . | . | . | . | . | . | . | . | . | . | . | . | . | . | . |
| 21-225_20G9_HC | . | . | . | . | . | . | . | . | . | . | . | . | . | . | . | . | . | . | . | . | . | . | . | . | . | . |
| 21-225_21G7_HC | . | . | . | . | . | . | . | . | . | . | . | . | . | . | . | . | . | . | . | . | . | . | . | . | . | Q |
| 21-225_22C1_HC | . | . | . | . | . | N | . | . | . | . | . | . | . | . | . | . | . | . | . | . | . | . | . | . | . | . |
| 21-225_22D12_HC | . | . | . | . | . | . | . | . | . | . | . | . | . | . | . | . | . | . | . | . | . | . | . | . | . | . |
| 21-225_22G9_HC | . | . | . | A | . | . | G | . | . | . | . | N | . | . | . | . | . | . | . | . | . | . | . | . | E | . |
| 21-225_23D1_HC | . | . | . | . | . | . | . | . | . | . | . | . | . | . | . | . | . | . | . | . | . | . | . | . | . | . |
| 21-225_24E5_HC | . | . | . | A | . | . | . | . | . | . | . | . | . | . | . | . | . | . | . | . | . | . | . | . | . | . |
| 21-225_34C4_HC | . | . | . | . | . | . | . | . | . | . | . | . | . | . | . | . | . | . | . | . | S | . | . | . | . | . |
| 21-225_59E6_HC | . | . | . | . | . | . | . | . | . | . | . | . | . | . | . | . | . | . | . | . | . | . | . | . | . | . |
| 21-225_68D12_HC | . | . | . | . | . | . | . | . | . | . | . | . | . | . | . | . | . | . | . | . | . | . | . | . | . | . |
| 21-225_6A6_HC | . | . | . | . | . | . | . | . | . | . | . | . | . | . | . | . | . | . | . | . | . | . | . | . | . | . |
| 21-225_6D4_HC | . | . | . | . | . | . | . | . | . | . | . | . | S | . | . | . | . | . | . | . | . | . | . | . | . | . |
| 21-225_7F4_HC | . | . | . | . | . | . | . | . | . | . | . | . | . | . | . | . | . | . | . | . | . | . | . | . | . | . |
| 21-225_8D12_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | I | G | N | I | Y | Y | - | - | - | - | S | G | S | T | Y | Y | N | P | S | L | K | S | R | V |
| 21-225_11F10_HC | . | . | . | . | S | . | . | . | . | . | . | . | . | . | . | A | Q | . | . | . | . | . | . | . | . | . |
| 21-225_15A1_HC | . | . | . | . | S | . | . | . | . | . | . | . | . | . | . | A | Q | . | . | . | . | . | . | . | . | . |
| 21-225_15G7_HC | . | . | . | . | S | . | . | . | . | . | . | . | . | . | Y | . | . | . | T | . | . | . | . | . | . | . |
| 21-225_15H1_HC | . | . | . | . | . | . | . | . | . | . | . | . | . | . | Y | P | . | . | . | . | . | . | . | . | . | . |
| 21-225_16A1_HC | . | . | . | . | S | . | . | . | . | . | . | . | . | . | . | A | . | H | . | . | . | . | . | . | . | G |
| 21-225_18A3_HC | . | . | . | . | . | . | . | . | . | . | . | . | . | . | Y | . | . | . | T | . | . | . | . | . | . | . |
| 21-225_18C6_HC | . | . | . | . | . | . | . | . | . | . | . | . | . | . | Y | . | . | . | . | . | . | . | . | . | . | . |
| 21-225_20C2_HC | . | . | . | . | . | . | . | . | . | . | . | . | . | . | T | A | . | C | . | S | . | . | . | . | . | . |
| 21-225_20G9_HC | . | . | . | . | S | . | . | . | . | . | . | . | . | . | . | A | S | . | . | . | . | . | . | . | . | . |
| 21-225_21G7_HC | . | . | . | . | . | . | . | . | . | . | . | . | . | . | Y | S | . | . | . | . | . | . | . | . | . | . |
| 21-225_22C1_HC | . | . | . | . | . | . | . | . | . | . | . | . | . | . | A | . | . | . | . | S | . | . | . | . | . | . |
| 21-225_22D12_HC | . | . | . | . | . | . | . | . | . | . | . | . | . | . | T | . | S | . | . | . | . | . | . | . | . | . |
| 21-225_22G9_HC | . | . | . | . | . | . | . | . | . | . | . | . | . | . | . | . | . | . | . | . | . | . | . | . | . | . |
| 21-225_23D1_HC | . | . | . | . | S | . | . | . | . | . | . | . | . | . | . | A | Q | . | . | . | . | . | . | . | . | . |
| 21-225_24E5_HC | . | . | . | . | S | . | . | . | . | . | . | . | . | . | . | A | N | . | . | . | . | . | . | . | . | . |
| 21-225_34C4_HC | . | . | . | . | S | . | . | . | . | . | . | . | . | . | . | . | A | . | . | . | . | . | . | . | . | . |
| 21-225_59E6_HC | . | . | . | . | . | . | . | . | . | . | . | . | . | . | . | . | . | . | . | . | . | . | . | . | . | . |
| 21-225_68D12_HC | . | . | . | . | . | . | . | . | . | . | . | . | . | . | . | . | . | . | I | . | . | . | . | . | . | . |
| 21-225_6A6_HC | . | . | . | . | . | . | . | . | . | . | . | . | . | . | I | P | . | . | . | . | . | . | . | . | . | . |
| 21-225_6D4_HC | . | . | . | . | . | . | . | . | . | . | . | . | . | . | . | A | . | . | I | . | . | . | . | . | . | . |
| 21-225_7F4_HC | . | . | . | . | . | . | . | . | . | . | . | . | . | . | A | . | . | . | . | . | . | . | . | . | . | . |
| 21-225_8D12_HC | . | . | . | . | . | . | . | . | . | . | . | . | . | . | Y | P | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | V | D | T | S | K | N | Q | F | S | L | K | L | S | S | V | T | A | A | D | T | A | V | Y |
| 21-225_11F10_HC | . | . | . | . | . | . | . | . | . | . | . | . | . | T | . | . | . | . | . | . | . | . | . | . | . | . |
| 21-225_15A1_HC | . | . | . | . | . | . | T | . | . | . | . | . | . | T | . | . | . | . | . | . | . | . | . | . | . | . |
| 21-225_15G7_HC | . | . | . | . | . | . | . | . | . | . | . | . | . | R | . | . | . | . | . | . | . | . | . | . | S | . |
| 21-225_15H1_HC | . | . | . | . | . | . | . | . | . | . | . | . | . | . | . | . | . | . | . | . | . | . | . | . | . | . |
| 21-225_16A1_HC | . | . | . | . | . | . | . | . | . | . | . | . | . | . | . | . | . | . | . | . | . | . | . | . | A | . |
| 21-225_18A3_HC | . | . | . | . | . | . | . | . | . | . | . | . | . | . | . | . | . | . | . | . | . | . | . | . | . | . |
| 21-225_18C6_HC | I | . | . | . | . | . | . | . | . | . | . | . | . | . | . | . | . | . | . | . | . | . | . | . | L | . |
| 21-225_20C2_HC | I | . | . | . | . | . | . | . | . | . | . | . | . | . | . | . | . | . | . | . | T | . | . | . | . | . |
| 21-225_20G9_HC | . | . | . | . | . | . | . | R | . | . | . | . | . | . | . | . | . | . | . | . | . | . | . | . | A | . |
| 21-225_21G7_HC | . | . | . | . | . | . | . | . | . | . | . | . | . | . | . | . | . | . | . | . | . | . | . | . | . | . |
| 21-225_22C1_HC | . | . | . | . | . | . | . | T | . | . | . | . | . | . | . | . | . | . | . | . | . | . | . | . | . | . |
| 21-225_22D12_HC | . | . | . | . | . | . | . | R | . | . | . | . | . | . | . | . | . | . | . | . | . | . | . | . | . | F |
| 21-225_22G9_HC | . | . | . | . | . | . | . | . | . | . | . | . | . | . | . | R | . | . | . | . | E | . | . | . | . | . |
| 21-225_23D1_HC | . | . | . | . | . | . | . | . | . | . | . | . | . | T | . | . | . | . | . | . | . | . | . | . | L | . |
| 21-225_24E5_HC | . | . | . | . | . | . | . | . | . | . | . | . | . | . | . | . | . | . | . | . | . | . | . | . | . | . |
| 21-225_34C4_HC | . | . | . | . | . | . | . | . | . | . | V | . | . | . | . | . | . | . | . | . | . | . | . | . | . | . |
| 21-225_59E6_HC | . | . | . | . | . | . | . | . | . | . | . | . | . | . | . | . | . | . | . | . | . | . | . | . | L | . |
| 21-225_68D12_HC | . | . | . | . | . | . | . | . | . | . | . | . | . | . | . | T | . | . | . | . | . | . | . | . | . | . |
| 21-225_6A6_HC | N | . | . | . | . | . | . | . | . | . | . | . | . | . | . | N | . | . | . | . | . | . | . | . | . | . |
| 21-225_6D4_HC | . | . | . | . | A | . | . | . | . | . | . | . | . | N | . | T | . | . | . | . | . | . | . | . | . | . |
| 21-225_7F4_HC | . | . | . | . | . | . | . | T | . | . | . | . | . | . | . | . | . | . | . | . | . | . | . | . | . | . |
| 21-225_8D12_HC | . | . | . | I | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | H | S | S | S | W | - | - | - | - | - | - | - | - | - | - | - | - | - | - | - | - | - |
| 21-225_11F10_HC | . | . | . | . | L | . | . | . | . | . | . | . | . | . | . | . | . | . | . | . | . | . | . | . | . | . |
| 21-225_15A1_HC | . | . | . | . | L | . | . | . | . | . | . | . | . | . | . | . | . | . | . | . | . | . | . | . | . | . |
| 21-225_15G7_HC | . | . | . | . | L | . | . | . | . | . | . | . | . | . | . | . | . | . | . | . | . | . | . | . | . | . |
| 21-225_15H1_HC | . | . | . | . | . | . | T | . | . | . | . | . | . | . | . | . | . | . | . | . | . | . | . | . | . | . |
| 21-225_16A1_HC | . | . | . | . | L | . | . | . | . | . | . | . | . | . | . | . | . | . | . | . | . | . | . | . | . | . |
| 21-225_18A3_HC | . | . | . | . | . | . | T | . | . | . | . | . | . | . | . | . | . | . | . | . | . | . | . | . | . | . |
| 21-225_18C6_HC | . | . | . | . | . | . | . | . | . | . | . | . | . | . | . | . | . | . | . | . | . | . | . | . | . | . |
| 21-225_20C2_HC | . | . | . | . | . | . | . | . | . | . | . | . | . | . | . | . | . | . | . | . | . | . | . | . | . | . |
| 21-225_20G9_HC | . | . | . | . | L | . | . | . | . | . | . | . | . | . | . | . | . | . | . | . | . | . | . | . | . | . |
| 21-225_21G7_HC | . | . | . | . | . | . | T | . | . | . | . | . | . | . | . | . | . | . | . | . | . | . | . | . | . | . |
| 21-225_22C1_HC | . | . | . | . | . | . | G | . | . | . | . | . | . | . | . | . | . | . | . | . | . | . | . | . | . | . |
| 21-225_22D12_HC | . | . | . | . | L | . | . | . | . | . | . | . | . | . | . | . | . | . | . | . | . | . | . | . | . | . |
| 21-225_22G9_HC | . | . | . | . | . | . | . | . | . | . | . | . | . | . | . | . | . | . | . | . | . | . | . | . | . | . |
| 21-225_23D1_HC | . | . | . | . | L | . | . | . | . | . | . | . | . | . | . | . | . | . | . | . | . | . | . | . | . | . |
| 21-225_24E5_HC | . | . | . | . | L | . | . | . | . | . | . | . | . | . | . | . | . | . | . | . | . | . | . | . | . | . |
| 21-225_34C4_HC | . | . | . | . | L | . | . | . | . | . | . | . | . | . | . | . | . | . | . | . | . | . | . | . | . | . |
| 21-225_59E6_HC | . | . | . | . | . | . | . | . | . | . | . | . | . | . | . | . | . | . | . | . | . | . | . | . | . | . |
| 21-225_68D12_HC | . | . | . | . | . | . | T | . | . | . | . | . | . | . | . | . | . | . | . | . | . | . | . | . | . | . |
| 21-225_6A6_HC | . | . | . | . | . | . | . | . | . | . | . | . | . | . | . | . | . | . | . | . | . | . | . | . | . | . |
| 21-225_6D4_HC | . | . | . | . | . | . | . | . | . | . | . | . | . | . | . | . | . | . | . | . | . | . | . | . | . | . |
| 21-225_7F4_HC | . | . | . | . | . | . | G | . | . | . | . | . | . | . | . | . | . | . | . | . | . | . | . | . | . | . |
| 21-225_8D12_HC | . | . | . | . | . | . | T | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | S | L | D | Y | W | G | Q | G | T | L | V | T | V | S | S |
| 21-225_11F10_HC | . | . | . | . | . | F | . | . | . | . | . | . | . | . | . | . | . | . | . |
| 21-225_15A1_HC | . | . | . | . | . | F | . | . | . | . | . | . | . | . | . | . | . | . | . |
| 21-225_15G7_HC | . | . | . | . | . | F | . | F | . | . | . | . | . | . | . | . | . | . | . |
| 21-225_15H1_HC | . | . | . | . | . | . | . | . | . | . | . | . | . | . | . | . | . | . | . |
| 21-225_16A1_HC | . | . | . | . | . | F | . | . | . | . | . | . | . | . | . | . | . | . | . |
| 21-225_18A3_HC | . | . | . | . | . | . | . | . | . | . | . | . | . | . | . | . | . | . | . |
| 21-225_18C6_HC | . | . | . | . | . | . | . | D | . | . | . | . | . | . | . | . | . | . | . |
| 21-225_20C2_HC | . | . | . | . | . | . | . | N | . | . | . | . | . | . | . | . | . | . | . |
| 21-225_20G9_HC | . | . | . | . | . | F | . | . | . | . | . | . | . | . | . | . | . | . | . |
| 21-225_21G7_HC | . | . | . | . | . | . | . | . | . | . | . | . | . | . | . | . | . | . | . |
| 21-225_22C1_HC | . | . | . | . | . | . | . | . | . | . | . | . | . | . | . | . | . | . | . |
| 21-225_22D12_HC | . | . | . | . | . | V | . | . | . | . | . | . | . | . | . | . | . | . | . |
| 21-225_22G9_HC | . | . | . | . | . | . | . | . | . | . | . | . | . | . | . | . | . | . | . |
| 21-225_23D1_HC | . | . | . | . | . | F | . | . | . | . | . | . | . | . | . | . | . | . | . |
| 21-225_24E5_HC | . | . | . | . | . | I | . | N | . | . | . | . | . | . | . | . | . | . | . |
| 21-225_34C4_HC | . | . | . | . | . | F | . | . | . | . | . | . | . | . | . | . | . | . | . |
| 21-225_59E6_HC | . | . | . | . | . | . | . | . | . | . | . | . | . | . | . | . | . | . | . |
| 21-225_68D12_HC | . | . | . | . | . | I | . | . | . | . | . | . | . | . | . | . | . | . | . |
| 21-225_6A6_HC | . | . | . | . | . | . | . | . | . | . | . | . | . | . | . | . | . | . | . |
| 21-225_6D4_HC | . | . | . | . | . | . | . | C | . | . | . | . | . | . | . | . | . | . | . |
| 21-225_7F4_HC | . | . | . | . | . | . | . | . | . | . | . | . | . | . | . | . | . | . | . |
| 21-225_8D12_HC | . | . | . | . | . | . | . | . | . | . | . | . | . | . | . | . | . | . | . |

**FIGURE 57**

Table 68. Consensus 34-VH1|1-02/D5|5-18|RF3/JH5 (SEQ ID NO: 50285):

QVQLVQS-GAEVKKPGASVKVSCKASG-YTFTG-----YYIHWVRQAPGQGLEWMGWINPN---SGGTNYAQKFQGRVTMTRDTSISTAYMELSRLRSDDTAVYYCARGYSYGY--------------------NWFDPWGQGTLVTVSS wherein:

G in position 33 can be substituted with D

Y in position 39 can be substituted with H

Y in position 40 can be substituted with F

I in position 41 can be substituted with M

H in position 42 can be substituted with N

P in position 60 can be substituted with S

N in position 61 can be substituted with K

S in position 65 can be substituted with N

G in position 66 can be substituted with D

G in position 67 can be substituted with D

A in position 71 can be substituted with E

Q in position 72 can be substituted with E

G in position 76 can be substituted with D

G in position 109 can be substituted with D

Y in position 110 can be substituted with G, T or F

S in position 111 can be substituted with Y, D or R

Y in position 112 can be substituted with S

**FIGURE 57**

G in position 113 can be substituted with null (-) or S

Y in position 114 can be substituted with S or null (-)

null (-) in position 115 can be substituted with G

null (-) in position 116 can be substituted with S

null (-) in position 132 can be substituted with Y

null (-) in position 133 can be substituted with Y, F or H

N in position 134 can be substituted with null (-) or D

W in position 135 can be substituted with null (-), D or E

F in position 136 can be substituted with L

D in position 137 can be substituted with A

P in position 138 can be substituted with S

Xaa Xaa Xaa Xaa Xaa (SEQ ID NO: 50049)

Trp Ile Asn Xaa Xaa Xaa Xaa Xaa Thr Asn Tyr Xaa Xaa Lys Phe Gln Xaa (SEQ ID NO: 50050)

Xaa Xaa Xaa Xaa Xaa Xaa Xaa Xaa Xaa Xaa Xaa Xaa Xaa Xaa Xaa (SEQ ID NO: 50051)

Gly Tyr Tyr Ile His (SEQ ID NO: 50525)

Trp Ile Asn Pro Asn Ser Gly Gly Thr Asn Tyr Ala Gln Lys Phe Gln Gly (SEQ ID NO: 50526)

Gly Tyr Ser Tyr Gly Tyr Asn Trp Phe Asp Pro (SEQ ID NO: 50527)

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | H_FR1 | | | | | | | | | | | | | | | | | | | | | | | | |
| CONSENSUS | Q | V | Q | L | V | Q | S | - | G | A | E | V | K | K | P | G | A | S | V | K | V | S | C | K | A |
| 21-225_17G4_HC | . | . | . | . | . | . | . | . | . | . | . | . | . | . | . | . | . | . | . | . | . | . | . | . | T |
| 21-225_19A1_HC | . | . | . | . | . | . | . | . | . | . | . | . | . | . | . | . | . | . | . | . | . | . | . | . | . |
| 21-225_201H10_HC | . | . | . | . | . | . | . | . | . | . | . | . | . | . | . | . | . | . | . | . | . | . | . | . | . |
| 21-225_203E3_HC | . | . | . | . | . | . | . | . | . | . | . | . | . | . | . | . | . | . | . | . | . | . | . | . | . |
| 21-225_204H4_HC | . | . | . | . | . | . | . | . | . | . | . | . | . | . | . | . | . | . | . | . | . | . | . | . | . |
| 21-225_206A4_HC | . | . | . | . | . | . | . | . | . | . | . | . | . | . | . | . | . | . | . | . | . | . | . | . | . |
| 21-225_206A5_HC | . | . | . | . | . | . | . | . | . | . | . | . | . | . | . | . | . | . | . | . | . | . | . | . | . |
| 21-225_208B11_HC | . | . | . | . | . | . | . | . | . | . | . | . | . | . | . | . | . | . | . | . | . | . | . | . | . |
| 21-225_208E8_HC | . | . | . | . | . | . | . | . | . | . | . | . | . | . | . | . | . | . | . | . | . | . | . | . | . |
| 21-225_210B12_HC | . | . | . | . | . | . | . | . | . | . | . | . | . | . | . | . | . | . | . | . | I | . | . | . | . |
| 21-225_210D11_HC | . | . | . | . | . | . | . | . | . | . | . | . | . | . | . | . | . | . | . | . | . | . | . | . | . |
| 21-225_213A7_HC | . | . | . | . | . | . | . | . | . | . | . | . | . | . | . | . | . | . | . | . | I | . | . | [illegible] | . |
| 21-225_214H8_HC | . | . | . | . | . | . | . | . | . | . | . | . | . | . | . | . | . | . | . | . | . | . | . | . | . |
| 21-225_215H6_HC | . | . | . | . | . | . | . | . | . | . | . | . | . | . | . | . | . | . | . | . | . | . | . | . | . |
| 21-225_226A10_HC | . | . | . | . | . | . | . | . | . | . | . | . | . | . | . | . | . | . | . | . | . | . | . | . | . |
| 21-225_226B2_HC | . | . | . | . | . | . | . | . | . | . | . | . | . | . | . | . | . | . | . | . | . | . | . | . | . |
| 21-225_226B7_HC | . | . | . | . | . | . | . | . | . | . | . | . | . | . | . | . | . | . | . | . | . | . | . | . | . |
| 21-225_32B11_HC | . | . | . | . | . | . | . | . | . | . | . | . | . | . | . | . | . | . | . | . | . | . | . | . | . |
| 21-225_34D7_HC | . | . | . | . | . | . | . | . | . | . | . | . | . | . | . | . | . | . | . | . | . | . | . | . | . |

FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | H_CDR1 | | | | | | | | | | H_FR2 | | | | | | | |
| CONSENSUS | S | G | - | Y | T | F | T | G | - | - | - | - | - | Y | Y | I | H | W | V | R | Q | A | P | G | Q |
| 21-225_17G4_HC | . | . | . | . | . | . | . | D | . | . | . | . | . | . | . | M | . | . | . | . | . | . | . | . | . |
| 21-225_19A1_HC | . | . | . | . | . | . | . | . | . | . | . | . | . | . | . | M | . | . | . | . | . | . | . | . | . |
| 21-225_201H10_HC | . | . | . | . | . | . | . | . | . | . | . | . | . | . | . | . | . | . | . | . | . | . | . | . | . |
| 21-225_203E3_HC | . | . | . | . | . | . | . | . | . | . | . | . | . | . | . | . | . | . | . | . | . | . | . | . | . |
| 21-225_204H4_HC | . | . | . | . | . | . | . | . | . | . | . | . | . | . | . | . | . | . | . | . | . | . | . | . | . |
| 21-225_206A4_HC | . | . | . | . | . | . | . | . | . | . | . | . | . | . | . | . | . | . | . | . | . | . | . | . | . |
| 21-225_206A5_HC | . | . | . | . | . | . | . | . | . | . | . | . | . | . | . | . | . | . | . | . | . | . | . | . | . |
| 21-225_208B11_HC | . | . | . | . | . | . | . | . | . | . | . | . | . | . | . | . | . | . | . | . | . | . | . | . | . |
| 21-225_208E8_HC | . | . | . | . | . | . | . | . | . | . | . | . | . | . | . | . | . | . | . | . | . | . | . | . | . |
| 21-225_210B12_HC | . | . | . | F | . | . | . | D | . | . | . | . | . | . | . | M | N | . | . | . | . | . | . | . | . |
| 21-225_210D11_HC | . | . | . | . | . | . | . | . | . | . | . | . | . | . | . | . | . | . | . | . | . | . | . | . | . |
| 21-225_213A7_HC | . | . | . | F | . | . | . | D | . | . | . | . | . | . | . | M | N | . | . | . | . | . | . | . | . |
| 21-225_214H8_HC | . | . | . | . | . | . | . | . | . | . | . | . | . | H | . | . | . | . | . | . | . | . | . | . | . |
| 21-225_215H6_HC | . | . | . | . | . | . | . | . | . | . | . | . | . | H | . | . | . | . | . | . | . | . | . | . | . |
| 21-225_226A10_HC | . | . | . | . | . | . | . | . | . | . | . | . | . | . | F | M | . | . | . | . | . | . | . | . | . |
| 21-225_226B2_HC | . | . | . | . | . | . | . | . | . | . | . | . | . | . | F | M | . | . | . | . | . | . | . | . | . |
| 21-225_226B7_HC | . | . | . | . | . | . | . | . | . | . | . | . | . | . | . | M | . | . | . | . | . | . | . | . | . |
| 21-225_32B11_HC | . | . | . | . | . | . | . | . | . | . | . | . | . | . | . | M | . | . | . | . | . | . | . | . | . |
| 21-225_34D7_HC | . | . | . | . | . | . | . | . | . | . | . | . | . | . | . | M | . | . | . | . | . | . | . | . | . |

**FIGURE 57**

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | H_CDR2 | | | | | | | | | | | | | | | | | | |
| CONSENSUS | G | L | E | W | M | G | W | I | N | P | N | - | - | - | S | G | G | T | N | Y | A | Q | K | F | Q |
| 21-225_17G4_HC | . | . | . | . | . | . | . | . | . | . | . | . | . | . | . | . | . | . | . | . | E | . | . | . | . |
| 21-225_19A1_HC | . | . | . | . | . | . | . | . | . | . | . | . | . | . | . | . | . | . | . | . | . | . | . | . | . |
| 21-225_201H10_HC | . | . | . | . | . | A | . | . | . | . | . | . | . | . | . | . | . | . | . | . | . | . | . | . | . |
| 21-225_203E3_HC | . | . | . | . | . | A | . | . | . | . | . | . | . | . | . | . | . | . | . | . | . | . | . | . | . |
| 21-225_204H4_HC | . | . | . | . | . | A | . | . | . | . | . | . | . | . | . | . | . | . | . | . | . | . | . | . | . |
| 21-225_206A4_HC | . | . | . | . | . | A | . | . | . | . | . | . | . | . | . | . | . | . | . | . | . | . | . | . | . |
| 21-225_206A5_HC | . | . | . | . | . | A | . | . | . | . | . | . | . | . | . | . | . | . | . | . | . | . | . | . | . |
| 21-225_208B11_HC | . | . | . | . | . | A | . | . | . | . | . | . | . | . | . | . | . | . | . | . | . | . | . | . | . |
| 21-225_208E8_HC | . | . | . | . | . | A | . | . | . | . | . | . | . | . | . | . | . | . | . | . | . | . | . | . | . |
| 21-225_210B12_HC | . | . | . | . | . | . | . | . | . | . | . | . | . | . | . | . | . | . | . | . | . | . | . | . | . |
| 21-225_210D11_HC | . | . | . | . | . | A | . | . | . | . | . | . | . | . | . | . | . | . | . | . | . | . | . | . | . |
| 21-225_213A7_HC | . | . | . | . | . | . | . | . | . | . | K | . | . | . | N | . | . | . | . | . | . | . | . | . | . |
| 21-225_214H8_HC | . | . | . | . | . | . | . | . | . | S | . | . | . | . | . | . | . | . | . | . | . | . | . | . | . |
| 21-225_215H6_HC | . | . | . | . | . | . | . | . | . | S | . | . | . | . | . | . | . | . | . | . | . | E | . | . | . |
| 21-225_226A10_HC | . | . | . | . | . | . | . | . | . | . | . | . | . | . | . | . | D | . | . | . | . | . | . | . | . |
| 21-225_226B2_HC | . | . | . | . | . | . | . | . | . | . | . | . | . | . | . | . | D | . | . | . | . | . | . | . | . |
| 21-225_226B7_HC | . | . | . | . | . | . | . | . | . | . | . | . | . | . | . | . | . | . | . | . | . | . | . | . | . |
| 21-225_32B11_HC | . | . | . | . | . | . | . | . | . | . | . | . | . | . | . | D | . | . | . | . | . | . | . | . | . |
| 21-225_34D7_HC | . | . | . | . | . | . | . | . | . | . | . | . | . | . | . | . | . | . | . | . | . | . | . | . | . |

FIGURE 57

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | H_FR3 | | | | | | | | | | | | | | | | | | | | | | | |
| CONSENSUS | G | R | V | T | M | T | R | D | T | S | I | S | T | A | Y | M | E | L | S | R | L | R | S | D | D |
| 21-225_17G4_HC | . | . | . | . | . | . | . | . | . | . | . | . | . | . | . | . | . | . | . | . | . | . | . | . | . |
| 21-225_19A1_HC | . | . | . | . | . | . | . | . | . | . | . | . | . | . | . | . | . | . | . | . | . | . | . | . | . |
| 21-225_201H10_HC | . | . | . | . | . | . | . | . | . | . | . | T | . | T | . | . | . | . | . | . | . | . | . | . | . |
| 21-225_203E3_HC | . | . | . | . | . | . | . | . | . | . | . | T | . | . | . | . | . | . | . | . | . | . | . | . | . |
| 21-225_204H4_HC | . | . | . | . | . | . | . | . | . | . | . | T | . | . | . | . | . | . | . | . | . | . | . | . | . |
| 21-225_206A4_HC | . | . | . | . | . | . | . | . | . | . | . | T | . | . | . | . | . | . | . | . | . | . | . | . | . |
| 21-225_206A5_HC | . | . | . | . | . | . | . | . | . | . | . | T | . | . | . | . | . | . | . | . | . | . | . | . | . |
| 21-225_208B11_HC | . | . | . | . | . | . | . | . | . | . | . | T | . | . | . | . | . | . | . | . | . | . | . | . | . |
| 21-225_208E8_HC | . | . | . | . | . | . | . | . | . | . | . | T | . | . | . | . | . | . | . | . | . | . | . | . | . |
| 21-225_210B12_HC | . | . | . | . | . | . | . | . | . | . | . | . | . | . | . | . | . | . | . | . | . | . | . | . | . |
| 21-225_210D11_HC | . | . | . | . | . | . | . | . | . | . | . | T | . | . | . | . | . | . | . | . | . | . | . | . | . |
| 21-225_213A7_HC | . | . | . | . | . | . | . | . | . | . | L | . | R | . | . | . | . | . | . | . | . | . | . | . | . |
| 21-225_214H8_HC | . | . | . | . | . | . | . | . | . | . | . | . | . | . | . | . | . | . | . | . | . | . | . | . | . |
| 21-225_215H6_HC | . | . | . | . | . | . | . | . | . | . | . | . | . | . | . | . | . | . | . | . | . | . | . | . | . |
| 21-225_226A10_HC | . | . | . | . | . | . | . | . | . | . | L | . | . | . | . | . | . | . | . | . | . | . | . | . | . |
| 21-225_226B2_HC | . | . | . | . | . | . | . | . | . | . | L | N | . | . | . | . | . | . | . | . | . | . | . | . | . |
| 21-225_226B7_HC | D | . | . | . | . | . | . | . | . | . | . | . | . | . | H | . | . | . | . | . | . | . | . | . | . |
| 21-225_32B11_HC | . | . | . | . | . | . | . | . | . | . | . | . | . | . | . | . | . | . | N | . | . | . | . | . | . |
| 21-225_34D7_HC | . | . | . | . | . | . | . | . | . | . | . | . | . | . | . | . | . | . | . | . | . | . | . | . | . |

# FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | R | G | Y | S | Y | G | Y | - | - | - | - | - | - | - | - | - | - | - |
| 21-225_17G4_HC | . | . | . | . | . | . | . | S | . | . | . | . | . | . | . | . | . | . | . | . | . | . | . | . | . |
| 21-225_19A1_HC | . | . | . | . | . | . | . | S | . | . | . | . | . | . | . | . | . | . | . | . | . | . | . | . | . |
| 21-225_201H10_HC | . | . | . | . | . | . | . | . | . | . | . | . | . | . | . | . | . | . | . | . | . | . | . | . | . |
| 21-225_203E3_HC | . | . | . | . | . | . | . | . | . | . | . | . | . | . | . | . | . | . | . | . | . | . | . | . | . |
| 21-225_204H4_HC | . | . | . | . | . | . | . | . | . | . | . | . | . | . | . | . | . | . | . | . | . | . | . | . | . |
| 21-225_206A4_HC | . | . | . | . | . | . | . | . | . | . | . | . | . | . | . | . | . | . | . | . | . | . | . | . | . |
| 21-225_206A5_HC | . | . | . | . | . | . | . | . | . | . | . | . | . | . | . | . | . | . | . | . | . | . | . | . | . |
| 21-225_208B11_HC | . | . | . | . | . | . | . | . | . | . | . | . | . | . | . | . | . | . | . | . | . | . | . | . | . |
| 21-225_208E8_HC | . | . | . | . | . | . | . | . | . | . | . | . | . | . | . | . | . | . | . | . | . | . | . | . | . |
| 21-225_210B12_HC | . | . | . | . | . | . | . | . | . | . | D | S | - | - | . | . | . | . | . | . | . | . | . | . | . |
| 21-225_210D11_HC | . | . | . | . | . | . | . | . | . | . | . | . | . | . | . | . | . | . | . | . | . | . | . | . | . |
| 21-225_213A7_HC | . | . | . | . | . | . | . | K | . | . | D | S | - | - | . | . | . | . | . | . | . | . | . | . | . |
| 21-225_214H8_HC | . | . | . | . | . | . | . | K | D | G | R | . | S | . | G | . | . | . | . | . | . | . | . | . | . |
| 21-225_215H6_HC | . | . | . | . | . | . | . | K | D | G | R | . | S | . | G | . | . | . | . | . | . | . | . | . | . |
| 21-225_226A10_HC | . | . | I | . | . | . | . | . | . | T | Y | . | . | S | G | S | . | . | . | . | . | . | . | . | . |
| 21-225_226B2_HC | . | . | I | . | . | . | . | . | . | T | Y | . | . | S | G | S | . | . | . | . | . | . | . | . | . |
| 21-225_226B7_HC | . | . | . | . | . | . | . | . | . | . | Y | . | . | S | G | S | . | . | . | . | . | . | . | . | . |
| 21-225_32B11_HC | . | . | . | . | . | . | . | . | . | F | Y | . | . | S | G | S | . | . | . | . | . | . | . | . | . |
| 21-225_34D7_HC | . | . | . | . | . | . | . | . | . | . | Y | . | . | S | G | S | . | . | . | . | . | . | . | . | . |

**FIGURE 57**

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | H_FR4 | | | | | | | | | | |
| CONSENSUS | - | - | - | - | - | - | - | - | N | W | F | D | P | W | G | Q | G | T | L | V | T | V | S | S |
| 21-225_17G4_HC | . | . | . | . | . | . | . | . | . | . | . | . | . | . | . | . | . | . | . | . | . | . | . | . |
| 21-225_19A1_HC | . | . | . | . | . | . | . | . | . | . | . | . | . | . | . | . | . | . | . | . | . | . | . | . |
| 21-225_201H10_HC | . | . | . | . | . | . | . | . | . | . | . | . | . | . | . | . | . | . | . | . | . | . | . | . |
| 21-225_203E3_HC | . | . | . | . | . | . | . | . | . | . | . | . | . | . | . | . | . | . | . | . | . | . | . | . |
| 21-225_204H4_HC | . | . | . | . | . | . | . | . | . | . | . | . | . | . | . | . | . | . | . | . | . | . | . | . |
| 21-225_206A4_HC | . | . | . | . | . | . | . | . | . | . | . | . | . | . | . | . | . | . | . | . | . | . | . | . |
| 21-225_206A5_HC | . | . | . | . | . | . | . | . | . | . | . | . | . | . | . | . | . | . | . | . | . | . | . | . |
| 21-225_208B11_HC | . | . | . | . | . | . | . | . | . | . | . | . | . | . | . | . | . | . | . | . | . | . | . | . |
| 21-225_208E8_HC | . | . | . | . | . | . | . | . | . | . | . | . | . | . | . | . | . | . | . | . | . | . | . | . |
| 21-225_210B12_HC | . | . | . | . | . | . | . | . | - | - | . | A | . | . | . | . | . | . | . | . | . | . | . | . |
| 21-225_210D11_HC | . | . | . | . | . | . | . | . | . | . | . | . | . | . | . | . | . | . | . | . | . | . | . | . |
| 21-225_213A7_HC | . | . | . | . | . | . | . | . | - | - | . | A | . | . | . | . | . | . | . | . | . | . | . | . |
| 21-225_214H8_HC | . | . | . | . | . | . | . | Y | D | . | . | . | . | . | . | . | . | . | . | . | . | . | . | . |
| 21-225_215H6_HC | . | . | . | . | . | . | . | H | D | . | . | . | . | . | . | . | . | . | . | . | . | . | . | . |
| 21-225_226A10_HC | . | . | . | . | . | . | Y | F | . | E | L | . | S | . | . | . | . | . | . | . | . | . | . | . |
| 21-225_226B2_HC | . | . | . | . | . | . | Y | F | . | E | L | . | S | . | . | . | . | . | . | . | . | . | . | . |
| 21-225_226B7_HC | . | . | . | . | . | . | Y | Y | . | . | . | . | S | . | . | . | . | . | . | . | . | . | . | . |
| 21-225_32B11_HC | . | . | . | . | . | . | Y | Y | . | D | L | . | . | . | . | . | . | . | . | . | . | . | . | . |
| 21-225_34D7_HC | . | . | . | . | . | . | Y | Y | . | D | L | . | . | . | . | . | . | . | . | . | . | . | . | . |

**FIGURE 57**

Table 69. Consensus 35- VH3|3-33/D1|1-1|RF3/JH6 (SEQ ID NO: 50286):

QVQLVES-GGGVVQPGRSLRLSCAASG-FTFSH-----YGMHWVRQAPGKGLEWVAVIWYD---GSNKYYADSVKGRFTISRDNSKNTLYLQMNSLRAEDTAVYYCARGDWNP------------------EGMDVWGQGTTVTVSS wherein:

H in position 33 can be substituted with N

Y in position 39 can be substituted with S

V in position 57 can be substituted with I

W in position 59 can be substituted with Y

N in position 67 can be substituted with Y

K in position 68 can be substituted with E

Y in position 70 can be substituted with C or N

G in position 109 can be substituted with D

D in position 110 can be substituted with R

W in position 111 can be substituted with H

N in position 112 can be substituted with Y

P in position 113 can be substituted with D

null (-) in position 114 can be substituted with F

null (-) in position 115 can be substituted with H

null (-) in position 116 can be substituted with V

null (-) in position 117 can be substituted with P

**FIGURE 57**

null (-) in position 130 can be substituted with Y

null (-) in position 131 can be substituted with Y

null (-) in position 132 can be substituted with Y

null (-) in position 133 can be substituted with Y

E in position 134 can be substituted with Y

M in position 136 can be substituted with L

Xaa Xaa Gly Met His (SEQ ID NO: 50052)

Xaa Ile Xaa Tyr Asp Gly Ser Xaa Xaa Xaa Tyr Ala Asp Ser Val Lys Gly (SEQ ID NO: 50053)

Xaa Xaa Xaa Xaa Xaa Xaa Xaa Xaa Xaa Xaa Xaa Xaa Xaa Xaa Gly Xaa Asp Val (SEQ ID NO: 50054)

His Tyr Gly Met His (SEQ ID NO: 50528)

Val Ile Trp Tyr Asp Gly Ser Asn Lys Tyr Tyr Ala Asp Ser Val Lys Gly (SEQ ID NO: 50529)

Gly Asp Trp Asn Pro Glu Gly Met Asp Val (SEQ ID NO: 50530)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | H_FR1 | | | | | | | | | | | | | | | | | | | | | | | | |
| CONSENSUS | Q | V | Q | L | V | E | S | - | G | G | G | V | V | Q | P | G | R | S | L | R | L | S | C | A | A |
| 21-225_179C7_HC | . | A | . | . | . | . | . | . | . | . | . | . | . | . | . | . | . | . | . | . | . | . | . | . | . |
| 21-225_210D12_HC | . | . | . | . | . | . | . | . | . | . | . | . | . | . | . | . | . | . | . | . | . | . | . | . | . |
| 21-225_210E12_HC | . | . | H | . | . | . | . | . | . | . | . | . | . | . | . | . | . | . | . | . | . | . | . | . | . |
| 21-225_211C1_HC | . | . | . | . | . | . | . | . | . | . | . | . | . | . | . | . | . | . | . | . | . | . | . | . | . |
| 21-225_211G5_HC | . | . | . | . | . | . | . | . | . | . | . | . | . | . | . | . | . | . | . | . | . | . | . | . | . |
| 21-225_212A4_HC | . | . | H | . | . | . | . | . | . | . | . | . | . | . | A | . | . | . | . | . | . | . | . | . | . |
| 21-225_212C2_HC | . | . | . | . | . | . | . | . | . | . | . | . | . | . | . | . | . | . | . | . | . | . | . | . | . |
| 21-225_212F6_HC | . | . | . | . | . | . | . | . | . | . | . | . | . | . | . | . | . | . | . | . | . | . | . | . | . |
| 21-225_212G7_HC | . | . | . | . | . | . | . | . | . | . | . | . | . | . | . | . | . | . | . | . | . | . | . | . | . |
| 21-225_213D5_HC | . | . | . | . | . | . | . | . | . | . | . | . | . | . | . | . | . | . | . | . | . | . | . | . | . |
| 21-225_213G3_HC | . | . | . | . | . | . | . | . | . | . | . | . | . | . | . | . | . | . | . | . | . | . | . | . | . |
| 21-225_215D3_HC | . | T | . | . | . | . | . | . | . | . | . | . | . | . | . | . | . | . | . | . | . | . | . | . | . |
| 21-225_216A3_HC | . | . | . | . | . | . | . | . | . | . | . | . | . | . | . | . | . | . | . | . | . | . | . | . | . |
| 21-225_216G1_HC | . | . | H | . | . | . | . | . | . | . | . | . | . | . | . | . | . | . | . | . | . | . | . | . | . |
| 21-225_217B2_HC | . | . | H | . | . | . | . | . | . | . | . | . | . | . | . | . | . | . | . | . | . | . | . | . | . |
| 21-225_219A7_HC | . | . | . | . | . | . | . | . | . | . | . | . | . | . | . | . | . | . | . | . | . | . | . | P | . |
| 21-225_221G4_HC | . | . | . | . | . | . | . | . | . | . | . | . | . | . | . | . | . | . | . | . | . | . | . | . | . |
| 21-225_221H2_HC | . | . | . | . | . | . | . | . | . | . | . | . | . | . | . | . | . | . | . | . | . | . | . | . | . |

FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | | | | | H_CDR1 | | | | | | | | | | | | H_FR2 | |
| CONSENSUS | S | G | - | F | T | F | S | H | - | - | - | - | - | Y | G | M | H | W | V | R | Q | A | P | G | K |
| 21-225_179C7_HC | . | . | . | . | . | . | . | N | . | . | . | . | . | S | . | . | . | . | . | . | . | G | . | . | . |
| 21-225_210D12_HC | . | . | . | . | . | . | . | . | . | . | . | . | . | . | . | . | . | . | . | . | . | . | . | . | . |
| 21-225_210E12_HC | . | . | . | . | . | . | . | N | . | . | . | . | . | . | . | . | . | . | . | . | . | . | . | . | . |
| 21-225_211C1_HC | . | . | . | . | . | . | . | . | . | . | . | . | . | . | . | . | . | . | . | . | . | . | . | . | . |
| 21-225_211G5_HC | . | . | . | . | . | . | . | . | . | . | . | . | . | . | . | . | . | . | . | . | . | . | . | . | . |
| 21-225_212A4_HC | . | . | . | . | . | . | . | N | . | . | . | . | . | . | . | . | . | . | . | . | . | . | . | . | . |
| 21-225_212C2_HC | . | . | . | . | . | . | . | . | . | . | . | . | . | . | . | . | . | . | . | . | . | . | . | . | . |
| 21-225_212F6_HC | . | . | . | . | . | . | . | . | . | . | . | . | . | . | . | . | . | . | . | . | . | . | . | . | . |
| 21-225_212G7_HC | . | . | . | . | . | . | . | . | . | . | . | . | . | . | . | . | . | . | . | . | . | . | . | . | . |
| 21-225_213D5_HC | . | . | . | . | . | . | . | . | . | . | . | . | . | . | . | . | . | . | . | . | . | . | . | . | . |
| 21-225_213G3_HC | . | . | . | . | . | . | . | . | . | . | . | . | . | . | . | . | . | . | . | . | . | . | . | . | . |
| 21-225_215D3_HC | . | . | . | . | . | . | . | . | . | . | . | . | . | . | . | . | . | . | . | . | . | . | . | . | . |
| 21-225_216A3_HC | . | . | . | . | . | . | . | . | . | . | . | . | . | . | . | . | . | . | . | . | . | . | . | . | . |
| 21-225_216G1_HC | . | . | . | . | . | . | . | N | . | . | . | . | . | . | . | . | . | . | . | . | . | . | . | . | . |
| 21-225_217B2_HC | . | . | . | . | . | . | . | N | . | . | . | . | . | . | . | . | . | . | . | . | . | . | . | . | . |
| 21-225_219A7_HC | . | . | . | . | . | . | . | . | . | . | . | . | . | . | . | . | . | . | . | . | . | . | . | . | . |
| 21-225_221G4_HC | . | . | . | . | . | . | . | . | . | . | . | . | . | . | . | . | . | . | . | . | . | . | . | . | . |
| 21-225_221H2_HC | . | . | . | . | . | . | . | . | . | . | . | . | . | . | . | . | . | . | . | . | . | . | . | . | . |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | H_CDR2 | | | | | | | | | | | | | | | | | | |
| CONSENSUS | G | L | E | W | V | A | V | I | W | Y | D | - | - | - | G | S | N | K | Y | Y | A | D | S | V | K |
| 21-225_179C7_HC | . | . | . | . | . | . | I | . | Y | . | . | . | . | . | . | . | Y | . | . | N | . | . | . | . | . |
| 21-225_210D12_HC | . | . | . | . | . | . | . | . | . | . | . | . | . | . | . | . | . | . | . | . | . | . | . | . | . |
| 21-225_210E12_HC | . | . | . | . | . | . | . | . | . | . | . | . | . | . | . | . | . | . | . | . | . | . | . | . | . |
| 21-225_211C1_HC | . | . | . | . | . | . | . | . | . | . | . | . | . | . | . | . | . | . | . | . | . | . | . | . | . |
| 21-225_211G5_HC | . | . | . | . | . | . | . | . | . | . | . | . | . | . | . | . | . | . | . | . | . | . | . | . | . |
| 21-225_212A4_HC | . | . | . | . | . | . | . | . | . | . | . | . | . | . | . | . | . | . | . | . | . | . | . | . | . |
| 21-225_212C2_HC | . | . | . | . | . | . | . | . | . | . | . | . | . | . | . | . | . | . | . | . | . | . | . | . | . |
| 21-225_212F6_HC | . | . | . | . | . | . | . | . | . | . | . | . | . | . | . | . | . | . | . | . | . | . | . | . | . |
| 21-225_212G7_HC | . | . | . | . | . | . | . | . | . | . | . | . | . | . | . | . | . | . | . | C | . | . | . | . | . |
| 21-225_213D5_HC | . | . | . | . | . | . | . | . | . | . | . | . | . | . | . | . | . | . | . | . | . | . | . | . | . |
| 21-225_213G3_HC | . | . | . | . | . | . | . | . | . | . | . | . | . | . | . | . | . | . | . | . | . | . | . | . | . |
| 21-225_215D3_HC | . | . | . | . | . | . | . | . | . | . | . | . | . | . | . | . | . | E | . | . | . | . | . | . | . |
| 21-225_216A3_HC | . | . | . | . | . | . | . | . | . | . | . | . | . | . | . | . | . | . | . | . | . | . | . | . | . |
| 21-225_216G1_HC | . | . | . | . | . | . | . | . | . | . | . | . | . | . | . | . | . | . | . | . | . | . | . | . | . |
| 21-225_217B2_HC | . | . | . | . | . | . | . | . | . | . | . | . | . | . | . | . | . | . | . | . | . | . | . | . | . |
| 21-225_219A7_HC | . | . | . | . | . | . | . | . | . | . | . | . | . | . | . | . | . | . | . | . | . | . | . | . | . |
| 21-225_221G4_HC | . | . | . | . | . | . | . | . | . | . | . | . | . | . | . | . | . | . | . | . | . | . | . | . | . |
| 21-225_221H2_HC | . | . | . | . | . | . | . | . | . | . | . | . | . | . | . | . | . | . | . | . | . | . | . | . | . |

# FIGURE 57

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | H_FR3 | | | | | | | | | | | | | | | | | | | | | | | |
| CONSENSUS | G | R | F | T | I | S | R | D | N | S | K | N | T | L | Y | L | Q | M | N | S | L | R | A | E | D |
| 21-225_179C7_HC | . | . | . | . | . | . | . | . | . | . | . | . | . | . | . | . | . | . | . | . | . | . | . | . | . |
| 21-225_210D12_HC | . | . | . | . | . | . | . | . | . | . | . | . | . | . | . | . | . | . | . | . | . | . | . | . | . |
| 21-225_210E12_HC | . | . | . | . | . | . | . | . | . | . | . | . | . | . | . | . | . | . | . | . | . | . | . | . | . |
| 21-225_211C1_HC | . | . | . | . | . | . | . | . | . | . | . | . | . | . | . | . | . | . | . | T | . | . | . | . | . |
| 21-225_211G5_HC | . | . | . | . | . | . | . | . | . | . | . | . | . | . | . | . | . | . | . | . | . | . | . | . | . |
| 21-225_212A4_HC | . | . | . | . | . | . | . | . | . | . | . | . | . | . | . | . | . | . | . | . | . | . | . | . | . |
| 21-225_212C2_HC | . | . | . | . | . | . | . | . | . | . | . | . | . | . | . | . | . | . | . | . | . | . | . | . | . |
| 21-225_212F6_HC | . | . | . | . | . | . | . | . | . | . | . | . | . | . | . | . | . | . | . | . | . | . | . | . | . |
| 21-225_212G7_HC | . | . | . | . | . | . | . | . | . | . | T | . | . | . | . | . | . | . | . | . | . | . | . | . | . |
| 21-225_213D5_HC | . | . | . | . | . | . | . | . | . | . | . | . | . | . | F | . | . | . | . | . | . | . | V | . | . |
| 21-225_213G3_HC | . | . | . | . | . | . | . | . | . | . | . | . | . | . | H | . | . | . | . | . | . | . | . | . | . |
| 21-225_215D3_HC | . | . | . | . | . | . | . | . | . | . | . | . | . | . | . | . | . | . | . | . | . | . | . | . | . |
| 21-225_216A3_HC | . | . | . | . | . | . | . | . | . | . | . | . | . | . | . | . | . | . | . | . | . | . | . | . | . |
| 21-225_216G1_HC | . | . | . | . | . | . | . | . | . | . | . | . | . | . | . | . | . | . | . | . | . | . | . | . | . |
| 21-225_217B2_HC | . | . | . | . | . | . | . | . | . | . | . | . | . | . | . | . | . | . | . | . | . | . | . | . | . |
| 21-225_219A7_HC | . | . | . | . | . | . | . | . | . | . | . | . | M | . | . | . | . | . | . | . | . | . | . | . | . |
| 21-225_221G4_HC | . | . | . | . | . | . | . | . | . | . | . | . | . | . | H | . | . | . | . | . | . | . | . | . | . |
| 21-225_221H2_HC | . | . | . | . | . | . | . | . | . | . | . | . | . | . | H | . | . | . | . | . | . | . | . | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | H_CDR3 | | | | | | | | | | | | | | | | |
| CONSENSUS | T | A | V | Y | Y | C | A | R | G | D | W | N | P | - | - | - | - | - | - | - | - | - | - | - | - |
| 21-225_179C7_HC | . | . | . | . | . | . | . | . | D | R | H | Y | D | F | H | V | P | . | . | . | . | . | . | . | . |
| 21-225_210D12_HC | . | . | . | . | . | . | . | . | . | . | . | . | . | . | . | . | . | . | . | . | . | . | . | . | . |
| 21-225_210E12_HC | . | . | . | . | . | . | . | . | . | . | . | . | . | . | . | . | . | . | . | . | . | . | . | . | . |
| 21-225_211C1_HC | . | . | I | . | . | . | . | . | . | . | . | . | . | . | . | . | . | . | . | . | . | . | . | . | . |
| 21-225_211G5_HC | . | . | . | . | . | . | . | . | . | . | . | . | . | . | . | . | . | . | . | . | . | . | . | . | . |
| 21-225_212A4_HC | . | . | . | . | . | . | . | . | . | . | . | . | . | . | . | . | . | . | . | . | . | . | . | . | . |
| 21-225_212C2_HC | . | . | . | . | . | . | . | . | . | . | . | . | . | . | . | . | . | . | . | . | . | . | . | . | . |
| 21-225_212F6_HC | . | . | . | . | . | . | . | . | . | . | . | . | . | . | . | . | . | . | . | . | . | . | . | . | . |
| 21-225_212G7_HC | . | . | . | . | . | . | . | . | . | . | . | . | . | . | . | . | . | . | . | . | . | . | . | . | . |
| 21-225_213D5_HC | . | . | . | . | . | . | . | . | . | . | . | . | . | . | . | . | . | . | . | . | . | . | . | . | . |
| 21-225_213G3_HC | . | . | . | . | . | . | . | . | . | . | . | . | . | . | . | . | . | . | . | . | . | . | . | . | . |
| 21-225_215D3_HC | . | . | . | . | . | . | . | . | . | . | . | . | . | . | . | . | . | . | . | . | . | . | . | . | . |
| 21-225_216A3_HC | . | . | . | . | . | . | . | . | . | . | . | . | . | . | . | . | . | . | . | . | . | . | . | . | . |
| 21-225_216G1_HC | . | . | . | . | . | . | . | . | . | . | . | . | . | . | . | . | . | . | . | . | . | . | . | . | . |
| 21-225_217B2_HC | . | . | . | . | . | . | . | . | . | . | . | . | . | . | . | . | . | . | . | . | . | . | . | . | . |
| 21-225_219A7_HC | . | . | . | . | . | . | . | . | . | . | . | . | . | . | . | . | . | . | . | . | . | . | . | . | . |
| 21-225_221G4_HC | . | . | . | . | . | . | . | . | . | . | . | . | . | . | . | . | . | . | . | . | . | . | . | . | . |
| 21-225_221H2_HC | . | . | . | . | . | . | . | . | . | . | . | . | . | . | . | . | . | . | . | . | . | . | . | . | . |

**FIGURE 57**

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | | | | | | H_FR4 | | | | | |
| CONSENSUS | - | - | - | - | - | - | - | - | E | G | M | D | V | W | G | Q | G | T | T | V | T | V | S | S |
| 21-225_179C7_HC | . | . | . | . | Y | Y | Y | Y | Y | . | . | . | . | . | . | . | . | . | . | . | . | . | . | . |
| 21-225_210D12_HC | . | . | . | . | . | . | . | . | . | . | L | . | . | . | . | . | . | . | . | . | . | . | . | . |
| 21-225_210E12_HC | . | . | . | . | . | . | . | . | . | . | . | . | . | . | . | . | . | . | . | . | I | . | . | . |
| 21-225_211C1_HC | . | . | . | . | . | . | . | . | . | . | L | . | . | . | . | . | . | . | . | . | . | . | . | . |
| 21-225_211G5_HC | . | . | . | . | . | . | . | . | . | . | L | . | . | . | . | . | . | . | . | . | . | . | . | . |
| 21-225_212A4_HC | . | . | . | . | . | . | . | . | . | . | . | . | . | . | . | . | . | . | . | . | I | . | . | . |
| 21-225_212C2_HC | . | . | . | . | . | . | . | . | . | . | . | . | . | . | . | . | . | . | S | . | . | . | . | . |
| 21-225_212F6_HC | . | . | . | . | . | . | . | . | . | . | L | . | . | . | . | . | . | . | . | . | . | . | . | . |
| 21-225_212G7_HC | . | . | . | . | . | . | . | . | . | . | L | . | . | . | . | . | . | . | . | . | . | . | . | . |
| 21-225_213D5_HC | . | . | . | . | . | . | . | . | . | . | . | . | . | . | . | . | . | . | . | . | . | . | . | . |
| 21-225_213G3_HC | . | . | . | . | . | . | . | . | . | . | . | . | . | . | . | . | . | . | S | . | . | . | . | . |
| 21-225_215D3_HC | . | . | . | . | . | . | . | . | . | . | . | . | . | . | . | . | . | . | . | . | . | . | . | . |
| 21-225_216A3_HC | . | . | . | . | . | . | . | . | . | . | L | . | . | . | . | . | . | . | . | . | . | . | . | . |
| 21-225_216G1_HC | . | . | . | . | . | . | . | . | . | . | . | . | . | . | . | . | . | . | . | . | I | . | . | . |
| 21-225_217B2_HC | . | . | . | . | . | . | . | . | . | . | . | . | . | . | . | . | . | . | . | . | I | . | . | . |
| 21-225_219A7_HC | . | . | . | . | . | . | . | . | . | . | . | . | . | . | . | . | . | . | . | . | . | . | . | . |
| 21-225_221G4_HC | . | . | . | . | . | . | . | . | . | . | . | . | . | . | . | . | . | . | S | . | . | . | . | . |
| 21-225_221H2_HC | . | . | . | . | . | . | . | . | . | . | . | . | . | . | . | . | . | . | . | . | . | . | . | . |

**FIGURE 57**

Table 70. Consensus 36- VH3|3-23/D4|4-17|RF2/JH4 (SEQ ID NO: 50287):

EVQLLES-GGGLVQPGGSLRLSCAASG-FTFSS-----YAMSWVRQAPGKGLEWVSAISGS---GGNTFYADSVKGRFTISRDNSKNTLYLQMNSLRAEDTAVYYCAKKDYDYVW----------------GSPYFDYWGQGTLVTVSS wherein:

Y in position 39 can be substituted with S

S in position 42 can be substituted with T or N

A in position 57 can be substituted with V

S in position 59 can be substituted with I

R in position 61 can be substituted with S, N or F

G in position 66 can be substituted with S

N in position 67 can be substituted with R or S

T in position 68 can be substituted with A

F in position 69 can be substituted with Y

K in position 109 can be substituted with D or R

D in position 110 can be substituted with Y, H, M or R

Y in position 111 can be substituted with G or N

D in position 112 can be substituted with I, R or Y

Y in position 113 can be substituted with null (-), S or V

V in position 114 can be substituted with null (-), G, R or S

W in position 115 can be substituted with null (-) or I

null (-) in position 116 can be substitute with A

**FIGURE 57**

G in position 132 can be substituted with null (-) or V

S in position 133 can be substituted with null (-), A, T or Y

P in position 134 can be substituted with null (-), G or I

Y in position 135 can be substituted with F or T

Ser Xaa Ala Met Xaa (SEQ ID NO: 50055)

Xaa Ile Xaa Gly Xaa Gly Xaa Xaa Xaa Xaa Tyr Ala Asp Ser Val Lys Gly (SEQ ID NO: 50056)

Xaa Xaa Xaa Xaa Xaa Xaa Xaa Xaa Xaa Xaa Xaa Xaa Phe Asp Tyr (SEQ ID NO: 50057)

Ser Tyr Ala Met Ser (SEQ ID NO: 50531)

Ala Ile Ser Gly Ser Gly Gly Asn Thr Phe Tyr Ala Asp Ser Val Lys Gly (SEQ ID NO: 50532)

Lys Asp Tyr Asp Tyr Val Trp Gly Ser Pro Tyr Phe Asp Tyr (SEQ ID NO: 50533)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | H_FR1 | | | | | | | | | | | | | | | | | | | | | | | | |
| CONSENSUS | E | V | Q | L | L | E | S | - | G | G | G | L | V | Q | P | G | G | S | L | R | L | S | C | A | A |
| 21-225_146A6_HC | . | . | . | . | . | . | . | . | . | . | . | . | . | . | . | . | . | . | . | . | . | . | . | . | . |
| 21-225_147D10_HC | . | . | . | . | . | . | . | . | . | . | . | . | . | . | . | . | . | . | . | . | . | . | . | . | . |
| 21-225_147D12_HC | . | . | . | . | . | . | . | . | . | . | . | . | . | . | S | . | . | . | . | . | . | . | . | . | . |
| 21-225_148E2_HC | . | . | . | . | . | . | . | . | . | . | . | . | . | . | . | . | . | . | . | . | . | . | . | . | . |
| 21-225_149C6_HC | . | . | . | . | . | . | . | . | . | . | . | . | . | . | . | . | . | . | . | . | . | . | . | . | . |
| 21-225_149D11_HC | . | . | . | . | . | . | . | . | . | . | . | . | . | . | . | . | . | . | . | . | . | . | . | . | . |
| 21-225_149F8_HC | . | . | . | . | . | . | . | . | . | . | . | . | . | . | . | . | . | . | . | . | . | . | . | . | . |
| 21-225_150C5_HC | . | . | . | . | . | . | . | . | . | . | . | . | . | . | . | . | . | . | . | . | . | . | . | . | . |
| 21-225_152G10_HC | . | . | . | . | . | . | . | . | . | . | . | . | . | . | . | . | . | . | . | . | . | . | . | . | . |
| 21-225_152H7_HC | . | . | . | . | . | . | . | . | . | . | . | . | . | . | . | . | . | . | . | . | . | . | . | . | . |
| 21-225_155A4_HC | . | . | . | . | . | . | . | . | . | . | . | . | . | . | . | . | . | . | . | . | . | . | . | . | . |
| 21-225_155B4_HC | . | . | . | . | . | . | . | . | . | . | . | . | . | . | . | . | . | . | . | . | . | . | . | . | . |
| 21-225_178F7_HC | . | . | H | . | . | . | . | . | . | . | . | . | . | . | T | . | . | . | . | . | . | . | . | . | . |
| 21-225_179G1_HC | . | . | H | . | . | . | . | . | . | . | . | . | . | . | T | . | . | . | . | . | . | . | . | . | . |
| 21-225_190D12_HC | . | . | . | . | . | . | . | . | . | . | . | . | . | . | . | . | . | . | . | . | . | . | . | . | . |
| 21-225_197C8_HC | . | . | . | . | . | . | . | . | . | . | . | . | . | . | . | . | . | . | . | . | . | . | . | . | . |
| 21-225_55E1_HC | . | . | . | . | . | . | . | . | . | . | . | . | . | . | . | . | . | . | . | . | . | . | . | . | . |

# FIGURE 57

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | H_CDR1 | | | | | | | | | | H_FR2 | | | | | | | |
| CONSENSUS | S | G | - | F | T | F | S | S | - | - | - | - | - | Y | A | M | S | W | V | R | Q | A | P | G | K |
| 21-225_146A6_HC | . | . | . | . | . | . | N | . | . | . | . | . | . | . | . | . | N | . | . | . | . | . | . | . | . |
| 21-225_147D10_HC | . | . | . | . | . | . | . | . | . | . | . | . | . | . | . | . | . | . | . | . | . | . | . | . | . |
| 21-225_147D12_HC | . | . | . | . | . | . | . | . | . | . | . | . | . | . | . | . | . | . | . | . | . | . | . | . | . |
| 21-225_148E2_HC | . | . | . | . | . | . | . | . | . | . | . | . | . | . | . | . | . | . | . | . | . | . | . | . | . |
| 21-225_149C6_HC | . | . | . | . | . | . | . | . | . | . | . | . | . | . | . | . | . | . | . | . | . | . | . | . | . |
| 21-225_149D11_HC | . | . | . | . | . | . | . | . | . | . | . | . | . | . | . | . | . | . | . | . | . | . | . | . | . |
| 21-225_149F8_HC | . | . | . | . | . | . | . | . | . | . | . | . | . | . | . | . | . | . | . | . | . | . | . | . | . |
| 21-225_150C5_HC | . | . | . | . | . | . | . | . | . | . | . | . | . | . | . | . | . | . | . | . | . | . | . | . | . |
| 21-225_152G10_HC | . | . | . | . | . | . | . | . | . | . | . | . | . | . | . | . | . | . | . | . | . | . | . | . | . |
| 21-225_152H7_HC | . | . | . | . | . | . | N | . | . | . | . | . | . | . | . | . | N | . | . | . | . | . | . | . | . |
| 21-225_155A4_HC | . | . | . | . | . | . | N | . | . | . | . | . | . | . | . | . | . | . | . | . | . | . | . | . | . |
| 21-225_155B4_HC | . | . | . | . | . | . | . | . | . | . | . | . | . | . | . | . | . | . | . | . | . | . | . | . | . |
| 21-225_178F7_HC | . | . | . | . | . | . | . | . | . | . | . | . | . | . | . | . | T | . | . | . | . | . | . | . | . |
| 21-225_179G1_HC | . | . | . | . | . | . | . | . | . | . | . | . | . | . | . | . | T | . | . | . | . | . | . | . | . |
| 21-225_190D12_HC | . | . | . | . | . | . | . | . | . | . | . | . | . | . | . | . | . | . | . | . | . | . | . | . | . |
| 21-225_197C8_HC | . | . | . | . | . | . | . | . | . | . | . | . | . | S | . | . | T | . | . | . | . | . | . | . | . |
| 21-225_55E1_HC | . | . | . | . | . | . | . | . | . | . | . | . | . | . | . | . | . | . | . | . | . | . | . | . | . |

# FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | H_CDR2 | | | | | | | | | | | | | | | | | | |
| CONSENSUS | G | L | E | W | V | S | A | I | S | G | | - | - | - | G | G | N | T | F | Y | A | D | S | V | K |
| 21-225_146A6_HC | . | . | . | . | . | . | . | . | . | . | S | . | . | . | . | . | . | . | . | . | . | . | . | . | . |
| 21-225_147D10_HC | . | . | . | . | . | . | . | . | . | . | R | . | . | . | . | . | . | . | . | . | . | . | . | . | . |
| 21-225_147D12_HC | . | . | . | . | . | . | . | . | . | . | R | . | . | . | . | . | . | . | . | . | . | . | . | . | . |
| 21-225_148E2_HC | . | . | . | . | . | . | . | . | . | . | R | . | . | . | . | . | . | . | . | . | . | . | . | . | . |
| 21-225_149C6_HC | . | . | . | . | . | . | . | . | . | . | S | . | . | . | . | . | . | . | . | . | . | . | . | . | . |
| 21-225_149D11_HC | . | . | . | . | . | . | . | . | . | . | R | . | . | . | . | . | . | . | . | . | . | . | . | . | . |
| 21-225_149F8_HC | . | . | . | . | . | . | . | . | . | . | R | . | . | . | . | . | . | . | . | . | . | . | . | . | . |
| 21-225_150C5_HC | . | . | . | . | . | . | . | . | . | . | S | . | . | . | . | . | . | . | . | . | . | . | . | . | . |
| 21-225_152G10_HC | . | . | . | . | . | . | . | . | . | . | R | . | . | . | . | . | . | . | . | . | . | . | . | . | . |
| 21-225_152H7_HC | . | . | . | . | . | . | . | . | . | . | S | . | . | . | . | . | . | . | . | . | . | . | . | . | . |
| 21-225_155A4_HC | . | . | . | . | . | . | . | . | . | . | S | . | . | . | . | . | . | . | . | . | . | . | . | . | . |
| 21-225_155B4_HC | . | . | . | . | . | . | . | . | . | . | S | . | . | . | . | . | . | . | . | . | . | . | . | . | . |
| 21-225_178F7_HC | . | . | . | . | . | . | V | . | . | . | S | . | . | . | . | . | . | . | . | . | . | . | . | . | . |
| 21-225_179G1_HC | . | . | . | . | . | . | V | . | . | . | F | . | . | . | . | . | . | . | . | . | . | . | . | . | . |
| 21-225_190D12_HC | . | . | . | . | D | . | . | . | I | . | N | . | . | . | . | . | R | . | Y | . | . | . | . | . | . |
| 21-225_197C8_HC | . | . | . | . | . | . | . | . | I | . | N | . | . | . | . | . | R | A | Y | . | . | . | . | . | . |
| 21-225_55E1_HC | . | . | . | . | . | . | . | . | . | . | R | . | . | . | . | S | S | . | . | . | . | . | . | . | . |

FIGURE 57

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | H_FR3 | | | | | | | | | | | | | | | | | | | | | | | |
| CONSENSUS | G | R | F | T | I | S | R | D | N | S | K | N | T | L | Y | L | Q | M | N | S | L | R | A | E | D |
| 21-225_146A6_HC | . | . | . | . | . | . | . | . | . | . | . | . | . | . | . | . | . | . | . | . | . | . | . | . | . |
| 21-225_147D10_HC | . | . | . | . | . | . | . | . | . | . | . | . | . | . | . | . | . | . | . | . | . | . | . | . | . |
| 21-225_147D12_HC | . | . | . | . | . | . | . | . | . | . | . | . | . | . | . | . | . | . | . | . | . | . | . | . | . |
| 21-225_148E2_HC | . | . | . | . | . | . | . | . | . | . | . | . | . | . | . | . | . | . | . | . | . | . | . | . | . |
| 21-225_149C6_HC | . | . | . | . | . | . | . | . | . | . | . | . | . | . | . | . | H | . | . | . | . | . | . | . | . |
| 21-225_149D11_HC | . | . | . | . | . | . | . | . | . | . | . | . | . | . | . | R | . | . | . | . | . | . | . | . | . |
| 21-225_149F8_HC | . | . | . | . | . | . | . | . | . | . | . | . | . | . | . | . | . | . | . | . | . | . | . | . | . |
| 21-225_150C5_HC | . | . | . | . | . | . | . | . | . | . | . | . | . | . | . | . | . | . | . | . | . | . | . | . | . |
| 21-225_152G10_HC | . | . | . | . | . | . | . | . | . | . | . | . | . | . | . | . | . | . | . | . | . | . | . | . | . |
| 21-225_152H7_HC | . | . | . | . | . | . | . | . | . | . | . | . | . | . | . | . | . | . | . | . | . | . | . | . | . |
| 21-225_155A4_HC | . | . | . | . | . | . | . | . | . | . | . | . | . | . | . | . | . | . | . | . | . | . | . | . | . |
| 21-225_155B4_HC | . | . | . | . | . | . | . | . | . | . | . | . | . | . | . | . | . | . | . | . | . | . | . | . | . |
| 21-225_178F7_HC | . | . | . | . | . | . | . | . | . | . | . | . | . | . | . | . | . | . | . | . | . | . | . | . | . |
| 21-225_179G1_HC | . | . | . | . | . | . | . | . | . | . | . | . | . | . | . | . | . | . | . | . | . | . | . | . | . |
| 21-225_190D12_HC | . | . | . | . | . | . | . | . | . | . | . | . | . | . | . | . | . | . | . | . | . | . | . | . | . |
| 21-225_197C8_HC | . | . | . | . | . | . | . | . | . | . | . | . | . | . | . | . | . | . | . | . | . | . | T | . | . |
| 21-225_55E1_HC | . | . | . | . | . | . | . | . | . | . | . | . | . | . | . | . | . | . | . | . | . | . | . | . | . |

**FIGURE 57**

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | A | K | K | D | Y | D | Y | V | W | - | - | - | - | - | - | - | - | - | - |
| 21-225_146A6_HC | . | . | . | . | . | . | . | . | . | . | N | . | . | . | . | . | . | . | . | . | . | . | . | . | . |
| 21-225_147D10_HC | . | . | . | . | . | . | . | . | . | . | . | . | . | . | . | . | . | . | . | . | . | . | . | . | . |
| 21-225_147D12_HC | . | . | . | . | . | . | . | . | . | . | . | . | . | . | . | . | . | . | . | . | . | . | . | . | . |
| 21-225_148E2_HC | . | . | . | . | . | . | . | . | . | . | . | . | . | . | . | . | . | . | . | . | . | . | . | . | . |
| 21-225_149C6_HC | . | . | . | . | . | . | . | . | . | . | . | . | . | . | . | . | . | . | . | . | . | . | . | . | . |
| 21-225_149D11_HC | . | . | . | . | . | . | . | . | . | . | . | . | . | . | . | . | . | . | . | . | . | . | . | . | . |
| 21-225_149F8_HC | . | . | . | . | . | . | . | . | . | . | . | . | . | . | . | . | . | . | . | . | . | . | . | . | . |
| 21-225_150C5_HC | . | . | . | . | . | . | . | . | . | . | . | . | . | . | . | . | . | . | . | . | . | . | . | . | . |
| 21-225_152G10_HC | . | . | . | . | . | . | . | . | . | . | . | . | . | . | . | . | . | . | . | . | . | . | . | . | . |
| 21-225_152H7_HC | . | . | . | . | . | . | . | . | . | . | N | . | . | . | . | . | . | . | . | . | . | . | . | . | . |
| 21-225_155A4_HC | . | . | . | . | . | . | . | . | . | . | . | . | . | . | . | . | . | . | . | . | . | . | . | . | . |
| 21-225_155B4_HC | . | . | . | . | . | . | . | . | . | . | . | . | . | . | . | . | . | . | . | . | . | . | . | . | . |
| 21-225_178F7_HC | . | . | . | . | . | . | . | R | R | Y | G | . | - | - | - | . | . | . | . | . | . | . | . | . | . |
| 21-225_179G1_HC | . | . | . | . | . | . | . | R | R | Y | G | . | - | - | - | . | . | . | . | . | . | . | . | . | . |
| 21-225_190D12_HC | . | . | . | . | . | . | . | . | D | M | G | R | . | S | - | . | . | . | . | . | . | . | . | . | . |
| 21-225_197C8_HC | . | . | . | . | . | . | . | . | D | R | G | Y | S | R | I | A | . | . | . | . | . | . | . | . | . |
| 21-225_55E1_HC | . | . | . | F | . | . | . | . | D | H | G | I | V | G | - | . | . | . | . | . | . | . | . | . | . |

FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | H_FR4 | | | | | | | | | | |
| CONSENSUS | - | - | - | - | - | - | G | S | P | Y | F | D | Y | W | G | Q | G | T | L | V | T | V | S | S |
| 21-225_146A6_HC | . | . | . | . | . | . | . | . | . | . | . | . | . | . | . | . | . | . | . | . | . | . | . | . |
| 21-225_147D10_HC | . | . | . | . | . | . | . | . | . | . | . | . | . | . | . | . | . | . | . | . | . | . | . | . |
| 21-225_147D12_HC | . | . | . | . | . | . | . | . | . | . | . | . | . | . | . | . | . | . | . | . | . | . | . | . |
| 21-225_148E2_HC | . | . | . | . | . | . | . | . | . | . | . | . | . | . | . | . | . | . | . | . | . | . | . | . |
| 21-225_149C6_HC | . | . | . | . | . | . | . | . | . | . | . | . | . | . | . | . | . | . | . | . | . | . | . | . |
| 21-225_149D11_HC | . | . | . | . | . | . | . | . | . | . | . | . | . | . | . | . | . | . | . | . | . | . | . | . |
| 21-225_149F8_HC | . | . | . | . | . | . | . | . | . | . | . | . | . | . | . | . | . | . | . | . | . | . | . | . |
| 21-225_150C5_HC | . | . | . | . | . | . | . | . | . | . | . | . | . | . | . | . | . | . | . | . | . | . | . | . |
| 21-225_152G10_HC | . | . | . | . | . | . | . | . | . | . | . | . | . | . | . | . | . | . | . | . | . | . | . | . |
| 21-225_152H7_HC | . | . | . | . | . | . | . | . | . | . | . | . | . | . | . | . | . | . | . | . | . | . | . | . |
| 21-225_155A4_HC | . | . | . | . | . | . | . | . | . | . | . | . | . | . | . | . | . | . | . | . | . | . | . | . |
| 21-225_155B4_HC | . | . | . | . | . | . | . | . | . | . | . | . | . | . | . | . | . | . | . | . | . | . | . | . |
| 21-225_178F7_HC | . | . | . | . | . | . | - | - | - | . | . | . | . | . | . | . | . | . | . | . | . | . | . | . |
| 21-225_179G1_HC | . | . | . | . | . | . | - | - | - | . | . | . | . | . | . | . | . | . | . | . | . | . | . | . |
| 21-225_190D12_HC | . | . | . | . | . | . | - | Y | G | F | . | . | . | . | . | . | . | . | . | . | . | . | . | . |
| 21-225_197C8_HC | . | . | . | . | . | . | V | A | G | T | . | . | . | . | . | . | . | . | . | . | . | . | . | . |
| 21-225_55E1_HC | . | . | . | . | . | . | - | T | I | . | . | . | . | . | . | . | . | . | . | . | . | . | . | . |

**FIGURE 57**

Table 71. Consensus 37- VH1|1-02/D3|3-22|RF2/JH4 (SEQ ID NO: 50288):

QVQLVQS-GAEVKKPGASVKVSCKASG-YTFTG-----YYMHWVRQAPGQGLEWMGWINPN---SGGTNYAQKFQGRVTMTRDTSISTAYMELSRLRSDDTAVYYCARSYYYGSGS--------------YYNEFDYWGQGTLVTVSS wherein:

G in position 33 can be substituted with D

Y in position 39 can be substituted with H

Y in position 40 can be substituted with C

M in position 41 can be substituted with I

W in position 57 can be substituted with S

N in position 59 can be substituted with Y

P in position 60 can be substitute with R

N in position 61 can be substituted with K

G in position 67 can be substituted with A

T in position 68 can be substituted with A

N in position 69 can be substituted with D

Y in position 70 can be substituted with N or S

A in position 71 can be substituted with G or V

G in position 76 can be substituted with D or V

S in position 109 can be substituted with A, V or T

Y in position 110 can be substituted with F or N

Y in position 112 can be substituted with H

**FIGURE 57**

S in position 116 can be substituted with T

Y in position 133 can be substituted with H

E in position 135 can be substituted with G or D

Xaa Xaa Xaa Xaa His (SEQ ID NO: 50058)

Xaa Ile Xaa Xaa Xaa Ser Gly Xaa Xaa Xaa Xaa Xaa Gln Lys Phe Gln Xaa (SEQ ID NO: 50059)

Xaa Xaa Tyr Xaa Gly Ser Gly Xaa Tyr Xaa Asn Xaa Phe Asp Tyr (SEQ ID NO: 50060)

Gly Tyr Tyr Met His (SEQ ID NO: 50534)

Trp Ile Asn Pro Asn Ser Gly Gly Thr Asn Tyr Ala Gln Lys Phe Gln Gly (SEQ ID NO: 50535)

Ser Tyr Tyr Tyr Gly Ser Gly Ser Tyr Tyr Asn Glu Phe Asp Tyr (SEQ ID NO: 50536)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 |
|---|---|---|---|---|---|---|---|---|---|---|---|---|---|---|---|---|---|---|---|---|---|---|---|---|---|---|---|
| | H_FR1 | | | | | | | | | | | | | | | | | | | | | | | | | | |
| CONSENSUS | Q | V | Q | L | V | Q | S | - | G | A | E | V | K | K | P | G | A | S | V | K | V | S | C | K | A | S | G |
| 21-225_13F6_HC | . | . | . | . | . | . | . | . | . | . | . | . | . | . | . | . | . | . | . | . | . | . | . | . | . | . | . |
| 21-225_16F3_HC | . | . | . | . | . | . | . | . | . | . | . | . | . | . | . | . | . | . | . | . | . | . | . | . | . | . | . |
| 21-225_17D3_HC | . | . | . | . | . | . | . | . | . | . | . | . | . | . | . | . | . | . | . | . | . | . | . | . | . | . | . |
| 21-225_18E10_HC | . | . | . | . | . | . | . | . | . | . | . | . | . | . | . | . | . | . | . | . | . | . | . | . | . | . | . |
| 21-225_195E9_HC | . | . | . | V | . | . | . | . | . | . | . | . | . | . | . | . | . | . | . | . | . | . | . | . | . | . | . |
| 21-225_21D12_HC | . | . | . | . | . | . | . | . | . | . | . | . | . | . | . | . | . | . | . | . | . | . | . | . | . | . | . |
| 21-225_31D12_LC1_HC | . | . | K | . | . | . | . | . | . | . | . | . | . | . | . | . | . | . | . | . | . | . | . | . | . | . | . |
| 21-225_31D12_LC2_HC | . | . | K | . | . | . | . | . | . | . | . | . | . | . | . | . | . | . | . | . | . | . | . | . | . | . | . |
| 21-225_33E6_HC | . | . | K | . | . | . | . | . | . | . | . | . | . | . | . | . | . | . | . | . | . | . | . | . | . | . | . |
| 21-225_35E1_HC | . | . | K | . | . | . | . | . | . | . | . | . | . | . | . | . | . | . | . | . | . | . | . | . | . | . | . |
| 21-225_4B3_HC | . | . | . | . | . | . | . | . | . | . | . | . | . | . | . | . | . | . | . | . | . | . | . | . | . | . | . |
| 21-225_68G6_HC | . | . | . | . | . | . | P | . | . | G | . | . | . | . | . | . | S | . | . | . | . | . | . | . | . | . | . |
| 21-225_69B11_HC | . | . | . | . | . | . | . | . | . | . | . | . | . | . | . | . | . | . | . | R | . | . | . | . | . | . | . |
| 21-225_71D4_HC | . | . | . | . | . | . | . | . | . | . | . | . | . | . | . | . | . | . | . | . | . | . | . | . | . | . | . |
| 21-225_72B4_HC | . | . | . | . | . | . | . | . | . | . | . | . | . | . | . | . | . | . | . | . | . | . | . | . | . | . | . |
| 21-225_9G9_HC | . | . | . | . | . | . | . | . | . | . | . | . | . | . | . | . | . | . | . | . | . | . | . | . | . | . | . |

**FIGURE 57**

| Reference # | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 |
|---|---|---|---|---|---|---|---|---|---|---|---|---|---|---|---|---|---|---|---|---|---|---|---|---|---|---|---|
| | | | | | | H_CDR1 | | | | | | | | | | H_FR2 | | | | | | | | | | | |
| CONSENSUS | - | Y | T | F | T | G | - | - | - | - | - | Y | Y | M | H | W | V | R | Q | A | P | G | Q | G | L | E | W |
| 21-225_13F6_HC | . | . | . | . | . | . | . | . | . | . | . | . | . | . | . | . | . | . | . | . | . | . | . | . | . | . | . |
| 21-225_16F3_HC | . | . | . | . | . | . | . | . | . | . | . | H | . | . | . | . | . | . | . | . | . | . | . | . | . | . | . |
| 21-225_17D3_HC | . | . | . | . | . | . | . | . | . | . | . | . | . | . | . | . | . | . | . | . | . | . | . | . | . | . | . |
| 21-225_18E10_HC | . | . | . | . | . | . | . | . | . | . | . | . | . | . | . | . | . | . | . | . | . | . | . | . | . | . | . |
| 21-225_195E9_HC | . | . | . | . | . | . | . | . | . | . | . | . | . | . | . | . | . | . | . | . | . | . | . | . | . | . | . |
| 21-225_21D12_HC | . | . | . | . | . | D | . | . | . | . | . | . | . | I | . | . | . | . | . | . | . | . | . | . | . | . | . |
| 21-225_31D12_LC1_HC | . | . | . | . | . | . | . | . | . | . | . | . | . | . | . | . | . | . | . | . | . | . | . | . | . | . | . |
| 21-225_31D12_LC2_HC | . | . | . | . | . | . | . | . | . | . | . | . | . | . | . | . | . | . | . | . | . | . | . | . | . | . | . |
| 21-225_33E6_HC | . | . | . | . | . | . | . | . | . | . | . | . | . | . | . | . | . | . | . | . | . | . | . | . | . | . | . |
| 21-225_35E1_HC | . | . | . | . | . | . | . | . | . | . | . | . | . | . | . | . | . | . | . | . | . | . | . | . | . | . | . |
| 21-225_4B3_HC | . | . | . | . | . | . | . | . | . | . | . | . | . | . | . | . | . | . | . | . | . | . | . | . | . | . | . |
| 21-225_68G6_HC | . | . | . | . | . | . | . | . | . | . | . | . | . | . | . | . | . | . | . | . | . | . | . | . | . | . | . |
| 21-225_69B11_HC | . | . | . | . | . | . | . | . | . | . | . | . | . | . | . | . | . | . | . | . | . | . | . | . | . | . | . |
| 21-225_71D4_HC | . | . | . | . | . | . | . | . | . | . | . | . | C | . | . | . | . | . | . | . | . | . | . | . | . | . | . |
| 21-225_72B4_HC | . | . | . | . | . | . | . | . | . | . | . | . | . | . | . | . | . | . | . | . | . | . | . | . | . | . | . |
| 21-225_9G9_HC | . | . | . | . | . | . | . | . | . | . | . | . | . | I | . | . | . | . | . | . | . | . | . | . | . | . | . |

**FIGURE 57**

| Reference # | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 | 81 |
|---|---|---|---|---|---|---|---|---|---|---|---|---|---|---|---|---|---|---|---|---|---|---|---|---|---|---|---|
| | | | H_CDR2 | | | | | | | | | | | | | | | | | | | | | | | | |
| CONSENSUS | M | G | W | I | N | P | N | - | - | - | S | G | G | T | N | Y | A | Q | K | F | Q | G | R | V | T | M | T |
| 21-225_13F6_HC | . | . | . | . | . | . | . | . | . | . | . | . | . | A | . | . | . | . | . | . | . | . | . | . | . | . | . |
| 21-225_16F3_HC | . | . | . | . | . | . | . | . | . | . | . | . | . | . | . | . | . | . | . | . | . | . | . | . | . | . | . |
| 21-225_17D3_HC | . | . | . | . | . | . | . | . | . | . | . | . | . | . | . | . | . | . | . | . | . | . | . | . | . | . | . |
| 21-225_18E10_HC | . | . | . | . | . | . | . | . | . | . | . | . | . | . | . | . | . | . | . | . | . | . | . | . | . | . | . |
| 21-225_195E9_HC | . | . | . | . | . | . | . | . | . | . | . | . | A | . | . | . | . | . | . | . | . | . | . | . | . | . | . |
| 21-225_21D12_HC | . | . | . | . | . | . | . | . | . | . | . | . | . | . | . | N | . | . | . | . | . | . | . | . | . | . | . |
| 21-225_31D12_LC1_HC | . | . | . | . | . | . | . | . | . | . | . | . | . | . | . | . | V | . | . | . | . | . | . | . | . | . | . |
| 21-225_31D12_LC2_HC | . | . | . | . | . | . | . | . | . | . | . | . | . | . | . | . | V | . | . | . | . | . | . | . | . | . | . |
| 21-225_33E6_HC | . | . | . | . | . | . | . | . | . | . | . | . | . | . | . | . | . | . | . | . | . | . | . | . | . | . | . |
| 21-225_35E1_HC | . | . | . | . | . | . | K | . | . | . | . | . | . | . | . | . | . | . | . | . | . | . | . | . | . | . | . |
| 21-225_4B3_HC | . | . | . | . | . | . | . | . | . | . | . | . | . | . | . | S | . | . | . | . | . | . | . | . | . | . | . |
| 21-225_68G6_HC | . | . | S | . | Y | R | . | . | . | . | . | . | . | . | . | . | . | . | . | . | . | . | . | . | . | . | . |
| 21-225_69B11_HC | . | . | . | . | . | . | . | . | . | . | . | . | . | . | . | . | G | . | . | . | . | D | . | . | . | . | . |
| 21-225_71D4_HC | . | . | . | . | . | . | . | . | . | . | . | . | . | . | . | . | . | . | . | . | . | V | . | . | . | . | . |
| 21-225_72B4_HC | . | . | . | . | . | . | . | . | . | . | . | . | . | . | . | . | G | . | . | . | . | . | . | . | . | . | . |
| 21-225_9G9_HC | . | . | . | . | . | . | . | . | . | . | . | . | . | . | D | . | . | . | . | . | . | . | . | . | . | . | . |

# FIGURE 57

| Reference # | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 |
|---|---|---|---|---|---|---|---|---|---|---|---|---|---|---|---|---|---|---|---|---|---|---|---|---|---|---|---|
| | H_FR3 | | | | | | | | | | | | | | | | | | | | | | | | | | |
| CONSENSUS | R | D | T | S | I | S | T | A | Y | M | E | L | S | R | L | R | S | D | D | T | A | V | Y | Y | C | A | R |
| 21-225_13F6_HC | . | . | . | . | . | . | . | . | . | . | . | . | . | . | . | . | . | . | . | . | . | . | . | F | . | . | . |
| 21-225_16F3_HC | . | . | . | . | . | . | . | . | . | . | . | . | . | . | . | . | . | . | . | . | . | . | . | . | . | . | . |
| 21-225_17D3_HC | . | . | . | . | . | . | . | . | . | . | . | . | . | . | . | . | . | . | . | . | . | . | . | . | . | . | . |
| 21-225_18E10_HC | . | . | S | . | . | N | . | . | . | . | . | . | . | . | . | . | . | . | . | . | . | . | . | . | . | . | . |
| 21-225_195E9_HC | . | . | . | . | . | . | . | . | . | . | . | . | N | . | . | . | . | . | . | . | . | . | . | . | . | . | . |
| 21-225_21D12_HC | . | . | . | . | . | . | . | . | . | . | . | . | . | . | . | . | . | . | . | . | . | . | . | S | . | . | . |
| 21-225_31D12_LC1_HC | . | . | . | . | . | . | . | . | . | . | . | . | . | . | . | . | . | . | . | . | . | . | . | . | . | . | . |
| 21-225_31D12_LC2_HC | . | . | . | . | . | . | . | . | . | . | . | . | . | . | . | . | . | . | . | . | . | . | . | . | . | . | . |
| 21-225_33E6_HC | . | . | . | . | . | . | . | . | . | . | . | . | . | . | . | . | . | . | . | . | . | . | . | . | . | . | . |
| 21-225_35E1_HC | . | . | . | . | . | . | . | V | . | . | . | P | . | . | . | . | . | . | . | . | . | . | . | . | . | . | . |
| 21-225_4B3_HC | . | . | . | . | . | . | . | . | . | . | . | . | . | . | . | . | . | . | . | . | . | . | . | . | . | . | . |
| 21-225_68G6_HC | . | . | K | . | . | . | . | . | . | . | . | K | . | . | I | . | . | . | . | . | . | . | . | . | . | . | . |
| 21-225_69B11_HC | . | . | . | . | . | . | . | . | F | . | . | . | . | . | . | . | . | . | . | . | . | A | . | . | . | . | . |
| 21-225_71D4_HC | . | . | . | . | V | . | . | V | . | . | D | . | . | . | . | . | . | . | . | . | . | . | . | . | . | . | . |
| 21-225_72B4_HC | . | . | . | . | . | . | . | . | . | . | . | . | . | . | . | . | . | . | . | . | . | . | . | . | . | . | . |
| 21-225_9G9_HC | . | . | . | . | . | . | . | . | . | . | . | . | . | . | . | . | . | . | . | . | . | . | . | . | . | . | . |

**FIGURE 57**

| Reference # | 109 | 110 | 111 | 112 | 113 | 114 | 115 | 116 | 117 | 118 | 119 | 120 | 121 | 122 | 123 | 124 | 125 | 126 | 127 | 128 | 129 | 130 | 131 | 132 | 133 | 134 | 135 |
|---|---|---|---|---|---|---|---|---|---|---|---|---|---|---|---|---|---|---|---|---|---|---|---|---|---|---|---|
| | H_CDR3 | | | | | | | | | | | | | | | | | | | | | | | | | | |
| CONSENSUS | S | Y | Y | Y | G | S | G | S | - | - | - | - | - | - | - | - | - | - | - | - | - | - | - | Y | Y | N | E |
| 21-225_13F6_HC | . | F | . | . | . | . | . | T | . | . | . | . | . | . | . | . | . | . | . | . | . | . | . | . | . | . | . |
| 21-225_16F3_HC | . | . | . | . | . | . | . | T | . | . | . | . | . | . | . | . | . | . | . | . | . | . | . | . | . | . | . |
| 21-225_17D3_HC | A | N | . | . | . | . | . | . | . | . | . | . | . | . | . | . | . | . | . | . | . | . | . | . | . | . | D |
| 21-225_18E10_HC | . | . | . | . | . | . | . | . | . | . | . | . | . | . | . | . | . | . | . | . | . | . | . | . | . | . | . |
| 21-225_195E9_HC | A | . | . | . | . | . | . | T | . | . | . | . | . | . | . | . | . | . | . | . | . | . | . | . | . | . | . |
| 21-225_21D12_HC | . | . | . | . | . | . | . | T | . | . | . | . | . | . | . | . | . | . | . | . | . | . | . | . | . | . | . |
| 21-225_31D12_LC1_HC | V | . | . | . | . | . | . | . | . | . | . | . | . | . | . | . | . | . | . | . | . | . | . | . | . | . | . |
| 21-225_31D12_LC2_HC | V | . | . | . | . | . | . | . | . | . | . | . | . | . | . | . | . | . | . | . | . | . | . | . | . | . | . |
| 21-225_33E6_HC | V | . | . | . | . | . | . | . | . | . | . | . | . | . | . | . | . | . | . | . | . | . | . | . | . | . | . |
| 21-225_35E1_HC | V | . | . | H | . | . | . | . | . | . | . | . | . | . | . | . | . | . | . | . | . | . | . | . | . | . | . |
| 21-225_4B3_HC | . | . | . | . | . | . | . | . | . | . | . | . | . | . | . | . | . | . | . | . | . | . | . | . | . | . | . |
| 21-225_68G6_HC | A | F | . | . | . | . | . | T | . | . | . | . | . | . | . | . | . | . | . | . | . | . | . | . | . | . | . |
| 21-225_69B11_HC | T | . | . | . | . | . | . | . | . | . | . | . | . | . | . | . | . | . | . | . | . | . | . | . | . | . | G |
| 21-225_71D4_HC | A | . | . | . | . | . | . | T | . | . | . | . | . | . | . | . | . | . | . | . | . | . | . | . | H | . | . |
| 21-225_72B4_HC | T | . | . | . | . | . | . | . | . | . | . | . | . | . | . | . | . | . | . | . | . | . | . | . | . | . | G |
| 21-225_9G9_HC | . | . | . | . | . | . | . | T | . | . | . | . | . | . | . | . | . | . | . | . | . | . | . | . | . | . | . |

FIGURE 57

| Reference # | 136 | 137 | 138 | 139 | 140 | 141 | 142 | 143 | 144 | 145 | 146 | 147 | 148 | 149 |
|---|---|---|---|---|---|---|---|---|---|---|---|---|---|---|
| | | | | H_FR4 | | | | | | | | | | |
| CONSENSUS | F | D | Y | W | G | Q | G | T | L | V | T | V | S | S |
| 21-225_13F6_HC | . | . | . | . | . | . | . | . | . | . | . | . | . | . |
| 21-225_16F3_HC | . | . | . | . | . | . | . | . | . | . | . | . | . | . |
| 21-225_17D3_HC | . | . | . | . | . | . | . | . | . | . | . | . | . | . |
| 21-225_18E10_HC | . | . | . | . | . | . | . | . | . | . | . | . | . | . |
| 21-225_195E9_HC | . | . | . | . | . | . | . | . | . | . | . | . | . | . |
| 21-225_21D12_HC | . | . | . | . | . | . | . | . | . | . | . | . | . | . |
| 21-225_31D12_LC1_HC | . | . | . | . | . | . | . | . | . | . | . | . | . | . |
| 21-225_31D12_LC2_HC | . | . | . | . | . | . | . | . | . | . | . | . | . | . |
| 21-225_33E6_HC | . | . | . | . | . | . | . | . | . | . | . | . | . | . |
| 21-225_35E1_HC | . | . | . | . | . | . | . | . | . | . | . | . | . | . |
| 21-225_4B3_HC | . | . | . | . | . | . | . | . | . | . | . | . | . | . |
| 21-225_68G6_HC | . | . | . | . | . | . | . | . | . | . | . | . | . | . |
| 21-225_69B11_HC | . | . | . | . | . | . | . | . | . | . | . | . | . | . |
| 21-225_71D4_HC | . | . | . | . | . | . | . | S | . | . | . | . | . | . |
| 21-225_72B4_HC | . | . | . | . | . | . | . | . | . | . | . | . | . | . |
| 21-225_9G9_HC | . | . | . | . | . | . | . | . | . | . | . | . | . | . |

**FIGURE 57**

Table 72. Consensus 38 - VH1|1-02/D4|4-23|RF2/JH6 (SEQ ID NO: 50289):

QVQLVQS-GAEVKKPGASVKVSCKASG-YTFTG-----YYIHWVRQAPGQGLEWMGWINPY---SGDTNYAQKFQGRVTMTRDTSISTAYMELSRLRFDDTAVFYCARDWGGYSS----------------YYYGMDVWGQGTTVTVSS wherein:

I in position 41 can be substituted with T

D in position 67 can be substituted with G

N in position 69 can be substituted with K

Y in position 70 can be substituted with S

Y in position 134 can be substituted with F

Gly Tyr Tyr Xaa His (SEQ ID NO: 50061)

Trp Ile Asn Pro Tyr Ser Gly Xaa Thr Xaa Xaa Ala Gln Lys Phe Gln Gly (SEQ ID NO: 50062)

Asp Trp Gly Gly Tyr Ser Ser Tyr Tyr Xaa Gly Met Asp Val (SEQ ID NO: 50236)

Gly Tyr Tyr Ile His (SEQ ID NO: 50537)

Trp Ile Asn Pro Tyr Ser Gly Asp Thr Asn Tyr Ala Gln Lys Phe Gln Gly (SEQ ID NO: 50538)

Asp Trp Gly Gly Tyr Ser Ser Tyr Tyr Tyr Gly Met Asp Val (SEQ ID NO: 50539)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | Q | S | - | G | A | E | V | K | K | P | G | A | S | V | K | V | S | C | K | A | S |
| 21-225_224A7_HC | . | . | . | . | . | . | . | . | . | . | . | . | . | . | . | . | . | . | . | . | . | . | . | . | . | . |
| 21-225_224C11_HC | . | . | . | . | . | . | . | . | . | . | . | . | . | . | . | . | . | . | . | . | . | . | . | . | . | . |
| 21-225_224G1_HC | . | . | . | . | . | . | . | . | . | . | . | . | . | . | . | . | . | . | . | . | . | . | . | . | . | . |
| 21-225_224H11_HC | . | . | . | . | . | . | . | . | . | . | . | . | . | . | . | . | . | . | . | . | . | . | . | . | . | . |
| 21-225_225G4_HC | . | . | . | . | . | . | . | . | . | . | . | . | . | . | . | . | . | . | . | . | . | . | . | . | . | . |
| 21-225_226E11_HC | . | . | . | . | . | . | . | . | . | . | . | . | . | . | . | . | . | . | . | . | . | . | . | . | . | . |
| 21-225_226F10_HC | . | . | . | . | . | . | . | . | . | G | . | . | . | . | L | R | . | . | . | . | . | . | . | . | . | . |
| 21-225_226F9_HC | . | . | . | . | . | . | . | . | . | . | . | . | . | . | . | . | . | . | . | . | . | . | . | . | . | . |
| 21-225_226G8_HC | . | . | . | . | . | . | . | . | . | . | . | . | . | . | . | . | . | . | . | . | . | . | . | . | . | . |
| 21-225_226H12_HC | . | . | . | . | . | . | . | . | . | . | . | . | . | . | . | . | . | . | . | . | . | . | . | . | . | . |
| 21-225_226H9_HC | . | . | . | . | . | . | . | . | . | . | . | . | . | . | . | . | . | . | . | . | . | . | . | . | . | . |
| 21-225_227A8_HC | . | . | . | . | . | . | . | . | . | . | . | . | . | . | . | . | . | . | . | . | . | . | . | . | . | . |
| 21-225_227C1_HC | . | . | . | . | . | . | . | . | . | . | . | . | . | . | . | . | . | . | . | . | . | . | . | . | . | . |
| 21-225_227F2_HC | . | . | H | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | Y | T | F | T | G | - | - | - | - | - | Y | Y | I | H | W | V | R | Q | A | P | G | Q | G | L |
| 21-225_224A7_HC | . | . | . | . | . | . | . | . | . | . | . | . | . | . | . | . | . | . | . | . | . | . | . | . | . | . |
| 21-225_224C11_HC | . | . | . | . | . | . | . | . | . | . | . | . | . | . | . | . | . | . | . | . | . | . | . | . | . | . |
| 21-225_224G1_HC | . | . | . | . | . | . | . | . | . | . | . | . | . | . | . | . | . | . | . | . | . | . | . | . | . | . |
| 21-225_224H11_HC | . | . | . | . | . | . | . | . | . | . | . | . | . | . | . | . | . | . | . | . | T | . | . | . | . | . |
| 21-225_225G4_HC | . | . | . | . | . | . | . | . | . | . | . | . | . | . | . | . | . | . | . | . | . | . | . | . | . | . |
| 21-225_226E11_HC | . | . | . | . | . | . | . | . | . | . | . | . | . | . | . | . | . | . | . | . | . | . | . | . | . | . |
| 21-225_226F10_HC | . | . | . | . | . | . | . | . | . | . | . | . | . | . | . | . | . | . | . | . | . | . | . | . | . | . |
| 21-225_226F9_HC | . | . | . | . | . | . | . | . | . | . | . | . | . | . | . | . | . | . | . | . | . | . | . | . | . | . |
| 21-225_226G8_HC | . | . | . | . | . | . | . | . | . | . | . | . | . | . | . | . | . | . | . | . | . | . | . | . | . | . |
| 21-225_226H12_HC | . | . | . | . | . | . | . | . | . | . | . | . | . | . | . | . | . | . | . | . | . | . | . | . | . | . |
| 21-225_226H9_HC | . | . | . | . | . | . | . | . | . | . | . | . | . | . | . | . | . | . | . | . | . | . | . | . | . | . |
| 21-225_227A8_HC | . | . | . | . | . | . | . | . | . | . | . | . | . | . | . | . | . | . | . | . | . | . | . | . | . | . |
| 21-225_227C1_HC | . | . | . | . | . | . | . | . | . | . | . | . | . | . | T | . | . | . | . | . | . | . | . | . | . | . |
| 21-225_227F2_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | M | G | W | I | N | P | Y | - | - | - | S | G | D | T | N | Y | A | Q | K | F | Q | G | R | V |
| 21-225_224A7_HC | . | . | . | . | . | . | . | . | . | . | . | . | . | . | . | . | . | . | . | . | . | . | . | . | . | . |
| 21-225_224C11_HC | . | . | . | . | . | . | . | . | . | . | . | . | . | . | . | . | . | . | . | . | . | . | . | . | . | . |
| 21-225_224G1_HC | . | . | . | . | . | . | . | . | . | . | . | . | . | . | . | . | . | . | . | . | . | . | . | . | . | . |
| 21-225_224H11_HC | . | . | . | . | . | . | . | . | . | . | . | . | . | . | . | . | . | . | . | . | . | . | . | . | . | . |
| 21-225_225G4_HC | . | . | . | . | . | . | . | . | . | . | . | . | . | . | . | . | . | . | . | . | . | . | . | . | . | . |
| 21-225_226E11_HC | . | . | . | . | . | . | . | . | . | . | . | . | . | . | . | . | . | . | . | . | . | . | . | . | . | . |
| 21-225_226F10_HC | . | . | . | . | . | . | . | . | . | . | . | . | . | . | . | . | . | . | . | . | . | . | . | . | . | . |
| 21-225_226F9_HC | . | . | . | . | . | . | . | . | . | . | . | . | . | . | . | . | K | . | . | . | . | . | . | . | . | . |
| 21-225_226G8_HC | . | . | . | . | . | . | . | . | . | . | . | . | . | . | . | . | K | . | . | . | . | . | . | . | . | . |
| 21-225_226H12_HC | . | . | . | . | . | . | . | . | . | . | . | . | . | . | . | . | . | . | . | . | . | . | . | . | . | . |
| 21-225_226H9_HC | . | . | . | . | . | . | . | . | . | . | . | . | . | . | . | . | . | S | . | . | . | . | . | . | . | . |
| 21-225_227A8_HC | . | . | . | . | . | . | . | . | . | . | . | . | . | . | . | . | . | . | . | . | . | . | . | . | . | . |
| 21-225_227C1_HC | . | . | . | . | . | . | . | . | . | . | . | . | . | . | G | . | . | . | . | . | . | . | . | . | . | . |
| 21-225_227F2_HC | . | . | . | . | . | . | . | . | . | . | . | . | . | . | . | . | K | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | M | T | R | D | T | S | I | S | T | A | Y | M | E | L | S | R | L | R | F | D | D | T | A | V | F |
| 21-225_224A7_HC | . | . | . | . | . | . | . | . | T | . | . | . | . | . | . | . | . | . | . | . | . | . | . | . | . | . |
| 21-225_224C11_HC | . | . | . | . | . | . | . | . | . | . | . | . | . | . | . | R | . | . | . | . | . | . | . | . | . | . |
| 21-225_224G1_HC | . | . | . | . | . | . | . | . | . | . | . | . | . | . | . | . | . | . | . | . | . | . | . | . | . | . |
| 21-225_224H11_HC | . | . | . | . | . | . | . | . | . | . | . | . | . | . | . | R | . | . | . | . | . | . | . | . | . | . |
| 21-225_225G4_HC | . | . | . | . | . | . | . | . | . | . | . | . | . | . | . | R | . | . | . | . | . | . | . | . | . | . |
| 21-225_226E11_HC | . | . | . | . | . | . | . | . | . | . | . | . | . | . | . | . | . | . | . | . | . | . | . | . | . | . |
| 21-225_226F10_HC | . | . | . | . | . | . | . | V | . | . | . | . | . | . | . | . | . | . | . | L | . | . | . | . | . | . |
| 21-225_226F9_HC | . | . | . | . | A | . | . | . | . | . | . | . | . | . | . | . | . | . | . | . | . | . | . | . | . | . |
| 21-225_226G8_HC | . | . | . | . | . | . | . | . | . | . | . | . | . | . | . | . | . | . | . | . | . | . | . | . | . | . |
| 21-225_226H12_HC | . | . | . | . | . | . | . | . | . | . | . | . | . | . | . | R | . | . | . | . | . | . | . | . | . | . |
| 21-225_226H9_HC | . | . | . | . | . | . | . | . | . | . | . | . | . | . | . | . | . | . | . | . | . | . | . | . | . | . |
| 21-225_227A8_HC | . | . | . | . | . | . | . | V | . | . | . | . | . | . | . | . | . | . | . | . | . | . | . | . | . | . |
| 21-225_227C1_HC | . | . | . | . | . | . | . | . | . | . | . | . | . | . | . | . | . | . | . | . | . | . | . | . | . | Y |
| 21-225_227F2_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | D | W | G | G | Y | S | S | - | - | - | - | - | - | - | - | - | - | - | - | - | - | - |
| 21-225_224A7_HC | . | . | . | . | . | . | . | . | . | . | . | . | . | . | . | . | . | . | . | . | . | . | . | . | . | . |
| 21-225_224C11_HC | . | . | . | . | . | . | . | . | . | . | . | . | . | . | . | . | . | . | . | . | . | . | . | . | . | . |
| 21-225_224G1_HC | . | . | . | . | . | . | . | . | . | . | . | . | . | . | . | . | . | . | . | . | . | . | . | . | . | . |
| 21-225_224H11_HC | . | . | . | . | . | . | . | . | . | . | . | . | . | . | . | . | . | . | . | . | . | . | . | . | . | . |
| 21-225_225G4_HC | . | . | . | . | . | . | . | . | . | . | . | . | . | . | . | . | . | . | . | . | . | . | . | . | . | . |
| 21-225_226E11_HC | . | . | . | . | . | . | . | . | . | . | . | . | . | . | . | . | . | . | . | . | . | . | . | . | . | . |
| 21-225_226F10_HC | . | . | . | . | . | . | . | . | . | . | . | . | . | . | . | . | . | . | . | . | . | . | . | . | . | . |
| 21-225_226F9_HC | . | . | . | . | . | . | . | . | . | . | . | . | . | . | . | . | . | . | . | . | . | . | . | . | . | . |
| 21-225_226G8_HC | . | . | . | . | . | . | . | . | . | . | . | . | . | . | . | . | . | . | . | . | . | . | . | . | . | . |
| 21-225_226H12_HC | . | . | . | . | . | . | . | . | . | . | . | . | . | . | . | . | . | . | . | . | . | . | . | . | . | . |
| 21-225_226H9_HC | . | . | . | . | . | . | . | . | . | . | . | . | . | . | . | . | . | . | . | . | . | . | . | . | . | . |
| 21-225_227A8_HC | . | . | . | . | . | . | . | . | . | . | . | . | . | . | . | . | . | . | . | . | . | . | . | . | . | . |
| 21-225_227C1_HC | . | . | . | . | . | . | . | . | . | . | . | . | . | . | . | . | . | . | . | . | . | . | . | . | . | . |
| 21-225_227F2_HC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | Y | Y | Y | G | M | D | V | W | G | Q | G | T | T | V | T | V | S | S |
| 21-225_224A7_HC | . | . | . | . | . | . | . | . | . | . | . | . | . | . | . | . | . | . | . |
| 21-225_224C11_HC | . | . | . | F | . | . | . | . | . | . | . | . | . | . | . | . | . | . | . |
| 21-225_224G1_HC | . | . | . | . | . | . | . | . | . | . | . | . | . | . | . | . | . | . | . |
| 21-225_224H11_HC | . | . | . | . | . | . | . | . | . | . | . | . | . | . | . | . | . | . | . |
| 21-225_225G4_HC | . | . | . | . | . | . | . | . | . | . | . | . | . | . | . | . | . | . | . |
| 21-225_226E11_HC | . | . | . | . | . | . | . | . | . | . | . | . | . | . | . | . | . | . | . |
| 21-225_226F10_HC | . | . | . | . | . | . | . | . | . | . | . | . | . | P | . | . | . | . | . |
| 21-225_226F9_HC | . | . | . | . | . | . | . | . | . | . | . | . | . | . | . | . | . | . | . |
| 21-225_226G8_HC | . | . | . | . | . | . | . | . | . | . | . | . | . | . | . | . | . | . | . |
| 21-225_226H12_HC | . | . | . | . | . | . | . | . | . | . | . | . | . | . | . | . | . | . | . |
| 21-225_226H9_HC | . | . | . | . | . | . | . | . | . | . | . | . | . | . | . | . | . | . | . |
| 21-225_227A8_HC | . | . | . | . | . | . | . | . | . | . | . | . | . | . | . | . | . | . | . |
| 21-225_227C1_HC | . | . | . | . | . | . | . | . | . | . | . | . | . | . | . | . | . | . | . |
| 21-225_227F2_HC | . | . | . | . | . | . | . | . | . | . | . | . | . | . | . | . | . | . | . |

**FIGURE 57**

Table 73. Consensus 39 - VH3|3-21/D7|7-27|RF1/JH4 (SEQ ID NO: 50290):

EVQLVES-GGGLVKPGGSLRLSCAASG-FTFSS-----YSMNWVRQAPGKGLEWVSSISGS---SSYIYYADSVKGRFTISRDNAKNSLYLQMNSLRAEDTAVYYCARLT------------------------FDYWGQGTLVTVSS wherein:

S in position 33 can be substituted with T

Y in position 39 can be substituted with F

S in position 40 can be substituted with T, N or G

N in position 42 can be substituted with S or I

G in position 60 can be substituted with S

S in position 61 can be substituted with T

S in position 65 can be substituted with I or N

S in position 66 can be substituted with N, T or Y

I in position 68 can be substituted with M or S

Y in position 69 can be substituted with N

A in position 71 can be substituted with T

Xaa Xaa Xaa Met Xaa (SEQ ID NO: 50063)

Ser Ile Ser Xaa Xaa Xaa Xaa Tyr Xaa Xaa Tyr Xaa Asp Ser Val Lys Gly (SEQ ID NO: 50064)

Leu Thr Phe Asp Tyr (SEQ ID NO: 50065)

Ser Tyr Ser Met Asn (SEQ ID NO: 50540)

Ser Ile Ser Gly Ser Ser Ser Tyr Ile Tyr Tyr Ala Asp Ser Val Lys Gly (SEQ ID NO: 50541)

Leu Thr Phe Asp Tyr (SEQ ID NO: 50542)

FIGURE 57

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | E | V | Q | L | V | E | S | - | G | G | G | L | V | K | P | G | G | S | L | R | L | S | C | A | A | S |
| 21-225_10C7_HC | . | . | . | . | . | . | . | . | . | . | . | . | . | . | . | . | . | . | . | . | . | . | . | . | . | . |
| 21-225_12D2_HC | . | . | . | . | . | . | . | . | . | . | . | . | . | . | . | . | . | . | . | . | . | . | . | . | . | . |
| 21-225_151F1_HC | . | . | . | . | . | . | . | . | . | . | . | . | . | . | . | . | . | . | . | . | . | . | . | . | . | . |
| 21-225_166H12_HC | . | . | . | . | . | . | . | . | . | . | A | . | . | . | . | . | . | . | . | . | . | . | . | . | . | . |
| 21-225_227F11_HC | . | . | . | . | . | . | . | . | . | . | . | . | . | . | . | . | . | . | . | . | . | . | . | . | V | . |
| 21-225_26G1_HC | . | . | . | . | . | . | . | . | . | . | . | . | . | . | . | . | . | . | . | . | . | . | . | . | . | . |
| 21-225_2B1_HC | . | . | . | . | . | . | . | . | . | . | . | . | . | . | . | . | . | . | . | . | . | . | . | . | . | . |
| 21-225_33B7_HC | . | . | . | . | . | . | . | . | . | . | . | . | . | . | . | . | . | . | . | . | . | . | . | . | . | . |
| 21-225_34C11_HC | . | . | . | . | . | . | . | . | . | . | . | . | . | . | . | . | . | . | . | . | . | . | . | . | . | . |
| 21-225_43A4_HC | . | . | . | . | . | . | . | . | . | . | . | . | . | . | . | . | . | . | . | . | . | . | . | . | . | . |
| 21-225_45D9_HC | . | . | . | . | . | . | . | . | . | . | . | . | . | . | . | . | . | . | . | . | . | . | . | . | . | . |
| 21-225_54H3_HC | . | . | . | . | . | . | . | . | . | . | . | . | . | . | . | . | . | . | . | . | . | . | . | . | . | . |
| 21-225_63H8_HC | . | . | . | . | . | . | . | . | . | . | . | . | . | . | . | . | . | . | . | . | . | . | . | . | . | . |
| 21-225_8D8_HC | . | . | . | . | . | . | . | . | . | . | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | S | M | N | W | V | R | Q | A | P | G | K | G | L |
| 21-225_10C7_HC | . | . | . | . | . | . | . | . | . | . | . | . | . | . | . | . | . | . | . | . | . | . | . | . | . | . |
| 21-225_12D2_HC | . | . | . | . | . | . | . | . | . | . | . | . | . | . | . | . | . | . | . | . | . | . | . | . | . | . |
| 21-225_151F1_HC | . | . | Y | . | . | R | . | . | . | . | . | . | F | . | . | . | . | . | . | . | . | . | . | . | . | . |
| 21-225_166H12_HC | . | . | . | . | . | . | . | . | . | . | . | . | . | . | . | S | . | . | . | . | . | . | . | . | . | . |
| 21-225_227F11_HC | . | . | . | . | . | . | T | . | . | . | . | . | . | . | . | . | . | . | . | . | . | . | . | . | . | . |
| 21-225_26G1_HC | . | . | . | . | . | . | . | . | . | . | . | . | . | G | . | . | . | . | . | . | . | . | . | . | . | . |
| 21-225_2B1_HC | . | . | . | . | . | . | . | . | . | . | . | . | . | . | . | . | . | . | . | . | . | . | . | . | . | . |
| 21-225_33B7_HC | . | . | . | . | . | . | . | . | . | . | . | . | . | N | . | . | . | . | . | . | . | . | . | . | . | . |
| 21-225_34C11_HC | . | . | . | . | . | . | . | . | . | . | . | . | . | T | . | S | . | . | . | . | . | . | . | . | . | . |
| 21-225_43A4_HC | . | . | . | . | . | . | . | . | . | . | . | . | . | T | . | . | . | . | . | . | V | . | . | . | . | . |
| 21-225_45D9_HC | . | . | . | . | . | . | . | . | . | . | . | . | . | T | . | . | . | . | . | . | V | . | . | . | . | . |
| 21-225_54H3_HC | . | . | . | . | . | . | . | . | . | . | . | . | . | . | . | I | . | . | . | . | . | . | . | . | . | . |
| 21-225_63H8_HC | . | . | . | . | . | . | . | . | . | . | . | . | . | N | . | . | . | . | . | . | . | . | . | . | . | . |
| 21-225_8D8_HC | . | . | . | . | . | . | . | . | . | . | . | . | . | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | S | S | I | S | G | S | - | - | - | S | S | Y | I | Y | Y | A | D | S | V | K | G | R | F |
| 21-225_10C7_HC | . | . | . | . | . | . | . | S | . | . | . | . | N | N | . | . | . | . | . | . | . | . | . | . | . | . |
| 21-225_12D2_HC | . | . | . | . | . | . | . | S | . | . | . | . | . | . | . | . | . | . | . | . | . | . | . | . | . | . |
| 21-225_151F1_HC | . | . | . | . | . | . | . | S | . | . | . | . | . | Y | . | . | . | . | . | . | . | . | . | . | . | . |
| 21-225_166H12_HC | G | . | . | . | . | . | . | . | . | . | . | . | . | . | . | S | . | . | . | . | . | . | . | . | . | . |
| 21-225_227F11_HC | . | . | . | . | . | . | . | S | . | . | . | . | I | N | . | M | . | . | . | . | . | . | . | . | . | . |
| 21-225_26G1_HC | . | . | . | . | . | . | . | . | . | . | . | . | . | . | . | . | . | . | . | . | . | . | . | . | . | . |
| 21-225_2B1_HC | . | . | . | . | . | . | . | S | . | . | . | . | . | . | . | . | . | . | . | . | . | . | . | . | . | . |
| 21-225_33B7_HC | . | . | . | . | . | . | . | . | . | . | . | . | . | . | . | . | . | . | . | . | . | . | . | . | . | . |
| 21-225_34C11_HC | . | . | . | . | . | . | . | . | . | . | . | . | . | N | . | . | . | . | . | . | . | . | . | . | . | . |
| 21-225_43A4_HC | . | . | . | . | . | . | . | . | . | . | . | . | . | T | . | . | . | . | . | . | . | . | . | . | . | . |
| 21-225_45D9_HC | . | . | . | . | . | . | . | . | . | . | . | . | . | T | . | . | . | . | . | . | . | . | . | . | . | . |
| 21-225_54H3_HC | . | . | . | . | . | . | . | . | T | . | . | . | . | . | . | . | . | . | . | . | . | . | . | . | . | . |
| 21-225_63H8_HC | . | . | . | . | . | . | . | . | . | . | . | . | . | T | . | M | N | . | T | . | . | . | . | . | . | . |
| 21-225_8D8_HC | . | . | . | . | . | . | . | S | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | A | K | N | S | L | Y | L | Q | M | N | S | L | R | A | E | D | T | A | V | Y |
| 21-225_10C7_HC | . | . | . | . | . | . | . | . | . | . | . | . | . | . | . | . | . | . | . | . | . | . | S | . | . | . |
| 21-225_12D2_HC | . | . | . | . | . | . | . | . | . | . | . | . | . | . | . | . | . | . | . | . | . | . | . | . | . | . |
| 21-225_151F1_HC | . | . | . | . | . | . | . | . | . | . | . | . | . | . | . | . | . | . | . | . | . | . | . | . | . | . |
| 21-225_166H12_HC | . | . | . | . | . | . | . | . | . | . | . | . | . | . | . | . | . | . | . | . | . | . | . | . | . | . |
| 21-225_227F11_HC | . | . | . | . | . | . | . | . | . | . | . | . | . | . | . | . | . | . | . | . | . | . | S | . | . | . |
| 21-225_26G1_HC | . | . | . | . | . | . | . | . | . | . | . | . | . | . | . | . | . | . | . | . | . | . | . | . | . | . |
| 21-225_2B1_HC | . | . | . | . | . | . | . | . | . | . | . | . | . | . | . | . | . | . | . | . | . | . | . | . | . | . |
| 21-225_33B7_HC | . | . | . | . | . | . | . | . | . | . | . | . | . | . | . | . | . | . | . | . | . | . | . | . | . | . |
| 21-225_34C11_HC | . | . | . | . | . | . | . | . | . | . | . | . | . | . | . | . | . | . | . | . | . | . | . | . | . | . |
| 21-225_43A4_HC | . | . | . | . | . | D | . | Q | . | . | . | . | . | . | . | . | . | . | . | G | . | . | . | . | . | . |
| 21-225_45D9_HC | . | . | . | . | . | D | . | Q | . | . | . | . | . | . | . | . | . | . | . | G | . | . | . | . | . | . |
| 21-225_54H3_HC | . | . | . | . | . | . | . | . | . | . | . | . | . | . | . | . | . | . | . | . | . | . | . | . | . | . |
| 21-225_63H8_HC | . | . | . | . | . | . | . | . | . | . | . | . | . | . | . | . | . | . | . | . | . | . | . | . | . | . |
| 21-225_8D8_HC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | L | T | - | - | - | - | - | - | - | - | - | - | - | - | - | - | - | - | - | - | - | - |
| 21-225_10C7_HC | . | . | . | . | . | G | . | . | . | . | . | . | . | . | . | . | . | . | . | . | . | . | . | . | . | . |
| 21-225_12D2_HC | . | . | . | . | . | G | . | . | . | . | . | . | . | . | . | . | . | . | . | . | . | . | . | . | . | . |
| 21-225_151F1_HC | . | . | . | . | D | . | P | . | . | . | . | . | . | . | . | . | . | . | . | . | . | . | . | . | . | . |
| 21-225_166H12_HC | . | . | . | . | . | . | G | . | . | . | . | . | . | . | . | . | . | . | . | . | . | . | . | . | . | . |
| 21-225_227F11_HC | . | . | . | . | . | G | . | . | . | . | . | . | . | . | . | . | . | . | . | . | . | . | . | . | . | . |
| 21-225_26G1_HC | . | . | . | . | . | . | T | . | . | . | . | . | . | . | . | . | . | . | . | . | . | . | . | . | . | . |
| 21-225_2B1_HC | . | . | . | . | . | G | . | . | . | . | . | . | . | . | . | . | . | . | . | . | . | . | . | . | . | . |
| 21-225_33B7_HC | . | . | . | . | . | N | G | . | . | . | . | . | . | . | . | . | . | . | . | . | . | . | . | . | . | . |
| 21-225_34C11_HC | . | . | . | . | . | . | G | . | . | . | . | . | . | . | . | . | . | . | . | . | . | . | . | . | . | . |
| 21-225_43A4_HC | . | . | . | . | V | . | S | . | . | . | . | . | . | . | . | . | . | . | . | . | . | . | . | . | . | . |
| 21-225_45D9_HC | . | . | . | . | V | . | S | . | . | . | . | . | . | . | . | . | . | . | . | . | . | . | . | . | . | . |
| 21-225_54H3_HC | . | . | . | G | M | . | T | . | . | . | . | . | . | . | . | . | . | . | . | . | . | . | . | . | . | . |
| 21-225_63H8_HC | . | . | . | . | . | R | N | . | . | . | . | . | . | . | . | . | . | . | . | . | . | . | . | . | . | . |
| 21-225_8D8_HC | . | . | . | . | . | G | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | - | F | D | Y | W | G | Q | G | T | L | V | T | V | S | S |
| 21-225_10C7_HC | . | . | . | . | . | - | V | . | . | . | . | . | . | . | . | . | . | . | . |
| 21-225_12D2_HC | . | . | . | . | . | - | . | . | . | . | . | . | . | . | . | A | . | . | . |
| 21-225_151F1_HC | . | . | . | . | . | L | V | . | . | . | . | . | . | . | . | . | . | . | . |
| 21-225_166H12_HC | . | . | . | . | . | . | . | . | . | . | . | . | . | . | . | . | . | . | . |
| 21-225_227F11_HC | . | . | . | . | . | - | V | . | . | . | . | . | . | . | . | . | . | . | . |
| 21-225_26G1_HC | . | . | . | . | . | . | . | F | . | . | . | . | . | . | . | . | . | . | . |
| 21-225_2B1_HC | . | . | . | . | . | - | N | . | . | . | . | . | . | . | . | . | . | . | . |
| 21-225_33B7_HC | . | . | . | . | . | . | . | . | . | . | . | . | . | . | . | . | . | . | . |
| 21-225_34C11_HC | . | . | . | . | . | . | . | . | . | . | . | . | . | . | . | . | . | . | . |
| 21-225_43A4_HC | . | . | . | . | . | . | . | . | . | . | . | . | A | . | . | . | . | . | . |
| 21-225_45D9_HC | . | . | . | . | . | . | . | . | . | . | . | . | A | . | . | . | . | . | . |
| 21-225_54H3_HC | . | . | . | . | . | - | V | I | . | . | . | . | . | . | . | . | . | . | . |
| 21-225_63H8_HC | . | . | . | . | . | . | . | . | . | . | . | . | . | . | . | . | . | . | . |
| 21-225_8D8_HC | . | . | . | . | . | - | N | . | . | . | . | . | . | . | . | . | . | . | . |

**FIGURE 57**

Table 74. Consensus 40 - VH3|3-33/D3|3-9|RF2/JH4 (SEQ ID NO: 50291):

QVQLVES-GGGVVQPGRSLRLSCAASG-FTFST-----YGMHWVRQAPGKGLEWVAIIWYD---GTNKYYADSVKGRFTISRDNSKNTLYLQLNSLRAEDTAVYYCARDPLRGYN-----------------DPVMDYWGQGTLVTVSS wherein:

T in position 33 can be substituted with S

I in position 57 can be substituted with V

T in position 66 can be substituted with S

K in position 75 can be substituted with R

M in position 136 can be substituted with L

Xaa Tyr Gly Met His (SEQ ID NO: 50066)

Xaa Ile Trp Tyr Asp Gly Xaa Asn Lys Tyr Tyr Ala Asp Ser Val Xaa Gly (SEQ ID NO: 50067)

Asp Pro Leu Arg Gly Tyr Asn Asp Pro Val Xaa Asp Tyr (SEQ ID NO: 50068)

Thr Tyr Gly Met His (SEQ ID NO: 50543)

Ile Ile Trp Tyr Asp Gly Thr Asn Lys Tyr Tyr Ala Asp Ser Val Lys Gly (SEQ ID NO: 50544)

Asp Pro Leu Arg Gly Tyr Asn Asp Pro Val Met Asp Tyr (SEQ ID NO: 50545)

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | E | S | - | G | G | G | V | V | Q | P | G | R | S | L | R | L | S | C | A | A | S |
| 21-225_169B1_HC | . | . | . | . | . | . | . | . | . | . | . | . | . | . | . | . | . | . | . | . | . | . | . | . | . | . |
| 21-225_169F9_HC | . | . | . | . | . | . | . | . | . | . | . | . | . | . | . | . | . | . | . | . | . | . | . | . | . | . |
| 21-225_170D11_HC | . | . | . | . | . | . | . | . | . | . | . | . | . | . | . | . | . | . | . | . | . | . | . | V | . | . |
| 21-225_170D5_HC | . | . | . | . | . | . | . | . | . | . | . | . | . | . | . | . | . | . | . | . | . | . | . | . | . | . |
| 21-225_170G4_HC | . | . | . | . | . | . | . | . | . | . | . | . | . | . | . | . | . | . | . | . | . | . | . | T | . | . |
| 21-225_170G5_HC | . | . | . | . | . | . | . | . | . | . | . | . | . | . | . | . | G | . | . | . | . | . | . | . | . | . |
| 21-225_171A4_HC | . | . | . | . | . | . | . | . | . | . | . | . | . | . | . | . | G | . | . | . | . | . | . | . | . | . |
| 21-225_171C3_HC | . | . | . | . | . | . | . | . | . | . | . | . | . | . | . | . | G | . | . | . | . | . | . | . | . | . |
| 21-225_172B3_HC | . | . | . | . | . | . | . | . | . | . | . | . | . | . | . | . | . | P | . | . | . | . | . | . | . | . |
| 21-225_172G8_HC | . | . | . | M | . | . | . | . | . | . | . | . | . | . | . | . | . | . | . | . | . | . | . | . | . | . |
| 21-225_173H12_HC | . | . | . | . | . | . | . | . | . | . | . | . | . | . | . | . | . | . | . | . | . | . | . | . | . | . |
| 21-225_175G1_HC | . | . | . | . | . | . | . | . | . | . | . | . | . | . | . | . | G | . | . | . | . | . | . | . | . | . |
| 21-225_176B11_HC | . | . | . | . | . | . | . | . | . | . | . | . | . | . | . | . | . | . | . | . | . | . | . | . | . | . |
| 21-225_178B10_HC | . | . | . | . | . | . | . | . | . | . | . | . | . | . | . | . | . | . | . | . | . | . | . | V | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | T | - | - | - | - | - | Y | G | M | H | W | V | R | Q | A | P | G | K | G | L |
| 21-225_169B1_HC | . | . | . | . | . | . | S | . | . | . | . | . | . | . | . | . | . | . | . | . | . | . | . | . | . | . |
| 21-225_169F9_HC | . | . | . | . | . | . | . | . | . | . | . | . | . | . | . | . | . | . | . | . | . | . | . | . | . | . |
| 21-225_170D11_HC | . | . | . | . | . | . | S | . | . | . | . | . | . | . | . | . | . | . | . | . | . | . | . | . | . | . |
| 21-225_170D5_HC | . | . | . | . | . | . | S | . | . | . | . | . | . | . | . | . | . | . | . | . | . | . | . | . | . | . |
| 21-225_170G4_HC | . | . | . | . | . | . | . | . | . | . | . | . | . | . | . | . | . | . | . | . | . | . | . | . | . | . |
| 21-225_170G5_HC | . | . | . | . | . | . | . | . | . | . | . | . | . | . | . | . | . | . | . | . | . | . | . | . | . | . |
| 21-225_171A4_HC | . | . | . | . | . | . | . | . | . | . | . | . | . | . | . | . | . | . | . | . | . | . | . | . | . | . |
| 21-225_171C3_HC | . | . | . | . | . | . | . | . | . | . | . | . | . | . | . | . | . | . | . | . | . | . | . | . | . | . |
| 21-225_172B3_HC | . | . | . | . | . | . | . | . | . | . | . | . | . | . | . | . | . | . | . | . | . | . | . | . | . | . |
| 21-225_172G8_HC | . | . | . | . | . | . | . | . | . | . | . | . | . | . | . | . | . | . | . | . | . | . | . | . | . | . |
| 21-225_173H12_HC | . | . | . | . | . | . | S | . | . | . | . | . | . | . | . | . | . | . | . | . | . | . | . | . | . | . |
| 21-225_175G1_HC | . | . | . | . | . | . | . | . | . | . | . | . | . | . | . | . | . | . | . | . | . | . | . | . | . | . |
| 21-225_176B11_HC | . | . | . | . | . | . | . | . | . | . | . | . | . | . | . | . | . | . | . | . | . | . | . | . | . | . |
| 21-225_178B10_HC | . | . | . | . | S | . | . | . | . | . | . | . | . | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | A | I | I | W | Y | D | - | - | - | G | T | N | K | Y | Y | A | D | S | V | K | G | R | F |
| 21-225_169B1_HC | . | . | . | . | V | . | . | . | . | . | . | . | . | S | . | . | . | . | . | . | . | . | R | . | . | . |
| 21-225_169F9_HC | . | . | . | S | . | . | . | . | . | . | . | . | . | . | . | . | . | . | . | . | . | . | . | . | . | . |
| 21-225_170D11_HC | . | . | . | . | . | . | . | . | . | . | . | . | . | . | . | . | . | . | . | . | . | . | . | . | . | . |
| 21-225_170D5_HC | . | . | . | . | . | . | . | . | . | . | . | . | . | . | . | . | . | . | . | . | . | . | . | . | . | . |
| 21-225_170G4_HC | . | . | . | S | . | . | . | . | . | . | . | . | . | . | . | . | . | . | . | . | . | . | . | . | . | . |
| 21-225_170G5_HC | . | . | . | T | . | . | . | . | . | . | . | . | . | . | . | . | . | . | . | . | . | . | . | . | . | . |
| 21-225_171A4_HC | . | . | . | . | . | . | . | . | . | . | . | . | . | S | . | . | . | . | . | . | . | . | . | . | . | . |
| 21-225_171C3_HC | . | . | . | T | . | . | . | . | . | . | . | . | . | . | . | . | . | . | . | . | . | . | . | . | . | . |
| 21-225_172B3_HC | . | . | . | . | V | . | . | . | . | . | . | . | . | S | . | . | . | . | . | . | . | . | . | . | . | . |
| 21-225_172G8_HC | . | . | M | . | . | . | . | . | . | . | . | . | . | . | . | . | . | . | . | . | . | . | . | . | . | . |
| 21-225_173H12_HC | . | . | . | . | . | . | . | . | . | . | . | . | . | . | . | . | . | . | . | . | . | . | . | . | . | . |
| 21-225_175G1_HC | . | . | . | . | V | . | . | . | . | . | . | . | . | S | . | . | . | . | . | . | . | . | . | . | . | . |
| 21-225_176B11_HC | . | . | . | S | . | . | . | . | . | . | . | . | . | . | . | . | . | . | . | . | . | . | . | . | . | . |
| 21-225_178B10_HC | . | . | M | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | L | N | S | L | R | A | E | D | T | A | V | Y |
| 21-225_169B1_HC | . | . | . | . | . | . | . | . | . | . | . | . | . | . | M | . | . | . | . | . | . | . | . | . | . | . |
| 21-225_169F9_HC | . | . | . | . | . | . | . | . | . | . | . | . | . | . | . | . | . | . | . | . | . | . | . | . | . | . |
| 21-225_170D11_HC | . | . | . | . | . | . | . | . | . | . | . | . | . | . | . | . | . | . | . | . | . | . | . | . | . | . |
| 21-225_170D5_HC | . | . | . | . | . | . | . | . | . | . | . | . | . | . | . | . | . | . | . | . | . | . | . | . | . | . |
| 21-225_170G4_HC | . | . | . | . | . | . | . | . | . | . | . | F | . | . | . | . | . | . | . | . | . | . | . | . | M | . |
| 21-225_170G5_HC | . | . | . | . | . | . | . | . | S | . | . | . | . | . | M | . | . | . | . | . | . | . | . | . | . | . |
| 21-225_171A4_HC | . | . | . | . | . | . | . | . | . | . | . | . | . | . | M | . | . | . | . | . | . | . | . | . | . | . |
| 21-225_171C3_HC | . | . | . | . | . | . | . | . | . | . | . | . | . | . | M | . | . | . | . | . | . | . | . | . | . | . |
| 21-225_172B3_HC | . | . | . | . | . | . | . | . | . | . | . | . | . | . | M | . | . | . | . | . | . | . | . | . | . | . |
| 21-225_172G8_HC | . | . | . | . | . | . | . | . | . | . | . | . | . | . | . | . | . | . | . | . | . | . | . | . | . | . |
| 21-225_173H12_HC | . | . | . | . | . | . | . | . | . | . | . | . | . | . | . | . | . | . | . | . | . | . | . | . | . | . |
| 21-225_175G1_HC | . | . | . | . | . | . | . | . | . | . | . | . | V | . | M | . | . | . | . | . | . | . | . | . | . | . |
| 21-225_176B11_HC | . | . | . | . | . | . | . | . | . | . | . | F | . | . | . | . | . | . | . | . | . | . | . | . | . | . |
| 21-225_178B10_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | D | P | L | R | G | Y | N | - | - | - | - | - | - | - | - | - | - | - | - | - | - | - |
| 21-225_169B1_HC | . | . | . | . | . | . | . | . | . | . | . | . | . | . | . | . | . | . | . | . | . | . | . | . | . | . |
| 21-225_169F9_HC | . | . | . | . | . | . | . | . | . | . | . | . | . | . | . | . | . | . | . | . | . | . | . | . | . | . |
| 21-225_170D11_HC | . | . | . | . | . | . | . | . | . | . | . | . | . | . | . | . | . | . | . | . | . | . | . | . | . | . |
| 21-225_170D5_HC | . | . | . | . | . | . | . | . | . | . | . | . | . | . | . | . | . | . | . | . | . | . | . | . | . | . |
| 21-225_170G4_HC | . | . | . | . | . | . | . | . | . | . | . | . | . | . | . | . | . | . | . | . | . | . | . | . | . | . |
| 21-225_170G5_HC | [illegible] | . | . | . | . | . | . | . | . | . | . | . | . | . | . | . | . | . | . | . | . | . | . | . | . | . |
| 21-225_171A4_HC | . | . | . | . | . | . | . | . | . | . | . | . | . | . | . | . | . | . | . | . | . | . | . | . | . | . |
| 21-225_171C3_HC | . | . | . | . | . | . | . | . | . | . | . | . | . | . | . | . | . | . | . | . | . | . | . | . | . | . |
| 21-225_172B3_HC | . | . | . | . | . | . | . | . | . | . | . | . | . | . | . | . | . | . | . | . | . | . | . | . | . | . |
| 21-225_172G8_HC | . | . | . | . | . | . | . | . | . | . | . | . | . | . | . | . | . | . | . | . | . | . | . | . | . | . |
| 21-225_173H12_HC | . | . | . | . | . | . | . | . | . | . | . | . | . | . | . | . | . | . | . | . | . | . | . | . | . | . |
| 21-225_175G1_HC | . | . | . | . | . | . | . | . | . | . | . | . | . | . | . | . | . | . | . | . | . | . | . | . | . | . |
| 21-225_176B11_HC | . | . | . | . | . | . | . | . | . | . | . | . | . | . | . | . | . | . | . | . | . | . | . | . | . | . |
| 21-225_178B10_HC | . | . | . | . | . | . | . | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | D | P | V | M | D | Y | W | G | Q | G | T | L | V | T | V | S | S |
| 21-225_169B1_HC | . | . | . | . | . | . | . | . | . | . | . | . | . | . | . | . | . | . | . |
| 21-225_169F9_HC | . | . | . | . | . | . | . | . | . | . | . | . | . | . | . | . | . | . | . |
| 21-225_170D11_HC | . | . | . | . | . | . | . | . | . | . | . | . | . | . | . | . | . | . | . |
| 21-225_170D5_HC | . | . | . | . | . | . | . | . | . | . | . | . | . | . | . | . | . | . | . |
| 21-225_170G4_HC | . | . | . | . | . | . | . | . | . | . | . | . | . | . | . | . | . | . | . |
| 21-225_170G5_HC | . | . | . | . | . | . | . | . | . | . | . | . | . | . | . | . | . | . | . |
| 21-225_171A4_HC | . | . | . | . | . | . | . | . | . | . | . | . | . | . | . | . | . | . | . |
| 21-225_171C3_HC | . | . | . | . | . | . | . | . | . | . | . | . | . | . | . | . | . | . | . |
| 21-225_172B3_HC | . | . | . | . | . | . | . | . | . | . | . | . | . | . | . | . | . | . | . |
| 21-225_172G8_HC | . | . | . | . | . | . | . | . | . | . | . | . | . | . | . | . | . | . | . |
| 21-225_173H12_HC | . | . | . | . | . | . | . | . | . | . | . | . | . | . | . | . | . | . | . |
| 21-225_175G1_HC | . | . | . | . | . | . | . | . | . | . | . | . | . | . | . | . | . | . | . |
| 21-225_176B11_HC | . | . | . | . | . | L | . | . | . | . | . | . | . | . | . | . | . | . | . |
| 21-225_178B10_HC | . | . | . | . | . | . | . | . | . | . | . | . | . | . | . | . | . | . | . |

**FIGURE 57**

Table 75. Consensus 41 - VH3|3-23/D4|4-17|RF2/JH5 (SEQ ID NO: 50292):

EVQLLES-GGGLVQPGGSLRLSCAASG-FTFSS-----YAMSWVRQAPGKGLEWVSAISGR---GGNTFYADSVKGRFTISRDNSKNTLYLQMNSLRAEDTAVYYCAKRVTDYGG-----------------NDWFDPWGQGTLVTVSS wherein:

S in position 33 can be substituted with N

S in position 42 can be substituted with N or T

R in position 61 can be substituted with S

G in position 66 can be substituted with K

Xaa Tyr Ala Met Xaa (SEQ ID NO: 50069)

Ala Ile Ser Gly Xaa Gly Xaa Asn Thr Phe Tyr Ala Asp Ser Val Lys Gly (SEQ ID NO: 50070)

Arg Val Thr Asp Tyr Gly Gly Asn Asp Trp Phe Asp Pro (SEQ ID NO: 50071)

Ser Tyr Ala Met Ser (SEQ ID NO: 50546)

Ala Ile Ser Gly Arg Gly Gly Asn Thr Phe Tyr Ala Asp Ser Val Lys Gly (SEQ ID NO: 50547)

Arg Val Thr Asp Tyr Gly Gly Asn Asp Trp Phe Asp Pro (SEQ ID NO: 50548)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | E | V | Q | L | L | E | S | - | G | G | G | L | V | Q | P | G | G | S | L | R | L | S | C | A | A | S |
| 21-225_146E9_HC | . | . | . | . | . | . | . | . | . | . | . | . | . | . | . | . | . | . | . | . | . | . | . | . | . | . |
| 21-225_146H1_HC | . | . | . | . | . | . | . | . | . | . | . | . | . | . | . | . | . | . | . | . | . | . | . | . | . | . |
| 21-225_148C4_HC | . | . | . | . | . | . | . | . | . | . | . | . | . | . | . | . | . | . | . | . | . | . | . | . | . | . |
| 21-225_148H9_HC | . | . | . | . | . | . | . | . | . | . | . | . | . | . | . | . | . | . | . | . | . | . | . | . | . | . |
| 21-225_149A3_HC | . | . | . | . | . | . | . | . | . | . | . | . | . | . | . | . | . | . | . | . | . | . | . | . | . | . |
| 21-225_150C11_HC | . | . | . | . | . | . | . | . | . | . | . | . | . | . | . | . | . | . | . | . | . | . | . | . | . | . |
| 21-225_150C12_HC | . | . | . | . | . | . | . | . | . | . | . | . | . | . | . | . | . | . | . | . | . | . | . | . | . | . |
| 21-225_151B12_HC | . | . | . | . | . | . | . | . | . | . | . | . | . | . | . | . | . | . | . | . | . | . | . | . | . | . |
| 21-225_152B11_HC | . | . | . | . | . | . | . | . | . | . | . | . | . | . | . | . | . | . | . | . | . | . | . | . | . | . |
| 21-225_152D2_HC | . | . | . | . | . | . | . | . | . | . | . | . | . | . | . | . | . | . | . | . | . | . | . | . | . | . |
| 21-225_152G4_HC | . | . | . | . | . | . | . | . | . | . | . | . | . | . | . | . | . | . | . | . | . | . | . | . | . | . |
| 21-225_155C4_HC | . | . | . | . | . | . | . | . | . | . | . | . | . | . | . | . | . | . | . | . | . | . | . | . | . | . |
| 21-225_156E4_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | A | M | S | W | V | R | Q | A | P | G | K | G | L |
| 21-225_146E9_HC | . | . | . | . | . | . | . | . | . | . | . | . | . | . | . | . | . | . | . | . | . | . | . | . | . | . |
| 21-225_146H1_HC | . | . | . | . | . | . | . | . | . | . | . | . | . | . | . | . | . | . | . | . | . | . | . | . | . | . |
| 21-225_148C4_HC | . | . | . | . | . | . | . | . | . | . | . | . | . | . | . | N | . | . | . | . | . | . | . | . | . | . |
| 21-225_148H9_HC | . | . | . | . | . | . | . | . | . | . | . | . | . | . | . | . | . | . | . | . | . | . | . | . | . | . |
| 21-225_149A3_HC | . | . | . | . | . | . | N | . | . | . | . | . | . | . | . | T | . | . | . | . | . | . | . | . | . | . |
| 21-225_150C11_HC | . | . | . | . | . | . | . | . | . | . | . | . | . | . | . | N | . | . | . | . | . | . | . | . | . | . |
| 21-225_150C12_HC | . | . | . | . | . | . | . | . | . | . | . | . | . | . | . | . | . | . | . | . | . | . | . | . | . | . |
| 21-225_151B12_HC | . | . | . | . | . | . | . | . | . | . | . | . | . | . | . | N | . | . | . | H | . | . | . | . | . | . |
| 21-225_152B11_HC | . | . | . | . | . | . | . | . | . | . | . | . | . | . | . | N | . | . | . | . | . | . | . | . | . | . |
| 21-225_152D2_HC | . | . | . | . | . | . | . | . | . | . | . | . | . | . | . | . | . | . | . | . | . | . | . | . | . | . |
| 21-225_152G4_HC | . | . | . | . | . | . | . | . | . | . | . | . | . | . | . | . | . | . | . | . | . | . | . | . | . | . |
| 21-225_155C4_HC | . | . | . | . | . | . | . | . | . | . | . | . | . | . | . | . | . | . | . | . | . | . | . | . | . | . |
| 21-225_156E4_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | S | A | I | S | G | R | - | - | - | G | G | N | T | F | Y | A | D | S | V | K | G | R | F |
| 21-225_146E9_HC | . | . | . | . | . | . | . | . | . | . | . | . | . | . | . | . | . | . | . | . | . | . | . | . | . | . |
| 21-225_146H1_HC | . | . | . | . | . | . | . | . | . | . | . | . | . | . | . | . | . | . | . | . | . | . | . | . | . | . |
| 21-225_148C4_HC | . | . | . | . | . | . | . | . | S | . | . | . | . | . | . | . | . | . | . | . | . | . | . | . | . | . |
| 21-225_148H9_HC | . | . | . | . | . | . | . | . | . | . | . | . | . | . | . | . | . | . | . | . | . | . | . | . | . | . |
| 21-225_149A3_HC | . | . | . | . | . | . | . | . | . | . | . | . | . | . | . | . | . | . | . | . | . | . | . | . | . | . |
| 21-225_150C11_HC | . | . | . | . | . | . | . | . | S | . | . | . | . | . | . | . | . | . | . | . | . | . | . | . | . | . |
| 21-225_150C12_HC | . | . | . | . | . | . | . | . | . | . | . | . | . | . | . | . | . | . | . | . | . | . | . | . | . | . |
| 21-225_151B12_HC | . | . | . | . | . | . | . | . | S | . | . | . | . | . | . | . | . | . | . | . | . | . | . | . | . | . |
| 21-225_152B11_HC | . | . | . | . | . | . | . | . | . | . | . | . | . | . | . | . | . | . | . | . | . | . | . | . | . | . |
| 21-225_152D2_HC | . | . | . | . | . | . | . | . | . | . | . | . | . | . | . | . | . | . | . | . | . | . | . | . | . | . |
| 21-225_152G4_HC | . | . | . | . | . | . | . | . | . | . | . | . | . | . | . | . | . | . | . | . | . | . | . | . | . | . |
| 21-225_155C4_HC | . | . | . | . | . | . | . | . | . | . | . | . | . | . | . | . | . | . | . | . | . | . | . | . | . | . |
| 21-225_156E4_HC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_146E9_HC | . | . | . | . | . | . | . | . | K | . | . | . | . | . | . | . | . | . | . | . | . | . | . | . | . | . |
| 21-225_146H1_HC | . | . | . | . | . | . | . | . | . | . | . | . | . | . | . | . | . | . | . | . | . | . | . | . | . | . |
| 21-225_148C4_HC | . | . | . | . | . | . | . | . | . | . | . | . | . | . | . | . | . | . | . | . | . | . | . | . | . | . |
| 21-225_148H9_HC | . | . | . | . | . | . | . | . | . | . | . | . | . | . | . | . | . | . | . | . | . | . | . | . | . | . |
| 21-225_149A3_HC | . | . | . | . | . | . | . | . | . | . | . | . | . | . | . | . | . | . | . | . | . | . | . | . | . | . |
| 21-225_150C11_HC | . | . | . | . | . | . | . | . | . | . | . | N | . | . | . | S | . | . | . | . | . | . | . | . | . | . |
| 21-225_150C12_HC | . | . | . | . | . | . | . | . | . | . | . | . | . | . | . | . | . | . | . | . | . | . | . | . | . | . |
| 21-225_151B12_HC | . | . | . | . | . | . | . | . | . | . | . | . | . | . | . | . | . | . | . | . | . | . | . | . | . | . |
| 21-225_152B11_HC | . | . | . | . | . | . | . | . | . | . | . | . | . | . | . | . | . | . | . | . | . | . | . | . | . | . |
| 21-225_152D2_HC | . | . | . | . | . | . | . | . | K | . | . | . | . | . | . | . | . | . | . | . | . | . | . | . | . | . |
| 21-225_152G4_HC | . | . | . | . | . | . | . | . | . | . | . | . | . | . | . | . | . | . | . | . | . | . | . | . | . | . |
| 21-225_155C4_HC | . | . | . | . | . | . | . | . | . | . | . | . | . | . | . | . | . | . | . | . | . | . | . | . | . | . |
| 21-225_156E4_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | K | R | V | T | D | Y | G | G | - | - | - | - | - | - | - | - | - | - | - | - | - | - | - |
| 21-225_146E9_HC | . | . | . | . | . | . | . | . | . | . | . | . | . | . | . | . | . | . | . | . | . | . | . | . | . | . |
| 21-225_146H1_HC | . | . | . | . | . | . | . | . | . | . | . | . | . | . | . | . | . | . | . | . | . | . | . | . | . | . |
| 21-225_148C4_HC | . | . | . | . | . | . | . | . | . | . | . | . | . | . | . | . | . | . | . | . | . | . | . | . | . | . |
| 21-225_148H9_HC | . | . | . | . | . | . | . | . | . | . | . | . | . | . | . | . | . | . | . | . | . | . | . | . | . | . |
| 21-225_149A3_HC | . | . | T | . | . | . | . | . | . | . | . | . | . | . | . | . | . | . | . | . | . | . | . | . | . | . |
| 21-225_150C11_HC | . | . | . | . | . | . | . | . | . | . | . | . | . | . | . | . | . | . | . | . | . | . | . | . | . | . |
| 21-225_150C12_HC | . | . | . | . | . | . | . | . | . | . | . | . | . | . | . | . | . | . | . | . | . | . | . | . | . | . |
| 21-225_151B12_HC | . | . | . | . | . | . | . | . | . | . | . | . | . | . | . | . | . | . | . | . | . | . | . | . | . | . |
| 21-225_152B11_HC | . | . | . | . | . | . | . | . | . | . | . | . | . | . | . | . | . | . | . | . | . | . | . | . | . | . |
| 21-225_152D2_HC | . | . | . | . | . | . | . | . | . | . | . | . | . | . | . | . | . | . | . | . | . | . | . | . | . | . |
| 21-225_152G4_HC | . | . | . | . | . | . | . | . | . | . | . | . | . | . | . | . | . | . | . | . | . | . | . | . | . | . |
| 21-225_155C4_HC | . | . | . | . | . | . | . | . | . | . | . | . | . | . | . | . | . | . | . | . | . | . | . | . | . | . |
| 21-225_156E4_HC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | N | D | W | F | D | P | W | G | Q | G | T | L | V | T | V | S | S |
| 21-225_146E9_HC | . | . | . | . | . | . | . | . | . | . | . | . | . | . | . | . | . | . | . |
| 21-225_146H1_HC | . | . | . | . | . | . | . | . | . | . | . | . | . | . | . | . | . | . | . |
| 21-225_148C4_HC | . | . | . | . | . | . | . | . | . | . | . | . | . | . | . | . | . | . | . |
| 21-225_148H9_HC | . | . | . | . | . | . | . | . | . | . | . | . | . | . | . | . | . | . | . |
| 21-225_149A3_HC | . | . | . | . | . | . | . | . | . | . | . | . | . | . | . | . | . | . | . |
| 21-225_150C11_HC | . | . | . | . | . | . | . | . | . | . | . | . | . | . | . | . | . | . | . |
| 21-225_150C12_HC | . | . | . | . | . | . | . | . | . | . | . | . | . | . | . | . | . | . | . |
| 21-225_151B12_HC | . | . | . | . | . | . | . | . | . | . | . | . | . | . | . | . | . | . | . |
| 21-225_152B11_HC | . | . | . | . | . | . | . | . | . | . | . | . | . | . | . | . | . | . | . |
| 21-225_152D2_HC | . | . | . | . | . | . | . | . | . | . | . | . | . | . | . | . | . | . | . |
| 21-225_152G4_HC | . | . | . | . | . | . | . | . | . | . | . | . | . | . | . | . | . | . | . |
| 21-225_155C4_HC | . | . | . | . | . | . | . | . | . | . | . | . | . | . | . | . | . | . | . |
| 21-225_156E4_HC | . | . | . | . | . | . | . | . | . | . | . | . | . | . | . | . | . | . | . |

**FIGURE 57**

Table 76. Consensus 42 - VH3|3-33/D3|3-3|RF2/JH6 (SEQ ID NO: 50293):

QVQLVES-GGGVVQPGRSLRLSCAASG-FTFST-----YGMHWVRQAPGKGLEWVAVIWYG---GSNKDYADSVKGRFTISRDISKNTLYLQMNSLRAEDTAVYYCARDRDYCSGGSC----------PYYYYYGMDVWGQGTTVTVSS wherein:

T in position 33 can be substituted with S

I in position 58 can be substituted with V

G in position 61 can be substituted with D

S in position 66 can be substituted with N

N in position 67 can be substituted with D or S

K in position 68 can be substituted with T

D in position 69 can be substituted with Y or S

Y in position 70 can be substituted with F

A in position 71 can be substituted with V

K in position 75 can be substituted with R or T

D in position 111 can be substituted with V

Y in position 112 can be substituted with F

G in position 116 can be substituted with T

S in position 117 can be substituted with T or N

Xaa Tyr Gly Met His (SEQ ID NO: 50072)

Val Xaa Trp Tyr Xaa Gly Xaa Xaa Xaa Xaa Xaa Xaa Asp Ser Val Xaa Gly (SEQ ID NO: 50073)

Asp Arg Xaa Xaa Cys Ser Gly Xaa Xaa Cys Pro Tyr Tyr Tyr Tyr Tyr Gly Met Asp Val (SEQ ID NO: 50074)

**FIGURE 57**

Thr Tyr Gly Met His (SEQ ID NO: 50549)

Val Ile Trp Tyr Gly Gly Ser Asn Lys Asp Tyr Ala Asp Ser Val Lys Gly (SEQ ID NO: 50550)

Asp Arg Asp Tyr Cys Ser Gly Gly Ser Cys Pro Tyr Tyr Tyr Tyr Tyr Gly Met Asp Val (SEQ ID NO: 50551)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | E | S | - | G | G | G | V | V | Q | P | G | R | S | L | R | L | S | C | A | A | S |
| 21-225_146B8_HC | . | . | . | . | . | . | . | . | . | . | . | . | . | . | . | . | . | . | . | . | . | . | . | . | . | . |
| 21-225_147F9_HC | . | . | . | . | . | . | . | . | . | . | . | . | . | . | . | . | . | . | . | . | . | . | . | . | . | . |
| 21-225_147G9_HC | . | . | . | . | . | . | . | . | . | . | . | . | . | . | . | . | . | . | . | . | . | . | . | . | . | . |
| 21-225_148A9_HC | . | . | . | . | . | . | . | . | . | . | . | . | . | . | . | . | . | . | . | . | . | . | . | . | . | . |
| 21-225_150D3_HC | . | . | . | . | . | . | . | . | . | . | . | . | . | . | . | . | . | . | . | . | . | . | . | . | . | . |
| 21-225_151F3_HC | . | . | . | . | . | . | . | . | . | . | . | . | . | . | . | . | . | . | . | . | . | . | . | . | . | . |
| 21-225_153D9_HC | . | . | . | . | . | . | . | . | . | . | . | . | . | . | . | . | . | . | . | . | . | . | . | . | . | . |
| 21-225_153F9_HC | . | . | H | . | . | . | . | . | . | . | . | . | . | . | . | . | . | . | . | . | . | . | . | . | . | . |
| 21-225_154C3_HC | . | . | . | . | . | . | . | . | . | . | . | . | . | . | . | . | . | . | . | . | . | . | . | T | . | . |
| 21-225_154D11_HC | . | . | . | . | . | . | . | . | . | . | . | . | . | . | . | . | . | . | . | . | . | . | . | . | . | . |
| 21-225_158E9_HC | . | . | . | . | . | . | . | . | . | . | . | . | . | . | . | . | . | . | . | . | . | . | . | . | . | . |
| 21-225_161E10_HC | . | . | . | . | . | . | . | . | . | . | . | . | . | . | . | . | . | . | . | . | . | . | . | . | . | . |
| 21-225_74A9_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | T | - | - | - | - | - | Y | G | M | H | W | V | R | Q | A | P | G | K | G | L |
| 21-225_146B8_HC | . | . | . | . | . | . | . | . | . | . | . | . | . | . | . | . | . | . | . | . | . | . | . | . | . | . |
| 21-225_147F9_HC | . | . | . | . | . | . | . | . | . | . | . | . | . | . | . | . | . | . | . | . | . | . | . | . | . | . |
| 21-225_147G9_HC | . | . | . | . | . | . | . | . | . | . | . | . | . | . | . | . | . | . | . | . | . | . | . | . | . | . |
| 21-225_148A9_HC | . | . | . | . | . | . | . | . | . | . | . | . | . | . | . | . | . | . | . | . | . | . | . | . | . | . |
| 21-225_150D3_HC | . | . | . | . | . | . | . | . | . | . | . | . | . | . | . | . | . | . | . | . | . | . | . | . | . | . |
| 21-225_151F3_HC | . | . | . | . | . | . | . | . | . | . | . | . | . | . | . | . | . | . | . | . | . | . | . | . | . | . |
| 21-225_153D9_HC | . | . | . | . | V | . | . | . | . | . | . | . | . | . | . | . | . | . | . | . | . | . | . | . | . | . |
| 21-225_153F9_HC | . | . | . | . | I | . | . | . | . | . | . | . | . | . | . | . | . | . | . | . | G | . | . | . | . | . |
| 21-225_154C3_HC | . | . | . | . | . | . | . | . | . | . | . | . | . | . | . | . | . | . | . | . | . | . | . | . | . | . |
| 21-225_154D11_HC | . | . | . | N | V | . | . | . | . | . | . | . | . | . | . | . | . | . | . | . | . | . | . | . | . | . |
| 21-225_158E9_HC | . | . | . | . | . | . | S | . | . | . | . | . | . | . | . | . | . | . | . | . | . | . | . | . | . | . |
| 21-225_161E10_HC | . | . | . | . | . | . | S | . | . | . | . | . | . | . | . | . | . | . | . | . | . | . | . | . | . | . |
| 21-225_74A9_HC | . | . | . | . | . | . | S | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | A | V | I | W | Y | G | - | - | - | G | S | N | K | D | Y | A | D | S | V | K | G | R | F |
| 21-225_146B8_HC | . | . | . | . | . | V | . | . | . | . | . | . | . | N | D | . | . | F | . | . | . | . | T | . | . | . |
| 21-225_147F9_HC | . | . | . | . | . | . | . | . | . | . | . | . | . | . | D | . | . | . | . | . | . | . | . | . | . | . |
| 21-225_147G9_HC | . | . | . | . | . | . | . | . | . | . | . | . | . | . | D | . | . | . | . | . | . | . | . | . | . | . |
| 21-225_148A9_HC | . | . | . | . | . | . | . | . | . | . | . | . | . | . | D | . | . | . | . | . | . | . | . | . | . | . |
| 21-225_150D3_HC | . | . | . | . | . | . | . | . | . | . | . | . | . | . | . | . | . | . | . | . | . | . | . | . | . | . |
| 21-225_151F3_HC | . | . | . | . | . | . | . | . | . | . | . | . | . | . | . | T | . | . | . | . | . | . | . | . | . | . |
| 21-225_153D9_HC | . | . | . | . | . | . | . | . | . | . | . | . | . | . | . | . | . | . | . | . | . | . | . | . | . | . |
| 21-225_153F9_HC | . | . | . | . | . | . | . | . | . | . | . | . | . | . | D | T | . | . | . | . | . | . | R | . | . | . |
| 21-225_154C3_HC | . | . | . | . | . | V | . | . | . | . | . | . | . | N | . | . | . | . | . | . | . | . | . | . | . | . |
| 21-225_154D11_HC | . | . | . | . | . | . | . | . | . | . | . | . | . | . | . | . | . | . | . | . | . | . | . | . | . | . |
| 21-225_158E9_HC | . | . | . | . | . | . | . | . | D | . | . | . | . | . | S | . | Y | . | . | . | . | . | . | . | . | . |
| 21-225_161E10_HC | . | . | . | . | . | . | . | . | D | . | . | . | . | . | . | . | Y | . | V | . | . | . | . | . | . | . |
| 21-225_74A9_HC | . | . | . | . | . | . | . | . | D | . | . | . | . | N | . | . | S | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | I | S | K | N | T | L | Y | L | Q | M | N | S | L | R | A | E | D | T | A | V | Y |
| 21-225_146B8_HC | . | . | . | . | . | . | . | . | . | . | . | . | . | . | . | . | . | . | . | . | . | . | . | . | . | . |
| 21-225_147F9_HC | . | . | . | . | . | . | . | . | . | . | . | . | . | . | . | . | . | . | . | . | . | . | . | . | . | . |
| 21-225_147G9_HC | . | . | . | . | . | . | . | . | . | . | . | . | . | . | . | . | . | . | . | . | . | . | . | . | . | . |
| 21-225_148A9_HC | . | . | . | . | . | . | . | . | . | . | . | . | . | . | . | . | . | . | . | . | . | . | . | . | . | . |
| 21-225_150D3_HC | . | . | . | . | . | N | . | . | . | . | . | . | . | . | . | . | . | . | . | . | . | . | . | . | . | . |
| 21-225_151F3_HC | . | . | . | . | . | . | . | . | . | . | . | . | . | . | . | . | . | . | . | . | . | . | . | . | . | . |
| 21-225_153D9_HC | . | . | . | . | . | . | . | . | . | . | . | . | . | . | . | . | . | . | . | . | . | . | . | . | . | . |
| 21-225_153F9_HC | . | . | . | . | . | . | . | . | . | . | . | . | . | . | . | . | . | . | . | . | . | . | . | . | . | . |
| 21-225_154C3_HC | . | . | . | . | . | . | . | . | . | . | . | . | . | . | . | . | . | . | . | . | . | . | . | . | . | . |
| 21-225_154D11_HC | . | . | . | . | . | S | . | . | . | . | . | . | . | . | . | . | . | . | . | . | . | . | . | . | . | . |
| 21-225_158E9_HC | . | . | . | . | . | N | . | . | . | . | . | . | . | . | . | . | . | . | . | . | . | . | . | . | . | . |
| 21-225_161E10_HC | . | . | . | . | . | N | . | . | . | . | . | . | . | . | . | . | . | . | . | . | . | . | . | . | . | . |
| 21-225_74A9_HC | . | . | . | . | . | . | . | . | . | . | . | F | . | . | . | . | . | . | . | . | D | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | D | R | D | Y | C | S | G | G | S | C | - | - | - | - | - | - | - | - | - | - | P | Y |
| 21-225_146B8_HC | . | . | . | . | . | . | . | . | . | . | . | . | . | . | . | . | . | . | . | . | . | . | . | . | . | . |
| 21-225_147F9_HC | . | . | . | . | . | . | . | . | . | . | . | . | T | . | . | . | . | . | . | . | . | . | . | . | . | . |
| 21-225_147G9_HC | . | . | . | . | . | . | . | . | . | . | . | . | . | . | . | . | . | . | . | . | . | . | . | . | . | . |
| 21-225_148A9_HC | . | . | . | . | . | . | . | . | . | . | . | . | T | . | . | . | . | . | . | . | . | . | . | . | . | . |
| 21-225_150D3_HC | . | . | . | . | . | . | . | . | . | . | . | . | T | . | . | . | . | . | . | . | . | . | . | . | . | . |
| 21-225_151F3_HC | . | . | . | . | . | . | . | . | . | . | . | T | . | . | . | . | . | . | . | . | . | . | . | . | . | . |
| 21-225_153D9_HC | . | . | . | . | . | . | . | . | . | . | . | . | . | . | . | . | . | . | . | . | . | . | . | . | . | . |
| 21-225_153F9_HC | . | . | . | . | . | . | . | . | . | . | . | . | N | . | . | . | . | . | . | . | . | . | . | . | . | . |
| 21-225_154C3_HC | . | . | . | . | . | . | . | . | . | . | . | . | . | . | . | . | . | . | . | . | . | . | . | . | . | . |
| 21-225_154D11_HC | . | . | . | . | . | . | . | . | . | . | . | . | . | . | . | . | . | . | . | . | . | . | . | . | . | . |
| 21-225_158E9_HC | . | . | . | . | . | . | V | F | . | . | . | T | . | . | . | . | . | . | . | . | . | . | . | . | . | . |
| 21-225_161E10_HC | . | . | . | . | . | . | V | F | . | . | . | T | . | . | . | . | . | . | . | . | . | . | . | . | . | . |
| 21-225_74A9_HC | . | . | . | . | . | . | . | . | . | . | . | T | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | Y | Y | Y | Y | G | M | D | V | W | G | Q | G | T | T | V | T | V | S | S |
| 21-225_146B8_HC | . | . | . | . | . | . | . | . | . | . | . | . | . | . | . | . | . | . | . |
| 21-225_147F9_HC | . | . | . | . | . | . | . | . | . | . | . | . | . | . | . | . | . | . | . |
| 21-225_147G9_HC | . | . | . | . | . | . | . | . | . | . | . | . | . | . | . | . | . | . | . |
| 21-225_148A9_HC | . | . | . | . | . | . | . | . | . | . | . | . | . | . | . | . | . | . | . |
| 21-225_150D3_HC | . | . | . | . | . | . | . | . | . | . | . | . | . | . | . | . | . | . | . |
| 21-225_151F3_HC | . | . | . | . | . | . | . | . | . | . | . | . | . | . | . | . | . | . | . |
| 21-225_153D9_HC | . | . | . | . | . | . | . | . | . | . | . | . | . | . | . | . | . | . | . |
| 21-225_153F9_HC | . | . | . | . | . | . | . | . | . | . | . | . | . | . | . | . | . | . | . |
| 21-225_154C3_HC | . | . | . | . | . | . | . | . | . | . | . | . | . | . | . | . | . | . | . |
| 21-225_154D11_HC | . | . | . | . | . | . | . | . | . | . | . | . | . | . | . | . | . | . | . |
| 21-225_158E9_HC | . | . | . | . | . | . | . | . | . | . | . | . | . | . | . | . | . | . | . |
| 21-225_161E10_HC | . | . | . | . | . | . | . | . | . | . | . | . | . | . | . | . | . | . | . |
| 21-225_74A9_HC | . | . | . | . | . | . | . | . | . | . | . | . | . | . | . | . | . | . | . |

**FIGURE 57**

Table 77. Consensus 43 - VH4|4-39/D4|4-17|RF2/JH4 (SEQ ID NO: 50294):

QLQLQES-GPGLVKPSETLSLTCTVSG-GSISRS---SYYWGWIRQPPGKGLEWIGNIYY----SGSTYYNPSLKSRVTISVDTSKNQFSLKLSSVTAADTAVYYCGRHGKDW---------------------GLDYWGQGTLVTVSS wherein:

S in position 65 can be substituted with G

S in position 67 can be substituted with T or N

T in position 68 can be substituted with A

Y in position 70 can be substituted with N, D, H or T

L in position 74 can be substituted with V

S in position 76 can be substituted with G

Y in position 138 can be substituted with F or N

Arg Ser Ser Tyr Tyr Trp Gly (SEQ ID NO: 50075)

Asn Ile Tyr Tyr Xaa Gly Xaa Xaa Tyr Xaa Asn Pro Ser Xaa Lys Xaa (SEQ ID NO: 50076)

His Gly Lys Asp Trp Gly Leu Asp Xaa (SEQ ID NO: 50077)

Arg Ser Ser Tyr Tyr Trp Gly (SEQ ID NO: 50552)

Asn Ile Tyr Tyr Ser Gly Ser Thr Tyr Tyr Asn Pro Ser Leu Lys Ser (SEQ ID NO: 50553)

His Gly Lys Asp Trp Gly Leu Asp Tyr (SEQ ID NO: 50554)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | L | Q | L | Q | E | S | - | G | P | G | L | V | K | P | S | E | T | L | S | L | T | C | T | V | S |
| 21-225_11F5_HC | . | . | . | . | . | . | . | . | . | . | . | . | . | . | . | . | . | . | . | . | . | . | . | . | . | . |
| 21-225_14B2_HC | . | . | . | . | . | . | . | . | . | . | . | . | . | . | . | . | . | . | . | . | . | . | . | . | . | . |
| 21-225_17F9_HC | . | . | . | . | . | . | . | . | . | . | . | . | . | . | . | . | . | . | . | . | . | . | . | . | . | . |
| 21-225_17H8_HC | . | . | . | . | . | . | . | . | . | . | . | . | . | . | . | . | . | . | . | . | . | . | . | . | . | . |
| 21-225_1F2_HC | . | . | . | . | . | . | . | . | . | . | . | . | . | . | . | . | . | . | . | . | . | . | . | . | . | . |
| 21-225_21H3_HC | . | . | . | . | . | . | . | . | . | . | . | . | . | . | . | . | . | . | . | . | . | . | . | . | . | . |
| 21-225_22G8_HC | . | . | . | . | . | . | . | . | . | . | . | . | . | . | . | . | . | . | . | . | . | . | . | . | . | . |
| 21-225_22H4_HC | . | . | . | . | . | . | . | . | . | . | . | . | . | . | . | . | . | . | . | . | . | . | . | . | . | . |
| 21-225_23A3_HC | . | . | . | . | . | . | . | . | . | . | . | . | . | . | . | . | . | . | . | . | . | . | . | . | . | . |
| 21-225_23C8_HC | . | . | . | . | . | . | . | . | . | . | . | . | . | . | . | . | . | . | . | . | . | . | . | . | . | . |
| 21-225_4F4_HC | . | . | . | . | . | . | . | . | . | . | . | . | . | R | . | . | . | . | . | . | . | . | . | . | . | . |
| 21-225_4H4_HC | . | . | . | . | . | . | . | . | . | . | . | . | . | . | . | . | . | . | . | . | . | . | . | . | . | . |
| 21-225_5H7_HC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | G | S | I | S | R | S | - | - | - | S | Y | Y | W | G | W | I | R | Q | P | P | G | K | G | L |
| 21-225_11F5_HC | . | . | . | . | . | . | . | . | . | . | . | . | . | . | . | . | . | . | . | . | . | . | . | . | . | . |
| 21-225_14B2_HC | . | . | . | . | . | N | . | . | . | . | . | . | . | . | . | . | . | . | . | . | . | . | . | . | . | . |
| 21-225_17F9_HC | . | . | . | . | . | . | . | . | . | . | . | . | . | . | . | . | . | . | . | . | . | . | . | . | . | . |
| 21-225_17H8_HC | . | . | . | . | . | . | . | . | . | . | . | . | . | . | . | . | . | . | . | . | . | . | . | [illegible] | . | . |
| 21-225_1F2_HC | . | . | . | . | . | . | . | . | . | . | . | . | . | . | . | . | . | . | . | . | . | . | . | . | . | . |
| 21-225_21H3_HC | . | . | . | . | . | . | . | . | . | . | . | . | . | . | . | . | . | . | . | . | . | . | . | . | . | . |
| 21-225_22G8_HC | . | . | . | . | . | N | . | . | . | . | . | . | . | . | . | . | . | . | . | . | . | . | . | . | . | . |
| 21-225_22H4_HC | . | . | . | . | . | . | . | . | . | . | . | . | . | . | . | . | . | . | . | . | . | . | . | . | . | . |
| 21-225_23A3_HC | . | . | . | . | . | . | . | . | . | . | . | . | . | . | . | . | . | . | . | . | . | . | . | Q | . | . |
| 21-225_23C8_HC | . | . | . | . | . | . | . | . | . | . | . | . | . | . | . | . | . | . | . | . | . | . | . | . | . | . |
| 21-225_4F4_HC | . | . | . | . | . | . | . | . | . | . | . | . | . | . | . | . | . | . | . | . | . | . | . | . | . | . |
| 21-225_4H4_HC | . | . | . | . | . | . | . | . | . | . | . | . | . | . | . | . | . | . | . | . | . | . | . | M | . | . |
| 21-225_5H7_HC | . | . | . | . | . | N | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | I | G | N | I | Y | Y | - | - | - | - | S | G | S | T | Y | Y | N | P | S | L | K | S | R | V |
| 21-225_11F5_HC | . | . | . | . | . | . | . | . | . | . | . | . | . | . | . | . | . | T | . | . | . | . | . | . | . | . |
| 21-225_14B2_HC | . | . | . | . | . | . | . | . | . | . | . | . | . | . | T | . | . | . | . | . | . | . | . | . | . | . |
| 21-225_17F9_HC | . | . | . | . | . | . | . | . | . | . | . | . | . | . | . | . | . | . | . | . | . | . | . | . | . | . |
| 21-225_17H8_HC | . | . | . | . | . | . | . | . | . | . | . | . | G | . | N | . | . | . | . | . | . | . | . | . | . | . |
| 21-225_1F2_HC | . | . | . | . | . | . | . | . | . | . | . | . | . | . | . | A | . | N | . | . | . | . | . | G | . | . |
| 21-225_21H3_HC | . | . | . | . | . | . | . | . | . | . | . | . | . | . | . | . | . | D | . | . | . | . | . | . | . | . |
| 21-225_22G8_HC | D | . | . | . | . | . | . | . | . | . | . | . | . | . | . | . | . | . | . | . | . | V | . | . | . | F |
| 21-225_22H4_HC | . | . | . | . | . | . | . | . | . | . | . | . | . | . | . | . | . | H | . | . | . | . | . | . | . | . |
| 21-225_23A3_HC | . | . | . | . | . | . | . | . | . | . | . | . | . | . | . | . | . | N | . | . | . | . | . | . | . | . |
| 21-225_23C8_HC | . | . | . | . | . | . | . | . | . | . | . | . | . | . | T | . | . | N | . | . | . | . | . | . | . | . |
| 21-225_4F4_HC | . | . | . | . | . | . | . | . | . | . | . | . | . | . | . | . | . | D | . | . | . | . | . | . | . | . |
| 21-225_4H4_HC | . | . | . | . | . | . | . | . | . | . | . | . | . | . | N | . | . | N | . | . | . | . | . | . | . | . |
| 21-225_5H7_HC | . | . | . | . | . | . | . | . | . | . | . | . | . | . | T | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | V | D | T | S | K | N | Q | F | S | L | K | L | S | S | V | T | A | A | D | T | A | V | Y |
| 21-225_11F5_HC | . | . | . | . | . | . | . | . | . | H | . | . | . | . | . | . | . | . | . | . | . | . | . | . | . | . |
| 21-225_14B2_HC | . | . | . | . | . | . | . | . | . | . | . | . | . | N | . | . | . | . | . | . | . | . | . | . | . | . |
| 21-225_17F9_HC | . | . | . | . | . | S | . | . | . | . | . | . | . | N | . | N | . | . | . | . | . | . | . | . | . | . |
| 21-225_17H8_HC | . | . | . | . | . | . | . | . | . | . | . | . | . | . | . | . | . | . | . | . | . | . | . | . | . | . |
| 21-225_1F2_HC | . | . | . | . | . | . | . | . | . | . | . | . | . | . | . | N | . | . | . | . | . | . | . | . | . | . |
| 21-225_21H3_HC | . | . | . | . | . | . | . | . | . | . | . | . | . | . | . | . | . | . | . | . | . | . | . | . | . | . |
| 21-225_22G8_HC | . | . | . | . | . | . | . | . | . | . | . | . | . | . | . | . | . | . | . | . | . | . | . | . | . | . |
| 21-225_22H4_HC | . | . | . | . | . | . | . | M | . | . | . | . | . | . | . | T | . | . | . | . | . | . | . | . | . | . |
| 21-225_23A3_HC | S | . | . | . | . | . | . | . | . | . | . | . | . | N | . | . | . | . | . | . | . | . | . | . | . | . |
| 21-225_23C8_HC | . | . | . | . | . | . | . | . | . | H | . | . | . | . | . | . | . | . | . | . | . | . | . | . | . | . |
| 21-225_4F4_HC | . | . | . | . | . | . | . | . | . | . | . | . | . | . | . | . | . | . | . | . | . | . | . | . | . | . |
| 21-225_4H4_HC | . | . | . | . | . | . | . | . | . | . | . | . | . | . | . | N | . | . | . | . | . | . | . | . | . | . |
| 21-225_5H7_HC | . | . | . | . | . | . | . | . | . | . | . | . | . | N | . | . | . | . | . | . | . | . | . | . | . | . |

# FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | G | R | H | G | K | D | W | - | - | - | - | - | - | - | - | - | - | - | - | - | - | - | - | - |
| 21-225_11F5_HC | . | . | . | . | . | . | . | . | . | . | . | . | . | . | . | . | . | . | . | . | . | . | . | . | . | . |
| 21-225_14B2_HC | S | . | A | . | . | . | . | . | . | . | . | . | . | . | . | . | . | . | . | . | . | . | . | . | . | . |
| 21-225_17F9_HC | S | . | A | . | . | . | . | . | . | . | . | . | . | . | . | . | . | . | . | . | . | . | . | . | . | . |
| 21-225_17H8_HC | . | . | A | . | . | . | . | . | . | . | . | . | . | . | . | . | . | . | . | . | . | . | . | . | . | . |
| 21-225_1F2_HC | . | . | . | . | . | . | . | . | . | . | . | . | . | . | . | . | . | . | . | . | . | . | . | . | . | . |
| 21-225_21H3_HC | . | . | . | . | . | . | . | . | . | . | . | . | . | . | . | . | . | . | . | . | . | . | . | . | . | . |
| 21-225_22G8_HC | . | . | A | . | . | . | . | . | . | . | . | . | . | . | . | . | . | . | . | . | . | . | . | . | . | . |
| 21-225_22H4_HC | F | . | . | . | . | . | . | . | . | . | . | . | . | . | . | . | . | . | . | . | . | . | . | . | . | . |
| 21-225_23A3_HC | . | . | . | . | . | . | . | . | . | . | . | . | . | . | . | . | . | . | . | . | . | . | . | . | . | . |
| 21-225_23C8_HC | . | . | . | . | . | . | . | . | . | . | . | . | . | . | . | . | . | . | . | . | . | . | . | . | . | . |
| 21-225_4F4_HC | . | . | . | . | . | . | . | . | . | . | . | . | . | . | . | . | . | . | . | . | . | . | . | . | . | . |
| 21-225_4H4_HC | . | . | . | . | . | . | . | . | . | . | . | . | . | . | . | . | . | . | . | . | . | . | . | . | . | . |
| 21-225_5H7_HC | S | . | A | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
| --- | --- | --- | --- | --- | --- | --- | --- | --- | --- | --- | --- | --- | --- | --- | --- | --- | --- | --- | --- |
| | | | | | | | | | H_FR4 | | | | | | | | | | |
| CONSENSUS | - | - | - | - | G | L | D | Y | W | G | Q | G | T | L | V | T | V | S | S |
| 21-225_11F5_HC | . | . | . | . | . | . | . | N | . | . | . | . | . | . | . | . | . | . | . |
| 21-225_14B2_HC | . | . | . | . | . | . | . | . | . | . | . | . | A | . | . | . | . | . | . |
| 21-225_17F9_HC | . | . | . | . | . | . | . | . | . | . | . | . | . | . | . | . | . | . | . |
| 21-225_17H8_HC | . | . | . | . | . | . | . | . | . | . | . | . | . | . | . | . | . | . | . |
| 21-225_1F2_HC | . | . | . | . | . | . | . | . | . | . | . | . | . | . | . | . | . | . | . |
| 21-225_21H3_HC | . | . | . | . | . | . | . | . | . | . | . | . | . | . | . | . | . | . | . |
| 21-225_22G8_HC | . | . | . | . | . | . | . | . | . | . | . | . | . | . | . | . | . | . | . |
| 21-225_22H4_HC | . | . | . | . | . | . | . | . | . | . | . | . | . | . | . | . | . | . | . |
| 21-225_23A3_HC | . | . | . | . | . | . | . | F | . | . | . | . | . | . | . | . | . | . | . |
| 21-225_23C8_HC | . | . | . | . | . | . | . | . | . | . | . | . | . | . | . | . | . | . | . |
| 21-225_4F4_HC | . | . | . | . | . | . | . | . | . | . | . | . | . | . | . | . | . | . | . |
| 21-225_4H4_HC | . | . | . | . | . | . | . | . | . | . | . | . | . | . | . | I | . | . | . |
| 21-225_5H7_HC | . | . | . | . | . | . | . | . | . | . | . | . | . | . | . | . | . | . | . |

**FIGURE 57**

Table 78. Consensus 44 - VH1|1-02/D7|7-27|RF1/JH4 (SEQ ID NO: 50295):

QVQLVQS-GAEVKKPGASVKVSCKASG-YTFTG-----YYMHWVRQAPGQGLEWMGWIKPN---SGGTNQAQKFQGRVTMTRDTSISTAYMELSGLRSDDTAVYYCARAPGTAAAG--------------TWGYFDYWGQGTLVTVSS wherein:

M at position 41 can be substituted with I

K in position 59 can be substituted with N

N in position 61 can be substituted with K

Q in position 70 can be substituted with S, H, N or Y

A in position 71 can be substituted with V

T in position 112 can be substituted with K or I

A in position 113 can be substituted with V

A in position 114 can be substituted with P

A in position 115 can be substituted with T

T in position 132 can be substituted with S

Y in position 135 can be substituted with F or C

Gly Tyr Tyr Xaa His (SEQ ID NO: 50078)

Trp Ile Xaa Pro Xaa Ser Gly Gly Thr Asn Xaa Xaa Gln Lys Phe Gln Gly (SEQ ID NO: 50079)

Ala Pro Gly Xaa Xaa Xaa Xaa Gly Xaa Trp Gly Xaa Phe Asp Tyr (SEQ ID NO: 50080)

Gly Tyr Tyr Met His (SEQ ID NO: 50555)

Trp Ile Lys Pro Asn Ser Gly Gly Thr Asn Gln Ala Gln Lys Phe Gln Gly (SEQ ID NO: 50556)

Ala Pro Gly Thr Ala Ala Ala Gly Thr Trp Gly Tyr Phe Asp Tyr (SEQ ID NO: 50557)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | Q | S | - | G | A | E | V | K | K | P | G | A | S | V | K | V | S | C | K | A | S |
| 21-225_62G7_HC | . | . | . | . | . | . | . | . | . | . | . | . | . | . | . | . | . | . | . | . | . | . | . | . | . | . |
| 21-225_64H10_HC | . | . | . | . | . | . | . | . | . | . | . | . | . | . | . | . | . | . | . | . | . | . | . | . | . | . |
| 21-225_65G3_HC | . | . | . | . | . | . | . | . | . | . | . | . | . | . | . | . | . | . | . | . | . | . | . | . | . | . |
| 21-225_66A7_HC | . | . | . | . | . | . | . | . | . | . | . | . | . | . | . | . | . | . | . | . | . | . | . | . | . | . |
| 21-225_66B1_HC | . | . | . | . | . | . | . | . | . | . | . | . | . | . | . | . | . | . | . | . | . | . | . | . | . | . |
| 21-225_67H4_HC | . | . | . | . | . | . | . | . | . | . | . | . | . | . | . | . | . | . | . | . | . | . | . | . | . | . |
| 21-225_70A12_HC | . | . | . | . | . | . | . | . | . | . | . | . | . | . | . | . | . | . | . | . | . | . | . | . | . | . |
| 21-225_70D6_HC | . | . | . | . | . | . | . | . | . | . | . | . | . | . | . | . | . | . | . | . | . | . | . | . | . | . |
| 21-225_70G9_HC | . | . | . | . | . | . | . | . | . | . | . | . | . | . | . | . | . | . | . | . | . | . | . | . | . | . |
| 21-225_71A7_HC | . | . | . | . | . | . | . | . | . | . | . | . | . | . | . | . | . | . | . | . | . | . | . | . | . | . |
| 21-225_71B7_HC | . | . | . | . | . | . | . | . | . | . | . | . | . | . | . | . | . | . | . | . | . | . | . | . | . | . |
| 21-225_73A3_HC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | Y | T | F | T | G | - | - | - | - | - | Y | Y | M | H | W | V | R | Q | A | P | G | Q | G | L |
| 21-225_62G7_HC | . | . | . | . | . | . | . | . | . | . | . | . | . | . | . | . | . | . | . | . | . | . | . | . | . | . |
| 21-225_64H10_HC | . | . | . | . | . | . | . | . | . | . | . | . | . | . | . | . | . | . | . | . | . | . | . | . | . | . |
| 21-225_65G3_HC | . | . | . | . | . | . | . | . | . | . | . | . | . | . | . | . | . | . | . | . | . | . | . | . | . | . |
| 21-225_66A7_HC | . | . | . | . | . | . | . | . | . | . | . | . | . | . | . | . | . | . | . | . | . | . | . | . | . | . |
| 21-225_66B1_HC | . | . | . | . | . | . | . | . | . | . | . | . | . | . | . | . | . | . | . | . | . | . | . | . | . | . |
| 21-225_67H4_HC | . | . | . | . | . | . | . | . | . | . | . | . | . | . | . | . | . | . | . | . | . | . | . | . | . | . |
| 21-225_70A12_HC | . | . | . | . | . | . | . | . | . | . | . | . | . | . | . | . | . | . | . | . | . | . | . | . | . | . |
| 21-225_70D6_HC | . | . | . | . | . | . | . | . | . | . | . | . | . | . | . | . | . | . | . | . | . | . | . | . | . | . |
| 21-225_70G9_HC | . | . | . | . | . | . | . | . | . | . | . | . | . | . | . | . | . | . | . | . | . | . | . | . | . | . |
| 21-225_71A7_HC | . | . | . | . | . | . | . | . | . | . | . | . | . | . | . | . | . | . | . | . | . | . | . | . | . | . |
| 21-225_71B7_HC | . | . | . | . | . | . | . | . | . | . | . | . | . | . | . | . | . | . | . | . | . | . | . | . | . | . |
| 21-225_73A3_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | M | G | W | I | | P | N | - | - | - | S | G | G | T | N | Q | A | Q | K | F | Q | G | R | V |
| 21-225_62G7_HC | . | . | . | . | . | . | K | . | K | . | . | . | . | . | . | . | . | . | . | . | . | . | . | . | . | . |
| 21-225_64H10_HC | . | . | . | . | . | . | K | . | . | . | . | . | . | . | . | . | . | . | . | . | . | . | . | . | . | . |
| 21-225_65G3_HC | . | . | . | . | . | . | N | . | . | . | . | . | . | . | . | . | . | S | . | . | . | . | . | . | . | . |
| 21-225_66A7_HC | . | . | . | . | . | . | K | . | . | . | . | . | . | . | . | . | . | . | . | . | . | . | . | . | . | . |
| 21-225_66B1_HC | . | . | . | . | . | . | N | . | . | . | . | . | . | . | . | . | . | N | . | . | . | . | . | . | . | . |
| 21-225_67H4_HC | . | . | . | . | . | . | K | . | . | . | . | . | . | . | . | . | . | . | . | . | . | . | . | . | . | . |
| 21-225_70A12_HC | . | . | . | . | . | . | K | . | . | . | . | . | . | . | . | . | . | . | . | . | . | . | . | . | . | . |
| 21-225_70D6_HC | . | . | . | . | . | . | N | . | K | . | . | . | . | . | . | . | . | S | . | . | . | . | . | . | . | . |
| 21-225_70G9_HC | . | . | . | . | . | . | K | . | . | . | . | . | . | . | . | . | . | . | . | . | . | . | . | . | . | . |
| 21-225_71A7_HC | . | . | . | . | . | . | N | . | K | . | . | . | . | . | . | . | . | Y | V | . | . | . | . | . | . | . |
| 21-225_71B7_HC | . | . | . | . | . | . | N | . | . | . | . | . | . | . | . | . | . | S | . | . | . | . | . | . | . | . |
| 21-225_73A3_HC | D | . | . | . | . | . | N | . | K | . | . | . | . | . | . | . | . | H | V | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | M | T | R | D | T | S | I | S | T | A | Y | M | E | L | S | G | L | R | S | D | D | T | A | V | Y |
| 21-225_62G7_HC | . | . | . | . | . | . | . | . | . | . | . | . | . | . | . | . | S | . | . | . | . | . | . | . | . | . |
| 21-225_64H10_HC | . | . | . | . | . | . | . | . | . | . | . | . | . | . | . | . | . | . | . | . | . | . | . | . | . | . |
| 21-225_65G3_HC | . | . | . | . | . | . | . | . | . | . | . | . | . | . | . | . | R | . | . | . | . | . | . | . | . | . |
| 21-225_66A7_HC | . | . | . | . | . | . | . | . | . | . | G | . | . | . | . | . | R | . | . | . | . | . | . | . | . | . |
| 21-225_66B1_HC | . | . | . | . | A | . | . | . | . | . | . | . | . | . | . | . | R | . | . | . | . | . | . | . | . | . |
| 21-225_67H4_HC | . | . | . | . | . | . | . | . | . | . | . | . | . | . | . | . | . | . | . | . | . | . | . | . | . | . |
| 21-225_70A12_HC | . | . | . | . | . | . | . | . | . | . | . | . | . | . | . | . | . | . | . | . | . | . | . | . | . | . |
| 21-225_70D6_HC | S | . | . | . | . | . | . | . | G | . | . | . | . | . | . | R | . | . | . | . | . | . | . | . | E | . |
| 21-225_70G9_HC | . | . | . | . | . | . | . | . | . | . | . | . | . | . | . | . | S | . | . | . | . | . | . | . | . | . |
| 21-225_71A7_HC | . | . | . | . | . | . | . | . | . | . | . | . | . | . | . | . | S | . | . | . | . | . | . | . | . | . |
| 21-225_71B7_HC | . | . | . | . | . | . | . | . | G | . | . | . | . | . | . | . | . | . | . | . | . | . | . | . | . | . |
| 21-225_73A3_HC | A | . | . | . | . | . | . | . | . | . | . | . | . | . | . | . | S | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | A | P | G | T | A | A | A | G | - | - | - | - | - | - | - | - | - | - | - | - | - | - |
| 21-225_62G7_HC | . | . | . | . | . | . | . | I | . | . | . | . | . | . | . | . | . | . | . | . | . | . | . | . | . | . |
| 21-225_64H10_HC | . | . | . | . | . | . | . | . | . | . | T | . | . | . | . | . | . | . | . | . | . | . | . | . | . | . |
| 21-225_65G3_HC | . | . | . | . | . | . | . | K | . | . | . | . | . | . | . | . | . | . | . | . | . | . | . | . | . | . |
| 21-225_66A7_HC | . | . | . | . | . | . | . | . | V | . | . | . | . | . | . | . | . | . | . | . | . | . | . | . | . | . |
| 21-225_66B1_HC | . | . | . | . | . | . | . | . | . | . | . | . | . | . | . | . | . | . | . | . | . | . | . | . | . | . |
| 21-225_67H4_HC | . | . | . | . | . | . | . | . | . | . | T | . | . | . | . | . | . | . | . | . | . | . | . | . | . | . |
| 21-225_70A12_HC | . | . | . | . | . | . | . | . | . | . | T | . | . | . | . | . | . | . | . | . | . | . | . | . | . | . |
| 21-225_70D6_HC | . | . | . | . | . | . | . | K | . | . | . | . | . | . | . | . | . | . | . | . | . | . | . | . | . | . |
| 21-225_70G9_HC | . | . | . | . | . | . | . | I | . | . | . | . | . | . | . | . | . | . | . | . | . | . | . | . | . | . |
| 21-225_71A7_HC | . | . | . | . | . | . | . | . | . | P | . | . | . | . | . | . | . | . | . | . | . | . | . | . | . | . |
| 21-225_71B7_HC | . | . | . | . | . | . | . | K | . | . | . | . | . | . | . | . | . | . | . | . | . | . | . | . | . | . |
| 21-225_73A3_HC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | T | W | G | Y | F | D | Y | W | G | Q | G | T | L | V | T | V | S | S |
| 21-225_62G7_HC | . | . | . | . | . | . | . | . | . | . | . | . | . | . | . | . | . | . | . |
| 21-225_64H10_HC | . | . | . | . | . | . | . | . | . | . | . | . | . | . | . | . | . | . | . |
| 21-225_65G3_HC | . | . | . | . | . | . | . | . | . | . | . | . | . | . | . | . | . | . | . |
| 21-225_66A7_HC | . | . | . | . | . | . | . | . | . | . | . | . | . | . | . | . | . | . | . |
| 21-225_66B1_HC | . | . | . | . | . | . | . | . | . | . | . | . | . | . | . | . | . | . | . |
| 21-225_67H4_HC | . | . | . | . | . | . | . | . | . | . | . | . | . | . | . | . | . | . | . |
| 21-225_70A12_HC | . | . | . | . | . | . | . | . | . | . | . | . | . | . | . | . | . | . | . |
| 21-225_70D6_HC | . | . | . | . | F | . | . | . | . | . | . | . | . | . | . | . | . | . | . |
| 21-225_70G9_HC | . | . | . | . | . | . | . | . | . | . | . | . | . | . | . | . | . | . | . |
| 21-225_71A7_HC | . | S | . | . | . | . | . | . | . | . | . | . | . | . | . | . | . | . | . |
| 21-225_71B7_HC | . | . | . | . | F | . | . | . | . | . | . | . | . | . | . | . | . | . | . |
| 21-225_73A3_HC | . | S | . | . | C | . | . | . | . | . | . | . | . | . | . | . | . | . | . |

**FIGURE 57**

Table 79. Consensus 45 - VH2|2-05/D6|6-6|RF2/JH4 (SEQ ID NO: 50296):

QITLKES-GPTLVKPTQTLTLTCTFSG-FSLSTG---GVGVGWIRQPPGKALEWLALIYW----DDDKRYSPSLKSRLTITKDTSKNQVVLTMTNMDPVDTATYYCAHLIAV---------------------AFDYWGQGTLVTVSS wherein:

G in position 34 can be substituted with S

L in position 57 can be substituted with F

D in position 65 can be substituted with H, N, K or S

K in position 68 can be substituted with E

K in position 75 can be substituted with R

L in position 109 can be substituted with I or A

I in position 110 can be substituted with V or A

A in position 135 can be substituted with S

F in position 136 can be substituted with C

Thr Xaa Gly Val Gly Val Gly (SEQ ID NO: 50081)

Xaa Ile Tyr Trp Xaa Asp Asp Xaa Arg Tyr Ser Pro Ser Leu Xaa Ser (SEQ ID NO: 50082)

Xaa Xaa Ala Val Xaa Xaa Asp Tyr (SEQ ID NO: 50083)

Thr Gly Gly Val Gly Val Gly (SEQ ID NO: 50558)

Leu Ile Tyr Trp Asp Asp Asp Lys Arg Tyr Ser Pro Ser Leu Lys Ser (SEQ ID NO: 50559)

Leu Ile Ala Val Ala Phe Asp Tyr (SEQ ID NO: 50560)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | I | T | L | K | E | S | - | G | P | T | L | V | K | P | T | Q | T | L | T | L | T | C | T | F | S |
| 21-225_10B10_HC | . | . | . | . | . | . | . | . | . | . | . | . | . | . | . | . | . | . | . | . | . | . | . | . | . | . |
| 21-225_15H11_HC | . | . | . | . | . | . | . | . | . | L | . | . | M | . | . | . | . | . | . | . | . | . | . | . | . | . |
| 21-225_17F5_HC | . | . | . | . | . | . | . | . | . | . | S | . | . | . | . | . | . | . | . | . | . | . | . | . | . | . |
| 21-225_30H6_HC | . | . | . | . | . | . | . | . | . | . | . | . | . | . | . | . | . | . | . | . | . | . | . | . | . | . |
| 21-225_31C2_HC | . | . | . | . | . | . | . | . | . | . | . | . | . | . | . | . | . | . | . | . | . | . | . | . | . | . |
| 21-225_57D9_HC | . | . | . | . | . | . | . | . | . | . | M | . | . | . | . | . | . | . | . | . | . | . | . | . | . | . |
| 21-225_60D6_HC | . | . | . | . | . | . | . | . | . | . | . | . | . | . | . | . | . | . | . | . | . | . | . | . | . | . |
| 21-225_74G9_HC | . | . | . | . | . | . | . | . | . | . | . | . | . | . | . | . | . | . | . | . | . | . | . | . | . | . |
| 21-225_75F11_HC | . | . | . | . | . | . | . | . | . | . | . | . | . | . | . | . | . | . | . | . | . | . | . | . | . | . |
| 21-225_76B4_HC | . | . | . | . | . | . | . | . | . | . | . | . | . | . | . | . | . | . | . | . | . | . | . | . | . | . |
| 21-225_77A2_HC | . | . | . | . | . | . | . | . | . | . | S | . | . | . | . | . | . | . | . | . | . | . | . | . | . | . |
| 21-225_96B5_HC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | S | L | S | T | G | - | - | - | G | V | G | V | G | W | I | R | Q | P | P | G | K | A | L |
| 21-225_10B10_HC | . | . | . | . | . | . | . | . | . | . | . | . | . | . | . | . | . | . | . | . | . | . | . | . | . | . |
| 21-225_15H11_HC | . | . | . | . | . | . | . | . | . | . | . | . | . | . | . | . | . | . | . | . | . | . | . | . | . | . |
| 21-225_17F5_HC | . | . | . | . | . | . | . | S | . | . | . | . | . | . | . | . | . | . | . | . | . | . | . | . | . | . |
| 21-225_30H6_HC | . | . | . | . | . | . | . | . | . | . | . | . | . | . | . | . | . | . | . | . | . | . | . | . | . | . |
| 21-225_31C2_HC | . | . | . | . | . | N | . | . | . | . | . | . | . | . | . | . | . | . | . | . | . | . | . | . | . | . |
| 21-225_57D9_HC | . | . | . | . | . | . | . | S | . | . | . | . | . | . | . | . | . | . | . | . | . | . | . | . | . | . |
| 21-225_60D6_HC | . | . | . | . | . | . | . | S | . | . | . | . | . | . | . | . | . | . | . | . | . | . | . | . | . | . |
| 21-225_74G9_HC | . | . | . | . | . | . | . | . | . | . | . | . | . | . | . | . | . | . | . | . | . | . | . | . | . | . |
| 21-225_75F11_HC | . | . | . | . | . | . | . | . | . | . | . | . | . | . | . | . | . | . | . | . | . | . | . | . | . | . |
| 21-225_76B4_HC | . | . | . | . | . | . | . | . | . | . | . | . | . | . | . | . | . | . | . | . | . | . | . | . | . | . |
| 21-225_77A2_HC | . | . | . | . | . | . | . | S | . | . | . | . | . | . | . | . | . | . | . | . | . | . | . | . | . | . |
| 21-225_96B5_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | L | A | L | I | Y | W | - | - | - | - | D | D | D | K | R | Y | S | P | S | L | K | S | R | L |
| 21-225_10B10_HC | . | . | . | V | F | . | . | . | . | . | . | . | S | . | . | . | . | . | . | . | . | . | . | . | . | . |
| 21-225_15H11_HC | . | . | . | . | . | . | . | . | . | . | . | . | H | . | . | . | . | . | . | . | . | . | R | . | . | . |
| 21-225_17F5_HC | . | . | . | . | . | . | . | . | . | . | . | . | . | . | . | . | . | . | . | . | . | . | . | . | . | . |
| 21-225_30H6_HC | . | . | . | . | . | . | . | . | . | . | . | . | H | . | . | . | . | . | . | . | . | . | . | . | . | . |
| 21-225_31C2_HC | . | . | . | . | . | . | . | . | . | . | . | . | N | . | . | E | . | . | . | . | . | . | . | . | . | . |
| 21-225_57D9_HC | . | . | . | . | . | . | . | . | . | . | . | . | N | . | . | . | . | . | . | . | . | . | . | . | . | . |
| 21-225_60D6_HC | . | . | . | . | . | . | . | . | . | . | . | . | K | . | . | E | . | . | . | . | . | . | . | . | . | . |
| 21-225_74G9_HC | . | . | . | . | . | . | . | . | . | . | . | . | . | . | . | . | . | . | . | . | . | . | . | . | . | . |
| 21-225_75F11_HC | . | . | . | . | . | . | . | . | . | . | . | . | . | . | . | . | . | . | . | . | . | . | . | . | . | . |
| 21-225_76B4_HC | . | . | . | . | . | . | . | . | . | . | . | . | . | . | . | . | . | . | . | . | . | . | . | . | . | . |
| 21-225_77A2_HC | . | . | . | V | F | . | . | . | . | . | . | . | . | . | . | . | . | . | . | . | . | . | . | . | . | . |
| 21-225_96B5_HC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | T | K | D | T | S | K | N | Q | V | V | L | T | M | T | N | M | D | P | V | D | T | A | T | Y |
| 21-225_10B10_HC | S | . | . | . | . | . | . | . | . | . | . | . | . | . | . | . | . | . | . | . | . | . | . | . | . | . |
| 21-225_15H11_HC | . | . | . | . | . | . | . | . | . | . | . | . | . | . | . | . | . | . | . | . | . | . | . | . | . | . |
| 21-225_17F5_HC | . | . | . | . | . | . | . | . | . | . | . | . | . | . | . | . | . | . | . | . | . | . | . | . | . | . |
| 21-225_30H6_HC | A | . | . | . | . | . | . | . | . | . | . | . | . | . | I | . | . | . | . | . | . | . | . | . | . | . |
| 21-225_31C2_HC | . | . | . | . | . | . | . | . | . | . | . | . | . | . | . | . | . | . | . | . | L | . | . | . | S | . |
| 21-225_57D9_HC | . | . | . | E | . | . | . | . | . | . | . | . | . | . | . | . | . | . | . | . | . | . | . | . | . | . |
| 21-225_60D6_HC | . | . | . | . | . | . | . | . | . | . | . | . | . | . | . | . | . | . | . | . | . | . | . | . | . | . |
| 21-225_74G9_HC | . | . | . | . | . | . | P | . | . | . | . | . | . | . | . | . | . | . | . | . | . | . | . | . | . | . |
| 21-225_75F11_HC | . | . | . | . | . | . | P | . | . | . | . | . | . | . | . | . | . | . | . | . | . | . | . | . | . | . |
| 21-225_76B4_HC | . | . | . | . | . | . | P | . | . | . | . | . | . | . | . | . | . | . | . | . | . | . | . | . | . | . |
| 21-225_77A2_HC | . | . | . | . | . | . | . | . | . | . | . | . | . | . | . | . | . | . | . | . | . | . | . | . | . | . |
| 21-225_96B5_HC | . | . | . | . | . | . | P | . | . | . | . | . | . | . | . | . | . | . | . | . | . | . | . | . | . | . |

# FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | H | L | I | A | V | - | - | - | - | - | - | - | - | - | - | - | - | - | - | - | - | - | - |
| 21-225_10B10_HC | . | . | . | R | I | A | . | . | . | . | . | . | . | . | . | . | . | . | . | . | . | . | . | . | . | . |
| 21-225_15H11_HC | . | . | . | R | I | V | . | . | . | . | . | . | . | . | . | . | . | . | . | . | . | . | . | . | . | . |
| 21-225_17F5_HC | S | . | . | . | I | . | . | . | . | . | . | . | . | . | . | . | . | . | . | . | . | . | . | . | . | . |
| 21-225_30H6_HC | . | . | . | . | . | . | . | . | . | . | . | . | . | . | . | . | . | . | . | . | . | . | . | . | . | . |
| 21-225_31C2_HC | . | . | . | . | . | . | . | . | . | . | . | . | . | . | . | . | . | . | . | . | . | . | . | . | . | . |
| 21-225_57D9_HC | . | . | . | . | A | V | . | . | . | . | . | . | . | . | . | . | . | . | . | . | . | . | . | . | . | . |
| 21-225_60D6_HC | . | . | . | . | A | V | . | . | . | . | . | . | . | . | . | . | . | . | . | . | . | . | . | . | . | . |
| 21-225_74G9_HC | . | . | . | . | . | . | . | . | . | . | . | . | . | . | . | . | . | . | . | . | . | . | . | . | . | . |
| 21-225_75F11_HC | . | . | . | . | . | . | . | . | . | . | . | . | . | . | . | . | . | . | . | . | . | . | . | . | . | . |
| 21-225_76B4_HC | . | . | . | . | . | . | . | . | . | . | . | . | . | . | . | . | . | . | . | . | . | . | . | . | . | . |
| 21-225_77A2_HC | . | . | . | . | . | . | . | . | . | . | . | . | . | . | . | . | . | . | . | . | . | . | . | . | . | . |
| 21-225_96B5_HC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | A | F | D | Y | W | G | Q | G | T | L | V | T | V | S | S |
| 21-225_10B10_HC | . | . | . | . | . | . | . | . | . | . | . | . | . | . | . | . | . | . | . |
| 21-225_15H11_HC | . | . | . | . | . | . | . | . | . | . | . | . | . | . | I | . | . | . | . |
| 21-225_17F5_HC | . | . | . | . | . | . | . | . | . | . | . | . | . | . | . | . | . | . | . |
| 21-225_30H6_HC | . | . | . | . | . | . | . | . | . | . | . | . | . | . | . | . | . | . | . |
| 21-225_31C2_HC | . | . | . | . | S | . | . | . | . | . | . | . | A | . | . | . | . | . | . |
| 21-225_57D9_HC | . | . | . | . | S | . | . | . | . | . | . | . | . | . | . | . | . | . | . |
| 21-225_60D6_HC | . | . | . | . | S | . | . | . | . | . | . | . | . | . | . | . | . | . | . |
| 21-225_74G9_HC | . | . | . | . | . | . | . | . | . | . | . | . | . | . | . | . | . | . | . |
| 21-225_75F11_HC | . | . | . | . | . | . | . | . | . | . | . | . | . | . | . | . | . | . | . |
| 21-225_76B4_HC | . | . | . | . | . | . | . | . | . | . | . | . | . | . | . | . | . | . | . |
| 21-225_77A2_HC | . | . | . | . | . | . | . | . | . | . | . | . | . | . | . | . | . | . | . |
| 21-225_96B5_HC | . | . | . | . | . | C | . | . | . | . | . | . | . | . | . | . | . | . | . |

**FIGURE 57**

Table 80. Consensus 46 - VH3|3-33/D3|3-22|RF2/JH4 (SEQ ID NO: 50297):

QVQLVES-GGGVVQPGRSLRLSCAASG-FTFSS-----YGMHWVRQAPGKGLEWVAIIWYD---GSYKYYADSVKGRFTISRDNSKNTLYLQMNSLRAEDTAVYYCAREAYDFW------------------SGYFDYWGQGTLVTVSS wherein:

S at position 33 can be substituted with N or T

I at position 57 can be substituted with V

Y at position 67 can be substituted with N

A at position 71 can be substituted with V

A at position 110 can be substituted with G, R, or N

Y at position 135 can be substituted with F or H

F at position 136 can be substituted with L, Y, or W

D at position 137 can be substituted with G

Y at position 138 can be substituted with S

Xaa Tyr Gly Met His (SEQ ID NO: 50084)

Xaa Ile Trp Tyr Asp Gly Ser Xaa Lys Tyr Tyr Xaa Asp Ser Val Lys Gly (SEQ ID NO: 50085)

Glu Xaa Tyr Asp Phe Trp Ser Gly Xaa Xaa Xaa Xaa (SEQ ID NO: 50086)

Ser Tyr Gly Met His (SEQ ID NO: 50561)

Ile Ile Trp Tyr Asp Gly Ser Tyr Lys Tyr Tyr Ala Asp Ser Val Lys Gly (SEQ ID NO: 50562)

Glu Ala Tyr Asp Phe Trp Ser Gly Tyr Phe Asp Tyr (SEQ ID NO: 50563)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | E | S | - | G | G | G | V | V | Q | P | G | R | S | L | R | L | S | C | A | A | S |
| 21-225_152C11_HC | . | . | . | . | . | . | . | . | . | . | . | . | . | . | . | . | . | . | . | . | . | . | . | . | . | . |
| 21-225_152F6_HC | . | . | . | . | . | . | . | . | . | . | . | . | . | . | . | . | . | . | . | . | . | . | . | . | . | . |
| 21-225_153D2_HC | . | . | . | . | . | . | . | . | . | . | . | . | . | . | . | . | . | . | . | . | . | . | . | . | . | . |
| 21-225_157G8_HC | . | . | . | . | . | . | . | . | . | . | . | . | . | . | . | . | . | . | . | . | . | . | . | . | . | . |
| 21-225_160B1_HC | . | . | . | . | . | . | . | . | . | . | . | . | . | . | . | . | . | . | . | . | . | . | . | . | . | . |
| 21-225_172B7_HC | . | . | . | . | . | . | . | . | . | . | . | . | . | . | . | . | . | . | . | . | . | . | . | . | . | . |
| 21-225_173A11_HC | . | . | . | . | . | . | . | . | . | . | . | . | . | . | . | . | . | . | . | . | . | . | . | . | . | . |
| 21-225_178G10_HC | . | . | . | . | . | . | . | . | . | . | . | . | . | . | . | . | . | . | . | . | . | . | . | . | . | . |
| 21-225_188G11_HC | . | . | . | . | . | . | . | . | . | . | . | . | . | . | . | . | . | . | . | . | . | . | . | . | . | . |
| 21-225_226E7_HC | . | . | . | . | . | . | . | . | . | . | . | . | . | . | . | . | . | . | . | . | . | . | . | . | . | . |
| 21-225_25H9_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | G | M | H | W | V | R | Q | A | P | G | K | G | L |
| 21-225_152C11_HC | . | . | . | . | . | R | N | . | . | . | . | . | . | . | . | . | . | . | . | . | . | . | . | . | . | . |
| 21-225_152F6_HC | . | . | . | . | . | . | . | . | . | . | . | . | . | . | . | . | . | . | . | . | . | . | . | . | . | . |
| 21-225_153D2_HC | . | . | . | . | . | . | . | . | . | . | . | . | . | . | . | . | . | . | . | . | . | . | . | . | . | . |
| 21-225_157G8_HC | . | . | . | . | . | . | N | . | . | . | . | . | . | . | . | . | . | . | . | . | . | . | . | . | . | . |
| 21-225_160B1_HC | . | . | . | . | . | . | . | . | . | . | . | . | . | . | . | . | . | . | . | . | . | . | . | . | . | . |
| 21-225_172B7_HC | . | . | . | . | . | . | N | . | . | . | . | . | . | . | . | . | . | . | . | . | . | . | . | . | . | . |
| 21-225_173A11_HC | . | . | . | . | . | . | . | . | . | . | . | . | . | . | . | . | . | . | . | . | . | . | . | . | . | . |
| 21-225_178G10_HC | . | . | . | . | . | . | T | . | . | . | . | . | . | . | . | . | . | . | . | . | . | . | . | . | . | . |
| 21-225_188G11_HC | . | . | . | . | . | . | . | . | . | . | . | . | . | . | . | . | . | . | . | . | . | . | . | . | . | . |
| 21-225_226E7_HC | . | . | . | . | . | . | T | . | . | . | . | . | . | . | . | . | . | . | . | . | . | . | . | . | . | . |
| 21-225_25H9_HC | . | . | . | P | . | N | N | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | A | I | I | W | Y | D | - | - | - | G | S | Y | K | Y | Y | A | D | S | V | K | G | R | F |
| 21-225_152C11_HC | . | . | . | . | . | . | . | . | . | . | . | . | . | . | N | . | . | . | . | . | . | . | . | . | . | . |
| 21-225_152F6_HC | . | . | . | . | . | . | . | . | . | . | . | . | . | . | . | . | . | . | . | . | . | . | . | . | . | . |
| 21-225_153D2_HC | . | . | . | . | . | . | . | . | . | . | . | . | . | . | . | . | . | . | . | . | . | . | . | . | . | . |
| 21-225_157G8_HC | . | . | . | . | . | . | . | . | . | . | . | . | . | . | . | . | . | . | . | . | . | . | . | . | . | . |
| 21-225_160B1_HC | . | . | . | . | V | . | . | . | . | . | . | . | . | . | . | . | . | . | . | . | . | . | . | . | . | . |
| 21-225_172B7_HC | . | . | . | . | . | . | . | . | . | . | . | . | . | . | N | . | . | . | V | . | . | . | . | . | . | . |
| 21-225_173A11_HC | . | . | . | . | . | . | . | . | . | . | . | . | . | . | N | . | . | . | . | . | . | . | . | . | . | . |
| 21-225_178G10_HC | . | . | . | . | V | . | . | . | . | . | . | . | . | . | N | . | . | . | . | . | . | . | . | . | . | . |
| 21-225_188G11_HC | . | . | . | T | . | . | . | . | . | . | . | . | . | . | . | . | . | . | . | . | . | . | . | . | . | . |
| 21-225_226E7_HC | . | . | . | . | . | . | . | . | . | . | . | . | . | . | N | . | . | . | . | . | . | . | . | . | . | . |
| 21-225_25H9_HC | . | . | . | S | . | . | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_152C11_HC | . | . | . | . | . | . | . | . | . | . | . | . | . | . | . | . | . | . | . | . | . | . | . | . | . | . |
| 21-225_152F6_HC | . | . | . | . | . | . | . | . | . | . | . | . | . | . | . | . | . | . | . | . | . | . | . | . | . | . |
| 21-225_153D2_HC | . | . | . | . | . | . | . | . | . | . | V | . | . | . | . | . | . | . | . | . | . | . | . | . | . | . |
| 21-225_157G8_HC | . | V | . | . | . | . | . | . | . | . | . | . | . | . | . | . | . | . | . | . | . | . | . | . | . | . |
| 21-225_160B1_HC | . | . | . | . | . | . | . | . | . | . | . | . | . | . | . | . | . | . | . | . | . | . | . | . | . | . |
| 21-225_172B7_HC | . | . | . | . | . | . | . | . | . | . | . | . | . | . | . | . | . | . | . | . | . | . | . | . | . | . |
| 21-225_173A11_HC | . | . | . | . | . | . | . | . | . | . | . | . | . | . | . | . | . | . | . | . | . | . | . | . | . | . |
| 21-225_178G10_HC | . | . | . | . | . | . | F | . | . | . | . | . | . | . | . | . | . | . | . | . | . | . | . | . | . | . |
| 21-225_188G11_HC | . | . | . | . | . | . | . | . | . | . | . | F | . | . | . | . | . | . | . | . | . | . | . | . | . | . |
| 21-225_226E7_HC | . | . | . | . | . | . | F | . | . | . | . | . | . | . | . | H | . | . | . | . | D | . | . | . | . | . |
| 21-225_25H9_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | E | | Y | D | F | W | - | - | - | - | - | - | - | - | - | - | - | - | - | - | - | - |
| 21-225_152C11_HC | . | . | . | . | . | A | . | . | . | . | . | . | . | . | . | . | . | . | . | . | . | . | . | . | . | . |
| 21-225_152F6_HC | . | . | . | . | . | G | . | . | . | . | . | . | . | . | . | . | . | . | . | . | . | . | . | . | . | . |
| 21-225_153D2_HC | . | . | . | . | . | G | . | . | . | . | . | . | . | . | . | . | . | . | . | . | . | . | . | . | . | . |
| 21-225_157G8_HC | . | . | . | . | . | G | . | . | . | . | . | . | . | . | . | . | . | . | . | . | . | . | . | . | . | . |
| 21-225_160B1_HC | . | . | . | . | . | A | . | . | . | . | . | . | . | . | . | . | . | . | . | . | . | . | . | . | . | . |
| 21-225_172B7_HC | . | . | . | . | . | A | . | . | . | . | . | . | . | . | . | . | . | . | . | . | . | . | . | . | . | . |
| 21-225_173A11_HC | . | . | . | . | . | A | . | . | . | . | . | . | . | . | . | . | . | . | . | . | . | . | . | . | . | . |
| 21-225_178G10_HC | . | . | . | . | . | R | . | . | . | . | . | . | . | . | . | . | . | . | . | . | . | . | . | . | . | . |
| 21-225_188G11_HC | . | . | . | . | . | R | . | . | . | . | . | . | . | . | . | . | . | . | . | . | . | . | . | . | . | . |
| 21-225_226E7_HC | . | . | . | . | . | N | . | . | . | . | . | . | . | . | . | . | . | . | . | . | . | . | . | . | . | . |
| 21-225_25H9_HC | . | . | . | . | . | G | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | S | G | Y | F | D | Y | W | G | Q | G | T | L | V | T | V | S | S |
| 21-225_152C11_HC | . | . | . | . | . | . | . | . | . | . | . | . | I | . | . | . | . | . | . |
| 21-225_152F6_HC | . | . | . | . | . | L | G | . | . | . | . | . | . | . | . | . | . | . | . |
| 21-225_153D2_HC | . | . | . | . | . | L | G | . | . | . | . | . | . | . | . | . | . | . | . |
| 21-225_157G8_HC | . | . | . | . | F | . | . | S | . | . | . | . | . | . | . | . | . | . | . |
| 21-225_160B1_HC | . | . | . | . | . | Y | . | . | . | . | . | . | . | . | . | . | . | . | . |
| 21-225_172B7_HC | . | . | . | . | . | W | . | . | . | . | . | . | . | . | . | . | . | . | . |
| 21-225_173A11_HC | . | . | . | . | F | . | . | S | . | . | . | . | . | . | . | . | . | . | . |
| 21-225_178G10_HC | . | . | . | . | H | . | . | . | . | . | . | . | . | . | . | . | . | . | . |
| 21-225_188G11_HC | . | . | . | . | H | . | . | . | . | . | . | . | . | . | . | . | . | . | . |
| 21-225_226E7_HC | . | . | . | . | . | Y | G | . | . | . | . | . | . | . | . | . | . | . | . |
| 21-225_25H9_HC | . | . | . | . | F | . | . | S | . | . | . | . | . | . | . | . | . | . | . |

**FIGURE 57**

Table 81. Consensus 47 -VH3|3-33/D5|5-18|RF3/JH6 (SEQ ID NO: 50298):

QVQLVES-GGGVVQPGRSLRLSCAASG-FTFSS-----YGMHWVRQAPGKGLEWVAVIWYD---GSNKYYADSVKGRFTISRDNSKNTLYLQMNSLRAEDTAVYYCARDRDYGD-------------------YGMDVWGQGTTVTVSS wherein:

M at position 41 can be substituted with L

Y at position 69 can be substituted with N

A at position 71 can be substituted with E

D at position 109 can be substituted with W

R at position 110 can be substituted with R, G or Y

D at position 111 can be substituted with S or Y

G at position 113 can be substituted with Y

D at position 114 can be substituted with Y

null (-) at position 115 can be substituted with P

null (-) at position 116 can be substituted with P

null (-) at position 117 can be substituted with Y

null (-) at position 131 can be substituted with Y

null (-) at position 132 can be substituted with Y

null (-) at position 133 can be substituted with Y

Y at position 134 can be substituted with D

Ser Tyr Gly Xaa His (SEQ ID NO: 50087)

Val Ile Trp Tyr Asp Gly Ser Asn Lys Xaa Tyr Xaa Asp Ser Val Lys Gly (SEQ ID NO: 50088)

**FIGURE 57**

Xaa Xaa Xaa Tyr Xaa Xaa Xaa Xaa Xaa Xaa Xaa Xaa Xaa Gly Met Asp Val (SEQ ID NO: 50089)

Ser Tyr Gly Met His (SEQ ID NO: 50564)

Val Ile Trp Tyr Asp Gly Ser Asn Lys Tyr Tyr Ala Asp Ser Val Lys Gly (SEQ ID NO: 50565)

Asp Arg Asp Tyr Gly Asp Tyr Gly Met Asp Val (SEQ ID NO: 50566)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | E | S | - | G | G | G | V | V | Q | P | G | R | S | L | R | L | S | C | A | A | S |
| 21-225_147B9_HC | . | . | . | . | . | . | . | . | . | . | . | . | . | . | . | . | . | . | . | . | . | . | . | . | . | . |
| 21-225_148C8_HC | . | . | . | . | . | . | . | . | . | . | . | . | . | . | . | . | . | . | . | . | . | . | . | . | . | . |
| 21-225_149A11_HC | . | . | . | . | . | . | . | . | . | . | . | . | . | . | . | . | . | . | . | . | . | . | . | . | . | . |
| 21-225_155E10_HC | . | . | . | . | . | . | . | . | . | . | . | . | . | . | . | . | . | . | . | . | . | . | . | . | . | . |
| 21-225_190C3_HC | . | . | . | . | . | . | . | . | . | . | . | . | . | . | . | . | . | . | . | . | . | . | . | . | . | . |
| 21-225_190D8_HC | . | . | . | . | . | . | . | . | . | . | D | . | . | . | . | . | . | . | . | . | . | . | . | E | . | . |
| 21-225_211A3_HC | . | . | . | . | . | . | . | . | . | . | . | . | . | . | . | . | . | . | . | . | . | . | . | . | V | . |
| 21-225_212H11_HC | . | . | . | . | . | . | . | . | . | . | . | . | . | . | . | . | . | . | . | . | . | . | . | . | V | . |
| 21-225_213E5_HC | . | . | . | . | . | . | . | . | . | . | . | . | . | . | . | . | . | . | . | . | . | . | . | . | V | . |
| 21-225_217B10_HC | . | . | . | . | . | . | . | . | . | . | . | . | . | . | . | . | . | . | . | . | . | . | . | . | V | . |
| 21-225_218G4_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | G | M | H | W | V | R | Q | A | P | G | K | G | L |
| 21-225_147B9_HC | . | . | . | . | . | . | . | . | . | . | . | . | . | . | . | . | . | . | . | . | . | . | . | . | . | . |
| 21-225_148C8_HC | . | . | . | . | . | . | . | . | . | . | . | . | . | . | . | . | . | . | . | . | . | . | . | . | . | . |
| 21-225_149A11_HC | . | . | . | . | . | . | . | . | . | . | . | . | . | . | . | . | . | . | . | . | . | . | . | . | . | . |
| 21-225_155E10_HC | . | . | . | . | . | . | . | . | . | . | . | . | . | . | . | . | . | . | . | . | . | . | . | . | . | . |
| 21-225_190C3_HC | . | . | . | . | . | . | . | . | . | . | . | . | . | . | . | . | . | . | . | . | . | . | . | . | . | . |
| 21-225_190D8_HC | . | . | . | . | . | . | . | . | . | . | . | . | . | . | L | . | . | . | . | . | . | . | . | . | . | . |
| 21-225_211A3_HC | . | . | I | . | . | . | . | . | . | . | . | . | . | . | . | . | . | A | . | . | . | . | . | . | . | . |
| 21-225_212H11_HC | . | . | I | . | . | . | . | . | . | . | . | . | . | . | . | . | . | A | . | . | . | . | . | . | . | . |
| 21-225_213E5_HC | . | . | I | . | . | . | . | . | . | . | . | . | . | . | . | . | . | A | . | . | . | . | . | . | . | . |
| 21-225_217B10_HC | . | . | I | . | . | . | . | . | . | . | . | . | . | . | . | . | . | A | . | . | . | . | . | . | . | . |
| 21-225_218G4_HC | . | . | . | . | . | . | . | . | . | . | . | . | . | . | L | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | A | V | I | W | Y | D | - | - | - | G | S | N | K | Y | Y | A | D | S | V | K | G | R | F |
| 21-225_147B9_HC | . | . | . | . | . | . | . | . | . | . | . | . | . | . | . | . | . | . | . | . | . | . | . | . | . | . |
| 21-225_148C8_HC | . | . | . | . | . | . | . | . | . | . | . | . | . | . | . | . | . | . | . | . | . | . | . | . | . | . |
| 21-225_149A11_HC | . | . | . | . | . | . | . | . | . | . | . | . | . | . | . | . | . | . | . | . | . | . | . | . | . | . |
| 21-225_155E10_HC | . | . | . | . | . | . | . | . | . | . | . | . | . | . | . | . | . | . | . | . | . | . | . | . | . | . |
| 21-225_190C3_HC | . | . | . | . | . | . | . | . | . | . | . | . | . | . | . | . | . | . | . | . | . | . | . | . | . | . |
| 21-225_190D8_HC | . | . | . | . | . | . | . | . | . | . | . | . | . | . | . | . | . | . | . | . | . | . | . | . | . | . |
| 21-225_211A3_HC | . | . | . | . | . | . | . | . | . | . | . | . | . | . | . | . | N | . | E | . | . | . | . | . | . | . |
| 21-225_212H11_HC | . | . | . | . | . | . | . | . | . | . | . | . | . | . | . | . | N | . | E | . | . | . | . | . | . | . |
| 21-225_213E5_HC | . | . | . | . | . | . | . | . | . | . | . | . | . | . | . | . | N | . | E | . | . | . | . | . | . | . |
| 21-225_217B10_HC | . | . | . | . | . | . | . | . | . | . | . | . | . | . | . | . | N | . | E | . | . | . | . | . | . | . |
| 21-225_218G4_HC | . | . | . | . | . | . | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_147B9_HC | . | . | . | . | . | . | . | . | . | . | . | . | . | . | . | . | . | . | . | . | . | . | . | . | . | . |
| 21-225_148C8_HC | . | . | . | . | . | . | . | . | . | . | . | . | . | . | . | . | . | . | . | . | . | . | . | . | . | . |
| 21-225_149A11_HC | . | . | . | . | . | . | . | . | . | . | . | . | . | . | . | . | . | . | . | . | . | . | . | . | . | . |
| 21-225_155E10_HC | . | . | . | . | . | . | . | . | . | . | . | . | . | . | . | . | . | . | . | . | . | . | . | . | . | . |
| 21-225_190C3_HC | . | . | . | . | . | . | . | . | . | . | . | . | . | . | . | . | . | . | . | . | . | . | . | . | . | . |
| 21-225_190D8_HC | . | . | . | . | . | . | . | . | . | . | . | . | . | . | . | . | . | . | . | . | . | . | . | . | . | . |
| 21-225_211A3_HC | . | . | . | . | . | . | . | . | . | . | . | . | . | . | . | . | . | . | . | . | . | . | . | . | . | . |
| 21-225_212H11_HC | . | . | . | . | . | . | . | . | . | . | . | . | . | . | . | . | . | . | . | . | . | . | . | . | . | . |
| 21-225_213E5_HC | . | . | . | . | . | . | . | . | . | . | . | . | . | . | . | . | . | . | . | . | . | . | . | . | . | . |
| 21-225_217B10_HC | . | . | . | . | . | . | . | . | . | . | . | . | . | . | . | . | . | . | . | . | . | . | . | . | . | . |
| 21-225_218G4_HC | . | . | . | . | . | . | . | . | . | . | . | . | . | . | . | . | . | . | . | V | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | D | | | Y | G | | - | - | - | - | - | - | - | - | - | - | - | - | - | - | - | - |
| 21-225_147B9_HC | . | . | . | . | . | R | D | . | . | D | P | P | Y | . | . | . | . | . | . | . | . | . | . | . | . | . |
| 21-225_148C8_HC | . | . | . | . | . | R | D | . | . | D | P | P | Y | . | . | . | . | . | . | . | . | . | . | . | . | . |
| 21-225_149A11_HC | . | . | . | . | . | R | D | . | . | D | P | P | Y | . | . | . | . | . | . | . | . | . | . | . | . | . |
| 21-225_155E10_HC | . | . | . | . | . | R | D | . | . | D | P | P | Y | . | . | . | . | . | . | . | . | . | . | . | . | . |
| 21-225_190C3_HC | . | . | . | N | W | Y | Y | . | Y | - | . | . | . | . | . | . | . | . | . | . | . | . | . | . | . | . |
| 21-225_190D8_HC | . | . | . | N | W | Y | Y | . | Y | - | . | . | . | . | . | . | . | . | . | . | . | . | . | . | . | . |
| 21-225_211A3_HC | . | . | . | . | . | G | S | . | . | Y | . | . | . | . | . | . | . | . | . | . | . | . | . | . | . | . |
| 21-225_212H11_HC | . | . | . | . | . | G | S | . | . | Y | . | . | . | . | . | . | . | . | . | . | . | . | . | . | . | . |
| 21-225_213E5_HC | . | . | . | . | . | G | S | . | . | Y | . | . | . | . | . | . | . | . | . | . | . | . | . | . | . | . |
| 21-225_217B10_HC | . | . | . | . | . | G | S | . | . | Y | . | . | . | . | . | . | . | . | . | . | . | . | . | . | . | . |
| 21-225_218G4_HC | . | . | . | N | W | Y | Y | . | Y | - | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | Y | G | M | D | V | W | G | Q | G | T | T | V | T | V | S | S |
| 21-225_147B9_HC | Y | Y | Y | . | . | . | . | . | . | . | . | . | . | . | . | . | . | . | . |
| 21-225_148C8_HC | Y | Y | Y | . | . | . | . | . | . | . | . | . | . | . | . | . | . | . | . |
| 21-225_149A11_HC | Y | Y | Y | . | . | . | . | . | . | . | . | . | . | . | . | . | . | . | . |
| 21-225_155E10_HC | Y | Y | Y | . | . | . | . | . | . | . | . | . | . | . | . | . | . | . | . |
| 21-225_190C3_HC | . | . | . | . | . | . | . | . | . | . | . | . | . | . | . | . | . | . | . |
| 21-225_190D8_HC | . | . | . | . | . | . | . | . | . | . | . | . | . | . | . | . | . | . | . |
| 21-225_211A3_HC | . | . | . | D | . | . | . | . | . | . | . | . | . | . | . | . | . | . | . |
| 21-225_212H11_HC | . | . | . | D | . | . | . | . | . | . | . | . | . | . | . | . | . | . | . |
| 21-225_213E5_HC | . | . | . | D | . | . | . | . | . | . | . | . | . | . | . | . | . | . | . |
| 21-225_217B10_HC | . | . | . | D | . | . | . | . | . | . | . | . | . | . | . | . | . | . | . |
| 21-225_218G4_HC | . | . | . | . | . | . | . | . | . | . | . | . | . | . | . | . | . | . | . |

**FIGURE 57**

Table 82. Consensus 48 -VH3|3-23/D4|4-17|RF2/JH6 (SEQ ID NO: 50299):

EVQLLES-GGGLVQPGGSLRLSCAASG-FTFSS-----YAMSWVRQAPGKGLEWVSAISGS---GGNTFYADSVKGRFTISRDNSKNTLYLQMNSLRAEDTAVYYCAKLGKDYY------------------YYGMDVWGQGTTVTVSS wherein:

S at position 33 can be substituted with N or T

Y at position 39 can be substituted with S

A at position 40 can be substituted with V

S at position 42 can be substituted with T or N

A at position 57 can be substituted with G

I at position 58 can be substituted with S or V

S at position 59 can be substituted with V

S at position 61 can be substituted with R

G at position 66 can be substituted with A, S or V

N at position 67 can be substituted with R or K

F at position 69 can be substituted with Y

Y at position 70 can be substituted with N

A at position 71 can be substituted with T

K at position 75 can be substituted with T

L at position 109 can be substituted with D or E

G at position 110 can be substituted with R

K at position 111 can be substituted with G or I

**FIGURE 57**

D at position 112 can be substituted with Q or Y

Y at position 113 can be substituted with W

Y at position 114 can be substituted with H or L

Y at position 133 can be substituted with null (-), I, or L

Y at position 134 can be substituted with G

M at position 136 can be substituted with V

Xaa Xaa Xaa Met Xaa (SEQ ID NO: 50090)

Xaa Xaa Xaa Gly Xaa Gly Xaa Xaa Thr Xaa Xaa Xaa Asp Ser Val Xaa Gly (SEQ ID NO: 50091)

Xaa Xaa Xaa Xaa Xaa Xaa Xaa Xaa Gly Xaa Asp Val (SEQ ID NO: 50092)

Ser Tyr Ala Met Ser (SEQ ID NO: 50567)

Ala Ile Ser Gly Ser Gly Gly Asn Thr Phe Tyr Ala Asp Ser Val Lys Gly (SEQ ID NO: 50568)

Leu Gly Lys Asp Tyr Tyr Tyr Tyr Gly Met Asp Val (SEQ ID NO: 50569)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | E | V | Q | L | L | E | S | - | G | G | G | L | V | Q | P | G | G | S | L | R | L | S | C | A | A | S |
| 21-225_171A9_HC | . | . | . | . | . | . | . | . | . | . | . | . | A | . | . | . | . | . | . | . | . | . | . | . | . | . |
| 21-225_224B1_HC | . | . | . | V | . | . | . | . | . | . | . | . | . | . | . | . | . | . | . | . | . | . | . | . | . | . |
| 21-225_44E10_HC | . | . | . | . | . | . | . | . | . | . | . | . | . | . | S | . | . | . | . | . | . | . | . | . | . | . |
| 21-225_47C7_HC | . | . | . | . | . | . | . | . | . | . | . | . | . | . | . | . | . | . | . | . | . | . | . | . | . | . |
| 21-225_54G3_HC | . | . | . | . | . | . | . | . | . | . | . | . | . | . | . | . | . | . | . | . | . | . | . | . | . | . |
| 21-225_57F8_HC | . | . | . | . | . | . | . | . | . | . | . | . | . | . | . | . | . | . | . | . | . | . | . | V | V | . |
| 21-225_5A4_HC | . | . | . | V | . | . | . | . | . | . | . | . | . | . | . | . | . | . | . | . | . | . | . | . | . | . |
| 21-225_60A11_HC | . | . | . | . | . | . | . | . | . | . | . | . | . | . | . | . | . | . | . | . | . | . | . | . | . | . |
| 21-225_62F7_HC | . | . | . | . | . | . | . | . | . | . | . | . | . | . | . | . | . | . | . | . | . | . | . | . | . | . |
| 21-225_66C10_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | A | M | S | W | V | R | Q | A | P | G | K | G | L |
| 21-225_171A9_HC | . | . | . | . | . | . | . | . | . | . | . | . | . | . | . | T | . | . | . | . | . | . | . | . | . | . |
| 21-225_224B1_HC | . | . | . | . | . | . | . | . | . | . | . | . | . | . | . | . | . | . | . | . | . | . | . | . | . | . |
| 21-225_44E10_HC | . | . | . | . | . | . | . | . | . | . | . | . | . | . | . | N | . | . | . | . | . | . | . | . | . | . |
| 21-225_47C7_HC | . | . | . | . | . | . | N | . | . | . | . | . | . | . | . | . | . | . | . | . | . | . | . | . | . | . |
| 21-225_54G3_HC | . | . | . | . | . | . | T | . | . | . | . | . | . | . | . | . | . | . | . | . | . | . | . | . | . | . |
| 21-225_57F8_HC | . | . | . | . | . | . | N | . | . | . | . | . | . | . | . | . | . | . | . | . | . | . | . | . | . | . |
| 21-225_5A4_HC | . | . | L | . | . | . | . | . | . | . | . | . | S | . | . | . | . | . | . | . | . | . | . | . | . | . |
| 21-225_60A11_HC | . | . | . | . | . | . | N | . | . | . | . | . | . | . | . | T | . | . | . | . | . | . | . | . | . | . |
| 21-225_62F7_HC | . | . | . | . | . | . | . | . | . | . | . | . | . | V | . | . | . | . | . | . | . | . | . | . | . | . |
| 21-225_66C10_HC | . | . | . | . | . | . | . | . | . | . | . | . | . | V | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | S | A | I | S | G | S | - | - | - | G | G | N | T | | Y | A | D | S | V | K | G | R | F |
| 21-225_171A9_HC | . | . | . | . | . | . | . | . | . | . | . | . | . | . | . | . | F | N | . | . | . | . | . | . | . | . |
| 21-225_224B1_HC | . | . | . | . | . | . | . | . | . | . | . | . | . | . | . | . | Y | . | . | . | . | . | . | . | . | . |
| 21-225_44E10_HC | . | . | . | . | G | V | V | . | . | . | . | . | . | . | R | . | Y | . | . | . | . | . | . | . | . | . |
| 21-225_47C7_HC | . | . | . | . | G | . | . | . | . | . | . | . | . | S | R | . | Y | . | . | . | . | . | . | . | . | . |
| 21-225_54G3_HC | . | . | . | . | . | . | . | . | . | . | . | . | . | V | . | . | F | . | . | . | . | . | . | . | . | . |
| 21-225_57F8_HC | . | . | . | . | . | S | . | . | . | . | . | . | . | . | . | . | F | . | . | . | . | . | T | . | . | V |
| 21-225_5A4_HC | . | . | . | . | . | . | . | . | R | . | . | . | . | . | . | . | F | . | T | . | . | . | . | . | . | . |
| 21-225_60A11_HC | . | . | . | . | . | . | . | . | . | . | . | . | . | . | . | . | F | . | . | . | . | . | T | . | . | V |
| 21-225_62F7_HC | . | . | . | . | G | . | . | . | . | . | . | . | . | A | K | . | Y | . | . | . | . | . | . | . | . | . |
| 21-225_66C10_HC | . | . | . | . | G | . | . | . | . | . | . | . | . | A | R | . | Y | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_171A9_HC | . | . | . | . | . | . | . | . | . | . | . | . | . | . | . | . | . | . | . | . | . | . | . | . | . | . |
| 21-225_224B1_HC | . | . | . | . | . | . | . | . | . | . | . | . | . | H | . | . | . | . | . | . | . | . | . | . | . | . |
| 21-225_44E10_HC | I | . | . | . | . | . | . | . | . | . | . | . | . | . | . | . | . | . | . | . | . | . | . | . | . | . |
| 21-225_47C7_HC | . | . | . | . | . | . | . | . | . | . | . | . | . | . | . | . | . | . | . | . | . | . | . | . | . | . |
| 21-225_54G3_HC | . | . | . | . | . | . | . | . | . | . | . | F | . | . | . | . | . | . | . | . | . | . | . | . | . | . |
| 21-225_57F8_HC | . | . | . | . | . | . | . | . | . | . | . | . | . | . | . | . | . | . | . | . | . | . | . | . | . | . |
| 21-225_5A4_HC | . | . | . | . | . | . | . | . | . | . | . | . | . | . | . | . | . | . | . | . | . | . | . | . | . | . |
| 21-225_60A11_HC | . | . | . | . | . | . | . | . | . | . | . | . | . | . | . | . | . | . | . | . | . | . | . | . | . | . |
| 21-225_62F7_HC | . | . | . | . | . | . | . | . | . | . | . | . | . | . | . | . | . | . | . | . | . | . | . | . | . | . |
| 21-225_66C10_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | K | L | G | K | D | Y | Y | - | - | - | - | - | - | - | - | - | - | - | - | - | - | - | - |
| 21-225_171A9_HC | . | . | . | . | . | . | I | . | . | . | . | . | . | . | . | . | . | . | . | . | . | . | . | . | . | . |
| 21-225_224B1_HC | . | . | . | G | E | . | G | Y | . | . | . | . | . | . | . | . | . | . | . | . | . | . | . | . | . | . |
| 21-225_44E10_HC | . | . | . | . | D | R | G | Q | W | L | . | . | . | . | . | . | . | . | . | . | . | . | . | . | . | . |
| 21-225_47C7_HC | . | . | . | . | D | R | G | Q | W | L | . | . | . | . | . | . | . | . | . | . | . | . | . | . | . | . |
| 21-225_54G3_HC | . | . | . | . | . | . | . | . | . | . | . | . | . | . | . | . | . | . | . | . | . | . | . | . | . | . |
| 21-225_57F8_HC | . | . | . | . | . | . | . | . | . | H | . | . | . | . | . | . | . | . | . | . | . | . | . | . | . | . |
| 21-225_5A4_HC | . | . | . | . | . | . | . | . | . | . | . | . | . | . | . | . | . | . | . | . | . | . | . | . | . | . |
| 21-225_60A11_HC | . | . | . | . | . | . | . | . | . | H | . | . | . | . | . | . | . | . | . | . | . | . | . | . | . | . |
| 21-225_62F7_HC | . | . | . | E | . | . | I | . | . | . | . | . | . | . | . | . | . | . | . | . | . | . | . | . | . | . |
| 21-225_66C10_HC | . | . | . | E | . | . | I | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | Y | Y | G | M | D | V | W | G | Q | G | T | T | V | T | V | S | S |
| 21-225_171A9_HC | . | . | . | . | . | . | . | . | . | . | . | . | . | . | . | . | . | . | . |
| 21-225_224B1_HC | . | . | . | . | . | V | . | . | . | . | . | . | . | . | . | . | . | . | . |
| 21-225_44E10_HC | . | . | L | G | . | . | . | . | . | . | . | . | . | . | . | . | . | . | . |
| 21-225_47C7_HC | . | . | I | G | . | . | . | . | . | . | . | . | . | . | . | . | . | . | . |
| 21-225_54G3_HC | . | . | . | . | . | . | . | . | . | . | . | . | . | . | . | . | . | . | . |
| 21-225_57F8_HC | . | . | . | . | . | . | . | . | . | . | . | . | . | . | . | . | . | . | . |
| 21-225_5A4_HC | . | . | . | . | . | . | . | . | . | . | . | . | . | . | . | . | . | . | . |
| 21-225_60A11_HC | . | . | . | . | . | . | . | . | . | . | . | . | . | . | . | . | . | . | . |
| 21-225_62F7_HC | . | . | - | . | . | . | . | . | . | . | . | . | . | . | . | . | . | . | . |
| 21-225_66C10_HC | . | . | - | . | . | . | . | . | . | . | . | . | . | . | . | . | . | . | . |

**FIGURE 57**

Table 83. Consensus 49 -VH3|3-23/D7|7-27|RF1/JH4 (SEQ ID NO: 50300):

EVQLLES-GGGLVQPGGSLRLSCAASG-FTFSS-----YVMSWVRQAPGKGLEWVSAMSGS---GGRTYYADSVKGRFTISRDNSKNTLYLQMNSLRAEDTAVYYCAKLTA------------------------FDYWGQGTLVTVSS wherein:

V at position 40 can be substituted with A

S at position 42 can be substituted with N

A at position 57 can be substituted with G, T or S

M at position 58 can be substituted with I or T

G at position 66 can be substituted with N or V

R at position 67 can be substituted with N or W

Y at position 69 can be substituted with F or N

K at position 75 can be substituted with N

G at position 76 can be substituted with D

L at position 109 can be substituted with V, Y or F

T at position 110 can be substituted with E, F or G

A at position 111 can be substituted with G, L, W, null (-) or F

null (-) at position 112 can be substituted with G or M

null (-) at position 113 can be substituted with V

null (-) at position 114 can be substituted with G

null (-) at position 133 can be substituted with A

null (-) at position 134 can be substituted with G

**FIGURE 57**

null (-) at position 135 can be substituted with I or F

F at position 136 can be substituted with N or null (-)

D at position 137 can be substituted with G or I

Y at position 138 can be substituted with D

Ser Tyr Xaa Met Xaa (SEQ ID NO: 50093)

Xaa Xaa Ser Gly Ser Gly Xaa Xaa Thr Xaa Tyr Ala Asp Ser Val Xaa Xaa (SEQ ID NO: 50094)

Xaa Xaa Xaa Xaa Xaa Xaa Xaa Xaa Xaa Xaa Xaa Xaa (SEQ ID NO: 50095)

Ser Tyr Val Met Ser (SEQ ID NO: 50570)

Ala Met Ser Gly Ser Gly Gly Arg Thr Tyr Tyr Ala Asp Ser Val Lys Gly (SEQ ID NO: 50571)

Leu Thr Ala Phe Asp Tyr (SEQ ID NO: 50572)

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | E | V | Q | L | L | E | S | - | G | G | G | L | V | Q | P | G | G | S | L | R | L | S | C | A | A | S |
| 21-225_157G5_HC | . | . | . | . | . | . | . | . | . | . | . | . | . | . | . | . | . | . | . | . | . | . | . | . | . | . |
| 21-225_167D12_HC | . | . | . | . | . | . | . | . | . | . | . | . | . | . | . | . | . | . | . | . | . | . | . | . | . | . |
| 21-225_177D3_HC | . | . | . | . | . | . | . | . | . | . | . | . | . | . | . | . | . | . | . | . | . | . | . | . | . | . |
| 21-225_51C5_HC | . | . | . | . | . | . | . | . | . | . | . | . | . | . | . | . | . | . | . | . | . | . | . | . | . | . |
| 21-225_51D5_HC | . | . | . | . | . | . | . | . | . | . | . | . | . | . | . | . | . | . | . | . | . | . | . | . | . | . |
| 21-225_57B8_HC | . | . | . | . | . | . | . | . | . | . | . | . | . | . | . | . | . | . | . | . | . | . | . | . | . | . |
| 21-225_58F11_HC | . | . | . | . | . | . | . | . | . | . | . | . | . | . | . | . | . | . | . | . | . | . | . | . | . | . |
| 21-225_60A4_HC | . | . | . | . | . | . | . | . | . | . | . | . | . | . | . | . | . | . | . | . | . | . | . | . | . | . |
| 21-225_60F7_HC | . | . | . | . | . | . | . | . | . | . | . | . | . | . | . | . | . | . | . | . | . | . | . | . | . | . |
| 21-225_61E6_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | V | M | S | W | V | R | Q | A | P | G | K | G | L |
| 21-225_157G5_HC | . | . | . | . | . | . | . | . | . | . | . | . | . | . | . | . | . | . | . | . | . | . | . | . | . | . |
| 21-225_167D12_HC | . | . | . | . | . | . | . | . | . | . | . | . | . | . | . | . | . | . | . | . | . | . | . | . | . | . |
| 21-225_177D3_HC | . | . | . | . | . | . | . | . | . | . | . | . | . | . | . | N | . | . | . | . | . | . | . | . | . | . |
| 21-225_51C5_HC | . | . | . | . | . | . | . | . | . | . | . | . | . | . | . | . | . | . | . | . | T | . | . | . | . | . |
| 21-225_51D5_HC | . | . | . | . | . | . | . | . | . | . | . | . | . | . | . | . | . | . | . | . | T | . | . | . | . | . |
| 21-225_57B8_HC | . | . | . | . | . | R | . | . | . | . | . | . | . | A | . | . | . | . | . | . | . | . | . | . | . | . |
| 21-225_58F11_HC | . | . | . | . | . | . | . | . | . | . | . | . | . | A | . | . | . | . | . | . | . | . | . | . | . | . |
| 21-225_60A4_HC | . | . | . | . | . | . | . | . | . | . | . | . | . | . | . | . | . | . | . | . | . | . | . | . | . | . |
| 21-225_60F7_HC | . | . | . | . | . | . | . | . | . | . | . | . | . | . | . | . | . | . | . | . | . | . | . | . | . | . |
| 21-225_61E6_HC | . | . | . | . | . | R | . | . | . | . | . | . | . | A | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | H_CDR2 | | | | | | | | | | | | |
| CONSENSUS | E | W | V | S | A | M | S | G | S | - | - | - | G | G | R | T | Y | Y | A | D | S | V | K | G | R | F |
| 21-225_157G5_HC | D | . | . | . | . | . | . | . | . | . | . | . | . | . | . | . | . | . | . | . | . | . | . | . | . | . |
| 21-225_167D12_HC | . | . | . | . | . | . | . | . | . | . | . | . | . | . | . | . | . | . | . | . | . | . | . | . | . | . |
| 21-225_177D3_HC | . | . | . | . | G | . | . | . | . | . | . | . | . | . | . | . | . | . | . | . | . | . | . | D | . | . |
| 21-225_51C5_HC | . | . | . | . | T | . | . | . | . | . | . | . | . | . | . | . | . | . | . | . | . | . | N | . | . | . |
| 21-225_51D5_HC | . | . | . | . | T | . | . | . | . | . | . | . | . | . | . | . | . | . | . | . | . | . | N | . | . | . |
| 21-225_57B8_HC | . | . | . | . | . | I | . | . | . | . | . | . | . | . | N | . | F | . | . | . | . | . | . | . | . | . |
| 21-225_58F11_HC | . | . | . | . | . | I | . | . | . | . | . | . | . | . | N | . | F | . | . | . | . | . | . | . | . | . |
| 21-225_60A4_HC | . | . | . | . | S | I | . | . | . | . | . | . | . | . | W | . | N | . | . | . | . | . | . | . | . | . |
| 21-225_60F7_HC | . | . | . | . | G | I | . | . | . | . | . | . | . | N | . | . | . | . | . | . | . | . | . | . | . | . |
| 21-225_61E6_HC | . | . | . | . | . | T | . | . | . | . | . | . | . | V | N | . | F | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | H_FR3 | | | | | | | | | | | | |
| CONSENSUS | T | I | S | R | D | N | S | K | N | T | L | Y | L | Q | M | N | S | L | R | A | E | D | T | A | V | Y |
| 21-225_157G5_HC | . | . | . | . | . | . | . | . | . | . | . | . | . | . | . | . | . | . | . | . | . | . | . | . | . | . |
| 21-225_167D12_HC | . | . | . | . | . | . | . | . | . | . | . | . | . | . | . | . | . | . | . | . | . | . | . | . | . | . |
| 21-225_177D3_HC | . | . | . | . | . | . | . | . | . | . | . | S | . | . | . | . | . | . | . | . | . | . | . | . | . | . |
| 21-225_51C5_HC | . | V | . | . | . | . | . | . | . | . | . | . | . | . | . | S | . | . | . | . | . | . | . | . | . | . |
| 21-225_51D5_HC | . | V | . | . | . | . | . | . | . | . | . | . | . | . | . | S | . | . | . | . | . | . | . | . | . | . |
| 21-225_57B8_HC | . | . | . | . | . | . | . | . | . | . | . | . | . | . | . | . | . | . | . | . | . | . | . | . | . | . |
| 21-225_58F11_HC | . | . | . | . | Y | . | . | . | . | . | . | . | . | . | . | . | . | . | . | . | . | . | . | . | . | . |
| 21-225_60A4_HC | . | T | . | . | . | . | . | . | . | . | . | . | . | . | . | . | . | . | . | . | . | . | . | . | . | . |
| 21-225_60F7_HC | . | . | . | . | . | . | . | . | . | . | . | . | . | . | . | . | . | . | . | . | . | . | S | . | . | . |
| 21-225_61E6_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | K | L | T | | - | - | - | - | - | - | - | - | - | - | - | - | - | - | - | - | - | - | - |
| 21-225_157G5_HC | . | . | . | . | Y | . | W | . | . | . | . | . | . | . | . | . | . | . | . | . | . | . | . | . | . | . |
| 21-225_167D12_HC | . | . | . | . | Y | . | W | . | . | . | . | . | . | . | . | . | . | . | . | . | . | . | . | . | . | . |
| 21-225_177D3_HC | . | . | . | R | V | . | F | . | . | . | . | . | . | . | . | . | . | . | . | . | . | . | . | . | . | . |
| 21-225_51C5_HC | . | . | . | R | V | . | A | . | . | . | . | . | . | . | . | . | . | . | . | . | . | . | . | . | . | . |
| 21-225_51D5_HC | . | . | . | R | V | . | A | . | . | . | . | . | . | . | . | . | . | . | . | . | . | . | . | . | . | . |
| 21-225_57B8_HC | . | . | . | . | . | E | L | G | . | . | . | . | . | . | . | . | . | . | . | . | . | . | . | . | . | . |
| 21-225_58F11_HC | . | . | . | . | F | F | G | M | V | G | . | . | . | . | . | . | . | . | . | . | . | . | . | . | . | . |
| 21-225_60A4_HC | . | . | . | R | . | . | G | . | . | . | . | . | . | . | . | . | . | . | . | . | . | . | . | . | . | . |
| 21-225_60F7_HC | . | . | G | S | . | G | - | . | . | . | . | . | . | . | . | . | . | . | . | . | . | . | . | . | . | . |
| 21-225_61E6_HC | . | . | . | . | . | E | L | G | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | - | F | D | Y | W | G | Q | G | T | L | V | T | V | S | S |
| 21-225_157G5_HC | . | . | . | . | . | N | G | . | . | . | . | . | . | . | . | . | . | . | . |
| 21-225_167D12_HC | . | . | . | . | . | N | G | . | . | . | . | . | . | . | . | . | . | . | . |
| 21-225_177D3_HC | . | . | . | . | . | . | . | . | . | . | . | . | . | . | . | . | . | . | . |
| 21-225_51C5_HC | . | . | . | . | . | . | . | . | . | . | . | . | . | . | . | . | . | . | . |
| 21-225_51D5_HC | . | . | . | . | . | . | . | . | . | . | . | . | . | . | . | . | . | . | . |
| 21-225_57B8_HC | . | . | . | . | I | . | . | . | . | . | . | . | . | . | . | . | . | . | . |
| 21-225_58F11_HC | . | . | A | G | F | . | . | . | . | . | . | . | . | . | . | . | . | . | . |
| 21-225_60A4_HC | . | . | . | . | . | . | . | . | . | . | . | . | . | . | . | . | . | . | . |
| 21-225_60F7_HC | . | . | . | . | . | - | I | D | . | . | . | . | . | . | . | . | . | . | . |
| 21-225_61E6_HC | . | . | . | . | I | . | . | . | . | . | . | . | . | . | . | . | . | . | . |

**FIGURE 57**

Table 84. Consensus 50 -VH3|3-30.3/D5|5-24|RF3/JH6 (SEQ ID NO: 50301):

QVQLVES-GGGVVQPGRSLRLSCAASG-FTFSS-----YGMHWVRQAPGKGLEWVAIISYA---GSNKYYADSVKGRFTISRDNSKNTLYLQMNSLRAEDTAVYYCVRRGYSYG------------------GYGMDVWGQGTTVTVSS wherein:

S at position 33 can be substituted with Y

M at position 41 can be substituted with L

I at position 57 can be substituted with V

A at position 61 can be substituted with G, D, S or V

S at position 66 can be substituted with I, N, R or T

K at position 68 can be substituted with N or Q

Y at position 69 can be substituted with S, D or H

Y at position 70 can be substituted with S

R at position 109 can be substituted with E

G at position 110 can be substituted with D

Y at position 111 can be substituted with R

S at position 112 can be substituted with Y

Y at position 113 can be substituted with C

G at position 114 can be substituted with S

null (-) at position 115 can be substituted with G

null (-) at position 116 can be substituted with T

null (-) at position 117 can be substituted with S

**FIGURE 57**

null (-) at position 118 can be substituted with C

null (-) at position 129 can be substituted with P

null (-) at position 130 can be substituted with Y

null (-) at position 131 can be substituted with Y

null (-) at position 132 can be substituted with Y

G at position 133 can be substituted with Y

Xaa Tyr Gly Xaa His (SEQ ID NO: 50096)

Xaa Ile Ser Tyr Xaa Gly Xaa Asn Xaa Xaa Xaa Ala Asp Ser Val Lys Gly (SEQ ID NO: 50097)

Xaa Xaa Xaa Xaa Xaa Xaa Xaa Xaa Xaa Xaa Xaa Xaa Xaa Xaa Xaa Tyr Gly Met Asp Val (SEQ ID NO: 50098)

Ser Tyr Gly Met His (SEQ ID NO: 50573)

Ile Ile Ser Tyr Ala Gly Ser Asn Lys Tyr Tyr Ala Asp Ser Val Lys Gly (SEQ ID NO: 50574)

Arg Gly Tyr Ser Tyr Gly Gly Tyr Gly Met Asp Val (SEQ ID NO: 50575)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | H_FR1 | | | | | | | | | | | | | | | | | | | | | | | | |
| CONSENSUS | Q | V | Q | L | V | E | S | - | G | G | G | V | V | Q | P | G | R | S | L | R | L | S | C | A | A |
| 21-225_11A2_HC | . | . | . | . | . | . | . | . | . | . | . | . | . | . | . | . | . | . | . | . | . | . | . | . | . |
| 21-225_13A1_HC | . | . | . | . | . | . | . | . | . | . | . | . | . | . | . | . | . | . | . | . | . | . | . | . | . |
| 21-225_15C2_HC | . | . | . | . | . | . | . | . | . | . | . | . | . | . | . | . | . | . | . | . | . | . | . | . | . |
| 21-225_15F10_HC | . | . | . | . | . | . | . | . | . | . | . | . | . | . | . | . | . | . | . | . | . | . | . | . | . |
| 21-225_16B3_HC | . | . | . | . | . | . | . | . | . | . | . | . | . | . | . | . | . | . | . | . | . | . | . | V | . |
| 21-225_1B12_HC | . | . | . | . | . | . | . | . | . | . | . | . | . | . | . | . | . | . | . | . | . | . | . | . | . |
| 21-225_225B11_HC | . | . | . | . | . | . | . | . | . | . | . | . | . | . | . | . | . | . | . | . | . | . | . | . | . |
| 21-225_24D6_HC | . | . | . | . | . | . | . | . | . | . | . | . | . | . | . | . | . | . | . | . | . | . | . | . | . |
| 21-225_45F6_HC | . | . | . | . | . | . | . | . | . | . | . | . | . | . | . | . | . | . | . | . | . | . | . | . | . |
| 21-225_5E6_HC | . | . | . | . | . | . | . | . | . | . | . | . | . | . | . | . | . | . | . | . | . | . | . | . | . |

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | H_CDR1 | | | | | | | | | | H_FR2 | | | | | | | |
| CONSENSUS | S | G | - | F | T | F | S | S | - | - | - | - | - | Y | G | M | H | W | V | R | Q | A | P | G | K |
| 21-225_11A2_HC | . | . | . | . | . | . | . | . | . | . | . | . | . | . | . | . | . | . | . | . | . | . | . | . | . |
| 21-225_13A1_HC | . | . | . | . | . | . | . | . | . | . | . | . | . | . | . | . | . | . | . | . | . | . | . | . | . |
| 21-225_15C2_HC | . | . | . | . | . | . | . | . | . | . | . | . | . | . | . | . | . | . | . | . | . | . | . | . | . |
| 21-225_15F10_HC | . | . | . | . | . | . | . | . | . | . | . | . | . | . | . | . | . | . | . | . | . | . | . | . | . |
| 21-225_16B3_HC | . | . | . | . | . | . | . | . | . | . | . | . | . | . | . | . | . | . | . | . | . | . | . | . | . |
| 21-225_1B12_HC | . | . | . | . | . | . | . | . | . | . | . | . | . | . | . | . | . | . | . | . | . | . | . | . | . |
| 21-225_225B11_HC | . | . | . | . | . | . | . | . | . | . | . | . | . | . | . | . | . | . | . | . | . | . | . | . | . |
| 21-225_24D6_HC | . | . | . | . | . | . | . | . | . | . | . | . | . | . | . | . | . | . | . | . | . | V | . | . | . |
| 21-225_45F6_HC | . | . | . | . | . | . | . | Y | . | . | . | . | . | . | . | L | . | . | . | . | . | . | . | . | . |
| 21-225_5E6_HC | . | . | . | . | . | . | . | . | . | . | . | . | . | . | . | . | . | . | . | . | . | . | . | . | . |

FIGURE 57

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | H_CDR2 | | | | | | | | | | | | | | | | | | |
| CONSENSUS | G | L | E | W | V | A | | I | S | Y | A | - | - | - | G | S | N | K | Y | Y | A | D | S | V | K |
| 21-225_11A2_HC | . | . | . | . | . | . | V | . | . | . | G | . | . | . | . | . | . | . | D | S | . | . | . | . | . |
| 21-225_13A1_HC | . | . | . | . | . | . | V | . | . | . | . | . | . | . | . | T | . | Q | . | . | . | . | . | . | . |
| 21-225_15C2_HC | . | . | . | . | . | S | I | . | . | . | G | . | . | . | . | R | . | N | H | . | . | . | . | . | . |
| 21-225_15F10_HC | . | . | . | . | . | S | V | . | . | . | G | . | . | . | . | . | . | N | . | . | . | . | . | . | . |
| 21-225_16B3_HC | . | . | . | . | . | . | V | . | . | . | . | . | . | . | . | I | . | . | S | . | . | . | . | . | . |
| 21-225_1B12_HC | . | . | . | . | . | . | I | . | . | . | . | . | . | . | . | . | . | . | S | . | . | . | . | . | . |
| 21-225_225B11_HC | . | . | . | . | . | T | I | . | . | . | S | . | . | . | . | . | . | . | . | . | . | . | . | . | . |
| 21-225_24D6_HC | . | . | . | . | . | . | I | . | . | . | . | . | . | . | . | . | . | . | S | . | . | . | . | . | . |
| 21-225_45F6_HC | . | . | . | . | . | . | V | . | . | . | D | . | . | . | . | . | . | . | . | . | . | . | . | . | . |
| 21-225_5E6_HC | . | . | . | . | . | . | I | . | . | . | V | . | . | . | . | N | . | . | . | . | . | . | . | . | . |

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | H_FR3 | | | | | | | | | | | | | | | | | | | | | | | |
| CONSENSUS | G | R | F | T | I | S | R | D | N | S | K | N | T | L | Y | L | Q | M | N | S | L | R | A | E | D |
| 21-225_11A2_HC | . | . | . | I | . | . | . | . | . | . | . | . | . | . | . | . | . | . | . | . | . | . | . | . | . |
| 21-225_13A1_HC | . | . | . | . | . | . | . | . | . | . | . | . | . | . | . | . | . | . | . | . | . | . | . | . | . |
| 21-225_15C2_HC | . | . | . | . | . | . | . | . | . | . | . | . | . | . | . | . | . | . | . | . | . | . | . | . | . |
| 21-225_15F10_HC | . | . | . | . | . | . | . | . | . | . | . | . | . | . | . | . | . | . | . | . | . | . | . | . | . |
| 21-225_16B3_HC | . | . | . | . | . | . | . | . | . | . | N | . | . | . | . | . | . | . | . | . | . | . | . | . | . |
| 21-225_1B12_HC | . | . | . | . | . | . | . | . | . | . | . | . | M | . | . | . | . | . | . | . | . | . | . | . | . |
| 21-225_225B11_HC | . | . | . | . | . | . | . | . | . | . | . | . | . | . | . | . | . | . | . | . | . | . | . | . | . |
| 21-225_24D6_HC | . | . | . | . | . | . | . | . | . | . | . | . | . | . | . | . | . | . | . | . | . | . | . | . | . |
| 21-225_45F6_HC | . | . | . | . | . | . | . | . | . | . | . | . | . | . | . | . | . | . | . | N | . | . | . | . | . |
| 21-225_5E6_HC | . | . | . | . | . | . | . | . | . | . | . | . | . | . | . | . | . | . | . | . | . | . | V | . | . |

FIGURE 57

| Reference # | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 |
|---|---|---|---|---|---|---|---|---|---|---|---|---|---|---|---|---|---|---|---|---|---|---|---|---|---|
| | | | | | | | | | | | | | | | | | | | | | | | H_CDR3 | | |
| CONSENSUS | T | A | V | Y | Y | C | V | R | R | G | Y | S | Y | G | - | - | - | - | - | - | - | - | - | - | - |
| 21-225_11A2_HC | . | . | . | . | . | . | . | . | . | . | . | . | . | . | . | . | . | . | . | . | . | . | . | . | . |
| 21-225_13A1_HC | . | . | . | . | . | . | . | . | . | . | . | . | . | . | . | . | . | . | . | . | . | . | . | . | . |
| 21-225_15C2_HC | . | . | . | . | . | . | . | . | . | . | . | . | . | . | . | . | . | . | . | . | . | . | . | . | . |
| 21-225_15F10_HC | . | . | . | . | . | . | . | . | . | . | . | . | . | . | . | . | . | . | . | . | . | . | . | . | . |
| 21-225_16B3_HC | . | . | . | . | . | . | . | . | . | . | . | . | . | . | . | . | . | . | . | . | . | . | . | . | . |
| 21-225_1B12_HC | . | . | . | . | . | . | . | . | . | . | . | . | . | . | . | . | . | . | . | . | . | . | . | . | . |
| 21-225_225B11_HC | . | . | . | . | . | . | A | . | . | . | . | . | . | . | . | . | . | . | . | . | . | . | . | . | . |
| 21-225_24D6_HC | . | . | . | . | . | . | . | . | . | . | . | . | . | . | . | . | . | . | . | . | . | . | . | . | . |
| 21-225_45F6_HC | . | . | . | F | . | . | A | . | E | D | R | Y | C | S | G | T | S | C | . | . | . | . | . | . | . |
| 21-225_5E6_HC | . | . | . | . | . | . | A | . | . | . | . | . | . | . | . | . | . | . | . | . | . | . | . | . | . |

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|---|---|---|---|---|
| | | | | | | | | | | | | | | | | | | | H_FR4 | | | | | |
| CONSENSUS | - | - | - | - | - | - | - | G | Y | G | M | D | V | W | G | Q | G | T | T | V | T | V | S | S |
| 21-225_11A2_HC | . | . | . | . | . | . | . | . | . | . | . | . | . | . | . | . | . | . | . | . | . | . | . | . |
| 21-225_13A1_HC | . | . | . | . | . | . | . | . | . | . | . | . | . | . | . | . | . | . | . | . | . | . | . | . |
| 21-225_15C2_HC | . | . | . | . | . | . | . | . | . | . | . | . | . | . | . | . | . | . | . | . | . | . | . | . |
| 21-225_15F10_HC | . | . | . | . | . | . | . | . | . | . | . | . | . | . | . | . | . | . | . | . | . | . | . | . |
| 21-225_16B3_HC | . | . | . | . | . | . | . | . | . | . | . | . | . | . | . | . | . | A | . | . | . | . | . | . |
| 21-225_1B12_HC | . | . | . | . | . | . | . | . | . | . | . | . | . | . | . | . | . | A | . | . | . | . | . | . |
| 21-225_225B11_HC | . | . | . | . | . | . | . | . | . | . | . | . | . | . | . | . | . | . | . | . | . | . | . | . |
| 21-225_24D6_HC | . | . | . | . | . | . | . | . | . | . | . | . | . | . | . | . | . | A | . | . | . | . | . | . |
| 21-225_45F6_HC | . | . | . | P | Y | Y | Y | Y | . | . | . | . | . | . | . | . | . | . | . | . | . | . | . | . |
| 21-225_5E6_HC | . | . | . | . | . | . | . | . | . | . | . | . | . | . | . | . | . | . | . | . | . | . | . | . |

**FIGURE 57**

Table 85. Consensus 51 -VH3|3-33/D3|3-10|RF2/JH6 (SEQ ID NO: 50302):

QVQLVES-GGGVVQPGRSLRLSCAASG-FTFSD-----YVMHWVRQAPGKGLEWVAVIWYD---
GSNKYYADSVKGRFTISRDNSKNTLYLQMNSLRAEDTAVYYCAREPYTSGW----------------YDYGMDVWGQGTTVTVSS wherein:

D at position 33 can be substituted with S

Y at position 39 can be substituted with C

V at position 40 can be substituted with G

H at position 42 can be substituted with Q

V at position 57 can be substituted with I

G at position 76 can be substituted with V

P at position 110 can be substituted with R

T at position 112 can be substituted with N

Y at position 132 can be substituted with H

M at position 136 can be substituted with L

Xaa Xaa Xaa Met Xaa (SEQ ID NO: 50099)

Xaa Ile Trp Tyr Asp Gly Ser Asn Lys Tyr Tyr Ala Asp Ser Val Lys Xaa (SEQ ID NO: 50100)

Glu Xaa Tyr Xaa Ser Gly Trp Xaa Asp Tyr Gly Xaa Asp Val (SEQ ID NO: 50101)

Asp Tyr Val Met His (SEQ ID NO: 50576)

Val Ile Trp Tyr Asp Gly Ser Asn Lys Tyr Tyr Ala Asp Ser Val Lys Gly (SEQ ID NO: 50577)

Glu Pro Tyr Thr Ser Gly Trp Tyr Asp Tyr Gly Met Asp Val (SEQ ID NO: 50578)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| | H_FR1 | | | | | | | | | | | | | | | | | | | | | | | | |
| CONSENSUS | Q | V | Q | L | V | E | S | - | G | G | G | V | V | Q | P | G | R | S | L | R | L | S | C | A | A |
| 21-225_158D4_HC | . | . | . | . | . | . | . | . | . | . | . | . | . | . | . | . | . | . | . | . | . | . | . | . | . |
| 21-225_159A3_HC | . | . | . | . | . | . | . | . | . | . | . | . | . | . | . | . | . | . | . | . | . | . | . | . | . |
| 21-225_159C8_HC | . | . | . | . | . | . | . | . | . | . | . | . | . | . | . | . | . | . | . | . | . | . | . | . | . |
| 21-225_160C4_HC | . | . | . | . | . | . | . | . | . | . | . | . | . | . | . | . | . | . | . | . | . | . | . | . | . |
| 21-225_161G12_HC | . | . | . | . | . | . | . | . | . | . | . | . | . | . | . | . | . | . | . | . | . | . | . | . | . |
| 21-225_20B11_HC | . | . | . | . | . | . | . | . | . | . | . | . | . | . | . | . | . | . | . | . | . | . | . | . | . |
| 21-225_226F7_HC | . | . | . | . | . | . | . | . | . | . | . | . | . | . | . | . | . | . | . | . | . | . | . | . | . |
| 21-225_2B10_LC1_HC | . | . | . | . | . | . | . | . | . | . | . | . | . | . | . | . | . | . | . | . | . | . | . | . | . |
| 21-225_2B10_LC2_HC | . | . | . | . | . | . | . | . | . | . | . | . | . | . | . | . | . | . | . | . | . | . | . | . | . |
| 21-225_7F8_HC | . | . | . | . | . | . | . | . | . | . | . | . | . | . | . | . | . | . | . | . | . | . | . | . | . |

| Reference # | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 |
|---|---|---|---|---|---|---|---|---|---|---|---|---|---|---|---|---|---|---|---|---|---|---|---|---|---|
| | | | | | | | | H_CDR1 | | | | | | | | | | H_FR2 | | | | | | | |
| CONSENSUS | S | G | - | F | T | F | S | | - | - | - | - | - | Y | V | M | H | W | V | R | Q | A | P | G | K |
| 21-225_158D4_HC | . | . | . | . | . | . | . | D | . | . | . | . | . | . | . | . | . | . | . | . | . | . | . | . | . |
| 21-225_159A3_HC | . | . | . | . | . | . | . | D | . | . | . | . | . | . | . | . | Q | . | . | . | . | . | . | . | . |
| 21-225_159C8_HC | . | . | . | . | . | . | . | D | . | . | . | . | . | . | . | . | Q | . | . | . | . | . | . | . | . |
| 21-225_160C4_HC | . | . | . | . | . | . | . | D | . | . | . | . | . | . | . | . | Q | . | . | . | . | . | . | . | . |
| 21-225_161G12_HC | . | . | . | . | . | . | . | D | . | . | . | . | . | . | . | . | Q | . | . | . | . | . | . | . | . |
| 21-225_20B11_HC | . | . | . | . | . | . | . | S | . | . | . | . | . | . | . | . | . | . | . | . | . | . | . | . | . |
| 21-225_226F7_HC | . | . | . | . | . | . | . | S | . | . | . | . | . | . | G | . | . | . | . | . | . | . | . | . | . |
| 21-225_2B10_LC1_HC | . | . | . | . | . | . | . | S | . | . | . | . | . | . | . | . | . | . | . | . | . | . | . | . | . |
| 21-225_2B10_LC2_HC | . | . | . | . | . | . | . | S | . | . | . | . | . | . | . | . | . | . | . | . | . | . | . | . | . |
| 21-225_7F8_HC | . | . | . | . | . | . | . | S | . | . | . | . | . | C | . | . | . | . | . | . | . | . | . | . | . |

**FIGURE 57**

| Reference # | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 |
|---|---|---|---|---|---|---|---|---|---|---|---|---|---|---|---|---|---|---|---|---|---|---|---|---|---|
| | | | | | | | H_CDR2 | | | | | | | | | | | | | | | | | | |
| CONSENSUS | G | L | E | W | V | A | V | I | W | Y | D | - | - | - | G | S | N | K | Y | Y | A | D | S | V | K |
| 21-225_158D4_HC | . | . | . | . | . | S | . | . | . | . | . | . | . | . | . | . | . | . | . | . | . | . | . | . | . |
| 21-225_159A3_HC | . | . | . | . | . | . | . | . | . | . | . | . | . | . | . | . | . | . | . | . | . | . | . | . | . |
| 21-225_159C8_HC | . | . | . | . | . | . | . | . | . | . | . | . | . | . | . | . | . | . | . | . | . | . | . | . | . |
| 21-225_160C4_HC | . | . | . | . | . | . | . | . | . | . | . | . | . | . | . | . | . | . | . | . | . | . | . | . | . |
| 21-225_161G12_HC | . | . | . | . | . | . | . | . | . | . | . | . | . | . | . | . | . | . | . | . | . | . | . | . | . |
| 21-225_20B11_HC | . | . | . | . | . | . | . | . | . | . | . | . | . | . | . | . | . | . | . | . | . | . | . | . | . |
| 21-225_226F7_HC | . | . | . | . | . | . | I | . | . | . | . | . | . | . | . | . | . | . | . | . | . | . | . | . | . |
| 21-225_2B10_LC1_HC | . | . | . | . | . | . | . | . | . | . | . | . | . | . | . | . | . | . | . | . | . | . | . | . | . |
| 21-225_2B10_LC2_HC | . | . | . | . | . | . | . | . | . | . | . | . | . | . | . | . | . | . | . | . | . | . | . | . | . |
| 21-225_7F8_HC | . | . | . | . | . | . | . | . | . | . | . | . | . | . | . | . | . | . | . | . | . | . | . | . | . |

| Reference # | 76 | 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 |
|---|---|---|---|---|---|---|---|---|---|---|---|---|---|---|---|---|---|---|---|---|---|---|---|---|---|
| | | H_FR3 | | | | | | | | | | | | | | | | | | | | | | | |
| CONSENSUS | G | R | F | T | I | S | R | D | N | S | K | N | T | L | Y | L | Q | M | N | S | L | R | A | E | D |
| 21-225_158D4_HC | . | . | . | . | . | . | . | . | . | . | . | . | . | . | . | . | . | . | . | . | . | . | . | . | . |
| 21-225_159A3_HC | . | . | . | . | . | . | . | . | I | . | . | . | . | . | . | . | . | . | . | . | . | . | . | . | . |
| 21-225_159C8_HC | . | . | . | . | . | . | . | . | . | . | . | . | . | . | . | . | . | . | . | . | . | . | . | . | . |
| 21-225_160C4_HC | . | . | . | . | . | . | . | . | . | . | . | . | . | . | . | . | . | . | . | . | . | . | . | . | . |
| 21-225_161G12_HC | . | . | . | . | . | . | . | . | . | . | . | . | . | . | . | . | . | . | . | . | . | . | . | . | . |
| 21-225_20B11_HC | V | . | . | . | V | . | . | . | . | . | . | . | . | . | S | . | . | . | . | . | . | . | . | . | . |
| 21-225_226F7_HC | . | . | . | . | . | . | . | . | . | . | . | . | . | . | . | . | . | . | . | . | . | . | . | . | . |
| 21-225_2B10_LC1_HC | V | . | . | . | V | . | . | . | . | . | . | . | . | . | S | . | . | . | . | . | . | . | . | . | . |
| 21-225_2B10_LC2_HC | V | . | . | . | V | . | . | . | . | . | . | . | . | . | S | . | . | . | . | . | . | . | . | . | . |
| 21-225_7F8_HC | . | . | . | . | . | . | . | . | . | . | N | . | . | . | . | . | . | . | . | . | . | . | . | . | . |

# FIGURE 57

| Reference # | 126 | 127 | 128 | 129 | 130 | 131 | 132 | 133 | 134 | 135 | 136 | 137 | 138 | 139 | 140 | 141 | 142 | 143 | 144 | 145 | 146 | 147 | 148 | 149 | Refe |
|---|---|---|---|---|---|---|---|---|---|---|---|---|---|---|---|---|---|---|---|---|---|---|---|---|---|
| | | | | | | | | | | | | | | H_FR4 | | | | | | | | | | | |
| CONSENSUS | - | - | - | - | - | - | Y | D | Y | G | M | D | V | W | G | Q | G | T | T | V | T | V | S | S | |
| 21-225_158D4_HC | . | . | . | . | . | . | . | . | . | . | . | . | . | . | . | . | . | . | . | . | . | . | . | . | |
| 21-225_159A3_HC | . | . | . | . | . | . | . | . | . | . | . | . | . | . | . | . | . | . | . | . | . | . | . | . | |
| 21-225_159C8_HC | . | . | . | . | . | . | . | . | . | . | . | . | . | . | . | . | . | . | . | . | . | . | . | . | |
| 21-225_160C4_HC | . | . | . | . | . | . | . | . | . | . | . | . | . | . | . | . | . | . | . | . | . | . | . | . | |
| 21-225_161G12_HC | . | . | . | . | . | . | . | . | . | . | . | . | . | . | . | . | . | . | . | . | . | . | . | . | |
| 21-225_20B11_HC | . | . | . | . | . | . | . | . | . | . | . | . | . | . | . | . | . | . | . | . | . | . | . | . | |
| 21-225_226F7_HC | . | . | . | . | . | . | . | . | . | . | L | . | . | . | . | . | . | . | . | . | . | . | . | . | |
| 21-225_2B10_LC1_HC | . | . | . | . | . | . | . | . | . | . | . | . | . | . | . | . | . | . | . | . | . | . | . | . | |
| 21-225_2B10_LC2_HC | . | . | . | . | . | . | . | . | . | . | . | . | . | . | . | . | . | . | . | . | . | . | . | . | |
| 21-225_7F8_HC | . | . | . | . | . | . | H | . | . | . | . | . | . | . | . | . | . | . | . | . | . | . | . | . | |

| Reference # | rence # |
|---|---|
| CONSENSUS | |
| 21-225_158D4_HC | |
| 21-225_159A3_HC | |
| 21-225_159C8_HC | |
| 21-225_160C4_HC | |
| 21-225_161G12_HC | |
| 21-225_20B11_HC | |
| 21-225_226F7_HC | |
| 21-225_2B10_LC1_HC | |
| 21-225_2B10_LC2_HC | |
| 21-225_7F8_HC | |

**FIGURE 57**

Table 86. Consensus 52 -VH1|1-18/D3|3-3|RF2/JH6 (SEQ ID NO: 50303):

QVQLVQS-GAEVKKPGASVKVSCKASG-YTFNS-----YGISWVRQAPGQGLEWMGWISAY---NGNTKYAQKLQGRVTMTTDTSTSTAYMELRSLRSDDTAVYYCARHDFWSGY------------------YKGMDVWGQGTTVTVSS wherein:

I in position 41 can be substituted with F or V

T in position 68 can be substituted with R

Y in position 70 can be substituted with N, E or F

K in position 73 can be substituted with R

L in position 74 can be substituted with F

Ser Tyr Gly Xaa Ser (SEQ ID NO: 50102)

Trp Ile Ser Ala Tyr Asn Gly Asn Xaa Lys Xaa Ala Gln Xaa Xaa Gln Gly (SEQ ID NO: 50103)

His Asp Phe Trp Ser Gly Tyr Tyr Lys Gly Met Asp Val (SEQ ID NO: 50227)

Ser Tyr Gly Ile Ser (SEQ ID NO: 50579)

Trp Ile Ser Ala Tyr Asn Gly Asn Thr Lys Tyr Ala Gln Lys Leu Gln Gly (SEQ ID NO: 50580)

His Asp Phe Trp Ser Gly Tyr Tyr Lys Gly Met Asp Val (SEQ ID NO: 50581)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | Q | S | - | G | A | E | V | K | K | P | G | A | S | V | K | V | S | C | K | A | S |
| 21-225_49C5_HC | . | . | . | . | . | . | . | . | . | . | . | . | . | . | . | . | . | . | . | . | . | . | . | . | . | . |
| 21-225_49D10_HC | . | . | . | . | . | . | . | . | . | . | . | . | . | . | . | . | . | . | . | . | . | . | . | . | . | . |
| 21-225_51E3_HC | . | . | . | . | . | . | . | . | . | . | . | . | . | . | . | . | . | . | . | . | . | . | . | . | . | . |
| 21-225_51F4_HC | . | . | . | . | . | . | . | . | . | . | . | . | . | . | . | . | . | . | . | . | . | . | . | . | . | . |
| 21-225_52F1_HC | . | . | . | . | . | . | . | . | . | . | . | . | . | . | . | . | . | . | . | . | . | . | . | . | . | . |
| 21-225_53E8_HC | . | . | . | . | . | . | . | . | . | . | . | . | . | . | . | . | . | . | . | . | . | . | . | . | . | . |
| 21-225_54E9_HC | . | . | . | . | . | . | . | . | . | . | . | . | . | . | . | . | . | . | . | . | . | . | . | . | . | . |
| 21-225_56E3_HC | . | . | . | . | . | . | . | . | . | . | . | . | . | . | . | . | . | . | . | . | . | . | . | . | . | . |
| 21-225_56G1_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | Y | T | F | N | S | - | - | - | - | - | Y | G | I | S | W | V | R | Q | A | P | G | Q | G | L |
| 21-225_49C5_HC | . | . | . | . | . | . | . | . | . | . | . | . | . | . | . | . | . | . | . | . | . | . | . | . | . | . |
| 21-225_49D10_HC | . | . | . | . | . | . | . | . | . | . | . | . | . | . | . | . | . | . | . | L | . | . | . | . | . | P |
| 21-225_51E3_HC | . | . | . | . | . | . | . | . | . | . | . | . | . | . | . | . | . | . | . | L | . | . | . | . | . | . |
| 21-225_51F4_HC | . | . | . | . | . | . | . | . | . | . | . | . | . | . | . | . | . | . | . | . | . | . | . | . | . | . |
| 21-225_52F1_HC | . | . | . | . | . | . | . | . | . | . | . | . | . | . | V | . | . | . | . | . | . | . | . | . | . | . |
| 21-225_53E8_HC | . | . | . | . | . | . | . | . | . | . | . | . | . | . | . | . | . | . | . | . | . | . | . | . | . | . |
| 21-225_54E9_HC | . | . | . | . | . | . | . | . | . | . | . | . | . | . | . | . | . | . | . | L | . | . | . | . | . | . |
| 21-225_56E3_HC | . | . | . | . | . | . | . | . | . | . | . | . | . | . | P | . | . | . | . | . | . | . | . | . | . | . |
| 21-225_56G1_HC | . | . | . | . | . | . | . | . | . | . | . | . | . | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | M | G | W | I | S | A | Y | - | - | - | N | G | N | T | K | Y | A | Q | K | L | Q | G | R | V |
| 21-225_49C5_HC | . | . | . | . | . | . | . | . | . | . | . | . | . | . | . | . | . | . | . | . | . | . | . | . | . | . |
| 21-225_49D10_HC | . | . | . | . | . | . | . | . | . | . | . | . | . | . | . | . | . | N | . | . | . | . | . | . | . | . |
| 21-225_51E3_HC | . | . | . | . | . | . | . | . | . | . | . | . | . | . | . | . | . | N | . | . | R | F | . | . | . | . |
| 21-225_51F4_HC | . | . | . | . | . | . | . | . | . | . | . | . | . | . | . | . | . | . | . | . | . | . | . | . | . | . |
| 21-225_52F1_HC | . | . | . | . | . | . | . | . | . | . | . | . | . | . | . | R | . | . | . | . | . | . | . | . | . | . |
| 21-225_53E8_HC | . | . | . | . | . | . | . | . | . | . | . | . | . | . | . | . | . | F | . | . | . | . | . | . | . | . |
| 21-225_54E9_HC | . | . | . | . | . | . | . | . | . | . | . | . | . | . | . | . | . | N | . | . | . | . | . | . | . | . |
| 21-225_56E3_HC | . | . | . | . | . | . | . | . | . | . | . | . | . | . | . | . | . | E | . | . | . | F | . | . | . | . |
| 21-225_56G1_HC | . | . | . | . | . | . | . | . | . | . | . | . | . | . | . | . | . | . | . | . | . | F | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | M | T | T | D | T | S | T | S | T | A | Y | M | E | L | R | S | L | R | S | D | D | T | A | V | Y |
| 21-225_49C5_HC | . | . | . | . | . | . | . | . | . | . | . | . | . | . | . | . | . | . | . | . | . | . | . | . | . | . |
| 21-225_49D10_HC | . | . | . | . | . | . | . | . | . | . | . | . | . | . | . | . | . | . | . | . | . | . | . | . | . | . |
| 21-225_51E3_HC | . | . | . | . | . | . | . | . | . | . | . | . | . | . | . | . | . | . | . | . | . | . | . | . | . | . |
| 21-225_51F4_HC | . | . | . | . | . | . | . | . | . | . | . | . | . | . | . | . | . | . | . | . | . | . | . | . | . | . |
| 21-225_52F1_HC | S | . | . | . | . | . | . | . | . | . | . | . | . | . | . | . | . | . | . | . | . | . | . | . | . | . |
| 21-225_53E8_HC | . | . | . | . | . | . | . | . | . | . | . | . | . | . | . | . | . | . | . | . | . | . | . | . | . | . |
| 21-225_54E9_HC | . | . | . | . | . | . | . | . | . | . | . | . | . | . | . | . | . | . | . | . | . | . | . | . | . | . |
| 21-225_56E3_HC | . | . | . | . | . | . | . | . | . | . | . | . | . | . | . | . | . | . | . | A | . | . | . | . | . | . |
| 21-225_56G1_HC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | H | D | F | W | S | G | Y | - | - | - | - | - | - | - | - | - | - | - | - | - | - | - |
| 21-225_49C5_HC | . | . | . | . | . | . | . | . | . | . | . | . | . | . | . | . | . | . | . | . | . | . | . | . | . | . |
| 21-225_49D10_HC | . | . | . | . | . | . | . | . | . | . | . | . | . | . | . | . | . | . | . | . | . | . | . | . | . | . |
| 21-225_51E3_HC | . | . | . | . | . | . | . | . | . | . | . | . | . | . | . | . | . | . | . | . | . | . | . | . | . | . |
| 21-225_51F4_HC | . | . | . | . | . | . | . | . | . | . | . | . | . | . | . | . | . | . | . | . | . | . | . | . | . | . |
| 21-225_52F1_HC | . | . | . | . | . | . | . | . | . | . | . | . | . | . | . | . | . | . | . | . | . | . | . | . | . | . |
| 21-225_53E8_HC | . | . | . | . | . | . | . | . | . | . | . | . | . | . | . | . | . | . | . | . | . | . | . | . | . | . |
| 21-225_54E9_HC | . | . | . | . | . | . | . | . | . | . | . | . | . | . | . | . | . | . | . | . | . | . | . | . | . | . |
| 21-225_56E3_HC | . | . | . | . | . | . | . | . | . | . | . | . | . | . | . | . | . | . | . | . | . | . | . | . | . | . |
| 21-225_56G1_HC | . | . | . | . | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | Y | K | G | M | D | V | W | G | Q | G | T | T | V | T | V | S | S |
| 21-225_49C5_HC | . | . | . | . | . | . | . | . | . | . | . | . | . | . | . | . | . | . | . |
| 21-225_49D10_HC | . | . | . | . | . | . | . | . | . | . | . | . | . | . | . | . | . | . | . |
| 21-225_51E3_HC | . | . | . | . | . | . | . | . | . | . | . | . | . | . | . | . | . | . | . |
| 21-225_51F4_HC | . | . | . | . | . | . | . | . | . | . | . | . | . | . | . | . | . | . | . |
| 21-225_52F1_HC | . | . | . | . | . | . | . | . | . | . | . | . | . | . | . | . | . | . | . |
| 21-225_53E8_HC | . | . | . | . | . | . | . | . | . | . | . | . | . | . | . | . | . | . | . |
| 21-225_54E9_HC | . | . | . | . | . | . | . | . | . | . | . | . | . | . | . | . | . | . | . |
| 21-225_56E3_HC | . | . | . | . | . | . | . | . | . | . | . | . | . | . | . | . | . | . | . |
| 21-225_56G1_HC | . | . | . | . | . | . | . | . | . | . | . | . | . | . | . | . | . | . | . |

**FIGURE 57**

Table 87. Consensus 53 - VH3|3-23/D6|6-6|RF1/JH4 (SEQ ID NO: 50304):

EVQLLES-GGGLVQPGGSLRLSCAASG-FTFS<u>S-----YAMS</u>WVRQAPGKGLEWVS<u>AISGR---GG</u>
<u>NTFDADSVKG</u>RFTISRDNSKNTLYLQMNSLRAEDTAVYYCAK<u>ERSGS---------------------YFDY</u>WGQGTLVTVSS wherein:

Y at position 39 can be substituted with S

A at position 57 can be substituted with V or S

R at position 61 can be substituted with S

G at position 66 can be substituted with I or V

N at position 67 can be substituted with S

D at position 70 can be substituted with Y

A at position 71 can be substituted with T

E at position 109 can be substituted with S

R at position 110 can be substituted with N

G at position 112 can be substituted with S

S at position 113 can be substituted with G

Y at position 135 can be substituted with W

Xaa Xaa Ala Met Ser (SEQ ID NO: 50104)

Xaa Ile Ser Gly Xaa Gly Xaa Xaa Thr Phe Xaa Xaa Asp Ser Val Lys Gly (SEQ ID NO: 50105)

Xaa Xaa Ser Xaa Xaa Xaa Phe Asp Tyr (SEQ ID NO: 50106)

Ser Tyr Ala Met Ser (SEQ ID NO: 50582)

Ala Ile Ser Gly Arg Gly Gly Asn Thr Phe Asp Ala Asp Ser Val Lys Gly (SEQ ID NO: 50583)

**FIGURE 57**

Glu Arg Ser Gly Ser Tyr Phe Asp Tyr (SEQ ID NO: 50584)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | E | V | Q | L | L | E | S | - | G | G | G | L | V | Q | P | G | G | S | L | R | L | S | C | A | A | S |
| 21-225_171A8_HC | . | . | . | . | . | . | . | . | . | . | . | . | . | . | . | . | . | . | . | . | . | . | . | . | . | . |
| 21-225_174G7_HC | . | . | . | . | . | . | . | . | . | . | . | . | . | . | . | . | . | . | . | . | . | . | . | . | . | . |
| 21-225_175C10_HC | . | . | . | . | . | . | . | . | . | . | . | . | . | . | . | . | . | . | . | . | . | . | . | . | . | . |
| 21-225_43C2_HC | . | . | . | . | . | . | . | . | . | . | . | . | . | . | . | . | . | . | . | . | . | . | . | . | . | . |
| 21-225_43E8_HC | . | . | . | . | . | . | . | . | . | . | . | . | . | . | . | . | . | . | . | . | . | . | . | . | . | . |
| 21-225_43H4_HC | . | . | . | . | . | . | . | . | . | . | . | . | . | . | . | . | . | . | . | . | . | . | . | . | . | . |
| 21-225_44D10_HC | . | . | . | . | . | . | . | . | . | . | . | . | . | . | . | . | . | . | . | . | . | . | . | . | . | . |
| 21-225_45F8_HC | . | . | . | . | . | . | . | . | . | . | . | . | . | . | . | . | . | . | . | . | . | . | . | . | . | . |
| 21-225_46A6_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | A | M | S | W | V | R | Q | A | P | G | K | G | L |
| 21-225_171A8_HC | . | . | . | . | . | R | . | . | . | . | . | . | S | . | . | . | . | . | . | . | . | . | . | . | . | . |
| 21-225_174G7_HC | . | . | . | . | . | R | . | . | . | . | . | . | S | . | . | . | . | . | . | . | . | . | . | . | . | . |
| 21-225_175C10_HC | . | . | . | . | . | . | . | . | . | . | . | . | . | . | . | . | . | . | . | . | . | . | . | . | . | . |
| 21-225_43C2_HC | . | . | . | . | . | . | . | . | . | . | . | . | . | . | . | . | . | . | . | . | . | . | . | . | . | . |
| 21-225_43E8_HC | . | . | . | . | . | . | . | . | . | . | . | . | . | . | . | . | . | . | . | . | . | . | . | . | . | . |
| 21-225_43H4_HC | . | . | . | . | . | . | . | . | . | . | . | . | . | . | . | . | . | . | . | . | . | . | . | . | . | . |
| 21-225_44D10_HC | . | . | . | . | . | . | . | . | . | . | . | . | . | . | . | . | . | . | . | . | . | . | . | . | . | . |
| 21-225_45F8_HC | . | . | . | . | . | . | . | . | . | . | . | . | . | . | . | . | . | . | . | . | . | . | . | . | . | . |
| 21-225_46A6_HC | . | . | . | . | . | . | . | . | . | . | . | . | . | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | S | A | I | S | G | R | - | - | - | G | | N | T | F | D | A | D | S | V | K | G | R | F |
| 21-225_171A8_HC | . | . | . | . | V | . | . | . | S | . | . | . | . | G | S | . | . | Y | T | . | . | . | . | . | . | . |
| 21-225_174G7_HC | . | . | . | . | V | . | . | . | S | . | . | . | . | G | S | . | . | Y | T | . | . | . | . | . | . | . |
| 21-225_175C10_HC | . | . | . | . | S | . | . | . | . | . | . | . | . | G | S | . | . | Y | . | . | . | . | . | . | . | . |
| 21-225_43C2_HC | . | . | . | . | . | . | . | . | . | . | . | . | . | V | . | . | . | . | . | . | . | . | . | . | . | . |
| 21-225_43E8_HC | . | . | . | . | . | . | . | . | . | . | . | . | . | I | . | . | . | . | . | . | . | . | . | . | . | . |
| 21-225_43H4_HC | . | . | . | . | . | . | . | . | . | . | . | . | . | I | . | . | . | . | . | . | . | . | . | . | . | . |
| 21-225_44D10_HC | . | . | . | . | . | . | . | . | . | . | . | . | . | V | . | . | . | . | . | . | . | . | . | . | . | . |
| 21-225_45F8_HC | . | . | . | . | . | . | . | . | . | . | . | . | . | V | . | . | . | . | . | . | . | . | . | . | . | . |
| 21-225_46A6_HC | . | . | . | . | . | . | . | . | . | . | . | . | . | I | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_171A8_HC | . | . | . | . | . | . | . | . | . | . | . | . | . | . | . | . | . | . | . | . | . | . | . | . | . | . |
| 21-225_174G7_HC | . | . | . | . | . | . | . | . | . | . | . | . | . | . | . | . | . | . | . | . | . | . | . | . | . | . |
| 21-225_175C10_HC | . | V | . | . | . | . | . | . | . | . | . | . | . | . | . | . | . | . | . | . | . | . | . | . | . | . |
| 21-225_43C2_HC | . | . | . | . | . | . | . | . | . | . | . | . | . | . | . | . | . | . | . | . | . | . | . | . | . | . |
| 21-225_43E8_HC | . | . | . | . | . | . | . | . | . | . | . | . | . | . | . | . | . | . | . | . | . | . | . | . | . | . |
| 21-225_43H4_HC | . | . | . | . | . | . | . | . | . | . | . | . | . | . | . | . | . | . | . | . | . | . | . | . | . | . |
| 21-225_44D10_HC | . | . | . | . | . | . | . | . | . | . | . | . | . | . | . | . | . | . | . | . | . | . | . | . | . | . |
| 21-225_45F8_HC | . | . | . | . | . | . | . | . | . | . | . | . | . | . | . | . | . | . | . | . | . | . | . | . | . | . |
| 21-225_46A6_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | K | E | R | S | G | S | - | - | - | - | - | - | - | - | - | - | - | - | - | - | - | - | - |
| 21-225_171A8_HC | . | . | . | . | S | N | . | S | G | . | . | . | . | . | . | . | . | . | . | . | . | . | . | . | . | . |
| 21-225_174G7_HC | . | . | . | . | S | N | . | S | G | . | . | . | . | . | . | . | . | . | . | . | . | . | . | . | . | . |
| 21-225_175C10_HC | . | . | . | . | S | N | . | S | G | . | . | . | . | . | . | . | . | . | . | . | . | . | . | . | . | . |
| 21-225_43C2_HC | . | . | . | . | . | . | . | . | . | . | . | . | . | . | . | . | . | . | . | . | . | . | . | . | . | . |
| 21-225_43E8_HC | . | . | . | . | . | . | . | . | . | . | . | . | . | . | . | . | . | . | . | . | . | . | . | . | . | . |
| 21-225_43H4_HC | . | . | . | . | . | . | . | . | . | . | . | . | . | . | . | . | . | . | . | . | . | . | . | . | . | . |
| 21-225_44D10_HC | . | . | . | . | . | . | . | . | . | . | . | . | . | . | . | . | . | . | . | . | . | . | . | . | . | . |
| 21-225_45F8_HC | . | . | . | . | . | . | . | . | . | . | . | . | . | . | . | . | . | . | . | . | . | . | . | . | . | . |
| 21-225_46A6_HC | . | . | . | . | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | Y | F | D | Y | W | G | Q | G | T | L | V | T | V | S | S |
| 21-225_171A8_HC | . | . | . | . | W | . | . | . | . | . | . | . | . | . | . | . | . | . | . |
| 21-225_174G7_HC | . | . | . | . | W | . | . | . | . | . | . | . | . | . | . | . | . | . | . |
| 21-225_175C10_HC | . | . | . | . | W | . | . | . | . | . | . | . | . | . | . | . | . | . | . |
| 21-225_43C2_HC | . | . | . | . | . | . | . | . | . | . | . | . | . | . | . | . | . | . | . |
| 21-225_43E8_HC | . | . | . | . | . | . | . | . | . | . | . | . | . | . | . | . | . | . | . |
| 21-225_43H4_HC | . | . | . | . | . | . | . | . | . | . | . | . | . | . | . | . | . | . | . |
| 21-225_44D10_HC | . | . | . | . | . | . | . | . | . | . | . | . | . | . | . | . | . | . | . |
| 21-225_45F8_HC | . | . | . | . | . | . | . | . | . | . | . | . | . | . | . | . | . | . | . |
| 21-225_46A6_HC | . | . | . | . | . | . | . | . | . | . | . | . | . | . | . | . | . | . | . |

**FIGURE 57**

Table 88. Consensus 54 -VH3|3-33/D6|6-6|RF1/JH4 (SEQ ID NO: 50305):

QVQLVES-GGGVVQPGRSLRLSCAASG-FTFSN-----YGMHWVRQAPGKGLEWVAVIWHD---GSNKYYADSVKGRFTISRDNSKNTLYLQMNSLRAEDTAVYYCARENSSS-------------------YYFDYWGQGTLVTVSS wherein:

N in position 33 can be substituted with S, Y or H

G in position 40 can be substituted with V

V in position 57 can be substituted with L

H in position 60 can be substituted with Y

S in position 66 can be substituted with T

N in position 67 can be substituted with D

K in position 68 can be substituted with A

A in position 71 can be substituted with V or G

Y in position 134 can be substituted with F

Xaa Tyr Xaa Met His (SEQ ID NO: 50107)

Xaa Ile Trp Xaa Asp Gly Xaa Xaa Xaa Tyr Tyr Xaa Asp Ser Val Lys Gly (SEQ ID NO: 50108)

Glu Asn Ser Ser Ser Xaa Tyr Phe Asp Tyr Trp Gly Gln Gly Thr Leu Val Thr Val Ser Ser (SEQ ID NO: 50109)

Asn Tyr Gly Met His (SEQ ID NO: 50585)

Val Ile Trp His Asp Gly Ser Asn Lys Tyr Tyr Ala Asp Ser Val Lys Gly (SEQ ID NO: 50586)

Glu Asn Ser Ser Ser Tyr Tyr Phe Asp Tyr Trp Gly Gln Gly Thr Leu Val Thr Val Ser Ser (SEQ ID NO: 50587)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | E | S | - | G | G | G | V | V | Q | P | G | R | S | L | R | L | S | C | A | A | S |
| 21-225_30G1_HC | . | . | . | . | . | . | . | . | . | . | . | . | . | . | . | . | . | . | . | . | . | . | . | . | . | . |
| 21-225_30G4_HC | . | . | . | . | . | . | . | . | . | . | . | . | . | . | . | . | . | . | . | . | . | . | . | . | . | . |
| 21-225_32D6_HC | . | . | . | . | . | . | . | . | . | . | . | . | . | . | . | . | . | . | . | . | . | . | . | . | . | . |
| 21-225_33B2_HC | . | . | . | . | . | . | . | . | . | . | . | . | . | . | . | . | . | . | . | . | . | . | . | . | . | . |
| 21-225_33C12_HC | . | . | . | . | . | . | . | . | . | . | . | . | . | . | . | . | . | . | . | . | . | . | . | . | . | . |
| 21-225_34C2_HC | . | . | . | . | . | . | . | . | . | . | . | . | . | . | . | . | . | . | . | . | . | . | . | . | . | . |
| 21-225_34D3_HC | . | . | . | . | . | . | . | . | . | . | . | . | . | . | . | . | . | . | . | . | . | . | . | . | . | . |
| 21-225_34D8_HC | . | . | . | . | . | . | . | . | . | . | . | . | . | . | . | . | . | . | . | . | . | . | . | . | . | . |
| 21-225_36B8_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | N | - | - | - | - | - | Y | G | M | H | W | V | R | Q | A | P | G | K | G | L |
| 21-225_30G1_HC | . | . | . | . | . | . | Y | . | . | . | . | . | . | . | . | . | . | . | . | . | . | . | . | . | . | . |
| 21-225_30G4_HC | . | . | . | . | . | . | S | . | . | . | . | . | . | . | . | . | . | . | . | . | . | . | . | R | . | . |
| 21-225_32D6_HC | . | . | . | . | . | . | S | . | . | . | . | . | . | . | . | . | . | . | . | . | . | . | . | . | . | . |
| 21-225_33B2_HC | . | . | . | . | . | . | . | . | . | . | . | . | . | . | . | . | . | . | . | . | . | . | . | . | . | . |
| 21-225_33C12_HC | . | . | . | . | . | . | H | . | . | . | . | . | . | . | . | . | . | . | . | . | . | . | . | . | . | . |
| 21-225_34C2_HC | . | . | . | . | . | . | . | . | . | . | . | . | . | V | . | . | . | . | . | . | . | . | . | . | . | . |
| 21-225_34D3_HC | . | . | . | . | . | . | Y | . | . | . | . | . | . | . | . | . | . | . | . | . | . | . | . | . | . | . |
| 21-225_34D8_HC | . | . | . | . | . | . | . | . | . | . | . | . | . | . | . | . | . | . | . | . | . | . | . | . | . | . |
| 21-225_36B8_HC | . | . | . | . | . | . | . | . | . | . | . | . | . | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | A | V | I | W | H | D | - | - | - | G | S | N | K | Y | Y | A | D | S | V | K | G | R | F |
| 21-225_30G1_HC | . | . | . | . | . | . | . | . | . | . | . | . | . | . | . | . | . | . | V | . | . | . | . | . | . | . |
| 21-225_30G4_HC | . | . | . | . | . | . | . | Y | . | . | . | . | . | T | D | . | . | . | V | . | . | . | . | . | . | . |
| 21-225_32D6_HC | . | . | . | T | . | . | . | . | . | . | . | . | . | . | . | A | . | . | . | . | . | . | . | . | . | . |
| 21-225_33B2_HC | . | . | . | . | . | . | . | . | . | . | . | . | . | . | . | . | . | . | . | . | . | . | . | . | . | . |
| 21-225_33C12_HC | . | . | . | . | . | . | . | Y | . | . | . | . | . | . | . | . | . | . | . | . | . | . | . | . | . | . |
| 21-225_34C2_HC | . | . | . | . | L | . | . | Y | . | . | . | . | . | . | . | . | . | . | . | . | . | . | . | . | . | . |
| 21-225_34D3_HC | . | . | . | . | . | . | . | . | . | . | . | . | . | . | . | . | . | . | V | . | . | . | . | . | . | . |
| 21-225_34D8_HC | . | . | . | . | . | . | . | Y | . | . | . | . | . | . | . | . | . | . | . | . | . | . | . | . | . | . |
| 21-225_36B8_HC | . | . | . | . | . | . | . | . | . | . | . | . | . | . | . | . | . | . | G | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_30G1_HC | . | . | . | . | . | . | . | . | . | . | . | . | . | . | . | . | . | . | . | . | . | . | . | . | . | . |
| 21-225_30G4_HC | . | . | . | . | . | . | . | . | . | . | . | . | . | . | . | . | . | . | . | . | . | . | S | . | . | . |
| 21-225_32D6_HC | . | . | . | . | . | . | . | . | . | . | . | . | . | . | . | . | . | . | . | . | . | . | . | . | . | . |
| 21-225_33B2_HC | . | . | . | . | . | . | . | . | . | . | . | . | . | . | . | . | . | . | . | . | . | . | . | . | . | . |
| 21-225_33C12_HC | . | . | . | . | . | . | . | . | . | . | . | . | . | . | . | . | . | . | . | . | . | . | . | . | . | . |
| 21-225_34C2_HC | . | . | . | . | . | . | . | . | . | . | . | . | . | . | . | . | . | . | . | . | . | . | . | . | . | . |
| 21-225_34D3_HC | . | . | . | . | . | . | . | . | . | . | . | . | . | . | . | . | . | . | . | . | . | . | . | . | . | . |
| 21-225_34D8_HC | . | . | . | . | . | . | . | . | . | . | . | . | . | . | . | . | . | . | . | . | . | . | . | . | . | . |
| 21-225_36B8_HC | . | . | . | . | . | . | . | . | . | . | . | . | . | . | . | . | . | . | . | . | . | . | . | . | . | . |

# FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | E | N | S | S | S | - | - | - | - | - | - | - | - | - | - | - | - | - | - | - | - | - |
| 21-225_30G1_HC | . | . | . | . | . | . | . | . | . | . | . | . | . | . | . | . | . | . | . | . | . | . | . | . | . | . |
| 21-225_30G4_HC | . | . | . | . | . | . | . | . | . | . | . | . | . | . | . | . | . | . | . | . | . | . | . | . | . | . |
| 21-225_32D6_HC | . | . | . | . | . | . | . | . | . | . | . | . | . | . | . | . | . | . | . | . | . | . | . | . | . | . |
| 21-225_33B2_HC | . | . | . | . | . | . | . | . | . | . | . | . | . | . | . | . | . | . | . | . | . | . | . | . | . | . |
| 21-225_33C12_HC | . | . | . | . | . | . | . | . | . | . | . | . | . | . | . | . | . | . | . | . | . | . | . | . | . | . |
| 21-225_34C2_HC | . | . | . | . | . | . | . | . | . | . | . | . | . | . | . | . | . | . | . | . | . | . | . | . | . | . |
| 21-225_34D3_HC | . | . | . | . | . | . | . | . | . | . | . | . | . | . | . | . | . | . | . | . | . | . | . | . | . | . |
| 21-225_34D8_HC | . | . | . | . | . | . | . | . | . | . | . | . | . | . | . | . | . | . | . | . | . | . | . | . | . | . |
| 21-225_36B8_HC | . | . | . | . | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | Y | Y | F | D | Y | W | G | Q | G | T | L | V | T | V | S | S |
| 21-225_30G1_HC | . | . | . | . | . | . | . | . | . | . | . | . | . | . | . | . | . | . | . |
| 21-225_30G4_HC | . | . | . | . | . | . | . | . | . | . | . | . | . | . | . | . | . | . | . |
| 21-225_32D6_HC | . | . | . | . | . | . | . | . | . | . | . | . | . | . | . | . | . | . | . |
| 21-225_33B2_HC | . | . | . | . | . | . | . | . | . | . | . | . | . | . | . | . | . | . | . |
| 21-225_33C12_HC | . | . | . | . | . | . | . | . | . | . | . | . | . | . | . | . | . | . | . |
| 21-225_34C2_HC | . | . | . | . | . | . | . | . | . | . | . | . | . | . | . | . | . | . | . |
| 21-225_34D3_HC | . | . | . | [illegible] | . | . | . | . | . | . | . | . | . | . | . | . | . | . | . |
| 21-225_34D8_HC | . | . | . | . | . | . | . | . | . | . | . | . | . | . | . | . | . | . | . |
| 21-225_36B8_HC | . | . | . | . | . | . | . | . | . | . | . | . | . | . | . | . | . | . | . |

**FIGURE 57**

Table 89. Consensus 55 - VH2|2-05/D1|1-1|RF1/JH3 (SEQ ID NO: 50306):

QITLKES-GPTLVKPTQTLTLTCTFSG-FSLSTS---GVGVGWIRQPPGKALEWLALINW----NDDKRYSPSLKSRFTITRDTSKDQVVLTMTNMDPVDTATYYCAHKATWV---------------------AFDIWGQGTMVTVSS wherein:

Y in position 70 can be substituted with F

A in position 110 can be substituted with T

Thr Ser Gly Val Gly Val Gly (SEQ ID NO: 50110)

Leu Ile Asn Trp Asn Asp Asp Lys Arg Xaa Ser Pro Ser Leu Lys Ser (SEQ ID NO: 50111)

Lys Xaa Thr Trp Val Ala Phe Asp Ile (SEQ ID NO: 50112)

Thr Ser Gly Val Gly Val Gly (SEQ ID NO: 50588)

Leu Ile Asn Trp Asn Asp Asp Lys Arg Tyr Ser Pro Ser Leu Lys Ser (SEQ ID NO: 50589)

Lys Ala Thr Trp Val Ala Phe Asp Ile (SEQ ID NO: 50590)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | I | T | L | K | E | S | - | G | P | T | L | V | K | P | T | Q | T | L | T | L | T | C | T | F | S |
| 21-225_62A12_HC | . | . | . | . | . | . | . | . | . | . | . | . | . | . | . | . | . | . | . | . | . | . | . | . | . | . |
| 21-225_62B12_HC | . | . | . | . | . | . | . | . | . | . | . | . | . | . | . | . | . | . | . | . | . | . | . | . | . | . |
| 21-225_62D10_HC | . | . | . | . | . | . | . | . | . | . | . | . | . | . | S | . | . | . | . | . | . | . | . | . | . | . |
| 21-225_62E3_HC | . | . | . | . | . | . | . | . | . | . | . | . | . | . | . | . | . | . | . | . | . | . | . | . | . | . |
| 21-225_62E8_HC | . | . | . | . | . | . | . | . | . | . | . | . | . | . | . | . | . | . | . | . | . | . | . | . | . | . |
| 21-225_66G9_HC | . | . | . | . | . | . | . | . | . | . | . | . | . | . | . | . | . | . | . | . | . | . | . | . | . | . |
| 21-225_72D5_HC | . | . | . | . | . | . | . | . | . | . | . | . | . | . | . | . | . | . | . | . | . | . | . | . | . | . |
| 21-225_73C4_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | S | L | S | T | S | - | - | - | G | V | G | V | G | W | I | R | Q | P | P | G | K | A | L |
| 21-225_62A12_HC | . | . | . | . | . | . | . | . | . | . | . | . | . | . | . | . | . | . | . | . | . | . | . | . | . | . |
| 21-225_62B12_HC | . | . | . | . | . | N | . | . | . | . | . | . | . | . | . | . | . | . | . | . | . | . | . | . | . | . |
| 21-225_62D10_HC | . | . | . | . | . | . | . | . | . | . | . | . | . | . | . | . | . | . | . | . | . | . | . | . | . | . |
| 21-225_62E3_HC | . | . | . | . | . | . | . | . | . | . | . | . | . | . | . | . | . | . | . | . | . | . | . | . | . | . |
| 21-225_62E8_HC | . | . | . | . | . | . | . | . | . | . | . | . | . | . | . | . | . | . | . | . | . | . | . | . | . | . |
| 21-225_66G9_HC | . | . | . | . | . | . | . | . | . | . | . | . | . | . | . | . | . | . | . | . | . | . | . | . | . | . |
| 21-225_72D5_HC | . | . | . | . | . | . | . | . | . | . | . | . | . | . | . | . | . | . | . | . | . | . | . | . | . | . |
| 21-225_73C4_HC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | L | A | L | I | N | W | - | - | - | - | N | D | D | K | R | Y | S | P | S | L | K | S | R | F |
| 21-225_62A12_HC | . | . | . | . | . | . | . | . | . | . | . | . | . | . | . | . | . | . | . | . | . | . | . | . | . | . |
| 21-225_62B12_HC | . | . | . | . | . | . | . | . | . | . | . | . | . | . | . | . | . | . | . | . | . | . | . | . | . | . |
| 21-225_62D10_HC | . | . | . | . | . | . | . | . | . | . | . | . | . | . | . | . | . | . | . | . | . | . | . | . | . | . |
| 21-225_62E3_HC | . | . | . | . | . | . | . | . | . | . | . | . | . | . | . | . | . | . | . | . | . | . | . | . | . | . |
| 21-225_62E8_HC | . | . | . | . | . | . | . | . | . | . | . | . | . | . | . | . | . | . | . | . | . | . | . | . | . | . |
| 21-225_66G9_HC | . | . | . | . | . | . | . | . | . | . | . | . | . | . | . | . | . | F | . | . | . | . | . | . | . | . |
| 21-225_72D5_HC | . | . | . | . | . | . | . | . | . | . | . | . | . | . | . | . | . | . | . | . | . | . | . | . | . | . |
| 21-225_73C4_HC | . | . | . | . | . | . | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | T | R | D | T | S | K | D | Q | V | V | L | T | M | T | N | M | D | P | V | D | T | A | T | Y |
| 21-225_62A12_HC | . | . | . | . | . | . | . | . | . | . | . | . | . | . | . | . | . | . | . | . | L | . | . | . | . | . |
| 21-225_62B12_HC | . | . | . | . | . | . | . | . | . | . | . | . | . | . | . | . | . | . | . | . | . | . | . | . | . | . |
| 21-225_62D10_HC | . | . | . | . | . | . | . | . | . | . | . | . | . | . | . | . | . | . | . | . | . | . | . | . | . | . |
| 21-225_62E3_HC | . | . | . | . | . | . | . | . | . | . | . | . | . | . | . | . | . | . | . | . | . | . | . | . | . | . |
| 21-225_62E8_HC | . | . | . | . | . | . | . | . | . | . | . | . | . | . | . | . | . | . | . | . | . | . | . | . | . | . |
| 21-225_66G9_HC | . | . | . | . | . | . | . | . | . | . | . | . | . | . | . | . | . | . | . | . | . | . | . | . | . | . |
| 21-225_72D5_HC | . | . | . | . | . | . | . | . | . | . | . | . | F | . | . | . | . | . | . | . | . | . | . | G | . | . |
| 21-225_73C4_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | H | K | A | T | W | V | - | - | - | - | - | - | - | - | - | - | - | - | - | - | - | - | - |
| 21-225_62A12_HC | . | . | . | . | . | T | . | . | . | . | . | . | . | . | . | . | . | . | . | . | . | . | . | . | . | . |
| 21-225_62B12_HC | . | . | . | . | . | . | . | . | . | . | . | . | . | . | . | . | . | . | . | . | . | . | . | . | . | . |
| 21-225_62D10_HC | . | . | . | . | . | . | . | . | . | . | . | . | . | . | . | . | . | . | . | . | . | . | . | . | . | . |
| 21-225_62E3_HC | . | . | . | . | . | . | . | . | . | . | . | . | . | . | . | . | . | . | . | . | . | . | . | . | . | . |
| 21-225_62E8_HC | . | . | . | . | . | . | . | . | . | . | . | . | . | . | . | . | . | . | . | . | . | . | . | . | . | . |
| 21-225_66G9_HC | . | . | . | . | . | . | . | . | . | . | . | . | . | . | . | . | . | . | . | . | . | . | . | . | . | . |
| 21-225_72D5_HC | . | . | . | . | . | . | . | . | . | . | . | . | . | . | . | . | . | . | . | . | . | . | . | . | . | . |
| 21-225_73C4_HC | . | . | . | . | . | T | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | - | - | - | - | A | F | D | I | W | G | Q | G | T | M | V | T | V | S | S |
| 21-225_62A12_HC | . | . | . | . | . | . | . | . | . | . | . | . | . | . | . | . | . | . | . |
| 21-225_62B12_HC | . | . | . | . | . | . | . | . | . | . | . | . | . | . | . | . | . | . | . |
| 21-225_62D10_HC | . | . | . | . | . | . | . | . | . | . | . | . | . | . | . | . | . | . | . |
| 21-225_62E3_HC | . | . | . | . | . | . | . | . | . | . | . | . | . | . | . | . | . | . | . |
| 21-225_62E8_HC | . | . | . | . | . | . | . | . | . | . | . | . | . | . | . | . | . | . | . |
| 21-225_66G9_HC | . | . | . | . | . | . | . | . | . | . | . | . | . | . | . | . | . | . | . |
| 21-225_72D5_HC | . | . | . | . | . | . | . | . | . | . | . | . | . | . | . | . | . | . | . |
| 21-225_73C4_HC | . | . | . | . | . | . | . | . | . | . | . | . | . | . | . | . | . | . | . |

**FIGURE 57**

Table 90. Consensus 56 - VH3|3-23/D4|4-11|RF3/JH4 (SEQ ID NO: 50307):

EVQLLES-GGGLVQPGGSLRLSCAASG-FTFSS-----YVMNWVRQAPGKGLEWVSAISGS---GGRTYYADSVKGRFTISRDNSKNTLYLQMNSLRAEDTAVYYCARTAT-----------------------FDYWGQGTLVTVSS wherein:

V at position 40 can be substituted with A

M at position 41 can be substituted with I or L

N at position 42 can be substituted with S or R

A at position 57 can be substituted with D

I at position 58 can be substituted with M

G at position 66 can be substituted with D or V

R at position 67 can be substituted with S, F or T

A at position 71 can be substituted with V

A at position 110 can be substituted with G, S, T or Y

T at position 111 can be substituted with V, null (-), H, L or G

F at position 136 can be substituted with null (-) or K

D at position 137 can be substituted with null (-)

Y at position 138 can be substituted with L

Ser Tyr Xaa Xaa Xaa (SEQ ID NO: 50113)

Xaa Xaa Ser Gly Ser Gly Xaa Xaa Thr Tyr Tyr Xaa Asp Ser Val Lys Gly (SEQ ID NO: 50114)

Thr Xaa Xaa Xaa Xaa Xaa (SEQ ID NO: 50115)

Ser Tyr Val Met Asn (SEQ ID NO: 50591)

**FIGURE 57**

Ala Ile Ser Gly Ser Gly Gly Arg Thr Tyr Tyr Ala Asp Ser Val Lys Gly (SEQ ID NO: 50592)

Thr Ala Thr Phe Asp Tyr (SEQ ID NO: 50593)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | E | V | Q | L | L | E | S | - | G | G | G | L | V | Q | P | G | G | S | L | R | L | S | C | A | A | S |
| 21-225_153A1_HC | . | . | . | . | . | . | . | . | . | . | . | . | . | . | . | . | . | . | . | . | . | . | . | . | . | . |
| 21-225_157H1_HC | . | . | . | . | . | . | . | . | . | . | . | . | . | . | . | . | . | . | . | . | . | . | . | . | . | . |
| 21-225_175C4_HC | . | . | . | . | . | . | . | . | . | . | . | . | . | . | . | . | . | . | . | . | . | . | . | . | . | . |
| 21-225_23C7_HC | . | . | . | . | . | . | . | . | . | . | . | . | . | . | . | . | . | . | . | . | . | . | . | . | . | . |
| 21-225_23E7_HC | . | . | . | . | . | . | . | . | . | . | . | . | . | . | . | . | . | . | . | . | . | . | . | T | . | . |
| 21-225_30A1_HC | . | . | . | . | . | . | . | . | . | . | . | . | . | . | . | . | . | . | . | . | . | . | . | . | . | . |
| 21-225_33A5_HC | . | . | . | . | . | . | . | . | . | . | . | . | . | . | . | . | . | . | . | . | . | . | . | . | . | . |
| 21-225_56F7_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | V | M | N | W | V | R | Q | A | P | G | K | G | L |
| 21-225_153A1_HC | . | . | . | S | . | . | . | . | . | . | . | . | . | . | . | S | . | . | . | . | G | . | . | . | . | . |
| 21-225_157H1_HC | . | . | . | . | . | . | . | . | . | . | . | . | . | A | . | [illegible] | . | I | . | . | . | . | . | . | . | . |
| 21-225_175C4_HC | A | . | . | . | . | . | . | . | . | . | . | . | . | . | . | S | . | . | . | . | . | . | . | . | . | . |
| 21-225_23C7_HC | . | . | . | . | . | . | . | . | . | . | . | . | . | . | . | S | . | . | . | . | . | . | . | . | . | . |
| 21-225_23E7_HC | . | . | . | . | . | . | . | . | . | . | . | . | . | A | . | . | . | . | . | . | . | . | . | . | . | . |
| 21-225_30A1_HC | . | . | . | . | . | . | . | . | . | . | . | . | . | . | . | . | . | . | . | . | . | . | . | . | . | . |
| 21-225_33A5_HC | . | . | . | . | . | . | . | . | . | . | . | . | . | . | I | . | . | . | . | . | . | . | . | . | . | . |
| 21-225_56F7_HC | . | . | . | . | . | . | . | . | . | . | . | . | . | . | L | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | S | A | I | S | G | S | - | - | - | G | G | | T | Y | Y | A | D | S | V | K | G | R | F |
| 21-225_153A1_HC | . | . | . | . | . | . | . | . | . | . | . | . | . | D | R | . | . | . | . | . | . | . | . | . | . | . |
| 21-225_157H1_HC | Q | . | . | . | D | . | . | . | . | . | . | . | . | . | T | . | . | . | . | . | . | . | . | . | . | . |
| 21-225_175C4_HC | . | . | . | . | . | . | . | . | . | . | . | . | . | D | R | . | . | . | . | . | . | . | . | . | . | . |
| 21-225_23C7_HC | . | . | . | . | . | M | . | . | . | . | . | . | . | . | S | . | . | . | V | . | . | . | . | . | . | . |
| 21-225_23E7_HC | . | . | . | . | . | . | . | . | . | . | . | . | . | . | F | . | . | . | . | . | . | . | . | . | . | . |
| 21-225_30A1_HC | . | . | . | . | . | . | . | . | . | . | . | . | . | . | S | . | . | . | . | . | . | . | . | . | . | . |
| 21-225_33A5_HC | . | . | . | . | . | . | . | . | . | . | . | . | . | V | S | . | . | . | . | . | . | . | . | . | . | . |
| 21-225_56F7_HC | . | . | . | . | . | M | . | . | . | . | . | . | . | . | R | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_153A1_HC | . | . | . | . | . | . | . | . | . | . | . | . | . | . | . | . | . | . | . | . | . | . | . | . | . | . |
| 21-225_157H1_HC | . | . | . | . | . | . | . | . | . | . | . | . | . | . | . | . | . | . | . | . | . | . | . | . | . | . |
| 21-225_175C4_HC | . | . | . | . | . | D | . | N | T | . | . | . | . | . | . | . | . | . | . | . | . | . | . | . | . | . |
| 21-225_23C7_HC | . | . | . | . | . | . | . | . | . | . | . | . | . | . | . | . | . | . | . | . | . | . | . | . | . | . |
| 21-225_23E7_HC | . | . | . | . | . | . | . | R | . | . | . | . | . | . | . | . | . | . | . | . | . | . | . | . | . | . |
| 21-225_30A1_HC | . | . | . | . | N | . | . | . | . | . | . | . | . | . | . | . | . | . | . | . | . | . | . | . | . | . |
| 21-225_33A5_HC | . | . | . | . | . | . | . | . | . | . | . | . | . | . | . | . | . | . | . | . | . | . | . | . | . | . |
| 21-225_56F7_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | T | | | - | - | - | - | - | - | - | - | - | - | - | - | - | - | - | - | - | - | - |
| 21-225_153A1_HC | . | . | . | . | . | A | T | . | . | . | . | . | . | . | . | . | . | . | . | . | . | . | . | . | . | . |
| 21-225_157H1_HC | . | . | . | K | . | Y | - | . | . | . | . | . | . | . | . | . | . | . | . | . | . | . | . | . | . | . |
| 21-225_175C4_HC | . | . | . | . | . | A | G | . | . | . | . | . | . | . | . | . | . | . | . | . | . | . | . | . | . | . |
| 21-225_23C7_HC | . | . | . | . | . | G | V | . | . | . | . | . | . | . | . | . | . | . | . | . | . | . | . | . | . | . |
| 21-225_23E7_HC | . | . | . | . | . | S | G | . | . | . | . | . | . | . | . | . | . | . | . | . | . | . | . | . | . | . |
| 21-225_30A1_HC | . | . | . | . | . | G | V | . | . | . | . | . | . | . | . | . | . | . | . | . | . | . | . | . | . | . |
| 21-225_33A5_HC | . | . | . | . | . | S | L | . | . | . | . | . | . | . | . | . | . | . | . | . | . | . | . | . | . | . |
| 21-225_56F7_HC | F | . | . | M | . | T | H | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | - | - | - | F | D | Y | W | G | Q | G | T | L | V | T | V | S | S |
| 21-225_153A1_HC | . | . | . | . | . | K | . | . | . | . | . | . | . | . | . | . | . | . | . |
| 21-225_157H1_HC | . | . | . | . | . | - | - | L | . | . | . | . | . | . | . | . | . | . | . |
| 21-225_175C4_HC | . | . | . | . | . | . | . | . | . | . | . | . | . | . | . | . | . | . | . |
| 21-225_23C7_HC | . | . | . | . | . | . | . | . | . | . | . | . | . | . | . | I | . | . | . |
| 21-225_23E7_HC | . | . | . | . | . | . | . | . | . | . | . | . | . | . | . | . | . | . | . |
| 21-225_30A1_HC | . | . | . | . | . | . | . | . | . | . | . | . | . | . | . | . | . | . | . |
| 21-225_33A5_HC | . | . | . | . | . | . | . | . | . | . | . | . | . | . | . | . | . | . | . |
| 21-225_56F7_HC | . | . | . | . | . | . | . | . | . | . | . | . | . | . | . | . | . | . | . |

**FIGURE 57**

Table 91. Consensus 57 -VH3|3-23/D7|7-27|RF1/JH3 (SEQ ID NO: 50308):

EVQLLES-GGGLVQPGGSLRLSCAASG-FTFSS-----YAMSWVRQAPGKGLEWVSVISGR---GGTTFYADSVKGRFTISRDNSKNTLYLQMNSLRAEDTAVYYCAKRTPSD---------------------VFDIWGQGTMVTVSS wherein:

Y at position 39 can be substituted with F

S at position 42 can be substituted with N

V at position 57 can be substituted with A or I

I at position 58 can be substituted with L

R at position 61 can be substituted with S or G

G at position 66 can be substituted with S or K

T at position 67 can be substituted with N or S

F at position 69 can be substituted with Y

P at position 111 can be substituted with G

S at position 112 can be substituted with D or E

V at position 135 can be substituted with A

I at position 138 can be substituted with V

Ser Xaa Ala Met Xaa (SEQ ID NO: 50116)

Xaa Xaa Ser Gly Xaa Gly Xaa Xaa Thr Xaa Tyr Ala Asp Ser Val Lys Gly (SEQ ID NO: 50117)

Lys Arg Thr Xaa Xaa Asp Xaa Phe Asp Xaa (SEQ ID NO: 50118)

Ser Tyr Ala Met Ser (SEQ ID NO: 50594)

Val Ile Ser Gly Arg Gly Gly Thr Thr Phe Tyr Ala Asp Ser Val Lys Gly (SEQ ID NO: 50595)

# FIGURE 57

Lys Arg Thr Pro Ser Asp Val Phe Asp Ile (SEQ ID NO: 50596)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | | | | | | H_FR1 | | | | | | | | | |
| CONSENSUS | E | V | Q | L | L | E | S | - | G | G | G | L | V | Q | P | G | G | S | L | R | L | S | C | A | A | S |
| 21-225_149B6_HC | . | . | . | . | . | . | . | . | . | . | . | . | . | . | . | . | . | . | . | . | . | . | . | . | . | . |
| 21-225_180A3_HC | . | . | . | . | . | . | . | . | . | . | . | . | E | . | . | . | . | . | . | . | . | . | . | . | . | . |
| 21-225_182H5_HC | . | . | . | . | . | . | . | . | . | . | . | . | . | . | . | . | . | . | . | . | . | . | . | . | . | . |
| 21-225_186A11_HC | . | . | . | . | . | . | . | . | . | . | . | . | . | . | . | . | . | . | . | . | . | . | . | . | . | . |
| 21-225_43H9_HC | . | . | . | . | . | . | . | . | . | . | . | . | . | . | . | . | . | . | . | . | . | . | . | . | . | . |
| 21-225_44F3_HC | . | . | H | . | . | . | . | . | . | . | . | . | . | . | . | . | . | . | . | . | . | . | . | . | . | . |
| 21-225_45C9_HC | . | . | . | . | . | . | . | . | . | . | . | . | . | . | . | . | . | . | . | . | . | . | . | . | . | . |
| 21-225_45H4_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | | | | | H_CDR1 | | | | | | | | | | | | | H_FR2 | | |
| CONSENSUS | G | - | F | T | F | S | S | - | - | - | - | - | Y | A | M | S | W | V | R | Q | A | P | G | K | G | L |
| 21-225_149B6_HC | . | . | . | . | . | . | . | . | . | . | . | . | . | . | . | . | . | . | . | . | . | . | . | . | . | . |
| 21-225_180A3_HC | . | . | . | . | . | . | . | . | . | . | . | . | F | . | . | N | . | . | . | . | . | . | . | . | . | . |
| 21-225_182H5_HC | . | . | . | . | . | . | . | . | . | . | . | . | . | . | . | . | . | . | . | . | . | . | . | . | . | . |
| 21-225_186A11_HC | . | . | . | . | . | . | . | . | . | . | . | . | . | . | . | . | . | . | . | . | . | . | . | . | . | . |
| 21-225_43H9_HC | . | . | . | . | . | . | . | . | . | . | . | . | . | . | . | . | . | . | . | . | . | . | . | M | . | . |
| 21-225_44F3_HC | . | . | . | . | . | . | . | . | . | . | . | . | . | . | . | . | . | . | . | . | . | . | . | . | . | . |
| 21-225_45C9_HC | . | . | . | . | . | N | . | . | . | . | . | . | . | . | . | . | . | . | . | . | . | . | . | . | . | . |
| 21-225_45H4_HC | . | . | . | . | . | . | . | . | . | . | . | . | . | . | . | . | . | . | . | . | . | . | . | M | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | S | V | I | S | G | R | - | - | - | G | G | T | T | F | Y | A | D | S | V | K | G | R | F |
| 21-225_149B6_HC | . | . | . | . | . | . | . | . | S | . | . | . | . | . | S | . | . | . | . | . | . | . | . | . | . | . |
| 21-225_180A3_HC | . | . | . | . | . | . | . | . | . | . | . | . | . | . | N | . | . | . | . | . | . | . | . | . | . | . |
| 21-225_182H5_HC | . | . | . | . | . | . | . | . | . | . | . | . | . | . | . | . | . | . | . | . | . | . | . | . | . | . |
| 21-225_186A11_HC | . | . | . | . | . | . | . | . | . | . | . | . | . | . | . | . | . | . | . | . | . | . | . | . | . | . |
| 21-225_43H9_HC | . | . | . | . | A | . | . | . | S | . | . | . | . | S | N | . | . | . | . | . | . | . | . | . | . | . |
| 21-225_44F3_HC | . | . | . | . | I | L | . | . | G | . | . | . | . | K | . | . | Y | . | . | . | . | . | . | . | . | . |
| 21-225_45C9_HC | . | . | . | . | . | . | . | . | . | . | . | . | . | . | . | . | Y | . | . | . | . | . | . | . | . | . |
| 21-225_45H4_HC | . | . | . | . | A | . | . | . | S | . | . | . | . | S | N | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_149B6_HC | . | . | . | . | . | . | . | . | . | . | . | F | . | . | . | . | . | . | . | . | . | . | . | . | . | . |
| 21-225_180A3_HC | . | . | . | . | . | . | . | . | . | . | . | F | . | . | . | . | . | . | . | . | . | . | . | . | . | . |
| 21-225_182H5_HC | . | . | . | . | . | . | . | . | . | . | . | . | . | . | . | . | . | . | . | . | . | . | . | . | . | . |
| 21-225_186A11_HC | . | . | . | . | . | . | . | . | . | . | . | . | . | . | . | . | . | . | . | . | . | . | . | . | . | . |
| 21-225_43H9_HC | . | . | . | . | . | . | . | . | . | . | . | . | . | H | . | . | . | . | . | . | . | . | . | . | . | . |
| 21-225_44F3_HC | . | . | . | . | . | . | . | . | . | . | . | F | . | . | . | . | . | . | . | . | . | . | . | . | . | . |
| 21-225_45C9_HC | . | . | . | . | . | . | . | . | . | . | . | . | . | . | . | . | . | . | . | . | . | . | . | . | . | . |
| 21-225_45H4_HC | . | . | . | . | . | . | . | . | . | . | . | . | . | H | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | K | R | T | P | S | D | - | - | - | - | - | - | - | - | - | - | - | - | - | - | - | - | - |
| 21-225_149B6_HC | . | . | . | . | . | . | . | E | . | . | . | . | . | . | . | . | . | . | . | . | . | . | . | . | . | . |
| 21-225_180A3_HC | F | . | . | . | . | . | G | D | . | . | . | . | . | . | . | . | . | . | . | . | . | . | . | . | . | . |
| 21-225_182H5_HC | . | . | . | . | . | . | G | D | . | . | . | . | . | . | . | . | . | . | . | . | . | . | . | . | . | . |
| 21-225_186A11_HC | . | . | . | . | . | . | G | D | . | . | . | . | . | . | . | . | . | . | . | . | . | . | . | . | . | . |
| 21-225_43H9_HC | F | . | . | . | . | . | . | . | . | . | . | . | . | . | . | . | . | . | . | . | . | . | . | . | . | . |
| 21-225_44F3_HC | . | . | . | . | . | . | . | . | . | . | . | . | . | . | . | . | . | . | . | . | . | . | . | . | . | . |
| 21-225_45C9_HC | . | . | . | . | . | . | . | . | . | . | . | . | . | . | . | . | . | . | . | . | . | . | . | . | . | . |
| 21-225_45H4_HC | F | . | . | . | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | - | - | V | F | D | I | W | G | Q | G | T | M | V | T | V | S | S |
| 21-225_149B6_HC | . | . | . | . | . | . | . | . | . | . | . | . | . | . | . | . | . | . | . |
| 21-225_180A3_HC | . | . | . | . | . | . | . | V | . | . | . | . | . | . | . | . | . | . | . |
| 21-225_182H5_HC | . | . | . | . | . | . | . | . | . | . | . | . | . | . | . | . | . | . | . |
| 21-225_186A11_HC | . | . | . | . | . | . | . | . | . | . | . | . | . | . | . | . | . | . | . |
| 21-225_43H9_HC | . | . | . | . | . | . | . | . | . | . | . | . | . | . | . | . | . | . | . |
| 21-225_44F3_HC | . | . | . | . | A | . | . | . | . | . | . | . | . | . | . | . | . | . | . |
| 21-225_45C9_HC | . | . | . | . | A | . | . | . | . | . | . | . | . | . | . | . | . | . | . |
| 21-225_45H4_HC | . | . | . | . | . | . | . | . | . | . | . | . | . | . | . | . | . | . | . |

**FIGURE 57**

Table 92. Consensus 58 -VH3|3-33/D4|4-11|RF2/JH4 (SEQ ID NO: 50309):

QVQLVES-GGGVVQPGRSLRLSCAASG-FTFSS-----YGMHWVRQAPGKGLEWVAVIWYD---GSNKYYADSVKGRFTISRDNSKNTLYLQMNSLRAEDTAVYYCARDRPRS---------------------SAF DYWGQGTLVTVSS wherein:

S at position 33 can be substituted with N or T

Y at position 39 can be substituted with F

G at position 40 can be substituted with D or N

V at position 57 can be substituted with A

Y at position 60 can be substituted with H

S at position 66 can be substituted with R

N at position 67 can be substituted with D or H

K at position 68 can be substituted with R

Y at position 70 can be substituted with C or S

A at position 71 can be substituted with E or T

R at position 110 can be substituted with D or H

P at position 111 can be substituted with S or A

I at position 112 can be substituted with R or Y

S at position 113 can be substituted with L, V or W

Null (-) at position 114 can be substituted with G

**FIGURE 57**

Null (-) at position 133 can be substituted with A

S at position 134 can be substituted with T or A

A at position 135 can be substituted with F, S or Y

F at position 136 can be substituted with G or S

Y at position 138 can be substituted with F

Xaa Xaa Xaa Met His (SEQ ID NO: 50237)

Xaa Ile Trp Xaa Asp Gly Xaa Xaa Xaa Tyr Xaa Xaa Asp Ser Val Lys Gly (SEQ ID NO: 50238)

Asp Xaa Xaa Xaa Xaa Xaa Xaa Xaa Xaa Xaa Asp Xaa (SEQ ID NO: 50239)

Ser Tyr Gly Met His (SEQ ID NO: 50597)

Val Ile Trp Tyr Asp Gly Ser Asn Lys Tyr Tyr Ala Asp Ser Val Lys Gly (SEQ ID NO: 50598)

Asp Arg Pro Arg Ser Ser Ala Phe Asp Tyr (SEQ ID NO: 50599)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | V | E | S | - | G | G | G | V | V | Q | P | G | R | S | L | R | L | S | C | A | A | S |
| 21-225_180F8_HC | . | . | . | . | . | . | . | . | . | . | . | . | . | . | . | . | . | . | . | . | . | . | . | . | . | . |
| 21-225_19B3_HC | . | . | . | . | . | . | . | . | . | . | . | . | . | . | . | . | . | . | . | . | . | . | . | . | . | . |
| 21-225_212C10_HC | . | . | . | . | . | . | . | . | . | . | . | . | . | . | . | . | . | . | . | . | . | . | . | . | . | . |
| 21-225_63C10_HC | . | . | . | . | . | . | . | . | . | . | . | . | . | . | . | . | . | . | . | . | . | . | . | . | . | . |
| 21-225_70H6_HC | . | . | . | . | . | . | . | . | . | . | . | . | . | . | . | . | . | . | . | . | . | . | . | . | . | . |
| 21-225_74C3_HC | . | . | . | . | . | . | . | . | . | . | . | . | . | . | . | . | . | . | . | . | . | . | . | . | . | . |
| 21-225_77H5_HC | . | . | . | . | . | . | . | . | . | . | . | . | . | . | . | . | . | . | . | . | . | . | . | . | . | . |
| 21-225_8H11_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | G | M | H | W | V | R | Q | A | P | G | K | G | L |
| 21-225_180F8_HC | . | . | . | . | . | . | . | . | . | . | . | . | . | . | . | . | . | . | . | . | . | . | . | . | . | . |
| 21-225_19B3_HC | . | . | . | . | . | . | . | . | . | . | . | . | . | D | . | . | . | . | . | . | . | . | . | . | . | . |
| 21-225_212C10_HC | . | . | . | . | . | . | . | . | . | . | . | . | . | . | . | . | . | . | . | . | . | . | . | . | . | . |
| 21-225_63C10_HC | . | . | . | . | . | . | . | . | . | . | . | . | . | . | . | . | . | . | . | . | . | . | . | . | . | . |
| 21-225_70H6_HC | . | . | . | . | . | . | . | . | . | . | . | . | . | . | . | . | . | . | . | . | T | . | . | . | . | . |
| 21-225_74C3_HC | . | . | . | . | . | . | . | . | . | . | . | . | F | . | . | . | . | A | . | . | . | . | . | . | . | . |
| 21-225_77H5_HC | . | . | . | . | . | . | N | . | . | . | . | . | . | . | . | . | . | . | . | . | . | . | . | . | . | . |
| 21-225_8H11_HC | . | . | . | . | . | . | T | . | . | . | . | . | . | N | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | A | V | I | W | Y | D | - | - | - | G | S | N | K | Y | Y | A | D | S | V | K | G | R | F |
| 21-225_180F8_HC | . | . | . | S | . | . | . | . | . | . | . | . | . | . | D | . | . | . | E | . | . | . | . | . | . | . |
| 21-225_19B3_HC | . | . | . | . | . | . | . | . | . | . | . | . | . | . | D | . | . | . | . | . | . | . | . | . | . | . |
| 21-225_212C10_HC | . | . | . | T | A | . | . | . | . | . | . | . | . | . | H | . | . | . | T | . | . | . | . | . | . | . |
| 21-225_63C10_HC | D | . | . | . | . | . | . | . | . | . | . | . | . | . | . | . | . | . | . | . | . | . | . | . | . | . |
| 21-225_70H6_HC | D | . | . | . | . | . | . | H | . | . | . | . | . | . | . | . | . | . | . | . | . | . | . | . | . | . |
| 21-225_74C3_HC | . | . | . | . | A | . | . | . | . | . | . | . | . | . | . | . | . | C | . | . | . | . | . | . | . | . |
| 21-225_77H5_HC | . | . | . | . | . | . | . | . | . | . | . | . | . | R | . | . | . | . | . | . | . | . | . | . | . | . |
| 21-225_8H11_HC | . | . | . | . | . | . | . | . | . | . | . | . | . | . | D | R | . | S | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | S | K | N | T | L | Y | L | Q | M | N | S | L | R | A | E | D | T | A | V | Y |
| 21-225_180F8_HC | . | . | . | . | . | . | . | . | . | . | . | . | . | . | . | . | . | . | . | . | . | . | . | . | . | . |
| 21-225_19B3_HC | . | . | . | . | . | . | . | . | . | . | . | F | . | . | . | . | . | . | . | . | . | . | . | . | . | . |
| 21-225_212C10_HC | . | . | . | . | . | . | . | . | . | . | . | . | . | . | . | . | . | . | . | . | . | . | . | . | . | . |
| 21-225_63C10_HC | . | . | . | . | . | . | . | . | . | . | . | . | . | . | . | . | . | . | . | . | . | . | . | . | . | . |
| 21-225_70H6_HC | . | . | . | . | . | . | . | . | . | . | . | . | . | . | . | . | . | . | . | . | . | . | . | . | . | . |
| 21-225_74C3_HC | . | . | . | . | . | . | . | . | . | . | . | S | . | . | . | . | . | . | . | . | . | . | S | . | . | . |
| 21-225_77H5_HC | . | . | . | . | . | . | . | . | . | . | . | . | . | . | . | . | . | . | . | . | . | . | . | . | L | . |
| 21-225_8H11_HC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | R | | | S | - | - | - | - | - | - | - | - | - | - | - | - | - | - | - | - | - |
| 21-225_180F8_HC | . | . | . | . | . | H | P | R | W | . | . | . | . | . | . | . | . | . | . | . | . | . | . | . | . | . |
| 21-225_19B3_HC | . | . | . | . | . | . | A | Y | . | . | . | . | . | . | . | . | . | . | . | . | . | . | . | . | . | . |
| 21-225_212C10_HC | . | . | . | . | . | . | S | I | V | G | . | . | . | . | . | . | . | . | . | . | . | . | . | . | . | . |
| 21-225_63C10_HC | . | . | . | . | . | D | P | R | . | . | . | . | . | . | . | . | . | . | . | . | . | . | . | . | . | . |
| 21-225_70H6_HC | . | . | . | . | . | D | P | R | . | . | . | . | . | . | . | . | . | . | . | . | . | . | . | . | . | . |
| 21-225_74C3_HC | . | . | . | . | . | . | S | I | L | G | . | . | . | . | . | . | . | . | . | . | . | . | . | . | . | . |
| 21-225_77H5_HC | . | . | . | . | . | . | S | I | L | G | . | . | . | . | . | . | . | . | . | . | . | . | . | . | . | . |
| 21-225_8H11_HC | . | . | . | . | . | . | A | Y | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | - | S | | | D | Y | W | G | Q | G | T | L | V | T | V | S | S |
| 21-225_180F8_HC | . | . | . | . | Y | G | . | . | . | . | . | . | . | . | . | . | . | . | . |
| 21-225_19B3_HC | . | . | . | . | S | S | . | . | . | . | . | . | . | . | . | . | . | . | . |
| 21-225_212C10_HC | . | . | A | T | Y | F | . | . | . | . | . | . | . | . | . | . | . | . | . |
| 21-225_63C10_HC | . | . | . | . | A | G | . | . | . | . | . | . | . | . | . | . | . | . | . |
| 21-225_70H6_HC | . | . | . | . | A | G | . | . | . | . | . | . | . | . | . | . | . | . | . |
| 21-225_74C3_HC | . | . | A | A | F | F | . | . | . | . | . | . | . | . | . | . | . | . | . |
| 21-225_77H5_HC | . | . | A | T | F | F | . | . | . | . | . | . | . | . | . | . | . | . | . |
| 21-225_8H11_HC | . | . | . | . | S | S | . | F | . | . | . | . | . | . | . | . | . | . | . |

## FIGURE 57

Table 93. Consensus 59 - VH3|3-48/D4|4-11|RF2/JH6 (SEQ ID NO: 50310):

EVQLVES-GGGLVQPGGSLRLSCAASG-FTFSS-----YNMNWVRQAPGKGLEWVSYISRS---SNTKYYADSVKGRFTISRDNAKNSLYLQMNSLRDEDTAVYYCARDRSGSYGY---------------FYYYGLDVWGQGTTVTVSS wherein:

S at position 33 can be substituted with N

N at position 40 can be substituted with S

R at position 60 can be substituted with S

S at position 65 can be substituted with G

N at position 66 can be substituted with S

K at position 68 can be substituted with T

Y at position 69 can be substituted with H

A at position 71 can be substituted with V

K at position 75 can be substituted with R, E or Q

R at position 110 can be substituted with S

S at position 111 can be substituted with R

G at position 112 can be substituted with K

S at position 113 can be substituted with G

Y at position 114 can be substituted with F

G at position 115 can be substituted with null (-)

Y at position 116 can be substituted with null (-)

**FIGURE 57**

F at position 131 can be substituted with null (-)

Y at position 132 can be substituted with null (-)

L at position 136 can be substituted with M

Xaa Tyr Xaa Met Asn (SEQ ID NO: 50240)

Tyr Ile Ser Xaa Ser Xaa Xaa Thr Xaa Xaa Tyr Xaa Asp Ser Val Xaa Gly (SEQ ID NO: 50241)

Asp Xaa Xaa Xaa Xaa Xaa Xaa Xaa Xaa Xaa Tyr Tyr Gly Xaa Asp Val (SEQ ID NO: 50242)

Ser Tyr Asn Met Asn (SEQ ID NO: 50600)

Tyr Ile Ser Arg Ser Ser Asn Thr Lys Tyr Tyr Ala Asp Ser Val Lys Gly (SEQ ID NO: 50601)

Asp Arg Ser Gly Ser Tyr Gly Tyr Phe Tyr Tyr Tyr Gly Leu Asp Val (SEQ ID NO: 50602)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | E | V | Q | L | V | E | S | - | G | G | G | L | V | Q | P | G | G | S | L | R | L | S | C | A | A | S |
| 21-225_146A8_HC | . | . | K | . | . | . | . | . | . | . | . | . | . | . | . | . | E | . | . | . | . | . | . | V | . | . |
| 21-225_146B6_HC | . | . | . | . | . | . | . | . | . | . | . | . | . | . | . | . | . | . | . | . | . | . | . | . | . | . |
| 21-225_146D8_HC | . | . | . | . | . | . | . | . | . | . | . | . | . | . | . | . | . | . | . | . | . | . | . | . | . | . |
| 21-225_149A1_HC | . | . | . | . | . | . | . | . | . | . | . | . | . | . | . | . | . | . | . | . | . | . | . | . | . | . |
| 21-225_150F9_HC | . | M | . | . | . | . | . | . | . | . | . | . | . | . | . | . | . | . | . | . | . | . | . | . | . | . |
| 21-225_156H2_HC | . | . | . | . | . | . | . | . | . | . | . | . | I | . | . | . | . | . | . | . | . | . | . | . | . | . |
| 21-225_170E4_HC | . | . | . | . | . | . | . | . | . | . | . | S | . | . | . | . | . | . | . | . | . | . | . | . | . | . |
| 21-225_179D10_HC | . | . | . | . | . | . | . | . | . | . | . | . | . | . | . | . | . | . | . | . | F | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | F | T | F | S | S | - | - | - | - | - | Y | N | M | N | W | V | R | Q | A | P | G | K | G | L |
| 21-225_146A8_HC | . | . | . | . | . | . | N | . | . | . | . | . | . | . | . | . | . | . | . | . | . | . | . | . | . | . |
| 21-225_146B6_HC | . | . | . | . | . | . | . | . | . | . | . | . | . | . | . | . | . | . | . | . | . | . | . | . | . | . |
| 21-225_146D8_HC | . | . | . | . | . | . | N | . | . | . | . | . | . | . | . | . | . | . | . | . | . | . | . | . | . | . |
| 21-225_149A1_HC | . | . | . | . | . | . | . | . | . | . | . | . | . | . | . | . | . | . | . | . | . | . | . | . | . | . |
| 21-225_150F9_HC | . | . | . | . | . | . | . | . | . | . | . | . | . | . | . | . | . | . | . | . | V | . | . | . | . | . |
| 21-225_156H2_HC | . | . | . | . | . | . | N | . | . | . | . | . | . | . | . | . | . | . | . | . | . | . | . | . | . | . |
| 21-225_170E4_HC | . | . | . | . | . | . | . | . | . | . | . | . | . | S | . | . | . | . | . | . | . | . | . | . | . | . |
| 21-225_179D10_HC | . | . | . | . | . | . | . | . | . | . | . | . | . | S | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | H_CDR2 | | | | | | | | | | | | | | | | | | | | | |
| CONSENSUS | E | W | V | S | Y | I | S | R | S | - | - | - | S | N | T | K | Y | Y | A | D | S | V | K | G | R | F |
| 21-225_146A8_HC | . | . | . | . | . | . | . | . | . | . | . | . | . | . | . | . | . | . | . | . | . | . | R | . | . | . |
| 21-225_146B6_HC | . | . | . | . | . | . | . | . | . | . | . | . | . | . | . | . | H | . | . | . | . | . | . | . | . | . |
| 21-225_146D8_HC | . | . | . | . | . | . | . | . | . | . | . | . | . | . | . | . | . | . | V | . | . | . | . | . | . | . |
| 21-225_149A1_HC | . | . | . | . | . | . | . | . | . | . | . | . | . | . | . | . | H | . | . | . | . | . | . | . | . | . |
| 21-225_150F9_HC | . | . | . | . | . | . | . | . | . | . | . | . | . | . | . | . | H | . | . | . | . | . | . | . | . | . |
| 21-225_156H2_HC | . | . | . | . | . | . | . | . | . | . | . | . | . | . | . | . | . | . | . | . | . | . | . | . | . | . |
| 21-225_170E4_HC | . | . | . | . | . | . | . | S | . | . | . | . | G | S | . | . | . | . | . | . | . | . | E | . | . | . |
| 21-225_179D10_HC | . | . | . | A | . | . | . | S | . | . | . | . | G | S | . | T | . | . | . | . | . | . | Q | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | H_FR3 | | | | | | | | | | | | | | | | | | | | | | | | | |
| CONSENSUS | T | I | S | R | D | N | A | K | N | S | L | Y | L | Q | M | N | S | L | R | D | E | D | T | A | V | Y |
| 21-225_146A8_HC | . | . | . | . | . | . | . | . | . | . | . | . | . | . | . | . | . | . | . | . | . | . | . | . | . | . |
| 21-225_146B6_HC | . | . | . | . | . | . | . | . | . | . | . | . | . | . | . | D | . | . | . | . | . | . | . | . | . | . |
| 21-225_146D8_HC | . | . | . | . | . | . | . | . | . | . | . | . | . | . | . | . | . | . | . | . | . | . | . | . | . | . |
| 21-225_149A1_HC | . | . | . | . | . | . | . | . | . | . | . | . | . | . | . | . | . | . | . | . | . | . | . | . | . | . |
| 21-225_150F9_HC | . | . | . | . | . | . | . | . | . | . | . | . | . | . | . | . | . | . | . | . | . | . | . | . | . | . |
| 21-225_156H2_HC | . | . | . | . | . | . | . | . | . | . | . | . | . | . | . | . | . | . | . | . | . | . | . | . | . | . |
| 21-225_170E4_HC | . | . | . | . | . | . | . | . | . | . | . | D | . | . | . | . | . | . | . | . | . | . | . | . | . | . |
| 21-225_179D10_HC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | H_CDR3 | | | | | | | | | | | | | | | | | | | | | |
| CONSENSUS | Y | C | A | R | D | R | S | G | S | Y | G | Y | - | - | - | - | - | - | - | - | - | - | - | - | - | - |
| 21-225_146A8_HC | . | . | . | . | . | . | . | . | . | . | . | . | . | . | . | . | . | . | . | . | . | . | . | . | . | . |
| 21-225_146B6_HC | . | . | . | . | . | . | . | . | . | . | . | . | . | . | . | . | . | . | . | . | . | . | . | . | . | . |
| 21-225_146D8_HC | . | . | . | . | . | . | . | . | . | . | . | . | . | . | . | . | . | . | . | . | . | . | . | . | . | . |
| 21-225_149A1_HC | . | . | . | . | . | . | . | . | . | . | . | . | . | . | . | . | . | . | . | . | . | . | . | . | . | . |
| 21-225_150F9_HC | . | . | . | . | . | . | . | . | . | . | . | . | . | . | . | . | . | . | . | . | . | . | . | . | . | . |
| 21-225_156H2_HC | . | . | . | . | . | . | . | . | . | . | . | . | . | . | . | . | . | . | . | . | . | . | . | . | . | . |
| 21-225_170E4_HC | R | . | . | . | . | S | R | K | G | F | - | - | . | . | . | . | . | . | . | . | . | . | . | . | . | . |
| 21-225_179D10_HC | R | . | . | . | . | S | R | K | G | F | - | - | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | H_FR4 | | | | | | | | | | |
| CONSENSUS | F | Y | Y | Y | G | L | D | V | W | G | Q | G | T | T | V | T | V | S | S |
| 21-225_146A8_HC | . | . | . | . | . | . | . | . | . | . | . | . | . | . | . | . | . | . | . |
| 21-225_146B6_HC | . | . | . | . | . | . | . | . | . | . | . | . | . | . | . | . | . | . | . |
| 21-225_146D8_HC | . | . | . | . | . | . | . | . | . | . | . | . | . | . | . | . | . | . | . |
| 21-225_149A1_HC | . | . | . | . | . | . | . | . | . | . | . | . | . | . | . | . | . | . | . |
| 21-225_150F9_HC | . | . | . | . | . | . | . | . | . | . | . | . | . | . | . | . | . | . | . |
| 21-225_156H2_HC | . | . | . | . | . | M | . | . | . | . | . | . | . | . | . | . | . | . | . |
| 21-225_170E4_HC | - | - | . | . | . | . | . | . | . | . | . | . | . | . | . | . | . | . | . |
| 21-225_179D10_HC | - | - | . | . | . | . | . | . | . | . | . | . | I | . | . | . | . | . | . |

**FIGURE 57**

Table 94. Consensus 60 - VH4|4-30.1/D4|4-11|RF2/JH6 (SEQ ID NO: 50311):

QVQLQES-GPGLVKPSQTLSLTCTVSG-GSIRSG---GDYWSWIRQHPGKGLEWIGYIYY----SGSTYYNPSLKSRVTISVDTSKNQFSLKLSSVTAADTAVYYCARDSSSY----------------------GMDVWGQGTTVTVSS wherein:

S at position 67 can be substituted with I or P

S at position 110 can be substituted with G or H

S at position 111 can be substituted with A

S at position 112 can be substituted with L or R

Y at position 113 can be substituted with R or H

Ser Gly Gly Asp Tyr Trp Ser (SEQ ID NO: 50119)

Tyr Ile Tyr Tyr Ser Gly Xaa Thr Tyr Tyr Asn Pro Ser Leu Lys Ser (SEQ ID NO: 50120)

Asp Xaa Xaa Xaa Xaa Gly Met Asp Val (SEQ ID NO: 50121)

Ser Gly Gly Asp Tyr Trp Ser (SEQ ID NO: 50603)

Tyr Ile Tyr Tyr Ser Gly Ser Thr Tyr Tyr Asn Pro Ser Leu Lys Ser (SEQ ID NO: 50604)

Asp Ser Ser Ser Tyr Gly Met Asp Val (SEQ ID NO: 50605)

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | H_FR1 | | | | | | | | | | | | | | | | | | | | | | | | | |
| CONSENSUS | Q | V | Q | L | Q | E | S | - | G | P | G | L | V | K | P | S | Q | T | L | S | L | T | C | T | V | S |
| 21-225_190F10_HC | . | . | . | . | . | . | . | . | . | . | . | . | . | . | . | . | . | . | . | . | . | . | . | . | . | . |
| 21-225_191A2_HC | . | . | . | . | . | . | . | . | . | . | . | . | . | . | . | . | . | . | . | . | . | . | . | . | . | . |
| 21-225_192H8_HC | . | . | . | . | . | . | . | . | . | . | . | V | . | . | . | . | . | . | . | . | . | . | . | . | . | . |
| 21-225_194D12_HC | . | . | . | . | . | . | . | . | . | . | . | . | . | . | . | . | . | . | . | . | . | . | . | . | . | . |
| 21-225_194G5_HC | . | . | . | . | . | . | . | . | . | . | . | . | . | . | . | . | . | . | . | . | . | I | . | . | . | . |
| 21-225_198B8_HC | . | . | . | . | . | . | . | . | . | . | . | . | . | . | . | . | . | . | . | . | . | . | . | . | . | . |
| 21-225_212H12_HC | . | . | . | . | . | . | . | . | . | . | . | . | . | . | . | . | . | . | . | . | . | . | . | . | . | . |
| 21-225_214H3_HC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | H_CDR1 | | | | | | | | | | H_FR2 | | | | | | | | | |
| CONSENSUS | G | - | G | S | I | R | S | G | - | - | - | G | D | Y | W | S | W | I | R | Q | H | P | G | K | G | L |
| 21-225_190F10_HC | . | . | . | . | . | . | . | . | . | . | . | . | . | . | . | . | . | . | . | . | . | . | . | E | . | . |
| 21-225_191A2_HC | . | . | . | . | . | . | . | . | . | . | . | . | . | . | . | . | . | . | . | . | . | . | . | E | . | . |
| 21-225_192H8_HC | . | . | . | . | . | S | . | . | . | . | . | . | . | . | . | . | . | . | . | . | . | . | . | . | . | . |
| 21-225_194D12_HC | . | . | . | . | . | S | . | . | . | . | . | . | . | . | . | . | . | . | . | . | . | . | . | . | . | . |
| 21-225_194G5_HC | . | . | . | . | . | . | . | . | . | . | . | . | . | . | . | . | . | . | . | . | . | . | . | . | . | . |
| 21-225_198B8_HC | . | . | . | . | . | . | . | . | . | . | . | . | . | . | . | . | . | . | . | . | . | . | . | E | . | . |
| 21-225_212H12_HC | . | . | . | . | . | . | . | . | . | . | . | . | . | . | . | . | . | . | . | . | . | . | . | . | . | . |
| 21-225_214H3_HC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | | | H_CDR2 | | | | | | | | | | | |
| CONSENSUS | E | W | I | G | Y | I | Y | Y | - | - | - | - | S | G | S | T | Y | Y | N | P | S | L | K | S | R | V |
| 21-225_190F10_HC | . | . | . | . | . | . | . | . | . | . | . | . | . | . | I | . | . | . | . | . | . | . | . | . | . | . |
| 21-225_191A2_HC | . | . | . | . | . | . | . | . | . | . | . | . | . | . | I | . | . | . | . | . | . | . | . | . | . | . |
| 21-225_192H8_HC | . | . | . | . | . | . | . | . | . | . | . | . | . | . | P | . | . | . | . | . | . | . | . | . | . | L |
| 21-225_194D12_HC | . | . | . | . | . | . | . | . | . | . | . | . | . | . | . | . | . | . | . | . | . | . | . | . | . | . |
| 21-225_194G5_HC | . | . | . | . | . | . | . | . | . | . | . | . | . | . | . | . | . | . | . | . | . | . | . | . | . | . |
| 21-225_198B8_HC | . | . | . | . | . | . | . | . | . | . | . | . | . | . | I | . | . | . | . | . | . | . | . | . | . | . |
| 21-225_212H12_HC | . | . | . | . | . | . | . | . | . | . | . | . | . | . | . | . | . | . | . | . | . | . | . | . | . | . |
| 21-225_214H3_HC | . | . | . | . | . | . | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | | | | | H_FR3 | | | | | | | | | | | |
| CONSENSUS | T | I | S | V | D | T | S | K | N | Q | F | S | L | K | L | S | S | V | T | A | A | D | T | A | V | Y |
| 21-225_190F10_HC | . | . | . | . | . | . | . | . | . | . | . | . | . | . | . | . | . | . | . | . | . | . | . | . | . | . |
| 21-225_191A2_HC | . | . | . | . | . | . | . | . | . | . | . | . | . | . | . | . | . | . | . | . | . | . | . | . | . | . |
| 21-225_192H8_HC | . | M | . | A | . | . | . | . | . | . | . | . | . | . | . | . | . | . | . | . | . | . | . | . | . | . |
| 21-225_194D12_HC | . | . | . | . | . | . | . | . | . | . | . | . | . | . | . | . | . | . | . | . | . | . | . | . | . | . |
| 21-225_194G5_HC | . | . | . | . | . | . | . | . | . | . | . | . | . | . | . | . | . | . | . | . | . | . | . | . | . | . |
| 21-225_198B8_HC | . | . | . | . | . | . | . | . | . | . | . | . | . | . | . | . | . | . | . | . | . | . | . | . | . | . |
| 21-225_212H12_HC | . | . | . | G | . | . | . | . | . | . | . | . | . | . | . | . | . | . | . | . | . | . | . | . | . | . |
| 21-225_214H3_HC | . | . | . | G | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | H_CDR3 | | | | | | | |
| CONSENSUS | Y | C | A | R | D | S | S | S | Y | - | - | - | - | - | - | - | - | - | - | - | - | - | - | - | - | - |
| 21-225_190F10_HC | . | . | . | . | . | . | . | . | R | . | . | . | . | . | . | . | . | . | . | . | . | . | . | . | . | . |
| 21-225_191A2_HC | . | . | . | . | . | . | . | . | R | . | . | . | . | . | . | . | . | . | . | . | . | . | . | . | . | . |
| 21-225_192H8_HC | . | . | . | . | . | . | . | L | . | . | . | . | . | . | . | . | . | . | . | . | . | . | . | . | . | . |
| 21-225_194D12_HC | . | . | . | . | . | Q | A | R | H | . | . | . | . | . | . | . | . | . | . | . | . | . | . | . | . | . |
| 21-225_194G5_HC | . | . | . | . | . | H | . | L | . | . | . | . | . | . | . | . | . | . | . | . | . | . | . | . | . | . |
| 21-225_198B8_HC | . | . | . | . | . | . | . | . | R | . | . | . | . | . | . | . | . | . | . | . | . | . | . | . | . | . |
| 21-225_212H12_HC | . | . | . | . | . | . | . | . | . | . | . | . | . | . | . | . | . | . | . | . | . | . | . | . | . | . |
| 21-225_214H3_HC | . | . | . | . | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | H_FR4 | | | | | |
| CONSENSUS | - | - | - | - | G | M | D | V | W | G | Q | G | T | T | V | T | V | S | S |
| 21-225_190F10_HC | . | . | . | . | . | . | . | . | . | . | . | . | . | . | . | . | . | . | . |
| 21-225_191A2_HC | . | . | . | . | . | . | . | . | . | . | . | . | . | . | . | . | . | . | . |
| 21-225_192H8_HC | . | . | . | . | . | . | . | . | . | . | . | . | . | . | . | . | . | . | . |
| 21-225_194D12_HC | . | . | . | . | . | . | . | . | . | . | . | . | . | . | . | . | . | . | . |
| 21-225_194G5_HC | . | . | . | . | . | . | . | . | . | . | . | . | . | . | . | . | . | . | . |
| 21-225_198B8_HC | . | . | . | . | . | . | . | . | . | . | . | . | . | . | . | . | . | . | . |
| 21-225_212H12_HC | . | . | . | . | . | . | . | . | . | . | . | . | . | . | . | . | . | . | . |
| 21-225_214H3_HC | . | . | . | . | . | . | . | . | . | . | . | . | . | . | . | . | . | . | . |

**FIGURE 57**

Table 95. Consensus 15 -VK1|A30/JK4 (SEQ ID NO: 50312):

DIQMTQSPSSLSASVGDRVTITCRAS--QGIR------NDLGWYQQKPGKAPKRLIYA--------ASSLQSGVPSRFSGSGSG--TEFTLTISSLQPEDFATYYCLQHNS------------------------YPLTFGGGTKVEIKR wherein:

R at position 24 can be substituted with L

A at position 25 can be substituted with T

Q at position 29 can be substituted with R

G at position 30 can be substituted with D, A or V

I at position 31 can be substituted with M or V

R at position 32 can be substituted with E, K, N or S

N at position 39 can be substituted with S, D, T, K or I

D at position 40 can be substituted with N or A

L at position 41 can be substituted with F or V

G at position 42 can be substituted with D or N

A at position 58 can be substituted with T, S, V, G, D or R

A at position 67 can be substituted with T, V or E

S at position 68 can be substituted with F, C or Y

S at position 69 can be substituted with N, T, F, R or I

L at position 70 can be substituted with V, F or S

Q at position 71 can be substituted with H or E

S at position 72 can be substituted with R, N, T or G

**FIGURE 57**

L at position 107 can be substituted with V or I

Q at position 108 can be substituted with H

H at position 109 can be substituted with D, Y, N or R

N at position 110 can be substituted with S, Y, T, D, K, A, I, E, H, P or R

S at position 111 can be substituted with I, N, R, T, D, A, L or V

Y at position 135 can be substituted with F, H or S

P at position 136 can be substituted with A or M

L at position 137 can be substituted with P, F, N or V

T at position 138 can be substituted with K or I

Xaa Xaa Ser Xaa Xaa Xaa Xaa Xaa Xaa Xaa Xaa (SEQ ID NO: 50122)

Xaa Xaa Xaa Xaa Xaa Xaa Xaa (SEQ ID NO: 50123)

Xaa Xaa Xaa Xaa Xaa Xaa Xaa Xaa Xaa (SEQ ID NO: 50124)

Arg Ala Ser Gln Gly Ile Arg Asn Asp Leu Gly (SEQ ID NO: 50606)

Ala Ala Ser Ser Leu Gln Ser (SEQ ID NO: 50607)

Leu Gln His Asn Ser Tyr Pro Leu Thr (SEQ ID NO: 50608)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_10G5_LC | . | . | . | . | . | . | . | . | . | . | . | . | . | . | . | . | . | . | . | . | . | . | . | . | . | . |
| 21-225_10H12_LC | . | . | . | . | . | . | . | . | . | . | . | . | . | . | . | . | . | . | . | . | . | . | . | . | . | . |
| 21-225_11F10_LC | . | . | . | . | . | . | . | . | . | . | . | . | . | . | . | . | . | . | . | . | . | . | . | . | . | . |
| 21-225_11F5_LC | . | . | . | . | . | . | . | . | . | . | . | . | . | . | . | . | . | . | . | . | . | . | . | . | . | . |
| 21-225_11G7_LC | . | . | . | . | . | . | . | . | . | . | . | . | . | . | . | . | . | . | . | . | . | . | . | . | . | . |
| 21-225_12A1_LC | . | . | . | . | . | . | . | . | . | . | . | . | . | . | . | . | . | . | . | . | . | . | . | . | . | . |
| 21-225_13E6_LC | . | . | . | V | . | . | . | . | . | . | . | . | . | . | . | . | . | . | . | . | . | . | S | . | . | . |
| 21-225_13H3_LC | . | . | . | . | . | . | . | . | . | . | . | . | . | . | . | . | . | . | . | . | . | . | . | . | . | . |
| 21-225_13H5_LC | . | . | . | . | . | . | . | . | . | . | . | . | . | . | . | . | . | . | . | . | . | . | . | . | . | . |
| 21-225_146F1_LC | . | . | . | . | . | . | . | . | . | . | . | . | . | . | . | . | . | . | . | . | . | . | . | . | . | . |
| 21-225_146G11_LC | . | . | . | . | . | . | . | . | . | . | . | . | . | . | . | . | . | . | . | . | . | . | . | . | . | . |
| 21-225_146H9_LC | . | . | . | . | . | . | . | . | . | . | . | . | . | . | . | . | . | . | . | . | . | . | . | . | . | . |
| 21-225_148E11_LC | . | . | . | . | . | . | . | . | . | . | . | . | . | . | . | . | . | . | . | . | . | . | . | . | . | . |
| 21-225_148F12_LC | . | . | . | . | . | . | . | . | . | . | . | . | . | . | . | . | . | . | . | . | . | . | . | . | . | . |
| 21-225_149F12_LC | . | . | . | . | . | . | . | . | . | . | . | . | . | . | . | . | . | . | . | . | . | . | . | . | . | . |
| 21-225_149G1_LC | . | . | . | . | . | . | . | . | . | . | . | . | . | . | . | . | A | . | . | . | . | . | . | . | . | . |
| 21-225_149G5_LC | . | . | . | . | . | . | . | . | . | . | . | . | . | . | . | . | . | . | . | . | . | . | . | . | . | . |
| 21-225_14B1_LC1_LC | . | . | . | . | . | . | . | . | . | Y | . | . | . | . | . | . | . | . | . | . | . | . | . | . | . | . |
| 21-225_14B2_LC | . | . | . | . | . | . | . | . | . | . | . | . | . | . | . | . | . | . | . | . | . | . | . | . | . | . |
| 21-225_14C2_LC | . | . | . | . | I | . | . | . | . | . | . | F | . | C | . | . | . | . | . | I | . | . | . | . | . | . |
| 21-225_14E3_LC | . | . | . | . | . | . | . | . | . | . | . | . | . | . | . | . | . | . | . | . | . | . | . | . | . | . |
| 21-225_150E7_LC | . | . | . | . | . | . | . | . | . | . | . | . | . | . | . | . | . | . | . | . | . | . | . | . | . | . |
| 21-225_152H9_LC | . | . | . | . | . | . | . | . | . | . | . | . | . | . | . | . | . | . | . | . | . | . | . | . | . | . |
| 21-225_156G1_LC | . | . | . | . | . | . | . | . | . | . | . | . | . | . | . | . | . | . | . | . | . | . | . | . | . | . |
| 21-225_158H5_LC | . | . | . | . | I | . | . | . | . | F | . | F | . | . | . | . | . | . | . | . | . | . | . | . | . | . |
| 21-225_15A1_LC | . | . | . | . | . | . | . | . | . | . | . | . | . | . | . | . | . | . | . | . | . | . | . | . | . | . |
| 21-225_15C9_LC | . | . | . | . | . | . | . | . | . | . | . | . | . | . | . | . | . | . | . | . | . | . | . | . | . | . |
| 21-225_15E3_LC | . | . | . | . | . | . | . | . | . | . | . | . | . | . | . | . | . | . | . | . | . | . | . | . | . | . |
| 21-225_15G7_LC | . | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_15H1_LC | . | . | . | . | . | . | . | . | . | . | . | . | . | . | . | . | . | . | . | . | . | . | . | . | . | . |
| 21-225_15H10_LC | . | . | . | . | . | . | . | . | . | . | . | . | . | . | . | . | . | . | . | . | . | . | . | . | . | . |
| 21-225_15H8_LC | . | . | . | . | . | . | . | . | . | . | . | . | . | . | . | . | . | . | . | . | . | . | . | . | . | . |
| 21-225_160B10_LC | . | . | . | . | . | . | . | . | . | . | P | . | . | . | . | . | . | . | . | . | . | . | . | . | . | . |
| 21-225_160H3_LC | . | . | . | . | . | . | . | . | . | . | . | . | . | . | . | . | . | . | . | . | . | . | . | . | . | . |
| 21-225_169B1_LC | . | . | . | . | . | . | . | . | . | . | . | . | . | . | . | . | . | . | . | . | . | . | . | . | . | . |
| 21-225_169F9_LC | . | . | . | . | . | . | . | . | . | . | . | . | . | . | . | . | . | . | . | . | . | . | . | . | . | . |
| 21-225_16A1_LC | . | . | . | . | . | . | . | . | . | . | . | . | . | . | . | . | . | . | . | . | . | . | . | . | . | . |
| 21-225_16B7_LC | . | . | . | . | . | . | . | . | . | . | . | . | . | . | . | . | . | . | . | . | . | . | . | . | . | . |
| 21-225_16E12_LC | . | . | . | . | . | . | . | . | . | . | . | . | . | . | . | . | . | . | . | . | . | . | . | . | . | . |
| 21-225_16F11_LC | . | . | . | . | . | . | . | . | . | . | . | . | . | . | . | . | . | . | . | . | . | . | . | . | . | . |
| 21-225_16F4_LC | . | . | . | . | I | . | . | . | . | . | P | . | . | . | . | . | . | . | . | . | . | . | . | . | . | . |
| 21-225_170D11_LC | . | . | . | . | . | . | . | . | . | . | . | . | . | . | . | . | . | . | . | . | . | . | . | . | . | . |
| 21-225_170D5_LC | . | . | . | . | . | . | . | . | . | . | . | . | . | . | . | . | . | . | . | . | . | . | . | . | . | . |
| 21-225_170G4_LC | . | . | . | . | . | . | . | . | . | . | . | . | . | . | . | . | . | . | . | . | . | . | . | . | . | . |
| 21-225_170G5_LC | . | . | . | . | . | . | . | . | . | . | . | . | . | . | . | . | . | . | . | . | . | . | . | . | . | . |
| 21-225_171A4_LC | . | . | . | . | . | . | . | . | . | . | . | . | . | . | . | . | . | . | . | . | . | . | . | . | . | . |
| 21-225_171C3_LC | . | . | . | . | . | . | . | . | . | . | . | . | . | . | . | . | . | . | . | . | . | . | . | . | . | . |
| 21-225_172B3_LC | . | . | . | . | . | . | . | . | . | . | . | . | . | . | . | . | . | . | . | . | . | . | . | . | . | . |
| 21-225_172G8_LC | . | . | . | . | . | . | . | . | . | . | . | . | . | . | . | . | . | . | . | . | . | . | . | . | . | . |
| 21-225_173H12_LC | . | . | . | . | . | . | . | . | . | . | . | . | . | . | . | . | . | . | . | . | . | . | . | . | . | . |
| 21-225_175G1_LC | . | . | . | . | . | . | . | . | . | . | . | . | . | . | . | . | . | . | . | . | . | . | . | . | . | . |
| 21-225_176B11_LC | . | . | . | . | . | . | . | . | . | . | . | . | . | . | . | . | . | . | . | . | . | . | . | . | . | . |
| 21-225_178B10_LC | . | . | . | . | . | . | . | . | . | . | . | . | . | . | . | . | . | . | . | . | . | . | . | . | . | . |
| 21-225_17A10_LC | . | . | . | . | . | . | . | . | . | . | . | . | . | . | . | . | . | . | . | . | . | . | . | . | . | . |
| 21-225_17B5_LC | . | . | . | . | . | . | . | . | . | . | . | . | . | . | . | . | . | . | . | . | . | . | . | . | . | . |
| 21-225_17F9_LC | . | . | . | . | . | . | . | . | . | . | . | . | . | . | . | . | . | . | . | . | . | . | . | . | . | . |
| 21-225_17H12_LC | . | . | . | . | . | . | . | . | . | . | . | . | . | . | . | . | . | . | . | . | . | . | . | . | . | . |
| 21-225_17H8_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_18A1_LC | . | . | . | . | . | . | . | . | . | . | . | . | . | . | . | . | . | . | . | . | . | . | . | . | . | . |
| 21-225_18A3_LC | . | . | . | . | . | . | . | . | . | . | . | . | . | . | . | . | . | . | . | . | . | . | . | . | . | . |
| 21-225_18C6_LC | . | . | . | . | . | . | . | . | . | . | . | . | . | . | . | . | . | . | . | . | . | . | . | . | T | . |
| 21-225_18E8_LC | . | . | . | . | . | . | A | . | . | . | . | . | . | . | . | . | . | . | . | . | . | . | . | . | . | . |
| 21-225_190C8_LC | . | . | . | . | . | . | . | . | . | . | . | . | . | . | . | . | . | . | . | . | . | . | . | . | . | . |
| 21-225_193A5_LC | . | . | . | . | . | . | . | . | . | . | . | P | . | . | . | . | . | . | . | I | . | S | . | . | . | . |
| 21-225_193E7_LC | . | . | . | . | . | . | . | . | . | . | . | . | . | . | . | . | . | . | . | . | . | . | . | . | . | . |
| 21-225_196C10_LC | . | . | . | . | . | . | . | . | . | . | . | . | . | . | . | . | . | . | . | . | . | . | . | . | . | . |
| 21-225_199E3_LC | . | . | . | . | . | . | . | . | . | . | . | . | . | . | . | . | . | . | . | . | . | . | . | . | . | . |
| 21-225_19D11_LC | . | . | . | . | . | . | . | . | . | . | . | . | . | . | . | . | . | . | . | . | . | . | . | . | . | . |
| 21-225_19E1_LC | . | . | . | . | . | . | . | . | . | . | . | . | . | . | . | . | . | . | . | . | . | . | . | . | . | . |
| 21-225_19F3_LC | . | . | . | . | . | . | . | . | . | . | . | . | . | . | . | R | . | . | . | . | . | . | . | . | . | . |
| 21-225_1A2_LC | . | . | . | . | . | . | . | . | . | . | . | . | . | . | . | . | . | . | . | . | . | . | . | . | . | . |
| 21-225_1C12_LC | . | . | . | . | . | . | . | . | . | . | . | . | . | . | . | . | . | . | . | . | . | . | . | . | . | . |
| 21-225_1F2_LC | . | . | . | . | . | . | . | . | . | . | . | . | . | . | . | . | . | . | . | . | . | . | . | . | . | . |
| 21-225_200F12_LC | . | . | . | . | . | . | . | . | . | . | . | . | . | . | . | . | . | . | . | . | . | . | . | . | . | . |
| 21-225_200H9_LC | . | . | . | . | . | . | . | . | . | . | . | . | . | . | . | . | . | . | . | . | . | . | . | . | . | . |
| 21-225_201A1_LC | . | . | . | . | . | . | . | . | . | . | . | . | . | . | . | . | . | . | . | . | . | . | . | . | . | . |
| 21-225_201A10_LC | . | . | . | . | . | . | . | . | . | . | . | . | . | . | . | . | . | . | . | . | . | . | . | . | . | . |
| 21-225_204H8_LC | . | . | . | . | . | . | . | . | . | . | . | . | . | . | . | . | . | . | . | . | . | . | . | . | . | . |
| 21-225_205H8_LC | . | . | . | . | . | . | . | . | . | . | . | . | . | . | . | . | . | . | . | . | . | . | . | . | . | . |
| 21-225_20A10_LC | . | . | . | . | . | . | . | . | . | . | . | . | . | . | . | . | . | . | . | . | . | . | . | . | . | . |
| 21-225_20A7_LC | . | . | . | . | . | . | . | . | . | . | . | . | . | . | . | . | . | . | . | . | . | . | . | . | . | . |
| 21-225_20C2_LC | . | . | . | . | . | . | . | . | . | . | . | . | . | . | . | Q | . | . | . | . | . | . | . | . | . | . |
| 21-225_20C8_LC | . | . | . | . | . | . | . | . | . | . | . | . | . | . | . | . | . | . | . | . | . | . | . | . | . | . |
| 21-225_20D5_LC | . | . | . | . | . | . | . | . | . | . | . | . | . | . | . | . | . | . | . | . | . | . | . | . | . | . |
| 21-225_20E12_LC | . | . | . | . | . | . | . | . | . | . | . | . | . | . | . | . | . | . | . | . | . | . | . | . | . | . |
| 21-225_20E5_LC | . | . | . | . | . | . | . | . | . | . | . | . | . | . | . | . | . | . | . | . | . | . | . | . | . | . |
| 21-225_20G2_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_20G9_LC | . | . | . | . | . | . | . | . | . | . | . | . | . | . | . | . | . | . | . | . | . | . | . | . | . | . |
| 21-225_20H10_LC | G | . | . | . | . | . | . | . | . | . | . | . | . | . | . | . | . | . | . | . | . | . | . | . | . | . |
| 21-225_20H12_LC | . | . | . | . | . | . | . | . | . | . | . | . | . | . | . | . | . | . | . | . | . | . | . | . | . | . |
| 21-225_21E5_LC | . | . | . | . | . | . | . | . | . | . | . | . | . | . | . | . | . | . | . | . | . | . | . | . | . | . |
| 21-225_21F3_LC | . | . | . | . | . | . | . | . | . | . | . | . | . | . | . | . | . | . | . | . | . | . | . | . | . | . |
| 21-225_21G7_LC | . | . | . | . | . | . | . | . | . | . | . | . | . | . | . | . | . | . | . | . | . | . | . | . | . | . |
| 21-225_21H3_LC | . | . | . | . | . | . | . | . | . | . | . | . | . | . | . | . | . | . | . | . | . | . | . | . | . | . |
| 21-225_224A1_LC | . | . | . | . | . | . | . | . | . | . | . | . | . | . | . | . | . | . | . | . | . | . | . | . | . | . |
| 21-225_224B1_LC | . | . | . | . | . | . | . | . | . | . | . | . | . | . | . | . | . | . | . | . | . | . | . | . | . | . |
| 21-225_224C3_LC | . | . | . | . | . | . | . | . | . | . | . | . | . | . | . | . | . | . | . | . | . | . | . | . | . | . |
| 21-225_224D10_LC | . | . | L | . | . | . | . | . | . | . | . | . | . | . | . | . | . | . | . | . | . | . | . | . | . | . |
| 21-225_22B7_LC | . | . | . | . | . | . | . | . | . | . | . | . | . | . | . | . | . | . | . | . | . | . | . | . | . | . |
| 21-225_22C1_LC | . | . | . | . | . | . | . | . | . | . | . | . | . | . | . | . | . | . | . | . | . | . | . | . | T | . |
| 21-225_22D12_LC | . | . | . | . | . | . | . | . | . | . | . | . | . | . | . | . | . | . | . | . | . | . | . | . | . | . |
| 21-225_22F2_LC | . | . | . | . | . | . | . | . | . | . | . | . | . | . | . | . | . | . | . | . | . | . | . | . | . | . |
| 21-225_22F9_LC | . | . | . | . | . | . | . | . | . | . | . | . | . | . | . | . | . | . | . | . | . | . | . | . | . | . |
| 21-225_22G8_LC | . | . | . | . | I | . | . | . | . | . | . | F | . | . | . | . | . | . | . | . | . | . | . | . | T | . |
| 21-225_22G9_LC | . | . | . | . | . | . | . | . | . | . | . | . | . | . | . | . | . | . | . | . | . | . | . | . | . | . |
| 21-225_22H4_LC | . | . | . | . | . | . | . | . | . | . | . | . | . | . | . | . | . | . | . | . | . | . | . | . | . | . |
| 21-225_23A3_LC | . | . | . | . | . | . | . | . | . | . | . | . | . | . | . | . | . | . | . | . | . | . | . | . | . | . |
| 21-225_23C8_LC | . | . | . | . | . | . | . | . | . | . | R | . | . | . | . | . | . | . | . | . | . | . | . | . | . | . |
| 21-225_23D1_LC | . | . | . | . | . | . | . | . | . | . | . | . | . | . | . | . | . | . | . | . | . | . | . | . | . | . |
| 21-225_23H11_LC | . | . | . | . | . | . | . | . | . | . | . | . | . | . | . | . | . | . | . | . | . | . | . | . | . | . |
| 21-225_24E5_LC | . | . | . | . | . | . | . | . | . | . | . | . | . | . | . | . | . | . | . | . | . | . | . | . | . | . |
| 21-225_25E6_LC | . | . | . | . | . | . | . | . | . | . | . | . | . | . | . | . | . | . | . | . | . | . | . | . | . | . |
| 21-225_25H10_LC | . | . | . | . | . | . | . | . | . | . | . | . | . | . | . | . | . | . | . | . | . | . | . | . | . | . |
| 21-225_25H11_LC | . | . | . | . | . | . | . | . | . | . | . | . | . | . | . | . | . | . | . | . | . | . | . | . | . | . |
| 21-225_27C3_LC | . | . | . | . | . | . | . | . | . | . | . | . | . | . | . | . | . | . | . | . | . | . | . | . | . | . |
| 21-225_27F2_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_29B8_LC | . | . | . | . | . | . | . | . | . | . | . | . | . | . | . | . | . | . | . | . | . | . | . | . | . | . |
| 21-225_29G4_LC | . | . | . | . | . | . | . | . | . | . | . | . | . | . | . | . | . | . | . | . | . | . | . | . | . | . |
| 21-225_29G8_LC | . | . | . | . | . | . | . | . | . | . | . | . | . | . | . | . | . | . | . | . | . | . | . | . | . | . |
| 21-225_29H6_LC | . | . | . | . | . | . | . | . | . | . | . | . | . | . | . | . | . | . | . | . | . | . | . | . | . | . |
| 21-225_2A11_LC | . | . | . | . | . | . | . | . | . | . | . | . | P | . | . | . | . | . | . | . | . | . | . | . | . | . |
| 21-225_2B2_LC | . | . | . | . | . | . | . | . | . | . | . | . | . | . | . | . | . | . | . | . | . | . | . | . | . | . |
| 21-225_2G9_LC | . | . | . | . | . | . | . | . | . | . | . | F | . | . | . | . | . | . | . | . | . | . | . | . | . | . |
| 21-225_30D1_LC | . | . | . | . | . | . | . | . | . | . | . | . | . | . | . | . | . | . | . | . | . | . | . | . | . | . |
| 21-225_30E3_LC | . | . | . | . | . | . | . | . | . | . | . | . | . | . | . | . | . | . | . | . | . | . | . | . | . | . |
| 21-225_30E9_LC | . | . | . | . | . | . | . | . | . | . | . | . | . | . | . | . | . | . | . | . | . | . | . | . | . | . |
| 21-225_30F3_LC | . | . | . | . | . | . | . | . | . | . | . | . | . | . | . | . | . | . | . | . | . | . | . | . | . | . |
| 21-225_30G11_LC | . | . | . | . | . | . | . | . | . | . | . | . | . | . | . | . | . | . | . | . | . | . | . | . | . | . |
| 21-225_31A8_LC | . | . | . | . | . | . | . | . | . | . | . | . | . | . | . | . | . | . | . | . | . | . | . | . | . | . |
| 21-225_31B8_LC | . | . | . | . | I | . | . | . | . | . | . | . | . | . | . | . | . | . | . | . | . | . | . | . | . | . |
| 21-225_31H3_LC | . | . | . | . | . | . | . | . | . | . | . | . | . | . | . | . | . | . | . | . | . | . | . | . | . | . |
| 21-225_31H5_LC | . | . | . | . | . | . | . | . | . | . | . | . | . | . | . | . | . | . | F | . | . | S | F | . | . | . |
| 21-225_32G1_LC | . | . | . | . | . | . | . | . | . | . | . | . | . | . | . | . | . | . | . | . | . | . | . | . | . | . |
| 21-225_32G12_LC | . | . | . | . | . | . | . | . | . | . | . | . | . | . | . | . | . | . | . | . | . | . | . | . | . | . |
| 21-225_33A4_LC | . | . | . | . | . | . | . | . | . | . | . | . | . | . | . | . | . | . | . | . | . | . | . | . | . | . |
| 21-225_33A7_LC | . | . | . | . | . | . | . | . | . | . | . | . | . | . | . | . | . | . | . | . | . | . | . | . | . | . |
| 21-225_33B1_LC | . | . | . | . | . | . | . | . | . | . | . | . | . | . | . | . | . | . | . | . | . | . | . | . | T | . |
| 21-225_33B8_LC | . | . | . | . | . | . | . | . | . | . | . | . | . | . | . | . | . | . | . | . | . | . | . | . | . | . |
| 21-225_33F1_LC | . | . | . | . | . | . | . | . | . | . | . | . | . | . | . | . | . | . | . | . | . | . | . | . | T | . |
| 21-225_33G7_LC | . | . | . | . | . | . | . | . | . | . | . | . | . | . | . | . | . | . | . | . | . | . | . | . | . | . |
| 21-225_34A6_LC | . | . | . | . | . | . | . | . | . | . | . | . | . | . | . | . | . | . | . | . | . | . | . | . | T | . |
| 21-225_34D11_LC | . | . | . | . | . | . | . | . | . | . | . | . | . | . | . | . | . | . | . | . | . | . | . | . | . | . |
| 21-225_34G8_LC | . | . | . | . | . | . | . | . | . | . | . | . | . | . | . | . | . | . | . | . | . | . | . | . | . | . |
| 21-225_34G9_LC | . | . | . | . | . | . | . | . | . | . | . | . | . | . | . | . | . | . | . | . | . | . | . | . | . | . |
| 21-225_34H11_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_35A6_LC | . | . | . | . | . | . | . | . | . | . | . | . | . | . | . | . | . | . | . | . | . | . | . | . | . | . |
| 21-225_35B7_LC | . | . | . | . | . | . | . | . | . | . | . | . | . | . | . | . | . | . | I | . | . | . | . | . | . | . |
| 21-225_35D1_LC | . | . | . | . | . | . | . | . | . | . | . | . | . | . | . | . | . | . | . | . | . | . | . | . | . | . |
| 21-225_35F11_LC | . | . | . | . | . | . | . | . | . | . | . | . | . | . | . | . | . | . | . | . | . | . | . | . | . | . |
| 21-225_36A5_LC | . | . | . | . | . | . | . | . | . | . | . | . | . | . | . | . | . | . | . | . | . | . | . | . | T | . |
| 21-225_36C5_LC | . | . | . | . | . | . | . | . | . | . | . | . | . | . | . | . | . | . | . | . | . | . | . | . | . | . |
| 21-225_43C3_LC | . | . | . | . | . | . | . | . | . | . | . | . | . | . | . | . | . | . | . | . | . | . | . | . | . | . |
| 21-225_44C3_LC | . | . | . | . | . | . | . | . | . | . | . | . | . | . | . | . | . | . | . | . | . | . | . | . | . | . |
| 21-225_44E12_LC | . | . | . | . | . | . | . | . | . | . | . | . | . | . | . | . | . | . | . | . | . | . | . | . | T | . |
| 21-225_46B1_LC | . | . | . | . | . | . | . | . | . | . | . | . | . | . | . | . | . | . | . | . | . | . | . | . | . | . |
| 21-225_46C6_LC | . | . | . | . | . | . | . | . | . | . | . | . | . | . | . | . | . | . | . | . | . | . | . | . | . | . |
| 21-225_46D8_LC | . | . | . | . | . | . | . | . | . | . | . | . | . | . | . | . | . | . | . | . | . | . | . | . | . | . |
| 21-225_46F3_LC | . | . | . | . | . | . | . | . | . | . | . | . | . | . | . | . | . | . | . | . | . | . | . | . | . | . |
| 21-225_47A1_LC | . | . | . | . | . | . | . | . | . | . | . | . | . | . | . | . | . | . | . | . | . | . | . | . | . | . |
| 21-225_47A5_LC | . | . | . | . | . | . | . | . | . | . | . | . | . | . | . | . | . | . | . | . | . | . | . | . | . | . |
| 21-225_48D12_LC | . | . | . | . | . | . | . | . | . | . | . | . | . | . | . | . | . | . | . | . | . | . | . | . | . | . |
| 21-225_48H4_LC | . | . | . | . | . | . | F | . | . | . | . | . | . | . | . | . | . | . | . | . | . | . | . | . | . | . |
| 21-225_49A1_LC | . | . | . | . | . | . | . | . | . | . | . | . | . | . | . | . | . | . | . | . | . | . | . | . | . | . |
| 21-225_49C6_LC | . | . | . | . | . | . | . | . | . | . | . | . | . | . | . | . | . | . | . | . | . | . | . | . | . | . |
| 21-225_4C8_LC | . | . | . | . | I | . | . | . | . | F | . | F | . | C | . | . | . | . | . | . | . | . | . | . | . | . |
| 21-225_4E10_LC | . | . | . | . | . | . | . | . | . | . | . | F | . | . | . | . | . | . | . | . | . | . | . | . | . | . |
| 21-225_4F12_LC | . | . | . | . | . | . | . | . | . | . | . | . | . | . | . | . | . | . | . | . | . | . | . | . | . | . |
| 21-225_4F4_LC | . | . | . | . | . | . | . | . | . | . | . | . | . | . | . | . | . | . | . | . | . | . | . | . | . | . |
| 21-225_4H4_LC | . | . | . | . | . | . | . | . | . | . | . | . | . | . | . | . | . | . | . | . | . | . | . | . | . | . |
| 21-225_50G10_LC | . | . | . | . | . | . | . | . | . | . | . | . | . | . | . | . | . | . | . | . | . | . | . | . | . | . |
| 21-225_51F11_LC | . | . | . | . | . | . | . | . | . | A | . | . | . | . | . | . | . | . | . | . | . | . | . | . | . | . |
| 21-225_52B2_LC | . | . | . | . | . | . | . | . | . | . | . | . | . | . | . | . | . | . | . | . | . | . | . | . | . | . |
| 21-225_53D2_LC | . | . | . | . | . | . | . | . | . | . | . | . | . | . | . | . | . | . | . | . | . | . | . | . | . | . |
| 21-225_53E6_LC | . | . | . | . | . | . | . | . | . | . | . | . | . | . | . | . | A | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_54C6_LC | . | . | . | . | . | . | . | . | . | . | . | . | . | . | . | . | A | . | . | . | . | . | . | . | . | . |
| 21-225_55B8_LC | . | . | . | . | . | . | . | . | . | . | . | . | S | . | . | . | . | . | . | . | . | . | . | . | . | . |
| 21-225_55H6_LC | . | . | . | . | . | . | . | . | . | . | . | . | . | . | . | . | . | . | . | . | . | . | . | . | . | . |
| 21-225_56F1_LC | . | . | . | . | . | . | . | . | . | . | . | . | . | . | . | . | . | . | . | . | . | . | . | . | . | . |
| 21-225_59E6_LC | . | . | . | . | . | . | . | . | . | . | . | . | . | . | . | . | . | . | . | . | . | . | . | . | . | . |
| 21-225_5H2_LC | . | . | . | . | . | . | . | . | . | . | . | . | . | . | . | T | . | . | . | . | . | . | . | . | . | . |
| 21-225_5H7_LC | . | . | . | . | . | . | . | . | . | . | . | . | . | . | . | . | . | . | . | . | . | . | . | . | . | . |
| 21-225_60E8_LC | . | . | . | . | . | . | . | . | . | . | . | . | . | . | . | . | . | . | . | . | F | . | . | . | . | . |
| 21-225_62E2_LC | . | . | . | . | . | . | . | . | . | . | . | . | . | . | . | . | . | . | . | . | . | . | . | . | . | . |
| 21-225_62E4_LC | . | . | . | . | . | . | . | . | . | . | . | . | . | . | . | . | . | . | . | . | . | . | . | . | . | . |
| 21-225_64E1_LC | . | . | . | . | . | . | . | . | . | . | . | . | . | . | . | . | . | . | . | . | . | . | . | . | . | . |
| 21-225_68D12_LC | . | . | . | . | . | . | . | . | . | . | . | . | . | . | . | . | . | . | . | . | . | . | . | . | . | . |
| 21-225_68F11_LC | . | . | . | . | . | . | . | . | . | . | . | . | . | . | . | . | . | . | . | . | . | . | . | . | . | . |
| 21-225_6A6_LC | . | . | . | . | . | . | . | . | . | . | . | . | . | . | . | . | . | . | . | . | . | . | . | . | . | . |
| 21-225_6C12_LC | . | . | . | . | . | . | . | . | . | . | . | . | . | . | . | . | . | . | G | . | . | . | . | . | . | . |
| 21-225_6D4_LC | . | . | . | . | . | . | . | . | . | . | . | . | . | . | . | . | . | . | . | . | . | . | . | . | . | . |
| 21-225_6G12_LC | . | . | . | . | . | . | . | . | . | . | . | . | . | . | . | . | . | . | . | . | . | . | . | . | . | . |
| 21-225_70E12_LC | . | . | . | . | . | . | . | . | . | . | . | . | . | . | . | . | . | . | . | . | . | . | . | . | . | . |
| 21-225_70E8_LC | . | . | . | . | . | . | . | . | . | . | . | . | T | . | . | . | . | . | . | . | . | . | . | . | . | . |
| 21-225_73G6_LC | . | . | . | . | . | . | . | . | . | . | . | . | . | . | . | . | . | . | . | . | . | . | . | . | . | . |
| 21-225_74A1_LC | . | . | . | . | . | . | . | . | . | . | . | . | . | . | . | . | . | . | . | . | . | . | . | . | . | . |
| 21-225_74D7_LC | . | . | . | . | . | . | . | . | . | . | . | . | . | . | . | . | . | . | . | . | . | . | . | . | . | . |
| 21-225_74E4_LC | . | . | . | . | . | . | . | . | . | . | . | . | . | . | . | . | . | . | . | . | . | . | . | . | . | . |
| 21-225_75H7_LC | . | . | . | . | . | . | . | . | . | . | [illegible] | Y | . | . | . | . | . | . | . | . | . | N | S | . | . | . |
| 21-225_7C4_LC | . | . | . | . | . | . | . | . | . | . | . | F | . | . | . | . | . | . | . | . | . | . | . | . | . | . |
| 21-225_7E9_LC | . | . | . | . | . | . | . | . | . | . | . | F | . | . | . | . | . | . | . | . | . | . | . | . | . | . |
| 21-225_7F4_LC | . | . | . | . | . | . | . | . | . | . | . | . | . | . | . | . | . | . | . | . | . | . | . | . | T | . |
| 21-225_7H11_LC | . | . | . | . | . | . | . | . | . | . | A | . | . | . | . | . | V | . | . | . | . | I | . | L | . | . |
| 21-225_7H7_LC | . | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_8C9_LC | . | . | . | . | . | . | . | . | . | . | . | . | . | . | I | . | . | . | . | . | . | . | . | . | . | . |
| 21-225_8D12_LC | . | . | . | . | . | . | . | . | . | . | . | . | . | . | . | . | . | . | . | . | . | . | . | . | . | . |
| 21-225_8F11_LC | . | . | . | . | . | . | . | . | . | . | . | . | . | . | . | . | . | . | . | . | . | . | . | . | . | . |
| 21-225_8H7_LC | . | . | . | . | . | . | . | . | . | . | . | . | . | . | . | . | . | . | . | . | . | . | . | . | . | . |
| 21-225_96A3_LC | . | . | . | . | . | . | . | . | . | . | . | . | . | . | . | . | . | . | . | . | . | . | . | . | . | . |
| 21-225_9E8_LC | . | . | . | . | . | . | . | . | . | . | . | P | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_10G5_LC | . | . | . | . | . | . | . | . | . | . | . | . | . | . | . | . | . | . | . | . | . | . | . | . | . | . |
| 21-225_10H12_LC | . | . | . | . | . | . | . | . | . | . | . | . | . | . | . | . | . | . | . | . | . | . | . | . | . | . |
| 21-225_11F10_LC | . | . | . | . | . | . | . | . | . | . | . | . | . | . | . | . | . | . | . | . | . | . | . | . | . | . |
| 21-225_11F5_LC | . | . | . | . | . | . | . | . | . | . | . | . | . | . | . | . | . | . | . | . | . | . | . | . | . | . |
| 21-225_11G7_LC | . | . | . | . | . | . | . | . | . | . | . | . | . | . | . | . | . | . | . | . | . | . | . | . | . | . |
| 21-225_12A1_LC | . | . | . | . | . | . | . | . | . | . | . | . | . | . | . | . | . | . | . | . | . | . | . | . | . | . |
| 21-225_13E6_LC | . | . | . | . | . | . | . | . | . | . | . | . | . | . | . | . | . | . | . | . | . | . | . | . | . | . |
| 21-225_13H3_LC | . | . | . | . | . | . | . | . | . | . | . | . | . | . | . | . | . | . | . | . | . | . | . | . | . | . |
| 21-225_13H5_LC | . | . | . | . | . | . | . | . | . | . | . | . | . | . | . | . | . | . | . | . | . | . | . | . | . | . |
| 21-225_146F1_LC | . | . | . | . | . | . | . | . | . | . | . | . | . | . | . | . | . | . | . | . | . | . | . | . | . | . |
| 21-225_146G11_LC | . | . | . | D | . | . | . | . | . | . | . | . | . | N | F | . | . | . | . | . | . | . | . | . | . | . |
| 21-225_146H9_LC | . | . | . | . | . | . | . | . | . | . | . | . | . | . | . | . | . | . | . | . | . | . | . | . | . | . |
| 21-225_148E11_LC | . | . | . | . | . | . | . | . | . | . | . | . | . | . | . | . | . | . | . | . | . | . | . | . | . | . |
| 21-225_148F12_LC | . | . | . | . | . | . | . | . | . | . | . | . | . | A | . | . | . | . | . | . | . | . | . | . | . | . |
| 21-225_149F12_LC | . | . | . | . | . | . | . | . | . | . | . | . | . | . | . | . | . | . | . | . | . | . | . | . | . | . |
| 21-225_149G1_LC | . | . | . | . | . | . | . | . | . | . | . | . | . | . | . | . | . | . | . | . | . | . | . | . | . | . |
| 21-225_149G5_LC | . | . | . | . | . | . | . | . | . | . | . | . | . | A | . | . | . | F | . | . | . | . | . | . | . | . |
| 21-225_14B1_LC1_LC | . | . | . | D | . | . | . | . | . | . | . | . | D | N | . | . | . | . | . | . | . | . | . | . | . | . |
| 21-225_14B2_LC | . | . | . | . | . | . | . | . | . | . | . | . | . | . | . | . | . | . | . | . | . | . | . | . | . | . |
| 21-225_14C2_LC | . | . | . | . | . | . | . | . | . | . | . | . | . | . | . | D | . | . | . | . | . | . | . | . | . | . |
| 21-225_14E3_LC | . | . | . | . | . | . | . | . | . | . | . | . | . | . | . | . | . | . | . | . | . | . | . | . | . | . |
| 21-225_150E7_LC | . | . | . | . | . | . | . | . | . | . | . | . | . | . | . | . | . | . | . | . | . | . | . | . | . | . |
| 21-225_152H9_LC | . | . | . | . | . | . | . | . | . | . | . | . | . | . | . | . | . | . | . | . | . | . | . | . | . | . |
| 21-225_156G1_LC | . | . | . | . | . | . | . | . | . | . | . | . | . | . | . | . | . | . | . | . | . | . | . | . | . | . |
| 21-225_158H5_LC | . | . | . | . | M | . | . | . | . | . | . | . | I | . | . | . | . | . | . | . | . | . | . | . | . | . |
| 21-225_15A1_LC | . | . | . | . | . | . | . | . | . | . | . | . | . | . | . | . | . | . | . | . | . | . | . | . | . | . |
| 21-225_15C9_LC | . | . | . | . | . | . | . | . | . | . | . | . | . | . | . | . | . | . | . | . | . | . | . | . | . | . |
| 21-225_15E3_LC | . | . | . | . | . | . | . | . | . | . | . | . | . | . | . | . | . | . | . | . | . | . | . | . | . | . |
| 21-225_15G7_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_15H1_LC | . | . | . | . | . | . | . | . | . | . | . | . | . | . | . | . | . | . | . | . | . | . | . | . | . | . |
| 21-225_15H10_LC | . | . | . | . | . | . | . | . | . | . | . | . | . | . | . | . | . | . | . | . | . | . | . | . | . | . |
| 21-225_15H8_LC | . | . | . | . | . | . | . | . | . | . | . | . | . | . | . | . | . | . | . | . | . | . | . | . | . | . |
| 21-225_160B10_LC | . | . | . | . | . | . | . | . | . | . | . | . | . | . | . | . | . | . | . | . | . | . | . | T | . | . |
| 21-225_160H3_LC | . | . | . | . | . | . | . | . | . | . | . | . | . | . | . | . | . | . | . | . | . | . | . | . | . | . |
| 21-225_169B1_LC | . | . | . | . | . | . | . | . | . | . | . | . | . | . | . | . | . | . | . | . | . | . | . | . | . | . |
| 21-225_169F9_LC | . | . | . | . | . | . | . | . | . | . | . | . | . | . | . | . | . | . | . | . | . | . | . | . | . | . |
| 21-225_16A1_LC | . | . | . | . | . | . | . | . | . | . | . | . | . | . | . | . | . | . | . | . | . | . | . | . | . | . |
| 21-225_16B7_LC | . | . | . | . | . | . | . | . | . | . | . | . | . | . | . | . | . | . | . | . | . | . | . | . | . | . |
| 21-225_16E12_LC | . | . | . | . | . | . | . | . | . | . | . | . | . | . | . | . | . | . | . | . | . | . | . | . | . | . |
| 21-225_16F11_LC | . | . | . | . | . | . | . | . | . | . | . | . | . | . | . | . | . | . | . | . | . | . | . | . | . | . |
| 21-225_16F4_LC | . | . | . | . | . | . | . | . | . | . | . | . | . | . | . | . | . | . | . | . | . | S | . | . | . | . |
| 21-225_170D11_LC | . | . | . | . | . | . | . | . | . | . | . | . | . | . | . | . | . | . | . | . | . | . | . | . | . | . |
| 21-225_170D5_LC | . | . | . | . | . | . | . | . | . | . | . | . | . | . | . | . | . | . | . | E | . | . | . | . | . | . |
| 21-225_170G4_LC | . | . | . | . | . | . | . | . | . | . | . | . | . | . | . | . | . | . | H | . | . | . | . | . | . | . |
| 21-225_170G5_LC | . | . | . | . | . | . | . | . | . | . | . | . | T | . | . | . | . | F | . | . | . | . | . | . | . | . |
| 21-225_171A4_LC | . | . | . | . | . | . | . | . | . | . | . | . | . | . | . | . | . | . | . | . | . | . | . | . | . | . |
| 21-225_171C3_LC | . | . | . | . | . | . | . | . | . | . | . | . | T | . | . | . | . | F | . | . | . | . | . | . | . | . |
| 21-225_172B3_LC | . | . | . | . | . | . | . | . | . | . | . | . | . | . | . | . | . | . | . | . | . | . | . | . | . | . |
| 21-225_172G8_LC | . | . | . | . | V | . | . | . | . | . | . | . | . | . | . | . | . | . | . | . | . | . | . | . | . | . |
| 21-225_173H12_LC | . | . | . | . | . | . | . | . | . | . | . | . | . | . | . | . | . | . | . | . | . | . | . | . | . | . |
| 21-225_175G1_LC | . | . | . | . | . | . | . | . | . | . | . | . | T | . | . | . | . | . | . | . | . | . | . | . | . | . |
| 21-225_176B11_LC | . | . | . | . | . | . | . | . | . | . | . | . | . | . | . | . | . | F | . | . | . | . | . | . | . | . |
| 21-225_178B10_LC | . | . | . | . | . | . | . | . | . | . | . | . | . | . | . | . | . | . | . | . | . | . | . | . | . | . |
| 21-225_17A10_LC | . | . | . | . | . | . | . | . | . | . | . | . | . | . | . | . | . | . | . | . | . | . | . | . | . | . |
| 21-225_17B5_LC | . | . | . | . | . | . | . | . | . | . | . | . | . | . | . | . | . | . | . | . | . | . | . | . | . | . |
| 21-225_17F9_LC | . | . | . | V | . | . | . | . | . | . | . | . | . | . | . | . | . | . | . | . | . | . | . | . | . | . |
| 21-225_17H12_LC | . | . | . | D | . | . | . | . | . | . | . | . | . | . | . | . | . | . | . | . | . | A | . | . | . | . |
| 21-225_17H8_LC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_18A1_LC | . | . | . | . | . | . | . | . | . | . | . | . | . | . | . | . | . | . | . | . | . | . | . | . | . | . |
| 21-225_18A3_LC | . | . | . | . | . | . | . | . | . | . | . | . | . | . | . | . | . | . | . | . | . | . | . | . | . | . |
| 21-225_18C6_LC | . | . | . | . | . | . | . | . | . | . | . | . | . | . | . | . | . | . | . | . | . | . | . | . | . | . |
| 21-225_18E8_LC | . | . | . | . | . | . | . | . | . | . | . | . | . | . | . | . | . | . | . | . | . | . | . | . | . | . |
| 21-225_190C8_LC | . | . | . | . | . | . | . | . | . | . | . | . | . | . | . | . | . | . | . | . | . | . | . | . | . | . |
| 21-225_193A5_LC | . | . | . | . | . | . | . | . | . | . | . | . | . | . | . | . | . | . | . | . | Y | . | . | . | . | . |
| 21-225_193E7_LC | . | . | . | . | . | . | . | . | . | . | . | . | . | . | . | . | . | . | . | . | . | . | . | . | . | . |
| 21-225_196C10_LC | . | . | . | . | . | . | . | . | . | . | . | . | . | . | . | . | . | . | . | . | . | . | . | . | . | . |
| 21-225_199E3_LC | . | . | . | . | . | . | . | . | . | . | . | . | . | . | . | . | . | . | . | . | . | . | . | . | . | . |
| 21-225_19D11_LC | . | . | . | . | . | . | . | . | . | . | . | . | . | . | . | . | . | . | . | . | . | . | . | . | . | . |
| 21-225_19E1_LC | . | . | . | . | . | . | . | . | . | . | . | . | . | . | . | . | . | . | . | . | . | . | . | . | . | . |
| 21-225_19F3_LC | . | . | . | . | . | . | . | . | . | . | . | . | . | . | . | . | . | . | . | . | . | . | . | . | . | . |
| 21-225_1A2_LC | . | . | . | . | . | . | . | . | . | . | . | . | . | . | . | . | . | . | . | . | . | . | . | . | . | . |
| 21-225_1C12_LC | . | . | . | . | . | . | . | . | . | . | . | . | . | . | . | . | . | . | . | . | . | . | . | . | . | . |
| 21-225_1F2_LC | . | . | . | . | . | . | . | . | . | . | . | . | . | . | . | . | . | . | . | . | . | . | . | . | . | . |
| 21-225_200F12_LC | . | . | . | . | . | . | . | . | . | . | . | . | . | . | . | . | . | . | . | . | . | . | . | R | . | . |
| 21-225_200H9_LC | . | . | . | . | . | . | . | . | . | . | . | . | . | . | . | . | . | . | . | . | . | . | . | . | . | . |
| 21-225_201A1_LC | . | . | . | . | . | . | . | . | . | . | . | . | . | . | . | . | . | . | . | . | . | . | . | . | . | . |
| 21-225_201A10_LC | . | . | . | . | . | . | . | . | . | . | . | . | . | . | . | . | . | . | . | . | . | . | . | R | . | . |
| 21-225_204H8_LC | . | . | . | . | . | . | . | . | . | . | . | . | . | . | . | . | . | . | . | . | . | . | . | . | . | . |
| 21-225_205H8_LC | . | . | . | . | . | . | . | . | . | . | . | . | . | . | . | . | . | . | . | . | . | Q | . | . | . | . |
| 21-225_20A10_LC | . | . | . | . | . | . | . | . | . | . | . | . | . | . | . | . | . | . | . | . | . | Q | . | . | . | . |
| 21-225_20A7_LC | . | . | . | . | . | . | . | . | . | . | . | . | . | . | . | . | . | . | . | . | . | . | . | . | . | . |
| 21-225_20C2_LC | . | . | . | . | . | . | . | . | . | . | . | . | . | . | . | . | . | . | . | . | . | . | . | . | . | . |
| 21-225_20C8_LC | . | . | . | . | . | . | . | . | . | . | . | . | . | . | . | . | . | . | . | K | . | . | . | . | . | . |
| 21-225_20D5_LC | . | . | . | . | . | . | . | . | . | . | . | . | . | . | . | . | . | . | . | . | . | . | . | . | . | . |
| 21-225_20E12_LC | . | . | . | . | . | . | . | . | . | . | . | . | . | . | . | . | . | . | . | . | . | . | . | . | . | . |
| 21-225_20E5_LC | . | . | . | . | . | . | . | . | . | . | . | . | . | . | . | . | . | . | . | . | . | . | . | . | . | . |
| 21-225_20G2_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_20G9_LC | . | . | . | . | . | . | . | . | . | . | . | . | . | . | . | . | . | . | . | . | . | . | . | . | . | . |
| 21-225_20H10_LC | . | . | . | . | . | K | . | . | . | . | . | . | . | N | . | . | . | . | . | . | . | . | . | . | G | . |
| 21-225_20H12_LC | . | . | . | . | . | . | . | . | . | . | . | . | . | . | . | D | . | . | . | . | . | . | . | . | . | . |
| 21-225_21E5_LC | . | . | . | . | . | . | . | . | . | . | . | . | . | . | . | . | . | . | . | . | . | . | . | . | . | . |
| 21-225_21F3_LC | . | . | . | . | . | . | . | . | . | . | . | . | . | . | . | . | . | . | . | . | . | . | . | . | . | . |
| 21-225_21G7_LC | . | . | . | . | V | . | . | . | . | . | . | . | . | . | . | . | . | . | . | . | . | . | . | . | . | . |
| 21-225_21H3_LC | . | . | . | . | . | . | . | . | . | . | . | . | . | . | . | . | . | . | . | . | . | . | . | . | . | . |
| 21-225_224A1_LC | . | . | . | . | . | E | . | . | . | . | . | . | . | . | . | . | . | . | . | . | . | . | . | . | . | . |
| 21-225_224B1_LC | . | . | . | . | . | S | . | . | . | . | . | . | . | . | . | . | . | . | . | . | . | . | . | . | . | . |
| 21-225_224C3_LC | . | . | . | D | . | . | . | . | . | . | . | . | . | . | . | . | . | . | . | . | . | . | . | . | . | . |
| 21-225_224D10_LC | . | . | . | . | . | . | . | . | . | . | . | . | . | . | . | . | . | . | . | . | . | . | . | . | . | . |
| 21-225_22B7_LC | . | . | . | . | . | . | . | . | . | . | . | . | . | . | . | . | . | . | . | . | . | . | . | . | . | . |
| 21-225_22C1_LC | . | . | . | . | . | . | . | . | . | . | . | . | . | . | . | . | . | . | . | . | . | . | . | . | . | . |
| 21-225_22D12_LC | . | . | . | . | . | . | . | . | . | . | . | . | . | . | . | . | . | . | . | . | . | . | . | . | . | . |
| 21-225_22F2_LC | . | . | . | . | . | . | . | . | . | . | . | . | . | . | . | . | . | . | . | . | . | . | . | . | . | . |
| 21-225_22F9_LC | . | . | . | . | . | . | . | . | . | . | . | . | . | . | . | . | . | . | . | . | . | . | . | . | . | . |
| 21-225_22G8_LC | . | . | . | . | . | . | . | . | . | . | . | . | . | . | . | . | . | . | . | . | . | . | . | . | . | . |
| 21-225_22G9_LC | . | . | . | . | . | . | . | . | . | . | . | . | . | . | . | . | . | . | . | . | . | . | . | . | . | . |
| 21-225_22H4_LC | . | . | . | . | . | . | . | . | . | . | . | . | . | . | . | . | . | . | . | . | . | . | . | . | . | . |
| 21-225_23A3_LC | . | . | . | . | . | . | . | . | . | . | . | . | . | . | . | . | . | . | . | . | . | . | . | . | . | . |
| 21-225_23C8_LC | . | . | . | D | . | . | . | . | . | . | . | . | . | . | . | . | . | . | . | . | . | . | . | R | . | . |
| 21-225_23D1_LC | . | . | . | . | . | . | . | . | . | . | . | . | . | . | . | . | . | . | . | . | . | . | . | . | . | . |
| 21-225_23H11_LC | . | . | . | . | . | . | . | . | . | . | . | . | . | . | . | D | . | . | . | . | . | . | . | . | . | . |
| 21-225_24E5_LC | . | . | . | . | . | . | . | . | . | . | . | . | . | . | . | . | . | . | . | . | . | . | . | . | . | . |
| 21-225_25E6_LC | . | . | . | . | . | . | . | . | . | . | . | . | . | . | . | . | . | . | . | R | . | . | . | . | . | . |
| 21-225_25H10_LC | . | . | R | . | . | . | . | . | . | . | . | . | . | . | . | . | . | . | . | . | . | . | . | . | . | . |
| 21-225_25H11_LC | . | . | . | . | . | . | . | . | . | . | . | . | T | . | . | . | . | . | . | . | . | . | . | . | . | . |
| 21-225_27C3_LC | . | . | . | . | . | . | . | . | . | . | . | . | . | . | . | . | . | . | . | . | . | . | . | . | . | . |
| 21-225_27F2_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_29B8_LC | . | . | . | . | . | . | . | . | . | . | . | . | . | . | . | . | . | . | . | . | . | . | . | . | . | . |
| 21-225_29G4_LC | . | . | . | . | . | . | . | . | . | . | . | . | . | . | . | . | . | . | . | . | . | . | . | . | . | . |
| 21-225_29G8_LC | . | . | . | . | . | . | . | . | . | . | . | . | . | . | . | . | . | . | . | L | . | . | . | . | . | . |
| 21-225_29H6_LC | . | . | . | . | . | . | . | . | . | . | . | . | . | . | . | . | . | . | . | . | . | S | . | . | T | . |
| 21-225_2A11_LC | . | . | . | . | . | . | . | . | . | . | . | . | . | . | . | . | . | . | . | . | . | . | . | . | . | . |
| 21-225_2B2_LC | . | . | . | . | . | . | . | . | . | . | . | . | . | . | . | . | . | . | . | . | . | . | . | Q | . | . |
| 21-225_2G9_LC | . | . | . | . | . | . | . | . | . | . | . | . | . | . | . | . | . | . | . | . | . | . | . | . | . | . |
| 21-225_30D1_LC | . | . | . | D | . | . | . | . | . | . | . | . | S | . | . | . | . | . | . | . | . | . | . | . | . | . |
| 21-225_30E3_LC | . | . | . | D | . | . | . | . | . | . | . | . | D | . | . | . | . | . | . | . | . | . | . | . | . | . |
| 21-225_30E9_LC | . | . | . | D | . | . | . | . | . | . | . | . | D | . | . | . | . | . | . | . | . | . | . | . | . | . |
| 21-225_30F3_LC | . | . | . | . | . | . | . | . | . | . | . | . | . | . | . | . | . | . | . | . | . | . | . | . | . | . |
| 21-225_30G11_LC | . | . | . | D | . | . | . | . | . | . | . | . | S | . | . | . | . | . | . | . | . | . | . | . | . | . |
| 21-225_31A8_LC | . | . | . | D | . | . | . | . | . | . | . | . | S | . | . | . | . | . | . | . | . | . | . | . | . | . |
| 21-225_31B8_LC | . | . | . | D | . | . | . | . | . | . | . | . | S | . | . | . | . | . | . | . | . | . | . | . | . | . |
| 21-225_31H3_LC | . | . | . | D | . | . | . | . | . | . | . | . | D | . | . | . | . | . | . | . | . | . | . | . | . | . |
| 21-225_31H5_LC | . | . | . | D | . | . | . | . | . | . | . | . | S | . | . | . | . | . | . | . | . | . | . | . | . | H |
| 21-225_32G1_LC | . | . | . | D | M | . | . | . | . | . | . | . | S | . | . | . | . | . | . | . | . | . | . | . | . | . |
| 21-225_32G12_LC | . | . | . | D | . | . | . | . | . | . | . | . | S | . | . | . | . | . | . | . | R | . | . | . | . | . |
| 21-225_33A4_LC | . | . | . | D | . | . | . | . | . | . | . | . | . | . | V | . | . | . | . | . | . | . | . | . | . | . |
| 21-225_33A7_LC | . | . | . | D | . | . | . | . | . | . | . | . | S | . | . | . | . | . | . | . | . | . | . | . | . | . |
| 21-225_33B1_LC | . | . | . | . | . | . | . | . | . | . | . | . | . | . | . | . | . | F | . | . | . | . | . | . | . | . |
| 21-225_33B8_LC | . | . | . | D | . | . | . | . | . | . | . | . | S | . | . | . | . | . | . | . | . | . | . | . | . | . |
| 21-225_33F1_LC | . | . | . | D | . | . | . | . | . | . | . | . | S | . | . | . | . | . | . | . | . | . | . | . | . | . |
| 21-225_33G7_LC | . | . | . | . | . | . | . | . | . | . | . | . | . | . | . | . | . | . | . | . | . | . | . | . | . | . |
| 21-225_34A6_LC | . | . | . | D | . | . | . | . | . | . | . | . | . | . | . | . | . | . | . | . | . | . | . | . | . | . |
| 21-225_34D11_LC | . | . | . | D | . | . | . | . | . | . | . | . | S | . | . | . | . | . | . | . | . | . | . | . | . | . |
| 21-225_34G8_LC | . | . | . | D | . | . | . | . | . | . | . | . | S | . | . | . | . | . | . | . | . | . | . | . | . | . |
| 21-225_34G9_LC | . | . | . | D | . | . | . | . | . | . | . | . | S | . | . | . | . | . | . | . | . | . | . | . | . | . |
| 21-225_34H11_LC | . | . | . | D | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_35A6_LC | . | . | . | D | . | . | . | . | . | . | . | . | S | . | . | . | . | . | . | . | . | . | . | . | . | . |
| 21-225_35B7_LC | . | . | . | D | . | . | . | . | . | . | . | . | S | . | . | . | . | . | . | . | . | . | . | . | . | . |
| 21-225_35D1_LC | . | . | . | D | . | . | . | . | . | . | . | . | S | . | . | . | . | . | . | . | . | . | . | . | . | . |
| 21-225_35F11_LC | . | . | . | D | . | . | . | . | . | . | . | . | S | . | . | . | . | . | . | . | . | . | . | . | . | . |
| 21-225_36A5_LC | . | . | . | D | . | . | . | . | . | . | . | . | . | . | . | . | . | . | . | . | . | . | . | . | . | . |
| 21-225_36C5_LC | . | . | . | D | . | . | . | . | . | . | . | . | S | . | . | . | . | . | . | . | . | . | . | . | . | . |
| 21-225_43C3_LC | . | . | . | . | . | . | . | . | . | . | . | . | . | . | . | . | . | . | . | . | . | . | . | . | . | . |
| 21-225_44C3_LC | . | . | . | . | . | . | . | . | . | . | . | . | . | . | . | . | . | . | . | . | . | . | . | . | . | . |
| 21-225_44E12_LC | . | . | . | . | . | . | . | . | . | . | . | . | . | . | . | . | . | . | . | . | . | . | . | . | . | . |
| 21-225_46B1_LC | . | . | . | . | . | . | . | . | . | . | . | . | . | . | . | . | . | . | . | . | . | . | . | . | . | . |
| 21-225_46C6_LC | . | . | . | . | . | . | . | . | . | . | . | . | . | . | . | . | . | . | . | . | . | . | . | . | . | . |
| 21-225_46D8_LC | . | . | . | . | . | . | . | . | . | . | . | . | K | . | . | . | . | . | . | . | . | . | . | . | . | . |
| 21-225_46F3_LC | . | . | . | . | . | . | . | . | . | . | . | . | K | . | . | . | . | . | . | . | . | . | . | . | . | . |
| 21-225_47A1_LC | . | . | . | D | . | . | . | . | . | . | . | . | . | . | . | . | . | . | . | . | . | . | . | . | . | . |
| 21-225_47A5_LC | . | . | . | . | . | . | . | . | . | . | . | . | . | . | . | . | . | . | . | . | . | . | . | . | . | . |
| 21-225_48D12_LC | . | . | . | . | . | . | . | . | . | . | . | . | . | . | . | . | . | . | . | . | . | . | . | . | . | . |
| 21-225_48H4_LC | . | . | R | D | . | . | . | . | . | . | . | . | . | . | . | . | . | . | . | . | . | . | . | . | . | . |
| 21-225_49A1_LC | . | . | . | . | . | . | . | . | . | . | . | . | . | . | . | D | . | . | . | . | . | . | . | . | . | . |
| 21-225_49C6_LC | . | . | . | . | . | . | . | . | . | . | . | . | . | . | . | . | . | . | . | . | . | . | . | . | . | . |
| 21-225_4C8_LC | . | . | . | . | . | . | . | . | . | . | . | . | . | . | . | . | . | . | . | . | . | . | . | . | . | . |
| 21-225_4E10_LC | . | . | . | . | . | . | . | . | . | . | . | . | . | . | . | . | . | . | . | . | . | . | . | . | . | . |
| 21-225_4F12_LC | . | . | . | . | . | . | . | . | . | . | . | . | . | . | . | . | . | . | . | . | . | . | . | . | . | . |
| 21-225_4F4_LC | . | . | . | . | . | . | . | . | . | . | . | . | . | . | . | . | . | . | . | . | . | . | . | . | . | . |
| 21-225_4H4_LC | . | . | . | . | . | . | . | . | . | . | . | . | . | . | . | . | . | . | . | . | . | . | . | . | . | . |
| 21-225_50G10_LC | . | . | . | . | . | . | . | . | . | . | . | . | . | N | . | . | . | . | . | . | . | . | . | . | V | . |
| 21-225_51F11_LC | . | . | . | . | . | . | . | . | . | . | . | . | . | . | . | . | . | . | . | . | . | . | . | . | . | . |
| 21-225_52B2_LC | . | . | . | . | . | . | . | . | . | . | . | . | . | . | . | . | . | . | . | . | . | . | . | . | . | . |
| 21-225_53D2_LC | . | . | . | . | . | . | . | . | . | . | . | . | . | . | . | . | . | . | . | . | . | . | . | . | . | . |
| 21-225_53E6_LC | . | . | . | . | . | . | . | . | . | . | . | . | . | . | . | . | . | . | . | . | N | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_54C6_LC | . | . | . | . | . | . | . | . | . | . | . | . | . | . | . | . | . | . | . | . | N | . | . | . | . | . |
| 21-225_55B8_LC | . | . | . | A | . | . | . | . | . | . | . | . | . | . | . | . | . | . | . | . | . | . | . | . | . | . |
| 21-225_55H6_LC | . | . | . | . | . | . | . | . | . | . | . | . | . | . | . | . | . | . | . | . | . | . | . | . | . | . |
| 21-225_56F1_LC | . | . | . | D | . | . | . | . | . | . | . | . | . | N | . | . | . | . | . | . | . | . | . | . | . | . |
| 21-225_59E6_LC | . | . | . | . | . | . | . | . | . | . | . | . | . | . | . | . | . | . | . | . | . | . | . | . | . | . |
| 21-225_5H2_LC | . | . | . | . | . | . | . | . | . | . | . | . | . | . | . | . | . | . | . | . | . | . | . | . | . | . |
| 21-225_5H7_LC | . | . | . | . | . | . | . | . | . | . | . | . | . | . | . | . | . | . | . | . | . | . | . | . | . | . |
| 21-225_60E8_LC | . | . | . | . | . | . | . | . | . | . | . | . | . | . | . | . | . | F | . | . | . | . | . | . | . | . |
| 21-225_62E2_LC | . | . | . | . | . | . | . | . | . | . | . | . | . | . | . | . | . | . | . | . | . | . | . | . | . | . |
| 21-225_62E4_LC | . | . | . | . | . | . | . | . | . | . | . | . | . | . | . | . | . | . | . | . | . | . | . | . | . | . |
| 21-225_64E1_LC | . | . | . | . | . | . | . | . | . | . | . | . | . | . | . | . | . | . | . | . | . | . | . | . | . | . |
| 21-225_68D12_LC | . | . | . | . | . | . | . | . | . | . | . | . | . | . | . | . | . | . | . | . | . | . | . | . | . | . |
| 21-225_68F11_LC | . | . | . | . | . | . | . | . | . | . | . | . | . | . | . | . | . | . | . | . | . | . | . | . | . | . |
| 21-225_6A6_LC | . | . | . | . | . | . | . | . | . | . | . | . | . | . | . | . | . | . | . | . | . | . | . | . | . | . |
| 21-225_6C12_LC | . | . | . | . | . | . | . | . | . | . | . | . | S | N | . | . | . | . | . | . | . | . | . | . | . | . |
| 21-225_6D4_LC | . | . | . | A | . | . | . | . | . | . | . | . | . | . | . | . | . | . | . | . | . | . | . | . | . | . |
| 21-225_6G12_LC | . | . | . | . | . | . | . | . | . | . | . | . | . | . | . | . | . | . | . | . | . | . | . | . | . | . |
| 21-225_70E12_LC | . | . | . | . | . | N | . | . | . | . | . | . | . | N | . | N | . | F | . | . | . | . | . | . | . | . |
| 21-225_70E8_LC | . | . | . | . | . | . | . | . | . | . | . | . | K | . | . | . | . | . | . | . | . | . | . | . | . | . |
| 21-225_73G6_LC | . | . | . | . | . | . | . | . | . | . | . | . | . | . | . | . | . | . | . | . | . | . | . | . | . | . |
| 21-225_74A1_LC | . | . | . | . | . | . | . | . | . | . | . | . | . | . | . | . | . | . | . | . | . | . | . | . | . | . |
| 21-225_74D7_LC | . | . | . | . | . | . | . | . | . | . | . | . | . | . | . | . | . | . | . | . | . | . | . | . | . | . |
| 21-225_74E4_LC | . | . | . | . | . | . | . | . | . | . | . | . | . | . | . | . | . | . | . | . | . | . | . | . | . | . |
| 21-225_75H7_LC | . | . | . | . | . | . | . | . | . | . | . | . | . | . | . | . | . | . | . | . | . | . | . | . | S | . |
| 21-225_7C4_LC | . | . | . | . | . | . | . | . | . | . | . | . | . | . | . | . | . | . | . | . | . | . | . | . | . | H |
| 21-225_7E9_LC | . | . | . | . | . | . | . | . | . | . | . | . | . | . | . | . | . | . | E | . | . | . | . | . | . | . |
| 21-225_7F4_LC | . | . | . | . | . | . | . | . | . | . | . | . | . | . | . | . | . | . | . | . | . | . | . | . | . | . |
| 21-225_7H11_LC | . | . | . | . | . | . | . | . | . | . | . | . | . | . | . | . | . | . | . | . | . | . | . | . | . | . |
| 21-225_7H7_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_8C9_LC | . | . | . | A | . | . | . | . | . | . | . | . | . | . | . | D | . | . | . | . | . | . | . | . | . | . |
| 21-225_8D12_LC | . | . | . | . | . | . | . | . | . | . | . | . | . | . | . | . | . | . | . | . | . | . | . | . | . | . |
| 21-225_8F11_LC | . | . | . | . | . | . | . | . | . | . | . | . | S | . | . | . | . | F | . | . | . | . | . | . | . | . |
| 21-225_8H7_LC | . | . | . | . | . | . | . | . | . | . | . | . | . | . | . | . | . | . | . | . | . | . | . | . | . | . |
| 21-225_96A3_LC | . | . | . | . | . | . | . | . | . | . | . | . | . | . | . | . | . | . | . | . | . | . | . | . | . | . |
| 21-225_9E8_LC | . | . | . | . | . | . | . | . | . | . | . | . | . | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | R | L | I | Y | A | - | - | - | - | - | - | - | - | A | S | S | L | Q | S | G | V | P | S | R | F |
| 21-225_10G5_LC | . | . | . | . | . | . | . | . | . | . | . | . | . | . | . | . | . | . | . | . | . | . | . | . | . | . |
| 21-225_10H12_LC | . | . | . | . | . | G | . | . | . | . | . | . | . | . | . | . | . | . | . | . | . | . | . | . | . | . |
| 21-225_11F10_LC | . | . | . | . | . | . | . | . | . | . | . | . | . | . | . | . | . | . | . | . | . | . | . | . | . | . |
| 21-225_11F5_LC | . | . | . | . | . | . | . | . | . | . | . | . | . | . | . | . | . | V | . | . | . | . | . | . | . | . |
| 21-225_11G7_LC | . | . | . | . | . | . | . | . | . | . | . | . | . | . | . | . | . | . | . | . | . | . | . | . | . | . |
| 21-225_12A1_LC | . | . | . | . | . | . | . | . | . | . | . | . | . | . | . | . | . | . | . | . | . | . | . | . | . | . |
| 21-225_13E6_LC | . | . | . | . | . | . | . | . | . | . | . | . | . | . | . | . | . | . | . | . | . | . | . | . | . | . |
| 21-225_13H3_LC | . | . | . | . | . | . | . | . | . | . | . | . | . | . | . | . | . | . | . | . | . | . | . | . | . | . |
| 21-225_13H5_LC | . | . | . | . | . | . | . | . | . | . | . | . | . | . | . | . | . | . | . | T | . | . | . | . | . | . |
| 21-225_146F1_LC | . | . | . | . | . | . | . | . | . | . | . | . | . | . | . | . | . | . | . | . | . | . | . | . | . | . |
| 21-225_146G11_LC | . | . | . | . | . | . | . | . | . | . | . | . | . | . | . | . | . | . | . | . | . | . | . | L | . | . |
| 21-225_146H9_LC | . | . | . | . | . | . | . | . | . | . | . | . | . | . | . | . | . | . | . | . | . | . | . | . | . | . |
| 21-225_148E11_LC | . | . | . | . | S | . | . | . | . | . | . | . | . | . | . | . | . | . | . | . | . | . | . | . | . | . |
| 21-225_148F12_LC | . | . | . | . | . | . | . | . | . | . | . | . | . | . | . | . | . | . | . | . | . | . | . | . | . | . |
| 21-225_149F12_LC | . | . | . | . | . | . | . | . | . | . | . | . | . | . | . | . | N | . | . | . | . | . | . | . | . | . |
| 21-225_149G1_LC | . | . | . | . | . | . | . | . | . | . | . | . | . | . | . | . | N | . | . | . | . | . | . | . | . | . |
| 21-225_149G5_LC | . | . | . | . | . | . | . | . | . | . | . | . | . | . | . | . | . | . | . | . | . | . | . | . | . | . |
| 21-225_14B1_LC1_LC | . | . | . | . | . | T | . | . | . | . | . | . | . | . | . | Y | . | . | . | . | . | . | . | . | . | . |
| 21-225_14B2_LC | . | . | . | . | . | V | . | . | . | . | . | . | . | . | T | . | . | . | . | G | . | . | . | . | . | . |
| 21-225_14C2_LC | . | . | . | . | . | . | . | . | . | . | . | . | . | . | . | . | . | . | . | . | . | . | . | . | . | . |
| 21-225_14E3_LC | . | . | I | . | . | S | . | . | . | . | . | . | . | . | . | . | . | . | . | . | . | . | . | . | . | . |
| 21-225_150E7_LC | . | . | . | . | . | . | . | . | . | . | . | . | . | . | . | . | N | . | . | . | . | . | . | L | . | . |
| 21-225_152H9_LC | . | . | . | . | . | . | . | . | . | . | . | . | . | . | . | . | . | . | . | . | . | . | . | . | . | . |
| 21-225_156G1_LC | . | . | . | . | . | . | . | . | . | . | . | . | . | . | . | . | . | . | . | . | . | . | . | . | . | . |
| 21-225_158H5_LC | . | . | . | . | . | R | . | . | . | . | . | . | . | . | . | . | . | . | . | . | . | . | . | . | . | . |
| 21-225_15A1_LC | . | . | . | . | . | . | . | . | . | . | . | . | . | . | . | . | . | . | . | . | . | . | . | . | . | . |
| 21-225_15C9_LC | . | . | . | . | . | . | . | . | . | . | . | . | . | . | . | . | . | . | . | . | . | . | . | . | . | . |
| 21-225_15E3_LC | . | . | . | . | . | . | . | . | . | . | . | . | . | . | . | . | . | S | . | . | . | . | . | . | . | . |
| 21-225_15G7_LC | . | . | . | . | . | . | . | . | . | . | . | . | . | . | . | . | . | . | . | . | . | I | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | R | L | I | Y | A | - | - | - | - | - | - | - | - | A | S | S | L | Q | S | G | V | P | S | R | F |
| 21-225_15H1_LC | . | . | . | . | . | T | . | . | . | . | . | . | . | . | . | . | . | . | . | . | . | . | . | . | . | . |
| 21-225_15H10_LC | . | . | . | . | . | . | . | . | . | . | . | . | . | . | . | . | . | . | . | . | . | . | . | . | . | . |
| 21-225_15H8_LC | . | . | . | . | F | . | . | . | . | . | . | . | . | . | . | . | . | . | . | . | . | . | . | . | . | . |
| 21-225_160B10_LC | . | . | . | . | . | . | . | . | . | . | . | . | . | . | . | . | . | . | . | . | . | . | . | . | . | . |
| 21-225_160H3_LC | . | . | . | . | . | . | . | . | . | . | . | . | . | . | . | . | . | . | . | . | . | . | . | . | . | . |
| 21-225_169B1_LC | . | . | . | . | G | . | . | . | . | . | . | . | . | . | E | . | . | . | . | . | . | . | . | . | . | . |
| 21-225_169F9_LC | . | . | . | . | F | . | . | . | . | . | . | . | . | . | . | . | . | . | . | . | . | . | . | . | . | . |
| 21-225_16A1_LC | . | . | . | . | . | . | . | . | . | . | . | . | . | . | . | . | . | . | . | . | . | . | . | . | . | . |
| 21-225_16B7_LC | . | . | . | . | . | . | . | . | . | . | . | . | . | . | . | . | . | . | . | . | . | . | . | . | . | . |
| 21-225_16E12_LC | . | . | . | . | . | . | . | . | . | . | . | . | . | . | . | . | . | . | . | . | . | . | . | . | . | . |
| 21-225_16F11_LC | . | . | . | . | . | . | . | . | . | . | . | . | . | . | . | . | . | V | . | . | . | . | . | . | . | . |
| 21-225_16F4_LC | . | . | . | . | . | V | . | . | . | . | . | . | . | . | . | . | . | . | . | . | . | . | . | . | . | . |
| 21-225_170D11_LC | . | H | . | . | . | . | . | . | . | . | . | . | . | . | . | . | . | . | . | N | . | . | . | . | . | . |
| 21-225_170D5_LC | . | H | . | . | . | . | . | . | . | . | . | . | . | . | . | . | . | . | . | N | . | . | . | . | . | . |
| 21-225_170G4_LC | . | . | . | . | . | . | . | . | . | . | . | . | . | . | . | . | T | . | . | . | . | . | . | . | . | . |
| 21-225_170G5_LC | . | . | . | . | . | T | . | . | . | . | . | . | . | . | . | . | . | . | . | . | . | . | . | . | . | . |
| 21-225_171A4_LC | . | . | . | . | . | T | . | . | . | . | . | . | . | . | . | . | . | . | . | . | . | . | . | . | . | . |
| 21-225_171C3_LC | . | . | . | . | . | T | . | . | . | . | . | . | . | . | . | . | . | . | . | . | . | . | . | . | . | . |
| 21-225_172B3_LC | . | . | . | . | G | . | . | . | . | . | . | . | . | . | E | . | . | . | . | . | . | . | . | . | . | . |
| 21-225_172G8_LC | . | H | . | . | . | . | . | . | . | . | . | . | . | . | . | . | . | . | . | N | . | . | . | . | . | . |
| 21-225_173H12_LC | . | . | . | . | . | T | . | . | . | . | . | . | . | . | T | . | . | . | . | . | . | . | . | . | . | . |
| 21-225_175G1_LC | . | . | . | . | . | T | . | . | . | . | . | . | . | . | . | . | . | . | . | . | . | . | . | . | . | . |
| 21-225_176B11_LC | . | . | . | . | . | . | . | . | . | . | . | . | . | . | . | . | . | . | . | . | . | . | . | . | . | . |
| 21-225_178B10_LC | . | H | . | . | . | . | . | . | . | . | . | . | . | . | . | . | . | . | . | N | . | . | . | . | . | . |
| 21-225_17A10_LC | . | . | . | . | . | . | . | . | . | . | . | . | . | . | . | . | . | . | . | . | . | . | . | . | . | . |
| 21-225_17B5_LC | . | . | . | . | . | . | . | . | . | . | . | . | . | . | . | . | N | . | . | . | . | . | . | . | . | . |
| 21-225_17F9_LC | . | . | . | . | . | . | . | . | . | . | . | . | . | . | V | . | . | . | . | . | . | . | . | . | . | . |
| 21-225_17H12_LC | . | . | . | . | N | . | . | . | . | . | . | . | . | . | . | . | . | . | . | . | . | . | . | . | . | . |
| 21-225_17H8_LC | . | . | . | . | . | G | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | R | L | I | Y | A | - | - | - | - | - | - | - | - | A | S | S | L | Q | S | G | V | P | S | R | F |
| 21-225_18A1_LC | . | . | . | . | . | . | . | . | . | . | . | . | . | . | . | . | . | . | . | . | . | . | . | . | . | . |
| 21-225_18A3_LC | . | . | . | . | . | V | . | . | . | . | . | . | . | . | . | . | . | . | . | . | . | . | . | . | . | . |
| 21-225_18C6_LC | . | . | . | . | . | . | . | . | . | . | . | . | . | . | . | . | . | . | . | . | . | . | . | . | . | . |
| 21-225_18E8_LC | . | . | . | . | . | . | . | . | . | . | . | . | . | . | . | . | . | . | . | . | . | . | . | . | . | . |
| 21-225_190C8_LC | . | . | . | . | . | . | . | . | . | . | . | . | . | . | . | . | . | . | . | . | . | . | . | . | . | . |
| 21-225_193A5_LC | . | . | V | . | . | . | . | . | . | . | . | . | . | . | . | . | . | . | . | . | . | . | . | . | . | . |
| 21-225_193E7_LC | . | . | . | . | . | S | . | . | . | . | . | . | . | . | . | . | . | . | . | R | . | . | . | . | . | . |
| 21-225_196C10_LC | . | . | . | . | . | . | . | . | . | . | . | . | . | . | . | . | . | . | . | . | . | . | . | . | . | . |
| 21-225_199E3_LC | . | . | . | . | S | S | . | . | . | . | . | . | . | . | . | . | . | . | . | R | . | . | . | . | . | . |
| 21-225_19D11_LC | . | . | . | . | . | . | . | . | . | . | . | . | . | . | T | . | . | . | . | . | . | . | . | . | . | . |
| 21-225_19E1_LC | . | . | . | . | . | . | . | . | . | . | . | . | . | . | . | . | N | . | . | . | . | . | . | . | . | . |
| 21-225_19F3_LC | . | . | . | . | . | . | . | . | . | . | . | . | . | . | V | . | . | . | . | . | . | . | . | . | . | . |
| 21-225_1A2_LC | . | . | . | . | . | . | . | . | . | . | . | . | . | . | . | . | . | . | . | . | . | . | . | . | . | . |
| 21-225_1C12_LC | . | . | . | . | . | . | . | . | . | . | . | . | . | . | . | . | . | . | . | . | . | . | . | . | . | . |
| 21-225_1F2_LC | . | . | . | . | . | . | . | . | . | . | . | . | . | . | . | . | . | V | . | . | . | . | . | . | . | . |
| 21-225_200F12_LC | . | . | . | . | . | S | . | . | . | . | . | . | . | . | . | . | . | . | H | T | . | . | . | . | . | . |
| 21-225_200H9_LC | . | . | . | . | . | . | . | . | . | . | . | . | . | . | . | . | . | . | . | . | . | I | . | . | . | . |
| 21-225_201A1_LC | . | . | . | . | . | S | . | . | . | . | . | . | . | . | . | . | I | . | . | R | . | . | . | . | . | . |
| 21-225_201A10_LC | . | . | . | . | . | S | . | . | . | . | . | . | . | . | T | . | . | . | M | . | . | . | . | . | . | . |
| 21-225_204H8_LC | . | . | . | . | . | S | . | . | . | . | . | . | . | . | . | . | . | . | . | . | . | . | . | . | . | . |
| 21-225_205H8_LC | . | L | . | . | . | S | . | . | . | . | . | . | . | . | . | . | F | . | . | R | . | . | . | . | . | . |
| 21-225_20A10_LC | . | . | . | . | . | . | . | . | . | . | . | . | . | . | . | . | . | . | . | . | . | . | . | . | . | . |
| 21-225_20A7_LC | . | . | . | . | . | . | . | . | . | . | . | . | . | . | . | . | . | . | . | . | . | . | . | . | . | . |
| 21-225_20C2_LC | . | . | . | . | F | . | . | . | . | . | . | . | . | . | . | . | . | . | . | . | . | . | . | . | . | . |
| 21-225_20C8_LC | . | . | . | . | N | . | . | . | . | . | . | . | . | . | . | . | . | . | . | . | . | . | . | . | . | . |
| 21-225_20D5_LC | . | . | . | . | . | . | . | . | . | . | . | . | . | . | . | . | . | . | . | . | . | . | . | . | . | . |
| 21-225_20E12_LC | . | . | . | . | F | . | . | . | . | . | . | . | . | . | . | . | . | . | . | . | . | . | . | . | . | . |
| 21-225_20E5_LC | . | . | . | . | . | . | . | . | . | . | . | . | . | . | . | . | . | . | . | . | . | . | . | . | . | . |
| 21-225_20G2_LC | . | . | . | . | . | . | . | . | . | . | . | . | . | . | . | . | . | F | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | R | L | I | Y | A | - | - | - | - | - | - | - | - | A | S | S | L | Q | S | G | V | P | S | R | F |
| 21-225_20G9_LC | . | . | . | . | . | . | . | . | . | . | . | . | . | . | . | . | . | . | . | . | . | . | . | . | . | . |
| 21-225_20H10_LC | . | C | . | . | . | . | . | . | . | . | . | . | . | . | . | . | . | . | . | . | . | . | . | . | . | . |
| 21-225_20H12_LC | . | . | . | . | . | . | . | . | . | . | . | . | . | . | . | . | . | . | . | . | . | . | . | . | . | . |
| 21-225_21E5_LC | . | . | . | . | . | . | . | . | . | . | . | . | . | . | T | . | . | . | . | . | . | . | . | . | . | . |
| 21-225_21F3_LC | . | . | . | . | . | . | . | . | . | . | . | . | . | . | . | . | . | . | . | . | . | . | . | . | . | . |
| 21-225_21G7_LC | Q | . | . | . | . | . | . | . | . | . | . | . | . | . | . | . | . | . | . | . | . | . | . | . | . | . |
| 21-225_21H3_LC | . | . | . | . | . | . | . | . | . | . | . | . | . | . | . | . | . | V | . | T | . | . | . | . | . | . |
| 21-225_224A1_LC | . | . | . | . | . | . | . | . | . | . | . | . | . | . | . | . | . | . | . | . | . | . | . | . | . | . |
| 21-225_224B1_LC | . | . | . | . | . | . | . | . | . | . | . | . | . | . | . | . | . | . | . | . | . | . | . | . | . | . |
| 21-225_224C3_LC | . | . | . | . | . | . | . | . | . | . | . | . | . | . | T | . | . | . | . | . | . | . | . | . | . | . |
| 21-225_224D10_LC | . | . | . | . | . | . | . | . | . | . | . | . | . | . | . | . | . | . | . | . | . | . | . | . | . | . |
| 21-225_22B7_LC | . | . | . | . | . | T | . | . | . | . | . | . | . | . | . | . | . | . | . | . | . | . | . | . | . | . |
| 21-225_22C1_LC | . | . | . | . | . | . | . | . | . | . | . | . | . | . | . | . | . | . | . | . | . | . | . | . | . | . |
| 21-225_22D12_LC | . | . | . | . | . | . | . | . | . | . | . | . | . | . | . | . | . | . | . | . | . | . | . | . | . | . |
| 21-225_22F2_LC | . | . | . | . | . | . | . | . | . | . | . | . | . | . | . | . | . | . | . | . | . | . | . | . | . | . |
| 21-225_22F9_LC | . | . | . | . | . | . | . | . | . | . | . | . | . | . | . | . | . | . | . | . | . | . | . | . | . | . |
| 21-225_22G8_LC | . | . | . | . | . | . | . | . | . | . | . | . | . | . | . | . | . | . | . | . | . | . | . | . | K | . |
| 21-225_22G9_LC | . | . | . | . | . | . | . | . | . | . | . | . | . | . | . | . | . | . | . | . | . | . | . | . | . | . |
| 21-225_22H4_LC | . | . | . | . | . | . | . | . | . | . | . | . | . | . | . | . | . | V | . | . | . | . | . | . | . | . |
| 21-225_23A3_LC | . | . | . | . | . | . | . | . | . | . | . | . | . | . | . | . | . | V | . | . | . | . | . | . | . | . |
| 21-225_23C8_LC | . | . | . | . | N | G | . | . | . | . | . | . | . | . | . | . | . | V | . | . | . | . | . | . | . | . |
| 21-225_23D1_LC | . | . | . | . | . | . | . | . | . | . | . | . | . | . | . | . | . | . | . | . | . | . | . | . | . | . |
| 21-225_23H11_LC | . | . | . | . | . | . | . | . | . | . | . | . | . | . | . | . | . | . | . | . | . | . | . | . | . | . |
| 21-225_24E5_LC | . | . | . | . | . | . | . | . | . | . | . | . | . | . | . | . | . | . | . | . | . | . | . | . | . | . |
| 21-225_25E6_LC | . | . | . | . | . | . | . | . | . | . | . | . | . | . | . | . | . | . | . | . | . | . | . | . | . | . |
| 21-225_25H10_LC | . | . | . | . | . | . | . | . | . | . | . | . | . | . | . | F | . | . | . | . | . | . | . | . | . | . |
| 21-225_25H11_LC | . | . | . | . | . | . | . | . | . | . | . | . | . | . | . | . | . | . | . | . | . | . | . | . | . | . |
| 21-225_27C3_LC | . | . | . | . | . | . | . | . | . | . | . | . | . | . | . | . | . | . | . | . | . | . | . | . | . | . |
| 21-225_27F2_LC | . | . | . | . | . | V | . | . | . | . | . | . | . | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | R | L | I | Y | A | - | - | - | - | - | - | - | - | A | S | S | L | Q | S | G | V | P | S | R | F |
| 21-225_29B8_LC | . | . | . | . | . | . | . | . | . | . | . | . | . | . | . | . | . | . | . | . | . | . | . | . | . | . |
| 21-225_29G4_LC | . | . | . | . | . | . | . | . | . | . | . | . | . | . | . | . | . | . | . | . | . | . | . | . | . | . |
| 21-225_29G8_LC | . | . | . | . | . | . | . | . | . | . | . | . | . | . | . | . | . | . | . | . | . | . | . | . | . | . |
| 21-225_29H6_LC | . | . | . | . | . | . | . | . | . | . | . | . | . | . | . | . | . | . | . | . | . | . | . | . | . | . |
| 21-225_2A11_LC | Q | . | . | . | S | . | . | . | . | . | . | . | . | . | . | . | . | . | . | . | . | . | . | . | . | . |
| 21-225_2B2_LC | . | . | . | . | . | . | . | . | . | . | . | . | . | . | . | . | . | . | . | . | . | . | . | . | . | . |
| 21-225_2G9_LC | Q | . | . | . | . | . | . | . | . | . | . | . | . | . | . | . | . | . | . | . | . | . | . | . | . | . |
| 21-225_30D1_LC | E | . | . | . | . | . | . | . | . | . | . | . | . | . | . | . | . | . | . | . | . | . | . | . | . | . |
| 21-225_30E3_LC | R | . | . | . | . | . | . | . | . | . | . | . | . | . | . | P | . | . | . | . | . | . | . | . | . | . |
| 21-225_30E9_LC | R | . | . | . | . | G | . | . | . | . | . | . | . | . | . | P | . | . | . | . | . | . | . | . | . | . |
| 21-225_30F3_LC | . | . | . | . | . | T | . | . | . | . | . | . | . | . | . | . | . | . | . | . | . | . | . | . | . | . |
| 21-225_30G11_LC | . | . | . | . | . | . | . | . | . | . | . | . | . | . | . | . | . | . | . | . | . | . | . | . | . | . |
| 21-225_31A8_LC | . | . | . | . | . | . | . | . | . | . | . | . | . | . | V | . | . | . | . | . | . | . | . | . | . | . |
| 21-225_31B8_LC | . | . | . | . | . | G | . | . | . | . | . | . | . | . | . | . | . | . | . | . | . | . | . | . | . | . |
| 21-225_31H3_LC | R | . | . | . | . | . | . | . | . | . | . | . | . | . | . | P | . | . | . | . | . | . | . | . | . | . |
| 21-225_31H5_LC | . | . | I | . | . | . | . | . | . | . | . | . | . | . | . | . | . | . | . | . | . | . | . | . | . | . |
| 21-225_32G1_LC | . | . | . | . | . | . | . | . | . | . | . | . | . | . | . | . | . | . | . | . | . | . | . | . | . | . |
| 21-225_32G12_LC | . | . | . | . | . | . | . | . | . | . | . | . | . | . | . | . | . | . | . | . | . | . | . | . | . | . |
| 21-225_33A4_LC | E | . | . | . | . | . | . | . | . | . | . | . | . | . | . | . | . | . | . | R | . | . | . | . | . | . |
| 21-225_33A7_LC | . | . | . | . | . | V | . | . | . | . | . | . | . | . | . | . | . | . | . | . | . | . | . | . | . | . |
| 21-225_33B1_LC | . | . | . | . | . | T | . | . | . | . | . | . | . | . | . | . | . | . | . | . | . | . | . | . | . | . |
| 21-225_33B8_LC | . | . | . | . | . | . | . | . | . | . | . | . | . | . | . | . | . | . | . | . | . | . | . | . | . | . |
| 21-225_33F1_LC | . | . | . | . | . | . | . | . | . | . | . | . | . | . | . | . | . | . | . | . | . | . | . | . | . | . |
| 21-225_33G7_LC | . | . | . | . | . | . | . | . | . | . | . | . | . | . | . | . | . | . | . | . | . | . | . | . | . | . |
| 21-225_34A6_LC | . | . | . | . | F | . | . | . | . | . | . | . | . | . | . | . | . | . | . | . | . | . | . | . | . | . |
| 21-225_34D11_LC | . | . | . | . | . | . | . | . | . | . | . | . | . | . | . | . | . | . | . | . | . | . | . | . | . | . |
| 21-225_34G8_LC | . | . | . | . | . | . | . | . | . | . | . | . | . | . | . | . | . | . | . | . | . | . | . | . | . | . |
| 21-225_34G9_LC | . | . | . | . | . | . | . | . | . | . | . | . | . | . | . | . | . | . | . | . | . | . | . | . | . | . |
| 21-225_34H11_LC | . | . | . | . | . | . | . | . | . | . | . | . | . | . | T | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | R | L | I | Y | A | - | - | - | - | - | - | - | - | A | S | S | L | Q | S | G | V | P | S | R | F |
| 21-225_35A6_LC | . | . | . | . | . | . | . | . | . | . | . | . | . | . | T | . | . | . | . | . | . | . | . | . | . | . |
| 21-225_35B7_LC | E | . | . | . | . | . | . | . | . | . | . | . | . | . | . | . | . | . | . | . | . | . | . | . | . | . |
| 21-225_35D1_LC | . | . | . | . | . | . | . | . | . | . | . | . | . | . | . | . | . | . | . | . | . | . | . | . | . | . |
| 21-225_35F11_LC | . | . | . | . | . | . | . | . | . | . | . | . | . | . | . | . | . | . | . | . | . | . | . | . | . | . |
| 21-225_36A5_LC | . | . | . | . | F | . | . | . | . | . | . | . | . | . | . | . | . | . | . | . | . | . | . | . | . | . |
| 21-225_36C5_LC | . | . | . | . | . | . | . | . | . | . | . | . | . | . | . | . | . | . | . | . | . | . | . | . | . | . |
| 21-225_43C3_LC | . | . | . | . | . | . | . | . | . | . | . | . | . | . | . | . | . | . | . | . | . | . | . | . | . | . |
| 21-225_44C3_LC | . | . | . | . | . | . | . | . | . | . | . | . | . | . | . | . | . | . | . | . | . | . | . | . | . | . |
| 21-225_44E12_LC | . | . | . | . | . | . | . | . | . | . | . | . | . | . | . | . | . | . | . | . | . | . | . | . | . | . |
| 21-225_46B1_LC | . | . | . | . | . | . | . | . | . | . | . | . | . | . | . | . | . | . | . | G | . | . | . | . | . | . |
| 21-225_46C6_LC | . | . | . | . | . | . | . | . | . | . | . | . | . | . | . | . | . | . | . | . | . | . | . | . | . | . |
| 21-225_46D8_LC | . | . | . | . | . | . | . | . | . | . | . | . | . | . | . | . | . | . | . | . | . | . | . | . | . | . |
| 21-225_46F3_LC | . | . | . | . | . | . | . | . | . | . | . | . | . | . | . | . | . | . | . | . | . | . | . | . | . | . |
| 21-225_47A1_LC | . | . | . | . | . | . | . | . | . | . | . | . | . | . | . | F | . | . | . | . | . | . | . | . | . | . |
| 21-225_47A5_LC | . | . | . | . | . | . | . | . | . | . | . | . | . | . | . | . | . | . | . | . | . | . | . | . | . | . |
| 21-225_48D12_LC | . | . | . | . | . | . | . | . | . | . | . | . | . | . | . | . | T | . | . | . | . | . | . | . | . | I |
| 21-225_48H4_LC | . | . | . | . | . | . | . | . | . | . | . | . | . | . | . | . | . | . | . | . | . | . | . | . | . | . |
| 21-225_49A1_LC | . | . | . | . | . | . | . | . | . | . | . | . | . | . | . | . | . | . | . | . | . | . | . | . | . | . |
| 21-225_49C6_LC | . | . | . | . | . | . | . | . | . | . | . | . | . | . | . | . | . | . | . | . | . | F | . | . | . | . |
| 21-225_4C8_LC | . | . | . | . | . | . | . | . | . | . | . | . | . | . | . | . | . | . | . | . | . | . | . | . | . | . |
| 21-225_4E10_LC | . | . | . | . | . | . | . | . | . | . | . | . | . | . | . | . | . | . | . | . | . | . | . | . | . | . |
| 21-225_4F12_LC | . | . | . | . | . | . | . | . | . | . | . | . | . | . | . | . | . | . | . | . | . | . | . | . | . | . |
| 21-225_4F4_LC | . | . | . | . | . | . | . | . | . | . | . | . | . | . | . | . | . | V | . | T | . | . | . | . | . | . |
| 21-225_4H4_LC | . | . | . | . | . | . | . | . | . | . | . | . | . | . | . | . | . | V | . | . | . | . | . | . | . | . |
| 21-225_50G10_LC | . | . | . | . | . | . | . | . | . | . | . | . | . | . | . | . | . | . | . | . | . | . | . | . | . | . |
| 21-225_51F11_LC | . | . | . | . | . | . | . | . | . | . | . | . | . | . | . | . | . | . | . | . | . | . | . | . | . | . |
| 21-225_52B2_LC | R | . | . | . | . | . | . | . | . | . | . | . | . | . | . | . | F | . | . | . | . | . | . | . | T | . |
| 21-225_53D2_LC | . | . | . | . | . | . | . | . | . | . | . | . | . | . | . | . | . | . | . | . | . | . | . | . | . | . |
| 21-225_53E6_LC | . | . | . | . | . | . | . | . | . | . | . | . | . | . | . | . | N | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | R | L | I | Y | A | - | - | - | - | - | - | - | - | A | S | S | L | Q | S | G | V | P | S | R | F |
| 21-225_54C6_LC | . | . | . | . | . | . | . | . | . | . | . | . | . | . | . | . | N | . | . | . | . | . | . | . | . | . |
| 21-225_55B8_LC | . | . | . | . | H | . | . | . | . | . | . | . | . | . | . | F | R | . | . | . | . | . | . | . | . | . |
| 21-225_55H6_LC | . | . | . | . | . | G | . | . | . | . | . | . | . | . | . | . | . | . | . | . | . | . | . | . | . | . |
| 21-225_56F1_LC | . | . | . | . | N | . | . | . | . | . | . | . | . | . | . | . | . | . | . | . | . | . | . | . | . | . |
| 21-225_59E6_LC | . | . | . | . | . | . | . | . | . | . | . | . | . | . | . | . | . | . | . | . | . | . | . | . | . | . |
| 21-225_5H2_LC | . | . | . | . | . | . | . | . | . | . | . | . | . | . | . | . | . | . | . | . | . | . | . | . | . | . |
| 21-225_5H7_LC | . | . | . | . | . | V | . | . | . | . | . | . | . | . | T | . | . | . | . | G | . | . | . | . | . | . |
| 21-225_60E8_LC | . | . | . | . | . | . | . | . | . | . | . | . | . | . | . | . | . | . | . | . | . | . | . | . | . | . |
| 21-225_62E2_LC | . | . | . | . | . | . | . | . | . | . | . | . | . | . | . | . | R | . | . | . | . | . | . | . | . | . |
| 21-225_62E4_LC | . | . | . | . | . | . | . | . | . | . | . | . | . | . | . | F | . | . | . | . | . | . | . | . | . | . |
| 21-225_64E1_LC | . | . | . | . | . | . | . | . | . | . | . | . | . | . | . | . | . | . | . | . | . | . | . | . | . | . |
| 21-225_68D12_LC | . | . | . | . | . | . | . | . | . | . | . | . | . | . | . | . | . | . | . | . | . | . | . | . | . | . |
| 21-225_68F11_LC | . | . | . | . | . | . | . | . | . | . | . | . | . | . | . | . | . | . | . | . | . | . | . | . | . | . |
| 21-225_6A6_LC | . | . | . | . | . | . | . | . | . | . | . | . | . | . | . | . | . | . | . | . | . | . | . | . | . | . |
| 21-225_6C12_LC | . | . | . | . | . | . | . | . | . | . | . | . | . | . | . | . | . | . | E | . | . | . | . | . | . | . |
| 21-225_6D4_LC | . | . | . | . | . | . | . | . | . | . | . | . | . | . | . | . | . | . | . | . | . | . | . | . | . | . |
| 21-225_6G12_LC | . | . | . | . | . | . | . | . | . | . | . | . | . | . | . | . | T | . | . | . | . | . | . | . | . | . |
| 21-225_70E12_LC | . | . | . | . | . | . | . | . | . | . | . | . | . | . | . | . | . | . | . | . | . | . | . | . | . | . |
| 21-225_70E8_LC | . | . | . | . | . | . | . | . | . | . | . | . | . | . | . | . | . | . | . | . | . | . | . | . | . | . |
| 21-225_73G6_LC | . | . | . | . | . | . | . | . | . | . | . | . | . | . | . | . | . | . | . | . | . | . | . | . | . | . |
| 21-225_74A1_LC | . | . | . | . | . | . | . | . | . | . | . | . | . | . | . | . | . | . | . | . | . | . | . | . | . | . |
| 21-225_74D7_LC | . | . | . | . | . | . | . | . | . | . | . | . | . | . | . | . | . | . | . | . | . | . | . | . | . | . |
| 21-225_74E4_LC | . | . | . | . | . | . | . | . | . | . | . | . | . | . | . | . | T | . | . | . | . | . | . | . | . | . |
| 21-225_75H7_LC | Q | . | . | . | . | D | . | . | . | . | . | . | . | . | . | . | . | . | . | . | . | . | . | . | . | . |
| 21-225_7C4_LC | . | . | . | . | . | . | . | . | . | . | . | . | . | . | . | . | . | . | . | . | . | . | . | . | . | . |
| 21-225_7E9_LC | . | . | . | . | . | T | . | . | . | . | . | . | . | . | . | . | . | . | . | . | . | . | . | . | . | . |
| 21-225_7F4_LC | . | . | . | . | . | . | . | . | . | . | . | . | . | . | . | . | N | . | . | . | . | . | . | . | . | . |
| 21-225_7H11_LC | . | . | I | . | . | V | . | . | . | . | . | . | . | . | . | C | . | F | . | . | . | . | . | . | . | . |
| 21-225_7H7_LC | . | . | . | . | . | . | . | . | . | . | . | . | . | . | . | . | . | . | . | . | . | . | T | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | R | L | I | Y | A | - | - | - | - | - | - | - | - | A | S | S | L | Q | S | G | V | P | S | R | F |
| 21-225_8C9_LC | . | . | . | . | . | . | . | . | . | . | . | . | . | . | . | . | . | . | . | . | . | . | . | . | . | . |
| 21-225_8D12_LC | . | . | . | . | . | . | . | . | . | . | . | . | . | . | . | . | . | . | . | . | . | . | . | . | . | . |
| 21-225_8F11_LC | . | . | . | . | F | D | . | . | . | . | . | . | . | . | . | . | . | V | . | . | . | . | . | . | . | . |
| 21-225_8H7_LC | . | . | . | . | . | . | . | . | . | . | . | . | . | . | . | . | . | . | . | . | . | . | . | . | . | . |
| 21-225_96A3_LC | . | . | . | . | . | . | . | . | . | . | . | . | . | . | . | . | T | . | . | . | . | . | . | . | . | . |
| 21-225_9E8_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | P | E | D | F | A | T | Y |
| 21-225_10G5_LC | . | . | . | . | . | . | . | . | . | . | . | . | . | . | . | . | . | . | . | . | . | . | . | . | . | . |
| 21-225_10H12_LC | . | . | . | . | . | . | . | . | . | . | . | . | . | . | . | . | . | . | . | . | . | . | . | . | . | . |
| 21-225_11F10_LC | . | . | . | . | . | . | . | . | . | . | . | . | . | . | V | . | . | . | . | . | . | . | . | . | . | . |
| 21-225_11F5_LC | . | . | . | . | . | . | . | . | . | . | . | . | . | . | . | . | . | . | . | . | . | . | . | . | I | . |
| 21-225_11G7_LC | . | . | . | . | . | . | . | . | . | . | . | . | . | . | . | . | . | . | . | . | . | . | . | . | . | . |
| 21-225_12A1_LC | . | . | . | . | . | . | . | . | . | . | . | . | . | . | . | . | . | . | . | . | . | . | . | . | . | . |
| 21-225_13E6_LC | . | . | . | . | . | . | . | . | . | . | . | . | . | . | . | . | . | . | . | . | . | . | . | . | . | . |
| 21-225_13H3_LC | . | . | . | . | . | . | . | . | . | . | . | . | . | . | . | . | . | . | . | . | . | . | . | . | . | . |
| 21-225_13H5_LC | . | . | . | . | . | . | . | . | . | . | . | I | . | . | . | . | . | . | . | . | . | . | . | . | . | . |
| 21-225_146F1_LC | . | . | G | . | . | . | . | . | . | . | . | . | . | . | . | . | . | . | . | . | . | . | . | . | . | . |
| 21-225_146G11_LC | . | . | . | . | . | . | . | . | . | . | . | . | . | . | . | . | . | . | . | . | . | . | . | . | . | . |
| 21-225_146H9_LC | . | . | . | . | . | . | . | . | . | . | . | . | . | . | . | . | . | . | . | . | . | . | . | . | . | . |
| 21-225_148E11_LC | T | . | . | . | . | . | . | . | . | . | . | . | F | . | . | . | . | V | . | . | . | . | . | . | . | . |
| 21-225_148F12_LC | . | . | . | . | . | . | . | . | . | . | . | . | . | . | . | . | . | . | . | . | . | . | . | . | . | . |
| 21-225_149F12_LC | . | . | . | . | . | . | . | . | . | . | . | . | . | . | . | . | . | . | . | . | . | . | . | . | I | . |
| 21-225_149G1_LC | . | . | . | . | . | . | . | . | . | . | . | . | . | . | . | . | . | . | . | . | . | . | . | . | . | . |
| 21-225_149G5_LC | . | . | . | . | . | . | . | . | . | . | . | . | . | . | . | . | . | . | . | . | . | . | . | . | . | . |
| 21-225_14B1_LC1_LC | . | . | . | . | . | . | . | . | . | . | . | . | . | . | . | . | . | . | . | . | . | . | . | . | . | . |
| 21-225_14B2_LC | . | . | . | . | . | . | . | . | . | . | . | . | . | . | . | . | . | . | . | . | . | . | . | . | . | . |
| 21-225_14C2_LC | . | . | . | . | . | . | . | . | . | . | . | . | . | . | . | . | . | V | . | R | . | . | . | . | . | . |
| 21-225_14E3_LC | I | . | . | . | C | . | . | . | . | . | . | . | . | . | . | . | . | . | . | R | . | . | . | . | A | . |
| 21-225_150E7_LC | . | . | . | . | . | . | . | . | . | D | . | . | . | . | . | . | . | . | . | . | . | . | . | . | A | . |
| 21-225_152H9_LC | T | . | . | . | . | . | . | . | . | . | . | . | F | . | . | . | . | . | . | . | . | . | . | . | . | . |
| 21-225_156G1_LC | . | . | . | . | . | . | . | . | . | . | . | . | F | . | . | . | . | . | . | . | . | . | . | . | . | . |
| 21-225_158H5_LC | . | . | . | . | C | . | . | . | . | . | . | . | . | . | . | . | . | V | . | R | . | . | . | . | S | . |
| 21-225_15A1_LC | . | . | . | . | . | . | . | . | . | . | . | . | . | . | V | . | . | . | . | . | . | . | . | . | S | . |
| 21-225_15C9_LC | . | . | . | . | . | . | . | . | . | . | . | . | . | . | V | . | . | . | . | . | . | . | . | . | . | . |
| 21-225_15E3_LC | . | . | . | R | . | . | . | . | . | . | . | . | . | . | . | . | . | . | . | . | . | . | L | . | A | . |
| 21-225_15G7_LC | . | . | . | . | F | . | . | . | . | . | . | . | . | . | . | . | . | . | . | . | . | . | . | . | . | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | P | E | D | F | A | T | Y |
| 21-225_15H1_LC | N | . | . | . | . | . | . | . | . | . | . | . | . | . | . | . | . | . | . | . | . | . | . | . | . | . |
| 21-225_15H10_LC | . | . | . | . | . | . | . | . | . | . | . | . | . | . | . | . | . | . | . | . | . | . | . | . | S | . |
| 21-225_15H8_LC | . | . | . | . | . | . | . | . | . | . | . | . | . | . | . | . | . | . | . | . | . | . | . | . | . | . |
| 21-225_160B10_LC | . | . | . | . | . | . | . | . | . | . | . | . | . | . | . | . | N | . | . | . | . | . | . | . | . | . |
| 21-225_160H3_LC | . | . | . | . | . | . | . | . | . | . | . | . | . | . | . | . | . | . | . | . | . | . | . | . | . | . |
| 21-225_169B1_LC | . | . | . | . | . | . | . | . | . | . | . | . | . | . | . | . | G | . | . | . | . | . | . | . | . | . |
| 21-225_169F9_LC | . | . | . | . | . | . | . | . | . | . | . | . | . | . | . | . | . | . | . | . | . | . | . | . | . | . |
| 21-225_16A1_LC | . | . | . | . | . | . | . | . | . | . | . | . | . | . | . | . | . | . | . | . | . | . | . | . | . | . |
| 21-225_16B7_LC | . | . | . | . | . | . | . | . | . | . | . | . | . | . | . | . | . | . | . | . | . | . | . | . | . | . |
| 21-225_16E12_LC | . | . | . | . | . | . | . | . | . | . | . | . | . | . | . | . | . | . | . | . | . | . | . | . | . | . |
| 21-225_16F11_LC | . | . | . | . | . | . | . | . | . | . | . | . | . | . | . | . | . | . | . | . | . | . | . | . | . | . |
| 21-225_16F4_LC | . | . | . | . | . | . | . | . | . | . | . | . | I | . | . | . | . | V | . | . | . | . | . | . | . | . |
| 21-225_170D11_LC | . | . | . | . | . | . | . | . | . | . | . | . | . | . | . | . | . | . | . | . | . | . | . | . | . | . |
| 21-225_170D5_LC | . | . | . | . | . | . | . | . | . | . | . | . | . | . | . | . | . | . | . | . | . | . | . | . | . | . |
| 21-225_170G4_LC | . | . | . | . | . | . | . | . | . | . | . | . | . | . | . | . | . | . | . | . | . | . | . | . | . | . |
| 21-225_170G5_LC | . | . | . | . | . | . | . | . | . | . | . | . | . | . | . | . | . | . | . | . | . | . | . | . | . | . |
| 21-225_171A4_LC | . | . | . | . | . | . | . | . | . | . | . | . | . | . | . | . | . | . | . | . | . | . | . | . | . | . |
| 21-225_171C3_LC | . | . | . | . | . | . | . | . | . | . | . | . | . | . | . | . | . | . | . | . | . | . | . | . | . | . |
| 21-225_172B3_LC | . | . | . | . | . | . | . | . | . | . | . | . | . | . | . | . | G | . | . | . | . | . | . | . | . | . |
| 21-225_172G8_LC | . | . | . | . | . | . | . | . | . | . | . | . | . | . | . | . | . | . | . | . | . | . | . | . | . | . |
| 21-225_173H12_LC | . | . | . | . | . | . | . | . | . | . | . | . | . | . | . | . | . | . | . | . | . | . | . | . | S | . |
| 21-225_175G1_LC | . | . | . | . | . | . | . | . | . | . | . | . | . | . | . | . | . | . | . | . | . | . | . | . | . | . |
| 21-225_176B11_LC | . | . | . | . | . | . | . | . | . | . | . | . | . | . | L | . | . | . | . | . | . | . | . | . | . | . |
| 21-225_178B10_LC | . | . | . | . | . | . | . | . | . | . | . | . | . | . | . | . | . | . | . | . | . | . | . | . | . | . |
| 21-225_17A10_LC | . | . | . | . | . | . | . | . | . | . | . | . | . | . | . | . | . | . | . | . | . | . | . | . | . | . |
| 21-225_17B5_LC | . | . | . | . | . | . | . | . | . | . | . | S | F | . | . | . | . | . | . | . | . | . | . | . | . | . |
| 21-225_17F9_LC | . | . | . | . | . | . | . | . | . | . | . | . | . | . | . | N | . | . | . | . | . | . | . | . | . | . |
| 21-225_17H12_LC | . | . | I | . | . | . | . | . | . | . | . | . | . | . | . | T | G | . | . | . | . | . | . | . | . | . |
| 21-225_17H8_LC | . | . | . | . | . | . | . | . | . | . | . | . | . | . | . | . | . | . | . | . | . | . | . | T | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | P | E | D | F | A | T | Y |
| 21-225_18A1_LC | . | . | . | . | . | . | . | . | . | . | . | . | . | . | . | . | . | . | . | . | . | . | . | . | . | . |
| 21-225_18A3_LC | N | . | . | . | . | . | . | . | . | . | . | . | . | . | . | . | . | . | . | . | . | . | . | . | . | . |
| 21-225_18C6_LC | . | . | . | . | . | . | . | . | . | . | . | . | . | . | . | . | . | . | . | . | . | . | . | . | . | . |
| 21-225_18E8_LC | . | . | . | . | . | . | . | . | . | . | . | . | . | . | . | . | . | . | . | . | . | . | . | . | . | . |
| 21-225_190C8_LC | . | . | . | . | . | . | . | . | . | . | . | . | . | . | . | . | . | . | . | . | . | . | . | E | . | . |
| 21-225_193A5_LC | . | . | . | . | F | . | . | . | . | . | . | . | I | . | . | . | . | V | . | . | . | . | . | E | . | . |
| 21-225_193E7_LC | . | . | . | . | . | . | . | . | . | . | . | . | . | . | . | . | . | . | . | . | . | . | . | . | . | . |
| 21-225_196C10_LC | . | . | . | . | . | . | . | . | . | . | . | . | . | . | . | . | . | . | . | . | . | . | . | . | . | . |
| 21-225_199E3_LC | . | . | . | . | . | . | . | . | . | . | . | . | . | . | . | . | . | . | . | . | . | . | . | . | . | . |
| 21-225_19D11_LC | . | . | . | . | . | . | . | . | . | . | . | . | . | . | . | . | . | . | . | . | . | . | . | . | . | . |
| 21-225_19E1_LC | . | . | . | . | . | . | . | . | . | . | . | . | . | . | . | . | . | . | . | . | . | . | . | . | . | . |
| 21-225_19F3_LC | . | . | . | . | . | . | . | . | . | . | . | . | . | . | . | . | . | . | . | . | . | . | . | . | . | . |
| 21-225_1A2_LC | . | . | . | . | . | . | . | . | . | . | . | . | . | . | . | . | . | . | . | . | . | . | . | . | . | . |
| 21-225_1C12_LC | . | . | . | . | . | . | . | . | . | . | . | . | . | . | . | . | . | . | . | . | . | . | . | . | . | . |
| 21-225_1F2_LC | . | . | . | . | . | . | . | . | . | . | . | . | . | . | . | . | . | . | . | . | . | . | . | . | I | . |
| 21-225_200F12_LC | . | . | . | . | . | . | . | . | . | . | . | . | . | . | . | . | . | . | . | . | . | . | . | . | . | . |
| 21-225_200H9_LC | . | . | . | . | . | . | . | . | . | . | . | . | . | . | . | . | . | . | . | . | . | . | . | . | . | . |
| 21-225_201A1_LC | . | . | . | . | . | . | . | . | . | . | . | . | . | . | . | . | . | . | . | . | . | . | . | . | . | . |
| 21-225_201A10_LC | . | . | . | . | . | . | . | . | . | . | . | . | . | . | . | . | . | . | . | . | . | . | . | . | . | . |
| 21-225_204H8_LC | . | . | R | . | . | . | . | . | . | . | . | . | . | . | V | . | . | . | . | . | . | . | . | . | . | . |
| 21-225_205H8_LC | . | . | . | . | . | . | . | . | . | . | . | . | . | . | . | . | . | . | . | . | . | . | S | . | A | . |
| 21-225_20A10_LC | . | . | . | . | . | . | . | . | . | . | . | . | . | . | . | . | . | . | . | . | . | . | . | . | . | . |
| 21-225_20A7_LC | . | . | . | . | . | . | . | . | . | . | . | S | . | . | . | . | . | . | . | . | . | . | . | . | . | . |
| 21-225_20C2_LC | . | . | . | . | . | . | . | . | . | . | . | . | . | . | . | . | . | . | . | . | . | . | . | . | . | . |
| 21-225_20C8_LC | . | . | . | . | . | . | . | . | . | . | . | . | . | . | . | . | . | . | . | . | . | . | . | . | . | . |
| 21-225_20D5_LC | . | . | . | . | . | . | . | . | . | . | . | . | . | . | . | . | . | . | . | . | . | . | . | . | . | . |
| 21-225_20E12_LC | . | . | . | . | . | . | . | . | . | . | . | . | . | . | . | . | . | . | . | . | . | . | . | . | . | . |
| 21-225_20E5_LC | . | . | . | . | . | . | . | . | . | . | . | . | . | . | . | . | . | . | . | . | . | . | . | . | . | . |
| 21-225_20G2_LC | . | . | . | . | . | . | . | . | . | . | . | . | . | . | . | . | . | . | . | . | . | . | . | . | S | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | P | E | D | F | A | T | Y |
| 21-225_20G9_LC | . | . | . | . | . | . | . | . | . | . | . | . | . | . | . | . | . | . | . | . | . | . | . | . | . | . |
| 21-225_20H10_LC | . | . | . | . | . | . | . | . | . | . | . | . | . | . | . | . | . | . | . | . | . | . | . | . | . | . |
| 21-225_20H12_LC | . | . | . | . | . | . | . | . | . | . | . | . | . | . | . | . | . | . | . | . | . | . | . | . | . | . |
| 21-225_21E5_LC | . | . | . | . | . | . | . | . | . | . | . | . | . | . | . | . | . | . | . | . | . | . | . | . | . | . |
| 21-225_21F3_LC | . | . | . | . | . | . | . | . | . | D | . | I | . | . | . | . | . | . | . | . | . | . | . | . | . | . |
| 21-225_21G7_LC | N | . | . | . | . | . | . | . | . | . | . | . | . | . | . | . | . | . | . | . | . | . | . | . | . | . |
| 21-225_21H3_LC | . | . | . | . | . | . | . | . | . | . | . | . | . | . | . | . | . | . | . | A | . | . | L | . | I | . |
| 21-225_224A1_LC | . | . | . | . | . | . | . | . | . | . | . | . | . | . | . | . | . | . | . | . | . | . | . | . | . | . |
| 21-225_224B1_LC | . | . | . | . | . | . | . | . | . | . | . | . | . | . | . | . | . | . | . | . | . | . | . | . | . | . |
| 21-225_224C3_LC | . | . | . | . | . | . | . | . | . | . | . | . | . | . | . | . | . | . | . | . | . | . | . | . | . | . |
| 21-225_224D10_LC | . | . | . | . | . | . | . | . | . | . | . | . | . | . | . | . | . | . | . | . | . | . | . | . | . | . |
| 21-225_22B7_LC | . | . | . | . | . | . | . | . | . | . | . | . | . | . | . | . | . | . | . | . | . | . | . | . | . | . |
| 21-225_22C1_LC | . | . | . | . | . | . | . | . | . | . | . | . | . | . | . | . | . | . | . | . | . | . | . | . | . | . |
| 21-225_22D12_LC | . | . | . | . | . | . | . | . | . | D | . | . | . | . | . | . | . | . | . | . | . | . | . | . | . | . |
| 21-225_22F2_LC | . | . | . | . | . | . | . | . | . | . | . | . | . | . | . | . | . | . | . | . | . | . | . | . | . | . |
| 21-225_22F9_LC | . | . | . | . | . | . | . | . | . | . | . | . | . | . | . | . | . | . | . | . | . | . | . | . | . | . |
| 21-225_22G8_LC | . | . | . | . | . | . | . | . | . | . | . | . | . | S | . | . | . | . | . | . | . | . | . | . | . | . |
| 21-225_22G9_LC | . | . | . | . | . | . | . | . | . | . | . | . | . | . | . | . | . | . | . | . | . | . | . | . | . | . |
| 21-225_22H4_LC | . | . | . | . | . | . | . | . | . | . | . | . | . | . | . | . | . | . | . | . | . | . | . | . | L | . |
| 21-225_23A3_LC | . | . | . | . | . | . | . | . | . | . | . | . | . | . | . | . | . | . | . | . | . | . | . | . | I | . |
| 21-225_23C8_LC | . | . | . | . | . | . | . | . | . | . | . | . | . | . | . | . | . | . | . | . | . | . | . | V | I | . |
| 21-225_23D1_LC | . | . | . | . | . | . | . | . | . | . | . | . | . | . | V | . | . | . | . | . | . | . | . | . | . | . |
| 21-225_23H11_LC | . | . | . | . | . | . | . | . | . | . | . | . | . | . | . | . | . | . | . | . | . | . | . | . | . | . |
| 21-225_24E5_LC | . | . | . | . | . | . | . | . | . | . | . | . | . | . | . | . | . | . | . | . | . | . | . | . | . | . |
| 21-225_25E6_LC | R | . | . | . | . | . | . | . | . | . | . | . | . | . | . | N | . | . | . | . | . | . | . | . | . | . |
| 21-225_25H10_LC | . | . | . | . | . | . | . | . | . | . | . | . | . | . | . | . | . | . | . | . | . | . | . | . | . | . |
| 21-225_25H11_LC | . | . | . | . | . | . | . | . | . | . | . | . | . | . | . | . | . | . | . | . | . | . | . | . | . | . |
| 21-225_27C3_LC | . | . | . | . | . | . | . | . | . | . | . | . | . | . | . | . | . | . | . | . | . | . | . | . | . | . |
| 21-225_27F2_LC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | P | E | D | F | A | T | Y |
| 21-225_29B8_LC | . | . | . | R | . | . | . | . | . | . | . | . | . | . | . | . | . | . | . | . | . | . | . | . | . | . |
| 21-225_29G4_LC | . | . | . | . | . | . | . | . | . | . | . | . | . | . | . | . | . | . | . | . | . | . | . | . | . | . |
| 21-225_29G8_LC | . | . | . | . | . | . | . | . | . | . | . | . | . | . | . | . | . | . | . | . | . | . | . | . | . | . |
| 21-225_29H6_LC | . | . | . | . | . | . | . | . | . | . | . | . | . | . | . | . | . | . | . | . | . | . | . | . | . | . |
| 21-225_2A11_LC | . | . | . | . | . | . | . | . | . | D | . | . | . | . | . | . | . | . | . | . | . | . | . | . | . | . |
| 21-225_2B2_LC | . | . | . | . | . | . | . | . | . | . | . | . | . | . | . | . | . | . | . | . | . | . | L | . | . | . |
| 21-225_2G9_LC | . | . | . | . | F | . | . | . | . | . | . | . | . | . | . | . | . | . | . | L | . | . | . | . | . | . |
| 21-225_30D1_LC | . | . | . | . | . | . | . | . | . | . | . | . | . | . | . | . | . | . | . | . | . | . | . | T | . | . |
| 21-225_30E3_LC | . | . | . | . | . | . | . | . | . | . | . | . | . | . | . | . | . | . | . | . | . | . | . | . | . | . |
| 21-225_30E9_LC | . | . | . | . | . | . | . | . | . | . | . | . | . | . | . | . | . | . | . | . | . | . | . | . | . | . |
| 21-225_30F3_LC | . | . | . | . | . | . | . | . | . | D | . | . | . | . | . | . | . | . | . | . | . | . | . | . | S | . |
| 21-225_30G11_LC | . | . | . | . | . | . | . | . | . | . | . | . | . | . | . | G | . | . | . | . | . | . | . | . | . | . |
| 21-225_31A8_LC | . | . | . | . | . | . | . | . | . | . | . | . | . | . | . | . | . | . | . | . | . | . | . | . | . | . |
| 21-225_31B8_LC | . | . | . | . | . | . | . | . | . | . | . | . | . | . | . | . | . | . | . | . | . | . | . | . | . | . |
| 21-225_31H3_LC | . | . | . | . | . | . | . | . | . | . | . | . | . | . | . | . | . | . | . | . | . | . | . | . | . | . |
| 21-225_31H5_LC | . | V | . | . | . | . | . | . | . | . | . | . | F | . | . | . | . | M | . | . | D | . | . | S | N | . |
| 21-225_32G1_LC | . | . | . | . | . | . | . | . | . | . | . | . | . | . | . | . | . | . | . | . | . | . | . | . | . | . |
| 21-225_32G12_LC | . | . | . | . | . | . | . | . | . | . | . | . | . | . | . | . | . | . | . | . | . | . | . | . | . | . |
| 21-225_33A4_LC | . | . | . | . | . | . | . | . | . | . | . | . | . | . | . | . | . | . | . | . | . | . | . | . | R | . |
| 21-225_33A7_LC | . | . | . | . | . | . | . | . | . | . | . | . | . | . | . | . | . | . | . | . | . | . | . | . | A | . |
| 21-225_33B1_LC | . | . | . | . | . | . | . | . | . | . | . | . | . | . | . | . | . | . | . | . | . | . | . | . | . | . |
| 21-225_33B8_LC | . | . | . | . | . | . | . | . | . | . | . | . | . | . | . | . | . | . | . | . | . | . | . | . | . | . |
| 21-225_33F1_LC | . | . | . | . | . | . | . | . | . | . | . | . | . | . | . | . | . | . | . | . | . | . | . | . | I | . |
| 21-225_33G7_LC | . | . | . | . | . | . | . | . | . | . | . | . | . | . | . | . | . | . | . | . | . | . | . | . | . | . |
| 21-225_34A6_LC | . | . | . | . | . | . | . | . | . | . | . | . | . | . | V | . | . | . | . | . | . | . | . | . | . | . |
| 21-225_34D11_LC | . | . | . | . | . | . | . | . | . | . | . | . | . | . | V | I | . | . | . | . | . | . | . | . | . | . |
| 21-225_34G8_LC | . | . | . | . | . | . | . | . | . | . | . | . | . | . | . | . | . | . | . | . | . | . | . | . | . | . |
| 21-225_34G9_LC | T | . | . | . | . | . | . | . | . | . | . | . | . | . | . | . | . | . | . | . | . | A | . | . | I | . |
| 21-225_34H11_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | P | E | D | F | A | T | Y |
| 21-225_35A6_LC | . | . | . | . | . | . | . | . | . | . | . | . | . | . | . | . | . | . | . | . | . | . | . | . | . | . |
| 21-225_35B7_LC | . | . | . | . | . | . | . | . | . | . | . | . | . | . | . | . | . | . | . | . | . | . | . | . | . | . |
| 21-225_35D1_LC | . | . | . | . | . | . | . | . | . | . | . | . | . | . | . | . | . | . | . | . | . | . | . | . | . | . |
| 21-225_35F11_LC | . | . | . | . | . | . | . | . | . | . | . | . | . | . | . | . | . | . | . | . | . | . | . | . | . | . |
| 21-225_36A5_LC | . | . | . | . | . | . | . | . | . | . | . | . | . | . | . | . | . | . | . | . | . | . | . | . | . | . |
| 21-225_36C5_LC | T | . | . | . | . | . | . | . | . | . | . | . | . | . | . | . | . | V | . | . | . | . | . | . | . | . |
| 21-225_43C3_LC | . | . | . | . | . | . | . | . | . | . | . | . | . | . | . | . | . | . | . | . | . | . | . | . | . | . |
| 21-225_44C3_LC | . | . | G | . | . | . | . | . | . | . | . | . | . | . | . | . | . | . | . | . | . | . | . | . | . | . |
| 21-225_44E12_LC | . | . | . | . | . | . | . | . | . | . | . | . | . | . | . | . | . | . | . | . | . | . | . | . | . | . |
| 21-225_46B1_LC | . | . | . | . | . | . | . | . | . | . | . | . | . | . | . | . | . | . | . | . | . | . | . | . | . | . |
| 21-225_46C6_LC | . | . | . | . | . | . | . | . | . | . | . | . | . | . | . | . | . | . | . | . | . | . | . | . | . | . |
| 21-225_46D8_LC | . | . | . | . | . | . | . | . | . | . | . | S | . | . | . | . | . | . | . | . | . | . | . | . | . | . |
| 21-225_46F3_LC | . | . | . | . | . | . | . | . | . | . | . | S | . | . | . | . | . | . | . | . | . | . | . | . | . | . |
| 21-225_47A1_LC | . | . | . | R | . | . | . | . | . | . | . | . | . | . | . | . | . | . | . | . | . | . | . | . | . | . |
| 21-225_47A5_LC | . | . | . | . | . | . | . | . | . | . | . | . | . | . | . | . | . | . | . | . | . | . | . | . | . | . |
| 21-225_48D12_LC | . | . | . | . | . | . | . | . | . | . | . | . | . | . | . | . | . | . | . | . | . | . | . | . | . | . |
| 21-225_48H4_LC | . | . | . | . | . | . | . | . | . | . | . | . | . | . | . | . | . | . | . | . | . | . | . | . | . | . |
| 21-225_49A1_LC | . | . | . | . | . | . | . | . | . | . | . | . | . | . | . | . | . | . | . | . | . | . | . | . | . | . |
| 21-225_49C6_LC | . | . | . | . | . | . | . | . | . | . | . | . | . | . | . | . | . | . | . | . | . | . | . | . | . | . |
| 21-225_4C8_LC | . | . | . | V | . | . | . | . | . | . | . | . | . | . | . | . | . | . | . | . | . | . | . | . | . | . |
| 21-225_4E10_LC | . | . | . | . | F | . | . | . | . | . | . | . | . | . | . | . | . | . | . | L | . | . | . | . | . | . |
| 21-225_4F12_LC | . | . | G | . | . | . | . | . | . | I | . | . | . | . | . | . | . | . | . | . | . | . | . | . | . | . |
| 21-225_4F4_LC | . | . | . | . | . | . | . | . | . | . | . | . | . | . | . | . | . | . | . | . | . | . | . | . | I | . |
| 21-225_4H4_LC | . | . | . | . | . | . | . | . | . | . | . | . | . | . | . | . | . | . | . | . | . | . | . | . | I | . |
| 21-225_50G10_LC | . | . | . | . | . | . | . | . | . | . | . | . | . | . | . | . | . | . | . | . | . | . | . | . | . | . |
| 21-225_51F11_LC | . | . | . | . | . | . | . | . | . | . | . | . | . | . | . | . | . | . | . | . | . | . | . | . | . | . |
| 21-225_52B2_LC | . | . | . | . | . | . | . | . | . | . | . | . | . | . | . | . | . | . | . | . | . | . | . | . | . | . |
| 21-225_53D2_LC | . | . | . | . | . | . | . | . | . | . | . | . | . | . | . | . | . | . | . | . | . | . | . | . | . | . |
| 21-225_53E6_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | P | E | D | F | A | T | Y |
| 21-225_54C6_LC | . | . | . | . | . | . | . | . | . | . | . | . | . | . | . | . | . | . | . | . | . | . | . | . | . | . |
| 21-225_55B8_LC | . | . | . | . | . | . | . | . | . | . | . | . | . | . | . | . | . | . | . | . | . | . | . | . | . | . |
| 21-225_55H6_LC | . | . | . | . | . | . | . | . | . | . | . | . | . | . | . | . | . | . | . | . | . | . | . | . | A | . |
| 21-225_56F1_LC | . | . | . | . | . | . | . | . | . | . | . | . | . | . | . | . | . | . | . | . | . | . | . | . | . | . |
| 21-225_59E6_LC | . | . | . | . | . | . | . | . | . | . | . | . | . | . | . | . | . | . | . | . | . | . | . | . | . | . |
| 21-225_5H2_LC | . | . | . | . | . | . | . | . | . | . | . | S | . | . | . | . | . | . | . | . | . | . | . | . | . | . |
| 21-225_5H7_LC | . | . | . | . | . | . | . | . | . | . | . | . | . | . | . | . | . | . | . | . | . | . | . | . | . | . |
| 21-225_60E8_LC | . | . | . | . | . | . | . | . | . | . | . | . | . | . | . | . | . | . | . | . | . | . | . | . | . | . |
| 21-225_62E2_LC | . | . | . | . | . | . | . | . | . | . | . | . | . | . | . | . | . | . | . | . | . | . | . | . | . | . |
| 21-225_62E4_LC | . | . | . | . | . | . | . | . | . | . | . | . | . | . | . | . | . | . | . | . | . | . | . | . | . | . |
| 21-225_64E1_LC | . | . | . | K | . | . | . | . | . | . | . | . | . | . | . | . | . | . | . | . | . | . | L | . | . | . |
| 21-225_68D12_LC | . | . | . | . | . | . | . | . | . | . | . | . | . | . | . | . | . | . | . | . | . | . | . | . | . | . |
| 21-225_68F11_LC | . | . | . | . | . | . | . | . | P | . | . | . | . | S | . | . | . | . | . | . | . | . | . | . | . | . |
| 21-225_6A6_LC | . | . | . | . | . | . | . | . | . | . | . | . | . | . | . | . | . | . | . | . | . | . | . | . | . | . |
| 21-225_6C12_LC | . | . | . | . | . | . | . | . | . | . | . | . | . | . | . | . | . | . | . | . | . | . | . | . | . | . |
| 21-225_6D4_LC | . | . | . | . | . | . | . | . | . | . | . | . | . | . | . | . | . | . | . | . | . | . | . | . | . | . |
| 21-225_6G12_LC | . | . | . | . | . | . | . | . | . | . | . | . | . | . | . | . | . | . | . | . | . | . | . | . | . | . |
| 21-225_70E12_LC | . | . | . | . | . | . | . | . | . | . | . | . | . | . | . | . | . | . | . | . | . | . | . | . | . | . |
| 21-225_70E8_LC | . | . | . | . | . | . | . | . | . | . | . | . | . | . | . | . | . | . | . | . | . | . | . | . | . | . |
| 21-225_73G6_LC | . | . | . | . | . | . | . | . | . | . | . | . | . | . | . | . | . | . | . | . | . | . | . | . | . | . |
| 21-225_74A1_LC | . | . | . | . | . | . | . | . | . | . | . | . | . | . | . | . | . | . | . | . | . | . | . | . | . | . |
| 21-225_74D7_LC | . | . | . | . | F | . | . | . | . | . | . | . | . | . | . | . | . | . | . | . | . | . | . | . | . | . |
| 21-225_74E4_LC | . | . | . | . | . | . | . | . | . | . | . | . | . | . | . | . | G | . | . | . | . | . | . | . | . | . |
| 21-225_75H7_LC | . | . | . | . | . | . | . | . | . | . | . | . | . | . | . | . | . | V | . | . | . | . | . | G | V | . |
| 21-225_7C4_LC | . | . | . | . | . | . | . | . | . | . | . | . | . | . | . | . | . | . | . | . | . | . | . | . | . | . |
| 21-225_7E9_LC | . | . | . | . | . | . | . | . | . | . | . | . | . | . | . | . | . | . | . | . | . | . | . | . | . | . |
| 21-225_7F4_LC | . | . | . | . | . | . | . | . | . | . | . | . | . | . | . | . | . | . | . | . | . | . | . | . | . | . |
| 21-225_7H11_LC | . | . | . | . | Y | . | . | . | . | . | . | . | . | . | . | . | I | M | . | . | . | . | . | . | . | . |
| 21-225_7H7_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | P | E | D | F | A | T | Y |
| 21-225_8C9_LC | . | . | . | . | . | . | . | . | . | . | . | . | . | . | . | . | . | . | . | T | . | . | . | . | . | . |
| 21-225_8D12_LC | N | . | . | . | . | . | . | . | . | . | . | . | . | . | . | . | . | . | . | . | . | . | . | . | . | . |
| 21-225_8F11_LC | . | . | . | . | . | . | . | . | . | . | . | . | . | . | . | . | . | . | . | . | . | . | . | . | I | . |
| 21-225_8H7_LC | . | . | . | . | . | . | . | . | . | . | . | . | . | . | . | . | . | . | . | . | . | . | . | . | . | . |
| 21-225_96A3_LC | . | . | . | . | . | . | . | . | . | . | . | . | . | . | . | . | G | . | . | R | . | . | . | . | . | . |
| 21-225_9E8_LC | . | . | . | . | . | . | . | . | . | . | . | . | . | . | . | . | . | . | . | . | . | . | . | . | . | . |

# FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | L | Q | H | N | S | - | - | - | - | - | - | - | - | - | - | - | - | - | - | - | - | - | - | - |
| 21-225_10G5_LC | . | . | . | . | . | . | . | . | . | . | . | . | . | . | . | . | . | . | . | . | . | . | . | . | . | . |
| 21-225_10H12_LC | . | . | . | . | . | . | . | . | . | . | . | . | . | . | . | . | . | . | . | . | . | . | . | . | . | . |
| 21-225_11F10_LC | . | . | . | . | . | S | . | . | . | . | . | . | . | . | . | . | . | . | . | . | . | . | . | . | . | . |
| 21-225_11F5_LC | . | . | . | . | . | . | . | . | . | . | . | . | . | . | . | . | . | . | . | . | . | . | . | . | . | . |
| 21-225_11G7_LC | . | . | . | . | . | S | N | . | . | . | . | . | . | . | . | . | . | . | . | . | . | . | . | . | . | . |
| 21-225_12A1_LC | . | . | . | . | . | . | . | . | . | . | . | . | . | . | . | . | . | . | . | . | . | . | . | . | . | . |
| 21-225_13E6_LC | . | . | V | . | . | . | . | . | . | . | . | . | . | . | . | . | . | . | . | . | . | . | . | . | . | . |
| 21-225_13H3_LC | . | . | . | . | . | . | . | . | . | . | . | . | . | . | . | . | . | . | . | . | . | . | . | . | . | . |
| 21-225_13H5_LC | . | . | . | . | . | . | . | . | . | . | . | . | . | . | . | . | . | . | . | . | . | . | . | . | . | . |
| 21-225_146F1_LC | . | . | . | . | . | S | T | . | . | . | . | . | . | . | . | . | . | . | . | . | . | . | . | . | . | . |
| 21-225_146G11_LC | . | . | . | . | . | D | . | . | . | . | . | . | . | . | . | . | . | . | . | . | . | . | . | . | . | . |
| 21-225_146H9_LC | . | . | . | . | . | . | . | . | . | . | . | . | . | . | . | . | . | . | . | . | . | . | . | . | . | . |
| 21-225_148E11_LC | . | . | . | . | . | R | N | . | . | . | . | . | . | . | . | . | . | . | . | . | . | . | . | . | . | . |
| 21-225_148F12_LC | F | . | . | . | . | . | . | . | . | . | . | . | . | . | . | . | . | . | . | . | . | . | . | . | . | . |
| 21-225_149F12_LC | . | . | . | . | . | . | . | . | . | . | . | . | . | . | . | . | . | . | . | . | . | . | . | . | . | . |
| 21-225_149G1_LC | . | . | . | . | . | . | . | . | . | . | . | . | . | . | . | . | . | . | . | . | . | . | . | . | . | . |
| 21-225_149G5_LC | . | . | . | . | . | . | . | . | . | . | . | . | . | . | . | . | . | . | . | . | . | . | . | . | . | . |
| 21-225_14B1_LC1_LC | . | . | . | . | . | Y | N | . | . | . | . | . | . | . | . | . | . | . | . | . | . | . | . | . | . | . |
| 21-225_14B2_LC | . | . | . | . | . | . | . | . | . | . | . | . | . | . | . | . | . | . | . | . | . | . | . | . | . | . |
| 21-225_14C2_LC | . | . | V | . | . | . | . | . | . | . | . | . | . | . | . | . | . | . | . | . | . | . | . | . | . | . |
| 21-225_14E3_LC | . | S | V | . | . | Y | . | . | . | . | . | . | . | . | . | . | . | . | . | . | . | . | . | . | . | . |
| 21-225_150E7_LC | . | . | . | . | . | . | . | . | . | . | . | . | . | . | . | . | . | . | . | . | . | . | . | . | . | . |
| 21-225_152H9_LC | . | . | . | . | . | S | N | . | . | . | . | . | . | . | . | . | . | . | . | . | . | . | . | . | . | . |
| 21-225_156G1_LC | . | . | . | . | . | S | N | . | . | . | . | . | . | . | . | . | . | . | . | . | . | . | . | . | . | . |
| 21-225_158H5_LC | . | . | V | . | . | . | . | . | . | . | . | . | . | . | . | . | . | . | . | . | . | . | . | . | . | . |
| 21-225_15A1_LC | . | . | . | H | N | S | . | . | . | . | . | . | . | . | . | . | . | . | . | . | . | . | . | . | . | . |
| 21-225_15C9_LC | . | . | . | . | . | T | . | . | . | . | . | . | . | . | . | . | . | . | . | . | . | . | . | . | . | . |
| 21-225_15E3_LC | . | . | . | . | . | H | . | . | . | . | . | . | . | . | . | . | . | . | . | . | . | . | . | . | . | . |
| 21-225_15G7_LC | . | . | . | . | . | S | N | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | K_CDR3 | | | | | | | |
| CONSENSUS | Y | C | L | Q | H | N | S | - | - | - | - | - | - | - | - | - | - | - | - | - | - | - | - | - | - | - |
| 21-225_15H1_LC | . | . | . | . | . | S | . | . | . | . | . | . | . | . | . | . | . | . | . | . | . | . | . | . | . | . |
| 21-225_15H10_LC | . | . | . | . | . | . | . | . | . | . | . | . | . | . | . | . | . | . | . | . | . | . | . | . | . | . |
| 21-225_15H8_LC | . | . | . | . | . | . | . | . | . | . | . | . | . | . | . | . | . | . | . | . | . | . | . | . | . | . |
| 21-225_160B10_LC | . | . | . | . | . | S | . | . | . | . | . | . | . | . | . | . | . | . | . | . | . | . | . | . | . | . |
| 21-225_160H3_LC | . | . | . | . | . | S | N | . | . | . | . | . | . | . | . | . | . | . | . | . | . | . | . | . | . | . |
| 21-225_169B1_LC | . | . | . | . | . | Y | . | . | . | . | . | . | . | . | . | . | . | . | . | . | . | . | . | . | . | . |
| 21-225_169F9_LC | . | . | . | . | . | Y | . | . | . | . | . | . | . | . | . | . | . | . | . | . | . | . | . | . | . | . |
| 21-225_16A1_LC | . | . | . | H | . | S | N | . | . | . | . | . | . | . | . | . | . | . | . | . | . | . | . | . | . | . |
| 21-225_16B7_LC | . | . | . | . | . | . | . | . | . | . | . | . | . | . | . | . | . | . | . | . | . | . | . | . | . | . |
| 21-225_16E12_LC | . | . | . | . | . | . | . | . | . | . | . | . | . | . | . | . | . | . | . | . | . | . | . | . | . | . |
| 21-225_16F11_LC | . | . | . | . | . | . | . | . | . | . | . | . | . | . | . | . | . | . | . | . | . | . | . | . | . | . |
| 21-225_16F4_LC | . | . | I | . | . | . | . | . | . | . | . | . | . | . | . | . | . | . | . | . | . | . | . | . | . | . |
| 21-225_170D11_LC | . | . | . | . | . | Y | . | . | . | . | . | . | . | . | . | . | . | . | . | . | . | . | . | . | . | . |
| 21-225_170D5_LC | . | . | . | . | . | Y | . | . | . | . | . | . | . | . | . | . | . | . | . | . | . | . | . | . | . | . |
| 21-225_170G4_LC | . | . | . | . | . | Y | . | . | . | . | . | . | . | . | . | . | . | . | . | . | . | . | . | . | . | . |
| 21-225_170G5_LC | . | . | . | . | . | Y | . | . | . | . | . | . | . | . | . | . | . | . | . | . | . | . | . | . | . | . |
| 21-225_171A4_LC | . | . | . | . | . | Y | . | . | . | . | . | . | . | . | . | . | . | . | . | . | . | . | . | . | . | . |
| 21-225_171C3_LC | . | . | . | . | . | Y | . | . | . | . | . | . | . | . | . | . | . | . | . | . | . | . | . | . | . | . |
| 21-225_172B3_LC | . | . | . | . | . | Y | . | . | . | . | . | . | . | . | . | . | . | . | . | . | . | . | . | . | . | . |
| 21-225_172G8_LC | . | . | . | H | . | Y | . | . | . | . | . | . | . | . | . | . | . | . | . | . | . | . | . | . | . | . |
| 21-225_173H12_LC | . | . | . | . | . | Y | . | . | . | . | . | . | . | . | . | . | . | . | . | . | . | . | . | . | . | . |
| 21-225_175G1_LC | . | . | . | . | . | Y | . | . | . | . | . | . | . | . | . | . | . | . | . | . | . | . | . | . | . | . |
| 21-225_176B11_LC | . | . | . | . | . | Y | . | . | . | . | . | . | . | . | . | . | . | . | . | . | . | . | . | . | . | . |
| 21-225_178B10_LC | . | . | . | H | . | Y | . | . | . | . | . | . | . | . | . | . | . | . | . | . | . | . | . | . | . | . |
| 21-225_17A10_LC | . | . | . | . | . | . | . | . | . | . | . | . | . | . | . | . | . | . | . | . | . | . | . | . | . | . |
| 21-225_17B5_LC | . | . | . | . | . | . | . | . | . | . | . | . | . | . | . | . | . | . | . | . | . | . | . | . | . | . |
| 21-225_17F9_LC | . | . | . | . | . | . | T | . | . | . | . | . | . | . | . | . | . | . | . | . | . | . | . | . | . | . |
| 21-225_17H12_LC | . | . | . | . | . | Y | . | . | . | . | . | . | . | . | . | . | . | . | . | . | . | . | . | . | . | . |
| 21-225_17H8_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_20G9_LC | . | . | . | . | . | S | T | . | . | . | . | . | . | . | . | . | . | . | . | . | . | . | . | . | . | . |
| 21-225_20H10_LC | . | . | . | . | . | . | . | . | . | . | . | . | . | . | . | . | . | . | . | . | . | . | . | . | . | . |
| 21-225_20H12_LC | . | . | . | . | . | . | . | . | . | . | . | . | . | . | . | . | . | . | . | . | . | . | . | . | . | . |
| 21-225_21E5_LC | . | . | . | . | . | . | . | . | . | . | . | . | . | . | . | . | . | . | . | . | . | . | . | . | . | . |
| 21-225_21F3_LC | . | . | . | . | . | S | . | . | . | . | . | . | . | . | . | . | . | . | . | . | . | . | . | . | . | . |
| 21-225_21G7_LC | . | . | . | . | . | S | . | . | . | . | . | . | . | . | . | . | . | . | . | . | . | . | . | . | . | . |
| 21-225_21H3_LC | . | . | . | . | . | . | . | . | . | . | . | . | . | . | . | . | . | . | . | . | . | . | . | . | . | . |
| 21-225_224A1_LC | . | . | . | . | . | . | . | . | . | . | . | . | . | . | . | . | . | . | . | . | . | . | . | . | . | . |
| 21-225_224B1_LC | H | . | . | . | . | . | . | . | . | . | . | . | . | . | . | . | . | . | . | . | . | . | . | . | . | . |
| 21-225_224C3_LC | . | . | . | . | . | . | . | . | . | . | . | . | . | . | . | . | . | . | . | . | . | . | . | . | . | . |
| 21-225_224D10_LC | . | . | . | . | . | . | . | . | . | . | . | . | . | . | . | . | . | . | . | . | . | . | . | . | . | . |
| 21-225_22B7_LC | . | . | . | . | . | . | T | . | . | . | . | . | . | . | . | . | . | . | . | . | . | . | . | . | . | . |
| 21-225_22C1_LC | . | . | . | . | . | S | T | . | . | . | . | . | . | . | . | . | . | . | . | . | . | . | . | . | . | . |
| 21-225_22D12_LC | . | . | . | . | . | S | T | . | . | . | . | . | . | . | . | . | . | . | . | . | . | . | . | . | . | . |
| 21-225_22F2_LC | . | . | . | . | . | . | . | . | . | . | . | . | . | . | . | . | . | . | . | . | . | . | . | . | . | . |
| 21-225_22F9_LC | . | . | . | . | . | . | . | . | . | . | . | . | . | . | . | . | . | . | . | . | . | . | . | . | . | . |
| 21-225_22G8_LC | . | . | . | . | . | . | . | . | . | . | . | . | . | . | . | . | . | . | . | . | . | . | . | . | . | . |
| 21-225_22G9_LC | . | . | . | . | . | A | . | . | . | . | . | . | . | . | . | . | . | . | . | . | . | . | . | . | . | . |
| 21-225_22H4_LC | . | . | . | . | . | . | . | . | . | . | . | . | . | . | . | . | . | . | . | . | . | . | . | . | . | . |
| 21-225_23A3_LC | F | . | . | . | . | . | . | . | . | . | . | . | . | . | . | . | . | . | . | . | . | . | . | . | . | . |
| 21-225_23C8_LC | . | . | . | . | . | . | . | . | . | . | . | . | . | . | . | . | . | . | . | . | . | . | . | . | . | . |
| 21-225_23D1_LC | . | . | . | . | . | S | . | . | . | . | . | . | . | . | . | . | . | . | . | . | . | . | . | . | . | . |
| 21-225_23H11_LC | . | . | . | . | . | . | R | . | . | . | . | . | . | . | . | . | . | . | . | . | . | . | . | . | . | . |
| 21-225_24E5_LC | . | . | . | . | . | S | N | . | . | . | . | . | . | . | . | . | . | . | . | . | . | . | . | . | . | . |
| 21-225_25E6_LC | . | . | . | . | . | . | . | . | . | . | . | . | . | . | . | . | . | . | . | . | . | . | . | . | . | . |
| 21-225_25H10_LC | . | . | . | . | . | . | . | . | . | . | . | . | . | . | . | . | . | . | . | . | . | . | . | . | . | . |
| 21-225_25H11_LC | . | . | . | . | . | . | . | . | . | . | . | . | . | . | . | . | . | . | . | . | . | . | . | . | . | . |
| 21-225_27C3_LC | . | . | . | . | . | . | R | . | . | . | . | . | . | . | . | . | . | . | . | . | . | . | . | . | . | . |
| 21-225_27F2_LC | . | . | . | . | . | . | . | . | . | . | . | . | . | . | . | . | . | . | . | . | . | . | . | . | . | . |

# FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | L | Q | H | N | S | - | - | - | - | - | - | - | - | - | - | - | - | - | - | - | - | - | - | - |
| 21-225_29B8_LC | . | . | . | . | . | . | . | . | . | . | . | . | . | . | . | . | . | . | . | . | . | . | . | . | . | . |
| 21-225_29G4_LC | . | . | . | . | . | . | . | . | . | . | . | . | . | . | . | . | . | . | . | . | . | . | . | . | . | . |
| 21-225_29G8_LC | N | . | . | . | . | . | . | . | . | . | . | . | . | . | . | . | . | . | . | . | . | . | . | . | . | . |
| 21-225_29H6_LC | . | . | . | . | . | . | . | . | . | . | . | . | . | . | . | . | . | . | . | . | . | . | . | . | . | . |
| 21-225_2A11_LC | . | . | . | . | . | . | . | . | . | . | . | . | . | . | . | . | . | . | . | . | . | . | . | . | . | . |
| 21-225_2B2_LC | H | . | . | . | . | . | . | . | . | . | . | . | . | . | . | . | . | . | . | . | . | . | . | . | . | . |
| 21-225_2G9_LC | . | . | . | . | . | E | . | . | . | . | . | . | . | . | . | . | . | . | . | . | . | . | . | . | . | . |
| 21-225_30D1_LC | . | . | . | . | . | T | I | . | . | . | . | . | . | . | . | . | . | . | . | . | . | . | . | . | . | . |
| 21-225_30E3_LC | . | . | . | . | . | T | . | . | . | . | . | . | . | . | . | . | . | . | . | . | . | . | . | . | . | . |
| 21-225_30E9_LC | . | . | . | . | . | T | . | . | . | . | . | . | . | . | . | . | . | . | . | . | . | . | . | . | . | . |
| 21-225_30F3_LC | . | . | . | . | . | . | . | . | . | . | . | . | . | . | . | . | . | . | . | . | . | . | . | . | . | . |
| 21-225_30G11_LC | F | . | . | . | . | T | I | . | . | . | . | . | . | . | . | . | . | . | . | . | . | . | . | . | . | . |
| 21-225_31A8_LC | . | . | . | . | . | T | I | . | . | . | . | . | . | . | . | . | . | . | . | . | . | . | . | . | . | . |
| 21-225_31B8_LC | . | . | . | . | . | I | I | . | . | . | . | . | . | . | . | . | . | . | . | . | . | . | . | . | . | . |
| 21-225_31H3_LC | . | . | . | . | . | T | . | . | . | . | . | . | . | . | . | . | . | . | . | . | . | . | . | . | . | . |
| 21-225_31H5_LC | . | . | I | . | . | I | I | . | . | . | . | . | . | . | . | . | . | . | . | . | . | . | . | . | . | . |
| 21-225_32G1_LC | . | . | . | . | . | T | I | . | . | . | . | . | . | . | . | . | . | . | . | . | . | . | . | . | . | . |
| 21-225_32G12_LC | . | . | . | . | . | T | I | . | . | . | . | . | . | . | . | . | . | . | . | . | . | . | . | . | . | . |
| 21-225_33A4_LC | . | . | . | . | . | Y | . | . | . | . | . | . | . | . | . | . | . | . | . | . | . | . | . | . | . | . |
| 21-225_33A7_LC | . | . | . | . | . | T | I | . | . | . | . | . | . | . | . | . | . | . | . | . | . | . | . | . | . | . |
| 21-225_33B1_LC | . | . | . | . | . | . | . | . | . | . | . | . | . | . | . | . | . | . | . | . | . | . | . | . | . | . |
| 21-225_33B8_LC | F | . | . | . | . | T | I | . | . | . | . | . | . | . | . | . | . | . | . | . | . | . | . | . | . | . |
| 21-225_33F1_LC | . | . | . | . | . | T | I | . | . | . | . | . | . | . | . | . | . | . | . | . | . | . | . | . | . | . |
| 21-225_33G7_LC | . | . | . | . | . | Y | . | . | . | . | . | . | . | . | . | . | . | . | . | . | . | . | . | . | . | . |
| 21-225_34A6_LC | . | . | . | . | . | . | . | . | . | . | . | . | . | . | . | . | . | . | . | . | . | . | . | . | . | . |
| 21-225_34D11_LC | . | . | . | . | . | T | I | . | . | . | . | . | . | . | . | . | . | . | . | . | . | . | . | . | . | . |
| 21-225_34G8_LC | . | . | . | . | . | T | I | . | . | . | . | . | . | . | . | . | . | . | . | . | . | . | . | . | . | . |
| 21-225_34G9_LC | . | . | . | . | . | T | I | . | . | . | . | . | . | . | . | . | . | . | . | . | . | . | . | . | . | . |
| 21-225_34H11_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | L | Q | H | N | S | - | - | - | - | - | - | - | - | - | - | - | - | - | - | - | - | - | - | - |
| 21-225_54C6_LC | F | . | . | . | . | . | R | . | . | . | . | . | . | . | . | . | . | . | . | . | . | . | . | . | . | . |
| 21-225_55B8_LC | . | . | . | . | . | . | . | . | . | . | . | . | . | . | . | . | . | . | . | . | . | . | . | . | . | . |
| 21-225_55H6_LC | . | . | . | . | R | D | . | . | . | . | . | . | . | . | . | . | . | . | . | . | . | . | . | . | . | . |
| 21-225_56F1_LC | . | . | . | . | D | . | . | . | . | . | . | . | . | . | . | . | . | . | . | . | . | . | . | . | . | . |
| 21-225_59E6_LC | . | . | . | . | . | . | . | . | . | . | . | . | . | . | . | . | . | . | . | . | . | . | . | . | . | . |
| 21-225_5H2_LC | . | . | . | . | . | . | . | . | . | . | . | . | . | . | . | . | . | . | . | . | . | . | . | . | . | . |
| 21-225_5H7_LC | . | . | . | . | . | . | . | . | . | . | . | . | . | . | . | . | . | . | . | . | . | . | . | . | . | . |
| 21-225_60E8_LC | . | . | . | . | . | S | . | . | . | . | . | . | . | . | . | . | . | . | . | . | . | . | . | . | . | . |
| 21-225_62E2_LC | . | . | . | . | . | S | N | . | . | . | . | . | . | . | . | . | . | . | . | . | . | . | . | . | . | . |
| 21-225_62E4_LC | . | . | . | . | . | . | . | . | . | . | . | . | . | . | . | . | . | . | . | . | . | . | . | . | . | . |
| 21-225_64E1_LC | . | . | . | . | . | . | . | . | . | . | . | . | . | . | . | . | . | . | . | . | . | . | . | . | . | . |
| 21-225_68D12_LC | . | . | . | . | . | S | T | . | . | . | . | . | . | . | . | . | . | . | . | . | . | . | . | . | . | . |
| 21-225_68F11_LC | . | . | . | . | . | . | . | . | . | . | . | . | . | . | . | . | . | . | . | . | . | . | . | . | . | . |
| 21-225_6A6_LC | . | . | . | . | . | S | . | . | . | . | . | . | . | . | . | . | . | . | . | . | . | . | . | . | . | . |
| 21-225_6C12_LC | F | . | . | . | D | . | . | . | . | . | . | . | . | . | . | . | . | . | . | . | . | . | . | . | . | . |
| 21-225_6D4_LC | . | . | . | . | . | S | . | . | . | . | . | . | . | . | . | . | . | . | . | . | . | . | . | . | . | . |
| 21-225_6G12_LC | . | . | . | . | . | . | . | . | . | . | . | . | . | . | . | . | . | . | . | . | . | . | . | . | . | . |
| 21-225_70E12_LC | . | . | . | . | . | . | . | . | . | . | . | . | . | . | . | . | . | . | . | . | . | . | . | . | . | . |
| 21-225_70E8_LC | . | . | . | . | . | . | R | . | . | . | . | . | . | . | . | . | . | . | . | . | . | . | . | . | . | . |
| 21-225_73G6_LC | . | . | . | . | . | . | . | . | . | . | . | . | . | . | . | . | . | . | . | . | . | . | . | . | . | . |
| 21-225_74A1_LC | . | . | . | . | . | D | . | . | . | . | . | . | . | . | . | . | . | . | . | . | . | . | . | . | . | . |
| 21-225_74D7_LC | . | . | . | . | . | S | N | . | . | . | . | . | . | . | . | . | . | . | . | . | . | . | . | . | . | . |
| 21-225_74E4_LC | H | . | . | . | . | S | N | . | . | . | . | . | . | . | . | . | . | . | . | . | . | . | . | . | . | . |
| 21-225_75H7_LC | . | . | I | . | . | S | N | . | . | . | . | . | . | . | . | . | . | . | . | . | . | . | . | . | . | . |
| 21-225_7C4_LC | . | . | . | . | . | . | . | . | . | . | . | . | . | . | . | . | . | . | . | . | . | . | . | . | . | . |
| 21-225_7E9_LC | . | . | . | . | . | . | . | . | . | . | . | . | . | . | . | . | . | . | . | . | . | . | . | . | . | . |
| 21-225_7F4_LC | . | . | . | . | . | S | A | . | . | . | . | . | . | . | . | . | . | . | . | . | . | . | . | . | . | . |
| 21-225_7H11_LC | . | . | . | . | . | . | L | . | . | . | . | . | . | . | . | . | . | . | . | . | . | . | . | . | . | . |
| 21-225_7H7_LC | . | . | . | . | . | S | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | L | Q | H | N | S | - | - | - | - | - | - | - | - | - | - | - | - | - | - | - | - | - | - | - |
| 21-225_8C9_LC | . | . | . | . | . | . | . | . | . | . | . | . | . | . | . | . | . | . | . | . | . | . | . | . | . | . |
| 21-225_8D12_LC | . | . | . | . | . | S | . | . | . | . | . | . | . | . | . | . | . | . | . | . | . | . | . | . | . | . |
| 21-225_8F11_LC | . | . | . | . | . | Y | T | . | . | . | . | . | . | . | . | . | . | . | . | . | . | . | . | . | . | . |
| 21-225_8H7_LC | . | . | . | . | . | . | . | . | . | . | . | . | . | . | . | . | . | . | . | . | . | . | . | . | . | . |
| 21-225_96A3_LC | H | . | . | . | . | S | N | . | . | . | . | . | . | . | . | . | . | . | . | . | . | . | . | . | . | . |
| 21-225_9E8_LC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | L | T | F | G | G | G | T | K | V | E | I | K | R |
| 21-225_10G5_LC | . | . | . | . | . | . | . | . | . | . | . | . | . | . | . | . | . | . | . |
| 21-225_10H12_LC | . | . | . | . | . | . | . | . | . | . | . | . | . | . | . | . | . | . | . |
| 21-225_11F10_LC | . | . | . | . | . | . | . | . | . | . | . | . | . | . | . | . | . | . | . |
| 21-225_11F5_LC | . | . | . | . | . | . | . | . | . | . | . | . | . | . | . | . | . | . | . |
| 21-225_11G7_LC | . | . | . | . | . | . | . | . | . | . | . | . | . | M | . | . | . | R | . |
| 21-225_12A1_LC | . | . | . | . | . | . | . | . | . | . | . | . | . | . | . | . | . | . | . |
| 21-225_13E6_LC | . | . | . | . | . | . | . | . | . | . | . | . | . | . | . | A | . | . | . |
| 21-225_13H3_LC | . | . | . | . | . | . | . | . | . | . | . | . | . | . | . | . | . | . | . |
| 21-225_13H5_LC | . | . | . | . | . | . | . | . | . | . | . | . | . | . | . | . | . | . | . |
| 21-225_146F1_LC | . | . | . | . | . | . | . | . | . | . | . | . | . | . | . | . | . | . | . |
| 21-225_146G11_LC | . | . | . | . | . | . | . | . | . | . | . | . | . | . | . | . | . | . | . |
| 21-225_146H9_LC | . | . | . | . | . | . | . | . | . | . | . | . | . | . | . | . | . | . | . |
| 21-225_148E11_LC | . | . | . | . | . | . | . | . | . | . | . | . | . | . | . | . | . | . | . |
| 21-225_148F12_LC | . | . | . | . | . | . | . | I | . | . | . | . | . | . | . | . | . | . | . |
| 21-225_149F12_LC | . | . | . | . | . | . | . | . | . | . | . | . | . | . | . | . | . | . | . |
| 21-225_149G1_LC | . | . | . | . | . | . | . | . | . | . | . | . | . | . | . | . | . | . | . |
| 21-225_149G5_LC | . | . | . | . | . | . | . | . | . | . | . | . | . | . | . | . | . | . | . |
| 21-225_14B1_LC1_LC | . | . | . | . | . | . | . | . | . | . | . | . | . | . | . | . | . | . | . |
| 21-225_14B2_LC | . | . | . | . | . | . | . | . | . | . | . | . | . | . | . | . | . | . | . |
| 21-225_14C2_LC | . | . | . | . | . | . | . | . | . | . | . | . | . | . | . | . | . | R | . |
| 21-225_14E3_LC | . | . | . | . | . | . | F | . | . | . | . | . | . | . | . | . | . | . | . |
| 21-225_150E7_LC | . | . | . | . | . | . | . | . | . | . | . | . | . | . | . | . | . | . | . |
| 21-225_152H9_LC | . | . | . | . | . | . | . | . | . | . | . | . | . | . | . | . | . | . | . |
| 21-225_156G1_LC | . | . | . | . | . | . | . | . | . | . | . | . | . | . | . | . | . | . | . |
| 21-225_158H5_LC | . | . | . | . | . | . | . | . | . | . | . | . | . | . | . | . | . | . | . |
| 21-225_15A1_LC | . | . | . | . | . | . | V | K | . | . | . | . | I | . | . | . | . | T | G |
| 21-225_15C9_LC | . | . | . | . | . | . | . | . | . | . | . | . | . | . | . | . | . | . | . |
| 21-225_15E3_LC | . | . | . | . | . | . | . | . | . | . | . | . | . | E | . | . | . | Y | . |
| 21-225_15G7_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | L | T | F | G | G | G | T | K | V | E | I | K | R |
| 21-225_15H1_LC | . | . | . | . | . | . | . | . | . | . | . | . | . | . | . | . | . | . | . |
| 21-225_15H10_LC | . | . | . | . | . | . | . | . | . | . | . | . | . | . | . | . | . | N | . |
| 21-225_15H8_LC | . | . | . | . | . | . | . | . | . | . | . | . | . | . | . | . | . | R | . |
| 21-225_160B10_LC | . | . | . | . | . | . | . | . | . | . | . | . | . | . | . | . | . | . | . |
| 21-225_160H3_LC | . | . | . | . | . | . | . | . | . | . | . | . | . | Q | . | . | S | . | . |
| 21-225_169B1_LC | . | . | . | . | . | . | . | . | . | . | . | . | . | . | . | . | . | . | . |
| 21-225_169F9_LC | . | . | . | . | . | . | . | . | . | . | . | . | . | . | . | . | . | . | . |
| 21-225_16A1_LC | . | . | . | . | . | . | . | . | . | . | . | . | . | . | . | . | . | E | . |
| 21-225_16B7_LC | . | . | . | . | . | . | . | . | . | . | . | . | . | . | . | . | F | N | . |
| 21-225_16E12_LC | . | . | . | . | . | . | . | . | . | . | . | . | . | . | . | . | . | . | . |
| 21-225_16F11_LC | . | . | . | . | . | . | . | . | . | . | . | . | . | . | . | . | . | . | . |
| 21-225_16F4_LC | . | . | . | . | . | . | F | . | . | . | . | . | . | . | . | . | . | . | . |
| 21-225_170D11_LC | . | . | . | . | F | . | . | . | . | . | . | . | . | . | . | . | . | R | . |
| 21-225_170D5_LC | . | . | . | . | F | . | . | . | . | . | . | . | . | . | . | . | . | . | . |
| 21-225_170G4_LC | . | . | . | . | . | . | . | . | . | . | . | . | . | . | . | . | . | . | . |
| 21-225_170G5_LC | . | . | . | . | . | . | . | . | . | . | . | . | . | . | . | . | . | . | . |
| 21-225_171A4_LC | . | . | . | . | . | . | . | . | . | . | . | . | . | . | . | . | . | . | . |
| 21-225_171C3_LC | . | . | . | . | . | . | . | . | . | . | . | . | . | . | . | . | . | . | . |
| 21-225_172B3_LC | . | . | . | . | . | . | . | . | . | . | . | . | . | . | . | . | . | . | . |
| 21-225_172G8_LC | . | . | . | . | F | . | . | . | . | . | . | . | . | . | . | . | . | . | . |
| 21-225_173H12_LC | . | . | . | . | F | . | N | . | . | . | . | . | . | . | . | . | . | . | . |
| 21-225_175G1_LC | . | . | . | . | . | . | . | . | . | . | . | . | . | . | . | . | . | . | . |
| 21-225_176B11_LC | . | . | . | . | . | . | . | . | . | . | . | . | . | . | . | . | . | . | . |
| 21-225_178B10_LC | . | . | . | . | F | . | . | . | . | . | . | . | . | . | . | . | . | . | . |
| 21-225_17A10_LC | . | . | . | . | . | . | . | . | . | . | . | . | . | . | . | . | . | . | . |
| 21-225_17B5_LC | . | . | . | . | . | . | . | . | . | . | . | . | . | . | . | . | . | . | . |
| 21-225_17F9_LC | . | . | . | . | . | . | . | . | . | . | . | . | . | . | . | . | F | . | . |
| 21-225_17H12_LC | . | . | . | . | . | M | F | . | . | . | . | . | . | . | . | . | . | . | . |
| 21-225_17H8_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | L | T | F | G | G | G | T | K | V | E | I | K | R |
| 21-225_18A1_LC | . | . | . | . | . | . | . | . | . | . | . | . | . | . | . | . | . | . | . |
| 21-225_18A3_LC | . | . | . | . | . | . | . | . | . | . | . | . | . | . | . | . | . | . | . |
| 21-225_18C6_LC | . | . | . | . | . | . | . | . | . | . | . | . | . | . | . | . | . | . | . |
| 21-225_18E8_LC | . | . | . | . | . | . | . | . | . | . | . | . | . | . | . | . | . | . | . |
| 21-225_190C8_LC | . | . | . | . | . | . | F | . | . | . | . | . | . | . | . | . | . | . | . |
| 21-225_193A5_LC | . | . | . | . | F | . | F | . | . | . | . | . | . | . | . | . | . | . | . |
| 21-225_193E7_LC | . | . | . | . | . | . | . | . | . | . | . | . | . | . | . | . | . | . | . |
| 21-225_196C10_LC | . | . | . | . | . | . | . | . | . | . | . | . | . | . | . | . | . | . | . |
| 21-225_199E3_LC | . | . | . | . | . | . | . | . | . | . | . | . | . | . | . | . | . | . | . |
| 21-225_19D11_LC | . | . | . | . | . | . | . | . | . | . | . | . | . | E | . | . | . | . | . |
| 21-225_19E1_LC | . | . | . | . | F | . | . | . | . | . | . | . | . | . | . | . | . | . | . |
| 21-225_19F3_LC | . | . | . | . | . | . | . | . | . | . | . | . | . | . | . | . | . | . | . |
| 21-225_1A2_LC | . | . | . | . | H | . | . | . | . | . | . | . | . | . | . | . | . | . | . |
| 21-225_1C12_LC | . | . | . | . | . | . | . | . | . | . | . | . | . | . | . | A | . | . | . |
| 21-225_1F2_LC | . | . | . | . | . | . | . | . | . | . | . | . | . | . | . | . | . | . | . |
| 21-225_200F12_LC | . | . | . | . | . | . | . | . | . | . | . | . | . | . | . | . | . | . | . |
| 21-225_200H9_LC | . | . | . | . | . | . | . | . | . | . | . | . | . | . | . | . | . | . | . |
| 21-225_201A1_LC | . | . | . | . | . | . | . | . | . | . | . | . | . | . | . | . | V | . | . |
| 21-225_201A10_LC | . | . | . | . | . | . | . | . | . | . | . | . | . | . | . | . | . | . | . |
| 21-225_204H8_LC | . | . | . | . | . | . | . | . | . | . | . | . | . | . | . | . | . | . | . |
| 21-225_205H8_LC | . | . | . | . | . | . | . | . | . | . | . | . | . | T | . | K | . | . | . |
| 21-225_20A10_LC | . | . | . | . | . | . | . | . | . | . | . | . | . | . | . | . | . | . | . |
| 21-225_20A7_LC | . | . | . | . | . | . | . | . | . | . | . | . | . | . | . | . | . | . | . |
| 21-225_20C2_LC | . | . | . | . | . | . | . | . | . | . | . | . | . | . | . | . | . | E | . |
| 21-225_20C8_LC | . | . | . | . | . | . | F | . | . | . | . | . | . | . | . | . | . | T | . |
| 21-225_20D5_LC | . | . | . | . | . | . | F | . | . | . | . | . | . | . | . | . | . | . | . |
| 21-225_20E12_LC | . | . | . | . | . | . | F | . | . | . | . | . | . | . | . | . | . | . | . |
| 21-225_20E5_LC | . | . | . | . | . | . | . | . | . | . | . | . | . | . | . | . | . | . | . |
| 21-225_20G2_LC | . | . | . | . | . | . | . | . | . | . | . | . | . | . | . | . | . | . | . |

# FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | L | T | F | G | G | G | T | K | V | E | I | K | R |
| 21-225_20G9_LC | . | . | . | . | . | . | . | . | . | . | . | . | . | . | . | . | . | . | . |
| 21-225_20H10_LC | . | . | . | . | . | . | . | . | . | . | . | . | . | . | . | . | . | . | . |
| 21-225_20H12_LC | . | . | . | . | . | . | . | . | . | . | . | . | . | . | . | . | . | . | . |
| 21-225_21E5_LC | . | . | . | . | . | . | . | . | . | . | . | . | . | . | . | . | . | . | . |
| 21-225_21F3_LC | . | . | . | . | . | . | . | . | . | . | . | . | . | . | . | . | . | . | . |
| 21-225_21G7_LC | . | . | . | . | . | . | . | . | . | . | . | . | . | . | . | . | . | . | . |
| 21-225_21H3_LC | . | . | . | . | . | . | . | . | . | . | . | . | . | . | . | . | . | . | . |
| 21-225_224A1_LC | . | . | . | . | . | . | . | . | . | . | . | . | . | . | . | . | . | . | . |
| 21-225_224B1_LC | . | . | . | . | . | . | . | . | . | . | . | . | . | . | . | . | . | . | . |
| 21-225_224C3_LC | . | . | . | . | . | . | . | . | . | . | . | . | . | . | . | . | . | . | . |
| 21-225_224D10_LC | . | . | . | . | . | . | . | . | . | . | . | . | . | . | . | . | . | . | . |
| 21-225_22B7_LC | . | . | . | . | . | . | . | . | . | . | . | . | . | . | . | . | . | . | . |
| 21-225_22C1_LC | . | . | . | . | . | . | . | . | . | . | . | . | . | . | . | . | . | . | . |
| 21-225_22D12_LC | . | . | . | . | . | . | . | . | . | . | . | . | . | . | . | . | . | . | . |
| 21-225_22F2_LC | . | . | . | . | . | . | . | . | . | . | . | . | . | . | . | . | . | . | . |
| 21-225_22F9_LC | . | . | . | . | . | . | . | . | . | . | . | . | . | . | . | . | . | . | . |
| 21-225_22G8_LC | . | . | . | . | . | . | . | . | . | . | . | . | . | . | . | . | . | . | . |
| 21-225_22G9_LC | . | . | . | . | . | . | . | . | . | . | . | . | . | . | . | . | . | . | . |
| 21-225_22H4_LC | . | . | . | . | . | . | . | . | . | . | . | . | . | . | . | . | . | . | . |
| 21-225_23A3_LC | . | . | . | . | . | . | . | . | . | . | . | . | . | . | . | . | . | . | . |
| 21-225_23C8_LC | . | . | . | . | . | . | . | . | . | . | . | . | . | . | . | . | . | . | . |
| 21-225_23D1_LC | . | . | . | . | . | . | . | . | . | . | . | . | . | . | . | . | . | . | . |
| 21-225_23H11_LC | . | . | . | . | . | . | . | . | . | . | . | . | . | . | . | . | . | . | . |
| 21-225_24E5_LC | . | . | . | . | . | . | . | . | . | . | . | . | . | . | . | . | . | . | . |
| 21-225_25E6_LC | . | . | . | . | . | . | . | . | . | . | . | . | . | . | . | . | . | . | . |
| 21-225_25H10_LC | . | . | . | . | . | . | . | . | . | . | . | . | . | . | . | . | . | . | . |
| 21-225_25H11_LC | . | . | . | . | . | . | . | . | . | . | . | . | . | . | . | . | . | . | . |
| 21-225_27C3_LC | . | . | . | . | . | . | . | . | . | . | . | . | . | . | . | . | . | . | . |
| 21-225_27F2_LC | . | . | . | . | . | . | . | . | . | . | . | . | . | . | . | . | . | . | . |

# FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | L | T | F | G | G | G | T | K | V | E | I | K | R |
| 21-225_29B8_LC | . | . | . | . | . | . | . | . | . | . | . | . | . | . | . | . | . | . | . |
| 21-225_29G4_LC | . | . | . | . | . | . | . | . | . | . | . | . | . | . | . | . | . | T | . |
| 21-225_29G8_LC | . | . | . | . | . | . | . | . | . | . | . | . | . | . | . | . | . | . | . |
| 21-225_29H6_LC | . | . | . | . | . | . | . | . | . | . | . | . | . | . | . | . | . | . | . |
| 21-225_2A11_LC | . | . | . | . | . | . | . | . | . | . | . | . | . | . | M | . | . | . | . |
| 21-225_2B2_LC | . | . | . | . | . | . | . | . | . | . | . | . | . | . | . | . | . | . | . |
| 21-225_2G9_LC | . | . | . | . | . | . | . | . | . | . | . | . | . | . | . | . | . | . | . |
| 21-225_30D1_LC | . | . | . | . | . | . | P | . | . | . | . | . | . | . | . | . | . | . | . |
| 21-225_30E3_LC | . | . | . | . | . | . | P | . | . | . | . | . | . | . | . | . | . | . | . |
| 21-225_30E9_LC | . | . | . | . | . | . | P | . | . | . | . | . | . | . | . | . | . | . | . |
| 21-225_30F3_LC | . | . | . | . | . | . | P | . | . | . | . | . | . | . | . | . | . | . | . |
| 21-225_30G11_LC | . | . | . | . | . | . | P | . | . | . | . | . | . | . | . | . | . | . | . |
| 21-225_31A8_LC | . | . | . | . | . | . | P | . | . | . | . | . | . | . | . | . | . | . | . |
| 21-225_31B8_LC | . | . | . | . | . | . | P | . | . | . | . | . | . | . | . | . | . | . | . |
| 21-225_31H3_LC | . | . | . | . | . | . | P | . | . | . | . | . | . | . | . | . | . | . | . |
| 21-225_31H5_LC | . | . | . | . | . | . | P | . | . | . | . | . | . | . | . | . | . | . | . |
| 21-225_32G1_LC | . | . | . | . | S | . | P | . | . | . | . | . | . | . | . | . | . | . | . |
| 21-225_32G12_LC | . | . | . | . | . | . | P | . | . | . | . | . | . | . | . | . | . | . | . |
| 21-225_33A4_LC | . | . | . | . | . | . | P | . | . | . | . | . | . | . | . | . | . | . | . |
| 21-225_33A7_LC | . | . | . | . | . | . | P | . | . | . | . | . | . | . | . | . | . | . | . |
| 21-225_33B1_LC | . | . | . | . | . | . | . | . | . | . | . | . | . | . | . | . | V | . | . |
| 21-225_33B8_LC | . | . | . | . | . | . | P | . | . | . | . | . | . | . | . | . | . | . | . |
| 21-225_33F1_LC | . | . | . | . | . | . | P | . | . | . | . | . | . | . | . | . | . | . | . |
| 21-225_33G7_LC | . | . | . | . | . | . | P | . | . | . | . | . | . | . | . | . | . | . | . |
| 21-225_34A6_LC | . | . | . | . | . | . | P | . | . | . | . | . | . | . | . | . | . | . | . |
| 21-225_34D11_LC | . | . | . | . | . | . | P | . | . | . | . | . | . | . | . | G | . | . | . |
| 21-225_34G8_LC | . | . | . | . | . | . | P | . | . | . | . | . | . | . | . | . | . | . | . |
| 21-225_34G9_LC | . | . | . | . | . | . | P | . | . | . | . | . | . | . | . | W | . | . | . |
| 21-225_34H11_LC | . | . | . | . | . | . | P | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | L | T | F | G | G | G | T | K | V | E | I | K | R |
| 21-225_35A6_LC | . | . | . | . | . | . | P | . | . | . | . | . | . | . | . | . | . | . | . |
| 21-225_35B7_LC | . | . | . | . | . | . | P | . | . | . | . | . | . | . | . | . | . | . | . |
| 21-225_35D1_LC | . | . | . | . | . | . | P | . | . | . | . | . | . | . | . | . | . | . | . |
| 21-225_35F11_LC | . | . | . | . | . | . | P | . | . | . | . | . | . | . | . | . | . | . | . |
| 21-225_36A5_LC | . | . | . | . | . | . | P | K | . | . | . | . | I | . | . | . | . | T | G |
| 21-225_36C5_LC | . | . | . | . | . | . | P | . | . | . | . | . | . | . | . | W | . | . | . |
| 21-225_43C3_LC | . | . | . | . | . | . | . | . | . | . | . | . | . | . | . | G | . | T | . |
| 21-225_44C3_LC | . | . | . | . | . | . | . | . | . | . | . | . | . | . | . | . | . | . | . |
| 21-225_44E12_LC | . | . | . | . | . | . | . | . | . | . | . | . | . | . | . | . | . | . | . |
| 21-225_46B1_LC | . | . | . | . | . | . | . | . | . | . | . | . | . | . | . | . | . | . | . |
| 21-225_46C6_LC | . | . | . | . | . | . | . | . | . | . | . | . | . | . | . | . | . | T | . |
| 21-225_46D8_LC | . | . | . | . | . | . | . | . | . | . | . | . | . | . | . | . | . | . | . |
| 21-225_46F3_LC | . | . | . | . | . | . | . | . | . | . | . | . | . | . | . | . | . | . | . |
| 21-225_47A1_LC | . | . | . | . | . | . | . | . | . | . | . | . | . | . | . | . | . | . | . |
| 21-225_47A5_LC | . | . | . | . | . | . | . | . | . | . | . | . | . | . | . | . | . | . | . |
| 21-225_48D12_LC | . | . | . | . | . | . | . | . | . | . | . | . | . | . | . | . | . | . | . |
| 21-225_48H4_LC | . | . | . | . | . | . | . | . | . | . | . | . | . | . | . | . | . | . | . |
| 21-225_49A1_LC | . | . | . | . | . | . | . | . | . | . | . | . | . | . | . | . | . | . | . |
| 21-225_49C6_LC | . | . | . | . | . | . | . | . | . | . | . | . | . | . | . | . | . | . | . |
| 21-225_4C8_LC | . | . | . | . | . | . | . | . | . | . | . | . | . | . | . | . | . | . | . |
| 21-225_4E10_LC | . | . | . | . | . | . | . | . | . | . | . | . | . | . | . | . | . | . | . |
| 21-225_4F12_LC | . | . | . | . | . | A | . | . | . | . | . | . | . | . | . | . | . | . | . |
| 21-225_4F4_LC | . | . | . | . | . | . | . | . | . | . | . | . | . | . | . | . | . | . | . |
| 21-225_4H4_LC | . | . | . | . | . | . | . | . | . | . | . | . | . | . | . | . | . | . | . |
| 21-225_50G10_LC | . | . | . | . | . | . | F | . | . | . | . | . | . | . | . | . | S | . | . |
| 21-225_51F11_LC | . | . | . | . | . | . | F | . | . | . | . | . | . | . | . | . | . | . | . |
| 21-225_52B2_LC | . | . | . | . | . | . | . | . | . | . | . | . | . | . | . | . | . | . | . |
| 21-225_53D2_LC | . | . | . | . | . | . | . | . | . | . | . | . | . | . | . | . | . | . | . |
| 21-225_53E6_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | L | T | F | G | G | G | T | K | V | E | I | K | R |
| 21-225_54C6_LC | . | . | . | . | . | . | . | . | . | . | . | . | . | . | . | . | . | . | . |
| 21-225_55B8_LC | . | . | . | . | . | . | . | . | . | . | . | . | . | . | . | G | . | . | . |
| 21-225_55H6_LC | . | . | . | . | . | . | F | . | . | . | . | . | . | . | . | . | . | . | . |
| 21-225_56F1_LC | . | . | . | . | H | . | F | . | . | . | . | . | . | . | . | . | . | . | . |
| 21-225_59E6_LC | . | . | . | . | . | . | . | . | . | . | . | . | . | R | . | . | . | . | . |
| 21-225_5H2_LC | . | . | . | . | . | . | . | . | . | . | . | . | . | . | . | . | . | . | . |
| 21-225_5H7_LC | . | . | . | . | . | . | . | . | . | . | . | . | . | . | . | . | . | . | . |
| 21-225_60E8_LC | . | . | . | . | . | . | . | . | . | . | . | . | . | . | . | . | . | . | . |
| 21-225_62E2_LC | . | . | . | . | . | . | . | . | . | . | . | . | . | R | . | . | . | . | . |
| 21-225_62E4_LC | . | . | . | . | . | . | . | . | . | . | . | . | . | . | . | . | . | . | . |
| 21-225_64E1_LC | . | . | . | . | . | . | . | . | . | . | . | . | . | . | . | . | . | . | . |
| 21-225_68D12_LC | . | . | . | . | . | . | . | . | . | . | . | . | . | . | . | . | . | . | . |
| 21-225_68F11_LC | . | . | . | . | . | . | F | . | . | . | . | . | . | . | . | . | . | . | . |
| 21-225_6A6_LC | . | . | . | . | . | . | . | . | . | . | . | . | . | . | . | . | . | . | . |
| 21-225_6C12_LC | . | . | . | . | . | . | F | . | . | . | . | . | . | . | . | . | . | . | . |
| 21-225_6D4_LC | . | . | . | . | . | . | . | . | . | . | . | . | . | . | . | . | . | . | . |
| 21-225_6G12_LC | . | . | . | . | . | . | . | . | . | . | . | . | . | . | . | . | . | . | . |
| 21-225_70E12_LC | . | . | . | . | . | . | . | . | . | . | . | . | S | . | . | . | . | . | . |
| 21-225_70E8_LC | . | . | . | . | . | . | . | . | . | . | . | . | . | . | . | . | . | . | . |
| 21-225_73G6_LC | . | . | . | . | . | . | . | . | . | . | . | . | . | . | . | . | . | . | . |
| 21-225_74A1_LC | . | . | . | . | . | . | . | . | . | . | . | . | A | . | . | . | . | . | . |
| 21-225_74D7_LC | . | . | . | . | . | . | . | . | . | . | . | . | . | . | . | . | . | . | . |
| 21-225_74E4_LC | . | . | . | . | . | . | . | . | . | . | . | . | . | . | . | . | . | . | . |
| 21-225_75H7_LC | . | . | . | . | . | . | . | . | . | . | . | . | . | . | . | . | . | . | . |
| 21-225_7C4_LC | . | . | . | . | . | . | . | . | . | . | . | . | . | . | . | . | . | . | . |
| 21-225_7E9_LC | . | . | . | . | . | . | . | . | . | . | . | . | . | . | . | . | . | . | . |
| 21-225_7F4_LC | . | . | . | . | . | . | . | . | . | . | . | . | . | . | . | . | . | . | . |
| 21-225_7H11_LC | . | . | . | . | . | . | . | . | . | . | . | . | . | . | . | . | . | . | . |
| 21-225_7H7_LC | . | . | . | . | . | . | F | . | . | V | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | L | T | F | G | G | G | T | K | V | E | I | K | R |
| 21-225_8C9_LC | . | . | . | . | . | . | . | . | . | . | . | . | . | . | . | . | . | . | . |
| 21-225_8D12_LC | . | . | . | . | . | . | . | . | . | . | . | . | . | . | . | . | . | . | . |
| 21-225_8F11_LC | . | . | . | . | . | . | . | . | . | . | . | . | . | . | . | . | . | . | . |
| 21-225_8H7_LC | . | . | . | . | . | . | . | . | . | . | . | . | . | . | . | . | . | . | . |
| 21-225_96A3_LC | . | . | . | . | . | . | . | . | . | . | . | . | . | . | . | . | . | . | . |
| 21-225_9E8_LC | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_18A1_LC | . | . | . | . | . | . | . | . | . | . | . | . | . | . | . | . | . | . | . | . | . | . | . | . | . | . |
| 21-225_18A3_LC | . | . | . | . | . | S | . | . | . | . | . | . | . | . | . | . | . | . | . | . | . | . | . | . | . | . |
| 21-225_18C6_LC | . | . | . | . | . | S | . | . | . | . | . | . | . | . | . | . | . | . | . | . | . | . | . | . | . | . |
| 21-225_18E8_LC | . | . | . | . | . | . | . | . | . | . | . | . | . | . | . | . | . | . | . | . | . | . | . | . | . | . |
| 21-225_190C8_LC | . | . | . | . | . | . | D | . | . | . | . | . | . | . | . | . | . | . | . | . | . | . | . | . | . | . |
| 21-225_193A5_LC | . | . | . | . | . | . | D | . | . | . | . | . | . | . | . | . | . | . | . | . | . | . | . | . | . | . |
| 21-225_193E7_LC | . | . | . | . | . | K | R | . | . | . | . | . | . | . | . | . | . | . | . | . | . | . | . | . | . | . |
| 21-225_196C10_LC | . | . | . | . | Y | . | . | . | . | . | . | . | . | . | . | . | . | . | . | . | . | . | . | . | . | . |
| 21-225_199E3_LC | . | . | . | . | . | K | R | . | . | . | . | . | . | . | . | . | . | . | . | . | . | . | . | . | . | . |
| 21-225_19D11_LC | . | . | . | . | Y | Y | . | . | . | . | . | . | . | . | . | . | . | . | . | . | . | . | . | . | . | . |
| 21-225_19E1_LC | . | . | . | . | . | . | . | . | . | . | . | . | . | . | . | . | . | . | . | . | . | . | . | . | . | . |
| 21-225_19F3_LC | . | . | . | . | . | A | . | . | . | . | . | . | . | . | . | . | . | . | . | . | . | . | . | . | . | . |
| 21-225_1A2_LC | . | . | . | . | . | . | . | . | . | . | . | . | . | . | . | . | . | . | . | . | . | . | . | . | . | . |
| 21-225_1C12_LC | . | . | . | . | . | . | . | . | . | . | . | . | . | . | . | . | . | . | . | . | . | . | . | . | . | . |
| 21-225_1F2_LC | . | . | . | . | . | . | . | . | . | . | . | . | . | . | . | . | . | . | . | . | . | . | . | . | . | . |
| 21-225_200F12_LC | . | . | . | . | . | K | . | . | . | . | . | . | . | . | . | . | . | . | . | . | . | . | . | . | . | . |
| 21-225_200H9_LC | . | . | . | . | . | . | . | . | . | . | . | . | . | . | . | . | . | . | . | . | . | . | . | . | . | . |
| 21-225_201A1_LC | . | . | . | . | . | K | . | . | . | . | . | . | . | . | . | . | . | . | . | . | . | . | . | . | . | . |
| 21-225_201A10_LC | . | . | . | . | . | . | . | . | . | . | . | . | . | . | . | . | . | . | . | . | . | . | . | . | . | . |
| 21-225_204H8_LC | . | . | . | . | . | . | . | . | . | . | . | . | . | . | . | . | . | . | . | . | . | . | . | . | . | . |
| 21-225_205H8_LC | . | . | . | . | . | . | . | . | . | . | . | . | . | . | . | . | . | . | . | . | . | . | . | . | . | . |
| 21-225_20A10_LC | . | . | . | . | . | . | R | . | . | . | . | . | . | . | . | . | . | . | . | . | . | . | . | . | . | . |
| 21-225_20A7_LC | . | . | . | . | . | . | . | . | . | . | . | . | . | . | . | . | . | . | . | . | . | . | . | . | . | . |
| 21-225_20C2_LC | . | . | . | . | . | A | . | . | . | . | . | . | . | . | . | . | . | . | . | . | . | . | . | . | . | . |
| 21-225_20C8_LC | . | . | . | . | D | . | . | . | . | . | . | . | . | . | . | . | . | . | . | . | . | . | . | . | . | . |
| 21-225_20D5_LC | . | . | . | . | . | . | . | . | . | . | . | . | . | . | . | . | . | . | . | . | . | . | . | . | . | . |
| 21-225_20E12_LC | . | . | . | . | . | . | . | . | . | . | . | . | . | . | . | . | . | . | . | . | . | . | . | . | . | . |
| 21-225_20E5_LC | . | . | . | . | . | . | . | . | . | . | . | . | . | . | . | . | . | . | . | . | . | . | . | . | . | . |
| 21-225_20G2_LC | . | . | . | . | . | . | . | . | . | . | . | . | . | . | . | . | . | . | . | . | . | . | . | . | . | . |

**FIGURE 57**

Table 96. Consensus 16 - VL3|3r/JL2 (SEQ ID NO: 50313):

SYELTQP-PSVSVSPGQTASITCSGD---KLGD-----KYACWYQQKPGQSPVLVIYQ--------DRKRPSGIPERFSGSNSG--NTATLTISGTQAMDEADYYCQAWDS----------------------STVVFGGGTKLTVLG wherein:

D at position 26 can be substituted with N, E, Y or S

K at position 30 can be substituted with R, N, E or T

L at position 31 can be substituted with M or S

D at position 33 can be substituted with N, E, G, Y, T, H or V

K at position 39 can be substituted with R

Y at position 40 can be substituted with F or S

A at position 41 can be substituted with V, T, D or S

C at position 42 can be substituted with S, Y, W or H

Q at position 58 can be substituted with E or K

D at position 67 can be substituted with N

R at position 68 can be substituted with S, N, K, Y, M, T, G or I

K at position 69 can be substituted with R or Q

P at position 71 can be substituted with S

S at position 72 can be substituted with L

Q at position 107 can be substituted with L or K

A at position 108 can be substituted with T

**FIGURE 57**

W at position 109 can be substituted with R

D at position 110 can be substituted with H, V, N or G

S at position 111 can be substituted with N, null (-), I, R, K or T

null (-) at position 134 can be substituted with S, N or R

S at position 135 can be substituted with T, N, R, G, F, I V or Y

T at position 136 can be substituted with S, Y, A, F, P, N, K, I or R

V at position 137 can be substituted with A, T, M L or G

V at position 138 can be substituted with I, L, A or M

Ser Gly Xaa Xaa Xaa Gly Xaa Xaa Xaa Xaa Xaa (SEQ ID NO: 50125)

Xaa Xaa Xaa Xaa Arg Xaa Xaa (SEQ ID NO: 50126)

Xaa Xaa Xaa Xaa Xaa Xaa Xaa Xaa Xaa Xaa (SEQ ID NO: 50228)

Ser Gly Asp Lys Leu Gly Asp Lys Tyr Ala Cys (SEQ ID NO: 50609)

Gln Asp Arg Lys Arg Pro Ser (SEQ ID NO: 50610)

Gln Ala Trp Asp Ser Ser Thr Val Val (SEQ ID NO: 50611)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 |
|---|---|---|---|---|---|---|---|---|---|---|---|---|---|---|---|---|---|---|---|---|---|---|---|---|---|---|---|
| | L_FR1 | | | | | | | | | | | | | | | | | | | | | | | | | | |
| CONSENSUS | S | Y | E | L | T | Q | P | - | P | S | V | S | V | S | P | G | Q | T | A | S | I | T | C | S | G | D | - |
| 21-225_10B10_LC | . | . | . | . | . | . | . | . | . | . | . | . | . | . | . | . | . | . | . | . | . | . | . | . | . | . | . |
| 21-225_12B1_LC | . | . | . | . | . | . | . | . | . | . | . | . | . | . | . | R | . | . | V | . | . | . | . | . | . | . | . |
| 21-225_13F6_LC | . | . | . | . | . | . | . | . | . | . | . | . | . | . | . | . | . | . | . | . | . | . | . | . | . | . | . |
| 21-225_146A10_LC | . | . | . | . | . | . | . | . | . | . | . | . | . | . | . | . | . | . | . | . | . | . | . | . | . | . | . |
| 21-225_146A2_LC | . | . | . | . | . | . | . | . | . | . | . | . | . | . | . | . | . | . | . | . | . | . | . | . | . | . | . |
| 21-225_146A3_LC | . | . | A | M | . | . | . | . | . | . | M | . | . | . | . | . | . | . | . | . | . | . | . | . | . | N | . |
| 21-225_146B11_LC | . | . | . | . | . | . | . | . | . | . | . | . | . | . | . | . | . | . | . | . | . | . | . | . | . | . | . |
| 21-225_146B8_LC | . | . | . | . | . | . | . | . | . | . | . | . | . | . | . | . | . | . | . | . | . | . | . | . | . | . | . |
| 21-225_146C11_LC | . | . | . | . | . | . | . | . | . | . | . | . | . | . | . | . | . | . | . | . | . | . | . | . | . | . | . |
| 21-225_146H8_LC | . | . | . | . | . | . | . | . | . | . | . | . | . | . | . | . | . | . | . | . | . | . | . | . | . | . | . |
| 21-225_147B12_LC | . | . | . | . | . | . | . | . | . | . | . | . | . | . | . | . | . | . | . | . | . | . | . | . | . | . | . |
| 21-225_147B8_LC | . | . | . | . | . | . | . | . | . | . | . | . | . | . | . | . | . | . | . | . | . | . | . | . | . | . | . |
| 21-225_147B9_LC | . | . | . | . | . | . | . | . | . | . | . | . | . | . | . | . | . | . | . | . | . | . | . | . | . | . | . |
| 21-225_147D9_LC | . | . | . | . | . | . | . | . | . | . | . | . | . | . | . | . | . | . | . | . | . | . | S | . | . | . | . |
| 21-225_147E11_LC | . | . | . | . | . | . | . | . | . | . | . | . | . | . | . | . | . | . | . | . | . | . | . | . | . | . | . |
| 21-225_147E7_LC | . | . | . | . | . | . | . | . | . | . | . | . | . | . | . | . | . | . | . | . | . | . | . | . | . | . | . |
| 21-225_147F9_LC | . | . | . | . | . | . | . | . | . | . | . | . | . | . | . | . | . | . | . | . | . | . | . | . | . | . | . |
| 21-225_147G9_LC | . | . | . | . | . | . | . | . | . | . | . | . | . | . | . | . | . | . | . | . | . | . | . | . | . | . | . |
| 21-225_148A9_LC | . | . | . | . | . | . | . | . | . | . | . | . | . | . | . | . | . | . | . | . | . | . | . | . | . | . | . |
| 21-225_148C8_LC | . | . | . | . | . | . | . | . | . | . | . | . | . | . | . | . | . | . | . | . | . | . | . | . | . | . | . |
| 21-225_148E10_LC | . | . | . | . | . | . | . | . | . | . | . | . | . | . | . | . | . | . | . | . | . | . | . | . | . | . | . |
| 21-225_148G11_LC | . | . | . | . | . | . | . | . | . | . | . | . | . | . | . | . | . | . | . | . | . | . | . | . | . | . | . |
| 21-225_148G6_LC | . | . | . | . | . | . | . | . | . | . | . | . | . | . | . | . | . | . | . | . | . | . | . | . | . | . | . |
| 21-225_149A11_LC | . | . | . | . | . | . | . | . | . | . | . | . | . | . | . | . | . | . | . | . | . | . | . | . | . | . | . |
| 21-225_149B5_LC | . | . | . | . | . | . | . | . | . | . | . | . | . | . | . | . | . | . | . | . | . | . | . | . | . | Y | . |
| 21-225_149C10_LC | . | . | . | . | . | . | . | . | . | . | . | . | . | . | . | . | . | . | . | . | . | . | . | . | . | . | . |
| 21-225_149C7_LC | . | . | . | . | . | . | . | . | . | . | . | . | . | . | . | . | . | . | . | . | . | . | . | . | . | N | . |
| 21-225_14B1_LC2_LC | . | F | . | . | . | . | . | . | . | . | . | . | . | . | . | . | . | . | . | . | . | . | . | . | . | . | . |
| 21-225_14B7_LC | . | . | . | . | . | . | . | . | . | . | . | . | . | . | . | . | R | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 |
|---|---|---|---|---|---|---|---|---|---|---|---|---|---|---|---|---|---|---|---|---|---|---|---|---|---|---|---|
| | L_FR1 | | | | | | | | | | | | | | | | | | | | | | | | | | |
| CONSENSUS | S | Y | E | L | T | Q | P | - | P | S | V | S | V | S | P | G | Q | T | A | S | I | T | C | S | G | D | - |
| 21-225_14G3_LC | . | . | . | . | . | . | . | . | . | . | . | . | . | . | . | . | . | . | . | . | . | . | . | . | . | . | . |
| 21-225_14H11_LC | T | . | . | . | . | . | . | . | S | . | . | . | . | . | . | . | . | . | . | . | . | . | . | . | . | . | . |
| 21-225_150D3_LC | . | . | . | . | . | . | . | . | . | . | . | . | . | . | . | . | . | . | . | . | . | . | . | . | . | . | . |
| 21-225_150F6_LC | . | . | . | . | . | . | . | . | . | . | . | . | . | . | . | . | . | . | . | . | . | . | . | . | . | . | . |
| 21-225_150H11_LC | . | . | . | . | . | . | . | . | . | . | . | . | . | . | . | . | . | . | . | . | . | . | . | . | . | . | . |
| 21-225_151B9_LC | . | . | . | . | . | . | . | . | . | . | . | . | . | . | . | . | . | . | . | . | . | A | . | . | . | . | . |
| 21-225_151F3_LC | . | . | . | . | . | . | . | . | . | . | . | . | . | . | . | . | . | . | . | . | . | . | . | . | . | . | . |
| 21-225_151H5_LC | . | . | . | . | . | . | . | . | . | . | . | . | . | . | . | . | . | . | . | . | . | A | . | . | . | . | . |
| 21-225_151H7_LC | . | . | D | . | . | . | . | . | . | . | . | . | . | . | . | . | . | . | . | . | . | . | . | . | . | . | . |
| 21-225_152D7_LC | . | . | . | . | . | . | . | . | . | . | . | . | . | . | . | . | . | . | . | . | . | . | . | . | . | . | . |
| 21-225_152G6_LC | . | . | . | . | . | . | . | . | . | . | . | . | . | . | . | . | . | . | . | . | . | . | . | . | . | . | . |
| 21-225_153D9_LC | . | . | . | . | . | . | . | . | . | . | . | . | . | . | . | . | . | . | . | . | . | . | . | . | . | . | . |
| 21-225_153E6_LC | . | . | . | . | . | . | . | . | . | . | . | . | . | . | . | . | . | . | . | . | . | . | . | . | . | S | . |
| 21-225_154C3_LC | . | . | . | . | . | . | . | . | . | . | . | . | . | . | . | . | . | . | . | . | . | . | . | . | . | . | . |
| 21-225_154C4_LC | . | . | . | . | . | . | . | . | . | . | . | . | . | . | . | . | . | . | . | R | . | . | . | . | . | . | . |
| 21-225_154D11_LC | . | . | . | . | . | . | . | . | . | . | . | . | . | . | . | . | . | . | . | . | . | . | . | . | . | . | . |
| 21-225_154E10_LC | . | . | . | . | . | . | . | . | . | . | . | . | . | . | . | . | . | . | . | . | . | . | . | . | . | . | . |
| 21-225_154F4_LC | . | . | . | . | . | . | . | . | . | . | . | . | . | . | . | . | . | . | . | . | . | . | . | . | . | . | . |
| 21-225_155C10_LC | . | . | . | . | . | . | . | . | . | . | . | . | . | . | . | . | . | . | V | . | . | . | . | . | . | . | . |
| 21-225_155E10_LC | . | . | . | . | . | . | . | . | . | . | . | . | . | . | . | . | . | . | . | . | . | . | . | . | . | . | . |
| 21-225_158D10_LC | . | . | . | . | . | . | . | . | . | . | . | T | . | . | . | . | . | . | . | . | . | . | . | . | . | . | . |
| 21-225_158E9_LC | . | . | D | . | . | . | . | . | . | . | . | . | . | . | . | . | . | . | . | . | . | . | . | . | . | . | . |
| 21-225_159H8_LC | . | . | . | . | . | . | . | . | . | . | . | . | . | . | . | . | . | . | . | . | . | . | . | . | . | . | . |
| 21-225_15H11_LC | . | . | . | . | . | . | . | . | . | . | . | . | . | . | . | . | . | . | . | . | . | . | . | . | . | . | . |
| 21-225_160B12_LC | . | D | . | . | . | . | S | . | . | . | . | . | . | . | . | . | . | . | . | . | . | . | . | . | . | . | . |
| 21-225_161E10_LC | . | F | D | . | . | . | . | . | . | . | . | . | . | . | . | . | . | . | . | . | . | . | . | . | . | . | . |
| 21-225_161F12_LC | . | . | . | . | . | . | . | . | . | . | . | . | . | . | . | . | . | . | . | . | . | . | . | . | . | . | . |
| 21-225_161H3_LC | . | . | . | . | . | . | . | . | . | . | . | . | . | . | . | . | . | . | . | . | . | . | . | . | . | . | . |
| 21-225_165H3_LC | . | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 |
|---|---|---|---|---|---|---|---|---|---|---|---|---|---|---|---|---|---|---|---|---|---|---|---|---|---|---|---|
| | L_FR1 | | | | | | | | | | | | | | | | | | | | | | | | | | |
| CONSENSUS | S | Y | E | L | T | Q | P | - | P | S | V | S | V | S | P | G | Q | T | A | S | I | T | C | S | G | D | - |
| 21-225_166G11_LC | . | . | . | . | S | . | . | . | . | . | . | . | . | . | . | . | . | . | . | . | . | . | . | . | . | . | . |
| 21-225_169A6_LC | . | . | . | . | . | . | . | . | . | . | . | . | . | . | . | . | . | . | . | . | . | . | . | . | . | . | . |
| 21-225_169B7_LC | A | . | D | . | . | . | . | . | . | . | . | . | . | . | . | . | . | . | . | R | . | . | . | . | . | . | . |
| 21-225_169C1_LC | . | . | . | . | . | . | . | . | . | . | . | . | . | . | . | . | . | . | . | . | . | . | . | . | . | . | . |
| 21-225_169G11_LC | . | . | . | . | . | . | . | . | . | . | . | . | . | . | . | . | . | . | . | . | . | . | . | . | . | . | . |
| 21-225_16D2_LC | . | . | . | . | . | . | . | . | . | . | . | . | . | . | . | . | . | . | . | . | . | . | . | . | . | . | . |
| 21-225_16E4_LC | P | . | . | . | . | . | . | . | . | . | . | . | . | . | . | . | . | . | . | . | . | . | . | . | . | . | . |
| 21-225_16G8_LC | . | . | . | . | S | . | . | . | . | . | . | . | . | . | . | . | . | . | . | . | . | . | . | . | . | . | . |
| 21-225_170A5_LC | . | . | D | . | . | . | . | . | . | . | . | . | . | . | . | . | . | . | . | . | . | . | . | . | . | . | . |
| 21-225_170F1_LC | . | . | . | . | S | . | . | . | . | . | . | . | . | . | . | . | . | . | . | . | . | . | . | . | . | . | . |
| 21-225_171E12_LC | . | . | . | . | . | . | . | . | . | . | . | . | . | . | . | . | . | . | . | . | . | . | . | . | . | . | . |
| 21-225_171F5_LC | A | . | D | . | . | . | . | . | . | . | . | . | . | . | . | . | . | . | . | . | . | . | . | . | . | . | . |
| 21-225_173F8_LC | . | . | . | . | . | . | . | . | . | . | . | . | . | . | . | . | . | . | . | . | . | . | . | . | . | N | . |
| 21-225_175C6_LC | . | . | D | . | . | . | . | . | . | . | . | . | . | . | . | . | . | . | . | . | . | . | . | . | . | . | . |
| 21-225_179C7_LC | . | . | . | . | . | . | . | . | . | . | . | . | . | . | . | . | . | . | . | . | . | . | . | . | . | . | . |
| 21-225_17D3_LC | . | . | . | . | . | . | S | . | . | . | . | . | . | . | . | . | . | . | . | . | . | . | . | . | . | . | . |
| 21-225_17F5_LC | . | . | . | . | . | . | . | . | . | . | . | . | . | . | . | . | . | . | . | . | . | . | . | . | . | . | . |
| 21-225_180C5_LC | . | . | . | . | . | . | . | . | . | . | . | . | . | . | . | . | . | . | . | . | . | . | . | . | . | . | . |
| 21-225_180D4_LC | . | . | . | . | . | . | T | . | . | . | . | . | . | . | . | . | . | . | . | . | . | . | . | . | . | . | . |
| 21-225_180G5_LC | . | . | . | . | . | . | . | . | . | . | . | . | . | Y | . | . | . | . | . | . | . | . | . | . | . | . | . |
| 21-225_181H1_LC | . | . | . | . | . | . | T | . | . | . | . | . | . | . | . | . | . | . | . | . | . | . | . | . | . | . | . |
| 21-225_182D12_LC | . | . | . | . | . | . | . | . | . | . | . | . | . | . | . | . | . | . | . | . | . | . | . | . | . | . | . |
| 21-225_183F4_LC | . | . | . | . | . | . | . | . | . | . | . | . | . | . | . | . | . | . | . | . | . | . | . | . | . | . | . |
| 21-225_185D2_LC | . | . | . | . | . | . | T | . | . | . | . | . | . | . | . | . | . | . | . | . | . | . | . | . | . | . | . |
| 21-225_185G7_LC | . | . | . | . | . | . | . | . | . | . | . | . | . | . | . | . | . | . | . | . | . | . | . | . | . | . | . |
| 21-225_186H6_LC | . | . | . | . | . | . | . | . | . | . | . | . | . | . | . | . | . | . | . | . | . | . | . | . | . | . | . |
| 21-225_18E10_LC | . | . | . | . | . | . | . | . | . | . | . | . | . | . | . | . | . | . | . | . | . | . | . | . | . | . | . |
| 21-225_18G5_LC | . | . | . | . | . | . | . | . | . | . | . | . | . | . | . | . | . | . | . | . | . | . | . | . | . | E | . |
| 21-225_190G9_LC | . | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 |
|---|---|---|---|---|---|---|---|---|---|---|---|---|---|---|---|---|---|---|---|---|---|---|---|---|---|---|---|
| | L_FR1 | | | | | | | | | | | | | | | | | | | | | | | | | | |
| CONSENSUS | S | Y | E | L | T | Q | P | - | P | S | V | S | V | S | P | G | Q | T | A | S | I | T | C | S | G | D | - |
| 21-225_190H1_LC | . | . | . | . | . | . | . | . | . | . | . | . | . | . | . | . | . | . | . | . | . | . | . | . | . | . | . |
| 21-225_193H5_LC | . | . | . | . | . | . | . | . | S | . | . | . | . | . | . | . | . | . | . | . | . | . | . | . | . | . | . |
| 21-225_195E3_LC | . | . | . | . | . | . | . | . | . | . | . | . | . | . | . | . | . | . | . | . | . | . | . | . | . | . | . |
| 21-225_195E9_LC | . | . | . | . | . | . | . | . | . | . | . | . | . | . | . | . | . | . | . | . | . | . | . | . | . | . | . |
| 21-225_19F6_LC | . | . | . | . | . | . | . | . | . | . | . | . | . | . | . | . | . | . | V | . | . | . | . | . | . | . | . |
| 21-225_201G11_LC | . | . | . | . | . | . | . | . | . | . | . | . | . | . | . | . | . | . | . | . | . | . | . | . | . | . | . |
| 21-225_203G6_LC | . | . | . | . | . | . | . | . | . | . | . | . | . | . | . | . | . | . | . | . | . | . | . | . | . | . | . |
| 21-225_215B3_LC | . | . | . | . | . | . | . | . | . | . | . | . | . | . | . | . | . | . | . | . | . | . | . | . | . | . | . |
| 21-225_21D12_LC | . | . | . | . | . | . | . | . | . | . | . | . | . | . | . | . | . | . | . | . | . | . | . | . | . | . | . |
| 21-225_224H2_LC | . | . | . | . | . | . | . | . | S | . | . | . | . | . | . | . | . | . | . | . | . | . | . | . | . | . | . |
| 21-225_225E9_LC | . | . | D | . | . | . | . | . | . | . | . | . | . | . | . | . | . | . | . | . | . | . | . | . | . | . | . |
| 21-225_226A10_LC | . | . | . | . | . | . | . | . | . | . | . | . | . | . | . | . | . | . | . | . | . | . | . | . | . | . | . |
| 21-225_226B2_LC | . | . | . | . | . | . | . | . | . | . | . | . | . | . | . | . | . | . | . | . | . | . | . | . | . | . | . |
| 21-225_227E12_LC | . | . | . | . | . | . | . | . | . | . | . | . | . | . | . | . | . | . | . | . | . | . | . | . | . | . | . |
| 21-225_227E5_LC | . | . | . | . | S | . | . | . | . | . | . | . | . | . | . | . | . | . | . | . | . | . | . | . | . | . | . |
| 21-225_22E6_LC | . | . | . | . | . | . | . | . | . | . | . | . | . | . | . | . | . | . | . | . | . | . | . | . | . | . | . |
| 21-225_23D7_LC | . | . | . | . | . | . | . | . | . | . | . | . | . | . | . | . | . | . | . | . | . | . | . | . | . | E | . |
| 21-225_26C10_LC | . | . | . | V | . | . | . | . | . | . | M | . | . | . | . | . | . | . | . | . | . | . | . | . | . | . | . |
| 21-225_27D9_LC | . | . | . | . | . | . | . | . | . | . | M | . | . | . | . | . | . | . | . | . | . | . | . | . | . | Y | . |
| 21-225_27E7_LC | . | . | . | . | . | . | . | . | . | . | . | . | . | . | . | . | . | . | . | . | . | . | . | . | . | . | . |
| 21-225_27G6_LC | . | . | . | . | . | . | . | . | . | . | . | . | . | . | . | . | . | . | . | . | . | . | . | . | . | . | . |
| 21-225_27H2_LC | . | . | . | . | . | . | . | . | . | . | . | . | . | . | . | . | . | . | . | . | . | . | . | . | . | . | . |
| 21-225_28A11_LC | . | . | . | . | . | . | . | . | . | . | . | . | . | . | . | . | . | . | . | . | . | . | . | . | . | . | . |
| 21-225_2B1_LC | . | . | . | . | . | . | . | . | . | . | . | . | . | . | . | . | . | . | V | . | . | . | . | . | . | . | . |
| 21-225_30D8_LC2_LC | . | . | . | . | . | . | . | . | . | . | . | . | . | . | . | . | . | . | . | . | . | . | . | . | . | . | . |
| 21-225_30H6_LC | . | . | . | . | . | . | . | . | . | . | . | . | . | . | . | . | . | . | . | . | . | . | . | . | . | . | . |
| 21-225_31C2_LC | . | . | . | . | . | . | . | . | . | . | . | . | . | . | . | . | . | . | . | . | . | . | . | . | . | . | . |
| 21-225_31D12_LC2_LC | . | . | . | . | . | . | . | . | . | . | . | . | . | . | . | . | . | . | . | . | . | . | . | . | . | . | . |
| 21-225_32B11_LC | . | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 |
|---|---|---|---|---|---|---|---|---|---|---|---|---|---|---|---|---|---|---|---|---|---|---|---|---|---|---|---|
| | L_FR1 | | | | | | | | | | | | | | | | | | | | | | | | | | |
| CONSENSUS | S | Y | E | L | T | Q | P | - | P | S | V | S | V | S | P | G | Q | T | A | S | I | T | C | S | G | D | - |
| 21-225_33A1_LC | . | . | . | . | . | . | . | . | . | . | . | . | . | . | . | . | . | . | . | . | . | . | . | . | . | . | . |
| 21-225_33E6_LC | . | . | . | . | . | . | . | . | . | . | . | . | . | . | . | . | . | . | . | . | . | . | . | . | . | . | . |
| 21-225_34B11_LC | . | . | . | . | . | . | . | . | . | . | . | . | . | . | . | . | . | . | . | . | . | . | . | . | . | . | . |
| 21-225_34B9_LC | . | . | . | . | . | . | A | . | . | . | . | . | . | . | . | . | . | . | . | . | . | . | . | . | . | . | . |
| 21-225_34D7_LC | . | . | . | . | . | . | . | . | . | . | . | . | . | . | . | . | . | . | . | . | . | . | . | . | . | . | . |
| 21-225_35E1_LC | . | . | . | . | . | . | . | . | . | . | . | . | . | . | . | . | . | . | . | . | . | . | . | . | . | . | . |
| 21-225_3B12_LC | . | . | . | . | S | . | . | . | . | . | . | . | . | . | . | . | . | . | . | . | . | . | . | . | . | . | . |
| 21-225_3H10_LC | . | . | . | . | S | . | . | . | . | . | . | . | . | . | . | . | . | . | . | . | . | . | . | . | . | . | . |
| 21-225_45F6_LC | . | . | . | . | . | . | . | . | . | . | . | . | . | . | . | . | . | . | . | . | . | . | . | . | . | . | . |
| 21-225_4B3_LC | . | . | . | . | . | . | . | . | . | . | . | . | . | . | . | R | . | . | V | . | . | . | . | . | . | . | . |
| 21-225_50G9_LC | . | . | . | . | . | . | . | . | . | . | . | . | . | . | . | . | . | . | . | . | . | . | . | . | . | . | . |
| 21-225_52H1_LC | . | . | . | . | . | . | . | . | . | . | . | F | . | . | . | . | . | . | . | . | . | . | S | . | . | . | . |
| 21-225_53D10_LC | . | . | . | . | . | . | . | . | . | . | . | . | . | . | . | . | . | . | . | . | . | . | . | . | . | . | . |
| 21-225_57D9_LC | . | . | . | . | . | . | . | . | . | . | . | . | . | . | . | . | . | . | . | . | . | . | . | . | . | E | . |
| 21-225_57F1_LC | . | . | . | . | . | . | . | . | . | . | A | . | . | . | . | . | . | . | . | . | . | . | . | . | . | . | . |
| 21-225_57H11_LC | . | . | A | . | . | . | . | . | . | . | A | . | . | . | . | . | . | . | . | . | . | . | . | . | . | . | . |
| 21-225_58C1_LC | . | . | . | . | . | . | . | . | . | . | . | . | . | . | . | . | . | . | . | N | . | . | . | . | . | . | . |
| 21-225_58E7_LC | . | . | . | . | . | . | S | . | . | . | . | . | . | . | . | . | . | . | . | . | . | . | . | . | . | . | . |
| 21-225_58F7_LC | . | . | . | . | . | . | . | . | . | . | . | . | . | . | . | . | . | . | . | . | . | . | . | . | . | . | . |
| 21-225_58F8_LC | . | . | . | . | . | . | . | . | . | . | . | . | . | . | . | . | . | . | . | . | . | . | . | . | . | . | . |
| 21-225_58G11_LC | . | . | . | . | . | . | . | . | . | . | . | . | . | . | . | . | . | . | . | . | . | . | . | . | . | . | . |
| 21-225_59B11_LC | . | . | . | . | . | . | . | . | . | . | . | . | . | . | . | . | . | . | . | . | . | . | . | . | . | . | . |
| 21-225_59F2_LC | . | . | . | . | . | . | . | . | . | . | . | . | . | . | . | . | . | . | . | . | . | . | . | . | . | . | . |
| 21-225_5G7_LC | . | . | . | . | . | . | . | . | . | . | . | . | . | . | . | . | . | . | . | . | . | . | . | . | . | . | . |
| 21-225_60A12_LC | . | . | . | . | . | . | . | . | . | . | . | . | . | . | . | . | . | . | . | N | . | . | . | . | . | . | . |
| 21-225_60B1_LC | . | . | . | . | . | . | . | . | . | . | A | . | . | . | . | . | . | . | . | . | . | . | . | . | . | . | . |
| 21-225_60D2_LC | . | . | . | . | . | . | . | . | . | . | . | . | . | . | . | . | . | . | . | . | . | . | . | . | . | N | . |
| 21-225_61F5_LC | . | . | . | . | . | . | . | . | . | . | . | . | . | . | . | . | . | . | . | . | . | . | . | . | . | . | . |
| 21-225_62A12_LC | . | . | D | . | . | . | . | . | . | . | . | . | . | . | . | . | . | . | . | . | . | . | . | . | . | . | . |

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 |
|---|---|---|---|---|---|---|---|---|---|---|---|---|---|---|---|---|---|---|---|---|---|---|---|---|---|---|---|
| | L_FR1 | | | | | | | | | | | | | | | | | | | | | | | | | | |
| CONSENSUS | S | Y | E | L | T | Q | P | - | P | S | V | S | V | S | P | G | Q | T | A | S | I | T | C | S | G | D | - |
| 21-225_62B12_LC | . | . | D | . | . | . | . | . | . | . | . | . | . | . | . | . | . | . | . | . | . | . | . | . | . | . | . |
| 21-225_62D10_LC | . | . | D | . | . | . | . | . | . | . | . | . | . | . | . | . | . | . | . | . | . | . | . | . | . | . | . |
| 21-225_62E3_LC | . | . | D | . | . | . | . | . | . | . | . | . | . | . | . | . | . | . | . | . | . | . | . | . | . | . | . |
| 21-225_62E8_LC | . | . | D | . | . | . | . | . | . | . | . | . | . | . | . | . | . | . | . | . | . | . | . | . | . | . | . |
| 21-225_65E9_LC | . | . | . | . | . | . | . | . | . | . | . | . | . | . | . | . | . | . | . | . | . | . | . | . | . | . | . |
| 21-225_66G9_LC | . | . | D | . | . | . | . | . | . | . | . | T | . | . | . | . | . | . | . | . | . | . | . | . | . | . | . |
| 21-225_67F10_LC | . | . | . | . | . | . | . | . | . | . | . | . | . | . | . | . | . | . | . | . | . | . | . | . | . | . | . |
| 21-225_68D8_LC | . | . | . | . | . | . | . | . | . | . | . | . | . | . | . | . | . | . | . | . | . | . | . | . | . | . | . |
| 21-225_68G6_LC | . | . | . | V | . | . | . | . | . | . | . | . | . | . | . | . | . | . | . | . | . | . | . | . | . | . | . |
| 21-225_69B11_LC | . | . | . | . | . | . | . | . | . | . | . | . | . | . | . | . | . | A | . | . | . | . | . | . | . | . | . |
| 21-225_69B9_LC | . | . | . | . | . | . | . | . | . | . | . | . | . | . | . | . | . | . | . | . | . | . | . | . | . | . | . |
| 21-225_6B4_LC | . | . | . | . | . | . | . | . | . | . | . | . | . | . | . | R | . | . | V | . | . | . | . | . | . | . | . |
| 21-225_6D9_LC | . | . | . | . | . | . | . | . | . | . | . | . | . | . | . | . | . | . | . | . | . | . | . | . | . | . | . |
| 21-225_71D4_LC | . | . | . | . | . | . | . | . | . | . | . | . | . | . | . | . | . | . | . | . | . | . | . | . | . | . | . |
| 21-225_72B4_LC | . | . | . | . | . | . | . | . | . | . | . | . | . | . | . | . | . | . | . | . | . | . | . | . | . | . | . |
| 21-225_72D5_LC | . | . | D | . | . | . | . | . | . | . | . | . | . | . | . | . | . | . | . | . | . | . | . | . | . | . | . |
| 21-225_73C4_LC | . | . | D | . | . | . | . | . | . | . | . | . | . | . | . | . | . | . | . | . | . | . | . | . | . | . | . |
| 21-225_74A9_LC | . | . | . | . | . | . | . | . | . | . | . | . | . | . | . | . | . | . | . | . | . | . | . | . | . | . | . |
| 21-225_74B3_LC | . | . | . | . | . | . | . | . | . | . | . | . | . | . | . | . | . | . | . | . | . | . | . | . | . | . | . |
| 21-225_74C9_LC | . | . | . | . | . | . | . | . | . | . | E | . | . | . | . | . | . | . | . | . | . | . | . | . | . | . | . |
| 21-225_76B4_LC | P | . | . | . | N | . | T | . | . | . | . | . | . | . | . | . | . | . | . | . | . | . | . | . | . | . | . |
| 21-225_77A2_LC | . | . | . | . | . | . | . | . | . | . | . | . | . | . | . | . | . | . | . | . | . | . | . | . | . | . | . |
| 21-225_78E9_LC | . | . | . | . | . | . | . | . | . | . | E | . | . | . | . | . | . | . | . | . | . | . | . | . | . | . | . |
| 21-225_79E7_LC | . | . | . | . | . | . | . | . | . | . | . | . | . | . | . | . | . | . | . | . | . | . | . | . | . | . | . |
| 21-225_8D8_LC | . | . | . | . | . | . | . | . | . | . | . | . | . | . | . | . | . | . | . | . | . | . | . | . | . | . | . |
| 21-225_92A4_LC | . | . | . | . | . | . | . | . | . | . | . | . | . | . | . | . | . | . | . | N | . | . | . | . | . | . | . |
| 21-225_96B5_LC | P | . | . | . | N | . | T | . | . | . | . | . | . | . | . | . | . | . | . | . | . | . | . | . | . | . | . |
| 21-225_97E6_LC | . | . | . | . | . | . | . | . | . | . | . | . | . | . | . | . | . | . | V | . | . | . | . | . | . | . | . |
| 21-225_9H10_LC | . | . | . | . | . | . | . | . | . | . | . | . | . | . | . | . | . | . | . | . | . | . | . | . | . | . | . |

**FIGURE 57**

| Reference # | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 |
|---|---|---|---|---|---|---|---|---|---|---|---|---|---|---|---|---|---|---|---|---|---|---|---|---|---|---|---|
| | L_CDR1 | | | | | | | | | | | | | | | L_FR2 | | | | | | | | | | | |
| CONSENSUS | - | - | K | L | G | D | - | - | - | - | - | K | Y | A | C | W | Y | Q | Q | K | P | G | Q | S | P | V | L |
| 21-225_10B10_LC | . | . | . | . | . | N | . | . | . | . | . | . | . | . | W | . | . | . | . | . | . | . | . | . | . | . | . |
| 21-225_12B1_LC | . | . | . | . | . | . | . | . | . | . | . | . | . | . | . | . | . | . | . | . | . | . | . | . | . | . | . |
| 21-225_13F6_LC | . | . | . | . | . | . | . | . | . | . | . | . | . | . | . | . | . | . | . | . | . | . | . | . | . | . | . |
| 21-225_146A10_LC | . | . | . | . | . | . | . | . | . | . | . | . | . | V | . | . | . | . | . | . | . | . | . | . | . | . | . |
| 21-225_146A2_LC | . | . | . | . | . | . | . | . | . | . | . | . | . | . | S | . | . | . | . | . | . | . | . | . | . | . | . |
| 21-225_146A3_LC | . | . | . | . | . | N | . | . | . | . | . | R | . | . | . | . | . | . | . | . | . | . | . | . | . | . | . |
| 21-225_146B11_LC | . | . | . | . | . | . | . | . | . | . | . | . | . | . | S | . | . | . | . | . | . | . | . | . | . | . | . |
| 21-225_146B8_LC | . | . | . | . | . | . | . | . | . | . | . | . | . | V | . | . | . | . | . | . | . | . | . | . | . | . | . |
| 21-225_146C11_LC | . | . | . | . | . | . | . | . | . | . | . | . | . | . | S | . | . | . | . | . | . | . | . | . | . | . | . |
| 21-225_146H8_LC | . | . | . | . | . | . | . | . | . | . | . | . | . | . | . | . | . | . | . | . | . | . | . | . | . | . | . |
| 21-225_147B12_LC | . | . | . | . | . | . | . | . | . | . | . | . | . | . | S | . | . | . | . | . | . | . | . | . | . | . | . |
| 21-225_147B8_LC | . | . | . | . | . | . | . | . | . | . | . | . | . | V | . | . | . | . | . | . | . | . | . | . | . | E | . |
| 21-225_147B9_LC | . | . | . | . | . | . | . | . | . | . | . | . | . | V | S | . | . | . | . | R | . | . | . | . | . | . | . |
| 21-225_147D9_LC | . | . | . | . | . | N | . | . | . | . | . | . | . | V | . | . | . | . | . | R | . | . | . | . | . | . | . |
| 21-225_147E11_LC | . | . | . | . | . | . | . | . | . | . | . | . | . | . | S | . | . | . | . | . | . | . | . | . | . | . | . |
| 21-225_147E7_LC | . | . | . | . | . | . | . | . | . | . | . | . | . | . | S | . | . | . | . | . | . | . | . | . | . | . | . |
| 21-225_147F9_LC | . | . | E | . | . | N | . | . | . | . | . | . | . | . | . | . | . | . | . | . | . | . | . | . | . | . | . |
| 21-225_147G9_LC | . | . | . | . | . | . | . | . | . | . | . | . | . | . | . | . | . | . | . | . | . | . | . | . | . | . | . |
| 21-225_148A9_LC | . | . | . | . | . | N | . | . | . | . | . | . | . | V | . | . | . | . | . | . | . | . | . | . | . | . | . |
| 21-225_148C8_LC | . | . | . | . | . | . | . | . | . | . | . | . | . | V | S | . | . | . | . | . | . | . | . | . | . | I | . |
| 21-225_148E10_LC | . | . | . | . | . | N | . | . | . | . | . | . | . | V | . | . | . | . | . | R | . | . | H | A | A | . | . |
| 21-225_148G11_LC | . | . | . | . | . | . | . | . | . | . | . | . | F | . | S | . | . | . | . | . | . | . | . | . | . | . | . |
| 21-225_148G6_LC | . | . | . | . | . | . | . | . | . | . | . | . | . | . | S | . | . | . | . | . | . | . | . | . | . | . | . |
| 21-225_149A11_LC | . | . | . | . | . | N | . | . | . | . | . | . | . | V | S | . | . | . | . | . | . | . | . | . | . | . | . |
| 21-225_149B5_LC | . | . | . | . | . | Y | . | . | . | . | . | . | . | V | . | . | . | . | . | . | . | . | . | . | . | . | . |
| 21-225_149C10_LC | . | . | . | . | . | . | . | . | . | . | . | . | . | . | S | . | . | . | . | . | . | . | . | . | . | . | . |
| 21-225_149C7_LC | . | . | . | . | . | . | . | . | . | . | . | . | . | . | S | . | . | . | . | . | . | . | . | . | . | . | . |
| 21-225_14B1_LC2_LC | . | . | . | . | . | . | . | . | . | . | . | . | . | . | Y | . | . | . | . | . | . | . | . | . | . | . | . |
| 21-225_14B7_LC | . | . | . | . | . | N | . | . | . | . | . | . | . | . | Y | . | . | . | . | . | . | . | . | . | . | . | . |

FIGURE 57

| Reference # | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 |
|---|---|---|---|---|---|---|---|---|---|---|---|---|---|---|---|---|---|---|---|---|---|---|---|---|---|---|---|
| | L_CDR1 | | | | | | | | | | | | | | | L_FR2 | | | | | | | | | | | |
| CONSENSUS | - | - | K | L | G | D | - | - | - | - | - | K | Y | A | C | W | Y | Q | Q | K | P | G | Q | S | P | V | L |
| 21-225_14G3_LC | . | . | . | . | . | . | . | . | . | . | . | . | . | V | . | . | . | . | . | . | . | . | . | . | . | . | . |
| 21-225_14H11_LC | . | . | . | . | . | E | . | . | . | . | . | . | . | V | . | . | . | . | . | . | . | . | . | . | . | . | . |
| 21-225_150D3_LC | . | . | E | . | . | N | . | . | . | . | . | . | . | . | . | . | . | . | . | . | . | . | . | . | . | . | . |
| 21-225_150F6_LC | . | . | . | . | . | . | . | . | . | . | . | . | . | . | S | . | . | . | . | . | . | . | . | . | . | . | . |
| 21-225_150H11_LC | . | . | . | . | . | . | . | . | . | . | . | . | . | . | S | . | . | . | . | . | . | . | . | . | . | . | . |
| 21-225_151B9_LC | . | . | N | . | . | . | . | . | . | . | . | . | . | . | S | . | . | . | . | . | . | . | . | . | . | . | . |
| 21-225_151F3_LC | . | . | . | . | . | . | . | . | . | . | . | . | . | V | . | . | . | . | . | . | . | . | . | . | . | . | . |
| 21-225_151H5_LC | . | . | . | . | . | . | . | . | . | . | . | . | . | . | S | . | . | . | . | . | . | . | . | . | . | . | . |
| 21-225_151H7_LC | . | . | . | . | . | . | . | . | . | . | . | . | . | . | S | . | . | . | . | . | . | . | . | . | . | . | . |
| 21-225_152D7_LC | . | . | . | . | . | . | . | . | . | . | . | . | . | . | S | . | . | . | . | . | . | . | . | . | . | . | . |
| 21-225_152G6_LC | . | . | . | . | . | . | . | . | . | . | . | . | . | . | S | . | . | . | . | . | . | . | . | . | . | . | . |
| 21-225_153D9_LC | . | . | . | . | . | . | . | . | . | . | . | . | . | . | . | . | . | . | . | . | . | . | . | . | . | . | . |
| 21-225_153E8_LC | . | . | . | . | . | N | . | . | . | . | . | . | . | V | . | . | . | . | . | R | . | . | . | . | . | . | . |
| 21-225_154C3_LC | . | . | . | . | . | . | . | . | . | . | . | . | . | V | . | . | . | . | . | . | . | . | . | . | . | . | . |
| 21-225_154C4_LC | . | . | . | . | . | . | . | . | . | . | . | . | . | . | S | . | . | . | . | . | . | . | . | . | . | . | . |
| 21-225_154D11_LC | . | . | . | . | . | . | . | . | . | . | . | . | . | . | . | . | . | . | . | . | . | . | . | . | . | . | . |
| 21-225_154E10_LC | . | . | . | . | . | . | . | . | . | . | . | . | . | . | S | . | . | . | . | . | . | . | . | . | . | . | . |
| 21-225_154F4_LC | . | . | . | . | . | N | . | . | . | . | . | . | . | V | . | . | . | . | . | . | . | . | . | . | . | . | E |
| 21-225_155C10_LC | . | . | . | . | . | . | . | . | . | . | . | . | . | V | S | . | . | . | . | . | . | . | . | . | . | . | . |
| 21-225_155E10_LC | . | . | . | . | . | . | . | . | . | . | . | . | . | V | S | . | . | . | . | . | . | . | . | . | . | . | . |
| 21-225_158D10_LC | . | . | . | . | . | . | . | . | . | . | . | . | . | V | . | . | . | . | . | . | . | . | . | . | . | . | . |
| 21-225_158E9_LC | . | . | . | . | . | . | . | . | . | . | . | . | . | V | . | . | . | . | . | . | . | . | . | . | . | . | . |
| 21-225_159H8_LC | . | . | . | . | . | . | . | . | . | . | . | . | . | V | . | . | . | . | . | . | . | . | . | . | . | . | . |
| 21-225_15H11_LC | . | . | . | . | . | E | . | . | . | . | . | . | . | . | . | . | . | . | . | . | . | . | . | . | . | . | V |
| 21-225_160B12_LC | . | . | . | . | . | . | . | . | . | . | . | . | . | V | . | . | . | . | . | . | . | . | . | . | . | . | . |
| 21-225_161E10_LC | . | . | . | . | . | . | . | . | . | . | . | . | . | V | . | . | . | . | . | . | . | . | . | . | . | . | . |
| 21-225_161F12_LC | . | . | . | . | . | . | . | . | . | . | . | . | . | . | . | . | . | . | . | . | . | . | . | . | . | . | . |
| 21-225_161H3_LC | . | . | R | . | . | . | . | . | . | . | . | . | . | V | . | . | . | . | . | . | . | . | . | . | . | . | . |
| 21-225_165H3_LC | . | . | . | . | . | . | . | . | . | . | . | . | . | . | . | . | . | . | . | . | . | . | . | . | . | . | . |

FIGURE 57

| Reference # | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 |
|---|---|---|---|---|---|---|---|---|---|---|---|---|---|---|---|---|---|---|---|---|---|---|---|---|---|---|---|
| | L_CDR1 | | | | | | | | | | | | | | | L_FR2 | | | | | | | | | | | |
| CONSENSUS | - | - | K | L | G | D | - | - | - | - | - | K | Y | A | C | W | Y | Q | Q | K | P | G | Q | S | P | V | L |
| 21-225_166G11_LC | . | . | . | . | . | . | . | . | . | . | . | . | . | V | H | . | . | . | . | . | . | . | . | . | . | . | . |
| 21-225_169A6_LC | . | . | . | . | . | . | . | . | . | . | . | . | . | V | . | . | . | . | R | . | . | . | . | . | . | . | . |
| 21-225_169B7_LC | . | . | . | . | . | G | . | . | . | . | . | . | . | . | S | . | . | . | . | . | . | . | . | . | . | . | . |
| 21-225_169C1_LC | . | . | . | . | . | . | . | . | . | . | . | . | . | V | . | . | . | . | . | . | . | . | . | . | . | L | . |
| 21-225_169G11_LC | . | . | . | . | . | . | . | . | . | . | . | . | . | . | . | . | . | . | . | . | . | . | . | . | . | . | . |
| 21-225_16D2_LC | . | . | . | . | . | . | . | . | . | . | . | . | . | . | . | . | . | . | . | . | . | . | . | . | . | . | . |
| 21-225_16E4_LC | . | . | . | . | . | . | . | . | . | . | . | . | . | V | Y | . | . | . | . | . | S | . | . | . | . | . | . |
| 21-225_16G8_LC | . | . | . | . | . | E | . | . | . | . | . | . | . | . | . | . | . | . | . | . | . | . | . | . | . | . | . |
| 21-225_170A5_LC | . | . | . | . | . | . | . | . | . | . | . | . | . | . | . | . | . | . | . | . | . | . | . | . | . | I | . |
| 21-225_170F1_LC | . | . | . | . | . | . | . | . | . | . | . | . | . | S | . | . | . | . | . | . | . | . | . | . | . | . | . |
| 21-225_171E12_LC | . | . | . | . | . | . | . | . | . | . | . | . | . | . | . | . | . | . | . | . | . | . | . | . | . | . | . |
| 21-225_171F5_LC | . | . | . | . | . | G | . | . | . | . | . | . | . | . | S | . | . | . | . | . | . | . | . | . | . | . | . |
| 21-225_173F8_LC | . | . | . | . | . | N | . | . | . | . | . | . | . | V | . | . | . | . | . | R | . | . | . | . | . | . | . |
| 21-225_175C6_LC | . | . | . | . | . | . | . | . | . | . | . | . | . | . | . | . | . | . | . | . | . | . | . | . | . | I | . |
| 21-225_179C7_LC | . | . | . | . | . | . | . | . | . | . | . | . | . | V | . | . | . | . | . | R | . | . | . | . | . | . | . |
| 21-225_17D3_LC | . | . | . | . | . | . | . | . | . | . | . | . | . | V | Y | . | . | . | . | . | . | . | . | . | . | . | V |
| 21-225_17F5_LC | . | . | . | . | . | E | . | . | . | . | . | . | . | . | . | . | . | . | . | . | . | . | . | . | . | . | . |
| 21-225_180C5_LC | . | . | . | . | . | E | . | . | . | . | . | . | . | . | . | . | . | . | . | . | . | . | . | . | . | . | . |
| 21-225_180D4_LC | . | . | R | . | . | . | . | . | . | . | . | . | . | . | . | . | . | . | . | . | . | . | . | . | . | . | . |
| 21-225_180G5_LC | . | . | . | . | . | . | . | . | . | . | . | . | . | V | S | . | . | . | . | . | . | . | . | . | . | . | . |
| 21-225_181H1_LC | . | . | . | . | . | . | . | . | . | . | . | . | . | . | . | . | . | . | . | . | . | . | . | . | . | . | . |
| 21-225_182D12_LC | . | . | . | . | . | E | . | . | . | . | . | . | . | . | . | . | . | . | . | . | S | . | . | . | . | . | . |
| 21-225_183F4_LC | . | . | . | . | . | . | . | . | . | . | . | . | . | . | . | . | . | . | . | . | . | . | . | . | . | . | . |
| 21-225_185D2_LC | . | . | . | . | . | . | . | . | . | . | . | . | . | . | . | . | . | . | . | . | . | . | . | . | . | . | . |
| 21-225_185G7_LC | . | . | . | . | . | H | . | . | . | . | . | . | F | V | . | . | . | . | . | . | . | . | . | . | . | . | . |
| 21-225_186H6_LC | . | . | . | M | . | E | . | . | . | . | . | . | . | . | . | . | . | . | . | . | . | . | . | . | . | . | . |
| 21-225_18E10_LC | . | . | R | . | . | . | . | . | . | . | . | . | . | . | W | . | . | . | . | . | . | . | . | . | . | . | . |
| 21-225_18G5_LC | . | . | . | . | . | . | . | . | . | . | . | . | . | V | . | . | . | . | . | . | . | . | . | . | . | . | . |
| 21-225_190G9_LC | . | . | . | . | . | . | . | . | . | . | . | R | F | . | Y | . | . | . | . | . | . | . | . | . | . | . | . |

# FIGURE 57

| Reference # | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 |
|---|---|---|---|---|---|---|---|---|---|---|---|---|---|---|---|---|---|---|---|---|---|---|---|---|---|---|---|
| | | | | | | L_CDR1 | | | | | | | | | | | | | | | | | L_FR2 | | | | |
| CONSENSUS | - | - | K | L | G | D | - | - | - | - | - | K | Y | A | C | W | Y | Q | Q | K | P | G | Q | S | P | V | L |
| 21-225_190H1_LC | . | . | . | . | . | . | . | . | . | . | . | R | F | . | Y | . | . | . | . | . | . | . | . | . | . | . | . |
| 21-225_193H5_LC | . | . | . | . | . | . | . | . | . | . | . | R | F | . | . | . | . | . | . | . | . | . | . | . | . | . | . |
| 21-225_195E3_LC | . | . | . | . | . | Y | . | . | . | . | . | R | S | V | . | . | . | . | . | . | . | . | . | . | . | . | . |
| 21-225_195E9_LC | . | . | . | . | . | N | . | . | . | . | . | . | . | . | Y | . | . | . | . | . | . | . | . | . | . | . | . |
| 21-225_19F6_LC | . | . | . | . | . | . | . | . | . | . | . | . | . | . | . | . | . | . | . | . | . | . | . | . | . | . | . |
| 21-225_201G11_LC | . | . | . | . | . | . | . | . | . | . | . | R | F | . | . | . | . | . | . | . | . | . | . | . | . | . | . |
| 21-225_203G6_LC | . | . | . | . | . | . | . | . | . | . | . | R | F | . | . | . | . | . | . | . | . | . | . | . | . | . | . |
| 21-225_215B3_LC | . | . | . | . | . | . | . | . | . | . | . | . | F | . | . | . | . | . | . | . | . | . | . | . | . | . | . |
| 21-225_21D12_LC | . | . | . | . | . | N | . | . | . | . | . | . | . | . | Y | . | . | . | . | . | . | . | . | . | . | . | . |
| 21-225_224H2_LC | . | . | N | . | . | V | . | . | . | . | . | . | . | T | Y | . | . | . | . | . | . | . | . | . | . | . | . |
| 21-225_225E9_LC | . | . | N | . | . | N | . | . | . | . | . | R | . | . | . | . | . | . | . | . | . | . | . | . | . | . | . |
| 21-225_226A10_LC | . | . | T | . | . | G | . | . | . | . | . | . | . | . | W | . | . | . | . | . | . | . | . | . | . | . | . |
| 21-225_226B2_LC | . | . | T | . | . | G | . | . | . | . | . | . | . | . | W | . | . | . | . | R | . | . | . | . | . | . | . |
| 21-225_227E12_LC | . | . | . | . | . | . | . | . | . | . | . | . | . | V | . | . | . | . | . | . | . | . | . | . | . | . | . |
| 21-225_227E5_LC | . | . | . | . | . | E | . | . | . | . | . | . | . | . | . | . | . | . | . | . | . | . | . | . | . | . | . |
| 21-225_22E6_LC | . | . | . | . | . | E | . | . | . | . | . | . | . | . | . | . | . | . | . | . | . | . | . | . | . | . | . |
| 21-225_23D7_LC | . | . | . | . | . | . | . | . | . | . | . | . | . | V | . | . | . | . | . | . | . | . | . | . | . | . | V |
| 21-225_26C10_LC | . | . | . | . | . | . | . | . | . | . | . | . | . | V | . | . | F | . | . | . | . | . | . | . | . | . | . |
| 21-225_27D9_LC | . | . | . | . | . | . | . | . | . | . | . | . | . | . | . | . | F | . | . | . | . | . | . | . | . | . | . |
| 21-225_27E7_LC | . | . | . | . | . | N | . | . | . | . | . | . | . | . | . | . | . | . | . | . | . | . | . | . | . | . | E |
| 21-225_27G6_LC | . | . | . | . | . | . | . | . | . | . | . | . | . | . | . | . | . | . | . | . | . | . | . | . | . | . | . |
| 21-225_27H2_LC | . | . | . | . | . | . | . | . | . | . | . | . | . | . | S | . | . | . | . | . | . | . | . | . | . | . | . |
| 21-225_28A11_LC | . | . | . | . | . | . | . | . | . | . | . | . | . | . | . | . | . | . | . | . | . | . | . | . | . | . | . |
| 21-225_2B1_LC | . | . | . | . | . | . | . | . | . | . | . | . | . | . | . | . | . | . | . | . | . | . | . | . | . | . | . |
| 21-225_30D8_LC2_LC | . | . | . | . | . | . | . | . | . | . | . | . | . | V | Y | . | F | . | . | . | . | . | . | . | . | . | . |
| 21-225_30H6_LC | . | . | . | . | . | N | . | . | . | . | . | . | . | . | . | . | . | . | . | . | . | . | . | . | . | . | . |
| 21-225_31C2_LC | . | . | . | . | . | N | . | . | . | . | . | . | . | . | . | . | . | . | . | . | . | . | . | . | . | . | . |
| 21-225_31D12_LC2_LC | . | . | . | . | . | . | . | . | . | . | . | . | . | . | Y | . | F | . | . | . | . | . | . | . | . | . | I |
| 21-225_32B11_LC | . | . | . | . | . | . | . | . | . | . | . | . | . | . | Y | . | . | . | . | . | . | . | . | . | . | . | . |

**FIGURE 57**

| Reference # | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 |
|---|---|---|---|---|---|---|---|---|---|---|---|---|---|---|---|---|---|---|---|---|---|---|---|---|---|---|---|
| | L_CDR1 | | | | | | | | | | | | | | | L_FR2 | | | | | | | | | | | |
| CONSENSUS | - | - | K | L | G | D | - | - | - | - | - | K | Y | A | C | W | Y | Q | Q | K | P | G | Q | S | P | V | L |
| 21-225_33A1_LC | . | . | . | . | . | . | . | . | . | . | . | . | F | V | Y | . | . | . | . | . | . | . | . | . | . | . | . |
| 21-225_33E6_LC | . | . | . | . | . | . | . | . | . | . | . | . | . | . | Y | . | F | . | . | . | . | . | . | . | . | . | I |
| 21-225_34B11_LC | . | . | . | . | . | . | . | . | . | . | . | . | . | . | . | . | . | . | . | . | . | . | . | . | . | . | V |
| 21-225_34B9_LC | . | . | . | . | . | E | . | . | . | . | . | . | . | V | S | . | . | . | E | . | . | . | . | . | . | . | . |
| 21-225_34D7_LC | . | . | . | . | . | E | . | . | . | . | . | . | . | . | Y | . | . | . | . | . | . | . | . | . | . | . | . |
| 21-225_35E1_LC | . | . | . | . | . | E | . | . | . | . | . | . | . | . | Y | . | F | . | . | . | . | . | . | . | . | . | I |
| 21-225_3B12_LC | . | . | . | . | . | E | . | . | . | . | . | . | . | . | . | . | . | . | . | . | . | . | . | . | . | . | . |
| 21-225_3H10_LC | . | . | . | . | . | E | . | . | . | . | . | . | . | . | . | . | . | . | . | . | . | . | . | . | . | . | . |
| 21-225_45F6_LC | . | . | . | . | . | . | . | . | . | . | . | . | . | . | S | . | . | . | . | . | . | . | . | . | . | . | . |
| 21-225_4B3_LC | . | . | . | . | . | . | . | . | . | . | . | . | . | . | . | . | . | . | . | . | . | . | . | . | . | . | . |
| 21-225_50G9_LC | . | . | . | . | . | . | . | . | . | . | . | . | . | . | . | . | . | . | . | . | . | . | . | . | . | . | . |
| 21-225_52H1_LC | . | . | . | . | . | . | . | . | . | . | . | . | . | V | S | . | . | . | . | . | . | . | . | . | . | . | . |
| 21-225_53D10_LC | . | . | . | . | . | N | . | . | . | . | . | . | . | . | . | . | . | . | . | R | . | . | . | . | . | . | . |
| 21-225_57D9_LC | . | . | . | . | . | E | . | . | . | . | . | . | F | . | . | . | S | . | . | . | . | . | . | . | . | . | . |
| 21-225_57F1_LC | . | . | . | . | . | E | . | . | . | . | . | . | . | . | . | . | . | . | . | . | . | . | . | . | . | . | . |
| 21-225_57H11_LC | . | . | . | . | . | E | . | . | . | . | . | . | . | . | . | . | . | . | . | . | . | . | . | . | . | . | . |
| 21-225_58C1_LC | . | . | . | . | . | N | . | . | . | . | . | . | . | . | . | . | . | . | . | . | . | . | . | . | . | . | . |
| 21-225_58E7_LC | . | . | . | . | . | . | . | . | . | . | . | . | . | T | . | . | . | . | K | . | . | . | . | . | . | . | . |
| 21-225_58F7_LC | . | . | . | . | . | . | . | . | . | . | . | . | . | . | . | . | . | . | . | . | . | . | . | . | . | . | . |
| 21-225_58F8_LC | . | . | . | . | . | N | . | . | . | . | . | . | . | . | . | . | . | . | . | . | . | . | . | . | . | . | . |
| 21-225_58G11_LC | . | . | . | . | . | . | . | . | . | . | . | . | . | . | S | . | . | . | . | . | . | . | . | . | . | . | . |
| 21-225_59B11_LC | . | . | . | . | . | . | . | . | . | . | . | . | . | . | . | . | . | . | . | . | . | . | . | . | . | . | . |
| 21-225_59F2_LC | . | . | . | . | . | . | . | . | . | . | . | . | . | . | S | . | . | . | . | R | . | . | . | . | . | . | . |
| 21-225_5G7_LC | . | . | . | . | . | N | . | . | . | . | . | . | . | . | W | . | . | . | . | . | . | . | . | . | . | . | . |
| 21-225_60A12_LC | . | . | . | . | . | N | . | . | . | . | . | . | . | . | . | . | . | . | . | . | . | . | . | . | . | . | . |
| 21-225_60B1_LC | . | . | . | . | . | E | . | . | . | . | . | . | . | . | . | . | . | . | . | . | . | . | . | . | . | . | . |
| 21-225_60D2_LC | . | . | . | . | . | . | . | . | . | . | . | . | . | . | S | . | . | . | . | R | . | . | . | . | . | . | . |
| 21-225_61F5_LC | . | . | . | . | . | E | . | . | . | . | . | . | . | . | . | . | . | . | . | . | . | . | . | . | . | . | . |
| 21-225_62A12_LC | . | . | . | . | . | N | . | . | . | . | . | . | . | . | . | . | . | . | . | . | . | . | . | . | . | . | V |

# FIGURE 57

| Reference # | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 |
|---|---|---|---|---|---|---|---|---|---|---|---|---|---|---|---|---|---|---|---|---|---|---|---|---|---|---|---|
| | L_CDR1 | | | | | | | | | | | | | | | L_FR2 | | | | | | | | | | | |
| CONSENSUS | - | - | K | L | G | D | - | - | - | - | - | K | Y | A | C | W | Y | Q | Q | K | P | G | Q | S | P | V | L |
| 21-225_62B12_LC | . | . | . | . | . | N | . | . | . | . | . | . | . | T | . | . | . | . | . | . | . | . | . | . | . | . | V |
| 21-225_62D10_LC | . | . | . | . | . | N | . | . | . | . | . | . | . | . | . | . | . | . | . | . | . | . | . | . | . | . | V |
| 21-225_62E3_LC | . | . | . | . | . | N | . | . | . | . | . | . | . | . | . | . | . | . | . | . | . | . | . | . | . | . | V |
| 21-225_62E8_LC | . | . | R | . | . | N | . | . | . | . | . | . | . | . | S | . | . | . | . | . | . | . | . | . | . | . | V |
| 21-225_65E9_LC | . | . | . | . | . | N | . | . | . | . | . | . | . | D | Y | . | . | . | . | . | . | . | . | . | . | . | . |
| 21-225_66G9_LC | . | . | . | . | . | N | . | . | . | . | . | . | . | . | . | . | . | . | . | . | . | . | . | . | . | . | V |
| 21-225_67F10_LC | . | . | . | . | . | Y | . | . | . | . | . | . | . | . | W | . | . | . | . | . | . | . | . | . | . | . | . |
| 21-225_68D8_LC | . | . | . | . | . | . | . | . | . | . | . | . | . | . | . | . | . | . | . | . | . | . | . | . | . | . | . |
| 21-225_68G6_LC | . | . | . | . | . | . | . | . | . | . | . | . | . | T | Y | . | . | . | . | . | A | . | . | . | . | F | . |
| 21-225_69B11_LC | . | . | . | . | . | . | . | . | . | . | . | . | . | . | W | . | . | . | . | . | . | . | . | . | . | . | . |
| 21-225_69B9_LC | . | . | . | . | . | N | . | . | . | . | . | . | . | D | Y | . | . | . | . | . | . | . | . | . | . | . | . |
| 21-225_6B4_LC | . | . | . | . | . | . | . | . | . | . | . | . | . | . | . | . | . | . | . | . | . | . | . | . | . | . | . |
| 21-225_6D9_LC | . | . | . | . | . | E | . | . | . | . | . | . | . | . | . | . | . | . | . | . | . | . | . | . | . | . | . |
| 21-225_71D4_LC | . | . | . | . | . | . | . | . | . | . | . | . | . | . | Y | . | . | . | . | . | . | . | . | . | . | . | V |
| 21-225_72B4_LC | . | . | . | . | . | . | . | . | . | . | . | . | . | . | W | . | . | . | . | R | . | . | . | . | . | . | . |
| 21-225_72D5_LC | . | . | . | . | . | N | . | . | . | . | . | . | . | . | . | . | . | . | . | . | . | . | . | . | . | . | V |
| 21-225_73C4_LC | . | . | . | . | . | N | . | . | . | . | . | . | . | . | . | . | . | . | . | . | . | . | . | . | . | . | V |
| 21-225_74A9_LC | . | . | . | . | . | N | . | . | . | . | . | . | . | V | . | . | . | . | . | . | . | . | . | . | . | . | . |
| 21-225_74B3_LC | . | . | . | . | . | . | . | . | . | . | . | . | . | . | . | . | . | . | . | . | . | . | . | . | . | . | . |
| 21-225_74C9_LC | . | . | . | S | . | N | . | . | . | . | . | . | . | V | S | . | . | . | . | . | . | . | . | . | . | . | . |
| 21-225_76B4_LC | . | . | R | . | . | T | . | . | . | . | . | . | F | . | . | . | . | . | . | . | . | . | . | . | . | . | . |
| 21-225_77A2_LC | . | . | R | . | . | . | . | . | . | . | . | . | . | . | . | . | . | . | . | . | . | . | . | . | . | . | . |
| 21-225_78E9_LC | . | . | . | . | . | N | . | . | . | . | . | . | . | V | S | . | . | . | . | . | . | . | . | . | . | . | . |
| 21-225_79E7_LC | . | . | . | . | . | N | . | . | . | . | . | . | . | V | S | . | . | . | . | . | . | . | . | . | . | . | . |
| 21-225_8D8_LC | . | . | . | . | . | E | . | . | . | . | . | . | . | . | . | . | . | . | . | . | . | . | . | . | . | . | V |
| 21-225_92A4_LC | . | . | . | . | . | N | . | . | . | . | . | . | . | V | . | . | . | . | . | . | . | . | . | . | . | . | . |
| 21-225_96B5_LC | . | . | R | . | . | T | . | . | . | . | . | . | F | . | . | . | . | . | . | . | . | . | . | . | . | . | . |
| 21-225_97E6_LC | . | . | . | . | . | N | . | . | . | . | . | . | . | V | S | . | . | . | . | . | . | . | . | . | . | . | . |
| 21-225_9H10_LC | . | . | . | . | . | . | . | . | . | . | . | . | . | . | . | . | . | . | . | . | . | . | . | . | . | . | . |

FIGURE 57

| Reference # | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 | 81 |
|---|---|---|---|---|---|---|---|---|---|---|---|---|---|---|---|---|---|---|---|---|---|---|---|---|---|---|---|
| | | | | L_CDR2 | | | | | | | | | | | | | | | | | | | | | | | |
| CONSENSUS | V | I | Y | Q | - | - | - | - | - | - | - | - | D | R | K | R | P | S | G | I | P | E | R | F | S | G | S |
| 21-225_10B10_LC | . | . | . | . | . | . | . | . | . | . | . | . | . | . | . | . | . | . | . | . | . | . | . | . | . | . | . |
| 21-225_12B1_LC | . | . | . | . | . | . | . | . | . | . | . | . | . | . | . | . | . | . | . | . | . | . | . | . | . | . | . |
| 21-225_13F6_LC | . | . | . | . | . | . | . | . | . | . | . | . | . | S | . | . | . | . | . | . | . | . | . | . | . | . | . |
| 21-225_146A10_LC | . | . | F | . | . | . | . | . | . | . | . | . | . | . | . | . | . | . | . | . | . | . | . | . | . | . | . |
| 21-225_146A2_LC | . | . | . | . | . | . | . | . | . | . | . | . | . | S | . | . | . | . | . | . | . | . | . | . | . | . | . |
| 21-225_146A3_LC | . | . | . | . | . | . | . | . | . | . | . | . | . | S | . | . | . | . | . | . | . | . | . | . | . | . | . |
| 21-225_146B11_LC | . | . | . | . | . | . | . | . | . | . | . | . | . | S | . | . | . | . | . | . | . | . | . | . | . | . | . |
| 21-225_146B8_LC | . | . | . | . | . | . | . | . | . | . | . | . | . | . | . | . | . | . | . | . | . | . | . | . | . | . | . |
| 21-225_146C11_LC | . | . | . | . | . | . | . | . | . | . | . | . | . | S | . | . | . | . | . | . | . | . | . | . | . | . | . |
| 21-225_146H8_LC | . | . | . | . | . | . | . | . | . | . | . | . | . | K | . | . | . | . | . | . | . | . | . | . | . | . | . |
| 21-225_147B12_LC | . | . | . | . | . | . | . | . | . | . | . | . | . | S | . | . | . | . | . | . | . | . | . | . | . | . | . |
| 21-225_147B8_LC | . | . | . | . | . | . | . | . | . | . | . | . | . | . | . | . | . | . | . | . | . | . | . | . | . | . | . |
| 21-225_147B9_LC | . | . | . | . | . | . | . | . | . | . | . | . | . | . | . | . | . | . | . | . | . | . | . | . | . | . | . |
| 21-225_147D9_LC | . | . | . | . | . | . | . | . | . | . | . | . | . | S | . | . | . | . | . | . | . | . | . | . | . | . | . |
| 21-225_147E11_LC | . | . | . | . | . | . | . | . | . | . | . | . | . | S | . | . | . | . | . | . | . | . | . | . | . | . | . |
| 21-225_147E7_LC | . | . | . | . | . | . | . | . | . | . | . | . | . | S | . | . | . | . | . | . | . | . | . | . | . | . | . |
| 21-225_147F9_LC | . | . | . | . | . | . | . | . | . | . | . | . | . | S | . | . | . | . | . | . | . | . | . | . | . | . | . |
| 21-225_147G9_LC | . | . | . | . | . | . | . | . | . | . | . | . | . | . | . | . | . | . | . | . | . | . | . | . | . | . | . |
| 21-225_148A9_LC | . | . | . | . | . | . | . | . | . | . | . | . | . | S | . | . | . | . | . | . | . | . | . | . | . | . | . |
| 21-225_148C8_LC | . | . | . | . | . | . | . | . | . | . | . | . | . | . | . | . | . | . | . | T | . | . | . | . | . | . | . |
| 21-225_148E10_LC | I | . | . | . | . | . | . | . | . | . | . | . | . | S | . | . | . | . | . | . | . | . | . | . | . | . | . |
| 21-225_148G11_LC | . | . | . | . | . | . | . | . | . | . | . | . | . | S | . | . | . | . | . | . | . | . | . | . | . | . | . |
| 21-225_148G6_LC | . | . | . | . | . | . | . | . | . | . | . | . | . | S | . | . | . | . | . | . | . | . | . | . | . | . | . |
| 21-225_149A11_LC | . | . | . | . | . | . | . | . | . | . | . | . | . | S | R | . | . | . | . | . | . | . | . | . | . | . | . |
| 21-225_149B5_LC | . | . | F | . | . | . | . | . | . | . | . | . | N | N | Q | . | . | . | . | . | . | . | . | . | . | . | . |
| 21-225_149C10_LC | . | . | . | . | . | . | . | . | . | . | . | . | . | N | . | . | . | . | . | . | . | . | . | . | . | . | . |
| 21-225_149C7_LC | . | . | . | . | . | . | . | . | . | . | . | . | . | S | . | . | . | . | . | . | . | . | . | . | . | . | . |
| 21-225_14B1_LC2_LC | . | . | . | . | . | . | . | . | . | . | . | . | . | . | . | . | . | . | . | . | . | . | . | . | . | . | . |
| 21-225_14B7_LC | . | . | . | . | . | . | . | . | . | . | . | . | . | . | . | . | . | . | . | . | . | . | . | . | . | . | . |

FIGURE 57

| Reference # | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 | 81 |
|---|---|---|---|---|---|---|---|---|---|---|---|---|---|---|---|---|---|---|---|---|---|---|---|---|---|---|---|
| | | | | | | | | | | | L_CDR2 | | | | | | | | | | | | | | | | |
| CONSENSUS | V | I | Y | Q | - | - | - | - | - | - | - | - | D | R | K | R | P | S | G | I | P | E | R | F | S | G | S |
| 21-225_14G3_LC | . | . | . | . | . | . | . | . | . | . | . | . | . | . | . | . | . | . | . | . | . | . | . | . | . | . | . |
| 21-225_14H11_LC | . | . | . | . | . | . | . | . | . | . | . | . | . | T | . | . | . | . | . | . | . | . | . | . | . | . | . |
| 21-225_150D3_LC | . | . | . | . | . | . | . | . | . | . | . | . | . | N | . | . | . | . | . | . | . | . | . | . | . | . | . |
| 21-225_150F6_LC | . | . | C | . | . | . | . | . | . | . | . | . | . | S | . | . | . | . | . | . | . | . | . | . | . | . | . |
| 21-225_150H11_LC | . | . | . | . | . | . | . | . | . | . | . | . | . | S | . | . | . | . | . | . | . | . | . | . | . | . | . |
| 21-225_151B9_LC | . | . | . | . | . | . | . | . | . | . | . | . | . | S | . | . | . | . | . | . | . | . | . | . | . | . | . |
| 21-225_151F3_LC | . | . | . | . | . | . | . | . | . | . | . | . | . | . | . | . | . | . | . | . | . | . | . | . | . | . | . |
| 21-225_151H5_LC | . | . | . | . | . | . | . | . | . | . | . | . | . | S | . | . | . | . | . | . | . | . | . | . | . | . | . |
| 21-225_151H7_LC | . | . | . | . | . | . | . | . | . | . | . | . | . | S | . | . | . | . | . | . | . | . | . | . | . | . | . |
| 21-225_152D7_LC | . | . | . | . | . | . | . | . | . | . | . | . | . | S | . | . | . | . | . | . | . | . | . | . | . | . | . |
| 21-225_152G6_LC | . | . | . | . | . | . | . | . | . | . | . | . | . | S | . | . | . | . | . | . | . | . | . | . | . | . | . |
| 21-225_153D9_LC | . | . | . | . | . | . | . | . | . | . | . | . | . | . | . | . | . | . | . | . | . | . | . | . | . | . | . |
| 21-225_153E6_LC | . | . | . | . | . | . | . | . | . | . | . | . | . | N | . | . | . | . | . | . | . | . | . | . | . | . | . |
| 21-225_154C3_LC | . | . | . | . | . | . | . | . | . | . | . | . | . | . | . | . | . | . | . | . | . | . | . | . | . | . | . |
| 21-225_154C4_LC | . | . | . | . | . | . | . | . | . | . | . | . | . | S | . | . | . | . | . | . | . | . | . | . | . | . | . |
| 21-225_154D11_LC | . | . | . | . | . | . | . | . | . | . | . | . | . | K | . | . | . | . | . | . | . | . | . | . | . | . | . |
| 21-225_154E10_LC | . | . | . | . | . | . | . | . | . | . | . | . | . | S | . | . | . | . | . | . | . | . | . | . | . | . | . |
| 21-225_154F4_LC | . | . | . | K | . | . | . | . | . | . | . | . | . | S | . | . | . | . | . | . | . | . | . | . | . | . | . |
| 21-225_155C10_LC | . | . | . | . | . | . | . | . | . | . | . | . | . | S | . | . | . | . | . | . | . | . | . | . | . | . | . |
| 21-225_155E10_LC | . | . | . | . | . | . | . | . | . | . | . | . | . | . | . | . | . | . | . | T | . | . | . | . | . | . | . |
| 21-225_158D10_LC | . | . | . | . | . | . | . | . | . | . | . | . | . | . | . | . | . | . | . | . | . | . | . | L | . | . | . |
| 21-225_158E9_LC | . | . | . | . | . | . | . | . | . | . | . | . | . | S | . | . | . | . | . | . | . | . | . | . | . | . | . |
| 21-225_159H8_LC | . | . | . | . | . | . | . | . | . | . | . | . | . | . | . | . | . | . | . | . | . | . | . | . | . | . | . |
| 21-225_15H11_LC | . | . | . | . | . | . | . | . | . | . | . | . | . | . | . | . | . | . | . | . | . | . | . | . | . | . | . |
| 21-225_160B12_LC | . | . | . | . | . | . | . | . | . | . | . | . | . | S | . | . | . | . | . | . | . | . | . | . | . | . | . |
| 21-225_161E10_LC | . | . | . | . | . | . | . | . | . | . | . | . | . | S | . | . | . | . | . | . | . | . | . | I | . | . | . |
| 21-225_161F12_LC | . | . | . | . | . | . | . | . | . | . | . | . | . | T | . | . | . | . | . | . | . | . | . | . | . | . | . |
| 21-225_161H3_LC | . | . | F | . | . | . | . | . | . | . | . | . | . | N | . | . | . | . | . | . | . | . | . | . | . | . | . |
| 21-225_165H3_LC | . | . | . | . | . | . | . | . | . | . | . | . | . | S | . | . | . | . | . | . | . | . | . | . | . | . | . |

FIGURE 57

| Reference # | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 | 81 |
|---|---|---|---|---|---|---|---|---|---|---|---|---|---|---|---|---|---|---|---|---|---|---|---|---|---|---|---|
| | | | | L_CDR2 | | | | | | | | | | | | | | | | | | | | | | | |
| CONSENSUS | V | I | Y | Q | - | - | - | - | - | - | - | - | D | R | K | R | P | S | G | I | P | E | R | F | S | G | S |
| 21-225_166G11_LC | . | . | . | . | . | . | . | . | . | . | . | . | . | S | . | . | . | . | . | . | . | . | . | . | . | . | . |
| 21-225_169A6_LC | . | . | . | . | . | . | . | . | . | . | . | . | . | Y | . | . | . | . | . | . | . | . | . | . | . | . | . |
| 21-225_169B7_LC | . | . | . | . | . | . | . | . | . | . | . | . | . | . | . | . | . | . | . | . | . | . | . | . | . | . | . |
| 21-225_169C1_LC | . | . | . | K | . | . | . | . | . | . | . | . | . | I | . | . | . | . | . | . | . | . | . | . | . | . | . |
| 21-225_169G11_LC | . | . | . | . | . | . | . | . | . | . | . | . | . | S | . | . | . | . | . | . | . | . | . | . | . | . | . |
| 21-225_16D2_LC | . | . | . | . | . | . | . | . | . | . | . | . | . | S | . | . | . | . | . | . | . | . | . | . | . | . | . |
| 21-225_16E4_LC | . | . | . | . | . | . | . | . | . | . | . | . | . | S | . | . | . | . | . | . | . | . | . | . | . | . | . |
| 21-225_16G8_LC | . | . | . | . | . | . | . | . | . | . | . | . | . | . | . | . | . | . | . | . | . | . | . | . | . | . | . |
| 21-225_170A5_LC | . | . | . | . | . | . | . | . | . | . | . | . | . | Y | . | . | . | . | . | . | . | . | . | . | . | . | . |
| 21-225_170F1_LC | . | . | . | . | . | . | . | . | . | . | . | . | . | S | . | . | . | . | . | . | . | A | . | . | . | . | . |
| 21-225_171E12_LC | . | . | . | . | . | . | . | . | . | . | . | . | . | . | . | . | . | . | . | . | . | . | . | . | . | . | . |
| 21-225_171F5_LC | . | . | . | . | . | . | . | . | . | . | . | . | . | . | . | . | . | . | . | . | . | . | . | . | . | . | . |
| 21-225_173F8_LC | . | . | S | . | . | . | . | . | . | . | . | . | . | S | R | . | . | . | . | . | . | . | . | . | . | . | . |
| 21-225_175C6_LC | . | . | . | . | . | . | . | . | . | . | . | . | . | Y | . | . | . | . | . | . | . | . | . | . | . | . | . |
| 21-225_179C7_LC | . | . | . | . | . | . | . | . | . | . | . | . | . | S | . | . | . | . | . | . | . | . | . | . | . | . | . |
| 21-225_17D3_LC | . | . | . | . | . | . | . | . | . | . | . | . | . | . | . | . | . | . | . | . | . | . | . | . | . | . | . |
| 21-225_17F5_LC | . | . | . | . | . | . | . | . | . | . | . | . | . | . | . | . | . | . | . | . | . | . | . | . | . | . | . |
| 21-225_180C5_LC | . | . | . | . | . | . | . | . | . | . | . | . | . | . | . | . | . | . | . | . | . | . | . | . | . | . | . |
| 21-225_180D4_LC | . | . | . | E | . | . | . | . | . | . | . | . | . | . | . | . | . | . | . | . | . | . | . | . | . | . | . |
| 21-225_180G5_LC | . | . | . | . | . | . | . | . | . | . | . | . | . | S | . | . | . | . | . | . | . | . | . | . | . | . | . |
| 21-225_181H1_LC | . | . | . | E | . | . | . | . | . | . | . | . | . | . | . | . | . | . | . | . | . | . | . | . | . | . | . |
| 21-225_182D12_LC | . | . | . | . | . | . | . | . | . | . | . | . | . | . | . | . | . | . | . | . | . | . | . | . | . | . | . |
| 21-225_183F4_LC | . | . | . | . | . | . | . | . | . | . | . | . | . | K | . | . | . | . | . | . | . | . | . | . | . | . | . |
| 21-225_185D2_LC | . | . | . | E | . | . | . | . | . | . | . | . | . | . | . | . | . | . | . | . | . | D | . | . | . | . | . |
| 21-225_185G7_LC | . | . | . | . | . | . | . | . | . | . | . | . | . | . | . | . | . | . | . | . | . | . | . | . | . | . | . |
| 21-225_186H6_LC | . | . | . | . | . | . | . | . | . | . | . | . | . | . | . | . | . | . | . | . | . | . | . | . | . | . | . |
| 21-225_18E10_LC | . | . | . | . | . | . | . | . | . | . | . | . | . | . | . | . | . | . | . | . | . | . | . | . | . | . | . |
| 21-225_18G5_LC | . | . | . | . | . | . | . | . | . | . | . | . | . | S | . | . | . | . | . | . | . | . | . | . | . | . | . |
| 21-225_190G9_LC | . | . | . | . | . | . | . | . | . | . | . | . | . | N | . | . | . | . | . | . | . | . | . | . | . | . | . |

FIGURE 57

| Reference # | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 | 81 |
|---|---|---|---|---|---|---|---|---|---|---|---|---|---|---|---|---|---|---|---|---|---|---|---|---|---|---|---|
| | | | | L_CDR2 | | | | | | | | | | | | | | | | | | | | | | | |
| CONSENSUS | V | I | Y | Q | - | - | - | - | - | - | - | - | D | R | K | R | P | S | G | I | P | E | R | F | S | G | S |
| 21-225_190H1_LC | . | . | . | . | . | . | . | . | . | . | . | . | . | S | . | . | . | . | . | . | . | . | . | . | . | . | . |
| 21-225_193H5_LC | . | . | . | . | . | . | . | . | . | . | . | . | . | S | . | . | . | . | . | . | . | . | . | . | . | . | . |
| 21-225_195E3_LC | . | . | . | E | . | . | . | . | . | . | . | . | . | S | . | . | . | . | . | . | . | . | . | . | . | . | . |
| 21-225_195E9_LC | . | . | . | . | . | . | . | . | . | . | . | . | . | . | . | . | . | . | . | . | . | . | . | . | . | . | . |
| 21-225_19F6_LC | . | . | . | . | . | . | . | . | . | . | . | . | . | S | . | . | . | . | . | T | . | . | . | . | . | . | . |
| 21-225_201G11_LC | . | . | . | . | . | . | . | . | . | . | . | . | . | S | . | . | . | . | . | . | . | . | . | . | . | . | . |
| 21-225_203G6_LC | . | . | . | . | . | . | . | . | . | . | . | . | . | N | . | . | . | . | . | . | . | . | . | . | . | . | . |
| 21-225_215B3_LC | . | . | . | . | . | . | . | . | . | . | . | . | . | S | . | . | . | . | . | . | . | . | . | . | . | . | . |
| 21-225_21D12_LC | . | . | . | . | . | . | . | . | . | . | . | . | . | . | . | . | . | . | . | . | . | . | . | . | . | . | . |
| 21-225_224H2_LC | . | V | . | . | . | . | . | . | . | . | . | . | . | S | . | . | . | . | . | . | . | . | . | . | . | . | . |
| 21-225_225E9_LC | . | M | . | . | . | . | . | . | . | . | . | . | . | . | . | . | . | . | . | . | . | . | . | . | . | . | . |
| 21-225_226A10_LC | . | . | . | . | . | . | . | . | . | . | . | . | . | . | . | . | . | . | . | . | . | . | . | . | . | . | . |
| 21-225_226B2_LC | . | . | . | . | . | . | . | . | . | . | . | . | . | . | . | . | . | . | . | . | . | . | . | . | . | . | . |
| 21-225_227E12_LC | . | . | . | . | . | . | . | . | . | . | . | . | . | S | . | . | . | . | . | . | . | . | . | . | . | . | . |
| 21-225_227E5_LC | . | . | . | . | . | . | . | . | . | . | . | . | . | . | . | . | . | . | . | . | . | . | . | . | . | . | . |
| 21-225_22E6_LC | . | . | . | . | . | . | . | . | . | . | . | . | . | . | . | . | . | . | . | . | . | . | . | Y | . | . | . |
| 21-225_23D7_LC | . | . | . | . | . | . | . | . | . | . | . | . | . | S | . | . | . | . | . | . | . | . | . | . | . | . | . |
| 21-225_26C10_LC | . | . | . | . | . | . | . | . | . | . | . | . | . | S | . | . | . | . | . | . | . | . | . | . | . | . | . |
| 21-225_27D9_LC | . | . | . | . | . | . | . | . | . | . | . | . | . | S | . | . | . | . | . | . | . | . | . | . | . | . | . |
| 21-225_27E7_LC | . | . | . | . | . | . | . | . | . | . | . | . | . | S | . | . | . | L | . | . | . | . | . | . | . | . | . |
| 21-225_27G6_LC | . | . | . | . | . | . | . | . | . | . | . | . | . | . | . | . | . | . | . | . | . | . | . | . | . | . | . |
| 21-225_27H2_LC | . | . | . | . | . | . | . | . | . | . | . | . | . | G | . | . | . | . | . | . | . | . | . | . | . | . | . |
| 21-225_28A11_LC | . | . | . | . | . | . | . | . | . | . | . | . | . | S | . | . | . | . | . | . | . | . | . | . | . | . | . |
| 21-225_2B1_LC | . | . | . | . | . | . | . | . | . | . | . | . | . | . | . | . | . | . | . | . | . | . | . | . | . | . | . |
| 21-225_30D8_LC2_LC | . | . | . | . | . | . | . | . | . | . | . | . | . | . | . | . | . | . | . | . | . | . | . | . | . | . | . |
| 21-225_30H6_LC | . | . | . | . | . | . | . | . | . | . | . | . | . | S | . | . | . | . | . | . | . | . | . | . | . | . | . |
| 21-225_31C2_LC | . | . | . | . | . | . | . | . | . | . | . | . | . | S | . | . | . | . | . | . | . | . | . | . | . | . | . |
| 21-225_31D12_LC2_LC | . | . | . | . | . | . | . | . | . | . | . | . | . | . | . | . | . | . | . | . | . | . | . | . | . | . | . |
| 21-225_32B11_LC | . | . | . | . | . | . | . | . | . | . | . | . | . | S | . | . | S | . | . | . | . | . | . | . | . | . | . |

FIGURE 57

| Reference # | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 | 81 |
|---|---|---|---|---|---|---|---|---|---|---|---|---|---|---|---|---|---|---|---|---|---|---|---|---|---|---|---|
| | | | | L_CDR2 | | | | | | | | | | | | | | | | | | | | | | | |
| CONSENSUS | V | I | Y | Q | - | - | - | - | - | - | - | - | D | R | K | R | P | S | G | I | P | E | R | F | S | G | S |
| 21-225_33A1_LC | . | . | . | . | . | . | . | . | . | . | . | . | . | S | . | . | . | . | . | . | . | . | . | . | . | . | . |
| 21-225_33E6_LC | . | . | . | . | . | . | . | . | . | . | . | . | . | . | . | . | . | . | . | . | . | . | . | . | . | . | . |
| 21-225_34B11_LC | . | . | . | . | . | . | . | . | . | . | . | . | . | M | . | . | . | . | . | . | . | . | . | . | . | . | . |
| 21-225_34B9_LC | . | . | . | . | . | . | . | . | . | . | . | . | . | S | . | . | . | . | . | . | . | . | . | . | . | . | . |
| 21-225_34D7_LC | . | L | . | . | . | . | . | . | . | . | . | . | . | S | . | . | . | . | . | . | . | . | . | . | . | . | . |
| 21-225_35E1_LC | . | . | . | . | . | . | . | . | . | . | . | . | . | . | . | . | . | . | . | . | . | . | . | . | . | . | . |
| 21-225_3B12_LC | . | . | . | . | . | . | . | . | . | . | . | . | . | . | . | . | . | . | . | . | . | . | . | . | . | . | . |
| 21-225_3H10_LC | . | . | . | . | . | . | . | . | . | . | . | . | . | . | . | . | . | . | . | . | . | . | . | . | . | . | . |
| 21-225_45F6_LC | . | . | . | . | . | . | . | . | . | . | . | . | . | S | . | . | . | . | . | . | . | . | . | . | . | . | . |
| 21-225_4B3_LC | . | . | . | . | . | . | . | . | . | . | . | . | . | . | . | . | . | . | . | . | . | . | . | . | . | . | . |
| 21-225_50G9_LC | . | . | . | . | . | . | . | . | . | . | . | . | . | S | . | . | . | . | . | . | . | . | . | . | . | . | . |
| 21-225_52H1_LC | . | . | . | . | . | . | . | . | . | . | . | . | . | . | . | . | . | . | . | . | . | . | . | . | . | . | . |
| 21-225_53D10_LC | . | . | . | . | . | . | . | . | . | . | . | . | . | . | . | . | . | . | . | . | . | . | . | . | . | . | . |
| 21-225_57D9_LC | . | . | . | . | . | . | . | . | . | . | . | . | . | S | . | . | . | . | . | . | . | . | . | . | . | . | . |
| 21-225_57F1_LC | . | . | . | . | . | . | . | . | . | . | . | . | . | . | . | . | . | . | . | . | . | . | . | . | . | . | . |
| 21-225_57H11_LC | . | . | . | . | . | . | . | . | . | . | . | . | . | . | . | . | . | . | . | . | . | . | . | . | . | . | . |
| 21-225_58C1_LC | . | . | . | . | . | . | . | . | . | . | . | . | . | . | . | . | . | . | . | . | . | . | . | . | . | . | . |
| 21-225_58E7_LC | . | . | . | . | . | . | . | . | . | . | . | . | . | N | . | . | . | . | . | . | . | . | . | . | . | . | . |
| 21-225_58F7_LC | . | . | . | . | . | . | . | . | . | . | . | . | . | . | . | . | . | . | . | . | . | . | . | . | . | . | . |
| 21-225_58F8_LC | . | . | . | . | . | . | . | . | . | . | . | . | . | S | . | . | . | . | . | . | . | . | . | . | . | . | . |
| 21-225_58G11_LC | . | . | . | . | . | . | . | . | . | . | . | . | . | N | . | . | . | . | . | . | . | . | . | . | . | . | . |
| 21-225_59B11_LC | . | . | . | . | . | . | . | . | . | . | . | . | . | S | . | . | . | . | . | . | L | . | . | . | . | . | . |
| 21-225_59F2_LC | . | . | . | . | . | . | . | . | . | . | . | . | . | N | . | . | . | . | . | . | . | . | . | . | . | . | . |
| 21-225_5G7_LC | . | . | . | . | . | . | . | . | . | . | . | . | . | . | . | . | . | . | . | . | . | . | . | . | . | . | . |
| 21-225_60A12_LC | . | . | . | . | . | . | . | . | . | . | . | . | . | . | . | . | . | . | . | . | . | . | . | . | . | . | . |
| 21-225_60B1_LC | . | . | . | . | . | . | . | . | . | . | . | . | . | . | . | . | . | . | . | . | . | . | . | . | . | . | . |
| 21-225_60D2_LC | . | . | . | . | . | . | . | . | . | . | . | . | . | N | . | . | . | . | . | . | . | . | . | . | . | . | . |
| 21-225_61F5_LC | . | . | . | . | . | . | . | . | . | . | . | . | . | S | . | . | . | . | . | . | . | . | . | . | . | . | . |
| 21-225_62A12_LC | . | . | . | . | . | . | . | . | . | . | . | . | . | . | . | . | . | . | . | . | . | . | . | . | . | . | . |

FIGURE 57

| Reference # | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 | 81 |
|---|---|---|---|---|---|---|---|---|---|---|---|---|---|---|---|---|---|---|---|---|---|---|---|---|---|---|---|
| | | | | | | | | | | L_CDR2 | | | | | | | | | | | | | | | | | |
| CONSENSUS | V | I | Y | Q | - | - | - | - | - | - | - | - | D | R | K | R | P | S | G | I | P | E | R | F | S | G | S |
| 21-225_62B12_LC | . | . | . | . | . | . | . | . | . | . | . | . | . | . | . | . | . | . | . | . | . | . | . | . | . | . | . |
| 21-225_62D10_LC | . | . | . | . | . | . | . | . | . | . | . | . | . | . | . | . | . | . | . | . | . | . | . | . | . | . | . |
| 21-225_62E3_LC | . | . | . | . | . | . | . | . | . | . | . | . | . | . | . | . | . | . | . | . | . | . | . | . | . | . | . |
| 21-225_62E8_LC | . | . | . | . | . | . | . | . | . | . | . | . | . | . | . | . | . | . | . | . | . | . | . | . | . | . | . |
| 21-225_65E9_LC | . | . | . | . | . | . | . | . | . | . | . | . | . | . | . | . | . | . | . | . | . | . | . | . | . | . | . |
| 21-225_66G9_LC | . | . | . | . | . | . | . | . | . | . | . | . | . | . | . | . | . | . | . | . | . | . | . | . | . | . | . |
| 21-225_67F10_LC | . | . | F | E | . | . | . | . | . | . | . | . | . | . | . | . | . | . | . | . | . | . | . | . | . | . | . |
| 21-225_68D8_LC | . | . | . | . | . | . | . | . | . | . | . | . | . | S | . | . | . | . | . | . | . | . | . | . | . | . | . |
| 21-225_68G6_LC | . | . | . | . | . | . | . | . | . | . | . | . | . | . | . | . | . | . | . | . | . | D | . | . | . | . | . |
| 21-225_69B11_LC | . | . | . | E | . | . | . | . | . | . | . | . | . | . | . | . | . | . | . | . | . | . | . | . | . | . | . |
| 21-225_69B9_LC | . | . | . | . | . | . | . | . | . | . | . | . | . | . | . | . | . | . | . | . | . | . | . | . | . | . | . |
| 21-225_6B4_LC | . | . | . | . | . | . | . | . | . | . | . | . | . | . | . | . | . | . | . | . | . | . | . | . | . | . | . |
| 21-225_6D9_LC | . | . | . | . | . | . | . | . | . | . | . | . | . | K | . | . | . | . | . | . | . | . | . | . | . | . | . |
| 21-225_71D4_LC | . | . | . | . | . | . | . | . | . | . | . | . | . | . | . | . | . | . | . | . | . | . | . | . | . | . | . |
| 21-225_72B4_LC | . | . | . | E | . | . | . | . | . | . | . | . | . | . | . | . | . | . | . | . | . | . | . | . | . | . | . |
| 21-225_72D5_LC | . | . | . | . | . | . | . | . | . | . | . | . | . | . | . | . | . | . | . | . | . | . | . | . | . | . | . |
| 21-225_73C4_LC | . | . | . | . | . | . | . | . | . | . | . | . | . | M | . | . | . | . | . | . | . | . | . | . | . | . | . |
| 21-225_74A9_LC | . | . | . | . | . | . | . | . | . | . | . | . | . | N | R | . | . | . | . | . | . | . | . | . | . | . | . |
| 21-225_74B3_LC | . | . | . | . | . | . | . | . | . | . | . | . | . | S | . | . | . | . | . | . | . | . | . | . | . | . | . |
| 21-225_74C9_LC | . | . | . | . | . | . | . | . | . | . | . | . | . | N | R | . | . | . | . | . | . | . | . | . | . | . | . |
| 21-225_76B4_LC | . | . | . | . | . | . | . | . | . | . | . | . | . | S | . | . | . | . | . | . | . | . | . | . | . | . | . |
| 21-225_77A2_LC | . | . | . | . | . | . | . | . | . | . | . | . | . | . | . | . | . | . | . | . | . | . | . | . | . | . | . |
| 21-225_78E9_LC | . | . | . | . | . | . | . | . | . | . | . | . | . | N | R | . | . | . | . | . | . | . | . | . | . | . | . |
| 21-225_79E7_LC | . | . | . | . | . | . | . | . | . | . | . | . | . | N | R | . | . | . | . | . | . | . | . | . | . | . | . |
| 21-225_8D8_LC | . | . | . | . | . | . | . | . | . | . | . | . | . | . | . | . | . | . | . | . | . | . | . | . | . | . | . |
| 21-225_92A4_LC | . | . | . | . | . | . | . | . | . | . | . | . | . | N | R | . | . | . | . | . | . | . | . | . | . | . | . |
| 21-225_96B5_LC | . | . | . | . | . | . | . | . | . | . | . | . | . | S | . | . | . | . | . | . | . | . | . | . | . | . | . |
| 21-225_97E6_LC | . | . | . | . | . | . | . | . | . | . | . | . | . | N | R | . | . | . | . | . | . | . | . | . | . | . | . |
| 21-225_9H10_LC | . | . | . | . | . | . | . | . | . | . | . | . | . | S | . | . | . | . | . | . | . | . | . | . | . | . | . |

FIGURE 57

| Reference # | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 |
|---|---|---|---|---|---|---|---|---|---|---|---|---|---|---|---|---|---|---|---|---|---|---|---|---|---|---|---|
| | L_FR3 | | | | | | | | | | | | | | | | | | | | | | | | | | |
| CONSENSUS | N | S | G | - | - | N | T | A | T | L | T | I | S | G | T | Q | A | M | D | E | A | D | Y | Y | C | Q | A |
| 21-225_10B10_LC | . | . | . | . | . | . | . | . | . | . | . | . | . | . | . | . | . | . | . | . | . | . | . | . | . | . | . |
| 21-225_12B1_LC | . | . | . | . | . | . | . | . | . | . | . | . | . | . | . | . | . | . | . | . | . | . | . | . | . | . | . |
| 21-225_13F6_LC | . | . | . | . | . | . | . | . | . | . | . | . | . | . | . | . | . | . | . | . | . | . | . | . | . | . | . |
| 21-225_146A10_LC | . | . | . | . | . | . | . | . | . | . | . | . | . | . | . | . | . | . | . | . | . | . | . | . | . | . | . |
| 21-225_146A2_LC | . | . | . | . | . | . | . | . | . | . | . | . | . | . | I | . | . | . | . | . | . | . | . | . | . | . | . |
| 21-225_146A3_LC | . | . | . | . | . | . | I | . | . | . | . | . | . | . | . | . | . | . | . | . | . | . | . | . | . | . | . |
| 21-225_146B11_LC | . | . | . | . | . | . | . | . | . | . | . | . | . | . | I | . | . | . | . | . | . | . | . | . | . | . | . |
| 21-225_146B8_LC | . | . | . | . | . | . | . | . | . | . | . | . | . | . | . | . | . | . | . | . | . | . | . | . | . | . | . |
| 21-225_146C11_LC | . | . | . | . | . | . | . | . | . | . | . | . | . | . | I | . | . | . | . | . | . | . | . | . | . | . | . |
| 21-225_146H8_LC | . | . | . | . | . | . | . | . | . | . | . | . | . | . | . | . | V | . | . | . | . | . | . | . | . | . | . |
| 21-225_147B12_LC | . | . | . | . | . | . | A | . | . | . | . | . | . | . | I | . | . | . | . | . | . | . | . | . | . | . | . |
| 21-225_147B8_LC | . | . | . | . | . | . | . | . | . | . | . | . | . | . | . | . | . | . | . | . | . | . | . | . | . | . | . |
| 21-225_147B9_LC | . | . | . | . | . | . | . | . | . | . | . | . | . | . | . | L | . | V | . | . | . | . | . | . | . | L | . |
| 21-225_147D9_LC | . | . | . | . | . | . | . | . | . | . | . | . | . | . | . | . | . | . | . | . | . | . | . | . | . | . | . |
| 21-225_147E11_LC | . | . | . | . | . | . | . | . | . | . | . | . | . | . | I | . | . | . | . | . | . | . | . | . | . | . | . |
| 21-225_147E7_LC | . | . | . | . | . | . | . | . | . | . | . | . | . | . | I | . | . | . | . | . | . | . | . | . | . | . | . |
| 21-225_147F9_LC | . | . | . | . | . | . | . | . | . | . | . | . | . | . | . | . | . | . | . | . | . | . | . | . | . | . | . |
| 21-225_147G9_LC | . | . | . | . | . | . | . | . | . | . | . | . | . | . | . | . | . | . | . | . | . | . | . | . | . | . | . |
| 21-225_148A9_LC | . | . | . | . | . | . | . | . | . | . | . | . | . | . | . | . | . | . | . | . | . | . | . | . | . | . | . |
| 21-225_148C8_LC | . | . | . | . | . | . | . | . | . | . | . | . | . | . | . | . | . | . | . | . | . | . | . | . | . | L | . |
| 21-225_148E10_LC | . | . | . | . | . | . | . | . | . | . | . | . | . | . | . | . | . | . | . | . | . | . | . | F | . | . | . |
| 21-225_148G11_LC | . | . | . | . | . | . | . | . | . | . | . | . | . | . | I | . | . | . | . | . | . | . | . | . | . | . | . |
| 21-225_148G6_LC | . | . | . | . | . | . | . | . | . | . | . | . | . | . | I | . | . | . | . | . | . | . | . | . | . | . | . |
| 21-225_149A11_LC | . | . | . | . | . | . | . | . | . | . | . | . | . | . | . | . | . | . | . | . | . | . | . | . | . | L | . |
| 21-225_149B5_LC | . | . | . | . | . | . | . | . | S | . | . | . | . | . | . | . | . | . | . | . | . | . | . | . | . | . | . |
| 21-225_149C10_LC | . | . | . | . | . | . | . | . | . | . | . | . | G | . | I | . | . | . | . | . | . | . | . | . | . | . | . |
| 21-225_149C7_LC | K | . | . | . | . | . | . | . | . | . | . | . | . | . | I | . | . | . | . | . | . | . | . | . | . | . | . |
| 21-225_14B1_LC2_LC | . | . | . | . | . | . | . | . | . | . | . | . | . | . | . | . | . | . | . | . | . | . | . | . | . | . | . |
| 21-225_14B7_LC | . | . | . | . | . | . | . | . | . | . | . | . | . | . | . | . | T | . | . | . | . | . | . | . | . | . | . |

FIGURE 57

| Reference # | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 |
|---|---|---|---|---|---|---|---|---|---|---|---|---|---|---|---|---|---|---|---|---|---|---|---|---|---|---|---|
| | L_FR3 | | | | | | | | | | | | | | | | | | | | | | | | | | |
| CONSENSUS | N | S | G | - | - | N | T | A | T | L | T | I | S | G | T | Q | A | M | D | E | A | D | Y | Y | C | Q | A |
| 21-225_14G3_LC | . | . | . | . | . | . | . | . | . | . | . | . | . | . | . | . | . | . | . | . | . | . | . | . | . | . | . |
| 21-225_14H11_LC | . | . | . | . | . | . | . | . | . | . | . | . | . | . | . | . | . | . | . | . | . | . | . | . | . | . | . |
| 21-225_150D3_LC | S | . | . | . | . | . | . | . | . | . | . | . | . | . | . | . | . | . | . | . | . | . | . | . | . | . | . |
| 21-225_150F6_LC | . | . | . | . | . | . | . | . | . | . | . | . | . | . | I | . | . | . | . | . | . | . | . | C | . | . | . |
| 21-225_150H11_LC | . | . | . | . | . | . | . | . | . | . | . | . | . | . | I | . | . | . | . | . | . | . | F | . | . | . | . |
| 21-225_151B9_LC | . | . | . | . | . | . | . | . | . | . | . | . | . | . | I | . | . | . | . | . | . | . | . | . | . | . | . |
| 21-225_151F3_LC | . | . | . | . | . | . | . | . | . | . | . | . | . | . | . | . | . | . | . | . | . | . | . | . | . | . | . |
| 21-225_151H5_LC | . | . | . | . | . | . | . | . | . | . | . | . | . | . | I | . | . | . | . | . | . | . | . | . | . | . | . |
| 21-225_151H7_LC | . | . | . | . | . | . | . | . | . | . | . | . | . | . | I | . | . | . | . | . | . | . | . | . | . | . | . |
| 21-225_152D7_LC | . | . | . | . | . | . | . | . | . | . | . | . | . | . | I | . | . | . | . | . | . | . | . | . | . | . | . |
| 21-225_152G6_LC | . | . | . | . | . | . | . | . | . | . | . | . | . | . | I | . | . | L | . | . | . | . | . | . | . | . | . |
| 21-225_153D9_LC | . | . | . | . | . | . | . | . | . | . | . | . | . | . | . | . | . | . | . | . | . | . | . | . | . | . | . |
| 21-225_153E6_LC | . | . | . | . | . | . | . | . | . | . | . | . | . | . | . | . | . | . | . | . | . | G | . | . | . | . | . |
| 21-225_154C3_LC | . | . | . | . | . | . | . | . | . | . | . | . | T | . | . | . | . | . | . | . | . | . | . | . | . | . | . |
| 21-225_154C4_LC | . | . | . | . | . | . | . | . | . | . | . | . | . | . | I | . | . | . | . | . | . | . | . | . | . | . | . |
| 21-225_154D11_LC | . | . | . | . | . | . | I | . | . | . | . | . | . | . | . | . | . | . | . | . | . | . | . | . | . | . | . |
| 21-225_154E10_LC | . | . | . | . | . | . | . | . | . | . | . | . | . | . | I | . | . | . | . | . | . | . | . | . | . | . | . |
| 21-225_154F4_LC | . | . | . | . | . | . | . | G | . | . | . | . | . | . | . | . | . | . | . | . | . | . | . | . | . | . | . |
| 21-225_155C10_LC | . | . | . | . | . | . | . | . | . | . | . | . | . | . | I | . | . | . | . | . | . | . | . | . | . | . | . |
| 21-225_155E10_LC | . | . | . | . | . | . | . | . | . | . | . | . | . | . | . | . | . | . | . | . | . | . | . | . | . | L | . |
| 21-225_158D10_LC | . | . | . | . | . | . | . | . | . | . | . | . | . | . | . | . | . | L | . | . | . | . | . | . | . | . | . |
| 21-225_158E9_LC | . | . | . | . | . | . | . | . | . | . | . | . | T | . | . | . | . | . | . | . | . | N | . | . | . | . | . |
| 21-225_159H8_LC | . | . | . | . | . | . | . | . | . | . | . | . | . | . | . | . | . | L | . | . | . | . | . | . | . | . | . |
| 21-225_15H11_LC | . | . | . | . | . | . | . | . | . | . | . | . | . | . | . | . | . | I | . | . | . | . | . | . | . | . | . |
| 21-225_160B12_LC | . | . | . | . | . | . | . | . | . | . | . | . | . | . | . | . | . | L | . | . | . | . | . | . | . | . | . |
| 21-225_161E10_LC | . | . | . | . | . | . | . | . | . | . | . | . | . | . | . | . | . | . | . | . | . | . | . | . | . | . | T |
| 21-225_161F12_LC | . | . | . | . | . | . | . | . | . | . | . | . | . | . | . | . | . | . | . | . | . | . | . | . | . | . | . |
| 21-225_161H3_LC | . | . | . | . | . | . | . | . | . | . | . | . | . | . | . | . | . | L | . | . | . | . | . | . | . | . | . |
| 21-225_165H3_LC | . | . | . | . | . | . | . | . | . | . | . | . | . | . | . | . | . | . | . | . | . | . | . | . | . | . | . |

FIGURE 57

| Reference # | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 |
|---|---|---|---|---|---|---|---|---|---|---|---|---|---|---|---|---|---|---|---|---|---|---|---|---|---|---|---|
| | L_FR3 | | | | | | | | | | | | | | | | | | | | | | | | | | |
| CONSENSUS | N | S | G | - | - | N | T | A | T | L | T | I | S | G | T | Q | A | M | D | E | A | D | Y | Y | C | Q | A |
| 21-225_166G11_LC | . | . | . | . | . | . | . | . | . | . | . | . | . | . | . | . | . | . | . | . | . | . | . | . | . | . | . |
| 21-225_169A6_LC | . | . | . | . | . | . | . | . | . | . | . | . | . | . | . | . | . | . | . | . | . | . | . | . | . | . | . |
| 21-225_169B7_LC | . | . | . | . | . | . | . | . | . | . | . | . | . | . | . | . | . | . | . | . | . | . | . | . | . | . | . |
| 21-225_169C1_LC | . | . | . | . | . | . | . | . | . | . | . | . | . | . | . | . | . | . | . | . | . | . | . | . | . | . | . |
| 21-225_169G11_LC | . | . | . | . | . | . | . | . | . | . | . | . | . | . | . | . | . | . | . | . | . | . | . | . | . | . | . |
| 21-225_16D2_LC | . | . | . | . | . | . | . | . | . | . | . | . | . | . | . | . | . | . | . | . | . | . | . | . | . | . | . |
| 21-225_16E4_LC | . | . | . | . | . | . | . | . | A | . | . | . | . | . | . | . | . | . | . | . | . | . | . | . | . | . | . |
| 21-225_16G8_LC | . | . | . | . | . | . | K | . | . | . | . | . | . | . | . | . | . | . | . | . | . | . | . | . | . | . | . |
| 21-225_170A5_LC | . | . | . | . | . | . | . | . | . | . | . | . | . | . | . | . | . | . | . | . | . | . | . | . | . | . | . |
| 21-225_170F1_LC | . | . | . | . | . | . | . | . | . | . | . | . | . | . | . | . | . | . | . | . | . | . | . | . | . | . | . |
| 21-225_171E12_LC | . | . | . | . | . | . | . | . | . | . | . | . | . | . | . | . | . | . | . | . | . | . | . | . | . | . | . |
| 21-225_171F5_LC | . | . | . | . | . | . | . | . | . | . | . | . | . | . | . | . | . | . | . | . | . | . | . | . | . | . | . |
| 21-225_173F8_LC | . | . | . | . | . | . | . | . | . | . | . | . | . | . | . | . | . | . | . | . | . | . | . | . | . | . | . |
| 21-225_175C6_LC | . | . | . | . | . | . | . | . | . | . | . | . | . | . | . | . | . | . | . | . | . | . | . | . | . | . | . |
| 21-225_179C7_LC | . | . | . | . | . | . | . | . | . | . | . | . | R | . | . | . | . | . | . | . | . | . | . | . | . | . | . |
| 21-225_17D3_LC | . | . | . | . | . | . | . | . | . | . | . | . | . | . | . | . | . | . | . | . | . | . | . | . | . | . | . |
| 21-225_17F5_LC | . | . | . | . | . | . | . | . | . | . | . | . | . | . | . | . | . | . | . | . | . | . | . | . | . | . | . |
| 21-225_180C5_LC | . | . | . | . | . | . | . | . | . | . | . | . | . | . | . | . | . | . | . | . | . | . | . | . | . | . | . |
| 21-225_180D4_LC | . | . | . | . | . | . | . | . | . | . | . | . | . | . | . | . | . | . | . | . | G | . | . | . | . | . | . |
| 21-225_180G5_LC | . | . | . | . | . | . | P | . | S | . | . | . | . | . | . | . | . | . | . | . | . | . | . | . | . | . | . |
| 21-225_181H1_LC | . | . | . | . | . | . | . | . | . | . | . | . | . | . | . | . | . | . | . | . | . | . | . | . | . | . | . |
| 21-225_182D12_LC | . | . | . | . | . | . | . | . | . | . | . | . | . | . | . | . | . | . | . | . | . | . | . | . | . | . | . |
| 21-225_183F4_LC | . | . | . | . | . | . | . | . | . | . | . | . | . | . | . | . | . | . | . | . | . | . | . | . | . | . | . |
| 21-225_185D2_LC | . | . | . | . | . | . | . | . | . | . | . | . | . | . | . | . | . | . | . | . | . | . | . | . | . | . | . |
| 21-225_185G7_LC | . | . | . | . | . | . | . | . | . | . | . | . | . | . | . | . | . | . | . | . | . | . | . | . | . | . | . |
| 21-225_186H6_LC | . | . | . | . | . | . | . | . | . | . | . | . | . | . | . | . | . | . | . | . | . | . | . | . | . | . | . |
| 21-225_18E10_LC | . | . | . | . | . | . | . | . | . | . | . | . | . | . | . | . | . | . | . | . | . | . | . | . | . | . | . |
| 21-225_18G5_LC | . | . | . | . | . | . | . | . | . | . | . | . | . | . | . | . | . | . | . | . | . | . | . | . | . | . | . |
| 21-225_190G9_LC | . | . | . | . | . | . | . | . | S | . | . | . | . | . | . | . | . | . | . | . | . | . | . | . | . | . | . |

FIGURE 57

| Reference # | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 |
|---|---|---|---|---|---|---|---|---|---|---|---|---|---|---|---|---|---|---|---|---|---|---|---|---|---|---|---|
| | L_FR3 | | | | | | | | | | | | | | | | | | | | | | | | | | |
| CONSENSUS | N | S | G | - | - | N | T | A | T | L | T | I | S | G | T | Q | A | M | D | E | A | D | Y | Y | C | Q | A |
| 21-225_190H1_LC | . | . | . | . | . | . | . | . | . | . | . | . | . | . | . | . | . | . | . | . | . | . | . | . | . | K | . |
| 21-225_193H5_LC | . | . | . | . | . | . | . | . | . | . | . | . | . | . | . | . | . | . | . | . | . | . | . | . | . | . | . |
| 21-225_195E3_LC | . | . | . | . | . | . | . | . | . | . | . | . | . | . | . | . | . | . | . | . | . | . | . | . | . | . | . |
| 21-225_195E9_LC | . | . | . | . | . | . | . | G | . | . | . | . | . | . | . | . | G | . | . | . | . | . | . | . | . | . | . |
| 21-225_19F6_LC | . | . | . | . | . | . | . | . | . | . | . | . | . | . | . | . | . | . | . | . | . | . | . | . | . | . | . |
| 21-225_201G11_LC | . | . | . | . | . | . | . | . | . | . | . | . | . | . | . | . | . | . | . | . | . | . | . | . | . | . | . |
| 21-225_203G6_LC | . | . | . | . | . | . | . | . | . | . | . | . | . | . | . | . | S | . | . | . | . | . | . | . | . | . | . |
| 21-225_215B3_LC | . | . | . | . | . | . | . | . | . | . | . | . | . | . | . | . | . | . | . | . | . | . | . | . | . | . | . |
| 21-225_21D12_LC | . | . | . | . | . | . | . | . | . | . | . | . | . | . | . | . | . | . | . | . | . | . | F | . | . | . | . |
| 21-225_224H2_LC | . | . | . | . | . | . | . | . | . | . | . | . | . | . | . | . | . | . | . | . | . | . | . | . | . | . | . |
| 21-225_225E9_LC | . | . | . | . | . | . | . | . | . | . | . | . | . | . | . | . | . | . | . | . | . | . | . | . | . | . | . |
| 21-225_226A10_LC | S | . | . | . | . | . | . | . | . | . | . | . | . | . | . | . | . | . | . | . | . | . | . | . | . | . | . |
| 21-225_226B2_LC | S | . | . | . | . | . | . | . | . | . | . | . | . | . | . | . | . | . | . | . | . | . | . | . | . | . | . |
| 21-225_227E12_LC | . | . | . | . | . | . | . | . | . | . | . | . | . | . | . | . | . | . | . | . | . | . | . | . | . | . | . |
| 21-225_227E5_LC | . | . | . | . | . | . | K | . | . | . | . | . | . | . | . | . | . | . | . | . | . | . | . | . | . | . | . |
| 21-225_22E6_LC | . | T | . | . | . | . | . | . | . | . | . | . | . | . | . | . | . | . | . | . | . | . | . | . | . | . | . |
| 21-225_23D7_LC | . | . | . | . | . | . | . | . | . | . | . | . | . | . | . | . | . | . | . | . | . | . | . | . | . | . | . |
| 21-225_26C10_LC | . | . | . | . | . | . | . | . | . | . | . | . | . | . | . | . | . | . | . | . | . | . | . | . | . | . | . |
| 21-225_27D9_LC | . | . | . | . | . | . | . | . | . | . | . | . | . | . | . | . | . | . | . | . | . | . | . | . | . | . | . |
| 21-225_27E7_LC | . | . | . | . | . | . | . | . | . | . | . | . | . | . | . | . | . | . | . | . | . | . | . | . | . | . | . |
| 21-225_27G6_LC | . | . | . | . | . | . | . | . | . | . | . | . | . | . | . | . | T | . | . | . | . | . | . | . | . | . | . |
| 21-225_27H2_LC | . | . | . | . | . | . | . | . | . | . | . | L | . | . | . | . | . | . | . | . | . | . | . | . | . | . | . |
| 21-225_28A11_LC | . | . | . | . | . | . | . | . | . | . | . | . | . | . | . | . | . | . | . | . | . | . | . | . | . | . | . |
| 21-225_2B1_LC | . | . | . | . | . | . | . | . | . | . | . | . | . | . | . | . | . | . | . | . | . | . | . | . | . | . | . |
| 21-225_30D9_LC2_LC | . | . | . | . | . | . | . | . | . | . | . | . | . | . | . | . | . | L | . | . | . | . | . | . | . | . | . |
| 21-225_30H6_LC | . | . | . | . | . | . | . | . | . | . | . | . | . | . | . | . | . | . | . | . | . | . | . | . | . | . | . |
| 21-225_31C2_LC | . | . | . | . | . | . | . | . | . | . | . | . | . | . | . | . | . | . | . | . | . | . | . | . | . | . | . |
| 21-225_31D12_LC2_LC | . | . | . | . | . | . | . | . | . | . | . | . | . | . | . | . | . | . | . | . | . | . | . | . | . | . | . |
| 21-225_32B11_LC | . | . | . | . | . | . | . | . | . | . | . | . | . | . | . | . | . | . | . | . | . | . | . | . | . | . | . |

# FIGURE 57

| Reference # | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 |
|---|---|---|---|---|---|---|---|---|---|---|---|---|---|---|---|---|---|---|---|---|---|---|---|---|---|---|---|
| | L_FR3 | | | | | | | | | | | | | | | | | | | | | | | | | | |
| CONSENSUS | N | S | G | - | - | N | T | A | T | L | T | I | S | G | T | Q | A | M | D | E | A | D | Y | Y | C | Q | A |
| 21-225_33A1_LC | . | . | . | . | . | . | . | . | . | . | . | . | . | . | . | . | . | . | . | . | . | . | . | . | . | . | . |
| 21-225_33E6_LC | . | . | . | . | . | . | . | . | . | . | . | . | . | . | . | . | . | . | . | . | . | . | . | . | . | . | . |
| 21-225_34B11_LC | . | . | . | . | . | . | . | . | . | . | . | . | . | . | . | . | . | . | . | . | . | . | . | . | . | . | . |
| 21-225_34B9_LC | . | . | . | . | . | . | . | . | . | . | . | . | . | . | . | . | . | . | . | . | . | . | . | . | . | . | . |
| 21-225_34D7_LC | . | . | . | . | . | . | . | . | . | . | . | . | . | . | . | . | T | . | . | . | . | . | F | . | . | . | . |
| 21-225_35E1_LC | . | . | . | . | . | . | . | . | . | . | . | . | . | . | . | . | . | . | . | . | . | . | . | . | . | . | . |
| 21-225_3B12_LC | . | . | . | . | . | . | K | . | . | . | . | . | . | . | . | . | . | . | . | . | . | . | . | . | . | . | . |
| 21-225_3H10_LC | . | . | . | . | . | . | K | . | . | . | . | . | . | . | . | . | . | . | . | . | . | . | . | . | . | . | . |
| 21-225_45F6_LC | . | . | . | . | . | . | . | . | . | . | . | . | . | . | . | . | . | . | . | . | . | . | . | . | . | . | . |
| 21-225_4B3_LC | . | . | . | . | . | . | . | . | . | . | . | . | . | . | . | . | . | . | . | . | . | . | . | . | . | . | . |
| 21-225_50G9_LC | . | . | . | . | . | . | . | . | . | . | . | . | . | . | . | . | . | R | . | . | . | E | . | . | . | . | . |
| 21-225_52H1_LC | . | . | . | . | . | . | . | . | . | . | . | . | . | . | . | . | . | . | . | . | . | . | . | . | . | . | . |
| 21-225_53D10_LC | . | . | . | . | . | . | . | . | . | . | . | . | . | . | . | . | . | . | . | . | . | . | . | . | . | . | . |
| 21-225_57D9_LC | . | . | . | . | . | . | . | . | . | . | . | . | . | . | . | . | . | . | . | . | . | . | . | . | . | . | . |
| 21-225_57F1_LC | . | . | . | . | . | . | . | . | . | . | . | . | . | . | . | . | . | . | . | . | . | . | . | . | . | . | . |
| 21-225_57H11_LC | . | . | . | . | . | . | . | . | . | . | . | . | . | . | . | . | . | . | . | . | . | . | . | . | . | . | . |
| 21-225_58C1_LC | . | . | . | . | . | . | . | . | . | . | . | . | . | . | . | . | . | . | . | . | . | . | . | . | . | . | . |
| 21-225_58E7_LC | . | . | . | . | . | . | . | . | . | . | . | . | . | . | . | . | . | . | . | . | . | . | . | F | . | . | . |
| 21-225_58F7_LC | . | . | . | . | . | . | . | . | . | . | . | . | . | . | . | . | . | . | . | . | . | . | . | . | . | . | . |
| 21-225_58F8_LC | . | . | . | . | . | . | . | . | . | . | . | . | . | . | . | . | . | . | . | . | . | . | . | . | . | . | . |
| 21-225_58G11_LC | . | . | . | . | . | . | . | . | . | . | . | . | . | . | . | . | . | . | . | . | . | . | . | . | . | . | . |
| 21-225_59B11_LC | . | . | . | . | . | . | . | . | . | . | . | . | . | . | . | . | . | . | . | . | . | . | . | . | . | . | . |
| 21-225_59F2_LC | . | . | . | . | . | . | . | . | . | . | . | . | . | . | . | . | . | . | . | . | . | . | . | . | . | . | . |
| 21-225_5G7_LC | . | . | . | . | . | . | . | . | . | . | . | . | . | . | . | . | . | . | . | . | . | . | . | . | . | . | . |
| 21-225_60A12_LC | . | . | . | . | . | . | . | . | I | . | . | . | . | . | . | . | . | . | . | . | . | . | . | . | . | . | . |
| 21-225_60B1_LC | . | . | . | . | . | . | . | . | . | . | . | . | . | . | . | . | . | . | . | . | . | . | . | . | . | . | . |
| 21-225_60D2_LC | . | . | . | . | . | . | . | . | . | . | . | . | . | . | . | . | T | . | . | . | . | . | . | . | . | . | . |
| 21-225_61F5_LC | . | . | . | . | . | . | . | . | . | . | . | . | . | . | . | . | . | . | . | . | . | . | . | . | . | . | . |
| 21-225_62A12_LC | . | . | . | . | . | . | . | . | . | . | . | . | . | . | . | . | . | . | . | . | . | . | . | . | . | . | . |

FIGURE 57

| Reference # | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 |
|---|---|---|---|---|---|---|---|---|---|---|---|---|---|---|---|---|---|---|---|---|---|---|---|---|---|---|---|
| | L_FR3 | | | | | | | | | | | | | | | | | | | | | | | | | | |
| CONSENSUS | N | S | G | - | - | N | T | A | T | L | T | I | S | G | T | Q | A | M | D | E | A | D | Y | Y | C | Q | A |
| 21-225_62B12_LC | . | . | . | . | . | . | . | . | . | . | . | . | . | . | . | . | . | . | . | . | . | . | . | . | . | . | . |
| 21-225_62D10_LC | . | . | . | . | . | . | . | . | . | . | . | . | . | . | . | . | . | . | . | . | . | . | . | . | . | . | . |
| 21-225_62E3_LC | . | . | . | . | . | . | . | . | . | . | . | . | . | . | . | . | . | . | . | . | . | . | . | . | . | . | . |
| 21-225_62E8_LC | . | . | . | . | . | . | . | . | . | . | . | . | . | . | . | . | . | . | . | . | . | . | . | . | . | . | . |
| 21-225_65E9_LC | . | . | . | . | . | . | . | . | . | . | . | . | . | . | . | . | . | . | . | . | . | . | . | . | . | . | . |
| 21-225_66G9_LC | . | . | . | . | . | . | . | . | . | . | . | . | . | . | . | . | . | . | . | . | . | . | . | . | . | . | . |
| 21-225_67F10_LC | . | . | . | . | . | . | . | . | . | . | . | . | . | . | . | . | . | . | . | . | . | . | . | . | . | . | . |
| 21-225_68D8_LC | . | . | . | . | . | . | . | . | . | . | . | . | . | . | . | . | . | . | . | . | . | . | . | . | . | . | . |
| 21-225_68G6_LC | . | . | . | . | . | . | . | . | . | . | . | . | . | . | . | . | . | . | . | A | . | . | F | . | . | . | . |
| 21-225_69B11_LC | . | . | . | . | . | . | . | . | . | . | . | . | . | . | . | . | . | . | . | . | . | . | . | . | . | . | . |
| 21-225_69B9_LC | . | . | . | . | . | . | . | . | . | . | . | . | . | . | . | . | . | . | . | . | . | . | . | . | . | . | . |
| 21-225_6B4_LC | . | . | . | . | . | . | . | . | . | . | . | . | . | . | . | . | . | . | . | . | . | . | . | . | . | . | . |
| 21-225_6D9_LC | . | . | . | . | . | . | . | . | . | . | . | . | . | . | . | . | . | . | . | . | . | . | . | . | . | . | . |
| 21-225_71D4_LC | . | . | . | . | . | . | . | . | . | . | . | . | . | . | . | . | . | . | . | . | . | . | . | . | . | . | . |
| 21-225_72B4_LC | . | . | . | . | . | . | . | . | . | . | . | . | . | . | . | . | . | . | . | . | . | . | . | . | . | . | . |
| 21-225_72D5_LC | . | . | . | . | . | . | . | . | . | . | . | . | . | . | . | . | . | . | . | . | . | . | . | . | . | . | . |
| 21-225_73C4_LC | . | . | . | . | . | . | . | . | . | . | . | . | . | . | . | . | . | . | . | . | . | . | . | . | . | . | . |
| 21-225_74A9_LC | . | . | . | . | . | . | . | . | . | . | . | . | . | . | . | . | . | . | . | . | . | . | . | . | . | . | . |
| 21-225_74B3_LC | . | . | . | . | . | . | . | . | . | . | . | . | . | . | . | . | . | . | . | . | . | . | . | . | . | . | . |
| 21-225_74C9_LC | . | . | . | . | . | S | . | . | . | . | . | . | . | . | . | . | . | . | . | . | . | . | . | . | . | . | . |
| 21-225_76B4_LC | . | . | . | . | . | . | . | . | . | . | . | . | . | . | . | . | . | . | . | . | . | . | . | . | . | . | . |
| 21-225_77A2_LC | . | . | . | . | . | . | . | . | . | . | . | . | . | . | . | . | . | . | . | . | . | . | . | . | . | . | . |
| 21-225_78E9_LC | . | . | . | . | . | S | . | . | . | . | . | . | . | . | . | . | . | . | . | . | . | . | . | . | . | . | . |
| 21-225_79E7_LC | . | . | . | . | . | S | . | . | . | . | . | . | . | . | . | . | . | . | . | . | . | . | . | . | . | . | . |
| 21-225_8D8_LC | . | . | . | . | . | . | . | . | . | . | . | . | . | . | . | . | . | I | . | . | . | . | . | . | . | . | . |
| 21-225_92A4_LC | . | . | . | . | . | . | . | . | . | . | . | . | . | . | . | . | . | . | . | . | . | . | . | . | . | . | . |
| 21-225_96B5_LC | . | . | . | . | . | . | . | . | . | . | . | . | . | . | . | . | . | . | . | . | . | . | . | . | . | . | . |
| 21-225_97E6_LC | . | . | . | . | . | S | . | . | . | . | . | . | . | . | . | . | . | . | . | . | . | . | . | . | . | . | . |
| 21-225_9H10_LC | . | . | . | . | . | . | . | . | . | . | . | . | . | . | . | . | . | . | . | . | . | . | . | . | . | . | . |

FIGURE 57

| Reference # | 109 | 110 | 111 | 112 | 113 | 114 | 115 | 116 | 117 | 118 | 119 | 120 | 121 | 122 | 123 | 124 | 125 | 126 | 127 | 128 | 129 | 130 | 131 | 132 | 133 | 134 | 135 |
|---|---|---|---|---|---|---|---|---|---|---|---|---|---|---|---|---|---|---|---|---|---|---|---|---|---|---|---|
| | | | | | | | | | | | | | | L_CDR3 | | | | | | | | | | | | | |
| CONSENSUS | W | D | S | - | - | - | - | - | - | - | - | - | - | - | - | - | - | - | - | - | - | - | - | - | - | - | S |
| 21-225_10B10_LC | . | . | N | . | . | . | . | . | . | . | . | . | . | . | . | . | . | . | . | . | . | . | . | . | . | . | . |
| 21-225_12B1_LC | . | . | N | . | . | . | . | . | . | . | . | . | . | . | . | . | . | . | . | . | . | . | . | . | . | . | N |
| 21-225_13F6_LC | . | G | N | . | . | . | . | . | . | . | . | . | . | . | . | . | . | . | . | . | . | . | . | . | . | S | T |
| 21-225_146A10_LC | . | . | . | . | . | . | . | . | . | . | . | . | . | . | . | . | . | . | . | . | . | . | . | . | . | . | . |
| 21-225_146A2_LC | . | . | . | . | . | . | . | . | . | . | . | . | . | . | . | . | . | . | . | . | . | . | . | . | . | . | . |
| 21-225_146A3_LC | . | . | . | . | . | . | . | . | . | . | . | . | . | . | . | . | . | . | . | . | . | . | . | . | . | . | . |
| 21-225_146B11_LC | . | . | . | . | . | . | . | . | . | . | . | . | . | . | . | . | . | . | . | . | . | . | . | . | . | . | . |
| 21-225_146B8_LC | . | H | . | . | . | . | . | . | . | . | . | . | . | . | . | . | . | . | . | . | . | . | . | . | . | . | . |
| 21-225_146C11_LC | . | . | . | . | . | . | . | . | . | . | . | . | . | . | . | . | . | . | . | . | . | . | . | . | . | . | . |
| 21-225_146H8_LC | . | . | I | . | . | . | . | . | . | . | . | . | . | . | . | . | . | . | . | . | . | . | . | . | . | . | R |
| 21-225_147B12_LC | . | . | . | . | . | . | . | . | . | . | . | . | . | . | . | . | . | . | . | . | . | . | . | . | . | . | . |
| 21-225_147B8_LC | . | G | . | . | . | . | . | . | . | . | . | . | . | . | . | . | . | . | . | . | . | . | . | . | . | N | T |
| 21-225_147B9_LC | . | . | . | . | . | . | . | . | . | . | . | . | . | . | . | . | . | . | . | . | . | . | . | . | . | S | T |
| 21-225_147D9_LC | . | . | R | . | . | . | . | . | . | . | . | . | . | . | . | . | . | . | . | . | . | . | . | . | . | N | T |
| 21-225_147E11_LC | . | . | . | . | . | . | . | . | . | . | . | . | . | . | . | . | . | . | . | . | . | . | . | . | . | . | . |
| 21-225_147E7_LC | . | . | . | . | . | . | . | . | . | . | . | . | . | . | . | . | . | . | . | . | . | . | . | . | . | . | . |
| 21-225_147F9_LC | . | H | . | . | . | . | . | . | . | . | . | . | . | . | . | . | . | . | . | . | . | . | . | . | . | . | . |
| 21-225_147G9_LC | . | H | . | . | . | . | . | . | . | . | . | . | . | . | . | . | . | . | . | . | . | . | . | . | . | . | . |
| 21-225_148A9_LC | . | H | . | . | . | . | . | . | . | . | . | . | . | . | . | . | . | . | . | . | . | . | . | . | . | . | . |
| 21-225_148C8_LC | . | . | . | . | . | . | . | . | . | . | . | . | . | . | . | . | . | . | . | . | . | . | . | . | . | S | T |
| 21-225_148E10_LC | . | . | R | . | . | . | . | . | . | . | . | . | . | . | . | . | . | . | . | . | . | . | . | . | . | N | T |
| 21-225_148G11_LC | . | . | . | . | . | . | . | . | . | . | . | . | . | . | . | . | . | . | . | . | . | . | . | . | . | . | . |
| 21-225_148G6_LC | . | . | . | . | . | . | . | . | . | . | . | . | . | . | . | . | . | . | . | . | . | . | . | . | . | . | . |
| 21-225_149A11_LC | . | . | . | . | . | . | . | . | . | . | . | . | . | . | . | . | . | . | . | . | . | . | . | . | . | S | T |
| 21-225_149B5_LC | . | . | . | . | . | . | . | . | . | . | . | . | . | . | . | . | . | . | . | . | . | . | . | . | . | . | . |
| 21-225_149C10_LC | . | . | . | . | . | . | . | . | . | . | . | . | . | . | . | . | . | . | . | . | . | . | . | . | . | . | . |
| 21-225_149C7_LC | . | . | . | . | . | . | . | . | . | . | . | . | . | . | . | . | . | . | . | . | . | . | . | . | . | . | . |
| 21-225_14B1_LC2_LC | . | . | N | . | . | . | . | . | . | . | . | . | . | . | . | . | . | . | . | . | . | . | . | . | . | . | . |
| 21-225_14B7_LC | . | N | N | . | . | . | . | . | . | . | . | . | . | . | . | . | . | . | . | . | . | . | . | . | . | . | . |

FIGURE 57

| Reference # | 109 | 110 | 111 | 112 | 113 | 114 | 115 | 116 | 117 | 118 | 119 | 120 | 121 | 122 | 123 | 124 | 125 | 126 | 127 | 128 | 129 | 130 | 131 | 132 | 133 | 134 | 135 |
|---|---|---|---|---|---|---|---|---|---|---|---|---|---|---|---|---|---|---|---|---|---|---|---|---|---|---|---|
| | | L_CDR3 | | | | | | | | | | | | | | | | | | | | | | | | | |
| CONSENSUS | W | D | S | - | - | - | - | - | - | - | - | - | - | - | - | - | - | - | - | - | - | - | - | - | - | - | S |
| 21-225_14G3_LC | . | G | N | . | . | . | . | . | . | . | . | . | . | . | . | . | . | . | . | . | . | . | . | . | . | S | T |
| 21-225_14H11_LC | . | . | - | . | . | . | . | . | . | . | . | . | . | . | . | . | . | . | . | . | . | . | . | . | . | . | . |
| 21-225_150D3_LC | . | H | . | . | . | . | . | . | . | . | . | . | . | . | . | . | . | . | . | . | . | . | . | . | . | . | . |
| 21-225_150F6_LC | . | . | . | . | . | . | . | . | . | . | . | . | . | . | . | . | . | . | . | . | . | . | . | . | . | . | . |
| 21-225_150H11_LC | . | . | . | . | . | . | . | . | . | . | . | . | . | . | . | . | . | . | . | . | . | . | . | . | . | . | . |
| 21-225_151B9_LC | . | . | . | . | . | . | . | . | . | . | . | . | . | . | . | . | . | . | . | . | . | . | . | . | . | . | . |
| 21-225_151F3_LC | . | H | . | . | . | . | . | . | . | . | . | . | . | . | . | . | . | . | . | . | . | . | . | . | . | . | . |
| 21-225_151H5_LC | . | . | . | . | . | . | . | . | . | . | . | . | . | . | . | . | . | . | . | . | . | . | . | . | . | . | . |
| 21-225_151H7_LC | . | . | . | . | . | . | . | . | . | . | . | . | . | . | . | . | . | . | . | . | . | . | . | . | . | . | . |
| 21-225_152D7_LC | . | . | . | . | . | . | . | . | . | . | . | . | . | . | . | . | . | . | . | . | . | . | . | . | . | . | . |
| 21-225_152G6_LC | . | . | N | . | . | . | . | . | . | . | . | . | . | . | . | . | . | . | . | . | . | . | . | . | . | . | . |
| 21-225_153D9_LC | . | H | . | . | . | . | . | . | . | . | . | . | . | . | . | . | . | . | . | . | . | . | . | . | . | . | . |
| 21-225_153E6_LC | . | . | . | . | . | . | . | . | . | . | . | . | . | . | . | . | . | . | . | . | . | . | . | . | . | S | T |
| 21-225_154C3_LC | . | H | . | . | . | . | . | . | . | . | . | . | . | . | . | . | . | . | . | . | . | . | . | . | . | . | . |
| 21-225_154C4_LC | . | . | . | . | . | . | . | . | . | . | . | . | . | . | . | . | . | . | . | . | . | . | . | . | . | . | . |
| 21-225_154D11_LC | . | H | . | . | . | . | . | . | . | . | . | . | . | . | . | . | . | . | . | . | . | . | . | . | . | . | . |
| 21-225_154E10_LC | . | . | N | . | . | . | . | . | . | . | . | . | . | . | . | . | . | . | . | . | . | . | . | . | . | . | . |
| 21-225_154F4_LC | . | . | N | . | . | . | . | . | . | . | . | . | . | . | . | . | . | . | . | . | . | . | . | . | . | . | . |
| 21-225_155C10_LC | . | . | . | . | . | . | . | . | . | . | . | . | . | . | . | . | . | . | . | . | . | . | . | . | . | . | . |
| 21-225_155E10_LC | . | . | . | . | . | . | . | . | . | . | . | . | . | . | . | . | . | . | . | . | . | . | . | . | . | S | T |
| 21-225_158D10_LC | . | G | N | . | . | . | . | . | . | . | . | . | . | . | . | . | . | . | . | . | . | . | . | . | . | S | . |
| 21-225_158E9_LC | . | . | N | . | . | . | . | . | . | . | . | . | . | . | . | . | . | . | . | . | . | . | . | . | . | S | . |
| 21-225_159H8_LC | . | G | N | . | . | . | . | . | . | . | . | . | . | . | . | . | . | . | . | . | . | . | . | . | . | S | . |
| 21-225_15H11_LC | . | . | . | . | . | . | . | . | . | . | . | . | . | . | . | . | . | . | . | . | . | . | . | . | . | . | . |
| 21-225_160B12_LC | . | G | N | . | . | . | . | . | . | . | . | . | . | . | . | . | . | . | . | . | . | . | . | . | . | S | . |
| 21-225_161E10_LC | . | . | N | . | . | . | . | . | . | . | . | . | . | . | . | . | . | . | . | . | . | . | . | . | . | S | . |
| 21-225_161F12_LC | . | . | . | . | . | . | . | . | . | . | . | . | . | . | . | . | . | . | . | . | . | . | . | . | . | . | T |
| 21-225_161H3_LC | . | V | N | . | . | . | . | . | . | . | . | . | . | . | . | . | . | . | . | . | . | . | . | . | . | N | T |
| 21-225_165H3_LC | . | . | . | . | . | . | . | . | . | . | . | . | . | . | . | . | . | . | . | . | . | . | . | . | . | . | T |

FIGURE 57

| Reference # | 109 | 110 | 111 | 112 | 113 | 114 | 115 | 116 | 117 | 118 | 119 | 120 | 121 | 122 | 123 | 124 | 125 | 126 | 127 | 128 | 129 | 130 | 131 | 132 | 133 | 134 | 135 |
|---|---|---|---|---|---|---|---|---|---|---|---|---|---|---|---|---|---|---|---|---|---|---|---|---|---|---|---|
| | | | | | | | | | | | | | L_CDR3 | | | | | | | | | | | | | | |
| CONSENSUS | W | D | S | - | - | - | - | - | - | - | - | - | - | - | - | - | - | - | - | - | - | - | - | - | - | - | S |
| 21-225_166G11_LC | . | . | N | . | . | . | . | . | . | . | . | . | . | . | . | . | . | . | . | . | . | . | . | . | . | . | . |
| 21-225_169A6_LC | . | . | T | . | . | . | . | . | . | . | . | . | . | . | . | . | . | . | . | . | . | . | . | . | . | . | N |
| 21-225_169B7_LC | . | . | K | . | . | . | . | . | . | . | . | . | . | . | . | . | . | . | . | . | . | . | . | . | . | . | N |
| 21-225_169C1_LC | . | . | T | . | . | . | . | . | . | . | . | . | . | . | . | . | . | . | . | . | . | . | . | . | . | . | N |
| 21-225_169G11_LC | . | . | N | . | . | . | . | . | . | . | . | . | . | . | . | . | . | . | . | . | . | . | . | . | . | . | . |
| 21-225_16D2_LC | . | . | . | . | . | . | . | . | . | . | . | . | . | . | . | . | . | . | . | . | . | . | . | . | . | S | T |
| 21-225_16E4_LC | . | . | . | . | . | . | . | . | . | . | . | . | . | . | . | . | . | . | . | . | . | . | . | . | . | . | . |
| 21-225_16G8_LC | . | V | N | . | . | . | . | . | . | . | . | . | . | . | . | . | . | . | . | . | . | . | . | . | . | . | N |
| 21-225_170A5_LC | . | . | N | . | . | . | . | . | . | . | . | . | . | . | . | . | . | . | . | . | . | . | . | . | . | . | . |
| 21-225_170F1_LC | . | . | . | . | . | . | . | . | . | . | . | . | . | . | . | . | . | . | . | . | . | . | . | . | . | . | N |
| 21-225_171E12_LC | . | . | I | . | . | . | . | . | . | . | . | . | . | . | . | . | . | . | . | . | . | . | . | . | . | S | T |
| 21-225_171F5_LC | . | . | K | . | . | . | . | . | . | . | . | . | . | . | . | . | . | . | . | . | . | . | . | . | . | . | N |
| 21-225_173F8_LC | . | . | . | . | . | . | . | . | . | . | . | . | . | . | . | . | . | . | . | . | . | . | . | . | . | . | F |
| 21-225_175C6_LC | . | . | N | . | . | . | . | . | . | . | . | . | . | . | . | . | . | . | . | . | . | . | . | . | . | . | . |
| 21-225_179C7_LC | . | . | . | . | . | . | . | . | . | . | . | . | . | . | . | . | . | . | . | . | . | . | . | . | . | N | T |
| 21-225_17D3_LC | . | . | . | . | . | . | . | . | . | . | . | . | . | . | . | . | . | . | . | . | . | . | . | . | . | . | I |
| 21-225_17F5_LC | . | . | - | . | . | . | . | . | . | . | . | . | . | . | . | . | . | . | . | . | . | . | . | . | . | . | . |
| 21-225_180C5_LC | . | . | . | . | . | . | . | . | . | . | . | . | . | . | . | . | . | . | . | . | . | . | . | . | . | . | . |
| 21-225_180D4_LC | . | . | . | . | . | . | . | . | . | . | . | . | . | . | . | . | . | . | . | . | . | . | . | . | . | . | [illegible] |
| 21-225_180G5_LC | . | . | I | . | . | . | . | . | . | . | . | . | . | . | . | . | . | . | . | . | . | . | . | . | . | . | T |
| 21-225_181H1_LC | . | . | . | . | . | . | . | . | . | . | . | . | . | . | . | . | . | . | . | . | . | . | . | . | . | . | [illegible] |
| 21-225_182D12_LC | . | . | . | . | . | . | . | . | . | . | . | . | . | . | . | . | . | . | . | . | . | . | . | . | . | . | [illegible] |
| 21-225_183F4_LC | . | . | N | . | . | . | . | . | . | . | . | . | . | . | . | . | . | . | . | . | . | . | . | . | . | S | T |
| 21-225_185D2_LC | . | . | . | . | . | . | . | . | . | . | . | . | . | . | . | . | . | . | . | . | . | . | . | . | . | . | [illegible] |
| 21-225_185G7_LC | . | . | - | . | . | . | . | . | . | . | . | . | . | . | . | . | . | . | . | . | . | . | . | . | . | . | . |
| 21-225_186H6_LC | . | . | . | . | . | . | . | . | . | . | . | . | . | . | . | . | . | . | . | . | . | . | . | . | . | . | . |
| 21-225_18E10_LC | . | . | . | . | . | . | . | . | . | . | . | . | . | . | . | . | . | . | . | . | . | . | . | . | . | . | . |
| 21-225_18G5_LC | . | N | . | . | . | . | . | . | . | . | . | . | . | . | . | . | . | . | . | . | . | . | . | . | . | . | . |
| 21-225_190G9_LC | . | . | . | . | . | . | . | . | . | . | . | . | . | . | . | . | . | . | . | . | . | . | . | . | . | . | . |

FIGURE 57

| Reference # | 109 | 110 | 111 | 112 | 113 | 114 | 115 | 116 | 117 | 118 | 119 | 120 | 121 | 122 | 123 | 124 | 125 | 126 | 127 | 128 | 129 | 130 | 131 | 132 | 133 | 134 | 135 |
|---|---|---|---|---|---|---|---|---|---|---|---|---|---|---|---|---|---|---|---|---|---|---|---|---|---|---|---|
| | L_CDR3 | | | | | | | | | | | | | | | | | | | | | | | | | | |
| CONSENSUS | W | D | S | - | - | - | - | - | - | - | - | - | - | - | - | - | - | - | - | - | - | - | - | - | - | - | S |
| 21-225_190H1_LC | . | . | . | . | . | . | . | . | . | . | . | . | . | . | . | . | . | . | . | . | . | . | . | . | . | . | . |
| 21-225_193H5_LC | . | . | . | . | . | . | . | . | . | . | . | . | . | . | . | . | . | . | . | . | . | . | . | . | . | . | . |
| 21-225_195E3_LC | . | . | . | . | . | . | . | . | . | . | . | . | . | . | . | . | . | . | . | . | . | . | . | . | . | . | V |
| 21-225_195E9_LC | . | . | R | . | . | . | . | . | . | . | . | . | . | . | . | . | . | . | . | . | . | . | . | . | . | . | G |
| 21-225_19F6_LC | . | . | N | . | . | . | . | . | . | . | . | . | . | . | . | . | . | . | . | . | . | . | . | . | . | . | N |
| 21-225_201G11_LC | . | . | . | . | . | . | . | . | . | . | . | . | . | . | . | . | . | . | . | . | . | . | . | . | . | . | . |
| 21-225_203G6_LC | . | . | . | . | . | . | . | . | . | . | . | . | . | . | . | . | . | . | . | . | . | . | . | . | . | . | . |
| 21-225_215B3_LC | . | . | . | . | . | . | . | . | . | . | . | . | . | . | . | . | . | . | . | . | . | . | . | . | . | . | . |
| 21-225_21D12_LC | . | . | N | . | . | . | . | . | . | . | . | . | . | . | . | . | . | . | . | . | . | . | . | . | . | . | . |
| 21-225_224H2_LC | . | . | . | . | . | . | . | . | . | . | . | . | . | . | . | . | . | . | . | . | . | . | . | . | . | . | . |
| 21-225_225E9_LC | . | . | . | . | . | . | . | . | . | . | . | . | . | . | . | . | . | . | . | . | . | . | . | . | . | R | T |
| 21-225_226A10_LC | . | . | N | . | . | . | . | . | . | . | . | . | . | . | . | . | . | . | . | . | . | . | . | . | . | . | G |
| 21-225_226B2_LC | . | . | N | . | . | . | . | . | . | . | . | . | . | . | . | . | . | . | . | . | . | . | . | . | . | . | G |
| 21-225_227E12_LC | . | N | . | . | . | . | . | . | . | . | . | . | . | . | . | . | . | . | . | . | . | . | . | . | . | . | . |
| 21-225_227E5_LC | . | V | N | . | . | . | . | . | . | . | . | . | . | . | . | . | . | . | . | . | . | . | . | . | . | . | N |
| 21-225_22E6_LC | . | . | . | . | . | . | . | . | . | . | . | . | . | . | . | . | . | . | . | . | . | . | . | . | . | . | . |
| 21-225_23D7_LC | . | N | . | . | . | . | . | . | . | . | . | . | . | . | . | . | . | . | . | . | . | . | . | . | . | . | . |
| 21-225_26C10_LC | . | V | - | . | . | . | . | . | . | . | . | . | . | . | . | . | . | . | . | . | . | . | . | . | . | . | N |
| 21-225_27D9_LC | . | V | - | . | . | . | . | . | . | . | . | . | . | . | . | . | . | . | . | . | . | . | . | . | . | . | N |
| 21-225_27E7_LC | . | . | . | . | . | . | . | . | . | . | . | . | . | . | . | . | . | . | . | . | . | . | . | . | . | . | . |
| 21-225_27G6_LC | . | . | . | . | . | . | . | . | . | . | . | . | . | . | . | . | . | . | . | . | . | . | . | . | . | S | . |
| 21-225_27H2_LC | . | . | . | . | . | . | . | . | . | . | . | . | . | . | . | . | . | . | . | . | . | . | . | . | . | S | T |
| 21-225_28A11_LC | . | . | . | . | . | . | . | . | . | . | . | . | . | . | . | . | . | . | . | . | . | . | . | . | . | S | T |
| 21-225_2B1_LC | . | . | N | . | . | . | . | . | . | . | . | . | . | . | . | . | . | . | . | . | . | . | . | . | . | . | N |
| 21-225_30D9_LC2_LC | . | . | N | . | . | . | . | . | . | . | . | . | . | . | . | . | . | . | . | . | . | . | . | . | . | . | . |
| 21-225_30H6_LC | . | . | - | . | . | . | . | . | . | . | . | . | . | . | . | . | . | . | . | . | . | . | . | . | . | . | . |
| 21-225_31C2_LC | . | . | - | . | . | . | . | . | . | . | . | . | . | . | . | . | . | . | . | . | . | . | . | . | . | . | . |
| 21-225_31D12_LC2_LC | . | . | N | . | . | . | . | . | . | . | . | . | . | . | . | . | . | . | . | . | . | . | . | . | . | . | . |
| 21-225_32B11_LC | . | . | N | . | . | . | . | . | . | . | . | . | . | . | . | . | . | . | . | . | . | . | . | . | . | . | . |

FIGURE 57

| Reference # | 109 | 110 | 111 | 112 | 113 | 114 | 115 | 116 | 117 | 118 | 119 | 120 | 121 | 122 | 123 | 124 | 125 | 126 | 127 | 128 | 129 | 130 | 131 | 132 | 133 | 134 | 135 |
|---|---|---|---|---|---|---|---|---|---|---|---|---|---|---|---|---|---|---|---|---|---|---|---|---|---|---|---|
| | | | | | | | | | | | | | | L_CDR3 | | | | | | | | | | | | | |
| CONSENSUS | W | D | S | - | - | - | - | - | - | - | - | - | - | - | - | - | - | - | - | - | - | - | - | - | - | - | S |
| 21-225_33A1_LC | . | . | . | . | . | . | . | . | . | . | . | . | . | . | . | . | . | . | . | . | . | . | . | . | . | . | T |
| 21-225_33E6_LC | . | . | N | . | . | . | . | . | . | . | . | . | . | . | . | . | . | . | . | . | . | . | . | . | . | . | . |
| 21-225_34B11_LC | . | . | N | . | . | . | . | . | . | . | . | . | . | . | . | . | . | . | . | . | . | . | . | . | . | . | . |
| 21-225_34B9_LC | . | . | - | . | . | . | . | . | . | . | . | . | . | . | . | . | . | . | . | . | . | . | . | . | . | . | . |
| 21-225_34D7_LC | . | . | N | . | . | . | . | . | . | . | . | . | . | . | . | . | . | . | . | . | . | . | . | . | . | . | T |
| 21-225_35E1_LC | . | . | N | . | . | . | . | . | . | . | . | . | . | . | . | . | . | . | . | . | . | . | . | . | . | . | . |
| 21-225_3B12_LC | . | V | N | . | . | . | . | . | . | . | . | . | . | . | . | . | . | . | . | . | . | . | . | . | . | . | N |
| 21-225_3H10_LC | . | V | N | . | . | . | . | . | . | . | . | . | . | . | . | . | . | . | . | . | . | . | . | . | . | . | N |
| 21-225_45F6_LC | . | . | N | . | . | . | . | . | . | . | . | . | . | . | . | . | . | . | . | . | . | . | . | . | . | . | R |
| 21-225_4B3_LC | . | . | N | . | . | . | . | . | . | . | . | . | . | . | . | . | . | . | . | . | . | . | . | . | . | . | N |
| 21-225_50G9_LC | R | N | . | . | . | . | . | . | . | . | . | . | . | . | . | . | . | . | . | . | . | . | . | . | . | . | R |
| 21-225_52H1_LC | . | . | N | . | . | . | . | . | . | . | . | . | . | . | . | . | . | . | . | . | . | . | . | . | . | . | . |
| 21-225_53D10_LC | . | . | . | . | . | . | . | . | . | . | . | . | . | . | . | . | . | . | . | . | . | . | . | . | . | . | . |
| 21-225_57D9_LC | . | . | . | . | . | . | . | . | . | . | . | . | . | . | . | . | . | . | . | . | . | . | . | . | . | . | . |
| 21-225_57F1_LC | . | . | . | . | . | . | . | . | . | . | . | . | . | . | . | . | . | . | . | . | . | . | . | . | . | . | . |
| 21-225_57H11_LC | . | . | . | . | . | . | . | . | . | . | . | . | . | . | . | . | . | . | . | . | . | . | . | . | . | . | . |
| 21-225_58C1_LC | . | . | - | . | . | . | . | . | . | . | . | . | . | . | . | . | . | . | . | . | . | . | . | . | . | . | . |
| 21-225_58E7_LC | . | N | N | . | . | . | . | . | . | . | . | . | . | . | . | . | . | . | . | . | . | . | . | . | . | . | Y |
| 21-225_58F7_LC | . | . | . | . | . | . | . | . | . | . | . | . | . | . | . | . | . | . | . | . | . | . | . | . | . | . | . |
| 21-225_58F8_LC | . | . | . | . | . | . | . | . | . | . | . | . | . | . | . | . | . | . | . | . | . | . | . | . | . | . | . |
| 21-225_58G11_LC | . | N | N | . | . | . | . | . | . | . | . | . | . | . | . | . | . | . | . | . | . | . | . | . | . | . | N |
| 21-225_59B11_LC | . | . | . | . | . | . | . | . | . | . | . | . | . | . | . | . | . | . | . | . | . | . | . | . | . | S | T |
| 21-225_59F2_LC | . | . | N | . | . | . | . | . | . | . | . | . | . | . | . | . | . | . | . | . | . | . | . | . | . | . | N |
| 21-225_5G7_LC | . | . | N | . | . | . | . | . | . | . | . | . | . | . | . | . | . | . | . | . | . | . | . | . | . | . | . |
| 21-225_60A12_LC | . | . | - | . | . | . | . | . | . | . | . | . | . | . | . | . | . | . | . | . | . | . | . | . | . | . | . |
| 21-225_60B1_LC | . | . | . | . | . | . | . | . | . | . | . | . | . | . | . | . | . | . | . | . | . | . | . | . | . | . | . |
| 21-225_60D2_LC | . | . | N | . | . | . | . | . | . | . | . | . | . | . | . | . | . | . | . | . | . | . | . | . | . | . | N |
| 21-225_61F5_LC | . | . | . | . | . | . | . | . | . | . | . | . | . | . | . | . | . | . | . | . | . | . | . | . | . | . | . |
| 21-225_62A12_LC | . | . | . | . | . | . | . | . | . | . | . | . | . | . | . | . | . | . | . | . | . | . | . | . | . | . | . |

FIGURE 57

| Reference # | 109 | 110 | 111 | 112 | 113 | 114 | 115 | 116 | 117 | 118 | 119 | 120 | 121 | 122 | 123 | 124 | 125 | 126 | 127 | 128 | 129 | 130 | 131 | 132 | 133 | 134 | 135 |
|---|---|---|---|---|---|---|---|---|---|---|---|---|---|---|---|---|---|---|---|---|---|---|---|---|---|---|---|
| | L_CDR3 | | | | | | | | | | | | | | | | | | | | | | | | | | |
| CONSENSUS | W | D | S | - | - | - | - | - | - | - | - | - | - | - | - | - | - | - | - | - | - | - | - | - | - | - | S |
| 21-225_62B12_LC | . | . | . | . | . | . | . | . | . | . | . | . | . | . | . | . | . | . | . | . | . | . | . | . | . | . | . |
| 21-225_62D10_LC | . | . | . | . | . | . | . | . | . | . | . | . | . | . | . | . | . | . | . | . | . | . | . | . | . | . | . |
| 21-225_62E3_LC | . | . | . | . | . | . | . | . | . | . | . | . | . | . | . | . | . | . | . | . | . | . | . | . | . | . | . |
| 21-225_62E8_LC | . | . | . | . | . | . | . | . | . | . | . | . | . | . | . | . | . | . | . | . | . | . | . | . | . | . | . |
| 21-225_65E9_LC | . | . | N | . | . | . | . | . | . | . | . | . | . | . | . | . | . | . | . | . | . | . | . | . | . | . | . |
| 21-225_66G9_LC | . | . | I | . | . | . | . | . | . | . | . | . | . | . | . | . | . | . | . | . | . | . | . | . | . | . | N |
| 21-225_67F10_LC | . | . | N | . | . | . | . | . | . | . | . | . | . | . | . | . | . | . | . | . | . | . | . | . | . | . | . |
| 21-225_68D8_LC | . | . | N | . | . | . | . | . | . | . | . | . | . | . | . | . | . | . | . | . | . | . | . | . | . | . | . |
| 21-225_68G6_LC | . | . | N | . | . | . | . | . | . | . | . | . | . | . | . | . | . | . | . | . | . | . | . | . | . | . | . |
| 21-225_69B11_LC | . | . | N | . | . | . | . | . | . | . | . | . | . | . | . | . | . | . | . | . | . | . | . | . | . | . | . |
| 21-225_69B9_LC | . | . | N | . | . | . | . | . | . | . | . | . | . | . | . | . | . | . | . | . | . | . | . | . | . | . | . |
| 21-225_6B4_LC | . | . | N | . | . | . | . | . | . | . | . | . | . | . | . | . | . | . | . | . | . | . | . | . | . | . | N |
| 21-225_6D9_LC | . | . | - | . | . | . | . | . | . | . | . | . | . | . | . | . | . | . | . | . | . | . | . | . | . | . | . |
| 21-225_71D4_LC | . | V | N | . | . | . | . | . | . | . | . | . | . | . | . | . | . | . | . | . | . | . | . | . | . | . | . |
| 21-225_72B4_LC | . | . | N | . | . | . | . | . | . | . | . | . | . | . | . | . | . | . | . | . | . | . | . | . | . | . | . |
| 21-225_72D5_LC | . | . | . | . | . | . | . | . | . | . | . | . | . | . | . | . | . | . | . | . | . | . | . | . | . | . | . |
| 21-225_73C4_LC | . | . | . | . | . | . | . | . | . | . | . | . | . | . | . | . | . | . | . | . | . | . | . | . | . | . | . |
| 21-225_74A9_LC | . | . | . | . | . | . | . | . | . | . | . | . | . | . | . | . | . | . | . | . | . | . | . | . | . | . | . |
| 21-225_74B3_LC | . | . | . | . | . | . | . | . | . | . | . | . | . | . | . | . | . | . | . | . | . | . | . | . | . | . | T |
| 21-225_74C9_LC | . | . | . | . | . | . | . | . | . | . | . | . | . | . | . | . | . | . | . | . | . | . | . | . | . | . | T |
| 21-225_76B4_LC | . | . | - | . | . | . | . | . | . | . | . | . | . | . | . | . | . | . | . | . | . | . | . | . | . | . | . |
| 21-225_77A2_LC | . | . | . | . | . | . | . | . | . | . | . | . | . | . | . | . | . | . | . | . | . | . | . | . | . | . | . |
| 21-225_78E9_LC | . | . | . | . | . | . | . | . | . | . | . | . | . | . | . | . | . | . | . | . | . | . | . | . | . | . | . |
| 21-225_79E7_LC | . | . | . | . | . | . | . | . | . | . | . | . | . | . | . | . | . | . | . | . | . | . | . | . | . | . | . |
| 21-225_8D8_LC | . | . | . | . | . | . | . | . | . | . | . | . | . | . | . | . | . | . | . | . | . | . | . | . | . | . | . |
| 21-225_92A4_LC | . | . | . | . | . | . | . | . | . | . | . | . | . | . | . | . | . | . | . | . | . | . | . | . | . | . | . |
| 21-225_96B5_LC | . | . | - | . | . | . | . | . | . | . | . | . | . | . | . | . | . | . | . | . | . | . | . | . | . | . | . |
| 21-225_97E6_LC | . | . | . | . | . | . | . | . | . | . | . | . | . | . | . | . | . | . | . | . | . | . | . | . | . | . | T |
| 21-225_9H10_LC | . | . | . | . | . | . | . | . | . | . | . | . | . | . | . | . | . | . | . | . | . | . | . | . | . | S | T |

# FIGURE 57

| Reference # | 136 | 137 | 138 | 139 | 140 | 141 | 142 | 143 | 144 | 145 | 146 | 147 | 148 | 149 |
|---|---|---|---|---|---|---|---|---|---|---|---|---|---|---|
| | | | | L_FR4 | | | | | | | | | | |
| CONSENSUS | T | V | V | F | G | G | G | T | K | L | T | V | L | G |
| 21-225_10B10_LC | . | . | . | . | . | . | . | . | . | . | . | . | . | . |
| 21-225_12B1_LC | . | . | I | . | . | . | . | . | . | . | . | . | . | . |
| 21-225_13F6_LC | A | . | . | . | . | . | . | . | . | . | . | . | . | . |
| 21-225_146A10_LC | . | . | . | . | . | . | . | . | . | V | . | . | . | . |
| 21-225_146A2_LC | . | . | . | . | . | . | . | . | . | . | . | . | . | . |
| 21-225_146A3_LC | . | . | . | . | . | . | . | . | . | . | . | . | . | . |
| 21-225_146B11_LC | . | . | . | . | . | . | . | . | . | . | . | . | . | . |
| 21-225_146B8_LC | . | . | . | . | . | . | . | . | . | . | . | . | . | . |
| 21-225_146C11_LC | . | . | . | . | . | . | . | . | . | . | . | . | . | . |
| 21-225_146H8_LC | . | . | . | . | . | . | . | . | . | . | . | . | . | . |
| 21-225_147B12_LC | . | . | . | . | . | . | . | . | . | . | . | . | . | . |
| 21-225_147B8_LC | A | . | . | . | . | . | . | . | . | . | . | . | . | . |
| 21-225_147B9_LC | F | . | . | . | . | . | . | . | . | . | . | . | . | . |
| 21-225_147D9_LC | Y | . | . | . | . | . | . | . | . | . | . | . | . | . |
| 21-225_147E11_LC | . | . | . | . | . | . | . | . | . | . | . | . | . | . |
| 21-225_147E7_LC | . | . | . | . | . | . | . | . | . | . | . | . | . | . |
| 21-225_147F9_LC | . | . | . | . | . | . | . | . | . | . | . | . | . | . |
| 21-225_147G9_LC | . | . | . | . | . | . | . | . | . | . | . | . | . | . |
| 21-225_148A9_LC | . | . | . | . | . | . | . | . | . | . | . | . | . | . |
| 21-225_148C8_LC | F | . | . | . | . | . | . | . | . | . | . | . | . | . |
| 21-225_148E10_LC | Y | . | . | . | . | . | . | . | . | . | . | . | . | . |
| 21-225_148G11_LC | . | . | . | . | . | . | . | . | . | . | . | . | . | . |
| 21-225_148G6_LC | . | . | . | . | . | . | . | . | . | . | . | . | . | . |
| 21-225_149A11_LC | F | . | . | . | . | . | . | . | . | . | . | . | . | . |
| 21-225_149B5_LC | . | . | . | . | . | . | . | . | . | . | . | . | . | . |
| 21-225_149C10_LC | . | . | . | . | . | . | . | . | . | . | . | . | . | . |
| 21-225_149C7_LC | . | . | . | . | . | . | . | . | . | . | . | . | P | . |
| 21-225_14B1_LC2_LC | . | . | . | . | . | . | . | . | . | . | . | . | . | . |
| 21-225_14B7_LC | . | . | . | . | . | . | . | . | . | . | . | . | . | S |

FIGURE 57

| Reference # | 136 | 137 | 138 | 139 | 140 | 141 | 142 | 143 | 144 | 145 | 146 | 147 | 148 | 149 |
|---|---|---|---|---|---|---|---|---|---|---|---|---|---|---|
| | | | L_FR4 | | | | | | | | | | | |
| CONSENSUS | T | V | V | F | G | G | G | T | K | L | T | V | L | G |
| 21-225_14G3_LC | A | . | . | . | . | . | . | . | . | . | . | . | . | . |
| 21-225_14H11_LC | S | T | I | . | . | . | . | . | . | . | . | . | . | . |
| 21-225_150D3_LC | . | . | . | . | . | . | . | . | . | . | . | . | . | . |
| 21-225_150F6_LC | . | . | . | . | . | . | . | . | . | . | . | . | . | . |
| 21-225_150H11_LC | . | . | . | . | . | . | . | . | . | . | . | . | . | . |
| 21-225_151B9_LC | . | . | . | . | . | . | . | . | . | . | . | . | . | . |
| 21-225_151F3_LC | . | . | . | . | . | . | . | . | . | . | . | . | . | . |
| 21-225_151H5_LC | . | . | . | . | . | . | . | . | . | . | . | . | . | . |
| 21-225_151H7_LC | . | . | . | . | . | . | . | . | . | . | . | . | . | . |
| 21-225_152D7_LC | . | . | . | . | . | . | . | . | . | . | . | . | . | . |
| 21-225_152G6_LC | . | . | . | . | . | . | . | . | . | . | . | . | . | . |
| 21-225_153D9_LC | . | . | . | . | . | . | . | . | . | V | . | . | . | . |
| 21-225_153E8_LC | Y | . | I | . | . | . | . | . | . | . | . | . | . | . |
| 21-225_154C3_LC | . | . | . | . | . | . | . | . | . | . | . | . | . | . |
| 21-225_154C4_LC | . | . | . | . | . | . | . | . | . | . | . | . | . | . |
| 21-225_154D11_LC | I | . | . | . | . | . | . | . | . | . | . | . | . | . |
| 21-225_154E10_LC | . | . | . | . | . | . | . | . | . | . | . | . | . | . |
| 21-225_154F4_LC | . | L | . | . | . | . | . | . | . | . | . | . | . | . |
| 21-225_155C10_LC | . | . | . | . | . | . | . | . | . | . | . | . | . | . |
| 21-225_155E10_LC | F | . | . | . | . | . | . | . | . | . | . | . | . | . |
| 21-225_158D10_LC | F | . | . | . | . | . | . | . | . | . | . | . | . | . |
| 21-225_158E9_LC | F | . | L | . | . | . | . | . | . | . | . | . | . | . |
| 21-225_159H8_LC | F | . | . | . | . | . | . | . | . | . | . | . | . | . |
| 21-225_15H11_LC | . | A | . | . | . | . | . | . | . | . | . | . | . | . |
| 21-225_160B12_LC | F | . | . | . | . | . | . | . | . | . | . | . | . | . |
| 21-225_161E10_LC | F | A | L | . | . | . | . | . | . | . | . | . | . | . |
| 21-225_161F12_LC | . | L | . | . | . | . | . | . | . | . | . | . | . | . |
| 21-225_161H3_LC | A | . | . | . | . | . | . | . | . | . | . | . | . | . |
| 21-225_165H3_LC | . | L | . | . | . | . | . | . | . | . | . | . | . | . |

FIGURE 57

| Reference # | 136 | 137 | 138 | 139 | 140 | 141 | 142 | 143 | 144 | 145 | 146 | 147 | 148 | 149 |
|---|---|---|---|---|---|---|---|---|---|---|---|---|---|---|
| | | | | L_FR4 | | | | | | | | | | |
| CONSENSUS | T | V | V | F | G | G | G | T | K | L | T | V | L | G |
| 21-225_166G11_LC | . | A | . | . | . | . | . | . | . | . | . | . | . | . |
| 21-225_169A6_LC | . | . | L | . | . | . | . | . | . | . | . | . | . | . |
| 21-225_169B7_LC | . | . | . | . | . | . | . | . | . | . | . | . | . | . |
| 21-225_169C1_LC | . | . | I | . | . | . | . | . | . | . | . | . | . | . |
| 21-225_169G11_LC | . | A | . | . | . | . | . | . | . | . | . | . | . | . |
| 21-225_16D2_LC | Y | . | . | . | . | . | . | . | . | . | . | . | . | . |
| 21-225_16E4_LC | . | . | . | . | . | . | . | . | . | . | . | . | . | . |
| 21-225_16G8_LC | . | M | I | . | . | . | . | . | . | . | . | . | . | . |
| 21-225_170A5_LC | . | M | . | . | . | . | . | . | R | . | . | . | . | . |
| 21-225_170F1_LC | . | A | . | . | . | . | . | . | . | . | . | . | . | . |
| 21-225_171E12_LC | Y | . | . | . | . | . | . | . | . | . | . | . | . | . |
| 21-225_171F5_LC | . | . | . | . | . | . | . | . | . | . | . | . | . | . |
| 21-225_173F8_LC | . | . | . | . | . | . | . | . | . | . | . | . | . | . |
| 21-225_175C6_LC | . | M | . | . | . | . | . | . | R | . | . | . | . | . |
| 21-225_179C7_LC | A | . | . | . | . | . | . | . | . | . | . | . | . | . |
| 21-225_17D3_LC | . | A | . | . | . | . | . | . | . | . | . | . | R | . |
| 21-225_17F5_LC | S | T | . | . | . | . | . | . | . | . | . | . | . | . |
| 21-225_180C5_LC | . | A | . | . | . | . | . | . | . | . | . | . | . | . |
| 21-225_180D4_LC | K | . | . | . | . | . | . | . | . | . | . | . | . | . |
| 21-225_180G5_LC | . | A | . | . | . | . | . | . | . | . | . | . | . | . |
| 21-225_181H1_LC | K | . | . | . | . | . | . | . | . | . | . | . | . | . |
| 21-225_182D12_LC | . | A | . | . | . | . | . | . | . | . | . | . | . | . |
| 21-225_183F4_LC | A | . | . | . | . | . | . | . | . | . | . | . | . | S |
| 21-225_185D2_LC | K | . | . | . | . | . | . | . | . | . | . | . | . | . |
| 21-225_185G7_LC | S | T | . | . | . | . | . | . | . | . | . | . | . | . |
| 21-225_186H6_LC | . | A | . | . | . | . | . | . | . | . | . | . | . | . |
| 21-225_18E10_LC | . | . | . | . | . | . | . | . | . | . | . | . | . | . |
| 21-225_18G5_LC | N | . | . | . | . | . | . | . | . | . | . | . | . | . |
| 21-225_190G9_LC | . | . | . | . | . | . | . | . | R | . | . | . | . | R |

FIGURE 57

| Reference # | 136 | 137 | 138 | 139 | 140 | 141 | 142 | 143 | 144 | 145 | 146 | 147 | 148 | 149 |
|---|---|---|---|---|---|---|---|---|---|---|---|---|---|---|
| | | | | L_FR4 | | | | | | | | | | |
| CONSENSUS | T | V | V | F | G | G | G | T | K | L | T | V | L | G |
| 21-225_190H1_LC | . | . | . | . | . | . | . | . | . | . | . | . | . | . |
| 21-225_193H5_LC | . | A | . | . | . | . | . | . | . | . | . | . | . | . |
| 21-225_195E3_LC | . | . | . | . | . | . | . | . | . | . | I | . | . | . |
| 21-225_195E9_LC | I | . | . | . | . | . | . | . | . | . | . | . | . | . |
| 21-225_19F6_LC | . | . | . | . | . | . | . | . | . | . | . | . | . | . |
| 21-225_201G11_LC | . | . | . | . | . | . | . | . | . | . | . | . | . | . |
| 21-225_203G6_LC | . | . | . | . | . | . | . | . | . | . | . | . | . | . |
| 21-225_215B3_LC | . | . | . | . | . | . | . | . | . | . | . | . | . | . |
| 21-225_21D12_LC | . | . | . | . | . | . | . | . | R | . | . | . | . | . |
| 21-225_224H2_LC | . | . | . | . | . | . | . | . | . | . | . | . | . | . |
| 21-225_225E9_LC | A | . | . | . | . | . | . | . | . | . | . | . | . | . |
| 21-225_226A10_LC | A | A | . | . | . | . | . | . | . | . | . | . | . | . |
| 21-225_226B2_LC | A | A | . | . | . | . | . | . | . | . | . | . | . | . |
| 21-225_227E12_LC | N | . | . | . | . | . | . | . | . | . | . | . | . | . |
| 21-225_227E5_LC | . | M | I | . | . | . | . | . | . | . | . | . | . | . |
| 21-225_22E6_LC | . | A | . | . | . | . | . | . | . | . | . | . | . | . |
| 21-225_23D7_LC | N | . | . | . | . | . | . | . | . | . | . | . | . | S |
| 21-225_26C10_LC | N | T | . | . | . | . | . | . | . | . | . | . | . | . |
| 21-225_27D9_LC | N | T | . | . | . | . | . | . | . | . | . | . | . | . |
| 21-225_27E7_LC | . | . | . | . | . | . | . | . | . | . | . | . | . | . |
| 21-225_27G6_LC | Y | . | . | . | . | . | . | . | . | . | . | . | . | . |
| 21-225_27H2_LC | Y | . | . | . | . | . | . | . | . | . | . | . | . | . |
| 21-225_28A11_LC | Y | . | . | . | . | . | . | . | . | . | . | . | . | . |
| 21-225_2B1_LC | . | . | . | . | . | . | . | . | . | . | . | . | . | . |
| 21-225_30D9_LC2_LC | . | A | . | . | . | . | . | . | . | . | . | . | . | . |
| 21-225_30H6_LC | S | T | . | . | . | . | . | . | . | . | . | . | . | . |
| 21-225_31C2_LC | S | T | . | . | . | . | . | . | . | . | . | . | . | . |
| 21-225_31D12_LC2_LC | . | A | . | . | . | . | . | . | . | . | . | . | . | . |
| 21-225_32B11_LC | . | . | . | . | . | . | . | . | . | . | . | . | . | . |

FIGURE 57

| Reference # | 136 | 137 | 138 | 139 | 140 | 141 | 142 | 143 | 144 | 145 | 146 | 147 | 148 | 149 |
|---|---|---|---|---|---|---|---|---|---|---|---|---|---|---|
| | | | | L_FR4 | | | | | | | | | | |
| CONSENSUS | T | V | V | F | G | G | G | T | K | L | T | V | L | G |
| 21-225_33A1_LC | . | . | . | . | . | . | . | . | . | . | . | . | . | . |
| 21-225_33E6_LC | . | A | . | . | . | . | . | . | . | . | . | . | . | . |
| 21-225_34B11_LC | . | A | . | . | . | . | . | . | . | . | . | . | . | . |
| 21-225_34B9_LC | S | T | . | . | . | . | . | . | . | . | . | . | . | . |
| 21-225_34D7_LC | . | . | . | . | . | . | . | . | . | . | . | . | . | . |
| 21-225_35E1_LC | . | A | . | . | . | . | . | . | . | . | . | . | . | . |
| 21-225_3B12_LC | . | M | I | . | . | . | . | . | . | . | . | . | . | . |
| 21-225_3H10_LC | . | M | I | . | . | . | . | . | . | . | . | . | . | . |
| 21-225_45F6_LC | . | M | . | . | . | . | . | . | . | . | . | . | . | . |
| 21-225_4B3_LC | . | . | I | . | . | . | . | . | . | . | . | . | . | . |
| 21-225_50G9_LC | R | G | . | . | . | . | . | . | R | . | . | . | . | . |
| 21-225_52H1_LC | . | . | . | . | . | . | . | . | . | . | . | . | . | . |
| 21-225_53D10_LC | . | . | . | . | . | . | . | . | . | . | . | . | . | . |
| 21-225_57D9_LC | . | . | . | . | . | . | . | . | . | . | . | . | . | . |
| 21-225_57F1_LC | . | . | . | . | . | . | . | . | . | . | . | . | . | . |
| 21-225_57H11_LC | . | . | . | . | . | . | . | . | . | . | . | . | . | . |
| 21-225_58C1_LC | S | T | A | . | . | . | . | . | . | . | . | . | . | . |
| 21-225_58E7_LC | . | . | . | . | . | . | . | . | . | . | . | A | . | . |
| 21-225_58F7_LC | . | . | . | . | . | . | . | . | . | . | . | . | . | . |
| 21-225_58F8_LC | . | . | . | . | . | . | . | . | . | . | . | . | . | . |
| 21-225_58G11_LC | . | . | M | . | . | . | . | . | . | . | . | . | . | . |
| 21-225_59B11_LC | Y | . | . | . | . | . | . | . | . | . | . | . | . | . |
| 21-225_59F2_LC | . | . | . | . | . | . | . | . | . | . | . | . | . | . |
| 21-225_5G7_LC | . | . | . | . | . | . | . | . | . | . | . | . | . | . |
| 21-225_60A12_LC | S | T | A | . | . | . | . | . | . | . | . | . | . | . |
| 21-225_60B1_LC | . | . | . | . | . | . | . | . | . | . | . | . | . | . |
| 21-225_60D2_LC | . | . | . | . | . | . | . | . | . | . | . | . | . | . |
| 21-225_61F5_LC | . | . | . | . | . | . | . | . | . | . | . | . | . | . |
| 21-225_62A12_LC | . | A | . | . | . | . | . | . | . | . | . | . | . | R |

FIGURE 57

| Reference # | 136 | 137 | 138 | 139 | 140 | 141 | 142 | 143 | 144 | 145 | 146 | 147 | 148 | 149 |
|---|---|---|---|---|---|---|---|---|---|---|---|---|---|---|
| | | | | L_FR4 | | | | | | | | | | |
| CONSENSUS | T | V | V | F | G | G | G | T | K | L | T | V | L | G |
| 21-225_62B12_LC | . | A | . | . | . | . | . | . | . | . | . | . | . | R |
| 21-225_62D10_LC | . | A | . | . | . | . | . | . | . | . | . | . | . | R |
| 21-225_62E3_LC | . | A | . | . | . | . | . | . | . | . | . | . | . | R |
| 21-225_62E8_LC | . | A | . | . | . | . | . | . | . | . | . | . | . | R |
| 21-225_65E9_LC | . | . | . | . | . | . | . | . | . | . | . | . | . | . |
| 21-225_66G9_LC | . | A | . | . | . | . | . | . | . | . | . | . | . | R |
| 21-225_67F10_LC | . | . | . | . | . | . | . | . | R | . | . | . | . | . |
| 21-225_68D8_LC | . | . | . | . | . | . | . | . | . | . | . | . | . | . |
| 21-225_68G6_LC | . | A | . | . | . | . | . | . | . | . | . | . | . | . |
| 21-225_69B11_LC | . | . | . | . | . | . | . | . | . | . | . | . | . | . |
| 21-225_69B9_LC | . | . | . | . | . | . | . | . | . | . | . | . | . | . |
| 21-225_6B4_LC | . | . | I | . | . | . | . | . | . | . | . | . | . | . |
| 21-225_6D9_LC | S | T | . | . | . | . | . | . | . | . | . | . | . | . |
| 21-225_71D4_LC | . | . | . | . | . | . | . | . | . | . | . | . | . | . |
| 21-225_72B4_LC | . | . | . | . | . | . | . | . | . | . | . | . | . | . |
| 21-225_72D5_LC | . | A | . | . | . | . | . | . | . | . | . | . | . | R |
| 21-225_73C4_LC | . | A | . | . | . | . | . | . | . | . | . | . | . | R |
| 21-225_74A9_LC | P | . | I | . | . | . | . | . | . | . | . | . | . | . |
| 21-225_74B3_LC | . | . | . | . | . | . | . | . | . | . | . | . | . | . |
| 21-225_74C9_LC | P | . | I | . | . | . | . | . | . | . | . | . | . | . |
| 21-225_76B4_LC | S | T | . | . | . | . | . | . | . | . | . | . | . | . |
| 21-225_77A2_LC | . | A | . | . | . | . | . | . | . | . | . | . | . | . |
| 21-225_78E9_LC | P | . | I | . | . | . | . | . | . | . | . | . | . | . |
| 21-225_79E7_LC | P | . | I | . | . | . | . | . | . | . | . | . | . | . |
| 21-225_8D8_LC | . | A | . | . | . | . | . | . | . | . | . | . | . | . |
| 21-225_92A4_LC | P | . | I | . | . | . | . | . | . | . | . | . | . | S |
| 21-225_96B5_LC | S | T | . | . | . | . | . | . | . | . | . | . | . | . |
| 21-225_97E6_LC | P | . | I | . | . | . | . | . | . | . | . | . | . | . |
| 21-225_9H10_LC | Y | . | . | . | . | . | . | . | . | . | . | . | . | . |

**FIGURE 57**

Table 97. Consensus 17 - VK3|A27/JK1 (SEQ ID NO: 50314):

EIVLTQSPGTLSLSPGERATLSCRAS--QSVYS-----SYLAWYQQKPGQAPRLLIYG--------ASSRATGIPDRFSGSGSG--TDFTLTISRLEPEDFAVYYCQQYDN----------------------SPWTFGQGTKVEIKR wherein:

R at position 24 can be substituted with W

A at position 25 can be substituted with T

S at position 26 can be substituted with G or R

Q at position 29 can be substituted with P

S at position 30 can be substituted with N, I or R

V at position 31 can be substituted with I or F

Y at position 32 can be substituted with R, S, N, D, F, H, G or W

S at position33 can be substituted with T, N, G, L or R

S at position 39 can be substituted with N, G, R, D, A or Y

Y at position 40 can be substituted with F or H

A at position 42 can be substituted with V or S

G at position 58 can be substituted with D or V

A at position 67 can be substituted with T, P or V

S at position 68 can be substituted with F, Y, A or T

S at position 69 can be substituted with R, N or A

A at position 71 can be substituted with S or T

T at position 72 can be substituted with P, S or A

**FIGURE 57**

Q at position 107 can be substituted with H

Q at position 108 can be substituted with H

Y at position 109 can be substituted with S

D at position 110 can be substituted with E, G or H

N at position 111 can be substituted with S, null (-), R, T, I, D or G

null (-) at position 134 can be substituted with S

S at position 135 can be substituted with P, N or R

P at position 136 can be substituted with S or V

W at position 137 can be substituted with R

T at position 138 can be substituted with A

Xaa Xaa Xaa Xaa Xaa Xaa Xaa Xaa Xaa Xaa Leu Xaa (SEQ ID NO: 50127)

Xaa Xaa Xaa Xaa Arg Xaa Xaa (SEQ ID NO: 50128)

Xaa Xaa Xaa Xaa Xaa Xaa Xaa Xaa Xaa Xaa (SEQ ID NO: 50129)

Arg Ala Ser Gln Ser Val Tyr Ser Ser Tyr Leu Ala (SEQ ID NO: 50612)

Gly Ala Ser Ser Arg Ala Thr (SEQ ID NO: 50613)

Gln Gln Tyr Asp Asn Ser Pro Trp Thr (SEQ ID NO: 50614)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | E | I | V | L | T | Q | S | P | G | T | L | S | L | S | P | G | E | R | A | T | L | S | C | R | A | S |
| 21-225_147G8_LC | Q | . | . | . | . | . | . | . | . | . | . | . | . | . | . | . | . | . | . | . | . | . | . | . | . | . |
| 21-225_190A3_LC | . | . | M | . | . | . | . | . | . | . | . | . | . | . | . | . | . | . | . | . | . | . | . | . | . | . |
| 21-225_190A7_LC | . | . | . | . | . | . | . | . | . | . | . | . | . | . | . | . | . | . | . | . | . | . | . | . | . | . |
| 21-225_190B4_LC | . | . | . | . | . | . | . | . | . | . | . | . | . | . | . | . | . | . | . | . | . | . | . | . | . | . |
| 21-225_190G10_LC | . | . | . | . | . | . | . | . | . | . | . | . | . | . | . | . | . | . | . | . | . | . | . | . | . | . |
| 21-225_191A3_LC | . | . | . | . | . | . | . | . | . | . | . | . | . | . | . | . | . | . | . | . | . | . | . | . | . | . |
| 21-225_191A5_LC | . | . | . | . | . | . | . | . | . | . | . | . | . | . | . | . | . | . | . | . | . | . | . | . | . | . |
| 21-225_191C3_LC | . | . | . | . | . | . | . | . | . | . | . | . | . | . | . | . | . | . | . | . | . | . | . | . | . | . |
| 21-225_191D3_LC | . | . | . | . | . | . | . | . | . | . | . | . | . | . | . | . | . | S | . | . | . | . | . | . | . | G |
| 21-225_191F1_LC | . | . | . | . | . | . | . | . | . | . | . | . | . | . | . | . | . | . | . | A | . | . | . | . | . | . |
| 21-225_191H7_LC | . | . | . | . | . | . | . | . | . | . | . | . | . | . | . | . | . | S | . | . | . | . | . | . | . | G |
| 21-225_192B3_LC | . | . | . | . | . | . | . | . | . | . | . | . | . | . | . | . | . | . | . | . | . | . | . | . | . | . |
| 21-225_192F8_LC | . | . | . | . | . | . | . | . | . | . | . | . | . | . | . | . | . | . | . | . | . | . | . | . | . | . |
| 21-225_192G12_LC | . | . | . | . | . | . | . | . | . | . | . | . | . | . | . | . | . | . | . | . | . | . | . | . | . | . |
| 21-225_192H3_LC | . | . | . | . | . | . | . | . | . | . | . | . | . | . | . | . | . | . | . | . | . | . | . | . | . | . |
| 21-225_193A1_LC | . | . | . | . | . | . | . | . | . | . | . | . | . | . | . | . | . | . | . | . | . | . | . | . | . | . |
| 21-225_193B1_LC | . | . | . | . | . | . | . | . | . | . | . | . | . | . | . | . | D | . | . | . | . | . | . | . | . | . |
| 21-225_193B10_LC | . | . | . | . | . | . | . | . | . | . | . | . | . | . | . | . | . | . | . | . | . | . | . | . | . | . |
| 21-225_193B12_LC | . | . | . | . | . | . | . | . | . | . | . | . | . | . | . | . | . | . | . | . | . | . | . | . | . | . |
| 21-225_193C8_LC | . | . | . | . | . | . | . | . | . | . | . | F | . | . | . | . | . | . | . | . | . | . | . | . | . | . |
| 21-225_193D3_LC | . | . | . | . | . | . | . | . | . | . | . | . | . | . | . | . | . | . | . | . | . | . | . | . | . | . |
| 21-225_193D8_LC | . | . | . | . | K | . | . | . | . | . | . | F | . | . | . | . | . | . | . | . | . | . | . | . | . | . |
| 21-225_193E6_LC | . | . | . | . | A | . | . | . | . | . | . | . | . | . | . | . | . | . | . | . | . | . | . | . | . | . |
| 21-225_193F3_LC | . | . | . | . | . | . | . | . | . | . | . | . | . | . | . | . | . | S | . | . | . | . | . | . | . | G |
| 21-225_193G8_LC | . | . | . | . | . | . | . | . | . | . | . | . | . | . | . | . | . | . | . | . | . | . | . | . | . | . |
| 21-225_193H6_LC | . | . | . | . | . | . | . | . | . | . | . | . | . | . | . | . | . | S | . | . | . | . | . | . | . | G |
| 21-225_194C10_LC | . | . | . | . | . | . | . | . | . | . | . | . | . | . | . | . | . | . | . | . | . | . | . | . | . | . |
| 21-225_194E5_LC | . | . | . | . | . | . | . | . | . | . | . | . | . | . | . | . | . | S | . | . | . | . | . | . | . | G |
| 21-225_194E6_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | E | I | V | L | T | Q | S | P | G | T | L | S | L | S | P | G | E | R | A | T | L | S | C | R | A | S |
| 21-225_195B1_LC | . | . | . | . | . | . | . | . | . | . | . | . | . | . | S | . | . | . | . | . | . | . | . | . | . | . |
| 21-225_195C8_LC | . | . | . | . | . | . | . | . | . | . | . | . | . | . | . | . | . | . | . | . | . | . | . | . | . | . |
| 21-225_196G10_LC | . | . | . | . | . | . | . | . | . | . | . | L | . | . | . | . | . | . | . | . | . | . | . | . | . | . |
| 21-225_196H12_LC | . | . | . | . | . | . | . | . | . | . | . | F | . | . | . | . | . | . | . | . | . | . | . | . | . | . |
| 21-225_197F4_LC | . | . | . | . | . | . | . | . | . | . | . | . | . | . | . | . | . | G | . | . | . | . | . | . | . | . |
| 21-225_198B1_LC | . | . | . | . | . | . | . | . | . | . | . | . | . | . | . | . | . | S | . | . | . | . | . | . | . | G |
| 21-225_198C2_LC | . | . | . | V | . | . | . | . | . | . | . | L | . | Y | S | E | . | . | S | . | . | . | . | . | . | . |
| 21-225_198E1_LC | . | . | . | . | . | . | . | . | . | . | . | . | . | . | . | . | . | . | . | . | . | . | . | . | . | . |
| 21-225_198G10_LC | . | . | . | . | . | . | . | . | . | . | . | . | . | . | . | . | . | . | . | . | . | . | . | . | . | . |
| 21-225_199C2_LC | . | . | . | . | . | . | . | . | . | . | . | . | . | . | . | . | . | . | . | . | . | . | . | . | . | . |
| 21-225_199C7_LC | . | . | . | . | . | . | . | . | . | . | . | . | . | . | . | . | . | . | . | . | . | . | . | . | . | . |
| 21-225_199G11_LC | . | . | . | V | . | . | . | . | . | . | . | . | . | . | . | . | . | . | . | . | . | . | . | . | . | . |
| 21-225_200A1_LC | . | . | . | . | . | . | . | . | . | I | . | . | . | Y | . | . | . | . | . | . | . | . | . | . | . | . |
| 21-225_200F10_LC | . | . | . | . | . | . | . | . | . | . | . | . | . | . | . | . | . | . | . | . | . | . | . | . | . | . |
| 21-225_201B2_LC | . | . | . | . | . | . | . | . | . | . | . | . | . | . | . | . | . | . | . | . | . | . | . | . | . | . |
| 21-225_201E1_LC | . | . | . | . | . | . | . | . | D | . | . | . | . | . | . | . | . | . | . | . | . | . | . | . | . | . |
| 21-225_202D11_LC | . | . | . | . | . | . | . | . | . | . | . | . | . | . | . | . | . | . | . | . | . | . | . | . | . | . |
| 21-225_202D9_LC | . | . | . | . | . | . | . | . | D | . | . | . | . | . | . | . | . | . | . | . | . | . | . | . | . | . |
| 21-225_205G7_LC | . | . | . | . | . | . | . | . | . | . | . | . | . | . | . | . | . | . | . | . | . | . | . | . | . | . |
| 21-225_208A9_LC | . | . | . | . | . | . | . | . | . | . | . | . | . | . | . | . | . | . | . | . | . | . | . | . | . | . |
| 21-225_208B5_LC | . | . | . | . | . | . | . | . | . | . | . | . | . | . | . | . | . | . | . | . | . | . | . | . | . | . |
| 21-225_220E1_LC | . | . | . | . | . | . | . | . | . | . | . | . | . | . | . | . | . | . | . | . | . | . | . | . | . | . |
| 21-225_222C7_LC | . | . | . | . | . | . | . | . | . | . | . | . | . | . | . | . | . | . | . | . | . | . | . | . | . | . |
| 21-225_225E7_LC | . | . | . | . | . | . | . | . | . | . | . | . | . | . | . | . | . | . | . | . | . | . | . | . | . | . |
| 21-225_23B9_LC | . | . | . | . | . | . | . | . | . | . | . | . | . | . | . | . | . | . | . | . | . | . | . | . | . | . |
| 21-225_44F6_LC | . | . | . | . | . | . | . | . | . | . | . | . | . | . | . | . | . | . | . | . | . | . | . | . | . | . |
| 21-225_59B2_LC | . | . | . | . | . | . | . | . | . | . | . | . | . | . | . | . | . | . | . | . | . | . | . | W | . | . |
| 21-225_74A3_LC | . | . | . | . | . | . | . | . | . | . | . | . | . | . | . | . | . | . | . | . | . | . | . | . | . | . |
| 21-225_74A4_LC | . | . | . | . | . | . | . | . | . | . | . | Y | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | E | I | V | L | T | Q | S | P | G | T | L | S | L | S | P | G | E | R | A | T | L | S | C | R | A | S |
| 21-225_74B12_LC | . | . | . | . | . | . | . | . | . | . | . | Y | . | . | . | . | . | . | . | . | . | . | . | . | . | . |
| 21-225_74B2_LC | . | L | . | . | . | . | . | . | . | . | . | . | . | . | . | . | . | . | . | . | . | . | . | . | . | . |
| 21-225_74C5_LC | . | . | . | . | . | . | . | . | . | . | . | . | . | . | . | . | . | . | . | . | . | . | . | . | . | . |
| 21-225_74C7_LC | . | . | . | . | . | . | . | . | . | . | . | . | . | . | . | . | . | . | . | . | . | . | . | . | T | . |
| 21-225_74D11_LC | . | . | . | . | . | . | . | . | . | . | . | . | . | . | . | . | . | . | . | . | . | . | . | . | . | . |
| 21-225_74D2_LC | . | . | . | . | . | . | . | . | . | . | . | . | . | . | . | . | . | . | . | . | . | . | . | . | . | . |
| 21-225_74D5_LC | . | . | . | . | . | . | . | . | . | . | . | . | . | . | . | . | . | . | . | . | . | . | . | . | . | . |
| 21-225_74D6_LC | . | . | . | . | . | . | . | . | . | . | . | . | . | . | . | . | . | . | . | . | . | . | . | . | . | . |
| 21-225_74F4_LC | . | . | . | . | . | . | . | . | . | . | . | . | . | . | . | . | . | . | . | . | . | . | . | . | . | . |
| 21-225_74F5_LC | . | . | . | . | . | . | . | . | . | . | . | Y | . | . | . | . | . | . | . | . | . | . | . | . | . | . |
| 21-225_74G8_LC | . | . | . | . | . | . | . | . | . | . | . | . | . | . | . | . | . | . | . | . | . | . | . | . | . | . |
| 21-225_74H5_LC | . | . | . | . | . | . | . | . | . | . | . | . | . | . | . | . | . | . | . | . | . | . | . | . | . | . |
| 21-225_74H7_LC | . | L | . | . | . | . | . | . | . | . | . | . | . | . | . | . | . | . | . | . | . | . | . | . | . | . |
| 21-225_74H8_LC | . | . | . | . | . | . | . | . | . | . | . | . | . | . | . | . | . | . | . | . | . | . | . | . | . | . |
| 21-225_75A12_LC | . | . | . | . | . | . | . | . | . | . | . | . | . | . | . | . | . | . | . | . | . | . | . | . | . | . |
| 21-225_75A5_LC | . | . | . | . | . | . | . | . | . | . | . | Y | . | . | . | . | . | . | . | . | . | . | . | . | . | . |
| 21-225_75A8_LC | . | . | . | . | . | . | . | . | . | . | . | Y | . | . | . | . | . | . | . | . | . | . | . | . | . | . |
| 21-225_75B10_LC | . | . | . | . | . | . | . | . | . | . | . | . | . | . | . | . | . | . | . | . | . | . | . | . | . | . |
| 21-225_75C2_LC | . | . | . | . | . | . | . | . | . | . | R | Y | . | . | . | . | . | . | . | . | . | . | . | . | . | . |
| 21-225_75C3_LC | . | . | . | . | . | . | . | . | . | . | . | . | . | . | Q | . | . | . | . | . | . | . | . | . | . | . |
| 21-225_75D3_LC | . | . | . | . | . | . | . | . | . | . | . | Y | . | . | . | . | . | . | . | . | . | . | . | . | . | . |
| 21-225_75D9_LC | . | . | . | . | . | . | . | . | . | . | . | . | . | . | . | . | . | . | . | . | . | . | . | . | . | . |
| 21-225_75F3_LC | . | . | . | . | . | . | . | . | . | . | . | Y | . | . | . | . | . | . | . | . | . | . | . | . | . | . |
| 21-225_75G12_LC | . | . | . | . | . | . | . | . | . | . | . | Y | . | . | . | . | . | . | . | . | . | . | . | . | . | . |
| 21-225_75G3_LC | . | . | . | . | . | . | . | . | . | . | . | . | . | . | . | . | . | . | . | . | . | . | . | . | . | R |
| 21-225_75G7_LC | . | . | . | . | . | . | . | . | . | . | . | . | . | . | . | . | . | . | . | . | . | . | . | . | . | R |
| 21-225_75G9_LC | . | . | A | . | . | . | . | . | . | . | . | . | . | . | . | . | . | . | . | . | . | . | . | . | . | . |
| 21-225_75H8_LC | . | P | . | . | . | . | . | . | . | . | . | . | . | . | . | . | . | . | . | . | . | . | . | . | . | . |
| 21-225_76A4_LC | . | . | . | . | . | . | . | . | . | . | . | Y | . | . | . | . | . | . | . | . | . | . | . | . | . | . |

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | E | I | V | L | T | Q | S | P | G | T | L | S | L | S | P | G | E | R | A | T | L | S | C | R | A | S |
| 21-225_76A7_LC | . | . | A | . | . | . | . | . | . | . | . | . | . | . | . | . | . | . | . | . | . | . | . | . | . | . |
| 21-225_76C9_LC | . | . | . | . | . | . | . | . | . | . | . | . | . | . | . | . | . | . | . | . | . | . | . | . | . | . |
| 21-225_76D1_LC | . | . | . | . | . | . | . | . | . | . | . | . | . | . | . | . | . | . | . | . | . | . | . | . | . | . |
| 21-225_76E10_LC | . | . | . | . | . | . | . | . | . | . | . | . | . | . | . | . | . | . | . | . | . | . | . | . | . | . |
| 21-225_76G4_LC | . | . | . | . | . | . | . | . | . | . | . | . | . | . | . | . | . | . | . | . | . | . | . | . | . | . |
| 21-225_77A10_LC | . | . | . | . | . | . | . | . | . | . | . | . | . | . | . | . | . | . | . | . | . | . | . | . | . | . |
| 21-225_77C12_LC | . | . | . | . | . | . | . | . | . | . | . | . | . | . | . | . | . | . | . | . | . | . | . | . | . | R |
| 21-225_77C5_LC | . | . | . | . | . | . | . | . | . | . | . | . | . | . | . | . | . | . | . | . | . | . | . | . | . | . |
| 21-225_77F7_LC | . | . | . | . | . | . | . | . | . | . | . | Y | . | . | . | . | . | . | . | . | . | . | . | . | . | . |
| 21-225_77G1_LC | . | . | . | . | . | . | . | . | . | . | . | Y | . | . | . | . | . | . | . | . | . | . | . | . | . | . |
| 21-225_78E7_LC | . | . | . | . | . | . | . | . | . | . | . | . | . | . | . | . | . | . | . | . | . | . | . | . | . | . |
| 21-225_79F11_LC | . | . | . | . | . | . | . | . | . | . | . | . | . | . | . | . | . | . | . | . | . | . | . | . | . | . |
| 21-225_79F3_LC | . | . | . | . | . | . | . | . | . | . | . | Y | . | . | . | . | . | . | . | . | . | . | . | . | . | . |
| 21-225_79G1_LC | . | . | . | . | . | . | . | . | . | . | . | Y | . | . | S | . | . | . | . | . | . | . | . | . | . | . |
| 21-225_79G12_LC | . | . | . | . | . | . | . | . | . | . | . | Y | . | . | . | . | . | . | . | . | . | . | . | . | . | . |
| 21-225_80A1_LC | . | . | . | . | . | . | . | . | . | . | R | Y | . | . | . | . | . | . | . | . | . | . | . | . | . | . |
| 21-225_80A6_LC | . | . | . | . | . | . | . | . | . | . | . | . | . | . | . | . | . | I | P | . | . | . | . | . | . | . |
| 21-225_80B10_LC | . | . | . | . | . | . | . | . | . | . | . | . | . | . | . | . | . | . | . | . | . | . | . | . | . | . |
| 21-225_80C1_LC | . | . | . | . | . | . | . | . | . | . | . | Y | . | . | . | . | . | . | . | . | . | . | . | . | . | . |
| 21-225_80D5_LC | . | L | . | . | . | . | . | . | . | . | . | . | . | . | . | . | K | . | V | . | . | . | . | . | . | . |
| 21-225_80E12_LC | . | . | . | . | . | . | C | . | . | . | . | . | . | . | . | . | . | . | . | . | V | . | . | . | . | . |
| 21-225_80E3_LC | . | . | . | . | . | . | . | . | . | . | . | Y | . | . | S | . | . | . | . | . | . | . | . | . | . | . |
| 21-225_80H12_LC | . | . | . | . | . | . | . | . | . | . | . | . | . | . | . | . | . | . | . | . | . | . | . | . | . | . |
| 21-225_80H7_LC | . | . | . | . | . | . | . | . | . | . | . | . | . | . | . | . | . | . | . | . | . | . | . | . | . | . |
| 21-225_81C11_LC | . | . | . | . | . | . | . | . | . | . | R | Y | . | . | . | V | . | . | . | . | . | . | . | . | . | . |
| 21-225_81C5_LC | . | . | . | . | . | . | . | . | . | . | . | Y | . | . | S | . | . | . | . | . | . | . | . | . | . | . |
| 21-225_83B6_LC | . | . | . | . | . | . | . | . | . | . | . | . | . | . | . | . | . | . | . | . | . | . | . | . | . | . |
| 21-225_83B7_LC | . | . | . | . | . | . | . | . | . | . | R | Y | . | . | S | V | . | . | . | . | . | . | . | . | . | . |
| 21-225_83C10_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | K_FR1 | | | | | | | | | | | | | | | |
| CONSENSUS | E | I | V | L | T | Q | S | P | G | T | L | S | L | S | P | G | E | R | A | T | L | S | C | R | A | S |
| 21-225_83G1_LC | . | . | . | . | . | . | . | . | . | . | R | Y | . | . | . | . | . | . | . | . | . | . | . | . | . | . |
| 21-225_84E12_LC | . | . | . | . | . | . | . | . | . | . | . | . | . | . | . | . | . | . | . | . | . | . | . | . | . | . |
| 21-225_85A3_LC | . | . | . | . | . | . | . | . | . | . | . | F | . | . | . | . | . | . | . | . | . | . | . | . | . | . |
| 21-225_85B4_LC | . | . | . | . | . | . | . | . | . | . | . | F | . | . | . | . | . | . | . | . | . | . | . | . | . | . |
| 21-225_85B9_LC | . | . | . | . | . | . | . | . | . | . | . | F | . | . | . | . | . | . | . | . | . | . | . | . | . | . |
| 21-225_85D6_LC | . | . | . | . | . | . | . | . | . | . | . | F | . | . | Q | . | . | . | . | . | . | . | . | . | . | . |
| 21-225_85G10_LC | . | . | . | . | . | . | . | . | . | S | . | . | . | . | . | . | . | . | . | . | . | . | . | . | . | . |
| 21-225_85G6_LC | . | . | A | . | . | . | . | . | . | . | . | . | . | . | . | . | . | . | . | . | . | . | . | . | . | . |
| 21-225_86C1_LC | . | . | . | . | . | . | . | . | . | . | . | Y | . | . | . | . | . | . | . | . | . | . | . | . | . | . |
| 21-225_86C11_LC | . | . | . | . | . | . | . | . | . | . | . | Y | . | . | . | . | . | . | . | . | . | . | . | . | . | . |
| 21-225_86E4_LC | . | . | . | . | . | . | . | . | . | . | R | Y | . | . | . | . | . | . | . | . | . | . | . | . | . | . |
| 21-225_87A12_LC | . | . | . | . | . | . | . | . | . | . | R | Y | . | . | . | . | . | . | . | . | . | . | . | . | . | . |
| 21-225_87C9_LC | . | . | . | . | . | . | . | . | . | . | . | Y | . | . | . | V | . | . | . | . | . | . | . | . | . | . |
| 21-225_87E5_LC | . | F | . | . | . | . | . | . | . | . | . | Y | . | . | . | . | . | . | . | . | . | . | . | . | . | . |
| 21-225_87H1_LC | . | . | . | . | . | . | . | . | . | . | . | . | . | . | . | . | . | . | . | . | . | . | . | . | . | . |
| 21-225_88E7_LC | . | . | . | . | . | . | . | . | . | . | . | . | . | . | . | . | . | . | . | . | . | . | . | . | . | . |
| 21-225_88F7_LC | . | . | . | . | . | . | . | . | . | . | . | . | . | . | . | . | . | . | . | . | . | . | . | . | . | . |
| 21-225_88G9_LC | . | . | . | . | . | . | . | . | . | . | R | Y | . | . | . | . | . | . | . | . | . | . | . | . | . | . |
| 21-225_88H1_LC | . | . | . | . | . | . | . | . | . | . | . | Y | . | . | . | . | . | . | . | . | . | . | . | . | . | . |
| 21-225_89A11_LC | . | . | . | . | . | . | . | . | . | . | . | . | . | . | . | . | . | . | . | . | . | . | . | . | . | . |
| 21-225_89D5_LC | . | . | . | . | . | . | . | . | . | . | . | Y | . | . | S | . | . | . | . | . | . | . | . | . | . | . |
| 21-225_89E10_LC | . | . | . | . | . | . | . | . | . | . | . | Y | . | . | S | . | . | . | . | . | . | . | . | . | . | . |
| 21-225_89H5_LC | . | . | . | . | . | . | . | . | . | . | . | . | . | . | . | . | . | . | . | . | . | . | . | . | . | . |
| 21-225_90A5_LC | . | . | . | . | . | . | . | . | . | . | . | Y | . | . | . | . | . | . | . | . | . | . | . | . | . | . |
| 21-225_90F10_LC | . | . | . | . | . | . | . | . | . | . | . | Y | . | . | . | . | . | . | . | . | . | . | . | . | . | . |
| 21-225_90G4_LC | . | . | . | . | . | . | . | . | . | . | . | Y | . | . | . | . | . | . | . | . | . | . | . | . | . | . |
| 21-225_90G5_LC | . | . | . | . | . | . | . | . | . | . | . | Y | . | . | . | . | . | . | . | . | . | . | . | . | . | . |
| 21-225_91B2_LC | . | . | . | . | . | . | . | . | . | . | . | Y | . | . | . | . | . | . | . | . | . | . | . | . | . | . |
| 21-225_91B3_LC | . | . | . | . | . | . | . | . | . | . | . | Y | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | E | I | V | L | T | Q | S | P | G | T | L | S | L | S | P | G | E | R | A | T | L | S | C | R | A | S |
| 21-225_91B4_LC | . | . | . | . | . | . | . | . | . | . | . | Y | . | . | . | . | . | . | . | . | . | . | . | . | . | . |
| 21-225_91E9_LC | . | . | . | . | . | . | . | . | . | . | . | Y | . | . | . | . | . | . | . | . | . | . | . | . | . | . |
| 21-225_91F3_LC | . | . | . | . | . | . | . | . | . | . | . | Y | . | . | . | . | . | . | . | . | . | . | . | . | . | . |
| 21-225_92B1_LC | . | . | . | . | . | . | . | . | . | . | . | . | . | . | . | . | . | . | . | . | . | . | . | . | . | . |
| 21-225_92D6_LC | . | . | . | . | . | . | . | . | . | . | . | Y | . | . | . | . | . | . | . | . | . | . | . | . | . | . |
| 21-225_93C2_LC | . | . | . | . | . | . | . | . | . | . | . | Y | . | . | . | . | . | . | . | . | . | . | . | . | . | . |
| 21-225_93E4_LC | . | . | . | . | . | . | . | . | . | . | . | Y | . | . | . | . | . | . | . | . | . | . | . | . | . | . |
| 21-225_93E9_LC | . | . | . | . | . | . | . | . | . | . | . | . | . | . | . | . | . | . | . | . | . | . | . | . | . | . |
| 21-225_94D3_LC | . | . | . | . | . | . | . | . | . | . | . | . | . | . | Q | . | . | . | . | . | . | . | . | . | . | . |
| 21-225_94F12_LC | . | . | . | . | . | . | . | . | . | . | . | Y | . | . | . | . | . | . | . | . | F | . | . | . | . | . |
| 21-225_94G10_LC | . | . | . | . | . | . | . | . | . | . | . | . | . | . | . | . | . | . | . | . | . | . | . | . | . | . |
| 21-225_95D2_LC | . | . | . | . | . | . | . | . | . | . | . | . | . | . | . | . | . | . | . | . | . | . | . | . | . | . |
| 21-225_95F9_LC | . | . | . | . | . | . | . | . | . | . | . | Y | . | . | . | . | . | . | . | . | . | . | . | . | . | . |
| 21-225_95G9_LC | . | . | . | . | . | . | . | . | . | . | . | Y | . | . | S | . | . | . | . | . | . | . | . | . | . | . |
| 21-225_95H10_LC | . | . | . | . | S | . | . | . | . | I | . | Y | . | . | S | . | . | . | . | . | . | . | . | . | . | . |
| 21-225_97A2_LC | . | . | . | . | . | . | . | . | . | . | . | Y | . | . | S | . | . | . | . | . | . | . | . | . | . | . |
| 21-225_97E5_LC | . | . | . | . | . | . | . | . | . | . | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | V | Y | S | - | - | - | - | - | S | Y | L | A | W | Y | Q | Q | K | P | G | Q | A | P |
| 21-225_147G8_LC | . | . | . | R | I | F | . | . | . | . | . | . | N | . | . | . | . | . | . | . | . | . | . | . | . | . |
| 21-225_190A3_LC | . | . | . | N | I | R | . | . | . | . | . | . | N | F | . | . | . | . | . | . | . | . | . | . | . | . |
| 21-225_190A7_LC | . | . | . | . | . | R | . | . | . | . | . | . | N | F | . | . | . | H | . | . | . | . | . | . | . | . |
| 21-225_190B4_LC | . | . | . | . | I | R | . | . | . | . | . | . | . | F | . | . | . | . | . | . | . | . | . | . | . | . |
| 21-225_190G10_LC | . | . | P | . | . | S | . | . | . | . | . | . | R | F | . | . | . | . | . | . | . | . | . | . | . | . |
| 21-225_191A3_LC | . | . | . | . | I | R | . | . | . | . | . | . | . | F | . | . | . | . | . | . | . | . | . | . | . | . |
| 21-225_191A5_LC | . | . | . | . | . | S | . | . | . | . | . | . | R | F | . | . | . | . | . | . | . | . | . | . | . | . |
| 21-225_191C3_LC | . | . | . | . | I | R | . | . | . | . | . | . | . | F | . | . | . | . | . | . | . | . | . | . | . | . |
| 21-225_191D3_LC | . | . | . | . | I | R | T | . | . | . | . | . | N | F | . | . | . | . | . | . | Q | . | . | . | . | . |
| 21-225_191F1_LC | . | . | . | . | I | S | L | . | . | . | . | . | N | F | . | . | . | . | . | . | . | . | . | . | . | . |
| 21-225_191H7_LC | . | . | . | . | I | R | T | . | . | . | . | . | D | F | . | . | . | . | . | . | Q | . | . | . | . | . |
| 21-225_192B3_LC | . | . | . | . | . | R | . | . | . | . | . | . | . | F | . | . | . | . | . | . | . | . | . | . | . | . |
| 21-225_192F8_LC | . | . | . | . | I | S | . | . | . | . | . | . | . | . | . | . | . | . | . | . | . | . | . | . | . | . |
| 21-225_192G12_LC | . | . | . | . | . | R | . | . | . | . | . | . | D | F | . | . | . | L | . | . | . | . | . | . | . | . |
| 21-225_192H3_LC | . | . | . | . | . | S | . | . | . | . | . | . | N | F | . | . | . | . | . | . | . | . | . | . | . | . |
| 21-225_193A1_LC | . | . | . | . | . | R | . | . | . | . | . | . | N | F | . | . | . | H | . | . | . | . | . | . | . | . |
| 21-225_193B1_LC | . | . | . | . | . | R | . | . | . | . | . | . | N | F | . | . | . | H | . | . | . | . | . | . | . | . |
| 21-225_193B10_LC | . | . | P | . | . | S | . | . | . | . | . | . | . | . | . | . | . | . | . | . | . | . | . | . | . | . |
| 21-225_193B12_LC | . | . | . | . | I | R | . | . | . | . | . | . | . | F | . | . | . | . | . | . | . | . | . | . | . | . |
| 21-225_193C8_LC | . | . | . | . | I | R | . | . | . | . | . | . | . | F | . | . | . | . | . | . | . | . | . | . | . | . |
| 21-225_193D3_LC | . | . | . | . | I | R | . | . | . | . | . | . | . | F | . | . | . | . | . | . | . | . | . | . | . | . |
| 21-225_193D8_LC | . | . | . | . | I | R | T | . | . | . | . | . | N | F | . | . | . | . | . | . | . | . | . | . | . | . |
| 21-225_193E6_LC | . | . | . | . | . | R | . | . | . | . | . | . | G | . | . | . | . | . | . | . | . | . | . | . | . | . |
| 21-225_193F3_LC | . | . | . | . | I | R | T | . | . | . | . | . | D | F | . | V | . | . | . | . | Q | . | . | . | . | . |
| 21-225_193G8_LC | . | . | . | . | . | R | T | . | . | . | . | . | N | F | . | . | . | H | . | . | . | . | . | . | . | . |
| 21-225_193H6_LC | . | . | . | . | I | R | T | . | . | . | . | . | N | F | . | . | . | . | . | . | Q | . | . | . | . | . |
| 21-225_194C10_LC | . | . | P | . | . | N | . | . | . | . | . | . | G | . | . | . | . | . | . | . | . | . | . | . | T | . |
| 21-225_194E5_LC | . | . | . | . | I | R | T | . | . | . | . | . | N | F | . | . | . | . | . | . | . | . | . | . | . | . |
| 21-225_194E6_LC | . | . | . | . | I | R | . | . | . | . | . | . | N | F | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | V | Y | S | - | - | - | - | - | S | Y | L | A | W | Y | Q | Q | K | P | G | Q | A | P |
| 21-225_195B1_LC | . | . | P | . | . | N | . | . | . | . | . | . | N | . | . | . | . | . | . | . | . | . | . | . | T | . |
| 21-225_195C8_LC | . | . | . | . | I | R | . | . | . | . | . | . | . | F | . | . | . | . | . | . | . | . | . | . | . | . |
| 21-225_196G10_LC | . | . | P | . | . | S | N | . | . | . | . | . | . | F | . | . | . | . | . | . | . | . | . | . | . | . |
| 21-225_196H12_LC | . | . | . | . | . | R | . | . | . | . | . | . | N | F | . | . | . | H | . | . | . | . | . | . | . | . |
| 21-225_197F4_LC | . | . | . | . | I | R | . | . | . | . | . | . | . | F | . | . | . | . | L | . | . | . | . | . | . | . |
| 21-225_198B1_LC | . | . | . | . | I | R | T | . | . | . | . | . | N | F | . | . | . | . | . | . | Q | . | . | . | . | . |
| 21-225_198C2_LC | . | . | . | . | . | R | . | . | . | . | . | . | . | . | . | . | . | . | . | . | . | . | . | . | . | . |
| 21-225_198E1_LC | . | . | . | . | I | R | . | . | . | . | . | . | N | F | . | . | . | . | . | . | . | . | . | . | . | . |
| 21-225_198G10_LC | . | . | . | . | I | R | . | . | . | . | . | . | . | F | . | . | . | . | . | . | . | . | . | . | . | . |
| 21-225_199C2_LC | . | . | . | . | I | R | . | . | . | . | . | . | . | F | . | . | . | . | . | . | . | . | . | . | . | . |
| 21-225_199C7_LC | . | . | . | . | I | R | T | . | . | . | . | . | N | F | . | . | . | . | . | . | . | . | . | . | . | . |
| 21-225_199G11_LC | . | . | . | . | . | R | . | . | . | . | . | . | . | . | . | . | . | . | . | . | . | . | . | . | . | . |
| 21-225_200A1_LC | . | . | . | . | . | N | . | . | . | . | . | . | N | . | . | . | . | . | . | . | R | . | . | . | . | . |
| 21-225_200F10_LC | . | . | . | N | I | R | . | . | . | . | . | . | . | F | . | . | . | . | . | . | . | . | . | . | . | . |
| 21-225_201B2_LC | . | . | . | . | . | S | . | . | . | . | . | . | N | . | . | . | . | . | . | . | R | . | . | . | . | . |
| 21-225_201E1_LC | . | . | P | . | I | N | . | . | . | . | . | . | G | F | . | . | . | . | . | . | . | . | . | . | . | . |
| 21-225_202D11_LC | . | . | . | . | I | N | . | . | . | . | . | . | N | . | . | . | . | . | . | R | . | . | . | . | . | . |
| 21-225_202D9_LC | . | . | . | . | . | N | . | . | . | . | . | . | G | . | . | . | . | . | . | . | . | . | . | . | S | . |
| 21-225_205G7_LC | . | . | . | . | . | F | . | . | . | . | . | . | N | . | . | . | . | . | . | . | . | . | . | . | . | . |
| 21-225_208A9_LC | . | . | . | . | . | S | N | . | . | . | . | . | N | . | . | . | . | . | . | . | . | . | . | . | . | . |
| 21-225_208B5_LC | . | . | . | . | . | S | . | . | . | . | . | . | N | . | . | . | . | . | . | . | R | . | . | . | . | . |
| 21-225_220E1_LC | . | . | . | . | I | S | R | . | . | . | . | . | . | H | . | V | . | . | . | . | . | . | N | . | . | . |
| 21-225_222C7_LC | . | . | . | . | . | . | . | . | . | . | . | . | N | . | . | . | . | . | . | . | . | . | . | . | . | . |
| 21-225_225E7_LC | . | . | . | . | . | . | . | . | . | . | . | . | . | . | . | . | . | . | . | . | . | . | . | . | . | . |
| 21-225_23B9_LC | . | . | . | N | . | . | . | . | . | . | . | . | . | . | . | . | . | . | . | . | . | . | . | . | T | . |
| 21-225_44F6_LC | . | . | . | . | . | S | G | . | . | . | . | . | . | . | . | . | . | . | . | . | . | . | . | . | . | . |
| 21-225_59B2_LC | . | . | . | . | . | . | . | . | . | . | . | . | . | F | . | . | . | F | . | . | . | S | . | . | . | . |
| 21-225_74A3_LC | . | . | . | . | . | . | . | . | . | . | . | . | . | . | . | . | . | . | . | . | . | . | . | . | . | . |
| 21-225_74A4_LC | . | . | . | . | . | . | . | . | . | . | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | V | Y | S | - | - | - | - | - | S | Y | L | A | W | Y | Q | Q | K | P | G | Q | A | P |
| 21-225_74B12_LC | . | . | . | . | . | . | . | . | . | . | . | . | . | . | . | . | . | . | . | . | . | . | . | . | . | . |
| 21-225_74B2_LC | . | . | . | N | I | . | . | . | . | . | . | . | . | . | . | . | . | . | . | . | . | . | . | . | . | . |
| 21-225_74C5_LC | . | . | . | . | . | N | . | . | . | . | . | . | N | . | . | . | . | . | . | . | . | . | . | . | . | . |
| 21-225_74C7_LC | . | . | P | N | . | D | . | . | . | . | . | . | . | . | . | . | . | . | . | . | . | . | . | . | . | . |
| 21-225_74D11_LC | . | . | . | . | . | . | . | . | . | . | . | . | . | . | . | . | . | . | . | . | . | . | . | . | . | . |
| 21-225_74D2_LC | . | . | . | . | . | D | . | . | . | . | . | . | N | . | . | . | . | . | . | . | . | . | . | . | . | . |
| 21-225_74D5_LC | . | . | . | . | . | N | . | . | . | . | . | . | N | . | . | . | . | . | R | . | . | . | . | . | . | . |
| 21-225_74D6_LC | . | . | . | . | . | . | . | . | . | . | . | . | . | . | . | . | . | . | . | . | . | . | . | . | . | . |
| 21-225_74F4_LC | . | . | . | I | F | S | . | . | . | . | . | . | . | . | . | . | . | . | . | . | . | . | . | . | S | . |
| 21-225_74F5_LC | . | . | . | . | . | . | . | . | . | . | . | . | . | . | . | . | . | . | . | . | . | . | . | . | . | . |
| 21-225_74G8_LC | . | . | . | . | F | S | . | . | . | . | . | . | A | . | . | . | . | . | . | . | . | . | . | . | . | . |
| 21-225_74H5_LC | . | . | . | . | . | N | . | . | . | . | . | . | N | . | . | . | . | . | R | . | . | . | . | . | . | . |
| 21-225_74H7_LC | . | . | . | N | I | . | . | . | . | . | . | . | . | . | . | . | . | . | . | . | . | . | . | . | . | . |
| 21-225_74H8_LC | . | . | . | . | F | S | . | . | . | . | . | . | . | . | . | . | . | . | . | . | . | . | . | . | . | . |
| 21-225_75A12_LC | . | . | . | . | . | S | . | . | . | . | . | . | R | . | . | . | . | . | . | . | . | . | . | . | . | . |
| 21-225_75A5_LC | . | . | . | . | . | . | . | . | . | . | . | . | . | . | . | . | . | . | . | . | . | . | . | . | . | . |
| 21-225_75A8_LC | . | . | . | . | . | . | . | . | . | . | . | . | . | . | . | . | . | . | . | . | . | . | . | . | . | . |
| 21-225_75B10_LC | . | . | P | . | . | N | . | . | . | . | . | . | Y | . | . | . | . | . | . | . | . | . | . | . | . | . |
| 21-225_75C2_LC | . | . | . | . | . | . | . | . | . | . | . | . | . | . | . | . | . | . | . | . | . | . | . | . | . | . |
| 21-225_75C3_LC | . | . | . | . | . | S | . | . | . | . | . | . | R | . | . | . | . | . | . | . | . | . | . | . | . | . |
| 21-225_75D3_LC | . | . | . | . | . | . | . | . | . | . | . | . | . | . | . | . | . | . | . | . | . | . | . | . | . | . |
| 21-225_75D9_LC | . | . | . | . | . | S | . | . | . | . | . | . | . | . | . | . | . | . | . | . | . | R | . | . | . | . |
| 21-225_75F3_LC | . | . | . | . | . | . | . | . | . | . | . | . | . | . | . | . | . | . | . | . | . | . | . | . | . | . |
| 21-225_75G12_LC | . | . | . | . | . | . | . | . | . | . | . | . | . | . | . | . | . | . | . | . | . | . | . | . | . | . |
| 21-225_75G3_LC | . | . | . | N | . | D | . | . | . | . | . | . | . | . | . | . | . | . | . | . | . | . | . | . | . | . |
| 21-225_75G7_LC | . | . | . | . | . | D | . | . | . | . | . | . | . | . | . | . | . | . | . | . | . | R | . | . | . | . |
| 21-225_75G9_LC | . | . | P | . | . | D | . | . | . | . | . | . | . | . | . | . | . | . | . | . | . | . | . | . | . | . |
| 21-225_75H8_LC | . | . | . | N | I | . | . | . | . | . | . | . | N | . | . | . | . | . | . | . | . | . | . | . | . | . |
| 21-225_76A4_LC | . | . | . | . | . | . | . | . | . | . | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | V | Y | S | - | - | - | - | - | S | Y | L | A | W | Y | Q | Q | K | P | G | Q | A | P |
| 21-225_76A7_LC | . | . | P | . | . | D | . | . | . | . | . | . | . | . | . | . | . | . | . | . | R | . | . | . | . | . |
| 21-225_76C9_LC | . | . | . | . | . | S | . | . | . | . | . | . | . | . | . | S | . | . | . | . | . | . | . | . | . | . |
| 21-225_76D1_LC | . | . | . | N | I | . | . | . | . | . | . | . | N | . | . | . | . | . | . | E | . | . | . | . | . | . |
| 21-225_76E10_LC | . | . | . | . | . | N | . | . | . | . | . | . | N | . | . | . | . | . | R | . | . | . | . | . | . | . |
| 21-225_76G4_LC | . | . | . | . | . | . | . | . | . | . | . | . | . | . | . | . | . | . | . | . | . | . | . | . | . | . |
| 21-225_77A10_LC | . | . | . | . | . | H | . | . | . | . | . | . | R | . | . | . | . | . | . | . | . | . | . | . | . | . |
| 21-225_77C12_LC | . | . | . | . | . | D | . | . | . | . | . | . | . | . | . | . | . | . | . | . | . | R | . | . | . | . |
| 21-225_77C5_LC | . | . | . | . | . | . | . | . | . | . | . | . | . | . | . | . | . | . | . | . | . | . | . | . | . | . |
| 21-225_77F7_LC | . | . | . | . | . | . | . | . | . | . | . | . | . | . | . | . | . | . | . | . | . | . | . | . | . | . |
| 21-225_77G1_LC | . | . | . | . | . | . | . | . | . | . | . | . | . | . | . | . | . | . | . | . | . | . | . | . | . | . |
| 21-225_78E7_LC | . | . | . | N | . | D | . | . | . | . | . | . | N | . | . | . | . | . | . | . | . | . | . | . | . | . |
| 21-225_79F11_LC | . | . | . | . | . | . | . | . | . | . | . | . | . | . | . | . | . | . | . | . | . | . | . | . | . | . |
| 21-225_79F3_LC | . | . | . | . | . | . | . | . | . | . | . | . | . | . | . | . | . | . | . | . | . | . | A | . | . | . |
| 21-225_79G1_LC | . | . | . | . | . | . | . | . | . | . | . | . | . | . | . | . | . | . | . | . | . | . | A | . | . | . |
| 21-225_79G12_LC | . | . | . | . | . | . | . | . | . | . | . | . | . | . | . | . | . | . | . | . | . | . | . | . | . | . |
| 21-225_80A1_LC | . | . | . | . | . | . | . | . | . | . | . | . | . | . | . | . | . | . | . | . | . | . | . | . | . | . |
| 21-225_80A6_LC | . | . | . | . | . | D | . | . | . | . | . | . | G | . | . | . | . | . | . | . | . | . | . | . | . | . |
| 21-225_80B10_LC | . | . | . | . | . | D | . | . | . | . | . | . | . | . | . | . | . | . | . | . | . | . | . | . | . | . |
| 21-225_80C1_LC | . | . | . | . | . | . | . | . | . | . | . | . | . | . | . | . | . | . | . | . | . | . | . | . | . | . |
| 21-225_80D5_LC | . | . | . | N | I | . | . | . | . | . | . | . | . | . | . | . | . | . | . | . | . | . | . | . | . | . |
| 21-225_80E12_LC | . | . | . | . | . | N | . | . | . | . | . | . | N | . | . | . | . | . | R | . | . | . | D | . | . | . |
| 21-225_80E3_LC | . | . | . | . | . | . | . | . | . | . | . | . | . | . | . | . | . | . | . | . | . | . | . | . | . | . |
| 21-225_80H12_LC | . | . | . | . | . | . | . | . | . | . | . | . | . | . | . | . | . | . | . | . | . | . | . | . | . | . |
| 21-225_80H7_LC | . | . | . | . | I | N | . | . | . | . | . | . | N | . | . | . | . | . | . | . | . | . | . | . | . | . |
| 21-225_81C11_LC | . | . | . | . | . | . | . | . | . | . | . | . | . | . | . | . | . | . | . | . | . | . | . | . | . | . |
| 21-225_81C5_LC | . | . | . | . | . | . | . | . | . | . | . | . | . | . | . | . | . | . | . | . | . | . | . | . | . | . |
| 21-225_83B6_LC | . | . | . | . | . | D | . | . | . | . | . | . | G | . | . | . | . | . | . | . | . | . | . | . | . | . |
| 21-225_83B7_LC | . | . | . | . | . | . | . | . | . | . | . | . | . | . | . | . | . | . | . | . | . | . | . | . | . | . |
| 21-225_83C10_LC | . | . | P | . | . | H | . | . | . | . | . | . | N | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | V | Y | S | - | - | - | - | - | S | Y | L | A | W | Y | Q | Q | K | P | G | Q | A | P |
| 21-225_83G1_LC | . | . | . | . | . | . | . | . | . | . | . | . | . | . | . | . | . | . | . | . | . | . | . | . | . | . |
| 21-225_84E12_LC | . | . | . | . | I | N | . | . | . | . | . | . | N | . | . | . | . | . | . | . | . | . | . | . | . | . |
| 21-225_85A3_LC | . | . | . | . | . | . | . | . | . | . | . | . | . | . | . | . | . | . | . | . | . | . | A | . | . | . |
| 21-225_85B4_LC | . | . | . | . | . | . | . | . | . | . | . | . | . | . | . | . | . | . | . | . | . | . | . | . | . | . |
| 21-225_85B9_LC | . | . | . | . | . | N | . | . | . | . | . | . | N | . | . | . | . | . | R | . | . | . | . | . | . | . |
| 21-225_85D6_LC | . | . | . | . | . | S | . | . | . | . | . | . | R | . | . | . | . | . | . | . | . | . | . | . | . | . |
| 21-225_85G10_LC | . | . | . | . | . | W | . | . | . | . | . | . | G | . | . | . | . | . | . | . | . | . | . | . | . | . |
| 21-225_85G6_LC | . | . | P | . | . | D | . | . | . | . | . | . | . | . | . | . | . | . | . | . | . | . | . | . | . | . |
| 21-225_86C1_LC | . | . | . | . | . | . | . | . | . | . | . | . | . | . | . | . | . | . | . | . | . | . | A | . | . | . |
| 21-225_86C11_LC | . | . | . | . | . | . | . | . | . | . | . | . | . | . | . | . | . | . | . | . | . | . | . | . | . | . |
| 21-225_86E4_LC | . | . | . | . | . | . | . | . | . | . | . | . | . | . | . | . | . | . | . | . | . | . | . | . | . | . |
| 21-225_87A12_LC | . | . | . | . | . | . | . | . | . | . | . | . | . | . | . | . | . | . | . | . | . | . | . | . | . | . |
| 21-225_87C9_LC | . | . | . | . | . | . | . | . | . | . | . | . | . | . | . | . | . | . | . | . | . | . | . | . | . | . |
| 21-225_87E5_LC | . | . | . | . | . | . | . | . | . | . | . | . | . | . | . | . | . | . | . | . | . | . | . | . | . | . |
| 21-225_87H1_LC | . | . | . | . | . | D | . | . | . | . | . | . | N | . | . | . | . | . | . | . | . | . | . | . | . | . |
| 21-225_88E7_LC | . | . | . | . | . | . | . | . | . | . | . | . | . | . | . | . | . | . | . | . | . | . | . | . | . | . |
| 21-225_88F7_LC | . | . | . | . | . | . | . | . | . | . | . | . | . | . | . | . | . | . | . | . | . | . | . | . | . | . |
| 21-225_88G9_LC | . | . | . | . | . | . | . | . | . | . | . | . | . | . | . | . | . | . | . | . | . | . | . | . | . | . |
| 21-225_88H1_LC | . | . | . | . | . | . | . | . | . | . | . | . | . | . | . | . | . | . | . | . | . | . | . | . | . | . |
| 21-225_89A11_LC | . | . | . | . | . | D | . | . | . | . | . | . | N | F | . | . | . | . | . | . | . | . | . | . | . | . |
| 21-225_89D5_LC | . | . | . | . | . | . | . | . | . | . | . | . | . | . | . | . | . | . | . | . | . | . | . | . | . | . |
| 21-225_89E10_LC | . | . | . | . | . | . | . | . | . | . | . | . | . | . | . | . | . | . | . | . | . | . | . | . | . | . |
| 21-225_89H5_LC | . | . | . | . | . | N | . | . | . | . | . | . | N | . | . | . | . | . | . | . | . | . | . | . | . | . |
| 21-225_90A5_LC | . | . | . | . | . | . | . | . | . | . | . | . | . | . | . | . | . | . | . | . | . | . | . | . | . | . |
| 21-225_90F10_LC | . | . | . | . | . | . | . | . | . | . | . | . | . | . | . | . | . | . | . | . | . | . | . | . | . | . |
| 21-225_90G4_LC | . | . | . | . | . | . | . | . | . | . | . | . | . | . | . | . | . | . | . | . | . | . | . | . | . | . |
| 21-225_90G5_LC | . | . | . | . | . | . | . | . | . | . | . | . | . | . | . | . | . | . | . | . | . | . | . | . | . | . |
| 21-225_91B2_LC | . | . | . | . | . | . | . | . | . | . | . | . | . | . | . | . | . | . | . | . | . | . | . | . | . | . |
| 21-225_91B3_LC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | V | Y | S | - | - | - | - | - | S | Y | L | A | W | Y | Q | Q | K | P | G | Q | A | P |
| 21-225_91B4_LC | . | . | . | . | . | . | . | . | . | . | . | . | . | . | . | . | . | . | . | . | . | . | . | . | . | . |
| 21-225_91E9_LC | . | . | . | . | . | . | . | . | . | . | . | . | . | . | . | . | . | . | . | . | . | . | . | . | . | . |
| 21-225_91F3_LC | . | . | . | . | . | . | . | . | . | . | . | . | . | . | . | . | . | . | . | . | . | . | A | . | . | . |
| 21-225_92B1_LC | . | . | . | . | . | G | . | . | . | . | . | . | N | . | . | . | . | . | . | . | . | R | . | . | . | . |
| 21-225_92D6_LC | . | . | . | . | . | . | . | . | . | . | . | . | . | . | . | . | . | . | . | . | . | . | . | . | . | . |
| 21-225_93C2_LC | . | . | . | . | . | . | . | . | . | . | . | . | . | . | . | . | . | . | . | . | . | . | . | . | . | . |
| 21-225_93E4_LC | . | . | . | . | . | . | . | . | . | . | . | . | . | . | . | . | . | . | . | . | . | . | . | . | . | . |
| 21-225_93E9_LC | . | . | . | . | . | D | . | . | . | . | . | . | . | . | . | . | . | . | . | . | . | T | . | . | . | . |
| 21-225_94D3_LC | . | . | . | . | . | S | . | . | . | . | . | . | R | . | . | . | . | . | . | . | . | . | . | . | . | . |
| 21-225_94F12_LC | . | . | . | . | . | . | . | . | . | . | . | . | . | . | . | . | . | . | . | . | . | . | . | . | . | . |
| 21-225_94G10_LC | . | . | . | . | . | N | . | . | . | . | . | . | N | . | . | . | . | . | R | . | . | . | . | . | . | . |
| 21-225_95D2_LC | . | . | . | . | . | . | . | . | . | . | . | . | . | . | . | . | . | . | . | . | . | . | . | . | . | . |
| 21-225_95F9_LC | . | . | . | . | . | . | . | . | . | . | . | . | . | . | . | . | . | . | . | . | . | . | . | . | . | . |
| 21-225_95G9_LC | . | . | . | . | . | . | . | . | . | . | . | . | . | . | . | . | . | . | . | . | . | . | . | . | . | . |
| 21-225_95H10_LC | . | . | . | . | . | . | . | . | . | . | . | . | . | . | . | . | . | . | . | . | . | . | . | . | . | . |
| 21-225_97A2_LC | . | . | . | . | . | . | . | . | . | . | . | . | . | . | . | . | . | . | . | . | . | . | . | . | . | . |
| 21-225_97E5_LC | . | . | . | . | . | . | . | . | . | . | . | . | . | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | R | L | L | I | Y | G | - | - | - | - | - | - | - | - | A | S | S | R | A | T | G | I | P | D | R | F |
| 21-225_147G8_LC | . | I | . | . | . | . | . | . | . | . | . | . | . | . | . | . | . | . | . | . | . | . | . | . | . | I |
| 21-225_190A3_LC | . | . | . | . | . | . | . | . | . | . | . | . | . | . | . | . | . | . | . | . | . | . | . | . | . | . |
| 21-225_190A7_LC | . | . | . | . | . | . | . | . | . | . | . | . | . | . | . | Y | R | . | . | . | . | . | . | . | . | . |
| 21-225_190B4_LC | . | . | . | . | . | . | . | . | . | . | . | . | . | . | . | . | . | . | . | . | . | . | . | . | . | . |
| 21-225_190G10_LC | . | . | . | . | . | . | . | . | . | . | . | . | . | . | . | . | . | . | . | . | . | . | . | . | . | . |
| 21-225_191A3_LC | . | . | . | . | . | . | . | . | . | . | . | . | . | . | . | . | . | . | . | . | . | . | . | . | . | . |
| 21-225_191A5_LC | . | . | . | . | . | . | . | . | . | . | . | . | . | . | . | . | N | . | . | . | . | . | . | . | . | . |
| 21-225_191C3_LC | . | . | . | . | . | . | . | . | . | . | . | . | . | . | . | . | . | . | . | . | . | . | . | . | . | . |
| 21-225_191D3_LC | . | . | . | . | . | . | . | . | . | . | . | . | . | . | . | . | . | . | . | . | . | . | . | . | . | . |
| 21-225_191F1_LC | . | . | . | . | . | . | . | . | . | . | . | . | . | . | . | . | . | . | . | . | . | . | . | . | . | . |
| 21-225_191H7_LC | . | . | . | . | . | . | . | . | . | . | . | . | . | . | P | . | . | . | . | . | . | . | . | . | . | . |
| 21-225_192B3_LC | . | . | . | . | . | . | . | . | . | . | . | . | . | . | . | . | . | . | . | . | . | . | . | . | . | . |
| 21-225_192F8_LC | . | . | . | . | . | . | . | . | . | . | . | . | . | . | . | . | . | . | . | . | . | . | . | A | . | . |
| 21-225_192G12_LC | . | . | . | . | . | . | . | . | . | . | . | . | . | . | V | . | R | . | . | . | . | . | . | . | . | . |
| 21-225_192H3_LC | . | . | . | . | F | . | . | . | . | . | . | . | . | . | . | . | . | . | . | . | . | . | . | . | . | . |
| 21-225_193A1_LC | . | . | F | . | . | . | . | . | . | . | . | . | . | . | . | . | R | . | . | . | . | . | . | . | . | . |
| 21-225_193B1_LC | . | . | . | . | . | . | . | . | . | . | . | . | . | . | . | . | R | . | . | . | . | . | . | . | . | . |
| 21-225_193B10_LC | . | . | V | . | . | . | . | . | . | . | . | . | . | . | . | . | R | . | . | . | . | . | . | . | . | . |
| 21-225_193B12_LC | . | . | . | . | . | D | . | . | . | . | . | . | . | . | . | . | . | . | . | . | . | . | . | . | . | . |
| 21-225_193C8_LC | . | . | . | . | . | . | . | . | . | . | . | . | . | . | . | . | . | . | . | . | . | . | . | . | . | . |
| 21-225_193D3_LC | . | . | . | . | . | . | . | . | . | . | . | . | . | . | . | . | N | . | . | . | . | . | . | . | . | . |
| 21-225_193D8_LC | . | . | . | . | . | . | . | . | . | . | . | . | . | . | . | . | . | . | . | . | . | . | . | . | . | . |
| 21-225_193E6_LC | . | . | . | . | . | . | . | . | . | . | . | . | . | . | . | . | . | . | . | . | . | . | . | . | . | . |
| 21-225_193F3_LC | . | . | . | . | . | . | . | . | . | . | . | . | . | . | . | . | . | . | . | . | . | F | . | E | . | . |
| 21-225_193G8_LC | . | . | . | . | . | . | . | . | . | . | . | . | . | . | . | . | R | . | . | . | . | . | . | . | . | . |
| 21-225_193H6_LC | . | . | . | . | . | . | . | . | . | . | . | . | . | . | . | . | . | . | . | . | . | . | . | . | . | . |
| 21-225_194C10_LC | . | . | . | . | F | . | . | . | . | . | . | . | . | . | . | . | . | . | . | . | . | . | . | . | . | . |
| 21-225_194E5_LC | . | . | . | . | . | . | . | . | . | . | . | . | . | . | . | . | . | . | . | . | . | . | . | . | . | . |
| 21-225_194E6_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | R | L | L | I | Y | G | - | - | - | - | - | - | - | - | A | S | S | R | A | T | G | I | P | D | R | F |
| 21-225_195B1_LC | . | . | . | . | . | . | . | . | . | . | . | . | . | . | . | . | N | . | . | . | . | V | . | . | . | . |
| 21-225_195C8_LC | . | . | . | . | . | . | . | . | . | . | . | . | . | . | . | F | . | . | . | . | . | . | . | . | . | . |
| 21-225_196G10_LC | . | . | . | . | . | . | . | . | . | . | . | . | . | . | . | . | . | . | . | . | . | . | . | . | . | . |
| 21-225_196H12_LC | . | . | . | . | . | . | . | . | . | . | . | . | . | . | . | Y | R | . | . | . | . | . | . | . | . | . |
| 21-225_197F4_LC | . | . | . | . | . | . | . | . | . | . | . | . | . | . | . | . | . | . | . | . | . | . | . | . | . | . |
| 21-225_198B1_LC | . | . | . | . | . | . | . | . | . | . | . | . | . | . | . | . | . | . | . | . | . | . | . | . | . | . |
| 21-225_198C2_LC | . | . | . | . | C | . | . | . | . | . | . | . | . | . | . | F | . | . | . | S | . | . | . | . | . | . |
| 21-225_198E1_LC | . | . | . | . | . | . | . | . | . | . | . | . | . | . | . | F | . | . | . | . | . | . | . | . | . | . |
| 21-225_198G10_LC | . | . | . | . | . | . | . | . | . | . | . | . | . | . | . | . | . | . | . | . | . | . | . | . | . | . |
| 21-225_199C2_LC | . | . | . | . | . | . | . | . | . | . | . | . | . | . | . | . | . | . | . | . | . | . | . | . | . | . |
| 21-225_199C7_LC | . | . | . | . | . | . | . | . | . | . | . | . | . | . | . | . | . | . | . | . | . | . | . | . | . | . |
| 21-225_199G11_LC | . | . | . | . | . | . | . | . | . | . | . | . | . | . | . | F | . | . | . | . | . | . | . | . | . | . |
| 21-225_200A1_LC | S | . | . | . | . | . | . | . | . | . | . | . | . | . | . | . | . | . | . | . | C | . | L | . | . | . |
| 21-225_200F10_LC | . | . | . | . | . | . | . | . | . | . | . | . | . | . | . | . | . | . | . | . | . | . | . | . | . | . |
| 21-225_201B2_LC | . | . | . | . | . | . | . | . | . | . | . | . | . | . | . | . | . | . | . | . | . | . | . | . | . | . |
| 21-225_201E1_LC | . | . | . | . | . | . | . | . | . | . | . | . | . | . | . | . | . | . | . | . | . | . | . | . | . | . |
| 21-225_202D11_LC | . | V | . | . | . | . | . | . | . | . | . | . | . | . | . | . | . | . | . | . | . | . | . | . | . | . |
| 21-225_202D9_LC | . | . | . | . | . | . | . | . | . | . | . | . | . | . | . | . | . | . | . | . | . | . | . | . | . | . |
| 21-225_205G7_LC | . | . | . | . | . | . | . | . | . | . | . | . | . | . | . | . | . | . | . | A | . | . | . | . | . | . |
| 21-225_208A9_LC | . | V | . | . | . | . | . | . | . | . | . | . | . | . | . | . | . | . | . | . | . | . | . | . | . | . |
| 21-225_208B5_LC | . | . | . | . | . | . | . | . | . | . | . | . | . | . | . | . | . | . | . | . | . | . | . | . | . | . |
| 21-225_220E1_LC | . | . | . | L | . | V | . | . | . | . | . | . | . | . | T | . | . | . | . | . | . | . | . | . | . | . |
| 21-225_222C7_LC | . | . | . | . | . | . | . | . | . | . | . | . | . | . | . | . | . | . | . | . | . | . | . | . | . | . |
| 21-225_225E7_LC | . | . | . | . | . | . | . | . | . | . | . | . | . | . | . | . | . | . | . | . | . | . | . | . | . | . |
| 21-225_23B9_LC | . | . | . | . | . | . | . | . | . | . | . | . | . | . | . | . | . | . | . | S | . | . | . | . | . | . |
| 21-225_44F6_LC | . | . | . | . | . | . | . | . | . | . | . | . | . | . | . | . | . | . | . | . | . | . | . | . | . | . |
| 21-225_59B2_LC | . | . | . | . | . | . | . | . | . | . | . | . | . | . | . | . | . | . | . | . | . | . | . | . | . | . |
| 21-225_74A3_LC | . | . | . | . | . | . | . | . | . | . | . | . | . | . | . | . | . | . | S | . | . | . | . | . | . | . |
| 21-225_74A4_LC | . | . | . | . | . | . | . | . | . | . | . | . | . | . | . | . | . | . | S | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | R | L | L | I | Y | G | - | - | - | - | - | - | - | - | A | S | S | R | A | T | G | I | P | D | R | F |
| 21-225_74B12_LC | . | . | . | . | . | . | . | . | . | . | . | . | . | . | . | . | . | . | S | . | . | . | . | . | . | . |
| 21-225_74B2_LC | . | . | . | . | . | . | . | . | . | . | . | . | . | . | . | . | . | . | . | . | . | . | . | . | . | . |
| 21-225_74C5_LC | . | . | . | . | . | . | . | . | . | . | . | . | . | . | . | F | . | . | . | . | . | . | . | . | . | V |
| 21-225_74C7_LC | . | . | . | . | . | . | . | . | . | . | . | . | . | . | . | . | . | . | . | . | . | . | . | . | . | . |
| 21-225_74D11_LC | . | . | . | . | . | . | . | . | . | . | . | . | . | . | . | . | . | . | . | . | . | . | . | . | . | . |
| 21-225_74D2_LC | . | . | . | . | . | . | . | . | . | . | . | . | . | . | . | . | . | . | . | P | . | . | . | . | . | . |
| 21-225_74D5_LC | . | . | . | . | . | . | . | . | . | . | . | . | . | . | . | . | . | . | . | . | . | . | . | . | . | . |
| 21-225_74D6_LC | . | . | . | . | . | . | . | . | . | . | . | . | . | . | . | . | . | . | . | . | . | . | . | . | . | . |
| 21-225_74F4_LC | . | . | . | . | . | . | . | . | . | . | . | . | . | . | . | . | . | . | . | . | . | . | . | . | . | . |
| 21-225_74F5_LC | . | . | . | . | . | . | . | . | . | . | . | . | . | . | . | . | . | . | S | . | . | . | . | . | . | . |
| 21-225_74G8_LC | . | . | . | . | . | . | . | . | . | . | . | . | . | . | T | . | . | . | . | . | . | . | . | . | . | . |
| 21-225_74H5_LC | . | . | . | . | . | . | . | . | . | . | . | . | . | . | . | . | . | . | . | . | . | . | . | . | . | . |
| 21-225_74H7_LC | . | . | . | . | . | . | . | . | . | . | . | . | . | . | . | . | . | . | . | . | . | . | . | . | . | . |
| 21-225_74H8_LC | . | . | . | . | . | . | . | . | . | . | . | . | . | . | . | . | . | . | . | . | . | . | . | . | . | . |
| 21-225_75A12_LC | . | . | . | . | . | . | . | . | . | . | . | . | . | . | . | . | . | . | . | . | . | . | . | . | . | . |
| 21-225_75A5_LC | . | . | . | . | . | . | . | . | . | . | . | . | . | . | . | . | . | . | . | . | . | . | . | . | . | . |
| 21-225_75A8_LC | . | . | . | . | . | . | . | . | . | . | . | . | . | . | . | . | . | . | . | . | . | . | . | . | . | . |
| 21-225_75B10_LC | . | . | . | . | . | . | . | . | . | . | . | . | . | . | . | T | . | . | . | . | . | . | . | . | . | . |
| 21-225_75C2_LC | . | . | . | . | . | . | . | . | . | . | . | . | . | . | . | . | . | . | . | . | . | . | . | . | . | . |
| 21-225_75C3_LC | . | . | . | . | . | . | . | . | . | . | . | . | . | . | . | . | . | . | . | . | . | . | . | . | . | . |
| 21-225_75D3_LC | . | . | I | . | . | . | . | . | . | . | . | . | . | . | . | . | . | . | S | . | . | . | . | . | . | . |
| 21-225_75D9_LC | . | . | . | . | . | . | . | . | . | . | . | . | . | . | . | . | . | . | . | . | . | . | . | . | . | . |
| 21-225_75F3_LC | . | . | . | . | . | . | . | . | . | . | . | . | . | . | . | . | . | . | . | . | . | . | . | . | . | . |
| 21-225_75G12_LC | . | . | . | . | . | . | . | . | . | . | . | . | . | . | . | . | . | . | . | . | . | . | . | . | . | . |
| 21-225_75G3_LC | . | . | . | . | . | . | . | . | . | . | . | . | . | . | . | . | . | . | . | . | . | . | . | . | . | . |
| 21-225_75G7_LC | . | . | . | . | . | . | . | . | . | . | . | . | . | . | . | . | . | . | . | . | . | . | . | . | . | . |
| 21-225_75G9_LC | . | . | . | . | . | . | . | . | . | . | . | . | . | . | . | . | . | . | . | P | . | . | . | . | . | . |
| 21-225_75H8_LC | . | . | . | . | . | . | . | . | . | . | . | . | . | . | . | . | . | . | . | . | . | . | . | . | . | . |
| 21-225_76A4_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | R | L | L | I | Y | G | - | - | - | - | - | - | - | - | A | S | S | R | A | T | G | I | P | D | R | F |
| 21-225_76A7_LC | . | . | . | . | . | . | . | . | . | . | . | . | . | . | . | . | . | . | . | P | . | . | . | . | . | . |
| 21-225_76C9_LC | . | . | . | . | . | . | . | . | . | . | . | . | . | . | . | . | . | . | . | . | . | . | . | . | . | . |
| 21-225_76D1_LC | . | . | . | . | . | . | . | . | . | . | . | . | . | . | . | . | . | . | . | . | . | . | . | . | . | . |
| 21-225_76E10_LC | . | . | . | . | . | . | . | . | . | . | . | . | . | . | . | . | . | . | . | . | . | . | . | . | . | . |
| 21-225_76G4_LC | . | . | . | . | . | . | . | . | . | . | . | . | . | . | . | . | . | . | . | . | . | . | . | . | . | . |
| 21-225_77A10_LC | K | . | . | . | F | . | . | . | . | . | . | . | . | . | . | . | . | . | . | . | . | . | . | . | . | . |
| 21-225_77C12_LC | . | . | . | . | . | . | . | . | . | . | . | . | . | . | . | . | . | . | . | . | . | . | . | . | . | . |
| 21-225_77C5_LC | . | . | . | . | . | . | . | . | . | . | . | . | . | . | . | . | . | . | S | . | . | . | . | . | . | . |
| 21-225_77F7_LC | . | . | I | . | . | . | . | . | . | . | . | . | . | . | . | . | . | . | . | . | . | . | . | . | . | . |
| 21-225_77G1_LC | . | . | . | . | . | . | . | . | . | . | . | . | . | . | . | . | . | . | . | . | . | . | . | . | . | . |
| 21-225_78E7_LC | . | . | . | . | . | . | . | . | . | . | . | . | . | . | . | . | . | . | . | . | . | . | . | . | . | . |
| 21-225_79F11_LC | . | . | . | . | . | . | . | . | . | . | . | . | . | . | . | . | . | . | . | . | . | . | . | . | . | . |
| 21-225_79F3_LC | . | . | . | . | . | . | . | . | . | . | . | . | . | . | . | . | . | . | S | . | . | . | . | . | . | . |
| 21-225_79G1_LC | . | . | . | . | . | . | . | . | . | . | . | . | . | . | . | . | . | . | S | . | . | . | . | . | . | . |
| 21-225_79G12_LC | . | . | . | . | . | . | . | . | . | . | . | . | . | . | . | . | . | . | S | . | . | . | . | . | . | . |
| 21-225_80A1_LC | . | . | . | . | . | . | . | . | . | . | . | . | . | . | . | . | . | . | S | . | . | . | . | . | . | . |
| 21-225_80A6_LC | . | . | . | . | . | . | . | . | . | . | . | . | . | . | . | . | . | . | . | P | . | . | . | . | . | . |
| 21-225_80B10_LC | . | . | . | . | . | . | . | . | . | . | . | . | . | . | . | . | . | . | . | P | . | . | . | . | . | . |
| 21-225_80C1_LC | . | . | . | . | . | . | . | . | . | . | . | . | . | . | . | . | . | . | S | . | . | . | . | . | . | . |
| 21-225_80D5_LC | . | . | . | . | . | . | . | . | . | . | . | . | . | . | . | . | . | . | . | . | . | . | . | . | . | . |
| 21-225_80E12_LC | . | . | . | . | . | . | . | . | . | . | . | . | . | . | . | . | . | . | . | . | . | . | . | . | . | . |
| 21-225_80E3_LC | . | . | . | . | . | . | . | . | . | . | . | . | . | . | . | . | . | . | S | . | . | . | . | . | . | . |
| 21-225_80H12_LC | . | . | . | . | . | . | . | . | . | . | . | . | . | . | . | . | . | . | S | . | . | . | . | . | . | . |
| 21-225_80H7_LC | . | . | . | . | . | . | . | . | . | . | . | . | . | . | . | . | . | . | . | . | . | . | . | . | . | . |
| 21-225_81C11_LC | . | . | . | . | . | . | . | . | . | . | . | . | . | . | . | . | . | . | S | . | . | L | . | . | . | . |
| 21-225_81C5_LC | . | . | . | . | . | . | . | . | . | . | . | . | . | . | . | . | . | . | S | . | . | . | . | . | . | . |
| 21-225_83B6_LC | . | . | . | . | . | . | . | . | . | . | . | . | . | . | . | . | . | . | T | P | . | . | . | . | . | . |
| 21-225_83B7_LC | . | . | . | . | . | . | . | . | . | . | . | . | . | . | . | . | . | . | S | . | . | . | . | . | . | . |
| 21-225_83C10_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | R | L | L | I | Y | G | - | - | - | - | - | - | - | - | A | S | S | R | A | T | G | I | P | D | R | F |
| 21-225_83G1_LC | . | . | . | . | . | . | . | . | . | . | . | . | . | . | . | . | . | . | S | . | V | L | . | . | . | . |
| 21-225_84E12_LC | . | . | . | . | . | . | . | . | . | . | . | . | . | . | . | . | . | . | . | . | . | . | . | . | . | . |
| 21-225_85A3_LC | . | . | . | . | . | . | . | . | . | . | . | . | . | . | . | . | . | . | . | S | . | . | . | . | . | . |
| 21-225_85B4_LC | . | . | . | . | . | . | . | . | . | . | . | . | . | . | . | . | . | . | . | . | . | . | . | . | . | . |
| 21-225_85B9_LC | . | . | . | . | . | . | . | . | . | . | . | . | . | . | . | . | . | . | . | . | . | . | . | . | . | . |
| 21-225_85D6_LC | . | . | . | . | . | . | . | . | . | . | . | . | . | . | . | . | . | . | . | . | . | . | . | . | . | . |
| 21-225_85G10_LC | . | . | . | . | . | . | . | . | . | . | . | . | . | . | . | . | . | . | . | . | . | . | . | . | . | . |
| 21-225_85G6_LC | . | . | . | . | . | . | . | . | . | . | . | . | . | . | . | A | . | . | . | P | . | . | . | . | . | . |
| 21-225_86C1_LC | . | . | . | . | . | . | . | . | . | . | . | . | . | . | . | . | . | . | . | . | . | . | . | . | . | . |
| 21-225_86C11_LC | . | . | . | . | . | . | . | . | . | . | . | . | . | . | . | . | . | . | S | . | . | L | . | . | . | . |
| 21-225_86E4_LC | . | . | . | . | . | . | . | . | . | . | . | . | . | . | . | . | . | . | S | . | . | . | . | . | . | . |
| 21-225_87A12_LC | . | . | . | . | . | . | . | . | . | . | . | . | . | . | . | . | . | . | S | . | V | . | . | . | . | . |
| 21-225_87C9_LC | . | . | . | . | . | . | . | . | . | . | . | . | . | . | . | . | . | . | S | . | . | . | . | . | . | . |
| 21-225_87E5_LC | . | . | . | . | . | . | . | . | . | . | . | . | . | . | . | . | . | . | S | . | . | . | . | . | . | . |
| 21-225_87H1_LC | . | . | . | . | . | . | . | . | . | . | . | . | . | . | . | . | A | . | T | . | . | . | . | . | . | . |
| 21-225_88E7_LC | . | . | . | . | . | . | . | . | . | . | . | . | . | . | . | . | . | . | S | . | . | . | . | . | . | . |
| 21-225_88F7_LC | . | . | . | . | . | . | . | . | . | . | . | . | . | . | . | . | . | . | S | . | . | . | . | . | . | . |
| 21-225_88G9_LC | . | . | . | . | . | . | . | . | . | . | . | . | . | . | . | . | . | . | S | . | . | . | . | . | . | . |
| 21-225_88H1_LC | . | . | . | . | . | . | . | . | . | . | . | . | . | . | . | . | . | . | S | . | V | . | . | . | . | . |
| 21-225_89A11_LC | . | . | . | . | . | . | . | . | . | . | . | . | . | . | . | . | A | . | T | . | . | . | . | . | . | . |
| 21-225_89D5_LC | . | . | . | . | . | . | . | . | . | . | . | . | . | . | . | . | . | . | S | . | . | . | . | . | . | . |
| 21-225_89E10_LC | . | . | . | . | . | . | . | . | . | . | . | . | . | . | . | . | . | . | S | . | V | . | . | . | . | . |
| 21-225_89H5_LC | . | . | . | . | . | . | . | . | . | . | . | . | . | . | . | P | . | . | . | . | . | . | . | . | . | V |
| 21-225_90A5_LC | . | . | . | . | . | . | . | . | . | . | . | . | . | . | . | . | . | . | . | . | . | . | . | . | . | . |
| 21-225_90F10_LC | . | . | . | . | . | . | . | . | . | . | . | . | . | . | . | . | . | . | S | . | . | . | . | . | . | . |
| 21-225_90G4_LC | . | . | . | . | . | . | . | . | . | . | . | . | . | . | . | . | . | . | S | . | V | . | . | . | . | . |
| 21-225_90G5_LC | . | . | I | . | . | . | . | . | . | . | . | . | . | . | . | . | . | . | . | . | . | . | . | . | . | . |
| 21-225_91B2_LC | . | . | . | . | . | . | . | . | . | . | . | . | . | . | . | . | . | . | S | . | . | . | . | . | . | . |
| 21-225_91B3_LC | . | . | . | . | . | . | . | . | . | . | . | . | . | . | . | . | . | . | S | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | R | L | L | I | Y | G | - | - | - | - | - | - | - | - | A | S | S | R | A | T | G | I | P | D | R | F |
| 21-225_91B4_LC | . | . | I | . | . | . | . | . | . | . | . | . | . | . | . | . | . | . | S | . | . | . | . | . | . | . |
| 21-225_91E9_LC | . | . | . | . | . | . | . | . | . | . | . | . | . | . | . | . | . | . | . | . | . | . | . | . | . | . |
| 21-225_91F3_LC | . | . | . | . | . | . | . | . | . | . | . | . | . | . | . | . | . | . | S | . | . | . | . | . | . | . |
| 21-225_92B1_LC | . | . | . | . | . | . | . | . | . | . | . | . | . | . | . | . | . | . | . | . | D | . | . | . | . | . |
| 21-225_92D6_LC | . | . | . | . | . | . | . | . | . | . | . | . | . | . | . | . | . | . | S | . | V | . | . | . | . | . |
| 21-225_93C2_LC | . | . | . | . | . | . | . | . | . | . | . | . | . | . | . | . | . | . | . | . | . | . | . | . | . | . |
| 21-225_93E4_LC | . | . | . | . | . | . | . | . | . | . | . | . | . | . | . | . | . | . | S | . | . | . | . | . | . | . |
| 21-225_93E9_LC | . | . | . | . | . | . | . | . | . | . | . | . | . | . | . | . | . | . | . | P | . | . | . | . | . | . |
| 21-225_94D3_LC | . | . | . | . | . | . | . | . | . | . | . | . | . | . | . | . | . | . | . | . | . | . | . | . | . | . |
| 21-225_94F12_LC | . | . | . | . | . | . | . | . | . | . | . | . | . | . | . | . | . | . | S | . | . | . | . | . | . | . |
| 21-225_94G10_LC | . | . | . | . | . | . | . | . | . | . | . | . | . | . | . | . | . | . | . | . | . | . | . | . | . | . |
| 21-225_95D2_LC | . | . | . | . | . | . | . | . | . | . | . | . | . | . | . | . | . | . | . | . | . | . | . | . | . | . |
| 21-225_95F9_LC | . | . | . | . | . | . | . | . | . | . | . | . | . | . | . | . | . | . | S | . | . | . | . | . | . | . |
| 21-225_95G9_LC | . | . | . | . | . | . | . | . | . | . | . | . | . | . | . | . | . | . | S | . | . | . | . | . | . | . |
| 21-225_95H10_LC | . | . | . | . | . | . | . | . | . | . | . | . | . | . | . | . | . | . | . | . | . | . | . | . | . | . |
| 21-225_97A2_LC | . | . | . | . | . | . | . | . | . | . | . | . | . | . | . | . | . | . | S | . | . | . | . | . | . | . |
| 21-225_97E5_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | R | L | E | P | E | D | F | A | V | Y |
| 21-225_147G8_LC | . | . | . | . | . | . | . | . | . | . | . | . | . | . | . | T | . | . | . | . | . | . | . | . | . | . |
| 21-225_190A3_LC | . | . | . | V | . | . | . | . | . | . | . | . | . | . | . | N | . | . | . | . | . | . | . | . | . | . |
| 21-225_190A7_LC | . | . | . | . | . | . | . | . | . | . | . | . | . | . | . | N | . | . | . | . | . | . | . | . | . | . |
| 21-225_190B4_LC | . | . | . | . | . | . | . | . | . | . | . | . | . | . | . | . | . | . | . | . | . | . | . | . | . | . |
| 21-225_190G10_LC | . | . | . | . | . | . | . | . | . | . | . | . | . | . | N | . | . | . | . | . | . | . | . | . | . | . |
| 21-225_191A3_LC | . | . | . | . | . | . | . | . | . | . | . | . | . | . | . | . | . | . | . | . | . | . | . | . | . | . |
| 21-225_191A5_LC | . | . | . | . | . | . | . | . | . | . | . | . | . | . | . | . | . | . | . | . | . | . | . | . | . | . |
| 21-225_191C3_LC | . | . | . | . | . | . | . | . | . | . | . | . | . | . | . | . | . | . | . | . | . | . | . | . | . | . |
| 21-225_191D3_LC | . | . | . | . | . | . | . | . | . | . | . | . | . | . | . | N | . | . | . | . | . | . | . | . | . | . |
| 21-225_191F1_LC | . | . | . | . | . | . | . | . | . | . | . | . | . | . | . | N | . | . | . | . | . | . | . | . | . | . |
| 21-225_191H7_LC | . | . | . | . | . | . | . | . | . | . | . | . | . | . | . | N | . | . | . | . | . | . | . | . | . | . |
| 21-225_192B3_LC | . | . | . | . | . | . | . | . | . | . | . | . | . | . | . | . | . | . | . | . | . | . | . | . | . | . |
| 21-225_192F8_LC | . | . | . | . | . | . | . | . | . | . | . | . | . | . | . | . | . | . | . | . | . | . | . | . | . | . |
| 21-225_192G12_LC | . | . | . | . | . | . | . | . | . | . | . | . | . | . | . | N | . | . | . | . | . | . | . | . | . | . |
| 21-225_192H3_LC | . | . | . | . | . | . | . | . | . | . | . | . | . | . | . | N | . | . | . | . | . | . | . | . | . | . |
| 21-225_193A1_LC | . | . | . | . | . | . | . | . | . | . | . | . | . | . | . | N | . | . | . | . | . | . | . | . | . | . |
| 21-225_193B1_LC | . | . | . | . | . | . | . | . | . | . | . | . | . | . | . | N | . | . | . | . | . | . | . | . | . | . |
| 21-225_193B10_LC | R | . | . | . | . | . | . | . | . | . | . | . | . | . | . | . | . | . | . | . | . | . | . | . | . | . |
| 21-225_193B12_LC | . | . | . | . | . | . | . | . | . | . | . | . | . | . | . | . | . | . | . | . | . | . | . | . | . | . |
| 21-225_193C8_LC | . | . | . | . | . | . | . | . | . | . | . | . | . | . | . | . | . | . | . | . | . | . | . | . | . | . |
| 21-225_193D3_LC | . | . | . | . | . | . | . | . | . | . | . | . | . | . | . | . | . | . | . | . | . | . | . | . | . | . |
| 21-225_193D8_LC | . | . | . | . | . | . | . | . | . | . | . | . | . | . | . | N | . | . | . | . | . | . | . | . | . | . |
| 21-225_193E6_LC | G | . | . | . | . | . | . | . | . | . | . | . | . | . | . | . | . | . | . | . | . | . | . | . | . | . |
| 21-225_193F3_LC | . | . | . | . | . | . | . | . | . | . | . | . | . | . | . | N | . | . | . | . | . | . | . | . | . | . |
| 21-225_193G8_LC | . | . | . | . | . | . | . | . | . | . | . | . | . | . | . | N | . | . | . | . | . | . | . | . | L | . |
| 21-225_193H6_LC | . | . | . | . | . | . | . | . | . | . | . | . | . | . | . | N | . | . | . | . | . | . | . | . | . | . |
| 21-225_194C10_LC | . | A | . | . | . | . | . | . | A | . | . | . | . | . | . | . | . | . | . | . | . | . | . | . | . | . |
| 21-225_194E5_LC | . | . | . | . | . | . | . | . | . | . | . | . | . | . | . | N | . | . | . | . | K | . | . | . | . | . |
| 21-225_194E6_LC | . | . | . | V | . | . | . | . | . | . | . | . | . | . | . | N | . | . | . | . | . | . | . | . | . | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | R | L | E | P | E | D | F | A | V | Y |
| 21-225_195B1_LC | . | A | . | . | . | . | . | . | . | . | . | . | . | . | . | R | . | . | . | . | . | . | . | . | . | . |
| 21-225_195C8_LC | . | . | . | . | . | . | . | . | . | . | . | . | . | . | . | . | . | . | . | . | . | . | . | . | . | . |
| 21-225_196G10_LC | . | . | . | . | . | . | . | . | . | . | . | . | . | . | N | . | . | . | . | . | . | . | . | . | . | . |
| 21-225_196H12_LC | . | . | . | . | . | . | . | . | . | . | . | . | . | . | . | N | . | . | . | . | . | . | . | . | . | . |
| 21-225_197F4_LC | . | . | . | . | . | . | . | . | . | . | . | . | . | . | . | . | . | . | . | . | . | . | . | . | . | . |
| 21-225_198B1_LC | . | . | . | . | . | . | . | . | . | . | . | . | . | . | . | N | . | . | . | . | . | . | . | . | . | . |
| 21-225_198C2_LC | . | . | . | . | . | . | . | . | . | . | . | . | . | . | . | . | . | . | . | . | . | . | . | . | . | . |
| 21-225_198E1_LC | . | . | . | . | . | . | . | . | . | . | . | . | . | . | . | N | . | . | . | . | . | . | . | . | . | . |
| 21-225_198G10_LC | . | . | . | . | . | . | . | . | . | . | . | . | . | . | . | . | . | . | . | . | . | . | . | . | . | . |
| 21-225_199C2_LC | . | . | . | . | . | . | . | . | . | . | . | . | . | . | . | N | . | . | . | . | . | . | . | . | . | . |
| 21-225_199C7_LC | . | . | . | . | . | . | . | . | . | . | . | . | . | . | . | N | . | . | . | . | . | . | . | . | . | . |
| 21-225_199G11_LC | . | . | . | . | . | . | . | . | . | . | . | . | . | . | . | . | . | . | . | . | . | . | . | . | . | . |
| 21-225_200A1_LC | . | . | . | . | C | . | . | . | . | . | . | . | . | . | . | . | . | . | . | . | . | . | . | . | . | . |
| 21-225_200F10_LC | . | . | . | . | . | . | . | . | . | . | . | . | . | . | . | N | . | . | . | . | . | . | . | . | . | . |
| 21-225_201B2_LC | . | . | . | . | . | . | . | . | . | . | . | . | . | . | . | . | . | . | . | . | . | . | . | . | . | . |
| 21-225_201E1_LC | . | . | . | . | . | . | . | . | . | . | . | . | . | . | . | . | . | . | . | . | . | . | . | . | M | F |
| 21-225_202D11_LC | . | . | . | . | . | . | . | . | . | . | . | . | . | . | . | . | . | . | . | . | . | . | . | . | . | F |
| 21-225_202D9_LC | . | . | . | . | . | . | . | . | . | . | . | . | . | . | . | . | . | . | . | . | . | . | . | . | . | H |
| 21-225_205G7_LC | . | . | . | . | . | . | . | . | . | . | . | . | . | . | . | . | . | . | . | . | . | N | . | . | . | . |
| 21-225_208A9_LC | . | . | . | . | . | . | . | . | . | . | . | . | . | . | . | . | . | . | . | . | . | . | . | . | . | . |
| 21-225_208B5_LC | . | . | . | . | . | . | . | . | . | . | . | . | . | . | . | . | . | . | . | . | . | . | . | . | . | . |
| 21-225_220E1_LC | . | . | . | . | . | . | . | . | . | . | . | . | . | . | . | R | S | . | . | . | . | . | . | . | M | . |
| 21-225_222C7_LC | G | . | . | . | . | . | . | . | . | . | . | . | . | A | . | . | . | . | . | . | . | . | . | T | I | . |
| 21-225_225E7_LC | . | . | . | . | . | . | . | . | . | . | . | . | . | . | . | . | . | . | . | . | . | . | . | . | . | . |
| 21-225_23B9_LC | . | . | . | . | . | . | . | . | . | . | . | . | . | . | . | . | . | . | . | . | . | . | . | . | . | . |
| 21-225_44F6_LC | . | . | . | . | . | . | . | . | . | . | . | . | . | . | . | . | . | . | . | . | . | . | . | . | . | . |
| 21-225_59B2_LC | . | . | . | . | . | . | . | . | . | . | . | . | . | . | . | . | . | . | . | . | . | . | . | . | . | F |
| 21-225_74A3_LC | . | . | . | . | C | . | . | . | . | . | . | A | . | . | . | . | . | . | . | . | . | . | . | . | . | . |
| 21-225_74A4_LC | . | . | . | . | C | . | . | . | . | . | . | A | . | . | . | . | . | . | . | . | . | . | C | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | R | L | E | P | E | D | F | A | V | Y |
| 21-225_74B12_LC | . | . | . | . | . | . | . | . | . | . | . | A | . | . | . | . | . | . | . | . | . | . | . | . | . | . |
| 21-225_74B2_LC | . | . | . | . | . | . | . | . | . | . | . | . | . | . | . | . | . | . | . | . | . | . | . | . | . | . |
| 21-225_74C5_LC | . | . | . | . | . | . | . | . | . | . | . | N | . | I | . | . | . | . | . | . | . | . | . | . | . | . |
| 21-225_74C7_LC | . | . | . | . | . | . | . | . | . | . | . | . | . | . | . | . | . | . | . | . | . | . | . | . | . | F |
| 21-225_74D11_LC | . | . | . | . | . | . | . | . | . | . | . | A | . | . | . | . | . | . | . | . | . | . | . | . | . | . |
| 21-225_74D2_LC | . | . | . | . | . | . | . | . | . | . | . | . | . | . | . | N | . | . | . | . | . | . | . | . | . | . |
| 21-225_74D5_LC | . | . | . | . | . | . | . | . | . | . | . | I | . | I | . | . | . | . | . | . | . | . | . | . | . | . |
| 21-225_74D6_LC | . | . | . | . | C | . | . | . | . | . | . | A | . | . | . | . | . | . | . | . | . | . | . | . | . | . |
| 21-225_74F4_LC | . | . | . | . | . | . | . | . | . | . | . | . | . | . | . | . | . | . | . | . | . | . | . | . | . | . |
| 21-225_74F5_LC | . | . | . | . | . | . | . | . | . | . | . | A | . | . | . | . | . | . | . | . | . | . | . | . | . | . |
| 21-225_74G8_LC | . | . | . | . | . | . | . | . | . | . | . | . | . | . | . | G | . | . | . | . | . | . | . | . | . | . |
| 21-225_74H5_LC | . | . | . | . | . | . | . | . | . | . | . | I | . | I | . | . | . | . | . | . | . | . | . | . | . | . |
| 21-225_74H7_LC | . | . | . | . | . | . | . | . | . | . | . | . | . | . | . | . | . | . | . | . | . | . | . | . | . | . |
| 21-225_74H8_LC | . | . | . | . | . | . | . | . | . | . | . | . | . | . | . | . | . | . | . | . | . | . | . | . | . | . |
| 21-225_75A12_LC | . | . | . | . | . | . | . | . | . | . | . | . | . | . | . | . | . | . | . | . | A | . | . | . | . | . |
| 21-225_75A5_LC | . | . | . | . | . | . | . | . | . | . | . | A | . | . | . | . | . | . | . | . | . | . | . | . | . | . |
| 21-225_75A8_LC | . | . | . | . | . | . | . | . | . | . | . | A | . | . | . | . | . | V | . | . | . | . | C | . | . | . |
| 21-225_75B10_LC | . | . | . | . | . | . | . | . | . | . | . | . | . | . | . | . | . | . | . | . | . | . | . | . | . | . |
| 21-225_75C2_LC | . | . | . | . | . | . | . | . | . | . | . | A | . | . | . | . | . | . | . | . | . | . | C | . | . | . |
| 21-225_75C3_LC | . | . | . | . | . | . | . | . | . | . | . | . | . | . | . | . | . | . | . | . | . | . | . | . | . | . |
| 21-225_75D3_LC | . | . | . | . | C | . | . | . | . | . | . | A | . | . | . | . | . | V | . | H | . | . | C | . | . | . |
| 21-225_75D9_LC | . | . | . | . | . | . | . | . | . | . | . | . | . | . | . | . | . | . | . | . | . | . | . | . | . | . |
| 21-225_75F3_LC | . | . | . | . | C | . | . | . | . | . | . | A | . | . | . | . | . | . | . | H | . | . | . | . | . | . |
| 21-225_75G12_LC | . | . | . | . | . | . | . | . | . | . | . | A | . | . | . | . | . | V | . | . | . | . | C | . | . | . |
| 21-225_75G3_LC | . | . | . | . | . | . | . | . | . | . | . | . | . | . | . | . | . | . | . | . | . | . | . | . | . | F |
| 21-225_75G7_LC | . | . | . | . | . | . | . | . | . | . | . | . | . | . | . | . | . | . | . | . | . | . | . | . | . | . |
| 21-225_75G9_LC | . | . | . | . | . | . | . | . | . | . | . | . | . | . | . | . | . | . | . | . | . | . | . | . | . | . |
| 21-225_75H8_LC | . | . | . | . | . | . | . | . | . | . | . | . | . | . | . | . | . | . | . | . | . | . | . | . | . | . |
| 21-225_76A4_LC | . | . | . | . | . | . | . | . | . | . | . | A | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | R | L | E | P | E | D | F | A | V | Y |
| 21-225_76A7_LC | . | . | . | . | . | . | . | . | . | . | . | . | . | . | . | . | . | . | . | . | . | . | . | . | . | . |
| 21-225_76C9_LC | . | . | . | . | . | . | . | . | . | . | . | . | . | . | . | . | . | . | . | . | . | . | . | . | . | . |
| 21-225_76D1_LC | . | . | . | . | . | . | . | . | . | . | . | . | . | . | . | . | . | . | . | . | . | . | . | V | . | . |
| 21-225_76E10_LC | . | . | . | . | . | . | . | . | . | . | . | I | . | I | . | . | . | . | . | . | . | . | . | . | . | . |
| 21-225_76G4_LC | . | . | . | . | . | . | . | . | . | . | . | A | . | . | . | . | . | . | . | . | . | . | C | . | . | . |
| 21-225_77A10_LC | . | . | . | . | . | . | . | . | . | . | . | . | . | . | . | . | . | . | . | . | . | . | . | . | . | . |
| 21-225_77C12_LC | . | . | . | . | . | . | . | . | . | . | . | . | . | . | . | . | . | . | . | . | . | . | . | . | . | . |
| 21-225_77C5_LC | . | . | . | . | . | . | . | . | . | . | . | A | . | . | . | . | . | . | . | . | . | . | . | . | . | . |
| 21-225_77F7_LC | . | . | . | . | C | . | . | . | . | . | . | A | . | . | . | . | . | V | . | H | . | . | C | . | . | . |
| 21-225_77G1_LC | . | . | . | . | . | . | . | . | . | . | . | A | . | . | . | . | . | . | . | . | . | . | C | . | . | . |
| 21-225_78E7_LC | . | . | . | . | . | . | . | . | . | . | . | . | . | . | . | . | . | . | . | . | . | . | . | . | . | F |
| 21-225_79F11_LC | . | . | . | . | . | . | . | . | . | . | . | A | . | . | . | . | . | . | . | . | . | . | . | . | . | . |
| 21-225_79F3_LC | . | . | . | . | . | . | . | . | . | . | . | A | . | . | . | . | . | . | . | . | . | . | . | . | . | . |
| 21-225_79G1_LC | . | . | . | . | . | . | . | . | . | . | . | A | . | . | . | . | . | . | . | . | . | . | . | . | . | . |
| 21-225_79G12_LC | . | . | . | . | . | . | . | . | . | . | . | A | . | . | . | . | . | . | . | . | . | . | C | . | . | . |
| 21-225_80A1_LC | . | . | . | . | C | . | . | . | . | . | . | A | . | . | . | . | . | V | . | . | . | . | C | . | . | . |
| 21-225_80A6_LC | . | . | . | . | . | . | . | . | . | . | . | . | . | . | . | . | . | . | . | . | . | . | . | . | . | . |
| 21-225_80B10_LC | . | . | . | . | . | . | . | . | . | . | . | . | . | . | . | . | . | . | . | . | . | . | . | . | . | . |
| 21-225_80C1_LC | . | . | . | . | . | . | . | . | . | . | . | A | . | . | . | . | . | V | . | . | . | . | C | . | . | . |
| 21-225_80D5_LC | . | . | . | . | . | . | . | . | . | . | . | . | . | . | . | T | . | . | . | . | . | . | . | . | . | . |
| 21-225_80E12_LC | . | . | . | . | . | . | . | . | . | . | . | I | . | I | . | . | . | . | . | . | . | . | . | . | . | . |
| 21-225_80E3_LC | . | . | . | . | C | . | . | . | . | . | . | A | . | . | . | . | . | V | . | . | . | . | . | . | . | . |
| 21-225_80H12_LC | . | . | . | . | . | . | . | . | . | . | . | A | . | . | . | . | . | . | . | . | . | . | . | . | . | . |
| 21-225_80H7_LC | . | . | . | . | . | . | . | . | . | . | . | . | . | . | . | . | . | . | . | . | . | . | . | . | . | F |
| 21-225_81C11_LC | . | . | . | . | C | . | . | . | . | . | . | A | . | . | . | . | . | V | . | . | . | . | C | . | . | . |
| 21-225_81C5_LC | . | . | . | . | C | . | . | . | . | . | . | A | . | . | . | . | . | V | . | . | . | . | C | . | . | . |
| 21-225_83B6_LC | . | . | . | . | . | . | . | . | . | . | . | . | . | . | . | . | . | . | . | . | . | . | . | . | . | . |
| 21-225_83B7_LC | . | . | . | . | . | . | . | . | . | . | . | A | . | . | . | . | . | . | . | . | . | . | C | . | . | . |
| 21-225_83C10_LC | . | . | . | . | . | . | . | . | . | . | . | I | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | R | L | E | P | E | D | F | A | V | Y |
| 21-225_83G1_LC | . | . | . | . | . | . | . | . | . | . | . | A | . | . | . | . | . | V | . | . | . | . | C | . | . | . |
| 21-225_84E12_LC | . | . | . | . | . | . | . | . | . | . | . | . | . | . | . | . | . | . | . | . | D | . | . | . | . | F |
| 21-225_85A3_LC | . | . | . | . | . | . | . | . | . | . | . | A | . | . | . | . | . | . | . | . | . | . | . | . | . | . |
| 21-225_85B4_LC | . | . | . | . | . | . | . | . | . | . | . | A | . | . | . | . | . | . | . | . | . | . | . | . | . | . |
| 21-225_85B9_LC | . | . | . | . | . | . | . | . | . | . | . | I | . | I | . | . | . | . | . | . | . | . | . | . | . | . |
| 21-225_85D6_LC | . | . | . | . | . | . | . | . | . | . | . | . | . | . | . | . | . | . | . | . | . | . | . | . | . | . |
| 21-225_85G10_LC | . | . | . | . | . | . | . | . | . | . | . | . | . | . | . | . | . | . | . | . | . | . | . | . | . | . |
| 21-225_85G6_LC | . | . | . | . | . | . | . | . | . | . | . | . | . | . | . | . | . | . | . | . | . | . | . | V | . | . |
| 21-225_86C1_LC | . | . | . | . | . | . | . | . | . | . | . | A | . | . | . | . | . | . | . | . | . | . | C | . | . | . |
| 21-225_86C11_LC | . | . | . | . | . | . | . | . | . | . | . | A | . | . | . | . | . | . | . | . | . | . | C | . | . | . |
| 21-225_86E4_LC | . | . | . | . | . | . | . | . | . | . | . | A | . | . | . | . | . | . | . | . | . | . | C | . | . | . |
| 21-225_87A12_LC | . | . | . | . | C | . | . | . | . | . | . | A | . | . | . | . | . | V | . | . | . | . | C | . | . | . |
| 21-225_87C9_LC | . | . | . | . | . | . | . | . | . | . | . | A | . | . | . | . | . | . | . | . | . | . | C | . | . | . |
| 21-225_87E5_LC | . | . | . | . | . | . | . | . | . | . | . | A | . | . | . | . | . | V | . | . | . | . | C | . | . | . |
| 21-225_87H1_LC | . | . | . | . | . | . | . | . | . | . | . | . | . | . | . | . | . | . | . | . | . | . | . | . | . | . |
| 21-225_88E7_LC | . | . | . | . | . | . | . | . | . | . | . | A | . | . | . | . | . | V | . | . | . | . | C | . | . | . |
| 21-225_88F7_LC | . | . | . | . | . | . | . | . | . | . | . | A | . | . | . | . | . | . | . | . | . | . | C | . | . | . |
| 21-225_88G9_LC | . | . | . | . | . | . | . | . | . | . | . | A | . | . | . | . | . | V | . | . | . | . | C | . | . | . |
| 21-225_88H1_LC | . | . | . | . | C | . | . | . | . | . | . | A | . | . | . | . | . | V | . | . | . | . | C | . | . | . |
| 21-225_89A11_LC | . | . | . | . | . | . | . | . | . | . | . | . | . | . | . | . | . | . | . | . | . | . | . | . | . | . |
| 21-225_89D5_LC | . | . | . | . | . | . | . | . | . | . | . | A | . | . | . | . | . | . | . | . | . | . | . | . | . | . |
| 21-225_89E10_LC | . | . | . | . | . | . | . | . | . | . | . | A | . | . | . | . | . | V | . | . | . | . | . | . | . | . |
| 21-225_89H5_LC | . | . | . | . | . | . | . | . | . | . | . | N | . | I | . | . | . | . | . | . | . | . | . | . | . | . |
| 21-225_90A5_LC | . | . | . | . | . | . | . | . | . | . | . | A | . | . | . | . | . | V | . | H | . | . | . | . | . | . |
| 21-225_90F10_LC | . | . | . | . | . | . | . | . | . | . | . | A | . | . | . | . | . | . | . | . | . | . | C | . | . | . |
| 21-225_90G4_LC | . | . | . | . | . | . | . | . | . | . | . | A | . | . | . | . | . | V | . | . | . | . | C | . | . | . |
| 21-225_90G5_LC | . | . | . | . | . | . | . | . | . | . | . | A | . | . | . | . | . | V | . | H | . | . | . | . | . | . |
| 21-225_91B2_LC | . | . | . | . | . | . | . | . | . | . | . | A | . | . | . | . | . | V | . | . | . | . | C | . | . | . |
| 21-225_91B3_LC | . | . | . | . | . | . | . | . | . | . | . | A | . | . | . | . | . | V | . | H | . | . | C | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | R | L | E | P | E | D | F | A | V | Y |
| 21-225_91B4_LC | . | . | . | . | . | . | . | . | . | . | . | A | . | . | . | . | . | V | . | . | . | . | C | . | . | . |
| 21-225_91E9_LC | . | . | . | . | C | . | . | . | . | . | . | A | . | . | . | . | . | V | . | H | . | . | C | . | . | . |
| 21-225_91F3_LC | . | . | . | . | . | . | . | . | . | . | . | A | . | . | . | . | . | V | . | . | . | . | C | . | . | . |
| 21-225_92B1_LC | . | . | . | . | . | . | . | . | . | . | . | . | . | . | . | . | . | . | . | . | . | . | . | . | . | . |
| 21-225_92D6_LC | . | . | . | . | C | . | . | . | . | . | . | A | . | . | . | . | . | . | . | . | . | . | C | . | . | . |
| 21-225_93C2_LC | . | . | . | . | . | . | . | . | . | . | . | A | . | . | . | . | . | . | . | H | . | . | C | . | . | . |
| 21-225_93E4_LC | . | . | . | . | C | . | . | . | . | . | . | A | . | . | . | . | . | V | . | . | . | . | . | . | . | . |
| 21-225_93E9_LC | . | . | . | . | . | . | . | . | . | . | . | . | . | . | . | . | . | . | . | . | . | . | . | . | . | . |
| 21-225_94D3_LC | . | . | . | . | . | . | . | . | . | . | . | . | . | . | . | . | . | . | . | . | . | . | . | . | . | . |
| 21-225_94F12_LC | . | . | . | . | . | . | . | . | . | . | . | A | . | . | . | . | . | . | . | . | . | . | . | . | . | . |
| 21-225_94G10_LC | . | . | . | . | . | . | . | . | . | . | . | I | . | I | . | . | . | . | . | . | . | . | . | . | . | . |
| 21-225_95D2_LC | . | . | . | . | . | . | . | . | . | . | . | A | . | . | . | . | . | . | . | . | . | . | C | . | . | . |
| 21-225_95F9_LC | . | . | . | . | C | . | . | . | . | . | . | A | . | . | . | . | . | . | . | . | . | . | . | . | . | . |
| 21-225_95G9_LC | . | . | . | . | . | . | . | . | . | . | . | A | . | . | . | . | . | V | . | H | . | . | C | . | . | . |
| 21-225_95H10_LC | . | . | . | . | . | . | . | . | . | . | . | A | . | . | . | . | . | . | . | . | . | . | . | . | . | . |
| 21-225_97A2_LC | . | . | . | . | C | . | . | . | . | . | . | A | . | . | . | . | . | . | . | . | . | . | C | . | . | . |
| 21-225_97E5_LC | . | . | . | . | C | . | . | . | . | . | . | A | . | . | . | . | . | V | . | . | . | . | C | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | D | N | - | - | - | - | - | - | - | - | - | - | - | - | - | - | - | - | - | - | - |
| 21-225_147G8_LC | . | . | . | . | . | . | S | . | . | . | . | . | . | . | . | . | . | . | . | . | . | . | . | . | . | . |
| 21-225_190A3_LC | . | . | . | . | . | G | . | . | . | . | . | . | . | . | . | . | . | . | . | . | . | . | . | . | . | . |
| 21-225_190A7_LC | . | . | . | . | . | G | . | . | . | . | . | . | . | . | . | . | . | . | . | . | . | . | . | . | . | . |
| 21-225_190B4_LC | . | . | . | . | . | G | . | . | . | . | . | . | . | . | . | . | . | . | . | . | . | . | . | . | . | . |
| 21-225_190G10_LC | . | . | . | . | . | G | S | . | . | . | . | . | . | . | . | . | . | . | . | . | . | . | . | . | . | . |
| 21-225_191A3_LC | . | . | . | . | . | G | . | . | . | . | . | . | . | . | . | . | . | . | . | . | . | . | . | . | . | . |
| 21-225_191A5_LC | . | . | . | . | . | G | G | . | . | . | . | . | . | . | . | . | . | . | . | . | . | . | . | . | . | . |
| 21-225_191C3_LC | . | . | . | . | . | G | . | . | . | . | . | . | . | . | . | . | . | . | . | . | . | . | . | . | . | . |
| 21-225_191D3_LC | . | . | . | . | . | G | S | . | . | . | . | . | . | . | . | . | . | . | . | . | . | . | . | . | . | . |
| 21-225_191F1_LC | . | . | . | . | . | G | R | . | . | . | . | . | . | . | . | . | . | . | . | . | . | . | . | . | . | . |
| 21-225_191H7_LC | F | . | . | . | . | G | S | . | . | . | . | . | . | . | . | . | . | . | . | . | . | . | . | . | . | . |
| 21-225_192B3_LC | . | . | . | . | . | G | . | . | . | . | . | . | . | . | . | . | . | . | . | . | . | . | . | . | . | . |
| 21-225_192F8_LC | . | . | . | . | . | E | S | . | . | . | . | . | . | . | . | . | . | . | . | . | . | . | . | . | . | . |
| 21-225_192G12_LC | . | . | . | . | . | G | . | . | . | . | . | . | . | . | . | . | . | . | . | . | . | . | . | . | . | . |
| 21-225_192H3_LC | . | . | . | . | . | G | I | . | . | . | . | . | . | . | . | . | . | . | . | . | . | . | . | . | . | . |
| 21-225_193A1_LC | . | . | . | . | . | G | . | . | . | . | . | . | . | . | . | . | . | . | . | . | . | . | . | . | . | . |
| 21-225_193B1_LC | F | . | . | . | . | G | . | . | . | . | . | . | . | . | . | . | . | . | . | . | . | . | . | . | . | . |
| 21-225_193B10_LC | . | . | . | . | . | G | S | . | . | . | . | . | . | . | . | . | . | . | . | . | . | . | . | . | . | . |
| 21-225_193B12_LC | . | . | . | . | . | G | . | . | . | . | . | . | . | . | . | . | . | . | . | . | . | . | . | . | . | . |
| 21-225_193C8_LC | . | . | . | . | . | G | . | . | . | . | . | . | . | . | . | . | . | . | . | . | . | . | . | . | . | . |
| 21-225_193D3_LC | . | . | . | . | . | G | . | . | . | . | . | . | . | . | . | . | . | . | . | . | . | . | . | . | . | . |
| 21-225_193D8_LC | . | . | . | . | . | G | . | . | . | . | . | . | . | . | . | . | . | . | . | . | . | . | . | . | . | . |
| 21-225_193E6_LC | . | . | . | . | . | E | S | . | . | . | . | . | . | . | . | . | . | . | . | . | . | . | . | . | . | . |
| 21-225_193F3_LC | . | . | . | . | . | G | S | . | . | . | . | . | . | . | . | . | . | . | . | . | . | . | . | . | . | . |
| 21-225_193G8_LC | . | . | . | . | . | G | . | . | . | . | . | . | . | . | . | . | . | . | . | . | . | . | . | . | . | . |
| 21-225_193H6_LC | F | . | . | . | . | G | S | . | . | . | . | . | . | . | . | . | . | . | . | . | . | . | . | . | . | . |
| 21-225_194C10_LC | F | . | . | . | . | E | S | . | . | . | . | . | . | . | . | . | . | . | . | . | . | . | . | . | . | . |
| 21-225_194E5_LC | . | . | . | . | . | G | S | . | . | . | . | . | . | . | . | . | . | . | . | . | . | . | . | . | . | . |
| 21-225_194E6_LC | . | . | . | . | . | G | S | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | D | N | - | - | - | - | - | - | - | - | - | - | - | - | - | - | - | - | - | - | - |
| 21-225_195B1_LC | F | . | . | . | . | E | S | . | . | . | . | . | . | . | . | . | . | . | . | . | . | . | . | . | . | . |
| 21-225_195C8_LC | . | . | . | . | . | G | . | . | . | . | . | . | . | . | . | . | . | . | . | . | . | . | . | . | . | . |
| 21-225_196G10_LC | . | . | . | . | . | G | S | . | . | . | . | . | . | . | . | . | . | . | . | . | . | . | . | . | . | . |
| 21-225_196H12_LC | . | . | . | . | . | G | . | . | . | . | . | . | . | . | . | . | . | . | . | . | . | . | . | . | . | . |
| 21-225_197F4_LC | . | . | . | . | . | G | . | . | . | . | . | . | . | . | . | . | . | . | . | . | . | . | . | . | . | . |
| 21-225_198B1_LC | . | . | . | . | . | G | S | . | . | . | . | . | . | . | . | . | . | . | . | . | . | . | . | . | . | . |
| 21-225_198C2_LC | . | . | . | . | . | G | . | . | . | . | . | . | . | . | . | . | . | . | . | . | . | . | . | . | . | . |
| 21-225_198E1_LC | . | . | . | . | . | G | . | . | . | . | . | . | . | . | . | . | . | . | . | . | . | . | . | . | . | . |
| 21-225_198G10_LC | . | . | . | . | . | G | . | . | . | . | . | . | . | . | . | . | . | . | . | . | . | . | . | . | . | . |
| 21-225_199C2_LC | . | . | . | . | . | G | . | . | . | . | . | . | . | . | . | . | . | . | . | . | . | . | . | . | . | . |
| 21-225_199C7_LC | . | . | . | . | . | G | . | . | . | . | . | . | . | . | . | . | . | . | . | . | . | . | . | . | . | . |
| 21-225_199G11_LC | . | . | . | . | . | G | . | . | . | . | . | . | . | . | . | . | . | . | . | . | . | . | . | . | . | . |
| 21-225_200A1_LC | C | . | . | . | . | E | I | . | . | . | . | . | . | . | . | . | . | . | . | . | . | . | . | . | . | . |
| 21-225_200F10_LC | . | . | . | . | . | G | . | . | . | . | . | . | . | . | . | . | . | . | . | . | . | . | . | . | . | . |
| 21-225_201B2_LC | F | . | . | . | . | E | . | . | . | . | . | . | . | . | . | . | . | . | . | . | . | . | . | . | . | . |
| 21-225_201E1_LC | H | . | H | . | . | E | T | . | . | . | . | . | . | . | . | . | . | . | . | . | . | . | . | . | . | . |
| 21-225_202D11_LC | . | . | . | . | . | E | . | . | . | . | . | . | . | . | . | . | . | . | . | . | . | . | . | . | . | . |
| 21-225_202D9_LC | . | . | . | . | . | E | T | . | . | . | . | . | . | . | . | . | . | . | . | . | . | . | . | . | . | . |
| 21-225_205G7_LC | . | . | . | . | . | E | S | . | . | . | . | . | . | . | . | . | . | . | . | . | . | . | . | . | . | . |
| 21-225_208A9_LC | . | . | . | H | . | H | S | . | . | . | . | . | . | . | . | . | . | . | . | . | . | . | . | . | . | . |
| 21-225_208B5_LC | F | . | . | H | . | E | . | . | . | . | . | . | . | . | . | . | . | . | . | . | . | . | . | . | . | . |
| 21-225_220E1_LC | . | . | . | . | . | G | S | . | . | . | . | . | . | . | . | . | . | . | . | . | . | . | . | . | . | . |
| 21-225_222C7_LC | . | . | . | . | . | E | D | . | . | . | . | . | . | . | . | . | . | . | . | . | . | . | . | . | . | . |
| 21-225_225E7_LC | . | . | . | . | . | G | T | . | . | . | . | . | . | . | . | . | . | . | . | . | . | . | . | . | . | . |
| 21-225_23B9_LC | . | . | . | . | . | G | S | . | . | . | . | . | . | . | . | . | . | . | . | . | . | . | . | . | . | . |
| 21-225_44F6_LC | . | . | . | . | . | G | S | . | . | . | . | . | . | . | . | . | . | . | . | . | . | . | . | . | . | . |
| 21-225_59B2_LC | . | . | . | . | . | G | T | . | . | . | . | . | . | . | . | . | . | . | . | . | . | . | . | . | . | . |
| 21-225_74A3_LC | . | . | . | H | S | . | . | . | . | . | . | . | . | . | . | . | . | . | . | . | . | . | . | . | . | . |
| 21-225_74A4_LC | . | . | . | H | S | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | D | N | - | - | - | - | - | - | - | - | - | - | - | - | - | - | - | - | - | - | - |
| 21-225_74B12_LC | . | . | . | H | . | . | . | . | . | . | . | . | . | . | . | . | . | . | . | . | . | . | . | . | . | . |
| 21-225_74B2_LC | C | . | . | . | . | E | S | . | . | . | . | . | . | . | . | . | . | . | . | . | . | . | . | . | . | . |
| 21-225_74C5_LC | . | . | . | . | . | E | S | . | . | . | . | . | . | . | . | . | . | . | . | . | . | . | . | . | . | . |
| 21-225_74C7_LC | . | . | . | . | . | E | R | . | . | . | . | . | . | . | . | . | . | . | . | . | . | . | . | . | . | . |
| 21-225_74D11_LC | . | . | . | H | . | . | . | . | . | . | . | . | . | . | . | . | . | . | . | . | . | . | . | . | . | . |
| 21-225_74D2_LC | F | . | . | . | . | E | S | . | . | . | . | . | . | . | . | . | . | . | . | . | . | . | . | . | . | . |
| 21-225_74D5_LC | . | . | . | . | . | E | S | . | . | . | . | . | . | . | . | . | . | . | . | . | . | . | . | . | . | . |
| 21-225_74D6_LC | . | . | . | H | . | . | . | . | . | . | . | . | . | . | . | . | . | . | . | . | . | . | . | . | . | . |
| 21-225_74F4_LC | . | . | . | . | . | G | - | . | . | . | . | . | . | . | . | . | . | . | . | . | . | . | . | . | . | . |
| 21-225_74F5_LC | . | . | . | H | S | . | . | . | . | . | . | . | . | . | . | . | . | . | . | . | . | . | . | . | . | . |
| 21-225_74G8_LC | . | . | . | . | . | G | - | . | . | . | . | . | . | . | . | . | . | . | . | . | . | . | . | . | . | . |
| 21-225_74H5_LC | . | . | . | . | . | E | S | . | . | . | . | . | . | . | . | . | . | . | . | . | . | . | . | . | . | . |
| 21-225_74H7_LC | C | . | . | . | . | E | S | . | . | . | . | . | . | . | . | . | . | . | . | . | . | . | . | . | . | . |
| 21-225_74H8_LC | . | . | . | . | . | G | - | . | . | . | . | . | . | . | . | . | . | . | . | . | . | . | . | . | . | . |
| 21-225_75A12_LC | . | . | . | H | . | . | S | . | . | . | . | . | . | . | . | . | . | . | . | . | . | . | . | . | . | . |
| 21-225_75A5_LC | . | . | . | H | . | . | . | . | . | . | . | . | . | . | . | . | . | . | . | . | . | . | . | . | . | . |
| 21-225_75A8_LC | . | . | . | H | S | . | . | . | . | . | . | . | . | . | . | . | . | . | . | . | . | . | . | . | . | . |
| 21-225_75B10_LC | F | . | . | . | . | E | D | . | . | . | . | . | . | . | . | . | . | . | . | . | . | . | . | . | . | . |
| 21-225_75C2_LC | . | . | . | H | . | . | . | . | . | . | . | . | . | . | . | . | . | . | . | . | . | . | . | . | . | . |
| 21-225_75C3_LC | . | . | . | H | . | . | S | . | . | . | . | . | . | . | . | . | . | . | . | . | . | . | . | . | . | . |
| 21-225_75D3_LC | . | . | . | H | . | . | . | . | . | . | . | . | . | . | . | . | . | . | . | . | . | . | . | . | . | . |
| 21-225_75D9_LC | . | . | . | . | . | E | S | . | . | . | . | . | . | . | . | . | . | . | . | . | . | . | . | . | . | . |
| 21-225_75F3_LC | . | . | . | H | . | . | . | . | . | . | . | . | . | . | . | . | . | . | . | . | . | . | . | . | . | . |
| 21-225_75G12_LC | . | . | . | H | . | . | . | . | . | . | . | . | . | . | . | . | . | . | . | . | . | . | . | . | . | . |
| 21-225_75G3_LC | . | . | . | . | . | E | R | . | . | . | . | . | . | . | . | . | . | . | . | . | . | . | . | . | . | . |
| 21-225_75G7_LC | . | . | . | . | . | E | S | . | . | . | . | . | . | . | . | . | . | . | . | . | . | . | . | . | . | . |
| 21-225_75G9_LC | . | . | . | . | . | E | S | . | . | . | . | . | . | . | . | . | . | . | . | . | . | . | . | . | . | . |
| 21-225_75H8_LC | C | . | . | . | . | E | S | . | . | . | . | . | . | . | . | . | . | . | . | . | . | . | . | . | . | . |
| 21-225_76A4_LC | . | . | . | H | S | . | . | . | . | . | . | . | . | . | . | . | . | . | . | . | . | . | . | . | . | . |

# FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | D | N | - | - | - | - | - | - | - | - | - | - | - | - | - | - | - | - | - | - | - |
| 21-225_76A7_LC | . | . | . | . | . | E | S | . | . | . | . | . | . | . | . | . | . | . | . | . | . | . | . | . | . | . |
| 21-225_76C9_LC | . | . | . | . | . | G | - | . | . | . | . | . | . | . | . | . | . | . | . | . | . | . | . | . | . | . |
| 21-225_76D1_LC | . | . | . | . | . | E | S | . | . | . | . | . | . | . | . | . | . | . | . | . | . | . | . | . | . | . |
| 21-225_76E10_LC | . | . | . | . | . | E | S | . | . | . | . | . | . | . | . | . | . | . | . | . | . | . | . | . | . | . |
| 21-225_76G4_LC | . | . | . | H | S | . | . | . | . | . | . | . | . | . | . | . | . | . | . | . | . | . | . | . | . | . |
| 21-225_77A10_LC | . | . | . | H | . | . | S | . | . | . | . | . | . | . | . | . | . | . | . | . | . | . | . | . | . | . |
| 21-225_77C12_LC | . | . | . | . | . | E | S | . | . | . | . | . | . | . | . | . | . | . | . | . | . | . | . | . | . | . |
| 21-225_77C5_LC | . | . | . | H | . | . | . | . | . | . | . | . | . | . | . | . | . | . | . | . | . | . | . | . | . | . |
| 21-225_77F7_LC | . | . | . | H | . | . | . | . | . | . | . | . | . | . | . | . | . | . | . | . | . | . | . | . | . | . |
| 21-225_77G1_LC | . | . | . | H | . | . | . | . | . | . | . | . | . | . | . | . | . | . | . | . | . | . | . | . | . | . |
| 21-225_78E7_LC | . | . | . | . | . | E | H | . | . | . | . | . | . | . | . | . | . | . | . | . | . | . | . | . | . | . |
| 21-225_79F11_LC | . | . | . | H | S | . | . | . | . | . | . | . | . | . | . | . | . | . | . | . | . | . | . | . | . | . |
| 21-225_79F3_LC | . | . | . | H | . | . | . | . | . | . | . | . | . | . | . | . | . | . | . | . | . | . | . | . | . | . |
| 21-225_79G1_LC | . | . | . | H | . | . | . | . | . | . | . | . | . | . | . | . | . | . | . | . | . | . | . | . | . | . |
| 21-225_79G12_LC | . | . | . | H | S | . | . | . | . | . | . | . | . | . | . | . | . | . | . | . | . | . | . | . | . | . |
| 21-225_80A1_LC | . | . | . | H | S | . | . | . | . | . | . | . | . | . | . | . | . | . | . | . | . | . | . | . | . | . |
| 21-225_80A6_LC | . | . | . | . | . | E | S | . | . | . | . | . | . | . | . | . | . | . | . | . | . | . | . | . | . | . |
| 21-225_80B10_LC | . | . | . | . | . | E | S | . | . | . | . | . | . | . | . | . | . | . | . | . | . | . | . | . | . | . |
| 21-225_80C1_LC | . | . | . | H | S | . | . | . | . | . | . | . | . | . | . | . | . | . | . | . | . | . | . | . | . | . |
| 21-225_80D5_LC | C | . | . | . | . | E | S | . | . | . | . | . | . | . | . | . | . | . | . | . | . | . | . | . | . | . |
| 21-225_80E12_LC | . | . | . | . | . | E | S | . | . | . | . | . | . | . | . | . | . | . | . | . | . | . | . | . | . | . |
| 21-225_80E3_LC | . | . | . | H | S | . | . | . | . | . | . | . | . | . | . | . | . | . | . | . | . | . | . | . | . | . |
| 21-225_80H12_LC | . | . | . | H | S | . | . | . | . | . | . | . | . | . | . | . | . | . | . | . | . | . | . | . | . | . |
| 21-225_80H7_LC | . | . | . | . | . | E | S | . | . | . | . | . | . | . | . | . | . | . | . | . | . | . | . | . | . | . |
| 21-225_81C11_LC | . | . | . | H | S | . | . | . | . | . | . | . | . | . | . | . | . | . | . | . | . | . | . | . | . | . |
| 21-225_81C5_LC | . | . | . | H | S | . | . | . | . | . | . | . | . | . | . | . | . | . | . | . | . | . | . | . | . | . |
| 21-225_83B6_LC | . | . | . | . | . | E | S | . | . | . | . | . | . | . | . | . | . | . | . | . | . | . | . | . | . | . |
| 21-225_83B7_LC | . | . | . | H | S | . | . | . | . | . | . | . | . | . | . | . | . | . | . | . | . | . | . | . | . | . |
| 21-225_83C10_LC | . | . | . | . | . | E | S | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | D | N | - | - | - | - | - | - | - | - | - | - | - | - | - | - | - | - | - | - | - |
| 21-225_83G1_LC | . | . | . | H | S | . | . | . | . | . | . | . | . | . | . | . | . | . | . | . | . | . | . | . | . | . |
| 21-225_84E12_LC | . | . | . | . | . | E | S | . | . | . | . | . | . | . | . | . | . | . | . | . | . | . | . | . | . | . |
| 21-225_85A3_LC | . | . | . | H | . | . | . | . | . | . | . | . | . | . | . | . | . | . | . | . | . | . | . | . | . | . |
| 21-225_85B4_LC | . | . | . | H | . | . | . | . | . | . | . | . | . | . | . | . | . | . | . | . | . | . | . | . | . | . |
| 21-225_85B9_LC | . | . | . | . | . | E | S | . | . | . | . | . | . | . | . | . | . | . | . | . | . | . | . | . | . | . |
| 21-225_85D6_LC | . | . | . | H | . | . | S | . | . | . | . | . | . | . | . | . | . | . | . | . | . | . | . | . | . | . |
| 21-225_85G10_LC | F | . | . | . | . | E | S | . | . | . | . | . | . | . | . | . | . | . | . | . | . | . | . | . | . | . |
| 21-225_85G6_LC | . | . | . | . | . | E | S | . | . | . | . | . | . | . | . | . | . | . | . | . | . | . | . | . | . | . |
| 21-225_86C1_LC | . | . | . | H | . | . | . | . | . | . | . | . | . | . | . | . | . | . | . | . | . | . | . | . | . | . |
| 21-225_86C11_LC | . | . | . | H | S | . | . | . | . | . | . | . | . | . | . | . | . | . | . | . | . | . | . | . | . | . |
| 21-225_86E4_LC | . | . | . | H | S | . | . | . | . | . | . | . | . | . | . | . | . | . | . | . | . | . | . | . | . | . |
| 21-225_87A12_LC | . | . | . | H | S | . | . | . | . | . | . | . | . | . | . | . | . | . | . | . | . | . | . | . | . | . |
| 21-225_87C9_LC | . | . | . | H | S | . | . | . | . | . | . | . | . | . | . | . | . | . | . | . | . | . | . | . | . | . |
| 21-225_87E5_LC | . | . | . | H | S | . | . | . | . | . | . | . | . | . | . | . | . | . | . | . | . | . | . | . | . | . |
| 21-225_87H1_LC | S | . | . | . | . | E | S | . | . | . | . | . | . | . | . | . | . | . | . | . | . | . | . | . | . | . |
| 21-225_88E7_LC | . | . | . | H | S | . | . | . | . | . | . | . | . | . | . | . | . | . | . | . | . | . | . | . | . | . |
| 21-225_88F7_LC | . | . | . | H | S | . | . | . | . | . | . | . | . | . | . | . | . | . | . | . | . | . | . | . | . | . |
| 21-225_88G9_LC | . | . | . | H | S | . | . | . | . | . | . | . | . | . | . | . | . | . | . | . | . | . | . | . | . | . |
| 21-225_88H1_LC | . | . | . | H | S | . | . | . | . | . | . | . | . | . | . | . | . | . | . | . | . | . | . | . | . | . |
| 21-225_89A11_LC | S | . | . | . | . | E | I | . | . | . | . | . | . | . | . | . | . | . | . | . | . | . | . | . | . | . |
| 21-225_89D5_LC | . | . | . | H | . | . | . | . | . | . | . | . | . | . | . | . | . | . | . | . | . | . | . | . | . | . |
| 21-225_89E10_LC | . | . | . | H | S | . | . | . | . | . | . | . | . | . | . | . | . | . | . | . | . | . | . | . | . | . |
| 21-225_89H5_LC | . | . | . | . | . | E | S | . | . | . | . | . | . | . | . | . | . | . | . | . | . | . | . | . | . | . |
| 21-225_90A5_LC | . | . | . | H | . | . | . | . | . | . | . | . | . | . | . | . | . | . | . | . | . | . | . | . | . | . |
| 21-225_90F10_LC | . | . | . | H | . | . | . | . | . | . | . | . | . | . | . | . | . | . | . | . | . | . | . | . | . | . |
| 21-225_90G4_LC | . | . | . | H | S | . | . | . | . | . | . | . | . | . | . | . | . | . | . | . | . | . | . | . | . | . |
| 21-225_90G5_LC | . | . | . | H | . | . | . | . | . | . | . | . | . | . | . | . | . | . | . | . | . | . | . | . | . | . |
| 21-225_91B2_LC | . | . | . | H | . | . | . | . | . | . | . | . | . | . | . | . | . | . | . | . | . | . | . | . | . | . |
| 21-225_91B3_LC | . | . | . | H | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | D | N | - | - | - | - | - | - | - | - | - | - | - | - | - | - | - | - | - | - | - |
| 21-225_91B4_LC | . | . | . | H | . | . | . | . | . | . | . | . | . | . | . | . | . | . | . | . | . | . | . | . | . | . |
| 21-225_91E9_LC | . | . | . | H | S | . | . | . | . | . | . | . | . | . | . | . | . | . | . | . | . | . | . | . | . | . |
| 21-225_91F3_LC | . | . | . | H | S | . | . | . | . | . | . | . | . | . | . | . | . | . | . | . | . | . | . | . | . | . |
| 21-225_92B1_LC | . | . | . | . | . | E | S | . | . | . | . | . | . | . | . | . | . | . | . | . | . | . | . | . | . | . |
| 21-225_92D6_LC | . | . | . | H | . | . | . | . | . | . | . | . | . | . | . | . | . | . | . | . | . | . | . | . | . | . |
| 21-225_93C2_LC | . | . | . | H | . | . | . | . | . | . | . | . | . | . | . | . | . | . | . | . | . | . | . | . | . | . |
| 21-225_93E4_LC | . | . | . | H | S | . | . | . | . | . | . | . | . | . | . | . | . | . | . | . | . | . | . | . | . | . |
| 21-225_93E9_LC | . | . | . | . | . | E | S | . | . | . | . | . | . | . | . | . | . | . | . | . | . | . | . | . | . | . |
| 21-225_94D3_LC | . | . | . | H | . | . | S | . | . | . | . | . | . | . | . | . | . | . | . | . | . | . | . | . | . | . |
| 21-225_94F12_LC | . | . | . | H | . | . | . | . | . | . | . | . | . | . | . | . | . | . | . | . | . | . | . | . | . | . |
| 21-225_94G10_LC | . | . | . | . | . | E | S | . | . | . | . | . | . | . | . | . | . | . | . | . | . | . | . | . | . | . |
| 21-225_95D2_LC | . | . | . | H | . | . | . | . | . | . | . | . | . | . | . | . | . | . | . | . | . | . | . | . | . | . |
| 21-225_95F9_LC | . | . | . | H | . | . | . | . | . | . | . | . | . | . | . | . | . | . | . | . | . | . | . | . | . | . |
| 21-225_95G9_LC | . | . | . | H | . | . | . | . | . | . | . | . | . | . | . | . | . | . | . | . | . | . | . | . | . | . |
| 21-225_95H10_LC | . | . | . | H | . | . | . | . | . | . | . | . | . | . | . | . | . | . | . | . | . | . | . | . | . | . |
| 21-225_97A2_LC | . | . | . | H | S | . | . | . | . | . | . | . | . | . | . | . | . | . | . | . | . | . | . | . | . | . |
| 21-225_97E5_LC | . | . | . | H | S | . | . | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | S | P | W | T | F | G | Q | G | T | K | V | E | I | K | R |
| 21-225_147G8_LC | . | . | . | . | . | . | . | . | . | . | . | . | . | . | . | . | . | N | . |
| 21-225_190A3_LC | . | . | . | . | . | . | . | . | . | . | . | . | . | . | . | . | . | . | . |
| 21-225_190A7_LC | . | . | . | . | . | . | . | . | . | . | . | . | . | . | . | . | . | . | . |
| 21-225_190B4_LC | . | . | . | . | . | . | . | A | . | . | . | . | . | . | . | . | . | . | . |
| 21-225_190G10_LC | . | . | . | S | P | . | . | . | . | . | . | . | . | . | . | . | . | . | . |
| 21-225_191A3_LC | . | . | . | . | . | . | . | A | . | . | . | . | . | . | . | . | V | . | . |
| 21-225_191A5_LC | . | . | . | S | P | . | . | . | . | V | . | . | . | . | . | . | . | . | . |
| 21-225_191C3_LC | . | . | . | . | . | . | . | A | . | . | . | . | . | . | . | . | . | . | . |
| 21-225_191D3_LC | . | . | . | . | . | . | . | . | . | . | . | . | . | . | . | . | . | . | . |
| 21-225_191F1_LC | . | . | . | . | . | . | . | . | . | . | . | . | . | . | . | . | V | . | . |
| 21-225_191H7_LC | . | . | . | . | . | . | . | . | . | . | . | . | . | . | . | . | . | N | . |
| 21-225_192B3_LC | . | . | . | . | . | . | . | A | . | . | . | . | . | . | . | . | . | . | . |
| 21-225_192F8_LC | . | . | . | . | . | . | . | . | . | . | . | . | . | . | . | . | . | . | . |
| 21-225_192G12_LC | . | . | . | . | . | . | . | . | . | . | . | . | . | . | . | . | . | . | . |
| 21-225_192H3_LC | . | . | . | . | . | . | . | . | . | . | . | . | . | . | . | . | . | . | . |
| 21-225_193A1_LC | . | . | . | . | . | . | . | . | . | . | . | . | . | . | . | . | . | . | . |
| 21-225_193B1_LC | . | . | . | . | . | . | . | . | . | . | . | . | . | . | . | . | . | . | . |
| 21-225_193B10_LC | . | . | . | S | P | . | . | . | . | . | . | . | . | . | . | . | . | . | . |
| 21-225_193B12_LC | . | . | . | . | . | . | . | A | . | . | . | . | . | . | . | . | . | . | . |
| 21-225_193C8_LC | . | . | . | . | . | . | . | A | . | . | . | . | I | . | . | . | V | . | . |
| 21-225_193D3_LC | . | . | . | . | . | . | . | A | . | . | . | . | . | . | . | . | . | . | . |
| 21-225_193D8_LC | . | . | . | . | . | . | . | . | . | . | . | . | . | . | . | . | . | . | . |
| 21-225_193E6_LC | . | . | . | . | . | . | . | . | . | . | . | . | . | . | . | . | . | . | . |
| 21-225_193F3_LC | . | . | . | . | . | . | . | . | . | . | . | . | . | . | . | . | . | N | . |
| 21-225_193G8_LC | . | . | . | . | . | . | . | . | . | . | . | . | . | . | . | . | . | . | . |
| 21-225_193H6_LC | . | . | . | . | . | . | . | . | . | . | . | . | . | . | . | . | . | N | . |
| 21-225_194C10_LC | . | . | . | . | . | . | . | . | . | . | . | . | . | . | . | . | . | . | . |
| 21-225_194E5_LC | . | . | . | . | . | . | . | . | . | . | . | . | . | . | . | . | . | . | . |
| 21-225_194E6_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | S | P | W | T | F | G | Q | G | T | K | V | E | I | K | R |
| 21-225_195B1_LC | . | . | . | . | . | . | . | . | . | . | . | . | . | . | . | . | . | . | . |
| 21-225_195C8_LC | . | . | . | . | . | . | . | A | . | . | . | . | . | . | . | . | . | . | . |
| 21-225_196G10_LC | . | . | . | S | P | . | . | . | . | . | . | . | . | . | . | . | L | . | . |
| 21-225_196H12_LC | . | . | . | . | . | . | . | . | . | . | . | . | . | . | . | . | . | . | . |
| 21-225_197F4_LC | . | . | . | . | . | . | . | A | . | . | . | . | . | . | . | . | . | . | . |
| 21-225_198B1_LC | . | . | . | . | . | . | . | . | . | . | . | . | . | . | . | . | . | . | . |
| 21-225_198C2_LC | . | . | . | . | . | . | . | . | L | . | H | A | . | . | . | . | . | . | . |
| 21-225_198E1_LC | . | . | . | . | . | . | . | . | . | . | . | . | . | . | . | . | . | . | . |
| 21-225_198G10_LC | . | . | . | . | . | . | . | A | . | . | . | . | . | . | . | . | . | . | . |
| 21-225_199C2_LC | . | . | . | . | . | . | . | A | . | . | . | . | . | . | . | . | . | . | . |
| 21-225_199C7_LC | . | . | . | . | . | . | . | . | . | . | . | . | . | . | . | . | . | . | . |
| 21-225_199G11_LC | . | . | . | . | . | . | . | . | . | . | H | . | . | . | . | . | . | . | . |
| 21-225_200A1_LC | . | . | . | . | . | . | . | . | . | . | . | . | . | . | . | . | S | . | . |
| 21-225_200F10_LC | . | . | . | . | . | . | . | A | . | . | . | . | . | . | . | . | . | . | . |
| 21-225_201B2_LC | . | . | . | . | . | . | . | . | . | . | . | . | . | . | . | . | . | . | . |
| 21-225_201E1_LC | . | . | . | . | . | . | . | . | . | . | . | . | . | . | . | . | . | . | . |
| 21-225_202D11_LC | . | . | . | . | . | . | . | . | . | . | . | . | . | . | . | . | . | . | . |
| 21-225_202D9_LC | . | . | . | . | . | . | . | . | . | . | . | . | . | . | . | . | . | . | . |
| 21-225_205G7_LC | . | . | . | . | . | . | . | . | . | . | . | . | . | . | . | . | . | . | . |
| 21-225_208A9_LC | . | . | . | . | . | . | . | . | . | . | . | . | . | . | . | . | . | . | . |
| 21-225_208B5_LC | . | . | . | . | . | . | . | . | . | . | . | . | . | . | . | . | . | . | . |
| 21-225_220E1_LC | . | . | . | . | . | . | . | . | . | . | . | . | . | . | . | . | . | . | . |
| 21-225_222C7_LC | . | . | . | . | . | . | . | . | . | . | . | . | . | . | . | . | . | . | . |
| 21-225_225E7_LC | . | . | . | . | . | . | R | . | . | . | . | . | . | . | . | . | . | . | . |
| 21-225_23B9_LC | . | . | . | . | . | . | R | . | . | . | . | . | . | . | . | . | . | . | . |
| 21-225_44F6_LC | . | . | . | . | . | . | . | . | . | . | . | . | . | . | . | . | . | . | . |
| 21-225_59B2_LC | . | . | . | . | . | . | . | . | . | . | . | . | . | . | . | . | . | . | . |
| 21-225_74A3_LC | . | . | . | . | . | . | . | . | . | . | . | . | . | . | . | . | . | . | . |
| 21-225_74A4_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | S | P | W | T | F | G | Q | G | T | K | V | E | I | K | R |
| 21-225_74B12_LC | . | . | . | . | . | . | . | . | . | . | . | . | . | . | . | . | . | . | . |
| 21-225_74B2_LC | . | . | . | . | . | . | . | . | . | . | . | . | . | . | . | . | . | . | . |
| 21-225_74C5_LC | . | . | . | . | . | . | . | . | . | . | . | . | . | . | . | . | . | . | . |
| 21-225_74C7_LC | . | . | . | . | . | . | . | . | . | . | . | . | . | . | . | . | . | . | . |
| 21-225_74D11_LC | . | . | . | . | . | . | . | . | . | . | . | . | . | . | . | . | . | . | . |
| 21-225_74D2_LC | . | . | . | . | . | . | . | . | . | . | . | . | . | . | . | . | . | . | . |
| 21-225_74D5_LC | . | . | . | . | . | . | . | . | . | . | . | . | . | . | . | . | . | . | . |
| 21-225_74D6_LC | . | . | . | . | . | . | . | . | . | . | . | . | . | . | . | . | . | . | . |
| 21-225_74F4_LC | . | . | . | . | . | S | R | . | . | . | . | . | . | . | . | . | . | . | . |
| 21-225_74F5_LC | . | . | . | . | . | . | . | . | . | . | . | . | . | . | . | . | . | . | . |
| 21-225_74G8_LC | . | . | . | . | N | S | R | . | . | . | . | . | . | . | . | . | . | . | . |
| 21-225_74H5_LC | . | . | . | . | . | . | . | . | . | . | . | . | . | . | . | . | . | . | . |
| 21-225_74H7_LC | . | . | . | . | . | . | . | . | . | . | . | . | . | . | . | . | . | . | . |
| 21-225_74H8_LC | . | . | . | . | . | S | R | . | . | . | . | . | . | . | . | . | . | . | . |
| 21-225_75A12_LC | . | . | . | . | . | . | . | . | . | . | . | . | . | . | . | . | . | . | . |
| 21-225_75A5_LC | . | . | . | . | . | . | . | . | . | . | . | . | . | . | . | . | . | . | . |
| 21-225_75A8_LC | . | . | . | . | . | . | . | . | . | . | . | . | . | . | . | . | . | . | . |
| 21-225_75B10_LC | . | . | . | . | . | . | . | . | . | . | . | . | . | . | . | . | . | . | . |
| 21-225_75C2_LC | . | . | . | . | . | . | . | . | . | . | R | . | . | . | . | . | . | . | T |
| 21-225_75C3_LC | . | . | . | . | . | . | . | . | . | . | . | . | . | . | . | . | . | . | . |
| 21-225_75D3_LC | . | . | . | . | . | . | . | . | . | . | . | . | . | . | . | . | . | . | . |
| 21-225_75D9_LC | . | . | . | . | . | . | . | . | . | . | . | . | . | . | . | . | . | . | . |
| 21-225_75F3_LC | . | . | . | . | . | . | . | . | . | . | . | . | . | . | . | . | . | . | . |
| 21-225_75G12_LC | . | . | . | . | . | . | . | . | . | . | . | . | . | . | . | . | . | . | . |
| 21-225_75G3_LC | . | . | . | . | . | . | . | . | . | . | . | . | . | . | . | . | . | . | . |
| 21-225_75G7_LC | . | . | . | . | . | . | . | . | . | . | . | . | . | . | . | . | . | . | . |
| 21-225_75G9_LC | . | . | . | . | . | . | . | . | . | . | . | . | . | . | . | . | . | . | . |
| 21-225_75H8_LC | . | . | . | . | . | . | . | . | . | . | . | . | . | . | . | . | . | . | . |
| 21-225_76A4_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | S | P | W | T | F | G | Q | G | T | K | V | E | I | K | R |
| 21-225_76A7_LC | . | . | . | . | . | . | . | . | . | . | . | . | . | . | . | . | . | . | . |
| 21-225_76C9_LC | . | . | . | . | . | S | . | . | . | . | . | . | . | . | . | . | . | . | . |
| 21-225_76D1_LC | . | . | . | . | . | . | . | . | . | . | . | . | . | . | . | . | . | . | . |
| 21-225_76E10_LC | . | . | . | . | . | . | . | . | . | . | . | . | . | . | . | . | . | . | . |
| 21-225_76G4_LC | . | . | . | . | . | . | . | . | . | . | . | . | . | . | . | . | . | . | . |
| 21-225_77A10_LC | . | . | . | . | . | . | . | . | . | . | . | . | . | . | . | . | . | . | . |
| 21-225_77C12_LC | . | . | . | . | . | . | . | . | . | . | . | . | . | . | . | . | . | . | . |
| 21-225_77C5_LC | . | . | . | . | . | . | . | . | . | . | . | . | . | . | . | . | . | . | . |
| 21-225_77F7_LC | . | . | . | . | . | . | . | . | . | . | . | . | . | . | . | . | . | . | . |
| 21-225_77G1_LC | . | . | . | . | . | . | . | . | . | . | . | . | . | . | . | . | . | . | . |
| 21-225_78E7_LC | . | . | . | . | . | . | . | . | . | . | . | . | . | . | . | . | . | . | . |
| 21-225_79F11_LC | . | . | . | . | . | . | . | . | . | . | . | . | . | . | . | . | . | . | . |
| 21-225_79F3_LC | . | . | . | . | . | . | . | . | . | . | . | . | . | . | . | . | . | . | . |
| 21-225_79G1_LC | . | . | . | . | . | . | . | . | . | . | . | . | . | . | . | . | . | . | . |
| 21-225_79G12_LC | . | . | . | . | . | . | . | . | . | . | . | . | . | . | . | . | . | . | . |
| 21-225_80A1_LC | . | . | . | . | . | . | . | . | . | . | . | . | . | . | . | . | . | . | . |
| 21-225_80A6_LC | . | . | . | . | . | . | . | . | . | . | . | . | . | . | . | . | . | . | . |
| 21-225_80B10_LC | . | . | . | . | . | . | . | . | . | . | . | . | . | . | . | . | . | . | . |
| 21-225_80C1_LC | . | . | . | . | . | . | . | . | . | . | . | . | . | . | . | . | . | . | . |
| 21-225_80D5_LC | . | . | . | . | . | . | . | . | . | . | . | . | . | . | . | . | . | . | . |
| 21-225_80E12_LC | . | . | . | . | . | . | . | . | . | . | . | . | . | . | . | . | . | . | . |
| 21-225_80E3_LC | . | . | . | . | . | . | . | . | . | . | . | . | . | . | . | . | . | . | . |
| 21-225_80H12_LC | . | . | . | . | . | . | . | . | . | . | . | . | . | . | . | . | . | . | . |
| 21-225_80H7_LC | . | . | . | . | . | . | . | . | . | . | . | . | . | . | . | . | . | . | . |
| 21-225_81C11_LC | . | . | . | . | . | . | . | . | . | . | . | . | . | . | . | . | . | . | . |
| 21-225_81C5_LC | . | . | . | . | . | . | . | . | . | . | . | . | . | . | . | . | . | . | . |
| 21-225_83B6_LC | . | . | . | . | . | . | . | . | . | . | . | . | . | . | . | . | . | . | . |
| 21-225_83B7_LC | . | . | . | . | . | . | . | . | . | . | . | . | . | . | . | . | . | . | . |
| 21-225_83C10_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | S | P | W | T | F | G | Q | G | T | K | V | E | I | K | R |
| 21-225_83G1_LC | . | . | . | . | . | . | . | . | . | . | . | . | . | . | . | . | . | . | . |
| 21-225_84E12_LC | . | . | . | . | . | . | . | . | . | . | . | . | . | . | . | . | . | . | . |
| 21-225_85A3_LC | . | . | . | . | . | . | . | . | . | . | . | . | . | . | . | . | . | . | . |
| 21-225_85B4_LC | . | . | . | . | . | . | . | . | . | . | . | . | . | . | . | . | . | . | . |
| 21-225_85B9_LC | . | . | . | . | . | . | . | . | . | . | . | . | . | . | . | . | . | . | . |
| 21-225_85D6_LC | . | . | . | . | . | . | . | . | . | . | . | . | . | . | . | . | . | . | . |
| 21-225_85G10_LC | . | . | . | . | . | . | . | . | . | . | . | . | . | . | . | . | . | . | . |
| 21-225_85G6_LC | . | . | . | . | . | . | . | . | . | . | . | . | . | . | . | . | . | . | . |
| 21-225_86C1_LC | . | . | . | . | . | . | . | . | . | . | . | . | . | . | . | . | . | . | . |
| 21-225_86C11_LC | . | . | . | . | . | . | . | . | . | . | . | . | . | . | . | . | . | . | . |
| 21-225_86E4_LC | . | . | . | . | . | . | . | . | . | . | . | . | . | . | . | . | . | . | . |
| 21-225_87A12_LC | . | . | . | . | . | . | . | . | . | . | . | . | . | . | . | . | . | . | . |
| 21-225_87C9_LC | . | . | . | . | . | . | . | . | . | . | . | . | . | . | . | . | . | . | . |
| 21-225_87E5_LC | . | . | . | . | . | . | . | . | . | . | . | . | . | . | . | . | . | . | . |
| 21-225_87H1_LC | . | . | . | . | . | . | . | . | . | . | . | . | . | . | . | . | . | . | . |
| 21-225_88E7_LC | . | . | . | . | . | . | . | . | . | . | . | . | . | . | . | . | . | . | . |
| 21-225_88F7_LC | . | . | . | . | . | . | . | . | . | . | . | . | . | . | . | . | . | . | . |
| 21-225_88G9_LC | . | . | . | . | . | . | . | . | . | . | . | . | . | . | . | . | . | . | . |
| 21-225_88H1_LC | . | . | . | . | . | . | . | . | . | . | . | . | . | . | . | . | . | . | . |
| 21-225_89A11_LC | . | . | . | . | . | . | . | . | . | . | . | . | . | . | . | . | . | . | . |
| 21-225_89D5_LC | . | . | . | . | . | . | . | . | . | . | . | . | . | . | . | . | . | . | . |
| 21-225_89E10_LC | . | . | . | . | . | . | . | . | . | . | . | . | . | . | . | . | . | . | . |
| 21-225_89H5_LC | . | . | . | . | . | V | . | . | . | . | . | . | . | . | . | . | . | . | . |
| 21-225_90A5_LC | . | . | . | . | . | . | . | . | . | . | . | . | . | . | . | . | . | . | . |
| 21-225_90F10_LC | . | . | . | . | . | . | . | . | . | . | . | . | . | . | . | . | . | . | . |
| 21-225_90G4_LC | . | . | . | . | . | . | . | . | . | . | . | . | . | . | . | . | . | . | . |
| 21-225_90G5_LC | . | . | . | . | . | . | . | . | . | . | . | . | . | . | . | . | . | . | . |
| 21-225_91B2_LC | . | . | . | . | . | . | . | . | . | . | . | . | . | . | . | . | . | . | . |
| 21-225_91B3_LC | . | . | . | . | . | . | . | . | . | . | . | . | . | . | . | . | . | . | . |

# FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | S | P | W | T | F | G | Q | G | T | K | V | E | I | K | R |
| 21-225_91B4_LC | . | . | . | . | . | . | . | . | . | . | . | . | . | . | . | . | . | . | . |
| 21-225_91E9_LC | . | . | . | . | . | . | . | . | . | . | . | . | . | . | . | . | . | . | . |
| 21-225_91F3_LC | . | . | . | . | . | . | . | . | . | . | . | . | . | . | . | . | . | . | . |
| 21-225_92B1_LC | . | . | . | . | . | . | . | . | . | . | . | . | . | . | . | . | . | . | . |
| 21-225_92D6_LC | . | . | . | . | . | . | . | . | . | . | . | . | . | . | . | . | . | . | . |
| 21-225_93C2_LC | . | . | . | . | . | . | . | . | . | . | . | . | . | . | . | . | . | . | . |
| 21-225_93E4_LC | . | . | . | . | . | . | . | . | . | . | . | . | . | . | . | . | . | . | . |
| 21-225_93E9_LC | . | . | . | . | . | . | . | . | . | . | . | . | . | . | . | . | . | . | . |
| 21-225_94D3_LC | . | . | . | . | . | . | . | . | . | . | . | . | . | . | . | . | N | Q | N |
| 21-225_94F12_LC | . | . | . | . | . | . | . | . | . | . | . | . | . | . | . | . | . | . | . |
| 21-225_94G10_LC | . | . | . | . | . | . | . | . | . | . | . | . | . | . | . | . | . | . | . |
| 21-225_95D2_LC | . | . | . | . | . | . | . | . | . | . | . | . | . | . | . | . | . | . | . |
| 21-225_95F9_LC | . | . | . | . | . | . | . | . | . | . | . | . | . | . | . | . | . | . | . |
| 21-225_95G9_LC | . | . | . | . | . | . | . | . | . | . | . | . | . | . | . | . | . | . | . |
| 21-225_95H10_LC | . | . | . | . | . | . | . | . | . | . | . | . | . | . | . | . | . | . | . |
| 21-225_97A2_LC | . | . | . | . | . | . | . | . | . | . | . | . | . | . | . | . | . | . | . |
| 21-225_97E5_LC | . | . | . | . | . | . | . | . | . | . | . | . | . | . | . | . | . | . | . |

**FIGURE 57**

Table 98. Consensus 18 - VK1|A30/JK1 (SEQ ID NO: 50315):

DIQMTQSPSSLSASVGDRVTITCRAS--QGIR------NDLGWYQQKPGKAPKRLIYA--------ASSLQSGVPSRFSGSGSG--TEFTLTISSLQPEDFATYYCLQHYS----------------------YPRTFGQGTKVEIKR wherein:

A at position 25 can be substituted with T

S at position 26 can be substituted with T

Q at position 29 can be substituted with R

G at position 30 can be substituted with D, A, N or V

R at position 32 can be substituted with G

N at position 39 can be substituted with K, D, H, S, G or T

D at position 40 can be substituted with I or Y

G at position 42 can be substituted with N

A at position 58 can be substituted with T, I, V, P, G, R or S

A at position 67 can be substituted with T or S

S at position 68 can be substituted with A, F, P or Y

S at position 69 can be substituted with N, R, G or T

L at position 70 can be substituted with C, F or S

Q at position 71 can be substituted with H or E

S at position 72 can be substituted with N, G or I

L at position 107 can be substituted with V, I or H

Q at position 108 can be substituted with M, H, L or V

**FIGURE 57**

H at position 109 can be substituted with Q, Y, L or S

Y at position 110 can be substituted with N, H, S or T

S at position 111 can be substituted with N, T, R, D, F or G

null (-) at position 134 can be substituted with Y or F

Y at position 135 can be substituted with F, P, C, N or T

P at position 136 can be substituted with L

R at position 137 can be substituted with W, F or L

Arg Xaa Xaa Xaa Xaa Ile Xaa Xaa Xaa Leu Xaa (SEQ ID NO: 50130)

Xaa Xaa Xaa Xaa Xaa Xaa Xaa (SEQ ID NO: 50131)

Xaa Xaa Xaa Xaa Xaa Xaa Xaa Xaa Xaa Thr (SEQ ID NO: 50132)

Arg Ala Ser Gln Gly Ile Arg Asn Asp Leu Gly (SEQ ID NO: 50615)

Ala Ala Ser Ser Leu Gln Ser (SEQ ID NO: 50616)

Leu Gln His Tyr Ser Tyr Pro Arg Thr (SEQ ID NO: 50617)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_10E9_LC | . | . | . | . | . | . | . | . | . | . | . | . | . | . | . | . | . | . | . | . | F | . | . | . | . | . |
| 21-225_12B12_LC | . | . | . | . | . | . | . | . | . | . | . | . | . | . | . | . | . | . | . | . | . | . | . | . | . | . |
| 21-225_12E6_LC | . | . | . | . | . | . | . | . | . | . | . | . | . | . | . | . | . | . | . | . | . | . | . | . | . | . |
| 21-225_12F2_LC | . | . | . | . | . | . | . | . | . | . | . | . | . | . | . | . | . | . | . | . | . | . | . | . | . | . |
| 21-225_147H5_LC | . | . | . | . | . | . | . | . | . | . | . | . | . | . | . | . | . | . | . | . | . | . | . | . | . | . |
| 21-225_148C9_LC | . | . | . | . | . | . | . | . | . | . | . | . | . | . | . | . | . | . | . | . | . | . | . | . | . | . |
| 21-225_149D10_LC | . | . | . | . | . | . | . | . | . | . | . | . | . | . | . | . | . | . | . | . | . | . | . | . | . | . |
| 21-225_14E10_LC | . | . | . | . | . | . | . | . | . | . | . | . | . | . | . | . | . | . | . | . | . | . | . | . | T | . |
| 21-225_158D4_LC | . | . | . | . | . | . | . | . | . | . | . | . | . | . | . | . | . | . | . | . | . | . | . | . | . | . |
| 21-225_158G1_LC | . | . | . | . | . | . | . | . | . | . | . | . | . | . | . | . | . | . | . | . | . | . | . | . | . | . |
| 21-225_159A3_LC | . | . | . | . | . | . | . | . | . | . | . | . | . | . | . | . | . | . | . | . | . | . | . | . | . | . |
| 21-225_159C8_LC | . | . | . | . | . | . | . | . | . | . | . | . | . | . | . | . | . | . | . | . | . | . | . | . | . | . |
| 21-225_159D8_LC | . | . | . | . | . | . | . | . | . | . | . | . | . | . | . | . | . | . | . | . | . | . | . | . | . | . |
| 21-225_15C11_LC | . | . | . | . | . | . | . | . | . | . | . | . | . | . | . | . | . | . | . | . | . | . | . | . | . | . |
| 21-225_160C4_LC | . | . | . | . | . | . | . | . | . | . | . | . | . | . | . | . | . | . | . | . | . | . | . | . | . | . |
| 21-225_160F2_LC | . | . | . | . | . | . | . | . | . | . | . | . | . | . | . | . | . | . | . | . | . | . | . | . | . | . |
| 21-225_160H1_LC | . | . | . | . | . | . | . | . | . | . | . | . | . | . | . | . | . | . | . | . | . | . | . | . | . | . |
| 21-225_161G12_LC | . | . | . | . | . | . | . | . | . | . | R | . | . | . | . | . | . | . | . | . | . | . | . | . | . | . |
| 21-225_169D11_LC | . | . | . | . | . | . | . | . | . | . | . | . | . | . | . | . | . | . | . | . | . | . | . | . | . | . |
| 21-225_169D7_LC | . | . | . | . | . | . | . | . | . | . | . | . | . | . | . | . | . | . | . | . | . | . | . | . | . | . |
| 21-225_16A5_LC | . | . | . | . | . | . | . | . | . | . | . | . | . | . | . | . | . | . | . | . | . | . | . | . | . | . |
| 21-225_16G7_LC | . | . | . | . | . | . | . | . | . | . | . | . | . | . | . | . | . | . | . | . | . | . | . | . | . | . |
| 21-225_16H4_LC | . | . | . | . | . | . | . | . | . | . | . | . | . | . | . | . | . | . | . | . | . | . | . | . | . | . |
| 21-225_170E4_LC | . | . | . | . | . | . | . | . | . | . | . | . | . | . | . | . | . | . | . | . | . | . | . | . | . | . |
| 21-225_170F3_LC | . | . | . | . | . | . | . | . | . | . | . | . | . | . | . | Q | . | . | . | . | . | . | . | . | . | . |
| 21-225_170H1_LC | . | . | . | . | . | . | . | . | . | . | . | . | . | . | . | . | . | . | . | . | . | . | . | . | . | . |
| 21-225_174G10_LC | . | . | . | . | . | . | . | . | . | . | . | . | . | . | . | . | . | . | . | . | . | . | . | . | . | . |
| 21-225_17C7_LC | . | . | . | . | . | . | . | . | . | . | . | . | . | . | . | . | . | . | . | . | . | . | . | . | . | . |
| 21-225_17H3_LC | . | . | . | . | . | . | . | . | . | . | . | . | . | . | . | . | . | . | . | . | F | . | . | . | . | . |

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_18A5_LC | . | . | . | . | . | . | . | . | . | . | . | . | . | . | . | . | . | . | . | . | . | . | . | . | . | . |
| 21-225_18C2_LC | . | . | . | . | . | . | . | . | . | . | . | . | . | . | . | . | . | . | . | . | . | . | . | . | . | . |
| 21-225_18F2_LC | . | . | . | . | . | . | . | . | . | . | . | . | . | . | . | . | . | . | . | . | . | . | . | . | . | . |
| 21-225_18H12_LC | . | . | . | . | . | . | . | . | . | . | . | . | . | . | . | . | . | . | . | . | . | . | . | . | . | . |
| 21-225_190C12_LC | . | . | . | . | . | . | . | . | . | . | . | . | . | . | . | . | . | . | . | . | . | . | . | . | . | . |
| 21-225_190G3_LC | . | . | . | . | . | . | . | . | . | . | . | . | . | . | . | R | . | . | . | . | . | . | . | . | . | . |
| 21-225_190H5_LC | . | . | . | . | . | . | . | . | . | . | . | . | . | . | . | . | . | . | . | . | . | . | . | . | . | . |
| 21-225_191A4_LC | . | . | . | . | . | . | . | . | . | . | . | . | . | . | . | . | . | . | . | . | . | . | . | . | . | . |
| 21-225_192F7_LC | . | . | . | . | . | . | . | . | . | . | . | . | . | . | . | . | . | . | . | . | . | . | . | . | . | . |
| 21-225_196C12_LC | . | . | . | . | . | L | . | . | . | . | . | F | . | C | . | . | . | . | . | I | . | . | . | . | . | . |
| 21-225_19A10_LC | . | . | . | . | . | . | . | . | . | . | . | . | . | . | . | . | . | . | . | . | . | . | . | . | . | . |
| 21-225_200C9_LC | . | . | . | . | . | . | . | . | . | . | . | . | . | . | . | . | . | . | . | . | . | . | . | . | . | T |
| 21-225_201C5_LC | . | . | . | . | . | . | . | . | . | . | . | . | . | . | . | . | . | . | . | . | . | . | . | . | . | . |
| 21-225_203C10_LC | . | . | . | . | . | . | . | . | . | . | . | . | . | . | . | . | . | . | . | . | . | . | . | . | . | . |
| 21-225_203F7_LC | . | . | . | . | . | . | . | . | . | . | R | F | . | . | . | . | . | . | . | . | . | . | . | . | . | . |
| 21-225_204H3_LC | . | . | . | . | . | . | . | . | . | . | . | . | . | . | . | . | . | . | . | . | . | . | . | . | . | . |
| 21-225_208B2_LC | . | . | . | . | . | . | . | . | . | . | . | . | . | . | . | . | . | . | . | . | . | . | . | . | . | . |
| 21-225_208F1_LC | . | . | . | . | . | . | . | . | . | . | . | . | . | . | . | . | . | . | . | . | . | . | . | . | . | . |
| 21-225_20B11_LC | . | . | . | . | . | . | . | . | . | . | . | . | . | . | . | . | . | . | . | . | . | . | . | . | . | . |
| 21-225_20B2_LC | . | . | . | . | . | . | . | . | . | . | . | . | . | . | . | . | . | . | . | N | . | . | . | . | . | . |
| 21-225_20D10_LC | . | . | . | . | . | . | . | . | . | . | . | . | . | F | . | . | . | . | . | . | . | . | . | . | . | . |
| 21-225_210G5_LC | . | . | . | . | . | . | . | . | . | . | . | . | . | . | . | . | . | . | . | . | . | . | . | . | . | . |
| 21-225_210G6_LC | . | . | . | . | . | . | . | . | . | . | R | F | . | F | . | . | . | . | . | . | . | . | . | . | . | . |
| 21-225_212B11_LC | . | . | . | . | . | . | . | . | . | . | . | P | . | . | . | . | . | . | . | . | . | . | . | . | . | . |
| 21-225_214G10_LC | . | . | . | . | . | L | C | . | . | . | P | P | . | . | . | . | . | . | . | . | . | . | . | . | . | . |
| 21-225_214G12_LC | . | . | . | . | . | . | . | . | . | . | P | P | . | . | . | . | . | . | . | . | . | . | . | . | . | . |
| 21-225_214H9_LC | . | . | . | . | . | . | . | . | . | . | . | . | . | . | . | . | . | . | . | . | . | . | . | . | . | . |
| 21-225_215E11_LC | . | . | . | . | . | . | C | . | . | . | P | P | . | . | . | . | . | . | . | . | . | . | . | . | . | . |
| 21-225_215E3_LC | . | . | . | . | . | . | . | . | . | . | P | P | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_216E8_LC | . | . | . | . | . | . | . | . | . | . | . | P | . | . | . | . | . | . | . | . | . | . | . | . | . | . |
| 21-225_216H12_LC | . | . | . | . | . | L | . | . | . | . | . | P | . | . | . | . | . | . | . | . | . | . | . | . | . | . |
| 21-225_217E5_LC | . | . | . | . | . | . | C | . | . | . | P | P | . | P | . | . | . | . | . | . | . | . | . | . | . | . |
| 21-225_217G10_LC | . | . | . | . | . | . | . | . | . | . | P | P | . | . | . | . | . | . | . | . | . | . | . | . | . | . |
| 21-225_217H12_LC | . | . | . | . | . | . | . | . | . | . | P | P | . | . | . | . | . | . | . | . | . | . | . | . | . | . |
| 21-225_218C4_LC | . | . | . | . | . | . | C | . | . | . | P | P | . | P | . | . | . | . | . | . | . | . | . | . | . | . |
| 21-225_21F7_LC | . | . | . | . | . | . | . | . | . | . | . | . | . | . | . | . | . | . | . | . | . | . | . | . | . | . |
| 21-225_21G11_LC | . | . | . | . | . | . | . | . | . | . | . | . | . | . | . | . | . | . | . | . | . | . | . | . | . | . |
| 21-225_21G2_LC | . | . | . | . | . | . | . | . | . | . | . | . | . | . | . | . | . | . | . | . | . | . | . | . | . | . |
| 21-225_21H8_LC | . | . | . | . | . | . | . | . | . | . | . | . | . | . | . | . | . | . | . | . | . | . | . | . | . | . |
| 21-225_220F8_LC | . | . | . | . | . | L | . | . | . | . | P | P | . | P | . | . | . | . | . | . | . | . | R | . | . | . |
| 21-225_224E7_LC | . | . | . | . | . | . | . | . | . | . | . | . | . | . | . | . | . | . | . | . | . | . | . | . | . | . |
| 21-225_224F1_LC | A | . | . | . | . | . | . | . | . | . | . | . | . | . | . | . | . | . | . | . | . | . | . | . | . | . |
| 21-225_224F3_LC | . | . | . | . | . | . | . | . | . | . | . | . | . | . | . | . | . | . | . | . | . | . | . | . | . | . |
| 21-225_225A1_LC | . | . | . | . | . | . | . | . | . | . | . | . | . | . | . | . | . | . | . | . | . | . | . | . | . | . |
| 21-225_226C6_LC | . | . | . | . | . | . | . | . | . | . | . | . | . | . | I | . | . | . | . | . | . | . | . | . | . | . |
| 21-225_226F7_LC | . | . | . | . | . | . | . | . | . | . | . | . | . | . | . | . | . | . | . | . | . | . | . | . | . | . |
| 21-225_226H11_LC | . | . | . | . | . | . | . | . | . | . | . | . | . | . | . | . | . | . | . | . | . | . | . | . | T | . |
| 21-225_22A4_LC | . | . | . | . | . | . | . | . | . | . | . | . | . | . | . | . | . | . | . | . | . | . | . | . | . | . |
| 21-225_22F4_LC | . | . | . | . | . | . | . | . | . | . | . | . | . | . | . | . | . | . | . | . | . | . | . | . | . | . |
| 21-225_23A10_LC | . | . | . | . | . | . | . | . | . | . | . | . | . | . | . | . | . | . | . | . | . | . | . | . | . | . |
| 21-225_23E11_LC | . | . | . | . | . | . | . | . | . | . | . | . | . | . | . | . | . | . | I | . | . | . | . | . | . | . |
| 21-225_23F12_LC | . | . | . | . | . | . | . | . | . | . | . | . | . | . | . | . | . | . | . | . | . | . | . | . | . | . |
| 21-225_24B6_LC | . | . | . | . | . | . | . | . | . | . | . | . | . | . | . | . | . | . | . | . | . | . | . | . | . | . |
| 21-225_25D12_LC | . | . | . | . | . | . | . | . | . | . | . | . | . | . | . | . | . | . | . | . | . | . | . | . | T | . |
| 21-225_26A2_LC | . | . | . | . | . | . | . | . | . | . | . | . | . | . | . | . | . | . | . | . | . | . | . | . | . | . |
| 21-225_28C7_LC | . | . | . | . | . | . | . | . | . | . | . | . | . | . | . | . | . | . | . | . | . | . | . | . | . | . |
| 21-225_28F5_LC | . | . | . | . | . | . | . | . | . | . | . | . | . | . | . | . | . | . | . | . | . | . | . | . | . | . |
| 21-225_2G4_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_32B6_LC | . | . | . | . | . | . | . | . | . | . | . | . | . | . | . | . | . | . | . | . | . | . | . | . | . | . |
| 21-225_33C2_LC | . | . | . | . | . | . | . | . | . | . | . | . | . | . | . | . | . | . | . | . | . | . | . | . | . | . |
| 21-225_44B3_LC | . | . | . | . | . | . | . | . | . | . | . | . | . | . | . | . | . | . | . | . | . | . | . | . | . | . |
| 21-225_44C10_LC | . | . | . | . | . | . | . | . | . | . | . | . | . | . | . | . | . | . | . | . | F | . | . | . | . | . |
| 21-225_44D3_LC | . | . | . | . | . | . | . | . | . | . | . | . | . | . | . | . | . | . | . | . | F | . | . | . | . | . |
| 21-225_44D5_LC | . | . | . | . | . | . | . | . | . | . | . | . | . | . | . | . | . | . | . | . | . | . | . | . | . | . |
| 21-225_44F9_LC | . | V | . | . | . | . | . | . | . | . | . | . | . | . | . | . | . | . | . | . | . | . | . | . | . | . |
| 21-225_45B4_LC | . | . | . | . | . | . | . | . | . | . | . | . | . | . | . | . | . | . | . | . | F | . | . | . | . | . |
| 21-225_45B8_LC | . | . | . | . | . | . | . | . | . | . | . | . | . | . | . | . | . | . | . | . | F | . | . | . | . | . |
| 21-225_46C3_LC | . | . | . | . | . | . | . | . | . | . | . | . | . | . | . | . | . | . | . | . | F | . | . | . | . | . |
| 21-225_46D4_LC | . | . | . | . | . | . | . | . | . | . | . | . | . | . | . | . | . | . | . | . | . | . | . | . | T | . |
| 21-225_48A9_LC | . | . | . | . | . | . | . | . | . | . | . | . | . | . | . | . | . | . | . | . | . | . | . | . | . | . |
| 21-225_48C1_LC | . | . | . | . | . | . | . | . | . | . | . | . | . | . | . | . | . | . | . | . | . | . | . | . | T | . |
| 21-225_48F2_LC | . | . | . | . | . | . | . | . | . | . | . | . | . | . | . | . | . | . | . | . | . | . | . | . | . | . |
| 21-225_4C12_LC | . | . | . | . | . | . | . | . | . | . | . | . | . | . | . | . | . | . | . | . | . | . | . | . | . | . |
| 21-225_4C5_LC | . | . | . | . | . | . | . | . | . | . | . | . | . | . | . | . | H | . | . | H | L | . | . | . | . | . |
| 21-225_50A12_LC | . | . | . | . | . | . | . | . | . | . | . | . | . | . | . | . | . | . | . | . | . | . | . | . | . | . |
| 21-225_50H4_LC | . | . | . | . | . | . | . | . | . | . | . | . | . | . | . | . | . | . | . | . | . | . | . | . | . | . |
| 21-225_51H8_LC | . | . | . | . | . | . | . | . | . | . | . | . | . | . | . | . | . | . | . | . | . | . | . | . | . | . |
| 21-225_54H3_LC | . | . | . | . | . | . | . | . | . | . | . | . | . | . | . | . | . | . | . | . | . | . | . | . | . | . |
| 21-225_56C7_LC | . | . | . | . | . | . | . | . | . | . | . | . | . | . | . | . | . | . | . | . | . | . | . | . | . | . |
| 21-225_57B8_LC | . | . | . | . | . | . | . | . | . | . | . | . | . | . | . | . | . | . | I | . | . | . | . | . | . | . |
| 21-225_59B9_LC | . | . | . | . | . | . | . | . | . | . | . | . | . | . | . | . | . | . | . | . | . | . | . | . | . | . |
| 21-225_59G7_LC | . | . | . | . | . | . | . | . | . | . | . | . | . | . | . | . | . | . | . | . | . | . | . | . | . | . |
| 21-225_5E5_LC | . | . | . | . | . | . | . | . | . | . | . | . | . | . | . | . | . | . | . | . | . | . | . | . | . | . |
| 21-225_5E5.003_LC | . | . | . | . | . | . | . | . | . | . | . | . | . | . | . | . | . | . | . | . | . | . | . | . | . | . |
| 21-225_5E5.004_LC | . | . | . | . | . | . | . | . | . | . | . | . | . | . | . | . | . | . | . | . | . | . | . | . | . | . |
| 21-225_5E5.005_LC | . | . | . | . | . | . | . | . | . | . | . | . | . | . | . | . | . | . | . | . | . | . | . | . | . | . |
| 21-225_5E5.006_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_5E5.011_LC | . | . | . | . | . | . | . | . | . | . | . | . | . | . | . | . | . | . | . | . | . | . | . | . | . | . |
| 21-225_5E5.012_LC | . | . | . | . | . | . | . | . | . | . | . | . | . | . | . | . | . | . | . | . | . | . | . | . | . | . |
| 21-225_5E5.015_LC | . | . | . | . | . | . | . | . | . | . | . | . | . | . | . | . | . | . | . | . | . | . | . | . | . | . |
| 21-225_5E5.016_LC | . | . | . | . | . | . | . | . | . | . | . | . | . | . | . | . | . | . | . | . | . | . | . | . | . | . |
| 21-225_5E5.017_LC | . | . | . | . | . | . | . | . | . | . | . | . | . | . | . | . | . | . | . | . | . | . | . | . | . | . |
| 21-225_5E5.018_LC | . | . | . | . | . | . | . | . | . | . | . | . | . | . | . | . | . | . | . | . | . | . | . | . | . | . |
| 21-225_5E5.019_LC | . | . | . | . | . | . | . | . | . | . | . | . | . | . | . | . | . | . | . | . | . | . | . | . | . | . |
| 21-225_5E5.023_LC | . | . | . | . | . | . | . | . | . | . | . | . | . | . | . | . | . | . | . | . | . | . | . | . | . | . |
| 21-225_5E5.024_LC | . | . | . | . | . | . | . | . | . | . | . | . | . | . | . | . | . | . | . | . | . | . | . | . | . | . |
| 21-225_5E5.025_LC | . | . | . | . | . | . | . | . | . | . | . | . | . | . | . | . | . | . | . | . | . | . | . | . | . | . |
| 21-225_60C3_LC | . | . | . | . | . | . | . | . | . | . | . | . | . | . | . | . | . | . | . | . | . | . | . | . | . | . |
| 21-225_60E2_LC | . | . | . | . | . | . | . | . | . | . | . | . | . | . | . | . | . | . | . | . | . | . | . | . | . | . |
| 21-225_61E6_LC | . | . | . | . | . | . | . | . | . | . | . | . | . | . | . | . | . | . | I | . | . | . | . | . | . | . |
| 21-225_61H1_LC | . | . | . | . | . | . | . | . | . | . | . | . | . | . | . | . | . | . | . | . | . | . | . | . | . | . |
| 21-225_62F7_LC | . | . | . | . | . | . | . | . | . | . | . | . | . | . | . | . | . | . | . | . | . | . | . | . | . | . |
| 21-225_64A4_LC | . | . | . | . | . | . | . | . | . | . | . | . | . | . | . | . | . | . | . | . | . | . | . | . | . | . |
| 21-225_64C8_LC | . | . | . | . | . | . | . | . | . | . | . | . | . | A | . | . | . | . | . | . | . | . | . | . | . | . |
| 21-225_66C10_LC | . | . | . | . | . | . | . | . | . | . | . | . | . | . | . | . | . | . | . | . | . | . | . | . | . | . |
| 21-225_66D11_LC | . | . | . | . | . | . | F | . | . | . | Q | . | . | . | . | . | . | . | . | . | . | . | . | . | . | . |
| 21-225_6A11_LC | . | . | . | . | . | . | . | . | . | . | . | . | . | . | . | . | . | . | . | . | . | . | . | . | . | . |
| 21-225_6G2_LC | . | . | . | . | . | . | . | . | . | . | R | . | . | F | . | . | . | . | . | . | . | . | Y | . | . | . |
| 21-225_71A2_LC | . | . | . | . | . | . | . | . | . | . | R | . | . | . | . | . | . | . | . | . | . | . | . | . | . | . |
| 21-225_73C9_LC | . | . | . | . | . | . | . | . | . | . | . | . | . | . | . | . | . | . | . | . | . | . | . | . | . | . |
| 21-225_7C9_LC | . | . | . | . | . | . | . | . | . | . | . | . | . | . | G | . | . | . | . | . | . | . | . | . | . | . |
| 21-225_7F8_LC | . | . | . | . | . | . | . | . | . | . | . | . | . | . | . | . | . | . | . | . | . | . | . | . | . | . |
| 21-225_7G2_LC | . | . | . | . | . | . | . | . | . | . | . | . | . | . | . | . | . | . | . | . | . | . | . | . | . | . |
| 21-225_7G4_LC | . | . | . | . | . | . | . | . | . | . | . | . | . | . | . | . | . | . | . | . | . | . | . | . | . | . |
| 21-225_94F3_LC | . | . | . | . | . | . | . | . | . | . | . | . | . | . | . | . | . | . | . | . | . | . | . | . | . | . |
| 21-225_9A1_LC | . | . | . | . | . | . | . | . | . | . | R | . | . | F | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_9C11_LC | . | . | . | . | . | . | . | . | . | . | . | . | . | . | . | . | . | . | . | . | . | . | . | . | . | . |
| 21-225_9D12_LC | . | . | . | . | . | . | . | . | . | . | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_10E9_LC | . | . | . | D | . | . | . | . | . | . | . | . | S | . | . | . | . | . | . | . | . | . | . | . | . | . |
| 21-225_12B12_LC | . | . | . | . | . | . | . | . | . | . | . | . | . | . | . | . | . | . | . | . | . | . | . | . | . | . |
| 21-225_12E6_LC | . | . | . | . | . | . | . | . | . | . | . | . | S | . | . | . | . | . | . | . | . | . | . | . | . | . |
| 21-225_12F2_LC | . | . | . | . | . | . | . | . | . | . | . | . | . | . | . | . | . | . | . | . | . | . | . | . | . | . |
| 21-225_147H5_LC | . | . | R | . | . | . | . | . | . | . | . | . | D | . | . | . | . | . | . | . | . | . | . | . | . | . |
| 21-225_148C9_LC | . | . | R | . | . | . | . | . | . | . | . | . | D | . | . | . | . | . | . | . | . | . | . | . | . | . |
| 21-225_149D10_LC | . | . | R | . | . | . | . | . | . | . | . | . | . | . | . | . | . | . | . | . | . | . | . | . | . | . |
| 21-225_14E10_LC | . | . | . | . | . | . | . | . | . | . | . | . | . | . | . | . | . | . | . | H | . | . | . | Q | . | . |
| 21-225_158D4_LC | . | . | . | D | . | . | . | . | . | . | . | . | K | . | . | . | . | . | . | . | . | . | . | . | . | . |
| 21-225_158G1_LC | . | . | . | . | . | . | . | . | . | . | . | . | . | . | . | . | . | . | . | . | . | . | . | . | . | . |
| 21-225_159A3_LC | . | . | . | D | . | . | . | . | . | . | . | . | K | . | . | . | . | . | . | . | . | . | . | . | . | . |
| 21-225_159C8_LC | . | . | . | D | . | . | . | . | . | . | . | . | K | . | . | . | . | . | . | . | . | . | . | . | . | . |
| 21-225_159D8_LC | . | . | R | D | . | G | . | . | . | . | . | . | . | . | . | . | . | . | . | . | . | . | . | . | . | . |
| 21-225_15C11_LC | . | . | . | . | . | . | . | . | . | . | . | . | . | . | . | . | . | . | . | . | . | . | . | . | V | . |
| 21-225_160C4_LC | . | . | . | D | . | . | . | . | . | . | . | . | K | . | . | . | . | . | . | . | . | . | . | . | . | . |
| 21-225_160F2_LC | . | . | . | . | . | . | . | . | . | . | . | . | . | . | . | . | . | . | . | . | . | . | . | . | . | . |
| 21-225_160H1_LC | . | . | . | D | . | . | . | . | . | . | . | . | . | . | . | . | . | . | . | . | . | . | . | . | . | . |
| 21-225_161G12_LC | . | . | . | D | . | . | . | . | . | . | . | . | K | . | . | . | . | . | . | . | . | . | . | . | . | . |
| 21-225_169D11_LC | . | . | . | . | . | . | . | . | . | . | . | . | D | . | . | . | . | . | . | . | . | . | . | . | V | . |
| 21-225_169D7_LC | . | . | . | . | . | . | . | . | . | . | . | . | . | . | . | . | . | . | . | . | . | . | . | . | . | . |
| 21-225_16A5_LC | . | . | . | . | . | . | . | . | . | . | . | . | . | . | . | . | C | . | . | . | . | . | . | . | . | . |
| 21-225_16G7_LC | . | . | . | . | . | . | . | . | . | . | . | . | . | . | . | . | . | . | . | . | . | . | . | . | . | . |
| 21-225_16H4_LC | . | . | . | . | . | . | . | . | . | . | . | . | . | . | . | . | . | . | . | . | . | . | . | . | . | . |
| 21-225_170E4_LC | . | . | . | . | . | . | . | . | . | . | . | . | . | . | . | . | Y | . | . | . | . | . | . | . | . | . |
| 21-225_170F3_LC | . | . | R | . | . | . | . | . | . | . | . | . | . | . | . | . | . | . | . | . | . | . | . | . | . | . |
| 21-225_170H1_LC | . | . | . | . | . | . | . | . | . | . | . | . | . | . | . | . | . | . | . | . | . | . | . | . | . | . |
| 21-225_174G10_LC | . | . | . | . | . | . | . | . | . | . | . | . | D | . | . | . | . | . | . | . | . | . | . | . | V | . |
| 21-225_17C7_LC | . | . | . | . | . | . | . | . | . | . | . | . | . | . | . | . | . | . | . | . | . | . | . | . | . | . |
| 21-225_17H3_LC | . | . | . | . | . | . | . | . | . | . | . | . | S | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_18A5_LC | . | . | . | . | . | . | . | . | . | . | . | . | . | . | . | . | . | . | . | . | . | . | . | . | . | . |
| 21-225_18C2_LC | . | . | . | . | . | . | . | . | . | . | . | . | . | . | . | . | . | . | . | . | . | . | . | . | . | . |
| 21-225_18F2_LC | . | . | . | . | . | . | . | . | . | . | . | . | . | . | . | . | . | . | . | . | . | . | . | . | . | . |
| 21-225_18H12_LC | . | . | . | . | . | . | . | . | . | . | . | . | . | . | . | . | . | . | . | . | . | . | . | . | . | . |
| 21-225_190C12_LC | . | . | . | . | . | . | . | . | . | . | . | . | K | . | . | . | . | . | . | . | . | . | . | . | . | . |
| 21-225_190G3_LC | . | . | . | . | . | . | . | . | . | . | . | . | K | . | . | . | . | . | . | . | . | . | . | . | . | . |
| 21-225_190H5_LC | . | . | . | . | . | . | . | . | . | . | . | . | K | . | . | . | . | . | . | . | . | . | . | . | . | . |
| 21-225_191A4_LC | . | . | . | . | . | . | . | . | . | . | . | . | K | . | . | . | . | . | . | . | . | . | . | . | . | . |
| 21-225_192F7_LC | . | . | . | . | . | . | . | . | . | . | . | . | K | . | . | . | . | . | . | . | . | . | . | . | . | . |
| 21-225_196C12_LC | . | . | . | . | . | . | . | . | . | . | . | . | . | . | . | . | . | S | . | . | N | . | . | . | . | . |
| 21-225_19A10_LC | . | . | . | . | . | . | . | . | . | . | . | . | T | . | . | . | . | . | . | . | R | . | . | . | . | . |
| 21-225_200C9_LC | . | . | . | . | . | . | . | . | . | . | . | . | K | . | . | . | . | . | . | . | . | . | . | . | . | . |
| 21-225_201C5_LC | . | . | . | . | . | . | . | . | . | . | . | . | H | . | . | . | . | . | . | . | . | . | . | . | . | . |
| 21-225_203C10_LC | . | . | . | D | . | . | . | . | . | . | . | . | . | . | . | . | . | . | . | . | . | . | A | . | . | . |
| 21-225_203F7_LC | . | . | . | . | . | . | . | . | . | . | . | . | H | . | . | . | . | . | . | . | . | . | . | . | . | L |
| 21-225_204H3_LC | . | . | . | . | . | . | . | . | . | . | . | . | . | . | . | . | . | . | . | . | . | . | . | . | . | . |
| 21-225_208B2_LC | . | . | . | . | . | . | . | . | . | . | . | . | H | . | . | . | . | . | . | . | . | . | . | . | . | . |
| 21-225_208F1_LC | . | . | . | . | . | . | . | . | . | . | . | . | H | . | . | . | . | . | . | . | . | . | . | . | . | . |
| 21-225_20B11_LC | . | . | . | . | . | G | . | . | . | . | . | . | . | . | . | . | . | . | . | . | . | . | E | . | . | . |
| 21-225_20B2_LC | . | . | . | D | . | . | . | . | . | . | . | . | . | . | . | . | . | . | . | . | . | . | . | . | . | . |
| 21-225_20D10_LC | . | . | . | . | . | . | . | . | . | . | . | . | . | . | . | . | . | . | . | . | . | . | . | . | . | R |
| 21-225_210G5_LC | . | . | . | . | . | . | . | . | . | . | . | . | H | . | . | . | . | . | . | . | . | . | . | . | . | . |
| 21-225_210G6_LC | . | . | . | . | . | . | . | . | . | . | . | . | H | . | . | . | . | . | . | . | . | . | . | . | . | L |
| 21-225_212B11_LC | . | . | . | . | . | . | . | . | . | . | . | . | . | . | . | . | . | . | . | . | . | . | . | . | . | . |
| 21-225_214G10_LC | . | . | . | . | . | . | . | . | . | . | . | . | . | . | . | . | . | . | . | . | . | . | . | . | . | . |
| 21-225_214G12_LC | . | . | . | . | . | . | . | . | . | . | . | . | . | . | . | . | . | . | . | . | . | . | . | . | . | . |
| 21-225_214H9_LC | . | . | . | . | . | . | . | . | . | . | . | . | . | . | . | . | . | . | . | . | . | . | . | . | . | . |
| 21-225_215E11_LC | . | . | . | . | . | . | . | . | . | . | . | . | . | . | . | . | . | . | . | . | . | . | . | . | . | . |
| 21-225_215E3_LC | . | . | . | . | . | . | . | . | . | . | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_216E8_LC | . | . | . | . | . | . | . | . | . | . | . | . | . | . | . | . | . | . | . | . | . | . | . | . | . | . |
| 21-225_216H12_LC | . | . | . | . | . | . | . | . | . | . | . | . | . | . | . | . | . | . | . | . | . | . | . | . | . | . |
| 21-225_217E5_LC | . | . | . | . | . | . | . | . | . | . | . | . | . | . | . | . | . | . | . | . | . | . | . | . | . | . |
| 21-225_217G10_LC | . | . | . | . | . | . | . | . | . | . | . | . | . | . | . | . | . | . | . | . | . | . | . | . | . | . |
| 21-225_217H12_LC | . | . | . | . | . | . | . | . | . | . | . | . | . | . | . | . | . | . | . | . | . | . | . | . | . | . |
| 21-225_218C4_LC | . | . | . | . | . | . | . | . | . | . | . | . | . | . | . | . | . | . | . | . | . | . | . | . | . | . |
| 21-225_21F7_LC | . | . | . | D | . | . | . | . | . | . | . | . | . | . | . | . | . | . | . | . | . | . | . | . | . | . |
| 21-225_21G11_LC | . | . | . | . | . | . | . | . | . | . | . | . | . | . | . | . | . | . | . | . | . | . | . | . | . | . |
| 21-225_21G2_LC | . | . | . | . | . | . | . | . | . | . | . | . | . | . | . | . | . | . | . | . | . | . | . | . | . | . |
| 21-225_21H8_LC | . | . | . | . | . | . | . | . | . | . | . | . | . | . | . | . | . | . | . | . | . | . | . | . | . | . |
| 21-225_220F8_LC | . | . | . | . | . | . | . | . | . | . | . | . | . | . | . | . | . | . | . | . | . | . | . | . | . | . |
| 21-225_224E7_LC | . | . | . | . | . | . | . | . | . | . | . | . | . | . | . | . | . | . | . | . | . | . | . | . | . | . |
| 21-225_224F1_LC | . | . | . | . | . | . | . | . | . | . | . | . | D | . | . | . | . | . | . | . | . | . | . | . | . | . |
| 21-225_224F3_LC | . | . | . | . | . | . | . | . | . | . | . | . | . | . | . | . | . | . | . | . | . | . | . | . | . | . |
| 21-225_225A1_LC | . | . | . | . | . | . | . | . | . | . | . | . | D | . | . | . | . | . | . | . | I | . | . | . | . | . |
| 21-225_226C6_LC | . | . | . | . | . | . | . | . | . | . | . | . | . | . | . | . | . | . | . | . | . | . | . | . | . | . |
| 21-225_226F7_LC | . | . | . | . | . | G | . | . | . | . | . | . | . | . | . | . | . | . | . | . | . | . | . | . | . | . |
| 21-225_226H11_LC | . | . | . | . | . | . | . | . | . | . | . | . | . | . | . | . | . | . | . | . | . | . | . | . | . | . |
| 21-225_22A4_LC | . | . | . | . | . | . | . | . | . | . | . | . | . | . | . | . | . | . | . | . | . | . | . | . | . | . |
| 21-225_22F4_LC | . | . | . | V | . | . | . | . | . | . | . | . | D | . | . | . | . | . | . | . | . | . | . | . | . | . |
| 21-225_23A10_LC | . | . | . | . | . | . | . | . | . | . | . | . | . | . | . | . | . | . | . | . | . | . | . | . | . | . |
| 21-225_23E11_LC | . | . | . | . | . | . | . | . | . | . | . | . | . | . | . | . | . | . | . | . | . | . | . | . | . | . |
| 21-225_23F12_LC | . | . | . | . | . | . | . | . | . | . | . | . | . | . | . | . | . | . | . | . | . | . | . | . | . | . |
| 21-225_24B6_LC | . | . | . | D | . | . | . | . | . | . | . | . | . | . | . | . | . | . | . | . | . | . | . | . | . | . |
| 21-225_25D12_LC | . | . | . | . | . | . | . | . | . | . | . | . | . | . | . | . | . | . | . | . | . | . | . | . | . | . |
| 21-225_26A2_LC | . | . | . | . | . | . | . | . | . | . | . | . | S | . | . | . | . | . | . | . | . | . | . | . | . | . |
| 21-225_28C7_LC | . | . | . | . | . | . | . | . | . | . | . | . | . | . | . | . | . | . | . | . | . | . | . | . | . | . |
| 21-225_28F5_LC | . | . | . | . | . | . | . | . | . | . | . | . | . | . | . | . | . | . | . | . | . | . | . | . | . | . |
| 21-225_2G4_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_32B6_LC | . | . | . | . | . | . | . | . | . | . | . | . | . | . | . | . | . | . | . | . | . | . | . | . | . | . |
| 21-225_33C2_LC | . | . | . | . | . | . | . | . | . | . | . | . | . | . | . | . | . | . | . | . | . | . | V | . | . | . |
| 21-225_44B3_LC | . | . | . | . | . | . | . | . | . | . | . | . | . | . | . | . | . | . | . | . | . | . | . | . | . | . |
| 21-225_44C10_LC | . | . | . | . | . | . | . | . | . | . | . | . | D | . | . | . | . | . | . | . | . | . | . | . | . | . |
| 21-225_44D3_LC | . | . | . | . | . | . | . | . | . | . | . | . | D | . | . | . | . | . | . | . | . | . | . | . | . | . |
| 21-225_44D5_LC | . | . | . | D | . | . | . | . | . | . | . | . | K | . | . | . | . | . | . | . | . | . | . | . | . | . |
| 21-225_44F9_LC | . | . | . | . | . | . | . | . | . | . | . | . | . | . | . | . | . | . | . | . | . | . | . | . | . | . |
| 21-225_45B4_LC | . | . | . | . | . | . | . | . | . | . | . | . | D | . | . | . | . | . | . | . | . | . | . | . | . | . |
| 21-225_45B8_LC | . | . | . | D | . | . | . | . | . | . | . | . | D | . | . | . | . | . | . | . | . | . | . | . | . | . |
| 21-225_46C3_LC | . | . | . | . | . | . | . | . | . | . | . | . | D | . | . | . | . | . | . | . | . | . | . | . | . | . |
| 21-225_46D4_LC | . | . | . | D | . | . | . | . | . | . | . | . | K | . | . | . | . | . | . | . | . | V | . | . | . | . |
| 21-225_48A9_LC | . | . | . | . | . | . | . | . | . | . | . | . | . | . | . | . | . | . | . | . | . | . | . | . | . | . |
| 21-225_48C1_LC | . | . | . | . | . | . | . | . | . | . | . | . | . | . | . | . | . | . | . | . | . | . | . | . | . | . |
| 21-225_48F2_LC | . | . | R | . | . | . | . | . | . | . | . | . | D | . | . | . | . | . | . | . | . | . | . | . | P | . |
| 21-225_4C12_LC | . | . | . | . | . | . | . | . | . | . | . | . | . | . | . | . | . | . | . | . | . | . | . | . | . | . |
| 21-225_4C5_LC | . | . | R | . | . | . | . | . | . | . | . | . | G | . | . | . | . | . | R | . | . | . | . | . | . | . |
| 21-225_50A12_LC | . | . | . | . | . | . | . | . | . | . | . | . | . | . | . | . | . | . | . | . | . | . | . | . | . | . |
| 21-225_50H4_LC | . | . | . | . | . | . | . | . | . | . | . | . | . | . | . | . | . | . | . | . | . | . | . | . | . | . |
| 21-225_51H8_LC | . | . | . | . | . | . | . | . | . | . | . | . | . | . | . | . | . | . | . | . | . | . | . | . | . | . |
| 21-225_54H3_LC | . | . | . | D | . | . | . | . | . | . | . | . | . | I | . | . | . | . | . | . | . | . | . | . | . | . |
| 21-225_56C7_LC | . | . | . | . | . | . | . | . | . | . | . | . | . | . | . | . | . | . | . | . | . | . | . | . | . | . |
| 21-225_57B8_LC | . | . | . | . | . | G | . | . | . | . | . | . | . | . | . | . | . | . | . | . | . | . | . | . | . | . |
| 21-225_59B9_LC | . | . | . | D | . | . | . | . | . | . | . | . | . | . | . | . | . | . | . | . | H | . | . | . | . | . |
| 21-225_59G7_LC | . | . | . | . | . | . | . | . | . | . | . | . | . | . | . | . | . | . | . | . | . | . | . | . | . | . |
| 21-225_5E5_LC | . | . | . | . | . | . | . | . | . | . | . | . | . | . | . | . | . | . | . | . | . | . | . | . | . | . |
| 21-225_5E5.003_LC | . | . | . | . | . | . | . | . | . | . | . | . | . | . | . | . | . | . | . | . | . | . | . | . | . | . |
| 21-225_5E5.004_LC | . | . | . | . | . | . | . | . | . | . | . | . | . | . | . | . | . | . | . | . | . | . | . | . | . | . |
| 21-225_5E5.005_LC | . | . | . | . | . | . | . | . | . | . | . | . | . | . | . | . | . | . | . | . | . | . | . | . | . | . |
| 21-225_5E5.006_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_5E5.011_LC | . | . | . | . | . | . | . | . | . | . | . | . | . | . | . | . | . | . | . | . | . | . | . | . | . | . |
| 21-225_5E5.012_LC | . | . | . | . | . | . | . | . | . | . | . | . | . | . | . | . | . | . | . | . | . | . | . | . | . | . |
| 21-225_5E5.015_LC | . | . | . | . | . | . | . | . | . | . | . | . | . | . | . | . | . | . | . | . | . | . | . | . | . | . |
| 21-225_5E5.016_LC | . | . | . | . | . | . | . | . | . | . | . | . | . | . | . | . | . | . | . | . | . | . | . | . | . | . |
| 21-225_5E5.017_LC | . | . | . | . | . | . | . | . | . | . | . | . | . | . | . | . | . | . | . | . | . | . | . | . | . | . |
| 21-225_5E5.018_LC | . | . | . | . | . | . | . | . | . | . | . | . | . | . | . | . | . | . | . | . | . | . | . | . | . | . |
| 21-225_5E5.019_LC | . | . | . | . | . | . | . | . | . | . | . | . | . | . | . | . | . | . | . | . | . | . | . | . | . | . |
| 21-225_5E5.023_LC | . | . | . | . | . | . | . | . | . | . | . | . | . | . | . | . | . | . | . | . | . | . | . | . | . | . |
| 21-225_5E5.024_LC | . | . | . | . | . | . | . | . | . | . | . | . | . | . | . | . | . | . | . | . | . | . | . | . | . | . |
| 21-225_5E5.025_LC | . | . | . | . | . | . | . | . | . | . | . | . | . | . | . | . | . | . | . | . | . | . | . | . | . | . |
| 21-225_60C3_LC | . | . | . | . | . | . | . | . | . | . | . | . | . | . | . | . | . | . | . | . | . | . | . | . | . | . |
| 21-225_60E2_LC | . | . | . | . | . | . | . | . | . | . | . | . | K | . | . | . | . | . | . | . | . | . | . | . | . | . |
| 21-225_61E6_LC | . | . | . | . | . | G | . | . | . | . | . | . | . | . | . | . | . | . | . | . | . | . | . | . | . | . |
| 21-225_61H1_LC | . | . | . | . | . | . | . | . | . | . | . | . | K | . | . | . | . | . | . | . | . | . | . | . | . | . |
| 21-225_62F7_LC | . | . | . | A | . | . | . | . | . | . | . | . | . | . | . | . | . | . | . | . | . | . | . | . | . | . |
| 21-225_64A4_LC | . | . | . | . | . | . | . | . | . | . | . | . | . | . | . | . | . | . | . | . | . | . | . | . | . | . |
| 21-225_64C8_LC | . | . | . | . | . | . | . | . | . | . | . | . | . | . | . | . | . | . | . | . | . | . | . | . | . | . |
| 21-225_66C10_LC | . | . | . | A | . | . | . | . | . | . | . | . | . | . | . | . | . | . | . | . | . | . | . | . | . | . |
| 21-225_66D11_LC | . | . | . | . | . | . | . | . | . | . | . | . | . | . | . | . | . | . | . | . | . | . | . | . | . | H |
| 21-225_6A11_LC | . | . | . | . | . | . | . | . | . | . | . | . | . | . | . | . | . | . | . | . | . | . | . | . | . | . |
| 21-225_6G2_LC | . | . | . | . | . | . | . | . | . | . | . | . | . | . | . | . | . | . | . | . | . | . | . | . | . | . |
| 21-225_71A2_LC | . | . | . | . | . | . | . | . | . | . | . | . | . | . | . | . | . | . | . | . | . | . | . | . | . | . |
| 21-225_73C9_LC | . | . | . | . | . | . | . | . | . | . | . | . | . | . | . | . | . | . | . | . | . | . | . | . | . | . |
| 21-225_7C9_LC | . | . | . | . | . | . | . | . | . | . | . | . | . | . | . | . | . | . | . | . | . | . | . | . | . | . |
| 21-225_7F8_LC | . | . | . | . | . | G | . | . | . | . | . | . | . | . | . | . | . | . | . | . | . | . | . | . | . | . |
| 21-225_7G2_LC | . | . | . | . | . | . | . | . | . | . | . | . | . | . | . | . | . | . | . | . | . | . | . | . | . | . |
| 21-225_7G4_LC | . | . | . | . | . | . | . | . | . | . | . | . | . | . | . | . | . | . | . | . | R | . | . | . | . | . |
| 21-225_94F3_LC | . | . | . | A | . | . | . | . | . | . | . | . | D | . | . | . | . | . | . | . | . | . | . | . | . | . |
| 21-225_9A1_LC | . | . | . | . | . | . | . | . | . | . | . | . | . | . | . | . | . | . | . | . | . | . | . | . | . | R |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_9C11_LC | . | . | . | N | . | . | . | . | . | . | . | . | . | Y | . | N | . | . | . | . | . | S | . | R | . | . |
| 21-225_9D12_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | R | L | I | Y | A | - | - | - | - | - | - | - | - | A | S | S | L | Q | S | G | V | P | S | R | F |
| 21-225_10E9_LC | T | . | . | . | F | . | . | . | . | . | . | . | . | . | . | . | . | . | . | . | . | . | . | . | . | . |
| 21-225_12B12_LC | . | . | . | . | . | T | . | . | . | . | . | . | . | . | . | . | . | . | . | . | . | . | . | . | . | . |
| 21-225_12E6_LC | . | . | . | . | . | . | . | . | . | . | . | . | . | . | . | . | . | . | . | . | . | . | . | . | . | . |
| 21-225_12F2_LC | . | . | . | . | . | . | . | . | . | . | . | . | . | . | . | . | . | . | . | . | . | . | . | . | . | . |
| 21-225_147H5_LC | . | . | . | . | . | . | . | . | . | . | . | . | . | . | . | . | . | . | . | . | . | . | . | . | . | . |
| 21-225_148C9_LC | . | . | . | . | . | . | . | . | . | . | . | . | . | . | . | . | . | . | . | . | . | . | . | . | . | . |
| 21-225_149D10_LC | . | . | . | . | . | . | . | . | . | . | . | . | . | . | . | . | . | . | . | . | . | . | . | . | . | . |
| 21-225_14E10_LC | . | . | . | . | . | . | . | . | . | . | . | . | . | . | . | . | . | . | . | . | . | . | . | . | . | . |
| 21-225_158D4_LC | . | . | . | . | . | . | . | . | . | . | . | . | . | . | . | . | . | . | . | . | . | . | . | . | . | . |
| 21-225_158G1_LC | . | . | . | . | . | T | . | . | . | . | . | . | . | . | . | . | N | . | . | . | . | . | . | . | . | . |
| 21-225_159A3_LC | N | . | . | . | . | . | . | . | . | . | . | . | . | . | . | . | . | . | . | . | . | . | . | . | . | . |
| 21-225_159C8_LC | N | . | . | . | . | . | . | . | . | . | . | . | . | . | . | . | . | . | . | . | . | . | . | . | . | . |
| 21-225_159D8_LC | . | . | . | . | S | . | . | . | . | . | . | . | . | . | . | . | . | . | . | . | . | . | . | . | . | . |
| 21-225_15C11_LC | . | . | . | . | . | . | . | . | . | . | . | . | . | . | . | . | . | . | . | . | . | . | . | . | . | . |
| 21-225_160C4_LC | N | . | . | . | . | . | . | . | . | . | . | . | . | . | . | . | . | . | . | . | . | . | . | . | . | . |
| 21-225_160F2_LC | . | . | . | . | . | T | . | . | . | . | . | . | . | . | . | . | N | . | . | . | . | . | . | . | . | . |
| 21-225_160H1_LC | . | . | . | . | . | . | . | . | . | . | . | . | . | . | . | . | . | . | . | N | . | . | . | . | . | . |
| 21-225_161G12_LC | N | . | . | . | . | . | . | . | . | . | . | . | . | . | . | . | . | . | . | . | . | . | . | . | . | . |
| 21-225_169D11_LC | . | . | . | . | . | . | . | . | . | . | . | . | . | . | . | . | . | . | . | . | . | . | . | . | . | . |
| 21-225_169D7_LC | . | . | . | . | . | . | . | . | . | . | . | . | . | . | . | . | . | . | . | . | . | . | . | . | . | . |
| 21-225_16A5_LC | . | . | . | . | . | . | . | . | . | . | . | . | . | . | . | . | . | . | . | . | . | . | . | . | . | . |
| 21-225_16G7_LC | . | . | . | . | . | . | . | . | . | . | . | . | . | . | . | . | . | . | . | . | . | . | . | . | . | . |
| 21-225_16H4_LC | . | . | . | . | . | T | . | . | . | . | . | . | . | . | . | . | . | . | . | . | . | . | . | . | . | . |
| 21-225_170E4_LC | . | . | . | . | . | V | . | . | . | . | . | . | . | . | . | . | G | . | . | . | . | . | . | . | . | . |
| 21-225_170F3_LC | . | . | . | . | . | . | . | . | . | . | . | . | . | . | . | . | . | . | . | . | . | . | . | . | . | . |
| 21-225_170H1_LC | . | . | . | . | . | . | . | . | . | . | . | . | . | . | T | . | . | . | . | . | . | . | . | . | . | . |
| 21-225_174G10_LC | . | . | . | . | . | . | . | . | . | . | . | . | . | . | . | . | . | . | . | . | . | . | . | . | . | . |
| 21-225_17C7_LC | . | . | . | . | . | . | . | . | . | . | . | . | . | . | . | . | . | . | . | . | . | . | . | . | . | . |
| 21-225_17H3_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | R | L | I | Y | A | - | - | - | - | - | - | - | - | A | S | S | L | Q | S | G | V | P | S | R | F |
| 21-225_18A5_LC | . | . | . | . | . | . | . | . | . | . | . | . | . | . | . | . | . | . | . | . | . | . | . | . | . | . |
| 21-225_18C2_LC | . | . | . | . | . | T | . | . | . | . | . | . | . | . | . | . | . | . | . | . | . | . | . | . | . | . |
| 21-225_18F2_LC | . | . | . | . | . | . | . | . | . | . | . | . | . | . | . | . | . | . | . | . | . | . | . | . | . | . |
| 21-225_18H12_LC | . | . | . | . | . | T | . | . | . | . | . | . | . | . | . | . | N | . | . | . | . | . | . | . | . | . |
| 21-225_190C12_LC | . | . | . | . | H | T | . | . | . | . | . | . | . | . | . | . | . | . | . | . | . | . | . | L | . | . |
| 21-225_190G3_LC | . | . | . | . | H | T | . | . | . | . | . | . | . | . | . | . | . | . | . | . | . | . | . | L | . | . |
| 21-225_190H5_LC | . | . | . | . | H | T | . | . | . | . | . | . | . | . | . | . | . | . | . | . | . | . | . | L | . | . |
| 21-225_191A4_LC | . | . | . | . | H | T | . | . | . | . | . | . | . | . | . | . | . | . | . | N | . | . | . | L | . | . |
| 21-225_192F7_LC | . | . | . | . | H | T | . | . | . | . | . | . | . | . | . | . | . | . | . | . | . | . | . | L | . | . |
| 21-225_196C12_LC | . | . | . | . | . | . | . | . | . | . | . | . | . | . | . | . | . | . | . | . | . | . | . | . | . | . |
| 21-225_19A10_LC | . | . | . | . | . | T | . | . | . | . | . | . | . | . | . | . | . | . | . | . | . | . | . | . | . | . |
| 21-225_200C9_LC | . | . | . | . | H | T | . | . | . | . | . | . | . | . | . | . | . | . | . | . | . | . | . | L | . | . |
| 21-225_201C5_LC | . | . | . | . | . | . | . | . | . | . | . | . | . | . | . | . | . | . | . | . | . | . | . | . | . | . |
| 21-225_203C10_LC | . | . | . | . | . | R | . | . | . | . | . | . | . | . | . | . | . | . | . | . | . | . | . | . | . | . |
| 21-225_203F7_LC | . | . | . | . | . | . | . | . | . | . | . | . | . | . | . | . | . | . | . | . | . | . | . | . | . | . |
| 21-225_204H3_LC | E | L | . | . | . | . | . | . | . | . | . | . | . | . | . | A | . | . | . | G | . | . | . | . | . | . |
| 21-225_208B2_LC | . | . | . | . | . | . | . | . | . | . | . | . | . | . | . | . | . | . | . | . | . | . | . | . | G | . |
| 21-225_208F1_LC | . | . | . | . | F | . | . | . | . | . | . | . | . | . | . | . | . | . | . | . | . | . | . | . | . | . |
| 21-225_20B11_LC | . | . | . | . | . | . | . | . | . | . | . | . | . | . | . | . | . | . | H | . | . | . | . | . | . | . |
| 21-225_20B2_LC | . | . | . | . | . | . | . | . | . | . | . | . | . | . | . | . | . | . | . | . | . | . | . | . | . | . |
| 21-225_20D10_LC | . | . | . | . | . | V | . | . | . | . | . | . | . | . | . | Y | . | . | . | . | . | . | . | . | . | . |
| 21-225_210G5_LC | . | . | . | . | . | . | . | . | . | . | . | . | . | . | . | . | . | . | . | . | . | . | . | . | G | . |
| 21-225_210G6_LC | . | . | . | . | . | . | . | . | . | . | . | . | . | . | . | . | . | S | . | . | . | . | . | . | . | . |
| 21-225_212B11_LC | . | . | . | . | . | . | . | . | . | . | . | . | . | . | . | . | . | . | . | . | . | . | . | . | . | . |
| 21-225_214G10_LC | . | . | . | . | . | . | . | . | . | . | . | . | . | . | . | . | . | . | . | . | . | . | . | . | . | . |
| 21-225_214G12_LC | . | . | . | . | . | . | . | . | . | . | . | . | . | . | . | . | . | . | . | . | . | . | . | . | . | . |
| 21-225_214H9_LC | . | . | . | . | . | . | . | . | . | . | . | . | . | . | . | . | . | . | . | . | . | . | . | . | . | . |
| 21-225_215E11_LC | . | . | . | . | . | . | . | . | . | . | . | . | . | . | . | . | . | . | . | . | . | . | . | . | . | . |
| 21-225_215E3_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | R | L | I | Y | A | - | - | - | - | - | - | - | - | A | S | S | L | Q | S | G | V | P | S | R | F |
| 21-225_216E8_LC | . | . | . | . | . | . | . | . | . | . | . | . | . | . | . | . | . | . | . | . | . | . | . | . | . | . |
| 21-225_216H12_LC | . | . | . | . | . | G | . | . | . | . | . | . | . | . | . | . | . | . | . | . | . | . | . | . | . | . |
| 21-225_217E5_LC | . | . | . | . | C | . | . | . | . | . | . | . | . | . | . | . | . | . | . | . | . | . | . | . | . | . |
| 21-225_217G10_LC | . | . | . | . | . | . | . | . | . | . | . | . | . | . | . | . | . | . | . | . | . | . | . | . | . | . |
| 21-225_217H12_LC | . | . | . | . | . | . | . | . | . | . | . | . | . | . | . | . | . | . | . | . | . | . | . | . | . | . |
| 21-225_218C4_LC | . | . | . | . | . | . | . | . | . | . | . | . | . | . | . | . | . | . | . | . | . | . | . | . | . | . |
| 21-225_21F7_LC | . | . | . | . | . | . | . | . | . | . | . | . | . | . | . | . | N | F | . | . | . | . | . | . | . | . |
| 21-225_21G11_LC | . | . | . | . | . | T | . | . | . | . | . | . | . | . | . | . | . | . | . | . | . | . | . | . | . | . |
| 21-225_21G2_LC | . | . | . | . | . | T | . | . | . | . | . | . | . | . | S | . | . | . | . | . | . | . | . | . | . | . |
| 21-225_21H8_LC | . | . | . | . | . | . | . | . | . | . | . | . | . | . | . | . | . | . | . | . | . | . | . | . | . | . |
| 21-225_220F8_LC | . | . | . | . | C | G | . | . | . | . | . | . | . | . | . | . | . | . | . | . | . | . | . | . | . | . |
| 21-225_224E7_LC | T | . | . | . | . | . | . | . | . | . | . | . | . | . | . | . | . | . | . | . | . | . | . | . | . | . |
| 21-225_224F1_LC | . | . | . | . | . | . | . | . | . | . | . | . | . | . | . | . | . | . | . | . | . | . | . | . | . | I |
| 21-225_224F3_LC | E | . | . | . | . | . | . | . | . | . | . | . | . | . | . | . | . | . | . | . | . | . | . | . | . | . |
| 21-225_225A1_LC | . | . | . | . | . | . | . | . | . | . | . | . | . | . | . | . | . | . | . | . | . | . | . | . | . | . |
| 21-225_226C6_LC | . | . | . | . | . | . | . | . | . | . | . | . | . | . | . | . | . | . | . | . | . | L | . | . | . | . |
| 21-225_226F7_LC | . | . | . | . | . | . | . | . | . | . | . | . | . | . | . | . | . | . | . | . | . | . | . | . | . | . |
| 21-225_226H11_LC | . | . | . | . | . | . | . | . | . | . | . | . | . | . | . | . | . | . | . | . | . | . | . | . | . | . |
| 21-225_22A4_LC | . | . | . | . | . | . | . | . | . | . | . | . | . | . | . | . | . | . | . | . | . | . | . | . | . | . |
| 21-225_22F4_LC | . | . | . | . | . | . | . | . | . | . | . | . | . | . | . | . | . | . | . | . | . | . | . | . | . | . |
| 21-225_23A10_LC | . | . | . | . | . | . | . | . | . | . | . | . | . | . | . | . | . | . | . | . | . | . | . | . | . | . |
| 21-225_23E11_LC | . | . | . | . | . | P | . | . | . | . | . | . | . | . | . | . | . | . | . | . | . | . | . | . | . | . |
| 21-225_23F12_LC | . | . | . | . | . | V | . | . | . | . | . | . | . | . | . | . | . | . | . | . | . | . | . | . | . | . |
| 21-225_24B6_LC | . | . | . | . | . | . | . | . | . | . | . | . | . | . | . | . | . | . | H | . | . | . | . | . | . | . |
| 21-225_25D12_LC | . | . | . | . | . | . | . | . | . | . | . | . | . | . | . | . | . | . | . | . | . | . | . | . | . | . |
| 21-225_26A2_LC | . | . | . | . | . | T | . | . | . | . | . | . | . | . | . | . | . | . | . | . | . | . | . | . | . | . |
| 21-225_28C7_LC | . | . | . | . | . | I | . | . | . | . | . | . | . | . | . | . | . | . | . | . | . | . | . | . | . | . |
| 21-225_28F5_LC | . | . | . | . | . | T | . | . | . | . | . | . | . | . | . | . | . | . | . | . | . | . | . | . | . | . |
| 21-225_2G4_LC | T | . | . | . | . | . | . | . | . | . | . | . | . | . | . | . | . | . | H | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | R | L | I | Y | A | - | - | - | - | - | - | - | - | A | S | S | L | Q | S | G | V | P | S | R | F |
| 21-225_32B6_LC | . | . | . | . | . | I | . | . | . | . | . | . | . | . | . | . | . | . | . | . | . | . | . | . | . | . |
| 21-225_33C2_LC | . | . | . | . | . | . | . | . | . | . | . | . | . | . | . | P | . | . | . | . | . | . | . | . | . | . |
| 21-225_44B3_LC | . | . | . | . | . | . | . | . | . | . | . | . | . | . | . | . | . | . | . | . | . | . | . | . | . | . |
| 21-225_44C10_LC | . | . | . | . | . | . | . | . | . | . | . | . | . | . | T | . | . | . | . | . | . | . | . | . | . | . |
| 21-225_44D3_LC | . | . | . | . | . | . | . | . | . | . | . | . | . | . | . | . | . | . | . | . | . | . | . | . | . | . |
| 21-225_44D5_LC | . | . | . | . | . | . | . | . | . | . | . | . | . | . | . | . | T | . | E | . | . | . | . | . | . | . |
| 21-225_44F9_LC | . | . | . | . | . | . | . | . | . | . | . | . | . | . | . | . | . | . | . | . | . | . | . | . | . | . |
| 21-225_45B4_LC | . | . | . | . | . | . | . | . | . | . | . | . | . | . | . | . | . | . | . | . | . | . | . | . | . | . |
| 21-225_45B8_LC | . | . | . | . | . | . | . | . | . | . | . | . | . | . | . | . | . | . | . | . | . | . | . | . | . | . |
| 21-225_46C3_LC | . | . | . | . | . | . | . | . | . | . | . | . | . | . | . | . | . | . | . | . | . | . | . | . | . | . |
| 21-225_46D4_LC | . | . | . | . | . | . | . | . | . | . | . | . | . | . | . | . | . | . | E | . | . | . | . | . | . | . |
| 21-225_48A9_LC | . | . | . | . | . | I | . | . | . | . | . | . | . | . | . | . | . | . | . | . | . | . | . | . | . | . |
| 21-225_48C1_LC | . | . | . | . | . | R | . | . | . | . | . | . | . | . | . | . | . | . | . | . | . | . | . | A | . | . |
| 21-225_48F2_LC | . | . | . | . | . | . | . | . | . | . | . | . | . | . | . | . | . | . | . | . | . | . | . | . | . | . |
| 21-225_4C12_LC | T | . | . | . | . | . | . | . | . | . | . | . | . | . | . | . | . | . | H | . | . | . | . | . | . | . |
| 21-225_4C5_LC | . | . | . | . | . | . | . | . | . | . | . | . | . | . | . | . | N | . | . | . | . | . | . | . | . | . |
| 21-225_50A12_LC | . | . | . | . | . | T | . | . | . | . | . | . | . | . | . | . | N | . | . | . | . | . | . | . | . | . |
| 21-225_50H4_LC | . | . | . | . | . | . | . | . | . | . | . | . | . | . | . | . | . | . | . | . | . | . | . | . | . | . |
| 21-225_51H8_LC | . | . | . | . | . | . | . | . | . | . | . | . | . | . | . | . | . | . | . | . | . | . | . | . | . | . |
| 21-225_54H3_LC | . | . | . | . | . | . | . | . | . | . | . | . | . | . | . | . | N | . | . | . | . | . | . | . | . | . |
| 21-225_56C7_LC | . | . | . | . | . | T | . | . | . | . | . | . | . | . | . | . | N | . | . | . | . | . | . | . | . | . |
| 21-225_57B8_LC | E | . | . | . | . | . | . | . | . | . | . | . | . | . | . | . | . | . | . | . | . | . | . | . | . | . |
| 21-225_59B9_LC | . | . | . | . | . | . | . | . | . | . | . | . | . | . | . | . | . | . | . | . | . | . | . | . | . | . |
| 21-225_59G7_LC | . | . | . | . | . | S | . | . | . | . | . | . | . | . | . | . | . | . | . | . | . | . | . | . | . | . |
| 21-225_5E5_LC | . | . | . | . | . | . | . | . | . | . | . | . | . | . | . | . | . | . | . | . | . | . | . | . | . | . |
| 21-225_5E5.003_LC | . | . | . | . | . | . | . | . | . | . | . | . | . | . | . | . | . | . | . | . | . | . | . | . | . | . |
| 21-225_5E5.004_LC | . | . | . | . | . | . | . | . | . | . | . | . | . | . | . | . | . | . | . | . | . | . | . | . | . | . |
| 21-225_5E5.005_LC | . | . | . | . | . | . | . | . | . | . | . | . | . | . | . | . | . | . | . | . | . | . | . | . | . | . |
| 21-225_5E5.006_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | R | L | I | Y | A | - | - | - | - | - | - | - | - | A | S | S | L | Q | S | G | V | P | S | R | F |
| 21-225_5E5.011_LC | . | . | . | . | . | . | . | . | . | . | . | . | . | . | . | . | . | . | . | . | . | . | . | . | . | . |
| 21-225_5E5.012_LC | . | . | . | . | . | . | . | . | . | . | . | . | . | . | . | . | . | . | . | . | . | . | . | . | . | . |
| 21-225_5E5.015_LC | . | . | . | . | . | . | . | . | . | . | . | . | . | . | . | . | . | . | . | . | . | . | . | . | . | . |
| 21-225_5E5.016_LC | . | . | . | . | . | . | . | . | . | . | . | . | . | . | . | . | . | . | . | . | . | . | . | . | . | . |
| 21-225_5E5.017_LC | . | . | . | . | . | . | . | . | . | . | . | . | . | . | . | . | . | . | . | . | . | . | . | . | . | . |
| 21-225_5E5.018_LC | . | . | . | . | . | . | . | . | . | . | . | . | . | . | . | . | . | . | . | . | . | . | . | . | . | . |
| 21-225_5E5.019_LC | . | . | . | . | . | . | . | . | . | . | . | . | . | . | . | . | . | . | . | . | . | . | . | . | . | . |
| 21-225_5E5.023_LC | . | . | . | . | . | . | . | . | . | . | . | . | . | . | . | . | . | . | . | . | . | . | . | . | . | . |
| 21-225_5E5.024_LC | . | . | . | . | . | . | . | . | . | . | . | . | . | . | . | . | . | . | . | . | . | . | . | . | . | . |
| 21-225_5E5.025_LC | . | . | . | . | . | . | . | . | . | . | . | . | . | . | . | . | . | . | . | . | . | . | . | . | . | . |
| 21-225_60C3_LC | . | . | . | . | . | S | . | . | . | . | . | . | . | . | . | . | . | . | . | . | . | . | . | . | . | . |
| 21-225_60E2_LC | . | . | . | . | . | . | . | . | . | . | . | . | . | . | . | . | . | . | . | . | . | . | . | . | . | . |
| 21-225_61E6_LC | E | . | . | . | . | . | . | . | . | . | . | . | . | . | . | . | . | . | . | . | . | . | . | . | . | . |
| 21-225_61H1_LC | . | . | . | . | F | . | . | . | . | . | . | . | . | . | . | . | . | . | . | . | . | . | . | . | . | . |
| 21-225_62F7_LC | . | . | . | . | . | P | . | . | . | . | . | . | . | . | . | . | R | . | . | . | . | . | . | . | . | . |
| 21-225_64A4_LC | . | . | . | . | . | . | . | . | . | . | . | . | . | . | . | . | . | . | . | . | . | . | . | . | . | . |
| 21-225_64C8_LC | . | C | . | . | . | . | . | . | . | . | . | . | . | . | . | . | . | . | . | . | . | . | . | . | . | . |
| 21-225_66C10_LC | . | . | . | . | . | P | . | . | . | . | . | . | . | . | . | . | . | . | . | . | . | . | . | . | . | . |
| 21-225_66D11_LC | . | . | . | . | . | . | . | . | . | . | . | . | . | . | . | . | . | Q | . | . | . | . | . | . | . | . |
| 21-225_6A11_LC | . | . | . | . | . | . | . | . | . | . | . | . | . | . | . | . | . | . | . | . | . | . | . | . | . | . |
| 21-225_6G2_LC | Q | . | . | . | . | . | . | . | . | . | . | . | . | . | . | . | . | . | . | . | . | . | . | . | . | . |
| 21-225_71A2_LC | . | . | . | . | . | I | . | . | . | . | . | . | . | . | . | F | R | . | . | I | . | . | . | . | . | . |
| 21-225_73C9_LC | . | . | . | . | . | . | . | . | . | . | . | . | . | . | . | . | . | . | . | . | . | . | . | . | . | . |
| 21-225_7C9_LC | . | . | . | . | . | . | . | . | . | . | . | . | . | . | . | . | . | . | . | . | . | . | . | . | . | . |
| 21-225_7F8_LC | . | . | . | . | . | . | . | . | . | . | . | . | . | . | . | . | . | . | . | . | . | . | . | . | . | . |
| 21-225_7G2_LC | . | . | . | . | . | T | . | . | . | . | . | . | . | . | . | . | . | . | . | . | . | . | . | . | . | . |
| 21-225_7G4_LC | . | . | . | . | . | . | . | . | . | . | . | . | . | . | . | . | . | . | . | . | . | . | . | . | . | . |
| 21-225_94F3_LC | Q | . | . | . | F | . | . | . | . | . | . | . | . | . | . | . | . | . | . | . | . | . | . | . | . | . |
| 21-225_9A1_LC | . | . | . | . | . | T | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | R | L | I | Y | A | - | - | - | - | - | - | - | - | A | S | S | L | Q | S | G | V | P | S | R | F |
| 21-225_9C11_LC | . | L | . | . | . | V | . | . | . | . | . | . | . | . | . | . | . | . | . | . | . | . | . | . | . | . |
| 21-225_9D12_LC | . | . | . | . | . | . | . | . | . | . | . | . | . | . | . | . | . | . | . | . | . | . | . | . | . | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | P | E | D | F | A | T | Y |
| 21-225_10E9_LC | . | . | . | . | . | . | . | . | . | . | . | . | . | . | . | N | . | . | . | . | . | . | . | . | . | . |
| 21-225_12B12_LC | . | . | . | . | . | . | . | . | . | . | . | . | . | . | . | . | . | . | . | . | . | . | . | . | . | . |
| 21-225_12E6_LC | . | . | . | . | . | . | . | . | . | . | . | . | . | . | . | . | . | . | . | . | . | . | . | . | . | . |
| 21-225_12F2_LC | . | . | . | . | . | . | . | . | . | . | . | . | . | . | . | . | . | . | . | . | . | . | . | V | . | . |
| 21-225_147H5_LC | . | . | . | . | . | . | . | . | . | . | . | . | . | . | . | . | . | . | . | . | . | . | . | . | . | . |
| 21-225_148C9_LC | . | . | . | . | . | . | . | . | . | . | . | . | . | . | . | . | . | . | . | . | . | . | . | . | . | . |
| 21-225_149D10_LC | . | . | . | . | . | . | . | . | . | . | . | . | . | . | . | . | . | . | . | . | . | . | . | . | . | . |
| 21-225_14E10_LC | . | . | . | . | . | . | . | . | . | . | . | . | . | . | . | . | . | . | . | . | . | . | . | T | A | . |
| 21-225_158D4_LC | . | . | . | . | . | . | . | . | . | . | . | . | . | . | F | . | . | . | . | . | . | . | . | . | . | . |
| 21-225_158G1_LC | . | . | . | . | C | . | . | . | . | . | . | . | . | . | V | . | . | . | . | . | . | . | . | . | . | . |
| 21-225_159A3_LC | . | . | . | . | . | . | . | . | . | . | . | . | . | . | F | . | . | . | . | . | . | . | . | . | . | . |
| 21-225_159C8_LC | . | . | . | . | . | . | . | . | . | . | . | . | . | . | F | . | . | . | . | . | . | . | . | . | . | . |
| 21-225_159D8_LC | . | . | . | . | . | . | . | . | . | . | . | . | . | . | F | . | . | . | . | . | . | . | . | . | . | . |
| 21-225_15C11_LC | . | . | . | . | . | . | . | . | . | . | . | . | . | . | . | . | . | . | . | . | . | . | . | . | . | . |
| 21-225_160C4_LC | . | . | . | . | . | . | . | . | . | . | . | . | . | . | F | . | . | . | . | . | . | . | . | . | . | . |
| 21-225_160F2_LC | . | . | . | . | . | . | . | . | . | . | . | . | . | . | V | . | . | . | . | . | . | . | . | . | . | . |
| 21-225_160H1_LC | . | . | . | . | . | . | . | . | . | . | . | . | . | . | . | . | . | . | . | . | . | . | . | T | . | . |
| 21-225_161G12_LC | . | . | . | . | . | . | . | . | . | . | . | . | . | . | F | . | . | . | . | . | . | . | . | . | . | . |
| 21-225_169D11_LC | . | . | . | . | . | . | . | . | . | . | . | . | . | . | . | . | . | . | . | . | . | . | . | . | . | . |
| 21-225_169D7_LC | . | . | . | . | . | . | . | . | . | . | . | . | . | . | . | . | . | . | . | . | . | . | . | . | . | . |
| 21-225_16A5_LC | . | . | N | . | Y | . | . | . | . | . | . | . | . | . | . | . | . | . | . | . | . | . | . | . | . | . |
| 21-225_16G7_LC | . | . | . | . | . | . | . | . | . | . | . | . | . | . | . | . | . | . | . | . | . | . | . | . | . | . |
| 21-225_16H4_LC | . | . | . | . | . | . | . | . | . | . | . | . | . | . | . | . | . | . | . | . | . | . | . | . | . | . |
| 21-225_170E4_LC | . | . | . | . | . | . | . | . | . | . | . | . | . | . | . | . | . | . | . | . | . | . | . | . | . | . |
| 21-225_170F3_LC | . | . | . | . | . | . | . | . | . | . | . | . | . | . | . | N | . | . | . | . | . | . | . | . | . | . |
| 21-225_170H1_LC | . | . | . | . | . | . | . | . | . | . | . | . | . | . | . | . | . | . | . | . | . | . | . | . | . | . |
| 21-225_174G10_LC | . | . | . | . | . | . | . | . | . | . | . | . | . | . | . | . | . | . | . | . | . | . | . | . | . | . |
| 21-225_17C7_LC | . | . | . | . | . | . | . | . | . | . | . | . | . | . | . | . | . | . | . | . | . | . | . | . | . | . |
| 21-225_17H3_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | P | E | D | F | A | T | Y |
| 21-225_18A5_LC | . | . | . | . | . | . | . | . | . | . | . | . | . | . | . | . | . | . | . | . | . | . | . | . | . | . |
| 21-225_18C2_LC | . | . | . | . | F | . | . | . | . | . | . | . | . | . | . | . | . | . | . | . | . | . | . | . | . | . |
| 21-225_18F2_LC | . | . | . | . | . | . | . | . | . | . | . | . | . | . | . | . | . | . | . | . | . | . | . | . | . | . |
| 21-225_18H12_LC | . | . | . | . | . | . | . | . | . | . | . | . | . | . | . | . | . | . | . | . | . | . | . | . | . | . |
| 21-225_190C12_LC | . | . | . | . | . | . | . | . | . | . | . | . | . | . | . | . | . | . | . | . | . | . | . | . | . | . |
| 21-225_190G3_LC | . | . | . | . | . | . | . | . | . | . | . | . | . | . | . | . | . | . | . | . | . | . | . | . | . | . |
| 21-225_190H5_LC | . | . | . | . | . | . | . | . | . | . | . | . | . | . | . | . | . | . | . | . | . | . | . | . | . | . |
| 21-225_191A4_LC | . | . | . | . | . | . | . | . | . | . | . | . | . | . | . | . | . | . | . | . | . | . | . | . | . | . |
| 21-225_192F7_LC | . | . | . | . | . | . | . | . | . | . | . | . | . | . | . | . | . | . | . | . | . | . | . | . | . | . |
| 21-225_196C12_LC | . | . | . | . | . | . | . | . | . | . | . | . | I | . | . | . | . | . | . | . | . | . | . | E | . | . |
| 21-225_19A10_LC | . | . | . | . | . | . | . | . | . | . | . | . | . | . | . | . | R | . | . | . | . | . | . | . | . | . |
| 21-225_200C9_LC | . | . | . | . | . | . | . | . | . | . | . | . | . | . | . | . | . | . | . | . | . | . | . | . | . | . |
| 21-225_201C5_LC | . | . | . | . | . | . | . | . | . | . | . | . | . | . | . | . | . | . | . | . | . | . | . | . | . | . |
| 21-225_203C10_LC | . | . | . | . | . | . | . | . | . | . | . | . | . | . | . | . | . | . | . | . | . | . | . | . | A | . |
| 21-225_203F7_LC | . | . | . | . | C | . | . | . | . | . | . | . | . | . | . | . | . | V | . | . | . | . | . | . | N | . |
| 21-225_204H3_LC | . | . | . | . | . | . | . | . | . | . | . | . | . | . | . | . | . | . | . | . | . | . | . | . | . | . |
| 21-225_208B2_LC | . | . | . | . | . | . | . | . | . | . | . | . | . | . | . | . | . | . | . | . | . | . | . | . | . | . |
| 21-225_208F1_LC | . | . | . | . | . | . | . | . | . | . | . | . | . | . | . | . | . | . | . | . | . | . | . | . | . | . |
| 21-225_20B11_LC | . | . | . | . | . | . | . | . | . | . | . | . | . | . | . | . | . | . | . | . | . | . | . | . | . | . |
| 21-225_20B2_LC | . | . | . | . | . | . | . | . | . | . | . | . | . | . | . | . | . | . | . | . | . | . | . | . | . | . |
| 21-225_20D10_LC | . | . | . | . | Y | . | . | . | . | . | . | . | . | . | . | . | . | . | . | . | . | . | . | . | . | . |
| 21-225_210G5_LC | . | . | . | . | . | . | . | . | . | . | . | . | . | . | . | . | . | . | . | . | . | . | . | . | . | . |
| 21-225_210G6_LC | . | . | . | . | C | . | . | . | . | . | . | . | . | . | . | . | . | V | . | R | . | . | . | . | N | . |
| 21-225_212B11_LC | . | . | . | . | . | . | . | . | . | . | . | I | . | . | . | . | . | . | . | . | . | . | . | . | . | . |
| 21-225_214G10_LC | . | . | . | . | . | . | . | . | . | . | . | I | . | . | . | . | . | . | . | . | . | . | . | . | . | . |
| 21-225_214G12_LC | . | . | . | . | . | . | . | . | . | . | . | I | . | . | . | . | . | . | . | . | . | . | . | . | . | . |
| 21-225_214H9_LC | . | . | . | . | . | . | . | . | . | . | . | . | . | . | . | . | . | . | . | . | . | . | . | . | . | . |
| 21-225_215E11_LC | . | . | . | . | . | . | . | . | . | . | . | I | . | . | . | . | . | V | . | R | . | . | . | . | . | . |
| 21-225_215E3_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | P | E | D | F | A | T | Y |
| 21-225_216E8_LC | . | . | . | . | . | . | . | . | . | . | . | I | . | . | . | . | . | . | . | . | . | . | . | . | . | . |
| 21-225_216H12_LC | . | . | . | . | . | . | . | . | . | . | . | I | . | . | . | . | . | . | . | . | . | . | . | . | . | . |
| 21-225_217E5_LC | . | . | . | . | . | . | . | . | . | . | . | I | . | . | . | . | . | . | . | . | . | . | . | . | . | . |
| 21-225_217G10_LC | . | . | . | . | . | . | . | . | . | . | . | I | . | . | . | . | . | . | . | . | . | . | . | . | . | . |
| 21-225_217H12_LC | . | . | . | . | . | . | . | . | . | . | . | I | . | . | . | . | . | . | . | . | . | . | . | . | . | . |
| 21-225_218C4_LC | . | . | . | . | . | . | . | . | . | . | . | I | . | . | . | . | . | V | . | R | . | . | . | . | . | . |
| 21-225_21F7_LC | . | . | . | . | . | . | . | . | . | . | . | . | . | . | . | . | . | . | . | . | . | . | . | . | . | . |
| 21-225_21G11_LC | . | . | . | . | . | . | . | . | . | . | . | . | . | . | . | . | . | . | . | . | . | . | . | . | . | . |
| 21-225_21G2_LC | . | . | . | . | . | . | . | . | . | . | . | . | . | . | . | . | . | . | . | . | . | . | . | . | . | . |
| 21-225_21H8_LC | R | . | . | . | . | . | . | . | . | . | . | . | . | . | . | . | . | . | . | . | . | . | . | . | . | . |
| 21-225_220F8_LC | . | . | . | . | . | . | . | . | . | . | . | I | . | . | . | . | . | . | . | R | . | . | . | . | . | . |
| 21-225_224E7_LC | . | . | . | . | . | . | . | . | . | . | . | . | . | . | . | . | . | . | . | . | . | . | . | . | . | . |
| 21-225_224F1_LC | . | . | . | . | . | . | . | . | . | . | . | . | . | . | . | . | . | . | . | . | . | . | . | . | I | . |
| 21-225_224F3_LC | . | . | . | . | . | . | . | . | . | . | . | . | . | . | . | . | . | . | . | . | . | . | . | . | . | . |
| 21-225_225A1_LC | . | . | . | . | . | . | . | . | . | . | . | . | . | . | . | . | . | . | . | . | . | . | . | . | . | . |
| 21-225_226C6_LC | . | . | . | . | . | . | . | . | . | . | . | . | . | . | . | . | N | . | . | . | . | . | . | . | . | . |
| 21-225_226F7_LC | . | . | . | V | . | . | . | . | . | . | . | I | . | . | . | . | . | . | . | . | . | . | . | . | . | . |
| 21-225_226H11_LC | . | . | . | . | . | . | . | . | . | D | . | . | . | . | . | . | . | . | . | . | . | . | . | . | . | . |
| 21-225_22A4_LC | . | . | . | . | . | . | . | . | . | . | . | . | . | . | . | . | . | . | . | . | . | . | . | . | . | . |
| 21-225_22F4_LC | . | . | . | R | . | . | . | . | . | D | . | . | . | . | . | . | . | . | . | . | . | . | . | . | . | . |
| 21-225_23A10_LC | . | . | . | . | . | . | . | . | . | . | . | . | . | . | . | G | . | . | . | . | . | . | . | . | . | . |
| 21-225_23E11_LC | . | . | . | . | . | . | . | . | . | . | . | . | . | . | . | . | . | . | . | . | . | . | . | . | . | . |
| 21-225_23F12_LC | . | . | . | . | . | . | . | . | . | . | . | . | . | . | . | . | . | . | . | . | . | . | . | . | S | . |
| 21-225_24B6_LC | . | . | . | . | . | . | . | . | . | . | . | . | . | . | . | . | . | . | . | T | . | . | . | . | . | . |
| 21-225_25D12_LC | . | . | . | . | . | . | . | . | . | . | . | . | . | . | . | . | . | . | . | . | . | . | . | . | . | . |
| 21-225_26A2_LC | . | . | . | . | . | . | . | . | . | . | . | . | . | . | F | . | . | . | . | . | . | . | . | . | . | . |
| 21-225_28C7_LC | . | . | . | . | . | . | . | . | . | . | . | . | . | . | . | . | . | . | . | . | . | . | . | . | . | . |
| 21-225_28F5_LC | . | . | . | . | . | . | . | . | . | . | . | . | . | . | . | . | . | . | . | . | . | . | . | . | . | . |
| 21-225_2G4_LC | . | . | . | . | . | . | . | . | . | . | . | . | . | . | . | . | . | . | . | . | . | . | L | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | P | E | D | F | A | T | Y |
| 21-225_32B6_LC | . | . | . | . | . | . | . | . | . | . | . | . | . | . | . | . | . | . | . | . | . | . | . | . | . | . |
| 21-225_33C2_LC | . | . | . | . | . | . | . | . | . | . | . | . | . | . | . | . | . | . | . | . | . | H | . | . | . | . |
| 21-225_44B3_LC | . | . | . | . | . | . | . | . | . | . | . | . | . | . | . | . | . | . | . | . | . | . | . | . | . | . |
| 21-225_44C10_LC | . | . | . | . | . | . | . | . | . | . | . | . | . | . | . | . | . | . | . | . | . | . | . | . | . | . |
| 21-225_44D3_LC | . | . | . | R | . | . | . | . | R | . | . | . | . | . | . | . | . | . | . | . | . | . | . | . | . | . |
| 21-225_44D5_LC | . | . | . | . | . | . | . | . | . | . | . | . | . | . | . | . | . | . | . | . | . | . | . | . | . | . |
| 21-225_44F9_LC | . | . | . | . | . | . | . | . | . | . | . | . | . | . | . | . | . | . | . | . | . | . | . | . | . | . |
| 21-225_45B4_LC | . | . | . | . | . | . | . | . | . | . | . | . | . | . | . | . | . | . | . | . | . | . | . | . | . | . |
| 21-225_45B8_LC | . | . | . | . | . | . | . | . | . | . | . | . | . | . | . | . | . | . | . | . | . | . | . | . | . | . |
| 21-225_46C3_LC | . | . | . | R | . | . | . | . | . | . | . | . | . | . | . | . | . | . | . | . | . | . | . | . | . | . |
| 21-225_46D4_LC | . | . | . | . | . | . | . | . | . | . | . | . | . | . | . | . | . | . | . | . | . | . | . | . | . | . |
| 21-225_48A9_LC | . | . | . | . | . | . | . | . | . | . | . | . | . | . | . | . | G | . | . | . | . | . | . | . | I | . |
| 21-225_48C1_LC | . | . | . | . | . | . | . | . | . | . | . | . | . | . | . | . | . | . | . | . | . | . | . | . | I | . |
| 21-225_48F2_LC | N | . | . | . | . | . | . | . | . | . | . | . | . | . | . | . | . | . | . | S | . | . | L | . | . | . |
| 21-225_4C12_LC | . | . | . | . | . | . | . | . | . | . | . | . | . | . | . | . | . | . | . | . | . | . | L | . | . | . |
| 21-225_4C5_LC | . | . | . | . | Y | . | . | . | . | . | . | . | . | . | . | G | . | . | . | . | . | . | . | . | . | . |
| 21-225_50A12_LC | . | . | . | . | . | . | . | . | . | . | . | . | . | . | . | . | . | . | . | . | . | . | . | . | . | . |
| 21-225_50H4_LC | . | . | . | . | . | . | . | . | . | . | . | . | . | . | . | . | . | . | . | . | . | . | . | . | . | . |
| 21-225_51H8_LC | . | . | G | . | . | . | . | . | . | H | . | . | . | . | . | . | . | . | . | . | . | . | . | . | . | . |
| 21-225_54H3_LC | . | . | . | . | . | . | . | . | . | . | . | . | . | . | . | . | . | . | . | . | . | . | . | . | . | . |
| 21-225_56C7_LC | . | . | . | . | . | . | . | . | . | . | . | . | . | . | . | . | . | . | . | . | . | . | . | . | . | . |
| 21-225_57B8_LC | . | . | . | . | . | . | . | . | . | . | . | . | . | . | . | . | . | . | . | . | . | . | . | . | . | . |
| 21-225_59B9_LC | . | . | T | R | . | . | . | . | . | . | . | . | . | . | . | . | . | . | . | . | . | . | . | . | . | . |
| 21-225_59G7_LC | . | . | . | . | . | . | . | . | . | . | . | . | . | . | . | . | . | . | . | . | . | . | . | . | . | . |
| 21-225_5E5_LC | . | . | . | . | . | . | . | . | . | . | . | . | . | . | . | . | . | . | . | . | . | . | . | . | . | . |
| 21-225_5E5.003_LC | . | . | . | . | . | . | . | . | . | . | . | . | . | . | . | . | . | . | . | . | . | . | . | . | . | . |
| 21-225_5E5.004_LC | . | . | . | . | . | . | . | . | . | . | . | . | . | . | . | . | . | . | . | . | . | . | . | . | . | . |
| 21-225_5E5.005_LC | . | . | . | . | . | . | . | . | . | . | . | . | . | . | . | . | . | . | . | . | . | . | . | . | . | . |
| 21-225_5E5.006_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | P | E | D | F | A | T | Y |
| 21-225_5E5.011_LC | . | . | . | . | . | . | . | . | . | . | . | . | . | . | . | . | . | . | . | . | . | . | . | . | . | . |
| 21-225_5E5.012_LC | . | . | . | . | . | . | . | . | . | . | . | . | . | . | . | . | . | . | . | . | . | . | . | . | . | . |
| 21-225_5E5.015_LC | . | . | . | . | . | . | . | . | . | . | . | . | . | . | . | . | . | . | . | . | . | . | . | . | . | . |
| 21-225_5E5.016_LC | . | . | . | . | . | . | . | . | . | . | . | . | . | . | . | . | . | . | . | . | . | . | . | . | . | . |
| 21-225_5E5.017_LC | . | . | . | . | . | . | . | . | . | . | . | . | . | . | . | . | . | . | . | . | . | . | . | . | . | . |
| 21-225_5E5.018_LC | . | . | . | . | . | . | . | . | . | . | . | . | . | . | . | . | . | . | . | . | . | . | . | . | . | . |
| 21-225_5E5.019_LC | . | . | . | . | . | . | . | . | . | . | . | . | . | . | . | . | . | . | . | . | . | . | . | . | . | . |
| 21-225_5E5.023_LC | . | . | . | . | . | . | . | . | . | . | . | . | . | . | . | . | . | . | . | . | . | . | . | . | . | . |
| 21-225_5E5.024_LC | . | . | . | . | . | . | . | . | . | . | . | . | . | . | . | . | . | . | . | . | . | . | . | . | . | . |
| 21-225_5E5.025_LC | . | . | . | . | . | . | . | . | . | . | . | . | . | . | . | . | . | . | . | . | . | . | . | . | . | . |
| 21-225_60C3_LC | . | . | . | . | . | . | . | . | . | . | . | . | . | . | . | . | . | . | . | . | . | . | . | . | . | . |
| 21-225_60E2_LC | . | . | . | . | . | . | . | . | . | . | . | . | . | . | . | . | . | . | . | . | . | . | . | . | . | . |
| 21-225_61E6_LC | . | . | . | . | . | . | . | . | . | . | . | . | . | . | . | . | . | . | . | . | . | . | . | . | . | . |
| 21-225_61H1_LC | . | . | . | . | . | . | . | . | . | . | . | . | . | . | . | . | . | . | . | . | . | . | . | . | . | . |
| 21-225_62F7_LC | . | . | . | . | . | . | . | . | . | . | . | . | . | . | . | . | . | . | . | . | . | . | . | . | . | . |
| 21-225_64A4_LC | . | . | . | . | . | . | . | . | . | . | . | . | . | . | . | . | . | . | . | . | . | . | L | . | . | . |
| 21-225_64C8_LC | . | . | . | . | . | . | . | . | . | . | . | . | . | . | . | . | . | . | . | . | . | . | . | . | . | . |
| 21-225_66C10_LC | . | . | . | . | . | . | . | . | . | . | . | . | . | . | . | . | . | . | . | . | . | . | . | . | . | . |
| 21-225_66D11_LC | . | . | . | . | C | . | . | . | . | . | . | . | I | S | . | . | . | M | . | R | . | . | . | . | . | . |
| 21-225_6A11_LC | . | . | . | . | . | . | . | . | . | . | . | . | . | . | . | . | . | . | . | . | . | . | . | . | . | . |
| 21-225_6G2_LC | . | . | . | . | Y | . | . | . | . | . | . | N | I | . | . | . | . | . | . | . | . | . | . | . | I | . |
| 21-225_71A2_LC | . | . | . | . | . | . | . | . | . | . | . | . | . | . | . | . | . | . | . | . | . | . | . | . | . | . |
| 21-225_73C9_LC | . | . | . | . | . | . | . | . | . | . | . | . | . | . | . | . | . | . | . | . | . | . | . | . | . | . |
| 21-225_7C9_LC | . | . | . | . | . | . | . | . | . | . | . | . | . | . | . | . | . | . | . | . | . | . | . | . | . | . |
| 21-225_7F8_LC | . | . | . | V | . | . | . | . | . | . | . | . | . | P | . | . | . | . | . | . | . | . | . | . | . | . |
| 21-225_7G2_LC | . | . | . | . | . | . | . | . | . | . | . | . | . | . | . | . | . | . | . | . | . | . | . | . | . | . |
| 21-225_7G4_LC | . | . | . | . | . | . | . | . | . | . | . | . | . | . | . | . | . | . | . | . | . | . | S | . | . | . |
| 21-225_94F3_LC | . | . | . | . | . | . | . | . | . | . | . | . | . | . | . | . | . | . | . | . | . | . | . | . | . | . |
| 21-225_9A1_LC | . | . | . | . | Y | . | . | . | . | . | . | . | I | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | P | E | D | F | A | T | Y |
| 21-225_9C11_LC | . | . | . | . | . | . | . | . | . | . | . | . | . | . | . | N | . | . | . | . | . | . | . | . | I | . |
| 21-225_9D12_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | L | Q | H | Y | S | - | - | - | - | - | - | - | - | - | - | - | - | - | - | - | - | - | - | - |
| 21-225_10E9_LC | . | . | . | . | . | . | . | . | . | . | . | . | . | . | . | . | . | . | . | . | . | . | . | . | . | . |
| 21-225_12B12_LC | . | . | . | . | . | N | . | . | . | . | . | . | . | . | . | . | . | . | . | . | . | . | . | . | . | . |
| 21-225_12E6_LC | . | . | . | . | . | N | . | . | . | . | . | . | . | . | . | . | . | . | . | . | . | . | . | . | . | . |
| 21-225_12F2_LC | . | . | . | . | . | N | . | . | . | . | . | . | . | . | . | . | . | . | . | . | . | . | . | . | . | . |
| 21-225_147H5_LC | . | . | . | . | . | . | . | . | . | . | . | . | . | . | . | . | . | . | . | . | . | . | . | . | . | . |
| 21-225_148C9_LC | . | . | . | . | . | . | . | . | . | . | . | . | . | . | . | . | . | . | . | . | . | . | . | . | . | . |
| 21-225_149D10_LC | . | . | . | . | . | . | . | . | . | . | . | . | . | . | . | . | . | . | . | . | . | . | . | . | . | . |
| 21-225_14E10_LC | . | . | . | . | . | . | . | . | . | . | . | . | . | . | . | . | . | . | . | . | . | . | . | . | . | . |
| 21-225_158D4_LC | . | . | . | . | . | . | . | . | . | . | . | . | . | . | . | . | . | . | . | . | . | . | . | . | . | . |
| 21-225_158G1_LC | . | . | . | . | . | N | . | . | . | . | . | . | . | . | . | . | . | . | . | . | . | . | . | . | . | . |
| 21-225_159A3_LC | . | . | . | . | . | H | . | . | . | . | . | . | . | . | . | . | . | . | . | . | . | . | . | . | . | . |
| 21-225_159C8_LC | . | . | . | . | . | H | . | . | . | . | . | . | . | . | . | . | . | . | . | . | . | . | . | . | . | . |
| 21-225_159D8_LC | . | . | . | . | . | . | . | . | . | . | . | . | . | . | . | . | . | . | . | . | . | . | . | . | . | . |
| 21-225_15C11_LC | . | . | . | L | . | . | . | . | . | . | . | . | . | . | . | . | . | . | . | . | . | . | . | . | . | . |
| 21-225_160C4_LC | . | . | . | . | . | . | . | . | . | . | . | . | . | . | . | . | . | . | . | . | . | . | . | . | . | . |
| 21-225_160F2_LC | . | . | . | . | . | N | . | . | . | . | . | . | . | . | . | . | . | . | . | . | . | . | . | . | . | . |
| 21-225_160H1_LC | . | . | . | . | . | N | . | . | . | . | . | . | . | . | . | . | . | . | . | . | . | . | . | . | . | . |
| 21-225_161G12_LC | . | . | . | . | . | H | . | . | . | . | . | . | . | . | . | . | . | . | . | . | . | . | . | . | . | . |
| 21-225_169D11_LC | . | . | . | . | . | . | . | . | . | . | . | . | . | . | . | . | . | . | . | . | . | . | . | . | . | . |
| 21-225_169D7_LC | . | . | . | . | . | H | . | . | . | . | . | . | . | . | . | . | . | . | . | . | . | . | . | . | . | . |
| 21-225_16A5_LC | . | . | . | . | . | N | . | . | . | . | . | . | . | . | . | . | . | . | . | . | . | . | . | . | . | . |
| 21-225_16G7_LC | . | . | . | . | . | N | . | . | . | . | . | . | . | . | . | . | . | . | . | . | . | . | . | . | . | . |
| 21-225_16H4_LC | . | . | . | . | . | N | . | . | . | . | . | . | . | . | . | . | . | . | . | . | . | . | . | . | . | . |
| 21-225_170E4_LC | . | . | . | . | . | N | . | . | . | . | . | . | . | . | . | . | . | . | . | . | . | . | . | . | . | . |
| 21-225_170F3_LC | . | . | . | . | . | . | . | . | . | . | . | . | . | . | . | . | . | . | . | . | . | . | . | . | . | . |
| 21-225_170H1_LC | . | . | . | . | . | S | . | . | . | . | . | . | . | . | . | . | . | . | . | . | . | . | . | . | . | . |
| 21-225_174G10_LC | . | . | . | . | . | H | . | . | . | . | . | . | . | . | . | . | . | . | . | . | . | . | . | . | . | . |
| 21-225_17C7_LC | . | . | . | . | . | N | . | . | . | . | . | . | . | . | . | . | . | . | . | . | . | . | . | . | . | . |
| 21-225_17H3_LC | . | . | . | . | Q | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | L | Q | H | Y | S | - | - | - | - | - | - | - | - | - | - | - | - | - | - | - | - | - | - | - |
| 21-225_18A5_LC | . | . | . | . | . | N | . | . | . | . | . | . | . | . | . | . | . | . | . | . | . | . | . | . | . | . |
| 21-225_18C2_LC | . | . | . | . | . | N | . | . | . | . | . | . | . | . | . | . | . | . | . | . | . | . | . | . | . | . |
| 21-225_18F2_LC | . | . | . | . | . | N | . | . | . | . | . | . | . | . | . | . | . | . | . | . | . | . | . | . | . | . |
| 21-225_18H12_LC | . | . | . | . | . | N | N | . | . | . | . | . | . | . | . | . | . | . | . | . | . | . | . | . | . | . |
| 21-225_190C12_LC | . | . | . | . | . | N | . | . | . | . | . | . | . | . | . | . | . | . | . | . | . | . | . | . | . | . |
| 21-225_190G3_LC | . | . | . | . | . | N | . | . | . | . | . | . | . | . | . | . | . | . | . | . | . | . | . | . | . | . |
| 21-225_190H5_LC | . | . | . | . | . | N | N | . | . | . | . | . | . | . | . | . | . | . | . | . | . | . | . | . | . | . |
| 21-225_191A4_LC | . | . | . | . | . | N | . | . | . | . | . | . | . | . | . | . | . | . | . | . | . | . | . | . | . | . |
| 21-225_192F7_LC | . | . | . | . | . | T | . | . | . | . | . | . | . | . | . | . | . | . | . | . | . | . | . | . | . | . |
| 21-225_196C12_LC | . | . | . | . | . | N | D | . | . | . | . | . | . | . | . | . | . | . | . | . | . | . | . | . | . | . |
| 21-225_19A10_LC | . | . | . | . | . | N | G | . | . | . | . | . | . | . | . | . | . | . | . | . | . | . | . | . | . | . |
| 21-225_200C9_LC | . | . | . | . | . | N | . | . | . | . | . | . | . | . | . | . | . | . | . | . | . | . | . | . | . | . |
| 21-225_201C5_LC | . | . | . | . | . | . | . | . | . | . | . | . | . | . | . | . | . | . | . | . | . | . | . | . | . | . |
| 21-225_203C10_LC | . | . | . | . | . | N | . | . | . | . | . | . | . | . | . | . | . | . | . | . | . | . | . | . | . | . |
| 21-225_203F7_LC | . | . | . | . | . | . | . | . | . | . | . | . | . | . | . | . | . | . | . | . | . | . | . | . | . | . |
| 21-225_204H3_LC | . | . | . | . | . | . | . | . | . | . | . | . | . | . | . | . | . | . | . | . | . | . | . | . | . | . |
| 21-225_208B2_LC | . | . | . | . | . | . | . | . | . | . | . | . | . | . | . | . | . | . | . | . | . | . | . | . | . | . |
| 21-225_208F1_LC | . | . | . | . | . | . | . | . | . | . | . | . | . | . | . | . | . | . | . | . | . | . | . | . | . | . |
| 21-225_20B11_LC | . | . | . | . | . | . | . | . | . | . | . | . | . | . | . | . | . | . | . | . | . | . | . | . | . | . |
| 21-225_20B2_LC | . | . | . | . | . | . | N | . | . | . | . | . | . | . | . | . | . | . | . | . | . | . | . | . | . | . |
| 21-225_20D10_LC | . | . | I | . | Q | N | . | . | . | . | . | . | . | . | . | . | . | . | . | . | . | . | . | . | . | . |
| 21-225_210G5_LC | . | . | . | H | . | . | . | . | . | . | . | . | . | . | . | . | . | . | . | . | . | . | . | . | . | . |
| 21-225_210G6_LC | . | . | V | . | . | . | . | . | . | . | . | . | . | . | . | . | . | . | . | . | . | . | . | . | . | . |
| 21-225_212B11_LC | . | . | . | L | . | . | . | . | . | . | . | . | . | . | . | . | . | . | . | . | . | . | . | . | . | . |
| 21-225_214G10_LC | . | . | . | L | . | N | . | . | . | . | . | . | . | . | . | . | . | . | . | . | . | . | . | . | . | . |
| 21-225_214G12_LC | . | . | V | M | . | . | . | . | . | . | . | . | . | . | . | . | . | . | . | . | . | . | . | . | . | . |
| 21-225_214H9_LC | . | . | . | . | . | . | . | . | . | . | . | . | . | . | . | . | . | . | . | . | . | . | . | . | . | . |
| 21-225_215E11_LC | . | . | V | M | . | . | . | . | . | . | . | . | . | . | . | . | . | . | . | . | . | . | . | . | . | . |
| 21-225_215E3_LC | . | . | V | M | . | N | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | L | Q | H | Y | S | - | - | - | - | - | - | - | - | - | - | - | - | - | - | - | - | - | - | - |
| 21-225_216E8_LC | . | . | . | M | . | . | . | . | . | . | . | . | . | . | . | . | . | . | . | . | . | . | . | . | . | . |
| 21-225_216H12_LC | . | . | V | M | . | N | . | . | . | . | . | . | . | . | . | . | . | . | . | . | . | . | . | . | . | . |
| 21-225_217E5_LC | . | . | V | M | . | N | . | . | . | . | . | . | . | . | . | . | . | . | . | . | . | . | . | . | . | . |
| 21-225_217G10_LC | . | . | V | M | . | N | . | . | . | . | . | . | . | . | . | . | . | . | . | . | . | . | . | . | . | . |
| 21-225_217H12_LC | . | . | . | M | . | . | . | . | . | . | . | . | . | . | . | . | . | . | . | . | . | . | . | . | . | . |
| 21-225_218C4_LC | . | . | V | M | . | . | . | . | . | . | . | . | . | . | . | . | . | . | . | . | . | . | . | . | . | . |
| 21-225_21F7_LC | . | . | . | . | . | N | N | . | . | . | . | . | . | . | . | . | . | . | . | . | . | . | . | . | . | . |
| 21-225_21G11_LC | . | . | . | . | . | N | N | . | . | . | . | . | . | . | . | . | . | . | . | . | . | . | . | . | . | . |
| 21-225_21G2_LC | . | . | . | . | . | N | . | . | . | . | . | . | . | . | . | . | . | . | . | . | . | . | . | . | . | . |
| 21-225_21H8_LC | . | . | . | . | . | N | . | . | . | . | . | . | . | . | . | . | . | . | . | . | . | . | . | . | . | . |
| 21-225_220F8_LC | . | . | V | M | . | N | . | . | . | . | . | . | . | . | . | . | . | . | . | . | . | . | . | . | . | . |
| 21-225_224E7_LC | . | . | . | . | . | . | . | . | . | . | . | . | . | . | . | . | . | . | . | . | . | . | . | . | . | . |
| 21-225_224F1_LC | . | . | . | . | . | . | . | . | . | . | . | . | . | . | . | . | . | . | . | . | . | . | . | . | . | . |
| 21-225_224F3_LC | . | . | . | . | . | . | N | . | . | . | . | . | . | . | . | . | . | . | . | . | . | . | . | . | . | . |
| 21-225_225A1_LC | . | . | . | . | . | . | . | . | . | . | . | . | . | . | . | . | . | . | . | . | . | . | . | . | . | . |
| 21-225_226C6_LC | . | . | . | . | . | . | N | . | . | . | . | . | . | . | . | . | . | . | . | . | . | . | . | . | . | . |
| 21-225_226F7_LC | . | . | . | H | . | . | . | . | . | . | . | . | . | . | . | . | . | . | . | . | . | . | . | . | . | . |
| 21-225_226H11_LC | . | . | . | . | . | . | . | . | . | . | . | . | . | . | . | . | . | . | . | . | . | . | . | . | . | . |
| 21-225_22A4_LC | . | . | . | . | . | N | . | . | . | . | . | . | . | . | . | . | . | . | . | . | . | . | . | . | . | . |
| 21-225_22F4_LC | . | . | . | H | . | . | . | . | . | . | . | . | . | . | . | . | . | . | . | . | . | . | . | . | . | . |
| 21-225_23A10_LC | . | . | . | . | . | . | . | . | . | . | . | . | . | . | . | . | . | . | . | . | . | . | . | . | . | . |
| 21-225_23E11_LC | . | . | . | . | . | N | . | . | . | . | . | . | . | . | . | . | . | . | . | . | . | . | . | . | . | . |
| 21-225_23F12_LC | . | . | . | . | . | N | . | . | . | . | . | . | . | . | . | . | . | . | . | . | . | . | . | . | . | . |
| 21-225_24B6_LC | . | . | . | . | . | . | . | . | . | . | . | . | . | . | . | . | . | . | . | . | . | . | . | . | . | . |
| 21-225_25D12_LC | . | . | . | . | . | . | . | . | . | . | . | . | . | . | . | . | . | . | . | . | . | . | . | . | . | . |
| 21-225_26A2_LC | . | . | . | . | . | N | R | . | . | . | . | . | . | . | . | . | . | . | . | . | . | . | . | . | . | . |
| 21-225_28C7_LC | . | . | . | . | . | N | T | . | . | . | . | . | . | . | . | . | . | . | . | . | . | . | . | . | . | . |
| 21-225_28F5_LC | . | . | . | . | . | N | T | . | . | . | . | . | . | . | . | . | . | . | . | . | . | . | . | . | . | . |
| 21-225_2G4_LC | . | . | . | . | . | . | . | . | . | . | . | . | . | . | . | . | . | . | . | . | . | . | . | . | . | . |

# FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | L | Q | H | Y | S | - | - | - | - | - | - | - | - | - | - | - | - | - | - | - | - | - | - | - |
| 21-225_32B6_LC | . | . | . | . | . | N | . | . | . | . | . | . | . | . | . | . | . | . | . | . | . | . | . | . | . | . |
| 21-225_33C2_LC | . | . | . | . | . | N | . | . | . | . | . | . | . | . | . | . | . | . | . | . | . | . | . | . | . | . |
| 21-225_44B3_LC | . | . | . | L | . | . | N | . | . | . | . | . | . | . | . | . | . | . | . | . | . | . | . | . | . | . |
| 21-225_44C10_LC | . | . | . | . | . | . | . | . | . | . | . | . | . | . | . | . | . | . | . | . | . | . | . | . | . | . |
| 21-225_44D3_LC | . | . | . | . | . | . | . | . | . | . | . | . | . | . | . | . | . | . | . | . | . | . | . | . | . | . |
| 21-225_44D5_LC | . | . | . | . | . | . | . | . | . | . | . | . | . | . | . | . | . | . | . | . | . | . | . | . | . | . |
| 21-225_44F9_LC | . | . | . | H | . | . | N | . | . | . | . | . | . | . | . | . | . | . | . | . | . | . | . | . | . | . |
| 21-225_45B4_LC | . | . | . | . | . | . | . | . | . | . | . | . | . | . | . | . | . | . | . | . | . | . | . | . | . | . |
| 21-225_45B8_LC | . | . | . | . | . | . | N | . | . | . | . | . | . | . | . | . | . | . | . | . | . | . | . | . | . | . |
| 21-225_46C3_LC | . | . | . | . | . | . | . | . | . | . | . | . | . | . | . | . | . | . | . | . | . | . | . | . | . | . |
| 21-225_46D4_LC | . | . | . | . | . | . | . | . | . | . | . | . | . | . | . | . | . | . | . | . | . | . | . | . | . | . |
| 21-225_48A9_LC | . | . | . | . | . | N | R | . | . | . | . | . | . | . | . | . | . | . | . | . | . | . | . | . | . | . |
| 21-225_48C1_LC | . | . | . | . | . | N | F | . | . | . | . | . | . | . | . | . | . | . | . | . | . | . | . | . | . | . |
| 21-225_48F2_LC | . | . | . | . | Q | . | . | . | . | . | . | . | . | . | . | . | . | . | . | . | . | . | . | . | . | . |
| 21-225_4C12_LC | . | . | . | . | . | . | . | . | . | . | . | . | . | . | . | . | . | . | . | . | . | . | . | . | . | . |
| 21-225_4C5_LC | . | . | . | . | . | . | . | . | . | . | . | . | . | . | . | . | . | . | . | . | . | . | . | . | . | . |
| 21-225_50A12_LC | . | . | . | . | . | N | . | . | . | . | . | . | . | . | . | . | . | . | . | . | . | . | . | . | . | . |
| 21-225_50H4_LC | . | . | . | . | . | . | . | . | . | . | . | . | . | . | . | . | . | . | . | . | . | . | . | . | . | . |
| 21-225_51H8_LC | . | . | . | . | . | N | . | . | . | . | . | . | . | . | . | . | . | . | . | . | . | . | . | . | . | . |
| 21-225_54H3_LC | . | . | . | . | Y | N | . | . | . | . | . | . | . | . | . | . | . | . | . | . | . | . | . | . | . | . |
| 21-225_56C7_LC | . | . | . | . | . | N | . | . | . | . | . | . | . | . | . | . | . | . | . | . | . | . | . | . | . | . |
| 21-225_57B8_LC | . | . | . | . | . | N | . | . | . | . | . | . | . | . | . | . | . | . | . | . | . | . | . | . | . | . |
| 21-225_59B9_LC | . | . | . | . | . | N | . | . | . | . | . | . | . | . | . | . | . | . | . | . | . | . | . | . | . | . |
| 21-225_59G7_LC | . | . | . | . | . | N | . | . | . | . | . | . | . | . | . | . | . | . | . | . | . | . | . | . | . | . |
| 21-225_5E5_LC | . | . | . | . | . | . | . | . | . | . | . | . | . | . | . | . | . | . | . | . | . | . | . | . | . | . |
| 21-225_5E5.003_LC | . | . | . | . | . | . | . | . | . | . | . | . | . | . | . | . | . | . | . | . | . | . | . | . | . | . |
| 21-225_5E5.004_LC | . | . | . | . | . | . | . | . | . | . | . | . | . | . | . | . | . | . | . | . | . | . | . | . | . | . |
| 21-225_5E5.005_LC | . | . | . | . | . | . | . | . | . | . | . | . | . | . | . | . | . | . | . | . | . | . | . | . | . | . |
| 21-225_5E5.006_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | Y | S | - | - | - | - | - | - | - | - | - | - | - | - | - | - | - | - | - | - | - |
| 21-225_5E5.011_LC | . | . | . | . | . | . | . | . | . | . | . | . | . | . | . | . | . | . | . | . | . | . | . | . | . | . |
| 21-225_5E5.012_LC | . | . | . | . | . | . | . | . | . | . | . | . | . | . | . | . | . | . | . | . | . | . | . | . | . | . |
| 21-225_5E5.015_LC | . | . | . | . | . | . | . | . | . | . | . | . | . | . | . | . | . | . | . | . | . | . | . | . | . | . |
| 21-225_5E5.016_LC | . | . | . | . | . | . | . | . | . | . | . | . | . | . | . | . | . | . | . | . | . | . | . | . | . | . |
| 21-225_5E5.017_LC | . | . | . | . | . | . | . | . | . | . | . | . | . | . | . | . | . | . | . | . | . | . | . | . | . | . |
| 21-225_5E5.018_LC | . | . | . | . | . | . | . | . | . | . | . | . | . | . | . | . | . | . | . | . | . | . | . | . | . | . |
| 21-225_5E5.019_LC | . | . | . | . | . | . | . | . | . | . | . | . | . | . | . | . | . | . | . | . | . | . | . | . | . | . |
| 21-225_5E5.023_LC | . | . | . | . | . | . | . | . | . | . | . | . | . | . | . | . | . | . | . | . | . | . | . | . | . | . |
| 21-225_5E5.024_LC | . | . | . | . | . | . | . | . | . | . | . | . | . | . | . | . | . | . | . | . | . | . | . | . | . | . |
| 21-225_5E5.025_LC | . | . | . | . | . | . | . | . | . | . | . | . | . | . | . | . | . | . | . | . | . | . | . | . | . | . |
| 21-225_60C3_LC | . | . | . | . | . | N | . | . | . | . | . | . | . | . | . | . | . | . | . | . | . | . | . | . | . | . |
| 21-225_60E2_LC | . | . | . | . | . | N | . | . | . | . | . | . | . | . | . | . | . | . | . | . | . | . | . | . | . | . |
| 21-225_61E6_LC | . | . | . | . | . | N | . | . | . | . | . | . | . | . | . | . | . | . | . | . | . | . | . | . | . | . |
| 21-225_61H1_LC | . | . | . | . | . | N | . | . | . | . | . | . | . | . | . | . | . | . | . | . | . | . | . | . | . | . |
| 21-225_62F7_LC | . | . | . | . | Y | N | . | . | . | . | . | . | . | . | . | . | . | . | . | . | . | . | . | . | . | . |
| 21-225_64A4_LC | . | . | . | . | . | . | . | . | . | . | . | . | . | . | . | . | . | . | . | . | . | . | . | . | . | . |
| 21-225_64C8_LC | . | . | . | H | . | . | . | . | . | . | . | . | . | . | . | . | . | . | . | . | . | . | . | . | . | . |
| 21-225_66C10_LC | . | . | . | . | Y | N | . | . | . | . | . | . | . | . | . | . | . | . | . | . | . | . | . | . | . | . |
| 21-225_66D11_LC | . | . | I | V | . | N | . | . | . | . | . | . | . | . | . | . | . | . | . | . | . | . | . | . | . | . |
| 21-225_6A11_LC | . | . | . | . | . | N | . | . | . | . | . | . | . | . | . | . | . | . | . | . | . | . | . | . | . | . |
| 21-225_6G2_LC | . | . | I | . | . | N | . | . | . | . | . | . | . | . | . | . | . | . | . | . | . | . | . | . | . | . |
| 21-225_71A2_LC | . | . | I | H | . | N | . | . | . | . | . | . | . | . | . | . | . | . | . | . | . | . | . | . | . | . |
| 21-225_73C9_LC | . | . | . | . | . | . | . | . | . | . | . | . | . | . | . | . | . | . | . | . | . | . | . | . | . | . |
| 21-225_7C9_LC | . | . | . | . | L | . | . | . | . | . | . | . | . | . | . | . | . | . | . | . | . | . | . | . | . | . |
| 21-225_7F8_LC | . | . | . | . | . | . | T | . | . | . | . | . | . | . | . | . | . | . | . | . | . | . | . | . | . | . |
| 21-225_7G2_LC | . | . | . | . | . | N | . | . | . | . | . | . | . | . | . | . | . | . | . | . | . | . | . | . | . | . |
| 21-225_7G4_LC | . | . | . | H | Q | . | . | . | . | . | . | . | . | . | . | . | . | . | . | . | . | . | . | . | . | . |
| 21-225_94F3_LC | . | . | . | . | . | . | . | . | . | . | . | . | . | . | . | . | . | . | . | . | . | . | . | . | . | . |
| 21-225_9A1_LC | . | . | I | . | . | N | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | L | Q | H | Y | S | - | - | - | - | - | - | - | - | - | - | - | - | - | - | - | - | - | - | - |
| 21-225_9C11_LC | H | . | H | . | S | N | . | . | . | . | . | . | . | . | . | . | . | . | . | . | . | . | . | . | . | . |
| 21-225_9D12_LC | . | . | . | . | . | N | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | R | T | F | G | Q | G | T | K | V | E | I | K | R |
| 21-225_10E9_LC | . | . | . | . | . | . | . | . | . | . | . | . | . | . | . | . | . | . | . |
| 21-225_12B12_LC | . | . | . | . | . | . | W | . | . | . | L | . | . | . | . | V | . | . | . |
| 21-225_12E6_LC | . | . | . | . | . | . | W | . | . | . | . | . | . | . | . | . | . | . | . |
| 21-225_12F2_LC | . | . | . | . | . | . | W | . | . | . | . | . | . | . | . | . | . | . | . |
| 21-225_147H5_LC | . | . | . | . | . | . | . | . | . | . | . | . | . | . | . | . | . | . | . |
| 21-225_148C9_LC | . | . | . | . | . | . | . | . | . | . | . | . | . | . | . | . | . | . | . |
| 21-225_149D10_LC | . | . | . | . | . | . | . | . | . | . | . | . | . | . | . | . | . | . | . |
| 21-225_14E10_LC | . | . | . | . | . | . | . | . | . | . | . | . | . | . | . | . | . | . | . |
| 21-225_158D4_LC | . | . | . | . | . | . | . | . | . | . | . | . | . | . | . | . | . | . | . |
| 21-225_158G1_LC | . | . | . | . | . | . | W | . | . | . | . | . | . | . | . | . | . | . | . |
| 21-225_159A3_LC | . | . | . | . | . | . | . | . | . | . | . | . | . | . | . | . | . | . | . |
| 21-225_159C8_LC | . | . | . | . | . | . | . | . | . | . | . | . | . | . | . | . | . | . | . |
| 21-225_159D8_LC | . | . | . | . | . | . | . | . | . | . | . | . | . | . | . | . | . | . | . |
| 21-225_15C11_LC | . | . | . | . | . | . | . | . | . | . | R | . | . | . | . | . | . | . | . |
| 21-225_160C4_LC | . | . | . | . | . | . | . | . | . | . | . | . | . | . | . | . | . | . | . |
| 21-225_160F2_LC | . | . | . | . | . | . | W | . | . | . | . | . | . | . | . | . | . | . | . |
| 21-225_160H1_LC | . | . | . | . | F | . | W | . | . | . | . | . | . | . | . | . | . | . | . |
| 21-225_161G12_LC | . | . | . | . | . | . | . | . | . | . | . | . | . | . | . | . | . | . | . |
| 21-225_169D11_LC | . | . | . | . | . | . | . | . | . | . | . | . | . | . | . | . | . | . | . |
| 21-225_169D7_LC | . | . | . | . | C | . | W | . | . | . | . | . | . | . | . | . | . | . | . |
| 21-225_16A5_LC | . | . | . | . | . | . | W | . | . | . | . | . | . | . | . | . | . | . | . |
| 21-225_16G7_LC | . | . | . | . | . | . | W | . | . | . | . | . | . | . | . | . | . | . | . |
| 21-225_16H4_LC | . | . | . | . | . | . | W | . | . | . | . | . | . | . | . | . | . | . | . |
| 21-225_170E4_LC | . | . | . | Y | F | . | W | . | . | . | . | . | . | . | . | D | . | . | . |
| 21-225_170F3_LC | . | . | . | . | . | . | . | . | . | . | . | . | . | . | . | . | . | . | . |
| 21-225_170H1_LC | . | . | . | . | N | . | W | . | . | . | . | . | . | . | . | . | S | . | . |
| 21-225_174G10_LC | . | . | . | . | . | . | . | . | . | . | . | . | . | . | . | . | . | . | . |
| 21-225_17C7_LC | . | . | . | . | . | . | W | . | . | . | . | . | . | . | . | . | . | . | . |
| 21-225_17H3_LC | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | R | T | F | G | Q | G | T | K | V | E | I | K | R |
| 21-225_18A5_LC | . | . | . | . | . | . | W | . | . | . | . | . | . | . | . | . | . | . | . |
| 21-225_18C2_LC | . | . | . | . | . | . | W | . | . | . | L | . | . | . | . | V | . | . | . |
| 21-225_18F2_LC | . | . | . | . | . | . | W | . | . | . | . | . | . | . | . | . | . | . | . |
| 21-225_18H12_LC | . | . | . | . | . | . | W | . | . | . | L | . | . | . | . | V | . | . | . |
| 21-225_190C12_LC | . | . | . | . | . | . | W | . | . | . | . | . | . | . | . | . | . | . | . |
| 21-225_190G3_LC | . | . | . | . | . | . | W | . | . | . | . | . | . | . | . | . | . | . | . |
| 21-225_190H5_LC | . | . | . | . | . | . | W | . | . | . | . | . | . | . | . | . | . | . | . |
| 21-225_191A4_LC | . | . | . | . | . | . | W | . | . | . | . | . | . | . | . | . | . | . | . |
| 21-225_192F7_LC | . | . | . | . | . | . | W | . | . | . | . | . | . | . | . | . | . | Y | . |
| 21-225_196C12_LC | . | . | . | . | F | . | F | . | . | . | R | . | . | . | . | . | . | . | . |
| 21-225_19A10_LC | . | . | . | . | . | . | W | . | . | . | . | . | . | . | . | . | . | . | . |
| 21-225_200C9_LC | . | . | . | . | . | . | W | . | . | . | . | . | . | . | . | . | . | . | . |
| 21-225_201C5_LC | . | . | . | . | F | . | . | . | . | . | . | . | . | . | . | . | . | . | . |
| 21-225_203C10_LC | . | . | . | . | . | . | W | . | . | . | . | . | . | . | . | . | . | . | . |
| 21-225_203F7_LC | . | . | . | . | F | . | . | . | . | . | . | . | . | . | . | . | . | . | . |
| 21-225_204H3_LC | . | . | . | . | . | . | . | . | . | . | . | . | . | . | . | . | L | Q | . |
| 21-225_208B2_LC | . | . | . | . | . | . | . | . | . | . | . | . | . | . | . | . | . | . | . |
| 21-225_208F1_LC | . | . | . | . | F | . | . | . | . | . | . | . | . | . | . | . | . | . | . |
| 21-225_20B11_LC | . | . | . | . | . | . | . | . | . | . | . | . | . | . | . | . | . | . | . |
| 21-225_20B2_LC | . | . | . | . | . | . | . | A | . | . | . | . | . | . | . | D | . | . | . |
| 21-225_20D10_LC | . | . | . | . | . | . | W | . | . | . | . | . | . | . | . | . | . | . | . |
| 21-225_210G5_LC | . | . | . | . | . | . | . | . | . | . | . | . | . | . | . | . | . | . | . |
| 21-225_210G6_LC | . | . | . | . | F | . | . | . | . | . | . | . | . | . | . | . | . | . | . |
| 21-225_212B11_LC | . | . | . | . | . | . | . | . | . | . | . | . | . | . | . | . | . | . | . |
| 21-225_214G10_LC | . | . | . | . | . | . | . | . | . | . | . | . | . | . | . | . | . | . | . |
| 21-225_214G12_LC | . | . | . | . | . | . | . | . | . | . | . | . | . | . | . | . | . | . | . |
| 21-225_214H9_LC | . | . | . | . | . | . | . | . | . | . | . | . | . | . | . | . | . | . | . |
| 21-225_215E11_LC | . | . | . | . | . | . | . | . | . | . | . | . | . | . | . | . | . | . | . |
| 21-225_215E3_LC | . | . | . | . | . | . | . | . | . | . | . | . | . | . | . | . | . | . | . |

# FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | R | T | F | G | Q | G | T | K | V | E | I | K | R |
| 21-225_216E8_LC | . | . | . | . | . | . | . | . | . | . | . | . | . | . | . | . | . | . | . |
| 21-225_216H12_LC | . | . | . | . | . | . | . | . | . | . | . | . | . | . | . | . | . | . | . |
| 21-225_217E5_LC | . | . | . | . | . | . | . | . | . | . | . | . | . | . | . | . | . | . | . |
| 21-225_217G10_LC | . | . | . | . | . | . | . | . | . | . | . | . | . | . | . | . | . | . | . |
| 21-225_217H12_LC | . | . | . | . | . | . | . | . | . | . | . | . | . | . | . | . | . | . | . |
| 21-225_218C4_LC | . | . | . | . | . | . | . | . | . | . | . | . | . | . | . | . | . | . | . |
| 21-225_21F7_LC | . | . | . | . | . | . | W | . | . | . | . | . | . | . | . | . | . | . | . |
| 21-225_21G11_LC | . | . | . | . | . | . | W | . | . | . | L | . | . | . | . | V | . | . | . |
| 21-225_21G2_LC | . | . | . | . | . | . | W | . | . | . | L | . | . | . | . | V | . | . | . |
| 21-225_21H8_LC | . | . | . | . | . | . | W | . | . | . | . | . | . | . | . | . | . | . | . |
| 21-225_220F8_LC | . | . | . | . | . | . | . | . | . | . | . | . | . | . | . | . | . | . | . |
| 21-225_224E7_LC | . | . | . | . | . | . | . | . | . | . | . | . | . | . | . | . | . | . | . |
| 21-225_224F1_LC | . | . | . | . | . | . | . | . | . | . | . | . | . | . | . | . | . | . | . |
| 21-225_224F3_LC | . | . | . | . | . | . | . | A | . | . | . | . | . | . | . | . | . | . | . |
| 21-225_225A1_LC | . | . | . | . | . | . | . | . | . | . | . | . | . | . | . | . | . | . | . |
| 21-225_226C6_LC | . | . | . | . | . | . | . | A | . | . | . | . | . | . | . | . | . | Q | . |
| 21-225_226F7_LC | . | . | . | . | . | . | . | . | . | . | . | . | . | . | . | . | . | . | . |
| 21-225_226H11_LC | . | . | . | . | . | . | . | . | . | . | . | . | . | . | . | . | . | . | . |
| 21-225_22A4_LC | . | . | . | . | . | . | W | . | . | . | . | . | . | . | . | . | . | . | . |
| 21-225_22F4_LC | . | . | . | . | . | . | . | . | . | . | . | . | . | . | . | . | . | . | . |
| 21-225_23A10_LC | . | . | . | . | . | . | . | . | . | . | L | . | . | . | . | . | . | . | . |
| 21-225_23E11_LC | . | . | . | . | . | . | W | . | . | . | . | . | . | . | . | . | . | . | . |
| 21-225_23F12_LC | . | . | . | . | . | . | W | . | . | . | . | . | . | . | . | . | . | . | . |
| 21-225_24B6_LC | . | . | . | . | . | . | . | . | . | . | . | . | . | . | . | . | V | . | . |
| 21-225_25D12_LC | . | . | . | . | F | . | . | . | . | . | . | . | . | . | . | . | . | . | . |
| 21-225_26A2_LC | . | . | . | . | . | . | W | . | . | . | . | . | . | . | . | . | . | . | . |
| 21-225_28C7_LC | . | . | . | . | . | . | W | . | . | . | . | . | . | . | . | . | . | . | . |
| 21-225_28F5_LC | . | . | . | . | . | . | W | . | . | . | . | . | . | . | . | . | V | . | . |
| 21-225_2G4_LC | . | . | . | . | F | . | . | . | . | . | R | . | . | . | . | . | . | . | . |

# FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | R | T | F | G | Q | G | T | K | V | E | I | K | R |
| 21-225_32B6_LC | . | . | . | . | . | . | W | . | . | . | . | . | . | . | . | . | . | . | . |
| 21-225_33C2_LC | . | . | . | . | . | . | W | . | . | . | . | . | . | . | . | . | . | . | . |
| 21-225_44B3_LC | . | . | . | . | . | . | . | . | . | . | . | . | . | . | . | . | . | . | . |
| 21-225_44C10_LC | . | . | . | . | . | . | . | . | . | . | . | . | . | . | . | . | . | . | . |
| 21-225_44D3_LC | . | . | . | . | . | . | . | . | . | . | . | . | . | . | . | . | . | . | . |
| 21-225_44D5_LC | . | . | . | . | F | . | W | . | . | . | . | . | . | . | . | . | . | . | . |
| 21-225_44F9_LC | . | . | . | . | . | . | . | . | . | . | . | . | . | . | . | . | . | T | . |
| 21-225_45B4_LC | . | . | . | . | . | . | . | . | . | . | . | . | . | . | . | . | . | . | . |
| 21-225_45B8_LC | . | . | . | . | . | . | . | . | . | . | . | . | . | . | . | . | . | . | . |
| 21-225_46C3_LC | . | . | . | . | . | . | . | . | . | . | . | . | . | . | . | . | . | . | . |
| 21-225_46D4_LC | . | . | . | . | F | . | W | . | . | . | . | . | . | . | . | . | . | . | . |
| 21-225_48A9_LC | . | . | . | . | . | . | W | . | . | . | . | . | . | . | . | . | . | . | . |
| 21-225_48C1_LC | . | . | . | . | . | . | W | . | . | . | . | . | . | . | . | . | . | . | . |
| 21-225_48F2_LC | . | . | . | . | . | . | . | . | . | . | . | . | . | . | . | . | . | . | . |
| 21-225_4C12_LC | . | . | . | . | F | . | . | . | . | . | . | . | . | . | . | . | . | . | . |
| 21-225_4C5_LC | . | . | . | . | . | . | . | . | . | . | . | . | . | . | . | . | . | . | . |
| 21-225_50A12_LC | . | . | . | . | . | . | W | . | . | . | . | . | . | . | . | . | . | . | . |
| 21-225_50H4_LC | . | . | . | . | . | . | . | . | . | . | . | . | . | . | . | . | . | . | . |
| 21-225_51H8_LC | . | . | . | . | . | . | W | . | . | . | . | . | . | . | . | . | . | . | . |
| 21-225_54H3_LC | . | . | . | . | . | . | W | . | . | . | . | . | . | . | . | . | . | . | . |
| 21-225_56C7_LC | . | . | . | . | . | . | W | . | . | . | . | . | . | . | . | . | . | . | . |
| 21-225_57B8_LC | . | . | . | F | P | . | W | . | . | . | . | . | . | . | . | . | . | . | . |
| 21-225_59B9_LC | . | . | . | . | . | . | W | . | . | . | . | . | . | . | . | . | . | . | . |
| 21-225_59G7_LC | . | . | . | . | . | . | W | . | . | . | . | . | . | . | . | . | . | . | . |
| 21-225_5E5_LC | . | . | . | . | . | . | . | . | . | . | . | . | . | . | . | . | V | . | . |
| 21-225_5E5.003_LC | . | . | . | . | . | . | . | . | . | . | . | . | . | . | . | . | V | . | . |
| 21-225_5E5.004_LC | . | . | . | . | . | . | . | . | . | . | . | . | . | . | . | . | V | . | . |
| 21-225_5E5.005_LC | . | . | . | . | . | . | . | . | . | . | . | . | . | . | . | . | V | . | . |
| 21-225_5E5.006_LC | . | . | . | . | . | . | . | . | . | . | . | . | . | . | . | . | V | . | . |

# FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | R | T | F | G | Q | G | T | K | V | E | I | K | R |
| 21-225_5E5.011_LC | . | . | . | . | . | . | . | . | . | . | . | . | . | . | . | . | V | . | . |
| 21-225_5E5.012_LC | . | . | . | . | . | . | . | . | . | . | . | . | . | . | . | . | V | . | . |
| 21-225_5E5.015_LC | . | . | . | . | . | . | . | . | . | . | . | . | . | . | . | . | V | . | . |
| 21-225_5E5.016_LC | . | . | . | . | . | . | . | . | . | . | . | . | . | . | . | . | V | . | . |
| 21-225_5E5.017_LC | . | . | . | . | . | . | . | . | . | . | . | . | . | . | . | . | V | . | . |
| 21-225_5E5.018_LC | . | . | . | . | . | . | . | . | . | . | . | . | . | . | . | . | V | . | . |
| 21-225_5E5.019_LC | . | . | . | . | . | . | . | . | . | . | . | . | . | . | . | . | V | . | . |
| 21-225_5E5.023_LC | . | . | . | . | . | . | . | . | . | . | . | . | . | . | . | . | V | . | . |
| 21-225_5E5.024_LC | . | . | . | . | . | . | . | . | . | . | . | . | . | . | . | . | V | . | . |
| 21-225_5E5.025_LC | . | . | . | . | . | . | . | . | . | . | . | . | . | . | . | . | V | . | . |
| 21-225_60C3_LC | . | . | . | . | . | . | W | . | . | . | L | . | . | . | . | . | . | . | . |
| 21-225_60E2_LC | . | . | . | . | . | . | W | . | . | . | . | . | . | . | . | . | . | . | . |
| 21-225_61E6_LC | . | . | . | F | P | . | W | . | . | . | . | . | . | . | . | . | . | . | . |
| 21-225_61H1_LC | . | . | . | . | . | . | W | . | . | . | . | . | . | . | . | . | . | . | . |
| 21-225_62F7_LC | . | . | . | Y | P | . | W | . | . | . | . | . | S | . | . | . | . | . | . |
| 21-225_64A4_LC | . | . | . | . | . | . | . | . | . | . | . | . | . | . | . | . | . | . | . |
| 21-225_64C8_LC | . | . | . | . | . | . | . | . | . | . | . | . | . | . | . | . | . | . | . |
| 21-225_66C10_LC | . | . | . | Y | P | . | W | . | . | . | . | . | S | . | . | . | . | . | . |
| 21-225_66D11_LC | . | . | . | . | . | . | . | . | . | . | . | . | . | . | . | . | . | . | . |
| 21-225_6A11_LC | . | . | . | . | . | . | W | . | . | . | . | . | . | . | . | . | . | . | . |
| 21-225_6G2_LC | . | . | . | . | . | L | W | . | . | . | . | . | . | . | . | . | . | T | . |
| 21-225_71A2_LC | . | . | . | . | . | . | W | . | . | . | . | . | . | . | . | . | . | . | . |
| 21-225_73C9_LC | . | . | . | . | . | . | . | . | . | . | . | . | . | . | . | . | . | . | . |
| 21-225_7C9_LC | . | . | . | . | . | . | . | . | . | . | . | . | . | . | . | D | . | . | . |
| 21-225_7F8_LC | . | . | . | . | . | . | . | . | . | . | . | . | . | . | . | . | . | . | . |
| 21-225_7G2_LC | . | . | . | . | . | . | W | . | . | . | L | . | . | . | . | V | . | . | . |
| 21-225_7G4_LC | . | . | . | . | . | . | . | . | . | . | . | . | . | . | . | . | . | . | . |
| 21-225_94F3_LC | . | . | . | . | . | . | . | . | . | . | R | . | . | . | . | A | . | . | . |
| 21-225_9A1_LC | . | . | . | . | . | L | W | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | R | T | F | G | Q | G | T | K | V | E | I | K | R |
| 21-225_9C11_LC | . | . | . | . | T | . | L | . | . | . | . | . | . | . | . | . | . | . | . |
| 21-225_9D12_LC | . | . | . | . | . | . | W | . | . | . | . | . | . | . | . | . | . | . | . |

**FIGURE 57**

Table 99. Consensus 19 - VK4|B3/JK1 (SEQ ID NO: 50316):

DIVMTQSPDSLAVSLGERATINCKSS--QSVLHSSNNNNYLAWYQQKPGQPPKLLIYW--------ASTRESGVPDRFSGSGSG--TDFTLTISSLQAEDVAVYYCQQYYS----------------------TPPTFGQGTKVEIKR wherein:

K at position 24 can be substituted with R or M

S at position 26 can be substituted with G or R

S at position 30 can be substituted with T, N or I

V at position 31 can be substituted with I or A

H at position 33 can be substituted with Y, F, S, K, D, L or M

S at position 34 can be substituted with T, R, N, I or D

S at position 35 can be substituted with F or P

N at position 36 can be substituted with H

N at position 37 can be substituted with K, S, D or H

N at position 38 can be substituted with Y, K, H, R, A, F or W

N at position 39 can be substituted with H or Y

Y at position 40 can be substituted with S

L at position 41 can be substituted with F

A at position 42 can be substituted with T, V or G

A at position 67 can be substituted with T or S

S at position 68 can be substituted with F

T at position 69 can be substituted with I, K or S

**FIGURE 57**

R at position 70 can be substituted with W or L

E at position 71 can be substituted with K, A, D or R

S at position 72 can be substituted with T

Q at position 107 can be substituted with H or L

Q at position 108 can be substituted with H

Y at position 109 can be substituted with F

Y at position 110 can be substituted with F, H, N, L or S

S at position 111 can be substituted with N, R, D, I, T, C, E or K

T at position 135 can be substituted with S, I, V, A or Y

P at position 137 can be substituted with W, L, V, C, G, R or S

T at position 138 can be substituted with K or S

Xaa Xaa Xaa Gln Ser Xaa Leu Xaa Xaa Xaa Xaa Xaa Xaa Xaa Xaa Xaa Xaa (SEQ ID NO: 50133)

Trp Xaa Xaa Xaa Xaa Xaa Xaa (SEQ ID NO: 50229)

Xaa Xaa Xaa Xaa Xaa Xaa Pro Xaa Xaa (SEQ ID NO: 50134)

Lys Ser Ser Gln Ser Val Leu His Ser Ser Asn Asn Asn Asn Tyr Leu Ala (SEQ ID NO: 50618)

Trp Ala Ser Thr Arg Glu Ser (SEQ ID NO: 50619)

Gln Gln Tyr Tyr Ser Thr Pro Pro Thr (SEQ ID NO: 50620)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | V | M | T | Q | S | P | D | S | L | A | V | S | L | G | E | R | A | T | I | N | C | K | S | S |
| 21-225_11E5_LC | . | . | . | . | . | . | . | . | . | . | . | . | . | . | . | . | . | . | . | . | L | . | . | . | . | . |
| 21-225_12F12_LC | . | . | . | . | . | . | . | . | . | . | . | . | . | . | . | . | . | . | . | . | . | . | . | . | . | . |
| 21-225_147E4_LC | . | . | . | . | . | . | . | . | . | . | . | . | . | . | . | . | . | . | . | . | . | . | . | . | . | . |
| 21-225_148F8_LC | . | . | . | . | . | . | . | L | . | . | . | . | . | . | . | . | . | . | . | . | . | . | . | . | . | . |
| 21-225_149A2_LC | . | . | . | . | . | . | . | . | . | . | . | . | . | . | . | . | . | . | . | . | . | . | . | . | . | . |
| 21-225_149E3_LC | . | . | . | . | . | . | . | . | . | . | . | . | . | . | . | . | . | . | . | . | . | S | . | . | . | . |
| 21-225_149H4_LC | . | . | . | . | . | . | . | . | . | . | . | . | . | . | . | . | . | . | . | . | . | . | . | . | . | . |
| 21-225_154A11_LC | . | . | . | . | . | . | . | . | . | . | . | . | . | . | . | . | . | . | . | . | . | . | . | R | . | . |
| 21-225_158B12_LC | . | . | . | . | . | . | . | . | . | . | P | . | . | . | . | . | . | . | . | . | . | . | . | . | . | . |
| 21-225_162F6_LC | . | . | . | . | . | . | . | . | . | . | . | . | . | . | . | . | . | . | . | . | . | . | . | . | . | . |
| 21-225_164A4_LC | . | . | . | . | . | . | Y | . | . | . | R | . | . | . | . | . | . | . | . | . | . | K | . | . | . | . |
| 21-225_164A7_LC | . | . | . | . | . | . | C | . | . | . | . | . | . | . | . | . | . | . | . | . | . | . | . | . | . | . |
| 21-225_170F6_LC | . | . | . | . | . | . | . | . | . | . | . | . | . | . | . | . | A | . | . | . | . | . | . | . | . | . |
| 21-225_174G5_LC | . | . | . | . | . | . | . | . | . | . | . | . | . | . | . | . | A | . | . | . | . | . | . | . | . | . |
| 21-225_175D10_LC | . | . | . | . | . | . | . | . | . | . | . | . | . | . | . | . | . | . | . | . | . | . | . | . | . | . |
| 21-225_177B12_LC | . | . | . | . | . | . | . | . | . | . | . | . | . | . | . | . | . | . | . | . | V | . | . | . | . | . |
| 21-225_181E5_LC | . | . | . | . | . | . | . | . | . | . | . | . | . | . | . | . | . | . | . | . | . | . | . | . | . | . |
| 21-225_190D7_LC | . | . | . | . | . | . | . | . | . | . | . | . | . | . | . | . | . | . | . | . | . | . | . | . | . | . |
| 21-225_191D8_LC | . | . | M | . | . | . | . | . | . | . | . | . | . | . | . | . | . | . | . | I | . | S | . | R | . | . |
| 21-225_193G4_LC | . | . | . | . | . | . | . | . | . | . | . | . | . | . | . | . | . | . | . | . | . | . | . | . | . | . |
| 21-225_196G8_LC | . | . | . | . | . | . | . | . | . | . | . | T | . | . | . | . | . | . | . | . | V | . | . | . | . | . |
| 21-225_197C6_LC | . | . | M | . | . | . | . | . | . | . | . | . | . | . | . | . | . | . | . | I | . | S | . | R | . | . |
| 21-225_197H4_LC | . | . | M | . | . | . | . | S | . | . | . | T | . | . | . | . | . | . | . | I | . | S | . | R | . | . |
| 21-225_200G7_LC | . | . | . | . | . | . | . | . | . | . | P | T | A | . | . | . | . | . | . | . | V | K | . | . | . | . |
| 21-225_201F5_LC | . | . | . | . | . | . | . | . | E | . | . | . | . | . | . | . | . | . | . | . | . | . | . | . | . | . |
| 21-225_213H12_LC | . | . | . | . | . | . | . | . | . | . | . | . | . | . | . | . | . | . | . | . | . | T | . | . | . | . |
| 21-225_213H7_LC | . | . | . | . | . | . | . | . | . | . | . | . | . | . | . | . | . | . | . | . | . | . | . | . | . | . |
| 21-225_222H3_LC | . | . | . | . | . | . | . | . | . | . | . | . | . | . | . | . | . | . | . | . | . | . | . | . | . | . |
| 21-225_223G10_LC | . | . | . | . | . | . | . | . | . | . | . | . | . | . | . | . | . | . | . | . | . | . | . | . | . | . |

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | V | M | T | Q | S | P | D | S | L | A | V | S | L | G | E | R | A | T | I | N | C | K | S | S |
| 21-225_224D8_LC | . | . | . | . | . | . | . | . | . | . | . | . | . | . | . | . | . | . | . | A | . | . | . | . | . | . |
| 21-225_224H5_LC | . | . | . | . | . | . | . | . | . | . | . | . | . | . | . | . | . | . | . | . | . | . | . | . | . | . |
| 21-225_225F11_LC | . | . | . | . | . | . | . | . | . | . | . | . | . | . | . | . | . | . | . | . | . | . | . | . | . | . |
| 21-225_225F5_LC | . | . | . | . | . | . | . | . | . | . | . | . | . | . | . | . | . | . | . | . | . | . | . | . | . | . |
| 21-225_226A12_LC | . | . | . | . | . | . | . | . | . | . | . | . | . | . | . | . | . | . | . | . | . | . | . | . | . | . |
| 21-225_226D11_LC | . | . | . | . | . | . | . | . | . | . | . | . | . | . | . | . | . | . | . | . | . | . | . | . | . | . |
| 21-225_226F6_LC | . | . | . | . | . | . | . | . | . | . | . | . | . | . | . | . | . | . | . | . | . | . | . | . | . | . |
| 21-225_227C7_LC | . | . | . | . | . | . | . | . | . | . | . | . | . | . | . | . | . | . | . | . | . | . | . | R | . | . |
| 21-225_23A12_LC | . | . | . | . | . | . | . | . | . | . | . | . | . | . | . | . | . | . | . | . | . | . | . | . | . | . |
| 21-225_23G12_LC | . | . | . | . | . | . | . | . | . | . | . | . | . | . | . | . | . | . | . | . | . | . | . | . | . | . |
| 21-225_24E1_LC | . | . | . | . | . | . | . | . | . | . | . | . | . | . | . | . | . | . | . | . | . | . | . | . | . | . |
| 21-225_25A4_LC | . | . | . | . | . | . | P | . | . | . | . | . | . | . | . | . | . | . | . | . | . | K | . | . | . | . |
| 21-225_25A4.001_LC | . | . | . | . | . | . | P | . | . | . | . | . | . | . | . | . | . | . | . | . | . | K | . | . | . | . |
| 21-225_25A4.001.001_LC | . | . | . | . | . | . | P | . | . | . | . | . | . | . | . | . | . | . | . | . | . | K | . | . | . | . |
| 21-225_25A4.001.002_LC | . | . | . | . | . | . | P | . | . | . | . | . | . | . | . | . | . | . | . | . | . | K | . | . | . | . |
| 21-225_25A4.001.003_LC | . | . | . | . | . | . | P | . | . | . | . | . | . | . | . | . | . | . | . | . | . | K | . | . | . | . |
| 21-225_25A4.001.004_LC | . | . | . | . | . | . | P | . | . | . | . | . | . | . | . | . | . | . | . | . | . | K | . | . | . | . |
| 21-225_25A4.001.005_LC | . | . | . | . | . | . | . | . | . | . | . | . | . | . | . | . | . | . | . | . | . | K | . | . | . | . |
| 21-225_25A4.001.006_LC | . | . | . | . | . | . | . | . | . | . | . | . | . | . | . | . | . | . | . | . | . | K | . | . | . | . |
| 21-225_25A4.001.007_LC | . | . | . | . | . | . | . | . | . | . | . | . | . | . | . | . | . | . | . | . | . | K | . | . | . | . |
| 21-225_25A4.001.008_LC | . | . | . | . | . | . | . | . | . | . | . | . | . | . | . | . | . | . | . | . | . | K | . | . | . | . |
| 21-225_25A4.001.013_LC | . | . | . | . | . | . | P | . | . | . | . | . | . | . | . | . | . | . | . | . | . | K | . | . | . | . |
| 21-225_25A4.001.019_LC | . | . | . | . | . | . | . | . | . | . | . | . | . | . | . | . | . | . | . | . | . | . | . | . | . | . |
| 21-225_25A4.001.020_LC | . | . | . | . | . | . | . | . | . | . | . | . | . | . | . | . | . | . | . | . | . | . | . | . | . | . |
| 21-225_25A4.001.021_LC | . | . | . | . | . | . | . | . | . | . | . | . | . | . | . | . | . | . | . | . | . | . | . | . | . | . |
| 21-225_25A4.001.029_LC | . | . | . | . | . | . | P | . | . | . | . | . | . | . | . | . | . | . | . | . | . | K | . | . | . | . |
| 21-225_26C4_LC | . | . | . | . | . | . | . | . | . | . | . | . | . | . | . | . | . | . | . | . | . | . | . | . | . | . |
| 21-225_29E6_LC | . | . | . | . | . | . | P | . | . | . | . | . | . | . | . | . | . | . | . | . | . | . | . | . | . | . |
| 21-225_29H8_LC | . | . | . | . | . | . | . | . | . | . | . | . | . | . | . | . | . | . | . | . | . | . | . | R | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | V | M | T | Q | S | P | D | S | L | A | V | S | L | G | E | R | A | T | I | N | C | K | S | S |
| 21-225_43D8_LC | . | . | . | . | . | . | . | . | . | . | . | . | . | . | . | . | . | . | . | . | . | . | . | . | . | . |
| 21-225_4A2.001.023_LC | . | . | . | . | . | . | . | . | . | . | . | . | . | . | . | . | . | . | . | . | . | . | . | . | . | . |
| 21-225_4A2.001.028_LC | . | . | . | . | . | . | . | . | . | . | . | . | . | . | . | . | . | . | . | . | . | . | . | . | . | . |
| 21-225_4A2.001.029_LC | . | . | . | . | . | . | . | . | . | . | . | . | . | . | . | . | . | . | . | . | . | . | . | . | . | . |
| 21-225_4A2.001.031_LC | . | . | . | . | . | . | . | . | . | . | . | . | . | . | . | . | . | . | . | . | . | . | . | . | . | . |
| 21-225_56A1_LC | . | . | . | . | . | . | . | . | . | . | . | . | . | . | . | . | . | . | . | . | . | . | . | . | . | . |
| 21-225_57A11_LC | . | . | . | . | . | . | . | . | . | . | . | T | . | . | . | . | . | . | . | . | . | . | . | . | . | . |
| 21-225_58B9_LC | . | . | . | . | . | . | . | . | . | . | . | . | . | . | . | . | . | . | . | . | . | . | . | . | . | G |
| 21-225_59F10_LC | . | . | . | . | . | . | P | . | . | . | . | . | . | . | . | . | . | . | . | . | . | . | . | . | . | . |
| 21-225_61B5_LC | . | . | . | . | . | . | . | . | . | . | . | . | . | . | . | . | . | . | . | . | . | . | . | . | . | . |
| 21-225_70E7_LC | . | . | . | . | . | . | . | . | . | . | . | . | . | . | . | . | . | . | . | . | L | . | . | . | . | . |
| 21-225_74A6_LC | . | . | . | . | . | . | . | . | . | . | . | . | . | . | P | . | . | . | . | . | . | . | . | . | . | . |
| 21-225_74A7_LC | . | . | . | . | . | . | . | . | . | . | . | . | . | . | . | . | . | . | . | . | . | . | . | . | . | . |
| 21-225_74B1_LC | . | . | . | . | . | . | . | . | . | P | . | . | . | . | . | . | . | . | . | . | T | . | . | . | . | . |
| 21-225_74B10_LC | . | . | . | . | . | . | . | . | . | . | . | . | . | . | . | . | . | . | . | . | . | . | . | . | . | . |
| 21-225_74B5_LC | . | . | . | . | . | . | . | . | . | . | . | . | . | . | . | . | . | . | . | . | . | . | . | . | . | . |
| 21-225_74B7_LC | . | . | . | . | . | . | . | . | . | . | . | . | . | . | . | . | . | . | . | . | . | . | . | . | . | . |
| 21-225_74B8_LC | . | S | . | . | . | . | . | . | . | . | . | . | . | . | . | . | . | . | . | . | . | . | . | . | . | . |
| 21-225_74C1_LC | . | . | . | . | . | . | C | . | . | . | P | . | . | . | . | . | . | . | . | S | . | . | . | . | . | . |
| 21-225_74F6_LC | . | . | . | . | . | . | . | . | . | . | . | . | . | . | . | . | . | . | . | . | . | . | . | . | . | . |
| 21-225_74F9_LC | . | . | . | . | . | . | . | . | . | . | . | . | . | . | . | . | . | . | . | . | . | . | . | . | . | . |
| 21-225_74G4_LC | . | . | . | . | . | . | . | . | . | . | . | . | . | . | . | . | . | . | . | . | . | . | . | . | . | . |
| 21-225_74H2_LC | . | . | . | . | . | . | . | . | . | . | . | . | . | . | . | . | . | . | . | . | . | . | . | . | . | . |
| 21-225_75A10_LC | N | . | . | . | . | . | . | . | . | . | . | . | . | . | . | . | A | . | . | . | . | . | . | . | . | . |
| 21-225_75A7_LC | . | . | . | . | . | . | . | . | . | . | . | . | . | . | . | . | . | . | . | . | . | . | . | . | . | . |
| 21-225_75C4_LC | . | . | . | . | . | . | . | . | . | . | . | . | . | . | . | . | . | . | . | . | . | . | . | . | . | . |
| 21-225_75F9_LC | . | . | . | . | . | . | . | . | . | . | . | P | . | . | . | . | . | . | . | . | V | . | . | . | . | . |
| 21-225_76E11_LC | . | . | . | . | . | . | . | . | . | . | . | . | . | . | . | . | . | G | . | . | . | . | . | . | . | R |
| 21-225_76E8_LC | . | . | . | . | . | . | . | . | . | . | . | . | . | . | . | . | . | . | . | . | . | . | . | . | . | . |

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | V | M | T | Q | S | P | D | S | L | A | V | S | L | G | E | R | A | T | I | N | C | K | S | S |
| 21-225_76F3_LC | . | . | . | . | . | . | C | . | . | . | P | . | . | . | . | . | . | . | . | . | . | . | . | . | . | . |
| 21-225_76F5_LC | V | . | . | L | . | . | . | . | . | . | . | . | . | . | . | . | . | . | . | . | . | . | . | . | . | . |
| 21-225_76G2_LC | . | . | . | . | . | . | . | . | . | . | . | . | . | . | . | . | . | . | . | . | . | . | . | . | . | . |
| 21-225_77C10_LC | . | . | . | . | . | . | . | . | . | C | . | . | . | . | . | . | . | . | . | . | . | . | . | . | . | . |
| 21-225_77C7_LC | . | . | . | . | . | . | . | . | . | . | . | . | . | . | . | . | . | . | . | . | . | . | . | . | . | . |
| 21-225_77E1_LC | . | . | . | . | . | . | . | . | . | . | . | . | . | . | . | . | . | . | . | . | . | . | . | . | . | . |
| 21-225_78C1_LC | . | . | . | . | . | . | . | . | . | C | . | . | . | . | . | . | . | . | . | . | . | . | . | . | . | . |
| 21-225_78E11_LC | . | . | . | . | . | . | . | . | . | . | . | . | . | . | . | . | . | . | . | . | . | . | . | . | . | . |
| 21-225_78H12_LC | . | . | . | . | . | . | . | . | . | . | . | . | . | . | . | . | . | . | . | . | . | . | . | . | . | . |
| 21-225_79E9_LC | . | . | . | . | . | . | . | . | . | . | . | . | . | . | . | . | . | . | . | . | . | . | . | . | . | . |
| 21-225_79F12_LC | . | . | . | . | . | . | . | . | . | . | . | . | . | . | . | . | . | . | . | . | . | . | . | . | . | . |
| 21-225_79G10_LC | . | . | . | . | . | . | . | . | . | . | . | . | . | . | . | . | . | . | . | . | . | . | . | . | . | . |
| 21-225_79G6_LC | . | . | . | . | . | . | . | . | . | C | . | . | . | . | . | . | . | . | . | . | . | S | . | M | . | . |
| 21-225_80D3_LC | . | . | . | . | . | . | . | . | . | C | . | . | . | . | . | . | . | . | . | . | . | . | . | . | . | . |
| 21-225_80E9_LC | . | . | . | . | . | . | . | . | . | C | . | . | . | . | . | . | . | . | . | . | . | . | . | . | . | . |
| 21-225_80H3_LC | . | . | . | . | . | . | . | . | . | . | . | . | . | . | . | . | . | . | . | . | T | . | . | . | . | . |
| 21-225_81E5_LC | . | . | . | . | . | . | . | . | . | . | . | . | . | . | . | . | . | . | . | . | . | . | . | . | . | . |
| 21-225_81F9_LC | . | . | . | . | . | . | . | . | . | C | . | . | . | . | . | . | . | . | . | . | . | . | . | . | . | . |
| 21-225_83F3_LC | . | . | . | . | . | . | . | . | . | C | . | . | . | . | . | . | . | . | . | . | . | . | . | . | . | . |
| 21-225_83G3_LC | . | . | . | . | . | . | . | . | . | C | . | . | . | . | . | . | . | . | . | . | . | . | . | . | . | . |
| 21-225_83G7_LC | . | . | . | . | . | . | . | . | . | . | . | . | . | . | . | . | . | . | . | . | . | I | . | . | . | . |
| 21-225_84G7_LC | . | . | . | . | . | . | . | . | . | . | P | . | . | . | . | . | . | . | . | . | . | I | . | . | . | . |
| 21-225_85H2_LC | . | S | M | . | . | . | . | . | . | . | . | . | . | . | . | . | . | . | . | . | . | . | . | . | . | . |
| 21-225_85H9_LC | . | . | . | . | . | . | . | . | . | . | . | . | . | . | . | . | . | . | . | . | T | . | . | . | . | . |
| 21-225_86E9_LC | . | . | . | . | . | . | C | . | . | . | P | . | . | . | . | . | . | . | . | . | . | . | . | . | . | . |
| 21-225_88A1_LC | N | . | . | . | . | . | . | . | . | . | . | . | . | . | . | . | A | . | . | . | . | . | . | . | . | . |
| 21-225_88B4_LC | . | . | . | . | . | . | . | . | . | . | . | . | . | . | . | . | . | . | . | . | . | . | . | . | . | . |
| 21-225_88C10_LC | . | . | . | . | . | . | . | . | . | . | . | . | . | . | . | . | . | . | . | . | . | . | . | . | . | . |
| 21-225_88G2_LC | . | . | . | . | . | . | . | . | . | C | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | V | M | T | Q | S | P | D | S | L | A | V | S | L | G | E | R | A | T | I | N | C | K | S | S |
| 21-225_90D9_LC | . | . | . | . | . | . | . | . | . | . | . | . | . | . | . | . | . | . | . | . | . | I | . | . | . | . |
| 21-225_91F1_LC | . | . | . | . | . | . | . | . | . | C | . | . | . | . | . | . | . | . | . | . | . | . | . | . | . | . |
| 21-225_91G8_LC | . | . | . | . | . | . | . | . | . | C | . | . | . | . | . | . | . | . | . | . | . | . | . | . | . | . |
| 21-225_92E6_LC | . | . | . | . | . | . | . | . | . | . | . | . | A | . | . | . | . | . | . | . | . | . | . | . | . | . |
| 21-225_92F12_LC | . | . | . | . | . | . | . | . | . | . | . | . | . | . | . | . | . | . | . | . | T | . | . | . | . | . |
| 21-225_94D2_LC | . | . | . | . | . | . | . | . | . | . | . | . | . | . | . | . | . | . | . | . | . | . | . | . | . | . |
| 21-225_94E11_LC | . | . | . | . | . | . | . | . | . | . | . | . | . | . | . | . | . | . | . | . | . | . | . | . | . | . |
| 21-225_94E12_LC | . | . | . | . | . | . | . | . | . | . | . | . | . | . | . | . | . | . | . | . | T | . | . | . | . | . |
| 21-225_95E12_LC | . | . | . | . | . | . | . | . | . | . | . | . | . | . | . | . | . | . | . | . | . | . | . | . | . | . |
| 21-225_95G4_LC | . | . | . | . | . | . | C | . | . | . | . | . | . | . | . | . | . | . | . | . | . | . | . | . | . | . |
| 21-225_95H4_LC | . | . | . | . | . | . | C | . | . | . | . | . | . | . | . | . | . | . | . | . | . | . | . | . | . | . |
| 21-225_96D10_LC | . | . | . | . | . | . | . | . | . | . | . | . | . | . | . | . | . | . | . | . | . | . | . | . | . | . |
| 21-225_96D5_LC | . | . | . | . | . | . | . | . | . | . | . | . | . | . | . | . | . | . | . | . | . | . | . | . | . | . |
| 21-225_96E2_LC | . | S | . | . | . | . | . | . | . | . | . | . | . | . | . | . | . | . | . | . | . | . | . | . | . | . |
| 21-225_96H5_LC | . | . | . | . | . | . | . | . | . | . | . | . | . | . | . | . | . | . | . | . | . | . | . | . | . | . |
| 21-225_97H4_LC | . | . | . | . | . | . | . | . | . | C | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | V | L | H | S | S | N | N | N | N | Y | L | A | W | Y | Q | Q | K | P | G | Q | P | P |
| 21-225_11E5_LC | . | . | . | . | . | . | Y | . | . | . | . | . | . | . | . | T | . | . | . | . | . | . | . | . | R | . |
| 21-225_12F12_LC | . | . | . | T | . | . | P | . | . | . | . | . | H | . | . | . | . | . | . | . | . | . | . | . | . | . |
| 21-225_147E4_LC | . | . | . | . | . | . | . | . | . | . | . | Y | . | . | . | . | . | . | . | L | . | . | R | . | . | . |
| 21-225_148F8_LC | . | . | . | . | A | . | . | . | . | . | . | Y | . | . | . | . | . | . | . | . | . | . | . | . | . | . |
| 21-225_149A2_LC | . | . | . | . | . | . | Y | . | P | . | . | . | . | . | . | . | . | . | . | . | . | . | . | . | . | . |
| 21-225_149E3_LC | . | . | . | T | . | . | . | N | . | . | . | H | . | . | F | . | . | . | . | . | . | . | . | . | . | . |
| 21-225_149H4_LC | . | . | . | . | . | . | S | . | . | . | D | . | . | . | . | . | . | . | . | . | . | . | . | R | . | . |
| 21-225_154A11_LC | . | . | . | . | . | . | . | . | . | . | . | Y | . | . | . | . | . | . | . | . | . | . | . | . | . | . |
| 21-225_158B12_LC | . | . | . | N | . | . | . | . | . | . | . | . | . | . | . | T | . | . | . | . | R | . | . | . | . | . |
| 21-225_162F6_LC | . | . | . | N | . | . | . | . | . | . | . | . | . | . | . | T | . | . | . | . | R | . | . | . | . | . |
| 21-225_164A4_LC | . | . | . | . | . | . | Y | . | . | . | . | K | . | . | . | T | . | . | . | . | . | . | . | . | . | . |
| 21-225_164A7_LC | . | . | . | . | . | . | . | . | . | . | . | . | . | . | . | . | . | . | . | . | . | . | . | . | . | H |
| 21-225_170F6_LC | . | . | . | . | . | . | . | . | . | . | . | Y | . | . | . | . | . | . | . | . | R | . | . | . | . | . |
| 21-225_174G5_LC | . | . | . | . | . | . | . | . | . | . | . | Y | . | . | . | T | . | . | . | . | . | S | . | . | . | . |
| 21-225_175D10_LC | . | . | . | . | . | . | Y | . | . | . | . | . | . | . | . | . | . | . | . | . | . | . | . | . | . | . |
| 21-225_177B12_LC | . | . | . | . | . | . | . | . | . | . | . | . | . | . | . | T | . | . | . | . | . | . | . | . | . | . |
| 21-225_181E5_LC | . | . | . | . | . | . | . | . | . | . | . | Y | . | . | . | T | . | . | . | . | . | . | . | . | . | . |
| 21-225_190D7_LC | . | . | . | . | . | . | S | . | . | . | . | Y | . | . | . | V | . | . | . | . | . | . | . | . | . | . |
| 21-225_191D8_LC | . | . | . | . | . | . | . | . | . | . | . | Y | . | . | . | . | . | . | . | . | . | . | . | H | . | . |
| 21-225_193G4_LC | . | . | . | . | . | . | Y | . | . | . | . | Y | . | . | . | T | . | . | . | . | . | . | . | . | . | . |
| 21-225_196G8_LC | . | . | . | . | . | . | . | . | . | . | . | . | . | . | . | . | . | . | . | . | . | . | . | . | . | . |
| 21-225_197C6_LC | . | . | . | . | . | . | . | . | . | . | . | Y | . | . | . | . | . | . | . | . | . | . | . | H | . | . |
| 21-225_197H4_LC | . | . | . | . | . | . | . | . | F | . | . | Y | . | . | . | . | . | . | . | . | . | A | . | H | . | . |
| 21-225_200G7_LC | . | . | . | . | . | . | . | . | . | . | . | . | . | . | . | . | . | . | . | . | . | . | . | . | . | . |
| 21-225_201F5_LC | . | . | . | . | . | . | Y | . | . | . | . | K | . | . | . | V | . | . | . | . | . | . | . | . | . | . |
| 21-225_213H12_LC | . | . | . | N | . | . | K | T | . | . | . | R | . | . | . | . | . | . | . | . | . | . | . | . | . | . |
| 21-225_213H7_LC | . | . | . | N | . | . | D | I | . | . | . | K | . | S | . | G | . | F | . | . | . | . | . | . | . | . |
| 21-225_222H3_LC | . | . | . | . | . | . | K | . | . | . | H | R | . | . | . | . | . | . | . | . | . | . | . | . | . | . |
| 21-225_223G10_LC | . | . | . | . | I | . | L | . | . | . | . | K | . | . | . | . | . | H | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | V | L | H | S | S | N | N | N | N | Y | L | A | W | Y | Q | Q | K | P | G | Q | P | P |
| 21-225_224D8_LC | . | . | . | . | . | . | Y | . | . | . | . | K | . | . | . | . | . | . | . | . | . | . | . | . | . | . |
| 21-225_224H5_LC | . | . | . | . | . | . | Y | . | . | . | . | F | . | . | . | T | . | . | . | . | . | . | . | . | . | . |
| 21-225_225F11_LC | . | . | . | . | . | . | Y | . | . | . | . | Y | . | . | . | . | . | . | . | . | R | . | . | . | . | . |
| 21-225_225F5_LC | . | . | . | N | . | . | Y | N | . | . | . | . | . | . | . | . | . | . | . | . | . | . | . | . | . | . |
| 21-225_226A12_LC | . | . | . | N | . | . | Y | . | . | . | . | . | . | . | . | . | . | . | . | . | T | . | . | . | . | . |
| 21-225_226D11_LC | . | . | . | N | . | . | . | . | . | . | S | . | . | . | . | V | . | . | . | . | . | . | . | . | . | . |
| 21-225_226F6_LC | . | . | . | . | I | . | Y | . | . | . | . | Y | . | . | . | . | . | . | . | . | . | . | . | . | . | . |
| 21-225_227C7_LC | . | . | . | I | . | . | S | D | . | . | . | . | . | . | . | . | . | . | . | . | . | . | . | . | . | . |
| 21-225_23A12_LC | . | . | . | . | . | . | Y | . | . | . | . | . | . | . | . | . | . | . | . | . | . | . | . | . | . | . |
| 21-225_23G12_LC | . | . | . | . | I | . | F | . | . | . | . | . | . | . | . | . | . | . | . | . | . | . | . | . | . | . |
| 21-225_24E1_LC | . | . | . | . | . | . | Y | T | . | . | . | . | . | . | . | T | . | . | . | . | . | . | . | . | R | . |
| 21-225_25A4_LC | . | . | . | . | . | . | Y | . | . | H | . | . | . | . | . | . | . | . | . | . | . | . | . | . | . | . |
| 21-225_25A4.001_LC | . | . | . | . | . | . | Y | . | . | H | . | . | . | . | . | . | . | . | . | . | . | . | . | . | . | . |
| 21-225_25A4.001.001_LC | . | . | . | . | . | . | Y | . | . | H | . | . | . | . | . | . | . | . | . | . | . | . | . | . | . | . |
| 21-225_25A4.001.002_LC | . | . | . | . | . | . | Y | . | . | H | . | . | . | . | . | . | . | . | . | . | . | . | . | . | . | . |
| 21-225_25A4.001.003_LC | . | . | . | . | . | . | Y | . | . | H | . | . | . | . | . | . | . | . | . | . | . | . | . | . | . | . |
| 21-225_25A4.001.004_LC | . | . | . | . | . | . | Y | . | . | H | . | . | . | . | . | . | . | . | . | . | . | . | . | . | . | . |
| 21-225_25A4.001.005_LC | . | . | . | . | . | . | Y | . | . | H | . | . | . | . | . | . | . | . | . | . | . | . | . | . | . | . |
| 21-225_25A4.001.006_LC | . | . | . | . | . | . | Y | . | . | H | . | . | . | . | . | . | . | . | . | . | . | . | . | . | . | . |
| 21-225_25A4.001.007_LC | . | . | . | . | . | . | Y | . | . | H | . | . | . | . | . | . | . | . | . | . | . | . | . | . | . | . |
| 21-225_25A4.001.008_LC | . | . | . | . | . | . | Y | . | . | H | . | . | . | . | . | . | . | . | . | . | . | . | . | . | . | . |
| 21-225_25A4.001.013_LC | . | . | . | . | . | . | Y | . | . | H | . | . | . | . | . | . | . | . | . | . | . | . | . | . | . | . |
| 21-225_25A4.001.019_LC | . | . | . | . | . | . | Y | . | . | H | . | . | . | . | . | . | . | . | . | . | . | . | . | . | . | . |
| 21-225_25A4.001.020_LC | . | . | . | . | . | . | Y | . | . | H | . | . | . | . | . | . | . | . | . | . | . | . | . | . | . | . |
| 21-225_25A4.001.021_LC | . | . | . | . | . | . | Y | . | . | H | . | . | . | . | . | . | . | . | . | . | . | . | . | . | . | . |
| 21-225_25A4.001.029_LC | . | . | . | . | . | . | Y | . | . | H | . | . | . | . | . | . | . | . | . | . | . | . | . | . | . | . |
| 21-225_26C4_LC | . | . | . | . | . | . | Y | R | . | . | . | Y | . | . | . | . | . | . | . | H | . | . | . | . | . | . |
| 21-225_29E6_LC | . | . | . | . | . | . | Y | . | . | H | . | . | Y | . | . | T | . | . | . | . | . | . | . | . | . | H |
| 21-225_29H8_LC | . | . | . | T | I | . | . | . | . | . | . | Y | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | V | L | H | S | S | N | N | N | N | Y | L | A | W | Y | Q | Q | K | P | G | Q | P | P |
| 21-225_43D8_LC | . | . | . | . | . | . | M | T | . | . | D | K | . | . | . | T | . | . | . | . | R | . | . | . | . | . |
| 21-225_4A2.001.023_LC | . | . | . | . | I | . | . | . | . | . | . | . | . | . | . | . | . | F | . | . | . | . | . | . | . | . |
| 21-225_4A2.001.028_LC | . | . | . | . | I | . | . | . | . | . | . | . | . | . | . | . | . | F | . | . | . | . | . | . | . | . |
| 21-225_4A2.001.029_LC | . | . | . | . | I | . | . | . | . | . | . | . | . | . | . | . | . | F | . | . | . | . | . | . | . | . |
| 21-225_4A2.001.031_LC | . | . | . | . | I | . | . | . | . | . | . | . | . | . | . | . | . | F | . | . | . | . | . | . | . | . |
| 21-225_56A1_LC | . | . | . | . | . | . | . | . | . | . | . | K | . | . | . | V | . | . | . | . | R | . | . | . | . | . |
| 21-225_57A11_LC | . | . | . | . | . | . | . | . | . | . | . | Y | . | . | . | . | . | . | . | . | R | . | . | . | . | . |
| 21-225_58B9_LC | . | . | . | . | I | . | Y | . | . | . | . | . | . | . | . | . | . | . | . | . | . | . | . | . | . | . |
| 21-225_59F10_LC | . | . | . | T | . | . | Y | R | . | . | . | Y | . | . | . | . | . | . | . | . | . | . | . | . | . | . |
| 21-225_61B5_LC | . | . | . | . | . | . | Y | . | . | . | . | . | . | S | . | T | . | . | . | L | . | . | . | . | . | . |
| 21-225_70E7_LC | . | . | . | . | . | . | Y | . | . | . | . | . | . | . | . | . | . | . | . | . | . | . | . | . | . | . |
| 21-225_74A6_LC | . | . | . | . | . | . | . | . | . | . | . | . | . | . | . | . | . | . | . | . | . | . | . | . | . | . |
| 21-225_74A7_LC | . | . | . | . | . | . | F | . | . | . | . | Y | . | . | . | . | . | . | . | . | R | . | . | . | . | . |
| 21-225_74B1_LC | . | . | . | . | . | . | . | R | . | . | . | Y | . | . | . | . | . | . | . | . | . | . | . | . | . | . |
| 21-225_74B10_LC | . | . | . | . | . | . | . | . | . | . | . | K | . | . | . | T | . | . | . | . | . | . | . | . | . | . |
| 21-225_74B5_LC | . | . | . | . | . | . | F | . | . | . | . | Y | . | . | . | . | . | . | . | . | R | . | . | . | . | . |
| 21-225_74B7_LC | . | . | . | T | . | . | . | . | . | . | . | Y | . | . | . | . | . | . | . | . | . | . | . | . | . | . |
| 21-225_74B8_LC | . | . | . | . | . | . | . | . | . | . | K | K | . | . | . | . | . | . | . | . | . | . | . | . | . | . |
| 21-225_74C1_LC | . | . | . | . | I | . | . | . | . | . | . | Y | . | . | . | . | . | . | . | . | . | . | . | H | . | H |
| 21-225_74F6_LC | . | . | . | N | I | . | S | . | . | . | . | K | . | . | . | T | . | . | . | . | . | . | . | . | . | . |
| 21-225_74F9_LC | . | . | . | . | . | . | Y | . | . | . | . | Y | . | . | . | . | . | . | . | . | . | . | . | . | . | . |
| 21-225_74G4_LC | . | . | . | . | . | . | Y | . | . | . | . | . | . | . | . | . | . | . | . | . | . | A | . | . | . | . |
| 21-225_74H2_LC | . | . | . | . | . | . | . | . | . | . | . | K | . | . | . | T | . | . | . | . | . | . | . | . | . | H |
| 21-225_75A10_LC | . | . | . | . | . | . | . | N | . | . | . | Y | . | . | . | T | . | . | . | . | . | . | . | . | . | . |
| 21-225_75A7_LC | . | . | . | T | . | . | . | . | . | . | . | Y | . | . | . | . | . | . | . | . | . | . | . | . | . | . |
| 21-225_75C4_LC | . | . | . | . | I | . | Y | . | . | . | . | . | . | . | . | . | . | F | . | . | . | . | . | . | . | . |
| 21-225_75F9_LC | . | . | . | . | . | . | . | N | . | . | . | . | . | . | . | . | . | . | . | . | . | . | . | . | . | . |
| 21-225_76E11_LC | . | . | . | . | . | . | . | . | . | . | . | Y | . | . | . | . | . | . | . | . | . | A | . | . | . | . |
| 21-225_76E8_LC | . | . | . | T | . | . | . | . | . | . | . | Y | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | V | L | H | S | S | N | N | N | N | Y | L | A | W | Y | Q | Q | K | P | G | Q | P | P |
| 21-225_76F3_LC | . | . | . | . | . | . | F | . | . | . | . | Y | . | . | . | . | . | . | . | . | R | . | . | . | . | H |
| 21-225_76F5_LC | . | . | . | . | . | . | . | . | . | . | S | Y | . | . | . | . | . | . | . | . | . | . | . | . | S | . |
| 21-225_76G2_LC | . | . | . | . | . | . | . | . | . | . | . | Y | . | . | . | . | . | . | . | . | R | . | . | . | . | . |
| 21-225_77C10_LC | . | . | . | . | . | . | Y | T | . | . | . | . | . | . | . | . | . | . | . | . | . | . | . | . | . | . |
| 21-225_77C7_LC | . | . | . | T | . | . | . | . | . | . | . | Y | . | . | . | . | . | . | . | . | . | . | . | . | . | . |
| 21-225_77E1_LC | . | . | . | T | . | . | . | . | . | . | . | Y | . | . | . | . | . | . | . | . | . | . | . | . | . | . |
| 21-225_78C1_LC | . | . | . | . | . | . | Y | T | . | . | . | . | . | . | . | . | . | . | . | . | . | . | . | . | . | . |
| 21-225_78E11_LC | . | . | . | . | . | . | Y | . | F | . | . | Y | . | . | . | . | . | . | . | . | . | . | . | . | . | . |
| 21-225_78H12_LC | . | . | . | T | . | . | . | . | F | . | . | Y | . | . | . | . | . | . | . | . | . | . | . | . | . | . |
| 21-225_79E9_LC | . | . | . | . | I | . | Y | . | . | . | . | . | . | . | . | . | . | F | . | . | . | . | . | . | . | . |
| 21-225_79F12_LC | . | . | . | . | I | . | Y | . | . | . | . | Y | . | . | . | . | . | . | . | . | . | . | . | . | . | . |
| 21-225_79G10_LC | . | . | . | . | . | . | F | . | . | . | . | Y | . | . | . | . | . | . | . | . | R | . | . | . | . | H |
| 21-225_79G6_LC | . | . | . | . | . | . | . | . | F | . | . | H | . | . | . | T | . | . | . | . | . | . | . | . | . | . |
| 21-225_80D3_LC | . | . | . | . | . | . | Y | T | . | . | . | . | . | . | . | . | . | . | . | . | . | . | . | . | . | . |
| 21-225_80E9_LC | . | . | . | . | . | . | Y | T | . | . | . | . | . | . | . | . | . | . | . | . | . | . | . | . | . | . |
| 21-225_80H3_LC | . | . | . | . | . | . | . | R | . | . | . | Y | . | . | . | . | . | . | . | . | . | . | . | . | . | . |
| 21-225_81E5_LC | . | . | . | . | . | . | F | . | . | . | . | Y | . | . | . | . | . | . | . | . | R | . | . | . | . | . |
| 21-225_81F9_LC | . | . | . | . | . | . | Y | T | . | . | . | . | . | . | . | . | . | . | . | . | . | . | . | . | . | . |
| 21-225_83F3_LC | . | . | . | . | . | . | Y | T | . | . | . | . | . | . | . | . | . | . | . | . | . | . | . | . | . | . |
| 21-225_83G3_LC | . | . | . | . | . | . | Y | T | . | . | . | . | . | . | . | . | . | . | . | . | . | . | . | . | . | . |
| 21-225_83G7_LC | . | . | . | T | . | . | . | . | . | . | . | Y | . | . | . | . | . | . | . | . | . | . | . | . | . | . |
| 21-225_84G7_LC | . | . | . | T | . | . | . | . | . | . | . | Y | . | . | . | . | . | . | . | . | . | . | . | . | . | . |
| 21-225_85H2_LC | . | . | . | . | . | . | . | . | . | . | K | H | . | . | . | . | . | . | . | . | . | . | . | . | . | . |
| 21-225_85H9_LC | . | . | . | . | . | . | . | R | . | . | . | Y | . | . | . | . | . | . | . | . | . | . | . | . | . | . |
| 21-225_86E9_LC | . | . | . | . | . | . | . | R | . | . | . | Y | . | . | . | . | . | . | . | . | . | . | . | . | . | . |
| 21-225_88A1_LC | . | . | . | . | . | . | . | . | . | . | . | Y | . | . | . | T | . | . | . | . | . | . | . | . | . | . |
| 21-225_88B4_LC | . | . | . | . | . | . | Y | I | . | . | . | . | . | . | . | . | . | . | . | . | . | . | . | . | . | . |
| 21-225_88C10_LC | . | . | . | . | . | . | . | . | . | . | . | W | . | . | . | . | . | H | . | . | . | . | . | . | . | . |
| 21-225_88G2_LC | . | . | . | . | . | . | Y | T | . | . | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | V | L | H | S | S | N | N | N | N | Y | L | A | W | Y | Q | Q | K | P | G | Q | P | P |
| 21-225_90D9_LC | . | . | . | T | . | . | . | . | . | . | . | Y | . | . | . | . | . | . | . | . | . | . | . | . | . | . |
| 21-225_91F1_LC | . | . | . | . | . | . | Y | T | . | . | . | . | . | . | . | . | . | . | . | . | . | . | . | . | . | . |
| 21-225_91G8_LC | . | . | . | . | . | . | Y | T | . | . | . | . | . | . | . | . | . | . | . | . | . | . | . | . | . | . |
| 21-225_92E6_LC | . | . | . | N | I | . | S | . | . | . | . | K | . | . | . | T | . | . | . | . | . | . | . | . | . | . |
| 21-225_92F12_LC | . | . | . | . | . | . | . | R | . | . | . | Y | . | . | . | . | . | . | . | . | . | . | . | . | . | . |
| 21-225_94D2_LC | . | . | . | . | . | . | . | . | . | . | . | K | . | . | . | T | . | . | . | . | . | . | . | . | . | H |
| 21-225_94E11_LC | . | . | . | . | . | . | F | . | . | . | . | Y | . | . | . | . | . | . | . | . | R | . | . | . | . | . |
| 21-225_94E12_LC | . | . | . | . | . | . | . | R | . | . | . | Y | . | . | . | . | . | . | . | . | . | . | . | . | . | . |
| 21-225_95E12_LC | . | . | . | . | . | . | Y | . | . | . | . | A | . | . | . | . | . | . | . | . | . | . | . | . | . | . |
| 21-225_95G4_LC | . | . | . | . | . | . | F | R | . | . | . | Y | . | . | . | . | . | . | . | . | R | . | . | . | . | H |
| 21-225_95H4_LC | . | . | . | . | . | . | F | . | . | . | . | Y | . | . | . | . | . | . | . | . | R | . | . | . | . | H |
| 21-225_96D10_LC | . | . | . | N | I | . | S | . | . | . | . | K | . | . | . | T | . | . | . | . | . | . | . | . | . | . |
| 21-225_96D5_LC | . | . | . | . | . | . | . | . | . | . | . | Y | . | . | . | . | . | . | . | . | . | . | . | . | . | . |
| 21-225_96E2_LC | . | . | . | . | . | . | . | . | . | . | K | K | . | . | . | . | . | . | . | . | . | . | . | . | . | . |
| 21-225_96H5_LC | . | . | . | . | . | . | Y | . | . | . | S | H | . | . | . | . | . | . | . | . | . | . | . | . | . | . |
| 21-225_97H4_LC | . | . | . | . | . | . | Y | T | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | Y | W | - | - | - | - | - | - | - | - | A | S | T | R | E | S | G | V | P | D | R | F |
| 21-225_11E5_LC | . | . | . | . | . | . | . | . | . | . | . | . | . | . | . | . | . | . | . | . | . | . | . | . | . | . |
| 21-225_12F12_LC | N | . | . | . | . | . | . | . | . | . | . | . | . | . | . | . | . | . | A | . | . | . | . | . | . | . |
| 21-225_147E4_LC | . | . | . | . | . | . | . | . | . | . | . | . | . | . | . | . | . | . | . | . | . | . | . | . | . | . |
| 21-225_148F8_LC | . | . | . | . | . | . | . | . | . | . | . | . | . | . | . | . | . | . | K | . | . | . | . | . | . | . |
| 21-225_149A2_LC | . | . | . | . | . | . | . | . | . | . | . | . | . | . | . | . | . | . | . | . | . | . | . | . | . | . |
| 21-225_149E3_LC | . | . | . | . | . | . | . | . | . | . | . | . | . | . | . | . | . | L | K | . | . | . | . | . | . | . |
| 21-225_149H4_LC | . | . | . | . | . | . | . | . | . | . | . | . | . | . | S | . | . | . | . | . | . | . | . | . | . | . |
| 21-225_154A11_LC | . | . | . | . | . | . | . | . | . | . | . | . | . | . | . | . | K | . | D | . | . | . | . | . | . | . |
| 21-225_158B12_LC | . | . | . | . | . | . | . | . | . | . | . | . | . | . | . | . | . | . | . | . | . | . | . | . | . | . |
| 21-225_162F6_LC | . | . | . | . | . | . | . | . | . | . | . | . | . | . | . | . | . | . | . | . | . | . | . | . | . | . |
| 21-225_164A4_LC | . | . | F | . | . | . | . | . | . | . | . | . | . | . | . | . | . | . | . | . | . | . | . | . | . | . |
| 21-225_164A7_LC | . | . | . | . | . | . | . | . | . | . | . | . | . | . | T | . | . | . | . | . | . | . | . | . | . | . |
| 21-225_170F6_LC | . | V | . | . | H | . | . | . | . | . | . | . | . | . | . | . | . | . | K | . | . | . | . | . | . | . |
| 21-225_174G5_LC | . | . | . | . | . | . | . | . | . | . | . | . | . | . | . | . | . | . | . | . | . | . | . | . | . | . |
| 21-225_175D10_LC | N | . | . | . | . | . | . | . | . | . | . | . | . | . | T | . | . | . | . | . | . | . | . | . | . | . |
| 21-225_177B12_LC | . | . | . | . | . | . | . | . | . | . | . | . | . | . | . | . | . | . | . | . | . | . | . | . | . | . |
| 21-225_181E5_LC | . | . | . | . | . | . | . | . | . | . | . | . | . | . | . | . | . | . | . | . | . | . | . | . | . | . |
| 21-225_190D7_LC | . | . | . | . | . | . | . | . | . | . | . | . | . | . | . | . | . | . | K | . | . | . | . | . | . | . |
| 21-225_191D8_LC | . | . | . | . | . | . | . | . | . | . | . | . | . | . | . | . | . | . | . | . | . | . | . | . | . | . |
| 21-225_193G4_LC | . | . | . | . | . | . | . | . | . | . | . | . | . | . | . | . | . | . | K | . | . | . | . | . | . | . |
| 21-225_196G8_LC | . | . | . | . | . | . | . | . | . | . | . | . | . | . | . | . | . | . | . | . | . | . | . | . | . | . |
| 21-225_197C6_LC | N | . | . | . | . | . | . | . | . | . | . | . | . | . | . | . | . | . | . | . | . | . | . | . | . | . |
| 21-225_197H4_LC | N | . | . | . | . | . | . | . | . | . | . | . | . | . | . | . | . | . | . | . | . | . | . | . | . | . |
| 21-225_200G7_LC | . | . | . | . | . | . | . | . | . | . | . | . | . | . | . | . | . | . | . | . | . | . | . | . | . | . |
| 21-225_201F5_LC | . | . | . | . | . | . | . | . | . | . | . | . | . | . | . | . | . | . | . | . | . | . | . | . | . | . |
| 21-225_213H12_LC | . | V | . | . | . | . | . | . | . | . | . | . | . | . | . | . | . | . | . | . | . | . | . | . | . | . |
| 21-225_213H7_LC | . | . | . | . | N | . | . | . | . | . | . | . | . | . | . | . | . | . | . | . | . | . | . | . | . | . |
| 21-225_222H3_LC | Q | . | . | . | . | . | . | . | . | . | . | . | . | . | . | . | . | . | . | T | . | . | . | . | . | . |
| 21-225_223G10_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | Y | W | - | - | - | - | - | - | - | - | A | S | T | R | E | S | G | V | P | D | R | F |
| 21-225_224D8_LC | . | . | . | . | . | . | . | . | . | . | . | . | . | . | S | . | . | . | K | . | . | . | . | . | . | . |
| 21-225_224H5_LC | . | . | . | . | . | . | . | . | . | . | . | . | . | . | . | . | . | . | . | . | . | . | . | . | . | . |
| 21-225_225F11_LC | . | . | . | . | . | . | . | . | . | . | . | . | . | . | . | . | . | . | . | . | . | . | . | . | . | . |
| 21-225_225F5_LC | N | . | . | . | . | . | . | . | . | . | . | . | . | . | . | . | . | . | . | . | . | . | . | . | . | . |
| 21-225_226A12_LC | . | . | . | F | . | . | . | . | . | . | . | . | . | . | . | . | . | . | K | . | . | . | . | . | . | . |
| 21-225_226D11_LC | . | . | . | . | . | . | . | . | . | . | . | . | . | . | . | . | . | . | . | . | . | . | . | . | . | . |
| 21-225_226F6_LC | . | . | . | . | . | . | . | . | . | . | . | . | . | . | . | . | . | . | . | . | . | . | . | . | . | . |
| 21-225_227C7_LC | N | . | . | . | . | . | . | . | . | . | . | . | . | . | . | . | . | . | . | . | . | . | . | . | . | . |
| 21-225_23A12_LC | . | . | . | . | . | . | . | . | . | . | . | . | . | . | T | . | . | . | . | . | . | . | . | . | . | . |
| 21-225_23G12_LC | . | . | . | . | . | . | . | . | . | . | . | . | . | . | . | . | . | . | . | . | . | . | . | . | . | . |
| 21-225_24E1_LC | N | . | . | . | . | . | . | . | . | . | . | . | . | . | . | . | . | . | . | . | . | . | . | . | . | . |
| 21-225_25A4_LC | . | . | . | L | . | . | . | . | . | . | . | . | . | . | . | . | . | . | . | . | . | . | . | . | . | . |
| 21-225_25A4.001_LC | . | . | . | L | . | . | . | . | . | . | . | . | . | . | . | . | . | . | . | . | . | . | . | . | . | . |
| 21-225_25A4.001.001_LC | . | . | . | L | . | . | . | . | . | . | . | . | . | . | . | . | . | . | . | . | . | . | . | . | . | . |
| 21-225_25A4.001.002_LC | . | . | . | L | . | . | . | . | . | . | . | . | . | . | . | . | . | . | . | . | . | . | . | . | . | . |
| 21-225_25A4.001.003_LC | . | . | . | L | . | . | . | . | . | . | . | . | . | . | . | . | . | . | . | . | . | . | . | . | . | . |
| 21-225_25A4.001.004_LC | . | . | . | L | . | . | . | . | . | . | . | . | . | . | . | . | . | . | . | . | . | . | . | . | . | . |
| 21-225_25A4.001.005_LC | . | . | . | L | . | . | . | . | . | . | . | . | . | . | . | . | . | . | . | . | . | . | . | . | . | . |
| 21-225_25A4.001.006_LC | . | . | . | L | . | . | . | . | . | . | . | . | . | . | . | . | . | . | . | . | . | . | . | . | . | . |
| 21-225_25A4.001.007_LC | . | . | . | L | . | . | . | . | . | . | . | . | . | . | . | . | . | . | . | . | . | . | . | . | . | . |
| 21-225_25A4.001.008_LC | . | . | . | L | . | . | . | . | . | . | . | . | . | . | . | . | . | . | . | . | . | . | . | . | . | . |
| 21-225_25A4.001.013_LC | . | . | . | L | . | . | . | . | . | . | . | . | . | . | . | . | . | . | . | . | . | . | . | . | . | . |
| 21-225_25A4.001.019_LC | . | . | . | L | . | . | . | . | . | . | . | . | . | . | . | . | . | . | . | . | . | . | . | . | . | . |
| 21-225_25A4.001.020_LC | . | . | . | L | . | . | . | . | . | . | . | . | . | . | . | . | . | . | . | . | . | . | . | . | . | . |
| 21-225_25A4.001.021_LC | . | . | . | L | . | . | . | . | . | . | . | . | . | . | . | . | . | . | . | . | . | . | . | . | . | . |
| 21-225_25A4.001.029_LC | . | . | . | L | . | . | . | . | . | . | . | . | . | . | . | . | . | . | . | . | . | . | . | . | . | . |
| 21-225_26C4_LC | . | . | . | . | . | . | . | . | . | . | . | . | . | . | . | . | . | . | . | . | . | . | . | . | . | . |
| 21-225_29E6_LC | . | . | . | L | . | . | . | . | . | . | . | . | . | . | . | . | S | . | . | . | . | . | . | . | . | . |
| 21-225_29H8_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | Y | W | - | - | - | - | - | - | - | - | A | S | T | R | E | S | G | V | P | D | R | F |
| 21-225_43D8_LC | . | . | . | . | . | . | . | . | . | . | . | . | . | . | . | . | . | . | . | . | . | . | . | . | . | . |
| 21-225_4A2.001.023_LC | . | . | . | L | . | . | . | . | . | . | . | . | . | . | . | . | . | . | . | . | . | . | . | . | . | . |
| 21-225_4A2.001.028_LC | . | . | . | L | . | . | . | . | . | . | . | . | . | . | . | . | . | . | . | . | . | . | . | . | . | . |
| 21-225_4A2.001.029_LC | . | . | . | L | . | . | . | . | . | . | . | . | . | . | . | . | . | . | . | . | . | . | . | . | . | . |
| 21-225_4A2.001.031_LC | . | . | . | L | . | . | . | . | . | . | . | . | . | . | . | . | . | . | . | . | . | . | . | . | . | . |
| 21-225_56A1_LC | N | . | . | . | . | . | . | . | . | . | . | . | . | . | . | . | . | . | . | . | . | . | . | . | . | . |
| 21-225_57A11_LC | . | . | V | . | . | . | . | . | . | . | . | . | . | . | . | . | . | . | A | . | . | . | . | . | . | . |
| 21-225_58B9_LC | . | K | . | . | . | . | . | . | . | . | . | . | . | . | . | . | . | . | D | . | . | . | . | A | . | . |
| 21-225_59F10_LC | . | . | . | . | . | . | . | . | . | . | . | . | . | . | . | . | . | . | . | . | . | . | . | . | . | . |
| 21-225_61B5_LC | . | K | . | . | . | . | . | . | . | . | . | . | . | . | . | . | . | . | . | . | . | . | . | . | . | . |
| 21-225_70E7_LC | . | . | . | . | . | . | . | . | . | . | . | . | . | . | . | . | . | . | . | . | . | . | . | . | . | . |
| 21-225_74A6_LC | D | . | . | . | . | . | . | . | . | . | . | . | . | . | . | . | . | . | . | . | . | . | . | . | . | . |
| 21-225_74A7_LC | N | . | . | . | . | . | . | . | . | . | . | . | . | . | . | . | . | . | . | . | . | . | . | . | . | . |
| 21-225_74B1_LC | . | . | . | . | . | . | . | . | . | . | . | . | . | . | . | . | . | . | . | . | . | . | . | . | . | . |
| 21-225_74B10_LC | . | . | . | . | . | . | . | . | . | . | . | . | . | . | . | . | . | . | . | . | . | . | . | . | . | . |
| 21-225_74B5_LC | N | . | . | . | . | . | . | . | . | . | . | . | . | . | . | . | . | . | . | . | . | . | . | . | . | . |
| 21-225_74B7_LC | . | V | . | . | . | . | . | . | . | . | . | . | . | . | T | . | . | . | . | . | . | . | . | . | . | . |
| 21-225_74B8_LC | . | . | . | . | . | . | . | . | . | . | . | . | . | . | . | . | . | . | . | . | . | . | . | . | . | . |
| 21-225_74C1_LC | . | . | . | . | . | . | . | . | . | . | . | . | . | . | . | . | . | . | . | . | . | . | . | . | . | . |
| 21-225_74F6_LC | . | I | . | . | . | . | . | . | . | . | . | . | . | . | T | . | . | . | . | . | . | . | . | . | . | . |
| 21-225_74F9_LC | . | K | . | . | . | . | . | . | . | . | . | . | . | . | . | . | . | . | . | . | . | . | . | . | . | . |
| 21-225_74G4_LC | . | . | . | . | . | . | . | . | . | . | . | . | . | . | . | . | . | . | . | . | . | . | . | . | . | . |
| 21-225_74H2_LC | . | . | . | . | . | . | . | . | . | . | . | . | . | . | . | . | . | . | . | . | . | . | . | . | . | . |
| 21-225_75A10_LC | . | . | . | L | . | . | . | . | . | . | . | . | . | . | . | . | . | . | K | . | . | . | . | . | . | . |
| 21-225_75A7_LC | . | V | . | . | . | . | . | . | . | . | . | . | . | . | T | . | . | . | . | . | . | . | . | . | . | . |
| 21-225_75C4_LC | . | . | . | . | . | . | . | . | . | . | . | . | . | . | . | . | . | . | . | . | . | . | . | . | . | . |
| 21-225_75F9_LC | . | . | . | . | . | . | . | . | . | . | . | . | . | . | . | . | . | . | . | . | . | . | . | . | . | . |
| 21-225_76E11_LC | . | . | . | . | . | . | . | . | . | . | . | . | . | . | . | . | . | . | . | . | . | . | . | . | . | . |
| 21-225_76E8_LC | . | V | . | . | . | . | . | . | . | . | . | . | . | . | T | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | Y | W | - | - | - | - | - | - | - | - | A | S | T | R | E | S | G | V | P | D | R | F |
| 21-225_76F3_LC | D | . | . | . | . | . | . | . | . | . | . | . | . | . | . | . | . | . | . | . | . | . | . | . | . | . |
| 21-225_76F5_LC | . | V | . | . | . | . | . | . | . | . | . | . | . | . | T | . | . | . | . | . | . | . | . | . | . | . |
| 21-225_76G2_LC | R | . | . | . | . | . | . | . | . | . | . | . | . | . | . | . | . | . | . | . | . | . | . | . | . | . |
| 21-225_77C10_LC | . | . | . | . | . | . | . | . | . | . | . | . | . | . | . | . | . | . | . | . | . | . | . | . | . | . |
| 21-225_77C7_LC | . | V | . | L | . | . | . | . | . | . | . | . | . | . | T | . | . | . | . | . | . | . | . | . | . | . |
| 21-225_77E1_LC | . | V | . | . | . | . | . | . | . | . | . | . | . | . | T | . | . | . | . | . | . | . | N | . | . | . |
| 21-225_78C1_LC | . | . | . | . | . | . | . | . | . | . | . | . | . | . | . | . | . | . | . | . | . | . | . | . | . | . |
| 21-225_78E11_LC | . | K | . | . | . | . | . | . | . | . | . | . | . | . | . | . | . | . | . | . | . | . | . | . | . | . |
| 21-225_78H12_LC | . | V | . | . | . | . | . | . | . | . | . | . | . | . | T | . | . | . | . | . | . | . | . | . | . | . |
| 21-225_79E9_LC | . | . | . | . | . | . | . | . | . | . | . | . | . | . | . | . | . | . | . | . | . | . | . | . | . | . |
| 21-225_79F12_LC | . | . | . | . | . | . | . | . | . | . | . | . | . | . | . | . | . | . | . | . | . | . | . | . | . | . |
| 21-225_79G10_LC | N | . | . | . | . | . | . | . | . | . | . | . | . | . | . | . | . | . | . | . | . | . | . | . | . | . |
| 21-225_79G6_LC | . | . | . | . | . | . | . | . | . | . | . | . | . | . | . | . | . | W | . | . | . | . | . | . | . | . |
| 21-225_80D3_LC | . | . | . | . | . | . | . | . | . | . | . | . | . | . | . | . | . | . | . | . | . | . | . | . | . | . |
| 21-225_80E9_LC | . | . | . | . | . | . | . | . | . | . | . | . | . | . | . | . | . | . | . | . | . | . | . | . | . | . |
| 21-225_80H3_LC | . | . | . | . | . | . | . | . | . | . | . | . | . | . | . | . | . | . | . | . | . | . | . | . | . | . |
| 21-225_81E5_LC | N | . | . | . | . | . | . | . | . | . | . | . | . | . | . | . | . | . | . | . | . | . | . | . | . | . |
| 21-225_81F9_LC | . | . | . | . | . | . | . | . | . | . | . | . | . | . | . | . | . | . | . | . | . | . | . | . | . | . |
| 21-225_83F3_LC | . | . | . | . | . | . | . | . | . | . | . | . | . | . | . | . | . | . | . | . | . | . | . | . | . | . |
| 21-225_83G3_LC | . | . | . | . | . | . | . | . | . | . | . | . | . | . | . | . | . | . | . | . | . | . | . | . | . | . |
| 21-225_83G7_LC | . | V | . | . | . | . | . | . | . | . | . | . | . | . | T | . | . | . | . | . | . | . | . | . | . | . |
| 21-225_84G7_LC | . | V | . | . | . | . | . | . | . | . | . | . | . | . | T | . | . | . | . | . | . | . | . | . | . | . |
| 21-225_85H2_LC | . | . | . | . | . | . | . | . | . | . | . | . | . | . | . | . | . | . | . | . | . | . | . | . | . | . |
| 21-225_85H9_LC | . | . | . | . | . | . | . | . | . | . | . | . | . | . | . | . | . | . | . | . | . | . | . | . | . | . |
| 21-225_86E9_LC | N | . | . | . | . | . | . | . | . | . | . | . | . | . | . | . | . | . | . | . | . | . | . | . | . | . |
| 21-225_88A1_LC | . | . | . | L | . | . | . | . | . | . | . | . | . | . | . | . | . | . | K | . | . | . | . | . | . | . |
| 21-225_88B4_LC | . | . | . | . | . | . | . | . | . | . | . | . | . | . | . | . | . | . | . | . | . | . | . | A | . | . |
| 21-225_88C10_LC | . | . | . | . | H | . | . | . | . | . | . | . | . | . | . | F | . | . | K | . | . | . | . | . | . | . |
| 21-225_88G2_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | Y | W | - | - | - | - | - | - | - | - | A | S | T | R | E | S | G | V | P | D | R | F |
| 21-225_90D9_LC | . | V | . | . | . | . | . | . | . | . | . | . | . | . | T | . | . | . | . | . | . | . | . | . | . | . |
| 21-225_91F1_LC | . | . | . | . | . | . | . | . | . | . | . | . | . | . | . | . | . | . | . | . | . | . | . | . | . | . |
| 21-225_91G8_LC | . | . | . | . | . | . | . | . | . | . | . | . | . | . | . | . | . | . | . | . | . | . | . | . | . | . |
| 21-225_92E6_LC | . | I | . | . | . | . | . | . | . | . | . | . | . | . | T | . | . | . | . | . | . | . | . | . | . | . |
| 21-225_92F12_LC | . | . | . | . | . | . | . | . | . | . | . | . | . | . | . | . | . | . | . | . | . | . | . | . | . | . |
| 21-225_94D2_LC | . | . | . | . | . | . | . | . | . | . | . | . | . | . | . | . | . | . | . | . | . | . | . | . | . | . |
| 21-225_94E11_LC | N | . | . | . | . | . | . | . | . | . | . | . | . | . | . | . | . | . | . | . | . | . | . | . | . | . |
| 21-225_94E12_LC | . | . | . | . | . | . | . | . | . | . | . | . | . | . | . | . | . | . | . | . | . | . | . | . | . | . |
| 21-225_95E12_LC | N | . | F | . | . | . | . | . | . | . | . | . | . | . | . | . | . | . | . | . | . | . | . | . | . | . |
| 21-225_95G4_LC | N | . | . | . | . | . | . | . | . | . | . | . | . | . | . | . | . | . | . | . | . | . | . | . | . | . |
| 21-225_95H4_LC | N | . | . | . | . | . | . | . | . | . | . | . | . | . | . | . | . | . | . | . | . | . | . | . | . | . |
| 21-225_96D10_LC | . | I | . | . | . | . | . | . | . | . | . | . | . | . | T | . | . | . | . | . | . | . | . | . | . | . |
| 21-225_96D5_LC | N | . | . | . | . | . | . | . | . | . | . | . | . | . | . | . | . | . | . | . | . | . | . | . | . | . |
| 21-225_96E2_LC | . | . | . | . | . | . | . | . | . | . | . | . | . | . | . | . | . | W | . | . | . | . | . | . | . | . |
| 21-225_96H5_LC | . | . | . | . | . | . | . | . | . | . | . | . | . | . | . | . | I | . | . | . | . | . | . | . | . | . |
| 21-225_97H4_LC | . | . | . | . | . | . | . | . | . | . | . | . | . | . | . | . | . | . | . | . | . | . | . | . | . | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | A | E | D | V | A | V | Y |
| 21-225_11E5_LC | . | . | . | . | . | . | . | . | . | N | . | . | . | . | . | . | . | . | . | . | . | . | . | . | . | . |
| 21-225_12F12_LC | . | . | . | . | . | . | . | . | . | . | . | . | . | . | . | . | . | . | . | . | . | . | . | . | . | . |
| 21-225_147E4_LC | . | . | [illegible] | . | . | . | . | . | . | . | . | . | . | . | . | . | . | . | . | . | . | . | . | . | I | . |
| 21-225_148F8_LC | . | . | . | . | . | . | . | . | . | . | . | . | . | . | . | . | . | . | . | . | . | . | . | . | . | . |
| 21-225_149A2_LC | . | . | . | . | . | . | . | . | . | . | . | . | . | . | . | . | . | . | . | . | . | . | . | . | . | . |
| 21-225_149E3_LC | . | . | . | . | . | . | . | . | . | . | . | . | . | . | . | . | . | . | . | . | . | . | . | . | . | . |
| 21-225_149H4_LC | . | . | . | . | . | . | . | . | . | . | . | . | . | . | . | . | . | . | . | . | . | . | . | . | . | . |
| 21-225_154A11_LC | . | . | . | . | . | . | . | . | . | . | . | . | . | . | . | . | . | . | . | . | . | . | . | . | . | . |
| 21-225_158B12_LC | . | . | . | . | . | . | . | . | . | E | . | . | . | . | . | . | . | . | . | . | . | . | . | . | . | . |
| 21-225_162F6_LC | . | . | . | . | . | . | . | . | . | E | . | . | . | . | . | . | . | . | . | . | . | . | . | . | . | . |
| 21-225_164A4_LC | . | . | . | . | C | . | . | . | . | . | . | . | . | . | . | . | . | V | . | . | . | . | . | . | . | . |
| 21-225_164A7_LC | . | . | . | . | Y | . | . | . | . | . | . | S | . | . | . | A | . | . | . | . | . | . | . | . | . | . |
| 21-225_170F6_LC | . | . | . | V | . | . | . | . | . | . | . | . | . | . | . | N | . | . | . | . | . | . | . | . | . | . |
| 21-225_174G5_LC | . | . | . | . | . | . | . | . | . | . | . | . | . | . | . | . | . | . | . | . | . | . | . | . | . | . |
| 21-225_175D10_LC | . | . | . | . | . | . | . | . | . | N | . | . | . | . | . | . | . | . | . | . | . | . | . | . | . | . |
| 21-225_177B12_LC | . | . | . | . | . | . | . | . | . | . | . | . | . | . | . | . | . | . | . | . | . | . | . | . | . | . |
| 21-225_181E5_LC | . | . | . | . | . | . | . | . | . | . | . | . | . | . | . | . | . | . | . | . | . | . | . | . | . | . |
| 21-225_190D7_LC | . | . | . | . | . | . | . | . | . | . | . | . | . | . | . | . | . | . | . | . | D | . | . | . | . | . |
| 21-225_191D8_LC | . | . | . | . | . | . | . | . | . | . | . | . | . | . | . | . | . | . | . | . | . | . | . | . | . | . |
| 21-225_193G4_LC | . | . | . | . | . | . | . | . | . | . | . | . | . | . | . | . | . | . | . | . | D | . | . | . | . | . |
| 21-225_196G8_LC | . | . | . | . | . | . | . | . | . | . | . | . | . | A | . | . | . | . | . | . | . | . | . | . | . | . |
| 21-225_197C6_LC | . | . | . | . | . | . | . | . | . | . | . | . | . | . | . | . | . | . | . | . | . | . | . | . | . | . |
| 21-225_197H4_LC | . | . | . | . | . | . | . | . | . | . | . | . | . | P | . | . | . | . | . | . | . | . | . | . | . | . |
| 21-225_200G7_LC | . | . | . | . | . | . | . | . | . | . | . | . | . | A | . | . | . | . | . | . | . | . | . | . | . | . |
| 21-225_201F5_LC | . | . | . | . | . | . | . | . | . | . | . | . | . | . | . | . | . | . | . | . | . | . | . | . | . | . |
| 21-225_213H12_LC | . | . | . | . | . | . | . | . | . | . | . | . | . | I | . | . | . | . | . | . | . | . | . | . | . | . |
| 21-225_213H7_LC | . | . | . | . | . | . | . | . | . | . | . | . | . | . | . | . | . | . | . | T | . | . | . | . | . | . |
| 21-225_222H3_LC | . | . | . | . | . | . | . | . | . | . | . | . | . | . | . | . | . | . | . | . | . | . | . | S | . | . |
| 21-225_223G10_LC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | A | E | D | V | A | V | Y |
| 21-225_224D8_LC | . | . | . | . | . | . | . | . | . | . | . | . | . | . | . | . | . | . | . | . | . | . | . | . | . | . |
| 21-225_224H5_LC | . | . | . | . | . | . | . | . | . | . | . | . | . | . | . | N | . | . | . | . | . | . | . | . | . | . |
| 21-225_225F11_LC | . | . | . | . | . | . | . | . | P | . | . | . | . | . | . | . | . | . | . | . | . | . | . | . | . | . |
| 21-225_225F5_LC | . | . | . | . | . | . | . | . | . | . | . | . | . | . | . | . | . | . | . | . | . | . | . | . | . | . |
| 21-225_226A12_LC | . | . | . | . | . | . | . | . | . | . | . | . | . | . | . | . | . | . | . | . | . | . | . | . | . | . |
| 21-225_226D11_LC | R | . | . | . | . | . | . | . | . | . | . | . | . | . | . | . | . | . | . | . | . | . | . | . | . | . |
| 21-225_226F6_LC | . | . | . | . | . | . | . | . | P | . | . | . | . | . | . | . | . | . | . | . | . | . | . | . | . | . |
| 21-225_227C7_LC | . | . | . | . | . | . | . | . | . | . | . | . | . | . | . | . | . | . | . | . | . | . | . | . | . | . |
| 21-225_23A12_LC | . | . | . | . | . | . | . | . | . | . | . | . | . | . | . | . | . | . | . | . | . | . | . | . | . | . |
| 21-225_23G12_LC | . | . | . | . | . | . | . | . | . | . | . | . | . | . | . | . | . | . | . | . | . | . | . | . | . | . |
| 21-225_24E1_LC | . | . | . | . | . | . | . | . | . | . | . | . | . | . | . | . | . | . | . | . | . | . | . | . | . | . |
| 21-225_25A4_LC | . | . | . | . | . | . | . | . | . | E | . | . | . | . | . | . | . | . | E | . | . | . | . | . | . | . |
| 21-225_25A4.001_LC | . | . | . | . | . | . | . | . | . | E | . | . | . | . | . | . | . | . | E | . | . | . | . | . | . | . |
| 21-225_25A4.001.001_LC | . | . | . | . | . | . | . | . | . | E | . | . | . | . | . | . | . | . | E | . | . | . | . | . | . | . |
| 21-225_25A4.001.002_LC | . | . | . | . | . | . | . | . | . | E | . | . | . | . | . | . | . | . | E | . | . | . | . | . | . | . |
| 21-225_25A4.001.003_LC | . | . | . | . | . | . | . | . | . | E | . | . | . | . | . | . | . | . | E | . | . | . | . | . | . | . |
| 21-225_25A4.001.004_LC | . | . | . | . | . | . | . | . | . | E | . | . | . | . | . | . | . | . | E | . | . | . | . | . | . | . |
| 21-225_25A4.001.005_LC | . | . | . | . | . | . | . | . | . | E | . | . | . | . | . | . | . | . | . | . | . | . | . | . | . | . |
| 21-225_25A4.001.006_LC | . | . | . | . | . | . | . | . | . | E | . | . | . | . | . | . | . | . | . | . | . | . | . | . | . | . |
| 21-225_25A4.001.007_LC | . | . | . | . | . | . | . | . | . | E | . | . | . | . | . | . | . | . | . | . | . | . | . | . | . | . |
| 21-225_25A4.001.008_LC | . | . | . | . | . | . | . | . | . | E | . | . | . | . | . | . | . | . | . | . | . | . | . | . | . | . |
| 21-225_25A4.001.013_LC | . | . | . | . | . | . | . | . | . | E | . | . | . | . | . | . | . | . | . | . | . | . | . | . | . | . |
| 21-225_25A4.001.019_LC | . | . | . | . | . | . | . | . | . | E | . | . | . | . | . | . | . | . | E | . | . | . | . | . | . | . |
| 21-225_25A4.001.020_LC | . | . | . | . | . | . | . | . | . | E | . | . | . | . | . | . | . | . | . | . | . | . | . | . | . | . |
| 21-225_25A4.001.021_LC | . | . | . | . | . | . | . | . | . | E | . | . | . | . | . | . | . | . | . | . | . | . | . | . | . | . |
| 21-225_25A4.001.029_LC | . | . | . | . | . | . | . | . | . | E | . | . | . | . | . | . | . | . | . | . | . | . | . | . | . | . |
| 21-225_26C4_LC | . | . | . | . | . | . | . | . | . | . | . | . | . | . | . | . | . | . | . | . | . | . | . | . | . | . |
| 21-225_29E6_LC | . | . | . | . | . | . | . | . | . | E | . | . | . | . | . | . | . | . | . | . | . | . | . | . | . | . |
| 21-225_29H8_LC | . | . | . | . | . | . | . | . | . | . | . | . | . | . | . | . | . | . | . | . | . | . | . | . | . | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | A | E | D | V | A | V | Y |
| 21-225_43D8_LC | . | . | G | . | . | . | . | . | . | . | . | . | . | . | . | . | G | . | . | . | . | . | . | . | . | . |
| 21-225_4A2.001.023_LC | . | . | . | . | . | . | . | . | . | . | . | . | . | . | . | . | . | . | . | P | . | . | . | . | . | . |
| 21-225_4A2.001.028_LC | . | . | . | . | . | . | . | . | . | . | . | . | . | . | . | . | . | . | . | P | . | . | . | . | . | . |
| 21-225_4A2.001.029_LC | . | . | . | . | . | . | . | . | . | . | . | . | . | . | . | . | . | . | . | P | . | . | . | . | . | . |
| 21-225_4A2.001.031_LC | . | . | . | . | . | . | . | . | . | . | . | . | . | . | . | . | . | . | . | P | . | . | . | . | . | . |
| 21-225_56A1_LC | . | . | . | . | . | . | . | . | . | . | . | . | . | . | . | . | . | . | . | . | . | . | . | . | . | . |
| 21-225_57A11_LC | . | . | . | . | . | . | . | . | . | . | . | . | . | . | . | . | . | . | . | . | . | . | M | . | . | . |
| 21-225_58B9_LC | . | . | . | . | . | . | . | . | . | . | . | . | . | . | . | . | . | . | . | . | . | . | . | . | . | . |
| 21-225_59F10_LC | . | . | . | . | . | . | . | . | . | . | . | . | . | . | . | . | . | . | . | . | . | . | . | . | . | . |
| 21-225_61B5_LC | . | . | . | . | . | . | . | . | . | . | . | . | . | . | . | . | . | . | . | . | . | . | . | . | . | . |
| 21-225_70E7_LC | . | . | . | . | . | . | . | . | . | . | . | . | . | . | . | . | . | . | . | . | . | . | . | . | . | . |
| 21-225_74A6_LC | . | . | . | . | . | . | . | . | . | . | . | . | . | . | . | . | . | . | . | . | . | . | . | . | . | . |
| 21-225_74A7_LC | . | . | . | . | . | . | . | . | . | . | . | . | . | . | . | . | . | . | . | . | A | . | . | . | . | . |
| 21-225_74B1_LC | . | . | . | . | . | . | . | . | . | . | . | . | . | . | . | . | . | . | . | . | . | . | . | . | . | . |
| 21-225_74B10_LC | . | . | . | . | . | . | . | . | . | . | . | S | . | . | . | G | . | . | . | . | . | . | . | . | . | . |
| 21-225_74B5_LC | . | . | . | . | . | . | . | . | . | . | . | . | . | . | . | . | . | . | . | . | A | . | . | . | . | . |
| 21-225_74B7_LC | . | . | . | . | . | . | . | . | . | . | . | . | . | . | . | . | . | . | . | . | . | . | . | . | . | . |
| 21-225_74B8_LC | . | . | . | . | . | . | . | . | . | . | . | . | . | . | . | . | . | . | . | . | . | . | . | . | . | . |
| 21-225_74C1_LC | . | . | . | . | Y | . | . | . | . | . | . | . | . | . | . | . | . | . | . | . | . | . | . | . | . | . |
| 21-225_74F6_LC | . | . | . | . | F | . | . | . | . | . | . | . | . | . | . | . | . | . | . | . | . | . | . | . | . | . |
| 21-225_74F9_LC | . | . | . | . | . | . | . | . | . | . | . | . | . | . | . | . | . | . | . | . | . | . | . | . | . | . |
| 21-225_74G4_LC | . | . | . | . | . | . | . | . | . | . | . | . | . | . | . | . | . | . | . | . | . | . | . | . | . | . |
| 21-225_74H2_LC | . | . | . | . | . | . | . | . | . | . | . | S | . | . | . | G | . | . | . | . | . | . | . | . | . | . |
| 21-225_75A10_LC | . | . | . | . | . | . | . | . | . | . | . | . | . | . | . | . | . | . | . | . | . | . | . | . | . | . |
| 21-225_75A7_LC | . | . | . | . | . | . | . | . | . | . | . | . | . | . | . | . | . | . | . | . | . | . | . | . | . | . |
| 21-225_75C4_LC | . | . | . | . | . | . | . | . | . | . | . | . | . | . | . | . | . | . | . | . | . | . | . | . | . | . |
| 21-225_75F9_LC | . | . | . | . | . | . | . | . | . | . | . | . | . | . | . | . | . | . | . | . | . | . | . | . | . | . |
| 21-225_76E11_LC | . | . | . | . | . | . | . | . | . | . | . | . | . | . | . | . | . | . | . | . | . | . | . | . | . | . |
| 21-225_76E8_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | A | E | D | V | A | V | Y |
| 21-225_76F3_LC | . | . | . | . | . | . | . | . | . | . | . | . | . | . | . | . | . | . | . | . | A | . | . | . | . | . |
| 21-225_76F5_LC | . | . | . | . | . | . | . | . | . | . | . | . | . | . | . | . | . | . | . | . | . | . | . | . | . | . |
| 21-225_76G2_LC | . | . | . | . | . | . | . | . | . | . | . | . | . | . | . | . | . | . | . | V | . | . | . | . | . | . |
| 21-225_77C10_LC | . | . | . | . | . | . | . | . | . | . | . | . | . | . | . | . | . | . | . | . | . | . | . | . | . | . |
| 21-225_77C7_LC | . | . | . | . | . | . | . | . | . | . | . | . | . | . | . | . | . | . | . | . | . | . | . | . | . | . |
| 21-225_77E1_LC | . | . | . | . | . | . | . | . | . | . | . | . | . | . | . | . | . | . | . | . | . | . | . | . | . | . |
| 21-225_78C1_LC | . | . | . | . | . | . | . | . | . | . | . | . | . | . | . | . | . | . | . | . | . | . | . | . | . | . |
| 21-225_78E11_LC | . | . | . | . | . | . | . | . | . | . | . | . | . | . | . | . | . | . | . | . | . | . | . | . | . | . |
| 21-225_78H12_LC | . | . | . | . | . | . | . | . | . | . | . | . | . | . | . | . | . | . | . | . | . | . | . | . | . | . |
| 21-225_79E9_LC | . | . | . | . | C | . | . | . | . | . | . | . | . | . | . | . | . | . | . | . | . | . | . | . | . | . |
| 21-225_79F12_LC | . | . | . | . | . | . | . | . | . | . | . | . | . | . | . | . | . | . | . | . | . | . | . | . | . | . |
| 21-225_79G10_LC | . | . | . | . | . | . | . | . | . | . | . | . | . | . | . | . | . | . | . | . | A | . | . | . | . | . |
| 21-225_79G6_LC | . | . | . | . | . | . | . | . | . | . | . | S | . | P | . | G | . | . | . | . | . | . | . | . | . | . |
| 21-225_80D3_LC | . | . | . | . | . | . | . | . | . | . | . | . | . | . | . | . | . | . | . | . | . | . | . | . | . | . |
| 21-225_80E9_LC | . | . | . | . | . | . | . | . | . | . | . | . | . | . | . | . | . | . | . | . | . | . | . | . | . | . |
| 21-225_80H3_LC | . | . | . | . | . | . | . | . | . | . | . | . | . | . | . | . | . | . | . | . | . | . | . | . | . | . |
| 21-225_81E5_LC | . | . | . | . | . | . | . | . | . | . | . | . | . | . | . | . | . | . | . | . | A | . | . | . | . | . |
| 21-225_81F9_LC | . | . | . | . | . | . | . | . | . | . | . | . | . | . | . | . | . | . | . | . | . | . | . | . | . | . |
| 21-225_83F3_LC | . | . | . | . | . | . | . | . | . | . | . | . | . | . | . | . | . | . | . | . | . | . | . | . | . | . |
| 21-225_83G3_LC | . | . | . | . | . | . | . | . | . | . | . | . | . | . | . | . | . | . | . | . | . | . | . | . | . | . |
| 21-225_83G7_LC | . | . | . | . | . | . | . | . | . | . | . | . | . | . | . | . | . | . | . | . | . | . | . | . | . | . |
| 21-225_84G7_LC | . | . | . | . | . | . | . | . | . | . | . | . | . | . | . | . | . | . | . | . | . | . | . | . | . | . |
| 21-225_85H2_LC | . | . | . | . | . | . | . | . | . | . | . | . | . | . | . | . | . | . | . | . | . | . | . | . | . | . |
| 21-225_85H9_LC | . | . | . | . | . | . | . | . | . | . | . | . | . | . | . | . | . | . | . | . | . | . | . | . | . | . |
| 21-225_86E9_LC | . | . | . | . | . | . | . | . | . | . | . | . | . | . | . | . | . | . | . | . | A | . | . | . | . | . |
| 21-225_88A1_LC | . | . | . | . | . | . | . | . | . | . | . | . | . | . | . | . | . | . | . | . | . | . | . | . | . | . |
| 21-225_88B4_LC | . | . | . | . | . | . | . | . | . | . | . | . | . | . | . | . | . | . | . | . | . | . | . | . | . | . |
| 21-225_88C10_LC | . | . | G | . | . | . | . | . | . | N | . | . | . | . | . | . | . | . | . | . | . | . | . | . | . | . |
| 21-225_88G2_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | A | E | D | V | A | V | Y |
| 21-225_90D9_LC | . | . | . | . | . | . | . | . | . | . | . | . | . | . | . | . | . | . | . | . | . | . | . | . | . | . |
| 21-225_91F1_LC | . | . | . | . | . | . | . | . | . | . | . | . | . | . | . | . | . | . | . | . | . | . | . | . | . | . |
| 21-225_91G8_LC | . | . | . | . | . | . | . | . | . | . | . | . | . | . | . | . | . | . | . | . | . | . | . | . | . | . |
| 21-225_92E6_LC | . | . | . | . | F | . | . | . | . | . | . | . | . | . | . | . | . | . | . | . | . | . | . | . | . | . |
| 21-225_92F12_LC | . | . | . | . | . | . | . | . | . | . | . | . | . | . | . | . | . | . | . | . | . | . | . | . | . | . |
| 21-225_94D2_LC | . | . | . | . | . | . | . | . | . | . | . | S | . | . | . | G | . | . | . | H | . | . | . | . | . | . |
| 21-225_94E11_LC | . | . | . | . | . | . | . | . | . | . | . | . | . | . | . | . | . | . | . | . | A | . | . | . | . | . |
| 21-225_94E12_LC | . | . | . | . | . | . | . | . | . | . | . | . | . | . | . | . | . | . | . | . | . | . | . | . | . | . |
| 21-225_95E12_LC | . | . | . | . | . | . | . | . | . | . | . | . | . | . | . | . | . | . | . | . | . | . | . | . | . | . |
| 21-225_95G4_LC | . | . | . | . | Y | . | . | . | . | . | . | . | . | . | . | . | . | V | . | . | A | . | . | . | . | . |
| 21-225_95H4_LC | . | . | . | . | . | . | . | . | . | . | . | . | . | . | . | . | . | . | . | . | A | . | . | . | . | . |
| 21-225_96D10_LC | . | . | . | . | F | . | . | . | . | . | . | . | . | . | . | . | . | . | . | . | . | . | . | . | . | . |
| 21-225_96D5_LC | . | . | . | . | . | . | . | . | . | . | . | . | . | . | . | . | . | . | . | . | A | . | . | . | . | . |
| 21-225_96E2_LC | . | . | . | . | . | . | . | . | . | . | . | . | . | . | . | . | . | . | . | . | . | . | . | . | . | . |
| 21-225_96H5_LC | . | . | . | . | . | . | . | . | . | . | . | . | . | S | . | . | . | M | . | . | . | . | . | . | . | . |
| 21-225_97H4_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | Y | S | - | - | - | - | - | - | - | - | - | - | - | - | - | - | - | - | - | - | - |
| 21-225_11E5_LC | . | . | . | . | . | . | . | . | . | . | . | . | . | . | . | . | . | . | . | . | . | . | . | . | . | . |
| 21-225_12F12_LC | . | . | . | . | . | . | . | . | . | . | . | . | . | . | . | . | . | . | . | . | . | . | . | . | . | . |
| 21-225_147E4_LC | . | . | . | . | . | . | . | . | . | . | . | . | . | . | . | . | . | . | . | . | . | . | . | . | . | . |
| 21-225_148F8_LC | . | . | . | . | . | . | . | . | . | . | . | . | . | . | . | . | . | . | . | . | . | . | . | . | . | . |
| 21-225_149A2_LC | . | . | . | . | . | . | . | . | . | . | . | . | . | . | . | . | . | . | . | . | . | . | . | . | . | . |
| 21-225_149E3_LC | . | . | . | . | . | . | . | . | . | . | . | . | . | . | . | . | . | . | . | . | . | . | . | . | . | . |
| 21-225_149H4_LC | . | . | M | . | . | . | . | . | . | . | . | . | . | . | . | . | . | . | . | . | . | . | . | . | . | . |
| 21-225_154A11_LC | . | . | . | . | . | F | . | . | . | . | . | . | . | . | . | . | . | . | . | . | . | . | . | . | . | . |
| 21-225_158B12_LC | . | . | . | . | . | . | . | . | . | . | . | . | . | . | . | . | . | . | . | . | . | . | . | . | . | . |
| 21-225_162F6_LC | . | . | . | . | . | . | . | . | . | . | . | . | . | . | . | . | . | . | . | . | . | . | . | . | . | . |
| 21-225_164A4_LC | . | . | . | . | . | P | . | . | . | . | . | . | . | . | . | . | . | . | . | . | . | . | . | . | . | . |
| 21-225_164A7_LC | . | . | . | . | . | . | . | . | . | . | . | . | . | . | . | . | . | . | . | . | . | . | . | . | . | . |
| 21-225_170F6_LC | . | . | . | . | . | . | . | . | . | . | . | . | . | . | . | . | . | . | . | . | . | . | . | . | . | . |
| 21-225_174G5_LC | . | . | . | . | . | . | R | . | . | . | . | . | . | . | . | . | . | . | . | . | . | . | . | . | . | . |
| 21-225_175D10_LC | . | . | . | . | . | . | . | . | . | . | . | . | . | . | . | . | . | . | . | . | . | . | . | . | . | . |
| 21-225_177B12_LC | . | . | . | . | . | . | . | . | . | . | . | . | . | . | . | . | . | . | . | . | . | . | . | . | . | . |
| 21-225_181E5_LC | . | . | M | . | . | P | I | . | . | . | . | . | . | . | . | . | . | . | . | . | . | . | . | . | . | . |
| 21-225_190D7_LC | . | . | . | . | . | . | R | . | . | . | . | . | . | . | . | . | . | . | . | . | . | . | . | . | . | . |
| 21-225_191D8_LC | . | . | . | . | . | . | . | . | . | . | . | . | . | . | . | . | . | . | . | . | . | . | . | . | . | . |
| 21-225_193G4_LC | . | . | . | . | . | . | I | . | . | . | . | . | . | . | . | . | . | . | . | . | . | . | . | . | . | . |
| 21-225_196G8_LC | . | . | . | . | . | . | N | . | . | . | . | . | . | . | . | . | . | . | . | . | . | . | . | . | . | . |
| 21-225_197C6_LC | . | . | . | . | . | . | . | . | . | . | . | . | . | . | . | . | . | . | . | . | . | . | . | . | . | . |
| 21-225_197H4_LC | . | . | . | . | . | . | . | . | . | . | . | . | . | . | . | . | . | . | . | . | . | . | . | . | . | . |
| 21-225_200G7_LC | . | . | . | . | . | . | N | . | . | . | . | . | . | . | . | . | . | . | . | . | . | . | . | . | . | . |
| 21-225_201F5_LC | . | . | . | . | . | . | . | . | . | . | . | . | . | . | . | . | . | . | . | . | . | . | . | . | . | . |
| 21-225_213H12_LC | . | . | M | . | . | . | . | . | . | . | . | . | . | . | . | . | . | . | . | . | . | . | . | . | . | . |
| 21-225_213H7_LC | H | . | . | . | . | . | N | . | . | . | . | . | . | . | . | . | . | . | . | . | . | . | . | . | . | . |
| 21-225_222H3_LC | S | . | . | . | . | S | . | . | . | . | . | . | . | . | . | . | . | . | . | . | . | . | . | . | . | . |
| 21-225_223G10_LC | . | . | L | . | . | F | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | Y | S | - | - | - | - | - | - | - | - | - | - | - | - | - | - | - | - | - | - | - |
| 21-225_224D8_LC | . | . | . | . | . | F | . | . | . | . | . | . | . | . | . | . | . | . | . | . | . | . | . | . | . | . |
| 21-225_224H5_LC | . | . | . | . | . | . | . | . | . | . | . | . | . | . | . | . | . | . | . | . | . | . | . | . | . | . |
| 21-225_225F11_LC | . | . | . | . | . | . | T | . | . | . | . | . | . | . | . | . | . | . | . | . | . | . | . | . | . | . |
| 21-225_225F5_LC | F | . | . | . | . | . | . | . | . | . | . | . | . | . | . | . | . | . | . | . | . | . | . | . | . | . |
| 21-225_226A12_LC | . | . | . | . | . | . | . | . | . | . | . | . | . | . | . | . | . | . | . | . | . | . | . | . | . | . |
| 21-225_226D11_LC | . | . | . | . | . | . | K | . | . | . | . | . | . | . | . | . | . | . | . | . | . | . | . | . | . | . |
| 21-225_226F6_LC | . | . | . | . | . | . | T | . | . | . | . | . | . | . | . | . | . | . | . | . | . | . | . | . | . | . |
| 21-225_227C7_LC | F | . | . | . | . | . | . | . | . | . | . | . | . | . | . | . | . | . | . | . | . | . | . | . | . | . |
| 21-225_23A12_LC | . | . | . | . | . | . | D | . | . | . | . | . | . | . | . | . | . | . | . | . | . | . | . | . | . | . |
| 21-225_23G12_LC | F | . | . | . | . | S | . | . | . | . | . | . | . | . | . | . | . | . | . | . | . | . | . | . | . | . |
| 21-225_24E1_LC | . | . | . | . | F | . | . | . | . | . | . | . | . | . | . | . | . | . | . | . | . | . | . | . | . | . |
| 21-225_25A4_LC | . | . | . | . | . | . | . | . | . | . | . | . | . | . | . | . | . | . | . | . | . | . | . | . | . | . |
| 21-225_25A4.001_LC | . | . | . | . | . | . | . | . | . | . | . | . | . | . | . | . | . | . | . | . | . | . | . | . | . | . |
| 21-225_25A4.001.001_LC | . | . | . | . | . | . | . | . | . | . | . | . | . | . | . | . | . | . | . | . | . | . | . | . | . | . |
| 21-225_25A4.001.002_LC | . | . | . | . | . | . | . | . | . | . | . | . | . | . | . | . | . | . | . | . | . | . | . | . | . | . |
| 21-225_25A4.001.003_LC | . | . | . | . | . | . | . | . | . | . | . | . | . | . | . | . | . | . | . | . | . | . | . | . | . | . |
| 21-225_25A4.001.004_LC | . | . | . | . | . | . | . | . | . | . | . | . | . | . | . | . | . | . | . | . | . | . | . | . | . | . |
| 21-225_25A4.001.005_LC | . | . | . | . | . | . | . | . | . | . | . | . | . | . | . | . | . | . | . | . | . | . | . | . | . | . |
| 21-225_25A4.001.006_LC | . | . | . | . | . | . | . | . | . | . | . | . | . | . | . | . | . | . | . | . | . | . | . | . | . | . |
| 21-225_25A4.001.007_LC | . | . | . | . | . | . | . | . | . | . | . | . | . | . | . | . | . | . | . | . | . | . | . | . | . | . |
| 21-225_25A4.001.008_LC | . | . | . | . | . | . | . | . | . | . | . | . | . | . | . | . | . | . | . | . | . | . | . | . | . | . |
| 21-225_25A4.001.013_LC | . | . | . | . | . | . | . | . | . | . | . | . | . | . | . | . | . | . | . | . | . | . | . | . | . | . |
| 21-225_25A4.001.019_LC | . | . | . | . | . | . | . | . | . | . | . | . | . | . | . | . | . | . | . | . | . | . | . | . | . | . |
| 21-225_25A4.001.020_LC | . | . | . | . | . | . | . | . | . | . | . | . | . | . | . | . | . | . | . | . | . | . | . | . | . | . |
| 21-225_25A4.001.021_LC | . | . | . | . | . | . | . | . | . | . | . | . | . | . | . | . | . | . | . | . | . | . | . | . | . | . |
| 21-225_25A4.001.029_LC | . | . | . | . | . | . | . | . | . | . | . | . | . | . | . | . | . | . | . | . | . | . | . | . | . | . |
| 21-225_26C4_LC | . | . | . | . | . | . | . | . | . | . | . | . | . | . | . | . | . | . | . | . | . | . | . | . | . | . |
| 21-225_29E6_LC | . | . | . | . | . | . | . | . | . | . | . | . | . | . | . | . | . | . | . | . | . | . | . | . | . | . |
| 21-225_29H8_LC | . | . | . | . | . | . | C | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | Y | S | - | - | - | - | - | - | - | - | - | - | - | - | - | - | - | - | - | - | - |
| 21-225_43D8_LC | . | . | . | . | . | . | . | . | . | . | . | . | . | . | . | . | . | . | . | . | . | . | . | . | . | . |
| 21-225_4A2.001.023_LC | . | . | . | . | . | . | . | . | . | . | . | . | . | . | . | . | . | . | . | . | . | . | . | . | . | . |
| 21-225_4A2.001.028_LC | . | . | . | . | . | . | . | . | . | . | . | . | . | . | . | . | . | . | . | . | . | . | . | . | . | . |
| 21-225_4A2.001.029_LC | . | . | . | . | . | . | . | . | . | . | . | . | . | . | . | . | . | . | . | . | . | . | . | . | . | . |
| 21-225_4A2.001.031_LC | . | . | . | . | . | . | N | . | . | . | . | . | . | . | . | . | . | . | . | . | . | . | . | . | . | . |
| 21-225_56A1_LC | . | . | . | . | . | . | . | . | . | . | . | . | . | . | . | . | . | . | . | . | . | . | . | . | . | . |
| 21-225_57A11_LC | . | . | . | . | . | . | . | . | . | . | . | . | . | . | . | . | . | . | . | . | . | . | . | . | . | . |
| 21-225_58B9_LC | . | . | . | . | . | . | . | . | . | . | . | . | . | . | . | . | . | . | . | . | . | . | . | . | . | . |
| 21-225_59F10_LC | . | . | . | . | . | F | . | . | . | . | . | . | . | . | . | . | . | . | . | . | . | . | . | . | . | . |
| 21-225_61B5_LC | . | . | . | . | . | . | . | . | . | . | . | . | . | . | . | . | . | . | . | . | . | . | . | . | . | . |
| 21-225_70E7_LC | . | . | . | . | . | . | N | . | . | . | . | . | . | . | . | . | . | . | . | . | . | . | . | . | . | . |
| 21-225_74A6_LC | . | . | . | . | . | F | . | . | . | . | . | . | . | . | . | . | . | . | . | . | . | . | . | . | . | . |
| 21-225_74A7_LC | . | . | . | . | . | H | . | . | . | . | . | . | . | . | . | . | . | . | . | . | . | . | . | . | . | . |
| 21-225_74B1_LC | . | . | . | . | . | . | . | . | . | . | . | . | . | . | . | . | . | . | . | . | . | . | . | . | . | . |
| 21-225_74B10_LC | . | . | . | . | . | . | . | . | . | . | . | . | . | . | . | . | . | . | . | . | . | . | . | . | . | . |
| 21-225_74B5_LC | . | . | . | . | . | . | . | . | . | . | . | . | . | . | . | . | . | . | . | . | . | . | . | . | . | . |
| 21-225_74B7_LC | . | . | . | . | . | F | . | . | . | . | . | . | . | . | . | . | . | . | . | . | . | . | . | . | . | . |
| 21-225_74B8_LC | . | . | . | . | . | . | R | . | . | . | . | . | . | . | . | . | . | . | . | . | . | . | . | . | . | . |
| 21-225_74C1_LC | . | . | . | . | . | . | . | . | . | . | . | . | . | . | . | . | . | . | . | . | . | . | . | . | . | . |
| 21-225_74F6_LC | . | . | . | . | . | F | . | . | . | . | . | . | . | . | . | . | . | . | . | . | . | . | . | . | . | . |
| 21-225_74F9_LC | S | . | . | . | . | . | . | . | . | . | . | . | . | . | . | . | . | . | . | . | . | . | . | . | . | . |
| 21-225_74G4_LC | . | . | . | . | . | . | . | . | . | . | . | . | . | . | . | . | . | . | . | . | . | . | . | . | . | . |
| 21-225_74H2_LC | . | . | . | . | . | . | . | . | . | . | . | . | . | . | . | . | . | . | . | . | . | . | . | . | . | . |
| 21-225_75A10_LC | F | . | . | . | . | . | . | . | . | . | . | . | . | . | . | . | . | . | . | . | . | . | . | . | . | . |
| 21-225_75A7_LC | . | . | . | . | . | F | . | . | . | . | . | . | . | . | . | . | . | . | . | . | . | . | . | . | . | . |
| 21-225_75C4_LC | . | . | . | . | . | . | I | . | . | . | . | . | . | . | . | . | . | . | . | . | . | . | . | . | . | . |
| 21-225_75F9_LC | . | . | . | . | . | . | . | . | . | . | . | . | . | . | . | . | . | . | . | . | . | . | . | . | . | . |
| 21-225_76E11_LC | . | . | . | . | . | . | . | . | . | . | . | . | . | . | . | . | . | . | . | . | . | . | . | . | . | . |
| 21-225_76E8_LC | . | . | . | . | . | F | . | . | . | . | . | . | . | . | . | . | . | . | . | . | . | . | . | . | . | . |

# FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | Y | S | - | - | - | - | - | - | - | - | - | - | - | - | - | - | - | - | - | - | - |
| 21-225_76F3_LC | . | . | . | . | . | H | . | . | . | . | . | . | . | . | . | . | . | . | . | . | . | . | . | . | . | . |
| 21-225_76F5_LC | . | . | . | . | . | . | . | . | . | . | . | . | . | . | . | . | . | . | . | . | . | . | . | . | . | . |
| 21-225_76G2_LC | . | . | . | . | . | . | . | . | . | . | . | . | . | . | . | . | . | . | . | . | . | . | . | . | . | . |
| 21-225_77C10_LC | . | . | . | H | . | . | N | . | . | . | . | . | . | . | . | . | . | . | . | . | . | . | . | . | . | . |
| 21-225_77C7_LC | . | . | . | . | . | F | . | . | . | . | . | . | . | . | . | . | . | . | . | . | . | . | . | . | . | . |
| 21-225_77E1_LC | . | . | H | . | . | L | . | . | . | . | . | . | . | . | . | . | . | . | . | . | . | . | . | . | . | . |
| 21-225_78C1_LC | . | . | . | H | . | . | N | . | . | . | . | . | . | . | . | . | . | . | . | . | . | . | . | . | . | . |
| 21-225_78E11_LC | S | . | . | . | . | . | . | . | . | . | . | . | . | . | . | . | . | . | . | . | . | . | . | . | . | . |
| 21-225_78H12_LC | . | . | . | . | . | F | . | . | . | . | . | . | . | . | . | . | . | . | . | . | . | . | . | . | . | . |
| 21-225_79E9_LC | . | . | . | . | . | . | I | . | . | . | . | . | . | . | . | . | . | . | . | . | . | . | . | . | . | . |
| 21-225_79F12_LC | . | . | . | . | . | . | . | . | . | . | . | . | . | . | . | . | . | . | . | . | . | . | . | . | . | . |
| 21-225_79G10_LC | . | . | . | . | . | H | . | . | . | . | . | . | . | . | . | . | . | . | . | . | . | . | . | . | . | . |
| 21-225_79G6_LC | . | . | . | . | . | . | . | . | . | . | . | . | . | . | . | . | . | . | . | . | . | . | . | . | . | . |
| 21-225_80D3_LC | . | . | . | H | . | N | E | . | . | . | . | . | . | . | . | . | . | . | . | . | . | . | . | . | . | . |
| 21-225_80E9_LC | . | . | . | . | . | . | N | . | . | . | . | . | . | . | . | . | . | . | . | . | . | . | . | . | . | . |
| 21-225_80H3_LC | . | . | . | . | . | . | . | . | . | . | . | . | . | . | . | . | . | . | . | . | . | . | . | . | . | . |
| 21-225_81E5_LC | . | . | . | . | . | . | . | . | . | . | . | . | . | . | . | . | . | . | . | . | . | . | . | . | . | . |
| 21-225_81F9_LC | . | R | . | H | . | N | D | . | . | . | . | . | . | . | . | . | . | . | . | . | . | . | . | . | . | . |
| 21-225_83F3_LC | . | . | . | H | . | N | D | . | . | . | . | . | . | . | . | . | . | . | . | . | . | . | . | . | . | . |
| 21-225_83G3_LC | . | . | . | H | . | . | N | . | . | . | . | . | . | . | . | . | . | . | . | . | . | . | . | . | . | . |
| 21-225_83G7_LC | . | . | . | . | . | F | . | . | . | . | . | . | . | . | . | . | . | . | . | . | . | . | . | . | . | . |
| 21-225_84G7_LC | . | . | . | . | . | F | . | . | . | . | . | . | . | . | . | . | . | . | . | . | . | . | . | . | . | . |
| 21-225_85H2_LC | . | . | . | . | . | . | R | . | . | . | . | . | . | . | . | . | . | . | . | . | . | . | . | . | . | . |
| 21-225_85H9_LC | . | . | . | . | . | . | . | . | . | . | . | . | . | . | . | . | . | . | . | . | . | . | . | . | . | . |
| 21-225_86E9_LC | . | . | . | . | . | H | . | . | . | . | . | . | . | . | . | . | . | . | . | . | . | . | . | . | . | . |
| 21-225_88A1_LC | F | . | . | . | . | . | . | . | . | . | . | . | . | . | . | . | . | . | . | . | . | . | . | . | . | . |
| 21-225_88B4_LC | . | . | . | . | . | . | . | . | . | . | . | . | . | . | . | . | . | . | . | . | . | . | . | . | . | . |
| 21-225_88C10_LC | . | . | . | . | . | . | R | . | . | . | . | . | . | . | . | . | . | . | . | . | . | . | . | . | . | . |
| 21-225_88G2_LC | . | . | . | . | . | . | N | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | Y | S | - | - | - | - | - | - | - | - | - | - | - | - | - | - | - | - | - | - | - |
| 21-225_90D9_LC | . | . | . | . | . | L | . | . | . | . | . | . | . | . | . | . | . | . | . | . | . | . | . | . | . | . |
| 21-225_91F1_LC | . | . | . | H | . | . | N | . | . | . | . | . | . | . | . | . | . | . | . | . | . | . | . | . | . | . |
| 21-225_91G8_LC | . | . | . | . | . | . | T | . | . | . | . | . | . | . | . | . | . | . | . | . | . | . | . | . | . | . |
| 21-225_92E6_LC | . | . | . | . | . | F | . | . | . | . | . | . | . | . | . | . | . | . | . | . | . | . | . | . | . | . |
| 21-225_92F12_LC | . | . | . | . | . | . | . | . | . | . | . | . | . | . | . | . | . | . | . | . | . | . | . | . | . | . |
| 21-225_94D2_LC | . | . | . | . | . | H | . | . | . | . | . | . | . | . | . | . | . | . | . | . | . | . | . | . | . | . |
| 21-225_94E11_LC | . | . | . | . | . | H | . | . | . | . | . | . | . | . | . | . | . | . | . | . | . | . | . | . | . | . |
| 21-225_94E12_LC | . | . | . | . | . | . | . | . | . | . | . | . | . | . | . | . | . | . | . | . | . | . | . | . | . | . |
| 21-225_95E12_LC | . | . | . | . | . | . | . | . | . | . | . | . | . | . | . | . | . | . | . | . | . | . | . | . | . | . |
| 21-225_95G4_LC | . | . | . | . | . | H | . | . | . | . | . | . | . | . | . | . | . | . | . | . | . | . | . | . | . | . |
| 21-225_95H4_LC | . | . | . | . | . | H | . | . | . | . | . | . | . | . | . | . | . | . | . | . | . | . | . | . | . | . |
| 21-225_96D10_LC | . | . | . | . | . | F | . | . | . | . | . | . | . | . | . | . | . | . | . | . | . | . | . | . | . | . |
| 21-225_96D5_LC | . | . | . | . | . | . | . | . | . | . | . | . | . | . | . | . | . | . | . | . | . | . | . | . | . | . |
| 21-225_96E2_LC | . | . | . | . | . | . | R | . | . | . | . | . | . | . | . | . | . | . | . | . | . | . | . | . | . | . |
| 21-225_96H5_LC | . | . | . | . | . | . | . | . | . | . | . | . | . | . | . | . | . | . | . | . | . | . | . | . | . | . |
| 21-225_97H4_LC | . | . | . | H | . | N | D | . | . | . | . | . | . | . | . | . | . | . | . | . | . | . | . | . | . | . |

# FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | T | P | P | T | F | G | Q | G | T | K | V | E | I | K | R |
| 21-225_11E5_LC | . | . | . | . | . | . | . | . | . | . | . | . | . | . | . | . | . | . | . |
| 21-225_12F12_LC | . | . | . | . | S | . | W | . | . | . | . | . | . | . | . | . | . | . | . |
| 21-225_147E4_LC | . | . | . | . | . | . | S | . | . | . | P | . | . | . | . | . | . | . | . |
| 21-225_148F8_LC | . | . | . | . | I | . | . | . | . | . | . | . | . | . | . | . | . | . | . |
| 21-225_149A2_LC | . | . | . | . | . | . | C | S | . | . | . | . | . | . | . | . | . | . | . |
| 21-225_149E3_LC | . | . | . | . | . | . | . | . | . | . | . | . | . | . | . | . | . | . | . |
| 21-225_149H4_LC | . | . | . | . | Y | . | . | . | . | . | . | . | . | . | . | . | . | . | . |
| 21-225_154A11_LC | . | . | . | . | S | . | R | . | . | . | . | . | . | . | . | . | . | . | . |
| 21-225_158B12_LC | . | . | . | . | . | . | . | . | . | . | . | . | . | . | . | . | . | . | . |
| 21-225_162F6_LC | . | . | . | . | . | . | . | . | . | . | . | . | . | . | . | . | . | . | . |
| 21-225_164A4_LC | . | . | . | . | . | . | W | . | . | . | . | . | . | . | . | . | . | . | . |
| 21-225_164A7_LC | . | . | . | . | I | . | L | . | . | . | . | . | . | . | . | . | . | . | . |
| 21-225_170F6_LC | . | . | . | . | . | . | W | . | . | . | . | . | . | . | . | . | . | . | . |
| 21-225_174G5_LC | . | . | . | . | . | . | W | . | . | . | . | . | . | . | . | . | . | . | . |
| 21-225_175D10_LC | . | . | . | . | . | . | . | . | . | . | . | . | . | . | . | . | . | . | . |
| 21-225_177B12_LC | . | . | . | . | S | . | . | . | . | . | . | . | . | . | . | . | . | . | . |
| 21-225_181E5_LC | . | . | . | . | . | . | W | . | . | . | . | . | . | . | . | . | . | . | . |
| 21-225_190D7_LC | . | . | . | . | . | . | W | . | . | . | . | . | . | . | . | . | . | . | . |
| 21-225_191D8_LC | . | . | . | . | . | . | . | . | . | . | L | . | . | . | . | . | . | . | . |
| 21-225_193G4_LC | . | . | . | . | . | . | W | . | . | . | . | . | . | . | . | D | . | . | . |
| 21-225_196G8_LC | . | . | . | . | . | . | . | . | . | . | . | . | . | . | . | . | . | . | . |
| 21-225_197C6_LC | . | . | . | . | . | . | . | . | . | . | L | . | . | . | . | . | . | . | . |
| 21-225_197H4_LC | . | . | . | . | . | . | . | . | . | . | L | . | . | . | . | . | . | . | . |
| 21-225_200G7_LC | . | . | . | . | . | . | . | . | . | . | . | . | . | . | . | . | . | . | . |
| 21-225_201F5_LC | . | . | . | . | . | . | . | . | . | . | . | . | . | . | . | . | . | . | . |
| 21-225_213H12_LC | . | . | . | . | I | . | W | . | . | . | . | . | . | . | . | . | . | . | . |
| 21-225_213H7_LC | . | . | . | . | I | . | . | . | . | . | R | . | . | . | . | . | . | . | . |
| 21-225_222H3_LC | . | . | . | . | I | . | W | . | . | . | . | . | . | . | . | . | . | N | . |
| 21-225_223G10_LC | . | . | . | . | . | . | W | . | . | . | . | . | . | . | . | . | . | . | . |

# FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | T | P | P | T | F | G | Q | G | T | K | V | E | I | K | R |
| 21-225_224D8_LC | . | . | . | . | . | . | . | . | . | . | . | . | . | . | . | . | . | . | . |
| 21-225_224H5_LC | . | . | . | . | . | . | . | . | . | . | . | . | . | . | . | . | . | . | . |
| 21-225_225F11_LC | . | . | . | . | . | . | . | . | . | . | . | . | . | . | . | . | . | . | . |
| 21-225_225F5_LC | . | . | . | . | S | . | . | . | . | . | . | . | . | . | . | . | . | N | . |
| 21-225_226A12_LC | . | . | . | . | S | . | . | . | . | . | . | . | . | . | . | . | . | . | . |
| 21-225_226D11_LC | . | . | . | . | . | . | W | . | . | . | . | . | . | . | . | . | . | . | . |
| 21-225_226F6_LC | . | . | . | . | . | . | . | . | . | . | . | . | . | . | . | . | . | . | . |
| 21-225_227C7_LC | . | . | . | . | S | . | . | . | . | . | . | . | . | . | . | . | . | . | . |
| 21-225_23A12_LC | . | . | . | . | . | . | . | . | . | . | . | . | . | . | . | . | . | . | . |
| 21-225_23G12_LC | . | . | . | . | . | . | W | . | . | . | . | . | . | . | . | . | . | . | . |
| 21-225_24E1_LC | . | . | . | . | . | . | . | . | . | . | . | . | . | . | . | . | . | . | . |
| 21-225_25A4_LC | . | . | . | . | . | . | . | . | . | . | . | . | . | . | . | . | . | . | . |
| 21-225_25A4.001_LC | . | . | . | . | . | . | . | . | . | . | . | . | . | . | . | . | . | . | . |
| 21-225_25A4.001.001_LC | . | . | . | . | . | . | . | . | . | . | . | . | . | . | . | . | . | . | . |
| 21-225_25A4.001.002_LC | . | . | . | . | . | . | . | . | . | . | . | . | . | . | . | . | . | . | . |
| 21-225_25A4.001.003_LC | . | . | . | . | . | . | . | . | . | . | . | . | . | . | . | . | . | . | . |
| 21-225_25A4.001.004_LC | . | . | . | . | . | . | . | . | . | . | . | . | . | . | . | . | . | . | . |
| 21-225_25A4.001.005_LC | . | . | . | . | . | . | . | . | . | . | . | . | . | . | . | . | . | . | . |
| 21-225_25A4.001.006_LC | . | . | . | . | . | . | . | . | . | . | . | . | . | . | . | . | . | . | . |
| 21-225_25A4.001.007_LC | . | . | . | . | . | . | . | . | . | . | . | . | . | . | . | . | . | . | . |
| 21-225_25A4.001.008_LC | . | . | . | . | . | . | . | . | . | . | . | . | . | . | . | . | . | . | . |
| 21-225_25A4.001.013_LC | . | . | . | . | . | . | . | . | . | . | . | . | . | . | . | . | . | . | . |
| 21-225_25A4.001.019_LC | . | . | . | . | . | . | . | . | . | . | . | . | . | . | . | . | . | . | . |
| 21-225_25A4.001.020_LC | . | . | . | . | . | . | . | . | . | . | . | . | . | . | . | . | . | . | . |
| 21-225_25A4.001.021_LC | . | . | . | . | . | . | . | . | . | . | . | . | . | . | . | . | . | . | . |
| 21-225_25A4.001.029_LC | . | . | . | . | . | . | . | . | . | . | . | . | . | . | . | . | . | . | . |
| 21-225_26C4_LC | . | . | . | . | . | . | . | . | . | . | . | . | . | . | . | . | F | . | . |
| 21-225_29E6_LC | . | . | . | . | . | . | . | . | . | . | . | . | . | . | . | . | . | . | . |
| 21-225_29H8_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | T | P | P | T | F | G | Q | G | T | K | V | E | I | K | R |
| 21-225_43D8_LC | . | . | . | . | . | . | . | . | . | . | . | . | . | . | . | . | . | . | . |
| 21-225_4A2.001.023_LC | . | . | . | . | . | . | V | . | . | . | . | . | . | . | . | . | . | . | . |
| 21-225_4A2.001.028_LC | . | . | . | . | . | . | V | . | . | . | . | . | . | . | . | . | . | . | . |
| 21-225_4A2.001.029_LC | . | . | . | . | . | . | V | . | . | . | . | . | . | . | . | . | . | . | . |
| 21-225_4A2.001.031_LC | . | . | . | . | . | . | V | . | . | . | P | . | . | . | . | . | . | . | . |
| 21-225_56A1_LC | . | . | . | . | . | . | . | . | . | . | . | . | . | . | . | . | . | . | . |
| 21-225_57A11_LC | . | . | . | . | . | . | W | . | . | . | . | . | . | . | . | . | F | . | . |
| 21-225_58B9_LC | . | . | . | . | . | . | . | . | . | . | . | . | S | . | . | . | . | . | . |
| 21-225_59F10_LC | . | . | . | . | . | . | . | . | . | . | . | . | . | . | . | . | . | . | . |
| 21-225_61B5_LC | . | . | . | . | . | . | . | . | . | . | . | . | S | . | . | . | . | . | . |
| 21-225_70E7_LC | . | . | . | . | I | . | . | . | . | . | . | . | . | . | . | . | . | . | . |
| 21-225_74A6_LC | . | . | . | . | . | . | W | . | . | . | . | . | . | Q | . | . | . | . | . |
| 21-225_74A7_LC | . | . | . | . | S | . | . | . | . | . | . | . | . | T | . | Q | . | . | . |
| 21-225_74B1_LC | . | . | . | . | . | . | . | . | . | . | . | . | . | . | . | . | . | . | . |
| 21-225_74B10_LC | . | . | . | . | I | . | . | . | . | . | . | . | . | . | . | . | . | . | . |
| 21-225_74B5_LC | . | . | . | . | S | . | . | . | . | . | . | . | . | T | . | Q | . | . | . |
| 21-225_74B7_LC | . | . | . | . | S | . | . | . | . | . | . | . | . | . | . | . | . | . | . |
| 21-225_74B8_LC | . | . | . | . | . | . | W | . | . | . | . | . | . | . | . | . | . | . | . |
| 21-225_74C1_LC | . | . | . | . | . | . | W | . | . | . | . | . | . | . | . | . | . | . | . |
| 21-225_74F6_LC | . | . | . | . | V | . | L | . | . | . | . | . | . | . | . | . | . | . | . |
| 21-225_74F9_LC | . | . | . | . | S | . | . | . | . | . | . | . | . | . | . | . | . | . | . |
| 21-225_74G4_LC | . | . | . | . | I | . | . | . | . | . | . | . | . | . | . | . | . | . | . |
| 21-225_74H2_LC | . | . | . | . | I | . | . | . | . | . | . | . | . | . | . | . | . | . | . |
| 21-225_75A10_LC | . | . | . | . | S | . | . | . | . | . | . | . | . | . | . | . | . | . | . |
| 21-225_75A7_LC | . | . | . | . | S | . | . | . | . | . | . | . | . | . | . | . | . | . | . |
| 21-225_75C4_LC | . | . | . | . | . | . | . | . | . | . | . | . | . | . | . | . | . | . | . |
| 21-225_75F9_LC | . | . | . | . | S | . | . | . | . | . | . | . | . | . | . | . | . | . | . |
| 21-225_76E11_LC | . | . | . | . | . | . | . | . | . | . | . | . | . | . | . | . | . | R | . |
| 21-225_76E8_LC | . | . | . | . | S | . | L | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | T | P | P | T | F | G | Q | G | T | K | V | E | I | K | R |
| 21-225_76F3_LC | . | . | . | . | S | . | L | . | . | . | . | . | . | T | . | Q | . | . | . |
| 21-225_76F5_LC | . | . | . | . | S | . | . | . | . | . | . | . | . | . | . | . | . | . | . |
| 21-225_76G2_LC | . | . | . | . | S | . | . | . | . | . | . | . | . | . | . | . | . | . | . |
| 21-225_77C10_LC | . | . | . | . | . | . | W | K | . | V | . | . | . | . | . | . | . | T | . |
| 21-225_77C7_LC | . | . | . | . | S | . | . | . | . | . | . | . | . | R | . | . | . | . | . |
| 21-225_77E1_LC | . | . | . | . | S | . | L | . | . | . | . | . | . | . | . | . | . | . | . |
| 21-225_78C1_LC | . | . | . | . | . | . | W | K | . | V | . | . | . | . | . | . | . | . | G |
| 21-225_78E11_LC | . | . | . | . | S | . | . | . | . | . | . | . | . | . | . | . | . | . | . |
| 21-225_78H12_LC | . | . | . | . | S | . | . | . | . | . | . | . | . | . | . | . | . | . | . |
| 21-225_79E9_LC | . | . | . | . | . | . | . | . | . | . | . | . | . | . | . | . | . | . | . |
| 21-225_79F12_LC | . | . | . | . | . | . | W | . | . | . | . | . | . | . | . | . | . | . | . |
| 21-225_79G10_LC | . | . | . | . | S | . | L | . | . | . | . | . | . | T | . | Q | . | . | . |
| 21-225_79G6_LC | . | . | . | . | I | . | . | . | . | . | . | . | . | . | . | . | . | . | . |
| 21-225_80D3_LC | . | . | . | . | . | . | G | K | . | V | . | V | . | . | . | . | . | T | G |
| 21-225_80E9_LC | . | . | . | . | . | . | W | . | . | V | . | . | . | . | . | . | . | . | . |
| 21-225_80H3_LC | . | . | . | . | . | . | . | . | . | . | . | . | . | . | . | . | . | . | . |
| 21-225_81E5_LC | . | . | . | . | S | . | L | . | . | . | . | . | . | T | . | Q | . | . | . |
| 21-225_81F9_LC | . | . | . | . | . | . | G | K | . | V | . | . | I | M | . | . | . | T | G |
| 21-225_83F3_LC | . | . | . | . | . | . | W | K | . | V | . | . | I | . | . | . | . | . | G |
| 21-225_83G3_LC | . | . | . | . | . | . | W | . | . | V | . | . | . | . | . | . | . | . | . |
| 21-225_83G7_LC | . | . | . | . | S | . | L | . | . | . | . | . | . | . | . | . | . | . | . |
| 21-225_84G7_LC | . | . | . | . | S | . | . | . | . | . | . | . | . | . | . | . | . | . | . |
| 21-225_85H2_LC | . | . | . | . | . | . | W | . | . | . | . | . | . | . | . | . | . | . | . |
| 21-225_85H9_LC | . | . | . | . | . | . | . | . | . | . | . | . | . | . | . | . | . | . | . |
| 21-225_86E9_LC | . | . | . | . | S | . | L | . | . | . | . | . | . | T | . | . | . | . | . |
| 21-225_88A1_LC | . | . | . | . | S | . | . | . | . | . | . | . | . | . | . | . | . | . | . |
| 21-225_88B4_LC | . | . | . | . | . | . | . | . | . | . | . | . | . | . | . | . | . | . | . |
| 21-225_88C10_LC | . | . | . | . | A | . | . | . | . | . | . | . | . | . | . | . | . | . | . |
| 21-225_88G2_LC | . | . | . | . | . | . | W | . | . | V | . | . | . | . | . | . | . | . | G |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | T | P | P | T | F | G | Q | G | T | K | V | E | I | K | R |
| 21-225_90D9_LC | . | . | . | . | S | . | L | . | . | . | . | . | . | . | . | . | . | . | . |
| 21-225_91F1_LC | . | . | . | . | . | . | W | K | . | V | . | . | . | . | . | . | . | . | . |
| 21-225_91G8_LC | . | . | . | . | . | . | W | . | . | . | . | . | . | . | . | . | . | . | . |
| 21-225_92E6_LC | . | . | . | . | V | . | . | . | . | . | . | . | . | . | . | . | . | . | . |
| 21-225_92F12_LC | . | . | . | . | . | . | . | . | . | . | . | . | . | . | . | . | . | . | . |
| 21-225_94D2_LC | . | . | . | . | I | . | . | . | . | . | H | . | . | . | . | . | . | . | G |
| 21-225_94E11_LC | . | . | . | . | S | . | L | . | . | . | . | . | . | T | . | Q | . | . | . |
| 21-225_94E12_LC | . | . | . | . | . | . | . | . | . | . | . | . | . | . | . | . | . | . | . |
| 21-225_95E12_LC | . | . | . | . | . | . | C | S | . | . | . | . | . | . | . | . | . | . | . |
| 21-225_95G4_LC | . | . | . | . | S | . | L | . | . | . | . | . | . | T | . | Q | . | . | . |
| 21-225_95H4_LC | . | . | . | . | S | . | L | . | . | . | . | . | . | T | . | Q | . | . | . |
| 21-225_96D10_LC | . | . | . | . | V | . | . | . | . | . | . | . | . | . | . | . | . | . | . |
| 21-225_96D5_LC | . | . | . | . | S | . | . | . | . | . | . | . | . | T | . | . | . | . | . |
| 21-225_96E2_LC | . | . | . | . | . | . | W | . | . | . | . | . | . | . | . | . | . | . | . |
| 21-225_96H5_LC | . | . | . | . | . | . | C | S | . | . | . | . | . | . | . | . | . | . | . |
| 21-225_97H4_LC | . | . | . | . | . | . | W | K | . | V | . | . | . | . | . | . | . | T | G |

**FIGURE 57**

Table 100. Consensus 20 - VK1|A30/JK3 (SEQ ID NO: 50317):

DIQMTQSPSSLSASVGDRVTITCRAS--QGIR------NDLGWYQQKPGKAPKRLIYA--------ASSLQSGVPSRFSGSGSG--TEFTLTISSLQPEDFATYYCLQHNS----------------------YPFTFGPGTKVDIKR wherein:

A at position 25 can be substituted with T

G at position 30 can be substituted with D or V

I at position 31 can be substituted with M

R at position 32 can be substituted with S

N at position 39 can be substituted with K, S, D or T

D at position 40 can be substituted with N, H, Y, V, A, I or L

L at position 41 can be substituted with F

G at position 42 can be substituted with D

A at position 58 can be substituted with P, T, G, I, R or V

A at position 67 can be substituted with V

S at position 68 can be substituted with F or T

S at position 69 can be substituted with N, T, R or D

L at position 70 can be substituted with V

Q at position 71 can be substituted with L

S at position 72 can be substituted with N, T, G or R

L at position 107 can be substituted with I

Q at position 108 can be substituted with H or L

**FIGURE 57**

H at position 109 can be substituted with Y, D or L

N at position 110 can be substituted with Y, T, H, G or I

S at position 111 can be substituted with R, D, T, G or N

Y at position 135 can be substituted with F or H

P at position 136 can be substituted with L

F at position 137 can be substituted with L

T at position 138 can be substituted with K

Arg Xaa Ser Gln Xaa Xaa Xaa Xaa Xaa Xaa Xaa (SEQ ID NO: 50135)

Xaa Xaa Xaa Xaa Xaa Xaa Xaa (SEQ ID NO: 50136)

Xaa Xaa Xaa Xaa Xaa Xaa Xaa Xaa Xaa (SEQ ID NO: 50137)

Arg Ala Ser Gln Gly Ile Arg Asn Asp Leu Gly (SEQ ID NO: 50621)

Ala Ala Ser Ser Leu Gln Ser (SEQ ID NO: 50622)

Leu Gln His Asn Ser Tyr Pro Phe Thr (SEQ ID NO: 50623)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_14D6_LC | . | . | . | . | . | . | . | . | . | . | . | . | . | . | . | . | . | S | . | . | . | . | . | . | . | . |
| 21-225_157C3_LC | . | . | . | . | . | . | . | . | . | . | . | . | . | . | . | . | . | . | . | . | . | . | . | . | . | . |
| 21-225_157G7_LC | . | . | . | . | . | . | . | . | . | . | . | C | . | . | . | . | . | . | . | . | . | . | . | . | . | . |
| 21-225_157H12_LC | . | . | . | . | . | . | . | . | . | . | . | . | . | . | . | . | . | . | . | . | . | . | . | . | . | . |
| 21-225_157H4_LC | . | . | . | . | . | . | . | . | . | . | . | . | . | . | . | . | . | . | . | . | . | . | . | . | . | . |
| 21-225_157H8_LC | . | . | . | . | . | . | . | . | . | . | . | . | . | . | . | . | . | . | . | . | . | . | . | . | . | . |
| 21-225_158F5_LC | . | . | . | . | . | . | . | . | . | . | . | . | . | . | . | . | . | . | . | . | . | . | . | . | . | . |
| 21-225_158G8_LC | . | . | . | . | . | . | . | . | . | . | . | . | . | . | . | . | . | . | . | . | . | . | . | . | . | . |
| 21-225_158H6_LC | . | . | . | . | . | . | . | . | . | . | . | . | . | . | . | . | . | . | . | . | . | . | . | . | . | . |
| 21-225_159C5_LC | . | . | . | . | . | . | . | . | . | . | . | . | . | . | . | . | . | . | . | . | . | . | . | . | T | . |
| 21-225_15B11_LC | . | . | . | . | . | . | . | . | . | . | . | . | . | . | . | . | . | . | . | . | . | . | . | . | . | . |
| 21-225_160F4_LC | . | . | . | . | . | . | . | . | . | . | P | . | . | . | . | . | . | . | . | . | . | . | . | . | . | . |
| 21-225_161D11_LC | . | . | . | . | . | . | . | . | . | . | . | . | . | . | . | . | . | . | . | . | . | . | . | . | . | . |
| 21-225_161F7_LC | . | . | . | . | . | . | . | . | . | . | . | . | . | . | . | . | . | . | . | . | . | . | . | . | . | . |
| 21-225_162F2_LC | . | . | . | . | . | . | . | . | . | . | . | C | . | . | . | . | . | . | . | . | . | . | . | . | . | . |
| 21-225_162H3_LC | . | . | . | . | . | . | . | . | . | . | . | . | . | . | . | . | . | . | . | . | . | . | . | . | . | . |
| 21-225_163E2_LC | . | . | . | . | . | . | . | . | . | . | . | . | . | . | . | . | . | . | . | . | . | . | . | . | . | . |
| 21-225_163F9_LC | . | . | . | . | . | . | . | . | . | . | . | . | . | . | . | . | . | . | . | . | . | . | . | . | . | . |
| 21-225_163G10_LC | . | . | . | . | . | . | . | . | . | . | . | . | . | . | . | . | . | . | . | . | . | . | . | . | . | . |
| 21-225_16A11_LC | . | . | . | . | . | . | . | . | . | . | . | . | . | . | . | . | . | S | . | . | . | S | . | . | . | . |
| 21-225_16H1_LC | . | . | . | . | . | . | . | . | . | . | . | . | . | . | . | . | . | . | . | . | . | . | . | . | . | . |
| 21-225_16H9_LC | . | . | . | . | . | . | . | . | . | . | . | . | . | . | . | . | . | . | . | . | . | . | . | . | . | . |
| 21-225_171A11_LC | . | . | . | . | . | . | . | . | . | . | . | . | . | . | . | . | . | . | . | . | . | . | . | . | . | . |
| 21-225_177B6_LC | . | . | . | . | . | . | . | . | . | . | . | . | . | . | . | . | . | . | . | . | . | . | . | . | . | . |
| 21-225_17E5_LC | . | . | . | . | . | . | . | . | . | . | . | . | . | . | . | . | . | . | . | . | . | . | . | . | . | . |
| 21-225_17F4_LC | . | . | . | . | . | . | . | . | . | . | . | . | . | . | . | . | . | . | . | . | . | . | . | . | . | . |
| 21-225_180H7_LC | . | . | . | . | . | . | . | . | . | . | . | . | . | . | . | . | . | . | . | . | . | . | . | . | . | . |
| 21-225_181A8_LC | . | . | . | . | . | . | . | . | . | . | . | . | . | . | . | . | . | . | . | . | . | . | . | . | . | . |
| 21-225_184D11_LC | . | . | . | . | . | . | . | . | . | . | . | . | . | . | . | . | . | . | . | . | . | . | . | . | . | . |

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_184H10_LC | . | . | . | . | . | . | . | . | . | . | . | . | . | . | . | . | . | . | . | . | . | . | . | . | . | . |
| 21-225_190D6_LC | . | . | . | . | . | . | . | . | . | . | . | . | . | . | . | . | . | . | . | . | . | . | . | . | . | . |
| 21-225_192F6_LC | . | . | . | . | . | . | . | . | . | . | . | . | . | . | . | . | . | . | . | . | . | . | . | . | . | . |
| 21-225_194F10_LC | . | . | . | . | . | . | . | . | . | . | . | . | . | . | . | . | . | . | . | . | . | . | . | . | . | . |
| 21-225_195B9_LC | . | . | . | . | . | . | . | . | . | . | . | . | . | . | . | . | . | . | . | . | . | . | . | . | . | . |
| 21-225_197D7_LC | . | . | . | . | . | . | . | . | . | . | . | . | . | . | . | . | . | . | . | . | . | . | . | . | . | . |
| 21-225_197G10_LC | . | . | . | . | . | . | . | . | . | . | . | . | . | . | . | . | . | . | . | . | . | . | . | . | . | . |
| 21-225_198B9_LC | . | . | . | . | . | . | . | . | . | . | . | . | . | . | . | . | . | . | . | . | . | . | . | . | . | . |
| 21-225_198E3_LC | . | . | . | . | . | . | . | . | . | . | . | . | . | . | . | . | . | . | . | . | . | . | . | . | . | . |
| 21-225_200F6_LC | . | . | . | . | . | . | . | . | . | . | . | . | . | . | . | . | . | . | . | . | . | . | . | . | . | . |
| 21-225_202A3_LC | . | . | . | . | S | . | . | . | . | . | . | . | . | . | . | . | . | . | . | . | . | . | . | . | . | . |
| 21-225_203B9_LC | . | . | . | . | . | . | . | . | . | . | . | . | . | . | . | . | . | . | . | . | . | . | . | . | . | . |
| 21-225_210E4_LC | . | . | . | . | . | . | . | . | . | . | . | . | . | . | . | . | . | . | . | . | . | . | . | . | . | . |
| 21-225_225B6_LC | . | . | . | . | . | . | . | . | . | . | . | . | . | . | . | . | . | . | . | I | . | . | . | . | . | . |
| 21-225_225D6_LC | . | . | . | . | . | . | . | . | . | . | . | . | . | . | . | . | . | . | . | . | . | . | . | . | . | . |
| 21-225_225F8_LC | . | . | . | . | . | . | . | . | . | . | . | . | . | . | . | . | . | . | . | . | . | . | . | . | . | . |
| 21-225_226A9_LC | . | . | . | . | . | . | . | . | . | . | . | . | . | . | . | . | . | . | . | . | . | . | . | . | . | . |
| 21-225_227C12_LC | . | . | . | . | . | . | . | . | . | . | . | . | . | . | . | . | . | . | . | . | . | . | . | . | . | . |
| 21-225_227G3_LC | . | . | . | . | . | . | . | . | . | . | . | . | . | . | . | . | . | . | . | . | . | . | . | . | . | . |
| 21-225_227H5_LC | . | . | . | . | . | . | . | . | . | . | . | . | . | . | . | . | . | . | . | . | . | . | . | . | . | . |
| 21-225_22H3_LC | . | . | . | . | . | . | . | . | . | . | . | . | . | . | . | . | . | . | . | . | . | . | . | . | . | . |
| 21-225_25A9_LC | . | . | . | . | . | . | . | . | . | . | . | . | . | . | . | . | . | . | . | . | . | . | . | . | . | . |
| 21-225_25B6_LC | . | . | . | . | . | . | . | . | . | . | . | . | . | . | . | . | . | . | . | . | . | . | . | . | . | . |
| 21-225_25G5_LC | . | . | . | . | . | . | . | . | . | . | . | . | . | . | . | . | . | . | . | . | . | . | . | . | . | . |
| 21-225_27D5_LC | . | . | . | . | . | . | . | . | . | . | . | . | . | . | . | . | . | . | . | . | . | . | . | . | . | . |
| 21-225_28A9_LC | . | . | . | . | . | . | . | . | . | . | . | . | . | . | . | . | . | . | . | . | . | . | . | . | . | . |
| 21-225_28B8_LC | . | . | . | . | . | . | . | . | . | . | . | . | . | . | . | . | . | . | . | . | . | . | . | . | . | . |
| 21-225_29D7_LC | . | . | . | . | . | . | . | . | . | . | . | . | . | . | . | . | . | . | . | . | . | . | . | . | . | . |
| 21-225_29D8_LC | . | . | . | . | . | . | . | . | . | . | . | . | . | . | . | . | . | . | . | . | . | . | . | . | . | . |

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_29D9_LC | . | . | . | . | . | . | . | . | . | . | . | . | . | . | . | . | . | . | . | . | . | . | . | . | . | . |
| 21-225_30H11_LC | . | . | . | . | . | . | . | . | . | . | . | . | . | . | . | . | . | . | . | . | . | . | . | . | . | . |
| 21-225_31G9_LC | . | . | . | . | . | . | . | . | . | . | . | . | . | . | I | . | . | . | . | . | . | . | . | . | . | . |
| 21-225_43B1_LC | . | . | . | . | . | . | . | . | . | . | . | . | . | . | . | . | . | . | . | . | . | . | . | . | . | . |
| 21-225_43E1_LC | . | . | . | . | . | . | . | . | . | . | . | . | . | . | . | . | . | . | . | . | . | . | . | . | . | . |
| 21-225_43E5_LC | . | . | . | . | . | . | . | . | . | . | . | . | . | . | . | . | . | . | . | . | . | . | . | . | . | . |
| 21-225_43H8_LC | . | . | . | . | . | . | . | . | . | . | . | . | . | . | . | . | . | . | . | . | . | . | . | . | . | . |
| 21-225_44C8_LC | . | . | . | . | . | . | . | . | . | . | . | . | . | . | . | . | . | . | . | . | . | . | . | . | . | . |
| 21-225_45H2_LC | . | . | . | . | . | . | . | . | . | . | . | . | . | . | . | . | . | . | . | . | . | . | . | . | . | . |
| 21-225_46E9_LC | . | . | . | . | . | . | . | . | . | . | . | . | . | . | . | . | . | . | . | . | . | . | . | . | . | . |
| 21-225_47H7_LC | . | . | . | . | . | . | . | . | . | . | . | . | . | . | . | . | . | . | . | . | . | . | . | . | T | . |
| 21-225_48E10_LC | . | . | . | . | . | . | . | . | . | . | . | . | . | . | . | . | . | . | . | . | . | . | . | . | . | . |
| 21-225_50C4_LC | . | . | . | . | . | . | . | . | . | . | . | . | . | . | . | . | . | . | . | . | L | . | . | . | . | . |
| 21-225_51B11_LC | A | . | . | . | . | . | . | . | . | . | . | . | . | . | . | . | . | . | . | . | . | . | . | . | T | . |
| 21-225_51E4_LC | . | . | . | . | . | . | . | . | . | . | . | . | . | . | . | . | . | . | . | . | . | . | . | . | . | . |
| 21-225_51F9_LC | . | . | . | . | . | . | . | . | . | . | . | . | . | . | . | . | . | . | . | . | . | . | . | . | . | . |
| 21-225_52B9_LC | A | . | . | . | . | . | . | . | . | . | . | . | . | . | . | . | . | . | . | . | . | . | . | . | . | . |
| 21-225_52H12_LC | . | . | . | . | . | . | . | . | . | . | . | . | . | . | . | . | . | . | . | . | . | . | . | . | . | . |
| 21-225_53B10_LC | . | . | . | . | . | . | . | . | . | . | . | . | . | . | A | . | . | . | . | . | . | . | . | . | . | . |
| 21-225_53B12_LC | . | . | . | . | . | . | . | . | . | . | . | . | . | . | . | . | . | . | . | . | . | . | . | . | . | . |
| 21-225_53G1_LC | . | . | . | . | . | . | . | . | . | . | . | . | . | . | . | . | . | . | . | . | . | . | . | . | . | . |
| 21-225_54D3_LC | . | . | . | . | . | . | . | . | . | . | . | . | . | . | . | . | . | . | . | . | . | . | . | . | . | . |
| 21-225_54G7_LC | . | . | . | . | . | . | . | . | . | . | . | . | . | . | . | . | . | . | . | . | . | . | . | . | . | . |
| 21-225_55B3_LC | . | . | . | . | . | . | . | . | . | . | . | . | . | . | . | . | . | . | . | . | . | . | . | . | . | . |
| 21-225_56A5_LC | . | . | . | . | . | . | . | . | . | . | . | . | . | . | . | . | . | . | . | . | . | . | . | . | . | . |
| 21-225_56H7_LC | . | . | . | L | . | . | . | . | . | . | . | . | . | . | . | . | . | . | . | . | . | . | . | . | . | . |
| 21-225_57A4_LC | . | . | . | . | . | . | . | . | . | . | . | . | . | . | . | . | . | . | . | . | . | . | . | . | . | . |
| 21-225_57B2_LC | . | . | . | . | . | . | . | . | . | . | . | . | V | . | . | . | . | . | . | . | . | . | . | . | . | . |
| 21-225_57F4_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_58D11_LC | . | . | . | . | . | . | . | . | . | . | . | . | . | . | . | . | . | . | . | . | . | . | . | . | . | . |
| 21-225_58F5_LC | . | . | . | . | . | . | . | . | . | . | . | . | . | . | . | . | . | . | . | . | . | . | . | . | . | . |
| 21-225_59F11_LC | . | . | . | . | . | . | . | . | . | . | . | . | . | . | . | . | . | . | . | . | . | . | . | . | . | . |
| 21-225_5F4_LC | . | . | . | . | . | . | . | . | . | . | . | . | . | . | . | . | . | . | . | . | . | . | . | . | . | . |
| 21-225_60A3_LC | . | . | . | . | . | . | . | . | . | . | . | . | . | . | . | . | . | . | . | . | . | . | . | . | . | . |
| 21-225_62G3_LC | . | . | H | . | . | . | . | . | . | . | . | . | . | . | . | . | . | . | . | . | . | . | . | . | . | . |
| 21-225_64A12_LC | . | . | . | . | . | . | . | . | . | . | . | . | . | . | . | . | . | . | . | . | . | . | . | . | . | . |
| 21-225_66F9_LC | . | . | . | . | . | . | . | . | . | . | . | . | . | . | . | . | . | . | . | . | . | . | . | . | . | . |
| 21-225_67B7_LC | . | . | . | . | . | . | . | . | . | . | . | . | . | . | . | . | . | . | . | . | . | . | . | . | . | . |
| 21-225_68G8_LC | . | . | . | . | . | . | . | . | . | . | . | . | . | . | . | . | . | . | . | . | . | . | . | . | . | . |
| 21-225_71B11_LC | . | . | . | . | . | . | . | . | . | . | . | . | . | . | . | . | . | . | . | . | . | . | . | . | . | . |
| 21-225_71B6_LC | . | . | . | . | . | . | . | . | . | . | P | . | . | . | . | . | . | . | . | . | . | . | . | . | . | . |
| 21-225_71H6_LC | . | . | . | . | . | . | . | . | . | . | . | . | . | . | . | . | . | . | . | . | . | . | . | . | . | . |
| 21-225_73A6_LC | . | . | . | . | . | . | . | . | . | . | . | . | . | . | . | . | . | . | . | . | . | . | . | . | . | . |
| 21-225_74A11_LC | . | . | . | . | . | . | . | . | . | . | . | . | . | . | . | . | . | . | . | . | . | . | . | . | . | . |
| 21-225_75C11_LC | . | . | . | . | . | . | . | . | . | . | . | . | . | . | . | . | . | . | . | . | . | . | . | . | . | . |
| 21-225_76H10_LC | . | . | . | . | . | . | . | . | . | . | . | . | . | . | A | . | . | . | . | . | . | . | . | . | . | . |
| 21-225_76H12_LC | . | . | . | . | . | . | . | . | . | . | . | . | . | . | . | . | . | . | . | . | . | . | . | . | . | . |
| 21-225_84H12_LC | . | . | . | . | . | . | . | . | . | Y | Q | . | . | . | . | . | . | . | . | . | . | . | . | . | . | . |
| 21-225_86E5_LC | . | . | . | . | . | . | . | . | . | . | . | . | . | . | . | . | . | . | . | . | . | . | . | . | . | . |
| 21-225_88A5_LC | . | . | . | . | . | . | . | . | . | . | . | . | . | . | . | . | . | . | . | . | . | . | . | . | . | . |
| 21-225_96A4_LC | . | . | . | . | . | . | . | . | . | . | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_14D6_LC | . | . | . | D | . | . | . | . | . | . | . | . | . | H | . | . | . | . | . | H | . | . | . | . | . | . |
| 21-225_157C3_LC | . | . | . | . | . | . | . | . | . | . | . | . | . | . | . | . | . | . | . | . | . | . | . | . | . | . |
| 21-225_157G7_LC | . | . | . | . | . | . | . | . | . | . | . | . | . | . | . | . | . | . | . | . | . | . | . | . | . | . |
| 21-225_157H12_LC | . | . | . | . | . | . | . | . | . | . | . | . | . | . | F | . | . | . | . | . | R | . | . | . | . | . |
| 21-225_157H4_LC | . | . | . | . | . | . | . | . | . | . | . | . | . | . | . | . | . | . | . | . | . | . | . | . | . | . |
| 21-225_157H8_LC | . | . | . | . | . | . | . | . | . | . | . | . | . | . | . | . | . | . | . | . | . | . | . | . | . | . |
| 21-225_158F5_LC | . | . | . | . | . | . | . | . | . | . | . | . | . | . | . | . | . | . | . | . | . | . | . | . | . | . |
| 21-225_158G8_LC | . | . | . | D | . | . | . | . | . | . | . | . | . | . | . | . | . | . | . | . | . | . | . | . | . | . |
| 21-225_158H6_LC | . | . | . | . | . | . | . | . | . | . | . | . | S | . | . | . | . | . | . | . | . | . | . | . | . | . |
| 21-225_159C5_LC | . | . | . | . | . | . | . | . | . | . | . | . | . | . | . | . | . | . | . | . | . | . | . | . | . | . |
| 21-225_15B11_LC | . | . | . | . | . | . | . | . | . | . | . | . | . | . | . | . | . | . | . | . | . | . | . | . | . | . |
| 21-225_160F4_LC | . | . | . | . | . | . | . | . | . | . | . | . | . | . | . | . | . | . | . | . | . | . | . | . | . | . |
| 21-225_161D11_LC | . | . | . | . | . | . | . | . | . | . | . | . | . | . | . | . | . | . | . | . | . | . | . | E | . | . |
| 21-225_161F7_LC | . | . | . | . | . | . | . | . | . | . | . | . | . | . | . | . | . | . | . | . | . | . | . | E | . | . |
| 21-225_162F2_LC | . | . | . | . | . | . | . | . | . | . | . | . | . | . | . | . | . | . | . | . | . | . | . | . | . | . |
| 21-225_162H3_LC | . | . | . | . | . | . | . | . | . | . | . | . | . | . | . | . | . | . | . | . | . | . | . | . | . | . |
| 21-225_163E2_LC | . | . | . | D | . | . | . | . | . | . | . | . | . | . | . | . | . | . | . | . | . | . | . | . | . | . |
| 21-225_163F9_LC | . | . | . | D | . | . | . | . | . | . | . | . | D | . | . | . | . | . | . | . | . | . | . | . | . | . |
| 21-225_163G10_LC | . | . | . | D | . | . | . | . | . | . | . | . | . | N | . | . | . | . | . | . | . | . | . | . | . | . |
| 21-225_16A11_LC | . | . | . | D | . | . | . | . | . | . | . | . | . | H | . | . | . | . | . | R | N | . | . | . | . | . |
| 21-225_16H1_LC | . | . | . | . | . | . | . | . | . | . | . | . | D | I | . | . | . | . | . | . | . | . | . | . | . | . |
| 21-225_16H9_LC | . | . | . | . | . | . | . | . | . | . | . | . | . | Y | . | . | . | F | . | . | . | . | . | . | . | . |
| 21-225_171A11_LC | . | . | . | . | . | . | . | . | . | . | . | . | . | N | . | . | . | . | . | . | . | . | . | . | . | . |
| 21-225_177B6_LC | . | . | . | D | . | S | . | . | . | . | . | . | . | . | . | . | . | . | . | . | . | . | . | . | . | . |
| 21-225_17E5_LC | . | . | . | . | . | . | . | . | . | . | . | . | . | . | . | . | . | . | . | . | . | . | . | . | . | . |
| 21-225_17F4_LC | . | . | . | . | . | . | . | . | . | . | . | . | . | . | . | . | . | . | . | . | . | . | . | . | . | . |
| 21-225_180H7_LC | . | . | . | . | . | . | . | . | . | . | . | . | . | . | . | . | . | . | . | . | . | . | . | . | . | . |
| 21-225_181A8_LC | . | . | . | . | . | . | . | . | . | . | . | . | . | . | . | . | . | . | . | . | . | . | . | . | . | . |
| 21-225_184D11_LC | . | . | . | . | . | . | . | . | . | . | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_184H10_LC | . | . | . | . | . | . | . | . | . | . | . | . | . | . | . | . | . | . | . | . | . | . | . | . | . | . |
| 21-225_190D6_LC | . | . | . | . | . | . | . | . | . | . | . | . | . | . | . | . | . | . | . | L | . | . | . | . | . | . |
| 21-225_192F6_LC | . | . | . | . | . | . | . | . | . | . | . | . | . | . | . | . | . | . | . | L | . | . | . | . | . | . |
| 21-225_194F10_LC | . | . | . | . | . | . | . | . | . | . | . | . | . | . | . | . | . | . | . | L | . | . | . | . | . | . |
| 21-225_195B9_LC | . | . | . | . | . | . | . | . | . | . | . | . | K | . | . | . | . | . | . | . | . | . | . | . | . | . |
| 21-225_197D7_LC | . | . | . | . | . | . | . | . | . | . | . | . | K | . | . | . | . | . | . | . | . | . | . | . | . | . |
| 21-225_197G10_LC | . | . | . | . | . | . | . | . | . | . | . | . | . | . | . | . | . | . | . | . | . | . | . | . | . | . |
| 21-225_198B9_LC | . | . | . | . | . | . | . | . | . | . | . | . | K | . | . | . | . | . | . | . | . | . | . | . | . | . |
| 21-225_198E3_LC | . | . | . | . | . | . | . | . | . | . | . | . | K | . | . | . | . | . | . | . | . | . | . | . | . | . |
| 21-225_200F6_LC | . | . | . | D | . | . | . | . | . | . | . | . | . | . | . | . | . | . | . | . | . | . | . | . | . | . |
| 21-225_202A3_LC | . | . | . | . | . | . | . | . | . | . | . | . | . | . | . | . | . | . | . | . | . | . | . | . | . | . |
| 21-225_203B9_LC | . | . | . | . | . | . | . | . | . | . | . | . | . | . | . | . | . | . | . | . | . | . | . | . | . | . |
| 21-225_210E4_LC | . | . | . | . | . | . | . | . | . | . | . | . | . | . | . | . | . | . | . | . | . | . | . | . | . | . |
| 21-225_225B6_LC | . | . | . | . | M | . | . | . | . | . | . | . | K | . | . | . | . | . | . | . | . | . | . | . | . | . |
| 21-225_225D6_LC | . | . | . | . | . | . | . | . | . | . | . | . | . | . | . | . | . | . | . | . | . | . | . | . | . | . |
| 21-225_225F8_LC | . | . | . | . | . | . | . | . | . | . | . | . | . | . | . | . | . | . | . | . | . | . | . | . | . | . |
| 21-225_226A9_LC | . | . | . | . | . | . | . | . | . | . | . | . | . | . | . | . | . | . | . | . | . | . | . | . | . | . |
| 21-225_227C12_LC | . | . | . | . | . | . | . | . | . | . | . | . | . | . | . | . | . | . | . | . | . | . | . | . | . | . |
| 21-225_227G3_LC | . | . | . | . | . | . | . | . | . | . | . | . | . | . | . | . | . | . | . | . | . | . | . | . | . | . |
| 21-225_227H5_LC | . | . | . | D | . | . | . | . | . | . | . | . | . | . | . | . | . | . | . | . | . | . | . | . | . | . |
| 21-225_22H3_LC | . | . | . | D | . | . | . | . | . | . | . | . | . | N | . | . | . | . | . | . | . | . | . | . | . | . |
| 21-225_25A9_LC | . | . | . | . | . | . | . | . | . | . | . | . | . | . | . | . | . | . | . | . | . | . | . | . | . | . |
| 21-225_25B6_LC | . | . | . | . | . | . | . | . | . | . | . | . | . | . | . | . | . | . | . | . | . | . | . | . | . | . |
| 21-225_25G5_LC | . | . | . | D | . | . | . | . | . | . | . | . | . | . | . | . | . | . | . | . | . | . | . | . | . | . |
| 21-225_27D5_LC | . | . | . | . | . | . | . | . | . | . | . | . | . | . | . | . | . | . | . | . | . | . | . | . | . | . |
| 21-225_28A9_LC | . | . | . | . | . | . | . | . | . | . | . | . | . | . | . | . | . | . | . | . | . | . | . | . | . | . |
| 21-225_28B8_LC | . | . | . | . | . | . | . | . | . | . | . | . | . | . | . | . | . | . | . | H | . | . | . | . | . | . |
| 21-225_29D7_LC | . | . | . | . | . | . | . | . | . | . | . | . | . | . | . | . | . | . | . | . | . | . | . | . | . | . |
| 21-225_29D8_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_29D9_LC | . | . | . | . | . | . | . | . | . | . | . | . | . | . | . | . | . | F | . | . | . | . | . | . | . | . |
| 21-225_30H11_LC | . | . | . | . | . | . | . | . | . | . | . | . | . | . | . | . | . | . | . | . | . | . | . | . | . | . |
| 21-225_31G9_LC | . | . | . | . | . | . | . | . | . | . | . | . | . | . | . | . | . | . | . | . | . | . | . | . | . | . |
| 21-225_43B1_LC | . | . | . | . | . | . | . | . | . | . | . | . | . | . | . | . | . | . | . | . | . | . | . | . | . | . |
| 21-225_43E1_LC | . | . | . | . | . | . | . | . | . | . | . | . | . | . | . | . | . | . | . | K | . | . | . | . | . | . |
| 21-225_43E5_LC | . | . | . | . | . | . | . | . | . | . | . | . | . | . | . | . | . | . | . | . | . | . | . | . | . | . |
| 21-225_43H8_LC | . | . | . | . | . | . | . | . | . | . | . | . | . | . | . | . | . | H | . | . | . | . | . | . | . | . |
| 21-225_44C8_LC | . | . | . | . | . | . | . | . | . | . | . | . | . | . | . | . | . | . | . | . | . | . | . | . | . | . |
| 21-225_45H2_LC | . | . | . | . | . | . | . | . | . | . | . | . | . | . | . | . | . | . | . | . | . | . | . | . | . | . |
| 21-225_46E9_LC | . | . | . | . | . | . | . | . | . | . | . | . | . | . | . | . | . | . | . | . | . | . | . | . | . | . |
| 21-225_47H7_LC | . | . | . | . | . | . | . | . | . | . | . | . | . | . | . | . | . | . | . | K | . | . | . | . | . | . |
| 21-225_48E10_LC | . | . | . | . | . | . | . | . | . | . | . | . | S | . | . | . | . | . | . | . | . | . | . | . | . | . |
| 21-225_50C4_LC | . | . | . | . | . | . | . | . | . | . | . | . | . | . | . | . | . | . | . | . | . | . | . | I | . | . |
| 21-225_51B11_LC | . | . | . | . | . | . | . | . | . | . | . | . | . | . | . | . | . | . | . | . | . | . | R | . | . | . |
| 21-225_51E4_LC | . | . | . | . | . | . | . | . | . | . | . | . | . | . | . | . | . | . | . | . | . | . | . | . | . | . |
| 21-225_51F9_LC | . | . | . | . | . | . | . | . | . | . | . | . | T | . | . | . | . | . | . | . | . | . | R | . | . | . |
| 21-225_52B9_LC | . | . | . | . | . | . | . | . | . | . | . | . | . | . | . | . | . | . | . | . | . | . | R | . | . | . |
| 21-225_52H12_LC | . | . | . | . | . | . | . | . | . | . | . | . | . | N | . | . | . | . | . | . | . | . | . | . | . | . |
| 21-225_53B10_LC | . | . | . | D | . | . | . | . | . | . | . | . | . | Y | . | . | . | . | . | . | . | . | . | . | . | . |
| 21-225_53B12_LC | . | . | . | . | . | . | . | . | . | . | . | . | . | . | . | . | . | . | . | . | . | . | . | . | . | . |
| 21-225_53G1_LC | . | . | . | . | . | . | . | . | . | . | . | . | . | . | . | . | . | . | . | . | . | . | . | . | . | . |
| 21-225_54D3_LC | . | . | . | . | . | . | . | . | . | . | . | . | . | . | . | . | . | . | . | . | . | . | . | . | . | . |
| 21-225_54G7_LC | . | . | . | . | . | . | . | . | . | . | . | . | . | . | . | . | . | . | . | . | . | . | . | . | . | . |
| 21-225_55B3_LC | . | . | . | . | . | . | . | . | . | . | . | . | . | . | . | . | . | . | . | . | . | . | . | . | . | . |
| 21-225_56A5_LC | . | . | . | D | . | . | . | . | . | . | . | . | . | L | . | . | . | . | . | . | . | . | . | . | . | . |
| 21-225_56H7_LC | . | . | . | D | . | . | . | . | . | . | . | . | . | . | . | . | . | . | . | . | R | . | . | . | . | . |
| 21-225_57A4_LC | . | . | . | . | . | . | . | . | . | . | . | . | . | . | . | . | . | . | L | . | . | . | . | . | . | . |
| 21-225_57B2_LC | . | . | . | . | . | . | . | . | . | . | . | . | . | A | . | . | . | . | . | . | . | . | . | . | . | . |
| 21-225_57F4_LC | . | . | . | V | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_58D11_LC | . | . | . | . | . | . | . | . | . | . | . | . | S | . | . | D | . | . | . | . | . | . | . | . | . | . |
| 21-225_58F5_LC | . | . | . | . | . | . | . | . | . | . | . | . | . | . | . | . | . | F | L | . | T | . | . | . | . | . |
| 21-225_59F11_LC | . | . | . | . | . | . | . | . | . | . | . | . | . | . | . | . | . | . | . | . | . | . | . | . | . | . |
| 21-225_5F4_LC | . | . | . | . | . | . | . | . | . | . | . | . | . | H | . | . | . | . | . | . | . | . | . | . | . | . |
| 21-225_60A3_LC | . | . | . | . | . | . | . | . | . | . | . | . | . | . | . | . | . | . | . | . | . | . | . | . | . | . |
| 21-225_62G3_LC | . | . | . | D | . | . | . | . | . | . | . | . | . | N | . | . | . | . | . | . | . | . | . | . | . | . |
| 21-225_64A12_LC | . | . | . | . | . | . | . | . | . | . | . | . | . | . | . | . | . | . | . | . | . | . | . | . | . | . |
| 21-225_66F9_LC | . | . | . | D | . | . | . | . | . | . | . | . | . | V | . | . | . | . | . | . | . | . | . | . | . | . |
| 21-225_67B7_LC | . | . | . | . | . | . | . | . | . | . | . | . | . | . | . | . | . | F | . | . | . | . | . | . | . | . |
| 21-225_68G8_LC | . | . | . | . | . | . | . | . | . | . | . | . | . | N | . | . | . | . | . | . | R | . | . | . | . | . |
| 21-225_71B11_LC | . | . | . | . | . | . | . | . | . | . | . | . | . | . | . | D | . | . | . | . | T | . | . | . | . | . |
| 21-225_71B6_LC | . | . | . | . | . | . | . | . | . | . | . | . | . | . | . | D | . | . | . | . | . | . | . | . | . | . |
| 21-225_71H6_LC | . | . | . | . | . | . | . | . | . | . | . | . | . | V | . | . | . | . | . | . | . | . | . | . | . | . |
| 21-225_73A6_LC | . | . | . | . | . | . | . | . | . | . | . | . | . | . | . | . | . | F | . | . | . | . | . | . | . | . |
| 21-225_74A11_LC | . | . | . | D | . | . | . | . | . | . | . | . | . | . | . | . | . | . | . | . | . | . | . | . | . | . |
| 21-225_75C11_LC | . | . | . | . | . | . | . | . | . | . | . | . | . | . | . | . | . | F | . | . | . | . | . | . | . | . |
| 21-225_76H10_LC | . | . | . | D | . | . | . | . | . | . | . | . | . | Y | . | . | . | . | . | . | . | . | . | . | . | . |
| 21-225_76H12_LC | . | . | . | . | . | . | . | . | . | . | . | . | . | . | . | . | . | F | . | . | . | . | . | . | . | . |
| 21-225_84H12_LC | . | . | . | . | . | . | . | . | . | . | . | . | . | . | . | . | . | F | . | . | . | . | . | . | . | . |
| 21-225_86E5_LC | . | . | . | . | . | . | . | . | . | . | . | . | . | . | . | . | . | . | . | . | . | . | . | . | . | . |
| 21-225_88A5_LC | . | . | . | . | . | . | . | . | . | . | . | . | . | . | . | . | . | . | . | . | . | . | . | . | . | . |
| 21-225_96A4_LC | . | . | . | D | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | R | L | I | Y | A | - | - | - | - | - | - | - | - | A | S | S | L | Q | S | G | V | P | S | R | F |
| 21-225_14D6_LC | . | . | . | . | . | . | . | . | . | . | . | . | . | . | . | . | . | . | . | . | . | . | . | . | . | . |
| 21-225_157C3_LC | . | . | . | . | . | . | . | . | . | . | . | . | . | . | . | . | . | . | . | . | . | . | . | . | . | . |
| 21-225_157G7_LC | . | . | . | . | N | V | . | . | . | . | . | . | . | . | . | . | . | . | . | . | . | . | . | . | . | . |
| 21-225_157H12_LC | . | C | . | . | H | . | . | . | . | . | . | . | . | . | . | . | . | . | . | . | . | . | . | . | . | . |
| 21-225_157H4_LC | . | . | . | . | . | P | . | . | . | . | . | . | . | . | . | . | . | . | . | T | . | . | . | . | . | . |
| 21-225_157H8_LC | . | . | . | . | . | . | . | . | . | . | . | . | . | . | . | . | . | . | . | . | . | . | . | . | . | . |
| 21-225_158F5_LC | . | . | . | . | N | . | . | . | . | . | . | . | . | . | . | . | . | . | . | . | . | . | . | . | . | . |
| 21-225_158G8_LC | . | . | . | M | . | T | . | . | . | . | . | . | . | . | . | . | . | . | . | . | . | . | . | . | . | . |
| 21-225_158H6_LC | . | . | . | . | . | T | . | . | . | . | . | . | . | . | . | . | . | . | . | . | . | . | . | . | . | . |
| 21-225_159C5_LC | . | . | . | . | . | . | . | . | . | . | . | . | . | . | . | . | . | . | . | . | . | . | . | . | . | . |
| 21-225_15B11_LC | . | . | . | . | . | . | . | . | . | . | . | . | . | . | . | . | . | . | . | . | . | . | . | . | . | . |
| 21-225_160F4_LC | . | . | . | . | N | V | . | . | . | . | . | . | . | . | . | . | . | . | . | . | . | . | . | . | . | . |
| 21-225_161D11_LC | . | . | . | . | . | . | . | . | . | . | . | . | . | . | . | . | T | . | . | . | . | . | . | . | . | . |
| 21-225_161F7_LC | . | . | . | . | . | . | . | . | . | . | . | . | . | . | . | . | T | . | . | . | . | . | . | . | . | . |
| 21-225_162F2_LC | . | . | . | . | N | . | . | . | . | . | . | . | . | . | . | . | . | . | . | . | . | . | . | . | . | . |
| 21-225_162H3_LC | . | . | . | . | N | . | . | . | . | . | . | . | . | . | . | . | . | . | . | . | . | . | . | . | . | . |
| 21-225_163E2_LC | T | . | . | . | . | P | . | . | . | . | . | . | . | . | . | . | . | . | . | . | . | . | . | . | . | . |
| 21-225_163F9_LC | . | . | . | . | . | P | . | . | . | . | . | . | . | . | . | . | . | . | . | . | . | . | . | . | . | . |
| 21-225_163G10_LC | . | . | . | . | . | P | . | . | . | . | . | . | . | . | . | . | . | . | . | . | . | . | . | . | . | . |
| 21-225_16A11_LC | . | . | . | . | . | . | . | . | . | . | . | . | . | . | . | . | . | . | . | . | . | . | . | . | . | . |
| 21-225_16H1_LC | . | . | . | . | . | . | . | . | . | . | . | . | . | . | . | . | . | . | . | N | . | . | . | . | . | . |
| 21-225_16H9_LC | . | . | . | . | . | G | . | . | . | . | . | . | . | . | . | . | . | . | . | . | . | . | . | . | . | I |
| 21-225_171A11_LC | . | . | . | . | . | P | . | . | . | . | . | . | . | . | . | . | . | . | . | . | . | . | . | . | . | . |
| 21-225_177B6_LC | . | . | . | . | . | . | . | . | . | . | . | . | . | . | . | . | . | . | . | . | . | . | . | . | . | . |
| 21-225_17E5_LC | . | . | . | . | . | T | . | . | . | . | . | . | . | . | . | . | . | . | . | . | . | . | . | . | . | . |
| 21-225_17F4_LC | . | . | . | . | . | . | . | . | . | . | . | . | . | . | . | . | . | . | . | . | . | . | . | . | . | . |
| 21-225_180H7_LC | . | . | . | . | . | T | . | . | . | . | . | . | . | . | . | . | . | . | . | . | . | . | . | . | . | . |
| 21-225_181A8_LC | . | . | . | . | . | T | . | . | . | . | . | . | . | . | . | . | . | . | . | . | . | . | . | . | . | . |
| 21-225_184D11_LC | . | . | . | . | . | I | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | R | L | I | Y | A | - | - | - | - | - | - | - | - | A | S | S | L | Q | S | G | V | P | S | R | F |
| 21-225_184H10_LC | . | . | . | . | . | I | . | . | . | . | . | . | . | . | . | . | . | . | . | . | . | . | . | . | . | . |
| 21-225_190D6_LC | . | . | . | . | . | . | . | . | . | . | . | . | . | . | . | . | . | . | . | . | . | . | . | . | . | . |
| 21-225_192F6_LC | . | . | . | . | . | . | . | . | . | . | . | . | . | . | . | . | . | . | . | . | . | . | . | . | . | . |
| 21-225_194F10_LC | . | . | . | . | . | . | . | . | . | . | . | . | . | . | . | . | . | . | . | . | . | . | . | . | . | . |
| 21-225_195B9_LC | . | . | . | . | . | . | . | . | . | . | . | . | . | . | . | . | D | . | . | . | . | . | . | . | . | . |
| 21-225_197D7_LC | . | . | . | . | . | . | . | . | . | . | . | . | . | . | . | . | . | . | . | . | . | . | . | . | . | . |
| 21-225_197G10_LC | . | . | . | . | . | G | . | . | . | . | . | . | . | . | . | . | . | . | . | . | . | . | . | . | . | . |
| 21-225_198B9_LC | . | . | . | . | . | . | . | . | . | . | . | . | . | . | . | . | . | . | . | . | . | . | . | . | . | . |
| 21-225_198E3_LC | . | . | . | . | . | . | . | . | . | . | . | . | . | . | . | . | . | . | . | . | . | . | . | . | . | . |
| 21-225_200F6_LC | . | . | . | . | . | . | . | . | . | . | . | . | . | . | . | . | . | . | . | . | . | . | . | . | . | . |
| 21-225_202A3_LC | . | . | . | . | C | . | . | . | . | . | . | . | . | . | . | . | R | . | . | . | . | . | . | . | . | . |
| 21-225_203B9_LC | . | . | . | . | . | R | . | . | . | . | . | . | . | . | . | . | . | V | . | N | . | . | . | . | . | . |
| 21-225_210E4_LC | . | . | . | . | . | . | . | . | . | . | . | . | . | . | . | . | . | . | . | . | . | . | . | . | . | . |
| 21-225_225B6_LC | . | . | . | . | . | . | . | . | . | . | . | . | . | . | . | T | . | . | . | . | . | . | . | . | . | . |
| 21-225_225D6_LC | . | . | . | . | . | . | . | . | . | . | . | . | . | . | . | . | . | . | . | . | . | . | . | . | . | . |
| 21-225_225F8_LC | . | . | . | . | . | . | . | . | . | . | . | . | . | . | . | . | . | . | L | G | . | . | . | . | . | . |
| 21-225_226A9_LC | . | . | . | . | . | . | . | . | . | . | . | . | . | . | . | . | . | . | . | . | . | . | . | . | . | . |
| 21-225_227C12_LC | . | . | . | . | . | . | . | . | . | . | . | . | . | . | . | . | . | . | . | . | . | . | . | . | . | . |
| 21-225_227G3_LC | . | . | . | . | . | . | . | . | . | . | . | . | . | . | . | . | . | . | . | . | . | . | . | . | . | . |
| 21-225_227H5_LC | . | . | . | . | . | . | . | . | . | . | . | . | . | . | . | . | . | . | . | . | . | . | . | . | . | . |
| 21-225_22H3_LC | . | . | . | . | . | P | . | . | . | . | . | . | . | . | . | . | . | . | . | T | . | . | . | . | . | . |
| 21-225_25A9_LC | . | . | . | . | . | . | . | . | . | . | . | . | . | . | . | . | . | . | . | . | . | . | . | . | . | . |
| 21-225_25B6_LC | . | . | . | . | . | R | . | . | . | . | . | . | . | . | . | . | . | . | . | . | . | . | . | . | . | . |
| 21-225_25G5_LC | . | . | . | . | . | . | . | . | . | . | . | . | . | . | . | . | . | . | . | . | . | . | . | . | . | . |
| 21-225_27D5_LC | . | . | . | . | . | . | . | . | . | . | . | . | . | . | . | . | . | . | . | . | . | . | . | . | . | . |
| 21-225_28A9_LC | . | . | . | . | . | . | . | . | . | . | . | . | . | . | . | . | . | . | . | N | . | . | . | . | . | . |
| 21-225_28B8_LC | . | . | . | . | . | . | . | . | . | . | . | . | . | . | . | . | . | . | . | . | . | . | . | . | . | . |
| 21-225_29D7_LC | . | . | . | . | . | . | . | . | . | . | . | . | . | . | . | . | . | . | . | . | . | . | . | . | . | . |
| 21-225_29D8_LC | . | . | . | . | . | T | . | . | . | . | . | . | . | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | R | L | I | Y | A | - | - | - | - | - | - | - | - | A | S | S | L | Q | S | G | V | P | S | R | F |
| 21-225_184H10_LC | . | . | . | . | . | I | . | . | . | . | . | . | . | . | . | . | . | . | . | . | . | . | . | . | . | . |
| 21-225_190D6_LC | . | . | . | . | . | . | . | . | . | . | . | . | . | . | . | . | . | . | . | . | . | . | . | . | . | . |
| 21-225_192F6_LC | . | . | . | . | . | . | . | . | . | . | . | . | . | . | . | . | . | . | . | . | . | . | . | . | . | . |
| 21-225_194F10_LC | . | . | . | . | . | . | . | . | . | . | . | . | . | . | . | . | . | . | . | . | . | . | . | . | . | . |
| 21-225_195B9_LC | . | . | . | . | . | . | . | . | . | . | . | . | . | . | . | . | D | . | . | . | . | . | . | . | . | . |
| 21-225_197D7_LC | . | . | . | . | . | . | . | . | . | . | . | . | . | . | . | . | . | . | . | . | . | . | . | . | . | . |
| 21-225_197G10_LC | . | . | . | . | . | G | . | . | . | . | . | . | . | . | . | . | . | . | . | . | . | . | . | . | . | . |
| 21-225_198B9_LC | . | . | . | . | . | . | . | . | . | . | . | . | . | . | . | . | . | . | . | . | . | . | . | . | . | . |
| 21-225_198E3_LC | . | . | . | . | . | . | . | . | . | . | . | . | . | . | . | . | . | . | . | . | . | . | . | . | . | . |
| 21-225_200F6_LC | . | . | . | . | . | . | . | . | . | . | . | . | . | . | . | . | . | . | . | . | . | . | . | . | . | . |
| 21-225_202A3_LC | . | . | . | . | C | . | . | . | . | . | . | . | . | . | . | . | R | . | . | . | . | . | . | . | . | . |
| 21-225_203B9_LC | . | . | . | . | . | R | . | . | . | . | . | . | . | . | . | . | . | V | . | N | . | . | . | . | . | . |
| 21-225_210E4_LC | . | . | . | . | . | . | . | . | . | . | . | . | . | . | . | . | . | . | . | . | . | . | . | . | . | . |
| 21-225_225B6_LC | . | . | . | . | . | . | . | . | . | . | . | . | . | . | . | T | . | . | . | . | . | . | . | . | . | . |
| 21-225_225D6_LC | . | . | . | . | . | . | . | . | . | . | . | . | . | . | . | . | . | . | . | . | . | . | . | . | . | . |
| 21-225_225F8_LC | . | . | . | . | . | . | . | . | . | . | . | . | . | . | . | . | . | . | L | G | . | . | . | . | . | . |
| 21-225_226A9_LC | . | . | . | . | . | . | . | . | . | . | . | . | . | . | . | . | . | . | . | . | . | . | . | . | . | . |
| 21-225_227C12_LC | . | . | . | . | . | . | . | . | . | . | . | . | . | . | . | . | . | . | . | . | . | . | . | . | . | . |
| 21-225_227G3_LC | . | . | . | . | . | . | . | . | . | . | . | . | . | . | . | . | . | . | . | . | . | . | . | . | . | . |
| 21-225_227H5_LC | . | . | . | . | . | . | . | . | . | . | . | . | . | . | . | . | . | . | . | . | . | . | . | . | . | . |
| 21-225_22H3_LC | . | . | . | . | . | P | . | . | . | . | . | . | . | . | . | . | . | . | . | T | . | . | . | . | . | . |
| 21-225_25A9_LC | . | . | . | . | . | . | . | . | . | . | . | . | . | . | . | . | . | . | . | . | . | . | . | . | . | . |
| 21-225_25B6_LC | . | . | . | . | . | R | . | . | . | . | . | . | . | . | . | . | . | . | . | . | . | . | . | . | . | . |
| 21-225_25G5_LC | . | . | . | . | . | . | . | . | . | . | . | . | . | . | . | . | . | . | . | . | . | . | . | . | . | . |
| 21-225_27D5_LC | . | . | . | . | . | . | . | . | . | . | . | . | . | . | . | . | . | . | . | . | . | . | . | . | . | . |
| 21-225_28A9_LC | . | . | . | . | . | . | . | . | . | . | . | . | . | . | . | . | . | . | . | N | . | . | . | . | . | . |
| 21-225_28B8_LC | . | . | . | . | . | . | . | . | . | . | . | . | . | . | . | . | . | . | . | . | . | . | . | . | . | . |
| 21-225_29D7_LC | . | . | . | . | . | . | . | . | . | . | . | . | . | . | . | . | . | . | . | . | . | . | . | . | . | . |
| 21-225_29D8_LC | . | . | . | . | . | T | . | . | . | . | . | . | . | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | R | L | I | Y | A | - | - | - | - | - | - | - | - | A | S | S | L | Q | S | G | V | P | S | R | F |
| 21-225_29D9_LC | . | . | . | . | . | . | . | . | . | . | . | . | . | . | . | . | . | . | . | . | . | . | . | . | . | . |
| 21-225_30H11_LC | . | . | . | V | . | P | . | . | . | . | . | . | . | . | . | . | . | . | . | . | . | . | . | . | . | . |
| 21-225_31G9_LC | . | . | . | . | . | P | . | . | . | . | . | . | . | . | . | . | . | . | . | . | . | . | . | . | . | . |
| 21-225_43B1_LC | . | . | . | . | . | . | . | . | . | . | . | . | . | . | . | . | . | . | . | . | . | . | . | . | . | . |
| 21-225_43E1_LC | . | . | . | . | N | . | . | . | . | . | . | . | . | . | . | . | . | . | . | . | . | . | . | . | . | . |
| 21-225_43E5_LC | . | . | . | . | . | G | . | . | . | . | . | . | . | . | . | . | N | . | . | . | . | . | . | . | . | . |
| 21-225_43H8_LC | . | . | . | . | . | . | . | . | . | . | . | . | . | . | . | . | . | . | . | . | . | . | . | . | . | . |
| 21-225_44C8_LC | . | . | . | . | . | . | . | . | . | . | . | . | . | . | . | . | N | . | . | . | . | . | . | . | . | . |
| 21-225_45H2_LC | . | . | . | . | . | G | . | . | . | . | . | . | . | . | . | . | N | . | . | . | . | . | . | . | . | . |
| 21-225_46E9_LC | . | . | . | . | . | . | . | . | . | . | . | . | . | . | . | . | . | . | . | . | . | . | . | . | . | . |
| 21-225_47H7_LC | . | . | . | . | . | . | . | . | . | . | . | . | . | . | . | . | . | . | . | . | . | . | . | . | . | . |
| 21-225_48E10_LC | . | . | . | . | . | T | . | . | . | . | . | . | . | . | . | . | . | . | . | . | . | . | . | . | . | . |
| 21-225_50C4_LC | . | . | . | . | . | . | . | . | . | . | . | . | . | . | . | . | . | . | . | . | . | . | . | . | . | . |
| 21-225_51B11_LC | . | . | . | . | . | . | . | . | . | . | . | . | . | . | . | . | . | . | . | . | . | . | . | . | . | . |
| 21-225_51E4_LC | Q | . | . | . | . | . | . | . | . | . | . | . | . | . | . | . | . | . | . | . | . | . | . | . | . | . |
| 21-225_51F9_LC | Q | . | . | . | . | . | . | . | . | . | . | . | . | . | . | . | . | . | . | R | . | . | . | . | . | . |
| 21-225_52B9_LC | . | . | . | . | . | . | . | . | . | . | . | . | . | . | . | . | . | . | . | . | . | . | . | L | . | . |
| 21-225_52H12_LC | . | . | . | . | . | G | . | . | . | . | . | . | . | . | . | . | . | . | . | . | . | . | . | . | . | . |
| 21-225_53B10_LC | . | . | . | . | . | G | . | . | . | . | . | . | . | . | . | . | . | . | . | . | . | . | . | . | . | . |
| 21-225_53B12_LC | . | . | . | . | . | . | . | . | . | . | . | . | . | . | . | . | . | . | . | . | . | . | . | . | . | . |
| 21-225_53G1_LC | . | . | . | . | . | P | . | . | . | . | . | . | . | . | . | . | . | . | . | . | . | . | . | . | . | . |
| 21-225_54D3_LC | . | . | . | . | . | . | . | . | . | . | . | . | . | . | . | . | . | . | . | . | . | . | . | . | . | . |
| 21-225_54G7_LC | . | . | . | . | . | . | . | . | . | . | . | . | . | . | . | . | . | . | . | . | . | . | . | . | . | . |
| 21-225_55B3_LC | . | . | . | . | . | . | . | . | . | . | . | . | . | . | . | . | . | . | . | . | . | . | . | . | . | . |
| 21-225_56A5_LC | . | . | . | . | . | . | . | . | . | . | . | . | . | . | . | . | . | . | . | . | . | . | . | . | . | . |
| 21-225_56H7_LC | . | . | . | . | . | P | . | . | . | . | . | . | . | . | . | . | . | . | L | . | . | . | . | . | . | . |
| 21-225_57A4_LC | . | . | . | . | . | . | . | . | . | . | . | . | . | . | . | . | . | . | L | . | . | . | . | . | . | . |
| 21-225_57B2_LC | . | . | . | . | . | . | . | . | . | . | . | . | . | . | . | . | . | . | . | . | . | . | . | . | . | . |
| 21-225_57F4_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | R | L | I | Y | A | - | - | - | - | - | - | - | - | A | S | S | L | Q | S | G | V | P | S | R | F |
| 21-225_58D11_LC | . | . | . | . | . | . | . | . | . | . | . | . | . | . | . | . | . | . | . | . | . | . | . | . | . | . |
| 21-225_58F5_LC | . | . | . | . | . | . | . | . | . | . | . | . | . | . | . | . | . | . | L | . | . | . | . | . | . | . |
| 21-225_59F11_LC | . | . | . | . | . | . | . | . | . | . | . | . | . | . | . | . | . | . | . | . | . | . | . | . | . | . |
| 21-225_5F4_LC | . | . | . | . | . | . | . | . | . | . | . | . | . | . | . | . | . | . | . | . | . | . | . | . | . | . |
| 21-225_60A3_LC | . | . | . | . | . | . | . | . | . | . | . | . | . | . | . | F | . | . | . | . | . | . | . | . | . | . |
| 21-225_62G3_LC | . | . | . | . | . | T | . | . | . | . | . | . | . | . | . | . | . | . | . | . | . | . | . | . | . | . |
| 21-225_64A12_LC | T | . | . | . | . | T | . | . | . | . | . | . | . | . | . | . | T | . | . | . | . | . | . | . | . | . |
| 21-225_66F9_LC | . | . | . | . | . | T | . | . | . | . | . | . | . | . | . | . | . | . | . | . | . | . | . | . | Q | . |
| 21-225_67B7_LC | . | . | . | . | . | . | . | . | . | . | . | . | . | . | . | . | . | . | . | . | . | . | . | . | . | . |
| 21-225_68G8_LC | . | . | . | . | . | . | . | . | . | . | . | . | . | . | . | . | . | V | . | . | . | . | . | . | . | . |
| 21-225_71B11_LC | . | . | . | . | . | . | . | . | . | . | . | . | . | . | . | . | . | . | . | . | . | . | . | . | . | . |
| 21-225_71B6_LC | Q | . | . | . | . | . | . | . | . | . | . | . | . | . | . | . | . | . | . | . | . | . | . | . | . | . |
| 21-225_71H6_LC | . | . | . | . | . | . | . | . | . | . | . | . | . | . | . | . | . | . | . | . | . | . | . | . | . | . |
| 21-225_73A6_LC | . | . | . | . | . | . | . | . | . | . | . | . | . | . | . | . | N | . | . | . | . | . | . | . | . | . |
| 21-225_74A11_LC | . | . | . | . | C | . | . | . | . | . | . | . | . | . | . | . | . | . | . | . | . | . | . | . | . | . |
| 21-225_75C11_LC | . | . | . | . | . | . | . | . | . | . | . | . | . | . | . | . | . | . | . | . | . | . | . | . | . | . |
| 21-225_76H10_LC | . | . | . | . | . | G | . | . | . | . | . | . | . | . | . | . | . | . | . | . | . | . | . | . | . | . |
| 21-225_76H12_LC | . | . | . | . | . | . | . | . | . | . | . | . | . | . | . | . | R | . | . | . | . | . | . | . | . | . |
| 21-225_84H12_LC | . | . | . | . | . | . | . | . | . | . | . | . | . | . | . | . | . | . | . | . | . | . | . | . | . | . |
| 21-225_86E5_LC | . | . | . | . | . | . | . | . | . | . | . | . | . | . | . | . | . | . | . | . | . | . | . | . | . | . |
| 21-225_88A5_LC | . | . | . | . | . | . | . | . | . | . | . | . | . | . | . | . | . | . | . | . | . | . | . | . | . | . |
| 21-225_96A4_LC | . | . | . | . | . | G | . | . | . | . | . | . | . | . | V | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | P | E | D | F | A | T | Y |
| 21-225_14D6_LC | . | . | . | . | . | . | . | . | . | . | . | . | . | . | . | . | . | . | . | . | . | . | . | . | . | . |
| 21-225_157C3_LC | . | . | . | . | . | . | . | . | . | D | . | . | . | . | . | . | . | . | . | . | D | . | . | . | . | . |
| 21-225_157G7_LC | . | . | . | . | . | . | . | . | . | . | . | . | . | . | . | . | . | V | . | R | . | . | . | . | . | . |
| 21-225_157H12_LC | . | . | . | . | . | . | . | . | . | . | . | . | . | . | . | . | . | . | . | . | . | . | . | . | . | . |
| 21-225_157H4_LC | . | . | . | . | . | . | . | . | . | . | . | . | . | . | . | . | . | . | . | . | . | . | . | . | . | . |
| 21-225_157H8_LC | . | . | . | . | . | . | . | . | . | D | . | . | . | . | . | . | . | . | . | . | D | . | . | . | . | . |
| 21-225_158F5_LC | . | . | . | . | . | . | . | . | . | . | . | . | . | . | . | . | . | . | . | . | . | . | . | . | . | . |
| 21-225_158G8_LC | . | . | . | . | . | . | . | . | . | . | . | . | . | . | . | . | . | . | . | . | . | . | . | . | . | . |
| 21-225_158H6_LC | . | . | . | . | . | . | . | . | . | . | . | . | . | . | . | . | . | . | . | . | . | . | . | . | . | . |
| 21-225_159C5_LC | . | . | . | . | . | . | . | . | . | D | . | . | . | . | . | I | . | . | . | . | D | . | . | . | . | . |
| 21-225_15B11_LC | . | . | . | . | . | . | . | . | . | . | . | . | . | . | . | . | . | . | . | . | . | . | . | . | . | . |
| 21-225_160F4_LC | . | . | . | . | . | . | . | . | . | . | . | . | . | . | . | . | . | V | . | . | . | . | . | . | . | . |
| 21-225_161D11_LC | . | . | . | . | . | . | . | . | A | . | . | . | . | . | . | G | . | V | . | R | . | . | . | . | . | . |
| 21-225_161F7_LC | . | . | . | . | . | . | . | . | A | . | . | . | V | . | . | G | . | V | . | R | . | . | . | . | . | . |
| 21-225_162F2_LC | . | . | . | . | . | . | . | . | . | . | . | . | . | . | . | . | . | V | . | . | . | . | . | . | . | . |
| 21-225_162H3_LC | . | . | . | . | . | . | . | . | . | . | . | . | . | . | . | . | . | . | . | . | . | . | . | . | . | . |
| 21-225_163E2_LC | . | . | . | . | . | . | . | . | . | . | . | . | . | S | . | . | . | . | . | . | . | . | . | . | . | . |
| 21-225_163F9_LC | . | . | . | . | . | . | . | . | . | . | . | . | . | . | . | . | . | . | . | . | . | . | . | . | A | . |
| 21-225_163G10_LC | . | . | . | . | . | . | . | . | . | . | . | . | . | . | . | . | . | . | . | . | . | . | . | . | . | . |
| 21-225_16A11_LC | . | . | . | . | . | . | . | . | . | . | . | . | . | . | . | . | . | . | . | . | . | . | . | . | . | . |
| 21-225_16H1_LC | . | . | . | . | . | . | . | . | . | . | . | . | . | . | . | . | . | . | . | . | . | . | . | . | . | . |
| 21-225_16H9_LC | . | . | . | . | . | . | . | . | . | . | . | . | . | . | . | . | . | . | . | . | . | . | . | . | . | . |
| 21-225_171A11_LC | . | . | . | . | . | . | . | . | . | . | . | . | . | . | . | . | . | . | . | . | . | . | . | . | . | . |
| 21-225_177B6_LC | . | . | . | . | . | . | . | . | . | . | . | . | . | . | . | N | . | . | . | . | . | . | . | . | . | . |
| 21-225_17E5_LC | . | . | . | . | . | . | . | . | . | . | . | . | . | . | . | . | . | . | . | . | . | . | . | . | . | . |
| 21-225_17F4_LC | . | . | . | . | . | . | . | . | . | . | . | . | . | . | . | . | . | . | . | . | . | . | . | . | . | . |
| 21-225_180H7_LC | . | . | . | . | . | . | . | . | . | . | . | . | . | . | . | . | . | . | . | . | . | . | . | . | S | . |
| 21-225_181A8_LC | . | . | . | . | . | . | . | . | . | D | . | . | . | . | . | . | . | . | . | . | . | . | . | . | S | . |
| 21-225_184D11_LC | . | . | . | . | . | . | . | . | . | . | . | . | . | . | . | . | . | . | . | . | . | . | . | . | . | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | P | E | D | F | A | T | Y |
| 21-225_184H10_LC | . | . | . | . | . | . | . | . | . | . | . | . | . | . | . | . | . | . | . | . | . | . | . | . | . | . |
| 21-225_190D6_LC | . | . | . | . | . | . | . | . | P | . | . | . | . | . | . | . | . | . | . | . | . | . | . | . | . | . |
| 21-225_192F6_LC | . | . | . | . | . | . | . | . | P | . | . | . | . | . | . | . | . | . | . | . | . | . | . | . | . | . |
| 21-225_194F10_LC | . | . | . | . | . | . | . | . | P | . | . | . | . | . | . | . | . | . | . | . | . | . | . | . | . | . |
| 21-225_195B9_LC | . | . | . | . | . | . | . | . | . | . | . | . | . | . | . | . | . | . | . | . | . | . | . | . | . | . |
| 21-225_197D7_LC | . | . | . | . | . | . | . | . | . | . | . | . | . | . | . | . | . | . | . | . | . | . | . | . | . | . |
| 21-225_197G10_LC | . | . | . | . | . | . | . | . | . | . | . | . | . | . | . | . | . | . | . | . | . | . | . | . | . | . |
| 21-225_198B9_LC | . | . | . | . | . | . | . | . | . | . | . | . | . | . | . | . | . | . | . | . | . | . | . | . | . | . |
| 21-225_198E3_LC | . | . | . | . | . | . | . | . | . | . | . | . | . | . | . | . | . | . | . | . | . | . | . | . | . | . |
| 21-225_200F6_LC | . | . | . | . | . | . | . | . | . | . | . | . | . | . | . | . | . | . | . | . | . | . | . | . | . | . |
| 21-225_202A3_LC | . | . | . | . | . | . | . | . | . | . | . | . | . | . | . | . | . | . | . | . | . | . | . | . | I | . |
| 21-225_203B9_LC | . | . | . | . | . | . | . | . | . | . | . | . | . | . | . | . | . | . | . | . | . | . | . | . | . | . |
| 21-225_210E4_LC | . | . | . | . | . | . | . | . | . | . | . | . | . | . | . | . | . | . | . | . | . | . | . | . | . | . |
| 21-225_225B6_LC | . | . | . | . | . | . | . | . | . | . | . | . | . | . | . | . | . | . | . | . | . | . | . | . | . | . |
| 21-225_225D6_LC | . | . | . | . | . | . | . | . | . | . | . | . | . | . | . | . | . | . | . | . | . | . | . | . | . | . |
| 21-225_225F8_LC | . | . | . | . | . | . | . | . | . | . | . | . | . | . | . | . | . | . | . | . | . | . | . | . | . | . |
| 21-225_226A9_LC | . | . | . | . | . | . | . | . | . | . | . | . | . | . | . | . | . | . | . | . | . | . | . | . | . | . |
| 21-225_227C12_LC | . | . | . | . | . | . | . | . | . | . | . | . | . | . | . | . | . | . | . | . | . | . | . | . | . | . |
| 21-225_227G3_LC | . | . | . | . | . | . | . | . | . | . | . | . | . | . | . | . | . | . | . | . | A | . | . | . | . | . |
| 21-225_227H5_LC | . | . | . | . | . | . | . | . | . | . | . | . | . | . | . | . | . | . | . | . | . | . | . | . | . | . |
| 21-225_22H3_LC | . | . | . | . | . | . | . | . | . | . | . | . | . | . | . | . | . | . | . | . | . | . | . | . | . | . |
| 21-225_25A9_LC | . | . | . | . | . | . | . | . | . | . | . | . | . | . | . | . | . | . | . | . | . | . | . | . | . | . |
| 21-225_25B6_LC | . | . | . | . | . | . | . | . | . | . | . | . | . | . | . | . | . | . | . | . | . | . | . | . | . | . |
| 21-225_25G5_LC | . | . | . | . | . | . | . | . | . | . | . | . | . | . | V | . | . | . | . | . | . | . | . | . | . | . |
| 21-225_27D5_LC | . | . | . | . | . | . | . | . | . | . | . | . | . | . | . | . | . | . | . | . | . | . | . | . | . | . |
| 21-225_28A9_LC | . | . | . | . | . | . | . | . | . | . | . | . | . | . | . | . | . | . | . | . | . | . | . | . | . | . |
| 21-225_28B8_LC | . | . | . | . | . | . | . | . | . | . | . | . | . | . | . | . | . | . | . | . | . | . | . | . | . | . |
| 21-225_29D7_LC | . | . | . | . | . | . | . | . | . | . | . | . | . | . | . | . | . | . | . | . | . | . | . | . | . | . |
| 21-225_29D8_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | P | E | D | F | A | T | Y |
| 21-225_29D9_LC | . | . | . | . | . | . | . | . | . | . | . | . | . | . | . | . | . | . | . | . | . | . | . | . | . | . |
| 21-225_30H11_LC | . | . | . | . | . | . | . | . | . | . | . | . | . | . | . | . | . | . | . | . | . | . | . | . | . | . |
| 21-225_31G9_LC | . | . | . | . | . | . | . | . | . | . | . | . | . | . | . | . | . | . | . | . | . | . | . | . | . | . |
| 21-225_43B1_LC | . | . | . | . | . | . | . | . | . | . | . | . | . | . | V | . | . | . | . | . | . | . | . | . | . | . |
| 21-225_43E1_LC | . | . | . | . | . | . | . | . | . | . | . | . | . | . | . | . | . | . | . | . | . | . | . | . | . | . |
| 21-225_43E5_LC | . | . | . | . | . | . | . | . | . | . | . | . | . | . | V | . | . | . | . | . | . | . | . | . | . | . |
| 21-225_43H8_LC | . | . | . | . | . | . | . | . | . | . | . | . | . | S | . | . | . | . | . | . | . | . | . | . | . | . |
| 21-225_44C8_LC | . | . | . | R | . | . | . | . | . | . | . | . | . | . | . | . | . | . | . | . | . | . | . | . | . | . |
| 21-225_45H2_LC | . | . | . | . | . | . | . | . | . | . | . | . | . | . | V | . | . | . | . | . | . | . | . | . | . | . |
| 21-225_46E9_LC | . | . | . | . | . | . | . | . | . | . | . | . | . | . | V | . | . | . | . | . | . | . | . | . | . | . |
| 21-225_47H7_LC | . | . | . | . | . | . | . | . | . | . | . | . | . | . | V | . | . | . | . | . | . | . | . | . | . | . |
| 21-225_48E10_LC | . | . | . | . | . | . | . | . | . | . | . | . | . | . | . | . | . | . | . | . | . | . | . | . | . | . |
| 21-225_50C4_LC | . | . | . | . | . | . | . | . | . | . | . | . | . | . | . | . | . | . | . | . | . | . | . | . | . | . |
| 21-225_51B11_LC | . | . | . | . | . | . | . | . | . | . | . | . | . | . | . | . | . | . | . | . | . | . | . | . | . | . |
| 21-225_51E4_LC | . | . | . | . | . | . | . | . | . | . | . | . | . | . | . | . | . | . | . | . | . | . | . | . | A | . |
| 21-225_51F9_LC | . | . | . | . | . | . | . | . | . | D | . | . | . | . | . | . | . | . | . | . | . | . | . | . | S | . |
| 21-225_52B9_LC | . | . | . | . | . | . | . | . | . | . | . | . | . | . | . | R | . | . | . | . | . | . | . | . | . | . |
| 21-225_52H12_LC | . | . | . | . | . | . | . | . | . | . | . | . | . | . | . | . | . | . | . | . | . | . | . | . | . | . |
| 21-225_53B10_LC | . | . | . | . | . | . | . | . | . | . | . | . | . | . | . | . | . | . | . | . | . | . | . | . | . | F |
| 21-225_53B12_LC | . | . | . | . | . | . | . | . | . | . | . | . | . | . | . | . | . | . | . | . | . | . | . | . | . | . |
| 21-225_53G1_LC | . | . | . | . | . | . | . | . | . | . | . | S | . | . | . | . | . | . | . | . | . | . | . | . | . | . |
| 21-225_54D3_LC | . | . | . | . | . | . | . | . | . | . | . | . | . | . | . | . | . | . | . | . | . | . | . | . | . | . |
| 21-225_54G7_LC | . | . | . | . | . | . | . | . | . | . | . | . | . | . | . | . | . | . | . | . | . | . | . | . | . | . |
| 21-225_55B3_LC | . | . | . | R | . | . | . | . | . | . | . | . | . | . | . | . | . | . | . | . | . | . | . | . | S | . |
| 21-225_56A5_LC | . | . | . | . | . | . | . | . | . | . | . | . | . | . | . | . | . | . | . | . | . | . | . | . | . | . |
| 21-225_56H7_LC | . | . | . | . | . | . | . | . | . | . | . | . | . | . | . | . | . | . | . | . | . | . | . | . | . | . |
| 21-225_57A4_LC | . | . | . | . | . | . | . | . | . | . | . | . | . | . | . | . | . | . | . | . | . | . | . | . | . | . |
| 21-225_57B2_LC | . | . | . | . | . | . | . | . | . | . | . | . | . | . | . | . | . | . | . | . | . | . | . | . | . | . |
| 21-225_57F4_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | P | E | D | F | A | T | Y |
| 21-225_58D11_LC | . | . | . | . | . | . | . | . | . | . | . | . | . | A | . | . | . | . | R | . | . | . | . | . | . | . |
| 21-225_58F5_LC | G | . | . | . | . | . | . | . | . | . | . | . | . | . | . | . | . | . | . | . | . | . | . | . | . | . |
| 21-225_59F11_LC | . | . | . | . | . | . | . | . | . | . | . | . | . | . | . | . | . | . | . | . | . | . | . | . | . | . |
| 21-225_5F4_LC | . | . | . | . | . | . | . | . | . | . | . | . | . | . | V | . | . | . | . | . | . | . | . | . | . | . |
| 21-225_60A3_LC | . | . | . | . | . | . | . | . | . | . | . | . | . | . | . | . | . | . | . | . | . | . | . | . | . | . |
| 21-225_62G3_LC | . | . | . | . | . | . | . | . | . | . | . | . | . | . | . | . | . | . | . | . | . | . | . | . | . | . |
| 21-225_64A12_LC | . | . | . | . | . | . | . | . | . | . | . | . | . | . | . | . | . | . | . | . | . | . | . | . | . | . |
| 21-225_66F9_LC | . | . | . | . | . | . | . | . | . | . | . | . | . | . | . | . | . | . | . | . | . | . | . | . | . | . |
| 21-225_67B7_LC | . | . | . | . | . | . | . | . | . | . | . | S | . | . | . | . | . | . | . | . | . | . | . | . | . | . |
| 21-225_68G8_LC | . | . | . | . | . | . | . | . | . | . | . | . | . | . | . | . | . | . | . | . | . | . | . | . | . | . |
| 21-225_71B11_LC | . | . | G | . | . | . | . | . | . | . | . | S | . | . | . | . | . | . | . | . | . | . | . | T | . | . |
| 21-225_71B6_LC | . | . | . | . | . | . | . | . | . | . | . | . | . | . | . | . | . | . | . | . | D | . | . | . | . | . |
| 21-225_71H6_LC | . | . | . | . | . | . | . | . | . | . | . | . | . | . | . | . | . | . | . | . | . | . | . | . | . | . |
| 21-225_73A6_LC | . | . | . | . | . | . | . | . | . | . | . | . | . | . | . | . | . | . | . | . | . | . | . | . | . | . |
| 21-225_74A11_LC | . | . | . | . | . | . | . | . | . | . | . | . | . | . | . | . | . | . | . | . | . | . | . | . | . | . |
| 21-225_75C11_LC | . | . | . | . | . | . | . | . | . | . | . | . | . | . | . | . | . | . | . | . | . | . | . | . | . | . |
| 21-225_76H10_LC | . | . | . | . | . | . | . | . | . | . | . | . | . | . | . | . | . | . | . | . | . | . | . | . | . | F |
| 21-225_76H12_LC | . | . | . | . | . | . | . | . | . | . | . | . | . | . | . | . | . | . | . | . | . | . | . | . | . | . |
| 21-225_84H12_LC | . | . | . | . | . | . | . | . | . | . | . | . | . | . | . | . | . | . | . | . | . | . | . | . | . | . |
| 21-225_86E5_LC | . | . | . | . | . | . | . | . | . | . | . | . | . | . | . | . | . | . | . | . | . | . | . | . | . | . |
| 21-225_88A5_LC | . | . | . | . | . | . | . | . | . | . | . | . | . | . | . | . | . | . | . | . | . | . | . | . | . | . |
| 21-225_96A4_LC | . | . | . | . | . | . | . | . | . | . | . | . | . | . | . | . | . | . | . | R | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_14D6_LC | . | . | . | . | Y | . | . | . | . | . | . | . | . | . | . | . | . | . | . | . | . | . | . | . | . | . |
| 21-225_157C3_LC | . | . | . | . | . | . | . | . | . | . | . | . | . | . | . | . | . | . | . | . | . | . | . | . | . | . |
| 21-225_157G7_LC | . | . | I | . | D | . | . | . | . | . | . | . | . | . | . | . | . | . | . | . | . | . | . | . | . | . |
| 21-225_157H12_LC | . | . | . | . | . | Y | . | . | . | . | . | . | . | . | . | . | . | . | . | . | . | . | . | . | . | . |
| 21-225_157H4_LC | . | . | . | . | D | . | . | . | . | . | . | . | . | . | . | . | . | . | . | . | . | . | . | . | . | . |
| 21-225_157H8_LC | . | . | . | . | . | . | . | . | . | . | . | . | . | . | . | . | . | . | . | . | . | . | . | . | . | . |
| 21-225_158F5_LC | . | . | . | . | D | . | . | . | . | . | . | . | . | . | . | . | . | . | . | . | . | . | . | . | . | . |
| 21-225_158G8_LC | . | . | . | . | . | . | . | . | . | . | . | . | . | . | . | . | . | . | . | . | . | . | . | . | . | . |
| 21-225_158H6_LC | . | . | . | . | . | . | . | . | . | . | . | . | . | . | . | . | . | . | . | . | . | . | . | . | . | . |
| 21-225_159C5_LC | . | . | . | . | . | . | . | . | . | . | . | . | . | . | . | . | . | . | . | . | . | . | . | . | . | . |
| 21-225_15B11_LC | . | . | . | . | . | . | . | . | . | . | . | . | . | . | . | . | . | . | . | . | . | . | . | . | . | . |
| 21-225_160F4_LC | . | . | I | . | D | . | . | . | . | . | . | . | . | . | . | . | . | . | . | . | . | . | . | . | . | . |
| 21-225_161D11_LC | . | G | . | . | Y | . | R | . | . | . | . | . | . | . | . | . | . | . | . | . | . | . | . | . | . | . |
| 21-225_161F7_LC | . | G | . | L | Y | I | R | . | . | . | . | . | . | . | . | . | . | . | . | . | . | . | . | . | . | . |
| 21-225_162F2_LC | . | . | I | . | D | . | . | . | . | . | . | . | . | . | . | . | . | . | . | . | . | . | . | . | . | . |
| 21-225_162H3_LC | . | . | . | . | D | . | . | . | . | . | . | . | . | . | . | . | . | . | . | . | . | . | . | . | . | . |
| 21-225_163E2_LC | . | . | . | . | . | . | . | . | . | . | . | . | . | . | . | . | . | . | . | . | . | . | . | . | . | . |
| 21-225_163F9_LC | . | . | . | . | D | . | . | . | . | . | . | . | . | . | . | . | . | . | . | . | . | . | . | . | . | . |
| 21-225_163G10_LC | . | . | . | . | D | Y | . | . | . | . | . | . | . | . | . | . | . | . | . | . | . | . | . | . | . | . |
| 21-225_16A11_LC | . | . | . | . | Y | . | . | . | . | . | . | . | . | . | . | . | . | . | . | . | . | . | . | . | . | . |
| 21-225_16H1_LC | . | . | . | . | Y | H | . | . | . | . | . | . | . | . | . | . | . | . | . | . | . | . | . | . | . | . |
| 21-225_16H9_LC | . | . | . | . | Y | H | . | . | . | . | . | . | . | . | . | . | . | . | . | . | . | . | . | . | . | . |
| 21-225_171A11_LC | . | . | . | . | D | H | . | . | . | . | . | . | . | . | . | . | . | . | . | . | . | . | . | . | . | . |
| 21-225_177B6_LC | . | . | . | . | L | . | . | . | . | . | . | . | . | . | . | . | . | . | . | . | . | . | . | . | . | . |
| 21-225_17E5_LC | . | . | . | . | . | . | . | . | . | . | . | . | . | . | . | . | . | . | . | . | . | . | . | . | . | . |
| 21-225_17F4_LC | . | . | . | . | . | . | . | . | . | . | . | . | . | . | . | . | . | . | . | . | . | . | . | . | . | . |
| 21-225_180H7_LC | . | . | . | . | . | . | . | . | . | . | . | . | . | . | . | . | . | . | . | . | . | . | . | . | . | . |
| 21-225_181A8_LC | . | . | . | . | . | . | . | . | . | . | . | . | . | . | . | . | . | . | . | . | . | . | . | . | . | . |
| 21-225_184D11_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | N | S | - | - | - | - | - | - | - | - | - | - | - | - | - | - | - | - | - | - | - |
| 21-225_184H10_LC | . | . | . | . | . | . | . | . | . | . | . | . | . | . | . | . | . | . | . | . | . | . | . | . | . | . |
| 21-225_190D6_LC | . | . | . | . | . | Y | . | . | . | . | . | . | . | . | . | . | . | . | . | . | . | . | . | . | . | . |
| 21-225_192F6_LC | . | . | . | . | . | Y | . | . | . | . | . | . | . | . | . | . | . | . | . | . | . | . | . | . | . | . |
| 21-225_194F10_LC | . | . | . | . | . | Y | . | . | . | . | . | . | . | . | . | . | . | . | . | . | . | . | . | . | . | . |
| 21-225_195B9_LC | C | . | . | . | . | Y | R | . | . | . | . | . | . | . | . | . | . | . | . | . | . | . | . | . | . | . |
| 21-225_197D7_LC | V | . | . | . | . | Y | R | . | . | . | . | . | . | . | . | . | . | . | . | . | . | . | . | . | . | . |
| 21-225_197G10_LC | . | . | . | . | . | Y | R | . | . | . | . | . | . | . | . | . | . | . | . | . | . | . | . | . | . | . |
| 21-225_198B9_LC | V | . | . | . | . | Y | R | . | . | . | . | . | . | . | . | . | . | . | . | . | . | . | . | . | . | . |
| 21-225_198E3_LC | V | . | . | . | . | Y | R | . | . | . | . | . | . | . | . | . | . | . | . | . | . | . | . | . | . | . |
| 21-225_200F6_LC | H | . | . | . | . | Y | R | . | . | . | . | . | . | . | . | . | . | . | . | . | . | . | . | . | . | . |
| 21-225_202A3_LC | . | . | . | . | . | H | D | . | . | . | . | . | . | . | . | . | . | . | . | . | . | . | . | . | . | . |
| 21-225_203B9_LC | H | . | . | . | . | . | . | . | . | . | . | . | . | . | . | . | . | . | . | . | . | . | . | . | . | . |
| 21-225_210E4_LC | . | . | . | . | . | . | . | . | . | . | . | . | . | . | . | . | . | . | . | . | . | . | . | . | . | . |
| 21-225_225B6_LC | . | . | . | . | . | . | . | . | . | . | . | . | . | . | . | . | . | . | . | . | . | . | . | . | . | . |
| 21-225_225D6_LC | C | . | . | . | . | . | T | . | . | . | . | . | . | . | . | . | . | . | . | . | . | . | . | . | . | . |
| 21-225_225F8_LC | . | . | . | . | . | . | . | . | . | . | . | . | . | . | . | . | . | . | . | . | . | . | . | . | . | . |
| 21-225_226A9_LC | . | . | . | . | . | . | . | . | . | . | . | . | . | . | . | . | . | . | . | . | . | . | . | . | . | . |
| 21-225_227C12_LC | . | . | . | . | . | . | . | . | . | . | . | . | . | . | . | . | . | . | . | . | . | . | . | . | . | . |
| 21-225_227G3_LC | . | . | . | H | . | . | . | . | . | . | . | . | . | . | . | . | . | . | . | . | . | . | . | . | . | . |
| 21-225_227H5_LC | . | . | . | . | . | . | . | . | . | . | . | . | . | . | . | . | . | . | . | . | . | . | . | . | . | . |
| 21-225_22H3_LC | F | . | . | . | D | . | . | . | . | . | . | . | . | . | . | . | . | . | . | . | . | . | . | . | . | . |
| 21-225_25A9_LC | . | . | . | . | . | . | . | . | . | . | . | . | . | . | . | . | . | . | . | . | . | . | . | . | . | . |
| 21-225_25B6_LC | . | . | . | . | . | . | . | . | . | . | . | . | . | . | . | . | . | . | . | . | . | . | . | . | . | . |
| 21-225_25G5_LC | . | . | . | . | . | . | . | . | . | . | . | . | . | . | . | . | . | . | . | . | . | . | . | . | . | . |
| 21-225_27D5_LC | . | . | . | . | . | . | . | . | . | . | . | . | . | . | . | . | . | . | . | . | . | . | . | . | . | . |
| 21-225_28A9_LC | . | . | . | . | . | . | R | . | . | . | . | . | . | . | . | . | . | . | . | . | . | . | . | . | . | . |
| 21-225_28B8_LC | . | . | . | . | . | . | . | . | . | . | . | . | . | . | . | . | . | . | . | . | . | . | . | . | . | . |
| 21-225_29D7_LC | . | . | . | . | . | . | . | . | . | . | . | . | . | . | . | . | . | . | . | . | . | . | . | . | . | . |
| 21-225_29D8_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | L | Q | H | N | S | - | - | - | - | - | - | - | - | - | - | - | - | - | - | - | - | - | - | - |
| 21-225_29D9_LC | . | . | . | . | . | . | T | . | . | . | . | . | . | . | . | . | . | . | . | . | . | . | . | . | . | . |
| 21-225_30H11_LC | . | . | . | . | D | . | . | . | . | . | . | . | . | . | . | . | . | . | . | . | . | . | . | . | . | . |
| 21-225_31G9_LC | . | . | . | . | D | . | . | . | . | . | . | . | . | . | . | . | . | . | . | . | . | . | . | . | . | . |
| 21-225_43B1_LC | F | . | . | . | . | T | . | . | . | . | . | . | . | . | . | . | . | . | . | . | . | . | . | . | . | . |
| 21-225_43E1_LC | . | . | . | . | . | . | . | . | . | . | . | . | . | . | . | . | . | . | . | . | . | . | . | . | . | . |
| 21-225_43E5_LC | F | . | . | . | . | T | . | . | . | . | . | . | . | . | . | . | . | . | . | . | . | . | . | . | . | . |
| 21-225_43H8_LC | . | . | . | . | . | . | . | . | . | . | . | . | . | . | . | . | . | . | . | . | . | . | . | . | . | . |
| 21-225_44C8_LC | . | . | . | . | . | . | . | . | . | . | . | . | . | . | . | . | . | . | . | . | . | . | . | . | . | . |
| 21-225_45H2_LC | F | . | . | . | . | T | . | . | . | . | . | . | . | . | . | . | . | . | . | . | . | . | . | . | . | . |
| 21-225_46E9_LC | F | . | . | . | . | T | . | . | . | . | . | . | . | . | . | . | . | . | . | . | . | . | . | . | . | . |
| 21-225_47H7_LC | F | . | . | . | . | T | . | . | . | . | . | . | . | . | . | . | . | . | . | . | . | . | . | . | . | . |
| 21-225_48E10_LC | . | . | . | . | Y | . | . | . | . | . | . | . | . | . | . | . | . | . | . | . | . | . | . | . | . | . |
| 21-225_50C4_LC | . | . | . | . | . | . | R | . | . | . | . | . | . | . | . | . | . | . | . | . | . | . | . | . | . | . |
| 21-225_51B11_LC | . | . | . | . | . | . | . | . | . | . | . | . | . | . | . | . | . | . | . | . | . | . | . | . | . | . |
| 21-225_51E4_LC | . | . | . | . | . | . | . | . | . | . | . | . | . | . | . | . | . | . | . | . | . | . | . | . | . | . |
| 21-225_51F9_LC | . | . | . | . | . | . | . | . | . | . | . | . | . | . | . | . | . | . | . | . | . | . | . | . | . | . |
| 21-225_52B9_LC | . | . | . | . | . | . | . | . | . | . | . | . | . | . | . | . | . | . | . | . | . | . | . | . | . | . |
| 21-225_52H12_LC | . | . | . | . | Y | . | . | . | . | . | . | . | . | . | . | . | . | . | . | . | . | . | . | . | . | . |
| 21-225_53B10_LC | . | . | . | . | Y | . | . | . | . | . | . | . | . | . | . | . | . | . | . | . | . | . | . | . | . | . |
| 21-225_53B12_LC | . | . | . | . | . | . | . | . | . | . | . | . | . | . | . | . | . | . | . | . | . | . | . | . | . | . |
| 21-225_53G1_LC | . | . | . | . | D | . | . | . | . | . | . | . | . | . | . | . | . | . | . | . | . | . | . | . | . | . |
| 21-225_54D3_LC | . | . | . | . | . | . | . | . | . | . | . | . | . | . | . | . | . | . | . | . | . | . | . | . | . | . |
| 21-225_54G7_LC | . | . | . | H | . | . | T | . | . | . | . | . | . | . | . | . | . | . | . | . | . | . | . | . | . | . |
| 21-225_55B3_LC | . | . | . | . | . | . | . | . | . | . | . | . | . | . | . | . | . | . | . | . | . | . | . | . | . | . |
| 21-225_56A5_LC | . | . | . | . | Y | . | . | . | . | . | . | . | . | . | . | . | . | . | . | . | . | . | . | . | . | . |
| 21-225_56H7_LC | . | . | . | . | D | . | . | . | . | . | . | . | . | . | . | . | . | . | . | . | . | . | . | . | . | . |
| 21-225_57A4_LC | . | . | . | . | . | . | . | . | . | . | . | . | . | . | . | . | . | . | . | . | . | . | . | . | . | . |
| 21-225_57B2_LC | . | . | . | . | . | . | . | . | . | . | . | . | . | . | . | . | . | . | . | . | . | . | . | . | . | . |
| 21-225_57F4_LC | H | . | . | . | Y | G | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | L | Q | H | N | S | - | - | - | - | - | - | - | - | - | - | - | - | - | - | - | - | - | - | - |
| 21-225_58D11_LC | . | . | . | . | . | . | N | . | . | . | . | . | . | . | . | . | . | . | . | . | . | . | . | . | . | . |
| 21-225_58F5_LC | . | . | . | . | . | . | . | . | . | . | . | . | . | . | . | . | . | . | . | . | . | . | . | . | . | . |
| 21-225_59F11_LC | . | . | . | . | . | . | R | . | . | . | . | . | . | . | . | . | . | . | . | . | . | . | . | . | . | . |
| 21-225_5F4_LC | . | . | . | . | Y | . | G | . | . | . | . | . | . | . | . | . | . | . | . | . | . | . | . | . | . | . |
| 21-225_60A3_LC | . | . | . | . | . | . | . | . | . | . | . | . | . | . | . | . | . | . | . | . | . | . | . | . | . | . |
| 21-225_62G3_LC | . | . | . | . | Y | . | . | . | . | . | . | . | . | . | . | . | . | . | . | . | . | . | . | . | . | . |
| 21-225_64A12_LC | . | . | . | . | . | . | G | . | . | . | . | . | . | . | . | . | . | . | . | . | . | . | . | . | . | . |
| 21-225_66F9_LC | . | . | . | . | Y | . | . | . | . | . | . | . | . | . | . | . | . | . | . | . | . | . | . | . | . | . |
| 21-225_67B7_LC | . | . | . | H | . | . | . | . | . | . | . | . | . | . | . | . | . | . | . | . | . | . | . | . | . | . |
| 21-225_68G8_LC | . | . | . | . | Y | . | . | . | . | . | . | . | . | . | . | . | . | . | . | . | . | . | . | . | . | . |
| 21-225_71B11_LC | . | . | . | . | . | . | T | . | . | . | . | . | . | . | . | . | . | . | . | . | . | . | . | . | . | . |
| 21-225_71B6_LC | . | . | . | . | . | . | . | . | . | . | . | . | . | . | . | . | . | . | . | . | . | . | . | . | . | . |
| 21-225_71H6_LC | . | . | . | . | Y | . | . | . | . | . | . | . | . | . | . | . | . | . | . | . | . | . | . | . | . | . |
| 21-225_73A6_LC | . | . | . | . | . | . | . | . | . | . | . | . | . | . | . | . | . | . | . | . | . | . | . | . | . | . |
| 21-225_74A11_LC | F | . | . | . | . | . | D | . | . | . | . | . | . | . | . | . | . | . | . | . | . | . | . | . | . | . |
| 21-225_75C11_LC | . | . | . | . | . | . | D | . | . | . | . | . | . | . | . | . | . | . | . | . | . | . | . | . | . | . |
| 21-225_76H10_LC | . | . | . | . | Y | . | . | . | . | . | . | . | . | . | . | . | . | . | . | . | . | . | . | . | . | . |
| 21-225_76H12_LC | . | . | . | . | . | . | D | . | . | . | . | . | . | . | . | . | . | . | . | . | . | . | . | . | . | . |
| 21-225_84H12_LC | . | . | . | . | . | . | D | . | . | . | . | . | . | . | . | . | . | . | . | . | . | . | . | . | . | . |
| 21-225_86E5_LC | H | . | . | . | . | . | D | . | . | . | . | . | . | . | . | . | . | . | . | . | . | . | . | . | . | . |
| 21-225_88A5_LC | . | . | . | . | . | . | D | . | . | . | . | . | . | . | . | . | . | . | . | . | . | . | . | . | . | . |
| 21-225_96A4_LC | . | . | . | . | . | . | D | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | F | T | F | G | P | G | T | K | V | D | I | K | R |
| 21-225_14D6_LC | . | . | . | . | . | . | . | . | . | . | . | . | . | . | . | . | . | N | . |
| 21-225_157C3_LC | . | . | . | . | . | . | . | . | . | . | . | . | . | . | . | . | . | . | . |
| 21-225_157G7_LC | . | . | . | . | H | . | . | . | . | . | . | . | . | . | . | E | . | . | . |
| 21-225_157H12_LC | . | . | . | . | . | . | . | . | . | . | . | . | . | . | . | . | . | . | . |
| 21-225_157H4_LC | . | . | . | . | . | . | . | . | . | . | . | . | . | . | . | . | . | . | . |
| 21-225_157H8_LC | . | . | . | . | . | . | . | . | . | . | . | . | . | . | . | . | . | . | . |
| 21-225_158F5_LC | . | . | . | . | H | . | . | . | . | . | . | . | . | . | . | E | . | . | . |
| 21-225_158G8_LC | . | . | . | . | . | . | . | . | . | . | . | . | . | . | . | . | . | . | . |
| 21-225_158H6_LC | . | . | . | . | . | . | . | . | . | . | . | . | . | . | . | . | . | . | . |
| 21-225_159C5_LC | . | . | . | . | . | . | . | . | . | . | . | . | . | . | . | . | . | . | . |
| 21-225_15B11_LC | . | . | . | . | . | . | . | . | . | . | . | . | . | . | . | . | . | . | . |
| 21-225_160F4_LC | . | . | . | . | H | . | . | . | . | . | . | . | . | . | . | E | . | . | . |
| 21-225_161D11_LC | . | . | . | . | . | . | . | . | . | . | R | . | . | . | . | . | . | . | . |
| 21-225_161F7_LC | . | . | . | . | . | . | . | . | . | . | R | . | . | . | . | . | . | . | . |
| 21-225_162F2_LC | . | . | . | . | H | . | . | . | . | . | . | . | . | . | . | E | . | . | . |
| 21-225_162H3_LC | . | . | . | . | H | . | . | . | . | . | . | . | . | . | . | E | . | . | . |
| 21-225_163E2_LC | . | . | . | . | H | . | . | . | . | . | . | . | . | . | . | . | . | . | . |
| 21-225_163F9_LC | . | . | . | . | . | . | . | . | . | . | . | . | . | . | . | . | . | . | . |
| 21-225_163G10_LC | . | . | . | . | . | . | . | . | . | . | . | . | . | . | . | . | . | . | . |
| 21-225_16A11_LC | . | . | . | . | . | . | . | . | . | . | . | . | . | . | . | . | . | . | . |
| 21-225_16H1_LC | . | . | . | . | . | . | . | . | . | . | . | . | . | . | . | . | V | . | . |
| 21-225_16H9_LC | . | . | . | . | . | L | . | . | . | . | . | . | . | . | . | . | . | . | . |
| 21-225_171A11_LC | . | . | . | . | . | . | . | . | . | . | . | . | . | . | . | . | . | . | . |
| 21-225_177B6_LC | . | . | . | . | . | . | . | . | . | . | . | . | . | . | . | . | . | . | . |
| 21-225_17E5_LC | . | . | . | . | . | . | L | . | . | . | . | . | . | . | . | V | . | . | . |
| 21-225_17F4_LC | . | . | . | . | . | . | . | . | . | . | . | . | . | . | . | . | . | . | . |
| 21-225_180H7_LC | . | . | . | . | . | . | . | . | . | . | . | . | . | . | . | . | F | . | . |
| 21-225_181A8_LC | . | . | . | . | . | . | . | . | . | . | . | . | . | . | . | . | F | . | . |
| 21-225_184D11_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | F | T | F | G | P | G | T | K | V | D | I | K | R |
| 21-225_184H10_LC | . | . | . | . | . | . | . | . | . | . | . | . | . | . | . | . | . | . | . |
| 21-225_190D6_LC | . | . | . | . | F | . | . | . | . | . | . | . | . | . | . | . | . | . | . |
| 21-225_192F6_LC | . | . | . | . | F | . | . | . | . | . | . | . | . | . | . | . | . | . | . |
| 21-225_194F10_LC | . | . | . | . | F | . | . | . | . | . | . | . | . | . | . | . | . | . | . |
| 21-225_195B9_LC | . | . | . | . | . | . | . | . | . | . | . | . | . | . | . | . | F | . | . |
| 21-225_197D7_LC | . | . | . | . | . | . | . | . | . | . | . | . | . | . | . | . | F | . | . |
| 21-225_197G10_LC | . | . | . | . | . | . | . | . | . | . | . | . | . | . | . | . | . | . | . |
| 21-225_198B9_LC | . | . | . | . | . | . | . | . | . | . | . | . | . | . | . | . | F | . | . |
| 21-225_198E3_LC | . | . | . | . | . | . | . | . | . | . | . | . | . | . | . | . | F | . | . |
| 21-225_200F6_LC | . | . | . | . | . | . | . | . | . | . | . | . | . | . | . | . | . | . | . |
| 21-225_202A3_LC | . | . | . | . | . | . | . | . | . | . | . | . | . | . | . | . | . | . | . |
| 21-225_203B9_LC | . | . | . | . | . | . | . | . | . | . | . | . | . | . | . | . | . | . | . |
| 21-225_210E4_LC | . | . | . | . | . | . | . | . | . | . | . | . | . | . | . | . | . | . | . |
| 21-225_225B6_LC | . | . | . | . | . | . | . | . | . | . | . | . | . | . | . | . | . | . | . |
| 21-225_225D6_LC | . | . | . | . | . | . | . | . | . | . | . | . | . | . | . | . | . | . | . |
| 21-225_225F8_LC | . | . | . | . | . | . | . | . | . | . | . | . | . | . | . | . | . | . | . |
| 21-225_226A9_LC | . | . | . | . | . | . | . | . | . | . | . | . | . | . | . | . | . | . | . |
| 21-225_227C12_LC | . | . | . | . | . | . | . | . | . | . | . | . | . | . | . | . | . | . | . |
| 21-225_227G3_LC | . | . | . | . | . | . | . | . | . | . | . | . | . | . | . | . | . | . | . |
| 21-225_227H5_LC | . | . | . | . | . | . | . | . | . | . | . | . | . | . | . | . | . | . | . |
| 21-225_22H3_LC | . | . | . | . | . | . | . | . | . | . | . | . | . | . | . | . | . | . | . |
| 21-225_25A9_LC | . | . | . | . | . | . | . | . | . | . | . | . | . | . | . | . | . | . | . |
| 21-225_25B6_LC | . | . | . | . | . | . | . | . | . | . | . | . | . | . | . | . | . | . | . |
| 21-225_25G5_LC | . | . | . | . | . | . | . | . | . | . | . | . | . | . | . | . | . | . | . |
| 21-225_27D5_LC | . | . | . | . | . | . | . | . | . | . | . | . | . | . | . | . | . | . | . |
| 21-225_28A9_LC | . | . | . | . | . | . | . | . | . | . | . | . | . | . | . | . | . | . | . |
| 21-225_28B8_LC | . | . | . | . | . | . | . | . | . | . | . | . | . | . | . | . | . | N | . |
| 21-225_29D7_LC | . | . | . | . | . | . | . | . | . | . | . | . | . | . | . | . | . | . | . |
| 21-225_29D8_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | F | T | F | G | P | G | T | K | V | D | I | K | R |
| 21-225_29D9_LC | . | . | . | . | . | . | . | . | . | . | . | . | . | . | . | . | F | . | . |
| 21-225_30H11_LC | . | . | . | . | H | . | . | . | . | . | . | . | . | . | . | . | . | T | . |
| 21-225_31G9_LC | . | . | . | . | . | . | . | . | . | . | . | . | . | . | . | . | . | . | . |
| 21-225_43B1_LC | . | . | . | . | F | . | . | . | . | . | . | . | . | . | . | . | . | . | . |
| 21-225_43E1_LC | . | . | . | . | . | . | . | . | . | . | . | . | . | . | . | . | . | . | . |
| 21-225_43E5_LC | . | . | . | . | F | . | . | . | . | . | . | . | . | . | . | . | . | . | . |
| 21-225_43H8_LC | . | . | . | . | F | . | . | . | . | . | . | . | . | . | . | . | . | . | . |
| 21-225_44C8_LC | . | . | . | . | . | . | . | . | . | . | . | . | . | . | . | . | . | . | . |
| 21-225_45H2_LC | . | . | . | . | F | . | . | . | . | . | . | . | . | . | . | . | . | . | . |
| 21-225_46E9_LC | . | . | . | . | F | . | . | . | . | . | . | . | . | . | . | . | . | . | . |
| 21-225_47H7_LC | . | . | . | . | F | . | . | . | . | . | . | . | . | . | . | . | . | . | . |
| 21-225_48E10_LC | . | . | . | . | . | . | . | . | . | . | . | . | . | . | . | . | . | . | . |
| 21-225_50C4_LC | . | . | . | . | . | . | . | . | . | . | . | . | . | . | . | . | . | . | . |
| 21-225_51B11_LC | . | . | . | . | . | . | . | . | . | . | . | . | . | . | . | . | . | . | . |
| 21-225_51E4_LC | . | . | . | . | . | . | . | . | . | . | . | . | . | . | . | . | . | . | . |
| 21-225_51F9_LC | . | . | . | . | . | . | . | . | . | . | . | . | . | . | . | . | . | . | . |
| 21-225_52B9_LC | . | . | . | . | . | . | . | . | . | . | . | . | . | . | . | . | . | . | . |
| 21-225_52H12_LC | . | . | . | . | . | . | . | . | . | . | . | . | . | . | . | . | . | . | . |
| 21-225_53B10_LC | . | . | . | . | . | . | . | . | . | . | . | . | . | . | . | . | . | . | . |
| 21-225_53B12_LC | . | . | . | . | . | . | . | . | . | . | . | . | . | . | . | . | . | . | . |
| 21-225_53G1_LC | . | . | . | . | . | . | . | . | . | . | . | . | . | . | . | . | . | . | . |
| 21-225_54D3_LC | . | . | . | . | . | . | . | . | . | . | . | . | . | . | . | . | . | . | . |
| 21-225_54G7_LC | . | . | . | . | . | . | . | . | . | . | . | . | . | . | . | . | . | . | . |
| 21-225_55B3_LC | . | . | . | . | . | . | . | . | . | . | . | . | . | . | . | . | . | . | . |
| 21-225_56A5_LC | . | . | . | . | . | . | . | . | . | . | . | . | . | . | . | . | . | R | . |
| 21-225_56H7_LC | . | . | . | . | . | . | . | . | . | . | . | . | . | . | . | . | S | . | . |
| 21-225_57A4_LC | . | . | . | . | . | . | . | . | . | . | . | . | . | . | . | . | . | . | . |
| 21-225_57B2_LC | . | . | . | . | . | . | . | . | . | . | . | . | . | . | . | . | . | . | . |
| 21-225_57F4_LC | . | . | . | . | F | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | F | T | F | G | P | G | T | K | V | D | I | K | R |
| 21-225_58D11_LC | . | . | . | . | F | . | . | . | . | . | . | . | . | . | . | . | . | . | . |
| 21-225_58F5_LC | . | . | . | . | . | . | . | . | . | . | . | . | . | . | . | E | . | . | . |
| 21-225_59F11_LC | . | . | . | . | . | . | . | . | . | . | . | . | . | . | . | . | F | . | . |
| 21-225_5F4_LC | . | . | . | . | . | . | . | . | . | . | . | . | . | . | . | . | . | . | . |
| 21-225_60A3_LC | . | . | . | . | . | . | . | . | . | . | . | . | . | . | . | . | . | . | . |
| 21-225_62G3_LC | . | . | . | . | . | . | . | . | . | . | . | . | . | . | . | . | . | . | . |
| 21-225_64A12_LC | . | . | . | . | . | . | . | . | . | . | . | . | . | . | . | . | . | . | . |
| 21-225_66F9_LC | . | . | . | . | . | . | . | . | . | . | . | . | . | . | . | . | . | . | . |
| 21-225_67B7_LC | . | . | . | . | . | . | . | K | . | . | . | . | . | . | . | . | . | . | . |
| 21-225_68G8_LC | . | . | . | . | . | . | . | . | . | . | . | . | . | . | . | . | . | . | . |
| 21-225_71B11_LC | . | . | . | . | . | . | . | . | . | . | . | . | . | . | . | . | V | . | . |
| 21-225_71B6_LC | . | . | . | . | . | . | . | . | . | . | . | . | . | . | . | . | . | . | . |
| 21-225_71H6_LC | . | . | . | . | . | . | . | . | . | . | . | . | . | . | . | . | . | . | . |
| 21-225_73A6_LC | . | . | . | . | . | . | . | . | . | . | . | . | . | . | . | . | . | . | . |
| 21-225_74A11_LC | . | . | . | . | . | . | . | . | . | . | . | . | . | . | . | . | . | . | . |
| 21-225_75C11_LC | . | . | . | . | . | . | . | . | . | . | . | . | . | . | . | . | . | . | . |
| 21-225_76H10_LC | . | . | . | . | . | . | . | . | . | . | . | . | . | . | . | . | . | . | . |
| 21-225_76H12_LC | . | . | . | . | . | . | . | . | . | . | . | . | . | . | . | . | . | . | . |
| 21-225_84H12_LC | . | . | . | . | . | . | . | . | . | . | . | . | . | . | . | . | . | . | . |
| 21-225_86E5_LC | . | . | . | . | . | . | . | . | . | . | . | . | . | . | . | E | . | . | . |
| 21-225_88A5_LC | . | . | . | . | . | . | . | . | . | . | . | . | . | . | . | E | . | . | . |
| 21-225_96A4_LC | . | . | . | . | . | . | . | . | . | . | R | . | . | . | . | . | . | . | . |

**FIGURE 57**

Table 101. Consensus 21 - VK1|L1/JK4 (SEQ ID NO: 50318):

DIQMTQSPSSLSASVGDRVTITCRAS--QGIS------NYLAWFQQKPGKAPKSLIYA--------ASSLQSGVPSKFSGSGSG--TDFTLTISSLQPEDFATYYCQQYST----------------------YPLTFGGGTKVEIKR wherein:

A at position 25 can be substituted with T

S at position 26 can be substituted with N

G at position 30 can be substituted with D, A, V or S

S at position 32 can be substituted with G, N, R, A or F

N at position 39 can be substituted with K, R, H, S, T or I

Y at position 40 can be substituted with H, C or D

A at position 42 can be substituted with D, V, I or N

A at position 58 can be substituted with K, S, T, V, D or G

A at position 67 an be substituted with T or V

S at position 68 can be substituted with P

S at position 69 can be substituted with N or R

Q at position 71 can be substituted with L, E or H

S at position 72 can be substituted with G, N or T

Q at position 107 can be substituted with L or H

Q at position 108 can be substituted with H, R or Y

Y at position 109 can be substituted with S, C or T

S at position 110 can be substituted with L, N, M, D, H, V, I or Y

**FIGURE 57**

T at position 111 can be substituted with N, S or K

Y at position 135 can be substituted with F, I or S

L at position 137 can be substituted with V, F or N

T at position 138 can be substituted with I, Q or S

Arg Xaa Xaa Gln Xaa Ile Xaa Xaa Xaa Leu Xaa (SEQ ID NO: 50138)

Xaa Xaa Xaa Xaa Leu Xaa Xaa (SEQ ID NO: 50139)

Xaa Xaa Xaa Xaa Xaa Xaa Pro Xaa Xaa (SEQ ID NO: 50140)

Arg Ala Ser Gln Gly Ile Ser Asn Tyr Leu Ala (SEQ ID NO: 50624)

Ala Ala Ser Ser Leu Gln Ser (SEQ ID NO: 50625)

Gln Gln Tyr Ser Thr Tyr Pro Leu Thr (SEQ ID NO: 50626)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_147E9_LC | . | . | . | . | . | . | . | . | . | . | . | . | . | . | . | . | . | . | . | . | . | . | . | . | . | . |
| 21-225_150G8_LC | . | . | . | . | . | . | . | . | . | . | . | . | . | . | . | . | . | . | . | . | . | . | . | . | . | . |
| 21-225_153F9_LC | . | . | . | . | . | . | . | . | . | . | . | . | . | . | . | . | . | . | . | . | . | . | . | . | . | . |
| 21-225_157C1_LC | . | . | . | . | . | . | . | . | . | . | . | . | . | . | I | . | . | . | I | . | L | . | . | . | . | . |
| 21-225_160H4_LC | . | . | . | . | . | . | . | . | . | . | . | . | . | . | . | . | . | . | . | . | . | . | . | . | . | . |
| 21-225_163G6_LC | . | . | . | . | . | . | . | . | . | . | . | . | . | . | . | . | . | . | . | . | . | . | . | . | . | . |
| 21-225_166H12_LC | . | . | . | . | . | . | . | . | . | . | . | . | T | . | . | . | . | . | . | . | . | . | . | . | . | . |
| 21-225_169C10_LC | . | . | . | . | . | . | . | . | . | . | . | . | . | . | . | . | . | . | . | . | . | . | . | . | . | . |
| 21-225_16A12_LC | . | . | . | . | . | . | . | . | . | . | . | . | . | . | . | . | . | . | . | . | . | . | . | . | . | . |
| 21-225_16D10_LC | . | . | . | . | . | . | . | . | . | . | . | . | . | . | . | . | . | . | . | . | . | . | . | . | . | . |
| 21-225_170D1_LC | . | . | . | . | . | . | . | . | . | . | . | A | . | . | . | . | . | . | . | . | . | . | . | . | . | . |
| 21-225_170D6_LC | . | . | . | . | . | . | . | . | . | . | . | . | . | . | . | . | . | . | . | A | . | . | . | . | . | . |
| 21-225_171H12_LC | . | . | . | . | . | . | . | . | . | . | . | . | . | . | . | . | . | . | . | . | . | . | . | . | . | . |
| 21-225_175G3_LC | . | . | . | . | . | . | . | . | . | . | . | . | . | . | . | . | . | . | . | . | . | . | . | . | . | . |
| 21-225_177A5_LC | . | . | . | . | . | . | . | . | . | . | . | . | . | . | . | . | . | . | . | . | . | . | . | . | . | . |
| 21-225_178H4_LC | . | . | . | . | . | . | . | . | . | . | . | . | . | . | . | . | . | . | . | . | . | . | . | . | . | . |
| 21-225_190C11_LC | . | . | . | . | . | . | . | . | . | . | . | . | . | . | . | . | . | . | . | . | . | . | . | . | T | . |
| 21-225_190E10_LC | . | . | . | . | A | . | . | . | . | . | . | . | . | . | . | . | . | . | . | . | . | . | . | . | T | . |
| 21-225_190E6_LC | . | . | . | . | . | . | . | . | . | . | . | . | . | . | . | . | . | . | . | . | . | . | . | . | T | . |
| 21-225_190F8_LC | . | . | . | . | . | . | . | . | . | . | . | . | . | . | . | . | . | . | . | . | . | . | . | . | T | . |
| 21-225_190G11_LC | . | . | . | . | . | . | . | . | . | . | . | . | . | . | . | . | . | . | . | . | . | . | . | . | T | . |
| 21-225_190G4_LC | . | . | . | . | . | . | . | . | . | . | . | . | . | . | . | . | . | . | . | . | . | . | . | . | . | . |
| 21-225_191A10_LC | . | . | . | . | . | . | . | . | . | . | . | . | . | . | . | . | . | . | . | . | . | . | . | . | . | . |
| 21-225_191E10_LC | . | . | . | . | . | . | . | . | . | . | . | . | . | . | . | . | . | . | . | . | . | . | . | . | . | . |
| 21-225_191G1_LC | . | . | . | . | . | . | . | . | . | . | . | . | . | . | . | . | . | . | . | . | . | . | . | . | . | . |
| 21-225_191G10_LC | . | . | . | . | . | . | . | . | . | . | . | . | . | . | . | . | . | . | . | . | . | . | . | . | T | . |
| 21-225_191G12_LC | . | . | . | . | . | . | . | . | . | . | . | . | . | . | . | . | . | . | . | . | . | . | . | . | T | . |
| 21-225_192C8_LC | . | . | . | . | . | . | . | . | . | . | . | . | . | . | . | . | . | . | . | . | . | . | . | . | . | . |
| 21-225_192D2_LC | . | . | . | . | I | . | . | . | . | . | . | . | . | . | . | . | . | . | . | . | . | . | . | . | . | . |

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_192D3_LC | . | . | . | . | . | . | . | . | . | . | . | . | . | . | . | . | . | . | . | . | . | . | . | . | . | . |
| 21-225_192G6_LC | . | . | K | . | . | . | . | . | . | . | . | . | . | . | . | . | . | . | . | . | . | . | . | . | T | . |
| 21-225_192G8_LC | . | . | . | . | . | . | . | . | . | . | . | . | . | . | . | . | . | . | . | . | . | . | . | . | T | . |
| 21-225_192H10_LC | . | . | . | . | . | . | . | . | . | . | . | . | . | . | . | . | . | . | . | . | . | . | . | . | . | . |
| 21-225_192H4_LC | . | . | . | . | . | . | . | . | . | . | . | . | . | . | . | . | . | . | . | . | . | . | . | . | . | . |
| 21-225_193A10_LC | . | . | . | . | . | . | . | . | . | . | . | . | . | . | . | . | . | . | . | . | . | . | . | . | . | . |
| 21-225_193C7_LC | . | . | . | . | . | . | . | . | . | . | . | . | . | . | . | . | . | . | . | . | . | . | . | . | . | . |
| 21-225_194C1_LC | . | . | . | . | . | . | . | . | . | . | . | . | . | . | . | . | . | . | . | . | . | . | . | . | . | . |
| 21-225_194H11_LC | . | . | . | . | . | . | . | . | . | . | . | . | . | . | . | . | . | . | . | . | . | . | . | . | . | . |
| 21-225_195A9_LC | . | . | . | . | . | . | . | . | . | . | . | . | . | . | . | . | . | . | . | . | . | . | . | . | . | . |
| 21-225_196C7_LC | . | . | . | . | . | . | . | . | . | . | . | . | . | . | . | . | . | . | . | . | . | . | . | . | . | N |
| 21-225_197B6_LC | . | . | . | . | . | . | . | . | . | . | . | . | . | . | . | . | . | . | . | . | . | . | . | . | . | . |
| 21-225_197D3_LC | . | . | . | . | . | . | . | . | . | . | . | . | . | . | . | . | . | . | . | . | . | . | . | . | . | . |
| 21-225_197F2_LC | . | . | . | . | I | . | . | . | . | . | . | . | . | . | . | . | . | . | . | . | . | . | . | . | T | . |
| 21-225_197G12_LC | . | . | . | . | I | . | . | . | . | . | . | . | . | . | . | . | . | . | . | . | . | . | . | . | T | . |
| 21-225_198B6_LC | . | . | . | . | . | . | . | . | . | . | . | . | . | . | . | . | . | . | . | . | . | . | . | . | . | . |
| 21-225_199C5_LC | . | . | . | . | . | . | . | . | . | . | . | . | . | . | . | . | . | . | . | . | . | . | . | . | . | . |
| 21-225_1F1_LC | . | . | . | . | . | . | . | . | . | . | . | . | . | . | . | . | . | . | . | . | . | . | . | . | . | . |
| 21-225_203H1_LC | . | . | . | . | . | . | . | . | . | . | . | . | . | . | . | . | . | . | . | . | . | . | . | . | . | . |
| 21-225_204G6_LC | . | . | R | . | . | . | . | . | . | . | . | . | . | . | . | . | . | . | I | . | . | . | . | . | T | . |
| 21-225_204G8_LC | . | . | . | . | . | . | . | . | . | . | . | . | . | . | . | . | . | . | . | . | . | . | . | . | . | . |
| 21-225_205F5_LC | . | . | . | . | . | . | . | . | . | . | . | . | . | . | . | . | . | . | . | . | . | . | . | . | . | . |
| 21-225_205H3_LC | . | . | P | . | . | . | . | . | . | . | . | . | . | . | . | . | . | . | . | . | . | . | . | . | . | . |
| 21-225_207G6_LC | . | . | . | . | . | . | . | . | . | . | . | . | . | . | . | . | . | . | . | . | . | . | . | . | . | . |
| 21-225_211A11_LC | . | . | . | . | . | . | . | . | . | . | . | . | . | . | . | . | . | . | . | . | . | . | . | . | . | . |
| 21-225_211A3_LC | . | . | R | . | . | . | . | . | . | . | . | . | . | . | . | . | . | . | . | . | . | . | . | . | . | . |
| 21-225_211A8_LC | . | . | E | . | . | . | . | . | P | . | . | . | . | F | . | . | . | . | . | . | . | . | . | . | . | . |
| 21-225_212D7_LC | . | . | . | . | . | . | . | . | . | . | . | . | . | . | . | . | . | . | . | . | . | . | . | . | . | . |
| 21-225_212E10_LC | . | . | . | . | . | . | . | . | . | . | . | . | . | . | . | . | . | . | . | . | . | . | . | . | . | . |

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_212E6_LC | . | . | . | . | . | . | . | . | . | . | . | . | . | . | . | . | . | . | . | . | . | . | . | . | . | . |
| 21-225_212F10_LC | . | . | . | . | . | . | . | . | . | . | . | . | . | . | . | . | . | . | . | . | . | . | . | . | . | . |
| 21-225_212H11_LC | . | . | R | . | . | . | . | . | . | . | . | . | . | . | . | . | . | . | . | . | . | . | . | . | . | . |
| 21-225_212H9_LC | . | . | E | . | . | . | . | . | . | . | . | . | . | . | . | . | . | . | . | . | . | . | . | . | . | . |
| 21-225_213B8_LC | . | . | . | . | . | . | . | . | . | . | . | . | . | . | . | . | . | . | . | . | . | . | . | . | . | . |
| 21-225_213C4_LC | . | . | H | . | . | . | . | . | . | . | . | . | . | . | . | . | . | . | . | . | . | . | . | . | . | . |
| 21-225_213D2_LC | . | . | . | . | . | . | . | . | . | . | . | . | . | . | . | . | . | . | . | . | . | . | . | . | . | . |
| 21-225_213E5_LC | . | . | R | . | . | . | . | . | . | . | . | . | . | . | . | . | . | . | . | . | . | . | . | . | . | . |
| 21-225_215A12_LC | . | . | . | . | . | . | . | . | . | . | . | . | . | . | . | . | . | . | I | . | . | . | . | . | T | . |
| 21-225_215B5_LC | . | . | . | . | . | . | . | . | . | . | . | . | . | . | . | . | . | . | . | . | . | . | . | . | . | . |
| 21-225_215D6_LC | . | . | . | . | . | . | . | . | . | . | . | . | . | . | . | . | . | . | . | . | . | . | . | . | . | . |
| 21-225_216G1_LC | . | . | E | . | . | . | . | . | . | . | . | . | . | . | . | . | . | . | . | . | . | . | . | . | . | . |
| 21-225_217B10_LC | . | . | R | . | . | . | . | . | . | . | . | . | . | . | . | . | . | . | . | . | . | . | . | . | . | . |
| 21-225_217G5_LC | . | . | L | . | . | . | . | . | . | . | . | . | . | . | . | . | . | . | . | . | . | . | . | . | . | . |
| 21-225_219H1_LC | . | . | . | . | . | . | . | . | . | . | Q | . | . | . | . | . | . | . | . | . | . | . | . | . | . | . |
| 21-225_221F6_LC | . | . | . | . | . | . | . | . | . | . | . | . | G | . | . | . | . | . | . | . | . | . | . | . | . | . |
| 21-225_222A11_LC | . | . | . | . | . | . | . | . | . | . | . | . | . | . | . | . | . | . | . | . | . | . | . | . | . | . |
| 21-225_222D10_LC | . | . | . | . | . | . | . | . | . | . | . | . | . | . | . | . | . | . | . | . | . | . | . | . | . | . |
| 21-225_223H10_LC | . | . | . | . | . | . | . | . | . | . | . | . | . | . | . | . | . | . | . | . | . | . | . | . | . | . |
| 21-225_22A2_LC | . | . | . | . | . | . | . | . | . | . | . | . | . | . | . | . | . | . | . | . | . | . | . | . | . | . |
| 21-225_23D11_LC | . | . | . | . | . | . | . | . | . | . | . | . | . | . | . | . | . | . | . | . | . | . | . | . | . | . |
| 21-225_32G3_LC | . | . | . | . | . | . | . | . | T | . | . | . | . | . | . | . | . | . | . | . | . | . | . | . | . | . |
| 21-225_55A11_LC | . | . | . | . | . | . | . | . | . | . | . | . | . | . | . | . | . | . | . | . | . | . | . | . | . | . |
| 21-225_55D4_LC | . | . | . | . | . | . | . | . | . | . | . | F | . | . | . | . | . | . | . | . | . | . | . | . | . | . |
| 21-225_65H11_LC | . | . | . | . | . | . | . | . | . | . | . | . | . | . | . | . | . | . | . | . | . | . | . | . | . | . |
| 21-225_70H6_LC | . | . | . | . | . | . | . | . | . | . | . | . | . | . | L | . | . | . | . | . | . | . | . | . | T | . |
| 21-225_74C8_LC | . | . | . | . | . | . | . | . | . | . | . | . | . | . | . | . | . | . | . | . | . | . | . | . | . | . |
| 21-225_74G6_LC | . | . | . | . | . | . | . | . | . | . | . | . | . | . | . | . | . | . | . | . | . | . | . | . | . | . |
| 21-225_76C5_LC | . | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_77E6_LC | . | . | . | . | . | . | . | . | . | . | . | . | . | . | . | . | . | . | . | . | . | . | . | . | . | . |
| 21-225_79A12_LC | . | . | . | . | . | . | . | . | . | . | . | . | . | . | . | . | . | . | . | . | . | . | . | . | . | . |
| 21-225_79F4_LC | . | . | . | . | . | . | . | . | . | . | . | . | . | . | . | . | . | . | . | . | . | . | . | . | . | . |
| 21-225_7F10_LC | . | . | . | . | . | . | . | . | . | A | . | . | . | . | . | . | . | . | . | . | . | . | . | . | . | . |
| 21-225_80C11_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | S | - | - | - | - | - | - | N | Y | L | A | W | F | Q | Q | K | P | G | K | A | P |
| 21-225_147E9_LC | . | . | . | D | . | N | . | . | . | . | . | . | . | . | . | . | . | . | . | . | . | . | . | . | . | . |
| 21-225_150G8_LC | . | . | . | . | . | . | . | . | . | . | . | . | H | . | . | . | . | . | . | . | . | . | . | . | . | . |
| 21-225_153F9_LC | . | . | . | . | . | . | . | . | . | . | . | . | . | . | . | . | . | . | . | . | . | . | . | . | . | . |
| 21-225_157C1_LC | . | . | . | . | . | . | . | . | . | . | . | . | . | . | . | . | . | . | . | . | R | . | . | . | . | . |
| 21-225_160H4_LC | . | . | . | D | . | . | . | . | . | . | . | . | . | . | . | V | . | . | . | . | . | L | . | . | . | . |
| 21-225_163G6_LC | . | . | . | D | . | . | . | . | . | . | . | . | . | . | . | V | . | . | . | . | . | . | . | . | . | . |
| 21-225_166H12_LC | . | . | . | D | . | . | . | . | . | . | . | . | H | . | . | . | . | . | . | . | . | . | . | . | . | . |
| 21-225_169C10_LC | . | . | . | D | . | . | . | . | . | . | . | . | . | . | . | . | . | . | . | . | . | . | . | . | . | . |
| 21-225_16A12_LC | . | . | . | A | . | N | . | . | . | . | . | . | T | . | . | . | . | . | . | . | . | . | . | . | . | . |
| 21-225_16D10_LC | . | . | . | D | . | . | . | . | . | . | . | . | . | . | . | . | . | . | . | . | . | . | . | . | . | . |
| 21-225_170D1_LC | . | . | . | D | . | . | . | . | . | . | . | . | . | . | . | . | . | . | . | . | . | . | . | . | . | . |
| 21-225_170D6_LC | . | . | . | D | . | G | . | . | . | . | . | . | . | C | . | . | . | . | . | . | . | . | . | . | . | . |
| 21-225_171H12_LC | . | . | . | D | . | . | . | . | . | . | . | . | . | . | . | . | . | . | . | . | . | . | . | . | . | . |
| 21-225_175G3_LC | . | . | . | D | . | . | . | . | . | . | . | . | . | D | . | . | . | . | . | . | . | . | . | . | . | . |
| 21-225_177A5_LC | . | . | . | D | . | . | . | . | . | . | . | . | . | . | . | . | . | . | . | . | . | . | . | . | . | . |
| 21-225_178H4_LC | . | . | . | D | . | . | . | . | . | . | . | . | . | . | . | . | . | . | . | . | . | . | . | . | . | . |
| 21-225_190C11_LC | . | . | . | . | . | G | . | . | . | . | . | . | . | . | . | . | . | . | . | . | . | . | . | . | . | . |
| 21-225_190E10_LC | . | . | . | . | . | G | . | . | . | . | . | . | K | . | . | . | . | . | . | . | . | . | . | . | . | . |
| 21-225_190E6_LC | . | . | . | . | . | G | . | . | . | . | . | . | . | . | . | . | . | . | . | . | . | . | . | . | . | . |
| 21-225_190F8_LC | . | . | . | . | . | G | . | . | . | . | . | . | . | . | . | . | . | . | . | . | . | . | . | . | . | . |
| 21-225_190G11_LC | . | . | . | . | . | G | . | . | . | . | . | . | . | . | . | . | . | . | . | . | . | . | . | . | . | . |
| 21-225_190G4_LC | . | . | . | D | . | G | . | . | . | . | . | . | R | . | . | . | . | . | . | . | . | . | . | . | . | . |
| 21-225_191A10_LC | . | . | . | . | . | G | . | . | . | . | . | . | K | . | . | . | . | . | . | . | . | . | . | . | . | . |
| 21-225_191E10_LC | . | . | . | . | . | G | . | . | . | . | . | . | K | . | . | . | . | . | . | . | . | . | . | . | . | . |
| 21-225_191G1_LC | . | . | . | . | . | . | . | . | . | . | . | . | . | . | . | . | . | . | . | . | . | . | . | . | . | . |
| 21-225_191G10_LC | . | . | . | . | . | G | . | . | . | . | . | . | K | . | . | . | . | . | . | . | . | . | . | . | . | . |
| 21-225_191G12_LC | . | . | . | . | . | G | . | . | . | . | . | . | . | . | . | . | . | . | . | . | . | . | . | . | . | . |
| 21-225_192C8_LC | . | . | . | . | . | . | . | . | . | . | . | . | . | . | . | . | . | . | . | . | . | . | . | . | . | . |
| 21-225_192D2_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | S | - | - | - | - | - | - | N | Y | L | A | W | F | Q | Q | K | P | G | K | A | P |
| 21-225_192D3_LC | . | . | . | . | . | . | . | . | . | . | . | . | . | . | . | . | . | . | . | . | . | . | . | . | . | . |
| 21-225_192G6_LC | . | . | . | . | . | G | . | . | . | . | . | . | . | . | . | . | . | . | . | . | . | . | . | . | . | . |
| 21-225_192G8_LC | . | . | . | . | . | G | . | . | . | . | . | . | . | . | . | . | . | . | . | . | . | . | . | . | . | . |
| 21-225_192H10_LC | . | . | . | . | . | G | . | . | . | . | . | . | K | . | . | . | . | . | . | . | . | . | . | . | . | . |
| 21-225_192H4_LC | . | . | . | D | . | . | . | . | . | . | . | . | . | . | . | . | . | . | . | . | . | . | . | . | . | . |
| 21-225_193A10_LC | . | . | . | . | . | . | . | . | . | . | . | . | . | . | . | . | . | . | . | . | . | . | . | . | . | . |
| 21-225_193C7_LC | . | . | . | . | . | G | . | . | . | . | . | . | K | . | . | . | . | . | . | . | . | . | . | . | . | . |
| 21-225_194C1_LC | . | . | . | . | . | . | . | . | . | . | . | . | . | . | . | . | . | . | . | . | . | . | . | . | . | . |
| 21-225_194H11_LC | . | . | . | . | . | . | . | . | . | . | . | . | . | . | . | . | . | . | . | . | . | . | . | . | . | . |
| 21-225_195A9_LC | . | . | . | . | . | . | . | . | . | . | . | . | . | . | . | . | . | . | . | . | . | . | . | . | . | . |
| 21-225_196C7_LC | . | . | . | A | . | . | . | . | . | . | . | . | . | . | . | . | . | . | . | . | . | . | . | . | . | . |
| 21-225_197B6_LC | . | . | . | . | . | G | . | . | . | . | . | . | K | . | . | . | . | . | . | . | . | . | . | . | . | . |
| 21-225_197D3_LC | . | . | . | . | . | R | . | . | . | . | . | . | . | . | . | . | . | . | . | . | . | . | . | . | . | . |
| 21-225_197F2_LC | . | . | . | . | . | G | . | . | . | . | . | . | . | . | . | . | . | . | . | . | . | . | . | . | . | . |
| 21-225_197G12_LC | . | . | . | . | . | G | . | . | . | . | . | . | . | . | . | . | . | . | . | . | . | . | . | . | . | . |
| 21-225_198B6_LC | . | . | . | . | . | G | . | . | . | . | . | . | . | . | . | . | . | . | . | . | . | . | . | . | . | . |
| 21-225_199C5_LC | . | . | . | . | . | . | . | . | . | . | . | . | K | . | . | . | . | . | . | . | . | . | . | . | . | . |
| 21-225_1F1_LC | . | . | . | . | . | N | . | . | . | . | . | . | . | . | . | . | . | . | . | . | . | S | . | . | . | . |
| 21-225_203H1_LC | . | . | . | . | . | G | . | . | . | . | . | . | . | . | . | . | . | . | . | . | . | . | . | . | . | . |
| 21-225_204G6_LC | . | . | . | . | . | G | . | . | . | . | . | . | . | . | . | . | . | . | . | . | . | . | . | . | . | . |
| 21-225_204G8_LC | . | . | . | . | . | . | . | . | . | . | . | . | . | H | . | . | . | . | . | . | . | . | . | . | . | . |
| 21-225_205F5_LC | . | . | . | . | . | G | . | . | . | . | . | . | . | . | . | . | . | . | . | . | . | . | . | . | . | . |
| 21-225_205H3_LC | . | . | . | A | . | . | . | . | . | . | . | . | . | H | . | . | . | . | . | L | . | . | . | . | . | . |
| 21-225_207G6_LC | . | . | . | . | . | R | . | . | . | . | . | . | . | . | . | . | . | L | . | . | . | . | . | . | . | . |
| 21-225_211A11_LC | . | . | . | . | . | G | . | . | . | . | . | . | . | . | . | . | . | . | . | . | . | . | . | . | . | . |
| 21-225_211A3_LC | . | . | . | A | . | G | . | . | . | . | . | . | K | H | . | . | . | . | . | . | R | . | . | . | . | . |
| 21-225_211A8_LC | . | . | . | . | . | . | . | . | . | . | . | . | R | . | . | . | . | V | . | . | . | . | . | . | . | . |
| 21-225_212D7_LC | . | . | . | . | . | . | . | . | . | . | . | . | . | H | . | . | . | . | . | . | . | . | . | . | . | . |
| 21-225_212E10_LC | . | . | . | . | . | . | . | . | . | . | . | . | . | H | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | S | - | - | - | - | - | - | N | Y | L | A | W | F | Q | Q | K | P | G | K | A | P |
| 21-225_212E6_LC | . | . | . | . | . | . | . | . | . | . | . | . | . | H | . | . | . | . | . | . | . | . | . | Q | . | . |
| 21-225_212F10_LC | . | . | . | . | . | . | . | . | . | . | . | . | . | . | . | D | . | . | . | . | . | . | . | . | . | . |
| 21-225_212H11_LC | . | . | . | A | . | G | . | . | . | . | . | . | K | H | . | . | . | . | . | . | R | . | . | . | . | . |
| 21-225_212H9_LC | . | . | . | . | . | . | . | . | . | . | . | . | S | . | . | . | . | L | . | . | . | . | . | . | . | . |
| 21-225_213B8_LC | . | . | . | . | . | . | . | . | . | . | . | . | . | H | . | . | . | . | . | . | . | . | . | . | . | . |
| 21-225_213C4_LC | . | . | . | A | . | R | . | . | . | . | . | . | . | . | . | . | . | C | . | . | . | . | . | . | . | . |
| 21-225_213D2_LC | . | . | . | . | . | . | . | . | . | . | . | . | . | H | . | . | . | . | . | . | . | . | . | . | . | . |
| 21-225_213E5_LC | . | . | . | A | . | G | . | . | . | . | . | . | K | H | . | . | . | . | . | . | R | . | . | . | . | . |
| 21-225_215A12_LC | . | . | . | D | . | G | . | . | . | . | . | . | . | . | . | . | . | . | . | . | . | . | . | . | . | . |
| 21-225_215B5_LC | . | . | . | . | . | . | . | . | . | . | . | . | . | H | . | . | . | . | . | . | . | . | . | . | . | . |
| 21-225_215D6_LC | . | . | . | . | . | . | . | . | . | . | . | . | . | H | . | . | . | . | . | . | . | . | . | . | . | . |
| 21-225_216G1_LC | . | . | . | . | . | . | . | . | . | . | . | . | S | . | . | . | . | L | . | . | . | . | . | . | . | . |
| 21-225_217B10_LC | . | . | . | A | . | G | . | . | . | . | . | . | K | H | . | . | . | . | . | . | R | . | . | . | . | . |
| 21-225_217G5_LC | . | . | . | . | . | G | . | . | . | . | . | . | . | . | . | . | . | . | . | . | R | . | . | . | . | . |
| 21-225_219H1_LC | . | . | . | . | . | . | . | . | . | . | . | . | . | . | . | D | . | . | . | . | . | . | . | . | . | . |
| 21-225_221F6_LC | . | . | . | . | . | . | . | . | . | . | . | . | . | . | . | . | . | . | . | . | . | . | . | . | . | . |
| 21-225_222A11_LC | . | . | . | . | . | . | . | . | . | . | . | . | . | . | . | . | . | . | . | . | . | . | . | . | . | . |
| 21-225_222D10_LC | . | . | . | . | . | . | . | . | . | . | . | . | . | . | . | . | . | . | . | . | . | . | . | . | . | . |
| 21-225_223H10_LC | . | . | . | . | . | . | . | . | . | . | . | . | . | . | . | . | . | . | . | . | . | . | . | . | . | . |
| 21-225_22A2_LC | . | . | . | D | . | . | . | . | . | . | . | . | . | . | . | . | . | V | . | . | . | . | . | . | . | . |
| 21-225_23D11_LC | . | . | . | . | . | A | . | . | . | . | . | . | . | . | . | . | . | . | . | . | R | . | . | . | . | . |
| 21-225_32G3_LC | . | . | . | A | . | . | . | . | . | . | . | . | . | . | . | . | . | . | . | . | . | . | . | . | . | . |
| 21-225_55A11_LC | . | . | . | D | . | N | . | . | . | . | . | . | I | . | . | . | . | . | . | . | . | . | . | . | . | . |
| 21-225_55D4_LC | . | . | . | D | . | . | . | . | . | . | . | . | . | . | . | I | . | . | . | . | . | . | . | . | . | . |
| 21-225_65H11_LC | . | . | . | D | . | . | . | . | . | . | . | . | T | . | . | . | . | . | . | . | . | . | . | . | . | . |
| 21-225_70H6_LC | . | . | . | S | . | F | . | . | . | . | . | . | . | . | . | N | . | . | . | R | . | . | . | . | . | . |
| 21-225_74C8_LC | . | . | . | . | . | G | . | . | . | . | . | . | K | . | . | . | . | . | . | . | . | . | . | . | . | . |
| 21-225_74G6_LC | . | . | . | . | . | G | . | . | . | . | . | . | K | . | . | . | . | . | . | . | . | . | . | . | . | . |
| 21-225_76C5_LC | . | . | . | V | . | . | . | . | . | . | . | . | K | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | S | - | - | - | - | - | - | N | Y | L | A | W | F | Q | Q | K | P | G | K | A | P |
| 21-225_77E6_LC | . | . | . | . | . | . | . | . | . | . | . | . | K | . | . | . | . | . | . | . | . | . | . | . | . | . |
| 21-225_79A12_LC | . | . | . | V | . | . | . | . | . | . | . | . | K | . | . | . | . | . | . | . | . | . | . | . | . | . |
| 21-225_79F4_LC | . | . | . | . | . | . | . | . | . | . | . | . | K | . | . | . | . | . | . | . | . | . | . | . | . | . |
| 21-225_7F10_LC | . | . | . | D | . | R | . | . | . | . | . | . | . | . | . | . | . | . | . | . | . | . | . | . | . | . |
| 21-225_80C11_LC | . | . | . | . | . | G | . | . | . | . | . | . | R | . | . | . | . | . | . | . | . | . | . | R | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | S | L | I | Y | A | - | - | - | - | - | - | - | - | A | S | S | L | Q | S | G | V | P | S | K | F |
| 21-225_147E9_LC | . | . | . | . | V | . | . | . | . | . | . | . | . | . | . | . | . | . | . | . | . | . | . | . | . | . |
| 21-225_150G8_LC | . | . | . | . | . | V | . | . | . | . | . | . | . | . | . | . | . | . | . | N | . | . | . | . | R | . |
| 21-225_153F9_LC | . | . | . | . | S | . | . | . | . | . | . | . | . | . | . | . | . | . | . | . | . | . | . | . | . | . |
| 21-225_157C1_LC | . | . | . | . | . | . | . | . | . | . | . | . | . | . | . | . | . | . | L | . | . | . | . | . | . | . |
| 21-225_160H4_LC | . | . | . | . | . | V | . | . | . | . | . | . | . | . | . | . | . | . | . | . | . | . | . | . | R | . |
| 21-225_163G6_LC | . | . | . | . | . | . | . | . | . | . | . | . | . | . | . | . | . | . | L | . | . | . | . | . | . | . |
| 21-225_166H12_LC | . | . | . | . | . | . | . | . | . | . | . | . | . | . | . | . | . | . | . | . | . | . | . | . | . | . |
| 21-225_169C10_LC | . | . | . | . | F | S | . | . | . | . | . | . | . | . | . | . | . | . | . | . | . | . | . | . | . | . |
| 21-225_16A12_LC | . | . | . | . | S | . | . | . | . | . | . | . | . | . | . | . | . | . | . | . | . | . | . | . | . | . |
| 21-225_16D10_LC | . | . | . | . | . | . | . | . | . | . | . | . | . | . | T | P | . | . | . | . | . | . | . | . | . | . |
| 21-225_170D1_LC | . | . | . | . | . | . | . | . | . | . | . | . | . | . | . | . | . | . | . | . | . | . | . | . | R | . |
| 21-225_170D6_LC | . | . | . | . | . | S | . | . | . | . | . | . | . | . | . | . | . | . | . | . | . | . | . | . | . | . |
| 21-225_171H12_LC | . | . | . | . | . | S | . | . | . | . | . | . | . | . | . | . | . | . | . | . | . | . | . | . | . | . |
| 21-225_175G3_LC | . | . | . | . | F | S | . | . | . | . | . | . | . | . | . | . | . | . | . | . | . | . | . | . | . | . |
| 21-225_177A5_LC | . | . | . | . | . | S | . | . | . | . | . | . | . | . | . | . | . | . | . | . | . | . | . | . | . | . |
| 21-225_178H4_LC | . | . | . | . | F | S | . | . | . | . | . | . | . | . | . | . | . | . | . | . | . | . | . | . | . | . |
| 21-225_190C11_LC | . | . | . | L | . | K | . | . | . | . | . | . | . | . | T | . | . | . | . | . | . | . | . | . | . | . |
| 21-225_190E10_LC | . | . | . | L | . | K | . | . | . | . | . | . | . | . | . | . | . | . | . | G | . | . | . | . | . | . |
| 21-225_190E6_LC | . | . | . | L | . | K | . | . | . | . | . | . | . | . | . | . | . | . | . | . | . | . | . | . | . | . |
| 21-225_190F8_LC | . | . | . | L | . | K | . | . | . | . | . | . | . | . | . | . | . | . | . | . | . | . | . | . | . | . |
| 21-225_190G11_LC | . | . | . | L | . | K | . | . | . | . | . | . | . | . | . | . | . | . | . | . | . | . | . | . | . | . |
| 21-225_190G4_LC | . | . | . | . | . | . | . | . | . | . | . | . | . | . | . | . | . | . | . | . | . | . | . | . | . | . |
| 21-225_191A10_LC | . | . | . | . | . | . | . | . | . | . | . | . | . | . | . | . | . | . | . | . | . | . | . | . | . | . |
| 21-225_191E10_LC | . | . | . | . | . | . | . | . | . | . | . | . | . | . | . | . | . | . | . | . | . | . | . | . | . | . |
| 21-225_191G1_LC | . | . | . | . | . | . | . | . | . | . | . | . | . | . | . | . | . | . | . | . | . | . | . | . | . | . |
| 21-225_191G10_LC | . | . | . | L | . | K | . | . | . | . | . | . | . | . | V | . | . | . | . | . | . | . | . | . | . | . |
| 21-225_191G12_LC | . | . | . | L | . | K | . | . | . | . | . | . | . | . | . | . | . | . | . | . | . | . | . | . | . | . |
| 21-225_192C8_LC | . | . | . | . | . | . | . | . | . | . | . | . | . | . | . | . | . | . | . | . | . | . | . | . | . | . |
| 21-225_192D2_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | S | L | I | Y | A | - | - | - | - | - | - | - | - | A | S | S | L | Q | S | G | V | P | S | K | F |
| 21-225_192D3_LC | . | . | . | . | . | . | . | . | . | . | . | . | . | . | . | . | . | . | . | . | . | . | . | . | . | . |
| 21-225_192G6_LC | . | . | . | L | . | K | . | . | . | . | . | . | . | . | . | . | . | . | . | . | . | . | . | . | . | . |
| 21-225_192G8_LC | . | . | . | L | . | K | . | . | . | . | . | . | . | . | . | . | . | . | . | . | . | . | . | . | . | . |
| 21-225_192H10_LC | . | . | . | . | . | . | . | . | . | . | . | . | . | . | . | . | . | . | . | . | . | . | . | . | . | . |
| 21-225_192H4_LC | . | . | . | . | S | . | . | . | . | . | . | . | . | . | . | . | . | . | . | . | . | . | . | . | . | . |
| 21-225_193A10_LC | . | . | . | . | . | . | . | . | . | . | . | . | . | . | . | . | . | . | . | . | . | . | . | . | . | . |
| 21-225_193C7_LC | . | . | . | . | . | . | . | . | . | . | . | . | . | . | . | . | . | . | . | . | . | . | . | . | . | . |
| 21-225_194C1_LC | . | . | . | . | . | . | . | . | . | . | . | . | . | . | . | . | . | . | . | . | . | . | . | . | . | . |
| 21-225_194H11_LC | . | . | . | . | . | . | . | . | . | . | . | . | . | . | . | . | . | . | . | . | . | . | . | . | . | . |
| 21-225_195A9_LC | . | . | . | . | . | . | . | . | . | . | . | . | . | . | . | . | . | . | . | . | . | . | . | . | . | . |
| 21-225_196C7_LC | . | . | . | . | . | . | . | . | . | . | . | . | . | . | . | . | . | . | . | . | . | . | . | . | . | . |
| 21-225_197B6_LC | . | . | . | . | . | G | . | . | . | . | . | . | . | . | . | . | . | . | . | . | . | . | . | . | . | . |
| 21-225_197D3_LC | . | . | . | . | . | . | . | . | . | . | . | . | . | . | . | . | . | . | . | . | . | . | . | . | . | . |
| 21-225_197F2_LC | . | . | . | L | . | K | . | . | . | . | . | . | . | . | T | . | . | . | . | . | . | . | . | . | . | . |
| 21-225_197G12_LC | . | . | . | L | . | K | . | . | . | . | . | . | . | . | T | . | . | . | . | . | . | F | . | . | . | . |
| 21-225_198B6_LC | . | . | . | . | . | . | . | . | . | . | . | . | . | . | . | . | . | . | . | . | . | . | . | . | . | . |
| 21-225_199C5_LC | . | . | . | . | . | . | . | . | . | . | . | . | . | . | . | . | . | . | . | . | . | . | . | . | . | . |
| 21-225_1F1_LC | . | . | . | . | S | V | . | . | . | . | . | . | . | . | . | . | . | . | . | T | . | . | . | . | R | . |
| 21-225_203H1_LC | . | . | . | . | . | V | . | . | . | . | . | . | . | . | . | . | R | . | . | . | . | . | . | . | . | . |
| 21-225_204G6_LC | . | . | . | . | . | . | . | . | . | . | . | . | . | . | . | . | . | . | . | . | . | . | . | . | R | . |
| 21-225_204G8_LC | . | . | . | . | F | . | . | . | . | . | . | . | . | . | . | . | . | . | . | . | . | . | . | . | Q | . |
| 21-225_205F5_LC | . | . | . | . | . | G | . | . | . | . | . | . | . | . | V | . | . | . | . | . | . | . | . | . | . | . |
| 21-225_205H3_LC | . | . | . | . | . | . | . | . | . | . | . | . | . | . | . | . | . | . | . | . | . | . | . | . | . | . |
| 21-225_207G6_LC | . | . | . | . | H | . | . | . | . | . | . | . | . | . | . | . | . | . | . | . | . | . | . | . | . | . |
| 21-225_211A11_LC | R | . | . | . | . | D | . | . | . | . | . | . | . | . | . | . | . | . | E | . | . | . | . | . | . | . |
| 21-225_211A3_LC | . | . | . | . | . | . | . | . | . | . | . | . | . | . | . | . | R | . | . | . | . | . | . | . | . | . |
| 21-225_211A8_LC | . | . | . | . | . | . | . | . | . | . | . | . | . | . | . | . | . | . | . | . | . | . | S | . | R | . |
| 21-225_212D7_LC | . | . | . | . | H | . | . | . | . | . | . | . | . | . | . | . | . | . | . | . | . | . | . | . | . | . |
| 21-225_212E10_LC | . | . | . | . | . | . | . | . | . | . | . | . | . | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | S | L | I | Y | A | - | - | - | - | - | - | - | - | A | S | S | L | Q | S | G | V | P | S | K | F |
| 21-225_212E6_LC | . | . | . | . | . | . | . | . | . | . | . | . | . | . | . | . | . | . | . | . | . | . | . | . | . | . |
| 21-225_212F10_LC | . | . | . | . | . | S | . | . | . | . | . | . | . | . | . | . | N | . | . | . | . | . | . | . | . | . |
| 21-225_212H11_LC | . | . | . | . | . | . | . | . | . | . | . | . | . | . | . | . | R | . | . | . | . | . | . | . | . | . |
| 21-225_212H9_LC | . | . | . | . | . | . | . | . | . | . | . | . | . | . | . | . | . | . | . | . | . | . | . | . | R | . |
| 21-225_213B8_LC | . | . | . | . | . | . | . | . | . | . | . | . | . | . | . | . | . | . | . | . | . | . | . | . | . | . |
| 21-225_213C4_LC | . | T | . | . | . | . | . | . | . | . | . | . | . | . | . | . | . | . | . | . | . | . | . | . | . | . |
| 21-225_213D2_LC | . | . | . | . | H | . | . | . | . | . | . | . | . | . | . | . | . | . | . | . | . | . | . | . | . | . |
| 21-225_213E5_LC | . | . | . | . | . | . | . | . | . | . | . | . | . | . | . | . | R | . | . | . | . | . | . | . | . | . |
| 21-225_215A12_LC | . | . | . | . | . | . | . | . | . | . | . | . | . | . | . | . | . | . | . | . | . | . | . | . | . | . |
| 21-225_215B5_LC | . | . | . | . | H | . | . | . | . | . | . | . | . | . | . | . | . | . | . | . | . | . | . | . | . | . |
| 21-225_215D6_LC | . | . | . | . | . | . | . | . | . | . | . | . | . | . | . | . | . | . | H | . | . | . | . | . | . | . |
| 21-225_216G1_LC | . | . | . | . | . | . | . | . | . | . | . | . | . | . | . | . | . | . | . | . | . | . | . | . | R | . |
| 21-225_217B10_LC | . | . | . | . | . | . | . | . | . | . | . | . | . | . | . | . | R | . | . | . | . | . | . | . | . | . |
| 21-225_217G5_LC | . | . | . | . | . | . | . | . | . | . | . | . | . | . | . | . | . | . | . | . | . | . | . | . | . | . |
| 21-225_219H1_LC | . | . | . | . | . | S | . | . | . | . | . | . | . | . | . | . | N | . | . | . | . | . | . | . | . | . |
| 21-225_221F6_LC | . | . | . | . | . | . | . | . | . | . | . | . | . | . | . | . | N | . | . | . | . | . | . | . | . | . |
| 21-225_222A11_LC | . | . | . | . | . | . | . | . | . | . | . | . | . | . | . | . | . | . | . | . | . | . | . | . | . | . |
| 21-225_222D10_LC | . | . | . | . | . | . | . | . | . | . | . | . | . | . | . | . | . | . | . | . | . | . | . | . | . | . |
| 21-225_223H10_LC | . | . | . | . | . | . | . | . | . | . | . | . | . | . | . | . | N | . | . | . | . | . | . | . | . | . |
| 21-225_22A2_LC | . | . | . | . | . | . | . | . | . | . | . | . | . | . | . | . | . | . | . | . | . | . | . | . | . | . |
| 21-225_23D11_LC | . | . | . | . | S | . | . | . | . | . | . | . | . | . | . | . | . | . | . | . | . | . | . | . | . | . |
| 21-225_32G3_LC | . | . | . | . | . | D | . | . | . | . | . | . | . | . | T | . | . | . | . | . | . | . | . | . | . | . |
| 21-225_55A11_LC | . | . | . | . | . | . | . | . | . | . | . | . | . | . | . | . | . | . | . | . | . | . | . | . | . | . |
| 21-225_55D4_LC | . | . | . | . | . | T | . | . | . | . | . | . | . | . | . | . | . | . | . | . | . | . | . | . | . | . |
| 21-225_65H11_LC | . | . | . | . | . | . | . | . | . | . | . | . | . | . | . | . | . | . | . | . | . | . | . | . | . | . |
| 21-225_70H6_LC | . | V | . | . | . | T | . | . | . | . | . | . | . | . | . | . | . | . | . | . | . | I | . | . | R | . |
| 21-225_74C8_LC | . | . | . | . | . | T | . | . | . | . | . | . | . | . | T | . | N | . | . | . | . | A | . | . | . | . |
| 21-225_74G6_LC | . | . | . | . | . | T | . | . | . | . | . | . | . | . | T | . | . | . | . | . | . | A | . | . | . | . |
| 21-225_76C5_LC | . | . | . | . | . | . | . | . | . | . | . | . | . | . | . | . | . | . | . | . | . | A | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | S | L | I | Y | A | - | - | - | - | - | - | - | - | A | S | S | L | Q | S | G | V | P | S | K | F |
| 21-225_77E6_LC | . | . | . | . | . | . | . | . | . | . | . | . | . | . | . | . | . | . | . | G | . | A | . | . | . | . |
| 21-225_79A12_LC | . | . | . | . | . | . | . | . | . | . | . | . | . | . | . | . | . | . | . | . | . | A | . | . | . | . |
| 21-225_79F4_LC | . | . | . | . | . | . | . | . | . | . | . | . | . | . | . | . | . | . | . | G | . | A | . | . | . | . |
| 21-225_7F10_LC | . | . | . | . | S | V | . | . | . | . | . | . | . | . | . | . | . | . | . | . | . | . | . | . | R | . |
| 21-225_80C11_LC | . | . | . | . | . | T | . | . | . | . | . | . | . | . | . | . | . | . | . | . | . | A | . | . | . | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_147E9_LC | . | . | . | . | . | . | . | . | . | . | . | . | . | . | . | . | . | . | . | . | . | . | . | . | . | . |
| 21-225_150G8_LC | . | . | . | . | . | . | . | . | . | . | . | . | . | . | . | . | . | . | . | . | . | . | . | . | . | . |
| 21-225_153F9_LC | . | . | . | . | . | . | . | . | . | . | . | . | . | . | . | . | . | . | . | . | . | . | . | . | . | . |
| 21-225_157C1_LC | . | . | . | . | . | . | . | . | . | . | . | . | . | . | . | . | . | . | . | . | . | . | . | . | . | . |
| 21-225_160H4_LC | . | . | . | . | . | . | . | . | . | . | . | . | . | . | . | . | . | . | . | . | . | . | . | . | . | . |
| 21-225_163G6_LC | . | . | . | . | . | . | . | . | . | . | . | . | . | . | . | . | . | . | . | . | . | . | . | . | . | . |
| 21-225_166H12_LC | . | . | . | . | . | . | . | . | . | . | . | . | . | . | . | . | . | . | . | . | . | . | . | . | . | . |
| 21-225_169C10_LC | . | . | . | . | . | . | . | . | . | . | . | N | . | . | . | . | . | . | . | . | . | . | . | . | . | . |
| 21-225_16A12_LC | . | . | . | . | . | . | . | . | . | E | . | . | . | . | . | . | . | . | . | . | . | . | . | . | . | . |
| 21-225_16D10_LC | . | . | . | . | . | . | . | . | . | E | . | . | . | . | . | . | . | . | . | . | . | . | . | . | . | . |
| 21-225_170D1_LC | . | . | . | . | . | . | . | . | . | . | . | . | . | . | . | . | . | . | . | . | . | . | . | . | . | . |
| 21-225_170D6_LC | . | . | . | . | . | . | . | . | . | . | . | . | . | . | . | . | . | . | . | . | . | . | . | . | . | . |
| 21-225_171H12_LC | . | . | . | . | . | . | . | . | . | . | . | . | . | . | . | . | . | . | . | . | . | . | . | . | . | . |
| 21-225_175G3_LC | . | . | . | . | . | . | . | . | . | . | . | . | . | . | . | . | . | . | . | . | . | . | . | . | . | . |
| 21-225_177A5_LC | . | . | . | . | . | . | . | . | . | . | . | . | . | . | . | . | . | . | . | . | . | . | . | . | . | . |
| 21-225_178H4_LC | . | . | . | . | . | . | . | . | . | . | . | . | . | . | . | . | N | . | . | . | . | . | . | . | . | . |
| 21-225_190C11_LC | . | . | . | . | . | . | . | . | . | . | . | . | . | . | . | . | . | . | . | . | . | . | . | . | . | . |
| 21-225_190E10_LC | . | . | . | . | . | . | . | . | . | . | . | . | . | . | . | . | . | . | . | . | . | . | . | . | . | . |
| 21-225_190E6_LC | . | . | . | . | . | . | . | . | . | . | . | . | . | . | . | . | . | . | . | . | . | . | . | . | . | . |
| 21-225_190F8_LC | . | . | . | . | . | . | . | . | . | . | . | . | . | . | . | . | . | . | . | . | . | . | . | . | . | . |
| 21-225_190G11_LC | . | . | . | . | . | . | . | . | . | . | . | . | . | . | . | . | . | . | . | . | . | . | . | . | . | . |
| 21-225_190G4_LC | . | . | . | . | . | . | . | . | . | . | . | . | . | . | . | . | . | . | . | . | . | N | . | . | . | . |
| 21-225_191A10_LC | . | . | . | . | Y | . | . | . | . | . | . | . | . | . | . | . | . | . | . | . | . | N | . | . | I | . |
| 21-225_191E10_LC | . | . | . | . | . | . | . | . | . | . | . | . | . | . | . | . | . | . | . | . | . | N | . | . | . | . |
| 21-225_191G1_LC | . | . | . | . | . | . | . | . | . | . | . | . | . | . | . | . | . | . | . | . | . | . | . | . | . | . |
| 21-225_191G10_LC | . | . | . | . | . | . | . | . | . | . | . | . | . | . | . | . | . | . | . | . | . | . | . | . | . | . |
| 21-225_191G12_LC | . | . | . | . | . | . | . | . | . | . | . | . | . | . | . | . | . | . | . | . | . | . | . | . | . | . |
| 21-225_192C8_LC | . | . | . | . | . | . | . | . | . | . | . | . | . | . | . | . | . | . | . | . | . | . | . | . | . | . |
| 21-225_192D2_LC | . | . | . | . | . | . | . | . | . | . | . | . | . | . | . | . | . | . | . | . | . | . | . | . | . | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_192D3_LC | . | . | . | . | . | . | . | . | . | . | . | . | . | . | . | . | . | . | . | . | . | . | . | . | . | . |
| 21-225_192G6_LC | . | . | . | . | . | . | . | . | . | . | . | . | . | . | . | . | . | . | . | . | . | . | . | . | . | . |
| 21-225_192G8_LC | . | . | . | . | . | . | . | . | . | . | . | . | . | . | . | . | . | . | . | . | . | . | . | . | . | . |
| 21-225_192H10_LC | . | . | . | . | Y | . | . | . | . | . | . | . | . | . | . | . | . | . | . | . | . | N | . | . | . | . |
| 21-225_192H4_LC | . | . | . | R | . | . | . | . | . | . | . | . | . | . | . | . | . | . | . | . | . | . | . | . | . | . |
| 21-225_193A10_LC | . | . | . | . | . | . | . | . | . | . | . | . | . | . | . | . | . | . | . | . | . | . | . | . | . | . |
| 21-225_193C7_LC | . | . | . | . | . | . | . | . | . | . | . | . | . | . | . | . | . | . | . | . | . | . | . | . | . | . |
| 21-225_194C1_LC | . | . | . | . | . | . | . | . | . | . | . | . | . | . | . | . | . | . | . | . | . | . | . | . | . | . |
| 21-225_194H11_LC | . | . | . | . | . | . | . | . | . | . | . | . | . | . | . | . | . | . | . | . | . | . | . | . | . | . |
| 21-225_195A9_LC | . | . | . | . | . | . | . | . | . | . | . | . | . | . | . | . | . | . | . | . | . | . | . | . | . | . |
| 21-225_196C7_LC | . | . | . | . | . | . | . | . | . | . | . | . | . | . | . | . | . | . | . | . | . | . | . | . | . | . |
| 21-225_197B6_LC | . | . | . | . | . | . | . | . | . | . | . | . | . | . | . | . | . | . | . | . | . | N | . | . | . | . |
| 21-225_197D3_LC | . | . | . | . | . | . | . | . | . | . | . | . | . | . | . | . | . | . | . | . | . | . | . | . | . | . |
| 21-225_197F2_LC | . | . | . | . | . | . | . | . | . | . | . | . | . | . | . | . | . | . | . | . | . | . | . | . | . | . |
| 21-225_197G12_LC | . | . | . | . | . | . | . | . | . | . | . | . | . | . | . | . | . | . | . | . | . | . | . | . | . | . |
| 21-225_198B6_LC | . | . | N | R | . | . | . | . | . | . | . | . | . | . | . | . | . | . | . | . | . | . | . | . | . | . |
| 21-225_199C5_LC | . | . | . | . | . | . | . | . | . | . | . | . | . | . | . | . | . | . | . | . | . | . | . | . | A | . |
| 21-225_1F1_LC | . | . | . | . | . | . | . | . | . | . | . | . | . | . | . | . | . | . | . | . | . | . | . | . | . | . |
| 21-225_203H1_LC | . | . | T | . | . | . | . | . | S | . | . | . | . | . | . | . | . | . | . | . | D | . | . | . | . | . |
| 21-225_204G6_LC | . | . | . | . | . | . | . | . | . | . | . | . | . | . | . | . | . | . | . | . | . | . | . | . | . | . |
| 21-225_204G8_LC | . | . | . | . | . | . | . | . | . | E | . | . | . | . | . | . | . | . | . | . | . | . | . | . | . | . |
| 21-225_205F5_LC | . | . | . | . | . | . | . | . | . | . | . | . | . | . | . | . | . | . | . | . | . | . | . | . | . | . |
| 21-225_205H3_LC | . | . | . | . | . | . | . | . | S | . | . | . | . | . | . | . | . | . | . | . | . | . | . | . | . | . |
| 21-225_207G6_LC | . | . | N | R | . | . | . | . | . | . | . | . | . | . | . | . | . | . | . | . | . | . | . | . | . | . |
| 21-225_211A11_LC | . | . | . | R | . | . | . | . | . | . | . | . | . | . | . | G | . | . | . | . | . | . | . | . | . | . |
| 21-225_211A3_LC | . | . | . | . | . | . | . | . | . | . | . | . | . | . | . | . | . | . | . | . | . | . | L | . | . | . |
| 21-225_211A8_LC | . | . | . | . | . | . | . | . | . | . | . | . | . | . | . | . | . | . | . | . | . | . | . | . | . | . |
| 21-225_212D7_LC | . | . | N | R | . | . | . | . | . | . | . | . | . | . | . | N | N | . | . | . | . | . | . | V | . | . |
| 21-225_212E10_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_212E6_LC | . | . | . | R | . | . | . | . | . | . | . | . | . | . | . | . | . | . | . | . | . | . | . | . | . | . |
| 21-225_212F10_LC | . | . | . | R | . | . | . | . | . | . | . | . | . | . | . | . | . | . | . | . | . | . | . | . | . | . |
| 21-225_212H11_LC | . | . | . | . | . | . | . | . | . | . | . | . | . | . | . | . | . | . | . | . | . | . | L | . | . | . |
| 21-225_212H9_LC | . | . | . | . | . | . | . | . | . | . | . | . | . | . | . | . | . | . | . | . | . | . | . | . | . | . |
| 21-225_213B8_LC | . | . | . | . | . | . | . | . | . | . | . | . | . | . | . | . | . | . | . | . | . | . | . | . | . | . |
| 21-225_213C4_LC | . | . | . | . | . | . | . | . | . | . | . | . | . | . | . | . | . | . | . | . | . | . | . | . | . | . |
| 21-225_213D2_LC | . | . | N | R | . | . | . | . | . | . | . | . | . | . | . | N | N | . | . | . | . | . | . | V | . | . |
| 21-225_213E5_LC | . | . | . | . | . | . | . | . | . | . | . | . | . | . | . | . | . | . | . | . | . | . | L | . | . | . |
| 21-225_215A12_LC | . | . | . | R | . | . | . | . | . | . | . | . | . | . | . | . | . | . | . | S | . | . | . | . | . | . |
| 21-225_215B5_LC | . | . | N | R | . | . | . | . | . | . | . | . | . | . | . | N | N | . | . | . | . | . | . | V | . | . |
| 21-225_215D6_LC | . | . | . | R | . | . | . | . | . | . | . | . | . | . | . | . | . | . | . | . | . | . | . | . | . | . |
| 21-225_216G1_LC | . | . | . | . | . | . | . | . | . | . | . | . | . | . | . | . | . | . | . | . | . | . | . | . | . | . |
| 21-225_217B10_LC | . | . | . | . | . | . | . | . | . | . | . | . | . | . | . | . | . | V | . | . | . | . | L | . | . | . |
| 21-225_217G5_LC | . | . | T | R | . | . | . | . | . | . | . | . | . | . | . | . | . | . | . | . | D | . | . | . | . | . |
| 21-225_219H1_LC | . | . | . | R | . | . | . | . | . | . | . | . | . | . | . | . | . | . | . | . | . | . | . | . | . | . |
| 21-225_221F6_LC | . | . | . | R | . | . | . | . | . | . | . | . | . | . | . | . | . | . | . | . | . | . | . | . | . | . |
| 21-225_222A11_LC | . | . | . | . | . | . | . | . | . | . | . | . | . | . | . | . | . | . | . | . | . | . | . | . | . | . |
| 21-225_222D10_LC | . | . | . | . | . | . | . | . | . | . | . | . | . | . | . | . | . | . | . | . | . | . | . | . | . | . |
| 21-225_223H10_LC | . | . | . | . | . | . | . | . | . | . | . | . | . | . | . | . | . | . | . | . | . | . | . | . | . | . |
| 21-225_22A2_LC | . | . | . | . | . | . | . | . | . | . | . | . | . | . | . | . | . | . | . | . | . | . | . | . | S | . |
| 21-225_23D11_LC | . | . | . | . | . | . | . | . | . | E | . | . | . | . | . | . | . | . | . | . | . | . | . | . | . | . |
| 21-225_32G3_LC | . | . | . | . | . | . | . | . | . | . | . | . | . | . | . | . | T | . | . | . | . | . | . | . | . | . |
| 21-225_55A11_LC | . | . | . | . | . | . | . | . | . | . | . | . | . | . | . | . | . | . | . | . | . | . | . | . | . | . |
| 21-225_55D4_LC | . | . | . | . | F | . | . | . | . | . | . | . | . | . | . | . | N | . | . | . | . | . | . | . | . | . |
| 21-225_65H11_LC | . | . | . | . | . | . | . | . | . | . | . | . | . | . | . | . | . | . | . | . | . | . | . | . | . | . |
| 21-225_70H6_LC | . | . | . | . | . | . | . | . | . | . | . | . | . | . | . | . | . | . | . | . | . | . | S | . | . | . |
| 21-225_74C8_LC | . | . | . | . | . | . | . | . | . | . | . | . | . | . | . | . | . | . | . | Y | . | . | . | . | . | . |
| 21-225_74G6_LC | . | . | . | . | . | . | . | . | . | . | . | . | . | . | . | . | . | . | . | Y | . | . | . | . | . | . |
| 21-225_76C5_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_77E6_LC | . | . | . | . | . | . | . | . | . | . | . | . | . | . | . | . | . | . | . | . | . | . | . | . | . | . |
| 21-225_79A12_LC | . | . | . | . | . | . | . | . | . | . | . | . | . | . | . | . | . | . | . | . | . | . | . | . | . | . |
| 21-225_79F4_LC | . | . | . | . | . | . | . | . | . | . | . | . | . | . | . | . | . | . | . | . | . | . | . | . | . | . |
| 21-225_7F10_LC | . | . | . | . | . | . | . | . | . | . | . | . | . | . | . | . | . | . | . | . | . | . | . | . | . | . |
| 21-225_80C11_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | S | T | - | - | - | - | - | - | - | - | - | - | - | - | - | - | - | - | - | - | - |
| 21-225_147E9_LC | . | . | . | . | . | N | S | . | . | . | . | . | . | . | . | . | . | . | . | . | . | . | . | . | . | . |
| 21-225_150G8_LC | . | . | . | . | . | N | N | . | . | . | . | . | . | . | . | . | . | . | . | . | . | . | . | . | . | . |
| 21-225_153F9_LC | . | . | . | . | . | N | S | . | . | . | . | . | . | . | . | . | . | . | . | . | . | . | . | . | . | . |
| 21-225_157C1_LC | . | . | . | . | . | N | S | . | . | . | . | . | . | . | . | . | . | . | . | . | . | . | . | . | . | . |
| 21-225_160H4_LC | . | . | . | . | . | N | S | . | . | . | . | . | . | . | . | . | . | . | . | . | . | . | . | . | . | . |
| 21-225_163G6_LC | . | . | . | . | . | H | S | . | . | . | . | . | . | . | . | . | . | . | . | . | . | . | . | . | . | . |
| 21-225_166H12_LC | . | . | . | . | . | N | S | . | . | . | . | . | . | . | . | . | . | . | . | . | . | . | . | . | . | . |
| 21-225_169C10_LC | . | . | . | . | S | D | S | . | . | . | . | . | . | . | . | . | . | . | . | . | . | . | . | . | . | . |
| 21-225_16A12_LC | . | . | . | . | . | Y | S | . | . | . | . | . | . | . | . | . | . | . | . | . | . | . | . | . | . | . |
| 21-225_16D10_LC | . | . | . | . | . | N | S | . | . | . | . | . | . | . | . | . | . | . | . | . | . | . | . | . | . | . |
| 21-225_170D1_LC | . | . | . | . | C | D | S | . | . | . | . | . | . | . | . | . | . | . | . | . | . | . | . | . | . | . |
| 21-225_170D6_LC | . | . | . | . | S | D | S | . | . | . | . | . | . | . | . | . | . | . | . | . | . | . | . | . | . | . |
| 21-225_171H12_LC | . | . | . | . | S | D | S | . | . | . | . | . | . | . | . | . | . | . | . | . | . | . | . | . | . | . |
| 21-225_175G3_LC | . | . | . | . | S | D | S | . | . | . | . | . | . | . | . | . | . | . | . | . | . | . | . | . | . | . |
| 21-225_177A5_LC | . | . | . | . | S | D | S | . | . | . | . | . | . | . | . | . | . | . | . | . | . | . | . | . | . | . |
| 21-225_178H4_LC | . | . | . | . | S | N | S | . | . | . | . | . | . | . | . | . | . | . | . | . | . | . | . | . | . | . |
| 21-225_190C11_LC | . | . | . | . | . | M | . | . | . | . | . | . | . | . | . | . | . | . | . | . | . | . | . | . | . | . |
| 21-225_190E10_LC | . | . | . | . | . | M | . | . | . | . | . | . | . | . | . | . | . | . | . | . | . | . | . | . | . | . |
| 21-225_190E6_LC | . | . | . | . | . | M | . | . | . | . | . | . | . | . | . | . | . | . | . | . | . | . | . | . | . | . |
| 21-225_190F8_LC | . | . | . | . | . | M | . | . | . | . | . | . | . | . | . | . | . | . | . | . | . | . | . | . | . | . |
| 21-225_190G11_LC | . | . | . | . | . | M | . | . | . | . | . | . | . | . | . | . | . | . | . | . | . | . | . | . | . | . |
| 21-225_190G4_LC | . | . | . | . | . | . | . | . | . | . | . | . | . | . | . | . | . | . | . | . | . | . | . | . | . | . |
| 21-225_191A10_LC | . | . | . | . | . | . | . | . | . | . | . | . | . | . | . | . | . | . | . | . | . | . | . | . | . | . |
| 21-225_191E10_LC | . | . | . | . | . | . | . | . | . | . | . | . | . | . | . | . | . | . | . | . | . | . | . | . | . | . |
| 21-225_191G1_LC | . | . | . | H | . | L | . | . | . | . | . | . | . | . | . | . | . | . | . | . | . | . | . | . | . | . |
| 21-225_191G10_LC | . | . | . | . | . | M | . | . | . | . | . | . | . | . | . | . | . | . | . | . | . | . | . | . | . | . |
| 21-225_191G12_LC | . | . | . | . | . | I | . | . | . | . | . | . | . | . | . | . | . | . | . | . | . | . | . | . | . | . |
| 21-225_192C8_LC | . | . | . | H | . | L | . | . | . | . | . | . | . | . | . | . | . | . | . | . | . | . | . | . | . | . |
| 21-225_192D2_LC | . | . | . | H | . | V | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | S | T | - | - | - | - | - | - | - | - | - | - | - | - | - | - | - | - | - | - | - |
| 21-225_192D3_LC | . | . | L | H | . | L | . | . | . | . | . | . | . | . | . | . | . | . | . | . | . | . | . | . | . | . |
| 21-225_192G6_LC | . | . | . | . | . | M | . | . | . | . | . | . | . | . | . | . | . | . | . | . | . | . | . | . | . | . |
| 21-225_192G8_LC | . | . | . | . | . | I | . | . | . | . | . | . | . | . | . | . | . | . | . | . | . | . | . | . | . | . |
| 21-225_192H10_LC | . | . | . | . | . | . | . | . | . | . | . | . | . | . | . | . | . | . | . | . | . | . | . | . | . | . |
| 21-225_192H4_LC | . | G | L | H | . | L | N | . | . | . | . | . | . | . | . | . | . | . | . | . | . | . | . | . | . | . |
| 21-225_193A10_LC | F | . | . | H | . | L | . | . | . | . | . | . | . | . | . | . | . | . | . | . | . | . | . | . | . | . |
| 21-225_193C7_LC | . | . | . | . | . | . | . | . | . | . | . | . | . | . | . | . | . | . | . | . | . | . | . | . | . | . |
| 21-225_194C1_LC | . | . | L | H | . | L | . | . | . | . | . | . | . | . | . | . | . | . | . | . | . | . | . | . | . | . |
| 21-225_194H11_LC | . | . | . | H | . | L | . | . | . | . | . | . | . | . | . | . | . | . | . | . | . | . | . | . | . | . |
| 21-225_195A9_LC | . | . | . | H | . | L | . | . | . | . | . | . | . | . | . | . | . | . | . | . | . | . | . | . | . | . |
| 21-225_196C7_LC | . | . | . | H | . | L | . | . | . | . | . | . | . | . | . | . | . | . | . | . | . | . | . | . | . | . |
| 21-225_197B6_LC | . | . | . | . | . | . | . | . | . | . | . | . | . | . | . | . | . | . | . | . | . | . | . | . | . | . |
| 21-225_197D3_LC | . | . | . | H | . | L | . | . | . | . | . | . | . | . | . | . | . | . | . | . | . | . | . | . | . | . |
| 21-225_197F2_LC | . | F | . | . | . | I | . | . | . | . | . | . | . | . | . | . | . | . | . | . | . | . | . | . | . | . |
| 21-225_197G12_LC | . | F | . | . | . | M | . | . | . | . | . | . | . | . | . | . | . | . | . | . | . | . | . | . | . | . |
| 21-225_198B6_LC | . | . | . | . | . | . | . | . | . | . | . | . | . | . | . | . | . | . | . | . | . | . | . | . | . | . |
| 21-225_199C5_LC | . | . | . | . | . | L | . | . | . | . | . | . | . | . | . | . | . | . | . | . | . | . | . | . | . | . |
| 21-225_1F1_LC | . | . | . | . | . | N | S | . | . | . | . | . | . | . | . | . | . | . | . | . | . | . | . | . | . | . |
| 21-225_203H1_LC | . | . | . | R | . | H | . | . | . | . | . | . | . | . | . | . | . | . | . | . | . | . | . | . | . | . |
| 21-225_204G6_LC | . | . | . | H | . | L | S | . | . | . | . | . | . | . | . | . | . | . | . | . | . | . | . | . | . | . |
| 21-225_204G8_LC | . | . | . | . | . | . | N | . | . | . | . | . | . | . | . | . | . | . | . | . | . | . | . | . | . | . |
| 21-225_205F5_LC | T | . | . | . | . | . | N | . | . | . | . | . | . | . | . | . | . | . | . | . | . | . | . | . | . | . |
| 21-225_205H3_LC | . | . | . | . | . | . | N | . | . | . | . | . | . | . | . | . | . | . | . | . | . | . | . | . | . | . |
| 21-225_207G6_LC | . | . | . | . | . | . | N | . | . | . | . | . | . | . | . | . | . | . | . | . | . | . | . | . | . | . |
| 21-225_211A11_LC | . | . | . | H | . | M | . | . | . | . | . | . | . | . | . | . | . | . | . | . | . | . | . | . | . | . |
| 21-225_211A3_LC | . | . | . | . | . | . | N | . | . | . | . | . | . | . | . | . | . | . | . | . | . | . | . | . | . | . |
| 21-225_211A8_LC | . | . | L | R | . | D | . | . | . | . | . | . | . | . | . | . | . | . | . | . | . | . | . | . | . | . |
| 21-225_212D7_LC | . | . | . | . | . | . | N | . | . | . | . | . | . | . | . | . | . | . | . | . | . | . | . | . | . | . |
| 21-225_212E10_LC | . | . | . | . | . | . | N | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | S | T | - | - | - | - | - | - | - | - | - | - | - | - | - | - | - | - | - | - | - |
| 21-225_212E6_LC | . | . | . | . | . | V | . | . | . | . | . | . | . | . | . | . | . | . | . | . | . | . | . | . | . | . |
| 21-225_212F10_LC | . | . | . | H | . | . | N | . | . | . | . | . | . | . | . | . | . | . | . | . | . | . | . | . | . | . |
| 21-225_212H11_LC | . | . | . | H | . | . | N | . | . | . | . | . | . | . | . | . | . | . | . | . | . | . | . | . | . | . |
| 21-225_212H9_LC | . | . | L | R | . | D | . | . | . | . | . | . | . | . | . | . | . | . | . | . | . | . | . | . | . | . |
| 21-225_213B8_LC | . | . | . | . | . | . | N | . | . | . | . | . | . | . | . | . | . | . | . | . | . | . | . | . | . | . |
| 21-225_213C4_LC | . | . | . | . | . | . | N | . | . | . | . | . | . | . | . | . | . | . | . | . | . | . | . | . | . | . |
| 21-225_213D2_LC | . | . | H | . | . | . | N | . | . | . | . | . | . | . | . | . | . | . | . | . | . | . | . | . | . | . |
| 21-225_213E5_LC | . | . | . | H | . | . | N | . | . | . | . | . | . | . | . | . | . | . | . | . | . | . | . | . | . | . |
| 21-225_215A12_LC | . | . | . | . | . | V | . | . | . | . | . | . | . | . | . | . | . | . | . | . | . | . | . | . | . | . |
| 21-225_215B5_LC | . | . | . | . | . | I | N | . | . | . | . | . | . | . | . | . | . | . | . | . | . | . | . | . | . | . |
| 21-225_215D6_LC | . | . | . | . | . | V | . | . | . | . | . | . | . | . | . | . | . | . | . | . | . | . | . | . | . | . |
| 21-225_216G1_LC | . | . | L | R | . | D | . | . | . | . | . | . | . | . | . | . | . | . | . | . | . | . | . | . | . | . |
| 21-225_217B10_LC | . | R | . | H | T | . | K | . | . | . | . | . | . | . | . | . | . | . | . | . | . | . | . | . | . | . |
| 21-225_217G5_LC | . | . | . | . | . | V | N | . | . | . | . | . | . | . | . | . | . | . | . | . | . | . | . | . | . | . |
| 21-225_219H1_LC | . | . | . | H | . | . | N | . | . | . | . | . | . | . | . | . | . | . | . | . | . | . | . | . | . | . |
| 21-225_221F6_LC | . | . | . | . | . | M | . | . | . | . | . | . | . | . | . | . | . | . | . | . | . | . | . | . | . | . |
| 21-225_222A11_LC | . | . | L | Y | . | L | N | . | . | . | . | . | . | . | . | . | . | . | . | . | . | . | . | . | . | . |
| 21-225_222D10_LC | . | . | L | H | . | L | N | . | . | . | . | . | . | . | . | . | . | . | . | . | . | . | . | . | . | . |
| 21-225_223H10_LC | . | . | L | H | . | L | N | . | . | . | . | . | . | . | . | . | . | . | . | . | . | . | . | . | . | . |
| 21-225_22A2_LC | . | . | . | . | . | N | S | . | . | . | . | . | . | . | . | . | . | . | . | . | . | . | . | . | . | . |
| 21-225_23D11_LC | . | . | . | . | . | H | S | . | . | . | . | . | . | . | . | . | . | . | . | . | . | . | . | . | . | . |
| 21-225_32G3_LC | . | . | . | . | . | H | S | . | . | . | . | . | . | . | . | . | . | . | . | . | . | . | . | . | . | . |
| 21-225_55A11_LC | . | . | . | . | . | N | S | . | . | . | . | . | . | . | . | . | . | . | . | . | . | . | . | . | . | . |
| 21-225_55D4_LC | . | . | . | . | . | H | S | . | . | . | . | . | . | . | . | . | . | . | . | . | . | . | . | . | . | . |
| 21-225_65H11_LC | . | . | . | . | . | N | S | . | . | . | . | . | . | . | . | . | . | . | . | . | . | . | . | . | . | . |
| 21-225_70H6_LC | . | . | . | . | S | Y | S | . | . | . | . | . | . | . | . | . | . | . | . | . | . | . | . | . | . | . |
| 21-225_74C8_LC | . | . | . | . | . | . | N | . | . | . | . | . | . | . | . | . | . | . | . | . | . | . | . | . | . | . |
| 21-225_74G6_LC | . | . | . | . | . | . | N | . | . | . | . | . | . | . | . | . | . | . | . | . | . | . | . | . | . | . |
| 21-225_76C5_LC | . | . | . | . | . | . | N | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | S | T | - | - | - | - | - | - | - | - | - | - | - | - | - | - | - | - | - | - | - |
| 21-225_77E6_LC | . | . | . | . | . | . | N | . | . | . | . | . | . | . | . | . | . | . | . | . | . | . | . | . | . | . |
| 21-225_79A12_LC | . | . | . | . | . | . | N | . | . | . | . | . | . | . | . | . | . | . | . | . | . | . | . | . | . | . |
| 21-225_79F4_LC | . | . | . | . | . | . | N | . | . | . | . | . | . | . | . | . | . | . | . | . | . | . | . | . | . | . |
| 21-225_7F10_LC | . | . | . | . | . | N | S | . | . | . | . | . | . | . | . | . | . | . | . | . | . | . | . | . | . | . |
| 21-225_80C11_LC | . | . | . | . | . | . | N | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | L | T | F | G | G | G | T | K | V | E | I | K | R |
| 21-225_147E9_LC | . | . | . | . | . | . | . | . | . | . | . | . | . | . | . | . | . | . | . |
| 21-225_150G8_LC | . | . | . | . | . | . | . | . | . | . | . | . | . | . | . | . | . | . | . |
| 21-225_153F9_LC | . | . | . | . | . | . | . | S | . | . | . | . | . | . | . | . | . | . | . |
| 21-225_157C1_LC | . | . | . | . | F | . | F | . | . | . | . | . | . | . | . | . | L | . | . |
| 21-225_160H4_LC | . | . | . | . | . | . | . | . | . | . | . | . | . | . | . | . | . | . | . |
| 21-225_163G6_LC | . | . | . | . | . | . | . | . | . | . | . | . | . | . | . | A | . | . | . |
| 21-225_166H12_LC | . | . | . | . | F | . | . | . | . | . | . | . | . | . | . | . | . | T | . |
| 21-225_169C10_LC | . | . | . | . | . | . | . | . | . | . | . | . | . | . | . | . | . | . | . |
| 21-225_16A12_LC | . | . | . | . | . | . | . | . | . | . | . | . | . | . | . | . | . | . | . |
| 21-225_16D10_LC | . | . | . | . | . | . | . | . | . | . | . | . | . | . | . | . | . | . | . |
| 21-225_170D1_LC | . | . | . | . | F | . | . | . | . | . | . | . | . | . | . | . | . | . | . |
| 21-225_170D6_LC | . | . | . | . | . | . | . | . | . | . | . | . | . | . | . | . | . | . | . |
| 21-225_171H12_LC | . | . | . | . | . | . | . | . | . | . | . | . | . | . | . | . | . | . | . |
| 21-225_175G3_LC | . | . | . | . | . | . | . | . | . | . | . | . | . | . | . | . | . | . | . |
| 21-225_177A5_LC | . | . | . | . | . | . | . | . | . | . | . | . | . | . | . | . | . | . | . |
| 21-225_178H4_LC | . | . | . | . | . | . | . | . | . | . | . | . | . | . | . | . | . | . | . |
| 21-225_190C11_LC | . | . | . | . | . | . | . | . | . | . | . | . | . | . | . | . | . | . | . |
| 21-225_190E10_LC | . | . | . | . | . | . | . | . | . | . | . | . | . | . | . | . | . | . | . |
| 21-225_190E6_LC | . | . | . | . | . | . | . | . | . | . | . | . | . | . | . | . | . | . | . |
| 21-225_190F8_LC | . | . | . | . | . | . | . | . | . | . | . | . | . | . | . | . | . | . | . |
| 21-225_190G11_LC | . | . | . | . | . | . | . | . | . | . | . | . | . | . | . | . | . | . | . |
| 21-225_190G4_LC | . | . | . | . | . | . | . | . | . | . | . | . | . | . | . | . | . | . | . |
| 21-225_191A10_LC | . | . | . | . | . | . | . | . | . | . | . | . | . | . | . | . | . | . | . |
| 21-225_191E10_LC | . | . | . | . | . | . | . | . | . | . | . | . | . | . | . | . | . | . | . |
| 21-225_191G1_LC | . | . | . | . | . | . | . | . | . | . | . | . | . | . | . | . | . | . | . |
| 21-225_191G10_LC | . | . | . | . | . | . | . | . | . | . | . | . | . | . | . | . | . | . | . |
| 21-225_191G12_LC | . | . | . | . | . | . | . | . | . | . | . | . | S | . | . | . | . | . | . |
| 21-225_192C8_LC | . | . | . | . | . | . | . | . | . | . | . | . | . | . | . | . | . | . | . |
| 21-225_192D2_LC | . | . | . | . | . | . | N | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | L | T | F | G | G | G | T | K | V | E | I | K | R |
| 21-225_192D3_LC | . | . | . | . | . | . | . | . | . | . | . | . | . | . | . | . | . | . | . |
| 21-225_192G6_LC | . | . | . | . | . | . | . | . | . | . | . | . | . | . | . | . | . | . | . |
| 21-225_192G8_LC | . | . | . | . | . | . | . | . | . | . | . | . | . | . | . | . | . | . | . |
| 21-225_192H10_LC | . | . | . | . | . | . | . | . | . | . | . | . | . | . | . | . | . | . | . |
| 21-225_192H4_LC | . | . | . | . | . | . | . | . | . | . | . | . | . | R | . | . | . | R | . |
| 21-225_193A10_LC | . | . | . | . | . | . | . | . | . | . | . | . | . | . | . | . | . | . | . |
| 21-225_193C7_LC | . | . | . | . | . | . | . | . | . | . | . | . | . | . | . | . | . | . | . |
| 21-225_194C1_LC | . | . | . | . | . | . | . | . | . | . | . | . | . | . | . | . | . | . | . |
| 21-225_194H11_LC | . | . | . | . | . | . | . | . | . | . | . | . | . | . | . | . | . | . | . |
| 21-225_195A9_LC | . | . | . | . | . | . | . | . | . | . | . | . | . | . | . | . | . | . | . |
| 21-225_196C7_LC | . | . | . | . | . | . | . | . | . | . | . | . | . | . | . | . | . | . | . |
| 21-225_197B6_LC | . | . | . | . | . | . | . | . | . | . | . | . | . | . | . | . | . | . | . |
| 21-225_197D3_LC | . | . | . | . | . | . | . | . | . | . | . | . | . | . | . | . | . | . | . |
| 21-225_197F2_LC | . | . | . | . | . | . | . | . | . | . | . | . | . | . | . | . | . | . | . |
| 21-225_197G12_LC | . | . | . | . | . | . | . | . | . | . | . | . | . | . | . | . | . | . | . |
| 21-225_198B6_LC | . | . | . | . | . | . | . | . | . | . | . | . | . | . | . | . | . | . | . |
| 21-225_199C5_LC | . | . | . | . | . | . | . | . | . | . | . | . | . | . | . | . | . | . | . |
| 21-225_1F1_LC | . | . | . | . | . | . | . | . | . | . | . | . | . | . | . | . | . | . | . |
| 21-225_203H1_LC | . | . | . | . | . | . | . | . | . | . | . | . | . | . | . | . | . | . | . |
| 21-225_204G6_LC | . | . | . | . | . | . | . | . | . | . | . | . | . | . | . | . | . | . | . |
| 21-225_204G8_LC | . | . | . | . | . | . | V | . | . | . | . | . | . | . | . | . | . | . | . |
| 21-225_205F5_LC | . | . | . | . | . | . | . | . | . | . | . | . | . | . | . | D | . | R | . |
| 21-225_205H3_LC | . | . | . | . | . | . | . | . | . | . | . | . | . | . | . | . | . | . | . |
| 21-225_207G6_LC | . | . | . | . | . | . | . | . | . | . | . | . | . | . | . | . | . | . | . |
| 21-225_211A11_LC | . | . | . | . | . | . | . | . | . | . | A | . | . | . | . | . | . | . | . |
| 21-225_211A3_LC | . | . | . | . | . | . | . | . | . | . | . | . | . | . | . | . | . | . | . |
| 21-225_211A8_LC | . | . | . | . | . | . | . | I | . | . | . | . | . | . | . | . | . | . | . |
| 21-225_212D7_LC | . | . | . | . | . | . | . | . | . | . | . | . | . | . | . | . | . | . | . |
| 21-225_212E10_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | L | T | F | G | G | G | T | K | V | E | I | K | R |
| 21-225_212E6_LC | . | . | . | . | . | . | . | . | . | . | . | . | . | . | . | . | . | . | . |
| 21-225_212F10_LC | . | . | . | . | . | . | . | . | . | . | . | . | . | . | . | . | . | . | . |
| 21-225_212H11_LC | . | . | . | . | . | . | . | . | . | V | . | . | . | . | . | . | . | T | G |
| 21-225_212H9_LC | . | . | . | . | . | . | . | . | . | . | . | . | . | . | . | . | . | . | . |
| 21-225_213B8_LC | . | . | . | . | . | . | . | . | . | . | . | . | . | . | . | . | . | . | . |
| 21-225_213C4_LC | . | . | . | . | . | . | . | . | . | . | . | . | . | . | . | . | . | . | . |
| 21-225_213D2_LC | . | . | . | . | . | . | . | . | . | . | . | . | . | . | . | . | . | . | . |
| 21-225_213E5_LC | . | . | . | . | . | . | . | . | . | . | . | . | . | . | . | . | . | . | . |
| 21-225_215A12_LC | . | . | . | . | . | . | . | . | . | . | . | . | . | . | . | . | . | . | . |
| 21-225_215B5_LC | . | . | . | . | . | . | . | . | . | . | . | . | . | . | . | . | . | . | . |
| 21-225_215D6_LC | . | . | . | . | . | . | . | . | . | . | . | . | . | . | . | . | . | . | . |
| 21-225_216G1_LC | . | . | . | . | . | . | . | . | . | . | . | . | . | . | . | . | . | . | . |
| 21-225_217B10_LC | . | . | . | . | S | . | V | Q | L | V | . | . | . | . | . | . | . | T | G |
| 21-225_217G5_LC | . | . | . | . | . | . | . | . | . | . | . | . | . | . | . | . | . | N | . |
| 21-225_219H1_LC | . | . | . | . | . | . | . | . | . | . | . | . | . | . | . | . | . | . | . |
| 21-225_221F6_LC | . | . | . | . | . | . | . | . | . | . | . | . | . | R | . | . | . | . | . |
| 21-225_222A11_LC | . | . | . | . | . | . | . | . | . | . | . | . | . | . | . | . | . | . | . |
| 21-225_222D10_LC | . | . | . | . | . | . | . | . | . | . | . | . | . | R | . | . | . | . | . |
| 21-225_223H10_LC | . | . | . | . | . | . | . | . | . | . | . | . | . | . | . | . | . | . | . |
| 21-225_22A2_LC | . | . | . | . | F | . | . | . | . | . | . | . | . | . | . | . | . | . | . |
| 21-225_23D11_LC | . | . | . | . | . | . | . | . | . | . | . | . | S | . | . | . | . | . | . |
| 21-225_32G3_LC | . | . | . | . | . | . | . | . | . | . | . | . | . | . | . | . | . | . | . |
| 21-225_55A11_LC | . | . | . | . | . | . | . | . | . | . | . | . | . | . | . | . | . | . | . |
| 21-225_55D4_LC | . | . | . | . | F | . | . | . | . | . | . | . | . | R | . | . | . | R | . |
| 21-225_65H11_LC | . | . | . | . | F | . | . | . | . | . | . | . | . | . | . | . | . | . | . |
| 21-225_70H6_LC | . | . | . | . | I | . | . | . | . | . | . | . | . | . | . | . | . | . | . |
| 21-225_74C8_LC | . | . | . | . | . | . | . | . | . | . | . | . | . | . | . | . | . | N | . |
| 21-225_74G6_LC | . | . | . | . | . | . | . | . | . | . | . | . | . | . | . | . | . | N | . |
| 21-225_76C5_LC | . | . | . | . | . | . | . | . | . | . | . | . | . | . | . | . | . | . | . |

# FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | L | T | F | G | G | G | T | K | V | E | I | K | R |
| 21-225_77E6_LC | . | . | . | . | . | . | . | . | . | . | . | . | . | R | . | . | . | . | . |
| 21-225_79A12_LC | . | . | . | . | . | . | . | . | . | . | . | . | . | . | . | . | . | . | . |
| 21-225_79F4_LC | . | . | . | . | . | . | . | . | . | . | . | . | . | R | . | . | . | . | . |
| 21-225_7F10_LC | . | . | . | . | . | . | . | . | . | . | . | . | . | . | . | . | . | . | . |
| 21-225_80C11_LC | . | . | . | . | . | . | . | . | . | . | . | . | . | . | . | . | . | N | . |

**FIGURE 57**

Table 102. Consensus 22 - VK1|O12/JK4 (SEQ ID NO: 50319):

DIQMTQSPSSLSASVGDRVTITCRAS--QNII------SYLNWYQQKPGKAPKLLIYT--------ASSLQSGVPSRFSGSGSG--TDFTLTISSLQPEDFATYYCQQSYS---------------------TPLTFGGGTKVEIKR wherein:

A at position 25 can be substituted with T

S at position 26 can be substituted with T

Q at position 29 can be substituted with H or R

N at position 30 can be substituted with S, R, T or I

I at position 31 can be substituted with V or F

I at position 32 can be substituted with S, N, Y, F, R, H, L, K or T

S at position 39 can be substituted with N, D, R, K or T

Y at position 40 can be substituted with F

N at position 42 an be substituted with H

T at position 58 can be substituted with A, V, G, I or S

A at position 67 can be substituted with T

S at position 69 can be substituted with N, R or T

L at position 70 can be substituted with F or S

Q at position 71 can be substituted with H, E or P

S at position 72 can be substituted with G, T, N or R

Q at position 108 can be substituted with L

S at position 109 can be substituted with T, N or P

**FIGURE 57**

Y at position 110 can be substituted with H, C, D, F or N

S at position 111 can be substituted with null (-), I, N, F, G or T

T at position 135 can be substituted with S, P, F, N, I or L

P at position 136 can be substituted with T, I, A or S

L at position 137 can be substituted with P, Y, F or V

Arg Xaa Xaa Xaa Xaa Xaa Xaa Xaa Xaa Leu Xaa (SEQ ID NO: 50141)

Xaa Xaa Ser Xaa Xaa Xaa Xaa (SEQ ID NO: 50142)

Gln Xaa Xaa Xaa Xaa Xaa Xaa Xaa Thr (SEQ ID NO: 50143)

Arg Ala Ser Gln Asn Ile Ile Ser Tyr Leu Asn (SEQ ID NO: 50627)

Thr Ala Ser Ser Leu Gln Ser (SEQ ID NO: 50628)

Gln Gln Ser Tyr Ser Thr Pro Leu Thr (SEQ ID NO: 50629)

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_10E12_LC | . | . | . | . | . | . | . | . | . | . | . | . | . | . | . | . | . | . | . | . | . | . | . | . | . | . |
| 21-225_10F5_LC | . | . | . | . | . | . | . | . | . | . | . | . | . | . | . | . | . | . | . | . | . | . | . | . | . | . |
| 21-225_12A10_LC | . | V | . | . | . | . | . | . | . | . | . | . | . | . | A | . | . | . | . | . | . | . | . | . | . | . |
| 21-225_13D4_LC | . | L | . | . | . | . | . | . | . | . | . | . | . | . | . | . | . | . | . | . | . | . | . | . | . | . |
| 21-225_146E9_LC | . | . | . | . | . | . | . | . | . | . | . | . | . | . | . | . | . | . | . | . | . | . | . | . | . | . |
| 21-225_146H1_LC | . | . | . | . | . | . | . | . | . | . | . | . | . | . | . | . | . | . | . | . | . | . | . | . | . | . |
| 21-225_148C4_LC | . | . | . | . | . | . | . | . | . | . | . | . | . | . | . | . | . | . | . | . | . | . | . | . | . | . |
| 21-225_148H9_LC | . | . | . | . | . | . | . | . | . | . | . | . | . | . | . | . | . | . | . | . | . | . | . | . | . | . |
| 21-225_149A3_LC | . | . | . | . | . | . | . | . | . | . | . | . | . | . | . | . | . | . | . | . | . | . | . | . | . | . |
| 21-225_150C11_LC | . | . | . | . | . | . | . | . | . | . | . | . | . | . | . | . | . | . | . | . | . | . | . | . | . | . |
| 21-225_150C12_LC | . | . | . | . | . | . | . | . | . | . | . | . | . | . | . | . | . | . | . | . | . | . | . | . | . | . |
| 21-225_151B12_LC | . | . | . | . | . | . | . | . | . | . | . | . | . | . | . | . | . | . | . | . | . | . | . | . | . | . |
| 21-225_152B11_LC | . | . | . | . | . | . | . | . | . | . | . | . | . | . | . | . | . | . | . | . | . | . | . | . | . | . |
| 21-225_152D2_LC | . | . | . | . | . | L | . | . | . | . | . | . | . | . | . | . | . | . | . | . | . | . | . | . | . | . |
| 21-225_152G4_LC | . | . | . | . | . | . | . | . | . | . | . | . | . | . | . | . | . | . | . | . | . | . | . | . | . | . |
| 21-225_155C4_LC | . | . | . | . | . | . | . | . | . | . | . | . | . | . | . | . | . | . | . | . | . | . | . | . | . | . |
| 21-225_156E4_LC | . | . | . | . | . | . | . | . | . | . | . | . | . | . | . | . | . | . | . | . | . | . | . | . | . | . |
| 21-225_159H2_LC | . | . | . | L | . | . | . | . | . | . | . | . | . | . | . | . | . | . | . | . | . | . | . | . | . | . |
| 21-225_15A3_LC | . | . | . | . | . | . | . | . | . | . | . | . | . | . | . | . | . | . | . | . | . | . | . | . | . | . |
| 21-225_16D11_LC | . | . | . | . | . | . | . | . | . | . | . | . | . | . | . | . | . | . | . | . | . | . | . | . | . | . |
| 21-225_16E6_LC | . | . | . | . | . | . | . | . | . | . | . | . | . | . | . | . | . | . | . | . | . | . | . | . | . | . |
| 21-225_17A6_LC | . | . | . | . | . | . | . | . | . | . | . | . | . | . | . | . | . | . | . | . | . | . | . | . | . | . |
| 21-225_17G4_LC | . | . | . | . | . | . | . | . | . | . | . | . | . | . | A | . | . | . | . | . | . | . | . | . | T | . |
| 21-225_18G4_LC | . | . | H | . | . | . | . | . | . | . | . | . | . | . | . | . | . | . | . | . | . | . | . | . | . | . |
| 21-225_196F4_LC | . | P | . | . | I | . | . | . | . | . | . | . | . | . | . | . | . | . | . | . | . | . | . | . | . | . |
| 21-225_19A1_LC | . | . | . | . | . | . | . | . | . | . | . | . | . | . | . | . | . | . | . | . | . | . | . | . | . | . |
| 21-225_1H12_LC | . | . | . | . | . | . | . | . | . | . | . | . | . | . | . | . | . | . | . | . | . | . | . | . | . | . |
| 21-225_201H10_LC | . | . | . | . | . | . | . | . | . | . | . | . | . | . | . | . | . | . | . | . | . | . | . | . | . | . |
| 21-225_203E3_LC | . | . | . | . | . | . | . | . | . | . | P | . | . | . | . | . | . | . | . | . | . | . | R | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_204D6_LC | . | S | . | . | . | . | . | . | . | . | . | . | . | . | . | . | . | . | . | . | . | . | . | . | . | . |
| 21-225_204H4_LC | . | . | . | . | . | L | . | . | . | . | P | . | . | P | . | . | . | . | . | . | . | . | R | . | . | . |
| 21-225_206A4_LC | . | . | . | . | . | L | . | . | . | . | P | . | . | P | . | . | . | . | . | . | . | . | R | . | . | . |
| 21-225_206A5_LC | . | . | . | . | . | . | . | . | . | . | P | . | . | . | . | . | . | . | . | . | . | . | R | . | . | . |
| 21-225_207C9_LC | . | N | . | . | . | . | . | . | . | . | . | . | . | . | . | . | . | . | . | . | . | . | . | . | . | . |
| 21-225_208B11_LC | . | . | . | . | . | . | . | . | . | . | P | . | . | . | . | . | . | . | . | . | . | . | . | . | . | . |
| 21-225_208E8_LC | . | . | . | . | . | . | . | . | . | . | P | . | . | P | . | . | . | . | . | . | . | . | R | . | . | . |
| 21-225_20F6_LC | . | . | . | . | . | . | . | . | . | . | . | . | . | . | . | . | . | . | . | . | . | . | . | . | . | . |
| 21-225_210D11_LC | . | . | . | . | . | . | . | . | . | . | . | . | . | . | . | . | . | . | . | . | . | . | . | . | . | . |
| 21-225_22C7_LC | . | . | . | . | . | . | . | . | . | . | . | . | . | . | . | . | . | . | . | . | . | . | . | . | . | . |
| 21-225_30G1_LC | . | . | . | . | . | . | . | . | . | . | . | . | . | . | . | . | . | . | . | . | . | . | . | . | . | . |
| 21-225_30G4_LC | . | . | . | . | . | . | . | . | P | . | . | . | T | . | . | . | . | . | . | . | . | . | . | . | . | T |
| 21-225_31F1_LC | . | . | . | . | . | . | . | . | . | . | . | . | . | . | . | . | . | . | . | . | . | . | . | . | . | . |
| 21-225_32D6_LC | . | . | . | . | . | . | . | . | . | . | . | . | T | . | . | . | . | . | . | . | . | . | . | . | . | . |
| 21-225_33B2_LC | . | . | . | . | . | . | . | . | . | . | . | . | T | . | . | . | . | K | . | . | . | . | . | . | . | . |
| 21-225_33C12_LC | . | . | . | . | . | . | . | . | . | . | . | . | T | . | . | . | . | . | . | . | . | . | . | . | . | . |
| 21-225_34C11_LC | . | . | . | . | . | . | P | . | . | . | . | . | T | . | . | . | . | . | . | . | S | . | . | . | . | . |
| 21-225_34C2_LC | . | . | . | . | . | . | . | . | . | . | . | . | T | P | . | . | . | . | . | . | . | . | . | . | . | . |
| 21-225_34D3_LC | . | . | . | . | . | . | . | . | . | . | . | . | . | . | . | . | . | . | . | . | . | . | . | . | . | . |
| 21-225_34D8_LC | . | . | . | . | . | . | . | . | . | . | . | . | T | . | . | . | . | . | . | . | . | . | . | . | . | . |
| 21-225_34G11_LC | . | . | . | . | . | . | . | . | . | . | . | . | . | . | . | . | . | . | . | . | . | . | . | . | . | . |
| 21-225_36B8_LC | . | . | . | . | . | . | . | . | . | . | . | . | T | . | . | . | . | . | . | . | . | . | . | . | . | . |
| 21-225_3F8_LC | . | P | . | . | . | . | . | . | . | . | . | . | . | . | . | . | . | . | . | . | . | . | . | . | . | . |
| 21-225_3G7_LC | . | . | . | . | . | . | . | . | . | . | . | . | . | . | . | . | . | . | . | . | V | . | . | . | . | . |
| 21-225_48B10_LC | . | . | . | . | . | . | . | . | . | . | . | . | . | . | . | . | . | . | . | . | . | . | . | . | . | . |
| 21-225_48F12_LC | . | . | . | . | . | . | . | . | . | . | . | . | . | . | . | . | . | . | . | . | . | . | . | . | . | . |
| 21-225_48G12_LC | . | . | . | . | . | . | . | . | . | . | . | . | . | . | . | . | . | . | . | . | . | . | . | . | . | . |
| 21-225_4G4_LC | . | . | . | . | . | . | . | . | . | . | . | A | . | . | . | . | . | . | . | . | . | . | . | . | . | . |
| 21-225_50F2_LC | . | . | . | . | . | . | . | . | . | . | . | . | . | . | . | . | . | . | I | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_56B6_LC | . | . | . | . | . | . | . | . | . | . | . | . | V | . | . | . | . | . | . | . | . | . | . | . | . | . |
| 21-225_62D2_LC | . | . | . | . | . | . | . | . | . | . | . | . | . | . | . | . | . | . | . | . | . | I | . | . | . | . |
| 21-225_63C10_LC | . | . | . | . | . | . | . | . | . | . | . | . | T | . | . | . | . | . | . | . | . | . | . | . | . | . |
| 21-225_64A11_LC | . | . | . | . | . | . | . | . | P | . | . | . | . | . | . | . | . | . | . | . | . | . | . | . | . | . |
| 21-225_64F7_LC | . | . | . | . | . | . | . | . | . | . | . | . | . | . | . | . | . | . | . | . | . | . | . | . | . | . |
| 21-225_6G1_LC | . | . | . | . | . | . | . | . | . | . | . | . | . | . | . | . | . | . | . | . | . | . | . | . | T | . |
| 21-225_7A10_LC | . | . | . | . | . | . | . | . | . | . | . | . | . | . | . | . | . | . | . | . | . | . | . | . | . | . |
| 21-225_7E11_LC | . | . | . | . | . | . | . | . | . | . | . | . | . | . | . | . | . | . | . | . | . | A | . | . | . | . |
| 21-225_7E11.001_LC | . | . | . | . | . | . | . | . | . | . | . | . | . | . | . | . | . | . | . | . | . | A | . | . | . | . |
| 21-225_7E11.001.001_LC | . | . | . | . | . | . | . | . | . | . | . | . | . | . | . | . | . | . | . | . | . | . | . | . | . | . |
| 21-225_7E11.001.002_LC | . | . | . | . | . | . | . | . | . | . | . | . | . | . | . | . | . | . | . | . | . | . | . | . | . | . |
| 21-225_7E11.001.003_LC | . | . | . | . | . | . | . | . | . | . | . | . | . | . | . | . | . | . | . | . | . | A | . | . | . | . |
| 21-225_7E11.001.004_LC | . | . | . | . | . | . | . | . | . | . | . | . | . | . | . | . | . | . | . | . | . | A | . | . | . | . |
| 21-225_7E11.001.005_LC | . | . | . | . | . | . | . | . | . | . | . | . | . | . | . | . | . | . | . | . | . | A | . | . | . | . |
| 21-225_7E11.001.006_LC | . | . | . | . | . | . | . | . | . | . | . | . | . | . | . | . | . | . | . | . | . | A | . | . | . | . |
| 21-225_7E11.001.007_LC | . | . | . | . | . | . | . | . | . | . | . | . | . | . | . | . | . | . | . | . | . | A | . | . | . | . |
| 21-225_7E11.001.008_LC | . | . | . | . | . | . | . | . | . | . | . | . | . | . | . | . | . | . | . | . | . | A | . | . | . | . |
| 21-225_7E11.001.009_LC | . | . | . | . | . | . | . | . | . | . | . | . | . | . | . | . | . | . | . | . | . | A | . | . | . | . |
| 21-225_7E11.001.010_LC | . | . | . | . | . | . | . | . | . | . | . | . | . | . | . | . | . | . | . | . | . | A | . | . | . | . |
| 21-225_7E11.001.011_LC | . | . | . | . | . | . | . | . | . | . | . | . | . | . | . | . | . | . | . | . | . | A | . | . | . | . |
| 21-225_7E11.001.012_LC | . | . | . | . | . | . | . | . | . | . | . | . | . | . | . | . | . | . | . | . | . | . | . | . | . | . |
| 21-225_7E11.001.013_LC | . | . | . | . | . | . | . | . | . | . | . | . | . | . | . | . | . | . | . | . | . | . | . | . | . | . |
| 21-225_7E11.001.014_LC | . | . | . | . | . | . | . | . | . | . | . | . | . | . | . | . | . | . | . | . | . | . | . | . | . | . |
| 21-225_7E11.001.015_LC | . | . | . | . | . | . | . | . | . | . | . | . | . | . | . | . | . | . | . | . | . | . | . | . | . | . |
| 21-225_7E11.001.016_LC | . | . | . | . | . | . | . | . | . | . | . | . | . | . | . | . | . | . | . | . | . | . | . | . | . | . |
| 21-225_7E11.001.017_LC | . | . | . | . | . | . | . | . | . | . | . | . | . | . | . | . | . | . | . | . | . | . | . | . | . | . |
| 21-225_7E11.001.018_LC | . | . | . | . | . | . | . | . | . | . | . | . | . | . | . | . | . | . | . | . | . | . | . | . | . | . |
| 21-225_7E11.001.019_LC | . | . | . | . | . | . | . | . | . | . | . | . | . | . | . | . | . | . | . | . | . | A | . | . | . | . |
| 21-225_7E11.001.021_LC | . | . | . | . | . | . | . | . | . | . | . | . | . | . | . | . | . | . | . | . | . | A | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_7E11.001.022_LC | . | . | . | . | . | . | . | . | . | . | . | . | . | . | . | . | . | . | . | . | . | . | . | . | . | . |
| 21-225_7E11.001.023_LC | . | . | . | . | . | . | . | . | . | . | . | . | . | . | . | . | . | . | . | . | . | A | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | N | I | I | - | - | - | - | - | - | S | Y | L | N | W | Y | Q | Q | K | P | G | K | A | P |
| 21-225_10E12_LC | . | . | . | . | . | Y | . | . | . | . | . | . | . | . | . | . | . | . | . | E | . | . | . | R | . | . |
| 21-225_10F5_LC | . | . | . | . | . | Y | . | . | . | . | . | . | R | . | . | . | . | . | . | E | . | . | . | R | . | . |
| 21-225_12A10_LC | . | . | . | . | . | Y | . | . | . | . | . | . | . | . | . | . | . | . | . | . | . | . | . | R | . | . |
| 21-225_13D4_LC | . | . | . | S | . | F | . | . | . | . | . | . | . | . | . | . | . | . | . | . | . | . | . | . | . | . |
| 21-225_146E9_LC | . | . | . | S | . | S | . | . | . | . | . | . | D | . | . | . | . | . | . | L | . | . | . | . | . | . |
| 21-225_146H1_LC | . | . | . | S | . | S | . | . | . | . | . | . | D | . | . | . | . | . | . | . | . | . | . | . | . | . |
| 21-225_148C4_LC | . | . | . | S | . | . | . | . | . | . | . | . | N | . | . | . | . | . | . | . | . | . | . | . | . | . |
| 21-225_148H9_LC | . | . | . | S | . | S | . | . | . | . | . | . | N | . | . | . | . | . | . | . | . | . | . | . | . | . |
| 21-225_149A3_LC | . | . | . | S | . | S | . | . | . | . | . | . | . | . | . | . | . | . | . | . | . | . | . | . | . | . |
| 21-225_150C11_LC | . | . | . | S | . | S | . | . | . | . | . | . | . | . | . | . | . | . | . | . | . | . | . | . | . | . |
| 21-225_150C12_LC | . | . | . | S | . | S | . | . | . | . | . | . | D | . | . | . | . | . | . | . | . | . | . | . | . | . |
| 21-225_151B12_LC | . | . | . | S | . | S | . | . | . | . | . | . | N | F | . | . | . | . | . | . | . | . | . | . | . | . |
| 21-225_152B11_LC | . | . | . | S | . | S | . | . | . | . | . | . | D | . | . | . | . | . | . | . | . | . | . | . | . | . |
| 21-225_152D2_LC | . | . | . | S | . | S | . | . | . | . | . | . | D | . | . | . | . | . | . | L | . | . | . | . | . | . |
| 21-225_152G4_LC | . | . | . | S | . | S | . | . | . | . | . | . | D | . | . | . | . | . | . | . | . | . | . | . | . | . |
| 21-225_155C4_LC | . | . | . | S | . | S | . | . | . | . | . | . | . | . | . | . | . | . | . | . | . | . | . | . | . | . |
| 21-225_156E4_LC | . | . | . | S | . | S | . | . | . | . | . | . | . | . | . | . | . | . | . | . | . | . | . | . | . | . |
| 21-225_159H2_LC | . | . | . | S | . | S | . | . | . | . | . | . | K | . | . | . | . | . | . | . | . | . | . | . | . | . |
| 21-225_15A3_LC | . | . | . | S | . | . | . | . | . | . | . | . | . | . | . | . | . | . | L | . | . | . | . | . | . | . |
| 21-225_16D11_LC | . | . | . | T | . | S | . | . | . | . | . | . | N | . | . | . | . | . | H | . | . | . | . | . | . | . |
| 21-225_16E6_LC | . | . | . | S | . | . | . | . | . | . | . | . | . | . | . | . | . | . | . | . | . | . | . | . | . | . |
| 21-225_17A6_LC | . | . | . | T | . | S | . | . | . | . | . | . | N | . | . | . | . | . | H | . | . | . | . | . | . | . |
| 21-225_17G4_LC | . | . | . | . | . | S | . | . | . | . | . | . | N | . | . | . | . | . | . | . | . | . | . | . | . | . |
| 21-225_18G4_LC | . | . | . | S | . | . | . | . | . | . | . | . | . | . | . | . | . | . | . | . | . | . | . | . | . | . |
| 21-225_196F4_LC | . | . | . | R | . | H | . | . | . | . | . | . | . | . | . | . | . | . | . | . | . | . | . | . | . | . |
| 21-225_19A1_LC | . | . | H | T | . | T | . | . | . | . | . | . | . | . | . | . | . | . | . | . | . | . | . | . | . | . |
| 21-225_1H12_LC | . | . | . | . | . | Y | . | . | . | . | . | . | R | . | . | . | . | . | . | E | . | . | . | R | . | . |
| 21-225_201H10_LC | . | . | H | . | . | N | . | . | . | . | . | . | . | . | . | . | . | . | . | . | . | S | . | . | . | . |
| 21-225_203E3_LC | . | . | H | . | . | N | . | . | . | . | . | . | . | . | . | . | . | . | . | . | . | S | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | N | I | I | - | - | - | - | - | - | S | Y | L | N | W | Y | Q | Q | K | P | G | K | A | P |
| 21-225_204D6_LC | . | . | R | S | V | H | . | . | . | . | . | . | T | . | . | . | . | . | . | . | . | . | . | . | . | . |
| 21-225_204H4_LC | . | . | H | . | . | N | . | . | . | . | . | . | . | . | . | . | . | . | . | . | . | S | . | . | . | . |
| 21-225_206A4_LC | . | . | H | . | . | N | . | . | . | . | . | . | . | . | . | . | . | . | . | . | . | S | . | . | . | . |
| 21-225_206A5_LC | . | . | H | . | . | N | . | . | . | . | . | . | . | . | . | . | . | . | . | . | . | S | . | . | . | . |
| 21-225_207C9_LC | . | . | . | R | F | S | . | . | . | . | . | . | N | . | . | . | . | . | . | . | . | . | . | . | . | . |
| 21-225_208B11_LC | . | . | H | . | . | N | . | . | . | . | . | . | . | . | . | . | . | . | . | . | . | S | . | . | . | . |
| 21-225_208E8_LC | . | . | H | . | . | N | . | . | . | . | . | . | . | . | . | . | . | . | . | . | . | S | . | . | . | . |
| 21-225_20F6_LC | . | . | . | S | . | . | . | . | . | . | . | . | T | . | . | . | . | . | . | . | . | . | . | . | . | . |
| 21-225_210D11_LC | . | . | H | . | . | N | . | . | . | . | . | . | . | . | . | . | . | . | . | . | . | S | . | . | . | . |
| 21-225_22C7_LC | . | . | . | . | . | . | . | . | . | . | . | . | . | . | . | . | . | . | . | . | . | . | E | . | . | . |
| 21-225_30G1_LC | . | . | . | . | . | Y | . | . | . | . | . | . | . | . | . | . | . | . | . | . | . | . | . | . | D | . |
| 21-225_30G4_LC | . | . | . | . | . | F | . | . | . | . | . | . | . | . | . | . | . | H | . | . | . | . | . | . | . | . |
| 21-225_31F1_LC | . | . | . | R | . | S | . | . | . | . | . | . | . | . | . | . | . | . | . | . | . | . | . | . | . | . |
| 21-225_32D6_LC | . | . | . | . | . | . | . | . | . | . | . | . | . | . | . | . | . | . | . | . | . | . | . | . | . | . |
| 21-225_33B2_LC | . | . | . | S | . | . | . | . | . | . | . | . | . | . | . | . | . | . | . | . | . | . | . | . | . | . |
| 21-225_33C12_LC | . | . | . | S | . | . | . | . | . | . | . | . | . | . | . | . | . | . | . | . | . | . | . | . | . | . |
| 21-225_34C11_LC | . | . | . | . | . | R | . | . | . | . | . | . | N | F | . | . | . | . | . | . | . | . | E | . | D | . |
| 21-225_34C2_LC | . | . | . | R | . | . | . | . | . | . | . | . | . | . | . | . | . | F | . | . | . | . | . | . | . | . |
| 21-225_34D3_LC | . | . | . | . | . | Y | . | . | . | . | . | . | . | . | . | . | . | . | . | . | . | . | . | . | D | . |
| 21-225_34D8_LC | . | . | . | I | . | . | . | . | . | . | . | . | . | . | . | . | . | . | . | . | . | . | . | . | . | . |
| 21-225_34G11_LC | . | . | . | . | . | N | . | . | . | . | . | . | R | . | . | . | . | . | . | . | . | . | . | . | . | . |
| 21-225_36B8_LC | . | . | . | S | . | . | . | . | . | . | . | . | . | . | . | . | . | . | . | . | . | . | . | . | . | . |
| 21-225_3F8_LC | . | . | . | S | . | L | . | . | . | . | . | . | . | . | . | . | . | . | R | . | . | . | . | R | . | . |
| 21-225_3G7_LC | . | . | . | . | . | Y | . | . | . | . | . | . | R | . | . | . | . | . | . | G | R | . | . | R | . | . |
| 21-225_48B10_LC | . | . | . | S | . | R | . | . | . | . | . | . | K | . | . | . | . | . | . | K | T | . | . | . | . | . |
| 21-225_48F12_LC | . | . | . | S | . | R | . | . | . | . | . | . | K | . | . | . | . | . | . | K | T | . | . | . | . | . |
| 21-225_48G12_LC | . | . | . | S | . | R | . | . | . | . | . | . | K | . | . | . | . | . | . | K | T | . | . | . | . | . |
| 21-225_4G4_LC | . | . | . | T | . | . | . | . | . | . | . | . | . | . | . | . | . | . | . | . | . | . | . | . | . | . |
| 21-225_50F2_LC | . | . | . | S | . | L | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | N | I | I | - | - | - | - | - | - | S | Y | L | N | W | Y | Q | Q | K | P | G | K | A | P |
| 21-225_56B6_LC | . | . | . | S | . | F | . | . | . | . | . | . | R | . | . | . | . | . | . | . | . | . | . | R | . | . |
| 21-225_62D2_LC | . | . | . | S | . | . | . | . | . | . | . | . | . | . | . | . | . | . | . | . | . | . | . | . | . | . |
| 21-225_63C10_LC | . | . | . | S | . | F | . | . | . | . | . | . | . | . | . | H | . | . | . | . | . | . | . | . | . | . |
| 21-225_64A11_LC | . | . | R | S | V | S | . | . | . | . | . | . | R | . | . | . | . | . | . | . | T | L | . | . | . | L |
| 21-225_64F7_LC | . | . | . | . | . | K | . | . | . | . | . | . | K | . | . | . | . | . | . | . | . | . | . | . | . | . |
| 21-225_6G1_LC | . | . | . | . | . | . | . | . | . | . | . | . | . | . | . | . | . | . | . | . | . | . | . | . | . | . |
| 21-225_7A10_LC | . | . | . | . | . | Y | . | . | . | . | . | . | . | . | . | . | . | . | . | . | . | . | . | R | . | . |
| 21-225_7E11_LC | . | . | . | . | . | . | . | . | . | . | . | . | . | . | . | . | . | . | . | . | . | . | . | . | . | . |
| 21-225_7E11.001_LC | . | . | . | . | . | . | . | . | . | . | . | . | . | . | . | . | . | . | . | . | . | . | . | . | . | . |
| 21-225_7E11.001.001_LC | . | . | . | . | . | . | . | . | . | . | . | . | . | . | . | . | . | . | . | . | . | . | . | . | . | . |
| 21-225_7E11.001.002_LC | . | . | . | . | . | . | . | . | . | . | . | . | . | . | . | . | . | . | . | . | . | . | . | . | . | . |
| 21-225_7E11.001.003_LC | . | . | . | . | . | . | . | . | . | . | . | . | . | . | . | . | . | . | . | . | . | . | . | . | . | . |
| 21-225_7E11.001.004_LC | . | . | . | . | . | . | . | . | . | . | . | . | . | . | . | . | . | . | . | . | . | . | . | . | . | . |
| 21-225_7E11.001.005_LC | . | . | . | . | . | . | . | . | . | . | . | . | . | . | . | . | . | . | . | . | . | . | . | . | . | . |
| 21-225_7E11.001.006_LC | . | . | . | . | . | . | . | . | . | . | . | . | . | . | . | . | . | . | . | . | . | . | . | . | . | . |
| 21-225_7E11.001.007_LC | . | . | . | . | . | . | . | . | . | . | . | . | . | . | . | . | . | . | . | . | . | . | . | . | . | . |
| 21-225_7E11.001.008_LC | . | . | . | . | . | . | . | . | . | . | . | . | . | . | . | . | . | . | . | . | . | . | . | . | . | . |
| 21-225_7E11.001.009_LC | . | . | . | . | . | . | . | . | . | . | . | . | . | . | . | . | . | . | . | . | . | . | . | . | . | . |
| 21-225_7E11.001.010_LC | . | . | . | . | . | . | . | . | . | . | . | . | . | . | . | . | . | . | . | . | . | . | . | . | . | . |
| 21-225_7E11.001.011_LC | . | . | . | . | . | . | . | . | . | . | . | . | . | . | . | . | . | . | . | . | . | . | . | . | . | . |
| 21-225_7E11.001.012_LC | . | . | . | . | . | . | . | . | . | . | . | . | . | . | . | . | . | . | . | . | . | . | . | . | . | . |
| 21-225_7E11.001.013_LC | . | . | . | . | . | . | . | . | . | . | . | . | . | . | . | . | . | . | . | . | . | . | . | . | . | . |
| 21-225_7E11.001.014_LC | . | . | . | . | . | . | . | . | . | . | . | . | . | . | . | . | . | . | . | . | . | . | . | . | . | . |
| 21-225_7E11.001.015_LC | . | . | . | . | . | . | . | . | . | . | . | . | . | . | . | . | . | . | . | . | . | . | . | . | . | . |
| 21-225_7E11.001.016_LC | . | . | . | . | . | . | . | . | . | . | . | . | . | . | . | . | . | . | . | . | . | . | . | . | . | . |
| 21-225_7E11.001.017_LC | . | . | . | . | . | . | . | . | . | . | . | . | . | . | . | . | . | . | . | . | . | . | . | . | . | . |
| 21-225_7E11.001.018_LC | . | . | . | . | . | . | . | . | . | . | . | . | . | . | . | . | . | . | . | . | . | . | . | . | . | . |
| 21-225_7E11.001.019_LC | . | . | . | . | . | . | . | . | . | . | . | . | . | . | . | . | . | . | . | . | . | . | . | . | . | . |
| 21-225_7E11.001.021_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | N | I | I | - | - | - | - | - | - | S | Y | L | N | W | Y | Q | Q | K | P | G | K | A | P |
| 21-225_7E11.001.022_LC | . | . | . | . | . | . | . | . | . | . | . | . | . | . | . | . | . | . | . | . | . | . | . | . | . | . |
| 21-225_7E11.001.023_LC | . | . | . | . | . | . | . | . | . | . | . | . | . | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | Y | T | - | - | - | - | - | - | - | - | A | S | S | L | Q | S | G | V | P | S | R | F |
| 21-225_10E12_LC | . | . | . | . | . | A | . | . | . | . | . | . | . | . | T | . | . | . | . | . | . | . | . | . | . | . |
| 21-225_10F5_LC | . | . | . | . | . | . | . | . | . | . | . | . | . | . | . | . | . | . | . | . | . | . | . | . | . | . |
| 21-225_12A10_LC | . | V | . | . | H | . | . | . | . | . | . | . | . | . | . | . | . | . | . | . | . | . | . | . | . | . |
| 21-225_13D4_LC | . | . | . | . | C | . | . | . | . | . | . | . | . | . | . | . | . | . | . | N | . | . | . | . | . | . |
| 21-225_146E9_LC | . | V | . | . | . | . | . | . | . | . | . | . | . | . | T | . | . | . | . | . | . | . | . | . | . | . |
| 21-225_146H1_LC | . | V | . | . | . | . | . | . | . | . | . | . | . | . | T | . | . | . | . | . | . | . | . | . | . | . |
| 21-225_148C4_LC | . | V | . | . | . | . | . | . | . | . | . | . | . | . | . | . | . | . | . | . | . | . | . | . | . | . |
| 21-225_148H9_LC | . | V | . | . | . | I | . | . | . | . | . | . | . | . | . | . | . | . | . | . | . | . | . | . | . | . |
| 21-225_149A3_LC | . | V | M | . | . | . | . | . | . | . | . | . | . | . | . | . | . | . | . | . | . | . | . | . | . | . |
| 21-225_150C11_LC | . | . | . | . | . | . | . | . | . | . | . | . | . | . | . | . | . | . | . | . | . | . | . | . | . | . |
| 21-225_150C12_LC | . | V | . | . | . | . | . | . | . | . | . | . | . | . | T | . | . | . | . | . | . | . | . | . | . | . |
| 21-225_151B12_LC | . | V | . | . | . | . | . | . | . | . | . | . | . | . | . | . | . | . | E | . | . | . | . | . | . | . |
| 21-225_152B11_LC | . | V | . | . | . | . | . | . | . | . | . | . | . | . | T | . | . | . | . | . | . | . | . | . | . | . |
| 21-225_152D2_LC | . | V | . | . | . | . | . | . | . | . | . | . | . | . | T | . | . | . | . | . | . | . | . | . | . | . |
| 21-225_152G4_LC | . | V | . | . | . | . | . | . | . | . | . | . | . | . | T | . | . | . | . | . | . | . | . | . | . | . |
| 21-225_155C4_LC | . | V | . | . | F | . | . | . | . | . | . | . | . | . | . | . | . | . | . | . | . | . | . | . | . | . |
| 21-225_156E4_LC | . | V | . | . | . | . | . | . | . | . | . | . | . | . | . | . | . | . | . | . | . | . | . | . | . | . |
| 21-225_159H2_LC | E | . | . | . | . | A | . | . | . | . | . | . | . | . | T | . | N | . | . | . | . | . | . | . | . | . |
| 21-225_15A3_LC | . | F | . | . | . | . | . | . | . | . | . | . | . | . | . | . | . | . | . | . | . | . | . | . | . | . |
| 21-225_16D11_LC | . | . | . | . | . | A | . | . | . | . | . | . | . | . | . | . | . | . | . | . | . | . | . | . | . | . |
| 21-225_16E6_LC | . | F | . | . | . | . | . | . | . | . | . | . | . | . | T | . | . | . | . | . | . | . | . | . | . | . |
| 21-225_17A6_LC | . | . | . | . | . | A | . | . | . | . | . | . | . | . | . | . | . | . | . | . | . | . | . | . | . | . |
| 21-225_17G4_LC | . | . | . | . | . | V | . | . | . | . | . | . | . | . | . | . | . | F | P | . | . | . | . | . | . | . |
| 21-225_18G4_LC | . | V | . | . | . | . | . | . | . | . | . | . | . | . | . | . | . | . | . | T | . | . | . | . | Q | . |
| 21-225_196F4_LC | . | . | . | . | . | . | . | . | . | . | . | . | . | . | . | . | . | . | . | G | . | . | . | . | . | . |
| 21-225_19A1_LC | . | F | . | . | . | A | . | . | . | . | . | . | . | . | T | . | N | . | . | . | . | . | . | . | . | . |
| 21-225_1H12_LC | . | . | . | . | . | . | . | . | . | . | . | . | . | . | . | . | . | . | . | . | . | . | . | . | . | . |
| 21-225_201H10_LC | . | . | . | . | . | A | . | . | . | . | . | . | . | . | . | . | . | . | . | . | . | . | . | . | . | . |
| 21-225_203E3_LC | . | . | . | . | . | A | . | . | . | . | . | . | . | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | L | L | I | Y | T | - | - | - | - | - | - | - | - | A | S | S | L | Q | S | G | V | P | S | R | F |
| 21-225_204D6_LC | . | V | . | . | . | G | . | . | . | . | . | . | . | . | . | . | . | . | . | N | . | . | . | . | . | . |
| 21-225_204H4_LC | . | . | . | . | . | A | . | . | . | . | . | . | . | . | . | . | . | . | . | . | . | . | . | . | . | . |
| 21-225_206A4_LC | . | . | . | . | C | A | . | . | . | . | . | . | . | . | . | . | . | . | . | . | . | . | . | . | . | . |
| 21-225_206A5_LC | . | . | . | . | C | A | . | . | . | . | . | . | . | . | . | . | . | . | . | . | . | . | . | . | . | . |
| 21-225_207C9_LC | . | . | . | . | . | . | . | . | . | . | . | . | . | . | . | . | . | . | . | . | . | . | . | . | . | . |
| 21-225_208B11_LC | . | . | . | . | . | A | . | . | . | . | . | . | . | . | . | . | . | . | . | . | . | . | . | . | . | . |
| 21-225_208E8_LC | . | . | . | . | . | A | . | . | . | . | . | . | . | . | . | . | . | . | . | . | . | . | . | . | . | . |
| 21-225_20F6_LC | . | V | . | . | H | . | . | . | . | . | . | . | . | . | . | . | . | . | . | . | . | . | . | . | . | . |
| 21-225_210D11_LC | . | . | . | . | . | A | . | . | . | . | . | . | . | . | . | . | . | . | . | . | . | . | . | . | . | . |
| 21-225_22C7_LC | . | . | . | . | H | I | . | . | . | . | . | . | . | . | . | . | . | . | . | . | . | . | . | . | . | . |
| 21-225_30G1_LC | . | . | . | . | . | A | . | . | . | . | . | . | . | . | . | . | . | . | H | . | . | . | . | . | . | . |
| 21-225_30G4_LC | . | . | . | . | H | . | . | . | . | . | . | . | . | . | . | . | . | . | . | G | . | . | . | . | . | . |
| 21-225_31F1_LC | . | . | . | . | F | A | . | . | . | . | . | . | . | . | . | . | . | S | . | . | . | . | . | . | . | . |
| 21-225_32D6_LC | . | . | . | M | . | V | . | . | . | . | . | . | . | . | . | . | N | . | . | . | . | . | . | . | . | . |
| 21-225_33B2_LC | . | . | . | . | . | V | . | . | . | . | . | . | . | . | . | . | . | . | . | . | . | . | . | . | . | . |
| 21-225_33C12_LC | . | . | . | . | . | V | . | . | . | . | . | . | . | . | . | . | . | . | . | G | . | . | . | . | . | . |
| 21-225_34C11_LC | . | . | Q | . | . | G | . | . | . | . | . | . | . | . | . | . | T | . | . | . | . | . | . | . | . | . |
| 21-225_34C2_LC | . | . | . | . | . | V | . | . | . | . | . | . | . | . | . | . | . | . | . | . | . | . | . | . | . | . |
| 21-225_34D3_LC | . | . | . | . | . | A | . | . | . | . | . | . | . | . | . | . | . | . | H | . | . | . | . | . | . | . |
| 21-225_34D8_LC | . | . | . | . | F | V | . | . | . | . | . | . | . | . | . | . | . | . | . | . | . | . | . | . | . | . |
| 21-225_34G11_LC | . | . | . | . | . | G | . | . | . | . | . | . | . | . | . | . | . | . | . | . | . | . | . | . | . | . |
| 21-225_36B8_LC | . | . | . | . | . | V | . | . | . | . | . | . | . | . | . | . | . | . | . | . | . | . | . | . | . | . |
| 21-225_3F8_LC | Q | V | . | . | H | . | . | . | . | . | . | . | . | . | T | . | . | . | . | . | . | . | . | . | . | . |
| 21-225_3G7_LC | . | . | M | . | . | A | . | . | . | . | . | . | . | . | . | . | . | S | . | . | . | . | . | . | . | . |
| 21-225_48B10_LC | . | . | . | . | . | A | . | . | . | . | . | . | . | . | . | . | . | . | . | . | . | . | . | . | . | . |
| 21-225_48F12_LC | . | . | . | . | . | A | . | . | . | . | . | . | . | . | . | . | . | . | . | . | . | . | . | . | . | . |
| 21-225_48G12_LC | . | . | . | . | . | A | . | . | . | . | . | . | . | . | . | . | . | . | . | . | . | . | . | . | . | . |
| 21-225_4G4_LC | . | . | . | . | H | . | . | . | . | . | . | . | . | . | . | . | . | . | . | . | . | . | . | . | . | . |
| 21-225_50F2_LC | . | . | . | . | . | V | . | . | . | . | . | . | . | . | . | . | . | F | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | L | L | I | Y | T | - | - | - | - | - | - | - | - | A | S | S | L | Q | S | G | V | P | S | R | F |
| 21-225_56B6_LC | . | . | . | . | F | A | . | . | . | . | . | . | . | . | . | . | . | F | . | . | . | . | . | . | . | . |
| 21-225_62D2_LC | . | . | . | . | . | A | . | . | . | . | . | . | . | . | T | . | . | . | . | . | . | . | . | . | . | . |
| 21-225_63C10_LC | . | . | . | . | S | A | . | . | . | . | . | . | . | . | . | . | . | . | . | . | . | . | . | . | . | . |
| 21-225_64A11_LC | . | . | . | . | S | V | . | . | . | . | . | . | . | . | . | . | R | . | . | . | . | . | . | . | . | . |
| 21-225_64F7_LC | . | F | . | . | . | G | . | . | . | . | . | . | . | . | . | . | . | . | . | . | . | . | . | . | . | . |
| 21-225_6G1_LC | . | V | . | . | . | . | . | . | . | . | . | . | . | . | . | . | N | . | . | T | . | . | . | . | G | . |
| 21-225_7A10_LC | . | . | V | . | . | S | . | . | . | . | . | . | . | . | T | . | . | . | . | . | . | . | . | . | . | . |
| 21-225_7E11_LC | . | F | . | . | . | . | . | . | . | . | . | . | . | . | . | . | . | . | . | . | . | . | . | . | . | . |
| 21-225_7E11.001_LC | . | F | . | . | . | . | . | . | . | . | . | . | . | . | . | . | . | . | . | . | . | . | . | . | . | . |
| 21-225_7E11.001.001_LC | . | F | . | . | . | . | . | . | . | . | . | . | . | . | . | . | . | . | . | . | . | . | . | . | . | . |
| 21-225_7E11.001.002_LC | . | . | . | . | . | . | . | . | . | . | . | . | . | . | . | . | . | . | . | . | . | . | . | . | . | . |
| 21-225_7E11.001.003_LC | . | F | . | . | . | . | . | . | . | . | . | . | . | . | . | . | . | . | . | . | . | . | . | . | . | . |
| 21-225_7E11.001.004_LC | . | F | . | . | . | . | . | . | . | . | . | . | . | . | . | . | . | . | . | . | . | . | . | . | . | . |
| 21-225_7E11.001.005_LC | . | F | . | . | . | . | . | . | . | . | . | . | . | . | . | . | . | . | . | . | . | . | . | . | . | . |
| 21-225_7E11.001.006_LC | . | F | . | . | . | . | . | . | . | . | . | . | . | . | . | . | . | . | . | . | . | . | . | . | . | . |
| 21-225_7E11.001.007_LC | . | F | . | . | . | . | . | . | . | . | . | . | . | . | . | . | . | . | . | . | . | . | . | . | . | . |
| 21-225_7E11.001.008_LC | . | F | . | . | . | . | . | . | . | . | . | . | . | . | . | . | . | . | . | . | . | . | . | . | . | . |
| 21-225_7E11.001.009_LC | . | F | . | . | . | . | . | . | . | . | . | . | . | . | . | . | . | . | . | . | . | . | . | . | . | . |
| 21-225_7E11.001.010_LC | . | F | . | . | . | . | . | . | . | . | . | . | . | . | . | . | . | . | . | . | . | . | . | . | . | . |
| 21-225_7E11.001.011_LC | . | F | . | . | . | . | . | . | . | . | . | . | . | . | . | . | . | . | . | . | . | . | . | . | . | . |
| 21-225_7E11.001.012_LC | . | F | . | . | . | . | . | . | . | . | . | . | . | . | . | . | . | . | . | . | . | . | . | . | . | . |
| 21-225_7E11.001.013_LC | . | F | . | . | . | . | . | . | . | . | . | . | . | . | . | . | . | . | . | . | . | . | . | . | . | . |
| 21-225_7E11.001.014_LC | . | F | . | . | . | . | . | . | . | . | . | . | . | . | . | . | . | . | . | . | . | . | . | . | . | . |
| 21-225_7E11.001.015_LC | . | . | . | . | . | . | . | . | . | . | . | . | . | . | . | . | . | . | . | . | . | . | . | . | . | . |
| 21-225_7E11.001.016_LC | . | . | . | . | . | . | . | . | . | . | . | . | . | . | . | . | . | . | . | . | . | . | . | . | . | . |
| 21-225_7E11.001.017_LC | . | . | . | . | . | . | . | . | . | . | . | . | . | . | . | . | . | . | . | . | . | . | . | . | . | . |
| 21-225_7E11.001.018_LC | . | F | . | . | . | . | . | . | . | . | . | . | . | . | . | . | . | . | . | . | . | . | . | . | . | . |
| 21-225_7E11.001.019_LC | . | F | . | . | . | . | . | . | . | . | . | . | . | . | . | . | . | . | . | . | . | . | . | . | . | . |
| 21-225_7E11.001.021_LC | . | F | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | Y | T | - | - | - | - | - | - | - | - | A | S | S | L | Q | S | G | V | P | S | R | F |
| 21-225_7E11.001.022_LC | . | . | . | . | . | . | . | . | . | . | . | . | . | . | . | . | . | . | . | . | . | . | . | . | . | . |
| 21-225_7E11.001.023_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_10E12_LC | G | . | . | . | . | . | . | . | . | . | . | . | . | . | . | . | . | . | . | . | . | . | . | . | . | . |
| 21-225_10F5_LC | . | . | . | D | . | . | . | . | . | . | . | . | . | . | . | . | . | . | . | . | . | . | . | . | . | . |
| 21-225_12A10_LC | . | . | . | . | . | . | . | . | A | . | . | . | . | . | . | N | . | . | . | . | . | . | . | . | . | . |
| 21-225_13D4_LC | . | . | . | . | . | . | . | . | . | . | . | . | . | . | . | . | . | . | . | . | . | . | . | . | . | . |
| 21-225_146E9_LC | . | . | . | . | . | . | . | . | . | . | . | . | . | . | . | . | . | . | . | . | . | . | . | . | . | . |
| 21-225_146H1_LC | . | . | . | . | . | . | . | . | . | . | . | . | . | . | . | . | . | . | . | . | . | . | . | . | . | . |
| 21-225_148C4_LC | . | . | . | . | . | . | . | . | . | . | . | . | . | . | . | . | . | . | . | . | . | . | . | T | . | . |
| 21-225_148H9_LC | . | . | . | . | . | . | . | . | . | . | . | . | . | . | . | . | . | . | . | . | . | . | . | . | . | . |
| 21-225_149A3_LC | . | . | . | . | . | . | . | . | . | . | . | . | . | . | . | N | . | . | . | . | . | . | . | . | . | . |
| 21-225_150C11_LC | . | . | . | . | . | . | . | . | . | . | . | . | . | . | . | . | . | . | . | . | . | . | . | . | . | . |
| 21-225_150C12_LC | . | . | . | . | . | . | . | . | . | . | . | . | . | . | . | . | . | . | . | . | . | . | . | . | . | . |
| 21-225_151B12_LC | . | . | . | E | . | . | . | . | . | . | . | . | . | . | . | . | . | . | . | . | . | . | . | . | . | . |
| 21-225_152B11_LC | . | . | . | . | . | . | . | . | . | . | . | . | . | . | . | . | . | . | . | . | . | . | . | . | . | . |
| 21-225_152D2_LC | . | . | . | . | . | . | . | . | . | . | . | . | . | . | . | . | . | . | . | . | . | . | . | . | . | . |
| 21-225_152G4_LC | . | . | . | . | . | . | . | . | . | . | . | . | . | . | . | . | . | . | . | . | . | . | . | . | . | . |
| 21-225_155C4_LC | . | . | . | . | . | . | . | . | . | . | . | . | . | . | . | . | . | . | . | . | . | . | . | . | . | . |
| 21-225_156E4_LC | . | . | . | . | . | . | . | . | . | . | . | . | . | . | . | . | . | . | . | . | . | . | . | . | . | . |
| 21-225_159H2_LC | . | . | . | . | . | . | . | . | . | . | . | . | . | . | . | . | T | . | . | . | . | . | . | V | . | . |
| 21-225_15A3_LC | . | . | . | . | . | . | . | . | . | . | . | . | . | . | . | . | . | . | . | . | . | . | . | . | . | . |
| 21-225_16D11_LC | . | . | . | . | . | . | . | . | . | . | . | . | . | . | . | . | . | . | . | . | . | . | . | . | . | . |
| 21-225_16E6_LC | . | . | . | . | . | . | . | . | . | . | . | . | . | . | . | . | . | . | . | . | . | . | . | . | . | . |
| 21-225_17A6_LC | . | . | . | . | . | . | . | . | . | . | . | . | . | . | . | . | . | . | . | . | . | . | . | . | . | . |
| 21-225_17G4_LC | . | . | . | . | . | . | . | . | . | E | . | . | . | . | . | . | . | . | . | . | . | . | . | . | . | . |
| 21-225_18G4_LC | . | . | . | . | . | . | . | . | . | . | . | . | . | . | . | . | . | . | . | . | . | . | . | . | . | . |
| 21-225_196F4_LC | . | . | . | . | . | . | . | . | . | . | . | . | . | . | . | . | . | . | . | . | . | . | . | . | . | . |
| 21-225_19A1_LC | . | . | . | . | . | . | . | . | . | . | . | . | P | . | . | . | . | . | . | . | . | . | . | . | I | . |
| 21-225_1H12_LC | . | . | . | D | . | . | . | . | . | . | . | . | . | . | . | . | . | . | . | . | . | . | . | . | . | . |
| 21-225_201H10_LC | . | . | . | . | . | . | . | . | . | . | . | . | . | . | F | . | . | . | . | . | . | . | . | . | . | . |
| 21-225_203E3_LC | . | . | . | . | . | . | . | . | . | . | . | . | . | . | F | . | . | . | . | . | . | . | . | . | . | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_204D6_LC | . | . | . | . | . | . | . | . | . | . | . | . | . | . | . | . | . | . | . | . | . | . | V | . | . | . |
| 21-225_204H4_LC | . | . | . | . | . | . | . | . | . | . | . | . | . | . | F | . | . | . | . | . | . | . | . | . | . | . |
| 21-225_206A4_LC | . | . | . | . | . | . | . | . | . | . | . | . | . | . | F | . | . | . | . | . | . | . | . | . | . | . |
| 21-225_206A5_LC | . | . | . | . | . | . | . | . | . | . | . | . | . | . | F | . | . | . | . | . | . | . | . | . | . | . |
| 21-225_207C9_LC | . | A | . | V | . | . | . | . | . | . | . | . | . | . | . | . | . | . | . | . | . | . | . | . | . | . |
| 21-225_208B11_LC | . | . | . | . | . | . | . | . | . | . | . | . | . | . | F | . | . | . | . | . | . | . | . | . | . | . |
| 21-225_208E8_LC | . | . | . | . | . | . | . | . | . | . | . | . | . | . | F | . | . | . | . | . | . | . | . | . | . | . |
| 21-225_20F6_LC | . | . | . | . | . | . | . | . | . | . | . | . | . | . | . | . | . | . | . | . | . | . | . | . | . | . |
| 21-225_210D11_LC | . | . | . | . | . | . | . | . | . | . | . | . | . | . | F | . | . | . | . | . | . | . | . | . | . | . |
| 21-225_22C7_LC | . | . | . | . | . | . | . | . | . | . | . | . | . | . | . | . | . | . | . | . | . | . | . | . | . | . |
| 21-225_30G1_LC | . | . | . | . | . | . | . | . | . | . | . | . | . | . | . | . | . | . | . | . | . | . | . | . | . | . |
| 21-225_30G4_LC | . | . | . | . | . | . | . | . | . | . | . | . | . | . | . | I | . | M | . | . | . | . | . | S | . | . |
| 21-225_31F1_LC | . | . | . | . | . | . | . | . | . | . | . | . | . | . | . | . | . | . | . | . | . | . | . | . | . | . |
| 21-225_32D6_LC | . | . | . | . | . | . | . | . | . | . | . | . | . | . | . | . | . | . | . | A | . | . | . | . | . | . |
| 21-225_33B2_LC | . | . | . | . | . | . | . | . | . | . | . | . | . | . | . | . | . | . | . | . | . | . | . | . | . | . |
| 21-225_33C12_LC | N | . | . | . | . | . | . | . | . | . | . | . | . | . | . | . | . | . | . | . | . | . | . | . | . | . |
| 21-225_34C11_LC | . | . | . | . | F | . | . | . | . | . | . | . | . | . | . | . | . | . | . | . | K | . | . | . | . | . |
| 21-225_34C2_LC | . | . | . | . | . | . | . | . | . | Y | . | . | . | . | . | . | . | . | . | . | . | . | . | . | . | . |
| 21-225_34D3_LC | . | . | . | . | . | . | . | . | . | . | . | . | . | . | . | . | . | . | . | . | . | . | . | . | . | F |
| 21-225_34D8_LC | . | . | . | . | . | . | . | . | . | . | . | . | . | . | . | . | . | . | . | . | . | . | . | . | . | . |
| 21-225_34G11_LC | . | . | . | . | . | . | . | . | . | . | . | . | . | . | . | . | T | . | . | . | . | . | . | . | . | . |
| 21-225_36B8_LC | . | . | . | . | . | . | . | . | . | . | . | . | . | . | . | . | . | . | . | . | . | . | . | . | . | . |
| 21-225_3F8_LC | . | . | . | . | . | . | . | . | . | V | . | . | . | . | . | . | . | . | . | . | . | . | . | . | . | . |
| 21-225_3G7_LC | . | . | . | . | . | . | . | . | . | . | . | . | V | . | . | . | . | . | . | . | . | . | . | . | . | . |
| 21-225_48B10_LC | . | . | . | . | . | . | . | . | . | . | . | . | . | . | . | . | . | V | . | . | . | . | . | . | . | . |
| 21-225_48F12_LC | . | . | . | . | . | . | . | . | . | . | . | . | . | . | . | . | . | . | . | . | . | . | . | . | N | . |
| 21-225_48G12_LC | . | . | . | . | . | . | . | . | . | . | . | . | . | . | . | . | . | V | . | . | . | . | . | . | N | . |
| 21-225_4G4_LC | . | . | . | . | . | . | . | . | . | . | . | . | . | S | . | . | . | . | . | . | . | . | . | . | . | . |
| 21-225_50F2_LC | . | . | . | . | . | . | . | . | . | . | . | . | . | . | . | . | . | . | . | . | D | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_56B6_LC | . | . | . | . | . | . | . | . | . | . | . | . | . | . | . | . | . | . | . | . | . | . | . | . | . | . |
| 21-225_62D2_LC | . | . | . | . | . | . | . | . | . | . | . | . | . | . | . | . | . | . | . | . | . | . | . | . | . | . |
| 21-225_63C10_LC | . | . | . | . | . | . | . | . | . | . | . | . | . | . | . | . | . | . | . | . | . | . | . | . | . | F |
| 21-225_64A11_LC | . | . | . | . | . | . | . | . | . | . | . | . | . | . | . | . | . | V | . | [illegible] | . | . | . | . | . | . |
| 21-225_64F7_LC | . | . | . | . | . | . | . | . | . | . | . | . | . | . | . | . | . | . | . | . | . | . | . | . | A | . |
| 21-225_6G1_LC | . | . | . | . | . | . | . | . | . | . | . | . | . | . | . | . | . | . | . | . | . | . | . | . | . | . |
| 21-225_7A10_LC | . | . | . | . | . | . | . | . | . | . | . | S | . | . | . | . | N | . | . | . | . | . | . | S | . | . |
| 21-225_7E11_LC | . | . | . | . | . | . | . | . | . | . | . | . | . | . | . | . | . | . | . | . | . | . | . | . | I | . |
| 21-225_7E11.001_LC | . | . | . | . | . | . | . | . | . | . | . | . | . | . | . | . | . | . | . | . | . | . | . | . | I | . |
| 21-225_7E11.001.001_LC | . | . | . | . | . | . | . | . | . | . | . | . | . | . | . | . | . | . | . | . | . | . | . | . | I | . |
| 21-225_7E11.001.002_LC | . | . | . | . | . | . | . | . | . | . | . | . | . | . | . | . | . | . | . | . | . | . | . | . | . | . |
| 21-225_7E11.001.003_LC | . | . | . | . | . | . | . | . | . | . | . | . | . | . | . | . | . | . | . | . | . | . | . | . | I | . |
| 21-225_7E11.001.004_LC | . | . | . | . | . | . | . | . | . | . | . | . | . | . | . | . | . | . | . | . | . | . | . | . | I | . |
| 21-225_7E11.001.005_LC | . | . | . | . | . | . | . | . | . | . | . | . | . | . | . | . | . | . | . | . | . | . | . | . | I | . |
| 21-225_7E11.001.006_LC | . | . | . | . | . | . | . | . | . | . | . | . | . | . | . | . | . | . | . | . | . | . | . | . | I | . |
| 21-225_7E11.001.007_LC | . | . | . | . | . | . | . | . | . | . | . | . | . | . | . | . | . | . | . | . | . | . | . | . | I | . |
| 21-225_7E11.001.008_LC | . | . | . | . | . | . | . | . | . | . | . | . | . | . | . | . | . | . | . | . | . | . | . | . | I | . |
| 21-225_7E11.001.009_LC | . | . | . | . | . | . | . | . | . | . | . | . | . | . | . | . | . | . | . | . | . | . | . | . | I | . |
| 21-225_7E11.001.010_LC | . | . | . | . | . | . | . | . | . | . | . | . | . | . | . | . | . | . | . | . | . | . | . | . | I | . |
| 21-225_7E11.001.011_LC | . | . | . | . | . | . | . | . | . | . | . | . | . | . | . | . | . | . | . | . | . | . | . | . | I | . |
| 21-225_7E11.001.012_LC | . | . | . | . | . | . | . | . | . | . | . | . | . | . | . | . | . | . | . | . | . | . | . | . | I | . |
| 21-225_7E11.001.013_LC | . | . | . | . | . | . | . | . | . | . | . | . | . | . | . | . | . | . | . | . | . | . | . | . | I | . |
| 21-225_7E11.001.014_LC | . | . | . | . | . | . | . | . | . | . | . | . | . | . | . | . | . | . | . | . | . | . | . | . | I | . |
| 21-225_7E11.001.015_LC | . | . | . | . | . | . | . | . | . | . | . | . | . | . | . | . | . | . | . | . | . | . | . | . | . | . |
| 21-225_7E11.001.016_LC | . | . | . | . | . | . | . | . | . | . | . | . | . | . | . | . | . | . | . | . | . | . | . | . | . | . |
| 21-225_7E11.001.017_LC | . | . | . | . | . | . | . | . | . | . | . | . | . | . | . | . | . | . | . | . | . | . | . | . | . | . |
| 21-225_7E11.001.018_LC | . | . | . | . | . | . | . | . | . | . | . | . | . | . | . | . | . | . | . | . | . | . | . | . | I | . |
| 21-225_7E11.001.019_LC | . | . | . | . | . | . | . | . | . | . | . | . | . | . | . | . | . | . | . | . | . | . | . | . | I | . |
| 21-225_7E11.001.021_LC | . | . | . | . | . | . | . | . | . | . | . | . | . | . | . | . | . | . | . | . | . | . | . | . | I | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_7E11.001.022_LC | . | . | . | . | . | . | . | . | . | . | . | . | . | . | . | . | . | . | . | . | . | . | . | . | \| | . |
| 21-225_7E11.001.023_LC | . | . | . | . | . | . | . | . | . | . | . | . | . | . | . | . | . | . | . | . | . | . | . | . | \| | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | K_CDR3 | | | | | | | | | | | | | | | | | | | | | | | | |
| CONSENSUS | Y | C | Q | Q | S | Y | S | - | - | - | - | - | - | - | - | - | - | - | - | - | - | - | - | - | - | - |
| 21-225_10E12_LC | . | . | . | . | N | . | . | . | . | . | . | . | . | . | . | . | . | . | . | . | . | . | . | . | . | . |
| 21-225_10F5_LC | . | . | . | . | . | . | . | . | . | . | . | . | . | . | . | . | . | . | . | . | . | . | . | . | . | . |
| 21-225_12A10_LC | . | . | . | . | . | . | . | . | . | . | . | . | . | . | . | . | . | . | . | . | . | . | . | . | . | . |
| 21-225_13D4_LC | . | . | . | . | T | . | . | . | . | . | . | . | . | . | . | . | . | . | . | . | . | . | . | . | . | . |
| 21-225_146E9_LC | F | . | . | . | . | . | - | . | . | . | . | . | . | . | . | . | . | . | . | . | . | . | . | . | . | . |
| 21-225_146H1_LC | . | . | . | . | . | . | - | . | . | . | . | . | . | . | . | . | . | . | . | . | . | . | . | . | . | . |
| 21-225_148C4_LC | . | . | . | . | . | . | - | . | . | . | . | . | . | . | . | . | . | . | . | . | . | . | . | . | . | . |
| 21-225_148H9_LC | . | . | . | . | . | . | - | . | . | . | . | . | . | . | . | . | . | . | . | . | . | . | . | . | . | . |
| 21-225_149A3_LC | . | . | . | . | . | . | - | . | . | . | . | . | . | . | . | . | . | . | . | . | . | . | . | . | . | . |
| 21-225_150C11_LC | . | . | . | . | . | . | - | . | . | . | . | . | . | . | . | . | . | . | . | . | . | . | . | . | . | . |
| 21-225_150C12_LC | . | . | . | . | . | F | - | . | . | . | . | . | . | . | . | . | . | . | . | . | . | . | . | . | . | . |
| 21-225_151B12_LC | . | . | . | . | . | . | - | . | . | . | . | . | . | . | . | . | . | . | . | . | . | . | . | . | . | . |
| 21-225_152B11_LC | F | . | . | . | . | . | - | . | . | . | . | . | . | . | . | . | . | . | . | . | . | . | . | . | . | . |
| 21-225_152D2_LC | F | . | . | . | . | . | - | . | . | . | . | . | . | . | . | . | . | . | . | . | . | . | . | . | . | . |
| 21-225_152G4_LC | F | . | . | . | . | . | - | . | . | . | . | . | . | . | . | . | . | . | . | . | . | . | . | . | . | . |
| 21-225_155C4_LC | . | . | . | . | . | . | - | . | . | . | . | . | . | . | . | . | . | . | . | . | . | . | . | . | . | . |
| 21-225_156E4_LC | . | . | . | . | . | . | - | . | . | . | . | . | . | . | . | . | . | . | . | . | . | . | . | . | . | . |
| 21-225_159H2_LC | . | . | . | . | . | . | . | . | . | . | . | . | . | . | . | . | . | . | . | . | . | . | . | . | . | . |
| 21-225_15A3_LC | . | . | . | . | T | . | . | . | . | . | . | . | . | . | . | . | . | . | . | . | . | . | . | . | . | . |
| 21-225_16D11_LC | . | . | . | . | . | H | . | . | . | . | . | . | . | . | . | . | . | . | . | . | . | . | . | . | . | . |
| 21-225_16E6_LC | . | . | . | . | T | . | . | . | . | . | . | . | . | . | . | . | . | . | . | . | . | . | . | . | . | . |
| 21-225_17A6_LC | . | . | . | . | . | H | T | . | . | . | . | . | . | . | . | . | . | . | . | . | . | . | . | . | . | . |
| 21-225_17G4_LC | . | . | . | . | . | N | F | . | . | . | . | . | . | . | . | . | . | . | . | . | . | . | . | . | . | . |
| 21-225_18G4_LC | . | . | . | . | T | . | . | . | . | . | . | . | . | . | . | . | . | . | . | . | . | . | . | . | . | . |
| 21-225_196F4_LC | . | . | . | . | . | . | G | . | . | . | . | . | . | . | . | . | . | . | . | . | . | . | . | . | . | . |
| 21-225_19A1_LC | . | . | . | . | . | . | N | . | . | . | . | . | . | . | . | . | . | . | . | . | . | . | . | . | . | . |
| 21-225_1H12_LC | . | . | . | . | . | . | . | . | . | . | . | . | . | . | . | . | . | . | . | . | . | . | . | . | . | . |
| 21-225_201H10_LC | . | . | . | . | . | . | . | . | . | . | . | . | . | . | . | . | . | . | . | . | . | . | . | . | . | . |
| 21-225_203E3_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | S | Y | S | - | - | - | - | - | - | - | - | - | - | - | - | - | - | - | - | - | - | - |
| 21-225_204D6_LC | . | . | . | . | . | . | . | . | . | . | . | . | . | . | . | . | . | . | . | . | . | . | . | . | . | . |
| 21-225_204H4_LC | . | . | . | . | . | . | . | . | . | . | . | . | . | . | . | . | . | . | . | . | . | . | . | . | . | . |
| 21-225_206A4_LC | . | . | . | . | . | . | . | . | . | . | . | . | . | . | . | . | . | . | . | . | . | . | . | . | . | . |
| 21-225_206A5_LC | . | . | . | . | . | . | . | . | . | . | . | . | . | . | . | . | . | . | . | . | . | . | . | . | . | . |
| 21-225_207C9_LC | . | . | . | . | . | . | . | . | . | . | . | . | . | . | . | . | . | . | . | . | . | . | . | . | . | . |
| 21-225_208B11_LC | . | . | . | . | . | . | . | . | . | . | . | . | . | . | . | . | . | . | . | . | . | . | . | . | . | . |
| 21-225_208E8_LC | . | . | . | . | . | . | . | . | . | . | . | . | . | . | . | . | . | . | . | . | . | . | . | . | . | . |
| 21-225_20F6_LC | . | . | . | . | T | . | . | . | . | . | . | . | . | . | . | . | . | . | . | . | . | . | . | . | . | . |
| 21-225_210D11_LC | . | . | . | . | . | . | . | . | . | . | . | . | . | . | . | . | . | . | . | . | . | . | . | . | . | . |
| 21-225_22C7_LC | . | . | . | . | T | . | . | . | . | . | . | . | . | . | . | . | . | . | . | . | . | . | . | . | . | . |
| 21-225_30G1_LC | . | . | . | . | . | . | . | . | . | . | . | . | . | . | . | . | . | . | . | . | . | . | . | . | . | . |
| 21-225_30G4_LC | . | . | . | L | . | . | . | . | . | . | . | . | . | . | . | . | . | . | . | . | . | . | . | . | . | . |
| 21-225_31F1_LC | . | . | . | . | . | . | I | . | . | . | . | . | . | . | . | . | . | . | . | . | . | . | . | . | . | . |
| 21-225_32D6_LC | . | . | . | . | . | . | . | . | . | . | . | . | . | . | . | . | . | . | . | . | . | . | . | . | . | . |
| 21-225_33B2_LC | . | . | . | . | . | . | . | . | . | . | . | . | . | . | . | . | . | . | . | . | . | . | . | . | . | . |
| 21-225_33C12_LC | . | . | . | . | . | . | . | . | . | . | . | . | . | . | . | . | . | . | . | . | . | . | . | . | . | . |
| 21-225_34C11_LC | . | . | . | . | T | C | . | . | . | . | . | . | . | . | . | . | . | . | . | . | . | . | . | . | . | . |
| 21-225_34C2_LC | . | . | . | . | . | . | . | . | . | . | . | . | . | . | . | . | . | . | . | . | . | . | . | . | . | . |
| 21-225_34D3_LC | . | . | . | . | . | . | . | . | . | . | . | . | . | . | . | . | . | . | . | . | . | . | . | . | . | . |
| 21-225_34D8_LC | . | . | . | . | . | . | . | . | . | . | . | . | . | . | . | . | . | . | . | . | . | . | . | . | . | . |
| 21-225_34G11_LC | . | . | . | . | T | . | I | . | . | . | . | . | . | . | . | . | . | . | . | . | . | . | . | . | . | . |
| 21-225_36B8_LC | . | . | . | . | . | . | . | . | . | . | . | . | . | . | . | . | . | . | . | . | . | . | . | . | . | . |
| 21-225_3F8_LC | . | . | . | . | T | . | . | . | . | . | . | . | . | . | . | . | . | . | . | . | . | . | . | . | . | . |
| 21-225_3G7_LC | . | . | . | . | . | . | . | . | . | . | . | . | . | . | . | . | . | . | . | . | . | . | . | . | . | . |
| 21-225_48B10_LC | . | . | . | . | T | . | . | . | . | . | . | . | . | . | . | . | . | . | . | . | . | . | . | . | . | . |
| 21-225_48F12_LC | . | . | . | . | T | . | . | . | . | . | . | . | . | . | . | . | . | . | . | . | . | . | . | . | . | . |
| 21-225_48G12_LC | . | . | . | . | T | . | . | . | . | . | . | . | . | . | . | . | . | . | . | . | . | . | . | . | . | . |
| 21-225_4G4_LC | . | . | . | . | T | . | . | . | . | . | . | . | . | . | . | . | . | . | . | . | . | . | . | . | . | . |
| 21-225_50F2_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | S | Y | S | - | - | - | - | - | - | - | - | - | - | - | - | - | - | - | - | - | - | - |
| 21-225_56B6_LC | . | . | . | . | T | . | . | . | . | . | . | . | . | . | . | . | . | . | . | . | . | . | . | . | . | . |
| 21-225_62D2_LC | . | . | . | . | T | . | . | . | . | . | . | . | . | . | . | . | . | . | . | . | . | . | . | . | . | . |
| 21-225_63C10_LC | . | . | . | . | T | . | . | . | . | . | . | . | . | . | . | . | . | . | . | . | . | . | . | . | . | . |
| 21-225_64A11_LC | F | . | . | . | . | D | . | . | . | . | . | . | . | . | . | . | . | . | . | . | . | . | . | . | . | . |
| 21-225_64F7_LC | . | . | . | . | . | . | N | . | . | . | . | . | . | . | . | . | . | . | . | . | . | . | . | . | . | . |
| 21-225_6G1_LC | . | . | . | . | F | H | . | . | . | . | . | . | . | . | . | . | . | . | . | . | . | . | . | . | . | . |
| 21-225_7A10_LC | . | . | . | . | . | . | . | . | . | . | . | . | . | . | . | . | . | . | . | . | . | . | . | . | . | . |
| 21-225_7E11_LC | . | . | . | . | T | . | . | . | . | . | . | . | . | . | . | . | . | . | . | . | . | . | . | . | . | . |
| 21-225_7E11.001_LC | . | . | . | . | T | . | . | . | . | . | . | . | . | . | . | . | . | . | . | . | . | . | . | . | . | . |
| 21-225_7E11.001.001_LC | . | . | . | . | T | . | . | . | . | . | . | . | . | . | . | . | . | . | . | . | . | . | . | . | . | . |
| 21-225_7E11.001.002_LC | . | . | . | . | T | . | . | . | . | . | . | . | . | . | . | . | . | . | . | . | . | . | . | . | . | . |
| 21-225_7E11.001.003_LC | . | . | . | . | T | . | . | . | . | . | . | . | . | . | . | . | . | . | . | . | . | . | . | . | . | . |
| 21-225_7E11.001.004_LC | . | . | . | . | T | . | . | . | . | . | . | . | . | . | . | . | . | . | . | . | . | . | . | . | . | . |
| 21-225_7E11.001.005_LC | . | . | . | . | T | . | . | . | . | . | . | . | . | . | . | . | . | . | . | . | . | . | . | . | . | . |
| 21-225_7E11.001.006_LC | . | . | . | . | T | . | . | . | . | . | . | . | . | . | . | . | . | . | . | . | . | . | . | . | . | . |
| 21-225_7E11.001.007_LC | . | . | . | . | T | . | . | . | . | . | . | . | . | . | . | . | . | . | . | . | . | . | . | . | . | . |
| 21-225_7E11.001.008_LC | . | . | . | . | T | . | . | . | . | . | . | . | . | . | . | . | . | . | . | . | . | . | . | . | . | . |
| 21-225_7E11.001.009_LC | . | . | . | . | T | . | . | . | . | . | . | . | . | . | . | . | . | . | . | . | . | . | . | . | . | . |
| 21-225_7E11.001.010_LC | . | . | . | . | T | . | . | . | . | . | . | . | . | . | . | . | . | . | . | . | . | . | . | . | . | . |
| 21-225_7E11.001.011_LC | . | . | . | . | T | . | . | . | . | . | . | . | . | . | . | . | . | . | . | . | . | . | . | . | . | . |
| 21-225_7E11.001.012_LC | . | . | . | . | T | . | . | . | . | . | . | . | . | . | . | . | . | . | . | . | . | . | . | . | . | . |
| 21-225_7E11.001.013_LC | . | . | . | . | T | . | . | . | . | . | . | . | . | . | . | . | . | . | . | . | . | . | . | . | . | . |
| 21-225_7E11.001.014_LC | . | . | . | . | T | . | . | . | . | . | . | . | . | . | . | . | . | . | . | . | . | . | . | . | . | . |
| 21-225_7E11.001.015_LC | . | . | . | . | T | . | . | . | . | . | . | . | . | . | . | . | . | . | . | . | . | . | . | . | . | . |
| 21-225_7E11.001.016_LC | . | . | . | . | T | . | . | . | . | . | . | . | . | . | . | . | . | . | . | . | . | . | . | . | . | . |
| 21-225_7E11.001.017_LC | . | . | . | . | T | . | . | . | . | . | . | . | . | . | . | . | . | . | . | . | . | . | . | . | . | . |
| 21-225_7E11.001.018_LC | . | . | . | . | T | . | . | . | . | . | . | . | . | . | . | . | . | . | . | . | . | . | . | . | . | . |
| 21-225_7E11.001.019_LC | . | . | . | . | T | . | . | . | . | . | . | . | . | . | . | . | . | . | . | . | . | . | . | . | . | . |
| 21-225_7E11.001.021_LC | . | . | . | . | T | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | S | Y | S | - | - | - | - | - | - | - | - | - | - | - | - | - | - | - | - | - | - | - |
| 21-225_7E11.001.022_LC | . | . | . | . | T | . | . | . | . | . | . | . | . | . | . | . | . | . | . | . | . | . | . | . | . | . |
| 21-225_7E11.001.023_LC | . | . | . | . | T | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | T | P | L | T | F | G | G | G | T | K | V | E | I | K | R |
| 21-225_10E12_LC | . | . | . | . | P | . | . | . | . | . | . | . | . | . | . | . | . | E | . |
| 21-225_10F5_LC | . | . | . | . | P | . | . | . | . | . | . | . | . | . | . | . | . | . | . |
| 21-225_12A10_LC | . | . | . | . | P | . | . | . | . | . | . | . | . | . | . | . | . | . | . |
| 21-225_13D4_LC | . | . | . | . | L | . | . | . | . | . | . | . | . | . | . | . | . | . | . |
| 21-225_146E9_LC | . | . | . | . | S | T | P | . | . | . | . | . | . | . | . | . | . | . | . |
| 21-225_146H1_LC | . | . | . | . | S | T | P | . | . | . | . | . | . | . | . | . | . | . | . |
| 21-225_148C4_LC | . | . | . | . | S | T | P | . | . | . | . | . | . | . | . | . | . | E | . |
| 21-225_148H9_LC | . | . | . | . | S | T | P | . | . | . | . | . | . | . | . | . | . | . | . |
| 21-225_149A3_LC | . | . | . | . | S | I | P | . | . | . | . | . | . | . | . | . | . | . | . |
| 21-225_150C11_LC | . | . | . | . | S | I | Y | . | . | . | . | . | S | . | . | . | . | . | . |
| 21-225_150C12_LC | . | . | . | . | S | T | P | . | . | . | . | . | . | . | . | . | . | . | . |
| 21-225_151B12_LC | . | . | . | . | S | T | P | . | . | . | . | . | . | R | . | . | . | . | . |
| 21-225_152B11_LC | . | . | . | . | S | T | P | . | . | . | . | . | . | . | . | . | . | . | . |
| 21-225_152D2_LC | . | . | . | . | S | T | P | . | . | . | . | . | . | . | . | . | . | . | . |
| 21-225_152G4_LC | . | . | . | . | S | T | P | . | . | . | . | . | . | . | . | . | . | . | . |
| 21-225_155C4_LC | . | . | . | . | S | T | P | . | . | . | . | . | . | R | . | . | . | . | . |
| 21-225_156E4_LC | . | . | . | . | S | A | P | . | . | . | . | . | . | . | . | . | . | . | . |
| 21-225_159H2_LC | . | . | . | . | L | . | V | . | . | . | . | . | . | . | . | . | . | . | . |
| 21-225_15A3_LC | . | . | . | . | . | . | . | . | . | . | . | . | . | . | . | . | . | . | . |
| 21-225_16D11_LC | . | . | . | . | S | . | . | . | . | . | . | . | . | . | . | . | . | . | . |
| 21-225_16E6_LC | . | . | . | . | . | . | . | . | . | . | . | . | . | . | . | . | . | . | . |
| 21-225_17A6_LC | . | . | . | . | S | . | . | . | . | . | . | . | . | . | . | . | . | . | . |
| 21-225_17G4_LC | . | . | . | . | P | . | . | . | . | . | . | . | . | . | . | . | . | I | . |
| 21-225_18G4_LC | . | . | . | . | P | . | . | . | . | . | . | . | . | . | . | . | . | . | . |
| 21-225_196F4_LC | . | . | . | . | S | . | . | . | . | . | . | . | . | . | . | . | . | . | . |
| 21-225_19A1_LC | . | . | . | . | P | . | . | . | . | . | . | . | . | . | . | . | . | . | . |
| 21-225_1H12_LC | . | . | . | . | P | . | . | . | . | . | . | . | . | . | . | . | . | . | . |
| 21-225_201H10_LC | . | . | . | . | P | . | . | . | . | . | . | . | . | . | . | . | M | R | . |
| 21-225_203E3_LC | . | . | . | . | P | . | . | . | . | . | . | . | . | . | . | . | M | R | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | T | P | L | T | F | G | G | G | T | K | V | E | I | K | R |
| 21-225_204D6_LC | . | . | . | . | S | . | . | . | . | . | . | . | . | . | . | . | . | Q | . |
| 21-225_204H4_LC | . | . | . | . | F | . | . | . | . | . | . | . | . | . | . | . | M | R | . |
| 21-225_206A4_LC | . | . | . | . | F | . | . | . | . | . | . | . | . | . | . | . | M | R | . |
| 21-225_206A5_LC | . | . | . | . | F | . | . | . | . | . | . | . | . | . | . | . | M | R | . |
| 21-225_207C9_LC | . | . | . | . | I | . | . | . | . | . | . | . | . | . | . | . | . | . | . |
| 21-225_208B11_LC | . | . | . | . | F | . | . | . | . | . | . | . | . | . | . | . | M | R | . |
| 21-225_208E8_LC | . | . | . | . | F | . | . | . | . | . | . | . | . | . | . | . | M | R | . |
| 21-225_20F6_LC | . | . | . | . | P | . | . | . | . | . | . | . | . | . | . | . | . | . | . |
| 21-225_210D11_LC | . | . | . | . | F | . | . | . | . | . | . | . | . | . | . | . | M | R | . |
| 21-225_22C7_LC | . | . | . | . | . | . | . | . | . | . | . | . | . | . | . | . | . | . | . |
| 21-225_30G1_LC | . | . | . | . | . | . | . | . | . | . | . | . | . | . | . | . | . | . | . |
| 21-225_30G4_LC | . | . | . | . | P | . | Y | . | . | . | . | . | . | . | . | . | . | . | . |
| 21-225_31F1_LC | . | . | . | . | . | . | . | . | . | . | . | . | . | T | . | . | . | R | . |
| 21-225_32D6_LC | . | . | . | . | . | . | Y | . | . | . | . | . | . | . | . | A | . | . | . |
| 21-225_33B2_LC | . | . | . | . | . | . | Y | . | . | . | . | . | . | . | . | . | . | . | . |
| 21-225_33C12_LC | . | . | . | . | . | . | Y | . | . | . | . | . | . | . | . | . | . | . | . |
| 21-225_34C11_LC | . | . | . | . | . | . | . | . | . | . | . | . | . | . | . | . | . | . | . |
| 21-225_34C2_LC | . | . | . | . | . | . | Y | . | . | . | . | . | . | . | M | . | . | . | . |
| 21-225_34D3_LC | . | . | . | . | . | . | . | . | . | . | . | . | . | . | . | . | . | . | . |
| 21-225_34D8_LC | . | . | . | . | . | . | Y | . | . | . | . | . | . | . | . | . | . | . | . |
| 21-225_34G11_LC | . | . | . | . | . | . | . | . | . | . | . | . | . | . | . | . | . | . | . |
| 21-225_36B8_LC | . | . | . | . | . | . | Y | . | . | . | . | . | . | . | M | . | . | . | . |
| 21-225_3F8_LC | . | . | . | . | . | . | . | . | . | . | . | . | . | R | . | . | . | N | . |
| 21-225_3G7_LC | . | . | . | . | P | . | F | . | . | . | . | . | A | . | . | . | . | D | . |
| 21-225_48B10_LC | . | . | . | . | N | . | . | . | . | . | . | . | . | . | . | . | F | T | . |
| 21-225_48F12_LC | . | . | . | . | N | . | . | . | . | . | . | . | . | E | . | . | . | T | . |
| 21-225_48G12_LC | . | . | . | . | N | . | . | . | . | . | . | . | . | E | . | . | . | T | . |
| 21-225_4G4_LC | . | . | . | . | . | . | . | . | . | . | . | . | . | . | . | . | . | . | . |
| 21-225_50F2_LC | . | . | . | . | P | . | . | . | . | . | . | . | . | . | . | . | . | . | . |

# FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | T | P | L | T | F | G | G | G | T | K | V | E | I | K | R |
| 21-225_56B6_LC | . | . | . | . | P | . | . | . | . | . | . | . | . | . | . | . | . | . | . |
| 21-225_62D2_LC | . | . | . | . | P | . | . | . | . | . | . | . | . | . | . | . | . | . | . |
| 21-225_63C10_LC | . | . | . | . | P | . | . | . | . | . | . | . | . | . | . | . | . | . | . |
| 21-225_64A11_LC | . | . | . | . | F | . | . | . | . | . | . | . | . | . | . | . | . | . | . |
| 21-225_64F7_LC | . | . | . | . | I | S | F | . | . | . | . | . | . | . | . | . | L | . | . |
| 21-225_6G1_LC | . | . | . | . | P | . | . | . | . | . | . | . | . | . | . | . | . | . | . |
| 21-225_7A10_LC | . | . | . | . | P | . | . | . | . | . | . | . | . | . | . | . | . | R | . |
| 21-225_7E11_LC | . | . | . | . | . | . | . | . | . | . | . | . | . | . | . | . | . | . | . |
| 21-225_7E11.001_LC | . | . | . | . | . | . | . | . | . | . | . | . | . | . | . | . | . | . | . |
| 21-225_7E11.001.001_LC | . | . | . | . | . | . | . | . | . | . | . | . | . | . | . | . | . | . | . |
| 21-225_7E11.001.002_LC | . | . | . | . | . | . | . | . | . | . | . | . | . | . | . | . | . | . | . |
| 21-225_7E11.001.003_LC | . | . | . | . | . | . | . | . | . | . | . | . | . | . | . | . | . | . | . |
| 21-225_7E11.001.004_LC | . | . | . | . | . | . | . | . | . | . | . | . | . | . | . | . | . | . | . |
| 21-225_7E11.001.005_LC | . | . | . | . | . | . | . | . | . | . | . | . | . | . | . | . | . | . | . |
| 21-225_7E11.001.006_LC | . | . | . | . | . | . | . | . | . | . | . | . | . | . | . | . | . | . | . |
| 21-225_7E11.001.007_LC | . | . | . | . | . | . | . | . | . | . | . | . | . | . | . | . | . | . | . |
| 21-225_7E11.001.008_LC | . | . | . | . | . | . | . | . | . | . | . | . | . | . | . | . | . | . | . |
| 21-225_7E11.001.009_LC | . | . | . | . | . | . | . | . | . | . | . | . | . | . | . | . | . | . | . |
| 21-225_7E11.001.010_LC | . | . | . | . | . | . | . | . | . | . | . | . | . | . | . | . | . | . | . |
| 21-225_7E11.001.011_LC | . | . | . | . | . | . | . | . | . | . | . | . | . | . | . | . | . | . | . |
| 21-225_7E11.001.012_LC | . | . | . | . | . | . | . | . | . | . | . | . | . | . | . | . | . | . | . |
| 21-225_7E11.001.013_LC | . | . | . | . | . | . | . | . | . | . | . | . | . | . | . | . | . | . | . |
| 21-225_7E11.001.014_LC | . | . | . | . | . | . | . | . | . | . | . | . | . | . | . | . | . | . | . |
| 21-225_7E11.001.015_LC | . | . | . | . | . | . | . | . | . | . | . | . | . | . | . | . | . | . | . |
| 21-225_7E11.001.016_LC | . | . | . | . | . | . | . | . | . | . | . | . | . | . | . | . | . | . | . |
| 21-225_7E11.001.017_LC | . | . | . | . | . | . | . | . | . | . | . | . | . | . | . | . | . | . | . |
| 21-225_7E11.001.018_LC | . | . | . | . | . | . | . | . | . | . | . | . | . | . | . | . | . | . | . |
| 21-225_7E11.001.019_LC | . | . | . | . | . | . | . | . | . | . | . | . | . | . | . | . | . | . | . |
| 21-225_7E11.001.021_LC | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | T | P | L | T | F | G | G | G | T | K | V | E | I | K | R |
| 21-225_7E11.001.022_LC | . | . | . | . | . | . | . | . | . | . | . | . | . | . | . | . | . | . | . |
| 21-225_7E11.001.023_LC | . | . | . | . | . | . | . | . | . | . | . | . | . | . | . | . | . | . | . |

**FIGURE 57**

Table 103. Consensus 23 - VK1|L1/JK3 (SEQ ID NO: 50320):

DIQMTQSPSSLSASVGDRVTITCRAS--QGIS------NYLAWFQQKPGKAPKSLIYA--------ASSLQSGVPSKFSGSGSG--TDFTLTISSLQPEDFATYYCQQYNS----------------------YPFTFGPGTKVDIKR wherein:

R at position 24 can be substituted with P

A at position 25 can be substituted with T

S at position 26 can be substituted with N

Q at position 29 can be substituted with R

G at position 30 can be substituted with D, V or A

I at position 31 can be substituted with V

S at position 32 can be substituted with N, G, R, T or K

N at position 39 can be substituted with K, Y, H, I or T

Y at position 40 can be substituted with H

A at position 42 can be substituted with V or S

A at position 58 can be substituted with V, G or T

A at position 67 can be substituted with S or V

S at position 68 can be substituted with F

S at position 69 can be substituted with G, N or T

L at position 70 can be substituted with V

Q at position 71 can be substituted with R, H, L or E

S at position 72 can be substituted with G or T

**FIGURE 57**

Q at position 107 can be substituted with H, L, P or R

Q at position 108 can be substituted with R, K, H or L

Y at position 109 can be substituted with F

N at position 110 can be substituted with H, D, Y, S, K, L, M or Q

S at position 111 can be substituted with T, G, N, C or D

Y at position 135 can be substituted with F or H

F at position 137 can be substituted with V, L or I

T at position 138 can be substituted with K

Xaa Xaa Xaa Xaa Xaa Xaa Xaa Xaa Xaa Leu Xaa (SEQ ID NO: 50144)

Xaa Xaa Xaa Xaa Xaa Xaa Xaa (SEQ ID NO: 50145)

Xaa Xaa Xaa Xaa Xaa Xaa Pro Xaa Xaa (SEQ ID NO: 50146)

Arg Ala Ser Gln Gly Ile Ser Asn Tyr Leu Ala (SEQ ID NO: 50630)

Ala Ala Ser Ser Leu Gln Ser (SEQ ID NO: 50631)

Gln Gln Tyr Asn Ser Tyr Pro Phe Thr (SEQ ID NO: 50632)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_148B6_LC | . | . | . | . | . | . | . | . | . | . | . | . | . | . | . | . | . | . | . | S | . | . | . | . | . | . |
| 21-225_14H8_LC | . | . | . | . | . | . | . | . | . | . | . | . | . | . | . | . | . | . | . | . | . | . | . | . | . | . |
| 21-225_153A1_LC | . | . | . | . | . | . | . | . | . | . | . | . | . | . | . | . | . | . | . | . | . | . | . | . | . | . |
| 21-225_157E4_LC | . | . | . | . | . | . | . | . | . | . | . | . | . | . | . | . | . | . | . | . | F | . | . | . | . | . |
| 21-225_157G5_LC | . | . | . | . | . | . | . | . | . | . | . | . | . | . | . | . | . | . | . | . | . | . | . | . | . | . |
| 21-225_157H1_LC | . | . | . | . | . | . | . | . | . | . | . | . | . | . | . | . | . | . | . | . | . | . | . | . | . | . |
| 21-225_157H10_LC | . | . | . | . | . | . | . | . | . | . | . | . | . | . | . | . | . | . | . | . | . | . | . | . | . | . |
| 21-225_158H12_LC | . | . | . | . | . | . | . | . | . | . | . | . | . | . | . | . | . | . | . | . | . | . | . | . | . | . |
| 21-225_159H11_LC | . | . | . | . | . | . | . | . | . | . | . | . | . | . | . | . | . | . | . | . | . | . | . | . | . | . |
| 21-225_15A2_LC | . | . | . | . | . | . | . | . | . | . | . | P | . | P | I | . | . | . | . | . | . | . | . | . | . | . |
| 21-225_15B1_LC | . | . | . | . | . | . | . | . | . | . | . | . | . | . | . | . | . | . | . | . | . | . | . | . | . | . |
| 21-225_160G1_LC | . | . | . | . | . | . | . | . | . | . | . | . | . | . | . | . | . | . | . | . | . | . | . | . | . | . |
| 21-225_160G3_LC | . | . | . | . | . | . | . | . | . | . | . | . | . | . | . | . | . | . | . | . | . | . | . | . | . | . |
| 21-225_163F1_LC | . | . | . | . | . | . | . | . | . | . | . | . | . | . | . | . | . | . | . | . | . | . | . | . | . | . |
| 21-225_167D12_LC | . | . | . | . | . | . | . | . | . | . | . | . | . | . | . | . | . | . | . | . | . | . | . | . | . | . |
| 21-225_169D10_LC | . | . | . | . | . | . | . | . | . | . | . | . | . | . | . | . | . | . | . | . | . | . | . | . | . | . |
| 21-225_16B8_LC | . | . | . | . | . | . | . | . | . | . | . | . | . | . | . | . | . | . | . | . | . | . | . | . | . | . |
| 21-225_16C7_LC | . | . | . | . | . | . | . | . | . | . | . | . | . | . | . | . | . | . | . | . | . | . | . | . | . | . |
| 21-225_16G12_LC | . | . | . | . | . | . | . | . | . | . | . | . | . | . | . | . | . | . | . | . | . | . | . | . | . | . |
| 21-225_170E1_LC | . | . | . | . | . | . | . | . | . | . | . | . | . | . | E | . | . | . | . | . | . | . | . | . | . | . |
| 21-225_175C4_LC | . | . | . | . | . | . | . | . | . | . | . | . | . | . | . | . | . | . | . | . | . | . | . | . | . | . |
| 21-225_177D3_LC | . | . | . | . | S | . | . | . | . | . | . | . | . | . | . | . | . | . | . | . | . | . | . | . | . | . |
| 21-225_17H5_LC | . | . | . | . | . | . | . | . | . | . | . | . | . | . | . | . | . | . | . | . | . | . | . | . | . | . |
| 21-225_17H6_LC | . | . | . | . | . | . | . | . | . | . | . | . | . | . | . | . | . | . | . | . | . | . | . | . | . | . |
| 21-225_181G2_LC | . | . | . | . | . | . | . | . | . | . | . | . | . | . | I | . | E | . | . | . | . | . | . | . | . | . |
| 21-225_184E7_LC | . | . | . | . | . | . | . | . | . | . | R | . | . | . | I | . | E | . | . | . | . | . | . | . | . | . |
| 21-225_185A1_LC | . | . | . | . | . | . | . | . | . | . | . | . | . | . | . | . | . | . | . | . | . | . | . | . | . | . |
| 21-225_185E10_LC | . | . | . | . | . | . | . | . | . | . | R | . | . | . | I | . | E | . | . | . | . | . | . | . | . | . |
| 21-225_188B9_LC | . | . | . | . | . | . | . | . | . | . | . | . | . | . | I | . | E | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_188H5_LC | . | . | . | . | . | . | . | . | . | . | . | . | . | . | I | . | E | . | . | . | . | . | . | . | . | . |
| 21-225_18D11_LC | . | . | . | . | . | . | . | . | . | . | . | P | . | P | . | . | . | . | . | . | . | . | . | . | . | . |
| 21-225_18G10_LC | . | . | . | . | . | . | . | . | . | . | . | . | . | . | . | . | . | . | . | . | . | . | . | . | . | . |
| 21-225_190A5_LC | . | . | . | . | . | . | . | . | . | . | . | . | . | . | . | . | . | . | . | . | . | . | . | . | . | . |
| 21-225_190B1_LC | . | . | . | . | . | . | . | . | . | . | . | . | . | . | . | . | . | . | . | . | . | . | . | . | . | . |
| 21-225_190F12_LC | . | . | . | . | . | . | . | . | . | . | . | . | . | . | . | . | . | . | . | . | . | . | . | . | . | . |
| 21-225_190H9_LC | . | . | . | . | . | . | . | . | . | . | . | . | . | . | . | . | . | . | . | . | . | . | . | . | . | . |
| 21-225_192A2_LC | . | . | . | . | . | . | . | . | . | . | . | . | . | . | . | . | . | . | . | . | . | . | . | . | . | N |
| 21-225_192C10_LC | . | . | . | . | . | . | . | . | . | . | . | . | . | . | . | . | . | . | . | . | . | . | . | . | . | . |
| 21-225_192E4_LC | . | . | . | . | . | . | . | . | . | . | . | . | . | . | . | . | . | . | . | . | . | . | . | . | . | . |
| 21-225_192H6_LC | . | . | . | . | . | . | . | . | . | . | . | . | . | . | . | . | . | . | . | . | . | . | . | . | . | . |
| 21-225_193F8_LC | . | . | . | . | I | . | . | . | . | . | . | . | . | . | . | . | . | . | . | . | . | . | . | . | . | . |
| 21-225_194H12_LC | . | . | . | . | . | . | . | . | . | . | . | . | . | . | . | . | . | . | . | . | . | . | R | . | . | . |
| 21-225_197E11_LC | . | . | . | . | . | . | . | . | . | . | . | . | . | . | . | . | . | . | . | . | . | . | . | . | . | . |
| 21-225_20B9_LC | . | . | . | . | . | . | . | . | . | . | . | . | . | . | . | . | . | . | . | . | . | . | . | . | . | . |
| 21-225_20C11_LC | . | . | . | . | . | . | . | . | . | . | . | . | . | . | . | . | . | . | . | . | . | . | . | . | . | . |
| 21-225_20F10_LC | . | . | . | L | . | . | . | . | . | . | . | . | . | . | . | . | . | . | . | . | . | . | . | . | . | . |
| 21-225_20H7_LC | . | . | . | . | . | . | . | . | . | . | . | . | . | . | . | . | . | . | . | . | . | . | . | . | T | . |
| 21-225_20H9_LC | . | . | . | . | . | . | . | . | . | . | . | . | . | . | . | . | . | . | . | . | . | . | . | . | . | . |
| 21-225_211A6_LC | . | . | . | . | . | . | . | . | . | . | . | . | . | . | . | . | . | S | . | . | . | . | . | . | . | . |
| 21-225_213B3_LC | . | . | . | . | . | . | . | . | . | . | . | . | . | . | . | . | . | . | . | . | . | . | . | . | . | . |
| 21-225_214C3_LC | . | . | . | . | . | . | . | . | . | . | . | . | . | . | . | . | . | . | . | . | . | . | . | . | . | . |
| 21-225_214E4_LC | . | . | . | . | . | . | . | . | . | . | . | . | . | . | . | . | . | . | . | . | . | . | . | . | . | . |
| 21-225_223D11_LC | . | . | . | . | . | . | . | . | . | . | . | . | . | . | . | . | . | . | . | . | . | . | . | . | . | . |
| 21-225_22B12_LC | . | . | . | . | . | . | . | . | . | . | . | . | . | . | . | . | . | . | . | . | . | I | . | . | . | . |
| 21-225_23A11_LC | . | . | . | . | . | . | . | . | . | . | . | . | T | . | . | . | . | . | . | . | . | . | . | . | . | . |
| 21-225_23C7_LC | . | . | . | . | . | . | . | . | . | . | . | . | . | . | . | . | . | . | . | . | . | . | . | . | . | . |
| 21-225_23E7_LC | . | . | . | . | . | . | . | . | . | . | . | . | . | . | . | . | . | . | . | . | . | . | . | . | . | . |
| 21-225_23G1_LC | . | . | . | . | . | . | . | . | . | . | . | . | . | . | . | . | . | . | . | . | . | . | . | . | . | . |

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_23G8_LC | . | . | . | . | . | . | . | . | . | . | . | . | . | . | . | . | . | . | . | . | . | . | . | . | . | . |
| 21-225_25C10_LC | . | . | . | . | . | . | . | . | . | . | . | . | . | . | . | . | . | . | . | . | . | . | . | . | . | . |
| 21-225_26G1_LC | . | . | . | . | . | . | . | . | . | . | . | . | . | . | . | . | . | . | . | . | . | . | . | . | . | . |
| 21-225_27H12_LC | . | . | . | . | . | . | . | . | . | . | . | . | . | . | . | . | . | . | . | . | . | . | . | . | . | . |
| 21-225_2A4_LC | . | . | . | . | . | . | . | . | . | . | . | P | . | . | . | . | . | . | . | . | . | . | . | . | . | . |
| 21-225_30A1_LC | . | . | . | . | . | . | . | . | . | . | . | . | . | . | . | . | . | . | . | . | . | . | . | . | . | . |
| 21-225_32B3_LC | . | . | . | . | . | . | . | . | . | . | . | . | . | . | I | . | . | . | . | . | . | . | . | . | . | . |
| 21-225_33A5_LC | . | . | . | . | . | . | . | . | . | . | . | . | . | . | . | . | . | . | . | . | . | . | . | . | . | . |
| 21-225_35H8_LC | . | . | . | . | . | . | . | . | . | . | . | . | . | . | . | . | . | . | . | . | . | . | . | . | . | . |
| 21-225_43A4_LC | . | . | . | . | . | . | . | . | . | . | . | . | . | . | . | . | . | . | . | . | . | . | . | . | . | . |
| 21-225_45D9_LC | . | . | . | . | . | . | . | . | . | . | . | . | . | . | . | . | . | . | . | . | . | . | . | . | . | . |
| 21-225_51C5_LC | . | . | . | . | . | . | . | . | . | . | . | . | . | . | . | . | . | . | . | . | . | . | . | . | . | . |
| 21-225_51D5_LC | . | . | . | . | . | . | . | . | . | . | . | . | . | . | . | . | . | . | . | . | . | . | . | . | . | . |
| 21-225_52E3_LC | . | . | . | . | . | . | . | . | . | . | . | . | . | . | . | . | . | . | . | . | . | . | . | . | . | . |
| 21-225_53E4_LC | . | . | . | . | . | . | . | . | . | . | . | . | . | . | . | . | . | . | . | . | . | . | . | . | . | . |
| 21-225_56F7_LC | . | . | L | . | . | . | . | . | . | . | . | . | . | . | . | . | . | . | . | . | . | . | . | . | . | . |
| 21-225_56H1_LC | . | . | . | . | . | . | . | . | . | . | . | . | . | . | . | . | . | . | . | . | . | . | . | . | . | . |
| 21-225_5A5_LC | . | . | . | . | . | . | . | . | . | . | . | . | . | . | . | . | . | . | . | . | . | . | . | . | . | . |
| 21-225_5E11_LC | V | . | . | . | . | . | . | . | . | . | . | . | . | . | . | . | . | . | . | . | . | . | . | . | . | . |
| 21-225_60A4_LC | . | . | . | . | . | . | . | . | . | . | . | . | . | . | . | . | . | . | . | . | . | . | . | . | . | . |
| 21-225_60F7_LC | . | . | . | . | . | . | . | . | . | . | . | . | . | . | . | . | . | . | . | . | . | S | . | . | . | . |
| 21-225_63H8_LC | . | . | . | . | . | . | . | . | . | . | . | . | . | . | . | . | . | . | . | . | . | . | . | . | . | . |
| 21-225_65D5_LC | . | . | . | . | . | . | . | . | . | . | . | . | . | . | . | . | . | . | . | . | . | . | . | . | . | . |
| 21-225_66H11_LC | . | . | . | . | . | . | P | . | . | . | . | P | . | P | . | . | . | . | . | . | . | . | . | P | . | . |
| 21-225_68E6_LC | . | . | . | . | . | . | . | . | . | . | . | . | . | . | . | . | . | . | . | . | . | . | . | . | . | . |
| 21-225_7B1_LC | . | . | . | . | . | . | A | . | . | . | . | . | . | . | . | . | . | . | . | . | . | . | . | . | . | . |
| 21-225_96C6_LC | . | . | . | . | . | . | . | . | . | . | . | . | . | . | . | . | . | . | . | . | . | . | . | . | . | . |
| 21-225_9C8_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | S | - | - | - | - | - | - | N | Y | L | A | W | F | Q | Q | K | P | G | K | A | P |
| 21-225_148B6_LC | . | . | . | . | . | . | . | . | . | . | . | . | K | . | . | . | . | . | . | . | . | . | . | . | . | . |
| 21-225_14H8_LC | . | . | . | . | . | . | . | . | . | . | . | . | Y | . | . | . | . | . | . | . | . | . | . | . | . | . |
| 21-225_153A1_LC | . | . | . | V | . | . | . | . | . | . | . | . | . | . | . | . | . | . | . | . | . | . | . | . | . | . |
| 21-225_157E4_LC | . | . | . | . | . | . | . | . | . | . | . | . | . | . | . | . | . | . | . | . | . | . | . | R | . | . |
| 21-225_157G5_LC | . | . | . | . | . | N | . | . | . | . | . | . | . | . | . | . | . | . | . | . | . | . | . | . | . | . |
| 21-225_157H1_LC | . | . | . | D | . | . | . | . | . | . | . | . | . | . | . | V | . | . | . | . | R | . | . | . | . | . |
| 21-225_157H10_LC | . | . | . | . | . | T | . | . | . | . | . | . | . | . | . | . | . | . | . | . | . | . | . | . | . | . |
| 21-225_158H12_LC | . | . | . | . | . | N | . | . | . | . | . | . | . | . | . | . | . | . | . | . | . | . | . | . | . | . |
| 21-225_159H11_LC | . | . | . | . | . | . | . | . | . | . | . | . | K | . | . | V | . | . | . | . | . | . | . | . | . | . |
| 21-225_15A2_LC | . | . | . | . | . | . | . | . | . | . | . | . | . | . | . | . | . | . | . | . | . | . | . | . | . | . |
| 21-225_15B1_LC | . | . | . | . | . | . | . | . | . | . | . | . | . | . | . | . | . | . | . | . | . | . | . | . | . | . |
| 21-225_160G1_LC | . | . | . | D | . | N | . | . | . | . | . | . | . | . | . | . | . | . | . | . | . | . | . | . | . | . |
| 21-225_160G3_LC | . | . | . | D | . | N | . | . | . | . | . | . | . | . | . | . | . | . | . | . | R | . | . | . | . | . |
| 21-225_163F1_LC | . | . | . | D | . | . | . | . | . | . | . | . | . | . | . | . | . | . | . | . | . | . | . | . | . | . |
| 21-225_167D12_LC | . | . | . | . | . | N | . | . | . | . | . | . | . | . | . | . | . | . | . | . | . | . | . | . | . | . |
| 21-225_169D10_LC | . | . | . | D | . | . | . | . | . | . | . | . | . | . | . | . | . | . | . | . | . | . | . | . | . | . |
| 21-225_16B8_LC | . | . | . | . | . | N | . | . | . | . | . | . | . | . | . | . | . | . | . | . | . | . | . | . | . | L |
| 21-225_16C7_LC | . | . | . | . | . | . | . | . | . | . | . | . | . | . | . | . | . | . | . | . | . | . | . | . | . | . |
| 21-225_16G12_LC | . | . | . | D | . | . | . | . | . | . | . | . | . | . | . | . | . | . | . | . | . | . | . | . | . | . |
| 21-225_170E1_LC | . | . | . | . | . | . | . | . | . | . | . | . | . | . | . | . | . | . | . | . | . | . | . | . | . | . |
| 21-225_175C4_LC | . | . | . | . | . | G | . | . | . | . | . | . | . | . | . | . | . | . | . | . | . | . | . | . | . | . |
| 21-225_177D3_LC | . | . | . | . | . | T | . | . | . | . | . | . | . | . | . | . | . | . | . | . | . | . | . | . | . | . |
| 21-225_17H5_LC | . | . | . | . | . | . | . | . | . | . | . | . | . | . | . | . | . | . | . | . | . | . | . | . | . | . |
| 21-225_17H6_LC | . | . | R | D | . | . | . | . | . | . | . | . | . | . | . | . | . | . | . | . | . | . | . | . | . | . |
| 21-225_181G2_LC | . | . | . | . | . | . | . | . | . | . | . | . | . | . | . | . | . | I | . | . | . | . | . | T | . | . |
| 21-225_184E7_LC | . | . | . | . | . | . | . | . | . | . | . | . | . | . | . | . | . | I | . | . | . | . | . | T | . | . |
| 21-225_185A1_LC | . | . | . | . | . | . | . | . | . | . | . | . | . | . | . | . | . | . | . | . | T | . | . | . | . | . |
| 21-225_185E10_LC | . | . | . | . | . | . | . | . | . | . | . | . | . | . | . | . | . | I | . | . | . | . | . | T | . | . |
| 21-225_188B9_LC | . | . | . | . | . | . | . | . | . | . | . | . | . | . | . | . | . | L | . | . | . | . | . | T | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | S | - | - | - | - | - | - | N | Y | L | A | W | F | Q | Q | K | P | G | K | A | P |
| 21-225_188H5_LC | . | . | . | . | . | . | . | . | . | . | . | . | . | . | . | . | . | L | . | . | . | . | . | T | . | . |
| 21-225_18D11_LC | . | . | . | . | . | . | . | . | . | . | . | . | Y | . | . | . | . | . | . | . | . | . | . | . | . | . |
| 21-225_18G10_LC | . | . | . | D | . | . | . | . | . | . | . | . | Y | . | . | . | . | . | . | . | . | . | . | . | . | . |
| 21-225_190A5_LC | . | . | . | . | . | G | . | . | . | . | . | . | . | H | . | . | . | . | . | . | . | . | . | . | . | . |
| 21-225_190B1_LC | . | . | . | . | . | R | . | . | . | . | . | . | . | . | . | . | . | . | . | . | . | . | . | . | . | . |
| 21-225_190F12_LC | . | . | . | . | . | G | . | . | . | . | . | . | K | . | . | . | . | . | . | . | . | . | . | . | . | . |
| 21-225_190H9_LC | . | . | . | . | . | G | . | . | . | . | . | . | K | . | . | . | . | . | . | . | . | . | . | . | . | L |
| 21-225_192A2_LC | . | . | . | . | . | N | . | . | . | . | . | . | . | . | . | . | . | . | . | . | . | . | . | . | . | . |
| 21-225_192C10_LC | . | . | . | . | . | G | . | . | . | . | . | . | K | . | . | . | . | . | . | . | . | . | . | . | . | . |
| 21-225_192E4_LC | . | . | . | . | . | G | . | . | . | . | . | . | . | . | . | . | . | . | . | . | . | . | . | . | . | . |
| 21-225_192H6_LC | . | . | . | . | . | G | . | . | . | . | . | . | . | H | . | . | . | . | . | . | . | . | . | . | . | . |
| 21-225_193F8_LC | . | . | . | . | V | . | . | . | . | . | . | . | . | H | . | . | . | . | . | . | . | . | . | R | . | . |
| 21-225_194H12_LC | . | . | . | . | . | G | . | . | . | . | . | . | K | . | . | . | . | . | . | . | . | . | . | . | . | L |
| 21-225_197E11_LC | . | . | . | . | . | N | . | . | . | . | . | . | K | . | . | S | . | . | . | . | . | . | . | . | . | . |
| 21-225_20B9_LC | . | . | . | . | . | . | . | . | . | . | . | . | . | . | . | . | . | . | . | . | . | . | . | . | . | . |
| 21-225_20C11_LC | . | . | . | D | . | . | . | . | . | . | . | . | . | . | . | . | . | . | . | . | . | . | . | . | . | . |
| 21-225_20F10_LC | . | . | . | . | . | N | . | . | . | . | . | . | . | . | . | . | . | . | . | . | . | . | . | . | . | . |
| 21-225_20H7_LC | . | . | . | . | . | N | . | . | . | . | . | . | . | . | . | V | . | . | . | . | . | . | . | . | . | . |
| 21-225_20H9_LC | . | . | . | . | . | . | . | . | . | . | . | . | . | . | . | . | . | . | . | . | . | . | . | . | . | . |
| 21-225_211A6_LC | . | . | . | . | . | G | . | . | . | . | . | . | K | . | . | . | . | . | . | . | . | . | . | . | . | . |
| 21-225_213B3_LC | . | . | . | . | . | G | . | . | . | . | . | . | K | . | . | . | . | . | . | . | . | . | . | . | . | . |
| 21-225_214C3_LC | . | . | . | . | . | G | . | . | . | . | . | . | . | . | . | . | . | . | . | . | . | . | . | . | V | . |
| 21-225_214E4_LC | . | . | . | . | . | . | . | . | . | . | . | . | . | . | . | . | . | . | . | . | . | . | . | . | . | . |
| 21-225_223D11_LC | . | . | . | . | . | . | . | . | . | . | . | . | . | . | . | . | . | . | . | . | . | . | . | . | . | . |
| 21-225_22B12_LC | . | . | . | D | . | . | . | . | . | . | . | . | . | . | . | . | . | . | . | E | . | . | . | . | . | H |
| 21-225_23A11_LC | . | . | . | D | . | . | . | . | . | . | . | . | . | . | . | . | . | . | . | . | . | . | . | . | . | . |
| 21-225_23C7_LC | . | . | . | . | . | G | . | . | . | . | . | . | I | . | . | . | . | . | . | . | . | . | . | . | . | . |
| 21-225_23E7_LC | . | . | . | . | . | . | . | . | . | . | . | . | . | . | . | . | . | . | . | . | I | . | . | . | . | . |
| 21-225_23G1_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | S | - | - | - | - | - | - | N | Y | L | A | W | F | Q | Q | K | P | G | K | A | P |
| 21-225_23G8_LC | . | . | . | . | . | R | . | . | . | . | . | . | . | . | . | . | . | . | . | . | . | . | . | . | . | . |
| 21-225_25C10_LC | . | . | . | . | . | . | . | . | . | . | . | . | . | . | . | . | . | . | . | . | . | . | . | . | . | . |
| 21-225_26G1_LC | . | . | . | D | . | . | . | . | . | . | . | . | . | . | . | . | . | . | . | . | . | . | . | . | . | . |
| 21-225_27H12_LC | . | . | . | . | . | . | . | . | . | . | . | . | . | . | . | . | . | . | . | . | . | . | . | . | . | . |
| 21-225_2A4_LC | . | . | . | . | . | . | . | . | . | . | . | . | . | . | . | . | . | . | . | . | . | . | . | . | . | . |
| 21-225_30A1_LC | . | . | . | . | . | . | . | . | . | . | . | . | . | . | . | . | . | . | . | . | . | . | . | . | . | . |
| 21-225_32B3_LC | . | . | . | . | . | . | . | . | . | . | . | . | . | . | . | . | . | . | . | . | . | . | . | . | . | . |
| 21-225_33A5_LC | . | . | . | . | . | . | . | . | . | . | . | . | . | . | . | . | . | . | . | . | . | . | . | . | . | . |
| 21-225_35H8_LC | . | . | . | A | . | . | . | . | . | . | . | . | . | . | . | . | . | . | . | . | . | . | . | . | . | . |
| 21-225_43A4_LC | . | . | . | . | . | N | . | . | . | . | . | . | . | . | . | . | . | . | . | . | . | . | . | . | . | . |
| 21-225_45D9_LC | . | . | . | . | . | N | . | . | . | . | . | . | . | . | . | . | . | . | . | . | . | . | . | . | . | . |
| 21-225_51C5_LC | . | . | . | . | . | . | . | . | . | . | . | . | . | . | . | . | . | . | . | . | . | . | . | E | . | . |
| 21-225_51D5_LC | . | . | . | . | . | N | . | . | . | . | . | . | . | . | . | . | . | . | . | . | . | . | . | E | . | . |
| 21-225_52E3_LC | . | . | . | . | . | . | . | . | . | . | . | . | . | . | . | . | . | . | . | . | . | . | . | . | . | . |
| 21-225_53E4_LC | . | . | . | V | . | . | . | . | . | . | . | . | . | . | . | . | . | . | . | . | . | . | . | . | . | . |
| 21-225_56F7_LC | . | . | . | . | . | . | . | . | . | . | . | . | T | . | . | . | . | . | . | . | T | . | . | T | . | . |
| 21-225_56H1_LC | . | . | . | . | . | . | . | . | . | . | . | . | H | . | . | . | . | . | . | . | . | . | . | . | . | . |
| 21-225_5A5_LC | . | . | . | . | . | . | . | . | . | . | . | . | . | . | . | . | . | . | . | . | . | . | . | . | . | . |
| 21-225_5E11_LC | . | . | . | . | . | . | . | . | . | . | . | . | . | . | . | . | . | . | . | . | . | . | . | . | . | . |
| 21-225_60A4_LC | . | . | . | . | . | . | . | . | . | . | . | . | . | . | . | . | . | . | . | . | . | . | . | . | . | . |
| 21-225_60F7_LC | . | . | . | V | . | K | . | . | . | . | . | . | . | . | . | V | . | V | . | . | . | . | . | . | . | . |
| 21-225_63H8_LC | . | . | . | . | . | . | . | . | . | . | . | . | . | . | . | . | . | . | . | . | . | . | . | . | . | H |
| 21-225_65D5_LC | . | . | . | D | . | N | . | . | . | . | . | . | . | . | . | . | . | . | . | . | . | . | . | . | . | . |
| 21-225_66H11_LC | . | . | . | . | . | . | . | . | . | . | . | . | . | . | . | . | . | . | . | . | . | . | . | . | . | . |
| 21-225_68E6_LC | . | . | . | . | . | . | . | . | . | . | . | . | Y | . | . | . | . | . | . | . | . | . | . | R | . | . |
| 21-225_7B1_LC | . | . | . | D | . | . | . | . | . | . | . | . | . | H | . | V | . | . | . | . | . | . | . | . | . | . |
| 21-225_96C6_LC | . | . | . | . | . | . | . | . | . | . | . | . | . | . | . | . | . | . | . | . | . | . | . | . | . | . |
| 21-225_9C8_LC | . | . | . | . | . | . | . | . | . | . | . | . | Y | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | S | L | I | Y | A | - | - | - | - | - | - | - | - | A | S | S | L | Q | S | G | V | P | S | K | F |
| 21-225_148B6_LC | . | . | . | . | F | . | . | . | . | . | . | . | . | . | . | . | . | . | . | . | . | . | . | . | . | . |
| 21-225_14H8_LC | . | . | . | . | . | V | . | . | . | . | . | . | . | . | . | . | . | . | . | . | . | . | . | . | . | . |
| 21-225_153A1_LC | . | . | . | . | S | . | . | . | . | . | . | . | . | . | . | . | . | . | R | . | . | . | . | . | N | . |
| 21-225_157E4_LC | . | . | . | . | . | . | . | . | . | . | . | . | . | . | . | . | . | . | R | . | . | . | . | . | Q | . |
| 21-225_157G5_LC | E | . | . | . | . | . | . | . | . | . | . | . | . | . | . | . | . | . | . | . | . | . | . | . | Q | . |
| 21-225_157H1_LC | R | . | . | . | . | . | . | . | . | . | . | . | . | . | . | . | . | . | . | . | . | . | . | . | . | . |
| 21-225_157H10_LC | . | . | . | . | . | T | . | . | . | . | . | . | . | . | . | . | N | . | . | . | . | . | . | . | . | . |
| 21-225_158H12_LC | . | . | . | . | S | . | . | . | . | . | . | . | . | . | . | . | . | . | . | . | . | . | . | . | . | . |
| 21-225_159H11_LC | . | . | . | . | . | . | . | . | . | . | . | . | . | . | . | . | . | . | . | . | . | . | . | . | . | . |
| 21-225_15A2_LC | . | . | . | . | F | . | . | . | . | . | . | . | . | . | . | . | . | . | . | . | . | . | . | . | . | . |
| 21-225_15B1_LC | . | . | . | . | S | . | . | . | . | . | . | . | . | . | . | . | . | . | . | . | . | . | . | . | N | . |
| 21-225_160G1_LC | T | . | . | . | . | . | . | . | . | . | . | . | . | . | S | . | . | . | . | . | . | . | . | . | . | . |
| 21-225_160G3_LC | T | . | . | . | . | . | . | . | . | . | . | . | . | . | S | . | . | . | . | . | . | . | . | . | . | . |
| 21-225_163F1_LC | . | . | . | . | . | . | . | . | . | . | . | . | . | . | . | . | . | . | . | . | . | . | . | . | . | . |
| 21-225_167D12_LC | E | . | . | . | . | . | . | . | . | . | . | . | . | . | . | . | . | . | . | . | . | . | . | . | . | . |
| 21-225_169D10_LC | . | . | . | . | . | . | . | . | . | . | . | . | . | . | . | . | . | . | . | . | . | . | . | . | . | . |
| 21-225_16B8_LC | . | . | . | . | N | . | . | . | . | . | . | . | . | . | . | . | . | V | . | . | . | . | . | . | . | . |
| 21-225_16C7_LC | . | . | . | . | S | . | . | . | . | . | . | . | . | . | . | . | . | . | . | . | . | . | . | . | . | . |
| 21-225_16G12_LC | . | . | . | . | . | . | . | . | . | . | . | . | . | . | . | . | . | . | . | . | . | . | . | . | . | . |
| 21-225_170E1_LC | . | . | . | . | . | . | . | . | . | . | . | . | . | . | . | . | . | . | . | . | . | . | . | . | . | . |
| 21-225_175C4_LC | . | . | . | . | . | . | . | . | . | . | . | . | . | . | . | . | G | . | . | . | . | F | . | . | . | . |
| 21-225_177D3_LC | . | . | . | . | . | . | . | . | . | . | . | . | . | . | . | . | . | . | . | . | . | . | . | . | . | . |
| 21-225_17H5_LC | . | . | . | . | N | . | . | . | . | . | . | . | . | . | . | . | . | . | . | . | . | . | . | . | . | . |
| 21-225_17H6_LC | . | . | . | . | S | T | . | . | . | . | . | . | . | . | . | . | . | . | . | . | . | . | . | . | . | . |
| 21-225_181G2_LC | . | . | . | . | . | . | . | . | . | . | . | . | . | . | . | . | . | . | . | . | . | . | S | . | R | . |
| 21-225_184E7_LC | . | . | . | . | . | . | . | . | . | . | . | . | . | . | . | . | . | . | . | . | . | . | S | . | R | . |
| 21-225_185A1_LC | . | . | . | . | S | V | . | . | . | . | . | . | . | . | . | . | . | . | . | . | . | . | . | . | R | . |
| 21-225_185E10_LC | . | . | . | . | . | . | . | . | . | . | . | . | . | . | . | . | . | . | . | . | . | . | S | . | R | . |
| 21-225_188B9_LC | . | . | . | . | . | . | . | . | . | . | . | . | . | . | . | . | . | . | . | . | . | . | S | . | R | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | S | L | I | Y | A | - | - | - | - | - | - | - | - | A | S | S | L | Q | S | G | V | P | S | K | F |
| 21-225_188H5_LC | . | . | . | . | . | . | . | . | . | . | . | . | . | . | . | . | . | . | . | . | . | . | S | . | R | . |
| 21-225_18D11_LC | . | . | . | . | . | V | . | . | . | . | . | . | . | . | . | . | . | . | . | . | . | . | . | . | . | . |
| 21-225_18G10_LC | . | . | . | . | . | V | . | . | . | . | . | . | . | . | . | . | . | . | . | . | . | . | . | . | . | . |
| 21-225_190A5_LC | . | . | . | . | H | . | . | . | . | . | . | . | . | . | . | . | . | . | . | . | . | . | . | . | . | . |
| 21-225_190B1_LC | . | . | . | . | . | V | . | . | . | . | . | . | . | . | . | . | . | . | E | . | . | . | . | . | . | . |
| 21-225_190F12_LC | . | . | . | . | . | . | . | . | . | . | . | . | . | . | . | . | . | . | . | . | . | . | . | . | . | . |
| 21-225_190H9_LC | . | . | . | . | . | . | . | . | . | . | . | . | . | . | . | . | . | . | . | . | . | . | . | . | . | . |
| 21-225_192A2_LC | . | . | . | . | . | . | . | . | . | . | . | . | . | . | . | . | . | . | . | . | . | . | . | . | . | . |
| 21-225_192C10_LC | . | . | . | . | . | . | . | . | . | . | . | . | . | . | . | . | . | . | . | . | . | . | . | . | . | . |
| 21-225_192E4_LC | . | . | . | . | . | V | . | . | . | . | . | . | . | . | V | . | . | . | . | . | . | . | . | . | R | . |
| 21-225_192H6_LC | . | . | . | . | . | . | . | . | . | . | . | . | . | . | . | . | . | . | . | . | . | . | . | . | . | . |
| 21-225_193F8_LC | . | . | . | . | . | . | . | . | . | . | . | . | . | . | . | . | . | . | . | . | . | . | . | . | . | . |
| 21-225_194H12_LC | . | . | . | . | . | . | . | . | . | . | . | . | . | . | . | . | . | . | . | . | . | . | . | . | . | . |
| 21-225_197E11_LC | . | . | . | . | . | . | . | . | . | . | . | . | . | . | . | . | . | V | . | . | . | . | . | . | . | . |
| 21-225_20B9_LC | . | . | . | . | . | V | . | . | . | . | . | . | . | . | . | . | . | . | . | . | . | . | . | . | . | . |
| 21-225_20C11_LC | . | . | . | . | . | . | . | . | . | . | . | . | . | . | . | . | . | . | . | . | . | . | . | . | . | . |
| 21-225_20F10_LC | . | . | . | . | . | . | . | . | . | . | . | . | . | . | . | . | . | . | . | . | . | . | . | . | R | . |
| 21-225_20H7_LC | . | R | . | . | . | . | . | . | . | . | . | . | . | . | . | . | . | . | . | . | . | . | . | . | . | . |
| 21-225_20H9_LC | . | . | . | . | S | . | . | . | . | . | . | . | . | . | . | . | . | . | . | . | . | . | . | . | . | . |
| 21-225_211A6_LC | . | . | . | . | . | . | . | . | . | . | . | . | . | . | . | . | . | . | L | . | . | . | . | . | . | . |
| 21-225_213B3_LC | . | . | . | . | S | . | . | . | . | . | . | . | . | . | . | . | . | V | L | . | . | . | . | . | . | . |
| 21-225_214C3_LC | . | . | . | . | . | . | . | . | . | . | . | . | . | . | . | . | . | . | . | . | . | . | . | . | . | . |
| 21-225_214E4_LC | . | . | . | . | . | . | . | . | . | . | . | . | . | . | . | . | . | . | . | . | . | . | . | . | . | . |
| 21-225_223D11_LC | . | . | . | . | . | . | . | . | . | . | . | . | . | . | . | . | T | . | . | . | . | . | . | . | . | . |
| 21-225_22B12_LC | . | . | . | . | . | G | . | . | . | . | . | . | . | . | . | . | . | . | R | . | . | . | . | . | . | . |
| 21-225_23A11_LC | . | R | . | . | . | . | . | . | . | . | . | . | . | . | . | . | T | . | . | . | . | . | . | . | . | . |
| 21-225_23C7_LC | . | . | . | . | S | . | . | . | . | . | . | . | . | . | . | . | . | . | . | . | . | . | . | . | . | . |
| 21-225_23E7_LC | . | . | . | . | . | . | . | . | . | . | . | . | . | . | . | . | . | . | . | . | . | . | . | . | . | . |
| 21-225_23G1_LC | . | . | . | . | S | . | . | . | . | . | . | . | . | . | . | . | . | . | . | . | . | . | . | . | Q | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | S | L | I | Y | A | - | - | - | - | - | - | - | - | A | S | S | L | Q | S | G | V | P | S | K | F |
| 21-225_23G8_LC | . | . | . | . | . | . | . | . | . | . | . | . | . | . | . | . | . | . | . | . | . | . | . | . | N | . |
| 21-225_25C10_LC | . | . | . | . | . | . | . | . | . | . | . | . | . | . | . | . | . | . | . | . | . | F | . | . | . | . |
| 21-225_26G1_LC | . | . | . | . | . | . | . | . | . | . | . | . | . | . | . | . | . | . | R | . | . | . | . | . | N | . |
| 21-225_27H12_LC | . | . | . | . | N | G | . | . | . | . | . | . | . | . | . | . | . | . | . | . | . | . | . | . | . | . |
| 21-225_2A4_LC | . | R | . | . | . | T | . | . | . | . | . | . | . | . | . | . | . | . | . | . | . | . | . | . | . | . |
| 21-225_30A1_LC | . | . | . | . | S | . | . | . | . | . | . | . | . | . | . | . | . | . | . | T | . | . | . | . | . | . |
| 21-225_32B3_LC | R | . | . | . | . | . | . | . | . | . | . | . | . | . | . | . | . | . | . | . | . | . | . | . | T | . |
| 21-225_33A5_LC | . | . | . | . | S | . | . | . | . | . | . | . | . | . | . | . | . | . | . | . | . | . | . | . | . | . |
| 21-225_35H8_LC | . | . | . | . | . | G | . | . | . | . | . | . | . | . | . | . | G | . | . | . | . | . | . | . | . | . |
| 21-225_43A4_LC | . | . | . | . | N | . | . | . | . | . | . | . | . | . | . | . | . | . | . | . | . | . | . | . | R | . |
| 21-225_45D9_LC | . | . | . | . | N | . | . | . | . | . | . | . | . | . | . | . | . | . | . | . | . | . | . | . | R | . |
| 21-225_51C5_LC | . | . | . | . | . | . | . | . | . | . | . | . | . | . | . | . | . | . | R | . | . | . | . | . | Q | . |
| 21-225_51D5_LC | . | . | . | . | . | . | . | . | . | . | . | . | . | . | . | . | . | . | R | . | . | . | . | . | Q | . |
| 21-225_52E3_LC | . | . | . | . | N | . | . | . | . | . | . | . | . | . | . | . | . | . | . | . | . | . | . | . | . | . |
| 21-225_53E4_LC | . | . | . | . | S | . | . | . | . | . | . | . | . | . | . | . | . | . | . | . | . | . | . | . | . | . |
| 21-225_56F7_LC | . | . | . | . | . | . | . | . | . | . | . | . | . | . | . | . | . | . | . | G | . | . | . | . | . | . |
| 21-225_56H1_LC | Q | . | . | M | S | . | . | . | . | . | . | . | . | . | . | . | G | . | . | . | . | . | . | . | . | . |
| 21-225_5A5_LC | . | . | . | . | S | . | . | . | . | . | . | . | . | . | . | . | . | . | . | . | . | . | . | . | . | . |
| 21-225_5E11_LC | . | . | . | . | N | . | . | . | . | . | . | . | . | . | . | . | . | V | . | . | . | . | . | . | . | . |
| 21-225_60A4_LC | . | . | . | . | . | . | . | . | . | . | . | . | . | . | . | . | . | . | . | . | . | . | . | . | . | . |
| 21-225_60F7_LC | . | . | . | . | . | . | . | . | . | . | . | . | . | . | . | . | . | . | . | . | . | . | . | . | T | . |
| 21-225_63H8_LC | . | . | . | . | . | . | . | . | . | . | . | . | . | . | . | . | . | . | . | . | . | . | . | . | . | . |
| 21-225_65D5_LC | R | . | . | . | . | . | . | . | . | . | . | . | . | . | . | . | . | . | H | . | . | . | . | . | Q | . |
| 21-225_66H11_LC | . | . | . | . | . | G | . | . | . | . | . | . | . | . | . | F | N | . | H | . | . | . | . | . | . | . |
| 21-225_68E6_LC | . | . | . | . | . | V | . | . | . | . | . | . | . | . | . | . | . | . | . | . | . | . | . | . | R | . |
| 21-225_7B1_LC | . | . | . | . | F | . | . | . | . | . | . | . | . | . | . | . | . | . | . | . | . | . | . | . | Q | . |
| 21-225_96C6_LC | . | . | . | . | . | . | . | . | . | . | . | . | . | . | . | . | . | . | . | . | . | . | . | . | . | . |
| 21-225_9C8_LC | . | . | . | . | . | V | . | . | . | . | . | . | . | . | . | . | . | . | . | . | . | . | . | . | . | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_148B6_LC | . | . | . | . | . | . | . | . | . | . | . | . | . | . | . | . | . | . | . | . | . | . | . | . | . | . |
| 21-225_14H8_LC | . | . | . | . | . | . | . | . | . | . | . | . | . | . | . | . | . | . | . | . | . | . | . | . | . | . |
| 21-225_153A1_LC | . | . | . | . | . | . | . | . | . | . | . | . | . | . | . | . | . | . | . | . | . | . | . | . | . | . |
| 21-225_157E4_LC | . | . | . | . | . | . | . | . | . | . | . | . | . | . | . | N | . | . | . | . | . | . | . | . | . | . |
| 21-225_157G5_LC | . | . | . | . | . | . | . | . | . | . | . | . | . | . | . | . | . | . | . | . | . | . | . | . | . | . |
| 21-225_157H1_LC | . | . | . | . | . | . | . | . | . | . | . | . | . | . | . | . | . | . | . | . | . | . | . | . | . | . |
| 21-225_157H10_LC | . | . | . | . | . | . | . | . | . | . | . | . | . | . | . | . | . | . | . | . | . | . | . | . | . | . |
| 21-225_158H12_LC | . | . | . | . | . | . | . | . | . | . | . | . | . | S | . | . | . | . | . | . | . | . | . | . | . | . |
| 21-225_159H11_LC | . | . | . | . | . | . | . | . | . | . | . | . | . | . | . | . | N | . | . | . | . | . | . | . | . | . |
| 21-225_15A2_LC | . | . | . | . | F | . | . | . | . | . | . | . | F | P | . | . | . | . | . | . | . | . | . | . | N | . |
| 21-225_15B1_LC | . | . | . | . | . | . | . | . | . | . | . | . | . | . | . | . | . | . | . | . | . | . | . | . | . | . |
| 21-225_160G1_LC | . | . | . | . | . | . | . | . | . | . | . | . | . | . | . | . | . | . | . | . | . | . | . | . | . | . |
| 21-225_160G3_LC | . | . | . | . | . | . | . | . | . | . | . | . | . | . | . | . | . | . | . | . | . | . | . | . | . | . |
| 21-225_163F1_LC | . | . | . | . | . | . | . | . | . | . | . | . | . | . | . | . | . | . | . | . | . | . | . | . | A | . |
| 21-225_167D12_LC | . | . | . | . | . | . | . | . | . | . | . | . | . | . | . | . | . | . | . | . | . | . | . | . | . | . |
| 21-225_169D10_LC | . | . | . | . | . | . | . | . | . | . | . | . | . | . | . | . | . | . | . | . | . | . | . | . | . | . |
| 21-225_16B8_LC | . | . | . | . | . | . | . | . | . | . | . | . | . | . | . | . | . | . | . | . | . | . | . | . | . | . |
| 21-225_16C7_LC | . | . | . | . | . | . | . | . | . | . | . | . | . | . | . | . | . | . | . | . | . | . | . | . | . | . |
| 21-225_16G12_LC | R | . | . | . | . | . | . | . | . | . | . | . | . | . | . | . | N | . | . | . | . | . | . | . | S | . |
| 21-225_170E1_LC | . | . | . | . | . | . | . | . | . | . | . | . | . | . | . | . | . | . | . | . | . | . | . | . | . | . |
| 21-225_175C4_LC | . | . | . | . | . | . | . | . | . | . | . | . | . | . | . | . | . | . | . | . | . | . | . | . | . | . |
| 21-225_177D3_LC | . | . | . | . | . | . | . | . | . | . | . | . | . | . | . | . | . | . | . | . | . | . | . | . | . | . |
| 21-225_17H5_LC | . | . | . | . | . | . | . | . | . | . | . | . | . | . | . | . | . | . | . | . | . | . | . | . | . | . |
| 21-225_17H6_LC | . | . | . | . | . | . | . | . | . | . | . | . | . | . | . | . | . | . | . | . | . | . | . | . | I | . |
| 21-225_181G2_LC | . | . | . | . | F | . | . | . | . | . | . | . | . | . | . | . | . | . | . | . | . | . | . | . | . | . |
| 21-225_184E7_LC | . | . | . | . | F | . | . | . | . | . | . | . | I | . | . | . | . | V | . | R | . | . | . | . | . | . |
| 21-225_185A1_LC | . | A | . | . | . | . | . | . | . | . | . | . | . | . | . | . | . | . | . | . | . | . | . | . | . | . |
| 21-225_185E10_LC | . | . | . | . | F | . | . | . | . | . | . | . | . | . | . | . | . | . | . | . | . | . | . | . | . | . |
| 21-225_188B9_LC | . | . | . | . | F | . | . | . | . | . | . | . | . | . | . | . | . | . | . | . | . | . | . | . | . | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_188H5_LC | . | . | . | . | F | . | . | . | . | . | . | . | . | . | . | . | . | . | . | . | . | . | . | . | . | . |
| 21-225_18D11_LC | . | . | . | . | F | . | . | . | . | . | . | . | . | . | . | . | . | . | . | . | . | . | . | . | . | . |
| 21-225_18G10_LC | G | . | . | . | F | . | . | . | . | . | . | . | . | . | . | . | . | . | . | . | . | . | . | . | . | . |
| 21-225_190A5_LC | . | . | . | . | . | . | . | . | . | . | . | . | . | . | . | . | . | . | . | . | . | . | . | . | . | . |
| 21-225_190B1_LC | . | . | . | . | . | . | . | . | . | E | . | . | . | . | . | . | . | . | . | . | . | . | . | G | . | . |
| 21-225_190F12_LC | . | . | . | . | . | . | . | . | . | . | . | . | . | . | . | . | . | . | . | . | D | . | . | . | . | . |
| 21-225_190H9_LC | . | . | . | . | . | . | . | . | . | . | . | . | . | . | . | . | . | . | . | . | D | . | . | . | . | . |
| 21-225_192A2_LC | . | . | . | . | . | . | . | . | . | . | . | . | . | . | . | . | . | . | . | . | . | . | . | . | . | . |
| 21-225_192C10_LC | . | . | . | . | . | . | . | . | . | . | . | . | . | . | . | . | . | . | . | . | D | . | . | . | . | . |
| 21-225_192E4_LC | . | . | . | . | . | . | . | . | . | . | . | . | . | . | . | . | . | . | . | . | . | . | . | . | . | . |
| 21-225_192H6_LC | . | . | . | R | . | . | . | . | . | . | . | S | . | . | . | . | . | . | . | . | . | . | . | . | . | . |
| 21-225_193F8_LC | . | . | . | . | . | . | . | . | A | . | . | . | . | . | . | . | . | . | . | . | . | . | . | . | . | . |
| 21-225_194H12_LC | . | . | . | . | . | . | . | . | . | . | . | . | . | . | . | . | . | . | . | . | D | . | . | . | . | . |
| 21-225_197E11_LC | . | . | . | . | . | . | . | . | . | . | . | . | . | . | . | . | R | . | . | . | D | . | . | . | . | . |
| 21-225_20B9_LC | . | . | . | . | . | . | . | . | . | . | . | . | . | . | . | . | . | . | . | . | . | . | . | . | . | . |
| 21-225_20C11_LC | R | . | . | . | . | . | . | . | . | . | . | . | . | . | . | . | . | . | . | . | . | . | . | . | . | . |
| 21-225_20F10_LC | . | . | . | . | F | . | . | . | . | . | . | . | . | . | . | . | . | . | . | . | . | . | . | . | . | F |
| 21-225_20H7_LC | . | . | . | . | . | . | . | . | . | . | . | . | . | . | . | . | . | . | . | . | . | . | . | . | . | . |
| 21-225_20H9_LC | . | . | . | . | . | . | . | . | . | . | . | . | . | . | . | . | . | . | . | . | . | . | . | . | . | . |
| 21-225_211A6_LC | . | . | . | . | . | . | . | . | . | . | . | . | . | . | . | . | . | . | . | . | . | . | . | . | . | . |
| 21-225_213B3_LC | . | . | . | . | . | . | . | . | . | . | . | . | . | . | . | . | . | . | . | . | . | . | . | . | . | . |
| 21-225_214C3_LC | . | . | . | . | . | . | . | . | . | . | . | . | . | . | . | . | . | . | . | . | . | . | . | T | . | . |
| 21-225_214E4_LC | . | . | . | . | . | . | . | . | . | . | . | . | . | . | . | . | . | . | . | . | . | . | . | . | . | . |
| 21-225_223D11_LC | . | . | . | R | . | . | . | . | . | . | . | N | . | . | . | . | N | . | . | . | . | . | . | . | . | . |
| 21-225_22B12_LC | . | . | . | . | . | . | . | . | . | . | . | N | . | . | . | . | . | . | . | . | . | . | L | . | . | . |
| 21-225_23A11_LC | . | . | . | . | . | . | . | . | . | . | . | . | . | . | . | . | . | . | . | . | . | . | . | . | . | . |
| 21-225_23C7_LC | . | . | . | . | . | . | . | . | . | E | . | . | . | . | . | . | . | . | . | . | . | . | . | . | . | . |
| 21-225_23E7_LC | . | . | . | . | . | . | . | . | . | . | . | . | . | . | . | . | . | . | . | . | . | . | . | . | . | . |
| 21-225_23G1_LC | . | . | . | . | F | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_23G8_LC | . | . | . | . | . | . | . | . | . | . | . | . | . | . | . | . | . | . | . | . | . | . | . | . | . | . |
| 21-225_25C10_LC | . | . | . | . | . | . | . | . | . | . | . | . | . | . | . | . | . | . | . | . | . | . | . | . | . | . |
| 21-225_26G1_LC | . | . | . | . | . | . | . | . | . | . | . | . | . | . | . | . | . | . | . | . | . | N | . | . | . | . |
| 21-225_27H12_LC | . | . | . | . | . | . | . | . | . | . | . | . | . | . | . | . | . | . | . | . | . | . | . | . | . | . |
| 21-225_2A4_LC | . | . | . | . | F | . | . | . | . | . | . | . | . | . | . | . | . | . | . | . | . | . | . | . | . | . |
| 21-225_30A1_LC | . | . | . | . | . | . | . | . | . | . | . | . | . | . | . | . | . | . | . | . | . | . | . | . | . | . |
| 21-225_32B3_LC | . | . | . | . | . | . | . | . | . | . | . | . | . | . | . | . | . | . | . | . | . | . | . | . | . | . |
| 21-225_33A5_LC | . | . | . | . | . | . | . | . | . | . | . | . | . | . | . | . | . | . | . | . | . | . | . | . | . | . |
| 21-225_35H8_LC | . | . | . | . | . | . | . | . | . | . | . | . | . | . | . | . | . | . | . | . | . | . | . | . | . | . |
| 21-225_43A4_LC | . | . | . | . | . | . | . | . | . | . | . | . | . | A | . | . | . | . | . | . | . | . | . | . | . | . |
| 21-225_45D9_LC | . | . | . | . | . | . | . | . | . | . | . | . | . | A | . | . | . | . | . | . | . | . | . | . | . | . |
| 21-225_51C5_LC | . | . | . | . | . | . | . | . | . | . | . | I | . | . | . | . | . | . | . | . | . | . | . | . | . | . |
| 21-225_51D5_LC | . | . | . | . | . | . | . | . | . | . | . | I | . | . | . | . | . | . | . | . | . | . | . | . | . | . |
| 21-225_52E3_LC | . | . | . | . | . | . | . | . | . | . | . | . | . | . | . | . | . | . | . | . | . | . | . | . | . | . |
| 21-225_53E4_LC | . | . | . | . | . | . | . | . | . | . | . | . | . | . | . | . | . | . | . | . | . | . | . | . | . | . |
| 21-225_56F7_LC | . | . | . | . | . | . | . | . | . | . | . | . | . | . | . | . | . | . | . | . | . | . | . | . | . | . |
| 21-225_56H1_LC | . | . | . | . | . | . | . | . | . | . | . | . | . | . | . | . | . | . | . | . | . | . | . | . | . | . |
| 21-225_5A5_LC | . | . | . | . | . | . | . | . | . | . | . | . | . | . | . | . | . | . | . | . | . | . | . | . | . | . |
| 21-225_5E11_LC | . | . | N | . | . | . | . | . | . | . | . | . | . | . | . | . | . | . | . | . | . | . | . | . | . | . |
| 21-225_60A4_LC | . | . | . | . | . | . | . | . | . | . | . | . | . | . | . | . | . | . | . | S | . | . | . | . | . | . |
| 21-225_60F7_LC | . | . | . | . | . | . | . | . | . | . | . | . | . | . | . | . | . | . | . | . | . | . | . | . | . | . |
| 21-225_63H8_LC | . | . | . | . | . | . | . | . | . | . | . | . | . | . | . | . | . | . | . | . | . | . | . | . | . | . |
| 21-225_65D5_LC | . | A | . | . | . | . | . | . | S | . | . | . | . | . | . | . | . | . | . | . | . | . | . | . | . | . |
| 21-225_66H11_LC | . | . | . | . | F | . | . | . | . | . | . | . | . | . | . | N | . | . | . | . | . | . | . | . | N | . |
| 21-225_68E6_LC | . | . | . | . | . | . | . | . | . | . | . | . | . | . | . | . | . | . | . | . | . | . | . | . | I | . |
| 21-225_7B1_LC | . | . | . | . | . | . | . | . | . | . | . | . | . | . | . | . | I | . | . | . | . | . | . | . | . | . |
| 21-225_96C6_LC | . | . | . | . | . | . | . | . | . | . | . | . | . | . | . | N | . | . | . | . | . | . | . | . | . | . |
| 21-225_9C8_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | N | S | - | - | - | - | - | - | - | - | - | - | - | - | - | - | - | - | - | - | - |
| 21-225_148B6_LC | . | . | . | . | . | . | . | . | . | . | . | . | . | . | . | . | . | . | . | . | . | . | . | . | . | . |
| 21-225_14H8_LC | . | . | . | . | . | . | . | . | . | . | . | . | . | . | . | . | . | . | . | . | . | . | . | . | . | . |
| 21-225_153A1_LC | . | . | . | . | . | H | . | . | . | . | . | . | . | . | . | . | . | . | . | . | . | . | . | . | . | . |
| 21-225_157E4_LC | . | . | . | . | . | H | . | . | . | . | . | . | . | . | . | . | . | . | . | . | . | . | . | . | . | . |
| 21-225_157G5_LC | . | . | . | . | . | . | . | . | . | . | . | . | . | . | . | . | . | . | . | . | . | . | . | . | . | . |
| 21-225_157H1_LC | . | . | . | . | . | H | . | . | . | . | . | . | . | . | . | . | . | . | . | . | . | . | . | . | . | . |
| 21-225_157H10_LC | . | . | . | . | . | H | . | . | . | . | . | . | . | . | . | . | . | . | . | . | . | . | . | . | . | . |
| 21-225_158H12_LC | . | . | . | . | . | H | . | . | . | . | . | . | . | . | . | . | . | . | . | . | . | . | . | . | . | . |
| 21-225_159H11_LC | . | . | . | . | . | . | . | . | . | . | . | . | . | . | . | . | . | . | . | . | . | . | . | . | . | . |
| 21-225_15A2_LC | . | . | . | . | . | Y | . | . | . | . | . | . | . | . | . | . | . | . | . | . | . | . | . | . | . | . |
| 21-225_15B1_LC | . | . | . | . | . | H | . | . | . | . | . | . | . | . | . | . | . | . | . | . | . | . | . | . | . | . |
| 21-225_160G1_LC | . | . | . | . | . | H | . | . | . | . | . | . | . | . | . | . | . | . | . | . | . | . | . | . | . | . |
| 21-225_160G3_LC | . | . | . | . | . | H | . | . | . | . | . | . | . | . | . | . | . | . | . | . | . | . | . | . | . | . |
| 21-225_163F1_LC | . | . | . | . | . | . | . | . | . | . | . | . | . | . | . | . | . | . | . | . | . | . | . | . | . | . |
| 21-225_167D12_LC | F | . | . | . | . | . | . | . | . | . | . | . | . | . | . | . | . | . | . | . | . | . | . | . | . | . |
| 21-225_169D10_LC | . | . | . | . | . | H | . | . | . | . | . | . | . | . | . | . | . | . | . | . | . | . | . | . | . | . |
| 21-225_16B8_LC | . | . | H | . | . | H | . | . | . | . | . | . | . | . | . | . | . | . | . | . | . | . | . | . | . | . |
| 21-225_16C7_LC | . | . | . | . | . | . | . | . | . | . | . | . | . | . | . | . | . | . | . | . | . | . | . | . | . | . |
| 21-225_16G12_LC | . | . | . | . | . | H | . | . | . | . | . | . | . | . | . | . | . | . | . | . | . | . | . | . | . | . |
| 21-225_170E1_LC | . | . | . | . | . | H | . | . | . | . | . | . | . | . | . | . | . | . | . | . | . | . | . | . | . | . |
| 21-225_175C4_LC | . | . | . | . | . | Y | . | . | . | . | . | . | . | . | . | . | . | . | . | . | . | . | . | . | . | . |
| 21-225_177D3_LC | . | . | . | . | . | . | . | . | . | . | . | . | . | . | . | . | . | . | . | . | . | . | . | . | . | . |
| 21-225_17H5_LC | . | . | . | . | . | H | . | . | . | . | . | . | . | . | . | . | . | . | . | . | . | . | . | . | . | . |
| 21-225_17H6_LC | . | . | . | . | . | H | . | . | . | . | . | . | . | . | . | . | . | . | . | . | . | . | . | . | . | . |
| 21-225_181G2_LC | . | . | . | . | . | . | G | . | . | . | . | . | . | . | . | . | . | . | . | . | . | . | . | . | . | . |
| 21-225_184E7_LC | . | . | . | . | . | . | G | . | . | . | . | . | . | . | . | . | . | . | . | . | . | . | . | . | . | . |
| 21-225_185A1_LC | . | . | R | . | . | H | . | . | . | . | . | . | . | . | . | . | . | . | . | . | . | . | . | . | . | . |
| 21-225_185E10_LC | . | . | . | . | . | . | G | . | . | . | . | . | . | . | . | . | . | . | . | . | . | . | . | . | . | . |
| 21-225_188B9_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | N | S | - | - | - | - | - | - | - | - | - | - | - | - | - | - | - | - | - | - | - |
| 21-225_188H5_LC | . | . | . | . | . | . | . | . | . | . | . | . | . | . | . | . | . | . | . | . | . | . | . | . | . | . |
| 21-225_18D11_LC | . | . | . | . | . | . | T | . | . | . | . | . | . | . | . | . | . | . | . | . | . | . | . | . | . | . |
| 21-225_18G10_LC | . | . | L | . | . | . | . | . | . | . | . | . | . | . | . | . | . | . | . | . | . | . | . | . | . | . |
| 21-225_190A5_LC | . | . | . | . | . | S | N | . | . | . | . | . | . | . | . | . | . | . | . | . | . | . | . | . | . | . |
| 21-225_190B1_LC | . | . | . | . | . | L | N | . | . | . | . | . | . | . | . | . | . | . | . | . | . | . | . | . | . | . |
| 21-225_190F12_LC | . | . | . | R | . | D | T | . | . | . | . | . | . | . | . | . | . | . | . | . | . | . | . | . | . | . |
| 21-225_190H9_LC | . | . | . | R | . | D | T | . | . | . | . | . | . | . | . | . | . | . | . | . | . | . | . | . | . | . |
| 21-225_192A2_LC | . | . | . | . | . | . | . | . | . | . | . | . | . | . | . | . | . | . | . | . | . | . | . | . | . | . |
| 21-225_192C10_LC | . | . | . | R | . | D | T | . | . | . | . | . | . | . | . | . | . | . | . | . | . | . | . | . | . | . |
| 21-225_192E4_LC | . | . | . | R | . | D | T | . | . | . | . | . | . | . | . | . | . | . | . | . | . | . | . | . | . | . |
| 21-225_192H6_LC | . | . | . | . | . | S | N | . | . | . | . | . | . | . | . | . | . | . | . | . | . | . | . | . | . | . |
| 21-225_193F8_LC | . | . | . | . | . | . | . | . | . | . | . | . | . | . | . | . | . | . | . | . | . | . | . | . | . | . |
| 21-225_194H12_LC | . | . | . | R | . | D | T | . | . | . | . | . | . | . | . | . | . | . | . | . | . | . | . | . | . | . |
| 21-225_197E11_LC | . | G | . | R | . | D | T | . | . | . | . | . | . | . | . | . | . | . | . | . | . | . | . | . | . | . |
| 21-225_20B9_LC | . | . | . | . | . | . | T | . | . | . | . | . | . | . | . | . | . | . | . | . | . | . | . | . | . | . |
| 21-225_20C11_LC | . | . | . | . | . | H | . | . | . | . | . | . | . | . | . | . | . | . | . | . | . | . | . | . | . | . |
| 21-225_20F10_LC | . | . | . | . | . | . | . | . | . | . | . | . | . | . | . | . | . | . | . | . | . | . | . | . | . | . |
| 21-225_20H7_LC | . | . | . | . | . | Y | . | . | . | . | . | . | . | . | . | . | . | . | . | . | . | . | . | . | . | . |
| 21-225_20H9_LC | . | . | . | . | . | . | . | . | . | . | . | . | . | . | . | . | . | . | . | . | . | . | . | . | . | . |
| 21-225_211A6_LC | . | . | . | K | . | D | T | . | . | . | . | . | . | . | . | . | . | . | . | . | . | . | . | . | . | . |
| 21-225_213B3_LC | . | . | . | K | . | D | T | . | . | . | . | . | . | . | . | . | . | . | . | . | . | . | . | . | . | . |
| 21-225_214C3_LC | . | . | . | . | . | M | T | . | . | . | . | . | . | . | . | . | . | . | . | . | . | . | . | . | . | . |
| 21-225_214E4_LC | . | . | . | . | . | L | T | . | . | . | . | . | . | . | . | . | . | . | . | . | . | . | . | . | . | . |
| 21-225_223D11_LC | . | . | . | . | . | S | G | . | . | . | . | . | . | . | . | . | . | . | . | . | . | . | . | . | . | . |
| 21-225_22B12_LC | . | . | . | . | . | H | . | . | . | . | . | . | . | . | . | . | . | . | . | . | . | . | . | . | . | . |
| 21-225_23A11_LC | . | . | . | . | . | . | . | . | . | . | . | . | . | . | . | . | . | . | . | . | . | . | . | . | . | . |
| 21-225_23C7_LC | . | . | . | . | . | . | . | . | . | . | . | . | . | . | . | . | . | . | . | . | . | . | . | . | . | . |
| 21-225_23E7_LC | . | . | . | . | F | Y | . | . | . | . | . | . | . | . | . | . | . | . | . | . | . | . | . | . | . | . |
| 21-225_23G1_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | N | S | - | - | - | - | - | - | - | - | - | - | - | - | - | - | - | - | - | - | - |
| 21-225_23G8_LC | . | . | . | . | . | . | . | . | . | . | . | . | . | . | . | . | . | . | . | . | . | . | . | . | . | . |
| 21-225_25C10_LC | . | . | . | . | . | H | . | . | . | . | . | . | . | . | . | . | . | . | . | . | . | . | . | . | . | . |
| 21-225_26G1_LC | . | . | . | . | . | H | . | . | . | . | . | . | . | . | . | . | . | . | . | . | . | . | . | . | . | . |
| 21-225_27H12_LC | . | . | . | . | . | Y | . | . | . | . | . | . | . | . | . | . | . | . | . | . | . | . | . | . | . | . |
| 21-225_2A4_LC | . | . | . | . | . | . | . | . | . | . | . | . | . | . | . | . | . | . | . | . | . | . | . | . | . | . |
| 21-225_30A1_LC | . | . | . | . | . | . | . | . | . | . | . | . | . | . | . | . | . | . | . | . | . | . | . | . | . | . |
| 21-225_32B3_LC | . | . | . | . | . | . | . | . | . | . | . | . | . | . | . | . | . | . | . | . | . | . | . | . | . | . |
| 21-225_33A5_LC | . | . | . | . | . | . | . | . | . | . | . | . | . | . | . | . | . | . | . | . | . | . | . | . | . | . |
| 21-225_35H8_LC | . | . | . | . | . | . | . | . | . | . | . | . | . | . | . | . | . | . | . | . | . | . | . | . | . | . |
| 21-225_43A4_LC | . | . | . | . | . | Y | . | . | . | . | . | . | . | . | . | . | . | . | . | . | . | . | . | . | . | . |
| 21-225_45D9_LC | . | . | . | H | . | Y | . | . | . | . | . | . | . | . | . | . | . | . | . | . | . | . | . | . | . | . |
| 21-225_51C5_LC | . | . | . | . | . | Y | . | . | . | . | . | . | . | . | . | . | . | . | . | . | . | . | . | . | . | . |
| 21-225_51D5_LC | . | . | . | . | . | Y | . | . | . | . | . | . | . | . | . | . | . | . | . | . | . | . | . | . | . | . |
| 21-225_52E3_LC | . | . | . | . | . | . | . | . | . | . | . | . | . | . | . | . | . | . | . | . | . | . | . | . | . | . |
| 21-225_53E4_LC | . | . | . | . | . | H | . | . | . | . | . | . | . | . | . | . | . | . | . | . | . | . | . | . | . | . |
| 21-225_56F7_LC | . | . | H | . | . | . | . | . | . | . | . | . | . | . | . | . | . | . | . | . | . | . | . | . | . | . |
| 21-225_56H1_LC | . | . | . | . | . | Q | N | . | . | . | . | . | . | . | . | . | . | . | . | . | . | . | . | . | . | . |
| 21-225_5A5_LC | . | . | . | . | . | . | . | . | . | . | . | . | . | . | . | . | . | . | . | . | . | . | . | . | . | . |
| 21-225_5E11_LC | . | . | H | . | . | H | . | . | . | . | . | . | . | . | . | . | . | . | . | . | . | . | . | . | . | . |
| 21-225_60A4_LC | . | . | . | . | . | K | . | . | . | . | . | . | . | . | . | . | . | . | . | . | . | . | . | . | . | . |
| 21-225_60F7_LC | . | . | L | . | F | H | . | . | . | . | . | . | . | . | . | . | . | . | . | . | . | . | . | . | . | . |
| 21-225_63H8_LC | . | . | . | . | . | H | . | . | . | . | . | . | . | . | . | . | . | . | . | . | . | . | . | . | . | . |
| 21-225_65D5_LC | . | . | P | L | . | K | . | . | . | . | . | . | . | . | . | . | . | . | . | . | . | . | . | . | . | . |
| 21-225_66H11_LC | . | . | . | . | . | S | C | . | . | . | . | . | . | . | . | . | . | . | . | . | . | . | . | . | . | . |
| 21-225_68E6_LC | . | . | . | . | . | D | . | . | . | . | . | . | . | . | . | . | . | . | . | . | . | . | . | . | . | . |
| 21-225_7B1_LC | . | . | H | . | . | . | . | . | . | . | . | . | . | . | . | . | . | . | . | . | . | . | . | . | . | . |
| 21-225_96C6_LC | . | . | H | . | . | . | D | . | . | . | . | . | . | . | . | . | . | . | . | . | . | . | . | . | . | . |
| 21-225_9C8_LC | . | . | . | . | . | D | . | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | F | T | F | G | P | G | T | K | V | D | I | K | R |
| 21-225_148B6_LC | . | . | . | . | F | . | . | . | . | . | . | . | . | . | . | . | . | . | . |
| 21-225_14H8_LC | . | . | . | . | . | . | . | . | . | . | . | . | . | . | . | . | . | N | . |
| 21-225_153A1_LC | . | . | . | . | . | . | . | . | . | . | . | . | . | . | . | . | F | . | . |
| 21-225_157E4_LC | . | . | . | . | . | . | . | . | . | . | . | . | . | . | . | . | . | . | . |
| 21-225_157G5_LC | . | . | . | . | . | . | . | . | . | . | . | . | . | . | . | . | . | . | . |
| 21-225_157H1_LC | . | . | . | . | . | . | . | . | . | . | . | . | . | . | . | . | . | . | . |
| 21-225_157H10_LC | . | . | . | . | . | . | . | . | . | . | . | . | . | . | . | . | . | . | . |
| 21-225_158H12_LC | . | . | . | . | . | . | . | . | . | . | . | . | . | . | . | . | . | . | . |
| 21-225_159H11_LC | . | . | . | . | . | . | . | . | . | . | . | . | . | . | . | . | . | . | . |
| 21-225_15A2_LC | . | . | . | . | . | . | . | . | . | . | . | . | . | Q | . | . | V | . | . |
| 21-225_15B1_LC | . | . | . | . | . | . | . | . | . | . | . | . | . | . | . | . | . | . | . |
| 21-225_160G1_LC | . | . | . | . | . | . | . | . | . | . | . | . | . | . | . | . | . | R | . |
| 21-225_160G3_LC | . | . | . | . | . | . | . | . | . | . | . | . | . | . | . | . | . | . | . |
| 21-225_163F1_LC | . | . | . | . | . | . | . | . | . | . | . | . | . | Q | . | . | A | Q | . |
| 21-225_167D12_LC | . | . | . | . | . | . | . | . | . | . | . | . | . | . | . | . | . | . | . |
| 21-225_169D10_LC | . | . | . | . | . | . | . | . | . | . | . | . | . | . | . | . | . | . | . |
| 21-225_16B8_LC | . | . | . | . | . | . | . | . | . | . | . | . | . | . | . | . | . | V | . |
| 21-225_16C7_LC | . | . | . | . | . | . | . | . | . | . | . | . | . | . | . | . | . | . | . |
| 21-225_16G12_LC | . | . | . | . | F | . | . | . | . | . | . | . | . | . | . | . | . | . | . |
| 21-225_170E1_LC | . | . | . | . | . | . | . | . | . | . | . | . | . | . | . | . | . | . | . |
| 21-225_175C4_LC | . | . | . | . | . | . | . | . | . | . | . | . | . | . | . | . | . | . | . |
| 21-225_177D3_LC | . | . | . | . | . | . | . | . | . | . | . | . | . | . | . | . | . | . | . |
| 21-225_17H5_LC | . | . | . | . | . | . | . | . | . | . | . | . | . | . | . | . | . | . | . |
| 21-225_17H6_LC | . | . | . | . | . | . | . | . | . | . | . | . | . | . | . | . | . | Q | . |
| 21-225_181G2_LC | . | . | . | . | . | . | . | . | . | . | . | . | . | . | . | . | . | . | . |
| 21-225_184E7_LC | . | . | . | . | . | . | . | . | . | . | H | . | . | . | . | . | . | . | . |
| 21-225_185A1_LC | . | . | . | . | . | . | . | . | . | . | . | . | . | . | . | . | . | . | . |
| 21-225_185E10_LC | . | . | . | . | . | . | . | . | . | . | . | . | . | . | . | . | . | . | . |
| 21-225_188B9_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | F | T | F | G | P | G | T | K | V | D | I | K | R |
| 21-225_188H5_LC | . | . | . | . | . | . | . | . | . | . | . | . | . | . | . | . | . | . | . |
| 21-225_18D11_LC | . | . | . | . | . | . | . | . | . | . | . | . | . | T | . | . | . | . | . |
| 21-225_18G10_LC | . | . | . | . | . | . | . | . | . | . | . | . | . | . | . | . | . | . | . |
| 21-225_190A5_LC | . | . | . | . | . | . | V | . | . | . | . | . | . | . | . | . | . | . | . |
| 21-225_190B1_LC | . | . | . | . | . | . | V | . | . | . | . | . | . | . | . | . | . | R | . |
| 21-225_190F12_LC | . | . | . | . | . | . | . | . | . | . | . | . | . | . | . | . | . | . | . |
| 21-225_190H9_LC | . | . | . | . | . | . | . | . | . | . | . | . | . | . | . | . | . | . | . |
| 21-225_192A2_LC | . | . | . | . | . | . | . | . | . | . | . | . | . | . | . | . | . | . | . |
| 21-225_192C10_LC | . | . | . | . | . | . | . | . | . | . | . | . | . | . | . | . | F | . | . |
| 21-225_192E4_LC | . | . | . | . | . | . | . | . | . | . | . | . | . | . | . | . | . | . | . |
| 21-225_192H6_LC | . | . | . | . | . | . | V | . | . | . | . | . | . | . | . | . | . | . | . |
| 21-225_193F8_LC | . | . | . | . | . | . | . | . | . | . | . | . | . | . | . | . | . | . | . |
| 21-225_194H12_LC | . | . | . | . | . | . | . | . | . | . | . | . | . | . | . | . | . | . | . |
| 21-225_197E11_LC | . | . | . | . | . | . | . | . | . | . | . | . | . | . | . | . | . | . | . |
| 21-225_20B9_LC | . | . | . | . | . | . | . | . | . | . | . | . | . | . | . | . | . | . | . |
| 21-225_20C11_LC | . | . | . | . | F | . | . | . | . | . | . | . | . | . | . | . | V | . | . |
| 21-225_20F10_LC | . | . | . | . | . | . | . | . | . | . | . | . | . | . | . | . | N | . | . |
| 21-225_20H7_LC | . | . | . | . | . | . | . | . | . | . | . | . | . | . | M | . | F | . | . |
| 21-225_20H9_LC | . | . | . | . | . | . | . | . | . | . | . | . | . | . | . | . | . | . | . |
| 21-225_211A6_LC | . | . | . | . | . | . | . | . | . | . | . | . | . | . | . | . | . | . | . |
| 21-225_213B3_LC | . | . | . | . | . | . | . | . | . | . | . | . | . | . | . | . | . | . | . |
| 21-225_214C3_LC | . | . | . | . | . | . | I | . | . | . | . | . | . | . | . | . | . | . | . |
| 21-225_214E4_LC | . | . | . | . | . | . | . | . | . | . | . | . | . | . | . | . | . | . | . |
| 21-225_223D11_LC | . | . | . | . | . | . | . | . | . | . | . | . | . | . | . | . | T | . | . |
| 21-225_22B12_LC | . | . | . | . | . | . | . | . | . | . | . | . | . | . | . | . | F | . | . |
| 21-225_23A11_LC | . | . | . | . | . | . | . | . | . | . | . | . | . | . | . | . | L | . | . |
| 21-225_23C7_LC | . | . | . | . | . | . | . | . | . | . | . | . | . | . | . | . | . | . | . |
| 21-225_23E7_LC | . | . | . | . | . | . | . | . | . | . | . | . | . | . | . | . | . | N | . |
| 21-225_23G1_LC | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | F | T | F | G | P | G | T | K | V | D | I | K | R |
| 21-225_23G8_LC | . | . | . | . | . | . | . | . | I | . | . | . | . | . | . | . | . | . | . |
| 21-225_25C10_LC | . | . | . | . | . | . | . | . | . | . | . | . | . | . | . | . | . | . | . |
| 21-225_26G1_LC | . | . | . | . | . | . | . | . | . | . | . | . | . | . | . | . | . | . | . |
| 21-225_27H12_LC | . | . | . | . | . | . | . | . | . | . | . | . | . | . | . | N | . | N | . |
| 21-225_2A4_LC | . | . | . | . | . | . | . | . | . | . | . | . | . | . | . | . | . | . | . |
| 21-225_30A1_LC | . | . | . | . | . | . | . | . | . | . | . | . | . | . | . | . | . | . | . |
| 21-225_32B3_LC | . | . | . | . | . | . | . | . | . | . | . | . | . | . | . | . | . | . | . |
| 21-225_33A5_LC | . | . | . | . | . | . | . | . | . | . | . | . | . | N | . | . | . | . | . |
| 21-225_35H8_LC | . | . | . | . | . | . | . | . | . | . | . | . | . | . | . | . | F | . | . |
| 21-225_43A4_LC | . | . | . | . | . | . | . | . | . | . | . | . | . | . | . | . | . | . | . |
| 21-225_45D9_LC | . | . | . | . | . | . | . | . | . | . | R | . | . | . | . | . | . | . | . |
| 21-225_51C5_LC | . | . | . | . | . | . | . | . | . | . | . | . | . | . | . | . | . | . | . |
| 21-225_51D5_LC | . | . | . | . | . | . | . | . | . | . | . | . | . | . | . | . | . | . | . |
| 21-225_52E3_LC | . | . | . | . | F | . | . | . | . | . | . | . | . | . | . | . | . | . | . |
| 21-225_53E4_LC | . | . | . | . | . | . | L | . | . | . | R | . | . | . | . | . | . | . | . |
| 21-225_56F7_LC | . | . | . | . | F | . | . | K | . | . | R | . | . | . | . | . | . | T | G |
| 21-225_56H1_LC | . | . | . | . | . | . | . | . | . | . | . | . | . | . | . | . | . | . | . |
| 21-225_5A5_LC | . | . | . | . | . | . | . | . | . | . | . | . | . | . | . | . | . | . | . |
| 21-225_5E11_LC | . | . | . | . | . | . | . | . | . | . | . | . | . | . | . | . | . | . | . |
| 21-225_60A4_LC | . | . | . | . | H | . | . | . | . | . | . | . | . | . | . | . | . | . | . |
| 21-225_60F7_LC | . | . | . | . | . | . | . | . | . | . | . | . | . | . | M | . | . | . | . |
| 21-225_63H8_LC | . | . | . | . | . | . | . | . | . | . | . | . | . | . | . | . | . | R | . |
| 21-225_65D5_LC | . | . | . | . | . | . | L | . | . | . | . | . | . | . | . | . | . | . | . |
| 21-225_66H11_LC | . | . | . | . | . | . | . | . | . | . | H | . | . | . | . | . | . | . | . |
| 21-225_68E6_LC | . | . | . | . | . | . | . | . | . | . | . | . | . | . | . | . | . | R | . |
| 21-225_7B1_LC | . | . | . | . | . | . | . | . | . | . | . | . | . | . | . | . | F | . | . |
| 21-225_96C6_LC | . | . | . | . | . | . | . | . | . | . | . | . | . | . | . | . | . | . | . |
| 21-225_9C8_LC | . | . | . | . | . | . | . | . | . | . | . | . | . | . | . | . | . | . | . |

**FIGURE 57**

Table 104. Consensus 24 - VK1|L5/JK3 (SEQ ID NO: 50321):

DIQMTQSPSSVSASVGDRVTITCRAS--QGIS------RWLAWYQQKPGKAPKLLIYA--------ASSLQSGVPSRFSGSGSG--TDFTLTISSLQPEDFATYYCQQANS-----------------------FPFTFGPGTKVDIKR wherein:

A at position 25 can be substituted with V, E or G

S at position 26 can be substituted with G

Q at position 29 can be substituted with R

G at position 30 can be substituted with D, N, A, L or V

I at position 31 can be substituted with V or F

S at position 32 can be substituted with N, T, R or I

R at position 39 can be substituted with S, N, K, T, D, I or G

W at position 40 can be substituted with Y

L at position 41 can be substituted with I

A at position 42 can be substituted with T or V

A at position 58 can be substituted with G, T, D or V

A at position 67 can be substituted with T or V

S at position 68 can be substituted with Y

S at position 69 can be substituted with R, N, T, G or I

L at position 70 can be substituted with F

Q at position 71 can be substituted with E

**FIGURE 57**

S at position 72 can be substituted with G, N or D

Q at position 107 can be substituted with H

A at position 109 can be substituted with T, G, S, V or D

N at position 110 can be substituted with D, K or S

S at position 111 can be substituted with I

F at position 135 can be substituted with L, I or V

F at position 137 can be substituted with I

Arg Xaa Xaa Xaa Xaa Xaa Xaa Xaa Xaa Xaa Xaa (SEQ ID NO: 50147)

Xaa Xaa Xaa Xaa Xaa Xaa Xaa (SEQ ID NO: 50148)

Xaa Gln Xaa Xaa Xaa Xaa Pro Xaa Thr (SEQ ID NO: 50149)

Arg Ala Ser Gln Gly Ile Ser Arg Trp Leu Ala (SEQ ID NO: 50633)

Ala Ala Ser Ser Leu Gln Ser (SEQ ID NO: 50634)

Gln Gln Ala Asn Ser Phe Pro Phe Thr (SEQ ID NO: 50635)

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | V | S | A | S | V | G | D | R | V | T | I | T | C | R | A | S |
| 21-225_10G6_LC | . | . | . | . | . | . | . | . | . | . | . | . | . | . | . | . | . | . | . | . | . | . | . | . | . | . |
| 21-225_12E9_LC | . | . | . | . | . | . | . | . | . | . | . | . | . | . | . | R | . | . | . | . | . | . | . | . | . | . |
| 21-225_146A6_LC | . | . | . | . | . | . | . | . | A | . | . | . | . | . | . | . | . | . | . | . | . | . | . | . | . | . |
| 21-225_146E1_LC | . | . | K | . | . | . | . | . | . | . | . | . | . | . | . | . | . | . | . | . | . | . | . | . | . | . |
| 21-225_147D10_LC | . | . | . | . | . | . | . | . | A | . | . | . | . | . | . | . | . | . | . | . | . | . | . | . | . | . |
| 21-225_147D12_LC | . | . | . | . | . | . | . | . | A | . | . | . | . | . | . | . | . | . | . | . | . | . | . | . | . | . |
| 21-225_148E2_LC | . | . | . | . | . | . | . | . | A | . | . | . | . | . | . | . | . | . | . | . | . | . | . | . | . | . |
| 21-225_149B6_LC | . | . | . | . | . | . | . | . | . | . | . | . | . | . | . | . | . | . | . | . | . | . | . | . | . | . |
| 21-225_149C6_LC | . | . | . | . | . | . | . | . | A | . | . | . | . | . | . | . | . | . | . | . | . | . | . | . | . | . |
| 21-225_149D11_LC | . | . | . | . | . | . | . | . | A | . | . | . | . | . | . | . | . | . | . | . | . | . | . | . | . | . |
| 21-225_149F8_LC | . | . | . | . | . | . | . | . | A | . | . | . | . | . | . | . | . | . | . | . | . | . | . | . | . | . |
| 21-225_150C5_LC | . | . | . | . | . | . | . | . | G | . | . | . | . | . | . | . | . | . | . | . | . | . | . | . | . | . |
| 21-225_152G10_LC | . | . | . | . | . | . | . | . | A | . | . | . | . | . | . | . | . | . | . | . | . | . | . | . | . | . |
| 21-225_152H7_LC | . | . | . | . | . | . | . | . | A | . | . | . | . | . | . | . | . | . | . | . | . | . | . | . | . | . |
| 21-225_155A4_LC | . | . | . | . | . | . | . | . | A | . | . | . | . | . | . | . | . | . | . | . | . | . | . | . | . | . |
| 21-225_155B4_LC | . | . | . | . | . | . | . | . | A | . | . | . | . | . | . | . | . | . | . | . | . | . | . | . | . | . |
| 21-225_180A3_LC | . | . | . | . | . | . | . | . | . | . | . | . | . | . | . | . | . | . | . | . | . | . | . | . | . | . |
| 21-225_182H5_LC | . | . | . | . | . | . | . | . | . | . | . | Y | . | . | . | . | . | . | . | . | . | . | . | . | . | . |
| 21-225_186A11_LC | . | . | . | . | . | . | . | . | . | . | . | . | . | . | . | . | . | . | . | . | . | . | . | . | . | . |
| 21-225_192H2_LC | . | . | . | . | . | . | . | . | . | . | . | . | . | . | . | . | . | . | . | . | . | . | . | . | . | . |
| 21-225_196F2_LC | . | . | . | . | . | . | . | . | . | . | . | . | . | . | . | . | . | . | . | . | . | . | . | . | . | . |
| 21-225_210H11_LC | . | . | . | . | . | . | . | . | . | . | . | . | . | . | . | . | . | . | . | . | . | . | . | . | . | . |
| 21-225_221A6_LC | . | . | . | . | . | . | . | . | . | . | . | . | . | . | . | . | . | . | . | . | . | . | . | . | . | . |
| 21-225_221F12_LC | . | . | . | . | . | . | . | . | . | . | . | . | . | . | . | . | . | . | . | . | . | . | . | . | . | . |
| 21-225_222C12_LC | . | . | . | . | . | . | C | . | . | . | . | . | . | . | . | . | . | . | . | . | . | . | . | . | V | . |
| 21-225_222E1_LC | . | . | . | . | . | . | . | . | . | . | . | . | . | . | . | . | . | . | . | . | . | . | . | . | . | . |
| 21-225_222F7_LC | . | . | . | . | . | . | . | . | . | . | . | . | . | . | . | . | . | . | . | . | . | . | . | . | . | . |
| 21-225_23B11_LC | . | . | . | . | . | . | . | . | . | . | . | . | . | . | . | . | . | . | . | . | . | . | . | . | . | . |
| 21-225_25E11_LC | . | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | V | S | A | S | V | G | D | R | V | T | I | T | C | R | A | S |
| 21-225_28B1_LC | . | . | . | . | . | . | . | . | . | . | . | . | . | . | . | . | . | . | . | . | . | . | . | . | . | . |
| 21-225_28F11_LC | . | . | . | . | . | . | . | . | . | . | . | . | . | . | . | . | . | . | . | . | . | . | . | . | . | . |
| 21-225_30A11_LC | . | . | . | . | . | . | . | . | . | . | . | . | . | . | . | . | . | . | . | . | . | . | . | . | . | . |
| 21-225_31C4_LC | . | . | . | . | . | . | . | . | . | . | . | . | . | . | . | . | . | . | . | . | . | . | . | . | . | . |
| 21-225_31H9_LC | . | . | . | . | . | . | . | . | . | . | . | . | . | . | . | . | . | . | . | . | . | . | . | . | . | . |
| 21-225_33G1_LC | . | . | . | . | . | . | . | . | . | . | . | . | V | . | . | . | . | . | . | . | . | . | . | . | . | . |
| 21-225_33H7_LC | . | . | . | . | . | . | . | . | . | . | . | . | . | . | . | . | . | . | . | . | . | . | . | . | . | . |
| 21-225_34F3_LC | . | . | . | . | . | . | . | . | . | . | . | . | . | T | . | . | . | . | . | . | S | . | . | . | . | . |
| 21-225_34G7_LC | . | . | . | . | . | . | . | . | . | . | . | . | . | . | . | . | . | . | . | . | . | . | . | . | . | . |
| 21-225_35C6_LC | . | . | . | . | . | . | . | . | . | . | . | . | . | . | . | . | . | . | . | . | . | . | . | . | . | . |
| 21-225_35G6_LC | . | . | . | . | . | . | . | . | . | . | . | . | . | . | . | . | . | . | L | . | . | . | . | . | . | . |
| 21-225_35H12_LC | . | . | . | . | . | . | . | . | . | . | . | . | . | . | . | . | . | . | . | . | . | . | . | . | . | . |
| 21-225_36H5_LC | . | . | . | . | . | . | . | . | . | . | . | . | . | . | . | . | . | . | . | . | . | . | . | . | . | . |
| 21-225_43H9_LC | . | . | . | . | . | . | . | . | . | . | . | . | . | . | . | . | . | . | . | . | . | . | . | . | . | . |
| 21-225_44F3_LC | . | . | . | . | . | . | . | . | . | . | . | . | . | . | . | . | . | . | . | . | . | . | . | . | . | . |
| 21-225_45C9_LC | . | . | . | . | . | . | . | . | . | . | . | . | V | . | . | . | . | . | . | . | . | . | . | . | . | . |
| 21-225_45H4_LC | . | . | . | . | . | . | . | . | . | . | . | . | . | . | . | . | . | . | . | . | . | . | . | . | . | . |
| 21-225_49F1_LC | . | . | . | . | . | . | . | . | . | . | . | . | . | . | . | . | . | . | . | . | . | . | . | . | . | . |
| 21-225_49F10_LC | . | . | . | . | . | . | . | . | . | . | . | . | . | . | . | . | . | . | . | . | . | . | . | . | . | . |
| 21-225_49H5_LC | . | . | . | . | . | . | . | . | . | . | . | . | . | . | . | . | . | . | . | . | . | . | . | . | . | . |
| 21-225_4H6_LC | . | . | . | . | . | . | . | . | . | . | . | . | . | . | . | . | . | . | . | . | . | . | . | . | . | . |
| 21-225_4H6.004_LC | . | . | . | . | . | . | . | . | . | . | . | . | . | . | . | . | . | . | . | . | . | . | . | . | . | . |
| 21-225_4H6.005_LC | . | . | . | . | . | . | . | . | . | . | . | . | . | . | . | . | . | . | . | . | . | . | . | . | . | . |
| 21-225_4H6.006_LC | . | . | . | . | . | . | . | . | . | . | . | . | . | . | . | . | . | . | . | . | . | . | . | . | . | . |
| 21-225_4H6.007_LC | . | . | . | . | . | . | . | . | . | . | . | . | . | . | . | . | . | . | . | . | . | . | . | . | . | . |
| 21-225_4H6.008_LC | . | . | . | . | . | . | . | . | . | . | . | . | . | . | . | . | . | . | . | . | . | . | . | . | . | . |
| 21-225_4H6.009_LC | . | . | . | . | . | . | . | . | . | . | . | . | . | . | . | . | . | . | . | . | . | . | . | . | . | . |
| 21-225_4H6.010_LC | . | . | . | . | . | . | . | . | . | . | . | . | . | . | . | . | . | . | . | . | . | . | . | . | . | . |
| 21-225_4H6.011_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | V | S | A | S | V | G | D | R | V | T | I | T | C | R | A | S |
| 21-225_4H6.014_LC | . | . | . | . | . | . | . | . | . | . | . | . | . | . | . | . | . | . | . | . | . | . | . | . | . | . |
| 21-225_50D4_LC | . | . | . | . | . | . | . | . | . | . | . | . | . | . | . | . | . | . | L | . | . | . | . | . | . | . |
| 21-225_50F3_LC | . | . | . | . | . | . | . | . | . | . | . | . | . | . | . | . | . | . | . | . | . | . | . | . | . | . |
| 21-225_51C7_LC | . | . | . | . | . | . | . | . | . | . | . | . | . | . | . | . | . | . | . | . | . | . | . | . | . | . |
| 21-225_51E9_LC | . | . | . | . | . | . | . | . | . | . | . | . | . | . | . | . | . | . | . | . | . | . | . | . | . | . |
| 21-225_52B1_LC | . | . | . | . | . | . | . | . | . | . | . | . | T | F | . | . | . | . | I | . | . | . | . | . | . | . |
| 21-225_52G11_LC | . | . | . | . | . | . | . | . | . | . | . | . | . | . | . | . | . | . | . | . | . | . | . | . | . | . |
| 21-225_52H10_LC | . | . | . | . | . | . | . | . | . | . | . | . | . | . | . | . | . | . | . | . | . | . | . | . | . | . |
| 21-225_53F2_LC | . | . | . | . | . | . | . | . | . | . | . | . | . | . | . | . | . | . | . | . | . | . | . | . | . | . |
| 21-225_53F7_LC | . | . | . | . | S | . | . | . | . | . | . | . | . | . | . | . | . | . | . | . | . | . | . | . | . | . |
| 21-225_55B1_LC | . | . | . | . | . | . | . | . | . | . | . | . | . | . | . | . | . | . | . | . | . | . | . | . | . | . |
| 21-225_56C6_LC | . | . | . | . | . | . | . | . | . | . | . | . | . | . | . | . | . | . | . | . | . | . | . | . | . | . |
| 21-225_56E5_LC | . | . | . | . | . | . | . | . | . | . | . | . | . | . | . | . | . | . | . | . | . | . | . | . | . | . |
| 21-225_56F6_LC | . | . | . | . | . | . | . | . | . | . | . | C | . | Y | . | . | . | . | . | . | . | . | . | . | V | . |
| 21-225_57A7_LC | . | . | . | . | . | . | . | . | . | . | . | . | . | . | . | . | . | . | . | . | . | . | . | . | . | . |
| 21-225_57E4_LC | . | . | . | . | . | . | . | . | . | . | . | . | . | . | . | . | . | . | . | S | . | . | . | . | . | . |
| 21-225_64E2_LC | . | . | . | . | . | . | . | . | . | . | . | . | . | . | . | . | . | . | . | . | . | . | . | . | . | . |
| 21-225_64G12_LC | . | . | . | . | . | . | . | . | . | . | . | . | . | . | . | . | . | . | . | . | . | . | . | . | . | . |
| 21-225_65F10_LC | . | . | . | . | . | . | . | . | . | . | . | . | . | . | . | . | . | . | . | . | . | . | . | . | G | G |
| 21-225_66F11_LC | . | . | . | . | . | . | . | . | . | . | . | C | . | . | . | . | . | . | . | . | . | . | . | . | E | . |
| 21-225_70D1_LC | . | . | . | . | . | . | . | . | . | . | . | . | . | . | . | . | . | . | . | . | . | . | . | . | . | . |
| 21-225_7C3_LC2_LC | . | . | . | . | . | . | . | . | . | . | . | . | . | . | L | . | . | . | . | . | . | . | . | . | . | . |
| 21-225_9F12_LC1_LC | . | . | . | . | . | . | . | . | . | . | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | S | - | - | - | - | - | - | R | W | L | A | W | Y | Q | Q | K | P | G | K | A | P |
| 21-225_10G6_LC | . | . | . | . | . | . | . | . | . | . | . | . | K | . | . | . | . | . | . | . | . | . | . | . | . | . |
| 21-225_12E9_LC | . | . | . | . | . | . | . | . | . | . | . | . | S | . | . | . | . | . | . | . | . | . | . | E | . | . |
| 21-225_146A6_LC | . | . | . | . | . | . | . | . | . | . | . | . | N | . | . | . | . | . | . | . | . | . | . | . | . | . |
| 21-225_146E1_LC | . | . | . | . | . | N | . | . | . | . | . | . | N | . | . | V | . | . | . | . | . | . | . | . | . | . |
| 21-225_147D10_LC | . | . | . | N | . | . | . | . | . | . | . | . | N | . | . | T | . | . | . | . | . | . | . | . | . | . |
| 21-225_147D12_LC | . | . | . | . | . | . | . | . | . | . | . | . | N | . | . | . | . | . | . | E | . | . | . | . | . | . |
| 21-225_148E2_LC | . | . | . | . | . | . | . | . | . | . | . | . | N | . | . | . | . | . | . | . | . | . | . | . | . | . |
| 21-225_149B6_LC | . | . | . | . | . | I | . | . | . | . | . | . | S | . | . | . | . | . | . | . | . | . | . | . | . | . |
| 21-225_149C6_LC | . | . | . | . | . | . | . | . | . | . | . | . | N | . | . | . | . | . | . | . | . | . | . | R | . | . |
| 21-225_149D11_LC | . | . | . | N | . | . | . | . | . | . | . | . | N | . | . | T | . | . | . | . | . | . | . | . | . | . |
| 21-225_149F8_LC | . | . | . | . | . | . | . | . | . | . | . | . | N | . | . | . | . | . | . | . | . | . | . | . | . | . |
| 21-225_150C5_LC | . | . | . | . | . | N | . | . | . | . | . | . | N | . | . | . | . | . | . | . | . | . | . | . | . | . |
| 21-225_152G10_LC | . | . | . | . | . | . | . | . | . | . | . | . | N | . | . | . | . | . | . | . | . | . | . | . | . | . |
| 21-225_152H7_LC | . | . | . | D | . | . | . | . | . | . | . | . | N | . | . | . | . | . | . | . | . | . | . | . | . | . |
| 21-225_155A4_LC | . | . | . | . | . | . | . | . | . | . | . | . | N | . | . | . | . | . | H | . | . | . | . | . | . | . |
| 21-225_155B4_LC | . | . | . | D | . | . | . | . | . | . | . | . | N | . | . | . | . | . | . | . | . | . | . | . | . | . |
| 21-225_180A3_LC | . | . | . | D | . | T | . | . | . | . | . | . | S | . | . | . | . | . | . | . | . | . | . | . | . | . |
| 21-225_182H5_LC | . | . | . | D | . | T | . | . | . | . | . | . | S | . | . | . | . | . | . | . | . | . | . | . | . | . |
| 21-225_186A11_LC | . | . | . | D | . | T | . | . | . | . | . | . | S | . | . | . | . | . | . | . | . | . | . | . | . | . |
| 21-225_192H2_LC | . | . | . | . | . | . | . | . | . | . | . | . | S | . | I | . | . | . | . | . | . | . | . | . | . | . |
| 21-225_196F2_LC | . | . | . | . | . | . | . | . | . | . | . | . | S | . | . | . | . | . | . | . | . | . | . | . | . | . |
| 21-225_210H11_LC | . | . | . | . | . | . | . | . | . | . | . | . | I | . | . | . | . | . | . | . | . | . | . | . | . | . |
| 21-225_221A6_LC | . | . | . | . | . | . | . | . | . | . | . | . | S | . | . | . | . | . | . | . | . | . | . | . | . | . |
| 21-225_221F12_LC | . | . | . | . | . | . | . | . | . | . | . | . | S | . | . | . | . | . | . | . | . | . | . | . | . | . |
| 21-225_222C12_LC | . | . | . | . | . | . | . | . | . | . | . | . | S | . | . | . | . | . | . | . | . | . | . | . | . | L |
| 21-225_222E1_LC | . | . | . | . | . | . | . | . | . | . | . | . | S | . | . | . | . | . | . | . | . | . | . | . | . | . |
| 21-225_222F7_LC | . | . | . | . | . | . | . | . | . | . | . | . | S | . | . | . | . | . | . | . | . | . | . | . | . | . |
| 21-225_23B11_LC | . | . | . | . | . | T | . | . | . | . | . | . | K | . | . | . | . | . | . | . | . | . | . | . | . | . |
| 21-225_25E11_LC | . | . | . | . | . | . | . | . | . | . | . | . | N | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | S | - | - | - | - | - | - | R | W | L | A | W | Y | Q | Q | K | P | G | K | A | P |
| 21-225_28B1_LC | . | . | . | A | . | N | . | . | . | . | . | . | D | . | . | . | . | . | . | . | . | . | . | . | . | . |
| 21-225_28F11_LC | . | . | . | . | . | . | . | . | . | . | . | . | N | . | . | . | . | . | . | . | . | . | . | . | . | . |
| 21-225_30A11_LC | . | . | . | . | . | . | . | . | . | . | . | . | . | . | . | . | . | . | . | . | . | . | . | R | . | . |
| 21-225_31C4_LC | . | . | . | . | . | . | . | . | . | . | . | . | K | . | . | . | . | . | . | . | . | . | . | . | . | . |
| 21-225_31H9_LC | . | . | . | . | . | T | . | . | . | . | . | . | S | . | . | T | . | . | . | . | R | . | . | . | . | . |
| 21-225_33G1_LC | . | . | . | . | . | . | . | . | . | . | . | . | . | . | . | . | . | . | . | . | . | . | . | . | . | . |
| 21-225_33H7_LC | . | . | . | . | . | . | . | . | . | . | . | . | . | . | . | . | . | . | . | . | . | V | . | . | V | . |
| 21-225_34F3_LC | . | . | . | . | . | . | . | . | . | . | . | . | K | . | . | . | . | . | . | . | . | . | . | . | . | . |
| 21-225_34G7_LC | . | . | . | L | . | . | . | . | . | . | . | . | K | . | . | . | . | . | . | . | . | . | . | . | . | . |
| 21-225_35C6_LC | . | . | . | . | . | . | . | . | . | . | . | . | K | . | . | . | . | . | . | . | . | . | . | . | . | . |
| 21-225_35G6_LC | . | . | . | . | . | . | . | . | . | . | . | . | . | . | . | . | . | . | . | . | . | V | . | . | V | . |
| 21-225_35H12_LC | . | . | . | . | . | . | . | . | . | . | . | . | . | . | . | . | . | . | . | . | . | . | . | . | . | . |
| 21-225_36H5_LC | . | . | . | . | . | . | . | . | . | . | . | . | . | . | . | . | . | . | . | . | . | . | . | . | . | . |
| 21-225_43H9_LC | . | . | . | D | . | . | . | . | . | . | . | . | S | . | . | . | . | . | . | K | . | . | . | . | . | . |
| 21-225_44F3_LC | . | . | . | . | . | . | . | . | . | . | . | . | D | . | . | . | . | . | . | . | . | . | . | . | . | . |
| 21-225_45C9_LC | . | . | . | D | . | . | . | . | . | . | . | . | D | . | . | . | . | . | . | . | R | . | . | . | . | . |
| 21-225_45H4_LC | . | . | . | D | . | . | . | . | . | . | . | . | S | . | . | . | . | . | . | K | . | . | . | . | . | . |
| 21-225_49F1_LC | . | . | R | D | . | N | . | . | . | . | . | . | G | . | . | . | . | . | . | . | . | . | . | . | . | . |
| 21-225_49F10_LC | . | . | . | . | . | . | . | . | . | . | . | . | . | . | . | . | . | . | . | . | . | . | . | . | . | . |
| 21-225_49H5_LC | . | . | . | . | F | . | . | . | . | . | . | . | T | . | . | . | . | F | . | . | . | . | . | . | . | . |
| 21-225_4H6_LC | . | . | . | . | . | . | . | . | . | . | . | . | . | . | . | . | . | . | . | . | . | . | . | . | . | . |
| 21-225_4H6.004_LC | . | . | . | . | . | . | . | . | . | . | . | . | . | . | . | . | . | . | . | . | . | . | . | . | . | . |
| 21-225_4H6.005_LC | . | . | . | . | . | . | . | . | . | . | . | . | . | . | . | . | . | . | . | . | . | . | . | . | . | . |
| 21-225_4H6.006_LC | . | . | . | . | . | . | . | . | . | . | . | . | . | . | . | . | . | . | . | . | . | . | . | . | . | . |
| 21-225_4H6.007_LC | . | . | . | . | . | . | . | . | . | . | . | . | . | . | . | . | . | . | . | . | . | . | . | . | . | . |
| 21-225_4H6.008_LC | . | . | . | . | . | . | . | . | . | . | . | . | . | . | . | . | . | . | . | . | . | . | . | . | . | . |
| 21-225_4H6.009_LC | . | . | . | . | . | . | . | . | . | . | . | . | . | . | . | . | . | . | . | . | . | . | . | . | . | . |
| 21-225_4H6.010_LC | . | . | . | . | . | . | . | . | . | . | . | . | . | . | . | . | . | . | . | . | . | . | . | . | . | . |
| 21-225_4H6.011_LC | . | . | . | . | . | . | . | . | . | . | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | S | - | - | - | - | - | - | R | W | L | A | W | Y | Q | Q | K | P | G | K | A | P |
| 21-225_4H6.014_LC | . | . | . | . | . | . | . | . | . | . | . | . | . | . | . | . | . | . | . | . | . | . | . | . | . | . |
| 21-225_50D4_LC | . | . | . | . | . | . | . | . | . | . | . | . | . | . | . | . | . | . | . | . | . | . | . | . | . | . |
| 21-225_50F3_LC | . | . | . | D | . | . | . | . | . | . | . | . | S | . | . | . | . | F | . | . | . | . | . | . | . | . |
| 21-225_51C7_LC | . | . | . | D | V | N | . | . | . | . | . | . | N | Y | . | . | . | F | . | . | . | . | . | . | . | . |
| 21-225_51E9_LC | . | . | . | . | . | . | . | . | . | . | . | . | S | . | . | . | . | . | . | . | . | . | . | . | . | . |
| 21-225_52B1_LC | . | . | . | D | . | R | . | . | . | . | . | . | T | . | . | . | . | . | . | . | . | . | . | . | . | . |
| 21-225_52G11_LC | . | . | . | . | . | . | . | . | . | . | . | . | K | . | . | . | . | . | . | . | . | . | . | . | . | . |
| 21-225_52H10_LC | . | . | . | D | . | N | . | . | . | . | . | . | S | . | . | . | . | . | . | . | . | . | . | . | . | . |
| 21-225_53F2_LC | . | . | . | . | . | . | . | . | . | . | . | . | K | . | . | . | . | . | . | . | . | . | . | . | . | . |
| 21-225_53F7_LC | . | . | . | . | . | . | . | . | . | . | . | . | . | . | . | . | . | . | . | . | . | . | . | . | . | . |
| 21-225_55B1_LC | . | . | . | V | . | . | . | . | . | . | . | . | . | . | . | . | . | . | . | . | . | . | . | . | . | . |
| 21-225_56C6_LC | . | . | . | . | . | . | . | . | . | . | . | . | S | . | . | . | . | . | . | . | . | S | . | N | . | . |
| 21-225_56E5_LC | . | . | . | . | . | R | . | . | . | . | . | . | K | . | . | . | . | . | . | . | . | . | . | . | . | . |
| 21-225_56F6_LC | . | . | . | D | . | . | . | . | . | . | . | . | K | . | . | . | . | F | . | . | . | . | . | . | . | . |
| 21-225_57A7_LC | . | . | . | . | . | . | . | . | . | . | . | . | . | . | . | . | . | . | . | . | . | . | . | . | . | . |
| 21-225_57E4_LC | . | . | . | D | . | . | . | . | . | . | . | . | N | . | . | . | . | . | . | . | . | . | . | . | . | . |
| 21-225_64E2_LC | . | . | . | . | . | . | . | . | . | . | . | . | . | . | . | . | . | . | . | . | . | . | . | . | . | . |
| 21-225_64G12_LC | . | . | . | N | . | T | . | . | . | . | . | . | T | . | . | . | . | . | . | . | . | . | . | . | . | . |
| 21-225_65F10_LC | . | . | . | . | . | . | . | . | . | . | . | . | T | . | . | . | . | . | . | . | . | . | . | . | . | . |
| 21-225_66F11_LC | . | . | . | . | . | . | . | . | . | . | . | . | I | . | . | . | . | . | . | . | . | . | . | . | . | . |
| 21-225_70D1_LC | . | . | . | . | V | . | . | . | . | . | . | . | . | . | . | . | . | . | . | . | . | . | . | . | . | . |
| 21-225_7C3_LC2_LC | . | . | . | . | . | . | . | . | . | . | . | . | S | . | . | . | . | . | . | . | . | Q | . | . | . | . |
| 21-225_9F12_LC1_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | Y | A | - | - | - | - | - | - | - | - | A | S | S | L | Q | S | G | V | P | S | R | F |
| 21-225_10G6_LC | . | I | . | . | . | . | . | . | . | . | . | . | . | . | . | Y | . | . | . | . | . | . | . | A | . | . |
| 21-225_12E9_LC | . | . | . | . | . | . | . | . | . | . | . | . | . | . | . | . | R | . | . | . | . | . | . | . | . | . |
| 21-225_146A6_LC | . | . | . | . | . | . | . | . | . | . | . | . | . | . | . | . | . | . | . | G | . | . | . | . | . | . |
| 21-225_146E1_LC | . | . | . | . | . | . | . | . | . | . | . | . | . | . | . | . | . | . | . | . | . | . | . | . | . | . |
| 21-225_147D10_LC | . | . | . | . | . | . | . | . | . | . | . | . | . | . | . | . | . | . | . | . | . | . | . | . | . | . |
| 21-225_147D12_LC | . | . | . | . | . | . | . | . | . | . | . | . | . | . | . | . | . | . | . | . | . | . | . | . | . | . |
| 21-225_148E2_LC | . | . | . | . | . | . | . | . | . | . | . | . | . | . | . | . | . | . | . | . | . | . | . | . | . | . |
| 21-225_149B6_LC | . | . | . | . | . | . | . | . | . | . | . | . | . | . | . | . | . | . | . | . | . | . | . | . | . | . |
| 21-225_149C6_LC | . | . | . | . | . | . | . | . | . | . | . | . | . | . | . | . | . | . | . | G | . | . | . | . | . | . |
| 21-225_149D11_LC | . | . | . | . | . | . | . | . | . | . | . | . | . | . | . | . | . | . | . | . | . | . | . | . | . | . |
| 21-225_149F8_LC | . | . | . | . | . | . | . | . | . | . | . | . | . | . | . | . | . | . | . | . | . | . | . | . | . | . |
| 21-225_150C5_LC | . | . | . | . | . | . | . | . | . | . | . | . | . | . | . | . | . | . | . | G | . | . | . | . | . | . |
| 21-225_152G10_LC | . | . | . | . | S | . | . | . | . | . | . | . | . | . | . | . | . | . | . | . | . | . | . | . | . | . |
| 21-225_152H7_LC | . | . | . | . | . | . | . | . | . | . | . | . | . | . | . | . | . | . | . | G | . | . | . | . | . | . |
| 21-225_155A4_LC | . | . | . | . | . | . | . | . | . | . | . | . | . | . | . | . | . | . | . | G | . | . | . | . | . | . |
| 21-225_155B4_LC | . | . | . | . | . | . | . | . | . | . | . | . | . | . | . | . | . | . | . | G | . | . | . | . | . | . |
| 21-225_180A3_LC | . | . | . | . | . | . | . | . | . | . | . | . | . | . | . | . | . | . | . | . | . | . | . | . | . | . |
| 21-225_182H5_LC | . | . | . | . | . | . | . | . | . | . | . | . | . | . | . | . | . | . | . | . | . | . | . | . | . | . |
| 21-225_186A11_LC | . | . | . | . | . | . | . | . | . | . | . | . | . | . | . | . | . | . | . | . | . | . | . | . | . | . |
| 21-225_192H2_LC | . | . | . | . | . | G | . | . | . | . | . | . | . | . | . | . | . | . | . | . | . | . | . | . | . | . |
| 21-225_196F2_LC | . | . | . | . | . | . | . | . | . | . | . | . | . | . | . | . | . | . | . | . | . | . | . | . | . | . |
| 21-225_210H11_LC | N | . | . | . | . | . | . | . | . | . | . | . | . | . | . | . | . | . | . | . | . | . | . | . | . | . |
| 21-225_221A6_LC | . | . | . | . | . | T | . | . | . | . | . | . | . | . | . | . | N | . | . | . | . | . | . | . | . | . |
| 21-225_221F12_LC | . | . | . | . | . | T | . | . | . | . | . | . | . | . | . | . | N | . | . | . | . | . | . | . | . | . |
| 21-225_222C12_LC | . | . | V | . | . | T | . | . | . | . | . | . | . | . | . | . | N | . | . | . | . | . | . | . | . | . |
| 21-225_222E1_LC | . | . | . | . | . | T | . | . | . | . | . | . | . | . | . | . | N | . | . | . | . | . | . | . | . | . |
| 21-225_222F7_LC | . | . | . | . | . | T | . | . | . | . | . | . | . | . | . | . | N | . | . | . | . | . | . | . | . | . |
| 21-225_23B11_LC | . | V | . | . | . | . | . | . | . | . | . | . | . | . | . | . | . | . | . | . | R | . | . | . | . | . |
| 21-225_25E11_LC | . | . | . | . | . | T | . | . | . | . | . | . | . | . | . | . | . | . | . | G | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | Y | A | - | - | - | - | - | - | - | - | A | S | S | L | Q | S | G | V | P | S | R | F |
| 21-225_28B1_LC | . | . | . | . | . | . | . | . | . | . | . | . | . | . | . | . | . | F | . | . | . | . | . | . | . | . |
| 21-225_28F11_LC | . | . | . | . | S | . | . | . | . | . | . | . | . | . | . | . | N | . | . | . | . | . | . | . | . | . |
| 21-225_30A11_LC | E | . | . | . | . | . | . | . | . | . | . | . | . | . | . | . | R | . | . | . | . | I | . | . | . | . |
| 21-225_31C4_LC | . | P | . | . | . | . | . | . | . | . | . | . | . | . | . | . | . | . | . | . | . | . | . | T | . | . |
| 21-225_31H9_LC | . | . | . | . | . | D | . | . | . | . | . | . | . | . | T | . | . | . | . | . | . | . | . | . | . | . |
| 21-225_33G1_LC | . | . | . | . | . | G | . | . | . | . | . | . | . | . | . | Y | . | . | . | . | . | . | . | . | . | . |
| 21-225_33H7_LC | . | . | . | . | . | . | . | . | . | . | . | . | . | . | . | . | R | . | . | . | . | . | . | . | . | . |
| 21-225_34F3_LC | . | . | . | . | . | . | . | . | . | . | . | . | . | . | . | . | . | . | . | . | . | . | . | . | . | . |
| 21-225_34G7_LC | . | . | . | . | . | . | . | . | . | . | . | . | . | . | . | . | . | . | . | . | . | . | . | L | . | . |
| 21-225_35C6_LC | . | P | . | . | . | . | . | . | . | . | . | . | . | . | . | . | . | . | . | . | . | . | . | T | . | . |
| 21-225_35G6_LC | . | . | . | . | . | . | . | . | . | . | . | . | . | . | . | . | R | . | . | . | . | . | . | . | . | . |
| 21-225_35H12_LC | E | . | . | . | . | . | . | . | . | . | . | . | . | . | . | . | R | . | . | . | . | I | . | . | . | . |
| 21-225_36H5_LC | E | . | . | . | . | . | . | . | . | . | . | . | . | . | . | . | R | . | . | . | . | I | . | . | . | . |
| 21-225_43H9_LC | . | . | . | . | . | D | . | . | . | . | . | . | . | . | . | . | . | . | . | . | . | . | . | . | . | . |
| 21-225_44F3_LC | . | . | . | . | . | . | . | . | . | . | . | . | . | . | . | . | . | . | . | . | . | . | . | . | . | . |
| 21-225_45C9_LC | . | . | . | . | . | . | . | . | . | . | . | . | . | . | . | . | . | . | E | . | . | . | . | . | . | . |
| 21-225_45H4_LC | . | Y | . | . | . | D | . | . | . | . | . | . | . | . | . | . | . | . | . | . | . | . | . | . | . | . |
| 21-225_49F1_LC | . | . | . | . | . | T | . | . | . | . | . | . | . | . | V | . | . | . | . | . | . | . | . | . | . | . |
| 21-225_49F10_LC | . | V | . | . | . | . | . | . | . | . | . | . | . | . | . | . | T | . | . | . | . | . | . | . | . | . |
| 21-225_49H5_LC | . | . | . | . | . | . | . | . | . | . | . | . | . | . | . | . | . | . | . | D | . | . | . | . | . | . |
| 21-225_4H6_LC | . | . | . | . | . | G | . | . | . | . | . | . | . | . | . | . | . | . | . | . | . | . | . | . | . | . |
| 21-225_4H6.004_LC | . | . | . | . | . | G | . | . | . | . | . | . | . | . | . | . | . | . | . | . | . | . | . | . | . | . |
| 21-225_4H6.005_LC | . | . | . | . | . | G | . | . | . | . | . | . | . | . | . | . | . | . | . | . | . | . | . | . | . | . |
| 21-225_4H6.006_LC | . | . | . | . | . | G | . | . | . | . | . | . | . | . | . | . | . | . | . | . | . | . | . | . | . | . |
| 21-225_4H6.007_LC | . | . | . | . | . | G | . | . | . | . | . | . | . | . | . | . | . | . | . | . | . | . | . | . | . | . |
| 21-225_4H6.008_LC | . | . | . | . | . | G | . | . | . | . | . | . | . | . | . | . | . | . | . | . | . | . | . | . | . | . |
| 21-225_4H6.009_LC | . | . | . | . | . | G | . | . | . | . | . | . | . | . | . | . | . | . | . | . | . | . | . | . | . | . |
| 21-225_4H6.010_LC | . | . | . | . | . | G | . | . | . | . | . | . | . | . | . | . | . | . | . | . | . | . | . | . | . | . |
| 21-225_4H6.011_LC | . | . | . | . | . | G | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | Y | A | - | - | - | - | - | - | - | - | A | S | S | L | Q | S | G | V | P | S | R | F |
| 21-225_4H6.014_LC | . | . | . | . | . | G | . | . | . | . | . | . | . | . | . | . | . | . | . | . | . | . | . | . | . | . |
| 21-225_50D4_LC | . | V | . | . | . | . | . | . | . | . | . | . | . | . | . | . | T | . | . | . | . | . | . | . | . | . |
| 21-225_50F3_LC | . | . | . | . | . | . | . | . | . | . | . | . | . | . | . | . | . | . | . | N | . | . | . | . | . | . |
| 21-225_51C7_LC | . | . | . | . | . | . | . | . | . | . | . | . | . | . | . | . | . | . | . | N | . | . | . | . | . | . |
| 21-225_51E9_LC | . | . | . | . | . | V | . | . | . | . | . | . | . | . | . | . | . | . | . | . | . | . | . | . | . | . |
| 21-225_52B1_LC | . | . | . | . | . | . | . | . | . | . | . | . | . | . | . | . | . | . | . | N | . | . | . | . | . | . |
| 21-225_52G11_LC | . | . | . | L | . | . | . | . | . | . | . | . | . | . | . | . | . | . | . | . | . | . | . | . | . | . |
| 21-225_52H10_LC | . | . | . | . | . | V | . | . | . | . | . | . | . | . | . | . | . | . | . | . | . | A | . | . | . | . |
| 21-225_53F2_LC | N | . | . | L | . | . | . | . | . | . | . | . | . | . | . | . | . | . | . | . | . | . | . | . | K | . |
| 21-225_53F7_LC | N | . | . | . | . | . | . | . | . | . | . | . | . | . | . | . | . | . | . | . | . | . | . | . | . | . |
| 21-225_55B1_LC | N | . | . | . | . | . | . | . | . | . | . | . | . | . | . | . | . | . | . | . | . | . | . | . | . | . |
| 21-225_56C6_LC | . | . | . | . | . | . | . | . | . | . | . | . | . | . | . | . | R | . | . | . | . | . | . | . | . | . |
| 21-225_56E5_LC | . | . | . | . | . | . | . | . | . | . | . | . | . | . | . | . | . | . | . | . | . | . | . | . | . | . |
| 21-225_56F6_LC | . | F | . | . | . | V | . | . | . | . | . | . | . | . | . | . | G | . | . | . | . | . | . | . | . | . |
| 21-225_57A7_LC | N | . | . | . | . | . | . | . | . | . | . | . | . | . | . | . | N | . | . | . | . | . | . | . | . | . |
| 21-225_57E4_LC | . | . | . | . | . | . | . | . | . | . | . | . | . | . | . | . | . | . | . | . | . | . | . | . | . | . |
| 21-225_64E2_LC | . | . | . | . | . | . | . | . | . | . | . | . | . | . | . | . | . | . | . | . | . | . | . | . | . | . |
| 21-225_64G12_LC | . | . | . | . | S | . | . | . | . | . | . | . | . | . | . | . | . | . | . | . | . | . | . | . | . | . |
| 21-225_65F10_LC | . | . | . | . | . | . | . | . | . | . | . | . | . | . | . | . | I | . | . | . | . | . | . | . | . | . |
| 21-225_66F11_LC | . | . | . | . | . | . | . | . | . | . | . | . | . | . | . | . | . | . | . | . | . | . | . | . | . | . |
| 21-225_70D1_LC | . | . | . | . | . | . | . | . | . | . | . | . | . | . | . | . | . | . | . | . | . | . | . | . | . | . |
| 21-225_7C3_LC2_LC | . | . | . | . | F | . | . | . | . | . | . | . | . | . | . | . | R | . | . | . | . | A | . | . | . | . |
| 21-225_9F12_LC1_LC | . | . | . | . | . | . | . | . | . | . | . | . | . | . | . | . | R | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_10G6_LC | . | . | . | . | . | . | . | . | . | . | . | . | . | . | . | R | . | . | . | . | . | . | . | . | . | . |
| 21-225_12E9_LC | . | . | . | . | . | . | . | . | . | . | . | . | . | . | . | . | . | . | . | . | . | . | . | . | . | . |
| 21-225_146A6_LC | . | . | . | . | . | . | . | . | . | . | . | . | . | S | . | . | . | . | . | . | . | . | . | . | . | . |
| 21-225_146E1_LC | R | . | . | . | . | . | . | . | . | . | . | . | . | . | . | . | . | . | . | . | . | . | . | . | . | . |
| 21-225_147D10_LC | . | . | . | . | . | . | . | . | . | . | . | . | . | S | . | . | . | . | . | . | . | . | . | . | . | . |
| 21-225_147D12_LC | . | . | . | . | . | . | . | . | . | . | . | . | . | S | . | . | . | . | . | . | . | . | . | . | . | . |
| 21-225_148E2_LC | . | . | N | E | . | . | . | . | . | . | . | . | . | S | . | . | . | . | . | . | . | . | . | . | . | . |
| 21-225_149B6_LC | . | . | . | . | . | . | . | . | . | . | . | . | . | . | . | . | . | . | . | . | . | . | . | . | . | . |
| 21-225_149C6_LC | . | . | . | . | . | . | . | . | . | . | Y | . | . | S | . | . | . | . | . | . | . | . | . | . | . | . |
| 21-225_149D11_LC | . | . | . | . | . | . | . | . | . | . | . | . | . | S | . | . | . | . | . | . | . | . | . | . | . | . |
| 21-225_149F8_LC | . | . | N | E | . | . | . | . | . | . | . | . | . | S | . | . | . | . | . | . | . | . | . | . | I | . |
| 21-225_150C5_LC | . | . | . | . | . | . | . | . | . | . | . | . | . | S | . | . | . | . | . | . | . | . | . | . | . | . |
| 21-225_152G10_LC | . | . | N | E | . | . | . | . | . | . | . | . | . | S | . | . | . | . | . | . | . | . | . | . | . | . |
| 21-225_152H7_LC | . | . | . | . | . | . | . | . | . | . | . | . | . | S | . | . | T | . | . | . | . | . | . | . | . | . |
| 21-225_155A4_LC | . | . | . | . | . | . | . | . | . | . | . | . | . | S | . | . | . | . | . | . | . | . | . | . | . | . |
| 21-225_155B4_LC | N | . | . | . | . | . | . | . | . | . | . | . | . | S | . | . | . | . | . | . | . | . | . | . | . | . |
| 21-225_180A3_LC | . | . | . | . | . | . | . | . | . | . | . | I | . | . | . | . | . | . | . | . | . | . | . | . | F | . |
| 21-225_182H5_LC | . | . | . | . | . | . | . | . | . | . | . | I | . | . | . | T | . | V | . | . | D | . | . | . | . | . |
| 21-225_186A11_LC | . | . | . | . | . | . | . | . | . | . | . | I | . | . | . | T | . | V | . | . | D | . | . | . | . | . |
| 21-225_192H2_LC | . | . | . | . | . | . | . | . | . | . | . | . | . | . | . | . | . | . | . | . | . | . | . | . | . | . |
| 21-225_196F2_LC | . | . | . | . | . | . | . | . | . | . | . | . | . | . | . | . | . | . | . | . | . | . | . | . | . | . |
| 21-225_210H11_LC | . | . | R | . | . | . | . | . | . | . | . | . | . | . | . | . | . | . | . | . | . | . | . | . | . | . |
| 21-225_221A6_LC | . | . | . | . | . | . | . | . | . | . | . | . | . | . | . | . | . | . | . | . | . | . | . | . | . | . |
| 21-225_221F12_LC | . | . | . | . | . | . | . | . | . | . | . | . | . | . | . | . | . | . | . | R | . | . | . | . | . | . |
| 21-225_222C12_LC | . | . | . | . | . | . | . | . | . | . | . | . | . | . | . | . | . | V | . | R | . | . | . | . | . | . |
| 21-225_222E1_LC | . | . | . | . | . | . | . | . | . | . | . | . | . | . | . | . | . | . | . | . | . | . | . | . | . | . |
| 21-225_222F7_LC | . | . | . | . | . | . | . | . | . | . | . | . | . | . | . | . | . | V | . | . | . | . | . | . | . | . |
| 21-225_23B11_LC | . | . | . | R | . | . | . | . | . | . | . | . | . | . | . | . | . | . | . | . | . | . | . | . | . | . |
| 21-225_25E11_LC | . | . | . | . | . | . | . | . | . | . | . | . | I | S | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_28B1_LC | . | . | . | . | . | . | . | . | . | . | . | . | . | . | . | T | . | . | . | . | . | . | . | . | . | . |
| 21-225_28F11_LC | R | . | . | . | . | . | . | . | . | . | . | . | . | . | . | T | . | . | . | . | . | . | . | . | . | . |
| 21-225_30A11_LC | . | . | . | . | . | . | . | . | . | . | . | . | . | . | . | . | . | . | . | . | . | . | . | . | . | . |
| 21-225_31C4_LC | . | . | . | . | . | . | . | . | . | . | . | . | . | . | . | . | . | . | . | . | . | . | . | . | I | . |
| 21-225_31H9_LC | . | . | . | . | . | . | . | . | . | . | . | I | F | . | . | . | . | . | . | . | . | . | . | . | . | . |
| 21-225_33G1_LC | . | . | . | . | . | . | . | . | . | . | . | . | . | . | . | . | . | . | . | . | . | . | . | . | . | . |
| 21-225_33H7_LC | . | . | . | . | . | . | . | . | . | . | . | . | . | . | . | G | . | . | . | . | . | . | . | . | . | . |
| 21-225_34F3_LC | . | . | . | . | . | . | . | . | . | . | . | . | . | . | . | . | . | . | . | . | . | . | S | . | . | . |
| 21-225_34G7_LC | . | . | . | . | . | . | . | . | . | . | . | . | . | . | . | . | . | . | . | . | . | . | . | . | . | . |
| 21-225_35C6_LC | . | . | . | . | . | . | . | . | . | . | . | . | . | . | . | . | . | . | . | . | . | . | . | . | I | . |
| 21-225_35G6_LC | . | . | . | . | . | . | . | . | . | A | . | . | . | . | . | G | . | . | . | . | . | . | . | . | . | . |
| 21-225_35H12_LC | . | . | . | . | . | . | . | . | . | . | . | . | . | . | . | . | . | . | . | . | . | . | . | . | . | . |
| 21-225_36H5_LC | . | . | . | . | . | . | . | . | . | . | . | . | . | . | . | . | . | . | . | . | . | . | . | . | . | . |
| 21-225_43H9_LC | . | . | . | . | . | . | . | . | . | . | . | I | . | . | . | . | . | . | . | . | . | . | . | . | . | . |
| 21-225_44F3_LC | . | . | . | . | F | . | . | . | . | . | . | . | . | . | . | . | . | . | . | . | . | . | . | . | . | . |
| 21-225_45C9_LC | . | . | . | . | . | . | . | . | . | . | . | . | . | . | . | . | . | . | . | . | . | . | . | . | . | . |
| 21-225_45H4_LC | . | . | . | . | . | . | . | . | . | . | . | I | . | . | . | . | . | . | . | . | . | . | . | . | I | . |
| 21-225_49F1_LC | . | . | . | . | . | . | . | . | . | . | . | . | . | . | . | . | . | . | . | . | . | . | . | . | . | . |
| 21-225_49F10_LC | . | . | . | . | . | . | . | . | . | . | . | . | . | . | . | . | . | . | . | . | . | . | . | . | . | . |
| 21-225_49H5_LC | . | . | . | . | . | . | . | . | . | . | . | . | . | . | . | . | . | . | . | . | . | . | . | . | . | . |
| 21-225_4H6_LC | . | . | . | . | . | . | . | . | . | . | . | . | . | . | . | . | . | . | . | . | . | . | . | . | I | . |
| 21-225_4H6.004_LC | . | . | . | . | . | . | . | . | . | . | . | . | . | . | . | . | . | . | . | . | . | . | . | . | I | . |
| 21-225_4H6.005_LC | . | . | . | . | . | . | . | . | . | . | . | . | . | . | . | . | . | . | . | . | . | . | . | . | I | . |
| 21-225_4H6.006_LC | . | . | . | . | . | . | . | . | . | . | . | . | . | . | . | . | . | . | . | . | . | . | . | . | . | . |
| 21-225_4H6.007_LC | . | . | . | . | . | . | . | . | . | . | . | . | . | . | . | . | . | . | . | . | . | . | . | . | I | . |
| 21-225_4H6.008_LC | . | . | . | . | . | . | . | . | . | . | . | . | . | . | . | . | . | . | . | . | . | . | . | . | I | . |
| 21-225_4H6.009_LC | . | . | . | . | . | . | . | . | . | . | . | . | . | . | . | . | . | . | . | . | . | . | . | . | I | . |
| 21-225_4H6.010_LC | . | . | . | . | . | . | . | . | . | . | . | . | . | . | . | . | . | . | . | . | . | . | . | . | I | . |
| 21-225_4H6.011_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_4H6.014_LC | . | . | . | . | . | . | . | . | . | . | . | . | . | . | . | . | . | . | . | . | . | . | . | . | . | . |
| 21-225_50D4_LC | . | . | . | . | . | . | . | . | . | . | . | . | . | . | . | . | . | . | . | . | . | . | . | . | . | . |
| 21-225_50F3_LC | . | . | . | . | . | . | . | . | . | . | . | . | . | . | . | . | . | . | . | . | . | . | . | . | . | . |
| 21-225_51C7_LC | . | . | . | . | . | . | . | . | . | . | . | . | . | . | . | . | . | . | L | . | . | . | . | . | . | . |
| 21-225_51E9_LC | . | . | . | . | . | . | . | . | . | . | . | . | . | . | . | R | . | . | . | . | . | . | . | . | . | . |
| 21-225_52B1_LC | . | . | . | . | . | . | . | . | . | . | . | . | . | . | . | . | . | . | . | . | . | . | . | . | . | . |
| 21-225_52G11_LC | . | A | . | . | . | . | . | . | . | . | . | . | . | . | . | . | . | . | . | . | . | . | . | . | . | . |
| 21-225_52H10_LC | . | . | . | . | . | . | . | . | . | . | . | . | . | . | . | R | . | . | . | . | . | . | . | . | . | . |
| 21-225_53F2_LC | . | . | . | . | . | . | . | . | . | . | . | . | . | . | . | . | . | . | . | . | . | . | . | . | . | . |
| 21-225_53F7_LC | . | . | . | . | . | . | . | . | . | . | . | . | . | . | . | . | . | . | . | . | . | . | . | . | I | . |
| 21-225_55B1_LC | . | . | . | . | . | . | . | . | . | . | . | . | . | . | . | . | . | . | . | . | . | . | . | . | . | . |
| 21-225_56C6_LC | . | . | . | . | . | . | . | . | . | Y | . | . | . | I | . | . | . | . | . | S | . | . | . | . | . | . |
| 21-225_56E5_LC | . | . | . | . | . | . | . | . | . | . | . | . | . | . | . | . | . | . | . | . | . | . | . | . | . | . |
| 21-225_56F6_LC | . | . | . | . | . | . | . | . | . | . | . | . | F | . | . | . | . | . | . | . | . | . | . | . | . | . |
| 21-225_57A7_LC | . | . | . | . | . | . | . | . | . | . | . | . | . | . | . | . | . | . | . | . | . | . | . | . | . | . |
| 21-225_57E4_LC | . | . | . | . | . | . | . | . | . | . | . | . | . | . | . | . | . | . | . | . | . | . | . | . | . | . |
| 21-225_64E2_LC | . | . | . | . | . | . | . | . | . | . | . | . | . | . | . | . | . | . | . | . | . | . | . | . | . | . |
| 21-225_64G12_LC | . | . | . | . | . | . | . | . | . | . | . | . | . | . | . | . | . | . | . | . | . | . | . | . | . | . |
| 21-225_65F10_LC | R | . | R | . | . | . | . | . | . | . | . | . | . | . | . | . | . | . | . | . | . | . | . | . | . | . |
| 21-225_66F11_LC | . | . | . | . | Y | . | . | . | . | . | . | . | . | . | . | . | . | V | . | . | . | . | . | . | . | . |
| 21-225_70D1_LC | . | . | . | . | . | . | . | . | . | . | . | . | V | . | . | . | . | . | . | . | . | . | . | . | . | . |
| 21-225_7C3_LC2_LC | . | . | . | . | . | . | . | . | . | . | . | . | . | . | . | . | . | . | . | . | . | . | . | . | . | . |
| 21-225_9F12_LC1_LC | . | . | . | . | . | . | . | . | . | . | . | . | . | . | . | . | G | . | L | . | . | . | . | . | I | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | A | N | S | - | - | - | - | - | - | - | - | - | - | - | - | - | - | - | - | - | - | - |
| 21-225_10G6_LC | . | . | . | . | . | . | . | . | . | . | . | . | . | . | . | . | . | . | . | . | . | . | . | . | . | . |
| 21-225_12E9_LC | . | . | . | . | . | . | . | . | . | . | . | . | . | . | . | . | . | . | . | . | . | . | . | . | . | . |
| 21-225_146A6_LC | . | . | . | . | T | D | . | . | . | . | . | . | . | . | . | . | . | . | . | . | . | . | . | . | . | . |
| 21-225_146E1_LC | . | . | . | . | . | . | . | . | . | . | . | . | . | . | . | . | . | . | . | . | . | . | . | . | . | . |
| 21-225_147D10_LC | . | . | . | . | T | D | . | . | . | . | . | . | . | . | . | . | . | . | . | . | . | . | . | . | . | . |
| 21-225_147D12_LC | . | . | . | . | T | D | . | . | . | . | . | . | . | . | . | . | . | . | . | . | . | . | . | . | . | . |
| 21-225_148E2_LC | . | . | . | . | T | D | . | . | . | . | . | . | . | . | . | . | . | . | . | . | . | . | . | . | . | . |
| 21-225_149B6_LC | . | . | . | . | G | . | . | . | . | . | . | . | . | . | . | . | . | . | . | . | . | . | . | . | . | . |
| 21-225_149C6_LC | . | . | . | . | T | D | . | . | . | . | . | . | . | . | . | . | . | . | . | . | . | . | . | . | . | . |
| 21-225_149D11_LC | . | . | . | . | T | D | . | . | . | . | . | . | . | . | . | . | . | . | . | . | . | . | . | . | . | . |
| 21-225_149F8_LC | . | . | . | . | T | D | . | . | . | . | . | . | . | . | . | . | . | . | . | . | . | . | . | . | . | . |
| 21-225_150C5_LC | . | . | . | . | T | D | . | . | . | . | . | . | . | . | . | . | . | . | . | . | . | . | . | . | . | . |
| 21-225_152G10_LC | . | . | . | . | T | D | . | . | . | . | . | . | . | . | . | . | . | . | . | . | . | . | . | . | . | . |
| 21-225_152H7_LC | . | . | . | . | T | D | . | . | . | . | . | . | . | . | . | . | . | . | . | . | . | . | . | . | . | . |
| 21-225_155A4_LC | . | . | H | . | T | D | . | . | . | . | . | . | . | . | . | . | . | . | . | . | . | . | . | . | . | . |
| 21-225_155B4_LC | . | . | H | . | T | D | . | . | . | . | . | . | . | . | . | . | . | . | . | . | . | . | . | . | . | . |
| 21-225_180A3_LC | H | . | . | . | . | . | . | . | . | . | . | . | . | . | . | . | . | . | . | . | . | . | . | . | . | . |
| 21-225_182H5_LC | . | . | . | . | V | . | . | . | . | . | . | . | . | . | . | . | . | . | . | . | . | . | . | . | . | . |
| 21-225_186A11_LC | . | . | . | . | V | . | . | . | . | . | . | . | . | . | . | . | . | . | . | . | . | . | . | . | . | . |
| 21-225_192H2_LC | . | . | . | . | S | . | . | . | . | . | . | . | . | . | . | . | . | . | . | . | . | . | . | . | . | . |
| 21-225_196F2_LC | . | . | . | . | T | . | . | . | . | . | . | . | . | . | . | . | . | . | . | . | . | . | . | . | . | . |
| 21-225_210H11_LC | . | . | . | . | . | K | . | . | . | . | . | . | . | . | . | . | . | . | . | . | . | . | . | . | . | . |
| 21-225_221A6_LC | . | . | . | . | . | . | . | . | . | . | . | . | . | . | . | . | . | . | . | . | . | . | . | . | . | . |
| 21-225_221F12_LC | . | . | . | . | . | . | . | . | . | . | . | . | . | . | . | . | . | . | . | . | . | . | . | . | . | . |
| 21-225_222C12_LC | . | . | . | . | D | . | . | . | . | . | . | . | . | . | . | . | . | . | . | . | . | . | . | . | . | . |
| 21-225_222E1_LC | . | . | . | . | . | . | . | . | . | . | . | . | . | . | . | . | . | . | . | . | . | . | . | . | . | . |
| 21-225_222F7_LC | . | . | . | . | . | . | . | . | . | . | . | . | . | . | . | . | . | . | . | . | . | . | . | . | . | . |
| 21-225_23B11_LC | . | . | . | . | . | . | . | . | . | . | . | . | . | . | . | . | . | . | . | . | . | . | . | . | . | . |
| 21-225_25E11_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | A | N | S | - | - | - | - | - | - | - | - | - | - | - | - | - | - | - | - | - | - | - |
| 21-225_28B1_LC | . | . | . | . | . | S | . | . | . | . | . | . | . | . | . | . | . | . | . | . | . | . | . | . | . | . |
| 21-225_28F11_LC | C | . | . | . | . | . | . | . | . | . | . | . | . | . | . | . | . | . | . | . | . | . | . | . | . | . |
| 21-225_30A11_LC | . | . | . | . | . | . | . | . | . | . | . | . | . | . | . | . | . | . | . | . | . | . | . | . | . | . |
| 21-225_31C4_LC | . | . | . | . | . | . | . | . | . | . | . | . | . | . | . | . | . | . | . | . | . | . | . | . | . | . |
| 21-225_31H9_LC | . | . | . | . | G | . | . | . | . | . | . | . | . | . | . | . | . | . | . | . | . | . | . | . | . | . |
| 21-225_33G1_LC | . | . | . | . | . | . | . | . | . | . | . | . | . | . | . | . | . | . | . | . | . | . | . | . | . | . |
| 21-225_33H7_LC | . | . | . | . | . | . | I | . | . | . | . | . | . | . | . | . | . | . | . | . | . | . | . | . | . | . |
| 21-225_34F3_LC | . | . | . | . | . | . | . | . | . | . | . | . | . | . | . | . | . | . | . | . | . | . | . | . | . | . |
| 21-225_34G7_LC | . | . | . | . | . | . | . | . | . | . | . | . | . | . | . | . | . | . | . | . | . | . | . | . | . | . |
| 21-225_35C6_LC | . | . | . | . | . | . | . | . | . | . | . | . | . | . | . | . | . | . | . | . | . | . | . | . | . | . |
| 21-225_35G6_LC | . | . | . | . | . | . | . | . | . | . | . | . | . | . | . | . | . | . | . | . | . | . | . | . | . | . |
| 21-225_35H12_LC | . | . | . | . | . | . | . | . | . | . | . | . | . | . | . | . | . | . | . | . | . | . | . | . | . | . |
| 21-225_36H5_LC | . | . | . | . | . | . | . | . | . | . | . | . | . | . | . | . | . | . | . | . | . | . | . | . | . | . |
| 21-225_43H9_LC | F | . | . | . | . | . | . | . | . | . | . | . | . | . | . | . | . | . | . | . | . | . | . | . | . | . |
| 21-225_44F3_LC | . | . | . | . | . | . | . | . | . | . | . | . | . | . | . | . | . | . | . | . | . | . | . | . | . | . |
| 21-225_45C9_LC | . | . | . | . | . | . | . | . | . | . | . | . | . | . | . | . | . | . | . | . | . | . | . | . | . | . |
| 21-225_45H4_LC | F | . | . | . | . | . | . | . | . | . | . | . | . | . | . | . | . | . | . | . | . | . | . | . | . | . |
| 21-225_49F1_LC | . | . | . | . | S | . | . | . | . | . | . | . | . | . | . | . | . | . | . | . | . | . | . | . | . | . |
| 21-225_49F10_LC | . | . | . | . | . | . | . | . | . | . | . | . | . | . | . | . | . | . | . | . | . | . | . | . | . | . |
| 21-225_49H5_LC | . | . | . | . | T | . | . | . | . | . | . | . | . | . | . | . | . | . | . | . | . | . | . | . | . | . |
| 21-225_4H6_LC | . | . | . | . | . | . | . | . | . | . | . | . | . | . | . | . | . | . | . | . | . | . | . | . | . | . |
| 21-225_4H6.004_LC | . | . | . | . | . | . | . | . | . | . | . | . | . | . | . | . | . | . | . | . | . | . | . | . | . | . |
| 21-225_4H6.005_LC | . | . | . | . | . | . | . | . | . | . | . | . | . | . | . | . | . | . | . | . | . | . | . | . | . | . |
| 21-225_4H6.006_LC | . | . | . | . | . | . | . | . | . | . | . | . | . | . | . | . | . | . | . | . | . | . | . | . | . | . |
| 21-225_4H6.007_LC | . | . | . | . | . | . | . | . | . | . | . | . | . | . | . | . | . | . | . | . | . | . | . | . | . | . |
| 21-225_4H6.008_LC | . | . | . | . | . | . | . | . | . | . | . | . | . | . | . | . | . | . | . | . | . | . | . | . | . | . |
| 21-225_4H6.009_LC | . | . | . | . | . | . | . | . | . | . | . | . | . | . | . | . | . | . | . | . | . | . | . | . | . | . |
| 21-225_4H6.010_LC | . | . | . | . | . | . | . | . | . | . | . | . | . | . | . | . | . | . | . | . | . | . | . | . | . | . |
| 21-225_4H6.011_LC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | A | N | S | - | - | - | - | - | - | - | - | - | - | - | - | - | - | - | - | - | - | - |
| 21-225_4H6.014_LC | . | . | . | . | . | . | . | . | . | . | . | . | . | . | . | . | . | . | . | . | . | . | . | . | . | . |
| 21-225_50D4_LC | . | . | . | . | . | . | . | . | . | . | . | . | . | . | . | . | . | . | . | . | . | . | . | . | . | . |
| 21-225_50F3_LC | . | . | . | . | T | . | . | . | . | . | . | . | . | . | . | . | . | . | . | . | . | . | . | . | . | . |
| 21-225_51C7_LC | . | . | . | . | T | . | . | . | . | . | . | . | . | . | . | . | . | . | . | . | . | . | . | . | . | . |
| 21-225_51E9_LC | . | . | . | . | . | K | . | . | . | . | . | . | . | . | . | . | . | . | . | . | . | . | . | . | . | . |
| 21-225_52B1_LC | F | . | . | . | . | K | . | . | . | . | . | . | . | . | . | . | . | . | . | . | . | . | . | . | . | . |
| 21-225_52G11_LC | . | . | . | . | . | . | . | . | . | . | . | . | . | . | . | . | . | . | . | . | . | . | . | . | . | . |
| 21-225_52H10_LC | . | . | . | . | . | K | . | . | . | . | . | . | . | . | . | . | . | . | . | . | . | . | . | . | . | . |
| 21-225_53F2_LC | . | . | . | . | . | . | . | . | . | . | . | . | . | . | . | . | . | . | . | . | . | . | . | . | . | . |
| 21-225_53F7_LC | . | . | . | . | . | . | . | . | . | . | . | . | . | . | . | . | . | . | . | . | . | . | . | . | . | . |
| 21-225_55B1_LC | . | . | . | . | . | . | . | . | . | . | . | . | . | . | . | . | . | . | . | . | . | . | . | . | . | . |
| 21-225_56C6_LC | . | . | . | . | . | . | . | . | . | . | . | . | . | . | . | . | . | . | . | . | . | . | . | . | . | . |
| 21-225_56E5_LC | . | . | . | . | . | . | . | . | . | . | . | . | . | . | . | . | . | . | . | . | . | . | . | . | . | . |
| 21-225_56F6_LC | . | . | . | . | . | . | . | . | . | . | . | . | . | . | . | . | . | . | . | . | . | . | . | . | . | . |
| 21-225_57A7_LC | . | . | . | . | . | . | . | . | . | . | . | . | . | . | . | . | . | . | . | . | . | . | . | . | . | . |
| 21-225_57E4_LC | S | . | . | . | G | . | . | . | . | . | . | . | . | . | . | . | . | . | . | . | . | . | . | . | . | . |
| 21-225_64E2_LC | . | . | . | . | . | . | . | . | . | . | . | . | . | . | . | . | . | . | . | . | . | . | . | . | . | . |
| 21-225_64G12_LC | I | . | . | . | . | . | . | . | . | . | . | . | . | . | . | . | . | . | . | . | . | . | . | . | . | . |
| 21-225_65F10_LC | . | . | . | . | G | . | I | . | . | . | . | . | . | . | . | . | . | . | . | . | . | . | . | . | . | . |
| 21-225_66F11_LC | . | . | . | . | . | . | . | . | . | . | . | . | . | . | . | . | . | . | . | . | . | . | . | . | . | . |
| 21-225_70D1_LC | . | . | . | . | . | . | . | . | . | . | . | . | . | . | . | . | . | . | . | . | . | . | . | . | . | . |
| 21-225_7C3_LC2_LC | . | . | . | . | . | . | . | . | . | . | . | . | . | . | . | . | . | . | . | . | . | . | . | . | . | . |
| 21-225_9F12_LC1_LC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | F | P | F | T | F | G | P | G | T | K | V | D | I | K | R |
| 21-225_10G6_LC | . | . | . | . | . | . | . | . | . | . | . | . | . | . | . | . | . | . | . |
| 21-225_12E9_LC | . | . | . | . | . | . | . | . | . | . | . | . | . | . | . | . | . | . | . |
| 21-225_146A6_LC | . | . | . | . | . | . | . | . | . | . | . | . | . | . | . | . | . | . | . |
| 21-225_146E1_LC | . | . | . | . | . | . | . | . | . | . | . | . | . | . | . | . | . | . | . |
| 21-225_147D10_LC | . | . | . | . | . | . | . | . | . | . | . | . | . | . | . | . | V | . | . |
| 21-225_147D12_LC | . | . | . | . | . | . | . | . | . | . | . | . | . | . | . | . | V | . | . |
| 21-225_148E2_LC | . | . | . | . | . | . | . | . | . | . | . | . | . | . | . | . | V | . | . |
| 21-225_149B6_LC | . | . | . | . | . | . | . | . | . | . | . | . | . | . | . | . | . | . | . |
| 21-225_149C6_LC | . | . | . | . | . | . | . | . | . | . | . | . | . | . | . | . | . | . | . |
| 21-225_149D11_LC | . | . | . | . | . | . | . | . | . | . | . | . | . | . | . | . | V | . | . |
| 21-225_149F8_LC | . | . | . | . | . | . | . | . | . | . | . | . | . | . | . | . | V | . | . |
| 21-225_150C5_LC | . | . | . | . | . | . | . | . | . | . | . | . | . | . | . | . | . | . | . |
| 21-225_152G10_LC | . | . | . | . | . | . | . | . | . | . | . | . | . | . | . | . | V | . | . |
| 21-225_152H7_LC | . | . | . | . | . | . | . | . | . | . | . | . | . | . | . | . | . | . | . |
| 21-225_155A4_LC | . | . | . | . | . | . | . | . | . | . | . | . | . | . | . | . | . | . | . |
| 21-225_155B4_LC | . | . | . | . | . | . | . | . | . | . | . | . | . | . | . | . | . | . | . |
| 21-225_180A3_LC | . | . | . | . | I | . | . | . | . | . | . | . | . | . | . | . | . | N | . |
| 21-225_182H5_LC | . | . | . | . | . | . | . | . | . | . | H | . | . | . | . | . | . | . | . |
| 21-225_186A11_LC | . | . | . | . | . | . | . | . | . | . | H | . | . | . | . | . | . | . | . |
| 21-225_192H2_LC | . | . | . | . | . | . | . | . | . | . | . | . | . | . | . | . | V | . | . |
| 21-225_196F2_LC | . | . | . | . | V | . | . | . | . | . | . | . | . | . | . | . | . | . | . |
| 21-225_210H11_LC | . | . | . | . | . | . | . | . | . | . | . | . | . | . | . | . | . | . | . |
| 21-225_221A6_LC | . | . | . | . | . | . | . | . | . | . | . | . | . | . | . | . | . | . | . |
| 21-225_221F12_LC | . | . | . | . | . | . | . | . | . | . | . | . | . | . | . | . | . | . | . |
| 21-225_222C12_LC | . | . | . | . | . | . | . | . | . | . | R | . | . | . | . | . | . | . | . |
| 21-225_222E1_LC | . | . | . | . | . | . | . | . | . | . | R | . | . | . | . | . | . | . | . |
| 21-225_222F7_LC | . | . | . | . | . | . | . | . | . | . | . | . | . | . | . | . | . | . | . |
| 21-225_23B11_LC | . | . | . | . | . | . | . | . | . | . | . | . | . | . | . | . | . | . | . |
| 21-225_25E11_LC | . | . | . | . | . | . | I | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | F | P | F | T | F | G | P | G | T | K | V | D | I | K | R |
| 21-225_28B1_LC | . | . | . | . | . | . | . | . | . | . | . | . | . | . | . | . | . | . | . |
| 21-225_28F11_LC | . | . | . | . | L | . | . | . | . | . | . | . | . | . | . | . | . | . | . |
| 21-225_30A11_LC | . | . | . | . | . | . | . | . | . | . | . | . | . | . | . | . | N | . | . |
| 21-225_31C4_LC | . | . | . | . | . | . | . | . | . | . | . | . | . | . | . | . | . | . | . |
| 21-225_31H9_LC | . | . | . | . | . | . | . | . | . | . | Q | . | . | . | . | . | . | . | . |
| 21-225_33G1_LC | . | . | . | . | . | . | . | . | . | . | . | . | . | . | . | . | . | . | . |
| 21-225_33H7_LC | . | . | . | . | . | . | . | . | . | . | . | . | . | . | . | . | L | . | . |
| 21-225_34F3_LC | . | . | . | . | . | . | . | . | . | . | . | . | . | . | . | . | . | . | . |
| 21-225_34G7_LC | . | . | . | . | . | . | . | . | . | . | . | . | . | . | . | . | . | . | . |
| 21-225_35C6_LC | . | . | . | . | . | . | . | . | . | . | . | . | . | . | . | . | . | . | . |
| 21-225_35G6_LC | . | . | . | . | . | . | . | . | . | . | . | . | . | . | . | . | L | . | . |
| 21-225_35H12_LC | . | . | . | . | . | . | . | . | . | . | . | . | . | . | . | . | . | . | . |
| 21-225_36H5_LC | . | . | . | . | . | . | . | . | . | . | . | . | . | . | . | . | . | . | . |
| 21-225_43H9_LC | . | . | . | . | L | . | . | . | . | . | . | . | . | . | . | . | . | . | . |
| 21-225_44F3_LC | . | . | . | . | . | . | . | . | . | . | . | . | . | . | . | . | . | . | . |
| 21-225_45C9_LC | . | . | . | . | . | . | . | . | . | . | . | . | . | . | . | . | . | . | . |
| 21-225_45H4_LC | . | . | . | . | L | . | . | . | . | . | . | . | . | . | . | . | . | . | . |
| 21-225_49F1_LC | . | . | . | . | . | . | . | . | . | . | . | . | . | . | . | . | . | . | . |
| 21-225_49F10_LC | . | . | . | . | . | . | . | . | . | . | . | . | . | . | . | . | . | . | . |
| 21-225_49H5_LC | . | . | . | . | . | . | . | . | . | . | . | . | . | . | . | . | . | . | . |
| 21-225_4H6_LC | . | . | . | . | . | . | . | . | . | . | . | . | . | . | . | . | . | . | G |
| 21-225_4H6.004_LC | . | . | . | . | . | . | . | . | . | . | . | . | . | . | . | . | . | . | . |
| 21-225_4H6.005_LC | . | . | . | . | . | . | . | . | . | . | Q | . | . | . | . | . | . | . | . |
| 21-225_4H6.006_LC | . | . | . | . | . | . | . | . | . | . | Q | . | . | . | . | . | . | . | . |
| 21-225_4H6.007_LC | . | . | . | . | . | . | . | . | . | . | . | . | . | . | . | . | . | . | . |
| 21-225_4H6.008_LC | . | . | . | . | . | . | . | . | . | . | . | . | . | . | . | . | . | . | . |
| 21-225_4H6.009_LC | . | . | . | . | . | . | . | . | . | . | Q | . | . | . | . | . | . | . | . |
| 21-225_4H6.010_LC | . | . | . | . | . | . | . | . | . | . | Q | . | . | . | . | . | . | . | . |
| 21-225_4H6.011_LC | . | . | . | . | . | . | . | . | . | . | Q | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | F | P | F | T | F | G | P | G | T | K | V | D | I | K | R |
| 21-225_4H6.014_LC | . | . | . | . | . | . | . | . | . | . | . | . | . | . | . | . | . | . | . |
| 21-225_50D4_LC | . | . | . | . | . | . | . | . | . | . | . | . | . | . | . | . | . | R | . |
| 21-225_50F3_LC | . | . | . | . | . | . | . | . | . | . | . | . | . | . | . | . | . | . | . |
| 21-225_51C7_LC | . | . | . | . | . | . | . | . | . | . | . | . | . | . | . | . | . | . | . |
| 21-225_51E9_LC | . | . | . | . | . | . | . | . | . | . | . | . | . | . | . | . | . | . | . |
| 21-225_52B1_LC | . | . | . | . | . | . | . | . | . | . | . | . | . | . | . | . | . | . | . |
| 21-225_52G11_LC | . | . | . | . | . | . | . | . | . | . | . | . | . | . | . | . | V | . | . |
| 21-225_52H10_LC | . | . | . | . | . | . | . | . | . | . | . | . | . | . | . | . | . | . | . |
| 21-225_53F2_LC | . | . | . | . | . | . | . | . | . | . | . | . | . | . | . | . | V | . | . |
| 21-225_53F7_LC | . | . | . | . | . | . | . | . | . | . | . | . | . | . | . | . | . | . | . |
| 21-225_55B1_LC | . | . | . | . | . | . | . | . | . | . | . | . | . | . | . | . | L | . | . |
| 21-225_56C6_LC | . | . | . | . | . | . | . | . | . | . | . | . | . | . | . | . | . | . | . |
| 21-225_56E5_LC | . | . | . | . | . | . | . | . | . | . | . | . | . | . | . | . | . | . | . |
| 21-225_56F6_LC | . | . | . | . | . | . | . | . | . | . | . | . | . | . | . | . | . | . | . |
| 21-225_57A7_LC | . | . | . | . | . | . | . | . | . | . | . | . | . | . | . | . | . | . | . |
| 21-225_57E4_LC | . | . | . | . | . | . | . | . | . | . | . | . | . | . | . | . | . | . | . |
| 21-225_64E2_LC | . | . | . | . | . | . | . | . | . | . | . | . | . | . | . | . | . | . | . |
| 21-225_64G12_LC | . | . | . | . | . | . | . | . | . | . | . | . | . | . | L | . | . | . | . |
| 21-225_65F10_LC | . | . | . | . | . | . | . | . | . | . | R | . | . | . | . | . | . | . | . |
| 21-225_66F11_LC | . | . | . | . | . | . | . | . | . | . | . | . | . | . | . | . | . | . | . |
| 21-225_70D1_LC | . | . | . | . | . | . | . | . | . | . | . | . | . | . | . | . | . | . | . |
| 21-225_7C3_LC2_LC | . | . | . | . | . | . | . | . | . | . | . | . | . | . | . | . | . | . | . |
| 21-225_9F12_LC1_LC | . | . | . | . | . | . | . | . | . | . | . | . | . | . | . | . | F | . | . |

**FIGURE 57**

Table 105. Consensus 25 - VK2|A18/JK1 (SEQ ID NO: 50322):

DIVMTQTPLSLSVTPGQPASISCKSS--QSLLHGD-GKTYLYWYLQKPGQPPQLLIYE--------VSNRFSGVPDRFSGSGSG--TDFTLKISRVEAEDVGVYYCMQSIQ----------------------LPWTFGQGTKVEIKR wherein:

K at position 24 can be substituted with M, R or T

S at position 26 can be substituted with G or T

Q at position 29 can be substituted with K

S at position 30 can be substituted with R, N or T

L at position 32 can be substituted with R or V

H at position 33 can be substituted with Y

G at position 34 can be substituted with S

D at position 35 can be substituted with E or G

K at position 38 can be substituted with R

Y at position 40 can be substituted with F

L at position 41 can be substituted with F

Y at position 42 can be substituted with F, T, C or S

E at position 58 can be substituted with A

V at position 67 can be substituted with I, L or T

N at position 69 can be substituted with K, H, I or S

F at position 71 can be substituted with L

S at position 72 can be substituted with A, T, C or P

**FIGURE 57**

M at position 107 can be substituted with K

S at position 109 can be substituted with T

I at position 110 can be substituted with T, F or L

Q at position 111 can be substituted with H

L at position 135 can be substituted with I, V or F

W at position 137 can be substituted with R

Xaa Ser Xaa Xaa Xaa Leu Xaa Xaa Xaa Xaa Gly Xaa Thr Xaa Xaa Xaa (SEQ ID NO: 50150)

Xaa Xaa Ser Xaa Arg Xaa Xaa (SEQ ID NO: 50151)

Xaa Gln Xaa Xaa Xaa Xaa Pro Xaa Thr (SEQ ID NO: 50152)

Lys Ser Ser Gln Ser Leu Leu His Gly Asp Gly Lys Thr Tyr Leu Tyr (SEQ ID NO: 50636)

Glu Val Ser Asn Arg Phe Ser (SEQ ID NO: 50637)

Met Gln Ser Ile Gln Leu Pro Trp Thr (SEQ ID NO: 50638)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | V | M | T | Q | T | P | L | S | L | S | V | T | P | G | Q | P | A | S | I | S | C | K | S | S |
| 21-225_146B3_LC | . | . | . | . | . | . | . | . | . | . | . | . | . | . | . | . | . | . | . | . | . | . | . | . | . | . |
| 21-225_147E10_LC | A | . | . | . | . | . | . | . | . | . | . | . | . | . | . | . | . | . | . | . | . | . | . | . | . | . |
| 21-225_148B2_LC | A | . | . | . | . | . | . | . | . | . | . | . | . | . | . | . | . | . | . | . | . | . | . | . | . | . |
| 21-225_148G10_LC | . | . | . | . | . | . | . | . | . | . | . | . | . | . | . | . | . | . | . | . | . | . | . | . | . | . |
| 21-225_149F1_LC | . | . | . | . | . | . | S | . | . | . | . | F | . | . | . | . | . | . | . | . | . | . | Y | . | . | . |
| 21-225_150B11_LC | . | . | . | . | . | . | . | . | . | . | . | . | . | . | . | . | . | . | . | . | . | . | . | . | . | . |
| 21-225_151A10_LC | . | . | . | . | . | . | . | . | . | . | . | . | . | . | . | . | . | . | . | . | . | . | . | . | . | . |
| 21-225_151G5_LC | . | . | . | . | . | . | . | . | . | . | . | . | . | . | . | . | . | . | . | . | . | . | . | . | . | . |
| 21-225_152C11_LC | . | . | . | . | . | . | A | . | . | . | . | . | . | . | . | . | . | . | . | . | . | . | . | . | . | . |
| 21-225_152F6_LC | . | . | . | . | . | . | . | . | . | . | . | . | . | . | . | . | . | . | . | . | . | . | . | . | . | . |
| 21-225_153D2_LC | . | . | . | . | . | . | . | . | . | . | . | . | . | . | . | . | . | . | . | . | . | . | . | . | . | . |
| 21-225_155A5_LC | . | . | . | . | . | . | . | . | . | . | . | . | . | . | . | . | . | . | . | . | . | . | . | . | . | . |
| 21-225_157G8_LC | . | . | . | . | . | . | . | . | . | . | . | . | . | . | . | . | . | . | . | . | . | . | . | . | . | . |
| 21-225_160B1_LC | . | . | . | . | . | . | . | . | . | . | . | . | . | . | . | . | . | . | . | . | . | . | . | . | . | . |
| 21-225_160G10_LC | A | . | . | . | . | . | . | . | . | . | . | . | . | . | . | . | . | . | . | . | . | . | . | . | . | . |
| 21-225_162H6_LC | . | . | . | . | . | . | . | . | . | . | . | . | . | . | . | . | . | . | . | . | . | . | . | . | . | T |
| 21-225_167E2_LC | . | . | . | . | . | . | . | . | . | . | . | . | . | . | . | . | . | . | . | . | . | . | . | . | . | . |
| 21-225_167H10_LC | . | . | . | . | . | . | . | . | . | . | . | . | . | . | . | . | . | . | . | . | . | . | . | . | . | . |
| 21-225_170A1_LC | E | . | . | . | . | . | . | . | . | F | . | . | . | . | . | . | . | . | . | . | . | . | . | . | . | . |
| 21-225_172B7_LC | . | . | . | . | . | . | . | . | . | . | . | . | . | . | . | . | . | . | . | . | . | . | . | . | . | . |
| 21-225_173A11_LC | . | . | . | . | . | . | . | . | . | . | . | . | . | . | . | . | . | . | . | . | . | . | . | . | . | . |
| 21-225_176H4_LC | . | . | . | . | . | . | . | . | . | . | . | . | . | . | . | . | . | . | . | . | . | . | . | . | . | . |
| 21-225_177B11_LC | . | . | . | . | . | . | . | . | . | . | . | . | . | . | . | . | . | . | . | . | . | . | . | . | . | . |
| 21-225_178G10_LC | N | . | . | . | . | . | . | . | . | . | . | . | . | . | . | . | . | . | . | . | . | . | . | . | . | . |
| 21-225_180C4_LC | . | . | . | . | . | . | . | . | . | . | . | . | . | . | . | . | . | . | . | . | . | . | . | . | . | . |
| 21-225_181C10_LC | . | . | . | . | . | . | . | . | . | . | . | . | . | . | . | . | . | . | . | . | . | . | . | . | . | . |
| 21-225_181C2_LC | E | . | . | . | . | . | . | . | . | . | . | . | . | . | . | . | . | . | . | . | . | . | . | . | . | . |
| 21-225_186F7_LC | . | V | . | . | A | . | . | . | . | . | . | . | . | . | . | . | . | . | . | . | . | . | . | . | . | . |
| 21-225_188G11_LC | . | . | . | . | . | . | . | . | . | . | . | . | . | . | . | . | . | . | . | . | . | . | . | N | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | V | M | T | Q | T | P | L | S | L | S | V | T | P | G | Q | P | A | S | I | S | C | K | S | S |
| 21-225_189G2_LC | . | . | . | . | . | . | . | . | . | . | . | . | . | . | . | . | . | . | . | . | . | . | . | . | . | . |
| 21-225_190E7_LC | . | . | . | L | . | . | . | . | . | . | . | . | . | . | . | . | . | . | . | . | . | . | . | . | . | . |
| 21-225_190H11_LC | . | . | . | . | . | . | . | . | . | . | . | . | . | . | . | . | . | . | . | . | . | . | . | . | . | . |
| 21-225_191B1_LC | . | . | . | . | . | . | . | . | . | . | . | . | . | . | . | . | . | . | . | . | . | . | . | R | . | . |
| 21-225_191E3_LC | . | . | . | . | . | . | . | . | . | . | . | . | . | . | . | . | . | . | . | . | . | . | . | . | . | . |
| 21-225_191E6_LC | . | . | . | . | . | . | . | . | . | . | . | . | . | . | . | . | . | . | . | . | . | . | . | . | . | . |
| 21-225_192F9_LC | . | . | . | . | . | . | . | . | . | . | . | . | . | . | . | . | . | . | . | . | . | . | . | . | . | . |
| 21-225_194F4_LC | . | . | . | . | . | . | . | . | . | . | . | . | . | . | . | . | . | . | . | . | . | . | . | . | . | . |
| 21-225_197G8_LC | E | . | . | . | . | . | . | . | . | . | . | . | . | I | . | . | . | . | . | . | . | . | . | . | . | . |
| 21-225_198A10_LC | . | . | . | L | . | . | . | . | . | . | . | . | . | . | . | . | . | . | . | . | . | . | . | . | . | . |
| 21-225_224A7_LC | . | . | . | . | . | . | . | . | . | . | . | . | . | . | . | . | . | . | . | . | . | . | . | . | . | . |
| 21-225_224C11_LC | . | F | . | . | . | . | . | . | . | . | . | . | . | . | . | . | . | . | . | . | . | . | . | . | . | . |
| 21-225_224G1_LC | . | . | . | . | . | . | . | . | . | . | . | . | . | . | . | . | . | . | . | . | . | . | . | . | . | G |
| 21-225_224H11_LC | . | . | . | . | . | . | . | . | . | . | . | . | . | . | . | . | . | . | . | . | . | . | . | . | . | . |
| 21-225_225G4_LC | . | . | . | . | . | . | . | . | . | . | . | . | . | . | . | . | . | . | . | . | . | . | . | . | . | . |
| 21-225_226A5_LC | . | . | . | . | . | . | . | . | . | . | . | . | . | . | . | . | . | . | . | . | . | . | . | . | . | . |
| 21-225_226B1_LC | . | . | . | . | . | . | . | . | . | . | . | . | . | . | . | . | . | . | . | . | . | . | . | . | . | . |
| 21-225_226E11_LC | . | . | . | . | . | . | . | . | . | . | . | . | . | . | . | . | . | . | . | . | . | . | . | . | . | . |
| 21-225_226E7_LC | . | . | . | . | . | . | . | . | . | . | . | . | . | . | . | . | . | . | . | . | . | . | . | . | . | . |
| 21-225_226F10_LC | . | . | . | . | . | . | . | . | . | . | . | . | . | . | . | . | . | . | . | . | . | . | . | . | . | . |
| 21-225_226F9_LC | . | . | . | . | . | . | . | . | . | . | . | . | . | . | . | . | . | . | . | . | . | . | . | . | . | . |
| 21-225_226G8_LC | . | . | . | . | . | . | . | . | . | . | . | . | . | . | . | . | . | . | . | . | . | . | . | . | . | . |
| 21-225_226H12_LC | . | . | . | . | . | . | . | . | . | . | . | . | . | . | . | . | . | . | . | . | . | . | . | . | . | . |
| 21-225_226H9_LC | . | . | . | . | . | . | . | . | . | . | . | . | . | I | . | . | . | . | . | . | . | . | . | . | . | . |
| 21-225_227A8_LC | . | . | . | . | . | . | . | . | . | . | . | . | . | . | . | . | . | . | . | . | . | . | . | . | . | . |
| 21-225_227C1_LC | . | . | . | . | . | . | . | . | . | . | . | . | . | . | . | . | . | . | . | . | . | . | . | . | . | . |
| 21-225_227F2_LC | . | . | . | . | . | . | . | . | . | . | . | . | . | . | . | . | . | . | . | . | . | . | . | . | . | . |
| 21-225_22A1_LC | . | . | . | . | . | . | . | . | . | . | . | . | . | . | . | . | . | . | . | . | . | . | . | . | . | . |
| 21-225_25H9_LC | . | . | . | . | . | . | . | . | . | . | . | . | . | . | . | . | . | . | . | . | . | . | . | . | . | . |

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | V | M | T | Q | T | P | L | S | L | S | V | T | P | G | Q | P | A | S | I | S | C | K | S | S |
| 21-225_26F8_LC | . | . | . | . | . | . | . | . | . | . | . | . | . | . | . | . | . | . | . | . | . | . | . | . | . | . |
| 21-225_28G3_LC | E | . | . | . | . | . | . | . | . | . | . | . | . | . | . | . | . | . | . | . | . | . | . | . | . | . |
| 21-225_43E11_LC | . | . | . | . | . | . | . | . | . | . | . | . | . | . | . | . | . | . | . | . | . | . | . | . | . | . |
| 21-225_46F2_LC | . | . | . | . | . | . | . | . | . | . | . | T | . | . | . | . | . | . | . | . | . | . | . | . | . | . |
| 21-225_47C1_LC | . | . | . | . | . | . | . | . | . | . | . | . | . | . | . | . | . | . | . | . | . | . | . | T | . | . |
| 21-225_47E7_LC | . | . | . | . | . | . | . | . | . | . | . | . | . | . | . | . | . | . | . | . | . | . | . | . | . | . |
| 21-225_64H9_LC | E | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | | K_FR2 | | |
| CONSENSUS | - | - | Q | S | L | L | H | G | D | - | G | K | T | Y | L | Y | W | Y | L | Q | K | P | G | Q | P | P |
| 21-225_146B3_LC | . | . | . | . | . | . | . | . | . | . | . | . | . | . | . | . | . | . | . | . | . | . | . | . | . | . |
| 21-225_147E10_LC | . | . | . | R | . | . | . | . | . | . | . | . | . | . | . | . | . | . | . | . | . | . | . | . | . | . |
| 21-225_148B2_LC | . | . | . | . | . | . | . | . | . | . | . | . | . | . | F | . | . | . | . | . | . | . | . | . | . | . |
| 21-225_148G10_LC | . | . | . | . | . | . | . | . | . | . | . | . | . | . | . | . | . | . | . | . | R | . | . | . | . | . |
| 21-225_149F1_LC | . | . | . | . | . | . | . | . | . | . | . | . | . | . | F | . | . | . | . | . | . | . | . | . | . | . |
| 21-225_150B11_LC | . | . | . | . | . | V | . | . | . | . | . | . | . | . | . | . | . | . | . | . | R | . | . | . | . | . |
| 21-225_151A10_LC | . | . | . | . | . | . | . | . | . | . | . | . | . | . | . | . | . | . | . | . | . | . | . | . | . | . |
| 21-225_151G5_LC | . | . | . | R | . | . | . | . | . | . | . | . | . | . | . | . | . | . | . | . | . | . | . | . | . | . |
| 21-225_152C11_LC | . | . | . | . | . | . | . | . | . | . | . | . | . | . | . | . | . | . | . | . | . | . | . | . | . | . |
| 21-225_152F6_LC | . | . | . | . | . | R | . | . | . | . | . | . | . | . | . | T | . | . | . | . | R | . | . | . | . | . |
| 21-225_153D2_LC | . | . | . | . | . | R | . | . | . | . | . | . | . | . | . | T | . | . | . | . | R | . | . | . | . | . |
| 21-225_155A5_LC | . | . | . | . | . | . | . | . | . | . | . | . | . | . | . | . | . | . | . | . | . | . | . | . | . | . |
| 21-225_157G8_LC | . | . | . | . | . | . | . | . | . | . | . | . | . | . | . | . | . | . | . | . | . | . | . | . | S | . |
| 21-225_160B1_LC | . | . | . | . | . | . | . | . | . | . | . | . | . | . | F | . | . | . | . | . | . | . | . | . | . | . |
| 21-225_160G10_LC | . | . | . | . | . | . | . | . | . | . | . | . | . | . | . | . | . | . | . | . | . | . | . | . | . | . |
| 21-225_162H6_LC | . | . | . | . | . | . | . | . | . | . | . | . | . | . | . | . | . | . | . | . | . | . | . | . | . | . |
| 21-225_167E2_LC | . | . | . | . | . | . | . | . | . | . | . | . | . | . | . | . | . | . | . | . | . | . | . | . | . | . |
| 21-225_167H10_LC | . | . | . | . | . | . | . | . | . | . | . | . | . | . | . | . | . | . | . | . | . | . | . | . | . | . |
| 21-225_170A1_LC | . | . | . | . | . | . | . | . | . | . | . | . | . | . | . | F | . | . | . | . | . | . | . | . | . | . |
| 21-225_172B7_LC | . | . | . | . | . | . | . | . | . | . | . | . | . | . | F | . | . | . | . | . | . | . | . | . | . | . |
| 21-225_173A11_LC | . | . | . | . | . | . | . | . | . | . | . | . | . | . | . | . | . | . | . | . | . | . | . | . | . | . |
| 21-225_176H4_LC | . | . | . | . | . | . | . | . | . | . | . | . | . | . | . | . | . | . | . | . | . | . | . | . | . | . |
| 21-225_177B11_LC | . | . | . | R | . | . | . | . | . | . | . | . | . | . | . | . | . | . | . | . | . | . | . | . | . | . |
| 21-225_178G10_LC | . | . | . | . | . | . | . | . | . | . | . | . | . | . | . | . | . | . | . | . | . | . | . | . | . | . |
| 21-225_180C4_LC | . | . | . | . | . | . | . | . | . | . | . | . | . | . | . | . | . | . | . | . | . | . | . | . | . | . |
| 21-225_181C10_LC | . | . | . | . | . | . | . | . | . | . | . | . | . | . | . | . | . | . | . | . | . | . | . | . | . | . |
| 21-225_181C2_LC | . | . | . | . | . | . | . | . | . | . | . | . | . | . | . | . | . | . | . | . | . | . | . | . | . | . |
| 21-225_186F7_LC | . | . | . | . | . | . | . | . | . | . | . | . | . | . | . | C | . | . | . | . | . | . | . | . | . | . |
| 21-225_188G11_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | L | L | H | G | D | - | G | K | T | Y | L | Y | W | Y | L | Q | K | P | G | Q | P | P |
| 21-225_189G2_LC | . | . | . | . | . | . | . | . | . | . | . | . | . | . | . | F | . | . | . | . | . | . | . | . | . | . |
| 21-225_190E7_LC | . | . | . | . | . | . | . | S | . | . | . | R | . | . | . | . | . | . | . | . | . | . | . | . | . | . |
| 21-225_190H11_LC | . | . | . | . | . | . | . | S | . | . | . | R | . | . | . | . | . | . | . | . | . | . | . | . | . | . |
| 21-225_191B1_LC | . | . | . | . | . | . | . | S | . | . | . | . | . | . | . | F | . | . | . | . | . | . | . | . | . | . |
| 21-225_191E3_LC | . | . | . | . | . | . | . | S | . | . | . | R | . | . | . | . | . | . | . | . | . | . | . | . | . | . |
| 21-225_191E6_LC | . | . | . | . | . | . | . | S | . | . | . | R | . | . | . | . | . | . | . | . | . | . | . | . | . | . |
| 21-225_192F9_LC | . | . | . | . | . | . | . | S | . | . | . | R | . | . | . | . | . | . | . | . | R | . | . | . | . | . |
| 21-225_194F4_LC | . | . | . | . | . | . | . | S | . | . | . | R | . | . | . | . | . | . | . | . | . | . | . | . | . | . |
| 21-225_197G8_LC | . | . | . | N | . | . | . | S | . | . | . | . | . | . | . | . | . | . | . | . | . | . | . | . | . | . |
| 21-225_198A10_LC | . | . | . | . | . | . | Y | S | . | . | . | R | . | . | . | . | . | . | . | . | . | . | . | . | . | . |
| 21-225_224A7_LC | . | . | . | . | . | . | . | S | . | . | . | . | . | F | . | . | . | F | . | . | . | . | . | . | . | . |
| 21-225_224C11_LC | . | . | . | . | . | . | . | S | . | . | . | . | . | F | . | . | . | . | . | . | . | . | . | . | . | . |
| 21-225_224G1_LC | . | . | . | . | . | . | . | S | . | . | . | . | . | F | . | S | . | . | . | . | . | . | . | . | . | . |
| 21-225_224H11_LC | . | . | . | . | . | . | . | S | . | . | . | . | . | F | . | . | . | . | . | . | . | . | . | . | . | . |
| 21-225_225G4_LC | . | . | . | . | . | . | . | S | . | . | . | . | . | F | . | . | . | . | . | . | . | . | . | . | . | . |
| 21-225_226A5_LC | . | . | . | . | . | . | . | . | . | . | . | . | . | . | . | . | . | . | . | . | . | . | . | . | . | . |
| 21-225_226B1_LC | . | . | . | . | . | . | . | . | . | . | . | . | . | . | . | . | . | . | . | . | R | . | . | . | . | . |
| 21-225_226E11_LC | . | . | . | . | . | . | . | S | . | . | . | . | . | F | . | . | . | . | . | H | . | . | . | . | . | . |
| 21-225_226E7_LC | . | . | . | . | . | . | . | . | . | . | . | . | . | . | . | . | . | . | Q | . | . | . | . | . | . | . |
| 21-225_226F10_LC | . | . | . | . | . | . | . | S | . | . | . | . | . | F | . | . | . | . | . | . | . | . | . | . | . | . |
| 21-225_226F9_LC | . | . | . | . | . | . | . | S | . | . | . | . | . | F | . | . | . | . | . | . | . | . | . | . | . | . |
| 21-225_226G8_LC | . | . | . | . | . | . | . | S | . | . | . | . | . | F | . | . | . | . | . | . | . | . | . | . | . | . |
| 21-225_226H12_LC | . | . | K | T | . | . | . | S | . | . | . | . | . | F | . | . | . | . | . | . | . | . | . | . | . | . |
| 21-225_226H9_LC | . | . | . | . | . | . | . | S | . | . | . | . | . | F | . | . | . | . | . | . | R | . | . | . | . | . |
| 21-225_227A8_LC | . | . | . | . | . | . | . | S | . | . | . | . | . | F | . | . | . | . | . | . | . | . | . | . | . | . |
| 21-225_227C1_LC | . | . | . | . | . | . | . | S | . | . | . | . | . | F | . | . | . | . | . | . | . | . | . | . | . | . |
| 21-225_227F2_LC | . | . | . | . | . | . | . | S | . | . | . | . | . | F | . | . | . | . | . | . | . | . | . | . | . | . |
| 21-225_22A1_LC | . | . | . | . | . | . | . | S | G | . | . | . | . | . | . | . | . | . | . | . | . | . | . | . | . | . |
| 21-225_25H9_LC | . | . | . | . | . | . | . | . | . | . | . | . | . | . | . | F | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | L | L | H | G | D | - | G | K | T | Y | L | Y | W | Y | L | Q | K | P | G | Q | P | P |
| 21-225_26F8_LC | . | . | . | . | . | . | . | . | . | . | . | . | . | . | . | . | . | . | . | . | . | . | . | . | . | . |
| 21-225_28G3_LC | . | . | . | . | . | . | . | . | . | . | . | . | . | . | . | . | . | . | . | . | . | . | . | . | . | . |
| 21-225_43E11_LC | . | . | . | . | . | . | . | S | . | . | . | R | . | . | . | . | . | . | . | . | . | . | . | . | . | . |
| 21-225_46F2_LC | . | . | . | . | . | . | . | . | . | . | . | . | . | . | . | . | . | . | . | . | . | . | . | . | . | . |
| 21-225_47C1_LC | . | . | . | . | . | . | . | S | E | . | . | . | . | . | . | . | . | . | . | . | . | . | . | . | . | . |
| 21-225_47E7_LC | . | . | . | . | . | . | . | S | . | . | . | R | . | . | . | . | . | . | . | . | . | . | . | . | . | . |
| 21-225_64H9_LC | . | . | . | . | . | . | . | . | . | . | . | . | . | . | . | F | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | Q | L | L | I | Y | E | - | - | - | - | - | - | - | - | V | S | N | R | F | S | G | V | P | D | R | F |
| 21-225_146B3_LC | . | . | . | . | . | . | . | . | . | . | . | . | . | . | . | . | . | . | . | . | . | . | . | . | . | . |
| 21-225_147E10_LC | H | . | . | . | S | . | . | . | . | . | . | . | . | . | . | . | . | . | . | . | . | . | . | . | . | . |
| 21-225_148B2_LC | . | . | . | . | . | . | . | . | . | . | . | . | . | . | . | . | H | . | . | . | . | . | . | . | . | . |
| 21-225_148G10_LC | . | . | . | . | . | . | . | . | . | . | . | . | . | . | . | . | . | . | . | . | . | . | . | . | . | . |
| 21-225_149F1_LC | . | . | . | . | . | . | . | . | . | . | . | . | . | . | . | . | H | . | . | . | . | . | . | . | . | . |
| 21-225_150B11_LC | . | . | . | . | . | . | . | . | . | . | . | . | . | . | . | . | . | . | . | . | . | . | . | . | . | . |
| 21-225_151A10_LC | . | . | . | . | . | . | . | . | . | . | . | . | . | . | . | . | H | . | . | . | . | . | . | . | . | . |
| 21-225_151G5_LC | . | . | . | L | . | . | . | . | . | . | . | . | . | . | . | . | . | . | . | . | . | . | . | . | . | . |
| 21-225_152C11_LC | . | I | . | . | . | . | . | . | . | . | . | . | . | . | . | . | . | . | . | . | . | . | . | . | . | . |
| 21-225_152F6_LC | . | V | . | . | H | . | . | . | . | . | . | . | . | . | I | . | K | . | . | T | . | . | . | . | . | . |
| 21-225_153D2_LC | . | V | . | . | H | . | . | . | . | . | . | . | . | . | . | . | K | . | . | T | . | . | . | . | . | . |
| 21-225_155A5_LC | . | . | . | . | . | . | . | . | . | . | . | . | . | . | . | . | . | . | . | . | . | . | . | . | . | . |
| 21-225_157G8_LC | . | . | . | . | . | . | . | . | . | . | . | . | . | . | I | . | K | . | . | . | . | . | . | . | . | . |
| 21-225_160B1_LC | . | V | . | . | . | . | . | . | . | . | . | . | . | . | . | . | K | . | . | . | . | . | S | E | . | . |
| 21-225_160G10_LC | H | . | . | . | . | . | . | . | . | . | . | . | . | . | . | . | K | . | . | . | . | . | . | . | . | . |
| 21-225_162H6_LC | . | . | . | . | . | . | . | . | . | . | . | . | . | . | . | . | K | . | . | . | . | . | . | E | . | . |
| 21-225_167E2_LC | H | . | . | . | . | . | . | . | . | . | . | . | . | . | . | . | K | . | . | . | . | . | . | . | . | . |
| 21-225_167H10_LC | H | . | . | . | . | . | . | . | . | . | . | . | . | . | . | . | K | . | . | . | . | . | . | . | . | . |
| 21-225_170A1_LC | . | V | . | . | . | . | . | . | . | . | . | . | . | . | . | . | . | . | . | . | . | . | . | . | . | . |
| 21-225_172B7_LC | . | V | . | L | F | . | . | . | . | . | . | . | . | . | . | . | H | . | . | . | . | . | . | . | . | . |
| 21-225_173A11_LC | . | . | . | . | . | . | . | . | . | . | . | . | . | . | . | . | . | . | . | . | . | . | . | . | . | . |
| 21-225_176H4_LC | . | . | . | . | . | . | . | . | . | . | . | . | . | . | . | . | . | . | . | . | . | . | . | . | . | . |
| 21-225_177B11_LC | . | I | . | . | . | . | . | . | . | . | . | . | . | . | . | . | . | . | . | . | . | . | . | . | . | . |
| 21-225_178G10_LC | . | . | . | . | . | . | . | . | . | . | . | . | . | . | . | . | . | . | . | . | . | . | . | . | . | L |
| 21-225_180C4_LC | . | . | . | . | . | A | . | . | . | . | . | . | . | . | T | . | . | . | . | P | . | . | S | . | . | . |
| 21-225_181C10_LC | . | . | . | . | . | . | . | . | . | . | . | . | . | . | . | . | . | . | . | . | . | . | . | . | . | . |
| 21-225_181C2_LC | . | . | . | . | H | . | . | . | . | . | . | . | . | . | . | . | . | . | . | . | . | . | . | . | . | . |
| 21-225_186F7_LC | . | . | . | . | . | . | . | . | . | . | . | . | . | . | . | . | K | . | . | . | . | . | . | . | . | . |
| 21-225_188G11_LC | . | . | . | . | . | . | . | . | . | . | . | . | . | . | . | . | . | . | . | . | . | . | . | . | T | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | Q | L | L | I | Y | E | - | - | - | - | - | - | - | - | V | S | N | R | F | S | G | V | P | D | R | F |
| 21-225_189G2_LC | . | . | . | . | . | . | . | . | . | . | . | . | . | . | . | . | . | . | . | . | . | . | S | . | . | . |
| 21-225_190E7_LC | . | V | . | . | C | . | . | . | . | . | . | . | . | . | . | . | . | . | . | . | . | . | . | . | . | . |
| 21-225_190H11_LC | . | . | . | . | C | . | . | . | . | . | . | . | . | . | . | . | . | . | . | A | . | . | T | . | . | . |
| 21-225_191B1_LC | . | V | . | . | . | . | . | . | . | . | . | . | . | . | L | . | . | . | . | . | . | . | . | . | . | . |
| 21-225_191E3_LC | . | . | . | . | C | . | . | . | . | . | . | . | . | . | . | . | . | . | . | A | . | . | T | . | . | . |
| 21-225_191E6_LC | . | . | . | . | C | . | . | . | . | . | . | . | . | . | . | . | . | . | . | A | . | . | T | . | . | . |
| 21-225_192F9_LC | . | . | . | . | C | . | . | . | . | . | . | . | . | . | . | . | . | . | . | A | . | . | T | . | . | . |
| 21-225_194F4_LC | . | . | . | . | C | . | . | . | . | . | . | . | . | . | . | . | . | . | . | . | . | . | . | . | . | . |
| 21-225_197G8_LC | . | V | . | . | . | . | . | . | . | . | . | . | . | . | . | . | . | . | . | . | . | . | . | . | . | . |
| 21-225_198A10_LC | . | V | . | . | C | . | . | . | . | . | . | . | . | . | . | . | . | . | . | . | . | . | . | . | . | . |
| 21-225_224A7_LC | . | . | . | . | . | . | . | . | . | . | . | . | . | . | I | . | . | . | . | . | . | . | . | . | . | . |
| 21-225_224C11_LC | . | . | . | . | . | . | . | . | . | . | . | . | . | . | I | . | . | . | . | . | . | . | . | . | . | . |
| 21-225_224G1_LC | . | . | . | . | . | . | . | . | . | . | . | . | . | . | I | . | . | . | . | . | . | . | . | . | . | . |
| 21-225_224H11_LC | . | . | . | . | . | . | . | . | . | . | . | . | . | . | I | . | . | . | . | . | . | . | . | . | . | . |
| 21-225_225G4_LC | . | . | . | . | . | . | . | . | . | . | . | . | . | . | I | . | . | . | . | . | . | . | . | . | . | . |
| 21-225_226A5_LC | . | . | . | . | . | . | . | . | . | . | . | . | . | . | . | . | . | . | . | . | . | . | . | . | . | . |
| 21-225_226B1_LC | . | . | . | . | N | . | . | . | . | . | . | . | . | . | . | . | I | . | . | . | . | . | . | . | . | . |
| 21-225_226E11_LC | H | . | . | . | . | . | . | . | . | . | . | . | . | . | I | . | . | . | . | . | . | . | . | . | . | . |
| 21-225_226E7_LC | . | I | . | . | . | . | . | . | . | . | . | . | . | . | . | . | . | . | . | . | . | . | . | . | . | . |
| 21-225_226F10_LC | . | . | . | . | . | . | . | . | . | . | . | . | . | . | I | . | . | . | . | . | . | . | . | . | . | . |
| 21-225_226F9_LC | . | . | . | . | . | . | . | . | . | . | . | . | . | . | I | . | . | . | . | . | . | . | . | . | . | . |
| 21-225_226G8_LC | . | . | . | . | . | . | . | . | . | . | . | . | . | . | I | . | . | . | . | . | . | . | . | . | . | . |
| 21-225_226H12_LC | . | P | . | . | . | . | . | . | . | . | . | . | . | . | I | . | . | . | . | . | . | . | . | . | . | . |
| 21-225_226H9_LC | . | . | . | . | . | . | . | . | . | . | . | . | . | . | . | . | . | . | . | . | . | . | . | . | . | . |
| 21-225_227A8_LC | . | . | . | . | . | . | . | . | . | . | . | . | . | . | I | . | . | . | . | . | . | . | . | . | . | . |
| 21-225_227C1_LC | . | . | . | . | . | . | . | . | . | . | . | . | . | . | I | . | . | . | . | . | . | . | . | . | . | . |
| 21-225_227F2_LC | . | . | . | . | . | . | . | . | . | . | . | . | . | . | I | . | . | . | . | . | . | . | . | . | . | . |
| 21-225_22A1_LC | . | . | . | . | . | . | . | . | . | . | . | . | . | . | . | . | . | . | . | . | . | L | . | . | . | . |
| 21-225_25H9_LC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | Q | L | L | I | Y | E | - | - | - | - | - | - | - | - | V | S | N | R | F | S | G | V | P | D | R | F |
| 21-225_26F8_LC | . | . | . | . | . | . | . | . | . | . | . | . | . | . | . | . | . | . | . | . | . | . | . | . | . | . |
| 21-225_28G3_LC | . | . | . | . | . | . | . | . | . | . | . | . | . | . | . | . | . | . | . | . | . | . | . | E | . | . |
| 21-225_43E11_LC | . | . | . | . | . | . | . | . | . | . | . | . | . | . | . | . | . | . | . | . | . | . | . | . | . | . |
| 21-225_46F2_LC | . | V | . | . | . | . | . | . | . | . | . | . | . | . | . | . | . | . | . | . | . | . | . | . | . | . |
| 21-225_47C1_LC | . | . | . | . | . | . | . | . | . | . | . | . | . | . | . | . | . | . | . | . | . | . | . | . | . | . |
| 21-225_47E7_LC | . | . | . | . | . | . | . | . | . | . | . | . | . | . | . | . | S | . | . | . | . | . | . | . | . | . |
| 21-225_64H9_LC | . | V | . | . | . | . | . | . | . | . | . | . | . | . | . | . | . | . | L | C | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | K | I | S | R | V | E | A | E | D | V | G | V | Y |
| 21-225_146B3_LC | . | . | . | . | . | . | . | . | . | . | . | . | . | . | . | . | . | . | . | . | . | . | . | . | L | . |
| 21-225_147E10_LC | . | . | . | . | . | . | . | . | . | . | . | . | . | . | . | . | . | . | . | . | . | . | . | . | . | . |
| 21-225_148B2_LC | . | . | . | . | . | . | . | . | . | . | . | . | . | . | . | . | . | . | . | . | . | . | . | . | . | . |
| 21-225_148G10_LC | . | . | . | . | . | . | . | . | . | . | . | . | . | . | . | . | . | . | . | . | . | . | . | . | . | . |
| 21-225_149F1_LC | . | . | . | . | . | . | . | . | . | . | . | . | . | . | . | . | . | . | . | . | . | . | . | . | . | . |
| 21-225_150B11_LC | . | . | . | . | . | . | . | . | . | . | . | . | . | . | . | . | . | . | . | . | . | . | . | . | . | . |
| 21-225_151A10_LC | . | . | . | . | . | . | . | . | . | . | . | . | . | . | . | . | . | . | . | . | . | . | . | . | . | . |
| 21-225_151G5_LC | . | . | . | . | . | . | . | . | . | . | . | . | . | . | . | . | . | . | . | . | . | . | . | . | . | . |
| 21-225_152C11_LC | . | . | . | . | . | . | . | . | . | . | . | . | . | N | . | . | . | . | . | . | . | . | F | . | F | . |
| 21-225_152F6_LC | . | . | . | . | . | . | . | . | . | . | . | . | . | N | . | . | . | . | . | . | . | . | . | . | F | . |
| 21-225_153D2_LC | . | . | . | . | . | . | . | . | . | . | . | . | . | N | . | . | . | . | . | . | . | . | . | . | F | . |
| 21-225_155A5_LC | . | . | . | . | . | . | . | . | . | . | . | . | . | . | . | . | . | . | . | . | . | . | . | . | F | . |
| 21-225_157G8_LC | . | . | . | . | . | . | . | . | . | . | . | . | . | . | . | . | . | . | . | . | . | . | . | . | . | . |
| 21-225_160B1_LC | . | . | . | . | . | . | . | . | . | . | . | . | . | . | . | . | . | . | . | . | . | . | . | . | . | . |
| 21-225_160G10_LC | . | . | . | . | . | . | . | . | . | . | . | . | . | . | . | . | . | . | . | . | . | . | . | . | L | . |
| 21-225_162H6_LC | . | . | . | . | . | . | . | . | . | . | . | . | . | . | . | . | . | . | . | . | . | . | . | . | . | . |
| 21-225_167E2_LC | . | . | . | . | . | . | . | . | . | . | . | . | . | . | . | . | . | . | . | . | . | . | . | . | L | . |
| 21-225_167H10_LC | . | . | . | . | . | . | . | . | . | . | . | . | . | . | . | . | . | . | . | . | . | . | . | . | L | . |
| 21-225_170A1_LC | . | . | . | . | . | . | . | . | . | . | . | . | . | . | . | . | . | . | . | . | . | . | . | . | . | . |
| 21-225_172B7_LC | . | . | G | . | . | . | . | . | . | . | . | . | . | . | . | . | . | . | . | . | . | . | . | . | . | . |
| 21-225_173A11_LC | . | . | . | . | . | . | . | . | . | . | . | . | . | . | . | . | . | . | . | . | . | . | . | . | . | F |
| 21-225_176H4_LC | . | . | . | . | . | . | . | . | . | . | . | . | . | . | . | . | . | . | . | . | . | . | . | . | I | . |
| 21-225_177B11_LC | . | . | . | . | . | . | . | . | . | E | . | . | . | . | . | . | . | . | . | . | . | . | . | . | . | . |
| 21-225_178G10_LC | . | . | G | . | . | . | . | . | . | . | . | . | . | . | . | . | . | . | . | . | . | . | . | . | I | . |
| 21-225_180C4_LC | . | . | . | . | . | . | . | . | . | . | . | . | . | . | . | . | . | . | . | . | . | . | . | . | . | . |
| 21-225_181C10_LC | . | . | . | . | . | . | . | . | . | . | . | . | . | . | . | . | . | . | . | . | . | . | . | . | . | . |
| 21-225_181C2_LC | . | . | . | . | . | . | . | . | . | . | . | . | . | . | . | . | . | . | . | . | . | . | . | . | . | . |
| 21-225_186F7_LC | . | . | . | . | . | . | . | . | . | . | . | . | . | . | . | . | . | . | . | . | . | . | . | . | . | . |
| 21-225_188G11_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | K | I | S | R | V | E | A | E | D | V | G | V | Y |
| 21-225_189G2_LC | . | . | . | . | . | . | . | . | . | . | . | . | . | . | . | . | . | . | . | . | . | . | . | . | . | . |
| 21-225_190E7_LC | . | . | . | . | . | . | . | . | . | . | . | . | . | . | . | . | . | . | . | . | G | . | . | . | . | . |
| 21-225_190H11_LC | . | . | . | . | . | . | . | . | . | . | . | . | . | . | . | . | . | . | . | . | G | . | . | . | . | . |
| 21-225_191B1_LC | . | . | . | . | . | . | . | . | . | . | . | . | . | . | . | . | . | . | . | . | . | . | . | . | . | . |
| 21-225_191E3_LC | . | . | . | . | . | . | . | . | . | . | . | . | . | . | . | . | . | . | . | . | G | . | . | . | . | . |
| 21-225_191E6_LC | . | . | . | . | . | . | . | . | . | . | . | . | . | . | . | . | . | . | . | . | G | . | . | . | I | . |
| 21-225_192F9_LC | . | . | . | . | . | . | . | . | . | . | . | . | . | . | . | . | . | . | . | . | G | . | . | . | . | . |
| 21-225_194F4_LC | . | . | . | . | . | . | . | . | . | . | . | . | . | . | . | . | . | . | . | . | G | . | . | . | . | . |
| 21-225_197G8_LC | . | . | . | . | . | . | . | . | . | . | . | . | . | . | . | . | . | . | . | . | . | . | . | . | . | . |
| 21-225_198A10_LC | . | . | . | . | . | . | . | . | . | . | . | . | . | . | . | . | . | . | . | . | G | . | . | . | . | . |
| 21-225_224A7_LC | . | . | . | . | . | . | . | . | . | . | . | . | . | . | . | . | . | . | . | . | . | . | . | . | I | . |
| 21-225_224C11_LC | . | . | . | . | . | . | . | . | . | . | . | . | . | . | . | . | . | . | . | . | . | . | . | . | I | . |
| 21-225_224G1_LC | . | . | . | . | . | . | . | . | . | . | . | . | . | . | . | . | . | . | . | . | . | . | . | . | I | F |
| 21-225_224H11_LC | . | . | . | . | . | . | . | . | . | . | . | . | . | . | . | . | . | . | . | . | . | . | . | . | I | . |
| 21-225_225G4_LC | . | . | . | . | . | . | . | . | . | . | . | . | . | . | . | . | . | . | . | . | . | . | . | . | I | . |
| 21-225_226A5_LC | . | . | . | . | . | . | . | . | . | . | . | . | . | . | . | . | . | . | . | . | . | . | . | . | . | . |
| 21-225_226B1_LC | . | . | . | . | . | . | . | . | . | . | . | . | . | . | . | . | . | . | . | . | . | . | . | . | . | . |
| 21-225_226E11_LC | . | . | . | . | . | . | . | . | . | . | . | . | . | . | . | . | . | . | . | . | . | . | . | . | I | . |
| 21-225_226E7_LC | . | . | . | . | . | . | . | . | . | . | . | . | . | . | . | . | . | . | . | . | . | . | . | . | . | . |
| 21-225_226F10_LC | . | . | . | . | . | . | . | . | . | . | . | . | . | . | . | . | . | . | . | . | . | . | . | . | I | . |
| 21-225_226F9_LC | . | . | . | . | . | . | . | . | . | . | . | . | . | . | . | . | . | . | . | . | . | . | . | . | I | . |
| 21-225_226G8_LC | . | . | . | . | . | . | . | . | . | . | . | . | . | . | . | . | . | . | . | . | . | . | . | . | I | . |
| 21-225_226H12_LC | . | . | . | . | . | . | . | . | . | . | . | . | . | . | . | . | . | . | . | . | . | . | . | . | I | . |
| 21-225_226H9_LC | . | . | . | . | . | . | . | . | . | . | . | . | . | . | . | . | . | . | . | . | . | . | . | . | I | . |
| 21-225_227A8_LC | . | . | . | . | . | . | . | . | . | . | . | . | . | . | . | . | . | . | . | . | . | . | . | . | I | . |
| 21-225_227C1_LC | . | . | . | . | . | . | . | . | . | . | . | . | . | . | . | . | . | . | . | . | . | . | . | . | I | . |
| 21-225_227F2_LC | . | . | . | . | . | . | . | . | . | . | . | . | . | . | . | . | . | . | . | . | . | . | . | . | I | . |
| 21-225_22A1_LC | . | . | . | . | . | . | . | . | . | . | . | . | . | . | . | . | . | . | . | . | A | . | . | . | . | . |
| 21-225_25H9_LC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | K | I | S | R | V | E | A | E | D | V | G | V | Y |
| 21-225_26F8_LC | . | . | . | . | . | . | . | . | . | . | . | . | . | . | . | . | . | . | . | . | . | . | . | . | . | . |
| 21-225_28G3_LC | . | . | . | . | . | . | . | . | . | . | . | . | . | . | . | . | . | . | . | . | . | . | . | . | . | . |
| 21-225_43E11_LC | . | . | . | . | . | . | . | . | . | . | . | . | . | . | . | . | . | . | . | . | . | . | . | . | . | . |
| 21-225_46F2_LC | . | . | . | . | . | . | . | . | . | . | . | . | . | . | L | . | . | . | . | . | . | . | . | . | . | . |
| 21-225_47C1_LC | . | . | . | . | . | . | . | . | . | . | . | . | . | . | . | . | . | . | . | . | . | . | . | . | . | F |
| 21-225_47E7_LC | . | . | . | . | . | . | . | . | . | . | . | A | . | . | . | . | . | . | . | . | . | . | . | . | . | . |
| 21-225_64H9_LC | . | . | . | . | . | . | . | . | . | . | . | S | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | M | Q | S | I | Q | - | - | - | - | - | - | - | - | - | - | - | - | - | - | - | - | - | - | - |
| 21-225_146B3_LC | H | . | . | . | . | . | . | . | . | . | . | . | . | . | . | . | . | . | . | . | . | . | . | . | . | . |
| 21-225_147E10_LC | . | . | . | . | . | . | . | . | . | . | . | . | . | . | . | . | . | . | . | . | . | . | . | . | . | . |
| 21-225_148B2_LC | . | . | . | . | . | . | . | . | . | . | . | . | . | . | . | . | . | . | . | . | . | . | . | . | . | . |
| 21-225_148G10_LC | . | . | . | . | . | . | . | . | . | . | . | . | . | . | . | . | . | . | . | . | . | . | . | . | . | . |
| 21-225_149F1_LC | . | . | . | . | . | . | . | . | . | . | . | . | . | . | . | . | . | . | . | . | . | . | . | . | . | . |
| 21-225_150B11_LC | . | . | . | . | . | . | . | . | . | . | . | . | . | . | . | . | . | . | . | . | . | . | . | . | . | . |
| 21-225_151A10_LC | . | . | . | . | . | . | . | . | . | . | . | . | . | . | . | . | . | . | . | . | . | . | . | . | . | . |
| 21-225_151G5_LC | . | . | . | . | . | . | . | . | . | . | . | . | . | . | . | . | . | . | . | . | . | . | . | . | . | . |
| 21-225_152C11_LC | . | . | . | . | . | . | . | . | . | . | . | . | . | . | . | . | . | . | . | . | . | . | . | . | . | . |
| 21-225_152F6_LC | . | . | . | . | . | . | . | . | . | . | . | . | . | . | . | . | . | . | . | . | . | . | . | . | . | . |
| 21-225_153D2_LC | . | . | . | . | . | . | . | . | . | . | . | . | . | . | . | . | . | . | . | . | . | . | . | . | . | . |
| 21-225_155A5_LC | . | . | . | . | . | . | . | . | . | . | . | . | . | . | . | . | . | . | . | . | . | . | . | . | . | . |
| 21-225_157G8_LC | . | . | . | . | . | . | . | . | . | . | . | . | . | . | . | . | . | . | . | . | . | . | . | . | . | . |
| 21-225_160B1_LC | . | . | . | . | . | . | . | . | . | . | . | . | . | . | . | . | . | . | . | . | . | . | . | . | . | . |
| 21-225_160G10_LC | . | . | . | . | . | . | . | . | . | . | . | . | . | . | . | . | . | . | . | . | . | . | . | . | . | . |
| 21-225_162H6_LC | . | . | K | . | . | . | . | . | . | . | . | . | . | . | . | . | . | . | . | . | . | . | . | . | . | . |
| 21-225_167E2_LC | H | . | . | . | . | . | . | . | . | . | . | . | . | . | . | . | . | . | . | . | . | . | . | . | . | . |
| 21-225_167H10_LC | H | . | . | . | . | . | . | . | . | . | . | . | . | . | . | . | . | . | . | . | . | . | . | . | . | . |
| 21-225_170A1_LC | . | . | . | . | . | . | . | . | . | . | . | . | . | . | . | . | . | . | . | . | . | . | . | . | . | . |
| 21-225_172B7_LC | . | . | . | . | . | . | . | . | . | . | . | . | . | . | . | . | . | . | . | . | . | . | . | . | . | . |
| 21-225_173A11_LC | F | . | . | . | . | . | . | . | . | . | . | . | . | . | . | . | . | . | . | . | . | . | . | . | . | . |
| 21-225_176H4_LC | . | . | . | . | . | . | . | . | . | . | . | . | . | . | . | . | . | . | . | . | . | . | . | . | . | . |
| 21-225_177B11_LC | . | . | . | . | . | . | . | . | . | . | . | . | . | . | . | . | . | . | . | . | . | . | . | . | . | . |
| 21-225_178G10_LC | . | . | . | . | . | . | . | . | . | . | . | . | . | . | . | . | . | . | . | . | . | . | . | . | . | . |
| 21-225_180C4_LC | . | . | . | . | . | . | . | . | . | . | . | . | . | . | . | . | . | . | . | . | . | . | . | . | . | . |
| 21-225_181C10_LC | H | . | . | . | . | . | . | . | . | . | . | . | . | . | . | . | . | . | . | . | . | . | . | . | . | . |
| 21-225_181C2_LC | . | . | . | . | . | . | . | . | . | . | . | . | . | . | . | . | . | . | . | . | . | . | . | . | . | . |
| 21-225_186F7_LC | . | . | . | . | . | . | . | . | . | . | . | . | . | . | . | . | . | . | . | . | . | . | . | . | . | . |
| 21-225_188G11_LC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | M | Q | S | I | Q | - | - | - | - | - | - | - | - | - | - | - | - | - | - | - | - | - | - | - |
| 21-225_189G2_LC | . | . | . | . | . | . | . | . | . | . | . | . | . | . | . | . | . | . | . | . | . | . | . | . | . | . |
| 21-225_190E7_LC | . | . | . | . | . | . | . | . | . | . | . | . | . | . | . | . | . | . | . | . | . | . | . | . | . | . |
| 21-225_190H11_LC | . | . | . | . | . | . | . | . | . | . | . | . | . | . | . | . | . | . | . | . | . | . | . | . | . | . |
| 21-225_191B1_LC | . | . | . | . | . | F | . | . | . | . | . | . | . | . | . | . | . | . | . | . | . | . | . | . | . | . |
| 21-225_191E3_LC | . | . | . | . | . | . | . | . | . | . | . | . | . | . | . | . | . | . | . | . | . | . | . | . | . | . |
| 21-225_191E6_LC | . | . | . | . | . | . | H | . | . | . | . | . | . | . | . | . | . | . | . | . | . | . | . | . | . | . |
| 21-225_192F9_LC | . | . | . | . | . | . | . | . | . | . | . | . | . | . | . | . | . | . | . | . | . | . | . | . | . | . |
| 21-225_194F4_LC | . | . | . | . | . | . | . | . | . | . | . | . | . | . | . | . | . | . | . | . | . | . | . | . | . | . |
| 21-225_197G8_LC | S | . | . | . | . | . | . | . | . | . | . | . | . | . | . | . | . | . | . | . | . | . | . | . | . | . |
| 21-225_198A10_LC | . | . | . | . | . | . | . | . | . | . | . | . | . | . | . | . | . | . | . | . | . | . | . | . | . | . |
| 21-225_224A7_LC | . | . | . | . | . | T | . | . | . | . | . | . | . | . | . | . | . | . | . | . | . | . | . | . | . | . |
| 21-225_224C11_LC | . | . | . | . | . | T | . | . | . | . | . | . | . | . | . | . | . | . | . | . | . | . | . | . | . | . |
| 21-225_224G1_LC | . | . | . | . | . | T | . | . | . | . | . | . | . | . | . | . | . | . | . | . | . | . | . | . | . | . |
| 21-225_224H11_LC | . | . | . | . | . | T | . | . | . | . | . | . | . | . | . | . | . | . | . | . | . | . | . | . | . | . |
| 21-225_225G4_LC | . | . | . | . | . | T | . | . | . | . | . | . | . | . | . | . | . | . | . | . | . | . | . | . | . | . |
| 21-225_226A5_LC | . | . | . | . | . | . | . | . | . | . | . | . | . | . | . | . | . | . | . | . | . | . | . | . | . | . |
| 21-225_226B1_LC | . | . | . | . | . | . | . | . | . | . | . | . | . | . | . | . | . | . | . | . | . | . | . | . | . | . |
| 21-225_226E11_LC | . | . | . | . | . | T | . | . | . | . | . | . | . | . | . | . | . | . | . | . | . | . | . | . | . | . |
| 21-225_226E7_LC | . | . | . | . | . | . | . | . | . | . | . | . | . | . | . | . | . | . | . | . | . | . | . | . | . | . |
| 21-225_226F10_LC | . | . | . | . | . | T | . | . | . | . | . | . | . | . | . | . | . | . | . | . | . | . | . | . | . | . |
| 21-225_226F9_LC | . | . | . | . | . | T | . | . | . | . | . | . | . | . | . | . | . | . | . | . | . | . | . | . | . | . |
| 21-225_226G8_LC | . | . | . | . | . | T | . | . | . | . | . | . | . | . | . | . | . | . | . | . | . | . | . | . | . | . |
| 21-225_226H12_LC | . | . | . | . | . | T | . | . | . | . | . | . | . | . | . | . | . | . | . | . | . | . | . | . | . | . |
| 21-225_226H9_LC | . | . | . | . | . | T | . | . | . | . | . | . | . | . | . | . | . | . | . | . | . | . | . | . | . | . |
| 21-225_227A8_LC | . | . | . | . | . | T | . | . | . | . | . | . | . | . | . | . | . | . | . | . | . | . | . | . | . | . |
| 21-225_227C1_LC | . | . | . | . | . | T | . | . | . | . | . | . | . | . | . | . | . | . | . | . | . | . | . | . | . | . |
| 21-225_227F2_LC | . | . | . | . | . | T | . | . | . | . | . | . | . | . | . | . | . | . | . | . | . | . | . | . | . | . |
| 21-225_22A1_LC | . | . | . | . | T | L | H | . | . | . | . | . | . | . | . | . | . | . | . | . | . | . | . | . | . | . |
| 21-225_25H9_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | M | Q | S | I | Q | - | - | - | - | - | - | - | - | - | - | - | - | - | - | - | - | - | - | - |
| 21-225_26F8_LC | . | . | . | . | . | . | . | . | . | . | . | . | . | . | . | . | . | . | . | . | . | . | . | . | . | . |
| 21-225_28G3_LC | . | . | . | . | . | . | . | . | . | . | . | . | . | . | . | . | . | . | . | . | . | . | . | . | . | . |
| 21-225_43E11_LC | . | . | . | . | . | . | . | . | . | . | . | . | . | . | . | . | . | . | . | . | . | . | . | . | . | . |
| 21-225_46F2_LC | . | . | . | . | . | . | . | . | . | . | . | . | . | . | . | . | . | . | . | . | . | . | . | . | . | . |
| 21-225_47C1_LC | . | . | . | . | . | . | . | . | . | . | . | . | . | . | . | . | . | . | . | . | . | . | . | . | . | . |
| 21-225_47E7_LC | . | . | . | . | . | T | . | . | . | . | . | . | . | . | . | . | . | . | . | . | . | . | . | . | . | . |
| 21-225_64H9_LC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | L | P | W | T | F | G | Q | G | T | K | V | E | I | K | R |
| 21-225_146B3_LC | . | . | . | . | . | . | . | . | . | . | . | . | . | . | . | . | . | . | . |
| 21-225_147E10_LC | . | . | . | . | V | . | . | . | . | . | . | . | . | . | . | . | . | . | . |
| 21-225_148B2_LC | . | . | . | . | I | . | . | . | . | . | . | . | . | . | . | . | . | . | . |
| 21-225_148G10_LC | . | . | . | . | . | . | . | . | . | . | . | . | . | . | . | . | . | . | . |
| 21-225_149F1_LC | . | . | . | . | I | . | . | . | . | . | . | . | . | . | . | . | . | . | . |
| 21-225_150B11_LC | . | . | . | . | . | . | . | . | . | . | . | . | . | . | . | . | . | . | . |
| 21-225_151A10_LC | . | . | . | . | I | . | . | . | . | . | . | . | . | . | . | . | . | . | . |
| 21-225_151G5_LC | . | . | . | . | V | . | . | . | . | . | . | . | . | . | . | . | . | . | . |
| 21-225_152C11_LC | . | . | . | . | I | . | . | . | . | . | . | . | . | . | . | D | . | . | . |
| 21-225_152F6_LC | . | . | . | . | V | . | . | . | . | . | . | . | . | . | . | . | . | . | . |
| 21-225_153D2_LC | . | . | . | . | V | . | . | . | . | . | . | . | . | . | . | . | . | . | . |
| 21-225_155A5_LC | . | . | . | . | V | . | . | . | . | . | . | . | . | . | . | . | . | . | . |
| 21-225_157G8_LC | . | . | . | . | V | . | . | . | . | . | . | . | . | . | . | . | . | . | . |
| 21-225_160B1_LC | . | . | . | . | I | . | . | . | . | . | . | . | . | . | . | . | . | . | . |
| 21-225_160G10_LC | . | . | . | . | . | . | . | . | . | V | . | . | . | . | . | . | . | T | . |
| 21-225_162H6_LC | . | . | . | . | . | . | . | . | . | . | . | . | . | . | . | . | . | . | . |
| 21-225_167E2_LC | . | . | . | . | . | . | . | . | . | . | . | . | . | . | . | . | . | . | . |
| 21-225_167H10_LC | . | . | . | . | . | . | . | . | . | . | . | . | . | . | . | . | . | . | . |
| 21-225_170A1_LC | . | . | . | . | . | . | . | . | . | . | . | . | . | . | . | . | . | . | . |
| 21-225_172B7_LC | . | . | . | . | F | . | . | . | . | . | . | . | . | R | . | D | . | . | . |
| 21-225_173A11_LC | . | . | . | . | V | . | . | . | . | . | . | . | . | . | . | . | . | . | . |
| 21-225_176H4_LC | . | . | . | . | I | . | . | . | . | . | . | . | . | R | . | . | . | . | . |
| 21-225_177B11_LC | . | . | . | . | I | . | . | . | . | . | . | . | . | . | . | . | . | . | . |
| 21-225_178G10_LC | . | . | . | . | V | . | . | . | . | . | . | . | . | . | . | . | . | . | . |
| 21-225_180C4_LC | . | . | . | . | V | . | . | . | . | . | . | . | . | . | . | . | . | . | . |
| 21-225_181C10_LC | . | . | . | . | I | . | . | . | . | . | . | . | . | . | . | . | . | . | . |
| 21-225_181C2_LC | . | . | . | . | V | . | . | . | . | . | . | . | . | . | . | . | . | . | . |
| 21-225_186F7_LC | . | . | . | . | V | . | . | . | . | . | . | . | . | . | . | . | . | . | . |
| 21-225_188G11_LC | . | . | . | . | I | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | L | P | W | T | F | G | Q | G | T | K | V | E | I | K | R |
| 21-225_189G2_LC | . | . | . | . | I | . | . | . | . | . | . | . | . | . | . | . | . | . | . |
| 21-225_190E7_LC | . | . | . | . | . | . | . | . | . | . | . | . | . | . | . | . | . | . | . |
| 21-225_190H11_LC | . | . | . | . | F | . | . | . | . | . | . | . | . | . | . | . | . | . | . |
| 21-225_191B1_LC | . | . | . | . | . | . | . | . | . | . | . | . | . | . | . | . | . | N | . |
| 21-225_191E3_LC | . | . | . | . | . | . | . | . | . | . | . | . | . | . | . | . | . | . | . |
| 21-225_191E6_LC | . | . | . | . | F | . | . | . | . | . | . | . | . | . | . | . | . | . | . |
| 21-225_192F9_LC | . | . | . | . | . | . | . | . | . | . | . | . | . | . | . | . | . | . | . |
| 21-225_194F4_LC | . | . | . | . | . | . | . | . | . | . | . | . | . | . | . | . | . | . | . |
| 21-225_197G8_LC | . | . | . | . | . | . | . | . | . | A | . | . | S | . | . | . | . | . | . |
| 21-225_198A10_LC | . | . | . | . | . | . | . | . | . | . | . | . | . | . | . | . | . | . | . |
| 21-225_224A7_LC | . | . | . | . | . | . | R | . | . | . | . | . | . | . | . | . | . | . | . |
| 21-225_224C11_LC | . | . | . | . | . | . | R | . | . | . | . | . | . | . | . | . | . | . | . |
| 21-225_224G1_LC | . | . | . | . | . | . | R | . | . | . | . | . | . | . | . | . | . | . | . |
| 21-225_224H11_LC | . | . | . | . | . | . | R | . | . | . | . | . | . | . | . | . | . | . | . |
| 21-225_225G4_LC | . | . | . | . | . | . | R | . | . | . | . | . | . | . | . | . | . | . | . |
| 21-225_226A5_LC | . | . | . | . | I | . | . | . | . | . | . | . | . | . | . | . | . | . | . |
| 21-225_226B1_LC | . | . | . | . | I | . | . | . | . | . | . | . | . | . | . | D | . | . | . |
| 21-225_226E11_LC | . | . | . | . | . | . | R | . | . | . | . | . | . | . | . | . | . | . | . |
| 21-225_226E7_LC | . | . | . | . | V | . | . | . | . | . | . | . | . | . | . | . | . | . | . |
| 21-225_226F10_LC | . | . | . | . | . | . | R | . | . | . | . | . | . | . | . | . | . | . | . |
| 21-225_226F9_LC | . | . | . | . | . | . | R | . | . | . | . | . | . | . | . | . | . | . | . |
| 21-225_226G8_LC | . | . | . | . | . | . | R | . | . | . | . | . | . | . | . | . | . | . | . |
| 21-225_226H12_LC | . | . | . | . | . | . | R | . | . | . | . | . | . | . | . | . | . | . | . |
| 21-225_226H9_LC | . | . | . | . | . | . | R | . | . | . | . | . | . | . | . | . | . | . | . |
| 21-225_227A8_LC | . | . | . | . | . | . | R | . | . | . | . | . | . | R | . | . | . | . | . |
| 21-225_227C1_LC | . | . | . | . | . | . | R | . | . | . | . | . | . | . | . | . | . | . | . |
| 21-225_227F2_LC | . | . | . | . | . | . | R | . | . | . | . | . | . | . | . | . | . | . | . |
| 21-225_22A1_LC | . | . | . | . | . | . | . | . | . | . | . | . | . | . | . | . | . | . | . |
| 21-225_25H9_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | L | P | W | T | F | G | Q | G | T | K | V | E | I | K | R |
| 21-225_26F8_LC | . | . | . | . | . | . | . | . | . | . | . | . | . | . | . | . | . | . | . |
| 21-225_28G3_LC | . | . | . | . | I | . | . | . | . | . | . | . | . | . | . | . | . | . | . |
| 21-225_43E11_LC | . | . | . | . | . | . | . | . | . | . | . | . | . | . | . | . | . | . | . |
| 21-225_46F2_LC | . | . | . | . | . | . | . | . | . | . | . | . | . | . | . | . | . | . | . |
| 21-225_47C1_LC | . | . | . | . | . | . | . | . | . | . | . | . | . | . | . | . | . | . | . |
| 21-225_47E7_LC | . | . | . | . | . | . | . | . | . | . | . | . | . | . | A | . | . | . | . |
| 21-225_64H9_LC | . | . | . | . | V | . | . | . | . | . | . | . | . | . | . | . | . | T | . |

**FIGURE 57**

Table 106. Consensus 26 - VK1|O12/JK3 (SEQ ID NO: 50323):

DIQMTQSPSSLSASVGDRVTITCRAS--QSIS------SYLNWYQQKPGKAPKLLIYA--------ASSLQSGVPSRFSGSGSG--TDFTLTISSLQPEDFATYYCQQSYS----------------------PPFTFGPGTKVDIKR wherein:

A at position 25 can be substituted with S or T

S at position 26 can be substituted with G or I

Q at position 29 can be substituted with H or R

S at position 30 can be substituted with N or T

I at position 31 can be substituted with F

S at position 32 can be substituted with I, F, N, R, Y, T, A, G or L

S at position 39 can be substituted with N, T, H or K

Y at position 40 can be substituted with F or H

L at position 41 can be substituted with V

N at position 42 can be substituted with I, M, H or Y

A at position 58 can be substituted with G, T, V or I

A at position 67 can be substituted with T, V or S

S at position 68 can be substituted with F

S at position 69 can be substituted with N, T or V

Q at position 71 can be substituted with H

S at position 72 can be substituted with N, G, H, I or T

S at position 109 can be substituted with T or Y

Y at position 110 can be substituted with N, F or H

**FIGURE 57**

S at position 111 can be substituted with R, N, F or I

null (-) at position 134 can be substituted with A or S

P at position 135 can be substituted with T, I, F, A, L or V

P at position 136 can be substituted with L, F, or S

T at position 138 can be substituted with A or S

Arg Xaa Xaa Xaa Xaa Xaa Xaa Xaa Xaa Xaa Xaa (SEQ ID NO: 50153)

Xaa Xaa Xaa Xaa Leu Xaa Xaa (SEQ ID NO: 50154)

Gln Gln Xaa Xaa Xaa Xaa Xaa Xaa Phe Xaa (SEQ ID NO: 50155)

Arg Ala Ser Gln Ser Ile Ser Ser Tyr Leu Asn (SEQ ID NO: 50639)

Ala Ala Ser Ser Leu Gln Ser (SEQ ID NO: 50640)

Gln Gln Ser Tyr Ser Pro Pro Phe Thr (SEQ ID NO: 50641)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_10F12_LC | . | . | . | . | . | . | . | . | . | . | . | . | . | . | . | . | . | . | . | . | . | . | . | . | . | . |
| 21-225_11A5_LC | . | . | . | . | . | . | . | . | . | . | . | . | . | . | . | . | . | . | . | . | . | . | . | . | . | . |
| 21-225_14A11_LC | . | . | . | . | . | . | . | . | . | . | . | . | . | . | I | . | . | . | . | . | . | . | . | . | . | . |
| 21-225_158F4_LC | . | . | . | . | . | . | . | . | . | . | . | P | . | . | . | . | . | . | . | . | . | . | . | . | . | . |
| 21-225_16H6_LC | . | . | . | . | . | . | . | . | . | . | . | . | . | . | . | . | . | . | . | . | . | . | . | . | . | . |
| 21-225_17A12_LC | . | . | . | . | . | . | . | . | . | . | . | . | . | . | . | . | . | . | . | . | . | . | . | . | . | . |
| 21-225_17B4_LC | . | . | . | . | . | . | . | . | . | . | . | . | . | . | . | . | . | . | . | . | . | . | . | . | . | . |
| 21-225_17C6_LC | . | . | . | . | . | . | . | . | . | . | . | . | . | . | . | . | . | . | . | . | . | . | . | . | . | . |
| 21-225_17F11_LC | . | . | . | . | . | . | . | . | . | . | . | . | . | . | I | . | . | . | . | S | . | . | . | . | . | . |
| 21-225_17F7_LC | . | . | . | . | . | . | . | . | . | . | . | . | . | . | . | . | . | . | . | . | . | . | . | . | . | . |
| 21-225_180F8_LC | . | . | . | . | . | . | . | . | . | . | . | . | . | . | . | . | . | G | . | . | . | . | . | . | . | . |
| 21-225_18E12_LC | . | . | . | . | . | . | . | . | . | . | . | . | . | . | . | . | . | . | . | . | . | . | . | . | . | . |
| 21-225_19A5_LC | . | . | . | . | . | . | . | . | . | . | . | . | . | P | . | . | . | . | . | S | . | . | . | . | . | . |
| 21-225_19B3_LC | . | . | . | . | . | . | . | . | . | . | . | . | . | . | . | . | . | . | I | . | . | . | . | . | . | G |
| 21-225_20A4_LC | . | . | . | . | . | . | . | . | A | . | . | . | . | . | . | . | . | . | . | . | . | . | . | . | . | . |
| 21-225_20F3_LC | . | . | . | . | . | . | . | . | . | . | . | . | . | . | . | . | . | . | . | . | . | . | . | . | . | . |
| 21-225_21A2_LC | . | . | . | . | . | . | . | . | . | . | . | . | . | . | . | . | . | . | . | . | . | . | . | . | . | . |
| 21-225_21A5_LC | . | . | . | . | . | . | . | . | . | . | . | C | . | . | . | . | . | . | . | . | . | . | . | . | . | . |
| 21-225_21F4_LC | . | . | . | . | . | . | . | . | . | . | . | . | . | . | . | . | . | . | . | . | . | . | . | . | . | . |
| 21-225_222H4_LC | . | . | . | . | . | H | . | . | . | . | . | . | . | . | . | . | . | . | . | . | . | . | . | . | . | . |
| 21-225_222H8_LC | . | . | . | . | . | H | . | . | . | . | . | . | . | . | . | . | . | . | . | . | . | . | . | . | . | . |
| 21-225_22C5_LC | . | . | . | . | . | . | . | . | A | . | . | . | . | . | . | . | . | . | . | . | . | . | . | . | . | . |
| 21-225_22D5_LC | . | . | . | . | . | . | . | . | . | . | . | . | . | . | . | . | . | . | . | . | . | . | . | . | . | . |
| 21-225_22E4_LC | . | . | . | . | . | . | . | . | . | . | . | . | . | . | . | . | . | . | . | . | . | . | . | . | . | . |
| 21-225_22G10_LC | . | . | . | . | . | . | . | . | . | . | . | . | . | . | . | . | . | . | . | . | . | . | . | . | . | . |
| 21-225_22G3_LC | . | . | . | . | . | . | . | . | . | . | . | . | . | . | . | . | . | . | . | . | . | . | . | . | . | . |
| 21-225_22G5_LC | . | . | . | . | . | . | . | . | . | . | . | . | . | . | . | . | . | . | . | . | . | . | . | . | . | . |
| 21-225_30E11_LC | . | . | . | . | . | . | . | . | . | . | . | . | . | . | . | . | . | . | . | . | . | . | . | . | . | . |
| 21-225_33G12_LC | . | . | . | . | . | . | . | . | . | . | . | . | . | . | . | . | . | . | . | . | . | . | . | . | . | . |

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_3B1_LC | . | . | . | . | . | . | . | . | . | . | . | . | . | . | . | . | . | . | . | . | . | . | . | . | . | . |
| 21-225_3E3_LC | . | . | . | . | . | . | . | . | . | . | P | . | . | . | . | . | . | . | . | . | . | . | F | . | . | . |
| 21-225_48C3_LC | . | . | . | . | . | . | . | . | . | . | . | . | . | . | . | . | . | . | . | N | . | . | . | . | . | . |
| 21-225_48D1_LC | . | . | . | . | . | . | . | . | . | . | . | . | . | . | . | . | . | . | . | N | . | . | . | . | . | . |
| 21-225_49G12_LC | . | . | . | . | . | . | . | . | . | . | . | . | . | . | . | . | . | . | . | . | . | . | . | . | S | . |
| 21-225_4B1_LC | . | . | . | . | . | . | . | . | . | . | . | . | . | . | . | . | . | . | . | . | . | . | . | . | . | . |
| 21-225_4H2_LC | . | . | . | . | . | L | . | . | . | . | . | . | . | . | . | . | . | . | . | . | . | . | . | . | . | I |
| 21-225_50B12_LC | . | . | . | . | . | . | . | . | . | . | . | . | . | . | . | . | . | . | . | . | . | . | . | . | . | . |
| 21-225_50H10_LC | . | . | . | . | . | . | . | . | . | . | . | . | . | . | . | . | . | . | . | N | . | . | . | . | . | . |
| 21-225_50H8_LC | . | . | . | . | . | . | . | . | . | . | . | . | . | . | . | . | . | . | . | N | . | . | . | . | . | . |
| 21-225_51H10_LC | . | . | . | . | . | . | . | . | . | . | . | . | . | . | . | . | . | . | . | . | . | . | . | . | . | . |
| 21-225_53E2_LC | . | . | . | . | . | . | . | . | . | . | . | . | . | . | . | . | . | . | . | . | . | . | . | . | . | . |
| 21-225_56B2_LC | . | . | . | . | . | . | . | . | . | . | . | . | . | . | . | . | . | . | . | . | . | . | . | . | . | . |
| 21-225_57F2_LC | . | . | . | . | . | . | . | . | . | . | . | . | . | . | . | . | . | . | . | . | . | . | . | . | . | . |
| 21-225_5F7_LC | . | . | . | . | . | . | . | . | . | . | P | . | . | . | . | . | . | . | . | . | . | . | F | . | . | . |
| 21-225_5G2_LC | . | . | . | . | . | . | . | . | . | . | P | . | . | . | . | . | . | . | . | . | . | . | F | . | . | . |
| 21-225_60E12_LC | . | . | . | . | . | . | . | . | . | . | . | . | . | . | . | . | . | . | . | . | . | . | . | . | . | . |
| 21-225_61A1_LC | . | . | . | . | . | . | . | . | . | . | . | . | . | . | . | . | . | . | . | . | . | . | . | . | . | . |
| 21-225_62H1_LC | . | . | . | . | . | . | . | . | . | . | . | . | . | Y | . | . | . | . | . | . | . | . | . | . | . | . |
| 21-225_62H10_LC | . | . | . | . | . | . | . | . | . | . | . | . | . | . | . | . | . | . | . | . | . | . | . | . | . | . |
| 21-225_62H8_LC | . | . | . | . | . | . | . | . | . | . | . | . | . | . | . | . | . | . | . | . | . | . | . | . | . | . |
| 21-225_66A9_LC | . | . | . | . | . | . | . | . | . | . | . | . | . | . | . | . | . | . | . | . | . | . | . | . | . | . |
| 21-225_69C8_LC | . | . | . | . | . | . | . | . | . | . | . | . | . | . | . | . | . | . | . | . | F | . | . | . | . | G |
| 21-225_6D3_LC | . | . | . | . | . | . | . | . | . | . | . | . | . | . | . | . | . | . | . | . | . | . | . | . | T | . |
| 21-225_72G9_LC | . | . | . | . | . | . | . | . | . | . | . | . | . | . | . | . | . | . | . | . | . | . | . | . | . | . |
| 21-225_8E12_LC | . | . | . | . | . | . | . | . | . | . | . | . | . | . | . | . | . | . | . | . | . | . | . | . | . | . |
| 21-225_8G6_LC | . | . | . | . | . | . | . | . | . | . | . | . | . | . | . | . | . | . | . | . | . | . | . | . | . | . |
| 21-225_8H11_LC | . | . | . | . | . | . | . | . | . | . | . | . | . | F | . | . | . | . | . | . | . | . | . | . | . | . |
| 21-225_9E5_LC | . | . | . | . | . | . | . | Q | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | I | S | - | - | - | - | - | - | S | Y | L | N | W | Y | Q | Q | K | P | G | K | A | P |
| 21-225_10F12_LC | . | . | . | N | . | . | . | . | . | . | . | . | . | . | . | . | . | . | . | . | . | . | . | . | . | . |
| 21-225_11A5_LC | . | . | . | N | . | Y | . | . | . | . | . | . | . | . | . | . | . | . | . | . | . | . | . | . | . | . |
| 21-225_14A11_LC | . | . | . | . | . | . | . | . | . | . | . | . | . | . | . | . | . | . | . | . | . | . | . | . | . | . |
| 21-225_158F4_LC | . | . | . | N | . | R | . | . | . | . | . | . | K | . | . | H | . | . | . | F | L | . | . | . | . | . |
| 21-225_16H6_LC | . | . | . | N | . | . | . | . | . | . | . | . | . | . | . | . | . | . | . | . | . | . | . | . | . | . |
| 21-225_17A12_LC | . | . | . | . | . | . | . | . | . | . | . | . | . | . | . | . | . | . | H | . | . | . | . | . | . | . |
| 21-225_17B4_LC | . | . | . | N | . | . | . | . | . | . | . | . | . | . | . | . | . | . | . | . | . | . | . | . | . | . |
| 21-225_17C6_LC | . | . | . | . | . | N | . | . | . | . | . | . | T | . | . | . | . | . | . | . | . | . | . | . | . | . |
| 21-225_17F11_LC | . | . | R | T | . | N | . | . | . | . | . | . | N | . | . | . | . | . | . | . | . | . | . | . | . | . |
| 21-225_17F7_LC | . | . | . | T | . | . | . | . | . | . | . | . | . | F | . | . | . | . | . | . | . | . | . | . | . | . |
| 21-225_180F8_LC | . | . | . | T | . | L | . | . | . | . | . | . | . | . | . | . | . | . | . | . | . | . | . | . | . | . |
| 21-225_18E12_LC | . | . | . | . | . | . | . | . | . | . | . | . | . | . | . | . | . | . | . | . | . | . | . | . | . | . |
| 21-225_19A5_LC | . | . | . | N | . | I | . | . | . | . | . | . | N | . | . | . | . | . | . | . | . | . | . | . | . | . |
| 21-225_19B3_LC | . | . | . | N | . | I | . | . | . | . | . | . | N | . | . | . | . | . | . | . | . | . | . | . | . | . |
| 21-225_20A4_LC | . | . | . | . | . | I | . | . | . | . | . | . | N | . | . | . | . | . | . | . | R | . | . | . | S | . |
| 21-225_20F3_LC | . | . | . | . | . | . | . | . | . | . | . | . | . | . | . | Y | . | . | . | . | . | . | . | . | . | . |
| 21-225_21A2_LC | . | . | . | . | . | . | . | . | . | . | . | . | . | . | . | . | . | . | . | . | . | . | . | . | . | . |
| 21-225_21A5_LC | . | . | . | T | . | . | . | . | . | . | . | . | . | H | . | . | . | . | . | R | . | . | . | . | . | . |
| 21-225_21F4_LC | . | . | . | N | . | G | . | . | . | . | . | . | . | . | . | . | . | . | . | . | . | . | . | . | . | . |
| 21-225_222H4_LC | . | . | . | N | . | . | . | . | . | . | . | . | N | . | V | . | . | . | . | . | . | . | . | . | . | . |
| 21-225_222H8_LC | . | . | . | N | . | . | . | . | . | . | . | . | N | . | V | . | . | . | . | . | . | . | . | . | . | . |
| 21-225_22C5_LC | . | . | . | N | . | . | . | . | . | . | . | . | . | . | . | . | . | . | . | . | . | . | . | . | . | . |
| 21-225_22D5_LC | . | . | . | N | . | F | . | . | . | . | . | . | . | . | . | . | . | . | H | . | . | . | . | . | . | . |
| 21-225_22E4_LC | . | . | . | N | . | . | . | . | . | . | . | . | . | . | . | . | . | . | . | . | . | . | . | . | . | . |
| 21-225_22G10_LC | . | . | . | . | . | F | . | . | . | . | . | . | . | . | . | . | . | . | . | . | . | . | . | . | . | . |
| 21-225_22G3_LC | . | . | . | . | . | . | . | . | . | . | . | . | N | . | . | . | . | . | . | . | . | . | . | . | . | . |
| 21-225_22G5_LC | . | . | . | N | . | . | . | . | . | . | . | . | . | . | . | . | . | . | . | . | . | Q | . | . | . | . |
| 21-225_30E11_LC | . | . | . | . | . | . | . | . | . | . | . | . | N | . | . | . | . | . | . | . | Q | T | . | . | . | . |
| 21-225_33G12_LC | . | . | . | . | . | . | . | . | . | . | . | . | N | . | . | . | . | . | . | . | Q | T | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | I | S | - | - | - | - | - | - | S | Y | L | N | W | Y | Q | Q | K | P | G | K | A | P |
| 21-225_3B1_LC | . | . | . | . | . | N | . | . | . | . | . | . | N | . | . | . | . | . | . | . | . | . | . | . | . | . |
| 21-225_3E3_LC | . | . | . | . | . | R | . | . | . | . | . | . | . | . | . | . | . | . | . | . | . | . | . | . | . | H |
| 21-225_48C3_LC | . | . | . | . | . | I | . | . | . | . | . | . | . | . | . | I | . | . | . | . | . | . | . | . | . | . |
| 21-225_48D1_LC | . | . | . | . | . | . | . | . | . | . | . | . | . | . | . | I | . | . | . | . | . | . | . | . | . | . |
| 21-225_49G12_LC | . | . | . | . | . | . | . | . | . | . | . | . | T | . | . | M | . | . | . | . | . | . | . | . | . | . |
| 21-225_4B1_LC | . | . | H | T | . | R | . | . | . | . | . | . | T | . | . | . | . | . | . | . | . | . | . | . | . | . |
| 21-225_4H2_LC | . | . | . | N | . | . | . | . | . | . | . | . | . | . | . | . | . | . | . | . | . | . | . | . | . | . |
| 21-225_50B12_LC | . | . | . | . | . | . | . | . | . | . | . | . | . | . | . | . | . | . | . | H | . | . | . | . | . | . |
| 21-225_50H10_LC | . | . | . | . | . | Y | . | . | . | . | . | . | . | . | . | I | . | . | . | . | . | . | . | . | . | . |
| 21-225_50H8_LC | . | . | . | . | . | . | . | . | . | . | . | . | . | . | . | I | . | . | . | . | . | . | . | . | . | . |
| 21-225_51H10_LC | . | . | . | . | . | . | . | . | . | . | . | . | T | . | . | M | . | . | . | . | . | . | . | . | . | . |
| 21-225_53E2_LC | . | . | . | . | F | . | . | . | . | . | . | . | H | . | . | . | . | . | . | . | . | . | . | . | . | . |
| 21-225_56B2_LC | . | . | . | . | F | . | . | . | . | . | . | . | H | . | . | . | . | . | . | . | . | . | . | . | . | . |
| 21-225_57F2_LC | . | . | . | . | . | . | . | . | . | . | . | . | . | . | . | . | . | . | . | . | . | . | . | . | . | . |
| 21-225_5F7_LC | . | . | . | T | . | . | . | . | . | . | . | . | . | . | . | . | . | . | . | . | . | . | . | . | . | . |
| 21-225_5G2_LC | . | . | . | N | . | I | . | . | . | . | . | . | . | . | . | . | . | . | . | . | . | . | . | . | . | R |
| 21-225_60E12_LC | . | . | . | . | . | . | . | . | . | . | . | . | . | . | . | . | . | . | . | . | . | . | . | . | . | . |
| 21-225_61A1_LC | . | . | . | . | . | . | . | . | . | . | . | . | . | . | . | . | . | . | . | . | . | . | . | . | . | . |
| 21-225_62H1_LC | . | . | . | N | . | F | . | . | . | . | . | . | . | . | . | . | . | . | . | . | . | . | . | . | . | . |
| 21-225_62H10_LC | . | . | . | . | . | T | . | . | . | . | . | . | . | . | . | . | . | . | . | . | . | . | . | . | V | . |
| 21-225_62H8_LC | . | . | . | . | . | F | . | . | . | . | . | . | . | . | . | . | . | . | . | . | N | . | . | . | . | . |
| 21-225_66A9_LC | . | . | . | N | . | F | . | . | . | . | . | . | . | . | . | . | . | . | . | . | . | . | . | . | . | . |
| 21-225_69C8_LC | . | . | . | T | . | Y | . | . | . | . | . | . | N | . | . | . | . | . | . | . | . | . | . | . | . | . |
| 21-225_6D3_LC | . | . | . | . | . | . | . | . | . | . | . | . | T | . | . | . | . | . | . | . | . | . | . | . | . | . |
| 21-225_72G9_LC | . | . | . | . | . | T | . | . | . | . | . | . | . | . | . | . | . | . | . | . | . | . | . | . | . | . |
| 21-225_8E12_LC | . | . | . | . | . | . | . | . | . | . | . | . | N | . | . | . | . | . | . | . | . | . | . | . | . | . |
| 21-225_8G6_LC | . | . | . | . | . | . | . | . | . | . | . | . | . | . | . | . | . | . | . | . | . | . | . | . | . | . |
| 21-225_8H11_LC | . | . | . | N | . | I | . | . | . | . | . | . | . | . | . | . | . | . | . | . | . | . | . | . | . | . |
| 21-225_9E5_LC | . | . | . | . | . | A | . | . | . | . | . | . | . | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | L | L | I | Y | A | - | - | - | - | - | - | - | - | A | S | S | L | Q | S | G | V | P | S | R | F |
| 21-225_10F12_LC | . | . | . | . | . | . | . | . | . | . | . | . | . | . | . | . | . | . | . | . | . | . | . | . | . | . |
| 21-225_11A5_LC | . | . | . | . | . | . | . | . | . | . | . | . | . | . | . | . | . | . | . | . | . | . | . | . | . | . |
| 21-225_14A11_LC | . | . | . | . | F | . | . | . | . | . | . | . | . | . | . | . | . | . | . | N | . | . | . | . | . | . |
| 21-225_158F4_LC | . | . | . | . | . | T | . | . | . | . | . | . | . | . | . | . | T | . | . | . | . | . | . | . | . | . |
| 21-225_16H6_LC | . | . | . | . | . | . | . | . | . | . | . | . | . | . | . | . | . | . | . | . | . | . | . | . | . | . |
| 21-225_17A12_LC | . | . | . | . | . | . | . | . | . | . | . | . | . | . | . | . | . | . | . | . | . | . | . | . | . | . |
| 21-225_17B4_LC | . | . | . | . | F | G | . | . | . | . | . | . | . | . | . | . | . | . | . | T | . | . | . | . | . | . |
| 21-225_17C6_LC | . | . | . | . | . | . | . | . | . | . | . | . | . | . | . | . | . | . | . | . | . | . | . | . | . | . |
| 21-225_17F11_LC | . | . | . | . | F | . | . | . | . | . | . | . | . | . | T | . | . | . | . | . | . | . | . | . | . | . |
| 21-225_17F7_LC | . | . | . | . | . | . | . | . | . | . | . | . | . | . | . | . | . | . | . | . | . | . | . | . | . | . |
| 21-225_180F8_LC | . | . | . | . | . | G | . | . | . | . | . | . | . | . | V | . | . | . | . | . | . | . | . | . | . | . |
| 21-225_18E12_LC | T | . | . | . | . | . | . | . | . | . | . | . | . | . | . | . | . | . | . | . | . | . | . | . | . | . |
| 21-225_19A5_LC | . | . | . | . | D | V | . | . | . | . | . | . | . | . | . | . | N | . | . | G | . | . | . | . | . | . |
| 21-225_19B3_LC | N | . | . | . | . | V | . | . | . | . | . | . | . | . | . | . | . | . | . | . | . | . | . | . | . | . |
| 21-225_20A4_LC | . | . | . | . | . | . | . | . | . | . | . | . | . | . | . | . | . | . | H | . | . | . | . | . | . | . |
| 21-225_20F3_LC | . | . | . | . | . | . | . | . | . | . | . | . | . | . | . | . | . | . | . | . | . | . | . | . | . | . |
| 21-225_21A2_LC | . | . | . | . | . | . | . | . | . | . | . | . | . | . | . | . | . | . | . | . | . | . | . | . | . | . |
| 21-225_21A5_LC | . | . | . | . | . | . | . | . | . | . | . | . | . | . | . | . | . | . | . | . | . | . | . | . | . | . |
| 21-225_21F4_LC | . | . | . | . | F | . | . | . | . | . | . | . | . | . | . | . | . | . | . | . | . | . | . | . | . | . |
| 21-225_222H4_LC | . | F | . | . | . | T | . | . | . | . | . | . | . | . | . | . | . | . | . | . | . | . | S | . | . | . |
| 21-225_222H8_LC | . | F | . | . | . | I | . | . | . | . | . | . | . | . | . | . | . | . | . | . | . | . | S | . | . | . |
| 21-225_22C5_LC | . | . | . | . | . | . | . | . | . | . | . | . | . | . | . | . | V | . | . | H | . | I | . | . | . | . |
| 21-225_22D5_LC | . | . | . | . | . | . | . | . | . | . | . | . | . | . | . | . | . | . | . | . | . | . | . | . | . | . |
| 21-225_22E4_LC | . | . | . | . | . | . | . | . | . | . | . | . | . | . | . | . | . | . | . | . | . | . | . | . | . | . |
| 21-225_22G10_LC | . | . | . | . | . | . | . | . | . | . | . | . | . | . | . | . | . | . | . | . | . | . | . | . | . | . |
| 21-225_22G3_LC | . | . | . | . | F | . | . | . | . | . | . | . | . | . | . | . | . | . | . | . | . | . | . | . | . | . |
| 21-225_22G5_LC | . | . | . | . | . | . | . | . | . | . | . | . | . | . | . | . | . | . | . | N | . | . | . | . | . | . |
| 21-225_30E11_LC | . | F | . | . | . | . | . | . | . | . | . | . | . | . | . | . | . | . | . | . | . | . | . | . | . | . |
| 21-225_33G12_LC | . | F | . | . | . | . | . | . | . | . | . | . | . | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | Y | A | - | - | - | - | - | - | - | - | A | S | S | L | Q | S | G | V | P | S | R | F |
| 21-225_3B1_LC | . | . | . | . | . | . | . | . | . | . | . | . | . | . | T | . | . | . | . | N | . | . | . | . | . | . |
| 21-225_3E3_LC | . | . | V | . | . | G | . | . | . | . | . | . | . | . | T | . | . | . | . | . | . | . | . | . | . | . |
| 21-225_48C3_LC | R | . | . | . | . | . | . | . | . | . | . | . | . | . | . | . | . | . | . | . | . | . | . | . | . | . |
| 21-225_48D1_LC | . | . | . | . | . | . | . | . | . | . | . | . | . | . | . | . | . | . | . | . | . | . | . | . | . | . |
| 21-225_49G12_LC | . | . | . | . | . | . | . | . | . | . | . | . | . | . | . | . | . | . | . | I | . | . | . | . | E | . |
| 21-225_4B1_LC | . | . | . | . | . | . | . | . | . | . | . | . | . | . | . | . | . | . | . | . | . | . | . | . | . | I |
| 21-225_4H2_LC | . | . | V | . | . | . | . | . | . | . | . | . | . | . | . | . | . | . | . | . | . | . | . | . | . | . |
| 21-225_50B12_LC | R | . | . | . | . | . | . | . | . | . | . | . | . | . | . | . | . | . | . | . | . | . | . | . | . | . |
| 21-225_50H10_LC | . | . | . | . | . | . | . | . | . | . | . | . | . | . | . | . | . | . | . | . | . | . | . | . | . | . |
| 21-225_50H8_LC | . | . | . | . | . | . | . | . | . | . | . | . | . | . | . | . | . | . | . | . | . | . | . | . | . | . |
| 21-225_51H10_LC | . | . | . | . | . | . | . | . | . | . | . | . | . | . | . | . | . | . | . | . | . | . | . | . | E | . |
| 21-225_53E2_LC | N | . | . | . | F | . | . | . | . | . | . | . | . | . | V | . | . | . | . | . | . | . | . | . | . | . |
| 21-225_56B2_LC | N | . | . | . | F | . | . | . | . | . | . | . | . | . | V | . | . | . | . | . | . | . | . | . | . | . |
| 21-225_57F2_LC | . | . | . | . | . | . | . | . | . | . | . | . | . | . | . | . | . | . | . | . | . | . | . | . | . | . |
| 21-225_5F7_LC | . | . | . | . | S | . | . | . | . | . | . | . | . | . | . | . | . | . | . | . | . | . | . | . | . | . |
| 21-225_5G2_LC | . | . | V | . | . | G | . | . | . | . | . | . | . | . | . | . | . | . | . | . | . | . | . | . | . | . |
| 21-225_60E12_LC | . | . | . | . | . | . | . | . | . | . | . | . | . | . | . | . | . | . | . | . | . | . | . | . | . | . |
| 21-225_61A1_LC | . | . | . | . | . | . | . | . | . | . | . | . | . | . | . | . | . | . | . | . | . | . | . | . | . | . |
| 21-225_62H1_LC | . | V | . | . | . | . | . | . | . | . | . | . | . | . | V | F | . | . | . | . | . | . | . | . | . | . |
| 21-225_62H10_LC | . | . | . | . | S | T | . | . | . | . | . | . | . | . | . | . | . | . | . | . | . | . | . | . | . | . |
| 21-225_62H8_LC | . | . | . | . | . | . | . | . | . | . | . | . | . | . | S | . | . | . | . | . | . | . | . | . | . | L |
| 21-225_66A9_LC | . | . | . | . | F | . | . | . | . | . | . | . | . | . | . | . | . | . | . | . | . | . | . | . | . | . |
| 21-225_69C8_LC | . | . | . | . | H | V | . | . | . | . | . | . | . | . | . | . | . | . | . | . | . | . | . | . | . | . |
| 21-225_6D3_LC | . | . | . | . | F | . | . | . | . | . | . | . | . | . | . | . | . | . | . | . | . | . | . | . | . | . |
| 21-225_72G9_LC | . | . | . | . | . | T | . | . | . | . | . | . | . | . | . | . | . | . | . | . | . | . | . | . | . | . |
| 21-225_8E12_LC | . | . | . | . | . | . | . | . | . | . | . | . | . | . | . | . | . | . | . | . | . | . | . | . | . | . |
| 21-225_8G6_LC | . | . | . | . | F | . | . | . | . | . | . | . | . | . | . | . | . | . | . | . | . | . | . | . | . | . |
| 21-225_8H11_LC | N | . | M | . | . | V | . | . | . | . | . | . | . | . | T | . | . | . | H | . | . | . | . | . | . | . |
| 21-225_9E5_LC | . | . | . | . | . | G | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_10F12_LC | . | . | . | . | . | . | . | . | . | . | . | . | . | . | . | . | . | . | . | . | . | . | . | . | . | . |
| 21-225_11A5_LC | . | . | . | . | . | . | . | . | . | . | . | . | . | . | . | . | . | . | . | . | . | . | . | . | . | . |
| 21-225_14A11_LC | . | . | . | . | . | . | . | . | . | . | . | . | . | . | . | . | . | . | . | . | . | . | . | . | . | . |
| 21-225_158F4_LC | . | . | . | . | . | . | . | . | . | . | . | . | . | . | . | . | . | . | . | . | . | . | . | . | . | . |
| 21-225_16H6_LC | . | . | R | . | . | . | . | . | . | . | . | . | . | . | . | . | . | . | . | . | . | . | . | . | . | . |
| 21-225_17A12_LC | . | . | . | . | . | . | . | . | . | . | . | . | . | . | . | . | N | . | . | . | . | . | . | . | . | . |
| 21-225_17B4_LC | . | . | . | . | . | . | . | . | . | . | . | . | . | . | . | N | . | . | . | . | . | . | . | . | . | . |
| 21-225_17C6_LC | . | . | . | . | . | . | . | . | . | . | . | . | . | . | . | . | . | . | . | . | . | . | . | . | . | . |
| 21-225_17F11_LC | . | . | . | . | . | . | . | . | . | . | . | . | . | . | . | . | . | . | . | . | . | . | . | . | . | . |
| 21-225_17F7_LC | . | . | . | . | . | . | . | . | . | . | . | . | . | . | . | . | G | . | . | . | . | . | . | . | . | . |
| 21-225_180F8_LC | . | . | . | . | . | . | . | . | . | . | . | S | . | . | . | . | . | . | . | . | . | . | . | . | . | . |
| 21-225_18E12_LC | . | . | . | . | . | . | . | . | . | . | . | . | . | . | . | N | . | . | . | . | . | . | . | . | . | . |
| 21-225_19A5_LC | . | . | . | . | . | . | . | . | . | . | . | . | . | . | . | . | . | . | . | . | . | . | . | . | . | . |
| 21-225_19B3_LC | N | . | . | . | . | . | . | . | . | . | . | . | . | . | . | . | . | . | . | . | . | . | . | . | . | . |
| 21-225_20A4_LC | . | . | R | . | . | . | . | . | . | . | . | . | . | . | . | . | . | . | . | . | . | . | . | . | . | . |
| 21-225_20F3_LC | . | . | . | . | . | . | . | . | . | . | . | . | . | . | . | . | . | . | . | . | . | . | . | . | . | . |
| 21-225_21A2_LC | . | . | . | . | . | . | . | . | . | . | . | . | . | . | . | . | . | . | . | . | . | . | . | . | . | . |
| 21-225_21A5_LC | . | . | . | . | . | . | . | . | . | . | . | . | . | . | . | . | . | V | . | . | . | . | . | . | . | . |
| 21-225_21F4_LC | . | . | . | . | . | . | . | . | . | . | . | . | . | . | . | . | . | . | . | . | . | . | . | . | . | F |
| 21-225_222H4_LC | . | . | . | . | . | . | . | . | . | . | . | . | . | . | . | . | . | V | H | R | D | . | . | . | I | . |
| 21-225_222H8_LC | . | . | . | . | . | . | . | . | . | . | . | . | . | . | . | . | . | V | H | R | D | . | . | . | I | . |
| 21-225_22C5_LC | . | . | R | . | . | . | . | . | . | . | . | . | . | I | . | . | . | . | . | . | . | . | . | . | . | . |
| 21-225_22D5_LC | . | . | . | . | . | . | . | . | . | . | . | . | . | . | . | . | . | . | . | . | . | . | . | . | . | . |
| 21-225_22E4_LC | . | . | . | . | . | . | . | . | . | . | . | . | . | . | . | . | . | . | . | . | . | . | . | . | . | . |
| 21-225_22G10_LC | . | . | . | . | . | . | . | . | . | . | . | . | . | . | . | . | . | . | . | . | . | . | . | . | . | . |
| 21-225_22G3_LC | . | . | . | . | . | . | . | . | . | . | . | . | . | . | . | . | G | . | . | . | . | . | . | . | . | . |
| 21-225_22G5_LC | . | . | R | . | . | . | . | . | . | . | . | . | . | . | . | . | . | . | . | . | . | . | . | . | . | . |
| 21-225_30E11_LC | . | . | . | . | . | . | . | . | . | . | . | . | . | . | . | . | . | . | . | . | . | . | . | . | . | . |
| 21-225_33G12_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_3B1_LC | . | . | . | . | . | . | . | . | . | . | . | . | . | . | . | . | . | . | . | . | . | . | . | . | . | . |
| 21-225_3E3_LC | . | . | . | . | . | . | . | . | . | . | . | . | . | . | . | . | . | . | . | . | . | . | . | . | . | . |
| 21-225_48C3_LC | . | A | . | . | . | . | . | . | . | . | . | . | . | . | . | . | . | . | . | . | . | . | . | . | S | . |
| 21-225_48D1_LC | . | A | . | . | . | . | . | . | . | . | . | . | . | . | . | . | . | . | . | . | . | . | . | . | S | . |
| 21-225_49G12_LC | . | A | . | . | . | . | . | . | . | . | . | . | . | . | . | . | . | . | . | . | . | . | . | . | . | . |
| 21-225_4B1_LC | N | . | . | . | . | . | . | . | . | . | . | . | . | . | . | T | N | . | . | . | . | . | . | . | . | . |
| 21-225_4H2_LC | . | . | R | . | . | . | . | . | . | . | . | . | . | . | . | G | C | V | . | R | . | . | . | . | . | . |
| 21-225_50B12_LC | . | . | . | E | . | . | . | . | . | . | . | I | . | . | . | . | . | . | . | . | . | . | . | T | . | . |
| 21-225_50H10_LC | . | A | . | . | . | . | . | . | . | . | . | . | . | . | . | . | . | . | . | . | . | . | . | . | S | . |
| 21-225_50H8_LC | . | A | . | . | . | . | . | . | . | . | . | . | . | . | . | . | . | . | . | . | . | . | . | . | S | . |
| 21-225_51H10_LC | . | A | . | . | . | . | . | . | . | . | . | . | . | . | . | . | . | . | . | . | . | . | . | . | . | . |
| 21-225_53E2_LC | . | . | . | . | . | . | . | . | S | . | . | . | . | P | . | . | . | . | . | . | . | . | . | . | I | . |
| 21-225_56B2_LC | . | . | . | . | . | . | . | . | . | . | . | . | . | . | . | . | . | . | . | . | . | . | . | . | I | . |
| 21-225_57F2_LC | . | . | . | . | . | . | . | . | . | . | . | . | . | . | . | . | . | . | . | . | . | . | . | . | P | . |
| 21-225_5F7_LC | . | . | . | . | . | . | . | . | . | . | . | . | . | . | . | . | . | . | . | . | . | . | . | . | . | . |
| 21-225_5G2_LC | . | . | . | . | . | . | . | . | . | . | . | . | . | N | . | R | . | . | . | . | . | . | . | . | . | . |
| 21-225_60E12_LC | . | . | . | . | . | . | . | . | . | . | . | . | . | . | . | . | . | . | . | . | . | . | . | . | P | . |
| 21-225_61A1_LC | . | . | . | . | . | . | . | . | . | . | . | . | . | . | . | . | . | . | . | . | . | . | . | . | P | . |
| 21-225_62H1_LC | . | . | . | . | . | . | . | . | . | . | . | . | . | . | . | . | . | . | . | . | . | . | . | . | . | . |
| 21-225_62H10_LC | . | . | . | . | . | . | . | . | . | . | . | . | . | . | . | . | . | . | . | . | . | . | . | . | . | . |
| 21-225_62H8_LC | . | . | N | . | . | . | . | . | . | . | . | I | . | . | . | . | . | . | . | . | . | . | . | . | . | . |
| 21-225_66A9_LC | . | . | . | . | . | . | . | . | . | . | . | . | . | . | . | . | . | . | . | . | . | . | . | . | . | . |
| 21-225_69C8_LC | . | . | . | . | . | . | . | . | . | . | . | . | . | V | . | . | . | . | . | . | . | . | . | . | . | . |
| 21-225_6D3_LC | . | . | . | . | . | . | . | . | . | . | . | . | . | . | . | . | . | . | . | . | . | . | . | . | . | . |
| 21-225_72G9_LC | . | . | . | . | . | . | . | . | . | . | . | . | . | . | . | . | . | . | . | . | . | . | . | . | . | . |
| 21-225_8E12_LC | . | . | . | . | . | . | . | . | . | . | . | . | . | . | . | . | . | . | . | . | . | . | . | . | . | . |
| 21-225_8G6_LC | . | . | . | . | . | . | . | . | . | . | . | . | . | . | . | . | . | . | . | . | . | . | . | . | . | . |
| 21-225_8H11_LC | . | . | . | . | . | . | . | . | . | . | . | S | . | . | . | . | . | . | . | . | . | . | . | . | . | . |
| 21-225_9E5_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | S | Y | S | - | - | - | - | - | - | - | - | - | - | - | - | - | - | - | - | - | - | - |
| 21-225_10F12_LC | . | . | . | . | . | . | N | . | . | . | . | . | . | . | . | . | . | . | . | . | . | . | . | . | . | . |
| 21-225_11A5_LC | . | . | . | . | . | . | N | . | . | . | . | . | . | . | . | . | . | . | . | . | . | . | . | . | . | . |
| 21-225_14A11_LC | . | . | . | . | T | . | . | . | . | . | . | . | . | . | . | . | . | . | . | . | . | . | . | . | . | . |
| 21-225_158F4_LC | . | . | . | . | . | . | N | . | . | . | . | . | . | . | . | . | . | . | . | . | . | . | . | . | . | . |
| 21-225_16H6_LC | . | . | . | . | . | . | R | . | . | . | . | . | . | . | . | . | . | . | . | . | . | . | . | . | . | . |
| 21-225_17A12_LC | . | . | . | . | . | . | N | . | . | . | . | . | . | . | . | . | . | . | . | . | . | . | . | . | . | . |
| 21-225_17B4_LC | F | . | . | . | . | . | R | . | . | . | . | . | . | . | . | . | . | . | . | . | . | . | . | . | . | . |
| 21-225_17C6_LC | F | . | . | . | . | . | R | . | . | . | . | . | . | . | . | . | . | . | . | . | . | . | . | . | . | . |
| 21-225_17F11_LC | . | . | . | . | T | . | . | . | . | . | . | . | . | . | . | . | . | . | . | . | . | . | . | . | . | . |
| 21-225_17F7_LC | F | . | . | . | . | . | R | . | . | . | . | . | . | . | . | . | . | . | . | . | . | . | . | . | . | . |
| 21-225_180F8_LC | . | . | . | . | . | . | . | . | . | . | . | . | . | . | . | . | . | . | . | . | . | . | . | . | . | . |
| 21-225_18E12_LC | F | . | . | . | . | . | R | . | . | . | . | . | . | . | . | . | . | . | . | . | . | . | . | . | . | . |
| 21-225_19A5_LC | . | . | . | . | . | . | . | . | . | . | . | . | . | . | . | . | . | . | . | . | . | . | . | . | . | . |
| 21-225_19B3_LC | . | . | . | . | . | . | . | . | . | . | . | . | . | . | . | . | . | . | . | . | . | . | . | . | . | . |
| 21-225_20A4_LC | F | . | . | . | . | . | R | . | . | . | . | . | . | . | . | . | . | . | . | . | . | . | . | . | . | . |
| 21-225_20F3_LC | . | . | . | . | . | . | . | . | . | . | . | . | . | . | . | . | . | . | . | . | . | . | . | . | . | . |
| 21-225_21A2_LC | F | . | . | . | . | . | R | . | . | . | . | . | . | . | . | . | . | . | . | . | . | . | . | . | . | . |
| 21-225_21A5_LC | . | . | . | . | . | . | N | . | . | . | . | . | . | . | . | . | . | . | . | . | . | . | . | . | . | . |
| 21-225_21F4_LC | F | . | . | . | . | . | R | . | . | . | . | . | . | . | . | . | . | . | . | . | . | . | . | . | . | . |
| 21-225_222H4_LC | . | . | . | . | Y | . | F | . | . | . | . | . | . | . | . | . | . | . | . | . | . | . | . | . | . | . |
| 21-225_222H8_LC | . | . | . | . | Y | . | F | . | . | . | . | . | . | . | . | . | . | . | . | . | . | . | . | . | . | . |
| 21-225_22C5_LC | . | . | . | . | . | . | R | . | . | . | . | . | . | . | . | . | . | . | . | . | . | . | . | . | . | . |
| 21-225_22D5_LC | . | . | . | . | . | . | . | . | . | . | . | . | . | . | . | . | . | . | . | . | . | . | . | . | . | . |
| 21-225_22E4_LC | . | . | . | . | . | . | N | . | . | . | . | . | . | . | . | . | . | . | . | . | . | . | . | . | . | . |
| 21-225_22G10_LC | F | . | . | . | . | F | R | . | . | . | . | . | . | . | . | . | . | . | . | . | . | . | . | . | . | . |
| 21-225_22G3_LC | F | . | . | . | . | F | R | . | . | . | . | . | . | . | . | . | . | . | . | . | . | . | . | . | . | . |
| 21-225_22G5_LC | . | . | . | . | . | . | R | . | . | . | . | . | . | . | . | . | . | . | . | . | . | . | . | . | . | . |
| 21-225_30E11_LC | . | . | . | . | . | . | . | . | . | . | . | . | . | . | . | . | . | . | . | . | . | . | . | . | . | . |
| 21-225_33G12_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | S | Y | S | - | - | - | - | - | - | - | - | - | - | - | - | - | - | - | - | - | - | - |
| 21-225_3B1_LC | . | . | . | . | . | . | . | . | . | . | . | . | . | . | . | . | . | . | . | . | . | . | . | . | . | . |
| 21-225_3E3_LC | . | . | . | . | . | . | . | . | . | . | . | . | . | . | . | . | . | . | . | . | . | . | . | . | . | . |
| 21-225_48C3_LC | . | . | . | . | T | N | . | . | . | . | . | . | . | . | . | . | . | . | . | . | . | . | . | . | . | . |
| 21-225_48D1_LC | . | . | . | . | . | N | . | . | . | . | . | . | . | . | . | . | . | . | . | . | . | . | . | . | . | . |
| 21-225_49G12_LC | . | . | . | . | . | . | . | . | . | . | . | . | . | . | . | . | . | . | . | . | . | . | . | . | . | . |
| 21-225_4B1_LC | . | . | . | . | . | . | N | . | . | . | . | . | . | . | . | . | . | . | . | . | . | . | . | . | . | . |
| 21-225_4H2_LC | . | . | . | . | . | . | R | . | . | . | . | . | . | . | . | . | . | . | . | . | . | . | . | . | . | . |
| 21-225_50B12_LC | . | . | . | . | . | . | I | . | . | . | . | . | . | . | . | . | . | . | . | . | . | . | . | . | . | . |
| 21-225_50H10_LC | . | . | . | . | . | N | . | . | . | . | . | . | . | . | . | . | . | . | . | . | . | . | . | . | . | . |
| 21-225_50H8_LC | . | . | . | . | . | N | . | . | . | . | . | . | . | . | . | . | . | . | . | . | . | . | . | . | . | . |
| 21-225_51H10_LC | . | . | . | . | . | . | . | . | . | . | . | . | . | . | . | . | . | . | . | . | . | . | . | . | . | . |
| 21-225_53E2_LC | F | . | . | . | . | F | . | . | . | . | . | . | . | . | . | . | . | . | . | . | . | . | . | . | . | . |
| 21-225_56B2_LC | F | . | . | . | . | . | . | . | . | . | . | . | . | . | . | . | . | . | . | . | . | . | . | . | . | . |
| 21-225_57F2_LC | . | . | . | . | . | . | N | . | . | . | . | . | . | . | . | . | . | . | . | . | . | . | . | . | . | . |
| 21-225_5F7_LC | . | . | . | . | . | . | N | . | . | . | . | . | . | . | . | . | . | . | . | . | . | . | . | . | . | . |
| 21-225_5G2_LC | . | . | . | . | . | . | . | . | . | . | . | . | . | . | . | . | . | . | . | . | . | . | . | . | . | . |
| 21-225_60E12_LC | . | . | . | . | . | . | N | . | . | . | . | . | . | . | . | . | . | . | . | . | . | . | . | . | . | . |
| 21-225_61A1_LC | . | . | . | . | . | . | N | . | . | . | . | . | . | . | . | . | . | . | . | . | . | . | . | . | . | . |
| 21-225_62H1_LC | . | . | . | . | . | . | . | . | . | . | . | . | . | . | . | . | . | . | . | . | . | . | . | . | . | . |
| 21-225_62H10_LC | . | . | . | . | . | H | . | . | . | . | . | . | . | . | . | . | . | . | . | . | . | . | . | . | . | . |
| 21-225_62H8_LC | . | . | . | . | . | . | . | . | . | . | . | . | . | . | . | . | . | . | . | . | . | . | . | . | . | . |
| 21-225_66A9_LC | . | . | . | . | T | . | . | . | . | . | . | . | . | . | . | . | . | . | . | . | . | . | . | . | . | . |
| 21-225_69C8_LC | . | . | . | . | . | . | . | . | . | . | . | . | . | . | . | . | . | . | . | . | . | . | . | . | . | . |
| 21-225_6D3_LC | F | . | . | . | . | . | R | . | . | . | . | . | . | . | . | . | . | . | . | . | . | . | . | . | . | . |
| 21-225_72G9_LC | . | . | . | . | . | H | . | . | . | . | . | . | . | . | . | . | . | . | . | . | . | . | . | . | . | . |
| 21-225_8E12_LC | F | . | . | . | . | . | R | . | . | . | . | . | . | . | . | . | . | . | . | . | . | . | . | . | . | . |
| 21-225_8G6_LC | F | . | . | . | . | . | R | . | . | . | . | . | . | . | . | . | . | . | . | . | . | . | . | . | . | . |
| 21-225_8H11_LC | . | . | . | . | . | . | . | . | . | . | . | . | . | . | . | . | . | . | . | . | . | . | . | . | . | . |
| 21-225_9E5_LC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | P | P | F | T | F | G | P | G | T | K | V | D | I | K | R |
| 21-225_10F12_LC | . | . | . | T | . | F | . | . | . | . | . | . | . | . | . | . | . | . | . |
| 21-225_11A5_LC | . | . | . | T | . | L | . | . | . | . | . | . | . | . | . | . | . | . | . |
| 21-225_14A11_LC | . | . | . | S | . | . | . | . | . | A | . | . | . | . | . | . | . | . | . |
| 21-225_158F4_LC | . | . | . | . | I | S | . | . | . | . | . | . | . | . | . | . | . | . | . |
| 21-225_16H6_LC | . | . | . | T | . | L | . | . | . | . | . | . | . | . | . | . | F | . | . |
| 21-225_17A12_LC | . | . | . | T | . | L | . | . | . | . | . | . | . | . | . | . | . | . | . |
| 21-225_17B4_LC | . | . | . | T | . | F | . | . | . | . | . | . | . | . | . | . | . | . | . |
| 21-225_17C6_LC | . | . | . | T | . | F | . | . | . | . | . | . | . | . | . | . | . | . | . |
| 21-225_17F11_LC | . | . | . | T | . | L | . | A | . | . | . | . | . | . | . | . | . | . | . |
| 21-225_17F7_LC | . | . | . | T | . | F | . | . | . | . | . | . | . | . | . | . | . | . | . |
| 21-225_180F8_LC | . | . | . | . | T | . | . | . | . | . | . | . | . | . | . | . | . | . | . |
| 21-225_18E12_LC | . | . | . | T | . | F | . | . | . | . | . | . | . | . | . | . | . | . | . |
| 21-225_19A5_LC | . | . | . | . | T | . | . | . | . | . | . | . | . | . | . | . | . | . | . |
| 21-225_19B3_LC | . | . | . | . | T | . | . | . | . | . | . | . | . | . | . | . | . | . | . |
| 21-225_20A4_LC | . | . | . | T | . | L | . | . | . | . | . | . | . | . | . | . | F | . | . |
| 21-225_20F3_LC | . | . | . | A | . | . | . | . | . | . | . | . | . | . | . | . | . | . | . |
| 21-225_21A2_LC | . | . | . | T | . | F | . | . | . | . | . | . | . | . | . | . | . | . | . |
| 21-225_21A5_LC | . | . | . | . | I | S | . | . | . | . | . | . | . | . | . | . | . | . | . |
| 21-225_21F4_LC | . | . | . | T | . | F | . | . | . | . | . | . | . | . | . | . | F | . | . |
| 21-225_222H4_LC | . | . | . | . | T | . | . | . | . | . | R | . | . | . | . | . | . | . | . |
| 21-225_222H8_LC | . | . | . | . | T | . | . | . | . | . | R | . | . | . | . | . | . | . | . |
| 21-225_22C5_LC | . | . | . | T | . | L | . | . | . | . | . | . | . | . | . | . | F | . | . |
| 21-225_22D5_LC | . | . | . | T | . | L | . | . | . | . | . | . | . | . | . | . | . | . | . |
| 21-225_22E4_LC | . | . | . | T | . | L | . | . | . | . | . | . | . | . | . | . | . | . | . |
| 21-225_22G10_LC | . | . | . | T | . | L | . | . | . | . | . | . | . | . | . | . | F | . | . |
| 21-225_22G3_LC | . | . | . | T | . | F | . | . | . | . | . | . | . | . | . | . | . | . | . |
| 21-225_22G5_LC | . | . | . | T | . | L | . | . | . | . | . | . | . | . | . | . | F | . | . |
| 21-225_30E11_LC | . | . | . | . | T | . | . | . | . | . | . | . | . | . | . | . | . | . | . |
| 21-225_33G12_LC | . | . | . | . | T | . | . | . | . | . | . | . | . | . | . | . | . | . | . |

# FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | P | P | F | T | F | G | P | G | T | K | V | D | I | K | R |
| 21-225_3B1_LC | . | . | . | T | . | L | . | . | . | . | . | . | . | . | . | . | . | N | . |
| 21-225_3E3_LC | . | . | . | T | . | L | . | . | . | . | . | . | . | . | . | . | . | . | . |
| 21-225_48C3_LC | . | . | . | . | I | . | . | . | . | . | . | . | . | . | . | . | . | . | . |
| 21-225_48D1_LC | . | . | . | . | I | . | . | . | . | . | . | . | . | . | . | . | . | . | . |
| 21-225_49G12_LC | . | . | . | . | I | . | . | . | . | . | . | . | . | . | . | . | . | . | . |
| 21-225_4B1_LC | . | . | . | . | I | S | . | . | . | . | . | . | . | . | . | . | . | . | . |
| 21-225_4H2_LC | . | . | . | T | . | L | . | . | . | . | . | . | . | . | . | . | . | . | . |
| 21-225_50B12_LC | . | . | . | . | A | . | . | . | . | . | . | . | . | . | . | . | . | . | . |
| 21-225_50H10_LC | . | . | . | . | I | . | . | . | . | . | . | . | . | . | . | . | . | . | . |
| 21-225_50H8_LC | . | . | . | . | L | . | . | . | . | . | . | . | . | . | . | . | . | . | . |
| 21-225_51H10_LC | . | . | . | . | I | . | . | . | . | . | . | . | . | . | . | . | . | . | . |
| 21-225_53E2_LC | . | . | . | . | T | . | . | . | . | . | . | . | . | . | . | . | . | . | . |
| 21-225_56B2_LC | . | . | . | . | T | . | . | . | . | . | . | . | . | . | . | . | . | . | . |
| 21-225_57F2_LC | . | . | . | . | I | S | . | . | . | . | . | . | . | . | . | . | . | . | . |
| 21-225_5F7_LC | . | . | . | T | . | L | . | . | . | . | . | . | . | . | . | V | . | . | . |
| 21-225_5G2_LC | . | . | . | T | . | L | . | . | . | . | . | . | . | . | . | . | . | . | . |
| 21-225_60E12_LC | . | . | . | . | I | S | . | . | . | . | . | . | . | . | . | . | . | . | . |
| 21-225_61A1_LC | . | . | . | . | I | S | . | . | . | . | . | . | . | . | . | . | . | . | . |
| 21-225_62H1_LC | . | . | . | . | T | . | . | . | . | . | . | . | . | . | . | . | . | . | . |
| 21-225_62H10_LC | . | . | . | . | F | . | . | . | . | . | . | . | . | N | . | . | F | . | . |
| 21-225_62H8_LC | . | . | . | . | T | . | . | . | . | . | . | . | . | R | . | . | . | . | . |
| 21-225_66A9_LC | . | . | . | . | V | . | . | . | . | . | . | . | . | . | . | . | F | . | . |
| 21-225_69C8_LC | . | . | . | . | T | . | . | . | . | . | . | . | . | . | . | . | . | . | . |
| 21-225_6D3_LC | . | . | . | T | . | F | . | . | . | . | . | . | . | . | . | . | . | . | . |
| 21-225_72G9_LC | . | . | . | . | F | . | . | . | . | . | . | . | . | . | . | . | . | . | . |
| 21-225_8E12_LC | . | . | . | T | . | F | . | . | . | . | . | . | . | . | . | . | . | . | . |
| 21-225_8G6_LC | . | . | . | T | . | F | . | . | . | . | . | . | . | . | . | . | . | . | . |
| 21-225_8H11_LC | . | . | . | . | T | . | . | . | . | . | . | . | . | . | . | . | . | . | . |
| 21-225_9E5_LC | . | . | . | T | . | L | . | S | . | . | . | . | . | . | . | . | . | . | . |

**FIGURE 57**

Table 107. Consensus 27 - VK4|B3/JK2 (SEQ ID NO: 50324):

DIVMTQSPDSLAVSLGERATINCKSS--QSVLYSSNNNNYLAWYQQKPGQPPKLLIYW--------ASTRESGVPDRFSGSGSG--TDFTLTISSLQAEDVAVYYCQQYYS----------------------TPCSFGQGTKLEIKR wherein:

K at position 24 can be substituted with R or T

S at position 26 can be substituted with I

S at position 30 can be substituted with N

V at position 31 can be substituted with I

Y at position 33 can be substituted with H, S or F

S at position 34 can be substituted with N, I, R or H

N at position 36 can be substituted with H

N at position 37 can be substituted with S or D

N at position 38 can be substituted with Y, K, H, A, M or Q

N at position 39 can be substituted with K

Y at position 40 can be substituted with F

A at position 42 can be substituted with T or D

A at position 67 can be substituted with T, G or S

S at position 68 can be substituted with F

T at position 69 can be substituted with I

E at position 71 can be substituted with K or D

S at position 72 can be substituted with F

Q at position 107 can be substituted with H

**FIGURE 57**

Q at position 108 can be substituted with H

Y at position 109 can be substituted with S

Y at position 110 can be substituted with F, K, or N

S at position 111 can be substituted with T, I, null (-) or N

T at position 135 can be substituted with S, I, R, A, G or N

P at position 136 can be substituted with S

C at position 137 can be substituted with Y, G, L or P

S at position 138 can be substituted with K or N

Xaa Ser Xaa Gln Xaa Xaa Leu Xaa Xaa Ser Xaa Xaa Xaa Xaa Xaa Leu Xaa (SEQ ID NO: 50156)

Trp Xaa Xaa Xaa Arg Xaa Xaa (SEQ ID NO: 50157)

Xaa Xaa Xaa Xaa Xaa Xaa Xaa Xaa Xaa (SEQ ID NO: 50158)

Lys Ser Ser Gln Ser Val Leu Tyr Ser Ser Asn Asn Asn Asn Tyr Leu Ala (SEQ ID NO: 50642)

Trp Ala Ser Thr Arg Glu Ser (SEQ ID NO: 50643)

Gln Gln Tyr Tyr Ser Thr Pro Cys Ser (SEQ ID NO: 50644)

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | V | M | T | Q | S | P | D | S | L | A | V | S | L | G | E | R | A | T | I | N | C | K | S | S |
| 21-225_146C9_LC | . | . | . | . | . | . | . | . | . | . | . | . | . | . | . | . | . | . | . | . | . | . | . | . | . | . |
| 21-225_146D4_LC | . | . | . | . | . | . | . | . | . | . | . | . | . | . | . | . | . | . | . | . | . | . | . | . | . | . |
| 21-225_146F9_LC | . | . | . | . | . | . | . | . | . | . | . | . | . | . | . | . | . | . | . | . | . | . | . | . | . | . |
| 21-225_147D5_LC | . | . | . | . | . | . | . | . | . | . | . | . | . | . | . | . | . | . | . | . | . | . | . | . | . | . |
| 21-225_150D2_LC | . | . | . | . | . | . | . | . | . | . | . | . | . | . | . | . | . | . | . | . | . | K | . | . | . | . |
| 21-225_152D10_LC | G | . | . | . | . | . | . | . | . | . | . | . | . | . | . | . | A | . | . | . | . | D | . | . | . | . |
| 21-225_152E12_LC | A | V | . | . | . | . | . | . | . | . | . | . | . | . | . | . | . | . | . | . | . | . | . | T | . | . |
| 21-225_152H3_LC | . | . | . | . | . | . | . | . | . | . | . | . | . | . | . | . | . | K | . | . | . | . | . | . | . | . |
| 21-225_153B9_LC | G | . | . | . | . | . | F | . | . | . | . | . | . | . | . | . | . | . | . | . | . | . | . | . | . | . |
| 21-225_153F11_LC | . | . | . | . | . | . | . | . | . | . | . | . | . | . | . | . | . | . | . | . | . | . | . | . | . | . |
| 21-225_154H6_LC | . | . | . | . | . | . | . | . | . | . | . | . | . | . | . | . | . | . | . | . | . | . | . | . | . | . |
| 21-225_155B6_LC | . | . | . | . | . | . | . | . | . | . | . | . | . | . | . | . | . | . | . | . | . | . | . | . | . | . |
| 21-225_155E5_LC | . | . | . | . | . | . | . | . | . | . | . | . | . | . | . | . | . | . | . | . | . | . | . | . | . | . |
| 21-225_156H1_LC | . | . | . | . | . | . | . | . | . | . | . | . | . | . | . | . | . | . | . | . | . | . | . | . | . | . |
| 21-225_160A4_LC | . | . | . | . | . | . | . | . | . | . | . | . | . | . | . | . | . | . | . | . | . | . | . | . | . | . |
| 21-225_160A7_LC | . | . | . | L | . | . | . | . | . | . | P | . | . | . | . | . | . | . | . | . | . | . | . | . | . | . |
| 21-225_172E11_LC | . | . | . | . | . | . | . | . | . | . | . | . | . | . | . | . | . | . | . | . | . | . | . | . | . | . |
| 21-225_224F11_LC | N | . | . | . | . | . | . | . | . | . | . | . | . | . | . | D | . | . | . | . | . | . | . | . | . | . |
| 21-225_225B9_LC | . | . | . | . | . | . | . | . | . | . | . | . | . | . | . | . | . | . | . | . | . | . | . | . | . | . |
| 21-225_225F12_LC | . | . | . | . | . | . | . | . | . | . | . | . | . | . | . | . | . | . | . | . | . | . | . | . | . | . |
| 21-225_225H12_LC | . | . | . | . | . | . | . | . | . | . | . | . | . | . | . | . | . | . | . | . | . | . | . | . | . | . |
| 21-225_226D6_LC | . | . | . | . | . | . | . | . | . | . | . | . | . | . | . | . | . | . | . | . | . | . | . | R | . | . |
| 21-225_227D11_LC | . | . | . | . | . | . | . | . | . | . | . | . | . | . | . | D | . | . | . | . | . | . | . | . | . | . |
| 21-225_227E6_LC | . | . | . | . | . | . | . | . | . | . | . | T | . | . | . | . | . | . | . | . | . | . | . | . | . | . |
| 21-225_227G9_LC | . | . | . | . | . | . | . | . | . | . | . | . | . | . | . | . | . | . | . | . | . | . | . | . | . | . |
| 21-225_22D2_LC | . | . | . | . | . | . | . | . | . | . | . | . | . | . | . | . | . | . | . | . | . | T | . | . | . | . |
| 21-225_25E12_LC | . | . | . | L | . | . | . | . | . | F | . | . | M | . | . | . | . | . | . | . | . | . | . | . | . | . |
| 21-225_32A1_LC | . | . | . | . | . | . | . | . | . | . | . | . | . | . | . | . | . | . | . | . | . | . | . | . | . | . |
| 21-225_32A5_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | V | M | T | Q | S | P | D | S | L | A | V | S | L | G | E | R | A | T | I | N | C | K | S | S |
| 21-225_33D1_LC | . | . | . | . | . | . | . | . | . | . | . | S | . | . | . | . | . | . | . | . | . | . | . | . | . | I |
| 21-225_34H7_LC | . | . | . | . | . | . | . | . | . | . | . | . | . | . | . | . | . | . | . | . | . | . | . | . | . | . |
| 21-225_57F12_LC | . | . | . | . | . | . | . | . | . | . | . | . | . | . | . | . | . | . | . | . | . | . | . | . | . | . |
| 21-225_59E1_LC | . | . | . | . | . | . | . | . | . | . | . | . | . | . | . | . | . | . | . | . | . | . | . | . | . | . |
| 21-225_60F3_LC | . | . | . | . | . | . | . | . | . | . | . | T | . | . | . | . | . | . | . | . | . | . | . | . | . | . |
| 21-225_61E3_LC | . | . | . | . | . | . | . | . | . | . | . | . | . | . | . | . | . | . | . | . | . | . | . | . | . | . |
| 21-225_63F4_LC | . | . | . | . | . | . | . | . | . | . | . | . | . | . | . | . | . | . | . | . | . | . | . | . | . | . |
| 21-225_71F3_LC | . | . | . | . | . | . | . | . | . | . | . | . | . | . | . | . | A | . | . | . | . | . | . | . | . | . |
| 21-225_71G3_LC | . | . | . | . | . | . | . | . | . | . | . | . | . | . | . | . | . | . | V | A | . | . | . | . | . | . |
| 21-225_74A8_LC | . | . | . | . | . | . | . | . | . | . | . | . | . | . | . | . | . | . | . | . | . | . | . | . | . | . |
| 21-225_74C10_LC | . | . | . | . | . | . | . | . | . | . | . | . | . | . | . | . | . | . | . | . | . | . | . | . | . | . |
| 21-225_74C12_LC | . | . | . | . | . | . | . | . | . | . | . | . | . | . | . | . | . | . | . | . | . | . | . | . | . | . |
| 21-225_77D12_LC | . | . | . | . | . | . | . | . | . | . | . | . | . | . | . | . | A | . | . | . | . | . | . | R | . | . |
| 21-225_78E6_LC | . | . | . | . | . | . | . | . | . | . | . | . | . | . | . | . | . | . | . | . | . | . | . | . | . | . |
| 21-225_79G7_LC | . | . | . | . | . | . | . | . | . | . | . | . | . | . | . | . | . | . | . | . | . | . | . | . | . | . |
| 21-225_85C11_LC | . | . | . | . | . | . | . | . | . | . | . | . | . | . | . | . | . | . | . | . | . | . | . | . | . | . |
| 21-225_87E10_LC | . | . | . | . | . | . | . | . | . | . | . | . | . | . | . | . | . | . | . | . | . | K | . | . | . | . |
| 21-225_8B11_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | V | L | Y | S | S | N | N | N | N | Y | L | A | W | Y | Q | Q | K | P | G | Q | P | P |
| 21-225_146C9_LC | . | . | . | . | . | . | H | . | . | . | . | Y | . | . | . | . | . | . | . | . | . | . | . | . | . | . |
| 21-225_146D4_LC | . | . | . | . | . | . | . | . | . | . | . | . | . | . | . | . | . | . | . | . | . | . | . | . | . | . |
| 21-225_146F9_LC | . | . | . | N | I | . | H | . | . | . | . | . | . | . | . | . | . | . | . | . | . | . | . | . | . | . |
| 21-225_147D5_LC | . | . | . | . | . | . | . | . | . | . | . | . | . | . | . | . | . | . | . | . | . | . | . | . | . | . |
| 21-225_150D2_LC | . | . | . | . | . | . | . | R | . | . | S | K | K | . | . | T | . | . | . | . | . | . | . | . | . | . |
| 21-225_152D10_LC | . | . | . | . | . | . | . | . | . | . | . | K | . | . | . | . | . | . | . | . | . | . | . | . | . | . |
| 21-225_152E12_LC | . | . | . | . | I | . | H | . | . | . | . | Y | . | . | . | . | . | . | . | . | . | . | . | . | . | . |
| 21-225_152H3_LC | . | . | . | . | . | . | H | . | . | . | . | Y | . | . | . | T | . | . | . | . | . | . | . | . | . | . |
| 21-225_153B9_LC | . | . | . | . | . | . | . | . | . | . | . | K | . | . | . | . | . | . | . | . | . | . | . | . | . | H |
| 21-225_153F11_LC | . | . | . | . | . | . | . | . | . | . | . | Y | K | . | . | . | . | . | . | . | . | . | . | . | . | . |
| 21-225_154H6_LC | . | . | . | . | . | . | H | . | . | . | . | Y | . | . | . | . | . | . | . | . | . | . | . | . | . | . |
| 21-225_155B6_LC | . | . | . | . | . | . | H | . | . | . | . | Y | . | . | . | . | . | . | . | . | . | . | . | . | . | . |
| 21-225_155E5_LC | . | . | . | . | . | . | S | . | . | . | . | . | . | . | . | . | . | . | . | . | . | . | . | . | . | . |
| 21-225_156H1_LC | . | . | . | . | . | . | . | . | . | . | . | . | . | . | . | . | . | . | . | . | . | . | . | . | . | . |
| 21-225_160A4_LC | . | . | . | . | . | . | H | . | . | . | . | . | . | . | . | . | . | . | . | . | . | . | . | . | . | . |
| 21-225_160A7_LC | . | . | . | . | . | . | S | N | . | H | . | . | . | . | . | . | . | . | . | . | R | . | . | H | . | H |
| 21-225_172E11_LC | . | . | . | . | . | . | H | . | . | . | . | . | . | . | . | . | . | . | . | . | . | . | . | . | . | . |
| 21-225_224F11_LC | . | . | . | . | . | . | S | . | . | . | . | H | . | . | . | . | . | . | . | . | . | . | . | . | . | . |
| 21-225_225B9_LC | . | . | . | . | . | . | . | . | . | . | . | . | . | . | . | . | . | . | . | . | . | . | . | . | . | . |
| 21-225_225F12_LC | . | . | . | . | . | . | . | . | . | . | . | Q | . | . | . | . | . | . | . | . | . | . | . | . | . | . |
| 21-225_225H12_LC | . | . | . | . | . | . | . | N | . | . | . | . | . | . | . | . | . | . | . | . | . | . | . | . | . | . |
| 21-225_226D6_LC | . | . | . | . | I | . | . | I | . | . | . | K | . | . | . | . | . | . | . | . | . | . | . | . | . | . |
| 21-225_227D11_LC | . | . | . | . | . | . | H | . | . | . | . | . | . | . | . | . | . | . | . | . | . | . | . | . | . | . |
| 21-225_227E6_LC | . | . | . | . | . | . | . | . | . | . | D | K | . | . | . | . | . | . | . | . | . | . | . | . | . | . |
| 21-225_227G9_LC | . | . | . | . | . | . | H | . | . | . | . | . | . | . | . | . | . | . | . | . | . | . | . | . | . | . |
| 21-225_22D2_LC | . | . | . | . | . | . | H | . | . | . | . | . | . | . | . | . | . | . | . | . | . | . | . | . | . | . |
| 21-225_25E12_LC | . | . | . | . | . | . | . | H | . | . | . | Y | . | . | . | . | . | . | . | . | . | . | K | . | . | . |
| 21-225_32A1_LC | . | . | . | . | . | . | . | . | . | . | . | Y | . | . | . | . | . | . | . | L | . | . | . | . | . | . |
| 21-225_32A5_LC | . | . | . | . | . | . | H | . | . | . | . | K | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | V | L | Y | S | S | N | N | N | N | Y | L | A | W | Y | Q | Q | K | P | G | Q | P | P |
| 21-225_33D1_LC | . | . | . | . | . | . | . | R | . | . | . | K | . | . | . | T | . | . | . | . | . | . | . | . | . | R |
| 21-225_34H7_LC | . | . | . | . | . | . | . | . | . | . | . | Y | . | . | . | . | . | . | . | L | . | . | . | . | . | . |
| 21-225_57F12_LC | . | . | . | . | . | . | F | . | . | . | . | Y | . | . | . | . | . | . | . | . | . | T | . | . | . | . |
| 21-225_59E1_LC | . | . | . | . | . | . | . | . | . | . | . | . | . | . | . | . | . | . | . | . | R | . | . | . | . | . |
| 21-225_60F3_LC | . | . | . | . | . | . | . | . | . | . | . | K | . | . | . | . | . | . | . | . | . | . | . | . | . | . |
| 21-225_61E3_LC | . | . | . | . | . | . | H | I | . | . | . | . | . | . | . | . | . | . | . | . | Q | . | . | . | . | . |
| 21-225_63F4_LC | . | . | . | . | . | . | S | . | . | . | . | . | . | . | . | . | . | . | . | . | . | . | . | . | . | . |
| 21-225_71F3_LC | . | . | . | . | . | . | S | N | . | . | . | . | . | . | . | . | . | . | . | . | . | . | . | . | . | . |
| 21-225_71G3_LC | . | . | . | . | . | . | H | . | . | . | . | . | . | . | . | D | . | . | . | . | . | . | . | . | L | . |
| 21-225_74A8_LC | . | . | . | . | . | . | . | . | . | . | S | H | . | . | . | . | . | . | . | . | . | . | . | . | . | . |
| 21-225_74C10_LC | . | . | . | . | . | . | . | . | . | . | . | A | . | . | . | . | . | . | . | . | . | . | . | . | . | . |
| 21-225_74C12_LC | . | . | . | . | . | . | . | . | . | . | . | A | . | . | . | . | . | . | . | . | . | . | . | . | . | . |
| 21-225_77D12_LC | . | . | . | . | . | . | . | . | . | . | . | Y | . | . | . | . | . | . | . | . | . | . | . | . | . | . |
| 21-225_78E6_LC | . | . | . | . | . | . | . | . | . | . | S | H | . | . | . | . | . | . | . | . | . | . | . | . | . | . |
| 21-225_79G7_LC | . | . | . | . | . | . | . | . | . | . | S | H | . | . | . | . | . | . | . | . | . | . | . | . | . | . |
| 21-225_85C11_LC | . | . | . | . | . | . | . | . | . | . | S | H | . | F | . | . | . | . | . | . | N | . | . | . | . | . |
| 21-225_87E10_LC | . | . | . | . | . | . | H | . | . | . | . | M | . | . | . | . | . | . | . | . | . | . | . | . | . | . |
| 21-225_8B11_LC | . | . | . | N | . | . | . | N | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | Y | W | - | - | - | - | - | - | - | - | A | S | T | R | E | S | G | V | P | D | R | F |
| 21-225_146C9_LC | . | V | . | . | . | . | . | . | . | . | . | . | . | . | . | . | . | . | K | . | . | . | . | . | . | . |
| 21-225_146D4_LC | . | . | V | . | . | . | . | . | . | . | . | . | . | . | . | . | . | . | . | . | . | . | . | . | . | . |
| 21-225_146F9_LC | Y | . | . | . | . | . | . | . | . | . | . | . | . | . | . | . | . | . | . | . | . | . | . | . | . | . |
| 21-225_147D5_LC | . | . | . | . | . | . | . | . | . | . | . | . | . | . | . | . | . | . | . | . | . | . | . | . | . | . |
| 21-225_150D2_LC | . | . | F | . | . | . | . | . | . | . | . | . | . | . | . | . | . | . | K | . | . | . | . | . | . | . |
| 21-225_152D10_LC | . | . | . | . | . | . | . | . | . | . | . | . | . | . | . | . | . | . | . | . | . | . | . | . | . | . |
| 21-225_152E12_LC | . | . | . | . | . | . | . | . | . | . | . | . | . | . | . | . | . | . | . | . | . | . | . | . | . | . |
| 21-225_152H3_LC | . | . | . | . | . | . | . | . | . | . | . | . | . | . | . | . | . | . | K | . | . | . | . | . | . | . |
| 21-225_153B9_LC | . | . | . | . | . | . | . | . | . | . | . | . | . | . | . | . | . | . | . | F | . | . | . | . | . | . |
| 21-225_153F11_LC | N | . | . | . | . | . | . | . | . | . | . | . | . | . | . | . | . | . | K | . | . | . | . | . | . | . |
| 21-225_154H6_LC | . | . | . | . | . | . | . | . | . | . | . | . | . | . | T | . | . | . | K | . | . | . | . | . | . | . |
| 21-225_155B6_LC | . | . | . | . | . | . | . | . | . | . | . | . | . | . | T | . | . | . | . | . | . | . | . | . | . | . |
| 21-225_155E5_LC | . | . | . | . | . | . | . | . | . | . | . | . | . | . | . | . | . | . | K | . | . | . | . | . | . | . |
| 21-225_156H1_LC | . | . | . | . | . | . | . | . | . | . | . | . | . | . | . | . | . | . | . | . | . | . | . | A | . | . |
| 21-225_160A4_LC | . | . | . | . | . | . | . | . | . | . | . | . | . | . | . | . | . | . | . | . | . | . | . | . | . | . |
| 21-225_160A7_LC | . | . | . | . | F | . | . | . | . | . | . | . | . | . | . | . | . | . | . | . | . | . | . | . | . | . |
| 21-225_172E11_LC | . | . | . | . | . | . | . | . | . | . | . | . | . | . | . | . | . | . | . | . | . | . | . | . | . | . |
| 21-225_224F11_LC | . | M | . | . | . | . | . | . | . | . | . | . | . | . | . | . | . | . | . | . | . | . | . | . | . | . |
| 21-225_225B9_LC | . | . | . | . | F | . | . | . | . | . | . | . | . | . | . | . | . | . | . | . | . | . | . | . | . | . |
| 21-225_225F12_LC | R | . | . | . | . | . | . | . | . | . | . | . | . | . | T | . | . | . | . | . | . | . | . | . | . | . |
| 21-225_225H12_LC | N | . | . | . | . | . | . | . | . | . | . | . | . | . | . | . | . | . | . | . | . | . | . | . | . | . |
| 21-225_226D6_LC | . | M | . | . | . | . | . | . | . | . | . | . | . | . | . | . | . | . | . | . | . | . | . | . | . | . |
| 21-225_227D11_LC | . | . | . | . | . | . | . | . | . | . | . | . | . | . | . | . | . | . | . | . | . | I | . | . | . | . |
| 21-225_227E6_LC | . | M | . | . | . | . | . | . | . | . | . | . | . | . | . | . | . | . | . | . | . | . | . | . | . | . |
| 21-225_227G9_LC | . | . | . | . | . | . | . | . | . | . | . | . | . | . | G | . | . | . | . | . | . | . | . | . | . | . |
| 21-225_22D2_LC | . | . | . | . | . | . | . | . | . | . | . | . | . | . | . | . | . | . | K | . | . | . | . | . | . | . |
| 21-225_25E12_LC | . | . | . | . | . | . | . | . | . | . | . | . | . | . | . | . | . | . | . | . | . | . | . | . | . | . |
| 21-225_32A1_LC | . | . | . | . | . | . | . | . | . | . | . | . | . | . | . | . | . | . | K | . | . | . | . | . | . | . |
| 21-225_32A5_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | L | L | I | Y | W | - | - | - | - | - | - | - | - | A | S | T | R | E | S | G | V | P | D | R | F |
| 21-225_33D1_LC | . | . | F | . | . | . | . | . | . | . | . | . | . | . | . | . | . | . | . | . | . | . | L | . | . | . |
| 21-225_34H7_LC | . | . | . | . | . | . | . | . | . | . | . | . | . | . | . | . | . | . | K | . | . | . | . | . | . | . |
| 21-225_57F12_LC | . | . | I | . | . | . | . | . | . | . | . | . | . | . | . | . | . | . | . | . | . | . | . | . | . | . |
| 21-225_59E1_LC | . | . | . | . | . | . | . | . | . | . | . | . | . | . | S | . | . | . | . | . | . | . | . | . | . | . |
| 21-225_60F3_LC | . | . | . | . | . | . | . | . | . | . | . | . | . | . | . | . | . | . | . | . | . | . | . | . | . | . |
| 21-225_61E3_LC | . | . | . | . | . | . | . | . | . | . | . | . | . | . | . | . | I | . | . | . | . | . | . | . | . | . |
| 21-225_63F4_LC | . | . | . | . | . | . | . | . | . | . | . | . | . | . | . | . | . | . | . | . | . | I | . | . | . | . |
| 21-225_71F3_LC | . | . | . | . | . | . | . | . | . | . | . | . | . | . | . | . | . | . | . | . | . | I | . | . | . | . |
| 21-225_71G3_LC | . | . | . | . | F | . | . | . | . | . | . | . | . | . | . | . | . | . | . | F | . | . | . | . | . | . |
| 21-225_74A8_LC | . | . | . | . | . | . | . | . | . | . | . | . | . | . | . | . | . | . | . | . | . | . | . | . | . | . |
| 21-225_74C10_LC | N | . | . | . | . | . | . | . | . | . | . | . | . | . | . | . | . | . | . | . | . | . | . | . | . | . |
| 21-225_74C12_LC | N | . | . | . | . | . | . | . | . | . | . | . | . | . | . | . | . | . | . | . | . | . | . | . | . | . |
| 21-225_77D12_LC | . | . | . | . | . | . | . | . | . | . | . | . | . | . | . | . | . | . | D | . | . | . | . | . | . | . |
| 21-225_78E6_LC | . | . | . | . | . | . | . | . | . | . | . | . | . | . | . | . | I | . | . | . | . | . | . | . | . | . |
| 21-225_79G7_LC | . | . | . | . | F | . | . | . | . | . | . | . | . | . | . | . | I | . | . | . | . | . | . | . | . | . |
| 21-225_85C11_LC | . | . | . | . | F | . | . | . | . | . | . | . | . | . | . | F | I | . | . | . | . | . | . | E | G | . |
| 21-225_87E10_LC | . | . | . | . | . | . | . | . | . | . | . | . | . | . | . | . | . | . | K | . | . | . | . | . | . | . |
| 21-225_8B11_LC | . | . | . | . | . | . | . | . | . | . | . | . | . | . | . | . | . | . | K | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | A | E | D | V | A | V | Y |
| 21-225_146C9_LC | . | . | . | . | . | . | . | . | . | . | . | . | . | . | . | . | . | . | . | . | . | . | . | . | . | . |
| 21-225_146D4_LC | . | . | . | . | . | . | . | . | . | . | . | . | . | . | . | . | . | . | . | . | . | . | . | . | . | . |
| 21-225_146F9_LC | . | . | . | . | . | . | . | . | . | . | . | . | . | . | . | T | . | . | . | . | . | . | . | . | . | . |
| 21-225_147D5_LC | . | . | . | . | . | . | . | . | . | . | . | . | . | S | . | . | . | . | . | . | . | . | . | . | . | . |
| 21-225_150D2_LC | . | . | . | . | . | . | . | . | . | . | . | . | . | . | . | . | N | . | . | . | . | . | . | . | . | . |
| 21-225_152D10_LC | . | . | . | . | . | . | . | . | . | . | . | . | . | . | . | Y | . | . | . | . | . | . | . | . | . | . |
| 21-225_152E12_LC | . | . | . | . | . | . | . | . | . | . | . | . | . | . | . | . | . | . | . | . | . | . | . | . | . | . |
| 21-225_152H3_LC | . | . | . | . | . | . | . | . | . | . | . | . | . | . | . | . | . | . | . | . | . | . | . | . | . | . |
| 21-225_153B9_LC | . | . | . | . | C | . | . | . | . | . | . | . | . | . | . | Y | . | V | . | . | . | . | . | . | . | . |
| 21-225_153F11_LC | . | . | . | . | . | . | . | . | . | . | . | . | . | . | . | . | . | . | . | . | . | . | . | . | . | . |
| 21-225_154H6_LC | . | . | . | . | . | . | . | . | . | H | . | . | . | S | . | . | . | . | . | . | . | . | . | . | . | . |
| 21-225_155B6_LC | . | . | . | . | . | . | . | . | . | . | . | . | . | . | . | . | . | . | . | . | . | . | . | . | . | . |
| 21-225_155E5_LC | . | . | . | . | . | . | . | . | . | . | . | . | . | . | . | . | . | . | . | . | . | . | . | . | . | . |
| 21-225_156H1_LC | . | . | . | . | . | . | . | . | . | . | . | . | . | . | . | . | . | . | . | . | . | . | . | . | . | . |
| 21-225_160A4_LC | . | . | . | . | . | . | . | . | . | . | . | . | . | . | . | . | . | . | . | . | . | . | . | . | . | . |
| 21-225_160A7_LC | . | . | . | . | Y | . | . | . | . | . | . | . | . | . | . | . | . | . | . | . | . | . | . | . | . | . |
| 21-225_172E11_LC | . | . | . | . | . | . | . | . | . | . | . | I | . | . | . | . | G | . | . | . | . | . | . | . | . | . |
| 21-225_224F11_LC | . | . | . | . | . | . | . | . | . | . | . | . | . | . | . | . | . | . | . | . | . | . | . | . | . | . |
| 21-225_225B9_LC | . | . | . | . | . | . | . | . | . | . | . | . | . | . | . | . | . | . | . | . | . | . | . | . | . | . |
| 21-225_225F12_LC | . | . | . | . | . | . | . | . | . | . | . | . | . | . | . | . | N | . | . | . | . | . | . | . | . | . |
| 21-225_225H12_LC | . | . | . | . | . | . | . | . | . | . | . | . | . | . | . | . | . | . | . | T | . | . | . | . | . | . |
| 21-225_226D6_LC | . | . | . | . | . | . | . | . | . | . | . | . | . | . | . | . | . | . | . | . | . | . | . | . | . | . |
| 21-225_227D11_LC | . | . | . | . | . | . | . | . | . | . | . | . | . | . | . | . | . | . | . | . | . | . | . | . | . | . |
| 21-225_227E6_LC | . | . | . | . | . | . | . | . | . | . | . | . | . | . | . | . | . | . | . | . | . | . | . | . | . | . |
| 21-225_227G9_LC | . | . | . | . | . | . | . | . | . | . | . | . | . | . | . | . | . | . | . | . | . | . | . | . | . | . |
| 21-225_22D2_LC | . | . | . | . | . | . | . | . | . | . | . | . | . | . | . | . | . | . | . | . | . | . | . | . | . | . |
| 21-225_25E12_LC | . | . | . | . | . | . | . | . | . | Y | . | . | . | . | . | . | . | . | . | . | . | . | . | . | . | . |
| 21-225_32A1_LC | . | . | . | . | . | . | . | . | . | . | . | . | . | . | . | . | . | . | . | T | . | . | . | . | L | . |
| 21-225_32A5_LC | . | . | . | . | . | . | . | . | . | . | . | . | . | A | . | . | . | . | . | . | . | . | . | . | L | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | A | E | D | V | A | V | Y |
| 21-225_33D1_LC | . | . | . | . | C | . | . | . | . | . | . | . | . | . | . | I | . | . | . | . | . | . | . | . | L | . |
| 21-225_34H7_LC | . | . | . | . | . | . | . | . | . | . | . | . | . | . | . | . | . | . | . | T | . | . | . | . | L | . |
| 21-225_57F12_LC | . | . | . | . | . | . | . | . | . | . | . | . | . | . | . | . | . | . | . | . | . | . | . | . | I | . |
| 21-225_59E1_LC | . | . | . | . | . | . | . | . | . | . | . | . | . | . | . | . | . | . | . | . | . | . | . | . | . | . |
| 21-225_60F3_LC | . | . | . | . | . | . | . | . | . | . | . | . | . | . | . | . | . | . | . | . | . | . | . | . | I | . |
| 21-225_61E3_LC | . | . | . | . | . | . | . | . | . | . | . | . | . | . | . | . | . | . | . | . | . | . | . | . | . | . |
| 21-225_63F4_LC | . | . | . | . | . | . | . | . | . | G | . | . | . | . | . | . | . | . | . | . | . | . | . | . | . | . |
| 21-225_71F3_LC | . | . | . | . | . | . | . | . | . | . | . | . | . | . | . | N | . | . | . | . | . | . | . | . | . | . |
| 21-225_71G3_LC | . | . | . | . | F | . | . | . | . | . | . | . | . | . | . | . | . | . | . | . | . | . | . | . | D | . |
| 21-225_74A8_LC | . | . | . | . | . | . | . | . | . | . | . | . | . | . | . | . | . | . | . | . | . | . | . | . | . | . |
| 21-225_74C10_LC | . | . | . | . | . | . | . | . | . | . | . | . | . | . | . | . | . | . | . | . | . | . | . | . | . | . |
| 21-225_74C12_LC | . | . | . | . | . | . | . | . | . | . | . | . | . | . | . | . | . | . | . | . | . | . | . | . | . | . |
| 21-225_77D12_LC | . | . | . | . | . | . | . | . | . | . | . | . | . | . | . | . | . | . | . | . | . | . | . | . | . | . |
| 21-225_78E6_LC | . | . | . | . | . | . | . | . | . | . | . | . | . | S | . | . | . | . | . | . | . | . | . | . | . | . |
| 21-225_79G7_LC | . | . | . | . | . | . | . | . | . | . | . | . | . | S | . | . | . | M | . | . | . | . | . | . | . | . |
| 21-225_85C11_LC | . | . | . | . | . | . | . | . | A | . | . | . | . | S | . | . | G | . | . | . | . | . | . | . | . | . |
| 21-225_87E10_LC | . | . | T | . | . | . | . | . | . | . | . | . | . | . | . | . | . | . | . | . | . | . | . | . | . | . |
| 21-225_8B11_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | Y | S | - | - | - | - | - | - | - | - | - | - | - | - | - | - | - | - | - | - | - |
| 21-225_146C9_LC | . | . | . | . | . | . | . | . | . | . | . | . | . | . | . | . | . | . | . | . | . | . | . | . | . | . |
| 21-225_146D4_LC | . | . | . | . | . | . | . | . | . | . | . | . | . | . | . | . | . | . | . | . | . | . | . | . | . | . |
| 21-225_146F9_LC | . | . | . | . | . | . | T | . | . | . | . | . | . | . | . | . | . | . | . | . | . | . | . | . | . | . |
| 21-225_147D5_LC | . | . | H | . | . | . | . | . | . | . | . | . | . | . | . | . | . | . | . | . | . | . | . | . | . | . |
| 21-225_150D2_LC | F | . | . | . | . | F | . | . | . | . | . | . | . | . | . | . | . | . | . | . | . | . | . | . | . | . |
| 21-225_152D10_LC | . | . | . | . | . | . | - | . | . | . | . | . | . | . | . | . | . | . | . | . | . | . | . | . | . | . |
| 21-225_152E12_LC | . | . | . | . | . | . | . | . | . | . | . | . | . | . | . | . | . | . | . | . | . | . | . | . | . | . |
| 21-225_152H3_LC | . | . | . | . | . | . | . | . | . | . | . | . | . | . | . | . | . | . | . | . | . | . | . | . | . | . |
| 21-225_153B9_LC | . | . | . | . | . | N | - | . | . | . | . | . | . | . | . | . | . | . | . | . | . | . | . | . | . | . |
| 21-225_153F11_LC | . | . | . | . | . | . | . | . | . | . | . | . | . | . | . | . | . | . | . | . | . | . | . | . | . | . |
| 21-225_154H6_LC | . | . | . | H | . | . | . | . | . | . | . | . | . | . | . | . | . | . | . | . | . | . | . | . | . | . |
| 21-225_155B6_LC | . | . | . | . | . | . | . | . | . | . | . | . | . | . | . | . | . | . | . | . | . | . | . | . | . | . |
| 21-225_155E5_LC | . | . | . | . | . | . | . | . | . | . | . | . | . | . | . | . | . | . | . | . | . | . | . | . | . | . |
| 21-225_156H1_LC | . | . | H | . | . | . | . | . | . | . | . | . | . | . | . | . | . | . | . | . | . | . | . | . | . | . |
| 21-225_160A4_LC | . | . | . | . | . | . | N | . | . | . | . | . | . | . | . | . | . | . | . | . | . | . | . | . | . | . |
| 21-225_160A7_LC | . | . | . | . | . | . | . | . | . | . | . | . | . | . | . | . | . | . | . | . | . | . | . | . | . | . |
| 21-225_172E11_LC | . | . | . | . | . | F | T | . | . | . | . | . | . | . | . | . | . | . | . | . | . | . | . | . | . | . |
| 21-225_224F11_LC | . | . | . | . | . | . | T | . | . | . | . | . | . | . | . | . | . | . | . | . | . | . | . | . | . | . |
| 21-225_225B9_LC | . | . | . | H | S | K | . | . | . | . | . | . | . | . | . | . | . | . | . | . | . | . | . | . | . | . |
| 21-225_225F12_LC | . | . | . | . | . | . | I | . | . | . | . | . | . | . | . | . | . | . | . | . | . | . | . | . | . | . |
| 21-225_225H12_LC | . | . | . | . | . | . | I | . | . | . | . | . | . | . | . | . | . | . | . | . | . | . | . | . | . | . |
| 21-225_226D6_LC | . | . | . | . | . | . | . | . | . | . | . | . | . | . | . | . | . | . | . | . | . | . | . | . | . | . |
| 21-225_227D11_LC | . | . | . | . | . | . | N | . | . | . | . | . | . | . | . | . | . | . | . | . | . | . | . | . | . | . |
| 21-225_227E6_LC | . | . | . | . | . | . | I | . | . | . | . | . | . | . | . | . | . | . | . | . | . | . | . | . | . | . |
| 21-225_227G9_LC | . | . | . | . | . | . | . | . | . | . | . | . | . | . | . | . | . | . | . | . | . | . | . | . | . | . |
| 21-225_22D2_LC | F | . | . | . | . | . | . | . | . | . | . | . | . | . | . | . | . | . | . | . | . | . | . | . | . | . |
| 21-225_25E12_LC | . | . | . | . | . | . | . | . | . | . | . | . | . | . | . | . | . | . | . | . | . | . | . | . | . | . |
| 21-225_32A1_LC | . | . | . | . | . | . | T | . | . | . | . | . | . | . | . | . | . | . | . | . | . | . | . | . | . | . |
| 21-225_32A5_LC | H | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | Y | S | - | - | - | - | - | - | - | - | - | - | - | - | - | - | - | - | - | - | - |
| 21-225_33D1_LC | . | . | . | . | . | . | . | . | . | . | . | . | . | . | . | . | . | . | . | . | . | . | . | . | . | . |
| 21-225_34H7_LC | . | . | . | . | . | . | T | . | . | . | . | . | . | . | . | . | . | . | . | . | . | . | . | . | . | . |
| 21-225_57F12_LC | . | . | . | . | . | . | . | . | . | . | . | . | . | . | . | . | . | . | . | . | . | . | . | . | . | . |
| 21-225_59E1_LC | . | . | . | . | . | F | . | . | . | . | . | . | . | . | . | . | . | . | . | . | . | . | . | . | . | . |
| 21-225_60F3_LC | . | . | . | . | . | . | . | . | . | . | . | . | . | . | . | . | . | . | . | . | . | . | . | . | . | . |
| 21-225_61E3_LC | . | . | . | . | . | . | . | . | . | . | . | . | . | . | . | . | . | . | . | . | . | . | . | . | . | . |
| 21-225_63F4_LC | . | . | . | . | . | . | T | . | . | . | . | . | . | . | . | . | . | . | . | . | . | . | . | . | . | . |
| 21-225_71F3_LC | . | . | . | . | . | . | T | . | . | . | . | . | . | . | . | . | . | . | . | . | . | . | . | . | . | . |
| 21-225_71G3_LC | . | . | . | . | . | . | I | . | . | . | . | . | . | . | . | . | . | . | . | . | . | . | . | . | . | . |
| 21-225_74A8_LC | . | . | . | . | . | . | . | . | . | . | . | . | . | . | . | . | . | . | . | . | . | . | . | . | . | . |
| 21-225_74C10_LC | . | . | . | . | . | . | . | . | . | . | . | . | . | . | . | . | . | . | . | . | . | . | . | . | . | . |
| 21-225_74C12_LC | . | . | . | . | . | . | . | . | . | . | . | . | . | . | . | . | . | . | . | . | . | . | . | . | . | . |
| 21-225_77D12_LC | . | . | . | . | . | . | T | . | . | . | . | . | . | . | . | . | . | . | . | . | . | . | . | . | . | . |
| 21-225_78E6_LC | . | . | . | . | . | . | . | . | . | . | . | . | . | . | . | . | . | . | . | . | . | . | . | . | . | . |
| 21-225_79G7_LC | . | . | . | . | . | . | . | . | . | . | . | . | . | . | . | . | . | . | . | . | . | . | . | . | . | . |
| 21-225_85C11_LC | . | . | . | . | . | . | . | . | . | . | . | . | . | . | . | . | . | . | . | . | . | . | . | . | . | . |
| 21-225_87E10_LC | . | . | . | . | . | . | . | . | . | . | . | . | . | . | . | . | . | . | . | . | . | . | . | . | . | . |
| 21-225_8B11_LC | . | . | . | . | . | . | T | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | T | P | C | S | F | G | Q | G | T | K | L | E | I | K | R |
| 21-225_146C9_LC | . | . | . | . | . | . | . | . | . | . | . | . | . | . | . | . | . | . | . |
| 21-225_146D4_LC | . | . | . | . | . | . | . | . | . | . | . | . | . | . | . | . | . | . | . |
| 21-225_146F9_LC | . | . | . | . | . | . | . | . | . | . | . | . | . | . | . | . | . | T | . |
| 21-225_147D5_LC | . | . | . | . | . | . | . | . | . | . | . | . | . | . | . | . | . | . | . |
| 21-225_150D2_LC | . | . | . | . | . | . | Y | N | . | . | . | . | . | . | R | . | . | . | . |
| 21-225_152D10_LC | . | . | . | . | R | S | P | . | . | . | . | . | . | . | . | . | . | . | . |
| 21-225_152E12_LC | . | . | . | . | G | . | . | . | . | . | . | . | . | . | . | . | . | . | . |
| 21-225_152H3_LC | . | . | . | . | . | . | . | . | . | . | . | . | . | . | . | . | . | . | . |
| 21-225_153B9_LC | . | . | . | . | R | S | L | . | . | . | . | . | . | . | . | . | . | . | . |
| 21-225_153F11_LC | . | . | . | . | . | . | . | . | . | . | . | . | . | . | . | . | . | . | . |
| 21-225_154H6_LC | . | . | . | . | . | . | . | . | . | . | . | . | . | . | . | . | . | . | . |
| 21-225_155B6_LC | . | . | . | . | . | . | . | . | . | . | . | . | . | . | . | . | . | . | . |
| 21-225_155E5_LC | . | . | . | . | . | . | . | . | . | . | . | . | . | . | . | . | . | . | . |
| 21-225_156H1_LC | . | . | . | . | . | . | . | . | . | . | . | . | . | . | . | . | . | . | . |
| 21-225_160A4_LC | . | . | . | . | S | . | . | . | . | . | . | . | . | . | . | . | . | . | . |
| 21-225_160A7_LC | . | . | . | . | . | . | . | . | . | . | . | . | . | . | . | . | . | . | . |
| 21-225_172E11_LC | . | . | . | . | . | . | . | . | . | . | . | . | . | . | . | . | . | . | . |
| 21-225_224F11_LC | . | . | . | . | . | . | . | . | . | . | . | . | . | . | . | . | . | . | . |
| 21-225_225B9_LC | . | . | . | . | I | . | G | K | . | . | . | . | I | . | . | . | . | Q | G |
| 21-225_225F12_LC | . | . | . | . | . | . | . | . | . | . | . | . | . | . | . | . | . | . | . |
| 21-225_225H12_LC | . | . | . | . | . | . | . | . | . | . | . | . | . | . | . | . | . | . | . |
| 21-225_226D6_LC | . | . | . | . | S | . | . | . | . | . | . | . | . | . | . | . | . | . | . |
| 21-225_227D11_LC | . | . | . | . | . | . | . | . | . | . | . | . | . | . | . | . | . | . | . |
| 21-225_227E6_LC | . | . | . | . | . | . | . | . | . | . | . | . | . | . | . | . | . | . | . |
| 21-225_227G9_LC | . | . | . | . | A | . | Y | . | . | . | . | . | . | . | . | . | . | . | . |
| 21-225_22D2_LC | . | . | . | . | . | . | . | . | . | . | . | . | . | . | . | . | . | . | . |
| 21-225_25E12_LC | . | . | . | . | . | . | . | . | . | . | . | . | . | N | . | . | . | . | . |
| 21-225_32A1_LC | . | . | . | . | S | . | . | . | . | . | . | . | . | . | . | . | . | . | . |
| 21-225_32A5_LC | . | . | . | . | S | . | . | . | . | . | . | . | . | G | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | T | P | C | S | F | G | Q | G | T | K | L | E | I | K | R |
| 21-225_33D1_LC | . | . | . | . | S | . | . | . | . | . | . | . | . | . | . | . | . | . | . |
| 21-225_34H7_LC | . | . | . | . | S | . | . | . | . | . | . | . | . | . | . | . | . | . | . |
| 21-225_57F12_LC | . | . | . | . | N | . | . | . | . | . | . | . | . | . | . | . | . | . | . |
| 21-225_59E1_LC | . | . | . | . | I | . | . | . | . | . | . | . | . | . | . | . | . | . | . |
| 21-225_60F3_LC | . | . | . | . | . | . | . | . | . | . | . | . | . | . | . | . | . | N | . |
| 21-225_61E3_LC | . | . | . | . | I | . | . | . | . | . | . | . | . | . | . | . | . | . | . |
| 21-225_63F4_LC | . | . | . | . | . | . | . | . | . | . | . | . | . | . | . | . | . | N | . |
| 21-225_71F3_LC | . | . | . | . | . | . | . | . | . | . | . | . | . | . | . | . | . | . | . |
| 21-225_71G3_LC | . | . | . | . | . | . | . | . | . | . | . | . | . | . | . | . | . | . | . |
| 21-225_74A8_LC | . | . | . | . | . | . | . | . | . | . | . | . | . | . | . | . | . | . | . |
| 21-225_74C10_LC | . | . | . | . | . | . | . | . | . | . | . | . | . | . | . | . | . | . | . |
| 21-225_74C12_LC | . | . | . | . | . | . | . | . | . | . | . | . | . | . | . | . | . | . | . |
| 21-225_77D12_LC | . | . | . | . | . | . | . | . | . | . | . | . | . | . | . | . | . | . | . |
| 21-225_78E6_LC | . | . | . | . | . | . | . | . | . | . | . | . | . | . | . | . | . | . | . |
| 21-225_79G7_LC | . | . | . | . | . | . | . | . | . | . | . | . | . | . | . | . | . | . | . |
| 21-225_85C11_LC | . | . | . | . | . | . | . | . | . | . | . | . | . | . | . | . | . | . | . |
| 21-225_87E10_LC | . | . | . | . | S | . | . | . | . | . | . | . | . | . | . | K | . | . | . |
| 21-225_8B11_LC | . | . | . | . | . | . | . | . | . | . | . | . | . | . | . | . | . | . | . |

**FIGURE 57**

Table 108. Consensus 28 - VK1|L5/JK1 (SEQ ID NO: 50325):

DIQMTQSPSSVSASVGDRVTITCRAS--QGIS------NWLAWYQQKPGKAPKLLIYA--------ASSLQSGVPSRFSGSGSG--TDFTLTISSLQPEDFATYYCQQANS----------------------FPWTFGQGTKVEIKR wherein:

S at position 26 can be substituted with N

G at position 30 can be substituted with D, F, N or V

I at position 31 can be substituted with L or V

S at position 32 can be substituted with N, T, I, F or G

N at position 39 can be substituted with S, D, T or R

W at position 40 can be substituted with C

A at position 58 can be substituted with G, D, T or S

A at position 67 can be substituted with V, P or T

S at position 68 can be substituted with F

S at position 69 can be substituted with N

S at position 72 can be substituted with G or N

Q at position 107 can be substituted with L

A at position 109 can be substituted with T, S, G, V or Y

N at position 110 can be substituted with D, H or Y

F at position 135 can be substituted with L

W at position 137 can be substituted with R or P

Arg Ala Xaa Gln Xaa Xaa Xaa Xaa Xaa Leu Ala (SEQ ID NO: 50159)

Xaa Xaa Xaa Xaa Leu Gln Xaa (SEQ ID NO: 50160)

**FIGURE 57**

Xaa Gln Xaa Xaa Ser Xaa Pro Xaa Thr (SEQ ID NO: 50161)

Arg Ala Ser Gln Gly Ile Ser Asn Trp Leu Ala (SEQ ID NO: 50645)

Ala Ala Ser Ser Leu Gln Ser (SEQ ID NO: 50646)

Gln Gln Ala Asn Ser Phe Pro Trp Thr (SEQ ID NO: 50647)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | V | S | A | S | V | G | D | R | V | T | I | T | C | R | A | S |
| 21-225_149G7_LC | . | . | . | . | . | . | . | . | . | . | . | . | . | . | . | . | . | . | . | . | . | . | . | . | . | . |
| 21-225_154E8_LC | . | . | . | . | . | . | . | . | . | . | . | . | . | . | I | . | . | . | . | . | . | . | . | . | . | . |
| 21-225_171A8_LC | A | . | . | . | . | . | . | . | . | . | . | . | . | . | . | . | . | . | . | . | . | . | . | . | . | . |
| 21-225_171A9_LC | . | . | . | . | . | . | . | . | . | . | . | . | . | . | . | . | . | . | . | . | . | . | . | . | . | . |
| 21-225_171G4_LC | . | . | . | . | . | . | . | . | . | . | . | . | . | . | . | . | . | . | . | . | . | . | . | . | . | . |
| 21-225_174G7_LC | A | . | . | . | . | . | . | . | . | . | . | . | . | . | . | . | . | . | . | . | . | . | . | . | . | . |
| 21-225_175C10_LC | . | . | . | . | . | . | . | . | . | . | . | . | . | . | . | . | . | . | . | . | . | . | . | . | . | . |
| 21-225_178A5_LC | . | . | . | . | . | . | . | . | . | . | . | . | . | . | . | . | . | . | . | . | . | . | . | . | . | . |
| 21-225_178F7_LC | . | . | . | . | . | . | . | . | . | . | . | . | . | . | . | . | . | . | . | . | . | . | . | . | . | . |
| 21-225_179G1_LC | . | . | . | . | . | . | . | . | . | . | . | . | . | . | . | . | . | . | . | . | . | . | . | . | . | . |
| 21-225_17A4_LC | . | . | . | . | . | . | . | . | . | . | . | . | . | . | . | . | . | . | . | . | . | . | . | . | . | . |
| 21-225_188E8_LC | . | . | . | . | . | . | . | . | . | . | . | . | . | . | . | . | . | . | . | . | . | . | . | . | . | N |
| 21-225_190B9_LC | . | . | . | . | . | . | . | . | . | . | . | . | . | . | . | . | . | . | . | . | . | . | . | . | . | . |
| 21-225_190H3_LC | . | . | . | . | . | . | . | . | . | . | . | . | . | . | . | . | . | . | . | . | . | . | . | . | . | . |
| 21-225_193F2_LC | . | . | . | . | . | . | . | . | . | . | . | . | . | . | . | . | . | . | . | . | . | . | . | . | . | . |
| 21-225_194F7_LC | . | . | . | . | . | . | . | . | . | . | . | . | . | . | . | . | . | . | . | . | . | . | . | . | . | . |
| 21-225_195G12_LC | . | . | . | . | . | . | . | . | . | . | . | . | . | . | . | . | . | . | . | . | . | . | . | . | . | . |
| 21-225_196B9_LC | . | . | . | . | . | . | . | . | . | . | . | . | . | . | . | . | . | . | . | . | . | . | . | . | . | . |
| 21-225_197C9_LC | . | . | . | . | . | . | . | . | . | . | . | . | . | . | . | . | . | . | . | . | . | . | . | . | . | . |
| 21-225_20F7_LC | . | . | . | . | . | . | . | . | . | . | . | . | . | . | . | . | . | Q | . | . | . | . | . | . | . | . |
| 21-225_224D6_LC | . | . | . | . | . | . | . | . | . | . | . | . | . | . | . | . | . | . | . | . | . | . | . | . | . | . |
| 21-225_227E4_LC | . | . | L | . | . | . | . | . | . | . | . | . | . | . | . | . | . | . | . | . | . | . | . | . | . | . |
| 21-225_25B3_LC | . | . | . | . | . | . | . | . | . | . | . | . | . | . | . | . | . | . | . | . | . | . | . | . | . | . |
| 21-225_25D7_LC | . | . | . | . | . | . | . | . | . | . | . | . | . | . | . | . | . | . | . | . | F | . | . | . | . | . |
| 21-225_26D12_LC | . | . | . | . | . | . | . | . | . | . | . | . | . | . | . | . | . | . | . | . | . | . | . | . | . | . |
| 21-225_27A11_LC | . | . | . | . | . | . | . | . | . | . | . | . | . | . | . | . | . | . | . | . | . | . | . | . | . | . |
| 21-225_27C5_LC | . | . | . | . | . | . | . | . | . | . | . | . | . | . | . | . | . | . | . | . | . | . | . | . | . | . |
| 21-225_43C2_LC | . | . | . | . | . | . | . | . | . | . | . | . | . | . | . | . | . | . | . | . | . | . | . | . | . | . |
| 21-225_43E8_LC | . | . | . | . | N | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | V | S | A | S | V | G | D | R | V | T | I | T | C | R | A | S |
| 21-225_43H4_LC | . | . | . | . | . | . | . | . | . | . | . | . | . | . | . | . | . | . | . | . | . | . | . | . | . | . |
| 21-225_44C12_LC | . | . | . | . | . | . | . | . | . | . | . | . | . | . | . | . | . | . | . | . | . | . | . | . | . | . |
| 21-225_44D10_LC | . | . | . | . | . | . | . | . | . | . | . | . | . | . | . | . | . | . | . | . | . | . | . | . | . | . |
| 21-225_45F8_LC | . | . | . | . | . | . | . | . | . | . | . | . | . | . | . | . | . | . | . | . | . | . | . | . | . | . |
| 21-225_46A6_LC | . | . | . | . | N | . | . | . | . | . | . | . | . | . | . | . | . | . | . | N | . | . | . | . | . | . |
| 21-225_47G7_LC | . | . | . | . | . | . | . | . | . | . | . | . | . | . | . | . | . | . | . | . | . | . | . | . | . | . |
| 21-225_48D7_LC | . | . | . | . | . | . | . | . | . | . | . | . | . | . | . | . | . | . | . | . | . | . | . | . | . | . |
| 21-225_51G7_LC | . | . | . | . | . | . | . | . | . | . | . | . | . | . | . | . | . | . | . | . | . | . | . | . | . | . |
| 21-225_52H2_LC | . | . | . | . | . | . | . | . | . | . | . | . | . | . | . | . | . | . | . | . | . | . | . | . | . | . |
| 21-225_54G3_LC | . | . | . | . | . | . | . | . | . | . | . | . | . | . | . | . | . | . | . | . | . | . | . | . | . | . |
| 21-225_57F8_LC | . | . | . | . | . | . | . | . | . | . | . | . | . | . | . | . | . | . | . | . | . | . | . | . | . | . |
| 21-225_5A4_LC | . | . | . | . | . | . | . | . | . | . | . | . | . | . | . | . | . | . | . | . | . | . | . | . | . | . |
| 21-225_60A11_LC | . | . | . | . | . | . | . | . | . | . | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | S | - | - | - | - | - | - | N | W | L | A | W | Y | Q | Q | K | P | G | K | A | P |
| 21-225_149G7_LC | . | . | . | F | . | . | . | . | . | . | . | . | S | . | . | . | . | . | . | . | . | . | . | . | . | . |
| 21-225_154E8_LC | . | . | . | F | . | . | . | . | . | . | . | . | S | . | . | . | . | . | . | . | . | . | . | . | . | . |
| 21-225_171A8_LC | . | . | . | . | . | . | . | . | . | . | . | . | . | . | . | . | . | . | . | . | . | . | . | . | . | . |
| 21-225_171A9_LC | . | . | . | D | . | T | . | . | . | . | . | . | T | . | . | . | . | . | . | . | . | . | . | . | . | . |
| 21-225_171G4_LC | . | . | . | . | V | N | . | . | . | . | . | . | D | . | . | . | . | . | . | . | . | . | . | R | . | . |
| 21-225_174G7_LC | . | . | . | . | . | . | . | . | . | . | . | . | . | . | . | . | . | . | . | . | . | . | . | . | . | . |
| 21-225_175C10_LC | . | . | . | . | . | T | . | . | . | . | . | . | D | . | . | . | . | . | . | . | . | . | . | . | . | . |
| 21-225_178A5_LC | . | . | . | . | . | . | . | . | . | . | . | . | . | . | . | . | . | . | . | . | . | . | . | . | . | . |
| 21-225_178F7_LC | . | . | . | D | . | T | . | . | . | . | . | . | D | . | . | . | . | . | . | . | . | . | . | . | . | . |
| 21-225_179G1_LC | . | . | . | D | . | . | . | . | . | . | . | . | D | . | . | . | . | . | . | . | . | S | . | . | . | . |
| 21-225_17A4_LC | . | . | . | . | . | G | . | . | . | . | . | . | . | . | . | . | . | . | . | . | . | . | . | . | . | . |
| 21-225_188E8_LC | . | . | . | D | . | . | . | . | . | . | . | . | S | . | . | . | . | . | . | . | . | . | . | . | . | . |
| 21-225_190B9_LC | . | . | . | . | . | N | . | . | . | . | . | . | . | . | . | . | . | . | . | . | . | . | . | . | . | . |
| 21-225_190H3_LC | . | . | . | . | L | N | . | . | . | . | . | . | . | . | . | . | . | . | . | L | . | . | . | . | . | . |
| 21-225_193F2_LC | . | . | . | . | . | N | . | . | . | . | . | . | . | . | . | . | . | . | . | . | . | . | . | . | . | . |
| 21-225_194F7_LC | . | . | . | . | . | . | . | . | . | . | . | . | R | . | . | . | . | F | . | . | . | . | . | . | . | . |
| 21-225_195G12_LC | . | . | . | . | . | N | . | . | . | . | . | . | . | . | . | . | . | . | . | . | . | . | . | . | . | . |
| 21-225_196B9_LC | . | . | . | . | . | . | . | . | . | . | . | . | . | C | . | . | . | . | . | . | . | . | . | . | . | . |
| 21-225_197C9_LC | . | . | . | . | . | . | . | . | . | . | . | . | . | . | . | . | . | F | . | . | . | . | . | . | . | . |
| 21-225_20F7_LC | . | . | . | . | . | . | . | . | . | . | . | . | S | . | . | . | . | . | . | . | . | . | . | . | . | . |
| 21-225_224D6_LC | . | . | . | . | . | . | . | . | . | . | . | . | S | . | . | . | . | . | . | . | . | . | . | . | . | . |
| 21-225_227E4_LC | . | . | . | . | . | I | . | . | . | . | . | . | . | . | . | . | . | . | . | . | . | . | . | . | . | . |
| 21-225_25B3_LC | . | . | . | . | . | . | . | . | . | . | . | . | S | . | . | . | . | . | . | . | . | . | . | . | . | . |
| 21-225_25D7_LC | . | . | . | D | . | P | . | . | . | . | . | . | D | . | . | . | . | . | . | . | . | . | . | T | . | . |
| 21-225_26D12_LC | . | . | . | . | . | . | . | . | . | . | . | . | S | . | . | . | . | . | . | . | . | . | . | . | . | . |
| 21-225_27A11_LC | . | . | . | . | . | . | . | . | . | . | . | . | S | . | . | . | . | . | . | . | . | . | . | . | . | . |
| 21-225_27C5_LC | . | . | . | . | . | . | . | . | . | . | . | . | S | . | . | . | . | . | . | . | . | . | . | . | . | . |
| 21-225_43C2_LC | . | . | . | . | . | . | . | . | . | . | . | . | D | . | . | . | . | . | . | . | . | . | . | . | . | . |
| 21-225_43E8_LC | . | . | . | . | . | . | . | . | . | . | . | . | . | . | . | . | . | . | . | . | . | . | . | R | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | S | - | - | - | - | - | - | N | W | L | A | W | Y | Q | Q | K | P | G | K | A | P |
| 21-225_43H4_LC | . | . | . | . | . | I | . | . | . | . | . | . | . | . | . | . | . | . | . | . | . | . | . | . | . | . |
| 21-225_44C12_LC | . | . | . | . | . | . | . | . | . | . | . | . | . | . | . | . | . | . | . | . | . | . | . | . | . | . |
| 21-225_44D10_LC | . | . | . | . | . | . | . | . | . | . | . | . | D | . | . | . | . | . | . | . | . | . | . | . | . | . |
| 21-225_45F8_LC | . | . | . | . | . | . | . | . | . | . | . | . | D | . | . | . | . | . | . | . | . | . | . | . | . | . |
| 21-225_46A6_LC | . | . | . | . | . | . | . | . | . | . | . | . | . | . | . | . | . | . | . | . | . | . | . | . | V | . |
| 21-225_47G7_LC | . | . | . | . | . | . | . | . | . | . | . | . | . | . | . | . | . | . | . | . | . | . | . | . | . | . |
| 21-225_48D7_LC | . | . | . | N | . | T | . | . | . | . | . | . | S | . | . | . | . | . | . | . | . | . | . | . | . | . |
| 21-225_51G7_LC | . | . | . | D | . | . | . | . | . | . | . | . | S | . | . | . | . | . | . | . | . | . | . | . | . | . |
| 21-225_52H2_LC | . | . | . | N | . | T | . | . | . | . | . | . | . | . | . | . | . | F | . | . | . | . | . | R | . | . |
| 21-225_54G3_LC | . | . | . | . | . | . | . | . | . | . | . | . | S | . | . | . | . | . | . | . | . | . | . | . | . | . |
| 21-225_57F8_LC | . | . | . | . | . | . | . | . | . | . | . | . | . | . | . | . | . | . | . | . | . | . | . | . | . | . |
| 21-225_5A4_LC | . | . | . | . | . | . | . | . | . | . | . | . | T | . | . | . | . | . | . | . | . | . | . | . | . | . |
| 21-225_60A11_LC | . | . | . | V | . | . | . | . | . | . | . | . | . | . | . | . | . | . | . | L | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | Y | A | - | - | - | - | - | - | - | - | A | S | S | L | Q | S | G | V | P | S | R | F |
| 21-225_149G7_LC | . | F | . | . | . | . | . | . | . | . | . | . | . | . | . | . | . | . | . | . | . | . | . | . | . | . |
| 21-225_154E8_LC | . | . | . | . | . | T | . | . | . | . | . | . | . | . | . | . | . | . | . | . | . | . | . | . | . | . |
| 21-225_171A8_LC | N | . | M | . | H | . | . | . | . | . | . | . | . | . | . | F | . | . | . | G | . | . | . | . | . | . |
| 21-225_171A9_LC | . | . | . | . | . | D | . | . | . | . | . | . | . | . | . | . | . | . | . | . | A | . | . | . | . | . |
| 21-225_171G4_LC | . | . | . | . | . | D | . | . | . | . | . | . | . | . | . | . | . | . | . | . | . | . | . | . | . | . |
| 21-225_174G7_LC | N | . | . | . | H | . | . | . | . | . | . | . | . | . | . | F | . | . | . | G | . | . | . | . | . | . |
| 21-225_175C10_LC | . | . | . | . | . | . | . | . | . | . | . | . | . | . | . | . | . | . | . | . | . | . | . | . | . | . |
| 21-225_178A5_LC | . | . | . | . | . | . | . | . | . | . | . | . | . | . | . | . | . | . | . | . | . | . | . | . | . | . |
| 21-225_178F7_LC | . | . | . | . | S | . | . | . | . | . | . | . | . | . | . | . | . | . | . | . | . | . | . | . | . | . |
| 21-225_179G1_LC | . | . | . | . | S | . | . | . | . | . | . | . | . | . | . | . | . | . | . | . | . | . | . | . | . | . |
| 21-225_17A4_LC | . | . | . | . | F | . | . | . | . | . | . | . | . | . | . | . | . | . | . | . | . | . | . | . | . | . |
| 21-225_188E8_LC | . | . | . | . | . | . | . | . | . | . | . | . | . | . | . | . | N | . | . | . | . | . | . | . | G | . |
| 21-225_190B9_LC | . | . | . | . | . | G | . | . | . | . | . | . | . | . | V | . | . | . | . | . | . | . | . | . | . | . |
| 21-225_190H3_LC | . | . | . | . | . | . | . | . | . | . | . | . | . | . | V | . | . | . | . | . | . | . | . | . | . | . |
| 21-225_193F2_LC | . | . | . | . | . | G | . | . | . | . | . | . | . | . | V | . | . | . | . | . | . | . | . | . | . | . |
| 21-225_194F7_LC | . | I | . | . | . | . | . | . | . | . | . | . | . | . | . | . | . | . | . | . | . | . | . | . | . | . |
| 21-225_195G12_LC | . | . | . | . | . | G | . | . | . | . | . | . | . | . | V | . | . | . | . | . | . | . | . | . | . | . |
| 21-225_196B9_LC | . | F | . | . | C | . | . | . | . | . | . | . | . | . | . | . | . | . | . | . | A | . | . | . | . | . |
| 21-225_197C9_LC | Q | . | . | . | . | . | . | . | . | . | . | . | . | . | . | . | . | . | . | . | . | . | . | . | . | . |
| 21-225_20F7_LC | . | . | . | . | . | . | . | . | . | . | . | . | . | . | . | . | . | . | . | . | . | . | . | . | . | . |
| 21-225_224D6_LC | . | . | . | . | . | . | . | . | . | . | . | . | . | . | . | . | . | . | . | . | . | . | . | . | . | . |
| 21-225_227E4_LC | . | . | . | . | . | . | . | . | . | . | . | . | . | . | . | . | . | . | . | . | . | . | . | . | . | . |
| 21-225_25B3_LC | . | . | . | . | . | G | . | . | . | . | . | . | . | . | . | . | . | . | . | . | . | . | . | . | . | . |
| 21-225_25D7_LC | . | . | . | . | . | . | . | . | . | . | . | . | . | . | . | . | . | . | . | . | . | . | . | . | . | . |
| 21-225_26D12_LC | . | . | . | . | . | G | . | . | . | . | . | . | . | . | . | . | . | . | . | . | . | . | . | . | . | . |
| 21-225_27A11_LC | . | . | . | . | . | G | . | . | . | . | . | . | . | . | . | . | . | . | . | . | . | . | . | . | . | . |
| 21-225_27C5_LC | . | F | . | . | . | G | . | . | . | . | . | . | . | . | . | . | . | . | . | . | . | . | . | . | . | . |
| 21-225_43C2_LC | R | . | . | . | . | . | . | . | . | . | . | . | . | . | . | . | . | . | . | . | . | . | . | . | . | . |
| 21-225_43E8_LC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | Y | A | - | - | - | - | - | - | - | - | A | S | S | L | Q | S | G | V | P | S | R | F |
| 21-225_43H4_LC | . | . | . | . | . | . | . | . | . | . | . | . | . | . | . | . | . | . | . | . | . | . | . | . | . | . |
| 21-225_44C12_LC | . | . | . | . | S | . | . | . | . | . | . | . | . | . | . | F | . | . | . | . | . | . | . | . | . | . |
| 21-225_44D10_LC | R | . | . | . | . | . | . | . | . | . | . | . | . | . | . | . | . | . | . | . | . | . | . | . | K | . |
| 21-225_45F8_LC | R | . | . | . | . | G | . | . | . | . | . | . | . | . | . | . | . | . | . | . | . | . | . | . | . | . |
| 21-225_46A6_LC | . | . | . | . | . | . | . | . | . | . | . | . | . | . | . | . | . | . | . | . | . | . | . | . | . | . |
| 21-225_47G7_LC | . | . | . | . | F | . | . | . | . | . | . | . | . | . | . | . | N | . | . | . | . | . | . | . | . | . |
| 21-225_48D7_LC | . | . | . | . | . | S | . | . | . | . | . | . | . | . | . | . | . | . | . | N | . | . | . | . | . | . |
| 21-225_51G7_LC | . | V | . | . | . | . | . | . | . | . | . | . | . | . | P | . | N | . | . | . | A | . | . | . | . | . |
| 21-225_52H2_LC | . | . | . | . | . | T | . | . | . | . | . | . | . | . | T | . | . | . | . | . | . | . | . | . | . | . |
| 21-225_54G3_LC | . | . | . | . | . | D | . | . | . | . | . | . | . | . | . | . | . | . | . | . | . | . | . | . | . | . |
| 21-225_57F8_LC | . | . | . | . | . | T | . | . | . | . | . | . | . | . | . | . | . | . | . | . | . | . | . | . | . | . |
| 21-225_5A4_LC | . | . | . | . | . | D | . | . | . | . | . | . | . | . | . | . | . | . | . | . | . | . | . | . | . | . |
| 21-225_60A11_LC | . | . | . | . | . | T | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_149G7_LC | . | . | . | . | . | . | . | . | . | . | . | . | . | . | . | . | . | . | . | . | . | . | . | . | . | . |
| 21-225_154E8_LC | . | . | . | . | . | . | . | . | . | . | . | . | . | . | . | . | . | . | . | . | . | . | . | . | . | . |
| 21-225_171A8_LC | . | . | . | . | . | . | . | . | . | . | . | . | . | . | . | . | . | . | . | . | . | . | . | . | . | . |
| 21-225_171A9_LC | . | . | . | . | . | . | . | . | . | . | . | . | . | . | V | . | . | . | . | . | . | . | . | . | . | . |
| 21-225_171G4_LC | . | . | . | . | . | . | . | . | . | . | . | . | . | . | . | . | . | . | . | . | . | . | . | . | . | . |
| 21-225_174G7_LC | . | . | . | . | . | . | . | . | . | . | . | . | . | . | . | . | . | . | . | . | . | . | . | . | . | . |
| 21-225_175C10_LC | . | . | . | . | . | . | . | . | . | . | . | . | . | . | F | . | . | . | . | . | D | . | . | . | . | . |
| 21-225_178A5_LC | N | . | . | . | . | . | . | . | . | . | . | . | . | . | . | . | . | . | . | . | . | . | . | . | . | . |
| 21-225_178F7_LC | . | . | . | . | . | . | . | . | . | . | . | . | . | A | . | . | . | . | . | . | . | . | . | . | . | . |
| 21-225_179G1_LC | G | . | . | . | . | . | . | . | . | . | . | . | . | . | . | . | . | . | . | . | . | . | . | . | . | . |
| 21-225_17A4_LC | . | . | . | . | . | . | . | . | . | . | . | . | . | . | . | . | . | . | . | . | . | . | . | V | . | . |
| 21-225_188E8_LC | . | . | . | . | . | . | . | . | . | . | . | . | . | . | . | . | . | . | . | . | . | . | . | . | . | . |
| 21-225_190B9_LC | . | . | . | . | . | . | . | . | . | . | . | . | . | . | . | . | . | . | . | . | . | . | . | . | . | . |
| 21-225_190H3_LC | . | . | . | . | . | . | . | . | S | E | . | . | . | . | . | . | . | . | . | . | . | . | . | . | . | . |
| 21-225_193F2_LC | . | . | . | . | . | . | . | . | . | . | . | . | . | . | . | . | . | . | . | . | . | . | . | . | . | . |
| 21-225_194F7_LC | . | . | . | . | . | . | . | . | . | . | . | . | . | . | . | . | . | . | . | . | . | . | . | . | . | . |
| 21-225_195G12_LC | . | . | . | . | . | . | . | . | . | . | . | . | . | . | . | . | . | . | . | . | . | . | . | . | . | . |
| 21-225_196B9_LC | . | . | . | . | . | . | . | . | . | . | . | . | . | . | . | . | . | . | . | . | . | . | . | . | S | . |
| 21-225_197C9_LC | . | . | . | . | . | . | . | . | . | . | . | S | . | . | . | . | . | . | . | . | . | . | . | . | . | . |
| 21-225_20F7_LC | . | . | . | . | . | . | . | . | . | . | . | . | . | . | . | . | . | . | . | . | . | . | . | . | . | . |
| 21-225_224D6_LC | . | . | . | . | . | . | . | . | . | . | . | . | . | . | . | . | . | . | . | . | . | . | . | . | . | . |
| 21-225_227E4_LC | . | . | . | . | . | . | . | . | . | . | . | . | . | . | . | . | . | . | . | . | . | . | . | . | I | . |
| 21-225_25B3_LC | . | A | . | . | . | . | . | . | . | . | . | . | . | . | . | . | . | . | . | . | . | . | . | . | . | . |
| 21-225_25D7_LC | . | . | . | . | . | . | . | . | . | N | . | . | . | . | V | . | G | . | . | . | . | . | . | . | . | . |
| 21-225_26D12_LC | . | A | . | . | . | . | . | . | . | . | . | . | . | . | . | . | . | . | . | . | . | . | . | . | . | . |
| 21-225_27A11_LC | . | A | . | . | . | . | . | . | . | . | . | . | . | . | . | . | . | . | . | . | . | . | . | . | . | . |
| 21-225_27C5_LC | . | A | . | . | . | . | . | . | . | E | . | . | . | . | . | . | . | . | . | . | . | . | . | . | . | . |
| 21-225_43C2_LC | . | . | . | . | . | . | . | . | . | . | . | . | . | . | . | . | N | . | . | . | . | . | . | . | . | . |
| 21-225_43E8_LC | . | . | . | . | . | . | . | . | . | . | . | . | . | . | . | . | N | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_43H4_LC | . | . | . | . | . | . | . | . | . | . | . | . | . | . | . | . | N | . | . | . | . | . | . | . | . | . |
| 21-225_44C12_LC | . | . | . | . | . | . | . | . | . | . | . | . | . | S | . | . | . | . | . | . | . | . | . | . | . | . |
| 21-225_44D10_LC | . | . | . | . | . | . | . | . | . | . | . | . | . | . | . | . | N | . | . | . | . | . | . | . | . | . |
| 21-225_45F8_LC | . | . | . | . | . | . | . | . | . | . | . | . | . | . | . | . | N | . | . | . | . | . | . | . | . | . |
| 21-225_46A6_LC | . | . | . | . | . | . | . | . | . | . | . | . | . | . | . | . | N | . | . | . | . | . | . | . | . | . |
| 21-225_47G7_LC | . | . | . | . | . | . | . | . | . | . | . | . | . | . | . | . | . | . | . | . | A | . | . | . | . | . |
| 21-225_48D7_LC | . | . | . | . | . | . | . | . | . | . | . | . | . | . | . | . | . | . | . | . | . | . | . | V | . | . |
| 21-225_51G7_LC | . | . | . | . | . | . | . | . | . | . | . | . | . | . | . | . | . | . | . | . | . | . | . | . | . | . |
| 21-225_52H2_LC | . | . | . | . | . | . | . | . | . | . | . | . | . | . | . | . | . | . | . | . | . | . | . | . | . | . |
| 21-225_54G3_LC | . | . | . | . | . | E | . | . | . | . | . | . | . | . | . | . | . | . | . | . | . | . | . | . | . | . |
| 21-225_57F8_LC | . | . | N | E | . | . | . | . | . | . | . | . | . | . | . | . | . | . | . | . | . | . | . | . | . | . |
| 21-225_5A4_LC | . | . | . | . | . | . | . | . | . | . | . | . | . | . | . | . | . | . | . | . | . | . | . | . | . | . |
| 21-225_60A11_LC | . | . | N | E | . | . | . | . | . | . | . | . | . | . | . | . | . | . | . | . | . | . | . | . | . | . |

# FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | A | N | S | - | - | - | - | - | - | - | - | - | - | - | - | - | - | - | - | - | - | - |
| 21-225_149G7_LC | . | . | . | . | . | . | . | . | . | . | . | . | . | . | . | . | . | . | . | . | . | . | . | . | . | . |
| 21-225_154E8_LC | C | . | . | . | . | . | . | . | . | . | . | . | . | . | . | . | . | . | . | . | . | . | . | . | . | . |
| 21-225_171A8_LC | . | . | . | . | T | . | . | . | . | . | . | . | . | . | . | . | . | . | . | . | . | . | . | . | . | . |
| 21-225_171A9_LC | . | . | L | . | T | . | . | . | . | . | . | . | . | . | . | . | . | . | . | . | . | . | . | . | . | . |
| 21-225_171G4_LC | . | . | . | . | . | . | . | . | . | . | . | . | . | . | . | . | . | . | . | . | . | . | . | . | . | . |
| 21-225_174G7_LC | . | . | . | . | T | . | . | . | . | . | . | . | . | . | . | . | . | . | . | . | . | . | . | . | . | . |
| 21-225_175C10_LC | . | . | . | . | T | . | . | . | . | . | . | . | . | . | . | . | . | . | . | . | . | . | . | . | . | . |
| 21-225_178A5_LC | C | . | . | . | . | . | . | . | . | . | . | . | . | . | . | . | . | . | . | . | . | . | . | . | . | . |
| 21-225_178F7_LC | . | . | . | . | . | . | . | . | . | . | . | . | . | . | . | . | . | . | . | . | . | . | . | . | . | . |
| 21-225_179G1_LC | . | . | . | . | . | . | . | . | . | . | . | . | . | . | . | . | . | . | . | . | . | . | . | . | . | . |
| 21-225_17A4_LC | . | . | . | . | . | . | . | . | . | . | . | . | . | . | . | . | . | . | . | . | . | . | . | . | . | . |
| 21-225_188E8_LC | . | . | . | . | . | . | . | . | . | . | . | . | . | . | . | . | . | . | . | . | . | . | . | . | . | . |
| 21-225_190B9_LC | S | . | . | . | . | . | . | . | . | . | . | . | . | . | . | . | . | . | . | . | . | . | . | . | . | . |
| 21-225_190H3_LC | H | . | . | . | . | . | . | . | . | . | . | . | . | . | . | . | . | . | . | . | . | . | . | . | . | . |
| 21-225_193F2_LC | S | . | . | . | . | . | . | . | . | . | . | . | . | . | . | . | . | . | . | . | . | . | . | . | . | . |
| 21-225_194F7_LC | . | . | . | . | . | . | . | . | . | . | . | . | . | . | . | . | . | . | . | . | . | . | . | . | . | . |
| 21-225_195G12_LC | S | . | . | . | . | . | . | . | . | . | . | . | . | . | . | . | . | . | . | . | . | . | . | . | . | . |
| 21-225_196B9_LC | . | . | . | . | G | D | . | . | . | . | . | . | . | . | . | . | . | . | . | . | . | . | . | . | . | . |
| 21-225_197C9_LC | . | . | . | . | . | . | . | . | . | . | . | . | . | . | . | . | . | . | . | . | . | . | . | . | . | . |
| 21-225_20F7_LC | . | . | . | . | . | . | . | . | . | . | . | . | . | . | . | . | . | . | . | . | . | . | . | . | . | . |
| 21-225_224D6_LC | . | . | . | . | . | . | . | . | . | . | . | . | . | . | . | . | . | . | . | . | . | . | . | . | . | . |
| 21-225_227E4_LC | . | . | . | . | . | . | . | . | . | . | . | . | . | . | . | . | . | . | . | . | . | . | . | . | . | . |
| 21-225_25B3_LC | C | . | . | . | S | D | . | . | . | . | . | . | . | . | . | . | . | . | . | . | . | . | . | . | . | . |
| 21-225_25D7_LC | . | . | . | . | . | Y | . | . | . | . | . | . | . | . | . | . | . | . | . | . | . | . | . | . | . | . |
| 21-225_26D12_LC | C | . | . | . | S | D | . | . | . | . | . | . | . | . | . | . | . | . | . | . | . | . | . | . | . | . |
| 21-225_27A11_LC | C | . | . | . | S | D | . | . | . | . | . | . | . | . | . | . | . | . | . | . | . | . | . | . | . | . |
| 21-225_27C5_LC | C | . | . | . | Y | D | . | . | . | . | . | . | . | . | . | . | . | . | . | . | . | . | . | . | . | . |
| 21-225_43C2_LC | . | . | . | . | T | . | . | . | . | . | . | . | . | . | . | . | . | . | . | . | . | . | . | . | . | . |
| 21-225_43E8_LC | . | . | . | . | T | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | A | N | S | - | - | - | - | - | - | - | - | - | - | - | - | - | - | - | - | - | - | - |
| 21-225_43H4_LC | . | . | . | . | T | . | . | . | . | . | . | . | . | . | . | . | . | . | . | . | . | . | . | . | . | . |
| 21-225_44C12_LC | . | . | . | . | S | . | . | . | . | . | . | . | . | . | . | . | . | . | . | . | . | . | . | . | . | . |
| 21-225_44D10_LC | . | . | . | . | T | . | . | . | . | . | . | . | . | . | . | . | . | . | . | . | . | . | . | . | . | . |
| 21-225_45F8_LC | . | . | . | . | T | . | . | . | . | . | . | . | . | . | . | . | . | . | . | . | . | . | . | . | . | . |
| 21-225_46A6_LC | . | . | . | . | T | . | . | . | . | . | . | . | . | . | . | . | . | . | . | . | . | . | . | . | . | . |
| 21-225_47G7_LC | C | . | . | . | V | . | . | . | . | . | . | . | . | . | . | . | . | . | . | . | . | . | . | . | . | . |
| 21-225_48D7_LC | C | . | . | . | . | . | . | . | . | . | . | . | . | . | . | . | . | . | . | . | . | . | . | . | . | . |
| 21-225_51G7_LC | . | . | . | . | . | H | . | . | . | . | . | . | . | . | . | . | . | . | . | . | . | . | . | . | . | . |
| 21-225_52H2_LC | C | . | . | . | T | . | . | . | . | . | . | . | . | . | . | . | . | . | . | . | . | . | . | . | . | . |
| 21-225_54G3_LC | . | . | . | . | . | . | . | . | . | . | . | . | . | . | . | . | . | . | . | . | . | . | . | . | . | . |
| 21-225_57F8_LC | . | . | . | . | . | . | . | . | . | . | . | . | . | . | . | . | . | . | . | . | . | . | . | . | . | . |
| 21-225_5A4_LC | . | . | . | . | . | . | . | . | . | . | . | . | . | . | . | . | . | . | . | . | . | . | . | . | . | . |
| 21-225_60A11_LC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | F | P | W | T | F | G | Q | G | T | K | V | E | I | K | R |
| 21-225_149G7_LC | . | . | . | . | . | . | . | . | . | . | . | . | . | . | . | . | . | N | . |
| 21-225_154E8_LC | . | . | . | . | . | . | . | . | . | . | . | . | . | . | . | . | F | N | . |
| 21-225_171A8_LC | . | . | . | . | . | . | . | . | . | . | . | . | . | . | . | . | . | . | . |
| 21-225_171A9_LC | . | . | . | . | . | . | . | . | . | . | . | . | . | . | . | . | . | . | . |
| 21-225_171G4_LC | . | . | . | . | . | . | . | . | . | . | . | . | . | . | . | . | . | . | . |
| 21-225_174G7_LC | . | . | . | . | . | . | . | . | . | . | . | . | . | . | . | . | . | . | . |
| 21-225_175C10_LC | . | . | . | . | . | . | . | . | . | . | . | . | . | . | . | . | . | . | . |
| 21-225_178A5_LC | . | . | . | . | L | . | . | . | . | . | . | . | . | . | . | . | . | . | . |
| 21-225_178F7_LC | . | . | . | . | L | . | . | . | . | . | . | . | . | . | . | . | . | . | . |
| 21-225_179G1_LC | . | . | . | . | L | . | . | . | . | . | . | . | . | . | . | . | . | . | . |
| 21-225_17A4_LC | . | . | . | . | . | . | R | . | . | . | . | . | . | . | . | . | . | . | . |
| 21-225_188E8_LC | . | . | . | . | . | . | . | . | . | . | . | . | . | . | . | . | . | . | . |
| 21-225_190B9_LC | . | . | . | . | . | . | . | . | . | . | . | . | . | . | . | . | . | R | . |
| 21-225_190H3_LC | . | . | . | . | L | . | . | . | . | . | . | . | . | . | . | . | . | . | . |
| 21-225_193F2_LC | . | . | . | . | . | . | . | . | . | . | . | . | . | . | . | . | . | R | . |
| 21-225_194F7_LC | . | . | . | . | . | . | . | . | . | . | . | . | . | . | . | . | . | . | . |
| 21-225_195G12_LC | . | . | . | . | . | . | . | . | . | . | R | . | . | . | . | . | N | Q | T |
| 21-225_196B9_LC | . | . | . | . | . | . | P | . | . | . | . | . | . | . | . | . | F | R | . |
| 21-225_197C9_LC | . | . | . | . | . | . | . | . | . | . | . | . | . | . | . | . | . | . | . |
| 21-225_20F7_LC | . | . | . | . | . | . | R | . | . | . | . | . | . | . | . | . | . | . | . |
| 21-225_224D6_LC | . | . | . | . | . | . | . | . | . | . | P | . | . | . | . | . | . | . | . |
| 21-225_227E4_LC | . | . | . | . | . | . | . | . | . | . | . | . | . | . | . | . | . | . | . |
| 21-225_25B3_LC | . | . | . | . | . | . | R | . | . | . | . | . | . | . | . | . | . | . | . |
| 21-225_25D7_LC | . | . | . | . | . | . | . | . | . | . | . | . | . | . | . | . | . | . | . |
| 21-225_26D12_LC | . | . | . | . | . | . | R | . | . | . | . | . | . | . | . | . | . | . | . |
| 21-225_27A11_LC | . | . | . | . | . | . | R | . | . | . | . | . | . | . | . | . | . | . | . |
| 21-225_27C5_LC | . | . | . | . | . | . | R | . | . | . | . | . | . | . | . | . | . | . | . |
| 21-225_43C2_LC | . | . | . | . | . | . | . | . | . | . | . | . | . | . | . | . | . | . | . |
| 21-225_43E8_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | F | P | W | T | F | G | Q | G | T | K | V | E | I | K | R |
| 21-225_43H4_LC | . | . | . | . | . | . | . | . | . | . | . | . | . | . | . | . | . | . | . |
| 21-225_44C12_LC | . | . | . | . | . | . | . | . | . | . | . | . | . | . | . | . | . | . | . |
| 21-225_44D10_LC | . | . | . | . | . | . | . | . | . | . | . | . | . | . | . | . | . | . | . |
| 21-225_45F8_LC | . | . | . | . | . | . | . | . | . | . | . | . | . | . | . | . | . | . | . |
| 21-225_46A6_LC | . | . | . | . | . | . | . | . | . | . | . | . | . | . | . | . | . | . | . |
| 21-225_47G7_LC | . | . | . | . | . | . | . | . | . | . | . | . | . | . | . | . | . | . | . |
| 21-225_48D7_LC | . | . | . | . | . | . | . | . | . | . | . | . | . | . | . | . | . | . | . |
| 21-225_51G7_LC | . | . | . | . | . | . | . | . | . | . | . | . | . | . | . | . | . | . | . |
| 21-225_52H2_LC | . | . | . | . | . | . | . | . | . | . | H | . | . | . | . | . | V | . | . |
| 21-225_54G3_LC | . | . | . | . | . | . | . | . | . | . | . | . | . | . | . | . | . | . | . |
| 21-225_57F8_LC | . | . | . | . | . | . | . | . | . | . | . | . | . | . | . | . | . | . | . |
| 21-225_5A4_LC | . | . | . | . | . | . | . | . | . | . | . | . | . | . | . | . | . | . | . |
| 21-225_60A11_LC | . | . | . | . | . | . | . | . | . | . | . | . | . | . | . | . | . | . | . |

**FIGURE 57**

Table 109. Consensus 29 - VK4|B3/JK3 (SEQ ID NO: 50326):

DIVMTQSPDSLAVSLGERATINCKSS--QSVLHSSNNNNYLAWYQQKPGQPPKLLIYW--------ASTRESGVPDRFSGSGSG--TDFTLTISSLQAEDVAVYYCQQYYS---------------------TPVTFGPGTKVDIKR wherein:

S at position 26 can be substituted with N

S at position 30 can be substituted with R or N

V at position 31 can be substituted with I or L

L at position 32 can be substituted with F

H at position 33 can be substituted with F, Y, K or S

S at position 34 can be substituted with N or H

N at position 36 can be substituted with H

N at position 37 can be substituted with S

N at position 38 can be substituted with K, Y or H

N at position 39 can be substituted with R or S

A at position 42 can be substituted with T or V

T at position 69 can be substituted with A, I or S

R at position 70 can be substituted with L

E at position 71 can be substituted with K or D

Q at position 107 can be substituted with H

Y at position 109 can be substituted with S

Y at position 110 can be substituted with C, F, S or H

S at position 111 can be substituted with N, Q, D or T

**FIGURE 57**

T at position 135 can be substituted with L, I, A, F or S

V at position 137 can be substituted with F or P

Lys Ser Xaa Gln Xaa Xaa Xaa Xaa Xaa Ser Xaa Xaa Xaa Xaa Tyr Leu Xaa (SEQ ID NO: 50162)

Trp Ala Ser Xaa Xaa Xaa Ser (SEQ ID NO: 50163)

Xaa Gln Xaa Xaa Xaa Xaa Pro Xaa Thr (SEQ ID NO: 50164)

Lys Ser Ser Gln Ser Val Leu His Ser Ser Asn Asn Asn Asn Tyr Leu Ala (SEQ ID NO: 50648)

Trp Ala Ser Thr Arg Glu Ser (SEQ ID NO: 50649)

Gln Gln Tyr Tyr Ser Thr Pro Val Thr (SEQ ID NO: 50650)

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | V | M | T | Q | S | P | D | S | L | A | V | S | L | G | E | R | A | T | I | N | C | K | S | S |
| 21-225_11E2_LC | . | . | . | . | . | . | . | . | . | . | . | . | . | . | . | . | . | . | . | . | . | . | . | . | . | N |
| 21-225_12F11_LC | . | . | . | . | . | . | . | . | . | . | . | . | . | . | . | . | . | . | . | . | . | . | . | . | . | . |
| 21-225_147G6_LC | . | . | . | . | . | . | . | . | . | . | . | . | . | . | . | . | . | . | . | . | . | . | . | . | . | . |
| 21-225_148G3_LC | . | . | . | . | . | . | . | . | . | . | . | . | . | . | . | . | . | . | . | . | . | H | . | . | . | . |
| 21-225_150B7_LC | . | . | . | . | . | . | . | . | . | . | . | . | . | . | . | . | . | . | . | . | . | . | . | . | . | . |
| 21-225_152E3_LC | . | . | . | . | . | . | . | . | . | . | . | . | . | . | . | . | . | . | . | . | . | . | . | . | . | . |
| 21-225_152F4_LC | . | . | . | . | . | . | . | . | . | . | . | . | . | . | . | . | . | . | . | . | . | . | . | . | . | . |
| 21-225_164B11_LC | . | . | . | . | . | . | . | . | . | . | . | . | . | . | . | . | . | . | . | . | . | . | . | . | . | . |
| 21-225_165H2_LC | . | . | . | . | . | . | . | . | . | . | . | T | . | . | P | . | . | . | . | . | . | . | . | . | . | . |
| 21-225_190E2_LC | . | . | . | . | . | . | . | . | . | . | . | . | . | . | . | . | . | . | . | . | . | . | . | . | . | . |
| 21-225_190H4_LC | A | N | . | . | . | . | . | . | . | . | . | . | . | . | . | . | . | . | T | . | . | . | . | . | . | . |
| 21-225_190H6_LC | . | . | . | . | . | . | . | . | . | . | . | . | . | . | . | . | . | . | . | . | . | . | . | . | . | . |
| 21-225_191B12_LC | . | . | . | . | . | . | . | . | . | . | . | . | . | . | . | . | . | . | . | . | . | . | . | . | . | . |
| 21-225_191G3_LC | . | . | . | . | . | . | . | . | . | . | . | . | . | . | . | . | . | . | . | . | . | D | . | . | . | . |
| 21-225_195G11_LC | . | . | . | . | . | . | . | . | . | . | . | . | . | . | . | . | . | . | . | . | . | . | . | . | . | . |
| 21-225_196B1_LC | . | . | . | . | . | . | . | . | . | . | . | . | . | F | . | . | . | . | . | . | . | . | . | . | . | . |
| 21-225_196C1_LC | . | . | . | . | . | . | . | . | . | . | . | . | . | . | . | . | . | . | . | . | . | . | . | . | . | . |
| 21-225_197C8_LC | . | . | . | . | . | P | . | . | . | . | . | . | . | . | . | . | . | . | . | . | . | . | . | . | . | . |
| 21-225_203F10_LC | . | . | . | . | . | . | . | . | . | . | . | . | . | . | . | . | . | . | . | . | . | . | . | . | . | . |
| 21-225_225F4_LC | . | . | . | . | . | . | . | . | . | . | . | . | . | . | . | . | . | . | . | . | . | . | . | . | . | . |
| 21-225_4A2_LC | . | . | . | . | . | . | . | . | . | . | . | . | . | . | . | . | . | . | . | . | . | . | . | . | . | . |
| 21-225_4A2.001_LC | . | . | . | . | . | . | . | . | . | . | . | . | . | . | . | . | . | . | . | . | . | . | . | . | . | . |
| 21-225_4A2.001.001_LC | . | . | . | . | . | . | . | . | . | . | . | . | . | . | . | . | . | . | . | . | . | . | . | . | . | . |
| 21-225_4A2.001.002_LC | . | . | . | . | . | . | . | . | . | . | . | . | . | . | . | . | . | . | . | . | . | . | . | . | . | . |
| 21-225_4A2.001.003_LC | . | . | . | . | . | . | . | . | . | . | . | . | . | . | . | . | . | . | . | . | . | . | . | . | . | . |
| 21-225_4A2.001.004_LC | . | . | . | . | . | . | . | . | . | . | . | . | . | . | . | . | . | . | . | . | . | . | . | . | . | . |
| 21-225_4A2.001.005_LC | . | . | . | . | . | . | . | . | . | . | . | . | . | . | . | . | . | . | . | . | . | . | . | . | . | . |
| 21-225_4A2.001.006_LC | . | . | . | . | . | . | . | . | . | . | . | . | . | . | . | . | . | . | . | . | . | . | . | . | . | . |
| 21-225_4A2.001.007_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | V | M | T | Q | S | P | D | S | L | A | V | S | L | G | E | R | A | T | I | N | C | K | S | S |
| 21-225_4A2.001.008_LC | . | . | . | . | . | . | . | . | . | . | . | . | . | . | . | . | . | . | . | . | . | . | . | . | . | . |
| 21-225_4A2.001.009_LC | . | . | . | . | . | . | . | . | . | . | . | . | . | . | . | . | . | . | . | . | . | . | . | . | . | . |
| 21-225_4A2.001.010_LC | . | . | . | . | . | . | . | . | . | . | . | . | . | . | . | . | . | . | . | . | . | . | . | . | . | . |
| 21-225_4A2.001.011_LC | . | . | . | . | . | . | . | . | . | . | . | . | . | . | . | . | . | . | . | . | . | . | . | . | . | . |
| 21-225_4A2.001.012_LC | . | . | . | . | . | . | . | . | . | . | . | . | . | . | . | . | . | . | . | . | . | . | . | . | . | . |
| 21-225_4A2.001.022_LC | . | . | . | . | . | . | . | . | . | . | . | . | . | . | . | . | . | . | . | . | . | . | . | . | . | . |
| 21-225_4A2.001.024_LC | . | . | . | . | . | . | . | . | . | . | . | . | . | . | . | . | . | . | . | . | . | . | . | . | . | . |
| 21-225_6G6_LC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | | K_FR2 | | |
| CONSENSUS | - | - | Q | S | V | L | H | S | S | N | N | N | N | Y | L | A | W | Y | Q | Q | K | P | G | Q | P | P |
| 21-225_11E2_LC | . | . | . | R | . | . | S | . | . | . | . | H | . | . | . | . | . | . | . | . | R | . | . | . | . | . |
| 21-225_12F11_LC | . | . | . | . | . | . | . | . | . | . | . | Y | . | . | . | . | . | . | . | . | . | . | . | . | . | . |
| 21-225_147G6_LC | . | . | . | . | . | . | Y | . | . | . | S | . | . | . | . | . | . | . | . | . | . | . | . | . | . | . |
| 21-225_148G3_LC | . | . | . | R | . | . | . | . | . | . | . | Y | . | . | . | . | . | . | . | . | . | . | . | . | . | . |
| 21-225_150B7_LC | . | . | . | N | . | . | Y | . | . | H | . | . | . | . | . | . | . | . | . | . | . | . | . | . | . | . |
| 21-225_152E3_LC | . | . | . | . | . | . | . | . | . | . | . | Y | . | . | . | V | . | . | . | . | . | . | . | . | S | . |
| 21-225_152F4_LC | . | . | . | . | . | . | Y | . | . | . | . | Y | . | . | . | . | . | . | . | . | . | . | . | . | . | . |
| 21-225_164B11_LC | . | . | . | . | L | . | K | . | . | . | . | K | S | . | . | . | S | . | . | . | . | . | . | . | L | . |
| 21-225_165H2_LC | . | . | . | . | . | . | . | . | . | . | . | K | . | . | . | T | . | . | . | . | . | . | . | . | . | . |
| 21-225_190E2_LC | . | . | . | . | . | . | F | N | . | . | . | K | . | . | . | . | . | . | . | . | . | . | . | . | . | . |
| 21-225_190H4_LC | . | . | . | . | . | . | . | . | . | . | . | Y | . | . | . | . | . | . | R | . | . | . | . | . | . | . |
| 21-225_190H6_LC | . | . | . | . | . | . | F | N | . | . | . | K | . | . | . | . | . | . | . | . | . | . | . | . | . | . |
| 21-225_191B12_LC | . | . | . | . | . | . | F | N | . | . | . | K | . | . | . | . | . | . | . | . | . | . | . | . | . | . |
| 21-225_191G3_LC | . | . | . | . | . | . | . | . | . | . | S | Y | . | . | . | . | . | . | . | . | . | L | . | . | . | . |
| 21-225_195G11_LC | . | . | . | . | . | . | F | N | . | . | . | K | . | . | . | . | . | . | . | . | . | . | . | . | . | . |
| 21-225_196B1_LC | . | . | . | . | I | . | F | . | . | . | . | K | R | . | . | . | . | . | . | . | . | . | . | . | . | . |
| 21-225_196C1_LC | . | . | . | . | . | . | F | N | . | . | . | K | . | . | . | . | . | . | . | . | . | . | . | . | L | . |
| 21-225_197C8_LC | . | . | . | . | . | . | . | . | . | . | . | K | . | . | . | . | . | . | . | . | . | . | . | . | . | . |
| 21-225_203F10_LC | . | . | . | . | . | F | F | H | . | . | . | K | . | . | . | . | . | . | . | . | . | . | . | . | . | . |
| 21-225_225F4_LC | . | . | . | . | . | . | Y | . | . | . | . | K | . | . | . | . | . | . | . | . | . | . | . | . | . | . |
| 21-225_4A2_LC | . | . | . | . | I | . | . | . | . | . | . | . | . | . | . | . | . | F | . | . | . | . | . | . | . | . |
| 21-225_4A2.001_LC | . | . | . | . | I | . | . | . | . | . | . | . | . | . | . | . | . | F | . | . | . | . | . | . | . | . |
| 21-225_4A2.001.001_LC | . | . | . | . | I | . | . | . | . | . | . | . | . | . | . | . | . | F | . | . | . | . | . | . | . | . |
| 21-225_4A2.001.002_LC | . | . | . | . | I | . | . | . | . | . | . | . | . | . | . | . | . | F | . | . | . | . | . | . | . | . |
| 21-225_4A2.001.003_LC | . | . | . | . | I | . | . | . | . | . | . | . | . | . | . | . | . | F | . | . | . | . | . | . | . | . |
| 21-225_4A2.001.004_LC | . | . | . | . | I | . | . | . | . | . | . | . | . | . | . | . | . | F | . | . | . | . | . | . | . | . |
| 21-225_4A2.001.005_LC | . | . | . | . | I | . | . | . | . | . | . | . | . | . | . | . | . | F | . | . | . | . | . | . | . | . |
| 21-225_4A2.001.006_LC | . | . | . | . | I | . | . | . | . | . | . | . | . | . | . | . | . | F | . | . | . | . | . | . | . | . |
| 21-225_4A2.001.007_LC | . | . | . | . | I | . | . | . | . | . | . | . | . | . | . | . | . | F | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | V | L | H | S | S | N | N | N | N | Y | L | A | W | Y | Q | Q | K | P | G | Q | P | P |
| 21-225_4A2.001.008_LC | . | . | . | . | I | . | . | . | . | . | . | . | . | . | . | . | . | F | . | . | . | . | . | . | . | . |
| 21-225_4A2.001.009_LC | . | . | . | . | I | . | . | . | . | . | . | . | . | . | . | . | . | F | . | . | . | . | . | . | . | . |
| 21-225_4A2.001.010_LC | . | . | . | . | I | . | . | . | . | . | . | . | . | . | . | . | . | F | . | . | . | . | . | . | . | . |
| 21-225_4A2.001.011_LC | . | . | . | . | I | . | . | . | . | . | . | . | . | . | . | . | . | F | . | . | . | . | . | . | . | . |
| 21-225_4A2.001.012_LC | . | . | . | . | I | . | . | . | . | . | . | . | . | . | . | . | . | F | . | . | . | . | . | . | . | . |
| 21-225_4A2.001.022_LC | . | . | . | . | I | . | . | . | . | . | . | . | . | . | . | . | . | F | . | . | . | . | . | . | . | . |
| 21-225_4A2.001.024_LC | . | . | . | . | I | . | . | . | . | . | . | . | . | . | . | . | . | F | . | . | . | . | . | . | . | . |
| 21-225_6G6_LC | . | . | . | . | . | . | . | N | . | . | . | Y | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | Y | W | - | - | - | - | - | - | - | - | A | S | T | R | E | S | G | V | P | D | R | F |
| 21-225_11E2_LC | . | . | . | . | . | . | . | . | . | . | . | . | . | . | . | . | . | . | . | . | . | . | . | . | . | . |
| 21-225_12F11_LC | N | . | . | . | F | . | . | . | . | . | . | . | . | . | . | . | . | . | . | . | . | . | . | . | . | . |
| 21-225_147G6_LC | . | . | . | . | . | . | . | . | . | . | . | . | . | . | . | . | A | . | . | . | . | . | . | . | . | . |
| 21-225_148G3_LC | . | . | . | . | . | . | . | . | . | . | . | . | . | . | . | . | . | . | D | . | . | . | . | . | . | . |
| 21-225_150B7_LC | . | . | . | . | . | . | . | . | . | . | . | . | . | . | . | . | . | . | K | . | . | . | . | . | . | . |
| 21-225_152E3_LC | . | R | . | . | . | . | . | . | . | . | . | . | . | . | . | . | . | . | . | . | . | . | . | . | . | . |
| 21-225_152F4_LC | . | . | . | . | . | . | . | . | . | . | . | . | . | . | . | . | . | . | K | . | . | . | . | . | . | . |
| 21-225_164B11_LC | . | . | . | . | . | . | . | . | . | . | . | . | . | . | . | . | S | . | . | . | . | . | . | . | . | . |
| 21-225_165H2_LC | . | . | . | . | . | . | . | . | . | . | . | . | . | . | . | . | . | . | . | . | . | . | . | V | . | . |
| 21-225_190E2_LC | N | . | . | . | . | . | . | . | . | . | . | . | . | . | . | . | . | . | . | . | . | . | . | . | . | . |
| 21-225_190H4_LC | . | . | . | . | . | . | . | . | . | . | . | . | . | . | . | . | . | . | . | . | . | . | . | . | . | . |
| 21-225_190H6_LC | N | . | . | . | . | . | . | . | . | . | . | . | . | . | . | . | . | . | . | . | . | . | . | . | . | . |
| 21-225_191B12_LC | . | . | . | . | . | . | . | . | . | . | . | . | . | . | . | . | . | . | . | . | . | . | . | . | . | . |
| 21-225_191G3_LC | . | . | . | . | . | . | . | . | . | . | . | . | . | . | . | . | . | . | K | . | . | . | . | . | . | . |
| 21-225_195G11_LC | N | . | . | . | . | . | . | . | . | . | . | . | . | . | . | . | . | L | . | . | . | . | . | . | . | . |
| 21-225_196B1_LC | . | . | . | . | . | . | . | . | . | . | . | . | . | . | . | . | I | . | . | . | . | . | . | . | . | . |
| 21-225_196C1_LC | N | . | . | . | . | . | . | . | . | . | . | . | . | . | . | . | . | L | . | . | . | . | . | . | . | . |
| 21-225_197C8_LC | . | . | . | . | . | . | . | . | . | . | . | . | . | . | . | . | . | . | . | . | . | . | . | . | . | . |
| 21-225_203F10_LC | . | . | . | . | . | . | . | . | . | . | . | . | . | . | . | . | . | . | . | . | . | . | . | . | . | . |
| 21-225_225F4_LC | . | . | . | . | . | . | . | . | . | . | . | . | . | . | . | . | . | . | . | . | . | . | . | . | . | . |
| 21-225_4A2_LC | . | . | . | L | . | . | . | . | . | . | . | . | . | . | . | . | . | . | . | . | . | . | . | . | . | . |
| 21-225_4A2.001_LC | . | . | . | L | . | . | . | . | . | . | . | . | . | . | . | . | . | . | . | . | . | . | . | . | . | . |
| 21-225_4A2.001.001_LC | . | . | . | L | . | . | . | . | . | . | . | . | . | . | . | . | . | . | . | . | . | . | . | . | . | . |
| 21-225_4A2.001.002_LC | . | . | . | L | . | . | . | . | . | . | . | . | . | . | . | . | . | . | . | . | . | . | . | . | . | . |
| 21-225_4A2.001.003_LC | . | . | . | L | . | . | . | . | . | . | . | . | . | . | . | . | . | . | . | . | . | . | . | . | . | . |
| 21-225_4A2.001.004_LC | . | . | . | L | . | . | . | . | . | . | . | . | . | . | . | . | . | . | . | . | . | . | . | . | . | . |
| 21-225_4A2.001.005_LC | . | . | . | L | . | . | . | . | . | . | . | . | . | . | . | . | . | . | . | . | . | . | . | . | . | . |
| 21-225_4A2.001.006_LC | . | . | . | L | . | . | . | . | . | . | . | . | . | . | . | . | . | . | . | . | . | . | . | . | . | . |
| 21-225_4A2.001.007_LC | . | . | . | L | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | Y | W | - | - | - | - | - | - | - | - | A | S | T | R | E | S | G | V | P | D | R | F |
| 21-225_4A2.001.008_LC | . | . | . | L | . | . | . | . | . | . | . | . | . | . | . | . | . | . | . | . | . | . | . | . | . | . |
| 21-225_4A2.001.009_LC | . | . | . | L | . | . | . | . | . | . | . | . | . | . | . | . | . | . | . | . | . | . | . | . | . | . |
| 21-225_4A2.001.010_LC | . | . | . | L | . | . | . | . | . | . | . | . | . | . | . | . | . | . | . | . | . | . | . | . | . | . |
| 21-225_4A2.001.011_LC | . | . | . | L | . | . | . | . | . | . | . | . | . | . | . | . | . | . | . | . | . | . | . | . | . | . |
| 21-225_4A2.001.012_LC | . | . | . | L | . | . | . | . | . | . | . | . | . | . | . | . | . | . | . | . | . | . | . | . | . | . |
| 21-225_4A2.001.022_LC | . | . | . | L | . | . | . | . | . | . | . | . | . | . | . | . | . | . | . | . | . | . | . | . | . | . |
| 21-225_4A2.001.024_LC | . | . | . | L | . | . | . | . | . | . | . | . | . | . | . | . | . | . | . | . | . | . | . | . | . | . |
| 21-225_6G6_LC | N | . | . | . | F | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | A | E | D | V | A | V | Y |
| 21-225_11E2_LC | . | . | . | . | . | . | . | . | . | . | . | . | . | . | . | . | . | . | . | . | . | . | . | . | . | . |
| 21-225_12F11_LC | . | . | . | . | F | . | . | . | . | . | . | . | . | N | . | . | . | . | . | . | . | . | . | . | . | . |
| 21-225_147G6_LC | . | . | . | . | . | . | . | . | . | . | . | . | . | . | . | . | . | . | . | . | . | . | . | . | . | . |
| 21-225_148G3_LC | . | . | . | . | . | . | . | . | A | . | . | . | . | . | . | . | . | . | . | . | . | . | . | . | L | . |
| 21-225_150B7_LC | . | . | . | . | . | . | . | . | . | . | . | . | . | . | . | T | . | . | . | . | . | . | . | . | . | . |
| 21-225_152E3_LC | . | . | . | . | . | . | . | . | . | . | . | S | . | . | . | . | . | . | . | . | . | . | . | . | . | . |
| 21-225_152F4_LC | . | . | . | . | . | . | . | . | . | . | . | . | . | . | V | . | . | . | . | . | . | . | . | . | . | . |
| 21-225_164B11_LC | . | . | . | . | . | . | . | . | . | . | . | . | . | . | . | . | . | . | . | . | . | . | . | . | L | . |
| 21-225_165H2_LC | . | . | . | . | . | . | . | . | . | . | . | . | V | P | . | . | . | M | . | D | D | . | . | . | . | . |
| 21-225_190E2_LC | . | . | . | . | . | . | . | . | . | . | . | . | . | . | . | . | . | M | . | . | . | . | . | . | . | . |
| 21-225_190H4_LC | . | . | . | . | . | . | . | . | . | . | . | . | . | . | . | . | . | . | . | . | . | . | . | . | . | . |
| 21-225_190H6_LC | . | . | . | . | C | . | . | . | . | . | . | . | . | . | . | . | . | . | . | . | . | . | . | . | . | . |
| 21-225_191B12_LC | . | . | . | . | . | . | . | . | . | . | . | . | . | . | . | . | N | . | . | . | . | . | . | . | I | . |
| 21-225_191G3_LC | . | . | . | . | . | . | . | . | . | . | . | . | . | . | . | . | . | . | . | . | . | . | . | . | F | . |
| 21-225_195G11_LC | . | . | . | . | C | . | . | . | . | . | . | . | . | . | . | . | . | M | . | . | . | . | . | . | . | . |
| 21-225_196B1_LC | . | . | . | . | . | . | . | . | . | . | . | . | . | . | . | . | . | . | . | . | . | . | . | . | . | . |
| 21-225_196C1_LC | . | . | . | . | C | . | . | . | . | . | . | . | . | . | . | . | . | M | . | . | . | . | . | . | . | . |
| 21-225_197C8_LC | . | . | . | . | . | . | . | . | . | . | . | . | . | S | . | . | . | . | . | . | . | . | . | . | . | . |
| 21-225_203F10_LC | . | . | . | . | . | . | . | . | . | . | . | . | . | . | . | . | . | . | . | . | . | . | . | T | . | . |
| 21-225_225F4_LC | T | . | . | . | . | . | . | . | . | . | . | . | . | . | . | . | C | V | . | P | . | . | . | . | . | . |
| 21-225_4A2_LC | . | . | . | . | . | . | . | . | . | . | . | . | . | . | . | . | . | . | . | P | . | . | . | . | . | . |
| 21-225_4A2.001_LC | . | . | . | . | . | . | . | . | . | . | . | . | . | . | . | . | . | . | . | P | . | . | . | . | . | . |
| 21-225_4A2.001.001_LC | . | . | . | . | . | . | . | . | . | . | . | . | . | . | . | . | . | . | . | P | . | . | . | . | . | . |
| 21-225_4A2.001.002_LC | . | . | . | . | . | . | . | . | . | . | . | . | . | . | . | . | . | . | . | P | . | . | . | . | . | . |
| 21-225_4A2.001.003_LC | . | . | . | . | . | . | . | . | . | . | . | . | . | . | . | . | . | . | . | P | . | . | . | . | . | . |
| 21-225_4A2.001.004_LC | . | . | . | . | . | . | . | . | . | . | . | . | . | . | . | . | . | . | . | P | . | . | . | . | . | . |
| 21-225_4A2.001.005_LC | . | . | . | . | . | . | . | . | . | . | . | . | . | . | . | . | . | . | . | P | . | . | . | . | . | . |
| 21-225_4A2.001.006_LC | . | . | . | . | . | . | . | . | . | . | . | . | . | . | . | . | . | . | . | P | . | . | . | . | . | . |
| 21-225_4A2.001.007_LC | . | . | . | . | . | . | . | . | . | . | . | . | . | . | . | . | . | . | . | P | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | A | E | D | V | A | V | Y |
| 21-225_4A2.001.008_LC | . | . | . | . | . | . | . | . | . | . | . | . | . | . | . | . | . | . | . | P | . | . | . | . | . | . |
| 21-225_4A2.001.009_LC | . | . | . | . | . | . | . | . | . | . | . | . | . | . | . | . | . | . | . | P | . | . | . | . | . | . |
| 21-225_4A2.001.010_LC | . | . | . | . | . | . | . | . | . | . | . | . | . | . | . | . | . | . | . | P | . | . | . | . | . | . |
| 21-225_4A2.001.011_LC | . | . | . | . | . | . | . | . | . | . | . | . | . | . | . | . | . | . | . | P | . | . | . | . | . | . |
| 21-225_4A2.001.012_LC | . | . | . | . | . | . | . | . | . | . | . | . | . | . | . | . | . | . | . | P | . | . | . | . | . | . |
| 21-225_4A2.001.022_LC | . | . | . | . | . | . | . | . | . | . | . | . | . | . | . | . | . | . | . | P | . | . | . | . | . | . |
| 21-225_4A2.001.024_LC | . | . | . | . | . | . | . | . | . | . | . | . | . | . | . | . | . | . | . | P | . | . | . | . | . | . |
| 21-225_6G6_LC | . | . | . | . | F | . | . | . | . | . | . | . | . | N | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | Y | S | - | - | - | - | - | - | - | - | - | - | - | - | - | - | - | - | - | - | - |
| 21-225_11E2_LC | . | . | . | . | . | F | . | . | . | . | . | . | . | . | . | . | . | . | . | . | . | . | . | . | . | . |
| 21-225_12F11_LC | . | . | . | . | . | . | D | . | . | . | . | . | . | . | . | . | . | . | . | . | . | . | . | . | . | . |
| 21-225_147G6_LC | . | . | . | . | . | . | T | . | . | . | . | . | . | . | . | . | . | . | . | . | . | . | . | . | . | . |
| 21-225_148G3_LC | . | . | . | . | . | . | . | . | . | . | . | . | . | . | . | . | . | . | . | . | . | . | . | . | . | . |
| 21-225_150B7_LC | . | . | . | . | . | . | . | . | . | . | . | . | . | . | . | . | . | . | . | . | . | . | . | . | . | . |
| 21-225_152E3_LC | . | . | . | . | . | . | . | . | . | . | . | . | . | . | . | . | . | . | . | . | . | . | . | . | . | . |
| 21-225_152F4_LC | . | . | . | . | . | F | N | . | . | . | . | . | . | . | . | . | . | . | . | . | . | . | . | . | . | . |
| 21-225_164B11_LC | . | . | . | . | . | . | . | . | . | . | . | . | . | . | . | . | . | . | . | . | . | . | . | . | . | . |
| 21-225_165H2_LC | . | R | . | . | S | . | . | . | . | . | . | . | . | . | . | . | . | . | . | . | . | . | . | . | . | . |
| 21-225_190E2_LC | . | . | . | . | . | C | . | . | . | . | . | . | . | . | . | . | . | . | . | . | . | . | . | . | . | . |
| 21-225_190H4_LC | . | . | . | . | . | . | . | . | . | . | . | . | . | . | . | . | . | . | . | . | . | . | . | . | . | . |
| 21-225_190H6_LC | . | . | . | . | . | C | . | . | . | . | . | . | . | . | . | . | . | . | . | . | . | . | . | . | . | . |
| 21-225_191B12_LC | . | . | . | . | . | S | . | . | . | . | . | . | . | . | . | . | . | . | . | . | . | . | . | . | . | . |
| 21-225_191G3_LC | . | . | . | . | . | . | . | . | . | . | . | . | . | . | . | . | . | . | . | . | . | . | . | . | . | . |
| 21-225_195G11_LC | . | . | . | . | . | C | . | . | . | . | . | . | . | . | . | . | . | . | . | . | . | . | . | . | . | . |
| 21-225_196B1_LC | . | . | . | . | . | S | . | . | . | . | . | . | . | . | . | . | . | . | . | . | . | . | . | . | . | . |
| 21-225_196C1_LC | . | . | . | . | . | C | . | . | . | . | . | . | . | . | . | . | . | . | . | . | . | . | . | . | . | . |
| 21-225_197C8_LC | . | . | . | . | S | . | T | . | . | . | . | . | . | . | . | . | . | . | . | . | . | . | . | . | . | . |
| 21-225_203F10_LC | . | . | . | . | . | F | . | . | . | . | . | . | . | . | . | . | . | . | . | . | . | . | . | . | . | . |
| 21-225_225F4_LC | . | . | H | . | . | H | N | . | . | . | . | . | . | . | . | . | . | . | . | . | . | . | . | . | . | . |
| 21-225_4A2_LC | . | . | . | . | . | . | N | . | . | . | . | . | . | . | . | . | . | . | . | . | . | . | . | . | . | . |
| 21-225_4A2.001_LC | . | . | . | . | . | . | N | . | . | . | . | . | . | . | . | . | . | . | . | . | . | . | . | . | . | . |
| 21-225_4A2.001.001_LC | . | . | . | . | . | . | . | . | . | . | . | . | . | . | . | . | . | . | . | . | . | . | . | . | . | . |
| 21-225_4A2.001.002_LC | . | . | . | . | . | . | N | . | . | . | . | . | . | . | . | . | . | . | . | . | . | . | . | . | . | . |
| 21-225_4A2.001.003_LC | . | . | . | . | . | . | Q | . | . | . | . | . | . | . | . | . | . | . | . | . | . | . | . | . | . | . |
| 21-225_4A2.001.004_LC | . | . | . | . | . | . | N | . | . | . | . | . | . | . | . | . | . | . | . | . | . | . | . | . | . | . |
| 21-225_4A2.001.005_LC | . | . | . | . | . | . | N | . | . | . | . | . | . | . | . | . | . | . | . | . | . | . | . | . | . | . |
| 21-225_4A2.001.006_LC | . | . | . | . | . | . | N | . | . | . | . | . | . | . | . | . | . | . | . | . | . | . | . | . | . | . |
| 21-225_4A2.001.007_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | Y | S | - | - | - | - | - | - | - | - | - | - | - | - | - | - | - | - | - | - | - |
| 21-225_4A2.001.008_LC | . | . | . | . | . | . | . | . | . | . | . | . | . | . | . | . | . | . | . | . | . | . | . | . | . | . |
| 21-225_4A2.001.009_LC | . | . | . | . | . | . | N | . | . | . | . | . | . | . | . | . | . | . | . | . | . | . | . | . | . | . |
| 21-225_4A2.001.010_LC | . | . | . | . | . | . | Q | . | . | . | . | . | . | . | . | . | . | . | . | . | . | . | . | . | . | . |
| 21-225_4A2.001.011_LC | . | . | . | . | . | . | Q | . | . | . | . | . | . | . | . | . | . | . | . | . | . | . | . | . | . | . |
| 21-225_4A2.001.012_LC | . | . | . | . | . | . | Q | . | . | . | . | . | . | . | . | . | . | . | . | . | . | . | . | . | . | . |
| 21-225_4A2.001.022_LC | . | . | . | . | . | . | . | . | . | . | . | . | . | . | . | . | . | . | . | . | . | . | . | . | . | . |
| 21-225_4A2.001.024_LC | . | . | . | . | . | . | . | . | . | . | . | . | . | . | . | . | . | . | . | . | . | . | . | . | . | . |
| 21-225_6G6_LC | . | . | . | . | . | . | D | . | . | . | . | . | . | . | . | . | . | . | . | . | . | . | . | . | . | . |

# FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | T | P | V | T | F | G | P | G | T | K | V | D | I | K | R |
| 21-225_11E2_LC | . | . | . | . | . | . | F | . | . | . | . | . | . | . | . | . | . | . | . |
| 21-225_12F11_LC | . | . | . | . | . | . | F | . | . | . | . | . | . | . | . | . | . | . | . |
| 21-225_147G6_LC | . | . | . | . | . | . | P | . | . | . | . | . | . | . | . | . | . | . | . |
| 21-225_148G3_LC | . | . | . | . | . | . | F | . | . | . | . | . | . | . | . | . | . | . | . |
| 21-225_150B7_LC | . | . | . | . | . | . | F | . | . | . | . | . | . | . | . | . | . | . | . |
| 21-225_152E3_LC | . | . | . | . | . | . | F | . | . | . | . | . | . | . | . | . | . | . | . |
| 21-225_152F4_LC | . | . | . | . | . | . | P | . | . | . | . | . | . | . | . | . | . | . | . |
| 21-225_164B11_LC | . | . | . | . | I | . | P | . | . | . | H | . | . | N | . | . | . | T | . |
| 21-225_165H2_LC | . | . | . | . | I | . | P | . | . | . | . | . | . | N | . | . | . | . | . |
| 21-225_190E2_LC | . | . | . | . | L | . | F | . | . | . | . | . | . | . | . | . | . | R | . |
| 21-225_190H4_LC | . | . | . | . | I | . | P | . | . | . | . | . | . | . | . | . | . | . | . |
| 21-225_190H6_LC | . | . | . | . | L | . | F | . | . | . | . | . | . | . | . | . | . | R | . |
| 21-225_191B12_LC | . | . | . | . | L | . | F | . | . | . | . | . | . | . | . | . | . | . | . |
| 21-225_191G3_LC | . | . | . | . | S | . | P | . | . | . | . | . | . | . | M | . | . | . | . |
| 21-225_195G11_LC | . | . | . | . | F | . | F | . | . | . | . | . | . | . | . | . | . | R | . |
| 21-225_196B1_LC | . | . | . | . | L | . | F | . | . | . | . | . | . | . | . | . | . | . | . |
| 21-225_196C1_LC | . | . | . | . | F | . | F | . | . | . | . | . | . | . | . | . | . | R | . |
| 21-225_197C8_LC | . | . | . | . | I | . | F | . | . | . | . | . | . | . | . | . | N | . | . |
| 21-225_203F10_LC | . | . | . | . | L | . | F | . | . | . | . | . | . | . | . | . | . | T | . |
| 21-225_225F4_LC | . | . | . | . | S | . | P | . | . | . | H | . | . | E | . | . | . | . | . |
| 21-225_4A2_LC | . | . | . | . | . | . | . | . | . | . | . | . | . | . | . | G | . | . | . |
| 21-225_4A2.001_LC | . | . | . | . | . | . | . | . | . | . | . | . | . | . | . | G | . | . | . |
| 21-225_4A2.001.001_LC | . | . | . | . | . | . | . | . | . | . | . | . | . | . | . | G | . | . | . |
| 21-225_4A2.001.002_LC | . | . | . | . | A | . | . | . | . | . | . | . | . | . | . | G | . | . | . |
| 21-225_4A2.001.003_LC | . | . | . | . | . | . | . | . | . | . | . | . | . | . | . | G | . | . | . |
| 21-225_4A2.001.004_LC | . | . | . | . | . | . | . | . | . | . | . | . | . | . | . | G | . | . | . |
| 21-225_4A2.001.005_LC | . | . | . | . | . | . | . | . | . | . | . | . | . | . | . | G | . | . | . |
| 21-225_4A2.001.006_LC | . | . | . | . | . | . | . | . | . | . | . | . | . | . | . | G | . | . | . |
| 21-225_4A2.001.007_LC | . | . | . | . | . | . | . | . | . | . | . | . | . | . | . | G | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | T | P | V | T | F | G | P | G | T | K | V | D | I | K | R |
| 21-225_4A2.001.008_LC | . | . | . | . | . | . | . | . | . | . | . | . | . | . | . | G | . | . | . |
| 21-225_4A2.001.009_LC | . | . | . | . | A | . | . | . | . | . | . | . | . | . | . | G | . | . | . |
| 21-225_4A2.001.010_LC | . | . | . | . | . | . | . | . | . | . | . | . | . | . | . | G | . | . | . |
| 21-225_4A2.001.011_LC | . | . | . | . | . | . | . | . | . | . | . | . | . | . | . | G | . | . | . |
| 21-225_4A2.001.012_LC | . | . | . | . | . | . | . | . | . | . | . | . | . | . | . | G | . | . | . |
| 21-225_4A2.001.022_LC | . | . | . | . | . | . | . | . | . | . | . | . | . | . | . | G | . | . | . |
| 21-225_4A2.001.024_LC | . | . | . | . | . | . | . | . | . | . | . | . | . | . | . | G | . | . | . |
| 21-225_6G6_LC | . | . | . | . | . | . | F | . | . | . | H | . | . | . | . | . | . | . | . |

**FIGURE 57**

Table 110. Consensus 30 - VL7|7a/JL2 (SEQ ID NO: 50327):

QTVVTQE-PSLTVSPGGTVTLTCASST-GAVTSG----YYPNWFQQKPGQAPRALIYS--------TSNKHSWTPARFSGSLLG--
GKAALTLSGVQPEDEAEYYCLLYYG----------------------GAQLVFGGGTKLTVLG wherein:

A at position 24 can be substituted with V or G

S at position 25 can be substituted with F or L

S at position 26 can be substituted with N

G at position 29 can be substituted with E

A at position 30 can be substituted with S or T

G at position 34 can be substituted with A

Y at position 39 can be substituted with S, N or F

Y at position 40 can be substituted with F

N at position 42 can be substituted with S or Q

S at position 58 can be substituted with H or N

S at position 68 can be substituted with N, D, I or T

K at position 70 can be substituted with R

L at position 107 can be substituted with M

L at position 108 can be substituted with I or F

Y at position 109 can be substituted with F

Y at position 110 can be substituted with C, F or S

G at position 111 can be substituted with D

A at position 135 can be substituted with V

**FIGURE 57**

Q at position 136 can be substituted with H

L at position 137 can be substituted with V or M

V at position 138 can be substituted with A, I, G or M

Xaa Xaa Xaa Thr Xaa Xaa Val Thr Ser Xaa Xaa Xaa Pro Xaa (SEQ ID NO: 50165)

Xaa Thr Xaa Asn Xaa His Ser (SEQ ID NO: 50166)

Xaa Xaa Xaa Xaa Xaa Gly Xaa Xaa Xaa Xaa (SEQ ID NO: 50167)

Ala Ser Ser Thr Gly Ala Val Thr Ser Gly Tyr Tyr Pro Asn (SEQ ID NO: 50651)

Ser Thr Ser Asn Lys His Ser (SEQ ID NO: 50652)

Leu Leu Tyr Tyr Gly Gly Ala Gln Leu Val (SEQ ID NO: 50653)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | L_FR1 | | | | | | | | | | | | | | | | | | | | | | | | | |
| CONSENSUS | Q | T | V | V | T | Q | E | - | P | S | L | T | V | S | P | G | G | T | V | T | L | T | C | A | S | S |
| 21-225_190D1_LC | . | . | . | . | . | . | . | . | . | . | . | . | . | . | . | . | . | . | . | . | . | . | . | . | . | . |
| 21-225_190F10_LC | . | . | . | . | . | . | . | . | . | . | . | . | . | . | . | . | . | . | . | . | . | . | . | V | F | . |
| 21-225_191A2_LC | . | . | . | . | . | . | . | . | . | . | . | . | . | . | . | . | . | . | . | . | . | . | . | V | L | . |
| 21-225_191H9_LC | . | . | . | . | . | . | . | . | . | . | . | . | . | . | . | . | . | . | . | . | . | . | . | . | . | . |
| 21-225_192E3_LC | . | . | . | . | . | . | . | . | . | . | . | . | . | . | . | . | . | . | . | . | . | . | . | . | F | . |
| 21-225_192G7_LC | . | . | . | . | . | . | . | . | . | . | . | . | . | . | . | . | . | . | . | . | . | . | . | . | . | . |
| 21-225_192H8_LC | . | . | . | . | . | . | . | . | . | . | . | . | . | . | . | . | . | . | . | . | . | . | . | . | F | . |
| 21-225_194D12_LC | . | . | . | . | . | . | . | . | . | . | . | . | . | . | . | . | . | . | . | . | . | . | . | . | . | . |
| 21-225_194G5_LC | . | . | . | . | . | . | . | . | . | . | . | . | . | . | . | . | . | . | . | . | . | . | . | . | . | . |
| 21-225_196E7_LC | . | . | . | . | . | . | . | . | . | . | . | . | . | . | . | . | . | . | . | . | . | . | . | . | . | . |
| 21-225_197B11_LC | . | . | . | . | . | . | . | . | . | . | . | . | . | . | . | . | . | . | . | . | . | . | . | . | F | . |
| 21-225_197C11_LC | . | . | . | . | . | . | . | . | . | . | . | . | . | . | . | . | . | . | . | . | . | . | . | . | . | . |
| 21-225_198B8_LC | . | . | . | . | . | . | . | . | S | . | . | . | . | . | . | . | . | . | . | . | . | . | . | V | L | . |
| 21-225_200A11_LC | . | . | . | . | . | . | . | . | . | . | . | . | . | . | . | . | . | . | . | . | . | . | . | . | . | . |
| 21-225_200G9_LC | . | . | . | . | . | . | . | . | . | . | . | . | . | . | . | . | . | . | . | . | . | . | . | . | . | N |
| 21-225_200H1_LC | . | . | . | . | . | . | . | . | . | . | . | . | . | . | . | . | . | . | . | . | . | . | . | . | . | N |
| 21-225_210F11_LC | . | . | . | . | . | . | . | . | . | . | . | . | . | . | . | . | . | . | . | . | . | . | . | . | F | . |
| 21-225_211C9_LC | . | . | . | . | . | . | . | . | . | . | . | . | . | . | . | . | . | . | . | . | . | . | . | G | . | . |
| 21-225_211E9_LC | . | . | . | . | . | . | . | . | . | . | . | . | . | . | . | . | . | . | . | . | . | . | . | . | . | . |
| 21-225_211H7_LC | . | . | . | . | . | . | . | . | . | . | . | . | . | . | . | . | . | . | . | . | . | . | . | . | F | . |
| 21-225_212A9_LC | . | . | . | . | . | . | . | . | . | . | . | . | . | . | . | . | . | . | . | . | . | . | . | . | . | . |
| 21-225_212H12_LC | . | . | . | . | . | . | . | . | . | . | . | . | . | . | . | . | . | . | . | . | . | . | . | . | . | . |
| 21-225_213F5_LC | . | . | . | . | . | . | . | . | . | . | . | . | . | . | . | . | . | . | . | . | . | . | . | G | . | . |
| 21-225_214E12_LC | . | . | . | . | . | . | . | . | . | . | . | . | . | . | . | . | . | . | . | . | . | . | . | . | . | . |
| 21-225_214H3_LC | . | . | . | . | . | . | . | . | . | . | . | . | . | . | . | . | . | . | . | . | . | . | . | . | . | . |
| 21-225_215A3_LC | . | . | . | . | . | . | . | . | . | . | . | . | . | . | . | . | . | . | . | . | . | . | . | . | . | . |
| 21-225_215H3_LC | . | . | . | . | . | . | . | . | . | . | . | . | . | . | . | . | . | . | . | . | . | . | . | . | . | . |
| 21-225_216A7_LC | . | . | . | . | . | . | . | . | . | . | . | . | . | . | . | . | . | . | . | . | . | . | . | . | . | . |
| 21-225_216H11_LC | . | . | . | . | . | . | . | . | . | . | . | . | . | . | . | . | . | . | . | . | . | . | . | . | . | . |

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | L_FR1 | | | | | | | | | | | | | | | | | | | | | | | | | |
| CONSENSUS | Q | T | V | V | T | Q | E | - | P | S | L | T | V | S | P | G | G | T | V | T | L | T | C | A | S | S |
| 21-225_74E5_LC | . | . | . | . | . | . | . | . | . | . | . | . | . | . | . | . | . | . | . | . | . | . | . | . | . | . |
| 21-225_78D10_LC | . | . | . | . | . | . | . | . | . | . | . | . | . | . | . | . | . | . | . | . | . | . | . | . | . | . |

# FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | L_CDR1 | | | | | | | | | | | | | | | | L_FR2 | | | | | | | | | |
| CONSENSUS | T | - | G | A | V | T | S | G | - | - | - | - | Y | Y | P | N | W | F | Q | Q | K | P | G | Q | A | P |
| 21-225_190D1_LC | . | . | . | . | . | . | . | . | . | . | . | . | S | . | . | . | . | . | . | . | . | . | . | . | . | . |
| 21-225_190F10_LC | . | . | . | . | . | . | . | . | . | . | . | . | S | F | . | . | . | . | . | . | . | . | . | . | . | . |
| 21-225_191A2_LC | . | . | . | . | . | . | . | . | . | . | . | . | S | F | . | . | . | . | . | . | . | . | . | . | . | . |
| 21-225_191H9_LC | . | . | . | . | . | . | . | A | . | . | . | . | . | . | . | . | . | L | . | . | . | . | . | . | . | . |
| 21-225_192E3_LC | . | . | . | S | . | . | . | . | . | . | . | . | S | . | . | . | . | . | . | . | . | . | . | . | . | . |
| 21-225_192G7_LC | . | . | . | . | . | . | . | . | . | . | . | . | N | . | . | Q | . | . | . | . | . | . | . | . | . | . |
| 21-225_192H8_LC | . | . | . | T | . | . | . | . | . | . | . | . | F | . | . | . | . | . | . | . | . | . | . | . | . | . |
| 21-225_194D12_LC | . | . | . | . | . | . | . | . | . | . | . | . | S | F | . | S | . | . | . | . | . | . | . | . | . | . |
| 21-225_194G5_LC | . | . | . | . | . | . | . | . | . | . | . | . | N | . | . | . | . | . | . | . | . | . | . | . | T | . |
| 21-225_196E7_LC | . | . | . | . | . | . | . | A | . | . | . | . | . | . | . | . | . | L | . | . | . | . | . | . | . | . |
| 21-225_197B11_LC | . | . | . | S | . | . | . | . | . | . | . | . | S | . | . | . | . | . | . | . | . | . | . | . | . | . |
| 21-225_197C11_LC | . | . | . | . | . | . | . | A | . | . | . | . | . | . | . | . | . | L | . | . | . | . | . | . | . | . |
| 21-225_198B8_LC | . | . | . | . | . | . | . | . | . | . | . | . | S | F | . | . | . | . | . | . | . | . | . | . | . | . |
| 21-225_200A11_LC | . | . | . | . | . | . | . | . | . | . | . | . | . | . | . | . | . | . | . | . | . | . | . | . | V | . |
| 21-225_200G9_LC | . | . | . | . | . | . | . | . | . | . | . | . | S | . | . | . | . | L | . | . | . | . | . | . | . | . |
| 21-225_200H1_LC | . | . | . | . | . | . | . | . | . | . | . | . | S | . | . | . | . | . | . | . | . | . | . | . | . | . |
| 21-225_210F11_LC | . | . | . | . | . | . | . | . | . | . | . | . | N | . | . | . | . | . | . | . | . | . | . | . | . | . |
| 21-225_211C9_LC | . | . | . | S | . | . | . | . | . | . | . | . | . | F | . | . | . | . | . | . | . | . | . | . | . | . |
| 21-225_211E9_LC | . | . | . | . | . | . | . | . | . | . | . | . | N | . | . | . | . | . | . | . | . | . | . | . | . | . |
| 21-225_211H7_LC | . | . | . | . | . | . | . | . | . | . | . | . | N | . | . | S | . | . | . | . | . | . | . | . | V | . |
| 21-225_212A9_LC | . | . | . | . | . | . | . | . | . | . | . | . | . | . | . | . | . | . | . | . | . | . | . | . | . | . |
| 21-225_212H12_LC | . | . | . | . | . | . | . | . | . | . | . | . | . | . | . | . | . | . | . | . | . | . | . | . | . | . |
| 21-225_213F5_LC | . | . | . | S | . | . | . | . | . | . | . | . | . | F | . | . | . | . | . | . | . | . | . | . | T | . |
| 21-225_214E12_LC | . | . | . | . | . | . | . | . | . | . | . | . | . | . | . | . | . | . | . | . | . | . | . | . | . | . |
| 21-225_214H3_LC | . | . | . | . | . | . | . | . | . | . | . | . | . | . | . | . | . | . | . | . | . | . | . | . | . | . |
| 21-225_215A3_LC | . | . | E | . | . | . | . | . | . | . | . | . | N | . | . | S | . | . | . | . | . | . | . | . | . | . |
| 21-225_215H3_LC | . | . | . | . | . | . | . | . | . | . | . | . | . | . | . | . | . | . | . | . | . | . | . | . | . | . |
| 21-225_216A7_LC | . | . | . | . | . | . | . | . | . | . | . | . | . | . | . | . | . | . | . | . | . | . | . | . | . | . |
| 21-225_216H11_LC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | L_CDR1 | | | | | | | | | | | | | | | | L_FR2 | | | | | | | | | |
| CONSENSUS | T | - | G | A | V | T | S | G | - | - | - | - | Y | Y | P | N | W | F | Q | Q | K | P | G | Q | A | P |
| 21-225_74E5_LC | . | . | E | T | . | . | . | . | . | . | . | . | S | . | . | . | . | . | . | . | . | . | . | . | . | . |
| 21-225_78D10_LC | . | . | . | . | . | . | . | . | . | . | . | . | . | F | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | L_CDR2 | | | | | | | | | | | | | | | | | | | | |
| CONSENSUS | R | A | L | I | Y | S | - | - | - | - | - | - | - | - | T | S | N | K | H | S | W | T | P | A | R | F |
| 21-225_190D1_LC | . | . | . | . | . | . | . | . | . | . | . | . | . | . | . | . | . | . | . | . | . | . | . | . | . | . |
| 21-225_190F10_LC | . | . | . | . | . | . | . | . | . | . | . | . | . | . | . | . | . | . | . | . | . | . | . | . | . | . |
| 21-225_191A2_LC | . | . | . | . | . | . | . | . | . | . | . | . | . | . | . | . | . | . | . | . | . | . | . | . | . | . |
| 21-225_191H9_LC | . | T | . | . | . | . | . | . | . | . | . | . | . | . | . | N | . | . | . | . | . | . | . | . | . | . |
| 21-225_192E3_LC | . | . | . | . | . | . | . | . | . | . | . | . | . | . | . | N | . | . | . | . | . | . | . | . | . | . |
| 21-225_192G7_LC | . | . | . | . | . | . | . | . | . | . | . | . | . | . | . | T | . | R | . | . | . | . | . | . | . | . |
| 21-225_192H8_LC | . | . | . | . | . | N | . | . | . | . | . | . | . | . | . | . | . | R | . | . | . | . | . | . | . | . |
| 21-225_194D12_LC | . | . | . | . | . | . | . | . | . | . | . | . | . | . | . | . | . | R | . | . | S | . | . | . | . | . |
| 21-225_194G5_LC | . | . | . | . | . | . | . | . | . | . | . | . | . | . | . | . | . | . | . | . | . | . | . | . | . | . |
| 21-225_196E7_LC | . | T | . | . | . | . | . | . | . | . | . | . | . | . | . | N | . | . | . | . | . | . | . | . | . | . |
| 21-225_197B11_LC | . | . | . | . | . | . | . | . | . | . | . | . | . | . | . | N | . | . | . | . | . | . | . | . | . | . |
| 21-225_197C11_LC | . | T | . | . | . | . | . | . | . | . | . | . | . | . | . | . | . | . | . | . | . | . | . | . | . | . |
| 21-225_198B8_LC | . | . | . | . | . | . | . | . | . | . | . | . | . | . | . | . | . | . | . | . | . | . | . | . | . | . |
| 21-225_200A11_LC | . | . | . | . | . | . | . | . | . | . | . | . | . | . | . | N | . | . | . | . | . | . | . | . | . | . |
| 21-225_200G9_LC | . | . | . | . | . | H | . | . | . | . | . | . | . | . | . | D | . | . | . | . | . | . | . | . | . | . |
| 21-225_200H1_LC | . | . | . | . | . | H | . | . | . | . | . | . | . | . | . | N | . | . | . | . | . | . | . | . | . | . |
| 21-225_210F11_LC | . | . | . | . | . | . | . | . | . | . | . | . | . | . | . | . | . | . | . | . | . | . | . | . | . | . |
| 21-225_211C9_LC | . | P | . | . | . | . | . | . | . | . | . | . | . | . | . | N | . | . | . | . | . | . | . | . | . | . |
| 21-225_211E9_LC | . | . | . | . | . | . | . | . | . | . | . | . | . | . | . | I | . | . | . | . | G | . | . | . | . | . |
| 21-225_211H7_LC | . | . | . | . | . | . | . | . | . | . | . | . | . | . | . | . | . | R | . | . | . | . | . | . | . | . |
| 21-225_212A9_LC | . | . | . | . | . | . | . | . | . | . | . | . | . | . | . | N | . | . | . | . | . | . | . | . | . | . |
| 21-225_212H12_LC | . | . | . | . | . | . | . | . | . | . | . | . | . | . | . | . | . | . | . | . | . | . | . | . | . | . |
| 21-225_213F5_LC | . | P | . | . | . | . | . | . | . | . | . | . | . | . | . | N | . | . | . | . | . | . | . | . | . | . |
| 21-225_214E12_LC | . | . | . | . | . | . | . | . | . | . | . | . | . | . | . | . | . | . | . | . | . | . | . | . | . | . |
| 21-225_214H3_LC | . | . | . | . | . | . | . | . | . | . | . | . | . | . | . | . | . | . | . | . | C | . | . | . | . | . |
| 21-225_215A3_LC | . | . | . | . | . | . | . | . | . | . | . | . | . | . | . | . | . | . | . | . | G | . | . | . | . | . |
| 21-225_215H3_LC | . | . | . | . | . | . | . | . | . | . | . | . | . | . | . | N | . | . | . | . | C | G | . | . | . | . |
| 21-225_216A7_LC | . | . | . | . | . | . | . | . | . | . | . | . | . | . | . | N | . | . | . | . | C | . | . | . | . | . |
| 21-225_216H11_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | L_CDR2 | | | | | | | | | | | | | | | | | | | | |
| CONSENSUS | R | A | L | I | Y | S | - | - | - | - | - | - | - | - | T | S | N | K | H | S | W | T | P | A | R | F |
| 21-225_74E5_LC | . | . | . | . | . | . | . | . | . | . | . | . | . | . | . | . | . | . | . | . | . | . | . | . | . | . |
| 21-225_78D10_LC | . | . | . | . | . | . | . | . | . | . | . | . | . | . | . | D | . | . | . | . | . | . | . | . | . | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | L_FR3 | | | | | | | | | | | | | | | | | | | | | | | | | |
| CONSENSUS | S | G | S | L | L | G | - | - | G | K | A | A | L | T | L | S | G | V | Q | P | E | D | E | A | E | Y |
| 21-225_190D1_LC | . | . | . | . | . | . | . | . | . | . | . | . | . | . | . | . | . | . | . | . | . | . | . | . | D | . |
| 21-225_190F10_LC | . | . | . | . | . | . | . | . | . | . | . | . | . | . | . | . | . | . | . | . | . | . | . | . | D | . |
| 21-225_191A2_LC | . | . | . | . | . | . | . | . | . | . | . | . | . | . | . | . | . | . | . | . | . | . | . | . | D | . |
| 21-225_191H9_LC | . | . | . | . | . | . | . | . | . | . | . | . | . | . | . | . | D | . | . | . | . | . | . | . | . | . |
| 21-225_192E3_LC | . | . | . | . | . | . | . | . | . | . | . | . | . | . | . | . | . | . | . | R | . | . | . | . | . | . |
| 21-225_192G7_LC | . | . | . | . | . | . | . | . | . | . | . | . | . | . | . | . | . | . | . | . | . | . | . | . | . | . |
| 21-225_192H8_LC | . | . | . | . | . | . | . | . | . | M | . | . | . | . | . | . | . | . | . | . | . | . | . | . | D | . |
| 21-225_194D12_LC | . | . | . | . | . | . | . | . | . | . | . | . | . | . | . | . | . | . | . | . | . | . | . | . | D | . |
| 21-225_194G5_LC | . | . | . | . | . | . | . | . | . | . | G | . | . | . | . | . | . | . | . | . | . | . | . | . | . | . |
| 21-225_196E7_LC | . | . | . | . | . | . | . | . | . | . | . | . | . | . | . | . | D | . | . | . | . | . | . | . | . | . |
| 21-225_197B11_LC | . | . | . | . | . | . | . | . | . | . | . | . | . | . | . | . | . | . | . | . | . | . | . | . | . | . |
| 21-225_197C11_LC | . | . | . | . | . | . | . | . | . | . | . | . | . | . | . | . | D | . | . | . | . | . | . | . | . | . |
| 21-225_198B8_LC | . | . | . | . | . | . | . | . | . | . | . | . | . | . | . | . | . | . | . | . | . | . | . | . | D | . |
| 21-225_200A11_LC | . | . | . | . | . | . | . | . | . | . | . | . | . | . | . | . | . | . | . | . | . | . | . | . | D | . |
| 21-225_200G9_LC | . | . | . | . | . | . | . | . | . | . | . | . | . | . | . | . | . | A | . | . | . | . | . | . | . | . |
| 21-225_200H1_LC | . | . | . | . | . | . | . | . | . | . | . | . | . | . | . | . | . | . | . | . | . | . | . | . | . | . |
| 21-225_210F11_LC | . | . | . | . | . | . | . | . | . | . | . | . | . | . | . | . | . | . | . | . | . | . | . | . | . | . |
| 21-225_211C9_LC | . | . | . | . | . | . | . | . | . | . | . | . | . | . | . | . | D | . | . | . | . | . | . | . | D | . |
| 21-225_211E9_LC | T | . | F | . | . | . | . | . | . | . | . | . | . | . | . | . | . | . | . | . | . | . | . | . | . | . |
| 21-225_211H7_LC | . | . | . | . | . | . | . | . | . | . | . | . | . | . | . | . | . | . | . | . | . | . | . | . | . | . |
| 21-225_212A9_LC | . | . | . | . | . | . | . | . | . | . | . | . | . | . | . | . | . | . | . | . | D | . | . | . | D | . |
| 21-225_212H12_LC | . | . | . | . | . | . | . | . | . | . | . | . | . | . | . | . | . | . | . | . | . | . | . | . | . | . |
| 21-225_213F5_LC | . | . | . | . | . | . | . | . | . | . | T | . | . | . | . | . | D | . | . | . | . | . | . | . | D | . |
| 21-225_214E12_LC | . | . | . | . | . | . | . | . | . | . | . | . | . | . | . | . | D | . | . | . | . | . | . | . | . | . |
| 21-225_214H3_LC | . | . | . | . | . | . | . | . | . | . | . | . | . | . | . | . | . | . | . | . | . | . | . | . | . | . |
| 21-225_215A3_LC | T | . | . | . | . | . | . | . | . | . | . | . | . | . | . | . | . | . | . | . | . | . | . | . | . | . |
| 21-225_215H3_LC | . | . | . | . | . | . | . | . | . | . | . | . | . | . | . | . | . | . | . | . | D | . | . | . | D | . |
| 21-225_216A7_LC | . | . | . | . | . | . | . | . | . | . | . | . | . | . | . | . | . | . | . | . | D | . | . | . | D | . |
| 21-225_216H11_LC | . | . | . | . | . | . | . | . | . | . | . | . | . | . | . | . | D | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | L_FR3 | | | | | | | | | | | | | | | | | | | | | | | | | |
| CONSENSUS | S | G | S | L | L | G | - | - | G | K | A | A | L | T | L | S | G | V | Q | P | E | D | E | A | E | Y |
| 21-225_74E5_LC | . | . | . | . | . | . | . | . | . | . | . | . | . | . | . | . | D | . | . | . | . | . | . | . | . | . |
| 21-225_78D10_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | L_CDR3 | | | | | | | | | | | | | | | | | | | | | | | | |
| CONSENSUS | Y | C | L | L | Y | Y | G | - | - | - | - | - | - | - | - | - | - | - | - | - | - | - | - | - | - | - |
| 21-225_190D1_LC | . | . | . | . | . | . | . | . | . | . | . | . | . | . | . | . | . | . | . | . | . | . | . | . | . | . |
| 21-225_190F10_LC | . | . | . | . | . | C | . | . | . | . | . | . | . | . | . | . | . | . | . | . | . | . | . | . | . | . |
| 21-225_191A2_LC | . | . | M | . | . | C | . | . | . | . | . | . | . | . | . | . | . | . | . | . | . | . | . | . | . | . |
| 21-225_191H9_LC | . | . | . | I | F | . | . | . | . | . | . | . | . | . | . | . | . | . | . | . | . | . | . | . | . | . |
| 21-225_192E3_LC | . | . | . | . | . | . | . | . | . | . | . | . | . | . | . | . | . | . | . | . | . | . | . | . | . | . |
| 21-225_192G7_LC | . | . | . | F | . | . | . | . | . | . | . | . | . | . | . | . | . | . | . | . | . | . | . | . | . | . |
| 21-225_192H8_LC | . | . | . | . | . | . | . | . | . | . | . | . | . | . | . | . | . | . | . | . | . | . | . | . | . | . |
| 21-225_194D12_LC | . | . | . | I | . | . | . | . | . | . | . | . | . | . | . | . | . | . | . | . | . | . | . | . | . | . |
| 21-225_194G5_LC | . | . | . | I | . | . | . | . | . | . | . | . | . | . | . | . | . | . | . | . | . | . | . | . | . | . |
| 21-225_196E7_LC | . | . | . | I | F | . | . | . | . | . | . | . | . | . | . | . | . | . | . | . | . | . | . | . | . | . |
| 21-225_197B11_LC | . | . | . | . | . | . | . | . | . | . | . | . | . | . | . | . | . | . | . | . | . | . | . | . | . | . |
| 21-225_197C11_LC | . | . | . | I | F | . | . | . | . | . | . | . | . | . | . | . | . | . | . | . | . | . | . | . | . | . |
| 21-225_198B8_LC | . | F | M | . | . | S | . | . | . | . | . | . | . | . | . | . | . | . | . | . | . | . | . | . | . | . |
| 21-225_200A11_LC | . | . | . | . | . | . | . | . | . | . | . | . | . | . | . | . | . | . | . | . | . | . | . | . | . | . |
| 21-225_200G9_LC | . | . | . | I | . | . | . | . | . | . | . | . | . | . | . | . | . | . | . | . | . | . | . | . | . | . |
| 21-225_200H1_LC | . | . | . | I | . | . | . | . | . | . | . | . | . | . | . | . | . | . | . | . | . | . | . | . | . | . |
| 21-225_210F11_LC | . | . | . | . | . | . | . | . | . | . | . | . | . | . | . | . | . | . | . | . | . | . | . | . | . | . |
| 21-225_211C9_LC | . | . | . | . | . | . | . | . | . | . | . | . | . | . | . | . | . | . | . | . | . | . | . | . | . | . |
| 21-225_211E9_LC | . | . | . | . | . | . | . | . | . | . | . | . | . | . | . | . | . | . | . | . | . | . | . | . | . | . |
| 21-225_211H7_LC | . | . | . | . | . | . | . | . | . | . | . | . | . | . | . | . | . | . | . | . | . | . | . | . | . | . |
| 21-225_212A9_LC | . | . | . | . | . | . | . | . | . | . | . | . | . | . | . | . | . | . | . | . | . | . | . | . | . | . |
| 21-225_212H12_LC | . | . | . | . | . | . | . | . | . | . | . | . | . | . | . | . | . | . | . | . | . | . | . | . | . | . |
| 21-225_213F5_LC | . | . | . | . | . | F | . | . | . | . | . | . | . | . | . | . | . | . | . | . | . | . | . | . | . | . |
| 21-225_214E12_LC | . | . | . | . | . | C | D | . | . | . | . | . | . | . | . | . | . | . | . | . | . | . | . | . | . | . |
| 21-225_214H3_LC | . | . | . | . | . | . | . | . | . | . | . | . | . | . | . | . | . | . | . | . | . | . | . | . | . | . |
| 21-225_215A3_LC | . | . | . | . | . | . | . | . | . | . | . | . | . | . | . | . | . | . | . | . | . | . | . | . | . | . |
| 21-225_215H3_LC | . | . | . | . | . | . | . | . | . | . | . | . | . | . | . | . | . | . | . | . | . | . | . | . | . | . |
| 21-225_216A7_LC | . | . | . | . | . | . | . | . | . | . | . | . | . | . | . | . | . | . | . | . | . | . | . | . | . | . |
| 21-225_216H11_LC | . | . | . | . | . | C | D | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | L_CDR3 | | | | | | | | | | | | | | | | | | | | | | | |
| CONSENSUS | Y | C | L | L | Y | Y | G | - | - | - | - | - | - | - | - | - | - | - | - | - | - | - | - | - | - | - |
| 21-225_74E5_LC | . | . | . | . | . | . | . | . | . | . | . | . | . | . | . | . | . | . | . | . | . | . | . | . | . | . |
| 21-225_78D10_LC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | L_FR4 | | | | | | | | | | |
| CONSENSUS | - | - | - | G | A | Q | L | V | F | G | G | G | T | K | L | T | V | L | G |
| 21-225_190D1_LC | . | . | . | . | . | . | V | A | . | . | . | . | . | . | . | . | . | . | . |
| 21-225_190F10_LC | . | . | . | . | . | . | . | . | . | . | . | . | . | . | . | . | . | . | . |
| 21-225_191A2_LC | . | . | . | . | . | . | . | . | . | . | . | . | . | . | . | . | . | . | . |
| 21-225_191H9_LC | . | . | . | . | V | H | V | I | . | . | . | . | . | . | . | . | . | . | . |
| 21-225_192E3_LC | . | . | . | . | . | . | . | . | . | . | . | . | . | . | . | . | . | . | . |
| 21-225_192G7_LC | . | . | . | . | . | . | V | I | . | . | . | . | . | . | . | . | . | . | . |
| 21-225_192H8_LC | . | . | . | . | . | . | . | M | . | . | . | . | . | . | . | . | . | . | . |
| 21-225_194D12_LC | . | . | . | . | . | . | . | A | . | . | . | . | . | . | . | . | . | . | S |
| 21-225_194G5_LC | . | . | . | . | . | . | . | M | . | . | . | . | . | . | . | . | . | . | . |
| 21-225_196E7_LC | . | . | . | . | V | H | V | I | . | . | . | . | . | . | . | . | . | . | . |
| 21-225_197B11_LC | . | . | . | . | . | . | . | . | . | . | . | . | . | . | . | . | . | . | . |
| 21-225_197C11_LC | . | . | . | . | V | H | V | I | . | . | . | . | . | . | . | . | . | . | . |
| 21-225_198B8_LC | . | . | . | . | . | . | M | . | . | . | . | . | . | . | . | . | . | . | . |
| 21-225_200A11_LC | . | . | . | . | . | H | . | A | . | . | . | . | . | . | . | . | . | . | . |
| 21-225_200G9_LC | . | . | . | . | . | . | . | . | . | . | . | . | . | . | . | . | . | . | . |
| 21-225_200H1_LC | . | . | . | . | . | . | . | . | . | . | . | . | . | . | . | . | . | . | . |
| 21-225_210F11_LC | . | . | . | . | . | . | . | . | . | . | . | . | . | . | . | . | . | . | . |
| 21-225_211C9_LC | . | . | . | . | . | . | . | A | . | . | . | . | . | . | . | . | . | . | . |
| 21-225_211E9_LC | . | . | . | . | . | . | . | A | . | . | . | . | . | . | . | . | . | . | . |
| 21-225_211H7_LC | . | . | . | . | . | . | . | . | . | . | . | . | . | . | . | . | . | . | . |
| 21-225_212A9_LC | . | . | . | . | . | . | V | G | . | . | . | . | . | . | . | . | . | . | S |
| 21-225_212H12_LC | . | . | . | . | . | H | V | . | . | . | . | . | . | . | . | . | . | . | . |
| 21-225_213F5_LC | . | . | . | . | . | . | . | A | . | . | . | . | . | . | . | . | . | . | . |
| 21-225_214E12_LC | . | . | . | . | . | . | . | . | . | . | . | . | . | . | . | . | . | . | . |
| 21-225_214H3_LC | . | . | . | . | . | H | V | . | . | . | . | . | . | . | . | . | . | . | . |
| 21-225_215A3_LC | . | . | . | . | . | . | . | A | . | . | . | . | . | . | . | A | . | . | . |
| 21-225_215H3_LC | . | . | . | . | . | . | V | G | . | . | . | . | . | . | . | . | . | . | S |
| 21-225_216A7_LC | . | . | . | . | . | . | V | G | . | . | . | . | . | . | . | . | . | . | S |
| 21-225_216H11_LC | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | L_FR4 | | | | | | | | | | |
| CONSENSUS | - | - | - | G | A | Q | L | V | F | G | G | G | T | K | L | T | V | L | G |
| 21-225_74E5_LC | . | . | . | . | . | . | . | . | . | . | . | . | . | . | . | . | . | . | . |
| 21-225_78D10_LC | . | . | . | . | . | . | . | | . | . | . | . | . | . | . | . | . | . | . |

**FIGURE 57**

Table 111. Consensus 31 - VK3|L2/JK2 (SEQ ID NO: 50328):

EIVMTQSPATLSVSPGERATLSCRAS--QSVN------SNLAWYQQKPGQAPRLLIYG--------ASTRATGIPARFSGSGSG--TEFTLTISSLQSEDFAVYYCQQYND-----------------------WPCSFGQGTKLEIKR wherein:

A at position 25 can be substituted with S

Q at position 29 can be substituted with L, M or V

S at position 30 can be substituted with N, D, R or T

V at position 31 can be substituted with I

N at position 32 can be substituted with K, S, V, A, I or L

S at position 39 can be substituted with N or T

N at position 40 can be substituted with S or Y

G at position 58 can be substituted with I, F or V

A at position 67 can be substituted with T

T at position 69 can be substituted with I

Q at position 108 can be substituted with E

Y at position 109 can be substituted with F

N at position 110 can be substituted with Y or D

D at position 111 can be substituted with N

Null (-) at position 134 can be substituted with W

W at position 135 can be substituted with P

P at position 136 can be substituted with L or M

**FIGURE 57**

Arg Xaa Ser Xaa Xaa Xaa Xaa Xaa Xaa Leu Ala (SEQ ID NO: 50168)

Xaa Xaa Ser Xaa Arg Ala Thr (SEQ ID NO: 50169)

Gln Xaa Xaa Xaa Xaa Xaa Xaa Xaa Cys Ser (SEQ ID NO: 50170)

Arg Ala Ser Gln Ser Val Asn Ser Asn Leu Ala (SEQ ID NO: 50654)

Gly Ala Ser Thr Arg Ala Thr (SEQ ID NO: 50655)

Gln Gln Tyr Asn Asp Trp Pro Cys Ser (SEQ ID NO: 50656)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | E | I | V | M | T | Q | S | P | A | T | L | S | V | S | P | G | E | R | A | T | L | S | C | R | A | S |
| 21-225_162A10_LC | . | . | . | . | . | . | . | . | . | . | . | . | . | . | . | . | . | . | . | . | . | . | . | . | . | . |
| 21-225_193G12_LC | . | . | . | . | . | . | . | . | . | . | . | . | . | . | . | . | . | . | . | . | . | . | . | . | . | . |
| 21-225_201A4_LC | . | . | . | . | . | . | . | . | . | . | . | . | . | . | . | . | . | S | . | . | . | . | . | . | S | . |
| 21-225_201F2_LC | . | . | . | . | . | . | . | . | . | . | . | . | . | . | . | . | . | . | . | . | . | . | . | . | . | . |
| 21-225_201F7_LC | . | . | . | . | . | . | . | . | . | . | . | . | . | . | . | . | . | . | . | . | . | . | . | . | . | . |
| 21-225_201H4_LC | . | . | . | . | . | . | . | . | . | . | . | . | . | . | . | . | . | . | . | . | . | . | . | . | . | . |
| 21-225_202A8_LC | . | . | . | . | . | . | . | . | . | . | . | . | . | . | . | . | . | . | . | . | . | . | . | . | . | . |
| 21-225_202F12_LC | . | . | . | . | . | . | . | . | . | . | . | . | . | . | . | . | . | . | . | . | . | . | . | . | . | . |
| 21-225_205G4_LC | . | . | . | . | . | . | . | . | . | . | . | . | . | . | . | . | . | . | . | . | . | . | . | . | . | . |
| 21-225_210H10_LC | . | . | . | . | . | . | . | . | . | . | . | . | . | . | . | . | . | . | . | . | . | . | . | . | . | . |
| 21-225_212C10_LC | . | . | . | . | . | . | . | . | . | . | . | . | . | . | . | . | . | . | . | . | . | . | . | . | . | . |
| 21-225_216B10_LC | . | . | . | . | . | E | . | . | . | . | . | . | . | . | . | . | . | . | . | . | . | . | . | . | . | . |
| 21-225_24H3_LC | . | . | . | . | . | . | . | . | . | . | . | . | . | . | . | . | . | . | . | . | . | . | . | . | . | . |
| 21-225_57H3_LC | . | . | . | . | . | . | . | . | . | . | . | . | . | . | . | . | . | . | . | . | . | . | . | . | . | . |
| 21-225_62E6_LC | . | . | . | . | . | . | . | . | . | . | . | . | . | . | . | . | . | . | . | . | . | . | . | . | . | . |
| 21-225_65A6_LC | . | . | . | . | . | . | . | . | V | . | . | F | . | . | . | . | . | . | . | . | . | . | . | . | . | . |
| 21-225_67C3_LC | . | . | . | . | . | . | . | . | . | . | . | . | . | . | . | . | . | . | . | . | . | . | . | . | . | . |
| 21-225_70A5_LC | . | . | . | . | . | . | . | . | . | . | . | . | . | . | . | . | . | . | . | . | . | . | . | . | . | . |
| 21-225_74C3_LC | . | . | . | . | . | . | . | . | . | . | . | . | . | . | . | . | . | . | . | . | . | . | . | . | . | . |
| 21-225_74E11_LC | . | . | . | . | . | . | . | . | . | . | . | . | . | . | . | . | . | . | . | . | . | . | . | . | . | . |
| 21-225_74E3_LC | . | . | . | . | . | . | . | . | . | . | . | . | L | . | . | . | . | . | . | . | . | . | . | . | . | . |
| 21-225_76D2_LC | . | . | . | . | . | . | . | . | . | . | P | . | . | . | . | . | . | . | . | . | . | . | . | . | . | . |
| 21-225_77H5_LC | . | . | . | . | . | . | . | . | V | . | . | . | . | . | . | . | . | . | . | . | . | . | . | . | . | . |
| 21-225_92H5_LC | . | . | . | . | . | . | . | . | . | . | . | . | . | . | . | . | . | . | . | . | . | . | . | . | . | . |
| 21-225_95G2_LC | . | . | . | . | . | . | . | . | . | . | . | . | L | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | V | N | - | - | - | - | - | - | S | N | L | A | W | Y | Q | Q | K | P | G | Q | A | P |
| 21-225_162A10_LC | . | . | . | T | . | . | . | . | . | . | . | . | . | . | . | . | . | . | . | . | . | S | . | . | . | . |
| 21-225_193G12_LC | . | . | . | . | . | S | . | . | . | . | . | . | . | S | . | . | . | . | . | . | . | . | . | . | . | . |
| 21-225_201A4_LC | . | . | . | N | I | K | . | . | . | . | . | . | . | . | . | . | . | . | . | . | . | . | . | . | . | . |
| 21-225_201F2_LC | . | . | . | N | I | K | . | . | . | . | . | . | . | . | . | . | . | . | . | . | . | . | . | . | . | . |
| 21-225_201F7_LC | . | . | . | N | I | K | . | . | . | . | . | . | N | . | . | . | . | . | . | . | . | . | . | . | . | . |
| 21-225_201H4_LC | . | . | . | . | . | . | . | . | . | . | . | . | . | Y | . | . | . | . | . | . | . | . | . | . | . | . |
| 21-225_202A8_LC | . | . | . | R | I | . | . | . | . | . | . | . | N | . | . | . | . | . | . | . | N | . | . | . | . | . |
| 21-225_202F12_LC | . | . | . | . | . | L | . | . | . | . | . | . | N | . | . | . | . | . | . | . | R | . | . | . | . | . |
| 21-225_205G4_LC | . | . | . | N | I | K | . | . | . | . | . | . | . | . | . | . | . | . | . | . | . | . | . | . | . | . |
| 21-225_210H10_LC | . | . | . | . | . | K | . | . | . | . | . | . | . | . | . | . | . | . | . | . | . | . | . | . | . | . |
| 21-225_212C10_LC | . | . | . | . | . | A | . | . | . | . | . | . | . | S | . | . | . | . | . | . | . | . | . | . | . | . |
| 21-225_216B10_LC | . | . | . | . | . | . | . | . | . | . | . | . | N | . | . | . | . | . | R | . | . | . | . | . | . | . |
| 21-225_24H3_LC | . | . | . | . | . | S | . | . | . | . | . | . | . | . | . | . | . | . | . | . | . | . | . | . | . | . |
| 21-225_57H3_LC | . | . | . | . | . | S | . | . | . | . | . | . | . | S | . | . | . | . | . | . | . | . | . | . | . | . |
| 21-225_62E6_LC | . | . | . | . | . | . | . | . | . | . | . | . | . | . | . | . | . | . | . | . | . | . | . | . | . | . |
| 21-225_65A6_LC | . | . | . | . | . | . | . | . | . | . | . | . | . | . | . | . | . | . | . | . | . | . | . | . | . | . |
| 21-225_67C3_LC | . | . | . | . | . | . | . | . | . | . | . | . | . | . | . | . | . | . | . | . | . | . | . | . | . | . |
| 21-225_70A5_LC | . | . | . | . | . | . | . | . | . | . | . | . | . | . | . | . | . | . | . | L | . | . | . | . | . | . |
| 21-225_74C3_LC | . | . | . | . | . | A | . | . | . | . | . | . | . | S | . | . | . | . | . | . | . | . | . | . | . | . |
| 21-225_74E11_LC | . | . | L | . | . | V | . | . | . | . | . | . | N | S | . | . | . | . | . | . | . | . | . | . | . | . |
| 21-225_74E3_LC | . | . | L | . | . | V | . | . | . | . | . | . | N | S | . | . | . | . | . | . | . | . | . | . | . | . |
| 21-225_76D2_LC | . | . | V | . | . | V | . | . | . | . | . | . | N | S | . | . | . | . | . | . | . | . | . | . | . | . |
| 21-225_77H5_LC | . | . | . | . | . | S | . | . | . | . | . | . | . | S | . | . | . | . | . | . | . | . | . | L | . | . |
| 21-225_92H5_LC | . | . | . | D | . | I | . | . | . | . | . | . | T | Y | . | . | . | . | . | . | . | . | . | . | . | . |
| 21-225_95G2_LC | . | . | M | . | . | V | . | . | . | . | . | . | N | S | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | R | L | L | I | Y | G | - | - | - | - | - | - | - | - | A | S | T | R | A | T | G | I | P | A | R | F |
| 21-225_162A10_LC | . | . | . | . | F | . | . | . | . | . | . | . | . | . | . | . | . | . | . | . | V | . | . | . | . | I |
| 21-225_193G12_LC | . | I | . | . | S | . | . | . | . | . | . | . | . | . | . | . | . | . | . | . | . | . | . | . | . | . |
| 21-225_201A4_LC | . | . | . | . | F | . | . | . | . | . | . | . | . | . | . | . | . | . | . | . | . | . | . | . | . | . |
| 21-225_201F2_LC | . | . | . | . | F | . | . | . | . | . | . | . | . | . | . | . | . | . | . | . | . | . | . | . | . | . |
| 21-225_201F7_LC | . | . | . | . | F | . | . | . | . | . | . | . | . | . | . | . | . | . | . | . | . | . | . | . | . | . |
| 21-225_201H4_LC | . | . | . | . | . | . | . | . | . | . | . | . | . | . | . | . | . | . | . | . | . | . | . | . | . | . |
| 21-225_202A8_LC | . | . | . | . | . | . | . | . | . | . | . | . | . | . | . | . | . | . | . | . | . | V | . | . | . | . |
| 21-225_202F12_LC | . | . | . | . | . | . | . | . | . | . | . | . | . | . | . | . | . | . | . | . | . | . | . | . | . | . |
| 21-225_205G4_LC | . | . | . | . | F | . | . | . | . | . | . | . | . | . | . | . | . | . | . | . | . | . | . | . | . | . |
| 21-225_210H10_LC | . | . | . | . | . | . | . | . | . | . | . | . | . | . | . | . | . | . | . | . | . | . | . | . | . | . |
| 21-225_212C10_LC | . | . | . | . | H | . | . | . | . | . | . | . | . | . | T | . | . | . | . | . | D | V | . | . | . | . |
| 21-225_216B10_LC | . | . | . | . | . | . | . | . | . | . | . | . | . | . | . | . | . | . | . | . | . | . | . | P | . | . |
| 21-225_24H3_LC | . | . | . | . | . | F | . | . | . | . | . | . | . | . | . | . | I | . | . | . | . | . | . | . | . | . |
| 21-225_57H3_LC | . | . | . | . | . | . | . | . | . | . | . | . | . | . | . | . | . | . | . | . | . | F | . | . | . | . |
| 21-225_62E6_LC | . | . | . | . | S | V | . | . | . | . | . | . | . | . | . | . | . | . | . | . | . | . | . | . | . | . |
| 21-225_65A6_LC | . | . | . | . | . | . | . | . | . | . | . | . | . | . | . | . | . | . | . | . | . | . | . | . | . | . |
| 21-225_67C3_LC | . | . | . | . | S | I | . | . | . | . | . | . | . | . | . | . | . | . | . | . | . | . | . | . | . | . |
| 21-225_70A5_LC | . | . | . | . | S | I | . | . | . | . | . | . | . | . | . | . | . | . | . | . | . | . | . | P | . | . |
| 21-225_74C3_LC | . | . | . | . | F | . | . | . | . | . | . | . | . | . | T | . | . | . | . | . | . | . | . | . | . | . |
| 21-225_74E11_LC | . | . | . | . | H | . | . | . | . | . | . | . | . | . | . | . | . | . | . | . | . | . | . | . | . | . |
| 21-225_74E3_LC | . | . | . | . | . | . | . | . | . | . | . | . | . | . | . | . | . | . | . | . | . | . | . | . | . | . |
| 21-225_76D2_LC | . | . | . | . | H | . | . | . | . | . | . | . | . | . | . | . | . | . | . | . | . | . | . | . | . | . |
| 21-225_77H5_LC | . | . | . | . | . | . | . | . | . | . | . | . | . | . | . | . | . | . | . | . | . | . | . | . | . | . |
| 21-225_92H5_LC | . | . | . | . | . | . | . | . | . | . | . | . | . | . | . | . | . | . | . | . | . | V | . | . | . | . |
| 21-225_95G2_LC | . | . | . | . | . | . | . | . | . | . | . | . | . | . | . | . | . | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | S | E | D | F | A | V | Y |
| 21-225_162A10_LC | N | . | . | . | . | . | . | . | . | . | . | . | . | . | . | . | . | . | R | . | . | . | . | . | . | . |
| 21-225_193G12_LC | . | . | . | . | . | . | . | . | . | . | . | . | . | . | . | . | . | . | . | . | A | . | . | . | . | . |
| 21-225_201A4_LC | . | . | . | . | . | . | . | . | . | . | . | . | . | . | . | . | . | . | . | . | . | . | . | . | . | . |
| 21-225_201F2_LC | . | . | . | . | . | . | . | . | . | . | . | . | . | . | . | . | . | . | . | . | . | . | . | . | . | . |
| 21-225_201F7_LC | . | . | . | . | . | . | . | . | . | . | . | . | . | . | . | . | . | . | . | . | . | . | . | . | . | . |
| 21-225_201H4_LC | . | . | . | . | . | . | . | . | . | . | . | . | . | . | . | . | . | . | . | . | . | . | . | . | . | . |
| 21-225_202A8_LC | . | . | . | . | . | . | . | . | . | . | . | . | . | . | . | . | . | . | . | . | . | . | . | T | . | . |
| 21-225_202F12_LC | . | . | . | . | . | . | . | . | . | . | . | . | . | . | . | . | . | . | . | . | . | . | . | . | . | . |
| 21-225_205G4_LC | . | . | . | . | . | . | . | . | . | . | . | . | . | . | . | . | . | . | . | . | . | . | . | . | . | . |
| 21-225_210H10_LC | . | . | . | . | . | . | . | . | . | . | . | . | . | . | . | . | . | . | . | . | . | . | . | . | . | . |
| 21-225_212C10_LC | . | . | F | . | . | . | . | . | S | D | . | . | . | . | . | . | . | . | . | . | . | . | . | . | . | . |
| 21-225_216B10_LC | . | . | . | . | . | . | . | . | . | . | . | . | . | S | . | . | . | . | . | . | . | . | . | . | . | . |
| 21-225_24H3_LC | . | . | . | . | . | . | . | . | . | . | . | . | . | . | . | . | . | . | . | . | . | . | . | . | . | . |
| 21-225_57H3_LC | N | . | . | . | . | W | . | . | . | . | . | . | . | . | . | . | . | . | . | . | . | . | . | . | I | . |
| 21-225_62E6_LC | N | . | . | . | . | . | . | . | . | . | . | . | . | . | . | . | . | . | . | . | . | . | . | . | . | . |
| 21-225_65A6_LC | N | . | . | . | . | . | . | . | . | . | . | . | . | . | . | . | . | . | . | . | . | . | . | . | . | . |
| 21-225_67C3_LC | N | . | . | . | . | . | . | . | . | . | . | . | . | . | . | . | . | . | . | . | . | . | . | . | . | . |
| 21-225_70A5_LC | N | . | . | . | . | . | . | . | . | . | . | . | . | . | . | . | . | . | . | . | . | . | . | . | . | . |
| 21-225_74C3_LC | . | . | . | . | . | . | . | . | . | . | . | . | . | I | . | . | . | . | . | . | . | . | . | . | . | . |
| 21-225_74E11_LC | . | . | . | . | . | . | . | . | . | . | . | . | . | . | . | . | . | . | . | . | . | . | . | . | I | . |
| 21-225_74E3_LC | . | . | . | . | . | . | . | . | . | . | . | . | . | . | . | . | . | . | . | . | . | . | . | . | I | . |
| 21-225_76D2_LC | . | . | . | . | . | . | . | . | . | . | . | . | . | . | . | . | . | V | . | . | . | . | . | . | I | . |
| 21-225_77H5_LC | . | . | . | . | . | . | . | . | . | . | . | S | F | . | . | . | . | . | . | . | . | . | . | . | . | . |
| 21-225_92H5_LC | . | . | . | . | . | . | . | . | . | . | . | . | . | . | . | T | . | . | . | . | . | . | . | . | L | . |
| 21-225_95G2_LC | . | . | . | . | . | . | . | . | . | . | . | . | . | . | . | . | . | . | . | . | . | . | . | . | I | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | K_CDR3 | | | | | | | | | | | | | | | | | | | | | | | | | |
| CONSENSUS | Y | C | Q | Q | Y | N | D | - | - | - | - | - | - | - | - | - | - | - | - | - | - | - | - | - | - | - |
| 21-225_162A10_LC | F | . | . | . | . | . | . | . | . | . | . | . | . | . | . | . | . | . | . | . | . | . | . | . | . | . |
| 21-225_193G12_LC | . | . | . | . | . | . | N | . | . | . | . | . | . | . | . | . | . | . | . | . | . | . | . | . | . | . |
| 21-225_201A4_LC | . | . | . | . | F | Y | N | . | . | . | . | . | . | . | . | . | . | . | . | . | . | . | . | . | . | . |
| 21-225_201F2_LC | . | . | . | . | F | Y | N | . | . | . | . | . | . | . | . | . | . | . | . | . | . | . | . | . | . | . |
| 21-225_201F7_LC | . | . | . | . | F | Y | N | . | . | . | . | . | . | . | . | . | . | . | . | . | . | . | . | . | . | . |
| 21-225_201H4_LC | . | . | . | E | . | . | . | . | . | . | . | . | . | . | . | . | . | . | . | . | . | . | . | . | . | . |
| 21-225_202A8_LC | . | . | . | . | . | Y | N | . | . | . | . | . | . | . | . | . | . | . | . | . | . | . | . | . | . | . |
| 21-225_202F12_LC | . | . | . | . | . | D | N | . | . | . | . | . | . | . | . | . | . | . | . | . | . | . | . | . | . | . |
| 21-225_205G4_LC | . | . | . | . | F | Y | N | . | . | . | . | . | . | . | . | . | . | . | . | . | . | . | . | . | . | . |
| 21-225_210H10_LC | S | . | . | . | . | Y | N | . | . | . | . | . | . | . | . | . | . | . | . | . | . | . | . | . | . | . |
| 21-225_212C10_LC | S | . | . | . | . | . | . | . | . | . | . | . | . | . | . | . | . | . | . | . | . | . | . | . | . | . |
| 21-225_216B10_LC | . | . | . | . | . | . | . | . | . | . | . | . | . | . | . | . | . | . | . | . | . | . | . | . | . | . |
| 21-225_24H3_LC | . | . | . | . | . | . | N | . | . | . | . | . | . | . | . | . | . | . | . | . | . | . | . | . | . | . |
| 21-225_57H3_LC | . | . | . | . | . | . | . | . | . | . | . | . | . | . | . | . | . | . | . | . | . | . | . | . | . | . |
| 21-225_62E6_LC | . | . | . | . | . | . | . | . | . | . | . | . | . | . | . | . | . | . | . | . | . | . | . | . | . | . |
| 21-225_65A6_LC | . | . | . | . | . | . | . | . | . | . | . | . | . | . | . | . | . | . | . | . | . | . | . | . | . | . |
| 21-225_67C3_LC | . | . | . | . | . | . | . | . | . | . | . | . | . | . | . | . | . | . | . | . | . | . | . | . | . | . |
| 21-225_70A5_LC | . | . | . | . | . | . | . | . | . | . | . | . | . | . | . | . | . | . | . | . | . | . | . | . | . | . |
| 21-225_74C3_LC | . | . | . | . | . | . | . | . | . | . | . | . | . | . | . | . | . | . | . | . | . | . | . | . | . | . |
| 21-225_74E11_LC | . | . | . | . | . | . | . | . | . | . | . | . | . | . | . | . | . | . | . | . | . | . | . | . | . | . |
| 21-225_74E3_LC | . | . | . | . | . | . | . | . | . | . | . | . | . | . | . | . | . | . | . | . | . | . | . | . | . | . |
| 21-225_76D2_LC | . | . | . | . | . | . | . | . | . | . | . | . | . | . | . | . | . | . | . | . | . | . | . | . | . | . |
| 21-225_77H5_LC | F | . | . | . | . | . | . | . | . | . | . | . | . | . | . | . | . | . | . | . | . | . | . | . | . | . |
| 21-225_92H5_LC | . | . | . | E | . | . | . | . | . | . | . | . | . | . | . | . | . | . | . | . | . | . | . | . | . | . |
| 21-225_95G2_LC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | W | P | C | S | F | G | Q | G | T | K | L | E | I | K | R |
| 21-225_162A10_LC | . | . | . | . | . | . | . | . | . | . | . | . | . | . | . | . | . | . | . |
| 21-225_193G12_LC | . | . | . | . | . | . | . | . | . | . | . | . | . | . | . | . | . | . | T |
| 21-225_201A4_LC | . | . | . | . | . | L | . | . | . | . | . | . | . | . | . | . | L | . | . |
| 21-225_201F2_LC | . | . | . | . | . | L | . | . | . | . | . | . | . | . | . | . | L | . | . |
| 21-225_201F7_LC | . | . | . | . | . | L | . | . | . | . | . | . | . | . | . | . | L | . | . |
| 21-225_201H4_LC | . | . | . | . | . | . | . | . | . | . | . | . | . | N | . | . | . | . | . |
| 21-225_202A8_LC | . | . | . | . | . | L | . | . | . | . | . | . | . | . | . | . | . | R | . |
| 21-225_202F12_LC | . | . | . | W | P | . | . | . | . | . | . | . | . | . | . | . | . | . | . |
| 21-225_205G4_LC | . | . | . | . | . | L | . | . | . | . | . | . | . | . | . | . | L | . | . |
| 21-225_210H10_LC | . | . | . | . | . | L | . | . | . | . | . | . | . | . | . | . | . | . | . |
| 21-225_212C10_LC | . | . | . | . | . | . | . | . | . | . | . | . | . | . | . | . | . | . | . |
| 21-225_216B10_LC | . | . | . | . | . | . | . | . | . | . | . | . | . | . | . | . | . | R | . |
| 21-225_24H3_LC | . | . | . | W | P | M | . | . | . | . | . | . | . | . | . | . | . | . | . |
| 21-225_57H3_LC | . | . | . | . | . | . | . | . | . | . | . | . | . | . | . | . | . | . | . |
| 21-225_62E6_LC | . | . | . | . | . | . | . | . | . | . | . | . | . | . | . | . | . | . | . |
| 21-225_65A6_LC | . | . | . | . | . | . | . | . | . | . | L | E | . | . | . | . | . | . | . |
| 21-225_67C3_LC | . | . | . | . | . | . | . | . | . | . | . | . | . | . | . | . | . | . | . |
| 21-225_70A5_LC | . | . | . | . | . | . | . | . | . | . | . | . | . | . | . | . | . | . | . |
| 21-225_74C3_LC | . | . | . | . | . | . | . | . | . | . | L | . | . | . | . | . | . | . | . |
| 21-225_74E11_LC | . | . | . | . | . | . | . | . | . | . | . | . | . | . | . | . | . | . | . |
| 21-225_74E3_LC | . | . | . | . | . | . | . | . | . | . | . | . | . | . | . | . | . | . | . |
| 21-225_76D2_LC | . | . | . | . | . | . | . | . | . | . | . | . | . | . | . | . | . | . | . |
| 21-225_77H5_LC | . | . | . | . | . | . | . | . | . | . | . | . | S | . | . | . | . | Q | . |
| 21-225_92H5_LC | . | . | . | . | . | . | . | . | . | . | . | . | . | . | . | . | . | . | . |
| 21-225_95G2_LC | . | . | . | . | . | . | . | . | . | . | . | . | . | . | . | . | . | . | . |

# FIGURE 57

Table 112. Consensus 32 - VK3|A27/JK4 (SEQ ID NO: 50329):

EFMLTQSPGTLSLSPGERATLSCRAS--QSVSS-----SYLVWYQQKPGQAPRLLIYG--------ASTRATGIPDRFSGSGSG--TDFTLTISRLEPEYFAVYYCQQYGC-----------------------SPLTFGGGTKVEITR wherein:

A at position 25 can be substituted with S

Q at position 29 can be substituted with E

S at position 30 can be substituted with R

V at position 31 can be substituted with I

S at position 32 can be substituted with T

S at position 33 can be substituted with T

S at position 39 can be substituted with N

Y at position 40 can be substituted with A

V at position 42 can be substituted with S

T at position 69 can be substituted with S

T at position 72 can be substituted with S or I

G at position 110 can be substituted with V

C at position 111 can be substituted with N or S

P at position 136 can be substituted with L

Arg Xaa Ser Xaa Xaa Xaa Xaa Xaa Xaa Xaa Leu Xaa (SEQ ID NO: 50171)

Gly Ala Ser Xaa Arg Ala Xaa (SEQ ID NO: 50172)

**FIGURE 57**

Gln Gln Tyr Xaa Xaa Ser Xaa Leu Thr (SEQ ID NO: 50173)

Arg Ala Ser Gln Ser Val Ser Ser Ser Tyr Leu Val (SEQ ID NO: 50657)

Gly Ala Ser Thr Arg Ala Thr (SEQ ID NO: 50658)

Gln Gln Tyr Gly Cys Ser Pro Leu Thr (SEQ ID NO: 50659)

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | E | F | M | L | T | Q | S | P | G | T | L | S | L | S | P | G | E | R | A | T | L | S | C | R | A | S |
| 21-225_211G3_LC | . | I | V | . | . | . | . | . | . | . | . | . | . | . | . | . | . | . | . | . | . | . | . | . | . | . |
| 21-225_215C7_LC | . | I | V | . | . | . | . | . | . | . | . | . | . | . | . | . | . | . | V | . | . | . | . | . | . | . |
| 21-225_215H12_LC | . | I | V | . | . | . | . | . | . | . | . | . | . | . | . | . | . | . | . | . | . | . | . | . | . | . |
| 21-225_74A5_LC | . | . | . | . | . | . | . | . | . | . | . | . | . | . | . | . | . | . | . | . | . | . | . | . | . | . |
| 21-225_74B6_LC | . | I | V | . | . | . | . | . | . | . | . | . | . | . | . | . | . | . | . | . | . | . | . | . | . | . |
| 21-225_74G3_LC | . | . | . | . | . | . | . | . | . | . | . | C | W | . | . | . | . | . | . | . | . | . | . | . | . | . |
| 21-225_74G9_LC | . | . | . | . | . | . | . | . | . | . | . | . | . | . | . | . | . | . | . | . | . | . | . | . | . | . |
| 21-225_75B1_LC | . | . | . | . | . | . | . | . | . | . | . | Y | W | . | . | . | . | . | . | . | I | . | S | . | . | . |
| 21-225_76A6_LC | . | I | V | . | . | . | . | . | . | . | R | . | W | . | . | . | . | . | . | . | . | . | . | . | . | . |
| 21-225_76B9_LC | . | . | . | . | . | . | . | . | . | . | . | . | W | . | . | . | . | . | . | . | . | . | . | . | . | . |
| 21-225_76H1_LC | . | . | . | . | . | . | . | . | . | . | . | Y | W | . | . | . | . | . | . | . | . | . | . | . | . | . |
| 21-225_77D11_LC | . | . | . | . | . | . | . | . | . | . | . | Y | W | . | . | . | . | . | . | . | I | . | . | . | . | . |
| 21-225_80A2_LC | . | . | . | . | . | . | . | . | . | . | . | Y | . | . | . | . | . | . | . | . | . | . | . | . | . | . |
| 21-225_80C12_LC | . | I | V | . | . | . | . | . | . | . | . | Y | . | . | . | . | . | . | . | . | . | . | . | . | . | . |
| 21-225_82A5_LC | . | I | V | . | . | . | . | . | . | . | . | . | W | . | . | . | . | . | . | . | . | . | . | . | . | . |
| 21-225_82C12_LC | . | I | V | . | . | . | . | . | . | . | . | . | W | . | . | . | . | . | . | . | . | . | . | . | . | . |
| 21-225_82D9_LC | . | . | . | . | . | . | . | . | . | . | . | . | W | . | . | . | . | . | . | . | . | . | . | . | . | . |
| 21-225_82G5_LC | . | . | . | . | . | . | . | . | . | . | . | Y | W | . | . | . | . | . | . | . | . | . | S | . | . | . |
| 21-225_83C2_LC | . | . | . | . | . | . | . | . | . | . | . | C | . | . | . | . | . | . | . | . | . | . | . | . | . | . |
| 21-225_83C5_LC | . | . | . | . | . | . | . | . | . | . | . | C | W | . | . | . | . | . | . | . | . | . | . | . | . | . |
| 21-225_85B5_LC | . | . | . | . | . | . | . | . | . | . | . | . | . | . | . | . | . | . | . | . | . | . | . | . | S | . |
| 21-225_85D11_LC | . | . | . | . | . | . | . | . | . | . | . | . | . | . | T | . | . | . | . | . | . | . | . | . | S | . |
| 21-225_87A10_LC | . | . | V | . | . | . | . | . | . | . | . | Y | . | . | . | . | . | . | . | . | . | . | . | . | . | . |
| 21-225_96G1_LC | . | I | V | . | . | . | . | . | . | . | . | . | . | . | T | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | V | S | S | - | - | - | - | - | S | Y | L | V | W | Y | Q | Q | K | P | G | Q | A | P |
| 21-225_211G3_LC | . | . | . | . | . | . | . | . | . | . | . | . | . | F | . | A | . | . | . | . | . | . | . | . | . | . |
| 21-225_215C7_LC | . | . | . | R | I | T | T | . | . | . | . | . | N | F | . | A | . | . | . | . | . | . | . | . | . | . |
| 21-225_215H12_LC | . | . | . | . | . | . | . | . | . | . | . | . | . | F | . | A | . | . | . | . | . | . | . | . | . | . |
| 21-225_74A5_LC | . | . | . | . | . | . | . | . | . | . | . | . | . | . | . | . | . | . | . | . | . | . | . | . | . | . |
| 21-225_74B6_LC | . | . | . | . | . | . | . | . | . | . | . | . | . | . | . | A | . | . | . | . | . | . | . | . | . | . |
| 21-225_74G3_LC | . | . | . | . | . | . | . | . | . | . | . | . | . | . | . | . | . | . | . | . | . | . | . | . | . | . |
| 21-225_74G9_LC | . | . | . | . | . | . | . | . | . | . | . | . | . | . | . | . | . | . | . | . | . | . | . | . | . | . |
| 21-225_75B1_LC | . | . | . | . | . | . | . | . | . | . | . | . | . | . | . | . | . | . | . | . | . | . | . | . | . | . |
| 21-225_76A6_LC | . | . | . | . | . | . | . | . | . | . | . | . | . | . | . | A | . | . | . | . | . | . | . | . | . | . |
| 21-225_76B9_LC | . | . | . | . | . | . | . | . | . | . | . | . | . | . | . | . | . | . | . | . | . | . | . | . | . | . |
| 21-225_76H1_LC | . | . | . | . | . | . | . | . | . | . | . | . | . | . | . | . | . | . | . | . | . | . | . | . | . | . |
| 21-225_77D11_LC | . | . | . | . | . | . | . | . | . | . | . | . | . | . | . | . | . | . | . | . | . | . | . | . | . | . |
| 21-225_80A2_LC | . | . | . | . | . | . | . | . | . | . | . | . | . | . | . | . | . | . | . | . | . | . | . | . | . | . |
| 21-225_80C12_LC | . | . | . | . | . | . | . | . | . | . | . | . | . | . | . | A | . | . | . | . | . | . | . | . | . | . |
| 21-225_82A5_LC | . | . | . | . | . | . | . | . | . | . | . | . | . | . | . | A | . | . | . | . | . | . | . | . | . | . |
| 21-225_82C12_LC | . | . | . | . | . | . | . | . | . | . | . | . | . | . | . | A | . | . | . | . | . | . | . | . | . | . |
| 21-225_82D9_LC | . | . | E | . | . | . | . | . | . | . | . | . | . | . | . | . | . | . | . | . | . | . | . | . | . | . |
| 21-225_82G5_LC | . | . | E | . | . | . | . | . | . | . | . | . | . | . | . | . | . | . | . | . | . | . | . | . | . | . |
| 21-225_83C2_LC | . | . | . | . | . | . | . | . | . | . | . | . | . | . | . | . | . | . | . | . | . | . | . | . | . | . |
| 21-225_83C5_LC | . | . | E | . | . | . | . | . | . | . | . | . | . | . | . | . | . | . | . | . | . | . | . | . | . | . |
| 21-225_85B5_LC | . | . | . | . | . | . | . | . | . | . | . | . | . | . | . | . | . | . | . | . | . | . | . | . | . | . |
| 21-225_85D11_LC | . | . | . | . | . | . | . | . | . | . | . | . | . | . | . | . | . | . | . | . | . | . | . | . | . | . |
| 21-225_87A10_LC | . | . | . | . | . | . | . | . | . | . | . | . | . | . | . | A | . | . | . | . | . | . | . | . | . | . |
| 21-225_96G1_LC | . | . | . | . | . | . | . | . | . | . | . | . | . | . | . | A | . | . | . | . | . | . | . | . | P | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | R | L | L | I | Y | G | - | - | - | - | - | - | - | - | A | S | T | R | A | T | G | I | P | D | R | F |
| 21-225_211G3_LC | . | . | . | . | . | . | . | . | . | . | . | . | . | . | . | . | S | . | . | . | . | . | . | . | . | . |
| 21-225_215C7_LC | . | . | . | . | . | . | . | . | . | . | . | . | . | . | . | . | S | . | . | . | . | . | . | . | . | . |
| 21-225_215H12_LC | . | . | . | M | . | . | . | . | . | . | . | . | . | . | . | . | S | . | . | I | . | . | . | . | . | . |
| 21-225_74A5_LC | . | . | . | . | . | . | . | . | . | . | . | . | . | . | . | . | . | . | . | . | . | . | . | . | . | . |
| 21-225_74B6_LC | . | . | . | . | . | . | . | . | . | . | . | . | . | . | . | . | . | . | . | . | . | . | . | . | . | . |
| 21-225_74G3_LC | . | . | . | . | . | . | . | . | . | . | . | . | . | . | . | . | . | . | . | . | . | . | . | . | . | . |
| 21-225_74G9_LC | . | . | . | . | . | . | . | . | . | . | . | . | . | . | . | . | . | . | . | . | . | . | . | . | . | . |
| 21-225_75B1_LC | . | . | . | . | . | . | . | . | . | . | . | . | . | . | . | . | . | . | . | . | . | . | . | . | . | . |
| 21-225_76A6_LC | . | . | . | . | . | . | . | . | . | . | . | . | . | . | . | . | . | . | . | . | . | . | . | . | . | . |
| 21-225_76B9_LC | . | . | . | . | . | . | . | . | . | . | . | . | . | . | . | . | . | . | . | . | . | . | . | . | . | . |
| 21-225_76H1_LC | . | . | . | . | . | . | . | . | . | . | . | . | . | . | . | . | . | . | . | . | . | . | . | . | . | . |
| 21-225_77D11_LC | . | . | . | . | . | . | . | . | . | . | . | . | . | . | . | . | . | . | . | . | . | . | . | . | . | . |
| 21-225_80A2_LC | . | . | . | . | . | . | . | . | . | . | . | . | . | . | . | . | . | . | . | . | . | . | . | . | . | . |
| 21-225_80C12_LC | . | . | . | . | . | . | . | . | . | . | . | . | . | . | . | . | . | . | . | . | . | . | . | . | . | . |
| 21-225_82A5_LC | . | . | . | . | . | . | . | . | . | . | . | . | . | . | . | . | . | . | . | . | . | . | . | . | . | . |
| 21-225_82C12_LC | . | . | . | . | . | . | . | . | . | . | . | . | . | . | . | . | . | . | . | S | . | . | . | . | . | . |
| 21-225_82D9_LC | . | . | . | . | . | . | . | . | . | . | . | . | . | . | . | . | . | . | . | . | . | . | . | . | . | . |
| 21-225_82G5_LC | . | . | . | . | . | . | . | . | . | . | . | . | . | . | . | . | . | . | . | . | . | . | . | . | . | . |
| 21-225_83C2_LC | . | . | . | . | . | . | . | . | . | . | . | . | . | . | . | . | . | . | . | . | . | . | . | . | . | . |
| 21-225_83C5_LC | . | . | . | . | . | . | . | . | . | . | . | . | . | . | . | . | . | . | . | . | . | . | . | . | . | . |
| 21-225_85B5_LC | . | . | . | . | . | . | . | . | . | . | . | . | . | . | . | . | . | . | . | . | . | . | . | . | . | . |
| 21-225_85D11_LC | . | . | . | . | . | . | . | . | . | . | . | . | . | . | . | . | . | . | . | . | . | . | . | . | . | . |
| 21-225_87A10_LC | . | . | . | . | . | . | . | . | . | . | . | . | . | . | . | . | . | . | . | S | . | . | . | . | . | . |
| 21-225_96G1_LC | . | . | . | . | . | . | . | . | . | . | . | . | . | . | . | . | . | . | . | S | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | R | L | E | P | E | Y | F | A | V | Y |
| 21-225_211G3_LC | . | . | . | . | . | . | . | . | . | . | . | . | . | . | . | . | . | . | . | . | . | D | . | . | . | . |
| 21-225_215C7_LC | . | . | . | . | . | . | . | . | . | . | . | . | . | . | . | . | . | . | . | S | . | D | . | . | . | . |
| 21-225_215H12_LC | . | . | . | . | . | . | . | . | . | . | . | . | . | . | . | . | . | . | . | . | . | D | . | . | . | . |
| 21-225_74A5_LC | . | . | . | . | . | . | . | . | . | . | . | . | . | . | . | . | . | . | . | . | . | . | . | . | . | . |
| 21-225_74B6_LC | . | . | . | . | . | . | . | . | . | . | . | . | . | . | . | . | . | . | . | . | . | . | . | . | . | . |
| 21-225_74G3_LC | . | . | . | . | . | . | . | . | . | . | . | . | . | . | . | . | . | . | . | . | . | . | . | . | . | . |
| 21-225_74G9_LC | . | . | . | . | . | . | . | . | . | . | . | . | . | . | . | . | . | . | . | . | . | . | . | . | . | . |
| 21-225_75B1_LC | . | . | . | . | . | . | . | . | . | . | . | . | . | . | . | . | . | . | . | . | . | . | . | . | . | . |
| 21-225_76A6_LC | . | . | . | . | . | . | . | . | . | . | . | . | . | . | . | . | . | . | . | . | . | . | . | . | . | . |
| 21-225_76B9_LC | . | . | . | . | . | . | . | . | . | . | . | . | . | . | . | . | . | . | . | . | . | . | . | . | . | . |
| 21-225_76H1_LC | . | . | . | . | . | . | . | . | . | . | . | . | . | . | . | . | . | . | . | . | . | . | . | . | . | . |
| 21-225_77D11_LC | . | . | . | . | . | . | . | . | . | . | . | . | . | . | . | . | . | . | . | . | . | . | . | . | . | . |
| 21-225_80A2_LC | . | . | . | . | . | . | . | . | . | . | . | . | . | . | . | . | . | . | . | . | . | . | . | . | . | . |
| 21-225_80C12_LC | . | . | . | . | . | . | . | . | . | . | . | . | . | . | . | . | . | . | . | . | . | . | . | . | . | . |
| 21-225_82A5_LC | . | . | . | . | . | . | . | . | . | . | . | . | . | . | . | . | . | . | . | . | . | . | . | . | . | . |
| 21-225_82C12_LC | . | . | . | . | . | . | . | . | . | . | . | . | . | . | . | . | . | . | . | . | . | . | . | . | . | . |
| 21-225_82D9_LC | . | . | . | . | . | . | . | . | . | . | . | . | . | . | . | . | . | . | . | . | . | . | . | . | . | . |
| 21-225_82G5_LC | . | . | . | . | . | . | . | . | . | . | . | . | . | . | . | . | . | . | . | . | . | . | . | . | . | . |
| 21-225_83C2_LC | . | . | . | . | . | . | . | . | . | . | . | . | . | . | . | . | . | . | . | . | . | . | . | . | . | . |
| 21-225_83C5_LC | . | . | . | . | . | . | . | . | . | . | . | . | . | . | . | . | . | . | . | . | . | . | . | . | . | . |
| 21-225_85B5_LC | . | . | . | . | . | . | . | . | . | . | . | . | . | . | . | . | . | . | . | . | . | . | . | . | . | . |
| 21-225_85D11_LC | . | . | . | . | . | . | . | . | . | . | . | . | . | . | . | . | . | . | . | . | . | . | . | . | . | . |
| 21-225_87A10_LC | . | . | . | . | . | . | . | . | . | . | . | . | . | . | . | . | . | . | . | . | . | . | . | . | . | . |
| 21-225_96G1_LC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | G | C | - | - | - | - | - | - | - | - | - | - | - | - | - | - | - | - | - | - | - |
| 21-225_211G3_LC | . | . | . | . | . | V | S | . | . | . | . | . | . | . | . | . | . | . | . | . | . | . | . | . | . | . |
| 21-225_215C7_LC | . | . | . | . | . | V | S | . | . | . | . | . | . | . | . | . | . | . | . | . | . | . | . | . | . | . |
| 21-225_215H12_LC | . | . | . | . | . | V | S | . | . | . | . | . | . | . | . | . | . | . | . | . | . | . | . | . | . | . |
| 21-225_74A5_LC | . | . | . | . | . | . | . | . | . | . | . | . | . | . | . | . | . | . | . | . | . | . | . | . | . | . |
| 21-225_74B6_LC | . | . | . | . | . | . | N | . | . | . | . | . | . | . | . | . | . | . | . | . | . | . | . | . | . | . |
| 21-225_74G3_LC | . | . | . | . | . | . | . | . | . | . | . | . | . | . | . | . | . | . | . | . | . | . | . | . | . | . |
| 21-225_74G9_LC | . | . | . | . | . | . | . | . | . | . | . | . | . | . | . | . | . | . | . | . | . | . | . | . | . | . |
| 21-225_75B1_LC | . | . | . | . | . | . | . | . | . | . | . | . | . | . | . | . | . | . | . | . | . | . | . | . | . | . |
| 21-225_76A6_LC | . | . | . | . | . | . | N | . | . | . | . | . | . | . | . | . | . | . | . | . | . | . | . | . | . | . |
| 21-225_76B9_LC | . | . | . | . | . | . | . | . | . | . | . | . | . | . | . | . | . | . | . | . | . | . | . | . | . | . |
| 21-225_76H1_LC | . | . | . | . | . | . | . | . | . | . | . | . | . | . | . | . | . | . | . | . | . | . | . | . | . | . |
| 21-225_77D11_LC | . | . | . | . | . | . | . | . | . | . | . | . | . | . | . | . | . | . | . | . | . | . | . | . | . | . |
| 21-225_80A2_LC | . | . | . | . | . | . | . | . | . | . | . | . | . | . | . | . | . | . | . | . | . | . | . | . | . | . |
| 21-225_80C12_LC | . | . | . | . | . | . | N | . | . | . | . | . | . | . | . | . | . | . | . | . | . | . | . | . | . | . |
| 21-225_82A5_LC | . | . | . | . | . | . | N | . | . | . | . | . | . | . | . | . | . | . | . | . | . | . | . | . | . | . |
| 21-225_82C12_LC | . | . | . | . | . | . | N | . | . | . | . | . | . | . | . | . | . | . | . | . | . | . | . | . | . | . |
| 21-225_82D9_LC | . | . | . | . | . | . | . | . | . | . | . | . | . | . | . | . | . | . | . | . | . | . | . | . | . | . |
| 21-225_82G5_LC | . | . | . | . | . | . | . | . | . | . | . | . | . | . | . | . | . | . | . | . | . | . | . | . | . | . |
| 21-225_83C2_LC | . | . | . | . | . | . | . | . | . | . | . | . | . | . | . | . | . | . | . | . | . | . | . | . | . | . |
| 21-225_83C5_LC | . | . | . | . | . | . | . | . | . | . | . | . | . | . | . | . | . | . | . | . | . | . | . | . | . | . |
| 21-225_85B5_LC | . | . | . | . | . | . | . | . | . | . | . | . | . | . | . | . | . | . | . | . | . | . | . | . | . | . |
| 21-225_85D11_LC | . | . | . | . | . | . | . | . | . | . | . | . | . | . | . | . | . | . | . | . | . | . | . | . | . | . |
| 21-225_87A10_LC | . | . | . | . | . | . | N | . | . | . | . | . | . | . | . | . | . | . | . | . | . | . | . | . | . | . |
| 21-225_96G1_LC | . | . | . | . | . | . | N | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | S | P | L | T | F | G | G | G | T | K | V | E | I | T | R |
| 21-225_211G3_LC | . | . | . | . | . | . | . | . | . | . | . | . | . | . | . | . | . | K | . |
| 21-225_215C7_LC | . | . | . | . | . | L | . | . | . | . | . | . | . | . | . | . | . | K | . |
| 21-225_215H12_LC | . | . | . | . | . | . | . | . | . | . | . | . | . | . | . | . | . | K | . |
| 21-225_74A5_LC | . | . | . | . | . | . | . | . | . | . | . | . | . | . | . | . | . | . | . |
| 21-225_74B6_LC | . | . | . | . | . | . | . | . | . | . | . | . | . | . | . | . | . | K | . |
| 21-225_74G3_LC | . | . | . | . | . | . | . | . | . | . | . | . | . | . | . | . | . | . | . |
| 21-225_74G9_LC | . | . | . | . | . | . | . | . | . | . | . | . | . | . | . | . | . | . | . |
| 21-225_75B1_LC | . | . | . | . | . | . | . | . | . | . | . | . | . | . | . | . | . | . | . |
| 21-225_76A6_LC | . | . | . | . | . | . | . | . | . | . | . | . | . | . | . | . | . | K | . |
| 21-225_76B9_LC | . | . | . | . | . | . | . | . | . | . | . | . | . | . | . | . | . | . | . |
| 21-225_76H1_LC | . | . | . | . | . | . | . | . | . | . | . | . | . | . | . | . | . | . | . |
| 21-225_77D11_LC | . | . | . | . | . | . | . | . | . | . | . | . | . | . | . | . | . | . | . |
| 21-225_80A2_LC | . | . | . | . | . | . | . | . | . | . | . | . | . | . | . | . | . | . | . |
| 21-225_80C12_LC | . | . | . | . | . | . | . | . | . | . | . | . | . | . | . | . | . | K | . |
| 21-225_82A5_LC | . | . | . | . | . | . | . | . | . | . | . | . | . | . | . | . | . | K | . |
| 21-225_82C12_LC | . | . | . | . | . | . | . | . | . | . | . | . | . | . | . | . | . | K | . |
| 21-225_82D9_LC | . | . | . | . | . | . | . | . | . | . | . | . | . | . | . | . | . | . | . |
| 21-225_82G5_LC | . | . | . | . | . | . | . | . | . | . | . | . | . | . | . | . | . | . | . |
| 21-225_83C2_LC | . | . | . | . | . | . | . | . | . | . | . | . | . | . | . | . | . | . | . |
| 21-225_83C5_LC | . | . | . | . | . | . | . | . | . | . | . | . | . | . | . | . | . | . | . |
| 21-225_85B5_LC | . | . | . | . | . | . | . | . | . | . | . | . | . | . | . | . | . | . | . |
| 21-225_85D11_LC | . | . | . | . | . | . | . | . | . | . | . | . | . | . | . | . | . | . | . |
| 21-225_87A10_LC | . | . | . | . | . | . | . | . | . | . | . | . | . | . | . | . | . | K | . |
| 21-225_96G1_LC | . | . | . | . | . | . | . | . | . | . | . | . | . | . | . | . | . | K | . |

**FIGURE 57**

Table 113. Consensus 33 - VK2|A18/JK4 (SEQ ID NO: 50330):

DIVMTQTPLSLSVTPGQPASISCKSS--QSLLHSE-GKTYLYWYLQKPGQPPQLLIYE--------VSNRFSGVPDRFSGSGSG--TDFTLKISRVEAEDVGVYYCMQSIQ----------------------LPLTFGGGTKVEIKR wherein:

S at position 30 can be substituted with T

L at position 32 can be substituted with Q

H at position 33 can be substituted with R

S at position 34 can be substituted with G

E at position 35 can be substituted with D

K at position 38 can be substituted with R

Y at position 40 can be substituted with H or F

Y at position 42 can be substituted with N

V at position 67 can be substituted with I

N at position 69 can be substituted with Y or H

F at position 71 can be substituted with I, V or L

M at position 107 can be substituted with F

Q at position 108 can be substituted with H

S at position 109 can be substituted with G or N

I at position 110 can be substituted with K or T

Q at position 111 can be substituted with null (-), K or H

**FIGURE 57**

L at position 135 can be substituted with Q, Y, F or H

P at position 136 can be substituted with L or V

L at position 137 can be substituted with F or P

T at position 138 can be substituted with P or S

Lys Ser Ser Gln Xaa Leu Xaa Xaa Xaa Xaa Gly Xaa Thr Xaa Leu Xaa (SEQ ID NO: 50174)

Glu Xaa Ser Xaa Arg Xaa Ser (SEQ ID NO: 50175)

Xaa Xaa Xaa Xaa Xaa Xaa Xaa Xaa Xaa (SEQ ID NO: 50176)

Lys Ser Ser Gln Ser Leu Leu His Ser Glu Gly Lys Thr Tyr Leu Tyr (SEQ ID NO: 50660)

Glu Val Ser Asn Arg Phe Ser (SEQ ID NO: 50661)

Met Gln Ser Ile Gln Leu Pro Leu Thr (SEQ ID NO: 50662)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | V | M | T | Q | T | P | L | S | L | S | V | T | P | G | Q | P | A | S | I | S | C | K | S | S |
| 21-225_148F5_LC | . | . | . | . | . | . | . | . | . | . | . | . | . | . | . | . | . | . | . | . | . | . | . | . | . | . |
| 21-225_148H10_LC | . | . | . | . | . | . | . | . | . | . | . | . | . | . | . | . | . | . | . | . | . | . | . | . | . | . |
| 21-225_150D11_LC | . | . | . | . | . | . | . | . | . | . | . | . | . | . | . | . | . | . | . | . | . | . | . | . | . | . |
| 21-225_153G9_LC | . | . | . | . | . | . | . | . | F | . | . | . | . | . | . | . | . | . | . | . | . | . | . | . | . | . |
| 21-225_16F10_LC | . | . | . | . | . | . | . | . | . | . | . | . | . | I | . | . | . | . | . | . | . | . | . | . | . | . |
| 21-225_173C11_LC | . | . | . | . | . | . | . | . | . | . | . | . | . | . | . | . | . | . | . | . | . | . | . | . | . | . |
| 21-225_179C2_LC | . | . | . | . | . | . | . | . | . | . | . | . | . | . | . | . | . | . | . | . | . | . | . | . | . | . |
| 21-225_22H8_LC | . | . | . | . | . | . | . | . | . | . | . | . | . | . | . | . | . | . | . | . | . | . | . | . | . | . |
| 21-225_25A2_LC | . | . | . | . | N | . | . | . | . | . | . | . | . | . | . | . | . | . | . | . | . | . | . | . | . | . |
| 21-225_26G11_LC | . | . | . | . | . | . | . | . | . | . | . | . | . | . | . | . | . | . | . | . | . | . | . | . | . | . |
| 21-225_26G7_LC | . | . | L | . | . | . | . | . | . | . | . | . | . | . | . | . | . | . | . | . | . | . | . | . | . | . |
| 21-225_29H4_LC | . | . | . | . | . | . | . | . | . | . | . | . | . | . | . | . | . | . | . | . | F | . | . | . | . | . |
| 21-225_34D2_LC | . | . | . | . | . | . | . | . | . | . | . | . | . | . | . | . | . | . | . | . | . | . | . | . | . | . |
| 21-225_44B10_LC | H | . | . | . | . | . | . | . | . | . | . | . | . | . | . | . | . | . | . | . | . | . | . | . | . | . |
| 21-225_46B9_LC | . | . | . | . | . | . | . | . | . | . | . | . | . | . | . | . | . | . | . | . | . | . | . | . | . | . |
| 21-225_58H11_LC | . | . | . | . | . | . | . | . | . | . | . | . | . | . | . | . | . | . | . | . | . | . | . | . | . | . |
| 21-225_59A12_LC | . | . | . | . | . | . | . | . | . | . | . | . | . | . | . | . | . | . | . | . | . | . | . | . | . | . |
| 21-225_60C12_LC | . | . | . | . | . | . | . | . | . | . | . | . | . | . | . | . | . | . | . | . | . | . | . | . | . | . |
| 21-225_60G2_LC | . | . | . | . | . | . | . | . | . | . | . | . | . | . | . | . | . | S | . | . | . | . | . | . | . | . |
| 21-225_61B3_LC | . | . | . | . | . | . | . | . | . | . | . | . | . | . | . | . | . | . | . | . | . | . | . | . | . | . |
| 21-225_71A6_LC | . | . | . | . | . | . | . | . | . | . | . | . | . | . | . | . | . | . | . | . | . | . | . | . | . | . |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | L | L | H | S | E | - | G | K | T | Y | L | Y | W | Y | L | Q | K | P | G | Q | P | P |
| 21-225_148F5_LC | . | . | . | . | . | . | . | . | . | . | . | . | . | . | . | . | . | . | . | . | . | . | . | . | . | . |
| 21-225_148H10_LC | . | . | . | . | . | . | . | . | . | . | . | . | . | . | . | . | . | . | . | . | . | . | . | . | . | . |
| 21-225_150D11_LC | . | . | . | . | . | . | . | . | . | . | . | . | . | . | . | . | . | . | . | . | . | . | . | . | . | . |
| 21-225_153G9_LC | . | . | . | . | . | . | . | . | D | . | . | . | . | . | . | . | . | . | . | . | . | . | . | . | . | . |
| 21-225_16F10_LC | . | . | . | . | . | . | . | . | D | . | . | . | . | H | . | N | . | . | . | . | . | . | . | . | . | . |
| 21-225_173C11_LC | . | . | . | . | . | . | . | . | . | . | . | . | . | . | . | . | . | . | . | . | . | . | . | . | . | . |
| 21-225_179C2_LC | . | . | . | . | . | . | . | . | . | . | . | . | . | . | . | . | . | . | . | . | . | . | . | . | . | . |
| 21-225_22H8_LC | . | . | . | . | . | . | . | . | D | . | . | . | . | F | . | . | . | . | . | . | . | . | . | H | . | . |
| 21-225_25A2_LC | . | . | . | . | . | . | . | . | . | . | . | . | . | . | . | . | . | . | . | . | . | . | . | . | . | . |
| 21-225_26G11_LC | . | . | . | T | . | . | . | G | . | . | . | . | . | . | . | . | . | . | . | . | . | . | . | . | . | . |
| 21-225_26G7_LC | . | . | . | . | . | . | . | . | . | . | . | . | . | . | . | . | . | . | . | . | . | . | . | . | . | . |
| 21-225_29H4_LC | . | . | . | . | . | . | . | . | D | . | . | . | . | . | . | . | . | . | . | . | . | . | . | . | . | . |
| 21-225_34D2_LC | . | . | . | . | . | . | . | . | D | . | . | . | . | . | . | . | . | . | . | . | . | . | . | . | . | . |
| 21-225_44B10_LC | . | . | . | . | . | . | . | . | . | . | . | R | . | . | . | . | . | . | . | . | . | . | . | . | . | . |
| 21-225_46B9_LC | . | . | . | . | . | . | . | . | . | . | . | . | . | . | . | . | . | . | . | . | . | . | . | . | . | . |
| 21-225_58H11_LC | . | . | . | . | . | . | . | . | . | . | . | . | . | . | . | . | . | . | V | . | . | . | . | . | . | . |
| 21-225_59A12_LC | . | . | . | . | . | Q | . | G | . | . | . | . | . | . | . | . | . | . | V | . | . | . | . | . | . | . |
| 21-225_60C12_LC | . | . | . | . | . | . | . | . | . | . | . | . | . | . | . | . | . | . | . | . | . | . | . | . | . | . |
| 21-225_60G2_LC | . | . | . | . | . | . | . | . | . | . | . | . | . | . | . | . | . | . | . | . | . | . | . | . | . | . |
| 21-225_61B3_LC | . | . | . | . | . | . | . | . | . | . | . | . | . | . | . | . | . | . | . | . | . | . | . | . | . | . |
| 21-225_71A6_LC | . | . | . | . | . | . | R | . | D | . | . | . | . | H | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | Q | L | L | I | Y | E | - | - | - | - | - | - | - | - | V | S | N | R | F | S | G | V | P | D | R | F |
| 21-225_148F5_LC | . | . | . | . | . | . | . | . | . | . | . | . | . | . | . | . | Y | . | V | . | . | . | . | . | . | . |
| 21-225_148H10_LC | . | . | . | . | . | . | . | . | . | . | . | . | . | . | . | . | Y | . | V | . | . | . | . | . | . | . |
| 21-225_150D11_LC | . | . | . | . | . | . | . | . | . | . | . | . | . | . | . | . | Y | . | . | . | . | . | . | . | . | . |
| 21-225_153G9_LC | . | F | . | . | . | . | . | . | . | . | . | . | . | . | . | . | . | . | . | . | . | . | . | . | . | . |
| 21-225_16F10_LC | . | . | . | . | . | . | . | . | . | . | . | . | . | . | . | . | Y | . | . | . | . | . | . | . | . | . |
| 21-225_173C11_LC | . | . | . | . | . | . | . | . | . | . | . | . | . | . | . | . | H | . | . | . | . | . | . | . | . | . |
| 21-225_179C2_LC | . | . | . | . | . | . | . | . | . | . | . | . | . | . | . | . | H | . | . | . | . | . | . | . | . | . |
| 21-225_22H8_LC | . | . | . | . | . | . | . | . | . | . | . | . | . | . | . | . | . | . | . | . | . | . | . | . | . | . |
| 21-225_25A2_LC | . | . | . | . | . | . | . | . | . | . | . | . | . | . | I | . | . | . | . | . | . | . | . | A | . | L |
| 21-225_26G11_LC | H | . | . | . | . | . | . | . | . | . | . | . | . | . | . | . | . | . | . | . | . | . | . | . | . | . |
| 21-225_26G7_LC | . | F | . | . | . | . | . | . | . | . | . | . | . | . | . | . | H | . | L | . | . | . | . | . | . | . |
| 21-225_29H4_LC | . | . | . | . | F | . | . | . | . | . | . | . | . | . | . | . | H | . | . | . | . | L | . | . | . | . |
| 21-225_34D2_LC | . | . | . | . | . | . | . | . | . | . | . | . | . | . | . | . | . | . | . | . | . | . | . | . | . | . |
| 21-225_44B10_LC | . | . | . | . | . | . | . | . | . | . | . | . | . | . | I | . | H | . | . | . | . | . | . | . | . | . |
| 21-225_46B9_LC | . | . | . | . | . | . | . | . | . | . | . | . | . | . | . | . | Y | . | . | . | . | . | . | . | . | . |
| 21-225_58H11_LC | . | . | . | . | . | . | . | . | . | . | . | . | . | . | . | . | Y | . | . | . | . | . | . | . | . | . |
| 21-225_59A12_LC | . | . | . | . | . | . | . | . | . | . | . | . | . | . | . | . | Y | . | . | . | . | . | . | . | . | . |
| 21-225_60C12_LC | . | F | . | . | . | . | . | . | . | . | . | . | . | . | . | . | . | . | . | . | . | . | . | . | . | . |
| 21-225_60G2_LC | . | . | . | . | . | . | . | . | . | . | . | . | . | . | . | . | . | . | I | . | . | . | . | . | . | . |
| 21-225_61B3_LC | . | . | . | . | . | . | . | . | . | . | . | . | . | . | . | . | . | . | I | . | . | . | . | . | . | . |
| 21-225_71A6_LC | . | . | . | . | . | . | . | . | . | . | . | . | . | . | . | . | . | . | . | . | . | . | S | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | K | I | S | R | V | E | A | E | D | V | G | V | Y |
| 21-225_148F5_LC | . | . | . | . | . | . | . | . | . | . | . | . | . | . | . | . | . | . | . | . | . | . | . | . | . | . |
| 21-225_148H10_LC | . | . | . | . | . | . | . | . | . | . | . | . | . | . | . | . | . | . | . | . | . | . | . | . | . | . |
| 21-225_150D11_LC | . | . | . | . | . | . | . | . | . | . | . | . | . | . | . | . | . | . | . | . | . | . | . | . | . | . |
| 21-225_153G9_LC | . | . | . | . | . | . | . | . | . | . | . | . | . | . | . | . | . | . | . | . | D | . | . | . | . | . |
| 21-225_16F10_LC | . | . | . | . | . | . | . | . | . | V | . | . | . | E | . | . | . | . | . | . | A | . | . | . | . | . |
| 21-225_173C11_LC | . | . | . | . | . | . | . | . | . | . | . | . | . | . | . | . | . | . | . | . | . | . | . | . | . | . |
| 21-225_179C2_LC | . | . | . | . | . | . | . | . | . | . | . | . | . | . | . | . | . | . | . | . | . | . | . | . | . | . |
| 21-225_22H8_LC | . | . | . | . | . | . | . | . | . | . | . | . | . | . | . | . | . | . | . | . | . | . | . | . | I | . |
| 21-225_25A2_LC | . | . | . | . | . | . | . | . | . | . | . | . | . | . | . | . | . | . | . | . | . | . | . | . | . | . |
| 21-225_26G11_LC | . | . | . | . | . | . | . | . | . | . | . | . | . | . | . | . | . | . | . | . | . | . | . | . | I | . |
| 21-225_26G7_LC | . | . | . | . | . | . | . | . | . | . | . | . | . | . | . | . | . | . | . | . | . | . | . | . | . | . |
| 21-225_29H4_LC | . | . | . | . | . | . | . | . | . | . | . | . | . | . | . | . | . | . | . | . | . | . | . | . | . | . |
| 21-225_34D2_LC | . | . | . | . | . | . | . | . | . | . | . | . | . | . | . | . | . | . | . | . | . | . | . | . | . | . |
| 21-225_44B10_LC | . | . | . | . | . | . | . | . | . | . | . | . | . | . | . | . | . | . | . | . | . | . | . | . | . | . |
| 21-225_46B9_LC | . | . | . | . | . | . | . | . | . | . | . | . | . | . | . | . | . | M | . | . | . | . | . | . | . | . |
| 21-225_58H11_LC | . | . | . | . | . | . | . | . | . | . | . | . | . | . | . | . | . | . | . | . | . | . | . | . | . | . |
| 21-225_59A12_LC | . | . | . | . | . | . | . | . | . | . | . | . | . | . | . | . | . | . | . | V | . | . | . | . | . | . |
| 21-225_60C12_LC | . | . | . | . | . | . | . | . | . | . | . | . | . | . | . | . | . | . | . | . | . | . | . | . | I | . |
| 21-225_60G2_LC | . | . | . | . | . | . | . | . | . | . | . | . | . | . | . | . | . | . | . | . | . | . | . | . | . | . |
| 21-225_61B3_LC | . | . | . | . | . | . | . | . | . | . | . | . | . | . | . | . | . | . | . | . | . | . | . | . | . | . |
| 21-225_71A6_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | M | Q | S | I | Q | - | - | - | - | - | - | - | - | - | - | - | - | - | - | - | - | - | - | - |
| 21-225_148F5_LC | F | . | . | . | . | . | . | . | . | . | . | . | . | . | . | . | . | . | . | . | . | . | . | . | . | . |
| 21-225_148H10_LC | . | . | . | . | . | . | . | . | . | . | . | . | . | . | . | . | . | . | . | . | . | . | . | . | . | . |
| 21-225_150D11_LC | . | . | . | . | G | . | . | . | . | . | . | . | . | . | . | . | . | . | . | . | . | . | . | . | . | . |
| 21-225_153G9_LC | . | . | . | . | N | . | K | . | . | . | . | . | . | . | . | . | . | . | . | . | . | . | . | . | . | . |
| 21-225_16F10_LC | . | . | F | . | . | . | . | . | . | . | . | . | . | . | . | . | . | . | . | . | . | . | . | . | . | . |
| 21-225_173C11_LC | . | . | . | . | . | . | - | . | . | . | . | . | . | . | . | . | . | . | . | . | . | . | . | . | . | . |
| 21-225_179C2_LC | . | . | . | . | . | . | - | . | . | . | . | . | . | . | . | . | . | . | . | . | . | . | . | . | . | . |
| 21-225_22H8_LC | . | . | . | . | . | . | . | . | . | . | . | . | . | . | . | . | . | . | . | . | . | . | . | . | . | . |
| 21-225_25A2_LC | . | . | . | . | . | T | . | . | . | . | . | . | . | . | . | . | . | . | . | . | . | . | . | . | . | . |
| 21-225_26G11_LC | . | . | . | . | . | . | K | . | . | . | . | . | . | . | . | . | . | . | . | . | . | . | . | . | . | . |
| 21-225_26G7_LC | . | . | . | . | . | . | . | . | . | . | . | . | . | . | . | . | . | . | . | . | . | . | . | . | . | . |
| 21-225_29H4_LC | . | . | . | . | . | . | . | . | . | . | . | . | . | . | . | . | . | . | . | . | . | . | . | . | . | . |
| 21-225_34D2_LC | . | . | . | . | . | K | - | . | . | . | . | . | . | . | . | . | . | . | . | . | . | . | . | . | . | . |
| 21-225_44B10_LC | . | . | . | . | . | . | H | . | . | . | . | . | . | . | . | . | . | . | . | . | . | . | . | . | . | . |
| 21-225_46B9_LC | . | . | . | H | . | . | . | . | . | . | . | . | . | . | . | . | . | . | . | . | . | . | . | . | . | . |
| 21-225_58H11_LC | . | . | . | . | . | . | . | . | . | . | . | . | . | . | . | . | . | . | . | . | . | . | . | . | . | . |
| 21-225_59A12_LC | . | . | . | . | . | T | . | . | . | . | . | . | . | . | . | . | . | . | . | . | . | . | . | . | . | . |
| 21-225_60C12_LC | . | . | . | . | . | . | K | . | . | . | . | . | . | . | . | . | . | . | . | . | . | . | . | . | . | . |
| 21-225_60G2_LC | . | . | . | . | . | . | . | . | . | . | . | . | . | . | . | . | . | . | . | . | . | . | . | . | . | . |
| 21-225_61B3_LC | . | . | . | . | . | . | . | . | . | . | . | . | . | . | . | . | . | . | . | . | . | . | . | . | . | . |
| 21-225_71A6_LC | . | . | . | . | . | K | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | L | P | L | T | F | G | G | G | T | K | V | E | I | K | R |
| 21-225_148F5_LC | . | . | . | . | . | . | . | . | . | . | . | . | . | . | . | . | . | . | . |
| 21-225_148H10_LC | . | . | . | . | . | . | . | . | . | . | . | . | . | . | . | . | . | . | . |
| 21-225_150D11_LC | . | . | . | . | . | . | . | . | . | . | . | . | . | . | . | . | . | . | . |
| 21-225_153G9_LC | . | . | . | . | Y | . | . | . | . | . | . | . | . | . | . | . | . | . | . |
| 21-225_16F10_LC | . | . | . | . | . | . | . | . | . | . | . | . | . | . | . | . | . | . | . |
| 21-225_173C11_LC | . | . | . | . | Q | L | . | . | . | . | . | . | . | . | . | . | . | . | . |
| 21-225_179C2_LC | . | . | . | . | Q | V | . | . | . | . | . | . | . | . | . | . | . | . | . |
| 21-225_22H8_LC | . | . | . | . | . | . | . | S | . | . | . | . | . | . | . | . | . | N | . |
| 21-225_25A2_LC | . | . | . | . | F | . | . | . | . | . | . | . | . | . | . | . | . | . | . |
| 21-225_26G11_LC | . | . | . | . | . | . | . | . | . | . | . | . | . | . | . | . | . | . | . |
| 21-225_26G7_LC | . | . | . | . | . | . | . | . | . | . | . | . | . | . | . | . | . | . | . |
| 21-225_29H4_LC | . | . | . | . | H | . | F | . | . | . | . | . | . | R | . | . | . | . | . |
| 21-225_34D2_LC | . | . | . | . | Q | L | P | P | . | . | . | . | . | . | . | . | . | R | . |
| 21-225_44B10_LC | . | . | . | . | . | . | F | . | . | . | . | . | . | . | . | . | . | . | . |
| 21-225_46B9_LC | . | . | . | . | Y | . | . | . | . | . | . | . | . | . | . | . | . | Q | . |
| 21-225_58H11_LC | . | . | . | . | . | . | . | . | . | . | . | . | . | . | . | . | . | . | . |
| 21-225_59A12_LC | . | . | . | . | . | . | . | . | . | . | . | . | . | . | . | . | . | . | . |
| 21-225_60C12_LC | . | . | . | . | Y | . | . | . | . | . | . | . | . | . | . | . | . | . | . |
| 21-225_60G2_LC | . | . | . | . | . | . | . | . | . | . | . | . | . | . | . | . | . | . | . |
| 21-225_61B3_LC | . | . | . | . | . | . | . | . | . | . | . | . | . | . | . | . | . | . | . |
| 21-225_71A6_LC | . | . | . | . | . | . | . | . | . | . | . | . | . | . | . | . | F | . | . |

**FIGURE 57**

Table 114. Consensus 34 - VL2|2a2/JL3b (SEQ ID NO: 50331):

QSALTQP-ASVSGSPGQSITISCTGTS-SDVGGY----NYVSWYQQHPGKAPKLMIYE--------VSNRPSGVSNRFSGSKSG--NTASLTISGLQAEDEADYYCNSYTR---------------------SITWVFGGGTKLTVLG wherein:

V at position 31 can be substituted with I

G at position 33 can be substituted with S

Y at position 40 can be substituted with F

S at position 68 can be substituted with R

N at position 107 can be substituted with G, C or S

T at position 110 can be substituted with V or K

R at position 111 can be substituted with S or K

S at position 134 can be substituted with G, N or R

I at position 135 can be substituted with S or Y

Thr Gly Thr Ser Ser Asp Xaa Gly Xaa Tyr Asn Xaa Val Ser (SEQ ID NO: 50177)

Glu Val Xaa Asn Arg Pro Ser (SEQ ID NO: 50178)

Xaa Ser Tyr Xaa Xaa Xaa Xaa Thr Trp Val (SEQ ID NO: 50179)

Thr Gly Thr Ser Ser Asp Val Gly Gly Tyr Asn Tyr Val Ser (SEQ ID NO: 50663)

Glu Val Ser Asn Arg Pro Ser (SEQ ID NO: 50664)

Asn Ser Tyr Thr Arg Ser Ile Thr Trp Val (SEQ ID NO: 50665)

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | L_FR1 | | | | | | | | | | | | | | | | | | | | | | | | | |
| CONSENSUS | Q | S | A | L | T | Q | P | - | A | S | V | S | G | S | P | G | Q | S | I | T | I | S | C | T | G | T |
| 21-225_178H8_LC | . | . | . | . | . | . | S | . | . | . | . | . | . | . | . | . | . | . | . | . | . | . | . | . | . | . |
| 21-225_210D12_LC | . | . | . | . | . | . | . | . | . | . | . | . | . | . | . | . | . | . | . | . | . | . | . | . | . | . |
| 21-225_210E12_LC | . | . | . | . | . | . | . | . | . | . | . | . | . | . | . | . | . | . | . | . | . | . | . | . | . | . |
| 21-225_211C1_LC | . | . | . | . | . | . | . | . | . | . | . | . | . | . | . | . | . | . | . | . | . | . | . | . | . | . |
| 21-225_211G5_LC | . | . | . | . | . | . | . | . | . | . | . | . | . | . | . | . | . | . | . | . | . | . | . | . | . | . |
| 21-225_212A4_LC | . | . | . | . | . | . | . | . | . | . | . | . | . | . | . | . | . | . | . | . | . | . | . | . | . | . |
| 21-225_212C2_LC | . | . | . | . | . | . | . | . | . | . | . | . | . | . | . | . | . | . | . | . | . | . | . | . | . | . |
| 21-225_212F6_LC | . | . | . | . | . | . | . | . | . | . | . | . | . | . | . | . | . | . | . | . | . | . | . | . | . | . |
| 21-225_212G7_LC | . | . | . | . | . | . | . | . | . | . | . | . | . | . | . | . | . | . | . | . | . | . | . | . | . | . |
| 21-225_213D5_LC | . | . | . | . | . | . | . | . | . | . | . | . | . | . | . | . | . | . | . | . | . | . | . | . | . | . |
| 21-225_213G3_LC | L | . | . | . | . | . | . | . | . | . | . | . | . | . | . | . | . | . | . | . | . | . | . | . | . | . |
| 21-225_215D3_LC | . | . | . | . | . | . | . | . | . | . | . | . | . | . | . | . | . | . | . | . | . | . | . | . | . | . |
| 21-225_216A3_LC | . | . | . | . | . | . | . | . | . | . | . | . | . | . | . | . | . | . | . | . | . | . | . | . | . | . |
| 21-225_217B2_LC | . | . | . | . | . | . | . | . | . | . | . | . | . | . | . | . | . | . | . | . | . | . | . | . | . | . |
| 21-225_219A7_LC | . | . | . | . | . | . | . | . | . | . | . | . | . | . | . | . | . | . | . | . | . | . | . | . | . | . |
| 21-225_221G4_LC | . | . | . | . | . | . | . | . | . | . | . | . | . | . | . | . | . | . | . | . | . | . | . | . | . | . |
| 21-225_221H2_LC | L | . | . | . | . | . | . | . | . | . | . | . | . | . | . | . | . | . | . | . | . | . | . | . | . | . |
| 21-225_52H4_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | L_CDR1 | | | | | | | | | | | | | | | | L_FR2 | | | | | | | | | |
| CONSENSUS | S | - | S | D | V | G | G | Y | - | - | - | - | N | Y | V | S | W | Y | Q | Q | H | P | G | K | A | P |
| 21-225_178H8_LC | . | . | . | . | . | . | . | . | . | . | . | . | . | F | . | . | . | . | . | . | . | . | . | . | V | . |
| 21-225_210D12_LC | . | . | . | . | . | . | . | . | . | . | . | . | . | . | . | . | . | . | . | . | . | . | V | . | . | . |
| 21-225_210E12_LC | . | . | . | . | . | . | . | . | . | . | . | . | . | . | . | . | . | . | . | . | . | . | . | . | . | . |
| 21-225_211C1_LC | . | . | . | . | . | . | S | . | . | . | . | . | . | . | . | . | . | . | . | . | Y | . | . | . | . | . |
| 21-225_211G5_LC | . | . | . | . | . | . | . | . | . | . | . | . | . | . | . | . | . | . | . | . | . | . | . | . | . | . |
| 21-225_212A4_LC | . | . | . | . | . | . | . | . | . | . | . | . | . | . | . | . | . | . | . | . | . | . | . | . | . | . |
| 21-225_212C2_LC | . | . | . | . | . | . | . | . | . | . | . | . | . | . | . | . | . | . | . | . | . | . | . | . | . | . |
| 21-225_212F6_LC | . | . | . | . | . | . | . | . | . | . | . | . | . | . | . | . | . | . | . | . | Y | . | . | . | . | . |
| 21-225_212G7_LC | . | . | . | . | . | . | . | . | . | . | . | . | . | . | . | . | . | . | . | . | . | . | . | . | T | . |
| 21-225_213D5_LC | . | . | . | . | . | . | . | . | . | . | . | . | . | . | . | . | . | . | . | . | . | . | . | . | . | . |
| 21-225_213G3_LC | . | . | . | . | . | . | . | . | . | . | . | . | . | . | . | . | . | . | . | . | . | . | . | . | . | . |
| 21-225_215D3_LC | . | . | . | . | I | . | . | . | . | . | . | . | . | . | . | . | . | . | . | . | . | . | . | . | . | . |
| 21-225_216A3_LC | . | . | . | . | . | . | . | . | . | . | . | . | . | . | . | . | . | . | . | . | . | . | . | . | . | . |
| 21-225_217B2_LC | . | . | . | . | . | . | . | . | . | . | . | . | . | . | . | . | . | . | . | . | . | . | . | . | . | . |
| 21-225_219A7_LC | . | . | . | . | . | . | . | . | . | . | . | . | . | . | . | . | . | . | . | . | . | . | . | . | . | . |
| 21-225_221G4_LC | . | . | . | . | . | . | . | . | . | . | . | . | . | . | . | . | . | . | . | . | . | . | . | . | . | . |
| 21-225_221H2_LC | . | . | . | . | . | . | . | . | . | . | . | . | . | . | . | . | . | . | . | . | . | . | . | . | . | . |
| 21-225_52H4_LC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | L_CDR2 | | | | | | | | | | | | | | | | | | | | |
| CONSENSUS | K | L | M | I | Y | E | - | - | - | - | - | - | - | - | V | S | N | R | P | S | G | V | S | N | R | F |
| 21-225_178H8_LC | . | F | . | . | . | . | . | . | . | . | . | . | . | . | . | . | . | . | . | . | . | . | P | . | . | . |
| 21-225_210D12_LC | . | . | L | . | . | . | . | . | . | . | . | . | . | . | . | . | . | . | . | . | . | . | . | . | . | . |
| 21-225_210E12_LC | . | . | . | . | . | . | . | . | . | . | . | . | . | . | . | . | . | . | . | . | . | . | . | . | . | . |
| 21-225_211C1_LC | . | . | . | . | . | . | . | . | . | . | . | . | . | . | . | . | . | . | . | . | . | . | . | . | . | . |
| 21-225_211G5_LC | . | . | . | . | . | . | . | . | . | . | . | . | . | . | . | . | . | . | . | . | . | . | . | . | . | . |
| 21-225_212A4_LC | . | . | . | . | . | . | . | . | . | . | . | . | . | . | . | . | . | . | . | . | . | . | . | . | . | . |
| 21-225_212C2_LC | . | . | . | . | . | . | . | . | . | . | . | . | . | . | . | R | . | . | . | . | . | . | . | . | . | . |
| 21-225_212F6_LC | . | . | . | . | . | . | . | . | . | . | . | . | . | . | . | . | . | . | . | . | . | . | . | . | . | . |
| 21-225_212G7_LC | . | . | . | . | . | . | . | . | . | . | . | . | . | . | . | . | . | . | . | . | . | . | . | . | . | . |
| 21-225_213D5_LC | . | . | V | . | . | . | . | . | . | . | . | . | . | . | . | R | . | . | . | . | . | . | . | T | . | . |
| 21-225_213G3_LC | . | . | . | . | S | . | . | . | . | . | . | . | . | . | . | R | . | . | . | . | . | . | . | . | . | . |
| 21-225_215D3_LC | T | . | . | . | . | . | . | . | . | . | . | . | . | . | . | . | . | . | . | . | . | . | . | . | . | . |
| 21-225_216A3_LC | . | . | . | . | . | . | . | . | . | . | . | . | . | . | . | . | . | . | . | . | . | . | . | . | . | . |
| 21-225_217B2_LC | . | . | L | . | . | . | . | . | . | . | . | . | . | . | . | . | . | . | . | . | . | . | Y | . | . | . |
| 21-225_219A7_LC | . | . | . | . | . | . | . | . | . | . | . | . | . | . | . | R | . | . | . | . | . | . | . | . | . | . |
| 21-225_221G4_LC | . | . | . | . | . | . | . | . | . | . | . | . | . | . | . | R | . | . | . | . | . | . | . | . | . | . |
| 21-225_221H2_LC | . | . | . | . | S | . | . | . | . | . | . | . | . | . | . | R | . | . | . | . | . | . | . | . | . | . |
| 21-225_52H4_LC | . | . | . | . | . | . | . | . | . | . | . | . | . | . | . | . | . | . | . | . | . | . | . | . | . | . |

# FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | L_FR3 | | | | | | | | | | | | | | | | | | | | | | | | | |
| CONSENSUS | S | G | S | K | S | G | - | - | N | T | A | S | L | T | I | S | G | L | Q | A | E | D | E | A | D | Y |
| 21-225_178H8_LC | . | . | . | . | . | . | . | . | . | . | . | . | . | . | . | . | . | . | . | . | . | . | . | . | . | . |
| 21-225_210D12_LC | . | . | . | . | . | . | . | . | . | . | . | . | . | . | . | . | . | . | . | . | . | . | . | . | . | . |
| 21-225_210E12_LC | . | . | . | . | . | . | . | . | . | . | . | . | . | . | . | . | . | . | . | . | . | . | . | . | . | . |
| 21-225_211C1_LC | . | . | . | . | . | . | . | . | . | . | . | . | . | . | . | . | . | . | . | . | . | . | . | . | . | . |
| 21-225_211G5_LC | . | . | . | . | . | . | . | . | . | . | . | . | . | . | . | . | . | . | . | . | . | . | . | . | . | . |
| 21-225_212A4_LC | . | . | . | . | . | . | . | . | . | . | . | . | . | . | . | . | . | . | . | . | . | . | . | . | . | . |
| 21-225_212C2_LC | . | . | . | . | . | . | . | . | . | . | . | . | . | . | . | . | . | . | . | . | . | . | . | . | . | . |
| 21-225_212F6_LC | . | . | . | . | . | . | . | . | . | . | . | . | . | . | . | . | . | . | . | . | . | . | . | . | . | . |
| 21-225_212G7_LC | . | . | . | . | . | . | . | . | . | . | . | . | . | . | . | . | . | . | . | . | . | . | . | . | . | . |
| 21-225_213D5_LC | . | . | . | . | . | . | . | . | . | K | . | . | . | . | . | . | . | . | . | . | . | . | . | . | . | . |
| 21-225_213G3_LC | . | . | . | . | . | . | . | . | . | . | . | . | . | . | . | . | . | . | . | . | . | . | . | . | . | . |
| 21-225_215D3_LC | . | . | . | . | . | . | . | . | . | . | . | . | . | . | . | . | . | . | . | . | . | . | . | . | . | . |
| 21-225_216A3_LC | . | . | . | . | . | . | . | . | . | . | . | . | . | . | . | . | . | . | . | . | . | . | . | . | . | . |
| 21-225_217B2_LC | . | . | . | . | . | . | . | . | . | . | . | . | . | . | . | . | . | . | . | . | . | . | . | . | . | . |
| 21-225_219A7_LC | . | . | . | . | . | . | . | . | . | . | . | . | . | . | . | . | . | . | . | . | . | . | . | . | . | . |
| 21-225_221G4_LC | . | . | . | . | . | . | . | . | . | . | . | . | . | . | . | . | . | . | . | . | . | . | . | . | . | . |
| 21-225_221H2_LC | . | . | . | . | . | . | . | . | . | . | . | . | . | . | . | . | . | . | . | . | . | . | . | . | . | . |
| 21-225_52H4_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | L_CDR3 | | | | | | | | | | | | | | | | | | | | | | | |
| CONSENSUS | Y | C | N | S | Y | T | R | - | - | - | - | - | - | - | - | - | - | - | - | - | - | - | - | - | - | - |
| 21-225_178H8_LC | . | . | S | . | . | . | S | . | . | . | . | . | . | . | . | . | . | . | . | . | . | . | . | . | . | . |
| 21-225_210D12_LC | . | . | . | . | . | . | S | . | . | . | . | . | . | . | . | . | . | . | . | . | . | . | . | . | . | . |
| 21-225_210E12_LC | . | . | G | . | . | V | K | . | . | . | . | . | . | . | . | . | . | . | . | . | . | . | . | . | . | . |
| 21-225_211C1_LC | . | . | . | . | . | . | S | . | . | . | . | . | . | . | . | . | . | . | . | . | . | . | . | . | . | . |
| 21-225_211G5_LC | . | . | . | . | . | . | . | . | . | . | . | . | . | . | . | . | . | . | . | . | . | . | . | . | . | . |
| 21-225_212A4_LC | . | . | G | . | . | V | K | . | . | . | . | . | . | . | . | . | . | . | . | . | . | . | . | . | . | . |
| 21-225_212C2_LC | . | . | S | . | . | . | . | . | . | . | . | . | . | . | . | . | . | . | . | . | . | . | . | . | . | . |
| 21-225_212F6_LC | . | . | . | . | . | . | S | . | . | . | . | . | . | . | . | . | . | . | . | . | . | . | . | . | . | . |
| 21-225_212G7_LC | . | . | . | . | . | . | S | . | . | . | . | . | . | . | . | . | . | . | . | . | . | . | . | . | . | . |
| 21-225_213D5_LC | . | . | C | . | . | . | . | . | . | . | . | . | . | . | . | . | . | . | . | . | . | . | . | . | . | . |
| 21-225_213G3_LC | . | . | . | . | . | . | . | . | . | . | . | . | . | . | . | . | . | . | . | . | . | . | . | . | . | . |
| 21-225_215D3_LC | . | . | . | . | . | K | . | . | . | . | . | . | . | . | . | . | . | . | . | . | . | . | . | . | . | . |
| 21-225_216A3_LC | . | . | . | . | . | . | S | . | . | . | . | . | . | . | . | . | . | . | . | . | . | . | . | . | . | . |
| 21-225_217B2_LC | . | . | G | . | . | V | K | . | . | . | . | . | . | . | . | . | . | . | . | . | . | . | . | . | . | . |
| 21-225_219A7_LC | . | . | C | . | . | . | . | . | . | . | . | . | . | . | . | . | . | . | . | . | . | . | . | . | . | . |
| 21-225_221G4_LC | . | . | . | . | . | . | . | . | . | . | . | . | . | . | . | . | . | . | . | . | . | . | . | . | . | . |
| 21-225_221H2_LC | . | . | . | . | . | . | . | . | . | . | . | . | . | . | . | . | . | . | . | . | . | . | . | . | . | . |
| 21-225_52H4_LC | . | . | . | . | . | . | S | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | L_FR4 | | | | | | | | | | |
| CONSENSUS | - | - | - | S | I | T | W | V | F | G | G | G | T | K | L | T | V | L | G |
| 21-225_178H8_LC | . | . | . | . | Y | . | . | . | . | . | . | . | . | . | . | . | . | . | . |
| 21-225_210D12_LC | . | . | . | . | . | . | . | . | . | . | . | . | . | . | . | . | . | . | . |
| 21-225_210E12_LC | . | . | . | G | . | . | . | . | . | . | . | . | . | S | . | . | . | . | . |
| 21-225_211C1_LC | . | . | . | . | . | . | . | . | . | . | . | . | . | . | . | . | . | . | . |
| 21-225_211G5_LC | . | . | . | . | . | . | . | . | . | . | . | . | . | . | . | . | . | . | . |
| 21-225_212A4_LC | . | . | . | G | . | . | . | . | . | . | . | . | . | S | . | . | . | . | . |
| 21-225_212C2_LC | . | . | . | . | . | . | . | . | . | . | . | . | . | . | . | . | . | . | . |
| 21-225_212F6_LC | . | . | . | . | . | . | . | . | . | . | . | . | . | . | . | . | . | . | . |
| 21-225_212G7_LC | . | . | . | . | . | . | . | . | . | . | . | . | . | . | . | . | . | . | . |
| 21-225_213D5_LC | . | . | . | R | . | . | . | . | . | . | . | . | . | . | . | . | . | . | S |
| 21-225_213G3_LC | . | . | . | . | . | . | . | . | . | . | . | . | . | . | . | . | . | . | . |
| 21-225_215D3_LC | . | . | . | G | S | . | . | . | . | . | . | . | . | . | V | . | . | . | . |
| 21-225_216A3_LC | . | . | . | . | . | . | . | . | . | . | . | . | . | . | . | . | . | . | . |
| 21-225_217B2_LC | . | . | . | G | . | . | . | . | . | . | . | . | . | S | . | . | . | . | . |
| 21-225_219A7_LC | . | . | . | . | . | . | . | . | . | . | . | . | . | . | . | . | . | . | . |
| 21-225_221G4_LC | . | . | . | . | . | . | . | . | . | . | . | . | . | . | . | . | . | . | . |
| 21-225_221H2_LC | . | . | . | . | . | . | . | . | . | . | . | . | . | . | . | . | . | . | . |
| 21-225_52H4_LC | . | . | . | N | . | . | . | . | . | . | . | . | . | . | . | . | . | . | . |

**FIGURE 57**

Table 115. Consensus 35 - VK1|O12/JK1 (SEQ ID NO: 50332):

DIQMTQSPSSLSASVGDRVTITCRAS--QSIS------NYLNWYQQKPGKAPKLLIYA--------ASSLQSGVPSRFSGSGSG--TDFTLTISSLQPEDFATYYCQQSYS---------------------TPTWTFGQGTKVEIKR wherein:

A at position 25 can be substituted with S

Q at position 29 can be substituted with H or R

S at position 30 can be substituted with N, T or H

S at position 32 can be substituted with N, G or T

N at position 39 can be substituted with S or R

Y at position 40 can be substituted with F

A at position 58 can be substituted with T, S or V

A at position 67 can be substituted with T, E or V

S at position 68 can be substituted with L

S at position 69 can be substituted with N

Q at position 71 can be substituted with H

S at position 72 can be substituted with I

S at position 109 can be substituted with G or T

S at position 111 can be substituted with T, N or R

T at position 134 can be substituted with S or null (-)

P at position 135 can be substituted with I

T at position 136 can be substituted with P, Q or L

**FIGURE 57**

Arg Xaa Ser Xaa Xaa Ile Xaa Xaa Xaa Leu Asn (SEQ ID NO: 50180)

Xaa Xaa Xaa Xaa Leu Xaa Xaa (SEQ ID NO: 50181)

Gln Gln Xaa Tyr Xaa Xaa Xaa Xaa Trp Thr (SEQ ID NO: 50182)

Arg Ala Ser Gln Ser Ile Ser Asn Tyr Leu Asn (SEQ ID NO: 50666)

Ala Ala Ser Ser Leu Gln Ser (SEQ ID NO: 50667)

Gln Gln Ser Tyr Ser Thr Pro Thr Trp Thr (SEQ ID NO: 50668)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_157F3_LC | . | . | . | . | . | . | . | . | . | . | . | . | . | . | . | . | . | . | . | . | . | . | . | . | . | . |
| 21-225_173E7_LC | . | . | . | . | . | . | . | . | . | . | R | . | . | C | I | . | . | . | A | . | . | . | Y | . | . | . |
| 21-225_175G10_LC | . | . | . | . | . | . | . | . | . | . | . | . | . | . | . | . | . | . | . | I | . | . | . | . | . | . |
| 21-225_17A1_LC | . | . | . | . | . | . | . | . | . | . | . | . | . | . | . | . | . | . | . | . | . | . | . | . | . | . |
| 21-225_17B10_LC | . | . | . | . | . | . | . | . | . | . | . | . | . | . | . | . | . | . | . | . | . | . | . | . | S | . |
| 21-225_17B12_LC | . | . | . | . | . | . | . | . | . | . | . | . | . | . | . | . | . | . | . | S | . | . | . | . | . | . |
| 21-225_181G3_LC | . | . | . | . | . | . | . | . | . | . | . | . | . | . | . | . | . | . | . | N | . | . | . | . | . | . |
| 21-225_183A12_LC | . | . | . | . | . | . | . | . | . | . | . | C | . | . | . | . | . | . | . | . | . | . | . | . | . | . |
| 21-225_192G10_LC | . | . | . | . | . | . | . | . | . | . | . | . | . | . | . | . | . | . | . | . | . | . | . | . | . | . |
| 21-225_200G1_LC | . | . | . | . | . | . | . | . | . | . | . | . | . | . | . | . | . | . | . | . | . | . | . | . | . | . |
| 21-225_20C10_LC | . | . | H | . | . | . | . | . | . | . | . | . | . | . | . | . | . | K | . | S | . | . | . | . | . | . |
| 21-225_20F8_LC | . | . | . | . | . | . | . | . | . | . | . | . | . | . | . | . | . | . | . | . | . | . | . | . | . | . |
| 21-225_23H4_LC | . | . | . | . | . | . | . | . | . | . | . | . | . | . | . | . | . | . | . | . | . | . | . | . | . | . |
| 21-225_26A10_LC | . | . | . | . | . | . | . | . | . | . | . | . | . | . | . | . | . | . | I | . | . | . | . | . | . | . |
| 21-225_54B1_LC | . | . | . | . | . | . | . | . | . | . | . | . | . | . | I | . | . | . | . | . | . | . | . | . | . | . |
| 21-225_58F1_LC | . | . | . | . | . | . | . | . | . | . | . | . | . | . | . | . | . | . | . | . | . | . | . | . | . | . |
| 21-225_7G12_LC | . | . | . | . | . | . | . | . | . | . | . | . | . | . | . | . | . | . | . | S | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | I | S | - | - | - | - | - | - | N | Y | L | N | W | Y | Q | Q | K | P | G | K | A | P |
| 21-225_157F3_LC | . | . | . | N | . | . | . | . | . | . | . | . | S | . | . | . | . | . | . | L | . | . | . | . | . | . |
| 21-225_173E7_LC | . | . | . | T | . | . | . | . | . | . | . | . | . | . | . | . | . | . | . | . | . | . | . | . | . | . |
| 21-225_175G10_LC | . | . | . | T | . | G | . | . | . | . | . | . | . | . | . | . | . | . | . | . | . | . | . | R | . | . |
| 21-225_17A1_LC | . | . | . | N | . | N | . | . | . | . | . | . | . | . | . | . | . | . | . | . | . | . | . | . | G | . |
| 21-225_17B10_LC | . | . | . | . | . | N | . | . | . | . | . | . | S | . | . | . | . | . | . | . | . | . | . | . | . | . |
| 21-225_17B12_LC | . | . | . | N | . | N | . | . | . | . | . | . | . | . | . | . | . | . | . | . | . | . | . | . | G | . |
| 21-225_181G3_LC | . | . | H | . | . | . | . | . | . | . | . | . | . | . | . | . | . | . | . | . | . | A | . | . | . | . |
| 21-225_183A12_LC | . | . | R | N | . | . | . | . | . | . | . | . | . | . | . | . | . | . | . | . | . | . | . | . | . | . |
| 21-225_192G10_LC | . | . | . | . | . | . | . | . | . | . | . | . | . | . | . | . | . | . | . | . | T | . | . | . | . | . |
| 21-225_200G1_LC | . | . | . | . | . | . | . | . | . | . | . | . | S | . | . | . | . | F | . | . | . | . | . | . | . | . |
| 21-225_20C10_LC | . | . | H | H | . | . | . | . | . | . | . | . | . | . | . | . | . | . | . | . | . | . | . | . | G | . |
| 21-225_20F8_LC | . | . | . | . | . | . | . | . | . | . | . | . | R | . | . | . | . | . | . | . | . | . | . | . | . | . |
| 21-225_23H4_LC | . | . | . | . | . | . | . | . | . | . | . | . | R | . | . | . | . | . | . | . | . | . | . | R | . | . |
| 21-225_26A10_LC | . | . | . | . | . | . | . | . | . | . | . | . | S | . | . | . | . | . | . | . | . | . | . | . | . | . |
| 21-225_54B1_LC | . | . | . | N | . | . | . | . | . | . | . | . | S | . | . | . | . | . | . | . | . | . | . | . | . | . |
| 21-225_58F1_LC | . | . | . | . | . | T | . | . | . | . | . | . | . | F | . | . | . | . | . | . | . | . | . | . | . | . |
| 21-225_7G12_LC | . | . | . | N | . | N | . | . | . | . | . | . | . | . | . | . | . | . | . | . | . | . | . | . | G | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | Y | A | - | - | - | - | - | - | - | - | A | S | S | L | Q | S | G | V | P | S | R | F |
| 21-225_157F3_LC | . | . | . | . | . | T | . | . | . | . | . | . | . | . | . | . | . | . | . | . | . | . | . | . | . | . |
| 21-225_173E7_LC | . | . | . | . | . | . | . | . | . | . | . | . | . | . | . | . | . | . | . | I | . | . | . | . | . | . |
| 21-225_175G10_LC | . | . | . | . | . | . | . | . | . | . | . | . | . | . | . | . | . | . | H | . | . | . | . | . | G | . |
| 21-225_17A1_LC | . | V | . | . | F | T | . | . | . | . | . | . | . | . | T | . | . | . | . | . | . | . | . | . | . | . |
| 21-225_17B10_LC | . | . | . | . | F | . | . | . | . | . | . | . | . | . | . | . | . | . | H | . | . | . | . | . | . | . |
| 21-225_17B12_LC | . | V | . | . | L | T | . | . | . | . | . | . | . | . | . | . | . | . | . | . | . | . | . | . | . | . |
| 21-225_181G3_LC | N | . | . | . | . | T | . | . | . | . | . | . | . | . | T | L | N | . | . | . | . | . | . | . | . | . |
| 21-225_183A12_LC | . | . | . | . | . | V | . | . | . | . | . | . | . | . | V | . | . | . | . | . | . | . | . | . | . | . |
| 21-225_192G10_LC | . | . | . | . | . | . | . | . | . | . | . | . | . | . | E | . | . | . | . | . | . | . | . | . | . | . |
| 21-225_200G1_LC | . | . | . | . | . | . | . | . | . | . | . | . | . | . | E | . | . | . | . | . | . | . | . | . | . | . |
| 21-225_20C10_LC | . | V | . | . | L | T | . | . | . | . | . | . | . | . | . | . | . | . | . | . | . | . | . | . | . | . |
| 21-225_20F8_LC | E | . | . | . | . | . | . | . | . | . | . | . | . | . | . | . | . | . | . | . | . | . | . | . | . | . |
| 21-225_23H4_LC | . | . | . | . | C | S | . | . | . | . | . | . | . | . | T | . | . | . | . | . | . | . | . | . | . | . |
| 21-225_26A10_LC | . | V | . | . | . | . | . | . | . | . | . | . | . | . | . | . | . | . | . | . | . | . | . | . | . | . |
| 21-225_54B1_LC | . | F | . | . | . | . | . | . | . | . | . | . | . | . | . | . | . | . | H | . | . | . | . | P | . | . |
| 21-225_58F1_LC | . | . | . | . | F | . | . | . | . | . | . | . | . | . | . | . | . | . | . | . | . | I | . | . | . | . |
| 21-225_7G12_LC | . | V | . | . | L | T | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_157F3_LC | . | . | . | . | . | . | . | . | . | . | . | . | . | . | . | . | . | . | . | . | . | . | . | . | . | . |
| 21-225_173E7_LC | . | . | . | . | . | . | . | . | . | . | . | . | . | . | . | . | . | V | . | . | . | . | . | . | . | . |
| 21-225_175G10_LC | . | . | . | . | . | . | . | . | . | . | . | . | . | . | . | . | . | . | . | . | . | . | . | . | . | . |
| 21-225_17A1_LC | . | . | . | . | . | . | . | . | . | . | . | . | . | . | . | . | . | . | . | . | . | . | . | . | . | . |
| 21-225_17B10_LC | . | . | . | . | . | . | . | . | . | . | . | . | . | . | . | . | . | . | . | . | . | . | . | . | . | . |
| 21-225_17B12_LC | . | . | . | . | . | . | . | . | . | . | . | . | . | . | . | . | . | . | . | . | . | . | . | . | . | . |
| 21-225_181G3_LC | . | . | . | . | . | . | . | . | . | . | . | . | . | . | . | . | . | . | . | . | . | . | . | . | . | . |
| 21-225_183A12_LC | . | . | . | . | . | . | . | . | . | . | . | . | . | . | . | . | . | . | . | . | . | . | . | . | . | . |
| 21-225_192G10_LC | . | . | . | . | . | . | . | . | . | . | . | . | . | . | . | . | . | . | . | . | . | . | . | . | . | . |
| 21-225_200G1_LC | . | . | . | . | . | . | . | . | . | . | . | . | . | . | . | . | . | . | . | . | . | . | . | . | . | . |
| 21-225_20C10_LC | . | . | . | . | . | . | . | . | . | . | . | . | . | . | . | . | . | . | . | . | . | . | . | . | . | . |
| 21-225_20F8_LC | . | . | . | . | . | . | . | . | . | . | . | I | . | . | . | . | . | . | . | . | . | . | . | . | . | . |
| 21-225_23H4_LC | . | . | . | . | . | . | . | . | . | . | . | . | . | . | . | . | . | . | . | . | . | . | . | . | . | . |
| 21-225_26A10_LC | . | . | . | . | . | . | . | . | . | . | . | . | . | . | . | . | . | . | . | . | . | . | . | . | . | . |
| 21-225_54B1_LC | . | . | . | . | . | . | . | . | . | . | . | . | . | . | . | . | . | . | . | . | . | . | . | . | I | . |
| 21-225_58F1_LC | . | . | . | . | . | . | . | . | . | . | . | . | . | . | . | . | . | . | . | . | . | . | . | . | . | . |
| 21-225_7G12_LC | . | . | . | . | . | . | . | . | . | . | . | . | . | . | . | N | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | S | Y | S | - | - | - | - | - | - | - | - | - | - | - | - | - | - | - | - | - | - | - |
| 21-225_157F3_LC | . | . | . | . | . | . | N | . | . | . | . | . | . | . | . | . | . | . | . | . | . | . | . | . | . | . |
| 21-225_173E7_LC | F | . | . | . | . | . | R | . | . | . | . | . | . | . | . | . | . | . | . | . | . | . | . | . | . | . |
| 21-225_175G10_LC | F | . | . | . | . | . | R | . | . | . | . | . | . | . | . | . | . | . | . | . | . | . | . | . | . | . |
| 21-225_17A1_LC | . | . | . | . | . | . | T | . | . | . | . | . | . | . | . | . | . | . | . | . | . | . | . | . | . | . |
| 21-225_17B10_LC | . | . | . | . | G | . | N | . | . | . | . | . | . | . | . | . | . | . | . | . | . | . | . | . | . | . |
| 21-225_17B12_LC | . | . | . | . | T | . | T | . | . | . | . | . | . | . | . | . | . | . | . | . | . | . | . | . | . | . |
| 21-225_181G3_LC | . | . | . | . | . | . | . | . | . | . | . | . | . | . | . | . | . | . | . | . | . | . | . | . | . | . |
| 21-225_183A12_LC | . | . | . | . | . | . | . | . | . | . | . | . | . | . | . | . | . | . | . | . | . | . | . | . | . | . |
| 21-225_192G10_LC | S | . | . | . | . | . | . | . | . | . | . | . | . | . | . | . | . | . | . | . | . | . | . | . | . | . |
| 21-225_200G1_LC | S | . | . | . | . | . | . | . | . | . | . | . | . | . | . | . | . | . | . | . | . | . | . | . | . | . |
| 21-225_20C10_LC | . | . | . | . | . | . | T | . | . | . | . | . | . | . | . | . | . | . | . | . | . | . | . | . | . | . |
| 21-225_20F8_LC | . | . | . | . | . | . | N | . | . | . | . | . | . | . | . | . | . | . | . | . | . | . | . | . | . | . |
| 21-225_23H4_LC | . | . | . | . | . | . | . | . | . | . | . | . | . | . | . | . | . | . | . | . | . | . | . | . | . | . |
| 21-225_26A10_LC | . | . | . | . | . | . | . | . | . | . | . | . | . | . | . | . | . | . | . | . | . | . | . | . | . | . |
| 21-225_54B1_LC | . | . | . | . | . | . | R | . | . | . | . | . | . | . | . | . | . | . | . | . | . | . | . | . | . | . |
| 21-225_58F1_LC | . | . | . | . | . | . | . | . | . | . | . | . | . | . | . | . | . | . | . | . | . | . | . | . | . | . |
| 21-225_7G12_LC | . | . | . | . | . | . | T | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | T | P | T | W | T | F | G | Q | G | T | K | V | E | I | K | R |
| 21-225_157F3_LC | . | . | . | . | . | . | . | . | . | . | . | . | . | . | . | . | . | . | . |
| 21-225_173E7_LC | . | . | . | . | . | Q | . | . | . | . | . | . | . | . | . | . | . | . | . |
| 21-225_175G10_LC | . | . | . | . | . | Q | . | . | . | . | . | . | . | . | . | . | . | . | . |
| 21-225_17A1_LC | . | . | . | . | . | . | . | . | . | . | . | . | . | . | . | . | . | . | . |
| 21-225_17B10_LC | . | . | . | . | . | . | . | . | . | . | . | . | . | . | . | . | . | N | . |
| 21-225_17B12_LC | . | . | . | . | . | . | . | . | . | . | . | . | . | . | . | . | . | . | . |
| 21-225_181G3_LC | . | . | . | S | . | P | . | . | . | . | . | . | . | . | . | . | . | . | . |
| 21-225_183A12_LC | . | . | . | S | . | P | . | . | . | . | . | . | . | . | . | D | . | R | . |
| 21-225_192G10_LC | . | . | . | S | . | P | . | . | . | . | . | . | . | . | . | . | . | . | . |
| 21-225_200G1_LC | . | . | . | S | . | P | . | . | . | . | . | . | . | . | . | . | F | . | . |
| 21-225_20C10_LC | . | . | . | . | . | . | . | . | . | . | . | . | . | . | . | . | . | . | . |
| 21-225_20F8_LC | . | . | . | . | . | . | . | . | . | . | . | . | . | . | . | . | . | . | . |
| 21-225_23H4_LC | . | . | . | . | . | . | . | . | . | . | . | . | . | . | . | . | . | R | . |
| 21-225_26A10_LC | . | . | . | . | . | . | . | . | . | . | . | . | . | . | . | . | . | . | . |
| 21-225_54B1_LC | . | . | . | . | . | L | . | . | . | . | . | . | . | . | . | . | . | . | . |
| 21-225_58F1_LC | . | . | . | - | I | P | . | . | . | . | . | . | . | . | . | . | . | . | . |
| 21-225_7G12_LC | . | . | . | . | . | . | . | . | . | . | . | . | . | . | . | . | . | . | . |

**FIGURE 57**

Table 116. Consensus 36 - VK1|L5/JK4 (SEQ ID NO: 50333):

DIQMTQSPSSVSASVGDRVTITCRAS--QGIS------SWLAWYQQKPGKAPKLLIYA--------ASSLQSGVPSRFSGSGSG--TDFTLTISSLQPEDFATYYCQQINS----------------------FPLTFGGGTKVEIKR wherein:

G at position 30 can be substituted with D

S at position 39 can be substituted with N, I or K

W at position 40 can be substituted with Y

S at position 72 can be substituted with G

I at position 109 can be substituted with T, V, A or G

N at position 110 can be substituted with K

Arg Ala Ser Gln Xaa Ile Ser Xaa Xaa Leu Ala (SEQ ID NO: 50183)

Ala Ala Ser Ser Leu Gln Xaa (SEQ ID NO: 50184)

Gln Gln Xaa Xaa Ser Phe Pro Leu Thr (SEQ ID NO: 50185)

Arg Ala Ser Gln Gly Ile Ser Ser Trp Leu Ala (SEQ ID NO: 50669)

Ala Ala Ser Ser Leu Gln Ser (SEQ ID NO: 50670)

Gln Gln Ile Asn Ser Phe Pro Leu Thr (SEQ ID NO: 50671)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | V | S | A | S | V | G | D | R | V | T | I | T | C | R | A | S |
| 21-225_154E9_LC | . | . | . | . | . | L | . | . | . | . | . | Y | . | . | . | . | . | . | . | . | . | . | . | . | . | . |
| 21-225_62G7_LC | . | . | . | . | . | . | . | . | . | . | . | . | . | . | . | . | . | . | . | . | [illegible] | . | . | . | . | . |
| 21-225_63C9_LC | . | . | . | . | . | . | . | . | . | . | . | . | . | . | . | . | . | . | . | . | . | . | . | . | . | . |
| 21-225_63E1_LC | . | . | . | . | . | . | . | . | . | . | . | Y | . | . | . | . | . | . | . | . | . | . | . | . | . | . |
| 21-225_64H10_LC | . | . | . | . | . | . | . | . | . | . | . | . | . | . | . | . | . | . | . | . | . | . | . | . | . | . |
| 21-225_65G3_LC | . | . | . | . | . | . | . | . | . | . | . | . | . | . | . | . | . | . | . | . | . | . | . | . | . | . |
| 21-225_66A7_LC | . | . | . | . | . | . | . | . | . | . | . | . | . | . | . | . | . | . | . | . | . | . | . | . | . | . |
| 21-225_66B1_LC | . | . | . | . | . | . | . | . | . | . | . | . | . | . | . | . | . | . | . | . | . | . | . | . | . | . |
| 21-225_67H4_LC | . | . | . | . | . | . | . | . | . | . | . | . | . | . | . | . | . | . | . | . | . | . | . | . | . | . |
| 21-225_70A12_LC | . | . | . | . | . | . | . | . | . | . | . | . | . | . | . | . | . | . | . | . | . | . | . | . | . | . |
| 21-225_70D6_LC | . | . | . | . | . | . | . | . | . | . | . | . | . | . | . | . | . | . | . | . | . | . | . | . | . | . |
| 21-225_70G9_LC | . | . | . | . | . | . | . | . | . | . | . | . | . | . | . | . | . | . | . | . | . | . | . | . | . | . |
| 21-225_71A7_LC | . | . | . | . | . | . | . | . | . | . | . | . | . | . | . | . | . | . | . | . | . | . | . | . | . | . |
| 21-225_71B7_LC | . | . | . | . | . | . | . | . | . | . | . | . | . | . | . | . | . | . | . | . | . | . | . | . | . | . |
| 21-225_73A3_LC | . | . | . | . | . | . | . | . | . | . | . | . | . | . | I | . | . | . | . | . | . | . | . | . | . | . |
| 21-225_75C10_LC | . | . | . | . | . | . | . | . | . | . | . | . | . | . | . | . | . | . | . | I | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | S | - | - | - | - | - | - | S | W | L | A | W | Y | Q | Q | K | P | G | K | A | P |
| 21-225_154E9_LC | . | . | . | D | . | . | . | . | . | . | . | . | N | . | . | . | . | . | . | . | R | . | . | . | . | . |
| 21-225_62G7_LC | . | . | . | D | . | . | . | . | . | . | . | . | . | . | . | . | . | . | . | . | N | . | . | . | . | . |
| 21-225_63C9_LC | . | . | . | . | . | . | . | . | . | . | . | . | . | . | . | . | . | . | . | . | . | . | . | . | . | . |
| 21-225_63E1_LC | . | . | . | . | . | . | . | . | . | . | . | . | . | Y | . | . | . | . | . | . | . | . | . | . | . | . |
| 21-225_64H10_LC | . | . | . | . | . | . | . | . | . | . | . | . | . | . | . | . | . | . | . | . | . | . | . | . | . | L |
| 21-225_65G3_LC | . | . | . | . | . | . | . | . | . | . | . | . | . | . | . | . | . | . | . | . | . | . | . | . | . | . |
| 21-225_66A7_LC | . | . | . | . | . | . | . | . | . | . | . | . | . | . | . | . | . | . | . | . | . | . | . | . | . | . |
| 21-225_66B1_LC | . | . | . | . | . | . | . | . | . | . | . | . | . | . | . | . | . | . | . | . | . | . | . | . | . | L |
| 21-225_67H4_LC | . | . | . | . | . | . | . | . | . | . | . | . | . | . | . | . | . | . | . | . | . | . | . | . | . | . |
| 21-225_70A12_LC | . | . | . | . | . | . | . | . | . | . | . | . | . | . | . | . | . | . | . | . | . | . | . | . | . | L |
| 21-225_70D6_LC | . | . | . | . | . | . | . | . | . | . | . | . | K | . | . | . | . | . | . | . | N | . | . | . | . | L |
| 21-225_70G9_LC | . | . | . | D | . | . | . | . | . | . | . | . | . | . | . | . | . | . | . | . | N | . | . | . | . | . |
| 21-225_71A7_LC | . | . | . | . | . | . | . | . | . | . | . | . | . | . | . | . | . | . | . | . | . | . | . | . | . | . |
| 21-225_71B7_LC | . | . | . | . | . | . | . | . | . | . | . | . | . | . | . | . | . | . | . | . | N | . | . | E | . | . |
| 21-225_73A3_LC | . | . | . | . | . | . | . | . | . | . | . | . | N | . | . | . | . | . | . | . | . | . | . | . | . | . |
| 21-225_75C10_LC | . | . | . | . | . | . | . | . | . | . | . | . | I | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | Y | A | - | - | - | - | - | - | - | - | A | S | S | L | Q | S | G | V | P | S | R | F |
| 21-225_154E9_LC | . | V | . | . | . | . | . | . | . | . | . | . | . | . | . | . | . | . | . | . | . | . | . | . | . | . |
| 21-225_62G7_LC | . | . | . | . | . | . | . | . | . | . | . | . | . | . | . | . | . | . | . | . | . | . | . | . | . | . |
| 21-225_63C9_LC | N | . | . | . | . | . | . | . | . | . | . | . | . | . | . | . | . | . | . | . | . | . | . | . | . | . |
| 21-225_63E1_LC | . | . | . | . | . | . | . | . | . | . | . | . | . | . | . | . | . | . | . | . | . | . | . | . | . | . |
| 21-225_64H10_LC | . | . | . | . | . | . | . | . | . | . | . | . | . | . | . | . | . | . | . | . | . | . | . | . | . | . |
| 21-225_65G3_LC | . | . | . | . | . | . | . | . | . | . | . | . | . | . | . | . | . | . | . | . | . | . | . | . | . | . |
| 21-225_66A7_LC | . | . | . | . | . | . | . | . | . | . | . | . | . | . | . | . | . | . | . | . | . | . | . | . | . | . |
| 21-225_66B1_LC | . | . | . | . | . | . | . | . | . | . | . | . | . | . | . | . | . | . | . | . | . | . | . | . | . | . |
| 21-225_67H4_LC | . | . | . | . | . | . | . | . | . | . | . | . | . | . | . | . | . | . | . | . | . | . | . | . | . | . |
| 21-225_70A12_LC | . | . | . | . | . | . | . | . | . | . | . | . | . | . | . | . | . | . | . | . | . | . | . | . | . | . |
| 21-225_70D6_LC | . | . | . | . | F | . | . | . | . | . | . | . | . | . | . | . | . | . | . | . | . | . | . | . | . | . |
| 21-225_70G9_LC | . | . | . | . | . | . | . | . | . | . | . | . | . | . | . | . | . | . | . | . | . | . | . | . | . | . |
| 21-225_71A7_LC | . | . | . | . | . | . | . | . | . | . | . | . | . | . | . | . | . | . | . | . | . | . | . | . | . | . |
| 21-225_71B7_LC | . | . | . | . | . | . | . | . | . | . | . | . | . | . | . | . | . | . | . | G | . | . | . | . | . | . |
| 21-225_73A3_LC | . | . | . | . | . | . | . | . | . | . | . | . | . | . | . | . | . | . | . | . | . | . | . | . | . | . |
| 21-225_75C10_LC | . | . | . | . | . | . | . | . | . | . | . | . | . | . | . | . | . | . | . | . | . | . | . | L | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | S | S | L | Q | P | E | D | F | A | T | Y |
| 21-225_154E9_LC | . | . | . | . | . | . | . | . | . | . | . | . | . | . | . | . | . | V | . | . | . | . | . | . | . | . |
| 21-225_62G7_LC | . | . | . | . | . | . | . | . | . | N | . | . | . | . | . | . | . | . | . | . | . | . | . | . | . | . |
| 21-225_63C9_LC | . | . | . | . | . | . | . | . | . | . | . | . | . | . | . | . | G | . | . | . | . | . | . | . | . | . |
| 21-225_63E1_LC | . | . | . | . | . | . | . | . | . | . | . | . | . | . | . | . | . | . | . | . | . | . | . | . | . | . |
| 21-225_64H10_LC | . | . | . | . | . | . | . | . | . | . | . | . | . | . | . | . | . | . | . | . | . | . | . | . | . | . |
| 21-225_65G3_LC | . | . | . | . | . | . | . | . | . | . | . | . | . | . | . | . | . | . | . | . | . | . | . | . | . | F |
| 21-225_66A7_LC | . | . | . | . | . | . | . | . | . | . | . | . | . | . | . | . | . | . | . | . | . | . | . | . | . | . |
| 21-225_66B1_LC | . | . | . | . | . | . | . | . | . | . | . | . | . | . | . | . | . | . | . | . | . | . | . | . | . | . |
| 21-225_67H4_LC | . | . | . | . | . | . | . | . | . | . | . | . | . | . | . | . | . | . | . | . | . | . | . | . | . | . |
| 21-225_70A12_LC | . | . | . | . | . | . | . | . | . | . | . | . | . | . | . | . | . | V | . | . | . | . | . | . | . | . |
| 21-225_70D6_LC | . | . | . | . | . | . | . | . | . | . | . | . | . | . | . | . | . | . | . | . | . | . | . | . | N | . |
| 21-225_70G9_LC | . | . | . | . | . | . | . | . | . | . | . | . | . | . | . | . | . | . | . | . | . | . | . | . | . | . |
| 21-225_71A7_LC | . | . | . | . | . | . | . | . | . | . | . | . | . | . | . | . | . | . | . | . | . | . | . | . | . | . |
| 21-225_71B7_LC | . | . | . | . | . | . | . | . | . | . | . | . | . | . | . | . | . | . | . | . | . | . | . | . | N | . |
| 21-225_73A3_LC | . | . | . | . | . | E | . | . | . | . | . | . | . | . | . | . | . | . | . | . | . | . | . | . | . | . |
| 21-225_75C10_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | | N | S | - | - | - | - | - | - | - | - | - | - | - | - | - | - | - | - | - | - | - |
| 21-225_154E9_LC | . | . | . | . | G | . | . | . | . | . | . | . | . | . | . | . | . | . | . | . | . | . | . | . | . | . |
| 21-225_62G7_LC | . | . | . | . | T | . | . | . | . | . | . | . | . | . | . | . | . | . | . | . | . | . | . | . | . | . |
| 21-225_63C9_LC | F | . | . | . | I | . | . | . | . | . | . | . | . | . | . | . | . | . | . | . | . | . | . | . | . | . |
| 21-225_63E1_LC | . | . | . | . | A | . | . | . | . | . | . | . | . | . | . | . | . | . | . | . | . | . | . | . | . | . |
| 21-225_64H10_LC | . | . | . | . | I | . | . | . | . | . | . | . | . | . | . | . | . | . | . | . | . | . | . | . | . | . |
| 21-225_65G3_LC | . | . | . | . | V | . | . | . | . | . | . | . | . | . | . | . | . | . | . | . | . | . | . | . | . | . |
| 21-225_66A7_LC | . | . | . | . | I | . | . | . | . | . | . | . | . | . | . | . | . | . | . | . | . | . | . | . | . | . |
| 21-225_66B1_LC | . | . | . | . | T | . | . | . | . | . | . | . | . | . | . | . | . | . | . | . | . | . | . | . | . | . |
| 21-225_67H4_LC | . | . | . | . | I | . | . | . | . | . | . | . | . | . | . | . | . | . | . | . | . | . | . | . | . | . |
| 21-225_70A12_LC | . | . | . | . | I | . | . | . | . | . | . | . | . | . | . | . | . | . | . | . | . | . | . | . | . | . |
| 21-225_70D6_LC | . | . | . | . | T | . | . | . | . | . | . | . | . | . | . | . | . | . | . | . | . | . | . | . | . | . |
| 21-225_70G9_LC | . | . | . | . | T | . | . | . | . | . | . | . | . | . | . | . | . | . | . | . | . | . | . | . | . | . |
| 21-225_71A7_LC | . | . | . | . | V | . | . | . | . | . | . | . | . | . | . | . | . | . | . | . | . | . | . | . | . | . |
| 21-225_71B7_LC | . | . | . | . | T | . | . | . | . | . | . | . | . | . | . | . | . | . | . | . | . | . | . | . | . | . |
| 21-225_73A3_LC | . | . | . | . | V | . | . | . | . | . | . | . | . | . | . | . | . | . | . | . | . | . | . | . | . | . |
| 21-225_75C10_LC | . | . | . | . | A | K | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | F | P | L | T | F | G | G | G | T | K | V | E | I | K | R |
| 21-225_154E9_LC | . | . | . | . | . | . | . | . | . | . | . | . | . | . | . | D | . | . | . |
| 21-225_62G7_LC | . | . | . | . | . | . | . | . | . | . | . | . | . | . | . | . | . | . | . |
| 21-225_63C9_LC | . | . | . | . | . | . | . | . | . | . | . | . | . | . | . | A | . | . | . |
| 21-225_63E1_LC | . | . | . | . | . | . | . | . | . | . | . | . | . | . | . | . | . | . | . |
| 21-225_64H10_LC | . | . | . | . | . | . | . | . | . | . | . | . | . | . | . | . | . | . | . |
| 21-225_65G3_LC | . | . | . | . | . | . | . | . | . | . | . | . | . | . | . | . | . | . | . |
| 21-225_66A7_LC | . | . | . | . | . | . | . | . | . | . | . | . | . | . | . | . | . | . | . |
| 21-225_66B1_LC | . | . | . | . | . | . | . | . | . | . | . | . | . | . | . | . | . | . | . |
| 21-225_67H4_LC | . | . | . | . | . | . | . | . | . | . | . | . | . | . | . | . | . | . | . |
| 21-225_70A12_LC | . | . | . | . | . | . | . | . | . | . | . | . | . | . | . | . | . | . | . |
| 21-225_70D6_LC | . | . | . | . | . | . | . | . | . | . | . | . | . | . | . | . | . | . | . |
| 21-225_70G9_LC | . | . | . | . | . | . | . | . | . | . | . | . | . | . | . | . | . | . | . |
| 21-225_71A7_LC | . | . | . | . | . | . | . | . | . | . | . | . | . | . | . | . | L | . | . |
| 21-225_71B7_LC | . | . | . | . | . | . | . | . | . | . | . | . | . | . | . | . | . | . | . |
| 21-225_73A3_LC | . | . | . | . | . | . | . | . | . | . | . | . | . | . | . | . | . | . | . |
| 21-225_75C10_LC | . | . | . | . | . | . | . | . | . | . | . | . | . | . | . | . | . | . | . |

**FIGURE 57**

Table 117. Consensus 37 - VK1|O18/JK5 (SEQ ID NO: 50334):

DIQMTQSPSSLSASVGDRVTITC<u>QAS--QDIN------NYLN</u>WYQQKPGKAPKLLIY<u>D--------ASNLET</u>GVPSRFSGSGSG--TDFTFTISSLQPEDIATYYC<u>QQYDN-----------------------LPIT</u>FGQGTRLEIKR wherein:

S at position 26 can be substituted with N

D at position 30 can be substituted with Y

N at position 32 can be substituted with S, T, F or Y

N at position 39 can be substituted with D

Y at position 40 can be substituted with F

A at position 67 can be substituted with G

N at position 69 can be substituted with T, D or S

Y at position 109 can be substituted with F

D at position 110 can be substituted with E

N at position 111 can be substituted with null (-) or I

L at position 135 can be substituted with N or V

P at position 136 can be substituted with L

Gln Ala Xaa Gln Xaa Ile Xaa Xaa Xaa Leu Asn (SEQ ID NO: 50186)

Asp Xaa Ser Xaa Leu Glu Thr (SEQ ID NO: 50187)

Gln Gln Xaa Xaa Xaa Xaa Xaa Ile Thr (SEQ ID NO: 50188)

Gln Ala Ser Gln Asp Ile Asn Asn Tyr Leu Asn (SEQ ID NO: 50672)

Asp Ala Ser Asn Leu Glu Thr (SEQ ID NO: 50673)

# FIGURE 57

Gln Gln Tyr Asp Asn Leu Pro Ile Thr (SEQ ID NO: 50674)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | Q | A | S |
| 21-225_11A2_LC | . | . | . | . | . | . | . | . | . | . | . | . | . | . | . | . | . | . | . | . | . | . | . | . | . | . |
| 21-225_13A1_LC | . | . | . | . | . | . | . | . | . | . | . | . | . | . | . | . | . | . | . | . | . | . | . | . | . | . |
| 21-225_159C4_LC | . | . | . | . | . | . | . | . | . | . | . | . | . | . | . | . | . | . | . | . | . | . | . | . | . | . |
| 21-225_15C2_LC | . | . | . | . | . | . | . | . | . | . | . | . | . | . | . | . | . | . | . | . | . | . | . | . | . | . |
| 21-225_15F10_LC | . | . | . | . | . | . | . | . | . | . | . | . | . | . | . | . | . | . | . | . | . | . | . | . | . | N |
| 21-225_161G4_LC | . | . | . | . | . | . | . | . | . | . | . | . | . | . | . | . | . | . | . | . | . | . | . | . | . | . |
| 21-225_16B3_LC | . | . | . | . | . | . | . | . | . | . | . | . | . | . | . | . | . | . | . | . | . | . | . | . | . | . |
| 21-225_16F6_LC | . | . | . | . | . | . | . | . | . | . | . | . | . | . | . | . | . | . | . | . | . | . | . | . | . | N |
| 21-225_1B12_LC | . | . | . | . | . | . | . | . | . | . | . | . | . | . | . | . | . | . | . | . | . | . | . | . | . | . |
| 21-225_225B11_LC | . | . | . | . | . | . | . | . | . | . | . | . | . | . | I | . | . | . | . | . | . | . | . | . | . | . |
| 21-225_24D6_LC | . | . | . | . | . | . | . | . | . | . | . | . | . | . | . | . | . | . | . | . | . | . | . | . | . | . |
| 21-225_30E2_LC | . | . | . | . | . | . | . | . | N | . | . | . | . | . | . | . | . | . | . | . | . | . | . | . | . | . |
| 21-225_35E3_LC | . | . | . | . | . | . | . | . | . | . | . | . | . | . | . | . | . | . | . | . | . | . | . | . | . | . |
| 21-225_50G4_LC | . | . | . | . | . | . | . | . | T | . | . | . | . | . | . | . | . | . | . | . | . | . | . | . | . | . |
| 21-225_5E6_LC | . | . | . | . | . | . | . | . | . | . | . | . | . | . | . | . | . | . | . | . | . | . | . | . | . | . |
| 21-225_6G7_LC | . | . | . | . | . | . | . | . | . | . | R | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | D | I | N | - | - | - | - | - | - | N | Y | L | N | W | Y | Q | Q | K | P | G | K | A | P |
| 21-225_11A2_LC | . | . | . | . | . | S | . | . | . | . | . | . | . | . | . | . | . | . | . | . | . | . | . | . | . | . |
| 21-225_13A1_LC | . | . | . | . | . | . | . | . | . | . | . | . | . | . | . | . | . | . | . | . | . | L | . | T | . | . |
| 21-225_159C4_LC | . | . | . | . | . | S | . | . | . | . | . | . | D | . | . | . | . | . | . | . | . | . | . | . | . | . |
| 21-225_15C2_LC | . | . | . | . | . | T | . | . | . | . | . | . | . | . | . | . | . | . | . | . | . | . | . | . | . | . |
| 21-225_15F10_LC | . | . | . | . | . | T | . | . | . | . | . | . | . | F | . | . | . | . | . | L | . | . | . | . | . | . |
| 21-225_161G4_LC | . | . | . | . | . | Y | . | . | . | . | . | . | . | . | . | . | . | . | . | . | . | . | . | . | . | . |
| 21-225_16B3_LC | . | . | . | . | . | . | . | . | . | . | . | . | . | . | . | . | . | . | . | . | . | . | . | R | . | . |
| 21-225_16F6_LC | . | . | . | . | . | T | . | . | . | . | . | . | . | F | . | . | . | . | . | L | . | . | . | . | . | . |
| 21-225_1B12_LC | . | . | . | . | . | . | . | . | . | . | . | . | . | . | . | . | . | . | . | . | . | . | . | . | . | . |
| 21-225_225B11_LC | . | . | . | . | . | F | . | . | . | . | . | . | . | . | . | . | . | . | . | . | . | . | . | . | . | . |
| 21-225_24D6_LC | . | . | . | . | . | . | . | . | . | . | . | . | . | . | . | . | . | . | . | . | . | . | . | . | . | L |
| 21-225_30E2_LC | . | . | . | Y | . | S | . | . | . | . | . | . | . | . | . | . | . | . | . | . | . | S | . | . | . | . |
| 21-225_35E3_LC | . | . | . | . | . | F | . | . | . | . | . | . | . | . | . | . | . | . | . | . | . | . | . | . | . | . |
| 21-225_50G4_LC | . | . | . | . | . | T | . | . | . | . | . | . | . | F | . | . | . | . | . | . | . | . | . | . | . | . |
| 21-225_5E6_LC | . | . | . | . | . | . | . | . | . | . | . | . | . | . | . | . | . | C | . | . | . | . | . | . | . | . |
| 21-225_6G7_LC | . | . | . | . | . | S | . | . | . | . | . | . | . | . | . | . | . | C | . | . | . | . | . | . | . | L |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | L | L | I | Y | D | - | - | - | - | - | - | - | - | A | S | N | L | E | T | G | V | P | S | R | F |
| 21-225_11A2_LC | . | . | . | . | . | . | . | . | . | . | . | . | . | . | . | . | . | . | . | . | . | . | . | . | . | . |
| 21-225_13A1_LC | . | . | . | . | . | . | . | . | . | . | . | . | . | . | G | . | . | . | . | . | . | . | . | . | . | . |
| 21-225_159C4_LC | . | . | . | . | . | . | . | . | . | . | . | . | . | . | . | . | T | . | . | . | . | . | . | . | . | . |
| 21-225_15C2_LC | N | . | . | . | . | . | . | . | . | . | . | . | . | . | . | . | . | . | . | . | . | . | . | . | . | . |
| 21-225_15F10_LC | N | . | . | . | S | . | . | . | . | . | . | . | . | . | . | . | . | . | . | . | . | . | . | . | . | . |
| 21-225_161G4_LC | . | . | . | . | . | . | . | . | . | . | . | . | . | . | . | . | . | . | . | . | . | . | . | . | . | . |
| 21-225_16B3_LC | . | . | . | . | . | . | . | . | . | . | . | . | . | . | . | . | . | . | . | . | . | . | . | . | . | . |
| 21-225_16F6_LC | N | . | . | . | S | . | . | . | . | . | . | . | . | . | . | . | . | . | . | . | . | . | . | . | . | . |
| 21-225_1B12_LC | . | . | . | . | . | . | . | . | . | . | . | . | . | . | . | . | . | . | . | . | . | . | . | . | . | . |
| 21-225_225B11_LC | . | . | . | . | . | . | . | . | . | . | . | . | . | . | . | . | T | . | . | . | . | . | . | . | . | . |
| 21-225_24D6_LC | . | . | . | . | . | . | . | . | . | . | . | . | . | . | . | . | D | . | . | . | . | . | . | . | . | . |
| 21-225_30E2_LC | . | . | . | . | . | . | . | . | . | . | . | . | . | . | G | . | S | . | . | . | . | . | . | . | . | . |
| 21-225_35E3_LC | N | . | . | . | . | . | . | . | . | . | . | . | . | . | . | . | . | . | . | . | . | . | . | . | . | . |
| 21-225_50G4_LC | . | . | . | . | . | . | . | . | . | . | . | . | . | . | . | . | . | . | . | . | . | . | . | . | . | . |
| 21-225_5E6_LC | . | . | . | . | . | . | . | . | . | . | . | . | . | . | . | . | . | . | . | . | . | . | . | . | . | . |
| 21-225_6G7_LC | . | . | . | . | . | . | . | . | . | . | . | . | . | . | . | . | T | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | F | T | I | S | S | L | Q | P | E | D | I | A | T | Y |
| 21-225_11A2_LC | . | . | . | . | . | . | . | . | . | . | . | . | . | . | . | . | . | . | . | . | . | . | V | . | . | . |
| 21-225_13A1_LC | . | . | . | . | . | . | . | . | . | . | . | . | . | . | . | . | . | . | . | . | . | . | . | . | . | . |
| 21-225_159C4_LC | . | . | . | . | . | . | . | . | . | . | . | . | . | . | . | . | . | . | . | . | . | . | . | . | . | . |
| 21-225_15C2_LC | . | . | . | . | . | . | . | . | . | . | . | . | . | . | . | . | . | . | . | . | . | . | . | . | . | . |
| 21-225_15F10_LC | . | . | . | . | . | . | . | . | . | . | . | . | . | . | . | . | . | . | . | . | . | . | V | . | . | . |
| 21-225_161G4_LC | . | . | . | . | . | . | . | . | . | . | . | . | . | A | . | . | . | . | . | . | . | . | . | . | . | . |
| 21-225_16B3_LC | . | . | . | . | . | . | . | . | . | . | . | . | . | . | . | . | . | . | . | . | . | . | . | . | . | . |
| 21-225_16F6_LC | . | . | . | . | . | . | . | . | . | . | . | . | . | . | . | . | . | . | . | . | . | . | V | . | . | . |
| 21-225_1B12_LC | . | . | . | . | . | . | . | . | . | . | . | . | . | . | . | . | . | . | . | . | . | . | . | T | . | . |
| 21-225_225B11_LC | . | . | . | . | . | . | . | . | . | . | . | . | . | . | . | . | . | . | . | . | . | . | . | . | . | . |
| 21-225_24D6_LC | . | . | . | . | . | . | . | . | . | . | . | . | . | . | . | . | . | . | . | . | . | . | . | . | I | . |
| 21-225_30E2_LC | . | . | . | . | . | . | . | . | . | . | . | . | . | . | . | . | . | . | . | . | . | . | L | . | . | . |
| 21-225_35E3_LC | . | . | . | . | . | . | . | . | . | . | . | . | . | . | . | . | . | . | . | . | . | . | . | . | . | . |
| 21-225_50G4_LC | . | . | . | . | . | . | . | . | . | . | . | . | . | . | . | . | . | . | . | . | . | . | . | . | . | . |
| 21-225_5E6_LC | . | . | . | . | . | . | . | . | . | . | . | . | . | . | . | . | . | . | . | . | . | . | . | . | . | . |
| 21-225_6G7_LC | . | . | . | . | . | . | . | . | . | . | . | . | . | . | . | . | . | V | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | D | N | - | - | - | - | - | - | - | - | - | - | - | - | - | - | - | - | - | - | - |
| 21-225_11A2_LC | . | . | . | . | . | . | . | . | . | . | . | . | . | . | . | . | . | . | . | . | . | . | . | . | . | . |
| 21-225_13A1_LC | . | . | . | . | . | [illegible] | . | . | . | . | . | . | . | . | . | . | . | . | . | . | . | . | . | . | . | . |
| 21-225_159C4_LC | [illegible] | . | . | . | . | . | . | . | . | . | . | . | . | . | . | . | . | . | . | . | . | . | . | . | . | . |
| 21-225_15C2_LC | . | . | . | . | . | . | . | . | . | . | . | . | . | . | . | . | . | . | . | . | . | . | . | . | . | . |
| 21-225_15F10_LC | . | . | . | . | . | . | . | . | . | . | . | . | . | . | . | . | . | . | . | . | . | . | . | . | . | . |
| 21-225_161G4_LC | . | . | . | . | . | . | [illegible] | . | . | . | . | . | . | . | . | . | . | . | . | . | . | . | . | . | . | . |
| 21-225_16B3_LC | . | . | . | . | [illegible] | . | . | . | . | . | . | . | . | . | . | . | . | . | . | . | . | . | . | . | . | . |
| 21-225_16F6_LC | . | . | . | . | . | . | . | . | . | . | . | . | . | . | . | . | . | . | . | . | . | . | . | . | . | . |
| 21-225_1B12_LC | . | . | . | . | . | [illegible] | . | . | . | . | . | . | . | . | . | . | . | . | . | . | . | . | . | . | . | . |
| 21-225_225B11_LC | . | . | . | . | . | . | . | . | . | . | . | . | . | . | . | . | . | . | . | . | . | . | . | . | . | . |
| 21-225_24D6_LC | . | . | . | . | . | [illegible] | . | . | . | . | . | . | . | . | . | . | . | . | . | . | . | . | . | . | . | . |
| 21-225_30E2_LC | . | . | . | . | . | . | . | . | . | . | . | . | . | . | . | . | . | . | . | . | . | . | . | . | . | . |
| 21-225_35E3_LC | [illegible] | . | . | . | . | . | . | . | . | . | . | . | . | . | . | . | . | . | . | . | . | . | . | . | . | . |
| 21-225_50G4_LC | . | . | . | . | . | . | - | . | . | . | . | . | . | . | . | . | . | . | . | . | . | . | . | . | . | . |
| 21-225_5E6_LC | . | . | . | . | . | . | . | . | . | . | . | . | . | . | . | . | . | . | . | . | . | . | . | . | . | . |
| 21-225_6G7_LC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | L | P | I | T | F | G | Q | G | T | R | L | E | I | K | R |
| 21-225_11A2_LC | . | . | . | . | . | . | . | . | . | . | . | . | . | . | . | D | . | . | . |
| 21-225_13A1_LC | . | . | . | . | . | . | . | . | . | . | . | . | . | . | . | . | . | . | . |
| 21-225_159C4_LC | . | . | . | . | . | . | . | . | . | . | . | . | . | . | . | . | . | . | . |
| 21-225_15C2_LC | . | . | . | . | . | . | . | . | . | A | . | . | . | . | . | . | . | . | . |
| 21-225_15F10_LC | . | . | . | . | . | . | . | . | . | . | . | . | . | . | . | . | . | . | . |
| 21-225_161G4_LC | . | . | . | . | . | . | . | . | . | . | . | . | . | . | . | . | . | . | . |
| 21-225_16B3_LC | . | . | . | . | . | . | . | . | . | . | . | . | . | . | . | . | . | . | . |
| 21-225_16F6_LC | . | . | . | . | . | . | . | . | . | . | . | . | . | . | . | . | . | . | . |
| 21-225_1B12_LC | . | . | . | . | . | . | . | . | . | . | . | . | . | . | . | . | . | . | . |
| 21-225_225B11_LC | . | . | . | . | . | . | . | . | . | . | . | . | . | . | . | . | . | . | . |
| 21-225_24D6_LC | . | . | . | . | . | . | . | . | . | . | . | . | . | . | . | . | . | . | . |
| 21-225_30E2_LC | . | . | . | . | . | . | . | . | . | . | . | . | . | . | . | . | . | . | . |
| 21-225_35E3_LC | . | . | . | . | . | . | . | . | . | . | . | . | . | . | . | D | . | R | . |
| 21-225_50G4_LC | . | . | . | . | N | L | . | . | . | . | . | . | . | . | . | . | . | . | . |
| 21-225_5E6_LC | . | . | . | . | . | . | . | . | . | . | . | . | . | . | . | . | . | . | . |
| 21-225_6G7_LC | . | . | . | . | V | . | . | . | . | . | . | . | . | . | . | . | . | . | . |

**FIGURE 57**

Table 118. Consensus 38 - VL1|1b/JL2 (SEQ ID NO: 50335):

QSVLTQP-PSVSAAPGQKVTISCSGSS-SNIGN-----NYVSWYQQLPGTAPKLLIYD--------NNKRPSGIPDRFSGSKSG--TSATLGITGLQTGDEADYYCGTWDSS----------------------LSVGVFGGGTKLTVLG wherein:

I at position 31 can be substituted with L

N at position 33 can be substituted with S

N at position 39 can be substituted with H or K

Y at position 40 can be substituted with F

V at position 41 can be substituted with L

N at position 67 can be substituted with S

N at position 68 can be substituted with Y or S

T at position 108 can be substituted with A or I

S at position 111 can be substituted with G, I or R

S at position 112 can be substituted with R

S at position 135 can be substituted with N

V at position 136 can be substituted with A or T

G at position 137 can be substituted with V or M

Ser Gly Ser Ser Ser Asn Xaa Gly Xaa Xaa Xaa Xaa Ser (SEQ ID NO: 50189)

Asp Xaa Xaa Lys Arg Pro Ser (SEQ ID NO: 50190)

Gly Xaa Trp Asp Xaa Xaa Leu Xaa Xaa Xaa Val (SEQ ID NO: 50191)

Ser Gly Ser Ser Ser Asn Ile Gly Asn Asn Tyr Val Ser (SEQ ID NO: 50675)

FIGURE 57

Asp Asn Asn Lys Arg Pro Ser (SEQ ID NO: 50676)

Gly Thr Trp Asp Ser Ser Leu Ser Val Gly Val (SEQ ID NO: 50677)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | L_FR1 | | | | | | | | | | | | | | | | | | | | | | | | | |
| CONSENSUS | Q | S | V | L | T | Q | P | - | P | S | V | S | A | A | P | G | Q | K | V | T | I | S | C | S | G | S |
| 21-225_190B10_LC | . | . | . | . | . | . | . | . | . | . | . | . | . | . | . | . | . | . | . | . | . | . | . | . | . | . |
| 21-225_190C3_LC | . | . | . | . | . | . | . | . | . | . | . | . | . | . | . | . | . | . | . | . | . | . | . | . | . | . |
| 21-225_190D10_LC | . | . | . | . | . | . | . | . | . | . | . | . | . | . | . | . | . | . | . | . | . | . | . | . | . | . |
| 21-225_190D8_LC | . | . | . | . | . | . | . | . | . | . | . | . | . | . | . | . | . | . | . | . | . | . | . | . | . | . |
| 21-225_190H7_LC | . | . | . | . | . | . | . | . | . | . | . | . | . | . | . | . | . | . | . | . | . | . | . | . | . | . |
| 21-225_191A1_LC | . | . | . | . | . | . | . | . | . | . | . | . | . | . | . | . | . | | . | . | . | . | . | . | . | . |
| 21-225_192G2_LC | . | . | . | . | . | . | . | . | . | . | . | . | . | . | . | . | . | . | . | . | . | . | . | . | . | . |
| 21-225_194F11_LC | . | . | . | . | . | . | . | . | . | . | . | . | . | . | . | . | . | . | . | . | . | . | . | . | . | . |
| 21-225_194G12_LC | . | . | . | . | . | . | . | . | . | . | . | . | . | . | . | . | . | . | . | . | . | . | . | . | . | . |
| 21-225_197E8_LC | . | . | . | . | . | . | . | . | . | . | . | . | . | . | . | . | . | . | . | . | . | . | . | . | . | . |
| 21-225_198D2_LC | . | . | . | . | . | . | . | . | . | . | . | . | . | . | . | . | . | . | . | . | . | . | . | . | . | . |
| 21-225_200G8_LC | . | . | . | . | . | . | . | . | . | . | . | . | . | . | . | . | . | | . | . | . | . | . | . | . | . |
| 21-225_209A8_LC | . | . | . | . | . | . | . | . | . | . | . | . | . | . | . | . | . | . | . | . | . | . | . | . | . | . |
| 21-225_214D8_LC | . | . | . | . | . | . | . | . | . | . | . | . | . | . | . | . | . | . | . | . | . | . | . | . | . | . |
| 21-225_218G4_LC | . | . | . | . | . | . | . | . | . | . | . | . | . | . | . | . | . | . | . | . | . | . | . | . | . | . |
| 21-225_58C5_LC | . | . | . | . | . | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | L_CDR1 | | | | | | | | | | | | | | | | L_FR2 | | | | | | | | | |
| CONSENSUS | S | - | S | N | I | G | N | - | - | - | - | - | N | Y | V | S | W | Y | Q | Q | L | P | G | T | A | P |
| 21-225_190B10_LC | . | . | . | . | . | . | . | . | . | . | . | . | . | . | . | . | . | . | . | H | . | . | . | . | . | . |
| 21-225_190C3_LC | . | . | . | . | . | . | . | . | . | . | . | . | . | . | . | . | . | . | . | . | . | . | . | . | . | . |
| 21-225_190D10_LC | . | . | . | . | . | . | S | . | . | . | . | . | . | . | . | . | . | . | . | . | . | . | . | . | V | . |
| 21-225_190D8_LC | . | . | . | . | . | . | . | . | . | . | . | . | H | . | . | . | . | . | . | . | . | . | . | . | . | . |
| 21-225_190H7_LC | . | . | . | . | . | . | S | . | . | . | . | . | . | . | . | . | . | . | . | . | . | . | . | . | . | . |
| 21-225_191A1_LC | . | . | . | . | L | . | . | . | . | . | . | . | . | F | . | . | . | . | . | . | F | . | . | . | . | . |
| 21-225_192G2_LC | . | . | . | . | . | . | . | . | . | . | . | . | . | . | . | . | . | . | . | . | . | . | . | . | . | . |
| 21-225_194F11_LC | . | . | . | . | . | . | . | . | . | . | . | . | . | . | . | . | . | . | . | . | . | . | . | . | . | . |
| 21-225_194G12_LC | . | . | . | . | . | . | . | . | . | . | . | . | . | . | . | . | . | . | . | . | . | . | . | . | . | . |
| 21-225_197E8_LC | . | . | . | . | . | . | . | . | . | . | . | . | K | . | . | . | . | . | . | . | F | . | . | . | . | . |
| 21-225_198D2_LC | . | . | . | . | . | . | S | . | . | . | . | . | . | . | . | . | . | . | . | . | . | . | . | . | . | . |
| 21-225_200G8_LC | . | . | . | . | L | . | . | . | . | . | . | . | . | F | . | . | . | . | . | . | F | . | . | . | . | . |
| 21-225_209A8_LC | . | . | . | . | . | . | S | . | . | . | . | . | . | F | L | . | . | . | . | . | . | . | . | . | . | . |
| 21-225_214D8_LC | . | . | . | . | . | . | . | . | . | . | . | . | . | . | . | . | . | . | . | . | F | . | . | . | . | . |
| 21-225_218G4_LC | . | . | . | . | . | . | . | . | . | . | . | . | . | . | . | . | . | . | . | . | . | . | . | . | . | . |
| 21-225_58C5_LC | . | . | . | . | . | . | . | . | . | . | . | . | . | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | L_CDR2 | | | | | | | | | | | | | |
| CONSENSUS | K | L | L | I | Y | D | - | - | - | - | - | - | - | - | N | N | K | R | P | S | G | I | P | D | R | F |
| 21-225_190B10_LC | . | . | . | . | . | . | . | . | . | . | . | . | . | . | . | . | . | . | . | . | . | . | . | . | . | . |
| 21-225_190C3_LC | . | . | . | L | . | . | . | . | . | . | . | . | . | . | S | . | . | . | . | . | . | . | . | G | . | . |
| 21-225_190D10_LC | . | V | . | . | . | . | . | . | . | . | . | . | . | . | . | . | . | . | . | . | . | . | . | . | . | . |
| 21-225_190D8_LC | . | . | . | . | . | . | . | . | . | . | . | . | . | . | S | S | . | . | . | . | E | . | . | . | . | . |
| 21-225_190H7_LC | . | V | . | . | . | . | . | . | . | . | . | . | . | . | . | . | . | . | . | . | . | . | . | . | . | . |
| 21-225_191A1_LC | . | . | . | . | . | . | . | . | . | . | . | . | . | . | . | Y | . | . | . | . | . | . | . | . | . | . |
| 21-225_192G2_LC | . | . | . | . | . | . | . | . | . | . | . | . | . | . | . | . | . | . | . | . | R | . | . | . | . | . |
| 21-225_194F11_LC | . | . | . | . | . | . | . | . | . | . | . | . | . | . | . | . | . | . | . | . | . | . | . | . | . | . |
| 21-225_194G12_LC | . | . | . | . | . | . | . | . | . | . | . | . | . | . | . | . | . | . | . | . | R | . | . | . | . | . |
| 21-225_197E8_LC | . | . | . | . | . | . | . | . | . | . | . | . | . | . | . | . | . | . | . | . | K | . | . | . | . | . |
| 21-225_198D2_LC | . | V | . | . | . | . | . | . | . | . | . | . | . | . | . | . | . | . | . | . | . | . | . | . | . | . |
| 21-225_200G8_LC | . | . | . | . | . | . | . | . | . | . | . | . | . | . | . | Y | . | . | . | . | . | . | . | . | . | . |
| 21-225_209A8_LC | . | . | . | . | . | . | . | . | . | . | . | . | . | . | . | . | . | . | . | . | . | . | . | . | . | . |
| 21-225_214D8_LC | . | . | . | . | H | . | . | . | . | . | . | . | . | . | . | . | . | . | . | . | . | . | . | . | . | . |
| 21-225_218G4_LC | . | . | . | L | . | . | . | . | . | . | . | . | . | . | S | . | . | . | . | . | . | . | . | A | . | . |
| 21-225_58C5_LC | . | . | . | . | . | . | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | L_FR3 | | | | | | | | | | | | | | | | | | | | | | | | | |
| CONSENSUS | S | G | S | K | S | G | - | - | T | S | A | T | L | G | I | T | G | L | Q | T | G | D | E | A | D | Y |
| 21-225_190B10_LC | . | . | . | . | . | . | . | . | . | . | . | . | . | . | . | . | . | . | . | . | . | . | . | . | . | . |
| 21-225_190C3_LC | . | . | . | . | . | . | . | . | . | . | . | . | . | . | . | . | . | . | . | . | . | . | . | . | . | . |
| 21-225_190D10_LC | . | . | . | . | . | . | . | . | . | . | . | . | . | . | . | . | . | . | . | . | . | . | . | . | . | . |
| 21-225_190D8_LC | . | . | . | . | . | . | . | . | . | . | . | . | . | . | . | . | . | . | . | . | . | . | . | . | . | . |
| 21-225_190H7_LC | . | . | . | . | . | . | . | . | . | . | . | . | . | . | . | . | . | . | . | . | . | . | . | . | . | . |
| 21-225_191A1_LC | . | V | . | . | . | . | . | . | . | . | . | . | . | . | . | . | . | . | . | . | . | . | . | . | . | . |
| 21-225_192G2_LC | . | . | . | . | . | . | . | . | . | . | . | . | . | . | . | . | . | . | . | . | . | . | . | . | . | . |
| 21-225_194F11_LC | . | . | . | . | . | . | . | . | . | . | . | . | . | . | . | . | . | . | . | . | . | . | . | . | . | . |
| 21-225_194G12_LC | . | . | . | . | . | . | . | . | . | . | . | . | . | . | . | . | . | . | . | . | . | E | . | . | . | . |
| 21-225_197E8_LC | . | . | . | . | . | . | . | . | . | . | . | . | . | . | . | . | . | . | L | . | . | . | . | . | . | . |
| 21-225_198D2_LC | . | . | . | . | . | . | . | . | . | . | . | . | . | . | . | . | . | . | . | . | . | . | . | . | . | . |
| 21-225_200G8_LC | . | V | . | . | . | . | . | . | . | . | . | . | . | . | . | . | . | . | . | . | . | . | . | . | . | . |
| 21-225_209A8_LC | . | . | . | . | . | . | . | . | . | . | . | . | . | . | . | . | . | . | . | . | . | . | . | . | . | . |
| 21-225_214D8_LC | . | . | . | . | . | . | . | . | . | . | . | . | . | . | . | . | . | . | . | . | . | . | . | . | . | . |
| 21-225_218G4_LC | . | . | . | . | . | . | . | . | . | . | . | . | . | . | . | . | . | . | . | . | . | . | . | . | . | . |
| 21-225_58C5_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | L_CDR3 | | | | | | | | | | | | | | | | | | | | | | | |
| CONSENSUS | Y | C | G | T | W | D | S | S | - | - | - | - | - | - | - | - | - | - | - | - | - | - | - | - | - | - |
| 21-225_190B10_LC | . | . | . | . | . | . | . | . | . | . | . | . | . | . | . | . | . | . | . | . | . | . | . | . | . | . |
| 21-225_190C3_LC | . | . | . | . | . | . | . | . | . | . | . | . | . | . | . | . | . | . | . | . | . | . | . | . | . | . |
| 21-225_190D10_LC | . | . | . | . | . | . | . | R | . | . | . | . | . | . | . | . | . | . | . | . | . | . | . | . | . | . |
| 21-225_190D8_LC | . | . | . | . | . | . | . | . | . | . | . | . | . | . | . | . | . | . | . | . | . | . | . | . | . | . |
| 21-225_190H7_LC | . | . | . | . | . | . | G | R | . | . | . | . | . | . | . | . | . | . | . | . | . | . | . | . | . | . |
| 21-225_191A1_LC | . | . | . | . | . | . | . | . | . | . | . | . | . | . | . | . | . | . | . | . | . | . | . | . | . | . |
| 21-225_192G2_LC | . | . | . | . | . | . | . | . | . | . | . | . | . | . | . | . | . | . | . | . | . | . | . | . | . | . |
| 21-225_194F11_LC | . | . | . | . | . | . | . | . | . | . | . | . | . | . | . | . | . | . | . | . | . | . | . | . | . | . |
| 21-225_194G12_LC | . | . | . | . | . | . | . | . | . | . | . | . | . | . | . | . | . | . | . | . | . | . | . | . | . | . |
| 21-225_197E8_LC | . | . | . | I | . | . | R | . | . | . | . | . | . | . | . | . | . | . | . | . | . | . | . | . | . | . |
| 21-225_198D2_LC | . | . | . | . | . | . | . | R | . | . | . | . | . | . | . | . | . | . | . | . | . | . | . | . | . | . |
| 21-225_200G8_LC | . | . | . | . | . | . | . | . | . | . | . | . | . | . | . | . | . | . | . | . | . | . | . | . | . | . |
| 21-225_209A8_LC | . | . | . | . | . | . | . | . | . | . | . | . | . | . | . | . | . | . | . | . | . | . | . | . | . | . |
| 21-225_214D8_LC | . | . | . | A | . | . | . | . | . | . | . | . | . | . | . | . | . | . | . | . | . | . | . | . | . | . |
| 21-225_218G4_LC | . | . | . | . | . | . | . | . | . | . | . | . | . | . | . | . | . | . | . | . | . | . | . | . | . | . |
| 21-225_58C5_LC | . | . | . | . | . | . | I | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | L_FR4 | | | | | | | | | | |
| CONSENSUS | - | - | - | L | S | V | G | V | F | G | G | G | T | K | L | T | V | L | G |
| 21-225_190B10_LC | . | . | . | . | . | A | . | . | . | . | . | . | . | . | . | . | . | . | . |
| 21-225_190C3_LC | . | . | . | . | . | T | V | . | . | . | . | . | . | . | . | . | . | . | . |
| 21-225_190D10_LC | . | . | . | . | N | . | . | . | . | . | . | . | . | . | . | . | . | . | . |
| 21-225_190D8_LC | . | . | . | . | . | T | V | . | . | . | . | . | . | . | . | . | . | . | . |
| 21-225_190H7_LC | . | . | . | . | N | . | . | . | . | . | . | . | . | . | . | . | . | . | . |
| 21-225_191A1_LC | . | . | . | . | N | T | . | . | . | . | . | . | . | . | . | . | . | . | . |
| 21-225_192G2_LC | . | . | . | . | . | A | . | . | . | . | . | . | . | . | . | . | . | . | . |
| 21-225_194F11_LC | . | . | . | . | . | A | . | . | . | . | . | . | . | . | . | . | . | . | . |
| 21-225_194G12_LC | . | . | . | . | . | . | . | . | . | . | . | . | . | . | . | . | . | . | . |
| 21-225_197E8_LC | . | . | . | . | . | . | M | . | . | . | . | . | . | . | . | . | . | . | . |
| 21-225_198D2_LC | . | . | . | . | N | . | . | . | . | . | . | . | . | . | . | . | . | . | . |
| 21-225_200G8_LC | . | . | . | . | N | T | . | . | . | . | . | . | . | . | . | . | . | . | . |
| 21-225_209A8_LC | . | . | . | . | . | A | . | . | . | . | . | . | . | . | . | . | . | . | . |
| 21-225_214D8_LC | . | . | . | . | . | A | V | . | I | . | . | . | S | . | . | . | . | . | . |
| 21-225_218G4_LC | . | . | . | . | N | T | V | . | . | . | . | . | . | . | . | . | . | . | S |
| 21-225_58C5_LC | . | . | . | . | . | . | . | . | . | . | . | . | . | . | . | . | . | . | . |

**FIGURE 57**

Table 119. Consensus 39 - VK2|A19/JK4 (SEQ ID NO: 50336):

DIVMTQSPLSLPVTPGEPASISCRSS--QSLLHSN-GYNYLDWYLQKPGQSPQLLIYL--------GSNRASGVPDRFSGSGSG--TDFTLKISRVEAEDVGVYYCMQALH-----------------------PPLTFGGGTKVEIKR wherein:

S at position 25 can be substituted with Y

L at position 32 can be substituted with V

H at position 33 can be substituted with Y

S at position 34 can be substituted with N or H

N at position 35 can be substituted with S

G at position 37 can be substituted with K or R

Y at position 38 can be substituted with H or N

Y at position 40 can be substituted with H or S

L at position 58 can be substituted with V

N at position 69 can be substituted with H

A at position 109 can be substituted with P, T or V

H at position 111 can be substituted with Q or null (-)

Null (-) at position 134 can be substituted with T

P at position 135 can be substituted with Q, T or I

P at position 136 can be substituted with T

L at position 137 can be substituted with P or F

Arg Xaa Ser Gln Ser Leu Xaa Xaa Xaa Xaa Xaa Xaa Asn Xaa Leu Asp (SEQ ID NO: 50243)

**FIGURE 57**

Xaa Gly Ser Xaa Arg Ala Ser (SEQ ID NO: 50244)

Met Gln Xaa Leu Xaa Xaa Xaa Xaa Xaa Thr (SEQ ID NO: 50245)

Arg Ser Ser Gln Ser Leu Leu His Ser Asn Gly Tyr Asn Tyr Leu Asp (SEQ ID NO: 50678)

Leu Gly Ser Asn Arg Ala Ser (SEQ ID NO: 50679)

Met Gln Ala Leu His Pro Pro Leu Thr (SEQ ID NO: 50680)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | V | M | T | Q | S | P | L | S | L | P | V | T | P | G | E | P | A | S | I | S | C | R | S | S |
| 21-225_171D7_LC | . | . | . | . | . | . | . | . | . | . | . | . | . | . | . | . | . | . | . | . | . | . | . | . | . | . |
| 21-225_17B8_LC | . | . | . | . | . | . | . | . | . | . | . | . | . | . | . | . | . | . | . | . | . | . | . | . | . | . |
| 21-225_201F3_LC | . | . | . | . | . | . | . | . | . | . | . | . | . | . | . | . | . | . | . | . | . | . | . | . | . | . |
| 21-225_201G6_LC | . | . | . | . | . | . | . | . | . | . | . | . | . | . | . | . | . | . | . | . | . | . | . | . | . | . |
| 21-225_202C12_LC | . | . | . | L | . | . | . | . | . | . | . | . | . | . | . | . | . | . | . | . | . | . | . | . | . | . |
| 21-225_208G3_LC | . | . | . | L | . | . | . | . | . | . | . | . | . | . | . | . | . | . | . | . | . | . | . | . | . | . |
| 21-225_74A2_LC | . | . | . | . | . | . | . | . | . | . | . | . | . | . | . | . | . | . | . | . | . | . | . | . | . | . |
| 21-225_74D8_LC | . | . | . | . | . | . | . | . | . | . | . | . | . | . | . | . | . | . | . | . | . | . | . | . | . | . |
| 21-225_75A1_LC | . | . | . | . | . | . | . | . | . | . | . | . | . | . | . | . | . | . | . | . | . | . | . | . | . | . |
| 21-225_75D8_LC | . | . | . | . | . | . | . | . | . | . | . | . | . | . | . | . | . | . | . | . | . | . | . | . | . | . |
| 21-225_89G4_LC | . | . | . | . | . | . | . | . | . | . | . | . | . | . | . | . | . | . | . | . | . | . | . | . | . | . |
| 21-225_90C11_LC | . | . | . | . | . | . | . | . | . | . | . | . | . | . | . | . | . | . | . | . | . | . | . | . | Y | . |
| 21-225_92B2_LC | . | . | . | . | . | . | . | . | . | . | . | . | . | . | . | . | . | . | . | . | . | . | . | . | . | . |
| 21-225_97H3_LC | . | . | . | . | I | . | . | . | . | . | . | . | . | . | . | . | . | . | . | . | . | . | . | . | . | . |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | L | L | H | S | N | - | G | Y | N | Y | L | D | W | Y | L | Q | K | P | G | Q | S | P |
| 21-225_171D7_LC | . | . | . | . | . | . | Y | H | . | . | . | . | . | . | . | . | . | . | . | . | . | T | . | . | . | . |
| 21-225_17B8_LC | . | . | . | . | . | . | . | . | . | . | . | N | . | S | . | . | . | . | . | . | . | . | . | . | . | . |
| 21-225_201F3_LC | . | . | . | . | . | . | . | N | . | . | R | . | . | H | . | . | . | . | . | . | . | . | . | . | . | . |
| 21-225_201G6_LC | . | . | . | . | . | . | . | N | . | . | R | . | . | H | . | . | . | . | . | . | . | . | . | . | . | . |
| 21-225_202C12_LC | . | . | . | . | . | . | . | N | . | . | K | . | . | H | . | . | . | . | . | . | . | . | . | . | . | . |
| 21-225_208G3_LC | . | . | . | . | . | . | . | N | . | . | R | . | . | H | . | . | . | . | . | . | . | . | . | . | . | . |
| 21-225_74A2_LC | . | . | . | . | . | . | . | . | . | . | . | H | . | . | . | . | . | . | . | . | . | . | . | R | . | . |
| 21-225_74D8_LC | . | . | . | . | . | V | . | . | S | . | . | . | . | . | . | . | . | . | . | . | . | . | . | . | . | . |
| 21-225_75A1_LC | . | . | . | . | . | . | . | . | . | . | . | H | . | . | . | . | . | . | . | . | . | . | . | R | . | . |
| 21-225_75D8_LC | . | . | . | . | . | . | . | . | S | . | . | . | . | . | . | . | . | . | . | . | . | . | . | . | . | . |
| 21-225_89G4_LC | . | . | . | . | . | . | . | . | S | . | . | . | . | . | . | . | . | . | . | . | . | . | . | . | . | . |
| 21-225_90C11_LC | . | . | . | . | . | V | . | . | S | . | . | . | . | . | . | . | . | . | . | . | . | . | . | . | . | . |
| 21-225_92B2_LC | . | . | . | . | . | V | . | . | S | . | . | . | . | . | . | . | . | . | . | . | . | . | . | . | . | . |
| 21-225_97H3_LC | . | . | . | . | . | . | . | . | . | . | . | H | . | . | . | . | . | . | . | . | . | . | . | R | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | Q | L | L | I | Y | L | - | - | - | - | - | - | - | - | G | S | N | R | A | S | G | V | P | D | R | F |
| 21-225_171D7_LC | . | . | . | . | . | V | . | . | . | . | . | . | . | . | . | . | . | . | . | . | . | . | . | . | . | . |
| 21-225_17B8_LC | . | . | . | . | . | . | . | . | . | . | . | . | . | . | . | . | H | . | . | . | . | . | . | . | . | . |
| 21-225_201F3_LC | . | . | . | . | . | . | . | . | . | . | . | . | . | . | . | . | . | . | . | . | . | . | . | . | . | . |
| 21-225_201G6_LC | . | . | . | . | . | . | . | . | . | . | . | . | . | . | . | . | . | . | . | . | . | . | . | . | . | . |
| 21-225_202C12_LC | . | . | . | . | . | . | . | . | . | . | . | . | . | . | . | . | . | . | . | . | . | . | . | . | . | . |
| 21-225_208G3_LC | . | . | . | . | . | . | . | . | . | . | . | . | . | . | . | . | . | . | . | . | . | . | . | . | . | . |
| 21-225_74A2_LC | . | . | . | . | . | . | . | . | . | . | . | . | . | . | . | . | . | . | . | . | . | . | . | E | . | . |
| 21-225_74D8_LC | . | . | V | . | . | . | . | . | . | . | . | . | . | . | . | . | . | . | . | . | . | . | . | . | . | . |
| 21-225_75A1_LC | . | . | . | . | . | . | . | . | . | . | . | . | . | . | . | . | . | . | . | . | . | . | . | E | . | . |
| 21-225_75D8_LC | . | . | . | . | . | . | . | . | . | . | . | . | . | . | . | . | . | . | . | . | . | . | . | . | . | . |
| 21-225_89G4_LC | . | . | . | . | . | . | . | . | . | . | . | . | . | . | . | . | . | . | . | . | . | . | . | . | . | . |
| 21-225_90C11_LC | . | . | V | . | . | . | . | . | . | . | . | . | . | . | . | . | . | . | . | . | . | . | . | . | . | . |
| 21-225_92B2_LC | . | . | V | . | . | . | . | . | . | . | . | . | . | . | . | . | . | . | . | . | . | . | . | . | . | . |
| 21-225_97H3_LC | . | . | . | . | . | . | . | . | . | . | . | . | . | . | . | . | . | . | . | . | . | . | . | E | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | K | I | S | R | V | E | A | E | D | V | G | V | Y |
| 21-225_171D7_LC | . | . | . | . | . | . | . | . | . | . | . | . | . | . | . | . | . | . | . | . | . | . | . | . | . | . |
| 21-225_17B8_LC | . | D | . | . | . | . | . | . | . | . | . | . | . | . | . | . | . | . | . | . | . | . | . | . | . | . |
| 21-225_201F3_LC | . | . | . | . | . | . | . | . | . | . | . | . | . | . | . | . | . | . | . | . | . | . | . | . | . | . |
| 21-225_201G6_LC | . | . | . | . | . | . | . | . | . | . | . | . | . | . | . | . | . | . | . | . | . | . | . | . | . | . |
| 21-225_202C12_LC | . | . | . | . | . | . | . | . | . | . | . | . | . | . | . | . | . | . | . | . | . | . | . | . | . | . |
| 21-225_208G3_LC | . | . | . | . | . | . | . | . | . | . | . | . | . | . | . | . | . | . | . | . | . | . | . | . | . | . |
| 21-225_74A2_LC | . | . | . | . | . | . | . | . | . | . | . | . | . | . | . | . | . | . | . | . | . | . | . | . | . | . |
| 21-225_74D8_LC | . | . | . | . | . | . | . | . | S | . | . | . | . | . | . | . | . | . | . | . | . | . | . | . | L | . |
| 21-225_75A1_LC | . | . | . | . | . | . | . | . | . | . | . | . | . | . | . | . | . | . | . | . | . | . | . | . | . | . |
| 21-225_75D8_LC | . | . | . | . | . | . | . | . | S | . | . | . | . | . | . | . | . | . | . | . | . | . | . | . | L | . |
| 21-225_89G4_LC | . | . | . | . | . | . | . | . | . | . | . | . | . | . | . | . | . | . | . | . | . | . | I | . | . | . |
| 21-225_90C11_LC | . | . | . | . | . | . | . | . | S | . | . | . | . | . | . | . | . | . | . | . | . | . | . | . | L | . |
| 21-225_92B2_LC | . | . | . | . | . | V | . | . | . | . | . | . | . | R | . | . | . | . | . | . | . | . | I | . | I | . |
| 21-225_97H3_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | | K_CDR3 | | | | | | | |
| CONSENSUS | Y | C | M | Q | A | L | | - | - | - | - | - | - | - | - | - | - | - | - | - | - | - | - | - | - | - |
| 21-225_171D7_LC | . | . | . | . | T | . | Q | . | . | . | . | . | . | . | . | . | . | . | . | . | . | . | . | . | . | . |
| 21-225_17B8_LC | . | . | . | . | V | . | Q | . | . | . | . | . | . | . | . | . | . | . | . | . | . | . | . | . | . | . |
| 21-225_201F3_LC | . | . | . | . | . | . | - | . | . | . | . | . | . | . | . | . | . | . | . | . | . | . | . | . | . | . |
| 21-225_201G6_LC | . | . | . | . | . | . | - | . | . | . | . | . | . | . | . | . | . | . | . | . | . | . | . | . | . | . |
| 21-225_202C12_LC | . | . | . | . | . | . | - | . | . | . | . | . | . | . | . | . | . | . | . | . | . | . | . | . | . | . |
| 21-225_208G3_LC | . | . | . | . | . | . | - | . | . | . | . | . | . | . | . | . | . | . | . | . | . | . | . | . | . | . |
| 21-225_74A2_LC | . | . | . | . | P | . | Q | . | . | . | . | . | . | . | . | . | . | . | . | . | . | . | . | . | . | . |
| 21-225_74D8_LC | . | . | . | . | . | . | H | . | . | . | . | . | . | . | . | . | . | . | . | . | . | . | . | . | . | . |
| 21-225_75A1_LC | . | . | . | . | P | . | Q | . | . | . | . | . | . | . | . | . | . | . | . | . | . | . | . | . | . | . |
| 21-225_75D8_LC | . | . | . | . | . | . | H | . | . | . | . | . | . | . | . | . | . | . | . | . | . | . | . | . | . | . |
| 21-225_89G4_LC | . | . | . | . | . | . | H | . | . | . | . | . | . | . | . | . | . | . | . | . | . | . | . | . | . | . |
| 21-225_90C11_LC | . | . | . | . | . | . | H | . | . | . | . | . | . | . | . | . | . | . | . | . | . | . | . | . | . | . |
| 21-225_92B2_LC | . | . | . | . | . | . | H | . | . | . | . | . | . | . | . | . | . | . | . | . | . | . | . | . | . | . |
| 21-225_97H3_LC | . | . | . | . | P | . | Q | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | | P | L | T | F | G | G | G | T | K | V | E | I | K | R |
| 21-225_171D7_LC | . | . | . | . | T | . | . | . | . | . | . | . | . | . | . | . | . | . | . |
| 21-225_17B8_LC | . | . | . | T | P | . | . | . | . | . | . | . | . | . | . | . | . | . | . |
| 21-225_201F3_LC | . | . | . | . | Q | T | P | . | . | . | . | . | . | . | . | . | . | . | G |
| 21-225_201G6_LC | . | . | . | . | Q | T | P | . | . | . | . | . | . | . | . | . | . | . | G |
| 21-225_202C12_LC | . | . | . | . | Q | T | P | . | . | . | . | . | . | . | . | . | . | . | . |
| 21-225_208G3_LC | . | . | . | . | Q | T | P | . | . | . | . | . | . | . | . | . | . | . | . |
| 21-225_74A2_LC | . | . | . | . | T | . | P | . | . | . | . | . | . | . | . | . | . | . | . |
| 21-225_74D8_LC | . | . | . | . | P | . | . | . | . | . | . | . | . | . | . | . | . | . | . |
| 21-225_75A1_LC | . | . | . | . | T | . | P | . | . | . | . | . | . | . | . | . | . | . | . |
| 21-225_75D8_LC | . | . | . | . | P | . | . | . | . | . | . | . | . | . | . | . | . | . | . |
| 21-225_89G4_LC | . | . | . | . | I | . | . | . | . | . | . | . | . | . | . | . | . | . | . |
| 21-225_90C11_LC | . | . | . | . | P | . | . | . | . | . | . | . | . | . | . | . | . | . | . |
| 21-225_92B2_LC | . | . | . | . | I | . | . | . | . | . | . | . | . | . | . | . | . | . | . |
| 21-225_97H3_LC | . | . | . | . | T | . | P | . | . | . | . | . | . | . | . | . | . | . | . |

**FIGURE 57**

Table 120. Consensus 40 - VL1|1c/JL2 (SEQ ID NO: 50337):

QSVLTQP-PSASGTPGQRVTISCSGSS-SNIGS-----NTVNWYQQLPGTAPKLLIYS--------NNQRPSGVPDRFSGSKSG--TSASLAISGLQSEDEADYYCAAWDDS---------------------LNGVVFGGGTKLTVLG wherein:

S at position 26 can be substituted with T

S at position 27 can be substituted with N

N at position 30 can be substituted with Y

S at position 33 can be substituted with N

N at position 39 can be substituted with Y

T at position 40 can be substituted with A or S

V at position 41 can be substituted with I

N at position 42 can be substituted with D or S

S at position 58 can be substituted with I

N at position 67 can be substituted with S

N at position 68 can be substituted with D or S

Q at position 69 can be substituted with H

A at position 107 can be substituted with E

S at position 112 can be substituted with null (-)

null (-) at position 133 can be substituted with L

L at position 134 can be substituted with N, M or S

N at position 135 can be substituted with G, K or L

**FIGURE 57**

G at position 136 can be substituted with H or N

V at position 137 can be substituted with P or G

V at position 138 can be substituted with P

Ser Gly Xaa Xaa Ser Xaa Ile Gly Xaa Xaa Xaa Xaa Xaa (SEQ ID NO: 50192)

Xaa Xaa Xaa Xaa Arg Pro Ser (SEQ ID NO: 50193)

Xaa Ala Trp Asp Asp Xaa Xaa Xaa Xaa Xaa Xaa Xaa (SEQ ID NO: 50194)

Ser Gly Ser Ser Ser Asn Ile Gly Ser Asn Thr Val Asn (SEQ ID NO: 50681)

Ser Asn Asn Gln Arg Pro Ser (SEQ ID NO: 50682)

Ala Ala Trp Asp Asp Ser Leu Asn Gly Val Val (SEQ ID NO: 50683)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | L_FR1 | | | | | | | | | | | | | | | | | | | | | | | | | |
| CONSENSUS | Q | S | V | L | T | Q | P | - | P | S | A | S | G | T | P | G | Q | R | V | T | I | S | C | S | G | S |
| 21-225_146A8_LC | . | . | . | . | . | . | . | . | . | . | . | . | . | . | . | . | . | . | . | . | . | . | . | . | . | . |
| 21-225_146B6_LC | . | . | . | . | . | . | . | . | . | . | . | . | . | . | . | . | . | . | . | . | . | . | . | . | . | . |
| 21-225_146D8_LC | . | . | . | . | . | . | . | . | . | . | T | . | . | . | . | . | . | . | . | . | . | . | . | . | . | . |
| 21-225_147H12_LC | . | . | . | . | . | . | . | . | . | . | . | . | . | . | . | . | . | . | . | . | . | . | . | . | . | . |
| 21-225_149A1_LC | . | . | . | . | . | . | . | . | . | . | . | . | . | . | . | . | . | . | . | . | . | . | . | . | . | . |
| 21-225_150F9_LC | . | . | . | . | . | . | . | . | . | . | . | . | . | . | . | . | . | . | . | . | . | . | . | . | . | . |
| 21-225_154G12_LC | . | . | . | . | . | . | . | . | . | . | . | . | . | . | . | . | . | . | . | . | . | . | . | . | . | . |
| 21-225_156H2_LC | . | . | . | V | . | . | . | . | . | . | . | . | . | . | . | . | . | . | . | . | . | . | . | . | . | . |
| 21-225_197F9_LC | . | . | . | . | . | . | . | . | . | . | . | . | . | . | . | . | . | . | . | . | . | . | . | . | . | . |
| 21-225_199C3_LC | . | . | . | . | . | . | . | . | . | . | . | . | . | . | . | . | . | . | . | . | . | . | . | . | . | . |
| 21-225_4G12_LC | . | . | . | . | . | . | . | . | . | . | . | . | . | . | . | . | . | . | . | N | M | . | . | . | . | T |
| 21-225_60D6_LC | . | . | . | . | . | . | . | . | . | . | . | . | . | . | . | . | . | . | . | . | . | . | . | . | . | . |
| 21-225_9G9_LC | . | . | . | . | . | . | . | . | . | . | . | . | . | . | . | . | . | . | . | N | M | . | . | . | . | T |

**FIGURE 57**

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | L_CDR1 | | | | | | | | | | | | | | | | L_FR2 | | | | | | | | | |
| CONSENSUS | S | - | S | N | I | G | S | - | - | - | - | - | N | T | V | N | W | Y | Q | Q | L | P | G | T | A | P |
| 21-225_146A8_LC | . | . | . | . | . | . | . | . | . | . | . | . | . | S | I | . | . | . | . | . | . | . | R | . | . | . |
| 21-225_146B6_LC | . | . | . | . | . | . | . | . | . | . | . | . | . | A | . | . | . | . | . | . | . | . | . | . | . | . |
| 21-225_146D8_LC | . | . | . | Y | . | . | . | . | . | . | . | . | . | . | . | D | . | . | . | . | . | . | . | . | . | . |
| 21-225_147H12_LC | . | . | . | . | . | . | . | . | . | . | . | . | Y | A | . | . | . | . | . | . | . | . | . | . | . | . |
| 21-225_149A1_LC | . | . | . | . | . | . | . | . | . | . | . | . | . | A | . | . | . | . | . | . | . | . | . | . | . | . |
| 21-225_150F9_LC | . | . | . | . | . | . | . | . | . | . | . | . | . | A | . | . | . | . | . | . | . | . | . | . | . | . |
| 21-225_154G12_LC | . | . | . | . | . | . | N | . | . | . | . | . | . | A | . | S | . | . | . | . | . | . | . | . | . | . |
| 21-225_156H2_LC | . | . | . | . | . | . | . | . | . | . | . | . | . | . | . | . | . | . | . | . | . | . | . | . | . | . |
| 21-225_197F9_LC | . | . | . | . | . | . | . | . | . | . | . | . | . | . | . | . | . | . | . | . | . | . | . | . | . | . |
| 21-225_199C3_LC | . | . | . | . | . | . | . | . | . | . | . | . | . | . | . | . | . | . | . | . | . | . | . | . | . | . |
| 21-225_4G12_LC | N | . | . | . | . | . | . | . | . | . | . | . | Y | . | . | . | . | . | . | . | . | . | . | . | . | . |
| 21-225_60D6_LC | . | . | . | . | . | . | . | . | . | . | . | . | . | . | . | . | . | . | . | . | . | . | . | . | . | . |
| 21-225_9G9_LC | N | . | . | . | . | . | . | . | . | . | . | . | Y | . | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | L_CDR2 | | | | | | | | | | | | | | | | | | | | |
| CONSENSUS | K | L | L | I | Y | S | - | - | - | - | - | - | - | - | N | N | Q | R | P | S | G | V | P | D | R | F |
| 21-225_146A8_LC | . | . | . | . | . | . | . | . | . | . | . | . | . | . | . | D | . | . | . | . | . | . | . | . | . | . |
| 21-225_146B6_LC | . | . | . | . | . | . | . | . | . | . | . | . | . | . | . | . | . | . | . | . | . | . | . | . | . | . |
| 21-225_146D8_LC | . | . | . | . | . | . | . | . | . | . | . | . | . | . | . | . | . | . | . | . | . | . | . | . | . | . |
| 21-225_147H12_LC | . | . | . | . | . | . | . | . | . | . | . | . | . | . | . | . | H | . | . | . | . | . | . | . | . | . |
| 21-225_149A1_LC | . | . | . | . | . | . | . | . | . | . | . | . | . | . | . | . | . | . | . | . | . | . | . | . | . | . |
| 21-225_150F9_LC | . | . | . | . | . | . | . | . | . | . | . | . | . | . | . | S | . | . | . | . | . | . | . | . | . | . |
| 21-225_154G12_LC | . | . | . | . | . | . | . | . | . | . | . | . | . | . | . | D | H | . | . | . | . | . | . | . | . | . |
| 21-225_156H2_LC | . | . | . | . | . | . | . | . | . | . | . | . | . | . | S | . | . | . | . | . | . | . | . | . | . | L |
| 21-225_197F9_LC | . | . | . | . | . | . | . | . | . | . | . | . | . | . | . | . | . | . | . | . | . | . | . | . | . | . |
| 21-225_199C3_LC | . | . | . | . | . | . | . | . | . | . | . | . | . | . | . | . | . | . | . | . | . | . | . | . | . | . |
| 21-225_4G12_LC | . | . | . | . | . | I | . | . | . | . | . | . | . | . | . | . | . | . | . | . | . | . | . | . | . | . |
| 21-225_60D6_LC | . | . | . | . | . | . | . | . | . | . | . | . | . | . | . | . | . | . | . | . | . | . | . | . | . | . |
| 21-225_9G9_LC | . | . | . | . | . | I | . | . | . | . | . | . | . | . | . | . | . | . | . | . | . | . | . | . | . | . |

FIGURE 57

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | L_FR3 | | | | | | | | | | | | | | | | | | | | | | | | | |
| CONSENSUS | S | G | S | K | S | G | - | - | T | S | A | S | L | A | I | S | G | L | Q | S | E | D | E | A | D | Y |
| 21-225_146A8_LC | . | . | . | . | . | . | . | . | . | . | . | . | . | . | . | . | . | . | . | . | . | . | . | . | . | . |
| 21-225_146B6_LC | . | . | . | . | . | . | . | . | . | . | . | . | . | . | . | . | . | . | . | . | . | . | . | . | . | . |
| 21-225_146D8_LC | . | . | . | . | . | . | . | . | . | . | . | . | . | . | . | . | . | . | . | . | . | . | . | . | . | . |
| 21-225_147H12_LC | . | . | . | . | . | . | . | . | . | . | . | . | . | . | . | . | . | . | . | . | . | . | . | . | . | . |
| 21-225_149A1_LC | . | . | . | . | . | . | . | . | . | . | . | . | . | . | . | . | . | . | . | . | . | . | . | . | . | . |
| 21-225_150F9_LC | . | . | . | . | . | . | . | . | . | . | . | . | . | . | . | . | . | . | . | . | . | . | . | . | . | . |
| 21-225_154G12_LC | . | . | . | . | . | . | . | . | . | . | . | . | . | . | . | . | . | . | . | . | . | . | . | . | . | . |
| 21-225_156H2_LC | . | . | . | . | . | . | . | . | . | . | . | . | . | . | . | . | . | . | . | . | . | . | . | . | . | . |
| 21-225_197F9_LC | . | . | . | . | . | . | . | . | . | . | . | . | . | . | . | . | . | . | . | . | . | . | . | . | . | . |
| 21-225_199C3_LC | . | . | . | . | . | . | . | . | . | . | . | . | . | . | . | . | . | . | . | . | . | . | . | . | . | . |
| 21-225_4G12_LC | . | . | . | . | . | . | . | . | . | . | . | . | . | . | . | . | . | . | . | . | . | . | . | . | . | . |
| 21-225_60D6_LC | . | . | . | . | . | . | . | . | . | . | . | . | . | . | . | . | . | . | . | . | . | . | . | . | . | . |
| 21-225_9G9_LC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | L_CDR3 | | | | | | | | | | | | | | | | | | | | | | | |
| CONSENSUS | Y | C | A | A | W | D | D | S | - | - | - | - | - | - | - | - | - | - | - | - | - | - | - | - | - | - |
| 21-225_146A8_LC | . | . | . | . | . | . | . | . | . | . | . | . | . | . | . | . | . | . | . | . | . | . | . | . | . | . |
| 21-225_146B6_LC | . | . | . | . | . | . | . | . | . | . | . | . | . | . | . | . | . | . | . | . | . | . | . | . | . | . |
| 21-225_146D8_LC | . | . | . | . | . | . | . | . | . | . | . | . | . | . | . | . | . | . | . | . | . | . | . | . | . | . |
| 21-225_147H12_LC | . | . | . | . | . | . | . | . | . | . | . | . | . | . | . | . | . | . | . | . | . | . | . | . | . | . |
| 21-225_149A1_LC | . | . | . | . | . | . | . | . | . | . | . | . | . | . | . | . | . | . | . | . | . | . | . | . | . | . |
| 21-225_150F9_LC | . | . | . | . | . | . | . | . | . | . | . | . | . | . | . | . | . | . | . | . | . | . | . | . | . | . |
| 21-225_154G12_LC | . | . | . | . | . | . | . | . | . | . | . | . | . | . | . | . | . | . | . | . | . | . | . | . | . | . |
| 21-225_156H2_LC | . | . | . | . | . | . | . | . | . | . | . | . | . | . | . | . | . | . | . | . | . | . | . | . | . | . |
| 21-225_197F9_LC | . | . | . | . | . | . | . | . | . | . | . | . | . | . | . | . | . | . | . | . | . | . | . | . | . | . |
| 21-225_199C3_LC | . | . | . | . | . | . | . | . | . | . | . | . | . | . | . | . | . | . | . | . | . | . | . | . | . | . |
| 21-225_4G12_LC | . | . | . | . | . | . | . | . | . | . | . | . | . | . | . | . | . | . | . | . | . | . | . | . | . | . |
| 21-225_60D6_LC | . | . | . | . | . | . | . | - | . | . | . | . | . | . | . | . | . | . | . | . | . | . | . | . | . | . |
| 21-225_9G9_LC | . | . | . | . | . | . | . | . | . | . | . | . | . | . | . | . | . | . | . | . | . | . | . | . | . | . |

FIGURE 57

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | L_FR4 | | | | | | | | | | |
| CONSENSUS | - | - | - | L | N | G | V | V | F | G | G | G | T | K | L | T | V | L | G |
| 21-225_146A8_LC | . | . | . | . | . | . | . | . | . | . | . | . | . | . | . | . | . | . | . |
| 21-225_146B6_LC | . | . | . | . | . | . | . | . | . | . | . | . | . | . | . | . | . | . | S |
| 21-225_146D8_LC | . | . | . | . | . | . | . | . | . | . | . | . | . | . | . | . | . | . | . |
| 21-225_147H12_LC | . | . | . | . | . | . | P | . | . | . | . | . | . | . | . | . | . | . | . |
| 21-225_149A1_LC | . | . | . | . | . | . | . | . | . | . | . | . | . | . | . | . | . | . | S |
| 21-225_150F9_LC | . | . | . | . | . | . | . | . | . | . | . | . | . | . | . | . | . | . | S |
| 21-225_154G12_LC | . | . | . | . | K | . | P | . | . | . | . | . | . | . | . | . | . | . | . |
| 21-225_156H2_LC | . | . | . | . | . | . | . | . | . | . | . | . | . | . | V | . | . | . | . |
| 21-225_197F9_LC | . | . | . | M | . | . | P | . | . | . | . | . | . | . | . | . | . | . | . |
| 21-225_199C3_LC | . | . | . | . | . | . | P | . | . | . | . | . | . | . | . | . | . | . | . |
| 21-225_4G12_LC | . | . | L | N | G | H | . | . | . | . | . | R | . | . | . | . | . | . | . |
| 21-225_60D6_LC | . | . | . | S | L | N | G | P | V | . | . | . | . | . | . | . | . | . | . |
| 21-225_9G9_LC | . | . | L | N | G | H | . | . | . | . | . | R | . | . | . | . | . | . | . |

**FIGURE 57**

Table 121. Consensus 41 - VL1|1c/JL3b (SEQ ID NO: 50338):

QSVLTQP-PSASGTPGQRVTISCSGSS-SNIGS-----NIVTWYQQLPGTAPKLLIYS--------NDQRPSGVPDRFSGSKSG--TSASLAISGLQSEDEADYYCAAWDDS---------------------LNGWVFGGGTTLTVLG wherein:

S at position 27 can be substituted with N or C

S at position 33 can be substituted with N

N at position 39 can be substituted with H

I at position 40 can be substituted with T

T at position 42 can be substituted with N

S at position 58 can be substituted with G, N or V

D at position 68 can be substituted with K, N or Y

A at position 107 can be substituted with T

A at position 108 can be substituted with T or V

N at position 135 can be substituted with I or S

G at position 136 can be substituted with D or V

Ser Gly Ser Xaa Ser Asn Ile Gly Xaa Xaa Xaa Val Xaa (SEQ ID NO: 50195)

Xaa Asn Xaa Gln Arg Pro Ser (SEQ ID NO: 50196)

Xaa Xaa Trp Asp Asp Ser Leu Xaa Xaa Trp Val (SEQ ID NO: 50197)

Ser Gly Ser Ser Ser Asn Ile Gly Ser Asn Ile Val Thr (SEQ ID NO: 50684)

Ser Asn Asp Gln Arg Pro Ser (SEQ ID NO: 50685)

Ala Ala Trp Asp Asp Ser Leu Asn Gly Trp Val (SEQ ID NO: 50686)

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | L_FR1 | | | | | | | | | | | | | | | | | | | | | | | | | |
| CONSENSUS | Q | S | V | L | T | Q | P | - | P | S | A | S | G | T | P | G | Q | R | V | T | I | S | C | S | G | S |
| 21-225_192G3_LC | . | . | . | . | . | . | . | . | . | . | . | . | . | . | . | . | . | . | . | . | M | . | . | . | . | . |
| 21-225_195H6_LC | . | . | . | . | . | . | . | . | . | . | . | . | . | . | . | . | . | . | . | . | . | . | . | . | . | . |
| 21-225_49C5_LC | . | . | . | . | . | . | . | . | . | . | . | . | . | . | . | . | . | . | . | . | . | . | . | . | . | . |
| 21-225_49D10_LC | . | . | . | . | . | . | . | . | . | . | . | . | . | . | . | . | . | . | . | . | . | . | . | . | . | . |
| 21-225_51E3_LC | . | . | . | . | . | . | . | . | . | . | . | . | . | . | . | . | . | . | . | . | . | . | . | . | . | . |
| 21-225_51F4_LC | . | . | . | . | . | . | . | . | . | . | . | . | . | . | . | . | . | . | . | . | . | . | . | . | . | . |
| 21-225_52F1_LC | . | . | . | . | . | . | . | . | . | . | . | . | . | . | . | . | . | . | . | . | . | . | . | . | . | . |
| 21-225_53E8_LC | . | . | . | . | . | . | . | . | . | . | . | . | . | . | . | . | . | . | . | . | . | . | . | . | . | . |
| 21-225_54E9_LC | . | . | . | . | . | . | . | . | . | . | . | . | . | . | . | . | . | . | . | . | . | . | . | . | . | . |
| 21-225_56E3_LC | . | . | . | . | . | . | . | . | . | . | . | . | . | . | . | . | . | . | . | . | . | . | . | . | . | . |
| 21-225_56G1_LC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | L_CDR1 | | | | | | | | | | | | | | | | L_FR2 | | | | | | | | | |
| CONSENSUS | S | - | S | N | I | G | S | - | - | - | - | - | N | I | V | T | W | Y | Q | Q | L | P | G | T | A | P |
| 21-225_192G3_LC | . | . | . | . | . | . | . | . | . | . | . | . | . | T | . | N | . | . | . | . | F | . | . | . | . | . |
| 21-225_195H6_LC | . | . | . | . | . | . | . | . | . | . | . | . | H | T | . | N | . | . | . | . | . | . | . | . | . | . |
| 21-225_49C5_LC | . | . | . | . | . | . | . | . | . | . | . | . | . | . | . | . | . | . | . | . | . | . | . | . | . | . |
| 21-225_49D10_LC | N | . | . | . | . | . | . | . | . | . | . | . | . | . | . | . | . | . | . | . | . | . | . | . | . | . |
| 21-225_51E3_LC | N | . | . | . | . | . | . | . | . | . | . | . | . | . | . | . | . | . | . | . | . | . | . | . | . | . |
| 21-225_51F4_LC | . | . | . | . | . | . | . | . | . | . | . | . | . | . | . | . | . | . | . | . | . | . | . | . | . | . |
| 21-225_52F1_LC | . | . | . | . | . | . | . | . | . | . | . | . | . | . | . | . | . | . | . | . | . | . | . | . | . | . |
| 21-225_53E8_LC | C | . | . | . | . | . | . | . | . | . | . | . | . | . | . | . | . | . | . | . | . | . | . | . | . | . |
| 21-225_54E9_LC | N | . | . | . | . | . | N | . | . | . | . | . | . | . | . | . | . | . | . | . | . | . | . | . | . | . |
| 21-225_56E3_LC | . | . | . | . | . | . | . | . | . | . | . | . | . | . | . | . | . | . | . | . | . | . | . | . | . | . |
| 21-225_56G1_LC | . | . | . | . | . | . | . | . | . | . | . | . | H | . | . | . | . | . | . | . | . | . | . | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | L_CDR2 | | | | | | | | | | | | | | | | | | | | |
| CONSENSUS | K | L | L | I | Y | S | - | - | - | - | - | - | - | - | N | D | Q | R | P | S | G | V | P | D | R | F |
| 21-225_192G3_LC | . | . | . | . | . | G | . | . | . | . | . | . | . | . | . | K | . | . | . | . | R | . | . | . | . | . |
| 21-225_195H6_LC | . | . | . | . | . | N | . | . | . | . | . | . | . | . | . | Y | . | . | . | . | . | . | . | . | . | . |
| 21-225_49C5_LC | . | . | . | . | . | . | . | . | . | . | . | . | . | . | . | . | . | . | . | . | . | . | . | . | . | . |
| 21-225_49D10_LC | . | . | . | . | . | . | . | . | . | . | . | . | . | . | . | . | . | . | . | . | . | . | . | . | . | . |
| 21-225_51E3_LC | . | . | . | . | . | . | . | . | . | . | . | . | . | . | . | . | . | . | . | . | . | . | . | . | . | . |
| 21-225_51F4_LC | . | . | . | . | . | . | . | . | . | . | . | . | . | . | . | . | . | . | . | . | . | . | . | . | . | . |
| 21-225_52F1_LC | . | . | . | . | . | . | . | . | . | . | . | . | . | . | . | . | . | . | . | . | . | . | . | . | . | . |
| 21-225_53E8_LC | . | . | . | . | . | . | . | . | . | . | . | . | . | . | . | . | . | . | . | . | . | . | . | . | . | . |
| 21-225_54E9_LC | . | . | . | . | . | V | . | . | . | . | . | . | . | . | . | . | . | . | . | . | . | . | . | . | . | . |
| 21-225_56E3_LC | . | . | . | . | . | . | . | . | . | . | . | . | . | . | . | N | . | . | . | . | . | . | . | . | . | . |
| 21-225_56G1_LC | . | . | . | . | . | . | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | L_FR3 | | | | | | | | | | | | | | | | | | | | | | | | | |
| CONSENSUS | S | G | S | K | S | G | - | - | T | S | A | S | L | A | I | S | G | L | Q | S | E | D | E | A | D | Y |
| 21-225_192G3_LC | . | . | . | . | . | . | . | . | . | . | . | . | . | . | . | . | . | . | . | . | . | . | . | T | . | . |
| 21-225_195H6_LC | . | . | . | . | . | . | . | . | . | . | . | . | . | T | . | . | . | . | . | . | . | . | . | . | . | . |
| 21-225_49C5_LC | . | . | . | . | . | . | . | . | . | . | . | . | . | . | . | . | . | . | . | . | . | . | . | . | . | . |
| 21-225_49D10_LC | . | . | . | . | . | . | . | . | . | . | . | . | . | . | . | . | . | . | . | . | . | . | . | . | . | . |
| 21-225_51E3_LC | . | . | . | . | . | . | . | . | . | . | . | . | . | . | . | . | . | . | . | . | . | . | . | . | . | . |
| 21-225_51F4_LC | . | . | . | . | . | . | . | . | . | . | . | . | . | . | . | . | . | . | . | . | . | . | . | T | . | . |
| 21-225_52F1_LC | . | . | . | . | . | . | . | . | . | . | . | . | . | . | . | . | . | . | . | . | . | . | . | . | . | . |
| 21-225_53E8_LC | . | . | . | . | . | . | . | . | . | . | . | . | . | . | . | . | . | . | . | . | . | . | . | . | . | . |
| 21-225_54E9_LC | . | . | . | . | . | . | . | . | . | . | . | . | . | . | . | . | . | . | . | . | . | . | . | . | . | . |
| 21-225_56E3_LC | . | . | . | . | . | . | . | . | . | . | . | . | . | . | . | . | . | . | . | . | . | . | . | . | . | . |
| 21-225_56G1_LC | . | . | . | . | . | . | . | . | . | . | . | . | . | . | . | . | . | . | . | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | L_CDR3 | | | | | | | | |
| CONSENSUS | Y | C | A | A | W | D | D | S | - | - | - | - | - | - | - | - | - | - | - | - | - | - | - | - | - | - |
| 21-225_192G3_LC | . | . | . | . | . | . | . | . | . | . | . | . | . | . | . | . | . | . | . | . | . | . | . | . | . | . |
| 21-225_195H6_LC | . | . | . | T | . | . | . | . | . | . | . | . | . | . | . | . | . | . | . | . | . | . | . | . | . | . |
| 21-225_49C5_LC | . | . | T | . | . | . | . | . | . | . | . | . | . | . | . | . | . | . | . | . | . | . | . | . | . | . |
| 21-225_49D10_LC | . | . | . | . | . | . | . | . | . | . | . | . | . | . | . | . | . | . | . | . | . | . | . | . | . | . |
| 21-225_51E3_LC | . | . | T | . | . | . | . | . | . | . | . | . | . | . | . | . | . | . | . | . | . | . | . | . | . | . |
| 21-225_51F4_LC | . | . | T | . | . | . | . | . | . | . | . | . | . | . | . | . | . | . | . | . | . | . | . | . | . | . |
| 21-225_52F1_LC | . | . | . | . | . | . | . | . | . | . | . | . | . | . | . | . | . | . | . | . | . | . | . | . | . | . |
| 21-225_53E8_LC | . | . | T | . | . | . | . | . | . | . | . | . | . | . | . | . | . | . | . | . | . | . | . | . | . | . |
| 21-225_54E9_LC | . | . | . | . | . | . | . | . | . | . | . | . | . | . | . | . | . | . | . | . | . | . | . | . | . | . |
| 21-225_56E3_LC | C | . | . | . | . | . | . | . | . | . | . | . | . | . | . | . | . | . | . | . | . | . | . | . | . | . |
| 21-225_56G1_LC | . | . | . | V | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | L_FR4 | | | | | |
| CONSENSUS | - | - | - | L | N | G | W | V | F | G | G | G | T | T | L | T | V | L | G |
| 21-225_192G3_LC | . | . | . | . | . | . | . | . | . | . | . | . | . | K | . | . | . | . | . |
| 21-225_195H6_LC | . | . | . | . | S | V | . | . | . | . | . | . | . | K | V | . | . | . | . |
| 21-225_49C5_LC | . | . | . | . | . | . | . | . | . | . | . | . | . | . | . | . | . | . | . |
| 21-225_49D10_LC | . | . | . | . | . | . | . | . | . | . | . | . | . | . | . | . | . | . | . |
| 21-225_51E3_LC | . | . | . | . | . | . | . | . | . | . | . | . | . | . | . | . | . | . | . |
| 21-225_51F4_LC | . | . | . | . | . | . | . | . | . | . | . | . | . | . | . | . | . | . | . |
| 21-225_52F1_LC | . | . | . | . | . | . | . | . | . | . | . | . | . | . | . | . | . | . | . |
| 21-225_53E8_LC | . | . | . | . | . | D | . | . | . | . | . | . | . | . | . | . | . | . | . |
| 21-225_54E9_LC | . | . | . | . | . | . | . | . | . | . | . | . | . | . | . | . | . | . | . |
| 21-225_56E3_LC | . | . | . | . | . | . | . | . | . | . | . | . | . | . | . | . | . | . | . |
| 21-225_56G1_LC | . | . | . | . | I | . | . | . | . | . | . | . | . | . | . | . | . | . | . |

**FIGURE 57**

Table 122. Consensus 42 - VK1|A30/JK5 (SEQ ID NO: 50339):

DIQMTQSPSSLSASVGDRVTITCRAS--QGIR------NDLGWYQQKPGKAPKRLIYA--------ASSLQSGVPSRFSGSGSG--TEFTLTISSLQPEDFATYYCLQHNS-----------------------YPITFGQGTRLEIKR wherein:

A at position 25 can be substituted with T

G at position 30 can be substituted with D or R

I at position 31 can be substituted with V

N at position 39 can be substituted with S

A at position 58 can be substituted with D, I or T

S at position 69 can be substituted with N

Q at position 71 can be substituted with E, F or L

L at position 107 can be substituted with I

Q at position 108 can be substituted with H

H at position 109 can be substituted with Y

N a position 110 can be substituted with H or S

S at position 111 can be substituted with N

null (-) at position 134 can be substituted with Y

Y at position 135 can be substituted with L, F or P

I at position 137 can be substituted with P or L

Arg Xaa Ser Gln Xaa Xaa Arg Xaa Asp Leu Gly (SEQ ID NO: 50198)

**FIGURE 57**

Xaa Ala Ser Xaa Leu Xaa Ser (SEQ ID NO: 50199)

Xaa Xaa Xaa Xaa Xaa Xaa Xaa Pro Xaa Thr (SEQ ID NO: 50200)

Arg Ala Ser Gln Gly Ile Arg Asn Asp Leu Gly (SEQ ID NO: 50687)

Ala Ala Ser Ser Leu Gln Ser (SEQ ID NO: 50688)

Leu Gln His Asn Ser Tyr Pro Ile Thr (SEQ ID NO: 50689)

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_159F1_LC | . | . | . | . | . | . | . | . | . | . | . | . | . | . | . | . | . | . | . | . | . | . | . | . | . | . |
| 21-225_18D4_LC | . | . | . | . | . | . | . | . | . | . | . | . | . | . | . | . | . | . | . | . | . | . | . | . | . | . |
| 21-225_207F12_LC | . | . | . | . | . | . | . | . | . | . | . | . | . | . | . | . | . | . | . | . | . | . | . | . | . | . |
| 21-225_208A2_LC | . | . | . | . | . | . | . | . | . | . | . | . | . | . | . | . | . | . | . | . | . | . | . | . | T | . |
| 21-225_210G10_LC | . | . | . | . | . | . | . | . | . | . | . | . | . | . | . | . | . | . | . | . | . | . | F | . | T | . |
| 21-225_34C4_LC | . | . | . | . | . | . | . | . | . | . | . | . | . | . | . | . | . | . | . | . | . | . | . | . | . | . |
| 21-225_73H8_LC | . | . | . | . | . | . | . | . | . | . | . | Y | . | . | . | . | . | . | . | . | . | . | R | . | . | . |
| 21-225_7C3_LC1_LC | . | . | . | . | . | . | . | . | . | . | . | . | . | . | . | . | . | . | . | . | . | . | . | . | . | . |
| 21-225_90H5_LC | . | . | . | . | . | . | . | . | . | . | . | . | . | . | . | . | . | . | . | . | . | . | . | . | . | . |
| 21-225_9F12_LC2_LC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | | K_FR2 | | |
| CONSENSUS | - | - | Q | G | I | R | - | - | - | - | - | - | N | D | L | G | W | Y | Q | Q | K | P | G | K | A | P |
| 21-225_159F1_LC | . | . | . | R | V | . | . | . | . | . | . | . | . | . | . | . | . | . | . | . | . | . | A | . | . | . |
| 21-225_18D4_LC | . | . | . | . | . | . | . | . | . | . | . | . | . | . | . | . | . | . | . | . | . | . | . | . | . | . |
| 21-225_207F12_LC | . | . | . | . | . | . | . | . | . | . | . | . | . | . | . | . | . | . | . | . | . | . | . | . | . | . |
| 21-225_208A2_LC | . | . | . | . | . | . | . | . | . | . | . | . | . | . | . | . | . | . | . | . | Q | . | . | . | T | . |
| 21-225_210G10_LC | . | . | . | . | . | . | . | . | . | . | . | . | . | . | . | . | . | . | . | . | Q | . | . | . | T | . |
| 21-225_34C4_LC | . | . | . | . | . | . | . | . | . | . | . | . | . | . | . | . | . | . | . | . | . | . | . | . | . | . |
| 21-225_73H8_LC | . | . | . | D | . | . | . | . | . | . | . | . | . | . | . | . | . | . | . | . | . | . | . | . | . | L |
| 21-225_7C3_LC1_LC | . | . | . | . | . | . | . | . | . | . | . | . | S | . | . | . | . | . | . | . | N | . | V | . | . | . |
| 21-225_90H5_LC | . | . | . | . | . | . | . | . | . | . | . | . | . | . | . | . | . | . | . | . | . | . | . | . | . | . |
| 21-225_9F12_LC2_LC | . | . | . | . | . | . | . | . | . | . | . | . | . | . | . | . | . | . | . | . | . | . | V | . | . | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | K_CDR2 | | | | | | | | | | | | | | |
| CONSENSUS | K | R | L | I | Y | A | - | - | - | - | - | - | - | - | A | S | S | L | Q | S | G | V | P | S | R | F |
| 21-225_159F1_LC | . | . | I | . | F | D | . | . | . | . | . | . | . | . | . | . | N | . | . | . | . | . | . | . | . | . |
| 21-225_18D4_LC | . | . | . | . | . | . | . | . | . | . | . | . | . | . | . | . | . | . | E | . | . | . | . | . | . | . |
| 21-225_207F12_LC | . | . | . | . | . | . | . | . | . | . | . | . | . | . | . | . | . | . | . | . | . | . | . | . | . | . |
| 21-225_208A2_LC | . | . | . | . | . | . | . | . | . | . | . | . | . | . | . | . | . | . | L | . | . | . | . | . | . | . |
| 21-225_210G10_LC | . | . | M | . | . | . | . | . | . | . | . | . | . | . | . | . | . | . | F | . | . | . | . | . | . | . |
| 21-225_34C4_LC | . | . | . | . | . | T | . | . | . | . | . | . | . | . | . | . | N | . | . | . | . | . | . | . | . | . |
| 21-225_73H8_LC | . | . | V | . | . | . | . | . | . | . | . | . | . | . | . | . | . | . | . | . | . | . | . | . | S | . |
| 21-225_7C3_LC1_LC | . | . | . | . | . | . | . | . | . | . | . | . | . | . | . | . | . | . | . | . | . | . | . | . | . | . |
| 21-225_90H5_LC | . | . | . | . | . | I | . | . | . | . | . | . | . | . | . | . | . | . | . | . | . | . | . | . | . | . |
| 21-225_9F12_LC2_LC | . | . | . | . | . | . | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | E | F | T | L | T | I | S | S | L | Q | P | E | D | F | A | T | Y |
| 21-225_159F1_LC | . | . | . | . | . | . | . | . | . | . | . | . | . | . | . | . | . | . | . | . | . | . | . | . | . | . |
| 21-225_18D4_LC | . | . | . | . | . | . | . | . | . | . | . | . | . | . | V | . | . | . | . | . | . | . | . | . | . | . |
| 21-225_207F12_LC | . | . | . | . | . | . | . | . | . | . | . | . | . | . | . | . | . | . | . | . | . | . | . | . | . | . |
| 21-225_208A2_LC | . | . | . | R | . | . | . | . | . | D | . | . | . | . | . | . | . | . | . | . | . | . | . | . | . | . |
| 21-225_210G10_LC | . | . | . | R | . | . | . | . | . | D | . | . | . | . | . | . | . | . | . | . | . | . | . | . | . | . |
| 21-225_34C4_LC | . | . | . | . | . | . | . | . | . | . | . | . | . | . | . | . | . | . | . | . | . | . | . | . | . | . |
| 21-225_73H8_LC | . | . | . | . | . | . | . | . | . | . | . | . | . | . | . | . | . | . | . | . | . | . | . | . | . | . |
| 21-225_7C3_LC1_LC | . | . | . | . | . | . | . | . | . | . | . | . | . | . | V | . | . | . | . | . | . | . | . | . | F | . |
| 21-225_90H5_LC | . | . | . | . | . | . | . | . | . | . | . | . | . | . | . | . | . | . | . | . | . | . | . | . | . | . |
| 21-225_9F12_LC2_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | L | Q | H | N | S | - | - | - | - | - | - | - | - | - | - | - | - | - | - | - | - | - | - | - |
| 21-225_159F1_LC | . | . | . | . | . | H | . | . | . | . | . | . | . | . | . | . | . | . | . | . | . | . | . | . | . | . |
| 21-225_18D4_LC | . | . | . | H | . | . | N | . | . | . | . | . | . | . | . | . | . | . | . | . | . | . | . | . | . | . |
| 21-225_207F12_LC | F | . | . | . | . | . | . | . | . | . | . | . | . | . | . | . | . | . | . | . | . | . | . | . | . | . |
| 21-225_208A2_LC | . | . | . | . | Y | . | . | . | . | . | . | . | . | . | . | . | . | . | . | . | . | . | . | . | . | . |
| 21-225_210G10_LC | . | . | . | . | Y | . | . | . | . | . | . | . | . | . | . | . | . | . | . | . | . | . | . | . | . | . |
| 21-225_34C4_LC | . | . | . | . | . | S | . | . | . | . | . | . | . | . | . | . | . | . | . | . | . | . | . | . | . | . |
| 21-225_73H8_LC | . | G | I | . | . | . | . | . | . | . | . | . | . | . | . | . | . | . | . | . | . | . | . | . | . | . |
| 21-225_7C3_LC1_LC | . | . | . | . | . | . | . | . | . | . | . | . | . | . | . | . | . | . | . | . | . | . | . | . | . | . |
| 21-225_90H5_LC | . | . | . | . | . | . | . | . | . | . | . | . | . | . | . | . | . | . | . | . | . | . | . | . | . | . |
| 21-225_9F12_LC2_LC | . | . | . | . | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | I | T | F | G | Q | G | T | R | L | E | I | K | R |
| 21-225_159F1_LC | . | . | . | . | F | . | . | . | . | . | . | . | . | . | . | . | . | . | . |
| 21-225_18D4_LC | . | . | . | Y | L | . | . | . | . | . | . | . | . | . | . | . | . | . | . |
| 21-225_207F12_LC | . | . | . | . | . | . | L | . | . | . | . | . | . | . | . | . | . | . | . |
| 21-225_208A2_LC | . | . | . | . | . | . | F | . | . | . | . | . | . | . | . | . | . | . | . |
| 21-225_210G10_LC | . | . | . | . | . | . | F | . | . | . | . | . | . | . | . | . | . | . | . |
| 21-225_34C4_LC | . | . | . | . | . | . | . | . | . | . | . | . | . | . | . | . | . | . | . |
| 21-225_73H8_LC | . | . | . | Y | F | . | . | . | V | . | . | . | . | . | . | . | . | . | . |
| 21-225_7C3_LC1_LC | . | . | . | Y | L | . | . | . | . | . | . | . | . | . | . | . | . | . | . |
| 21-225_90H5_LC | . | . | . | . | . | . | . | . | . | . | . | . | . | . | . | . | . | . | . |
| 21-225_9F12_LC2_LC | . | . | . | Y | L | . | . | . | . | . | . | . | . | . | . | . | . | . | . |

Table 123. Consensus 43 - VK1|O12/JK5 (SEQ ID NO: 50340):

**FIGURE 57**

DIQMTQSPSSLSASVGDRVTITCRAS--QSIS------SYLNWYQQKPGKAPKLLIYA--------ASSLQSGVPSRFSGSGSG--TDFTLTISSLQPEDFATYYCQQSYS----------------------IPITFGQGTRLEIKR wherein:

A at position 25 can be substituted with T

S at position 30 can be substituted with N or Y

I at position 31 can be substituted with S or F

S at position 32 can be substituted with F, N, R or T

S at position 39 can be substituted with D, G or R

L at position 41 can be substituted with S

N at position 42 can be substituted with S

A at position 58 can be substituted with D, G or S

A at position 67 can be substituted with T

S at position 68 can be substituted with Y

S at position 69 can be substituted with T

L at position 70 can be substituted with F

Q at position 71 can be substituted with E or K

S at position 72 can be substituted with T

Q at position 107 can be substituted with H

Q at position 108 can be substituted with E

S at position 109 can be substituted with T

Y at position 110 can be substituted with F

**FIGURE 57**

S at position 111 can be substituted with G or N

I at position 135 can be substituted with T, L, N or S

P at position 136 can be substituted with S, R or T

I at position 137 can be substituted with F or P

T at position 138 can be substituted with A

Arg Xaa Ser Gln Xaa Xaa Xaa Xaa Tyr Xaa Xaa (SEQ ID NO: 50201)

Xaa Xaa Xaa Xaa Xaa Xaa Xaa (SEQ ID NO: 50202)

Xaa Xaa Xaa Xaa Xaa Xaa Xaa Xaa Xaa (SEQ ID NO: 50203)

Arg Ala Ser Gln Ser Ile Ser Ser Tyr Leu Asn (SEQ ID NO: 50690)

Ala Ala Ser Ser Leu Gln Ser (SEQ ID NO: 50691)

Gln Gln Ser Tyr Ser Ile Pro Ile Thr (SEQ ID NO: 50692)

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_157E7_LC | . | . | . | . | . | . | . | . | . | . | . | . | . | . | . | . | . | . | . | . | . | . | . | . | . | . |
| 21-225_21D3_LC | . | . | . | . | . | . | . | . | . | . | . | . | . | . | . | . | . | . | . | . | . | . | . | . | . | . |
| 21-225_22D8_LC | . | . | . | . | . | . | . | . | . | . | . | . | . | . | . | . | . | . | . | . | . | . | . | . | T | . |
| 21-225_33A9_LC | . | . | . | . | . | . | . | . | . | . | . | . | . | . | . | . | . | . | . | . | . | . | . | . | . | . |
| 21-225_33D12_LC | . | V | . | . | . | . | . | . | . | . | . | . | . | . | L | . | . | . | . | . | . | . | . | . | . | . |
| 21-225_35E5_LC | . | . | . | . | I | . | . | . | . | . | . | . | . | . | . | . | . | . | . | . | . | . | Y | . | . | . |
| 21-225_53C6_LC | . | . | . | . | . | . | . | . | . | . | . | . | . | . | . | . | . | . | . | . | . | . | . | . | . | . |
| 21-225_59E8_LC | . | . | . | . | . | . | . | . | . | . | . | . | . | . | . | . | . | . | . | . | . | . | . | . | . | . |
| 21-225_63G6_LC | . | . | . | . | . | . | . | . | . | . | . | . | . | . | . | . | . | . | . | I | . | S | . | . | . | . |
| 21-225_72F5_LC | . | . | . | . | . | . | . | . | . | . | . | . | . | . | . | . | . | . | . | I | . | P | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | I | S | - | - | - | - | - | - | S | Y | L | N | W | Y | Q | Q | K | P | G | K | A | P |
| 21-225_157E7_LC | . | . | . | . | F | . | . | . | . | . | . | . | . | . | . | . | . | . | . | . | . | . | . | I | . | . |
| 21-225_21D3_LC | . | . | . | . | . | T | . | . | . | . | . | . | G | . | S | . | . | . | . | . | . | . | . | . | T | . |
| 21-225_22D8_LC | . | . | . | N | S | N | . | . | . | . | . | . | . | . | . | . | . | . | . | . | . | . | . | . | . | . |
| 21-225_33A9_LC | . | . | . | . | . | . | . | . | . | . | . | . | R | . | . | S | . | . | . | . | . | . | . | R | . | . |
| 21-225_33D12_LC | . | . | . | . | . | R | . | . | . | . | . | . | . | . | . | . | . | . | . | . | . | . | . | . | . | . |
| 21-225_35E5_LC | . | . | . | . | . | . | . | . | . | . | . | . | . | . | . | . | . | . | . | . | . | . | A | . | . | . |
| 21-225_53C6_LC | . | . | . | Y | S | . | . | . | . | . | . | . | D | . | . | . | . | . | . | . | . | . | . | . | . | . |
| 21-225_59E8_LC | . | . | . | . | . | . | . | . | . | . | . | . | . | . | . | . | . | . | . | . | . | . | . | . | . | . |
| 21-225_63G6_LC | . | . | . | . | . | F | . | . | . | . | . | . | . | . | . | . | . | . | . | V | . | . | . | . | . | . |
| 21-225_72F5_LC | . | . | . | N | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | Y | A | - | - | - | - | - | - | - | - | A | S | S | L | Q | S | G | V | P | S | R | F |
| 21-225_157E7_LC | . | . | . | . | . | . | . | . | . | . | . | . | . | . | . | . | . | . | . | . | . | . | . | . | . | . |
| 21-225_21D3_LC | . | . | . | . | F | . | . | . | . | . | . | . | . | . | T | Y | . | . | E | . | . | . | . | . | . | . |
| 21-225_22D8_LC | . | . | Q | . | F | . | . | . | . | . | . | . | . | . | . | Y | . | . | E | . | . | . | . | . | . | . |
| 21-225_33A9_LC | N | . | Q | . | . | . | . | . | . | . | . | . | . | . | . | . | . | . | . | . | . | . | . | . | . | . |
| 21-225_33D12_LC | N | . | . | . | . | S | . | . | . | . | . | . | . | . | . | . | . | . | . | . | . | . | . | . | . | . |
| 21-225_35E5_LC | . | . | H | . | . | G | . | . | . | . | . | . | . | . | . | . | . | F | . | . | W | . | . | . | . | . |
| 21-225_53C6_LC | . | V | . | . | F | . | . | . | . | . | . | . | . | . | . | . | . | . | K | . | . | . | . | . | . | . |
| 21-225_59E8_LC | . | . | . | . | . | . | . | . | . | . | . | . | . | . | . | . | . | . | . | . | . | . | . | . | . | . |
| 21-225_63G6_LC | . | . | . | . | . | D | . | . | . | . | . | . | . | . | T | . | T | . | . | T | . | . | . | . | . | . |
| 21-225_72F5_LC | . | . | . | . | . | . | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_157E7_LC | . | . | . | . | . | . | . | . | . | . | . | . | . | . | . | . | . | . | . | . | . | . | . | . | . | . |
| 21-225_21D3_LC | . | . | . | . | F | . | . | . | . | N | . | . | . | . | . | T | . | . | . | . | . | . | . | . | . | . |
| 21-225_22D8_LC | . | . | N | R | . | . | . | . | . | E | . | . | . | . | . | . | . | . | . | . | . | . | . | . | . | . |
| 21-225_33A9_LC | . | . | . | . | . | . | . | . | . | . | . | . | . | . | . | . | . | . | . | . | . | . | . | . | . | . |
| 21-225_33D12_LC | . | . | . | . | . | . | . | . | . | . | . | . | . | . | . | . | . | . | . | . | . | . | . | . | N | . |
| 21-225_35E5_LC | . | . | . | . | . | S | . | . | . | . | . | . | . | . | . | I | . | M | . | . | G | . | Y | . | . | . |
| 21-225_53C6_LC | . | . | . | . | . | . | . | . | . | . | . | . | . | . | . | . | . | . | E | . | . | . | . | . | . | . |
| 21-225_59E8_LC | . | . | . | . | . | . | . | . | . | . | . | . | . | . | . | . | . | . | . | . | . | . | . | . | . | . |
| 21-225_63G6_LC | . | . | . | . | . | . | . | . | . | . | . | . | . | . | . | N | . | . | . | . | . | . | . | . | . | . |
| 21-225_72F5_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | S | Y | S | - | - | - | - | - | - | - | - | - | - | - | - | - | - | - | - | - | - | - |
| 21-225_157E7_LC | . | . | . | E | . | . | . | . | . | . | . | . | . | . | . | . | . | . | . | . | . | . | . | . | . | . |
| 21-225_21D3_LC | . | . | . | . | . | . | . | . | . | . | . | . | . | . | . | . | . | . | . | . | . | . | . | . | . | . |
| 21-225_22D8_LC | . | . | . | . | T | . | G | . | . | . | . | . | . | . | . | . | . | . | . | . | . | . | . | . | . | . |
| 21-225_33A9_LC | . | . | . | . | . | . | N | . | . | . | . | . | . | . | . | . | . | . | . | . | . | . | . | . | . | . |
| 21-225_33D12_LC | . | . | . | . | . | . | . | . | . | . | . | . | . | . | . | . | . | . | . | . | . | . | . | . | . | . |
| 21-225_35E5_LC | . | . | H | . | . | . | N | . | . | . | . | . | . | . | . | . | . | . | . | . | . | . | . | . | . | . |
| 21-225_53C6_LC | F | . | . | . | . | . | . | . | . | . | . | . | . | . | . | . | . | . | . | . | . | . | . | . | . | . |
| 21-225_59E8_LC | F | . | . | . | . | F | . | . | . | . | . | . | . | . | . | . | . | . | . | . | . | . | . | . | . | . |
| 21-225_63G6_LC | . | . | . | . | . | . | G | . | . | . | . | . | . | . | . | . | . | . | . | . | . | . | . | . | . | . |
| 21-225_72F5_LC | S | . | . | . | T | . | - | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | I | P | I | T | F | G | Q | G | T | R | L | E | I | K | R |
| 21-225_157E7_LC | . | . | . | . | . | R | F | A | . | . | . | . | . | . | . | . | . | . | . |
| 21-225_21D3_LC | . | . | . | . | T | S | . | . | . | . | . | . | . | . | . | . | . | . | . |
| 21-225_22D8_LC | . | . | . | . | T | S | . | . | . | . | . | . | . | . | . | . | . | . | . |
| 21-225_33A9_LC | . | . | . | . | . | . | . | . | . | . | . | . | . | . | . | . | . | . | . |
| 21-225_33D12_LC | . | . | . | . | . | . | . | . | . | . | . | . | . | . | . | . | . | . | . |
| 21-225_35E5_LC | . | . | . | . | L | . | . | . | . | . | . | . | . | . | . | . | . | . | . |
| 21-225_53C6_LC | . | . | . | . | T | . | F | . | . | . | . | . | . | . | . | . | . | . | . |
| 21-225_59E8_LC | . | . | . | . | N | S | . | . | . | . | . | . | . | . | . | . | . | . | . |
| 21-225_63G6_LC | . | . | . | . | . | . | . | . | . | . | . | . | . | . | . | . | . | Q | . |
| 21-225_72F5_LC | . | . | . | . | S | T | P | . | . | . | . | . | . | . | . | D | . | N | . |

**FIGURE 57**

Table 124. Consensus 44 - VK2|A18/JK5 (SEQ ID NO: 50341):

DIVMTQTPLSLSVTPGQPASISCKSS--QSLLHSE-GKTYLYWYLQKPGQPPQLLIYE--------VSNRFSGVPDRFSGSGSG--TDFTLKISRVEAEDVGVYYCMQSIQ----------------------LPITFGQGTRLEIKR wherein:

K at position 24 can be substituted with R

S at position 25 can be substituted with T

S at position 26 can be substituted with N

S at position 30 can be substituted with I

L at position 31 can be substituted with F

L at position 32 can be substituted with V

S at position 34 can be substituted with N or R

E at position 35 can be substituted with D

K at position 38 can be substituted with R

V at position 67 can be substituted with L

N at position 69 can be substituted with K or H

F at position 71 can be substituted with L or V

M at position 107 can be substituted with I or L

I at position 110 can be substituted with M

Q at position 111 can be substituted with null (-) or L

L at position 135 can be substituted with Y, I or Q

**FIGURE 57**

P at position 136 can be substituted with L

T at position 138 can be substituted with I

Xaa Xaa Xaa Gln Xaa Xaa Xaa His Xaa Xaa Gly Xaa Thr Tyr Leu Tyr (SEQ ID NO: 50204)

Glu Xaa Ser Xaa Arg Xaa Ser (SEQ ID NO: 50205)

Xaa Gln Ser Xaa Xaa Xaa Xaa Ile Xaa (SEQ ID NO: 50246)

Lys Ser Ser Gln Ser Leu Leu His Ser Glu Gly Lys Thr Tyr Leu Tyr (SEQ ID NO: 50693)

Glu Val Ser Asn Arg Phe Ser (SEQ ID NO: 50694)

Met Gln Ser Ile Gln Leu Pro Ile Thr (SEQ ID NO: 50695)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | V | M | T | Q | T | P | L | S | L | S | V | T | P | G | Q | P | A | S | I | S | C | K | S | S |
| 21-225_147A8_LC | . | . | . | . | . | . | . | . | . | . | . | . | . | . | . | . | . | . | . | . | . | . | . | . | T | . |
| 21-225_32H2_LC | . | . | . | . | . | . | . | . | . | . | . | . | . | . | . | . | . | . | . | . | . | . | . | R | . | . |
| 21-225_49C1_LC | . | . | . | . | . | . | . | . | . | . | . | . | . | . | . | . | . | . | . | . | . | . | . | . | . | . |
| 21-225_49E7_LC | . | . | . | . | . | . | . | . | . | . | . | . | . | . | . | . | . | . | . | F | M | . | . | . | . | . |
| 21-225_49F9_LC | . | . | . | . | . | . | . | . | . | . | . | . | . | . | . | . | . | . | . | . | . | . | . | . | . | N |
| 21-225_49G3_LC | . | . | . | . | . | . | . | . | . | . | . | . | . | . | . | . | . | . | . | . | M | . | . | . | . | . |
| 21-225_52D10_LC | . | V | M | . | . | . | I | . | . | . | . | . | . | . | . | . | . | . | . | . | . | . | . | . | . | . |
| 21-225_52G8_LC | . | . | . | . | . | . | . | . | . | . | . | . | . | . | . | . | . | . | . | . | M | . | . | . | . | . |
| 21-225_55C2_LC | . | V | M | . | . | . | I | . | . | . | . | . | . | . | . | . | . | . | . | . | . | . | . | . | . | . |
| 21-225_55F9_LC | . | . | . | . | . | . | . | . | . | . | . | . | . | . | . | . | . | S | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | L | L | H | S | E | - | G | K | T | Y | L | Y | W | Y | L | Q | K | P | G | Q | P | P |
| 21-225_147A8_LC | . | . | . | . | . | . | . | . | . | . | . | . | . | . | . | . | . | . | . | . | . | . | . | . | . | . |
| 21-225_32H2_LC | . | . | . | . | . | . | . | . | D | . | . | R | . | . | . | . | . | . | . | . | . | . | . | . | . | . |
| 21-225_49C1_LC | . | . | . | . | . | . | . | R | . | . | . | . | . | . | . | . | . | . | . | . | . | . | . | . | A | . |
| 21-225_49E7_LC | . | . | . | I | F | . | . | . | . | . | . | . | . | . | . | . | . | . | . | . | . | T | . | . | . | . |
| 21-225_49F9_LC | . | . | . | . | . | V | . | N | . | . | . | . | . | . | . | . | . | . | . | . | . | . | . | . | . | . |
| 21-225_49G3_LC | . | . | . | . | . | . | . | . | . | . | . | . | . | . | . | . | . | . | . | . | . | T | . | . | . | . |
| 21-225_52D10_LC | . | . | . | . | . | . | . | . | . | . | . | . | . | . | . | . | . | . | . | . | . | . | . | . | . | . |
| 21-225_52G8_LC | . | . | . | . | . | . | . | . | . | . | . | . | . | . | . | . | . | . | . | . | . | T | . | . | . | . |
| 21-225_55C2_LC | . | . | . | . | . | V | . | . | . | . | . | . | . | . | . | . | . | . | . | . | . | . | . | . | . | . |
| 21-225_55F9_LC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | Q | L | L | I | Y | E | - | - | - | - | - | - | - | - | V | S | N | R | F | S | G | V | P | D | R | F |
| 21-225_147A8_LC | . | . | . | . | . | . | . | . | . | . | . | . | . | . | . | . | . | . | . | . | . | . | . | . | . | . |
| 21-225_32H2_LC | . | . | . | . | . | . | . | . | . | . | . | . | . | . | L | . | . | . | . | . | . | . | . | . | . | . |
| 21-225_49C1_LC | . | F | . | . | F | . | . | . | . | . | . | . | . | . | . | . | H | . | . | . | . | . | . | . | . | . |
| 21-225_49E7_LC | H | . | . | . | . | . | . | . | . | . | . | . | . | . | . | . | K | . | L | . | . | . | . | . | . | . |
| 21-225_49F9_LC | . | . | . | . | F | . | . | . | . | . | . | . | . | . | . | . | . | . | . | . | . | . | . | . | . | . |
| 21-225_49G3_LC | H | . | . | . | . | . | . | . | . | . | . | . | . | . | . | . | . | . | L | . | . | . | . | . | . | . |
| 21-225_52D10_LC | . | . | . | . | F | . | . | . | . | . | . | . | . | . | . | . | . | . | V | . | . | . | . | . | . | . |
| 21-225_52G8_LC | H | . | . | . | . | . | . | . | . | . | . | . | . | . | . | . | K | . | L | . | . | . | . | . | . | . |
| 21-225_55C2_LC | . | . | . | . | F | . | . | . | . | . | . | . | . | . | . | . | . | . | V | . | . | . | . | . | . | . |
| 21-225_55F9_LC | . | . | . | . | . | . | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | K | I | S | R | V | E | A | E | D | V | G | V | Y |
| 21-225_147A8_LC | . | . | . | . | . | . | . | . | . | . | . | . | . | . | . | . | . | . | . | . | . | . | . | . | . | . |
| 21-225_32H2_LC | . | . | . | . | . | . | . | . | . | . | . | . | . | . | . | . | . | . | . | . | . | . | . | . | I | . |
| 21-225_49C1_LC | . | . | . | . | . | . | . | . | . | . | . | . | . | . | . | . | . | . | . | . | . | . | . | . | . | . |
| 21-225_49E7_LC | . | . | . | . | . | . | . | . | . | . | . | . | . | . | . | . | . | M | . | . | . | . | . | . | . | . |
| 21-225_49F9_LC | . | . | . | . | . | . | . | . | . | . | . | . | . | . | . | . | . | . | . | . | . | . | . | . | . | . |
| 21-225_49G3_LC | . | . | . | . | . | . | . | . | . | . | . | . | . | . | . | . | . | M | . | . | . | . | . | . | . | . |
| 21-225_52D10_LC | . | . | R | . | . | . | . | . | . | . | . | . | . | . | . | . | . | . | . | . | . | . | . | . | . | . |
| 21-225_52G8_LC | . | . | . | . | . | . | . | . | . | . | . | . | . | . | . | . | . | M | . | . | . | . | . | . | . | . |
| 21-225_55C2_LC | . | . | R | . | . | . | . | . | . | . | . | . | . | . | . | . | . | . | . | . | . | . | . | . | . | . |
| 21-225_55F9_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | M | Q | S | I | Q | - | - | - | - | - | - | - | - | - | - | - | - | - | - | - | - | - | - | - |
| 21-225_147A8_LC | . | . | . | . | . | . | - | . | . | . | . | . | . | . | . | . | . | . | . | . | . | . | . | . | . | . |
| 21-225_32H2_LC | S | . | L | . | . | . | . | . | . | . | . | . | . | . | . | . | . | . | . | . | . | . | . | . | . | . |
| 21-225_49C1_LC | . | . | . | . | . | . | . | . | . | . | . | . | . | . | . | . | . | . | . | . | . | . | . | . | . | . |
| 21-225_49E7_LC | . | . | . | . | . | M | . | . | . | . | . | . | . | . | . | . | . | . | . | . | . | . | . | . | . | . |
| 21-225_49F9_LC | . | . | . | . | . | . | . | . | . | . | . | . | . | . | . | . | . | . | . | . | . | . | . | . | . | . |
| 21-225_49G3_LC | F | . | . | . | . | M | . | . | . | . | . | . | . | . | . | . | . | . | . | . | . | . | . | . | . | . |
| 21-225_52D10_LC | . | . | . | . | . | . | . | . | . | . | . | . | . | . | . | . | . | . | . | . | . | . | . | . | . | . |
| 21-225_52G8_LC | . | . | . | . | . | M | . | . | . | . | . | . | . | . | . | . | . | . | . | . | . | . | . | . | . | . |
| 21-225_55C2_LC | . | . | . | . | . | . | L | . | . | . | . | . | . | . | . | . | . | . | . | . | . | . | . | . | . | . |
| 21-225_55F9_LC | . | . | I | . | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | | P | I | T | F | G | Q | G | T | R | L | E | I | K | R |
| 21-225_147A8_LC | . | . | . | . | Q | L | . | . | . | . | . | . | . | . | . | . | . | . | . |
| 21-225_32H2_LC | . | . | . | . | Y | . | . | . | . | . | . | . | . | . | . | . | . | . | . |
| 21-225_49C1_LC | . | . | . | . | I | . | . | . | L | . | . | . | . | . | . | . | . | . | . |
| 21-225_49E7_LC | . | . | . | . | L | . | . | I | . | . | . | . | . | . | . | . | . | . | . |
| 21-225_49F9_LC | . | . | . | . | Y | . | . | . | . | . | . | . | . | . | . | . | . | . | . |
| 21-225_49G3_LC | . | . | . | . | L | . | . | . | . | . | . | . | . | . | . | . | . | . | . |
| 21-225_52D10_LC | . | . | . | . | Y | . | . | . | . | . | . | . | . | . | . | . | . | . | . |
| 21-225_52G8_LC | . | . | . | . | L | . | . | I | . | . | . | . | . | . | . | . | . | . | . |
| 21-225_55C2_LC | . | . | . | . | Y | . | . | . | . | . | . | . | . | . | . | . | . | . | . |
| 21-225_55F9_LC | . | . | . | . | L | . | . | . | . | . | . | . | . | . | . | . | . | . | . |

**FIGURE 57**

Table 125. Consensus 45 - VK3|A27/JK3 (SEQ ID NO: 50342):

EIVLTQSPGTLSLFPGERATLSCRAS--QSVIS-----SYLAWYQQKPGQAPRLLIFG--------VSSRATGIPDRFSGSGSG--TDFTLTISRLEPEDFAVYYCQQYGR----------------------SPFNFGPGTKVDIKR wherein:

Q at position 29 can be substituted with R

S at position 30 can be substituted with G or N

V at position 31 can be substituted with I

I at position 32 can be substituted with S or G

S at position 33 can be substituted with N

S at position 39 can be substituted with I or N

Y at position 40 can be substituted with F

V at position 67 can be substituted with A or T

S at position 69 can be substituted with N or T

R at position 70 can be substituted with W

Q at position 107 can be substituted with H

Q at position 108 can be substituted with H

Y at position 109 can be substituted with N

G at position 110 can be substituted with D

R at position 111 can be substituted with null (-), N or Y

P at position 136 can be substituted with L or M

**FIGURE 57**

N at position 138 can be substituted with T

Arg Ala Ser Xaa Xaa Xaa Xaa Xaa Xaa Xaa Leu Ala (SEQ ID NO: 50206)

Gly Xaa Ser Xaa Xaa Ala Thr (SEQ ID NO: 50207)

Xaa Xaa Xaa Xaa Xaa Ser Xaa Xaa Asn (SEQ ID NO: 50208)

Arg Ala Ser Gln Ser Val Ile Ser Ser Tyr Leu Ala (SEQ ID NO: 50696)

Gly Val Ser Ser Arg Ala Thr (SEQ ID NO: 50697)

Gln Gln Tyr Gly Arg Ser Pro Phe Asn (SEQ ID NO: 50698)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | E | I | V | L | T | Q | S | P | G | T | L | S | L | | P | G | E | R | A | T | L | S | C | R | A | S |
| 21-225_146G4_LC | . | . | . | . | . | . | . | . | . | . | . | . | . | F | . | . | . | . | . | . | . | . | . | . | . | . |
| 21-225_147D2_LC | . | . | . | . | . | . | . | . | . | . | . | . | . | S | . | . | . | . | . | . | . | . | . | . | . | . |
| 21-225_147E3_LC | . | . | . | . | . | . | . | . | . | . | . | . | . | S | . | . | . | . | . | . | . | . | . | . | . | . |
| 21-225_149D7_LC | . | . | . | . | . | . | . | . | . | . | . | . | . | F | . | . | . | . | V | . | . | . | . | . | . | . |
| 21-225_149D8_LC | . | . | . | . | . | . | . | . | . | . | . | . | . | F | . | . | . | . | . | . | . | . | . | . | . | . |
| 21-225_152E7_LC | . | . | . | . | . | . | . | . | . | . | . | . | . | F | . | . | . | . | . | . | . | . | . | . | . | . |
| 21-225_153A6_LC | . | . | . | . | . | . | . | . | . | . | . | . | . | F | . | . | . | . | . | P | . | . | . | . | . | . |
| 21-225_194A4_LC | . | . | . | . | . | . | . | . | . | . | . | . | . | S | . | . | . | . | . | . | . | . | . | . | . | . |
| 21-225_216F10_LC | . | V | . | . | . | . | . | . | . | . | . | . | . | S | . | . | . | . | . | . | . | . | . | . | . | . |
| 21-225_59H5_LC | . | . | . | . | . | . | . | . | . | . | . | . | . | S | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | V | I | S | - | - | - | - | - | S | Y | L | A | W | Y | Q | Q | K | P | G | Q | A | P |
| 21-225_146G4_LC | . | . | . | N | I | . | . | . | . | . | . | . | . | . | . | . | . | . | . | . | . | . | . | . | . | . |
| 21-225_147D2_LC | . | . | . | . | . | G | . | . | . | . | . | . | . | . | . | . | . | . | . | . | . | . | . | . | . | . |
| 21-225_147E3_LC | . | . | . | . | . | . | . | . | . | . | . | . | . | . | . | . | . | . | . | . | . | . | . | . | . | . |
| 21-225_149D7_LC | . | . | . | . | I | . | . | . | . | . | . | . | N | . | . | . | . | . | . | . | . | . | . | . | . | . |
| 21-225_149D8_LC | . | . | . | . | . | . | . | . | . | . | . | . | . | . | . | . | . | . | . | . | . | . | . | . | . | . |
| 21-225_152E7_LC | . | . | . | . | . | . | . | . | . | . | . | . | . | . | . | . | . | . | . | . | . | . | . | . | . | . |
| 21-225_153A6_LC | . | . | . | . | . | . | . | . | . | . | . | . | . | . | . | . | . | . | . | . | . | . | . | . | . | . |
| 21-225_194A4_LC | . | . | R | G | . | S | N | . | . | . | . | . | I | . | . | . | . | . | . | . | . | . | . | . | . | . |
| 21-225_216F10_LC | . | . | . | . | . | S | . | . | . | . | . | . | . | F | . | . | . | . | . | . | . | . | . | . | . | . |
| 21-225_59H5_LC | . | . | . | . | . | S | . | . | . | . | . | . | I | . | . | . | . | F | L | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | R | L | L | I | | G | - | - | - | - | - | - | - | - | V | S | S | R | A | T | G | I | P | D | R | F |
| 21-225_146G4_LC | . | . | . | . | F | . | . | . | . | . | . | . | . | . | . | . | . | . | . | . | . | . | . | . | . | . |
| 21-225_147D2_LC | . | . | . | . | Y | . | . | . | . | . | . | . | . | . | A | . | . | . | . | . | . | . | . | . | . | . |
| 21-225_147E3_LC | . | . | . | . | Y | . | . | . | . | . | . | . | . | . | A | . | . | . | . | . | A | . | . | . | . | . |
| 21-225_149D7_LC | . | . | . | . | F | . | . | . | . | . | . | . | . | . | . | . | . | . | . | . | . | . | . | . | . | . |
| 21-225_149D8_LC | . | . | . | . | F | . | . | . | . | . | . | . | . | . | . | . | . | W | . | . | . | . | . | . | . | . |
| 21-225_152E7_LC | . | . | . | . | F | . | . | . | . | . | . | . | . | . | . | . | . | . | . | . | . | . | . | . | . | . |
| 21-225_153A6_LC | . | . | . | . | F | . | . | . | . | . | . | . | . | . | . | . | . | . | . | . | . | . | . | . | . | . |
| 21-225_194A4_LC | . | . | . | . | Y | . | . | . | . | . | . | . | . | . | A | . | N | . | . | . | . | . | . | . | . | . |
| 21-225_216F10_LC | . | . | . | . | Y | . | . | . | . | . | . | . | . | . | T | . | T | . | . | . | . | . | . | . | . | . |
| 21-225_59H5_LC | . | . | . | . | Y | . | . | . | . | . | . | . | . | . | A | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | R | L | E | P | E | D | F | A | V | Y |
| 21-225_146G4_LC | . | . | . | . | . | . | . | . | . | . | . | . | . | . | . | . | . | . | . | . | . | . | . | . | . | . |
| 21-225_147D2_LC | . | . | . | . | . | . | . | . | . | . | . | . | . | . | . | . | . | . | . | . | . | . | . | . | . | . |
| 21-225_147E3_LC | . | . | . | . | . | . | . | . | . | . | . | . | . | . | . | . | . | . | . | . | . | . | . | . | . | . |
| 21-225_149D7_LC | . | . | . | . | . | . | . | . | . | . | . | . | . | . | . | . | . | . | . | . | . | . | . | . | . | . |
| 21-225_149D8_LC | . | . | . | . | . | . | . | . | . | . | . | . | . | . | . | . | . | . | . | . | . | . | . | . | . | . |
| 21-225_152E7_LC | . | . | . | . | . | . | . | . | . | . | . | . | . | . | . | . | . | . | . | . | . | . | . | . | . | . |
| 21-225_153A6_LC | . | . | . | . | . | . | . | . | . | . | . | . | . | . | . | . | . | . | . | . | . | . | . | . | . | . |
| 21-225_194A4_LC | . | . | . | . | . | . | . | . | . | . | . | . | . | . | . | . | . | . | . | . | . | . | . | . | . | . |
| 21-225_216F10_LC | . | . | . | . | . | . | . | . | . | . | . | I | . | . | . | . | . | . | . | . | . | . | . | . | . | . |
| 21-225_59H5_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | G | R | - | - | - | - | - | - | - | - | - | - | - | - | - | - | - | - | - | - | - |
| 21-225_146G4_LC | . | . | . | . | . | . | . | . | . | . | . | . | . | . | . | . | . | . | . | . | . | . | . | . | . | . |
| 21-225_147D2_LC | . | . | . | . | . | . | - | . | . | . | . | . | . | . | . | . | . | . | . | . | . | . | . | . | . | . |
| 21-225_147E3_LC | . | . | . | . | . | . | . | . | . | . | . | . | . | . | . | . | . | . | . | . | . | . | . | . | . | . |
| 21-225_149D7_LC | . | . | . | . | . | . | . | . | . | . | . | . | . | . | . | . | . | . | . | . | . | . | . | . | . | . |
| 21-225_149D8_LC | . | . | . | . | . | . | . | . | . | . | . | . | . | . | . | . | . | . | . | . | . | . | . | . | . | . |
| 21-225_152E7_LC | . | . | . | . | . | . | . | . | . | . | . | . | . | . | . | . | . | . | . | . | . | . | . | . | . | . |
| 21-225_153A6_LC | . | . | . | . | . | . | . | . | . | . | . | . | . | . | . | . | . | . | . | . | . | . | . | . | . | . |
| 21-225_194A4_LC | . | . | . | H | N | D | Y | . | . | . | . | . | . | . | . | . | . | . | . | . | . | . | . | . | . | . |
| 21-225_216F10_LC | . | . | . | . | . | D | . | . | . | . | . | . | . | . | . | . | . | . | . | . | . | . | . | . | . | . |
| 21-225_59H5_LC | . | . | H | . | . | . | N | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | S | P | F | N | F | G | P | G | T | K | V | D | I | K | R |
| 21-225_146G4_LC | . | . | . | . | . | . | . | . | . | . | . | . | . | . | . | . | . | N | . |
| 21-225_147D2_LC | . | . | . | . | . | L | . | T | . | . | . | . | . | . | . | . | . | . | . |
| 21-225_147E3_LC | . | . | . | . | . | . | . | . | . | . | . | . | . | . | . | . | . | . | . |
| 21-225_149D7_LC | . | . | . | . | . | . | . | . | . | . | . | . | . | . | . | . | . | . | . |
| 21-225_149D8_LC | . | . | . | . | . | . | . | . | . | . | . | . | . | . | . | . | . | . | . |
| 21-225_152E7_LC | . | . | . | . | . | . | . | . | . | . | . | . | . | . | . | . | . | . | . |
| 21-225_153A6_LC | . | . | . | . | . | . | . | . | . | . | . | . | . | . | . | . | . | . | . |
| 21-225_194A4_LC | . | . | . | . | . | M | . | T | . | . | . | . | . | . | . | . | . | . | . |
| 21-225_216F10_LC | . | . | . | . | . | . | . | T | . | . | . | . | . | . | . | . | . | . | . |
| 21-225_59H5_LC | . | . | . | . | . | . | . | T | . | . | . | . | . | . | . | . | F | . | . |

**FIGURE 57**

Table 126. Consensus 46 - VK3|L2/JK1 (SEQ ID NO: 50343):

EIVMTQSPATLSVSPGERATLSCRAS--QSVS------SNLAWYQQKPGQAPRLLIYG--------ASTRATGIPARFSGSGSG--TEFTLTISSLQSEDFAVYYCQQYND-----------------------WPWTFGQGTKVEIKR wherein:

A at position 25 can be substituted with S

S at position 30 can be substituted with D or T

V at position 31 can be substituted with I

S at position 32 can be substituted with N, R or I

S at position 39 can be substituted with I or T

N at position 40 can be substituted with Y

L at position 41 can be substituted with I

T at position 72 can be substituted with S

Q at position 108 can be substituted with E

Y at position 109 can be substituted with S

N at position 110 can be substituted with D, F or H

D at position 111 can be substituted with N, null (-) or T

null (-) at position 134 can be substituted with W

W at position 135 can be substituted with P, C or N

P at position 136 can be substituted with L or W

W at position 137 can be substituted with L, C, P or R

**FIGURE 57**

T at position 138 can be substituted with P or S

Arg Xaa Ser Gln Xaa Xaa Xaa Xaa Xaa Xaa Ala (SEQ ID NO: 50209)

Gly Ala Ser Thr Arg Ala Xaa (SEQ ID NO: 50210)

Gln Xaa Xaa Xaa Xaa Xaa Xaa Xaa Xaa Xaa (SEQ ID NO: 50211)

Arg Ala Ser Gln Ser Val Ser Ser Asn Leu Ala (SEQ ID NO: 50699)

Gly Ala Ser Thr Arg Ala Thr (SEQ ID NO: 50700)

Gln Gln Tyr Asn Asp Trp Pro Trp Thr (SEQ ID NO: 50701)

# FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | E | I | V | M | T | Q | S | P | A | T | L | S | V | S | P | G | E | R | A | T | L | S | C | R | A | S |
| 21-225_13D3_LC | . | . | . | . | . | . | . | . | . | . | . | . | . | . | . | . | . | S | . | . | . | . | . | . | . | . |
| 21-225_151F1_LC | . | M | . | . | . | . | . | . | . | . | . | . | . | . | . | . | . | . | V | . | . | . | . | . | . | . |
| 21-225_155H9_LC | . | . | . | . | . | . | . | . | . | . | . | . | . | . | . | . | . | . | . | . | . | . | . | . | . | . |
| 21-225_161A4_LC | . | . | . | . | . | . | . | . | . | . | . | . | . | . | . | . | . | . | . | S | . | . | . | . | S | . |
| 21-225_17D8_LC | . | . | . | . | . | . | . | . | S | . | . | . | . | . | . | . | . | . | . | . | . | . | . | . | . | . |
| 21-225_191C8_LC | . | . | . | . | . | . | . | . | . | . | . | . | . | . | . | . | . | . | . | . | . | . | . | . | . | . |
| 21-225_20B8_LC | . | . | . | . | . | . | . | . | S | . | . | . | . | . | . | . | . | . | V | . | . | . | . | . | . | . |
| 21-225_25B8_LC | . | . | . | . | . | . | . | . | . | . | . | . | . | . | . | . | . | . | . | . | . | . | . | . | . | . |
| 21-225_28C5_LC | . | . | . | . | . | . | . | . | . | . | . | . | . | . | . | . | . | . | . | . | . | . | . | . | . | . |
| 21-225_85H1_LC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | V | S | - | - | - | - | - | - | S | N | L | A | W | Y | Q | Q | K | P | G | Q | A | P |
| 21-225_13D3_LC | . | . | . | T | . | . | . | . | . | . | . | . | . | . | . | . | . | F | . | . | . | . | . | . | . | . |
| 21-225_151F1_LC | . | . | . | . | I | N | . | . | . | . | . | . | I | . | I | . | . | . | . | . | . | . | . | . | . | . |
| 21-225_155H9_LC | . | . | . | . | . | . | . | . | . | . | . | . | . | . | . | . | . | . | . | . | . | . | . | . | . | . |
| 21-225_161A4_LC | . | . | . | . | . | N | . | . | . | . | . | . | . | . | . | . | . | . | . | . | R | . | . | . | . | L |
| 21-225_17D8_LC | . | . | . | . | . | . | . | . | . | . | . | . | . | . | . | . | . | F | . | . | . | . | . | . | . | . |
| 21-225_191C8_LC | . | . | . | . | . | R | . | . | . | . | . | . | . | . | . | . | . | . | . | . | E | . | . | . | . | . |
| 21-225_20B8_LC | . | . | . | . | . | . | . | . | . | . | . | . | . | . | . | . | . | F | . | . | . | . | . | . | . | . |
| 21-225_25B8_LC | . | . | . | . | . | R | . | . | . | . | . | . | . | . | . | . | . | . | . | . | . | . | . | . | . | . |
| 21-225_28C5_LC | . | . | . | . | . | . | . | . | . | . | . | . | . | . | . | . | . | . | H | . | . | . | . | . | . | . |
| 21-225_85H1_LC | . | . | . | D | . | I | . | . | . | . | . | . | T | Y | . | . | . | . | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | R | L | L | I | Y | G | - | - | - | - | - | - | - | - | A | S | T | R | A | T | G | I | P | A | R | F |
| 21-225_13D3_LC | . | . | . | . | N | . | . | . | . | . | . | . | . | . | . | . | . | . | . | . | . | . | . | . | . | . |
| 21-225_151F1_LC | . | . | . | . | . | . | . | . | . | . | . | . | . | . | . | . | . | . | . | . | . | . | . | . | . | . |
| 21-225_155H9_LC | . | . | . | . | . | . | . | . | . | . | . | . | . | . | . | . | . | . | . | . | . | . | . | . | . | . |
| 21-225_161A4_LC | . | . | . | . | . | . | . | . | . | . | . | . | . | . | . | . | I | . | . | . | D | . | . | . | . | . |
| 21-225_17D8_LC | . | . | . | . | N | . | . | . | . | . | . | . | . | . | . | . | . | . | . | S | . | . | . | . | . | . |
| 21-225_191C8_LC | . | . | . | . | . | . | . | . | . | . | . | . | . | . | . | . | . | . | . | . | . | . | . | . | . | . |
| 21-225_20B8_LC | . | . | . | . | N | . | . | . | . | . | . | . | . | . | . | . | . | . | . | S | . | . | . | . | . | . |
| 21-225_25B8_LC | . | . | . | . | . | . | . | . | . | . | . | . | . | . | . | . | . | . | . | . | . | . | . | . | . | . |
| 21-225_28C5_LC | . | . | . | . | . | . | . | . | . | . | . | . | . | . | . | . | . | . | . | . | . | . | . | . | . | . |
| 21-225_85H1_LC | . | . | . | . | . | . | . | . | . | . | . | . | . | . | . | . | . | . | . | . | . | V | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | S | E | D | F | A | V | Y |
| 21-225_13D3_LC | . | . | . | . | . | . | . | . | . | . | . | . | . | . | . | . | . | . | . | . | . | . | . | . | . | . |
| 21-225_151F1_LC | . | . | . | . | . | . | . | . | . | . | . | . | . | . | . | . | . | . | . | . | . | . | . | . | . | . |
| 21-225_155H9_LC | . | . | . | . | . | . | . | . | . | . | . | . | . | . | . | . | . | . | . | . | . | . | . | . | . | . |
| 21-225_161A4_LC | N | . | . | . | . | . | . | . | . | . | . | . | . | . | . | . | . | . | . | . | . | . | . | . | . | . |
| 21-225_17D8_LC | . | . | . | . | . | . | . | . | . | . | . | . | . | . | . | . | . | . | . | . | . | . | . | . | . | . |
| 21-225_191C8_LC | . | . | . | . | . | . | . | . | . | . | . | . | . | . | . | . | . | . | . | . | . | . | . | . | . | . |
| 21-225_20B8_LC | . | . | . | . | . | . | . | . | . | . | . | . | . | . | . | . | . | . | . | . | . | . | . | . | . | . |
| 21-225_25B8_LC | . | . | . | . | . | . | . | . | . | . | . | . | . | . | . | . | . | . | . | . | . | . | . | . | L | . |
| 21-225_28C5_LC | . | . | . | . | . | . | . | . | . | . | . | . | . | . | . | . | . | . | . | . | . | . | . | . | L | . |
| 21-225_85H1_LC | . | . | . | . | . | . | . | . | . | . | . | . | . | . | . | . | . | . | . | . | . | . | . | . | L | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | N | | - | - | - | - | - | - | - | - | - | - | - | - | - | - | - | - | - | - | - |
| 21-225_13D3_LC | F | . | . | . | . | H | D | . | . | . | . | . | . | . | . | . | . | . | . | . | . | . | . | . | . | . |
| 21-225_151F1_LC | . | . | . | . | . | . | D | . | . | . | . | . | . | . | . | . | . | . | . | . | . | . | . | . | . | . |
| 21-225_155H9_LC | . | . | . | . | . | D | D | . | . | . | . | . | . | . | . | . | . | . | . | . | . | . | . | . | . | . |
| 21-225_161A4_LC | F | . | . | . | . | . | - | . | . | . | . | . | . | . | . | . | . | . | . | . | . | . | . | . | . | . |
| 21-225_17D8_LC | . | . | . | . | . | . | N | . | . | . | . | . | . | . | . | . | . | . | . | . | . | . | . | . | . | . |
| 21-225_191C8_LC | . | . | . | . | S | F | T | . | . | . | . | . | . | . | . | . | . | . | . | . | . | . | . | . | . | . |
| 21-225_20B8_LC | . | . | . | . | . | . | N | . | . | . | . | . | . | . | . | . | . | . | . | . | . | . | . | . | . | . |
| 21-225_25B8_LC | . | . | . | . | . | . | N | . | . | . | . | . | . | . | . | . | . | . | . | . | . | . | . | . | . | . |
| 21-225_28C5_LC | . | . | . | . | . | . | N | . | . | . | . | . | . | . | . | . | . | . | . | . | . | . | . | . | . | . |
| 21-225_85H1_LC | . | . | . | E | . | . | D | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | | P | W | T | F | G | Q | G | T | K | V | E | I | K | R |
| 21-225_13D3_LC | . | . | . | . | W | . | P | . | . | . | . | . | . | . | . | . | . | . | . |
| 21-225_151F1_LC | . | . | . | W | P | . | . | . | . | . | . | . | . | . | . | . | . | . | . |
| 21-225_155H9_LC | . | . | . | W | P | . | . | . | . | . | . | . | . | . | . | . | . | . | . |
| 21-225_161A4_LC | . | . | . | . | N | W | . | . | . | . | . | . | . | T | . | . | . | . | . |
| 21-225_17D8_LC | . | . | . | . | W | . | L | . | . | . | . | . | . | . | . | . | . | . | . |
| 21-225_191C8_LC | . | . | . | . | W | L | R | . | . | . | . | . | . | . | . | . | . | . | . |
| 21-225_20B8_LC | . | . | . | . | C | . | L | . | . | . | . | . | . | . | . | . | . | . | . |
| 21-225_25B8_LC | . | . | . | W | P | . | . | P | . | . | . | . | . | . | . | . | . | . | . |
| 21-225_28C5_LC | . | . | . | W | P | . | . | P | . | . | . | . | . | . | . | . | . | . | . |
| 21-225_85H1_LC | . | . | . | . | W | . | C | S | . | . | . | . | . | . | . | . | . | . | . |

**FIGURE 57**

Table 127. Consensus 47 - VK6|A26/JK1 (SEQ ID NO: 50344):

EIVLTQSPDFQSVTPKEKVTITCRAS--QSIG------SSLHWYQQKPDQSPKLLIKY--------ASQSFSGVPSRFSGSGSG--TDFTLTINSLEAEDAATYYCHQSSS----------------------LPWTFGQGTKVEIKR wherein:

S at position 30 can be substituted with N

S at position 39 can be substituted with N

S at position 40 can be substituted with N or T

Y at position 58 can be substituted with S

H at position 107 can be substituted with Q

S at position 110 can be substituted with G or R

L at position 135 can be substituted with F

W at position 137 can be substituted with R or Q

Arg Ala Ser Gln Xaa Ile Gly Xaa Xaa Leu His (SEQ ID NO: 50256)

Xaa Ala Ser Gln Ser Phe Ser (SEQ ID NO: 50257)

Xaa Gln Ser Xaa Ser Xaa Pro Xaa Thr (SEQ ID NO: 50258)

Arg Ala Ser Gln Ser Ile Gly Ser Ser Leu His (SEQ ID NO: 50702)

Tyr Ala Ser Gln Ser Phe Ser (SEQ ID NO: 50703)

His Gln Ser Ser Ser Leu Pro Trp Thr (SEQ ID NO: 50704)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | E | I | V | L | T | Q | S | P | D | F | Q | S | V | T | P | K | E | K | V | T | I | T | C | R | A | S |
| 21-225_190B11_LC | . | . | . | . | . | . | . | . | . | . | . | . | . | A | . | . | . | . | . | . | . | . | . | . | . | . |
| 21-225_190D5_LC | . | . | . | . | . | . | . | . | . | . | . | . | . | A | . | . | . | . | . | . | . | . | . | . | . | . |
| 21-225_190D9_LC | . | . | . | . | . | . | F | . | . | S | . | . | . | . | . | . | . | . | . | . | . | . | . | . | . | . |
| 21-225_190F11_LC | . | . | . | . | . | . | F | . | . | S | . | . | . | . | . | . | . | . | . | . | . | . | . | . | . | . |
| 21-225_190H8_LC | . | . | . | . | . | . | F | . | . | S | . | . | . | . | . | . | . | . | . | . | . | . | . | . | . | . |
| 21-225_191E5_LC | . | . | . | . | . | . | F | . | . | S | . | . | . | . | . | . | . | . | . | . | . | . | . | . | . | . |
| 21-225_194C3_LC | . | . | . | . | . | . | . | . | . | . | . | . | . | A | . | . | . | . | . | . | . | . | . | . | . | . |
| 21-225_200B7_LC | . | . | . | . | . | . | . | . | . | . | . | . | . | . | . | . | . | . | . | . | . | . | . | . | . | . |
| 21-225_200F8_LC | . | . | . | . | . | . | . | . | . | . | . | . | . | A | . | . | . | . | . | . | . | . | . | . | . | . |
| 21-225_217A3_LC | . | . | . | . | . | . | . | . | . | . | K | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | I | G | - | - | - | - | - | - | S | S | L | H | W | Y | Q | Q | K | P | D | Q | S | P |
| 21-225_190B11_LC | . | . | . | . | . | . | . | . | . | . | . | . | . | N | . | . | . | . | . | . | . | . | . | . | . | . |
| 21-225_190D5_LC | . | . | . | . | . | . | . | . | . | . | . | . | . | N | . | . | . | . | . | . | . | . | . | . | . | . |
| 21-225_190D9_LC | . | . | . | . | . | . | . | . | . | . | . | . | . | . | . | . | . | . | . | . | . | . | . | . | . | . |
| 21-225_190F11_LC | . | . | . | N | . | . | . | . | . | . | . | . | . | . | . | . | . | . | . | . | . | . | E | . | . | . |
| 21-225_190H8_LC | . | . | . | . | . | . | . | . | . | . | . | . | . | . | . | . | . | . | . | . | . | . | . | . | . | . |
| 21-225_191E5_LC | . | . | . | . | . | . | . | . | . | . | . | . | . | . | . | . | . | . | . | . | . | . | . | . | . | . |
| 21-225_194C3_LC | . | . | . | . | . | . | . | . | . | . | . | . | . | N | . | . | . | . | . | . | . | . | . | . | . | . |
| 21-225_200B7_LC | . | . | . | N | . | . | . | . | . | . | . | . | N | T | . | . | . | . | . | . | . | . | . | . | . | . |
| 21-225_200F8_LC | . | . | . | . | . | . | . | . | . | . | . | . | . | N | . | . | . | . | . | . | . | . | . | . | . | . |
| 21-225_217A3_LC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | K | Y | - | - | - | - | - | - | - | - | A | S | Q | S | F | S | G | V | P | S | R | F |
| 21-225_190B11_LC | . | . | . | . | . | S | . | . | . | . | . | . | . | . | . | . | . | . | . | . | . | . | . | . | . | . |
| 21-225_190D5_LC | . | . | . | . | . | S | . | . | . | . | . | . | . | . | . | . | . | . | . | . | . | . | . | . | . | . |
| 21-225_190D9_LC | . | . | . | . | . | . | . | . | . | . | . | . | . | . | . | . | . | . | . | . | . | . | . | . | . | . |
| 21-225_190F11_LC | . | V | . | . | . | . | . | . | . | . | . | . | . | . | . | . | . | . | . | . | . | . | . | . | . | . |
| 21-225_190H8_LC | . | . | . | . | . | . | . | . | . | . | . | . | . | . | . | . | . | . | . | . | . | . | . | . | . | . |
| 21-225_191E5_LC | . | . | . | . | . | . | . | . | . | . | . | . | . | . | . | . | . | . | . | . | . | . | . | . | . | . |
| 21-225_194C3_LC | . | . | . | . | . | S | . | . | . | . | . | . | . | . | . | . | . | . | . | . | . | . | . | . | . | . |
| 21-225_200B7_LC | . | . | . | . | . | . | . | . | . | . | . | . | . | . | . | . | . | . | . | . | . | . | . | . | . | . |
| 21-225_200F8_LC | . | . | . | . | . | S | . | . | . | . | . | . | . | . | . | . | . | . | . | . | . | . | . | . | . | . |
| 21-225_217A3_LC | . | . | . | V | . | . | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | N | S | L | E | A | E | D | A | A | T | Y |
| 21-225_190B11_LC | . | . | . | . | . | . | . | . | . | . | . | . | . | . | . | . | . | . | . | T | . | . | . | . | . | . |
| 21-225_190D5_LC | . | . | . | . | . | . | . | . | . | . | . | . | . | . | . | . | . | . | . | T | . | . | . | . | . | . |
| 21-225_190D9_LC | . | . | . | . | . | . | . | . | . | . | . | . | . | . | . | . | . | . | . | . | . | . | . | . | . | . |
| 21-225_190F11_LC | . | . | . | . | . | . | . | . | . | . | . | . | . | . | . | . | . | . | . | . | . | . | . | . | . | . |
| 21-225_190H8_LC | . | . | . | . | . | . | . | . | . | . | . | . | . | . | . | . | . | . | . | . | . | . | . | . | . | . |
| 21-225_191E5_LC | . | . | . | . | . | . | . | . | . | . | . | . | . | . | . | . | . | . | . | . | . | . | . | . | . | . |
| 21-225_194C3_LC | . | . | . | . | . | . | . | . | . | . | . | . | . | . | . | . | . | . | . | T | . | . | . | . | . | . |
| 21-225_200B7_LC | . | . | . | . | . | . | . | . | . | . | . | I | . | . | . | . | . | . | . | . | . | . | . | . | . | . |
| 21-225_200F8_LC | . | . | . | . | . | . | . | . | . | . | . | . | . | . | . | . | . | . | . | T | . | . | . | . | . | . |
| 21-225_217A3_LC | . | . | . | . | . | . | . | . | . | . | . | . | . | . | . | . | . | . | . | . | . | . | G | . | . | . |

# FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | H | Q | S | S | S | - | - | - | - | - | - | - | - | - | - | - | - | - | - | - | - | - | - | - |
| 21-225_190B11_LC | . | . | Q | . | . | . | . | . | . | . | . | . | . | . | . | . | . | . | . | . | . | . | . | . | . | . |
| 21-225_190D5_LC | . | . | Q | . | . | . | . | . | . | . | . | . | . | . | . | . | . | . | . | . | . | . | . | . | . | . |
| 21-225_190D9_LC | . | . | . | . | . | . | . | . | . | . | . | . | . | . | . | . | . | . | . | . | . | . | . | . | . | . |
| 21-225_190F11_LC | . | . | . | . | . | . | . | . | . | . | . | . | . | . | . | . | . | . | . | . | . | . | . | . | . | . |
| 21-225_190H8_LC | . | . | . | . | . | . | . | . | . | . | . | . | . | . | . | . | . | . | . | . | . | . | . | . | . | . |
| 21-225_191E5_LC | . | . | . | . | . | . | . | . | . | . | . | . | . | . | . | . | . | . | . | . | . | . | . | . | . | . |
| 21-225_194C3_LC | . | . | Q | . | . | . | . | . | . | . | . | . | . | . | . | . | . | . | . | . | . | . | . | . | . | . |
| 21-225_200B7_LC | . | . | . | . | . | G | . | . | . | . | . | . | . | . | . | . | . | . | . | . | . | . | . | . | . | . |
| 21-225_200F8_LC | . | . | Q | . | . | . | . | . | . | . | . | . | . | . | . | . | . | . | . | . | . | . | . | . | . | . |
| 21-225_217A3_LC | . | . | . | . | . | R | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | L | P | W | T | F | G | Q | G | T | K | V | E | I | K | R |
| 21-225_190B11_LC | . | . | . | . | . | . | . | . | . | . | . | . | . | . | . | . | . | . | . |
| 21-225_190D5_LC | . | . | . | . | . | . | . | . | . | . | . | . | . | . | . | . | . | . | . |
| 21-225_190D9_LC | . | . | . | . | F | . | R | . | . | . | . | . | . | . | . | . | . | . | . |
| 21-225_190F11_LC | . | . | . | . | F | . | R | . | . | . | . | . | . | . | . | . | . | . | . |
| 21-225_190H8_LC | . | . | . | . | F | . | R | . | . | . | . | . | . | . | . | . | . | . | . |
| 21-225_191E5_LC | . | . | . | . | F | . | R | . | . | . | . | . | . | . | . | . | . | . | . |
| 21-225_194C3_LC | . | . | . | . | . | . | . | . | . | . | . | . | . | . | . | . | . | . | . |
| 21-225_200B7_LC | . | . | . | . | . | . | Q | . | . | . | . | . | . | . | . | . | . | . | . |
| 21-225_200F8_LC | . | . | . | . | . | . | . | . | . | . | . | . | . | . | . | . | . | . | . |
| 21-225_217A3_LC | . | . | . | . | . | . | . | . | . | . | . | . | . | . | . | . | . | . | . |

**FIGURE 57**

Table 128. Consensus 48 - VK1|A30/JK2 (SEQ ID NO: 50345):

DIQMTQSPSSLSASVGDRVTITC<u>RAS--QGIR------NDLG</u>WYQQKPGKAPKRLIY<u>A--------ASSLQS</u>GVPSRFSGSGSG--TEFTLTISSLQPEDFATYYC<u>LQHYS-----------------------YPRS</u>FGQGTKLEIKR wherein:

G at position 30 can be substituted with A

R at position 32 can be substituted with G

N at position 39 can be substituted with D

A at position 67 can be substituted with T

Y at position 110 can be substituted with N

S at position 111 can be substituted with N

Y at position 135 can be substituted with F

R at position 137 can be substituted with Y

Arg Ala Ser Gln Xaa Ile Xaa Xaa Asp Leu Gly (SEQ ID NO: 50247)

Ala Xaa Ser Ser Leu Gln Ser (SEQ ID NO: 50248)

Leu Gln His Xaa Xaa Xaa Pro Xaa Ser (SEQ ID NO: 50249)

Arg Ala Ser Gln Gly Ile Arg Asn Asp Leu Gly (SEQ ID NO: 50705)

Ala Ala Ser Ser Leu Gln Ser (SEQ ID NO: 50706)

Leu Gln His Tyr Ser Tyr Pro Arg Ser (SEQ ID NO: 50707)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | I | Q | M | T | Q | S | P | S | S | L | S | A | S | V | G | D | R | V | T | I | T | C | R | A | S |
| 21-225_150E2_LC | . | . | . | . | . | . | . | . | . | . | . | . | . | . | . | . | . | . | . | . | . | S | . | . | . | . |
| 21-225_152F7_LC | . | . | . | . | . | . | . | . | . | . | . | . | . | . | . | . | . | . | . | . | . | . | . | . | . | . |
| 21-225_176H12_LC | . | . | . | . | . | . | . | . | . | . | . | . | . | . | . | . | . | . | . | . | . | . | . | . | . | . |
| 21-225_177B4_LC | . | . | . | . | . | . | . | . | . | . | . | . | . | . | . | . | . | . | . | . | . | . | . | . | . | . |
| 21-225_25A12_LC | . | . | . | . | . | . | . | . | . | . | . | . | . | . | . | . | . | . | . | . | V | . | . | . | . | . |
| 21-225_26A11_LC | . | . | . | . | . | . | . | . | I | . | . | . | . | . | . | . | . | . | . | . | . | . | . | . | . | . |
| 21-225_28H2_LC | . | . | . | . | . | . | . | . | . | . | . | . | . | . | I | . | . | . | . | . | . | . | . | . | . | . |
| 21-225_29E2_LC | . | . | . | . | . | . | . | . | . | . | . | . | . | . | . | . | . | . | . | . | . | . | . | . | . | . |
| 21-225_61F2_LC | . | . | . | . | . | . | . | . | . | . | . | . | . | . | . | . | . | . | . | . | V | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | G | I | R | - | - | - | - | - | - | N | D | L | G | W | Y | Q | Q | K | P | G | K | A | P |
| 21-225_150E2_LC | . | . | . | . | . | G | . | . | . | . | . | . | . | . | . | . | . | . | . | . | . | . | . | . | . | . |
| 21-225_152F7_LC | . | . | . | . | . | . | . | . | . | . | . | . | . | . | . | . | . | . | . | . | . | . | . | . | . | . |
| 21-225_176H12_LC | . | . | . | . | . | . | . | . | . | . | . | . | . | . | . | . | . | . | . | . | . | . | . | . | . | . |
| 21-225_177B4_LC | . | . | . | . | . | . | . | . | . | . | . | . | . | . | . | . | . | . | . | . | . | . | . | . | . | . |
| 21-225_25A12_LC | . | . | . | A | . | . | . | . | . | . | . | . | D | . | . | . | . | . | . | . | R | . | . | . | . | . |
| 21-225_26A11_LC | . | . | . | A | . | . | . | . | . | . | . | . | . | . | . | . | . | . | . | . | R | . | . | . | . | . |
| 21-225_28H2_LC | . | . | . | . | . | . | . | . | . | . | . | . | . | . | . | . | . | . | . | . | . | . | . | . | . | . |
| 21-225_29E2_LC | . | . | . | A | . | . | . | . | . | . | . | . | . | . | . | . | . | . | . | . | R | . | . | . | . | . |
| 21-225_61F2_LC | . | . | . | . | . | . | . | . | . | . | . | . | D | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | R | L | I | Y | A | - | - | - | - | - | - | - | - | A | S | S | L | Q | S | G | V | P | S | R | F |
| 21-225_150E2_LC | T | . | . | . | . | . | . | . | . | . | . | . | . | . | . | . | . | . | . | . | . | . | . | . | . | . |
| 21-225_152F7_LC | . | . | . | . | . | . | . | . | . | . | . | . | . | . | T | . | . | . | . | . | . | . | . | . | . | . |
| 21-225_176H12_LC | N | . | . | . | . | . | . | . | . | . | . | . | . | . | . | . | . | . | . | . | . | . | . | . | . | . |
| 21-225_177B4_LC | . | . | . | . | . | . | . | . | . | . | . | . | . | . | . | . | . | . | . | . | . | . | . | . | . | . |
| 21-225_25A12_LC | . | . | . | . | . | . | . | . | . | . | . | . | . | . | . | . | . | . | . | . | . | . | . | . | . | . |
| 21-225_26A11_LC | . | . | . | . | . | . | . | . | . | . | . | . | . | . | . | . | . | . | . | . | . | . | . | . | . | . |
| 21-225_28H2_LC | . | . | . | . | . | . | . | . | . | . | . | . | . | . | . | . | . | . | . | . | . | . | . | . | . | . |
| 21-225_29E2_LC | . | . | . | . | . | . | . | . | . | . | . | . | . | . | . | . | . | . | . | . | . | . | . | . | . | . |
| 21-225_61F2_LC | E | . | . | . | . | . | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | P | E | D | F | A | T | Y |
| 21-225_150E2_LC | . | . | . | . | . | . | . | . | . | . | . | . | . | . | . | . | . | . | . | . | A | . | . | . | . | . |
| 21-225_152F7_LC | . | . | . | . | . | . | . | . | . | . | . | . | . | . | . | . | . | . | . | . | . | . | . | . | . | . |
| 21-225_176H12_LC | . | . | . | . | . | . | . | . | . | . | . | . | . | . | . | . | . | . | . | H | . | . | . | . | . | . |
| 21-225_177B4_LC | . | . | . | . | . | . | . | . | . | . | . | . | . | . | . | . | . | . | . | . | . | . | . | . | . | . |
| 21-225_25A12_LC | . | . | . | . | . | . | . | . | . | . | . | I | . | . | . | . | . | . | . | . | . | . | . | . | . | . |
| 21-225_26A11_LC | . | . | . | . | . | . | . | . | . | . | . | I | . | . | . | . | . | . | . | . | . | . | . | . | . | . |
| 21-225_28H2_LC | . | . | . | . | . | . | . | . | . | . | . | . | . | . | . | . | . | . | . | . | . | . | . | . | . | . |
| 21-225_29E2_LC | . | . | . | . | . | . | . | . | . | . | . | I | . | . | . | . | . | . | . | . | . | . | . | . | . | . |
| 21-225_61F2_LC | . | . | . | . | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | L | Q | H | Y | S | - | - | - | - | - | - | - | - | - | - | - | - | - | - | - | - | - | - | - |
| 21-225_150E2_LC | . | . | . | . | . | . | . | . | . | . | . | . | . | . | . | . | . | . | . | . | . | . | . | . | . | . |
| 21-225_152F7_LC | . | . | . | . | . | . | N | . | . | . | . | . | . | . | . | . | . | . | . | . | . | . | . | . | . | . |
| 21-225_176H12_LC | . | G | . | . | . | N | . | . | . | . | . | . | . | . | . | . | . | . | . | . | . | . | . | . | . | . |
| 21-225_177B4_LC | . | . | . | . | . | . | . | . | . | . | . | . | . | . | . | . | . | . | . | . | . | . | . | . | . | . |
| 21-225_25A12_LC | . | . | . | . | . | . | N | . | . | . | . | . | . | . | . | . | . | . | . | . | . | . | . | . | . | . |
| 21-225_26A11_LC | . | . | . | . | . | . | N | . | . | . | . | . | . | . | . | . | . | . | . | . | . | . | . | . | . | . |
| 21-225_28H2_LC | . | . | . | . | . | . | . | . | . | . | . | . | . | . | . | . | . | . | . | . | . | . | . | . | . | . |
| 21-225_29E2_LC | . | . | . | . | . | . | N | . | . | . | . | . | . | . | . | . | . | . | . | . | . | . | . | . | . | . |
| 21-225_61F2_LC | . | . | . | . | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | Y | P | R | S | F | G | Q | G | T | K | L | E | I | K | R |
| 21-225_150E2_LC | . | . | . | . | F | . | Y | . | . | . | . | . | . | . | . | . | . | . | . |
| 21-225_152F7_LC | . | . | . | . | F | . | Y | . | . | . | . | . | . | . | . | . | . | . | . |
| 21-225_176H12_LC | . | . | . | . | . | . | . | . | . | . | . | . | . | . | . | . | . | . | . |
| 21-225_177B4_LC | . | . | . | . | F | . | . | . | . | . | . | . | . | . | . | . | . | . | . |
| 21-225_25A12_LC | . | . | . | . | . | . | . | . | . | . | . | . | . | . | . | . | . | . | . |
| 21-225_26A11_LC | . | . | . | . | . | . | . | . | . | . | . | . | . | . | . | . | . | R | . |
| 21-225_28H2_LC | . | . | . | . | . | . | . | . | . | . | . | . | . | . | . | . | . | . | . |
| 21-225_29E2_LC | . | . | . | . | . | . | . | . | . | . | . | . | . | . | . | . | . | . | . |
| 21-225_61F2_LC | . | . | . | . | . | . | . | . | . | . | . | . | . | . | . | . | . | . | . |

**FIGURE 57**

Table 129. Consensus 49 - VK1|L1/JK5 (SEQ ID NO: 50346):

DIQMTQSPSSLSASVGDRVTITCRAS--QGIS------NYLAWFQQKPGKAPKSLIYA--------ASSLQSGVPSKFSGSGSG--TDFTLTISSLQPEDFATYYCQQYLS---------------------YPITFGQGTRLEIKR wherein:

R at position 24 can be substituted with Q

G at position 30 can be substituted with D

S at position 32 can be substituted with N

N at position 39 can be substituted with K

Y at position 40 can be substituted with F

A at position 42 can be substituted with N or V

A at position 58 can be substituted with D, G, or T

S at position 68 can be substituted with T

S at position 69 can be substituted with R or N

Q at position 71 can be substituted with H, L. or V

S at position 72 can be substituted with T

Q at position 107 can be substituted with H or L

Q at position 108 can be substituted with H or L

L at position 110 can be substituted with H, D, K, N, or Y

S at position 111 can be substituted with N, H, or T

Y at position 135 can be substituted with L

**FIGURE 57**

I at position 137 can be substituted with L

Xaa Ala Ser Gln Xaa Ile Xaa Xaa Xaa Leu Xaa (SEQ ID NO: 50212)

Xaa Ala Xaa Xaa Leu Xaa Xaa (SEQ ID NO: 50213)

Xaa Xaa Tyr Xaa Xaa Xaa Pro Xaa Thr (SEQ ID NO: 50214)

Arg Ala Ser Gln Gly Ile Ser Asn Tyr Leu Ala (SEQ ID NO: 50708)

Ala Ala Ser Ser Leu Gln Ser (SEQ ID NO: 50709)

Gln Gln Tyr Leu Ser Tyr Pro Ile Thr (SEQ ID NO: 50710)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | K_FR1 | | | | | | | | | | | | | | |
| CONSENSUS | D | I | Q | M | T | Q | S | P | S | S | L | S | A | S | V | G | D | R | V | T | I | T | C | R | A | S |
| 21-225_151C9_LC | . | . | . | . | . | . | . | . | . | . | . | . | . | . | . | . | . | . | . | . | . | . | . | . | . | . |
| 21-225_157H7_LC | . | . | . | . | . | . | . | . | . | . | . | . | . | . | . | . | . | . | . | . | . | . | . | . | . | . |
| 21-225_194B7_LC | . | . | . | . | . | . | . | . | . | . | . | . | . | . | . | . | . | . | . | . | . | . | . | . | . | . |
| 21-225_196D4_LC | . | . | . | . | . | . | . | . | . | . | . | . | . | . | . | . | . | . | . | . | . | . | . | . | . | . |
| 21-225_20A8_LC | . | . | . | . | . | . | . | . | . | . | . | . | . | . | . | . | . | . | . | . | . | . | . | . | . | . |
| 21-225_2C12_LC | . | . | . | . | . | . | . | . | . | . | . | . | . | . | . | . | . | . | . | . | . | . | . | . | . | . |
| 21-225_33B7_LC | . | . | . | . | . | . | . | . | . | . | . | . | . | . | . | . | . | . | . | . | . | . | . | . | . | . |
| 21-225_33H3_LC | . | . | . | . | . | . | . | . | . | . | . | . | . | . | . | . | . | . | . | . | . | . | . | Q | . | . |
| 21-225_87B7_LC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | K_CDR1 | | | | | | | | | | | | | | | | | K_FR2 | | |
| CONSENSUS | - | - | Q | G | I | S | - | - | - | - | - | - | N | Y | L | A | W | F | Q | Q | K | P | G | K | A | P |
| 21-225_151C9_LC | . | . | . | . | . | . | . | . | . | . | . | . | K | . | . | . | . | . | . | . | . | . | . | . | . | . |
| 21-225_157H7_LC | . | . | . | D | . | . | . | . | . | . | . | . | . | F | . | . | . | . | . | . | . | . | . | . | . | . |
| 21-225_194B7_LC | . | . | . | . | . | . | . | . | . | . | . | . | K | . | . | . | . | . | . | . | . | . | . | . | . | . |
| 21-225_196D4_LC | . | . | . | . | . | . | . | . | . | . | . | . | . | . | . | . | . | . | . | . | N | . | . | . | . | . |
| 21-225_20A8_LC | . | . | . | . | . | N | . | . | . | . | . | . | . | . | . | V | . | . | . | . | . | . | . | . | . | . |
| 21-225_2C12_LC | . | . | . | . | . | . | . | . | . | . | . | . | . | . | . | . | . | . | . | . | . | S | . | . | . | . |
| 21-225_33B7_LC | . | . | . | D | . | . | . | . | . | . | . | . | . | . | . | . | . | . | . | . | . | . | . | . | . | . |
| 21-225_33H3_LC | . | . | . | D | . | . | . | . | . | . | . | . | . | F | . | N | . | . | R | . | . | . | . | . | . | . |
| 21-225_87B7_LC | . | . | . | . | . | . | . | . | . | . | . | . | . | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | | | | | | | | K_CDR2 | | | | | | | | | | | | | |
| CONSENSUS | K | S | L | I | Y | A | - | - | - | - | - | - | - | - | A | S | S | L | Q | S | G | V | P | S | K | F |
| 21-225_151C9_LC | . | . | . | . | . | D | . | . | . | . | . | . | . | . | . | . | R | . | . | . | . | . | . | . | . | . |
| 21-225_157H7_LC | . | . | . | V | S | T | . | . | . | . | . | . | . | . | . | . | . | . | . | . | . | . | . | . | . | . |
| 21-225_194B7_LC | . | . | . | . | . | G | . | . | . | . | . | . | . | . | . | . | R | . | . | . | . | . | . | . | . | . |
| 21-225_196D4_LC | . | . | . | . | . | . | . | . | . | . | . | . | . | . | . | . | . | . | . | . | . | . | . | . | . | . |
| 21-225_20A8_LC | . | . | . | . | . | . | . | . | . | . | . | . | . | . | . | . | . | . | L | . | . | . | . | . | . | . |
| 21-225_2C12_LC | . | . | . | . | S | . | . | . | . | . | . | . | . | . | . | T | . | . | . | . | . | . | . | . | Q | . |
| 21-225_33B7_LC | T | . | . | . | . | . | . | . | . | . | . | . | . | . | . | . | . | . | . | . | . | . | . | . | . | . |
| 21-225_33H3_LC | N | . | . | . | . | D | . | . | . | . | . | . | . | . | . | . | N | . | V | T | . | . | . | . | R | . |
| 21-225_87B7_LC | . | . | . | . | . | . | . | . | . | . | . | . | . | . | . | . | . | . | H | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | | | | | | | | | | | K_FR3 | | | | | | | | | | | | | | | |
| CONSENSUS | S | G | S | G | S | G | - | - | T | D | F | T | L | T | I | S | S | L | Q | P | E | D | F | A | T | Y |
| 21-225_151C9_LC | . | . | . | . | . | . | . | . | . | . | . | . | . | . | . | . | . | . | . | . | . | . | . | . | S | . |
| 21-225_157H7_LC | . | . | . | . | . | . | . | . | . | . | . | . | . | . | . | . | . | . | . | . | . | . | . | . | . | . |
| 21-225_194B7_LC | . | . | . | . | . | . | . | . | . | . | . | . | . | . | . | . | . | . | . | . | . | . | . | . | . | . |
| 21-225_196D4_LC | . | . | . | . | C | . | . | . | . | . | . | . | . | . | . | . | . | P | . | . | . | . | V | . | . | . |
| 21-225_20A8_LC | . | . | . | . | . | . | . | . | . | . | . | . | . | . | . | . | . | . | . | . | . | . | . | . | . | . |
| 21-225_2C12_LC | . | . | . | . | . | . | . | . | . | . | . | . | . | . | . | . | . | . | . | . | . | . | . | . | . | . |
| 21-225_33B7_LC | . | . | . | . | . | . | . | . | . | . | . | . | . | . | . | . | . | . | . | . | . | . | . | . | . | F |
| 21-225_33H3_LC | . | . | R | . | . | . | . | . | . | . | . | . | F | . | . | . | . | . | . | . | . | . | . | . | . | . |
| 21-225_87B7_LC | . | . | . | . | . | . | . | . | . | . | . | . | . | . | . | . | . | . | . | . | . | . | S | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | Q | Q | Y | L | S | - | - | - | - | - | - | - | - | - | - | - | - | - | - | - | - | - | - | - |
| 21-225_151C9_LC | . | . | H | . | . | K | H | . | . | . | . | . | . | . | . | . | . | . | . | . | . | . | . | . | . | . |
| 21-225_157H7_LC | . | . | . | . | . | H | . | . | . | . | . | . | . | . | . | . | . | . | . | . | . | . | . | . | . | . |
| 21-225_194B7_LC | . | . | . | H | . | . | N | . | . | . | . | . | . | . | . | . | . | . | . | . | . | . | . | . | . | . |
| 21-225_196D4_LC | . | . | . | H | . | . | N | . | . | . | . | . | . | . | . | . | . | . | . | . | . | . | . | . | . | . |
| 21-225_20A8_LC | . | . | . | . | . | N | . | . | . | . | . | . | . | . | . | . | . | . | . | . | . | . | . | . | . | . |
| 21-225_2C12_LC | . | . | . | . | . | Y | . | . | . | . | . | . | . | . | . | . | . | . | . | . | . | . | . | . | . | . |
| 21-225_33B7_LC | . | . | . | . | . | H | . | . | . | . | . | . | . | . | . | . | . | . | . | . | . | . | . | . | . | . |
| 21-225_33H3_LC | . | . | . | . | . | D | N | . | . | . | . | . | . | . | . | . | . | . | . | . | . | . | . | . | . | . |
| 21-225_87B7_LC | F | . | L | L | . | . | T | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | Y | P | I | T | F | G | Q | G | T | R | L | E | I | K | R |
| 21-225_151C9_LC | . | . | . | . | . | . | . | . | . | . | . | . | . | . | . | . | . | . | . |
| 21-225_157H7_LC | . | . | . | . | . | . | . | . | . | . | . | . | . | . | . | . | . | . | . |
| 21-225_194B7_LC | . | . | . | . | . | . | L | . | . | . | . | . | . | . | . | . | . | . | . |
| 21-225_196D4_LC | . | . | . | . | . | . | L | . | . | . | . | . | . | . | . | . | . | . | . |
| 21-225_20A8_LC | . | . | . | . | . | . | . | . | . | . | . | . | . | . | . | . | . | . | . |
| 21-225_2C12_LC | . | . | . | . | . | . | . | . | . | . | . | . | . | . | . | . | . | . | . |
| 21-225_33B7_LC | . | . | . | . | . | . | L | . | . | . | . | . | . | . | . | . | . | . | . |
| 21-225_33H3_LC | . | . | . | . | L | . | . | . | . | . | . | . | . | . | . | . | . | . | . |
| 21-225_87B7_LC | . | . | . | . | . | . | L | . | . | . | . | . | . | . | . | . | . | . | . |

**FIGURE 57**

Table 130. Consensus 50 - VK6|A26/JK4 (SEQ ID NO: 50347):

EIVLTQSPDFQSVTPKEKVTITCRAS--QSIG------SSLHWYQQKPDQSPKLLIKY--------ASQSFSGVPSRFSGSGSG--TDFTLTINSLEAEDAATYYCHQSRR-----------------------LPLTFGGGTKVEIKR wherein:

S at position 26 can be substituted with N

S at position 30 can be substituted with N

S at position 39 can be substituted with R

F at position 71 can be substituted with L

S at position 109 can be substituted with T

R at position 110 can be substituted with G or S

R at position 111 can be substituted with S or T

Xaa Ala Xaa Gln Ser Xaa Gly Ser Ser Leu His (SEQ ID NO: 50215)

Tyr Ala Ser Gln Ser Xaa Ser (SEQ ID NO: 50216)

His Gln Xaa Xaa Xaa Leu Pro Leu Thr (SEQ ID NO: 50217)

Arg Ala Ser Gln Ser Ile Gly Ser Ser Leu His (SEQ ID NO: 50711)

Tyr Ala Ser Gln Ser Phe Ser (SEQ ID NO: 50712)

His Gln Ser Arg Arg Leu Pro Leu Thr (SEQ ID NO: 50713)

**FIGURE 57**

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | E | I | V | L | T | Q | S | P | D | F | Q | S | V | T | P | K | E | K | V | T | I | T | C | R | A | S |
| 21-225_190B12_LC | . | . | . | . | . | . | . | . | . | . | . | . | . | . | . | . | . | . | . | . | . | . | . | . | . | . |
| 21-225_191C9_LC | . | . | . | . | . | . | . | . | . | . | . | . | . | . | . | . | . | . | . | . | . | . | . | . | . | . |
| 21-225_191H4_LC | . | . | . | . | . | . | . | . | . | . | . | . | . | . | . | . | . | . | . | . | . | . | . | . | . | N |
| 21-225_192E5_LC | . | . | . | . | . | . | . | . | . | . | . | . | . | . | . | . | . | . | . | . | . | . | . | . | . | . |
| 21-225_193G9_LC | . | . | . | . | . | . | . | . | . | . | . | . | . | . | . | . | . | . | . | . | . | . | . | . | . | . |
| 21-225_195B10_LC | . | . | . | . | . | . | . | . | . | . | . | . | . | . | . | . | . | . | . | . | . | . | . | . | . | . |
| 21-225_195F2_LC | . | . | . | . | . | . | . | . | . | . | . | . | . | . | . | . | . | . | . | . | . | . | . | . | . | . |
| 21-225_201E8_LC | . | . | . | . | . | . | . | . | A | . | . | . | . | . | . | . | . | . | . | . | . | . | . | . | . | . |
| 21-225_206G4_LC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | I | G | - | - | - | - | - | - | S | S | L | H | W | Y | Q | Q | K | P | D | Q | S | P |
| 21-225_190B12_LC | . | . | . | . | . | . | . | . | . | . | . | . | . | . | . | . | . | . | . | . | . | . | . | . | . | . |
| 21-225_191C9_LC | . | . | . | . | . | . | . | . | . | . | . | . | . | . | . | . | . | . | . | . | . | . | . | . | . | . |
| 21-225_191H4_LC | . | . | . | . | . | . | . | . | . | . | . | . | . | . | . | . | . | . | . | . | . | . | . | . | . | . |
| 21-225_192E5_LC | . | . | . | . | . | . | . | . | . | . | . | . | R | . | . | . | . | . | . | . | . | . | . | . | . | . |
| 21-225_193G9_LC | . | . | . | . | . | . | . | . | . | . | . | . | R | . | . | . | . | . | . | . | R | . | . | . | . | L |
| 21-225_195B10_LC | . | . | . | . | . | . | . | . | . | . | . | . | . | . | . | . | . | . | . | . | . | . | . | . | . | . |
| 21-225_195F2_LC | . | . | . | . | . | . | . | . | . | . | . | . | . | . | . | . | . | . | . | . | . | . | . | . | . | . |
| 21-225_201E8_LC | . | . | . | N | . | . | . | . | . | . | . | . | R | . | . | . | . | . | . | . | . | . | . | . | . | . |
| 21-225_206G4_LC | . | . | . | . | . | . | . | . | . | . | . | . | R | . | . | . | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | K | L | L | I | K | Y | - | - | - | - | - | - | - | - | A | S | Q | S | F | S | G | V | P | S | R | F |
| 21-225_190B12_LC | . | . | . | . | . | . | . | . | . | . | . | . | . | . | . | . | . | . | . | . | . | D | . | . | . | . |
| 21-225_191C9_LC | . | . | . | . | . | . | . | . | . | . | . | . | . | . | . | . | . | . | . | . | . | D | . | . | . | . |
| 21-225_191H4_LC | . | . | . | . | . | . | . | . | . | . | . | . | . | . | . | . | . | . | L | . | . | . | . | . | . | . |
| 21-225_192E5_LC | . | . | . | . | . | . | . | . | . | . | . | . | . | . | . | . | . | . | . | . | . | . | . | . | . | . |
| 21-225_193G9_LC | . | . | . | . | . | . | . | . | . | . | . | . | . | . | . | . | . | . | . | . | . | . | . | . | . | . |
| 21-225_195B10_LC | . | . | . | . | . | . | . | . | . | . | . | . | . | . | . | . | . | . | . | . | . | . | . | . | . | . |
| 21-225_195F2_LC | . | . | . | . | . | . | . | . | . | . | . | . | . | . | . | . | . | . | . | . | . | . | . | . | . | . |
| 21-225_201E8_LC | . | . | . | . | . | . | . | . | . | . | . | . | . | . | . | . | . | . | . | . | . | . | . | . | . | . |
| 21-225_206G4_LC | . | . | . | . | . | . | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | T | I | N | S | L | E | A | E | D | A | A | T | Y |
| 21-225_190B12_LC | . | . | . | . | . | . | . | . | . | . | . | . | . | . | . | . | . | . | . | . | . | . | . | . | . | . |
| 21-225_191C9_LC | . | . | . | . | . | . | . | . | . | . | . | . | . | . | . | . | . | . | . | . | . | . | . | . | . | . |
| 21-225_191H4_LC | . | A | . | . | . | . | . | . | . | . | . | . | . | . | . | . | . | . | D | . | . | . | . | . | . | . |
| 21-225_192E5_LC | . | . | . | . | . | . | . | . | . | . | . | . | . | . | . | . | . | . | . | . | . | . | . | . | . | . |
| 21-225_193G9_LC | . | . | . | . | . | . | . | . | . | . | . | . | . | . | . | . | . | . | . | . | . | . | . | . | . | . |
| 21-225_195B10_LC | . | . | . | . | . | . | . | . | . | . | . | . | . | . | . | . | . | . | . | . | . | . | . | . | . | . |
| 21-225_195F2_LC | . | . | . | . | . | . | . | . | . | . | . | A | . | . | . | S | . | . | . | . | . | . | . | . | . | . |
| 21-225_201E8_LC | . | . | . | . | . | . | . | . | . | N | . | . | . | . | . | . | . | . | . | . | . | . | . | V | . | . |
| 21-225_206G4_LC | . | . | . | . | . | . | . | . | . | . | . | . | . | . | . | . | . | . | . | . | . | . | . | . | . | . |

# FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | H | Q | S | R | | - | - | - | - | - | - | - | - | - | - | - | - | - | - | - | - | - | - | - |
| 21-225_190B12_LC | . | . | . | . | T | . | S | . | . | . | . | . | . | . | . | . | . | . | . | . | . | . | . | . | . | . |
| 21-225_191C9_LC | . | . | . | . | T | . | S | . | . | . | . | . | . | . | . | . | . | . | . | . | . | . | . | . | . | . |
| 21-225_191H4_LC | . | . | . | . | T | G | R | . | . | . | . | . | . | . | . | . | . | . | . | . | . | . | . | . | . | . |
| 21-225_192E5_LC | . | . | . | . | . | S | R | . | . | . | . | . | . | . | . | . | . | . | . | . | . | . | . | . | . | . |
| 21-225_193G9_LC | F | . | . | . | . | S | R | . | . | . | . | . | . | . | . | . | . | . | . | . | . | . | . | . | . | . |
| 21-225_195B10_LC | . | . | . | . | . | G | R | . | . | . | . | . | . | . | . | . | . | . | . | . | . | . | . | . | . | . |
| 21-225_195F2_LC | . | . | . | . | . | . | T | . | . | . | . | . | . | . | . | . | . | . | . | . | . | . | . | . | . | . |
| 21-225_201E8_LC | . | . | . | . | . | . | S | . | . | . | . | . | . | . | . | . | . | . | . | . | . | . | . | . | . | . |
| 21-225_206G4_LC | . | . | . | . | . | . | S | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | L | P | L | T | F | G | G | G | T | K | V | E | I | K | R |
| 21-225_190B12_LC | . | . | . | . | . | . | . | . | . | . | . | . | . | . | . | . | . | . | . |
| 21-225_191C9_LC | . | . | . | . | . | . | . | . | . | . | . | . | . | . | . | . | . | . | . |
| 21-225_191H4_LC | . | . | . | . | . | . | . | . | . | . | . | . | . | . | . | . | . | . | . |
| 21-225_192E5_LC | . | . | . | . | . | . | . | . | . | . | . | . | . | . | . | . | . | . | . |
| 21-225_193G9_LC | . | . | . | . | . | . | . | . | . | . | . | . | . | . | . | . | . | . | . |
| 21-225_195B10_LC | . | . | . | . | . | . | . | . | . | . | . | . | . | . | . | . | . | . | . |
| 21-225_195F2_LC | . | . | . | . | . | . | . | . | . | . | . | . | . | . | . | . | . | . | . |
| 21-225_201E8_LC | . | . | . | . | . | . | . | . | . | . | . | . | . | . | . | . | . | R | . |
| 21-225_206G4_LC | . | . | . | . | . | . | . | . | . | . | . | . | . | . | . | . | . | . | . |

**FIGURE 57**

Table 131. Consensus 51 - VL3|3l/JL2 (SEQ ID NO: 50348):

SSELTQD-PAVSVALGQTVRITCQGD---SLRP-----YYASWYQQKPGQAPVLVIYG--------KNNRPSGIPDRFSGSSSG--NTASLTITGAQAEDEADYYCNSRDSS--------------------GNHLVVFGGGTKLTVLG wherein:

S at position 30 can be substituted with T or K

P at position 33 can be substituted with N, S, or T

A at position 41 can be substituted with V

S at position 42 can be substituted with N

G at position 58 can be substituted with A or T

N at position 69 can be substituted with S

S at position 112 can be substituted with C

G at position 133 can be substituted with null (-)

N at position 134 can be substituted with G

H at position 135 can be substituted with N or S

L at position 136 can be substituted with H

V at position 137 can be substituted with L

V at position 138 can be substituted with L

Gln Gly Asp Xaa Leu Arg Xaa Tyr Tyr Xaa Xaa (SEQ ID NO: 50218)

Xaa Lys Asn Xaa Arg Pro Ser (SEQ ID NO: 50219)

Asn Ser Arg Asp Ser Xaa Xaa Xaa Xaa Xaa Xaa Xaa (SEQ ID NO: 50220)

# FIGURE 57

Gln Gly Asp Ser Leu Arg Pro Tyr Tyr Ala Ser (SEQ ID NO: 50714)

Gly Lys Asn Asn Arg Pro Ser (SEQ ID NO: 50715)

Asn Ser Arg Asp Ser Ser Gly Asn His Leu Val Val (SEQ ID NO: 50716)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | | | | | | | | | | | | L_FR1 | | | | | | | | | | | | | | |
| CONSENSUS | S | S | E | L | T | Q | D | - | P | A | V | S | V | A | L | G | Q | T | V | R | I | T | C | Q | G | D |
| 21-225_171D12_LC | . | . | . | . | . | . | . | . | . | . | . | . | . | . | . | . | . | . | . | . | . | . | . | . | . | . |
| 21-225_172B12_LC | . | . | . | . | . | . | . | . | . | . | . | . | . | . | . | . | . | . | . | . | . | . | . | . | . | . |
| 21-225_172C3_LC | . | . | . | . | . | . | . | . | . | . | . | . | . | . | . | . | . | . | . | . | . | . | . | . | . | . |
| 21-225_190B3_LC | . | . | . | . | . | . | . | . | . | . | . | . | . | . | . | . | . | . | . | . | . | . | . | . | . | . |
| 21-225_190B8_LC | . | . | . | . | . | . | . | . | . | . | . | . | . | . | . | . | . | . | . | . | . | . | . | . | . | . |
| 21-225_190C10_LC | . | . | . | . | . | . | . | . | . | . | . | . | . | . | . | . | . | . | . | . | . | . | . | . | . | . |
| 21-225_191A9_LC | . | . | . | . | . | . | . | . | . | . | . | . | . | . | . | . | . | . | . | . | . | . | . | . | . | . |
| 21-225_191B8_LC | . | . | . | . | . | . | . | . | . | . | . | . | . | . | . | . | . | . | . | . | . | . | . | . | . | . |
| 21-225_193A6_LC | . | . | . | . | . | . | . | . | . | . | . | . | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | | | | | | | L_CDR1 | | | | | | | | | | | | | | | | | L_FR2 | | |
| CONSENSUS | - | - | - | S | L | R | P | - | - | - | - | - | Y | Y | A | S | W | Y | Q | Q | K | P | G | Q | A | P |
| 21-225_171D12_LC | . | . | . | . | . | . | S | . | . | . | . | . | . | . | . | . | . | . | . | . | . | . | . | . | . | . |
| 21-225_172B12_LC | . | . | . | . | . | . | N | . | . | . | . | . | . | . | . | . | . | . | . | . | . | . | . | . | . | . |
| 21-225_172C3_LC | . | . | . | . | . | . | N | . | . | . | . | . | . | . | V | . | . | . | . | . | . | . | . | . | . | . |
| 21-225_190B3_LC | . | . | . | K | . | . | T | . | . | . | . | . | . | . | . | . | . | . | . | . | . | . | . | . | . | . |
| 21-225_190B8_LC | . | . | . | T | . | . | . | . | . | . | . | . | . | . | V | . | . | . | . | . | . | . | . | . | . | . |
| 21-225_190C10_LC | . | . | . | . | . | . | . | . | . | . | . | . | . | . | . | . | . | . | . | . | . | . | . | . | . | . |
| 21-225_191A9_LC | . | . | . | . | . | . | . | . | . | . | . | . | . | . | . | . | . | . | . | . | . | . | . | . | . | . |
| 21-225_191B8_LC | . | . | . | T | . | . | . | . | . | . | . | . | . | . | . | . | . | . | . | . | . | . | . | . | . | . |
| 21-225_193A6_LC | . | . | . | . | . | . | S | . | . | . | . | . | . | . | . | N | . | . | . | . | . | . | . | . | . | . |

**FIGURE 57**

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | L_CDR2 | | | | | | | | | | | | | | | | | | | | |
| CONSENSUS | V | L | V | I | Y | G | - | - | - | - | - | - | - | - | K | N | N | R | P | S | G | I | P | D | R | F |
| 21-225_171D12_LC | I | . | . | . | . | A | . | . | . | . | . | . | . | . | . | . | . | . | . | . | . | . | . | . | . | . |
| 21-225_172B12_LC | I | . | . | . | . | T | . | . | . | . | . | . | . | . | . | . | S | . | . | . | . | . | . | . | . | . |
| 21-225_172C3_LC | I | . | . | . | . | T | . | . | . | . | . | . | . | . | . | . | S | . | . | . | . | . | . | . | . | . |
| 21-225_190B3_LC | . | . | . | V | . | . | . | . | . | . | . | . | . | . | . | . | . | . | . | . | . | . | . | . | . | . |
| 21-225_190B8_LC | . | . | . | . | . | . | . | . | . | . | . | . | . | . | . | . | . | . | . | . | . | . | . | . | . | . |
| 21-225_190C10_LC | . | . | . | . | . | . | . | . | . | . | . | . | . | . | . | . | . | . | . | . | . | . | . | . | . | . |
| 21-225_191A9_LC | . | . | . | . | . | . | . | . | . | . | . | . | . | . | . | . | . | . | . | . | . | . | . | . | . | . |
| 21-225_191B8_LC | . | . | . | . | . | . | . | . | . | . | . | . | . | . | . | . | . | . | . | . | . | . | S | . | . | . |
| 21-225_193A6_LC | . | . | F | . | . | A | . | . | . | . | . | . | . | . | . | . | . | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | L_FR3 | | | | | | | | | | | | | | | | | | | | | | | | | |
| CONSENSUS | S | G | S | S | S | G | - | - | N | T | A | S | L | T | I | T | G | A | Q | A | E | D | E | A | D | Y |
| 21-225_171D12_LC | . | . | . | N | . | . | . | . | . | . | . | . | . | . | . | . | . | . | . | . | . | . | . | . | . | . |
| 21-225_172B12_LC | . | . | . | T | . | . | . | . | . | . | . | . | . | . | . | . | . | . | . | . | . | . | . | . | . | . |
| 21-225_172C3_LC | . | . | . | T | . | . | . | . | . | . | . | . | . | . | . | . | . | T | . | . | . | . | . | . | . | . |
| 21-225_190B3_LC | . | . | . | . | . | . | . | . | . | . | . | . | . | . | . | . | . | . | . | . | . | . | . | . | . | . |
| 21-225_190B8_LC | . | . | . | . | . | . | . | . | . | . | . | . | . | . | . | . | . | . | . | . | . | . | . | . | . | . |
| 21-225_190C10_LC | . | . | . | . | . | . | . | . | . | . | . | . | . | . | . | . | E | . | . | . | . | . | . | . | . | . |
| 21-225_191A9_LC | . | . | . | . | . | . | . | . | . | . | . | . | . | . | . | . | E | . | . | . | . | . | . | . | . | . |
| 21-225_191B8_LC | . | . | . | . | . | . | . | . | . | . | . | . | . | . | . | . | . | . | . | . | . | . | . | . | . | . |
| 21-225_193A6_LC | . | . | . | N | . | . | . | . | . | . | . | . | . | . | . | . | . | . | . | . | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | L_CDR3 | | | | | | | | | | | | | | | | | | | | | | | |
| CONSENSUS | Y | C | N | S | R | D | S | S | - | - | - | - | - | - | - | - | - | - | - | - | - | - | - | - | - | - |
| 21-225_171D12_LC | . | . | . | . | . | . | . | . | . | . | . | . | . | . | . | . | . | . | . | . | . | . | . | . | . | . |
| 21-225_172B12_LC | . | . | . | . | . | . | . | . | . | . | . | . | . | . | . | . | . | . | . | . | . | . | . | . | . | . |
| 21-225_172C3_LC | . | . | . | . | . | . | . | . | . | . | . | . | . | . | . | . | . | . | . | . | . | . | . | . | . | . |
| 21-225_190B3_LC | . | . | . | . | . | . | . | . | . | . | . | . | . | . | . | . | . | . | . | . | . | . | . | . | . | . |
| 21-225_190B8_LC | . | . | . | . | . | . | . | . | . | . | . | . | . | . | . | . | . | . | . | . | . | . | . | . | . | . |
| 21-225_190C10_LC | . | . | . | . | . | . | . | . | . | . | . | . | . | . | . | . | . | . | . | . | . | . | . | . | . | . |
| 21-225_191A9_LC | . | . | . | . | . | . | . | G | . | . | . | . | . | . | . | . | . | . | . | . | . | . | . | . | . | . |
| 21-225_191B8_LC | . | . | . | . | . | . | . | . | . | . | . | . | . | . | . | . | . | . | . | . | . | . | . | . | . | . |
| 21-225_193A6_LC | . | . | . | . | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | L_FR4 | | | | | | | | | | |
| CONSENSUS | - | - | G | N | H | L | V | V | F | G | G | G | T | K | L | T | V | L | G |
| 21-225_171D12_LC | . | . | - | G | S | H | . | . | . | . | . | . | . | . | . | . | . | . | S |
| 21-225_172B12_LC | . | . | - | G | N | H | . | . | . | . | . | . | . | . | . | . | . | . | . |
| 21-225_172C3_LC | . | . | - | G | N | H | . | . | . | . | . | . | . | . | . | . | . | . | . |
| 21-225_190B3_LC | . | . | . | . | . | . | . | L | . | . | . | . | . | . | . | . | . | . | S |
| 21-225_190B8_LC | . | . | . | . | . | . | . | . | . | . | . | . | . | . | . | . | . | . | . |
| 21-225_190C10_LC | . | . | . | . | . | . | . | . | . | . | . | . | . | . | . | . | . | . | . |
| 21-225_191A9_LC | . | . | . | . | . | . | . | . | . | . | . | . | . | . | . | . | . | . | . |
| 21-225_191B8_LC | . | . | . | . | . | . | . | . | . | . | . | . | . | . | . | . | . | . | . |
| 21-225_193A6_LC | . | . | - | G | N | H | L | . | . | . | . | . | . | . | . | . | . | . | . |

**FIGURE 57**

Table 132. Consensus 52 - VK2|A17/JK4 (SEQ ID NO: 50349):

DVVMTQSPLSLPVTLGQPASISCRSS--QSLVYSD-GNTYLNWFQQRPGQSPRRLIYK--------VSNWDSGVPDRFSGSGSG--TDFTLKISRVEAEDVGVYYCMQGTH-----------------------WPLTFGGGTKVEIKR wherein:

S at position 26 can be substituted with G

Y at position 40 can be substituted with S

K at position 58 can be substituted with E

N at position 69 can be substituted with K

S at position 72 can be substituted with Y

T at position 110 can be substituted with I

H at position 111 can be substituted with null (-)

W at position 135 can be substituted with H

P at position 136 can be substituted with L, S or W

L at position 137 can be substituted with P

Arg Ser Xaa Gln Ser Leu Val Tyr Ser Asp Gly Asn Thr Xaa Leu Asn (SEQ ID NO: 50221)

Xaa Val Ser Xaa Trp Asp Xaa (SEQ ID NO: 50222)

Met Gln Gly Xaa Xaa Xaa Xaa Xaa Thr (SEQ ID NO: 50223)

Arg Ser Ser Gln Ser Leu Val Tyr Ser Asp Gly Asn Thr Tyr Leu Asn (SEQ ID NO: 50717)

Lys Val Ser Asn Trp Asp Ser (SEQ ID NO: 50718)

Met Gln Gly Thr His Trp Pro Leu Thr (SEQ ID NO: 50719)

FIGURE 57

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | D | V | V | M | T | Q | S | P | L | S | L | P | V | T | L | G | Q | P | A | S | I | S | C | R | S | S |
| 21-225_148C6_LC | . | . | . | . | . | . | . | . | . | . | . | . | . | . | . | . | . | . | . | . | . | . | . | . | . | . |
| 21-225_148H11_LC | . | . | . | . | . | . | . | . | . | . | . | . | . | . | . | . | . | . | . | . | . | . | . | . | . | . |
| 21-225_149F2_LC | . | . | . | . | . | . | . | . | . | . | . | . | . | . | . | . | . | . | . | . | . | . | . | . | . | . |
| 21-225_150F11_LC | . | . | . | . | S | . | Y | . | . | . | . | . | . | . | F | . | . | . | . | . | . | . | . | . | . | . |
| 21-225_177D2_LC | . | . | . | . | . | . | . | . | . | . | . | . | . | . | . | . | . | . | . | . | . | . | . | . | . | . |
| 21-225_32G4_LC | . | . | . | . | . | . | . | . | . | . | . | S | . | . | . | . | . | . | . | . | . | . | . | . | . | . |
| 21-225_43F11_LC1_LC | . | . | . | . | . | . | . | . | . | . | . | . | . | . | . | . | . | . | . | . | . | . | . | . | . | . |
| 21-225_72C4_LC | . | . | . | . | . | . | . | . | . | . | . | . | . | . | . | . | . | . | . | . | . | . | . | . | . | G |

FIGURE 57

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | L | V | Y | S | D | - | G | N | T | Y | L | N | W | F | Q | Q | R | P | G | Q | S | P |
| 21-225_148C6_LC | . | . | . | . | . | . | . | . | . | . | . | . | . | . | . | . | . | . | . | . | . | . | . | . | . | . |
| 21-225_148H11_LC | . | . | . | . | . | . | . | . | . | . | . | . | . | . | . | . | . | . | . | . | . | . | . | . | . | . |
| 21-225_149F2_LC | . | . | . | . | . | . | . | . | . | . | . | . | . | S | . | . | . | . | . | . | . | . | . | . | . | . |
| 21-225_150F11_LC | . | . | . | . | . | . | . | . | . | . | . | . | . | S | . | . | . | . | . | . | . | . | . | . | Y | . |
| 21-225_177D2_LC | . | . | . | . | . | . | . | . | . | . | . | . | . | . | . | . | . | . | . | . | . | . | . | . | . | . |
| 21-225_32G4_LC | . | . | . | . | . | . | . | . | . | . | . | . | . | . | . | . | . | . | . | . | . | . | . | . | . | . |
| 21-225_43F11_LC1_LC | . | . | . | . | . | . | . | . | . | . | . | . | . | . | . | . | . | . | . | . | . | . | . | . | . | . |
| 21-225_72C4_LC | . | . | . | . | . | . | . | . | . | . | . | . | . | . | . | . | . | . | . | . | . | . | . | . | . | . |

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | R | R | L | I | Y | K | - | - | - | - | - | - | - | - | V | S | N | W | D | S | G | V | P | D | R | F |
| 21-225_148C6_LC | . | . | . | . | . | . | . | . | . | . | . | . | . | . | . | . | . | . | . | . | . | . | . | . | . | . |
| 21-225_148H11_LC | . | . | . | . | . | . | . | . | . | . | . | . | . | . | . | . | . | . | . | . | . | . | . | . | . | . |
| 21-225_149F2_LC | . | . | . | . | . | . | . | . | . | . | . | . | . | . | . | . | . | . | . | . | . | . | . | V | . | . |
| 21-225_150F11_LC | . | . | . | . | . | . | . | . | . | . | . | . | . | . | . | . | . | . | . | Y | . | . | . | V | . | . |
| 21-225_177D2_LC | . | . | . | . | . | . | . | . | . | . | . | . | . | . | . | . | . | . | . | . | . | . | . | . | . | . |
| 21-225_32G4_LC | . | . | . | . | . | . | . | . | . | . | . | . | . | . | . | . | . | . | . | . | . | . | . | . | . | . |
| 21-225_43F11_LC1_LC | . | . | . | . | . | . | . | . | . | . | . | . | . | . | . | . | . | . | . | . | . | . | . | . | . | . |
| 21-225_72C4_LC | . | . | . | . | . | E | . | . | . | . | . | . | . | . | . | . | K | . | . | . | . | . | . | . | . | . |

**FIGURE 57**

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | D | F | T | L | K | I | S | R | V | E | A | E | D | V | G | V | Y |
| 21-225_148C6_LC | . | . | . | . | . | . | . | . | . | . | . | . | . | . | . | . | . | . | . | . | . | . | . | . | . | . |
| 21-225_148H11_LC | . | . | . | . | . | . | . | . | . | . | . | . | . | . | . | . | . | . | . | . | . | . | . | . | . | . |
| 21-225_149F2_LC | . | . | . | . | . | . | . | . | . | . | . | . | . | . | . | . | . | . | . | . | . | . | . | . | [illegible] | . |
| 21-225_150F11_LC | . | . | . | . | . | . | . | . | . | . | . | . | . | . | . | . | . | . | . | . | . | . | . | . | [illegible] | . |
| 21-225_177D2_LC | . | . | . | . | . | . | . | . | . | . | . | . | . | . | . | . | . | . | . | . | . | . | . | . | . | . |
| 21-225_32G4_LC | . | . | . | . | . | . | . | . | . | . | . | . | . | . | . | . | . | . | . | . | . | . | . | . | . | . |
| 21-225_43F11_LC1_LC | . | . | . | . | . | . | . | . | . | . | . | . | . | . | . | . | . | . | . | . | . | . | . | . | . | . |
| 21-225_72C4_LC | . | . | . | . | . | . | . | . | . | N | . | . | . | . | . | . | . | . | . | . | . | . | . | . | . | [illegible] |

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | K_CDR3 | | | | | | | | | | | | | | | | | | | | | | | |
| CONSENSUS | Y | C | M | Q | G | T | H | - | - | - | - | - | - | - | - | - | - | - | - | - | - | - | - | - | - | - |
| 21-225_148C6_LC | . | . | . | . | . | . | . | . | . | . | . | . | . | . | . | . | . | . | . | . | . | . | . | . | . | . |
| 21-225_148H11_LC | . | . | . | . | . | . | . | . | . | . | . | . | . | . | . | . | . | . | . | . | . | . | . | . | . | . |
| 21-225_149F2_LC | . | . | . | . | . | . | . | . | . | . | . | . | . | . | . | . | . | . | . | . | . | . | . | . | . | . |
| 21-225_150F11_LC | . | . | . | . | . | . | . | . | . | . | . | . | . | . | . | . | . | . | . | . | . | . | . | . | . | . |
| 21-225_177D2_LC | . | . | . | . | . | . | . | . | . | . | . | . | . | . | . | . | . | . | . | . | . | . | . | . | . | . |
| 21-225_32G4_LC | . | . | . | . | . | [illegible] | - | . | . | . | . | . | . | . | . | . | . | . | . | . | . | . | . | . | . | . |
| 21-225_43F11_LC1_LC | . | . | . | . | . | . | . | . | . | . | . | . | . | . | . | . | . | . | . | . | . | . | . | . | . | . |
| 21-225_72C4_LC | . | . | . | . | . | . | . | . | . | . | . | . | . | . | . | . | . | . | . | . | . | . | . | . | . | . |

**FIGURE 57**

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | K_FR4 | | | | | | | | | | |
| CONSENSUS | - | - | - | - | W | P | L | T | F | G | G | G | T | K | V | E | I | K | R |
| 21-225_148C6_LC | . | . | . | . | . | S | . | . | . | . | . | . | . | . | . | . | . | . | . |
| 21-225_148H11_LC | . | . | . | . | . | L | . | . | . | . | . | . | . | . | . | . | . | . | . |
| 21-225_149F2_LC | . | . | . | . | . | . | P | . | . | . | . | . | . | . | . | . | . | . | . |
| 21-225_150F11_LC | . | . | . | . | . | . | P | . | . | . | . | . | . | . | . | . | . | . | T |
| 21-225_177D2_LC | . | . | . | . | . | . | . | . | . | . | . | . | . | . | . | . | . | . | . |
| 21-225_32G4_LC | . | . | . | . | H | W | . | . | . | . | . | . | . | . | . | . | . | . | . |
| 21-225_43F11_LC1_LC | . | . | . | . | . | . | . | . | . | . | . | . | . | . | . | . | . | . | . |
| 21-225_72C4_LC | . | . | . | . | . | . | . | . | . | . | . | . | . | . | . | . | . | . | . |

**FIGURE 57**

Table 133. Consensus 53 - VK3|L2/JK4 (SEQ ID NO: 50350):

EIVMTQSPATLSVSPGERATLSCRAS--QSVS------RNLAWYQQKPGQAPRLLIYG--------ASTRATGIPARFSGSGSG--TEFTLTISSLQSEDFAVYYCQQYNN-----------------------WPLTFGGGTKVEIKR wherein:

A at position 25 can be substituted with P or T

V at position 31 can be substituted with F

S at position 32 can be substituted with R or W

R at position 39 can be substituted with I or S

N at position 40 can be substituted with D or S

L at position 41 can be substituted with V

G at position 58 can be substituted with D

S at position 68 can be substituted with A

T at position 69 can be substituted with I or A

N at position 110 can be substituted with Y

N at position 111 can be substituted with T or Y

null(-) at position 134 can be substituted with W

W at position 135 can be substituted with P

Arg Xaa Ser Gln Ser Xaa Xaa Xaa Xaa Xaa Ala (SEQ ID NO: 50224)

Xaa Ala Xaa Xaa Arg Ala Thr (SEQ ID NO: 50225)

Gln Gln Tyr Xaa Xaa Xaa Xaa Pro Leu Thr (SEQ ID NO: 50226)

Arg Ala Ser Gln Ser Val Ser Arg Asn Leu Ala (SEQ ID NO: 50720)

**FIGURE 57**

Gly Ala Ser Thr Arg Ala Thr (SEQ ID NO: 50721)

Gln Gln Tyr Asn Asn Trp Pro Leu Thr (SEQ ID NO: 50722)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | K_FR1 | | | | | | | | | | | | | | | | | | | | | | | | | |
| CONSENSUS | E | I | V | M | T | Q | S | P | A | T | L | S | V | S | P | G | E | R | A | T | L | S | C | R | A | S |
| 21-225_190E11_LC | . | . | . | . | . | . | . | . | . | . | . | . | . | . | . | . | . | . | . | . | . | . | . | . | . | . |
| 21-225_191E8_LC | . | . | . | . | . | . | . | . | . | . | . | . | . | . | . | . | . | . | . | . | . | . | . | . | P | . |
| 21-225_191G11_LC | . | . | . | . | . | . | . | . | . | . | . | . | . | . | . | . | . | . | . | N | . | . | . | . | T | . |
| 21-225_199A6_LC | D | . | . | . | . | . | . | . | . | . | . | . | . | . | . | . | D | . | . | . | . | . | . | . | P | . |
| 21-225_55E1_LC | . | . | . | . | . | . | . | . | . | . | . | . | . | . | . | . | . | . | . | . | . | . | . | . | . | . |
| 21-225_65C12_LC | . | . | . | . | . | . | . | . | . | . | . | . | . | F | . | . | . | G | . | . | . | . | . | . | . | . |
| 21-225_74D1_LC | . | . | . | . | . | . | . | . | . | . | . | . | . | . | . | . | . | . | . | . | . | . | . | . | . | . |
| 21-225_75F11_LC | . | . | . | . | . | . | . | . | . | . | . | F | . | . | . | . | . | . | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | K_CDR1 | | | | | | | | | | | | | | | | K_FR2 | | | | | | | | | |
| CONSENSUS | - | - | Q | S | V | S | - | - | - | - | - | - | R | N | L | A | W | Y | Q | Q | K | P | G | Q | A | P |
| 21-225_190E11_LC | . | . | . | . | F | R | . | . | . | . | . | . | I | . | . | . | . | . | . | . | R | . | . | . | . | . |
| 21-225_191E8_LC | . | . | . | . | F | . | . | . | . | . | . | . | . | . | . | . | . | . | . | . | . | . | . | . | . | . |
| 21-225_191G11_LC | . | . | . | . | . | W | . | . | . | . | . | . | I | S | V | . | . | . | H | . | . | . | . | . | . | . |
| 21-225_199A6_LC | . | . | . | . | F | . | . | . | . | . | . | . | . | . | . | . | . | . | . | . | . | . | . | . | . | . |
| 21-225_55E1_LC | . | . | . | . | . | . | . | . | . | . | . | . | S | D | . | . | . | . | . | L | . | . | . | . | . | . |
| 21-225_65C12_LC | . | . | . | . | . | . | . | . | . | . | . | . | I | . | . | . | . | . | . | . | . | . | . | . | . | . |
| 21-225_74D1_LC | . | . | . | . | . | . | . | . | . | . | . | . | . | . | . | . | . | F | . | . | . | . | . | . | . | . |
| 21-225_75F11_LC | . | . | . | . | . | . | . | . | . | . | . | . | . | . | . | . | . | F | . | . | . | . | . | . | . | . |

FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | K_CDR2 | | | | | | | | | | | | | | | | | | | | |
| CONSENSUS | R | L | L | I | Y | G | - | - | - | - | - | - | - | - | A | S | T | R | A | T | G | I | P | A | R | F |
| 21-225_190E11_LC | . | . | . | . | . | . | . | . | . | . | . | . | . | . | . | . | . | . | . | . | . | . | . | . | . | . |
| 21-225_191E8_LC | . | . | . | . | . | . | . | . | . | . | . | . | . | . | . | . | . | . | . | . | . | . | . | S | . | . |
| 21-225_191G11_LC | . | . | . | . | . | . | . | . | . | . | . | . | . | . | . | A | . | . | . | . | . | . | . | . | . | . |
| 21-225_199A6_LC | . | . | . | . | . | . | . | . | . | . | . | . | . | . | . | . | . | . | . | . | . | . | . | . | . | . |
| 21-225_55E1_LC | . | . | . | . | . | D | . | . | . | . | . | . | . | . | . | . | A | . | . | . | . | . | . | . | . | . |
| 21-225_65C12_LC | . | . | . | . | . | . | . | . | . | . | . | . | . | . | . | . | . | . | . | . | . | . | . | . | . | . |
| 21-225_74D1_LC | . | . | . | . | . | . | . | . | . | . | . | . | . | . | . | . | I | . | . | . | . | . | . | . | . | . |
| 21-225_75F11_LC | . | . | . | F | . | . | . | . | . | . | . | . | . | . | . | . | I | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | K_FR3 | | | | | | | | | | | | | | | | | | | | | | | | | |
| CONSENSUS | S | G | S | G | S | G | - | - | T | E | F | T | L | T | I | S | S | L | Q | S | E | D | F | A | V | Y |
| 21-225_190E11_LC | . | . | . | . | . | . | . | . | . | . | . | . | . | . | . | . | . | . | . | . | . | . | . | . | . | . |
| 21-225_191E8_LC | . | . | . | . | . | . | . | . | . | . | . | . | . | . | . | . | . | . | E | . | . | . | . | . | . | . |
| 21-225_191G11_LC | . | . | . | . | . | . | . | . | . | . | . | . | . | . | . | . | . | . | . | . | . | . | . | . | . | . |
| 21-225_199A6_LC | . | . | . | . | . | . | . | . | . | . | . | . | . | . | . | . | . | . | E | C | . | . | . | . | . | . |
| 21-225_55E1_LC | . | . | . | . | . | . | . | . | . | . | . | . | . | . | . | . | . | . | . | . | . | . | . | . | F | . |
| 21-225_65C12_LC | N | A | . | . | . | . | . | . | . | . | . | . | . | S | . | . | . | . | . | . | . | N | . | . | . | . |
| 21-225_74D1_LC | . | . | . | . | . | . | . | . | . | . | . | . | . | . | . | Y | . | . | . | . | . | . | . | . | . | . |
| 21-225_75F11_LC | . | . | . | . | . | . | . | . | . | . | . | . | . | . | . | Y | . | . | . | Y | . | . | . | . | . | . |

FIGURE 57

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | | | | | | | | | | | | | | | | K_CDR3 | | | | | | | | |
| CONSENSUS | Y | C | Q | Q | Y | N | N | - | - | - | - | - | - | - | - | - | - | - | - | - | - | - | - | - | - | - |
| 21-225_190E11_LC | . | . | . | . | . | . | . | . | . | . | . | . | . | . | . | . | . | . | . | . | . | . | . | . | . | . |
| 21-225_191E8_LC | . | . | . | . | . | . | . | . | . | . | . | . | . | . | . | . | . | . | . | . | . | . | . | . | . | . |
| 21-225_191G11_LC | . | . | . | . | . | . | Y | . | . | . | . | . | . | . | . | . | . | . | . | . | . | . | . | . | . | . |
| 21-225_199A6_LC | . | . | . | . | . | . | . | . | . | . | . | . | . | . | . | . | . | . | . | . | . | . | . | . | . | . |
| 21-225_55E1_LC | . | . | . | . | . | Y | . | . | . | . | . | . | . | . | . | . | . | . | . | . | . | . | . | . | . | . |
| 21-225_65C12_LC | . | . | . | . | . | . | T | . | . | . | . | . | . | . | . | . | . | . | . | . | . | . | . | . | . | . |
| 21-225_74D1_LC | . | . | . | . | . | . | . | . | . | . | . | . | . | . | . | . | . | . | . | . | . | . | . | . | . | . |
| 21-225_75F11_LC | . | . | . | . | . | . | . | . | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | | | | | | K_FR4 | | | | | |
| CONSENSUS | - | - | - | - | W | P | L | T | F | G | G | G | T | K | V | E | I | K | R |
| 21-225_190E11_LC | . | . | . | . | . | . | . | . | . | . | . | . | . | . | . | . | . | . | . |
| 21-225_191E8_LC | . | . | . | . | . | . | . | . | . | . | . | . | I | . | . | . | . | . | . |
| 21-225_191G11_LC | . | . | . | . | . | . | . | . | . | . | . | . | . | . | . | . | . | . | . |
| 21-225_199A6_LC | . | . | . | . | . | . | . | . | . | . | . | . | . | . | . | . | . | . | . |
| 21-225_55E1_LC | . | . | . | . | . | . | . | . | . | . | . | . | . | . | . | . | . | . | . |
| 21-225_65C12_LC | . | . | . | . | . | . | . | . | . | . | . | . | . | . | . | . | . | . | . |
| 21-225_74D1_LC | . | . | . | W | P | . | . | . | . | . | . | . | . | . | . | . | . | . | . |
| 21-225_75F11_LC | . | . | . | W | P | . | . | . | . | . | . | . | . | . | . | . | . | . | . |

**FIGURE 57**

Table 134. Consensus 54 - VL3|3j/JL2 (SEQ ID NO: 50351):

SYELTQP-LSVSVALGQTARITCGGN---NIGR-----KNVHWYQQKPGQAPVLVIYR--------DSDRPSGIPERFSGSNSG--NTATLTISRAQAGDEADYYCQVWDS-----------------------STVVFGGGTKLTVLG wherein:

N at position 26 can be substituted with D

G at position 32 can be substituted with R

R at position 33 can be substituted with S

K at position 39 can be substituted with R

N at position 40 can be substituted with A

R at position 58 can be substituted with S

S at position 68 can be substituted with R

D at position 69 can be substituted with N or Y

P at position 71 can be substituted with S

V at position 108 can be substituted with D

null (-) at position 112 can be substituted with S

null (-) at position 134 can be substituted with S

S at position 135 can be substituted with D or G

T at position 136 can be substituted with H

V at position 137 can be substituted with A or G

Gly Gly Xaa Asn Ile Xaa Xaa Xaa Xaa Val His (SEQ ID NO: 50250)

Xaa Asp Xaa Xaa Arg Xaa Ser (SEQ ID NO: 50251)

# FIGURE 57

Gln Xaa Trp Asp Ser Xaa Xaa Xaa Xaa Xaa Val (SEQ ID NO: 50252)

Gly Gly Asn Asn Ile Gly Arg Lys Asn Val His (SEQ ID NO: 50723)

Arg Asp Ser Asp Arg Pro Ser (SEQ ID NO: 50724)

Gln Val Trp Asp Ser Ser Thr Val Val (SEQ ID NO: 50725)

| Reference # | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|---|---|---|---|---|---|---|
| | L_FR1 | | | | | | | | | | | | | | | | | | | | | | | | | |
| CONSENSUS | S | Y | E | L | T | Q | P | - | L | S | V | S | V | A | L | G | Q | T | A | R | I | T | C | G | G | N |
| 21-225_203B2_LC | . | . | . | . | . | . | . | . | . | . | . | . | . | . | . | . | . | . | . | . | . | . | . | . | . | . |
| 21-225_205E12_LC | . | . | . | . | . | . | . | . | . | . | . | . | A | . | . | . | . | . | . | . | . | . | . | . | . | . |
| 21-225_206B5_LC | . | . | . | . | . | . | . | . | . | . | . | . | . | . | . | . | . | . | . | . | . | A | . | . | . | . |
| 21-225_209H10_LC | . | . | . | . | . | . | . | . | . | . | . | . | . | . | . | . | . | . | . | . | . | A | . | . | . | . |
| 21-225_211H2_LC | . | . | . | . | . | . | . | . | . | . | . | . | . | . | . | . | . | . | . | . | . | . | . | . | . | . |
| 21-225_216B12_LC | . | . | . | . | . | . | . | . | . | . | . | . | . | . | . | . | . | . | . | . | . | . | . | . | . | D |
| 21-225_3E10_LC | . | . | . | . | . | . | . | . | H | . | . | . | . | . | T | A | . | M | . | . | . | . | . | . | . | . |
| 21-225_8C12_LC | . | . | . | . | . | . | . | . | H | . | . | . | . | . | T | A | . | M | . | . | . | . | . | . | . | . |

| Reference # | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 |
|---|---|---|---|---|---|---|---|---|---|---|---|---|---|---|---|---|---|---|---|---|---|---|---|---|---|---|
| | L_CDR1 | | | | | | | | | | | | | | | | L_FR2 | | | | | | | | | |
| CONSENSUS | - | - | - | N | I | G | R | - | - | - | - | - | K | N | V | H | W | Y | Q | Q | K | P | G | Q | A | P |
| 21-225_203B2_LC | . | . | . | . | . | . | . | . | . | . | . | . | . | . | . | . | . | . | . | . | . | . | . | . | S | . |
| 21-225_205E12_LC | . | . | . | . | . | . | . | . | . | . | . | . | . | . | . | . | . | . | . | . | . | . | . | . | S | . |
| 21-225_206B5_LC | . | . | . | . | . | . | . | . | . | . | . | . | . | . | . | . | . | . | . | . | . | . | . | L | . | . |
| 21-225_209H10_LC | . | . | . | . | . | . | . | . | . | . | . | . | . | . | . | . | . | . | . | . | . | . | . | L | . | . |
| 21-225_211H2_LC | . | . | . | . | . | . | . | . | . | . | . | . | R | . | . | . | . | . | . | . | . | . | . | . | . | . |
| 21-225_216B12_LC | . | . | . | . | . | R | . | . | . | . | . | . | R | . | . | . | . | . | . | . | . | . | . | . | . | . |
| 21-225_3E10_LC | . | . | . | . | . | . | S | . | . | . | . | . | . | A | . | . | . | . | . | . | . | . | . | . | D | . |
| 21-225_8C12_LC | . | . | . | . | . | . | S | . | . | . | . | . | . | A | . | . | . | . | . | . | . | . | . | . | D | . |

# FIGURE 57

| Reference # | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 |
|---|---|---|---|---|---|---|---|---|---|---|---|---|---|---|---|---|---|---|---|---|---|---|---|---|---|---|
| | | | | | | L_CDR2 | | | | | | | | | | | | | | | | | | | | |
| CONSENSUS | V | L | V | I | Y | R | - | - | - | - | - | - | - | - | D | S | | R | P | S | G | I | P | E | R | F |
| 21-225_203B2_LC | . | . | I | . | H | . | . | . | . | . | . | . | . | . | . | . | D | . | . | . | . | . | . | . | . | . |
| 21-225_205E12_LC | . | . | I | . | H | . | . | . | . | . | . | . | . | . | . | . | D | . | . | . | . | . | . | . | . | . |
| 21-225_206B5_LC | . | P | . | . | X | . | . | . | . | . | . | . | . | . | . | . | Y | . | S | . | . | . | . | D | . | . |
| 21-225_209H10_LC | . | P | . | . | L | . | . | . | . | . | . | . | . | . | . | . | Y | . | S | . | . | . | . | D | . | . |
| 21-225_211H2_LC | I | . | . | . | . | . | . | . | . | . | . | . | . | . | . | R | D | . | . | . | . | . | . | . | . | . |
| 21-225_216B12_LC | . | . | . | . | . | . | . | . | . | . | . | . | . | . | . | . | N | . | . | . | . | . | . | . | . | . |
| 21-225_3E10_LC | . | . | . | . | . | S | . | . | . | . | . | . | . | . | . | . | N | . | . | . | . | . | . | . | . | . |
| 21-225_8C12_LC | . | . | . | . | . | S | . | . | . | . | . | . | . | . | . | . | N | . | . | . | . | . | . | . | . | . |

| Reference # | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 |
|---|---|---|---|---|---|---|---|---|---|---|---|---|---|---|---|---|---|---|---|---|---|---|---|---|---|---|
| | L_FR3 | | | | | | | | | | | | | | | | | | | | | | | | | |
| CONSENSUS | S | G | S | N | S | G | - | - | N | T | A | T | L | T | I | S | R | A | Q | A | G | D | E | A | D | Y |
| 21-225_203B2_LC | . | . | . | . | . | . | . | . | . | . | . | . | . | . | . | . | . | . | . | . | . | . | . | . | . | . |
| 21-225_205E12_LC | . | . | . | . | . | . | . | . | . | . | . | . | . | . | . | . | . | . | . | . | . | . | . | . | . | . |
| 21-225_206B5_LC | . | . | . | . | C | . | . | . | . | . | T | . | V | . | . | . | . | . | . | . | . | E | . | . | E | . |
| 21-225_209H10_LC | . | . | . | . | W | . | . | . | . | . | . | . | V | . | . | . | . | . | . | . | . | E | . | . | E | . |
| 21-225_211H2_LC | . | . | . | . | . | . | . | . | . | . | . | . | . | . | . | . | . | . | . | . | . | . | . | . | . | . |
| 21-225_216B12_LC | . | . | . | . | . | . | . | . | . | . | . | . | . | . | . | . | . | . | . | . | . | . | . | . | . | . |
| 21-225_3E10_LC | . | . | . | . | P | . | . | . | . | . | . | . | . | . | . | . | . | I | E | . | . | . | . | . | . | . |
| 21-225_8C12_LC | . | . | . | . | P | . | . | . | . | . | . | . | . | . | . | . | . | I | E | . | . | . | . | . | . | . |

**FIGURE 57**

| Reference # | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 | 128 | 129 | 130 |
|---|---|---|---|---|---|---|---|---|---|---|---|---|---|---|---|---|---|---|---|---|---|---|---|---|---|---|
| | | | L_CDR3 | | | | | | | | | | | | | | | | | | | | | | | |
| CONSENSUS | Y | C | Q | V | W | D | S | - | - | - | - | - | - | - | - | - | - | - | - | - | - | - | - | - | - | - |
| 21-225_203B2_LC | . | . | . | . | . | . | . | . | . | . | . | . | . | . | . | . | . | . | . | . | . | . | . | . | . | . |
| 21-225_205E12_LC | . | . | . | . | . | . | . | . | . | . | . | . | . | . | . | . | . | . | . | . | . | . | . | . | . | . |
| 21-225_206B5_LC | . | . | . | D | . | . | . | . | . | . | . | . | . | . | . | . | . | . | . | . | . | . | . | . | . | . |
| 21-225_209H10_LC | . | . | . | D | . | . | . | . | . | . | . | . | . | . | . | . | . | . | . | . | . | . | . | . | . | . |
| 21-225_211H2_LC | F | . | . | . | . | . | . | . | . | . | . | . | . | . | . | . | . | . | . | . | . | . | . | . | . | . |
| 21-225_216B12_LC | . | . | . | . | . | . | . | . | . | . | . | . | . | . | . | . | . | . | . | . | . | . | . | . | . | . |
| 21-225_3E10_LC | . | . | . | . | . | . | . | S | . | . | . | . | . | . | . | . | . | . | . | . | . | . | . | . | . | . |
| 21-225_8C12_LC | . | . | . | . | . | . | . | S | . | . | . | . | . | . | . | . | . | . | . | . | . | . | . | . | . | . |

| Reference # | 131 | 132 | 133 | 134 | 135 | 136 | 137 | 138 | 139 | 140 | 141 | 142 | 143 | 144 | 145 | 146 | 147 | 148 | 149 |
|---|---|---|---|---|---|---|---|---|---|---|---|---|---|---|---|---|---|---|---|
| | | | | | | | | | L_FR4 | | | | | | | | | | |
| CONSENSUS | - | - | - | - | S | T | V | V | F | G | G | G | T | K | L | T | V | L | G |
| 21-225_203B2_LC | . | . | . | . | G | . | A | . | . | . | . | . | . | . | . | P | . | . | . |
| 21-225_205E12_LC | . | . | . | . | G | . | A | . | . | . | . | . | . | . | . | P | . | . | . |
| 21-225_206B5_LC | . | . | . | . | . | . | . | . | . | . | . | . | . | . | . | . | . | . | . |
| 21-225_209H10_LC | . | . | . | . | . | . | . | . | . | . | . | . | . | . | . | . | . | . | . |
| 21-225_211H2_LC | . | . | . | . | . | . | A | . | . | . | . | . | . | . | . | . | . | . | . |
| 21-225_216B12_LC | . | . | . | . | . | . | G | . | . | . | . | . | . | . | . | . | . | . | . |
| 21-225_3E10_LC | . | . | . | S | D | H | . | . | . | . | . | . | . | . | . | . | . | . | . |
| 21-225_8C12_LC | . | . | . | S | D | H | . | . | . | . | . | . | . | . | . | . | . | . | . |

Figure 58

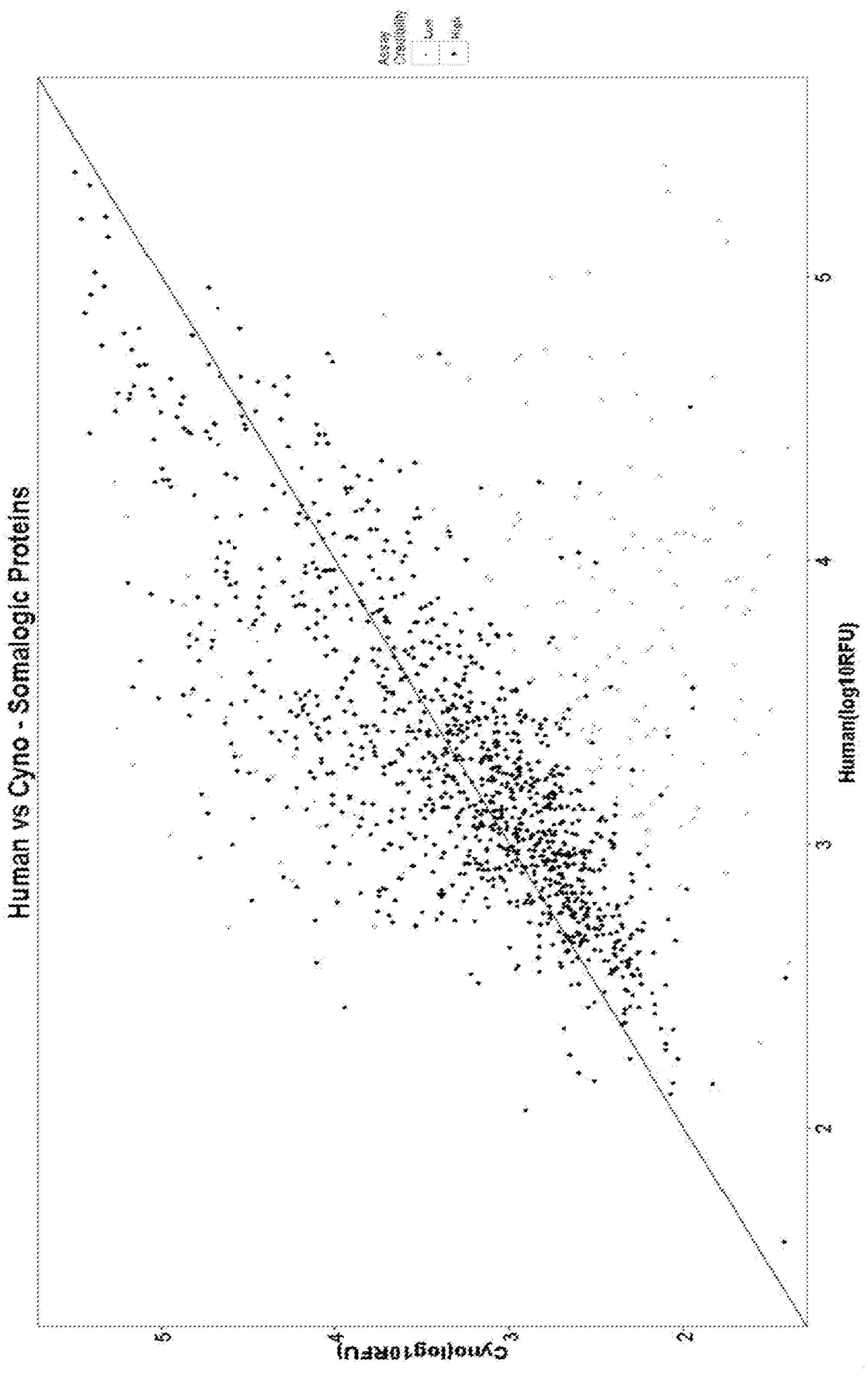
Human vs Cyno - Somalogic Proteins
Human(log10RFU)
Cyno(log10RFU)
2
3
4
5
2
3
4
5

Figure 59

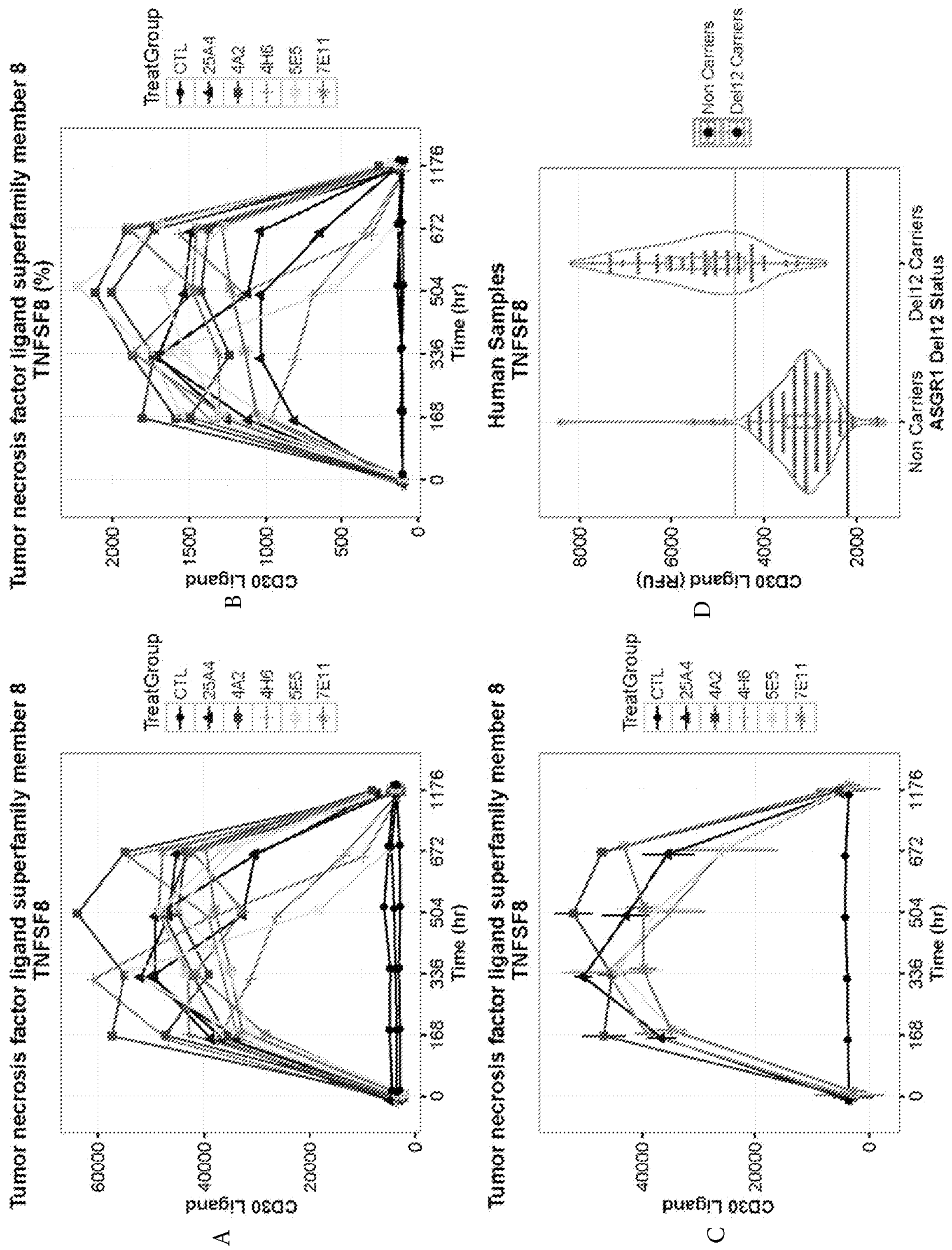
A
Tumor necrosis factor ligand superfamily member 8
TNFSF8
CD30 Ligand
Time (hr)
TreatGroup
CTL
25A4
4A2
4H6
5E5
7E11
B
Tumor necrosis factor ligand superfamily member 8
TNFSF8 (%)
CD30 Ligand
Time (hr)
C
Tumor necrosis factor ligand superfamily member 8
TNFSF8
CD30 Ligand
Time (hr)
D
Human Samples
TNFSF8
CD30 Ligand (RFU)
Non Carriers
Del12 Carriers
ASGR1 Del12 Status

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62319740
- US 62259553
- US 62234546
- US 8030457 B, Jackson
- WO 2011068798 A1
- US 6506559 B
- US 8394628 B
- US 7056704 B
- US 7078196 B
- US 6107094 A
- US 5898031 A
- US 6573099 B
- EP 1144623 A
- US 20150259689
- US 20150197746 A
- US 20110092565 A
- US 8877917 B
- US 8507455 B
- US 7579451 B
- US 5539082 A
- US 5714331 A
- US 5719262 A
- US 6693187 B
- US 9181551 B
- US 20160122761 A
- US 6194389 B
- US 6168587 B
- US 6472375 B
- US 6471996 B
- US 4522811 A
- US 4737456 A
- US 3720760 A, Bennich
- NL 5156
- NL 15
- NL 5537
- US 7192933 B
- US 6114598 A
- US 6162963 A
- US 6833268 B
- US 7049426 B
- US 7064244 B
- US 2012028596 W, Kannan, G


### Non-patent literature cited in the description


- **SAMBROOK et al.** Molecular Cloning: A Laboratory Manual. Cold Spring Harbor Laboratory Press, 1989
- **AUSUBEL et al.** Current Protocols in Molecular Biology. Greene Publishing Associates, 1992
- **HARLOW.** Lane Antibodies: A Laboratory Manual. Cold Spring Harbor Laboratory Press, 1990
- **GOEDDEL.** Gene Expression Technology: Methods in Enzymology. Academic Press, 1990, vol. 185
- **BARON et al.** *Nucleic Acids Res.,* 1995, vol. 23, 3605-06
- **GUDBJARTSSON et al.** *Nature Genetics,* May 2015, vol. 47 (5), 435-444
- **SABRAUTZKI et al.** *Mamm. Genome,* 2012, vol. 23, 416-430
- **GREWAL.** Methods in Enzymology. 2010, vol. 479, 223-241
- **ROGGENBUCK et al.** *Autoimmune Highlights,* 2012, vol. 3, 119-125
- **M. MEIER et al.** *JMB,* 2000, vol. 300, 857-865
- **LIU J ; HU B ; YANG Y et al.** A new splice variant of the major subunit of human asialoglycoprotein receptor encodes a secreted form in hepatocytes. *PloS one,* 2010, vol. 5, e12934
- **WEI et al.** *Int J Nanomedicine,* 2015, vol. 10, 5123-37
- **D'SOUZA et al.** *J Control Release,* 2015, vol. 203, 126-39
- **RAJEEV et al.** *Chembiochem,* 2015, vol. 16 (6), 903-8
- **WEIGEL PH ; YIK JH.** Glycans as endocytosis signals: the cases of the asialoglycoprotein and hyaluronan/chondroitin sulfate receptors. *Biochimica et biophysica acta,* 2002, vol. 1572, 341-63
- **TOZAWA R ; ISHIBASHI S ; OSUGA J et al.** Asialoglycoprotein receptor deficiency in mice lacking the major receptor subunit. *ts obligate requirement for the stable expression of oligomeric receptor. The Journal of Biological Chemistry,* 2001, vol. 276, 12624-8

- **TUIN A ; HUIZINGA-VAN DER VLAG A ; VAN LOENEN- WEEMAES AM ; MEIJER DK ; POELSTRA K.** On the role and fate of LPS-dephosphorylating activity in the rat liver. *American Journal of Physiology Gastrointestinal and Liver Physiology,* 2006, vol. 290, G377-85
- **FURGER E ; FEDOSOV SN ; LILDBALLE DL et al.** Comparison of recombinant human haptocorrin expressed in human embryonic kidney cells and native haptocorrin. *PloS one,* 2012, vol. 7, e37421
- **BURGER RL ; SCHNEIDER RJ ; MEHLMAN CS ; ALLEN RH.** Human plasma R-type vitamin B12-binding proteins. II. The role of transcobalamin I, transcobalamin III, and the normal granulocyte vitamin B12-binding protein in the plasma transport of vitamin B12. *The Journal of Biological Chemistry,* 1975, vol. 250, 7707-13
- **STEIRER LM ; PARK EI ; TOWNSEND RR ; BAENZIGER JU.** The asialoglycoprotein receptor regulates levels of plasma glycoproteins terminating with sialic acid alpha2,6-galactose. *The Journal of Biological Chemistry,* 2009, vol. 284, 3777-83
- **YANG A ; GYULAY G ; MITCHELL M ; WHITE E ; TRIGATTI BL ; IGDOURA SA.** Hypomorphic sialidase expression decreases serum cholesterol by downregulation of VLDL production in mice. *Journal of Lipid Research,* 2012, vol. 53, 2573-2585
- COMPUTATIONAL MOLECULAR BIOLOGY. Oxford University Press, 1988
- BIOCOMPUTING: INFORMATICS AND GENOME PROJECTS. Academic Press, 1993
- COMPUTER ANALYSIS OF SEQUENCE DATA, PART I. Humana Press, 1994
- SEQUENCE ANALYSIS IN MOLECULAR BIOLOGY. Academic Press, 1987
- SEQUENCE ANALYSIS PRIMER. M Stockton Press, 1991
- **H. CARILLO ; D. LIPTON.** *SIAM J. Applied Math,* 1988, vol. 48, 1073
- Guide to Huge Computers. Academic Press, 1994
- **H. CARILLO ; D. LIPTON.** *SIAM J. Applied Math.,* 1988, vol. 48, 1073
- **J. DEVEREUX et al.** *Nucl. Acids Res.,* 1984, vol. 12 (1), 387
- **DELEAVEY ; DAMHA.** *Chemistry and Biology,* 2012, vol. 19, 937-954
- **HAMAJIMA et al.** *Clin. Immunol. Immunopathol.,* 1998, vol. 88 (2), 205-10
- **SUBBARAO et al.** *Biochemistry,* 1987, vol. 26, 2964-2972
- **VOGEL et al.** *J. Am. Chem. Soc.,* 1996, vol. 118, 1581-1586
- **TURK et al.** *Biochem. Biophys. Acta,* 2002, vol. 1559, 56-68
- **LAM et al.** *Nature,* 1991, vol. 354, 82-84
- **AOKI et al.** *Cancer Gene Therapy,* 2001, vol. 8, 783-787
- **HAUBNER et al.** *Jour. Nucl. Med.,* 2001, vol. 42, 326-336
- **SIMEONI et al.** *Nucl. Acids Res.,* 2003, vol. 31, 2717-2724
- **TRUFERT et al.** *Tetrahedron,* 1996, vol. 52, 3005
- Oligonucleotide Conjugates in Antisense Technology. **MANOHARAN.** Antisense Drug Technology. Marcel Dekker, Inc, 2001
- **R. LAROCK.** Comprehensive Organic Transformations. VCH Publishers, 1989
- **T. W. GREENE ; P. G. M. WUTS.** Protective Groups in Organic Synthesis. John Wiley and Sons, 1991
- **L. FIESER ; M. FIESER.** Fieser and Fieser's Reagents for Organic Synthesis. John Wiley and Sons, 1994
- Encyclopedia of Reagents for Organic Synthesis. John Wiley and Sons, 1995
- **TOZAWA et al.** *J Biol Chem,* 2001, vol. 276, 12624-12628
- **STOCKERT et al.** *Science,* 1977, vol. 197, 667-668
- **HARDONK MJ ; SCHOLTENS HB.** *Histochemistry,* 1980, vol. 69 (3), 289-97
- **SCHOLTENS HB ; MEIJER DK ; HARDONK MJ.** *Liver,* March 1982, vol. 2 (1), 14-21
- **WINDLER et al.** *Biochem J,* 1991, vol. 276, 79-87
- **ROTUNDO et al.** *Hepatology,* 1998, vol. 28, 475-485
- **STOCKERT et al.** *PNAS,* 1982, vol. 79, 6229-6231
- **FRIEDEWALD, W. T. ; LEVY, R. I. ; FREDRICKSON, D. S.** Estimation of the concentration of low-density lipoprotein cholesterol in plasma, without use of the preparative ultracentrifuge. *Clin. Chem.,* 1972, vol. 18, 499-502
- **GUDBJARTSSON et al.** Large-scale whole-genome sequencing of the Icelandic population. *Nature genetics,* 2015
- **SAMANI NJ et al.** Genomewide association analysis of coronary artery disease. *The New England journal of medicine,* 2007, vol. 357, 443-53
- **GRETARSDOTTIR S et al.** Genome-wide association study identifies a sequence variant within the DAB2IP gene conferring susceptibility to abdominal aortic aneurysm. *Nature genetics,* 2010, vol. 42, 692
- **OLSEN MH et al.** N-terminal pro-brain natriuretic peptide, but not high sensitivity C-reactive protein, improves cardiovascular risk prediction in the general population. *European heart journal,* 2007, vol. 28, 1374-81
- **JORGENSEN AB et al.** Loss-of-function mutations in APOC3 and risk of ischemic vascular disease. *The New England journal of medicine,* 2014, vol. 371, 32-41

- **HOOGENDOORN EH et al.** Thyroid function and prevalence of anti-thyroperoxidase antibodies in a population with borderline sufficient iodine intake: influences of age and sex. *Clinical chemistry,* 2006, vol. 52, 104-11
- **GUDBARTSSOON, DF et al.** Large Scale whole-genome sequencing of the Icelandic population. *Nature Genetics,* 2015
- **DI ANGELANTONIO E et al.** Major lipids, apolipoproteins, and risk of vascular disease. *Jama,* 2009, vol. 302, 1993-2000
- **GUDBARTSSOON, DF et al.** Large Scale whole-genome sequencing of the Icelandic population. *Nature Genetics,* 2015
- **GUDBJARTSSON DF et al.** Large-scale whole-genome sequencing of the Icelandic population. *Nature genetics,* 2015
- **STEINTHORSDOTTIR V et al.** Identification of low-frequency and rare sequence variants associated with elevated or reduced risk of type 2 diabetes. *Nature genetics,* 2014, vol. 46, 294-8
- **WILLER CJ et al.** METAL: fast and efficient meta-analysis of genomewide association scans. *Bioinformatics,* 2010, vol. 26, 2190-1
- **GRETARSDOTTIR S et al.** The gene encoding phosphodiesterase 4D confers risk of ischemic stroke. *Nature genetics,* 2003, vol. 35, 131-8
- **MORELL AG ; GREGORIADIS G ; SCHEINBERG IH ; HICKMAN J ; ASHWELL G.** The role of sialic acid in determining the survival of glycoproteins in the circulation. *The Journal of biological chemistry,* 1971, vol. 246, 1461-7
- **VAN DEN HAMER CJ ; MORELL AG ; SCHEINBERG IH ; HICKMAN J ; ASHWELL G.** Physical and chemical studies on ceruloplasmin. IX. The role of galactosyl residues in the clearance of ceruloplasmin from the circulation. *The Journal of biological chemistry,* 1970, vol. 245, 4397-402
- **ASHWELL G ; HARFORD J.** Carbohydrate-specific receptors of the liver. *Annual review of biochemistry,* 1982, vol. 51, 531-54
- **WEIGEL PH.** Galactosyl and N-acetylgalactosaminyl homeostasis: a function for mammalian asialoglycoprotein receptors. *BioEssays: news and reviews in molecular, cellular and developmental biology,* 1994, vol. 16, 519-24
- **HADDAD L ; DAY IN ; HUNT S ; WILLIAMS RR ; HUMPHRIES SE ; HOPKINS PN.** Evidence for a third genetic locus causing familial hypercholesterolemia. A non- LDLR, non-APOB kindred. *Journal of lipid research,* 1999, vol. 40, 1113-22
- **TIMMS KM ; WAGNER S ; SAMUELS ME et al.** A mutation in PCSK9 causing autosomal-dominant hypercholesterolemia in a Utah pedigree. *Human genetics,* 2004, vol. 114, 349-53
- **VARRET M ; RABES JP ; SAINT-JORE B et al.** A third major locus for autosomal dominant hypercholesterolemia maps to 1p34.1-p32. *American journal of human genetics,* 1999, vol. 64, 1378-87
- **HUNT SC ; HOPKINS PN ; BULKA K et al.** Genetic localization to chromosome 1p32 of the third locus for familial hypercholesterolemia in a Utah kindred. *Arterioscler Thromb Vasc Biol,* 2000, vol. 20, 1089-93
- **DO R ; WILLER CJ ; SCHMIDT EM et al.** Common variants associated with plasma triglycerides and risk for coronary artery disease. *Nature genetics,* 2013, vol. 45, 1345-52
- **CHAMBERS JC ; ZHANG W ; SEHMI J et al.** Genome-wide association study identifies loci influencing concentrations of liver enzymes in plasma. *Nature genetics,* 2011, vol. 43, 1131-8
- **WILLER CJ ; SCHMIDT EM ; SENGUPTA S et al.** Discovery and refinement of loci associated with lipid levels. *Nature genetics,* 2013, vol. 45, 1274-83
- **HELGADOTTIR A ; THORLEIFSSON G ; MANOLESCU A et al.** A common variant on chromosom e 9p21 affects the risk of myocardial infarction. *Science,* 2007, vol. 316, 1 491-3
- **HELGADOTTIR A ; MANOLESCU A ; THORLEIFSSON G et al.** *The gene encoding 5- lipoxygenas e activating protein confers risk of myocardial infarction and stroke*
- *Nature genetics,* 2004, vol. 36, 23 3-9
- Genome- wide association study of 14,000 cases of seven common diseases and 3,000 shared controls. *Nature,* 2007, vol. 447, 6 61-78
- **SAMANI NJ ; ERDMANN J ; HALL AS et al.** Genomewid e association analysis of coronary artery disease. *The New England journal of medicine,* 2007, vol. 357, 4 43-53
- **GRETARSDOTTI R S ; BAAS AF ; THORLEIFSSON G et al.** Genome- wide association study identifies a sequence variant within the DAB2IP gene conferring susceptibilit y to abdominal aortic aneurysm. *Nature genetics,* 2010, vol. 42, 69 2-7
- The Inter99 study is a population-based randomized controlled trial (CT00289237, ClinicalTrials.gov) investigating the effects of lifestyle intervention on cardiovascular disease. *Cardiovascular events were defined as first ever non-fatal or fatal CVD (ICD- 8: 390-448/ICD-10: 100- I79)*
- **JORG ENSEN AB ; FRIKKE- SCHMIDT R ; NORDESTGAA RD BG ; TYBJAERG- HANSEN A.** Loss-of- function mutations in APOC3 and risk of ischemic vascular disease. *The New England journal of medicine,* 2014, vol. 371, 3 2-41
- **GREEN et al.** *Nature Genetics,* 1994, vol. 7, 13-21
- **MENDEZ et al.** *Nature Genetics,* 1997, vol. 15, 146-156
- **GREEN ; JAKOBOVITIS.** *J. Ex. Med,* 1998, vol. 188, 483-495

- **KELLERMAN ; GREEN.** *Current Opinion in Biotechnology,* 2002, vol. 13, 593-597
- **JANSSENS et al.** *PNAS,* 2006, vol. 103, 15130-15135
- **MEIER, M. ; BIDER, M. D. ; MALASHKEVICH, V. N ; SPIESS, M ; BURKHARD, P.** Crystal structure of the carbohydrate recognition domain of the H1 subunit of the asialoglycoprotein receptor. *Journal of molecular biology,* 2000, vol. 300, 857-865
- **BATTYE, T. G ; KONTOGIANNIS, L. ; JOHNSON, O ; POWELL, H. R. ; LESLIE, A. G.** iMOSFLM: a new graphical interface for diffraction-image processing with MOSFLM. *Acta crystallographica,* 2011, vol. 67, 271-281
- **EVANS, P.** Scaling and assessment of data quality. *Acta crystallographica,* 2006, vol. 62, 72-82
- CCP4. (1994) The CCP4 suite: programs for protein crystallography. *Acta crystallographica,* vol. 50, 760-763
- **MCCOY, A. J. ; GROSSE-KUNSTLEVE, R. W. ; ADAMS, P. D ; WINN, M. D. ; STORONI, L. C. ; READ, R. J.** Phaser crystallographic software. *Journal of applied crystallography,* 2007, vol. 40, 658-674
- **EMSLEY, P ; LOHKAMP, B ; SCOTT, W. G. ; COWTAN, K.** Features and development of Coot. *Acta crystallographica,* 2010, vol. 66, 486-501
- **ADAMS, P. D. ; AFONINE, P. V ; BUNKOCZI, G ; CHEN, V. B. ; DAVIS, I. W ; ECHOLS, N. ; HEADD, J. J. ; HUNG, L. W ; KAPRAL, G. J ; GROSSE-KUNSTLEVE, R. W.** PHENIX: a comprehensive Python-based system for macromolecular structure solution. *Acta crystallographica,* 2010, vol. 66, 213-221
- **DELANO, W. L.** The PyMOL Molecular Graphics System. *Palo Alto,* 2002
- **BATTYE, T.G. ; KONTOGIANNIS, L. ; JOHNSON, O ; POWELL, H.R. ; LESLIE, A.G.** iMOSFLM: a new graphical interface for diffraction-image processing with MOSFLM. *Acta Crystallogr D Biol Crystallogr,* 2011, vol. 67, 271-81
- CCP4. The CCP4 suite: programs for protein crystallography. *Acta Crystallogr D Biol Crystallogr,* 1994, vol. 50, 760-3
- **EMSLEY, P. ; LOHKAMP, B ; SCOTT, W.G ; COWTAN, K.** Features and development of Coot. *Acta Crystallogr D Biol Crystallogr,* 2010, vol. 66, 486-501
- **ADAMS, P.D. et al.** PHENIX: a comprehensive Python-based system for macromolecular structure solution. *Acta Crystallogr D Biol Crystallogr,* 2010, vol. 66, 213-21
- **DELANO, W.L.** The PyMOL Molecular Graphics System. *Palo Alto,* 2002
- Molecular Operating Environment (MOE), 2013.08. *Chemical Computing Group, Inc., 1010 Sherbooke St. West, Suit #910, Montreal, QC, Canada,* 2016
- **NIOI, P et al.** Variant ASGR1 Associated with a Reduced Risk of Coronary Artery Disease. *The New England journal of medicine,* 2016, vol. 374, 2131-2141
- **GOLD, L. et al.** Aptamer-based multiplexed proteomic technology for biomarker discovery. *PLoS One,* 2010, vol. 5, e15004
- **SHAM, P. C. ; PURCELL, S. M.** Statistical power and significance testing in large-scale genetic studies. *Nature reviews. Genetics,* 2014, vol. 15, 335-346
- **GOLD, L. et al.** Aptamer-based multiplexed proteomic technology for biomarker discovery. *PLoS One,* 2010, vol. 5, 5004